(19) Europäisches Patentamt
European Patent Office
Office européen des brevets

(11) **EP 4 276 092 A1**

(12) **EUROPEAN PATENT APPLICATION**
published in accordance with Art. 153(4) EPC

(43) Date of publication:
**15.11.2023 Bulletin 2023/46**

(21) Application number: **22736785.1**

(22) Date of filing: **06.01.2022**

(51) International Patent Classification (IPC):
***C07C 311/21*** *(2006.01)*

(52) Cooperative Patent Classification (CPC):
**C07C 311/21; C07D 213/56; C07D 213/65; C07D 231/12; C07D 237/24; C07D 295/155; C07D 295/185; C07D 295/192; C07D 295/26; C07D 333/20; C07D 333/24; C07D 333/34; C07D 333/38; C07D 333/40; C07D 401/04;** (Cont.)

(86) International application number:
**PCT/JP2022/001007**

(87) International publication number:
**WO 2022/149617 (14.07.2022 Gazette 2022/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.01.2021 JP 2021001132**

(71) Applicant: **CHUGAI SEIYAKU KABUSHIKI KAISHA**
**Tokyo 115-8543 (JP)**

(72) Inventors:
- **TSUCHIYA, Satoshi**
  **Gotemba-shi, Shizuoka 412-8513 (JP)**
- **EMURA, Takashi**
  **Gotemba-shi, Shizuoka 412-8513 (JP)**
- **HAYASE, Tadakatsu**
  **Gotemba-shi, Shizuoka 412-8513 (JP)**
- **NOMURA, Kenichi**
  **Gotemba-shi, Shizuoka 412-8513 (JP)**
- **OGAWA, Hiroko**
  **Kamakura-shi, Kanagawa 247-8530 (JP)**
- **KINOSHITA, Kazutomo**
  **Tokyo 103-8324 (JP)**
- **ITO, Taisuke**
  **Gotemba-shi, Shizuoka 412-8513 (JP)**
- **KAMAKURA, Daiki**
  **Gotemba-shi, Shizuoka 412-8513 (JP)**
- **IIKURA, Hitoshi**
  **Gotemba-shi, Shizuoka 412-8513 (JP)**
- **NISHIMOTO, Masahiro**
  **Gotemba-shi, Shizuoka 412-8513 (JP)**
- **ORI, Kazutomo**
  **Kamakura-shi, Kanagawa 247-8530 (JP)**
- **MURATA, Yoshihisa**
  **Gotemba-shi, Shizuoka 412-8513 (JP)**
- **SAITO, Masato**
  **Gotemba-shi, Shizuoka 412-8513 (JP)**

(74) Representative: **Vossius & Partner**
**Patentanwälte Rechtsanwälte mbB**
**Siebertstraße 3**
**81675 München (DE)**

(54) **COMPOUND LIBRARY**

(57) The present invention provides a library comprising $1 \times 10^2$ to $1 \times 10^8$ compounds, wherein the compounds have the following structure where a first core block (first CB), a first linker (first L), and a second core block (second CB) are covalently linked: (first CB)- (first L)-(second CB), wherein the library comprises two or more types of first CB, two or more types of first L, and two or more types of second CB, and the library is constituted by one or two or more mixtures comprising $1 \times 10^2$ to $1 \times 10^5$ of the compounds.

Figure 2

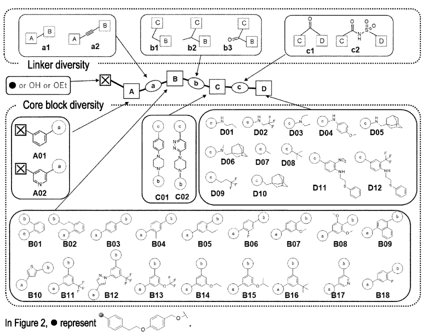


EP 4 276 092 A1

(52) Cooperative Patent Classification (CPC): (Cont.)
**C07D 401/12; C07D 401/14; C07D 403/12;
C07D 409/04; C07D 409/12; C07D 409/14;
C40B 30/04; C40B 40/04; C40B 50/14;
G01N 33/15; G01N 33/50**

## Description

### TECHNICAL FIELD

The present disclosure relates to a compound library having structural diversity and a method for producing the same, and a screening method, etc. significantly

### BACKGROUND ART

In medicament development, search for novel compounds having intended pharmacological activity holds an important position.

Approaches of expanding and screening a compound library having structural diversity are key techniques for obtaining hit compounds (hit generation) (Non Patent Literatures 1 and 2). The idea of scaffold hopping (identification of compounds having both "isofunctional molecular structures" and "different molecular backbones" relative to parent compounds) was proposed in 1999 as an approach of searching known compounds for novel compounds (Non Patent Literature 30), and has been widely accepted and used in the field of medicinal chemistry (Non Patent Literature 31).

Taking a look at the characteristics of compounds, many cases of previous drug discovery have emphasized the idea of rule of 5 proposed in 1998, and carried out in a molecular weight range of less than 500. On the other hand, recent cases have been reported where oral agents or action on intracellular targets is attained even when falling outside the scope of rule of 5 (which mostly exceeds a molecular weight of 500), and drug discovery beyond rule of 5 has received attention (Non Patent Literature 27). As for tough targets (generic name for medicinal targets characterized by lacking a deep hole to which small molecules are capable of binding; examples thereof include the inhibition of protein-protein interaction, the inhibition of RNA-protein interaction, and the inhibition of nucleic acid- nucleic acid interaction) in conventional low-molecular drug discovery, the possibility has been pointed out that compounds within the scope of rule of 5 do not obtain sufficient affinity for proteins (Non Patent Literature 28). Also, the relationship between molecular complexity and the probability of success in clinical trials (Non Patent Literature 29) has received attention, and methods for supplying a compound library having a lot of complexity are also regarded as being important.

Thus, it is strongly desired to provide a compound group having novel characteristics, and novel hit generation or scaffold hopping approaches of conducting evaluation using a large number of compounds are indispensable. Methods for actually conducting evaluation using compounds include (a) a method using an existing compound library, and (b) a method of newly constructing and evaluating a compound library.

Examples of the method (a) include HTS (high-throughput screening) libraries, which have played a central role as an approach for obtaining hit compounds for a long time (Non Patent Literature 3). The expansion of a HTS library is strongly influenced by rule of 5 proposed by Lipinski, and has mostly been carried out within a given range (e.g., not exceeding a molecular weight of 500) (Non Patent Literature 4). Also, an approach of obtaining hit compounds by FBDD (fragment-based drug discovery) is used (Non Patent Literature 5). It has been reported that hit compounds leading to drug discovery were obtained for a plurality of target molecules (Non Patent Literature 6). However, due to the small molecular weights of the fragment compounds used, the resulting hit compounds have weak affinity and require an approach of growing or linking for improvement in activity (Non Patent Literature 7). Structural information on target molecules is essential for largely elevating activity, and this method is unsuitable for target molecules that lack structural information (Non Patent Literature 2). An approach of exploiting a calculation procedure in search for the optimum linkers that link fragments are used for target molecules having structural information (Non Patent Literature 8).

A peptide library having amino acids as building blocks is known as an approach of newly expanding a compound library in the method (b) (Non Patent Literature 9). The linking pattern between the building blocks is limited to an amide bond from amidation reaction, and the building blocks used are also limited to amino acids.

Chemical reaction other than amidation reaction was also used in combinatorial chemistry in 1990s and received a lot of attention as a promising method for searching for novel drugs. On the other hand, taking a look at the structural diversity of library compounds, each individual library was a library in which building blocks were linked with a focus on a common structure (scaffold) (structurally homogeneous library) (Non Patent Literature 10). By contrast, a structurally heterogeneous library has been reported as a library that can enhance the structural diversity of compounds because the compounds constituting the library have no specific common structure (scaffold) and are constructed by sequentially linking building blocks (Non Patent Literatures 11 and 12). However, it has been reported as points in common of such conventional compound library construction that, since the number of compounds and a synthesis period are focused on, chemical reaction feasible by a simple operation is used for library construction (Non Patent Literature 13) and building blocks are used without the use of a protective group (Non Patent Literature 14).

Seeing methods for constructing compound libraries from another perspective, the methods can be broadly divided into an approach by parallel synthesis and an approach by synthesis using a mixture (mixture-based synthesis). Although cases of a library of several thousands of compounds synthesized by parallel synthesis have also been reported

(Patent Literatures 1 and 2), the mixture-based synthesis is considered as a methodology capable of synthesizing more compounds for a short period (Non Patent Literature 10). Meanwhile, it is necessary to select an approach of identifying compounds having binding activity against target molecules (decoding) in screening when a library is supplied with a mixture.

DNA-encoded library technology (DELT) using building blocks that are not limited to amino acids is known as an example of library synthesis with a mixture (Non Patent Literature 16). A feature of DELT is use of a DNA tag for decoding compounds having binding activity against target molecules. Decoding using the DNA tag is to correctly show structural information on each individual library compounds through one-to-one relation on the basis of sequence information thereon. Hence, the DNA tag needs to be bound within the same molecule as a library compound (e.g., Non Patent Literatures 20 and 21). The DNA tag, albeit responsible for decoding, causes constraints on reaction conditions for chemical reaction that can be used in library synthesis, because the DNA tag exists within the same molecule as a library compound. As a result, the scope of synthesizable compounds is restricted (Non Patent Literature 17). Furthermore, it has also been reported that damage on the DNA tag occurs depending on the chemical reaction used (Non Patent Literature 18). This damage on the DNA tag disables correct decoding of compounds that bind to target molecules. In actuality, it has been reported that in libraries of DELT, false negative is increased at a scale of the 8th or more power of 10 (Non Patent Literature 19).

An approach using AS-MS (affinity selection mass spectrometry) in screening for molecules having binding activity against target molecules is also known as another approach of decoding (Non Patent Literature 22). This approach is recognized as a non-biased assay approach for screening for compounds through the use of affinity for target molecules without the need of labeling and immobilization, and also enables allosteric inhibitors to be identified. Cases where existing compounds present as single compounds are mixed and screened (Non Patent Literatures 23 and 24), and cases of applying AS-MS to derivative evaluation, not to hit generation (Non Patent Literatures 25 and 26) have also been reported as cases using AS-MS. Thus, usefulness thereof as an approach of screening compounds is recognized. Although there is also a report on AS-MS screening performed on a library synthesized by reagent mixture synthesis (one of the mixture-based approaches; an approach in which a plurality of building blocks to be linked are mixed for use) (Non Patent Literature 25), its use remains in structurally homogeneous library centered on a scaffold structure.

There is a demand for a novel compound library having structural diversity which can be used in the development of medicaments. Nonetheless, the establishment of such a compound library is susceptible to improvement in various points.

CITATION LIST

PATENT LITERATURE

[Patent Literature 1] JP2019-527672(A)
[Patent Literature 2] JP2020-524701(A)

NON PATENT LITERATURE

[Non Patent Literature 1] Ruiwu Liu et al., Combinatorial chemistry in drug discovery. Current Opinion in Chemical Biology 2017, 38: 117-126

[Non Patent Literature 2] Joerg Holenz, Patrick Stoy., Advances in Lead Generation. Bioorganic & Medicinal Chemistry Letters 29 (2019) 517-524

[Non Patent Literature 3] R. Macarron et al., Impact of high-throughput screening in biomedical research. Nature Reviews Drug Discovery volume 10, pages 188-195 (2011)

[Non Patent Literature 4] M. J. Wigglesworth et al., Increasing the delivery of next generation therapeutics from high throughput screening libraries. Current Opinion in Chemical Biology 2015, 26: 104-110

[Non Patent Literature 5] P. J. Hajduk et al., A decade of fragment-based drug design: strategic advances and lessons learned. Nature Reviews Drug Discovery volume 6, pages 211-219 (2007)

[Non Patent Literature 6] G. Chessari et al., From fragment to clinical candidate- a historical perspective. Drug Discovery Today, Volume 14, Issues 13-14, July 2009, Pages 668-675

[Non Patent Literature 7] A. Bancet. et al., Fragment Linking Strategies for Structure-Based Drug Design., J. Med. Chem. 2020, 63, 20, 11420-11435

[Non Patent Literature 8] F. Imrie et al., Deep Generative Models for 3D Linker Design., J. Chem. Inf. Model. 2020,

60, 4, 1983-1995.
[Non Patent Literature 9] R. Liu et al., Combinatorial peptide library methods for immunobiology research., Experimental Hematology 31 (2003) 11-30.
[Non Patent Literature 10] The "One-Bead-One-Compound" Combinatorial Library Method. Chem. Rev. 1997, 97, 2, 411-448.
[Non Patent Literature 11] Structurally homogeneous and heterogeneous synthetic combinatorial libraries., Molecular Diversity volume 1, pages 149-164 (1996)
[Non Patent Literature 12] R. Severinsen et al., Library of Biphenyl Privileged Substructures using a Safety-Catch Linker Approach., J. Chem. Inf. Model. 2020, 60, 4, 1983-1995
[Non Patent Literature 13] Souyaku Kagaku (Drug Discovery Chemistry in English), Tokyo Kagaku Dojin, ISBN978-4-8079-0584-3, p. 186
[Non Patent Literature 14] The Practice of Medicinal Chemistry, p. 483
[Non Patent Literature 15] Richard A. Houghten et al., Mixture-Based Synthetic Combinatorial Libraries., J. Med. Chem. 1999, 42, 19, 3743-3778
[Non Patent Literature 16] Robert A. Goodnow et al., DNA-encoded chemistry: enabling the deeper sampling of chemical space. Nature Reviews Drug Discovery volume 16, pages 131-147 (2017)
[Non Patent Literature 17] Kin-Chun Luk, Alexander Lee Satz, DNA-Compatible Chemistry. A Handbook for DNA-Encoded Chemistry 67-98
[Non Patent Literature 18] Marie L. Malone, Brian M. Paegel, What is a "DNA- Compatible" Reaction? ACS Combinatorial Science 2016, 18, 4, 182-187
[Non Patent Literature 19] A. Satz et al., Analysis of Current DNA Encoded Library Screening Data Indicates Higher False Negative Rates for Numerically Larger Libraries. ACS Comb. Sci. 2017, 19, 4, 234-238
[Non Patent Literature 20] C. S. Kollmann et al., Application of encoded library technology (ELT) to a protein-protein interaction target: Discovery of a potent class of integrin lymphocyte function-associated antigen 1 (LFA-1) antagonists. Bioorg. Med. Chem. 22 (2014) 235
[Non Patent Literature 21] Y. C. Chen et al., C-N Coupling of DNA-Conjugated (Hetero)aryl Bromides and Chlorides for DNA-Encoded Chemical Library Synthesis. Bioconjugate Chem. 2020, 31, 3, 770-780
[Non Patent Literature 22] D. A. Annis, et al., Affinity selection-mass spectrometry screening techniques for small molecule drug discovery. Current Opinion in Chemical Biology 2007,11: 518-526
[Non Patent Literature 23] J. Qian et al., Discovery of novel inhibitors of Bcl-xL using multiplehigh-throughput screening platforms. Analytical Biochemistry 328 (2004) 131-138
[Non Patent Literature 24] K. M. Comess, et al., Kinase Drug Discovery by Affinity Selection/Mass Spectrometry (ASMS): Application to DNA Damage Checkpoint Kinase Chk1. Journal of Biomolecular Screening 11(7); 2006, 755-764
[Non Patent Literature 25] D. A. Annis, et al., An affinity selection-mass spectrometry method for the identification of small molecule ligands from self-encoded combinatorial libraries: Discovery of a novel antagonist of E. coli dihydrofolate reductase. International Journal of Mass Spectrometry 238 (2004) 77-83
[Non Patent Literature 26] B. R. Lahue et al., Substituted benzimidazoles: A novel chemotype for small molecule hKSP inhibitors. Bioorganic & Medicinal Chemistry Letters 19 (2009) 3405
[Non Patent Literature 27] B. C. Doak, J. Kihlberg, Drug discovery beyond the rule of 5 - Opportunities and challenges. Expert Opinion on Drug Discovery, (2017), 12: 2, 115- 119
[Non Patent Literature 28] H. Lu et al., Recent advances in the development of protein-protein interactions modulators: mechanisms and clinical trials, Sig Transduct Target Ther 5, 213 (2020). https://doi.org/10.1038/s41392-020-00315-3
[Non Patent Literature 29] F. Lovering et al., Escape from Flatland: Increasing Saturation as an Approach to Improving Clinical Success. J. Med. Chem. 2009, 52, 21, 6752-6756
[Non Patent Literature 30] G. Schneider et al., "Scaffold-Hopping" by Topological Pharmacophore Search: A Contribution to Virtual Screening. Angew. Chem. Int. Ed., 38 (1999), pp. 2894-2896
[Non Patent Literature 31] H. Sun et al., Classification of scaffold-hopping approaches. Drug Discovery Today Volume 17, Issues 7-8, April 2012, Pages 310-32

## SUMMARY OF INVENTION

### TECHNICAL PROBLEM

An approach of synthesizing compounds constituting a library as a mixture is advantageous for obtaining true hit compounds against target molecules, from the viewpoint of the efficiency of compound synthesis. On the other hand, in the case of synthesizing compounds constituting a library as a mixture, the diversity of compounds and the ensuring

of quality are important.

The viewpoint of the diversity of library compounds will be described. A large number of conventional compound libraries have been constituted by compound groups having a defined scaffold (structurally homogeneous library). A building block group having a common reactive functional group is attached to a given scaffold so that pharmacophore diversity derived from the structural diversity of building blocks emerges. Furthermore, the structural diversity is caused by the combination of pharmacophores at a plurality of locations. In short, only the structural diversity of pharmacophores derived from the structures of building blocks has received attention. In other words, no attention has been given to the diversity of linking moieties (linkers) that link building blocks.

A compound library having diversity not only in building blocks but in binding patterns that link these building blocks can exert diversity in pharmacophore conformations (i.e., the distances or angles between functional groups that form pharmacophores) and can also be expected to acquire affinity for target molecules through linkers. Hence, a library that allows core blocks containing a functional group for pharmacophore formation or the like to be freely selected and also allows linkers therebetween to be freely selected is capable of markedly expanding structural diversity and is of very high value. Although involving such value, the library prepared by taking into consideration the diversity of linkers using building blocks having two or more reactive functional groups (multifunctional building blocks) is not sufficiently exploited. For example, while a structurally heterogeneous library is constructed by solid-phase synthesis, the multifunctional building blocks have only limited use and are merely applied to the capping of one type of specific reactive functional group (introduction of building blocks to molecular ends), though a plurality of binding patterns are used for one binding point (Non Patent Literature 12). There is a report on DELT using a plurality of linkers in the introduction of building blocks to the inside of a molecule (Non Patent Literature 21). However, problems associated with quality mentioned later still remain.

In the case of obtaining hit compounds by FBDD, the compounds generally have weak affinity for target molecules and therefore require an approach of growing or linking for improvement in activity. However, during the course of the approach of linking expected to produce more highly active compounds, problems remain in search for highly active compounds by sufficiently evaluating the diversity of linkers and the diversity of core blocks, which form pharmacophores. Specifically, most of the linking approaches optimize linkers and bonds while the type of core blocks that form pharmacophores is fixed (Non Patent Literatures 7 and 8). Therefore, a wide range of search cannot be made.

In the case of searching for novel compounds on the basis of known compounds by use of an approach of scaffold hopping, the optimum conformation of each pharmacophore is likely to differ among backbones (scaffolds). Hence, it may be desirable that diverse core blocks and diverse linkers can be searched at the same time. However, general approaches that achieve this still present problems. Even use of an advanced calculation approach remains in search for linkers and bonds while the type of core blocks is fixed (Non Patent Literature 8).

Specifically, a methodology of a wide range of search in which the diversity of core blocks and the diversity of linkers are multiplied is required for the drug discovery of hit generation, scaffold hopping and FBDD.

Meanwhile, a purification operation other than solid-phase extraction and liquid- liquid extraction, distilling-off under reduced pressure or removal using solid-phase reagents, aimed at removing reagents, is not performed in the synthesis of library compounds as a mixture. Therefore, if unintended reaction progresses in linking building blocks, the abundance ratio of unintended compounds is increased so that assay is carried out with the compounds left. This problem becomes more serious through reaction in the state where many compounds and functional groups coexist, leading to marked reduction in the yields of the compounds of interest.

Conventional libraries involve a large number of compounds in one reaction space, in pursuit of library scales. For example, a peptide library formed from only amide bonds is synthesized by allowing 100000 or more compounds to be contained in one reaction space (Non Patent Literature 15). However, a large number of sequences cannot be synthesized even though only amide bonds are used. Therefore, false negative occurs and makes it difficult to obtain information on the affinity of designed compounds for targets. Furthermore, contamination by impurities ascribable to the progression of unintended reaction or impurities derived from remaining unreacted synthetic intermediates cannot be avoided. As a result, false negative ascribable to unintended formed compounds cannot be eliminated.

As for DELT having a DNA tag, damage on the DNA tag may be caused, in addition to the formation of impurities in the compounds of interest, because the DNA tag itself is also capable of reacting. In short, not only are the yields of the compounds of interest reduced, but DNA itself which encodes structural information on compounds is damaged, causing false negative due to which hit compounds cannot be identified.

In synthesis approaches using a mixture, building blocks to be added to a reaction system are mixed and added to a substrate. Therefore, the yields of compounds formed vary due to difference in reactivity among the building blocks. This probably facilitates difference in concentration among compounds in assaying the mixture. Although an approach of adjusting the molar numbers of building blocks to be added is possible, it is not realistic to compare beforehand reactivity among the building blocks used. Therefore, reaction control is difficult, and unfortunately, the quality of the resulting mixture is not ensured.

There are also problems associated with the exploitation of protective groups. In conventional libraries, use of

a protective group is avoided with a focus on the simplification of synthesis. As a result, the diversity of functional groups contained in the compounds of interest is restricted.

The conventional libraries for which library compounds are synthesized as a mixture as described above cannot ensure the quality of compounds contained in the mixture, due to (i) a large number of compounds per flask, (ii) limitations on available reaction due to the presence of a DNA tag, and damage on the DNA tag, (iii) difficult reaction control ascribable to the mixture, and (iv) insufficient exploitation of protective groups. The conventional techniques have not yet solved the technical problems described above, and fallen short of being able to prepare a compound library having diversity in linkers that link core blocks among compound libraries prepared using a mixture.

Specifically, none of the previous methodologies of library construction ensure quality while pursuing structural diversity.

The present invention has been made in light of these circumstances. An object of the present invention is to provide a compound library having diverse binding patterns, and a method for producing the same.

SOLUTION TO PROBLEM

In order to rapidly synthesize large-scale (e.g., 1000000 or more) compounds as a mixture with ensured quality, the present inventors have conceived a mixture & parallel library for which compounds are synthesized by limiting the number of compounds contained in one reaction space, and increasing the number of flasks (e.g., 1000 or more types of libraries including 1000 compounds). Specifically, the present inventors have conceived the idea that, while the quality is ensured in a reaction space which is one small group, the diversity of compounds can be acquired by arranging in parallel and grouping such reaction spaces. Based on this idea, for example, the quality can be ensured even when a compound group of 1000000 or more compounds is supplied.

One embodiment of a specific library construction method based on the idea has been found as follows.

Molecules are constructed using building blocks having two or more reactive functional groups (multifunctional building blocks) therein. In this respect, core blocks of the building blocks having reactive functional groups are not limited to a specific scaffold, unlike many conventional libraries. First building blocks (first BB) can be linked via one of the reactive functional groups to solid-phase reagents to prepare solid-phase supported reagents having the other reactive functional group. A group of reagents having the common reactive functional group on the first building blocks in this solid-phase reagent group is homogeneously mixed for use in reaction in a subsequent step. The resulting mixture is divided to a plurality of containers again, and the containers can be reacted with different second building blocks, respectively, to prepare solid-phase reagents by which the first BB and the second BB linked via a first linker (first L) are supported. Again, a group of reagents having the common reactive functional group on the second building blocks in this solid- phase reagent group is homogeneously mixed for use in reaction in a subsequent step. The resulting mixture is divided to a plurality of containers again. These steps are repeated as many times as necessary. Then, compounds can be cleaved from the solid phase to provide a compound group having diversity in core blocks and linkers while having exponential diversity within the same flask.

This series of procedures is regarded as one round, and similar procedures are separately performed for another building block group so that the diversity of compounds can be enhanced while the quality of compounds prepared in one flask is ensured.

Various protective groups may be exploited in the preparation of such a library that achieves both the bond diversity and the ensuring of quality. Specifically, the exploitation of a protective group enables side reaction to be circumvented in bond formation reaction, enables bond diversity to be achieved by exposing the functional groups used in an arbitrary step, and enables an arbitrary hydrogen bond-donating group to be contained in the compounds of interest.

As for the method for producing a compound library provided in one aspect of the present invention, it has been able to be confirmed in actual cases as a result of diligent studies that in actuality, this method can markedly improve the diversity of molecules by the combination of core blocks and linkers, and is capable of constructing a library with ensured quality of compounds constituting the library (the presence or absence of each compound, abundance ratio, and the presence or absence of contamination by impurities). It has also been confirmed that hit compounds that can specifically bind to target molecules are obtained using the library. As a result, the present invention has been completed as a practical method for enhancing the efficiency of screening against an arbitrary target molecule. One aspect of the present invention provides the following invention.

[A1] A library comprising $1 \times 10^2$ to $1 \times 10^8$ compounds, wherein
the compounds have the following structure where a first core block (first CB), a first linker (first L), and a second core block (second CB) are covalently linked:

(first CB)-(first L)-(second CB), wherein
the library comprises two or more types of first CB, two or more types of first L, and two or more types of second CB, and the library is constituted by one or two or more mixtures comprising $1 \times 10^2$ to $1 \times 10^5$ of the compounds.

[A2] The library according to [A1], wherein
the compounds have the following structure where a second linker (second L) and a third core block (third CB) are further covalently linked:

(first CB)-(first L)-(second CB)-(second L)-(third CB), and
the library comprises two or more types of second L and two or more types of third CB.
[A3] The library according to [A1] or [A2], wherein
the compounds have the following structure where a third linker (third L) and a fourth core block (fourth CB) are further covalently linked:

(first CB)-(first L)-(second CB)-(second L)-(third CB)-(third L)-(fourth CB); or (first CB)-(first L)-(second CB)(-(third L)-(fourth CB))-(second L)-(third CB), and
the library comprises two or more types of third L and two or more types of fourth CB.

[A4] The library according to any of [A1] to [A3], wherein the compounds comprise no oligonucleotide tag.
[A5] The library according to any of [A1] to [A4], wherein the first L, the second L, and the third L have a structure independently selected from the group consisting of amide, sulfonamide, acylsulfonamide, amino, ether, thioether, ester, ketone, sulfone, a single bond, ethyne-1,2-diyl (-C=C-), and nitrogen-containing 5-membered heteroarylene.
[A6] The library according to any of [A1] to [A5], wherein the first L, the second L, and the third L are each independently represented by a formula selected from

$-C(=O)-NR^1-$

$-NR^1-C(=O)-$

$-SO_2-NR^1-$

$-NR^1-SO_2-$

$-SO_2-NR^1-C(=O)-$

$-C(=O)-NR^1-SO_2-$

$-NR^1-$

-O-

-S-

-C(=O)-O-

-O-C(=O)-

-C(=O)-

$-SO_2-$

$-C\equiv C-$

$-CHR^2-$

a single bond, and

[Formula 1]

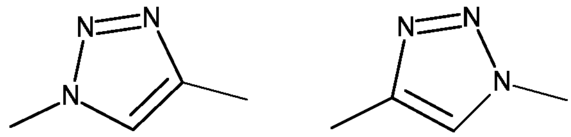

or

wherein $R^1$ and $R^2$ are each independently a hydrogen atom or $C_{1-6}$ alkyl.

[A7] The library according to [A5] or [A6], wherein the single bond is a carbon- nitrogen single bond or a carbon-carbon single bond.

[A8] The library according to any of [A1] to [A7], wherein the first L, the second L, and the third L are formed through reaction independently selected from the group consisting of amidation, sulfonamidation, acylsulfonamidation, reductive amination, etherification, thioetherification, esterification, O-alkylation, N-alkylation, ketone bond formation reaction, carbon-nitrogen bond formation reaction, carbon-carbon bond formation reaction, and nitrogen-containing 5-membered heteroarylene bond formation reaction.

[A9] The library according to any of [A1] to [A8], wherein the first L, the second L, and the third L are formed through reaction independently selected from the group consisting of amidation, sulfonamidation, acylsulfonamidation, reductive amination, O-alkylation, and carbon-carbon bond formation reaction.

[A10] The library according to any of [A7] to [A9], wherein the carbon-nitrogen bond formation reaction is aromatic nucleophilic substitution reaction and/or Buchwald- Hartwig coupling, and the carbon-carbon bond formation reaction is Suzuki coupling, Negishi coupling, Stille coupling and/or Sonogashira coupling.

[A11] The library according to any of [A1] to [A9], wherein the library comprises 100 or more, 200 or more, 300 or more, 400 or more, 500 or more, or 1000 or more compounds.

[A12] The library according to any of [A1] to [A11], wherein the library comprises $1 \times 10^7$ or less, $5 \times 10^6$ or less, $1 \times 10^6$ or less, $5 \times 10^5$ or less, $1 \times 10^5$ or less, 50000 or less, 20000 or less, 10000 or less, 8000 or less, 7000 or less, 6000 or less, 5000 or less, 4000 or less, 3000 or less, 2000 or less, 1500 or less, or 1200 or less compounds.

[A13] The library according to any of [A1] to [A12], wherein the compounds constituting the compound library satisfy one or more of the following conditions:

1) a standard deviation of a clogp value calculated as to each compound is 1.2 or more, 1.3 or more, 1.4 or more, or 1.5 or more;
2) a dispersion of the clogp value calculated as to each compound is 1.5 or more, 1.75 or more, 2.0 or more, or 2.5 or more;
3) a median of Fsp3 of [(the number of sp3 carbon atoms contained in the molecule) / the number of atoms other than hydrogen contained in the molecule] calculated as to each compound is 0.10 or more, 0.15 or more, 0.20 or more, 0.25 or more, or 0.30 or more;
4) a standard deviation of a molecular weight calculated as to each compound is 25 or more, 50 or more, or 75 or more;
5) a median of the number of hydrogen bond-accepting groups in each compound is 2 to 30, 3 to 20, or 3 to 18;
6) a difference between a compound with the largest number of hydrogen bond- donating groups and a compound with the smallest number of hydrogen bond-donating groups among the compounds is 0 to 10, 0 to 8, or 1 to 5; and
7) a dispersion of tPSA (topological polar surface area) calculated as to each compound is 20 or more, 25 or more, 30 or more; and
8) a median of Tanimoto similarity (ECFP_6: extended-connectivity fingerprints up to 6 bonds differentiated at an atomic level, for example, a value calculated by ECFP_6 using Pipeline Pilot 2017HF2 from Dassault Systemes S.E.) calculated as to each compound is 0.98 or less, 0.95 or less, 0.90 or less, 0.85 or less, or 0.80 or less; and
9) the median of Tanimoto similarity (ECFP_6: extended-connectivity fingerprints up to 6 bonds differentiated at an atomic level, for example, a value calculated by ECFP_6 using Pipeline Pilot 2017HF2 from Dassault Systemes S.E.) calculated as to each compound is 0.50 or more, 0.55 or more, 0.60 or more, 0.65 or more, 0.70 or more, or 0.75 or more;

[A14] The library according to any of [A1] to [A13], wherein the library is produced as a mixture of $1 \times 10^2$ to $1 \times 10^5$ compounds.

[A15] The library according to any of [A1] to [A14], wherein as for one, two, or all members selected from the group consisting of the first CB, the second CB, and the third CB,

1) the total number of carbon atoms, nitrogen atoms, and oxygen atoms included in the constituent atoms is 1 or more and 18 or less, 2 or more and 17 or less, 3 or more and 16 or less, 4 or more and 15 or less, 5 or more and

14 or less, or 6 or more and 13 or less.

[A16] The library according to any of [A1] to [A15], wherein one, two, or all members selected from the group consisting of the first CB, the second CB, and the third CB have at least one or more cyclic structures.

[A17] The library according to any of [A1] to [A16], wherein as for one, two, three, or all members selected from the group consisting of the first CB, the second CB, the third CB, and the fourth CB,

1) the total number of carbon atoms, nitrogen atoms, and oxygen atoms included in the constituent atoms is 1 or more and 18 or less, 2 or more and 17 or less, 3 or more and 16 or less, 4 or more and 15 or less, 5 or more and 14 or less, or 6 or more and 13 or less.

[A18] The library according to any of [A1] to [A17], wherein one, two, three, or all members selected from the group consisting of the first CB, the second CB, the third CB, and the fourth CB have at least one or more cyclic structures.

[A19] The library according to any of [A1] to [A18], wherein a ratio between a compound contained at the largest molar quantity (compound A) and a compound contained at the smallest molar quantity (compound B) (compound A/compound B) in the mixture of the compounds constituting the library is 500 or less, 400 or less, 300 or less, 200 or less, 100 or less, 90 or less, 80 or less, 70 or less, 60 or less, 50 or less, 40 or less, 30 or less, 20 or less, 10 or less, 5 or less, 4 or less, 3 or less, or 2 or less.

[A20] The library according to any of [A1] to [A19], wherein 80% or more compounds among the compounds constituting the library are detected from a retention time and a mass-to-charge ratio (m/z) in a liquid chromatograph mass spectrometer (LC-MS).

[A21] The library according to any of [A1] to [A20], wherein 90% or more compounds among the compounds constituting the library are detected from a retention time and a mass-to-charge ratio (m/z) in a liquid chromatograph mass spectrometer (LC-MS).

[A22] The library according to any of [A1] to [A21], wherein 91% or more, 92% or more, 93% or more, 94% or more, 95% or more, or 96% or more compounds among the compounds constituting the library are detected from a retention time and a mass-to-charge ratio (m/z) in a liquid chromatograph mass spectrometer (LC-MS).

[A23] A library assembly comprising 200 or more compounds, the library assembly being prepared by combining two or more libraries according to any of [A1] to [A22].

[B1] A method for screening for a compound that binds to a target molecule, comprising:

(1) providing a library according to any of [A1] to [A23];
(2) bringing each of the one or two or more mixtures constituting the library into contact with the target molecule; and
(3) identifying a compound that binds to the target molecule.

[B2] The method according to [B1], wherein the target molecule is a protein, a nucleic acid, a polypeptide, or a sugar chain.

[B3] The method according to [B1] or [B2], wherein the identification of the compound that binds to the target molecule is carried out by AS-MS.

[B4] The method according to any of [B1] to [B3], wherein the mixtures comprise 100 to 50000, 100 to 10000, 200 to 5000, 400 to 3000, or 600 to 1500 compounds.

[B5] The method according to any of [B1] to [B4], wherein 2 to 100000, 2 to 50000, 2 to 20000, 2 to 10000, 2 to 5000, or 2 to 2500 mixtures contained in the library are screened.

[C1] A method for producing a library, comprising:

(a) preparing supporting carriers to which a first building block (first BB) is covalently linked (first BB-supporting carriers), wherein the first BB-supporting carriers are solid-phase carriers comprising na types of first building blocks (first $BB_1$ to first $BB_{na}$) (first $BB_{1-na}$-supporting carriers), the first $BB_{1-na}$ moieties of the first $BB_{1-na}$-supporting carriers each have a reactive functional group for forming a bond with a second building block (second BB), and the reactive functional groups contained in the first $BB_{1-na}$-supporting carriers are of one type or two or more types;
(b) preparing mixtures of supporting carriers having the reactive functional group common among the first $BB_{1-na}$-supporting carriers, and reacting each mixture with one type of second building block introduction reagent (second BB introduction reagent) or a mixture of two or more types of second BB introduction reagents to prepare supporting carriers in which the second BB is covalently linked to the first BB (second BB-first BB-supporting carriers), wherein the resulting supporting carriers are supporting carriers comprising nb types of second building blocks (second $BB_1$ to second $BB_{nb}$) (second $BB_{1-nb}$-first $BB_{1-na}$- supporting carriers), and any of the second $BB_1$ introduction reagent to the second $BB_{nb}$ introduction reagent reacts with the respective reactive functional groups of the first $BB_{1-na}$-supporting carriers to form first linkers (first L); and

(e) cleaving compounds from the solid-phase carriers, wherein
na is an integer of 2 or larger, nb is an integer of 2 or larger, and the resulting compound library comprises 100 or more types of compounds as a mixture.

[C2] The method according to [C1], wherein (b) comprises preparing mixtures of supporting carriers having the reactive functional group common among the first $BB_{1-na}$- supporting carriers, and reacting each mixture with one of nb types of second BB introduction reagents.
[C3] The method according to [C1] or [C2], wherein

the second $BB_{1-nb}$-first $BB_{1-na}$-supporting carriers each have a reactive functional group capable of forming a linker with a third building block (third BB), and the reactive functional group that is singly contained in each of the first $BB_{1-na}$ moieties or the second $BB_{1-nb}$ moieties is of one type or two or more types,
the method further comprising

(c) preparing mixtures of supporting carriers having the reactive functional group common among the second $BB_{1-nb}$-first $BB_{1-na}$-supporting carriers, and reacting each mixture with one type of third building block introduction reagent (third BB introduction reagent) or a mixture of two or more types of third BB introduction reagents to prepare supporting carriers in which the third BB is covalently linked to the first BB or the second BB (third BB-second BB-first BB-supporting carriers or second BB-(third BB-)first BB-supporting carriers), wherein the resulting supporting carriers are supporting carriers comprising nc types of third building blocks (third $BB_1$ to third $BB_{nc}$) (third $BB_{1-nc}$-second $BB_{1-nb}$-first $BB_{1-na}$-supporting carriers or second $BB_{1-nb}$-(third $BB_{1-nc}$-)first $BB_{1-na}$-supporting carriers), and any of the third $BB_1$ introduction reagent to the third $BB_{nc}$ introduction reagent reacts with the respective reactive functional groups of the second $BB_{1-nb}$-first $BB_{1-na}$-supporting carriers to form second linkers (second L), wherein

nc is an integer of 2 or larger, and the first linkers or the second linkers of the compounds contained in the library are of two or more types.

[C4] The method according to [C3], wherein in (c), the resulting supporting carriers are third $BB_{1-nc}$-second $BB_{1-nb}$-first $BB_{1-na}$-supporting carriers.
[C5] The method according to any of [C1] to [C4], wherein the third $BB_{1-nc}$-second $BB_{1-nb}$-first $BB_{1-na}$-supporting carriers or the second $BB_{1-nb}$-(third $BB_{1-nc}$-)first $BB_{1-na}$- supporting carriers each have a reactive functional group for forming a bond with a fourth building block (fourth BB), and the reactive functional group singly contained in each of the first $BB_{1-na}$ moieties, the second $BB_{1-nb}$ moieties, or the third $BB_{1-nc}$ moieties is of one type or two or more types,
the method further comprising

(d) preparing mixtures of supporting carriers having the reactive functional group common among the third $BB_{1-nc}$-second $BB_{1-na}$-first $BB_{1-na}$-supporting carriers or the second $BB_{1-nb}$-(third $BB_{1-nc}$-)first $BB_{1-na}$-supporting carriers, and reacting each mixture with one type of fourth building block introduction reagent (fourth BB introduction reagent) or a mixture of two or more types of fourth BB introduction reagents to prepare supporting carriers in which the fourth BB is covalently linked to the third BB, the second BB, or the first BB, wherein the resulting supporting carriers are supporting carriers comprising nd types of fourth building blocks (fourth $BB_1$ to fourth $BB_{nd}$), and any of the fourth $BB_1$ introduction reagent to the fourth $BB_{nd}$ introduction reagent reacts with the respective reactive functional groups of the third $BB_{1-nc}$-second $BB_{1-nb}$-first $BB_{1-na}$-supporting carriers or the second $BB_{1-nb}$-(third $BB_{1-nc}$-)first $BB_{1-na}$-supporting carriers to form third linkers, wherein nd is an integer of 2 or larger, and the first linkers, the second linkers or the third linkers of the compounds contained in the library are of two or more types.

[C6] The method according to [C5], wherein in (d), the resulting supporting carriers are

fourth $BB_{1-nd}$-third $BB_{1-nc}$-second $BB_{1-nb}$-first $BB_{1-na}$-supporting carriers;
fourth $BB_{1-nd}$-(third $BB_{1-nc}$-)second $BB_{1-nb}$-first $BB_{1-na}$-supporting carriers;
third $BB_{1-nc}$-second $BB_{1-nb}$-(fourth $BB_{1-nd}$-)first $BB_{1-na}$-supporting carriers;
fourth $BB_{1-nd}$-third $BB_{1-nc}$-(second $BB_{1-nb}$-)first $BB_{1-na}$-supporting carriers; or
fourth $BB_{1-nd}$-second $BB_{1-nb}$-(third $BB_{1-nc}$-)first $BB_{1-na}$-supporting carriers.

[C7] The method according to [C5] or [C6], wherein in (d), the resulting supporting carriers are fourth $BB_{1-nd}$-third $BB_{1-nc}$-second $BB_{1-nb}$-first $BB_{1-na}$-supporting carriers.
[C8] The method according to any of [C1] to [C7], wherein

the first linkers and the second linkers satisfy one or more of the following conditions:

1-1) the first linkers are of two or more types;
1-2) the second linkers are of two or more types; and
1-3) the first linkers or the second linkers are of three or more types, or

the first linkers, the second linkers and the third linkers satisfy one or more of the following conditions:

2-1) the first linkers are of two or more types;
2-2) the second linkers are of two or more types;
2-3) the third linkers are of two or more types; and
2-4) the first linkers, the second linkers or the third linkers are of three or more types.

[C9] The method according to any of [C1] to [C8], wherein

na is an integer of 2 to 1000, nb is an integer of 2 to 1000, and any of na and nb is an integer of 3 or larger; or
na is an integer of 2 to 1000, nb is an integer of 2 to 1000, nc is an integer of 2 to 1000, and any of na, nb and nc is an integer of 3 or larger.

[C10] The method according to any of [C1] to [C9], wherein one type of first BB introduction reagent among the first $BB_1$ introduction reagent to the first $BB_{na}$ introduction reagent is reacted with solid-phase carriers so that the first BB is introduced to the solid- phase carriers, and the first $BB_{1-na}$-supporting carriers are prepared using the resulting first BB-supporting carriers.

[C11] The method according to any of [C1] to [C10], wherein a mixture of two to na types of first BB introduction reagents among the first $BB_1$ introduction reagent to the first $BB_{na}$ introduction reagent is reacted with solid-phase carriers so that the first BB is introduced to the solid-phase carriers to prepare the first $BB_{1-na}$-supporting carriers.

[C12] The method according to any of [C1] to [C11], wherein the first $BB_{1-na}$ moieties of the first $BB_{1-na}$-supporting carriers each have one reactive functional group for forming a bond with a second building block (second BB), and/or the second $BB_{1-nb}$ moieties of the second $BB_{1-nb}$-first $BB_{1-na}$-supporting carriers each have one reactive functional group for forming a bond with a third building block (third BB).

[C13] The method according to any of [C1] to [C11], wherein the first $BB_{1-na}$ moieties of the first $BB_{1-na}$-supporting carriers each have one reactive functional group for forming a bond with a second building block (second BB), and/or the first $BB_{1-na}$ moieties of the second $BB_{1-nb}$-first $BB_{1-na}$-supporting carriers each have one reactive functional group for forming a bond with a third building block (third BB).

[C14] The method according to any of [C1] to [C13], further comprising a deprotection step, a protection step, or a derivatization step for the reactive functional group.

[C15] The method according to [C14], further comprising, after the deprotection step for the reactive functional group, the step of washing the solid-phase supporting carriers using a solution mixed with an acidic component.

[C16] The method according to [C15], wherein the acidic component is an acidic compound having pKa of 0 to 10 in water.

[C17] The method according to [C15] or [C16], wherein the acidic component is at least one member selected from the group consisting of 1-hydroxy-7-azabenzotriazole (HOAt), 1-hydroxybenzotriazole (HOBt), ethyl (hydroxyimino)cyanoacetate (oxyma) and 4- nitrophenol.

[C18] The method according to any of [C15] to [C17], wherein a functional group formed by the deprotection step for the reactive functional group is carboxyl or a hydroxy- substituted aromatic ring.

[C19] The method according to any of [C1] to [C18], further comprising the step of linking two cross-linkable groups present in the molecule to obtain a cyclic compound.

[C20] The method according to any of [C1] to [C19], further comprising modifying the functional groups contained in the compounds cleaved from the solid-phase carriers in the step (e).

[C21] The method according to [C20], wherein the functional group is a functional group resulting from the cleavage of the compounds from the solid-phase carriers.

[C22] The method according to [C20] or [C21], wherein the modification is the $C_{1-6}$ alkylation of hydroxy or carboxy.

[C23] The method according to any of [C1] to [C22], wherein

the step (b) further comprises reacting a remaining reactive functional group of the first BB in first BB-supporting carriers having less than 100% conversion rate of the reaction between the second BB introduction reagent and the reactive functional group of the first BB, with a different second BB introduction reagent having higher reactivity, and/or

the step (c) further comprises reacting a remaining reactive functional group in second BB-first BB-supporting carriers having less than 100% conversion rate of the reaction between the third BB introduction reagent and the reactive functional group, with a different third BB introduction reagent having higher reactivity, and/or
the step (d) further comprises reacting a remaining reactive functional group in third BB-second BB-first BB-supporting carriers or second BB-(third BB-)first BB-supporting carriers having less than 100% conversion rate of the reaction between the fourth BB introduction reagent and the reactive functional group, with a different fourth BB introduction reagent having higher reactivity.

[C24] The method according to any of [C1] to [C23], wherein one or more compounds contained in the library comprise a UV tag.
[C25] The method according to [C24], wherein

the UV tag has a structure represented by formula 1:

[Formula 2]

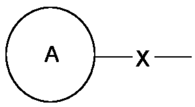

(1)

wherein ring A is selected from benzene, naphthalene, anthracene and biphenyl;
the benzene, the naphthalene, the anthracene and the biphenyl are each optionally substituted by 1 to 3 identical or different groups selected from the group consisting of a halogen atom, nitro, cyano, carboxy, sulfoxy, $-COR^1$, $-OR^1$, $-NR^1R^2$, $-CONR^1R^2$,

and $-SO_2NR^1R^2$; $R^1$ and $R^2$ are each independently $C_{1-6}$ alkyl, or optionally form a hetero ring together with the nitrogen atom linked thereto; and X represents that one member selected from the group consisting of -CONH-, -NHCO-, -CHzO- and -O- is bonded to a building block.
[C26] The method according to [C24], wherein
the UV tag has a structure represented by formula 2 or formula 3:

[Formula 3]

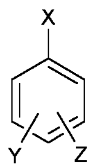

(2)

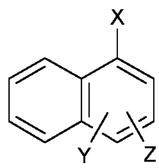

(3)

wherein X is a divalent group selected from the group consisting of -CONH-, -NHCO-, - CHzO- and -O-, and the group is bonded to a building block; Y and Z are each optionally substituted by 1 to 3 identical or different groups selected from the group consisting of a hydrogen atom, halogen, nitro, cyano, carboxy, sulfoxyl, $-COR^1$, $-OR^1$, $-NR^1R^2$, $-CONR^1R^2$, and $-SO_2NR^1R^2$; and $R^1$ and $R^2$ are each independently $C_{1-6}$ alkyl, or optionally together form a hetero ring.
[C27] The method according to [C24], wherein
the UV tag has a structure represented by formula 3:

[Formula 4]

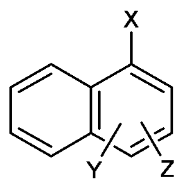

(3)

wherein X represents that one member selected from the group consisting of -CONH-, - NHCO-, -CHzO- and -O- is bonded to a building block; Y and Z are each optionally substituted by 1 to 3 identical or different groups selected from the group consisting of a hydrogen atom, a halogen atom, nitro, cyano, carboxy, sulfoxyl, $-COR^1$, $-O-R^1$, $-NR^1R^2$, - $CO-NR^1R^2$, and $-SO_2-NR^1R^2$; and $R^1$ and $R^2$ are each independently $C_{1-6}$ alkyl, or optionally together form a hetero ring.

[C28] The method according to [C24], wherein the UV tag has a structure represented by formula 4:

[Formula 5]

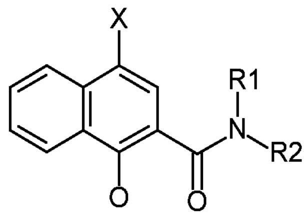

(4)

wherein X represents that one member selected from the group consisting of -CONH-, - NHCO-, -CHzO- and -O- is bonded to a building block; and $R^1$ and $R^2$ are each independently $C_{1-6}$ alkyl, or optionally form a hetero ring together with the nitrogen atom linked thereto.

[C29] The method according to any of [C1] to [C28], wherein the compounds contained in the library comprise no oligonucleotide tag.

[C30] The method according to any of [C1] to [C23] and [C29], wherein the compounds contained in the library comprise no UV tag.

[C31] The method according to any of [C1] to [C30], further comprising the steps of:

1) obtaining solid-phase supporting carriers obtained in any step;
2) cleaving intermediate compounds from the solid-phase supporting carriers obtained in the step 1); and
3) analyzing the intermediate compounds; and

optionally comprising the step of analyzing the compounds cleaved in the step (e).

[C32] The method according to [C31], wherein the analysis is the step of conducting analysis using a liquid chromatogram.

[C33] The method according to any of [C1] to [C32], wherein the first L, the second L, the third L and the fourth L are formed through reaction independently selected from the group consisting of amidation, sulfonamidation, acylsulfonamidation, reductive amination, etherification, thioetherification, esterification, O-alkylation, N-alkylation, ketone bond formation reaction, carbon-nitrogen bond formation reaction, carbon-carbon bond formation reaction, and nitrogen-containing 5-membered heteroarylene formation reaction.

[C34] The method according to any of [C1] to [C33], wherein the first L, the second L, the third L and the fourth L have a structure independently selected from the group consisting of amide, sulfonamide, acylsulfonamide, amino, ether, thioether, ester, ketone, sulfone, a single bond, ethyne-1,2-diyl (-C---C-), and nitrogen-containing 5-membered heteroarylene.

[C35] The method according to any of [C1] to [C34], wherein the carbon-nitrogen bond formation reaction is aromatic nucleophilic substitution reaction and/or Buchwald- Hartwig coupling, and the carbon-carbon bond formation reaction is Suzuki coupling, Negishi coupling, Stille coupling and/or Sonogashira coupling.

[C36] The method according to any of [C1] to [C35], wherein the resulting library comprises 100 or more, 200 or more, 300 or more, 400 or more, 500 or more, or 1000 or more compounds.

[C37] The method according to any of [C1] to [C36], wherein the resulting compound library comprises $1 \times 10^8$ or less, $1 \times 10^7$ or less, $5 \times 10^6$ or less, $1 \times 10^6$ or less, 500000 or less, 100000 or less, 50000 or less, 20000 or less, 10000 or less, 8000 or less, 7000 or less, 6000 or less, 5000 or less, 4000 or less, 3000 or less, 2000 or less, 1500 or less, or 1200 or less compounds.

[C38] The method according to any of [C1] to [C37], wherein production is confirmed as to 90% or more compounds among the compounds of interest designed so as to be synthesized as the compounds constituting the library.

[C39] The method according to any of [C1] to [C38], wherein production is confirmed as to 91% or more, 92% or more, 93% or more, 94% or more, 95% or more, or 96% or more compounds among the compounds of interest designed so as to be synthesized as the compounds constituting the library.

[C40] The method according to [C38] or [C39], wherein the synthesis is confirmed from a retention time and a mass-to-charge ratio (m/z) in a liquid chromatograph mass spectrometer (LC-MS).

[C41] The method according to any of [C1] to [C37], wherein the resulting library is a library according to any of [A1] to [A23].

[C42] A method for producing a compound library assembly comprising $1 \times 10^2$ to $1 \times 10^8$, $3 \times 10^2$ to $1 \times 10^8$, $1 \times 10^2$ to $1 \times 10^7$, or $5 \times 10^2$ to $1 \times 10^6$ compounds, the method comprising combining compound libraries prepared by a method according to any of [C1] to [C40].

[D1] A method for forming a carbon-carbon bond through Suzuki coupling reaction, comprising reacting compound V having leaving group $X^8$ on a carbon atom of an aromatic ring with boron atom-containing compound W that permits carbon-carbon bond formation reaction by the substitution of the leaving group and has a boron atom linked to a carbon atom, in the presence of a palladium catalyst including at least one member selected from the group consisting of a 1,3,5,7-tetramethyl-6-phenyl-2,4,6-trioxa-6-phosphaadamantane palladium fourth-generation Buchwald precatalyst (meCgPPh Pd G4) and [1,1'-bis(di-tert- butylphosphino)ferrocene]dichloropalladium(II) ((dtbpf)PdClz), and an organic base, wherein

$X^8$ is a halogen atom or $-O-SO_2-R^5$; $R^5$ is $C_{1-6}$ alkyl optionally substituted by one or more fluorine atoms, or phenyl optionally substituted by one or more fluorine atoms or $C_{1-6}$ alkyl optionally substituted by a fluorine atom; and the compound V is a resin for solid-phase synthesis, or the boron atom-containing compound W is a resin for solid-phase synthesis.

[D2] The method according to [D1], wherein the organic base is selected from the group consisting of an organic base whose conjugated acid has pKa of 23 or more in acetonitrile, and two or more different bases are optionally used.

[D3] The method according to [D1] or [D2], wherein the organic base is selected from the group consisting of amidines, guanidines, and phosphazenes.

[D4] The method according to any of [D1] to [D3], wherein the organic base is selected from the group consisting of 2-tert-butyl-1,1,3,3-tetramethylguanidine (BTMG), 7- methyl-1,5,7-triazabicyclo[4.4.0]dec-5-ene (MTBD), 1-ethyl-2,2,4,4,4- pentakis(dimethylamino)-$2\lambda^5$,$4\lambda^5$-catenadi(phosphazene) (P2Et), 1-tert-butyl-2,2,4,4,4- pentakis(dimethylamino)-$2\lambda^5$,$4\lambda^5$-catenadi(phosphazene) (P2tBu), tert-butylimino- tris(dimethylamino)phosphorane (P1tBu), 2-tert-butylimino-2-diethylamino-1,3- dimethylperhydro-1,3,2-diazaphospholine (BEMP), and tert-butylimino- tri(pyrrolidino)phosphorane (BTPP).

[D5] The method according to any of [D1] to [D4], wherein the organic base is 2- tert-butyl-1,1,3,3-tetramethylguanidine (BTMG).

[D6] The method according to any of [D1] to [D5], wherein the compound V is the resin for solid-phase synthesis having compound Va via a degradable linker on a side chain, and
the compound Va comprises a compound represented by $X^8$-$Ar^8$ in a partial chemical structure.

[D7] The method according to any of [D1] to [D6], wherein the moiety $X^8$ is a halogen atom.

[D8] The method according to any of [D1] to [D7], wherein the moiety $X^8$ is a bromine atom.

[D9] The method according to any of [D1] to [D8], wherein the moiety $Ar^8$ is independently selected from the group consisting of phenyl, naphthyl, pyrrolyl, thienyl, furyl, pyridyl, thiazolyl, isothiazolyl, pyrazolyl, oxazolyl, isoxazolyl, imidazolyl, triallyl, pyrimidyl, pyrazinyl, pyridazinyl, quinolinyl, isoquinolinyl, 4H-quinolizinyl, phthalazinyl, naphthyridinyl, quinoxalinyl, quinazolinyl, cinnolinyl, pteridinyl, indolyl, indolinyl, benzothiophenyl, benzofuranyl, benzisothiazolyl, benzisoxazolyl, indazolyl, benzimidazolyl, benzotriazolyl, azaindolyl, and imidazopyridyl which are each optionally substituted.

[D10] The method according to any of [D1] to [D9], wherein the moiety $Ar^8$ is selected from the group consisting of phenyl and pyridyl which are each optionally substituted.

[D11] The method according to any of [D1] to [D10], wherein

the resin for solid-phase synthesis has boron atom-containing compound Wa via a degradable linker on a side chain,
the boron atom-containing compound Wa is a compound represented by $X^9$-$Ar^9$ or $X^9$-$CR^6R^7R^8$ having a boron atom-containing group linked to a carbon atom through a boron atom,
$X^9$ is $-B(-OR^{10})(-OR^{11})$, $-BF_3K$, or the following (a), (b), (c), or (d):

[Formula 6]

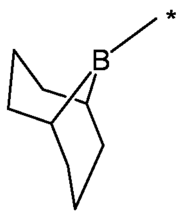

(a)

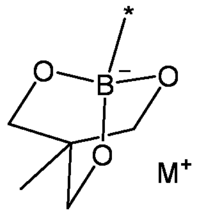

(b)

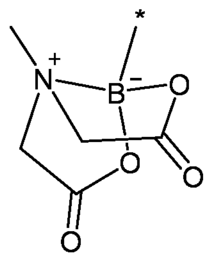

(c)

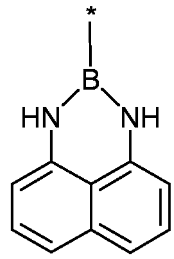

(d)

M is lithium, sodium, or potassium,

$R^{10}$ and $R^{11}$ are each independently a hydrogen atom, or $C_{1-6}$ alkyl optionally substituted by $C_{1-6}$ alkoxy or $C_{6-10}$ aryl, or $R^{10}$ and $R^{11}$ form a 5- to 8-membered saturated or unsaturated ring together with the intervening oxygen atom and boron atom, and the ring is optionally substituted by one or more substituents selected from $C_{1-6}$ alkyl group, $C_{1-6}$ alkoxy and $C_{6-10}$ aryl,

$Ar^9$ is $C_{6-10}$ aryl, or 5- to 10-membered heteroaryl containing one or more ring heteroatoms each independently selected from O, N and S, each of which is optionally substituted by one or more groups each independently selected from the group consisting of a fluorine atom, cyano, $C_{1-6}$ alkyl, $C_{1-6}$ alkoxy, ($C_{1-6}$ alkoxy)carbonyl, ($C_{1-6}$ alkoxy)carbonylamino, ($C_{1-6}$ alkyl)carbonylamino, ($C_{6-10}$ aryl)carbonylamino, 5- to 10- membered heteroarylcarbonylamino containing one or more ring heteroatoms each independently selected from O, N and S, di($C_{1-6}$ alkyl)amino, 4- to 8-membered cyclic amino, aminocarbonyl, ($C_{1-6}$ alkyl)aminocarbonyl, di($C_{1-6}$ alkyl)aminocarbonyl, and 4- to 8- membered cyclic aminocarbonyl, and

$R^6$, $R^7$ and $R^8$ are each independently a hydrogen atom, $C_{1-6}$ alkyl, $C_{2-6}$ alkenyl, $C_{2-6}$ alkynyl, $C_{3-8}$ cycloalkyl, $C_{7-14}$ aralkyl, ($C_{1-6}$ alkyl)carbonyl, ($C_{6-10}$ aryl)carbonyl, 5- to 10- membered heteroarylcarbonyl containing one or more ring heteroatoms each independently selected from O, N and S, $C_{6-10}$ aryl, or 5- to 10-membered heteroaryl containing one or more ring heteroatoms each independently selected from O, N and S, each of which is optionally substituted by one or more groups each independently selected from the group consisting of a fluorine atom, cyano, $C_{1-6}$ alkyl, $C_{1-6}$ alkoxy, ($C_{1-6}$ alkoxy)carbonyl, ($C_{1-6}$ alkoxy)carbonylamino, ($C_{1-6}$ alkyl)carbonylamino, ($C_{6-10}$ aryl)carbonylamino, 5- to 10- membered heteroarylcarbonylamino containing one or more ring heteroatoms each independently selected from O, N and S, di($C_{1-6}$ alkyl)amino, 4- to 8-membered cyclic amino, aminocarbonyl, ($C_{1-6}$ alkyl)aminocarbonyl, di($C_{1-6}$ alkyl)aminocarbonyl, and 4- to 8- membered cyclic aminocarbonyl.

[D12] The method according to any of [D1] to [D11], wherein

the boron atom-containing compound Wa is $X^9$-$Ar^9$, and

$Ar^9$ is $C_{6-10}$ aryl, or 5- to 10-membered heteroaryl containing one or more ring heteroatoms each independently selected from O, N and S, each of which is optionally substituted by one or more groups each independently selected from the group consisting of a fluorine atom, cyano, $C_{1-6}$ alkyl, $C_{1-6}$ alkoxy, ($C_{1-6}$ alkoxy)carbonyl, ($C_{1-6}$ alkoxy)carbonylamino, ($C_{1-6}$ alkyl)carbonylamino, ($C_{6-10}$ aryl)carbonylamino, 5- to 10- membered heteroarylcarbonylamino containing one or more ring heteroatoms each independently selected from O, N and S, di($C_{1-6}$ alkyl)amino, 4- to 8-membered cyclic amino, aminocarbonyl, ($C_{1-6}$ alkyl)aminocarbonyl, di($C_{1-6}$ alkyl)aminocarbonyl, and 4- to 8- membered cyclic aminocarbonyl.

[D13] The method according to any of [D1] to [D12], wherein the reaction is performed in the presence of a solvent.

[D14] The method according to [D13], wherein the solvent is selected from the group consisting of a halogen solvent, a nitrile solvent, an amide solvent, an ether solvent, and an aromatic hydrocarbon solvent, two or more of which are optionally used.

[D15] The method according to [D13], wherein the solvent is selected from the group consisting of an ether solvent, two or more of which are optionally used.

[D16] The method according to [D15], wherein the ether solvent is selected from the group consisting of tetrahydrofuran, diethyl ether, 2-methyltetrahydrofuran, 1,4-dioxane, 1,2- dimethoxyethane, 1,3-dioxolane, diisopropyl ether, cyclopentyl methyl ether, t-butyl methyl ether, and 4-methyltetrahydropyran, two or more of which are optionally used.

[D17] The method according to [D15], wherein the ether solvent is tetrahydrofuran.

[D18] The method according to any of [D1] to [D17], wherein the reaction is performed at a reaction temperature of 0°C to 100°C.

[D19] The method according to [D18], wherein the reaction temperature is 20°C to 90°C, 40°C to 80°C, 50°C to 70°C, or 55°C to 65°C.

[D20] The method according to any of [D1] to [D19], wherein the resin for solid- phase synthesis is bound to the compound Va via a linker having a benzyl ether structure, or bound to the boron atom-containing compound Wa via a linker having a benzyl ether structure.

[D21] The method according to [D20], wherein the benzyl ether structure is represented by the following formula:

[Formula 7]

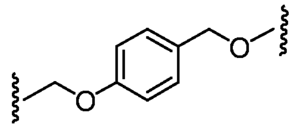

[D22] The method according to any of [D1] to [D21], wherein the reaction is performed in a mixture containing a resin for solid-phase synthesis comprising two or more different compounds Va on a side chain as a substrate.

[D23] The method according to any of [D1] to [D22], wherein the reaction is performed in a mixture containing a resin for solid-phase synthesis comprising two or more different boron atom-containing compounds Wa on a side chain as a substrate.

[D24] The method according to any of [D1] to [D23], wherein the mixture contains resins for solid-phase syntheses bound to two or more different compounds Va or resins for solid-phase syntheses bound to two or more different boron atom-containing compounds Wa as a substrate, and the compounds Va or the boron atom-containing compounds Wa bound to the individual resins are the same.

[D25] A method for producing compounds constituting a compound library, comprising a method of forming a carbon-carbon bond by a method according to any one of [D1] to [D24].

ADVANTAGEOUS EFFECTS OF INVENTION

The present specification provides a library constituted by compounds having the diversity in their chemical structures. The present specification further provides an assay method using the library, and a method for producing the library.

BRIEF DESCRIPTION OF DRAWINGS

Figure 1 is a diagram showing the "diversity of core blocks" and the "diversity of linkers" contained in a library synthesized in Example 3.

Figure 2 is a diagram showing the "diversity of core blocks" and the "diversity of linkers" contained in a library synthesized in Example 4.

Figure 3 is a diagram showing the X ray crystal structure (6QGG) of a complex of Bcl-2 protein and an ABT-737 analog registered in PDB (Protein Data Bank), and the chemical structural formula of the ABT-737 analog.

Figure 4 is a diagram showing the "diversity of core blocks" and the "diversity of linkers" contained in a library synthesized in Example 8.

Figure 5A is a diagram showing step names, mixture names, and library numbers in library synthesis in Example 8. In Figure 5A, Figure 5B, and Figure 5C, the step names are described above horizontal lines, and the mixture names are described in boxes. The step names and the mixture names in Figure 5A, Figure 5B, and Figure 5C are described with the library number "2-1-" omitted. For example, "h01" represents the step "2-1-h01", and "h01A01-0" represents the mixture "2-1-h01 A01-0".

Figure 5B is a portion of Figure 5 showing step names, mixture names, and library numbers in library synthesis in Example 8.

Figure 5C is a portion of Figure 5 showing step names, mixture names, and library numbers in library synthesis in Example 8.

Figure 6 is a diagram showing the "diversity of core blocks" and the "diversity of linkers" contained in a library

synthesized in Example 9.

Figure 7A is a diagram showing step names, mixture names, and library numbers in library synthesis in Example 9. In Figure 7A, Figure 7B, and Figure 7C, the step names are described above horizontal lines, and the mixture names are described in boxes. The step names and the mixture names in Figure 7A, Figure 7B, and Figure 7C are described with the library number "2-2-" omitted. For example, "h01" represents the step "2-2-h01", and "a1B01-0" represents the mixture "2-2-a1B01-0".

Figure 7B is a portion of Figure 7 showing step names, mixture names, and library numbers in library synthesis in Example 9.

Figure 7C is a portion of Figure 7 showing step names, mixture names, and library numbers in library synthesis in Example 9.

Figure 8 is a diagram showing the "diversity of core blocks" and the "diversity of linkers" contained in a library synthesized in Example 10 (case 1).

Figure 9 is a diagram showing the "diversity of core blocks" and the "diversity of linkers" contained in a library synthesized in Example 10 (case 2).

Figure 10 is a diagram showing step names, mixture names, and library numbers in library synthesis in Example 10 (case 1). In Figure 10, the step names are described above horizontal lines, and the mixture names are described in boxes. The step names and the mixture names in Figure 10 are described with the library number "4-1-" omitted. For example, "a1B01" represents the step "4-1-a1B01", and "a1B01-0" represents the mixture "4- 1-a1B01-0".

Figure 11 is a diagram showing step names, mixture names, and library numbers in library synthesis in Example 10 (case 2). In Figure 11, the step names are described above horizontal lines, and the mixture names are described in boxes. The step names and the mixture names in Figure 11 are described with the library number "4-2-" omitted. For example, "a1B05" represents the step "4-2-a1B05", and "a1B05-0" represents the mixture "4- 1-a1B05-0".

DESCRIPTION OF EMBODIMENTS

In the present specification, "$C_{1-6}$ alkyl" is a monovalent group derived from linear or branched saturated aliphatic hydrocarbon having 1 to 6 carbon atoms by the removal of one arbitrary hydrogen atom. Specific examples thereof include methyl, ethyl, n-propyl, isopropyl, n-butyl, isobutyl, sec-butyl, t-butyl, 1-methylpropyl, n-pentyl, isopentyl, 2- methylbutyl, 1,1-dimethylpropyl, 1-ethylpropyl, hexyl, 4-methylpentyl, and 2-ethylbutyl.

In the present specification, "$C_{3-7}$ cycloalkyl" means a cyclic saturated aliphatic hydrocarbon group having 3 to 7 carbon atoms. Specific examples thereof include cyclopropyl, cyclobutyl, cyclopentyl, cyclohexyl, and cycloheptyl.

In the present invention, "$C_{6-10}$ aryl" means a monovalent aromatic hydrocarbon ring group. Examples of the $C_{6-10}$ aryl include phenyl, 1-naphthyl, and 2-naphthyl.

In the present specification, "halogen atom" means a fluorine atom, a chlorine atom, a bromine atom, an iodine atom, or the like. In the present invention, when the halogen atom serves as a substituent for aryl, heteroaryl, or the like, the halogen atom is preferably a fluorine atom, a chlorine atom or a bromine atom. In the present invention, when the halogen atom serves as a substituent for alkyl or a group containing an alkyl moiety (alkoxy, alkenyl, alkylthio, etc.), the halogen atom is preferably a fluorine atom. Specific examples of the group having a halogen atom as a substituent include trifluoromethyl, pentafluoroethyl, trifluoromethoxy, pentafluoroethoxy, trifluoromethylthio, and pentafluoroethylthio.

One aspect of the present invention provides a library constituted by compounds having a predetermined chemical structure. The number of compounds constituting the library is not particularly limited as long as the library can be subjected to screening. The number of compounds may be, for example, $1 \times 10^2$ to $1 \times 10^8$, $3 \times 10^2$ to $1 \times 10^7$, or $5 \times 10^2$ to $1 \times 10^6$. In this context, the number of compounds means the number of compounds differing in chemical structure, and tautomers, for example, are regarded as the same compound. On the other hand, compounds differing in chemical structure, such as positional isomers or stereoisomers, are interpreted as different compounds. A compound containing an isotope at a ratio largely different from a natural abundance ratio can be interpreted as a compound.

In this context, the lower limit of the number of compounds constituting the library is, for example, 100 or more, 200 or more, 300 or more, 400 or more, 500 or more, or 1000 or more. The upper limit of the number of such compounds is, for example, $1 \times 10^8$ or less, $1 \times 10^7$ or less, $5 \times 10^6$ or less, $1 \times 10^6$ or less, 500000 or less, 100000 or less, 50000 or less, 20000 or less, 10000 or less, 8000 or less, 7000 or less, 6000 or less, 5000 or less, 4000 or less, 3000 or less, 2000 or less, 1500 or less, or 1200 or less.

Each compound constituting the library is not particularly limited as long as the compound can exist stably under screening conditions. The compound may be prepared by synthesis or may be obtained by the purchase of a portion thereof. In one embodiment, the compound comprises atoms selected from a hydrogen atom, a carbon atom, an oxygen atom, a nitrogen atom, a sulfur atom, a halogen atom, and a phosphorus atom. In another embodiment, the compound only consists of atoms selected from a hydrogen atom, a carbon atom, an oxygen atom, a nitrogen atom, a sulfur atom, a halogen atom (e.g., a fluorine atom, a chlorine atom, a bromine atom, and an iodine atom), and a phosphorus

atom. The compound constituting the library has a molecular weight in the range of, for example, but not particularly limited to, 200 to 3000, specifically 300 to 2000, more specifically 300 to 1800, further specifically 400 to 1800. In one aspect of the present invention, the standard deviation of the molecular weight of the compound constituting the library is not particularly limited and is, for example, 25 or more, specifically 50 or more, more specifically 75 or more. The compound constituting the library contains, for example, but not particularly limited to, 7 to 200, specifically 11 to 130, more specifically 11 to 120 carbon atoms; for example, 0 to 40, specifically 2 to 27, more specifically 2 to 24 nitrogen atoms; and, for example, 0 to 30, specifically 2 to 20, more specifically 2 to 18 oxygen atoms. The amount of each compound constituting the library is not particularly limited as long as the library can be used in screening. Each compound can be in the range of, for example, 0.001 to 50000 μg, specifically 0.1 to 40000 μg, particularly specifically 1 to 30000 μg. In one embodiment of the present invention, the ratio between a compound contained in the largest molar quantity (compound A) and a compound contained in the smallest molar quantity (compound B) (compound A/compound B) in a mixture is 500 or less, 400 or less, 300 or less, 200 or less, 100 or less, 90 or less, 80 or less, 70 or less, 60 or less, 50 or less, 40 or less, 30 or less, 20 or less, 10 or less, 5 or less, 4 or less, 3 or less, or 2 or less.

As for the compound constituting the library, the number of compounds contained in one mixture is not particularly limited as long as the library is applicable to screening. For example, 100 to 100000, 100 to 10000, or 100 to 5000 different compounds are contained in the mixture.

In one aspect of the present invention, the compound constituting the library is contained in a mixture. The mixture is not particularly limited as long as the library can be used in screening using a mixture of sample compounds. The mixture may be a concentrated mixture of compounds or may be a solution of compounds diluted with a solvent or the like. The mixture may be prepared by mixing individually synthesized or obtained compounds, or a mixture obtained as a product through chemical reaction with the mixture as a substrate may be used. In one embodiment of the present invention, the library is produced as, for example, a mixture of 100 to 100000, 100 to 10000, or 100 to 5000 different compounds.

In one aspect of the present invention, a parameter (e.g., clogp and topological polar surface area (tPSA)) as to the characteristics of each compound constituting the library may be calculated, and the calculation of the parameter can be performed by a method that is usually performed by those skilled in the art in the technical field to which the present invention belongs. For example, clogp and topological polar surface area (tPSA) can be calculated as to individual compounds by ADMET predicto. In one aspect of the present invention, the standard deviation of the clogp value of the compound constituting the library is, for example, 1.2 or more, specifically 1.3 or more, more specifically 1.4 or more, further specifically 1.5 or more. In a further alternative aspect of the present invention, the dispersion of the tPSA value of the compound constituting the library is, for example, 20 or more, specifically 25 or more, more specifically 30 or more.

In one aspect of the present invention, the compound constituting the library can be characterized by the number of sp3 carbon atoms contained in its chemical structure. In this context, the sp3 carbon atom means a carbon atom having only single bonds as all bonds. In one embodiment, the median of the value of [(the number of sp3 carbon atoms contained in the molecule) / the number of atoms other than hydrogen contained in the molecule] calculated as to each compound is, for example, 0.1 or more, specifically 0.15 or more, more specifically 0.2 or more, further specifically 0.25 or more, most specifically 0.3 or more.

In one aspect of the present invention, the compound constituting the library can be characterized by the number of oxygen atoms contained in its chemical structure. In this context, the oxygen atom means every oxygen atom contained in the chemical structure of the compound and encompasses an oxygen atom contained in a hydroxy group, an ether structure, a carbonyl group, or the like. In one embodiment, the number of oxygen atoms contained in the chemical structure of each compound is in the range of, for example, 0 to 30, specifically 2 to 20, more specifically 2 to 18. In another embodiment, the value of [(the number of oxygen atoms contained in the molecule) / the number of atoms other than hydrogen contained in the molecule] calculated as to each compound is in the range of, for example, 0 to 0.20, specifically 0.01 to 0.20, more specifically 0.03 to 0.17, further specifically 0.06 to 0.14.

In one aspect of the present invention, the compound constituting the library can be characterized by the number of nitrogen atoms contained in its chemical structure. In this context, the nitrogen atom means every nitrogen atom contained in the chemical structure of the compound and encompasses a nitrogen atom contained in an amino group, a pyridine ring (as an endocyclic nitrogen atom), an amide group, an imino group, or the like. In one embodiment, the number of nitrogen atoms contained in the chemical structure of each compound is in the range of, for example, 0 to 40, specifically 2 to 27, more specifically 2 to 24. In another embodiment, the value of [(the number of nitrogen atoms contained in the molecule) / the number of atoms other than hydrogen contained in the molecule] calculated as to each compound is in the range of, for example, 0 to 0.2, specifically 0 to 0.18, more specifically 0.05 to 0.18.

In one embodiment, the total number of oxygen atoms and nitrogen atoms contained in the chemical structure of each compound is in the range of, for example, 1 to 70, specifically 2 to 60, more specifically 2 to 40. In another embodiment, the value of [(the number of oxygen atoms and nitrogen atoms contained in the molecule) / the number of atoms other than hydrogen contained in the molecule] calculated as to each compound is in the range of, for example, 0.01 to 0.35, specifically 0.02 to 0.3, more specifically 0.01 to 0.27.

In one embodiment, the total number of atoms other than a carbon atom and a hydrogen atom contained in the chemical structure of each compound is in the range of, for example, 1 to 85, specifically 2 to 56, more specifically 3 to 50. In another embodiment, the value of [(the number of atoms other than a carbon atom and a hydrogen atom contained in the molecule) / the number of atoms other than hydrogen contained in the molecule] calculated as to each compound is in the range of, for example, 0.05 to 0.45, specifically 0.05 to 0.4, more specifically 0.1 to 0.4.

In one aspect of the present invention, the compound constituting the library can be characterized by the number of basic nitrogen atoms contained in its chemical structure. In this context, the basic nitrogen atom is a nitrogen atom contained in a basic compound, and means a nitrogen atom that allows pKb in water of the compound to be, for example, 14 or less, specifically 13.5 or less, more specifically 13 or less. In one embodiment, the number of basic nitrogen atoms contained in the compound constituting the library is in the range of, for example, 0 to 30, specifically 0 to 20, more specifically 0 to 18.

In one aspect of the present invention, the compound constituting the library can be characterized by the number of hydrogen bond-donating groups contained in its chemical structure. In this context, the hydrogen bond-donating group is a group that has hydrogen atoms covalently bonded and is capable of causing attractive non-covalent interaction with a lone electron pair of nearby located nitrogen, oxygen, sulfur, fluorine, a $\pi$ electron system, or the like. The hydrogen atoms of the group are not particularly limited as long as an atom bonded thereto has higher electronegativity than that of the hydrogen atoms. Examples of such a group include groups having hydrogen atoms that bind to an atom such as an oxygen atom, a nitrogen atom, a sulfur atom, or a phosphorus atom. In one embodiment, the number of hydrogen bond-donating groups in the compound constituting the library is in the range of, for example, 0 to 20, specifically 0 to 15, more specifically 0 to 10. In another embodiment, the difference between a compound with the largest number of hydrogen bond-donating groups and a compound with the smallest number of hydrogen bond-donating groups among the compounds is, for example, 1 or more, specifically 2 or more, more specifically 3 or more.

In one aspect of the present invention, the compound constituting the library can be characterized by the number of hydrogen bond-accepting groups contained in its chemical structure. In this context, the hydrogen bond-accepting group is a group that has a lone electron pair of nitrogen, oxygen, sulfur, fluorine, a $\pi$ electron system, or the like and is capable of causing attractive non-covalent interaction with nearby located hydrogen atoms covalently bonded. The group having a lone electron pair is not particularly limited as long as the group can exist stably. Examples thereof include carbonyl groups such as a ketone group, an ester group, and an amide group, and groups such as an ether group, an amino group, an imino group, a cyano group, a sulfoxide group, and a sulfone group. In one embodiment, the number of hydrogen bond-accepting groups in the compound constituting the library is in the range of, for example, 0 to 40, specifically 0 to 30, more specifically 0 to 20. In another embodiment, the difference between a compound with the largest number of hydrogen bond-donating groups and a compound with the smallest number of hydrogen bond-donating groups among the compounds is, for example, 1 or more, specifically 2 or more, more specifically 3 or more.

In one aspect of the present invention, the compound constituting the library is represented by the following formula which represents a chemical structure where a first core block (first CB), a first linker (first L), and a second core block (second CB) are covalently linked:

(first CB)-(first L)-(second CB)

and characterized in that the compounds of the library share the common chemical structure. In this context, the first CB and the second CB are not particularly limited as long as they are arbitrary chemical structures containing at least one atom and are substructures of the compound. The first CB and the second CB may be any chemical structure as long as a compound different from the other compounds constituting the library is formed. For example, the first CB and the second CB may be the same chemical structures. The first CB and the second CB may contain, as mentioned later, a chemical structure defined as a third core block (third CB) and/or a fourth core block (fourth CB) as a substitution. In this context, the first L is not particularly limited as long as the first L is an arbitrary chemical structure that links the first CB and the second CB. The first L may be, for example, a direct bond (single bond, double bond, or triple bond).

In one aspect of the present invention, the compound constituting the library comprises the following structure where first CB, first L, second CB, a second linker (second L), and a third core block (third CB) are covalently linked:

(first CB)-(first L)-(second CB)-(second L)-(third CB).

In this context, the first CB, the first L, and the second CB are as already mentioned. The third CB is not particularly limited as long as the third CB is an arbitrary chemical structure containing at least one atom and is a substructure of the compound. The third CB may be any chemical structure as long as a compound different from the other compounds constituting the library is formed. For example, the third CB may be the same chemical structure as the first CB and/or the second CB. The first CB, the second CB and the third CB may contain, as mentioned later, a chemical structure defined as a fourth core block (fourth CB) as a substitution. In this context, the second L is not particularly limited as long as the second L is an arbitrary chemical structure that links the second CB and the third CB. The second L may be, for example, a direct bond (single bond, double bond, or triple bond).

In one aspect of the present invention, the compound constituting the library comprises the following structure where first CB, first L, second CB, second L, third CB, a third linker (third L), and a fourth core block (fourth CB) are

covalently linked:

(first CB)-(first L)-(second CB)-(second L)-(third CB)-(third L)-(fourth CB); or
(first CB)-(first L)-(second CB)(-(third L)-(fourth CB))-(second L)-(third CB).

Of the formulas, the second one means that all of -(first L)-(first CB), -(second L)-(third CB), and -(third L)-(fourth CB) are linked to the second CB. In one embodiment, the compounds represented by these two formulas are encompassed within the scope of the compound represented by (first CB)-(first L)-(second CB) described above.

In this context, the first CB, the first L, the second CB, the second L, and the third CB are as already mentioned. The fourth CB is not particularly limited as long as the fourth CB is an arbitrary chemical structure containing at least one atom and is a substructure of the compound. The fourth CB may be any chemical structure as long as a compound different from the other compounds constituting the library is formed. For example, the fourth CB may be the same chemical structure as the first CB and/or the second CB. The first CB, the second CB and the third CB may contain, as mentioned above, the chemical structure defined as the fourth core block (fourth CB) as a substitution. In this context, the second L is not particularly limited as long as the second L is an arbitrary chemical structure that links the second CB and the third CB. The third L may be, for example, a direct bond (single bond, double bond, or triple bond).

In one embodiment, one mixture contains compounds represented by the following formulas:

(first CB)-(first L)-(second CB)-(second L)-(third CB)-(third L)-(fourth CB); and
(first CB)-(first L)-(second CB)(-(third L)-(fourth CB))-(second L)-(third CB).

In another embodiment, one mixture contains compounds represented by the following formula:

(first CB)-(first L)-(second CB)-(second L)-(third CB)-(third L)-(fourth CB); or
(first CB)-(first L)-(second CB)(-(third L)-(fourth CB))-(second L)-(third CB).

In an alternative embodiment, one mixture contains compounds represented by the following formula:
(first CB)-(first L)-(second CB)-(second L)-(third CB)-(third L)-(fourth CB).

The first CB, the second CB, the third CB, and the fourth CB may further have a fifth core block linked via a fourth linker (-(fourth L)-(fifth CB)). In a compound having the group -(fourth L)-(fifth CB), the fourth L and the fifth CB may have chemical structures defined as the first L, the second L, or the third L, and the first CB, the second CB, the third CB, or the fourth CB, respectively, in the present specification. The compound may further have a sixth core block linked via a fifth linker (-(fifth L)-(sixth CB)). A compound having the groups -(fourth L)-(fifth CB) and -(fifth L)-(sixth CB) can be synthesized by a method of sequentially introducing the groups on the basis of a method described in the present specification.

In one embodiment of the present invention, the compound constituting the library comprises no tag for decoding. The tag for decoding is not particularly limited as long as the tag can be used for identifying the structure of the compound. Examples thereof include oligonucleotide tags. A DNA-encoded library (DEL) is known as a library having the tag for decoding. In one embodiment, the library of the present invention excludes a library encoded by oligonucleotides, which is typified by DEL.

In one aspect of the present invention, the linker, for example, the first L, the second L, and the third L, contained in the compound has a structure independently selected from the group consisting of amide, sulfonamide, acylsulfonamide, amino, ether, thioether, ester, ketone, sulfone, a single bond, ethyne-1,2-diyl (-C=C-), and nitrogen-containing 5-membered heteroarylene. In one embodiment, the first L, the second L, and the third L are each independently represented by a formula selected from

$-C(=O)-NR^1-$

$-NR^1-C(=O)-$

$-SO_2-NR^1-$

$-NR^1-SO_2-$

$-SO_2-NR^1-C(=O)-$

$-C(=O)-NR^1-SO_2-$

-$NR^1$-

-O-

-S-

-C(=O)-O-

-O-C(=O)-

-C(=O)-

-$SO_2$-

-C≡C-

-$CHR^2$-

a single bond, and

[Formula 8]

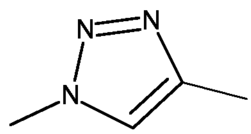

or

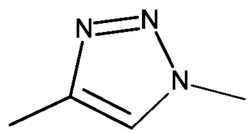

wherein $R^1$ and $R^2$ are each independently a hydrogen atom or $C_{1-6}$ alkyl.

In this context, the single bond is a carbon-nitrogen single bond or a carbon-carbon single bond.

In one aspect of the present invention, the linker is derived from a chemical structure resulting from the linkage of two core blocks selected from each core block, for example, the first CB, the second CB, the third CB, and the fourth CB, via a covalent bond through reaction with a reactive functional group. In one embodiment, the first L, the second L, and the third L are formed through reaction independently selected from the group consisting of amidation, sulfonamidation, acylsulfonamidation, reductive amination, etherification, thioetherification, esterification, O-alkylation, N-alkylation, ketone bond formation reaction, carbon-nitrogen bond formation reaction, carbon-carbon bond formation reaction, and nitrogen-containing 5-membered heteroarylene cyclization. In this context, the carbon- nitrogen bond formation reaction is, for example, aromatic nucleophilic substitution reaction and/or Buchwald-Hartwig coupling, and the carbon-carbon bond formation reaction is, for example, Suzuki coupling, Negishi coupling, Stille coupling and/or Sonogashira coupling.

In one embodiment of the present invention, the compound constituting the library is a compound comprising the first CB to the third CB, the first L and the second L, and as for at least one, at least two, or all members selected from the group consisting of the first CB, the second CB, and the third CB, the total number of carbon atoms, nitrogen atoms, and oxygen atoms in each CB is 2 or more and 17 or less, 3 or more and 16 or less, 4 or more and 15 or less, 5 or more and 14 or less, or 6 or more and 13 or less. In one embodiment of the present invention, all the compounds constituting the library satisfy this condition.

In one embodiment of the present invention, the compound constituting the library is a compound comprising the first CB to the third CB, the first L and the second L, and as for at least one, at least two, or all members selected from the group consisting of the first CB, the second CB, and the third CB, the total number of carbon atoms, nitrogen atoms, and oxygen atoms in each CB is 1 or more and 18 or less. In one embodiment of the present invention, all the compounds constituting the library satisfy this condition.

In one embodiment of the present invention, the compound constituting the library is a compound comprising the first CB to the third CB, the first L and the second L, and at least one, at least two, or all members selected from the group consisting of the first CB, the second CB, and the third CB have at least one or two cyclic structures. In one embodiment of the present invention, all the compounds constituting the library satisfy this condition.

In one embodiment of the present invention, the compound constituting the library is a compound comprising the first CB to the fourth CB and the first L to the third L, and as for at least one, at least two, at least three or all of the first CB, the second CB, the third CB, and the fourth CB, the total number of carbon atoms, nitrogen atoms, and oxygen

atoms in each CB is 2 or more and 17 or less, 3 or more and 16 or less, 4 or more and 15 or less, 5 or more and 14 or less, or 6 or more and 13 or less. In one embodiment of the present invention, all the compounds constituting the library satisfy this condition.

In one embodiment of the present invention, the compound constituting the library is a compound comprising the first CB to the fourth CB and the first L to the third L, and as for at least one, at least two, at least three or all of the first CB, the second CB, the third CB, and the fourth CB, the total number of carbon atoms, nitrogen atoms, and oxygen atoms in each CB is 1 or more and 18 or less. In one embodiment of the present invention, all the compounds constituting the library satisfy this condition.

In one embodiment of the present invention, the compound constituting the library is a compound comprising the first CB to the fourth CB and the first L to the third L, and at least one, at least two, at least three or all of the first CB, the second CB, the third CB, and the fourth CB have at least one or two cyclic structures. In one embodiment of the present invention, all the compounds constituting the library satisfy this condition.

One embodiment of the present invention provides a library comprising $1 \times 10^2$ to $1 \times 10^8$ compounds, wherein the library comprises two or more types of first CB, two or more types of first L, and two or more types of second CB; comprises two or more types of first CB, two or more types of first L, two or more types of second CB, two or more types of second L, and two or more types of third CB; or comprises two or more types of first CB, two or more types of first L, two or more types of second CB, two or more types of second L, two or more types of third CB, two or more types of third L, and two or more types of fourth CB. In one embodiment of the present invention, the total number of types of the first L, the second L, and the third L is 3 or more, 4 or more, 5 or more, 6 or more, 7 or more, or 8 or more. In another embodiment of the present invention, the total number of types of the first L, the second L, and the third L is 3 to 30, 4 to 30, 5 to 30, 6 to 30, 7 to 20, or 8 to 15.

In one embodiment, the present invention provides the following:

a library comprising $1 \times 10^2$ to $1 \times 10^8$ compounds, wherein the compounds constituting the library have the following structure where first CB, first L, second CB, second L, and third CB are covalently linked:
(first CB)-(first L)-(second CB)-(second L)-(third CB),
the total number of types of the first L and the second L is 3 or more, the total number of types of the first CB, the second CB, and the third CB is 6 or more, the library is constituted by one or two or more mixtures comprising $1 \times 10^2$ to $1 \times 10^5$ of the compounds, and 90% or more compounds among the compounds constituting the library are detected from a retention time and a mass-to-charge ratio (m/z) in a liquid chromatograph mass spectrometer (LC-MS).

In one embodiment, the present invention provides the following:

a library comprising $1 \times 10^2$ to $1 \times 10^4$ compounds, wherein the compounds constituting the library have the following structure where first CB, first L, second CB, second L, third CB, third L, and fourth CB are covalently linked:
(first CB)-(first L)-(second CB)-(second L)-(third CB)-(third L)-(fourth CB),
the total number of types of the first L, the second L, and the third L is 5 or more, the total number of types of the first CB, the second CB, the third CB, and the fourth CB is 8 or more, the library is constituted by one or two or more mixtures comprising $1 \times 10^2$ to $5 \times 10^3$ of the compounds, and 95% or more compounds among the compounds constituting the library are detected from a retention time and a mass-to-charge ratio (m/z) in a liquid chromatograph mass spectrometer (LC-MS).

Two or more, 3 or more, 4 or more, 5 or more, 6 or more, 7 or more, 8 or more, 9 or more, 10 or more, 20 or more, 30 or more, 40 or more, 50 or more, or 100 or more of the libraries described above may be prepared and combined to prepare a library assembly. Specifically, one aspect of the present invention provides a library assembly prepared by combining 2 or more, 3 or more, 4 or more, 5 or more, 6 or more, 7 or more, 8 or more, 9 or more, 10 or more, 20 or more, 30 or more, 40 or more, 50 or more, or 100 or more libraries. In one embodiment, the library assembly can comprise, for example, 200 or more, 300 or more, 400 or more, 500 or more, 600 or more, 700 or more, 800 or more, 900 or more, 1000 or more, 2000 or more, 4000 or more, 10000 or more, 50000 or more, or 100000 or more compounds. In one embodiment of the present invention, each individual library constituting the library assembly described above can be a mixture of compounds.

Screening can be performed using the library described in the present specification. Specifically, one embodiment of the present invention provides a method for screening for a compound that binds to a target molecule, the method comprising:

(1) providing the library described in present specification;
(2) bringing each of the one or two or more mixtures constituting the library into contact with the target molecule; and

(3) identifying a compound that binds to the target molecule.

In this context, the target molecule is not particularly limited as long as the molecule is targeted in the search of medicaments. Examples thereof include proteins, nucleic acids, polypeptides, and sugar chains, specifically enzymes, G protein-coupled receptors, ion channels, transporters, and nuclear receptors. Specific examples of the target molecule include Bcl-2, IL-6, A2aR, NMDA glutamate receptors, and estrogen receptors.

The method for identifying a compound that binds to the target molecule is not particularly limited as long as the method is known to those skilled in the art in the technical field to which the present invention belongs. The method is preferably a method capable of identifying the chemical structure of the compound that binds to the target molecule. Examples thereof include approaches using AS-MS (affinity selection mass spectrometry), approaches using SPR (surface plasmon resonance), approaches using X ray crystallography, and approaches using a cryogenic electron microscope and preferably include approaches using AS-MS. An approach using AS-MS can be carried out by, for example, an approach described in Current Opinion in Chemical Biology 2007, 11: 518-526, and can be carried out, for example, using 96-Well MacroSpin Column, P-2 Material (Harvard Bioscience, Inc., 74- 5655) or Panquish UHPLC (Thermo Fisher Scientific Inc.).

The number of compounds contained in the mixture is, for example, 100 to 50000, 100 to 10000, 200 to 5000, 400 to 3000, or 600 to 1500. The number of mixtures subjected to screening is not particularly limited and may be one mixture. The lower limit is, for example, 1 or more, 2 or more, 3 or more, 4 or more, 5 or more, 6 or more, 7 or more, 8 or more, 9 or more, 10 or more, 20 or more, 30 or more, 40 or more, 50 or more, or 100 or more, and the upper limit is, for example, 100000 or less, 50000 or less, 20000 or less, 10000 or less, 5000 or less, 3000 or less, 2000 or less, or 1000 or less. For example, 1 to 5000, 1 to 3000, 2 to 2000, or 2 to 1500 mixtures may each be screened.

One aspect of the present invention provides a method for producing a library, comprising (a) preparing supporting carriers to which first BB is covalently linked (first $BB_{1-na}$-supporting carriers) using na types of first building block introduction reagents (first BB introduction reagents); (b) preparing mixtures of supporting carriers having the reactive functional group common among the first $BB_{1-na}$-supporting carriers, and reacting each mixture with one type of second building block introduction reagent (second BB introduction reagent) or a mixture of two or more types of second BB introduction reagents to prepare supporting carriers in which nb types of second BB are covalently linked to the first BB (second BB-first BB-supporting carriers); and (e) cleaving compounds from the solid-phase carriers. Compounds represented by second $BB_{1-nb}$-first $BB_{1-na}$ can be obtained as a mixture of some or all thereof, and the library can be constituted by the compounds. The compounds represented by second $BB_{1-nb}$-first $BB_{1-na}$ encompass a compound group of na × nb compounds at the maximum produced from na types of first BB--supporting carrier introduction reagents (first $BB_{1-na}$ introduction reagents) and nb types of second BB introduction reagents (second $BB_{1-nb}$-supporting carrier introduction reagents), or some of the compound groups, and a compound group obtained by further modifying the compound group.

In this context, na is an integer of 2 or larger, 3 or larger, 4 or larger, or 5 or larger, and nb is an integer of 2 or larger, 3 or larger, 4 or larger, or 5 or larger. The resulting compound library can comprise 100 or more, 200 or more, 300 or more, 400 or more, 500 or more, or 1000 or more compounds as a mixture.

In the step (a), the first BB-supporting carriers are solid-phase carriers that support na types of first building blocks (first $BB_{1-na}$) (first $BB_{1-na}$-supporting carriers), and the first $BB_{1-na}$ moieties of the first $BB_{1-na}$-supporting carriers each have a reactive functional group for forming a bond with a second building block (second BB). The reactive functional groups contained in the first $BB_{1-na}$-supporting carriers are of one type, two or more types, or three or more types. The first $BB_{1-na}$-supporting carriers can be obtained by reacting solid- phase carriers with one type of first BB introduction reagent using each of first $BB_1$ introduction reagent to first $BB_{na}$ introduction reagent, and first $BB_1$-supporting carriers to first $BB_{na}$-supporting carriers are each obtained as one type of first BB-supporting carrier. A mixture of first BB-supporting carriers (first $BB_{1-na}$-supporting carriers) can be obtained using these supporting carriers. Alternatively, a mixture of first BB introduction reagents (e.g., a mixture of first $BB_{1-na}$ introduction reagents) are reacted with solid-phase carriers, and the resulting mixture of first $BB_{1-na}$-supporting carriers can be used in (b). In one embodiment, some first $BB_{1-na}$-supporting carriers may be obtained as a mixture using a mixture of some first BB introduction reagents, and the first BB introduction reagents to be contained in the mixture can be selected on the basis of the types of reactive functional groups in the first BB introduction reagents.

In the step (b), the resulting supporting carriers are supporting carriers comprising nb types of second building blocks (second $BB_1$ to second $BB_{nb}$) (second $BB_{1-nb}$-first $BB_{1-na}$- supporting carriers), and any of the second $BB_1$ introduction reagent to the second $BB_{nb}$ introduction reagent reacts with the respective reactive functional groups of the first $BB_{1-na}$- supporting carriers to form first linkers (first L).

The solid-phase carrier that can be used in the method for producing a library described in the present specification is not particularly limited as long as the solid-phase carrier is usually used. Examples thereof can include CTC resin, Trt resin, SASRIN resin, Rink amide resin, Merrifield resin, Wang resin, and 2-(4-bromomethylphenoxy)ethyl polystyrene as well as solid-phase carriers having an arbitrary functional group such as a carboxyl group, an amino

group, an aminomethyl group, a hydroxy group, or a hydroxymethyl group on polystyrene. The solid-phase carrier may be designed using an arbitrary linker that covalently links such a carrier and the first BB such that the cleavage between the linker and the first BB can be performed. Examples of the carrier can include, but are not particularly limited to, polystyrene as well as PEG (polyethylene glycol).

The conditions for the reaction between the first BB and the solid-phase carrier can be appropriately set by those skilled in the art on the basis of a method described in a literature known in the art or the like.

In the step (e), the reaction conditions for the cleavage of the compounds from the solid-phase carriers can be appropriately set by those skilled in the art on the basis of the chemical structures of the solid-phase carriers used. Examples of the reagent that can be used in cleavage include hydrochloric acid, carboxylic acid such as trifluoroacetic acid (TFA), fluoro alcohols such as 2,2,2-trifluoroethanol (TFE) and 1,1,1,3,3,3-hexafluoroisopropyl alcohol (HFIP), Bronsted acids, and arbitrary Lewis acids having pKa of less than 10 in water. In one embodiment, the compounds cleaved from the solid-phase carriers are obtained as a mixture.

One aspect of the present invention provides a method for producing a library, comprising (a), (b), and (e), wherein the second $BB_{1\text{-}nb}$-first $BB_{1\text{-}na}$-supporting carriers each have a reactive functional group capable of forming a linker with a third building block (third BB), and the reactive functional group that is singly contained in each of the first $BB_{1\text{-}na}$ moieties or the second $BB_{1\text{-}nb}$ moieties is of one type, two or more types or three or more types, the method further comprising (c) preparing mixtures of supporting carriers having the reactive functional group common among the second $BB_{1\text{-}nb}$-first $BB_{1\text{-}na}$-supporting carriers, and reacting each mixture with one type of third building block introduction reagent (third BB introduction reagent) or a mixture of two or more types of third BB introduction reagents to prepare supporting carriers in which the third BB is covalently linked to the first BB or the second BB (third BB-second BB-first BB-supporting carriers or second BB-(third BB-)first BB-supporting carriers).

In this step (c), the resulting supporting carriers are supporting carriers comprising nc types of third building blocks (third $BB_1$ to third $BB_{nc}$) (third $BB_{1\text{-}nc}$-second $BB_{1\text{-}nb}$-first $BB_{1\text{-}na}$-supporting carriers or second $BB_{1\text{-}nb}$-(third $BB_{1\text{-}nc}$-)first $BB_{1\text{-}na}$-supporting carriers), and any of the third $BB_1$ introduction reagent to the third $BB_{nc}$ introduction reagent reacts with the respective reactive functional groups of the second $BB_{1\text{-}nb}$-first $BB_{1\text{-}na}$-supporting carriers to form second linkers (second L). In this context, nc is an integer of 2 or larger, 3 or larger, 4 or larger, or 5 or larger, and the first linkers or the second linkers of the compounds contained in the library are of two or more types.

In the step (c), the second $BB_{1\text{-}nb}$-(third $BB_{1\text{-}nc}$-)first $BB_{1\text{-}na}$-supporting carriers have structures where third $BB_{1\text{-}nc}$ moieties are linked to first $BB_{1\text{-}na}$ moieties via second linkers. For example, a reactive functional group protected with a protective group exists in the first $BB_{1\text{-}na}$ moieties, and after introduction of the second $BB_{1\text{-}nb}$ moieties, the group is deprotected so that reactive functional groups of third $BB_{1\text{-}nc}$ introduction reagents and second linkers can be formed and introduced. In one embodiment, in (c), the resulting supporting carriers are third $BB_{1\text{-}nc}$-second $BB_{1\text{-}nb}$-first $BB_{1\text{-}na}$-supporting carriers, and the second $BB_{1\text{-}nb}$ moieties of the second $BB_{1\text{-}nb}$-first $BB_{1\text{-}na}$-supporting carriers have one reactive functional group for forming a bond with a third building block (third BB).

One aspect of the present invention provides a method for producing a library, comprising (a), (b), (c), and (e), wherein the third $BB_{1\text{-}nc}$-second $BB_{1\text{-}nb}$-first $BB_{1\text{-}na}$- supporting carriers or the second $BB_{1\text{-}nb}$-(third $BB_{1\text{-}nc}$-)first $BB_{1\text{-}na}$-supporting carriers each have a reactive functional group for forming a bond with a fourth building block (fourth BB), and the reactive functional group that is singly contained in each of the first $BB_{1\text{-}na}$ moieties, the second $BB_{1\text{-}nb}$ moieties, or the third $BB_{1\text{-}nc}$ moieties is of one type, two or more types or three or more types, the method further comprising (d) preparing mixtures of supporting carriers having the reactive functional group common among the third $BB_{1\text{-}nc}$-second $BB_{1\text{-}na}$- first $BB_{1\text{-}na}$-supporting carriers or the second $BB_{1\text{-}nb}$-(third $BB_{1\text{-}nc}$-)first $BB_{1\text{-}na}$-supporting carriers, and reacting each mixture with one type of fourth building block introduction reagent (fourth BB introduction reagent) or a mixture of two or more types of fourth BB introduction reagents to prepare supporting carriers in which the fourth BB is covalently linked to the third BB, the second BB, or the first BB.

In this step (e), the resulting supporting carriers are supporting carriers comprising nd types of fourth building blocks (fourth $BB_1$ to fourth $BB_{nd}$), and any of the fourth $BB_1$ introduction reagent to the fourth $BB_{nd}$ introduction reagent reacts with the respective reactive functional groups of the third $BB_{1\text{-}nc}$-second $BB_{1\text{-}nb}$-first $BB_{1\text{-}na}$-supporting carriers or the second $BB_{1\text{-}nb}$-(third $BB_{1\text{-}nc}$-)first $BB_{1\text{-}na}$-supporting carriers to form third linkers, thereby introducing the fourth BB. The reactive functional group that forms a third linker with the fourth BB introduction reagent may have a protective group. In this case, deprotection is performed before reaction with the fourth BB introduction reagent. In this context, nd is an integer of 2 or larger, 3 or larger, 4 or larger, or 5 or larger, and the first linkers, the second linkers or the third linkers of the compounds contained in the library are of two or more types.

In (d), the resulting supporting carriers are

fourth $BB_{1\text{-}na}$-third $BB_{1\text{-}nc}$-second $BB_{1\text{-}nb}$-first BBi-na-supporting carriers;
fourth $BB_{1\text{-}nd}$-(third $BB_{1\text{-}nc}$-)second $BB_{1\text{-}nb}$-first $BB_{1\text{-}na}$-supporting carriers;
third BBi-nc-second $BB_{1\text{-}nb}$-(fourth $BB_{1\text{-}nd}$-)first BBi-na-supporting carriers;
fourth $BB_{1\text{-}na}$-third $BB_{1\text{-}nc}$-(second $BB_{1\text{-}nb}$-)first $BB_{1\text{-}na}$-supporting carriers; or

fourth $BB_{1\text{-}na}$-second $BB_{1\text{-}nb}$-(third $BB_{1\text{-}nc}$-)first $BB_{1\text{-}na}$-supporting carriers.

In one embodiment, in (d), the resulting supporting carriers may be a mixture of supporting carriers represented by two or more formulas. In one embodiment, in (d), the resulting supporting carriers are fourth $BB_{1\text{-}na}$-third $BB_{1\text{-}nc}$-second $BB_{1\text{-}nb}$-first $BB_{1\text{-}na}$-supporting carriers.

In one aspect of the method for producing a library according to the present invention, the first linkers and the second linkers satisfy one or more of the following conditions:

1-1) the first linkers are of two or more types, three or more types, or four or more types;
1-2) the second linkers are of two or more types, three or more types, or four or more types; and
1-3) the first linkers or the second linkers are of three or more types, four or more types, or five or more times.

In another aspect, the first linkers, the second linkers and the third linkers satisfy one or more of the following conditions:

2-1) the first linkers are of two or more types, three or more types, or four or more types;
2-2) the second linkers are of two or more types, three or more types, or four or more types;
2-3) the third linkers are of two or more types, three or more types, or four or more types; and
2-4) the first linkers, the second linkers or the third linkers are of three or more types, four or more types, or five or more types.

In one embodiment of the present invention, na is an integer of 2 to 1000, nb is an integer of 2 to 1000, and any of na and nb is an integer of 3 or larger. In one embodiment of the present invention, na is an integer of 2 to 1000, nb is an integer of 2 to 1000, nc is an integer of 2 to 1000, and any of na, nb and nc is an integer of 3 or larger.

In one embodiment, the resulting library comprises 100 or more, 200 or more, 300 or more, 400 or more, 500 or more, or 1000 or more compounds.

In one aspect of the present invention, the number of compounds contained in the library obtained by such a library synthesis method can be, for example, 800 or less, 1000 or less, 1200 or less, 1500 or less, 1700 or less, 2000 or less, 2500 or less, 3000 or less, 4000 or less, 5000 or less, 6000 or less, 7000 or less, 8000 or less, 10000 or less, 20000 or less, 50000 or less, or 100000 or less as the upper limit and can be, for example, 100 or more, 200 or more, 300 or more, 400 or more, 500 or more, or 1000 or more as the lower limit.

In one aspect of the present invention, the library synthesis method may further comprise a deprotection and/or protection step for the reactive functional group. In one embodiment, after a deprotection step for the reactive functional group, the supporting carriers can be washed using a solution mixed with an acidic component to remove impurities. An acidic compound having pKa of 0 to 10 in water can be used as the acidic component. Examples of the acidic component can include 1-hydroxy-7-azabenzotriazole (HOAt), 1- hydroxybenzotriazole (HOBt), ethyl (hydroxyimino)cyanoacetate (oxyma) and 4-nitrophenol. A functional group that is formed by the deprotection step for the reactive functional group is not particularly limited as long as the group has suitable reactivity. The functional group may be, for example, carboxyl or a hydroxy-substituted aromatic ring.

In one aspect of the present invention, the library synthesis method may further comprise the step of linking two cross-linkable groups present in the molecule to obtain a cyclic compound. The cyclization step can be performed, for example, after cleavage of the compounds from the solid-phase carriers. Alternatively, this step may also be performed before cleavage on the solid-phase carriers (cyclization on resins). Examples of the two cross-linkable groups present in the molecule can include combinations of a carboxyl group and an amino group, combinations of a carboxyl group and a hydroxy group, combinations of an acetylene group and an azide group, combinations of alkenes substituted by an arbitrary substituent at an arbitrary position, and combinations of thiol groups. In one embodiment, the cyclization step can be performed with the resulting mixture of compounds as a substrate after cleavage of the compounds from the solid-phase carrier.

In one aspect of the present invention, the library synthesis method may further comprise modifying (or derivatizing) the functional groups contained in the compounds contained in the resulting library. In this context, examples of the functional groups to be modified can include, but are not particularly limited to, a hydroxy group, a carboxyl group, and an amino group. Examples of the functional groups obtained by modification can include ether, amide, ester, sulfonamide, and acylsulfonamide. The step required for modifying the functional groups may be a single step or may be a plurality of steps. In one embodiment, the functional groups are functional groups resulting from the cleavage of the compounds from the solid-phase carriers. Examples of the modification include the $C_{1\text{-}6}$ alkylation of hydroxy or carboxy.

The conversion rate of the reaction between the building block introduction reagent and the reactive functional group is preferably 100%, though some reaction may presumably not reach 100%. In one embodiment of the present invention, the step (b) further comprises reacting a remaining reactive functional group of the first BB in first BB-supporting carriers having less than 100% conversion rate of the reaction between the second BB introduction reagent and the

reactive functional group of the first BB, with a different second BB introduction reagent having higher reactivity. In another embodiment of the present invention, the step (c) further comprises reacting a remaining reactive functional group in second BB-first BB-supporting carriers having less than 100% conversion rate of the reaction between the third BB introduction reagent and the reactive functional group, with a different third BB introduction reagent having higher reactivity. In a further alternative embodiment of the present invention, the step (d) further comprises reacting a remaining reactive functional group in third BB-second BB-first BB-supporting carriers or second BB-(third BB-)first BB-supporting carriers having less than 100% conversion rate of the reaction between the fourth BB introduction reagent and the reactive functional group, with a different fourth BB introduction reagent having higher reactivity.

In one aspect of the present invention, one or more compounds, for example, all the compounds, of the library may comprise a UV tag or may comprise no UV tag. When one or more compounds, for example, all the compounds, of the library comprise a UV tag, for example, a group having a structure represented by formula 1:

[Formula 9]

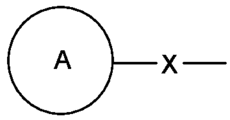

(1)

wherein ring A is selected from benzene, naphthalene, anthracene and biphenyl;
the benzene, the naphthalene, the anthracene and the biphenyl are each optionally substituted by 1 to 3 identical or different groups selected from the group consisting of a halogen atom, nitro, cyano, carboxy, sulfoxy, $-COR^1$, $-OR^1$, $-NR^1R^2$, $-CONR^1R^2$, and - $SO_2NR^1R^2$; $R^1$ and $R^2$ are each independently $C_{1-6}$ alkyl, or optionally form a hetero ring together with the nitrogen atom linked thereto; and X represents that one member selected from the group consisting of -CONH-, -NHCO-, -CHzO- and -O- is bonded to a building block
can be introduced as the UV tag to the compounds.

A group having a structure represented by formula 2 or formula 3:

[Formula 10]

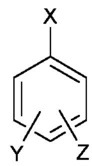

(2)

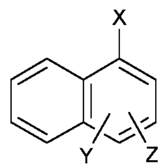

(3)

wherein X is a divalent group selected from the group consisting of -CONH-, -NHCO-, - CHzO- and -O-, and the group is bonded to a building block; Y and Z are each optionally substituted by 1 to 3 identical or different groups selected from the group consisting of a hydrogen atom, halogen, nitro, cyano, carboxy, sulfoxyl, $-COR^1$, $-OR^1$, $-NR^1R^2$, $-CONR^1R^2$, and $-SO_2NR^1R^2$; and $R^1$ and $R^2$ are each independently $C_{1-6}$ alkyl, or optionally together form a hetero ring
may be introduced as the UV tag to the compounds.

In one embodiment, a group having a structure represented by formula 3:

[Formula 11]

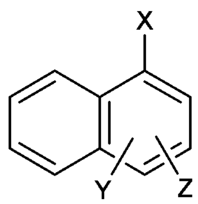

(3)

wherein X represents that one member selected from the group consisting of -CONH-, - NHCO-, -CHzO- and -O- is bonded to a building block; Y and Z are each optionally substituted by 1 to 3 identical or different groups selected from

the group consisting of a hydrogen atom, a halogen atom, nitro, cyano, carboxy, sulfoxyl, $-COR^1$, $-O-R^1$, $-NR^1R^2$, $-CO-NR^1R^2$, and $-SO_2-NR^1R^2$; and $R^1$ and $R^2$ are each independently $C_{1-6}$ alkyl, or optionally together form a hetero ring may be introduced as the UV tag to the compounds.

In one embodiment, a group having a structure represented by formula 4:

[Formula 12]

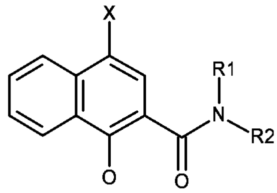

(4)

wherein X represents that one member selected from the group consisting of -CONH-, - NHCO-, -CHzO- and -O- is bonded to a building block; and $R^1$ and $R^2$ are each independently $C_{1-6}$ alkyl, or optionally form a hetero ring together with the nitrogen atom linked thereto
may be introduced as the UV tag to the compounds.

In one embodiment of the present invention, the compounds contained in the library comprise no UV tag or no oligonucleotide tag or comprise neither a UV tag nor an oligonucleotide tag.

In one aspect of the present invention, the compounds constituting the library comprise a UV tag and can be used in the analysis of intermediate compounds in the solid- phase synthesis of the compounds. In one embodiment, the method for producing a library further comprises the steps of:

1) obtaining supporting carriers obtained in any step;
2) cleaving intermediate compounds from the supporting carriers obtained in the step 1); and
3) analyzing the intermediate compounds; and

optionally comprises the step of analyzing the compounds cleaved in the step (e). Results obtained from the analysis of the intermediate compounds can be used in, for example, the optimization of reaction conditions for solid-phase reaction. This enables a high-quality library to be produced. In one embodiment, the analysis can be performed using a liquid chromatogram.

In one aspect of the present invention, the first linkers, the second linkers, the third linkers and the fourth linkers formed in the method for producing a library have, for example, a chemical structure formed through reaction independently selected from the group consisting of amidation, sulfonamidation, acylsulfonamidation, reductive amination, etherification, thioetherification, esterification, O-alkylation, N-alkylation, carbon-nitrogen bond formation reaction, carbon-carbon bond formation reaction, and nitrogen-containing 5- membered heteroarylene formation reaction. In one embodiment, the first linkers, the second linkers, the third linkers and the fourth linkers have a structure independently selected from the group consisting of amide, sulfonamide, acylsulfonamide, amino, ether, thioether, ester, ketone, sulfone, a single bond, ethyne-1,2-diyl (-C---C-), and nitrogen-containing 5-membered heteroarylene.

In one aspect of the present invention, the method for producing a library can be performed for the production of the library described in the present specification. In one embodiment, the first linkers, the second linkers, the third linkers and the fourth linkers formed in the method for producing a library correspond to the first linkers, the second linkers, the third linkers and the fourth linkers formed in the method for producing a library defined about the library described above. For example, products obtained by the cleavage of the compounds from the third BB-second BB-first BB-supporting carriers or the second BB- (third BB-)first BB-supporting carriers correspond to (first CB)-(first L)-(second CB)- (second L)-(third CB) or (third CB)-(second L)-(first CB)-(first L)-(second CB).

A method for forming the linker described above which links core blocks will be illustrated below. However, the present invention is not limited by the described formation method and structure. Each reagent (base, condensing agent, catalyst, ligand, etc.) may be used singly, or a plurality of reagents having the same function may be concurrently used in combination (e.g., DIPEA and potassium phosphate as a base). The equivalents of the reagents given below refer to how many times the molar number of the reagent used is based on the total molar number of functional groups for use in linker formation carried by each compound of a mixture on the solid-phase side. For example, the term "10 equivalents" means that the reagent is used at a molar number of 10 times the total molar number of functional groups for use in linker formation carried by each compound of a mixture on the solid-phase side. When reaction conditions are described about A containing a solid-phase carrier, the case where B contains a solid-phase carrier can also be performed under

similar reaction conditions (the type of a reagent, the type of a solvent, reaction temperature, reaction time, , etc.), unless otherwise specified. In this respect, the equivalents of a substrate on the liquid-phase side can be appropriately set by those skilled in the art and can be, for example, 1 to 100 equivalents, 1 to 75 equivalents, 1 to 50 equivalents, 1 to 30 equivalents, or 1 to 10 equivalents, based on a substrate on the solid-phase side.

Unless otherwise specified, the reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers.

A and B in the formulas given below each represent a core block to which a reactive functional group is linked. Each core block may be further linked to another core block via a linker. Either A or B contains a solid-phase carrier.

[Formula 13]

Amide bond formation reaction

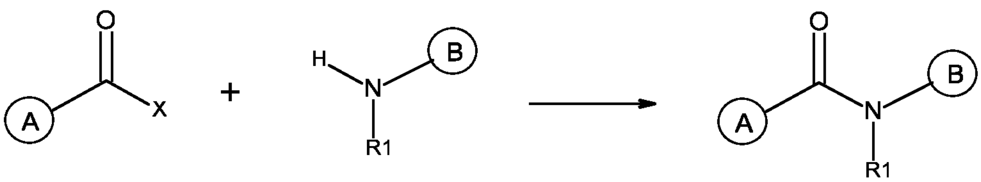

wherein R1 is a hydrogen atom or $C_{1-6}$ alkyl optionally having a substituent, and optionally forms a ring together with the nitrogen atom and B bonded thereto.

The amide bond as the linker can be formed through the reaction of carboxylic acid or a derivative thereof with amine.

When A in the formula contains a solid-phase carrier, the reactive functional group X linked to A is hydroxy or a halogen atom. When X is hydroxy, the amide bond as the linker can be formed through the reaction thereof with amine containing an amino group lined to B using a condensing agent. In this context, the condensing agent is not particularly limited as long as the condensing agent can be usually used. Examples of the condensing agent that can be used include N,N'-dicyclohexylcarbodiimide, N,N'-diisopropylcarbodiimide, 1-ethyl-3-(3-dimethylaminopropyl)carbodiimide hydrochloride, N,N'-carbonyldiimidazole, 1,1'-carbonyldi(1,2,4-triazole), 4-(4,6-dimethoxy-1,3,5-triazin-2-yl)-4-methylmorpholinium chloride, (benzotriazol-1-yloxy)tris(dimethylamino)phosphonium hexafluorophosphate, (benzotriazol-1-yloxy)tripyrrolidinophosphonium hexafluorophosphate, (7-azabenzotriazol- 1-yloxy)tripyrrolidinophosphonium hexafluorophosphate, bromotripyrrolidinophosphonium hexafluorophosphate, O-(7-azabenzotriazol-1-yl)-N,N,N',N'-tetramethyluronium hexafluorophosphate, O-(benzotriazol-1-yl)-N,N,N',N'-tetramethyluronium hexafluorophosphate, O-(7-azabenzotriazol-1-yl)-N,N,N',N'-tetramethyluronium tetrafluoroborate, (1-cyano-2-ethoxy-2-oxoethylideneaminooxy)dimethylamino-morpholino- carbenium hexafluorophosphate, [ethylcyano(hydroxyimino)acetato-$O^2$]tri-1- pyrrolidinylphosphonium hexafluorophosphate, imidazolium 2-chloro-4,5-dihydro-1,3- dimethyl-1H-hexafluorophosphate, chloro-N,N,N',N'-tetramethylformamidinium hexafluorophosphate, 1-(chloro-1-pyrrolidinylmethylene)pyrrolidinium hexafluorophosphate, isobutyl chlorocarbonate, triphosgene, diphenylphoshoryl azide, propylphosphonic anhydride, cyanuric acid chloride, 2-chloro-4,6-dimethoxytriazine, 4-(4,6-dimethoxy-1,3,5-triazin-2-yl)- 4-methylmorpholinium chloride, (4,6-dimethoxy-1,3,5-triazin-2-yl)-(2-octoxy-2- oxoethyl)dimethylammonium trifluoromethanesulfonate, 2-methyl-6-nitrobenzoic anhydride, 2-chloro-1-methylpyridinium iodide, 2-fluoro-1-methylpyridinium p-toluenesulfonate, N- ethynyl-N,4-dimethylbenzenesulfonylformamide, and 1-chloro-N,N,2-trimethyl-1- propenylamine. The number of equivalents of the condensing agent used can be appropriately set by those skilled in the art and can be, for example, 1 to 100 equivalents, 1 to 75 equivalents, 1 to 50 equivalents, 1 to 30 equivalents, or 1 to 10 equivalents, based on the carboxylic acid containing A. The number of equivalents of the amine used can be appropriately set by those skilled in the art and can be, for example, 1 to 100 equivalents, 1 to 75 equivalents, 1 to 50 equivalents, 1 to 30 equivalents, or 1 to 10 equivalents, based on the carboxylic acid containing A.

R1 in the formula is as already defined. Preferred examples of R1 include a hydrogen atom, $C_{1-6}$ alkyl optionally having a substituent, for example, methyl and ethyl, and aromatic rings optionally having a substituent, for example, benzene and ethylpyridine.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL ($\gamma$-valerolactone), pentyl acetate, methyl propionate, etc.), aromatic hydrocarbon solvents (anisole, toluene, xylene, chlorobenzene, bromobenzene, ethylbenzene, nitrobenzene, $\alpha,\alpha,\alpha$- trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. A single solvent may be used, or a combined solvent of a plurality of solvents may be used. The reaction temperature can be appropriately set by those skilled in the art and can be, for example, -20 to 200°C, 0 to 150°C, 0 to 100°C, 0 to 80°C, or 10 to 80°C. If necessary, a base may be used. Examples of the base that can be used include pyridine, 2,6- lutidine, 2,4,6-collidine, DTBP, DTBMP, triethylamine, DIPEA, 4-methylmorpholine, NMI, DMAP, potassium hydride, sodium hydride, lithium hydride, sodium amide, lithium amide, KOH, NaOH, LiOH, sodium carbonate, potassium carbonate, cesium carbonate, sodium bicarbonate, sodium phosphate, potassium phosphate, $K_2HPO_4$, $KHCO_3$, $Na_2HPO_4$, $NaHCO_3$, sodium tetraborate, DBU, DBN, MTBD, BTMG, TMG, 1,8-bis(dimethylamino)naphthalene, LiOtBu, NaOtBu, KOtBu, potassium t-pentoxide, sodium t-pentoxide, lithium t-pentoxide, LiHMDS, NaHMDS, KHMDS, HMDS, lithium diisopropylamide, lithium dicyclohexylamide, 2,2,6,6-tetramethylpiperidinyl lithium, 2,2,6,6-tetramethylpiperidinyl magnesium chloride-lithium chloride complexes, N,N-dimethylaniline, P1-tBu, P2tBu, tetrabutylammonium hydroxide, benzyl triethylammonium hydroxide, butyllithium, methyllithium, tetrabutylammonium fluoride, and cesium fluoride. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the carboxylic acid containing A. The reaction time can be appropriately set by those skilled in the art and can be a reaction time required for a model experiment, or the point in time of the completion of the reaction can be determined or confirmed by cleaving products from some solid-phase carriers. After the completion of the reaction, the target compound can be obtained by a usual aftertreatment method.

When X is a halogen atom, specific examples thereof include a chlorine atom and a fluorine atom. The reaction of acid halide with amine may be performed in the presence of a base or may be performed using 2 equivalents or more of the amine. In this context, examples of the base that may be used can include pyridine, 2,6-lutidine, 2,4,6-collidine, DTBP, DTBMP, triethylamine, DIPEA, 4-methylmorpholine, NMI, DMAP, potassium hydride, sodium hydride, lithium hydride, sodium amide, lithium amide, KOH, NaOH, LiOH, sodium carbonate, potassium carbonate, cesium carbonate, sodium bicarbonate, sodium phosphate, potassium phosphate, $K_2HPO_4$, $KHCO_3$, $Na_2HPO_4$, $NaHCO_3$, sodium tetraborate, DBU, DBN, MTBD, BTMG, TMG, 1,8-bis(dimethylamino)naphthalene, LiOtBu, NaOtBu, KOtBu, potassium t-pentoxide, sodium t-pentoxide, lithium t-pentoxide, LiHMDS, NaHMDS, KHMDS, HMDS, lithium diisopropylamide, lithium dicyclohexylamide, 2,2,6,6- tetramethylpiperidinyl lithium, 2,2,6,6-tetramethylpiperidinyl magnesium chloride-lithium chloride complexes, N,N-dimethylaniline, P1-tBu, P2tBu, tetrabutylammonium hydroxide, benzyl triethylammonium hydroxide, butyllithium, methyllithium, tetrabutylammonium fluoride, and cesium fluoride. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the acid halide containing A. The number of equivalents of the amine used can be appropriately set by those skilled in the art and can be, for example, 1 to 100 equivalents, 1 to 75 equivalents, 1 to 50 equivalents, 1 to 30 equivalents, or 1 to 10 equivalents, based on the acid halide containing A.

R1 in the formula is as already defined. Preferred examples of R1 include a hydrogen atom and $C_{1-6}$ alkyl, for example, methyl and ethyl.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate,

ethyl acetate, propyl acetate, isopropyl acetate, butyl acetate, isobutyl acetate, GVL (γ-valerolactone), pentyl acetate, methyl propionate, etc.), aromatic hydrocarbon solvents (anisole, toluene, xylene, chlorobenzene, bromobenzene, ethylbenzene, nitrobenzene, α,α,α- trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. The reaction temperature can be appropriately set by those skilled in the art and can be, for example, -80 to 150°C, -80 to 100°C, -80 to 80°C, -40 to 80°C, or 0 to 80°C.

The acid halide for use in the reaction may be prepared from corresponding carboxylic acid in the reaction system. In this respect, the reagent is not particularly limited as long as the reagent can be usually used. Examples of the reagent that can be used include thionyl chloride, oxalyl chloride, phosphorus trichloride, phosphorus pentachloride, phosphoryl chloride, sulfuryl chloride, (chloromethylene)dimethyliminium chloride (Vilsmeier reagent), 1-chloro-N,N,2-trimethyl-1-propenylamine (Ghosez reagent), chloro- N,N,N',N'-bis(tetramethylene)formamidinium tetrafluoroborate, PyClU, PipClU, chloro- N,N,N',N'-tetramethylformamidinium hexafluorophosphate, fluoro-N,N,N',N'- tetramethylformamidinium hexafluorophosphate (TFFH), and fluoro-N,N,N',N'- bis(tetramethylene)formamidinium hexafluorophosphate (BTFFH). In the case of using thionyl chloride, oxalyl chloride, phosphorus trichloride, phosphorus pentachloride, phosphoryl chloride, sulfuryl chloride, or the like, an amide solvent such as DMF may be allowed to coexist therewith. If necessary, a base may be used. Examples of the base that can be used include pyridine, 2,6-lutidine, 2,4,6-collidine, DTBP, DTBMP, triethylamine, DIPEA, 4-methylmorpholine, NMI, DMAP, potassium hydride, sodium hydride, lithium hydride, sodium amide, lithium amide, KOH, NaOH, LiOH, sodium carbonate, potassium carbonate, cesium carbonate, sodium bicarbonate, sodium phosphate, potassium phosphate, $K_2HPO_4$, $KHCO_3$, $Na_2HPO_4$, $NaHCO_3$, sodium tetraborate, DBU, DBN, MTBD, BTMG, TMG, 1,8-bis(dimethylamino)naphthalene, LiOtBu, NaOtBu, KOtBu, potassium t-pentoxide, sodium t-pentoxide, lithium t-pentoxide, LiHMDS, NaHMDS, KHMDS, HMDS, lithium diisopropylamide, lithium dicyclohexylamide, 2,2,6,6-tetramethylpiperidinyl lithium, 2,2,6,6- tetramethylpiperidinyl magnesium chloride-lithium chloride complexes, N,N-dimethylaniline, P1-tBu, P2tBu, tetrabutylammonium hydroxide, benzyl triethylammonium hydroxide, butyllithium, methyllithium, tetrabutylammonium fluoride, and cesium fluoride. A solvent can be used in the reaction. Examples of the solvent that can be used include, but are not particularly limited to, halogen solvents (DCM (dichloromethane), chloroform, DCE (1,2- dichloroethane), carbon tetrachloride, etc.), ether solvents (diethyl ether, diisopropyl ether, THF (tetrahydrofuran), 2-methyltetrahydrofuran, 4-methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'- tetramethylurea), DMI (1,3-dimethyl-2-imidazolidinone), DMPU (N,N'- dimethylpropyleneurea), etc.), amide solvents (DMF (N,N-dimethylformamide), DMA (N,N- dimethylacetamide), NFM (N-formylmorpholine), NMP (N-methyl-2-pyrrolidone), NEP (N- ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3-methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL (γ-valerolactone), pentyl acetate, methyl propionate, etc.), aromatic hydrocarbon solvents (anisole, toluene, xylene, chlorobenzene, bromobenzene, ethylbenzene, nitrobenzene, α,α,α-trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. The reaction temperature can be appropriately set by those skilled in the art and can be, for example, -40 to 150°C, -40 to 100°C, -40 to 80°C, -20 to 80°C, or 0 to 80°C. The number of equivalents of the reagent or the base used can be appropriately set by those skilled in the art and can be, for example, 1 to 100 equivalents, 1 to 75 equivalents, 1 to 50 equivalents, 1 to 30 equivalents, 1 to 20 equivalents, 1 to 15 equivalents, 1 to 10 equivalents, or 1 to 5 equivalents, based on the carboxylic acid containing A. The number of equivalents of the amine used can be appropriately set by those skilled in the art and can be, for example, 1 to 100 equivalents, 1 to 75 equivalents, 1 to 50 equivalents, 1 to 30 equivalents, 1 to 20 equivalents, 1 to 15 equivalents, 1 to 10 equivalents, or 1 to 5 equivalents, based on the carboxylic acid containing A. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier, the reactive functional group X linked to A is hydroxy or a halogen atom. When X is hydroxy, the amide bond as the linker can be formed through the reaction thereof with amine containing an amino group lined to B using a condensing agent. In this context, the reagent used and other reaction conditions are the same as those when A contains a solid-phase carrier. In this context, in the case of using a condensing agent, the number of equivalents thereof can be appropriately set by those skilled in the art and can be, for example, 1

to 100 equivalents, 1 to 75 equivalents, 1 to 50 equivalents, 1 to 30 equivalents, or 1 to 10 equivalents, based on the amine containing B. In the case of using a condensing agent, the number of equivalents of the carboxylic acid used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing B. When A is acid halide, the number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing B. The number of equivalents of the acid halide used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing B. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

[Formula 14]

Sulfonamide bond formation reaction

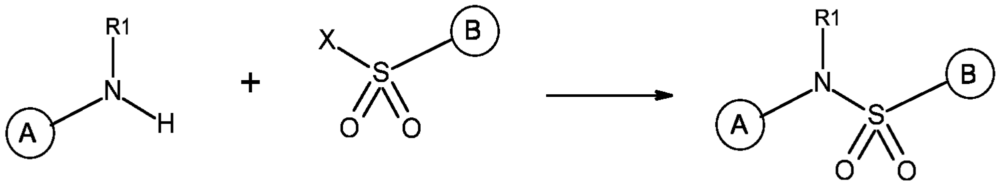

wherein R1 is a hydrogen atom or $C_{1-6}$ alkyl optionally having a substituent, and optionally forms a ring together with the nitrogen atom and A bonded thereto.

The sulfonamide bond as the linker can be formed through the reaction of amine with sulfonyl halide or a derivative thereof.

When A in the formula contains a solid-phase carrier, the reactive functional group X linked to B is a halogen atom, for example, a chlorine atom or a fluorine atom. The sulfonamide bond as the linker can be formed through the reaction thereof with amine containing an amino group linked to A using a base. In this context, the base is not particularly limited as long as the base can be usually used. Examples of the base that can be used include pyridine, 2,6-lutidine, 2,4,6-collidine, DTBP, DTBMP, triethylamine, DIPEA, 4-methylmorpholine, NMI, DMAP, potassium hydride, sodium hydride, lithium hydride, sodium amide, lithium amide, KOH, NaOH, LiOH, sodium carbonate, potassium carbonate, cesium carbonate, sodium bicarbonate, potassium phosphate, sodium phosphate, $K_2HPO_4$, $KHCO_3$, $Na_2HPO_4$, sodium tetraborate, DBU, DBN, MTBD, BTMG, TMG, 1,8- bis(dimethylamino)naphthalene, LiOtBu, NaOtBu, KOtBu, potassium t-pentoxide, sodium t- pentoxide, LiHMDS, NaHMDS, KHMDS, HMDS, lithium diisopropylamide, lithium dicyclohexylamide, 2,2,6,6-tetramethylpiperidinyl lithium, 2,2,6,6-tetramethylpiperidinyl magnesium chloride-lithium chloride complexes, N,N-dimethylaniline, P1-tBu, P2tBu, tetrabutylammonium hydroxide, benzyl triethylammonium hydroxide, butyllithium, tetrabutylammonium fluoride, cesium fluoride, sodium phosphate, lithium t-pentoxide, methyllithium, and NaBHT.

The number of equivalents of the sulfonyl halide used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing A. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing A.

A solvent can be used in the reaction. Examples of the solvent that can be used include, but are not particularly limited to, halogen solvents such as DCM, chloroform, DCE, and carbon tetrachloride, ether solvents such as diethyl ether, diisopropyl ether, THF (tetrahydrofuran), 2-methyltetrahydrofuran, 4-methyltetrahydropyran, 1,4-dioxane, DME, TBME, CPME, diisopropyl ether, isosorbide dimethyl ether, diglyme, triglyme, and tetraglyme, ketone solvents such as acetone, MEK, MIBK, 4-methyl-2-pentanone, cyclopentanone, cyclohexanone, and diethyl ketone, urea solvents such as TMU, DMI, and DMPU, amide solvents such as DMF, DMA, NFM, NMP, NEP, and NBP, sulfoxide solvents such as DMSO, sulfone solvents such as diphenylsulfone, dimethylsulfone, diethylsulfone, sulfolane, 3-methylsulfolane, ethylmethylsulfone, and ethylisopropylsulfone, ester solvents such as methyl acetate, ethyl acetate, propyl acetate, isopropyl acetate, butyl acetate, isobutyl acetate, GVL ($\gamma$-valerolactone), pentyl acetate, and methyl propionate, aromatic solvents such as anisole, toluene, xylene, chlorobenzene, bromobenzene, ethylbenzene, nitrobenzene, $\alpha,\alpha,\alpha$-trifluorotoluene,

1,2-dichlorobenzene, benzene, and pyridine, carbonate solvents such as dimethyl carbonate, diethyl carbonate, ethylene carbonate, and propylene carbonate, and others such as acetonitrile and limonene. A single solvent may be used, or a combined solvent of a plurality of solvents may be used.

The reaction temperature can be appropriately set by those skilled in the art and can be, for example, -20 to 100°C, 0 to 60°C, or 0 to 30°C. If necessary, an additive may be used. Examples of the additive that can be used include NMI, DMAP, HOAt, oxyma, pentafluorophenol, pyridine N-oxide, and NaI. The number of equivalents of the additive used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing A. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier, the reactive functional group X linked to B is a halogen atom. X may be prepared from hydroxy. In this respect, the reagent is not particularly limited as long as the reagent can be usually used. Examples of the reagent that can be used include phosphorus oxychloride, phosphorus trichloride, thionyl chloride, and phosgene. An amide solvent such as DMF may be allowed to coexist therewith. In this context, the reagent used and other reaction conditions are the same as those when A contains a solid-phase carrier. For example, when A is amine, the number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the sulfonic acid halide containing B. The number of equivalents of the amine used can be appropriately set by those skilled in the art and can be, for example, 1 to 100 equivalents, 1 to 75 equivalents, 1 to 50 equivalents, 1 to 30 equivalents, or 1 to 10 equivalents, based on the sulfonic acid halide containing B. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

[Formula 15]

Acylsulfonamide bond formation reaction

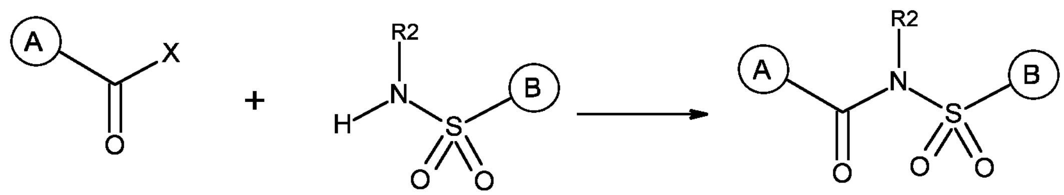

wherein R2 is a hydrogen atom or $C_{1-6}$ alkyl optionally having a substituent, and optionally forms a ring together with the nitrogen atom, the sulfur atom and B bonded thereto.

The acylsulfonamide bond as the linker can be formed through the reaction of carboxylic acid or a derivative thereof with sulfonamide.

When A in the formula contains a solid-phase carrier, the reactive functional group X linked to A is hydroxy. The acylsulfonamide as the linker can be formed through the reaction thereof with amine containing an amino group linked to B using a condensing agent. In this context, the condensing agent is not particularly limited as long as the condensing agent can be usually used. Examples of the condensing agent that can be used include combinations of carbodiimide condensing agents such as DCC (N,N'- dicyclohexylcarbodiimide), DIC (N,N'-diisopropylcarbodiimide), and EDCI (1-ethyl-3-(3- dimethylaminopropyl)carbodiimide hydrochloride) and activators such as HOAt (1-hydroxy- 7-azabenzotriazole), HOBt (1-hydroxybenzotriazole), HOOBt (3,4-dihydro-3-hydroxy-4- oxo-1,2,3-benzotriazine), and oxyma (ethyl cyano(hydroxyimino)acetate), uronium salt condensing agents such as HATU (O-(7-aza-1H-benzotriazol-1-yl)-N,N,N',N'- tetramethyluronium hexafluorophosphate), HBTU (O-(1H-benzotriazol-1-yl)-N,N,N',N'- tetramethyluronium hexafluorophosphate), HCTU (O-(6-chloro-1H-benzotriazol-1-yl)- N,N,N',N'-tetramethyluronium hexafluorophosphate), TATU (O-(7-azabenzotriazol-1-yl)- N,N,N',N'-tetramethyluronium tetrafluoroborate), TBTU (N,N,N',N'-tetramethyl-O- (benzotriazol-1-yl)uronium tetrafluoroborate), and COMU ((1-cyano-2-ethoxy-2- oxoethylideneaminooxy)dimethylaminomorpholinocarbenium hexafluorophosphate), phosphonium salt condensing agents such as BOP (benzotriazol-1- yloxy)tris(dimethylamino)phosphonium hexafluorophosphate, PyAOP ((7-azabenzotriazol-1- yloxy)trispyrrolidinophosphonium hexafluorophosphate), PyBOP (1H-benzotriazol-1-yloxy- tri(pyrrolidino)phosphonium hexafluorophosphate), PyBrOP (bromotripyrrolidinophosphonium hexafluorophosphate), and PyOxim ([ethylcyano(hydroxyimino)acetato-$O^2$]tri-1-pyrrolidinylphosphonium hexafluorophosphate), formamidinium salt condensing agents such as 1-chloro-N,N-2-trimethyl-1-propenylamine (Ghosez reagent), TCFH (chloro-N,N,N',N'-tetramethylformamidinium hexafluorophosphate), PyClU (N,N,N',N'-bis(tetramethylene)chloroformamidinium hexafluorophosphate), BTFFH (fluoro-N,N,N',N'-bis(tetramethylene)formamidinium hexafluorophosphate), TFFH (fluoro- N,N,N',N'-tetramethylamidinium hexafluorophosphate), TCFH (chloro-N,N,N',N'- tetramethylformamidinium hexafluorophosphate), PyClU (1-(chloro-1- pyrrolidinylmethylene)pyrrolidinium hexafluorophosphate), and PipClU (chlorodipiperidinocarbenium hexafluorophosphate), and other condensing agents such as CDI (N,N'-carbonyldiimidazole), CDT (1,1'-carbonyldi(1,2,4-triazole)), DMT-MM (4-(4,6- dimethoxy-1,3,5-triazin-2-yl)-4-methylmorpholinium chloride), CIP (imidazolium 2-chloro- 4,5-dihydro-1,3-dimethyl-1H-hexafluorophosphate), isobutyl chlorocarbonate, triphosgene, diphenylphoshoryl azide, propylphosphonic anhydride, cyanuric acid chloride, 2-chloro-4,6- dimethoxytriazine, 4-(4,6-dimethoxy-1,3,5-triazin-2-yl)-4-methylmorpholinium chloride, (4,6-dimethoxy-1,3,5-triazin-2-yl)-(2-octoxy-2-oxoethyl)dimethylammonium trifluoromethanesulfonate, 2-methyl-6-nitrobenzoic anhydride, 2-chloro-1-methylpyridinium iodide, 2-fluoro-1-methylpyridinium p-toluenesulfonate, N-ethynyl-N,4- dimethylbenzenesulfonylformamide, and 1-chloro-N,N,2-trimethyl-1-propenylamine.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL ($\gamma$-valerolactone), pentyl acetate, methyl propionate, etc.), aromatic hydrocarbon solvents (anisole, toluene, xylene, chlorobenzene, bromobenzene, ethylbenzene, nitrobenzene, $\alpha,\alpha,\alpha$- trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. A single solvent may be used, or a combined solvent of a plurality of solvents may be used. The reaction temperature can be appropriately set by those skilled in the art and can be, for example, 0 to 25°C, 0 to 50°C, 0 to 80°C, -20 to 80°C, -20 to 140°C, or 0 to 140°C. If necessary, a base may be used. Examples of the base that can be used include pyridine, 2,6-lutidine, 2,4,6-collidine, DTBP, DTBMP, triethylamine, DIPEA, 4- methylmorpholine, NMI, DMAP, potassium hydride, sodium hydride, lithium hydride, sodium amide, lithium amide, KOH, NaOH, LiOH, sodium carbonate, potassium carbonate, cesium carbonate, sodium bicarbonate, potassium phosphate, $K_2HPO_4$, $KHCO_3$, $NaHCO_3$, $Na_2HPO_4$, sodium tetraborate, DBU, DBN, MTBD, BTMG, TMG, 1,8- bis(dimethylamino)naphthalene, LiOtBu, NaOtBu, KOtBu, potassium t-pentoxide, sodium t-pentoxide, LiHMDS, NaHMDS, KHMDS, HMDS, lithium diisopropylamide, lithium dicyclohexylamide, 2,2,6,6-tetramethylpiperidinyl lithium, 2,2,6,6-tetramethylpiperidinyl magnesium chloride-lithium chloride complexes, N,N-dimethylaniline, P1-tBu, P2tBu, tetrabutylammonium hydroxide, benzyl triethylammonium hydroxide, hexamethyldisilazide, potassium hexamethyldisilazide, butyllithium, tetrabutylammonium fluoride, cesium fluoride, sodium phosphate, lithium t-pentoxide, and methyllithium. These bases can be used in combination.

The number of equivalents of the condensing agent used can be appropriately set by those skilled in the art and can be, for example, 1 to 100 equivalents, 1 to 75 equivalents, 1 to 50 equivalents, 1 to 30 equivalents, or 1 to 10 equivalents, based on the carboxylic acid containing A. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the carboxylic acid containing A. The number of equivalents of the sulfonamide containing B used can be appropriately set by those skilled in the art and can be, for example, 1 to 100 equivalents, 1 to 75 equivalents, 1 to 50 equivalents, 1 to 30 equivalents, or 1 to 10 equivalents, based on the carboxylic acid containing A.

R2 in the formula is as already defined. Preferred examples of R2 include a hydrogen atom and $C_{1-6}$ alkyl, for example, methyl and ethyl.

X may be halide and may be prepared from corresponding carboxylic acid (X = hydroxy) in the reaction system. In this respect, the reagent is not particularly limited as long as the reagent can be usually used. Examples of the reagent that can be used include thionyl chloride, oxalyl chloride, phosphorus trichloride, phosphorus pentachloride, phosphoryl chloride, sulfuryl chloride, (chloromethylene)dimethyliminium chloride (Vilsmeier reagent), 1-chloro-N,N,2-trimethyl-1-propenylamine (Ghosez reagent), chloro-N,N,N',N'- bis(tetramethylene)formamidinium tetrafluoroborate, PyClU, PipClU, chloro-N,N,N',N'- tetramethylformamidinium hexafluorophosphate, fluoro-N,N,N',N'- tetramethylformamidinium hexafluorophosphate (TFFH), and fluoro-N,N,N',N'- bis(tetramethylene)formamidinium hexafluorophosphate (BTFFH). In the case of using thionyl chloride, oxalyl chloride, phosphorus trichloride, phosphorus pentachloride, phosphoryl chloride, sulfuryl chloride, or the like, an amide solvent such as DMF may be allowed to coexist therewith. If necessary, a base may be used. Examples of the base that can be used include pyridine, 2,6-lutidine, 2,4,6-collidine, DTBP, DTBMP, triethylamine, DIPEA, 4-methylmorpholine, NMI, DMAP, potassium hydride, sodium hydride, lithium hydride, sodium amide, lithium amide, KOH, NaOH, LiOH, sodium carbonate, potassium carbonate, cesium carbonate, sodium bicarbonate, sodium phosphate, potassium phosphate, $K_2HPO_4$, $KHCO_3$, $Na_2HPO_4$, $NaHCO_3$, sodium tetraborate, DBU, DBN, MTBD, BTMG, TMG, 1,8-bis(dimethylamino)naphthalene, LiOtBu, NaOtBu, KOtBu, potassium t-pentoxide, sodium t-pentoxide, lithium t-pentoxide, LiHMDS, NaHMDS, KHMDS, HMDS, lithium diisopropylamide, lithium dicyclohexylamide, 2,2,6,6-tetramethylpiperidinyl lithium, 2,2,6,6- tetramethylpiperidinyl magnesium chloride-lithium chloride complexes, N,N-dimethylaniline, P1-tBu, P2tBu, tetrabutylammonium hydroxide, benzyl triethylammonium hydroxide, butyllithium, methyllithium, tetrabutylammonium fluoride, and cesium fluoride. A solvent can be used in the reaction. Examples of the solvent that can be used include, but are not particularly limited to, halogen solvents (DCM (dichloromethane), chloroform, DCE (1,2- dichloroethane), carbon tetrachloride, etc.), ether solvents (diethyl ether, diisopropyl ether, THF (tetrahydrofuran), 2-methyltetrahydrofuran, 4-methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'- tetramethylurea), DMI (1,3-dimethyl-2-imidazolidinone), DMPU (N,N'- dimethylpropyleneurea), etc.), amide solvents (DMF (N,N-dimethylformamide), DMA (N,N- dimethylacetamide), NFM (N-formylmorpholine), NMP (N-methyl-2-pyrrolidone), NEP (N- ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3-methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL ($\gamma$-valerolactone), pentyl acetate, methyl propionate, etc.), aromatic hydrocarbon solvents (anisole, toluene, xylene, chlorobenzene, bromobenzene, ethylbenzene, nitrobenzene, $\alpha,\alpha,\alpha$-trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. The reaction temperature can be appropriately set by those skilled in the art and can be, for example, -40 to 150°C, -40 to 100°C, -40 to 80°C, -20 to 80°C, or 0 to 80°C. The number of equivalents of the reagent or the base used can be appropriately set by those skilled in the art and can be, for example, 1 to 100 equivalents, 1 to 75 equivalents, 1 to 50 equivalents, 1 to 30 equivalents, 1 to 20 equivalents, 1 to 15 equivalents, 1 to 10 equivalents, or 1 to 5 equivalents, based on the carboxylic acid containing A. The number of equivalents of the sulfonamide containing B used can be appropriately set by those skilled in the art and can be, for example, 1 to 100 equivalents, 1 to 75 equivalents, 1 to 50 equivalents, 1 to 30 equivalents, 1 to 20 equivalents, 1 to 15 equivalents, 1 to 10 equivalents, or 1 to 5 equivalents, based on the carboxylic acid containing A. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier, the reactive functional group X linked to A is a halogen atom (specifically a chlorine atom or a fluorine atom) or hydroxy. When X is hydroxy, the acylsulfonamide as the linker can be formed through the reaction thereof with sulfonamide containing an amino group linked to B using a condensing agent. In this context, the acylsulfonamide formation reaction can be performed using the already described reagent and reaction conditions. In this context, in the case of using a base, the number of equivalents thereof can be appropriately set by those skilled in the art and can be, for example, 1 to 100 equivalents, 1 to 75 equivalents, 1 to 50 equivalents, 1 to 30 equivalents, or 1 to 10 equivalents, based on the sulfonamide containing B. The number of equivalents of the condensing agent used can be appropriately set by those skilled in the art and can be, for example, 1 to 100 equivalents, 1 to 75 equivalents, 1 to 50 equivalents, 1 to 30 equivalents, or 1 to 10 equivalents, based on the sulfonamide containing B. The number of equivalents of the carboxylic acid or the acid halide containing A used can be appropriately set by those skilled in the art and can be, for example, 1 to 100 equivalents, 1 to 75 equivalents, 1 to 50 equivalents, 1 to 30 equivalents, or 1 to 10 equivalents, based on the sulfonamide containing B. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound

can be obtained by a usual aftertreatment method.

[Formula 16]

Carbon-nitrogen single bond formation reaction

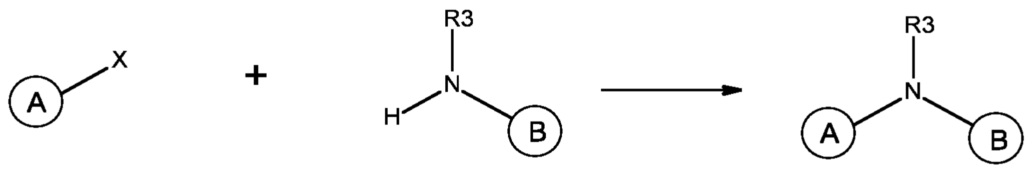

wherein R3 is a hydrogen atom or $C_{1-6}$ alkyl optionally having a substituent, and optionally forms a ring together with the nitrogen atom and B bonded thereto.

The amine bond as the linker can be formed through aromatic nucleophilic substitution reaction using aryl halide or a derivative thereof and amine, catalytic coupling reaction using a metal catalyst, or nucleophilic substitution reaction using alkyl halide, hydroxyalkyl or a derivative thereof and amine. The linker represented by -NR3- can be formed through the reaction described in the formula. Further, the linker may be formed through the reaction of primary amide or secondary amide with aryl halide or a derivative thereof, nucleophilic substitution reaction using primary amide or secondary amide and alkyl halide or a hydroxyalkyl derivative, the reaction of primary sulfonamide or secondary sulfonamide with aryl halide or a derivative thereof, or nucleophilic substitution reaction using primary sulfonamide or secondary sulfonamide and alkyl halide or a hydroxyalkyl derivative.

When A in the formula contains a solid-phase carrier and when A-X is aryl halide or a derivative thereof, the reactive functional group X linked to A is halogen or a group derived from a hydroxy group. Examples of the group derived from a hydroxy group can include a methanesulfonyl group, a trifluoromethanesulfonyl group, a nonafluorobutanesulfonyl group, a benzenesulfonyl group, a para-toluenesulfonyl group, a 2-nitrobenzenesulfonyl group, a 4- nitrobenzenesulfonyl group, and a 2,4-dinitrobenzenesulfonyl group. A-X can be subjected to reaction with amine linked to B in the presence of a base. In this context, the base is not particularly limited as long as the base can be usually used. Examples of the base that can be used include sodium carbonate, potassium carbonate, cesium carbonate, sodium bicarbonate, potassium bicarbonate, sodium phosphate, potassium phosphate, disodium hydrogen phosphate, dipotassium hydrogen phosphate, lithium acetate, sodium acetate, potassium acetate, cesium acetate, lithium hydroxide, sodium hydroxide, potassium hydroxide, cesium hydroxide, barium hydroxide, tetraalkylammonium hydroxide (tetramethylammonium hydroxide, tetrabutylammonium hydroxide, benzyl triethylammonium hydroxide, , etc.), cesium fluoride, potassium fluoride, LiOtBu, NaOtBu, KOtBu, potassium t-pentoxide, sodium t-pentoxide, lithium t-pentoxide, sodium hydride, potassium hydride, lithium hydride, sodium amide, lithium amide, lithium bis(trimethylsilyl)amide, sodium bis(trimethylsilyl)amide, potassium bis(trimethylsilyl)amide, hexamethyldisilazane, lithium diisopropylamide, lithium dicyclohexylamide, 2,2,6,6-tetramethylpiperidinyl lithium, 2,2,6,6- tetramethylpiperidinyl magnesium chloride-lithium chloride complexes, methyllithium, butyllithium, pyridine, alkyl-substituted pyridine (2,6-lutidine, 2,4,6-collidine, DTBP, DTBMP, etc.), DMAP, N,N-dimethylaniline, N-methylmorpholine, triethylamine, DIPEA, DBU, DBN, MTBD, TMG, BTMG, P1tBu, BEMP, BTPP, P2Et, P2tBu, NMI, sodium tetraborate, 1,8-bis(dimethylamino)naphthalene, N,N-dimethylaniline, and tetrabutylammonium fluoride. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The number of equivalents of the amine containing B used can be appropriately set by those skilled in the art and can be, for example, 1 to 100 equivalents, 1 to 75 equivalents, 1 to 50 equivalents, 1 to 30 equivalents, or 1 to 10 equivalents, based on A-X.

R3 in the formula is as already defined. Preferred examples of R3 include a hydrogen atom, $C_{1-6}$ alkyl optionally having a substituent, for example, methyl and ethyl, and aromatic rings optionally having a substituent, for example, benzene and pyridine. Examples of the ring structure that is formed together with the nitrogen atom and B bonded include pyrrolidine, piperidine, piperazine, and morpholine. Examples of the heterocyclic aromatic amine include pyrrole, imidazole, pyrazole, indole, and benzimidazole.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents

(TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL ($\gamma$-valerolactone), pentyl acetate, methyl propionate, etc.), aromatic hydrocarbon solvents (anisole, toluene, xylene, chlorobenzene, bromobenzene, ethylbenzene, nitrobenzene, $\alpha,\alpha,\alpha$- trifluorotoluene, 1,2-dichlorobenzene, benzene, tetralin, pyridine, quinoline, etc.), alcohol solvents (ethanol, isopropanol, tert-butanol, tert-amyl alcohol, etc.), hydrocarbon solvents (hexane, pentane, heptane, cyclohexane, methylcyclohexane, etc.), and others such as acetonitrile, water, dimethylacetal, dimethyl sulfate, silene, and limonene. A single solvent may be used, or a combined solvent of a plurality of solvents may be used. The reaction temperature can be appropriately set by those skilled in the art and can be, for example, 0 to 150°C, 10 to 120°C, 20 to 100°C, 20 to 80°C, or 20 to 60°C. If necessary, a metal catalyst may be used. Examples of the metal catalyst that can be used include palladium, for example, palladium acetate, $Pd(dba)_2$, $Pd_2(dba)_3$, $Pd_2(dba)_3CHCl_3$, allylpalladium chloride dimers, palladium($\pi$-cinnamyl) chloride dimers, (1-methylallyl)palladium chloride dimers, and (1,5- cyclooctadiene)bis(trimethylsilylmethyl)palladium(II), nickel, for example, $NiCl_2$, (nickel(II) chloride) and $Ni(COD)_2$ (bis(1,5-cyclooctadiene)nickel(0)), and copper, for example, copper(I) oxide (CuzO), copper(I) iodide (CuI), copper(I) bromide (CuBr), copper(I) chloride (CuCl), copper acetate (Cu(OAc)z), bis(acetylacetonato)copper(II) ($Cu(acac)_2$), and copper(I) 2-thiophenecarboxylate (CuTc).

If necessary, a ligand may be used. Examples of the ligand for palladium that can be used include trialkylphosphine ($Cy_3P$ (tricyclohexylphosphine), $P(tBu)_3$ (tri-tert- butylphosphine), cataCXium A (di(1-adamantyl)-n-butylphosphine), (tBu)zPMe (di-tert- butylmethylphosphine), $P(Ad)_3$ (tri(1-adamantyl)phosphine), neopentyl (tBu)zP (di-tert- butylneopentylphosphine), etc.), triarylphosphine ($PPh_3$ (triphenylphosphine), etc.), dialkylbiarylphosphine (XPhos (2-dicyclohexylphosphino-2',4',6'-triisopropylbiphenyl), SPhos (2-dicyclohexylphosphino-2',6'-dimethoxybiphenyl), RuPhos (2-dicyclohexylphosphino-2',6'-diisopropoxybiphenyl), BrettPhos (2-(dicyclohexylphosphino)- 3,6-dimethoxy-2',4',6'-triisopropyl-1,1'-biphenyl), tBuXPhos (2-di-tert-butylphosphino- 2',4',6'-triisopropylbiphenyl), CPhos (2-dicyclohexylphosphino-2',6'-bis(N,N- dimethylamino)biphenyl), DavePhos (2-dicyclohexylphosphino-2'-(N,N- dimethylamino)biphenyl), RockPhos (2-di(tertiary butyl)phosphino-2',4',6'-triisopropyl-3- methoxy-6-methylbiphenyl, di-tertiary butyl(2',4',6'-triisopropyl-3-methoxy-6-methyl-[1,1'- biphenyl]-2-yl)phosphine), $Me_4$tBuXPhos (2-di-tert-butylphosphino-3,4,5,6-tetramethyl- 2',4',6'-triisopropyl-1,1'-biphenyl), tBuBrettPhos (2-(di-tert-butylphosphino)-2',4',6'- triisopropyl-3,6-dimethoxy-1,1'-biphenyl), AdBrettPhos (2-(di-1 -adamantylphosphino)- 2',4',6'-triisopropyl-3,6-dimethoxy-1,1'-biphenyl), BippyPhos (5-(di-tert-butylphosphino)- 1',3',5'-triphenyl-1'H-[1,4']bipyrazole), AdBippyPhos (5-[di(1-adamantyl)phosphino]-1',3',5'-triphenyl-1'H-[1,4']bipyrazole), etc.), dialkylmonoarylphosphine (APhos, ((4-(N,N- dimethylamino)phenyl)di-tert-butylphosphine), $(tBu)_2PPh$, MorDalPhos (2- morpholinophenyldi(1-adamantyl)phosphine), etc.), bidentate phosphine ligands (dppf (1,1'- bis(diphenylphosphino)ferrocene), dtbpf (1,1'-bis(di-tert-butylphosphino)ferrocene), BINAP (2,2'-bis(diphenylphosphino)-1,1'-binaphthyl), XantPhos (4,5-bis(diphenylphosphino)-9,9- dimethylxanthene), Josiphos ((R)-1-[(Sp)-2-(dicyclohexylphosphino)ferrocenyl]ethyldi-tert- butylphosphine), etc.), and NHC ligands (IPr (1,3-bis(2,6-diisopropylphenyl)imidazol-2- ylidene), SIPr (1,3-bis(2,6-di-i-propylphenyl)imidazolidin-2-ylidene), IMes (1,3-bis(2,4,6- trimethylphenyl)imidazol-2-ylidene), IPent (1,3-bis(2,6-di-3-pentylphenyl)imidazol-2- ylidene), etc.). Alternatively, the ligand described above and a precursor of the palladium catalyst may be used. Examples of the precursor of the palladium catalyst that can be used include first-generation Buchwald precatalysts (G1) (XPhos Pd G1, SPhos Pd G1, RuPhos Pd G1, BrettPhos Pd G1, tBuXPhos Pd G1, etc.), second-generation Buchwald precatalysts (G2) (XPhos Pd G2, SPhos Pd G2, RuPhos Pd G2, BrettPhos Pd G2, tBuXPhos Pd G2, CPhos Pd G2, DavePhos Pd G2, APhos Pd G2, $(tBu)_2PPh$ Pd G2, MorDalPhos Pd G2, $PCy_3$ Pd G2, $P(tBu)_3Pd$ G2, cataCXium A Pd G2, XantPhos Pd G2, etc.), third-generation Buchwald precatalysts (G3) (XPhos Pd G3, SPhos Pd G3, RuPhos Pd G3, BrettPhos Pd G3, tBuXPhos Pd G3, CPhos Pd G3, DavePhos Pd G3, RockPhos Pd G3, $Me_4$tBuXPhos Pd G3, tBuBrettPhos Pd G3, AdBrettPhos Pd G3, APhos Pd G3, $(tBu)_2PPh$ Pd G3, MorDalPhos Pd G3, $PCy_3$ Pd G3, $P(tBu)_3Pd$ G3, cataCXium A Pd G3, (tBu)zPMe Pd G3, neopentyl (tBu)zP Pd G3, dppf Pd G3, dtbpf Pd G3, BINAP Pd G3, XantPhos Pd G3, Josiphos Pd G3, etc.), fourth-generation Buchwald precatalysts (G4) (XPhos Pd G4, SPhos Pd G4, RuPhos Pd G4, BrettPhos Pd G4, tBuXPhos Pd G4, CPhos Pd G4, tBuBrettPhos Pd G4, APhos Pd G4, $PCy_3$ Pd G4, $P(tBu)_3Pd$ G4, cataCXium A Pd G4, dppf Pd G4, BINAP Pd G4, XantPhos Pd G4, etc.), dichloropalladium(II) complexes ($Pd(PPh_3)_2c1_2$, $Pd(PCy_3)_2c1_2$, $Pd(dppf)Cl_2$, $Pd(dtbpf)Cl_2$, $(APhos)_2PdCl_2$, etc.), palladium(0) complexes (tetrakistriphenylphosphine palladium(0), bis(tricyclohexylphosphine)palladium(0), bis(tri-tert- butylphosphine)palladium(0), etc.), palladium(I) halide complexes (bromo(tri-tert-butylphosphine)palladium(I) dimers, iodo(tri-tert-butylphosphine)palladium(I) dimers, etc.), phosphine $\pi$-allylpalladium catalysts (XPhos Pd(crotyl)Cl (chloro(crotyl)(2- dicyclohexylphosphino-2',4',6'-triisopropyl-1,1'-biphenyl)palladium(II)), SPhos Pd(crotyl)Cl (chloro(crotyl)(2-dicyclohexylphosphino-2',6'-dimethoxy-1,1'-biphenyl)palladium(II)), RuPhos Pd(crotyl)Cl (chloro(crotyl)(2-dicyclohexylphosphino-2',6'-diisopropoxy-1,1'- biphenyl)palladium(II)), [BrettPhos Pd(crotyl)]OTf (crotyl(2-dicyclohexylphosphino-2',4',6'- triisopropyl-3,6-dimethoxy-1,1'-biphenyl)palladium(II) triflate),

[tBuXPhos Pd(allyl)]OTf (allyl(2-di-tert-butylphosphino-2',4',6'-triisopropyl-1,1'-biphenyl)palladium(II) triflate), [tBuBrettPhos Pd(allyl)]OTf (allyl(2-di-tert-butylphosphino-3,6-dimethoxy-2',4',6'- triisopropyl-1,1'-biphenyl)palladium(II) triflate), AmPhos Pd(crotyl)Cl (chloro(crotyl)[(p- dimethylaminophenyl)(di-tert-butylphosphine)]palladium(II)), $P(Cy)_3$ Pd(crotyl)Cl (chloro(crotyl)(tricyclohexylphosphine)palladium(II)), $[P(tBu)_3]$ Pd(crotyl)Cl (tri-tert- butylphosphine(chloro)(crotyl)palladium(II)), [BINAP Pd(allyl)]Cl [XantPhos Pd(allyl)]Cl (allyl[4,5-bis(diphenylphosphino)-9,9-dimethylxanthene]palladium(II) chloride), etc.), PEPPSI catalysts (Pd-PEPPSI IPr, Pd-PEPPSI SIPr ((1,3-bis(2,6- diisopropylphenyl)imidazolidene)(3-chloropyridyl)palladium(II) dichloride), Pd-PEPPSI IPent, etc.), (NHC)Pd(allyl)Cl catalysts (allyl[1,3-bis(2,6-diisopropylphenyl)imidazol-2- ylidene]chloropalladium(II), allyl[1,3-bis(2,6-diisopropylphenyl)-2- imidazolidinylidene]chloropalladium(II), [1,3-bis(2,6-diisopropylphenyl)imidazol-2- ylidene]chloro[3-phenylallyl]palladium(II), etc.), and NHC-Pd naphthoquinone catalysts (1,3-bis(2,6-diisopropylphenyl)imidazol-2-ylidene(1,4-naphthoquinone)palladium(0) dimers, etc.).

Examples of the ligand for copper that can be used include diamine ligands, for example, di-tert-butyl bipyridine (dtBBPy (4,4'-di-tert-butyl-2,2'-dipyridyl), etc.), dimethylethylenediamine (DMEDA (N,N'-dimethylethylenediamine), etc.), cyclohexyldimethylethylenediamine (CyDMEDA (trans-N,N'-dimethylcyclohexane-1,2- diamine), etc.), and 1,10-phenanthroline (Phen), amino acid ligands, for example, N,N'- dimethylglycine, sarcosine (N-methylglycine), L-proline, 4-hydroxy-1-proline, pyrrole-2- carboxylic acid, and pipecolinic acid, diamide ligands, for example, $N^1$-(2-methyl-1- naphthalenyl)-$N^2$-(phenylmethyl)ethanediamide, $N^1$,$N^2$-bis(phenylmethyl)ethanediamine, $N^1$,$N^2$-bis(2,6-dimethylphenyl)ethanediamine, $N^1$,$N^2$-bis(2-furanylmethyl)ethanediamine, $N^1$,$N^2$-bis(2,4,6-trimethoxyphenyl)ethanediamine, $N^1$,$N^2$-bis(5-methyl[1,1'-biphenyl]-2-yl)- ethanediamide, [(2,6-dimethylphenyl)amino](oxo)acetic acid (DMPAO), N,N'-bis(furan-2- ylmethyl)oxalamide (BFMO), N,N'-bis(2,4,6-trimethoxyphenyl)-oxalamide (BTMPO), and N-[1,1'-biphenyl]-2-yl-1H-indole-2-carboxamide, and others such as 1,1,1-trifluoro-3- phenyl-2-(1H-pyrrol-2-yl)propan-2-ol and 2-methyl-8-hydroxyquinoline. In the case of using a metal catalyst, the number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. In the case of using a ligand, the number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. In the case of using a catalyst precursor, the number of equivalents of the catalyst precursor used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier and A-X is aryl halide or an analog thereof, the aromatic nucleophilic substitution reaction with amine or the catalytic coupling reaction using a metal catalyst can be performed using the already mentioned reagent and reaction conditions. In this context, in the case of using a base, the number of equivalents thereof can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing B. The number of equivalents of A-X used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing B. In the case of using a metal catalyst, the number of equivalents of the metal catalyst can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the amine containing B. In the case of using a ligand, the number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the amine containing B. In the case of using a catalyst precursor, the number of equivalents of the catalyst precursor used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the amine containing B.

When A-X in the formula is alkyl halide, hydroxyalkyl or an analog thereof, the reactive functional group X linked to A is a leaving group, for example, halogen or a group derived from a hydroxy group. Examples of the group derived from a hydroxy group include groups such as a methanesulfonyl group, a trifluoromethanesulfonyl group, a nonafluorobutanesulfonyl group, a benzenesulfonyl group, a para-toluenesulfonyl group, a 2- nitrobenzenesulfonyl group, a 4-nitrobenzenesulfonyl group, and a 2,4- dinitrobenzenesulfonyl group. The linker represented by -NR3- can be formed through the reaction thereof with amine linked to B in the presence of a base.

In this context, the base is not particularly limited as long as the base can be usually used. Examples of the base that can be used include sodium carbonate, potassium carbonate, cesium carbonate, sodium bicarbonate, potassium bicarbonate, lithium acetate, sodium acetate, potassium acetate, cesium acetate, lithium hydroxide, sodium hydroxide, potassium hydroxide, barium hydroxide, tetraalkylammonium hydroxide (tetramethylammonium hydroxide, tetrabutylammonium hydroxide, etc.), sodium hydride, potassium hydride, DBU, DBN, MTBD, BTMG, TMG, 1,8-bis(dimethylamino)naphthalene, BEMP, BTPP, P2Et, P2tBu, potassium phosphate, LiOtBu, NaOtBu, KOtBu, LiHMDS, NaHMDS, KHMDS, HMDS, pyridine, 2,6-lutidine, 2,4,6-collidine, DTBP, DTBMP, triethylamine, DIPEA, 4- methylmorpholine, NMI, DMAP, potassium hydride, sodium hydride, lithium hydride, sodium amide, lithium amide, sodium phosphate, $K_2HPO_4$, $KHCO_3$, $Na_2HPO_4$, sodium tetraborate, potassium t-pentoxide, sodium t-pentoxide, lithium t-pentoxide, lithium diisopropylamide, lithium dicyclohexylamide, 2,2,6,6-tetramethylpiperidinyl lithium, 2,2,6,6- tetramethylpiperidinyl magnesium chloride-lithium chloride complexes, N,N-dimethylaniline, P1-tBu, P2tBu, benzyl triethylammonium hydroxide, butyllithium, methyllithium, tetrabutylammonium fluoride, and cesium fluoride. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The number of equivalents of the amine containing B used can be appropriately set by those skilled in the art and can be, for example, 1 to 100 equivalents, 1 to 75 equivalents, 1 to 50 equivalents, 1 to 30 equivalents, or 1 to 10 equivalents, based on A-X.

$R^3$ in the formula is as already defined. Preferred examples of $R^3$ include a hydrogen atom, $C_{1-6}$ alkyl optionally having a substituent, for example, methyl and ethyl, and aromatic rings optionally having a substituent, for example, benzene and pyridine. Examples of the ring structure that is formed together with the nitrogen atom and B bonded include pyrrolidine, piperidine, piperazine, and morpholine. Examples of the heterocyclic aromatic amine include pyrrole, imidazole, pyrazole, indole, and benzimidazole.

A solvent can be used in the reaction. Examples of the solvent that can be used include, but are not particularly limited to, halogen solvents such as DCM, chloroform, DCE, and carbon tetrachloride, ether solvents such as diethyl ether, diisopropyl ether, THF, 2- methyltetrahydrofuran, 4-methyltetrahydropyran, 1,4-dioxane, DME, TBME, CPME, diisopropyl ether, isosorbide dimethyl ether, diglyme, triglyme, and tetraglyme, urea solvents such as TMU, DMI, and DMPU, amide solvents such as DMF, DMA, NFM, NMP, NEP, and NBP, sulfoxide solvents such as DMSO, sulfone solvents such as diphenylsulfone, dimethylsulfone, diethylsulfone, sulfolane, 3-methylsulfolane, ethylmethylsulfone, and ethylisopropylsulfone, aromatic solvents such as anisole, toluene, xylene, chlorobenzene, bromobenzene, ethylbenzene, nitrobenzene, $\alpha,\alpha,\alpha$-trifluorotoluene, 1,2-dichlorobenzene, benzene, and pyridine, carbonate solvents such as dimethyl carbonate, diethyl carbonate, ethylene carbonate, and propylene carbonate, and others such as acetonitrile and limonene. A single solvent may be used, or a combined solvent of a plurality of solvents may be used. The reaction temperature can be appropriately set by those skilled in the art and can be, for example, -20 to 120°C, -20 to 80°C, 0 to 80°C, or 0 to 60°C. If necessary, an additive may be used. Examples of the additive that can be used include 12-crown-4, 15-crown-5, 18- crown-6, HMPA, TMEDA, tetrabutylammonium chloride, tetrabutylammonium bromide, tetrabutylammonium iodide, and tetrabutylammonium bisulfate. After the completion of the reaction, the target compound can be obtained by a usual aftertreatment method.

When B in the formula contains a solid-phase carrier and A-X is alkyl halide, hydroxyalkyl or an analog thereof, the nucleophilic substitution reaction with amine can be performed using the already mentioned reagent and reaction conditions. In this context, in the case of using a base, the number of equivalents thereof can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing B. The number of equivalents of A-X used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing B.

[Formula 17]

Carbon-nitrogen single bond formation through reaction of primary amide or secondary amide with aryl halide or derivative thereof

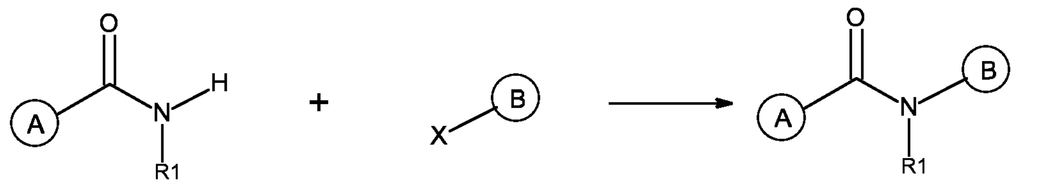

wherein $R^1$ is a hydrogen atom or $C_{1-6}$ alkyl optionally having a substituent, and optionally forms a ring together with the nitrogen atom, the carbonyl carbon atom and A bonded thereto.

The amide bond as the linker can also be formed through the reaction of primary amide or secondary amide with aryl halide or a derivative thereof.

When A in the formula contains a solid-phase carrier and is a derivative of amide linked to A via a carbonyl group, the amide bond as the linker can be formed through the reaction thereof with B-X wherein the reactive functional group X linked to B is halogen or a hydroxy group derivative using a base. In this context, the base is not particularly limited as long as the base can be usually used. Examples of the base that can be used include NaOtBu, KOtBu, $K_2CO_3$, $Cs_2CO_3$, $K_3PO_4$, NaOH, NaOPh, KOPh, NaBHT, DBU, TEA, TMG, BTMG, MTBD, BTPP, P1tBu, P2Et, P2tBu, LiHMDS, NaHMDS, KHMDS, potassium hydride, sodium hydride, lithium hydride, sodium amide, lithium amide, KOH, NaOH, LiOH, sodium carbonate, potassium carbonate, cesium carbonate, sodium phosphate, potassium phosphate, LiOtBu, potassium t-pentoxide, sodium t-pentoxide, lithium t-pentoxide, lithium diisopropylamide, lithium dicyclohexylamide, 2,2,6,6-tetramethylpiperidinyl lithium, 2,2,6,6- tetramethylpiperidinyl magnesium chloride-lithium chloride complexes, tetrabutylammonium hydroxide, benzyl triethylammonium hydroxide, butyllithium, methyllithium, tetrabutylammonium fluoride, and cesium fluoride. The number of equivalents of B-X used can be appropriately set by those skilled in the art and can be, for example, 1 to 100 equivalents, 1 to 75 equivalents, 1 to 50 equivalents, 1 to 30 equivalents, or 1 to 10 equivalents, based on the amide containing A. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the amide containing A.

A solvent can be used in the reaction. Examples of the solvent that can be used include, but are not particularly limited to, ether solvents (tetrahydrofuran, 2- methyltetrahydrofuran, 1,4-dioxane, 1,2-dimethoxyethane, diisopropyl ether, cyclopentyl methyl ether, t-butyl methyl ether, 4-methyltetrahydropyran, diglyme, triglyme, tetraglyme, etc.), amide solvents (N,N-dimethylformamide (DMF), N-methylpyrrolidone (NMP), N,N- dimethylacetamide (DMA), N-ethylpyrrolidone (NEP), N-butylpyrrolidone (NBP), etc.), sulfoxide solvents (dimethyl sulfoxide (DMSO), methylphenyl sulfoxide, etc.), urea solvents (1,3-dimethyl-2-imidazolidinone (DMI), 1,3-dimethyl-3,4,5,6-tetrahydro-2(1H)- pyrimidinone (DMPU), etc.), and alcohol solvents (tBuOH, tAmOH, etc.). A single solvent may be used, or a combined solvent of a plurality of solvents may be used. The reaction temperature can be appropriately set by those skilled in the art and can be, for example, 0 to 200°C, 0 to 150°C, 20 to 150°C, 40 to 120°C, or 60 to 100°C. If necessary, a metal catalyst may be used. Examples of the metal catalyst that can be used include palladium salts such as $PdCl_2$, $Pd(OAc)_2$, and $Pd_2(dba)_3$-$CHCl_3$, nickel salts such as NiClz (nickel(II) chloride) and $Ni(COD)_2$ (bis(1,5-cyclooctadiene)nickel(0)), copper salts such as CuI, CuBr, CuCl, Cu(OAc)z, copper(II) oxide, and copper(I) oxide, and iron(III) chloride. If necessary, a ligand may be used. Examples of the ligand that can be used include trialkylphosphine ($tBu_3P$ (tri-tert-butylphosphine), $Cy_3P$ (tricyclohexylphosphine), $tBu_2MeP$ (di-tert- butylmethylphosphine), etc.), biaryldialkylphosphine (XPhos (2-dicyclohexylphosphino-2',4',6'-triisopropylbiphenyl), tBuXPhos (2-di-tert-butylphosphino-2',4',6'- triisopropylbiphenyl), $Me_4$tBuXPhos (2-di-tert-butylphosphino-3,4,5,6-tetramethyl-2',4',6'- triisopropyl-1,1'-biphenyl), RuPhos (2-dicyclohexylphosphino-2',6'-diisopropoxybiphenyl), BrettPhos (2-(dicyclohexylphosphino)-3,6-dimethoxy-2',4',6'-triisopropyl-1,1'-biphenyl), tBuBrettPhos (2-(di-tert-butylphosphino)-2',4',6'-triisopropyl-3,6-dimethoxy-1,1'-biphenyl), AdBrettPhos (2-(di-1-adamantylphosphino)-2',4',6'-triisopropyl-3,6-dimethoxy-1,1 '-biphenyl), etc.), AlPhos (di-1-adamantyl(4"-butyl-2",3",5",6"-tetrafluoro-2',4',6'-triisopropyl-2-methoxy- meta-terphenyl)phosphine), triarylphosphine (e.g., JackiePhos (bis(3,5- bis(trifluoromethyl)phenyl)(2',4',6'-triisopropyl-3,6-dimethoxybiphenyl-2-yl)phosphine)), bisphosphine (e.g., DPPF (1,1'-bis(diphenylphosphino)ferrocene), DtBPF (1,1'-bis(di-tert- butylphosphino)ferrocene), Xantphos (4,5-bis(diphenylphosphino)-9,9-dimethylxanthene), and BINAP (2,2'-bis(diphenylphosphino)-1,1'-binaphthyl)), N-heterocyclic carbene derivatives (e.g., IPr (1,3-bis(2,6-diisopropylphenyl)imidazol-2-ylidene), SIPr (1,3-bis(2,6- di-i-propylphenyl)imidazolidin-2-ylidene), IMes (1,3-bis(2,4,6-trimethylphenyl)imidazol-2- ylidene), and IPent (1,3-bis(2,6-di-3-pentylphenyl)imidazol-2-ylidene)), and diamine (e.g., dtBBPy (4,4'-di-tert-butyl-2,2'-dipyridyl), DMEDA (N,N'-dimethylethylenediamine), CyDMEDA (trans-N,N'-dimethylcyclohexane-1,2-diamine), and Phen (1,10-phenanthroline)). Alternatively, a metal-ligand complex may be used as a catalyst. Examples of the catalyst that can be used include Buchwald precatalysts such as XPhos Pd G1 to G4, Xantphos Pd G3 and G4, tBuBrettPhos Pd G4, and AlPhos Pd G6 Br (CAS: 2097600-15-0), and NHC-Pd complexes such as PEPPSI-IPr and [(NHC)Pd(allyl)Cl] (allyl[1,3-bis(2,6- diisopropylphenyl)imidazol-2-ylidene]chloropalladium(II)). In the case of using a metal catalyst, the number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the amide containing A. In the case of using a ligand, the number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the amide containing A. In the case of using a metal-ligand complex, the number of equivalents of the metal-ligand complex used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the amide containing A. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of

the completion of the reaction can be determined or confirmed by cleaving products from some solid-phase carriers. After the completion of the reaction, the target compound can be obtained by a usual aftertreatment method.

When B in the formula contains a solid-phase carrier, the reaction of primary amide or secondary amide containing A with aryl halide or a derivative thereof (B-X) can be performed using the already mentioned reagent and reaction conditions. In this context, the number of equivalents of the primary amide or the secondary amide containing A used can be appropriately set by those skilled in the art and can be, for example, 1 to 100 equivalents, 1 to 75 equivalents, 1 to 50 equivalents, 1 to 30 equivalents, or 1 to 10 equivalents, based on B-X. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on B-X. In the case of using a metal catalyst, the number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on B-X. In the case of using a ligand, the number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on B-X. In the case of using a metal-ligand complex, the number of equivalents of the metal-ligand complex used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on B-X.

[Formula 18]

Carbon-nitrogen single bond formation through reaction of primary sulfonamide or secondary sulfonamide with aryl halide or derivative thereof

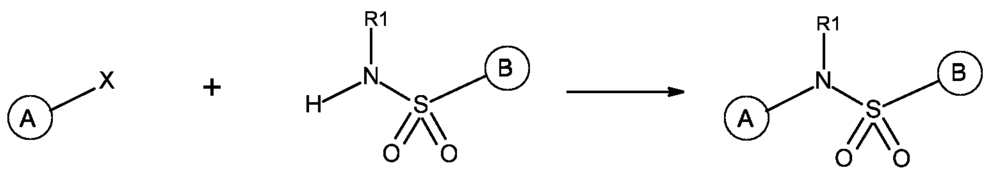

wherein R1 is a hydrogen atom or $C_{1-6}$ alkyl optionally having a substituent, and optionally forms a ring together with the nitrogen atom, the sulfur atom and B bonded thereto.

The sulfonamide bond as the linker can also be formed through the reaction of primary sulfonamide or secondary sulfonamide with aryl halide or a derivative thereof.

When A in the formula contains a solid-phase carrier and when A-X is aryl halide or a derivative thereof, the reactive functional group X linked to A is halogen or a hydroxy group derivative. The sulfonamide bond as the linker can be formed through the reaction thereof with a compound containing a sulfonamide group linked to B using a base. In this context, the base is not particularly limited as long as the base can be usually used. Examples of the base that can be used include NaOtBu, KOtBu, $K_2CO_3$, $Cs_2CO_3$, $K_3PO_4$, NaOH, NaOPh, KOPh, NaBHT, DBU, TEA, TMG, BTMG, MTBD, BTPP, P1tBu, P2Et, P2tBu, LiHMDS, NaHMDS, KHMDS, potassium hydride, sodium hydride, lithium hydride, sodium amide, lithium amide, KOH, NaOH, LiOH, sodium carbonate, potassium carbonate, cesium carbonate, sodium phosphate, potassium phosphate, LiOtBu, potassium t-pentoxide, sodium t-pentoxide, lithium t-pentoxide, lithium diisopropylamide, lithium dicyclohexylamide, 2,2,6,6-tetramethylpiperidinyl lithium, 2,2,6,6-tetramethylpiperidinyl magnesium chloride- lithium chloride complexes, N,N-dimethylaniline, tetrabutylammonium hydroxide, benzyl triethylammonium hydroxide, butyllithium, methyllithium, tetrabutylammonium fluoride, and cesium fluoride.

A solvent can be used in the reaction. Examples of the solvent that can be used include, but are not particularly limited to, ether solvents (tetrahydrofuran, 2- methyltetrahydrofuran, 1,4-dioxane, 1,2-dimethoxyethane, diisopropyl ether, cyclopentyl methyl ether, t-butyl methyl ether, 4-methyltetrahydropyran, diglyme, triglyme, tetraglyme, etc.), amide solvents (N,N-dimethylformamide (DMF), N-methylpyrrolidone (NMP), N,N- dimethylacetamide (DMA), N-ethylpyrrolidone (NEP), N-butylpyrrolidone (NBP), etc.), sulfoxide solvents (dimethyl sulfoxide (DMSO), methylphenyl sulfoxide, etc.), urea solvents (1,3-dimethyl-2-imidazolidinone (DMI), 1,3-dimethyl-3,4,5,6-tetrahydro-2(1H)- pyrimidinone (DMPU), etc.), and alcohol solvents (tBuOH, tAmOH, etc.). A single solvent may be used, or a combined solvent of a plurality of solvents may be used. The reaction temperature can be appropriately set by those skilled in the art and can be, for example, 0 to 150°C, 20 to 150°C, 40 to 120°C, or 60 to 100°C. If necessary, a metal catalyst may be used. Examples of the metal catalyst that can be used include palladium salts such as $PdCl_2$, $Pd(OAc)_2$, and $Pd_2(dba)_3$-$CHCl_3$, nickel salts such as $NiCl_2$ (nickel(II) chloride) and $Ni(COD)_2$ (bis(1,5-cyclooctadiene)nickel(0)), and copper salts such as CuI,

CuBr, CuCl, $Cu(OAc)_2$, copper(II) oxide, and copper(I) oxide. If necessary, a ligand may be used. Examples of the ligand that can be used include trialkylphosphine ($tBu_3P$ (tri-tert- butylphosphine), $Cy_3P$ (tricyclohexylphosphine), $tBu_2MeP$ (di-tert-butylmethylphosphine), etc.), biaryldialkylphosphine (XPhos (2-dicyclohexylphosphino-2',4',6'-triisopropylbiphenyl), tBuXPhos (2-di-tert-butylphosphino-2',4',6'-triisopropylbiphenyl), $Me_4$tBuXPhos (2-di-tert- butylphosphino-3,4,5,6-tetramethyl-2',4',6'-triisopropyl-1,1'-biphenyl), RuPhos (2- dicyclohexylphosphino-2',6'-diisopropoxybiphenyl), BrettPhos (2-(dicyclohexylphosphino)- 3,6-dimethoxy-2',4',6'-triisopropyl-1,1'-biphenyl), tBuBrettPhos (2-(di-tert-butylphosphino)-2',4',6'-triisopropyl-3,6-dimethoxy-1,1'-biphenyl), AdBrettPhos (2-(di-1- adamantylphosphino)-2',4',6'-triisopropyl-3,6-dimethoxy-1,1'-biphenyl), BippyPhos (5-(di- tert-butylphosphino)-1',3',5'-triphenyl-1'H-[1,4']bipyrazole), AdBippyPhos (5-[di(1-adamantyl)phosphino]-1',3',5'-triphenyl-1'H-[1,4']bipyrazole), etc.), AlPhos (di-1-adamantyl(4"-butyl-2",3",5",6"-tetrafluoro-2',4',6'-triisopropyl-2-methoxy-meta- terphenyl)phosphine), triarylphosphine (e.g., JackiePhos (bis(3,5-bis(trifluoromethyl)phenyl)(2',4',6'-triisopropyl-3,6-dimethoxybiphenyl-2-yl)phosphine), etc.), bisphosphine (e.g., DPPF (1,1'-bis(diphenylphosphino)ferrocene), DtBPF (1,1'-bis(di-tert- butylphosphino)ferrocene), Xantphos (4,5-bis(diphenylphosphino)-9,9-dimethylxanthene), and BINAP (2,2'-bis(diphenylphosphino)-1,1'-binaphthyl)), N-heterocyclic carbene derivatives (e.g., IPr (1,3-bis(2,6-diisopropylphenyl)imidazol-2-ylidene), SIPr (1,3-bis(2,6- di-i-propylphenyl)imidazolidin-2-ylidene), IMes (1,3-bis(2,4,6-trimethylphenyl)imidazol-2- ylidene), and IPent (1,3-bis(2,6-di-3-pentylphenyl)imidazol-2-ylidene)) and diamine (e.g., dtBBPy (4,4'-di-tert-butyl-2,2'-dipyridyl), DMEDA (N,N'-dimethylethylenediamine), Cy-DMEDA (trans-N,N'-dimethylcyclohexane-1,2-diamine), and Phen (1,10-phenanthroline)). Alternatively, a metal-ligand complex may be used as a catalyst. Examples of the catalyst that can be used include Buchwald precatalysts such as XPhos Pd G1 to G4, Xantphos Pd G3 and G4, tBuBrettPhos Pd G4, and AlPhos Pd G6 Br (CAS: 2097600-15-0), and NHC-Pd complexes such as PEPPSI-IPr and [(NHC)Pd(allyl)Cl] (allyl[1,3-bis(2,6- diisopropylphenyl)imidazol-2-ylidene]chloropalladium(II)). In this context, the number of equivalents of the sulfonamide containing B used can be appropriately set by those skilled in the art and can be, for example, 1 to 100 equivalents, 1 to 75 equivalents, 1 to 50 equivalents, 1 to 30 equivalents, or 1 to 10 equivalents, based on the aryl halide or the derivative thereof containing A (A-X). The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the aryl halide or the derivative thereof containing A (A-X). In the case of using a metal catalyst, the number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. In the case of using a ligand, the number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. In the case of using a metal-ligand complex, the number of equivalents of the metal-ligand complex used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier, the reaction of aryl halide or a derivative thereof containing A (A-X) with primary sulfonamide or secondary sulfonamide containing B can be performed using the already mentioned reagent and reaction conditions. In this context, the number of equivalents of the aryl halide or the derivative thereof containing A (A-X) used can be appropriately set by those skilled in the art and can be, for example, 1 to 100 equivalents, 1 to 75 equivalents, 1 to 50 equivalents, 1 to 30 equivalents, or 1 to 10 equivalents, based on the sulfonamide containing B. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the sulfonamide containing B. In the case of using a metal catalyst, the number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the sulfonamide containing B. In the case of using a ligand, the number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the sulfonamide containing B. In the case of using a metal-ligand complex, the number of equivalents of the metal-ligand complex used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the sulfonamide containing B.

[Formula 19]

Reductive amination reaction

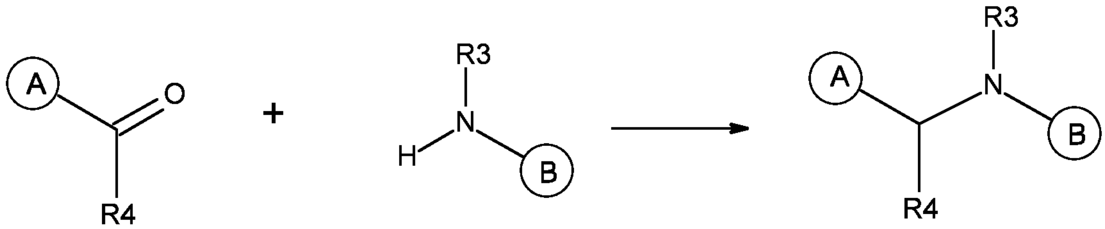

wherein R4 is a hydrogen atom or $C_{1-6}$ alkyl optionally having a substituent, and optionally forms a ring together with the carbon atom and A bonded thereto, and R3 is a hydrogen atom or $C_{1-6}$ alkyl optionally having a substituent, and optionally forms a ring together with the nitrogen atom and B bonded thereto.

The amine bond as the linker can also be formed through reductive amination reaction using aldehyde and amine, or ketone and amine.

When A in the formula contains a solid-phase carrier, R4 in the reactive functional group -C(=O)-R4 linked to A is selected from $C_{1-6}$ alkyl, $C_{6-10}$ aryl, 5- to 10-membered heteroaryl, and $C_{3-7}$ cycloalkyl. For example, the group -C(=O)-R4 is formyl or acetyl. The carbon-nitrogen single bond can be formed through the reaction thereof with amine containing an amino group linked to B using a reducing agent. In this context, the reducing agent is not particularly limited as long as the reducing agent can be usually used. Examples of the reducing agent that can be used include pyridine borane, 2-picoline borane, tert- butylamine borane, dimethyl sulfide borane, sodium triacetoxyborohydride, sodium borohydride, sodium cyanoborohydride, tetramethylammonium triacetoxyborohydride, tetramethylammonium borohydride, tetraethylammonium borohydride, lithium triethyl borohydride, diisobutyl aluminum hydride, sodium bis(2-methoxyethoxy)aluminum hydride, lithium aluminum hydride, triethylsilane, phenylsilane, and phenyldimethylsilane. The number of equivalents of the reducing agent used can be appropriately set by those skilled in the art and can be, for example, 1 to 100 equivalents, 1 to 75 equivalents, 1 to 50 equivalents, 1 to 30 equivalents, or 1 to 10 equivalents, based on the aldehyde containing A. The number of equivalents of the amine containing B used can be appropriately set by those skilled in the art and can be, for example, 1 to 100 equivalents, 1 to 75 equivalents, 1 to 50 equivalents, 1 to 30 equivalents, or 1 to 10 equivalents, based on the aldehyde containing A.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), aromatic hydrocarbon solvents (anisole, toluene, xylene, chlorobenzene, bromobenzene, ethylbenzene, nitrobenzene, α ,α,α-trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and alcohol solvents (methanol, ethanol, etc.). A single solvent may be used, or a combined solvent of a plurality of solvents may be used. The reaction temperature can be appropriately set by those skilled in the art and can be, for example, 0 to 200°C, 20 to 200°C, 20 to 150°C, 40 to 150°C, or 60 to 150°C. If necessary, an acid may be used. Examples of the acid that can be used include acetic acid, formic acid, trifluoroacetic acid, hydrochloric acid, and sulfuric acid. If necessary, a Lewis acid may be used. Examples of the Lewis acid that can be used include zinc chloride, titanium tetrachloride, titanium trichloride, boron trifluoride-ethyl ether complexes, tetraethyl ortho-titanate, tetraisopropyl ortho-titanate, nickel chloride, and lithium chloride. In the case of using an acid, the number of equivalents of the acid used can be appropriately set by those skilled in the art and can be, for example, 0.01 to 100 equivalents, 0.01 to 75 equivalents, 0.01 to 50 equivalents, 0.01 to 30 equivalents, 0.1 to 30 equivalents, or 0.1 to 10 equivalents, based on the aldehyde containing A. In the case of using a Lewis acid, the number of equivalents of the Lewis acid used can be appropriately set by those skilled in the art and can be, for example, 0.01 to 100 equivalents, 0.01 to 75 equivalents, 0.01 to 50 equivalents, 0.01 to 30 equivalents, 0.1 to 30 equivalents, or 0.1 to 10 equivalents, based on the aldehyde containing A. The reaction time can be appropriately set by those skilled in the art and can be,

for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier, the reaction can be performed using the already mentioned reagent and reaction conditions. In this context, the number of equivalents of the aldehyde or the ketone containing A used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing B. The number of equivalents of the reducing agent can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing B. In the case of using an acid, the number of equivalents of the acid used can be appropriately set by those skilled in the art and can be, for example, 0.01 to 100 equivalents, 0.01 to 75 equivalents, 0.01 to 50 equivalents, 0.01 to 30 equivalents, 0.1 to 30 equivalents, or 0.1 to 10 equivalents, based on the amine containing B. In the case of using a Lewis acid, the number of equivalents of the Lewis acid used can be appropriately set by those skilled in the art and can be, for example, 0.01 to 100 equivalents, 0.01 to 75 equivalents, 0.01 to 50 equivalents, 0.01 to 30 equivalents, 0.1 to 30 equivalents, or 0.1 to 10 equivalents, based on the amine containing B.

[Formula 20]

Ether bond formation reaction

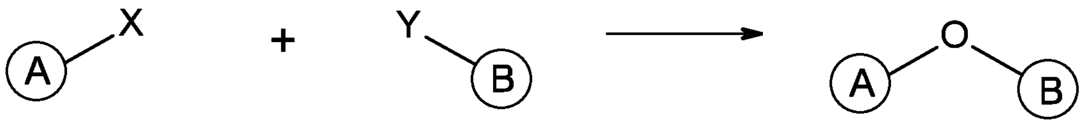

The ether bond as the linker can be formed through the reaction of hydroxyaryl with alcohol, the reaction of aryl halide or a derivative thereof with hydroxyaryl or alcohol, or the reaction of hydroxyaryl or alcohol with alkyl halide.

When A in the formula contains a solid-phase carrier and A-X is hydroxyaryl, the reactive functional group X linked to A is a hydroxy group. The ether bond as the linker can be formed through the reaction thereof with alcohol wherein the reactive functional group Y linked to B is a hydroxy group using an azodicarboxylic acid derivative and phosphine, or a cyanomethylenephosphorane derivative. In this context, the azodicarboxylic acid derivative is not particularly limited as long as the azodicarboxylic acid derivative can be usually used. Examples of the azodicarboxylic acid derivative that can be used include diethyl azodicarboxylate (DEAD), bis(2,2,2-trichloroethyl) azodicarboxylate, N,N,N',N'- tetramethylazodicarboxamide (TMAD), di-tert-butyl azodicarboxylate, dimethyl azodicarboxylate, dibenzyl azodicarboxylate, diisopropyl azodicarboxylate (DIAD), 1,1'- (azodicarbonyl)dipiperidine (ADDP), N,N,N',N'-tetraisopropylazodicarboxamide (TIPA), 1,6-dimethyl-1,5,7-hexahydro-1,4,6,7-tetrazocine-2,5-dione (DHTD), and bis(2- methoxyethyl) azodicarboxylate. The phosphine is not particularly limited as long as the phosphine can be usually used. Examples of the phosphine that can be used include triphenylphosphine (TPP), tributylphosphine (TBP), 4- (dimethylamino)phenyldiphenylphosphine, isopropyldiphenylphosphine, phenoxydiphenylphosphine, tri-n-octylphosphine, diphenyl-2-pyridylphosphine, tri-tert- butylphosphine, dicyclohexylphenylphosphine, tricyclohexylphosphine, and trihexylphosphine. The cyanomethylenephosphorane derivative is not particularly limited as long as the cyanomethylenephosphorane derivative can be usually used. Examples of the cyanomethylenephosphorane derivative that can be used include cyanomethylene tributyl phosphorane (CMBP) and cyanomethylene trimethyl phosphorane (CMMP). The number of equivalents of the alcohol containing B used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. In the case of using an azodicarboxylic acid derivative, the number of equivalents of the azodicarboxylic acid derivative used can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on A-X. In the case of using phosphine, the number of equivalents of the phosphine used can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on A-X. In the case of using a cyanomethylenephosphorane derivative, the number of equivalents of the cyanomethylenephosphorane derivative used can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on A-X.

A solvent can be used in the reaction. Examples of the solvent that can be used include, but are not particularly

limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL (γ-valerolactone), pentyl acetate, methyl propionate, etc.), aromatic hydrocarbon solvents (anisole, toluene, xylene, chlorobenzene, bromobenzene, ethylbenzene, nitrobenzene, α,α,α- trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. A single solvent may be used, or a combined solvent of a plurality of solvents may be used. The reaction temperature can be appropriately set by those skilled in the art and can be, for example, -20 to 200°C, 0 to 150°C, 0 to 120°C, or 0 to 100°C. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier and A-X is hydroxyaryl, the reactive functional group X linked to A is a hydroxy group. The formation of the ether bond as the linker through the reaction thereof with alcohol wherein the reactive functional group Y linked to B is a hydroxy group using an azodicarboxylic acid derivative and phosphine, or a cyanomethylenephosphorane derivative can be performed using the already mentioned reagent and reaction conditions. In this context, in the case of using an azodicarboxylic acid derivative, the number of equivalents thereof can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on the alcohol containing B. In the case of using phosphine, the number of equivalents thereof can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on the alcohol containing B. In the case of using a cyanomethylenephosphorane derivative, the number of equivalents thereof can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on the alcohol containing B. The number of equivalents of A-X used can be appropriately set by those skilled in the art and can be, for example, 1 to 100 equivalents, 1 to 75 equivalents, 1 to 50 equivalents, 1 to 30 equivalents, or 1 to 10 equivalents, based on the alcohol containing B.

When A in the formula contains a solid-phase carrier and A-X is aryl halide or a derivative thereof, the reactive functional group X linked to A is halogen or a hydroxy group derivative. The ether bond as the linker can be formed through the reaction thereof with hydroxyaryl or alcohol wherein the reactive functional group Y linked to B is a hydroxy group using a base. In this context, the base is not particularly limited as long as the base can be usually used. Examples of the base that can be used include sodium carbonate, potassium carbonate, cesium carbonate, sodium bicarbonate, potassium bicarbonate, sodium phosphate, potassium phosphate, disodium hydrogen phosphate, dipotassium hydrogen phosphate, sodium hydroxide, potassium hydroxide, cesium hydroxide, barium hydroxide, tetraalkylammonium hydroxide (tetramethylammonium hydroxide, tetrabutylammonium hydroxide, etc.), LiOtBu, NaOtBu, KOtBu, sodium hydride, potassium hydride, lithium bis(trimethylsilyl)amide, sodium bis(trimethylsilyl)amide, potassium bis(trimethylsilyl)amide, lithium diisopropylamide, methyllithium, butyllithium, pyridine, alkyl-substituted pyridine (2,6-lutidine, DTBP, DTBMP, etc.), DMAP, N,N-dimethylaniline, N-methylmorpholine, triethylamine, DIPEA, DBU, DBN, MTBD, TMG, BTMG, P1tBu, BEMP, BTPP, P2Et, P2tBu, NMI, lithium hydride, sodium amide, lithium amide, sodium tetraborate, 1,8- bis(dimethylamino)naphthalene, potassium t-pentoxide, sodium t-pentoxide, lithium t- pentoxide, lithium dicyclohexylamide, 2,2,6,6-tetramethylpiperidinyl lithium, 2,2,6,6- tetramethylpiperidinyl magnesium chloride-lithium chloride complexes, tetrabutylammonium fluoride, cesium fluoride, silver oxide, and silver carbonate.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents

(TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL ($\gamma$-valerolactone), pentyl acetate, methyl propionate, etc.), aromatic hydrocarbon solvents (anisole, toluene, xylene, chlorobenzene, bromobenzene, ethylbenzene, nitrobenzene, $\alpha,\alpha,\alpha$- trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. A single solvent may be used, or a combined solvent of a plurality of solvents may be used. The reaction temperature can be appropriately set by those skilled in the art and can be, for example, 0 to 140°C, 20 to 110°C, or 40 to 80°C. If necessary, a metal catalyst may be used. Examples of the metal catalyst that can be used include palladium acetate, $Pd(dba)_2$, $Pd_2(dba)_3$, $Pd_2(dba)_3CHCl_3$, allylpalladium chloride dimers, palladium($\pi$- cinnamyl) chloride dimers, (1-methylallyl)palladium chloride dimers, and (1,5- cyclooctadiene)bis(trimethylsilylmethyl)palladium(II). If necessary, a ligand may be used. Examples of the ligand that can be used include trialkylphosphine ($PCy_3$ (tricyclohexylphosphine), $P(tBu)_3$ (tri-tert-butylphosphine), cataCXium A (di(1-adamantyl)- n-butylphosphine), $(tBu)_2PMe$ (di-tert-butylmethylphosphine), $P(Ad)_3$, neopentyl (tBu)zP (di- tert-butylneopentylphosphine), etc.), triarylphosphine ($PPh_3$ (triphenylphosphine), etc.), dialkylbiarylphosphine (XPhos (2-dicyclohexylphosphino-2',4',6'-triisopropylbiphenyl), SPhos (2-dicyclohexylphosphino-2',6'-dimethoxybiphenyl), RuPhos (2- dicyclohexylphosphino-2',6'-diisopropoxybiphenyl), BrettPhos, tBuXPhos (2-di-tert- butylphosphino-2',4',6'-triisopropylbiphenyl), CPhos (2-dicyclohexylphosphino-2',6'- bis(N,N-dimethylamino)biphenyl), DavePhos (2-dicyclohexylphosphino-2'-(N,N- dimethylamino)biphenyl), RockPhos (2-di(tertiary butyl)phosphino-2',4',6'-triisopropyl-3- methoxy-6-methylbiphenyl, di-tertiary butyl(2',4',6'-triisopropyl-3-methoxy-6-methyl-[1,1'- biphenyl]-2-yl)phosphine), $Me_4$tBuXPhos (2-di-tert-butylphosphino-3,4,5,6-tetramethyl- 2',4',6'-triisopropyl-1,1'-biphenyl), tBuBrettPhos (2-(di-tert-butylphosphino)-2',4',6'- triisopropyl-3,6-dimethoxy-1,1'-biphenyl), AdBrettPhos (2-(di-1 -adamantylphosphino)- 2',4',6'-triisopropyl-3,6-dimethoxy-1,1'-biphenyl), etc.), dialkylmonoarylphosphine (APhos ((4-(N,N-dimethylamino)phenyl)di-tert-butylphosphine), $(tBu)_2PPh$ (di-tert- butylphenylphosphine), MorDalPhos (2-morpholinophenyldi(1-adamantyl)phosphine), etc.), bidentate phosphine ligands (dppf (1,1'-bis(diphenylphosphino)ferrocene), dtbpf (1,1'-bis(di- tert-butylphosphino)ferrocene), BINAP (2,2'-bis(diphenylphosphino)-1,1'-binaphthyl), XantPhos (4,5-bis(diphenylphosphino)-9,9-dimethylxanthene), Josiphos ((R)-1-[(Sp)-2- (dicyclohexylphosphino)ferrocenyl]ethyldi-tert-butylphosphine), etc.), NHC ligands (IPr (1,3-bis(2,6-diisopropylphenyl)imidazol-2-ylidene), SIPr (1,3-bis(2,6-di-i- propylphenyl)imidazolidin-2-ylidene), IMes (1,3-bis(2,4,6-trimethylphenyl)imidazol-2- ylidene), IPent (1,3-bis(2,6-di-3-pentylphenyl)imidazol-2-ylidene), etc.), BippyPhos (5-(di- tert-butylphosphino)-1',3',5'-triphenyl-1'H-[1,4']biprazole), and AdBippyPhos (5-[di(1- adamantyl)phosphino]-1',3',5'-triphenyl-1'H-[1,4']biprazole). Alternatively, the ligand described above and a precursor of the palladium catalyst may be used. Examples of the precursor of the palladium catalyst that can be used include first-generation Buchwald precatalysts (G1) (XPhos Pd G1, SPhos Pd G1, RuPhos Pd G1, BrettPhos Pd G1, tBuXPhos Pd G1, etc.), second-generation Buchwald precatalysts (G2) (XPhos Pd G2, SPhos Pd G2, RuPhos Pd G2, BrettPhos Pd G2, tBuXPhos Pd G2, CPhos Pd G2, DavePhos Pd G2, APhos Pd G2, $(tBu)_2PPh$ Pd G2, MorDalPhos Pd G2, $PCy_3$ Pd G2, $P(tBu)_3$ Pd G2, cataCXium A Pd G2, XantPhos Pd G2, etc.), third-generation Buchwald precatalysts (G3) (XPhos Pd G3, SPhos Pd G3, RuPhos Pd G3, BrettPhos Pd G3, tBuXPhos Pd G3, CPhos Pd G3, DavePhos Pd G3, RockPhos Pd G3, $Me_4$tBuXPhos Pd G3, tBuBrettPhos Pd G3, AdBrettPhos Pd G3, APhos Pd G3, $(tBu)_2PPh$ Pd G3, MorDalPhos Pd G3, $PCy_3$ Pd G3, $P(tBu)_3$ Pd G3, cataCXium A Pd G3, (tBu)zPMe Pd G3, neopentyl (tBu)zP Pd G3, dppf Pd G3, dtbpf Pd G3, BINAP Pd G3, XantPhos Pd G3, Josiphos Pd G3, etc.), fourth-generation Buchwald precatalysts (G4) (XPhos Pd G4, SPhos Pd G4, RuPhos Pd G4, BrettPhos Pd G4, tBuXPhos Pd G4, CPhos Pd G4, tBuBrettPhos Pd G4, APhos Pd G4, $PCy_3$ Pd G4, $P(tBu)_3$ Pd G4, cataCXium A Pd G4, dppf Pd G4, BINAP Pd G4, XantPhos Pd G4, etc.), dichloropalladium(II) complexes ($Pd(PPh_3)_2c1_2$, $Pd(PCy_3)_2c1_2$, $Pd(dppf)Cl_2$, $Pd(dtbpf)Cl_2$, $(APhos)_2PdCl_2$, etc.), palladium(0) complexes (tetrakistriphenylphosphine palladium(0), bis(tricyclohexylphosphine)palladium(0), bis(tri-tert-butylphosphine)palladium(0), etc.), palladium(I) halide complexes (bromo(tri-tert-butylphosphine)palladium(I) dimers, iodo(tri-tert-butylphosphine)palladium(I) dimers, etc.), phosphine $\pi$-allylpalladium catalysts (XPhos Pd(crotyl)Cl (chloro(crotyl)(2-dicyclohexylphosphino-2',4',6'-triisopropyl-1,1'- biphenyl)palladium(II)), SPhos Pd(crotyl)Cl (chloro(crotyl)(2-dicyclohexylphosphino-2',6'- dimethoxy-1,1'-biphenyl)palladium(II)), RuPhos Pd(crotyl)Cl (chloro(crotyl)(2- dicyclohexylphosphino-2',6'-diisopropoxy-1,1'-biphenyl)palladium(II)), [BrettPhos Pd(crotyl)]OTf (crotyl(2-dicyclohexylphosphino-2',4',6'-triisopropyl-3,6-dimethoxy-1,1'- biphenyl)palladium(II) triflate), [tBuXPhos Pd(allyl)]OTf (allyl(2-di-tert-butylphosphino-2',4',6'-triisopropyl-1,1'-biphenyl)palladium(II) triflate), [tBuBrettPhos Pd(allyl)]OTf (allyl(2- di-tert-butylphosphino-3,6-dimethoxy-2',4',6'-triisopropyl-1,1'-biphenyl)palladium(II) triflate), AmPhos Pd(crotyl)Cl (chloro(crotyl)[(p-dimethylaminophenyl)(di-tert- butylphosphine)]palladium(II)), $P(Cy)_3$ Pd(crotyl)Cl (chloro(crotyl)(tricyclohexylphosphine)palladium(II)), $[P(tBu)_3]$ Pd(crotyl)Cl (tri-tert- butylphosphine(chloro)(crotyl)palladium(II)), [BINAP Pd(allyl)]Cl ((R)-BINAP Pd(allyl)]Cl; allyl[(R)-2,2'-bis(diphenylphosphino)-1,1'-binaphthalene]palladium(II) chloride), [XantPhos Pd(allyl)]Cl (allyl[4,5-bis(diphenylphosphino)-9,9-dimethylxanthene]palladium(II) chloride), etc.), PEPPSI catalysts (Pd-PEPPSI IPr, Pd-PEPPSI SIPr ((1,3-bis(2,6- diisopropylphenyl)imidazolidene)(3-chloropyridyl)palladium(II) dichloride), Pd-PEPPSI IPent, etc.), (NHC)Pd(allyl)Cl catalysts (allyl[1,3-bis(2,6-diisopropylphenyl)imidazol-2- ylidene]chloropalladium(II), allyl[1,3-bis(2,6-diisopropylphenyl)-2- imidazolidinylidene]chloropalladium(II), [1,3-bis(2,6-diisopropylphenyl)imidazol-2- ylidene]chloro[3-phenylallyl]palladium(II), etc.), and NHC-Pd naphthoquinone catalysts (1,3-bis(2,6-diisopropylphenyl)imidazol-2-ylidene(1,4-naphthoquinone)palladium(0) dimers, etc.). The number of equivalents of the hydroxyaryl or the alcohol containing B used can be appropriately set by those skilled in the art and can be, for example, 1 to 100 equivalents, 1 to 75 equivalents, 1 to 50 equivalents, 1 to 30 equivalents, or 1 to 10 equivalents, based on A-X.

The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X.

The number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X.

The number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X.

The number of equivalents of the precursor of the palladium catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X.

After the completion of the reaction, the target compound can be obtained by a usual aftertreatment method.

When B in the formula contains a solid-phase carrier and A-X is aryl halide or an analog thereof, the reactive functional group X linked to A is halogen or a hydroxy group derivative. The formation of the ether bond as the linker through the reaction thereof with hydroxyaryl or alcohol wherein the reactive functional group Y linked to B is a hydroxy group using a base can be performed using the already mentioned reagent and reaction conditions. In this context, in the case of using a base, the number of equivalents thereof can be appropriately set by those skilled in the art and can be, for example, 1 to 100 equivalents, 1 to 75 equivalents, 1 to 50 equivalents, 1 to 30 equivalents, or 1 to 10 equivalents, based on the hydroxyaryl or the alcohol containing B. The number of equivalents of A-X used can be appropriately set by those skilled in the art and can be, for example, 1 to 100 equivalents, 1 to 75 equivalents, 1 to 50 equivalents, 1 to 30 equivalents, or 1 to 10 equivalents, based on the hydroxyaryl or the alcohol containing B. In the case of using a metal catalyst, the number of equivalents thereof can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the hydroxyaryl or the alcohol containing B. In the case of using a ligand, the number of equivalents thereof can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the hydroxyaryl or the alcohol containing B. In the case of using a catalyst precursor, the number of equivalents thereof can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the hydroxyaryl or the alcohol containing B.

When A in the formula contains a solid-phase carrier and A-X is hydroxyaryl or alcohol, the reactive functional group X linked to A is a hydroxy group. The ether bond as the linker can be formed through the reaction thereof with alkyl halide wherein the reactive functional group Y linked to B is halogen or an alkylating agent wherein Y is a hydroxy group derivative using a base. In this context, the base is not particularly limited as long as the base can be usually used. Examples of the base that can be used include pyridine derivatives such as pyridine, 2,6-lutidine, 2,4,6-collidine, DTBP, and DTBMP, tertiary amine such as triethylamine, DIPEA, and 4-methylmorpholine, sodium carbonate, potassium carbonate, cesium carbonate, sodium bicarbonate, potassium bicarbonate, lithium hydroxide, sodium hydroxide, potassium hydroxide, tetraalkylammonium hydroxide (tetramethylammonium hydroxide, tetrabutylammonium hydroxide, etc.), sodium hydride, potassium hydride, DBU, DBN, MTBD, BTMG, 1,8-bis(dimethylamino)naphthalene, BEMP, BTPP, P2Et, P2tBu, potassium phosphate, LiOtBu, NaOtBu, KOtBu, LiHMDS, NaHMDS, KHMDS, HMDS, triethylamine, DIPEA, 4-methylmorpholine, lithium hydride, sodium amide, lithium amide, sodium phosphate, potassium phosphate, $KHCO_3$, $Na_2HPO_4$, sodium tetraborate, potassium t-pentoxide, sodium t-pentoxide, lithium t-pentoxide, lithium diisopropylamide, lithium dicyclohexylamide, 2,2,6,6-tetramethylpiperidinyl lithium, 2,2,6,6- tetramethylpiperidinyl magnesium chloride-lithium chloride complexes, N,N-dimethylaniline, P1-tBu, P2tBu, butyllithium, methyllithium, tetrabutylammonium fluoride, cesium fluoride, silver oxide, and silver carbonate.

A solvent can be used in the reaction. Examples of the solvent that can be used include, but are not particularly limited to, halogen solvents such as DCM, chloroform, DCE, and carbon tetrachloride, ether solvents such as diethyl ether, diisopropyl ether, THF (tetrahydrofuran), 2-methyltetrahydrofuran, 4-methyltetrahydropyran, 1,4-dioxane, DME, TBME, CPME, diisopropyl ether, isosorbide dimethyl ether, diglyme, triglyme, and tetraglyme, ketone solvents such as acetone, MEK, MIBK, 4-methyl-2-pentanone, cyclopentanone, cyclohexanone, and diethyl ketone, urea solvents such

as TMU, DMI, and DMPU, amide solvents such as DMF, DMA, NFM, NMP, NEP, and NBP, sulfoxide solvents such as DMSO, ester solvents such as methyl acetate, ethyl acetate, propyl acetate, isopropyl acetate, butyl acetate, isobutyl acetate, GVL (γ-valerolactone), pentyl acetate, and methyl propionate, sulfone solvents such as diphenylsulfone, dimethylsulfone, diethylsulfone, sulfolane, 3-methylsulfolane, ethylmethylsulfone, and ethylisopropylsulfone, aromatic solvents such as anisole, toluene, xylene, chlorobenzene, bromobenzene, ethylbenzene, nitrobenzene, α,α,α-trifluorotoluene, 1,2-dichlorobenzene, benzene, and pyridine, carbonate solvents such as dimethyl carbonate, diethyl carbonate, ethylene carbonate, and propylene carbonate, and others such as acetonitrile and limonene. A single solvent may be used, or a combined solvent of a plurality of solvents may be used. The reaction temperature can be appropriately set by those skilled in the art and can be, for example, 0 to 120°C, 20 to 100°C, or 20 to 60°C. If necessary, an additive may be used. Examples of the additive that can be used include AgzO, 12-crown-4, 15-crown-5, 18-crown-6, HMPA, TMEDA, tetrabutylammonium chloride, tetrabutylammonium bromide, tetrabutylammonium iodide, and tetrabutylammonium bisulfate. The number of equivalents of the alkyl halide containing B used or the alkylating agent which is a hydroxy group derivative can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The number of equivalents of the additive used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier and A-X is hydroxyaryl or alcohol, the reactive functional group X linked to A is a hydroxy group. The formation of the ether bond as the linker through the reaction thereof with alkyl halide wherein the reactive functional group Y linked to B is halogen or an alkylating agent wherein Y is a hydroxy group derivative using a base can be performed using the already mentioned reagent and reaction conditions. In this context, in the case of using a base, the number of equivalents thereof can be appropriately set by those skilled in the art and can be, for example, 1 to 100 equivalents, 1 to 75 equivalents, 1 to 50 equivalents, 1 to 30 equivalents, or 1 to 10 equivalents, based on the alkyl halide or the alkylating agent containing B. The number of equivalents of A-X used can be appropriately set by those skilled in the art and can be, for example, 1 to 100 equivalents, 1 to 75 equivalents, 1 to 50 equivalents, 1 to 30 equivalents, or 1 to 10 equivalents, based on the alkyl halide or the alkylating agent containing B.

[Formula 21]

Carbon-carbon single bond formation reaction

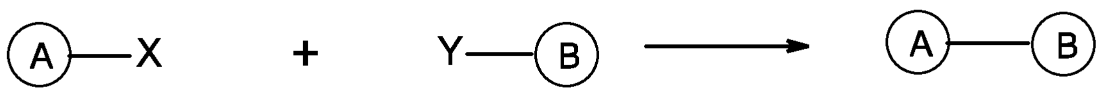

The carbon-carbon single bond as the linker can be formed, for example, through the reaction of aryl halide with a boron derivative or a zinc derivative.

When A in the formula contains a solid-phase carrier, the reactive functional group X linked to A is halogen or $-O-SO_2-R^5$ wherein $R^5$ is $C_{1-6}$ alkyl optionally substituted by one or more fluorine atoms, or phenyl optionally substituted by one or more fluorine atoms or $C_{1-6}$ alkyl optionally substituted by fluorine atom(s). The carbon-carbon single bond as the linker can be formed through the reaction thereof with a boronic acid analog wherein the reactive functional group Y linked to B is a boron derivative or an alkyl borane reagent derived from a carbon-carbon terminal double bond using a metal catalyst. The number of equivalents of the boronic acid derivative or the alkyl borane reagent used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. In this context, the metal catalyst is not particularly limited as long as the metal catalyst can be usually used. Examples of the metal catalyst that can be used include palladium acetate, $Pd(dba)_2$, $Pd_2(dba)_3$, $Pd_2(dba)_3CHCl_3$, allylpalladium chloride dimers, palladium(π-cinnamyl) chloride dimers, (1-methylallyl)palladium chloride dimers, and (1,5- cyclooctadiene)bis(trimethylsilylmethyl)palladium(II). If necessary, a ligand may be used. Examples of the ligand that can be used include trialkylphosphine ($PCy_3$ (tricyclohexylphosphine), $P(tBu)_3$ (tri-tert-butylphosphine), cataCXium A (di(1-adamantyl)- n-butylphosphine), $(tBu)_2PMe$ (di-tert-butylmethylphosphine), $P(Ad)_3$, neopentyl (tBu)zP (di- tert-butylneopentylphosphine), etc.), triarylphosphine ($PPh_3$ (triphe-

nylphosphine), etc.), dialkylbiarylphosphine (XPhos (2-dicyclohexylphosphino-2',4',6'-triisopropylbiphenyl), SPhos (2-dicyclohexylphosphino-2',6'-dimethoxybiphenyl), RuPhos (2- dicyclohexylphosphino-2',6'-diisopropoxybiphenyl), BrettPhos (2-(dicyclohexylphosphino)- 3,6-dimethoxy-2',4',6'-triisopropyl-1,1'-biphenyl), tBuXPhos (2-di-tert-butylphosphino- 2',4',6'-triisopropylbiphenyl), CPhos (2-dicyclohexylphosphino-2',6'-bis(N,N- dimethylamino)biphenyl), DavePhos (2-dicyclohexylphosphino-2'-(N,N- dimethylamino)biphenyl), RockPhos (2-di(tertiary butyl)phosphino-2',4',6'-triisopropyl-3- methoxy-6-methylbiphenyl, di-tertiary butyl(2',4',6'-triisopropyl-3-methoxy-6-methyl-[1,1'- biphenyl]-2-yl)phosphine), $Me_4$tBuXPhos (2-di-tert-butylphosphino-3,4,5,6-tetramethyl- 2',4',6'-triisopropyl-1,1'-biphenyl), tBuBrettPhos (2-(di-tert-butylphosphino)-2',4',6'- triisopropyl-3,6-dimethoxy-1,1'-biphenyl), AdBrettPhos (2-(di-1 -adamantylphosphino)- 2',4',6'-triisopropyl-3,6-dimethoxy-1,1'-biphenyl), etc.), dialkylmonoarylphosphine (APhos ((4-(N,N-dimethylamino)phenyl)di-tert-butylphosphine), $(tBu)_2$PPh, MorDalPhos (2- morpholinophenyldi(1-adamantyl)phosphine), etc.), bidentate phosphine ligands (dppf (1,1'- bis(diphenylphosphino)ferrocene), dtbpf (1,1'-bis(di-tert-butylphosphino)ferrocene), BINAP (2,2'-bis(diphenylphosphino)-1,1'-binaphthyl), XantPhos (4,5-bis(diphenylphosphino)-9,9- dimethylxanthene), Josiphos ((R)-1-[(Sp)-2-(dicyclohexylphosphino)ferrocenyl]ethyldi-tert- butylphosphine), etc.), and NHC ligands (IPr (1,3-bis(2,6-diisopropylphenyl)imidazol-2- ylidene), SIPr (1,3-bis(2,6-di-i-propylphenyl)imidazolidin-2-ylidene), IMes (1,3-bis(2,4,6- trimethylphenyl)imidazol-2-ylidene), IPent (1,3-bis(2,6-di-3-pentylphenyl)imidazol-2- ylidene), etc.). Alternatively, the ligand described above and a precursor of the palladium catalyst may be used. Examples of the precursor of the palladium catalyst that can be used include first-generation Buchwald precatalysts (G1) (XPhos Pd G1, SPhos Pd G1, RuPhos Pd G1, BrettPhos Pd G1, tBuXPhos Pd G1, etc.), second-generation Buchwald precatalysts (G2) (XPhos Pd G2, SPhos Pd G2, RuPhos Pd G2, BrettPhos Pd G2, tBuXPhos Pd G2, CPhos Pd G2, DavePhos Pd G2, APhos Pd G2, $tBu)_2$PPh Pd G2, MorDalPhos Pd G2, $PCy_3$ Pd G2, $P(tBu)_3$ Pd G2, cataCXium A Pd G2, XantPhos Pd G2, etc.), third-generation Buchwald precatalysts (G3) (XPhos Pd G3, SPhos Pd G3, RuPhos Pd G3, BrettPhos Pd G3, tBuXPhos Pd G3, CPhos Pd G3, DavePhos Pd G3, RockPhos Pd G3, $Me_4$tBuXPhos Pd G3, tBuBrettPhos Pd G3, AdBrettPhos Pd G3, APhos Pd G3, $(tBu)_2$PPh Pd G3, MorDalPhos Pd G3, $PCy_3$ Pd G3, $P(tBu)_3$ Pd G3, cataCXium A Pd G3, $tBu)_2$PMe Pd G3, neopentyl (tBu)zP Pd G3, dppf Pd G3, dtbpf Pd G3, BINAP Pd G3, XantPhos Pd G3, Josiphos Pd G3, etc.), fourth-generation Buchwald precatalysts (G4) (XPhos Pd G4, SPhos Pd G4, RuPhos Pd G4, BrettPhos Pd G4, tBuXPhos Pd G4, CPhos Pd G4, tBuBrettPhos Pd G4, APhos Pd G4, $PCy_3$ Pd G4, $P(tBu)_3$ Pd G4, cataCXium A Pd G4, dppf Pd G4, BINAP Pd G4, XantPhos Pd G4, etc.), dichloropalladium(II) complexes ($Pd(PPh_3)_2c1_2$, $Pd(PCy_3)_2c1_2$, $Pd(dppf)Cl_2$, $Pd(dtbpf)Cl_2$, $(APhos)_2PdCl_2$, etc.), palladium(0) complexes (tetrakistriphenylphosphine palladium(0), bis(tricyclohexylphosphine)palladium(0), bis(tri-tert- butylphosphine)palladium(0), etc.), palladium(I) halide complexes (bromo(tri-tert-butylphosphine)palladium(I) dimers, iodo(tri-tert-butylphosphine)palladium(I) dimers, etc.), phosphine $\pi$-allylpalladium catalysts (XPhos Pd(crotyl)Cl (chloro(crotyl)(2- dicyclohexylphosphino-2',4',6'-triisopropyl-1,1'-biphenyl)palladium(II)), SPhos Pd(crotyl)Cl (chloro(crotyl)(2-dicyclohexylphosphino-2',6'-dimethoxy-1,1'-biphenyl)palladium(II)), RuPhos Pd(crotyl)Cl (chloro(crotyl)(2-dicyclohexylphosphino-2',6'-diisopropoxy-1,1'- biphenyl)palladium(II)), [BrettPhos Pd(crotyl)]OTf (crotyl(2-dicyclohexylphosphino-2',4',6'- triisopropyl-3,6-dimethoxy-1,1'-biphenyl)palladium(II) triflate), [tBuXPhos Pd(allyl)]OTf (allyl(2-di-tert-butylphosphino-2',4',6'-triisopropyl-1,1'-biphenyl)palladium(II) triflate), [tBuBrettPhos Pd(allyl)]OTf (allyl(2-di-tert-butylphosphino-3,6-dimethoxy-2',4',6'- triisopropyl-1,1'-biphenyl)palladium(II) triflate), AmPhos Pd(crotyl)Cl (chloro(crotyl)[(p- dimethylaminophenyl)(di-tert-butylphosphine)]palladium(II)), $P(Cy)_3$ Pd(crotyl)Cl (chloro(crotyl)(tricyclohexylphosphine)palladium(II)), $[P(tBu)_3]$ Pd(crotyl)Cl (tri-tert- butylphosphine(chloro)(crotyl)palladium(II)), [BINAP Pd(allyl)]Cl ((R)-BINAP Pd(allyl)]Cl; allyl[(R)-2,2'-bis(diphenylphosphino)-1,1'-binaphthalene]palladium(II) chloride), [XantPhos Pd(allyl)]Cl (allyl[4,5-bis(diphenylphosphino)-9,9-dimethylxanthene]palladium(II) chloride), etc.), PEPPSI catalysts (Pd-PEPPSI IPr, Pd-PEPPSI SIPr ((1,3-bis(2,6- diisopropylphenyl)imidazolidene)(3-chloropyridyl)palladium(II) dichloride), Pd-PEPPSI IPent (1,3-bis(2,6-di-3-pentylphenyl)imidazol-2-ylidene), etc.), (NHC)Pd(allyl)Cl catalysts (allyl[1,3-bis(2,6-diisopropylphenyl)imidazol-2-ylidene]chloropalladium(II), allyl[1,3- bis(2,6-diisopropylphenyl)-2-imidazolidinylidene]chloropalladium(II), [1,3-bis(2,6- diisopropylphenyl)imidazol-2-ylidene]chloro[3-phenylallyl]palladium(II), etc.), and NHC-Pd naphthoquinone catalysts (1,3-bis(2,6-diisopropylphenyl)imidazol-2-ylidene(1,4- naphthoquinone)palladium(0) dimers, etc.). The number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The number of equivalents of the catalyst precursor used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents

(TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL ($\gamma$-valcrolactonc), pentyl acetate, methyl propionate, etc.), aromatic hydrocarbon solvents (anisole, toluene, xylene, chlorobenzene, bromobenzene, ethylbenzene, nitrobenzene, $\alpha,\alpha,\alpha$- trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. A single solvent may be used, or a combined solvent of a plurality of solvents may be used. The reaction temperature can be appropriately set by those skilled in the art and can be, for example, 0 to 200°C, 0 to 140°C, 10 to 100°C, or 20 to 60°C. If necessary, a base may be used. Examples of the base that can be used include sodium carbonate, potassium carbonate, cesium carbonate, sodium phosphate, potassium phosphate, sodium hydroxide, potassium hydroxide, cesium hydroxide, barium hydroxide, tetraalkylammonium hydroxide (tetramethylammonium hydroxide, tetrabutylammonium hydroxide, etc.), NaOtBu, KOt-Bu, sodium fluoride, potassium fluoride, cesium fluoride, triethylamine, DIPEA, DBU, DBN, MTBD, TMG, BTMG, P1tBu, BEMP, BTPP, P2Et, P2tBu, sodium tetraborate, 1,8- bis(dimethylamino)naphthalene, potassium t-pentoxide, sodium t-pentoxide, lithium t- pentoxide, tetrabutylammonium fluoride, and cesium fluoride. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

In the formula, the reactive functional group X linked to A is halogen or $-O-SO_2-R^5$ wherein $R^5$ is $C_{1-6}$ alkyl optionally substituted by one or more fluorine atoms, or phenyl optionally substituted by one or more fluorine atoms or $C_{1-6}$ alkyl optionally substituted by fluorine atom(s). The carbon-carbon single bond as the linker can be formed through the reaction thereof with a compound wherein the reactive functional group Y linked to B is a zinc derivative using a metal catalyst. The number of equivalents of the zinc derivative used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. In this context, the metal catalyst is not particularly limited as long as the metal catalyst can be usually used. Examples of the metal catalyst that can be used include palladium acetate, $Pd(dba)_2$, $Pd_2(dba)_3$, $Pd_2(dba)_3CHCl_3$, allylpalladium chloride dimers, palladium($\pi$-cinnamyl) chloride dimers, (1-methylallyl)palladium chloride dimers, and (1,5-cyclooctadiene)bis(trimethylsilylmethyl)palladium(II). If necessary, a ligand may be used. Examples of the ligand that can be used include trialkylphosphine ($PCy_3$ (tricyclohexylphosphine), $P(tBu)_3$ (tri-tert-butylphosphine), cataCXium A (di(1-adamantyl)- n-butylphosphine), $(tBu)_2PMe$ (di-tert-butylmethylphosphine), $P(Ad)_3$ (tri(1- adamantyl)phosphine), neopentyl $(tBu)_2P$ (di-tert-butylneopentylphosphine), etc.), triarylphosphine ($PPh_3$ (triphenylphosphine), etc.), dialkylbiarylphosphine (XPhos (2- dicyclohexylphosphino-2',4',6'-triisopropylbiphenyl), SPhos (2-dicyclohexylphosphino-2',6'- dimethoxybiphenyl), RuPhos (2-dicyclohexylphosphino-2',6'-diisopropoxybiphenyl), BrettPhos (2-(dicyclohexylphosphino)-3,6-dimethoxy-2',4',6'-triisopropyl-1,1'-biphenyl), tBuXPhos (2-di-tert-butylphosphino-2',4',6'-triisopropylbiphenyl), CPhos (2- dicyclohexylphosphino-2',6'-bis(N,N-dimethylamino)biphenyl), DavePhos (2- dicyclohexylphosphino-2'-(N,N-dimethylamino)biphenyl), RockPhos (2-di(tertiary butyl)phosphino-2',4',6'-triisopropyl-3-methoxy-6-methylbiphenyl, di-tertiary butyl(2',4',6'- triisopropyl-3-methoxy-6-methyl-[1,1'-biphenyl]-2-yl)phosphine), $Me_4$tBuXPhos (2-di-tert- butylphosphino-3,4,5,6-tetramethyl-2',4',6'-triisopropyl-1,1'-biphenyl), tBuBrettPhos (2-(di- tert-butylphosphino)-2',4',6'-triisopropyl-3,6-dimethoxy-1,1'-biphenyl), AdBrettPhos (2-(di- 1-adamantylphosphino)-2',4',6'-triisopropyl-3,6-dimethoxy-1,1'-biphenyl), etc.), dialkylmonoarylphosphine (APhos ((4-(N,N-dimethylamino)phenyl)di-tert-butylphosphine), $(tBu)_2PPh$ (di-tert-butylphenylphosphine), MorDalPhos (2-morpholinophenyldi(1- adamantyl)phosphine), etc.), bidentate phosphine ligands (dppf (1,1'- bis(diphenylphosphino)ferrocene), dtbpf (1,1'-bis(di-tert-butylphosphino)ferrocene), BINAP (2,2'-bis(diphenylphosphino)-1,1'-binaphthyl), XantPhos (4,5-bis(diphenylphosphino)-9,9-dimethylxanthene), Josiphos ((R)-1-[(Sp)-2-(dicyclohexylphosphino)ferrocenyl]ethyldi-tert- butylphosphine), etc.), and NHC ligands (IPr (1,3-bis(2,6-diisopropylphenyl)imidazol-2- ylidene), SIPr (1,3-bis(2,6-di-i-propylphenyl)imidazolidin-2-ylidene), IMes (1,3-bis(2,4,6- trimethylphenyl)imidazol-2-ylidene), IPent (1,3-bis(2,6-di-3-pentylphenyl)imidazol-2- ylidene), etc.). Alternatively, the ligand described above and a precursor of the palladium catalyst may be used. Examples of the precursor of the palladium catalyst that can be used include first-generation Buchwald precatalysts (G1) (XPhos Pd G1, SPhos Pd G1, RuPhos Pd G1, BrettPhos Pd G1, tBuXPhos Pd G1, etc.), second-generation Buchwald precatalysts (G2) (XPhos Pd G2, SPhos Pd G2, RuPhos Pd G2, BrettPhos Pd G2, tBuXPhos Pd G2, CPhos Pd G2, DavePhos Pd G2, APhos Pd G2, $(tBu)_2PPh$ Pd G2, MorDalPhos Pd G2, $PCy_3$ Pd G2, $P(tBu)_3$ Pd G2, cataCXium A Pd G2, XantPhos

Pd G2, etc.), third-generation Buchwald precatalysts (G3) (XPhos Pd G3, SPhos Pd G3, RuPhos Pd G3, BrettPhos Pd G3, tBuXPhos Pd G3, CPhos Pd G3, DavePhos Pd G3, RockPhos Pd G3, $Me_4$tBuXPhos Pd G3, tBuBrettPhos Pd G3, AdBrettPhos Pd G3, APhos Pd G3, $(tBu)_2$PPh Pd G3, MorDalPhos Pd G3, $PCy_3$ Pd G3, $P(tBu)_3$ Pd G3, cataCXium A Pd G3, (tBu)2PMe Pd G3, neopentyl (tBu)zP Pd G3, dppf Pd G3, dtbpf Pd G3, BINAP Pd G3, XantPhos Pd G3, Josiphos Pd G3, etc.), fourth-generation Buchwald precatalysts (G4) (XPhos Pd G4, SPhos Pd G4, RuPhos Pd G4, BrettPhos Pd G4, tBuXPhos Pd G4, CPhos Pd G4, tBuBrettPhos Pd G4, APhos Pd G4, $PCy_3$ Pd G4, $P(tBu)_3$ Pd G4, cataCXium A Pd G4, dppf Pd G4, BINAP Pd G4, XantPhos Pd G4, etc.), dichloropalladium(II) complexes ($Pd(PPh_3)_2c1_2$, $Pd(PCy_3)_2c1_2$, Pd(dppf)$Cl_2$, Pd(dtbpf)$Cl_2$, (APhos)zPdClz, etc.), palladium(0) complexes (tetrakistriphenylphosphine palladium(0), bis(tricyclohexylphosphine)palladium(0), bis(tri-tert- butylphosphine)palladium(0), etc.), palladium(I) halide complexes (bromo(tri-tert- butylphosphine)palladium(I) dimers, iodo(tri-tert-butylphosphine)palladium(I) dimers, etc.), phosphine π-allylpalladium catalysts (XPhos Pd(crotyl)Cl (chloro(crotyl)(2- dicyclohexylphosphino-2',4',6'-triisopropyl-1,1'-biphenyl)palladium(II)), SPhos Pd(crotyl)Cl (chloro(crotyl)(2-dicyclohexylphosphino-2',6'-dimethoxy-1,1'-biphenyl)palladium(II)), RuPhos Pd(crotyl)Cl (chloro(crotyl)(2-dicyclohexylphosphino-2',6'-diisopropoxy-1,1'- biphenyl)palladium(II)), [BrettPhos Pd(crotyl)]OTf (crotyl(2-dicyclohexylphosphino-2',4',6'- triisopropyl-3,6-dimethoxy-1,1'-biphenyl)palladium(II) triflate), [tBuXPhos Pd(allyl)]OTf (allyl(2-di-tert-butylphosphino-2',4',6'-triisopropyl-1,1'-biphenyl)palladium(II) triflate), [tBuBrettPhos Pd(allyl)]OTf (allyl(2-di-tert-butylphosphino-3,6-dimethoxy-2',4',6'- triisopropyl-1,1'-biphenyl)palladium(II) triflate), AmPhos Pd(crotyl)Cl (chloro(crotyl)[(p- dimethylaminophenyl)(di-tert-butylphosphine)]palladium(II)), $P(Cy)_3$ Pd(crotyl)Cl (chloro(crotyl)(tricyclohexylphosphine)palladium(II)), $[P(tBu)_3]$ Pd(crotyl)Cl (tri-tert- butylphosphine(chloro)(crotyl)palladium(II)), [BINAP Pd(allyl)]Cl ((R)-BINAP Pd(allyl)]Cl; allyl[(R)-2,2'-bis(diphenylphosphino)-1,1'-binaphthalene]palladium(II) chloride), [XantPhos Pd(allyl)]Cl (allyl [4,5-bis(diphenylphosphino)-9,9-dimethylxanthene]palladium(II) chloride), etc.), PEPPSI catalysts (Pd-PEPPSI IPr, Pd-PEPPSI SIPr ((1,3-bis(2,6-diisopropylphenyl)imidazolidene)(3-chloropyridyl)palladium(II) dichloride), Pd-PEPPSI IPent, etc.), (NHC)Pd(allyl)Cl catalysts (allyl[1,3-bis(2,6-diisopropylphenyl)imidazol-2- ylidene]chloropalladium(II), allyl[1,3-bis(2,6-diisopropylphenyl)-2-imidazolidinylidene]chloropalladium(II), [1,3-bis(2,6-diisopropylphenyl)imidazol-2- ylidene]chloro[3-phenylallyl]palladium(II), etc.), and NHC-Pd naphthoquinone catalysts (1,3-bis(2,6-diisopropylphenyl)imidazol-2-ylidene(1,4-naphthoquinone)palladium(0) dimers, etc.). The number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The number of equivalents of the catalyst precursor used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL (γ-valerolactone), pentyl acetate, methyl propionate, etc.), aromatic hydrocarbon solvents (anisole, toluene, xylene, chlorobenzene, bromobenzene, ethylbenzene, nitrobenzene, α,α,α- trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. A single solvent may be used, or a combined solvent of a plurality of solvents may be used. The reaction temperature can be appropriately set by those skilled in the art and can be, for example, -40 to 100°C, -20 to 70°C, or 20 to 60°C. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

However, it has been found that depending on the type of the palladium catalyst mentioned above, cleavage from the solid phase also progresses in applying carbon-carbon single bond formation reaction to the production of a compound library. A low reactive substrate among substrates contained on the solid-phase side and/or a low reactive

reagent may require a long reaction time for the completion of the reaction, and the progression of cleavage from the solid phase may largely influence reduction in yield in solid-phase reaction. In order to solve this problem, the present inventors have studied conditions under which carbon-carbon single bond formation reaction progresses without causing the progression of cleavage from the solid phase, and consequently found that in the case of using meCgPPh Pd G4 or (dtbpf)$PdCl_2$ as a palladium catalyst, Suzuki coupling reaction can be performed at high yields without cleavage from the solid phase. Use of the conditions in the synthesis of a compound library can suppress cleavage from the solid phase and can achieve high yields, even if the reaction is delayed due to some factor. This method enables robust reaction and improved yields for the purpose of supplying a "high-quality" mixture library.

[Formula 22]

Ethyne-1,2-diyl group formation reaction

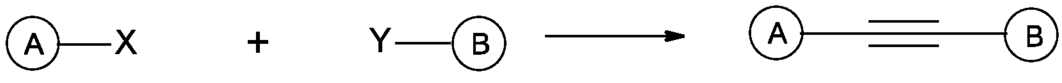

The ethyne-1,2-diyl bond as the linker can be formed through the reaction of aryl halide with an acetylene derivative.

When A in the formula contains a solid-phase carrier, the reactive functional group X linked to A is halogen. The ethyne-1,2-diyl group as the linker can be formed through the reaction thereof with a compound wherein the reactive functional group Y linked to B is an acetylene derivative using a metal catalyst. The number of equivalents of the acetylene derivative used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. In this context, the metal catalyst is not particularly limited as long as the metal catalyst can be usually used. Examples of the metal catalyst that can be used include palladium acetate, $Pd(dba)_2$, $Pd_2(dba)_3$, $Pd_2(dba)_3CHCl_3$, allylpalladium chloride dimers, palladium($\pi$-cinnamyl) chloride dimers, (1-methylallyl)palladium chloride dimers, (1,5-cyclooctadiene)bis(trimethylsilylmethyl)palladium(II), copper, for example, copper(I) oxide (CuzO), copper(II) oxide, copper(I) iodide (CuI), copper(I) bromide (CuBr), copper(I) chloride (CuCl), copper acetate ($Cu(OAc)_2$), copper sulfate, Ullmann $\eta$-type conditions (e.g., metal catalysts such as copper(II) acetate in a aprotic solvent such as DMF), and Buchwald- type conditions (e.g., alkali metal carbonate such as cesium carbonate; BINAP; and palladium catalysts such as $Pd_2(dba)_3$ and $Pd(OAc)_2$). The number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL ($\gamma$-valeolactone), pentyl acetate, methyl propionate, etc.), aromatic hydrocarbon solvents (anisole, toluene, xylene, chlorobenzene, bromobenzene, ethylbenzene, nitrobenzene, $\alpha,\alpha,\alpha$- trifluorotoluene, 1,2-dichlorobenzene, benzene, tetralin, pyridine, quinoline, etc.), alcohol solvents (ethanol, isopropanol, tert-butanol, tert-amyl alcohol, triethanolamine, etc.), hydrocarbon solvents (hexane, pentane, heptane, cyclohexane, methylcyclohexane, etc.), and others such as acetonitrile, water, silene, and limonene. A single solvent may be used, or a combined solvent of a plurality of solvents may be used. The reaction temperature can be appropriately set by those skilled in the art and can be, for example, 0 to 140°C, 5 to 120°C, 10 to 100°C or 20 to 80°C. If necessary, a ligand may be used. Examples of the ligand for palladium that can be used include trialkylphosphine ($PCy_3$ (tricyclohexylphosphine), $P(tBu)_3$ (tri-tert-butylphosphine), cataCXium A (di(1-adamantyl)-n-butylphosphine), $(tBu)_2PMe$ (di- tert-butylmethylphosphine), $P(Ad)_3$ (tri(1-adamantyl)phosphine), neopentyl (tBu)zP, etc.), triarylphosphine ($PPh_3$ (triphenylphosphine), etc.), dialkylbiarylphosphine (XPhos, SPhos (2- dicyclohexylphosphino-2',6'-dimethoxybiphenyl), RuPhos (2-dicyclohexylphosphino-2',6'- diisopropoxybiphenyl), BrettPhos (2-(dicyclohexylphosphino)-3,6-dimethoxy-2',4',6'- triisopropyl-1,1'-biphenyl), tBuXPhos (2-di-tert-butylphosphino-2',4',6'-triisopropylbiphenyl), CPhos (2-dicyclohexylphosphino-2',6'-bis(N,N-dimethylamino)biphenyl), DavePhos (2- dicyclohexylphosphino-2'-(N,N-dimethylamino)biphenyl), RockPhos (2-di(tertiary butyl)phosphino-2',4',6'-triisopropyl-3-methoxy-6-methylbiphenyl, di-tertiary butyl(2',4',6'- triisopropyl-3-methoxy-6-methyl-[1,1'-biphenyl]-2-yl)phosphine), $Me_4$tBuXPhos (2-di-tert- butyl-phosphino-3,4,5,6-tetramethyl-2',4',6'-triisopropyl-1,1'-biphenyl), tBuBrettPhos (2-(di- tert-butylphosphino)-2',4',6'-triisopropyl-3,6-dimethoxy-1,1'-biphenyl), AdBrettPhos (2-(di- 1-adamantylphosphino)-2',4',6'-triisopropyl-3,6-dimethoxy-1,1'-biphenyl), BippyPhos (5-(di- tert-butylphosphino)-1',3',5'-triphenyl-1'H-[1,4']bipyrazole), AdBippyPhos (5-[di(1- adamantyl)phosphino]-1',3',5'-triphenyl-1'H-[1,4']bipyrazole), etc.), dialkylmonoarylphosphine (APhos ((4-(N,N-dimethylamino)phenyl)di-tert-butylphosphine), $(tBu)_2$PPh (di-tert-butylphenylphosphine), MorDalPhos (2-morpholinophenyldi(1-adamantyl)phosphine), etc.), bidentate phosphine ligands (dppf (1,1'- bis(diphenylphosphino)ferrocene), dtbpf (1,1'-bis(di-tert-butylphosphino)ferrocene), BINAP (2,2'-bis(diphenylphosphino)-1,1'-binaphthyl), XantPhos (4,5-bis(diphenylphosphino)-9,9- dimethylxanthene), Josiphos ((R)-1-[(Sp)-2-(dicyclohexylphosphino)ferrocenyl]ethyldi-tert- butylphosphine), etc.), and NHC ligands (IPr (1,3-bis(2,6-diisopropylphenyl)imidazol-2- ylidene), SIPr (1,3-bis(2,6-di-i-propylphenyl)imidazolidin-2-ylidene), IMes (1,3-bis(2,4,6- trimethylphenyl)imidazol-2-ylidene), IPent (1,3-bis(2,6-di-3-pentylphenyl)imidazol-2- ylidene), etc.). Alternatively, the ligand described above and a precursor of the palladium catalyst may be used. Examples of the precursor of the palladium catalyst that can be used include first-generation Buchwald precatalysts (G1) (XPhos Pd G1, SPhos Pd G1, RuPhos Pd G1, BrettPhos Pd G1, tBuXPhos Pd G1, etc.), second-generation Buchwald precatalysts (G2) (XPhos Pd G2, SPhos Pd G2, RuPhos Pd G2, BrettPhos Pd G2, tBuXPhos Pd G2, CPhos Pd G2, DavePhos Pd G2, APhos Pd G2, $(tBu)_2$PPh Pd G2, MorDalPhos Pd G2, $PCy_3$ Pd G2, $P(tBu)_3$Pd G2, cataCXium A Pd G2, XantPhos Pd G2, etc.), third-generation Buchwald precatalysts (G3) (XPhos Pd G3, SPhos Pd G3, RuPhos Pd G3, BrettPhos Pd G3, tBuXPhos Pd G3, CPhos Pd G3, DavePhos Pd G3, RockPhos Pd G3, $Me_4$tBuXPhos Pd G3, tBuBrettPhos Pd G3, AdBrettPhos Pd G3, APhos Pd G3, $(tBu)_2$PPh Pd G3, MorDalPhos Pd G3, $PCy_3$ Pd G3, $P(tBu)_3$Pd G3, cataCXium A Pd G3, (tBu)2PMe Pd G3, neopentyl (tBu)zP Pd G3, dppf Pd G3, dtbpf Pd G3, BINAP Pd G3, XantPhos Pd G3, Josiphos Pd G3, etc.), fourth-generation Buchwald precatalysts (G4) (XPhos Pd G4, SPhos Pd G4, RuPhos Pd G4, BrettPhos Pd G4, tBuXPhos Pd G4, CPhos Pd G4, tBuBrettPhos Pd G4, APhos Pd G4, $PCy_3$ Pd G4, $P(tBu)_3$ Pd G4, cataCXium A Pd G4, dppf Pd G4, BINAP Pd G4, XantPhos Pd G4, etc.), dichloropalladium(II) complexes ($Pd(PPh_3)_2c1_2$, $Pd(PCy_3)_2c1_2$, $Pd(dppf)Cl_2$, $Pd(dtbpf)Cl_2$, $(APhos)_2PdCl_2$, etc.), palladium(0) complexes (tetrakistriphenylphosphine palladium(0), bis(tricyclohexylphosphine)palladium(0), bis(tri-tert- butylphosphine)palladium(0), etc.), palladium(I) halide complexes (bromo(tri-tert- butylphosphine)palladium(I) dimers, iodo(tri-tert-butylphosphine)palladium(I) dimers, etc.), phosphine $\pi$-allylpalladium catalysts (XPhos Pd(crotyl)Cl (chloro(crotyl)(2- dicyclohexylphosphino-2',4',6'-triisopropyl-1,1'-biphenyl)palladium(II)), SPhos Pd(crotyl)Cl (chloro(crotyl)(2-dicyclohexylphosphino-2',6'-dimethoxy-1,1'-biphenyl)palladium(II)), RuPhos Pd(crotyl)Cl (chloro(crotyl)(2-dicyclohexylphosphino-2',6'-diisopropoxy-1,1'- biphenyl)palladium(II)), [BrettPhos Pd(crotyl)]OTf (crotyl(2-dicyclohexylphosphino-2',4',6'- triisopropyl-3,6-dimethoxy-1,1'-biphenyl)palladium(II) triflate), [tBuXPhos Pd(allyl)]OTf (allyl(2-di-tert-butylphosphino-2',4',6'-triisopropyl-1,1'-biphenyl)palladium(II) triflate), [tBuBrettPhos Pd(allyl)]OTf (allyl(2-di-tert-butylphosphino-3,6-dimethoxy-2',4',6'- triisopropyl-1,1'-biphenyl)palladium(II) triflate), AmPhos Pd(crotyl)Cl (chloro(crotyl)[(p- dimethylaminophenyl)(di-tert-butylphosphine)]palladium(II)), $P(Cy)_3$ Pd(crotyl)Cl (chloro(crotyl)(tricyclohexylphosphine)palladium(II)), $[P(tBu)_3]$ Pd(crotyl)Cl (tri-tert- butylphosphine(chloro)(crotyl)palladium(II)), [BINAP Pd(allyl)]Cl ((R)-BINAP Pd(allyl)]Cl; allyl[(R)-2,2'-bis(diphenylphosphino)-1,1'-binaphthalene]palladium(II) chloride), [XantPhos Pd(allyl)]Cl (allyl[4,5-bis(diphenylphosphino)-9,9-dimethylxanthene]palladium(II) chloride), etc.), PEPPSI catalysts (Pd-PEPPSI IPr, Pd-PEPPSI SIPr, Pd-PEPPSI IPent, etc.), (NHC)Pd(allyl)Cl catalysts (allyl[1,3-bis(2,6-diisopropylphenyl)imidazol-2- ylidene]chloropalladium(II), allyl[1,3-bis(2,6-diisopropylphenyl)-2- imidazolidinylidene]chloropalladium(II), [1,3-bis(2,6-diisopropylphenyl)imidazol-2- ylidene]chloro[3-phenylallyl]palladium(II), etc.), and NHC-Pd naphthoquinone catalysts (1,3-bis(2,6-diisopropylphenyl)imidazol-2-ylidene(1,4-naphthoquinone)palladium(0) dimers, etc.). Examples of the ligand for copper that can be used include diamine ligands, for example, di-tert-butyl bipyridine (dtBBPy (4,4'-di-tert-butyl-2,2'-dipyridyl), etc.), dimethylethylenediamine (DMEDA (N,N'-dimethylethylenediamine), etc.), cyclohexyldimethylethylenediamine (CyDMEDA (trans-N,N'-dimethylcyclohexane-1,2- diamine), etc.), and 1,10-phenanthroline (Phen), amino acid ligands, for example, N,N'-dimethylglycine, sarcosine (N-methylglycine), L-proline, 4-hydroxy-1-proline, and 6- methylpicolinic acid, diamide ligands, for example, $N^1$-(2-methyl-1-naphthalenyl)-$N^2$- (phenylmethyl)ethanediamide, $N^1$,$N^2$-bis(phenylmethyl)ethanediamine, $N^1$,$N^2$-bis(2,6- dimethylphenyl)ethanediamine, $N^1$,$N^2$-bis(2-furanylmethyl)ethanediamine, $N^1$,$N^2$-bis(2,4,6- trimethoxy-phenyl)ethanediamine, $N^1$,$N^2$-bis(5-methyl[1,1'-biphenyl]-2-yl)-ethanediamide, [(2,6-dimethylphenyl)amino](oxo)acetic acid (DMPAO), and N-[1,1'-biphenyl]-2-yl-1H- indole-2-carboxamide, and others such as 2-methyl-8-hydroxyquinoline. The number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. In the case of using a catalyst precursor, the number of equivalents of the catalyst precursor can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. If necessary, a base may be used. Examples of the base that can be used include sodium carbonate, potassium carbonate, cesium carbonate, sodium

bicarbonate, potassium bicarbonate, sodium phosphate, potassium phosphate, disodium hydrogen phosphate, dipotassium hydrogen phosphate, sodium hydroxide, potassium hydroxide, cesium hydroxide, barium hydroxide, tetraalkylammonium hydroxide (tetramethylammonium hydroxide, tetrabutylammonium hydroxide, etc.), cesium fluoride, potassium fluoride, LiOtBu, NaOtBu, KOtBu, sodium hydride, potassium hydride, lithium bis(trimethylsilyl)amide, sodium bis(trimethylsilyl)amide, potassium bis(trimethylsilyl)amide, lithium diisopropylamide, methyllithium, butyllithium, pyridine, alkyl-substituted pyridine (2,6-lutidine, DTBP, DTBMP, DMAP, etc.), N,N-dimethylaniline, N-methylmorpholine, triethylamine, DIPEA, diethylamine, dimethylamine, diisopropylamine, dibutylamine, piperidine, morpholine, methylamine, ethylamine, isopropylamine, butylamine, DBU, DBN, MTBD, TMG, BTMG, P1tBu, BEMP, BTPP, P2Et, and P2tBu. If necessary, an additive may be used. Examples of the additive that can be used include zinc, zinc chloride, zinc bromide, zinc iodide, zinc acetate, zinc trifluoromethanesulfonate, tetrabutylammonium bromide, tetrabutylammonium chloride, and tetrabutylammonium iodide. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The number of equivalents of the additive used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

[Formula 23]

Ester bond formation reaction

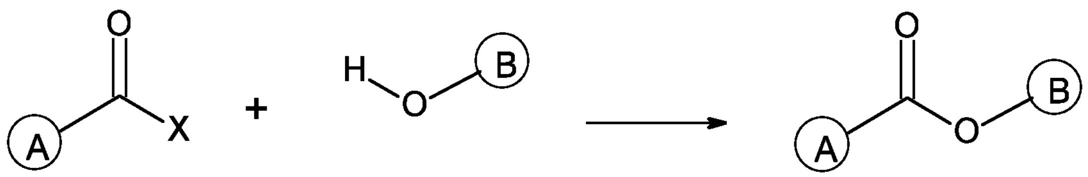

The ester bond as the linker can be formed through the reaction of carboxylic acid or a derivative thereof with alcohol.

When A in the formula contains a solid-phase carrier, the reactive functional group X linked to A is hydroxy. The ester bond as the linker can be formed through the reaction thereof with alcohol containing a hydroxy group linked to B using a condensing agent. The number of equivalents of the alcohol containing B used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. In this context, the condensing agent is not particularly limited as long as the condensing agent can be usually used. Examples of the condensing agent that can be used include combinations of carbodiimide condensing agents such as DCC, DIC, N,N'-di-tert-butylcarbodiimide, EDCI, and EDCI·HCl and activators such as HOAt, HOBt, and oxyma, uronium salt condensing agents such as HATU, TATU (1-[bis(dimethylamino)methylene]-1H-1,2,3-triazolo[4,5- b]pyridinium 3-oxide tetrafluoroborate), and COMU, phosphonium salt condensing agents such as PyBOP (1H-benzotriazol-1-yloxytripyrrolidinophosphonium hexafluorophosphate) and PyOxim ([ethylcyano(hydroxyimino)acetato-$O^2$]tri-1-pyrrolidinylphosphonium hexafluorophosphate), formamidinium salt condensing agents such as TCFH (chloro- N,N,N',N'-tetramethylformamidinium hexafluorophosphate), PyClU (1-(chloro-1- pyrrolidinylmethylene)pyrrolidinium hexafluorophosphate), PipClU (chlorodipiperidinocarbenium hexafluorophosphate), and CIP (2-chloro-1,3- dimethylimidazolinium hexafluorophosphate), acid anhydrides such as 3-pyridinecarboxylic anhydride, 2-methyl-6-nitrobenzoic anhydride, 4-trifluoromethylbenzoic anhydride, and 2- fluoro-6-(trifluoromethyl)benzoic anhydride, acid chlorides such as isobutyl chloroformate, 4-nitrophenyl chloroformate, 4-nitrobenzyl chloroformate, and 2,4,6-trichlorobenzoyl chloride, phosgene derivatives such as triphosgene, and carbodiimidazole derivatives such as 1,1'-carbonyldiimidazole. The number of equivalents of the condensing agent used can be appropriately set by those skilled in the art and can be, for example, 1 to 100 equivalents, 1 to 75 equivalents, 1 to 50 equivalents, 1 to 30 equivalents, or 1 to 10 equivalents, based on A-X.

A solvent can be used in the reaction. Examples of the solvent that can be used include, but are not particularly limited to, halogen solvents such as DCM, chloroform, DCE, and carbon tetrachloride, ether solvents such as diethyl ether, diisopropyl ether, THF, 2- methyltetrahydrofuran, 4-methyltetrahydropyran, 1,4-dioxane, DME, TBME, CPME,

diisopropyl ether, isosorbide dimethyl ether, diglyme, triglyme, and tetraglyme, ketone solvents such as acetone, MEK, MIBK, 4-methyl-2-pentanone, cyclopentanone, cyclohexanone, and diethyl ketone, urea solvents such as TMU, DMI, and DMPU, amide solvents such as DMF, DMA, NFM, NMP, NEP, and NBP, sulfoxide solvents such as DMSO, sulfone solvents such as diphenylsulfone, dimethylsulfone, diethylsulfone, sulfolane, 3-methylsulfolane, ethylmethylsulfone, and ethylisopropylsulfone, ester solvents such as methyl acetate, ethyl acetate, propyl acetate, isopropyl acetate, butyl acetate, isobutyl acetate, GVL (γ-valerolactone), pentyl acetate, and methyl propionate, aromatic solvents such as anisole, toluene, xylene, chlorobenzene, bromobenzene, ethylbenzene, nitrobenzene, α,α,α- trifluorotoluene, 1,2-dichlorobenzene, benzene, and pyridine, carbonate solvents such as dimethyl carbonate, diethyl carbonate, ethylene carbonate, and propylene carbonate, and others such as acetonitrile and limonene. A single solvent may be used, or a combined solvent of a plurality of solvents may be used. The reaction temperature can be appropriately set by those skilled in the art and can be, for example, 0 to 100°C, 20 to 80°C, or 20 to 50°C. If necessary, a base may be used. Examples of the base that can be used include pyridine, 2,6-lutidine, 2,4,6-collidine, DTBP, DTBMP, triethylamine, DIPEA, 4-methylmorpholine, NMI, DMAP, sodium carbonate, potassium carbonate, cesium carbonate, sodium bicarbonate, potassium phosphate, $K_2HPO_4$, potassium bicarbonate, $Na_2HPO_4$, DBU, DBN, MTBD, BTMG, TMG, and 1,8-bis(dimethylamino)naphthalene. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X.

After the completion of the reaction, the target compound can be obtained by a usual aftertreatment method.

In the formula, the reactive functional group X linked to A is hydroxy. The ether bond as the linker can be formed through the reaction thereof with alcohol containing a hydroxy group linked to B using an azodicarboxylic acid derivative and phosphine, or a cyanomethylenephosphorane derivative. The number of equivalents of the alcohol containing B used can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on A-X. In this context, the azodicarboxylic acid derivative is not particularly limited as long as the azodicarboxylic acid derivative can be usually used. Examples of the azodicarboxylic acid derivative that can be used include diethyl azodicarboxylate (DEAD), bis(2,2,2-trichloroethyl) azodicarboxylate, N,N,N',N'- tetramethylazodicarboxamide (TMAD), di-tert-butyl azodicarboxylate, dimethyl azodicarboxylate, dibenzyl azodicarboxylate, diisopropyl azodicarboxylate (DIAD), 1,1'- (azodicarbonyl)dipiperidine (ADDP), N,N,N',N'-tetraisopropylazodicarboxamide (TIPA), 1,6-dimethyl-1,5,7-hexahydro-1,4,6,7-tetrazocine-2,5-dione (DHTD), and bis(2- methoxyethyl) azodicarboxylate. The phosphine is not particularly limited as long as the phosphine can be usually used. Examples of the phosphine that can be used include triphenylphosphine (TPP), tributylphosphine (TBP), 4- (dimethylamino)phenyldiphenylphosphine, isopropyldiphenylphosphine, phenoxydiphenylphosphine, tri-n-octylphosphine, diphenyl-2-pyridylphosphine, tri-tert- butylphosphine, dicyclohexylphenylphosphine, tricyclohexylphosphine, and trihexylphosphine. The cyanomethylenephosphorane derivative is not particularly limited as long as the cyanomethylenephosphorane can be usually used. Examples of the cyanomethylenephosphorane that can be used include cyanomethylene tributyl phosphorane (CMBP) and cyanomethylene trimethyl phosphorane (CMMP). The number of equivalents of the azodicarboxylic acid derivative used can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on A-X. The number of equivalents of the phosphine used can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on A-X. In the case of using a cyanomethylenephosphorane derivative, the number of equivalents of the cyanomethylenephosphorane derivative used can be appropriately set by those skilled in the art and can be, for example, 1 to 150 equivalents, 1 to 100 equivalents, 1 to 75 equivalents, 1 to 50 equivalents, 1 to 30 equivalents, or 1 to 10 equivalents, based on A-X.

A solvent can be used in the reaction. Examples of the solvent that can be used include, but are not particularly limited to, halogen solvents such as DCM, chloroform, DCE, and carbon tetrachloride, ether solvents such as diethyl ether, diisopropyl ether, THF, 2- methyltetrahydrofuran, 4-methyltetrahydropyran, 1,4-dioxane, DME, TBME, CPME, diisopropyl ether, isosorbide dimethyl ether, diglyme, triglyme, and tetraglyme, ketone solvents such as acetone, MEK, MIBK, 4-methyl-2-pentanone, cyclopentanone, cyclohexanone, and diethyl ketone, urea solvents such as TMU, DMI, and DMPU, amide solvents such as DMF, DMA, NFM, NMP, NEP, and NBP, sulfoxide solvents such as DMSO, ester solvents such as methyl acetate, ethyl acetate, propyl acetate, isopropyl acetate, butyl acetate, isobutyl acetate, GVL (γ-valerolactone), pentyl acetate, and methyl propionate, sulfone solvents such as diphenylsulfone, dimethylsulfone, diethylsulfone, sulfolane, 3- methylsulfolane, ethylmethylsulfone, and ethylisopropylsulfone, aromatic solvents such as anisole, toluene, xylene, chlorobenzene, bromobenzene, ethylbenzene, nitrobenzene, α,α,α- trifluorotoluene, 1,2-dichlorobenzene, benzene, and pyridine, hydrocarbon solvents such as hexane, pentane, heptane, and cyclohexane, carbonate solvents such as dimethyl carbonate, diethyl carbonate, ethylene carbonate, and propylene carbonate, and others such as acetonitrile and limonene. A single solvent may be used, or a combined solvent of a plurality of solvents may be used. The reaction temperature can be appropriately set by those skilled in the art and can be, for example, -20 to 150°C, 0 to 120°C, or 20 to 100°C. The reaction time can be appropriately set by those skilled in the art and can be, for

example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

[Formula 24]

Ester bond formation reaction

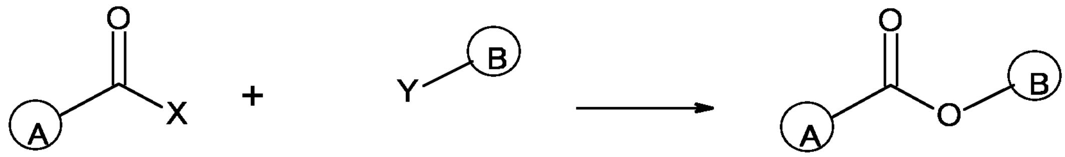

The ester bond as the linker can be formed through the reaction of carboxylic acid or a derivative thereof with alkyl halide, in addition to the method mentioned above.

When A in the formula contains a solid-phase carrier, the reactive functional group X linked to A is hydroxy. The ester bond as the linker can be formed through the reaction thereof with alkyl halide wherein the reactive functional group Y linked to B is halogen or an alkylating agent wherein Y is a hydroxy group derivative using a base. The number of equivalents of the alkylating agent containing B used can be appropriately set by those skilled in the art and can be, for example, 1 to 100 equivalents, 1 to 75 equivalents, 1 to 50 equivalents, 1 to 30 equivalents, or 1 to 10 equivalents, based on A-X.

In this context, the base is not particularly limited as long as the base can be usually used. Examples of the base that can be used include alkylpyridine derivatives such as 2,6- lutidine and 2,4,6-collidine, tertiary amine such as triethylamine, DIPEA, and 4- methylmorpholine, sodium carbonate, potassium carbonate, cesium carbonate, lithium hydroxide, sodium hydroxide, potassium hydroxide, barium hydroxide, tetraalkylammonium hydroxide (tetramethylammonium hydroxide, tetrabutylammonium hydroxide, etc.), lithium hydride, sodium hydride, potassium hydride, DBU, DBN, MTBD, BTMG, TMG, 1,8- bis(dimethylamino)naphthalene, BEMP, BTPP, P2Et, P2tBu, potassium phosphate, sodium phosphate, LiOtBu, NaOtBu, KOtBu, LiHMDS, NaHMDS, KHMDS, and HMDS. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on the carboxylic acid containing A.

A solvent can be used in the reaction. Examples of the solvent that can be used include, but are not particularly limited to, halogen solvents such as DCM, chloroform, DCE, and carbon tetrachloride, ether solvents such as diethyl ether, diisopropyl ether, THF, 2- methyltetrahydrofuran, 4-methyltetrahydropyran, 1,4-dioxane, DME, TBME, CPME, diisopropyl ether, isosorbide dimethyl ether, diglyme, triglyme, and tetraglyme, ketone solvents such as acetone, MEK, MIBK, 4-methyl-2-pentanone, cyclopentanone, cyclohexanone, and diethyl ketone, urea solvents such as TMU, DMI, and DMPU, amide solvents such as DMF, DMA, NFM, NMP, NEP, and NBP, sulfoxide solvents such as DMSO, ester solvents such as methyl acetate, ethyl acetate, propyl acetate, isopropyl acetate, butyl acetate, isobutyl acetate, GVL ($\gamma$-valerolactone), pentyl acetate, and methyl propionate, sulfone solvents such as diphenylsulfone, dimethylsulfone, diethylsulfone, sulfolane, 3- methylsulfolane, ethylmethylsulfone, and ethylisopropylsulfone, aromatic solvents such as anisole, toluene, xylene, chlorobenzene, bromobenzene, ethylbenzene, nitrobenzene, $\alpha,\alpha,\alpha$- trifluorotoluene, 1,2-dichlorobenzene, benzene, and pyridine, carbonate solvents such as dimethyl carbonate, diethyl carbonate, ethylene carbonate, and propylene carbonate, and others such as acetonitrile and limonene. A single solvent may be used, or a combined solvent of a plurality of solvents may be used. If necessary, an additive may be used. Examples of the additive that can be used include AgzO, 12-crown-4, 15-crown-5, 18-crown- 6, HMPA, TMEDA, tetrabutylammonium chloride, tetrabutylammonium bromide, and tetrabutylammonium iodide. In the case of using an additive, the number of equivalents of the additive used can be appropriately set by those skilled in the art and can be, for example, 0.01 to 100 equivalents, 0.01 to 75 equivalents, 0.01 to 50 equivalents, 0.01 to 30 equivalents, 0.1 to 30 equivalents, or 0.1 to 10 equivalents, based on A-X. The reaction temperature can be appropriately set by those skilled in the art and can be, for example, -20 to 150°C, 0 to 120°C, 20 to 100°C, or 20 to 60°C. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

[Formula 25]

Sulfone bond formation reaction

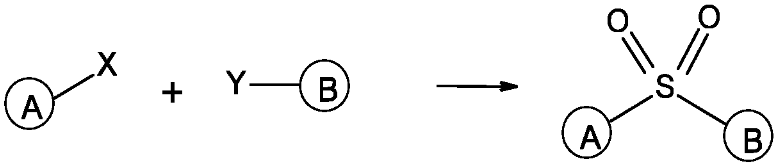

The sulfone bond as the linker can be formed, for example, through the reaction of aryl halide or a derivative thereof with alkyl halide or a derivative thereof and sulfur dioxide or a sulfur dioxide equivalent. When A in the formula contains a solid-phase carrier, the reactive functional group X linked to A is halogen or $-O-SO_2-R^5$ wherein $R^5$ is $C_{1-6}$ alkyl optionally substituted by one or more fluorine atoms, or phenyl optionally substituted by one or more fluorine atoms or $C_{1-6}$ alkyl optionally substituted by fluorine atom(s). The sulfone bond as the linker can be formed through the reaction thereof with a hydroxyalkyl derivative wherein the reactive functional group Y linked to B is halogen or $-O-SO_2-R^5$, and sulfur dioxide or a sulfur dioxide equivalent using a metal catalyst. In this context, the sulfur dioxide equivalent is not particularly limited as long as the sulfur dioxide equivalent can be usually used. Examples of the sulfur dioxide equivalent that can be used include bis(sulfur dioxide)-1,4-diazabicyclo[2.2.2]octane adducts, sulfur dioxide 1-methylpyrrolidine adducts, and potassium pyrosulfite. The number of equivalents of the alkyl halide or the derivative thereof containing B used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The number of equivalents of the sulfur dioxide or the sulfur dioxide equivalent used can be appropriately set by those skilled in the art and can be, for example, 1 to 100 equivalents, 1 to 75 equivalents, 1 to 50 equivalents, 1 to 30 equivalents, 1 to 10 equivalents, or 1 to 5 equivalents, based on A-X. The metal catalyst is not particularly limited as long as the metal catalyst can be usually used. Examples of the metal catalyst that can be used include palladium acetate, $Pd(dba)_2$, $Pd_2(dba)_3$, $Pd_2(dba)_3CHCl_3$, allylpalladium chloride dimers, palladium($\pi$-cinnamyl) chloride dimers, (1- methylallyl)palladium chloride dimers, and (1,5- cyclooctadiene)bis(trimethylsilylmethyl)palladium(II). If necessary, a ligand may be used. Examples of the ligand that can be used include trialkylphosphine ($PCy_3$ (tricyclohexylphosphine), $P(tBu)_3$ (tri-tert-butylphosphine), cataCXium A (di(1-adamantyl)- n-butylphosphine), $(tBu)_2PMe$ (di-tert-butylmethylphosphine), $P(Ad)_3$, neopentyl $(tBu)_2P$ (di- tert-butylneopentylphosphine), etc.), triarylphosphine ($PPh_3$ (triphenylphosphine), etc.), dialkylbiarylphosphine (XPhos (2-dicyclohexylphosphino-2',4',6'-triisopropylbiphenyl), SPhos (2-dicyclohexylphosphino-2',6'-dimethoxybiphenyl), RuPhos (2- dicyclohexylphosphino-2',6'-diisopropoxybiphenyl), BrettPhos (2-(dicyclohexylphosphino)- 3,6-dimethoxy-2',4',6'-triisopropyl-1,1'-biphenyl), tBuXPhos (2-di-tert-butylphosphino- 2',4',6'-triisopropylbiphenyl), CPhos (2-dicyclohexylphosphino-2',6'-bis(N,N- dimethylamino)biphenyl), DavePhos (2-dicyclohexylphosphino-2'-(N,N- dimethylamino)biphenyl), RockPhos (2-di(tertiary butyl)phosphino-2',4',6'-triisopropyl-3- methoxy-6-methylbiphenyl, di-tertiary butyl(2',4',6'-triisopropyl-3-methoxy-6-methyl-[1,1'- biphenyl]-2-yl)phosphine), $Me_4$tBuXPhos (2-di-tert-butylphosphino-3,4,5,6-tetramethyl- 2',4',6'-triisopropyl-1,1'-biphenyl), tBuBrettPhos (2-(di-tert-butylphosphino)-2',4',6'- triisopropyl-3,6-dimethoxy-1,1'-biphenyl), AdBrettPhos (2-(di-1-adamantylphosphino)- 2',4',6'-triisopropyl-3,6-dimethoxy-1,1'-biphenyl), etc.), dialkylmonoarylphosphine (APhos ((4-(N,N-dimethylamino)phenyl)di-tert-butylphosphine), $(tBu)_2PPh$, MorDalPhos (2- morpholinophenyldi(1-adamantyl)phosphine), etc.), bidentate phosphine ligands (dppf (1,1'- bis(diphenylphosphino)ferrocene), dtbpf (1,1'-bis(di-tert-butylphosphino)ferrocene), BINAP (2,2'-bis(diphenylphosphino)-1,1'-binaphthyl), XantPhos (4,5-bis(diphenylphosphino)-9,9- dimethylxanthene), Josiphos ((R)-1-[(Sp)-2-(dicyclohexylphosphino)ferrocenyl]ethyldi-tert-butylphosphine), etc.), and NHC ligands (IPr (1,3-bis(2,6-diisopropylphenyl)imidazol-2- ylidene), SIPr (1,3-bis(2,6-di-i-propylphenyl)imidazolidin-2-ylidene), IMes (1,3-bis(2,4,6- trimethylphenyl)imidazol-2-ylidene), IPent (1,3-bis(2,6-di-3-pentylphenyl)imidazol-2- ylidene), etc.). Alternatively, the ligand described above and a precursor of the palladium catalyst may be used. Examples of the precursor of the palladium catalyst that can be used include first-generation Buchwald precatalysts (G1) (XPhos Pd G1, SPhos Pd G1, RuPhos Pd G1, BrettPhos Pd G1, tBuXPhos Pd G1, etc.), second-generation Buchwald precatalysts (G2) (XPhos Pd G2, SPhos Pd G2, RuPhos Pd G2, BrettPhos Pd G2, tBuXPhos Pd G2, CPhos Pd G2, DavePhos Pd G2, APhos Pd G2, $tBu)_2PPh$ Pd G2, MorDalPhos Pd G2, $PCy_3$ Pd G2, $P(tBu)_3$ Pd G2, cataCXium A Pd G2, XantPhos Pd G2, etc.), third-generation Buchwald precatalysts (G3) (XPhos Pd G3, SPhos Pd G3, RuPhos Pd G3, BrettPhos Pd G3, tBuXPhos Pd G3, CPhos Pd G3, DavePhos Pd G3, RockPhos Pd G3, $Me_4$tBuXPhos Pd G3, tBuBrettPhos Pd G3, AdBrettPhos Pd G3, APhos Pd G3, $(tBu)_2PPh$ Pd G3, MorDalPhos Pd G3, $PCy_3$ Pd G3, $P(tBu)_3$ Pd G3, cataCXium A Pd G3, $tBu)_2PMe$ Pd G3, neopentyl $(tBu)_2P$ Pd G3, dppf Pd G3, dtbpf Pd G3, BINAP Pd G3, XantPhos Pd G3, Josiphos Pd G3, etc.), fourth-generation Buchwald precatalysts (G4) (XPhos Pd G4, SPhos Pd G4, RuPhos Pd G4, BrettPhos Pd G4, tBuXPhos Pd G4, CPhos Pd G4, tBuBrettPhos Pd G4, APhos Pd G4, $PCy_3$ Pd G4, $P(tBu)_3$ Pd G4, cataCXium A Pd G4, dppf Pd G4, BINAP Pd G4, XantPhos Pd G4, etc.), dichloropalla-

dium(II) complexes (Pd($PPh_3$)$_2$c$1_2$, Pd($PCy_3$)$_2$c$1_2$, Pd(dppf)$Cl_2$, Pd(dtbpf)$Cl_2$, (APhos)$_2$Pd$Cl_2$, etc.), palladium(0) complexes (tetrakistriphenylphosphine palladium(0), bis(tricyclohexylphosphine)palladium(0), bis(tri-tert- butylphosphine)palladium(0), etc.), palladium(I) halide complexes (bromo(tri-tert- butylphosphine)palladium(I) dimers, iodo(tri-tert-butylphosphine)palladium(I) dimers, etc.), phosphine π-allylpalladium catalysts (XPhos Pd(crotyl)Cl (chloro(crotyl)(2-dicyclohexylphosphino-2',4',6'-triisopropyl-1,1'-biphenyl)palladium(II)), SPhos Pd(crotyl)Cl (chloro(crotyl)(2-dicyclohexylphosphino-2',6'-dimethoxy-1,1'-biphenyl)palladium(II)), RuPhos Pd(crotyl)Cl (chloro(crotyl)(2-dicyclohexylphosphino-2',6'-diisopropoxy-1,1'- biphenyl)palladium(II)), [BrettPhos Pd(crotyl)]OTf (crotyl(2-dicyclohexylphosphino-2',4',6'- triisopropyl-3,6-dimethoxy-1,1'-biphenyl)palladium(II) triflate), [tBuXPhos Pd(allyl)]OTf (allyl(2-di-tert-butylphosphino-2',4',6'-triisopropyl-1,1'-biphenyl)palladium(II) triflate), [tBuBrettPhos Pd(allyl)]OTf (allyl(2-di-tert-butylphosphino-3,6-dimethoxy-2',4',6'- triisopropyl-1,1'-biphenyl)palladium(II) triflate), AmPhos Pd(crotyl)Cl (chloro(crotyl)[(p- dimethylaminophenyl)(di-tert-butylphosphine)]palladium(II)), P(Cy)$_3$ Pd(crotyl)Cl (chloro(crotyl)(tricyclohexylphosphine)palladium(II)), [P(tBu)$_3$] Pd(crotyl)Cl (tri-tert- butylphosphine(chloro)(crotyl)palladium(II)), [BINAP Pd(allyl)]Cl ((R)-BINAP Pd(allyl)]Cl; allyl[(R)-2,2'-bis(diphenylphosphino)-1,1'-binaphthalene]palladium(II) chloride), [XantPhos Pd(allyl)]Cl (allyl[4,5-bis(diphenylphosphino)-9,9-dimethylxanthene]palladium(II) chloride), etc.), PEPPSI catalysts (Pd-PEPPSI IPr, Pd-PEPPSI SIPr ((1,3-bis(2,6- diisopropylphenyl)imidazolidene)(3-chloropyridyl)palladium(II) dichloride), Pd-PEPPSI IPent (1,3-bis(2,6-di-3-pentylphenyl)imidazol-2-ylidene), etc.), (NHC)Pd(allyl)Cl catalysts (allyl[1,3-bis(2,6-diisopropylphenyl)imidazol-2-ylidene]chloropalladium(II), allyl[1,3- bis(2,6-diisopropylphenyl)-2-imidazolidinylidene]chloropalladium(II), [1,3-bis(2,6- diisopropylphenyl)imidazol-2-ylidene]chloro[3-phenylallyl]palladium(II), etc.), and NHC-Pd naphthoquinone catalysts (1,3-bis(2,6-diisopropylphenyl)imidazol-2-ylidene(1,4- naphthoquinone)palladium(0) dimers, etc.). The number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The number of equivalents of the catalyst precursor used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL (γ-valerolactone), pentyl acetate, methyl propionate, etc.), aromatic hydrocarbon solvents (anisole, toluene, xylene, chlorobenzene, bromobenzene, ethylbenzene, nitrobenzene, α,α,α- trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. A single solvent may be used, or a combined solvent of a plurality of solvents may be used. The reaction temperature can be appropriately set by those skilled in the art and can be, for example, 0 to 200°C, 0 to 140°C, 10 to 100°C, or 20 to 60°C. If necessary, a base may be used. Examples of the base that can be used include sodium carbonate, potassium carbonate, cesium carbonate, sodium phosphate, potassium phosphate, sodium hydroxide, potassium hydroxide, cesium hydroxide, barium hydroxide, tetraalkylammonium hydroxide (tetramethylammonium hydroxide, tetrabutylammonium hydroxide, etc.), NaOtBu, KOt-Bu, sodium fluoride, potassium fluoride, cesium fluoride, triethylamine, DIPEA, DBU, DBN, MTBD, TMG, BTMG, P1tBu, BEMP, BTPP, P2Et, P2tBu, sodium tetraborate, 1,8- bis(dimethylamino)naphthalene, potassium t-pentoxide, sodium t-pentoxide, lithium t- pentoxide, and tetrabutylammonium fluoride. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier, the reaction can be performed using the already mentioned

reagent and reaction conditions. In this context, the number of equivalents of the aryl halide containing A used can be appropriately set by those skilled in the art and can be, for example, 1 to 100 equivalents, 1 to 75 equivalents, 1 to 50 equivalents, 1 to 30 equivalents, or 1 to 10 equivalents, based on the alkyl halide containing B. The number of equivalents of the sulfur dioxide or the sulfur dioxide equivalent can be appropriately set by those skilled in the art and can be, for example, 1 to 100 equivalents, 1 to 75 equivalents, 1 to 50 equivalents, 1 to 30 equivalents, 1 to 10 equivalents, or 1 to 5 equivalents, based on the alkyl halide containing B. The number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the alkyl halide containing B. The number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the alkyl halide containing B. The number of equivalents of the catalyst precursor used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the alkyl halide containing B.

[Formula 26]

Thioether bond formation reaction

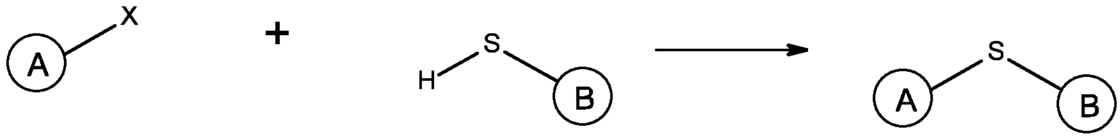

The thioether bond as the linker can be formed through aromatic nucleophilic substitution reaction using aryl halide or a derivative thereof and thiol, catalytic coupling reaction using a metal catalyst, or nucleophilic substitution reaction using alkyl halide, hydroxyalkyl or a derivative thereof and thiol.

When A in the formula contains a solid-phase carrier and A-X is aryl halide or a derivative thereof, the reactive functional group X linked to A is halogen or a group derived from a hydroxy group. Examples of the group derived from a hydroxy group can include a methanesulfonyl group, a trifluoromethanesulfonyl group, a nonafluorobutanesulfonyl group, a benzenesulfonyl group, a para-toluenesulfonyl group, a 2-nitrobenzenesulfonyl group, a 4- nitrobenzenesulfonyl group, and a 2,4-dinitrobenzenesulfonyl group. A-X can be subjected to reaction with thiol linked to B in the presence of a base. In this context, the base is not particularly limited as long as the base can be usually used. Examples of the base that can be used include sodium carbonate, potassium carbonate, cesium carbonate, sodium bicarbonate, potassium bicarbonate, sodium phosphate, potassium phosphate, disodium hydrogen phosphate, dipotassium hydrogen phosphate, lithium hydroxide, sodium hydroxide, potassium hydroxide, cesium hydroxide, barium hydroxide, tetraalkylammonium hydroxide (tetramethylammonium hydroxide, tetrabutylammonium hydroxide, benzyl triethylammonium hydroxide, , etc.), cesium fluoride, potassium fluoride, LiOtBu, NaOtBu, KOtBu, potassium t-pentoxide, sodium t-pentoxide, lithium t-pentoxide, sodium hydride, potassium hydride, lithium hydride, sodium amide, lithium amide, lithium bis(trimethylsilyl)amide, sodium bis(trimethylsilyl)amide, potassium bis(trimethylsilyl)amide, hexamethyldisilazane, lithium diisopropylamide, lithium dicyclohexylamide, 2,2,6,6- tetramethylpiperidinyl lithium, 2,2,6,6-tetramethylpiperidinyl magnesium chloride-lithium chloride complexes, methyllithium, butyllithium, pyridine, alkyl-substituted pyridine (2,6-lutidine, 2,4,6-collidine, DTBP, DTBMP, etc.), DMAP, N,N-dimethylaniline, N- methylmorpholine, triethylamine, DIPEA, DBU, DBN, MTBD, TMG, BTMG, P1tBu, BEMP, BTPP, P2Et, P2tBu, NMI, sodium tetraborate, 1,8-bis(dimethylamino)naphthalene, N,N-dimethylaniline, tetrabutylammonium fluoride, and cesium fluoride. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The number of equivalents of the thiol containing B used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N-dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide),

methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL (γ-valerolactone), pentyl acetate, methyl propionate, etc.), aromatic hydrocarbon solvents (anisole, toluene, xylene, chlorobenzene, bromobenzene, ethylbenzene, nitrobenzene, α,α,α-trifluorotoluene, 1,2-dichlorobenzene, benzene, tetralin, pyridine, quinoline, etc.), alcohol solvents (ethanol, isopropanol, tert-butanol, tert-amyl alcohol, etc.), hydrocarbon solvents (hexane, pentane, heptane, cyclohexane, methylcyclohexane, etc.), and others such as acetonitrile, water, dimethylacetal, dimethyl sulfate, silene, and limonene. A single solvent may be used, or a combined solvent of a plurality of solvents may be used. The reaction temperature can be appropriately set by those skilled in the art and can be, for example, 0 to 150°C, 10 to 120°C, 20 to 100°C, 20 to 80°C, or 20 to 60°C. If necessary, a metal catalyst may be used. Examples of the metal catalyst that can be used include palladium, for example, palladium acetate, $Pd(dba)_2$, $Pd_2(dba)_3$, $Pd_2(dba)_3CHCl_3$, allylpalladium chloride dimers, palladium(π-cinnamyl) chloride dimers, (1-methylallyl)palladium chloride dimers, and (1,5- cyclooctadiene)bis(trimethylsilylmethyl)palladium(II), nickel, for example, NiClz, (nickel(II) chloride) and $Ni(COD)_2$ (bis(1,5-cyclooctadiene)nickel(0)), copper, for example, copper(I) oxide (CuzO), copper(I) iodide (CuI), copper(I) bromide (CuBr), copper(I) chloride (CuCl), and copper acetate ($Cu(OAc)_2$).

If necessary, a ligand may be used. Examples of the ligand for palladium that can be used include trialkylphosphine ($Cy_3P$ (tricyclohexylphosphine), $P(tBu)_3$ (tri-tert- butylphosphine), cataCXium A (di(1-adamantyl)-n-butylphosphine), $(tBu)_2PMe$ (di-tert- butylmethylphosphine), $P(Ad)_3$ (tri(1-adamantyl)phosphine), neopentyl (tBu)zP (di-tert- butylneopentylphosphine), etc.), triarylphosphine ($PPh_3$ (triphenylphosphine), etc.), dialkylbiarylphosphine (XPhos (2-dicyclohexylphosphino-2',4',6'-triisopropylbiphenyl), SPhos (2-dicyclohexylphosphino-2',6'-dimethoxybiphenyl), RuPhos (2-dicyclohexylphosphino-2',6'-diisopropoxybiphenyl), BrettPhos (2-(dicyclohexylphosphino)- 3,6-dimethoxy-2',4',6'-triisopropyl-1,1'-biphenyl), tBuXPhos (2-di-tert-butylphosphino- 2',4',6'-triisopropylbiphenyl), CPhos (2-dicyclohexylphosphino-2',6'-bis(N,N- dimethylamino)biphenyl), DavePhos (2-dicyclohexylphosphino-2'-(N,N- dimethylamino)biphenyl), RockPhos (2-di(tertiary butyl)phosphino-2',4',6'-triisopropyl-3- methoxy-6-methylbiphenyl, di-tertiary butyl(2',4',6'-triisopropyl-3-methoxy-6-methyl-[1,1'- biphenyl]-2-yl)phosphine), $Me_4$tBuXPhos (2-di-tert-butylphosphino-3,4,5,6-tetramethyl- 2',4',6'-triisopropyl-1,1'-biphenyl), tBuBrettPhos (2-(di-tert-butylphosphino)-2',4',6'- triisopropyl-3,6-dimethoxy-1,1'-biphenyl), AdBrettPhos (2-(di-1 -adamantylphosphino)- 2',4',6'-triisopropyl-3,6-dimethoxy-1,1'-biphenyl), BippyPhos (5-(di-tert-butylphosphino)- 1',3',5'-triphenyl-1'H-[1,4']biprazole), AdBippyPhos (5-[di(1-adamantyl)phosphino]-1',3',5'-triphenyl-1'H-[1,4']biprazole), etc.), dialkylmonoarylphosphine (APhos, ((4-(N,N- dimethylamino)phenyl)di-tert-butylphosphine), $(tBu)_2PPh$, MorDalPhos (2- morpholinophenyldi(1-adamantyl)phosphine), etc.), bidentate phosphine ligands (dppf (1,1'- bis(diphenylphosphino)ferrocene), dtbpf (1,1'-bis(di-tert-butylphosphino)ferrocene), BINAP (2,2'-bis(diphenylphosphino)-1,1'-binaphthyl), XantPhos (4,5-bis(diphenylphosphino)-9,9- dimethylxanthene), Josiphos ((R)-1-[(Sp)-2-(dicyclohexylphosphino)ferrocenyl]ethyldi-tert- butylphosphine), etc.), and NHC ligands (IPr (1,3-bis(2,6-diisopropylphenyl)imidazol-2- ylidene), SIPr (1,3-bis(2,6-di-i-propylphenyl)imidazolidin-2-ylidene), IMes (1,3-bis(2,4,6- trimethylphenyl)imidazol-2-ylidene), IPent (1,3-bis(2,6-di-3-pentylphenyl)imidazol-2- ylidene), etc.). Alternatively, the ligand described above and a precursor of the palladium catalyst may be used. Examples of the precursor of the palladium catalyst that can be used include first-generation Buchwald precatalysts (G1) (XPhos Pd G1, SPhos Pd G1, RuPhos Pd G1, BrettPhos Pd G1, tBuXPhos Pd G1, etc.), second-generation Buchwald precatalysts (G2) (XPhos Pd G2, SPhos Pd G2, RuPhos Pd G2, BrettPhos Pd G2, tBuXPhos Pd G2, CPhos Pd G2, DavePhos Pd G2, APhos Pd G2, $(tBu)_2PPh$ Pd G2, MorDalPhos Pd G2, $PCy_3$ Pd G2, $P(tBu)_3$Pd G2, cataCXium A Pd G2, XantPhos Pd G2, etc.), third-generation Buchwald precatalysts (G3) (XPhos Pd G3, SPhos Pd G3, RuPhos Pd G3, BrettPhos Pd G3, tBuXPhos Pd G3, CPhos Pd G3, DavePhos Pd G3, RockPhos Pd G3, $Me_4$tBuXPhos Pd G3, tBuBrettPhos Pd G3, AdBrettPhos Pd G3, APhos Pd G3, $(tBu)_2PPh$ Pd G3, MorDalPhos Pd G3, $PCy_3$ Pd G3, $P(tBu)_3$Pd G3, cataCXium A Pd G3, (tBu)zPMe Pd G3, neopentyl (tBu)zP Pd G3, dppf Pd G3, dtbpf Pd G3, BINAP Pd G3, XantPhos Pd G3, Josiphos Pd G3, etc.), fourth-generation Buchwald precatalysts (G4) (XPhos Pd G4, SPhos Pd G4, RuPhos Pd G4, BrettPhos Pd G4, tBuXPhos Pd G4, CPhos Pd G4, tBuBrettPhos Pd G4, APhos Pd G4, $PCy_3$ Pd G4, $P(tBu)_3$Pd G4, cataCXium A Pd G4, dppf Pd G4, BINAP Pd G4, XantPhos Pd G4, etc.), dichloropalladium(II) complexes ($Pd(PPh_3)_2c1_2$, $Pd(PCy_3)_2c1_2$, $Pd(dppf)Cl_2$, $Pd(dtbpf)Cl_2$, (APhos)zPdClz, etc.), palladium(0) complexes (tetrakistriphenylphosphine palladium(0), bis(tricyclohexylphosphine)palladium(0), bis(tri-tert- butylphosphine)palladium(0), etc.), palladium(I) halide complexes (bromo(tri-tert-butylphosphine)palladium(I) dimers, iodo(tri-tert-butylphosphine)palladium(I) dimers, etc.), phosphine π-allylpalladium catalysts (XPhos Pd(crotyl)Cl (chloro(crotyl)(2- dicyclohexylphosphino-2',4',6'-triisopropyl-1,1'-biphenyl)palladium(II)), SPhos Pd(crotyl)Cl (chloro(crotyl)(2-dicyclohexylphosphino-2',6'-dimethoxy-1,1'-biphenyl)palladium(II)), RuPhos Pd(crotyl)Cl (chloro(crotyl)(2-dicyclohexylphosphino-2',6'-diisopropoxy-1,1'- biphenyl)palladium(II)), [BrettPhos Pd(crotyl)]OTf (crotyl(2-dicyclohexylphosphino-2',4',6'- triisopropyl-3,6-dimethoxy-1,1'-biphenyl)palladium(II) triflate), [tBuXPhos Pd(allyl)]OTf (allyl(2-di-tert-butylphosphino-2',4',6'-triisopropyl-1,1'-biphenyl)palladium(II) triflate), [tBuBrettPhos Pd(allyl)]OTf (allyl(2-di-tert-butylphosphino-3,6-dimethoxy-2',4',6'- triisopropyl-1,1'-biphenyl)palladium(II) triflate), AmPhos Pd(crotyl)Cl (chloro(crotyl)[(p- dimethylaminophenyl)(di-tert-butylphosphine)]palladium(II)), $P(Cy)_3$ Pd(crotyl)Cl (chloro(crotyl)(tricyclohexylphosphine)palladium(II)), $[P(tBu)_3]$ Pd(crotyl)Cl (tri-tert- butylphosphine(chloro)(crotyl)palladium(II)), [BINAP Pd(allyl)]Cl [XantPhos Pd(allyl)]Cl (allyl[4,5-bis(diphenylphosphino)-9,9-dimethylxanthene]palladium(II) chloride), etc.), PEPPSI catalysts (Pd-PEPPSI IPr, Pd-PEPPSI SIPr ((1,3-bis(2,6- diisopropylphenyl)imidazolidene)(3-chloropyridyl)palladium(II) dichloride), Pd-PEPPSI IPent, etc.), (NHC)Pd(allyl)Cl catalysts (allyl[1,3-bis(2,6-diisopropylphenyl)imidazol-2- ylidene]chloropalladium(II), allyl[1,3-bis(2,6-diisopropylphenyl)-2- imidazolidinylidene]chloropalladium(II), [1,3-bis(2,6-diisopropylphenyl)imidazol-2- ylidene]chloro[3-phenylallyl]palladium(II), etc.), and NHC-Pd naphthoquinone catalysts (1,3-bis(2,6-diisopropylphenyl)imidazol-2-ylidene(1,4-naphthoquinone)palladium(0) dimers, etc.).

Examples of the ligand for copper that can be used include diamine ligands, for example, di-tert-butyl bipyridine (dtBBPy (4,4'-di-tert-butyl-2,2'-dipyridyl), etc.), dimethylethylenediamine (DMEDA (N,N'-dimethylethylenediamine), etc.), cyclohexyldimethylethylenediamine (CyDMEDA (trans-N,N'-dimethylcyclohexane-1,2- diamine), etc.), and 1,10-phenanthroline (Phen), amino acid ligands, for example, N,N'- dimethylglycine, sarcosine (N-methylglycine), L-proline, and 4-hydroxy-1-proline, diamide ligands, for example, $N^1$-(2-methyl-1-naphthalenyl)-$N^2$-(phenylmethyl)ethanediamide, $N^1$,$N^2$-bis(phenylmethyl)ethanediamine, $N^1$,$N^2$-bis(2,6-dimethylphenyl)ethanediamine, $N^1$,$N^2$-bis(2-furanylmethyl)ethanediamine, $N^1$,$N^2$-bis(2,4,6-trimethoxyphenyl)ethanediamine, $N^1$,$N^2$-bis(5-methyl[1,1'-biphenyl]-2-yl)-ethanediamide, [(2,6- dimethylphenyl)amino](oxo)acetic acid (DMPAO), and N-[1,1'-biphenyl]-2-yl-1H-indole-2- carboxamide, and other such as 2-methyl-8-hydroxyquinoline. In the case of using a metal catalyst, the number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. In the case of using a ligand, the number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. In the case of using a catalyst precursor, the number of equivalents of the catalyst precursor used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier and A-X is aryl halide or an analog thereof, the aromatic nucleophilic substitution reaction with thiol or the catalytic coupling reaction using a metal catalyst can be performed using the already mentioned reagent and reaction conditions. In this context, in the case of using a base, the number of equivalents thereof can be appropriately set by those skilled in the art and can be, for example, 1 to 100 equivalents, 1 to 75 equivalents, 1 to 50 equivalents, 1 to 30 equivalents, or 1 to 10 equivalents, based on the thiol containing B. The number of equivalents of A-X used can be appropriately set by those skilled in the art and can be, for example, 1 to 100 equivalents, 1 to 75 equivalents, 1 to 50 equivalents, 1 to 30 equivalents, or 1 to 10 equivalents, based on the thiol containing B. In the case of using a metal catalyst, the number of equivalents of the metal catalyst can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the thiol containing B. In the case of using a ligand, the number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the thiol containing B. In the case of using a catalyst precursor, the number of equivalents of the catalyst precursor used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the thiol containing B.

When A-X in the formula is alkyl halide, hydroxyalkyl or an analog thereof, the reactive functional group X linked to A is a leaving group, for example, halogen or a group derived from a hydroxy group. Examples of the group derived from a hydroxy group include groups such as a methanesulfonyl group, a trifluoromethanesulfonyl group, a nonafluorobutanesulfonyl group, a benzenesulfonyl group, a para-toluenesulfonyl group, a 2- nitrobenzenesulfonyl group, a 4-nitrobenzenesulfonyl group, and a 2,4- dinitrobenzenesulfonyl group. The thioether bond as the linker can be formed through the reaction thereof with thiol linked to B in the presence of a base.

In this context, the base is not particularly limited as long as the base can be usually used. Examples of the base that can be used include sodium carbonate, potassium carbonate, cesium carbonate, sodium bicarbonate, potassium bicarbonate, lithium hydroxide, sodium hydroxide, potassium hydroxide, barium hydroxide, tetraalkylammonium hydroxide (tetramethylammonium hydroxide, tetrabutylammonium hydroxide, etc.), sodium hydride, potassium hydride, DBU, DBN, MTBD, BTMG, TMG, 1,8-bis(dimethylamino)naphthalene, BEMP, BTPP, P2Et, P2tBu, potassium phosphate, LiOtBu, NaOtBu, KOtBu, LiHMDS, NaHMDS, KHMDS, HMDS, pyridine, 2,6-lutidine, 2,4,6-collidine, DTBP, DTBMP, triethylamine, DIPEA, 4-methylmorpholine, NMI, DMAP, potassium hydride, sodium hydride, lithium hydride, sodium amide, lithium amide, sodium phosphate, $K_2HPO_4$, $KHCO_3$, $Na_2HPO_4$, sodium tetraborate, potassium t-pentoxide, sodium t-pentoxide, lithium t-pentoxide, lithium diisopropylamide, lithium dicyclohexylamide, 2,2,6,6-tetramethylpiperidinyl lithium, 2,2,6,6-tetramethylpiperidinyl magnesium chloride-lithium chloride complexes, N,N- dimethylaniline, P1-tBu, P2tBu, benzyl triethylammonium hydroxide, butyllithium, methyllithium, tetrabutylammonium fluoride, and cesium fluoride. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-X. The number of equivalents of the thiol containing B used can be appropriately set by those skilled in the art and can be, for example, 1 to 100 equivalents, 1 to 75 equivalents, 1 to 50 equivalents, 1 to 30 equivalents, or 1 to 10 equivalents, based on A-X.

A solvent can be used in the reaction. Examples of the solvent that can be used include, but are not particularly limited to, halogen solvents such as DCM, chloroform, DCE, and carbon tetrachloride, ether solvents such as diethyl ether, diisopropyl ether, THF, 2- methyltetrahydrofuran, 4-methyltetrahydropyran, 1,4-dioxane, DME, TBME, CPME, diisopropyl ether, isosorbide dimethyl ether, diglyme, triglyme, and tetraglyme, urea solvents such as TMU, DMI, and DMPU, amide solvents such as DMF, DMA, NFM, NMP, NEP, and NBP, sulfoxide solvents such as DMSO, sulfone solvents such as diphenylsulfone, dimethylsulfone, diethylsulfone, sulfolane, 3-methylsulfolane, ethylmethylsulfone, and ethylisopropylsulfone, aromatic solvents such as anisole, toluene, xylene, chlorobenzene, bromobenzene, ethylbenzene, nitrobenzene, $\alpha,\alpha,\alpha$-trifluorotoluene, 1,2-dichlorobenzene, benzene, and pyridine, carbonate solvents such as dimethyl carbonate, diethyl carbonate, ethylene carbonate, and propylene carbonate, and others such as acetonitrile and limonene. A single solvent may be used, or a combined solvent of a plurality of solvents may be used. The reaction temperature can be appropriately set by those skilled in the art and can be, for example, -20 to 120°C, -20 to 80°C, 0 to 80°C, or 0 to 60°C. If necessary, an additive may be used. Examples of the additive that can be used include 12-crown-4, 15-crown-5, 18- crown-6, HMPA, TMEDA, tetrabutylammonium chloride, tetrabutylammonium bromide, tetrabutylammonium iodide, and tetrabutylammonium bisulfate. After the completion of the reaction, the target compound can be obtained by a usual aftertreatment method.

When B in the formula contains a solid-phase carrier and A-X is alkyl halide, hydroxyalkyl or an analog thereof, the nucleophilic substitution reaction with thiol can be performed using the already mentioned reagent and reaction conditions. In this context, in the case of using a base, the number of equivalents thereof can be appropriately set by those skilled in the art and can be, for example, 1 to 100 equivalents, 1 to 75 equivalents, 1 to 50 equivalents, 1 to 30 equivalents, or 1 to 10 equivalents, based on the thiol containing B. The number of equivalents of A-X used can be appropriately set by those skilled in the art and can be, for example, 1 to 100 equivalents, 1 to 75 equivalents, 1 to 50 equivalents, 1 to 30 equivalents, or 1 to 10 equivalents, based on the thiol containing B.

[Formula 27]

Ketone bond formation reaction

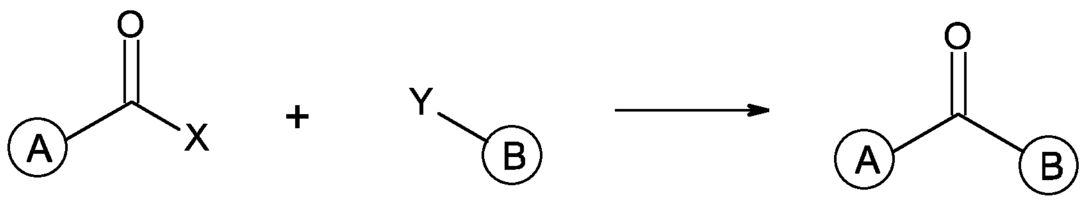

The ketone bond as the linker can be formed through the reaction of carboxylic acid or a derivative thereof with a boron derivative or a zinc derivative.

When A in the formula contains a solid-phase carrier, the reactive functional group X linked to A is hydroxy, an alkoxy group, an aryloxy group, an alkylthio group, or an arylthio group. When X is hydroxy, a mixed acid anhydride can be formed beforehand or in the reaction system by the addition of an acid anhydride and thereby subjected to ketone bond formation reaction. In this context, the acid anhydride is not particularly limited as long as the acid anhydride can be usually used. Examples of the acid anhydride that can be used include benzoic anhydride, acetic anhydride, isobutyric anhydride, isovaleric anhydride, and pivalic anhydride. The number of equivalents of the acid anhydride used can be appropriately set by those skilled in the art and can be, for example, 1 to 100 equivalents, 1 to 75 equivalents, 1 to 50 equivalents, 1 to 30 equivalents, or 1 to 10 equivalents, based on the carboxylic acid containing A. The number of equivalents of the boron derivative or the zinc derivative can be appropriately set by those skilled in the art and can be, for example, 1 to 100 equivalents, 1 to 75 equivalents, 1 to 50 equivalents, 1 to 30 equivalents, or 1 to 10 equivalents, based on the carboxylic acid containing A. The ketone bond as the linker can be formed through the reaction thereof with a boronic acid analog wherein the reactive functional group Y linked to B is a boron derivative, an alkyl borane

reagent derived from a carbon-carbon terminal double bond, or a compound wherein Y is a zinc derivative using a metal catalyst. The number of equivalents of the boronic acid derivative, the alkyl borane reagent, or the zinc derivative used can be appropriately set by those skilled in the art and can be, for example, 1 to 100 equivalents, 1 to 75 equivalents, 1 to 50 equivalents, 1 to 30 equivalents, or 1 to 10 equivalents, based on A-CO-X. In this context, the metal catalyst is not particularly limited as long as the metal catalyst can be usually used. Examples of the metal catalyst that can be used include palladium acetate, $Pd(dba)_2$, $Pd_2(dba)_3$, $Pd_2(dba)_3CHCl_3$, allylpalladium chloride dimers, palladium($\pi$-cinnamyl) chloride dimers, (1- methylallyl)palladium chloride dimers, and (1,5- cyclooctadiene)bis(trimethylsilylmethyl)palladium(II). If necessary, a ligand may be used. Examples of the ligand that can be used include phosphorus acid esters ($P(OEt)_3$ (triethyl phosphite), $P(OPh)_3$ (triphenyl phosphite), etc.), trialkylphosphine ($PCy_3$ (tricyclohexylphosphine), $P(tBu)_3$ (tri-tert-butylphosphine), cataCXium A (di(1-adamantyl)- n-butylphosphine), $(tBu)_2PMe$ (di-tert-butylmethylphosphine), $P(Ad)_3$, neopentyl (tBu)zP (di- tert-butylneopentylphosphine), etc.), triarylphosphine ($PPh_3$ (triphenylphosphine), tri(2- furyl)phosphine, etc.), dialkylbiarylphosphine (XPhos (2-dicyclohexylphosphino-2',4',6'- triisopropylbiphenyl), SPhos (2-dicyclohexylphosphino-2',6'-dimethoxybiphenyl), RuPhos (2-dicyclohexylphosphino-2',6'-diisopropoxybiphenyl), BrettPhos (2- (dicyclohexylphosphino)-3,6-dimethoxy-2',4',6'-triisopropyl-1,1'-biphenyl), tBuXPhos (2-di- tert-butylphosphino-2',4',6'-triisopropylbiphenyl), CPhos (2-dicyclohexylphosphino-2',6'- bis(N,N-dimethylamino)biphenyl), DavePhos (2-dicyclohexylphosphino-2'-(N,N- dimethylamino)biphenyl), RockPhos (2-di(tertiary butyl)phosphino-2',4',6'-triisopropyl-3- methoxy-6-methylbiphenyl, di-tertiary butyl(2',4',6'-triisopropyl-3-methoxy-6-methyl-[1,1'- biphenyl]-2-yl)phosphine), $Me_4$tBuXPhos (2-di-tert-butylphosphino-3,4,5,6-tetramethyl- 2',4',6'-triisopropyl-1,1'-biphenyl), tBuBrettPhos (2-(di-tert-butylphosphino)-2',4',6'- triisopropyl-3,6-dimethoxy-1,1'-biphenyl), AdBrettPhos (2-(di-1 -adamantylphosphino)- 2',4',6'-triisopropyl-3,6-dimethoxy-1,1'-biphenyl), etc.), dialkylmonoarylphosphine (APhos ((4-(N,N-dimethylamino)phenyl)di-tert-butylphosphine), $(tBu)_2PPh$, MorDalPhos (2- morpholinophenyldi(1-adamantyl)phosphine), etc.), bidentate phosphine ligands (dppf (1,1'- bis(diphenylphosphino)ferrocene), dtbpf (1,1'-bis(di-tert-butylphosphino)ferrocene), BINAP (2,2'-bis(diphenylphosphino)-1,1'-binaphthyl), XantPhos (4,5-bis(diphenylphosphino)-9,9- dimethylxanthene), Josiphos ((R)-1-[(Sp)-2-(dicyclohexylphosphino)ferrocenyl]ethyldi-tert- butylphosphine), etc.), and NHC ligands (IPr (1,3-bis(2,6-diisopropylphenyl)imidazol-2- ylidene), SIPr (1,3-bis(2,6-di-i-propylphenyl)imidazolidin-2-ylidene), IMes (1,3-bis(2,4,6- trimethylphenyl)imidazol-2-ylidene), IPent (1,3-bis(2,6-di-3-pentylphenyl)imidazol-2- ylidene), etc.). Alternatively, the ligand described above and a precursor of the palladium catalyst may be used. Examples of the precursor of the palladium catalyst that can be used include first-generation Buchwald precatalysts (G1) (XPhos Pd G1, SPhos Pd G1, RuPhos Pd G1, BrettPhos Pd G1, tBuXPhos Pd G1, etc.), second-generation Buchwald precatalysts (G2) (XPhos Pd G2, SPhos Pd G2, RuPhos Pd G2, BrettPhos Pd G2, tBuXPhos Pd G2, CPhos Pd G2, DavePhos Pd G2, APhos Pd G2, $tBu)_2PPh$ Pd G2, MorDalPhos Pd G2, $PCy_3$ Pd G2, $P(tBu)_3$ Pd G2, cataCXium A Pd G2, XantPhos Pd G2, etc.), third-generation Buchwald precatalysts (G3) (XPhos Pd G3, SPhos Pd G3, RuPhos Pd G3, BrettPhos Pd G3, tBuXPhos Pd G3, CPhos Pd G3, DavePhos Pd G3, RockPhos Pd G3, $Me_4$tBuXPhos Pd G3, tBuBrettPhos Pd G3, AdBrettPhos Pd G3, APhos Pd G3, $(tBu)_2PPh$ Pd G3, MorDalPhos Pd G3, $PCy_3$ Pd G3, $P(tBu)_3$ Pd G3, cataCXium A Pd G3, $tBu)_2PMe$ Pd G3, neopentyl (tBu)zP Pd G3, dppf Pd G3, dtbpf Pd G3, BINAP Pd G3, XantPhos Pd G3, Josiphos Pd G3, etc.), fourth-generation Buchwald precatalysts (G4) (XPhos Pd G4, SPhos Pd G4, RuPhos Pd G4, BrettPhos Pd G4, tBuXPhos Pd G4, CPhos Pd G4, tBuBrettPhos Pd G4, APhos Pd G4, $PCy_3$ Pd G4, $P(tBu)_3$ Pd G4, cataCXium A Pd G4, dppf Pd G4, BINAP Pd G4, XantPhos Pd G4, etc.), dichloropalladium(II) complexes ($Pd(PPh_3)_2c1_2$, $Pd(PCy_3)_2c1_2$, $Pd(dppf)Cl_2$, $Pd(dtbpf)Cl_2$, $(APhos)_2PdCl_2$, etc.), palladium(0) complexes (tetrakistriphenylphosphine palladium(0), bis(tricyclohexylphosphine)palladium(0), bis(tri-tert- butylphosphine)palladium(0), etc.), palladium(I) halide complexes (bromo(tri-tert-butylphosphine)palladium(I) dimers, iodo(tri-tert-butylphosphine)palladium(I) dimers, etc.), phosphine $\pi$-allylpalladium catalysts (XPhos Pd(crotyl)Cl (chloro(crotyl)(2- dicyclohexylphosphino-2',4',6'-triisopropyl-1,1'-biphenyl)palladium(II)), SPhos Pd(crotyl)Cl (chloro(crotyl)(2-dicyclohexylphosphino-2',6'-dimethoxy-1,1'-biphenyl)palladium(In), RuPhos Pd(crotyl)Cl (chloro(crotyl)(2-dicyclohexylphosphino-2',6'-diisopropoxy-1,1'- biphenyl)palladium(II)), [BrettPhos Pd(crotyl)]OTf (crotyl(2-dicyclohexylphosphino-2',4',6'- triisopropyl-3,6-dimethoxy-1,1'-biphenyl)palladium(II) triflate), [tBuXPhos Pd(allyl)]OTf (allyl(2-di-tert-butylphosphino-2',4',6'-triisopropyl-1,1'-biphenyl)palladium(II) triflate), [tBuBrettPhos Pd(allyl)]OTf (allyl(2-di-tert-butylphosphino-3,6-dimethoxy-2',4',6'- triisopropyl-1,1'-biphenyl)palladium(II) triflate), AmPhos Pd(crotyl)Cl (chloro(crotyl)[(p- dimethylaminophenyl)(di-tert-butylphosphine)]palladium(II)), $P(Cy)_3$ Pd(crotyl)Cl (chloro(crotyl)(tricyclohexylphosphine)palladium(II)), $[P(tBu)_3]$ Pd(crotyl)Cl (tri-tert- butylphosphine(chloro)(crotyl)palladium(II)), [BINAP Pd(allyl)]Cl ((R)-BINAP Pd(allyl)]Cl; allyl[(R)-2,2'-bis(diphenylphosphino)-1,1'-binaphthalene]palladium(II) chloride), [XantPhos Pd(allyl)]Cl (allyl[4,5-bis(diphenylphosphino)-9,9-dimethylxanthene]palladium(II) chloride), etc.), PEPPSI catalysts (Pd-PEPPSI IPr, Pd-PEPPSI SIPr ((1,3-bis(2,6- diisopropylphenyl)imidazolidene)(3-chloropyridyl)palladium(II) dichloride), Pd-PEPPSI IPent (1,3-bis(2,6-di-3-pentylphenyl)imidazol-2-ylidene), etc.), (NHC)Pd(allyl)C catalysts (allyl[1,3-bis(2,6-diisopropylphenyl)imidazol-2-ylidene]chloropalladium(II), allyl[1,3- bis(2,6-diisopropylphenyl)-2-imidazolidinylidene]chloropalladium(II), [1,3-bis(2,6- diisopropylphenyl)imidazol-2-ylidene]chloro[3-phenylallyl]palladium(II), etc.), and NHC-Pd naphthoquinone catalysts (1,3-bis(2,6-diisopropylphenyl)imidazol-2-ylidene(1,4- naphthoquinone)palladium(0) dimers, etc.). The number of equivalents of the metal catalyst used can be appropriately set by those skilled in the art and can be, for example, 0.001 to 10 equivalents, 0.001 to 7.5 equivalents, 0.001 to 5 equivalents,

0.01 to 5 equivalents, or 0.01 to 3 equivalents, based on A-X. The number of equivalents of the ligand used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X. The number of equivalents of the catalyst precursor used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on A-X.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL (γ-valcrolactonc), pentyl acetate, methyl propionate, etc.), aromatic hydrocarbon solvents (anisole, toluene, xylene, chlorobenzene, bromobenzene, ethylbenzene, nitrobenzene, α,α,α- trifluorotoluene, 1,2-dichlorobenzene, benzene, etc.), carbonate solvents (dimethyl carbonate, diethyl carbonate, ethylene carbonate, propylene carbonate, etc.), hydrocarbon solvents (hexane, pentane, heptane, cyclohexane, etc.), and others such as acetonitrile, silene, and limonene. A single solvent may be used, or a combined solvent of a plurality of solvents may be used. The reaction temperature can be appropriately set by those skilled in the art and can be, for example, 0 to 200°C, 0 to 140°C, 10 to 100°C, or 20 to 90°C. If necessary, a base may be used. Examples of the base that can be used include sodium carbonate, potassium carbonate, cesium carbonate, sodium phosphate, potassium phosphate, sodium hydroxide, potassium hydroxide, cesium hydroxide, barium hydroxide, tetraalkylammonium hydroxide (tetramethylammonium hydroxide, tetrabutylammonium hydroxide, etc.), NaOtBu, KOtBu, sodium fluoride, potassium fluoride, cesium fluoride, triethylamine, DIPEA, DBU, DBN, MTBD, TMG, BTMG, P1tBu, BEMP, BTPP, P2Et, P2tBu, sodium tetraborate, 1,8- bis(dimethylamino)naphthalene, potassium t-pentoxide, sodium t-pentoxide, lithium t- pentoxide, tetrabutylammonium fluoride, and cesium fluoride. The number of equivalents of the base used can be appropriately set by those skilled in the art and can be, for example, 1 to 100 equivalents, 1 to 75 equivalents, 1 to 50 equivalents, 1 to 30 equivalents, or 1 to 10 equivalents, based on A-CO-X. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the target compound can be obtained by a usual aftertreatment method.

When B in the formula contains a solid-phase carrier, the reactive functional group X linked to A is hydroxy, an alkoxy group, an aryloxy group, an alkylthio group, or an arylthio group, and the reactive functional group Y linked to B is a boron derivative or a zinc derivative. The formation of the ketone bond as the linker can be performed using the already mentioned reagent and reaction conditions. In this context, in the case of using a base, the number of equivalents thereof can be appropriately set by those skilled in the art and can be, for example, 1 to 100 equivalents, 1 to 75 equivalents, 1 to 50 equivalents, 1 to 30 equivalents, or 1 to 10 equivalents, based on B-Y. In the case of using a metal catalyst, the number of equivalents thereof can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on B-Y. In the case of using a ligand, the number of equivalents thereof can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on B-Y. In the case of using a catalyst precursor, the number of equivalents thereof can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on B-Y. The number of equivalents of A-X used can be appropriately set by those skilled in the art and can be, for example, 1 to 100 equivalents, 1 to 75 equivalents, 1 to 50 equivalents, 1 to 30 equivalents, or 1 to 10 equivalents, based on B-Y.

[Formula 28]

Bond formation reaction using nitrogen-containing 5-membered heteroarylene

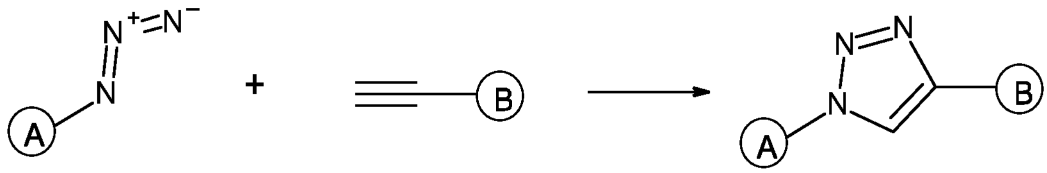

The bond using a triazole ring, an example of the nitrogen-containing 5-membered heteroarylene, as the linker can be formed through the reaction of an azide derivative with an acetylene derivative.

When A in the formula contains a solid-phase carrier, the bond using a triazole ring as the linker can be formed through the reaction thereof with an acetylene derivative containing a propargyl group linked to B using a metal catalyst. The number of equivalents of the acetylene derivative used can be appropriately set by those skilled in the art and can be, for example, 1 to 100 equivalents, 1 to 75 equivalents, 1 to 50 equivalents, 1 to 30 equivalents, or 1 to 10 equivalents, based on the azide derivative containing A. In this context, the metal catalyst is not particularly limited as long as the metal catalyst can be usually used. Examples of the metal catalyst that can be used include metal copper, copper(I) chloride, copper(II) chloride, copper(I) bromide, copper(II) bromide, copper iodide, copper(I) acetate, copper(II) acetate, copper(I) oxide, copper(II) oxide, copper sulfate, zinc acetate, chloro(H5-pentamethylcyclopentadiene)[bis(triphenylphosphine)]ruthenium(II), and carbonyl(dihydrido)tris(triphenylphosphine)ruthenium(II). In the case of using a metal catalyst, the number of equivalents of the metal catalyst used can be appropriately set by those skilled in the art and can be, for example, 0.001 to 10 equivalents, 0.001 to 7.5 equivalents, 0.001 to 5 equivalents, 0.01 to 5 equivalents, or 0.01 to 3 equivalents, based on the azide derivative containing A. Examples of the additive that can be used include sodium L-ascorbate, cellulose, Amberlyst, and tetrabutylammonium bromide. In the case of using an additive, the number of equivalents of the additive used can be appropriately set by those skilled in the art and can be, for example, 0.01 to 100 equivalents, 0.01 to 75 equivalents, 0.01 to 50 equivalents, 0.01 to 30 equivalents, or 0.01 to 10 equivalents, based on the azide derivative containing A.

A solvent can be used in the reaction. Examples of the solvent that can be used include, but are not particularly limited to, halogen solvents (DCM (dichloromethane), chloroform, DCE (1,2-dichloroethane), carbon tetrachloride, etc.), ether solvents (diethyl ether, diisopropyl ether, THF (tetrahydrofuran), 2-methyltetrahydrofuran, 4- methyltetrahydropyran, 1,4-dioxane, DME (1,2-dimethoxyethane), TBME (t-butyl methyl ether), CPME (cyclopentyl methyl ether), isosorbide dimethyl ether, diglyme, triglyme, tetraglyme, etc.), ketone solvents (acetone, MEK (methyl ethyl ketone), MIBK (methyl isobutyl ketone), 4-methyl-2-pentanone, cyclopentanone, cyclohexanone, diethyl ketone, etc.), urea solvents (TMU (N,N,N',N'-tetramethylurea), DMI (1,3-dimethyl-2- imidazolidinone), DMPU (N,N'-dimethylpropyleneurea), etc.), amide solvents (DMF (N,N- dimethylformamide), DMA (N,N-dimethylacetamide), NFM (N-formylmorpholine), NMP (N-methyl-2-pyrrolidone), NEP (N-ethyl-2-pyrrolidone), NBP (N-butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3- methylsulfolane, ethylmethylsulfone, ethylisopropylsulfone, etc.), ester solvents (methyl acetate, ethyl acetate, propyl acetate, isopropyl acetate, butyl acetate, isobutyl acetate, GVL ($\gamma$-valcrolactonc), pentyl acetate, methyl propionate, etc.), aromatic hydrocarbon solvents (anisole, toluene, xylene, chlorobenzene, bromobenzene, ethylbenzene, nitrobenzene, $\alpha,\alpha,\alpha$- trifluorotoluene, 1,2-dichlorobenzene, benzene, tetralin, pyridine, quinoline, etc.), alcohol solvents (ethanol, isopropanol, tert-butanol, tert-amyl alcohol, etc.), hydrocarbon solvents (hexane, pentane, heptane, cyclohexane, methylcyclohexane, decalin, etc.), and others such as acetonitrile, water, acetic acid, silene, and limonene. A single solvent may be used, or a combined solvent of a plurality of solvents may be used. The reaction temperature can be appropriately set by those skilled in the art and can be, for example, -20 to 110°C, 0 to 100°C, 10 to 90°C or 20 to 80°C. The reaction may be carried out under a high-temperature condition of 100°C or higher using an excessive amount of a reagent without the use of a solvent. If necessary, a base may be used. Examples of the base that can be used include pyridine, 2,6-lutidine, 2,4,6-collidine, quinoline, DTBP, DTBMP, triethylamine, DIPEA, 4- methylmorpholine, NMI, DMAP, 1,4-diazabicyclo[2.2.2]octane (DABCO), potassium hydride, sodium hydride, sodium amide, lithium amide, KOH, NaOH, LiOH, sodium carbonate, potassium carbonate, cesium carbonate, sodium bicarbonate, potassium phosphate, $K_2HPO_4$, $KHCO_3$, $NaHCO_3$, $Na_2HPO_4$, sodium tetraborate, DBU, DBN, MTBD, BTMG, TMG, 1,8-bis(dimethylamino)naphthalene, LiOtBu, NaOtBu, KOtBu, potassium t-pentoxide, sodium t-pentoxide, N,N-dimethylaniline, P1-tBu, P2tBu, tetrabutylammonium hydroxide, benzyl triethylammonium hydroxide, tetrabutylammonium fluoride, and cesium fluoride. In the case of using a base, the number of equivalents of the base used can be appropriately set by those skilled in the art and can be, for example, 1 to 100 equivalents, 1 to 75 equivalents, 1 to 50 equivalents, 1 to 30 equivalents,

or 1 to 10 equivalents, based on the azide derivative containing A. The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers.

After the completion of the reaction, the compounds of interest can be obtained by a usual aftertreatment method.

In one aspect of the present invention, two or more core blocks are linked via a linker to constitute a chemical structure of a compound contained in the library. In one embodiment, the compound constituting the library can be prepared by linking core blocks through linker formation reaction. In this context, the core blocks are not particularly limited as long as the core blocks are chemical structures that are linked via a linker in the compound molecule. In one embodiment, two or more chemical structures separated via a linker in the molecular structure of each compound contained in the library are each defined as a core block. In this context, the linker means a linking moiety that is formed in compound synthesis. In another embodiment, the compound contained in the library is prepared through linker formation reaction with reactive functional groups of building blocks, and means two or more chemical structures other than the linker in the branched structure of the compound.

In one aspect of the present invention, the library comprises one or two or more compounds prepared by linking core blocks through linker formation reaction, and may further comprise an additional compound comprising a linker that corresponds to the linker contained in the compound. In this context, the additional compound may be a compound prepared without linker formation reaction and may be, for example, a compound obtained by purchase.

In one aspect of the present invention, when a single bond serves as a linker, the linker links two chemical structures selected from $C_{5-10}$ aryl, 5- to 10-membered heteroaryl, 5- to 8-membered heterocyclyl, and $C_{3-10}$ cycloalkyl and the chemical structures constitute the whole or a portion of core blocks to be linked by the linker.

In one aspect of the present invention, the core blocks are each independently a monovalent to trivalent core block represented by any of the following formulas.

1) Divalent core block:
A divalent core block represented by

1-1)

$$-A^1-;$$

1-2)

$$-X^1-Q^1-X^2-;$$

or
1-3)

$$-X^3-Q^2-X^4-Q^3-X^5-,$$

wherein $A^1$ is $C_{1-10}$ alkylene, and the alkylene optionally has one or more substituents each independently selected from Ra;

$Q^1$ is selected from $C_{5-10}$ arylene, 5- to 10-membered heteroarylene, 5- to 8- membered nonaromatic heterocyclylene, and $C_{3-10}$ cycloalkylene, and each of these groups optionally has one or more substituents each independently selected from Rb;

$X^1$ and $X^2$ are each independently a single bond or $C_{1-10}$ alkylene, and the alkylene optionally has one or more substituents each independently selected from Ra;

$Q^2$ and $Q^3$ are each independently selected from $C_{5-10}$ arylene, 5- to 10-membered heteroarylene, 5- to 8-membered nonaromatic heterocyclylene, and $C_{3-10}$ cycloalkylene, and each of these groups optionally has one or more substituents each independently selected from Rb; and

$X^3$, $X^4$ and $X^5$ are each independently a single bond or $C_{1-10}$ alkylene, and the alkylene optionally has one or more substituents each independently selected from Ra;

in the above definition, when each of $A^1$, $X^1$, $X^2$, $X^3$, $X^4$, and $X^5$ is $C_{2-10}$ alkylene, one to three groups selected from -O-, -S-, and $-NR^{11}-$ are optionally inserted to the alkylene, and $R^{11}$ is a hydrogen atom or $C_{1-6}$ alkyl;

Ra is independently selected from the group consisting of a halogen atom, hydroxy, $C_{1-6}$ alkoxy, cyano,

($C_{1-6}$ alkoxy)carbonyl, carboxy, ($C_{1-6}$ alkoxy)carbonylamino, ($C_{1-6}$ alkyl)carbonylamino, amino, $C_{1-6}$ alkylamino, di($C_{1-6}$ alkyl)amino, aminocarbonyl, ($C_{1-6}$ alkyl)aminocarbonyl, di($C_{1-6}$ alkyl)aminocarbonyl, $C_{1-6}$ alkylsulfonylamino, $C_{3-10}$ cycloalkylsulfonylamino, $C_{7-14}$ aralkyl, $C_{3-10}$ cycloalkyl optionally substituted by one or two or more substituents selected from Rc, $C_{6-10}$ aryl optionally substituted by one or two or more substituents selected from Rd, 5- to 10-membered heteroaryl optionally substituted by one or two or more substituents selected from Rc, 4- to 12-membered nonaromatic heterocyclyl optionally substituted by one or two or more substituents selected from Rc, $C_{6-10}$ aryloxy optionally substituted by one or two or more substituents selected from Rd, $C_{6-10}$ arylsulfanyl optionally substituted by one or two or more substituents selected from Rd, and adamantyl;

Rb is independently selected from the group consisting of a halogen atom, hydroxy, $C_{1-6}$ alkyl optionally substituted by one or two or more substituents selected from Ra, $C_{1-6}$ alkoxy optionally substituted by one or two or more substituents selected from Ra, cyano, ($C_{1-6}$ alkoxy)carbonyl, carboxy, $-NR^{21}R^{22}$, $-CONR^{23}R^{24}$, di($C_{1-6}$ alkyl)phosphono, $C_{3-10}$ cycloalkyl optionally substituted by one or two or more substituents selected from Rc, $C_{6-10}$ aryl optionally substituted by one or two or more substituents selected from Rd, 5- to 10- membered heteroaryl optionally substituted by one or two or more substituents selected from Rc, and 3- to 12-membered nonaromatic heterocyclyl optionally substituted by one or two or more substituents selected from Rc;

Rc is independently selected from the group consisting of nitro, hydroxy, $C_{1-6}$ alkyl optionally substituted by one or two or more halogen atoms, $C_{1-6}$ alkoxy optionally substituted by one or two or more halogen atoms, a halogen atom, amino, cyano, $C_{1-6}$ alkylamino optionally substituted by one or two or more hydroxy groups, di($C_{1-6}$ alkyl)amino optionally substituted by one or two or more hydroxy groups, $C_{3-10}$ cycloalkylamino, ($C_{1-6}$ alkyl)carbonyl, ($C_{1-6}$ alkoxy)carbonyl, $C_{1-6}$ alkylsulfonyl, $C_{3-10}$ cycloalkylsulfonyl, $C_{7-14}$ aralkyl optionally substituted by one or two or more substituents selected from Re, $C_{6-10}$ aryl optionally substituted by one or two or more substituents selected from Re, $C_{3-10}$ cycloalkyl optionally substituted by one or two or more substituents selected from Re, 3- to 12- membered nonaromatic heterocyclyl optionally substituted by one or two or more substituents selected from Re, 5- to 10-membered heteroaryl optionally substituted by one or two or more substituents selected from Re, and oxo;

Rd is independently selected from the group consisting of nitro, hydroxy, $C_{1-6}$ alkyl optionally substituted by one or two or more halogen atoms, $C_{1-6}$ alkoxy optionally substituted by one or two or more halogen atoms, a halogen atom, amino, cyano, $C_{1-6}$ alkylamino, di($C_{1-6}$ alkyl)amino, $C_{1-6}$ alkylcarbonyl, ($C_{1-6}$ alkoxy)carbonyl, ($C_{1-6}$ alkoxy)carbonylamino, N-($C_{1-6}$ alkoxy)carbonyl-N-($C_{1-6}$ alkyl)amino, $C_{1-6}$ alkylsulfonyl, $C_{3-8}$ cycloalkylsulfonyl, $C_{7-14}$ aralkyl optionally substituted by one or two or more substituents selected from Re, $C_{6-10}$ aryl optionally substituted by one or two or more substituents selected from Re, $C_{3-8}$ cycloalkyl optionally substituted by one or two or more substituents selected from Re, 3- to 12-membered nonaromatic heterocyclyl optionally substituted by one or two or more substituents selected from Re, and 5- to 10-membered heteroaryl optionally substituted by one or two or more substituents selected from Re;

Re is independently selected from the group consisting of nitro, hydroxy, $C_{1-6}$ alkyl optionally substituted by one or two or more substituents selected from a halogen atom and hydroxy, a halogen atom, amino, cyano, $C_{1-6}$ alkylamino, di($C_{1-6}$ alkyl)amino, $C_{1-6}$ alkylcarbonyl optionally substituted by one or two or more substituents selected from phenyl and hydroxy, ($C_{1-6}$ alkoxy)carbonyl optionally substituted by one or two or more substituents selected from phenyl and hydroxy, $C_{1-6}$ alkylsulfonyl, $C_{3-8}$ cycloalkylsulfonyl, $C_{1-6}$ alkylsulfonylamino, $C_{3-8}$ cycloalkylsulfonylamino, and oxo;

$R^{21}$ is selected from the group consisting of a hydrogen atom, $C_{1-6}$ alkyl optionally substituted by one or two or more substituents selected from Ra, $C_{6-10}$ aryl optionally substituted by one or two or more substituents selected from Rd, 4- to 12-membered heterocyclyl optionally substituted by one or two or more substituents selected from Rc, 5- to 10-membered heteroaryl optionally substituted by one or two or more substituents selected from Rc, ($C_{1-6}$ alkoxy)carbonyl optionally substituted by one or two or more substituents selected from Ra, ($C_{1-6}$ alkyl)carbonyl optionally substituted by one or two or more substituents selected from Ra, ($C_{3-10}$ cycloalkyl)carbonyl, ($C_{6-10}$ aryl)carbonyl optionally substituted by one or two or more substituents selected from Rd, (3- to 12-membered nonaromatic heterocyclyl)carbonyl optionally substituted by one or two or more substituents selected from Rc, (5- to 10-membered heteroaryl)carbonyl optionally substituted by one or two or more substituents selected from Rg, aminocarbonyl, ($C_{1-6}$ alkyl)aminocarbonyl optionally substituted by one or two or more substituents selected from Ra, di($C_{1-6}$ alkyl)aminocarbonyl optionally substituted by one or two or more substituents selected from Ra, $C_{1-6}$ alkylsulfonyl optionally substituted by one or two or more halogen atoms, $C_{7-14}$ aralkylsulfonyl, $C_{3-10}$ cycloalkylsulfonyl, aminosulfonyl, $C_{1-6}$ alkylaminosulfonyl, di($C_{1-6}$ alkyl)aminosulfonyl, and di($C_{1-6}$ alkyl)phosphono;

$R^{22}$ is selected from the group consisting of a hydrogen atom and $C_{1-6}$ alkyl optionally substituted by one or two or more substituents selected from Ra;

$R^{23}$ is selected from the group consisting of a hydrogen atom, $C_{1-6}$ alkyl optionally substituted by one or two or more substituents selected from Ra, [($C_{1-6}$ alkyl)amino]$C_{1-6}$ alkyl optionally substituted by one or two or more substituents selected from Ra, [di($C_{1-6}$ alkyl)amino]$C_{1-6}$ alkyl optionally substituted by one or two or more substituents selected from Ra, $C_{3-10}$ cycloalkyl optionally substituted by one or two or more substituents selected from Rc, $C_{6-10}$ aryl optionally substituted by one or two or more substituents selected from Rd, 5- to 10-membered heteroaryl optionally substituted by one or two or more substituents selected from Rc, and 3- to 12-membered nonaromatic heterocyclyl optionally substituted by one or two or more substituents selected from Rc; and

$R^{24}$ is selected from the group consisting of a hydrogen atom and $C_{1-6}$ alkyl optionally substituted by one or two or more substituents selected from Ra.

2) Monovalent core block:

A monovalent core block represented by

2-1)

$-A^2$;

2-2)

$-X^1-Q^1-X^6$;

or

2-3)

$-X^3-Q^2-X^4-Q^3-X^7$,

wherein $A^2$ is $C_{1-10}$ alkyl, and the alkyl optionally has one or more substituents each independently selected from Ra;

$Q^1$ is selected from $C_{5-10}$ arylene, 5- to 10-membered heteroarylene, 5- to 8- membered nonaromatic heterocyclylene, and $C_{3-10}$ cycloalkylene, and each of these groups optionally has one or more substituents each independently selected from Rb;

$X^6$ and $X^7$ are each independently a hydrogen atom or $C_{1-10}$ alkyl, and the alkyl optionally has one or more substituents each independently selected from Ra;

$Q^2$ and $Q^3$ are each independently selected from $C_{5-10}$ arylene, 5- to 10-membered heteroarylene, 5- to 8-membered nonaromatic heterocyclylene, and $C_{3-10}$ cycloalkylene, and each of these groups optionally has one or more substituents each independently selected from Rb;

$X^1$, $X^3$ and $X^4$ are each independently a single bond or $C_{1-10}$ alkylene, and the alkylene optionally has one or more substituents each independently selected from Ra;

in the above definition, when each of $X^1$, $X^3$, and $X^4$ is $C_{2-10}$ alkylene, one to three groups selected from -O-, -S-, and $-NR^{11}-$ are optionally inserted to the alkylene, and $R^{11}$ is a hydrogen atom or $C_{1-6}$ alkyl;

in the above definition, when each of $X^6$ and $X^7$ is $C_{2-10}$ alkyl, one to three groups selected from -O-, -S-, and $-NR^{11}-$ are optionally inserted to the alkyl, and $R^{11}$ is a hydrogen atom or $C_{1-6}$ alkyl;

Ra is independently selected from the group consisting of a halogen atom, hydroxy, $C_{1-6}$ alkoxy, cyano, ($C_{1-6}$ alkoxy)carbonyl, carboxy, ($C_{1-6}$ alkoxy)carbonylamino, ($C_{1-6}$ alkyl)carbonylamino, amino, $C_{1-6}$ alkylamino, di($C_{1-6}$ alkyl)amino, aminocarbonyl, ($C_{1-6}$ alkyl)aminocarbonyl, di($C_{1-6}$ alkyl)aminocarbonyl, $C_{1-6}$ alkylsulfonylamino, $C_{3-10}$ cycloalkylsulfonylamino, $C_{7-14}$ aralkyl, $C_{3-10}$ cycloalkyl optionally substituted by one or two or more substituents selected from Rc, $C_{6-10}$ aryl optionally substituted by one or two or more substituents selected from Rd, 5- to 10-membered heteroaryl optionally substituted by one or two or more substituents selected from Rc, 4- to 12-membered nonaromatic heterocyclyl optionally substituted by one or two or more substituents selected from Rc, $C_{6-10}$ aryloxy optionally substituted by one or two or more substituents selected from Rd, $C_{6-10}$ arylsulfanyl optionally substituted by one or two or more substituents selected from Rd, and adamantyl;

Rb is independently selected from the group consisting of a halogen atom, hydroxy, $C_{1-6}$ alkyl optionally substituted by one or two or more substituents selected from Ra, $C_{1-6}$ alkoxy optionally substituted by one or two or more substituents selected from Ra, cyano, ($C_{1-6}$ alkoxy)carbonyl, carboxy, $-NR^{21}R^{22}$, $-CONR^{23}R^{24}$, di($C_{1-6}$ alkyl)phosphono, $C_{3-10}$ cycloalkyl optionally substituted by one or two or more sub-

stituents selected from Rc, $C_{6-10}$ aryl optionally substituted by one or two or more substituents selected from Rd, 5- to 10- membered heteroaryl optionally substituted by one or two or more substituents selected from Rc, and 3- to 12-membered nonaromatic heterocyclyl optionally substituted by one or two or more substituents selected from Rc;

Rc is independently selected from the group consisting of nitro, hydroxy, $C_{1-6}$ alkyl optionally substituted by one or two or more halogen atoms, $C_{1-6}$ alkoxy optionally substituted by one or two or more halogen atoms, a halogen atom, amino, cyano, $C_{1-6}$ alkylamino optionally substituted by one or two or more hydroxy groups, di($C_{1-6}$ alkyl)amino optionally substituted by one or two or more hydroxy groups, $C_{3-10}$ cycloalkylamino, ($C_{1-6}$ alkyl)carbonyl, ($C_{1-6}$ alkoxy)carbonyl, $C_{1-6}$ alkylsulfonyl, $C_{3-10}$ cycloalkylsulfonyl, $C_{7-14}$ aralkyl optionally substituted by one or two or more substituents selected from Re, $C_{6-10}$ aryl optionally substituted by one or two or more substituents selected from Re, $C_{3-10}$ cycloalkyl optionally substituted by one or two or more substituents selected from Re, 3- to 12- membered nonaromatic heterocyclyl optionally substituted by one or two or more substituents selected from Re, 5- to 10-membered heteroaryl optionally substituted by one or two or more substituents selected from Re, and oxo;

Rd is independently selected from the group consisting of nitro, hydroxy, $C_{1-6}$ alkyl optionally substituted by one or two or more halogen atoms, $C_{1-6}$ alkoxy optionally substituted by one or two or more halogen atoms, a halogen atom, amino, cyano, $C_{1-6}$ alkylamino, di($C_{1-6}$ alkyl)amino, $C_{1-6}$ alkylcarbonyl, ($C_{1-6}$ alkoxy)carbonyl, ($C_{1-6}$ alkoxy)carbonylamino, N-($C_{1-6}$ alkoxy)carbonyl-N-($C_{1-6}$ alkyl)amino, $C_{1-6}$ alkylsulfonyl, $C_{3-8}$ cycloalkylsulfonyl, $C_{7-14}$ aralkyl optionally substituted by one or two or more substituents selected from Re, $C_{6-10}$ aryl optionally substituted by one or two or more substituents selected from Re, $C_{3-8}$ cycloalkyl optionally substituted by one or two or more substituents selected from Re, 3- to 12-membered nonaromatic heterocyclyl optionally substituted by one or two or more substituents selected from Re, and 5- to 10-membered heteroaryl optionally substituted by one or two or more substituents selected from Re;

Re is independently selected from the group consisting of nitro, hydroxy, $C_{1-6}$ alkyl optionally substituted by one or two or more substituents selected from a halogen atom and hydroxy, a halogen atom, amino, cyano, $C_{1-6}$ alkylamino, di($C_{1-6}$ alkyl)amino, $C_{1-6}$ alkylcarbonyl optionally substituted by one or two or more substituents selected from phenyl and hydroxy, ($C_{1-6}$ alkoxy)carbonyl optionally substituted by one or two or more substituents selected from phenyl and hydroxy, $C_{1-6}$ alkylsulfonyl, $C_{3-8}$ cycloalkylsulfonyl, $C_{1-6}$ alkylsulfonylamino, $C_{3-8}$ cycloalkylsulfonylamino, and oxo;

$R^{21}$ is selected from the group consisting of a hydrogen atom, $C_{1-6}$ alkyl optionally substituted by one or two or more substituents selected from Ra, $C_{6-10}$ aryl optionally substituted by one or two or more substituents selected from Rd, 4- to 12-membered heterocyclyl optionally substituted by one or two or more substituents selected from Rc, 5- to 10-membered heteroaryl optionally substituted by one or two or more substituents selected from Rc, ($C_{1-6}$ alkoxy)carbonyl optionally substituted by one or two or more substituents selected from Ra, ($C_{1-6}$ alkyl)carbonyl optionally substituted by one or two or more substituents selected from Ra, ($C_{3-10}$ cycloalkyl)carbonyl, ($C_{6-10}$ aryl)carbonyl optionally substituted by one or two or more substituents selected from Rd, (3- to 12-membered nonaromatic heterocyclyl)carbonyl optionally substituted by one or two or more substituents selected from Rc, (5- to 10-membered heteroaryl)carbonyl optionally substituted by one or two or more substituents selected from Rg, aminocarbonyl, ($C_{1-6}$ alkyl)aminocarbonyl optionally substituted by one or two or more substituents selected from Ra, di($C_{1-6}$ alkyl)aminocarbonyl optionally substituted by one or two or more substituents selected from Ra, $C_{1-6}$ alkylsulfonyl optionally substituted by one or two or more halogen atoms, $C_{7-14}$ aralkylsulfonyl, $C_{3-10}$ cycloalkylsulfonyl, aminosulfonyl, $C_{1-6}$ alkylaminosulfonyl, di($C_{1-6}$ alkyl)aminosulfonyl, and di($C_{1-6}$ alkyl)phosphono;

$R^{22}$ is selected from the group consisting of a hydrogen atom and $C_{1-6}$ alkyl optionally substituted by one or two or more substituents selected from Ra;

$R^{23}$ is selected from the group consisting of a hydrogen atom, $C_{1-6}$ alkyl optionally substituted by one or two or more substituents selected from Ra, [($C_{1-6}$ alkyl)amino]$C_{1-6}$ alkyl optionally substituted by one or two or more substituents selected from Ra, [di($C_{1-6}$ alkyl)amino]$C_{1-6}$ alkyl optionally substituted by one or two or more substituents selected from Ra, $C_{3-10}$ cycloalkyl optionally substituted by one or two or more substituents selected from Rc, $C_{6-10}$ aryl optionally substituted by one or two or more substituents selected from Rd, 5- to 10-membered heteroaryl optionally substituted by one or two or more substituents selected from Rc, and 3- to 12-membered nonaromatic heterocyclyl optionally substituted by one or two or more substituents selected from Rc; and

$R^{24}$ is selected from the group consisting of a hydrogen atom and $C_{1-6}$ alkyl optionally substituted by one or two or more substituents selected from Ra.

3) Trivalent core block:

A trivalent core block further having one linker binding position at an arbitrary position in the divalent core block.

In one aspect of the present invention, the core blocks are each independently a monovalent or divalent core block represented by any of the following formulas.

1) Divalent core block:
A divalent core block represented by

1-1)

$-A^1-$;

1-2)

$-X^1-Q^1-X^2-$;

or
1-3)

$-X^3-Q^2-X^4-Q^3-X^5-$,

wherein $A^1$ is $C_{1-10}$ alkylene, and the alkylene optionally has one or more substituents each independently selected from Ra;
$Q^1$ is selected from $C_{5-10}$ arylene, 5- to 10-membered heteroarylene, 5- to 8- membered nonaromatic heterocyclylene, and $C_{3-10}$ cycloalkylene, and each of these groups optionally has one or more substituents each independently selected from Rb;
$X^1$ and $X^2$ are each independently a single bond or $C_{1-10}$ alkylene, and the alkylene optionally has one or more substituents each independently selected from Ra;
$Q^2$ and $Q^3$ are each independently selected from $C_{5-10}$ arylene, 5- to 10-membered heteroarylene, 5- to 8-membered nonaromatic heterocyclylene, and $C_{3-10}$ cycloalkylene, and each of these groups optionally has one or more substituents each independently selected from Rb;
$X^3$, $X^4$ and $X^5$ are each independently a single bond or $C_{1-10}$ alkylene, and the alkylene optionally has one or more substituents each independently selected from Ra;
in the above definition, when each of $A^1$, $X^1$, $X^2$, $X^3$, $X^4$, and $X^5$ is $C_{2-10}$ alkylene, one to three groups selected from -O-, -S-, and $-NR^{11}-$ are optionally inserted to the alkylene, and $R^{11}$ is a hydrogen atom or $C_{1-6}$ alkyl;
Ra is independently selected from the group consisting of a halogen atom, hydroxy, $C_{1-6}$ alkoxy, cyano, ($C_{1-6}$ alkoxy)carbonyl, carboxy, ($C_{1-6}$ alkoxy)carbonylamino, ($C_{1-6}$ alkyl)carbonylamino, amino, $C_{1-6}$ alkylamino, di($C_{1-6}$ alkyl)amino, aminocarbonyl, ($C_{1-6}$ alkyl)aminocarbonyl, di($C_{1-6}$ alkyl)aminocarbonyl, $C_{1-6}$ alkylsulfonylamino, $C_{3-10}$ cycloalkylsulfonylamino, $C_{7-14}$ aralkyl, $C_{3-10}$ cycloalkyl, $C_{6-10}$ aryl optionally substituted by one or two or more halogen atoms, 5- to 10-membered heteroaryl optionally substituted by one or two or more substituents selected from Rc, 4- to 12-membered nonaromatic heterocyclyl optionally substituted by one or two or more substituents selected from Rc, $C_{6-10}$ aryloxy optionally substituted by one or two or more halogen atoms, $C_{6-10}$ arylsulfanyl optionally substituted by one or two or more halogen atoms, and adamantyl; and
Rb is independently selected from the group consisting of a halogen atom, hydroxy, $C_{1-6}$ alkyl optionally substituted by one or two or more substituents selected from Ra, $C_{1-6}$ alkoxy optionally substituted by one or two or more substituents selected from Ra, cyano, ($C_{1-6}$ alkoxy)carbonyl, carboxy, $C_{3-10}$ cycloalkyl, $C_{6-10}$ aryl optionally substituted by one or two or more halogen atoms, 5- to 10-membered heteroaryl optionally substituted by one or two or more halogen atoms, and 3- to 12-membered nonaromatic heterocyclyl.

2) Monovalent core block:
A monovalent core block represented by

2-1)

$-A^2$;

2-2)

$-X^1-Q^1-X^6$;

or

2-3)

$-X^3-Q^2-X^4-Q^3-X^7$,

wherein $A^2$ is $C_{1-10}$ alkyl, and the alkyl optionally has one or more substituents each independently selected from Ra;

$Q^1$ is selected from $C_{5-10}$ arylene, 5- to 10-membered heteroarylene, 5- to 8- membered nonaromatic heterocyclylene, and $C_{3-10}$ cycloalkylene, and each of these groups optionally has one or more substituents each independently selected from Rb;

$X^6$ and $X^7$ are each independently a hydrogen atom or $C_{1-10}$ alkyl, and the alkyl optionally has one or more substituents each independently selected from Ra;

$Q^2$ and $Q^3$ are each independently selected from $C_{5-10}$ arylene, 5- to 10-membered heteroarylene, 5- to 8-membered nonaromatic heterocyclylene, and $C_{3-10}$ cycloalkylene, and each of these groups optionally has one or more substituents each independently selected from Rb;

$X^1$, $X^3$ and $X^4$ are each independently a single bond or $C_{1-10}$ alkylene, and the alkylene optionally has one or more substituents each independently selected from Ra;

in the above definition, when each of $X^1$, $X^3$, and $X^4$ is $C_{2-10}$ alkylene, one to three groups selected from -O-, -S-, and $-NR^{11}-$ are optionally inserted to the alkylene, and $R^{11}$ is a hydrogen atom or $C_{1-6}$ alkyl;

in the above definition, when each of $X^2$ and $X^5$ is $C_{2-10}$ alkyl, one to three groups selected from -O-, -S-, and $-NR^{11}-$ are optionally inserted to the alkyl, and $R^{11}$ is a hydrogen atom or $C_{1-6}$ alkyl;

Ra is independently selected from the group consisting of a halogen atom, hydroxy, $C_{1-6}$ alkoxy, cyano, ($C_{1-6}$ alkoxy)carbonyl, carboxy, ($C_{1-6}$ alkoxy)carbonylamino, ($C_{1-6}$ alkyl)carbonylamino, amino, $C_{1-6}$ alkylamino, di($C_{1-6}$ alkyl)amino, aminocarbonyl, ($C_{1-6}$ alkyl)aminocarbonyl, di($C_{1-6}$ alkyl)aminocarbonyl, $C_{1-6}$ alkylsulfonylamino, $C_{3-10}$ cycloalkylsulfonylamino, $C_{7-14}$ aralkyl, $C_{3-10}$ cycloalkyl, $C_{6-10}$ aryl optionally substituted by one or two or more halogen atoms, 5- to 10-membered heteroaryl optionally substituted by one or two or more substituents selected from Rc, 4- to 12-membered nonaromatic heterocyclyl optionally substituted by one or two or more substituents selected from Rc, $C_{6-10}$ aryloxy optionally substituted by one or two or more halogen atoms, $C_{6-10}$ arylsulfanyl optionally substituted by one or two or more halogen atoms, and adamantyl; and

Rb is independently selected from the group consisting of a halogen atom, hydroxy, $C_{1-6}$ alkyl optionally substituted by one or two or more substituents selected from Ra, $C_{1-6}$ alkoxy optionally substituted by one or two or more substituents selected from Ra, cyano, ($C_{1-6}$ alkoxy)carbonyl, carboxy, $C_{3-10}$ cycloalkyl, $C_{6-10}$ aryl optionally substituted by one or two or more halogen atoms, 5- to 10-membered heteroaryl optionally substituted by one or two or more halogen atoms, and 3- to 12-membered nonaromatic heterocyclyl.

In one aspect of the present invention, the core blocks are each independently a monovalent or divalent core block represented by any of the following formulas.

1) Divalent core block:

A divalent core block represented by

1-1)

$-A^1-$;

1-2)

$-X^1-Q^1-X^2-$;

or

1-3)

$-X^3-Q^2-X^4-Q^3-X^5-$,

wherein $A^1$ is $C_{1-10}$ alkylene, and the alkylene optionally has one or more substituents each independently selected from Ra;
each of $Q^1$, $Q^2$ and $Q^3$ is a divalent group composed of a ring selected from the group consisting of a benzene ring, a pyridine ring, a piperidine ring, a piperazine ring, a pyrimidine ring, a pyrazine ring, a pyridazine ring, a pyrazole ring, an imidazole ring, a thiophene ring, a furan ring, and a naphthalene ring, and each of these groups optionally has one or more substituents each independently selected from Rb;
$X^1$ and $X^2$ are each independently a single bond or $C_{1-10}$ alkylene, and the alkylene optionally has one or more substituents each independently selected from Ra;
$X^3$, $X^4$ and $X^5$ are each independently a single bond or $C_{1-16}$ alkylene, and the alkylene optionally has one or more substituents each independently selected from Ra;
in the above definition, when each of $A^1$, $X^1$, $X^2$, $X^3$, $X^4$, and $X^5$ is $C_{2-10}$ alkylene, one to three groups selected from -O-, -S-, and $-NR^{11}-$ are optionally inserted to the alkylene, and $R^{11}$ is a hydrogen atom or $C_{1-6}$ alkyl;
Ra is independently selected from the group consisting of a halogen atom, hydroxy, $C_{1-6}$ alkoxy, cyano, ($C_{1-6}$ alkoxy)carbonyl, carboxy, ($C_{1-6}$ alkoxy)carbonylamino, ($C_{1-6}$ alkyl)carbonylamino, amino, $C_{1-6}$ alkylamino, di($C_{1-6}$ alkyl)amino, aminocarbonyl, ($C_{1-6}$ alkyl)aminocarbonyl, di($C_{1-6}$ alkyl)aminocarbonyl, $C_{1-6}$ alkylsulfonylamino, $C_{3-10}$ cycloalkylsulfonylamino, $C_{7-14}$ aralkyl, $C_{3-10}$ cycloalkyl, $C_{6-10}$ aryl optionally substituted by one or two or more halogen atoms, 5- to 10-membered heteroaryl optionally substituted by one or two or more substituents selected from Rc, 4- to 12-membered nonaromatic heterocyclyl optionally substituted by one or two or more substituents selected from Rc, $C_{6-10}$ aryloxy optionally substituted by one or two or more halogen atoms, $C_{6-10}$ arylsulfanyl optionally substituted by one or two or more halogen atoms, and adamantyl; and
Rb is independently selected from the group consisting of a halogen atom, hydroxy, $C_{1-6}$ alkyl optionally substituted by one or two or more substituents selected from Ra, $C_{1-6}$ alkoxy optionally substituted by one or two or more substituents selected from Ra, cyano, ($C_{1-6}$ alkoxy)carbonyl, carboxy, $C_{3-10}$ cycloalkyl, $C_{6-10}$ aryl optionally substituted by one or two or more halogen atoms, 5- to 10-membered heteroaryl optionally substituted by one or two or more halogen atoms, and 3- to 12-membered nonaromatic heterocyclyl.

2) Monovalent core block:
A monovalent core block represented by

2-1)

$$-A^2;$$

2-2)

$$-X^1-Q^1-X^6;$$

or 2-3)

$$-X^3-Q^2-X^4-Q^3-X^7,$$

wherein $A^2$ is $C_{1-10}$ alkyl, and the alkyl optionally has one or more substituents each independently selected from Ra;
each of $Q^1$, $Q^2$ and $Q^3$ is a divalent group composed of a ring selected from the group consisting of a benzene ring, a pyridine ring, a piperidine ring, a piperazine ring, a pyrimidine ring, a pyrazine ring, a pyridazine ring, a pyrazole ring, an imidazole ring, a thiophene ring, a furan ring, and a naphthalene ring, and each of these groups optionally has one or more substituents each independently selected from Rb;
$X^6$ and $X^7$ are each independently a hydrogen atom or $C_{1-10}$ alkyl, and the alkyl optionally has one or more substituents each independently selected from Ra;
$X^1$, $X^3$ and $X^4$ are each independently a single bond or $C_{1-10}$ alkylene, and the alkylene optionally has one or more substituents each independently selected from Ra;
in the above definition, when each of $X^1$, $X^3$, and $X^4$ is $C_{2-10}$ alkylene, one to three groups selected from

-O-, -S-, and -$NR^{11}$- are optionally inserted to the alkylene, and $R^{11}$ is a hydrogen atom or $C_{1-6}$ alkyl;
in the above definition, when each of $X^6$ and $X^7$ is $C_{2-10}$ alkyl, one to three groups selected from -O-, -S-, and -$NR^{11}$- are optionally inserted to the alkyl, and $R^{11}$ is a hydrogen atom or $C_{1-6}$ alkyl;
Ra is independently selected from the group consisting of a halogen atom, hydroxy, $C_{1-6}$ alkoxy, cyano, ($C_{1-6}$ alkoxy)carbonyl, carboxy, ($C_{1-6}$ alkoxy)carbonylamino, ($C_{1-6}$ alkyl)carbonylamino, amino, $C_{1-6}$ alkylamino, di($C_{1-6}$ alkyl)amino, aminocarbonyl, ($C_{1-6}$ alkyl)aminocarbonyl, di($C_{1-6}$ alkyl)aminocarbonyl, $C_{1-6}$ alkylsulfonylamino, $C_{3-10}$ cycloalkylsulfonylamino, $C_{7-14}$ aralkyl, $C_{3-10}$ cycloalkyl, $C_{6-10}$ aryl optionally substituted by one or two or more halogen atoms, 5- to 10-membered heteroaryl optionally substituted by one or two or more substituents selected from Rc, 4- to 12-membered nonaromatic heterocyclyl optionally substituted by one or two or more substituents selected from Rc, $C_{6-10}$ aryloxy optionally substituted by one or two or more halogen atoms, $C_{6-10}$ arylsulfanyl optionally substituted by one or two or more halogen atoms, and adamantyl; and
Rb is independently selected from the group consisting of a halogen atom, hydroxy, $C_{1-6}$ alkyl optionally substituted by one or two or more substituents selected from Ra, $C_{1-6}$ alkoxy optionally substituted by one or two or more substituents selected from Ra, cyano, ($C_{1-6}$ alkoxy)carbonyl, carboxy, $C_{3-10}$ cycloalkyl, $C_{6-10}$ aryl optionally substituted by one or two or more halogen atoms, 5- to 10-membered heteroaryl optionally substituted by one or two or more halogen atoms, and 3- to 12-membered nonaromatic heterocyclyl.

In the present specification, the reactive functional group derivatization step means the step of converting the reactive functional group to another group. Examples thereof include the step of introducing a protective group to the reactive functional group (protection step), a deprotection step for the protective group contained in the reactive functional group, the step of converting the reactive functional group to a group for linker formation, and the step of converting the reactive functional group to another functional group. Examples of the reactive functional group derivatization step include, but are not particularly limited to, the step of converting a primary or secondary amino group to a sulfonamide group, the step of converting a primary or secondary amino group to an amide group, the step of converting a primary or secondary amino group to a secondary or tertiary amino group, the step of converting a carboxyl group to an amide group, the step of converting a carboxyl group to an acylsulfonamide group, the step of converting a carboxyl group to an ester group, the step of converting a carboxyl group to a hydroxymethyl group, the step of converting a formyl group to an amino group, the step of converting a ketone group to an amino group, the step of converting a halogen atom to a hydroxy group, the step of converting a hydroxy group to an ether group, the step of converting a hydroxy group to an ester group, the step of converting a hydroxy group to a sulfonic acid ester group, the step of converting a hydroxy group to a halogen atom, the step of converting a hydroxymethyl group to a formyl group, the step of converting a hydroxymethyl group to a carboxyl group, the step of converting an olefin group to a boron atom-containing group through reaction with a boron reagent or the like, the step of converting a halogen atom to a boron atom-containing group through reaction with a boron reagent or the like, the step of converting a halogen atom to a carboxy group, the step of converting a carboxy group to a thioester group, the step of converting a thioester group to a boron atom-containing group, and the step of converting an amino group to a halogen atom.

The step of converting a halogen atom to a hydroxy group is not particularly limited. For example, the halogen atom can be converted to a hydroxy group through reaction with water at 20 to 80°C in a solvent such as NMP, DMF, THF, DMPr (N,N- dimethylpropionamide), or DEAc (N,N-diethylacetamide).

The step of converting a hydroxy group to a sulfonic acid ester group is not particularly limited. For example, the hydroxy group can be reacted with MsCl, TsCl, NsCl, DNsCl, chloromethanesulfonyl chloride, nonafluorobutanesulfonyl chloride, methanesulfonic anhydride, trifluoromethanesulfonic anhydride, or nonafluorobutanesulfonic anhydride, or the like at -78 to 80°C in a solvent such as THF or DCM. If necessary, the reaction may be performed in the presence of tertiary amine such as triethylamine or an aromatic base such as pyridine or 2,6-lutidine.

The step of converting an olefine group to a boron atom-containing group through reaction with a boron reagent or the like is not particularly limited. For example, the olefine group can be converted to a boron atom-containing group through reaction with 9-BBN, pinacol borane, catechol borane, bis(pinacolato)diboron, or the like at 20 to 80°C in a solvent such as THF. If necessary, the reaction may be performed in the presence of a metal catalyst such as a palladium catalyst, an iridium catalyst, a rhodium catalyst, a ruthenium catalyst, or a copper catalyst.

The step of converting a halogen atom to a boron atom-containing group through reaction with a boron reagent or the like is not particularly limited. For example, the halogen atom can be converted to a boron atom-containing group through reaction with bis(pinacolato)diboron or the like at 20 to 100°C in a solvent such as THF in the presence of a metal catalyst such as a palladium catalyst or a copper catalyst.

The step of converting a halogen atom to a carboxy group is not particularly limited. The halogen atom can be converted to a carboxy group through reaction with carbon monoxide or a reagent that forms carbon monoxide in the reaction system (oxalic acid, N- formyl saccharin, etc.) at 20 to 100°C in a solvent such as DMF in the presence of a metal catalyst such as a palladium catalyst.

The step of converting a carboxy group to a thioester group is not particularly limited. The carboxy group can be converted to a thioester group through reaction with thiol at 20 to 80°C in a solvent such as DCM or NMP in the presence of a condensing agent such as DIC, PyClU, or PipClU.

The step of converting a thioester group to a boron atom-containing group is not particularly limited. For example, the thioester group can be converted to a boron atom- containing group through reaction with bis(pinacolato)diboron or the like at 20 to 100°C in a solvent such as THF in the presence of a metal catalyst such as a rhodium catalyst.

[Formula 29]

Step of converting amino group to halogen atom

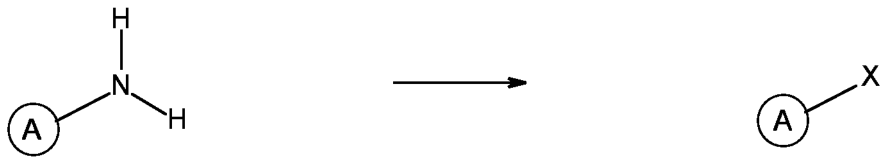

When A in the formula contains a solid-phase carrier, the product A-X is aryl halide, and the reactive functional group X linked to A is a halogen atom, for example, a fluorine atom, a chlorine atom, a bromine atom, or an iodine atom. The step of converting an amino group to a halogen atom can be carried out through reaction with the amino group of the reactive functional group linked to A in a starting material using a nitrous acid derivative, an acid, and a halogen source.

In this context, the nitrous acid derivative is not particularly limited as long as the nitrous acid derivative can be usually used. Examples of the nitrous acid derivative that can be used include sodium nitrite, potassium nitrite, methyl nitrite, ethyl nitrite, propyl nitrite, isopropyl nitrite, tert-butyl nitrite, isobutyl nitrite, butyl nitrite, amyl nitrite, isoamyl nitrite, and hexyl nitrite.

The acid is not particularly limited as long as the acid can be usually used. Examples of the acid that can be used include hydrochloric acid, hydrobromic acid, sulfuric acid, methanesulfonic acid, p-toluenesulfonic acid, camphorsulfonic acid, acetic acid, and tetrafluoroboric acid.

The halogen source is not particularly limited as long as the halogen source can be usually used. Examples of the halogen source that can be used include hydrochloric acid, hydrobromic acid, bromine, copper(I) chloride (CuCl), copper(I) bromide (CuBr), copper(I) iodide (CuI), copper(II) bromide ($CuBr_2$), potassium bromide (KBr), sodium bromide (NaBr), potassium iodide (KI), lithium chloride (LiCl), bromoform ($CHBr_3$), dibromomethane ($CH_2Br_2$), diiodomethane ($CH_2I_2$), bromotrichloromethane ($BrCCl_3$), bromotrimethylsilane (TMSBr), tetrabutylammonium bromide (TBAB), benzylammonium chloride, N- bromosuccinimide (NBS), N-chlorosuccinimide (NCS), and N-iodosuccinimide (NIS).

The number of equivalents of the nitrous acid derivative used can be appropriately set by those skilled in the art and can be, for example, 1 to 100 equivalents, 1 to 75 equivalents, 1 to 50 equivalents, 1 to 30 equivalents, or 1 to 20 equivalents, based on the amine containing A. The number of equivalents of the acid used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing A. The number of equivalents of the halogen source used can be appropriately set by those skilled in the art and can be, for example, 1 to 100 equivalents, 1 to 75 equivalents, 1 to 50 equivalents, 1 to 30 equivalents, or 1 to 20 equivalents, based on the amine containing A.

A solvent can be used in the reaction. Examples of the solvent that can be used include, but are not particularly limited to, halogen solvents such as DCM, dibromomethane (DBM), chloroform, bromoform, DCE, dibromoethane, and carbon tetrachloride, ether solvents such as diethyl ether, diisopropyl ether, THF (tetrahydrofuran), 2- methyltetrahydrofuran, 4-methyltetrahydropyran, 1,4-dioxane, DME, TBME, CPME, diisopropyl ether, isosorbide dimethyl ether, diglyme, triglyme, and tetraglyme, ketone solvents such as acetone, MEK, MIBK, 4-methyl-2-pentanone, cyclopentanone, cyclohexanone, and diethyl ketone, ester solvents such as methyl acetate, ethyl acetate, propyl acetate, isopropyl acetate, butyl acetate, isobutyl acetate, GVL ($\gamma$-valerolactone), pentyl acetate, and methyl propionate, urea solvents (TMU (N,N,N',N'-tetramethylurea), DMI (1,3-dimethyl-2-imidazolidinone), DMPU (N,N'-dimethylpropyleneurea), etc.), amide solvents (DMF (N,N-dimethylformamide), DMA (N,N-dimethylacetamide), NFM (N- formylmorpholine), NMP (N-methyl-2-pyrrolidone), NEP (N-ethyl-2-pyrrolidone), NBP (N- butyl-2-pyrrolidone), formamide, etc.), sulfoxide solvents (DMSO (dimethyl sulfoxide), methylphenyl sulfoxide, etc.), sulfone solvents (diphenylsulfone, dimethylsulfone, diethylsulfone, sulfolane, 3-methylsulfolane, ethylmethylsulfone, ethylisopropylsulfone, etc.), aromatic solvents such as anisole, toluene, xylene, chlorobenzene, bromobenzene, ethylbenzene, nitrobenzene, $\alpha,\alpha,\alpha$-trifluorotoluene, 1,2-dichlorobenzene, benzene, and pyridine, hydrocarbon solvents such as hexane, pentane, heptane, cyclohexane, methylcyclohexane, and decalin, alcohol solvents such as methanol, ethanol, isopropanol, tert-butanol, and tert-amyl alcohol, as well as solvents such as acetonitrile, water, and acetic acid. A single solvent may be used, or a combined solvent of a plurality of solvents may be used.

The reaction temperature can be appropriately set by those skilled in the art and can be, for example, -20 to 100°C, -100 to 80°C, -5 to 60°C, 0 to 45°C, or 0 to 30°C.

If necessary, an additive may be used. Examples of the additive that can be used include copper(II) oxide (CuO), copper(II) sulfate ($CuSO_4$), and iron(II) sulfate ($FeSO_4$). The number of equivalents of the additive used can be appropriately set by those skilled in the art and can be, for example, 1 to 100 equivalents, 1 to 75 equivalents, 1 to 50 equivalents, 1 to 30 equivalents, or 1 to 10 equivalents, based on the amine containing A.

The reaction time can be appropriately set by those skilled in the art and can be, for example, a time required to attain a sufficient conversion rate in a study experiment (also called model experiment) using the substrate concerned or a similar substrate, or a time predicted from the study results by some approach, or the point in time of the completion of the reaction can be determined or confirmed by cleaving products from some solid-phase carriers. After the completion of the reaction, the compounds of interest can be obtained by a usual aftertreatment method.

The step of introducing a protective group to the reactive functional group and the step of deprotecting the protective group are not particularly limited, and an arbitrary protective group, introduction conditions, and deprotection conditions known in the art can be used. These factors can be appropriately set for use with reference to, for example, "Greene's Protective Groups in Organic Synthesis, Fifth edition".

In the step of introducing a protective group to the reactive functional group, examples of the group for introducing the protective group to a primary or secondary amino group include, but are not particularly limited to, a Boc group, a Fmoc group, a Cbz group, a Teoc group, a Troc group, an Alloc group, a Trt group, a Ns group, a DNs group, a Ts group, a PMB group, a DMB group, a trifluoroacetyl group, a THP group, and a SEM group. Examples of the form in which a protective group for a carboxyl group is introduced include, but are not particularly limited to, methyl ester, ethyl ester, propyl ester, isopropyl ester, isobutyl ester, t-butyl ester, allyl ester, trityl ester, 2-chlorotrityl ester, benzyl ester, and OBO ester.

Examples of the protective group to be introduced to a hydroxy group include, but are not particularly limited to, an acetyl group, a pivaloyl group, a t-butyl group, a trityl group, a 2-chlorotrityl group, a THP group, a MOM group, a TMS group, a TES group, a triisopropylsilyl group, a TBDMS group, a TBDPS group, a SEM group, a tert- butyldiphenylsilylethyl group, an allyl group, a benzyl group, a phenyl carbamate group, and a methylacetal group.

Examples of the protective group to be introduced to a formyl group include, but are not particularly limited to, a dimethylacetal group, a diisopropylacetal group, and an ethylideneacetal group.

Examples of the protective group to be introduced to a sulfonamide group include, but are not particularly limited to, a PMB group, a DMB group, a Trt group, a SEM group, an allyl group, and a benzyl group.

Examples of the protective group to be introduced to a nitrogen atom of a pyrazole ring, an imidazole ring, an indole ring, an indazole ring, or the like include, but are not particularly limited to, a PMB group, a DMB group, a Trt group, a SEM group, an allyl group, a benzyl group, and a THP group.

In the step of deprotecting the protective group introduced to the reactive functional group, examples of the group resulting from deprotection include an amino group, a carboxy group, a hydroxy group, a formyl group, a ketone group, and a thiol group. Examples of the protective group to be removed from an amino group include a Boc group, a Fmoc group, a Cbz group, a Teoc group, a Troc group, an Alloc group, a Trt group, a Ns group, a DNs group, a Ts group, a PMB group, a DMB group, a trifluoroacetyl group, a THP group, and a SEM group.

Examples of the form at a stage prior to the exposure of a carboxyl group by deprotection include methyl ester, ethyl ester, propyl ester, isopropyl ester, isobutyl ester, t- butyl ester, allyl ester, trityl ester, 2-chlorotrityl ester, benzyl ester, and OBO ester.

Examples of the protective group to be removed from a hydroxy group include an acetyl group, a pivaloyl group, a t-butyl group, a trityl group, a 2-chlorotrityl group, a THP group, a MOM group, a TMS group, a TES group, a triisopropylsilyl group, a TBDMS group, a TBDPS group, a SEM group, a tert-butyldiphenylsilylethyl group, an allyl group, and a benzyl group.

Examples of the protective group to be removed from a formyl group include, but are not particularly limited to, a dimethylacetal group, a diisopropylacetal group, and an ethylideneacetal group.

Examples of the protective group to be removed from a sulfonamide group include, but are not particularly limited to, a PMB group, a DMB group, a Trt group, a SEM group, an allyl group, and a benzyl group.

Examples of the protective group to be removed from a nitrogen atom of a pyrazole ring, an imidazole ring, an indole ring, an indazole ring, or the like include, but are not particularly limited to, a PMB group, a DMB group, a Trt group, a SEM group, an allyl group, a benzyl group, and a THP group.

One embodiment of the present invention provides a production method having a high yield in solid-phase synthesis through carbon-carbon bond formation reaction by Suzuki coupling. Specifically, in the case of using meCgPPh Pd G4 or (dtbpf)$PdCl_2$ as a palladium catalyst, cleavage from the solid phase is reduced, and the Suzuki coupling reaction can be performed at a high yield.

When an arbitrary linker that covalently links a resin for solid-phase synthesis and the first BB has a benzyl ether structure, it has been confirmed that the benzyl ether structure is cleaved in carbon-carbon bond formation reaction

mediated by a palladium catalyst so that the cleavage of the compound progresses. A low reactive substrate among substrates contained on the solid-phase side and/or a low reactive reagent may require a long reaction time for the completion of the reaction, and the progression of cleavage from the solid phase may largely influence reduction in yield in solid-phase reaction. In the case of using meCgPPh Pd G4 or (dtbpf)$PdCl_2$, it has been found that carbon-carbon single bond formation reaction progresses without causing the progression of cleavage from the solid phase. Use of this condition in the synthesis of a compound library suppresses cleavage from the solid phase even if reaction is delayed due to some factor, and can achieve a high yield. This method enables the robustness and yield of reaction to be improved for the purpose of supplying a "high-quality" mixture library.

The following abbreviations were used in the present specification or Examples.

[Table 1]

| | |
|---|---|
| AA | Ammonium acetate |
| AC | Ammonium bicarbonate |
| AcOH | Acetic acid |
| AdBippyPhos | 5-(di-tert-Butylphosphino)-1',3',5'-triphenyl-1'H-[1,4']bipirazole |
| AdBrettPhos Pd G6 Br | Sigma-Aldrich catalog No: 915378 |
| $Ag_2O$ | Silver(I) oxide |
| Alloc | Allyloxycarbonyl |
| tAmOH | tert-Amyl alcohol |
| BB | Building block |
| 9-BBN | 9-Borabicyclo[3.3.1]nonane |
| BEMP | 2-tert-Butylimino-2-diethylamino-1,3-dimethylperhydro-1,3,2-diazaphosphorin |
| Boc | tert-butoxycarbonyl |
| $Boc_2O$ | di-tert-Butyl dicarbonate |
| $B_2Pin_2$ | Bis(pinacolato)diboron |
| tBu | tert-Butyl |
| tBuBrettphos Pd G4 | CAS: 1980785-05-4, LEAP Chem Batch NO: 20200401 |
| tBuOH | tert-Butyl alcohol |
| (tBu)PhCPhos Pd G4 | Sigma-Aldrich catalog No: 900533 |
| tBuOK and KOtBu | Potassium tert-butoxide |
| $Bu_3P$ | Tributylphosphine |
| tBu-TMG and BTMG | 2-tert-Butyl-1,1,3,3-tetramethylguanidine |
| BTPP | tert-Butylimino-tri(pyrrolidino)phosphorane |
| cataCXium Pd G4 | CAS: 2230788-67-5, Aldrich product No: 900349 |
| Cbz | Benzyloxycarbonyl |
| CMMP | Cyanomethylene trimethylphosphorane |
| COMU | (1-Cyano-2-ethoxy-2-oxoethylidenaminooxy)dimethylaminomorpholinocarbenium hexafluorophosphate |
| CPME | Cyclopentyl methyl ether |
| 12-Crown-4 | 1,4,7,10-Tetraoxacyclododecane |
| 15-Crown-5 | 1,4,7,10,13-Pentaoxacyclopentadecane |
| 18-Crown-6 | 1,4,7,10,13,16-Hexaoxacyclooctadecane |
| CuI | Copper(I) iodide |
| CuBr | Copper(I) bromide |
| CuCl | Copper(I) chloride |
| $Cu(OAc)_2$ | Copper(II) acetate |
| $Cs_2CO_3$ | Cesium carbonate |
| DABSO | bis(sulfur dioxide)-1,4-diazabicyclo[2.2.2]octane adduct |
| DEAD | Diethyl azodicarboxylate |
| DEAc | N,N-Diethylacetamide |
| DBU | 1,8-Diazabicyclo[5.4.0]undec-7-ene |
| DBM | Dibromomethane |
| DBN | 1,5-Diazabicyclo[4.3.0]non-5-ene |
| DCC | N,N'-Dicyclohexylcarbodiimide |
| DCM | Dichloromethane |
| DCE | 1,2-Dichloroethane |
| DIA | N,N-Diisopropylamine |
| DIAD | Diisopropyl azodicarboxylate |
| DIC | N,N'-Diisopropylcarbodiimide |
| Diglyme | Diethylene glycol dimethyl ether |
| DIPEA and Hunig's base | N,N-Diisopropylethylamine |
| DMA | N,N-Dimethylacetamide |
| DMAP | N,N-Dimethyl-4-aminopyridine |
| DME | 1,2-Dimethoxyethane |
| DMEDA | N,N'-Dimethylethylenediamine |

| | |
|---|---|
| DMF | N,N-Dimethylformamide |
| DMPU | N,N'-Dimethylpropylene urea |
| DMSO | Dimethyl sulfoxide |
| DMI | 3-Dimethyl-2-imidazolidinone |
| DNs | 1,4-Dinitrobenzenesulfonyl |
| DTBP | 2,6-di-tert-Butylpyridine |
| DTBMP | 2,6-di-tert-Butyl-4-methylpyridine |
| dtbpf | 1,1'-Bis(di-tert-butylphosphino)ferrocene |
| Dtbpy | 4,4'-di-tert-Butyl-2,2'-dipyridyl |
| EDCI | 1-Ethyl-3-(3-dimethylaminopropyl)carbodiimide |
| EDCI·HCl | 1-Ethyl-3-(3-dimethylaminopropyl)carbodiimide hydrochloride |
| EtOAc | Ethyl acetate |
| EtOH | Ethanol |
| $Et_3PO_4$ | Triethyl phosphate |
| FA | Formic acid |
| GVL | γ-Valerolactone |
| GPhos | (3-(tert-Butoxy)-2',6'-diisopropyl-6-methoxy-[1,1'-biphenyl]-2-yl)dicyclohexylphosphane, Sigma-Aldrich, catalog No: 918008 |
| HATU | O-(7-Azabenzotriazol-1-yl)-N,N,N',N'-tetramethyluronium hexafluorophosphate |
| Hex | Hexane |
| HCl | Hydrochloric acid |
| HFIP | 1,1,1,3,3,3-Hexafluoroisopropyl alcohol |
| HMDS | Bis(trimethylsilyl)amine |
| HMPA | Hexamethylphosphoric triamide |
| $H_2O$ | Water |
| HOAt or HOAT | 1-Hydroxy-7-azabenzotriazole |
| HOBt | 1-Hydroxybenzotriazole |
| HOOBt | 3,4-Dihydro-3-hydroxy-4-oxo-1,2,3-benzotriazine |
| $[Ir(cod)OMe]_2$ | (1,5-cyclooctadiene)(methoxy)iridium(I) (dimer) bis(1,5-cyclooctadiene)di-μ-methoxydiiridium(I), CAS: 12148-71-9 |
| Josiphos Pd G3 | {(R)-1-[(Sp)-2-(Dicyclohexylphosphino)ferrocenyl]ethyl di-tert-butylphosphine[2-(2'-amino-1,1'-biphenyl)]palladium(II) methanesulfonate, CAS: 1702311-34-9, Sigma-Aldrich, catalog No: 747130 |
| $K_2CO_3$ | Potassium carbonate |
| KHMDS | Lithium bis(trimethylsilyl)amide |
| KOH | Potassium hydroxide |
| KOPh | Potassium phenoxide |
| KOTf | Potassium trifluoromethanesulfonate |
| $K_2HPO4$ | Dipotassium hydrogen phosphate |
| $K_3PO_4$ | Tripotassium phosphate |
| $K_3PO_4$-$H_2O$ | Potassium phosphate,tribasic acid monohydrate |
| LDA | Lithium diisopropylamide |
| LiCl | Lithium chloride |
| LiHMDS | Lithium bis(trimethylsilyl)amide |
| LiOtBu | Lithium tert-butoxide |
| $Me_4Si$ | Tetramethylsilane |
| MeCN | Acetonitrile |
| MEK | Methyl ethyl ketone |
| meCgPPh Pd G4 | 1,3,5,7-Tetramethyl-6-phenyl-2,4,6-trioxa-6-phosphaadamantane Pd G4, Sigma-Aldrich, catalog code: 900697 |
| MIBK | Methyl isobutyl ketone |
| MeI | Iodomethane |
| MOM | Methoxymethyl |
| MeOH | Methanol |
| 4-MeTHP | 4-Methyltetrahydropyran |

| | |
|---|---|
| MTBD | 7-Methyl-1,5,7-triazabicyclo[4.4.0]dec-5-ene |
| $Me_4NBH(OAc)_3$ | Tetramethylammonium triacetoxyborohydride |
| MsCl | Methanesulfonyl chloride |
| $NaBH(OAc)_3$ | Sodium triacetoxyborohydride |
| NaBHT | Sodium butylated hydroxytoluene |
| $Na_2CO_3$ | Sodium carbonate |
| $NaHCO_3$ | Sodium bicarbonate |
| NaHMDS | Sodium bis(trimethylsilyl)amide |
| NaOtBu | Sodium tert-butoxide |
| NaOH | Sodium hydroxide |
| NaOPh | Sodium phenoxide |
| NBP | N-Butyl-2-pyrrolidone |
| NCS | N-Chlorosuccinimide |
| NEP | N-ethyl-2-pyrrolidone |
| $NiCl_2$ | Nickel(II) chloride |
| $Ni(COD)_2$ | Bis(1,5-cyclooctadiene)nickel(0) |
| NMF | N-Formylmorpholine |
| NMM | N-Methylmorpholine |
| NMP | N-Methyl-2-pyrrolidone |
| NMI | 1-Methylimidazole |
| NAC | N-Acetyl-L-cysteine |
| Ns | 2-Nitrobenzenesulfonyl |
| OBO | Oxabicyclo[2.2.2]octyl |
| oxyma | Ethyl cyano(hydroxyimino)acetate |
| P1-tBu, Phosphazene P1tBu and P1tBu | tert-Butylimino-tris(dimethylamino)phosphorane |
| P2-Et and P2Et | 1-Ethyl-2,2,4,4,4-pentakis(dimethylamino)-2$\lambda^5$,4$\lambda^5$-catenadi(phosphazene),tetramethyl(tris(dimethylamino)phosphoranylidene)phosphoric triamide-Et-imine |
| P2-tBu and P2tBu | 1-tert-Butyl-2,2,4,4,4-pentakis(dimethylamino)-2$\lambda^5$,4$\lambda^5$-catenadi(phosphazene) |
| Pd-PEPPSI-IPent and PEPPSI-IPent | [1,3-Bis(2,6-di-3-pentylphenyl)imidazol-2-ylidene](3-chloropyridyl)dichloropalladium(II), CAS: 1158652-41-5 |
| Pd-PEPPSI-IPr and PEPPSI-IPr | [1,3-Bis(2,6-diisopropylphenyl)imidazol-2-ylidene](3-chloropyridyl)palladium(II) dichloride, CAS: 905459-27-0 |
| $PdCl_2$ | Palladium(II) chloride |
| $P(Cy)_3$ Pd G3 | [(Tricyclohexylphosphine)-2-(2'-aminobiphenyl)]palladium(II) methanesulfonate, CAS: 1445086-12-3 |
| $PdCl_2(PPh_3)_2$ | Bis(triphenylphosphine)palladium(II) dichloride, CAS: 13965-0-2 |
| $Pd(PPh_3)_2Cl_2$ | Bis(triphenylphosphine)palladium(II) dichloride, CAS: 13965-03-2 |
| $Pd(PPh_3)_4$ | Tetrakistriphenylphosphine palladium, CAS: 14221-01-3 |
| $Pd(dppf)Cl_2 \cdot DCM$ | [1,1'-Bis(diphenylphosphino)ferrocene]dichloropalladium(II) (1:1), CAS: 95464-05-4 |
| $Pd(dppf)Cl_2$ | [1,1'-Bis(diphenylphosphino)ferrocene]dichloropalladium(II),CAS: 72287-26-4 |
| $Pd_2(dba)_3$-$CHCl_3$ | Tris(dibenzylideneacetone)dipalladium(0) (chloroform adduct), CAS: 52522-40-4 |
| $Pd(OAc)_2$ | Palladium acetate, CAS: 3375-31-3 |
| Pd G1 | First-generation Buchwald precatalyst (see https://www.sigmaaldrich.com/content/dam/sigma-aldrich/docs/SAJ/Brochure/1/saj1975.pdf and https://www.strem.com/uploads/resources/documents/buchwaldligprecat.pdf) |
| Pd G2 | Second-generation Buchwald precatalyst (see the literatures of Pd G1 described above) |
| Pd G3 | Third-generation Buchwald precatalyst (see the literatures of Pd G1 described above) |
| Pd G4 | Fourth-generation Buchwald precatalyst (see the literatures of Pd G1 described above) |

| | |
|---|---|
| $(PinB)_2$ | Bis(pinacolato)diboron |
| Proton Sponge | 1,8-Bis(dimethylamino)naphthalene |
| PipClU | Chlorodipiperidinocarbenium hexafluorophosphate |
| PMB | p-Methoxybenzyl |
| PPTS | Pyridinium p-toluenesulfonate |
| nPr | Normal propyl |
| $n-PrNH_2$ | Normal propylamine |
| RuPhos | 2-Dicyclohexylphosphino-2',6'-diisopropoxybiphenyl |
| RuPhos Pd G4 | (Methanesulfonato-2"-methylamino-1,1"-biphenyl-2-yl-palladacycle Gen. 4), (see HP of Strem Chemicals, Inc.) |
| Rochelle salt | Sodium potassium (+)-tartrate tetrahydrate |
| SEM | 2-(Trimethylsilyl)ethoxymethyl |
| TBAF | Tetra-n-butylammonium fluoride |
| $TBAHSO_4$ | Tetrabutylammonium bisulfate |
| TBDMS | tert-Butyldimethylsilyl |
| TBDPS | tert-Butyldiphenylsilyl |
| TBME | t-Butyl methyl ether |
| TBP | Tributylphosphine |
| TEA | Triethylamine |
| Teoc | 2-(Trimethylsilyl)ethoxycarbonyl |
| TES | Triethylsilyl |
| TFE | 2,2,2-Trifluoroethanol |
| Tetraglyme | Bis[2-(2-methoxyethoxy) ethyl ether |
| THF | Tetrahydrofuran |
| THP | Tetrahydropyranyl |
| $Ti(OiPr)_4$ | Tetraisopropyl o-titanate |
| $Ti(OtBu)_4$ | Tetra-tert-butyl o-titanate |
| TFA | Trifluoroacetic acid |
| TIPS | Triisopropylsilyl |
| TMAD | N,N,N',N'-Tetramethylazodicarboxamide |
| TMEDA | N,N,N',N'-Tetramethylethylenediamine |
| TMG | N,N,N',N'-Tetramethylguanidine |
| TMS | Trimethylsilyl |
| TMSOTf | Trimethylsilyl trifluoromethanesulfonate |
| TMU | N,N,N',N'-tetramethyl urea |
| TPP | Triphenylphosphine |
| Troc | 2,2,2-Trichloroethoxycarbonyl |
| Triglyme | 1,2-Bis(2-methoxyethoxy)ethane |
| trimethylbenzene | 1,2,3-Trimethylbenzene |
| Trt | Trityl |
| Ts | p-Toluenesulfonyl |
| WSCI·HCl and WSC·HCl | 1-Ethyl-3-(3-dimethylaminopropyl)carbodiimide hydrochloride |
| Xantphos Pd G4 | CAS: 1621274-19-8, Sigma-Aldrich, catalog No: 900329 |
| XPhos | 2-Dicyclohexylphosphino-2',4',6'-triisopropyl-1,1'-biphenyl |
| Xphos Pd G4 | CAS: 1599466-81-5, Sigma-Aldrich, catalog No: 804274 |

"Tanimoto similarity" used in the present specification or Examples was calculated by ECFP_6 using Pipeline Pilot 2017HF2 from Dassault Systemes S.E.

"cpKa" used in the present specification or Examples refers to a calculated value of pKa, and employed a value calculated using ADMET predictor version 9.5.

"ASA" used in the present specification or Examples refers to an accessible surface area, and was used as an index that indicates the accessible area of a size surrounding a specific atom by another molecule (Science. 1983 Aug 19; 221 (4612): 709-13; and J. Org. Chem. 2011, 76, 2, 435-440). ASA was selected as the largest ASA among ASA values of a plurality of conformations, by generating conformations (search from the most stable conformation in $CHCl_3$ to conformations having high energy up to 30 kJ/mol) with MacroModel of Maestro from Schrodinger, Inc., and calculating

ASA as to each of the plurality of conformations thus obtained with Advanced Surfaces of Maestro also from Schrodinger, Inc. (Type: Extended Surface).

EXAMPLES

General synthesis information

The contents of the present invention will be further described with reference to Examples and Reference Examples given below. However, the present invention is not limited by the contents.

All starting materials, reagents and solvents were obtained from commercial suppliers or synthesized by use of methods known in the art. The reagents and the solvents had reagent quality or greater quality and were used as they were obtained from various commercial suppliers, unless otherwise specified.

A 0.04 M solution of $P(Cy)_3$ Pd G3 in 1,4-dioxane used in Examples was prepared as follows: under a nitrogen atmosphere, $P(Cy)_3$ Pd G3 (26 mg, 0.04 mmol) and 1,4-dioxane (1 mL) were added to a 2 mL glass vial and dissolved by shaking to prepare a solution.

A 1.5 M aqueous $K_3PO_4$ solution used in Examples was prepared as follows: under a nitrogen atmosphere, $K_3PO_4$ (3.18 g, 15 mmol) was diluted with distilled water using a 10 mL measuring flask to prepare a solution.

A 0.02 M solution of $P(Cy)_3$ Pd G3 in NMP used in Examples was prepared as follows: under a nitrogen atmosphere, NMP (2.98 mL) was added to $P(Cy)_3$ Pd G3 (38.7 mg, 60 $\mu$mol) and 9-methylcarbazole (5.7 mg, 31 $\mu$mol) in a 4 mL glass vial, and the mixture was dissolved by shaking to prepare a solution.

Reaction monitoring and purity measurement were performed by carrying out the measurement of retention times and mass spectrometry under the following analysis conditions using SQD (manufactured by Waters Corp.) or 2020 (manufactured by Shimadzu Corp.) and a mass spectrometer, unless otherwise specified.

[Table 2]

| Analysis method | Apparatus | Column | Mobile phase | | Gradient | Flow rate mL/min |
|---|---|---|---|---|---|---|
| | | | A | B | | |
| SMD-FA05-1 | nexera/2020 | Ascentis Express C18 2.1mmI.D.x50mm,2.7um | 0.1%FA $H_2O$ | 0.1 %FA MeCN | A/B = 95/5→ 0/100(1.5min) → 0/100(0.5min) | 1 |
| | nexera/2020 | Ascentis Express C18 2.1mmI.D.x50mm,2.7um | 0.1%FA $H_2O$ | 0.1 %FA MeCN | A/B = 95/5→ 0/100(1.5min)→ 0/100(0.5min) | 1 |
| SMD-FA50-1 | nexera/2020 | Ascentis Express C18 2.1mmI.D.x50mm,2.7um | 0.1%FA $H_2O$ | 0.1 %FA MeCN | A/B = 50/50→ 0/100(1.1min)→ 0/100(1.1min) | 1 |
| SMD-FA50-2 | nexera/2020 | Speed Core C18 2.1mmI.D.x50mm,2.7um | 0.1%FA $H_2O$ | 0.1 %FA MeCN | A/B = 50/30→ 0/100(1.0min)→ 0/100(1.0min) | 1 |
| SMD- FA05-long | nexera/2020 | Ascentis Express C18 2.1mmI.D.x50mm,2.7um | 0.05%FA $H_2O$ | 0.05%FA MeCN | A/B = 95/5→ 0/100(4.5min)→ 0/100(0.5min) | 1 |
| SMD- FA05-RP | nexera/2020 | Ascentis Express RP- Amide 2.1mmI.D.x50mm,2.7um | 0.1%FA $H_2O$ | 0.1 %FA MeCN | A/B = 95/5→ 0/100(1.5min)→ 0/100(0.5min) | 1 |
| SMD- FA50-RP | nexera/2020 | Ascentis Express RP- Amide 2.1mmI.D.x50mm,2.7um | 0.1%FA$H_2O$ | 0.1 %FA MeCN | A/B = 50/50→ 0/100(1.0min)→ 0/100(1.0min) | 1 |
| SMD- FA25-long25mi n | nexera/2020 | Ascentis Express C18 2.1mmI.D.x150mm,2.7u m | 0.1%FA $H_2O$ | 0.1 %FA MeCN | A/B = 75/25→ 75/25(1min)→ 0/100(18min)→ 0/100(3min) | 0.5 |
| SMD- FA05-long25mi n | nexera/2020 | Ascentis Express C18 2.1mmI.D.x150mm,2.7u m | 0.1%FA $H_2O$ | 0.1 %FA MeCN | A/B = 95/5→ 95/5(1min)→ 0/100(15min)→ 0/100(4min) | 0.5 |
| SMD-TFA05-1 | nexera/2020 | Ascentis Express C18 2.1mmI.D.x50mm,2.7um | 0.05%TFA $H_2O$ | 0.05%TFA MeCN | A/B = 95/5→ 0/100(1.5min)→ 0/100(0.5min) | 1 |
| SMD-TFA50-1 | nexera/2020 | Ascentis Express C18 2.1mmI.D.x50mm,2.7um | 0.05%TFA $H_2O$ | 0.05%TFA MeCN | A/B = 50/50→ 0/100(1min)→ 0/100(1min) | 1 |
| SMD-TFA05- RP | nexera/2020 | Ascentis Express RP- Amide 2.1mmI.D.x50mm,2.7um | 0.05%TFA $H_2O$ | 0.05%TFA MeCN | A/B = 95/5→ 0/100(1.5min)→ 0/100(0.5min) | 1 |
| SMD-TFA50- RP | nexera/2020 | Ascentis Express RP- Amide 2.1mmI.D.x50mm,2.7um | 0.05%TFA $H_2O$ | 0.05%TFA MeCN | A/B = 50/50→ 0/100(1min)→ 0/100(1min) | 1 |
| SMD-TFA05- long | nexera/2020 | Ascentis Express C18 2.1mmI.D.x50mm,2.7um | 0.05%TFA $H_2O$ | 0.05%TFA MeCN | A/B = 95/5→ 0/100(4.5min)→ 0/100(0.5min) | 1 |

(continued)

| Analysis method | Apparatus | Column | Mobile phase | | Gradient | Flow rate mL/min |
|---|---|---|---|---|---|---|
| | | | A | B | | |
| SMD-TFA05- RP-long | nexera/2020 | Ascentis Express RP- Amide 2.1mmI.D.x50mm,2.7um | 0.05%TFA $H_2O$ | 0.05%TFA MeCN | A/B = 95/5→ 0/100(4.5min)→ 0/100(0.5min) | 1 |
| SMD-AC05-1 | nexera/2020 | Ascentis Express C18 2.1mmI.D.x50mm,2.7um | 10mM $NH_4HCO_3$ $H_2O$ | MeCN | A/B = 95/5→ 5/95(1.5min)→ 5/95(0.5min) | 1 |
| SMD-AC50-1 | nexera/2020 | Ascentis Express C18 2.1mmI.D.x50mm,2.7um | 10mM $NH_4HCO_3$ $H_2O$ | MeCN | A/B = 50/50→ 5/95(1.5min)→ 5/95(0.5min) | 1 |
| SMD- AC05-long25 min | nexera/2020 | Ascentis Express C18 2.1mmI.D.x150mm,2.7u m | 10mM $NH_4HCO_3$ $H_2O$ | MeCN | A/B = 95/5→ 5/95(15min)→ 5/95(5min) | 0.5 |
| SQD-FA05-1 | Acquity UPLC-I-Class/SQD | Ascentis Express C18 2.1mmI.D.x50mm,2.7um | 0.1%FA $H_2O$ | 0.1 %FA MeCN | A/B = 95/5→ 0/100(1.0min)→ 0/100(0.4min) | 1 |
| SQD-FA05-2 | Acquity UPLC/SQD2 | Ascentis Express C18 2.1mmI.D.x50mm,2.7um | 0.1%FA $H_2O$ | 0.1 %FA MeCN | A/B = 95/5→ 0/100(1.0min)→ 0/100(0.4min) | 1 |
| SQD-FA50-1 | Acquity UPLC-I-Class/SQD | Ascentis Express C18 2.1mmI.D.x50mm,2.7um | 0.1%FA $H_2O$ | 0.1 %FA MeCN | A/B = 50/50→ 0/100(0.7min)→ 0/100(0.7min) | 1 |
| SQD-FA50-4 | Acquity UPLC/SQD2 | Ascentis Express C18 2.1mmI.D.x50mm,2.7um | 0.1%FA $H_2O$ | 0.1 %FA MeCN | A/B =50/50→ 0/100(0.7min)→ 0/100(0.7min) | 1 |
| SQD-AA05-1 | Acquity UPLC-I-Class/SQD | Ascentis Express C18 2.1mmI.D.x50mm,5um | 10mM $AcONH_4$ $H_2O$ | MeOH | A/B = 95/5→ 0/100(1min)→ 100(0.4min) | 1 |
| SQD-AA05-2 | Acquity UPLC/SQD2 | Ascentis Express C18 2.1mmI.D.x50mm,5um | 10mM $AcONH_4$ $H_2O$ | MeOH | A/B = 95/5→ 0/100(1min)→ 100(0.4min) | 1 |
| SQD-AA50-1 | Acquity UPLC-I-Class/SQD | Ascentis Express C18 2.1mmI.D.x50mm,5um | 10mM $AcONH_4$ $H_2O$ | MeOH | A/B = 50/50→ 0/100(0.7min)→ 100(0.7min) | 1 |
| SQD-AA50-2 | Acquity UPLC/SQD2 | Ascentis Express C18 2.1mmI.D.x50mm,5um | 10mM $AcONH_4$ $H_2O$ | MeOH | A/B = 50/50→ 0/100(0.7min)→ 100(0.7min) | 1 |
| PH- FA05-1 | Shimadzu- 2020 | Ascentis Express C18 2.1mmI.D.x50mm,2.7um | 0.1%FA $H_2O$ | 0.1 %FA MeCN | A/B = 95/5→ 0/100(1.0 min)→ 0/100(0.5 min) | 1 |

(continued)

| Analysis method | Apparatus | Column | Mobile phase | | Gradient | Flow rate mL/min |
|---|---|---|---|---|---|---|
| | | | A | B | | |
| PH- FA05-2 | Shimadzu- 2020 | YMC-Triart C18 3.0mmI.D.x50mm,2.5um | 0.1%FA $H_2O$ | 0.1 %FA MeCN | A/B = 95/5(0.01 min)→ 5/95(1.09 min)→ 5/95(0.6 min) | 1.2 |
| PH- FA05-3 | Shimadzu- 2020 | HALO C18 2.1mmI.D.x50mm,2.0um | 0.1%FA $H_2O$ | 0.1 %FA MeCN | A/B = 95/5→ 0/100(0.7 min)→ 0/100(0.4 min) | 1 |
| PH- FA05-4 | Shimadzu- 2020 | HALO C18 2.1mmI.D.x30mm,2.0um | 0.1%FA $H_2O$ | 0.1 %FA MeCN | A/B = 95/5(0.01 min)→ 0/100(1.19 min)→ 0/100 (0.6 min) | 1 |
| PH- FA05-5 | Shimadzu- 2020 | HALO C18 3.0mmI.D.x30mm,2.0um | 0.1%FA $H_2O$ | 0.1 %FA MeCN | A/B = 95/5→ 0/100(1.0 min)→ 0/100(0.5 min) | 1.5 |
| PH-FA- 11 | Shimadzu- 2020 | HALO2.0umC1890A 2.1mmI.D.x30mm, 2.0um | 0.1%FA $H_2O$ | 0.1%FAMe CN | A/B=95/5→ 0/100(1.2min)→ 0/100(0.6min)→ 95/5(0.1min) | 1.0 |
| PH-FA- 12 | Shimadzu- 2020 | HALO2.0umC1890A 3.0mmI.D.x30mm,2.0um | 0.1%FA $H_2O$ | 0.1%FAMe CN | A/B=95/5→ 5/95(0.7min)→ →5/95(0.4min)→ 95/5(0.02min) | 1.2 |
| PH-FA- 13 | Shimadzu- 2020 | HALO2.0umC1890A 3.0mmI.D.x30mm, 2.0um | 0.1%FA $H_2O$ | 0.1%FAMe CN | A/B=70/30→ 30/70(1.7min)→ 0/100(0.6min)→ 0/100(0.5min)→ 95/5(0.03min) | 1.2 |
| PH-FA- 14 | Shimadzu- 2020 | HALO2.0umC1890A 3.0mmI.D.x30mm, 2.0um | 0.1%FA $H_2O$ | 0.1%FAMe CN | A/B=95/5→ 0/100(1.10min)→ 0/100(0.6min)→ 95/5(0.05min) | 1.3 |
| PH-FA- 15 | Shimadzu- 2020 | HALO2.0umC1890A 2.1mmI.D.x30mm, 2.0um | 0.1%FA $H_2O$ | 0.1%FAMe CN | A/B=95/5→ 0/100(0.7min)→ 0/100(0.4min)→ 95/5(0.02min) | 1.0 |
| PH-FA- 16 | Shimadzu- 2020 | HALO2.0umC1890A 3.0mmI.D.x30mm,2.0um | 0.1%FA $H_2O$ | 0.1%FAMe CN | A/B=95/5→ 5/95(1.1min)→ 5/95(0.6min)→ 95/5(0.05min) | 1.2 |
| PH-FA- 17 | Shimadzu- 2020 | HALO2.0umC1890A 2.1mmI.D.x30mm, 2.0um | 0.1%FA $H_2O$ | 0.1%FAMe CN | A/B=95/5→ 0/100(1.2min)-> 0/100(0.6min)→ 95/5(0.02min) | 1.0 |
| PH- TFA05-1 | Shimadzu- 2020 | Ascentis Express C18 3.0mmI.D.x50mm,2.7um | 0.05TFA% $H_2O$ | 0.05TFA% MeCN | A/B = 95/5(0.01 min)→ 5/95(1.09 min)→ 5/95(0.5 min) | 1 |
| PH- TFA05-2 | Shimadzu- 2020 | HALO C18 3.0mmI.D.x30mm,2.0um | 0.05TFA% $H_2O$ | 0.05TFA% MeCN | A/B = 95/5(0.01 min)→ 0/100(1.09 min)→ 0/100(0.6 min) | 1.3 |

(continued)

| Analysis method | Apparatus | Column | Mobile phase | | Gradient | Flow rate mL/min |
|---|---|---|---|---|---|---|
| | | | A | B | | |
| PH- TFA-21 | Shimadzu- 2020 | HALO2.0umC1890A 3.0mmI.D.x30mm, 2.0um | 0.05TFA% $H_2O$ | 0.05TFA% MeCN | A/B=95/5→ 40/60(1.7min)→ 0/100(0.6min)→ 0/100(0.5min)- 95/5(0.03min) | 1.2 |
| PH- TFA-22 | Shimadzu- 2020 | HALO2.0um C1890A 3.0mmI.D.x30mm, 2.0um | 0.05TFA% $H_2O$ | 0.05TFA% MeCN | A/B=95/5→ 0/100(0.7min)→ 0/100(0.4min)→ 95/5(0.02min) | 1.2 |
| PH- TFA-23 | Shimadzu- 2020 | HALO2.0um C1890A 3.0mmI.D.x30mm, 2.0um | 0.05TFA% $H_2O$ | 0.05TFA% MeCN | A/B=95/5→ 0/100(1.1min)→ 0/100(0.6min)→ 95/5(0.05min) | 1.5 |
| PH- TFA-24 | Shimadzu- 2020 | HALO2.0um C1890A 3.0mmI.D.x30mm, 2.0um | 0.05TFA% $H_2O$ | 0.05TFA% MeCN | A/B=95/5→ 0/100(1.10min)→ 0/100(0.6min)→ 95/5(0.05min) | 1.3 |
| PH-AC- 11 | Shimadzu- 2020 | Poroshell HPH-C18 3.0mmI.D.x50mm,L 2.7um | $H_2O$+6.5mMNH$_4$CO$_3$+ammonium hydroxide(pH10) | MeCN | A/B=90/10→ 5/95(2.0min)→ 5/95(0.7min)→ 90/10(0.1min) | 1.2 |
| PH-AC- 12 | Shimadzu- 2020 | Poroshell HPH-C18 3.0mmI.D.x50mm, 2.7um | $H_2O$ +6.5mMNH$_4$CO$_3$ +ammoniumhydr oxide(pH10) | MeCN | A/B=50/50→ 5/95(2.7min)→ 5/9(0.5min)→ 5/95to90/10(0.1min) | 1.2 |

The terms "m/z[M+H]+" and "(M+H)+" described in LCMS analysis results in Examples were all described as values detected in the positive mode, unless otherwise specified. UV area% in LCMS was indicated by values in PDA (210 to 400 nm), unless otherwise specified. When a specific wavelength (e.g., 319 nm, 290 nm, and 280 nm) was described herein, UV area% at a wavelength of +/- 4 nm centered on the described wavelength was described.

"Conversion rate" refers to the degree of progression of reaction and was determined according to the following expression, unless otherwise specified.

"Conversion rate" (%) = (UV area% of the target compound) / ((UV area% of starting materials) + (UV area% of the target compound) + (UV area% of by-products)) × 100

For example, SHOKO Scientific Purif-Pack(R) SI (60 μm), SHOKO Purif-Pack(R) - EX SI (50 μm), Biotage(R) SNAP Cartridge KP-Sil, Biotage(R), SNAP Ultra, Biotage(R) Sfar D (Duo) (60 μm) or Biotage(R) Sfar HC D (Duo) (20 μm) was appropriately used as silica gel in column chromatography. For example, SHOKO Purif-Pack(R) -EX NH (50 μm), Biotage(R) SNAP Isolute NH2 (50 μm) or Biotage(R) SNAP Cartridge KP-NH was appropriately used as amino silica gel in column chromatography. For example, SHOKO Purif-Pack(R) -EX ODS (50 μm), Biotage(R) SNAP Ultra C18 (25 μm) or Biotage(R) Sfar C18 (30 μm) was appropriately used as reverse-phase silica gel in column chromatography.

The HPLC purification of a compound was performed using AutoPurification HPLC/MS System (manufactured by Waters Corp.), Trilution (manufactured by Gilson, Inc.), or the like.

$^1$H-NMR or $^{13}$C-NMR spectra were measured with or without $Me_4Si$ as internal standards using, for example, ECP-400 (manufactured by JEOL Ltd.), Agilent 400-MR (manufactured by Agilent Technologies, Inc.) or AVANCE III HD (manufactured by Broker) (s = singlet, brs = broad singlet, d = doublet, t = triplet, q = quartet, dd = double doublet, ddd = double double doublet, dt = double triplet, td = triple doublet, m = multiplet).

The phrase "phase separator" refers to an operation of removing moisture contained in an organic phase using, for example, Biotage(R) ISOLUTE Phase Separators.

"Concentration" or "concentration under reduced pressure" refers to the evaporative removal of a solvent under reduced pressure using a rotary evaporator, a mechanical oil vacuum pump or a mechanical oil-free vacuum pump.

The phrase "solvent was distilled off under reduced pressure by Genevac" or "distilled off" refers to the evaporative removal of a solvent using a centrifugal evaporator system (manufactured by Genevac Ltd., EZ-2 Elite, HT-6 or HT-12).

The phrase "dried overnight under reduced pressure" refers to the evaporative removal of a solvent under reduced pressure using a rotary evaporator, a mechanical oil vacuum pump or a mechanical oil-free vacuum pump.

The phrase "overnight" refers to, but is not limited to, approximately 8 to 14 hours, unless otherwise specified.

Solid-phase reaction can be carried out in an arbitrary suitable container, for example, a glass vial hermetically sealable with a cap having a Teflon packing, a fritted filter or a column having a suitable stopper. The container size is appropriately selected as a size that has a sufficient space for a solvent and has sufficient room for effective stirring of a resin in consideration of the possibility that a given resin swells markedly when treated with an organic solvent.

"Shaking" in solid-phase reaction was carried out at 50 to 200 rpm or 600 to 1000 rpm using a shaker suitable for securing sufficient mixing, which is a factor generally recognized as being important for successful reaction on a resin (e.g., Tokyo Rikakikai Co., Ltd., EYELA, MMS-320, MMS-220H, or AS ONE Corp., MyBL-100CS, TAITEC, or M·BR-104).

In solid-phase reaction, the equivalents of reagents when the substrate is a mixture refer to how many times (equivalents) the molar number of the reagent used is based on the total molar number of functional groups for use in the reaction carried by each compound of the mixture on the solid-phase side. For example, the term "10 equivalents" means that the reagent is used at a molar number of 10 times (10 equivalents) the total molar number of functional groups for use in the reaction carried by each compound of the mixture on the solid-phase side.

In liquid-phase reaction, the equivalents of reagents when the substrate is a mixture refer to how many times (equivalents) the molar number of the reagent used is based on the total molar number of functional groups for use in the reaction carried by each compound of the mixture. For example, the term "10 equivalents" means that the reagent is used at a molar number of 10 times (10 equivalents) the total molar number of functional groups for use in the reaction carried by each compound of the mixture.

A possible molar number of a mixture is as described below. Any value may be adopted unless otherwise specified. In the case of mixing individual compounds with their molar numbers found in advance, the total sum thereof can be considered as the molar number of the mixture. In the case of synthesizing a mixture in solid-phase synthesis, the total sum obtained by multiplying the amount of a prepared solid-phase supported substrate supported (mmol/g) by the amount of solid-phase supported substrates used, and adding up the resulting values can be considered as the molar number of the mixture. In this respect, even if the reaction on the solid phase undergoes a plurality of steps, the respective rates of conversion in the steps can be considered as 100% (i.e., a theoretical amount). When compounds are cleaved from the solid phase, cleavage efficiency may be considered as 100% (i.e., compounds are cleaved in a theoretical amount), followed by liquid-phase reaction.

The rate of loading (mmol/g) will be shown for solid-phase supported compounds used in solid-phase synthesis.

This rate indicates the rate of loading calculated when a starting material was supported on the solid phase (initial rate of loading). Since the linking reaction of building blocks causes change in molecular weight, the rate of loading after reaction may be different from the initial rate of loading. However, the initial rate of loading may be used throughout the synthesis process.

The rate of loading may differ depending on a lot even if solid-phase supported compounds are the same. However, the same compound number may be used as a compound number.

The amount of a solvent used in solid-phase reaction was used in volume in an arbitrary fold based on the weight of the resin used in the reaction (v/w). For example, in the case of using 20 mg of a resin and 20 v/w of a solvent, the solvent was used in 20 mg × 20 v/w = 400 μL.

The amount of a solvent used in washing a resin after reaction was used in volume in an arbitrary fold based on the weight of the resin used in the reaction (v/w). For example, in the case of using 20 mg of a resin and 20 v/w of a solvent, the solvent was used in 20 mg × 20 v/w = 400 μL.

For monitoring the progression of reaction on the solid phase, it is necessary to collect a resin from a reaction container. In this respect, the resin was collected by the aspiration of approximately 5 to 20 μL aliquot so as to inevitably contain the resin, using a micropipette equipped with a pipette tip from which a suitable length at the tip was cut off. The collected resin was transferred onto a filter of a pipette tip with a filter (e.g., Thermo Fisher Scientific Inc., ART Pipette with Filter, ART20P, 2149P-05). Then, the compound supported on the resin was cleaved from the resin by the following typical procedures as to the resin on the filter. The resin was continuously washed three times with NMP, three times with MeOH, and three times with DCM. Then, for example, a 10% solution of TFA in DCM containing 0.05 M trimethylbenzene (50 μL) was added to the filter tip, and the resin was dipped therein for 3 to 10 minutes. The solution was filtered by the application of air or nitrogen pressure from above the filter tip, and the filtrate was dissolved in NMP or DMI (250 μL). The solution (50 μL) was diluted with acetonitrile (250 μL) to prepare an LC sample, which was then analyzed by UPLC-MS analysis to measure the degree of progression of reaction.

The phrase "filtered through a vial with a filter" in the preparation of LC samples refers to an operation of removing insoluble matter contained in a reaction solution or a diluted solution, and means that the solution is added into an LC vial with a filter (e.g., Thomson SINGLE StEP Filter Vials, TH-35541) and pushed into an inner vial to prepare an LC solution.

The phrase "cleavage" from the solid phase refers to the dissociation of a compound supported on a resin from the resin, and means that the resin is treated with, for example, a 10% solution of TFA in DCM containing 0.05 M trimethylbenzene to recover the supported compound into the solution.

Compound numbers used in Examples were indicated by the combination of arbitrary alphabets, numbers, and symbols. A compound in a state supported on the solid phase was indicated by, for example, "ZZ001-01R" with "R" attached to the end. By contrast, a compound cleaved from the solid phase was indicated by "ZZ001" with "-01R" removed.

[Formula 30]

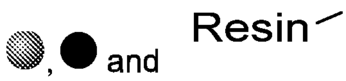
, and

used in chemical structure notation in Examples each represent polystyrene resin, and mean that a compound is supported on the solid phase, unless otherwise specified.

The number in "-01R", which represents a compound supported on the solid phase, used in Examples, indicated the type of the resin used. For example, "01" in "-01R" represents that a compound is in a state supported on brominated Wang resin (4- (bromomethyl)phenoxymethyl polystyrene) (e.g., Merck KGaA, 4-(benzyloxy)benzyl bromide, polymer-bound). "02" in "-02R" represents that a compound is in a state supported on 4-(bromomethyl)phenoxyethyl polystyrene (e.g., Merck KGaA, 2-(4- bromomethylphenoxy)ethyl polystyrene; also referred to as modified Wang resin in Examples). "03" in "-03R" represents that a compound is in a state supported via a linker moiety on polystyrene carboxylate (e.g., Chem-Impex International, Inc., polystyrene carboxylate; also referred to as carboxylic acid resin in Examples).

Exemplary notation of "-01R"

[Formula 31]

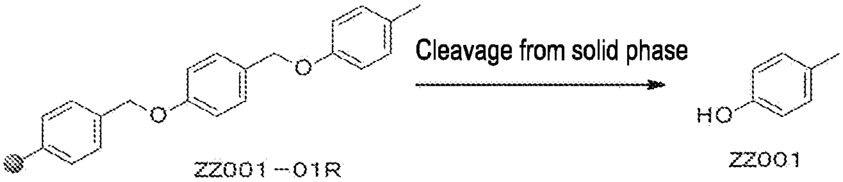


ZZ001-01R ZZ001

Exemplary notation of "-02R"

[Formula 32]

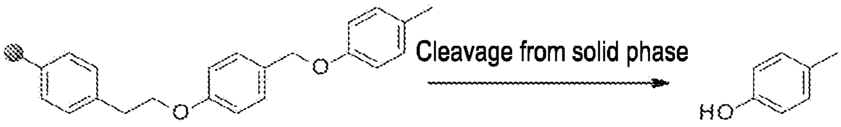


ZZ001-02R ZZ001

Exemplary notation of "-03R"

[Formula 33]

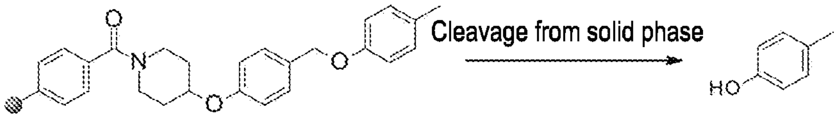


ZZ001-03R ZZ001

All the solid-phase supported compounds used in Examples 8, 9 and 10 employed a compound in a state supported via a linker moiety on polystyrene carboxylate (e.g., Chem- Impex International, Inc., polystyrene carboxylate; also referred to as carboxylic acid resin in Examples).

In Examples 8, 9 and 10, mixture numbers may be used to indicate synthesized mixtures. For example, "2-1" in "2-1-a1B01-0" represents the type of a library, and "a1B01" represents the type of a mixture. "-0" represents a state supported on the solid phase, and "-1" represents a state cleaved from the solid phase. Also, "-2" represents a state where functional group conversion was performed after cleavage from the solid phase. ID may be used to indicate each compound contained in a mixture. For example, "0001" in ID "2-1- a1B01-0-0001" is a number that defines one arbitrary compound contained in the mixture "2- 1-a1B01-0", and the mixture and the contained compounds are defined by ID.

Exemplary notation of "2-1-a1B01-0-0001"

[Formula 34]

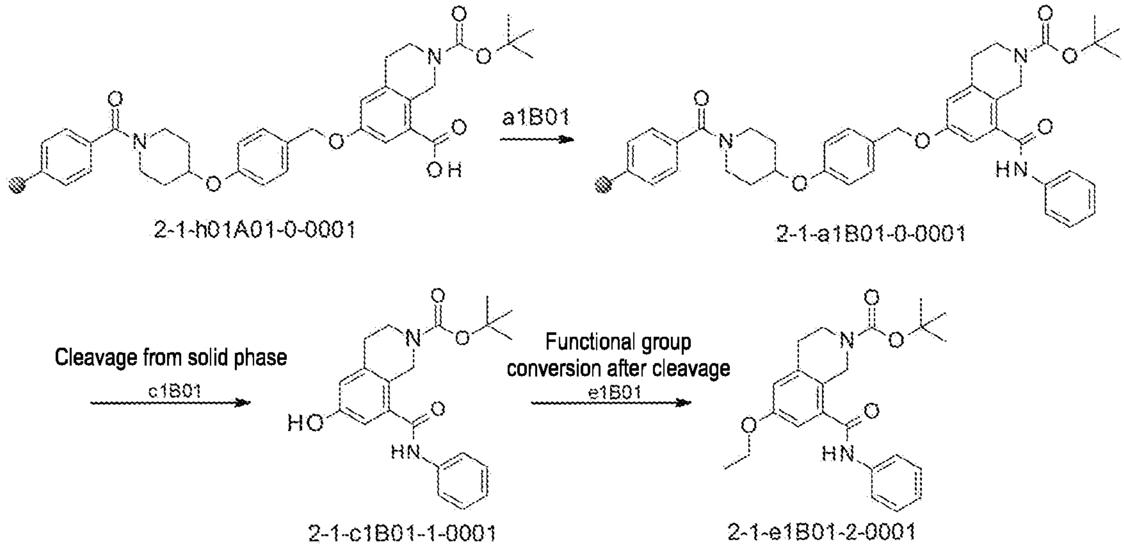


2-1-h01A01-0-0001 2-1-a1B01-0-0001 2-1-c1B01-1-0001 2-1-e1B01-2-0001

Example 1; Synthesis of substrate for reaction study and building block for use in mixture library, etc.

Example 1-1: Synthesis of substrate for reaction study for use mainly in Example 2- 1, and building block for use in mixture library, etc.

Example 1-1-1: Synthesis of 2-nitro-N-phenylbenzenesulfonamide (compound F101)

[Formula 35]

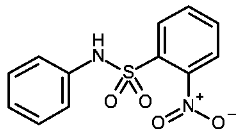

2-Nitrobenzenesulfonyl chloride (1.0 g, 4.51 mmol), dichloromethane (9.0 mL), aniline (0.453 mL, 4.96 mmol) and DIPEA (1.576 mL, 9.02 mmol) were added, and the reaction solution was stirred at 0°C for 2 hours.

Subsequently, 1 M hydrochloric acid was added thereto, followed by extraction with ethyl acetate. The obtained organic layer was washed with a saturated aqueous solution of sodium chloride and then concentrated. The obtained concentrate residue was purified by reverse-phase column chromatography (C18, mobile phase: 0.1% aqueous formic acid solution/0.1% solution of formic acid in acetonitrile), and the obtained fraction was freeze dried to obtain 2-nitro-N-phenylbenzenesulfonamide (compound F101, 0.70 g, 2.52 mmol, 55.7%).

LCMS (ESI) m/z = 279.1$[M+H]^+$.

Retention time: 0.75 min (analysis conditions SQD-FA05-1).

Example 1-1-2: Synthesis ofN-benzyl-2-nitrobenzenesulfonamide (compound F102)

[Formula 36]

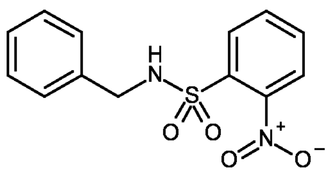

2-Nitrobenzenesulfonyl chloride (1.0 g, 4.51 mmol), dichloromethane (9.0 mL), benzylamine (0.542 mL, 4.96

mmol) and DIPEA (1.576 mL, 9.02 mmol) were added, and the reaction solution was stirred at 0°C for 2 hours.

Subsequently, 1 M hydrochloric acid was added thereto, followed by extraction with ethyl acetate. The obtained organic layer was washed with a saturated aqueous solution of sodium chloride and then concentrated. The obtained concentrate residue was purified by reverse-phase column chromatography (C18, mobile phase: 0.1% aqueous formic acid solution/0.1% solution of formic acid in acetonitrile), and the obtained fraction was freeze dried to obtain N-benzyl-2-nitrobenzenesulfonamide (compound F102, 1.0 g, 3.42 mmol, 76%).

LCMS (ESI) m/z = 293.2[M+H]+.

Retention time: 0.78 min (analysis conditions SQD-FA05-1).

Example 1-1-3: Synthesis of tert-butyl 4-[4-[[4-(4- ethoxycarbonylphenyl)phenoxy]methyl]phenoxy]piperidine-1-carboxylate (compound D066)

[Formula 37]

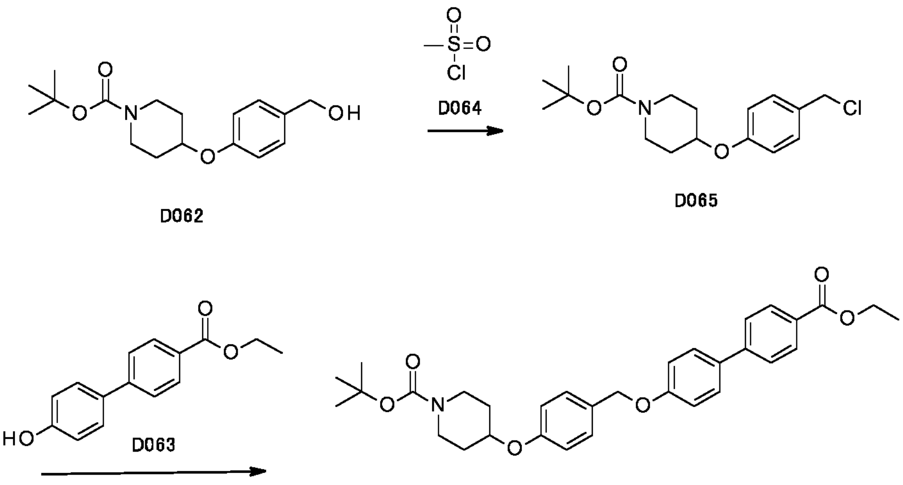


D062 D065 D066

Under a nitrogen atmosphere, tert-butyl 4-[4-(hydroxymethyl)phenoxy]piperidine-1- carboxylate (D062, 1.00 g, 3.25 mmol), triethylamine (0.499 mL, 3.58 mmol), and DCM (16.3 mL) were added to a 100 mL three-neck flask, and the reaction container was cooled to 0°C. Methanesulfonyl chloride (D064, 0.266 mL, 3.42 mmol) was added thereto, and the mixture was stirred at 0°C for 3 hours. A saturated aqueous solution of sodium bicarbonate (4.9 mL) was added to the obtained mixture. An organic layer was extracted with dichloromethane (24 mL) three times, dried over sodium sulfate, and then concentrated under reduced pressure. Under a nitrogen atmosphere, the obtained residue, ethyl 4-(4-hydroxyphenyl)benzoate (D063, 0.866 g, 3.58 mmol), cesium carbonate (2.12 g, 6.50 mmol), and NMP (12.0 mL) were mixed in a 100 mL three-neck flask and stirred at room temperature for 24 hours. A saturated aqueous solution of ammonium chloride (6 mL) was added to the obtained mixture. An organic layer was extracted with ethyl acetate (10 mL) three times. Then, the obtained organic layers were mixed, and hexane (20 mL) was added thereto. The organic layer was washed three times with water (15 mL) and once with a saturated aqueous solution of sodium chloride (15 mL) and concentrated under reduced pressure. The obtained residue was purified by silica gel column chromatography (ethyl acetate/hexane, 0-25%). Then, the obtained crude product was purified by silica gel column chromatography (NH-silica gel, dichloromethane/hexane, 0-100%). The obtained crude product was dissolved in ethyl acetate (100 mL) and hexane (200 mL), and the solution was washed three times with water (200 mL) and once with a saturated aqueous solution of sodium chloride (100 mL) and concentrated under reduced pressure to obtain a white solid of the title compound D066 (1.44 g, 2.71 mmol, 83%).

$^1$H-NMR (400 MHz, $CDCl_3$) δ: 8.08 (d, J = 8.4 Hz, 2H), 7.61 (d, J = 8.4 Hz, 2H), 7.57 (d, J = 8.8 Hz, 2H), 7.37 (d, J = 8.4 Hz, 2H), 7.06 (d, J = 8.8 Hz, 2H), 6.94 (d, J = 8.4 Hz, 2H), 5.04 (s, 1H), 4.50-4.45 (m, 1H), 4.40 (q, J = 7.2 Hz, 2H), 3.73-3.67 (m, 2H), 3.38-3.31 (m, 2H), 1.96-1.88 (m, 2H), 1.80-1.71 (m, 2H), 1.47 (s, 9H), 1.41 (t, J = 7.2 Hz, 3H).

LCMS: m/z 554 [M+Na]+.

Retention time: 1.684 min (analysis conditions SMD-FA05-1, 290 nm).

Example 1-1-4: Synthesis of ethyl 4-[4-[(4-piperidin-4- yloxyphenyl)methoxy]phenyl]benzoate (compound D067)

[Formula 38]

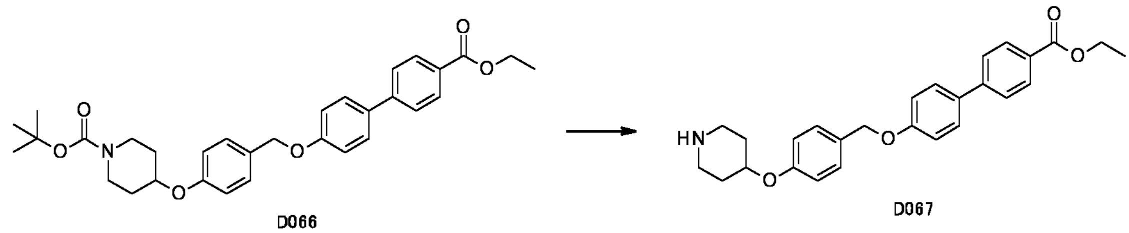

D066 D067

Under a nitrogen atmosphere, tert-butyl 4-[4-[[4-(4- ethoxycarbonylphenyl)phenoxy]methyl]phenoxy]piperidine-1-carboxylate (D066, 50.0 mg, 94.0 μmol), DIPEA (29.5 μL, 0.169 mmol), and THF (1.88 mL) were added to a 5 mL screw-cap vial, and the reaction container was cooled to 0°C. TMSOTf (20.4 μL, 0.113 mmol) was added thereto, and the mixture was stirred at 0°C for 3 hours. Then, DIPEA (2.95 μL, 16.9 μmol) and TMSOTf (2.0 μL, 11 μmol) were added thereto, and the mixture was stirred at 0°C for 1.5 hours. Triethylamine (26.2 μL) was added to the obtained mixture at 0°C, and the mixture was stirred at room temperature for 30 minutes. Water (847 μL), DMSO (1 mL), and formic acid (12.1 μL, 0.282 mmol) were added thereto, and the mixture was purified by reverse-phase column chromatography (C18, 0.1% solution of formic acid in acetonitrile/0.1% aqueous formic acid solution, 0-60%). The obtained product was dissolved in dichloromethane (100 mL), and the solution was washed three times with a saturated aqueous solution of sodium bicarbonate (50 mL) and once with a saturated aqueous solution of sodium chloride (50 mL) and concentrated under reduced pressure to obtain the title compound D067 (19.8 mg, 45.9 mmol, 49%) as a white solid.

$^1$H-NMR (400 MHz, $CDCl_3$) δ: 8.08 (d, J = 8.4 Hz, 2H), 7.61 (d, J = 8.8 Hz, 2H), 7.57 (d, J = 8.8 Hz, 2H), 7.36 (d, J = 8.8 Hz, 2H), 7.06 (d, J = 8.8 Hz, 2H), 6.94 (d, J = 8.8 Hz, 2H), 5.03 (s, 1H), 4.42-4.36 (m, 3H), 3.15 (ddd, J = 13.6, 4.8, 4.8 Hz, 2H), 2.74 (ddd, J = 13.6, 9.2, 3.2 Hz, 2H), 2.06-1.99 (m, 2H), 1.73-1.64 (m, 2H), 1.41 (t, J = 7.2 Hz, 3H).

LCMS: m/z 432 $[M+H]^+$.

Retention time: 0.904 min (analysis conditions SMD-FA05-1, 290 nm).

D067 whose synthesis method is illustrated in Example 1-1-4 is also referred to as compound C102 in the subsequent examples.

Example 1-1-5: Synthesis of methyl 3-[4-[(4-piperidin-4- yloxyphenyl)methoxy]phenyl]benzoate (D068)

[Formula 39]

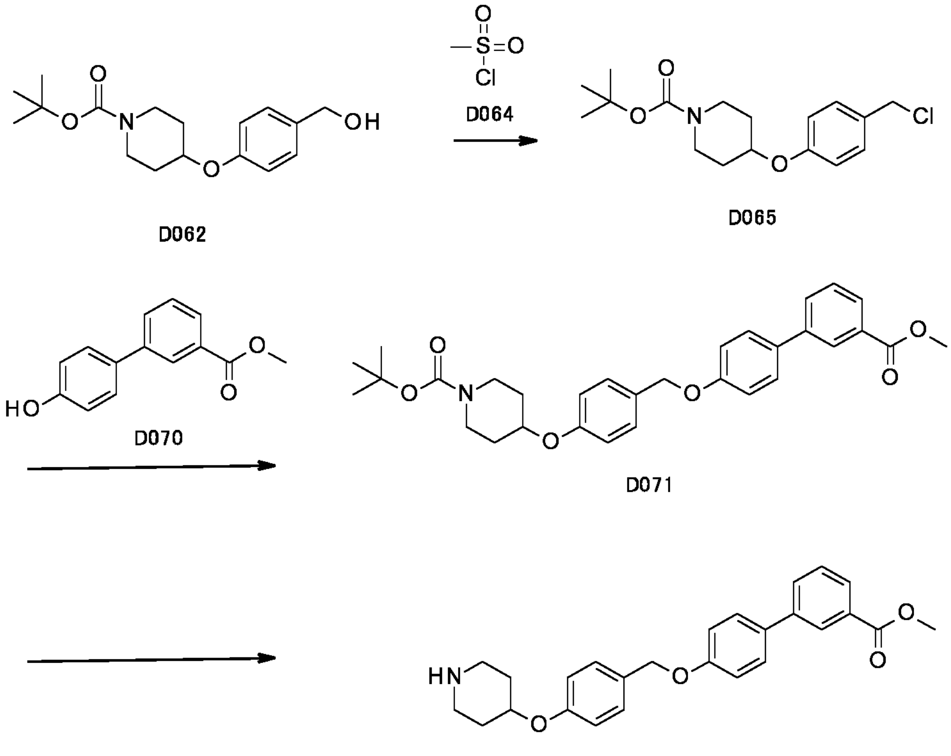


D062 D065 D071 D068

Methyl 3-[4-[(4-piperidin-4-yloxyphenyl)methoxy]phenyl]benzoate (D068) can be synthesized from tert-butyl 4-[4-(hydroxymethyl)phenoxy]piperidine-1-carboxylate (D062) and methyl 3-(4-hydroxyphenyl)benzoate (D070) by use of the methods shown in Examples 1-1-3 and 1-1-4.

D068 whose synthesis method is illustrated in Example 1-1-5 is also referred to as compound C101 in the subsequent examples.

Example 1-1-6: Synthesis of compound C002-03R

[Formula 40]

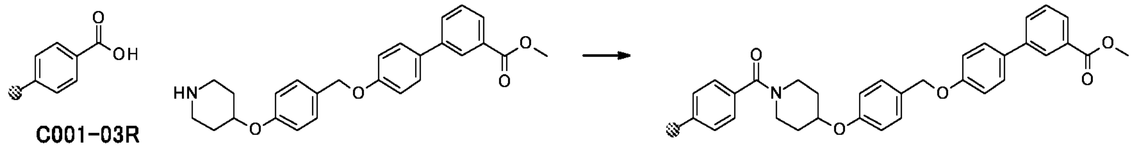

C001-03R C101 C002-03R

Under a nitrogen atmosphere, carboxylic acid resin (C001-03R) (loading rate: 2.19 mmol/g, 4.50 g, 9.86 mmol) and NMP (67.5 mL) were added to a 100 mL glass vial and shaken at room temperature for 1 hour. Methyl 3-[4-[(4-piperidin-4- yloxyphenyl)methoxy]phenyl]benzoate (C101) (1.315 g, 3.15 mmol), HOAt (1.34 g, 9.86 mmol), and DIC (1.54 mL, 9.86 mmol) were added thereto, and the mixture was shaken at room temperature for 22 hours. Piperidine (5.85 mL, 59.1 mmol), HOAt (8.05 g, 59.1 mmol), and DIC (9.21 mL, 59.1 mmol) were added thereto, and the mixture was shaken overnight at room temperature.

Solid-phase purification

The suspension of the reaction solution and the resin was wholly transferred onto a filter and washed three times with NMP (90 mL), three times with methanol (90 mL), three times with DCM (90 mL), and three times with heptane

(90 mL), and the obtained solid phase was dried overnight under reduced pressure to obtain compound C002-03R (loading rate: 0.507 mmol/g, 6.87 g, 3.48 mmol).

Reaction monitoring

The suspension of the reaction solution and the resin (0.010 mL) was transferred onto a filter, washed three times with NMP (0.1 mL), three times with methanol (0.1 mL), and three times with DCM (0.1 mL), and then dipped in a 10% solution of TFA in DCM containing 0.2 M pentamethylbenzene (0.05 mL) for 2 minutes. After filtration, NMP (0.25 mL) was added to the filtrate. Acetonitrile (0.25 mL) was added to a 0.050 mL aliquot of the obtained solution to prepare an LC sample. The degree of progression of the reaction was measured by LCMS analysis. As a result, 100% of compound C002 of interest was observed.

[Formula 41]

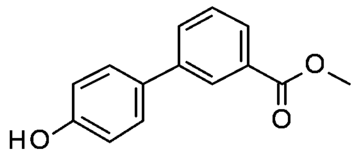

Compound C002

LRMS: m/z 229[M+H]+.

Retention time: 1.006 min (analysis conditions SMD-FA05-1, 280 nm).

Example 1-1-7: Synthesis of compound C003-03R

[Formula 42]

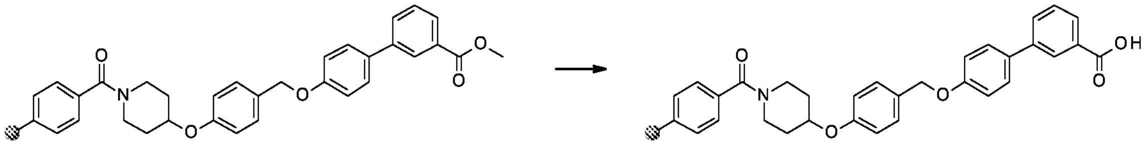

C002-03R C003-03R

Under a nitrogen atmosphere, compound C002-03R (loading rate: 0.507 mmol/g, 6.79 g, 3.44 mmol), methanol (4.07 mL), and THF (36.6 mL) were added to a 50 mL glass vial and shaken at room temperature for 1 hour. An aqueous sodium hydroxide solution (5 M, 3.55 mL, 17.8 mmol) was added thereto, and the mixture was shaken at 60°C for 3 hours.

Solid-phase purification

The suspension of the reaction solution and the resin was wholly transferred onto a filter and washed three times with NMP (130 mL), three times with water (130 mL), three times with HOAt (0.2 M, a solution in NMP, 130 mL), three times with NMP (130 mL), three times with methanol (130 mL), and three times with DCM (130 mL), and the obtained solid phase was dried overnight under reduced pressure to obtain compound C003-03R (loading rate: 0.511 mmol/g, 5.88 g, 3.00 mmol).

Reaction monitoring

The suspension of the reaction solution and the resin (0.010 mL) was transferred onto a filter, washed three times with NMP (0.1 mL), three times with methanol (0.1 mL), and three times with DCM (0.1 mL), and then dipped in a 10% solution of TFA in DCM containing 0.2 M pentamethylbenzene (0.05 mL) for 2 minutes. After filtration, NMP (0.25 mL) was added to the filtrate. Acetonitrile (0.25 mL) was added to a 0.050 mL aliquot of the obtained solution to prepare an LC sample. The degree of progression of the reaction was measured by LC-MS analysis. As a result, 100% of compound C003 of interest was observed.

[Formula 43]

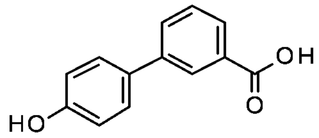

Compound C003

Maximum wavelength: 267 nm.

Retention time: 0.806 min (analysis conditions SMD-FA05-1, 280 nm).

Example 1-1-8: Synthesis of compound C004-03R

[Formula 44]

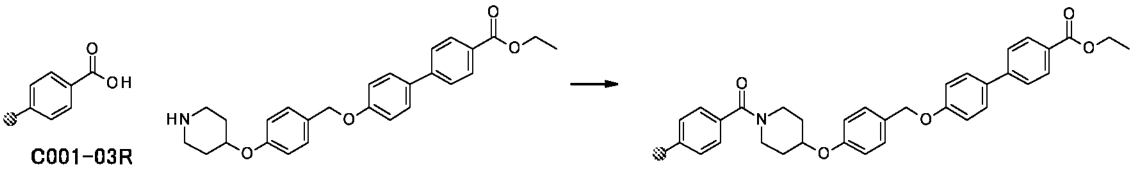

C001-03R C102 C004-03R

Under a nitrogen atmosphere, carboxylic acid resin (C001-03R) (loading rate: 2.19 mmol/g, 1.00 g, 2.19 mmol) and NMP (15 mL) were added to a 20 mL glass vial and shaken at room temperature for 1 hour. Ethyl 4-[4-[(4-piperidin-4- yloxyphenyl)methoxy]phenyl]benzoate (C102) (0.106 g, 0.246 mmol), piperidine (0.033 mL, 0.329 mmol), HOAt (0.298 g, 2.19 mmol), and DIC (0.341 mL, 2.19 mmol) were added thereto, and the mixture was shaken at room temperature for 4 hours. Piperidine (1.30 mL, 13.1 mmol) and HOAt (1.79 g, 13.1 mmol), DIC (2.05 mL, 13.1 mmol) were added thereto, and the mixture was shaken overnight at room temperature.

Solid-phase purification

The suspension of the reaction solution and the resin was wholly transferred onto a filter and washed three times with NMP (20 mL), three times with methanol (20 mL), and three times with DCM (20 mL), and the obtained solid phase was dried overnight under reduced pressure to obtain compound C004-03R (loading rate: 0.200 mmol/g, 1.25 g, 0.250 mmol).

Reaction monitoring

The suspension of the reaction solution and the resin (10 μL) was transferred onto a filter, washed three times with NMP (0.1 mL), three times with methanol (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with NMP (0.05 mL), and acetonitrile (0.25 mL) was added to the filtrate to prepare an LC sample. The degree of progression of the reaction was measured by LC-MS analysis. As a result, 100% of compound C004 of interest was observed.

[Formula 45]

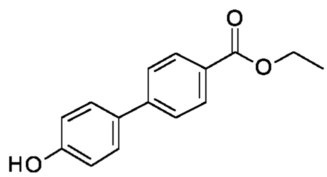

Compound C004

LRMS: m/z 243$[M+H]^+$.

Retention time: 1.085 min (analysis conditions SMD-FA05-1, 299 nm).

Example 1-1-9: Synthesis of compound C005-03R

[Formula 46]

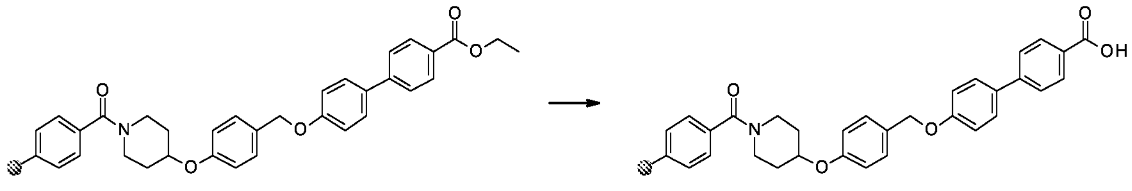

C004-03R C005-03R

Under a nitrogen atmosphere, compound C004-03R (loading rate: 0.200 mmol/g, 1.25 g, 0.250 mmol), THF (7.2 mL), methanol (0.8 mL), and an aqueous sodium hydroxide solution (5 M, 0.8 mL, 4.0 mmol) were added to a 10 mL glass vial and shaken at 60°C for 6 hours.

Solid-phase purification

The suspension of the reaction solution and the resin was wholly transferred onto a filter and washed three times with NMP (20 mL), three times with water (20 mL), three times with HOAt (0.2 M, a solution in NMP, 20 mL), three times with NMP (20 mL), three times with methanol (20 mL), and three times with DCM (20 mL), and the obtained solid phase was dried overnight under reduced pressure to obtain compound C005-03R (loading rate: 0.201 mmol/g, 1.16 g, 0.232 mmol).

Reaction monitoring

The suspension of the reaction solution and the resin (10 μL) was transferred onto a filter, washed three times with NMP (0.1 mL), three times with methanol (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with NMP (0.05 mL), and acetonitrile (0.25 mL) was added to the filtrate to prepare an LC sample. The degree of progression of the reaction was measured by LC-MS analysis. As a result, 100% of compound C005 of interest was observed.

[Formula 47]

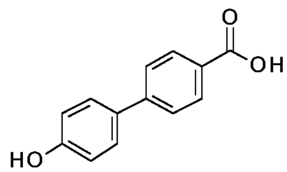

Compound C005

Maximum wavelength: 293 nm.

Retention time: 0.775 min (analysis conditions SMD-FA05-1, 299 nm).

Example 1-1-10: Synthesis of compound C006-03R

[Formula 48]

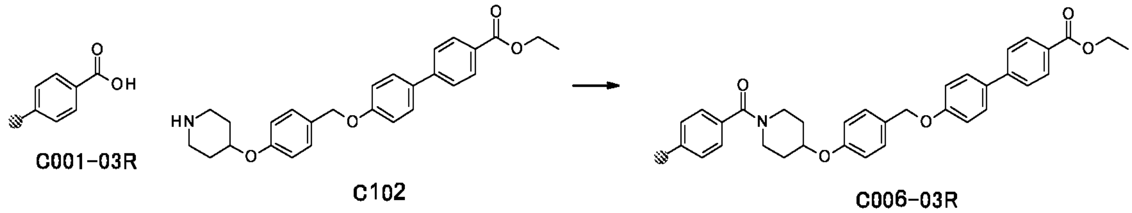

C001-03R C102 C006-03R

Under a nitrogen atmosphere, carboxylic acid resin (C001-03R) (loading rate: 2.19 mmol/g, 1.00 g, 2.19 mmol) and NMP (15 mL) were added to a 20 mL glass vial and shaken at room temperature for 1 hour. Ethyl 4-[4-[(4-piperidin-4- yloxyphenyl)methoxy]phenyl]benzoate (C102) (0.025 g, 0.058 mmol), piperidine (0.033 mL, 0.329 mmol), HOAt (0.298 g, 2.19 mmol), and DIC (0.341 mL, 2.19 mmol) were added thereto, and the mixture was shaken at room temperature for 6 hours. Piperidine (1.30 mL, 13.1 mmol) and HOAt (1.79 g, 13.1 mmol), DIC (2.05 mL, 13.1 mmol) were added thereto, and the mixture was shaken overnight at room temperature.

Solid-phase purification

The suspension of the reaction solution and the resin was wholly transferred onto a filter and washed three times with NMP (20 mL), three times with methanol (20 mL), and three times with DCM (20 mL), and the obtained solid phase was dried overnight under reduced pressure to obtain compound C006-03R (loading rate: 0.050 mmol/g, 1.18 g, 0.059 mmol).

Example 1-1-11: Synthesis of compound C007-03R

[Formula 49]

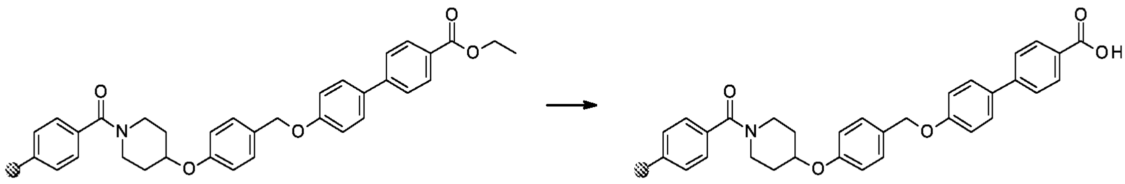

C006-03R C007-03R

Under a nitrogen atmosphere, compound C006-03R (loading rate: 0.050 mmol/g, 1.18 g, 0.059 mmol), THF (7.2 mL), methanol (0.8 mL), and an aqueous sodium hydroxide solution (5 M, 0.8 mL, 4.0 mmol) were added to a 10 mL glass vial and shaken at 60°C for 6 hours.

Solid-phase purification

The suspension of the reaction solution and the resin was wholly transferred onto a filter and washed three times with NMP (20 mL), three times with water (20 mL), three times with HOAt (0.2 M, a solution in NMP, 20 mL), three times with NMP (20 mL), three times with methanol (20 mL), and three times with DCM (20 mL), and the obtained solid phase was dried overnight under reduced pressure to obtain compound C007-03R (loading rate: 0.050 mmol/g, 1.10 g, 0.055 mmol).

Example 1-1-12: Synthesis of compound AA001-03R

[Formula 50]

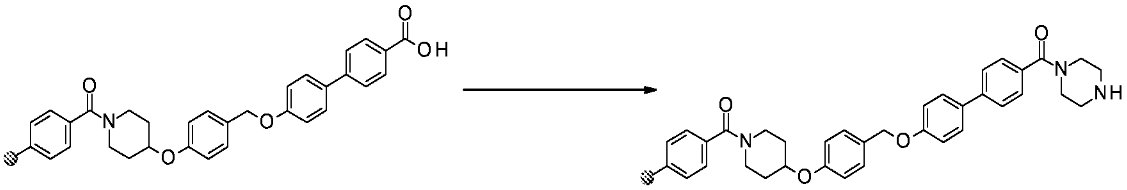

C007-03R

AA001-03R

Under a nitrogen atmosphere, compound C007-03R (loading rate: 0.050 mmol/g, 0.030 g, 1.5 μmol) and NMP (0.300 mL) were added to a 0.6 mL glass vial and shaken at room temperature for 1 hour. Piperazine (a 0.5 M solution in NMP, 30.0 μL, 0.015 mmol), HOAt (a 0.5 M solution in NMP, 30.0 μL, 0.015 mmol), and DIC (2.34 μL, 0.015 mmol) were added thereto, and the mixture was shaken at 40°C for 14 hours.

Solid-phase purification

The suspension of the reaction solution and the resin was transferred onto a filter using NMP (0.6 mL) and washed twice with NMP (0.6 mL), three times with methanol (0.6 mL), and three times with DCM (0.6 mL), and the obtained solid phase was dried overnight under reduced pressure to obtain compound AA001-03R (loading rate: 0.050 mmol/g, 25.6 mg, 1.28 mmol).

Reaction monitoring

The suspension of the reaction solution and the resin (0.020 mL) was transferred onto a filter, washed three times with NMP (0.1 mL), three times with methanol (0.1 mL), and three times with DCM (0.1 mL), and then dipped in a 10% solution of TFA in DCM containing 0.02 M pentamethylbenzene (0.05 mL) for 5 minutes. After filtration, the resin on the filter was washed with NMP (0.05 mL). Acetonitrile (0.25 mL) was added to 50 μL of the obtained filtrate to prepare an LC sample. The degree of progression of the reaction was measured by LC-MS analysis. As a result, compound AA001 of interest was observed at a purity of 88.9%.

[Formula 51]

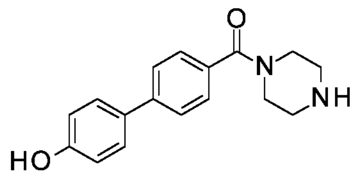

AA001

Compound AA001

LRMS: m/z 283[M+H]$^+$.

Retention time: 0.471 min (analysis conditions SMD-FA05-1, 280 nm).

AA001-03R whose synthesis method is illustrated in Example 1-1-12 is also referred to as compound D021-03R in the subsequent examples.

Example 1-1-13: Synthesis of compound AA003-03R

[Formula 52]

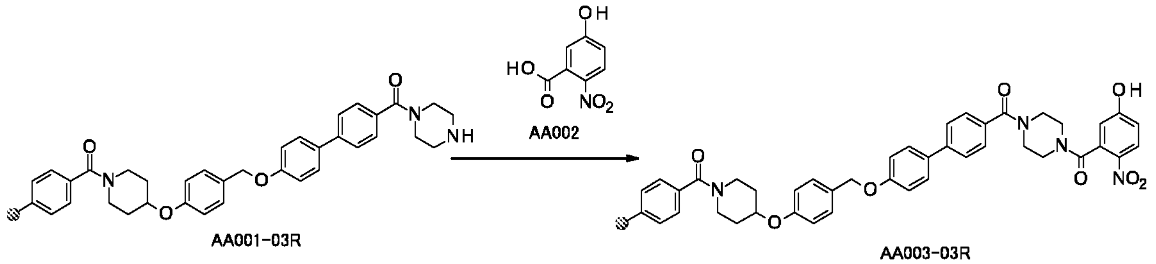


Under a nitrogen atmosphere, compound AA001-03R (loading rate: 0.195 mmol/g, 298 mg, 58.1 μmol) and NMP (4.47 mL) were added to an 8 mL glass vial and shaken at room temperature for 1 hour. 5-Hydroxy-2-nitrobenzoic acid (AA002, 26.6 mg, 0.145 mmol), HOAt (19.8 mg, 0.145 mmol), and DIC (22.6 μL, 0.145 mmol) were added thereto, and the mixture was shaken at room temperature for 2 hours. Under a nitrogen atmosphere, methanol (447 μL) and lithium hydroxide (a 2 M aqueous solution, 447 μL, 0.894 mmol) were added to the reaction mixture, and the mixture was shaken at room temperature for 1 hour.

Solid-phase purification

The suspension of the reaction solution and the resin was transferred onto a filter using NMP (5.96 mL) and washed twice with NMP (5.96 mL), three times with water (5.96 mL), three times with HOAt (0.2 M, a solution in NMP, 5.96 mL), three times with NMP (5.96 mL), three times with methanol (5.96 mL), three times with DCM (5.96 mL), and three times with heptane (5.96 mL), and the obtained solid phase was dried overnight under reduced pressure to obtain compound AA003-03R (loading rate: 0.189 mmol/g, 275.2 mg, 52.0 μmol).

Reaction monitoring

The suspension of the reaction solution and the resin (0.020 mL) was transferred onto a filter, washed three times with NMP (0.1 mL), three times with methanol (0.1 mL), and three times with DCM (0.1 mL), and then dipped in a 10% solution of TFA in DCM containing 0.02 M pentamethylbenzene (0.05 mL) for 5 minutes. After filtration, the resin on the filter was washed with NMP (0.05 mL). Acetonitrile (0.25 mL) was added to 50 μL of the obtained filtrate to prepare an LC sample. The degree of progression of the reaction was measured by LC-MS analysis. As a result, compound AA003 of interest was observed at a purity of 95.6%.

[Formula 53]

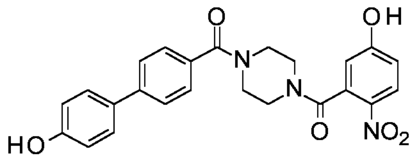

AA003

Compound AA003
LCMS: m/z 448[M+H]+.
Retention time: 0.817 min (analysis conditions SMD-FA05-1, 280 nm).

Example 1-1-14: Synthesis of compound AA013-03R

[Formula 54]

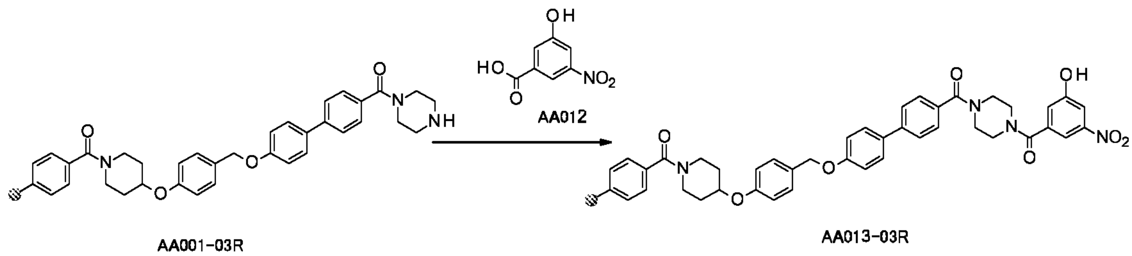


Compound AA013-03R (loading rate: 0.189 mmol/g, 32.4 mg, 6.5 μmol) was obtained by synthesis in the same manner as in Example 1-1-13 using compound AA001- 03R (loading rate: 0.195 mmol/g, 37.0 mg, 7.4 μmol) and 3-hydroxy-5-nitrobenzoic acid (AA012, 3.96 mg, 0.0022 mmol).

[Formula 55]

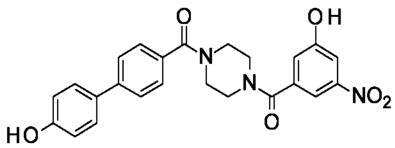

AA013-03R

Compound AA013

LCMS: m/z 448[M+H]$^+$.

Retention time: 0.852 min (analysis conditions SMD-FA05-1, 280 nm).

Example 1-1-15: Synthesis of AA015-03R

[Formula 56]

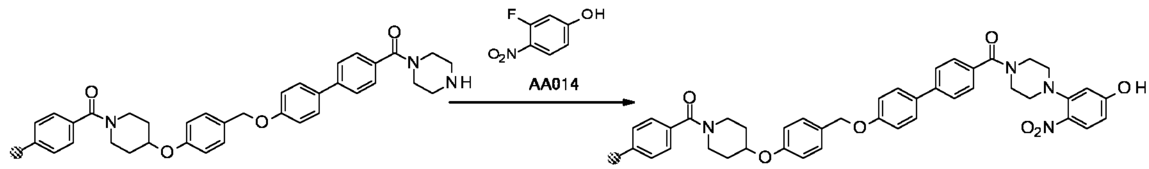


Under a nitrogen atmosphere, compound AA001-03R (loading rate: 0.195 mmol/g, 10.0 mg, 1.95 μmol) and NMP (150 mL) were added to a 0.6 mL glass vial and shaken at room temperature for 1 hour. 3-Fluoro-4-nitrophenol (AA014, 1.53 mg, 9.75 μmol) and DIPEA (3.41 μL, 0.020 mmol) were added thereto, and the mixture was shaken at 100°C for 24 hours.

Solid-phase purification

The suspension of the reaction solution and the resin was transferred onto a filter using NMP (0.2 mL) and washed twice with NMP (0.2 mL), three times with water (0.2 mL), three times with NMP (0.2 mL), three times with HOAt (0.2 M, a solution in NMP, 0.2 mL), three times with NMP (0.2 mL), three times with methanol (0.2 mL), three times with DCM (0.2 mL), and three times with heptane (0.2 mL), and the obtained solid phase was dried overnight under reduced pressure to obtain compound AA015-03R (loading rate: 0.189 mmol/g, 10.0 mg, 1.89 μmol).

Reaction monitoring

The suspension of the reaction solution and the resin (0.020 mL) was transferred onto a filter, washed three times with NMP (0.1 mL), three times with methanol (0.1 mL), and three times with DCM (0.1 mL), and then dipped in a 10% solution of TFA in DCM containing 0.02 M pentamethylbenzene (0.05 mL) for 5 minutes. After filtration, the resin

on the filter was washed with NMP (0.05 mL). Acetonitrile (0.25 mL) was added to 50 μL of the obtained filtrate to prepare an LC sample. The degree of progression of the reaction was measured by LC-MS analysis. As a result, compound AA015 of interest was observed at a purity of 96.9%.

[Formula 57]

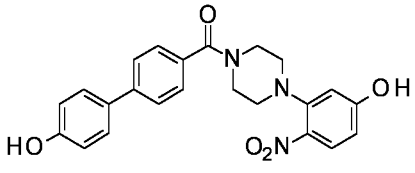

AA015

Compound AA015

LCMS: m/z 420[M+H]+.

Retention time: 0.937 min (analysis conditions SMD-FA05-1, 280 nm).

Example 1-1-16: Synthesis of AA017-03R

[Formula 58]

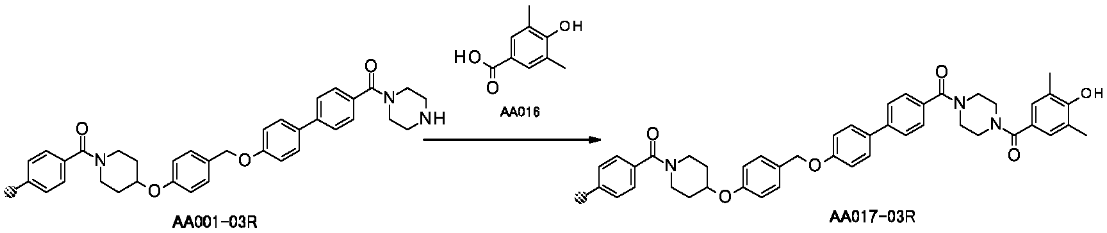

AA001-03R AA016 AA017-03R

Compound AA017-03R (loading rate: 0.189 mmol/g, 32.1 mg, 6.1 μmol) was obtained by synthesis in the same manner as in Example 1-1-13 using compound AA001- 03R (loading rate: 0.195 mmol/g, 37.0 mg, 7.4 μmol) and 4-hydroxy-3,5-dimethylbenzoic acid (AA016, 3.60 mg, 0.0022 mmol).

[Formula 59]

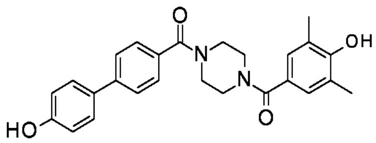

AA017

Compound AA017

LCMS: m/z 431[M+H]+.

Retention time: 0.843 min (analysis conditions SMD-FA05-1, 280 nm).

Example 1-2: Synthesis of substrate for reaction study for use mainly in Example 2- 2, building block for use in mixture library, etc.

Example 1-2-1: Method for synthesizing 4-(4-bromophenyl)-1-hydroxy-N,N- dimethyl-2-naphthamide (compound E002)

Naphthol reagents (compound E002 and compound E003) used in Examples 1-2-7 and 1-2-9 were synthesized by the following method.

[Formula 60]

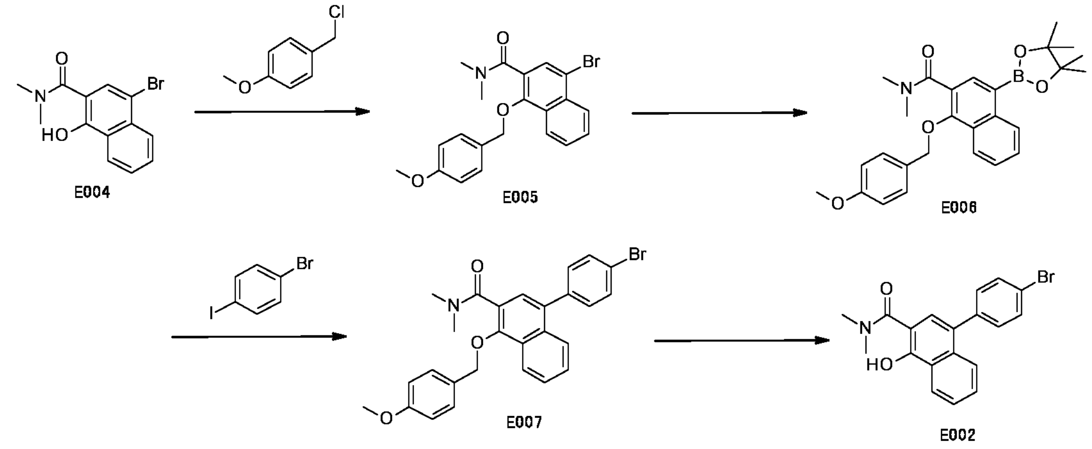

Example 1-2-2: Synthesis of 4-bromo-1-((4-methoxybenzyl)oxy)-N,N-dimethyl-2- naphthamide (compound E005)

To a solution of 4-bromo-1-hydroxy-N,N-dimethyl-2-naphthamide (compound E004) (60 g, 204 mmol) in DMA (510 mL), 1-(chloromethyl)-4-methoxybenzene (33.3 mL, 245 mmol) and tBuOK (25.2 g, 224 mmol) were added at room temperature. After stirring at 90°C for 1 hour and 30 minutes, water (1 L), EtOAc (300 mL), and hexane (200 mL) were added to the reaction solution, and an organic layer was extracted. The organic layer was washed with sodium chloride aqueous solution and then concentrated under reduced pressure to obtain the title compound (compound E005) as a crude product. The subsequent reaction was performed without carrying out further purification.

LCMS: m/z 412$[M-H]^-$.

Retention time: 1.31 min (analysis conditions SMD-FA05-1).

Example 1-2-3: Synthesis of 1-((4-methoxybenzyl)oxy)-N,N-dimethyl-4-(4,4,5,5- tetramethyl-1,3,2-dioxaboloran-2-yl)-2-naphthamide (compound E006)

Under a nitrogen atmosphere, dioxane (1026 mL) was added at room temperature to 4-bromo-1-((4-methoxybenzyl)oxy)-N,N-dimethyl-2-naphthamide (compound E005) (85 g, 205 mmol), bis(pinacolato)diboron, Pd(dppf)$Cl_2$·DCM (8.38 g, 10.3 mmol), and potassium acetate (60.4 g, 615 mmol), and the mixture was heated at 90°C for 2 hours to obtain the title compound (compound E006) as a solution of a crude product. The subsequent reaction was performed without carrying out further purification.

LCMS: m/z 462$[M+H]^+$.

Retention time: 1.45 min (analysis conditions SMD-FA05-1).

Example 1-2-4: Synthesis of 4-(4-bromophenyl)-1-((4-methoxybenzyl)oxy)-N,N- dimethyl-2-naphthamide (compound E007)

Under a nitrogen atmosphere, 1-bromo-4-iodobenzene (69.6 g, 246 mmol) and Pd(dppf)$Cl_2$·DCM (8.37 g, 10.3 mmol) were added at room temperature to the reaction solution containing 1-((4-methoxybenzyl)oxy)-N,N-dimethyl-4-(4,4,5,5-tetramethyl-1,3,2- dioxaboloran-2-yl)-2-naphthamide (compound E006) (95 g, 205 mmol) obtained in the preceding step (Example 1-2-2), and the mixture was stirred. Potassium phosphate (131 g, 615 mmol) and water (137 mL) were added at room temperature to the reaction solution, and the mixture was then stirred at 90°C for 2 hours. The organic layer of the reaction solution was filtered through silica gel (300 mL), followed by elution with ethyl acetate (800 mL).

The filtrate was concentrated under reduced pressure, and the residue was purified by silica gel column chromatography (ethyl acetate/hexane, 0-35%) to obtain a brown amorphous crude product. The obtained crude product was milled and washed with a mixed solvent of ethyl acetate (20 mL) and hexane (180 mL), and the obtained solid was filtered for solvent removal to obtain a white solid of the title compound (compound E007, 51%, 50.7 g, 103 mmol).

LCMS: m/z 488$[M-H]^-$.

Retention time: 1.50 min (analysis conditions SMD-FA05-1).

Example 1-2-5: Synthesis of 4-(4-bromophenyl)-1-hydroxy-N,N-dimethyl-2- naphthamide (compound E002)

To a solution of (4-(4-bromophenyl)-1-((4-methoxybenzyl)oxy)-N,N-dimethyl-2- naphthamide) (62.6 g, 128

mmol) (compound E007) in DCM (638 mL), TFA (48.8 mL, 638 mmol) was added, and the mixture was stirred at room temperature for 1 hour. The reaction solution was concentrated under reduced pressure. Then, ethyl acetate (200 mL) was added thereto, and the mixture was concentrated under reduced pressure again. The residue was milled and washed by the addition of hexane (500 mL), and the obtained solid was washed with a mixed solvent of ethyl acetate (30 mL) and hexane (270 mL) to obtain a white solid. The obtained solid was dissolved in ethyl acetate (500 mL), and an aqueous sodium bicarbonate solution (300 mL) was added to the solution. An organic layer was extracted and concentrated under reduced pressure. The residue was milled and washed with ethyl acetate (50 mL) and hexane (500 mL) to obtain a white solid of the title compound (compound E002, 85%, 40.1 g, 103 mmol).

LCMS: m/z 370[M+H]+.

Retention time: 1.41 min (analysis conditions SMD-FA05-1).

Example 1-2-6: Synthesis of 4-(5-bromopyridin-2-yl)-1-hydroxy-N,N-dimethyl-2- naphthamide (compound E003)

The compound was synthesized in the same manner as in Example 1-2-4 (compound E007) and Example 1-2-5 (compound E002) using 5-bromo-2-iodopyridine.

[Formula 61]

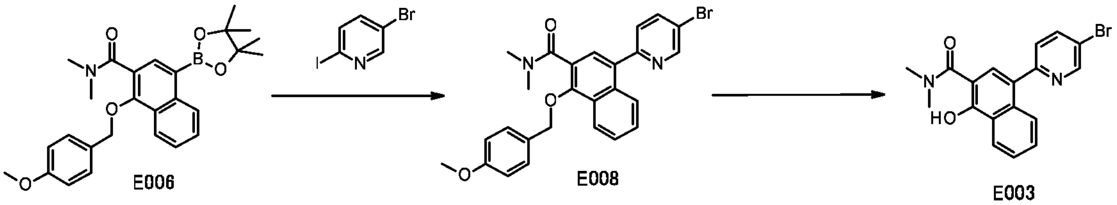

E006 E008 E003

4-(5-Bromopyridin-2-yl)-1-((4-methoxybenzyl)oxy)-N,N-dimethyl-2-naphthamide (compound E008)

LCMS: m/z 491[M+H]+.

Retention time: 1.32 min (analysis conditions SMD-FA05-1).

4-(5-Bromopyridin-2-yl)-1-hydroxy-N,N-dimethyl-2-naphthamide (compound E003)

LCMS: m/z 371[M+H]+.

Retention time: 1.17 min (analysis conditions SMD-FA05-1).

Example 1-2-7: Synthesis of compound EA01-01-01R

[Formula 62]

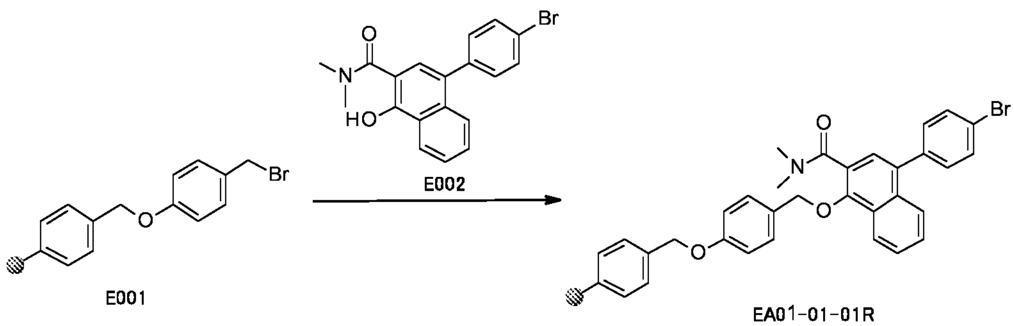

E002

E001 EA01-01-01R

Under a nitrogen atmosphere, NMP (200 mL) was added to brominated Wang resin (compound E001, Sigma-Aldrich Co., LLC, 534471, 100-200 mesh, loading rate: 0.5 mmol/g, 1% cross-linked) (10 g, 5.00 mmol) and 4-(4-bromophenyl)-1-hydroxy-N,N-dimethyl-2- naphthamide (compound E002, 3.70 g, 10.0 mmol), and the mixture was shaken at room temperature for 1 hour. tBuOK (1.07 g, 9.5 mmol) was added thereto, and the mixture was shaken at room temperature for 18 hours. The reaction suspension was transferred to a column with a filter, and the reaction

solution was removed by the application of nitrogen pressure, followed by the washing of the resin three times with NMP (200 mL). Further, the resin was repetitively washed three times with water (200 mL), three times with methanol (200 mL), and three times with DCM (200 mL), and the obtained resin was dried overnight under reduced pressure to obtain the title compound (compound EA01-01-01R) (10.2 g, loading rate: 0.4 mmol/g).

Example 1-2-8: Determination of loading rate of compound EA01-01-01R supported

The loading rate a (mmol/g) of the resin was determined by the following method.

The following experimental operation is an example and does not uniformly define the loading rate as to all lots.

[Formula 63]

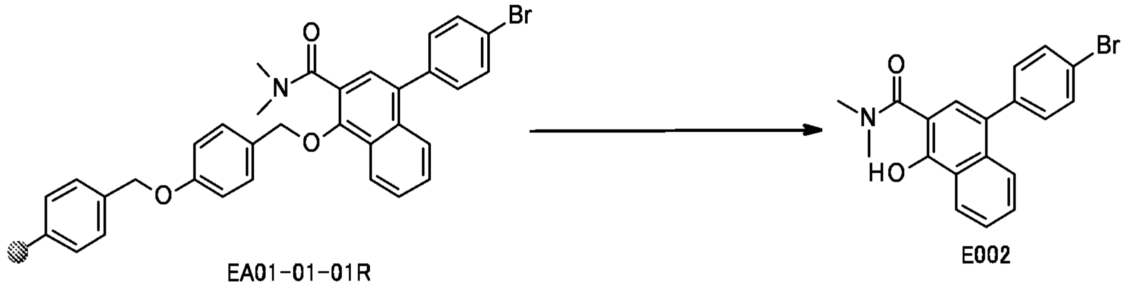

EA01-01-01R E002

Under a nitrogen atmosphere, compound EA01-01-01R (31.0 mg), DCM (2.70 mL), and pentamethylbenzene (230 mg, 1.55 mmol) were added to a 0.6 mL glass vial and shaken at room temperature for 1 hour. TFA (300 μL, 2.63 mmol) was added thereto, and the mixture was shaken at room temperature for 2 hours. 30 μL of the supernatant solution of the reaction was sampled and diluted with TFE (1170 μL) to prepare an LC sample. The reaction was monitored by LC-MS analysis.

The concentration of the obtained E002 in the solution was calculated from UV area% and a calibration curve to determine the amount of E002 supported by the resin. As a result, the amount of the compound supported was calculated as 0.40 mmol/g. Analysis was conducted under analysis conditions SMD-FA05-1, and the UV area% was confirmed at a UV wavelength of 319 nm.

LCMS: m/z 370.0,372.0$[M+H]^+$
Retention time: 1.45 min.

Example 1-2-9: Synthesis of compound EA01-02-01R

[Formula 64]

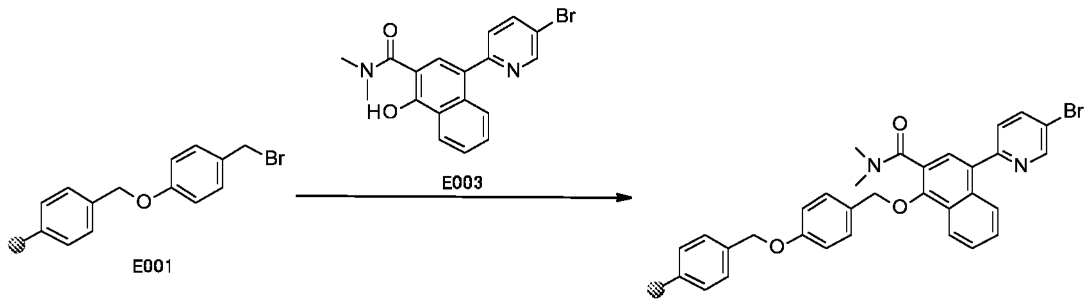

E001 E003 EA01-02-01R

The compound was synthesized in the same manner as in Example 1-2-7 (compound EA01-02-01R) using 4-(5-bromopyridin-2-yl)-1-hydroxy-N,N-dimethyl-2-naphthamide (compound E003).
(Loading rate: 0.37 mmol/g)

Example 1-2-10:

The amount of compound EA01-02-01R supported was determined in the same manner as in Example 1-2-8.

[Formula 65]

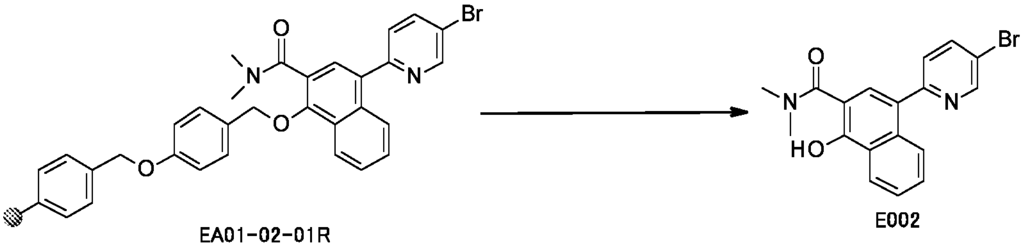

EA01-02-01R

E002

LCMS: m/z 371.0,373.0[M+H]+.
Retention time: 1.21 min.

Example 1-2-11: Method for synthesizing {1-[3-ethoxycarbonyl-5- (trifluoromethyl)phenyl]pyrazol-4-yl}boronic acid (BB06)

[Formula 66]

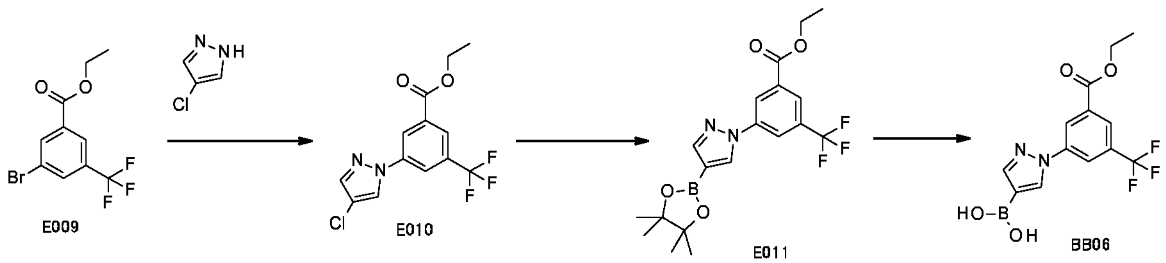

E009 E010 E011 BB06

Example 1-2-12: Synthesis of ethyl 3-(4-chloro-1H-pyrazol-1-yl)-5- (trifluoromethyl)benzoate (compound E010)

A solution of ethyl 3-bromo-5-(trifluoromethyl)benzoate (compound E009) (2.08 g, 7 mmol), 4-chloro-1H-pyrazole (1.08 g, 10.5 mmol), copper iodide (0.133 g, 0.700 mmol), N,N'-dimethylethylenediamine (0.30 mL, 2.8 mmol), and potassium carbonate (2.90 g, 21.00 mmol) in dioxane (17.5 mL) was purged with nitrogen three times. The solution was stirred at 110°C for 16 hours. The mixture was filtered through celite and washed five times with ethyl acetate (25 mL). The filtrate was washed twice with an aqueous ammonium chloride solution (40 mL), once with water (40 mL), and once with saturated sodium chloride aqueous solution (40 mL), then dried over sodium sulfate, and concentrated under reduced pressure. The residue was purified by silica gel column chromatography (ethyl acetate/hexane, 20%) to obtain a white solid of the title compound (compound E010, 87%, 1.93 g, 6.07 mmol).
LCMS: m/z 319[M+H]+.
Retention time: 1.33 min (analysis conditions SMD-TFA05-1).

Example 1-2-13: Synthesis of ethyl 3-(4-(4,4,5,5-tetramethyl-1,3,2-dioxaboloran-2- yl)-1H-pyrazol-1-yl)-5-(trifluoromethyl)benzoate (compound E011)

A solution of ethyl (3-(4-chloro-1H-pyrazol-1-yl)-5-(trifluoromethyl)benzoate (compound E010) (1.93 g, 6.06 mmol), bis(pinacolato)diboron (1.92 g, 7.57 mmol), RuPhos (424 mg, 0.908 mmol), an allyl-palladium(II) chloride dimer (111 mg, 0.303 mmol), and potassium acetate (1.49 g, 15.1 mmol) in dioxane (30 mL) was purged with nitrogen. The solution was stirred at 90°C for 16.5 hours. The mixture was filtered through celite and washed five times with ethyl acetate (20 mL). The filtrate was washed with saturated sodium chloride aqueous solution (40 mL), then dried over sodium sulfate, and concentrated under reduced pressure. The obtained title compound (compound E011) in a pale

yellow oil form was used as a mixture in the subsequent step without being purified.
LCMS: m/z 411[M+H]+.
Retention time: 1.53 min (analysis conditions SMD-FA05-1).

Example 1-2-14: Synthesis of (1-(3-(ethoxycarbonyl)-5-(trifluoromethyl)phenyl)- 1H-pyrazol-4-yl)boronic acid (BB06)

Ethyl 3-(4-(4,4,5,5-tetramethyl-1,3,2-dioxaboloran-2-yl)-1H-pyrazol-1-yl)-5- (trifluoromethyl)benzoate (compound E011) (corresponding to 6.06 mmol) obtained in the preceding step (Example 1-2-13), sodium periodate (6.48 g, 30.3 mmol), ammonium acetate (2.34 g, 30.3 mmol), acetone (40 mL), and water (20 mL) were stirred at room temperature for 19 hours. Ethyl acetate (100 mL) and water (50 mL) were added to the mixture. An organic layer was extracted, followed by further extraction twice with ethyl acetate (100 mL). The organic layer was dried over sodium sulfate and concentrated under reduced pressure. The residue was purified by reverse-phase column chromatography (0.1% solution of formic acid in acetonitrile/0.1% aqueous formic acid solution, 50-100%). The obtained solid was washed five times with water (15 mL) using Kiriyama Rohto. The obtained solid was dried under reduced pressure to obtain the title compound (BB06, 55%, 1.09 g, 3.32 mmol).
LCMS: m/z 329[M+H]+.
Retention time: 1.02 min (analysis conditions SMD-FA05-1).

Example 1-3: Synthesis of substrate for reaction study for use mainly in Example 2- 3, and building block for use in mixture library, etc.

Example 1-3-1: Synthesis of ethyl 3-ethynyl-5-(trifluoromethyl)benzoate (BB25)

[Formula 67]

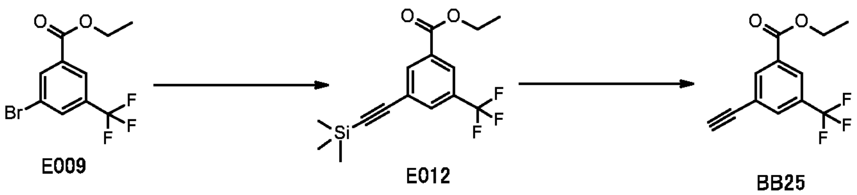

E009 E012 BB25

Example 1-3-2: Synthesis of ethyl 3-(trifluoromethyl)-5- ((trimethylsilyl)ethynyl)benzoate (compound E012)

Under a nitrogen atmosphere, a solution of ethyl 3-bromo-5- (trifluoromethyl)benzoate (compound E009, 1.49 g, 5 mmol), trimethylsilylacetylene (1.39 mL, 10.0 mmol), $PdCl_2(PPh_3)_2$ (0.175 g, 0.25 mmol), and copper iodide (48 mg, 0.25 mmol) in triethylamine (14.9 mL, 107 mmol) was stirred at 90°C for 2.5 hours. The mixture was filtered through celite and washed four times with ethyl acetate (20 mL). The filtrate was concentrated under reduced pressure. The residue was purified by silica gel column chromatography (NH-silica gel) (ethyl acetate/hexane, 10%) to obtain a light brown oil of the title compound (compound E012, 104%, 1.64 g, 5.22 mmol).
Retention time: 1.70 min (analysis conditions SMD-FA05-1).
$^1$H-NMR (400 MHz, DMSO-$d_6$) δ: 8.18 (s, 1H), 8.15 (s, 1H), 8.10 (s, 1H), 4.36 (q, J = 7.0 Hz, 2H), 1.35 (t, J = 7.0 Hz, 3H), 0.26 (s, 9H).

Example 1-3-3: Synthesis of ethyl 3-ethynyl-5-(trifluoromethyl)benzoate (BB25)

A solution of ethyl 3-(trifluoromethyl)-5-((trimethylsilyl)ethynyl)benzoate (compound E012, 1.64 g, 5.22 mmol) and potassium carbonate (0.721 g, 5.22 mmol) in ethanol (20.8 mL) was stirred at room temperature for 1.5 hours. The mixture was filtered through celite and washed three times with methanol (20 mL) and three times with ethyl acetate (20 mL). The filtrate was concentrated. The residue was dissolved in DMSO and a 0.1% aqueous formic acid solution, and the solution was purified by reverse-phase column chromatography (0.1% solution of formic acid in acetonitrile/0.1% aqueous formic acid solution, 50-100%). After concentration, ethyl acetate and water were added to the obtained residue. An organic layer was extracted, washed with an aqueous sodium bicarbonate solution and saturated sodium chloride aqueous solution, and dried over sodium sulfate. The residue was concentrated to obtain a light brown oil of the title compound (BB25, 89%, 1.12 g, 4.62 mmol).

Retention time: 1.32 min (analysis conditions SMD-FA05-1)
Maximum wavelength: 213 nm

Example 1-3-4: Synthesis of ethyl 5-ethynylnicotinate (BB26)

[Formula 68]

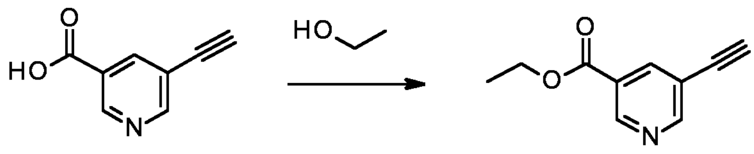

E013

BB26

To a solution of 5-ethynylpyridine-3-carboxylic acid (compound E013, 1.5 g, 10.19 mmol) in DMA (20 mL), DMAP (1.25 g, 10.2 mmol) and WSCI·HCl (3.91 g, 20.4 mmol) were added at 0°C, and the mixture was stirred for 15 minutes. Ethanol (1.12 mL, 20.4 mmol) was added thereto, and the mixture was stirred at room temperature for 20 hours. Formic acid was added to the mixture, and the resulting mixture was purified by reverse-phase column chromatography (0.1% solution of formic acid in acetonitrile/0.1% aqueous formic acid solution, 5-35%) to obtain a brown solid of the title compound (BB26, 50%, 0.89 g, 5.1 mmol).
LCMS: m/z 176[M+H]$^+$.
Retention time: 0.91 min (analysis conditions SMD-FA05-1).

Example 1-4: Synthesis of substrate for reaction study for use mainly in Examples 2-4 and 2-5, and building block for use in mixture library, etc.

Example 1-4-1: Method for synthesizing allyl 4-(piperazin-1-yl)benzoate (BB27)

[Formula 69]

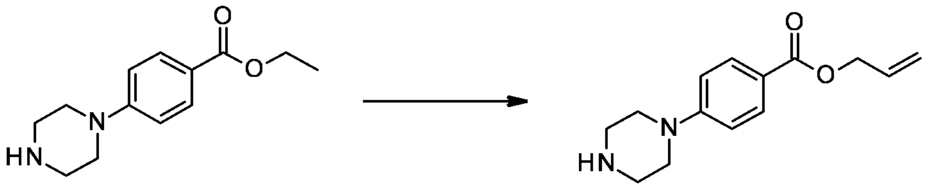

B010

BB27

Allyl alcohol (10.00 mL) and KOtBu (96.00 mg, 0.854 mmol) were added to a 50 mL eggplant flask and stirred for 10 minutes under a nitrogen atmosphere. Ethyl 4- piperazin-1-ylbenzoate (compound BO10, 1 g, 4.27 mmol) was added thereto, and the mixture was stirred at 100°C for 1 hour. The mixture was cooled to room temperature and then concentrated under reduced pressure. Allyl alcohol (12 mL) was added to the obtained residue, and the mixture was stirred at 100°C for 16.5 hours. The mixture was cooled to room temperature and then concentrated under reduced pressure. The obtained residue was purified by NH-silica gel column chromatography (ethyl acetate/hexane, 50-100%) to obtain a colorless waxy solid of the title compound (BB27, 69.0%, 0.72 g, 2.94 mmol).
LCMS (ESI, m/z): 247.1[M+H]$^+$.
Retention time: 0.595 min (analysis conditions SMD-FA05-1).
$^1$H-NMR (400 MHz, DMSO-$d_6$) $\delta$: 7.795 (d, J = 9.0 Hz, 2H), 6.956 (d, J = 9.0 Hz, 2H), 6.072-5.977 (m, 1H), 5.387-5.332 (m, 1H), 5.262-5.225 (m, 1H), 4.737-4.716 (m, 2H), 3.229-3.204 (m, 4H), 2.816-2.790 (m, 4H), 2.323 (bs, 1H).
BB27 whose synthesis method is illustrated in Example 1-4-1 is also referred to as compound D049 in the subsequent examples.

Example 1-4-2: Method for synthesizing 6-[4-[(2-methylpropan-2- yl)oxycarbonyl]piperazin-1-yl]pyridazine-3-carboxylic acid (AC012)

[Formula 70]

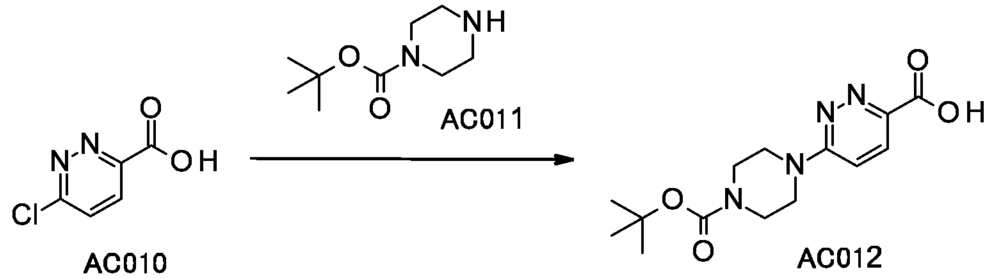

AC010 AC011 AC012

6-Chloropyridazine-3-carboxylic acid (AC010, CAS: 5096-73-1, 1.00 g, 6.31 mmol), tert-butyl piperazine-1-carboxylate (AC011, CAS: 57260-71-6, 1.76 g, 9.46 mmol, 1.5 equivalents), DIPEA (3.30 mL, 18.9 mmol, 3.0 equivalents), and acetonitrile (15.00 mL) were added to a 50 mL eggplant flask and heated to reflux at 100°C for 87 hours under a nitrogen atmosphere. The reaction solution was cooled to room temperature. Then, formic acid was added thereto, and the mixture was purified by reverse-phase column chromatography (0.1 % solution of formic acid in acetonitrile/0.1% aqueous formic acid solution, 15-50%). The resulting product was freeze-dried to obtain the title compound (AC012, 66%, 1.95 g, 4.2 mmol).

LCMS: m/z 309.2[M+H]$^+$.

Retention time: 0.70 min (analysis conditions SMD-FA05-1).

Example 1-4-3: Method for synthesizing prop-2-enyl 6-[4-[(2-methylpropan-2- yl)oxycarbonyl]piperazin-1-yl]pyridazine-3-carboxylate (AC013)

[Formula 71]

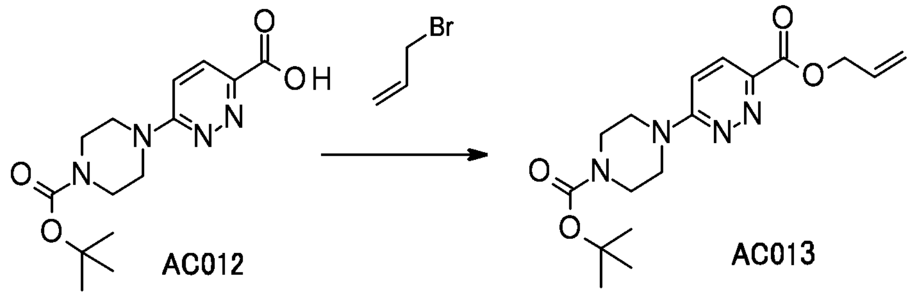

AC012 AC013

6-[4-[(2-Methylpropan-2-yl)oxycarbonyl]piperazin-1-yl]pyridazine-3-carboxylic acid (AC012, 2.10 g, 6.81 mmol), $K_2CO_3$ (2.82 g, 20.4 mmol, 3.0 equivalents), DMA (18.2 mL), and allyl bromide (0.71 mL, 8.17 mmol, 1.2 equivalents) were added to a 100 mL eggplant flask and stirred at room temperature for 3 hours and 45 minutes under a nitrogen atmosphere. DMA (9.1 mL), $K_2CO_3$ (2.82 g, 20.4 mmol, 3.0 equivalents), and allyl bromide (0.88 mL, 10.2 mmol, 1.5 equivalents) were added thereto, and the mixture was stirred at room temperature for 40 hours under a nitrogen atmosphere. Formic acid (3.66 mL) and $H_2O$ were added to the reaction solution, and the mixture was purified by reverse-phase column chromatography (0.1% solution of formic acid in acetonitrile/0.1% aqueous formic acid solution, 15-65%). After concentration, the obtained suspension was filtered through Kiriyama Rohto. The resulting product was dried under reduced pressure to obtain the title compound (AC013, 57%, 1.35 g, 3.87 mmol).

LCMS: m/z 349.2[M+H]$^+$.

Retention time: 1.06 min (analysis conditions SMD-FA05-1).

Example 1-4-4: Method for synthesizing allyl 6-(piperazin-1-yl)pyridazine-3- carboxylate (BB28)

[Formula 72]

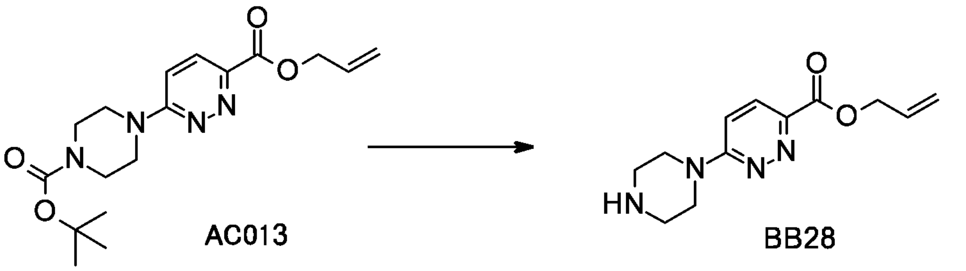

AC013 BB28

Prop-2-enyl 6-[4-[(2-methylpropan-2-yl)oxycarbonyl]piperazin-1-yl]pyridazine-3- carboxylate (AC013, 1.23 g, 3.53 mmol) and TFE (15.4 mL) were added to a 50 mL eggplant flask. Under a nitrogen atmosphere, trimethylsilyl chloride (2.26 mL, 17.7 mmol, 5.0 equivalents) was added thereto at room temperature, and the mixture was stirred at room temperature for 30 minutes. The reaction solution was concentrated under reduced pressure. Then, DIPEA (3.39 mL) was added to the obtained residue, and the mixture was purified by NH-silica gel column chromatography (DCM/methanol, 0-10%) to obtain the title compound (BB28, 91%, 0.80 g, 3.22 mmol).

LCMS: m/z 249.2[M+H]$^+$.

Retention time: 0.39 and 0.46 min (for a bimodal peak shape) (analysis conditions SMD-FA05-1).

$^1$H-NMR (400 MHz, $CDCl_3$) $\delta$: 7.88 (d, J = 9.7 Hz, 1H), 6.84 (d, J = 9.7 Hz, 1H), 6.12-6.02 (m, 1H), 5.44 (dd, J = 1.6, 17.2 Hz, 1H), 5.30 (dd, J = 1.6, 10.7 Hz, 1H), 4.90 (m, 2H), 3.79-3.76 (m, 4H), 3.02-2.99 (m, 4H).

Example 1-4-5: Method for synthesizing tert-butyl 6-piperazin-1-ylpyridazine-3- carboxylate (BB29)

[Formula 73]

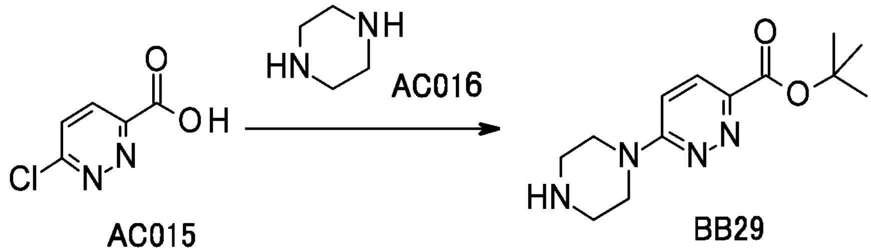


AC015 BB29

tert-Butyl 6-chloropyridazine-3-carboxylate (AC015, CAS: 1340506-55-9, 3.00 g, 18.9 mmol), DMAP (116 mg, 0.946 mmol, 0.05 equivalents), acetonitrile (23.7 mL), and DIPEA (9.91 mL, 56.8 mmol, 3.0 equivalents) were added to a 50 mL eggplant flask. BoczO (6.19 g, 28.4 mmol, 1.5 equivalents) was added thereto, and the mixture was heated to reflux at 80°C for 1 hour under a nitrogen atmosphere. The reaction solution was cooled to room temperature. Then, piperazine (AC016, CAS: 110-85-0, 9.46 mmol, 1.5 equivalents) was added thereto, and the mixture was heated to reflux at 80°C for 1 hour under a nitrogen atmosphere. The reaction solution was cooled to room temperature. Formic acid was added thereto, and the mixture was purified by reverse-phase column chromatography (0.1% solution of formic acid in acetonitrile/0.1% aqueous formic acid solution, 5-20%). After concentration, the obtained residue was purified by NH-silica gel column chromatography (ethyl acetate/hexane, 0-20%, and then DCM/methanol, 0-10%) to obtain the title compound (BB29, 43%, 2.17 g, 8.21 mmol).

LCMS: m/z 265.2[M+H]$^+$.

Retention time: 0.53 min (analysis conditions SMD-FA05-1).

$^1$H-NMR (400 MHz, $CDCl_3$) $\delta$: 7.83 (d, J = 9.7 Hz, 1H), 6.83 (d, J = 9.7 Hz, 1H), 3.77-3.75 (m, 4H), 3.01-2.99 (m, 4H).

Example 1-4-6: Method for synthesizing propan-2-yl 6-piperazin-1-ylpyridazine-3- carboxylate (AC019)

[Formula 74]

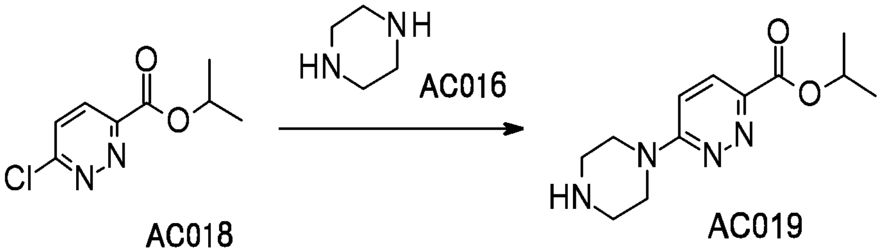


AC018

AC019

Propan-2-yl 6-chloropyridazine-3-carboxylate (AC018, CAS: 321946-09-2, 100 mg, 0.498 mmol), piperazine (AC016, 129 mg, 1.50 mmol, 3.0 equivalents), DMAP (6.1 mg, 0.05 mmol, 0.1 equivalents), acetonitrile (2.49 mL), and DIPEA (131 μL, 0.748 mmol, 1.5 equivalents) were added to a 20 mL eggplant flask and heated and stirred at 70°C for 1.5 hours under a nitrogen atmosphere. The reaction solution was cooled to room temperature. Then, the reaction solution was diluted with AcOEt (30 mL) and washed with a saturated aqueous solution of ammonium chloride (10 mL), water (10 mL), and saturated sodium chloride aqueous solution (10 mL). The organic layer was filtered through a phase separator, and the filtrate was concentrated under reduced pressure. The obtained residue was purified by silica gel column chromatography (NH-silica gel) (ethyl acetate/hexane, 0-20%, and then DCM/methanol, 0-10%) to obtain the title compound (AC019, 52%, 65 mg, 0.260 mmol).

LCMS: m/z 251.2[M+H]$^+$.

Retention time: 0.46 and 0.48 min (for a bimodal peak shape) (analysis conditions SMD-FA05-1).

$^1$H-NMR (400 MHz, $CDCl_3$) δ: 7.87 (d, J = 9.7 Hz, 1H), 6.84 (d, J = 9.7 Hz, 1H), 5.37-5.28 (m, 1H), 3.78-3.75 (m, 4H), 3.01-2.99 (m, 4H), 1.41 (d, J = 6.4 Hz, 6H).

Example 1-4-7: Synthesis of solid-phase supported aromatic aldehyde (AC003-01R)

[Formula 75]

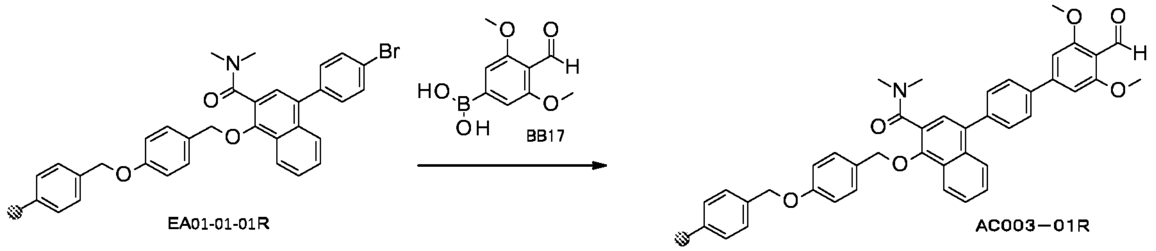


EA01-01-01R

AC003−01R

Under a nitrogen atmosphere, compound EA01-01-01R (20 mg, loading rate: 0.29 mmol/g) and NMP (350 μL) were added to a 0.6 mL glass vial and shaken at room temperature for 1 hour. (2-Fluoro-4-formylphenyl)boronic acid (BB17) (12.2 mg, 58.0 μmol, 6.0 equivalents), an aqueous $K_3PO_4$ solution (1.5 M, 23.2 μL, 35.0 μmol, 6.0 equivalents), and a solution of $P(Cy)_3Pd$ G3 in NMP (0.04 M, 29.0 μL, 1.16 μmol, 20 mol%) were added thereto, and the mixture was shaken at 90°C for 14 hours. The suspension of the reaction solution and the resin was transferred to a syringe with a filter using NMP and washed three times with NMP (400 μL), three times with an NMP:$H_2O$ (1:1) solution (400 μL) containing NAC (10 mg/mL), three times with water (400 μL), three times with methanol (400 μL), and three times with DCM (400 μL). Then, the resin was dried under reduced pressure to obtain the title compound AC003-01R.

Reaction monitoring

After a lapse of a given time, under a nitrogen atmosphere, 10 μL of the suspension of the reaction solution and the resin was sampled, transferred to a pipette tip with a filter, washed three times with NMP (100 μL), three times with methanol (100 μL), and three times with DCM (100 μL), and then dipped in a 10% solution of TFA in DCM containing

0.1 M trimethylbenzene (50 μL) for 5 minutes. After filtration, the filtrate was dissolved in TFE (1000 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

Compound to be analyzed: 4-[4-(4-formyl-3,5-dimethoxyphenyl)phenyl]-1- hydroxy-N,N-dimethylnaphthalene-2-carboxamide (AC003)

[Formula 76]

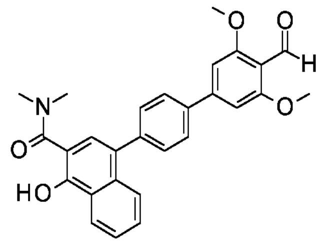

LRMS: m/z 456.1[M+H]+.
Retention time: 1.30 min.

Example 1-4-8: Synthesis of solid-phase supported aromatic aldehyde (AC004-01R)

[Formula 77]

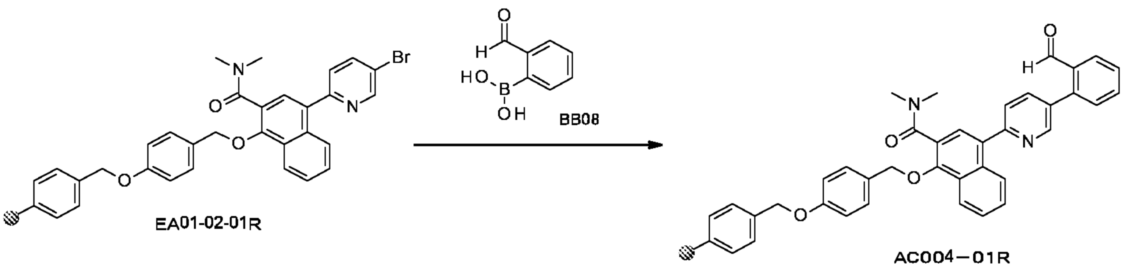


EA01-02-01R

AC004−01R

Under a nitrogen atmosphere, compound EA01-02-01R (20 mg, loading rate: 0.37 mmol/g) and 1,4-dioxane (200 μL) were added to a 0.6 mL glass vial and shaken at room temperature for 1 hour. (2-Formylphenyl)boronic acid (BB08) (6.7 mg, 44 μmol, 6.0 equivalents), $K_3PO_4H_2O$ (10.2 mg, 44 μmol, 6.0 equivalents), pinacol (5.3 mg, 44 μmol, 6.0 equivalents), and a solution of $P(Cy)_3Pd$ G3 in 1,4-dioxane (0.04 M, 37 μL, 1.5 μmol, 20 mol%) were added thereto, and the mixture was shaken overnight at 90°C. The suspension of the reaction solution and the resin was transferred to a syringe with a filter using NMP and washed three times with NMP (400 μL), three times with an NMP:$H_2O$ (1:1) solution (400 μL) containing NAC (10 mg/mL), three times with water (400 μL), three times with methanol (400 μL), and three times with DCM (400 μL). Then, the resin was dried under reduced pressure to obtain the title compound AC004-01R.

Reaction monitoring

After a lapse of a given time, under a nitrogen atmosphere, 10 μL of the suspension of the reaction solution and the resin was sampled, transferred to a pipette tip with a filter, washed three times with NMP (100 μL), three times with methanol (100 μL), and three times with DCM (100 μL), and then dipped in a 10% solution of TFA in DCM containing 0.1 M trimethylbenzene (50 μL) for 5 minutes. After filtration, the filtrate was dissolved in DMI (250 μL). The solution was (50 μL) was diluted with acetonitrile (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

Compound to be analyzed: 4-[5-(2-formylphenyl)pyridin-2-yl]-1-hydroxy-N,N- dimethylnaphthalene-2-carboxamide (AC004)

[Formula 78]

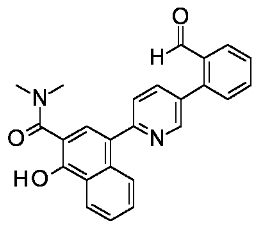

MS: m/z 397.1[M+H]+.

Retention time: 1.14 min.

Example 1-4-9: Synthesis of solid-phase supported aromatic aldehyde (AC006-01R)

[Formula 79]

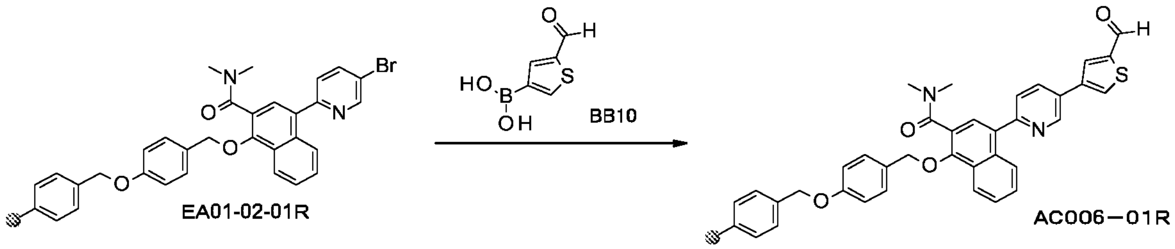


EA01-02-01R

AC006−01R

Under a nitrogen atmosphere, compound EA01-02-01R (20 mg, loading rate: 0.37 mmol/g) and 1,4-dioxane (200 μL) were added to a 0.6 mL glass vial and shaken at room temperature for 1 hour. (5-Formylthiophen-3-yl)boronic acid (BB10) (6.7 mg, 44 μmol, 6.0 equivalents), $K_3PO_4 \cdot H_2O$ (10.2 mg, 44 μmol, 6.0 equivalents), pinacol (5.3 mg, 44 μmol, 6.0 equivalents), and a solution of $P(Cy)_3Pd$ G3 in 1,4-dioxane (0.04 M, 37 μL, 1.5 μmol, 20 mol%) were added thereto, and the mixture was shaken overnight at 90°C. The suspension of the reaction solution and the resin was transferred to a syringe with a filter using NMP and washed three times with NMP (400 μL), three times with an NMP:$H_2O$ (1:1) solution (400 μL) containing NAC (10 mg/mL), three times with water (400 μL), three times with methanol (400 μL), and three times with DCM (400 μL). Then, the resin was dried under reduced pressure to obtain the title compound AC004-01R.

Reaction monitoring

After a lapse of a given time, under a nitrogen atmosphere, 10 μL of the suspension of the reaction solution and the resin was sampled, transferred to a pipette tip with a filter, washed three times with NMP (100 μL), three times with methanol (100 μL), and three times with DCM (100 μL), and then dipped in a 10% solution of TFA in DCM containing 0.05 M trimethylbenzene (50 μL) for 5 minutes. After filtration, the filtrate was dissolved in DMI (250 μL), and the solution (50 μL) was diluted with acetonitrile (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

Compound to be analyzed: 4-[5-(5-formylthiophen-3-yl)pyridin-2-yl]-1-hydroxy- N,N-dimethylnaphthalene-2-carboxamide (AC006)

[Formula 80]

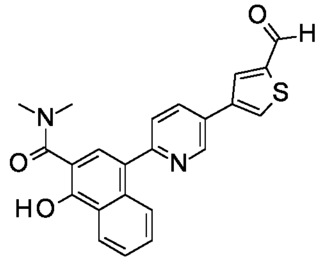

MS: m/z 403.1[M+H]+.

Retention time: 1.10 min.

Example 1-4-10: Synthesis of solid-phase supported aromatic aldehyde (AC008- 01R)

[Formula 81]

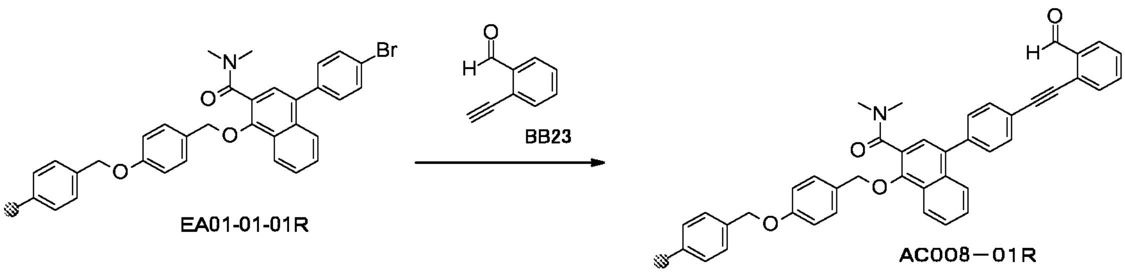


EA01-01-01R

AC008−01R

Under a nitrogen atmosphere, compound EA01-01-01R (0.40 mmol/g, 50 mg, 0.020 mmol), NMP (438 μL), and DIA (63 μL) were added to a 1.5 mL screw-cap vial and shaken at room temperature for 1 hour. Methyl 2-ethynylbenzoate (BB23) (15.6 mg, 0.12 mmol, 6.0 equivalents) and $Pd(PPh_3)_4$ (11.6 mg, 0.010 mmol, 50 mol%) were added thereto, and the mixture was shaken at 80°C for 22 hours. The reaction solution was cooled to room temperature, and the suspension of the reaction solution and the resin was transferred to a syringe with a filter using NMP and washed three times with an NMP:HzO (1:1) solution (1000 μL) containing NAC (10 mg/mL), three times with water (1000 μL), three times with methanol (1000 μL), and three times with DCM (1000 μL). Then, the resin was dried under reduced pressure to obtain the title compound AC008-01R.

Purity confirmation

A portion of the resin after drying was transferred to a pipette tip with a filter, washed three times with DCM (100 μL), and then dipped in a 10% solution of TFA in DCM containing 0.05 M trimethylbenzene (50 μL) for 5 minutes. After filtration, the filtrate was dissolved in NMP (250 μL), and the solution (50 μL) was diluted with acetonitrile (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

Compound to be analyzed: 4-[4-[2-(2-formylphenyl)ethynyl]phenyl]-1-hydroxy- N,N-dimethylnaphthalene-2-carboxamide (AC008)

[Formula 82]

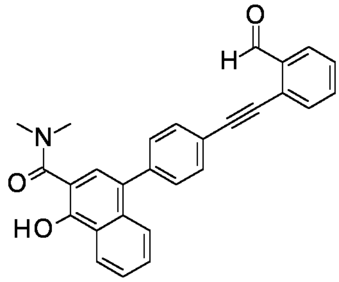

MS: m/z 420.1[M+H]+.

Retention time: 1.50 min.

Example 1-4-11: Synthesis of solid-phase supported aromatic aldehyde (AC009- 01R)

[Formula 83]

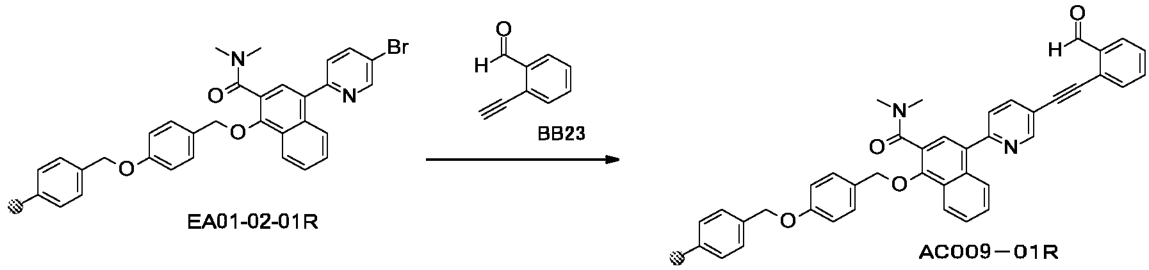


EA01-02-01R

AC009−01R

Under a nitrogen atmosphere, compound EA01-02-01R (0.37 mmol/g, 50 mg, 0.019 mmol), NMP (438 μL), and DIA (63 μL) were added to a 1.5 mL screw-cap vial and shaken at room temperature for 1 hour. Methyl 2-ethynylbenzoate (BB23) (14.4 mg, 0.11 mmol, 6.0 equivalents) and $Pd(PPh_3)_4$ (10.7 mg, 0.009 mmol, 50 mol%) were added thereto, and the mixture was shaken at 80°C for 22 hours. The reaction solution was cooled to room temperature, and the suspension of the reaction solution and the resin was transferred to a syringe with a filter using NMP and washed three times with an NMP:$H_2O$ (1:1) solution (1000 μL) containing NAC (10 mg/mL), three times with water (1000 μL), three times with methanol (1000 μL), and three times with DCM (1000 μL). Then, the resin was dried under reduced pressure to obtain the title compound AC009-01R.

Purity confirmation

A portion of the resin after drying was transferred to a pipette tip with a filter, washed three times with DCM (100 μL), and then dipped in a 10% solution of TFA in DCM containing 0.05 M trimethylbenzene (50 μL) for 5 minutes. After filtration, the filtrate was dissolved in NMP (250 μL), and the solution (50 μL) was diluted with acetonitrile (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

Compound to be analyzed: 4-[5-[2-(2-formylphenyl)ethynyl]pyridin-2-yl]-1- hydroxy-N,N-dimethylnaphthalene-2-carboxamide (AC009)

[Formula 84]

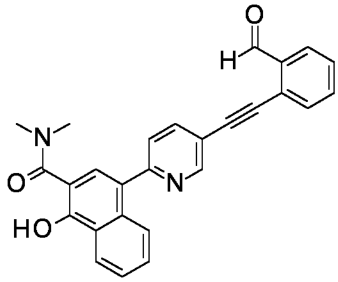

MS: m/z 421.1[M+H]+.

Retention time: 1.30 min.

Example 1-4-12: Synthesis of solid-phase supported $ArCOCH_3$ (B013-01R)

[Formula 85]

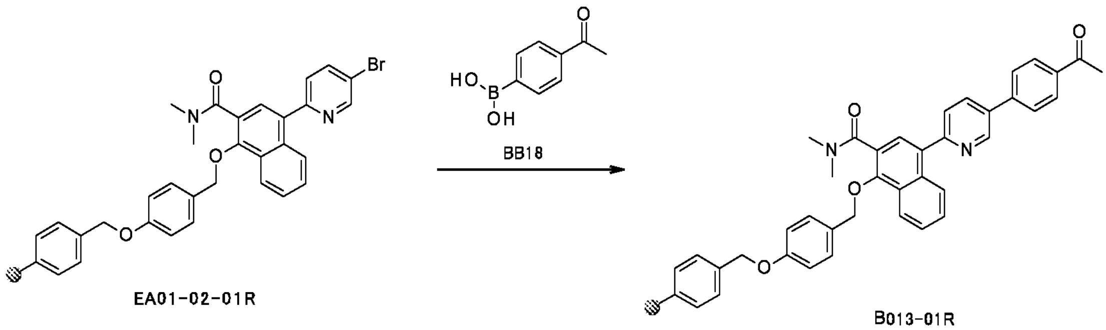


Under a nitrogen atmosphere, compound EA01-02-01R (338 mg, loading rate: 0.37 mmol/g, 0.125 mmol) and NMP (5.68 mL) were added to a 10 mL glass vial and shaken at room temperature for 1 hour. (4-Acetylphenyl)boronic acid (BB18) (205 mg, 1.250 mmol, 10 equivalents), $K_3PO_4$ (159 mg, 0.750 mmol, 6 equivalents), $P(Cy)_3Pd$ G3 (16 mg, 0.025 mmol, 0.2 equivalents), and degassed water (568 μL) were added thereto, and the mixture was shaken at 90°C for 20 hours. The reaction container was cooled to room temperature. Then, the suspension of the reaction solution and the resin was transferred to a syringe with a filter using NMP, washed three times with NMP (7 mL), three times with an NMP:HzO (1:1) solution (7 mL) containing NAC (10 mg/mL), three times with water (7 mL), three times with methanol (7 mL), and three times with DCM (7 mL), and then dried under reduced pressure to obtain compound (B013-01R).

Compound B013-01R was sampled, transferred to a pipette tip with a filter, washed three times with NMP (100 μL), three times with methanol (100 μL), and three times with DCM (100 μL), and then dipped in a 10% solution of TFA in DCM containing 0.05 M trimethylbenzene (50 μL) for 5 minutes. After filtration, the filtrate was dissolved in TFE-acetonitrile (200-800 μL) to prepare an LC sample. The purity was measured by LC-MS analysis.

[Formula 86]

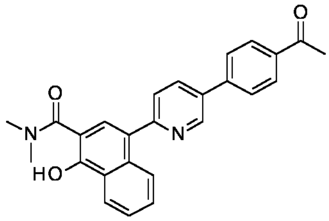

4-[5-(4-Acetylphenyl)pyridin-2-yl]-1-hydroxy-N,N-dimethylnaphthalene-2- carboxamide: compound B013

LCMS: m/z 411.1[M+H]+.
Retention time: 1.164 min (analysis conditions SMD-FA05-1, 319 nm).

Example 1-4-13: Synthesis of solid-phase supported $ArCOCH_3$ (B021-01R)

[Formula 87]

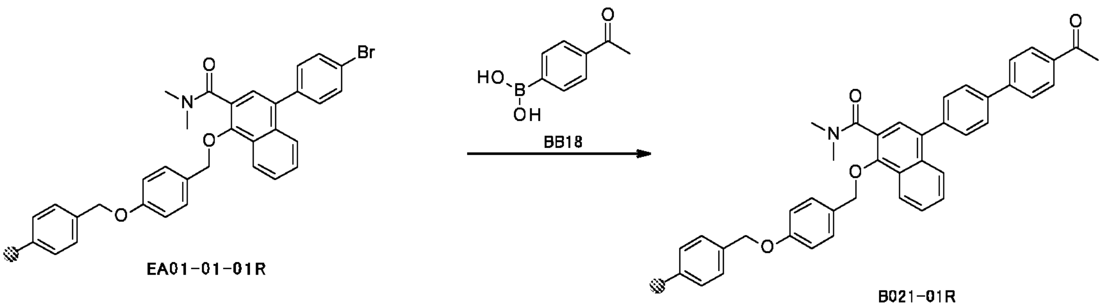


EA01-01-01R

B021-01R

Under a nitrogen atmosphere, compound EA01-01-01R (357 mg, loading rate: 0.35 mmol/g, 0.125 mmol) and NMP (5.68 mL) were added to a 10 mL glass vial and shaken at room temperature for 1 hour. (4-Acetylphenyl)boronic acid (BB18) (205 mg, 1.250 mmol, 10 equivalents), $K_3PO_4$ (159 mg, 0.750 mmol, 6 equivalents), $P(Cy)_3$Pd G3 (16 mg, 0.025 mmol, 0.2 equivalents), and degassed water (568 μL) were added thereto, and the mixture was shaken at 90°C for 7 hours. The reaction container was cooled to room temperature. Then, the suspension of the reaction solution and the resin was transferred to a syringe with a filter using NMP, washed three times with NMP (7 mL), three times with an NMP:HzO (1:1) solution (7 mL) containing NAC (10 mg/mL), three times with water (7 mL), three times with methanol (7 mL), and three times with DCM (7 mL), and then dried under reduced pressure to obtain compound (B021-01R).
Compound B021-01R was sampled, transferred to a pipette tip with a filter, washed three times with NMP (100 μL), three times with methanol (100 μL), and three times with DCM (100 μL), and then dipped in a 10% solution of TFA in DCM containing 0.05 M trimethylbenzene (50 μL) for 5 minutes. After filtration, the filtrate was dissolved in TFE-acetonitrile (200-800 μL) to prepare an LC sample. The purity was measured by LC-MS analysis.

[Formula 88]

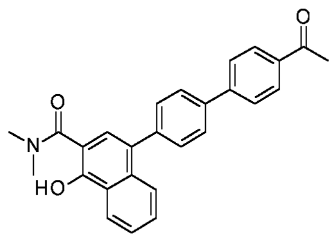

4-[4-(4-Acetylphenyl)phenyl]-1-hydroxy-N,N-dimethylnaphthalene-2-carboxamide: compound B021

LCMS: m/z 410.2[M+H]+.
Retention time: 1.368 min (analysis conditions SMD-FA05-1, 319 nm).

Example 1-4-14: Synthesis of solid-phase supported $ArCOCH_3$ (B025-01R)

[Formula 89]

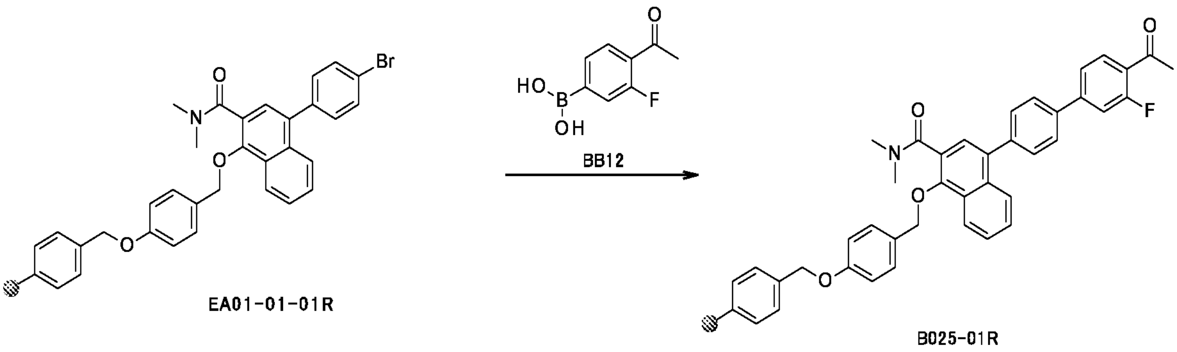


Under a nitrogen atmosphere, compound EA01-01-01R (125 mg, loading rate: 0.35 mmol/g, 0.044 mmol) and NMP (1.99 mL) were added to a 10 mL glass vial and shaken at room temperature for 1 hour. (4-Acetyl-3-fluorophenyl)boronic acid (BB12) (80 mg, 0.438 mmol, 10 equivalents), $K_3PO_4$ (5.69 mg, 8.75 mmol, 6 equivalents), $P(Cy)_3Pd$ G3 (5.69 mg, 8.75 μmol, 0.2 equivalents), and degassed water (199 μL) were added thereto, and the mixture was shaken at 90°C for 15 hours. The reaction container was cooled to room temperature. Then, the suspension of the reaction solution and the resin was transferred to a syringe with a filter using NMP and washed three times with NMP (2.5 mL), three times with an NMP:HzO (1:1) solution (2.5 mL) containing NAC (10 mg/mL), three times with water (2.5 mL), three times with methanol (2.5 mL), and three times with DCM (2.5 mL), and then dried under reduced pressure to obtain compound (B025-01R).

Compound B025-01R was sampled, transferred to a pipette tip with a filter, washed three times with NMP (100 μL), three times with methanol (100 μL), and three times with DCM (100 μL), and then dipped in a 10% solution of TFA in DCM containing 0.05 M trimethylbenzene (100 μL) for 5 minutes. After filtration, the filtrate was dissolved in TFE (200 μL) to prepare an LC sample. The purity was measured by LC-MS analysis.

[Formula 90]

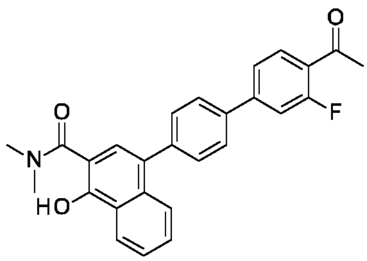

4-[4-(4-Acetyl-3-fluorophenyl)phenyl]-1-hydroxy-N,N-dimethylnaphthalene-2- carboxamide: compound B025

LCMS: m/z 428.1[M+H]$^+$.

Retention time: 1.461 min (analysis conditions SMD-FA05-1, 319 nm).

Example 1-5: Synthesis of substrate for reaction study for use mainly in Examples 2-5 and 2-7, and building block for use in mixture library, etc.

Example 1-5-1: Synthesis of compound AE025-01R

[Formula 91]

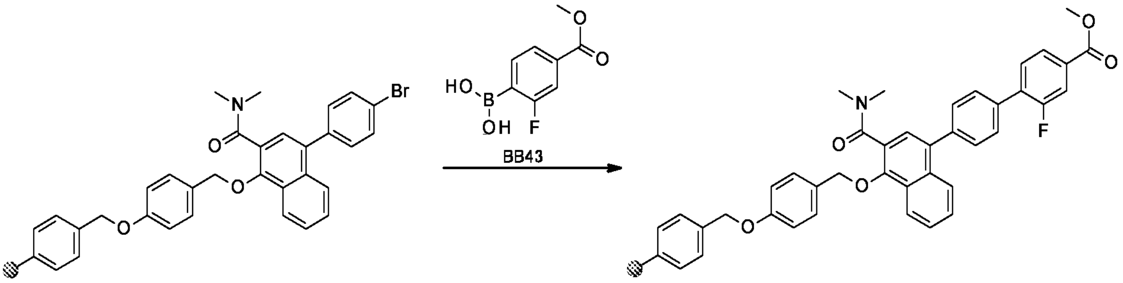


EA01-01-01R AE025-01R

Under a nitrogen atmosphere, compound EA01-01-01R (loading rate: 0.400 mmol/g, 1.00 g, 0.400 mmol) and NMP (13 mL) were added to a 30 mL glass vial and shaken at room temperature for 1 hour. (2-Fluoro-4-formylphenyl)boronic acid (BB43) (0.475 g, 2.40 mmol), potassium phosphate trihydrate (0.509 g, 2.40 mmol), $P(Cy)_3Pd$ G3 (0.106 g, 0.151 mmol), and water (1.3 mL) were added thereto, and the mixture was shaken at 90°C for 21 hours.

The suspension of the reaction solution and the resin was wholly transferred onto a filter and washed three times with NMP (20 mL), three times with NAC (10 mg/mL, NMP/water = 1/1 solution, 20 mL), three times with water (20 mL), three times with methanol (20 mL), three times with DCM (20 mL), and three times with heptane (20 mL), and the obtained solid phase was dried under reduced pressure for 1 hour to obtain compound AE025-01R (1.102 g).

A portion of the obtained solid phase was dipped in a 10% solution of TFA in DCM containing 0.1 M trimethylbenzene. A portion of the solution was subjected to LC/MS analysis, so that AE025 and its hydrolysate AE026 were observed.

[Formula 92]

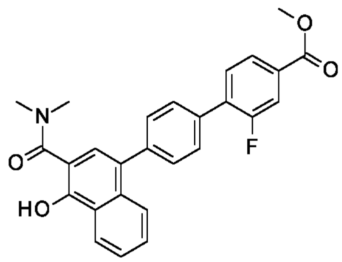

Compound AE025

LRMS: m/z 444$[M+H]^+$.

Retention time: 1.493 min (analysis conditions SMD-FA05-1, 319 nm).

Example 1-5-2: Synthesis of compound AE026-01R

[Formula 93]

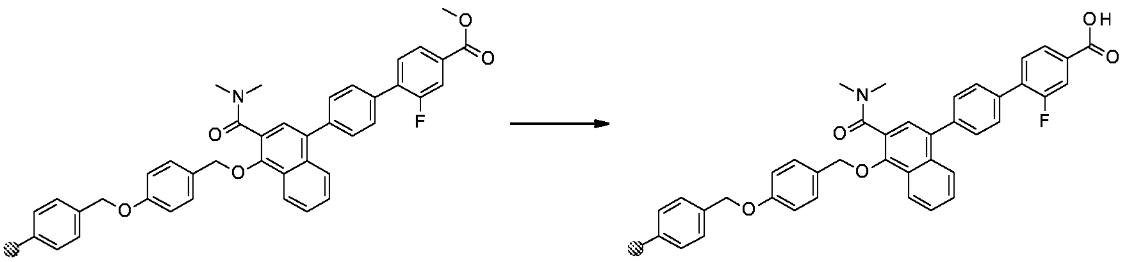

AE025-01R AE026-01R

Compound AE025-01R (1.102 g), NMP (16 mL), and methanol (4 mL) were added to a 30 mL glass vial and shaken at room temperature for 1 hour. A 5 M aqueous sodium hydroxide solution (0.6 mL, 3.00 mmol) was added

thereto, and the mixture was shaken at room temperature for 4 hours.

The suspension of the reaction solution and the resin was wholly transferred onto a filter and washed three times with NAC (10 mg/mL, NMP/water = 1/1 solution, 22 mL), three times with malonic acid (0.05 M, a solution in NMP, 22 mL), three times with HOAt (0.2 M, a solution in NMP, 22 mL), three times with NMP (22 mL), three times with methanol (22 mL), three times with DCM (22 mL), and three times with heptane (22 mL), and the obtained solid phase was dried under reduced pressure for 1 hour to obtain compound AE026-01R (1.001 g).

A portion of the obtained solid phase was dipped in a 10% solution of TFA in DCM containing 0.1 M trimethylbenzene. A portion of the solution was subjected to LC/MS analysis, so that AE026 was observed.

[Formula 94]

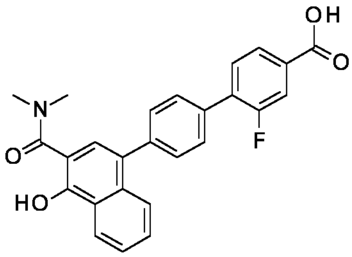

Compound AE026

LRMS: m/z 430[M+H]+

Retention time: 1.224 min (analysis conditions SMD-FA05-1, 319 nm)

Example 1-5-3: Synthesis of compound AE028-01R

[Formula 95]

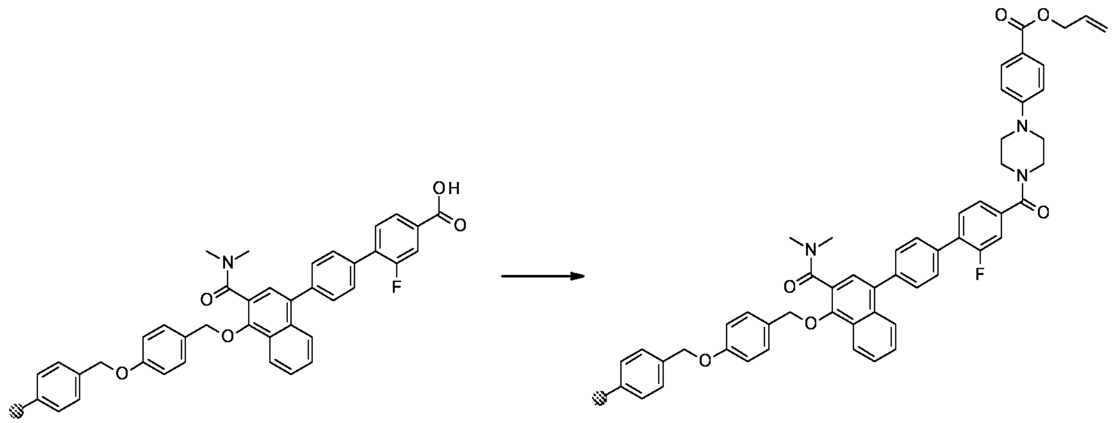

AE026-01R AE028-01R

Compound AE026-01R (1.00 g) and NMP (17 mL) were added to a 30 mL glass vial and shaken at room temperature for 1 hour. Allyl 4-(piperazin-1-yl)benzoate (BB27) (0.296 g, 1.20 mmol), DIC (0.303 g, 2.40 mmol), and HOAt (0.327 g, 2.40 mmol) were added thereto, and the mixture was shaken overnight at room temperature.

The suspension of the reaction solution and the resin was wholly transferred onto a filter and washed three times with NMP (20 mL), three times with methanol (20 mL), three times with DCM (20 mL), and three times with heptane (20 mL), and the obtained solid phase was dried under reduced pressure for 1 hour to obtain compound AE028-01R (1.124 g).

A portion of the obtained solid phase was dipped in a 10% solution of TFA in DCM containing 0.1 M trimethylbenzene. A portion of the solution was subjected to LC/MS analysis, so that AE028 was observed.

[Formula 96]

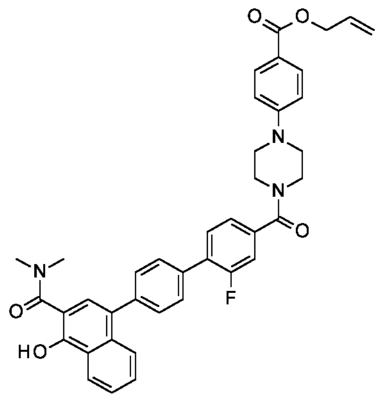

Compound AE028

LRMS: m/z 658[M+H]+.

Retention time: 1.492 min (analysis conditions SMD-FA05-1, 319 nm).

Example 1-5-4: Synthesis of compound AE029-01R

[Formula 97]

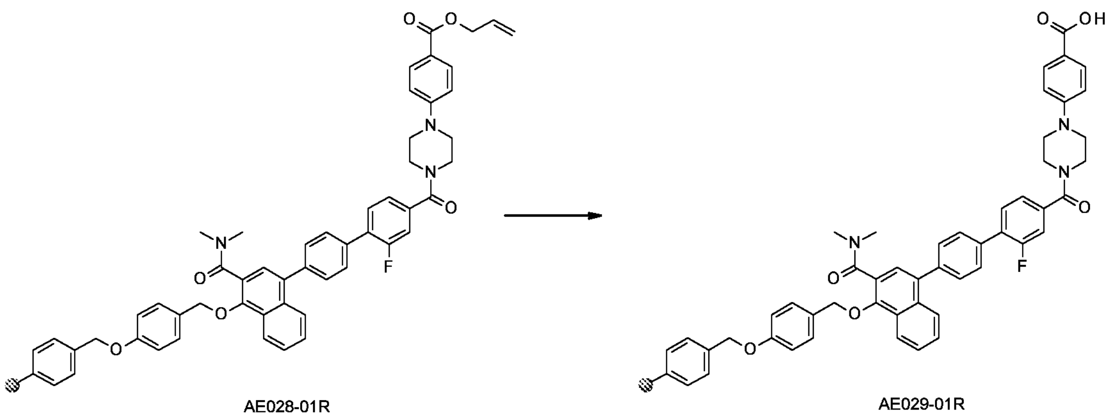

AE028-01R AE029-01R

Under a nitrogen atmosphere, compound AE028-01R (1.124 g) and THF (22 mL) were added to a 30 mL glass vial and shaken at room temperature for 1 hour. $Pd(PPh_3)_4$ (0.156 g, 0.135 mmol) and morpholine (0.194 mL, 2.25 mmol) were added thereto, and the mixture was shaken at room temperature for 2 hours.

The suspension of the reaction solution and the resin was wholly transferred onto a filter and washed three times with NAC (0.05 M, NMP/water = 1/1 solution, 22 mL), three times with malonic acid (0.05 M, a solution in NMP, 22 mL), three times with HOAt (0.2 M, a solution in NMP, 22 mL), three times with water (22 mL), three times with methanol (22 mL), three times with DCM (22 mL), and three times with heptane (22 mL), and the obtained solid phase was dried under reduced pressure for 1 hour to obtain compound AE029-01R (1.054 g).

A portion of the obtained solid phase was dipped in a 10% solution of TFA in DCM containing 0.1 M trimethylbenzene. A portion of the solution was subjected to LC/MS analysis, so that AE029 was observed.

[Formula 98]

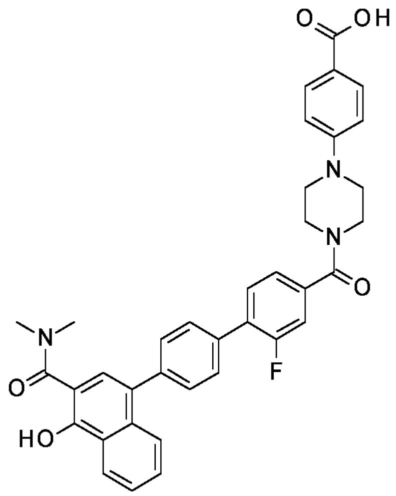

Compound AE029

LRMS: m/z 618[M+H]+.

Retention time: 1.216 min (analysis conditions SMD-FA05-1, 319 nm).

AE029-01R whose synthesis method is illustrated in Example 1-5-4 is also referred to as compound BAD006-01R in the subsequent examples.

Example 1-6: Synthesis of substrate for reaction study for use mainly in Example 2- 8, and building block for use in mixture library, etc.

Example 1-6-1: Synthesis of 2-bromo-4-(3,3,4,4,5,5,6,6,7,7,8,8,9,9,10,10,10- heptadecafluorodecylsulfanyl)benzaldehyde (compound AE003)

[Formula 99]

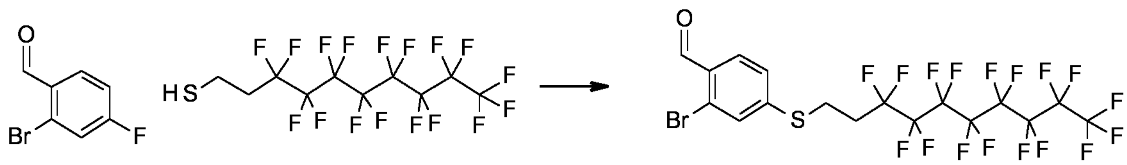

AE001 AE002 AE003

To a solution of 2-bromo-4-fluorobenzaldehyde (AE001) (3.00 g, 14.8 mmol) in THF (29.6 mL) placed in a 200 mL flask, cesium carbonate (5.78 g, 17.7 mmol) was added. The container was purged with nitrogen and then cooled to 0°C. 3,3,4,4,5,5,6,6,7,7,8,8,9,9,10,10,10-Heptadecafluorodecane-1-thiol (AE002) (7.10 g, 14.8 mmol) was added dropwise thereto, and the mixture was stirred at room temperature for 3 hours. The mixture was filtered, and the filtrate was purified by reverse-phase column chromatography (C18, 0.1% solution of formic acid in acetonitrile/0.1% aqueous formic acid solution) to obtain the title compound AE003 (6.08 g, 9.17 mmol, 62%) as a white solid.

Maximum wavelength: 309 nm.

Retention time: 1.156 min (analysis conditions SMD-FA50-2).

Example 1-6-2: Synthesis of 2-(4-chlorophenyl)-4- (3,3,4,4,5,5,6,6,7,7,8,8,9,9, 10, 10, 1 0-heptadecafluorodecylsulfanyl)benzaldehyde (compound AE005)

[Formula 100]

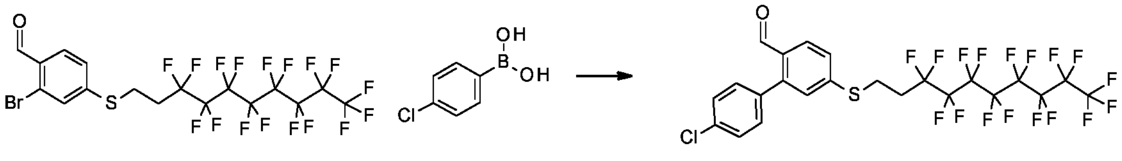

AE003 AE004 AE005

2-Bromo-4-(3,3,4,4,5,5,6,6,7,7,8,8,9,9,10,10,10- heptadecafluorodecylsulfanyl)benzaldehyde (AE003) (1.00 g, 1.51 mmol), (4- chlorophenyl)boronic acid (AE004) (0.259 g, 1.66 mmol), cesium carbonate (1.23 g, 3.77 mmol), $(PPh_3)PdCl_2$ (0.106 g, 0.151 mmol), dioxane (12.92 mL), and water (2.154 mL) were added to a 100 mL flask. The container was purged with nitrogen and then stirred at 80°C for 1.5 hours. The mixture was diluted with ethyl acetate, and water was added thereto. An organic layer was extracted and concentrated. Then, the obtained residue was purified by solid-phase extraction (FluoroFlash(R), an 80% aqueous methanol solution and subsequently methanol). The obtained crude product was purified by reverse-phase column chromatography (C18, 0.1% solution of formic acid in acetonitrile/0.1% aqueous formic acid solution) to obtain the title compound AE005 (0.88 g, 1.3 mmol, 84%) as a white solid.

Maximum wavelength: 308 nm.

Retention time: 1.229 min (analysis conditions SMD-FA50-2).

Example 1-6-3: Synthesis of ethyl 4-[4-[[2-(4-chlorophenyl)-4- (3,3,4,4,5,5,6,6,7,7,8,8,9,9,10,10,10-heptadecafluorodecylsulfanyl)phenyl]methyl]piperazin- 1-yl]benzoate (compound AE007)

[Formula 101]

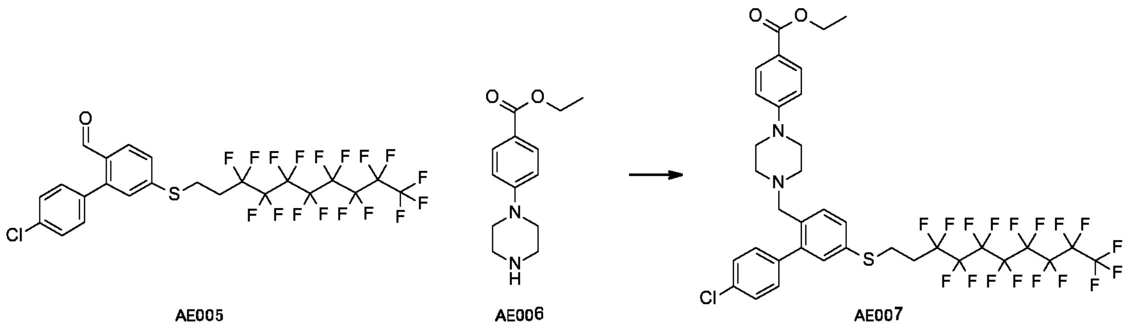

AE005 AE006 AE007

To a solution of 2-(4-chlorophenyl)-4-(3,3,4,4,5,5,6,6,7,7,8,8,9,9,10,10,10- heptadecafluorodecylsulfanyl)benzaldehyde (AE005) (400 mg, 0.576 mmol) in THF (5.76 mL), ethyl 4-piperazin-1-ylbenzoate (AE006) (175 mg, 0.748 mmol) and acetic acid (83.0 mg, 1.38 mmol) were added thereto, and the mixture was stirred at room temperature for 15 minutes. Sodium triacetoxyborohydride (244 mg, 1.15 mmol) was added thereto, and the mixture was stirred at room temperature for 2 hours. The mixture was diluted with dichloromethane. A 1 M aqueous sodium hydroxide solution was added thereto, and the mixture was stirred at room temperature for 10 minutes. The aqueous layer was removed through a phase separator, and the filtrate was concentrated. Then, the obtained residue was purified by reverse-phase column chromatography (C18, 0.1% solution of formic acid in acetonitrile/0.1% aqueous formic acid solution, 70-100%) to obtain the title compound AE007 (189 mg, 0.207 mmol, 36%) as a white solid.

LRMS: m/z 913[M+H]+.

Retention time: 1.065 min (analysis conditions SMD-FA50-2).

Example 1-6-4: Synthesis of 4-[4-[[2-(4-chlorophenyl)-4- (3,3,4,4,5,5,6,6,7,7,8,8,9,9,10,10,10-heptadecafluorodecylsulfanyl)phenyl]methyl]piperazin- 1-yl]benzoic acid dihydrochloride (compound AE008)

[Formula 102]

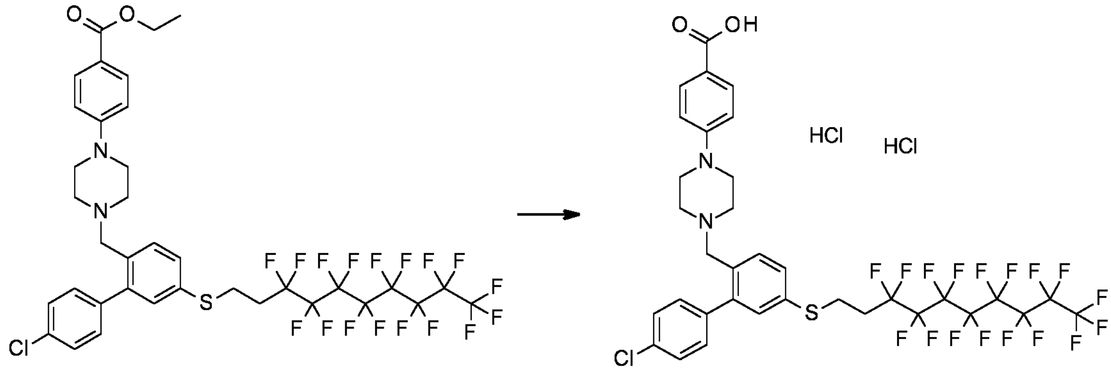

AE007 AE008

To a solution of ethyl 4-[4-[[2-(4-chlorophenyl)-4- (3,3,4,4,5,5,6,6,7,7,8,8,9,9,10,10,10-heptadecafluorodecylsulfanyl)phenyl]methyl]piperazin- 1-yl]benzoate (AE007) (185 mg, 0.203 mmol) in THF (1.8 mL)/methanol (0.225 mL), a 5 M aqueous sodium hydroxide solution (0.405 mL, 2.03 mmol) was added, and the mixture was stirred at room temperature for 1.5 hours and subsequently stirred at 60°C for 3 hours. A 5 M aqueous sodium hydroxide solution (0.405 mL, 2.03 mmol) was added thereto, and the mixture was stirred overnight at 60°C. THF (1.8 mL), methanol (0.225 mL), and a 5 M aqueous sodium hydroxide solution (0.405 mL, 2.03 mmol) were added thereto, and the mixture was stirred overnight at 60°C. The mixture was purified by reverse-phase column chromatography (C18, 0.1% solution of formic acid in acetonitrile/0.1% aqueous formic acid solution, 70-100%). The obtained crude product was dissolved in dichloromethane. An excessive amount of a 4 M solution of hydrogen chloride in dioxane was added to the solution, and the mixture was stirred at room temperature for 30 minutes. The resulting solid was collected by filtration to obtain the title compound AE008 (156 mg, 0.163 mmol, 80%) as a pale yellow solid.

LRMS: m/z 885[M+H]$^+$.

Retention time: 0.788 min (analysis conditions SMD-TFA50-1).

Example 1-6-5: Synthesis of aromatic aldehyde (AE010)

[Formula 103]

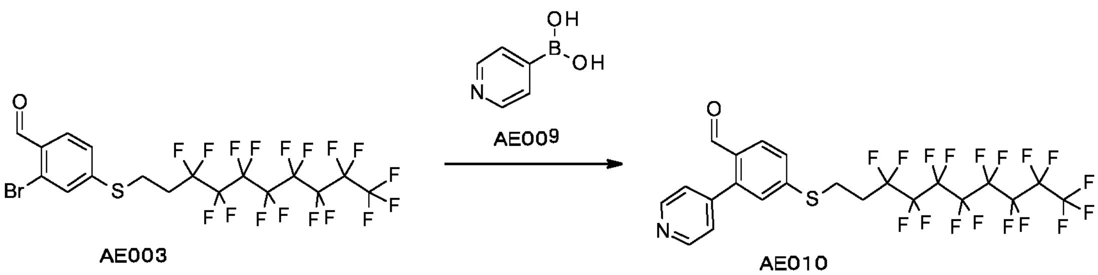

AE003 AE009 AE010

AE003 (3.00 g, 4.52 mmol), AE009 (0.612 g, 4.98 mmol), cesium carbonate (3.68 g, 11.3 mmol), $(PPh_3)PdCl_2$ (0.318 g, 0.452 mmol), dioxane (38.8 mL), and water (1.00 mL) were added to a 300 mL flask. The container was purged with nitrogen and then stirred at 80°C for 1.5 hours. The reaction mixture was cooled to room temperature, and an aqueous formic acid solution was added thereto. Then, the mixture was purified by reverse-phase column chromatography (C 18, 0.1 % solution of formic acid in acetonitrile/0.1% aqueous formic acid solution) to obtain the title compound AE010 (2.51 g, 3.8 mmol, 99%) as a white solid.

MS: m/z 661.9[M+H]$^+$.

Retention time: 0.92 min (analysis conditions SMD-FA50-2).

Example 1-6-6: Synthesis of aromatic carboxylic acid ester form (AE012)

[Formula 104]

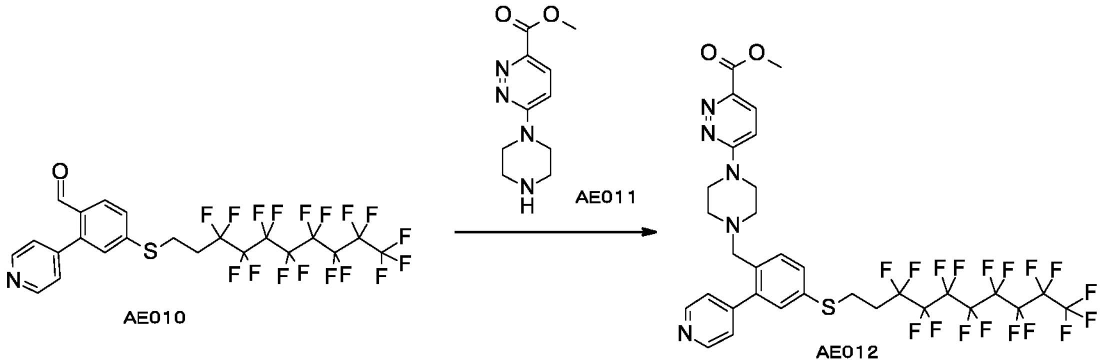


To a solution of AE010 (500 mg, 0.756 mmol) in DCM (7.56 mL), AE011 (202 mg, 0.907 mmol) and acetic acid (109 mg, 1.81 mmol) were added, and the mixture was stirred at room temperature for 10 minutes. Sodium triacetoxyborohydride (320 mg, 1.51 mmol) was added thereto, and the mixture was stirred at room temperature for 6 hours. The mixture was diluted with dichloromethane, and TFA was added thereto. Then, the mixture was purified by reverse-phase column chromatography (C18, 0.1% solution of TFA in acetonitrile/0.1% aqueous formic acid solution, 40-85%) to obtain the title compound AE012 (554 mg, 0.639 mmol, 84%).

LRMS: m/z 868.1[M+H]$^+$.

Retention time: 0.39 min (analysis conditions SMD-TFA50-1).

Example 1-6-7: Synthesis of aromatic carboxylic acid (AE013)

[Formula 105]

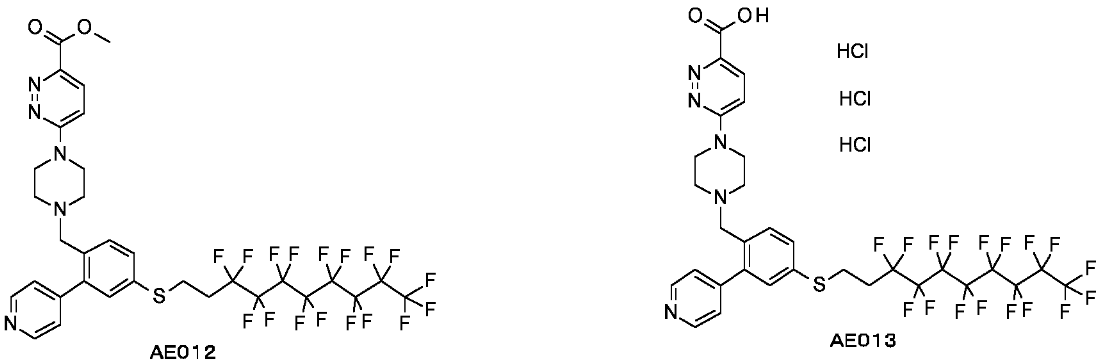


To a solution of AE012 (554 mg, 0.639 mmol) in THF (18.4 mL)/methanol (2.31 mL)/water (2.31 mL), lithium hydroxide (CAS: 1310-65-2, 153 mg, 6.39 mmol) was added, and the mixture was stirred at room temperature for 1.5 hours. TFA was added to the reaction solution, and the mixture was purified by reverse-phase column chromatography (C18, 0.1% solution of TFA in acetonitrile/0.1% aqueous TFA solution, 30-80%). The obtained crude product was dissolved in dichloromethane. An excessive amount of a 4 M solution of hydrogen chloride in dioxane was added to the solution, and the mixture was stirred at room temperature for 30 minutes. The resulting solid was collected by filtration to obtain the title compound AE013 (541 mg, 0.581 mmol, 91%).

LRMS: m/z 854.0[M+H]$^+$.

Retention time: 1.05 min (analysis conditions SMD-TFA05-1).

$^1$H-NMR (400 MHz, MeOD) $\delta$: 8.96 (d, J = 6.4 Hz, 2H), 8.24-8.10 (m, 3H), 7.97 (d, J = 8.1 Hz, 1H), 7.74 (t, J = 8.3 Hz, 2H), 7.51 (d, J = 1.6 Hz, 1H), 4.47 (s, 2H), 3.66 (s, 2H), 3.37 (m, 2H), 2.61 (m, 2H).

Example 1-6-8: Synthesis of solid-phase supported aromatic carboxylic acid (AE016-01R)

[Formula 106]

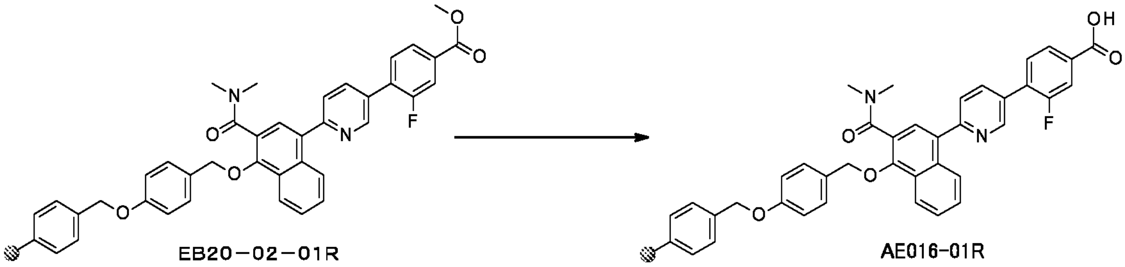

EB20-02-01R

AE016-01R

EB20-02-01R used in this example was synthesized in Example 2-2-4. Under a nitrogen atmosphere, solid-phase supported aromatic ester compound EB20-02-01R (0.37 mmol/g, 15 mg), NMP (6.08 mL, 16 v/w), and methanol (1.52 mL, 4 v/w) were added to a 0.6 mL screw-cap vial and shaken at room temperature for 1 hour. An aqueous NaOH solution (5 M, 228 μL) was added thereto, and the mixture was shaken at room temperature for 2 hours. The suspension of the reaction solution and the resin was transferred to a syringe with a filter using NMP and washed three times with an NMP:HzO (1:1) solution (1000 μL) containing NAC (10 mg/mL), three times with water (1000 μL), three times with methanol (1000 μL), and three times with DCM (1000 μL). Then, the resin was dried under reduced pressure to obtain the title compound AE016-01R.

Purity confirmation

A portion of the resin after drying was transferred to a pipette tip with a filter, washed three times with DCM (100 μL), and then dipped in a 10% solution of TFA in DCM containing 0.2 M trimethylbenzene (50 μL) for 5 minutes. After filtration, the filtrate was dissolved in NMP (250 μL), and the solution (50 μL) was diluted with acetonitrile (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

Compound to be analyzed: 4-[6-[3-(dimethylcarbamoyl)-4-hydroxynaphthalen-1- yl]pyridin-3-yl]-3-fluorobenzoic acid (AE016)

[Formula 107]

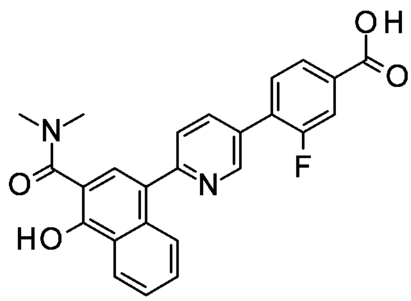

MS: m/z 431.1[M+H]+.
Retention time: 1.01 min.

Example 1-6-9: Synthesis of solid-phase supported aromatic carboxylic acid allyl ester (AE018-01R)

[Formula 108]

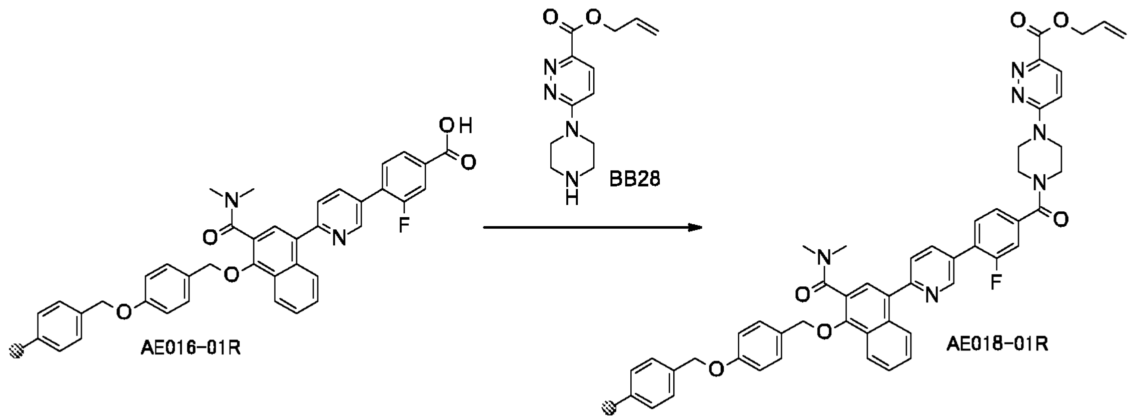


Under a nitrogen atmosphere, AE016-01R (0.37 mmol/g, 350 mg) and NMP (5.95 mL, 17 v/w) were added to an 8.0 mL screw-cap vial and shaken at room temperature for 1 hour. BB28 (96 mg, 3.0 equivalents), HOAt (106 mg, 6.0 equivalents), and DIC (121 μL, 6.0 equivalents) were added thereto, and the mixture was shaken at room temperature for 14 hours. The suspension of the reaction solution and the resin was transferred to a syringe with a filter using NMP and washed three times with NMP (3.5 mL), three times with methanol (3.5 mL), three times with DCM (3.5 mL), and three times with heptane (3.5 mL). Then, the resin was dried under reduced pressure to obtain the title compound AE018-01R.

Purity confirmation

A portion of the resin after drying was transferred to a pipette tip with a filter, washed three times with DCM (100 μL), and then dipped in a 10% solution of TFA in DCM containing 0.2 M trimethylbenzene (50 μL) for 5 minutes. After filtration, the filtrate was dissolved in NMP (250 μL), and the solution (50 μL) was diluted with acetonitrile (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

Compound to be analyzed: prop-2-enyl 6-[4-[4-[6-[3-(dimethylcarbamoyl)-4- hydroxynaphthalen-1-yl]pyridin-3-yl]-3-fluorobenzoyl]piperazin-1-yl]pyridazine-3- carboxylate (AE018)

[Formula 109]

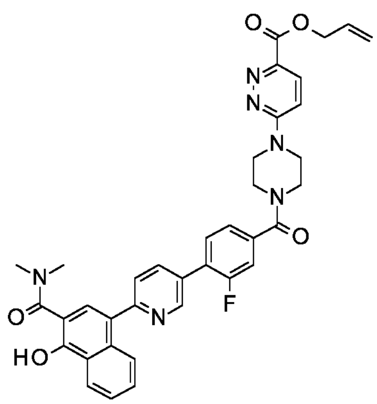

MS: m/z 661.1[M+H]$^+$.

Retention time: 1.07 min.

Example 1-6-10: Synthesis of solid-phase supported aromatic carboxylic acid (AE019-01R)

[Formula 110]

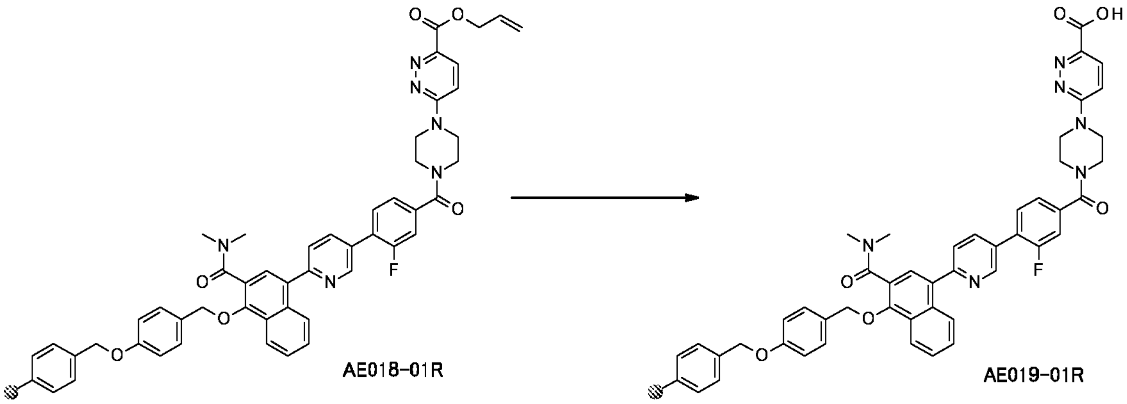


Under a nitrogen atmosphere, solid-phase supported aromatic carboxylic acid AE018-01R (0.37 mmol/g, 57 mg) and THF (855 μL, 15 v/w) were added to a 2.0 mL screw- cap vial and shaken at room temperature for 1 hour. Tetrakistriphenylphosphine palladium(0) (1.2 mg) and morpholine (6.4 μL) were added thereto, and the mixture was shaken at room temperature for 1 hour. The resin suspension was transferred to a column with a filter using NMP, repetitively washed three times with NMP (1.14 mL), three times with methanol (1.14 mL), three times with NAC (0.3 M, NMP:HzO = 5:1 solution, 1.14 mL), three times with malonic acid (0.05 M, a solution in NMP, 1.14 mL), three times with HOAt (0.2 M, a solution in NMP, 1.14 mL), three times with NMP (1.14 mL), three times with methanol (12.7 mL), three times with DCM (1.14 mL), and three times with heptane (1.14 mL), and then dried under reduced pressure to obtain the title compound AE019-01R.

Purity confirmation

A portion of the resin after drying was transferred to a pipette tip with a filter, washed three times with DCM (100 μL), and then dipped in a 10% solution of TFA in DCM containing 0.2 M trimethylbenzene (50 μL) for 5 minutes. After filtration, the filtrate was dissolved in NMP (250 μL), and the solution (50 μL) was diluted with acetonitrile (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

Compound to be analyzed: 6-[4-[4-[6-[3-(dimethylcarbamoyl)-4- hydroxynaphthalen-1-yl]pyridin-3-yl]-3-fluorobenzoyl]piperazin-1-yl]pyridazine-3- carboxylic acid (AE019)

[Formula 111]

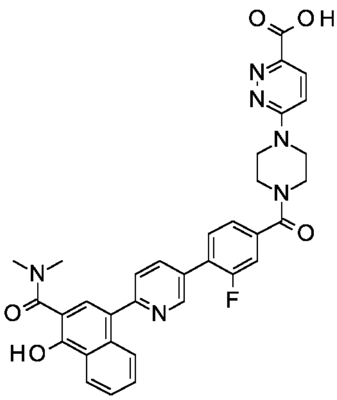

MS: m/z 621.2[M+H]+.

Retention time: 0.86 min.

Example 1-6-11: Synthesis of solid-phase supported aromatic carboxylic acid ester (AE021-01R)

[Formula 112]

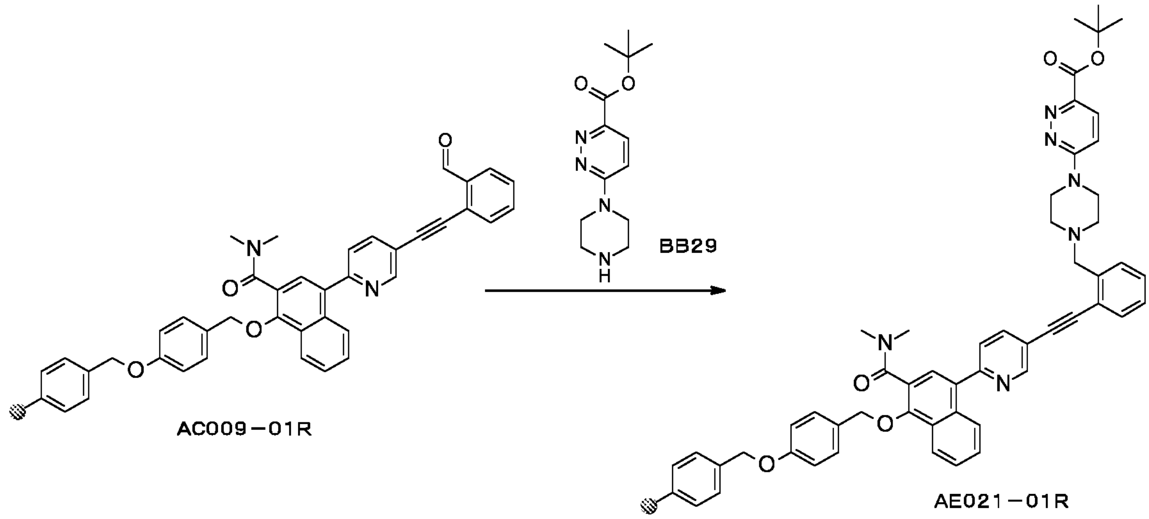


Aromatic aldehyde AC009-01R used in this example was synthesized in Example 1- 4-11. Under a nitrogen atmosphere, solid-phase supported aromatic aldehyde AC009-01R (0.37 mmol/g, 20 mg) and DCE (400 μL, 20 v/w) were added to a 0.6 mL screw-cap vial and shaken at room temperature for 1 hour. BB29 (11.7 mg, 6.0 equivalents) and $Ti(OtBu)_4$ (28.6 μL, 10 equivalents) were added thereto, and the mixture was shaken at 60°C for 1 hour. After cooling to room temperature, sodium triacetoxyborohydride (18.8 mg, 12 equivalents) and acetic acid (1.3 μL, 3.0 equivalents) were added thereto, and the mixture was shaken at room temperature for 24 hours. Methanol (400 μL) was added to the reaction solution, and then, the resin suspension was transferred to a syringe with a filter using methanol (400 μL) (this operation was repeated a total of six times), repetitively washed three times with methanol (400 μL), three times with sodium bicarbonate (0.15 M NMP:HzO = 1:5 solution, 400 μL), three times with water (400 μL), three times with methanol (400 μL), and three times with DCM (400 μL), and then dried under reduced pressure to obtain the title compound AE021-01R.

Purity confirmation

A portion of the resin after drying was transferred to a pipette tip with a filter, washed three times with DCM (100 μL), and then dipped in a 10% solution of TFA in DCM containing 0.2 M trimethylbenzene (50 μL) for 5 minutes. After filtration, the filtrate was dissolved in NMP (250 μL), and the solution (50 μL) was diluted with acetonitrile (250 μL) to prepare an LC sample. The degree of progression of the reaction was measured by LC-MS analysis. The following

compound to be detected was analyzed under analysis conditions SMD-FA05-1, and the UV area% was confirmed at a UV wavelength of 319 nm.

Compound to be analyzed: 6-[4-[4-[6-[3-(dimethylcarbamoyl)-4- hydroxynaphthalen-1-yl]pyridin-3-yl]-3-fluorobenzoyl]piperazin-1-yl]pyridazine-3- carboxylic acid (AE021)

[Formula 113]

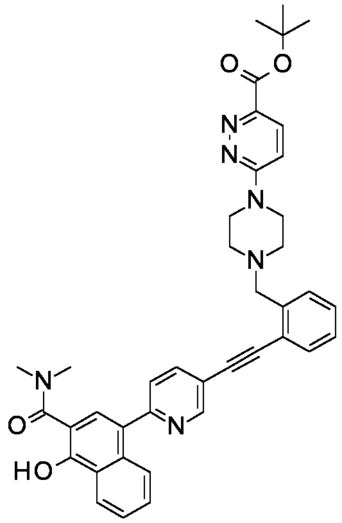

MS: m/z 669.3[M+H]+.
Retention time: 0.92 min.

Example 1-6-12: Synthesis of solid-phase supported aromatic carboxylic acid (AE022-01R)

[Formula 114]

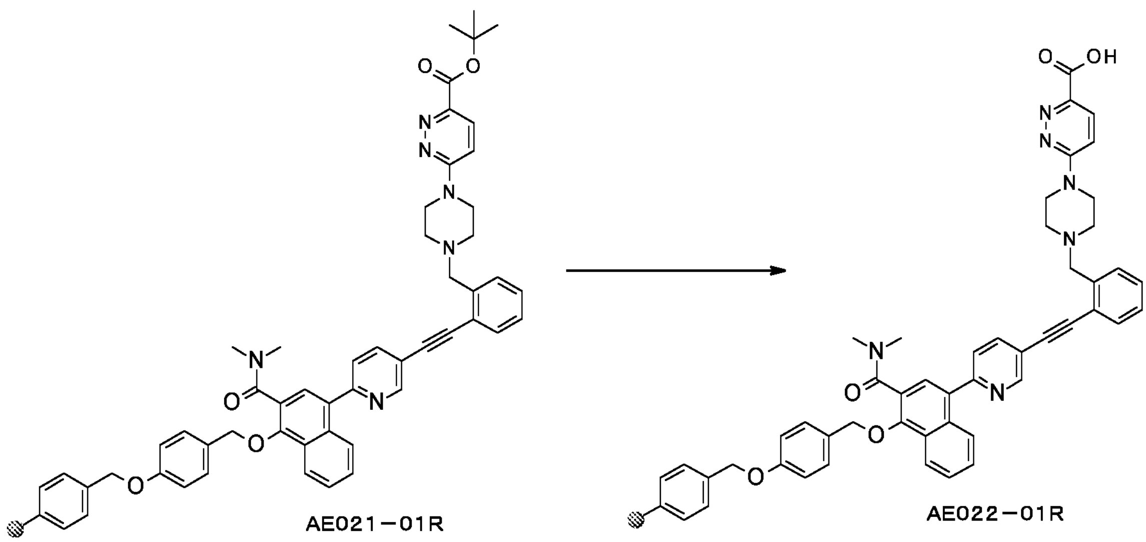


Under a nitrogen atmosphere, solid-phase supported aromatic carboxylic acid ester form AE021-01R (0.37 mmol/g, 16 mg), THF (240 μL, 15 v/w), and methanol (24 μL, 1.5 v/w) were added to a 0.6 mL screw-cap vial and shaken at room temperature for 1 hour. An aqueous LiOH solution (2 M, 22 μL) was added thereto, and the mixture was shaken

at room temperature for 22 hours. The resin suspension was transferred to a column with a filter using methanol, repetitively washed three times with NMP (432 μL), three times with tetrabutylammonium bisulfate (a 0.05 M solution in NMP, 432 μL), three times with NMP (432 μL), three times with MeOH (432 μL), and three times with DCM (432 μL), and then dried under reduced pressure to obtain the title compound AE022-01R.

Purity confirmation

A portion of the resin after drying was transferred to a pipette tip with a filter, washed three times with DCM (100 μL), and then dipped in a 10% solution of TFA in DCM containing 0.2 M trimethylbenzene (50 μL) for 5 minutes. After filtration, the filtrate was dissolved in NMP (250 μL), and the solution (50 μL) was diluted with acetonitrile (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

Compound to be analyzed: 6-[4-[[2-[2-[6-[3-(dimethylcarbamoyl)-4- hydroxynaphthalen-1-yl]pyridin-3-yl]ethynyl]phenyl]methyl]piperazin-1-yl]pyridazine-3- carboxylic acid (AE022)

[Formula 115]

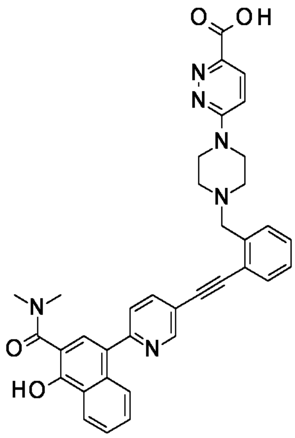

MS: m/z 613.2[M+H]+.
Retention time: 0.78 min.

Example 1-7: Synthesis of substrate for reaction study for use mainly in Examples 2-9 and 2-10, and building block for use in mixture library, etc.

Example 1-7-1: Synthesis of compound C008-03R

[Formula 116]

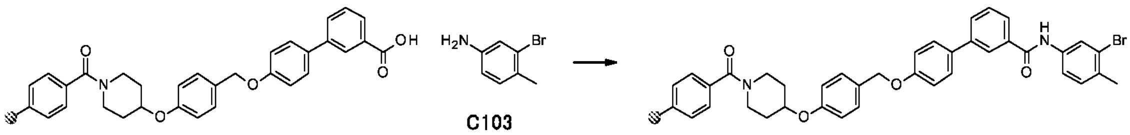

C003-03R C103 C008-03R

Under a nitrogen atmosphere, compound C003-03R (loading rate: 0.511 mmol/g, containing dichloromethane (0.050 g), 0.950 g, 0.460 mmol) and NMP (13.5 mL) were added to a 20 mL glass vial and shaken at room temperature for 1 hour. 3-Bromo-4-methylaniline (C103) (0.171 g, 0.920 mmol), HOAt (0.125 g, 0.920 mmol), and DIC (0.143 mL, 0.920 mmol) were added thereto, and the mixture was shaken at 60°C for 18 hours. The suspension of the reaction

solution and the resin was wholly transferred onto a filter and washed three times with NMP (18 mL), three times with methanol (18 mL), and three times with DCM (18 mL), and the obtained solid phase was dried overnight under reduced pressure to obtain compound C008-03R (loading rate: 0.471 mmol/g, 0.890 g, 0.419 mmol).

Reaction monitoring

The suspension of the reaction solution and the resin (0.010 mL) was transferred onto a filter, washed three times with NMP (0.1 mL), three times with methanol (0.1 mL), and three times with DCM (0.1 mL), and then dipped in a 10% solution of TFA in DCM containing 0.2 M pentamethylbenzene (0.05 mL) for 2 minutes. After filtration, NMP (0.25 mL) was added to the filtrate. Acetonitrile (0.25 mL) was added to a 0.050 mL aliquot of the obtained solution to prepare an LC sample. The degree of progression of the reaction was measured by LC-MS analysis. As a result, 100% of compound C008 of interest was observed.

[Formula 117]

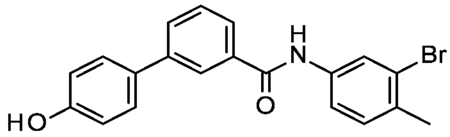

Compound C008

LRMS: m/z 382, 384[M+H]+.

Retention time: 1.232 min (analysis conditions SMD-FA05-1, 280 nm).

Example 1-7-2: Synthesis of compound C009-03R

[Formula 118]

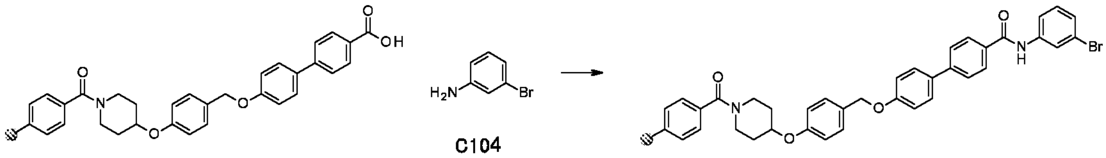

C005-03R C104 C009-03R

Under a nitrogen atmosphere, compound C005-03R (loading rate: 0.201 mmol/g, 250 mg, 0.0502 mmol), NMP (3.75 mL), 3-bromoaniline (C104) (20 μL, 0.19 mmol), HOAt (26 mg, 0.19 mmol), and DIC (29 μL, 0.19 mmol) were added to a 5 mL glass vial and shaken at 60°C for 15 hours. 3-Bromoaniline (C104) (41 μL, 0.38 mmol), HOAt (51 mg, 0.38 mmol), and DIC (58 μL, 0.38 mmol) were added thereto, and the mixture was shaken at 60°C for 4 hours.

Solid-phase purification

The suspension of the reaction solution and the resin was wholly transferred onto a filter and washed three times with NMP (5 mL), once with NMP/methanol = 1:1 (5 mL), three times with methanol (5 mL), and three times with DCM (5 mL), and the obtained solid phase was dried overnight under reduced pressure to obtain compound C009-03R (loading rate: 0.195 mmol/g, 248 mg, 0.0484 mmol).

C009-03R was dipped in a 10% solution of TFA in DCM containing 0.1 M pentamethylbenzene (0.05 mL) for 2 minutes. NMP (0.05 mL) and acetonitrile (0.25 mL) were added thereto, and the mixture was filtered. The filtrate was subjected to LC-MS analysis, so that compound C009 of interest was observed.

[Formula 119]

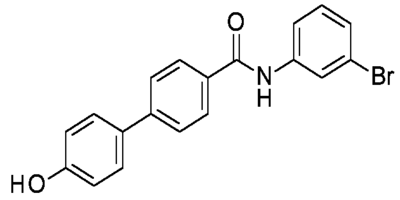

Compound C009

LRMS: m/z 368, 370[M+H]+.

Retention time: 1.166 min (analysis conditions SMD-FA05-1, 299 nm).

Example 1-7-3: Synthesis of compound C010-03R

[Formula 120]

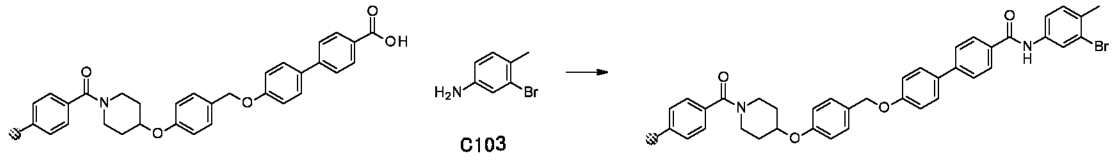

C103

C005-03R

C010-03R

Compound CO10-03R (loading rate: 0.194 mmol/g) can be synthesized by the same approach as in compound C009-03R using compound C005-03R (loading rate: 0.201 mmol/g) and 3-bromo-4-methylaniline (C103).

C010-03R was dipped in a 10% solution of TFA in DCM containing 0.1 M pentamethylbenzene (0.05 mL) for 2 minutes. NMP (0.05 mL) and acetonitrile (0.25 mL) were added thereto, and the mixture was filtered. The filtrate was subjected to LC-MS analysis, so that compound C010 of interest was observed.

[Formula 121]

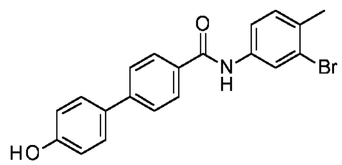

Compound C010

LRMS: m/z 382, 384[M+H]+.

Retention time: 1.221 min (analysis conditions SMD-FA05-1, 299 nm).

Example 1-7-4: Synthesis of compound C011-03R

[Formula 122]

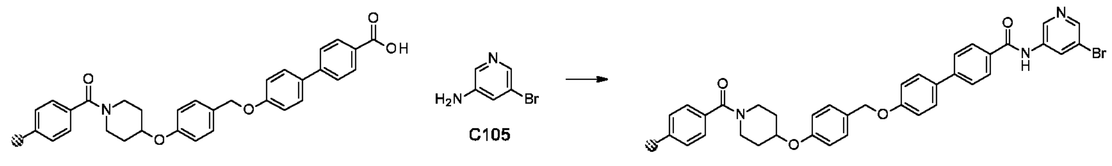

C105

C005-03R

C011-03R

Under a nitrogen atmosphere, compound C005-03R (loading rate: 0.201 mmol/g, 1.00 g, 0.20 mmol) and NMP

(15 mL) were added to a 20 mL empty column with a filter and shaken at room temperature for 1 hour. 3-Amino-5-bromopyridine (C105) (70 mg, 0.40 mmol), 1-methylimidazole (64 μL, 0.804 mmol), and PyClU (134 mg, 0.402 mmol) were added thereto, and the mixture was shaken at room temperature for 1 hour. 3-Amino-5- bromopyridine (C105) (35 mg, 0.201 mmol), 1-methylimidazole (32 μL, 0.402 mmol), and PyClU (67 mg, 0.201 mmol) were added thereto, and the mixture was shaken at room temperature for 20 minutes.

The solid phase was washed twice with NMP (15 mL), three times with MeOH (15 mL), and three times with DCM (1 mL), and the obtained solid phase was dried overnight under reduced pressure to obtain compound CO11-03R (loading rate: 0.195 mmol/g, 1.04 g, 0.203 mmol).

The suspension of the reaction solution and the resin (10 μL) was transferred onto a filter, washed three times with NMP (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with NMP (0.1 mL). MeCN (0.25 mL) was added to the filtrate (50 μL) to prepare an LC sample. The degree of progression of the reaction was measured by LC-MS analysis. As a result, 100% of compound C011 of interest was observed.

[Formula 123]

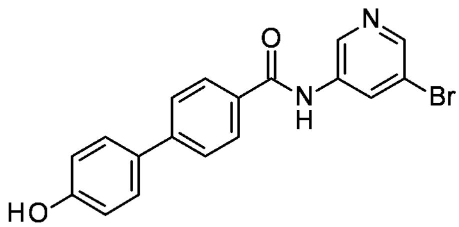

Compound C011

LRMS: m/z 369, 371[M+H]+.

Retention time: 1.014 min (analysis conditions SMD-FA05-1, 299 nm).

Example 1-8: Synthesis of substrate for reaction study for use mainly in Example 2- 11, and building block for use in mixture library, etc.

Example 1-8-1: Synthesis of solid-phase supported Ar-$NH_2$ (compound D001-03R)

Solid-phase supported Ar-$NH_2$ (compound D001-03R) was synthesized as follows by the condensation of solid-phase supported carboxylic acid (C007-03R) and 3- (aminomethyl)aniline (D038).

[Formula 124]

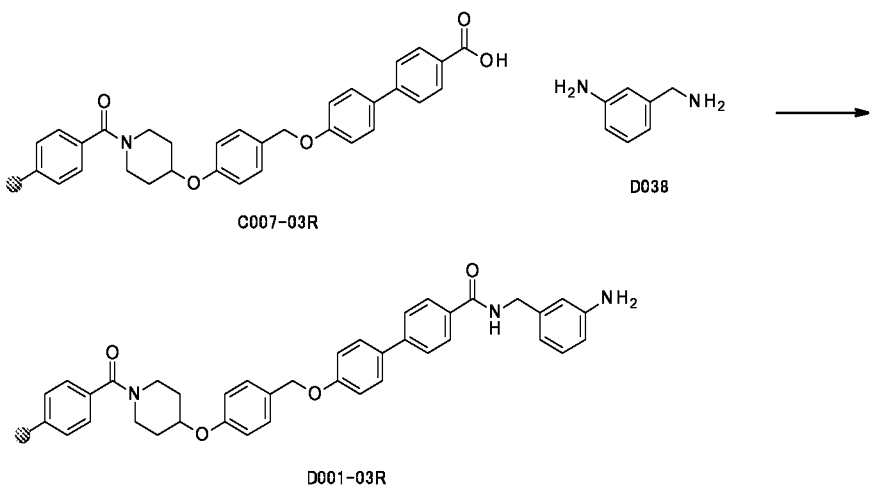

Under a nitrogen atmosphere, compound C007-03R (loading rate: 0.050 mmol/g, 0.450 g, 0.0225 mmol) and NMP (0.675 mL) were added to a 20 mL glass vial and shaken at room temperature for 1 hour. 3-(Aminomethyl)aniline (D038, 76 μL, 0.68 mmol), oxyma (96.0 mg, 0.675 mmol), and DIC (0.105 mL, 0.675 mmol) were added thereto, and the mixture was shaken at room temperature for 9 hours.

Solid-phase purification

The suspension of the reaction solution and the resin was transferred onto a filter using NMP (6 mL) and washed twice with NMP (6 mL), three times with methanol (6mL), and three times with DCM (6 mL), and the obtained solid phase was dried overnight under reduced pressure to obtain compound D001-03R (loading rate: 0.050 mmol/g, 450 mg, 0.0225 mmol).

Reaction monitoring

The suspension of the reaction solution and the resin (0.020 mL) was transferred onto a filter, washed three times with NMP (0.1 mL), three times with methanol (0.1 mL), and three times with DCM (0.1 mL), and then dipped in a 10% solution of TFA in DCM containing 0.02 M pentamethylbenzene (0.05 mL) for 5 minutes. After filtration, the resin on the filter was washed with NMP (0.05 mL). Acetonitrile (0.50 mL) was added to the obtained filtrate to prepare an LC sample. The degree of progression of the reaction was measured by LC-MS analysis. As a result, compound D001 of interest was observed at a purity of 98.2%.

[Formula 125]

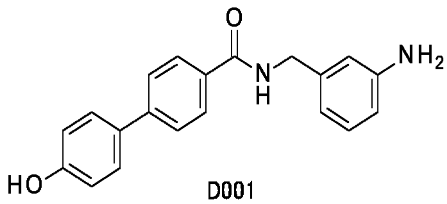

D001

Compound D001
LRMS: m/z 319[M+H]+.
Retention time: 0.707 min (analysis conditions SMD-TFA05-RP, 290 nm).

Example 1-8-2: Synthesis of solid-phase supported Ar-NHMe (compound D020- 03R)

Solid-phase supported Ar-NHMe (D020-03R) was synthesized by the condensation of solid-phase supported carboxylic acid (C007-03R) and 3-(aminomethyl)-N-methylaniline (D039) by the same approach as in Example 1-8-1. Solid-phase purification and reaction monitoring (compound to be monitored: D020) were also performed in the same manner as in Example 1-8-1.

[Formula 126]

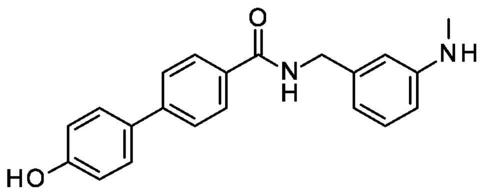

D020

Compound to be monitored: D020
LRMS: m/z 333[M+H]+.
Retention time: 0.679 min (analysis conditions SMD-FA05-1).

Example 1-9: Synthesis of substrate for reaction study for use mainly in Example 3, and building block for use in mixture library, etc.

Example 1-9-1: Synthesis of compound E015-02R

[Formula 127]

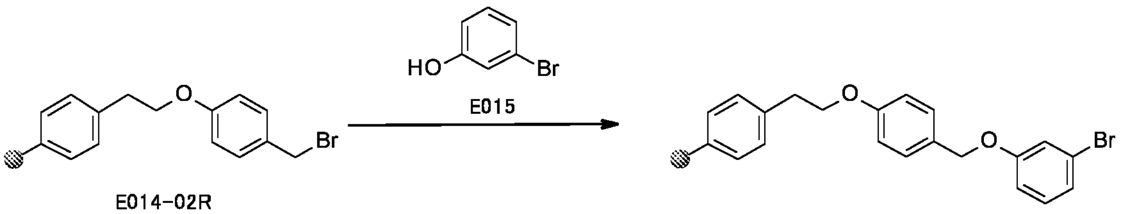


E014-02R

E015-02R

Under a nitrogen atmosphere, NMP (15 mL) was added to brominated modified Wang resin (compound E014-02R, Sigma-Aldrich Co., LLC, 534471, 100-200 mesh, loading rate: 1.23 mmol/g, copolymer (styrene-1% DVB), 1.50 g, 1.845 mmol), and the mixture was shaken at room temperature for 1 hour. 3-Bromophenol (compound E015, 638 mg, 3.690 mmol) and tBuOK (393 mg, 3.51 mmol) were added thereto, and the mixture was shaken at room temperature for 20 hours. The suspension of the reaction solution and the resin was transferred to a syringe with a filter using NMP (30 mL) and washed three times with NMP (30 mL), three times with methanol (30 mL), three times with DCM (30 mL), and twice with heptane (30 mL). The obtained resin was dried overnight under reduced pressure to obtain the title compound (compound E015-02R).

Example 1-9-2: Calculation of amount of compound E015-02R supported

The amount of the compound supported (mmol/g) by the resin was determined by the following method. The following experimental operation is an example and does not uniformly define the amount of the compound supported as to all lots.

[Formula 128]

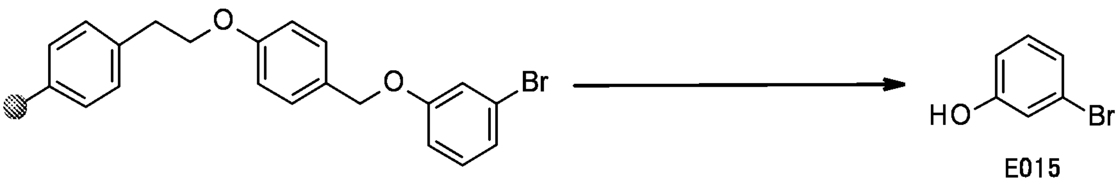

E015

E015-02R

Under a nitrogen atmosphere, compound E015-02R (20 mg) and DCM (280 μL) were added to a 0.6 mL glass vial and shaken at room temperature for 1 hour. Trimethylbenzene (6.02 μL, 0.045 mmol) and TFA (120 μL, 1.568 mmol) were added thereto, and the mixture was shaken at room temperature for 15 minutes. 10 μL of the supernatant of the reaction was sampled and diluted with acetonitrile (1000 μL) to prepare an LC sample. The reaction was monitored by LC-MS analysis.

The concentration of the obtained E015 in the solution was calculated from UV area% and a calibration curve to determine the amount of E015 supported by the resin. As a result, the amount of the compound supported was calculated as 0.556 mmol/g.

Example 1-9-3: Synthesis of compound E016-02R

[Formula 129]

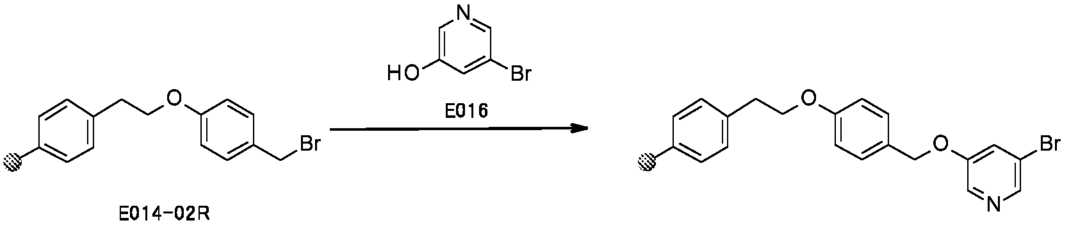


E014-02R E016-02R

The compound was synthesized in the same manner as in Example 1-9-1 using 5- bromopyridin-3-ol (compound E016, 642 mg, 3.69 mmol).

Example 1-9-4: Calculation of amount of compound E016-02R supported

The amount of the compound supported (mmol/g) by the resin was determined by the following method. The following experimental operation is an example and does not uniformly define the amount of the compound supported as to all lots.

[Formula 130]

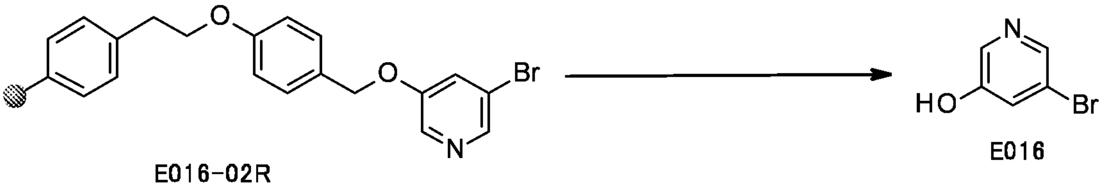

E016-02R E016

Under a nitrogen atmosphere, compound E016-02R (20 mg) and DCM (120 μL) were added to a 0.6 mL glass vial and shaken at room temperature for 1 hour. Trimethylbenzene (4.93 μL, 0.037 mmol), HFIP (240 μL), and 1 M hydrochloric acid (40 μL) were added thereto, and the mixture was shaken at room temperature for 15 minutes. 10 μL of the supernatant of the reaction was sampled and diluted with acetonitrile (1000 μL) to prepare an LC sample. The reaction was monitored by LC-MS analysis.

The concentration of the obtained E016 in the solution was calculated from UV area% and a calibration curve to determine the amount of E016 supported by the resin. As a result, the amount of the compound supported was calculated as 0.733 mmol/g.

Example 1-10: Synthesis of compound for use mainly in Example 7

Example 1-10-1: Synthesis of 4-(2-phenylsulfanylethylamino)-3- (trifluoromethyl)benzenesulfonamide (AG010)

[Formula 131]

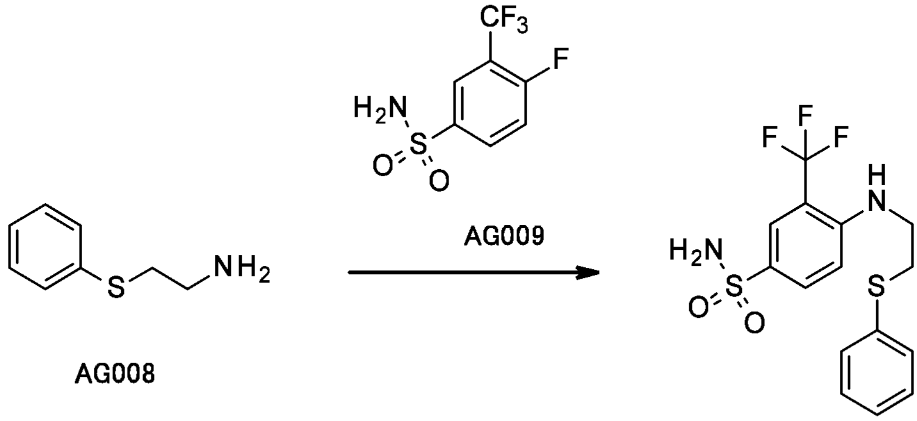

AG008 AG009 AG010

To a solution of 2-phenylsulfanylethanamine (AG008) (3.00 g, 19.48 mmol) and 4- fluoro-3-(trifluoromethyl)benzenesulfonamide (AG009) (4.76 g, 19.48 mmol) in DMSO (30 mL), DIPEA (5.06 g, 38.96 mmol) was added, and the mixture was stirred at 70°C for 16 hours. The mixture was purified by reverse-phase column chromatography (C18, acetonitrile/water, 5-80%) to obtain the title compound AG010 (3.8 g, 10.1 mmol, 50.5%).

$^1$H-NMR (400 MHz, DMSO-$d_6$) $\delta$: 7.82 (d, J = 2.0 Hz, 1H), 7.79-7.73 (m, 1H), 7.45-7.38 (m, 2H), 7.36-7.30 (m, 2H), 7.26-7.19 (m, 1H), 7.15 (s, 2H), 6.89 (d, J = 8.0 Hz, 1H), 6.37 (t, J = 4.0 Hz, 1H), 3.52-3.44 (m, 2H), 3.32-3.14 (m, 2H).

LRMS (ESI): m/z 377 $[M+H]^+$.

Example 1-10-2: Synthesis of 4-[4-[(2-bromophenyl)methyl]piperazin-1-yl]-N-[3- nitro-4-(2-phenylsulfanylethylamino)phenyl]sulfonylbenzamide (AG003)

[Formula 132]

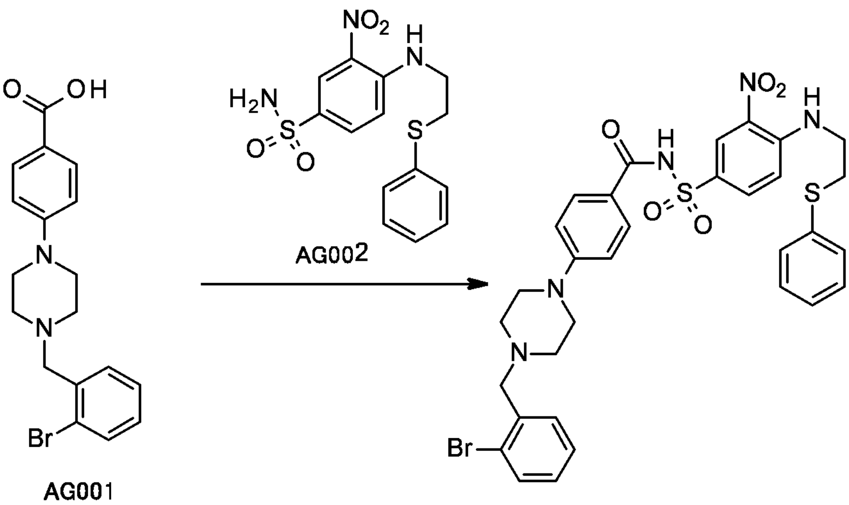

AG001 AG002 AG003

To a solution of 3-nitro-4-(2-phenylsulfanylethylamino)benzenesulfonamide (AG002) (596 mg, 1.589 mmol), 4-[4-[(2-bromophenyl)methyl]piperazin-1-yl]benzoic acid (AG001) (1.19 g, 3.179 mmol), EDCI (607.1 mg, 3.179 mmol), and DMAP (193.4 g, 1.589 mmol) in dichloromethane (12 mL), DIPEA (1.76 g, 6.357 mmol) was added, and the mixture was stirred at room temperature for 16 hours. The mixture was diluted with dichloromethane, and water was added

thereto. An organic layer was extracted and dried over sodium sulfate. After concentration, the obtained residue was purified by silica gel column chromatography (ethyl acetate/petroleum ether, 0-100%, and subsequently methanol/dichloromethane, 0-10%) to obtain the title compound AG003 (2.2 g, 3.1 mmol, 94%) as a yellow solid.
LRMS (ESI): m/z 710[M+H]+.
Retention time: 1.166 min (analysis conditions PH-TFA05-1).

Example 1-10-3: Synthesis of 4-[4-[[2-(3-ethoxyphenyl)phenyl]methyl]piperazin-1- yl]-N-[3-nitro-4-(2-phenylsulfanylethylamino)phenyl]sulfonylbenzamide [AG005 (B2Q045- 01)]

[Formula 133]

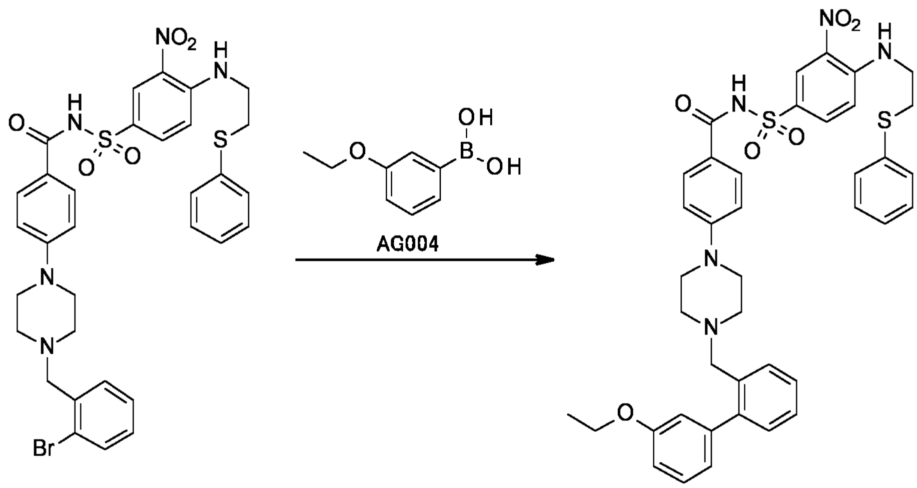


AG003

AG005

Under a nitrogen atmosphere, a solution of 4-[4-[(2-bromophenyl)methyl]piperazin- 1-yl]-N-[3-nitro-4-(2-phenylsulfanylethylamino)phenyl]sulfonylbenzamide (AG003) (250 mg, 0.352 mmol), (3-ethoxyphenyl)boronic acid (AG004) (116.8 mg, 0.704 mmol), $Pd(PPh_3)_2c1_2$ (24.7 mg, 0.035 mmol), and sodium carbonate (48.5 mg, 0.457 mmol) in dimethoxyethane/ethanol/water = 1/1/1 (5 mL) was stirred at 80°C for 3 hours. The mixture was filtered, and the filtrate was diluted with ethyl acetate. Water was added thereto. An organic layer was extracted and dried over sodium sulfate. After concentration, the obtained residue was purified by reverse-phase column chromatography (C18, acetonitrile/water, 5-60%). The obtained crude product was purified by preparative HPLC (YMC-Actus Triart C18, acetonitrile/10 mM aqueous ammonium bicarbonate solution, 50-80%) to obtain the title compound AG005 (45 mg, 0.060 mmol, 16.9%) as a yellow solid.
$^1$H-NMR (300 MHz, DMSO-$d_6$) $\delta$: 11.93 (s, 1H), 8.72 (s, 1H), 8.55 (d, J = 2.4 Hz, 1H), 7.92-7.83 (m, 1H), 7.71 (d, J = 8.7 Hz, 2H), 7.52 (d, J = 7.2 Hz, 1H), 7.46-7.16 (m, 8H), 7.21-7.06 (m, 2H), 7.05-6.69 (m, 5H), 4.03 (q, J = 6.9 Hz, 2H), 3.63 (d, J = 6.6 Hz, 2H), 3.45 (s, 2H), 3.26 (t, J = 6.6 Hz, 6H), 2.41 (s, 4H), 1.30 (t, J = 6.9 Hz, 3H).
LRMS (ESI): m/z 752 [M+H]+.
AG005 whose synthesis method is illustrated in Example 1-10-3 is also referred to as compound B2Q045-01 in the subsequent examples.

Example 1-10-4: Synthesis of 4-[4-[[2-(5-ethoxypyridin-3- yl)phenyl]methyl]piperazin-1-yl]-N-[3-nitro-4-(2- phenylsulfanylethylamino)phenyl]sulfonylbenzamide [AG007 (B2Q046-01)]

[Formula 134]

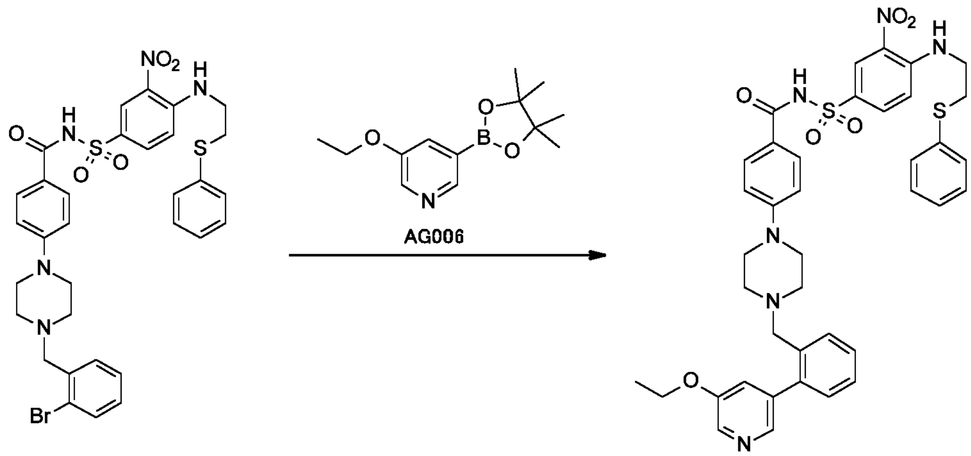


AG003

AG007

Under a nitrogen atmosphere, a solution of 4-[4-[(2-bromophenyl)methyl]piperazin- 1-yl]-N-[3-nitro-4-(2-phenylsulfanylethylamino)phenyl]sulfonylbenzamide (AG003) (350 mg, 0.492 mmol), 3-ethoxy-5-(4,4,5,5-tetramethyl-1,3,2-dioxaboloran-2-yl)pyridine (AG006) (245.4 mg, 0.985 mmol), $Pd(PPh_3)_2c1_2$ (34.6 mg, 0.049 mmol), and sodium carbonate (68.5 mg, 0.640 mmol) in dimethoxyethane/ethanol/water = 1/1/1 (6 mL) was stirred at 80°C for 3 hours. The mixture was filtered, and the filtrate was diluted with ethyl acetate. Water was added thereto. An organic layer was extracted and dried over sodium sulfate. After concentration, the obtained residue was purified by reverse-phase column chromatography (C18, acetonitrile/water, 5-80%). The obtained crude product was purified by preparative HPLC (XBridge Shield RP18 OBD Column, acetonitrile/10 mM aqueous ammonium bicarbonate solution, 37-52%) to obtain the title compound AG007(28.4 mg, 0.038 mmol, 7.6%) as a yellow solid.

$^1$H-NMR (300 MHz, DMSO-$d_6$) δ: 11.97 (s, 1H), 8.68 (s, 1H), 8.53 (d, J = 2.1 Hz, 1H), 8.25 (d, J = 2.7 Hz, 1H), 8.16 (d, J = 1.8 Hz, 1H), 7.90-7.83 (m, 1H), 7.71 (d, J = 9.0 Hz, 2H), 7.59-7.46 (m, 2H), 7.46-7.17 (m, 7H), 7.17-6.95 (m, 2H), 6.85 (d, J = 8.7 Hz, 2H), 4.11 (q, J = 6.9 Hz, 2H), 3.68-3.56 (m, 2H), 3.42 (s, 2H), 3.23 (s, 5H), 2.39 (s, 4H), 1.32 (t, J = 6.9 Hz, 3H).

LRMS (ESI): m/z 753 $[M+H]^+$.

AG007 whose synthesis method is illustrated in Example 1-10-4 is also referred to as compound B2Q046-01 in the subsequent examples.

Example 1-10-5: Synthesis of 4-[4-[(2-bromophenyl)methyl]piperazin-1-yl]-N-[4- (2-phenylsulfanylethylamino)-3 -(trifluoromethyl)phenyl] sulfonylbenzamide (AGO 11)

[Formula 135]

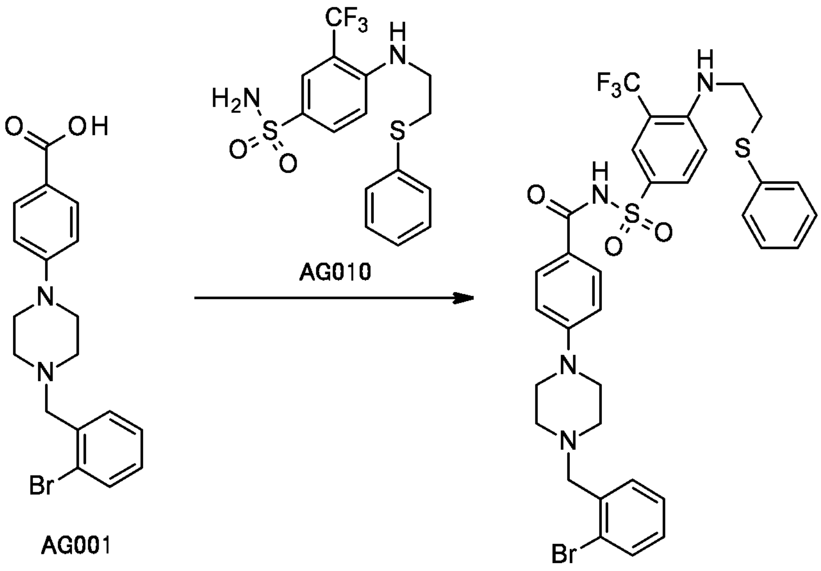


AG001

AG011

To a solution of 4-(2-phenylsulfanylethylamino)-3- (trifluoromethyl)benzenesulfonamide (AG010) (0.80 g, 2.125 mmol), 4-[4-[(2- bromophenyl)methyl]piperazin-1-yl]benzoic acid (AG001) (1.20 g, 3.188 mmol), EDCI (0.81 g, 4.251 mmol), and DMAP (0.26 g, 2.125 mmol) in dichloromethane (16 mL), DIPEA (1.10 g, 8.501 mmol) was added, and the mixture was stirred at room temperature for 16 hours. An aqueous ammonium chloride solution was added to the mixture. An organic layer was extracted with dichloromethane three times and dried over sodium sulfate. After concentration, the obtained residue was purified by silica gel column chromatography (ethyl acetate/petroleum ether, 0-100%) to obtain the title compound AG011 (1.3 g, 1.77 mmol, 83.4%) as a yellow solid.

LRMS (ESI): m/z 733[M+H]+.

Retention time: 1.319 min (analysis conditions PH-FA05-2).

Example 1-10-6: Synthesis of 4-[4-[[2-(3-ethoxyphenyl)phenyl]methyl]piperazin-1- yl]-N-[4-(2-phenylsulfanylethylamino)-3-(trifluoromethyl)phenyl] sulfonylbenzamide [AG012 (B2R045-01)]

[Formula 136]

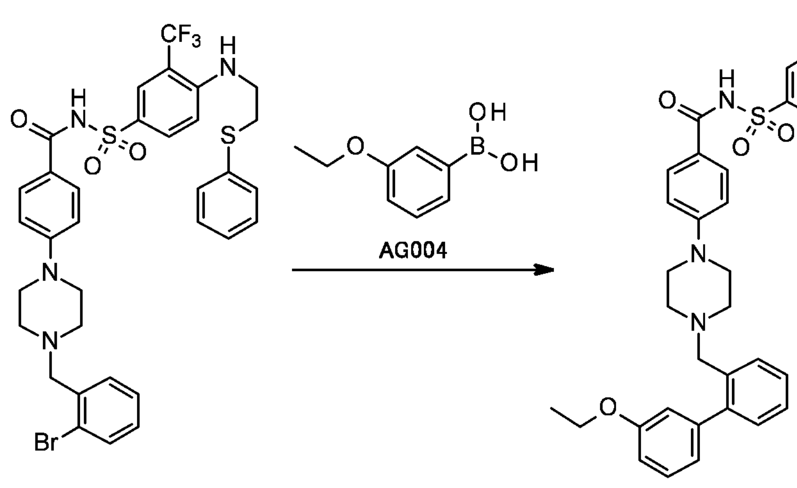


AG011

AG012

Under a nitrogen atmosphere, a solution of 4-[4-[(2-bromophenyl)methyl]piperazin- 1-yl]-N-[4-(2-phenylsulfanylethylamino)-3-(trifluoromethyl)phenyl] sulfonylbenzamide (AG011) (165 mg, 0.225 mmol), (3-ethoxyphenyl)boronic acid (AG004) (74.7 mg, 0.450 mmol), $Pd(PPh_3)_2c1_2$ (15.8 mg, 0.022 mmol), and sodium carbonate (31.0 mg, 0.292 mmol) in dimethoxyethane/ethanol/water = 1/1/1 (4 mL) was stirred at 80°C for 3 hours. The mixture was filtered, and the filtrate was diluted with ethyl acetate. Water was added thereto. An organic layer was extracted and dried over sodium sulfate. After concentration, the obtained residue was purified by reverse-phase column chromatography (C18, acetonitrile/water, 5-90%). The obtained crude product was purified by preparative HPLC (YMC-Actus Triart C18, acetonitrile/10 mM aqueous ammonium bicarbonate solution, 53-83%) to obtain the title compound AG012 (19.7 mg, 0.025 mmol, 11.3%) as a white solid.

$^1$H-NMR (400 MHz, DMSO-$d_6$) δ: 11.84 (s, 1H), 7.94 (d, J = 2.0 Hz, 1H), 7.89-7.84 (m, 1H), 7.72 (d, J = 8.0 Hz, 2H), 7.57-7.50 (m, 1H), 7.45-7.25 (m, 7H), 7.26-7.17 (m, 2H), 7.05-6.81 (m, 6H), 6.60 (s, 1H), 4.04 (q, J = 8 Hz, 2H), 3.57-3.42 (m, 4H), 3.26 (t, J = 4 Hz, 4H), 3.22-3.12 (m, 2H), 2.42 (d, J = 5.7 Hz, 4H), 1.32 (t, J = 8 Hz, 3H).

LRMS (ESI): m/z 775 $[M+H]^+$.

AG012 whose synthesis method is illustrated in Example 1-10-6 is also referred to as compound B2R045-01 in the subsequent examples.

Example 1-10-7: Synthesis of 4-[4-[[2-(5-ethoxypyridin-3- yl)phenyl]methyl]piperazin-1-yl]-N-[4-(2-phenylsulfanylethylamino)-3- (trifluoromethyl)phenyl]sulfonylbenzamide [AG013 (B2R046-01)]

[Formula 137]

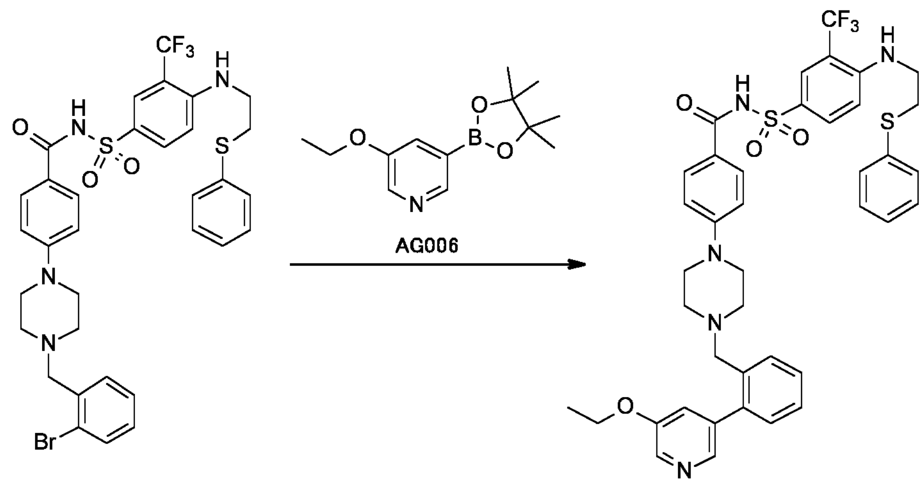


AG011

AG013

Under a nitrogen atmosphere, a solution of 4-[4-[(2-bromophenyl)methyl]piperazin- 1-yl]-N-[4-(2-phenylsulfanylethylamino)-3-(trifluoromethyl)phenyl] sulfonylbenzamide (AG011) (250 mg, 0.341 mmol), 3-ethoxy-5-(4,4,5,5-tetramethyl-1,3,2-dioxaboloran-2- yl)pyridine (AG006) (169.8 mg, 0.682 mmol), $Pd(PPh_3)_2c1_2$ (23.9 mg, 0.034 mmol), and sodium carbonate (47.0 mg, 0.443 mmol) in dimethoxyethane/ethanol/water = 1/1/1 (5 mL) was stirred at 80°C for 16 hours. The mixture was filtered, and the filtrate was diluted with ethyl acetate. Water was added thereto. An organic layer was extracted and dried over sodium sulfate. After concentration, the obtained residue was purified by reverse-phase column chromatography (C18, acetonitrile/water, 5-80%). The obtained crude product was purified by preparative HPLC (XBridge Prep OBD C18 Column, acetonitrile/10 mM aqueous ammonium bicarbonate solution, 45-75%) to obtain the title compound AG013 (34.2 mg, 0.044 mmol, 12.8%) as a gray solid.

$^1$H-NMR (300 MHz, DMSO-$d_6$) δ: 11.98 (s, 1H), 8.25 (d, J = 2.7 Hz, 1H), 8.16 (d, J = 1.8 Hz, 1H), 7.89 (d, J = 2.1 Hz, 1H), 7.80 (d, J = 9.3 Hz, 1H), 7.70 (d, J = 8.7 Hz, 2H), 7.55-7.47 (m, 2H), 7.48-7.23 (m, 7H), 7.22-7.15 (m, 1H), 6.83 (d, J = 8.6 Hz, 3H), 6.37 (s, 1H), 4.12 (q, J = 6.9 Hz, 2H), 3.40 (s, 4H), 3.17 (s, 4H), 3.13 (d, J = 8.4 Hz, 2H), 2.38 (s, 4H), 1.32 (t, J = 7.2 Hz, 3H).

LRMS (ESI): m/z 776 $[M+H]^+$.

AG013 whose synthesis method is illustrated in Example 1-10-7 is also referred to as compound B2R046-01 in the subsequent examples.

Example 1-11: Synthesis of other compounds

Example 1-11-1: Synthesis of compound A102-3R

[Formula 138]

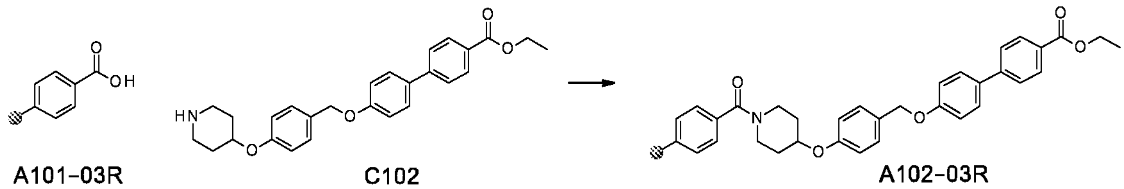

A101-03R C102 A102-03R

Under a nitrogen atmosphere, Carboxylic Resin (A101-03R) (Rapp Polymer, loading rate: 1.70 mmol/g, 40.0 g) and NMP (600 mL) were added to an 800 mL empty column with a filter and shaken at room temperature for 1 hour. HOAt (9.26 g, 68.0 mmol), DIC (10.6 mL, 68.0 mmol), and ethyl 4-[4-[(4-piperidin-4- yloxyphenyl)methoxy]phenyl]benzoate (C102) (4.13 g, 9.58 mmol) were added thereto, and the mixture was shaken at room temperature for 3.5 hours. HOAt (18.5 g, 136 mmol), DIC (21.2 mL, 136 mmol), and piperidine (16.8 mL, 170 mmol) were added thereto, and the mixture was shaken overnight at room temperature.

Solid-phase purification

The suspension of the reaction solution and the resin was wholly transferred to a 2 L separable flask and washed three times with NMP (800 mL), three times with MeOH (800 mL), three times with DCM (800 mL), and three times with heptane (800 mL), and the obtained solid phase was dried overnight under reduced pressure to obtain compound A102- 03R (loading rate: 0.200 mmol/g, 47.9 g).

Reaction monitoring

The suspension of the reaction solution and the resin (10 μL) was transferred onto a filter, washed three times with NMP (0.1 mL), three times with MeOH (0.1 mL), and three times with DCM (0.1 mL), and then dipped in a 10% solution of TFA in DCM containing 0.05 M pentamethylbenzene (0.1 mL) for 5 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 100% of compound A102 of interest was observed.

[Formula 139]

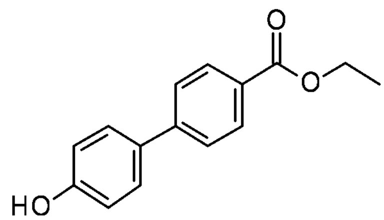

Compound A102

LRMS: m/z 243[M+H]+

Retention time: 1.081 min (analysis conditions FA05-1, 299 nm).

Example 1-11-2: Synthesis of compound A103-03R

[Formula 140]

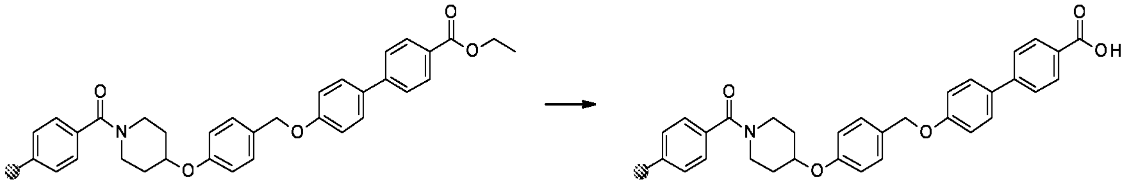

A102-03R A103-03R

Under a nitrogen atmosphere, compound A102-03R (loading rate: 0.200 mmol/g, 47.9 g), NMP (503 mL), and 2-methyl-2-butanol (144 mL) were added to a 2 L separable flask and stirred at room temperature for 1 hour. The suspension was cooled to 5°C. Then, an aqueous normal tetrabutylammonium hydroxide solution (1 M, 14.4 mL, 14.4 mmol) was added dropwise thereto, and the mixture was stirred at room temperature for 3.5 hours. An aqueous normal tetrabutylammonium hydroxide solution (1 M, 2.39 mL, 2.39 mmol) was added dropwise thereto, and the mixture was stirred at room temperature for 1 hour. This operation was repeated twice.

Solid-phase purification

The suspension of the reaction solution and the resin was washed three times with HOAt (0.1 M in NMP, 800 mL), three times with NMP (800 mL), three times with MeOH (800 mL), three times with DCM (800 mL), and three times with heptane (800 mL), and the obtained solid phase was dried under reduced pressure for 6 days to obtain compound A103- 3R (loading rate: 0.201 mmol/g, 49.8 g, 10.0 mmol).

Reaction monitoring

The suspension of the reaction solution and the resin (12 μL) was transferred onto a filter, washed three times with NMP (0.1 mL), three times with MeOH (0.1 mL), and three times with DCM (0.1 mL), and then dipped in a 10% solution of TFA in DCM containing 0.02 M pentamethylbenzene (0.05 mL) for 5 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 100% of compound A103 of interest was observed.

[Formula 141]

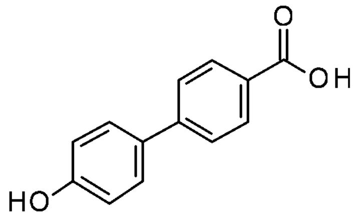

Compound A103

Maximum wavelength: 294 nm

Retention time: 0.761 min (analysis conditions FA05-1, 299 nm).

Example 1-11-3: Synthesis of compound A130-03R

[Formula 142]

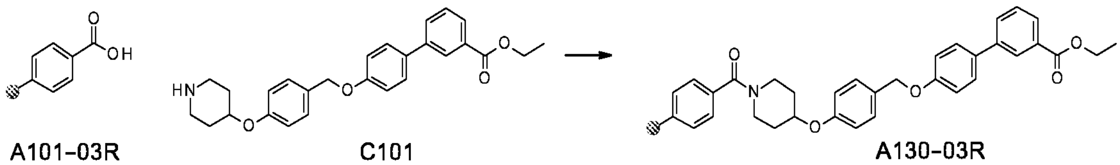

A101-03R C101 A130-03R

Compound A130-03R (loading rate: 0.200 mmol/g) can be synthesized by the same approach as in compound A102-03R using compound A101-03R (loading rate: 1.70 mmol/g) and 3-[4-[(4-piperidin-4-yloxyphenyl)methoxy]phenyl]benzoate (C101).

Example 1-11-4: Synthesis of compound A131-03R

[Formula 143]

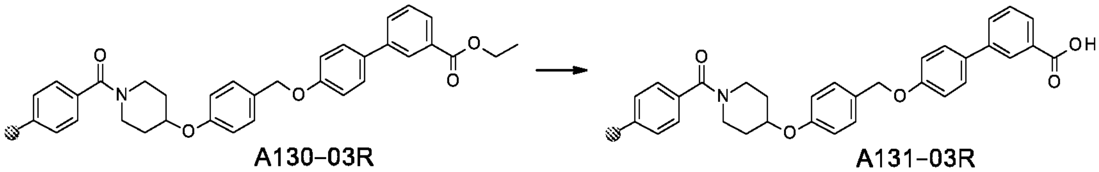

A130-03R A131-03R

Compound A131-03R (loading rate: 0.201 mmol/g) can be synthesized by the same approach as in compound A103-03R using compound A130-03R (loading rate: 0.200 mmol/g).

A131-03R was dipped in a 10% solution of TFA in DCM containing 0.02 M pentamethylbenzene (0.05 mL) for 5 minutes. DMF (0.05 mL) and MeCN (0.25 mL) were added thereto, and the mixture was filtered. The filtrate was subjected to LC-MS analysis, so that 100% of compound A131 of interest was observed.

[Formula 144]

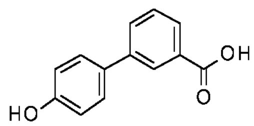

Compound A131

Maximum wavelength: 266 nm

Retention time: 0.797 min (analysis conditions FA05-1, 299 nm).

Example 1-11-4: Synthesis of methyl 2-[3-bromo-5-hydroxyphenyl]propanoate (D084)

[Formula 145]

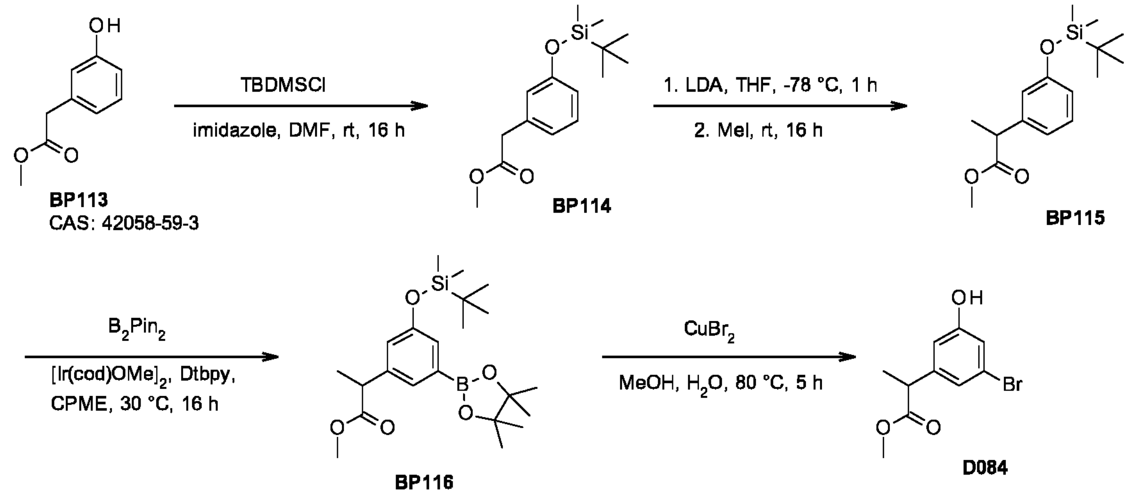


BP113
CAS: 42058-59-3
BP114
BP115
BP116
D084

First step: Synthesis of methyl 2-[3-[tert-butyl(dimethyl)silyl]oxyphenyl]acetate: compound BP114

Under a nitrogen atmosphere, methyl 2-(3-hydroxyphenyl)acetate (compound BP113, CAS: 42058-59-3) (15.0 g, 90.3 mmol, 1.0 eq), imidazole (18.4 g, 270.8 mmol, 3.0 eq), and DMF (150 mL) were added to a 500 mL flask and stirred at room temperature for 10 minutes. TBDMSCl (20.4 g, 135.4 mmol, 1.5 eq) was added dropwise to this reaction mixture. This reaction mixture was stirred at room temperature for 16 hours and diluted with ethyl acetate (450 mL). The obtained organic layer was washed three times with water (150 mL) and dried over $Na_2SO_4$. After filtration of the solid, the filtrate was concentrated under reduced pressure. The residue was purified by silica gel column chromatography (ethyl acetate/petroleum ether, 0-20%) to obtain methyl 2-[3-[tert- butyl(dimethyl)silyl]oxyphenyl]acetate (compound BP114) as a colorless oil (24.4 g, 93.5%).
LCMS (ESI, m/z): 281[M+H]$^+$.
Retention time: 0.825 min.
Analysis conditions: PH-FA-15.

Second step: Synthesis of methyl 2-[3-[tert- butyl(dimethyl)silyl]oxyphenyl]propanoate: compound BP115

Under a nitrogen atmosphere, methyl 2-[3-[tert- butyl(dimethyl)silyl]oxyphenyl]acetate (compound BP114) (16.4 g, 58.5 mmol, 1.0 eq) and THF (550 mL) were added to a 500 mL flask and cooled to -78°C. LDA (a 2 M solution in THF, 38 mL, 76.0 mmol, 1.3 eq) was added dropwise to this reaction solution, and the mixture was stirred at -78°C for 1 hour. MeI (10.0 g, 70.2 mmol, 1.2 eq) was added at -78°C to the obtained reaction solution, and the reaction mixture was stirred at room temperature for 16 hours. The reaction was terminated with water (200 mL), followed by extraction with ethyl acetate (400 mL) three times. The organic layers were mixed and dried over $Na_2SO_4$. After filtration of the solid, the filtrate was concentrated under reduced pressure. The residue was purified by silica gel column chromatography (ethyl acetate/petroleum ether, 0-12%) to obtain methyl 2-[3-[tert-butyl(dimethyl)silyl]oxyphenyl]propanoate (compound BP115) as a yellow oil (16.2 g, 89.2%).
LCMS (ESI, m/z): 295[M+H]$^+$.
Retention time: 0.851 min.
Analysis conditions: PH-FA-15.

Third step: Synthesis of methyl 2-[3-[tert-butyl(dimethyl)silyl]oxy-5-(4,4,5,5- tetramethyl-1,3,2-dioxaboloran-2-yl)phenyl]propanoate: compound BP116

Under a nitrogen atmosphere, methyl 2-[3-[tert- butyl(dimethyl)silyl]oxyphenyl]propanoate (compound BP115) (10.0 g, 34.0 mmol, 1.0 eq), BzPinz (8.6 g, 34.0 mmol, 1.0 eq), $[Ir(cod)OMe]_2$ (2.3 g, 3.4 mmol, 0.1 eq), Dtbpy (1.8 g, 6.8 mmol, 0.2 eq), and CPME (cyclopentyl methyl ether) (200 mL) were added to a 500 mL flask. This reaction mixture was stirred at 30°C for 16 hours. The reaction mixture was concentrated under reduced pressure, and the residue was purified by silica gel column chromatography (ethyl acetate/petroleum ether, 0-30%) and reverse-phase silica gel column chromatography (C18) ($CH_3CN$/water, 30-80%) to obtain methyl 2-[3-[tert- butyl(dimethyl)silyl]oxy-5-(4,4,5,5-tetramethyl-1,3,2-dioxaboloran-2-yl)phenyl]propanoate (compound BP116) as a pale yellow oil (6.6 g, 41.4%).
LCMS (ESI, m/z): 421[M+H]$^+$.
Retention time: 0.883 min.

Analysis conditions: PH-FA-15.

Fourth step: Synthesis of methyl 2-[3-bromo-5-hydroxyphenyl]propanoate: compound D084

Under a nitrogen atmosphere, methyl 2-[3-[tert-butyl(dimethyl)silyl]oxy-5-(4,4,5,5- tetramethyl-1,3,2-dioxaborolan-2-yl)phenyl]propanoate (compound BP116) (3.9 g, 9.3 mmol, 1.0 eq), $CuBr_2$ (6.2 g, 27.8 mmol, 3.0 eq), MeOH (60 mL), and water (60 mL) were added to a 300 mL flask. This reaction mixture was stirred at 80°C for 5 hours. The reaction mixture was diluted with ethyl acetate (250 mL) to separate an organic layer. The organic layer was washed with a saturated aqueous solution of NaCl (100 mL) and dried over $Na_2SO_4$. After filtration of the solid, the filtrate was concentrated under reduced pressure. The residue was purified by reverse-phase silica gel column chromatography (C18) ($CH_3CN$/water, 5-55%) to obtain methyl 2-[3-bromo-5-hydroxyphenyl]propanoate (compound D084) as a colorless oil (0.9 g, 36.6%).
$^1$H-NMR (400 MHz, DMSO-$d_6$) δ: 9.92 (s, 1H), 6.88 (t, J = 1.6 Hz, 1H), 6.84 (t, J = 2.0 Hz, 1H), 6.67 (t, J = 2.0 Hz, 1H), 3.73 (q, J = 7.2 Hz, 1H), 3.59 (s, 3H), 1.34 (d, J = 7.2 Hz, 3H).
LCMS (ESI, m/z): 257, 259 [M-H]+.
Retention time: 0.926 min.
Analysis conditions: PH-FA-16.

Example 1-11-5: Synthesis of tert-butyl 4-[4-[[3-bromo-5-(1-methoxy-1-oxopropan- 2-yl)phenoxy]methyl]phenoxy]piperidine-1-carboxylate (D088)

[Formula 146]

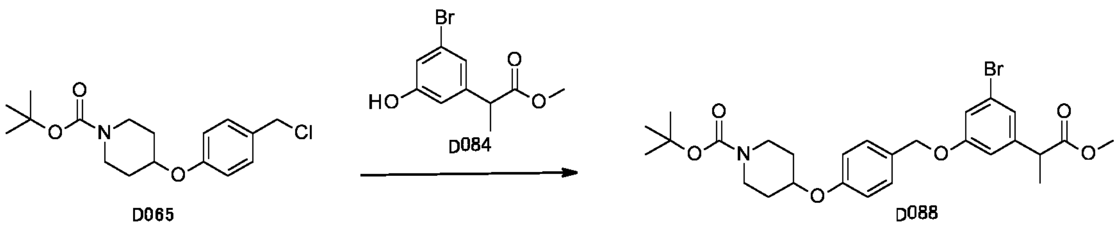

D065 D084 D088

A solution of known compound D065 (3.01 g, 9.22 mmol, 63%) in NMP (23.9 mL) was cooled to 0°C. Then, methyl 2-(3-bromo-5-hydroxyphenyl)propanoate (D084, 2.39 g, 9.22 mmol) and cesium carbonate (3.61 g, 11.1 mmol) were added thereto, and the mixture was stirred at room temperature for 20 hours. An aqueous formic acid solution (50%, 10 mL) was added to the obtained mixture, and the resulting mixture was purified by reverse-phase silica gel column chromatography (0.1% solution of formic acid in acetonitrile/0.1% aqueous formic acid solution, 10-80%) to obtain the title compound D088 (4.93 g, 8.99 mmol, 95%) in a colorless oil state.
$^1$H-NMR (400 MHz, $CDCl_3$) δ 7.33 (2H, d, J = 8.6 Hz), 7.04-7.02 (2H, m), 6.91 (2H, d, J = 8.6 Hz), 6.85 (1H, m), 4.94 (2H, s), 4.51-4.45 (1H, m), 3.71 (2H, dd, J = 8.3, 4.6 Hz), 3.67 (3H, s), 3.63 (1H, q, J = 7.3 Hz), 3.34 (2H, ddd, J = 13.4, 4.0, 2.0 Hz), 1.95-1.89 (1H, m), 1.79-1.72 (2H, m), 1.47 (9H, s), 1.45 (3H, d, J = 10 Hz).
LCMS: m/z570, 572 [M+Na]$^+$.
Retention time: 1.589 min (analysis conditions SMD-FA05-1)

Example 1-11-6: Synthesis of methyl 2-[3-bromo-5-[(4-piperidin-4- yloxyphenyl)methoxy]phenyl]propanoate (D089)

[Formula 147]

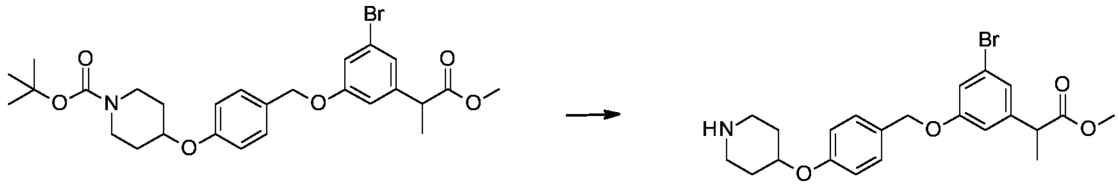

D088

D089

A solution of tert-butyl 4-[4-[[3-bromo-5-(1-methoxy-1-oxopropan-2- yl)phenoxy]methyl]phenoxy]piperidine-1-carboxylate (D088, 4.82 g, 8.79 mmol) in 4- MeTHP (48.2 mL) was cooled to 0°C, and HMDS (5.53 mL, 26.4 mmol) was added thereto. $Sn(OTf)_2$ (7.33 g, 17.6 mmol) was added thereto, and the mixture was stirred at room temperature for 1.5 hours. The mixture was concentrated under reduced pressure, and the obtained residue was purified by silica gel column chromatography (NH-silica gel, dichloromethane/hexane, 0-100%, and methanol/dichloromethane, 1-3%) to obtain the title compound D089 (3.89 g, 8.67 mmol, 99%) in a colorless oil state.

$^1$H-NMR (400 MHz, $CDCl_3$) δ: 7.32 (2H, d, J = 9.1 Hz), 7.04-7.02 (2H, m), 6.92 (2H, d, J = 8.6 Hz), 6.85-6.84 (1H, m), 4.94 (2H, s), 4.42-4.36 (1H, m), 3.66 (3H, s), 3.63 (1H, q, J = 7.3 Hz), 3.18-3.12 (2H, m), 2.78-2.71 (2H, m), 2.04-2.00 (2H, m), 1.72-1.64 (2H, m), 1.46 (3H, d, J = 7.0 Hz).

LCMS: m/z448, 450 $[M+H]^+$.

Retention time: 0.863 min (analysis conditions SMD-FA05-1).

Example 1-11-7: Synthesis of compound D084-03R

[Formula 148]

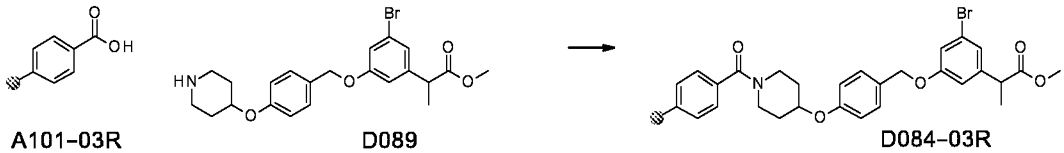

A101-03R D089 D084-03R

Compound D084-03R (loading rate: 0.200 mmol/g) can be synthesized by the same approach as in compound A102-03R using compound A101-03R (loading rate: 1.70 mmol/g) and methyl 2-[3-bromo-5-[(4-piperidin-4-yloxyphenyl)methoxy]phenyl]propanoate (D089).

D084-03R was dipped in a 10% solution of TFA in DCM containing 0.05 M pentamethylbenzene (0.10 mL) for 5 minutes. NMP (0.10 mL) and MeCN (0.25 mL) were added thereto, and the mixture was filtered. The filtrate was subjected to LC-MS analysis, so that 99.2% of compound D084 of interest was observed.

[Formula 149]

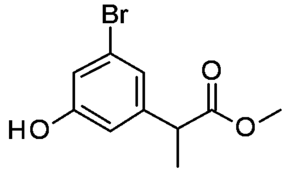

Compound D084

Maximum wavelength: 278 nm

Retention time: 0.983 min (analysis conditions FA05-1, 277 nm).

Example 1-11-8: Synthesis of compound D127-03R

[Formula 150]

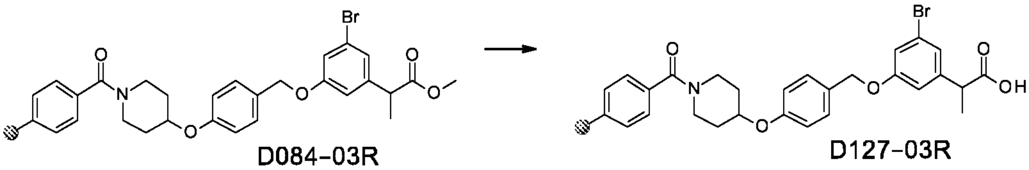

D084-03R D127-03R

Compound D127-03R (loading rate: 0.201 mmol/g) can be synthesized by the same approach as in compound A103-03R using compound D084-03R (loading rate: 0.200 mmol/g).

Under a nitrogen atmosphere, solid-phase supported ester (compound D084-03R) (11.58 g, 0.200 mmol/g), NMP (162 mL), and tAmylOH (46.3 mL) were added to a 250 mL column with a filter and shaken at room temperature for 1 hour. A 1 M aqueous TBAOH solution (4.63 mL, 4.63 mmol) was added thereto, and the mixture was shaken at room temperature for 7.5 hours.

Solid-phase purification

The suspension of the reaction solution and the resin was filtered through the filter of the column and washed three times with NMP/water = 1/1 (208 mL), three times with NMP (208 mL), three times with 0.05 M $TBAHSO_4$ + DTBP in NMP (208 mL), three times with NMP/water = 1/1 (208 mL), three times with NMP (208 mL), three times with MeOH (208 mL), three times with DCM (208 mL), and three times with heptane (208 mL), and the obtained solid phase was dried under reduced pressure to obtain the title compound D127- 03R (13.8 g, 0.201 mmol/g).

Reaction monitoring

The suspension of the reaction solution and the resin (0.015 mL) was transferred onto a filter, washed three times with DMA (0.1 mL), three times with methanol (0.1 mL), and three times with DCM (0.1 mL), and then dipped in a 10% solution of TFA in DCM containing 0.05 M pentamethylbenzene (0.05 mL) for 5 minutes. After filtration, the resin on the filter was washed with DMA (0.05 mL). Acetonitrile (0.10 mL) was added to the obtained filtrate to prepare an LC sample. The degree of progression of the reaction was measured by LC-MS analysis. As a result, compound D127 of interest was observed at a purity of 98.4%.

[Formula 151]

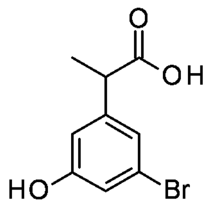

D127

Compound D127

Maximum wavelength: 278 nm.

Retention time: 0.807 min (analysis conditions SMD-FA05-1,280 nm).

Example 1-11-9: Synthesis of methyl 5-[tert-butyl(dimethyl)silyl]oxy-1,2,3,4- tetrahydronaphthalene-1-carboxylate (BP124)

[Formula 152]

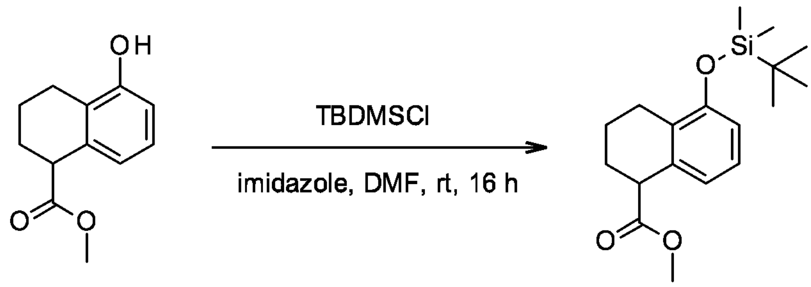


BP123 BP124

To a solution of known compound methyl 5-hydroxy-1,2,3,4-tetrahydronaphthalene- 1-carboxylate (BP123) (19 g, 92.1 mmol) and imidazole (22.6 g, 331.7 mmol) in DMF (200 ml), TBSCl (25.0 g, 165.8 mmol) dissolved in DMF (200 mL) was added at room temperature. After stirring at room temperature for 16 hours, water was added thereto, followed by extraction with ethyl acetate three times. The obtained organic layer was washed with saturated sodium chloride aqueous solution, then dried over $Na_2SO_4$, and concentrated under reduced pressure. The residue was purified by silica gel column chromatography (ethyl acetate/petroleum ether, 0-20%) to obtain the title compound BP124 (25 g, 83.8%).
LCMS (ESI, m/z): 321[M+H]+.
Retention time: 1.250 min.
Analysis conditions: PH-TFA-23.

Example 1-11-10: Synthesis of methyl 5-[tert-butyl(dimethyl)silyl]oxy-7-(4,4,5,5- tetramethyl-1,3,2-dioxaboloran-2-yl)-1,2,3,4-tetrahydronaphthalene-1-carboxylate (BP125)

[Formula 153]

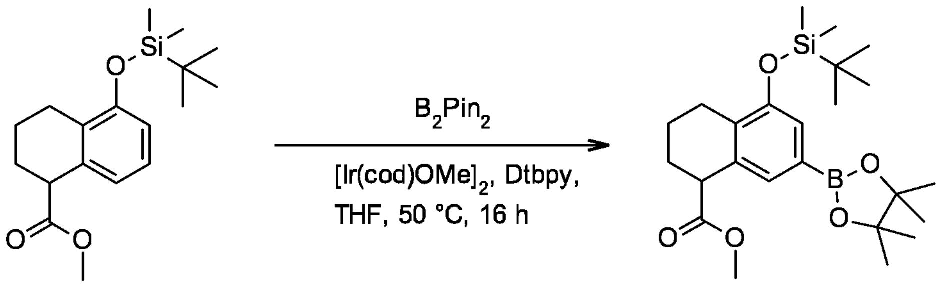


BP124 BP125

Under a nitrogen atmosphere, a solution of methyl 5-[tert-butyl(dimethyl)silyl]oxy- 1,2,3,4-tetrahydronaphthalene-1-carboxylate (BP124) (25 g, 78.0 mmol), bis(pinacolato)diboron (39.6 g, 156.0 mmol), $[Ir(COD)OMe]_2$ (1.3 g, 2.0 mmol), and dtbbpy (1.1 g, 3.9 mmol) in THF (300 mL) was stirred at 50°C for 16 hours. Then, water was added thereto, followed by extraction with ethyl acetate three times. The obtained organic layer was washed with saturated sodium chloride aqueous solution, then dried over $Na_2SO_4$, and concentrated under reduced pressure. The residue was purified by silica gel column chromatography (dichloromethane/petroleum ether, 0-100%) to obtain the title compound BP125 (28 g, 76.4%).
LCMS (ESI, m/z): 447[M+H]+.
Retention time: 1.337 min.
Analysis conditions: PH-TFA-23.

Example 1-11-11: Synthesis of methyl 7-bromo-5-hydroxy-1,2,3,4- tetrahydronaphthalene-1-carboxylate (D085)

[Formula 154]

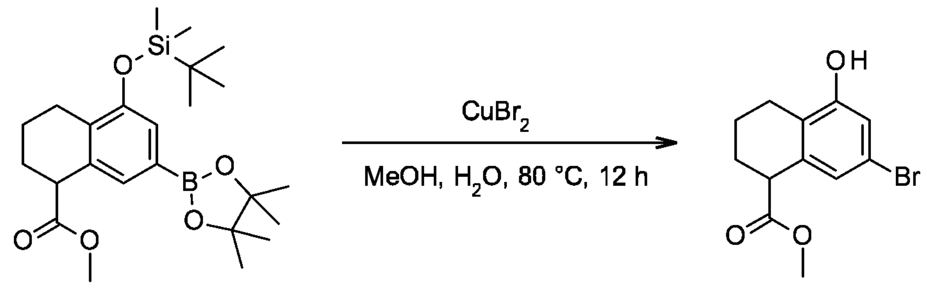


BP125 D085

A solution of methyl 5-[tert-butyl(dimethyl)silyl]oxy-7-(4,4,5,5-tetramethyl-1,3,2- dioxaboloran-2-yl)-1,2,3,4-tetrahydronaphthalene-1-carboxylate (BP125) (28 g, 62.7 mmol) and copper(II) bromide (42.0 g, 188.1 mmol) in water (560 mL)-methanol (560 mL) was stirred at 80°C for 12 hours and then diluted with ethyl acetate. The organic layer was washed with saturated sodium chloride aqueous solution, then dried over $Na_2SO_4$, and concentrated under reduced pressure. The residue was purified by reverse-phase silica gel column chromatography (acetonitrile/water, 5-55%) to obtain the title compound D085 (12.6 g, 60.8%).

LCMS (ESI, m/z): 283[M-H]$^+$.

Retention time: 0.995 min.

Analysis conditions: PH-FA-16.

$^1$HNMR (300 MHz, DMSO-d6) δ 9.84 (s, 1H), 6.81 (d, J = 2.1 Hz, 1H), 6.71 (d, J = 2.1, 1H), 3.78 (t, J = 5.4 Hz, 1H), 3.62 (s, 3H), 2.47-2.34 (m, 2H), 2.03-1.89 (m, 1H), 1.88- 1.76 (m, 1H), 1.72-1.64 (m, 2H).

Example 1-11-12: Synthesis of tert-butyl 4-[4-[(3-bromo-5-methoxycarbonyl- 5,6,7,8-tetrahydronaphthalen-1-yl)oxymethyl]phenoxy]piperidine-1-carboxylate (D086)

[Formula 155]

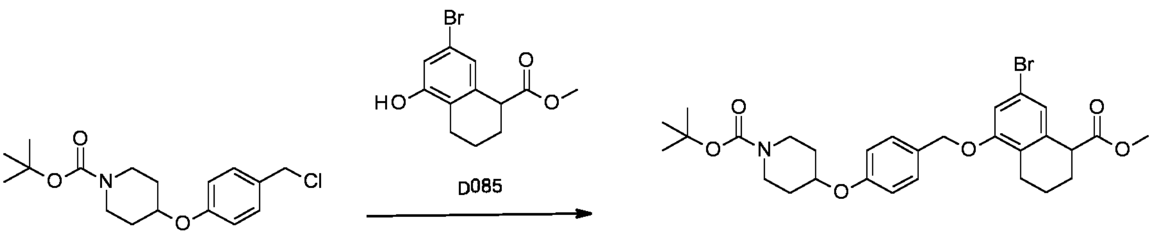

D065 D085 D086

A solution of tert-butyl 4-[4-(chloromethyl)phenoxy]piperidine-1-carboxylate (D065) (2.41 g, 7.41 mmol), methyl 7-bromo-5-hydroxy-1,2,3,4-tetrahydronaphthalene-1- carboxylate (D085) (2.11 g, 7.41 mmol), and cesium carbonate (2.66 g, 8.15 mmol) in NMP (27.4 ml) was stirred overnight at room temperature. The reaction solution was cooled to 0°C. Then, an aqueous formic acid solution was added thereto, and the mixture was purified by reverse-phase silica gel column chromatography (0.1% solution of formic acid in acetonitrile/0.1% aqueous formic acid solution, 10-80%) to obtain the title compound D086 (3.7 g, 7.4 mmol, 87%).

$^1$H-NMR (400 MHz, $CDCl_3$) δ: 7.33 (2H, d, J = 8.6 Hz), 6.95-6.92 (4H, m), 4.94 (2H, s), 4.51-4.45 (1H, m), 3.77 (1H, t, J = 5.4 Hz), 3.73 (3H, s), 3.70-3.69 (2H, m), 3.37- 3.31 (2H, m), 2.73-2.55 (2H, brm), 2.11-2.09 (1H, m), 1.92-1.90 (4H, m), 1.77-1.75 (3H, m), 1.47 (9H, s).

LRMS (ESI): m/z572, 574 [M+H]$^+$.

Retention time: 1.651 min (analysis conditions SMD-FA05-1).

Example 1-11-13: Synthesis of methyl 7-bromo-5-[(4-piperidin-4- yloxyphenyl)methoxy]-1,2,3,4-tetrahydronaphthalene-1-carboxylate (D087)

[Formula 156]

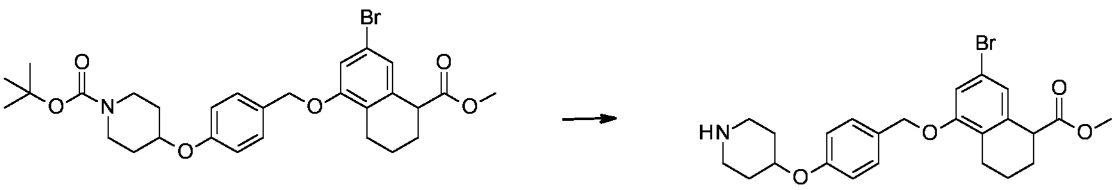

D086

D087

tert-Butyl 4-[4-[(3-bromo-5-methoxycarbonyl-5,6,7,8-tetrahydronaphthalen-1 - yl)oxymethyl]phenoxy]piperidine-1-carboxylate (D086, 4.69 g, 8.16 mmol) and 4-MeTHP (46.9 mL) were added to a 200 mL flask, and the reaction container was cooled to 0°C. HMDS (5.20 mL, 24.5 mmol) and $Sn(OTf)_2$ (6.81 g, 16.3 mmol) were added thereto, and the mixture was stirred at room temperature for 1 hour. The mixture was concentrated under reduced pressure, and the obtained residue was purified by silica gel column chromatography (NH-silica gel, dichloromethane/hexane, 0-100%, and methanol/dichloromethane, 1-2%) to obtain the title compound D087 (3.33 g, 7.02 mmol, 86%).

$^1$H-NMR (400 MHz, $CDCl_3$)7.32 (2H, d, J = 8.6 Hz), 6.94-6.91 (4H, m), 4.94 (2H, s), 4.40-4.36 (1H, m), 3.77 (1H, t, J = 5.1 Hz), 3.72 (3H, s), 3.15 (2H, td, J = 8.9, 4.3 Hz), 2.76-2.55 (4H, m), 2.14-1.63 (8H, m).

LCMS: m/z472, 474 $[M+H]^+$.

Retention time: 0.906 min (analysis conditions SMD-FA05-1).

Example 1-11-14: Synthesis of compound D085-03R

[Formula 157]

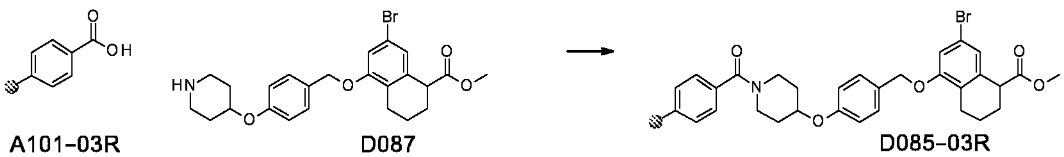

A101-03R D087 D085-03R

Compound D085-03R (loading rate: 0.200 mmol/g) can be synthesized by the same approach as in compound A102-03R using compound A101-03R (loading rate: 1.70 mmol/g) and methyl 7-bromo-5-[(4-piperidin-4-yloxyphenyl)methoxy]-1,2,3,4-tetrahydronaphthalene- 1-carboxylate (D087).

D085-03R was dipped in a 10% solution of TFA in DCM containing 0.05 M pentamethylbenzene (0.10 mL) for 5 minutes. NMP (0.10 mL) and MeCN (0.25 mL) were added thereto, and the mixture was filtered. The filtrate was subjected to LC-MS analysis, so that 100% of compound D085 of interest was observed.

[Formula 158]

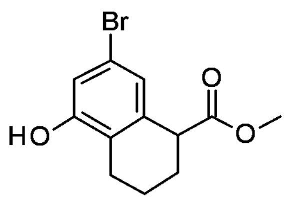

Compound D085

LRMS: m/z 285, $287[M+H]^+$

Retention time: 1.051 min (analysis conditions FA05-1,277 nm).

Example 1-11-15: Synthesis of compound D128-03R

[Formula 159]

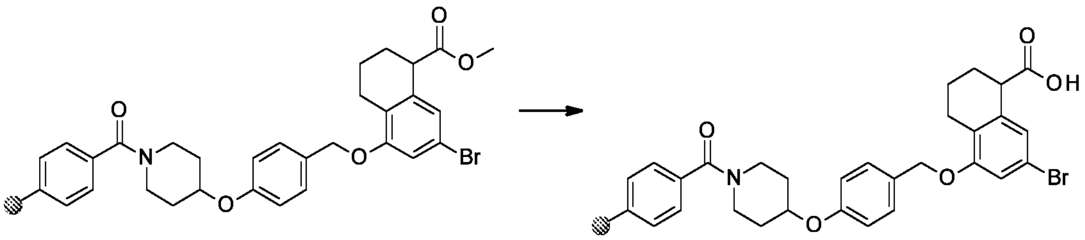

D085-03R

D128-03R

The title compound D128-03R (12.8 g, 0.201 mmol/g) was obtained by synthesis by the same approach as in Example 1-11-8 using solid-phase supported ester (compound D085- 03R) (11.28 g, 0.200 mmol/g). The same cleavage operation as in Example 1-11-8 was performed, so that 97.6 area% of compound D128 of interest was observed.

[Formula 160]

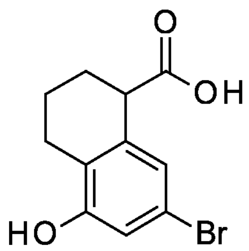

D128

Compound D128
Maximum wavelength: 285 nm.
Retention time: 0.831 min (analysis conditions SMD-FA05-1, 280 nm).

Example 1-11-16: Synthesis of prop-2-enyl 4-[4- (hydroxymethyl)phenoxy]piperidine-1-carboxylate (D078)

[Formula 161]

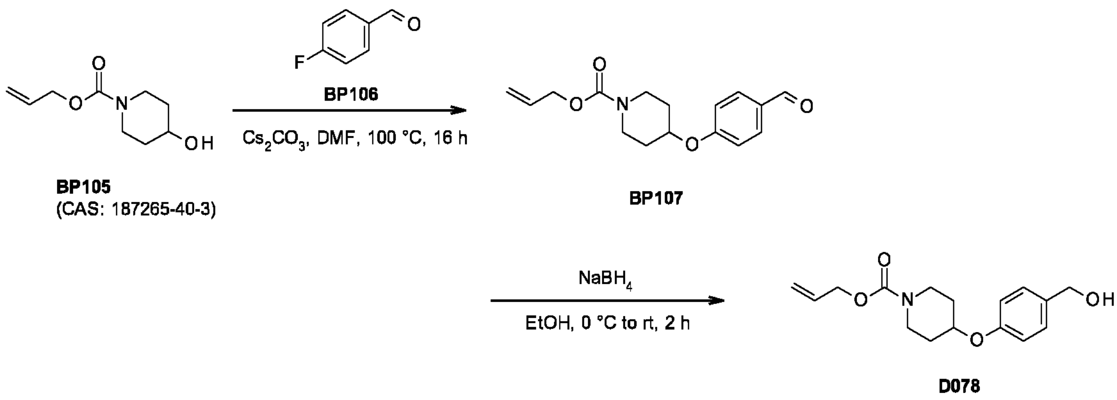

First step: Synthesis of prop-2-enyl 4-(4-formylphenoxy)piperidine-1-caxboxylate: compound BP107

Under a nitrogen atmosphere, 4-fluorobenzaldehyde (compound BP106, CAS: 459- 57-4) (53.1 g, 427.4 mmol, 1.0 eq), prop-2-enyl 4-piperidine-1-carboxylate (compound BP105, CAS: 187265-40-3) (95.0 g, 512.9 mmol, 1.2 eq), $Cs_2CO_3$ (334.2 g, 1025.8 mmol, 2.4 eq), and DMF (480 mL) were added to a 1 L flask. This reaction mixture was stirred at 100°C for 16 hours. The reaction mixture was cooled to room temperature, and water (400 mL) was added thereto, followed by extraction with ethyl acetate (500 mL) three times. The organic layers were mixed, washed with a saturated NaCl solution (500 mL), and dried over $Na_2SO_4$. After filtration of the solid, the filtrate was concentrated under reduced pressure. The residue was purified by silica gel column chromatography (ethyl acetate/petroleum ether, 0-50%) to obtain prop-2-enyl 4-(4-formylphenoxy)piperidine-1- carboxylate (compound BP107) as a pale yellow oil (89.4 g, 61.5%).
LCMS (ESI, m/z): 290$[M+H]^+$.
Retention time: 0.704 min.
Analysis conditions: PH-TFA-22.

Second step: Synthesis of prop-2-enyl 4-[4-(hydroxymethyl)phenoxy]piperidine-1- carboxylate: compound D081

Under a nitrogen atmosphere, prop-2-enyl 4-(4-formylphenoxy)piperidine-1- carboxylate (compound BP107) (89.4 g, 309.0 mmol, 1.0 eq) and EtOH (900 mL) were added to a 3 L flask. $NaBH_4$ (23.4 g, 618.0 mmol, 2.0 eq) was added in small portions to this reaction mixture under ice cooling, and the obtained reaction mixture was stirred at room temperature for 2 hours. The reaction was terminated by the addition of water (200 mL), and the reaction mixture was filtered. The solid collected by filtration was washed with water (200 mL) and further washed through dioxane (300 mL) and hexane (300 mL) to obtain prop-2-enyl 4-[4-(hydroxymethyl)phenoxy]piperidine-1-carboxylate (compound D081) as a white solid (70.7 g, 76.2%).
$^1$H-NMR (300 MHz, DMSO-$d_6$) δ: 7.22 (d, J = 8.4 Hz, 2H), 6.91 (d, J = 8.4 Hz, 2H), 5.98-5.85 (m, 1H), 5.34-5.12 (m, 2H), 5.02 (t, J = 5.7 Hz, 1H), 4.67-4.50 (m, 3H), 4.39 (d, J = 5.7 Hz, 2H), 3.73-3.66 (m, 2H), 3.33-3.21 (m, 2H), 1.94-1.84 (m, 2H), 1.58-1.46 (m, 2H).
LCMS (ESI, m/z): 292 $[M+H]^+$.
Retention time: 0.797 min.
Analysis conditions: PH-TFA-22.

Example 1-11-17: Synthesis of 2-O-tert-butyl 8-O-ethyl 6-hydroxy-3,4-dihydro-1H- isoquinoline-2,8-dicarboxylate (D082)

[Formula 162]

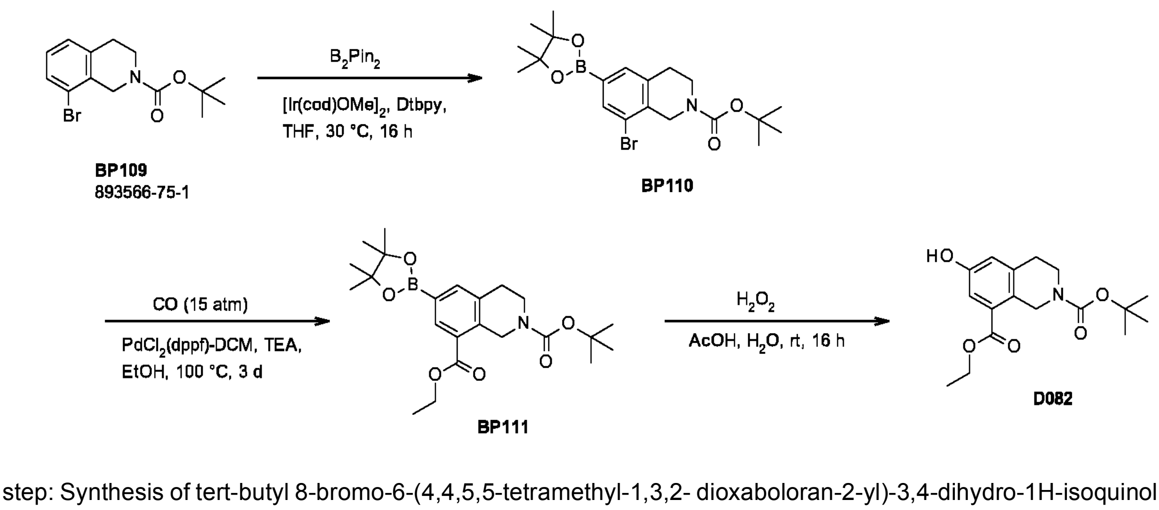


First step: Synthesis of tert-butyl 8-bromo-6-(4,4,5,5-tetramethyl-1,3,2- dioxaboloran-2-yl)-3,4-dihydro-1H-isoquinoline-2-carboxylate: compound BP110

Under a nitrogen atmosphere, tert-butyl 8-bromo-3,4-dihydro-1H-isoquinoline-2- carboxylate (compound BP109, CAS: 893566-75-1) (24.0 g, 76.9 mmol, 1.0 eq), BzPinz (19.5 g, 76.9 mmol, 1.0 eq), $[Ir(cod)OMe]_2$ (5.1 g, 7.7 mmol, 0.1 eq), Dtbpy (4.1 g, 15.4 mmol, 0.2 eq), and THF (240 mL) were added to a 500 mL flask. This reaction mixture was stirred at 30°C for 16 hours. The reaction mixture was concentrated under reduced pressure, and the residue was

purified by silica gel column chromatography (ethyl acetate/petroleum ether, 0-15%) and reverse-phase silica gel column chromatography (C18) ($CH_3CN$/water, 30-80%) to obtain tert-butyl 8-bromo-6-(4,4,5,5-tetramethyl-1,3,2- dioxaboloran-2-yl)-3,4-dihydro-1H-isoquinoline-2-carboxylate (compound BP110) as a brown oil (24.0 g, 60.6%).

LCMS (ESI, m/z): 423,425$[M+H]^+$.

Retention time: 1.070 min.

Analysis conditions: PH-FA-12.

Second step: Synthesis of 2-O-tert-butyl 8-O-ethyl 6-(4,4,5,5-tetramethyl-1,3,2- dioxaboloran-2-yl)-3,4-dihydro-1H-isoquinoline-2,8-dicarboxylate: compound BP111

Under a nitrogen atmosphere, tert-butyl 8-bromo-6-(4,4,5,5-tetramethyl-1,3,2- dioxaboloran-2-yl)-3,4-dihydro-1H-isoquinoline-2-carboxylate (compound BP110) (24.0 g, 54.8 mmol, 1.0 eq), $PdCl_2$(dppf)-DCM (6.7 g, 8.2 mmol, 0.15 eq), TEA (16.6 g, 164.3 mmol, 3.0 eq), and EtOH (3.7 L) were added to a 5 L reaction container. This reaction mixture was stirred at 100°C for 3 days in a 15-atm CO atmosphere. After removal of the CO gas, the reaction mixture was concentrated under reduced pressure, and the residue was purified by silica gel column chromatography (ethyl acetate/petroleum ether, 0-30%) to obtain tert-butyl 8-bromo-6-(4,4,5,5-tetramethyl-l,3,2-dioxaboloran-2-yl)-3,4-dihydro-1H-isoquinoline-2- carboxylate (compound BP111) as a brown oil (21.0 g, 38.2%).

LCMS (ESI, m/z): 432$[M+H]^+$.

Retention time: 0.991 min.

Analysis conditions: PH-FA-12.

Third step: Synthesis of 2-O-tert-butyl 8-O-ethyl 6-hydroxy-3,4-dihydro-1H- isoquinoline-2,8-dicarboxylate: compound D082

Under a nitrogen atmosphere, 2-O-tert-butyl 8-O-ethyl 6-(4,4,5,5-tetramethyl-1,3,2- dioxaboloran-2-yl)-3,4-dihydro-1H-isoquinoline-2,8-dicarboxylate (compound BP111) (22.0 g, 51.0 mmol, 1.0 eq), AcOH (50 mL), and water (50 mL) were added to a 500 mL flask. Hydrogen peroxide water (33 mL, 970.2 mmol, 19.0 eq) was added dropwise to this reaction mixture at room temperature. The obtained reaction mixture was stirred at room temperature for 16 hours and diluted with water (100 mL). The reaction mixture was rendered basic (pH to 8) with a saturated aqueous solution of sodium carbonate (200 mL), and a solid was filtered. The solid collected by filtration was purified by reverse-phase silica gel column chromatography (C18) ($CH_3CN$/water, 30-50%) and further purified by suspension in hexane (300 mL) and subsequently in water (200 mL) to obtain 2-O-tert-butyl 8-O-ethyl 6-hydroxy-3,4-dihydro-1H-isoquinoline-2,8-dicarboxylate (compound D082) as a yellow solid (7.3 g, 42.4%).

$^1$H-NMR (400 MHz, DMSO-$d_6$) $\delta$: 9.64 (s, 1H), 7.18 (s, 1H), 6.79 (s, 1H), 4.69 (s, 2H), 4.30-4.25 (m, 2H), 3.51-3.48 (m, 2H), 2.76 (d, J = 6.4 Hz, 2H), 1.40 (s, 9H), 1.33-1.29 (m, 3H).

LRMS (ESI): m/z 320 $[M-H]^+$.

Retention time: 1.039 min.

Analysis conditions: PH-FA-13.

Example 1-11-18: Synthesis of 2-O-tert-butyl 8-O-ethyl 6-[[4-(1-prop-2- enoxycarbonylpiperidin-4-yl)oxyphenyl]methoxy]-3,4-dihydro-1H-isoquinoline-2,8- dicarboxylate (D076)

[Formula 163]

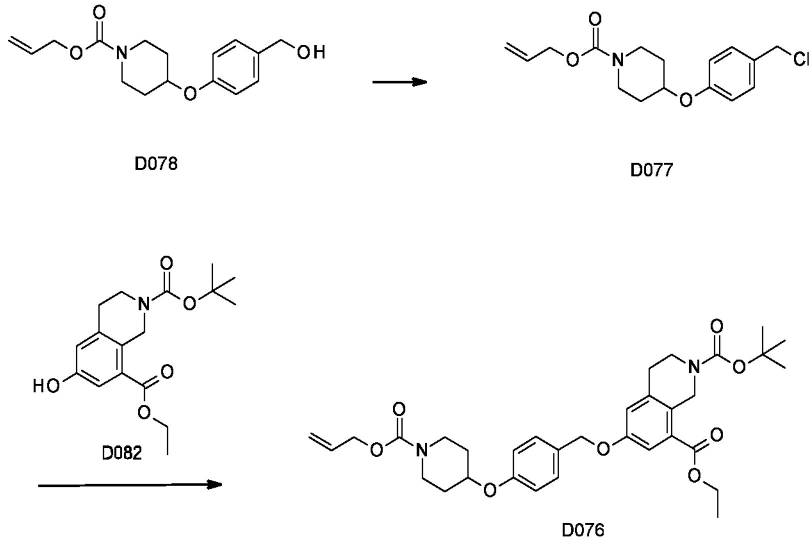

D078 D077 D082 D076

A solution of prop-2-enyl 4-[4-(hydroxymethyl)phenoxy]piperidine-1-carboxylate (D078) (5.28 g, 18.1 mmol) and lithium chloride (1.54 g, 36.2 mmol) in DMI (52.8 ml) was cooled to 0°C. Then, 2,6-lutidine (6.33 mL, 54.3 mmol) was added thereto. Methanesulfonic anhydride (4.73 g, 27.2 mmol) was added thereto, and the mixture was stirred at 0°C for 15 minutes and then stirred at room temperature for 4 hours. Water (300 mL), ethyl acetate (250 mL), and hexane (200 mL) were added to the obtained mixture. The obtained organic layer was washed with 0.5 M hydrochloric acid (200 mL) and a half-saturated aqueous solution of sodium chloride (200 mL). The organic layer was dried over sodium sulfate and then concentrated under reduced pressure. The obtained residue was added to a solution of 2- O-tert-butyl 8-O-ethyl 6-hydroxy-3,4-dihydro-1H-isoquinoline-2,8-dicarboxylate (D082) (4.59 g, 14.3 mmol) in NMP (52.0 ml) cooled to 0°C. Cesium carbonate (6.56 g, 20.1 mmol) was added thereto, and the mixture was stirred at 0°C for 45 minutes and then stirred at room temperature for 12 hours. Then, cesium carbonate (1.64 g, 5.03 mmol) was added thereto, and the mixture was stirred at room temperature for 24 hours. Further, cesium carbonate (0.546 g, 1.68 mmol) was added thereto, and the mixture was stirred at room temperature for 22 hours. An aqueous formic acid solution (50%) was added to the reaction solution, and the mixture was purified by reverse-phase silica gel column chromatography (0.1% solution of formic acid in acetonitrile/0.1% aqueous formic acid solution, 10-85%) to obtain the title compound D076 (3.40 g, 5.72 mmol, 34%) in a colorless amorphous form.

LRMS (ESI): m/z 617$[M+Na]^+$.

Retention time: 1.563 min (analysis conditions SMD-FA05-1)

Example 1-11-19: Synthesis of 2-O-tert-butyl 8-O-ethyl 6-[(4-piperidin-4- yloxyphenyl)methoxy]-3,4-dihydro-1H-isoquinoline-2,8-dicarboxylate (D074)

[Formula 164]

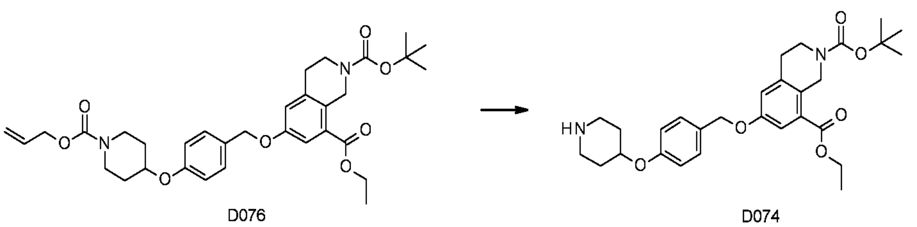

D076 D074

To a solution of 2-O-tert-butyl 8-O-ethyl 6-[[4-(1-prop-2-enoxycarbonylpiperidin-4- yl)oxyphenyl]methoxy]-3,4-dihydro-1H-isoquinoline-2,8-dicarboxylate (D076) (3.39 g, 5.70 mmol) in DCM (50.9 ml), pyrrolidine (4.68 mL, 57.0 mmol) and $Pd(PPh)_4$ (0.066 g, 0.057 mmol) were added, and the mixture was stirred at room temperature for 1.5 hours. Then, $Pd(PPh)_4$ (0.132 g, 0.114 mmol) was added thereto, and the mixture was stirred at room temperature for 3 hours. Further, $Pd(PPh)_4$ (0.132 g, 0.114 mmol) was added thereto, and the mixture was stirred at room temperature for 15.5 hours.

Further, $Pd(PPh)_4$ (0.132 g, 0.114 mmol) was added thereto, and the mixture was stirred at room temperature for 1 hour. The mixture was concentrated under reduced pressure. Then, the obtained crude product was purified by silica gel column chromatography (NH-silica gel, dichloromethane/hexane, 0- 80%) and further repurified by reverse-phase silica gel column chromatography (0.1% solution of formic acid in acetonitrile/0.1% aqueous formic acid solution, 10-50%) three times to obtain the title compound D074 (2.3 g, 4.5 mmol, 79%) in a colorless amorphous form.

$^1$H-NMR (400 MHz, $CDCl_3$) δ 7.47 (1H, s), 7.33 (2H, d, J = 8.6 Hz), 6.93-6.91 (3H, m), 4.99 (2H, s), 4.86 (2H, s), 4.38-4.32 (3H, m), 3.62 (2H, q, J = 10.0 Hz), 3.14 (2H, dt, J = 12.4, 4.7 Hz), 2.85 (2H, t, J = 5.4 Hz), 2.75-2.69 (2H, m), 2.03-1.99 (2H, m), 1.97 (2H, dq, J = 13.2, 2.1 Hz), 1.66 (2H, tt, J = 13.4, 4.7 Hz), 1.48 (9H, s), 1.39 (3H, t, J = 7.3 Hz).

LRMS (ESI): m/z511 $[M+H]^+$.

Retention time: 2.572 min (analysis conditions SMD-AC05-long).

Example 1-11-20: Synthesis of compound D082-03R

[Formula 165]

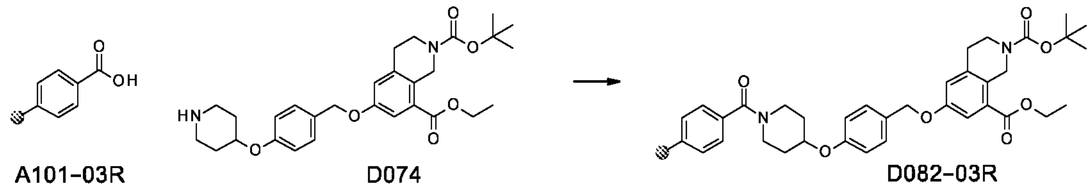

A101-03R D074 D082-03R

Compound D082-03R (loading rate: 0.200 mmol/g) can be synthesized by the same approach as in compound A102-03R using compound A101-03R (loading rate: 1.70 mmol/g) and 2-O-tert-butyl 8-O-ethyl 6-[(4-piperidin-4-yloxyphenyl)methoxy]-3,4-dihydro-1H- isoquinoline-2,8-dicarboxylate (D074).

D082-03R was dipped in a 10% solution of TFA in DCM containing 0.02 M pentamethylbenzene (0.10 mL) for 5 minutes. NMP (0.10 mL) and MeCN (0.25 mL) were added thereto, and the mixture was filtered. The filtrate was subjected to LC-MS analysis, so that 81.1% of compound D082 of interest and 18.9% of D082-Boc were observed.

[Formula 166]

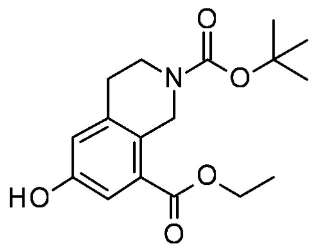

Compound D082

LRMS: m/z 266$[M-tBu+H]^+$

Retention time: 1.099 min (analysis conditions FA05-1, 299 nm).

[Formula 167]

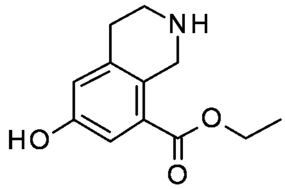

Compound D082-Boc

LRMS: m/z 222$[M+H]^+$

Retention time: 0.460 min (analysis conditions FA05-1, 299 nm).

Example 1-11-21: Synthesis of compound D166-03R

[Formula 168]

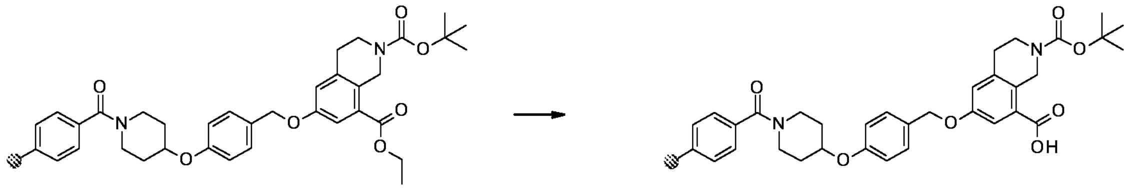

D082-03R D166-03R

D082-03R (8.61 g, 0.200 mmol/g), NMP (121 mL), and tAmylOH (34.4 mL) were added to a 200 mL column with a filter and shaken at room temperature for 1 hour. A 1 M aqueous TBAOH solution (5.17 mL, 5.17 mmol) was added thereto, and the mixture was shaken at room temperature for 7.5 hours.

Solid-phase purification

The suspension of the reaction solution and the resin was filtered through the filter of the column and washed three times with NMP/water = 1/1 (172 mL), three times with 0.05 M $TBAHSO_4$ + DTBP in NMP (172 mL), three times with NMP/water = 1/1 (172 mL), three times with NMP (172 mL), three times with MeOH (172 mL), three times with DCM (172 mL), and three times with heptane (172 mL), and the obtained solid phase was dried under reduced pressure to obtain compound D166-03R (8.59 g, 0.201 mmol/g). The same cleavage operation as in Example 1-11-8 was performed, so that 96.9 area% of compound D166 of interest was observed.

[Formula 169]

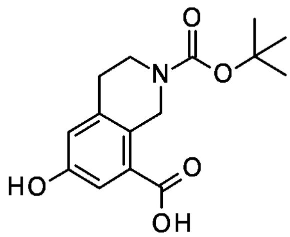

D166

Compound D166
Maximum wavelength: 304 nm.
Retention time: 0.871 min (analysis conditions SMD-FA05-1, 280 nm).

Example 1-11-22: Synthesis of 2-trimethylsilylethyl 4-[4- (hydroxymethyl)phenoxy]piperidine-1-carboxylate (D081)

[Formula 170]

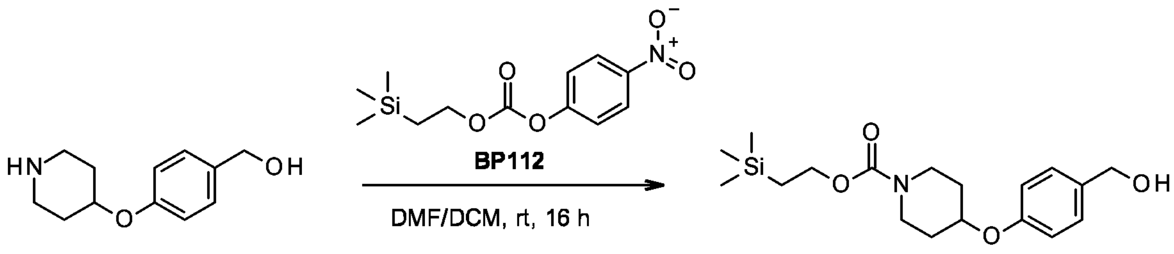


BP103
CAS: 1251336-69-2

D081

Synthesis of 2-trimethylsilylethyl4-[4-(hydroxymethyl)phenoxy]piperidine-1- carboxylate: compound D081

Under a nitrogen atmosphere, (4-piperidin-4-yloxyphenyl)methanol (compound BP103, CAS: 1251336-69-2) (8.7 g, 41.8 mmol, 1.0 eq) and DMF (85 mL) were added to a 500 mL flask.

A solution of (4-nitrophenyl) 2-trimethylsilylethylcarbonate (compound BP112) (11.6 g, 41.0 mmol, 1.0 eq) in DCM (85 mL) was added at room temperature to this solution. The obtained reaction mixture was stirred for 16 hours, and the reaction solution was concentrated under reduced pressure. The residue was purified by reverse-phase silica gel column chromatography (C18) ($CH_3CN$/water, 5-70%) to obtain 2-trimethylsilylethyl4-[4- (hydroxymethyl)phenoxy]piperidine-1-carboxylate (compound D081) as a white solid (5.4 g, 36.7%).

$^1$H-NMR (300 MHz, DMSO-d6) δ 7.22-7.19 (m, 2H), 6.97-6.87 (m, 2H), 5.03 (t, J = 5.7 Hz, 1H), 4.57-4.50 (m, 1H), 4.40 (d, J = 5.7 Hz, 2H), 4.12-4.07 (m, 2H), 3.72-3.65 (m, 2H), 3.19 (s, 2H), 1.95-1.83 (m, 2H), 1.56-1.45 (m, 2H), 1.01-0.89 (m, 2H), 0.02 (s, 9H).

LCMS (ESI, m/z): 374 $[M+Na]^+$.

Retention time: 1.180 min.

Analysis conditions: PH-TFA-24.

Example 1-11-23: Synthesis of tert-butyl 8-bromo-6-[[4-[1-(2- trimethylsilylethoxycarbonyl)piperidin-4-yl]oxyphenyl]methoxy]-3,4-dihydro-1H- isoquinoline-2-carboxylate (D079)

[Formula 171]

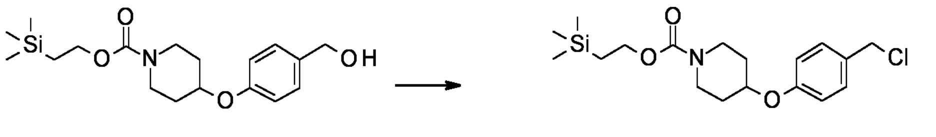

D081 D080

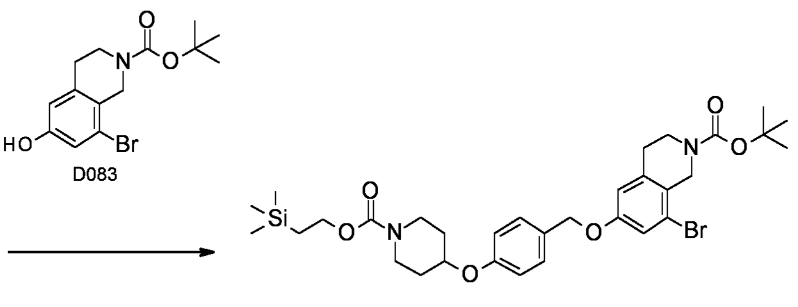

D083

D079

A solution of 2-trimethylsilylethyl 4-[4-(hydroxymethyl)phenoxy]piperidine-1- carboxylate (D081) (1.00 g, 2.84 mmol) and lithium chloride (0.241 g, 5.69 mmol) in DMI (10 ml) was cooled to 0°C. Then, 2,6-lutidine (0.988 mL, 8.53

mmol) was added thereto. Methanesulfonic anhydride (0.743 g, 4.27 mmol) was added thereto, and the mixture was stirred at 0°C for 15 minutes and then stirred at room temperature for 3 hours. Water, ethyl acetate (60 mL), and hexane (40 mL) were added to the obtained mixture. The obtained organic layer was washed with 0.5 M hydrochloric acid (40 mL) and a half-saturated aqueous solution of sodium chloride (40 mL). The organic layer was dried over sodium sulfate and then concentrated under reduced pressure. The obtained residue was added into a solution of tert-butyl 8-bromo-6-hydroxy-3,4-dihydro-1H-isoquinoline-2-carboxylate (D083, CAS: 1579518-76-5) (0.885 g, 2.70 mmol) in NMP (8.85 ml) cooled to 0°C. Cesium carbonate (1.11 g, 3.41 mmol) was added thereto, and the mixture was stirred overnight at room temperature. The reaction solution was cooled to 0°C. Then, an aqueous formic acid solution was added thereto, and the mixture was purified by reverse-phase silica gel column chromatography (0.1% solution of formic acid in acetonitrile/0.1% aqueous formic acid solution, 10-100%). Then, the obtained crude product was purified by silica gel column chromatography (dichloromethane/ethyl acetate) to obtain the title compound D079 (1.48 g, 2.24 mmol, 83%).

$^1$H-NMR (400 MHz, $CDCl_3$) δ: 7.32 (2H, d, J = 8.6 Hz), 7.08-7.07 (1H, m), 6.92 (2H, d, J = 8.6 Hz), 6.71 (1H, s), 4.94 (2H, s), 4.50-4.47 (3H, m), 4.21-4.17 (2H, m), 3.73- 3.71 (2H, m), 3.60 (2H, t, J = 10.0 Hz), 3.43-3.40 (2H, m), 2.80-2.77 (2H, m), 1.93-1.90 (2H, m), 1.80-1.77 (2H, m), 1.50 (9H, s), 1.03-0.99 (2H, m), 0.05 (9H, s).

LRMS (ESI): m/z683, 685 $[M+Na]^+$.

Retention time: 1.826 min (analysis conditions SMD-FA05-1).

Example 1-11-24: Synthesis of tert-butyl 8-bromo-6-[(4-piperidin-4- yloxyphenyl)methoxy]-3,4-dihydro-1H-isoquinoline-2-carboxylate (D075)

[Formula 172]

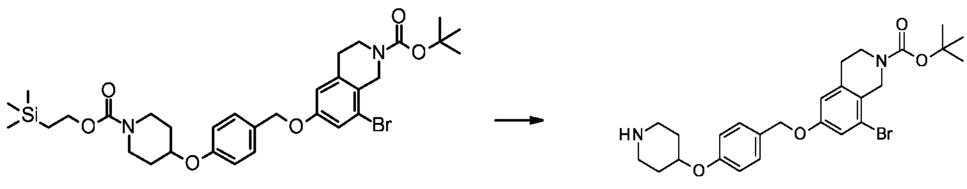

D079 D075

To a solution of tert-butyl 8-bromo-6-[[4-[1-(2- trimethylsilylethoxycarbonyl)piperidin-4-yl]oxyphenyl]methoxy]-3,4-dihydro-1H- isoquinoline-2-carboxylate (D079) (1.98 g, 2.99 mmol) in THF (21.8 mL), TBAF (1 M in THF, 8.97 mL, 8.97 mmol) was added, and the mixture was stirred at room temperature for 1 hour. Then, TBAF (1 M in THF, 2.99 mL, 2.99 mmol) was added thereto, and the mixture was stirred at room temperature for 1 hour. Further, TBAF (1 M in THF, 2.99 mL, 2.99 mmol) was added thereto, and the mixture was stirred at room temperature for 1 hour.

The mixture was concentrated under reduced pressure. Then, the obtained crude product was purified by silica gel column chromatography (NH-silica gel, dichloromethane/hexane) to obtain the title compound D075 (0.936 g, 2.99 mmol, 61%).

$^1$H-NMR (400 MHz, $CDCl_3$) δ: 7.31 (2H, d, J = 9.1 Hz), 7.08 (1H, d, J = 2.7 Hz), 6.92 (2H, d, J = 8.6 Hz), 6.71 (1H, s), 4.93 (2H, s), 4.47 (2H, s), 4.41-4.35 (1H, m), 3.60 (2H, t, J = 5.9 Hz), 3.17-3.12 (2H, m), 2.78-2.70 (4H, m), 2.02-1.99 (2H, m), 1.71-1.66 (2H, m), 1.50 (9H, s).

LRMS (ESI): m/z517, 519 $[M+H]^+$.

Retention time: 0.994 min (analysis conditions SMD-FA05-1).

Example 1-11-25: Synthesis of compound D083-03R

[Formula 173]

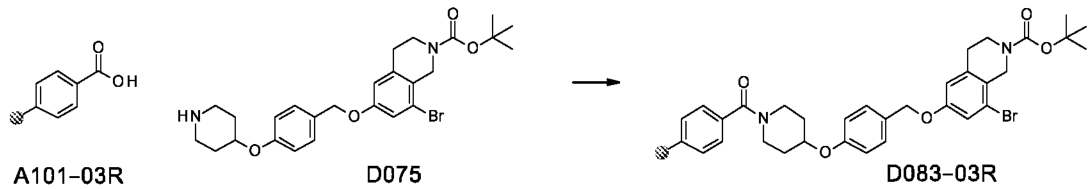

A101-03R D075 D083-03R

Compound D083-03R (loading rate: 0.200 mmol/g) can be synthesized by the same approach as in compound A102-03R using compound A101-03R (loading rate: 1.70 mmol/g) and tert-butyl 8-bromo-6-[(4-piperidin-4-yloxyphenyl)methoxy]-3,4-dihydro-1H- isoquinoline-2-carboxylate (D075).

D083-03R was dipped in a 10% solution of TFA in DCM containing 0.02 M pentamethylbenzene (0.10 mL) for 5 minutes. NMP (0.10 mL) and MeCN (0.25 mL) were added thereto, and the mixture was filtered. The filtrate was subjected to LC-MS analysis, so that 100% of compound D083 of interest was observed.

[Formula 174]

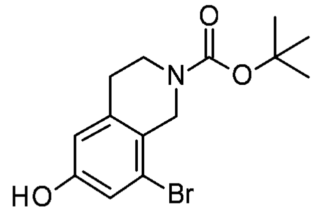

Compound D083

LRMS: m/z 272, 274[M-tBu+H]+

Retention time: 1.159 min (analysis conditions FA05-1, 277 nm).

Example 1-11-26: Synthesis of compound B101-03R

[Formula 175]

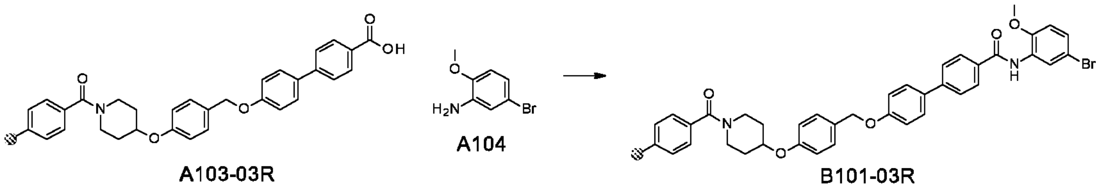

A103-03R A104 B101-03R

Under a nitrogen atmosphere, a solution of compound A103-03R (loading rate: 0.201 mmol/g, 1.00 g), DCM (12.0 mL), 5-bromo-2-methoxyaniline (A104) (0.162 g, 0.804 mmol), PipClU (0.145 mg, 0.402 mmol), and NMI (0.033 mg, 0.402 mmol) in MeCN (402 μL) was added to a 20 mL glass vial and shaken at room temperature for 3 hours.

Solid-phase purification

The suspension of the reaction solution and the resin was wholly transferred onto a filter and washed three times with NMP:$H_2O$ (1:1) (v/v, 5 mL), three times with NMP (5 mL), three times with MeOH (5.2 mL), with DCM (5.2 mL), and three times with heptane (5.2 mL), and the obtained solid phase was dried overnight under reduced pressure to obtain compound B101-03R (loading rate: 0.194 mmol/g, 1.00 g).

B101-03R was dipped in a 10% solution of TFA in DCM containing 0.05 M pentamethylbenzene (0.05 mL) for 5 minutes. DMF (0.05 mL) and MeCN (0.25 mL) were added thereto, and the mixture was filtered. The filtrate was subjected to LC-MS analysis, so that 99.5% of compound B101 of interest was observed.

[Formula 176]

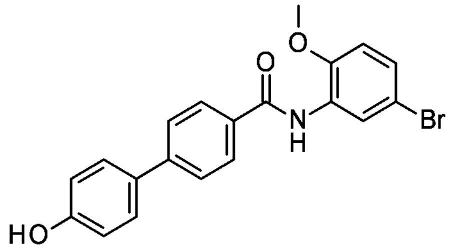

Compound B101

LRMS: m/z 398, 400$[M+H]^+$

Retention time: 1.228 min (analysis conditions FA05-1, 299 nm).

Example 1-11-27: Synthesis of compound A133-03R

[Formula 177]

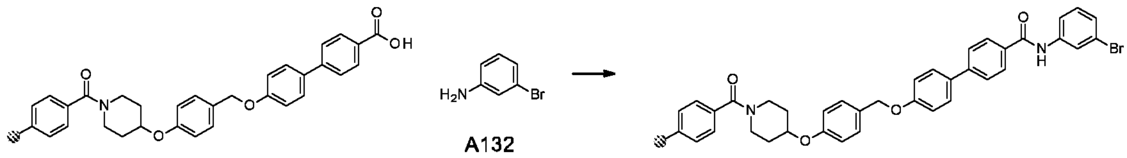

A103-03R A132 A133-03R

Compound B133-03R (loading rate: 0.195 mmol/g) can be synthesized by the same approach as in compound B101-03R using compound A103-03R (loading rate: 0.201 mmol/g) and 3-bromoaniline (A132).

B133-03R was dipped in a 10% solution of TFA in DCM containing 0.05 M pentamethylbenzene (0.05 mL) for 5 minutes. DMF (0.05 mL) and MeCN (0.25 mL) were added thereto, and the mixture was filtered. The filtrate was subjected to LC-MS analysis, so that 99.6% of compound A133 of interest was observed.

[Formula 178]

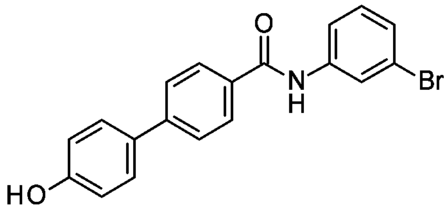

Compound A133

LRMS: m/z 368, 370$[M+H]^+$

Retention time: 1.161 min (analysis conditions FA05-1, 299 nm).

Example 1-11-28: Synthesis of compound B107-03R

[Formula 179]

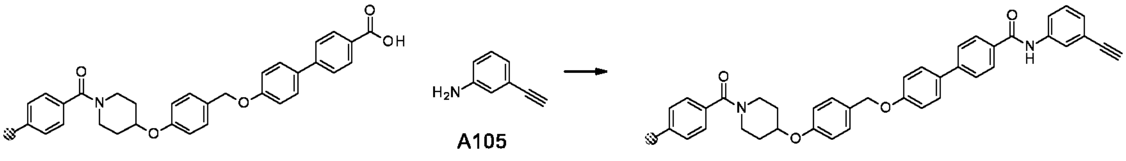

A105

A103-03R

B107-03R

Compound B107-03R (loading rate: 0.197 mmol/g) can be synthesized by the same approach as in compound B101-03R using compound A103-03R (loading rate: 0.201 mmol/g) and 3-ethynylaniline (A105).

B107-03R was dipped in a 10% solution of TFA in DCM containing 0.05 M pentamethylbenzene (0.05 mL) for 5 minutes. DMF (0.10 mL) and MeCN (0.25 mL) were added thereto, and the mixture was filtered. The filtrate was subjected to LC-MS analysis, so that 100% of compound B107 of interest was observed.

[Formula 180]

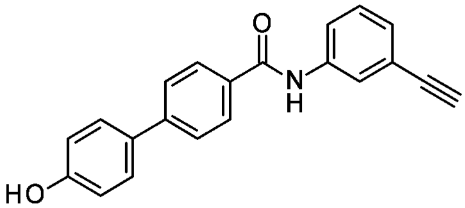

Compound B107

LRMS: m/z 314[M+H]+

Retention time: 1.083 min (analysis conditions FA05-1, 299 nm).

Example 1-11-29: Synthesis of compound B102-03R

[Formula 181]

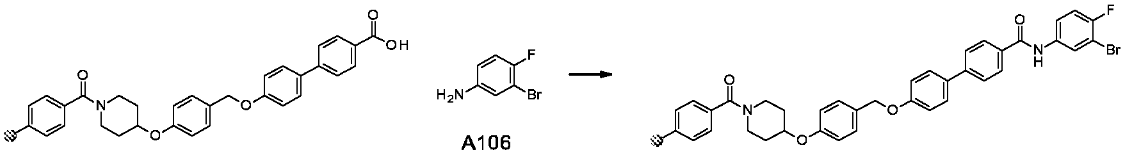

A106

A103-03R

B102-03R

Compound B102-03R (loading rate: 0.194 mmol/g) can be synthesized by the same approach as in compound B101-03R using compound A103-03R (loading rate: 0.201 mmol/g) and 3-bromo-4-fluoroaniline (A106).

B102-03R was dipped in a 10% solution of TFA in DCM containing 0.05 M pentamethylbenzene (0.05 mL) for 5 minutes. DMF (0.05 mL) and MeCN (0.25 mL) were added thereto, and the mixture was filtered. The filtrate was subjected to LC-MS analysis, so that 99.1 % of compound B102 of interest was observed.

[Formula 182]

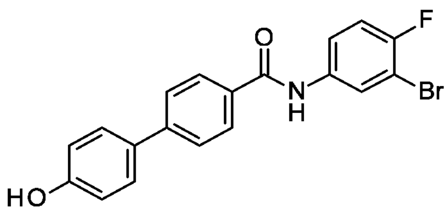

Compound B102

LRMS: m/z 388, 390[M+H]+

Retention time: 1.165 min (analysis conditions FA05-1, 299 nm).

Example 1-11-30: Synthesis of compound D090-03R

[Formula 183]

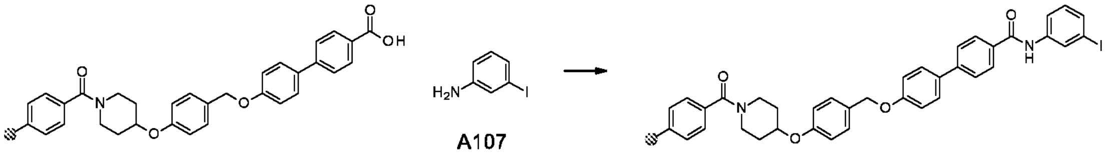

A103-03R A107 D090-03R

Compound D090-03R (loading rate: 0.193 mmol/g) can be synthesized by the same approach as in compound B101-03R using compound A103-03R (loading rate: 0.201 mmol/g) and 3-iodoaniline (A107).

D090-03R was dipped in a 10% solution of TFA in DCM containing 0.05 M pentamethylbenzene (0.05 mL) for 5 minutes. DMF (0.05 mL) and MeCN (0.25 mL) were added thereto, and the mixture was filtered. The filtrate was subjected to LC-MS analysis, so that 100% of compound D090 of interest was observed.

[Formula 184]

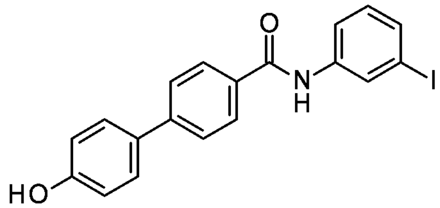

Compound D090

LRMS: m/z 416[M+H]+

Retention time: 1.192 min (analysis conditions FA05-1, 299 nm).

Example 1-11-31: Synthesis of compound A109-03R

[Formula 185]

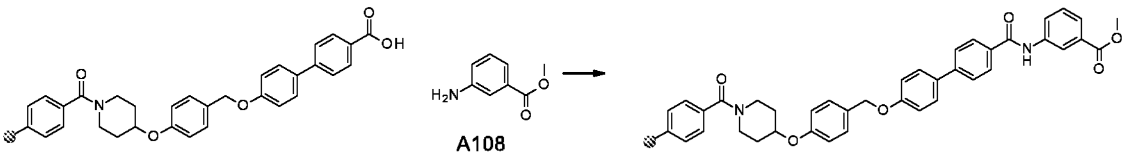

A103-03R A108 A109-03R

Compound A109-03R (loading rate: 0.196 mmol/g) can be synthesized by the same approach as in compound B101-03R using compound A103-03R (loading rate: 0.201 mmol/g) and methyl 3-aminobenzoate (A108).

A109-03R was dipped in a 10% solution of TFA in DCM containing 0.05 M pentamethylbenzene (0.05 mL) for 5 minutes. NMP (0.10 mL) and MeCN (0.25 mL) were added thereto, and the mixture was filtered. The filtrate was subjected to LC-MS analysis, so that 100% of compound A109 of interest was observed.

[Formula 186]

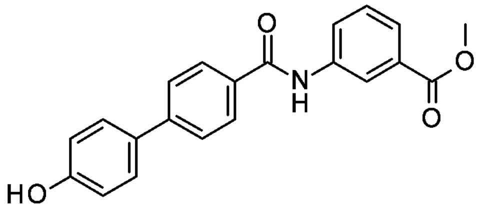

Compound A109

LRMS: m/z 348[M+H]+

Retention time: 1.036 min (analysis conditions FA05-1, 299 nm).

Example 1-11-32: Synthesis of compound A135-03R

[Formula 187]

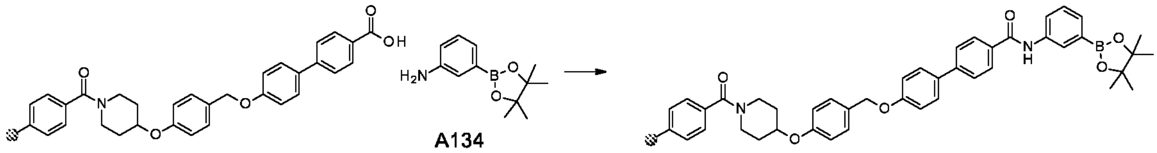

A134

A103-03R

A135-03R

Compound A135-03R (loading rate: 0.193 mmol/g) can be synthesized by the same approach as in compound B101-03R using compound A103-03R (loading rate: 0.201 mmol/g) and 3-(4,4,5,5-tetramethyl-1,3,2-dioxaboloran-2-yl)aniline (A134).

A135-03R was dipped in a 10% solution of TFA in DCM containing 0.05 M pentamethylbenzene (0.05 mL) for 5 minutes. NMP (0.10 mL) and MeCN (0.25 mL) were added thereto, and the mixture was filtered. The filtrate was subjected to LC-MS analysis, so that 91.9% of compound A135 of interest and 6.9% of A135B were observed.

[Formula 188]

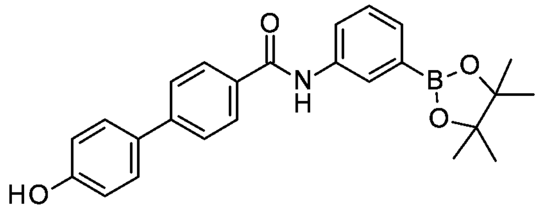

Compound A135

LRMS: m/z 416[M+H]+

Retention time: 1.236 min (analysis conditions FA05-1, 299 nm).

[Formula 189]

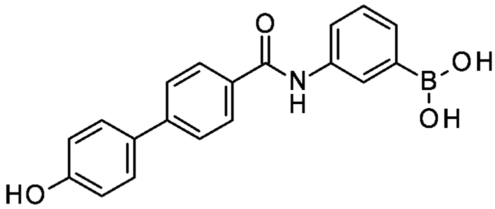

Compound A135B
LRMS: m/z 334[M+H]+
Retention time: 0.821 min (analysis conditions FA05-1, 299 nm).

Example 1-11-33: Synthesis of compound A111-03R

[Formula 190]

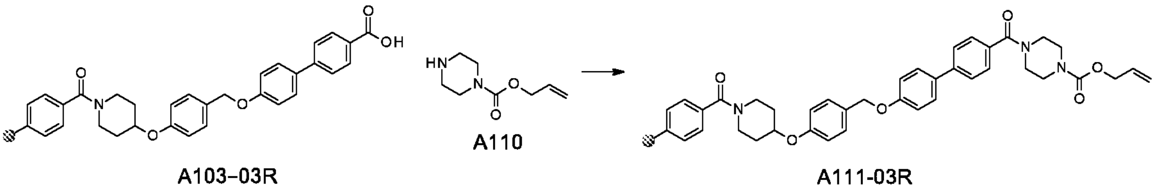

A103-03R A110 A111-03R

Compound A111-03R (loading rate: 0.195 mmol/g) can be synthesized by the same approach as in compound B101-03R using compound A103-03R (loading rate: 0.201 mmol/g) and prop-2-enyl piperazine-1-carboxylate (A110).
A111-03R was dipped in a 10% solution of TFA in DCM containing 0.05 M pentamethylbenzene (0.05 mL) for 5 minutes. NMP (0.10 mL) and MeCN (0.25 mL) were added thereto, and the mixture was filtered. The filtrate was subjected to LC-MS analysis, so that 97.4% of compound A111 of interest was observed.

[Formula 191]

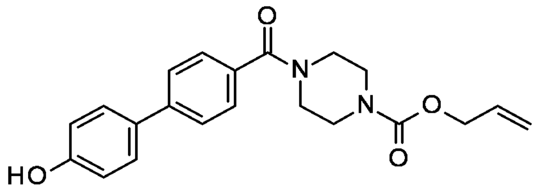

Compound A111
LRMS: m/z 367[M+H]+
Retention time: 0.917 min (analysis conditions FA05-1, 299 nm).

Example 1-11-34: Synthesis of compound A122-03R

[Formula 192]

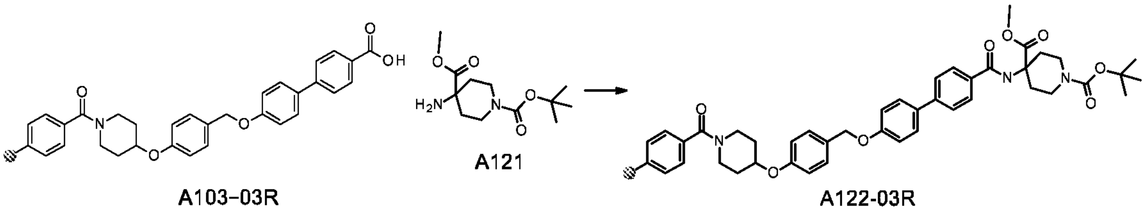

A103-03R A121 A122-03R

Compound A122-03R (loading rate: 0.192 mmol/g) can be synthesized by the same approach as in compound B101-03R using compound A103-03R (loading rate: 0.201 mmol/g) and 1-0-tert-butyl 4-O-methyl 4-aminopiperidine-1,4-dicarboxylate (A121).
A122-03R was dipped in a 10% solution of TFA in DCM containing 0.02 M pentamethylbenzene (0.05 mL) for 5 minutes. DMF (0.05 mL) and MeCN (0.25 mL) were added thereto, and the mixture was filtered. The filtrate was subjected to LC-MS analysis, so that 87.0% of compound A122 of interest and 12.2% of A122-Boc were observed.

[Formula 193]

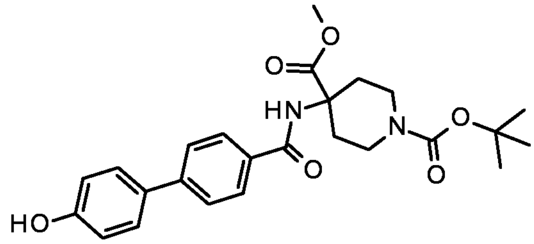

Compound A122

LRMS: m/z 399[M-tBu+H]+

Retention time: 1.060 min (analysis conditions FA05-1, 299 nm).

[Formula 194]

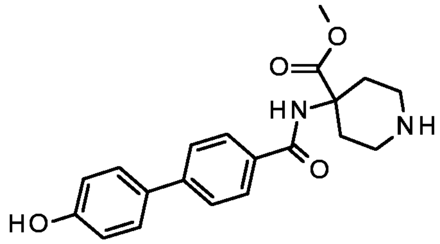

Compound A122-Boc

LRMS: m/z 355[M+H]+

Retention time: 0.548 min (analysis conditions FA05-1, 299 nm).

Example 1-11-35: Synthesis of compound B108-03R

[Formula 195]

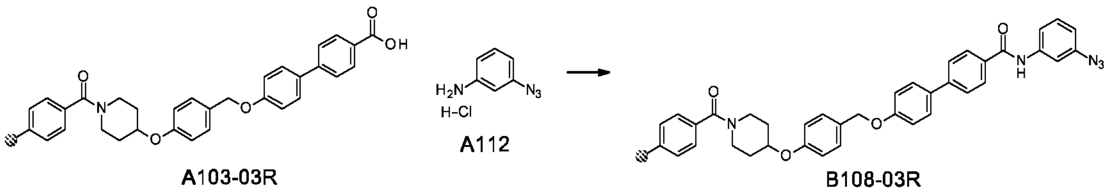


Under a nitrogen atmosphere, compound A103-03R (loading rate: 0.201 mmol/g, 500 mg) and DCM (7.50 mL) were added to a 20 mL glass vial and shaken at room temperature for 1 hour. 3-Azidoaniline hydrochloride (A112) (68.6 mg, 0.402 mmol) was added thereto, then a solution of PipClU (91 mg, 0.251 mmol) and NMI (20.6 mg, 0.251 mmol) in MeCN (251 $\mu$L) was added thereto, and the mixture was shaken at room temperature for 5 hours. 3-Azidoaniline hydrochloride (A112) (68.6 mg, 0.402 mmol) was added thereto, then a solution of PipClU (91 mg, 0.251 mmol) and NMI (20.6 mg, 0.251 mmol) in MeCN (251 $\mu$L) was added thereto, and the mixture was shaken at room temperature for 2 hours. 3-Azidoaniline hydrochloride (A112) (68.6 mg, 0.402 mmol) was added thereto, then a solution of PipClU (91 mg, 0.251 mmol) and NMI (20.6 mg, 0.251 mmol) in MeCN (251 $\mu$L) was added thereto, and the mixture was shaken at room temperature for 72 hours.

Solid-phase purification

The suspension of the reaction solution and the resin was wholly transferred onto a filter and washed three

times with NMP:HzO (1:1) (v/v, 5 mL), three times with NMP (5 mL), three times with MeOH (5 mL), three times with DCM (5 mL), and three times with heptane (5 mL), and the obtained solid phase was dried overnight under reduced pressure to obtain compound B108-03R (loading rate: 0.196 mmol/g, 528 mg).

B108-03R was dipped in a 10% solution of TFA in DCM containing 0.05 M pentamethylbenzene (0.05 mL) for 5 minutes. DMF (0.10 mL) and MeCN (0.25 mL) were added thereto, and the mixture was filtered. The filtrate was subjected to LC-MS analysis, so that 98.9% of compound B108 of interest was observed.

[Formula 196]

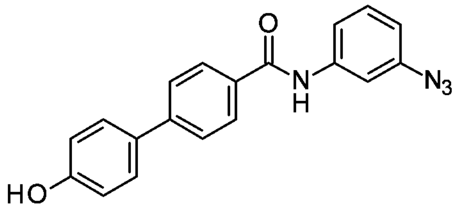

Compound B108

LRMS: m/z 331[M+H]$^+$

Retention time: 1.125 min (analysis conditions FA05-1, 299 nm).

Example 1-11-36: Synthesis of compound B105-03R

[Formula 197]

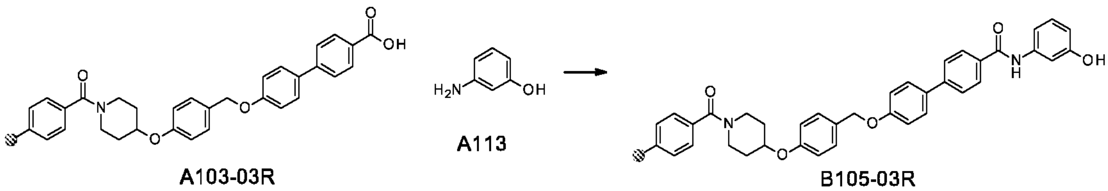


Under a nitrogen atmosphere, compound A103-03R (loading rate: 0.201 mmol/g, 3.0 g) and NMP (49.5 mL) were added to a 60 mL column and shaken at room temperature for 1 hour. 3-Aminophenol (A113) (197 mg, 1.81 mmol), DIC (280 μL, 1.81 mmol), and HOAt (246 mg, 1.81 mmol) were added thereto, and the mixture was shaken at room temperature for 96 hours.

Solid-phase purification

The suspension of the reaction solution and the resin was transferred onto a filter using NMP (45 mL) and washed four times with NMP (45 mL), four times with methanol (45 mL), four times with DCM (45 mL), and four times with heptane (45 mL), and the obtained solid phase was dried overnight under reduced pressure to obtain compound B105- 03R (loading rate: 0.197 mmol/g, 3.13 g).

B105-03R was dipped in a 10% solution of TFA in DCM containing 0.05 M pentamethylbenzene (0.05 mL) for 5 minutes. NMP (0.10 mL) and MeCN (0.25 mL) were added thereto, and the mixture was filtered. The filtrate was subjected to LC-MS analysis, so that 98.4% of compound B105 of interest was observed.

[Formula 198]

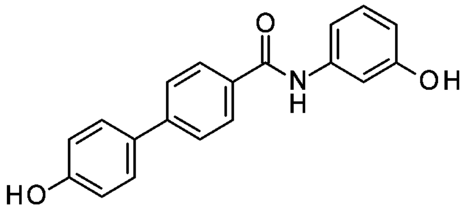

Compound B105

LRMS: m/z 306[M+H]+

Retention time: 0.868 min (analysis conditions FA05-1, 299 nm).

Example 1-11-37: Synthesis of compound H005-03R

[Formula 199]

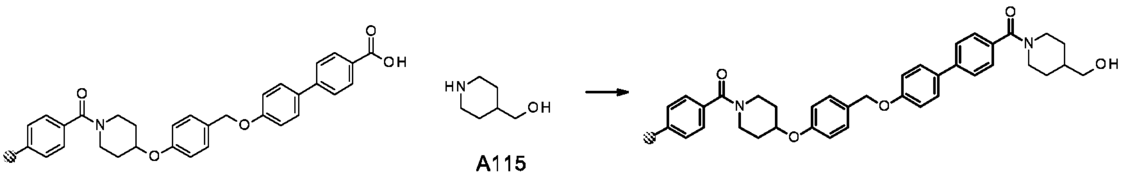

A115

A103–03R

H005-03R

Compound H005-03R (loading rate: 0.197 mmol/g) can be synthesized by the same approach as in compound B105-03R using compound A103-03R (loading rate: 0.201 mmol/g) and piperidin-4-ylmethanol (A115).

H005-03R was dipped in a 10% solution of TFA in DCM containing 0.02 M pentamethylbenzene (0.10 mL) for 5 minutes. DMF (0.10 mL) and MeCN (0.25 mL) were added thereto, and the mixture was filtered. The filtrate was subjected to LC-MS analysis, so that 96.7% of compound H005 of interest was observed.

[Formula 200]

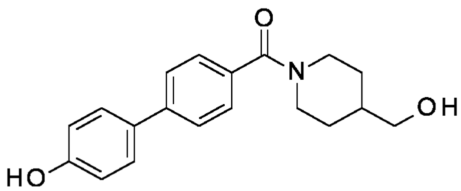

Compound H005

LRMS: m/z 312[M+H]+

Retention time: 0.712 min (analysis conditions FA05-1, 299 nm).

Example 1-11-38: Synthesis of compound H002-03R

[Formula 201]

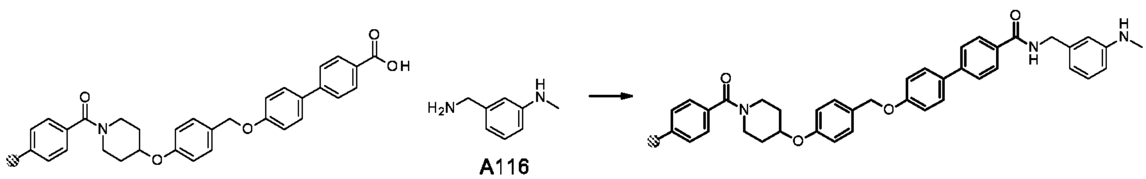

A116

A103–03R

H002-03R

Compound H002-03R (loading rate: 0.196 mmol/g) can be synthesized by the same approach as in compound B105-03R using compound A103-03R (loading rate: 0.201 mmol/g) and 3-(aminomethyl)-N-methylaniline (A116).

H002-03R was dipped in a 10% solution of TFA in DCM containing 0.02 M pentamethylbenzene (0.05 mL) for 5 minutes. DMF (0.05 mL) and MeCN (0.25 mL) were added thereto, and the mixture was filtered. The filtrate was subjected to LC-MS analysis, so that 97.2% of compound H002 of interest was observed. H002-03R is the same compound as D020-03R, but differs therefrom in the amount of the compound supported by the solid phase.

[Formula 202]

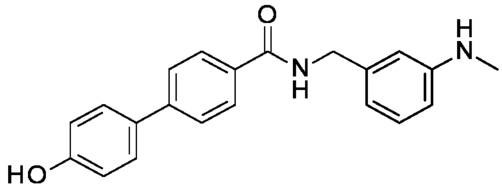

Compound H002

LRMS: m/z 333[M+H]$^+$

Retention time: 0.675 min (analysis conditions FA05-1,299 nm).

Example 1-11-39: Synthesis of compound A118-03R

[Formula 203]

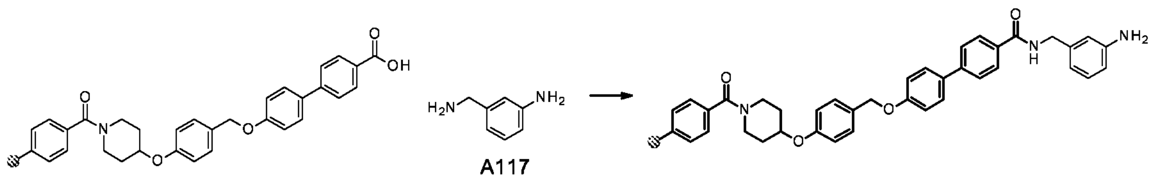

A103-03R A117 A118-03R

Compound A118-03R (loading rate: 0.197 mmol/g) can be synthesized by the same approach as in compound B105-03R using compound A103-03R (loading rate: 0.201 mmol/g) and 3-(aminomethyl)-aniline (A117).

A118-03R was dipped in a 10% solution of TFA in DCM containing 0.2 M pentamethylbenzene (0.05 mL) for 5 minutes. DMA (0.20 mL) and MeCN (0.10 mL) were added thereto, and the mixture was filtered. The filtrate was subjected to LC-MS analysis, so that 98.1% of compound A118 of interest was observed. A118-03R is the same compound as D001-03R, but differs therefrom in the amount of the compound supported by the solid phase.

[Formula 204]

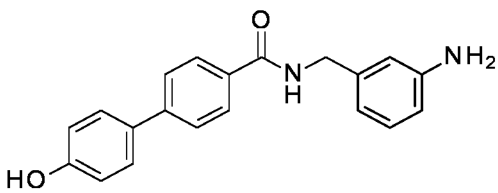

Compound A118

LRMS: m/z 319[M+H]$^+$

Retention time: 0.625 min (analysis conditions FA05-1, 299 nm).

Example 1-11-40: Synthesis of compound A127-03R

[Formula 205]

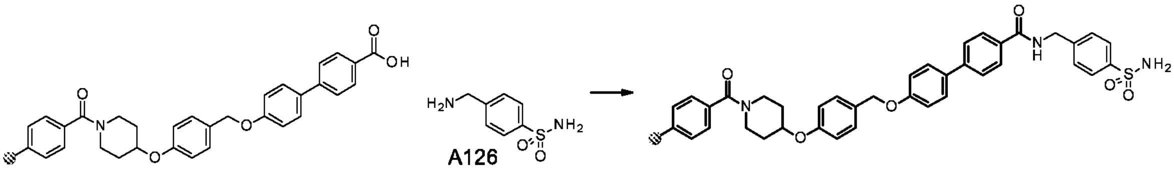

A103-03R

A127-03R

Compound A127-03R (loading rate: 0.194 mmol/g) can be synthesized by the same approach as in compound B105-03R using compound A103-03R (loading rate: 0.201 mmol/g) and 3-(aminomethyl)benzenesulfonamide (A126).

A127-03R was dipped in a 10% solution of TFA in DCM containing 0.02 M pentamethylbenzene (0.05 mL) for 5 minutes. DMF (0.05 mL) and MeCN (0.25 mL) were added thereto, and the mixture was filtered. The filtrate was subjected to LC-MS analysis, so that 100% of compound A127 of interest was observed.

[Formula 206]

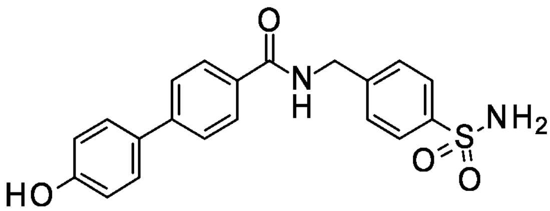

Compound A127

LRMS: m/z 383[M+H]+

Retention time: 0.771 min (analysis conditions FA05-1, 299 nm).

Example 1-11-41: Synthesis of compound H004-03R

[Formula 207]

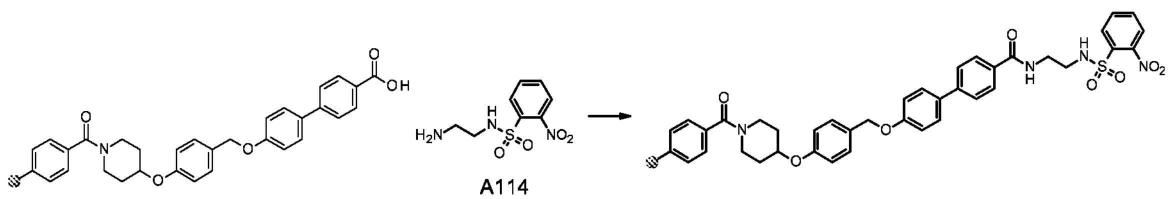

A103-03R

H004-03R

Under a nitrogen atmosphere, compound A103-03R (loading rate: 0.201 mmol/g, 500 mg) and NMP (6.00 mL) were added to an 8 mL glass vial, then N-(2-aminoethyl)-2- nitrobenzenesulfonamide (A114) (85 mg, 302 μmol), DIC (47.0 μL, 302 μmol), HOAt (47.9 mg, 352 μmol), and DIPEA (79.0 μL, 452 μmol) were added thereto, and the mixture was shaken at room temperature for 48 hours.

Solid-phase purification

The suspension of the reaction solution and the resin was transferred onto two filters using NMP:HzO (1:1) (v/v, 10 mL), each of which was washed three times with NMP:$H_2O$ (1:1) (v/v, 5 mL), three times with NMP (5 mL), three times with a 0.05 M solution of TBAHSO4·DTBP in NMP (5 mL), three times with a 0.05 M solution of NMM in NMP (5 mL), three times with NMP:$H_2O$ (1:1) (v/v, 5 mL), three times with NMP (5 mL), three times with methanol (5 mL), three

times with DCM (5 mL), and three times with heptane (5 mL), and the obtained solid phase was dried overnight under reduced pressure to obtain compound H004-03R (loading rate: 0.192 mmol/g, 523 mg).

H004-03R was dipped in a 10% solution of TFA in DCM containing 0.02 M pentamethylbenzene (0.05 mL) for 1 minute. DMF (0.05 mL) and MeCN (0.25 mL) were added thereto, and the mixture was filtered. The filtrate was subjected to LC-MS analysis, so that 99.7% of compound H004 of interest was observed.

[Formula 208]

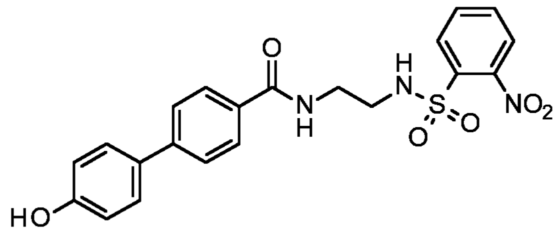

Compound H004

LRMS: m/z 442[M+H]+

Retention time: 0.901 min (analysis conditions FA05-1, 299 nm).

Example 1-11-42: Synthesis of compound A119-03R

[Formula 209]

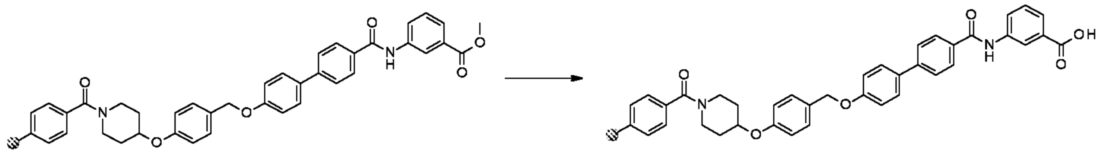

A109-03R A119-03R

Under a nitrogen atmosphere, compound A109-03R (loading rate: 0.196 mmol/g, 2.05 g), NMP (28.7 mL), and 2-methyl-2-butanol (8.2 mL) were added to a 50 mL glass vial and stirred at room temperature for 1 hour. An aqueous normal tetrabutylammonium hydroxide solution (1 M, 1.21 mL, 1.21 mmol) was added thereto, and the mixture was stirred at 60°C for 19 hours. An aqueous normal tetrabutylammonium hydroxide solution (1 M, 1.21 mL, 1.21 mmol) was added thereto, and the mixture was stirred at 60°C for 3.5 hours.

Solid-phase purification

The suspension of the reaction solution and the resin was transferred onto eight filters using NMP:HzO (1:1) (v/v, 5 mL), each of which was washed three times with NMP:$H_2O$ (1:1) (v/v, 5 mL), three times with a 0.05 M solution of $TBAHSO_4$·DTBP in NMP (5 mL), three times with NMP:$H_2O$ (1:1) (v/v, 5 mL), three times with NMP (5 mL), three times with methanol (5 mL), three times with DCM (5 mL), and three times with heptane (5 mL), and the obtained solid phase was dried overnight under reduced pressure to obtain compound A1 19-03R (loading rate: 0.196 mmol/g, 2.05 g).

A119-03R was dipped in a 10% solution of TFA in DCM containing 0.05 M pentamethylbenzene (0.05 mL) for 5 minutes. DMF (0.05 mL) and MeCN (0.25 mL) were added thereto, and the mixture was filtered. The filtrate was subjected to LC-MS analysis, so that 98.8% of compound A119 of interest was observed.

[Formula 210]

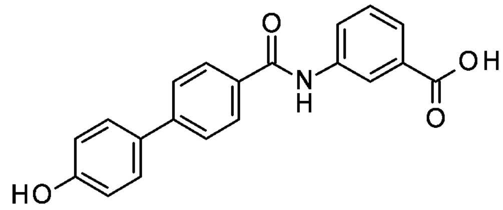

Compound A119

LRMS: m/z 334[M+H]+

Retention time: 0.875 min (analysis conditions FA05-1, 299 nm).

Example 1-11-43: Synthesis of compound A120-03R

[Formula 211]

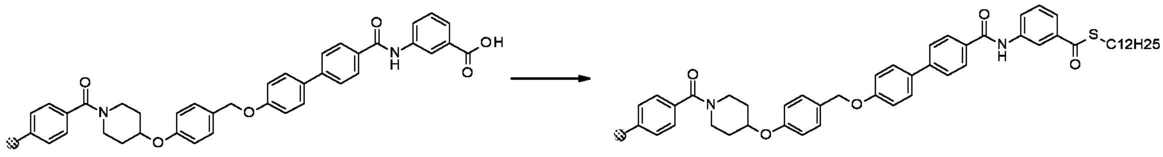

A119-03R A120-03R

Under a nitrogen atmosphere, compound A119-03R (loading rate: 0.197 mmol/g, 500 mg), HOAt (73.7 mg, 542 μmol), and NMP (5.00 mL) were added to an 8 mL glass vial. DIC (77.0 μL, 493 μmol), DIPEA (112 μL, 640 μmol), and 1-dodecanethiol (120 μL, 493 μmol) were added thereto, and the mixture was shaken at 60°C for 6 hours.

Solid-phase purification

The suspension of the reaction solution and the resin was transferred onto two filters using NMP:HzO (1:1) (v/v, 10 mL), each of which was washed three times with NMP:$H_2O$ (1:1) (v/v, 5 mL), three times with NMP (5 mL), three times with methanol (5.5 mL), three times with DCM (5.5 mL), and three times with heptane (5.5 mL), and the obtained solid phase was dried overnight under reduced pressure to obtain compound A120- 03R (loading rate: 0.190 mmol/g, 507 mg).

A120-03R was dipped in a 10% solution of TFA in DCM containing 0.02 M pentamethylbenzene (0.05 mL) for 1 minute. DMF (0.05 mL) and MeCN (0.25 mL) were added thereto, and the mixture was filtered. The filtrate was subjected to LC-MS analysis, so that 99.1% of compound A120 of interest was observed.

[Formula 212]

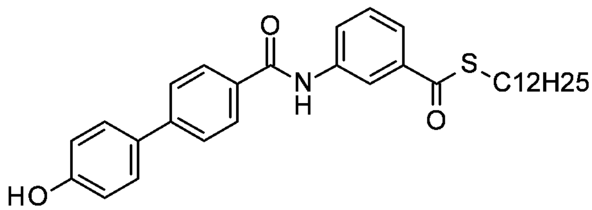

Compound A120

LRMS: m/z 518[M+H]+

Retention time: 1.868 min (analysis conditions FA05-1, 299 nm).

Example 1-11-44: Synthesis of compound H003-03R

[Formula 213]

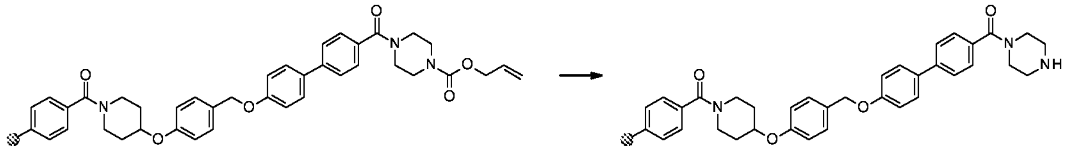

A111-03R H003-03R

Under a nitrogen atmosphere, compound A111-03R (loading rate: 0.195 mmol/g, 5.0 g), pentamethylbenzene (1.45 g, 9.75 mmol), pyrrolidine (4.03 mL, 48.8 mmol), and DCE (75.0 mL) were added to a 100 mL column and stirred at room temperature for 1 hour. $Pd(PPh_3)_4$ (113 mg, 98 μmol) was added thereto, and the mixture was shaken at room temperature for 2 hours.

Solid-phase purification

The suspension of the reaction solution and the resin was washed three times with NMP (100 mL), three times with a 0.3 M solution of N-acetylcysteine in NMP:HzO (5:1, v/v) (100 mL), three times with a 0.2 M solution of DIPEA in NMP (100 mL), three times with NMP (100 mL), three times with methanol (100 mL), three times with DCM (100 mL), and three times with heptane (100 mL), and the obtained solid phase was dried overnight under reduced pressure to obtain compound H003-03R (loading rate: 0.197 mmol/g, 4.71 g).

H003-03R was dipped in a 10% solution of TFA in DCM containing 0.05 M pentamethylbenzene (0.05 mL) for 5 minutes. NMP (0.10 mL) and MeCN (0.25 mL) were added thereto, and the mixture was filtered. The filtrate was subjected to LC-MS analysis, so that 96.8% of compound H003 of interest was observed. H003-03R is the same compound as D021-03R, but differs therefrom in the loading rate of the solid phase.

[Formula 214]

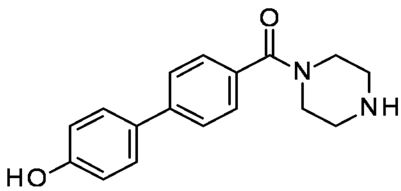

Compound H003

LRMS: m/z 283$[M+H]^+$

Retention time: 0.472 min (bimodal) (analysis conditions FA05-1, 299 nm).

Example 1-11-45: Synthesis of compound A137-03R

[Formula 215]

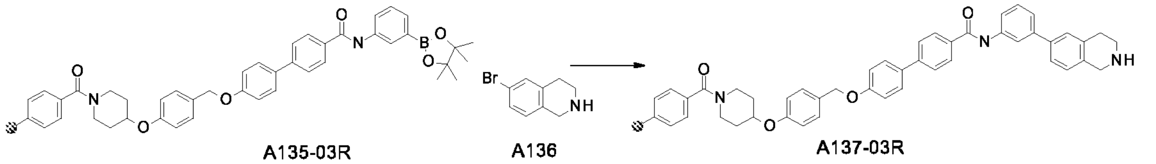

A135-03R A136 A137-03R

Under a nitrogen atmosphere, compound A135-03R (loading rate: 0.193 mmol/g, 750 mg), 6-bromo-1,2,3,4-tetrahydroisoquinoline A136 (92 mg, 434 μmol), and THF (11.3 mL) were added to a 20 mL glass vial and stirred at room temperature for 1 hour. $H_2O$ (39 μL, 2.17 mmol), CataCXium A Pd G4 (107 mg, 145 μmol), and phosphazene

P2tBu (326 μL, 651 μmol) were added thereto, and the mixture was shaken at 60°C for 1 hour.

Solid-phase purification

The suspension of the reaction solution and the resin was transferred onto three filters using NMP (5 mL), each of which was washed three times with NMP (5.0 mL), three times with a 0.2 M solution of N-acetylcysteine in NMP:$H_2O$ (5:1, v/v) (5.0 mL), three times with NMP (5.0 mL), three times with methanol (5.0 mL), three times with DCM (5.0 mL), and three times with heptane (5.0 mL), and the obtained solid phase was dried overnight under reduced pressure to obtain compound A137-03R (loading rate: 0.193 mmol/g, 753 mg).

A137-03R was dipped in a 10% solution of TFA in DCM containing 0.05 M pentamethylbenzene (0.05 mL) for 5 minutes. NMP (0.10 mL) and MeCN (0.25 mL) were added thereto, and the mixture was filtered. The filtrate was subjected to LC-MS analysis, so that 94.0% of compound A137 of interest was observed.

[Formula 216]

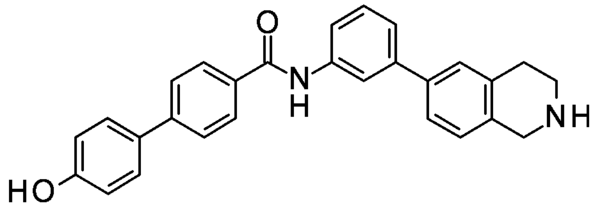

Compound A137

LRMS: m/z 421[M+H]+

Retention time: 0.748 min (analysis conditions FA05-1, 299 nm).

Example 1-11-46: Synthesis of compound A123-03R

[Formula 217]

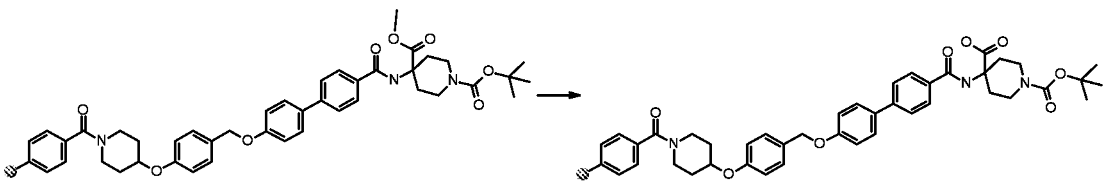

A122-03R A123-03R

Under a nitrogen atmosphere, compound A122-03R (loading rate: 0.192 mmol/g, 3.0 g), NMP (42.0 mL), and 2-methyl-2-butanol (12.0 mL) were added to a 120 mL column and stirred at room temperature for 1 hour. An aqueous normal tetrabutylammonium hydroxide solution (1 M, 1.15 mL, 1.15 mmol) was added thereto, and the mixture was stirred at room temperature for 4 hours.

Solid-phase purification

The suspension of the reaction solution and the resin was transferred onto eight filters using NMP:HzO (1:1) (v/v, 60 mL), each of which was washed three times with NMP:$H_2O$ (1:1) (v/v, 60 mL), three times with a 0.05 M solution of $TBAHSO_4$·DTBP in NMP (60 mL), three times with NMP:$H_2O$ (1:1) (v/v, 60 mL), three times with NMP (60 mL), three times with methanol (60 mL), three times with DCM (60 mL), and three times with heptane (60 mL), and the obtained solid phase was dried overnight under reduced pressure to obtain compound A123-03R (loading rate: 0.193 mmol/g, 3.0 g).

A123-03R was dipped in a 10% solution of TFA in DCM containing 0.05 M pentamethylbenzene (0.05 mL) for 5 minutes. NMP (0.10 mL) and MeCN (0.25 mL) were added thereto, and the mixture was filtered. The filtrate was subjected to LC-MS analysis, so that 96.3% of compound A123 of interest and 3.7% of A123-Boc were observed.

[Formula 218]

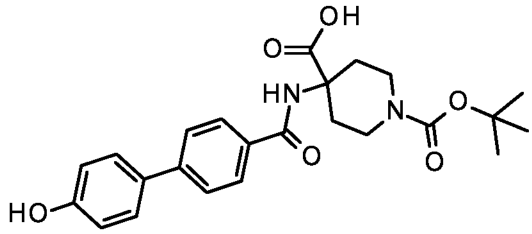

Compound A123

LRMS: m/z 385[M-tBu+H]+

Retention time: 0.945 min (analysis conditions FA05-1, 299 nm).

[Formula 219]

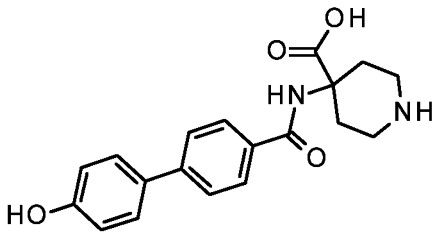

Compound A123-Boc

LRMS: m/z 341[M+H]+

Retention time: 0.464 min (analysis conditions FA05-1, 299 nm).

Example 1-11-47: Synthesis of compound A125-03R

[Formula 220]

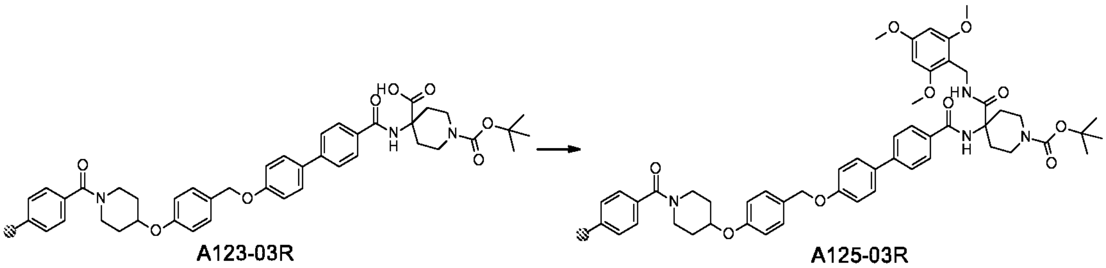


Under a nitrogen atmosphere, compound A123-03R (loading rate: 0.192 mmol/g, 3.0 g) and NMP (45.0 mL) were added to a 100 mL glass vial and shaken at room temperature for 1 hour. HOAt (235 mg, 1.73 mmol), DIC (269 μL, 1.73 mmol), and (2,4,6- trimethoxyphenyl)methanamine (A124) (341 mg, 1.73 mmol) were added thereto, and the mixture was shaken at 60°C for 3 hours.

Solid-phase purification

The suspension of the reaction solution and the resin was washed three times with NMP (60 mL), three times with NMP:HzO (1:1) (v/v, 60 mL), three times with NMP (60 mL), three times with methanol (60 mL), three times with DCM (60 mL), and three times with heptane (60 mL), and the obtained solid phase was dried overnight under reduced pressure to obtain compound A125-03R (loading rate: 0.185 mmol/g, 3.35 g).

A125-03R was dipped in a 10% solution of TFA in DCM containing 0.05 M pentamethylbenzene (0.05 mL) for 5 minutes. NMP (0.10 mL) and MeCN (0.25 mL) were added thereto, and the mixture was filtered. The filtrate was

subjected to LC-MS analysis, so that 90.2% of compound A125 of interest and 5.2% of A125-Boc were observed.

[Formula 221]

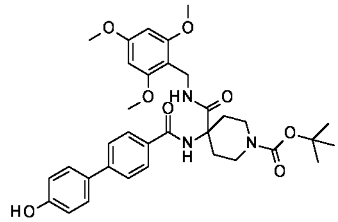

Compound A125

LRMS: m/z 620[M+H]+

Retention time: 1.119 min (analysis conditions FA05-1, 299 nm).

[Formula 222]

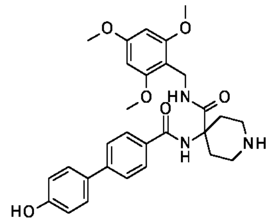

Compound A125-Boc

Compound A125-Boc

LRMS: m/z 520[M+H]+

Retention time: 0.713 min (analysis conditions FA05-1, 299 nm).

Example 1-11-48: Synthesis of compound H001-03R

[Formula 223]

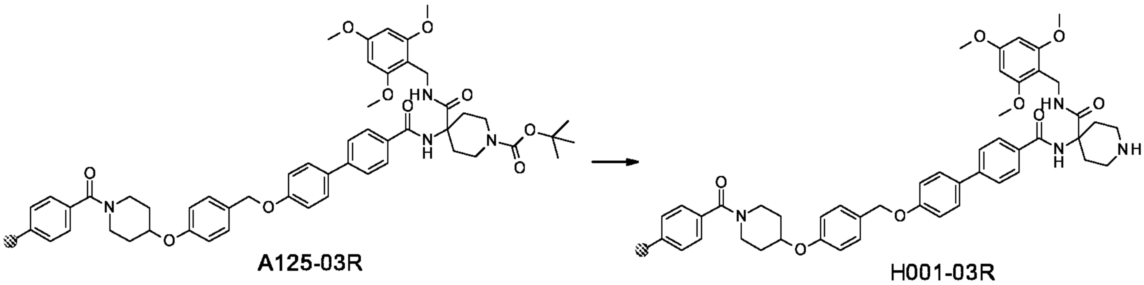

A125-03R

H001-03R

Under a nitrogen atmosphere, compound A125-03R (loading rate: 0.185 mmol/g, 250 mg) and 2-MeTHF (5.0 mL) were added to each of three 8.0 mL glass vials and shaken at room temperature for 1 hour. 2,6-di-tert-Butylpyridine (312 μL, 1.39 mmol) and $Tm(OTf)_3$ (570 mg, 925 μmol) were added thereto, and the mixture was shaken at 60°C for 22 hours.

Solid-phase purification

The suspension of the reaction solution and the resin were transferred onto three filters, respectively, with a 0.2 M solution of DMEDA in NMP (3 mL) and washed three times with a 0.2 M solution of DMEDA in NMP (5.0 mL),

three times with NMP (5.0 mL), three times with a 0.05 M solution of $TBAHSO_4$·DTBP in NMP (5.0 mL), three times with a 0.05 M solution of BTMG in NMP (5.0 mL), three times with a 0.05 M solution of DTBP in NMP (5.0 mL), three times with NMP:HzO (1:1) (v/v, 5.0 mL), three times with NMP (5.0 mL), three times with methanol (5.0 mL), three times with DCM (5.0 mL), and three times with heptane (5.0 mL), and the obtained solid phase was dried overnight under reduced pressure to obtain compound H001-03R (loading rate: 0.188 mmol/g, 895 mg).

H001-03R was dipped in a 10% solution of TFA in DCM containing 0.05 M pentamethylbenzene (0.05 mL) for 5 minutes. NMP (0.10 mL) and MeCN (0.25 mL) were added thereto, and the mixture was filtered. The filtrate was subjected to LC-MS analysis, so that 87.1% of compound H001 of interest and 9.4% of H001B were observed.

[Formula 224]

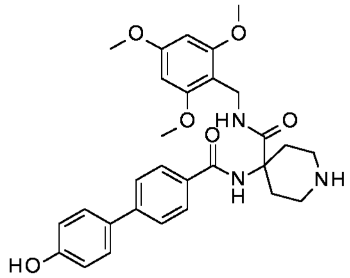

Compound H001

LRMS: m/z 520 $[M+H]^+$.

Retention time: 0.708 min (analysis conditions FA05-1, 299 nm).

[Formula 225]

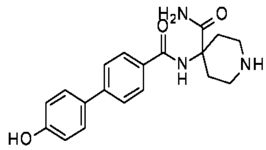

Compound H001B

LRMS: m/z 340 $[M+H]^+$.

Retention time: 0.448 min (analysis conditions FA05-1, 299 nm).

Example 1-11-49: Synthesis of methyl 2-[3-bromo-5-hydroxyphenyl]propanoate (D120)

[Formula 226]

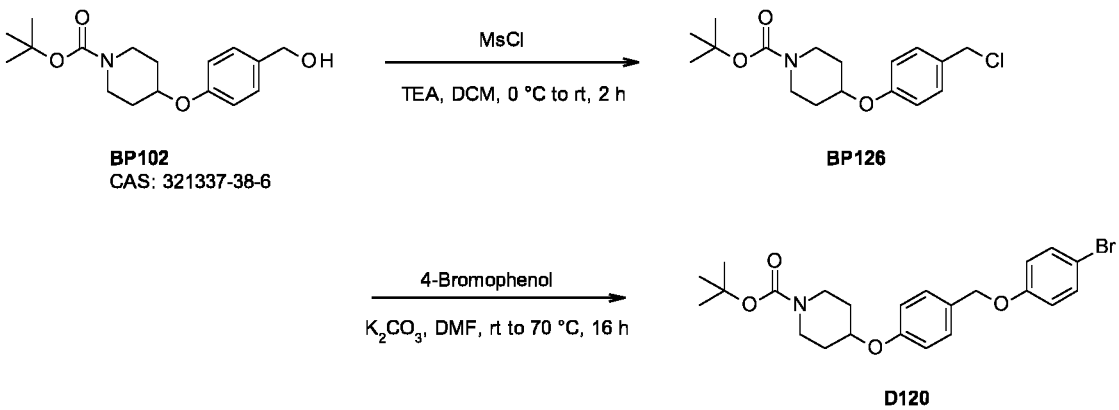

First step: Synthesis of tert-butyl 4-[4-(chloromethyl)phenoxy]piperidine-1- carboxylate: compound BP126

Under a nitrogen atmosphere, tert-butyl 4-[4-(hydroxymethyl)phenoxy]piperidine-1- carboxylate (compound BP102, CAS: 321337-38-6) (20.0 g, 65.1 mmol, 1.0 eq), TEA (19.8 g, 195.8 mmol, 3.0 eq), and DCM (100 mL) were added to a 500 mL flask. This reaction mixture was cooled to 0°C, and MsCl (14.9 g, 130.1 mmol, 2.0 eq) was added dropwise thereto. This reaction mixture was stirred at room temperature for 2 hours and diluted with DCM (250 mL). The obtained organic layer was washed with water (100 mL) and dried over $Na_2SO_4$. After filtration of the solid, the filtrate was concentrated under reduced pressure to obtain a crude product of tert-butyl 4-[4- (chloromethyl)phenoxy]piperidine-1-carboxylate (compound BP126) as a brown oil (20.0 g).
LCMS (ESI, m/z): 226[M-Boc+H]$^+$.
Retention time: 1.171 min.
Analysis conditions: PH-FA-17.

Second step: Synthesis of tert-butyl 4-[4-[(4- bromophenoxy)methyl]phenoxy]piperidine-1-carboxylate: compound D120

Under a nitrogen atmosphere, tert-butyl 4-[4-(chloromethyl)phenoxy]piperidine-1- carboxylate (compound BP126) (crude product, 20.0 g), $K_2CO_3$ (7.2 g, 51.9 mmol, 1.0 eq), 4- bromophenol (9.0 g, 51.9 mmol, 1.0 eq), and DMF (100 mL) were added to a 300 mL flask. This reaction mixture was stirred at 70°C for 16 hours. The reaction mixture was filtered, and the solid collected by filtration was washed with ethyl acetate (300 mL). The filtrate was separated, and the separated organic layer was washed with water (100 mL) and dried over $Na_2SO_4$. After filtration of the solid, the filtrate was concentrated under reduced pressure. The residue was purified by silica gel column chromatography (ethyl acetate/petroleum ether, 0-15%) to obtain tert-butyl 4-[4-[(4-bromophenoxy)methyl]phenoxy]piperidine-1- carboxylate (compound D120) as a white solid (11.0 g, 45.4%).
$^1$H-NMR (400 MHz, DMSO-$d_6$) δ: 7.46-7.44 (m, 2H), 7.37-7.34 (m, 2H), 6.99-6.96 (m, 4H), 5.00 (s, 2H), 4.58-4.54 (m, 1H), 3.68-3.64 (m, 2H), 3.20-3.13 (m, 2H), 1.91-1.89 (m, 2H), 1.55-1.46 (m, 2H), 1.41 (s, 9H).
LCMS (ESI, m/z): 362,364[M+H]$^+$: observed as a Boc-deprotected form.
Retention time: 2.187 min.
Analysis conditions: PH-AC-12.

Example 1-11-50: Synthesis of 4-[4-[(4-bromophenoxy)methyl]phenoxy]piperidine (compound D126)

[Formula 227]

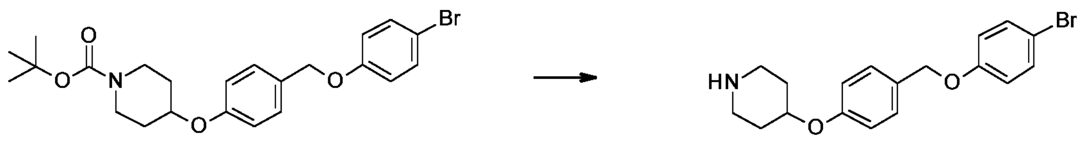

D120 D126

tert-Butyl 4-[4-[(4-bromophenoxy)methyl]phenoxy]piperidine-1-carboxylate (D120, 1.60 g, 3.46 mmol) and 4-MeTHP (34.6 mL) were added to a 100 mL flask. HMDS (2.21 mL, 10.4 mmol) and $Sn(OTf)_2$ (2.88 g, 6.92 mmol) were added thereto, and the mixture was stirred at room temperature for 1 hour. Triethylamine (2.4 mL, 17 mmol) was added thereto, and the mixture was stirred at room temperature for 1 hour. The reaction solution was cooled to 0°C. Water (8.7 mL) was added thereto, and the mixture was stirred at room temperature for 30 minutes. The mixture was filtered through celite using DCM (500 mL), and the collected filtrate was concentrated under reduced pressure. DMSO (20 mL) and formic acid (0.890 mL, 20.8 mmol) were added to the obtained residue, and the mixture was purified by reverse-phase silica gel column chromatography (0.1% solution of formic acid in acetonitrile/0.1% aqueous formic acid solution, 0-100%) to obtain the title compound D 126 (1.00 g, 2.76 mmol, 80%) as a white solid.

[Formula 228]

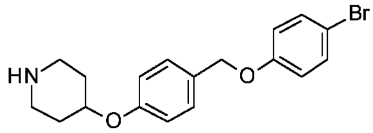

D126

$^1$H-NMR (400 MHz, $CDCl_3$) δ: 7.39-7.35 (2H, m), 7.33-7.30 (2H, m), 6.93-6.91 (2H, m), 6.86-6.82 (2H, m), 4.94 (2H, s), 4.38 (1H, dddd, J = 8.4, 8.0, 3.6, 3.6 Hz), 3.15 (2H, ddd, J = 14.0, 4.0, 4.0 Hz), 2.74 (2H, ddd, J = 12.4, 9.2, 3.2 Hz), 2.04-1.98 (2H, m), 1.72-1.63 (2H, m).

LCMS: m/z 362, 364 $[M+H]^+$.

Retention time: 0.780 min (analysis conditions SMD-FA05-1,305 ± 95 nm).

Example 1-11-51: Synthesis of solid-phase supported ArBr (compound D121-03R)

[Formula 229]

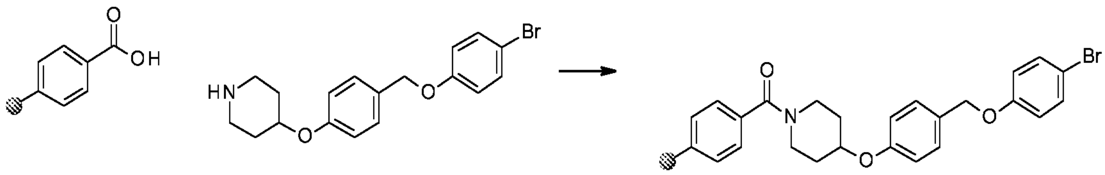

C001-03R D126 D121-03R

Solid-phase supported ArBr (compound D0121-03R) was synthesized by the condensation of carboxylic acid resin (C001-03R) (loading rate: 2.19 mmol/g, 2.00 g, 4.38 mmol) and 4-[4-[(4-bromophenoxy)methyl]phenoxy]piperidine (D126) (0.176 g, 0.486 mmol) by the same approach as in Example 1-1-8. Solid-phase purification and reaction monitoring (compound to be monitored: D121) were also performed in the same manner as in Example 1-1-8. As a result, compound D121 of interest (loading rate: 0.200 mmol/g, 2.87 g) was observed at a purity of 100%.

[Formula 230]

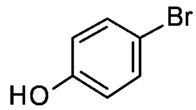

D121

Compound D121

Maximum wavelength: 226 and 281 nm.

Retention time: 0.880 min (analysis conditions SMD-FA05-1, 280 nm).

Example 1-11-52: Synthesis of compound D122-03R

Solid-phase experiment (exemplary reaction operation) in which Ar boronic acid ester (D122-03R) was synthesized using solid-phase supported ArBr (D121-03R) and 4,4,5,5-tetramethyl-2-(4,4,5,5-tetramethyl-1,3,2-dioxaborolan-2-yl)-1,3,2-dioxabolorane (D125)

[Formula 231]

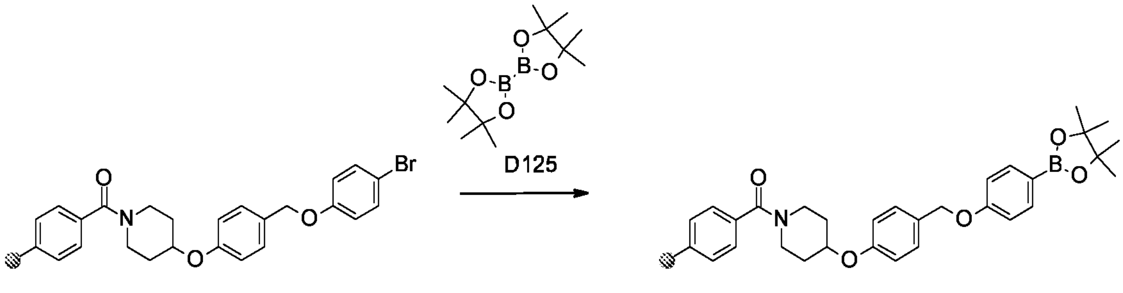


D121-03R D122-03R

Under a nitrogen atmosphere, solid-phase supported ArBr (D121-03R) (loading rate: 0.200 mmol/g, 20 mg, 4.0 μmol) and NMP (0.3 mL) were added to a 0.6 mL glass vial and shaken at room temperature for 1 hour. 4,4,5,5-Tetramethyl-2-(4,4,5,5-tetramethyl-1,3,2- dioxaboloran-2-yl)-1,3,2-dioxabolorane (D125, 20.3 mg, 0.080 mmol), potassium acetate (11.78 mg, 0.120 mmol), and Pd(dppf)$Cl_2$·DCM (3.27 mg, 4.0 μmol) were added thereto, and the mixture was shaken at 80°C for 2 hours.

Solid-phase purification

The suspension of the reaction solution and the resin was transferred onto a filter using NMP (0.4 mL) and washed three times with NMP (0.4 mL), three times with water (0.4 mL), three times with NMP/water = 1/1 (0.4 mL), three times with NMP (0.4 mL), three times with methanol (0.4 mL), and three times with DCM (0.4 mL), and the obtained solid phase was dried overnight under reduced pressure to obtain compound D122-03R (loading rate: 0.198 mmol/g, 16.0 mg).

Reaction monitoring

Under a nitrogen atmosphere, 20 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.1 M pentamethylbenzene (50 μL) for 5 minutes. The cleavage solution was filtered, and the solid phase was washed with NMP (50 μL). The obtained filtrate was diluted with MeCN (500 μL) to prepare an LC sample. The degree of progression of the reaction was measured by LC-MS analysis. As a result, 100 area% of compound D122 of interest was observed.

[Formula 232]

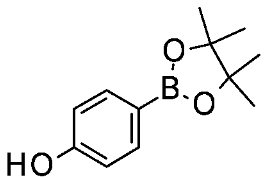

D122

Compound D122

Maximum wavelength: 238 nm.

Retention time: 1.007 min (analysis conditions SMD-FA05-1, 250 nm).

Example 1-11-53: Synthesis of solid-phase supported hetero-Ar-$NH_2$ (compound D072-03R)

[Formula 233]

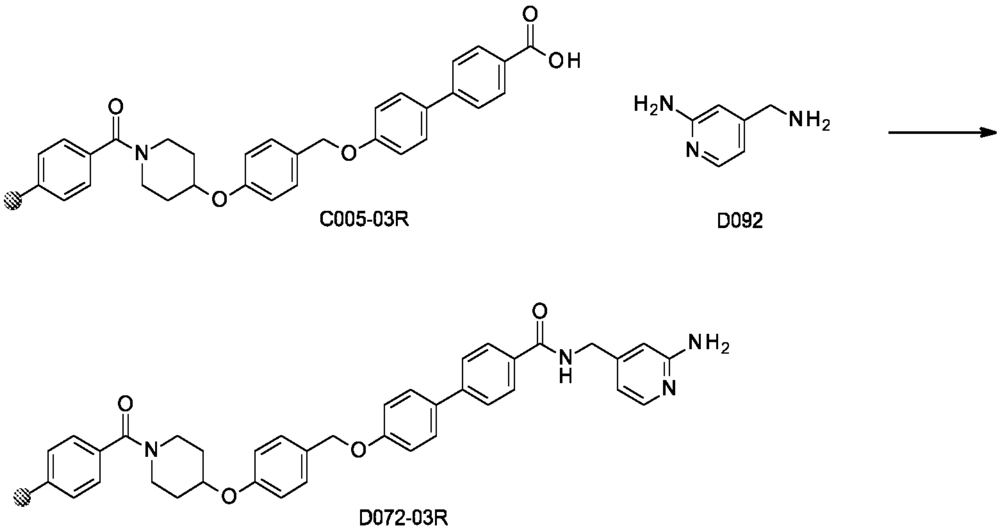

C005-03R D092

D072-03R

Solid-phase supported hetero-Ar-$NH_2$ (compound D072-03R) was synthesized by the condensation of solid-phase supported carboxylic acid (C005-03R, loading rate: 0.2011 mmol/g, 500 mg, 100.5 μmol) and 4-(aminomethyl)pyridine-2-amine (D092) (99 mg, 0.804 mmol) by the same approach as in Example 1-8-1. Solid-phase purification and reaction monitoring (compound to be monitored: D072) were also performed in the same manner as in Example 1-8-1. As a result, compound D072 of interest (loading rate: 0.197 mmol/g, 497.9 mg) was observed at a purity of 100%.

[Formula 234]

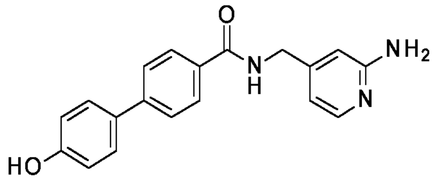

D072

Compound to be monitored: D072

LRMS: m/z 320[M+H]$^+$.

Retention time: 0.681 min (analysis conditions SMD-TFA05-RP, 299 nm).

Example 1-11-54: Synthesis of solid-phase supported hetero-Ar-$NH_2$ (compound D106-03R)

Solid-phase supported hetero-Ar-$NH_2$ (compound D106-03R) was synthesized as follows by the condensation of solid-phase supported amine (H003-03R) and 5- aminopyridine-2-carboxylic acid (D108).

[Formula 235]

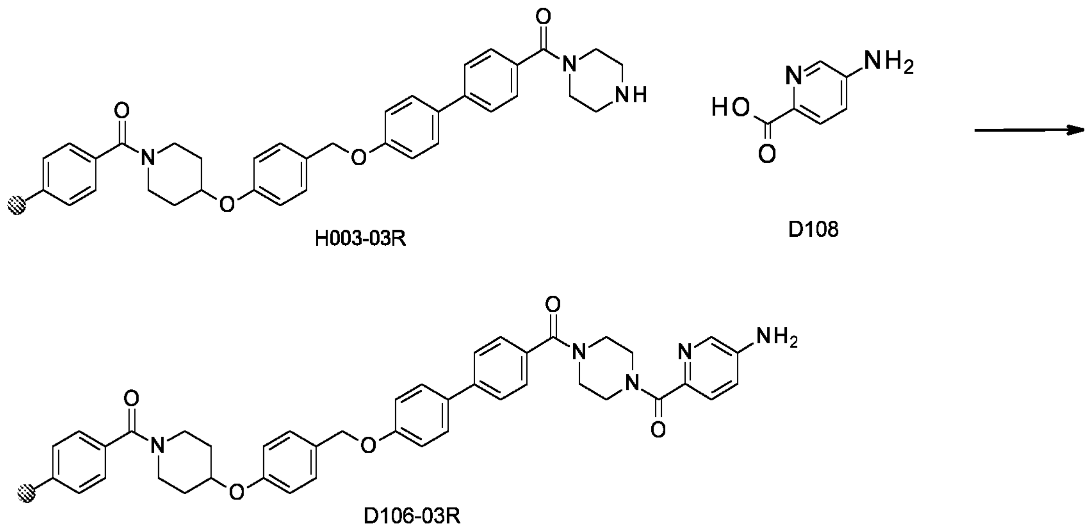

H003-03R

D108

D106-03R

Under a nitrogen atmosphere, compound H003-03R (loading rate: 0.198 mmol/g, 200 mg, 0.0396 mmol) and NMP (3.0 mL) were added to a 4 mL glass vial and shaken at room temperature for 1 hour. 5-Aminopyridine-2-carboxylic acid (D108, 49.0 mg, 0.355 mmol), HOAt (48.3 mg, 0.355 mmol), and DIC (55.2 μL, 0.355 mmol) were added thereto, and the mixture was shaken at room temperature for 2.5 hours.

Solid-phase purification

The suspension of the reaction solution and the resin was transferred onto a filter using NMP (2 mL) and washed three times with NMP (4 mL), three times with NMP/water = 1/1 (4 mL), three times with NMP (4 mL), three times with methanol (4 mL), three times with DCM (4 mL), and three times with heptane (4 mL), and the obtained solid phase was dried overnight under reduced pressure to obtain compound D106-03R (loading rate: 0.192 mmol/g, 434 mg).

Reaction monitoring

The suspension of the reaction solution and the resin (0.012 mL) was transferred onto a filter, washed three times with NMP (0.1 mL), three times with methanol (0.1 mL), and three times with DCM (0.1 mL), and then dipped in a 10% solution of TFA in DCM containing 0.05 M pentamethylbenzene (0.05 mL) for 5 minutes. After filtration, the resin on the filter was washed with NMP (0.05 mL). Acetonitrile (0.50 mL) was added to the obtained filtrate to prepare an LC sample. The degree of progression of the reaction was measured by LC-MS analysis. As a result, compound D106 of interest was observed at a purity of 93.2%.

[Formula 236]

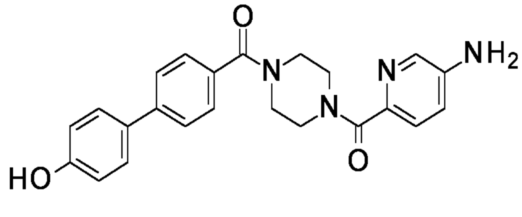

D106

Compound D106
LRMS: m/z 403[M+H]$^+$.
Retention time: 0.667 min (analysis conditions SMD-FA05-1, 299 nm)

Example 1-11-55: Synthesis of solid-phase supported N-Boc amine (compound D116-03R)

[Formula 237]

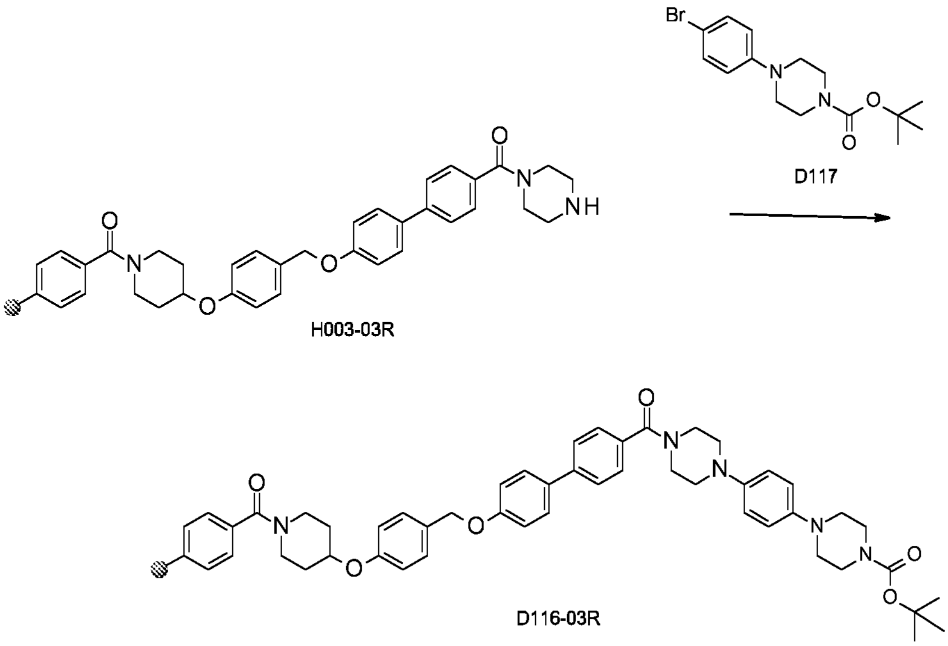

D117

H003-03R

D116-03R

Under a nitrogen atmosphere, compound H003-03R (loading rate: 0.197 mmol/g, 100 mg, 0.0197 mmol), tert-butyl 4-(4-bromophenyl)piperazine-1-carboxylate (D117, 33.6 mg, 0.099 mmol), and NMP (2.00 mL) were added to a 3 mL glass vial and shaken at room temperature for 1 hour. RuPhos Pd G4 (16.75 mg, 0.020 mmol) and 2 M P2tBu in THF (0.148 mL, 0.296 mmol) were added thereto, and the mixture was shaken at 40°C for 2 hours.

Solid-phase purification

The suspension of the reaction solution and the resin was transferred onto a filter using NMP (1 mL) and washed three times with NMP (2 mL), three times with 0.05 M NAC in NMP (2 mL), three times with NMP/water = 1/1 (2 mL), three times with NMP (2 mL), three times with MeOH (3 mL), three times with DCM (2 mL), and three times with heptane (2 mL), and the obtained solid phase was dried overnight under reduced pressure. The same reaction and solid-phase purification as above were performed using the obtained solid phase to obtain compound D116-03R (loading rate: 0.187 mmol/g, 109 mg).

Reaction monitoring

The suspension of the reaction solution and the resin (0.012 mL) was transferred onto a filter, washed three times with NMP (0.1 mL), three times with methanol (0.1 mL), and three times with DCM (0.1 mL), and then dipped in a 10% solution of TFA in DCM containing 0.05 M pentamethylbenzene (0.05 mL) for 5 minutes. After filtration, the resin on the filter was washed with NMP (0.05 mL). Acetonitrile (0.50 mL) was added to the obtained filtrate to prepare an LC sample. The degree of progression of the reaction was measured by LC-MS analysis. As a result, compound D116 of interest was observed at a purity of 86.0%.

[Formula 238]

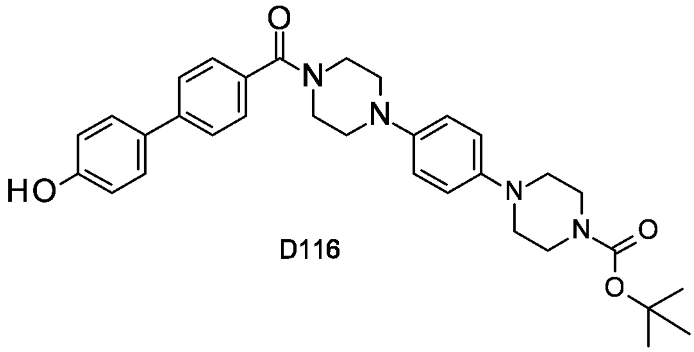

D116

Compound D116

LRMS: m/z 543[M+H]+.

Retention time: 1.055 min (analysis conditions SMD-FA05-1, 290 nm)

Example 1-11-56: Synthesis of solid-phase supported amine (compound D109-03R)

[Formula 239]

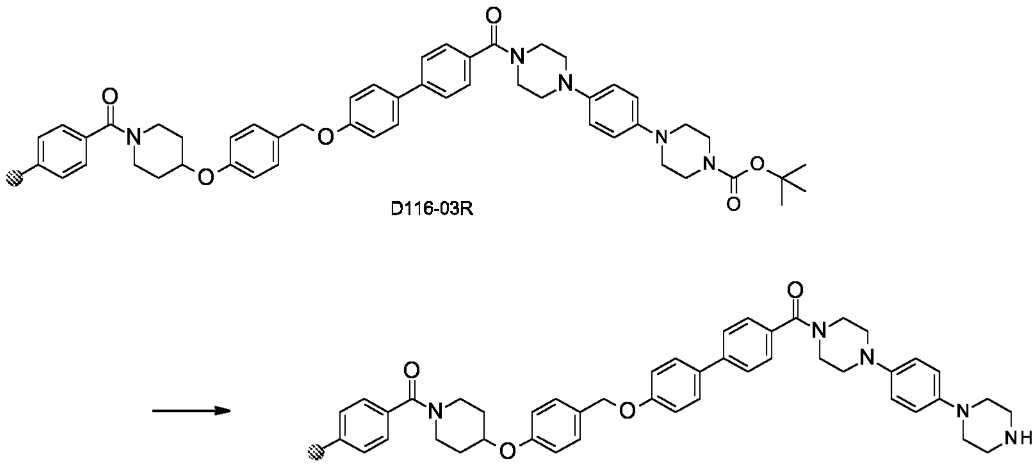

D116-03R

D109-03R

Under a nitrogen atmosphere, compound D116-03R (loading rate: 0.187 mmol/g, 100 mg, 0.0187 mmol) and 2-methyltetrahydrofuran (2.0 mL) were added to a 3 mL glass vial and shaken at room temperature for 1 hour. DTBP (0.123 mL, 0.561 mmol) and $Tm(OTf)_3$ (230 mg, 0.374 mmol) were added thereto, and the mixture was shaken at 50°C for 23 hours.

Solid-phase purification

1 M DMEDA in NMP (0.935 mL, 0.935 mmol) was added to the reaction solution, and the mixture was shaken at room temperature for 2 hours. The suspension of the reaction solution and the resin was transferred onto a filter using NMP (1 mL) and washed three times with 0.2 M DMEDA in NMP (2 mL), three times with NMP/water = 1/1 (2 mL), three times with 0.2 M $TBAHSO_4$ + DTBP in NMP (2 mL), three times with NMP/water = 1/1 (2 mL), three times with NMP (2 mL), three times with MeOH (2 mL), three times with DCM (2 mL), and three times with heptane (2 mL), and the obtained solid phase was dried overnight under reduced pressure to obtain compound D109-03R (loading rate: 0.191 mmol/g, 107.3 mg).

Reaction monitoring

The suspension of the reaction solution and the resin (0.012 mL) was transferred onto a filter, washed three times with NMP (0.1 mL), three times with methanol (0.1 mL), and three times with DCM (0.1 mL), and then dipped in a 10% solution of TFA in DCM containing 0.05 M pentamethylbenzene (0.05 mL) for 5 minutes. After filtration, the resin on the filter was washed with NMP (0.05 mL). Acetonitrile (0.50 mL) was added to the obtained filtrate to prepare an LC sample. The degree of progression of the reaction was measured by LC-MS analysis. As a result, compound D109 of interest was observed at a purity of 92.5%.

[Formula 240]

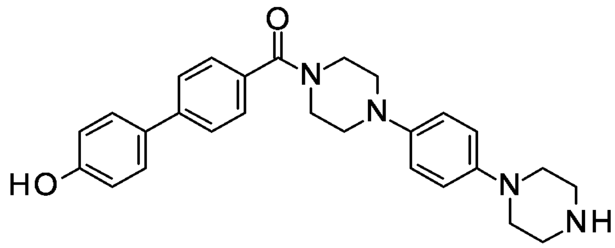

D109

Compound D109
LRMS: m/z 443[M+H]$^+$.
Retention time: 0.605 min (analysis conditions SMD-FA05-1, 290 nm)

Example 1-11-57: Synthesis of solid-phase supported N-Boc amine (compound D119-03R)

[Formula 241]

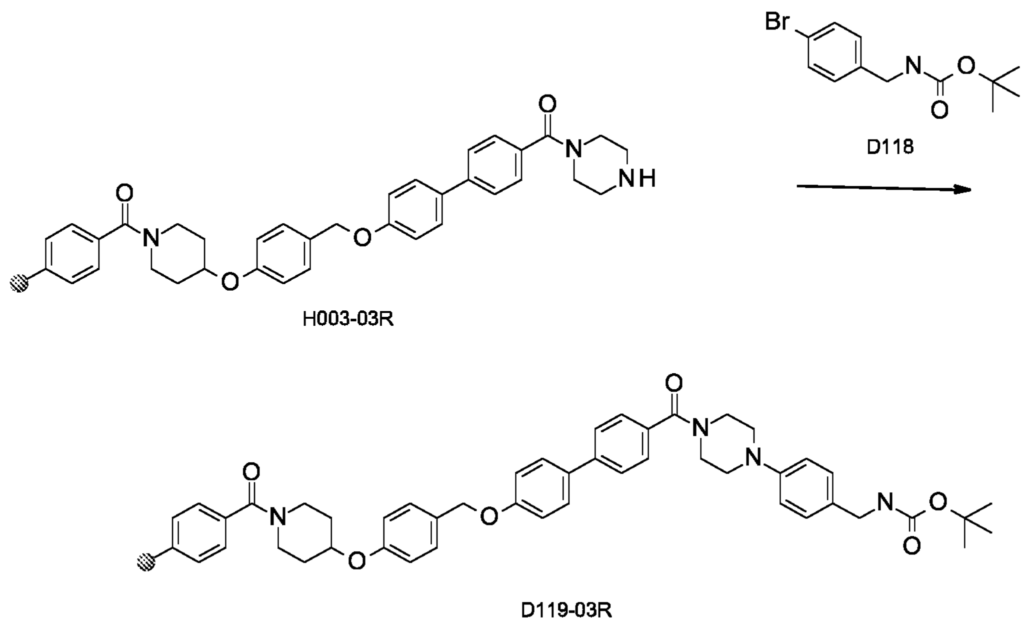

Solid-phase supported N-Boc amine (compound D119-03R) was synthesized by the same approach as in Example 1-11-55 using solid-phase supported amine (H003-03R) and tert-butyl N-[(4-bromophenyl)methyl]carbamate (D118). Solid-phase purification and reaction monitoring (compound to be monitored: D119) were also performed in the

same manner as in Example 1-11-55. As a result, compound D119 of interest was observed at a purity of 86.5%.

[Formula 242]

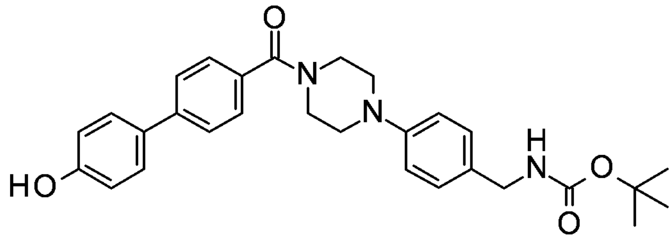

D119

Compound D119

LRMS: m/z 488[M+H]+.

Retention time: 1.087 min (analysis conditions SMD-FA05-1, 290 nm)

Example 1-11-58: Synthesis of solid-phase supported amine (compound D111-03R)

[Formula 243]

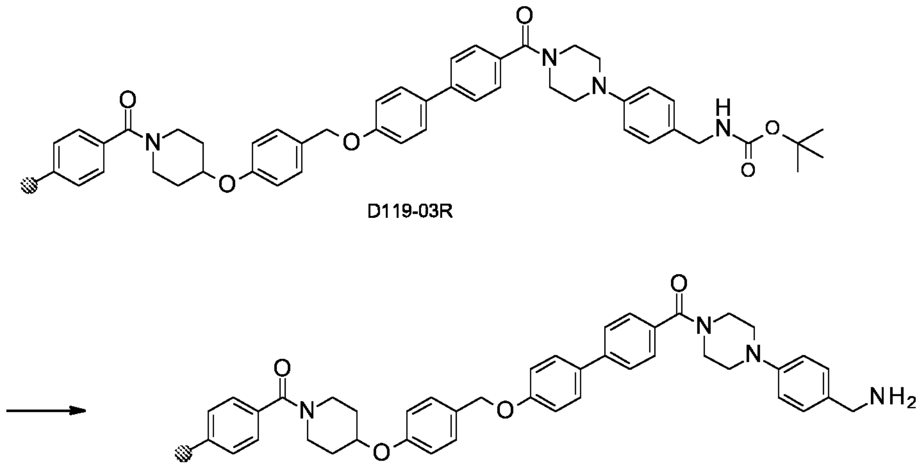

D119-03R

D111-03R

Solid-phase supported amine (compound D111-03R) was synthesized by the same approach as in Example 1-11-56 using solid-phase supported N-Boc amine (D119-03R). Solid-phase purification and reaction monitoring (compound to be monitored: D111) were also performed in the same manner as in Example 1-11-56. As a result, compound D111 of interest was observed at a purity of 93.6%.

[Formula 244]

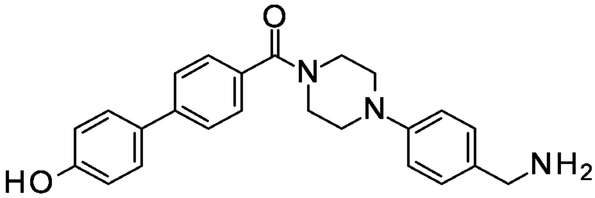

D111

Compound D111

Maximum wavelength: 267 nm.

Retention time: 0.611 min (analysis conditions SMD-FA05-1, 290 nm)

Example 1-11-59: Synthesis of solid-phase supported sulfinic acid (compound D093-03R)

Solid-phase supported sulfinic acid (compound D093-03R) was synthesized as follows from solid-phase supported ArI (D090-03R).

[Formula 245]

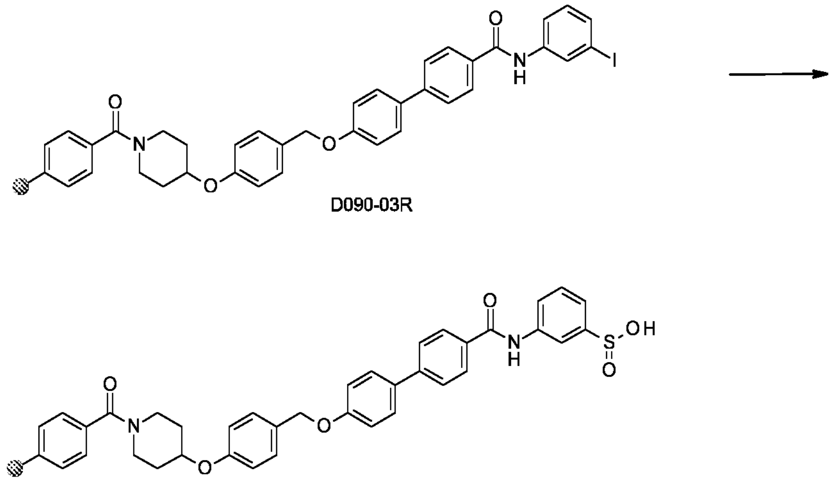

D090-03R

D093-03R

Under a nitrogen atmosphere, compound D090-03R (loading rate: 0.193 mmol/g, 20.0 mg, 3.86 μmol), DABSO (9.28 g, 0.039 mmol), and 1,4-dioxane (0.4 mL) were added to a 0.6 mL glass vial and shaken at room temperature for 1 hour. XPhos Pd G4 (3.3 mg, 3.9 μmol) and BTMG (7.7 μL, 0.039 mmol) were added thereto, and the mixture was shaken at 80°C for 4 hours.

Solid-phase purification

The suspension of the reaction solution and the resin was transferred onto a filter using NMP (0.4 mL) and washed three times with NMP/water (0.4 mL), three times with NMP (0.4 mL), three times with 0.2 M NAC in NMP/water = 5/1 (0.4 mL), three times with 0.05 M $TBAHSO_4$ + DTBP in NMP (0.4 mL), three times with 0.05 M NMM in NMP (0.4 mL), three times with NMP/water = 1/1 (0.4 mL), three times with NMP (0.4 mL), three times with MeOH (0.4 mL), three times with DCM (0.4 mL), and three times with heptane (0.4 mL), and the obtained solid phase was dried overnight under reduced pressure to obtain compound D093-03R (loading rate: 0.196 mmol/g, 18.9 mg).

Reaction monitoring

The suspension of the reaction solution and the resin (12 μL) was transferred onto a filter, washed three times with NMP (0.1 mL), three times with methanol (0.1 mL), and three times with DCM (0.1 mL), and then dipped in a 10% solution of TFA in DCM containing 0.05 M pentamethylbenzene (0.05 mL) for 5 minutes. After filtration, the resin on the filter was washed with DMA (0.05 mL). Acetonitrile (0.50 mL) was added to the obtained filtrate to prepare an LC sample. The degree of progression of the reaction was measured by LC-MS analysis. As a result, compound D093 of interest was observed at a purity of 96.5%.

[Formula 246]

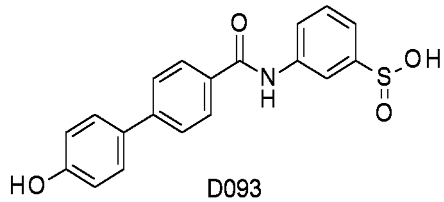

D093

Compound D093

LRMS: m/z 354[M+H]+.

Retention time: 0.736 min (analysis conditions SMD-FA05-1, 299 nm).

Example 1-11-60: Synthesis of solid-phase supported sulfonyl chloride (compound D095-03R)

Solid-phase supported sulfonyl chloride (compound D095-03R) was synthesized as follows by the sulfonamidation of solid-phase supported N-Me aniline (H002-03R) and benzene-1,3-disulfonyl chloride (D096).

[Formula 247]

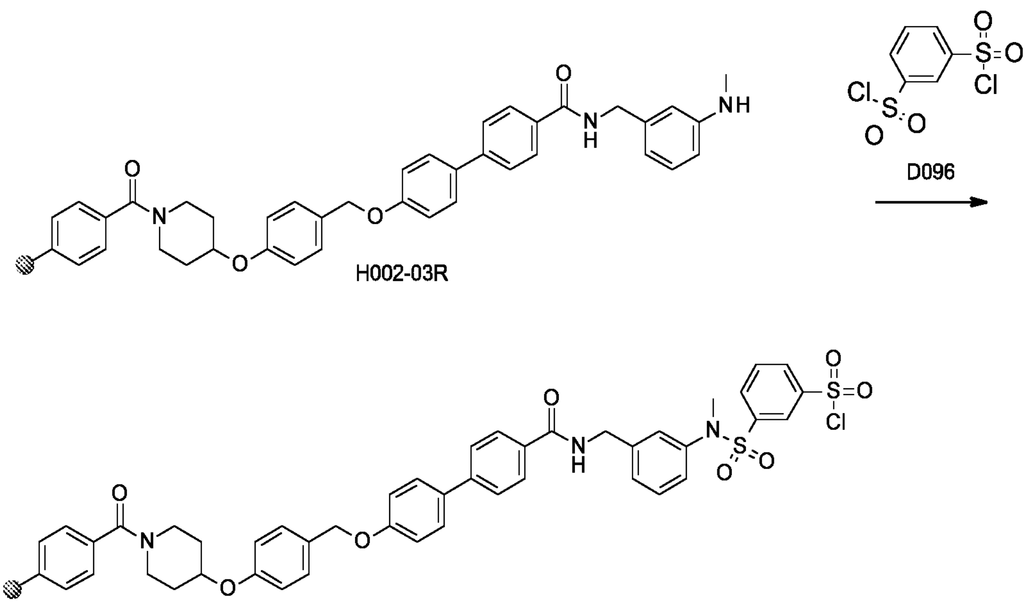

Under a nitrogen atmosphere, compound H002-03R (loading rate: 0.196 mmol/g, 0.020 g, 3.92 μmol) and THF (0.4 mL) were added to a 0.6 mL glass vial and shaken at room temperature for 1 hour. Pyridine (4.76 μL, 0.059 mmol), NMI (0.625 μL, 7.84 μmol), and benzene-1,3-disulfonyl chloride (D096, 32.4 mg, 0.118 mmol) were added thereto, and the mixture was shaken at room temperature for 2 hours.

Solid-phase purification

The suspension of the reaction solution and the resin was transferred onto a filter using DCM (0.4 mL) and washed three times with DCM (0.4 mL) and three times with heptane (0.4 mL), and the obtained solid phase was dried overnight under reduced pressure to obtain compound D095-03R (19.9 mg, 0.187 mmol/g).

Reaction monitoring

The suspension of the reaction solution and the resin (12 μL) was transferred onto a filter, washed three times with DMA (0.1 mL), three times with methanol (0.1 mL), and three times with DCM (0.1 mL), and then dipped in a 10% solution of TFA in DCM containing 0.05 M pentamethylbenzene (0.05 mL) for 5 minutes. After filtration, the resin on the

filter was washed with DMA (0.05 mL). Acetonitrile (0.50 mL) was added to the obtained filtrate to prepare an LC sample. The degree of progression of the reaction was measured by LC-MS analysis. As a result, compound D095 of interest was observed at a purity of 91.0%.

[Formula 248]

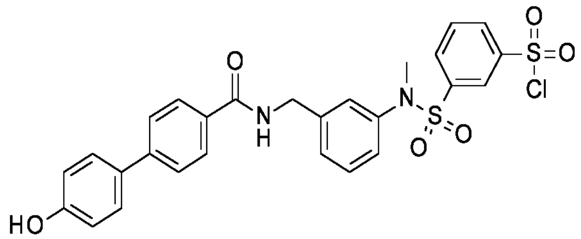

D095

Compound D095

LRMS: m/z 571[M+H]$^+$.

Retention time: 1.156 min (analysis conditions SMD-FA05-1, 299 nm)

Example 1-11-61: Synthesis of compound B103-03R

[Formula 249]

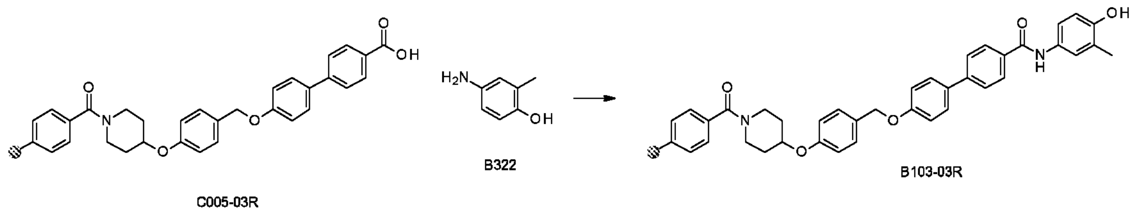

Under a nitrogen atmosphere, compound C005-03R (loading rate: 0.201 mmol/g, 400 mg, 0.080 mmol) and NMP (6.6 mL) were added to a 10 mL glass vial and shaken at room temperature for 1 hour. 4-Amino-2-methylphenol (B322) (30 mg, 0.241 mmol), HOAt (33 mg, 0.241 mmol), and DIC (37 μL, 0.241 mmol) were added thereto, and the mixture was shaken at 60°C for 5 hours. Together with a lot synthesized from compound C005-03R (loading rate: 0.201 mmol/g, 100 mg, 0.020 mmol) in the same manner as above, the suspension of the reaction solution and the resin was wholly transferred onto a filter and washed three times with NMP (10 mL), three times with NMP-water (1:1, 10 mL), three times with NMP (10 mL), three times with methanol (10 mL), three times with DCM (10 mL), and three times with heptane (10 mL), and the obtained solid phase was dried overnight under reduced pressure to obtain compound B103-03R (loading rate: 0.197 mmol/g, 0.546 g).

Reaction monitoring

The suspension of the reaction solution and the resin (15 μL) was transferred onto a filter, washed three times with NMP (100 μL), three times with methanol (100 μL), and three times with DCM (100 μL), and then dipped in a 10% solution of TFA in DCM containing 0.05 M pentamethylbenzene (50 μL) for 2 minutes. After filtration, DMA (200 μL) was added to the filtrate. Acetonitrile (100 μL) was added to a 50 μL aliquot of the obtained solution to prepare an LC sample. The degree of progression of the reaction was measured by LC-MS analysis. As a result, 99% of compound B103 of interest was observed.

[Formula 250]

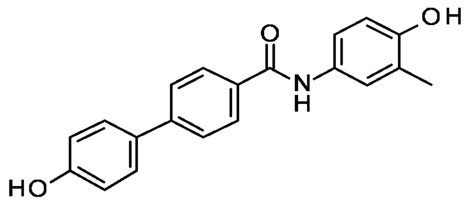

Compound B103

LRMS: m/z 320[M+H]+.

Retention time: 0.867 min (analysis conditions SMD-FA05-1, 299 nm).

Example 1-11-62: Synthesis of compound B104-03R

[Formula 251]

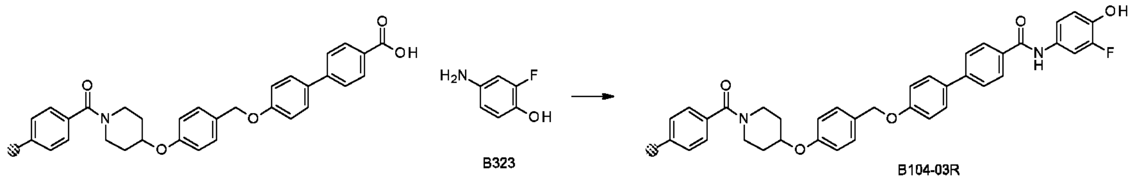

C005-03R B323 B104-03R

Under a nitrogen atmosphere, compound C005-03R (loading rate: 0.201 mmol/g, 400 mg, 0.080 mmol) and NMP (6.6 mL) were added to a 10 mL glass vial and shaken at room temperature for 1 hour. 4-Amino-2-fluorophenol (B323) (31 mg, 0.241 mmol), HOAt (33 mg, 0.241 mmol), and DIC (37 μL, 0.241 mmol) were added thereto, and the mixture was shaken at 60°C for 5 hours. Together with a lot synthesized from compound C005-03R (loading rate: 0.201 mmol/g, 100 mg, 0.020 mmol) in the same manner as above, the suspension of the reaction solution and the resin was wholly transferred onto a filter and washed three times with NMP (10 mL), three times with NMP-water (1:1, 10 mL), three times with NMP (10 mL), three times with methanol (10 mL), three times with DCM (10 mL), and three times with heptane (10 mL), and the obtained solid phase was dried overnight under reduced pressure to obtain compound B104-03R (loading rate: 0.197 mmol/g, 0.526 g).

Reaction monitoring

The suspension of the reaction solution and the resin (15 μL) was transferred onto a filter, washed three times with NMP (100 μL), three times with methanol (100 μL), and three times with DCM (100 μL), and then dipped in a 10% solution of TFA in DCM containing 0.05 M pentamethylbenzene (50 μL) for 2 minutes. After filtration, DMA (200 μL) was added to the filtrate. Acetonitrile (100 μL) was added to a 50 μL aliquot of the obtained solution to prepare an LC sample. The degree of progression of the reaction was measured by LC-MS analysis. As a result, 97% of compound B104 of interest was observed.

[Formula 252]

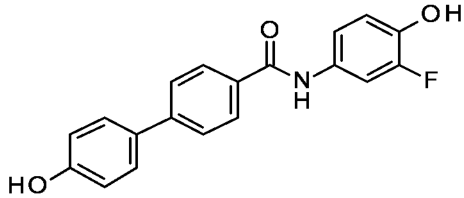

Compound B104

LRMS: m/z 324[M+H]+.

Retention time: 0.857 min (analysis conditions SMD-FA05-1, 299 nm).

Example 2; Study on each bond formation reaction to be applied to library synthesis

For supplying a library allowed to have diversity by the linking of core blocks via linkers through the reaction between building blocks (BB), it is essential to clarify the scope of substrate application under reaction conditions for library synthesis, and carry out the reaction at a high yield and a high purity. Alternatively, it is essential to find reaction conditions under which a wider scope of substrates can be reacted at a high yield and a high purity, and apply the reaction conditions to library synthesis.

If there is a generally known problem that may reduce a purity in arbitrary reaction, it is preferred to apply reaction conditions capable of solving the problem to library synthesis. In Example 2, search for reaction conditions applicable to library synthesis will be illustrated for each bond formation reaction.

Example 2-1: Experiment to confirm scope of substrate application to Mitsunobu reaction as typical example of identifying substrate scope that can be applied to library synthesis

Hereinafter, an exemplary method for identifying an applicable substrate scope under reaction conditions expected to attain a wide scope of substrate application to Mitsunobu reaction (Ar-O-R bond formation) will be illustrated. The scope given below is a mere illustration of an exemplary identification method, and the present invention is not limited by the scope given below in actual application to library synthesis.

Example 2-1-1: Liquid-phase experiment to confirm difference in reactivity depending on electronic factor of Ar-OH (arenol) with R-OH (alcohol) fixed to 1-propanol in Mitsunobu reaction for forming Ar-O-R bond from Ar-OH (arenol) and R-OH (alcohol)

Difference in reactivity was confirmed in substrates prepared by introducing various substituents to phenol as Ar-OH (arenol). The factor that influences Mitsunobu reaction includes an electronic factor of a nucleophile (Ar-OH (arenol)).

The difference in reactivity was confirmed in substrates prepared by the introduction of various substituents. The electronic factor was confirmed to influence reactivity by calculating pKa using ADMET predictor or ACD (Advanced Chemistry Development (ACD/Labs) Software V11.02 (copyright 1994-2020 ACD/Labs)).

Mitsunobu reaction (exemplary reaction operation) between phenol (unsubstituted) and 1-propanol

Under a nitrogen atmosphere, phenol (10.00 mg, 0.106 mmol, 1.0 equivalents) and THF (1.06 mL, 0.10 M) were added to a 3 mL screw-cap vial. Subsequently, 1-propanol (31.9 μL, 0.425 mmol, 4.0 equivalents), TMAD (36.6 mg, 0.213 mmol, 2.0 equivalents), and tributylphosphine (52.4 μL, 0.213 mmol, 2.0 equivalents) were added thereto at room temperature, and the obtained mixture was stirred at room temperature.

Reaction monitoring

Under a nitrogen atmosphere, 1 μL of the reaction mixture was collected 30 minutes after the start of reaction and dissolved in 200 μL of acetonitrile to prepare an LC sample. The reaction was monitored by LC-MS analysis (analysis conditions SQD-FA05-1).

As a result, the completion of the reaction within 30 minutes was confirmed.

[Formula 253]

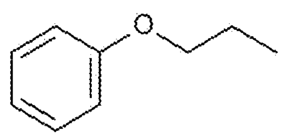

Compound F001

Compound F001

Retention time: 0.93 min (analysis conditions SQD-FA05-1)

(Retention time of the starting material phenol: 0.50 min (analysis conditions SQD- FA05-1))

Reaction was also carried out as to substrates Ar-OH (arenol) other than phenol under similar conditions and operation as above.

Ar-OH (1.0 equivalent), THF (0.10 M), 1-propanol (4.0 equivalents), TMAD (2.0 equivalents), tributylphosphine (2.0 equivalents), stirring at room temperature.

The results are as shown in Table X1 below.

The reaction monitoring was basically performed by LC-MS analysis (measurement conditions SQD-FA05-1) 30 minutes, 1 hour, 2 hours, and 3 hours after the start of reaction, unless otherwise specified. The fastest time when the consumption of the starting material Ar-OH (arenol) was confirmed was described.

The conversion rate through reaction (conv.) was calculated as follows, unless otherwise specified.

Conversion rate through reaction (conv.) = UV area% of the product (Ar-O-nPr) at 280 nm/ (UV area% of the product (Ar-O-nPr) at 280 nm + UV area% of the starting material (Ar-OH) at 280 nm)

[Table 3]

Table X1

| run | Substrate (Ar-OH) | pKa of substrate (ACD) | Product | Compound No. (product) | Time | Conv. at time of confirmation (%) | Substrate retention time (min) | Product retention time (min) | Maximum wavelength of diode array substrate (nm) | Maximum wavelength of diode array product (nm) | Product name |
|---|---|---|---|---|---|---|---|---|---|---|---|
| 1 | 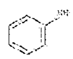 | 9.86 | 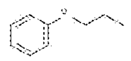 | F001 | 30min | 100 | 0.50 | 0.93 | 271.0 | 272.0 | Propoxybenzene |
| 2 | 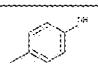 | 10.21 | 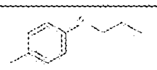 | F002 | 30min | 100 | 0.59 | 1.00 | 279.0 | 279.0 | 1-Methyl-4-propoxybenzene |
| 3 | 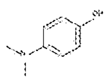 | 10.11 | 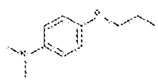 | F003 | 30min | 100 | 0.16 | 0.43 | 271.0 | 272.0 | N,N-Dimethyl-4-propoxyaniline |
| 4 | 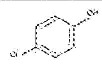 | 9.47 | 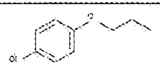 | F004 | 30min | 100 | 0.64 | 1.02 | 282.0 | 282.0 | 1-Chloro-4-propoxybenzene |
| 5 | 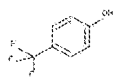 | 8.51 | 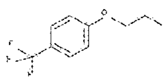 | F005 | 30min | 100 | 0.71 | 1.02 | 270.0 | 270.0 | 1-Propoxy-4-(trifluoromethyl) benzene |
| 6 | 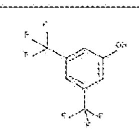 | 7.96 | 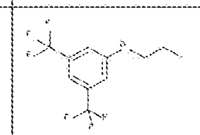 | F006 | 3h | 100 | 0.84 | 1.12 | 284.0 | 283.0 | 1-Propoxy-3,5-bis (trifluoromethyl) benzene |
| 7 | 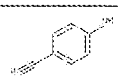 | 7.79 | 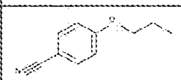 | F007 | 1h | 100 | 0.50 | 0.84 | 250.0 | 249.0 | 4-Propoxybenzonitrile |
| 8 | 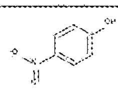 | 7.23 | 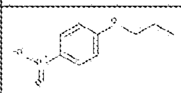 | F008 | 3h | 99.29 | 0.56 | 0.89 | 320.0 | 314.0 | 1-Nitro-4-propoxybenzene |
| 9 | 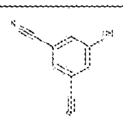 | 7.20 | 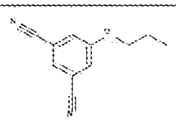 | F009 | 2h | 100 | 0.55 | 0.84 | 311.0 | 313.0 | 5-Propoxyisophthalonitrile |
| 10 | 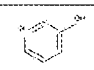 | 9.00 | 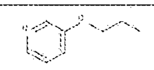 | F010 | 30min | 100 | 0.13 | 0.28 | 282.9 | 284.9 | 3-Propoxypyridine |
| 11 | 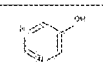 | 6.60 | 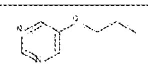 | F011 | 30min | 100 | 0.14 | 0.46 | 271.9 | 273.9 | 5-Propoxypyrimidine |

From the results described above, the influence of the electronic status of Ar-OH (arenol) in reaction on the reaction was confirmed. Particularly, when the pKa value fell below 8, it was confirmed that a time of 30 minutes or longer is required for the completion of reaction under the same reaction conditions. Particularly, in the case of using 4-nitrophenol (pKa: 7.23) as a substrate, the reaction was not completed even 3 hours after its start, and 0.71% of unreacted 4-nitrophenol was confirmed (run 8).

In the case of using a heterocyclic compound such as pyridin-3-ol or pyrimidin-5-ol as Ar-OH (arenol) serving as a substrate, this reaction was able to be confirmed to progress without problems (runs 10 and 11). In these cases, the reaction was completed within 30 minutes.

Example 2-1-2: Liquid-phase experiment to confirm difference in reactivity depending on steric factor of Ar-OH (arenol) with R-OH (alcohol) fixed to 1-propanol in Mitsunobu reaction for forming Ar-O-R bond from Ar-OH (arenol) and R-OH (alcohol)

When R-OH (alcohol) was fixed to 1-propanol in Mitsunobu reaction, the influence of a steric factor of a nucleophile (Ar-OH (arenol)) on the reaction was confirmed as described below.
The confirmation experiment was conducted by the method described in Example 2- 1-1. The results are as shown in Table X2.
The reaction monitoring was basically performed by LC-MS analysis (measurement conditions SQD-FA05-1) 30 minutes, 1 hour, 2 hours, and 3 hours after the start of reaction, unless otherwise specified. The time when the consumption of the starting material Ar-OH (arenol) was confirmed was described.
The conversion rate through reaction (conv.) was calculated as follows, unless otherwise specified.

Conversion rate through reaction (conv.) = UV area% of the product (Ar-O-nPr) at 280 nm/ (UV area% of the product (Ar-O-nPr) at 280 nm + UV area% of the starting material (Ar-OH) at 280 nm)

As for run 7 and run 8 of Table X2, the confirmation was performed at a UV area at 270 nm instead of 280 nm.

[Table 4]

Table X2

| run | Substrate (Ar-OH) | pKa of substrate (ACD) | Product | Compound No. (product) | Time required for completion of reaction | Substrate retention time (min) | Product retention time (min) | Maximum wavelength of diode array substrate (nm) | Maximum wavelength of diode array product (nm) | Name |
|---|---|---|---|---|---|---|---|---|---|---|
| 1 | 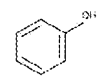 | 9.86 | 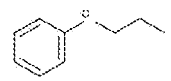 | F001 | <30min | 0.50 | 0.93 | 271.0 | 272.0 | Propoxybenzene |
| 2 | 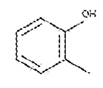 | 10.32 | 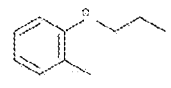 | F012 | <30min | 0.61 | 1.04 | 272.0 | 272.0 | 1-Methyl-2-propoxybenzene |
| 3 | 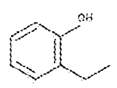 | 10.27 | 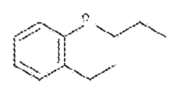 | F013 | <30min | 0.70 | 1.10 | 273.0 | 272.0 | 1-Ethyl-2-propoxybenzene |
| 4 | 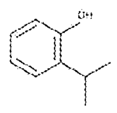 | 10.49 | 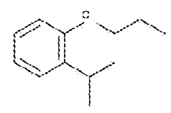 | F014 | <30min | 0.77 | 1.15 | 273.0 | 272.0 | 1-Isopropyl-2-propoxybenzene |
| 5 | 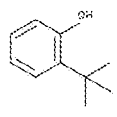 | 11.34 | 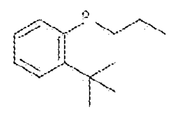 | F015 | <30min | 0.80 | 1.13 | 271.9 | 270.9 | 1-(tert-Butyl)-2-propoxybenzene |
| 6 | 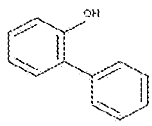 | 10.00 | 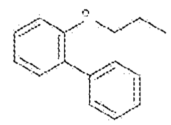 | F016 | <30min | 0.79 | 1.09 | 244.0, 286.0 | 244.0, 286.0 | 2-Propoxy-1,1'-biphenyl |
| 7 | 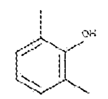 | 10.66 | 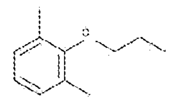 | F017 | <30min (confirmed at 270 nm) | 0.70 | 1.05 | 271.0 | 264.0 | 1,3-Dimethyl-2-propoxybenzene |
| 8 | 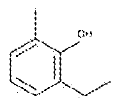 | 10.62 | 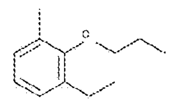 | F018 | <30min (confirmed at 270 nm) | 0.78 | 1.11 | 272.0 | 264.0 | 1-Ethyl-3-methyl-2-propoxybenzene |

As seen from the results described above, the reaction was completed within 30 minutes for all the examined substrates.

From the results of Example 2-1-1 and Example 2-1-2 taken together, it was confirmed that an electronic factor has larger influence as a factor that influences the success or failure of the completion of reaction in the examined scope.

Based on the results described above, Ar-OH (arenol) was fixed to 4-nitrophenol, which took time to complete reaction, and used as a substrate for the confirmation of reactivity in Example 2-1-3 or later to confirm reactivity with various R-OH (alcohol).

Example 2-1-3: Liquid-phase experiment to confirm difference in reactivity with various R-OH (alcohol) with Ar-OH (arenol) fixed to 4-nitrophenol in Mitsunobu reaction for forming Ar-O-R bond from Ar-OH (arenol) and R-OH (alcohol).

Reaction was carried out under similar conditions and operation as in Example 2-1-1 with Ar-OH (arenol) fixed to 4-nitrophenol.

4-Nitrophenol (1.0 equivalent), THF (0.10 M), R-OH (4.0 equivalents), TMAD (2.0 equivalents), tributylphosphine (2.0 equivalents), stirring at room temperature.

The reaction was monitored by LC-MS analysis (measurement conditions SQD- FA05-1). The results are as shown in Table X3.

The conversion rate through reaction (conv.) was calculated as follows, unless otherwise specified.

Conversion rate through reaction (conv.) = UV area% of the product (Ar-O-nPr) at 280 nm/ (UV area% of the

product (Ar-O-nPr) at 280 nm + UV area% of the starting material (Ar-OH) at 280 nm)

[Table 5]

Table X3

| run | Substrate (R-OH) | ASA of substrate | Product | Compound No. (product) | Reaction time | Conv. (%) | Product retention time (min) | Maximum wavelength of diode array substrate(nm) | Name |
|---|---|---|---|---|---|---|---|---|---|
| 1 | 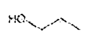 | 73.3 | 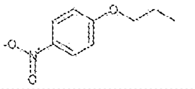 | F008 | 3h | 99.29 | 0.89 | 314.0 | 1-Nitro-4-propoxybenzene |
| 2 | 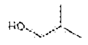 | 50.5 | 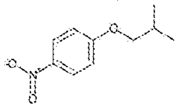 | F019 | 96h | 99.20 | 0.96 | 314.0 | 1-Isobutoxy-4-nitrobenzene |
| 3 | 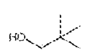 | 6 | 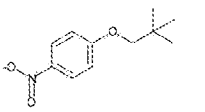 | F020 | 20h (1h: rt. 19h, 60°C) | 7.22 | 1.02 | 314.0 | 1-(Neopentyloxy)-4-nitrobenzene |
| 4 | 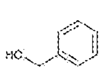 | 74.9 | 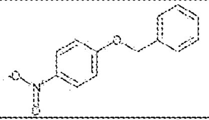 | F021 | 30min | 100 | 0.92 | 310.0 | 1-(Benzyloxy)-4-nitrobenzene |
| 5 | 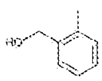 | 61.4 | 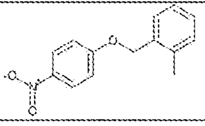 | F022 | 30min | 100 | 0.97 | 312.0 | 1-Methyl-2-((4-nitrophenoxy)methyl) benzene |
| 6 | 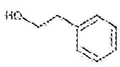 | 50.6 | 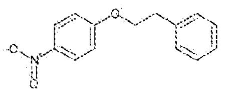 | F023 | 5h | 99.28 | 0.96 | 312.0 | 1-Nitro-4-phenethoxybenzene |
| 7 | 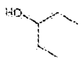 | 18.3 | 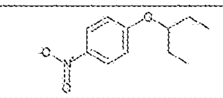 | F024 | 20h | 100 | 1.00 | 317.0 | 1-Nitro-4-(pentan-3-yloxy)benzene |
| 8 | 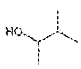 | 22.9 | 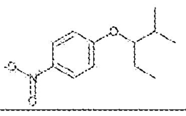 | F025 | 72h | 89.57 | 1.06 | 318.0 | 1-((2-Methylpentan-3-yloxy)-4-nitrobenzene |
| 9 | 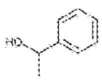 | 31.3 | 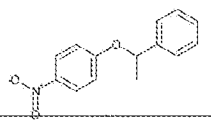 | F026 | 1h | 100 | 0.96 | 312.0 | 1-Nitro-4-(1-phenylethoxy)benzene |
| 10 | 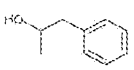 | 33.8 | 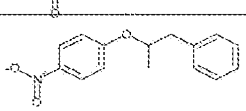 | F027 | 24h | 87.36 | 1.00 | 315.0 | 1-Nitro-4-((1-phenylpropan-2-yl)oxy)benzene |
| 11 | 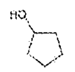 | 33.2 | 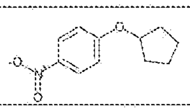 | F028 | 20h | 100 | 0.97 | 317.0 | 1-Nitro-4-(pentan-3-yloxy)benzene |
| 12 | 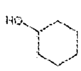 | 24.9 | 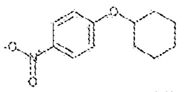 | F029 | 72h | 25.88 | 1.03 | 318.0 | 1-(Cyclohexyloxy)-4-nitrobenzene |
| 13 | 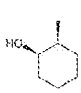 | 6.1 | 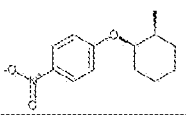 | F030 | 72h | 26.49 | 1.08 | 318.0 | Mixture of 1-(((1R,2S)-2-methylcyclohexyl)oxy)-4-nitrobenzene and 1-(((1S,2R)-2-methylcyclohexyl)oxy)-4-nitrobenzene |
| 14 | 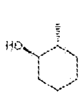 | 18.3 | 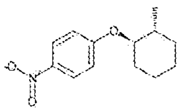 | F031 | 72h | 8.48 | 1.10 | 319.0 | Mixture of 1-(((1R,2R)-2-methylcyclohexyl)oxy)-4-nitrobenzene and 1-(((1S,2S)-2-methylcyclohexyl)oxy)-4-nitrobenzene |
| 15 | 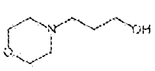 | | 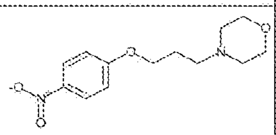 | F032 | 7h | 100 | 0.39 | 313.0 | 4-(3-(4-Nitrophenoxy) propyl)morpholine |

When the reaction substrate R-OH (alcohol) was a primary alcohol, bulkiness on carbon (carbon at the β position with respect to the hydroxy group) adjacent to carbon bonded to the hydroxy group was able to be confirmed to have large influence. When the β carbon was substituted by two carbon atoms, it was able to be confirmed that 99% or more conversion rate through reaction can be achieved over 96 hours which are a relatively long time (run 2). On the other hand, when the β carbon was substituted by three carbon atoms, the conversion rate through reaction remained at approximately 7% even though heating to 60°C was performed (run 3). Although it can be found possible to further pursue the reaction, this substrate was able to be confirmed as a typical example that requires attention in actual library synthesis.

It was confirmed that a primary alcohol can be used in reaction without particular problems as long as the primary alcohol is benzyl alcohol or phenethyl alcohol (runs 4 to 6).

Subsequently, reaction was confirmed as to a secondary alcohol serving as a substrate. In the case of using 3-pentanol, it was able to be confirmed that the reaction was completely completed in 20 hours (run 7). By contrast, for example, the scope of less bulky secondary alcohols such as isopropyl alcohol and 2-butanol than 3-pentanol is extremely likely to similarly achieve a high conversion rate through reaction.

In the case of using 2-methyl-3-pentanol as a substrate, approximately 90% conversion rate through reaction was achieved in 72 hours (run 8). 2-Methyl-3-pentanol has a thing in common with neopentyl alcohol of run 3 in that one additional carbon is bonded to isobutyl alcohol used in run 2. From the comparison between run 3 and run 8, it was able to be confirmed that quaternary carbon at one of the β positions becomes a factor that reduces a reaction rate.

In the case of using cyclic secondary alcohol as a substrate, it was confirmed that the reaction was completed in a 5-membered ring cyclopentyl alcohol, whereas the reactivity of a 6-membered ring cyclohexyl alcohol was extremely reduced (run 11 and run 12).

Even when the substrate contained an amino group, it was able to be confirmed that the reaction can be completed without particular problems (run 15).

Impurities other than reagents, generated active species, and by-products generated from the reagents were not observed in these reactions. In short, impurities generated from the substrate 4-nitrophenol or the product of interest were not observed. This indicates that even a combination of substrates that failed to achieve a sufficient conversion rate through reaction in this confirmation experiment can achieve a high conversion rate through reaction by an approach that is applied to the acceleration of general organic reaction, such as the prolongation of a reaction time, heating during reaction, or the addition of a reagent.

Alternatively, this indicates that in the presence of Ar-OH (arenol) on the solid- phase side, a high conversion rate through reaction can also be achieved by an operation of discharging the liquid phase used in reaction, replacing the reagent with a fresh one, and repeating the same reaction (also called double coupling).

In light of the results of confirming liquid-phase reaction as described above, solid- phase reaction which actually performed library synthesis was confirmed in Example 2-1-5.

Example 2-1-4: Liquid-phase experiment to confirm Mitsunobu reaction for forming R-N(Ns)-nPr bond from R-NHNs and 1-propanol

It is known that not only Ar-OH (arenol) but sulfonamide is applicable to Mitsunobu reaction as long as the nucleophile falls within the optimum acidity range. The success or failure of reaction and a reaction rate were confirmed using sulfonamide as a substrate.

Mitsunobu reaction between 2-nitro-N-phenylbenzenesulfonamide (compound F101) and 1-propanol

[Formula 254]

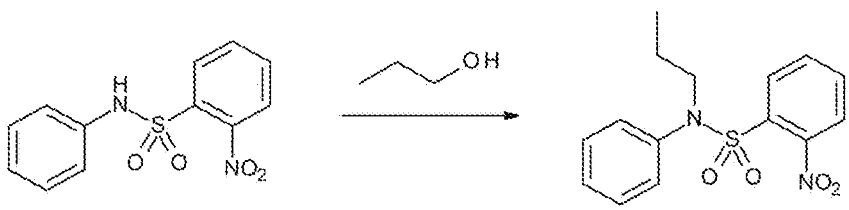

Compound F001 Compound F103

Under a nitrogen atmosphere, 2-nitro-N-phenylbenzenesulfonamide (compound F101, 20.00 mg, 0.072 mmol, 1.0 equivalent) and THF (0.72 mL, 0.10 M) were added to a 3 mL screw-cap vial. Subsequently, 1-propanol (21.6 μL, 0.287 mmol, 4.0 equivalents), TMAD (24.75 mg, 0.144 mmol, 2.0 equivalents), and tributylphosphine (35.5 μL, 0.144 mmol, 2.0 equivalents) were added thereto at room temperature, and the obtained mixture was stirred at room temperature.

Under a nitrogen atmosphere, 1 μL of the reaction mixture was sampled 30 minutes, 1 hour, 2 hours, and 5 hours after the start of reaction and dissolved in 200 μL of acetonitrile to prepare an LC sample. The reaction was monitored by LC-MS analysis. The formation of 2-nitro-N-phenyl-N-propylbenzenesulfonamide (compound F103) progressed smoothly at each point in time in reaction monitoring, and the completion of the reaction was confirmed 5 hours after its start.

[Formula 255]

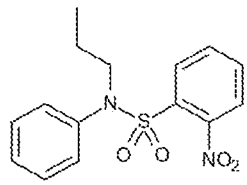

Compound F103

Compound F103

LCMS (ESI) m/z = 321.1(M+H)+.
Retention time: 0.90 min (analysis conditions SQD-FA05-1).

Mitsunobu reaction between N-benzyl-2-nitrobenzenesulfonamide (compound F102) and 1-propanol

[Formula 256]

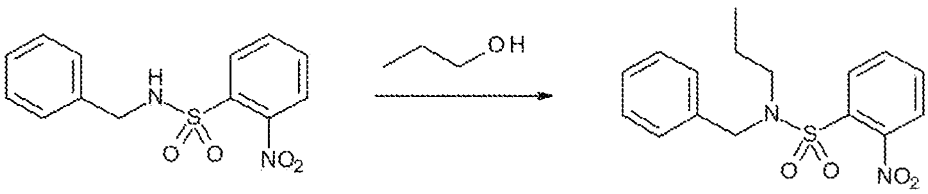

Compound F102 Compound F104

Under a nitrogen atmosphere, N-benzyl-2-nitrobenzenesulfonamide (compound F102, 21.01 mg, 0.072 mmol, 1.0 equivalent) and THF (0.72 mL, 0.10 M) were added to a 3 mL screw-cap vial. Subsequently, 1-propanol (21.6 μL, 0.287 mmol, 4.0 equivalents), TMAD (24.75 mg, 0.144 mmol, 2.0 equivalents), and tributylphosphine (35.5 μL, 0.144 mmol, 2.0 equivalents) were added thereto at room temperature, and the obtained mixture was stirred at room temperature.

Under a nitrogen atmosphere, 1 μL of the reaction mixture was sampled 30 minutes and 1 hour after the start of reaction and dissolved in 200 μL of acetonitrile to prepare an LC sample. The reaction was monitored by LC-MS analysis. The formation of 2-nitro-N- phenyl-N-propylbenzenesulfonamide (compound F104) was confirmed 30 minutes after the start of reaction, and the completion of the reaction was confirmed 1 hour after its start.

[Formula 257]

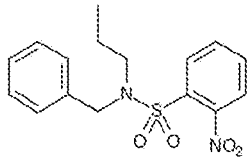

Compound F104

Compound F104

LCMS (ESI) m/z = 335.3$(M+H)^+$.

Retention time: 0.93 min (analysis conditions SQD-FA05-1).

From the results described above, it was able to be confirmed that not only Ar-OH (arenol) but sulfonamide can complete reaction as long as the substrate has the optimum pKa. Use of 2-nitro-N-phenylbenzenesulfonamide (compound F101, pKa 7.74) or N-benzyl-2- nitro-N-propylbenzenesulfonamide (compound F102, pKa 10.38) as a substrate was also able to complete reaction. In this respect, low pKa was able to be confirmed to require a longer time for the completion of reaction, as found in the results of Example 2-1-1.

Example 2-1-5: Application of conditions for solid-phase Mitsunobu reaction and confirmation experiment of scope of substrate application elucidated by liquid-phase experiment

The Mitsunobu reaction of solid-phase supported Ar-OH with alcohols AA004 to AA007 shown in Table AA-01 was carried out by the application of the reaction conditions confirmed by the liquid-phase experiments of Examples 2-1-1 to 2-1-3, to confirm the scope of building blocks applicable to library synthesis.

The operation of the reaction between AA003-03R and AA007 will be shown as an exemplary experiment.

[Formula 258]

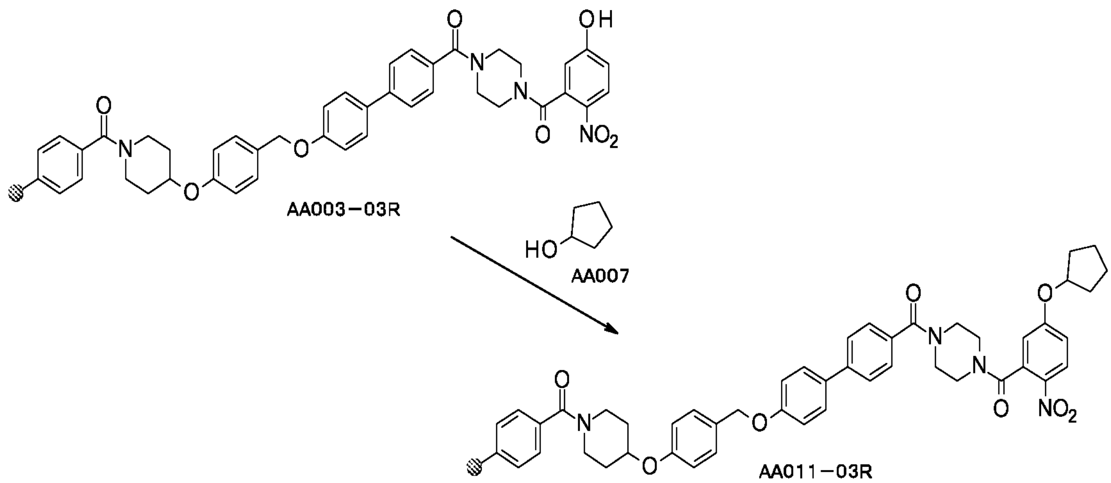

Under a nitrogen atmosphere, solid-phase supported Ar-OH (AA003-03R) (0.0494 mmol/g, 20 mg, 1.0 equivalent) and THF (300 μL, 15 v/w) were added to a 1.5 mL screw-cap vial and shaken at room temperature for 1 hour. Under a nitrogen atmosphere, secondary alcohol AA007 (7.17 μl, 80 equivalents), TMAD (6.8 mg, 40 equivalents), and Bu3P (9.8 μL, 40 equivalents) were added thereto, and the mixture was shaken at room temperature for 68 hours.

The reaction was monitored as follows.

After a lapse of a given time, under a nitrogen atmosphere, 20 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.02 M pentamethylbenzene (50 μL) for 5 minutes. After filtration, the filtrate was dissolved in NMP (250 μL), and the solution (50 μL) was diluted with MeCN (250 μL) to prepare an LC sample. The degree of progression of the reaction was measured by LC-MS analysis. The compounds shown in Table AA-02 were to be monitored as the compounds of interest (TM). As for the starting material (SM), AA003 given below was to be monitored. The compounds were analyzed under analysis conditions SMD-FA05-1, and the UV area% was confirmed at a UV wavelength of 280 nm.

Compound to be monitored as starting material (SM): [4-(5-hydroxy-2- nitrobenzoyl)piperazin-1-yl]-[4-(4-hydroxyphenyl)phenyl]methanone (AA003)

[Formula 259]

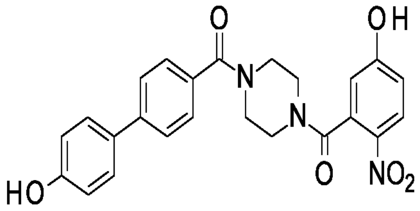

LCMS (ESI) m/z = 448.0(M+H)+.
Retention time: 0.82 min (analysis conditions SQDFA05-1).
Experiments using the other alcohols in Table AA-01 were carried out in the same manner as in the experimental operation using AA003-03R and AA007 described above. The results of LCMS after a lapse of 68 hours are shown in Table AA-01. In the case of using the alcohol AA005, AA006, or AA007, 78.3 area% or more of the target compound in an ether form was confirmed.

[Table 6]

[Table AA-01]

Table AA-01

| entry | 1 | | 2 | | 3 | | 4 | |
|---|---|---|---|---|---|---|---|---|
| Structure of alcohol | 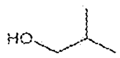 | | 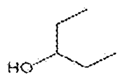 | | 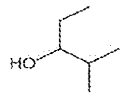 | | 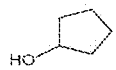 | |
| Compound No. of alcohol | AA004 | | AA005 | | AA006 | | AA007 | |
| Compound to be analyzed | TM (AA008) | SM (AA003) | TM (AA009) | SM (AA003) | TM (AA010) | SM (AA003) | TM (AA011) | SM (AA003) |
| UV area% (280 nm) | 51.3 | 32.1 | 80.9 | 3 | 78.3 | 6.3 | 83.1 | 1.6 |

From the results of this experiment, the scope of substrate application was identified on the basis of the bulkiness of a carbon atom bonded to an oxygen atom of the corresponding active phosphonium salt species formed from the alcohol (R-OH) against solid-phase supported Ar-OH.
Within the substrate scope shown here, solid-phase supported Ar-OH still remained without being converted. Therefore, the reaction has room to a higher conversion rate by the prolongation of a reaction time or the adjustment of a reaction temperature or the amount of a reagent. In other words, in one embodiment, the bulkiness of the carbon atom can be exploited as an index to determine beforehand the difficulty of library synthesis. However, it is not always true that this reaction cannot be applied even if the bulkiness is higher.

[Table 7]

[Table AA-02] Structure of target compound to be monitored

Table AA-02

| entry | Compound No. | Structure of compound of interest (TM) after cleavage | Compound name of compound of interest | MS: m/z [M+H]+ | Retention time (min) |
|---|---|---|---|---|---|
| 1 | AA008 | 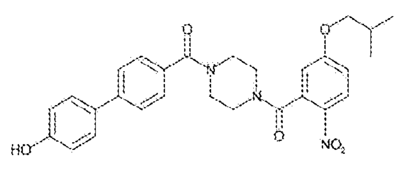 | [4-(4-Hydroxyphenyl)phenyl]-[4-[5-(2-methylpropoxy)-2-nitrobenzoyl]piperazin-1-yl]methanone | 504.1 | 1.10 |
| 2 | AA009 | 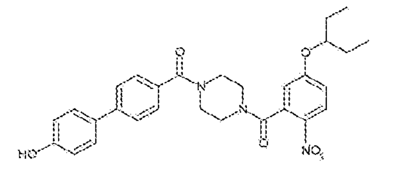 | [4-(4-Hydroxyphenyl)phenyl]-[4-(2-nitro-5-pentan-3-yloxybenzoyl)piperazin-1-yl]methanone | 518.2 | 1.14 |
| 3 | AA010 | 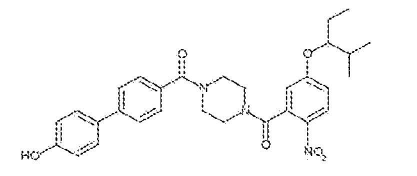 | [4-(4-Hydroxyphenyl)phenyl]-[4-[5-(2-methylpentan-3-yloxy)-2-nitrobenzoyl]piperazin-1-yl]methanone | 532.2 | 1.19 |
| 4 | AA011 | 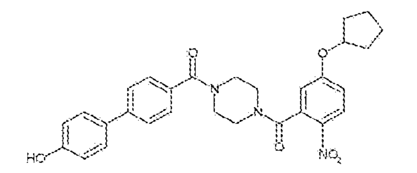 | [4-(5-Cyclopentyloxy-2-nitrobenzoyl)piperazin-1-yl]-[4-(4-hydroxyphenyl)phenyl]methanone | 516.2 | 1.10 |

Compound to be monitored as starting material (SM): [4-(5-hydroxy-2- nitrobenzoyl)piperazin-1 -yl] - [4-(4-hydroxyphenyl)phenyl]methanone (AA003)

[Formula 260]

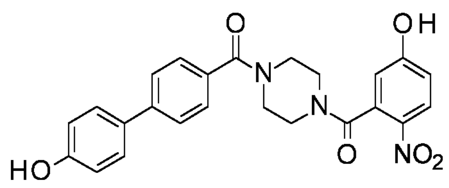

LCMS: m/z 448.1[M+H]$^+$.

Retention time: 0.82 min.

Next, an experiment was carried out to elucidate difference in reactivity depending on pKa of Ar-OH with the alcohol AA006 identified as a bulky alcohol from the results shown in Table AA-01. The Mitsunobu reaction of the solid-phase supported Ar-OH shown in Table AA-03 (AA003-03R, AA013-03R, AA015-03R, and AA017-03R) with the alcohol AA006 was carried out to confirm the scope of building blocks applicable to library synthesis.

The operation of the reaction between AA013-03R and AA006 will be shown as an exemplary experiment.

[Formula 261]

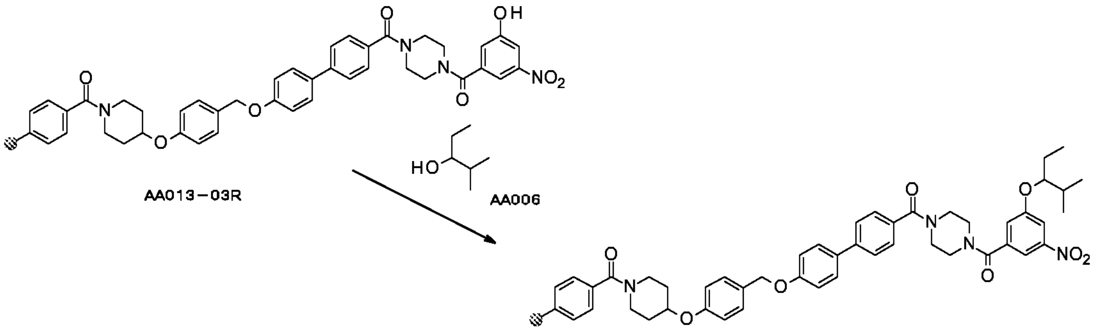

AA013-03R

AA006

AA018-03R

Under a nitrogen atmosphere, solid-phase supported Ar-OH (AA021-01R) (0.195 mmol/g, 10 mg, 1.0 equivalents) and THF (120 μL, 12 v/w) were added to a 1.5 mL screw-cap vial and shaken at room temperature for 1 hour. Under a nitrogen atmosphere, 2- methylpentan-3-ol (AA006) (7.8 μL, 32 equivalents), TMAD (5.4 mg, 16 equivalents), and Bu3P (7.7 μL, 16 equivalents) were added thereto, and the mixture was shaken at room temperature for 22 hours.

The reaction was monitored as follows.

After a lapse of a given time, under a nitrogen atmosphere, 20 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.02 M pentamethylbenzene (50 μL) for 5 minutes. After filtration, the filtrate was dissolved in NMP (250 μE), and the solution (50 μL) was diluted with MeCN (250 μL) to prepare an LC sample. The degree of progression of the reaction was measured by LC-MS analysis. The compounds shown in Table AA-04 to be monitored were analyzed under analysis conditions SMD-FA05-1, and the UV area% was confirmed at a UV wavelength of 280 nm.

Experiments using the other Ar-OH in Table AA-03 were carried out in the same manner as in the operation of the reaction between AA013-03R and AA006 described above.

The results of LCMS after a lapse of 22 hours are shown in Table AA-03. In the case of using the Ar-OH AA013-03R, AA015-03R, or AA017-03R, 75.0 area% or more of the target compound in an ether form was confirmed.

[Table 8]

[Table AA-03] Results of LCMS after lapse of 22 hours

Table AA-03

| entry | 1 | | 2 | | 3 | | 4 | |
|---|---|---|---|---|---|---|---|---|
| Ar-OH | 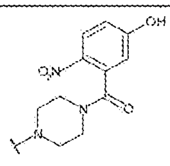 | | 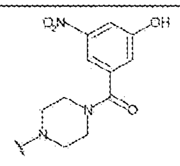 | | 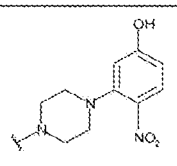 | | 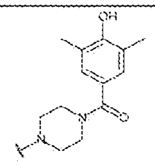 | |
| Compound No. of Ar-OH | AA003-03R | | AA013-03R | | AA015-03R | | AA017-03R | |
| cpKa of Ar-OH | 8.03 | | 8.64 | | 8.97 | | 9.16 | |
| Analysis results | TM | SM | TM | SM | TM | SM | TM | SM |
| UV area% (280 nm) | 52.8 | 42.3 | 90.8 | 0.6 | 75 | ND | 88.3 | ND |

ND = not detected

cpKa in Table AA-03 employed values calculated using ADMET predictor version 9.5.

The compounds to be monitored are shown in Table AA-04.

[Table 9]

[Table AA-04] Structure of compound to be monitored

Table AA-04

| entry | Compound No. after cleavage of starting material | Structure after cleavage of starting material (SM) | Compound name of starting material | MS:m/z [M+H]+ | Retention time (min) |
|---|---|---|---|---|---|
| 1 | AA003 | 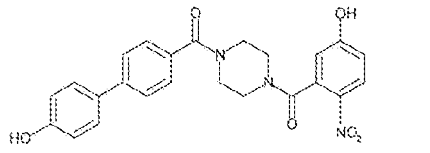 | [4-(5-Hydroxy-2-nitrobenzoyl)piperazin-1-yl]-[4-(4-hydroxyphenyl)phenyl] methanone | 448.0 | 0.82 |
| 2 | AA013 | 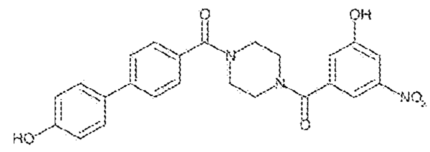 | [4-(3-Hydroxy-5-nitrobenzoyl)piperazin-1-yl]-[4-(4-hydroxyphenyl)phenyl] methanone | 448.0 | 0.85 |
| 3 | AA015 | 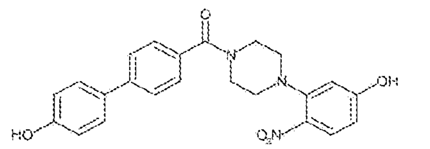 | [4-(5-Hydroxy-2-nitrophenyl)piperazin-1-yl]-[4-(4-hydroxyphenyl)phenyl] methanone | 420.0 | 0.94 |
| 4 | AA017 | 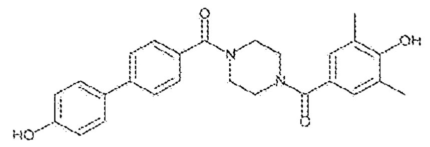 | [4-(4-Hydroxy-3,5-dimethylbenzoyl)piperazin-1-yl]-[4-(4-hydroxyphenyl)phenyl] methanone | 431.0 | 0.84 |

| entry | Compound No. after cleavage of compound of interest | Structure after cleavage of compound of interest (TM) | Compound name of compound of interest | MS:m/z [M+H]+ | Retention time (min) |
|---|---|---|---|---|---|
| 1 | AA010 | 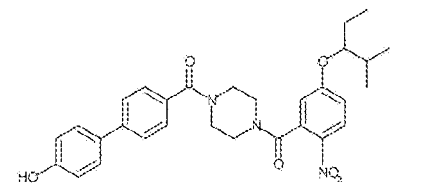 | [4-(4-Hydroxyphenyl)phenyl]-[4-[5-(2-methylpentan-3-yloxy)-2-nitrobenzoyl]piperazin-1-yl]methanone | 532.2 | 1.19 |
| 2 | AA018 | 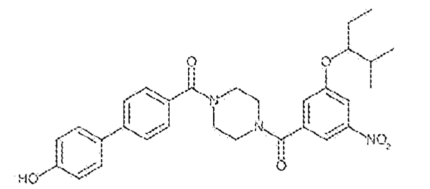 | [4-(4-Hydroxyphenyl)phenyl]-[4-[3-(2-methylpentan-3-yloxy)-5-nitrobenzoyl]piperazin-1-yl]methanone | 532.3 | 1.24 |
| 3 | AA019 | 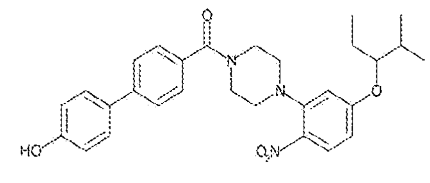 | [4-(4-Hydroxyphenyl)phenyl]-[4-[5-(2-methylpentan-3-yloxy)-2-nitrophenyl]piperazin-1-yl]methanone | 504.2 | 1.38 |
| 4 | AA020 | 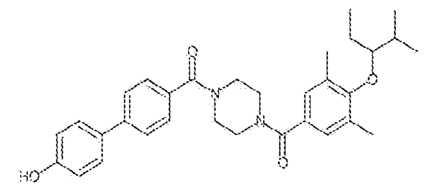 | [4-[3,5-Dimethyl-4-(2-methylpentan-3-yloxy)benzoyl]piperazin-1-yl]-[4-(4-hydroxyphenyl)phenyl] methanone | 515.3 | 1.31 |

The results of this experiment demonstrated that, when 2-methylpentan-3-ol (AA006) is used, reaction progresses at a high yield using Ar-OH that exhibits cpKa of 8.64 or more. Thus, the scope of substrate application was identified. At cpKa of 8.64 or less shown here, solid-phase supported Ar-OH still remained without being converted. Therefore, the reaction has room to a higher conversion rate by the prolongation of a reaction time or the adjustment of a reaction temperature or the amount of a reagent. In other words, in one embodiment, cpKa can be exploited as an index to determine beforehand the difficulty of library synthesis. However, it is not always true that this reaction cannot be applied even if cpKa is 8.64 or less.

The Mitsunobu reaction by the approach of the present invention was found to have a practical level applicable to library synthesis even through solid phase reaction. This Example illustrates an exemplary method for identifying an applicable scope before implementation of library synthesis. The results indicate that Ar-O-R bond formation through the Mitsunobu reaction has a practical level in performing solid-phase library synthesis. The substrate scopes of Ar-OH and alcohols may be expanded beyond the scope shown in this Example, by improvement in reaction conditions and additional substrate study, or the application of actual findings of library synthesis.

Example 2-1-6: Setting of solid-phase reaction conditions

The scopes of solid-phase supported ArOH and building block alcohols applicable to library synthesis were confirmed by the application of the reaction conditions confirmed by the solid-phase experiment of Example 2-1-5. First, Mitsunobu reaction with alcohols having various ArBr was carried out using solid-phase supported ortho-substituted Ar-OH to confirm reaction conditions.

Example 2-1-6-1: Mitsunobu reaction between solid-phase supported phenol derivative having Me group at ortho position (B103-03R) and (3-bromo-2- fluorophenyl)methanol (B311)

[Formula 262]

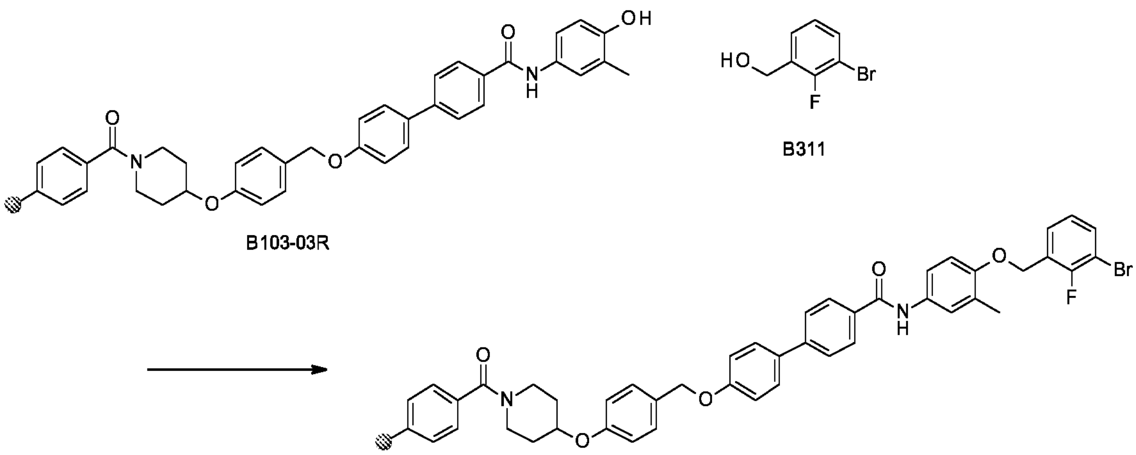

B311

B103-03R

B211-03R

Under a nitrogen atmosphere, the solid-phase supported phenol derivative (B103- 03R) (loading rate: 0.197 mmol/g, 20 mg, 0.0039 mmol) and THF (0.4 mL) were added to a 0.6 mL glass vial and shaken at room temperature for 1 hour. (3-Bromo-2- fluorophenyl)methanol (B311) (16 mg, 0.079 mmol), tri-n-butylphosphine (9.8 μL, 0.039 mmol), and TMAD (6.8 mg, 0.039 mmol) were added thereto, and the mixture was shaken at 25°C for 3 hours.

The suspension of the reaction solution and the resin were transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 95% of compound B211 of interest was observed.

[Formula 263]

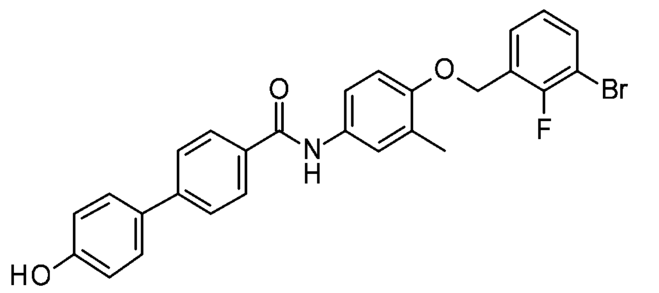

Compound B211

LRMS: m/z 506,508[M+H]+

Retention time: 1.355 min (analysis conditions SMD-FA05-1, 299 nm)

Example 2-1-6-2: Mitsunobu reaction between solid-phase supported phenol derivative substituted by F at ortho position (B104-03R) and (3-bromo-2- fluorophenyl)methanol (B311)

[Formula 264]

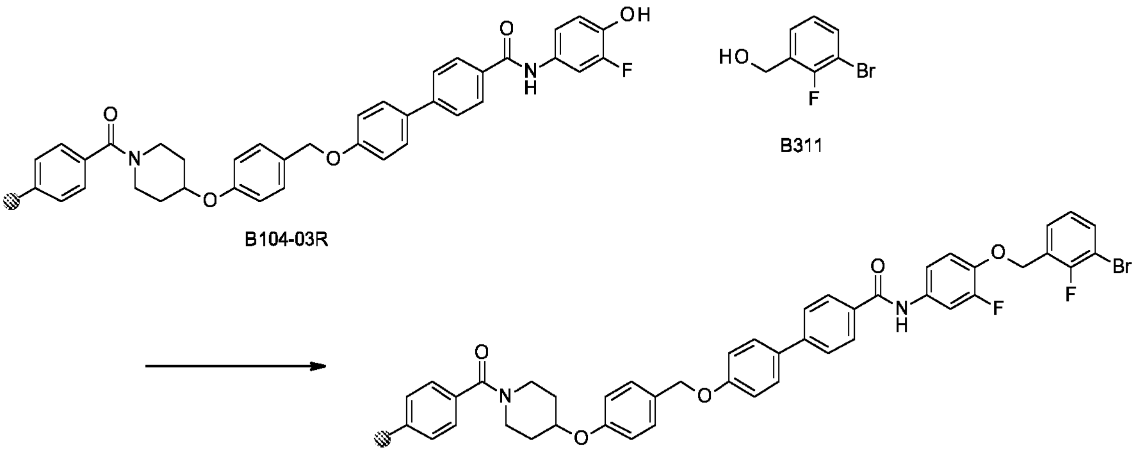

B104-03R B311 B212-03R

Under a nitrogen atmosphere, the solid-phase supported phenol derivative (B104- 03R) (loading rate: 0.197 mmol/g, 20 mg, 0.0039 mmol) and THF (0.4 mL) were added to a 0.6 mL glass vial and shaken at room temperature for 1 hour. (3-Bromo-2- fluorophenyl)methanol (B311) (16 mg, 0.079 mmol), tri-n-butylphosphine (9.8 μL, 0.039 mmol), and TMAD (6.8 mg, 0.039 mmol) were added thereto, and the mixture was shaken at 25°C for 3 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 79% of compound B212 of interest was observed.

[Formula 265]

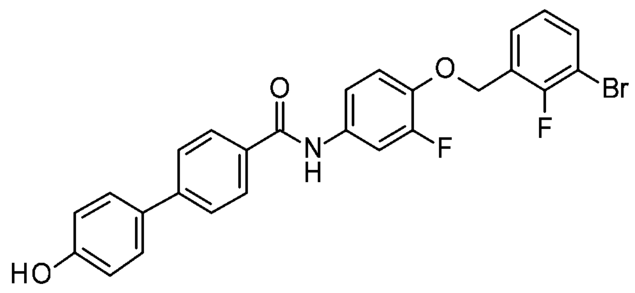

Compound B212

LRMS: m/z 510,512[M+H]+

Retention time: 1.301 min (analysis conditions SMD-FA05-1, 299 nm)

Example 2-1-6-3: Mitsunobu reaction between solid-phase supported phenol derivative having Me group at ortho position (B103-03R) and 1-(3-bromophenyl)ethan-1-ol (B312)

[Formula 266]

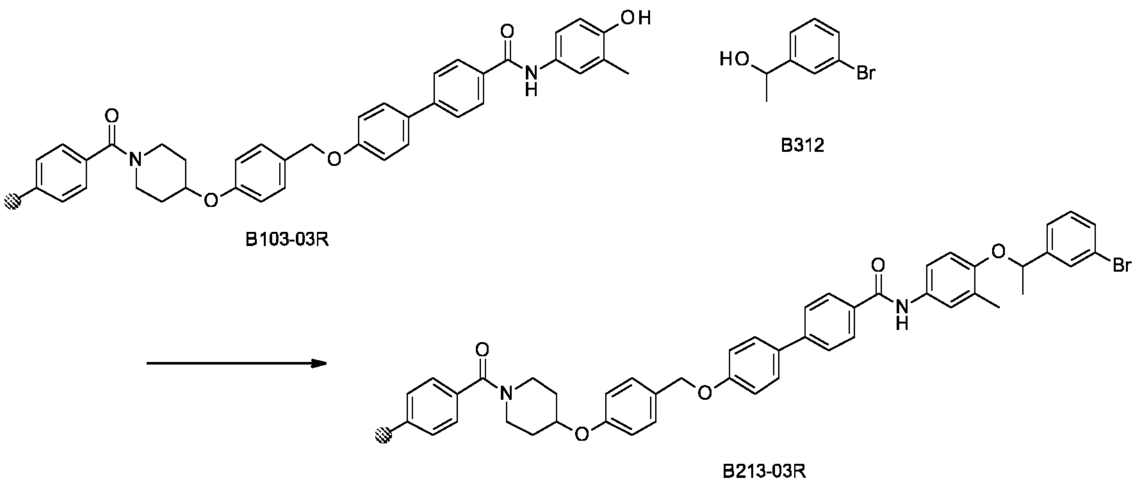

Under a nitrogen atmosphere, the solid-phase supported phenol derivative (B103- 03R) (loading rate: 0.197 mmol/g, 20 mg, 0.0039 mmol) and THF (0.4 mL) were added to a 0.6 mL glass vial and shaken at room temperature for 1 hour. 1-(3-Bromophenyl)ethan-1-ol (B312) (10.8 μL, 0.079 mmol), tri-n-butylphosphine (9.8 μL, 0.039 mmol), and TMAD (6.8 mg, 0.039 mmol) were added thereto, and the mixture was shaken at 25°C for 5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 59% of compound B213 of interest was observed.

[Formula 267]

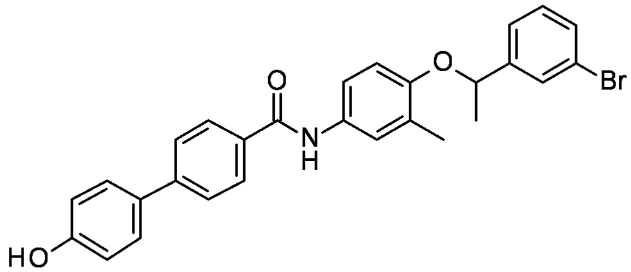

Compound B213
LRMS: m/z 502,504[M+H]+
Retention time: 1.376 min (analysis conditions SMD-FA05-1, 299 nm)

Example 2-1-6-4: Mitsunobu reaction between solid-phase supported phenol derivative having Me group at ortho position (B103-03R) and 1-(3-bromophenyl)ethan-1-ol (B312) using CMMP as Mitsunobu reagent

In Examples 2-1-6-1 to -3, nBu3P/TMAD was used as a Mitsunobu reagent, and 59% of the product ether was observed in reaction with the secondary alcohol (1-(3- bromophenyl)ethan-1-ol). For the purpose of confirming whether to be able to improve the ether production ratio when the Mitsunobu reagent was changed, Mitsunobu reaction was carried out by changing the Mitsunobu reagent to CMMP.

[Formula 268]

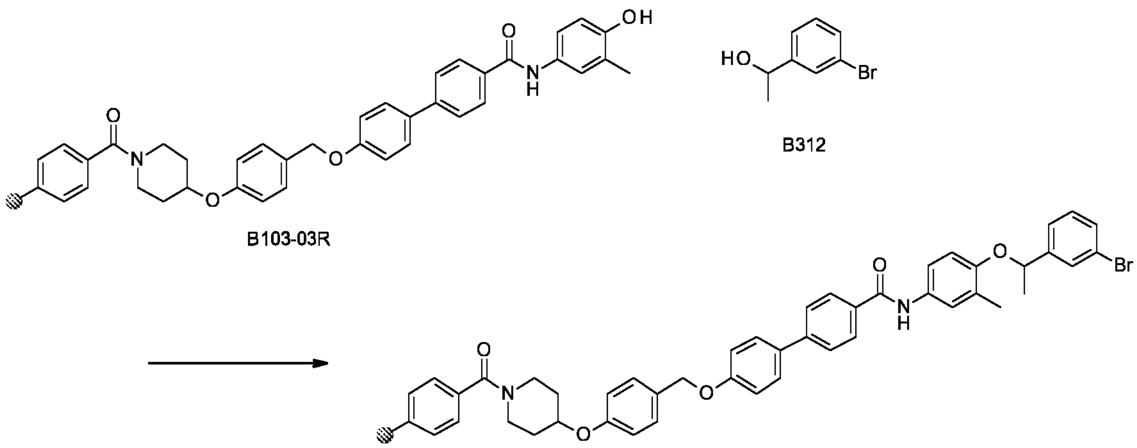


Under a nitrogen atmosphere, the solid-phase supported phenol derivative (B103- 03R) (loading rate: 0.197 mmol/g, 20 mg, 0.0039 mmol) and THF (0.4 mL) were added to a 0.6 mL glass vial and shaken at room temperature for 1 hour. 1-(3-Bromophenyl)ethan-1-ol (B312) (10.8 μL, 0.079 mmol) and CMMP (0.5 M solution in THF, 79 μL, 0.039 mmol) were added thereto, and the mixture was shaken at 60°C for 5 hours.
The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 85% of compound B213 of interest was observed.
From these results, it was confirmed that CMMP can be used as a Mitsunobu reagent in the Mitsunobu reaction between a solid-phase supported phenol derivative and an alcohol.
Example 2-1-6-5: Mitsunobu reaction between solid-phase supported phenol derivative having Me group at

ortho position (B103-03R) and (3-bromo-2- fluorophenyl)methanol (B311) using CMMP as Mitsunobu reagent

[Formula 269]

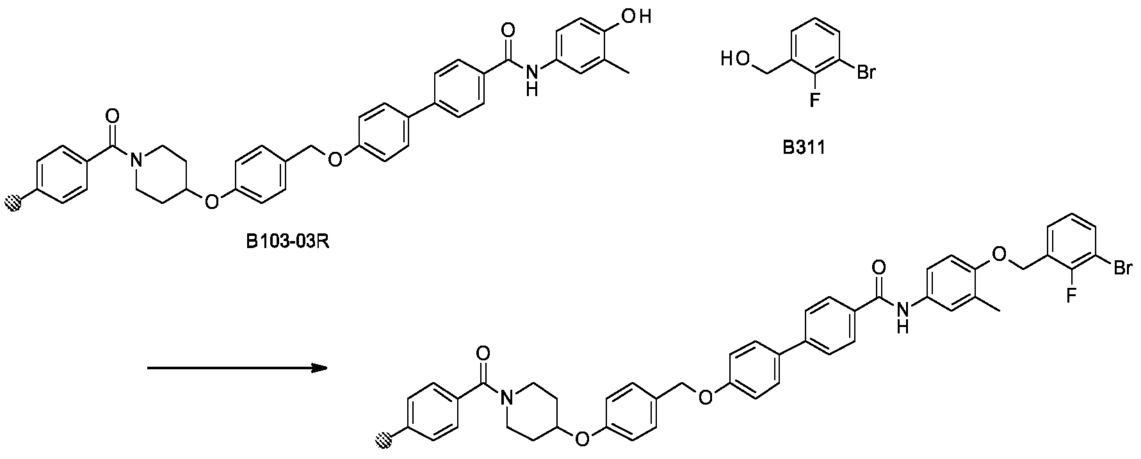

B311

B103-03R

B211-03R

Under a nitrogen atmosphere, the solid-phase supported phenol derivative (B103- 03R) (loading rate: 0.197 mmol/g, 20 mg, 0.0039 mmol) and THF (0.4 mL) were added to a 0.6 mL glass vial and shaken at room temperature for 1 hour. (3-Bromo-2- fluorophenyl)methanol (B311) (16 mg, 0.079 mmol) and CMMP (0.5 M solution in THF, 79 μL, 0.039 mmol) were added thereto, and the mixture was shaken at 60°C for 5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 97% of compound B211 of interest was observed.

Example 2-1-6-6: Mitsunobu reaction between solid-phase supported phenol derivative having Me group at ortho position (B103-03R) and (3-bromo-5- methylphenyl)methanol (B313) using CMMP as Mitsunobu reagent

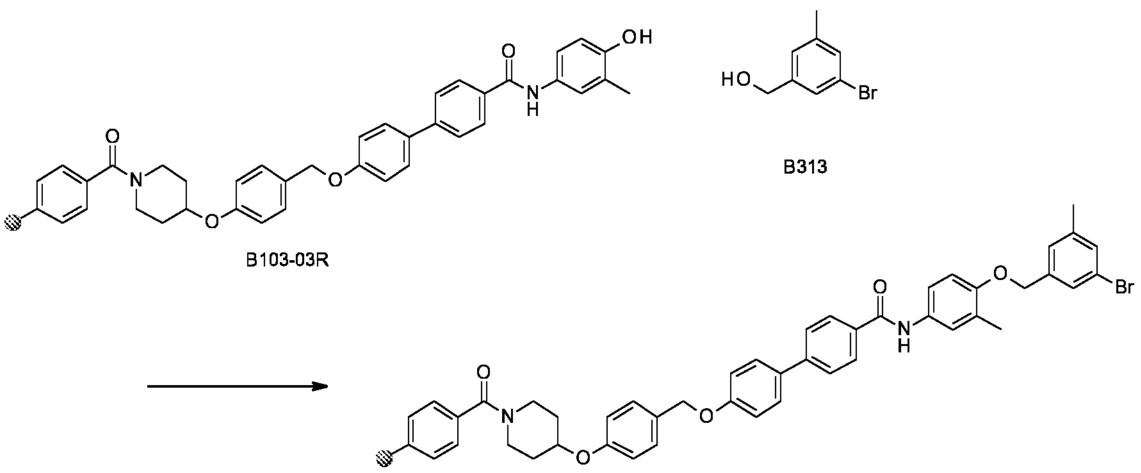

B313

B103-03R

B214-03R

Under a nitrogen atmosphere, the solid-phase supported phenol derivative (B103- 03R) (loading rate: 0.197 mmol/g, 20 mg, 0.0039 mmol) and THF (0.4 mL) were added to a 0.6 mL glass vial and shaken at room temperature for 1 hour. (3-Bromo-5- methylphenyl)methanol (B313) (16 mg, 0.079 mmol) and CMMP (0.5 M solution in THF, 79 μL,

0.039 mmol) were added thereto, and the mixture was shaken at 60°C for 5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 97% of compound B214 of interest was observed.

[Formula 270]

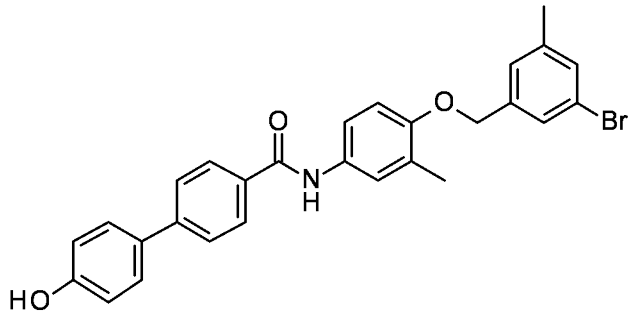

Compound B214

LRMS: m/z 502,504[M+H]$^+$

Retention time: 1.419 min (analysis conditions SMD-FA05-1, 299 nm)

Example 2-1-6-7: Mitsunobu reaction between solid-phase supported phenol derivative having Me group at ortho position (B103-03R) and 3-(3-bromophenyl)propanol (B314) using CMMP as Mitsunobu reagent

[Formula 271]

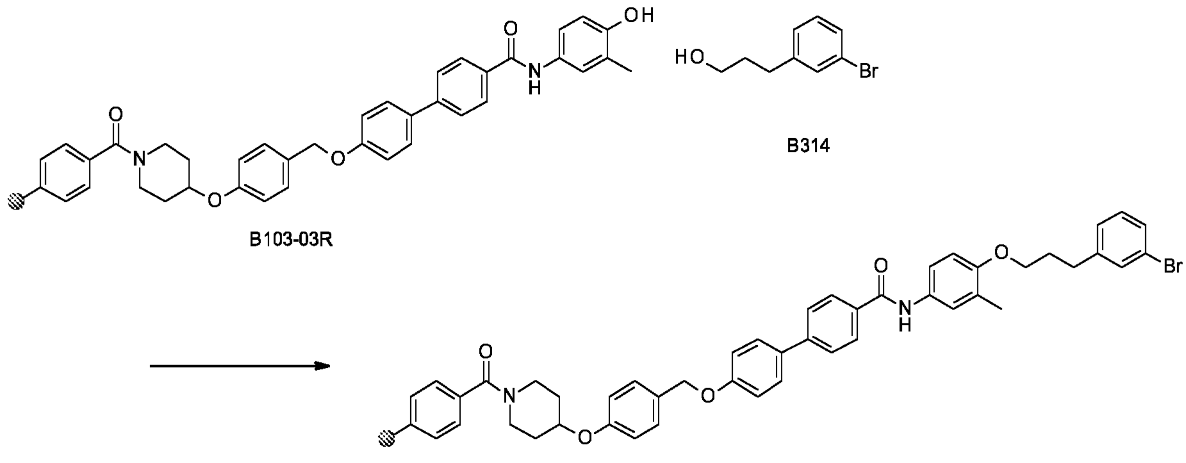

B103-03R

B314

B215-03R

Under a nitrogen atmosphere, the solid-phase supported phenol derivative (B103- 03R) (loading rate: 0.197 mmol/g, 20 mg, 0.0039 mmol) and THF (0.4 mL) were added to a 0.6 mL glass vial and shaken at room temperature for 1 hour. 3-(3-Bromophenyl)propanol (B314) (12 μL, 0.079 mmol) and CMMP (0.5 M solution in THF, 79 μL, 0.039 mmol) were added thereto, and the mixture was shaken at 60°C for 2 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 98% of compound B215 of interest was

observed.

[Formula 272]

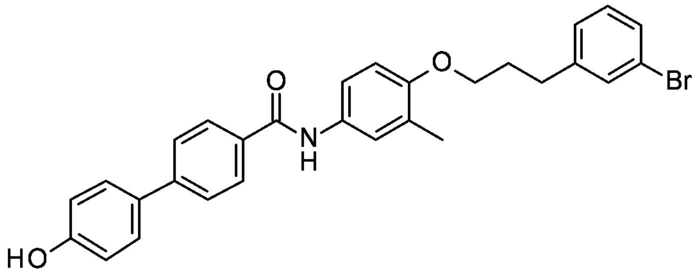

Compound B215

LRMS: m/z 516,518[M+H]+

Retention time: 1.449 min (analysis conditions SMD-FA05-1, 299 nm)

Example 2-1-6-8: Mitsunobu reaction between solid-phase supported phenol derivative substituted by F at ortho position (B104-03R) and (3-bromo-2- fluorophenyl)methanol (B311) using CMMP as Mitsunobu reagent

[Formula 273]

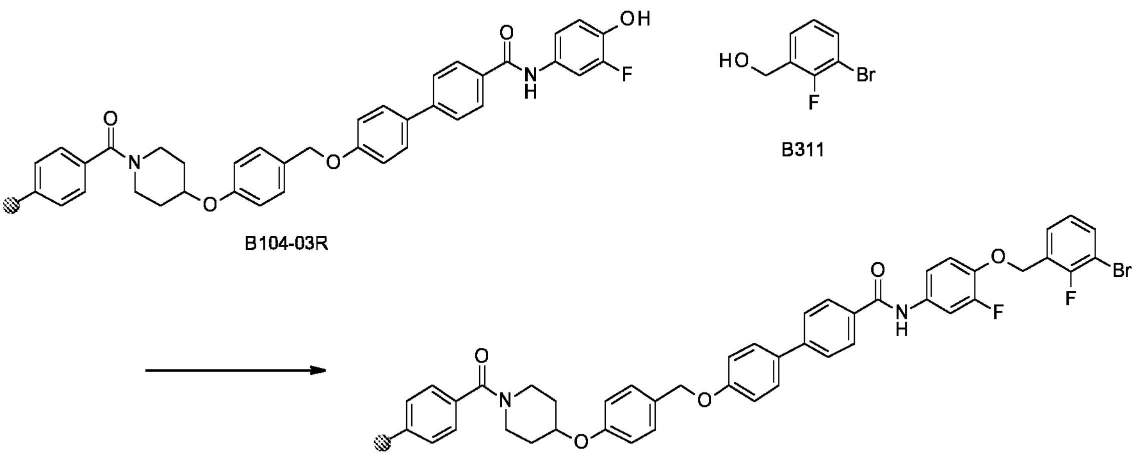

B104-03R

B311

B212-03R

Under a nitrogen atmosphere, the solid-phase supported phenol derivative (B104- 03R) (loading rate: 0.197 mmol/g, 20 mg, 0.0039 mmol) and THF (0.4 mL) were added to a 0.6 mL glass vial and shaken at room temperature for 1 hour. (3-Bromo-2- fluorophenyl)methanol (B311) (16 mg, 0.079 mmol) and CMMP (0.5 M solution in THF, 79 μL, 0.039 mmol) were added thereto, and the mixture was shaken at 60°C for 5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 91% of compound B212 of interest was observed.

Example 2-1-6-9: Mitsunobu reaction between solid-phase supported phenol derivative substituted by F at ortho position (B104-03R) and 2-(3-bromophenyl)ethanol (B315) using CMMP as Mitsunobu reagent

[Formula 274]

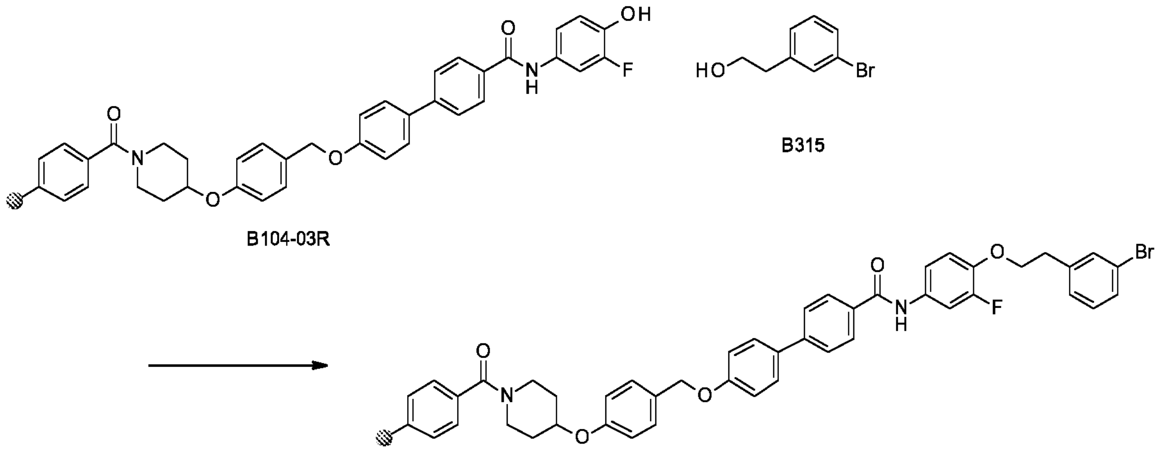

B104-03R

B315

B216-03R

Under a nitrogen atmosphere, the solid-phase supported phenol derivative (B104- 03R) (loading rate: 0.197 mmol/g, 20 mg, 0.0039 mmol) and THF (0.4 mL) were added to a 0.6 mL glass vial and shaken at room temperature for 1 hour. 2-(3-Bromophenyl)ethanol (B315) (10.7 μL, 0.079 mmol) and CMMP (0.5 M solution in THF, 79 μL, 0.039 mmol) were added thereto, and the mixture was shaken at 60°C for 5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 94% of compound B216 of interest was observed.

[Formula 275]

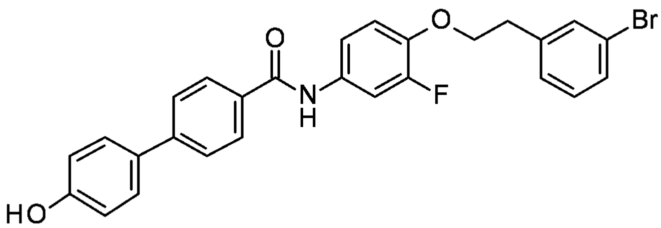

Compound B216

LRMS: m/z 506,508$[M+H]^+$

Retention time: 1.349 min (analysis conditions SMD-FA05-1, 299 nm)

Example 2-1-6-10: Mitsunobu reaction between solid-phase supported phenol derivative substituted by F at ortho position (B104-03R) and (3-bromo-5- methylphenyl)methanol (B313) using CMMP as Mitsunobu reagent

[Formula 276]

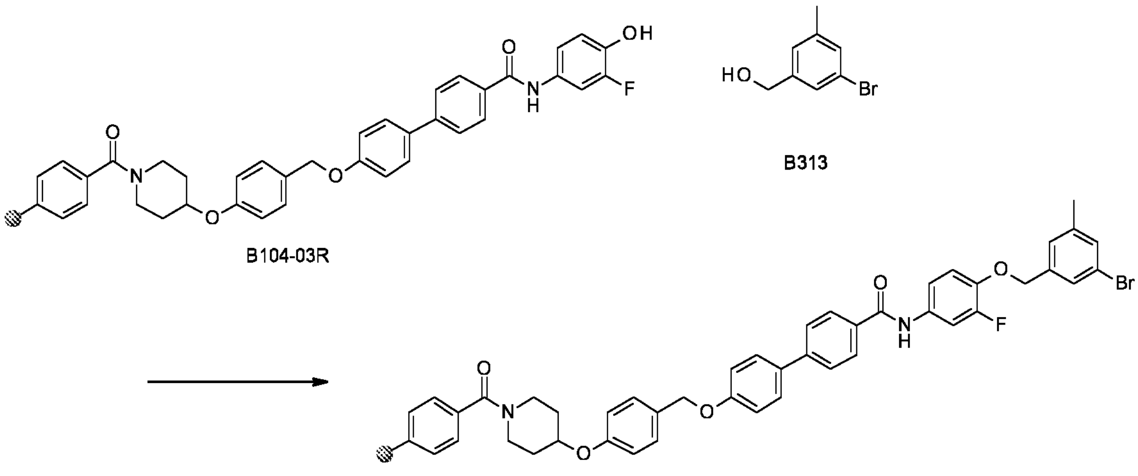

B313

B104-03R

B217-03R

Under a nitrogen atmosphere, the solid-phase supported phenol derivative (B104- 03R) (loading rate: 0.197 mmol/g, 20 mg, 0.0039 mmol) and THF (0.4 mL) were added to a 0.6 mL glass vial and shaken at room temperature for 1 hour. (3-Bromo-5- methylphenyl)methanol (B313) (16 mg, 0.079 mmol) and CMMP (0.5 M solution in THF, 79 μL, 0.039 mmol) were added thereto, and the mixture was shaken at 60°C for 5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 89% of compound B217 of interest was observed.

[Formula 277]

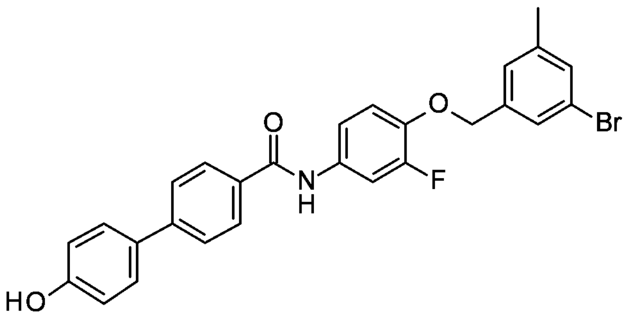

Compound B217

LRMS: m/z 506,508$[M+H]^+$

Retention time: 1.381 min (analysis conditions SMD-FA05-1, 299 nm)

Example 2-1-6-11: Mitsunobu reaction between solid-phase supported phenol derivative (B105-03R) and (3-bromo-2-fluorophenyl)methanol (B311) at room temperature using CMMP as Mitsunobu reagent

For the purpose of finding milder reaction conditions toward library synthesis, Mitsunobu reaction was performed with a solid-phase supported phenol derivative (B105- 03R) as a substrate by lowering the reaction temperature to room temperature (25°C), to confirm the reaction.

[Formula 278]

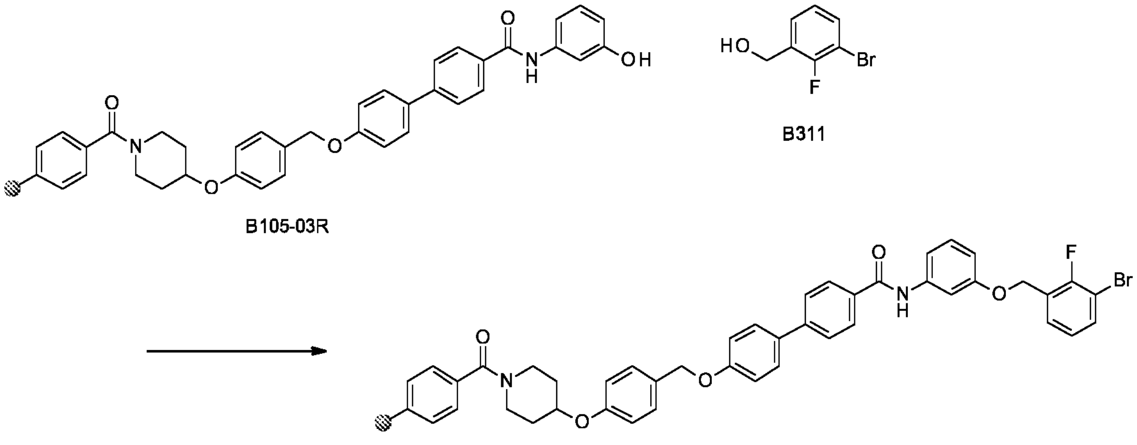

B311

B105-03R

B218-03R

Under a nitrogen atmosphere, the solid-phase supported phenol derivative (B105- 03R) (loading rate: 0.197 mmol/g, 20 mg, 0.0039 mmol) and THF (0.4 mL) were added to a 0.6 mL glass vial and shaken at room temperature for 1 hour. (3-Bromo-2- fluorophenyl)methanol (B311) (16 mg, 0.079 mmol) and CMMP (0.5 M solution in THF, 79 μL, 0.039 mmol) were added thereto, and the mixture was shaken at 25°C for 15 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 97% of compound B218 of interest was observed.

[Formula 279]

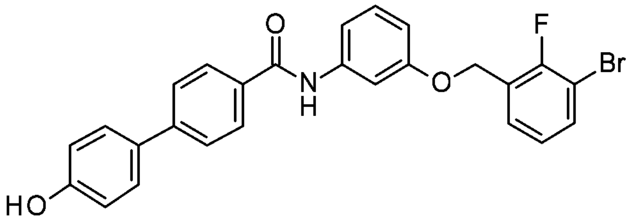

Compound B218

LRMS: m/z 492,494[M+H]$^+$

Retention time: 1.323 min (analysis conditions SMD-FA05-1, 299 nm)

Example 2-1-6-12: Mitsunobu reaction between solid-phase supported phenol derivative (B105-03R) and (3-bromo-5-methylphenyl)methanol (B313) at room temperature using CMMP as Mitsunobu reagent

[Formula 280]

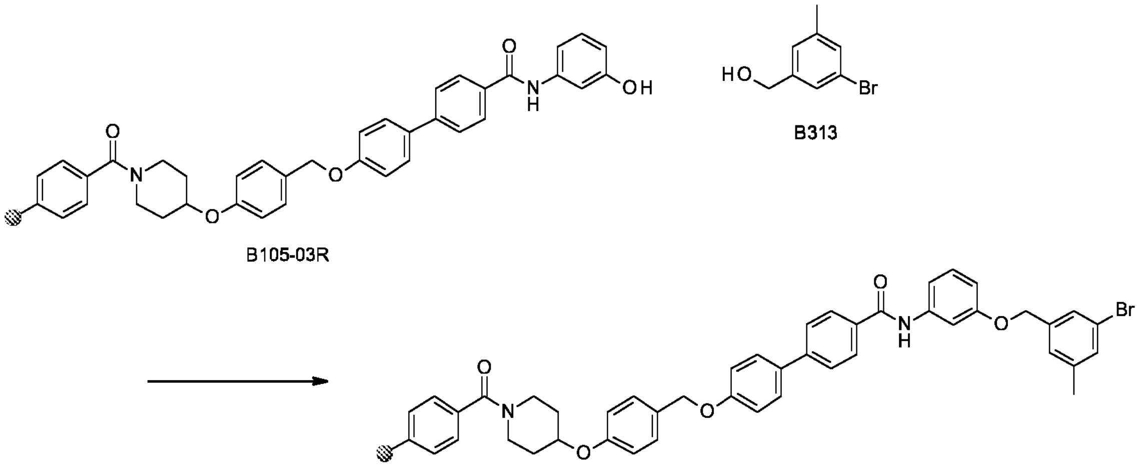

B313

B105-03R

B219-03R

Under a nitrogen atmosphere, the solid-phase supported phenol derivative (B105- 03R) (loading rate: 0.197 mmol/g, 20 mg, 0.0039 mmol) and THF (0.4 mL) were added to a 0.6 mL glass vial and shaken at room temperature for 1 hour. (3-Bromo-5- methylphenyl)methanol (B313) (16 mg, 0.079 mmol) and CMMP (0.5 M solution in THF, 79 μL, 0.039 mmol) were added thereto, and the mixture was shaken at 25°C for 15 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 96% of compound B219 of interest was observed.

[Formula 281]

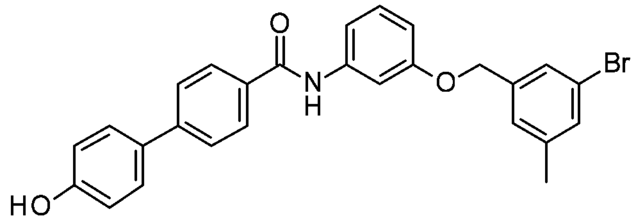

Compound B219

LRMS: m/z 488,490$[M+H]^+$

Retention time: 1.385 min (analysis conditions SMD-FA05-1, 299 nm)

Example 2-1-6-13: Mitsunobu reaction between solid-phase supported phenol derivative (B105-03R) and 2-(3-bromophenyl)ethanol (B315) at room temperature using CMMP as Mitsunobu reagent

[Formula 282]

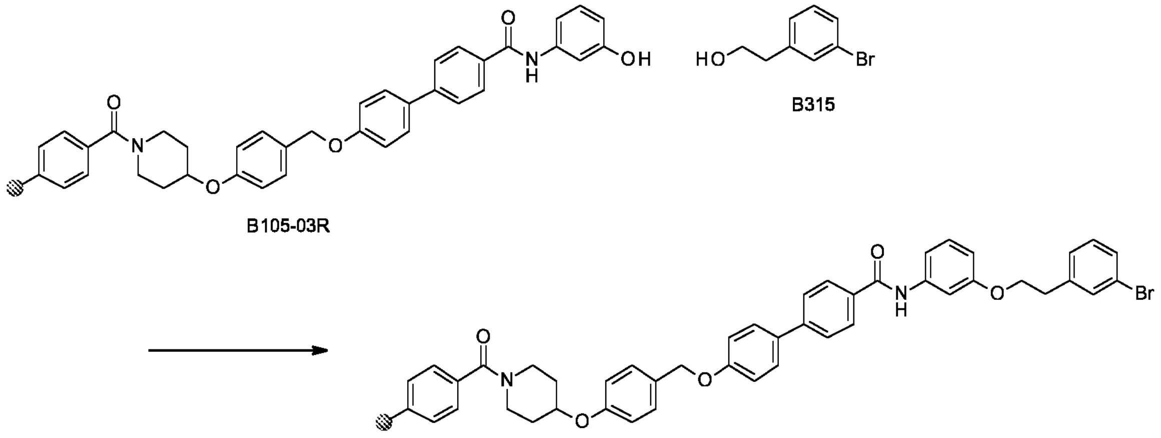

B315

B105-03R

B220-03R

Under a nitrogen atmosphere, the solid-phase supported phenol derivative (B105- 03R) (loading rate: 0.197 mmol/g, 20 mg, 0.0039 mmol) and THF (0.4 mL) were added to a 0.6 mL glass vial and shaken at room temperature for 1 hour. 2-(3-Bromophenyl)ethanol (B315) (10.7 μL, 0.079 mmol) and CMMP (0.5 M solution in THF, 79 μL, 0.039 mmol) were added thereto, and the mixture was shaken at 25°C for 15 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 96% of compound B220 of interest was observed.

[Formula 283]

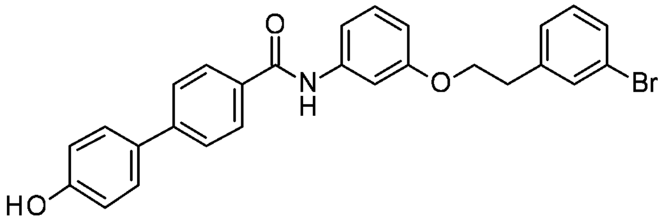

Compound B220

LRMS: m/z 488,490$[M+H]^+$

Retention time: 1.355 min (analysis conditions SMD-FA05-1, 299 nm)

From these results, it was confirmed that the Mitsunobu reaction of solid-phase supported ArOH with an alcohol, for example, substituted benzyl alcohol or substituted phenethyl alcohol, may be performed with CMMP as a Mitsunobu reagent at room temperature (25°C) which is more preferred mild condition for library synthesis in which a large number of unspecified compounds are treated.

Example 2-2: Experiment to confirm scope of substrate application to Suzuki coupling reaction as typical example of identifying substrate scope that can be applied to library synthesis

Hereinafter, exemplary conditions for enhancing reproducibility by selecting suitable reaction conditions for Suzuki coupling will be illustrated. The scope given below is a mere illustration of an exemplary identification method, and the present invention is not limited by the scope given below in actual application to library synthesis.

Identification of challenge to solid-phase library synthesis through Suzuki coupling reaction, and presentation of solution thereto

Example 2-2-0: Extraction of challenge of Suzuki coupling reaction to solid-phase library synthesis

[Formula 284]

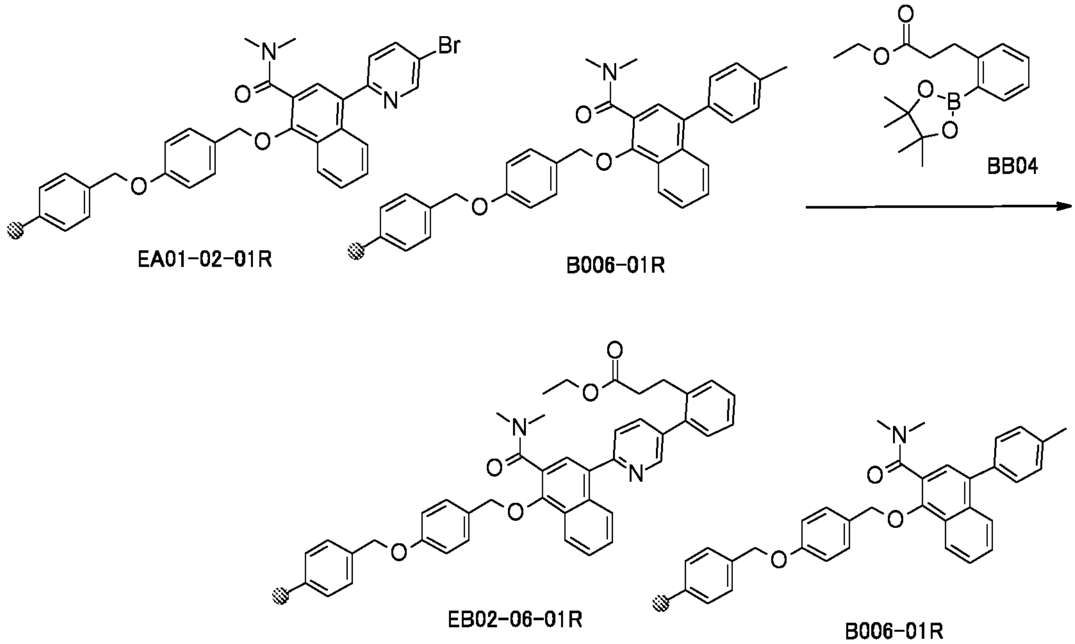

EA01-02-01R B006-01R BB04

EB02-06-01R B006-01R

Compound EA01-02-01R (20 mg, loading rate: 0.35 mmol/g, 10.4 μmol), compound B006-01R (added as an internal standard reagent, 5.36 mg, loading rate: 0.21 mmol/g, 1 μmol, 0.1 equivalents), and NMP (367 μL) were added to a 0.6 mL glass vial. The glass vial containing the reaction solution was purged with nitrogen by the blowing of nitrogen for approximately 10 seconds, and then shaken at room temperature for 1 hour. Ethyl 3-[2-(4,4,5,5-tetramethyl-1,3,2-dioxaborolan-2-yl)phenyl]propanoate (BB04) (9.49 mg, 31 μmol), an aqueous $K_3PO_4$ solution (1.5 M, 42 μL, 62 μmol), and a solution of $P(Cy)_3$ Pd G3 in NMP (0.02 M, 52 μL, 1.04 μmol) were added thereto, and the glass vial containing the reaction solution was purged with nitrogen by the blowing of nitrogen for approximately 10 seconds, and then shaken at 90°C for 3 hours.

The reaction was monitored as follows. Sampling was performed after a lapse of 3 hours from the start of shaking. For the purpose of confirming the conversion rate through reaction on the solid phase with EB02-06 to be detected, under a nitrogen atmosphere, 10 μL of the suspension of the reaction solution and the resin was sampled so as to inevitably involve the resin, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (100 μL) for 5 minutes. After filtration, the filtrate was dissolved in TFE (100 μL) to prepare an LC sample. The degree of progression of the reaction was measured by LC-MS analysis. The UV area% was confirmed at a UV wavelength of 319 nm.

For the purpose of confirming a remaining reagent on the liquid-phase side with BB04 to be detected, under a nitrogen atmosphere, the filtrate washed three times with NMP (100 μL) by the operation described above was filtered through a vial with a filter to prepare an LC sample. The consumption rate of the reagent was measured by calculating the peak area and concentration of a BB04 standard standard solution, followed by comparison. The UV area% was confirmed by PDA (210-400 nm).

The results are as shown in run BB04-1 of Table ER1-1A.

[Table 10]

Table ER1-1A

| Table [ER1-1A] | | | | SM and TM in cleavage solution (area ratio at 319 nm) | | BB in supernatant (area ratio of boronic acid and boronic acid ester to separately prepared standard sample) (area ratio in PDA) |
|---|---|---|---|---|---|---|
| BB | | | | TM | SM | BB |
| BB04 | Resin starting material | run | hr | EB02-06 | EA01-02 | BB04 |
| 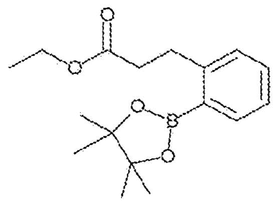 | EA01-02-01R | BB04-1 | 3 | 90.1 | 9.5 | 0 |

The starting material remained at 3 hours after the start of reaction, and the reaction was not completed. The reagent BB04 and its boronic acid ((2-(3-ethoxy-3- oxopropyl)phenyl)boronic acid) were degraded and consumed due to protodeborylation, oxidation reaction, or the like. A challenge to solid-phase reaction identified from the results described above was to set conditions under which reaction was completed in a state where reagents such as boronic acid and a catalyst remained sufficiently. This challenge is not limited to Suzuki coupling, and it is generally essential that sufficient amounts of reagents remain in the liquid phase, for reproducibly completing solid-phase reaction.

[Formula 285]

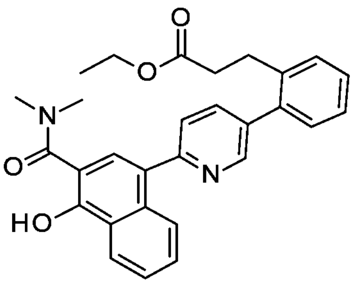

EB02-06

Ethyl 3-[2-[6-[3-(dimethylcarbamoyl)-4-hydroxynaphthalen-1-yl]pyridin-3- yl]phenyl]propanoate (EB02-06)

LCMS: m/z 468[M+H]+.

Retention time: 1.53 min (analysis conditions SMD-FA05-1).

Example 2-2-1: Presentation regarding solution for challenge identified in Example 2-2-0

An experiment that was able to solve the challenge identified in Example 2-2-0 will be shown below.

[Formula 286]

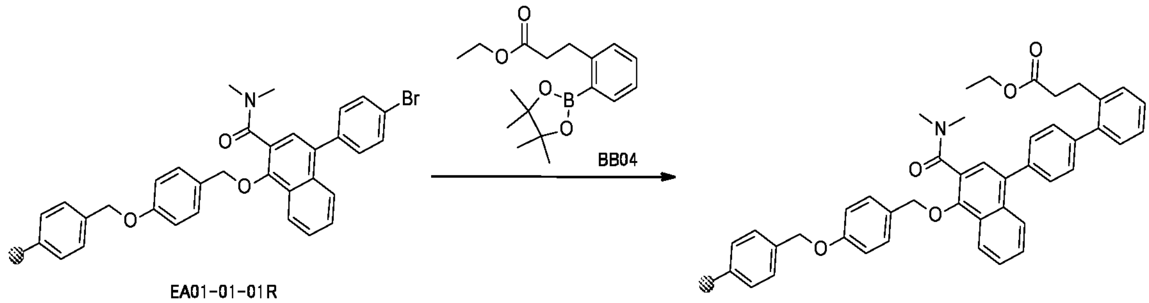


EA01-01-01R

EB02-05-01R

Under a nitrogen atmosphere, compound EA01-01-01R (20 mg, loading rate: 0.4 mmol/g, 8 μmol) and NMP (280 μL) were added to a 0.6 mL glass vial and shaken at room temperature for 1 hour. Under a nitrogen atmosphere, ethyl 3-[2-(4,4,5,5-tetramethyl- 1,3,2-dioxaborolan-2-yl)phenyl]propanoate (BB04) (14.6 mg, 48 μmol), an aqueous $K_3PO_4$ solution (1.5 M, 32 μL, 48 μmol), and a solution of $P(Cy)_3$ Pd G3 in NMP (0.04 M, 40 μL, 1.6 μmol) were added thereto, and the mixture was shaken at 90°C for 27 hours.

The reaction was monitored as follows.

Sampling was performed after a lapse of 1, 2, and 27 hours from the start of shaking. For the purpose of confirming the conversion rate through reaction on the solid phase with EB02-05 to be detected, under a nitrogen atmosphere, 10 μL of the suspension of the reaction solution and the resin was sampled so as to inevitably involve the resin, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in DMI (250 μL), and the solution (50 μL) was diluted with MeCN (200 μL) to prepare an LC sample. The degree of progression of the reaction was measured by LC-MS analysis. The UV area% was confirmed at a UV wavelength of 319 nm.

For the purpose of confirming a remaining reagent on the liquid-phase side with BB04 to be detected, under a nitrogen atmosphere, 5 μL of only the reaction solution of the supernatant was sampled, then diluted with MeCN (1 mL), and filtered through a vial with a filter to prepare an LC sample. BB04 (14.2 mg, 46.7 μmol) was completely dissolved in MeCN (249 μL), and the solution (5 μL) was diluted with MeCN (1150 μL) to prepare an LC sample as a standard solution of boronic acid. LC-MS analysis was performed, and the consumption rate of the reagent was measured by calculating the peak area and concentration of BB04, followed by comparison with BB04 at the start of reaction defined as 100%. The UV area% was confirmed by PDA (210-400 nm).

The 0.04 M solution of $P(Cy)_3$ Pd G3 in NMP was prepared as follows.

Under a nitrogen atmosphere, 3 mL of NMP was added to $P(Cy)_3$ Pd G3 (78 mg, 60 μmol) in a 4 mL glass vial and dissolved by shaking to prepare the solution.

The results are as shown in run BB04-2, -3, and -4 of Table ER1-1B. As shown in run BB04-3, conditions were found under which reaction was completed while the starting material was consumed in a state where 78% of the reagent BB04 remained.

[Table 11]

Table ER1-1B

| Table [ER1-1B] | | | | SM and TM in cleavage solution (area ratio at 319 nm) | | BB in supernatant (area ratio of boronic acid and boronic acid ester to separately prepared standard sample) (area ratio in PDA) |
|---|---|---|---|---|---|---|
| BB | | | | TM | SM | BB |
| BB04 | Resin starting material | run | hr | EB02-05 | EA01-02 | BB04 |
| 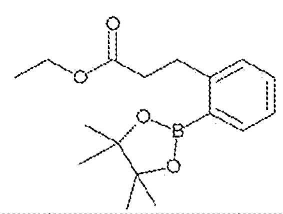 | EA01-01-01R | BB04-2 | 1 | 97.5 | 0.4 | 76 |
| | | BB04-3 | 2 | 97.4 | 0.0 | 78 |
| | | BB04-4 | 27 | 92.1 | 0.0 | 50 |

[Formula 287]

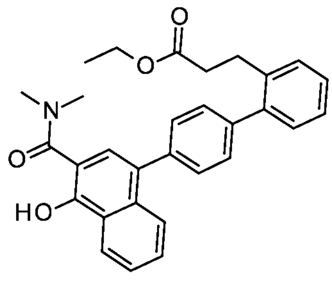

EB02-05

Ethyl 3-[2-[4-[3-(dimethylcarbamoyl)-4-hydroxynaphthalen-1- yl]phenyl]phenyl]propanoate (EB02-05)

LCMS: m/z 468[M+H]+.

Retention time: 1.53 min (analysis conditions SMD-FA05-1).

Example 2-2-2: Synthesis of EB02-06-01R

An experiment will be described below in which, in the case of using compound EA01-02-01R as a starting material, Suzuki coupling was also completed under the same conditions as in the case of using EB02-05-01R as a starting material by monitoring the rate of progression of reaction.

[Formula 288]

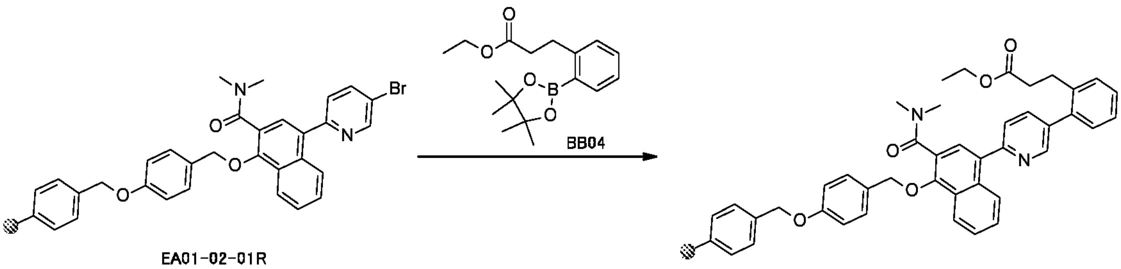


EA01-02-01R

EB02-06-01R

Under a nitrogen atmosphere, compound EA01-02-01R (20 mg, loading rate: 0.37 mmol/g, 7.4 μmol) and NMP (1.33 mL) were added to a 0.6 mL glass vial and shaken at room temperature for 1 hour. Under a nitrogen atmosphere, ethyl 3-[2-(4,4,5,5-tetramethyl- 1,3,2-dioxaborolan-2-yl)phenyl]propanoate (BB04) (13.5 mg, 44 μmol), an aqueous $K_3PO_4$ solution (1.5 M, 30 μL, 44 μmol), and a solution of $P(Cy)_3$ Pd G3 in NMP (0.04 M, 37 μL, 1.48 μmol) were added thereto, and the mixture was shaken at 90°C for 27 hours.

The reaction was monitored as follows.

Sampling was performed after a lapse of 1, 2, and 27 hours from the start of shaking.

For the purpose of confirming the conversion rate through reaction on the solid phase with EB02-06 to be detected, under a nitrogen atmosphere, 10 μL of the suspension of the reaction solution and the resin was sampled so as to inevitably involve the resin, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in TFE (200 μL), and the solution was diluted by the addition of TFE (800 μL) to prepare an LC sample. The degree of progression of the reaction was measured by LC-MS analysis. The UV area% was confirmed at a UV wavelength of 319 nm.

By using BB04 to be detected, the filtrate washed three times with NMP (100 μL) by the operation described above was filtered through a vial with a filter to prepare an LC sample. The consumption rate of the reagent was measured by calculating the peak area and concentration of a BB04 standard solution, followed by comparison. The UV area% was confirmed by PDA (210-400 nm).

The results are as shown in run BB04-5, -6, and -7 of Table ER1-1C. As shown in run BB04-6, in the case of using EA01-02-01R as a starting material resin, reaction also progressed at a rate substantially equivalent to that in the case of using EA01-01-01R as a starting material. Thus, conditions were found under which reaction was completed while the starting material was consumed in a state where 42% of the reagent BB04 remained. In a mixture library in which reaction is carried out by mixing a plurality of starting material resins, it is very important for constructing a mixture library having a high purity to confirm the conversion rate through reaction of each starting material, because the conversion rate through reaction differs among the starting materials (here, the resins).

[Table 12]

Table ER1-1C

| Table [ER1-1C] | | | | SM and TM in cleavage solution (area ratio at 319 nm) | | BB in supernatant (area ratio of boronic acid and boronic acid ester to separately prepared standard sample) (area ratio in PDA) |
|---|---|---|---|---|---|---|
| BB | | | | TM | SM | BB |
| BB04 | Resin starting material | run | hr | EB02-06 | EA01-02 | BB04 |
| 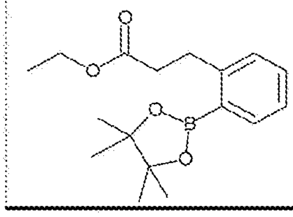 | EA01-02-01R | BB04-5 | 1 | 96.6 | 0.2 | 101 |
| | | BB04-6 | 2 | 95.8 | 0.0 | 42 |
| | | BB04-7 | 14 | 94.0 | 0.0 | 47 |

Example 2-2-3: Synthesis of EB06-02-01R

[Formula 289]

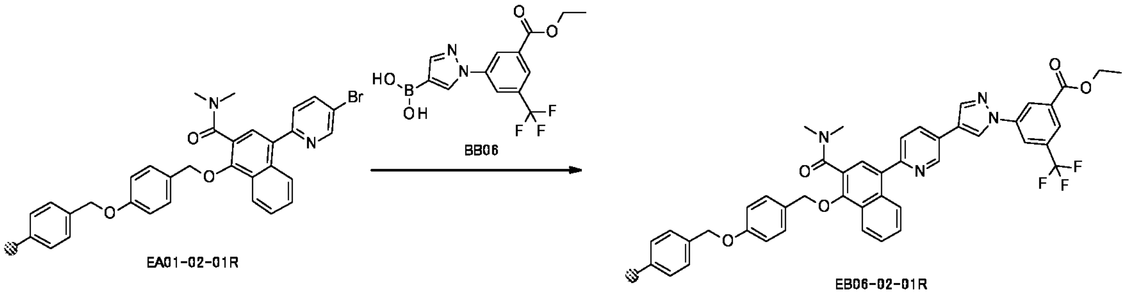


EA01-02-01R

EB06-02-01R

Under a nitrogen atmosphere, compound EA01-02-01R (20 mg, loading rate: 0.37 mmol/g, 7.4 μmol) and NMP (250 μL) were added to a 0.6 mL glass vial and shaken at room temperature for 1 hour. Under a nitrogen atmosphere, {1-[3-ethoxycarbonyl-5- (trifluoromethyl)phenyl]pyrazol-4-yl}boronic acid (BB06) (29.1 mg, 89 μmol), an aqueous $K_3PO_4$ solution (1.5 M, 30 μL, 44 μmol), and a solution of $P(Cy)_3$ Pd G3 in NMP (0.04 M, 37 μL, 1.48 μmol) were added thereto,

and the mixture was shaken at 90°C for 2 hours.

The reaction was monitored as follows.

Sampling was performed after a lapse of 1, and 2 hours from the start of shaking.

For the purpose of confirming the conversion rate through reaction on the solid phase with EB06-02 to be detected, under a nitrogen atmosphere, 10 μL of the suspension of the reaction solution and the resin was sampled so as to inevitably involve the resin, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in DMI (250 μL), and the solution (50 μL) was diluted with MeCN (200 μL) to prepare an LC sample. The degree of progression of the reaction was measured by LC-MS analysis. The UV area% was confirmed at a UV wavelength of 319 nm.

By using BB06 to be detected, under a nitrogen atmosphere, 5 μL of only the reaction solution of the supernatant was sampled, then diluted with MeCN (1 mL), and filtered through a vial with a filter to prepare an LC sample. An LC sample was prepared as a BB06 standard solution. LC-MS analysis was performed, and the consumption rate of the reagent was measured by calculating the peak area and concentration of BB06, followed by comparison with BB06 at the start of reaction defined as 200%. The UV area% was confirmed by PDA (210-400 nm).

The results are as shown in run BB06-1 and -2 of Table ER1-2.

[Table 13]

Table ER1-2

| Table [ER1-2] | | | | SM and TM in cleavage solution (area ratio at 319 nm) | | BB in supernatant (area ratio of boronic acid and boronic acid ester to separately prepared standard sample) (area ratio in PDA) |
|---|---|---|---|---|---|---|
| BB06 | Resin starting material | run | hr | EB06-02 | EA01-02 | BB06 |
| 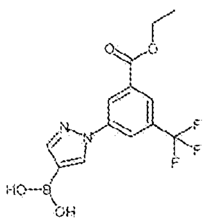 | EA01-02-01R | BB06-1 | 1 | 98.7 | 0 | 90 |
| | | BB06-2 | 2 | 97.3 | 0 | 78 |

From the results of Examples 2-2-1 to 2-2-3, 42% or more of boronic acid was able to remain even at the completion of reaction by increasing the number of equivalents of the boronic acid when the boronic acid was rapidly consumed (degraded). The consumption rate of a reagent or a substrate (here, boronic acid) under reaction conditions is very important. If reaction is delayed due to some factor, the conversion rate through reaction may be reduced, causing reduction in purity of a mixture library. These Examples indicated that the reaction of interest can be completed even if reaction delay occurs, by adopting this condition under which the number of equivalents of boronic acid is increased.

[Formula 290]

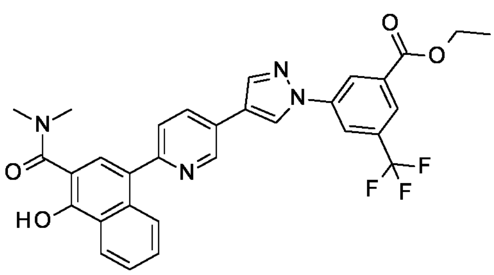

EB06-02

Ethyl 3-[4-[6-[3-(dimethylcarbamoyl)-4-hydroxynaphthalen-1-yl]pyridin-3- yl]pyrazol-1-yl]-5-(trifluoromethyl)benzoate (EB06-02)

LCMS: m/z 575[M+H]+.

Retention time: 1.47 min (analysis conditions SMD-FA05-1).

Example 2-2-4: Synthesis of EB20-02-01R

An example will be shown below in which, in the case of using boronic acid, which is unstable and has a rapid consumption rate, as a reagent, reaction was completed while the consumption of the boronic acid was suppressed, by allowing the boronic acid to exist in a stable pinacol ester form by the addition of pinacol, and changing the base to $K_3PO_4$-$H_2O$ without the addition of water.

[Formula 291]

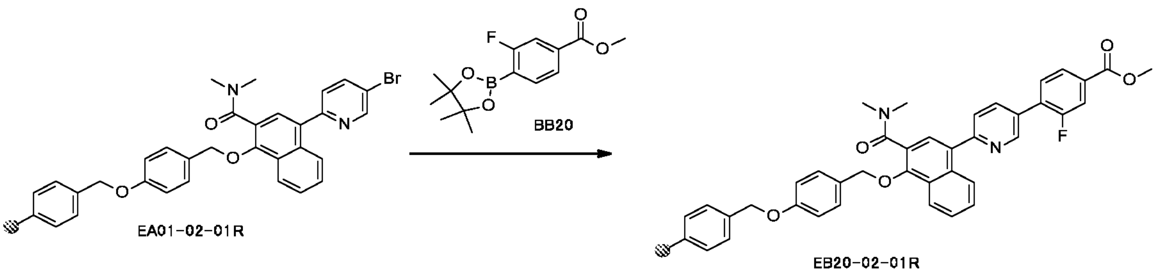


EA01-02-01R

EB20-02-01R

Under a nitrogen atmosphere, compound EA01-02-01R (20 mg, loading rate: 0.37 mmol/g, 7.4 μmol) and 1,4-dioxane (200 μL) were added to a 0.6 mL glass vial and shaken at room temperature for 1 hour. Under a nitrogen atmosphere, methyl 3-fluoro-4- (4,4,5,5-tetramethyl-1,3,2-dioxaborolan-2-yl)benzoate (BB20) (12.4 mg, 44 μmol), $K_3PO_4$- $H_2O$ (10.2 mg, 44 μmol), a solution of $P(Cy)_3$ Pd G3 in 1,4-dioxane (0.04 M, 37 μL, 1.48 μmol), and pinacol (5.3 mg, 44 μmol) were added thereto, and the mixture was shaken at 90°C for 14 hours.

The 0.04 M solution of $P(Cy)_3$ Pd G3 in 1,4-dioxane was prepared as follows.

Under a nitrogen atmosphere, 1 mL of 1,4-dioxane was added to $P(Cy)_3$ Pd G3 (26 mg, 0.04 mmol) in a 2 mL glass vial and dissolved by shaking to prepare the solution.

The reaction was monitored as follows. Sampling was performed after a lapse of 1, 2, and 14 hours from the start of shaking.

For the purpose of confirming the conversion rate through reaction on the solid phase with compound EB20-02 to be detected, under a nitrogen atmosphere, 10 μL of the suspension of the reaction solution and the resin was sampled so as to inevitably involve the resin, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in DMI(250 μL), and the solution (50 μL) was diluted with MeCN (250 μL) to prepare an LC sample. The degree of progression of the reaction was measured by LC-MS analysis. The UV area% was confirmed at a UV wavelength of 319 nm.

By using BB20 to be detected, under a nitrogen atmosphere, 5 μL of only the reaction solution of the supernatant was sampled, then diluted with MeCN (1 mL), and filtered through a vial with a filter to prepare an LC sample. BB20 (31.1 mg, 111 μmol) was completely dissolved in MeCN (595 μL), and the solution (5 μL) was diluted with MeCN (1000 μL) to prepare an LC sample as a standard solution of boronic acid. LC-MS analysis was performed, and the consumption rate of the reagent was measured by calculating the peak area and concentration of BB20, followed by comparison with BB20 at the start of reaction defined as 100%. The UV area% was confirmed by PDA (210-400 nm).

The results are as shown in run BB 20-1, -2, and -3 of Table ER1-3.

[Table 14]

[Table ER1-3]

| Table [ER1-3] | | | | SM and TM in cleavage solution (area ratio at 319 nm) | | BB in supernatant (area ratio of boronic acid and boronic acid ester to separately prepared standard sample) (area ratio in PDA) |
|---|---|---|---|---|---|---|
| BB20 | Resin starting material | run | hr | EB20-02 | EA01-02 | BB20 |
| 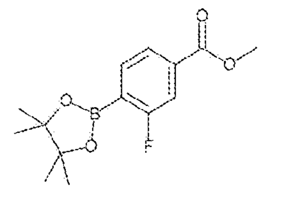 | EA01-02-01R | BB20-1 | 1 | 96.6 | 3.4 | 118 |
| | | BB20-2 | 2 | 99.5 | 0.0 | 54 |
| | | BB20-3 | 14 | 99.1 | 0.0 | 1 |

[Formula 292]

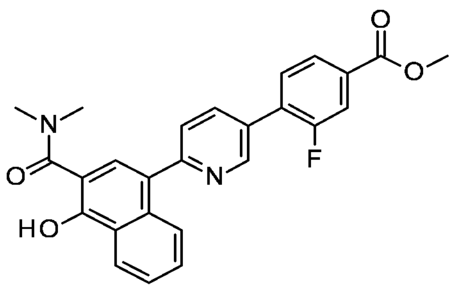

EB20-02

Methyl 4-[6-[3-(dimethylcarbamoyl)-4-hydroxynaphthalen-1-yl]pyridin-3-yl]-3- fluorobenzoate (EB20-02).

LCMS: m/z 445[M+H]$^+$.

Retention time: 1.25 min (analysis conditions SMD-FA05-1).

In the following Examples, examples will be shown in which problems of specific boronic acid were identified and solutions thereto were identified.

Example 2-2-5: Identification of challenge in Suzuki coupling to yield target compound having formyl group therein

This Example illustrates reaction using boronic acid BB19 and shows problems of Suzuki coupling reaction to yield the target compound having an formyl group therein.

[Formula 293]

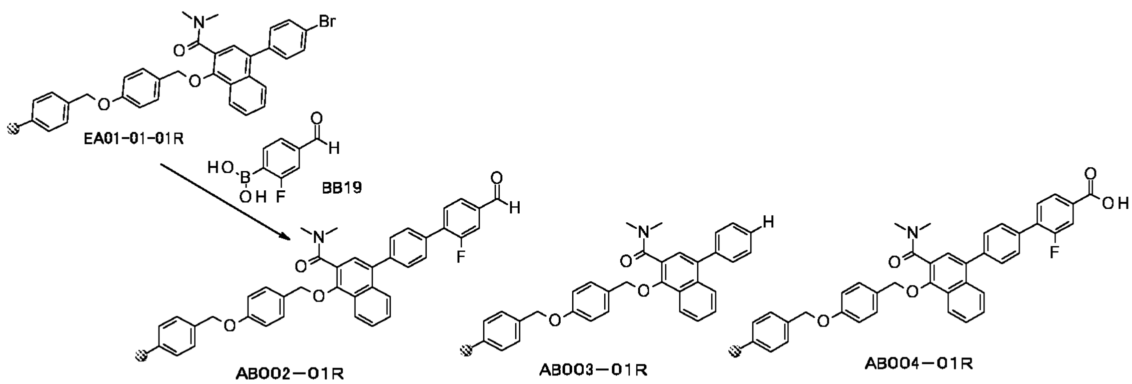

EA01-01-01R

BB19

AB002-01R

AB003-01R

AB004-01R

As shown in Table AB-01, run 1 provided approximately 97 area% of AB002 cleaved from the target compound (TM). By contrast, in run 2, 17 area% of AB002 was confirmed, and 64 area% of EA01-01 cleaved from the starting material (SM), 3 area% of a H form (AB0038) (structure; putative), and 5.6 area% of a COOH form (AB004) (structure: putative) as a new by-product were confirmed, revealing poor reproducibility of reaction (the structural formulas are shown in Table AB-02). In short, reduction in the conversion rate of the starting material and variations in the degree of progression of conversion reaction of the starting material to the H form and conversion of the target compound to the COOH form were confirmed. Although not wishing to be bound by any theory, the formation of carboxylic acid is presumably due to the oxidation reaction of a formyl group in the target compound, for example, oxidation involving palladium or Cannizzaro reaction.

[Table 15]

[Table AB-01]

| Table AB-01 | | | | | | |
|---|---|---|---|---|---|---|
| run | condition | time (h) | TM (AB002) | SM (EA01-01) | H form (AB003) | COOH form (AB004) |
| 1 | $Cy_3P$ Pd (10 mol%), $K_3PO_4$ (6 eq.) NMP (9.4 v/w), $H_2O$ (1 v/w), 90 deg. | 6 | 97.4 | 2.0 | 0.3 | nd |
| 2 | $Cy_3P$ Pd (10 mol%), $K_3PO_4$ (6 eq.) NMP (9.4 v/w), $H_2O$ (1 v/w), 90 deg. | 6 | 16.6 | 63.9 | 8.3 | 5.6 |
| | nd = not detected | | | | | |

On the other hand, when the degree of remaining boronic acid BB 19 present on the liquid-phase side was confirmed, only approximately 10% of the boronic acid BB 19 was found to remain in both run 1 and 2.

Although not wishing to be bound by any theory, a challenge identified from the results described above to Suzuki coupling reaction to yield the target compound having an formyl group therein, particularly, when a side reaction that caused boronic acid to be consumed in the liquid phase progressed, was to ensure the reproducibility of the conversion rate of a starting material on the solid phase.

Hereinafter, the experiment of run 1 of Table AB-01 will be shown as a typical experimental operation.

[Formula 294]

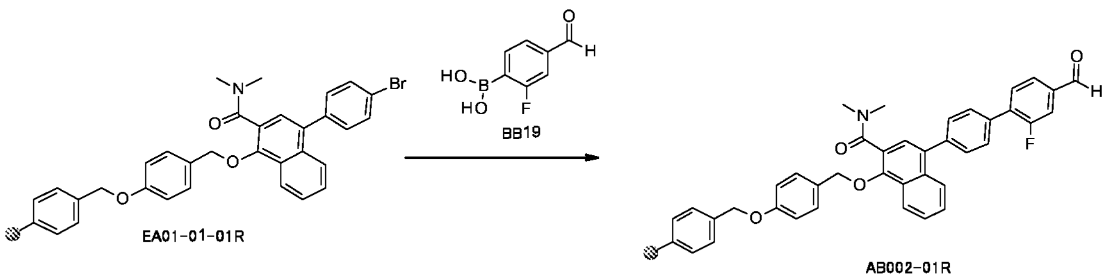


EA01-01-01R

AB002-01R

Under a nitrogen atmosphere, compound EA01-01-01R (25 mg, loading rate: 0.37 mmol/g, 7.5 μmol) and NMP (234 μL) were added to a 0.6 mL glass vial and shaken at room temperature for 1 hour. Under a nitrogen atmosphere, 2-fluoro-4-formylphenylboronic acid (BB19) (3.8 mg, 23 μmol), $K_3PO_4$ (9.6 mg, 45 μmol), a solution of $P(Cy)_3$ Pd G3 in NMP (0.04 M, 18.8 μL, 0.75 μmol), and $H_2O$ (25.0 μL, 44 μmol) were added thereto, and the mixture was shaken at 90°C for 3 hours.

The reaction was monitored as follows.

Sampling was performed both on the solid-phase side and on the liquid-phase side after a lapse of a given time from the start of shaking.

For the purpose of confirming the conversion rate through reaction on the solid phase with each compound shown in Table AB-02 to be monitored, under a nitrogen atmosphere, 10 μL of the suspension of the reaction solution and the resin was sampled, then transferred to a pipette tip with a filter, washed three times with NMP (100 μL), three times with MeOH (100 μL), and three times with DCM (100 μL), and then dipped in a 10% solution of TFA in DCM containing 0.2 M trimethylbenzene (100 μL) for 5 minutes. After filtration, the filtrate was dissolved in TFE (250 μL) to prepare an LC sample. The degree of progression of the reaction was measured by LC-MS analysis under analysis

conditions SMD-FA05-1. The UV area% was confirmed at a UV wavelength of 319 nm.

[Table 16]

Table AB-02

| Table AB-02 | | | | |
|---|---|---|---|---|
| Compound to be detected | Compound No. | Compound name | Detected m/z | Retention time |
| 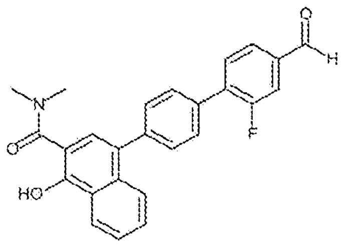 | AB002 | 4-[4-(2-Fluoro-4-formylphenyl)phenyl]-1-hydroxy-N,N-dimethylnaphthalene-2-carboxamide | 414.0 | 1.38 |
| 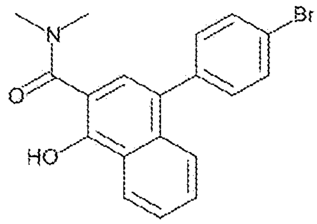 | EA01-01 | 4-(4-Bromophenyl)-1-hydroxy-N,N-dimethylnaphthalene-2-carboxamide | 369.9 | 1.42 |
| 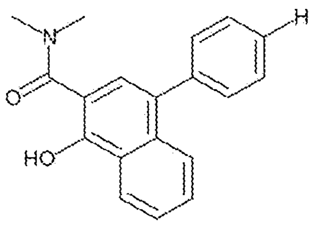 | AB003 | 1-Hydroxy-N,N-dimethyl-4-phenylnaphthalene-2-carboxamide | 292.1 | 1.28 |
| 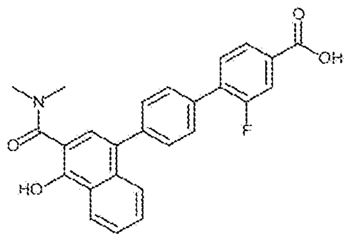 | AB004 | 4-[4-[3-(Dimethylcarbamoyl)-4-hydroxynaphthalen-1-yl]phenyl]-3-fluorobenzoic acid | 430.0 | 1.22 |

For the purpose of confirming a remaining reagent on the liquid-phase side with BB19 to be monitored, under a nitrogen atmosphere, 5 μL of only the reaction solution of the supernatant was sampled, then diluted with MeCN (1 mL), and filtered through a vial with a filter to prepare an LC sample. The degree of progression of the reaction was measured by LC-MS analysis under analysis conditions SMD-FA05-1. The UV area% was confirmed by PDA (cumulative UV area at UV wavelengths of 210-400 nm).

Compound to be monitored on the liquid-phase side

(2-Fluoro-4-formylphenyl)boronic acid (BB19)

[Formula 295]

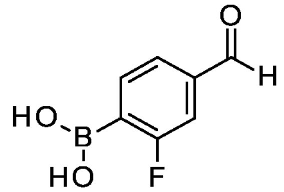

LCMS: m/z not detected[M+H]+.

Retention time: 0.61 min (analysis conditions SMD-FA05-1).

Example 2-2-6: Synthesis of EB19-02-01R

Reaction conditions under which boronic acid was allowed to exist as a stable pinacol ester form by the addition of pinacol while the consumption of the boronic acid was restricted through a limited number of equivalents of water were searched for with reference to Non Patent Reference (ACS Catal. 2018, 8, 4, 2989-2994) as to boronic acid which was rapidly consumed in the liquid phase. As a result, reaction was completed while the consumption of boronic acid was suppressed, by changing the base to $K_3PO_4$-$H_2O$ without the addition of water. An exemplary operation will be shown.

[Formula 296]

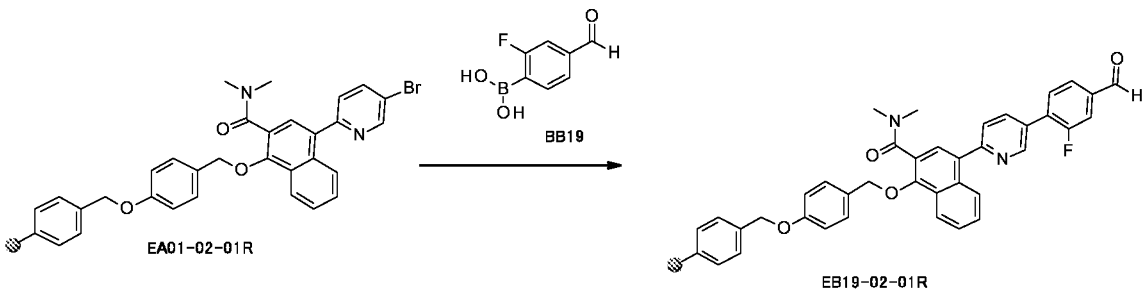


Under a nitrogen atmosphere, compound EA01-02-01R (20 mg, loading rate: 0.37 mmol/g, 7.4 μmol) and 1,4-dioxane (200 μL) were added to a 0.6 mL glass vial and shaken at room temperature for 1 hour. Under a nitrogen atmosphere, methyl 3-fluoro-4- (4,4,5,5-tetramethyl-1,3,2-dioxaborolan-2-yl)benzoate (BB19) (4.5 mg, 44 μmol), $K_3PO_4$-$H_2O$ (10.2 mg, 44 μmol), a solution of $P(Cy)_3$ Pd G3 in 1,4-dioxane (0.04 M, 37 μL, 1.48 μmol), and pinacol (5.3 mg, 44 μmol) were added thereto, and the mixture was shaken at 90°C for 21 hours.

The reaction was monitored as follows. Sampling was performed after a lapse of 1, 3, 7, and 21 hours from the start of shaking. For the purpose of confirming the conversion rate through reaction on the solid phase with compound EB19-02 to be detected, under a nitrogen atmosphere, 10 μL of the suspension of the reaction solution and the resin was sampled so as to inevitably involve the resin, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in DMI (250 μL), and the solution (50 μL) was diluted with MeCN (250 μL) to prepare an LC sample. The degree of progression of the reaction was measured by LC-MS analysis. The UV area% was confirmed at a UV wavelength of 319 nm.

By BB19 to be detected, under a nitrogen atmosphere, 5 μL of only the reaction solution of the supernatant was sampled, then diluted with MeCN (1 mL), and filtered through a vial with a filter to prepare an LC sample. A standard solution and an LC sample of BB19 were prepared by the same operation as in BB20 in Example 2-2-4 and subjected to LC-MS analysis. The consumption rate of the reagent was measured by calculating the peak area and concentration of BB19, followed by comparison. The UV area% was confirmed by PDA (210-400 nm).

The results are as shown in run BB19-1, -2, -3, and -4 of Table ER1-4.

[Table 17]

Table ER1-4

| Table [ER1-4] | | | | SM and TM in cleavage solution (area ratio at 319 nm) | | BB in supernatant (area ratio of boronic acid and boronic acid ester to separately prepared standard sample) (area ratio in PDA) |
|---|---|---|---|---|---|---|
| BB19 | Resin starting material | run | hr | EB19-02 | EA01-02 | BB19 |
| 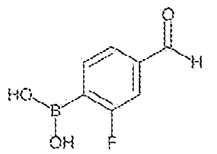 | EA01-02-01R | BB19-1 | 1 | 39.1 | 60.9 | 176 |
| | | BB19-2 | 3 | 99.3 | 0.0 | 114 |
| | | BB19-3 | 7 | 97.7 | 0.0 | 117 |
| | | BB19-4 | 21 | 97.5 | 0.0 | 17 |

Thus, as in the results of Examples 2-2-4 to 2-2-6, conditions under which boronic acid was not consumed at the completion of reaction were able to be set by: allowing the boronic acid to be in a stable pinacol ester form by the addition of pinacol when the boronic acid was unstable and had a rapid boronic acid consumption rate due to protodeborylation; and using a hydrate of a base (here, potassium phosphate monohydrate) as a method of adding a bare minimum amount of water for the progression of reaction, instead of aggressively adding water. In an embodiment, the conditions appropriately adopted in library synthesis enables a high conversion rate through reaction to be achieved and enables the quality of a mixture library to be ensured.

4-[5-(2-Fluoro-4-formylphenyl)pyridin-2-yl]-1-hydroxy-N,N-dimethylnaphthalene- 2-carboxamide (EB19-02)

[Formula 297]

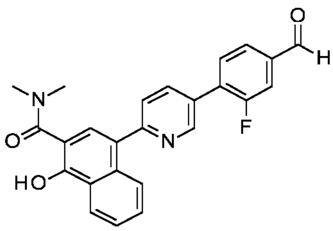

EB19-02

LCMS: m/z 415[M+H]$^+$

Retention time: 1.14 min (analysis conditions SMD-FA05-1)

EB19-02-01R whose synthesis method is illustrated in Example 2-2-6 is also referred to as compound AC005-01R in the subsequent examples.

Example 2-2-7: Synthesis of EB08-15-01R

In the case of a target compound having a formyl group, side reaction that seemed to be Cannizzaro reaction progressed under the conditions of Examples 2-2-0 to 2-2-4. An example will be shown in which reaction was completed under conditions that solved the problem. In the example shown, the reproducibility of solubility of a base in a NMP-water mixed solvent was enhanced by the addition of water in the step of swelling a resin so that the reproducibility of the rate of progression of reaction was enhanced even under conditions having a slow rate of progression of reaction, thereby reducing variations among batches.

[Formula 298]

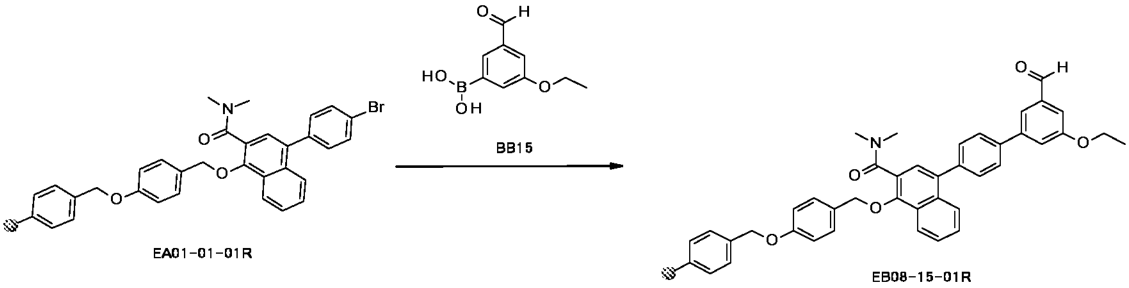


Under a nitrogen atmosphere, NMP (300 μL) and water (15 μL) were added to compound EA01-01-01R (30 mg, loading rate: 0.4 mmol/g, 12 μmol) in a 0.6 mL glass vial, and the mixture was shaken at room temperature for 1 hour. Under a nitrogen atmosphere, (3-ethoxy-5-formylphenyl)boronic acid (BB15) (14 mg, 72 μmol), $Na_2CO_3$ (7.6 mg, 72 μmol), and a solution of $P(Cy)_3$ Pd G3 in NMP (0.04 M, 60 μL, 1.48 μmol) were added thereto, and the mixture was shaken at 90°C for 91 hours.

The 0.04 M solution of $P(Cy)_3$ Pd G3 in NMP was prepared in the same manner as in EB02-05-01R.

The reaction was monitored as follows.

Sampling was performed after a lapse of 1, 3, 19, 25, and 91 hours from the start of shaking.

For the purpose of confirming the conversion rate through reaction on the solid phase with EB08-15 to be detected, under a nitrogen atmosphere, 10 μL of the suspension of the reaction solution and the resin was sampled so as to inevitably involve the resin, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in DMI (250 μL), and the solution (50 μL) was diluted with MeCN (200 μL) to prepare an LC sample. The degree of progression of the reaction was measured by LC-MS analysis. The UV area% was confirmed at a UV wavelength of 319 nm.

By BB15 to be detected, under a nitrogen atmosphere, 5 μL of only the reaction solution of the supernatant was sampled, then diluted with MeCN (1 mL), and filtered through a vial with a filter to prepare an LC sample. A standard solution and an LC sample of BB15 were prepared by the same operation as in BB20 in Example 2-2-4 and subjected to LC-MS analysis. The consumption rate of the reagent was measured by calculating the peak area and concentration of BB15, followed by comparison. The UV area% was confirmed by PDA (210-400 nm).

The results are as shown in run BB15-1, -2, -3, -4, and -5 of Table ER1-5.

[Table 18]

Table ER1-5

| Table [ER1-5] | | | | SM and TM in cleavage solution (area ratio at 319 nm) | | BB in supernatant (area ratio of boronic acid and boronic acid ester to separately prepared standard sample) (area ratio in PDA) |
|---|---|---|---|---|---|---|
| BB15 | Resin starting material | run | hr | EB08-15 | EA01-01 | BB15 |
| 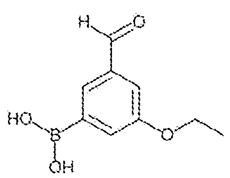 | EA01-01-01R | BB15-1 | 1 | 48.1 | 50.5 | 123 |
| | | BB15-2 | 3 | 67.7 | 30.8 | 116 |
| | | BB15-3 | 19 | 96.6 | 2.1 | 108 |
| | | BB15-4 | 25 | 97.1 | 1.8 | 101 |
| | | BB15-5 | 91 | 97.3 | 0.3 | 80 |

[Formula 299]

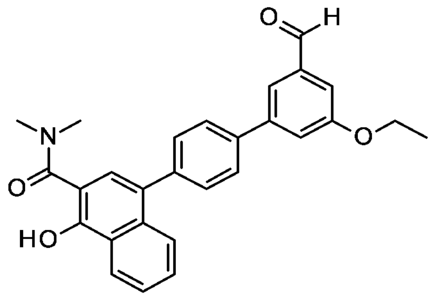

EB08-15

4-[4-(3-Ethoxy-5-formylphenyl)phenyl]-1-hydroxy-N,N-dimethylnaphthalene-2- carboxamide (EB08-15)

LCMS: m/z 440$[M+H]^+$.

Retention time: 1.47 min (analysis conditions SMD-FA05-1).

Example 2-2-8: Synthesis of EB21-02-01R

An example will be shown in which reaction was completed while the problem of Cannizzaro reaction was solved, by changing the solvent from NMP to 4-MeTHP and thereby suppressing the consumption of boronic acid.

[Formula 300]

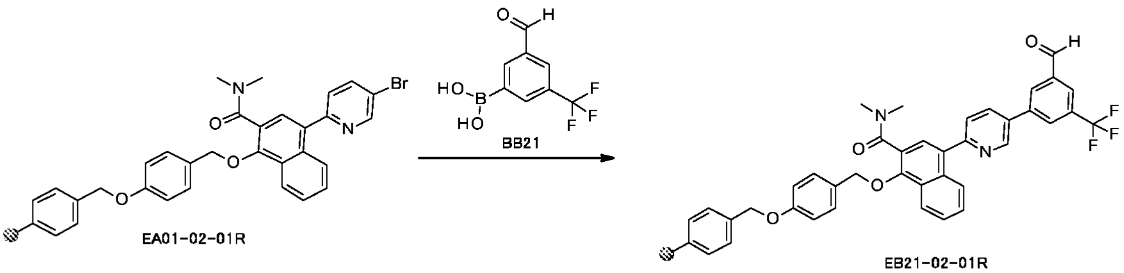


EA01-02-01R

EB21-02-01R

Under a nitrogen atmosphere, 4-MeTHP (200 μL) was added to compound EA01- 02-01R (20 mg, loading rate: 0.37 mmol/g, 7.4 μmol) in a 0.6 mL glass vial, and the mixture was shaken at room temperature for 1 hour. Under a nitrogen atmosphere, [3-formyl-5- (trifluoromethyl)phenyl]boronic acid (BB21) (9.7 mg, 44 μmol), $Na_2CO_3$ (4.7 mg, 44 μmol), a solution of $P(Cy)_3$ Pd G3 in 4-MeTHP (0.04 M, 37 μL, 1.48 μmol), and water (10 μL) were added thereto, and the mixture was shaken at 90°C for 21 hours.

The 0.04 M solution of $P(Cy)_3$ Pd G3 in 4-MeTHP was prepared as follows.

Under a nitrogen atmosphere, 1 mL of a 4-MeTHP solution was added to $P(Cy)_3$ Pd G3 (26 mg, 0.04 mmol) in a 2 mL glass vial and dissolved by shaking to prepare the solution.

The reaction was monitored as follows.

Sampling was performed after a lapse of 1, 3, and 21 hours from the start of shaking.

For the purpose of confirming the conversion rate through reaction on the solid phase with EB21-02 to be detected, under a nitrogen atmosphere, 10 μL of the suspension of the reaction solution and the resin was sampled so as to inevitably involve the resin, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in DMI (250 μL), and the solution (50 μL) was diluted with MeCN (200 μL) to prepare an LC sample. The degree of progression of the reaction was measured by LC-MS analysis. The UV area% was confirmed at a UV wavelength of 319 nm.

By BB21 to be detected, under a nitrogen atmosphere, 50 μL of the filtrate washed three times with NMP was collected, diluted with MeCN (1 mL), and filtered through a vial with a filter to prepare an LC sample. A standard solution and an LC sample of BB21 were prepared by the same operation as in BB20 in Example 2-2-4 and subjected to LC-MS analysis. The consumption rate of the reagent was measured by calculating the peak area and concentration of BB21, followed by comparison. The UV area% was confirmed by PDA (210-400 nm).

The results are as shown in run BB21-1, -2, and -3 of Table ER1-6.

[Table 19]

Table ER1-6

| Table [ER1-6] | | | | SM and TM in cleavage solution (area ratio at 319 nm) | | BB in supernatant (area ratio of boronic acid and boronic acid ester to separately prepared standard sample) (area ratio in PDA) |
|---|---|---|---|---|---|---|
| BB21 | Resin starting material | run | hr | EB21-02 | EA01-02 | BB21 |
| 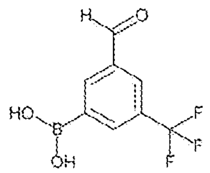 | EA01-02-01R | BB21-1 | 1 | 78.3 | 19.8 | 57 |
| | | BB21-2 | 3 | 98.0 | 2.0 | 41 |
| | | BB21-3 | 14 | 94.4 | 0.0 | 34 |

[Formula 301]

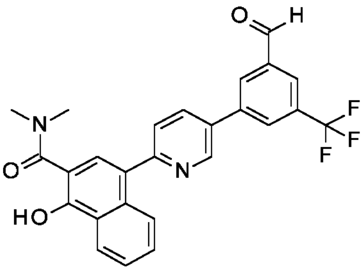

EB21-02

4-[5-[3-Formyl-5-(trifluoromethyl)phenyl]pyridin-2-yl]-1-hydroxy-N,N- dimethylnaphthalene-2-carboxamide (EB21-02)

LCMS: m/z 411[M+H]+.

Retention time: 1.27 min (analysis conditions SMD-FA05-1).

EB21-02-01R whose synthesis method is illustrated in Example 2-2-8 is also referred to as compound AC007-01R in the subsequent examples.

The results of this experiment revealed that the approach of the present invention is capable of ensuring the robustness of Suzuki coupling reaction. Specifically, reaction conditions under which a reagent and a substrate on the liquid-phase side remained without being degraded even at the completion of reaction were identified for Ar-Br confirmed to have low reactivity (in other words, a substrate on the solid-phase side that requires a longer reaction time for the completion of reaction) among substrates on the solid-phase side contained in a library. Use of the conditions in library synthesis can achieve a high conversion rate through reaction even if reaction delay occurs due to some factor, because the reagent and the substrate remain. This is an exemplary approach of improving the robustness of reaction for the purpose of supplying a "high-quality" mixture library.

Example 2-2-9: Suzuki coupling reaction using organic base

Conditions will be shown under which use of an organic base rendered Suzuki coupling applicable to a wide range of substrates with high functional group tolerance.

Example 2-2-9-1: Synthesis of C207-03R through Suzuki coupling of solid-phase supported ArBr (C011-03R) with 2,6-dimethylphenylboronic acid (C305)

In Suzuki coupling reaction using BTMG as an organic base, the cleavage of the target compound from the solid phase progresses depending on a Pd catalyst. By contrast, an example will be shown in which, in the case of using

meCgPPh Pd G4 or (dtbpf)$PdCl_2$ as a Pd catalyst, Suzuki coupling reaction can be performed at high yields without cleavage from the solid phase. The substrate is not limited to those given below in actual application to library synthesis.

Example 2-2-9-1-1: Suzuki coupling reaction using meCgPPh Pd G4 as Pd catalyst

[Formula 302]

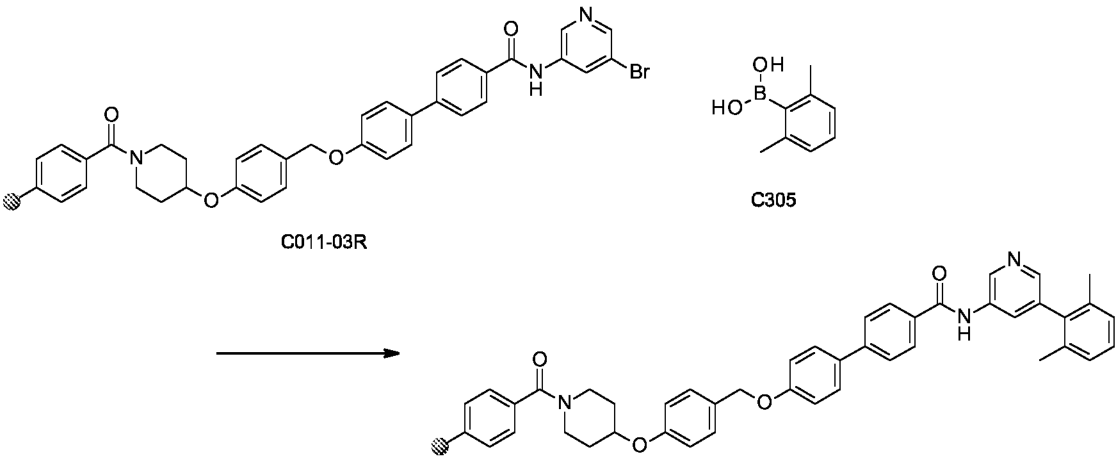

C011-03R

C305

C207-03R

Under a nitrogen atmosphere, compound C011-03R (0.195 mmol/g, 20 mg), THF (0.4 mL), and water (2.11 μL, 0.117 mmol) were added to a 0.6 mL glass vial and shaken at room temperature for 1 hour. meCgPPh Pd G4 (2.6 mg, 0.0039 mmol), a solution of 2,6- dimethylphenylboronic acid (C305) in THF (2.0 M, 39.0 μL, 0.078 mmol), and BTMG (23.0 μL, 0.117 mmol) were added thereto, and the mixture was shaken at 60°C for 24 hours.

As a result of performing the LC-MS analysis of a solution containing 3 μL of the supernatant of the reaction solution dissolved in MeCN (0.5 mL), compound C207 of interest cleaved from the solid phase was not observed.

12 μL of the suspension of the reaction solution and the resin was transferred onto a tip with a filter and washed three times with NMP (0.1 mL), three times with MeOH (0.1 mL), and three times with DCM (0.1 mL). The resultant was dipped in a 10% solution of TFA in DCM containing pentamethylbenzene (0.1 M, 0.05 mL) for 2 minutes, filtered, and washed with NMP (0.05 mL). Combined filtrates were diluted with MeCN (0.25 mL), and the solution was subjected to LC-MS analysis, so that 99.8% of compound C207 of interest was observed. However, the compound was cleaved and analyzed at a wavelength of 299 nm (±4 nm).

[Formula 303]

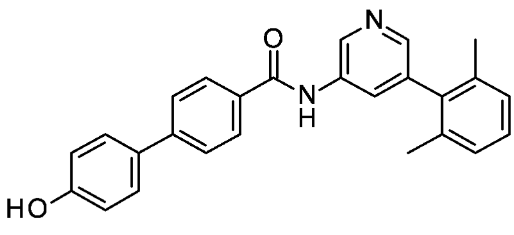

Compound C207

LRMS: m/z 395$[M+H]^+$.

Retention time: 1.040 min (analysis conditions SMD-FA05-1).

Example 2-2-9-1-2: Suzuki coupling reaction using (dtbpf)$PdCl_2$ as Pd catalyst

The same operation as in Example 2-2-9-1-1 was performed using (dtbpf)PdClz (2.5 mg, 0.0039 mmol) instead of meCgPPh Pd G4, and the obtained mixture was shaken at 60°C for 24 hours.

As a result of performing the LC-MS analysis of a solution containing 3 μL of the supernatant of the reaction

solution dissolved in MeCN (0.5 mL), compound C207 of interest cleaved from the solid phase was not observed.

12 μL of the suspension of the reaction solution and the resin was transferred onto a tip with a filter and washed three times with NMP (0.1 mL), three times with MeOH (0.1 mL), and three times with DCM (0.1 mL). The resultant was dipped in a 10% solution of TFA in DCM containing pentamethylbenzene (0.1 M, 0.05 mL) for 2 minutes, filtered, and washed with NMP (0.05 mL). Combined filtrates were diluted with MeCN (0.25 mL), and the solution was subjected to LC-MS analysis, so that 99.2% of compound C207 of interest was observed. However, the compound was cleaved and analyzed at a wavelength of 299 nm (±4 nm).

Example 2-2-9-1-3: Suzuki coupling reaction using XPhos Pd G4, RuPhos Pd G4, APhos Pd G4, cataCXium Pd G4 as Pd catalyst

The same operation as in Example 2-2-9-1-1 was performed using XPhos Pd G4 (3.4 mg, 0.0039 mmol), RuPhos Pd G4 (3.3 mg, 0.0039 mmol), APhos Pd G4 (2.5 mg, 0.0039 mmol), or CataCXium Pd G4 (2.9 mg, 0.0039 mmol) instead of meCgPPh Pd G4, and the obtained mixture was shaken at 60°C for 24 hours.

As a result of performing the LC-MS analysis of a solution containing 3 μL of the supernatant of the reaction solution dissolved in MeCN (0.5 mL), compound C207 of interest cleaved from the solid phase was observed under any of the conditions.

The results of Examples 2-2-9-1-1 to -3 indicated that: in the case of using XPhos Pd G4, RuPhos Pd G4, APhos Pd G4, or cataCXium Pd G4 as a Pd catalyst for a solid-phase supported substrate of arenol through an ether bond via a p-alkoxybenzyl linker typified by Wang resin, the cleavage of the target compound from the solid phase progresses during Suzuki coupling reaction, causing reduction in yield; by contrast, in the case of using meCgPPh Pd G4 or (dtbpf)$PdCl_2$ as a Pd catalyst, Suzuki coupling reaction can be performed at high yields without cleavage from the solid phase.

Example 2-2-9-2: Synthesis of C204-03R by Suzuki coupling of solid-phase supported ArBr (C009-03R) and 2,6-dimethylphenylboronic acid (C305)

[Formula 304]

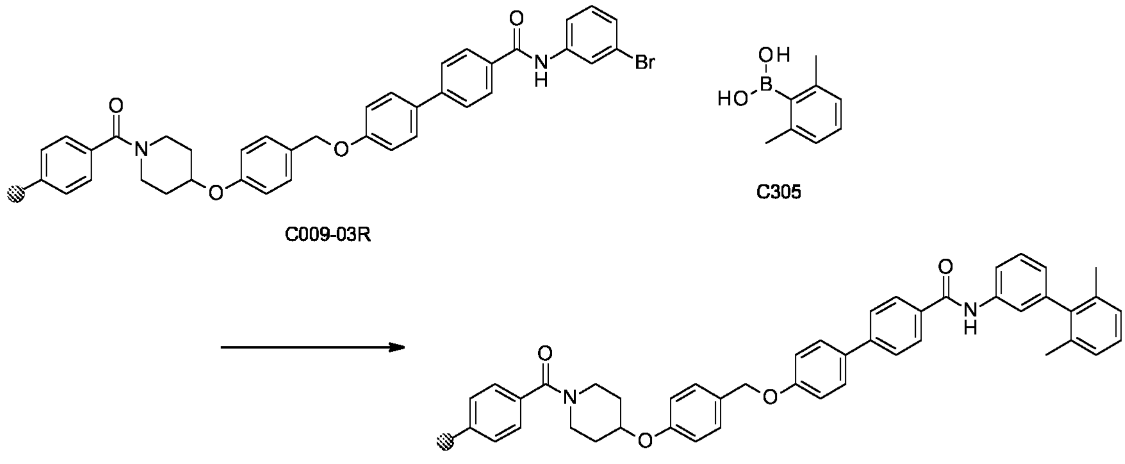

C009-03R C305 C204-03R

Under a nitrogen atmosphere, compound C009-03R (0.195 mmol/g, 20 mg), THF (0.4 mL), and water (2.11 μL, 0.117 mmol) were added to a 0.6 mL glass vial and shaken at room temperature for 1 hour. meCgPPh Pd G4 (2.6 mg, 0.0039 mmol), a solution of 2,6- dimethylphenylboronic acid (C305) in THF (2.0 M, 39.0 μL, 0.078 mmol), and BTMG (23.0 μL, 0.117 mmol) were added thereto, and the mixture was shaken at 60°C for 30 minutes.

12 μL of the suspension of the reaction solution and the resin was transferred onto a tip with a filter and washed three times with NMP (0.1 mL), three times with MeOH (0.1 mL), and three times with DCM (0.1 mL). The resultant was dipped in a 10% solution of TFA in DCM containing pentamethylbenzene (0.1 M, 0.05 mL) for 2 minutes, filtered, and washed with NMP (0.05 mL). Combined filtrates were diluted with MeCN (0.25 mL), and the solution was subjected to LC-MS analysis, so that 99.6% of compound C204 of interest was observed. However, the compound was cleaved and

analyzed at a wavelength of 299 nm (±4 nm).

[Formula 305]

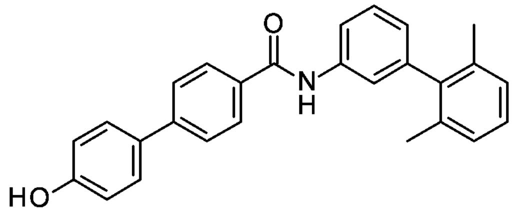

Compound C204

LRMS: m/z 394[M+H]+

Retention time: 1.335 min (analysis conditions SMD-FA05-1).

The results of Example 2-2-9-1 and -2 indicated that the Suzuki coupling reaction of solid-phase supported ArBr with boronic acid can be carried out under a mild condition of 60°C using an organic base BTMG.

Example 2-2-10: Solid-phase experiment of Suzuki coupling using solid-phase supported Ar boronic acid ester

Solid-phase experiment (exemplary reaction operation) of Suzuki coupling of solid- phase supported Ar boronic acid ester (D122-03R) and ethyl 4-(4-hydroxyphenyl)benzoate (D124)

[Formula 306]

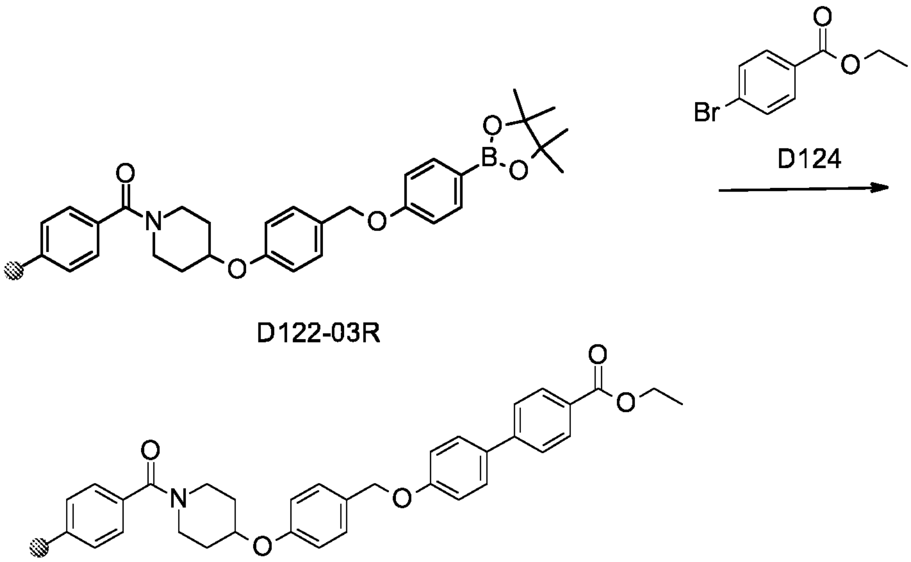

D124

D122-03R

D123-03R

Under a nitrogen atmosphere, solid-phase supported Ar boronic acid ester (D122- 03R) (loading rate: 0.198 mmol/g, 8.0 mg, 1.6 μmol) and THF (0.304 mL) were added to a 0.6 mL glass vial and shaken at room temperature for 1 hour. cataCXiumA Pd G4 (2.2 mg, 3.0 μmol), ethyl 4-bromobenzoate (D124, 13.79 mg, 0.060 mmol), BTMG (17.7 μL, 0.089 mmol), and water (1.6 μL, 0.089 mmol) were added thereto, and the mixture was shaken at 60°C for 1 hour.

The reaction was monitored as follows.

Under a nitrogen atmosphere, 12 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.02 M pentamethylbenzene (100 μL) for 5 minutes.

The cleavage solution was filtered, and the solid phase was washed with NMP (50 μL). The obtained filtrate was diluted with MeCN (500 μL) to prepare an LC sample. The degree of progression of the reaction was measured by LC-MS analysis. As a result, 98.4 area% of compound D123 of interest was observed.

[Formula 307]

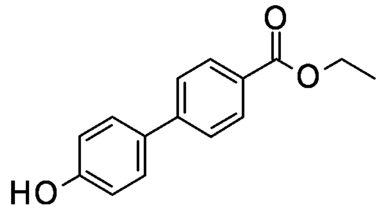

D123

Compound D123
LRMS: m/z 243[M+H]$^+$.
Retention time: 1.085 min (analysis conditions SMD-FA05-1, 295 nm).

Example 2-3: Experiment to confirm scope of substrate application to acetylene coupling reaction mediated by Pd catalyst as typical example of identifying substrate scope that can be applied to library synthesis

Hereinafter, exemplary conditions for enhancing robustness by selecting suitable reaction conditions for acetylene coupling reaction will be illustrated. The scope given below is a mere illustration of an exemplary identification method, and the present invention is not limited by the scope given below in actual application to library synthesis.

Example 2-3-1: Solid-phase experiment of coupling reaction of solid-phase supported Ar-Br with acetylene derivative

Conditions without the use of CuI were selected as possible reaction conditions applicable to a wide scope of substrates with reference to literatures known in the art (J. Org. Chem. 2013, 78, 568-581; J. Orgnomet. Chem. 1975, 93, 253; and J. Organomet. Chem. 1975, 93, 259). DIA was selected as a base from organic bases easily adaptable for solid- phase reaction, and $Pd(PPh_3)_4$ was selected as a Pd catalyst. Coupling reaction with an acetylene derivative was carried out.

Example 2-3-1-1: Coupling reaction (exemplary reaction operation) of solid-phase supported ArBr (EA01-01-01R) with methyl 2-ethynylbenzoate (BB24)

[Formula 308]

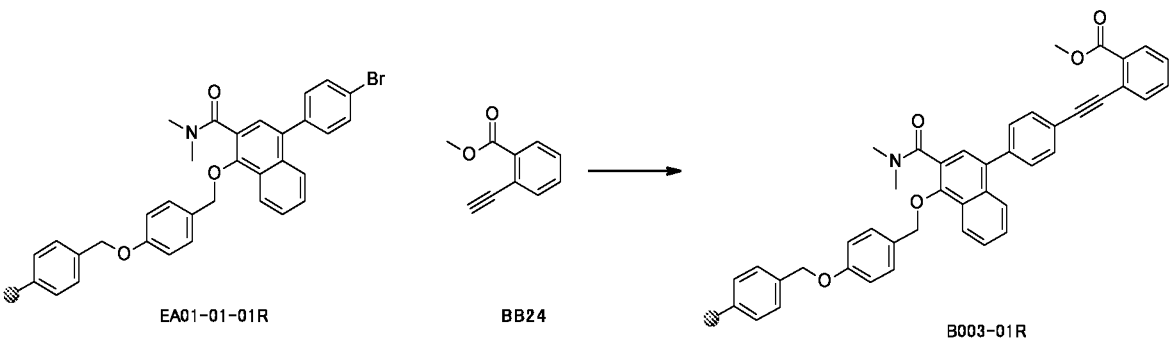

EA01-01-01R BB24 B003-01R

Under a nitrogen atmosphere, solid-phase supported ArBr (EA01-01-01R) (0.29 mmol/g, 50.0 mg, 0.015 mmol, 1.0 equivalent), NMP (437.5 μL), and DIA (62.5 μL) were added to a 1.5 mL screw-cap vial and shaken at room temperature for 1 hour. Under a nitrogen atmosphere, methyl 2-ethynylbenzoate (BB24) (14.0 mg, 0.087 mmol, 6 equivalents) and $Pd(PPh_3)_4$ (5.03 mg, 0.0043 mmol, 0.3 equivalents) were added thereto, and the obtained mixture was shaken at 80°C for 22 hours.

Reaction monitoring

After a lapse of 3 hours from the start of reaction, under a nitrogen atmosphere, 10 μL of the suspension of the reaction solution and the resin was sampled, transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in TFE (1000 μL) to prepare an LC sample. The degree of progression of the reaction was measured by LC-MS analysis.
As a result, 2.7% of the starting material ArBr remained, and 95.9% of the product in an acetylene form (B003) was observed.
After a lapse of 22 hours at the completion of the reaction, it was confirmed that the starting material ArBr was consumed and reaction was completed.
From these results, it was confirmed that coupling reaction under this condition can be carried out on the solid phase.

[Formula 309]

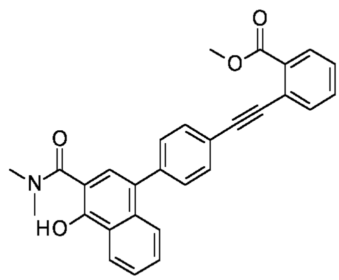

Methyl 2-[2-[4-[3-(dimethylcarbamoyl)-4-hydroxynaphthalen-1- yl]phenyl]ethynyl]benzoate:compound B003

LCMS: m/z 450.1[M+H]$^+$.
Retention time: 1.514 min (analysis conditions SMD-FA05-1, 319 nm).
(Retention time of the starting material ArBr: 1.421 min (analysis conditions SMD- FA05-1, 319 nm).

Example 2-3-2: Coupling reaction of solid-phase supported ArBr (EA01-01-01R) with ethyl 3-ethynyl-5-(trifluoromethyl)benzoate (BB25) (experiment to identify challenge using $Pd(PPh_3)_4$ catalyst)

A reaction example will be shown below in which the coupling reaction of an acetylene derivative having ester and a trifluoromethyl group serving as an electron- withdrawing group is not completed using $Pd(PPh_3)_4$ as a catalyst.

[Formula 310]

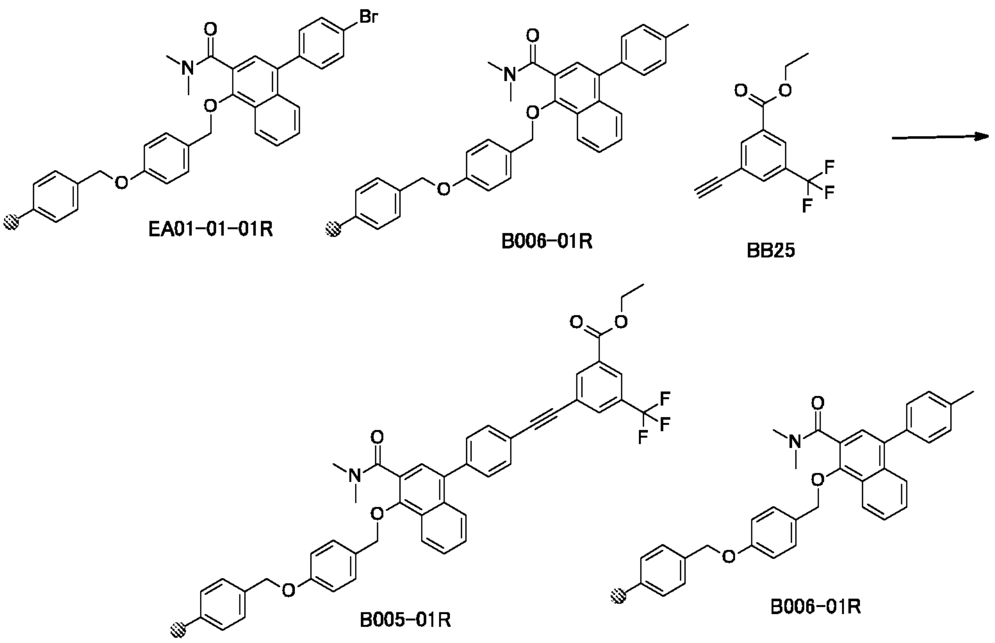

EA01-01-01R B006-01R BB25

B005-01R B006-01R

Under a nitrogen atmosphere, solid-phase supported ArBr (EA01-01-01R) (0.3 mmol/g, 50.0 mg, 15.0 μmol, 1.0 equivalent), solid-phase supported reagent B006-01R (added as an internal standard reagent, 0.773 mg, 0.1 equivalents), and NMP (262.5 μL) were added to a 1.5 mL screw-cap vial and shaken at room temperature for 1 hour.

Under a nitrogen atmosphere, ethyl 5-trifluoromethyl-3-ethynylbenzoate (BB25) (18.0 mg, 0.075 mmol, 5 equivalents), DIA (37.5 μL), and $Pd(PPh_3)_4$ (5.20 mg, 0.0045 mmol, 0.3 equivalents) were added thereto, and the obtained mixture was shaken at 80°C for 15 hours.

Reaction monitoring

After a lapse of 15 hours from the start of reaction, under a nitrogen atmosphere, 10 μL of the suspension of the reaction solution and the resin was sampled, transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (100 μL) for 5 minutes. After filtration, the filtrate was dissolved in TFE (100 μL) to prepare an LC sample. The degree of progression of the reaction was measured by LC-MS analysis.

As a result, 35.1 % of the starting material ArBr remained, and 54.1% of the product in an acetylene form (B005) was observed.

From these results, it was confirmed that, in the case of using an electron-poor acetylene derivative such as BB25 used in this Example as a substrate, a catalyst and reaction conditions need to be studied for completing reaction.

[Formula 311]

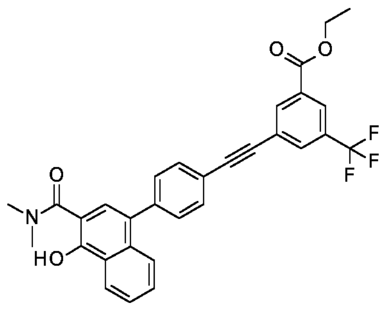

Ethyl 3-[2-[4-[3-(dimethylcarbamoyl)-4-hydroxynaphthalen-1-yl]phenyl]ethynyl]-5- (trifluoromethyl)benzoate: compound B005

LCMS: m/z 532.1[M+H]$^+$.

Retention time: 1.204 min (analysis conditions SMD-FA50-1, 319 nm).

(Retention time of the starting material ArBr: 0.786 min (analysis conditions SMD- FA50-1, 319 nm).

B034-01R whose synthesis method is illustrated in Example 2-3-4 is also referred to as compound AF001-01R in the subsequent examples.

Example 2-3-3: Coupling reaction of solid-phase supported ArBr (EA01-01-01R) with acetylene derivative (BB25) (overcoming of challenge using $Pd(PPh_3)_4$ as catalyst)

As a result of studying reaction conditions as to the substrate for which the conversion rate through reaction remained at 35% in Example 2-3-2, it was confirmed that reaction can be completed by changing the catalyst to Xphos Pd G4. Hereinafter, a specific operation will be shown.

[Formula 312]

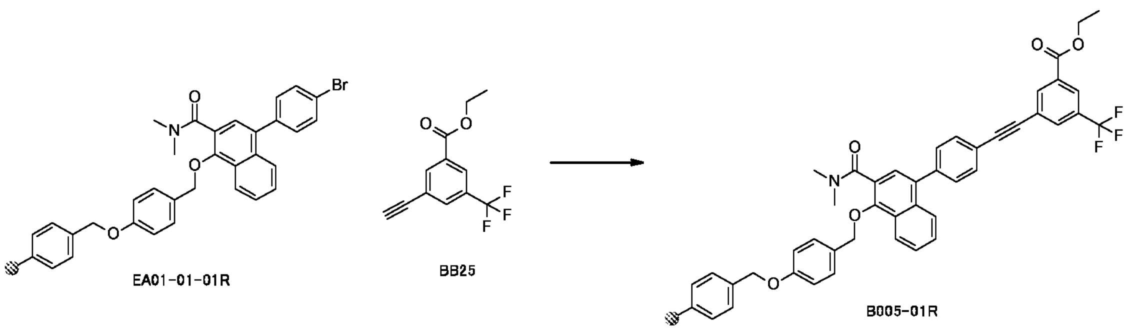

Under a nitrogen atmosphere, solid-phase supported ArBr (EA01-01-01R) (0.29 mmol/g, 50.0 mg, 15.0 μmol, 1.0 equivalent), NMP (437.5 μL), and DIA (62.5 μL) were added to a 1.5 mL screw-cap vial and shaken at room temperature for 1 hour. Under a nitrogen atmosphere, ethyl 5-trifluoromethyl-3-ethynylbenzoate (BB25) (21.0 mg, 0.087 mmol, 6 equivalents) and Xphos Pd G4 (1.87 mg, 0.0021 mmol, 0.15 equivalents) were added thereto, and the obtained mixture was shaken at 80°C for 22 hours.

Reaction monitoring

After a lapse of 1 hour from the start of reaction, under a nitrogen atmosphere, 10 μL of the suspension of the reaction solution and the resin was sampled, transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in TFE (1000 μL) to prepare an LC sample. The degree of progression of the reaction was measured by LC-MS analysis.

As a result, 36.5% of the starting material ArBr remained, and 61.9% of the product in an acetylene form (B005) was observed.

Then, the reaction was monitored over time (3, 5, 7, and 22 hours). After a lapse of 22 hours, the starting material was consumed, and 98.8% of the acetylene form (B005) was observed.

From these results, reaction conditions were found under which reaction with electron-poor acetylene confirmed to cause insufficient progression of reaction using $Pd(PPh_3)_4$ can be completed by changing the catalyst to Xphos Pd G4.

Example 2-3-4: Coupling reaction of solid-phase supported ArBr (EA01-02-01R) with acetylene derivative (BB25)

The reaction of ArBr having a pyridine ring as a substrate on the solid-phase side with an electron-poor acetylene derivative was confirmed under the reaction conditions found in Example 2-3-3.

[Formula 313]

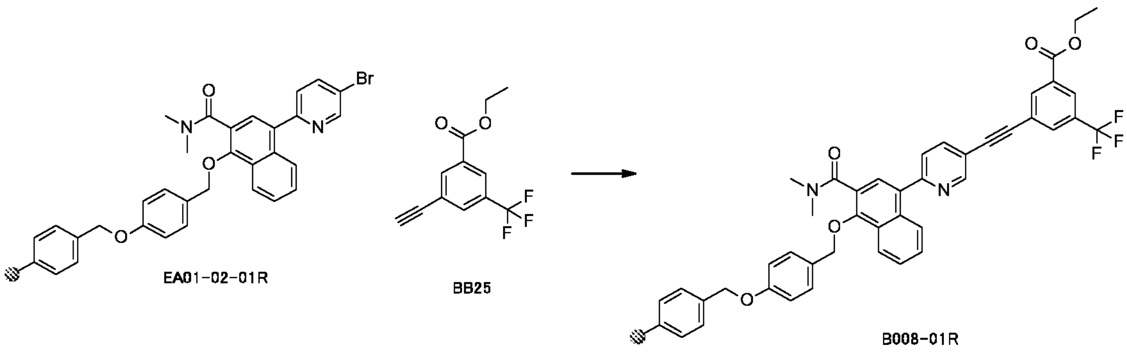

Under a nitrogen atmosphere, solid-phase supported ArBr (EA01-02-01R) (0.32 mmol/g, 50.0 mg, 16.0 μmol, 1.0 equivalent), NMP (437.5 μL), and DIA (62.5 μL) were added to a 1.5 mL screw-cap vial and shaken at room temperature for 1 hour. Under a nitrogen atmosphere, ethyl 5-trifluoromethyl-3-ethynylbenzoate (BB25) (23.0 mg, 0.096 mmol, 6 equivalents) and Xphos Pd G4 (2.065 mg, 0.0024 mmol, 0.15 equivalents) were added thereto, and the obtained mixture was shaken at 80°C for 22 hours.

Reaction monitoring

After a lapse of 1 hour from the start of reaction, under a nitrogen atmosphere, 10 μL of the suspension of the reaction solution and the resin was sampled, transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in TFE (1000 μL) to prepare an LC sample. The degree of progression of the reaction was measured by LC-MS analysis.

As a result, 25.8% of the starting material ArBr remained, and 71.6% of the product in an acetylene form (B008) was observed.

Then, the reaction was monitored over time (3, 5, 7, and 22 hours). After a lapse of 22 hours, the starting material was consumed, and 98.7% of the acetylene form (B008) was observed.

From these results, it was confirmed that the conditions found in Example 2-3-3 can achieve a high conversion rate through coupling reaction with electron-poor acetylene derivative using a wide range of ArBr on the solid-phase side.

[Formula 314]

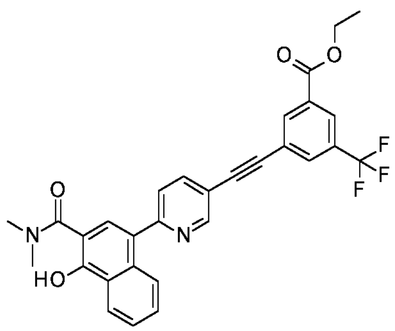

Ethyl 3-[2-[6-[3-(Dimethylcarbamoyl)-4-hydroxynaphthalen-1-yl]pyridin-3- yl]ethynyl]-5-(trifluoromethyl)benzoate: compound B008

LCMS: m/z 533.1[M+H]+.

Retention time: 1.034 min (analysis conditions SMD-FA50-1, 319 nm).

(Retention time of the starting material ArBr: 0.501 min (analysis conditions SMD- FA50-1, 319 nm).

B034-01R whose synthesis method is illustrated in Example 2-3-4 is also referred to as compound AF001-01R in the subsequent examples.

Example 2-4: Experiment to confirm scope of substrate application to reductive amination reaction of aldehyde as typical example of identifying substrate scope that can be applied to library synthesis

Hereinafter, an exemplary method for carrying out reductive amination reaction applicable to library synthesis will be shown, and selection and exploitation examples of a protective group having suitable stability will also be illustrated. The scope given below is a mere illustration of an exemplary identification method, and the present invention is not limited by the scope given below in actual application to library synthesis.

Example 2-4-1: Identification of ester protective group of aminocarboxylic acid under reductive amination reaction conditions using $Ti(OtBu)_4$

An example of finding a problem and identifying a solution thereto in the course of the setting of reductive amination reaction conditions will be shown.

The progression of solid-phase reaction was confirmed using a general reducing agent, acid, and solvent for use in reductive amination reaction. Tetramethylammonium triacetoxyborohydride was selected as the reducing agent. AcOH was selected as the acid. DCE was selected as the solvent. Reductive amination reaction was carried out using aldehyde AC005-01R and amine BB28 as substrates. The aldehyde AC005-01R was synthesized in Example 2-2-6, and the amine BB28 was synthesized in Example 1-4-4. Hereinafter, an exemplary operation will be shown.

[Formula 315]

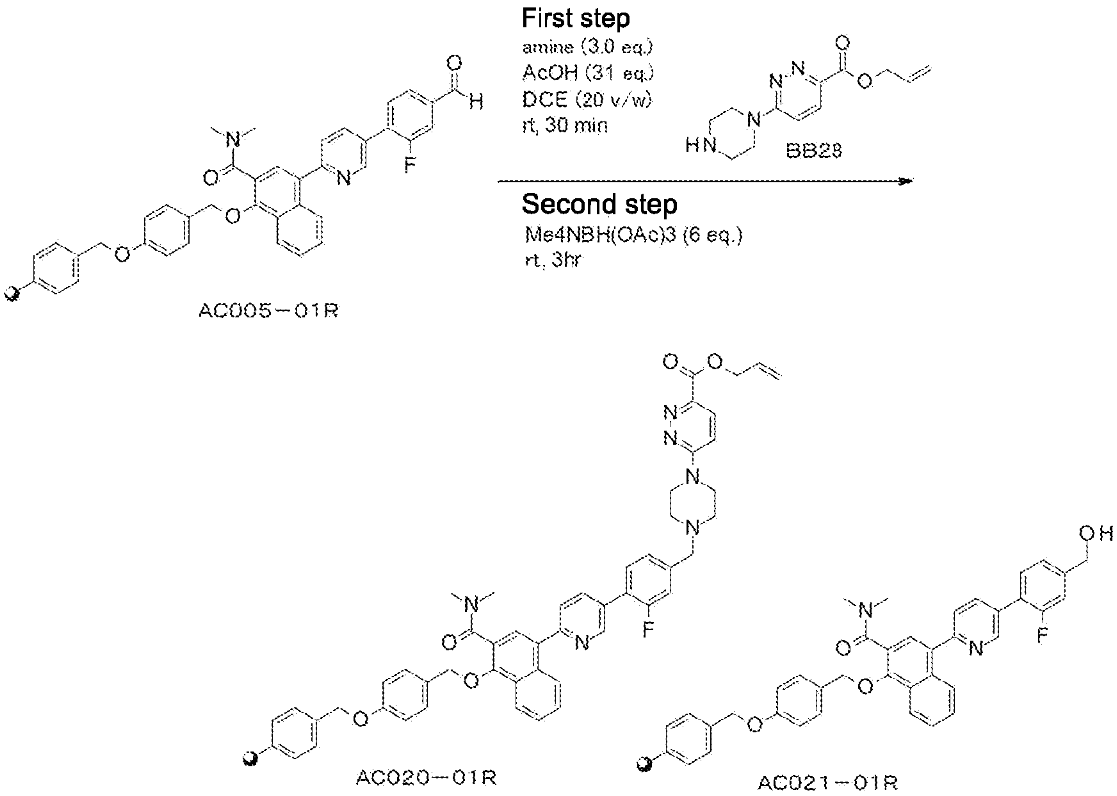


Under a nitrogen atmosphere, AC005-01R (0.34 mmol/g, 10.0 mg, 1.0 equivalent) and DCE (200 μL, 20 v/w) were added to a 0.6 mL screw-cap vial and shaken at room temperature for 1 hour. Under a nitrogen atmosphere, prop-2-enyl 6-piperazin-1- ylpyridazine-3-carboxylate (BB28) (2.5 mg, 3.0 equivalents) and AcOH (6.0 μL, 31 equivalents) were added thereto, and the mixture was shaken at room temperature for 30 minutes. Then, tetramethylammonium triacetoxyborohydride (5.4 mg, 12 equivalents) was added thereto, and the mixture was shaken at room temperature for 3 hours.

The reaction was monitored as follows.

Under a nitrogen atmosphere, 10 μL of the resin suspension was sampled at a given time, then transferred to a pipette tip with a filter, washed three times with NMP (100 μL), three times with MeOH (100 μL), and three times with DCM (100 μL), and then dipped in a 10% solution of TFA in DCM containing 0.2 M trimethylbenzene (100 μL) for 5 minutes. After filtration, the filtrate was dissolved in TFE:toluene (9:1, 500 μL) to prepare an LC sample. The degree of

progression of the reaction was measured by LC-MS analysis. The following compound to be monitored was analyzed under analysis conditions SMD-AC05-1, and the UV area% was confirmed at a UV wavelength of 319 nm.

Compound to be monitored

[Formula 316]

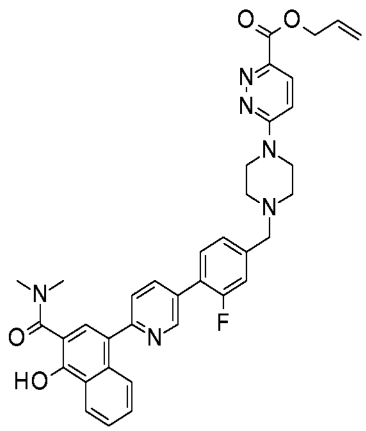

AC020
MS: m/z 647.2[M+H]+
Retention time: 1.28 min
(Retention time of the starting material ArCHO: 1.11 min)

[Formula 317]

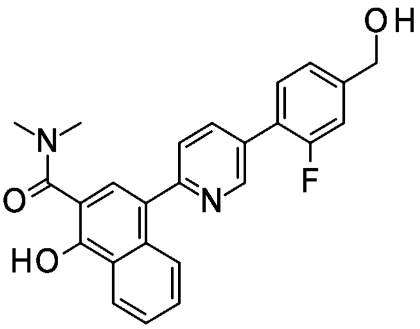

AC021
MS: m/z 417.0[M+H]+.
Retention time: 0.96 min.
(Retention time of the starting material ArCHO: 1.11 min).
After a lapse of 3 hours in the second step, 79 area% of the target compound in reductively aminated form AC20 was confirmed. On the other hand, 14 area% of alcohol form AC021 was confirmed which appeared when aldehyde supposed to be reduced after reaction with amine was directly reduced by the reducing agent. In the presence of aldehyde on the solid phase, side reaction ascribable to direct reduction into an alcohol form markedly reduces the purity of a library. Hence, it is desirable to set reaction conditions that suppress the formation of the by-product alcohol form. The following experiment was carried out in order to solve this challenge.
Reductive amination reaction using aldehyde AC005-01R and aminocarboxylic acid esters, BB28, AC019, or BB29 was carried out with reference to a literature known in the art (J. Med. Chem. 2016, 59, 9837-9854) by selecting sodium triacetoxyborohydride as a reducing agent, $Ti(OtBu)_4$ as a Lewis acid, AcOH as an additive, and DCE as a solvent. Since a protective group stably present during reductive amination reaction was identified as to the ester moiety of aminocarboxylic acid, study results thereabout are shown in Table AC- 01. The following experimental example is

the experimental example of entry 3 in Table AC-01 as one example.

[Formula 318]

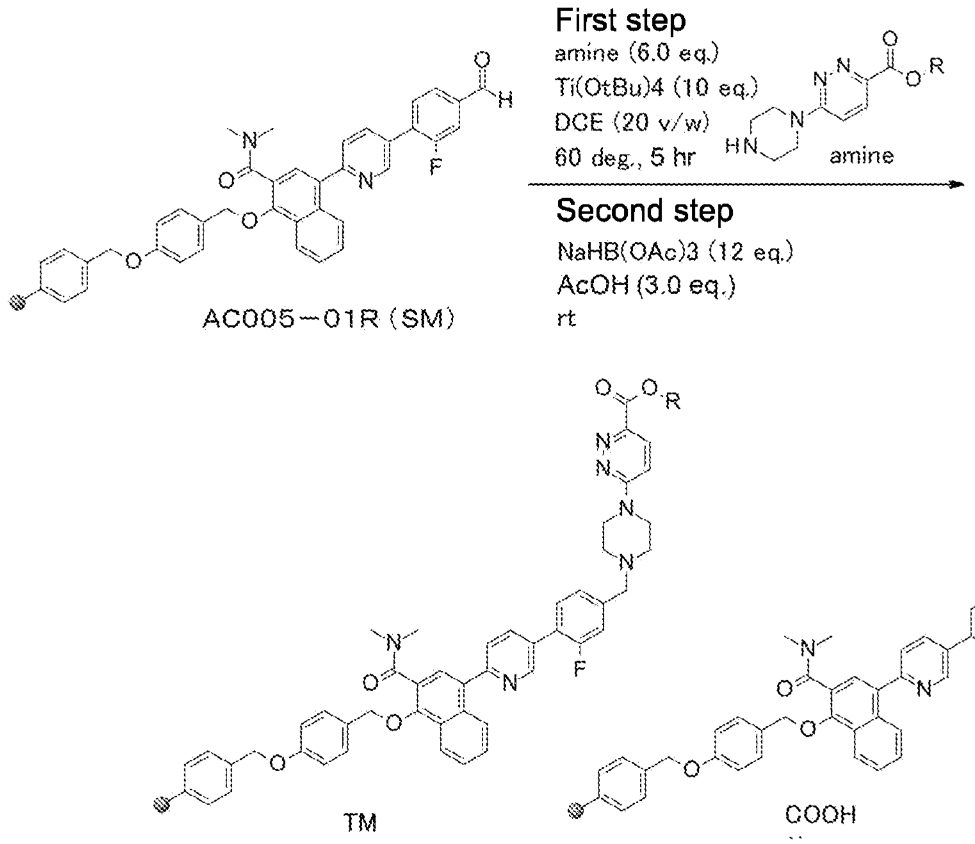


AC005−01R (SM)

TM

COOH

Under a nitrogen atmosphere, solid-phase supported Ar-CHO (AC005-01R) (0.41 mmol/g, 15.0 mg, 1.0 equivalent) and DCE (300 μL, 20 v/w) were added to a 0.6 mL screw-cap vial and shaken at room temperature for 2.5 hours. Under a nitrogen atmosphere, a piperazine derivative (BB29) (9.2 mg, 6.0 equivalents) and $Ti(OtBu)_4$ (23.8 μL, 10 equivalents) were added thereto, and the mixture was shaken at 60°C for 5 hours. Then, sodium triacetoxyborohydride (15.6 mg, 12 equivalents) and AcOH (1.1 μL, 3.0 equivalents) were added thereto, and the mixture was shaken at room temperature for 14 hours.

The reaction was monitored as follows.

Under a nitrogen atmosphere, 10 μL of the resin suspension was sampled at a given time, then transferred to a pipette tip with a filter, washed three times with MeOH (100 μL) and three times with DCM (100 μL), and then dipped in a 10% solution of TFA in DCM containing 0.05 M trimethylbenzene (50 μL) for 5 minutes. After filtration, the filtrate was dissolved in DMI (250 μL), and the solution (50 μL) was diluted with MeCN (250 μL) to prepare an LC sample. The degree of progression of the reaction was measured by LC-MS analysis. Each compound shown in Table AC-02 to be monitored was analyzed under analysis conditions SMD-AC05-1, and the UV area% was confirmed at a UV wavelength of 319 nm.

This reaction may produce a compound (COOH) resulting from the conversion of the ester moiety of TM to COOH, an amide form resulting from the substitution of the ester moiety of TM by the amine used, and an OH form resulting from the direct reduction of starting material aldehyde, in addition to the product (TM) of the reductive amination reaction described above.

[Table 20]

[Table AC-01]

Table AC-01

| entry | R on ester | Second step (hr) | UV area% of compound to be monitored (319 nm) | | | | |
|---|---|---|---|---|---|---|---|
| | | | TM | COOH form (AC022) | SM (AC005) | amide form | OH form (AC021) |
| 1 | Allyl | 14 | 76.4 | 13.3 | ND | 5.0 | ND |
| 2 | iPr | 17 | 94.0 | 1.0 | ND | 0.9 | ND |
| 3 | tBu | 17 | 92.5 | 2.3 | ND | ND | ND |

When aminocarboxylic acid esters protected with various esters were used, it was thus revealed that, in the case of using tBu as an ester protective group (entry 3), the target compound can be obtained at high yields while the formation of an amide form is circumvented. Furthermore, the formation of an OH form can be completely suppressed.

The results of this experiment demonstrated that the approach of the present invention can also circumvent the formation of a by-product OH form in the solid phase by using sodium triacetoxyborohydride as a reducing agent, $Ti(OtBu)_4$ as a Lewis acid, AcOH as an additive, and DCE as a solvent for aldehyde. The results further demonstrated that aminocarboxylic acid ester can be used at high yields in reductive amination by selecting a suitable protective group and thereby circumventing the formation of a by-product amide form.

This example gives an exemplary experiment, but illustrates library synthesis having bond diversity which can be performed at a high purity and a high yield by finding or selecting the optimum protective group.

[Table 21]

[Table AC-02]

| Table AC-02 | | | | | |
|---|---|---|---|---|---|
| entry | Compound classification | Compound No. | Structure of compound to be monitored | MS：m／z [M＋H]＋ | Retention time (min) |
| 1 | TM | ＡＣ０２０ | 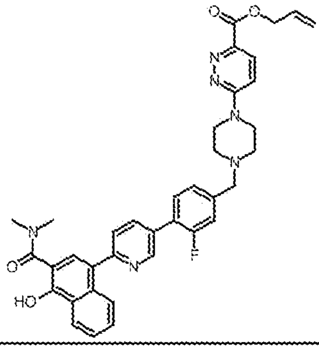 | 647.2 | 1.28 |
| 1 | Amide form | ＡＣ０２３ | 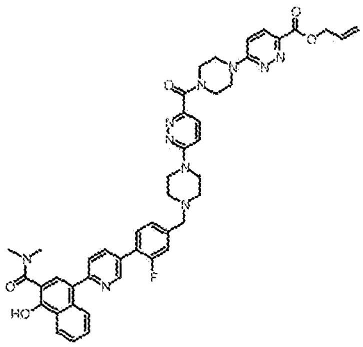 | 837.2 | 1.19 |
| 2 | TM | ＡＣ０２４ | 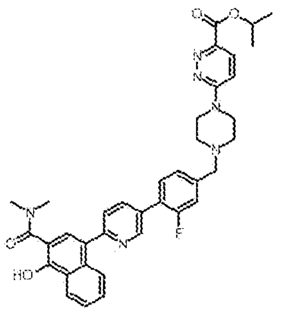 | 649.2 | 1.30 |
| 2 | Amide form | ＡＣ０２５ | 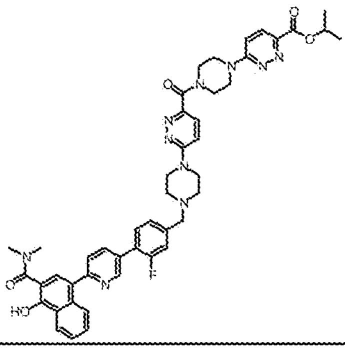 | 840.2 | 1.19 |

[Table 22]

| | | | | | |
|---|---|---|---|---|---|
| 3 | TM | AC026 | 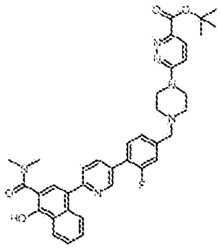 | 663.2 | 1.37 |
| 3 | Amide form | AC027 | 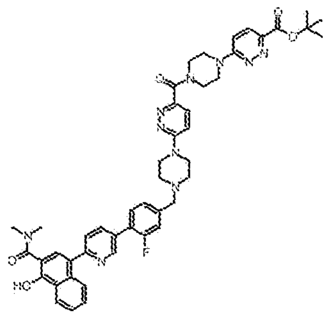 | nd | nd |
| Common | COOH form | AC022 | 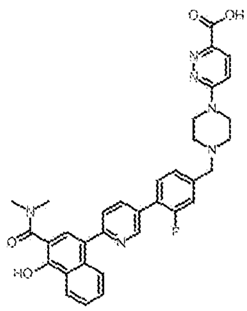 | 607.2 | 0.84 |
| Common | OH form | AC021 | 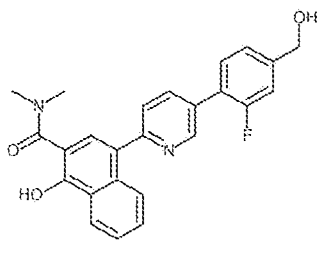 | 417.0 | 0.99 |

Example 2-4-2: Application of reductive amination conditions to substrates having various functional groups in molecule

The reductive amination reaction of aldehydes having various functional groups and differing in reactivity with BB29 was performed to elucidate the scope of substrate application. In the experiment, the compounds shown in Table AC-03 and BB29 were used with reference to the reaction conditions found in Example 2-4-1. Each aldehyde was synthesized in Example 2-2-6 for AC005-01R, Example 1-4-8 for AC004-01R, Example 1-4- 9 for AC006-01R, Example 2-2-8 for AC007-01R, and Example 1-4-7 for AC003-01R.

Hereinafter, an exemplary operation of run 1 in Table AC-03 will be shown as one example.

[Formula 319]

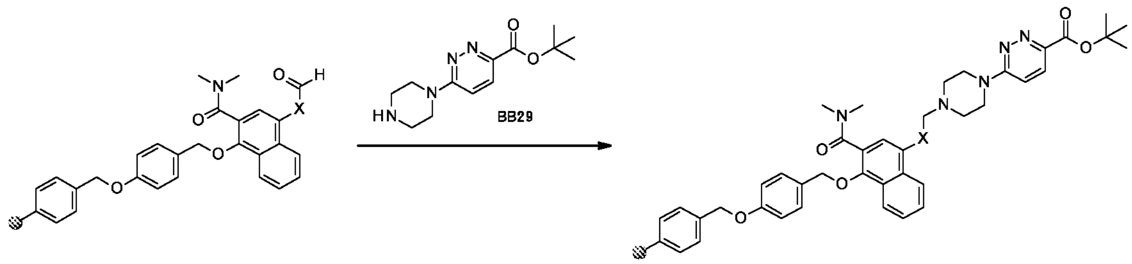


Under a nitrogen atmosphere, solid-phase supported Ar-CHO (AC005-01R) (0.37 mmol/g, 20 mg, 1.0 equivalent) and DCE (300 μL, 15 v/w) were added to a 0.6 mL screw-cap vial and shaken at room temperature for 1 hour. Under a nitrogen atmosphere, a piperazine derivative (BB29) (5.9 mg, 3.0 equivalents) and $Ti(OtBu)_4$ (14.3 μL, 5 equivalents) were added thereto, and the mixture was shaken at 60°C for 1 hour. Then, sodium triacetoxyborohydride (18.8 mg, 12 equivalents) and a 2 v/v% solution of AcOH in DCE (100 μL, 4.7 equivalents) were added thereto, and the mixture was shaken at room temperature for 6 hours.

The reaction was monitored as follows.

Under a nitrogen atmosphere, 10 μL of the resin suspension was sampled, then transferred to a pipette tip with a filter, washed three times with MeOH (100 μL) and three times with DCM (100 μL), and then dipped in a 10% solution of TFA in DCM containing 0.05 M trimethylbenzene (50 μL) for 5 minutes. After filtration, the filtrate was dissolved in DMI (250 μL), and the solution (50 μL) was diluted with MeCN (250 μL) to prepare an LC sample. The degree of progression of the reaction was measured by LC-MS analysis. Each compound shown in Table AC-04 to be monitored was analyzed under analysis conditions SMD-AC05-1, and the UV area% was confirmed at a UV wavelength of 319 nm.

The types of substrates in other runs and results of each experiment are shown in Table AC-03. The structural formulas in the row R in the tables represent the X-CHO moiety in the above formula.

[Table 23]

[Table AC-03]

| Table AC-03 | | | | | | | | | |
|---|---|---|---|---|---|---|---|---|---|
| entry | 1 | | | 2 | | | 3 | | |
| R | 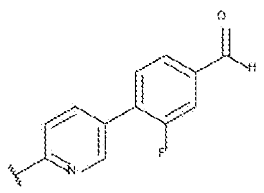 | | | 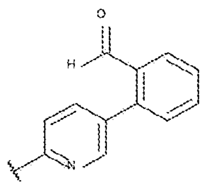 | | | 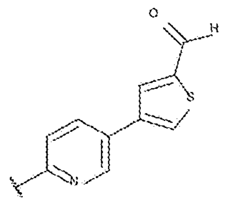 | | |
| Compound No. of substrate used | AC005−01R | | | AC004−01R | | | AC006−01R | | |
| UV area% | TM (AC026) | COOH (AC023) | SM (AC005) | TM (AC029) | COOH (AC030) | SM (AC004) | TM (AC031) | COOH (AC032) | SM (AC006) |
| at 3 hr | 85.7 | 0.7 | nd | 89.8 | 1.7 | nd | 95.4 | 0.8 | 0.2 |
| at 6 hr | 89.1 | 1.0 | nd | 90.6 | 1.3 | nd | 95.8 | 1.2 | 0.3 |
| at 20 hr | 89.5 | 1.2 | nd | 90.3 | 1.0 | nd | 95.6 | 0.9 | 0.1 |
| entry | 4 | | | 5 | | | | | |
| R | 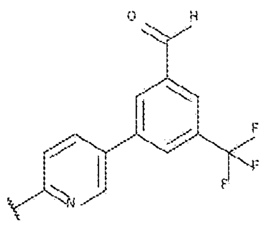 | | | 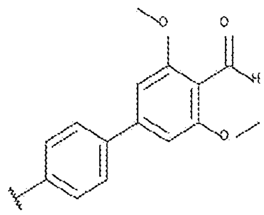 | | | | | |
| Compound No. of substrate used | AC007−01R | | | AC003−01R | | | | | |
| UV area% | TM (AC033) | COOH (AC034) | SM (AC007) | TM (AC035) | COOH (AC036) | SM (AC003) | | | |
| at 3 hr | 93.3 | 1.3 | 0.3 | 91.4 | 1.2 | 0.2 | | | |
| at 6 hr | 94.6 | 1.1 | 0.3 | 91.3 | 1.6 | 0.1 | | | |
| at 20 hr | 93.4 | 0.8 | nd | 91.0 | 1.3 | nd | | | |

In the case of using aldehydes AC003-01R to AC007-01R, the starting material (SM) was consumed in 6 hours for all the substrates, and reaction was completed. 88.9 area% or more of the target compound in a reductively aminated form (TM) was confirmed. No large change was seen in the area% of TM even after a lapse of 20 hours, showing no reduction in purity. From these results, the reaction time was set to 6 hours when BB29 was used as a building block.

[Table 24]

[Table AC-04]

Table AC-04

| entry | Compound classification | Compound No. | Structural formula | MS:m/z [M+H]+ | Retention time (min) |
|---|---|---|---|---|---|
| 1 | TM | AC026 | 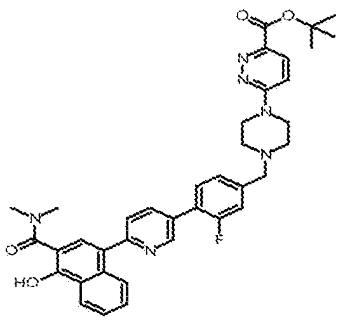 | 663.2 | 1.38 |
| 1 | COOH form | AC023 | 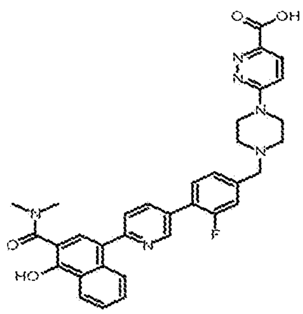 | 607.2 | 0.82 |
| 2 | TM | AC029 | 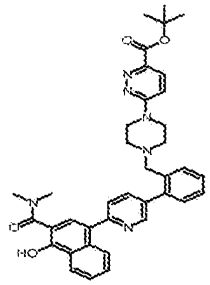 | 645.2 | 1.42 |
| 2 | COOH form | AC030 | 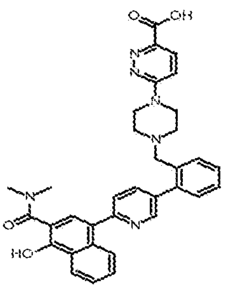 | 589.1 | 0.84 |
| 3 | TM | AC031 | 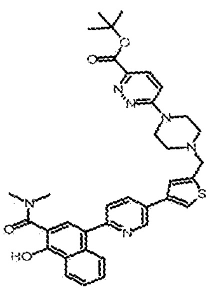 | 651.1 | 1.32 |

[Table 25]

| | | | | | |
|---|---|---|---|---|---|
| 3 | COOH form | AC032 | 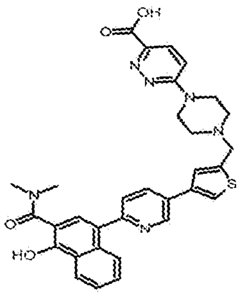 | 595.2 | 0.79 |
| 4 | TM | AC033 | 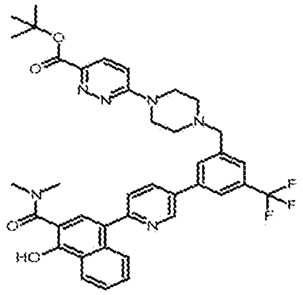 | 713.2 | 1.49 |
| 4 | COOH form | AC034 | 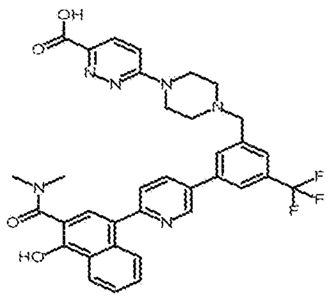 | 657.1 | 0.92 |
| 5 | TM | AC035 | 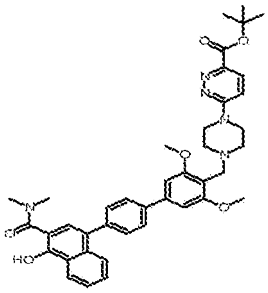 | 704.2 | 1.56 |
| 5 | COOH form | AC036 | 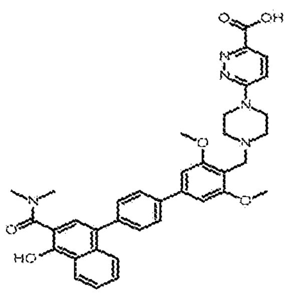 | 648.1 | 0.96 |

The reductive amination reaction of piperazine derivative BB27 with aldehydes having various functional groups

and differing in reactivity was also carried out to elucidate the scope of substrate application. In the experiment, the compounds shown in Table AC-05 and BB27 were used with reference to the reaction conditions found in Example 2-4-1. Each aldehyde was synthesized in Example 1-4-10 for AC008-01R and Example 1-4-11 for AC009-01R.

The case of run 1 in Table AC-05 will be shown as one example.

[Formula 320]

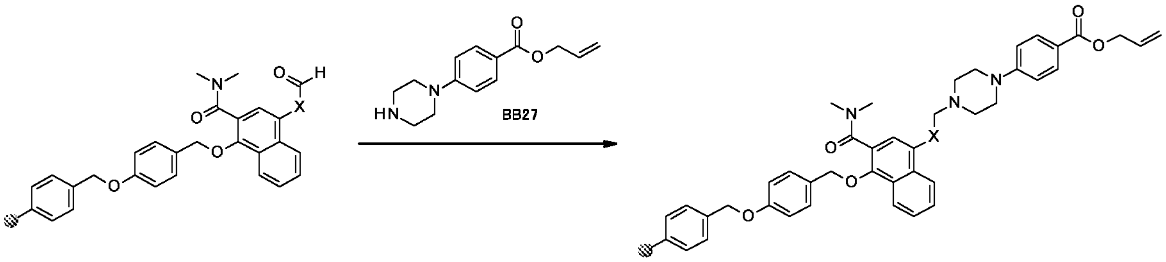


Under a nitrogen atmosphere, solid-phase supported Ar-CHO (AC005-01R) (0.37 mmol/g, 20 mg, 1.0 equivalent) and DCE (300 μL, 15 v/w) were added to a 0.6 mL screw-cap vial and shaken at room temperature for 1 hour. Under a nitrogen atmosphere, a piperazine derivative (BB27) (5.5 mg, 3.0 equivalents) and $Ti(OtBu)_4$ (14.3 μL, 5 equivalents) were added thereto, and the mixture was shaken at 60°C for 1 hour. Then, sodium triacetoxyborohydride (18.8 mg, 12 equivalents) and a 2 v/v% solution of AcOH in DCE (100 μL, 4.7 equivalents) were added thereto, and the mixture was shaken at room temperature for 6 hours.

The reaction was monitored as follows.

Under a nitrogen atmosphere, 10 μL of the resin suspension was sampled, then transferred to a pipette tip with a filter, washed three times with MeOH (100 μL) and three times with DCM (100 μL), and then dipped in a 10% solution of TFA in DCM containing 0.05 M trimethylbenzene (50 μL) for 5 minutes. After filtration, the filtrate was dissolved in DMI (250 μL), and the solution (50 μL) was diluted with MeCN (250 μL) to prepare an LC sample. The degree of progression of the reaction was measured by LC-MS analysis. Each compound shown in Table AC-06 to be monitored was analyzed under analysis conditions SMD-AC05-1, and the UV area% was confirmed at a UV wavelength of 319 nm.

The types of substrates in other runs and results of each experiment are shown in Table AC-05. The structures in the row R in the tables represent the X-CHO moiety in the above formula.

[Table 26]

[Table AC-05]

Table AC-05

| entry | 1 | | | 2 | | | 3 | | |
|---|---|---|---|---|---|---|---|---|---|
| R | 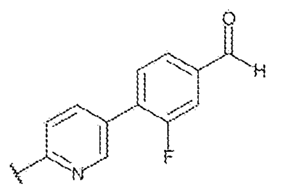 | | | 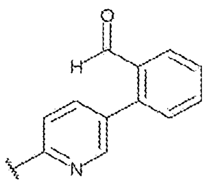 | | | 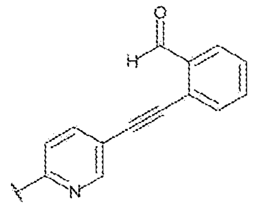 | | |
| Compound No. of substrate used | AC005-01R | | | AC004-01R | | | AC009-01R | | |
| UV area% | TM (AC037) | COOH (AC038) | SM (AC005) | TM (AC039) | COOH (AC040) | SM (AC004) | TM (AC041) | COOH (AC041) | SM (AC009) |
| at 3 hr | 93.9 | 0.4 | 0 | 95.2 | 1.1 | 0 | 98.2 | 0.7 | 0 |
| at 6 hr | 94.2 | 0.6 | 0 | 93.3 | 2.0 | 0 | 96.8 | 1.1 | 0 |
| entry | 4 | | | 5 | | | 6 | | |
| R | 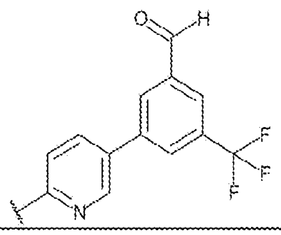 | | | 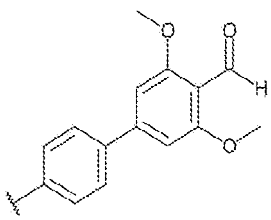 | | | 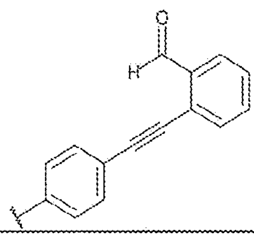 | | |
| Compound No. of substrate used | AC007-01R | | | AC003-01R | | | AC008-01R | | |
| UV area% | TM (AC043) | COOH (AC044) | SM (AC007) | TM (AC045) | COOH (AC046) | SM (AC003) | TM (AC047 | COOH (AC048) | SM (AC003) |
| at 3 hr | 95.6 | 1.6 | 0 | 92.4 | 1.1 | 0 | 98.5 | 1.0 | 0 |
| at 6 hr | 96.1 | 2.2 | 0 | 85.7 | 1.7 | 0 | 97.7 | 1.8 | 0 |

LCMS results obtained after a lapse of 3 and 6 hours are shown in Table AC-05. In the case of using aldehydes AC003-01R to AC005-01R and AC007-01R to AC009-01R, the starting material (SM) was consumed in 3 hours for all the substrates, and reaction was completed. 92.1 area% or more of the target compound in a reductively aminated form (TM) was confirmed. No large change was seen in the area% of TM even after a lapse of 6 hours, showing no reduction in purity. From these results, the reaction time was set to 6 hours when BB27 was used as a building block.

From the results of this experiment, conditions were found under which reaction is allowed to progress at high yields while the stability of an ester moiety is ensured, by using sodium triacetoxyborohydride as a reducing agent, $Ti(OtBu)_4$ as a Lewis acid, AcOH as an additive, and DCE as a solvent and thereby circumventing the formation of a by-product OH form. Also, the reductive amination reaction by the approach of the present invention was found to have a practical level applicable to library synthesis using aldehydes having various functional groups even through solid-phase reaction.

[Table 27]

[Table AC-06]

Table AC-06

| entry | Compound classification | Compound No. | | MS: m/z [M+H]+ | Retention time (min) |
|---|---|---|---|---|---|
| 1 | TM | AC037 | 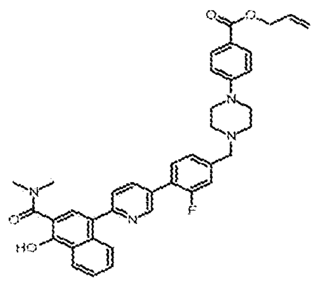 | 645.2 | 1.62 |
| 1 | COOH form | AC038 | 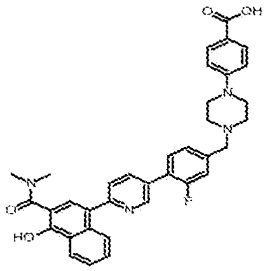 | 605.2 | 0.91 |
| 2 | TM | AC039 | 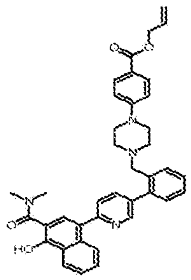 | 627.2 | 1.66 |
| 2 | COOH form | AC040 | 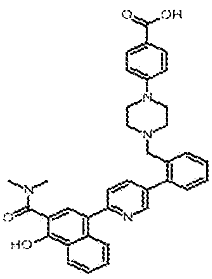 | 587.2 | 0.93 |
| 3 | TM | AC041 | 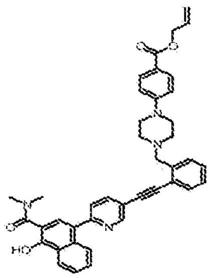 | 651.2 | 1.74 |

[Table 28]

| | | | | | |
|---|---|---|---|---|---|
| 3 | COOH form | AC042 | 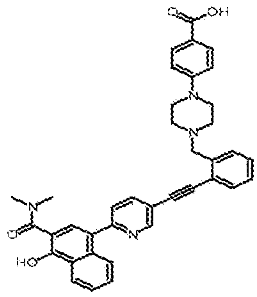 | 611.2 | 1.00 |
| 4 | TM | AC043 | 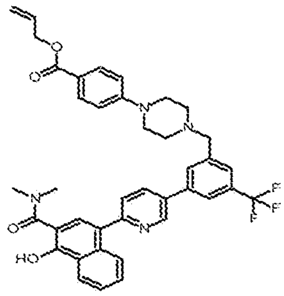 | 695.2 | 1.70 |
| 4 | COOH form | AC044 | 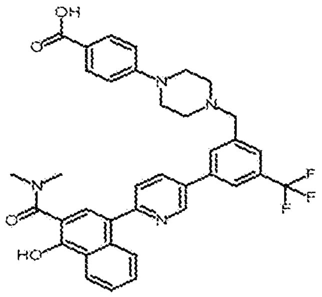 | 655.1 | 1.01 |
| 5 | TM | AC045 | 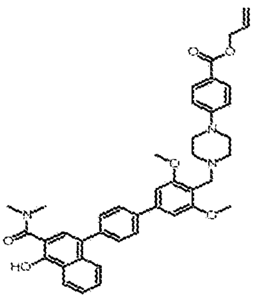 | 686.2 | 1.75 |
| 5 | COOH form | AC046 | 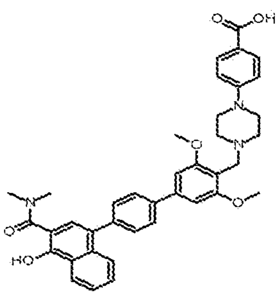 | 646.1 | 1.03 |

[Table 29]

| | | | | | |
|---|---|---|---|---|---|
| 6 | TM | AC047 | 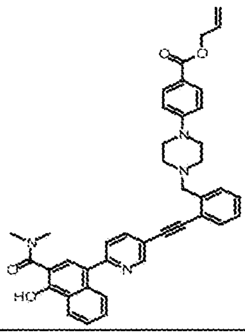 | 650.2 | 1.89 |
| 6 | COOH form | AC048 | 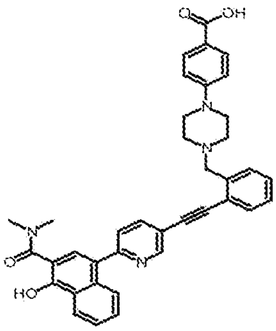 | 610.2 | 1.17 |

Example 2-4-3: Deprotection experiment of ester protective group of aminocarboxylic acid

The confirmation of a deprotection method is also important, as a matter of course, for selecting a protective group. Particularly, deprotection with high functional group tolerance maintained in the presence of a plurality of functional groups in a plurality of substrates is desirable for library synthesis. In this example, it is essential for ensuring the purity of a library that the deprotection reaction of tBu ester which is bulky ester progresses at high yields without influencing other functional groups.

The deprotection of tBu ester was carried out for the compounds shown in Table AC-07. Hereinafter, the case of run 1 will be shown as one example.

[Formula 321]

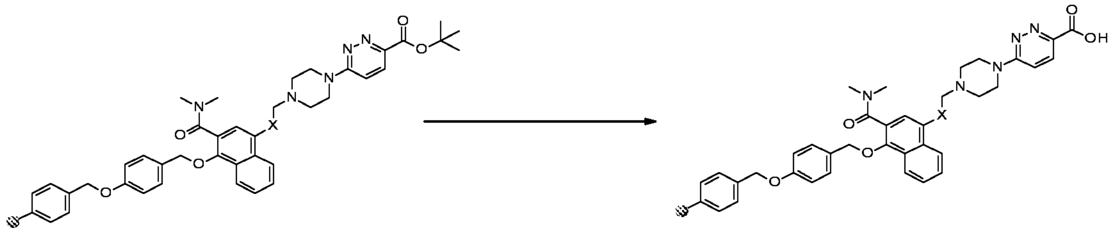

Under a nitrogen atmosphere, a solid-phase supported tBu ester compound (AC029- 01R) (0.37 mmol/g, 16 mg), THF (240 μL, 15 v/w), and MeOH (24 μL, 1.5 v/w) were added to a 0.6 mL screw-cap vial and shaken at room temperature for 1 hour. Under a nitrogen atmosphere, an aqueous LiOH solution (2 M, 22 μL) was added thereto, and the mixture was shaken at room temperature for 22 hours.

The reaction was monitored as follows.

Under a nitrogen atmosphere, 10 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.05 M trimethylbenzene (50 μL) for 5 minutes. After filtration, the filtrate was dissolved in DMI (250 μL), and the solution (50 μL) was diluted with MeCN (250 μL) to prepare an LC sample. The degree of progression of the reaction was measured by LC-MS analysis. Each compound shown in Table AC-08 to be monitored was analyzed under analysis conditions SMD-FA05-1, and the UV area% was confirmed

at a UV wavelength of 319 nm.

X in Table AC-07 represents that the lower left linking site is bonded to naphthol and the other linking site is bonded to an aminomethyl group.

[Table 30]

[Table AC-07]

Table AC-07

| entry | 1 | | 2 | | 3 | |
|---|---|---|---|---|---|---|
| X | 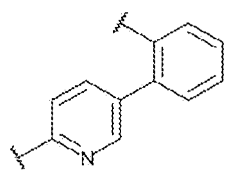 | | 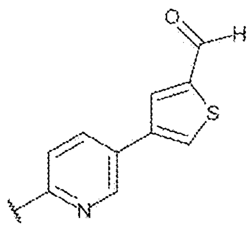 | | 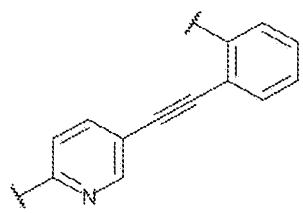 | |
| Compound No. of substrate used | AC029-01R | | AC031-01R | | AC049-01R | |
| UV area% | TM (AC030) | SM (AC029) | TM (AC032) | SM (AC031) | TM (AC051) | SM (AC049) |
| at 8 hr | 95.5 | nd | 94.2 | nd | 96.5 | nd |
| at 20 hr | 95.3 | nd | 90.9 | nd | 95.7 | nd |

| entry | 4 | | 5 | |
|---|---|---|---|---|
| X | 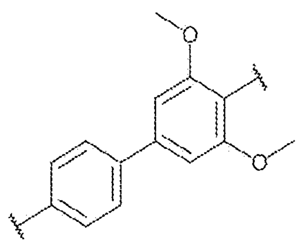 | | 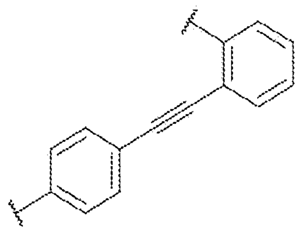 | |
| Compound No. of substrate used | AC035-01R | | AC050-01R | |
| UV area% | TM (AC036) | SM (AC035) | TM (AC052) | SM (AC050) |
| at 8 hr | 95.5 | nd | 94.1 | nd |
| at 20 hr | 94.6 | nd | 96.6 | nd |

As shown in Table AC-07, it was able to be confirmed that the starting material is consumed by shaking at room temperature for 8 hours so that tBu ester can be deprotected at a purity of 90.9 area% or more without markedly reducing purity even after a lapse of 20 hours. These results demonstrated that tBu ester can also be deprotected at high yields and can be used without impairing the purity of a library.

The results described above demonstrated that tBu ester can be deprotected with high functional group tolerance maintained in the presence of various functional groups. Stability during bond formation is obtained at a desired level, and easy progression of deprotection is obtained at the same level. For this purpose, a method for tuning protective groups will be illustrated.

[Table 31]

[Table AC-08]

Table AC-08

| entry | Compound No. | Structure of compound to be monitored | MS:m/z [M+H]+ | Retention time (min) |
|---|---|---|---|---|
| 1 | AC030 | 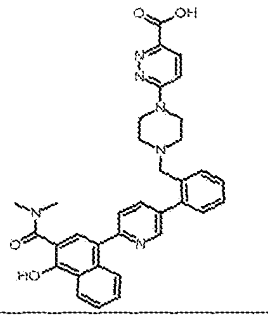 | 589.2 | 0.70 |
| 2 | AC032 | 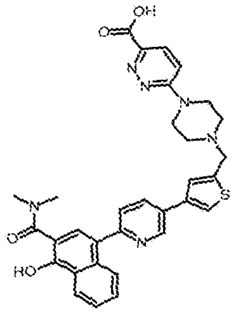 | 595.2 | 0.66 |
| 3 | AC051 | 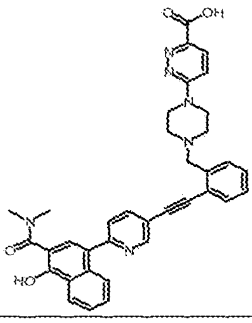 | 613.2 | 0.78 |
| 4 | AC036 | 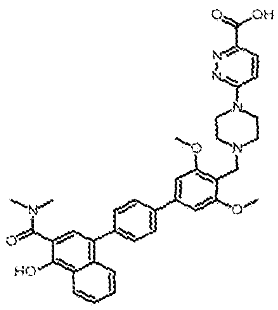 | 648.3 | 0.86 |
| 5 | AC052 | 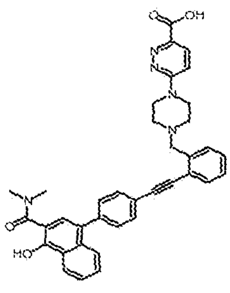 | 612.2 | 0.88 |

The starting materials shown in Table AC-07 and compounds AC049-01R and AC050-01R were synthesized from AC009-01R or AC008-01R by the following exemplary method.

[Formula 322]

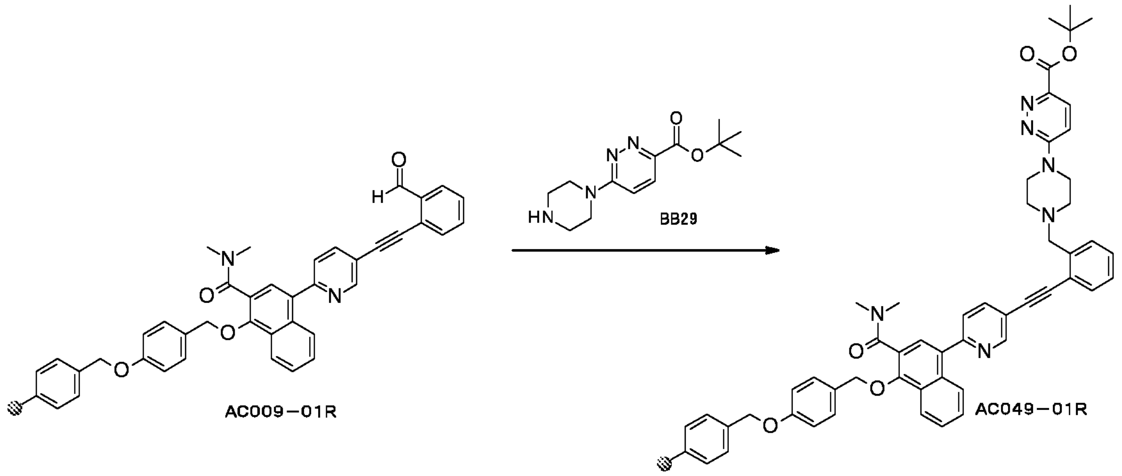


AC009-01R

AC049-01R

Under a nitrogen atmosphere, solid-phase supported Ar-CHO (AC009-01R) (0.37 mmol/g, 20 mg) and DCE (400 μL, 20 v/w) were added to a 0.6 mL screw-cap vial and shaken at room temperature for 1 hour. Under a nitrogen atmosphere, a piperazine derivative (BB29) (11.7 mg, 6.0 equivalents) and $Ti(OtBu)_4$ (28.6 μL, 10 equivalents) were added thereto, and the mixture was shaken at 60°C for 1 hour. Then, $NaBH(OAc)_3$ (18.8 mg, 12 equivalents) and AcOH (1.3 μL, 3.0 equivalents) were added thereto, and the mixture was shaken at room temperature for 20 hours. MeOH (1 mL) was added to the reaction solution, and then, the resin suspension was transferred to a syringe with a filter using MeOH, repetitively washed three times with MeOH (400 μL), three times with NMP (400 μL), three times with a $NaHCO_3$ solution (0.15 M, NMP-$H_2O$ 1:5 solution, 400 μL), three times with water (400 μL), three times with MeOH (400 μL), and three times with DCM (400 μL), and then dried under reduced pressure to obtain the title mixture (AC049-01R).

The reaction was monitored as follows.

In a glove bag purged with nitrogen, 10 μL of the resin suspension was sampled, then transferred to a pipette tip with a filter, repetitively washed three times with MeOH (100 μL) and three times with DCM (100 μL), and then dipped in a 10% TFA in DCM containing 0.05 M trimethylbenzene (cleaving solution) (50 μL) for 5 minutes. The cleaving solution was filtered. The filtrate was dissolved in NMP (250 μL), and the solution (50 μL) was diluted with MeCN (250 μL) to prepare an LC sample. The degree of progression of the reaction was measured by LC-MS analysis. The following compound to be monitored was analyzed under analysis conditions SMD-FA05-1, and the UV area% was confirmed at a UV wavelength of 319 nm.

Compound to be monitored

[Formula 323]

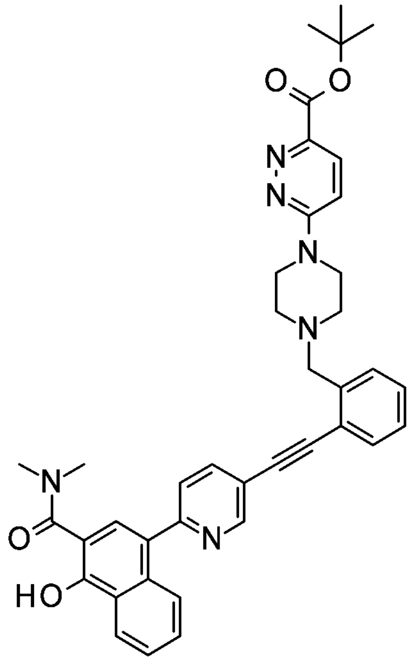

AC049

MS: m/z 669.3[M+H]+.

Retention time: 0.92 min.

AC050-01R was synthesized from AC008-01R by the method described above. The following compound to be monitored was analyzed under analysis conditions SMD-FA05-1, and the UV area% was confirmed at a UV wavelength of 319 nm.

[Formula 324]

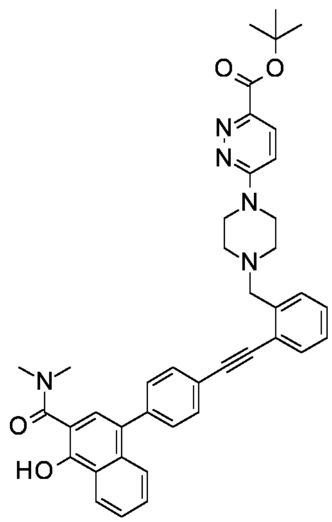

AC050

MS: m/z 668.4[M+H]+.

Retention time: 1.02 min.

Example 2-4-4: Solid-phase experiment on reductive amination reaction of NHR1R2 with aldehyde

The reductive amination reaction of solid-phase supported amine with aldehyde was carried out under conditions involving selecting $NaBH(OAc)_3$ as a reducing agent and adding acetic acid in NMP.

Example 2-4-4-1: Reductive amination reaction of solid-phase supported amine (H001-03R) with H201 (synthesis of H101-03R)

[Formula 325]

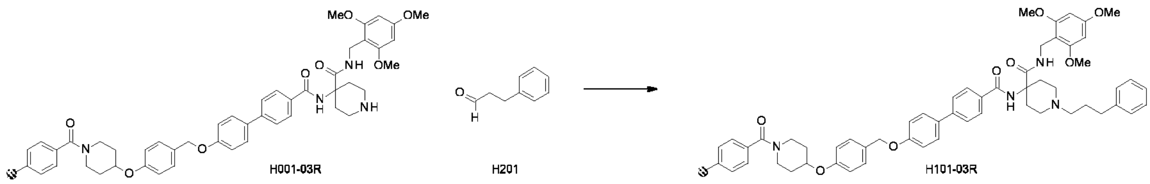

Under a nitrogen atmosphere, solid-phase supported amine (H001-03R) (loading rate: 0.188 mmol/g, 20 mg, 0.0038 mmol), 3-phenylpropanal (H201) (9.91 µL, 0.075 mmol), acetic acid (9.0 µL, 0.157 mmol), and NMP (0.4 mL) were added to a 0.6 mL glass vial and shaken at room temperature for 1 hour. Under a nitrogen atmosphere, $NaBH(OAc)_3$ (17.53 mg, 0.083 mmol) was added thereto, and the mixture was shaken at room temperature for 2 hours and then shaken at 60°C for 1.5 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 96% of compound H101 of interest was observed.

[Formula 326]

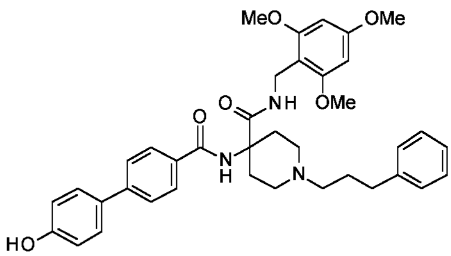

Compound H101

LRMS: m/z 638$[M+H]^+$

Retention time: 0.839 min (analysis conditions SMD-FA05-1, 299 nm)

Example 2-4-4-2: Reductive amination reaction of solid-phase supported amine (H002-03R) with H201 (synthesis of H102-03R)

[Formula 327]

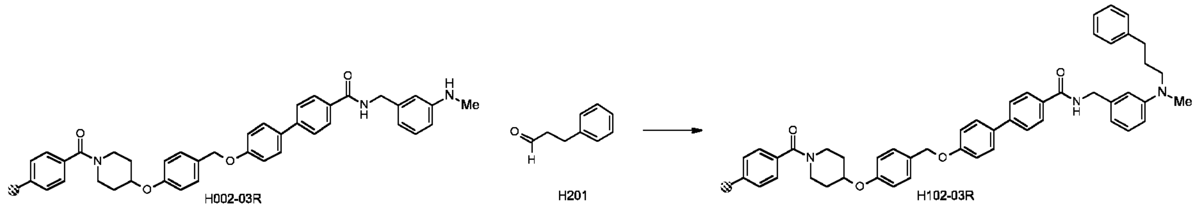

Under a nitrogen atmosphere, solid-phase supported amine (H002-03R) (loading rate: 0.188 mmol/g, 20 mg, 0.0038 mmol), 3-phenylpropanal (H201) (5.17 μL, 0.039 mmol), acetic acid (9.0 μL, 0.157 mmol), and NMP (0.4 mL) were added to a 0.6 mL glass vial and shaken at room temperature for 1 hour. Under a nitrogen atmosphere, $NaBH(OAc)_3$ (9.97 mg, 0.047 mmol) was added thereto, and the mixture was shaken at 60°C for 2 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 98% of compound H102 of interest was observed.

[Formula 328]

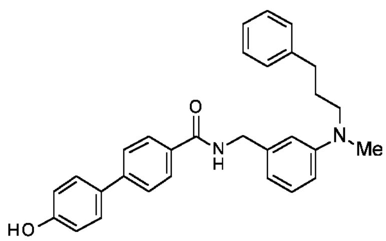

Compound H102

LRMS: m/z 451[M+H]+

Retention time: 1.056 min (analysis conditions SMD-FA05-1, 299 nm)

Example 2-4-4-3: Reductive amination reaction of solid-phase supported amine (H001-03R) with H202 (synthesis of H103-03R)

[Formula 329]

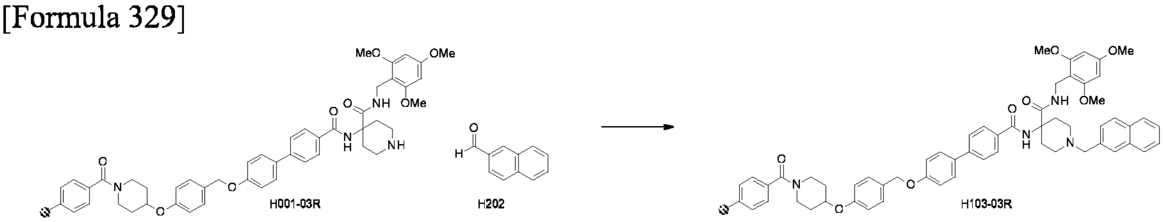

Under a nitrogen atmosphere, solid-phase supported amine (H001-03R) (loading rate: 0.188 mmol/g, 20 mg, 0.0038 mmol), naphthalene-2-carbaldehyde (H202) (11.8 mg, 0.075 mmol), acetic acid (9.0 μL, 0.157 mmol), and NMP (0.4 mL) were added to a 0.6 mL glass vial and shaken at room temperature for 1 hour. Under a nitrogen atmosphere, $NaBH(OAc)_3$ (17.53 mg, 0.083 mmol) was added thereto, and the mixture was shaken at 60°C for 2 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with DMA (0.05 mL), and MeCN (0.25 mL) was added to the filtrate to prepare an LC sample. The degree of

progression of the reaction was measured by LC-MS analysis. As a result, 89% of compound H103 of interest was observed.

[Formula 330]

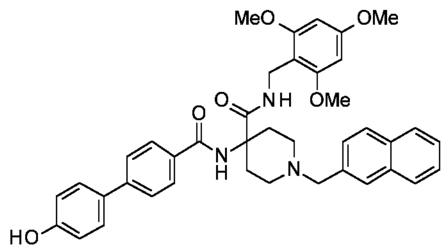

Compound H103

LRMS: m/z 660[M+H]+

Retention time: 0.848 min (analysis conditions SMD-FA05-1, 299 nm)

Example 2-4-4-4: Reductive amination reaction of solid-phase supported amine (H001-03R) with H203 (synthesis of H104-03R)

[Formula 331]

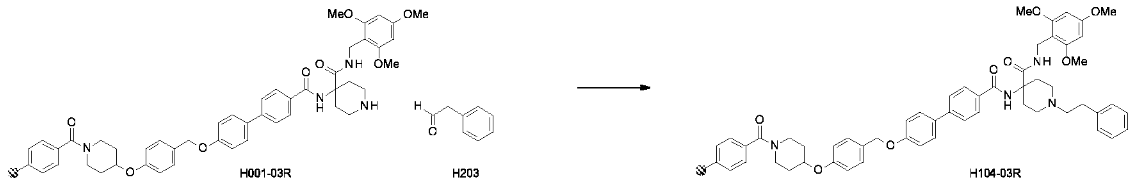

Under a nitrogen atmosphere, solid-phase supported amine (H001-03R) (loading rate: 0.188 mmol/g, 20 mg, 0.0038 mmol), 2-phenylacetaldehyde (H203) (21.1 μL, 40 w% solution in diethyl phthalate, 0.075 mmol), acetic acid (9.0 μL, 0.157 mmol), and NMP (0.4 mL) were added to a 0.6 mL glass vial and shaken at room temperature for 1 hour. Under a nitrogen atmosphere, $NaBH(OAc)_3$ (17.53 mg, 0.083 mmol) was added thereto, and the mixture was shaken at 60°C for 2 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 92% of compound H104 of interest was observed.

[Formula 332]

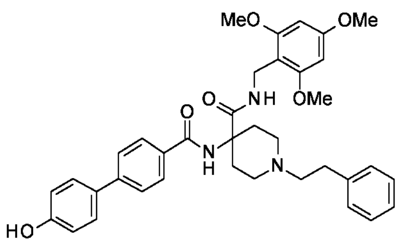

Compound H104

LRMS: m/z 624[M+H]+

Retention time: 0.817 min (analysis conditions SMD-FA05-1, 299 nm)

From these Examples 2-4-4-1 to 2-4-4-4, it was confirmed that this condition can be applied to aniline or secondary alkylamine on the amino group side and benzaldehyde type or alkylaldehyde on the aldehyde side, and enables a C-N bond to be constructed. This approach is applicable without being limited by the substrate scope given here.

Example 2-5: Experiment to confirm scope of substrate application to reductive amination reaction of ketone as typical example of identifying substrate scope that can be applied to library synthesis

Hereinafter, an exemplary method for carrying out the reductive amination reaction of ketone applicable to library synthesis will be shown, and selection and exploitation examples of a protective group having suitable stability will also be illustrated. The scope given below is a mere illustration of an exemplary identification method, and the present invention is not limited by the scope given below in actual application to library synthesis.

Example 2-5-1: Reductive amination reaction of Ar-$COCH_3$ with 4-(piperazin-1- yl)benzoic acid ester: solid-phase experiment using $Ti(OiPr)_4$ as activator

$Ti(OiPr)_4$ was selected as an activator for ketone with reference to a literature known in the art (J. Med. Chem. 2007, 50, 3528). An ether solvent Me-THP was selected as a reaction solvent in order to carry out heating reaction at a high temperature. The reductive amination reaction of solid-phase aryl ketone with piperidine was carried out.

Example 2-5-1-1: Reductive amination reaction (exemplary reaction operation) of solid-phase supported $ArCOCH_3$ (B013-01R) with ethyl 4-(piperazin-1-yl)benzoate (B010)

[Formula 333]

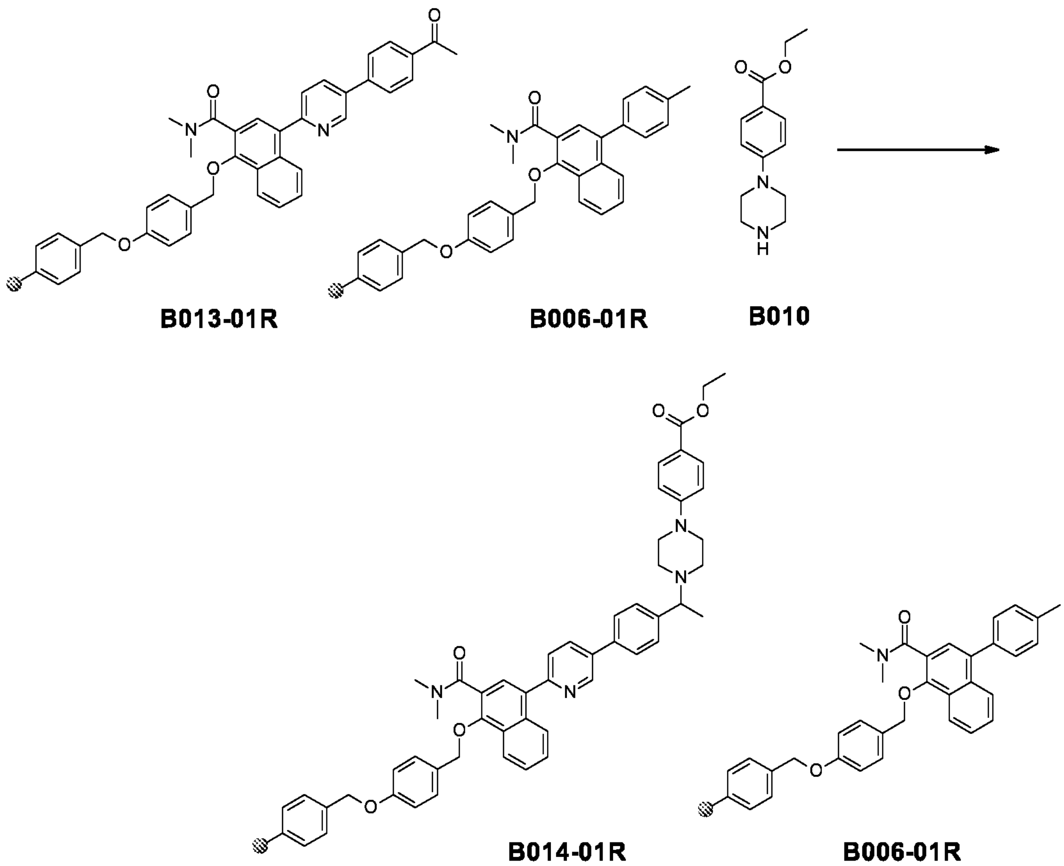

B013-01R B006-01R B010

B014-01R B006-01R

Under a nitrogen atmosphere, solid-phase supported aryl ketone B013-01R (0.35 mmol/g, 20.0 mg, 7.0 μmol, 1.0 equivalent), solid-phase supported reagent B006-01R (added as an internal standard reagent, 0.361 mg, 0.1 equivalents), and 4-MeTHP (400 μL) were added to a 1.5 mL screw-cap vial and shaken at room temperature for 1 hour. Under a nitrogen atmosphere, ethyl 4-(piperazin-1-yl)benzoate (B010) (9.84 mg, 0.042 mmol, 6 equivalents) and $Ti(OiPr)_4$

(20.51 μL, 0.070 mmol, 10 equivalents) were added thereto, and the obtained mixture was shaken at 100°C for 22 hours.

The reaction solution was cooled to room temperature. Then, under a nitrogen atmosphere, sodium triacetoxyborohydride (17.80 mg, 0.084 mmol, 12 equivalents) was added thereto, and the mixture was shaken at 100°C for 15 hours.

Reaction monitoring

After a lapse of 15 hours from the addition of sodium triacetoxyborohydride, under a nitrogen atmosphere, 10 μL of the suspension of the reaction solution and the resin was sampled, transferred to a pipette tip with a filter, washed three times with NMP (100 μL), three times with MeOH (100 μL), and three times with DCM (100 μL), and then dipped in a 10% solution of TFA in DCM (100 μL) for 5 minutes. After filtration, the filtrate was dissolved in TFE (100 μL) to prepare an LC sample. The degree of progression of the reaction was measured by LC-MS analysis.

As a result, 23% of the starting material ketone (B006) remained, and 11% and 48% of the product in an ethyl ester form (B014) and an isopropyl ester form (B015), respectively, were observed.

The isopropyl ester form was presumably derived from $Ti(OiPr)_4$ used as a reagent, and formed by the progression of transesterification.

The need to study reaction conditions that reduced the amount of the starting material ketone remaining (achievement of a high conversion rate through reaction) and hindered the progression of transesterification (reduction in undesirable side reaction) was able to be confirmed for preparing mixture library at a high purity.

[Formula 334]

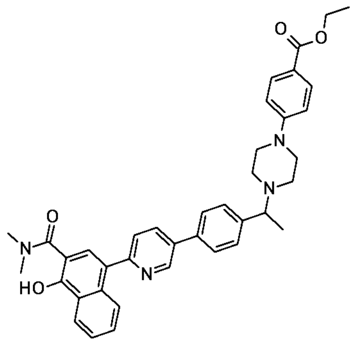

Ethyl 4-[4-[1-[4-[6-[3-(dimethylcarbamoyl)-4-hydroxynaphthalen-1-yl]pyridin-3- yl]phenyl]ethyl]piperazin-1-yl]benzoate: compound B014

LCMS: m/z 629.2[M+H]+.

Retention time: 1.594 min (analysis conditions SMD-FA05-1,319 nm).

(Retention time of the starting material ketone: 1.106 min (analysis conditions SMD-FA05-1, 319 nm).

[Formula 335]

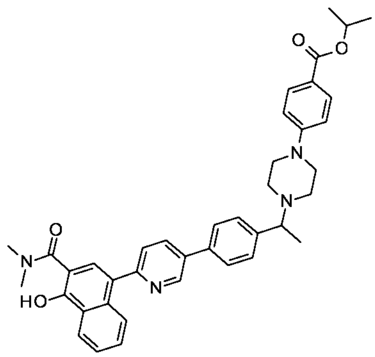

Propan-2-yl 4-[4-[1-[4-[6-[3-(dimethylcarbamoyl)-4-hydroxynaphthalen-1- yl]pyridin-3-yl]phenyl]ethyl]piperazin-1-yl]benzoate: compound B015

LCMS: m/z 643.2[M+H]+.

Retention time: 1.663 min (analysis conditions SMD-FA05-1, 319 nm).

Example 2-5-2: Reductive amination reaction of solid-phase supported $ArCOCH_3$ (B013-01R) with ethyl 4-(piperazin-1-yl)benzoate (B010) (change of Ti reagent)

The reductive amination reaction of solid-phase supported ketone (B013-01R) with ethyl 4-(piperazin-1-yl)benzoate (B010) was carried out by changing the activator to $Ti(OtBu)_4$ for the purpose of reducing transesterification

[Formula 336]

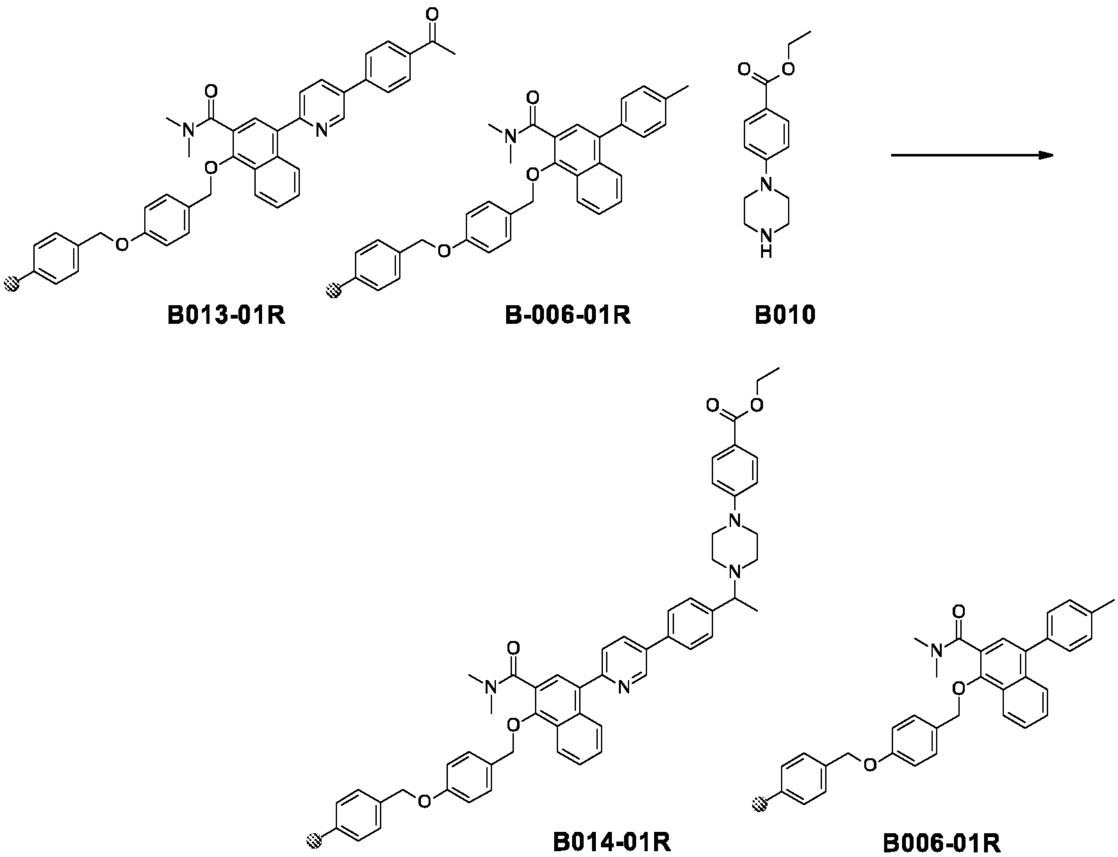

B013-01R B-006-01R B010

B014-01R B006-01R

Under a nitrogen atmosphere, solid-phase supported aryl ketone B013-01R (0.35 mmol/g, 20.0 mg, 7.0 μmol, 1.0 equivalent), solid-phase supported reagent B006-01R (added as an internal standard reagent, 0.361 mg, 0.1 equivalents), and 4-MeTHP (400 μL) were added to a 1.5 mL screw-cap vial and shaken at room temperature for 1 hour. Under a nitrogen atmosphere, ethyl 4-(piperazin-1-yl)benzoate (B010) (9.84 mg, 0.042 mmol, 6 equivalents) and $Ti(OtBu)_4$ (27.0 μL, 0.070 mmol, 10 equivalents) were added thereto, and the obtained mixture was shaken at 100°C for 22 hours.

The reaction solution was cooled to room temperature. Then, under a nitrogen atmosphere, sodium triacetoxyborohydride (17.80 mg, 0.084 mmol, 12 equivalents) was added thereto, and the mixture was shaken at 100°C for 15 hours.

Reaction monitoring

After a lapse of 15 hours from the addition of sodium triacetoxyborohydride, under a nitrogen atmosphere, 10

μL of the suspension of the reaction solution and the resin was sampled, transferred to a pipette tip with a filter, washed three times with NMP (100 μL), three times with MeOH (100 μL), and three times with DCM (100 μL), and then dipped in a 10% solution of TFA in DCM (100 μL) for 5 minutes. After filtration, the filtrate was dissolved in TFE (100 μL) to prepare an LC sample. The degree of progression of the reaction was measured by LC-MS analysis.

As a result, the amount of the starting material ketone (B013) remaining was reduced to 7%, and the amount of the product in an ethyl ester form (B014) was increased to 74%. Use of $Ti(OtBu)_4$ instead of $Ti(OiPr)_4$ was able to circumvent the formation of a transesterified form such as the isopropyl ester form seen in Example 2-5-1. Use of $Ti(OtBu)_4$ as an activator was confirmed to be effective for improvement in the conversion rate. On the other hand, 11% of alcohol (B016), a reduced form of ketone, was observed.

[Formula 337]

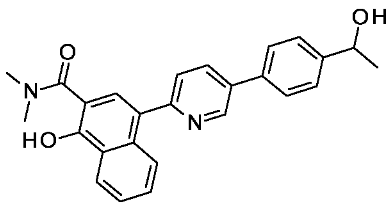

1 -Hydroxy-4- [5-[4-(1-hydroxyethyl)phenyl]pyridin-2-yl]-N,N- dimethylnaphthalene-2-carboxamide: B016

LCMS: m/z 413.1$[M+H]^+$.

Retention time: 0.810 min (analysis conditions SMD-TFA05-1, 319 nm).

Example 2-5-3: Reductive amination reaction of solid-phase supported $ArCOCH_3$ (B013-01R) with ethyl 4-(piperazin-1-yl)benzoate (B010) (change of reaction solvent and reaction temperature)

Reductive amination reaction was carried out by selecting anisole as a reaction solvent for the purpose of further improving the conversion rate, and setting a reaction temperature to 120°C in order to elevate the reaction temperature.

[Formula 338]

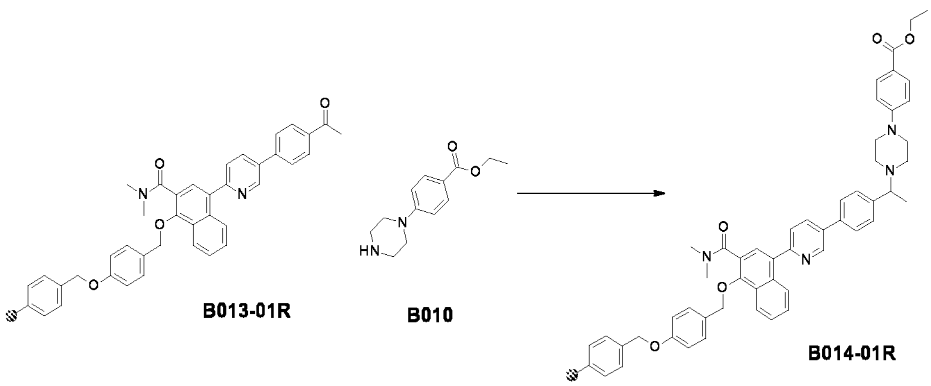

Under a nitrogen atmosphere, solid-phase supported aryl ketone B013-01R (0.35 mmol/g, 20.0 mg, 7.0 μmol, 1.0 equivalent) and anisole (375 μL) were added to a 0.6 mL screw-cap vial and shaken at room temperature for 1 hour. Under a nitrogen atmosphere, ethyl 4-(piperazin-1-yl)benzoate (B010) (6.75 mg, 0.029 mmol, 6 equivalents) and $Ti(OtBu)_4$ (18.54 μL, 0.048 mmol, 10 equivalents) were added thereto, and the obtained mixture was shaken at 120°C for 22 hours.

The reaction solution was cooled to room temperature. Then, under a nitrogen atmosphere, sodium triacetoxyborohydride (12.2 mg, 0.058 mmol, 12 equivalents) was added thereto, and the mixture was shaken at 120°C for 15 hours.

Reaction monitoring

After a lapse of 15 hours from the addition of sodium triacetoxyborohydride, measurement was performed in the same manner as in Example 2-5-2.

As a result, the amount of the starting material ketone (B013) remaining was reduced to 3%, and the amount of the product in an ethyl ester form (B014) was increased to 84%. Also, 12% of alcohol (B016), a reduced form of ketone, was observed. Although not wishing to be bound by any theory, this revealed insufficient iminium formation at the first step.

Example 2-5-4: Reductive amination reaction of solid-phase supported $ArCOCH_3$ (B013-01R) with allyl 4-(piperazin-1-yl)benzoate (BB27) (change to allyl ester)

Next, reductive amination was carried out using allyl 4-(piperazin-1-yl)benzoate (BB27).

[Formula 339]

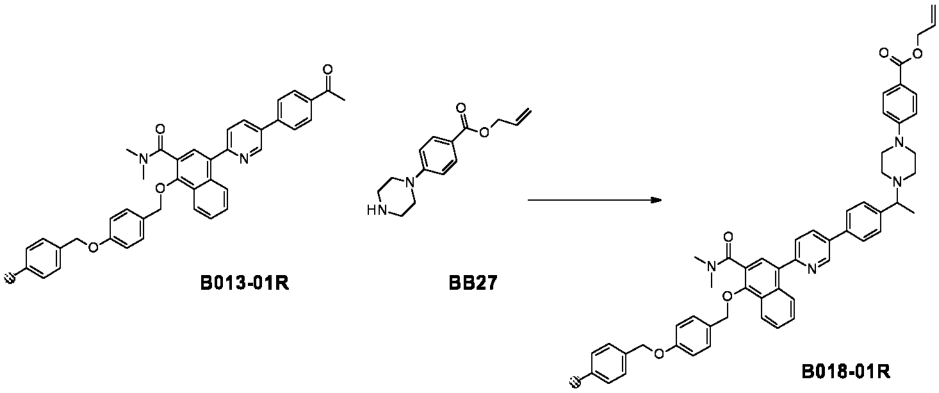

B013-01R BB27 B018-01R

Under a nitrogen atmosphere, solid-phase supported aryl ketone B013-01R (0.32 mmol/g, 15.0 mg, 4.8 μmol, 1.0 equivalent) and anisole (375 μL) were added to a 0.6 mL screw-cap vial and shaken at room temperature for 1 hour. Under a nitrogen atmosphere, allyl 4-(piperazin-1-yl)benzoate (BB27) (7.09 mg, 0.029 mmol, 6 equivalents) and $Ti(OtBu)_4$ (18.54 μL, 0.048 mmol, 10 equivalents) were added thereto, and the obtained mixture was shaken at 120°C for 22 hours.

The reaction solution was cooled to room temperature. Then, under a nitrogen atmosphere, sodium triacetoxyborohydride (12.21 mg, 0.058 mmol, 12 equivalents) was added thereto, and the mixture was shaken at 120°C for 18 hours.

Reaction monitoring

After a lapse of 18 hours from the addition of sodium triacetoxyborohydride, measurement was performed in the same manner as in Example 2-5-2.

As a result, the amount of the starting material ketone (B013) remaining was 25%, and 40% of the product in an allyl ester form (B018) and 15% of alcohol (B016), a reduced form of ketone, were observed.

[Formula 340]

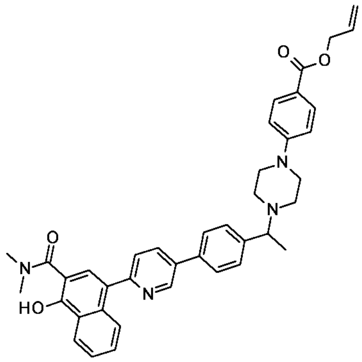

Prop-2-enyl 4-[4-[1-[4-[6-[3-(dimethylcarbamoyl)-4-hydroxynaphthalen-1- yl]pyridin-3-yl]phenyl]ethyl]piperazin-1-yl]benzoate: compound B018

LCMS: m/z 641.1[M+H]+.

Retention time: 0.910 min (analysis conditions SMD-FA05-1, 319 nm).

Example 2-5-5-1: Reductive amination reaction of solid-phase supported $ArCOCH_3$ (B021-01R) with allyl 4-(piperazin-1-yl)benzoate (BB27) (confirmation of effect of elevation of reaction temperature)

Reaction was carried out by setting a reaction temperature to 140°C for the purpose of improving the conversion rate, and increasing the numbers of equivalents of amine and $Ti(OtBu)_4$ in order to promote iminium formation.

[Formula 341]

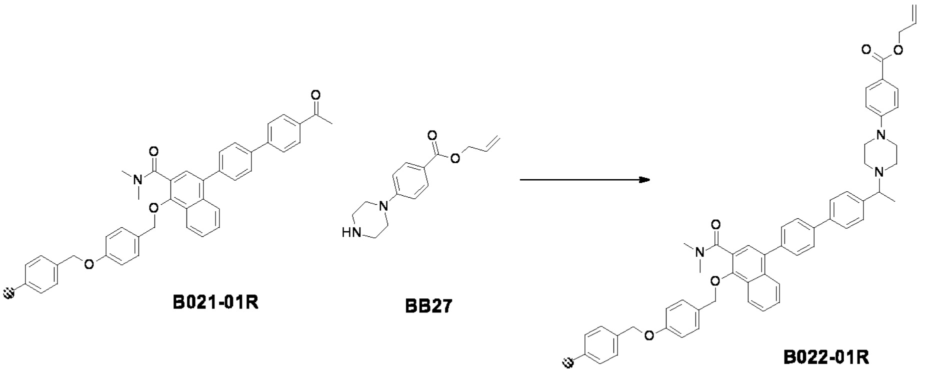

B021-01R BB27 B022-01R

Under a nitrogen atmosphere, solid-phase supported aryl ketone B021-01R (0.32 mmol/g, 20.0 mg, 6.4 μmol, 1.0 equivalent) and anisole (500 μL) were added to a 1.5 mL screw-cap vial and shaken at room temperature for 1.5 hours. Under a nitrogen atmosphere, allyl 4-(piperazin-1-yl)benzoate (BB27) (9.46 mg, 0.038 mmol, 6 equivalents) and $Ti(OtBu)_4$ (25.0 μL, 0.064 mmol, 10 equivalents) were added thereto, and the obtained mixture was shaken at 140°C for 21 hours.

The reaction solution was cooled to room temperature. Then, under a nitrogen atmosphere, sodium triacetoxyborohydride (16.00 mg, 0.077 mmol, 12 equivalents) was added thereto, and the mixture was shaken at 140°C for 5 hours.

Reaction monitoring

After a lapse of 5 hours from the addition of sodium triacetoxyborohydride, measurement was performed in the same manner as in Example 2-5-2.

The results are shown in run 1 of Table 2-5-5.

Example 2-5-5-2: Reductive amination reaction of solid-phase supported $ArCOCH_3$ (B021-01R) with allyl 4-(piperazin-1-yl)benzoate (BB27) (increase in the number of equivalents of reagent)

Under a nitrogen atmosphere, solid-phase supported aryl ketone B021-01R (0.32 mmol/g, 20.0 mg, 6.4 μmol, 1.0 equivalent) and anisole (500 μL) were added to a 1.5 mL screw-cap vial and shaken at room temperature for 1.5 hours. Under a nitrogen atmosphere, allyl 4-(piperazin-1-yl)benzoate (BB27) (19.00 mg, 0.077 mmol, 12 equivalents) and $Ti(OtBu)_4$ (49.0 μL, 0.128 mmol, 20 equivalents) were added thereto, and the obtained mixture was shaken at 140°C for 21 hours.

The reaction solution was cooled to room temperature. Then, under a nitrogen atmosphere, sodium triacetoxyborohydride (33.00 mg, 0.154 mmol, 24 equivalents) was added thereto, and the mixture was shaken at 140°C for 5 hours.

Reaction monitoring

After a lapse of 5 hours from the addition of sodium triacetoxyborohydride, measurement was performed in the same manner as in Example 2-5-2. The results are shown in run 2 of Table 2-5-5.

[Formula 342]

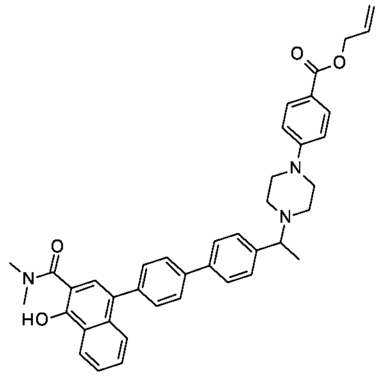

Prop-2-enyl 4-[4-[1-[4-[4-[3-(dimethylcarbamoyl)-4-hydroxynaphthalen-1- yl]phenyl]phenyl]ethyl]piperazin-1-yl]benzoate: compound B022

LCMS: m/z 640.2[M+H]+.

Retention time: 1.067 min (analysis conditions SMD-FA05-1, 319 nm).

[Formula 343]

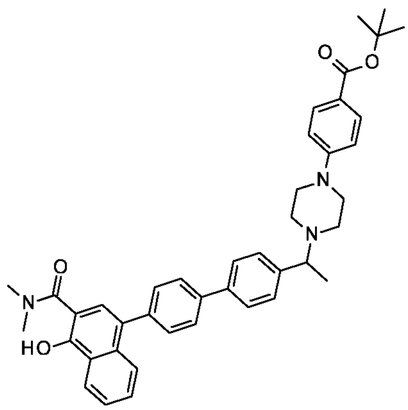

tert-Butyl 4-[4-[1-[4-[4-[3-(dimethylcarbamoyl)-4-hydroxynaphthalen-1- yl]phenyl]phenyl]ethyl]piperazin-1-yl]benzoate: compound B023

LCMS: m/z 656[M+H]+.

Retention time: 1.127 min (analysis conditions SMD-FA05-1, 319 nm).

[Formula 344]

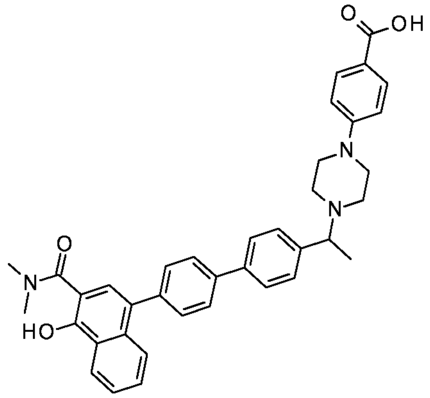

4-[4-[1-[4-[4-[3-(Dimethylcarbamoyl)-4-hydroxynaphthalen-1- yl]phenyl]phenyl]ethyl]piperazin-1-yl]benzoic acid: compound B024

LCMS: m/z 600[M+H]+.

Retention time: 0.941 min (analysis conditions SMD-FA05-1, 319 nm).

The results described above are shown in Table 2-5-5.

[Table 32]

[Table 2-5-5]

| Run | Amine / $Ti(OtBu)_4$ / $NaBH(OAc)_3$ | Ketone (**B021**) (area%) | allyl ester (**B022**) (area%) | tBu ester (**B023**) (area%) | carboxylic acid (**B024**) (area%) | Byproducts (not identified) |
|---|---|---|---|---|---|---|
| 1 | 6/10/12 | 24.0 | 60.0 | 1.8 | 5.5 | 8.7 |
| 2 | 12/20/24 | 6.9 | 77.2 | 5.3 | 5.7 | 4.9 |

From these results, it was confirmed that a high conversion rate can be achieved by setting a reaction temperature to 140°C and increasing the number of equivalents of a reagent. Although not wishing to be bound by any theory, the formation of an OH form was not seen in this reaction, probably because of sufficient progression of iminium formation at the first step. The formation of the product of interest in an allyl ester form (B022) as well as a tBu ester form (B023) as a transesterified form and a hydrolyzed carboxylic acid form (B024) was also seen. However, these compounds can be converted to the subsequent product of interest in a carboxylic acid form (B024) by the subsequent deprotection step and therefore do not correspond to the problem of reduction in purity.

Example 2-5-6: Reductive amination reaction of solid-phase supported $ArCOCH_3$ (B021-01R) with allyl 4-(piperazin-1-yl)benzoate (BB27) (confirmation of effect of increase in the number of equivalents of reducing agent)

Reaction conditions were confirmed through reaction using an increased number of equivalents of sodium triacetoxyborohydride and scale-up reaction for the purpose of further improving the conversion rate.

[Formula 345]

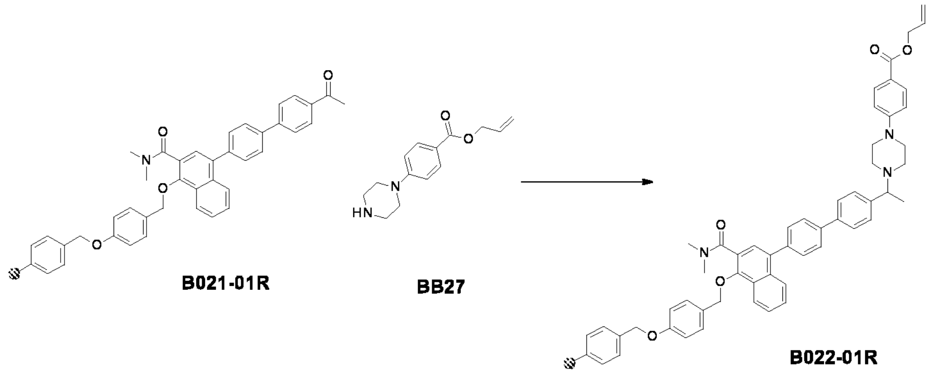

Example 2-5-6-1

Under a nitrogen atmosphere, solid-phase supported aryl ketone B021-01R (0.35 mmol/g, 20.0 mg, 7.0 μmol, 1.0 equivalent) and anisole (500 μL) were added to a 1.5 mL screw-cap vial and shaken at room temperature for 1.5 hours. Under a nitrogen atmosphere, allyl 4-(piperazin-1-yl)benzoate (BB27) (21.00 mg, 0.084 mmol, 12 equivalents) and $Ti(OtBu)_4$ (54.0 μL, 0.140 mmol, 20 equivalents) were added thereto, and the obtained mixture was shaken at 140°C for 22 hours.

The reaction solution was cooled to room temperature. Then, under a nitrogen atmosphere, sodium triacetoxyborohydride (45.00 mg, 0.210 mmol, 30 equivalents) was added thereto, and the mixture was shaken at 140°C for 20 hours.

Reaction monitoring

After a lapse of 20 hours from the addition of sodium triacetoxyborohydride, under a nitrogen atmosphere, 10 μL of the suspension of the reaction solution and the resin was sampled, transferred to a pipette tip with a filter, washed three times with NMP (100 μL), three times with MeOH (100 μL), and three times with DCM (100 μL), and then dipped in a 10% solution of TFA in DCM (50 μL) for 5 minutes. After filtration, the filtrate was diluted with NMP (200 μL) and acetonitrile (800 μL) to prepare an LC sample. The degree of progression of the reaction was measured by LC-MS analysis. The results are shown in run 1 of Table 2-5-6.

Example 2-5-6-2

Under a nitrogen atmosphere, solid-phase supported aryl ketone B021-01R (0.35 mmol/g, 100.0 mg, 35 μmol, 1.0 equivalent) and anisole (2.5 mL) were added to a 4.0 mL screw-cap vial and shaken at room temperature for 1.5 hours. Under a nitrogen atmosphere, allyl 4-(piperazin-1-yl)benzoate (BB27) (103.0 mg, 0.42 mmol, 12 equivalents) and $Ti(OtBu)_4$ (270.0 μL, 0.700 mmol, 20 equivalents) were added thereto, and the obtained mixture was shaken at 140°C for 21 hours.

The reaction solution was cooled to room temperature. Then, under a nitrogen atmosphere, sodium triacetoxyborohydride (223.0 mg, 1.05 mmol, 30 equivalents) was added thereto, and the mixture was shaken at 140°C for 18 hours.

Reaction monitoring

After a lapse of 18 hours from the addition of sodium triacetoxyborohydride, measurement was performed in the same manner as in Example 2-5-6-1. The results are shown in run 2 of Table 2-5-6.

[Table 33]

[Table 2-5-6]

| Run | Scale (mg) | Ketone (**B021**) (area%) | Total area% (**B022** + **B023** + **B024)** | allyl ester (**B022**) (area%) | tBu ester (**B023**) (area%) | carboxylic acid (**B024**) (area%) | Byproducts (not identified) |
|---|---|---|---|---|---|---|---|
| 1 | 20.0 | 0.7 | 96.5 | 83.3 | 7.1 | 6.1 | 2.8 |
| 2 | 100.0 | 0.8 | 97.2 | 86.5 | 5.1 | 5.6 | 2.0 |

From these results, it was able to be confirmed that a high conversion rate through reaction can be achieved by increasing the number of equivalents of sodium triacetoxyborohydride to 30 equivalents and thereby reducing the amount of ketone remaining to 1 area% or less. Even when the same reaction as above was carried out on a 5- fold scale, any problem caused by scale-up was not seen.

Example 2-5-7: Reductive amination reaction of solid-phase supported $ArCOCH_3$ (B013-01R) with allyl 4-(piperazin-1-yl)benzoate (BB27) (confirmation of substrate)

The progression of reaction was confirmed using substrate B013-01R having pyridine for the purpose of expanding applied substrates under the reductive amination reaction conditions set for solid-phase supported ketone B021-01R.

[Formula 346]

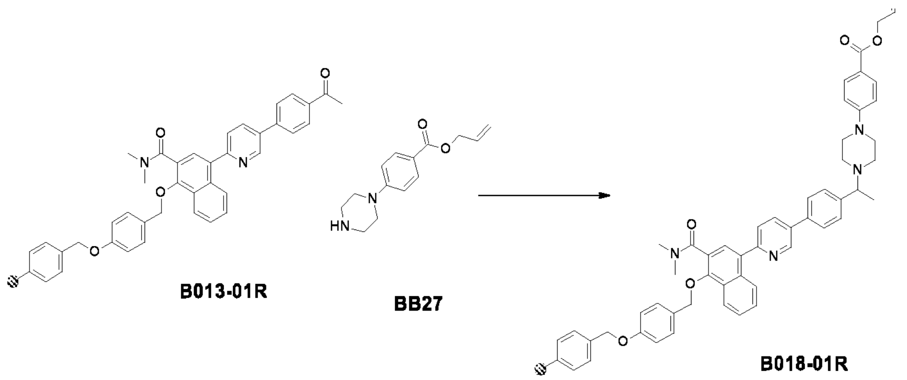

B013-01R BB27 B018-01R

Under a nitrogen atmosphere, solid-phase supported aryl ketone B013-01R (0.37 mmol/g, 100.0 mg, 37 μmol, 1.0 equivalent) and anisole (2.5 mL) were added to a 4.0 mL screw-cap vial and shaken at room temperature for 1.5 hours. Under a nitrogen atmosphere, allyl 4-(piperazin-1-yl)benzoate (BB27) (109.0 mg, 0.44 mmol, 12 equivalents) and $Ti(OtBu)_4$ (286.0 μL, 0.740 mmol, 20 equivalents) were added thereto, and the obtained mixture was shaken at 140°C for 22 hours.

The reaction solution was cooled to room temperature. Then, under a nitrogen atmosphere, sodium triacetoxyborohydride (235.0 mg, 1.10 mmol, 30 equivalents) was added thereto, and the mixture was shaken at 140°C for 20 hours.

Reaction monitoring

After a lapse of 20 hours from the addition of sodium triacetoxyborohydride, measurement was performed in the same manner as in Example 2-5-6-1. The results are shown in Table 2-5-7.

[Table 34]

[Table 2-5-7]

| Run | Ketone (**B013**) (area%) | Total area% **(B018** + **B019** + **B020)** | allyl ester (**B018**) (area%) | tBu ester (**B019**) (area%) | carboxylic acid (**B020**) (area%) | Byproducts (not identified) |
|---|---|---|---|---|---|---|
| 1 | 0.8 | 96.4 | 88.1 | 5.1 | 3.2 | 2.8 |

[Formula 347]

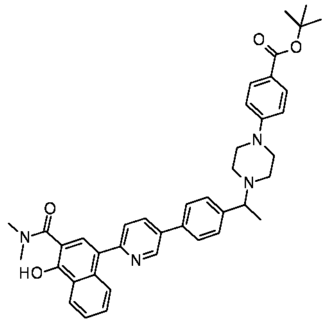

tert-Butyl 4-[4-[1-[4-[6-[3-(dimethylcarbamoyl)-4-hydroxynaphthalen-1-yl]pyridin- 3-yl]phenyl]ethyl]piperazin-1-yl]benzoate: compound B019

LCMS: m/z 657[M+H]+.
Retention time: 0.957 min (analysis conditions SMD-FA05-1, 319 nm).

[Formula 348]

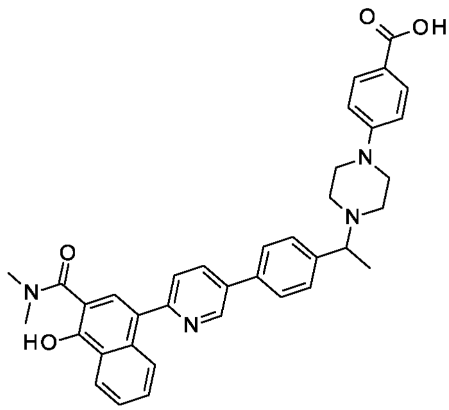

4-[4-[1-[4-[6-[3-(Dimethylcarbamoyl)-4-hydroxynaphthalen-1-yl]pyridin-3- yl]phenyl]ethyl]piperazin-1-yl]benzoic acid: compound B020

LCMS: m/z 601.3[M+H]+.
Retention time: 0.760 min (analysis conditions SMD-FA05-1, 319 nm).

Example 2-5-8: Reductive amination reaction of solid-phase supported $ArCOCH_3$ (B025-01R) with allyl 4-(piperazin-1-yl)benzoate (BB27) (confirmation of substrate)

The progression of reaction was confirmed using substrate (B025-01R) having fluorine for the purpose of expanding applied substrates under the reductive amination reaction conditions set above.

[Formula 349]

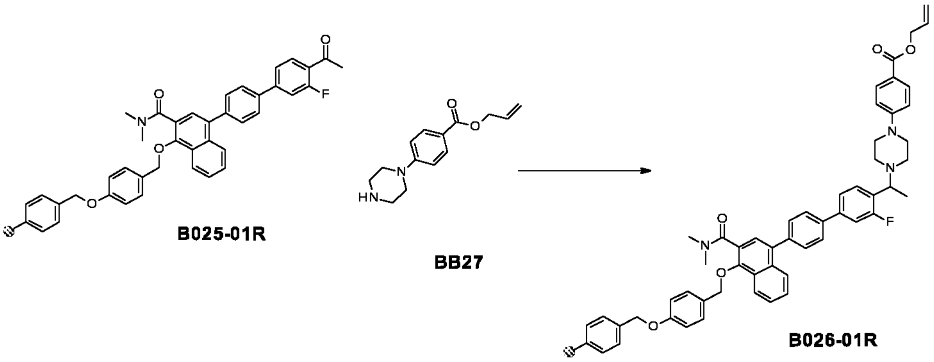

B025-01R

BB27

B026-01R

Under a nitrogen atmosphere, solid-phase supported aryl ketone B025-01R (0.35 mmol/g, 20.0 mg, 7.0 μmol, 1.0 equivalent) and anisole (500 μL) were added to a 1.5 mL screw-cap vial and shaken at room temperature for 1.5 hours. Under a nitrogen atmosphere, allyl 4-(piperazin-1-yl)benzoate (BB27) (21.00 mg, 0.084 mmol, 12 equivalents) and $Ti(OtBu)_4$ (54.0 μL, 0.140 mmol, 20 equivalents) were added thereto, and the obtained mixture was shaken at 140°C for 22 hours.

The reaction solution was cooled to room temperature. Then, under a nitrogen atmosphere, sodium triacetoxyborohydride (45.00 mg, 0.210 mmol, 30 equivalents) was added thereto, and the mixture was shaken at 140°C for 20 hours.

Reaction monitoring

After a lapse of 20 hours from the addition of sodium triacetoxyborohydride, under a nitrogen atmosphere, 10 μL of the suspension of the reaction solution and the resin was sampled, transferred to a pipette tip with a filter, washed three times with NMP (100 μL), three times with MeOH (100 μL), and three times with DCM (100 μL), and then dipped in a 10% solution of TFA in DCM (50 μL) for 5 minutes. After filtration, the filtrate was diluted with TFE (200 μL) and acetonitrile (800 μL) to prepare an LC sample. The degree of progression of the reaction was measured by LC-MS analysis. The results are shown in Table 2-5-8.

[Table 35]

[Table 2-5-8]

| Run | Ketone (**B025**) (area%) | Total area% (**B026 + B027 + B028**) | allyl ester (**B026**) (area%) | tBu ester (**B027**) (area%) | carboxylic acid (**B028**) (area%) | Byproducts (not identified) |
|---|---|---|---|---|---|---|
| 1 | 0.2 | 95.1 | 76.2 | 8.6 | 10.3 | 4.7 |

[Formula 350]

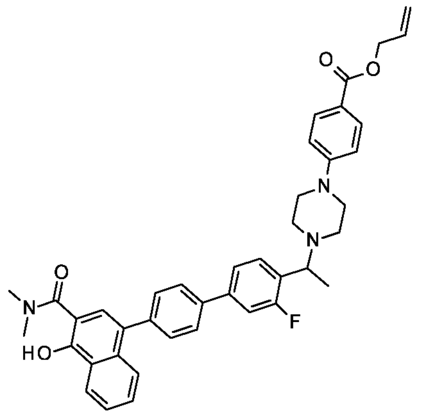

Prop-2-enyl 4-[4-[1-[4-[4-[3-(dimethylcarbamoyl)-4-hydroxynaphthalen-1- yl]phenyl]-2-fluorophenyl]ethyl]piperazin-1-yl]benzoate: compound B026

LCMS: m/z 658.3$[M+H]^+$.

Retention time: 1.112 min (analysis conditions SMD-FA05-1, 319 nm).

[Formula 351]

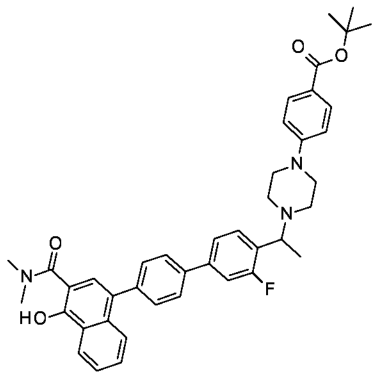

tert-Butyl 4-[4-[1-[4-[4-[3-(dimethylcarbamoyl)-4-hydroxynaphthalen-1-yl]phenyl]- 2-fluorophenyl]ethyl]piperazin-1-yl]benzoate: compound B027

LCMS: m/z 674.3$[M+H]^+$.

Retention time: 1.181 min (analysis conditions SMD-FA05-1, 319 nm).

[Formula 352]

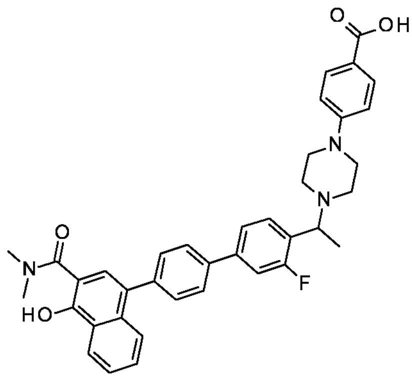

4-[4-[1-[4-[4-[3-(Dimethylcarbamoyl)-4-hydroxynaphthalen-1-yl]phenyl]-2- fluorophenyl]ethyl]piperazin-1-yl]benzoic acid: compound B028

LCMS: m/z 618.3[M+H]$^+$.

Retention time: 0.941 min (analysis conditions SMD-FA05-1, 319 nm).

The results described above demonstrated that reductive amination can be performed at a high purity and a high conversion rate through reaction for a wide range of substrates by applying thereto the reaction conditions found in Example 2-5-6. Although the reaction conditions for reductive amination reaction are not limited to this example, this example is excellent conditions applicable to mixture library synthesis.

Example 2-6: Ensuring of robustness of reaction by selection of suitable method for washing resin

For achieving a high conversion rate through reaction in mixture library synthesis, it is necessary not only to suitably set individual reaction conditions but to contrive a washing step after implementation of reaction. Hereinafter, an exemplary method for selecting a suitable washing method after hydrolysis reaction of an ester moiety and thereby enhancing the robustness of the subsequent amide coupling reaction will be illustrated.

Example 2-6-1: Solid-phase experiment to suppress influence of difference in washing method after hydrolysis of solid-phase supported Ar-COOR on amide coupling reaction in subsequent step

In the case of hydrolyzing an ester site using a base to expose carboxylic acid for use in the subsequent bond formation reaction, the carboxylic acid may exist in the form of a salt formed with a counter cation of the base used, not in a free form (formula AF-1). For example, when the subsequent bond formation reaction is amidation using a condensing agent, reduction in the conversion rate through reaction derived from the salt, not a free form, formed by carboxylic acid is of concern depending on the type of the condensing agent. The following experiment was made on the influence of difference in washing method after hydrolysis of an ester site on amide coupling reaction in the subsequent step.

[Formula 353]

[Formula AF-1]

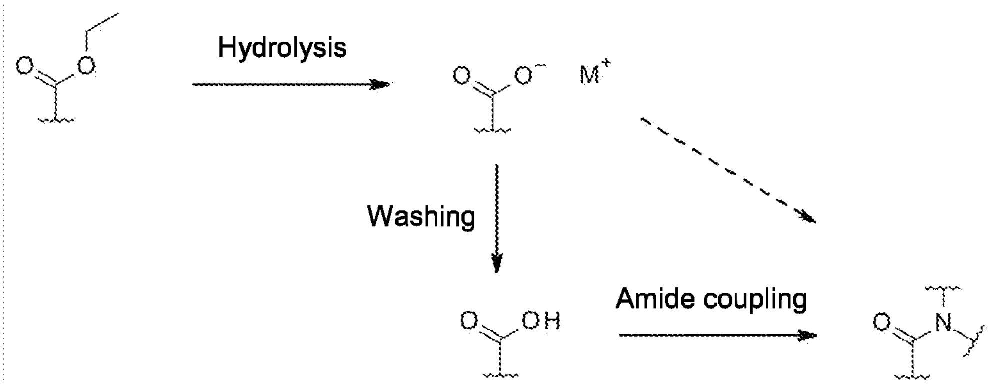

As shown in formula AF-2, ester compound AF001-01R was subjected to hydrolysis reaction and subjected to amide coupling reaction with piperazine derivative BB28. In this respect, a method for washing a resin after hydrolysis reaction was carried out by the methods shown in Table AF-01, and the rate of progression of amide coupling reaction in the subsequent step and reproducibility were comparatively studied. The ester compound AF001-01R was synthesized by the method described in Example 2-3-4.

In resin washing after hydrolysis, a solution of tetrabutylammonium hydrogen sulfate in NMP or a solution of HOAt in NMP was selected here as a reagent solution capable of protonating carboxylate, in consideration of pKa of various carboxylic acids. In amide coupling reaction, DIC was selected as a condensing agent capable of coexisting with amine, i.e., condensing agent that was not consumed through unintended reaction with amine, and HOAt was selected as an additive for suppressing rearrangement to urea from primary active ester formed from carboxylic acid and DIC. Results of the study are shown in Table AF-01. The operation of reaction shown in run 4 will be shown as an exemplary experiment.

[Formula 354]

[Formula AF-2]

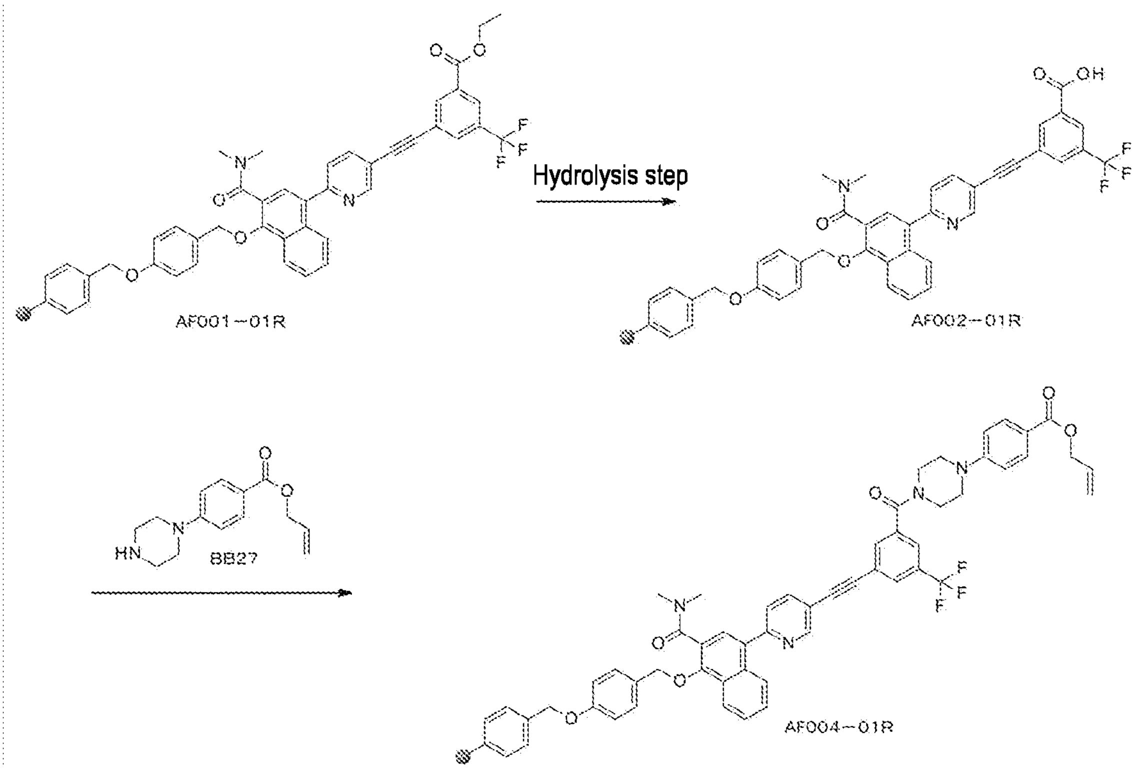


Under a nitrogen atmosphere, AF001-01R (0.37 mmol/g, 15 mg, 1.0 equivalent), NMP (240 μL, 16 v/w), and MeOH (60 μL, 4 v/w) were added to a 0.6 mL screw-cap vial and shaken at room temperature for 1 hour. An aqueous NaOH solution (5.0 M, 9.0 μL, 4 v/w) was added thereto, and the mixture was shaken at room temperature for 1 hour and 30 minutes. The resin suspension was transferred to a syringe with a filter using NMP, washed three times with NMP (300 μL, 20 v/w), three times with $H_2O$ (300 μL, 20 v/w), three times with NMP (300 μL, 20 v/w), three times with a solution of HOAt in NMP (0.2 M, 300 μL, 20 v/w), three times with NMP (300 μL, 20 v/w), three times with MeOH (300 μL, 20 v/w), three times with DCM (300 μL, 20 v/w), and three times with heptane (300 μL, 20 v/w), and then dried under reduced pressure to obtain AF001-01R.

After a lapse of 1 hour and 30 minutes from the start of shaking, under a nitrogen atmosphere, 10 μL of the suspension of the reaction solution and the resin was sampled, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in NMP (50 μL), and the solution (50 μL) was diluted with MeCN (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

[Formula 355]

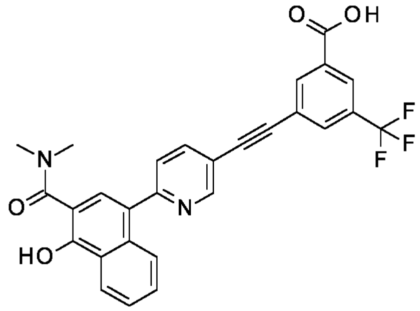

AF002

LRMS: m/z 505.1[M+H]+

Retention time: 1.30 min (analysis conditions SMD-FA05-1)

The amide coupling reaction was carried out in accordance with following experimental example.

Under a nitrogen atmosphere, AF002-01R (0.37 mmol/g, 15 mg, 1.0 equivalent) and NMP (255 μL, 17 v/w) were added to a 0.6 mL screw-cap vial and shaken at room temperature for 1 hour. HOAt (5.3 mg, 6.0 equivalents) and DIC (6.0 mg, 6.0 equivalents) were added thereto, and the mixture was shaken at 40°C for 22 hours.

After a lapse of each time shown in Table AF-01, under a nitrogen atmosphere, 10 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in NMP (250 μL), and the solution was diluted with MeCN (250 μL) to prepare an LC sample. The degree of progression of the reaction was measured by LC-MS analysis. The following compound to be detected was analyzed under analysis conditions SMD-FA05-1, and the UV area% was confirmed at a UV wavelength of 319 nm.

[Formula 356]

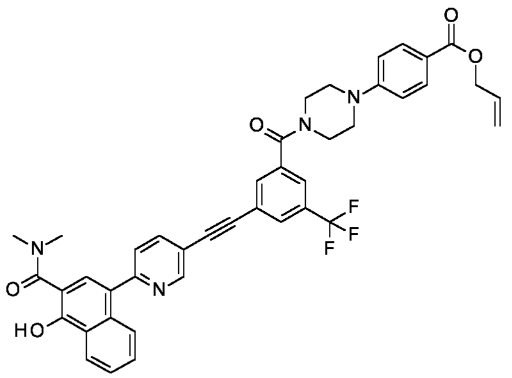

AF004

LRMS: m/z 733.2[M+H]+.

Retention time: 1.51 min (analysis conditions SMD-FA05-1).

The LCMS results are shown in Table AF-1. In the case of carrying out amide coupling reaction using AF002-01R washed by the method of run 1 using a $nBu_4NHSO_4$ solution as a protonation source for carboxylic acid, the reaction was not completed even after a lapse of 6 hours, and the conversion rate through reaction was on the order of 98%. These results were confirmed to have reproducibility between two experiments. In the case of using AF002-01R washed by an additional washing step with $H_2O$ after the first washing with NMP and after washing with a $nBu_4NHSO_4$ solution (run 2), the reaction was completed after a lapse of 3 hours, and the peak of compound AF004 of interest was confirmed at 100 area%. These results were confirmed to have reproducibility between two experiments. In order to establish a method for further enhancing the robustness of reaction, a washing method was studied by selecting HOAt as an acid component for use in the protonation of carboxylic acid. HOAt is used as an additive in amide coupling reaction and therefore does not influence amide coupling reaction in the subsequent step even if HOAt remains on a resin, for example, because of adhering to the resin when used in the preceding washing step. For this reason, this approach is useful for enhancing the robustness of reaction in the subsequent step. In the case of using AF002-01R washed using a HOAt

solution (run 3), the reaction was completed after a lapse of 3 hours, and the peak of compound AF004 of interest was confirmed at 100 area%. In the case of using AF002-01R washed in the same manner as above and then washed by an additional washing method with heptane (run 4), the reaction was also completed after a lapse of 3 hours, and the peak of compound AF004 of interest was confirmed at 100 area%. These methods were able to be confirmed to have reproducibility.

[Table 36]

[Table AF-01]

| | Table AF-01 | | | | |
|---|---|---|---|---|---|
| | | | Reaction time | Conversion rate in amidation | |
| run | Washing solvent | Amidation conditions | hr | n=1 | n=2 |
| 1 | 1. NMP<br>2. 0.05M nBu4NHSO4 in NMP<br>3. NMP<br>4. MeOH<br>5. DCM | amine (3 eq.)<br>DIC(6 eq.)<br>HOAt (6 eq.)<br>NMP (17 v/w) | 6 | 97.7 | 98.6 |
| 2 | 1. NMP<br>2. H2O<br>3. NMP<br>4. 0.05M nBu4NHSO4 in NMP<br>5. H2O<br>6. NMP<br>7. MeOH<br>8. DCM | amine (3 eq.)<br>DIC (6 eq.)<br>HOAt (6 eq.)<br>NMP (17 v/w) | 3 | 100 | 100 |
| 3 | 1. NMP<br>2. H2O<br>3. NMP<br>4. 0.2M HOAt in NMP<br>5. NMP<br>6. MeOH<br>7. DCM | amine (3 eq.)<br>DIC (6 eq.)<br>HOAt (6 eq.)<br>NMP (17 v/w) | 3 | 100 | - |
| 4 | 1. NMP<br>2. H2O<br>3. NMP<br>4. 0.2M HOAt in NMP<br>5. NMP<br>6. MeOH<br>7. DCM<br>8. heptane | amine (3 eq.)<br>DIC (6 eq.)<br>HOAt (6 eq.)<br>NMP (17 v/w) | 3 | 100 | - |

The results of this experiment demonstrated that the subsequent amidation reaction can be reproducibly carried out at a high conversion rate through reaction by exposing carboxylic acid by the hydrolysis of an ester site, and then introducing a washing step with a reagent solution that can protonate carboxylate. The results demonstrated that the robustness of amide coupling reaction in the subsequent step can be enhanced, particularly, by selecting a HOAt solution for washing. The approach of the present invention is an exemplary approach for enhancing the robustness of reaction for constructing a library in order to supply a "high-quality" library.

Example 2-7: Liquid-phase experiment to confirm scope of substrate application to amide coupling reaction as typical example of identifying substrate scope that can be applied to library synthesis

Hereinafter, an exemplary method for identifying an applicable substrate scope under reaction conditions expected to attain a wide scope of substrate application to amide coupling reaction will be illustrated. The scope given below is a mere illustration of an exemplary identification method, and the present invention is not limited by the scope given below in actual application to library synthesis.

Although not bound by amide coupling, it is not realistic in one aspect to experimentally confirm the success or failure of reaction as to individual substrates one by one, for carrying out large-scale mixture library synthesis at a high conversion rate through reaction and a high purity. Hence, if the success or failure of reaction can be predicted for a large number of unspecified substrates without implementation of study, this approach is of very high value. In Example 2-7, an exemplary approach of predicting the success or failure of reaction of a substrate whose scope of application is not experimentally confirmed, at the same time with the confirmation of the scope of substrate application to amide coupling reaction will also be shown.

Although not wishing to be bound by any theory, an electronic factor and a steric factor of a nucleophile make a great contribution as determinants for the success or failure of amidation reaction. The success or failure of reaction can be predicted using suitable parameters of these factors. cpKa and ASA were used here as indexes for the electronic factor and the steric factor, respectively.

In this context, ASA is an abbreviation of accessible surface area and serves as an index that indicates the accessible area of a size surrounding a specific atom by another molecule. Here, ASA is used as an index that indicates the area of the reaction point of an electrophile accessible to the reaction point of an electrophile. A smaller value of ASA means that another molecule is less accessible to the atom and steric hindrance surrounding the atom is large (literatures known in the art: Science. 1983 Aug 19; 221 (4612): 709-13; and J. Org. Chem. 2011, 76, 2, 435-440).

Method for calculating ASA used in present specification

ASA was selected as the largest ASA among ASA values of a plurality of conformations, by generating conformations (search from the most stable conformation in $CHCl_3$ to conformations having high energy up to 30 kJ/mol) with MacroModel of Maestro from Schrodinger, Inc., and calculating ASA as to each of the plurality of conformations thus obtained with Advanced Surfaces of Maestro also from Schrodinger, Inc. (Type: Extended Surface).

In this Example 2-7, ASA was selected as the largest ASA among ASA values of a plurality of conformations, by generating conformations for a nucleophile amine compound (molecule), and calculating ASA of the nitrogen atom of an amino group serving as a nucleophilic site as to each of the plurality of conformations thus obtained.

Example 2-7-1: Solid-phase experiment of amide coupling reaction of solid-phase supported Ar-COOH with amine

From among reagents generally used in amide coupling reaction, DIC was selected as a condensing agent capable of slowly reacting and coexisting with amine present in the liquid phase, i.e., a condensing agent that was not consumed through unintended reaction with amine, and HOAt was selected as an additive for suppressing rearrangement to urea from primary active ester formed from carboxylic acid and DIC. Amide coupling reaction was carried out for amines BB30 to BB33 and AD013 to AD021 shown in Table AD-01 to confirm the scope of applicable building blocks.

Hereinafter, the operation of the reaction of AD006-01R with BB32 will be shown as an exemplary experiment. The compound AD006-01R was synthesized by the method described in Example 1-5-4.

[Formula 357]

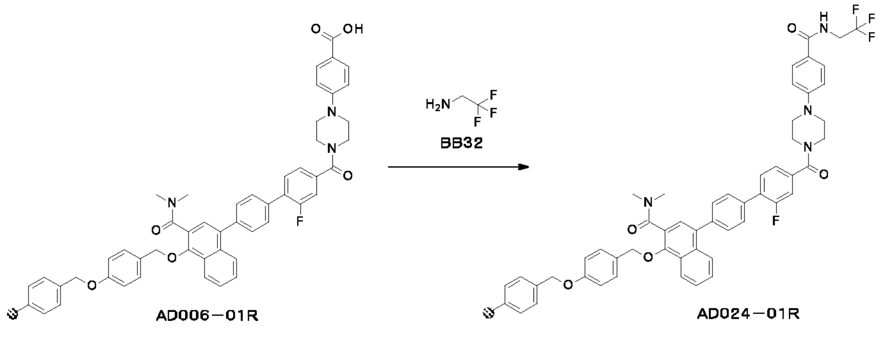


Under a nitrogen atmosphere, solid-phase supported Ar-COOH (AD006-01R) (0.40 mmol/g, 16.1 mg, 1.0

equivalent) and NMP (274 μL, 17 v/w) were added to a 0.6 mL screw-cap vial and shaken at room temperature for 1 hour. 2,2,2-Trifluoroethanamine (BB32) (1.5 mg, 3.0 equivalents) was added thereto, and the mixture was shaken at room temperature for 30 minutes. Then, HOAt (5.3 mg, 6.0 equivalents) and DIC (6.0 mg, 6.0 equivalents) were added thereto, and the mixture was shaken at 40°C for 22 hours.

The amounts of solid-phase supported Ar-COOH and each reagent used in experiments using the other amines are shown in Table AD-01. The compound number and structure of the target compound obtained from each amine are shown in Table AD-02.

[Table 37]

[Table AD-01]

Table AD-01

| Compound No. of amine | Structural formula of amine | Amount of Ar-COOH resin used (mg) | mmol | Amount of NMP used (uL) | Amount of amine used (mg) | Amount of DIC used (mg) | Amount of HOAt used (mg) |
|---|---|---|---|---|---|---|---|
| BB30 | 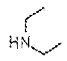 | 17.5 | 0.0070 | 298 | 2.19 | 6.54 | 5.7 |
| BB31 | 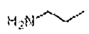 | 15.71 | 0.0063 | 267 | 1.55 | 5.87 | 5.1 |
| BB32 | 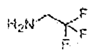 | 16.1 | 0.0064 | 274 | 1.52 | 6.02 | 5.3 |
| BB33 | 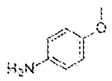 | 16.3 | 0.0065 | 277 | 2.41 | 6.10 | 5.3 |
| AD013 | 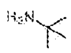 | 16.36 | 0.0065 | 278 | 2.08 | 6.12 | 5.3 |
| AD014 | 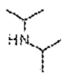 | 17.14 | 0.0069 | 291 | 2.89 | 6.41 | 5.6 |
| AD015 | 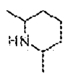 | 16.65 | 0.0067 | 283 | 2.76 | 6.23 | 5.4 |
| AD016 | 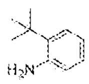 | 16.07 | 0.0064 | 273 | 3.01 | 6.01 | 5.2 |
| AD017 | 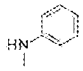 | 16.63 | 0.0067 | 283 | 2.16 | 6.22 | 5.4 |
| AD018 | 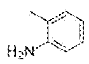 | 16.55 | 0.0066 | 281 | 2.13 | 6.19 | 5.4 |
| AD019 | 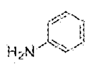 | 17.57 | 0.0070 | 299 | 1.93 | 6.57 | 5.7 |
| AD020 | 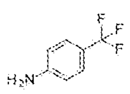 | 15.77 | 0.0063 | 268 | 2.38 | 5.90 | 5.2 |
| AD021 | 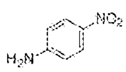 | 15.39 | 0.0062 | 262 | 2.55 | 5.75 | 5.0 |

[Table 38]

[Table AD-02]

| Table AD-02 | | | | |
|---|---|---|---|---|
| Compound No. of amine | Structure of amine | Compound No. of compound of interest | Structure of compound of interest | Exact MS of compound of interest |
| BB30 | 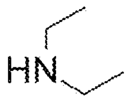 | AD022 | 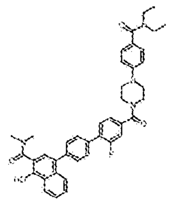 | 672.30 |
| BB31 | 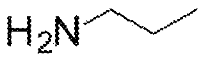 | AD023 | 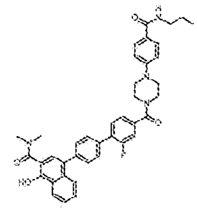 | 658.30 |
| BB32 | 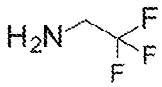 | AD024 | 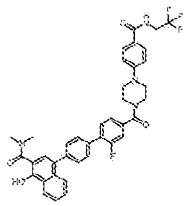 | 698.30 |
| BB33 | 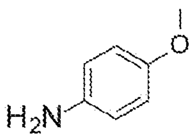 | AD025 | 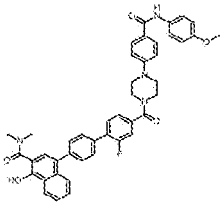 | 722.30 |
| AD013 | 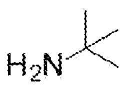 | AD026 | 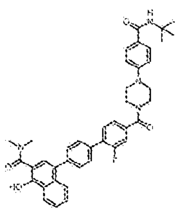 | 672.30 |

[Table 39]

| | | | | |
|---|---|---|---|---|
| AD014 | 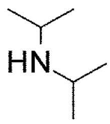 | AD027 | 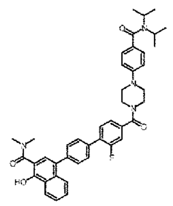 | 700.30 |
| AD015 | 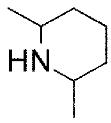 | AD028 | 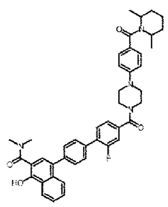 | 712.30 |
| AD016 | 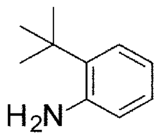 | AD029 | 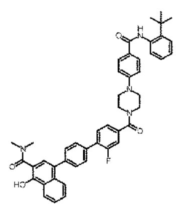 | 748.30 |
| AD017 | 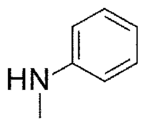 | AD030 | 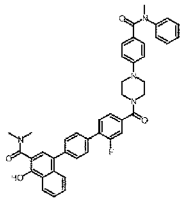 | 706.30 |
| AD018 | 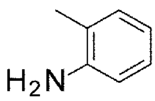 | AD031 | 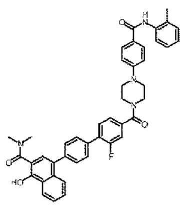 | 706.30 |

[Table 40]

| | | | | |
|---|---|---|---|---|
| AD019 | 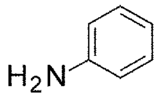 | AD032 | 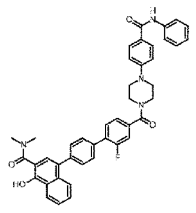 | 692.30 |
| AD020 | 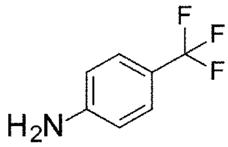 | AD033 | 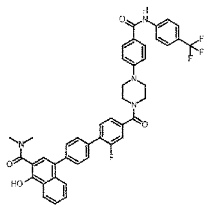 | 760.30 |
| AD021 | 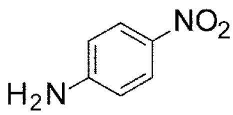 | AD034 | 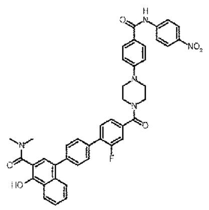 | 737.30 |

The reaction was monitored as follows.

After a lapse of a given reaction time, under a nitrogen atmosphere, 10 μL of the resin suspension was sampled and then discharged to n-$PrNH_2$ (20 μL) in a 1.5 mL glass vial. After pipetting several times, the mixture was transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in NMP (50 μL), and the solution (50 μL) was diluted with MeCN (250 μL) to prepare an LC sample. The degree of progression of the reaction was measured by LC-MS analysis.

The LCMS results are shown in Table AD-03. In the case of using each of AD007 to AD010 and AD019 as amine, 94.3 area% or more of the target compound in an amide form was confirmed after a lapse of 22 hours.

[Table 41]

| [Table AD-03] | | | | |
|---|---|---|---|---|
| Table AD-03 | | | | |
| Compound No. of amine | Compound No. of target compound | MS m/z [M+H]+ of target compound | Retention time of target compound (min) | UV area% (319 nm) |
| BB30 | AD022-01R | 673.3 | 1.33 | 98.0 |
| BB31 | AD023-01R | 659.3 | 1.27 | 98.2 |
| BB32 | AD024-01R | 699.2 | 1.30 | 100.0 |
| BB33 | AD025-01R | 723.2 | 1.36 | 99.6 |
| AD013 | AD026-01R | 673.3 | 1.37 | 88.4 |
| AD014 | AD027-01R | ND | ND | 0 |
| AD015 | AD028-01R | 713.3 | 1.46 | 0.5 |
| AD016 | AD029-01R | 749.3 | 1.47 | 1.6 |
| AD017 | AD030-01R | 707.2 | 1.38 | 11.1 |
| AD018 | AD031-01R | 707.3 | 1.38 | 50.7 |
| AD019 | AD032-01R | 693.2 | 1.38 | 94.3 |
| AD020 | AD033-01R | 761.2 | 1.50 | 3.9 |

(continued)

| [Table AD-03] | | | | |
|---|---|---|---|---|
| Table AD-03 | | | | |
| Compound No. of amine | Compound No. of target compound | MS m/z [M+H]+ of target compound | Retention time of target compound (min) | UV area% (319 nm) |
| AD021 | AD034-01R | ND | ND | 0 |

Among the results of Table AD-03, the results obtained using primary amine are excerpted in Table AD-04, and the ASA value of the nitrogen atom of the amine used is also described.

[Table 42]

[Table AD-04] Influence of bulkiness of primary amine

| Table AD-04 | | | | |
|---|---|---|---|---|
| Compound No. of amine | Structure of amine | cpKa | ASA | TM |
| BB31 | 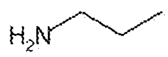 | 10.5 | 109.3 | 98.2 |
| BB32 | 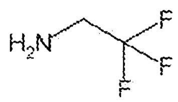 | 6.1 | 107.1 | 100 |
| AD013 | 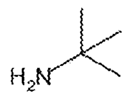 | 10.5 | 73.8 | 88.4 |

In the case of using benzoic acid having a substituent at the 4-position as a substrate on the electrophile side, as in this time, and in the case of using amine having nitrogen atom ASA > 73.8 as primary amine, reaction progressed at 88.4 area% or more under the reaction conditions of this Example.

From this tendency, reaction is difficult to complete if ASA of the nitrogen atom of primary amine falls below 73.8, and reduction in the purity of a mixture library is of concern. On the contrary, when ASA exceeds 73.8, it is expected that a mixture library having a high conversion rate through reaction and a high purity can be provided without causing reduction in purity.

Among the results of Table AD-05, the results obtained using secondary amine are excerpted in Table AD-04, and the ASA value of the nitrogen atom of the amine used is also described.

[Table 43]

[Table AD-05] Influence of bulkiness of secondary amine

Table AD-05

| Compound No. of amine | Structure of amine | cpKa | ASA | TM |
|---|---|---|---|---|
| BB30 | 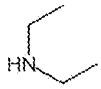 | 10.8 | 31.9 | 98.0 |
| AD014 | 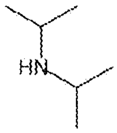 | 10.8 | 17.1 | 0 |
| AD015 | 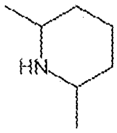 | 11.0 | 9.8 | 0 |

In the case of using benzoic acid having a substituent at the 4-position as a substrate on the electrophile side, as in the case of the primary amine in Table AD-04, and in the case of using amine having nitrogen atom ASA < 17.1 as secondary amine, reaction did not progress under the reaction conditions of this Example.

Thus, there is concern that no target compound is obtained if ASA of the nitrogen atom of secondary amine falls below 20. On the other hand, when ASA exceeds 30, it is expected that reaction can be completed and a mixture library having a high conversion rate through reaction and a high purity can be provided without causing reduction in purity.

Among the results of Table AD-03, the results obtained using aromatic amine having a cpKa value of 4.0 to 5.0 are excerpted in Table AD-06, and the ASA value of the nitrogen atom of the amine used is also described.

[Table 44]

[Table AD-06] Influence of bulkiness of aromatic amine

| Table AD-06 | | | | |
|---|---|---|---|---|
| Compound No. of amine | Structure of amine | cpKa | ASA | TM |
| BB33 | 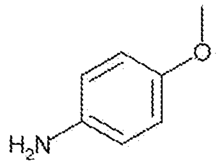 | 4.9 | 78.6 | 99.6 |
| AD019 | 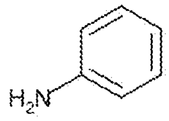 | 4.5 | 78.2 | 94.3 |
| AD018 | 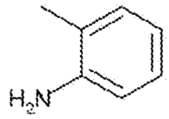 | 4.3 | 67.6 | 50.7 |
| AD016 | 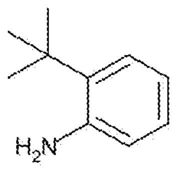 | 4.5 | 49.8 | 1.6 |

In the case of using benzoic acid having a substituent at the 4-position as a substrate on the electrophile side, reduction in purity is of concern if ASA of the nitrogen atom of aromatic amine falls below 70. On the other hand, when ASA exceeds 75, the conversion rate through reaction exceeds 90%. Thus, it is expected that a high-purity mixture library can be provided.

In the case of using amine having nitrogen atom ASA > 78.2 as aromatic amine, reaction progressed at 88.4 area% or more under the reaction conditions of this Example.

From these results, the scope of amine application to a library was found by identifying amine that attained the progression at high yields of amidation reaction using DIC as a condensing agent and HOAt as an additive for solid-phase supported Ar-COOH. Particularly, the ASA value of the nitrogen atom of amine was used as an index for the steric hindrance of a nucleophile to elucidate the scope of amines that attained the progression of reaction at high yields and identify the scope of substrate application.

This Example is a mere illustration of an exemplary method for identifying a substrate scope that can be applied to a mixture library, and the present invention is not limited thereby in actual application to library synthesis. Particularly, even if ASA falls outside the range described above, there is room for a higher conversion rate through reaction by the prolongation of a reaction time or by the adjustment of a reaction temperature or the amount of a reagent, because solid-phase supported Ar-COOH or its active ester remains without being converted. In other words, in one embodiment, ASA can be exploited as an index to determine beforehand the difficulty of library synthesis. However, it is not always true that this reaction cannot be applied even if ASA falls outside the range described above. Furthermore, the scope of substrates that can be applied may be expanded by additional experimental study or from results about actual mixture library synthesis.

Example 2-8: Liquid-phase experiment to confirm scope of substrate application to acylsulfonamidation as typical example of identifying substrate scope that can be applied to library synthesis

Hereinafter, an exemplary applicable method for carrying out acylsulfonamidation reaction will be illustrated.

An approach of suppressing reduction in the purity of a mixture library when the conversion rate through reaction is insufficient will also be illustrated. The scope given below is a mere illustration of an exemplary identification method, and the present invention is not limited by the scope given below in actual application to library synthesis.

Example 2-8-1: Setting of conditions for liquid-phase acylsulfonamidation reaction of carboxylic acid with sulfonamide

An example of finding a problem and identifying a solution thereto in the course of the setting of acylsulfonamidation reaction conditions will be shown below.

With reference to a literature known in the art (J. Med. Chem. 2006, 49, 1165-1181), EDCI-HCl was selected as a condensing agent, DMAP was selected as an activator, and DCM was selected as a solvent. Acylsulfonamidation reaction was carried out using Ar- COOH AE008 and sulfonamide BB40. AE008 was synthesized by the method described in Example 1-6-4.

[Formula 358]

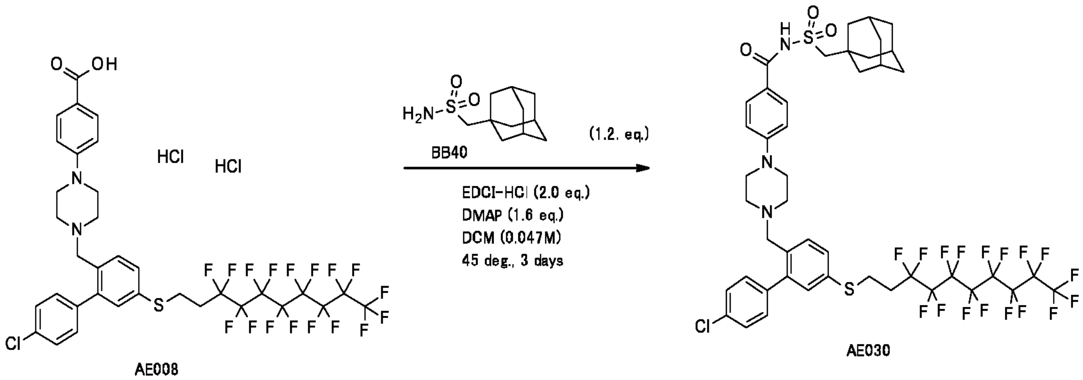


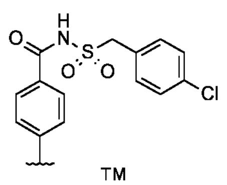
TM

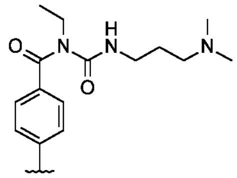
BP

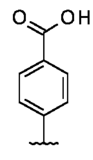
SM

Stirring was performed at 45°C for 3 days, and the reaction was monitored (PDA; 210-400 nm) by LC/MS. As a result, 57.7 area% of the target compound (TM) in an acylsulfonamide form (AE030) was confirmed, whereas 24.8 area% of a by-product (BP; structure: putative) resulting from the intramolecular rearrangement of active ester was observed.

[Formula 359]

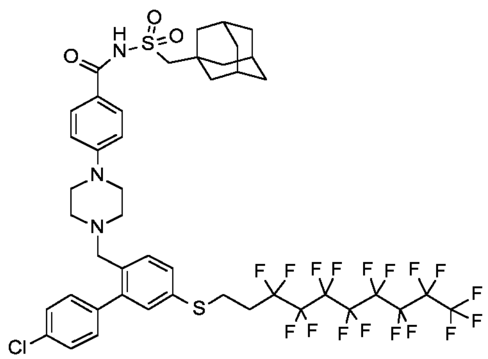

TM (AE030)

LRMS: m/z 1096.2[M+H]$^+$.

Retention time: 1.01 min (analysis conditions SMD-TFA05-1).

[Formula 360]

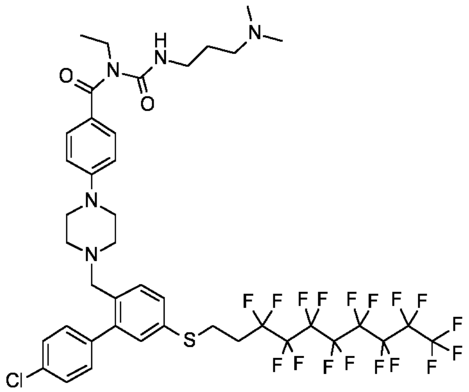

BP (AE051; structure: putative)

LRMS: m/z 1040.2[M+H]+.

Retention time: 0.59 min (analysis conditions SMD-TFA05-1).

The by-product thus confirmed may appear in common not only for EDCI but for other carbodiimide condensing agents such as DCC and DIC. Hence, a method for circumventing by-products by using a condensing agent other than the carbodiimide condensing agent was studied. The study was carried out by an operation given below using Ar-COOH AE013 and sulfonamide BB40. Hereinafter, the experimental example of run 3 in Table AE-01 will be shown as one example.

[Formula 361]

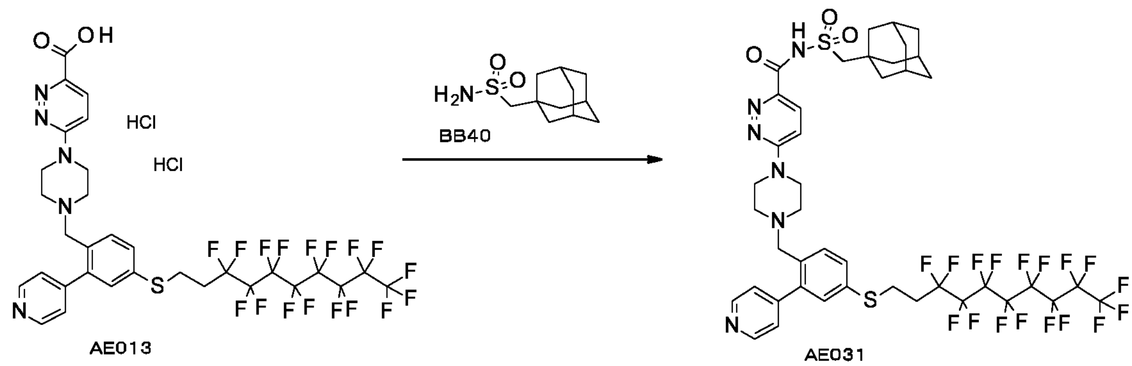


AE013 AE031

AE013 (5.0 mg, 1.0 equivalent), DCM (51.9 μL, 0.1 M), HATU (3.0 mg, 1.5 equivalents), and DIPEA (4.5 μL, 5.0 equivalents) were added to a 0.5 mL screw-cap vial, purged with nitrogen, and then stirred at room temperature for 20 minutes. Then, 1- adamantylmethanesulfonamide (BB40) (1.4 mg, 1.2 equivalents) and phosphazene PltBu (4.0 μL, 3.0 equivalents) were added thereto, and the mixture was stirred at room temperature for 1 hour.

The reaction was monitored as follows.

3 μL of the reaction solution was sampled and then discharged to n-PrNHz (30 μL) in a 1.5 mL glass vial. After pipetting several times, the mixture was diluted with MeCN to prepare an LC sample. The degree of progression of the reaction was measured by LC-MS analysis. Each compound shown in Table AE-00 to be detected was analyzed under analysis conditions SMD-TFA05-1, and the UV area% was confirmed by PDA (210-400 nm).

[Table 45]

[Table AE-00]

| Table AE-00 | | | |
|---|---|---|---|
| Compound No. of compound to be monitored | Structure | Observed MS m/z $[M+H]^+$ | Retention time (min) |
| AE013 | 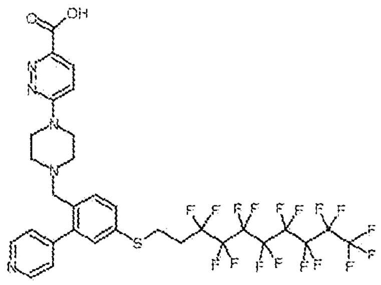 | 854.1 | 1.05 |
| AE0101 | 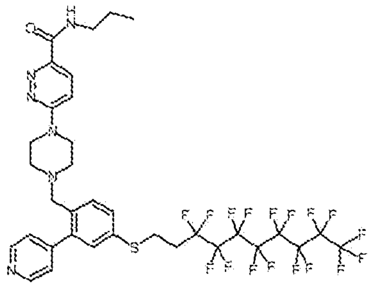 | 895.1 | 1.12 |
| AE031 | 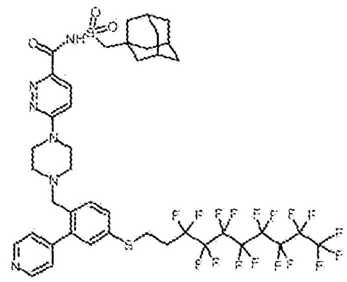 | 1065.2 | 1.31 |
| AE0102 | 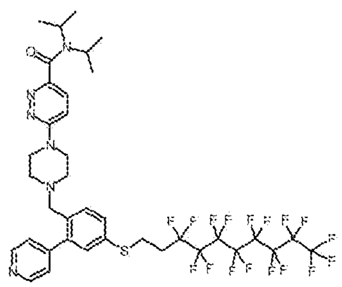 | 937.2 | 1.15 |

The LCMS results obtained after a lapse of 1 hour in the condensation step are shown in Table AE-01.

[Table 46]

| [Table AE-01] | | | | | | | | |
|---|---|---|---|---|---|---|---|---|
| Run | Condensing agent (eq.) | Base 1 (eq.) | Base 2 (eq.) | LCUV area % (319 nm) | | | | |
| | | | | SM (AE013) | IM (AE101) | TM (AE031) | Amide form (AE102) | Other |
| 1 | HATU (1.5) | DIPEA (5.0) | BTMG (3.0) | 19.0 | 11.6 | 66.6 | 2.8 | |

(continued)

| [Table AE-01] | | | | | | | | |
|---|---|---|---|---|---|---|---|---|
| Run | Condensing agent (eq.) | Base 1 (eq.) | Base 2 (eq.) | LCUV area % (319 nm) | | | | |
| | | | | SM (AE013) | IM (AE101) | TM (AE031) | Amide form (AE102) | Other |
| 2 | HATU (1.5) | DIPEA (5.0) | MTBD (3.0) | 23.9 | 20.0 | 44.3 | 2.1 | 9.6 |
| 3 | HATU (1.5) | DIPEA (5.0) | Phosphazene PltBu (3.0) | 22.3 | ND | 70.8 | 6.5 | |

In the case of using phosphazene PltBu as base 2, formed active ester (detected as IM (AE101) by quenching with n-$PrNH_2$) was consumed, and 70.8 area% of the target compound was able to be formed. The structures of IM and an amide form are shown below.

The results of this experiment demonstrated that use of HATU as a condensing agent for Ar-COOH can circumvent by-products observed using a carbodiimide condensing agent. The results further demonstrated that use of phosphazene PltBu as base 2 to be added after activation of carboxylic acid enables sulfonamide to be coupled at high yields.

Example 2-8-2: Study of base for use in step of forming active ester

An exemplary problem and solution thereto found in the course of the optimization of the conditions mentioned above will be shown. An operation given below was carried out using Ar-COOH AE032 and (4-chlorophenyl)methanesulfonamide AE033. Hereinafter, the case of run 4 in Table AE-02 will be shown as one example.

[Formula 362]

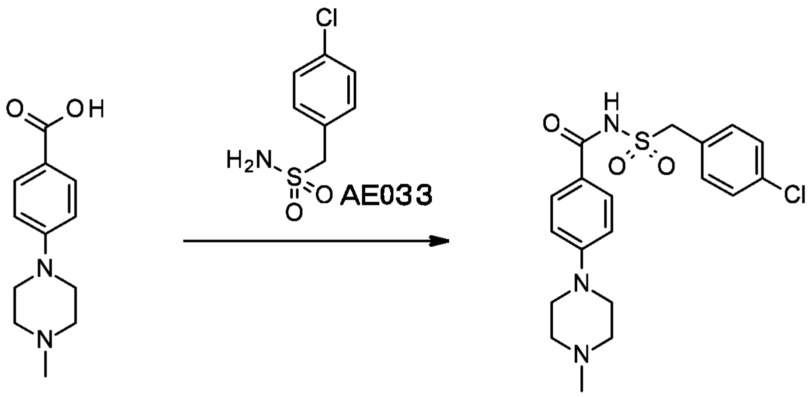

AE033

AE032

AE034

AE032 (CAS: 86620-62-4, 15.0 mg, 1.0 equivalent), NMP (300 μL, 20 v/w), 2,6- lutidine (7.9 μL, 1.0 equivalent), and HATU (12.7 mg, 6.0 equivalents) were added to a 5 mL screw-cap vial, purged with nitrogen, and then stirred at room temperature for 1 hour and 20 minutes. Then, (4-chlorophenyl)methanesulfonamide (AE033) (CAS: 71799-35-4, 35.0 mg, 2.5 equivalents) and phosphazene P1tBu (173 μL, 10 equivalents) were added thereto, and the mixture was shaken at room temperature for 1 hour.

Solvents and reagents used in the other runs and the amounts thereof used are shown in Table AE-02. The reaction was monitored as follows.

3 μL of the reaction solution was sampled, then discharged to n-$PrNH_2$ (30 μL) in a 1.5 mL glass vial, and quenched. After pipetting several times, the mixture was diluted with MeCN to prepare an LC sample. The degree of progression of the reaction was measured by LC-MS analysis. The following compound to be detected was analyzed under analysis conditions SMD-TFA05-1, and the UV area% was confirmed at a UV wavelength of 319 nm.

[Formula 363]

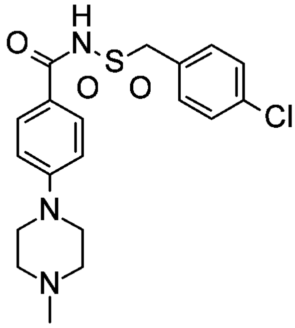

TM AE034

LRMS: m/z 408.0[M+H]+.

Retention time: 0.79 min (analysis conditions SMD-TFA05-1).

[Formula 364]

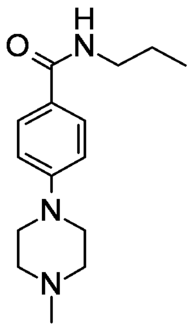

IM (AE103)

LRMS: m/z 262.1[M+H]+.

Retention time: 0.54 min (analysis conditions SMD-TFA05-1).

[Formula 365]

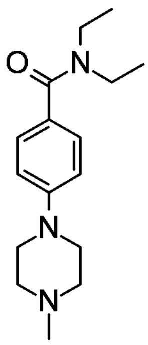

di-Et amide form (AE104)

LRMS: m/z 276.1[M+H]+.

Retention time: 0.60 min (analysis conditions SMD-TFA05-1).

The putative reaction pathway of this reaction is shown in formula AE001. The reaction is presumed to progress at two stages where Ar-COOH (SM) is converted to active ester by the action of HATU and a base and subsequently to the target compound (TM) by the addition of phosphazene PltBu together with sulfonamide. After sampling, the resultant is dipped in n-$PrNH_2$ so that remaining active ester is converted to an amide form (IM) by the progression of amidation. When tertiary amine such as triethylamine is used, the formation of a di-Et amide form is observed.

[Formula 366]

[Formula AE001]

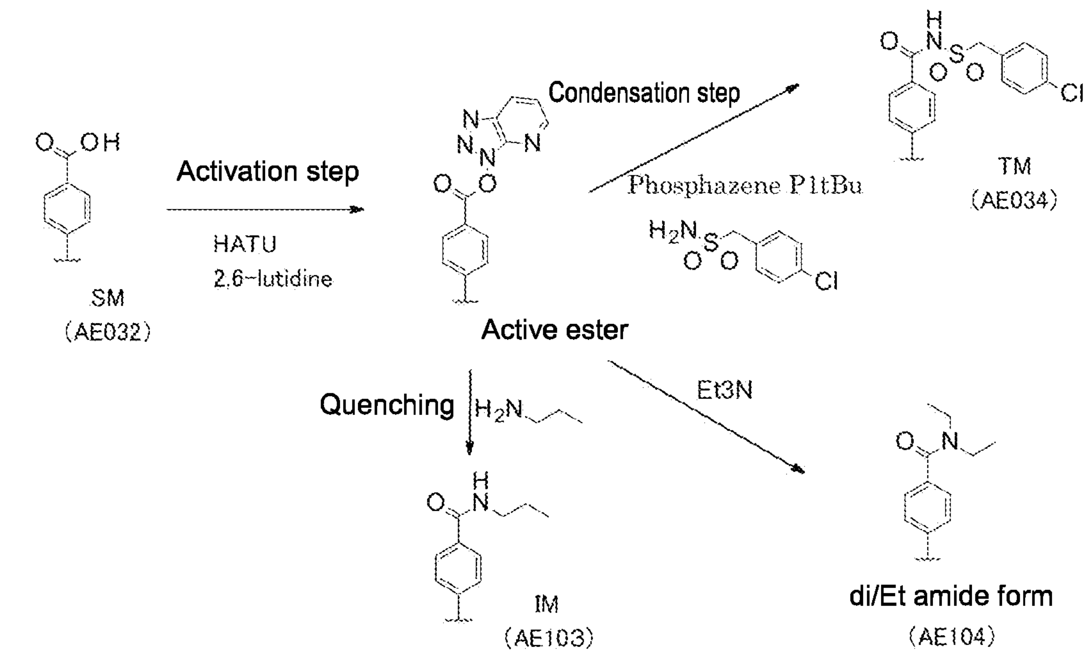


The LCMS results obtained after a lapse of 1 hour in the condensation step are shown in Table AE-02.

[Table 47]

| [Table AE-02] | | | | | | | | |
|---|---|---|---|---|---|---|---|---|
| | Table AE-02 | | | | | | | |
| | | | | UV area% (319 nm) | | | | |
| Run | Base (eq.) | DMAP (eq.) | Solvent | SM (AE032) | IM (AE103) | TM (AE034) | di-Etamide form (AE104) | others |
| 1 | TEA (20) | 1 | DCM | nd | nd | 91.3 | 5.2 | 1 |
| 2 | - | 2.5 | DCM | nd | nd | 83.4 | nd | 16.6 |
| 3 | 2,6-lutidine (2.5) | - | DCM | 1.8 | nd | 96.8 | nd | 1.4 |
| 4 | 2,6-lutidine (2.5) | - | NMP | 1.7 | nd | 98.9 | nd | 1.1 |
| | nd = not detected | | | | | | | |

In the case of using TEA as a base, a problem was the marked formation of a di-Et amide form. The formation of the di-Et amide form was circumvented by changing the base used in the activation step to alkylamine. Particularly, in the case of using 2,6-lutidine as a base and NMP as a solvent, the target compound in a acylsulfonamide form was confirmed at high yields, and side reaction was reduced.

The results of this experiment demonstrated that the approach of the present invention enables acylsulfonamidation reaction to proceed at high yields by using HATU as a condensing agent and 2,6-lutidine as a base for Ar-COOH, activating carboxylic acid, and then adding sulfonamide together with a strong base such as phosphazene P1tBu.

Example 2-8-3: Application of sulfonamide coupling to solid phase and determination of conditions

Solid-phase reaction was further improved by applying the conditions found by the liquid-phase experiment in Example 2-8-2 to the solid-phase reaction. The solid-phase experiment was carried out by an operation given below using solid-phase supported Ar- COOH AE019-01R and sulfonamide AE033. Hereinafter, the case of run 4 in Table AE-03 will be shown as one example.

[Formula 367]

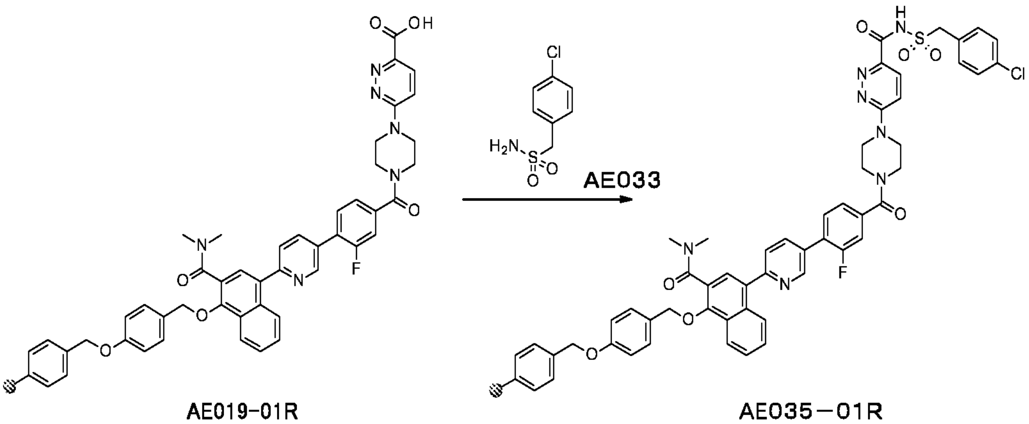


AE019-01R AE035−01R

Under a nitrogen atmosphere, solid-phase supported Ar-COOH (AE019-01R) (0.37 mmol/g, 15.1 mg, 1.0 equivalent) and NMP (300 μL, 20 v/w) were added to a 0.6 mL screw-cap vial and shaken at room temperature for 1 hour. Under a nitrogen atmosphere, HATU (12.7 mg, 6.0 equivalents) and 2,6-lutidine (3.9 μL, 3.0 equivalents) were added thereto, and the mixture was shaken at 40°C for 3 hours. Then, (4- chlorophenyl)methanesulfonamide (AE033) (CAS: 71799-35-4, 6.9 mg, 6.0 equivalents) and phosphazene PltBu (18.4 μL, 13 equivalents) were added thereto, and the mixture was shaken at room temperature for 1 hour.

Reaction temperatures and the numbers of equivalents of reagents in the other runs are shown in Table AE-03. The reaction was monitored as follows.

After a lapse of 1 and 18 hours from the start of reaction, under a nitrogen atmosphere, 10 μL of the suspension of the reaction solution and the resin was sampled and then discharged to n-$PrNH_2$ (30 μL) in a 1.5 mL glass vial. After pipetting several times, the mixture was transferred to a pipette tip with a filter, washed three times with NMP (100 μL), three times with MeOH (100 μL), and three times with DCM (100 μL), and then dipped in a 10% solution of TFA in DCM containing 0.05 M trimethylbenzene (50 μL) for 5 minutes. After filtration, the filtrate was dissolved in NMP (250μL), and the solution (50 μL) was diluted with MeCN (250 μL) to prepare an LC sample. The degree of progression of the reaction was measured by LC-MS analysis. The following compound to be detected was analyzed under analysis conditions SMD-AC05-1, and the UV area% was confirmed at a UV wavelength of 319 nm.

[Formula 368]

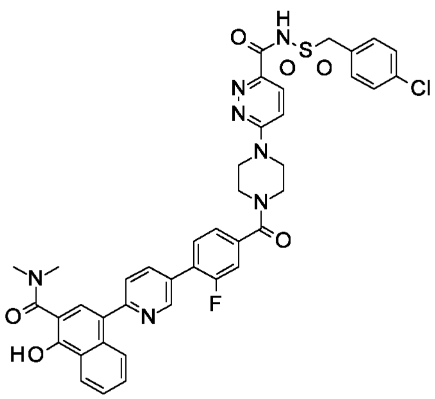

TM (AE035)

LRMS: m/z 808.1[M+H]$^+$.

Retention time: 0.89 min (analysis conditions SMD-AC05-1).

Retention time of the starting material (AE019): 0.74 min (analysis conditions SMD-AC05-1).

[Formula 369]

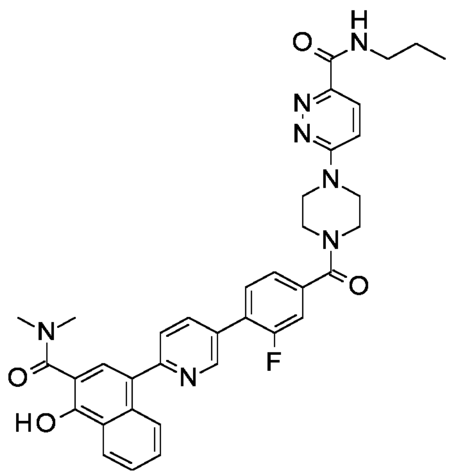

IM (AE105)

LRMS: m/z 662.2[M+H]+.

Retention time: 1.05 min (analysis conditions SMD-AC05-1).

The putative reaction pathway of this reaction will be shown below. The reaction is presumed to progress at two stages where Ar-COOH (SM) is converted to active ester by the action of HATU and 2,6-lutidine and subsequently to the target compound (TM) by the addition of phosphazene PltBu together with sulfonamide. After sampling, the resultant is dipped in n-$PrNH_2$ so that remaining active ester is converted to an amide form (IM) by the progression of amidation.

[Formula 370]

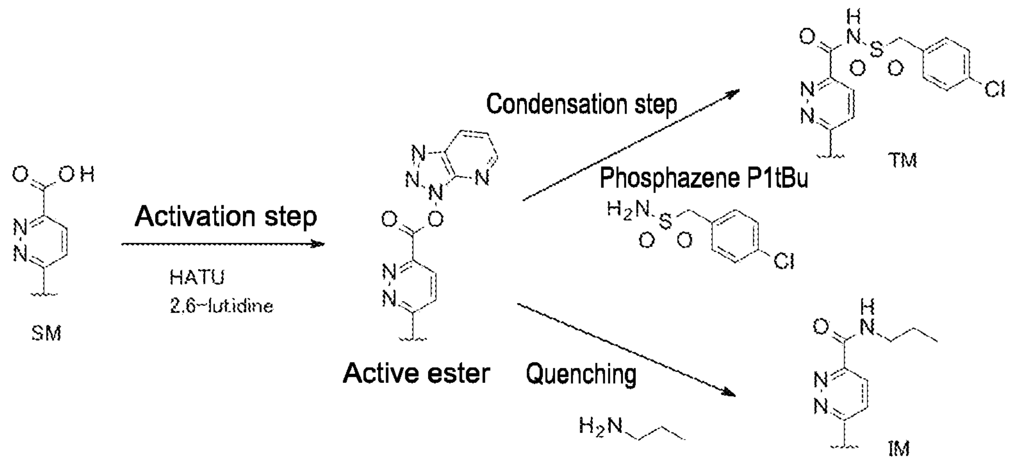


The LCMS results obtained after a lapse of 3 hours (run 1 and 2) and 1 hour (run 3 and 4) in the condensation step are shown in Table AE-03.

[Table 48]

| [Table AE-03] | | | | | | | | |
|---|---|---|---|---|---|---|---|---|
| Table AE-03 | | | | | | | | |
| Run | Activation step | HATU | 2,6-lutidine | Sulfonamide | Phosphazene P1tBu | LCUV area % (319 nm) | | |
| | Temperature | (eq.) | (eq.) | (eq.) | (eq.) | SM (AE019) | IM (AE105) | TM (AE035) |
| 1 | rt | 3.0 | 3.0 | 3.0 | 10.0 | 3.2 | 1.0 | 95.0 |
| 2 | 40 | 3.0 | 3.0 | 3.0 | 10.0 | 2.4 | 0.2 | 95.2 |
| 3 | 40 | 3.0 | 6.0 | 3.0 | 10.0 | 1.9 | 1.1 | 95.3 |
| 4 | 40 | 6.0 | 6.0 | 6.0 | 13.0 | 0.5 | 0.1 | 98.7 |

In the case of carrying out an activation step at 40°C and using HATU (6.0 eq.), 2,6- lutidine (6.0 eq.), sulfonamide (6.0 eq.), and phosphazene P1tBu (13 eq.) (run 4), 98.1 area% of the target compound in an acylsulfonamide form was confirmed.

The results of this experiment demonstrated that the approach of the present invention also enables acylsulfonamidation reaction to be performed at high yields through solid-phase reaction by using HATU as a condensing agent and 2,6-lutidine as a base for solid-phase supported Ar-COOH, activating carboxylic acid, and then adding sulfonamide together with a strong base such as phosphazene P1tBu.

Example 2-8-4: Method for suppressing reduction in purity by converting remaining reaction intermediate to library component

When the conversion rate through reaction is not sufficient, a "high-quality" library can be supplied with contamination by unintended compounds reduced if a starting material or an active intermediate can be converted to another compound contained in the mixture library. Therefore, this approach can be very important.

Hereinafter, an exemplary approach of circumventing the return of a remaining active intermediate during acylsulfonamidation reaction to a starting material by suitable treatment, and consequently preventing increase in the amount of impurities other than library components will be shown. Specifically, in the illustrated approach, after reaction with a low reactive building block, a more highly reactive building block to be contained in a mixture library is added into the reaction system so that an active intermediate is converted to a library component. This can reduce the contamination of the mixture library by impurities such as unintended unreacted matter or active intermediates. Hereinafter, an exemplary experiment using AE022-01R as carboxylic acid, AE049 as a sulfonamide building block, and AE023 as a more highly reactive amine building block will be shown. Here, AE049 and AE023 simulated building blocks for use in mixture library preparation by the split & pool method. AE022-01R was synthesized by the method described in Example 1-6-12.

[Formula 371]

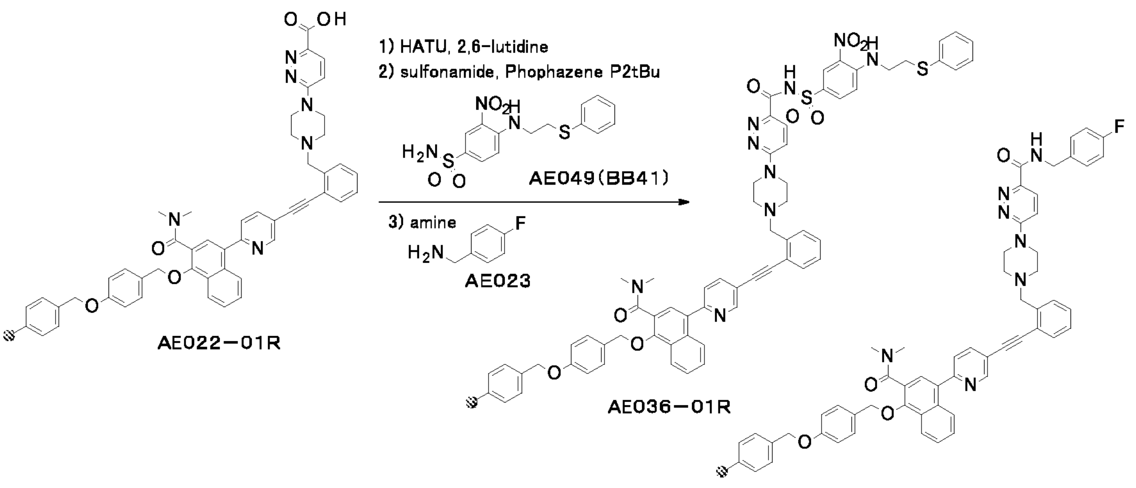

AE022−01R
AE036−01R
AE063−01R

Under a nitrogen atmosphere, solid-phase supported Ar-COOH (AE022-01R) (0.37 mmol/g, 6.9 mg) and NMP (138 μL, 20 v/w) were added to a 0.6 mL screw-cap vial and shaken at room temperature for 1 hour. Under a nitrogen atmosphere, HATU (5.8 mg, 6.0 equivalents) and 2,6-lutidine (1.8 μL, 6.0 equivalents) were added thereto, and the mixture was shaken at 40°C for 3 hours. Then, sulfonamide derivative BB41 (CAS: 406233- 13-4, 6.3 mg, 7.0 equivalents) and phosphazene P1tBu (9.7 μL, 15 equivalents) were added thereto, and the mixture was shaken at room temperature for 3 hours. Then, (4- fluorophenyl)methanamine (AE023) (CAS: 140-75-0, 2.92 μL, 10 equivalents) was added thereto, and the mixture was shaken at room temperature for 15 hours. The reaction was monitored as follows.

After a lapse of a given time, under a nitrogen atmosphere, 10 μL of the resin suspension was sampled and then discharged to n-$PrNH_2$ (30 μL) in a 1.5 mL glass vial. After pipetting several times, the mixture was transferred to a pipette tip with a filter, washed three times with NMP (100 μL), three times with MeOH (100 μL), and three times with DCM (100 μL), and then dipped in a 10% solution of TFA in DCM containing 0.05 M trimethylbenzene (50 μL) for 5 minutes. After filtration, the filtrate was dissolved in NMP (250 μL), and the solution (50 μL) was diluted with MeCN (250 μL) to prepare an LC sample. The degree of progression of the reaction was measured by LC-MS analysis. The following compound to be detected was analyzed under analysis conditions SMD-FA05-1 or SMD-AC05-1, and the UV area% was confirmed at a UV wavelength of 319 nm.

[Formula 372]

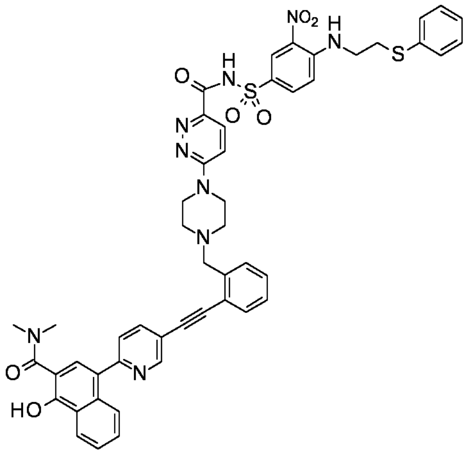

TM (AE036)

LRMS: m/z 948.3[M+H]+.

Retention time: 1.13 min (analysis conditions SMD-FA05-1).

Retention time: 1.17 min (analysis conditions SMD-AC05-1).

[Formula 373]

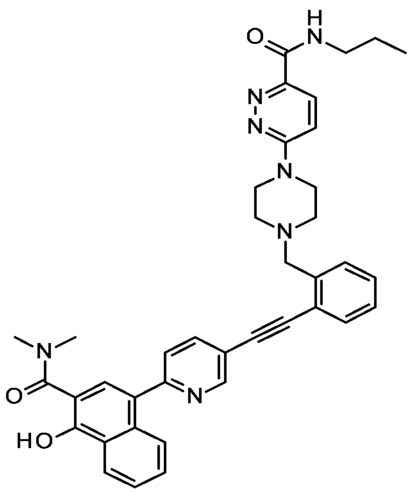

IM (AE064)

LRMS: m/z 654.3[M+H]+.

Retention time: 0.88 min (analysis conditions SMD-FA05-1).

[Formula 374]

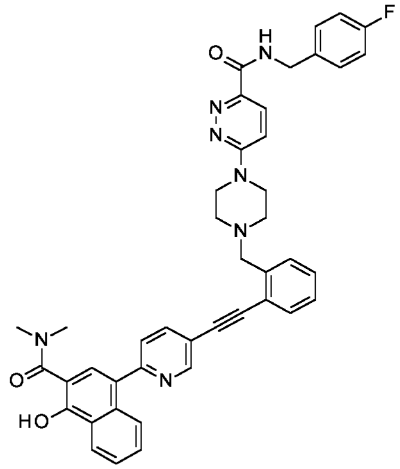

TM2 (AE063)

LRMS: m/z 720.1[M+H]$^+$.

Retention time: 1.47 min (analysis conditions SMD-AC05-1).

The LCMS results obtained after a lapse of each given time are shown in Table AE- 04. The putative reaction pathway of this reaction will be shown below. The reaction progresses at two stages where Ar-COOH (SM) is converted to active ester by the action of HATU and 2,6-lutidine and subsequently to the target compound (TM) by the addition of phosphazene P1tBu together with sulfonamide. After sampling, the resultant is dipped in n- $PrNH_2$ so that remaining active ester is converted to an amide form (IM) by the progression of amidation. The remaining active ester is converted to a product (TM2) when reacted with a more highly reactive building block.

[Formula 375]

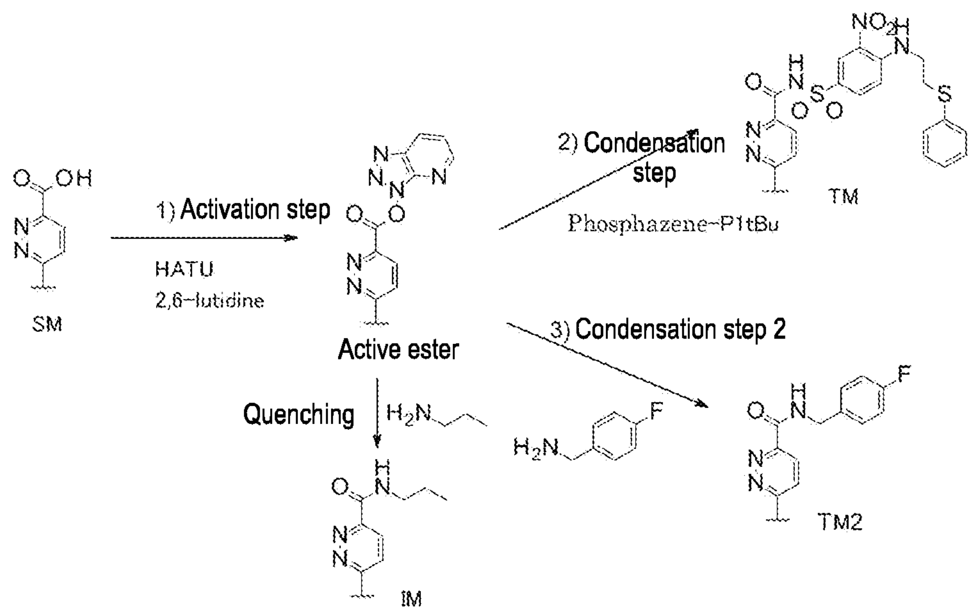

[Table 49]

| [Table AE-04] | | | | | |
|---|---|---|---|---|---|
| Table AE-04 | | | | | |
| | Reaction time | TM | TM2 | IM | SM |
| First step | 3 hr | nd | - | 97.9 | nd |
| Second step | 3 hr | 94.6 | - | 1.4 | 2.4 |
| Third step | 15 hr | 94.3 | 1.7 | nd | 0.5 |
| nd = not detected | | | | | |

From the LC results indicating the activation for 3 hours in the first step, 97.9 area% of IM was observed. From the LC activation results obtained after a lapse of 3 hours in the second step, 94.6 area% of TM was confirmed. On the other hand, 1.4 area% of IM was observed, indicating that reaction in the condensation step was not completed so that activated ester remained. From the LC activation results obtained after a lapse of 15 hours in the third step, IM was not observed, and 1.7 area% of TM2 was observed instead. This indicated that the remaining activated ester was converted to TM2 through reaction with more highly reactive building block AE023.

These results of the present invention demonstrated that the suitable treatment of a remaining reaction intermediate can circumvent its return to a starting material and prevent increase in the amount of impurities other than library components. This approach of the present invention is not limited by the acylsulfonamidation illustrated in this Example, and is an approach widely applicable to every reaction for suppressing the content of matter other than a designed compound in order to supply a "high-quality" library.

Example 2-8-5: Identification of scope of sulfonamide application

Acylsulfonamidation reaction was carried out under the conditions found in Example A-E2, 3 using sulfonamides AE037, AE038, and AE040 to AE049 shown in Table AE-05 to confirm the scope of building blocks applicable to library synthesis.

Hereinafter, the operation of the reaction of AE029-01R with AE037 (BB36) will be shown as an exemplary experiment.

[Formula 376]

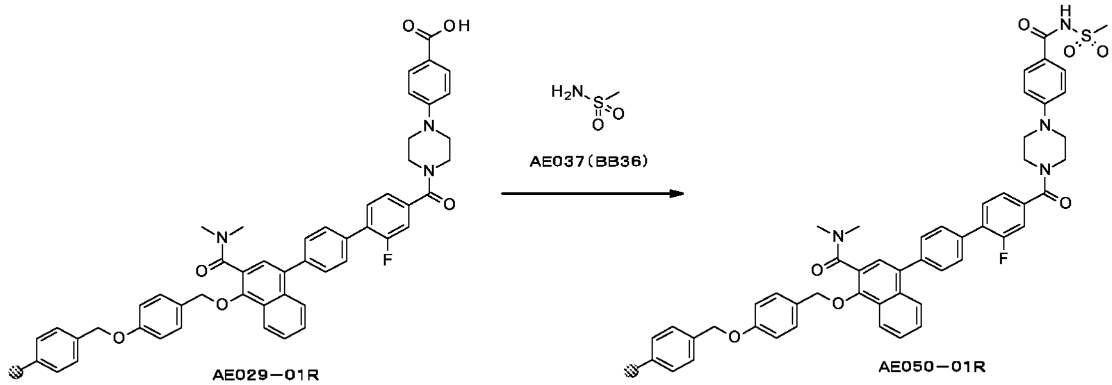


AE029−01R AE050−01R

Under a nitrogen atmosphere, solid-phase supported Ar-COOH (AE029-01R) (0.40 mmol/g, 14.8 mg, 1.0 equivalent) and NMP (221 μL, 15 v/w) were added to a 0.6 mL screw-cap vial and shaken at room temperature for 1 hour. Under a nitrogen atmosphere, HATU (13.5 mg, 6.0 equivalents) and 2,6-lutidine (4.1 μL, 6.0 equivalents) were added thereto, and the mixture was shaken at 40°C for 3 hours. Then, methanesulfonamide (AE037) (BB36, 5.6 mg, 7.0 equivalents) and phosphazene P1tBu (22.5 μL, 15 equivalents) were added thereto, and the mixture was shaken at 40°C for 18 hours.

The amounts of solid-phase supported Ar-COOH and each reagent used in the experiments using the other sulfonamides are shown in Table AE-05. The compound number and structure of the target compound obtained from each sulfonamide are shown in Table AE-06. The reaction was monitored as follows.

After a lapse of a given time, under a nitrogen atmosphere, 10 μL of the resin suspension was sampled and

then discharged to n-Pr$NH_2$ (20 μL) in a 1.5 mL glass vial. After pipetting several times, the mixture was transferred to a pipette tip with a filter, washed three times with NMP (100 μL), three times with MeOH (100 μL), and three times with DCM (100 μL), and then dipped in a 10% solution of TFA in DCM containing 0.1 M trimethylbenzene (50 μL) for 5 minutes. After filtration, the filtrate was dissolved in NMP (250 μL), and the solution (50 μL) was diluted with MeCN (250 μL) to prepare an LC sample. The degree of progression of the reaction was measured by LC-MS analysis.

[Table 50]

[Table AE-05]

Table AE-05

| entrey | Compound No. in this chapter | Compound No. in other chapters | Structural formula of sulfonamide | Molecular weight | Amount of Ar-COOH resin used (mg) | mmol | Amount of NMP used (uL) | Amount of HATU used (mg) | Amount of lutidine used (mg) | Amount of sulfonamide used (mg) | Amount of P1-tBu used (uL) |
|---|---|---|---|---|---|---|---|---|---|---|---|
| 1 | AE037 | BB36 | 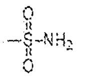 | 95.12 | 14.8 | 0.0059 | 221 | 13.5 | 4.1 | 5.6 | 22.5 |
| 2 | AE038 | BB37 | 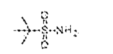 | 137.2 | 15.4 | 0.0062 | 231 | 14.1 | 4.3 | 8.5 | 23.5 |
| 3 | AE040 | BB38 | 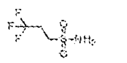 | 177.14 | 15.2 | 0.0061 | 229 | 13.9 | 4.3 | 10.8 | 23.3 |
| 4 | AE041 | | 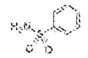 | 157.19 | 17.6 | 0.0070 | 264 | 16.1 | 4.9 | 11.1 | 26.9 |
| 5 | AE042 | | 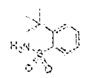 | 213.3 | 16.4 | 0.0066 | 246 | 15.0 | 4.6 | 14.0 | 25.0 |
| 6 | AE043 | | 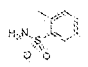 | 171.21 | 16.4 | 0.0065 | 246 | 14.9 | 4.6 | 11.2 | 25.0 |
| 7 | AE044 | BB39 | 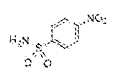 | 202.19 | 15.0 | 0.0060 | 225 | 13.7 | 4.2 | 12.1 | 22.9 |
| 8 | AE045 | | 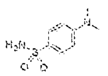 | 200.25 | 16.1 | 0.0064 | 241 | 14.7 | 4.5 | 12.9 | 24.5 |
| 9 | AE046 | | 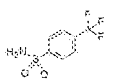 | 225.19 | 16.1 | 0.0064 | 241 | 14.7 | 4.5 | 14.5 | 24.5 |
| 10 | AE047 | | 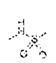 | 109.15 | 16.4 | 0.0065 | 245 | 14.9 | 4.6 | #REF! | 25.0 |
| 11 | AE048 | BB40 | 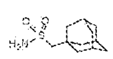 | 229.11 | 15.1 | 0.0060 | 226 | 13.8 | 4.2 | 13.8 | 23.0 |
| 12 | AE049 | BB41 | 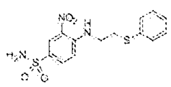 | 353.05 | 15.6 | 0.0063 | 235 | 14.3 | 4.4 | 22.1 | 23.9 |

[Table 51]

[Table AE-06]

Table AE-06

| entrey | Compound No. in this chapter | Structure of sulfonamide | Compound No. of compound of interest | Structure of compound of interest |
|---|---|---|---|---|
| 1 | AE037 | 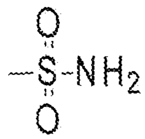 | AE050-01R | 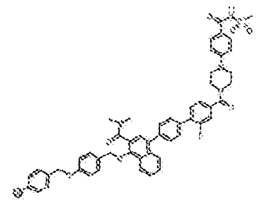 |
| 2 | AE038 | 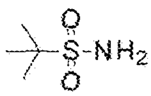 | AE051-01R | 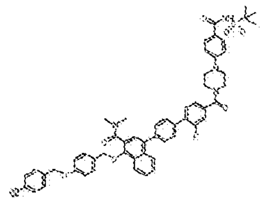 |
| 3 | AE040 | 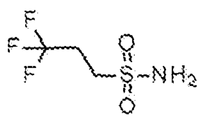 | AE053-01R | 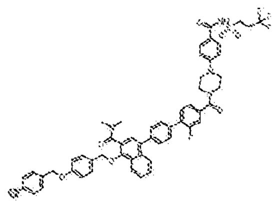 |
| 4 | AE041 | 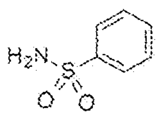 | AE054-01R | 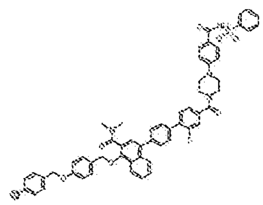 |
| 5 | AE042 | 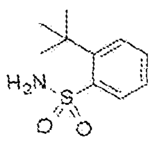 | AE055-01R | 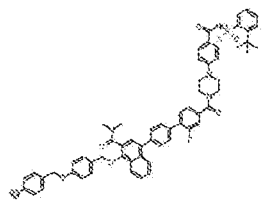 |

[Table 52]

| | | | | |
|---|---|---|---|---|
| 6 | AE043 | 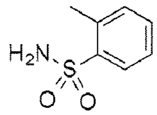 | AE056-01R | 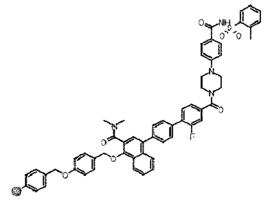 |
| 7 | AE044 | 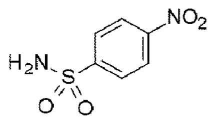 | AE057-01R | 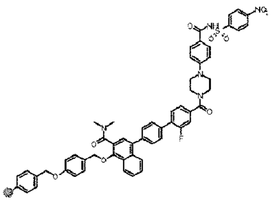 |
| 8 | AE045 | 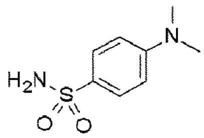 | AE058-01R | 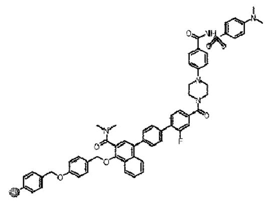 |
| 9 | AE046 | 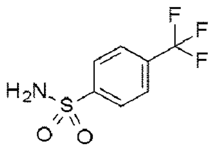 | AE059-01R | 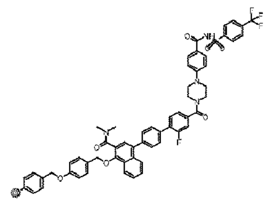 |
| 10 | AE047 | 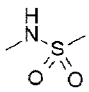 | AE060-01R | 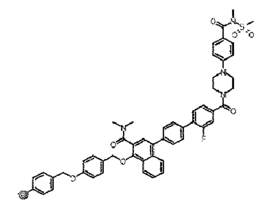 |

[Table 53]

| | | | | |
|---|---|---|---|---|
| 11 | AE048 | 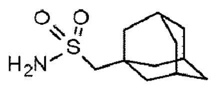 | AE061-01R | 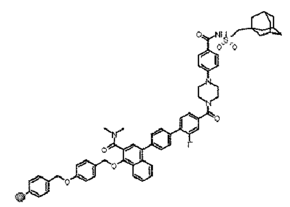 |
| 12 | AE049 | 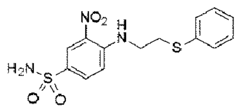 | AE062-01R | 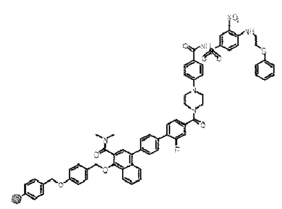 |

The LCMS results obtained after a lapse of 18 hours are shown in Table AE-07. In the case of using sulfonamide AE037, AE038, AE040, AE043, AE044, AE046, AE048, or AE049, 93.1 area% or more of the target compound in an

acylsulfonamide form was confirmed.

[Table 54]

| [Table AE-07] | | | | |
|---|---|---|---|---|
| Table AE-07 | | | | |
| entrey | Compound No. of target compound to be monitored | Observed MS m/z [M+H]+ | Retention time (min) | Area % (319 nm) |
| 1 | AE050 | 695.2 | 1.21 | 96.8 |
| 2 | AE051 | 737.3 | 1.30 | 94.4 |
| 3 | AE053 | 777.2 | 1.33 | 97.2 |
| 4 | AE054 | 757.2 | 1.32 | 8.0 |
| 5 | AE055 | 813.4 | 1.48 | 44.2 |
| 6 | AE056 | 771.2 | 1.36 | 93.5 |
| 7 | AE057 | 802.2 | 1.36 | 94.4 |
| 8 | AE058 | 800.4 | 1.34 | 15.1 |
| 9 | AE059 | 825.2 | 1.43 | 93.1 |
| 10 | AE060 | nd | nd | 0.0 |
| 11 | AE061 | 829.3 | 1.57 | 98.8 |
| 12 | AE062 | 953.3 | 1.48 | 98.1 |

From the results of this experiment, conditions were found under which reaction proceeded at high yields by using HATU as a condensing agent and 2,6-lutidine as a base for solid-phase supported Ar-COOH, activating carboxylic acid, and then adding sulfonamide together with a strong base such as phosphazene P1tBu. The scope of substrate application of sulfonamide was identified. Also, the acylsulfonamidation reaction by the approach of the present invention was found to have a practical level applicable to library synthesis even through solid-phase reaction. The substrate scope for sulfonamidation is not limited to the scope confirmed in this Example, and the scope of substrates that can be applied may be expanded by additional experimental study or from results about actual mixture library synthesis.

Example 2-8-6: Expansion of binding orientation

Acylation reaction was carried out for solid-phase supported sulfonamide under the reaction conditions found in Example 2-8-3 to expand the binding orientation of an acylsulfonamide linker.

Hereinafter, the operation of the acylation reaction of A127-03R using A128 will be shown as an exemplary experiment.

[Formula 377]

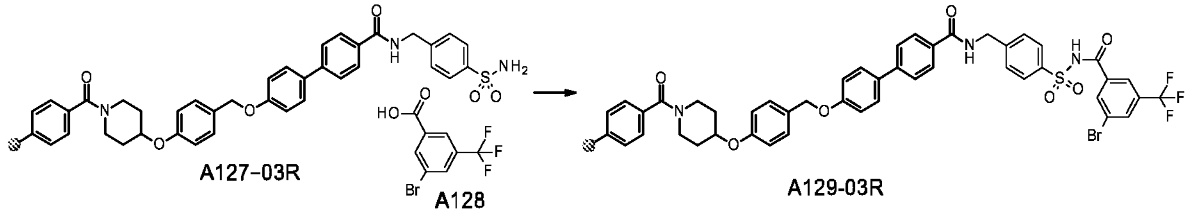

A127-03R A128 A129-03R

Under a nitrogen atmosphere, 3-bromo-5-(trifluoromethyl)benzoic acid A128 (4.2 mg, 16 μmol), NMP (140 μL), 2,6-lutidine (2.7 μL, 23 μmol), and HATU (8.9 mg, 23 μmol) were added to a 0.6 mL glass vial and stirred at 40°C for 1 hour.

Under a nitrogen atmosphere, compound A127-03R (loading rate: 0.194 mmol/g, 20 mg) and NMP (160 μL) were added to a 0.6 mL glass vial and shaken at room temperature for 1 hour. Then, the reaction solution prepared

above and phosphazene P1tBu (9.9 μL, 39 μmol) were added thereto, and the mixture was shaken at room temperature for 24 hours.

Reaction monitoring

15 μL of the resin suspension was sampled, then transferred to a pipette tip with a filter, washed three times with DMF (100 μL), three times with MeOH (100 μL), and three times with DCM (100 μL), and then dipped in a 10% solution of TFA in DCM containing 0.05 M pentamethylbenzene (50 μL) for 5 minutes. After filtration, the filter was washed with NMP (100 μL), and combined solutions (100 μL) were diluted with MeCN (250 μL). 98.2% of compound A129 of interest was observed by LC-MS analysis.

[Formula 378]

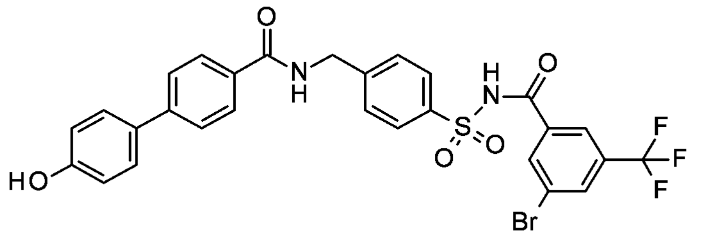

Compound A129

LRMS: m/z 633, 635[M+H]+

Retention time: 1.179 min (analysis conditions FA05-1, 299 nm).

From the results of this experiment, a method for forming an acylsulfonamide linker through the acylation reaction of solid-phase supported sulfonamide was identified. Diversity was imparted to the orientation of an acylsulfonamide linker by this method added to the method for forming a linker from solid-phase supported carboxylic acid through acylsulfonamidation, indicating the diversity of linkers applicable to library synthesis.

Example 2-9: Liquid-phase experiment to confirm scope of substrate application to sp2-sp3 coupling reaction as typical example of identifying substrate scope that can be applied to library synthesis

Hereinafter, an exemplary method for identifying an applicable substrate scope under reaction conditions expected to attain a wide scope of substrate application to sp2-sp3 coupling reaction will be illustrated. The scope given below is a mere illustration of an exemplary identification method, and the present invention is not limited by the scope given below in actual application to library synthesis.

Example 2-9-1: Solid-phase experiment of Suzuki coupling reaction of solid-phase supported Ar-Br with alkylboronic acid

Suzuki coupling reaction with alkylboronic acid was carried out with reference to a literature known in the art (Org. Lett. 2015, 17, 3370-3383.) by selecting an organic base P2tBu as a base and RuPhos Pd G4 as a Pd catalyst.

Example 2-9-1-1: Coupling reaction (C201-03R) of solid-phase supported ArBr (C008-03R) with alkylboronic acid pinacol ester (C301)

[Formula 379]

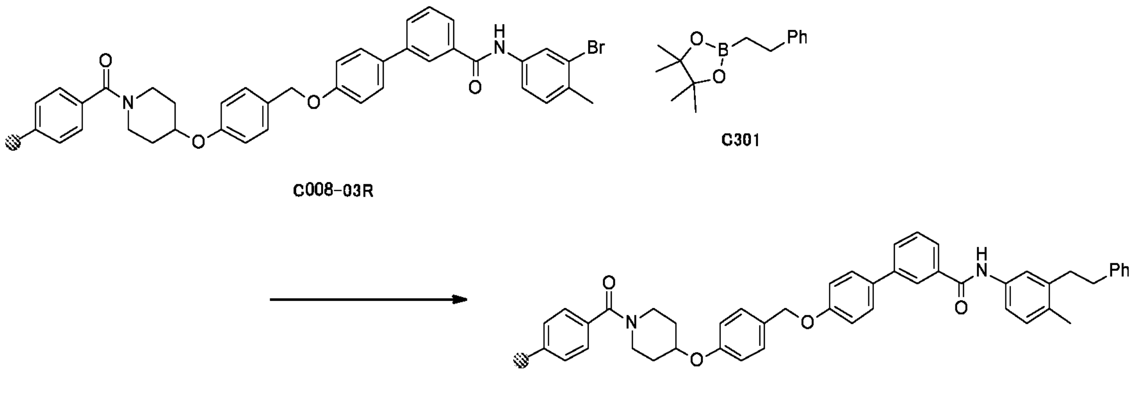

C008-03R

C301

C201-03R

Under a nitrogen atmosphere, solid-phase supported Ar-Br (C008-03R) (loading rate: 0.471 mmol/g, 20 mg, 0.0094 mmol), THF (0.3 mL), and water (1.7 μL, 0.094 mmol) were added to a 0.6 mL glass vial and shaken at room temperature for 1 hour. 4,4,5,5- Tetramethyl-2-(2-phenylethyl)-1,3,2-dioxaborolane (C301) (13 mg, 0.056 mmol), RuPhos Pd G4 (1.6 mg, 0.0019 mmol), and P2tBu (2 M, a solution in THF, 47 μL, 0.094 mmol) were added thereto, and the mixture was shaken at 80°C for 24 hours.

The suspension of the reaction solution and the resin (0.010 mL) was transferred onto a filter, washed three times with NMP (0.1 mL), three times with MeOH (0.1 mL), and three times with DCM (0.1 mL), and then dipped in a 10% solution of TFA in DCM containing 0.2 M pentamethylbenzene (0.05 mL) for 2 minutes. After filtration, NMP (0.25 mL) was added to the filtrate. MeCN (0.25 mL) was added to a 0.050 mL aliquot of the obtained solution to prepare an LC sample. The degree of progression of the reaction was measured by LC-MS analysis. As a result, 66% of compound C201 of interest was observed.

[Formula 380]

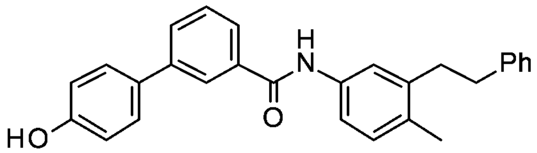

Compound C201

LRMS: m/z 408[M+H]+.

Retention time: 1.351 min (analysis conditions SMD-FA05-1, 280 nm).

Example 2-9-1-2: Coupling reaction (C202-03R) of solid-phase supported ArBr (C008-03R) with alkyl 9-BBN prepared before use

[Formula 381]

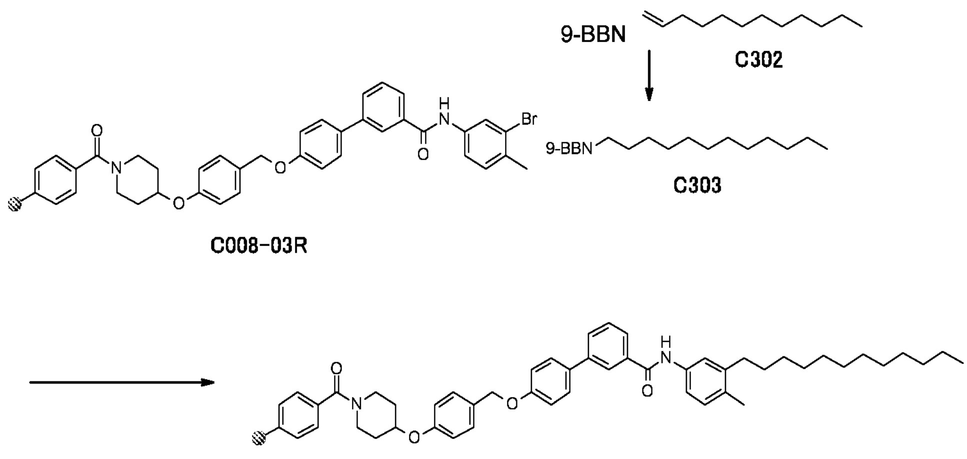


C302

C303

C008-03R

C202-03R

Under a nitrogen atmosphere, solid-phase supported Ar-Br (C008-03R) (loading rate: 0.471 mmol/g, 20 mg, 0.0094 mmol) and THF (0.3 mL) were added to a 0.6 mL glass vial and shaken at room temperature for 1 hour. 9-Dodecyl-9-borabicyclo[3.3.1]nonane (C303) (mixture of 9-BBN (0.5 M, a solution in THF, 113 μL, 0.056 mmol) and 1-dodecene (C302) (13 μL, 0.056 mmol) left standing overnight), RuPhos Pd G4 (1.6 mg, 0.0019 mmol), water (1.7 μL, 0.094 mmol), and P2tBu (2 M, a solution in THF, 47 μL, 0.094 mmol) were added thereto, and the mixture was shaken at 60°C for 30 minutes.

The suspension of the reaction solution and the resin (0.010 mL) was transferred onto a filter, washed three times with NMP (0.1 mL), three times with MeOH (0.1 mL), and three times with DCM (0.1 mL), and then dipped in a 10% solution of TFA in DCM containing 0.2 M pentamethylbenzene (0.05 mL) for 2 minutes. After filtration, NMP (0.25 mL) was added to the filtrate. MeCN (0.25 mL) was added to a 0.050 mL aliquot of the obtained solution to prepare an LC sample. The degree of progression of the reaction was measured by LC-MS analysis. As a result, 90% of compound C202 of interest was observed.

[Formula 382]

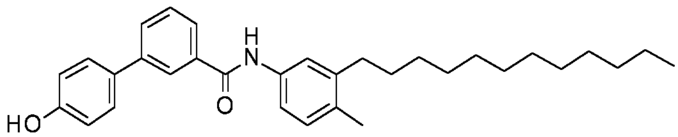

Compound C202

LRMS: m/z 472[M+H]$^+$.

Retention time: 1.852 min (analysis conditions SMD-FA05-1, 280 nm).

An example of changing the Pd catalyst so that sp2-sp3 Suzuki coupling reaction progressed using a milder organic base than P2tBu without impairing the functional group of ester, etc. will be shown.

Example 2-9-1-3: Coupling reaction (C205-03R) of solid-phase supported ArBr (C010-03R) with alkyl 9-BBN prepared before use

[Formula 383]

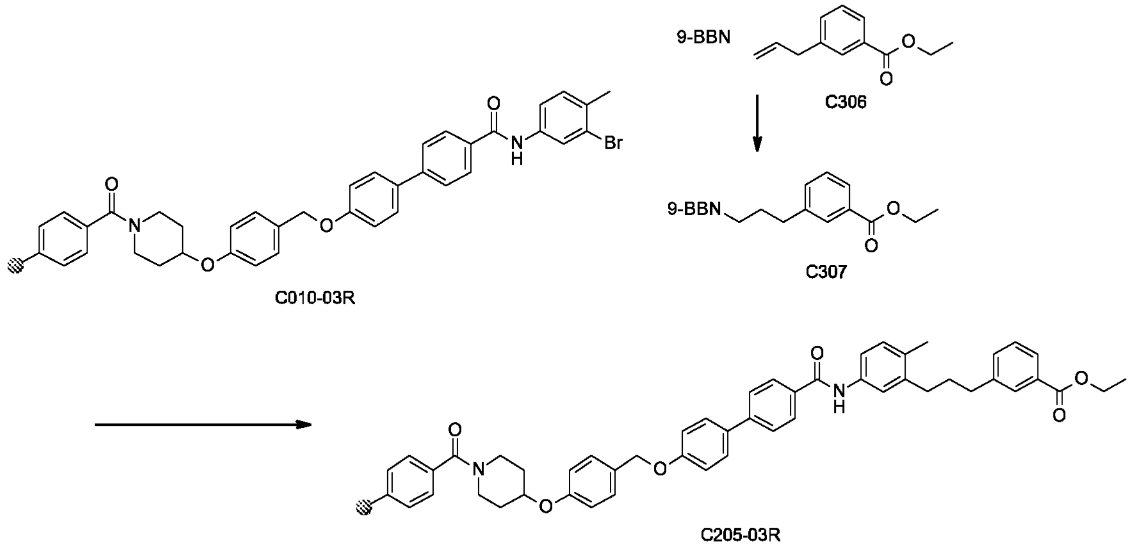


C306

C307

C010-03R

C205-03R

Under a nitrogen atmosphere, compound C010-03R (0.194 mmol/g, 20 mg) and THF (0.4 mL) were added to a 0.6 mL glass vial and shaken at room temperature for 1 hour. Water (1.05 μL, 0.058 mmol) and (dtbpf)PdCh (2.5 mg, 0.0039 mmol) were added thereto. Ethyl 3-(3-(9-borabicyclo[3.3.1]nonan-9-yl)propyl)benzoate (C307) (mixture of a solution of 9-BBN in THF (0.5 M, 78 μL, 0.039 mmol) and ethyl 3-allylbenzoate (C306) (7.4 mg, 0.039 mmol) left standing at room temperature for 1 hour) was added thereto. BTMG (11.6 μL, 0.058 mmol) was added thereto, and the mixture was shaken at 60°C for 30 minutes.

12 μL of the suspension of the reaction solution and the resin was transferred onto a tip with a filter and washed three times with DMF (0.1 mL), three times with MeOH (0.1 mL), and three times with DCM (0.1 mL). The resultant was dipped in a 10% solution of TFA in DCM containing pentamethylbenzene (0.1 M, 0.05 mL) for 2 minutes, filtered, and washed with DMF (0.05 mL). Combined filtrates were diluted with MeCN (0.25 mL), and the solution was subjected to LC-MS analysis, so that 98.9% of compound C205 of interest was observed. However, the compound was cleaved and analyzed at a wavelength of 299 nm (±4 nm).

[Formula 384]

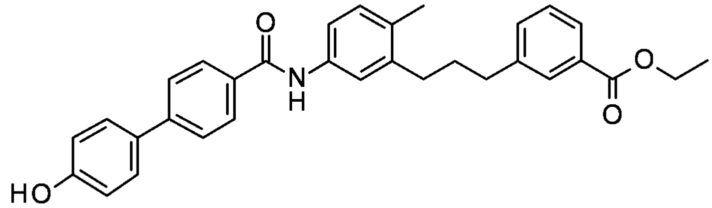

Compound C205

LRMS: m/z 494[M+H]$^+$

Retention time: 1.417 min (analysis conditions SMD-FA05-1)

From the results described above, it was confirmed that sp2-sp3 Suzuki coupling reaction can be carried out on the solid phase. Example 2-9-1-1 is an example of using alkylboronic acid obtained in advance, and Examples 2-9-1-2 and 2-9-1-3 are examples of preparing alkyl 9-BBN before use by the hydroboration of the corresponding alkene with 9- BBN, and using it in reaction. The latter method was confirmed, indicating that not only can available alkylboronic acid be used, but diverse compounds of interest can be induced through sp2-sp3 Suzuki coupling reaction as long as the corresponding alkene is available.

Example 2-9-2: Solid-phase experiment of Negishi coupling reaction of solid-phase supported Ar-Br with alkylzinc compound

Suzuki coupling reaction with an alkylzinc compound was carried out with reference to a literature known in the art (Chem. Commun. 2011, 47, 5181-5183) by selecting Pd- PEPPSI-IPent as a Pd catalyst.

Example 2-9-2-1: Coupling reaction (C203-03R) of solid-phase supported ArBr (C008-03R) with alkylzinc compound (C303)

[Formula 385]

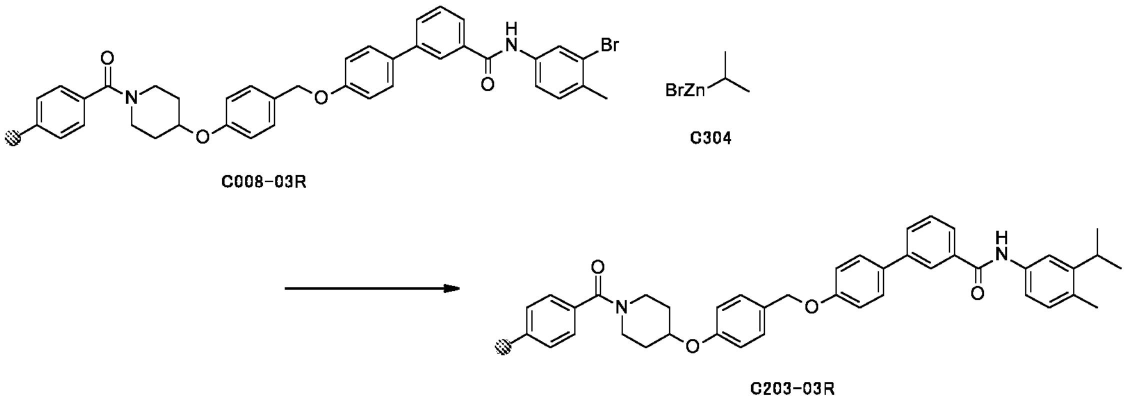

C008-03R

C304

C203-03R

Under a nitrogen atmosphere, solid-phase supported Ar-Br (C008-03R) (loading rate: 0.471 mmol/g, 20 mg, 0.0094 mmol) and THF (0.3 mL) were added to a 0.6 mL glass vial and shaken at room temperature for 1 hour. Zinc 2-propylbromide (C304) (0.5 M, a solution in THF, 113 μL, 0.056 mmol) and Pd-PEPPSI-IPent (1.5 mg, 0.0019 mmol) were added thereto, and the mixture was shaken at room temperature for 2 hours.

The suspension of the reaction solution and the resin (0.010 mL) was transferred onto a filter, washed three times with NMP (0.1 mL), three times with MeOH (0.1 mL), and three times with DCM (0.1 mL), and then dipped in a 10% solution of TFA in DCM containing 0.2 M pentamethylbenzene (0.05 mL) for 2 minutes. After filtration, NMP (0.25 mL) was added to the filtrate. MeCN (0.25 mL) was added to a 0.050 mL aliquot of the obtained solution to prepare an LC sample. The degree of progression of the reaction was measured by LC-MS analysis. As a result, 94% of compound C203 of interest was observed.

[Formula 386]

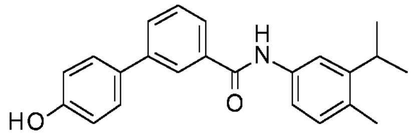

Compound C203

LRMS: m/z 346[M+H]+.

Retention time: 1.260 min (analysis conditions SMD-FA05-1, 280 nm).

From the results described above, sp2-sp3 Negishi coupling reaction can be carried out on the solid phase.

The results of Example 2-9-1 and Example 2-9-2 indicate that in the bonding of an aryl group to an alkyl group, i.e., in sp2-sp3 coupling, at least the reaction conditions described in this Example can be freely selected according to the reactivity or availability of a substrate.

Example 2-10: Liquid-phase experiment to confirm scope of substrate application to C-N coupling reaction as typical example of identifying substrate scope that can be applied to library synthesis

Hereinafter, an exemplary method for identifying an applicable substrate scope under reaction conditions expected to attain a wide scope of substrate application to C-N coupling reaction will be illustrated. The scope given below is a mere illustration of an exemplary identification method, and the present invention is not limited by the scope given below in actual application to library synthesis.

Example 2-10-1: Buchwald-Hartwig amination reaction of solid-phase supported Ar-Br with amine

Buchwald-Hartwig amination reaction with aliphatic amine was carried out with reference to a literature known in the art (Org. Lett. 2015, 17, 3370-3383) by selecting an organic base P2tBu or NaOtBu as a base and RuPhos Pd G4 as a Pd catalyst.

Example 2-10-1-1: Coupling reaction of solid-phase supported ArBr (C009-03R) with 4-phenylpiperidine (C501)

[Formula 387]

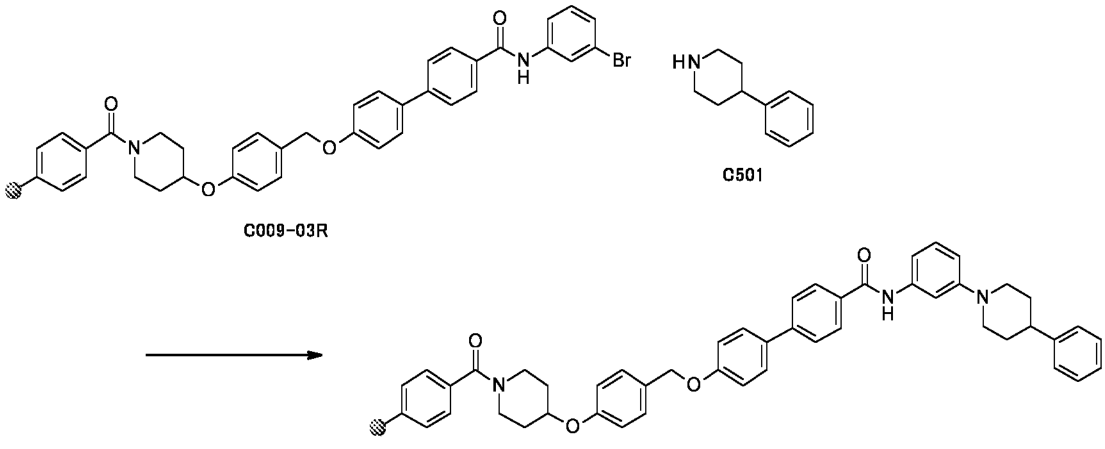

C009-03R

C501

C401-3R

Under a nitrogen atmosphere, solid-phase supported Ar-Br (C009-03R) (loading rate: 0.195 mmol/g, 20 mg, 0.0039 mmol) and THF (0.4 mL) were added to a 0.6 mL glass vial and shaken at room temperature for 1 hour. 4-Phenylpiperidine (C501) (13 mg, 0.078 mmol), RuPhos Pd G4 (0.66 mg, 0.00078 mmol), and P2tBu (2 M, a solution in THF, 59 μL, 0.117 mmol) were added thereto, and the mixture was shaken at 80°C for 30 minutes.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.2 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 94% of compound C401 of interest was observed.

[Formula 388]

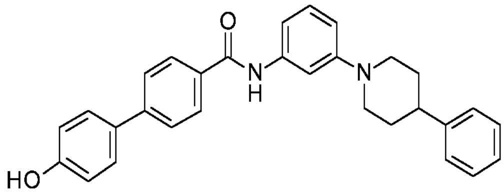

Compound C401

LRMS: m/z 449[M+H]$^+$.

Retention time: 1.115 min (analysis conditions SMD-FA05-1, 299 nm).

Example 2-10-1-2: Coupling reaction of solid-phase supported ArBr (C009-03R) with N-methyl-3-phenylpropan-1-amine (C502)

[Formula 389]

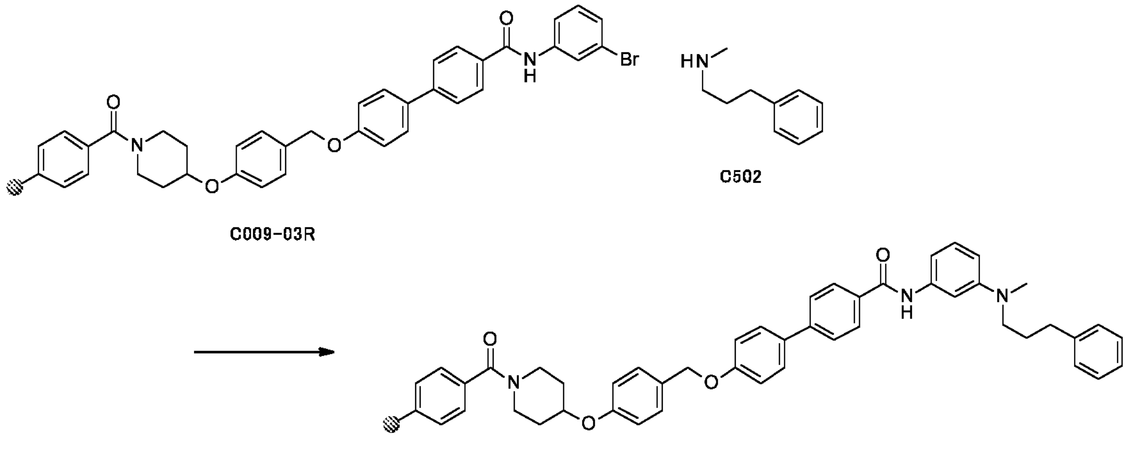

C009-03R

C502

C402-03R

Under a nitrogen atmosphere, solid-phase supported Ar-Br (C009-03R) (loading rate: 0.195 mmol/g, 20 mg, 0.0039 mmol) and THF (0.4 mL) were added to a 0.6 mL glass vial and shaken at room temperature for 1 hour. N-Methyl-3-phenylpropan-1-amine (C502) (12 mg, 0.078 mmol), RuPhos Pd G4 (0.66 mg, 0.00078 mmol), and P2tBu (2 M, a solution in THF, 59 μL, 0.117 mmol) were added thereto, and the mixture was shaken at 80°C for 2 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.2 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 85% of compound C402 of interest was observed.

[Formula 390]

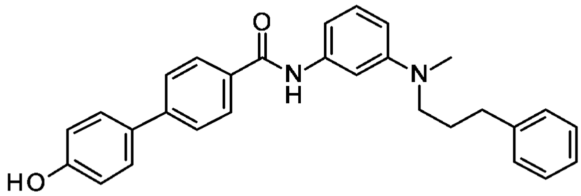

Compound C402

LRMS: m/z 437[M+H]+.

Retention time: 1.213 min (analysis conditions SMD-FA05-1, 299 nm).

Example 2-10-1-3: Coupling reaction of solid-phase supported ArBr (C010-03R) with 4-phenylpiperidine (C501)

[Formula 391]

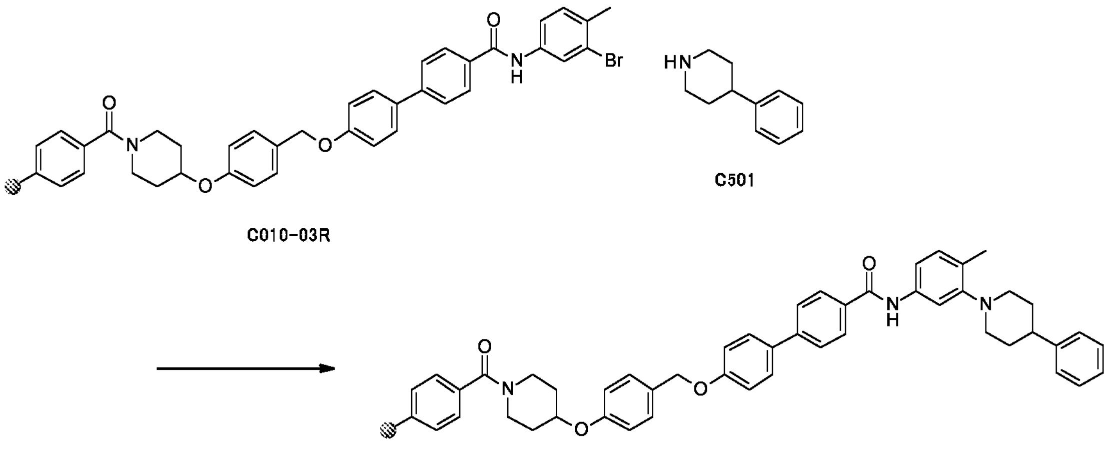

C501

C010-03R

C403-03R

Under a nitrogen atmosphere, solid-phase supported Ar-Br (C010-03R) (loading rate: 0.194 mmol/g, 20 mg, 0.0039 mmol) and THF (0.4 mL) were added to a 0.6 mL glass vial and shaken at room temperature for 1 hour. 4-Phenylpiperidine (C501) (13 mg, 0.078 mmol), RuPhos Pd G4 (0.66 mg, 0.00078 mmol), and NaOtBu (2 M, a solution in THF, 58 μL, 0.116 mmol) were added thereto, and the mixture was shaken at 80°C for 30 minutes.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.2 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 74% of compound C403 of interest was observed.

[Formula 392]

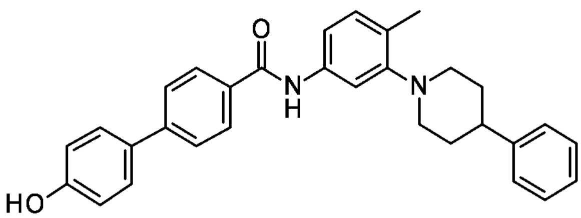

Compound C403

LRMS: m/z 463[M+H]$^+$.

Retention time: 1.344 min (analysis conditions SMD-FA05-1, 299 nm).

Example 2-10-1-4: Coupling reaction of solid-phase supported ArBr (C010-03R) with N-methyl-3-phenylpropan-1-amine (C502)

[Formula 393]

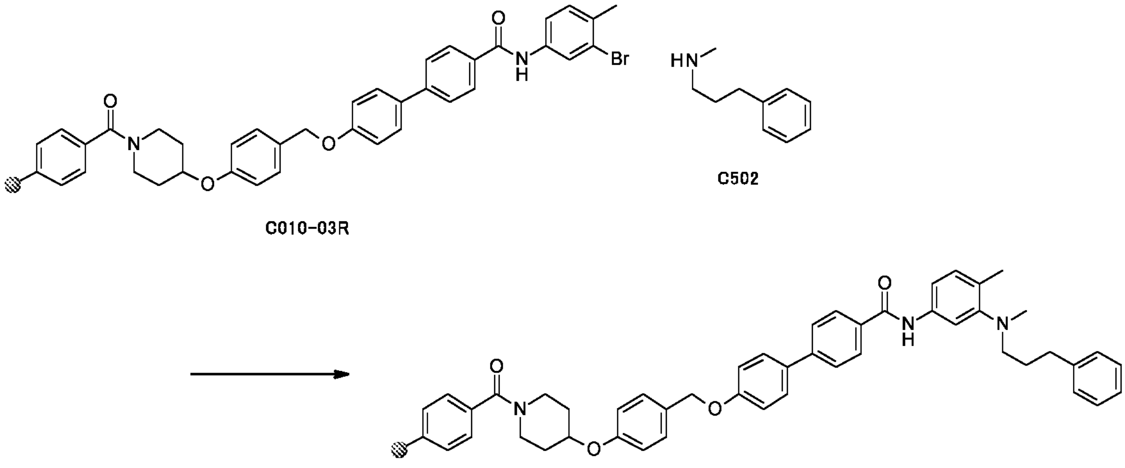

C502

C010-03R

C404-03R

Under a nitrogen atmosphere, solid-phase supported Ar-Br (C010-03R) (loading rate: 0.194 mmol/g, 20 mg, 0.0039 mmol) and THF (0.4 mL) were added to a 0.6 mL glass vial and shaken at room temperature for 1 hour. N-Methyl-3-phenylpropan-1-amine (C502) (12 mg, 0.078 mmol), RuPhos Pd G4 (0.66 mg, 0.00078 mmol), and NaOtBu (2 M, a solution in THF, 58 μL, 0.116 mmol) were added thereto, and the mixture was shaken at 80°C for 30 minutes.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.2 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 60% of compound C404 of interest was observed.

[Formula 394]

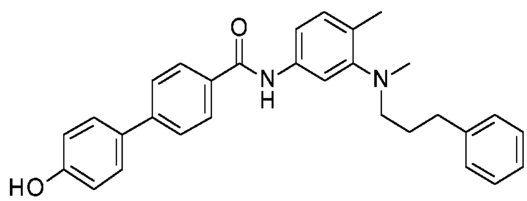

Compound C404

LRMS: m/z 451$[M+H]^+$.

Retention time: 1.021 min (analysis conditions SMD-FA05-1, 299 nm).

From the results described above, bond formation through Buchwald-Hartwig amination reaction was able to be performed on the solid phase for combinations of various substrates, and reaction conditions also applicable to mixture library synthesis were confirmed.

Example 2-10-2: C-N coupling reaction of solid-phase supported ArBr with aromatic amine

Hereinafter, exemplary bond formation reaction under reaction conditions expected to attain a wide scope of substrate application to the C-N coupling reaction of solid-phase supported ArBr with aromatic amine or heteroaromatic amine will be presented. The substrate is not limited to those given below in actual application to library synthesis.

Example 2-10-2-1: Synthesis of C405-03R by coupling of solid-phase supported ArBr (C010-03R) and 4-(methylamino)benzonitrile (C503)

[Formula 395]

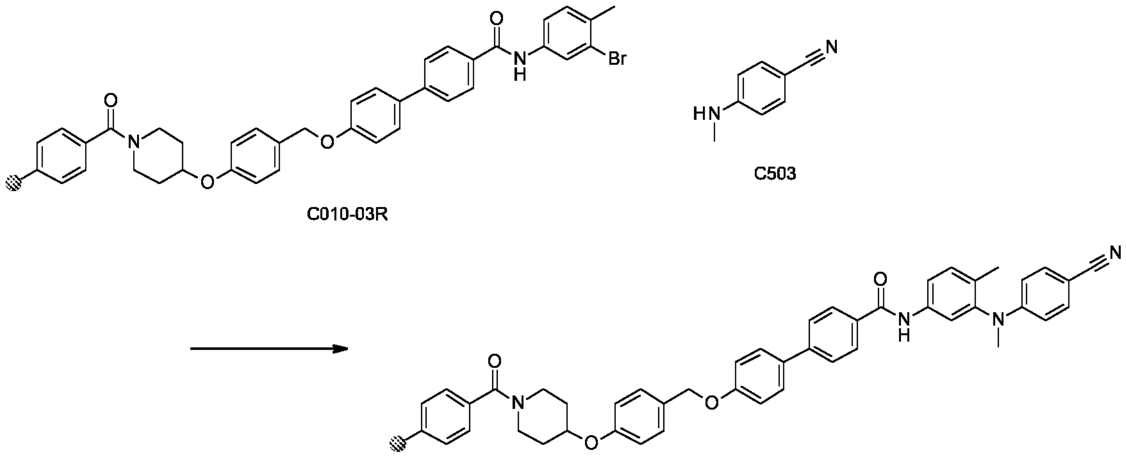

C010-03R

C503

C405-03R

Under a nitrogen atmosphere, compound C010-03R (0.194 mmol/g, 20 mg), 4- (methylamino)benzonitrile (C503) (10.3 mg, 0.078 mmol), and THF (0.4 mL) were added to a 0.6 mL glass vial and shaken at room temperature for 1 hour. (tBu)PhCPhos Pd G4 (0.6 mg, 0.00078 mmol) and a solution of P2tBu in THF (2.0 M, 58.2 μL, 0.116 mmol) were added thereto, and the mixture was shaken at 60°C for 30 minutes.

12 μL of the suspension of the reaction solution and the resin was transferred onto a tip with a filter and washed three times with DMF (0.1 mL), three times with MeOH (0.1 mL), and three times with DCM (0.1 mL). The resultant was dipped in a 10% solution of TFA in DCM containing pentamethylbenzene (0.1 M, 0.05 mL) for 2 minutes, filtered, and washed with DMF (0.05 mL). Combined filtrates were diluted with MeCN (0.25 mL), and the solution was subjected to LC-MS analysis, so that 89.5% of compound C405 of interest was observed. However, the compound was cleaved and analyzed at a wavelength of 299 nm (±4 nm).

[Formula 396]

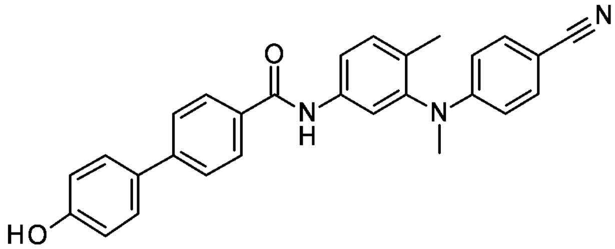

Compound C405

LRMS: m/z 434[M+H]+

Retention time: 2.708 min (analysis conditions SMD-FA05-long)

Example 2-10-2-2: Synthesis of C406-03R by coupling of solid-phase supported ArBr (C009-03R) and ethyl 2-(methylamino)thiazole-4-carboxylate (C504)

[Formula 397]

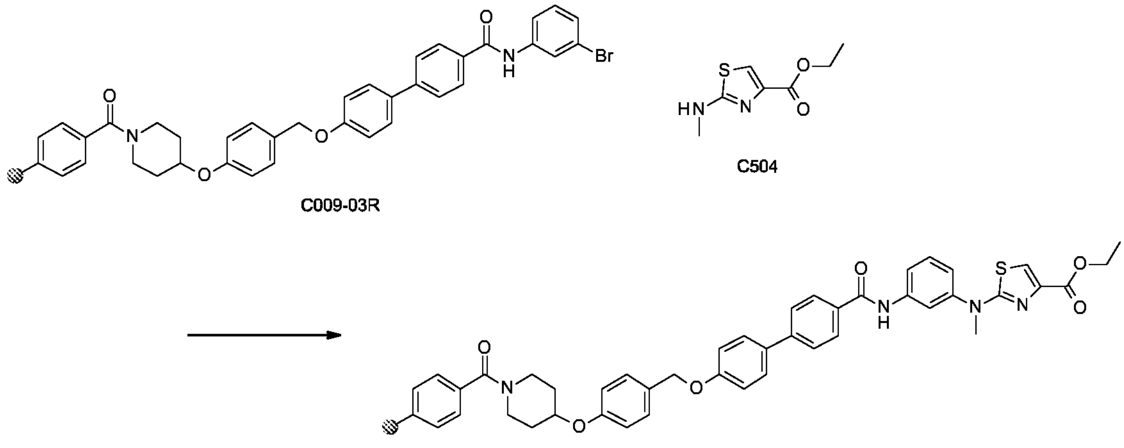

C009-03R C504 C406-03R

Under a nitrogen atmosphere, compound C009-03R (0.195 mmol/g, 20 mg), ethyl 2- (methylamino)thiazole-4-carboxylate (C504) (7.3 mg, 0.039 mmol), and THF (0.4 mL) were added to a 0.6 mL glass vial and shaken at room temperature for 1 hour. $Pd_2(dba)_3$-$CHCl_3$ (2.0 mg, 0.0020 mmol), AdBippyPhos (5.2 mg, 0.0078 mmol), potassium trifluoromethanesulfonate (3.7 mg, 0.020 mmol), and BTMG (11.7 μL, 0.059 mmol) were added thereto, and the mixture was shaken at 60°C for 30 minutes.

12 μL of the suspension of the reaction solution and the resin was transferred onto a tip with a filter and washed three times with DMF (0.1 mL), three times with MeOH (0.1 mL), and three times with DCM (0.1 mL). The resultant was dipped in a 10% solution of TFA in DCM containing pentamethylbenzene (0.1 M, 0.05 mL) for 2 minutes, filtered, and washed with DMF (0.05 mL). Combined filtrates were diluted with MeCN (0.25 mL), and the solution was subjected to LC-MS analysis, so that 97.7% of compound C406 of interest was observed. However, the compound was cleaved and analyzed at a wavelength of 299 nm (±4 nm).

[Formula 398]

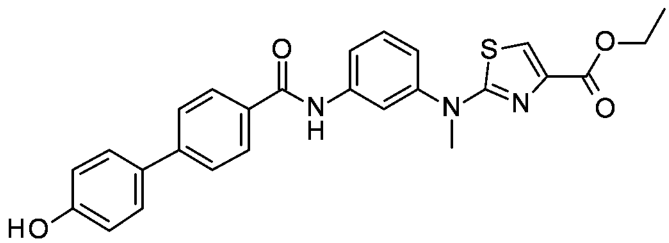

Compound C406

LRMS: m/z 474$[M+H]^+$

Retention time: 1.129 min (analysis conditions SMD-FA05-1)

From the results of Example 2-10-2, C-N coupling reaction with aromatic amine or heteroaromatic amine was found applicable to solid-phase supported ArBr.

Example 2-10-3: C-N coupling reaction of solid-phase supported amine with aryl bromide

Hereinafter, exemplary bond formation reaction under reaction conditions expected to attain a wide scope of substrate application to the C-N coupling reaction of solid-phase supported amine with aryl bromide will be presented. The substrate is not limited to those given below in actual application to library synthesis.

Example 2-10-3-1: Synthesis of C407-03R by coupling of solid-phase supported amine (A137-03R) and ethyl 5-bromothiophene-2-carboxylate (C801)

[Formula 399]

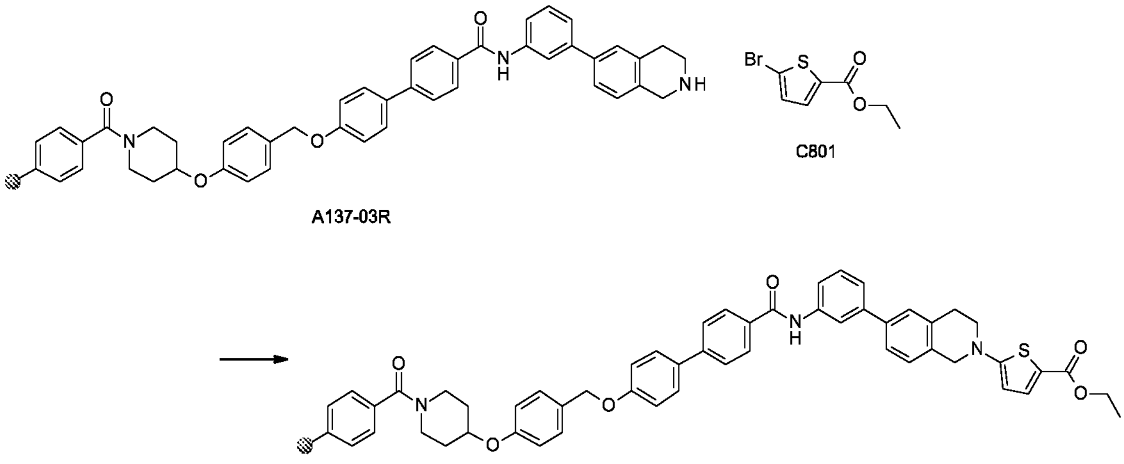

A137-03R

C801

C407-03R

Under a nitrogen atmosphere, compound A137-03R (0.193 mmol/g, 20 mg) and NMP (0.4 mL) were added to a 0.6 mL glass vial. Ethyl 5-bromothiophene-2-carboxylate (5.9 μL, 0.039 mmol) was added thereto, and the mixture was shaken at room temperature for 1 hour. RuPhos Pd G4 (3.3 mg, 0.0039 mmol) and a solution of P2tBu in THF (2 M, 77.0 μL, 0.058 mmol) were added thereto, and the mixture was shaken at 40°C for 24 hours.

12 μL of the suspension of the reaction solution and the resin was transferred onto a tip with a filter and washed three times with DMF (0.1 mL), three times with MeOH (0.1 mL), and three times with DCM (0.1 mL). The resultant was dipped in a 10% solution of TFA in DCM containing pentamethylbenzene (0.1 M, 0.05 mL) for 2 minutes, filtered, and washed with DMF (0.05 mL). Combined filtrates were diluted with MeCN (0.25 mL), and the solution was subjected to LC-MS analysis, so that 69.8% of compound C407 of interest was observed. However, the compound was cleaved and analyzed at a wavelength of 299 nm (±4 nm).

[Formula 400]

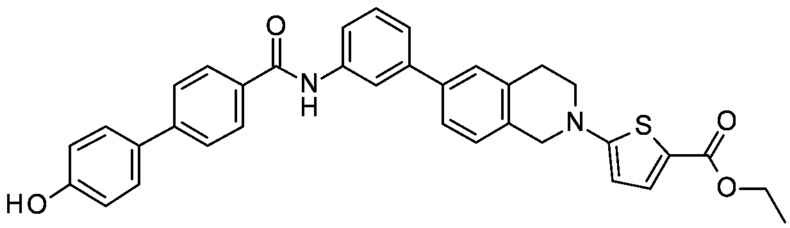

Compound C407

LRMS: m/z 575$[M+H]^+$

Retention time: 1.427 min (analysis conditions SMD-FA05-1)

From the results of Example 2-10-3, C-N coupling reaction with aryl bromide was found applicable to solid-phase supported amine.

Example 2-10-4: Solid-phase experiment of C-N bond formation reaction of solid- phase supported ArBr with 5-membered ring HetAr using Cu reagent

C-N bond formation reaction with ethyl pyrazole-3-carboxylate was carried out with reference to a non patent literature (J. Am. Chem. Soc. 2001, 123, 7727) by selecting an organic base MTBD (7-methyl-1,5,7-triazabicyclo[4.4.0]dec-5-ene) as a base, selecting CuI as a Cu catalyst, and selecting DMEDA (N,N'-dimethylethylenediamine) as a ligand.

Example 2-10-4-1: Coupling reaction of solid-phase supported ArBr (C009-03R) with ethyl pyrazole-3-carboxylate (B321)

[Formula 401]

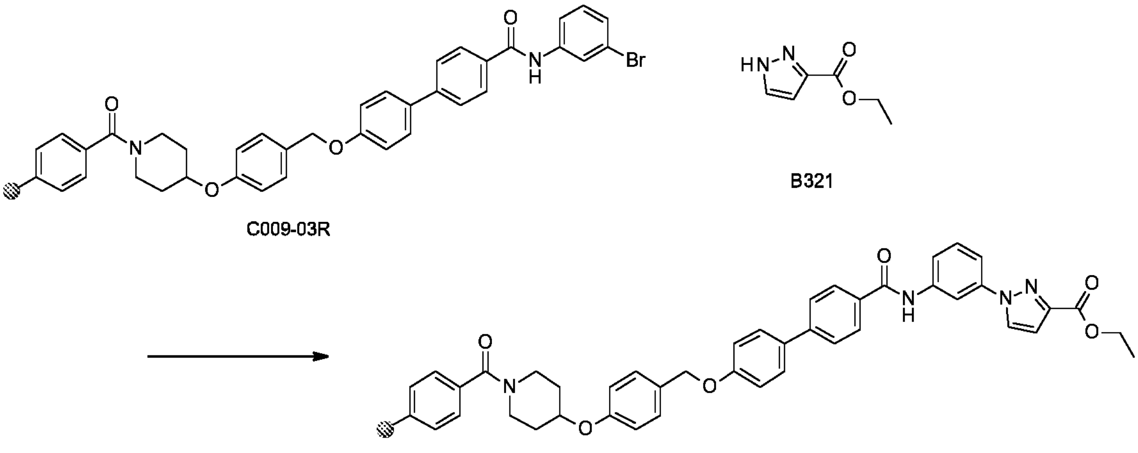

C009-03R

B321

B226-03R

Under a nitrogen atmosphere, solid-phase supported ArBr (C009-03R) (loading rate: 0.195 mmol/g, 20 mg, 0.0039 mmol) and DMF (0.4 mL) were added to a 0.6 mL glass vial and shaken at room temperature for 1 hour. Ethyl pyrazole-3-carboxylate (B321) (13.7 mg, 0.098 mmol), CuI (7.4 mg, 0.039 mmol), DMEDA (8.4 μL, 0.078 mmol), and MTBD (14 μL, 0.098 mmol) were added thereto, and the mixture was shaken at 110°C for 12 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 77% of compound B226 of interest was observed.

[Formula 402]

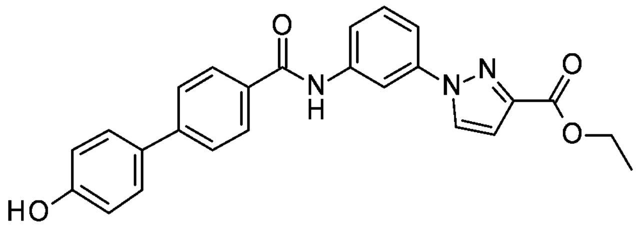

Compound B226

LRMS: m/z 428[M+H]$^+$.

Retention time: 1.096 min (analysis conditions SMD-FA05-1, 299 nm).

From these results, it was confirmed that the C-N bond formation reaction of N-H of HeteroAr with solid phase ArBr can be carried out using CuI.

Example 2-11: Experiment to confirm scope of substrate application to sulfonamidation reaction of amines as typical example of identifying substrate scope that can be applied to library synthesis

Hereinafter, an exemplary method for identifying an applicable substrate scope under reaction conditions expected to attain a wide scope of substrate application to sulfonamidation reaction will be illustrated. The scope given below is a mere illustration of an exemplary identification method, and the present invention is not limited by the scope given below in actual application to library synthesis.

Solid-phase supported Ar-NHz in this Example refers to a resin on which a compound having Ar-$NH_2$ as a substructure is supported (e.g., D001-03R). Likewise, solid- phase supported Ar-NHMe refers to a resin on which a compound having Ar-NHMe as a substructure is supported (e.g., D020-03R). Solid-phase supported alkylamine refers

to a resin on which a compound having alkylamine as a substructure is supported (e.g., D021- 03R).

Example 2-11-1: Solid-phase experiment to confirm conditions under which product is obtained at high purity through sulfonamidation reaction of solid-phase supported Ar-NHz with sulfonyl chloride

The sulfonamidation of solid-phase supported Ar-NHz was carried out with reference to literatures known in the art (Eur. J. Org. Chem. 2012, 764-771; and Eur. J. Med. Chem. 2018, 143, 48-65) using THF as a solvent and pyridine or triethylamine as a base. In this respect, the overreaction of sulfonyl chloride to aniline was of concern, as shown in a literature known in the art (Chem. Pharm. Bull. 1999, 47, 809-812). Accordingly, in order to find reaction conditions that were able to suppress overreaction to a wide range of solid- phase supported Ar-NHz, reaction conditions were studied using sulfonyl chloride presumed to have high reactivity. Specifically, the sulfonamidation of solid-phase supported Ar-$NH_2$ (D001-03R) was carried out using 4-nitrobenzenesulfonyl chloride (D002) having a nitro group serving as an electron-withdrawing group on the aromatic ring. The reaction results of performing the sulfonamidation of solid-phase supported Ar-NHz (D001-03R) with 4- nitrobenzenesulfonyl chloride (D002) using THF as a solvent and pyridine or triethylamine as a base are shown in Table 2-11-1. Hereinafter, the case of the experimental operation using pyridine as a base (run 2 in Table 2-11-1) will be shown.

Sulfonamidation reaction (exemplary reaction operation) of solid-phase supported Ar-NHz (D001-03R) with 4-nitrobenzenesulfonyl chloride (D002)

[Formula 403]

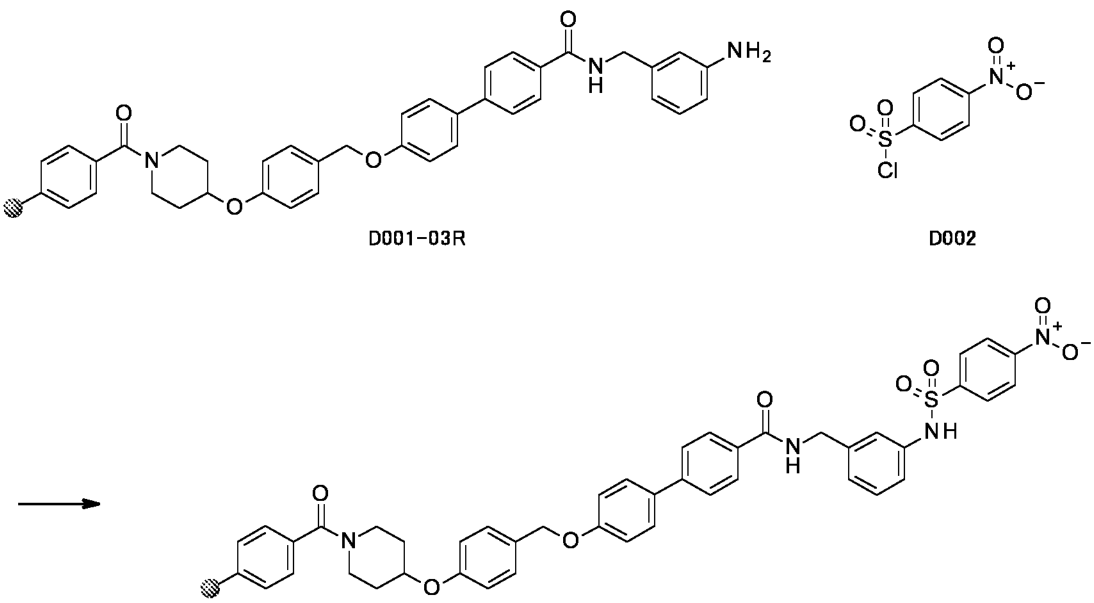

D001-03R

D002

D003-03R

Under a nitrogen atmosphere, solid-phase supported Ar-$NH_2$ (D001-03R, 0.050 mmol/g, 20.0 mg, 1.0 μmol) and THF (300 μL) were added to a 0.6 mL screw-cap vial and shaken at room temperature for 1 hour. Under a nitrogen atmosphere, pyridine (3.6 μL, 0.045 mmol) and 4-nitrobenzenesulfonyl chloride (D002, 6.65 mg, 0.030 mmol) were added thereto, and the obtained mixture was shaken at room temperature for 4 hours and then left standing at room temperature for 60 hours to obtain solid-phase supported sulfonamide (D003-03R).

The reaction was monitored as follows.

Under a nitrogen atmosphere, 10 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.02 M pentamethylbenzene (50 μL) for 5 minutes. After filtration, the filtrate was diluted with NMP (250 μL), and the solution (50 μL) was diluted with MeCN (250 μL) to prepare an LC sample. The degree of progression of the reaction was measured by LC-MS analysis. As a result, 98.9 area% of compound D003 of interest was observed after 4 hours at room temperature. The purity of D003 was 98.9 area% even after 60 hours at room temperature.

[Formula 404]

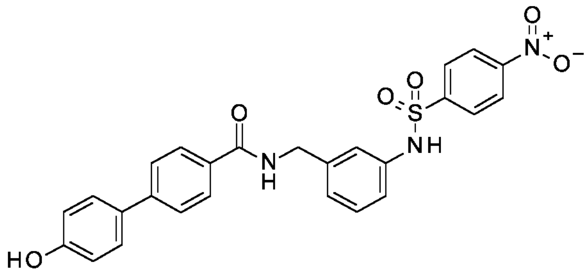

D003

Compound D003

LCMS: m/z 504[M+H]+.

Retention time: 1.018 min (analysis conditions SMD-FA05-1, 290 nm).

[Table 55]

| [Table 2-11-1] | | | | | |
|---|---|---|---|---|---|
| Run | base | Time (h) | UV area% | | |
| | | | TM (D003) | SM (D001) | BP (D004) |
| 1 | $Et_3N$ | 4 | 9.7 | ND | 90.3 |
| | | 60 | ND | ND | 98.8 |
| 2 | pyridine | 4 | 98.9 | ND | ND |
| | | 60 | 98.9 | ND | ND |

Results of analyzing the by-product (D004) at the reaction time of 60 hours in run 1 in Table 2-11-1 are shown below.

[Formula 405]

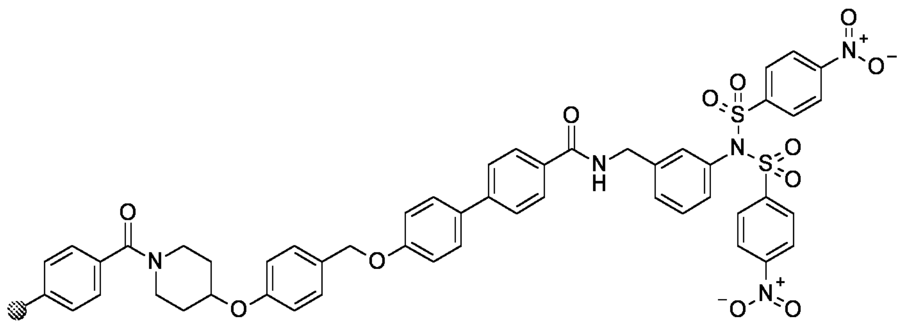

D004

Compound D004

LCMS: m/z 689[M+H]+.

Retention time: 1.222 min (analysis conditions SMD-FA05-1, 290 nm).

From the results described above, the starting material (D001) was consumed in 4 hours at room temperature when any of the bases was used. In the case of using triethylamine as a base, 90 area% or more of the overreaction product (D004) was observed at the point in time of 4 hours at room temperature. On the other hand, in the case of using pyridine, 98 area% or more of the target compound (D003) was observed after 4 hours at room temperature, and further, it was confirmed that the purity of the target compound (D003) was not reduced even after 60 hours at room temperature.

For constructing a mixture library, it is necessary to set conditions under which desired reaction progresses at high yields using a mixture of various substrates differing in reactivity. For example, a product from a highly reactive substrate is exposed to reaction conditions until a low reactive substrate reaches a sufficient conversion rate through reaction. Hence, it is preferred to confirm that overreaction, etc. does not occur during that time, and set these conditions as reaction conditions for mixture library synthesis. The conditions using THF and pyridine in Example 2-11-1 were concluded as sulfonamidation conditions suitable for mixture library construction, because overreaction associated with a prolonged reaction time did not progress.

Example 2-11-2: Solid-phase experiment to confirm scope of application of reaction conditions using THF and pyridine to sulfonamidation reaction using solid-phase supported Ar-NHz and various sulfonyl chlorides

The sulfonamidation conditions found in Example 2-11-1 were used to confirm the scope of application of sulfonyl chloride to the sulfonamidation of solid-phase supported Ar- $NH_2$ (D001-03R). The sulfonamidation was carried out by the same experimental operation as in Example 2-11-1 using sulfonyl chloride (D005 to D011).

The structure of the sulfonyl chloride used (D005 to D01 1) is shown in Table 2-11- 2-1, and the obtained results are shown in Table 2-11-2-2.

[Table 56]

[Table 2-11-2-1]

| [Table 2-11-2-1] | | |
|---|---|---|
| Compound No. | Structural formula | Compound name |
| D005 | 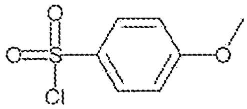 | 4-Methoxybenzenesulfonyl chloride |
| D006 | 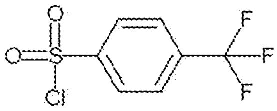 | 4-(Trifluoromethyl)benzenesulfonyl chloride |
| D007 | 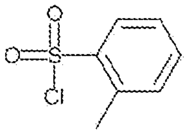 | 2-Methylbenzenesulfonyl chloride |
| D008 | 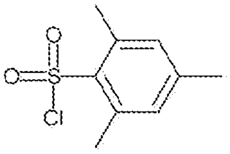 | 2,4,6-Trimethylbenzenesulfonyl chloride |
| D009 | 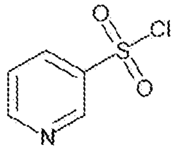 | Pyridine-3-sulfonyl chloride |
| D010 | 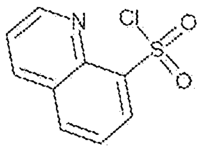 | Quinoline-8-sulfonyl chloride |
| D011 | 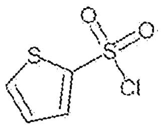 | Thiophene-2-sulfonyl chloride |

[Table 57]

| [Table 2-11-2-2] | | | | |
|---|---|---|---|---|
| Run | RSO2Cl | time (h) | UV area% | |
| | | | SM (D001) | TM |
| 1 | D005 | 21 | ND | 99.1 (D012) |
| 2 | D006 | 21 | ND | 98.6 (D013) |
| 3 | D007 | 25 | ND | 98.6 (D014) |
| 4 | D008 | 45 | ND | 71.0 (D015) |
| 5 | D009 | 21 | ND | 98.8 (D016) |

(continued)

| [Table 2-11-2-2] | | | | |
|---|---|---|---|---|
| Run | RSO2Cl | time (h) | UV area% | |
| | | | SM (D001) | TM |
| 6 | D010 | 21 | 2.9 | 92.8 (D017) |
| 7 | D011 | 21 | 57.8 | 40.9 (D018) |

Results of analyzing the compounds described in Table 2-11-2-2 are shown in Table 2-11-2-3.

[Table 58]

[Table 2-11-2-3]

[Table 2-11-2-3]

| Compound No. | Structural formula | Analysis approach | Cleavage wavelength (nm) | (ESI) (M+H)+ | Retention time (min) |
|---|---|---|---|---|---|
| D012 | 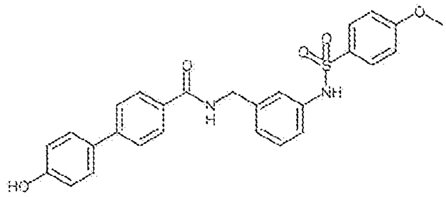 | SMD-FA05-1 | 290 | 489 | 0.990 |
| D013 | 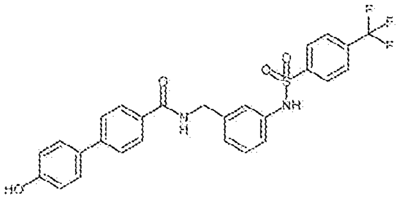 | SMD-FA05-1 | 290 | 527 | 1.094 |
| D014 | 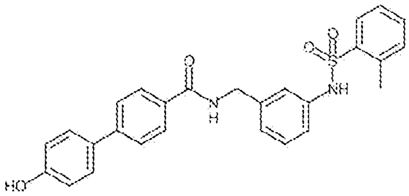 | SMD-TFA05-RP | 290 | 473 | 1.088 |
| D015 | 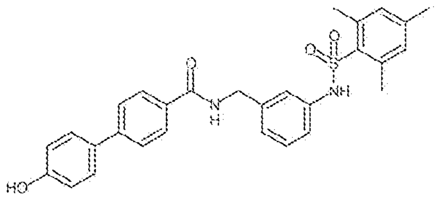 | SMD-TFA05-RP | 290 | 501 | 1.160 |
| D016 | 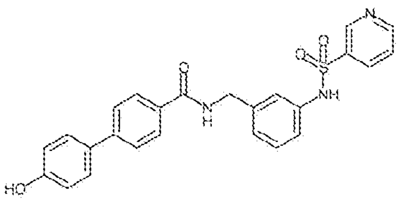 | SMD-TFA05-RP | 290 | 460 | 0.967 |
| D017 | 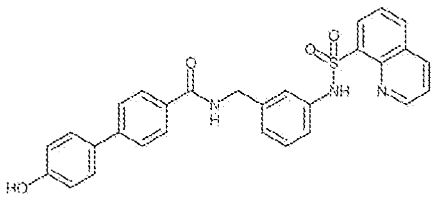 | SMD-TFA05-RP | 290 | 510 | 1.062 |
| D018 | 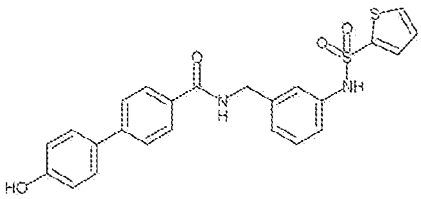 | SMD-TFA05-RP | 290 | 465 | 1.049 |

From the results described above, it was confirmed that, in the case of using sulfonyl chloride (D005 and D006) having an electron-withdrawing group or an electron-donating group on the aromatic ring, sulfonamide of interest (D012 and D013) was obtained at a purity of 99 area% or more. In the case of using 2-methylbenzenesulfonyl chloride (D007) having a methyl group at the ortho position, 4-(4-hydroxyphenyl)-N-[[3-[(2- methylphenyl)sulfonylamino]phenyl]methyl]benzamide of interest (D014) was produced at a purity of 98.6 area% in 25 hours at room temperature. On the other

hand, in the case of using 2,4,6-trimethylbenzenesulfonyl chloride (D008), the purity of 4-(4-hydroxyphenyl)-N-[[3- [(2,4,6-trimethylphenyl)sulfonylamino]phenyl]methyl]benzamide of interest (D015) was 71.0 area% at the point in time of 45 hours at room temperature. In the case of using sulfonyl chloride (D009 and D010) having an electron-poor aromatic heterocyclic ring, the corresponding sulfonamide (D016 and D017) was obtained at a purity of 90 area% or more. On the other hand, in the case of using thiophene-2-sulfonyl chloride (D011), 57.8 area% of the starting material (D001) remained after 21 hours at room temperature, and 40.9% of compound D018 of interest was obtained. From the study described above, the scope of applicable sulfonyl chloride under reaction conditions using THF and pyridine was confirmed.

Example 2-11-3: Solid-phase experiment to obtain product of interest at high purity through sulfonamidation reaction using solid-phase supported Ar-$NH_2$ and thiophene-2- sulfonyl chloride (D011)

In Example 2-11-2, 40.9 area% of the starting material (D001) was unreacted at the point in time of 21 hours at room temperature when thiophene-2-sulfonyl chloride (D011) was used. Accordingly, reaction conditions were set under which the product of interest was obtained at a high purity even when low reactive sulfonyl chloride such as D011 was used. Conditions involving using NMI (45 equivalents) as a base and conditions involving adding NMI (5 equivalents) to pyridine (45 equivalents) were carried out with reference to a literature known in the art (Tetrahedron Lett. 2001, 42, 979-982) to examine whether NMI had an effect of accelerating reaction. At the same time therewith, whether overreaction did not progress was confirmed using highly reactive 4-nitrobenzenesulfonyl chloride (D002). The study results are shown in Table 2-11-3. The example of adding NMI (5 equivalents) to pyridine (45 equivalents) and performing sulfonamidation using thiophene-2-sulfonyl chloride (D011) (run 3 in Table 2-11-3) in Table 2-11-3 will be shown below.

Sulfonamidation reaction (exemplary reaction operation) using solid-phase supported Ar-$NH_2$ and thiophene-2-sulfonyl chloride (D011)

[Formula 406]

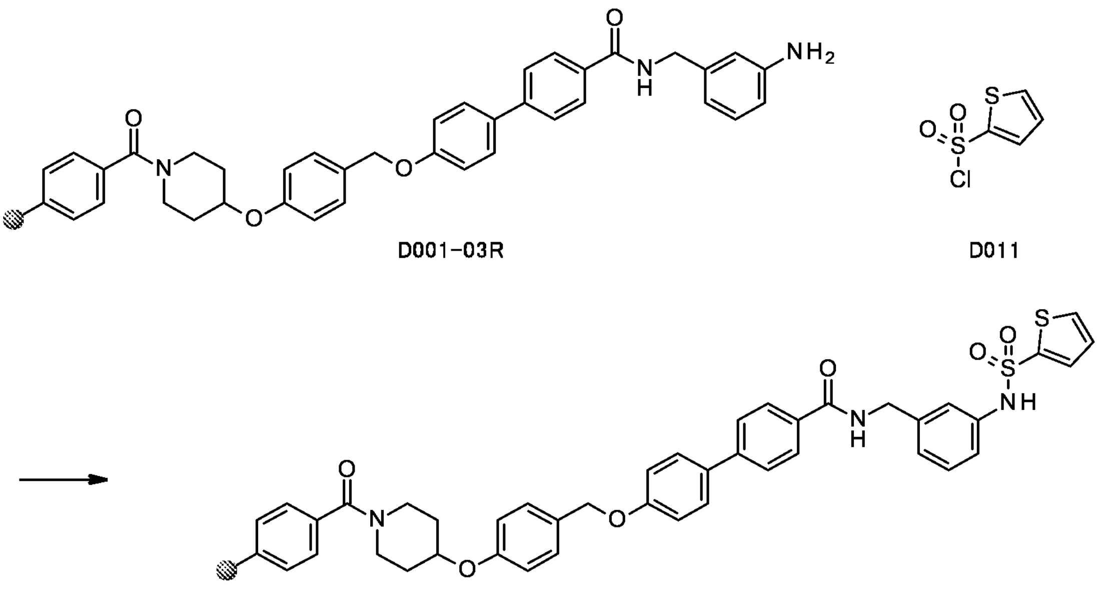

D001-03R D011

D018-03R

Under a nitrogen atmosphere, solid-phase supported Ar-$NH_2$ (D001-03R, 0.050 mmol/g, 20.0 mg, 1.00 μmol) and THF (300 μL) were added to a 0.6 mL screw-cap vial and shaken at room temperature for 1 hour.

Under a nitrogen atmosphere, pyridine (3.6 μL, 0.045 mmol), NMI (10 v/v, a solution in THF, 4.0 μL, 5.0 μmol), and thiophene-2-sulfonyl chloride (D011) (5.48 mg, 0.0300 mmol) were added thereto, and the obtained mixture was shaken at room temperature for 21 hours.

The reaction was monitored as follows.

Under a nitrogen atmosphere, 20 μL of the resin suspension was sampled, then transferred to a pipette tip with a filter, washed three times with NMP (100 μL), three times with MeOH (100 μL), and three times with DCM (100

μL), and then dipped in a 10% solution of TFA in DCM containing 0.02 M pentamethylbenzene (50 μL) for 5 minutes. The cleavage solution was filtered, and the solid phase was washed with NMP (50 μL). The obtained filtrate was diluted with MeCN (500 μL) to prepare an LC sample. The degree of progression of the reaction was measured by LC-MS analysis. As a result, 91.4 area% of compound D018 of interest was observed.

[Formula 407]

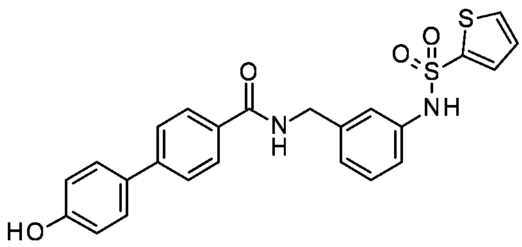

D018

Compound D018
LCMS: m/z 465[M+H]+.
Retention time: 1.046 min (analysis conditions SMD-TFA05-RP, 290 nm).
[Table 59]

| [Table 2-11-3] | | | | | | | |
|---|---|---|---|---|---|---|---|
| Run | base (eq.) | RSO2Cl | UV area% | | | | |
| | | | TM | | SM (D001) | BP | |
| 1 | NMI (45 eq.) | D011 | TM (D018) | 88.9 | ND | | |
| 2 | | D002 | TM (D003) | 1.1 | 0.83 | BP (D004) | 95.1 |
| 3 | pyridine (45 eq.) | D011 | TM (D018) | 91.4 | 3.8 | | |
| 4 | NMI (5 eq.) | D002 | TM (D003) | 94.3 | ND | BP (D004) | 2.9 |

Results of analyzing the by-product (D004) in run 2 in Table 2-11-3 are shown below.

[Formula 408]

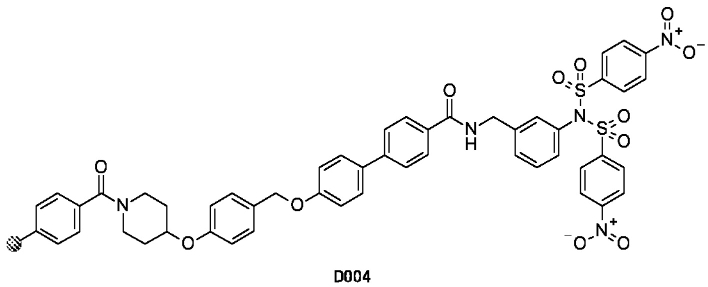

D004

Compound D004
LCMS: m/z 689[M+H]+.
Retention time: 1.244 min (analysis conditions SMD-TFA05-RP, 290 nm).
Results of analyzing the main product (D003) in run 4 in Table 2-11-3 are shown below.

[Formula 409]

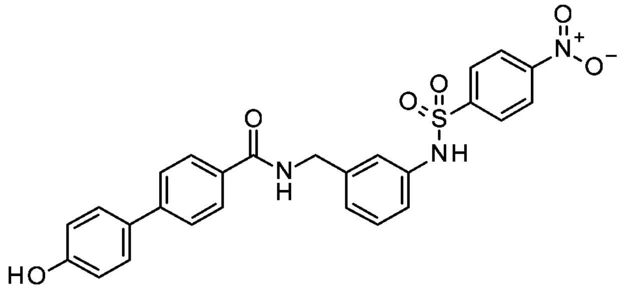

D003

Compound D003

LCMS: m/z 504[M+H]+.

Retention time: 1.244 min (analysis conditions SMD-TFA05-RP, 290 nm).

As shown in Table 2-11-3, as a result of performing the sulfonamidation of solid- phase supported Ar-$NH_2$ (D001) with thiophene-2-sulfonyl chloride (D011) using NMI (45 equivalents) as a base, D001 was consumed in 21 hours at room temperature, and compound D018 of interest was obtained at a purity of 88.9 area%. These results were compared with the results about run 7 in Table 2-11-2-2 shown in Example 2-11-2 to confirm an effect of accelerating sulfonamidation by NMI. On the other hand, as a result of applying conditions using NMI (45 equivalents) to highly reactive sulfonyl chloride, i.e., 4-nitrobenzenesulfonyl chloride (D002) having an electron-withdrawing group on the aromatic ring, 95.1 area% of the overreaction product (D004) was confirmed. This revealed that conditions using NMI (45 equivalents) cannot be applied to highly reactive 4-nitrobenzenesulfonyl chloride (D002).

Accordingly, NMI (5 equivalents) was added to the conditions using pyridine (45 equivalents) in a THF solvent which were set in Example 2-11-2. As a result, the target compound was obtained at a purity of 90 area% or more using any of thiophene-2-sulfonyl chloride (D011) and 4-nitrobenzenesulfonyl chloride (D002). Thus, the reaction conditions using pyridine (45 equivalents) and NMI (5 equivalents) in THF were set as conditions under which the target compound was obtained at a high purity even when various sulfonyl chlorides differing in reactivity were used.

Example 2-11-4-1: Solid-phase experiment to confirm scope of application under reaction conditions using THF and pyridine supplemented with NMI to sulfonamidation reaction using solid-phase supported Ar-NHz, Ar-NHMe or alkylamine and sulfonyl chloride

The scope of application of the reaction conditions using pyridine (45 equivalents) and NMI (5 equivalents) in THF, which were found in Example 2-11-3, was confirmed. Sulfonamidation reaction using solid-phase supported Ar-$NH_2$ (D001-03R), Ar-NHMe (D020-03R) or alkylamine (D021-03R) and sulfonyl chloride (D002, D005, D007, D011, D008, and D019) was carried out by the same experimental operation as in Example 2-11-3.

The structure of 2-tert-butylbenzenesulfonyl chloride (D019) used as a reagent in sulfonamidation is shown below.

[Formula 410]

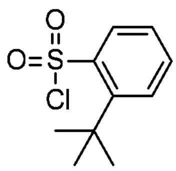

D019

Compound name: 2-tert-butylbenzenesulfonyl chloride

Compound No: D019

The obtained results are shown in Table 2-11-4-1. The reaction operation of sulfonamidation using solid-phase supported Ar-NHMe (D020-03R) or alkylamine (D021- 03R) and sulfonyl chloride (DOll) in Table 2-11-4-1 will be shown as a typical example.

Solid-phase experiment (exemplary reaction operation) of sulfonamidation reaction using solid-phase supported Ar-NHMe (D020-03R) and thiophene-2-sulfonyl chloride (D011)

[Formula 411]

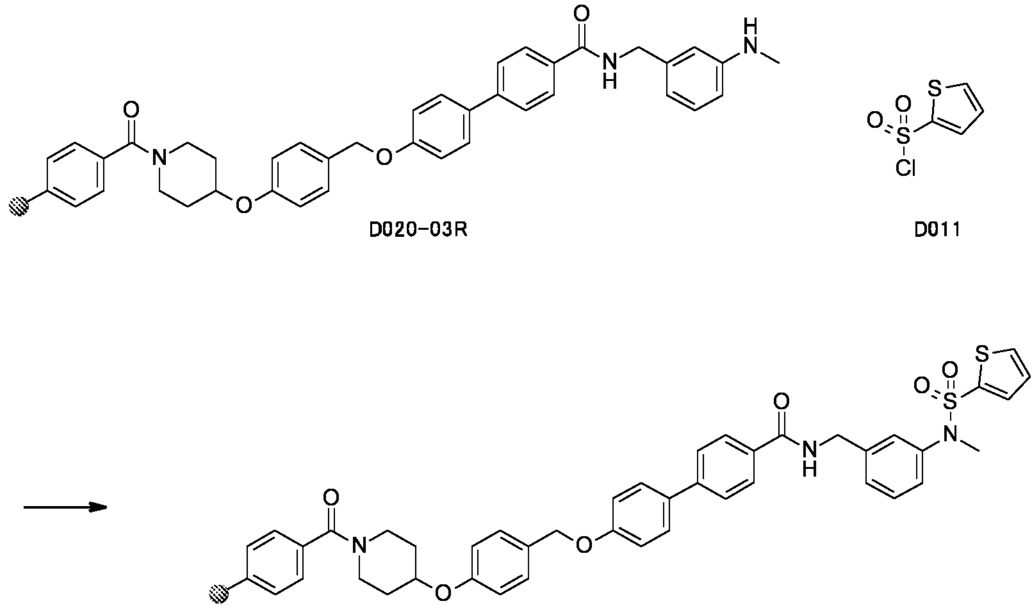

D020-03R D011

D024-03R

Under a nitrogen atmosphere, solid-phase supported Ar-NHMe (D020-03R, 0.050 mmol/g, 20.0 mg, 1.0 μmol) and THF (300 μL) were added to a 0.6 mL screw-cap vial and shaken at room temperature for 1 hour.

Under a nitrogen atmosphere, pyridine (3.6 μL, 0.045 mmol), NMI (10 v/v, a solution in THF, 4.0 μL, 5.0 μmol), and thiophene-2-sulfonyl chloride (D011) (5.48 mg, 0.0300 mmol) were added thereto, and the obtained mixture was shaken at room temperature for 21 hours.

The reaction was monitored as follows.

Under a nitrogen atmosphere, 20 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.02 M pentamethylbenzene (50 μL) for 5 minutes. The cleavage solution was filtered, and the solid phase was washed with NMP (50 μL). The obtained filtrate was diluted with MeCN (500 μL) to prepare an LC sample. The degree of progression of the reaction was measured by LC-MS analysis. As a result, 64.1 area% of compound D024 of interest was observed.

[Formula 412]

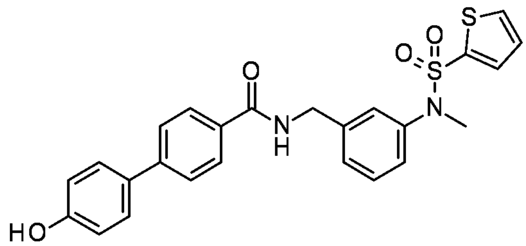

D024

Compound D024

LCMS: m/z 479$[M+H]^+$.

Retention time: 1.103 min (analysis conditions SMD-TFA05-RP, 290 nm).

Solid-phase experiment (exemplary reaction operation) of sulfonamidation reaction using solid-phase supported alkylamine (D021-03R) and thiophene-2-sulfonyl chloride (D011)

[Formula 413]

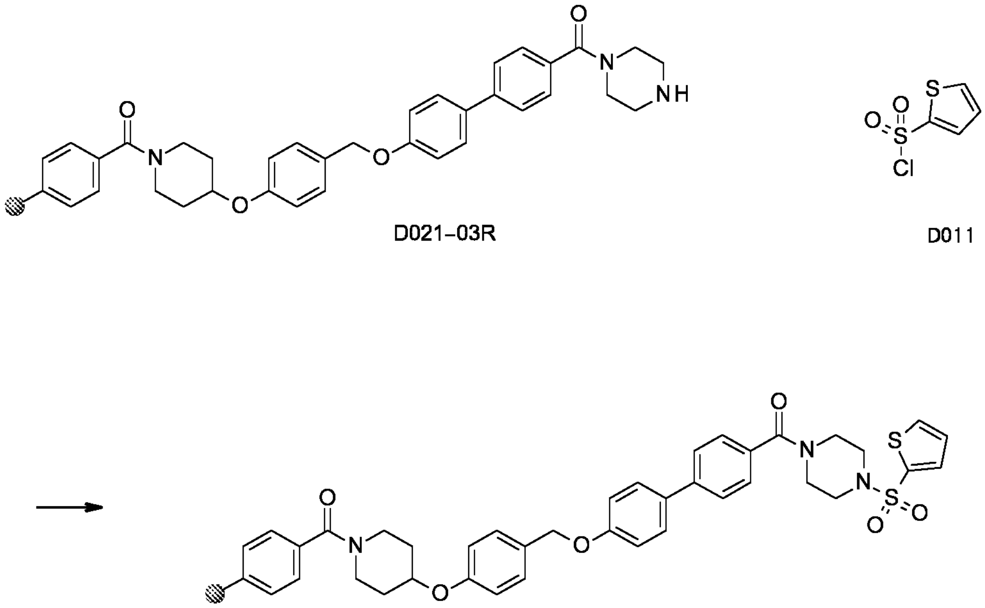

D021-03R D011

D029-03R

Under a nitrogen atmosphere, solid-phase supported alkylamine (D021-03R, 0.050 mmol/g, 20.0 mg, 1.0 μmol) and THF (300 μL) were added to a 0.6 mL screw-cap vial and shaken at room temperature for 1 hour. Under a nitrogen atmosphere, pyridine (3.6 μL, 0.045 mmol), NMI (10 v/v, a solution in THF, 4.0 μL, 5.0 μmol), and thiophene-2-sulfonyl chloride (D011) (5.48 mg, 0.0300 mmol) were added thereto, and the obtained mixture was shaken at room temperature for 21 hours.

The reaction was monitored as follows. Under a nitrogen atmosphere, 20 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.02 M pentamethylbenzene (50 μL) for 5 minutes. The cleavage solution was filtered, and the solid phase was washed with NMP (50 μL). The obtained filtrate was diluted with MeCN (500 μL) to prepare an LC sample. The degree of progression of the reaction was measured by LC-MS analysis. As a result, 96.2 area% of compound D029 of interest was observed.

[Formula 414]

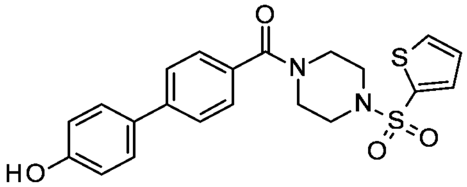

D029

Compound D029

LCMS: m/z 429$[M+H]^+$.

Retention time: 1.032 min (analysis conditions SMD-TFA05-RP, 280 nm).

[Table 60]

[Table 2-11-4-1]

| Solid-phase supported substrate | RSO 2Cl | | | | | | | | | | | |
|---|---|---|---|---|---|---|---|---|---|---|---|---|
| | D002 | | D005 | | D011 | | D007 | | D008 | | D019 | |
| D001-03R | SM (D001) | ND | | | SM (D001) | 3.8 | SM (D001) | ND | | | | |
| | TM (D003) | 94.3 | | | TM (D018) | 91.4 | TM (D014) | 96.6 | | | | |
| D020-03R | SM (D020) | ND | SM (D020) | ND | SM (D020) | 34.4 | SM (D020) | ND | SM (D020) | 24.4 | SM (D020) | 98.1 |
| | TM (D022) | 94.4 | TM (D023) | 96.4 | TM (D024) | 64.1 | TM (D025) | 97.0 | TM (D026) | 57.2 | TM (D027) | 1.9 |
| D021-03R | SM (D021) | ND | | | SM (D021) | ND | SM (D021) | ND | SM (D021) | ND | SM (D021) | 18.8 |
| | TM (D028) | 95.5 | | | TM (D029) | 96.2 | TM (D030) | 97.5 | TM (D031) | 90.5 | TM (D032) | 78.8 |

Results of analyzing the compounds described in Table 2-11-4-1 are shown in Table 2-11-4-2.

[Table 61]

[Table 2-11-4-2]

[Table 2-11-4-2]

| Compound No. | Structural formula | Analysis approach | Cleavage wavelength (nm) | (ESI) (M+H)+ | Retention time (min) |
|---|---|---|---|---|---|
| D003 | 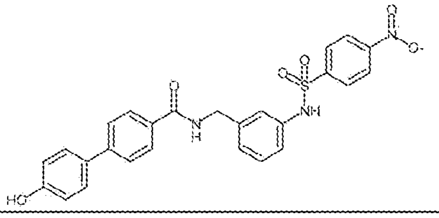 | SMD-TFA05-RP | 290 | 504 | 1.076 |
| D018 | 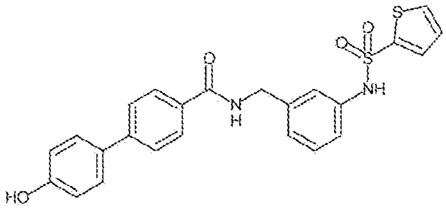 | SMD-TFA05-RP | 290 | 465 | 1.046 |
| D014 | 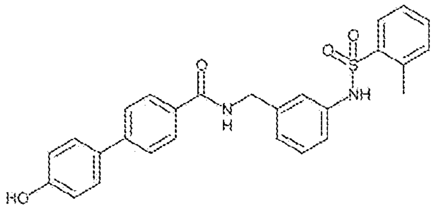 | SMD-TFA05-RP | 290 | 473 | 1.093 |
| D022 | 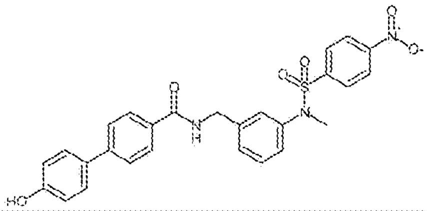 | SMD-TFA05-RP | 290 | 518 | 1.134 |
| D023 | 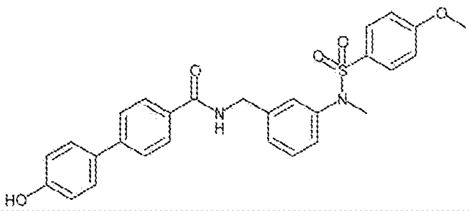 | SMD-TFA05-RP | 290 | 503 | 1.119 |
| D024 | 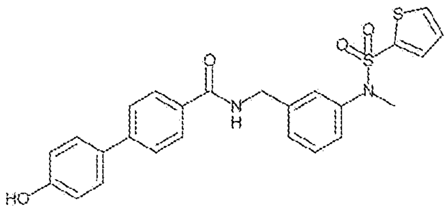 | SMD-TFA05-RP | 290 | 479 | 1.103 |
| D025 | 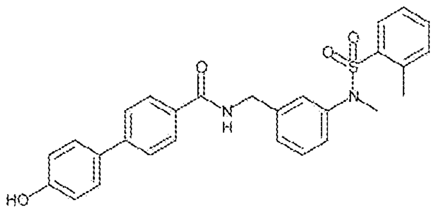 | SMD-TFA05-RP | 290 | 487 | 1.150 |

[Table 62]

| | | | | | |
|---|---|---|---|---|---|
| D026 | 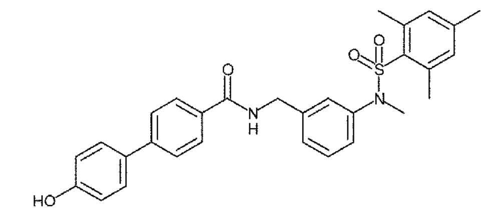 | SMD−TFA05−RP | 290 | 515 | 1.231 |
| D027 | 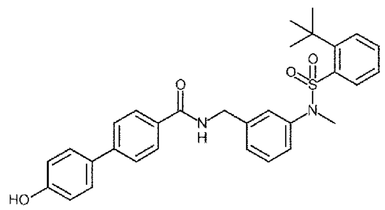 | SMD−TFA05−RP | 290 | 529 | 1.253 |
| D028 | 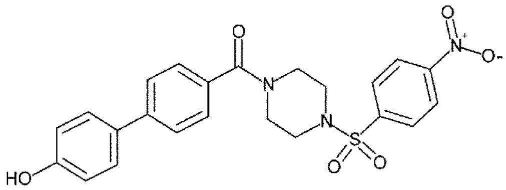 | SMD−TFA05−RP | 280 | 468 | 1.067 |
| D029 | 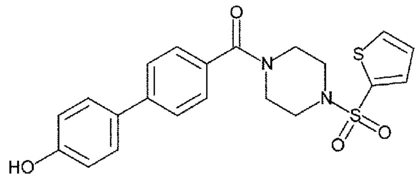 | SMD−TFA05−RP | 280 | 429 | 1.032 |
| D030 | 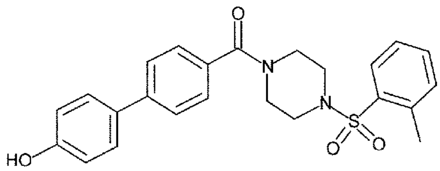 | SMD−TFA05−RP | 280 | 437 | 1.090 |
| D031 | 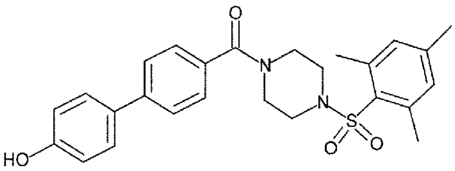 | SMD−TFA05−RP | 280 | 465 | 1.191 |
| D032 | 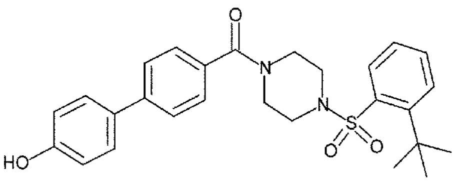 | SMD−TFA05−RP | 280 | 479 | 1.228 |

As shown in Table 2-11-4-1, in the case of using solid-phase supported Ar-NHz (D001-03R), 90 area% or more of the target compound was obtained through reaction with D002, D011 or 2-methylbenzenesulfonyl chloride (D007) having a methyl group at the ortho position of sulfone on the aromatic ring. In the case of using solid-phase supported Ar- NHMe (D020-03R), 90 area% or more of the target compound was confirmed through reaction with sulfonyl chloride (D002, D005, and D007), whereas 20 area% or more of the starting material remained after 21 hours at room temperature through reaction with sulfonyl chloride (D011, D008, and D019). The sulfonamidation of solid-phase supported alkylamine (D021-03R) with sulfonyl chloride (D002, D011, D007, and D008) produced 90 area% or more of the target compound, whereas 18.8% of the starting material remained through the reaction thereof with 2-tert-butylbenzenesulfonyl chloride

(D019) having a tert-butyl group at the ortho position of sulfone on the aromatic ring.

The study described above clarified the scope of application of reaction conditions using pyridine (45 equivalents) and NMI (5 equivalents) in THF. In an embodiment, the sulfonamidation of a mixture of aniline derivatives or amines differing in reactivity can be allowed to progress at high yields with reference to this scope of application, and a high-quality mixture library containing sulfonamide can be constructed. For sulfonamidation using the combination (e.g., solid-phase supported Ar-NHMe (D020-03R) and thiophene-2- sulfonyl chloride (D011)) resulting in a low purity of the target compound in Table 2-11-4-1, the target compound can be obtained at a high purity by setting other more severe conditions in order to consume the starting material.

Example 2-11-4-2: Solid-phase experiment to perform sulfonamidation reaction using solid-phase supported hetero-Ar-NHz and sulfonyl chloride

The sulfonamidation of solid-phase supported hetero-Ar-NHz was performed under the reaction conditions using pyridine and NMI as a base which were found in Example 2-11- 3. Sulfonamidation reaction using solid-phase supported hetero-Ar-NHz (D072-03R) and 4- methoxybenzenesulfonyl chloride (D006) was carried out by the same experimental operation as in Example 2-11-3. The obtained results are shown in run 1 in Table 2-11-4-3. Results obtained by changing the solvent of run 1 to DCM are shown in run 2. Results obtained by using NMI (15 equivalents) or 3,4-lutidine (15 equivalents) as a base in a DCM solvent are shown in run 3 and 4, respectively. The reaction operation of sulfonamidation under reaction conditions using 3, 4-lutidine in DCM (run 4) in Table 2-11-4-3 will be shown as a typical example, The same experimental operation thereas was performed for run 1 to 3.

Solid-phase experiment (exemplary reaction operation) of sulfonamidation reaction using solid-phase supported hetero-Ar-NHMe (D072-03R) and 4-methoxybenzenesulfonyl chloride (D005)

[Formula 415]

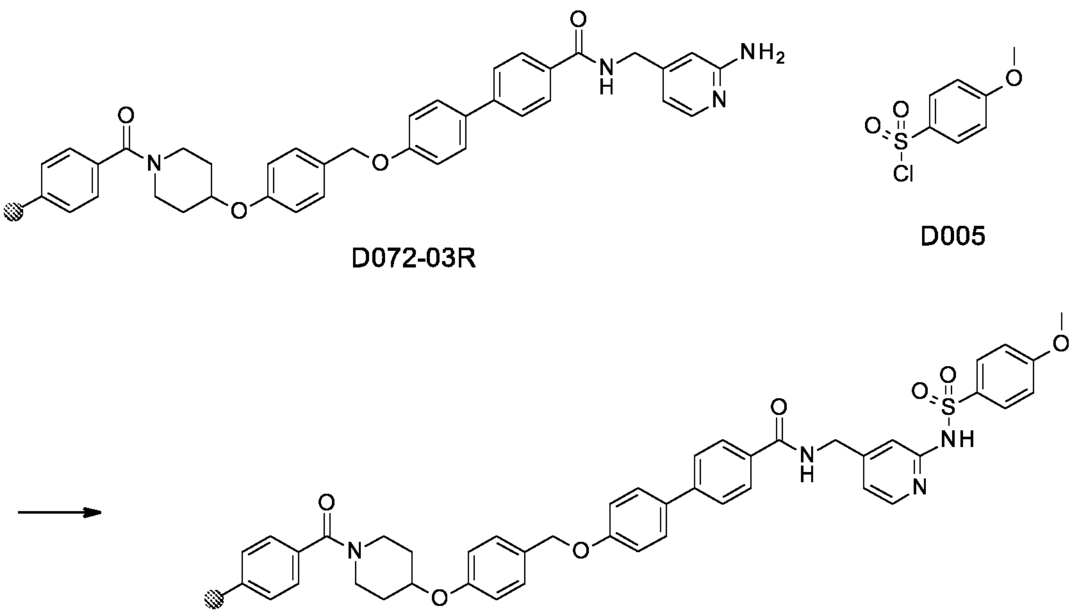

D072-03R

D005

D073-03R

Under a nitrogen atmosphere, solid-phase supported hetero-Ar-NHMe (D072-03R, 0.197 mmol/g, 20.0 mg, 3.94 μmol) and DCM (400 μL) were added to a 0.6 mL screw-cap vial and shaken at room temperature for 1 hour.

Under a nitrogen atmosphere, 3,4-lutidine (6.6 μL, 59 μmol) and 4- methoxybenzenesulfonyl chloride (D005) (8.1 mg, 0.039 mmol) were added thereto, and the obtained mixture was shaken at room temperature for 24 hours.

The reaction was monitored as follows.

Under a nitrogen atmosphere, 12 μL of the resin suspension was sampled, then transferred to a pipette tip with a filter, washed three times with DMA (100 μL), three times with MeOH (100 μL), and three times with DCM (100 μL), and then dipped in a 10% solution of TFA in DCM containing 0.05 M pentamethylbenzene (50 μL) for 5 minutes.

The cleavage solution was filtered, and the solid phase was washed with DMA (50 μL). The obtained filtrate was diluted with MeCN (500 μL) to prepare an LC sample. The degree of progression of the reaction was measured by LC-MS analysis. As a result, 90.8 area% of compound D073 of interest was observed.

[Formula 416]

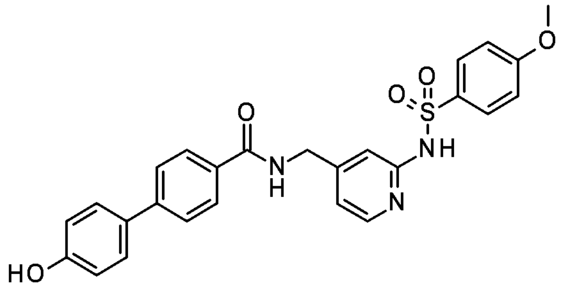

D073

Compound D073

LCMS: m/z 490[M+H]+.

Retention time: 0.800 min (analysis conditions SMD-FA05-1, 299 nm).

[Table 63]

[Table 2-11-4-3]

| Run | base (eq.) | solvent | UV area% | |
|---|---|---|---|---|
| | | | TM (D073) | SM (D072) |
| 1 | pyridine(15 eq.), NMI (5 eq.) | THF | 33.1 | 66.5 |
| 2 | | DCM | 41.2 | 58.8 |
| 3 | NMI (15 eq.) | DCM | 84.1 | 13.6 |
| 4 | 3,4-lutidine (15 eq.) | DCM | 90.8 | 8.2 |

Thus, conditions (run 4) were found which were able to improve the conversion rate to more than 90 area% even for a substrate that had only a low conversion rate under the conditions (run 1 in Table 2-11-4-3) found in Example 2-11-4-1.

This condition can be applied to mixture library synthesis, and the scope of substrate application is not restricted.

Example 2-11-5: Solid-phase experiment to determine optimum conditions for sulfonamidation reaction using solid-phase supported Ar-$NH_2$ or Ar-NHMe and sulfonyl chloride having fluorine on aromatic ring

The sulfonamidation of 4-fluoro-3-nitrobenzenesulfonyl chloride (D033) having fluorine on the aromatic ring with solid-phase supported Ar-NHMe (D020) was carried out under the conditions using pyridine in THF which were found in Example 2-11-1 (run 1 in Table 2-11-5). As a result, 7.0 area% of a by-product 4-(4-hydroxyphenyl)-N-[[3-[methyl-(3-nitro-4-pyridin-1-ium-1-ylphenyl)sulfonylamino]phenyl]methyl]benzamide (D036) resulting from the substitution of the fluorine atom contained in sulfonyl chloride by pyridine used as a base was confirmed. Hence, the sulfonamidation reaction of solid-phase supported Ar-$NH_2$ (D001-03R) or Ar-NHMe (D020-03R) with 4-fluoro-3-nitrobenzenesulfonyl chloride (D033) was carried out under another condition. Solid-phase supported Ar-NHMe (D020-03R) was studied using pyridine, 2,6-lutidine or 2,6-di-tert-butylpyridine (DTBP) as a base in a THF solvent. Solid-phase supported Ar-$NH_2$ (D001-03R) was studied for conditions using 2,6-lutidine in a THF solvent or conditions using 2,6-lutidine or 2,6-di-tert- butylpyridine in a DCE solvent. The obtained results are shown in Table 2-11-5. The reaction operation of the sulfonamidation of solid-phase supported Ar-NHMe (D020-03R) with 4-fluoro-3-nitrobenzenesulfonyl chloride (D033) using 2,6-lutidine as a base in a THF solvent in run 2 in Table 2-11-5 will be shown as a typical example. The same experimental operation as in run 2 was performed for run 1, 3, 4, and 5.

Solid-phase experiment (exemplary reaction operation) of sulfonamidation reaction using solid-phase supported Ar-NHMe (D020-03R) and 4-fluoro-3-nitrobenzenesulfonyl chloride (D033)

[Formula 417]

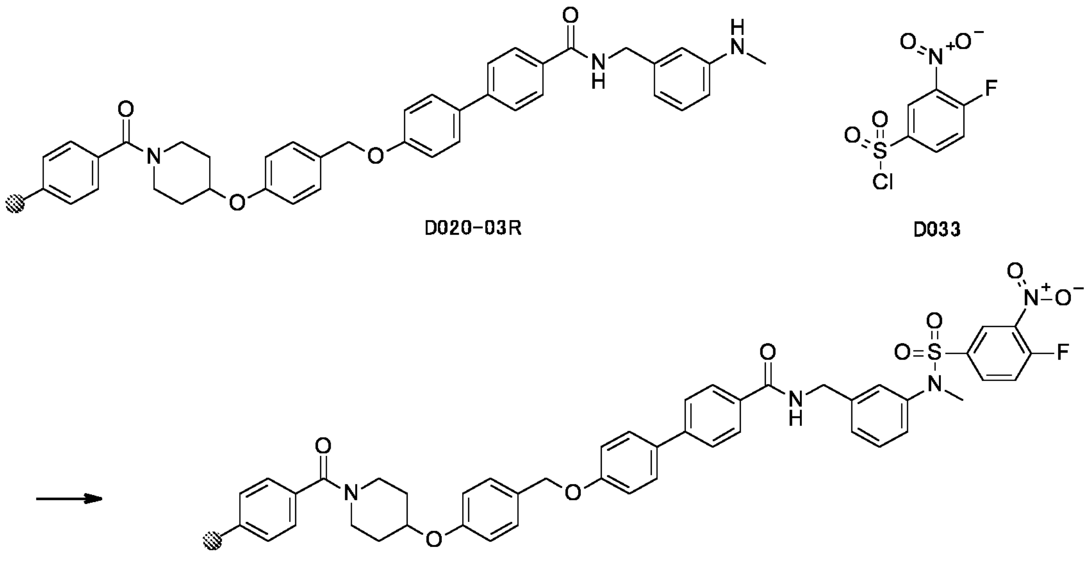

D020-03R D033

D034-03R

Under a nitrogen atmosphere, solid-phase supported Ar-NHMe (D020-03R, 0.050 mmol/g, 20.0 mg, 1.0 μmol) and THF (300 μL) were added to a 0.6 mL screw-cap vial and shaken at room temperature for 1 hour.

Under a nitrogen atmosphere, 2,6-lutidine (5.2 μL, 0.045 mmol) and 4-fluoro-3- nitrobenzenesulfonyl chloride (D033) (7.2 mg, 0.0300 mmol) were added thereto, and the obtained mixture was shaken at room temperature for 35 hours.

The reaction was monitored as follows. Under a nitrogen atmosphere, 10 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.02 M pentamethylbenzene (50 μL) for 5 minutes. The cleavage solution was filtered, and the solid phase was washed with NMP (50 μL). The obtained filtrate was diluted with MeCN (500 μL) to prepare an LC sample. The degree of progression of the reaction was measured by LC-MS analysis. As a result, 97.1 area% of compound D034 of interest was observed.

[Formula 418]

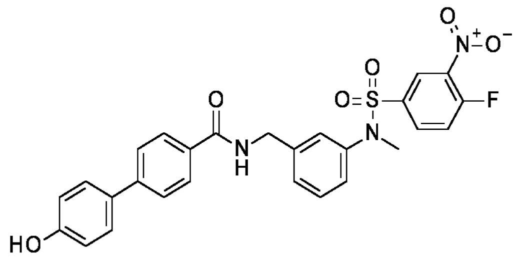

D034

Compound D034

LCMS: m/z 536[M+H]$^+$.

Retention time: 1.142 min (analysis conditions SMD-TFA05-RP, 290 nm).

[Table 64]

| [Table 2-11-5] | | | | | | |
|---|---|---|---|---|---|---|
| Solid-phase supported substrate | Run | solvent | base | time (h) | UV area% | |
| D020-03R | 1 | THF | pyridine | 4 | TM (D034) | 83.7 |
| | | | | | SM (D020) | ND |
| | | | | | BP(D036) | 7.0 |
| | 2 | THF | 2,6-lutidine | 35 | TM (D034) | 97.1 |
| | | | | | SM (D020) | ND |
| D001-03R | 3 | THF | 2,6-lutidine | 8 | TM (D035) | 65.8 |
| | | | | | SM (D001) | 0.77 |
| | | | | | BP(D037) | 15.3 |
| | 4 | DCE | 2,6-lutidine | 6 | TM (D035) | 83.2 |
| | | | | | SM (D001) | 1.1 |
| | | | | | BP(D037) | 6.8 |
| | 5 | DCE | DTBP | 6 | TM (D035) | 95.4 |
| | | | | | SM (D001) | ND |
| | | | | | BP(D037) | 1.4 |

Results of analyzing the by-product (D036, structure: putative) in run 1 in Table 2- 11-5 are shown below.

[Formula 419]

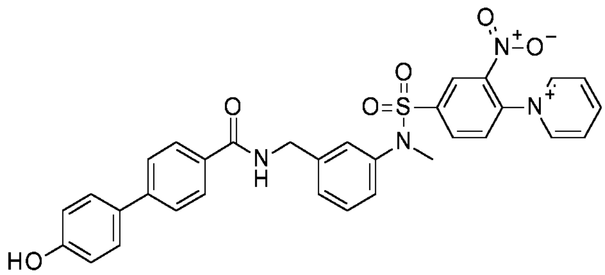

D036

Compound D036

LCMS: m/z 595[M+H]+.

Retention time: 0.870 min (analysis conditions SMD-TFA05-RP, 290 nm).

Results of analyzing the main product (D035) and the by-product (D037, structure: putative) in run 3 in Table 2-11-5 are shown below.

[Formula 420]

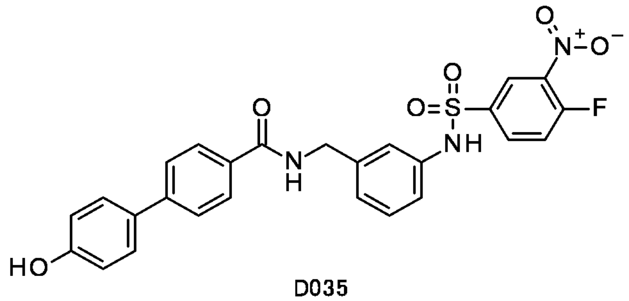

D035

Compound D035

LCMS: m/z 522[M+H]+.

Retention time: 1.104 min (analysis conditions SMD-TFA05-RP, 290 nm).

[Formula 421]

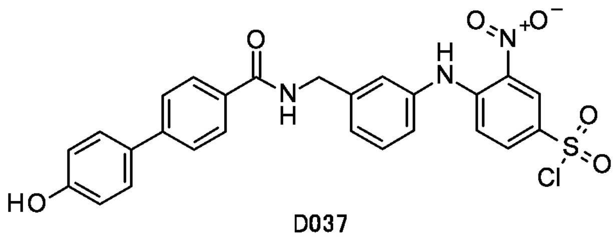

D037

Compound D037

LCMS: m/z 538[M+H]+.

Retention time: 1.256 min (analysis conditions SMD-TFA05-RP, 290 nm).

Sulfonamidation reaction using solid-phase supported Ar-NHMe (D020-03R) and 4- fluoro-3-nitrobenzenesulfonyl chloride (D033) was able to suppress side reaction involving a base, by changing the base from pyridine to 2,6-lutidine, and the target compound (D034) was obtained at a purity of 97.1 area%. On the other hand, in the reaction of solid-phase supported Ar-$NH_2$ (D001-03R) with 4-fluoro-3-nitrobenzenesulfonyl chloride (D033), 15.3 area% of a by-product 4-[3-[[[4-(4-hydroxyphenyl)benzoyl]amino]methyl]anilino]-3- nitrobenzenesulfonyl chloride (D037, structure: putative) resulting from $S_N$Ar reaction caused by solid-phase supported aniline (D001-03R) against 4-fluoro-3-nitrobenzenesulfonyl chloride (D033) was confirmed when 2,6-lutidine in a THF solvent was used. Accordingly, the formation of the by-product 4-[3-[[[4-(4-hydroxyphenyl)benzoyl]amino]methyl]anilino]- 3-nitrobenzenesulfonyl chloride (D037, structure: putative) was suppressed to 6.6 area% by changing the solvent to DCE, and compound D035 of interest was obtained at a purity of 83.2 area%. Further, the formation of by-product D037 was able to be reduced to 1.4 area% by changing the base to DTBP, and 95.4 area% of compound D035 of interest was obtained. Thus, reaction conditions were set under which the target compound was obtained at a high purity by selecting a suitable solvent and base and thereby controlling side reaction. In this way, a high-purity mixture library can be constructed by examining beforehand the reactivity of even a reactant (e.g., 4-fluoro-3-nitrobenzenesulfonyl chloride, D033) capable of causing side reaction, and setting suitable reaction conditions. This example is a mere illustration, and conditions for the application of reactants or substrates differing in reactivity to a mixture library can also be elucidated by making similar study beforehand.

Example 2-11-6: Solid-phase experiment to confirm progression of sulfonamidation reaction with sulfonyl chloride (D033) having fluorine on aromatic ring using solid-phase supported hetero-Ar-NHz

The sulfonamidation of 4-fluoro-3-nitrobenzenesulfonyl chloride (D033) having fluorine on the aromatic ring with solid-phase supported hetero-Ar-NHz (D106-03R) was carried out under the conditions using DTBP in DCE which were found in Example 2-11-5. As a result, 89.4 area% of compound D107 of interest was observed. From this, the reaction conditions using DTBP in DCE were also confirmed to be applicable to the sulfonamidation of hetero-Ar-NHz.

Solid-phase experiment (exemplary reaction operation) of sulfonamidation reaction using solid-phase supported hetero-Ar-$NH_2$ (D106-03R) and 4-fluoro-3-nitrobenzenesulfonyl chloride (D033)

[Formula 422]

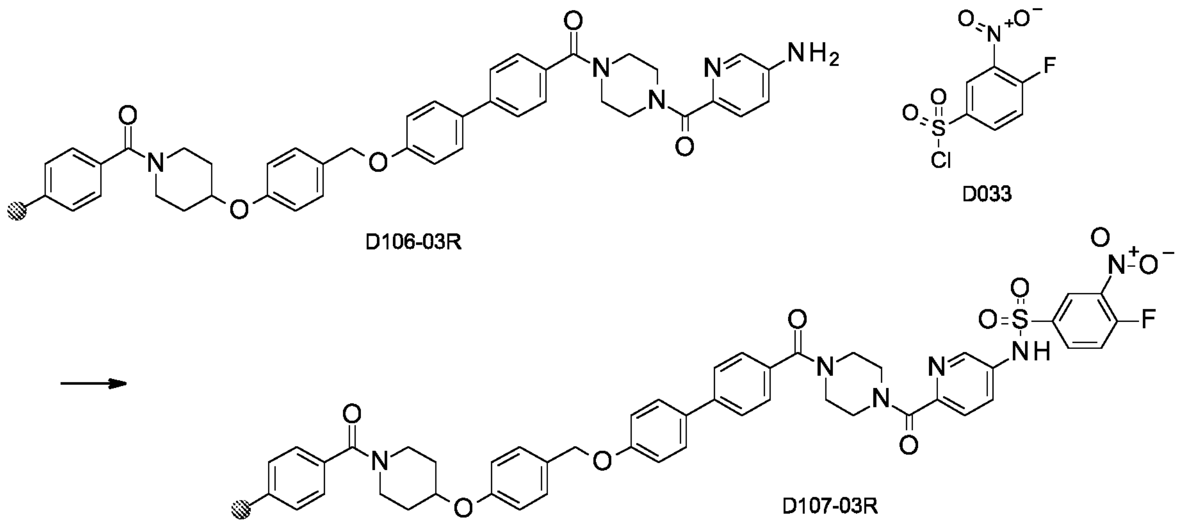

D106-03R

D033

D107-03R

Under a nitrogen atmosphere, solid-phase supported hetero-Ar-$NH_2$ (D106-03R) (0.200 mmol/g, 20 mg, 4.0 μmol) and DCE (350 μL) were added to a 0.6 mL screw-cap vial and shaken at room temperature for 1 hour.

Under a nitrogen atmosphere, a solution of DTBP (52.8 μL, 0.240 mmol) and 4- fluoro-3-nitrobenzenesulfonyl chloride (D033) (9.20 mg, 0.038 mmol) in DCE (50 μL) was added thereto, and the obtained mixture was shaken at 60°C for 23 hours.

The reaction was monitored as follows. Under a nitrogen atmosphere, 12 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.05 M pentamethylbenzene (50 μL) for 5 minutes. The cleavage solution was filtered, and the solid phase was washed with NMP (50 μL). The obtained filtrate was diluted with MeCN (500 μL) to prepare an LC sample. The degree of progression of the reaction was measured by LC-MS analysis. As a result, 89.4 area% of compound D107 of interest was observed.

[Formula 423]

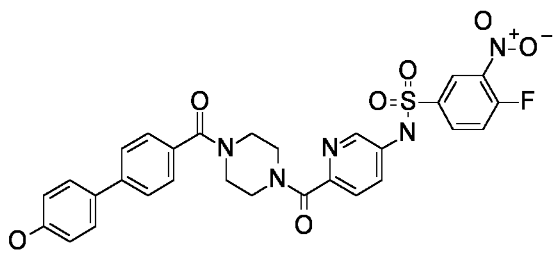

D107

Compound D107

LCMS: m/z 606$[M+H]^+$.

Retention time: 0.928 min (analysis conditions SMD-FA05-1, 299 nm).

Example 2-11-7-1: Solid-phase experiment to confirm progression of sulfonamidation reaction with sulfonyl chloride (D033) having fluorine on aromatic ring using solid-phase supported secondary alkylamine (D109-03R)

The sulfonamidation reaction of solid-phase supported alkylamine (D109-03R) was performed to synthesize a

compound (D110-03R) as follows (exemplary reaction operation).

[Formula 424]

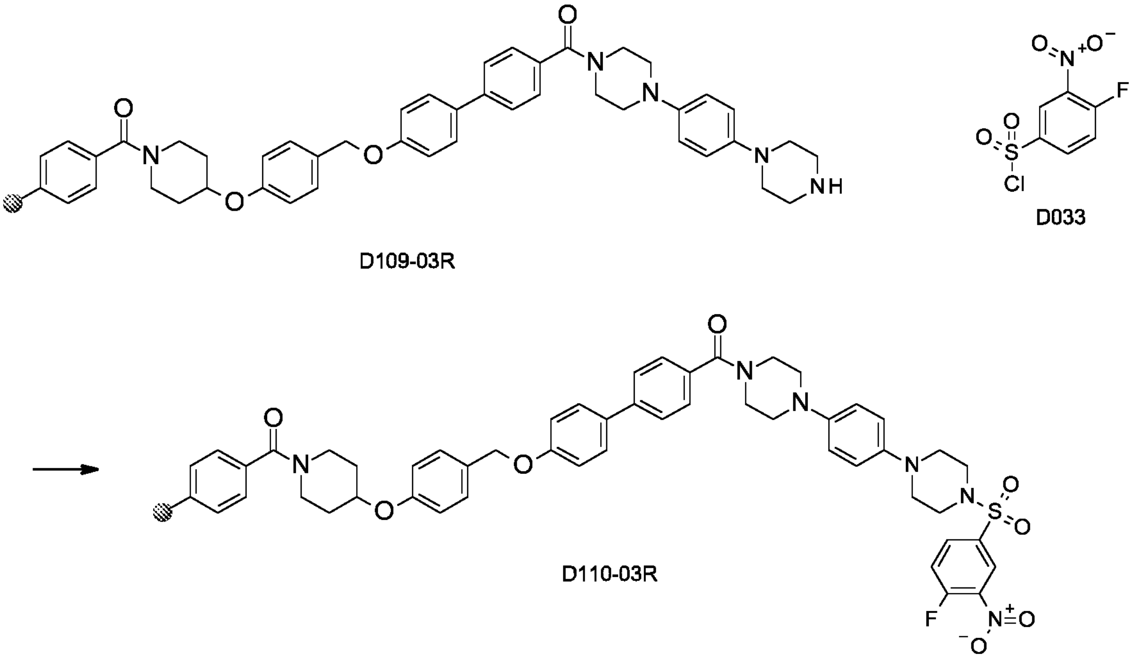

D109-03R

D033

D110-03R

Under a nitrogen atmosphere, solid-phase supported alkylamine (D109-03R) (0.187 mmol/g, 20 mg, 3.74 μmol), DTBP (52.8 μL, 0.240 mmol), and DCE (350 μL) were added to a 0.6 mL screw-cap vial and shaken at room temperature for 1 hour. Under a nitrogen atmosphere, a solution of DIPEA (2.1 μL, 0.012 mmol) and 4-fluoro-3- nitrobenzenesulfonyl chloride (D033) (9.58 mg, 0.040 mmol) in DCE (50 μL) was added thereto, and the obtained mixture was shaken at 60°C for 23 hours.

The reaction was monitored as follows. Under a nitrogen atmosphere, 12 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.05 M pentamethylbenzene (50 μL) for 5 minutes. The cleavage solution was filtered, and the solid phase was washed with NMP (50 μL). The obtained filtrate was diluted with MeCN (500 μL) to prepare an LC sample. The degree of progression of the reaction was measured by LC-MS analysis. As a result, 87.7 area% of compound D110 of interest was observed.

[Formula 425]

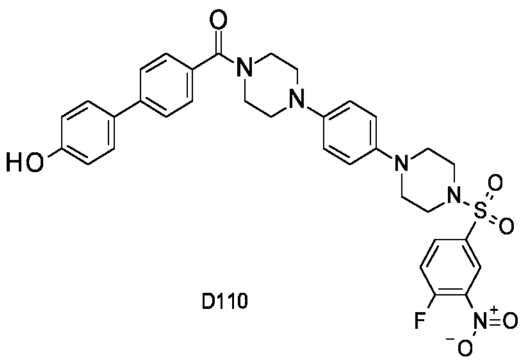

D110

Compound D110

LCMS: m/z 646[M+H]$^+$.

Retention time: 1.113 min (analysis conditions SMD-FA05-1, 290 nm).

Example 2-11-7-2: Solid-phase experiment to confirm progression of sulfonamidation reaction with sulfonyl chloride(D033) or (D113) using solid-phase supported primary alkylamine (D111-03R)

The sulfonamidation of solid-phase supported alkylamine (D111-03R) with sulfonyl chloride (D033) was performed under the same reaction conditions as in Example 2-11-7-1 (run 1 in Table 2-11-6). As a result, 31.0 area% of the target compound (D1 14) was observed. At the same time therewith, there arose 50.1 area% of a by-product (D115) presumably resulting from the aromatic nucleophilic substitution reaction of solid-phase supported alkylamine (D111-03R) with the fluorine atom contained in sulfonyl chloride (D033). In run 2, 4-chloro-3-nitrobenzenesulfonyl chloride (D113) having a chlorine atom on the aromatic ring was used together with 2,6-lutidine for the purpose of preventing the undesirable aromatic nucleophilic substitution reaction. As a result, 87.0 area% of compound D112 of interest was observed. The reaction operation of run 2 in Table 2-11-6 will be shown as a typical example. The same experimental operation as in run 2 was performed for run 1.

The sulfonamidation reaction of solid-phase supported alkylamine (D111-03R) was performed to synthesize a compound (D112-03R) as follows (exemplary reaction operation).

[Formula 426]

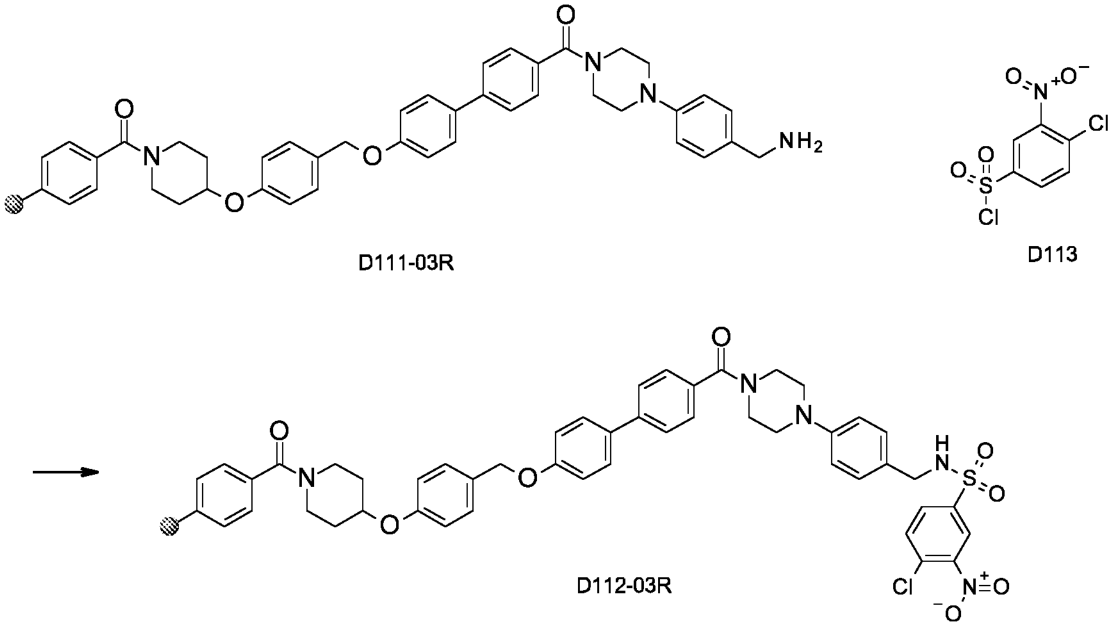

D111-03R

D113

D112-03R

Under a nitrogen atmosphere, solid-phase supported alkylamine (D111-03R) (0.193 mmol/g, 20 mg, 3.86 μmol) and DCE (400 μL) were added to a 0.6 mL screw-cap vial and shaken at room temperature for 1 hour. Under a nitrogen atmosphere, 2,6-lutidine (27 μL, 0.232 mmol) and 4-chloro-3-nitrobenzenesulfonyl chloride (D113) (9.88 mg, 0.039 mmol) were added thereto, and the obtained mixture was shaken at 60°C for 3 hours.

The reaction was monitored as follows. Under a nitrogen atmosphere, 12 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.05 M pentamethylbenzene (50 μL) for 5 minutes. The cleavage solution was filtered, and the solid phase was washed with NMP (50 μL). The obtained filtrate was diluted with MeCN (500 μL) to prepare an LC sample. The degree of progression of the reaction was measured by LC-MS analysis. As a result, 88.3 area% of compound D112 of interest was observed.

[Formula 427]

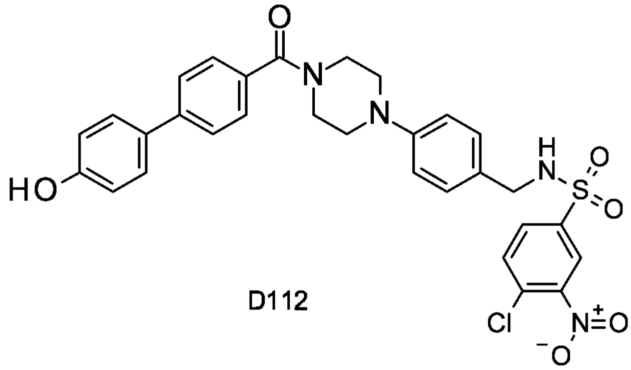

D112

Compound D112

LCMS: m/z 607[M+H]+.

Retention time: 1.099 min (analysis conditions SMD-FA05-1, 299 nm).

[Table 65]

| Run | Sulfonyl chloride | base (eq.) | UV area% | |
|---|---|---|---|---|
| | | | TM | BP ($S_NAr$) |
| 1 | D033 | DTBP (60 eq.), DIPEA (3 eq.) | 31.0 (D114) | 50.1 (D115) |
| 2 | D113 | 2,6-lutidine (15 eq.) | 88.3 (D112) | ND |

Results of analyzing the target compound (D114) and the by-product (D115, structure: putative) in run 1 in Table 2-11-6 are shown below.

[Formula 428]

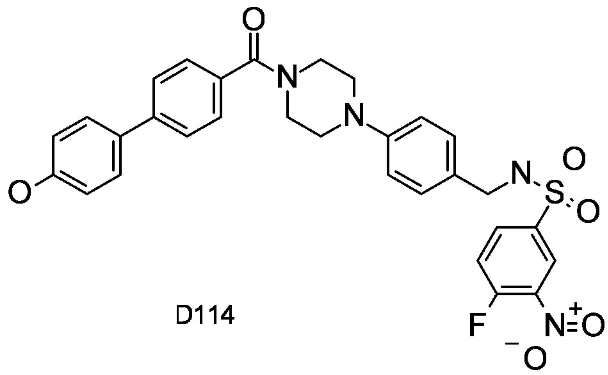

D114

Compound D114

LCMS: m/z 591[M+H]+.

Retention time: 1.061 min (analysis conditions SMD-FA05-1, 299 nm).

[Formula 429]

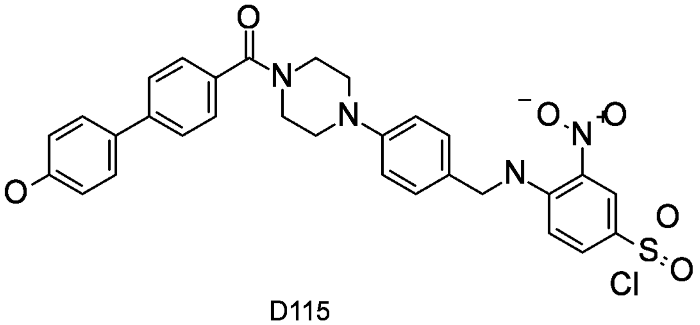

D115

Compound D115

LCMS: m/z 607[M+H]+.

Retention time: 1.255 min (analysis conditions SMD-FA05-1, 299 nm).

The fluorine atom of D033 used under the conditions shown in run 1 in Table 2-11-6 was introduced for the purpose of further forming a bond through aromatic nucleophilic substitution reaction after sulfonamide bond formation. On the other hand, it can be expected that the same purpose as above is also achieved by replacing the fluorine atom with a chlorine atom, as in 4-chloro-3-nitrobenzenesulfonyl chloride (D113).

Example 2-11-8-1-1: Solid-phase experiment to perform sulfonamidation reaction using solid-phase supported Ar-NHMe (D020-03R) and phenylmethanesulfonyl chloride (D100)

Sulfonamidation using solid-phase supported Ar-NHMe (D020-03R) and phenylmethanesulfonyl chloride (D100) was carried out under the reaction conditions using pyridine and NMI as a base in THF which were found in Example 2-11-3.

Solid-phase experiment (exemplary reaction operation) of sulfonamidation reaction using solid-phase supported Ar-NHMe (D020-03R) and phenylmethanesulfonyl chloride (D100)

[Formula 430]

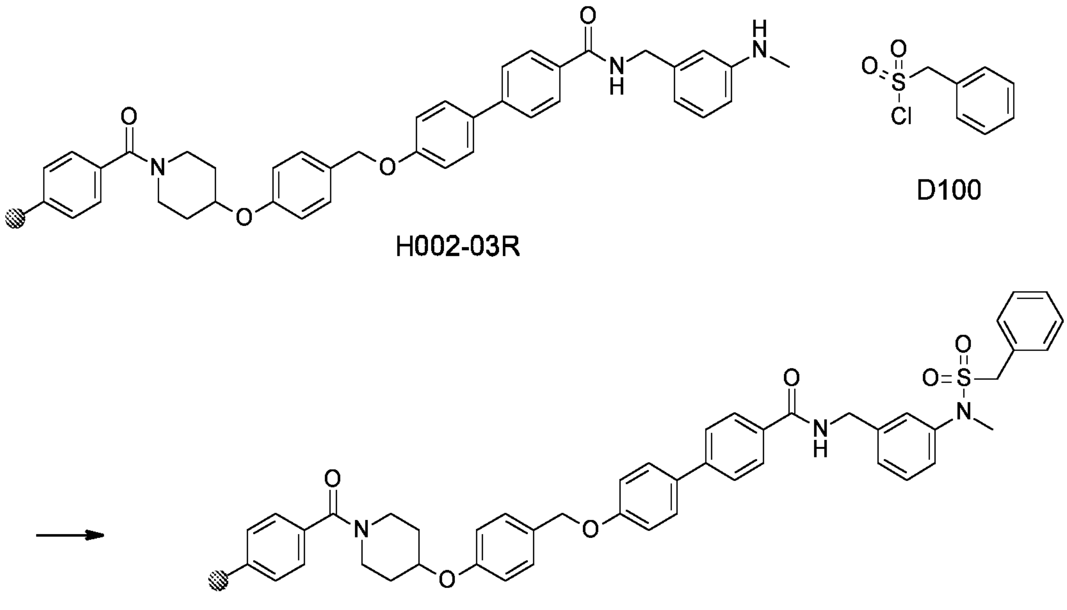

H002-03R

D100

D101-03R

Under a nitrogen atmosphere, solid-phase supported Ar-NHMe (H002-03R, 0.196 mmol/g, 20.0 mg, 3.9 μmol) and THF (240 μL) were added to a 0.6 mL screw-cap vial and shaken at room temperature for 1 hour.

Under a nitrogen atmosphere, pyridine (4.8 μL, 0.059 mmol), NMI (1.6 μL, 2.0 μmol), and phenylmethanesulfonyl chloride (D100) (7.5 mg, 0.039 mmol) were added thereto, and the mixture was shaken at room temperature for 24 hours.

The reaction was monitored as follows.

Under a nitrogen atmosphere, 12 μL of the resin suspension was sampled, then transferred to a pipette tip with a filter, washed three times with DMA (100 μL), three times with MeOH (100 μL), and three times with DCM (100 μL), and then dipped in a 10% solution of TFA in DCM containing 0.05 M pentamethylbenzene (50 μL) for 5 minutes. The cleavage solution was filtered, and the solid phase was washed with DMA (50 μL). The obtained filtrate was diluted with MeCN (500 μL) to prepare an LC sample. The degree of progression of the reaction was measured by LC-MS analysis. As a result, 95.9 area% of (compound D101) of interest was observed.

[Formula 431]

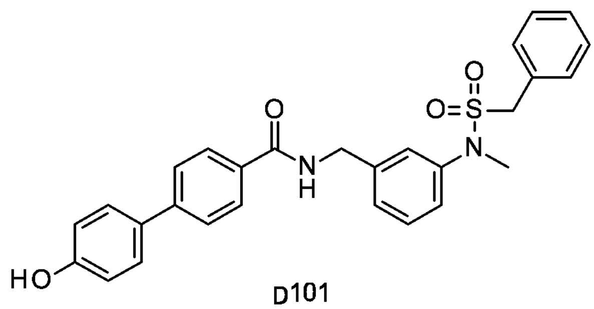

D101

Compound D101

LCMS: m/z 487[M+H]+.

Retention time: 1.056 min (analysis conditions SMD-FA05-1, 299 nm).

Example 2-11-8-1-2: Solid-phase experiment to perform sulfonamidation reaction using solid-phase supported Ar-NHMe (D020-03R) and 2-naphthalen-1-ylethanesulfonyl chloride (D102)

Sulfonamidation was carried out by the same approach as in Example 2-11-6-1-1 using solid-phase supported Ar-NHMe (D020-03R) and 2-naphthalen-1-ylethanesulfonyl chloride (D102). The obtained results are shown in run 1 in Table 2-11-7. First, in the case of performing reaction at room temperature for 24 hours using pyridine (15 equivalents) and NMI (5 equivalents) as a base in THF, 82.5 area% of compound D103 of interest was obtained, whereas 14.4% of starting material H002 remained (run 1). Next, as a result of changing the solvent to DCM, the efficiency of reaction was improved, and 91.8 area% of the target compound was obtained (run 2). As a result of changing the base to 4-tBu-pyridine (15 equivalents) in run 3, the purity of the target compound (D103) was improved to 92.2%. As a result of further attempting conditions using 3,4-lutidine (15 equivalents) in THF, the formation of 93.8 area% of compound D103 of interest was observed.

[Table 66]

| [Table 2-11-7] | | | | |
|---|---|---|---|---|
| Run | base (eq.) solvent | | UV area% | |
| | | | TM (D103) | SM (H002) |
| 1 | pyridine(15 eq.), NMI (5 eq.) | THF | 82.5 | 14.4 |
| 2 | | DCM | 91.8 | 5.3 |
| 3 | 4-tBu-pyridine (15 eq.) | DCM | 92.2 | 2.7 |
| 4 | 3,4-lutidine (15 eq.) | THF | 93.8 | 3.2 |

The experimental operation under reaction conditions using 3,4-lutidine (15 equivalents) in THF in run 4 in

Table 2-11-7 will be shown below.

Solid-phase experiment (exemplary reaction operation) of sulfonamidation reaction using solid-phase supported Ar-NHMe (H002-03R) and 2-naphthalen-1-ylethanesulfonyl chloride (D102)

[Formula 432]

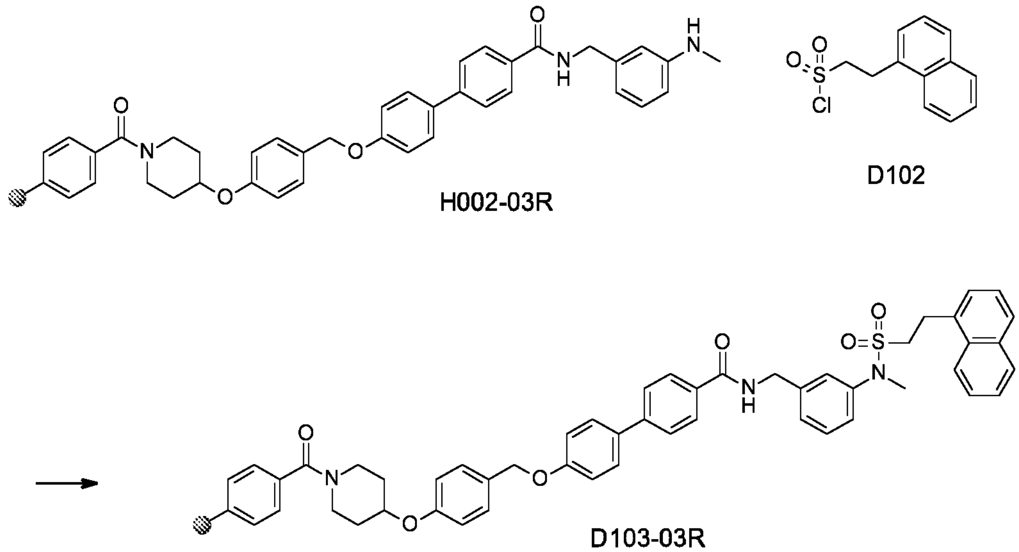

H002-03R

D102

D103-03R

Under a nitrogen atmosphere, solid-phase supported Ar-NHMe (H002-03R, 0.196 mmol/g, 20.0 mg, 3.9 μmol) and THF (240 μL) were added to a 0.6 mL screw-cap vial and shaken at room temperature for 1 hour.
Under a nitrogen atmosphere, 3,4-lutidine (6.56 μL, 0.059 mmol) and 2-naphthalen- 1-ylethanesulfonyl chloride (D102) (9.4 mg, 0.039 mmol) were added thereto, and the obtained mixture was shaken at room temperature for 24 hours.
The reaction was monitored as follows.
Under a nitrogen atmosphere, 12 μL of the resin suspension was sampled, then transferred to a pipette tip with a filter, washed three times with DMA (100 μL), three times with MeOH (100 μL), and three times with DCM (100 μL), and then dipped in a 10% solution of TFA in DCM containing 0.05 M pentamethylbenzene (50 μL) for 5 minutes. The cleavage solution was filtered, and the solid phase was washed with DMA (50 μL). The obtained filtrate was diluted with MeCN (500 μL) to prepare an LC sample. The degree of progression of the reaction was measured by LC-MS analysis. As a result, 93.8 area% of compound D103 of interest was observed.

[Formula 433]

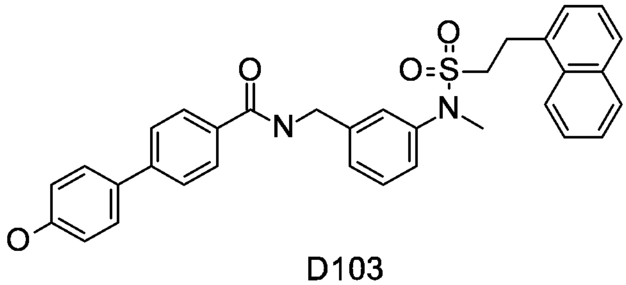

D103

Compound D103
LCMS: m/z 551[M+H]$^+$.
Retention time: 1.199 min (analysis conditions SMD-FA05-1, 299 nm).

Example 2-11-8-2-1: Solid-phase experiment to perform sulfonamidation reaction using solid-phase supported amine (H003-03R) and phenylmethanesulfonyl chloride (D100)

The sulfonamidation of solid-phase supported amine (D021-03R) with phenylmethanesulfonyl chloride (D100) was carried out under the reaction conditions using pyridine and NMI as a base in THF which were found in Example 2-11-3. The experimental operation will be shown below.

Solid-phase experiment (exemplary reaction operation) of sulfonamidation reaction using solid-phase supported amine (H003-03R) and phenylmethanesulfonyl chloride (D100)

[Formula 434]

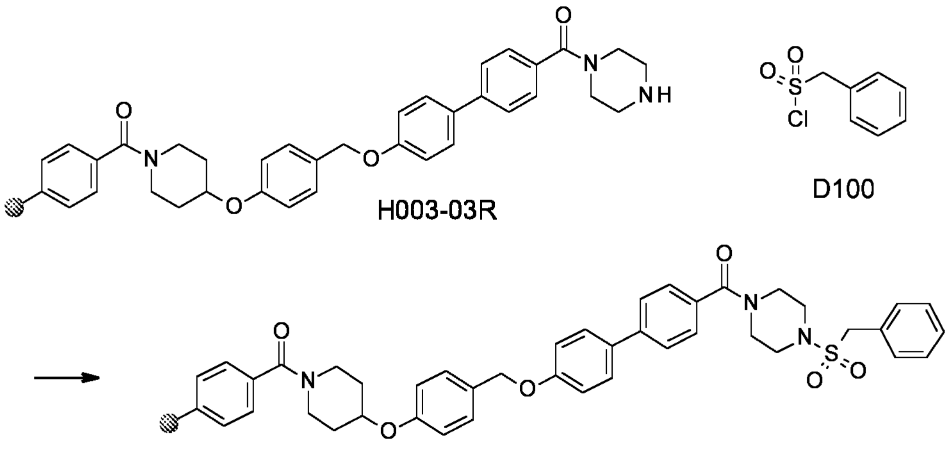

H003-03R

D100

D104-03R

Under a nitrogen atmosphere, solid-phase supported amine (H003-03R) (0.197 mmol/g, 20.0 mg, 3.9 μmol) and THF (240 μL) were added to a 0.6 mL screw-cap vial and shaken at room temperature for 1 hour.

Under a nitrogen atmosphere, pyridine (4.8 μL, 0.059 mmol), NMI (1.6 μL, 2.0 μmol), and phenylmethanesulfonyl chloride (D100) (7.5 mg, 0.039 mmol) were added thereto, and the obtained mixture was shaken at room temperature for 7 hours.

The reaction was monitored as follows.

Under a nitrogen atmosphere, 12 μL of the resin suspension was sampled, then transferred to a pipette tip with a filter, washed three times with DMA (100 μL), three times with MeOH (100 μL), and three times with DCM (100 μL), and then dipped in a 10% solution of TFA in DCM containing 0.05 M pentamethylbenzene (50 μL) for 5 minutes. The cleavage solution was filtered, and the solid phase was washed with DMA (50 μL). The obtained filtrate was diluted with MeCN (500 μL) to prepare an LC sample. The degree of progression of the reaction was measured by LC-MS analysis. As a result, 97.2 area% of compound D104 of interest was observed.

[Formula 435]

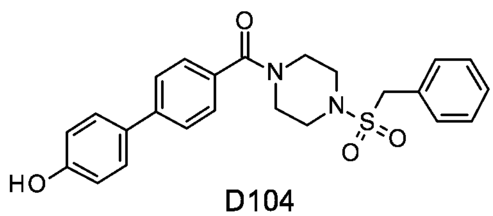

D104

Compound D104

LCMS: m/z 437[M+H]$^+$.

Retention time: 0.975 min (analysis conditions SMD-FA05-1, 299 nm).

Example 2-11-8-2-2: Solid-phase experiment to perform sulfonamidation reaction using solid-phase supported amine (H003-03R) and 2-naphthalen-1-ylethanesulfonyl chloride (D102)

Sulfonamidation using solid-phase supported amine (H003-03R) and 2-naphthalen- 1-ylethanesulfonyl chloride (D102) was carried out under the reaction conditions using pyridine and NMI as a base in DCM which were found in Example 2-11-6-1-2. The experimental operation will be shown below.

Solid-phase experiment (exemplary reaction operation) of sulfonamidation reaction using solid-phase supported amine (H003-03R) and 2-naphthalen-1-ylethanesulfonyl chloride (D102)

[Formula 436]

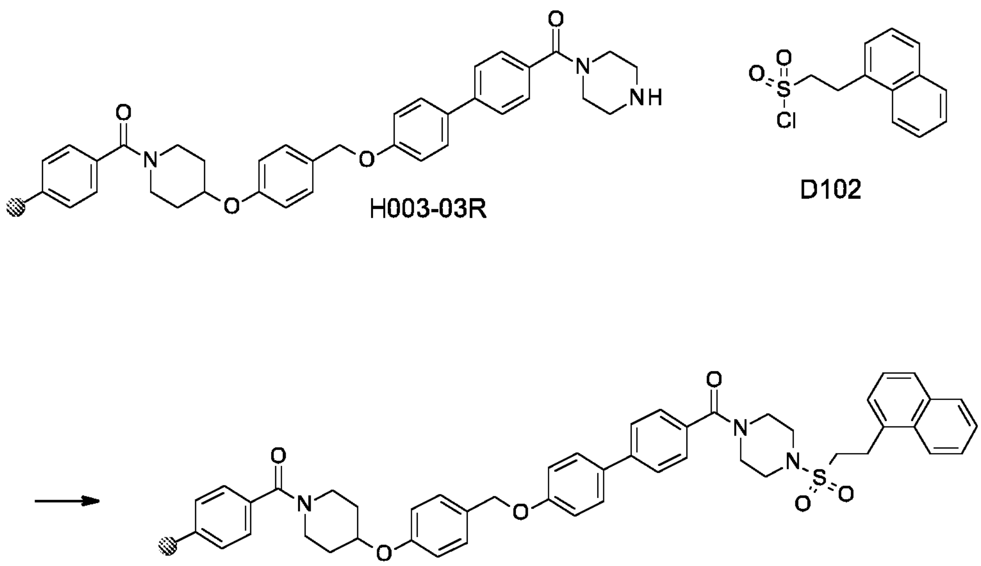

H003-03R

D102

D105-03R

Under a nitrogen atmosphere, solid-phase supported amine (H003-03R) (0.197 mmol/g, 20.0 mg, 3.9 μmol) and THF (240 μL) were added to a 0.6 mL screw-cap vial and shaken at room temperature for 1 hour.

Under a nitrogen atmosphere, pyridine (4.8 μL, 0.059 mmol), NMI (1.6 μL, 2.0 μmol), and 2-naphthalen-1-ylethanesulfonyl chloride (D102) (10.4 mg, 0.039 mmol) were added thereto, and the obtained mixture was shaken at room temperature for 7 hours.

The reaction was monitored as follows.

Under a nitrogen atmosphere, 12 μL of the resin suspension was sampled, then transferred to a pipette tip with a filter, washed three times with DMA (100 μL), three times with MeOH (100 μL), and three times with DCM (100 μL), and then dipped in a 10% solution of TFA in DCM containing 0.05 M pentamethylbenzene (50 μL) for 5 minutes. The cleavage solution was filtered, and the solid phase was washed with DMA (50 μL). The obtained filtrate was diluted with MeCN (500 μL) to prepare an LC sample. The degree of progression of the reaction was measured by LC-MS analysis. As a result, 94.8 area% of compound D105 of interest was observed.

[Formula 437]

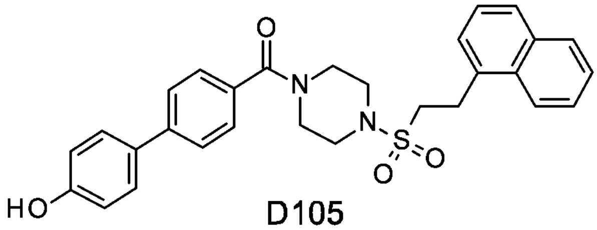

D105

Compound D105

LCMS: m/z 501[M+H]+.

Retention time: 1.144 min (analysis conditions SMD-FA05-1, 299 nm).

Example 2-11-9-1: Solid-phase experiment to perform sulfonamidation reaction using solid-phase supported sulfonyl chloride (D095-03R)

Compound D097-03R was synthesized as follows through the sulfonamidation of solid-phase supported sulfonyl chloride (D095-03R).

[Formula 438]

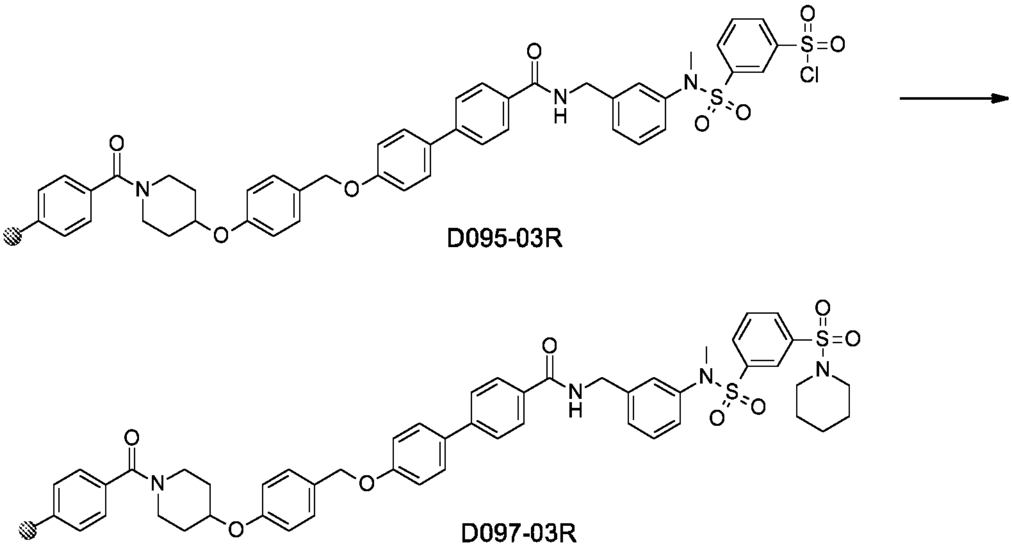

D095-03R

D097-03R

Under a nitrogen atmosphere, compound D095-03R (loading rate: 0.187 mmol/g, 18.9 mg, 3.50 mmol) and THF (0.374 mL) were added to a 0.6 mL glass vial and shaken at room temperature for 1 hour. Piperidine (6.92 μL, 0.070 mmol) was added thereto, and the mixture was shaken at room temperature for 2 hours.

Reaction monitoring

The suspension of the reaction solution and the resin (12 μL) was transferred onto a filter, washed three times with DMA (0.1 mL), three times with methanol (0.1 mL), and three times with DCM (0.1 mL), and then dipped in a 10% solution of TFA in DCM containing 0.05 M pentamethylbenzene (0.1 mL) for 5 minutes. After filtration, the resin on the filter was washed with DMA (0.05 mL). Acetonitrile (0.50 mL) was added to the obtained filtrate to prepare an LC sample. The degree of progression of the reaction was measured by LC-MS analysis. As a result, compound D097 of interest was observed at a purity of 85.2%.

[Formula 439]

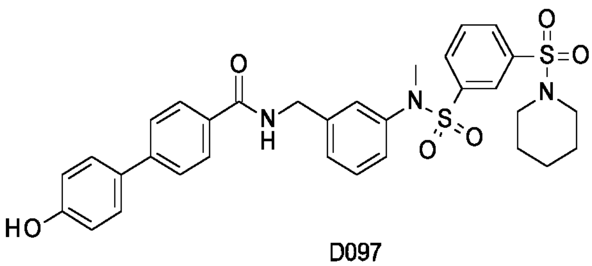

D097

Compound D097

LRMS: m/z 620[M+H]+.

Retention time: 2.483 min (analysis conditions SMD-TFA05-RP-long, 299 nm).

Example 2-11-9-2: Solid-phase experiment to synthesize sulfonamide (D094-03R) using solid-phase supported sulfinic acid (D093)

Sulfonamide (D094-03R) was synthesized as follows using solid-phase supported sulfinic acid (compound D093-03R), NCS, and piperidine.

[Formula 440]

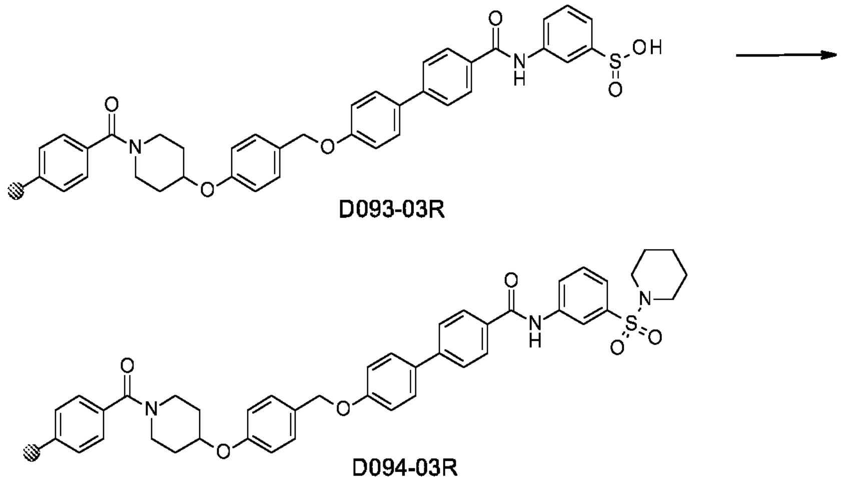

D093-03R

D094-03R

Under a nitrogen atmosphere, compound D093-03R (18.9 mg) and THF (0.3 mL) were added to a 0.6 mL glass vial and shaken at room temperature for 1 hour. A solution of piperidine (11.47 μL, 0.116 mmol) and NCS (2.58 mg, 0.019 mmol) in THF (100 μL) was added thereto and shaken at room temperature for 1 hour.

Reaction monitoring

The suspension of the reaction solution and the resin (12 μL) was transferred onto a filter, washed three times with NMP (0.1 mL), three times with methanol (0.1 mL), and three times with DCM (0.1 mL), and then dipped in a 10% solution of TFA in DCM containing 0.05 M pentamethylbenzene (0.05 mL) for 5 minutes. After filtration, the resin on the filter was washed with DMA (0.05 mL). Acetonitrile (0.50 mL) was added to the obtained filtrate to prepare an LC sample. The degree of progression of the reaction was measured by LC-MS analysis. As a result, compound D094 of interest was observed at a purity of 86.7%.

[Formula 441]

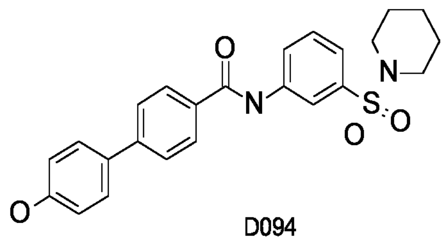

D094

Compound D094
LRMS: m/z 437[M+H]+.
Retention time: 1.132 min (analysis conditions SMD-FA05-1, 299 nm).

Example 2-12-1: Solid-phase experiment to synthesize solid-phase supported sulfone using solid-phase supported ArI (D090-03R) and DABSO

Sulfone (D099-03R) was synthesized with reference to a non patent literature (Angew. Chem. Int. Ed. 2014, 53, 10204-10208) using solid-phase supported ArI (D090- 03R) and DABSO.

Solid-phase experiment (exemplary reaction operation) to synthesize sulfone (D099- 03R) using solid-phase supported ArI (D090-03R) and DABSO

[Formula 442]

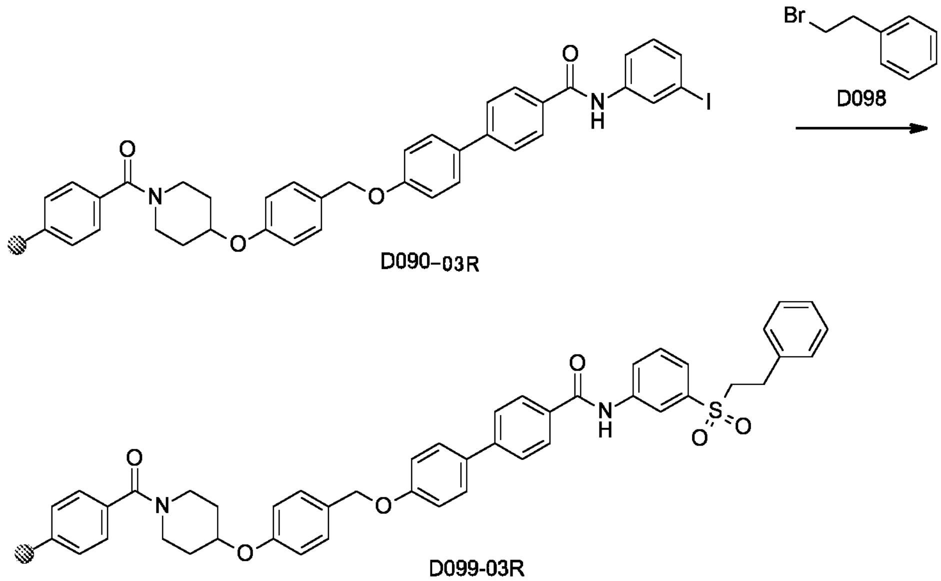
D098

D090-03R

D099-03R

Under a nitrogen atmosphere, solid-phase supported ArI (D090-03R) (0.193 mmol/g, 20.0 mg, 3.9 μmol), DABSO (9.3 mg, 0.039 mmol), and 1,4-dioxane (400 μL) were added to a 0.6 mL screw-cap vial and shaken at room temperature for 1 hour. XPhos Pd G4 (3.3 mg, 3.9 μmol) and BTMG (7.7 μL, 0.039 mmol) were added thereto, and the mixture was shaken at 80°C for 4 hours. Then, 2-bromoethylbenzene (D098) (10.51 μL, 0.077 mmol) was added thereto, and the mixture was shaken at 80°C for 2 hours.

The reaction was monitored as follows. Under a nitrogen atmosphere, 12 μL of the resin suspension was sampled, then transferred to a pipette tip with a filter, washed three times with NMP (100 μL), three times with MeOH (100 μL), and three times with DCM (100 μL), and then dipped in a 10% solution of TFA in DCM containing 0.05 M pentamethylbenzene (50 μL) for 5 minutes. The cleavage solution was filtered, and the solid phase was washed with DMA (50 μL). The obtained filtrate was diluted with MeCN (500 μL) to prepare an LC sample. The degree of progression of the reaction was measured by LC-MS analysis. As a result, 79.1 area% of compound D099 of interest was observed.

[Formula 443]

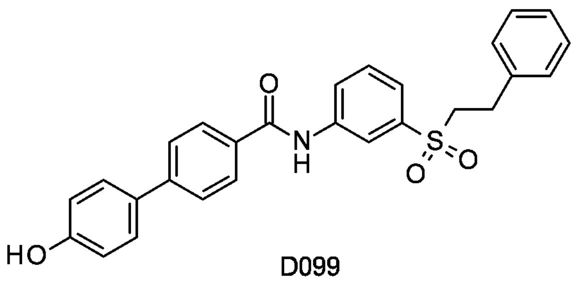

D099

Compound D099

LCMS: m/z 458[M+H]$^+$.

Retention time: 1.135 min (analysis conditions SMD-FA05-1, 299 nm).

As described above, it was confirmed that corresponding sulfone D099-03R can be obtained using solid-phase supported ArI (D090-03R), DABSO and an alkylating agent (D098). The exploitation of this reaction enables various molecules having solid-phase supported sulfone as a substructure to be synthesized.

Example 2-13: Experiment to confirm scope of substrate application to C-N bond formation reaction as typical example of identifying substrate scope that can be applied to library synthesis

Hereinafter, an exemplary method for identifying an applicable substrate scope under reaction conditions expected to attain a wide scope of substrate application to C-N bond formation reaction will be illustrated. The scope given below is a mere illustration of an exemplary identification method, and the present invention is not limited by the scope given below in actual application to library synthesis.

Example 2-13-1: Solid-phase experiment to confirm scope of substrate application to nucleophilic substitution reaction of amine with alkyl halide or alcohol derivative as typical example of identifying substrate scope that can be applied to library synthesis

Reductive amination reaction as well as nucleophilic substitution reaction with alkyl halide or an alcohol derivative is widely accepted as a methodology for the alkylation of secondary amine. The success or failure of C-N bond formation was confirmed below under reaction conditions expected to attain a wide scope of substrate application to the alkylation reaction of solid-phase amine with alkyl halide or an alcohol derivative. The substrate is not limited to those given below in actual application to library synthesis.

Example 2-13-1-1: Solid-phase experiment on nucleophilic substitution reaction of NHR1R2 with alkyl halide

The nucleophilic substitution reaction of solid-phase supported amine with alkyl halide was carried out in NMP by selecting $Na_2CO_3$ as a base. More specifically, the nucleophilic substitution reaction (synthesis of H105-03R) of solid-phase supported amine (B012-03R) with (2-bromoethyl)benzene (H204) using $Na_2CO_3$ as a base will be illustrated.

[Formula 444]

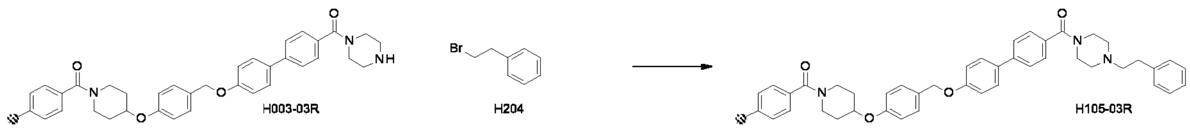

Under a nitrogen atmosphere, solid-phase supported amine (H003-03R) (loading rate: 0.197 mmol/g, 20 mg, 0.0039 mmol), $Na_2CO_3$ (5.01 mg, 0.047 mmol), and NMP (0.4 mL) were added to a 0.6 mL glass vial and shaken at room temperature for 1 hour. Under a nitrogen atmosphere, (2-bromoethyl)benzene (H204) (5.4 µL, 0.039 mmol) was added thereto, and the mixture was shaken at 60°C for 12 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 86% of compound H105 of interest was observed.

[Formula 445]

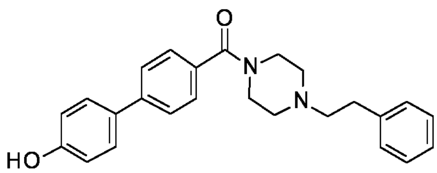

Compound H105

LRMS: m/z 387[M+H]+

Retention time: 0.677 min (analysis conditions SMD-FA05-1, 299 nm)

Example 2-13-1-2: Solid-phase experiment on nucleophilic substitution reaction of NHR1R2 with alcohol derivative

The nucleophilic substitution reaction of solid-phase supported amine with an alcohol derivative was carried out in THF by selecting tetrakistriphenylphosphine palladium as an activator. More specifically, the nucleophilic substitution reaction (synthesis of H106- 03R) of solid-phase supported amine (H003-03R) with allylmethyl carbonate (H205) using tetrakistriphenylphosphine palladium as an activator will be illustrated.

[Formula 446]

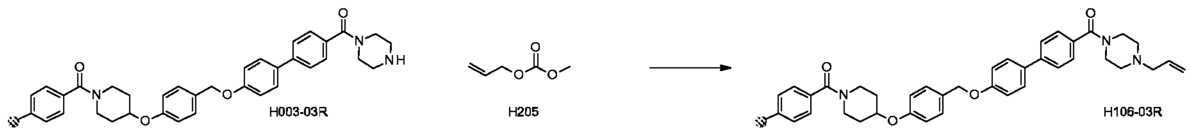


Under a nitrogen atmosphere, solid-phase supported amine (H003-03R) (loading rate: 0.197 mmol/g, 20 mg, 0.0039 mmol) and THF (0.4 mL) were added to a 0.6 mL glass vial and shaken at room temperature for 1 hour. Under a nitrogen atmosphere, allylmethyl carbonate (H205) (9.0 μL, 0.079 mmol) and tetrakistriphenylphosphine palladium (4.6 mg, 0.0039 mmol) were added thereto, and the mixture was shaken at 25°C for 2 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 89% of compound H106 of interest was observed.

[Formula 447]

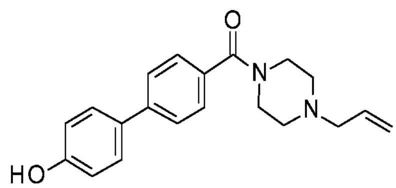

Compound H106

LRMS: m/z 323[M+H]+

Retention time: 0.537 min (analysis conditions SMD-FA05-1, 299 nm)

Thus, the reductive amination reaction described above as well as the nucleophilic substitution reaction of amine with an alcohol derivative or alkyl halide using a base or a transition metal was found applicable to the construction of a C-N bond.

Example 2-13-2: Solid-phase experiment to confirm scope of substrate application to formal alkylation reaction of primary amine as typical example of identifying substrate scope that can be applied to library synthesis

The alkylation reaction of secondary amine is described above in Examples. In general, overreaction is of concern for primary amine. Accordingly, the formal monoalkylation of primary amine by the alkylation of an o-nitrobenzenesulfonamide derivative followed by the deprotection of a sulfonyl group is widely used. Hereinafter, exemplary alkylation reaction of solid-phase sulfonamide with alkyl halide or an alcohol will be illustrated. The substrate given below is a mere illustration of an exemplary identification method, and the substrate is not limited to those given below in actual application to library synthesis.

Example 2-13-2-1: Solid-phase experiment on nucleophilic substitution reaction of solid-phase supported sulfonamide with alcohol derivative

The nucleophilic substitution reaction of solid-phase supported amine with an alcohol derivative was carried

out in THF by selecting CMMP as an activator. More specifically, the nucleophilic substitution reaction (synthesis of H107-03R) of solid-phase supported sulfonamide (H004-03R) with 2-(3-bromophenyl)ethanol (H206) using CMMP as an activator will be illustrated.

[Formula 448]

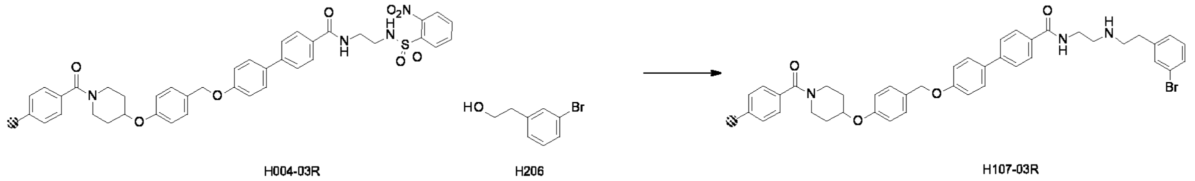


Under a nitrogen atmosphere, solid-phase supported sulfonamide (H004-03R) (loading rate: 0.192 mmol/g, 20 mg, 0.0038 mmol), 2-(3-bromophenyl)ethanol (H206) (10.4 μL, 0.077 mmol), and THF (0.4 mL) were added to a 0.6 mL glass vial and shaken at room temperature for 1 hour. Under a nitrogen atmosphere, CMMP (77.0 μL, 0.038 mmol, 0.5 M in THF) was added thereto, and the mixture was shaken at 25°C for 12 hours. Then, under a nitrogen atmosphere, dodecane-1-thiol (37.5 μL, 0.154 mmol) and phosphazene base PltBu (14.7 μL, 0.058 mmol) were added thereto, and the mixture was shaken at room temperature for 12 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 94% of compound H107 of interest was observed.

[Formula 449]

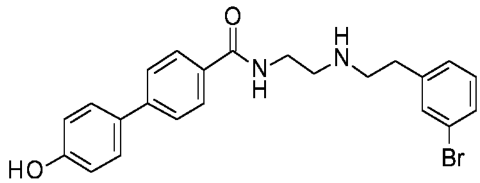

Compound H107

LRMS: m/z 439,441[M+H]+

Retention time: 0.768 min (analysis conditions SMD-FA05-1, 299 nm)

Example 2-13-2-2: Solid-phase experiment on nucleophilic substitution reaction of solid-phase supported sulfonamide with alkyl bromide

The nucleophilic substitution reaction of solid-phase supported sulfonamide with alkyl halide was carried out in NMP by selecting phosphazene base P1tBu as a base. More specifically, the nucleophilic substitution reaction (synthesis of H108-03R) of solid-phase supported sulfonamide (H004-03R) with (2-bromoethyl)benzene (H204) using phosphazene base PltBu as a base will be illustrated.

[Formula 450]

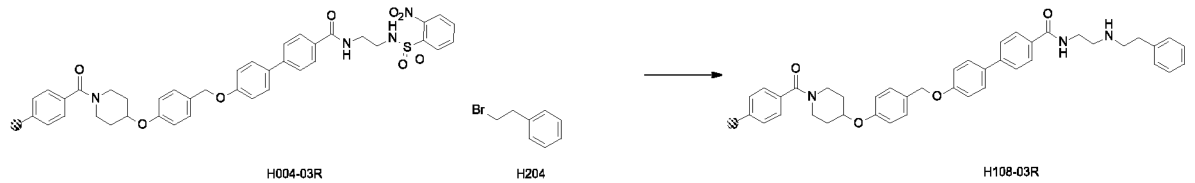


Under a nitrogen atmosphere, solid-phase supported sulfonamide (H004-03R) (loading rate: 0.192 mmol/g, 20 mg, 0.0038 mmol) and NMP (0.4 mL) were added to a 0.6 mL glass vial and shaken at room temperature for 1 hour. Under a nitrogen atmosphere, (2-bromoethyl)benzene (H204) (5.2 μL, 0.038 mmol) and phosphazene base PltBu (14.7 μL, 0.058 mmol) were added thereto, and the mixture was shaken at room temperature for 12 hours. Then, dodecane-1-thiol (36.6 μL, 0.154 mmol) and phosphazene base PltBu (14.7 μL, 0.058 mmol) were added thereto, and the mixture was shaken at room temperature for 12 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 95% of compound H108 of interest was observed.

[Formula 451]

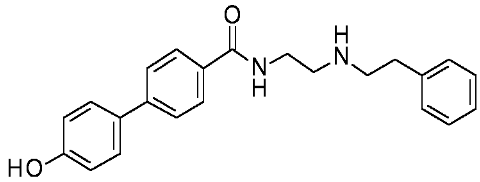

Compound H108

LRMS: m/z 361[M+H]+

Retention time: 0.697 min (analysis conditions SMD-FA05-1, 299 nm)

Example 2-13-3: Solid-phase experiment of coupling reaction of solid-phase supported Ar-Br with amide using Pd reagent

Coupling reaction with amide using a Pd reagent was carried out with reference to a non patent literature (Nature, 2018, 557, 228-232) by selecting an organic base MTBD (7- methyl-1,5,7-triazabicyclo[4.4.0]dec-5-ene) as a base and selecting tBuBrettphos Pd G4 as a Pd catalyst.

Example 2-13-3-1: Coupling reaction of solid-phase supported ArBr (C009-03R) with methyl 3-(aminocarbonyl)benzoate (B301)

[Formula 452]

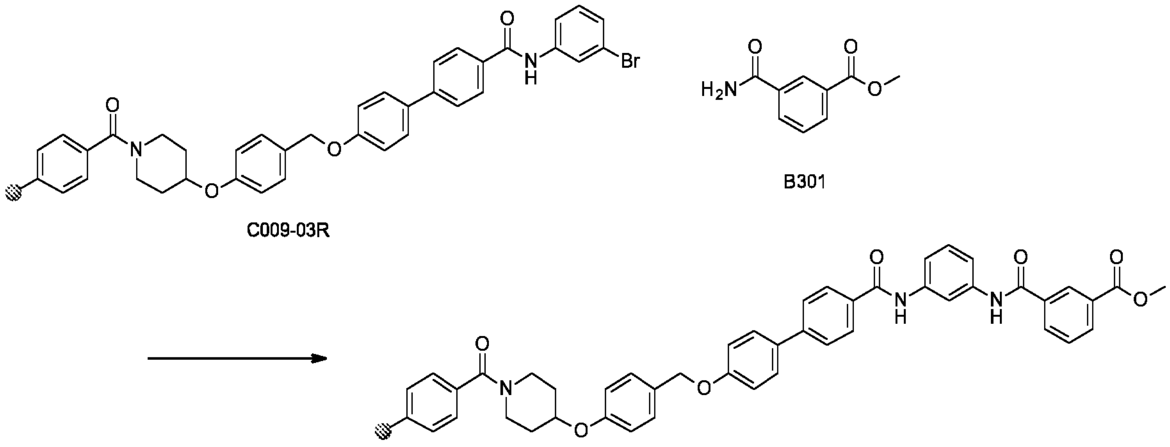

C009-03R

B301

B201-03R

Under a nitrogen atmosphere, solid-phase supported ArBr (C009-03R) (loading rate: 0.195 mmol/g, 20 mg, 0.0039 mmol) and THF (0.4 mL) were added to a 0.6 mL glass vial and shaken at room temperature for 1 hour. Methyl 3-(aminocarbonyl)benzoate (B301) (7.0 mg, 0.039 mmol), tBuBrettphos Pd G4 (3.39 mg, 0.0039 mmol), and MTBD (8.4 μL, 0.059 mmol) were added thereto, and the mixture was shaken at 60°C for 2.5 hours.

The suspension of the reaction solution and the resin was transferred onto a filter, washed three times with DMF (0.1 mL), three times with MeOH (0.1 mL), and three times with DCM (0.1 mL), and then dipped in a 10% solution

of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 96% of compound B201 of interest was observed.

[Formula 453]

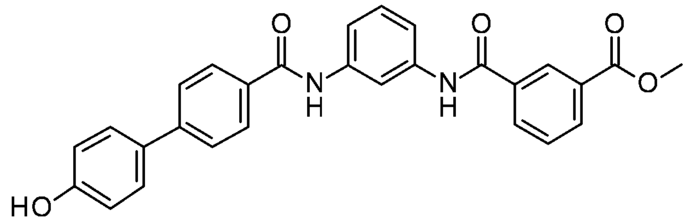

Compound B201

LRMS: m/z 467[M+H]+

Retention time: 1.060 min (analysis conditions SMD-FA05-1, 299 nm)

Example 2-13-3-2: Coupling reaction of solid-phase supported ArBr (C009-03R) with methyl 5-oxopyrrolidine-3-carboxylate (B302)

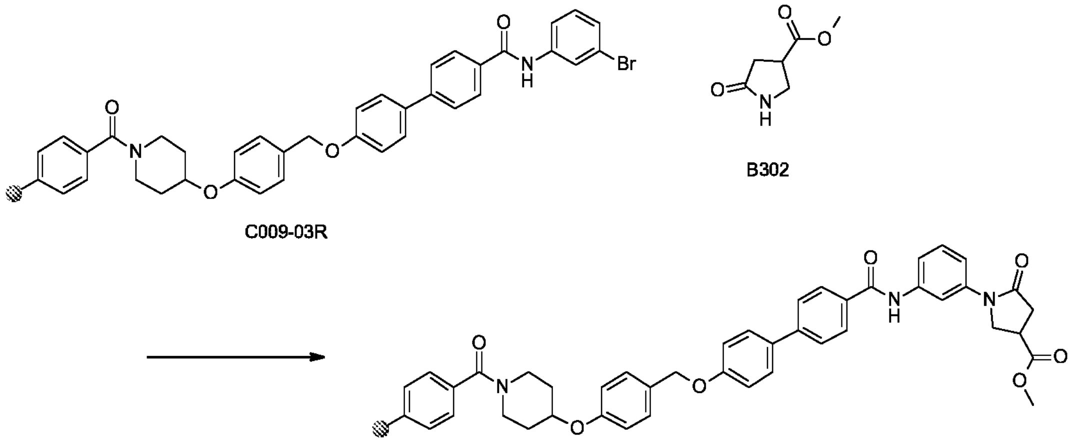

C009-03R

B302

B202-03R

Under a nitrogen atmosphere, solid-phase supported ArBr (C009-03R) (loading rate: 0.195 mmol/g, 20 mg, 0.0039 mmol) and THF (0.4 mL) were added to a 0.6 mL glass vial and shaken at room temperature for 1 hour. Methyl 5-oxopyrrolidine-3-carboxylate (B302) (5.6 mg, 0.039 mmol), tBuBrettphos Pd G4 (3.39 mg, 0.0039 mmol), and MTBD (8.4 μL, 0.059 mmol) were added thereto, and the mixture was shaken at 60°C for 2.5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 89% of compound B202 of interest was observed.

[Formula 454]

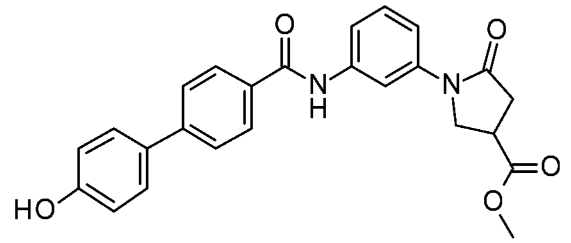

Compound B202

LRMS: m/z 431 $[M+H]^+$

Retention time: 0.923 min (analysis conditions SMD-FA05-1, 299 nm)

Example 2-13-3-3: Coupling reaction of solid-phase supported ArBr (B101-03R) with methyl 3-(aminocarbonyl)benzoate (B301)

[Formula 455]

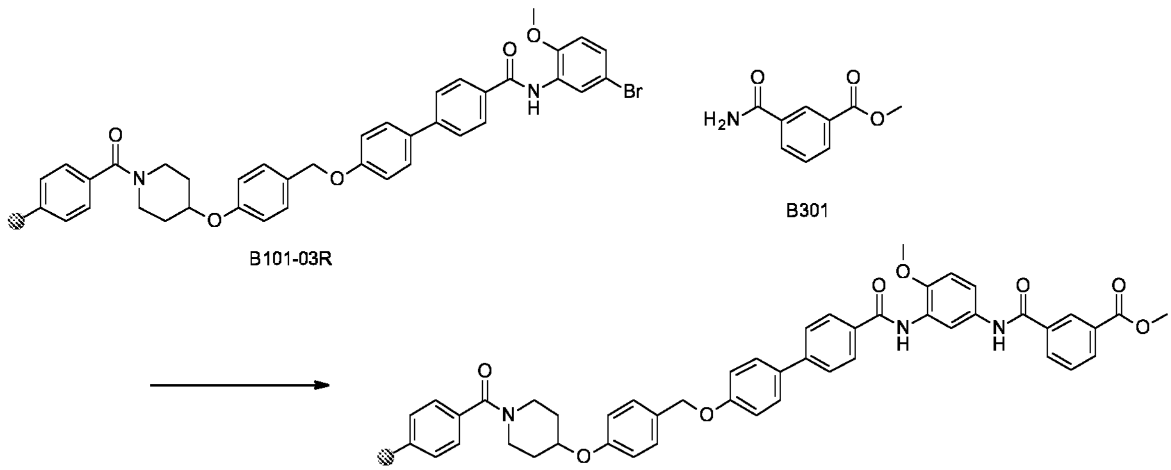

B101-03R

B301

B203-03R

Under a nitrogen atmosphere, solid-phase supported ArBr (B101-03R) (loading rate: 0.194 mmol/g, 20 mg, 0.0039 mmol) and THF (0.4 mL) were added to a 0.6 mL glass vial and shaken at room temperature for 1 hour. Methyl 3-(aminocarbonyl)benzoate (B301) (7.0 mg, 0.039 mmol), tBuBrettphos Pd G4 (3.37 mg, 0.0039 mmol), and MTBD (8.4 μL, 0.058 mmol) were added thereto, and the mixture was shaken at 60°C for 2.5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 83% of compound B203 of interest was observed.

[Formula 456]

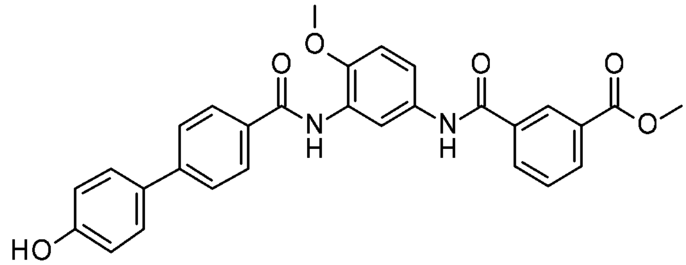

Compound B203

LRMS: m/z 497[M+H]+

Retention time: 1.089 min (analysis conditions SMD-FA05-1, 299 nm)

Example 2-13-3-4: Coupling reaction of solid-phase supported ArBr (B101-03R) with methyl 5-oxopyrrolidine-3-carboxylate (B302)

[Formula 457]

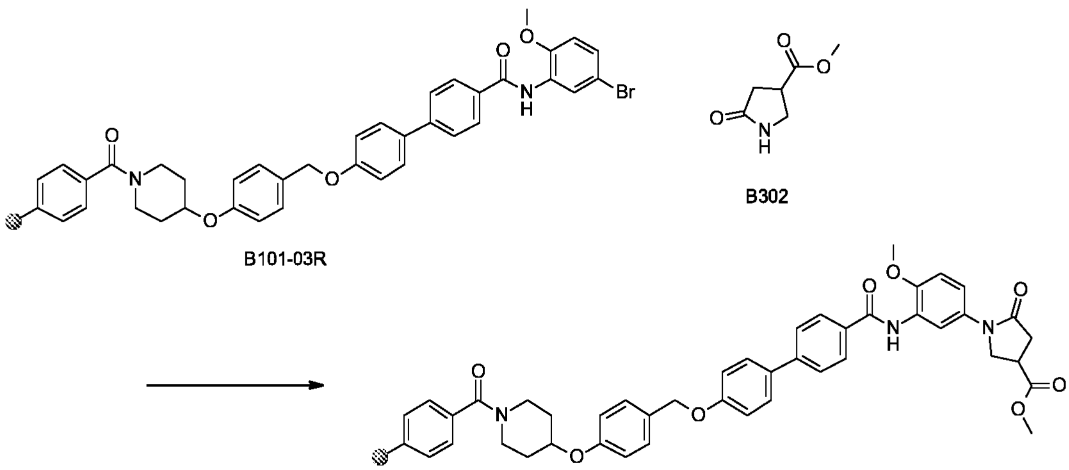

B302

B101-03R

B204-03R

Under a nitrogen atmosphere, solid-phase supported ArBr (B101-03R) (loading rate: 0.194 mmol/g, 20 mg, 0.0039 mmol) and THF (0.4 mL) were added to a 0.6 mL glass vial and shaken at room temperature for 1 hour. Methyl 5-oxopyrrolidine-3-carboxylate (B302) (5.6 mg, 0.039 mmol), tBuBrettphos Pd G4 (3.37 mg, 0.0039 mmol), and MTBD (8.4 μL, 0.058 mmol) were added thereto, and the mixture was shaken at 60°C for 2.5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 76% of compound B204 of interest was observed.

[Formula 458]

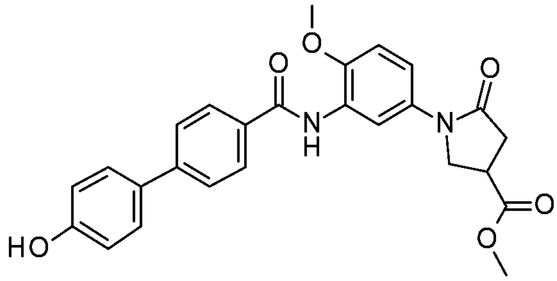

Compound B204

LRMS: m/z 461[M+H]$^+$

Retention time: 0.943 min (analysis conditions SMD-FA05-1, 299 nm)

From these results, it was confirmed that the coupling reaction of ArBr with amide can be carried out using MTBD as a base and tBuBrettphos Pd G4 as a Pd catalyst.

Example 2-13-4: Solid-phase experiment of coupling reaction of solid-phase supported Ar-Br with sulfonamide using Pd reagent

Coupling reaction with sulfonamide was carried out with reference to a non patent literature (Nature, 2018, 557, 228-232) by selecting an organic base P2Et as a base and selecting tBuBrettphos Pd G4 as a Pd catalyst.

Example 2-13-4-1: Coupling reaction of solid-phase supported ArBr (C009-03R) with 2-(methoxycarbonyl)benzylsulfonamide (B303)

[Formula 459]

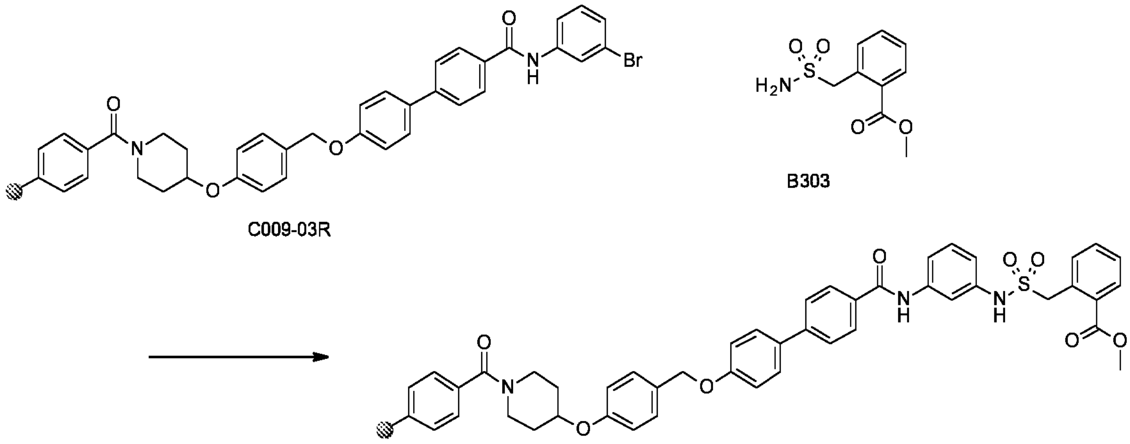

C009-03R

B303

B205-03R

Under a nitrogen atmosphere, solid-phase supported ArBr (C009-03R) (loading rate: 0.195 mmol/g, 20 mg, 0.0039 mmol) and THF (0.4 mL) were added to a 0.6 mL glass vial and shaken at room temperature for 1 hour. 2-(Methoxycarbonyl)benzylsulfonamide (B303) (8.9 mg, 0.039 mmol), tBuBrettphos Pd G4 (3.39 mg, 0.0039 mmol), and P2Et (19.5 μL, 0.059 mmol) were added thereto, and the mixture was shaken at 60°C for 3 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 93% of compound B205 of interest was observed.

[Formula 460]

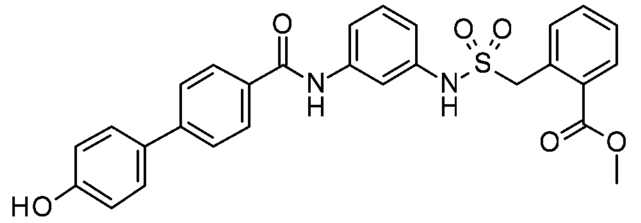

Compound B205

LRMS: m/z 517[M+H]+

Retention time: 1.060 min (analysis conditions SMD-FA05-1, 299 nm)

Example 2-13-4-2: Coupling reaction of solid-phase supported ArBr (C009-03R) with 3,3,3-trifluoropropane-1-sulfonamide (B304)

[Formula 461]

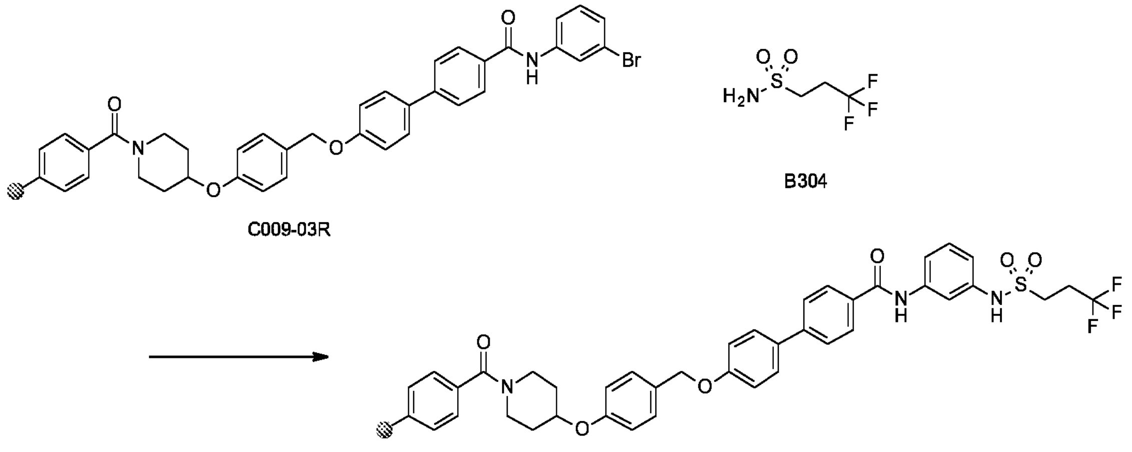

C009-03R

B304

B206-03R

Under a nitrogen atmosphere, solid-phase supported ArBr (C009-03R) (loading rate: 0.195 mmol/g, 20 mg, 0.0039 mmol) and THF (0.4 mL) were added to a 0.6 mL glass vial and shaken at room temperature for 1 hour. 3,3,3-Trifluoropropane-1-sulfonamide (B304) (6.9 mg, 0.039 mmol), tBuBrettphos Pd G4 (3.39 mg, 0.0039 mmol), and P2Et (19.5 μL, 0.059 mmol) were added thereto, and the mixture was shaken at 60°C for 3 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 87% of compound B206 of interest was observed.

[Formula 462]

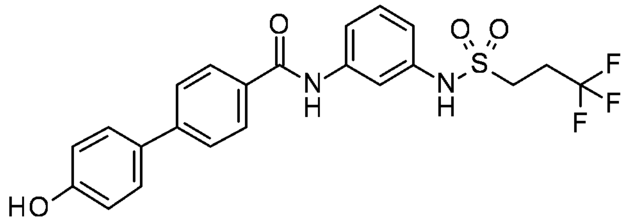

Compound B206

LRMS: m/z 465[M+H]+

Retention time: 1.040 min (analysis conditions SMD-FA05-1, 299 nm)

Example 2-13-4-3: Coupling reaction of solid-phase supported ArBr (B102-03R) with methyl 3-(aminosulfonyl)thiophene-2-carboxylate (B305)

[Formula 463]

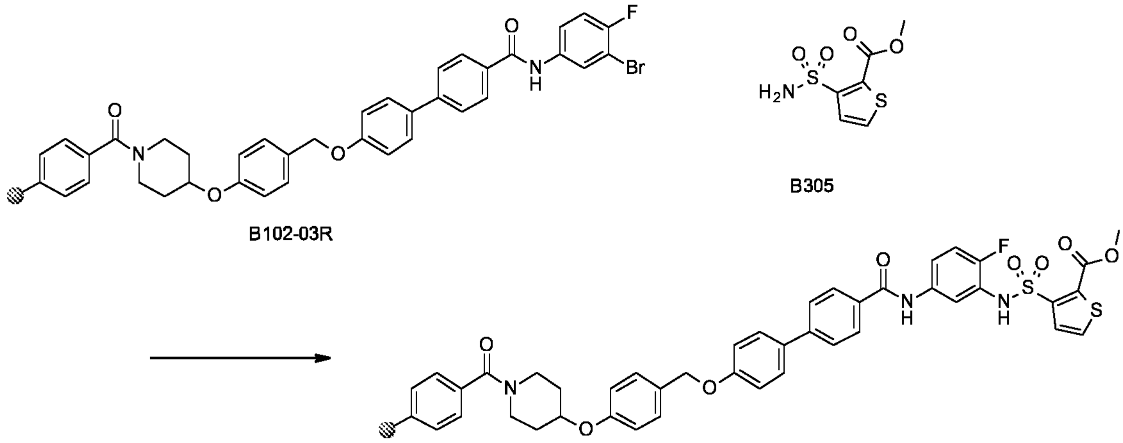

B102-03R

B305

B207-03R

Under a nitrogen atmosphere, solid-phase supported ArBr (B102-03R) (loading rate: 0.194 mmol/g, 20 mg, 0.0039 mmol) and THF (0.4 mL) were added to a 0.6 mL glass vial and shaken at room temperature for 1 hour. Methyl 3-(aminosulfonyl)thiophene-2- carboxylate (B305) (8.6 mg, 0.039 mmol), tBuBrettphos Pd G4 (3.37 mg, 0.0039 mmol), and P2Et (19.4 μL, 0.058 mmol) were added thereto, and the mixture was shaken at 60°C for 15.5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 88% of compound B207 of interest was observed.

[Formula 464]

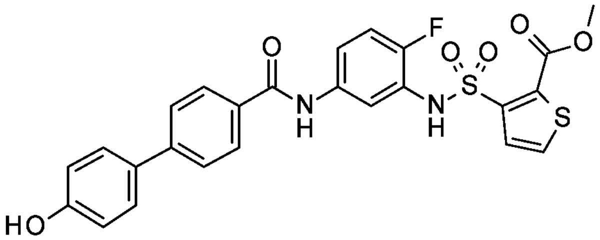

Compound B207

LRMS: m/z 527[M+H]+

Retention time: 1.091 min (analysis conditions SMD-FA05-1, 299 nm)

Example 2-13-4-4: Coupling reaction of solid-phase supported ArBr (B102-03R) with methanesulfonamide (B306)

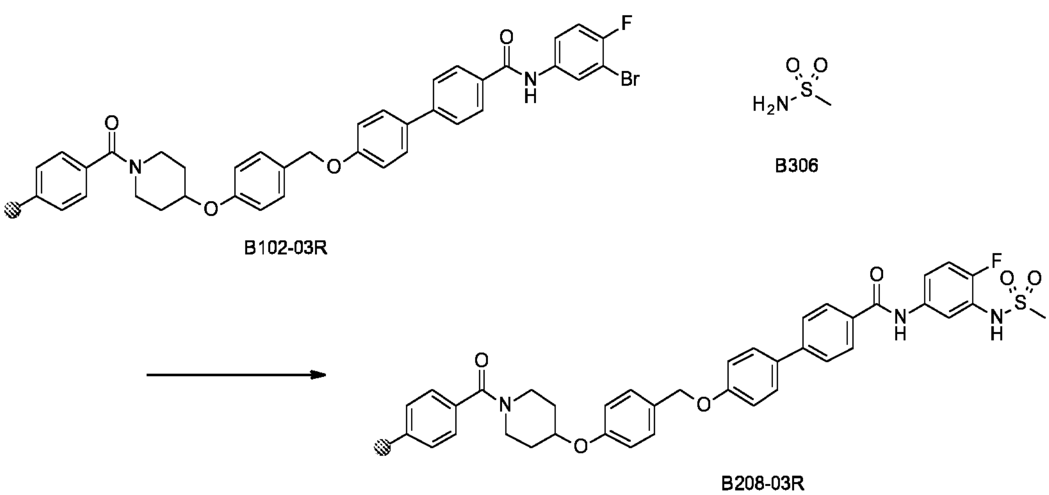

Under a nitrogen atmosphere, solid-phase supported ArBr (B102-03R) (loading rate: 0.194 mmol/g, 20 mg, 0.0039 mmol) and THF (0.4 mL) were added to a 0.6 mL glass vial and shaken at room temperature for 1 hour. Methanesulfonamide (B306) (3.7 mg, 0.039 mmol), tBuBrettphos Pd G4 (3.37 mg, 0.0039 mmol), and P2Et (19.4 μL, 0.058 mmol) were added thereto, and the mixture was shaken at 60°C for 2.5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 86% of compound B208 of interest was observed.

[Formula 465]

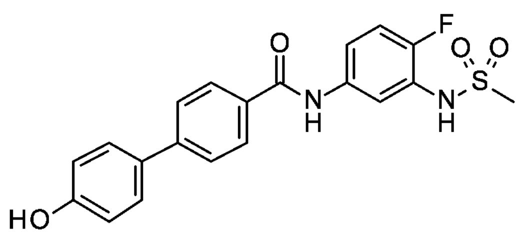

Compound B208

LRMS: m/z 401[M+H]+

Retention time: 0.884 min (analysis conditions SMD-FA05-1, 299 nm)

From these results, it was confirmed that the coupling reaction of ArBr with sulfonamide can be carried out using P2Et as a base and tBuBrettphos Pd G4 as a Pd catalyst.

Example 2-14: Solid-phase experiment to confirm scope of substrate application to C-O bond formation reaction as typical example of identifying substrate scope that can be applied to library synthesis

Example 2-14-1: Solid-phase experiment on nucleophilic substitution reaction of ROH with alkyl halide

Hereinafter, exemplary nucleophilic substitution reaction of solid-phase alcohol with alkyl halide will be illustrated. The substrate is not limited to those given below in actual application to library synthesis.

The nucleophilic substitution reaction of solid-phase supported alcohol with alkyl halide was carried out in THF by selecting KOtBu as a base. More specifically, the nucleophilic substitution reaction (synthesis of H109-03R) of solid-phase supported alcohol (H005-03R) with bromomethylbenzene (H207) using KOtBu as a base will be illustrated.

[Formula 466]

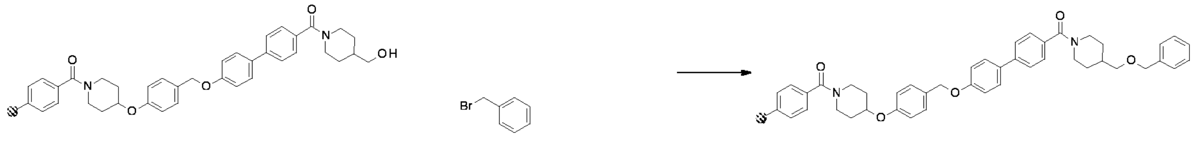

H005-03R H207 H109-03R

Under a nitrogen atmosphere, solid-phase supported alcohol (H004-03R) (loading rate: 0.197 mmol/g, 20 mg, 0.0039 mmol), KOtBu (11.8 μL, 0.012 mmol, 1 M in THF), and THF (0.4 mL) were added to a 0.6 mL glass vial and shaken at room temperature for 1 hour. Under a nitrogen atmosphere, bromomethylbenzene (H207) (4.7 μL, 0.039 mmol) was added thereto, and the mixture was shaken at room temperature for 1 hour.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.1 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 92% of compound H109 of interest was observed.

[Formula 467]

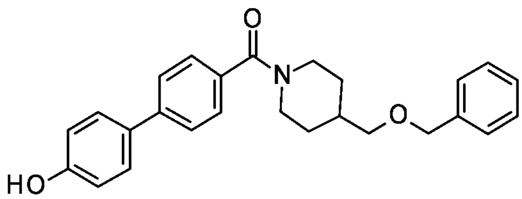

Compound H109

LRMS: m/z 402[M+H]+

Retention time: 1.155 min (analysis conditions SMD-FA05-1, 299 nm)

Example 2-14-2: Synthesis of C601-03R by coupling of solid-phase supported ArBr (C010-03R) and o-cresol (C701)

Hereinafter, exemplary C-O coupling reaction of solid-phase supported ArBr with arenol will be presented. The substrate is not limited to those given below in actual application to library synthesis.

[Formula 468]

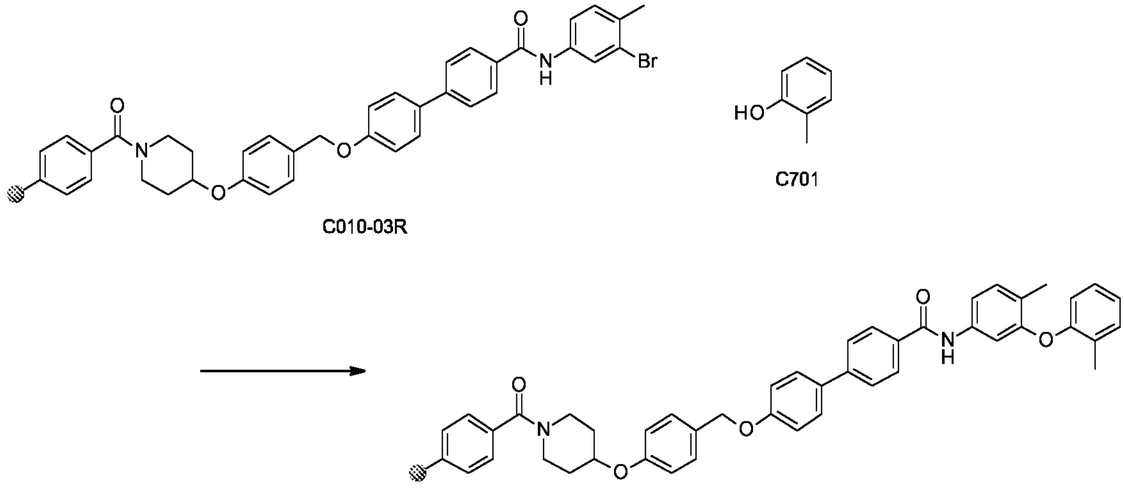

C010-03R

C701

C601-03R

Under a nitrogen atmosphere, compound C010-03R (0.194 mmol/g, 20 mg) and toluene (0.4 mL) were added to a 0.6 mL glass vial and shaken at room temperature for 1 hour. $Pd_2(dba)_3$-$CHCl_3$ (0.4 mg, 0.00039 mmol), AdBippyPhos (1.0 mg, 0.0015 mmol), o- cresol (C701) (8.0 $\mu$L, 0.078 mmol), and $K_3PO_4$ (24.7 mg, 0.116 mmol) were added thereto, and the mixture was shaken at 80°C for 2 hours.

12 $\mu$L of the suspension of the reaction solution and the resin was transferred onto a tip with a filter and washed three times with NMP (0.1 mL), three times with MeOH (0.1 mL), and three times with DCM (0.1 mL). The resultant was dipped in a 10% solution of TFA in DCM containing pentamethylbenzene (0.1 M, 0.05 mL) for 2 minutes, filtered, and washed with NMP (0.05 mL). Combined filtrates were diluted with MeCN (0.25 mL), and the solution was subjected to LC-MS analysis, so that 94.6% of compound C601 of interest was observed. However, the compound was cleaved and analyzed at a wavelength of 299 nm ($\pm$4 nm).

[Formula 469]

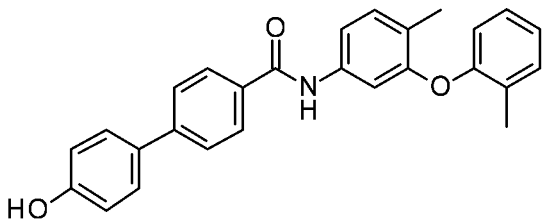

Compound C601

LRMS: m/z 410$[M+H]^+$

Retention time: 1.352 min (analysis conditions SMD-FA05-1)

From these results, C-O coupling reaction with arenol was found applicable to solid- phase ArBr.

Example 2-14-3: Synthesis of C602-03R by coupling of solid-phase supported ArOH (B105-03R) and ethyl 2-bromothiazole-4-carboxylate (C802)

Hereinafter, exemplary C-O coupling reaction of solid-phase supported ArOH with aryl bromide will be presented. The substrate is not limited to those given below in actual application to library synthesis.

[Formula 470]

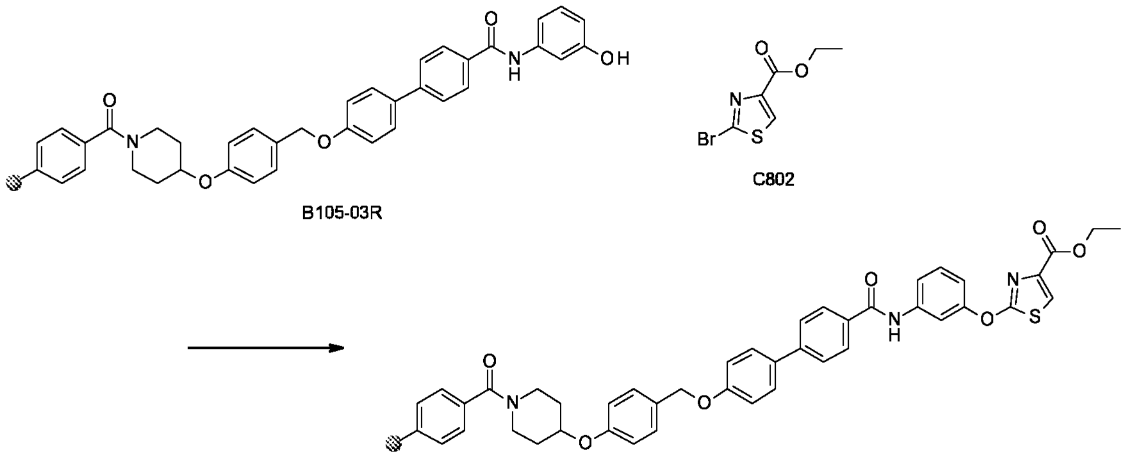

B105-03R

C802

C602-03R

Under a nitrogen atmosphere, compound B105-03R (0.197 mmol/g, 20 mg) and DME (0.4 mL) were added to a 0.6 mL glass vial. Ethyl 2-bromothiazole-4-carboxylate (C802) (9.3 mg, 0.039 mmol) was added thereto, and the mixture was shaken at room temperature for 1 hour. GPhos (4.2 mg, 0.0079 mmol), $Pd_2(dba)_3$-$CHCl_3$ (2.0 mg, 0.0020 mmol), potassium trifluoromethanesulfonate (11.1 mg, 0.059 mmol), and BTMG (11.8 μL, 0.059 mmol) were added thereto, and the mixture was shaken at 80°C for 24 hours.

12 μL of the suspension of the reaction solution and the resin was transferred onto a tip with a filter, washed three times with DMF (0.1 mL), three times with MeOH (0.1 mL), and three times with DCM (0.1 mL). The resultant was dipped in a 10% solution of TFA in DCM containing pentamethylbenzene (0.1 M, 0.05 mL) for 2 minutes, filtered, and washed with DMF (0.05 mL). Combined filtrates were diluted with MeCN (0.25 mL), and the solution was subjected to LC-MS analysis, so that 92.1% of compound C602 of interest was observed. However, the compound was cleaved and analyzed at a wavelength of 299 nm (±4 nm).

[Formula 471]

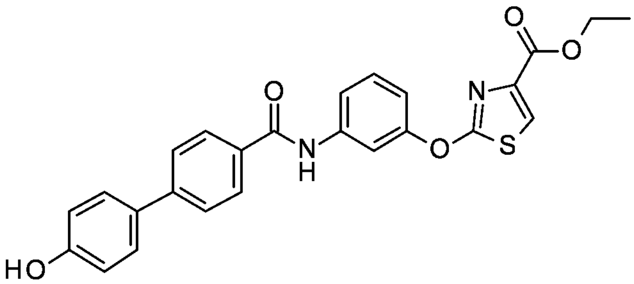

Compound C602

LRMS: m/z 461[M+H]+

Retention time: 1.140 min (analysis conditions SMD-FA05-1)

From these results, C-O coupling reaction with aryl bromide was found applicable to solid-phase ArOH.

Example 2-15-1: Synthesis of C603-03R by coupling of solid-phase supported ArBr (C009-03R) and 1-dodecanethiol (C702)

Hereinafter, exemplary C-S coupling reaction of solid-phase supported ArBr with thiol will be presented. The substrate is not limited to those given below in actual application to library synthesis.

[Formula 472]

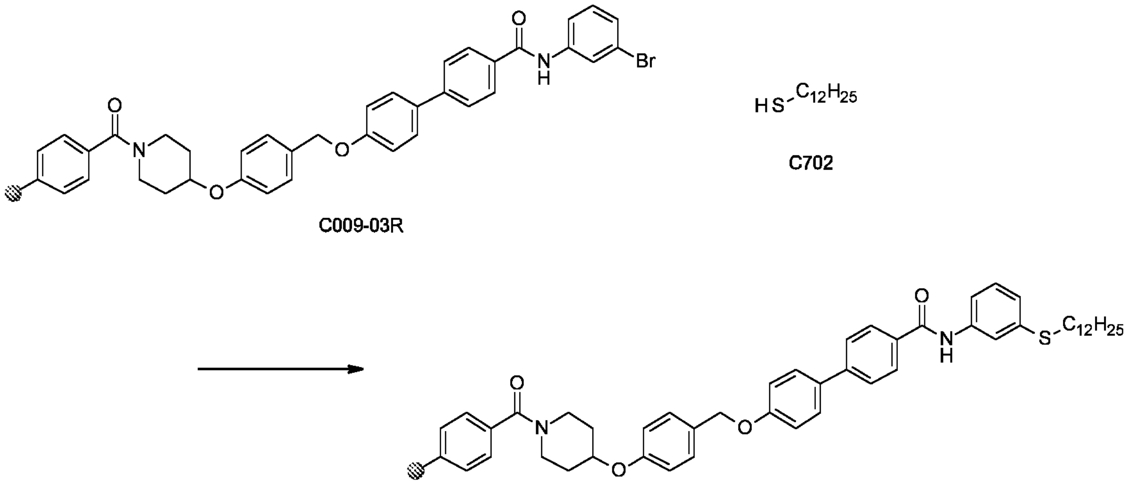

C009-03R

C702

C603-03R

Under a nitrogen atmosphere, compound C009-03R (0.195 mmol/g, 20 mg), DME (0.4 mL), and 1-dodecanethiol (C702) (9.3 µL, 0.039 mmol) were added to a 0.6 mL glass vial and shaken at room temperature for 1 hour. Josiphos Pd G3 (3.6 mg, 0.0039 mmol) and a solution of NaOtBu in THF (2 M, 29.3 µL, 0.059 mmol) were added thereto, and the mixture was shaken at 90°C for 30 minutes.

12 µL of the suspension of the reaction solution and the resin was transferred onto a tip with a filter and washed three times with DMF (0.1 mL), three times with MeOH (0.1 mL), and three times with DCM (0.1 mL). The resultant was dipped in a 10% solution of TFA in DCM containing pentamethylbenzene (0.02 M, 0.05 mL) for 2 minutes, filtered, and washed with DMF (0.05 mL). Combined filtrates were diluted with MeCN (0.25 mL), and the solution was subjected to LC-MS analysis, so that 96.6% of compound C603 of interest was observed. However, the compound was cleaved and analyzed at a wavelength of 299 nm (±4 nm).

[Formula 473]

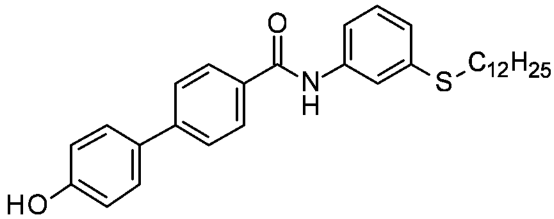

Compound C603

LRMS: m/z 490[M+H]$^+$

Retention time: 1.829 min (analysis conditions SMD-FA05-1)

From these results, C-S coupling reaction with thiol was found applicable to solid- phase ArBr.

Example 2-16-1: Synthesis of C206-03R by coupling of solid-phase supported thioester (A120-03R) and phenylboronic acid (C308)

Hereinafter, exemplary ketone synthesis reaction by coupling of solid-phase thioester obtained through the condensation reaction of solid-phase supported carboxylic acid with thiol, and boronic acid will be presented. The substrate is not limited to those given below in actual application to library synthesis.

[Formula 474]

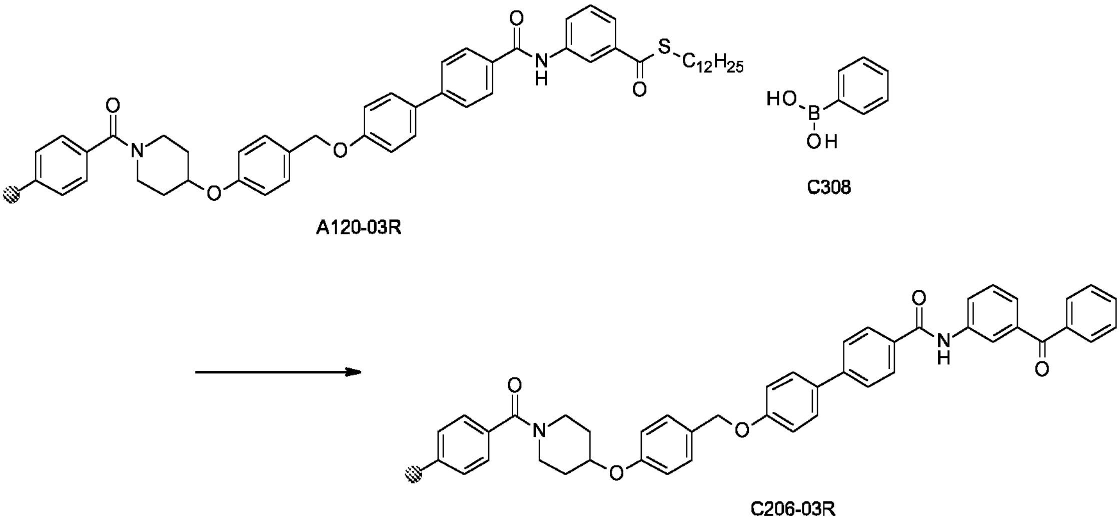

A120-03R

C308

C206-03R

Under a nitrogen atmosphere, compound A120-03R (0.190 mmol/g, 20 mg), phenylboronic acid (C308) (4.6 mg, 0.038 mmol), and THF (0.4 mL) were added to a 0.6 mL glass vial and shaken at room temperature for 1 hour. Triethyl phosphite (1.3 μL, 0.0076 mmol), $Pd_2(dba)_3$-$CHCl_3$ (2.0 mg, 0.0019 mmol), and copper(I) thiophene-2- carboxylate (10.9 mg, 0.057 mmol) were added thereto, and the mixture was shaken at 60°C for 2 hours.

12 μL of the suspension of the reaction solution and the resin was transferred onto a tip with a filter and washed three times with DMF (0.1 mL), three times with MeOH (0.1 mL), and three times with DCM (0.1 mL). The resultant was dipped in a 10% solution of TFA in DCM containing pentamethylbenzene (0.02 M, 0.05 mL) for 2 minutes, filtered, and washed with DMF (0.05 mL). Combined filtrates were diluted with MeCN (0.25 mL), and the solution was subjected to LC-MS analysis, so that 99.4% of compound C206 of interest was observed. However, the compound was cleaved and analyzed at a wavelength of 299 nm (±4 nm).

[Formula 475]

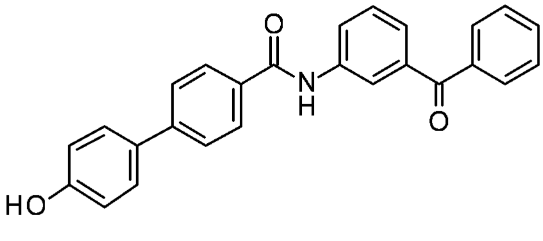

Compound C206

LRMS: m/z 394$[M+H]^+$

Retention time: 1.149 min (analysis conditions SMD-FA05-1)

These results demonstrated that ketone can be synthesized through coupling reaction with boronic acid via solid-phase thioester from solid-phase supported carboxylic acid.

Example 2-17: Experiment to confirm scope of substrate application to ester bond formation reaction

Hereinafter, exemplary conditions for enhancing reproducibility by selecting suitable reaction conditions for ester bond formation reaction ($R1CO_2H$ + R2OH) will be illustrated. The scope given below is a mere illustration of an exemplary identification method, and the present invention is not limited by the scope given below in actual application to library synthesis.

Example 2-17-1: Esterification reaction of solid-phase supported $ArCO_2H$ (B106- 03R) with 2,2,2-trifluoroethanol (B316) using WSC·HCl/DMAP as condensing agent

The esterification reaction of solid-phase supported carboxylic acid with an alcohol was carried out with reference

to a non patent literature (RSC Advances, 2018, 8, 25168) by selecting WSC·HCl as a condensing agent and using DMAP as a base.

[Formula 476]

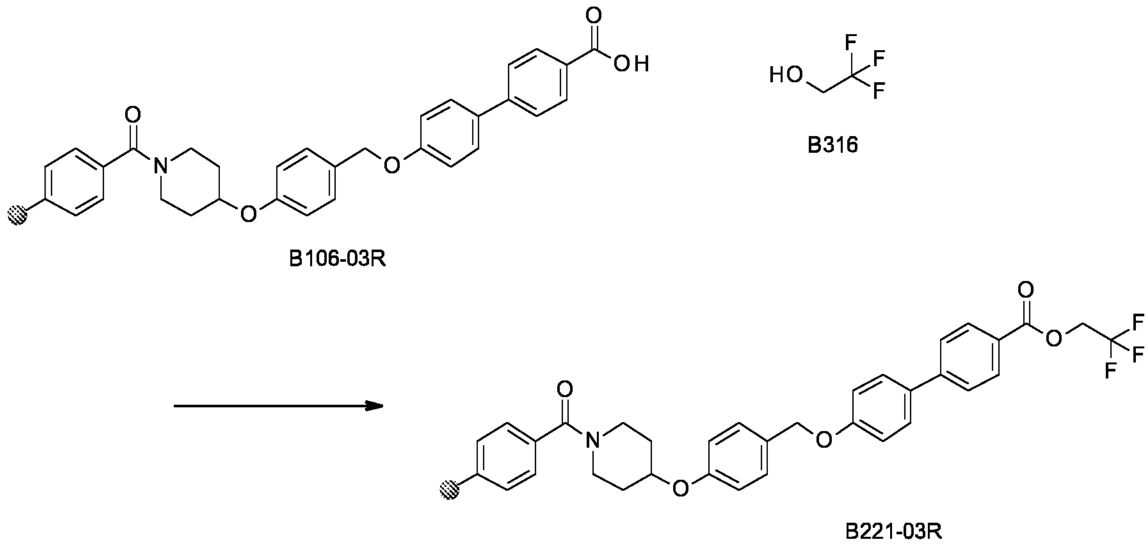

B106-03R

B316

B221-03R

Under a nitrogen atmosphere, solid-phase supported Ar$CO_2$H (B106-03R) (loading rate: 0.201 mmol/g, 20 mg, 0.0040 mmol) and THF (0.4 mL) were added to a 0.6 mL glass vial and shaken at room temperature for 1 hour. 2,2,2-Trifluoroethanol (B316) (5.8 μL, 0.080 mmol), DMAP (4.9 mg, 0.040 mmol), and WSC·HCl (7.7 mg, 0.040 mmol) were added thereto, and the mixture was shaken at 25°C for 18 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 95% of compound B221 of interest was observed.

[Formula 477]

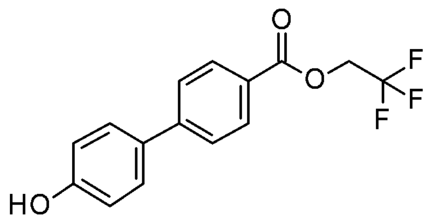

Compound B221

Maximum wavelength: 300.4 nm

Retention time: 1.144 min (analysis conditions SMD-FA05-1, 299 nm)

Example 2-17-2: Esterification reaction of solid-phase supported ArCOzH (B106- 03R) with butanol (B317) using WSC·HCl/DMAP as condensing agent

[Formula 478]

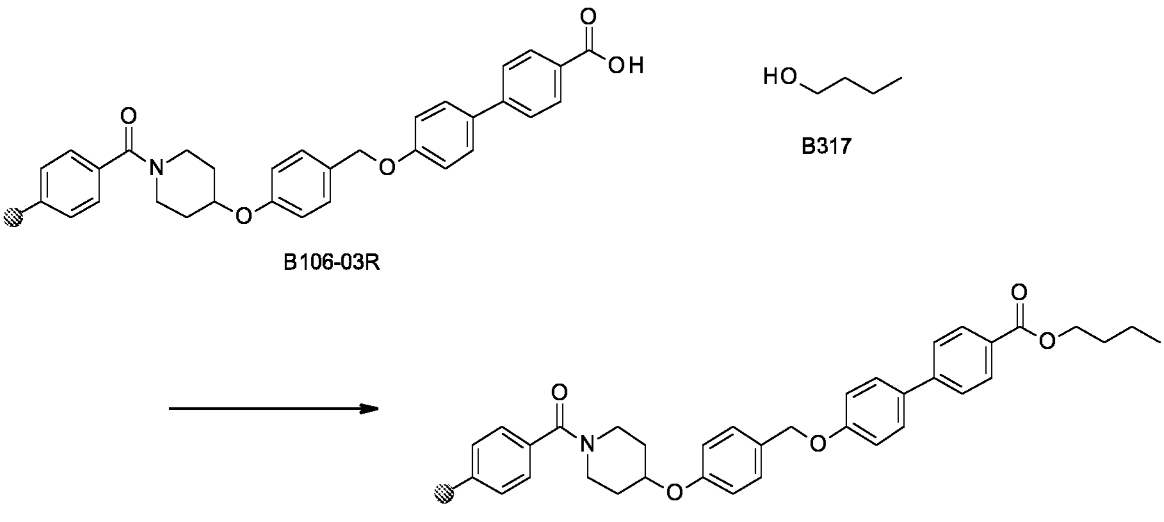

B317

B106-03R

B222-03R

Under a nitrogen atmosphere, solid-phase supported ArCOzH (B106-03R) (loading rate: 0.201 mmol/g, 20 mg, 0.0040 mmol) and DCM (0.4 mL) were added to a 0.6 mL glass vial and shaken at room temperature for 1 hour. Butanol (B317) (7.4 μL, 0.080 mmol), DMAP (4.9 mg, 0.040 mmol), and WSC·HCl (7.7 mg, 0.040 mmol) were added thereto, and the mixture was shaken at 25°C for 16.5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 98% of compound B222 of interest was observed.

[Formula 479]

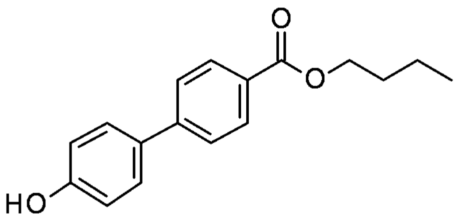

Compound B222

LRMS: m/z 271[M+H]$^+$

Retention time: 1.256 min (analysis conditions SMD-FA05-1, 299 nm)

Example 2-17-3: Esterification reaction of solid-phase supported ArCOzH (B106- 03R) with cyclohexanol (B318) using WSC·HCl/DMAP as condensing agent

[Formula 480]

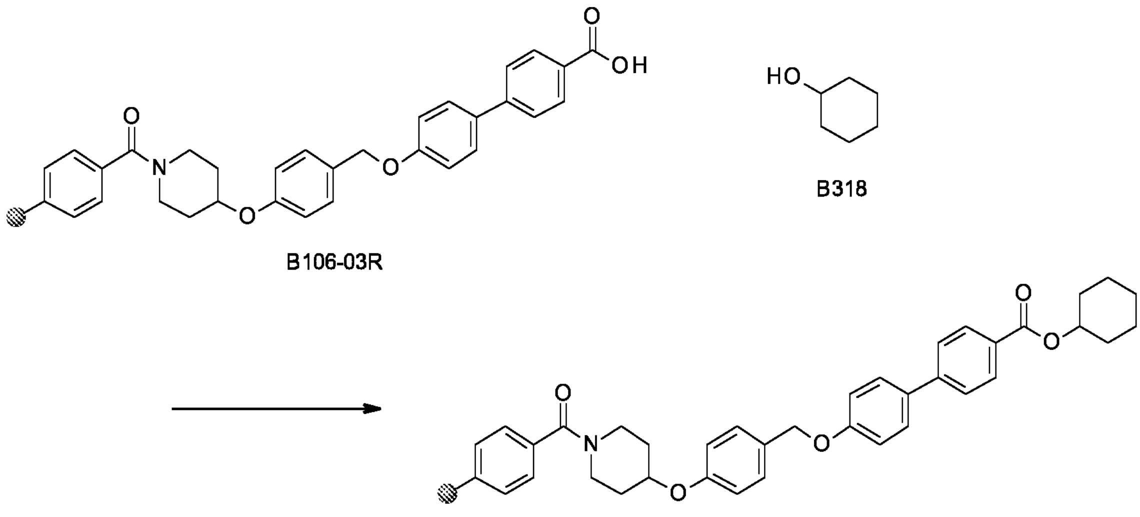

B106-03R

B318

B223-03R

Under a nitrogen atmosphere, solid-phase supported ArCOzH (B106-03R) (loading rate: 0.201 mmol/g, 20 mg, 0.0040 mmol) and DCM (0.4 mL) were added to a 0.6 mL glass vial and shaken at room temperature for 1 hour. Cyclohexanol (B318) (8.5 μL, 0.080 mmol), DMAP (4.9 mg, 0.040 mmol), and WSC·HCl (7.7 mg, 0.040 mmol) were added thereto, and the mixture was shaken at 25°C for 2.5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 97% of compound B223 of interest was observed.

[Formula 481]

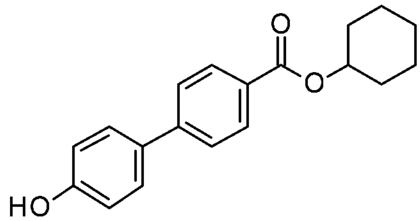

Compound B223

Maximum wavelength: 294.4 nm

Retention time: 1.344 min (analysis conditions SMD-FA05-1, 299 nm)

From these results, it was confirmed that the esterification reaction of solid-phase supported ArCO2H with primary and secondary alcohols can be carried out under condensation conditions using WSC·HCl as a condensing agent and DMAP as a base.

Example 2-17-4: Esterification reaction of solid-phase supported ArCOzH (B106- 03R) with butanol (B317) under Mitsunobu reaction conditions using CMMP

The esterification reaction of solid-phase supported ArCOzH (B106-03R) with an alcohol was carried out with reference to a non patent literature (Tetrahedron Lett. 1995, 36, 2529) and the solid phase etherification reaction (Examples 2-1-6-4 to -13) using CMMP as a Mitsunobu reagent.

[Formula 482]

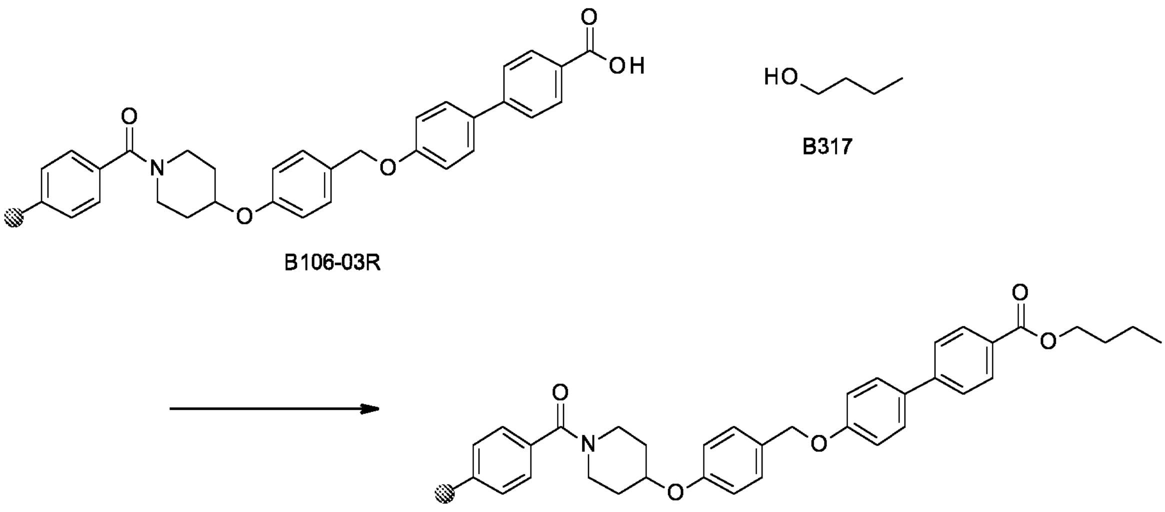

B106-03R

B317

B222-03R

Under a nitrogen atmosphere, solid-phase supported $ArCO_2H$ (B106-03R) (loading rate: 0.201 mmol/g, 20 mg, 0.0040 mmol) and THF (0.4 mL) were added to a 0.6 mL glass vial and shaken at room temperature for 1 hour. Butanol (B317) (7.4 μL, 0.080 mmol) and CMMP (0.5 M solution in THF, 80 μL, 0.040 mmol) were added thereto, and the mixture was shaken at 25°C for 18 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 98% of compound B222 of interest was observed.

Example 2-17-5: Esterification reaction of solid-phase supported $ArCO_2H$ (B106- 03R) with cyclohexanol (B318) under Mitsunobu reaction conditions using CMMP

[Formula 483]

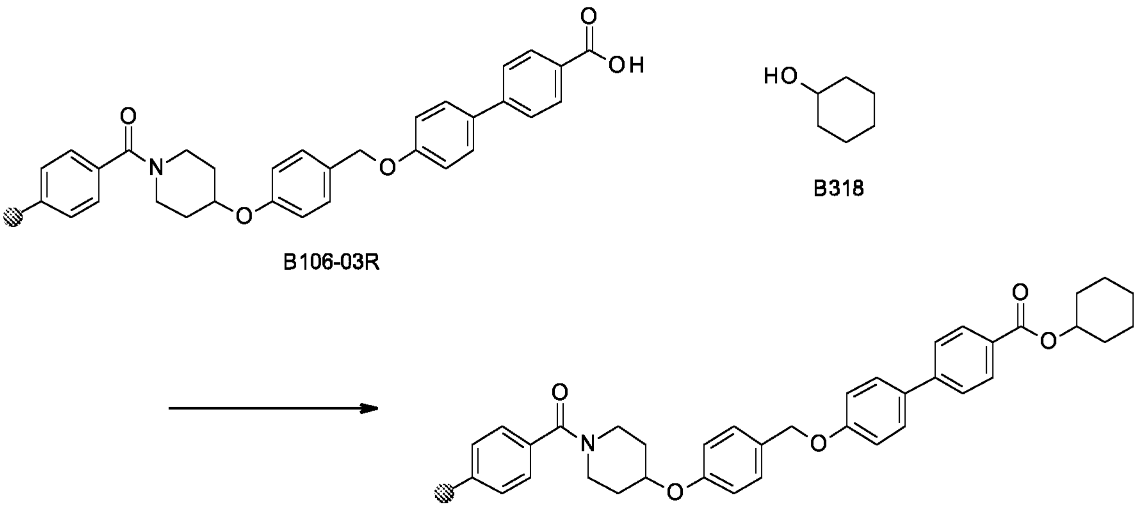

B106-03R

B318

B223-03R

Under a nitrogen atmosphere, solid-phase supported $ArCO_2H$ (B106-03R) (loading rate: 0.201 mmol/g, 20 mg, 0.0040 mmol) and THF (0.4 mL) were added to a 0.6 mL glass vial and shaken at room temperature for 1 hour. Cyclohexanol (B318) (8.5 μL, 0.080 mmol) and CMMP (0.5 M a solution in THF, 80 μL, 0.040 mmol) were added thereto, and the mixture was shaken at 60°C for 6 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 95% of compound B223 of interest was observed.

From these results, it was confirmed that the esterification reaction of solid-phase supported $ArCO_2H$ with primary and secondary alcohols can be carried out under esterification conditions using CMMP as a Mitsunobu reagent.

Example 2-17-6: Formation of solid-phase supported arenol ester (C604-03R) by condensation of solid-phase supported carboxylic acid (A119-03R) and p-cresol (C703)

Hereinafter, exemplary ester synthesis reaction through the condensation reaction of solid-phase supported carboxylic acid with arenol will be presented. The substrate is not limited to those given below in actual application to library synthesis.

[Formula 484]

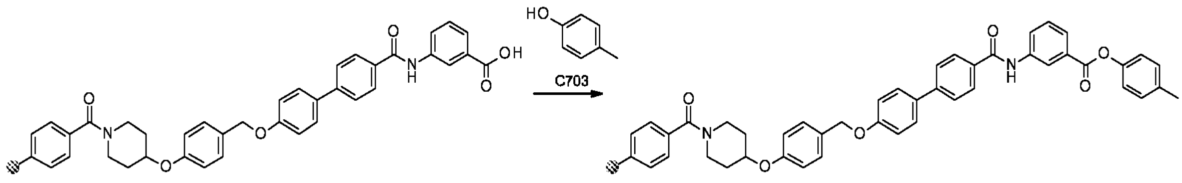


Under a nitrogen atmosphere, compound A119-03R (0.197 mmol/g, 20 mg), DCM (0.4 mL), and p-cresol (C703) (4.3 mg, 0.039 mmol) were added to a 0.6 mL glass vial. NMI (6.3 μL, 0.079 mmol) and PipClU (14.2 mg, 0.039 mmol) were added thereto, and the mixture was shaken at 25°C for 1 hour.

12 μL of the suspension of the reaction solution and the resin was transferred onto a tip with a filter and washed three times with DMF (0.1 mL), three times with MeOH (0.1 mL), and three times with DCM (0.1 mL). The resultant was dipped in a 10% solution of TFA in DCM containing pentamethylbenzene (0.02 M, 0.05 mL) for 1 minute, filtered, and washed with DMF (0.05 mL). Combined filtrates were diluted with MeCN (0.25 mL), and the solution was subjected to LC-MS analysis, so that 98.4% of compound C604 of interest was observed. However, the compound was cleaved and analyzed at a wavelength of 299 nm (±4 nm).

[Formula 485]

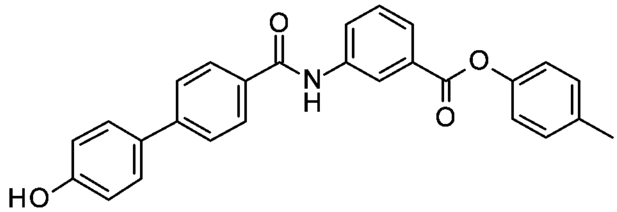

Compound C604

LRMS: m/z 424[M+H]+.

Retention time: 1.283 min (analysis conditions SMD-FA05-1).

These results demonstrated that the condensation reaction of solid-phase supported carboxylic acid with arenol can be carried out.

Example 2-18: Experiment to confirm scope of substrate application to nitrogen- containing 5-membered ring HetAr formation

Hereinafter, exemplary conditions will be illustrated under which nitrogen- containing 5-membered ring HetAr is synthesized as a linker by selecting suitable synthesis reaction conditions for nitrogen-containing 5-membered ring HetAr formation. The scope given below is a mere illustration of an exemplary identification method, and the present invention is not limited by the scope given below in actual application to library synthesis.

Example 2-18-1: Huisgen cyclization reaction of solid-phase supported acetylene compound ArCCH (B107-03R) with ethyl azidoacetate (B319)

The Huisgen cyclization reaction of solid-phase supported acetylene compound ArCCH (B107-03R) with ethyl azidoacetate (B319) was carried out with reference to a non patent literature (J. Org. Chem. 2011, 76, 6832) by selecting CuI, DIPEA, or AcOH as a reaction accelerator.

[Formula 486]

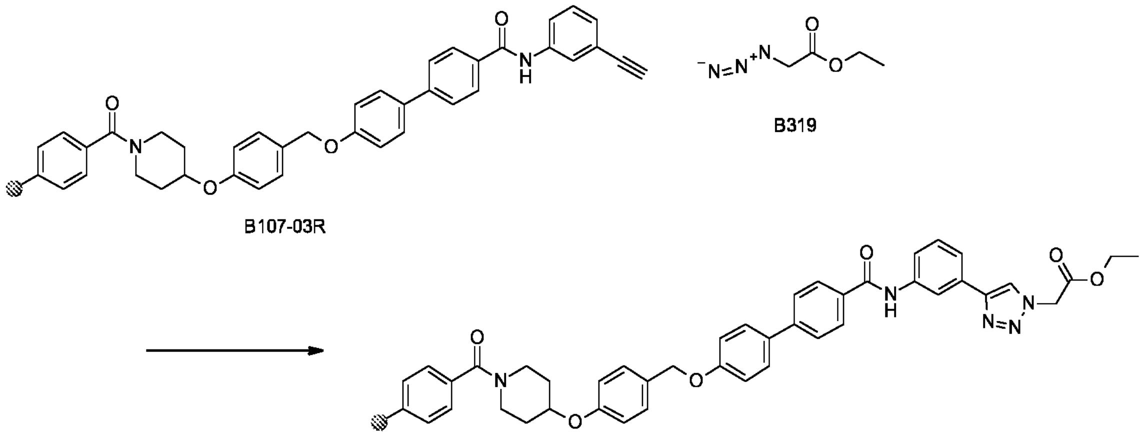

B107-03R

B319

B224-03R

Under a nitrogen atmosphere, solid-phase supported phenylacetylene PhCCH (B107-03R) (loading rate: 0.197 mmol/g, 20 mg, 0.0039 mmol) and DCM (0.4 mL) were added to a 0.6 mL glass vial and shaken at room temperature for 1 hour. Ethyl azidoacetate (B319) (4.5 μL, 0.039 mmol), CuI (3.8 mg, 0.020 mmol), DIPEA (6.9 μL, 0.039 mmol), and AcOH (2.3 μL, 0.039 mmol) were added thereto, and the mixture was shaken at 25°C for 2.5 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 99% of compound B224 of interest was observed.

[Formula 486-1]

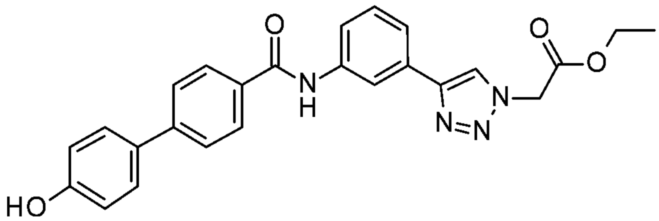

Compound B224

LRMS: m/z 443[M+H]$^+$

Retention time: 0.984 min (analysis conditions SMD-FA05-1, 299 nm)

Example 2-18-2: Huisgen cyclization reaction of solid-phase supported azide compound $ArN_3$ (B108-03R) with 4-ethynyltoluene (B320)

The Huisgen cyclization reaction of solid-phase supported azide compound $ArN_3$ (B108-03R) with 4-ethynyltoluene (B320) can be carried out with reference to a non patent literature (J. Org. Chem. 2011, 76, 6832) by selecting CuI,DIPEA,AcOH as a reaction accelerator.

[Formula 487]

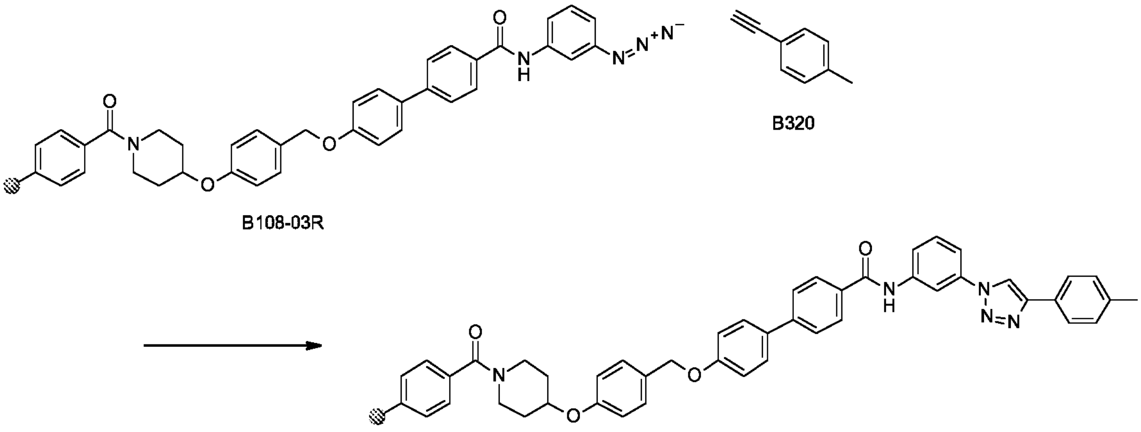

B320

B108-03R

B225-03R

Under a nitrogen atmosphere, solid-phase supported azide compound $ArN_3$ (B108- 03R) (loading rate: 0.196 mmol/g, 20 mg, 0.0039 mmol) and DCE (0.4 mL) were added to a 0.6 mL glass vial and shaken at room temperature for 1 hour. 4-Ethynyltoluene (B320) (5.0 μL, 0.039 mmol), CuI (3.7 mg, 0.020 mmol), DIPEA (6.8 μL, 0.039 mmol), and AcOH (2.2 μL, 0.039 mmol) were added thereto, and the mixture was shaken at 60°C for 22 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 96% of compound B225 of interest was observed.

[Formula 488]

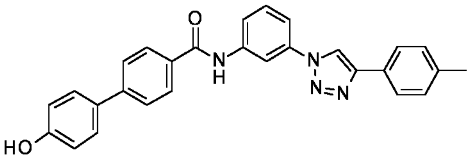

Compound B225

LRMS: m/z 447$[M+H]^+$

Retention time: 1.219 min (analysis conditions SMD-FA05-1, 299 nm)

From these results, it was confirmed that nitrogen-containing 5-membered ring HetAr can be formed as a linker through the reaction of solid-phase supported acetylene with an azide compound or the reaction of a solid-phase supported azide compound with an acetylene compound by carrying out Huisgen cyclization reaction.

Example 2-18-3: Solid-phase experiment of Buchwald-Hartwig C-N bond formation reaction of solid-phase supported Ar-Br with 5-membered ring HetAr

C-N bond formation reaction with ethyl pyrazole-3-carboxylate was carried out with reference to a non patent literature (J. Am. Chem. Soc. 2001, 123, 7727.) by selecting an organic base MTBD (7-methyl-1,5,7-triazabicyclo[4.4.0]dec-5-ene) as a base, selecting CuI as a Cu catalyst, and selecting DMEDA (N,N'-dimethylethylenediamine) as a ligand.

Example 2-18-3-1: Coupling reaction of solid-phase supported ArBr (C009-03R) with ethyl pyrazole-3-carboxylate (B321)

[Formula 489]

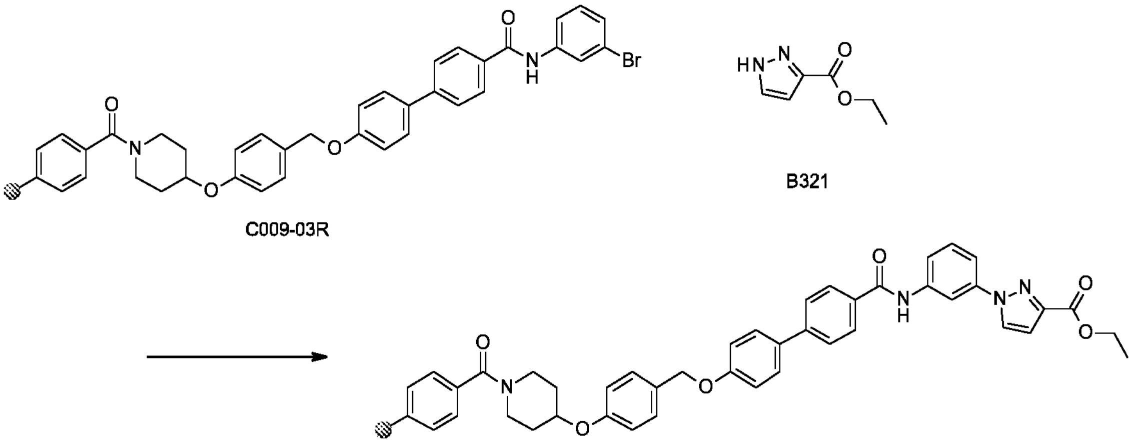

C009-03R

B321

B226-03R

Under a nitrogen atmosphere, solid-phase supported Ar-Br (C009-03R) (loading rate: 0.195 mmol/g, 20 mg, 0.0039 mmol) and DMF (0.4 mL) were added to a 0.6 mL glass vial and shaken at room temperature for 1 hour. Ethyl pyrazole-3-carboxylate (B321) (13.7 mg, 0.098 mmol), CuI (7.4 mg, 0.039 mmol), DMEDA (8.4 μL, 0.078 mmol), and MTBD (14 μL, 0.098 mmol) were added thereto, and the mixture was shaken at 110°C for 12 hours.

The suspension of the reaction solution and the resin was transferred onto a filter, washed three times with DMF (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMF (0.05 mL), and MeCN (0.25 mL) was added to the filtrate to prepare an LC sample. The degree of progression of the reaction was measured by LC-MS analysis. As a result, 77% of compound B226 of interest was observed.

[Formula 490]

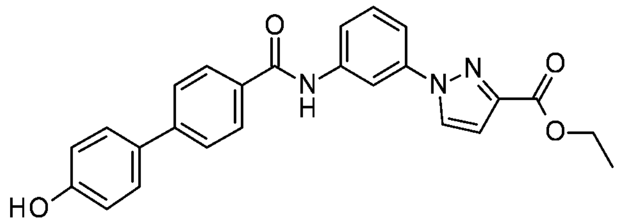

Compound B226

LRMS: m/z 428[M+H]+

Retention time: 1.096 min (analysis conditions SMD-FA05-1, 299 nm)

From these results, it was confirmed that the C-N bond formation reaction of N-H of HeteroAr with solid-phase ArBr can be carried out using CuI.

Example 2-19: Reaction for converting functional group of substrate to another reactive functional group on solid phase

Example 2-19-1: Functional group conversion reaction of solid-phase supported ArBr (C009-03R) to solid-phase supported carboxylic acid (C119-03R)

Hereinafter, exemplary functional group conversion reaction of solid-phase supported ArBr to solid-phase supported carboxylic acid will be presented. The solid-phase supported carboxylic acid is a substrate important for obtaining amide, acylsulfonamide, ketone, and ester, as shown in Examples 2-7, 2-8, 2-16, and 2-17. When the functional group conversion of solid-phase supported ArBr to solid-phase supported carboxylic acid is applicable, amide, acylsulfonamide, ketone, and ester can be synthesized from the solid- phase supported ArBr. The substrate is not limited to those given below in actual application to library synthesis.

[Formula 491]

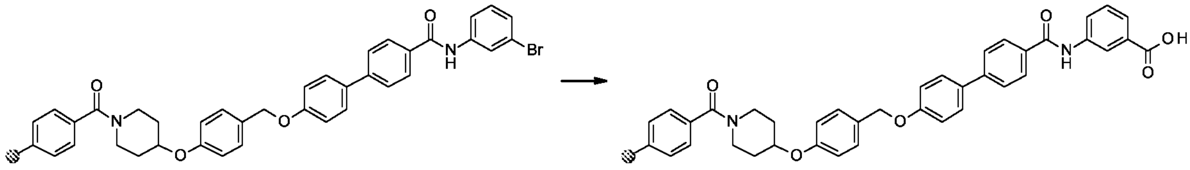

C009-03R A119-03R

Under a nitrogen atmosphere, compound C009-03R (0.195 mmol/g, 20 mg) and DMF (0.4 mL) were added to a 0.6 mL glass vial and shaken at room temperature for 1 hour. Oxalic acid dihydrate (14.8 mg, 0.117 mmol), acetic anhydride (11.0 μL, 0.117 mmol), DIPEA (30.7 μL, 0.176 mmol), and Xantphos Pd G4 (0.75 mg, 0.00078 mmol) were added thereto, and the mixture was shaken at 100°C for 24 hours.

12 μL of the suspension of the reaction solution and the resin was transferred onto a tip with a filter and washed three times with DMF (0.1 mL), three times with MeOH (0.1 mL), and three times with DCM (0.1 mL). The resultant was dipped in a 10% solution of TFA in DCM containing pentamethylbenzene (0.02 M, 0.05 mL) for 2 minutes, filtered, and washed with DMF (0.05 mL). Combined filtrates were diluted with MeCN (0.25 mL), and the solution was subjected to LC-MS analysis, so that 96.1% of compound A119 of interest was observed. However, the compound was cleaved and analyzed at a wavelength of 299 nm (±4 nm).

These results demonstrated that aryl bromide can be converted to carboxylic acid on the solid phase.

Example 2-19-2: Functional group conversion reaction of solid-phase supported thioester (A120-03R) to solid-phase supported boronic acid ester (C901-03R) or boronic acid (C902-03R)

Hereinafter, exemplary conversion reaction of solid-phase thioester obtained through the condensation reaction of solid-phase supported carboxylic acid with thiol to solid-phase supported boronic acid ester or boronic acid will be presented. The solid-phase supported boronic acid ester is an important substrate applicable to Suzuki coupling reaction, as shown in Example 2-2-10. The substrate is not limited to those given below in actual application to library synthesis.

[Formula 492]

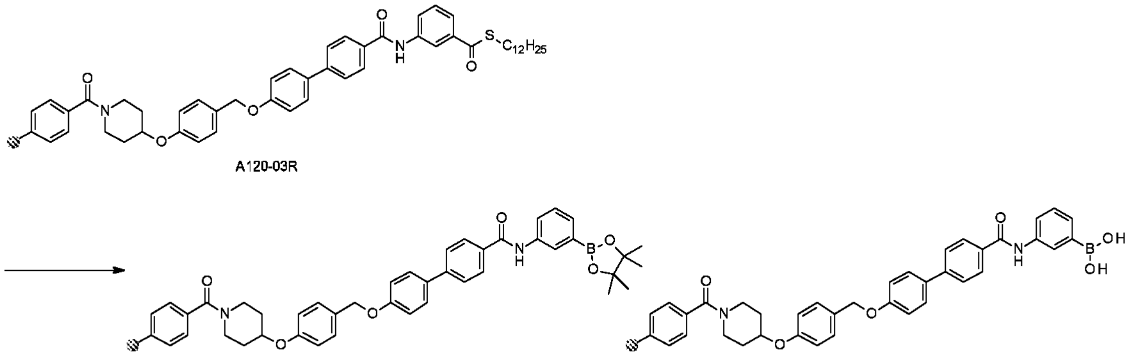

A120-03R C901-03R C902-03R

Under a nitrogen atmosphere, compound A120-03R (0.190 mmol/g, 15 mg) and CPME (0.3 mL) were added to a 0.6 mL glass vial and shaken at 60°C for 1 hour. $(PinB)_2$ (14.5 mg, 0.057 mmol), tributylphosphine (14.1 μL, 0.057 mmol), hydroxy(1,5- cyclooctadiene)rhodium(I) dimer (0.65 mg, 0.0014 mmol), and potassium acetate (0.56 mg, 0.0057 mmol) were added thereto, and the mixture was shaken at 80°C for 24 hours.

12 μL of the suspension of the reaction solution and the resin was transferred onto a tip with a filter and washed

three times with DMF (0.1 mL), three times with MeOH (0.1 mL), and three times with DCM (0.1 mL). The resultant was dipped in a 10% solution of TFA in DCM containing pentamethylbenzene (0.02 M, 0.05 mL) for 1 minute, filtered, and washed with DMF (0.05 mL). Combined filtrates were diluted with MeCN (0.25 mL), and the solution was subjected to LC-MS analysis, so that 77.9% of boronic acid ester C901 and 3.7% of boronic acid C902 of interest were observed. However, the compound was cleaved and analyzed at a wavelength of 299 nm (±4 nm).

[Formula 493]

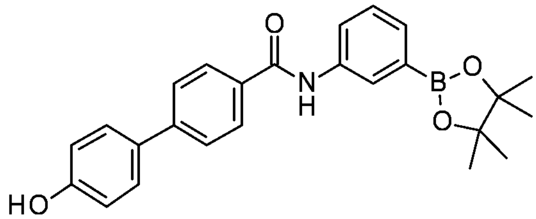

Compound C901

LRMS: m/z 416[M+H]+

Retention time: 1.224 min (analysis conditions SMD-FA05-1)

[Formula 494]

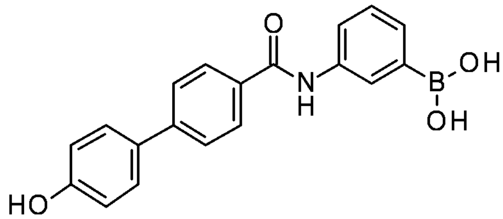

Compound C902

LRMS: m/z 334[M+H]+

Retention time: 0.809 min (analysis conditions SMD-FA05-1)

These results demonstrated that carboxylic acid can be converted through the formation of thioester to boronic acid on the solid phase.

Example 2-19-3: Functional group transformation reaction of solid-phase supported $ArNH_2$ to ArBr through Sandmeyer reaction

One method for expanding the available scope of bond formation reaction using $ArNH_2$ as a starting material includes a method of converting $ArNH_2$ to ArBr and carrying out bond formation reaction. Sandmeyer reaction was selected as a method for converting $ArNH_2$ to ArBr. Sandmeyer bromination reaction to solid-phase $ArNH_2$ was carried out with reference to a non patent literature (Org. Chem. 2017, 19, 2518.) which discloses that reaction is carried out under mild reaction conditions using organic acid, by selecting $NaNO_2$ (sodium nitrite) as a diazotization reagent, $BrCCl_3$ (bromotrichloromethane) as a brominating agent, AcOH as an acid, and THF-$H_2O$ as a reaction solvent.

Example 2-19-3-1: Confirmation of progression of Sandmeyer bromination reaction to solid-phase supported $ArNH_2$ (A118-03R)

[Formula 495]

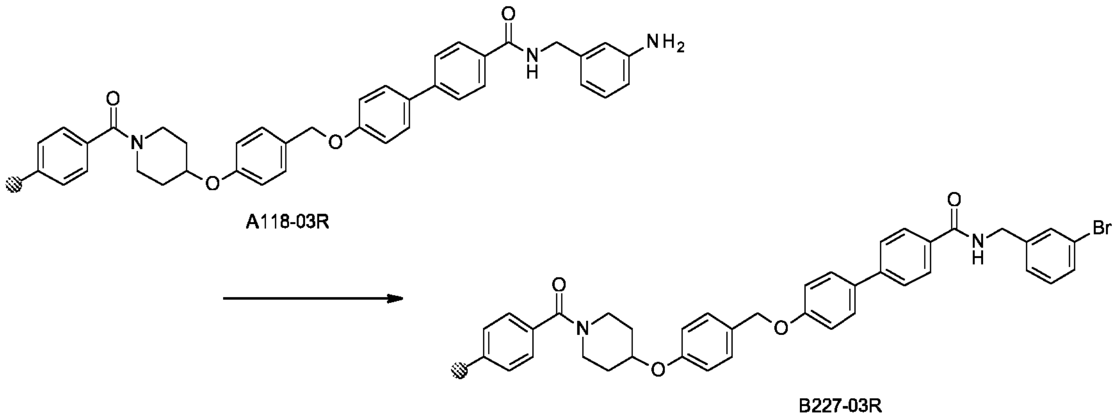

Under a nitrogen atmosphere, solid-phase supported $ArNH_2$ (A118-03R) (loading rate: 0.197 mmol/g, 20 mg, 0.0039 mmol) and THF (0.3 mL) were added to a 0.6 mL glass vial and shaken at room temperature for 1 hour. $NaNO_2$ (sodium nitrite, 6.8 mg, 0.099 mmol), $BrCCl_3$ (bromotrichloromethane, 9.7 μL, 0.099 mmol), AcOH (22.6 μL, 0.394 mmol), and $H_2O$ (0.1 mL) were added thereto, and the mixture was shaken at room temperature for 21 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 73% of compound B227 of interest was observed.

[Formula 496]

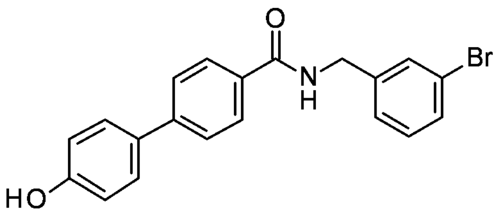

Compound B227

LRMS: m/z 382, 284[M+H]$^+$

Retention time: 1.081 min (analysis conditions SMD-FA05-1, 299 nm)

Example 2-19-3-2: Change of solvent in Sandmeyer bromination reaction to solid- phase supported $ArNH_2$ (A118-03R)

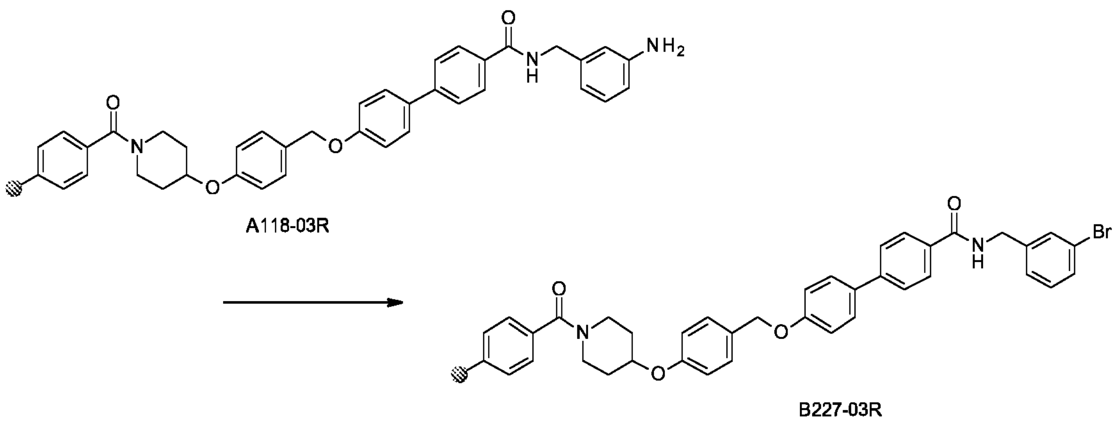

Reaction was carried out by changing the reaction solvent from THF to 1,4-dioxane and increasing the number of reagent equivalents, for the purpose of improving an ArBr production ratio.

Under a nitrogen atmosphere, solid-phase supported $ArNH_2$ (A118-03R) (loading rate: 0.197 mmol/g, 20 mg, 0.0039 mmol) and 1,4-dioxane (0.3 mL) were added to a 0.6 mL glass vial and shaken at room temperature for 1 hour. $NaNO_2$ (sodium nitrite, 13.6 mg, 0.197 mmol), $BrCCl_3$ (bromotrichloromethane, 19.4 μL, 0.197 mmol), AcOH (22.6 μL, 0.394 mmol), and $H_2O$ (0.1 mL) were added thereto, and the mixture was shaken at room temperature for 3 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 86% of compound B227 of interest was observed.

Example 2-19-3-3: Sandmeyer bromination reaction to solid-phase supported $ArNH_2$ (A118-03R): study of reaction using nitrous acid ester as diazotization reagent

Under reaction conditions using sodium nitrite, water is used for dissolving the sodium nitrite. For solid-phase reaction, particularly, library synthesis, it is preferred to select an organic solvent that allows a resin to swell more easily. Accordingly, under reaction conditions that employed nitrous acid ester as a diazotization reagent in an organic solvent and was considered applicable to a wide scope of substrates, Sandmeyer reaction was carried out with reference to a non patent literature (Org. Biomol. Chem. 2019, 17, 88) or a non patent literature (Tetrahedron, 2013, 69, 3511) by selecting hexyl nitrite as a diazotization reagent, TMSBr (bromotrimethylsilane) as a brominating agent, and PPTS (pyridinium p-toluenesulfonate) as an acid, and using DCE (dichloroethane) as a reaction solvent.

[Formula 497]

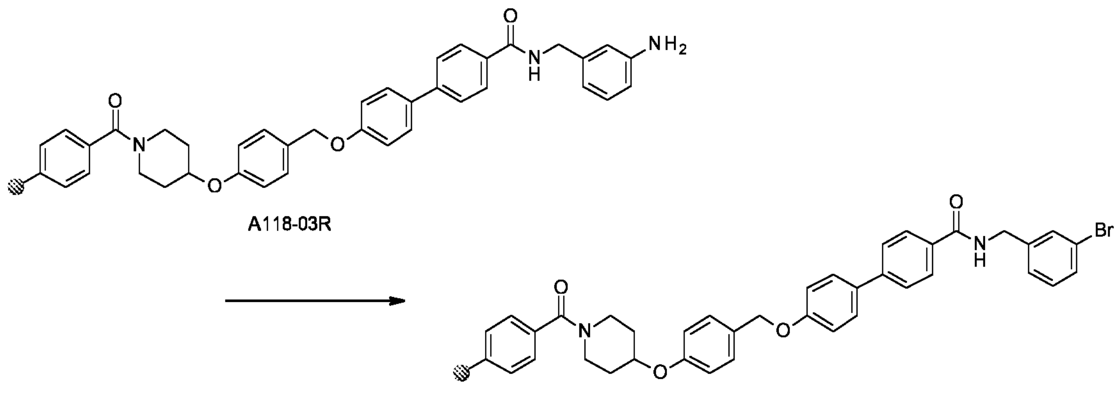

A118-03R

B227-03R

Under a nitrogen atmosphere, solid-phase supported $ArNH_2$ (A118-03R) (loading rate: 0.197 mmol/g, 20 mg, 0.0039 mmol) and DCE (0.4 mL) were added to a 0.6 mL glass vial and shaken at room temperature for 1 hour. PPTS (4.95 mg, 0.02 mmol), TMSBr (10.40 μL, 0.079 mmol), and hexyl nitrite (11.8 μL, 0.079 mmol) were added thereto, and the mixture was shaken at room temperature for 2 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 71 % of compound B227 of interest and 12% of a by-products ArCl (B228) were observed.

[Formula 498]

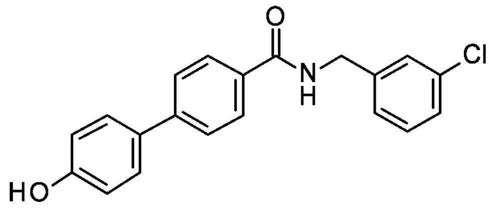

Compound B228

LRMS: m/z 338[M+H]+

Retention time: 1.047 min (analysis conditions SMD-FA05-1, 299 nm)

Example 2-19-3-4: Sandmeyer bromination reaction to solid-phase supported $ArNH_2$ (A118-03R): reaction using DBM (dibromomethane) as reaction solvent

Although not wishing to be bound by any theory, ArCl (B228) observed in Example 2-19-3-3 was presumably formed by the action of the reaction solvent DCE (dichloroethane) used as a chloro source. Therefore, reaction was carried out by selecting proven DBM (dibromomethane) used in a patent literature (WO2020178282) as a reaction solvent.

[Formula 499]

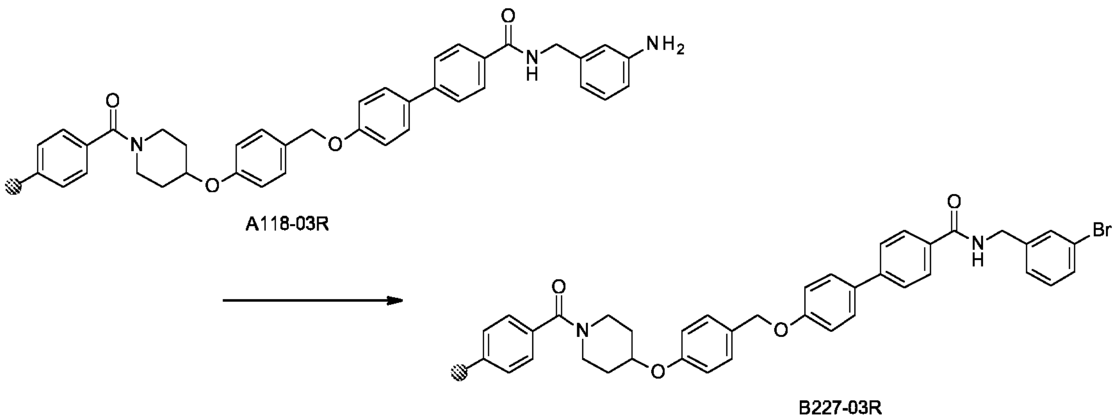

Under a nitrogen atmosphere, solid-phase supported $ArNH_2$ (A118-03R) (loading rate: 0.197 mmol/g, 20 mg, 0.0039 mmol) and DBM (dibromomethane) (0.4 mL) were added to a 0.6 mL glass vial and shaken at room temperature for 1 hour. PPTS (4.95 mg, 0.02 mmol), TMSBr (10.40 μL, 0.079 mmol), and hexyl nitrite (11.8 μL, 0.079 mmol) were added thereto, and the mixture was shaken at room temperature for 2.5 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 83% of compound B227 of interest was observed, whereas no by-product ArCl (B228) was observed. These results suggest, although not wishing to be bound by any theory, the possibility that a reaction solvent becomes a chloro source. It was confirmed that the formation of ArCl can be circumvented under this condition excluding a solvent capable of becoming a chloro source.

Example 2-19-3-5: Sandmeyer bromination reaction to solid-phase supported $ArNH_2$ (A118-03R): confirmation of scale-up reaction

Reaction was carried out on a 5-fold scale in order to confirm solid-phase Sandmeyer reaction progressed without problems even if the scale of the reaction was increased.

[Formula 500]

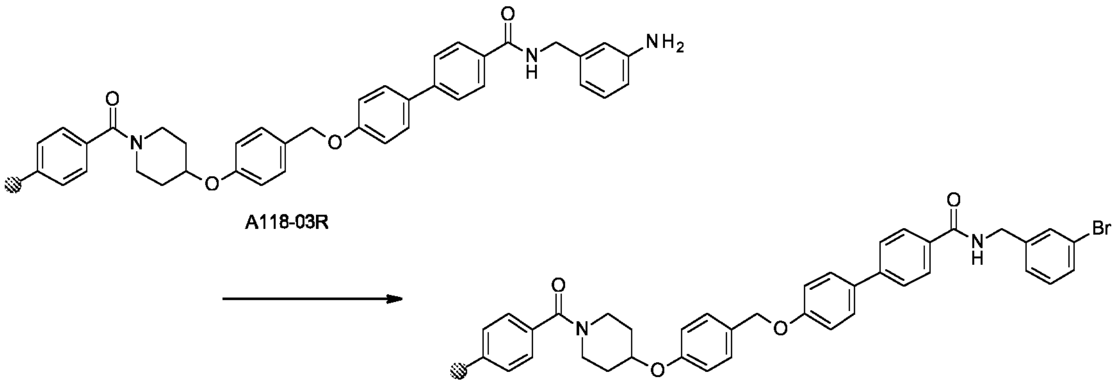

A118-03R

B227-03R

Under a nitrogen atmosphere, solid-phase supported ArNHz (A118-03R) (loading rate: 0.197 mmol/g, 100 mg, 0.0197 mmol) and DBM (dibromomethane) (2.0 mL) were added to 3.0 mL glass vial and shaken at room temperature for 1 hour. PPTS (24.8 mg, 0.099 mmol), TMSBr (52.0 μL, 0.394 mmol), and hexyl nitrite (58.7 μL, 0.394 mmol) were added thereto, and the mixture was shaken at room temperature for 4.5 hours.

The suspension of the reaction solution and the resin was transferred onto a filter, washed three times with DMA (0.1 mL), three times with MeOH (0.1 mL), and three times with DCM (0.1 mL), and then dipped in a 10% solution of TFA in DCM containing 0.05 M pentamethylbenzene (0.05 mL) for 2 minutes. After filtration, the solid phase was washed with DMA (0.05 mL), and MeCN (0.25 mL) was added to the filtrate to prepare an LC sample. The degree of progression of the reaction was measured by LC-MS analysis. As a result, 85% of compound B227 of interest was observed. The degree of progression of reaction similar to that on a 20-mg scale was confirmed even if the scale of the reaction was quintupled to 100 mg. Thus, any problem caused by scale-up was not seen.

From these results, it was confirmed that ArNH2 can be converted to ArBr through Sandmeyer reaction using sodium nitrite or hexyl nitrite as a diazotization reagent.

Example 3; Practice example of library synthesis at high purity and high yield

Both the securing of the diversity of library compounds and the ensuring of library quality are important factors for the success or failure of compound screening, which is the purpose of use of a library, and are important challenges in supplying a compound library.

Exemplary synthesis of a mixture library of compounds in which the diversity of building blocks, which is taken into consideration in general library synthesis, and diversity imparted to linking moieties (linkers) that link the building blocks as conceived by the present invention are multiplied will be shown as a solution to the challenge to diversity. Also, a practice example of the step of confirming that "intended library compounds are synthesized and exist in a mixture library" as well as "difference in content among the library compounds is small" will be shown as an exemplary solution to the challenge to the ensuring of quality.

More specifically, mixture library synthesis having bond diversity was practiced using the reaction conditions illustrated in Example 2 in which a wide scope of substrates was reactable at a high yield and a high purity. A practice example of library synthesis of compounds containing a UV tag having an absorption band at a specific UV wavelength will be shown as an exemplary step of confirming that the compound library can be obtained with expected quality. Use of the UV tag that permits analysis at a specific UV wavelength enables the analysis to be conducted in a state where difference in molar absorbance coefficient depending on the distinctive absorption band of each library compound structure is reduced.

Example 3 illustrates exemplary synthesis of a library in which the "diversity of core blocks" that form pharmacophores and the "diversity of linkers" are multiplied, by exploiting the findings obtained in Example 2. Figure 1 shows the "diversity of core blocks" and the "diversity of linkers" contained in the library.

From among diverse compounds brought about by the combination of "diversity of core blocks" and the "diversity of linkers" shown in Figure 1, 1,200 compounds were set as one example of library compounds to carry out mixture library synthesis. The library synthesis was performed by the following method: first building blocks were supported onto the solid phase, and after introduction of each building block to a solid-phase supported compound, i.e., after bond formation reaction, the resulting products were homogeneously mixed on a type basis of functional groups present on the introduced building blocks, and equally or unequally divided according to the need, followed by bond formation reaction. Hereinafter, the method will be shown as an exemplary mixture library synthesis method having bond diversity.

In the library synthesis given below, the building blocks shown in Table LBB01 were used.

[Table 67]

[Table LBB01]

| Compound No. | Structural formula | Compound name |
|---|---|---|
| BB01 | 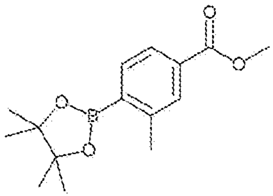 | Methyl 3-methyl-4-(4,4,5,5-tetramethyl-1,3,2-dioxaborolan-2-yl)benzoate |
| BB02 | 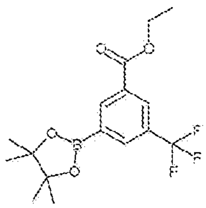 | Ethyl 3-(4,4,5,5-tetramethyl-1,3,2-dioxaborolan-2-yl)-5-(trifluoromethyl)benzoate |
| BB03 | 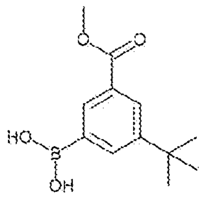 | (3-tert-Butyl-5-methoxycarbonylphenyl)boronic acid |
| BB04 | 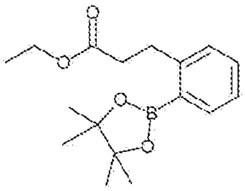 | Ethyl 3-[2-(4,4,5,5-tetramethyl-1,3,2-dioxaboloran-2-yl)phenyl]propanoate |
| BB05 | 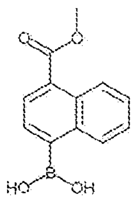 | (4-Methoxycarbonylnaphthalen-1-yl)boronic acid |

[Table 68]

| | | |
|---|---|---|
| BB06 | 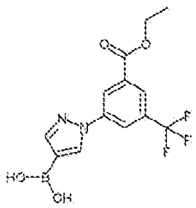 | {1-[3-Ethoxycarbonyl-5-(trifluoromethyl)phenyl]pyrazol-4-yl}boronic acid |
| BB07 | 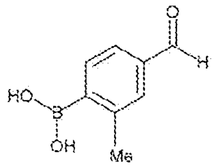 | (4-Formyl-2-methylphenyl)boronic acid |
| BB08 | 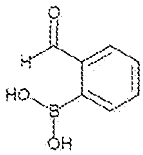 | (2-Formylphenyl)boronic acid |
| BB09 | 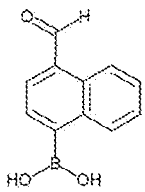 | (4-Formylnaphthalen-1-yl)boronic acid |
| BB10 | 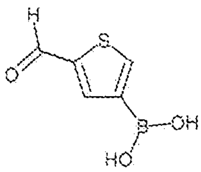 | (5-Formylthiophen-3-yl)boronic acid |
| BB11 | 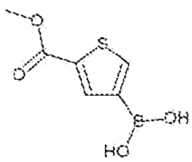 | (5-(Methoxycarbonyl)thiophen-3-yl)boronic acid |

[Table 69]

| | | |
|---|---|---|
| BB12 | 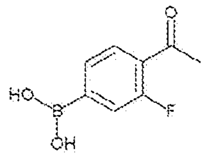 | (4-Acetyl-3-fluorophenyl)boronic acid |
| BB13 | 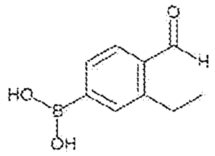 | (3-Ethyl-4-formylphenyl)boronic acid |
| BB14 | 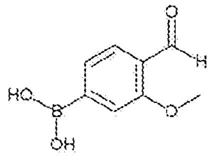 | (4-Formyl-3-methoxyphenyl)boronic acid |
| BB15 | 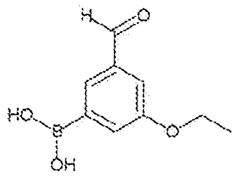 | (3-Ethoxy-5-formylphenyl)boronic acid |
| BB16 | 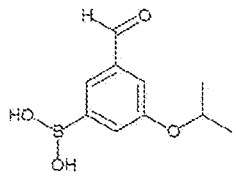 | (3-Formyl-5-propan-2-yloxyphenyl)boronic acid |
| BB17 | 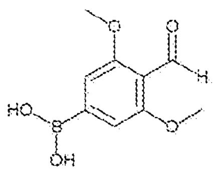 | (4-Formyl-3,5-dimethoxyphenyl)boronic acid |
| BB18 | 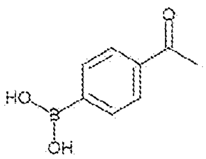 | (4-Acetylphenyl)boronic acid |

[Table 70]

| | | |
|---|---|---|
| BB19 | 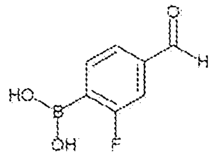 | (2-Fluoro-4-formylphenyl)boronic acid |
| BB20 | 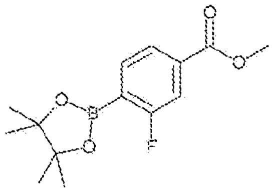 | Methyl 3-fluoro-4-(4,4,5,5-tetramethyl-1,3,2-dioxaborolan-2-yl)benzoate |
| BB21 | 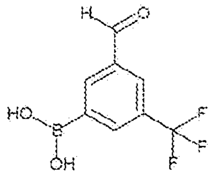 | [3-Formyl-5-(trifluoromethyl)phenyl]boronic acid |
| BB22 | 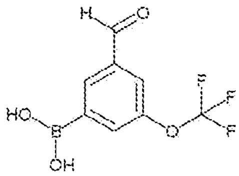 | [3-Formyl-5-(trifluoromethoxy)phenyl]boronic acid |
| BB23 | 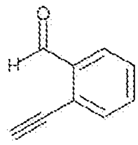 | 2-Ethynylbenzaldehyde |
| BB24 | 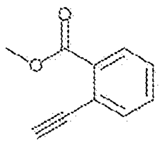 | Methyl 2-ethynylbenzoate |

[Table 71]

| | | |
|---|---|---|
| BB25 | 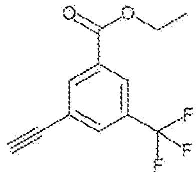 | Ethyl 3-ethynyl-5-(trifluoromethyl)benzoate |
| BB26 | 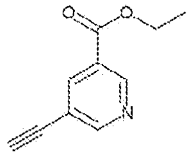 | Ethyl 5-ethynylpyridine-3-carboxylate |
| BB27 | 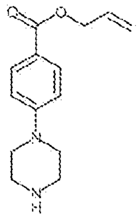 | Allyl 4-(piperazin-1-yl)benzoate |
| BB28 | 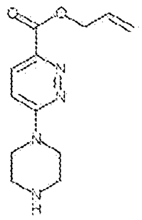 | Allyl 6-(piperazin-1-yl)pyridazine-3-carboxylate |
| BB29 | 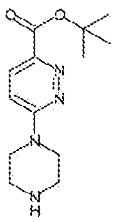 | tert-Butyl 6-(piperazin-1-yl)pyridazine-3-carboxylate |
| BB30 | 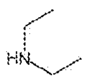 | Diethylamine |

[Table 72]

| | | |
|---|---|---|
| BB31 | 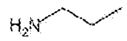 | Propylamine |
| BB32 | 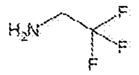 | 2,2,2-Trifluoroethanamine |
| BB33 | 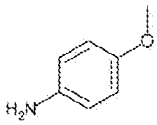 | 4-Methoxyaniline |
| BB34 | 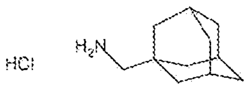 | 1-Adamantylmethanamine hydrochloride |
| BB35 | 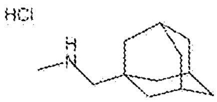 | 1-(1-Adamantyl)-N-methylmethanamine hydrochloride |
| BB36 | 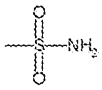 | Methanesulfonamide |
| BB37 | 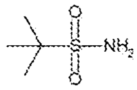 | 2-Methylpropane-2-sulfonamide |
| BB38 | 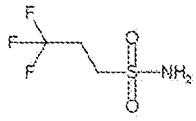 | 3,3,3-Trifluoropropane-1-sulfonamide |
| BB39 | 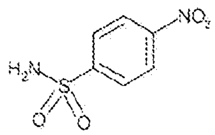 | 4-Nitrobenzenesulfonamide |

[Table 73]

| | | |
|---|---|---|
| BB40 | 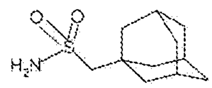 | 1-Adamantylmethanesulfonamide |
| BB41 | 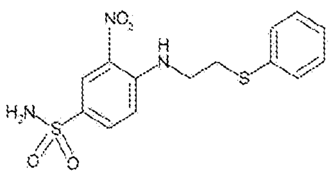 | 3-Nitro-4-{[2-(phenylthio)ethyl] amino}benzenesulfonamide |

In this Example 3, a compound supported on brominated Wang resin (4- (bromomethyl)phenoxymethyl polystyrene) (e.g., Merck KGaA, 4-(benzyloxy)benzyl bromide, polymer-bound) was used, and "-01R" was described at the end of the compound number of the compound supported on the solid phase. In the tables in Example 3, the solid-phase supported compound may be indicated by a compound number excluding "-01R", also because only a structural formula after cleavage is described in conversion reaction.

Example 3-1: Practice of mixture library synthesis to which bond formation reaction is applied through Suzuki coupling reaction

Example 3-1-1: Synthesis of mixEB01-01R

[Formula 501]

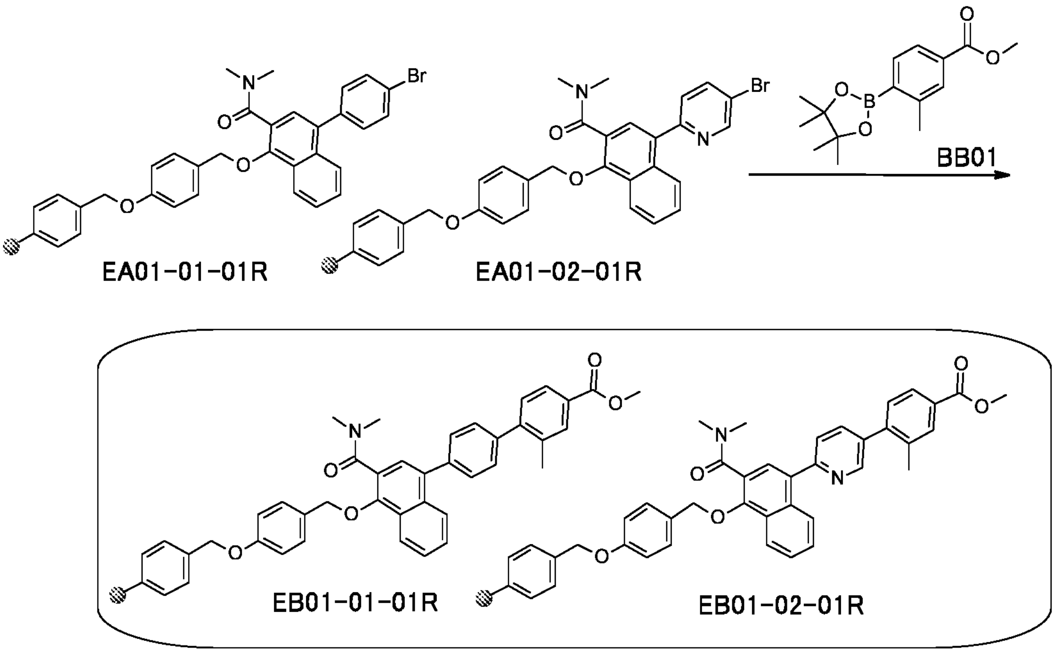


EA01-01-01R EA01-02-01R

EB01-01-01R EB01-02-01R

mixEB01-01R

Under a nitrogen atmosphere, NMP (1.33 mL) was added to EA01-01-01R (48 mg, loading rate: 0.4 mmol/g, 0.019 mmol) and EA01-02-01R (52 mg, loading rate: 0.37 mmol/g, 0.019 mmol) in a 2 mL glass vial, and the mixture was shaken at room temperature for 1 hour. Under a nitrogen atmosphere, methyl 3-methyl-4-(4,4,5,5-tetramethyl-1,3,2-dioxaborolan-2- yl)benzoate (BB01) (63 mg, 0.228 mmol), an aqueous $K_3PO_4$ solution (1.5 M, 152 μL, 0.228 mmol), and a solution of $P(Cy)_3$ Pd G3 in NMP (0.04 M, 190 μL, 7.6 μmol) were added thereto, and the mixture was shaken at 90°C for 3 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), and three times with DCM (2 mL), and the resin was then dried under reduced pressure to obtain the title compound shown in Table EB01.

[Table 74]

[EB01]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB01 | mixEB01-01R | EB01-01-01R | mixEB01 | EB01-01 | Methyl 4-[4-[3-(dimethylcarbamoyl)-4-hydroxynaphthalen-1-yl]phenyl]-3-methylbenzoate | 3 | 439.2 | 440 | 1.51 | SMD-FA05-1 |
| | | EB01-02-01R | | EB01-02 | Methyl 4-[6-[3-(dimethylcarbamoyl)-4-hydroxynaphthalen-1-yl]pyridin-3-yl]-3-methylbenzoate | | 440.2 | 441 | 1.21 | |

Example 3-1-2: Synthesis of mixtures mixEB02-01R to mixEB04-01R

The title compounds shown in Table EB02 were synthesized in the same manner as in Example 3-1-1 using compound EA01-01-01R (48 mg, loading rate: 0.4 mmol/g, 0.019 mmol), EA01-02-01R (52 mg, loading rate: 0.37 mmol/g, 0.019 mmol), and the boronic acid or boronic acid ester reagents (BB02 to BB04) shown in Table LBB01. The structures of the building blocks used and the compounds to be analyzed, the reaction times, and analysis data are also shown in Table EB02.

[Table 75]

[Table EB02]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB02 | mixEB02-01R | EB02-01-01R | mixEB02 | EB02-01 | Ethyl 3-[4-[3-(dimethylcarbamoyl)-4-hydroxynaphthalen-1-yl]phenyl]-5-(trifluoromethyl)benzoate | 5 | 507.2 | 508 | 1.66 | SMD-FA05-1 |
| | | EB02-02-01R | | EB02-02 | Ethyl 3-[6-[3-(dimethylcarbamoyl)-4-hydroxynaphthalen-1-yl]pyridin-3-yl]-5-(trifluoromethyl)benzoate | | 508.2 | 509 | 1.45 | |

| | | | | | | | | | | |
|---|---|---|---|---|---|---|---|---|---|---|
| BB03 | mixEB03-01R | EB02-03-01R | mixEB03 | EB02-03 | Methyl 3-tert-butyl-5-[4-[3-(dimethylcarbamoyl)-4-hydroxynaphthalen-1-yl]phenyl]benzoate | 5 | 481.2 | 482 | 1.70 | SMD-FA05-1 |
| | | EB02-04-01R | | EB02-04 | Methyl 3-tert-butyl-5-[6-[3-(dimethylcarbamoyl)-4-hydroxynaphthalen-1-yl]pyridin-3-yl]benzoate | | 482.2 | 483 | 1.45 | |
| BB04 | mixEB04-01R | EB02-05-01R | mixEB04 | EB02-05 | Ethyl 3-[2-[4-[3-(dimethylcarbamoyl)-4-hydroxynaphthalen-1-yl]phenyl]phenyl]propanoate | 5 | 467.2 | 468 | 1.53 | SMD-FA05-1 |
| | | EB02-06-01R | | EB02-06 | Ethyl 3-[2-[6-[3-(dimethylcarbamoyl)-4-hydroxynaphthalen-1-yl]pyridin-3-yl]phenyl]propanoate | | 468.2 | 469 | 1.22 | |

Example 3-1-3: Synthesis of mixture mixEB05-01R

[Formula 502]

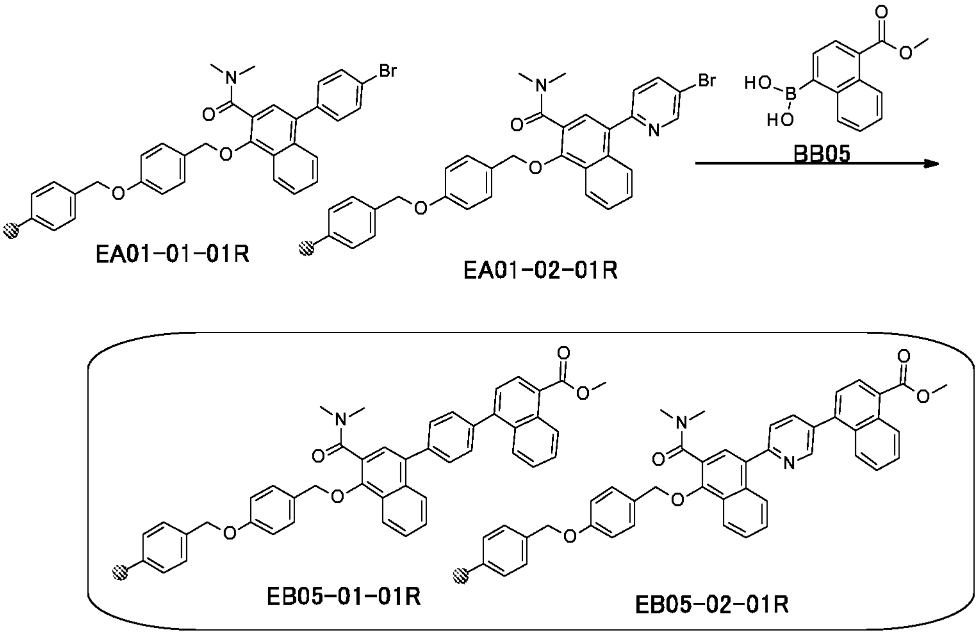

EA01-01-01R EA01-02-01R BB05

EB05-01-01R EB05-02-01R

mixEB05-01R

Under a nitrogen atmosphere, NMP (1.33 mL) was added to EA01-01-01R (48 mg, loading rate: 0.4 mmol/g, 0.019 mmol) and EA01-02-01R (52 mg, loading rate: 0.37 mmol/g, 0.019 mmol) in a 2 mL glass vial, and the mixture was shaken at room temperature for 1 hour. Under a nitrogen atmosphere, (4-methoxycarbonylnaphthalen-1-yl)boronic acid (BB05) (105 mg, 0.456 mmol), an aqueous $K_3PO_4$ solution (1.5 M, 152 μL, 0.228 mmol), and a solution of $P(Cy)_3$ Pd G3 in NMP (0.04 M, 190 μL, 7.6 μmol) were added thereto, and the mixture was shaken at 90°C for 5 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), and three times with DCM (2 mL), and the resin was then dried under reduced pressure to obtain the title compound shown in Table EB05. The structures of the building block used and the compounds to be analyzed, the reaction time, and analysis data are also shown in Table EB05.

[Table 76]

[Table EB05]

| No. of BB used | No. of synthesized mixture | Resin Compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB05 | mixEB05 -01R | EB05-01 -01R | mixEB05 | EB05-01 | Methyl 4-[4-[3-(dimethylcarbamoyl)-4-hydroxynaphthalen-1-yl]phenyl]naphthalene-1-carboxylate | 5 | 475.2 | 476 | 1.60 | SMD-FA05-1 |
| | | EB05-02 -01R | | EB05-02 | Methyl 4-[6-[3-(dimethylcarbamoyl)-4-hydroxynaphthalen-1-yl]pyridin-3-yl]naphthalene-1-carboxylate | | 476.2 | 477 | 1.34 | |

Example 3-1-4: Synthesis of mixture mixEB06-01R

The title compound shown in Table EB06 was synthesized in the same manner as in Example 3-1-3 using compound EA01-01-01R (48 mg, loading rate: 0.4 mmol/g, 0.019 mmol), EA01-02-01R (52 mg, loading rate: 0.37 mmol/g, 0.019 mmol), and [1-[3- ethoxycarbonyl-5-(trifluoromethyl)phenyl]pyrazol-4-yl]boronic acid (BB06) shown in Table LBB01. The structures of the building block used and the compounds to be analyzed, the reaction time, and analysis data are also shown in Table EB06.

[Table 77]

[Table EB06]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB06 | mixEB06 -01R | EB06-01 -01R | mixEB06 | EB06-01 | Ethyl 3-[4-[4-[3-(dimethylcarbamoyl)-4-hydroxynaphthalen-1-yl]phenyl]pyrazol-1-yl]-5-(trifluoromethyl)benzoate | 3 | 573.2 | 574 | 1.67 | SMD-FA05-1 |
| | | EB06-02 -01R | | EB06-02 | Ethyl 3-[4-[6-[3-(dimethylcarbamoyl)-4-hydroxynaphthalen-1-yl]pyridin-3-yl]pyrazol-1-yl]-5-(trifluoromethyl)benzoate | | 574.2 | 575 | 1.47 | |

Example 3-1-5: Synthesis of mixture mixEB07-01R

[Formula 503]

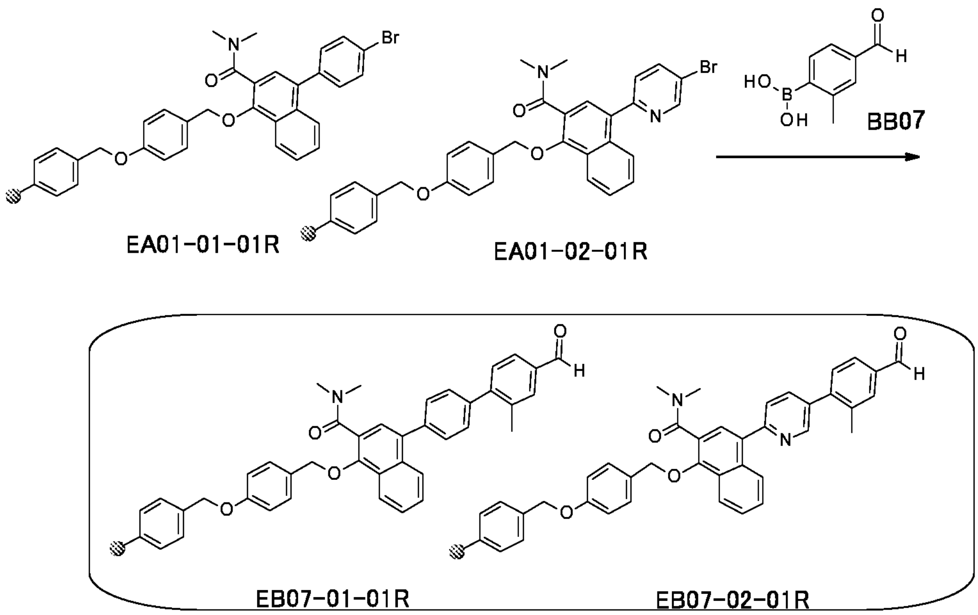

EA01-01-01R EA01-02-01R BB07

EB07-01-01R EB07-02-01R

mixEB07-01R

Under a nitrogen atmosphere, NMP (1 mL) was added to compound EA01-01-01R (48 mg, loading rate: 0.4 mmol/g, 0.019 mmol) and compound EA01-02-01R (52 mg, loading rate: 0.37 mmol/g, 0.019 mmol) in a 2 mL glass vial, and the mixture was shaken at room temperature for 1 hour. Under a nitrogen atmosphere, (4-formyl-2- methylphenyl)boronic acid (BB07) (37 mg, 0.228 mmol), $Na_2CO_3$ (24 mg, 0.228 mmol), water (50 μL), and a solution of $P(Cy)_3$ Pd G3 in NMP (0.04 M, 190 μL, 7.6 μmol) were added thereto, and the mixture was shaken at 90°C for 21 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), and three times with DCM (2 mL), and the resin was then dried under reduced pressure to obtain the title compound shown in Table EB07. The structures of the building block used and the compounds to be analyzed, the reaction time, and analysis data are also shown in Table EB07.

[Table 78]

[Table EB07]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB07 | mixEB07-01R | EB07-01-01R | mixEB07 | EB07-01 | 4-[4-(4-Formyl-2-methylphenyl)phenyl]-1-hydroxy-N,N-dimethylnaphthalene-2-carboxamide | 21 | 409.2 | 410 | 1.42 | SMD-FA05-1 |
| | | EB07-02-01R | | EB07-02 | 4-[5-(4-Formyl-2-methylphenyl)pyridin-2-yl]-1-hydroxy-N,N-dimethylnaphthalene-2-carboxamide | | 410.2 | 411 | 1.12 | |

Example 3-1-6: Synthesis of mixtures mixEB08-01R to mixEB18-01R

The title compounds shown in Table EB08 were synthesized in the same manner as in Example 3-1-5 using compound EA01-01-01R (48 mg, loading rate: 0.4 mmol/g, 0.019 mmol), compound EA01-02-01R (52 mg, loading rate:

0.37 mmol/g, 0.019 mmol), and the boronic acid reagents (BB08 to BB18) shown in Table LBB01. The structures of the building blocks used and the compounds to be analyzed, the reaction times, and analysis data are also shown in Table EB08.

[Table 79]

[Table EB08]

| No. of BB used | No. of synthesized mixture | Resin compound No. | No. of mixture after cleavage | Compound No. after cleavage | Compound name | Reaction time (hr) | Exact MS of compound after cleavage | Detected MS m/z $[M+H]^+$ | LC retention time | Analysis method |
|---|---|---|---|---|---|---|---|---|---|---|
| BB08 | mixEB08-01R | EB08-01-01R | mixEB08 | EB08-01 | 4-[4-(2-Formylphenyl)phenyl]-1-hydroxy-N,N-dimethylnaphthalene-2-carboxamide | 21 | 395.2 | 396 | 1.38 | SMD-FA05-1 |

| | | | | | | | | | | |
|---|---|---|---|---|---|---|---|---|---|---|
| | | EB08-02 -01R | | EB08-02 | 4-[5-(2-Formylphenyl)pyridin-2-yl]-1-hydroxy-N,N-dimethylnaphthalene-2-carboxamide | | 396.1 | 397 | 1.08 | |
| BB09 | mixEB09 -01R | EB08-03 -01R | mixEB09 | EB08-03 | 4-[4-(4-Formylnaphthalen-1-yl)phenyl]-1-hydroxy-N,N-dimethylnaphthalene-2-carboxamide | 21 | 445.2 | 446 | 1.53 | SMD-FA05-1 |
| | | EB08-04 -01R | | EB08-04 | 4-[5-(4-Formylnaphthalen-1-yl)pyridin-2-yl]-1-hydroxy-N,N-dimethylnaphthalene-2-carboxamide | | 446.2 | 447 | 1.27 | |
| BB10 | mixEB10 -01R | EB08-05 -01R | mixEB10 | EB08-05 | 4-[4-(5-Formylthiophen-3-yl)phenyl]-1-hydroxy-N,N-dimethylnaphthalene-2-carboxamide | 21 | 401.1 | 402 | 1.31 | SMD-FA05-1 |
| | | EB08-06 -01R | | EB08-06 | 4-[5-(5-Formylthiophen-3-yl)pyridin-2-yl]-1-hydroxy-N,N-dimethylnaphthalene-2-carboxamide | | 402.1 | 403 | 1.02 | |
| BB11 | mixEB11 -01R | EB08-07 -01R | mixEB11 | EB08-07 | Methyl 4-[4-[3-(dimethylcarbamoyl)-4-hydroxynaphthalen-1-yl]phenyl]thiophene-2-carboxylate | 21 | 431.1 | 432 | 1.42 | SMD-FA05-1 |
| | | EB08-08 -01R | | EB08-08 | Methyl 4-[6-[3-(dimethylcarbamoyl)-4-hydroxynaphthalen-1-yl]pyridin-3-yl]thiophene-2-carboxylate | | 432.1 | 433 | 1.13 | |
| BB12 | mixEB12 -01R | EB08-09 -01R | mixEB12 | EB08-09 | 4-[4-(4-Acetyl-3-fluorophenyl)phenyl]-1-hydroxy-N,N-dimethylnaphthalene-2-carboxamide | 21 | 427.2 | 428 | 1.44 | SMD-FA05-1 |
| | | EB08-10 -01R | | EB08-10 | 4-[5-(4-Acetyl-3-fluorophenyl)pyridin-2-yl]-1-hydroxy-N,N-dimethylnaphthalene-2-carboxamide | | 428.2 | 429 | 1.19 | |
| BB13 | mixEB13 -01R | EB08-11 -01R | mixEB13 | EB08-11 | 4-[4-(3-Ethyl-4-formylphenyl)phenyl]-1-hydroxy-N,N-dimethylnaphthalene-2-carboxamide | 26 | 423.2 | 424 | 1.50 | SMD-FA05-1 |
| | | EB08-12 -01R | | EB08-12 | 4-[5-(3-Ethyl-4-formylphenyl)pyridin-2-yl]-1-hydroxy-N,N-dimethylnaphthalene-2-carboxamide | | 424.2 | 425 | 1.23 | |
| BB14 | mixEB14 -01R | EB08-13 -01R | mixEB14 | EB08-13 | 4-[4-(4-Formyl-3-methoxyphenyl)phenyl]-1-hydroxy-N,N-dimethylnaphthalene-2-carboxamide | 26 | 425.2 | 426 | 1.38 | SMD-FA05-1 |

| | | | | | | | | | | |
|---|---|---|---|---|---|---|---|---|---|---|
| | | EB08-14 -01R | | EB08-14 | 4-[5-(4-Formyl-3-methoxyphenyl)pyridin-2-yl]-1-hydroxy-N,N-dimethylnaphthalene-2-carboxamide | | 426.2 | 427 | 1.12 | |
| BB15 | mixEB15 -01R | EB08-15 -01R | mixEB15 | EB08-15 | 4-[4-(3-Ethoxy-5-formylphenyl)phenyl]-1-hydroxy-N,N-dimethylnaphthalene-2-carboxamide | 26 | 439.2 | 440 | 1.47 | SMD-FA05-1 |
| | | EB08-16 -01R | | EB08-16 | 4-[5-(3-Ethoxy-5-formylphenyl)pyridin-2-yl]-1-hydroxy-N,N-dimethylnaphthalene-2-carboxamide | | 440.2 | 441 | 1.21 | |
| BB16 | mixEB16 -01R | EB08-17 -01R | mixEB16 | EB08-17 | 4-[4-(3-Formyl-5-propan-2-yloxyphenyl)phenyl]-1-hydroxy-N,N-dimethylnaphthalene-2-carboxamide | 26 | 453.2 | 454 | 1.52 | SMD-FA05-1 |
| | | EB08-18 -01R | | EB08-18 | 4-[5-(3-Formyl-5-propan-2-yloxyphenyl)pyridin-2-yl]-1-hydroxy-N,N-dimethylnaphthalene-2-carboxamide | | 454.2 | 455 | 1.27 | |
| BB17 | mixEB17 -01R | EB08-19 -01R | mixEB17 | EB08-19 | 4-[4-(4-Formyl-3,5-dimethoxyphenyl)phenyl]-1-hydroxy-N,N-dimethylnaphthalene-2-carboxamide | 26 | 455.2 | 456 | 1.27 | SMD-FA05-1 |
| | | EB08-20 -01R | | EB08-20 | 4-[5-(4-Formyl-3,5-dimethoxyphenyl)pyridin-2-yl]-1-hydroxy-N,N-dimethylnaphthalene-2-carboxamide | | 456.2 | 457 | 1.03 | |
| BB18 | mixEB18 -01R | EB08-21 -01R | mixEB18 | EB08-21 | 4-[4-(4-Acetylphenyl)phenyl]-1-hydroxy-N,N-dimethylnaphthalene-2-carboxamide | 51 | 409.2 | 410 | 1.37 | SMD-FA05-1 |
| | | EB08-22 -01R | | EB08-22 | 4-[5-(4-Acetylphenyl)pyridin-2-yl]-1-hydroxy-N,N-dimethylnaphthalene-2-carboxamide | | 410.2 | 411 | 1.08 | |

Example 3-1-7: Synthesis of mixture mixEB19-01R

[Formula 504]

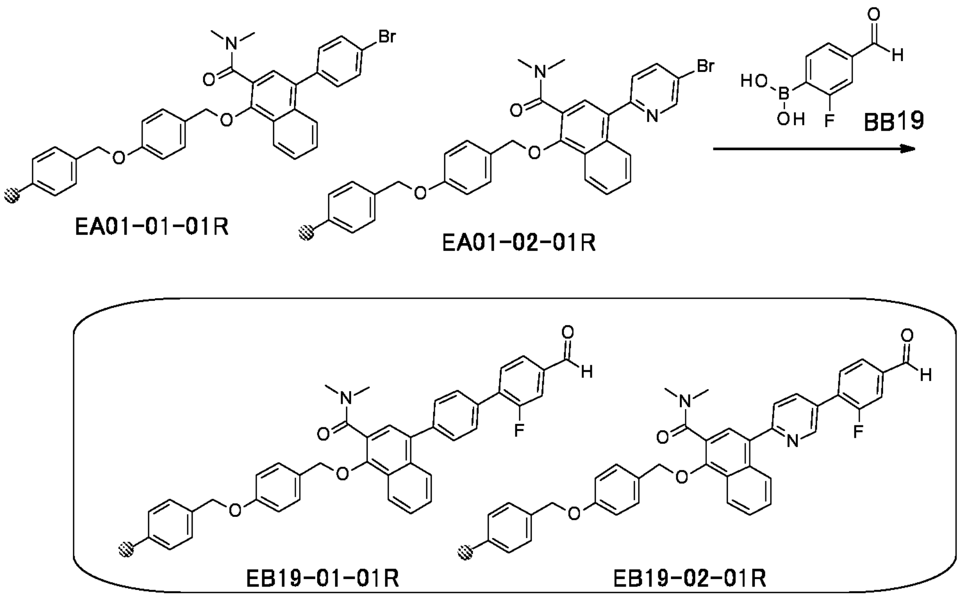


EA01-01-01R EA01-02-01R

EB19-01-01R EB19-02-01R

mixEB19-01R

Under a nitrogen atmosphere, 1,4-dioxane (1 mL) was added to compound EA01- 01-01R (48 mg, loading rate: 0.4 mmol/g, 0.019 mmol) and compound EA01-02-01R (52 mg, loading rate: 0.37 mmol/g, 0.019 mmol) in a 2 mL glass vial, and the mixture was shaken at room temperature for 1 hour. Under a nitrogen atmosphere, (2-fluoro-4-formylphenyl)boronic acid (BB19) (38.3 mg, 0.228 mmol), $K_3PO_4$-$H_2O$ (52.5 mg, 0.228 mmol), pinacol (26.9 mg, 0.228 mmol), and a solution of $P(Cy)_3$ Pd G3 in 1,4-dioxane (0.04 M, 190 μL, 7.6 μmol) were added thereto, and the mixture was shaken at 90°C for 19 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), and three times with DCM (2 mL), and the resin was then dried under reduced pressure to obtain the title compound shown in Table EB19. The structures of the building block used and the compounds to be analyzed, the reaction time, and analysis data are also shown in Table EB19.

[Table 80]

[Table EB19]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB19 | mixEB19-01R | EB19-01-01R | mixEB19 | EB19-01 | 4-[4-(2-Fluoro-4-formylphenyl)phenyl]-1-hydroxy-N,N-dimethylnaphthalene-2-carboxamide | 19 | 413.1 | 414 | 1.38 | SMD-FA05-1 |
| | | EB19-02-01R | | EB19-02 | 4-[5-(2-Fluoro-4-formylphenyl)pyridin-2-yl]-1-hydroxy-N,N-dimethylnaphthalene-2-carboxamide | | 414.1 | 415 | 1.14 | |

Example 3-1-8: Synthesis of mixture mixEB20-01R

The title compound shown in Table EB20 was synthesized in the same manner as in Example 3-1-7 using

compound EA01-01-01R (48 mg, loading rate: 0.4 mmol/g, 0.019 mmol), EA01-02-01R (52 mg, loading rate: 0.37 mmol/g, 0.019 mmol), and methyl 3- fluoro-4-(4,4,5,5-tetramethyl-1,3,2-dioxaborolan-2-yl)benzoate (BB20) shown in Table LBB01. The structures of the building block used and the compounds to be analyzed, the reaction time, and analysis data are also shown in Table EB20.

[Table 81]

[Table EB20]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB20 | mixEB20 -01R | EB20-01 -01R | mixEB20 | EB20-01 | Methyl 4-[4-[3-(dimethylcarbamoyl)-4-hydroxynaphthalen-1-yl]phenyl]-3-fluorobenzoate | 19 | 443.2 | 444 | 1.49 | SMD-FA05-1 |
| | | EB20-02 -01R | | EB20-02 | Methyl 4-[6-[3-(dimethylcarbamoyl)-4-hydroxynaphthalen-1-yl]pyridin-3-yl]-3-fluorobenzoate | | 444.1 | 445 | 1.25 | |

Example 3-1-9: Synthesis of mixture mixEB21-01R

[Formula 505]

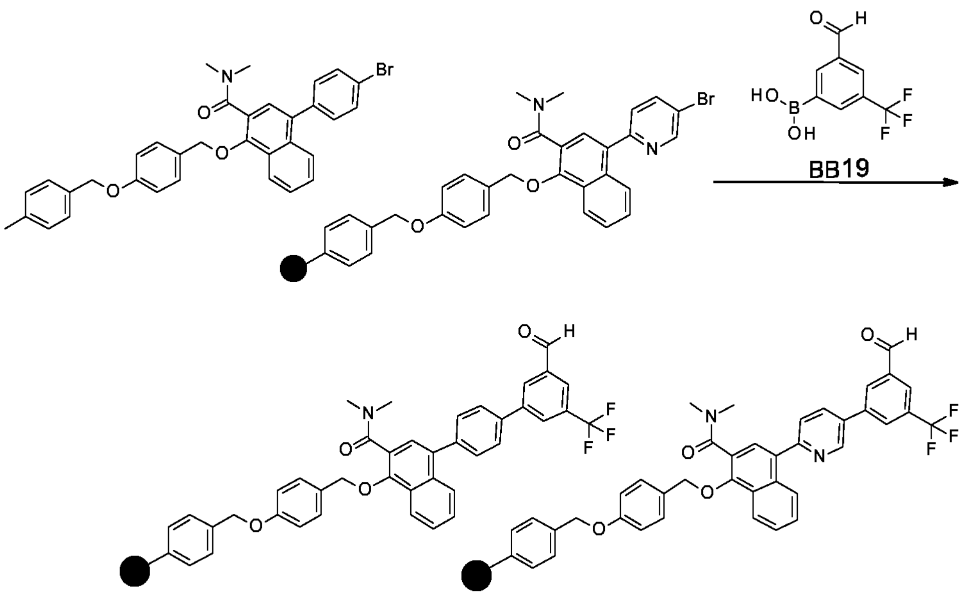


Under a nitrogen atmosphere, 4-MeTHP (1 mL) was added to compound EA01-01- 01R (48 mg, loading rate: 0.4 mmol/g, 0.019 mmol) and compound EA01-02-01R (52 mg, loading rate: 0.37 mmol/g, 0.019 mmol) in a 2 mL glass vial, and the mixture was shaken at room temperature for 1 hour. Under a nitrogen atmosphere, [3-formyl-5- (trifluoromethyl)phenyl]boronic acid (BB21) (49.7 mg, 0.228 mmol), $Na_2CO_3$ (24.2 mg, 0.228 mmol), and a solution of $P(Cy)_3$ Pd G3 in 4-MeTHP (0.04 M, 190 μL, 7.6 μmol) were added thereto. After shaking at 90°C for 19 hours, water (50 μL) was added thereto at room temperature, and the mixture was shaken at 90°C for 3 hours. The suspension of the reaction

solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), and three times with DCM (2 mL), and the resin was then dried under reduced pressure to obtain the title compound shown in Table EB21. The structures of the building block used and the compounds to be analyzed, the reaction time, and analysis data are also shown in Table EB21.

[Table 82]

[Table EB21]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB21 | mixEB21 -01R | EB21-01 -01R | mixEB21 | EB21-01 | 4-[4-[3-Formyl-5-(trifluoromethyl)phenyl] phenyl]-1-hydroxy-N,N-dimethylnaphthalene-2-carboxamide | 22 | 463.1 | 464 | 1.50 | SMD-FA05-1 |
| | | EB21-02 -01R | | EB21-02 | 4-[5-[3-Formyl-5-(trifluoromethyl)phenyl] pyridin-2-yl]-1-hydroxy-N,N-dimethylnaphthalene-2-carboxamide | | 464.1 | 465 | 1.27 | |

Example 3-1-10: Synthesis of mixture mixEB22-01R

The title compound shown in Table EB22 was synthesized in the same manner as in Example 3-1-9 using compound EA01-01-01R (48 mg, loading rate: 0.4 mmol/g, 0.019 mmol), EA01-02-01R (52 mg, loading rate: 0.37 mmol/g, 0.019 mmol), and [3-formyl- 5-(trifluoromethoxy)phenyl]boronic acid (BB22) shown in Table LBB01. The structures of the building block used and the compounds to be analyzed, the reaction time, and analysis data are also shown in Table EB22.

[Table 83]

[Table EB22]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB21 | mixEB22 -01R | EB22-01 -01R | mixEB22 | EB22-01 | 4-[4-[3-Formyl-5-(trifluoromethoxy)phenyl] phenyl]-1-hydroxy-N,N-dimethylnaphthalene-2-carboxamide | 22 | 479.1 | 480 | 1.52 | SMD-FA05-1 |
| | | EB22-02 -01R | | EB22-02 | 4-[5-[3-Formyl-5-(trifluoromethoxy)phenyl] pyridin-2-yl]-1-hydroxy-N,N-dimethylnaphthalene-2-carboxamide | | 480.1 | 481 | 1.30 | |

Example 3-2: Practice of mixture library synthesis to which bond formation reaction is applied through acetylene coupling reaction using Pd

Example 3-2-1: Synthesis of mixture mixEB23-01R

[Formula 506]

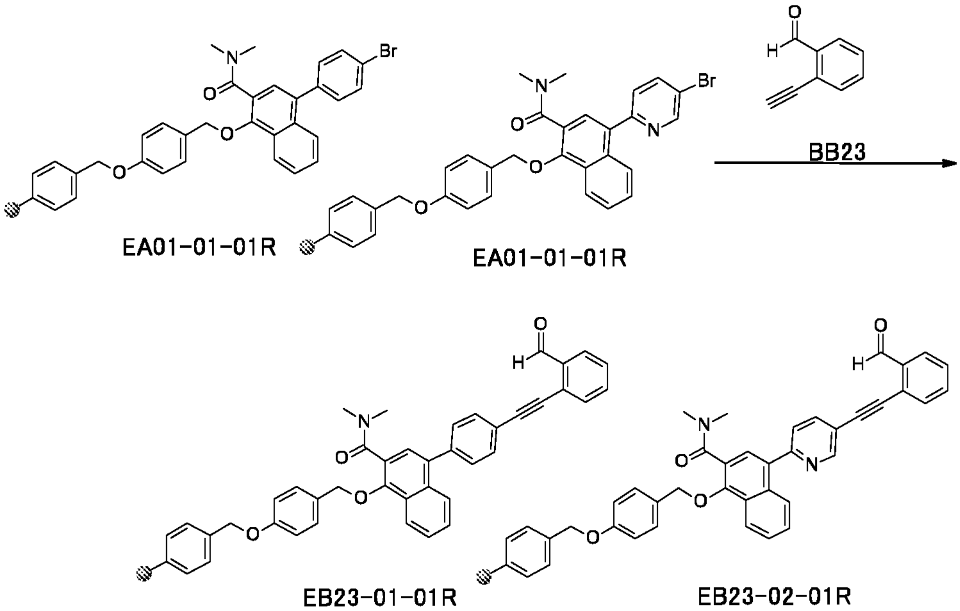


EA01-01-01R EA01-01-01R

EB23-01-01R EB23-02-01R

Under a nitrogen atmosphere, compound EA01-01-01R (48 mg, loading rate: 0.4 mmol/g, 0.019 mmol), compound EA01-02-01R (52 mg, loading rate: 0.37 mmol/g, 0.019 mmol), NMP (875 μL), and diisopropylamine (125 μL) were added to a 1.5 mL glass vial and shaken at room temperature for 1 hour. Under a nitrogen atmosphere, 2- ethynylbenzaldehyde (BB23) (133 mg, 0.760 mmol) and XPhos Pd G4 (9.8 mg, 11 μmol) were added thereto, and the mixture was then shaken at 80°C for 18 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), and three times with DCM (2 mL), and the resin was then dried under reduced pressure to obtain the title compound shown in Table EB23. The structures of the building block used and the compounds to be analyzed, the reaction time, and analysis data are also shown in Table EB23.

[Table 84]

[Table EB23]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|

| | | | | | | | | | | |
|---|---|---|---|---|---|---|---|---|---|---|
| BB23 | mixEB23-01R | EB23-01-01R | mixEB23 | EB23-01 | 4-[4-[2-(2-Formylphenyl)ethynyl]phenyl]-1-hydroxy-N,N-dimethylnaphthalene-2-carboxamide | 17 | 419.2 | 420 | 0.89 | SMD-FA05-1 |
| | | EB23-02-01R | | EB23-02 | 4-[5-[2-(2-Formylphenyl)ethynyl]pyridin-2-yl]-1-hydroxy-N,N-dimethylnaphthalene-2-carboxamide | | 420.1 | 421 | 0.65 | |

Example 3-2-2: Synthesis of mixture mixEB24-01R

[Formula 507]

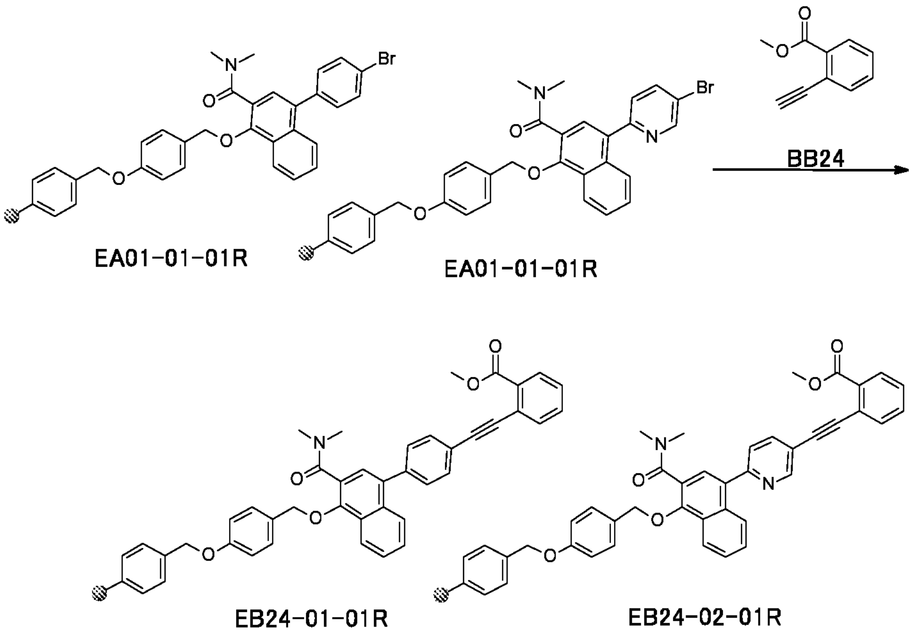


EA01-01-01R EA01-01-01R

EB24-01-01R EB24-02-01R

Under a nitrogen atmosphere, compound EA01-01-01R (48 mg, loading rate: 0.4 mmol/g, 0.019 mmol), EA01-02-01R (52 mg, loading rate: 0.37 mmol/g, 0.019 mmol), NMP (875 μL), and DIA (125 μL) were added to a 2.0 mL glass vial and shaken at room temperature for 1 hour. Under a nitrogen atmosphere, methyl-2-ethynylbenzoate (BB24) (122 mg, 0.760 mmol) and $Pd(PPh_3)_4$ (26 mg, 23 μmol) were added thereto, and the mixture was then shaken at 80°C for 18 hours. The suspension of the reaction solution and the resin was transferred to a syringe with a filter using NMP, repetitively washed three times with NMP (2 mL), three times with a NMP:water (1:1) solution containing NAC (10 mg/mL) (2 mL), three times with water (2 mL), three times with MeOH (2 mL), and three times with DCM (2 mL), and then dried under reduced pressure to obtain the title compound shown in Table EB24. The structures of the building block used and the compounds to be analyzed, the reaction time, and analysis data are also shown in Table EB24.

[Table 85]

[Table EB24]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB24 | mixEB24 -01R | EB24-01 -01R | mixEB24 | EB24-01 | Methyl 2-((4-(3-(dimethylcarbamoyl)-4-hydroxynaphthalen-1-yl)phenyl)ethynyl)benzoate | 18 | 449.2 | 450 | 1.555 | SMD-FA05-1 |
| | | EB24-02 -01R | | EB24-02 | Methyl 2-((6-(3-(dimethylcarbamoyl)-4-hydroxynaphthalen-1-yl)pyridin-3-yl)ethynyl)benzoate | | 450.2 | 451 | 1.376 | |

Example 3-2-3: Synthesis of mixture mixEB25-01R

[Formula 508]

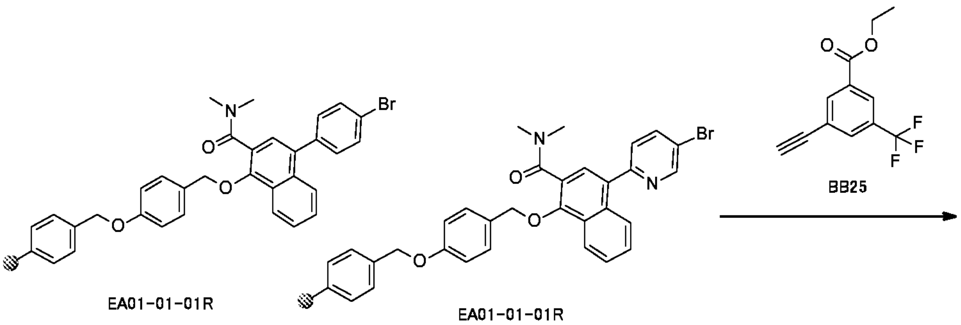


EA01-01-01R EA01-01-01R

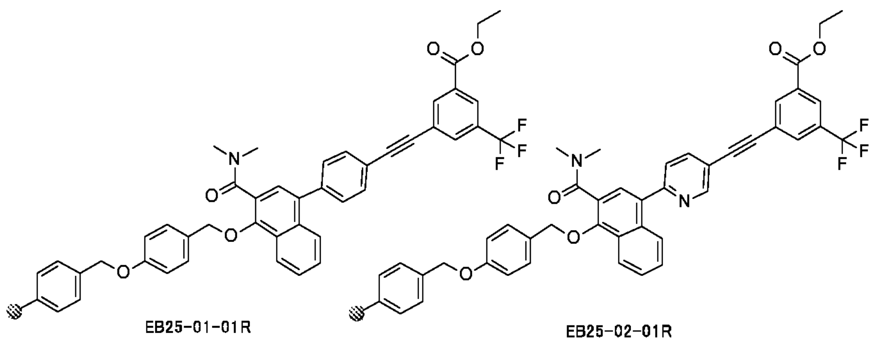

EB25-01-01R EB25-02-01R

Under a nitrogen atmosphere, compound EA01-01-01R (48 mg, loading rate: 0.4 mmol/g, 0.019 mmol), EA01-02-01R (52 mg, loading rate: 0.37 mmol/g, 0.019 mmol), NMP (875 μL), and DIA (125 μL) were added to a 2.0 mL glass vial and shaken at room temperature for 1 hour. Under a nitrogen atmosphere, ethyl 3-ethynyl-5- trifluoromethylbenzoate (BB25) (276 mg, 1.140 mmol) and XPhos Pd G4 (9.8 mg, 11 μmol) were added thereto, and the mixture was then shaken at 80°C for 18 hours. The suspension of the reaction solution and the resin was transferred to a syringe with a filter using NMP, repetitively washed three times with NMP (2 mL), three times with a NMP:water (1:1) solution containing NAC (10 mg/mL) (2 mL), three times with water (2 mL), three times with MeOH (2 mL), and three times with DCM (2 mL), and then dried under reduced pressure to obtain the title compound shown in Table EB25. The structures of the building block used and the compounds to be analyzed, the reaction time, and analysis data are also shown in Table EB25.

[Table 86]

[Table EB25]

| No. of BB used | No. of synthesized mixture | Resin compound No. | No. of mixture after cleavage | Compound No. after cleavage | Compound name | Reaction time (hr) | Exact MS of compound after cleavage | Detected MS m/z [M+H]+ | LC retention time | Analysis method |
|---|---|---|---|---|---|---|---|---|---|---|
| BB25 | mixEB25-01R | EB25-01-01R | mixEB25 | EB25-01 | Ethyl 3-[2-[4-[3-(dimethylcarbamoyl)-4-hydroxynaphthalen-1-yl]phenyl]ethynyl]-5-(trifluoromethyl)benzoate | 18 | 531.2 | 532 | 1.245 | SMD-FA50-1 |
| | | EB25-02-01R | | EB25-02 | Ethyl 3-[2-[6-[3-(dimethylcarbamoyl)-4-hydroxynaphthalen-1-yl]pyridin-3-yl]ethynyl]-5-(trifluoromethyl)benzoate | | 532.2 | 533 | 1.077 | |

Example 3-2-4: Synthesis of mixture mixEB26-01R

[Formula 509]

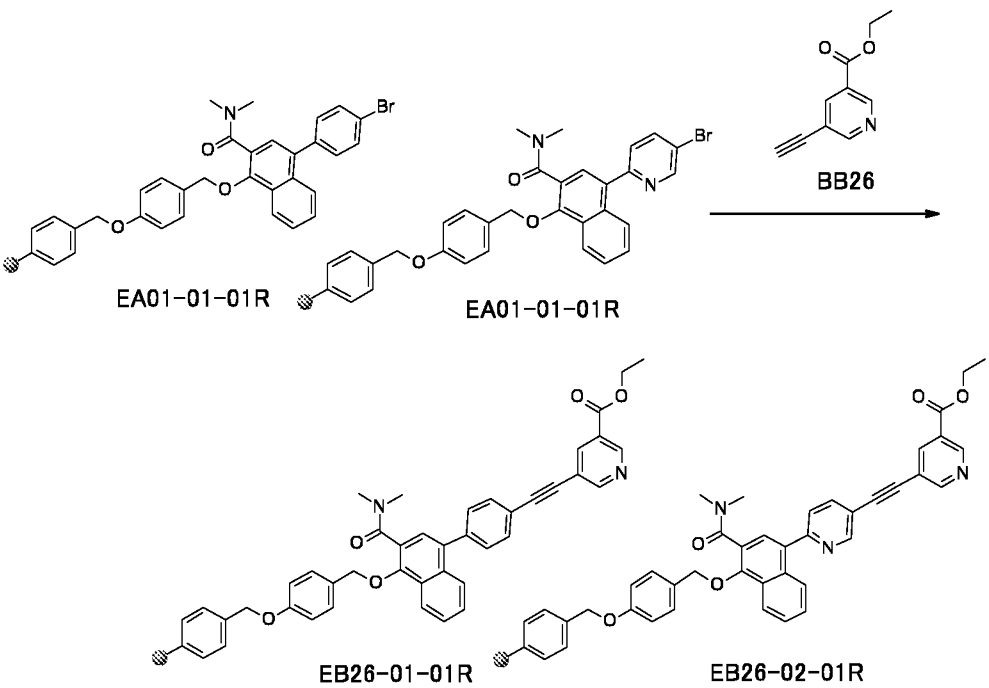

EA01-01-01R

EA01-01-01R

BB26

EB26-01-01R

EB26-02-01R

Under a nitrogen atmosphere, compound EA01-01-01R (48 mg, loading rate: 0.4 mmol/g, 0.019 mmol), EA01-02-01R (52 mg, loading rate: 0.37 mmol/g, 0.019 mmol), NMP (875 μL), and DIA (125 μL) were added to a 2.0 mL glass vial and shaken at room temperature for 1 hour. Under a nitrogen atmosphere, ethyl 5-ethynylnicotinate (BB26) (133 mg, 0.760 mmol) and XPhos Pd G4 (9.8 mg, 11 μmol) were added thereto, and the mixture was then shaken at 80°C for 18 hours. The suspension of the reaction solution and the resin was transferred to a syringe with a filter using NMP, repetitively washed three times with NMP (2 mL), three times with a NMP:water (1:1) solution containing NAC (10 mg/mL) (2 mL), three times with water (2 mL), three times with MeOH (2 mL), and three times with DCM (2 mL), and then dried under reduced pressure to obtain the title compound shown in Table EB26. The structures of the building

block used and the compounds to be analyzed, the reaction time, and analysis data are also shown in Table E26.

[Table 87]

[Table EB26]

| No. of BB used | No. of synthesized mixture | Resin compound No. | No. of mixture after cleavage | Compound No. after cleavage | Compound name | Reaction time (hr) | Exact MS of compound after cleavage | Detected MS m/z $[M+H]^+$ | LC retention time | Analysis method |
|---|---|---|---|---|---|---|---|---|---|---|
| BB26 | mixEB26-01R | EB26-01-01R | mixEB26 | EB26-01 | Ethyl 5-[2-[4-[3-(dimethylcarbamoyl)-4-hydroxynaphthalen-1-yl]phenyl]ethynyl]pyridine-3-carboxylate | 18 | 464.2 | 465 | 1.544 | SMD-FA05-1 |
| | | EB26-02-01R | | EB26-02 | Ethyl 5-[2-[6-[3-(dimethylcarbamoyl)-4-hydroxynaphthalen-1-yl]pyridin-3-yl]ethynyl]pyridine-3-carboxylate | | 465.2 | 466 | 1.343 | |

Example 3-3: Practice of mixture library synthesis to which bond formation reaction is applied through reductive amination reaction of aldehyde

Example 3-3-1: Preparation of mixAG-C01-01R

The mixtures mixEB07-01R, mixEB08-01R, mixEB09-01R, mixEB10-01R, mixEB13-01R, mixEB14-01R, mixEB15-01R, mixEB16-01R, mixEB17-01R, mixEB19-01R, mixEB21-01R, mixEB22-01R, and mixEB23-01R shown in Table AG-C-01 were transferred into a glass vial to prepare mixture mix AG-C01-01R for use in Example 3-3-2. The amount of each mixture used and the numbers of compounds contained therein are shown in Table AG-C-01.

[Table 88]

| [Table AG-C-01] | | | |
|---|---|---|---|
| No. of prepared mixture | No. of mixture used | Amount of mixture used (mg) | No. of contained compound |
| mixAG-C01-01R | mixEB07-01R | 21.9 | EB07-01-01R |
| | | | EB07-02-01R |
| | mixEB08-01R | 21.0 | EB08-01-01R |
| | | | EB08-02-01R |
| | mixEB09-01R | 20.4 | EB08-03-01R |
| | | | EB08-04-01R |
| | mixEB10-01R | 20.8 | EB08-05-01R |
| | | | EB08-06-01R |
| | mixEB13-01R | 20.0 | EB08-11-01R |
| | | | EB08-12-01R |
| | mixEB14-01R | 20.4 | EB08-13-01R |
| | | | EB08-14-01R |
| | mixEB15-01R | 20.8 | EB08-15-01R |
| | | | EB08-16-01R |
| | mixEB16-01R | 20.5 | EB08-17-01R |
| | | | EB08-18-01R |
| | mixEB17-01R | 21.6 | EB08-19-01R |
| | | | EB08-20-01R |
| | mixEB19-01R | 19.9 | EB19-01-01R |
| | | | EB19-02-01R |
| | mixEB21-01R | 20.8 | EB21-01-01R |
| | | | EB21-02-01R |
| | mixEB22-01R | 20.2 | EB22-01-01R |
| | | | EB22-02-01R |
| | mixEB23-01R | 21.0 | EB23-01-01R |
| | | | EB23-02-01R |

Example 3-3-2: Synthesis ofmixEC04-01R

[Formula 510]

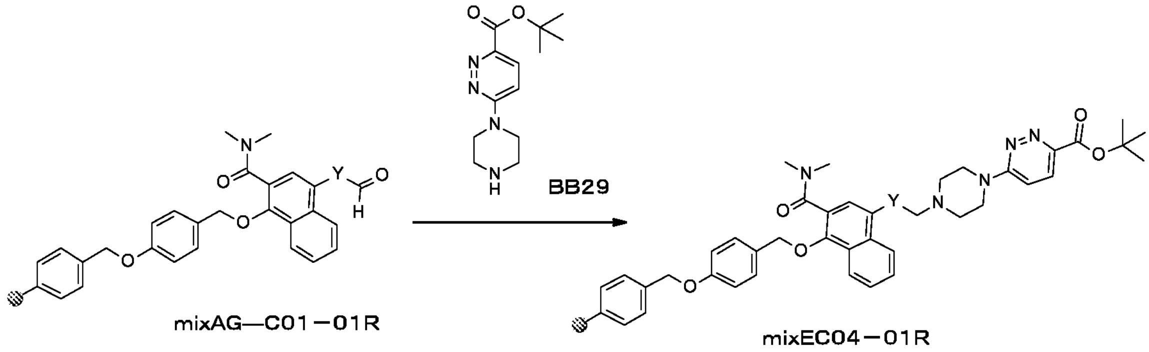


Under a nitrogen atmosphere, solid-phase supported Ar-CHO mixture mixAG-C01- 01R (0.385 mmol/g, 269 mg) and DCE (4.0 mL, 15 v/w) were added to an 8.0 mL screw-cap vial and shaken at room temperature for 1 hour. Under a nitrogen atmosphere, tert-butyl 6- piperazin-1-ylpyridazine-3-carboxylate (BB29) (82 mg) and $Ti(OtBu)_4$ (200 μL) were added thereto, and the mixture was shaken at 60°C for 1 hour. Then, under a nitrogen atmosphere, sodium triacetoxyborohydride (264 mg) and a 2 v/v% solution of AcOH in DCE (1.35 mL) were added thereto, and the mixture was shaken at room temperature for 21 hours. MeOH (1 mL) was added to the reaction solution, and then, the resin suspension was transferred to a syringe with a filter using MeOH (1 mL) (this operation was repeated a total of six times), repetitively washed with MeOH (5 mL), three times with NMP (5 mL), three times with a $NaHCO_3$ solution (0.15 M, NMP:water = 1:5 solution, 5 mL), three times with water (5 mL), three times with MeOH (5 mL), three times with DCM (5 mL), and three times with heptane (5 mL), and then dried under reduced pressure to obtain the title mixture (mixEC04-01R). The structures of compounds contained in the mixture are shown in Table AG-C-02.

The reaction was monitored as follows. Under a nitrogen atmosphere, 10 μL of the resin suspension was sampled, then transferred to a pipette tip with a filter, repetitively washed three times with MeOH (100 μL) and three times with DCM (100 μL), and then dipped in a 10% solution of TFA in DCM containing 0.05 M trimethylbenzene (50 μL) for 5 minutes. The cleaving solution was filtered, and the filtrate was dissolved in NMP (250 μL). The solution (50 μL) was diluted with MeCN (250 μL) to prepare an LC sample. The degree of progression of the reaction was measured by LC-MS analysis.

The analysis data (LCMS and retention times of the products) obtained after a lapse of 21 hours in reaction is shown in Table AG-C-02.

[Table 89]

[Table AG-C-02]

| No. of mixture | Compound No. | No. of mixture after cleavage | Compound No. after cleavage | Compound name after cleavage | Exact MS of compound after cleavage | Detected MS (M+H)+ | Retention time of MS peak | Analysis method |
|---|---|---|---|---|---|---|---|---|
| mixEC04-01R | AG-C-001-01R | mixEC04 | AG-C-001 | tert-Butyl 6-[4-[[4-[4-[3-(dimethylcarbamoyl)-4-hydroxynaphthalen-1-yl]phenyl]-3-methylphenyl]methyl]piperazin-1-yl]pyridazine-3-carboxylate | 657.3 | 658.3 | 8.40 | SMD-FA05-long25min |
| | AG-C-002-01R | | AG-C-002 | tert-Butyl 6-[4-[[4-[6-[3-(dimethylcarbamoyl)-4-hydroxynaphthalen-1-yl]pyridin-3-yl]-3-methylphenyl]methyl]piperazin-1-yl]pyridazine-3-carboxylate | 658.3 | 659.3 | 7.08 | |
| | AG-C-003-01R | | AG-C-003 | tert-Butyl 6-[4-[[2-[4-[3-(dimethylcarbamoyl)-4-hydroxynaphthalen-1-yl]phenyl]phenyl]methyl]piperazin-1-yl]pyridazine-3-carboxylate | 643.3 | 644.3 | 8.27 | |
| | AG-C-004-01R | | AG-C-004 | tert-Butyl 6-[4-[[2-[6-[3-(dimethylcarbamoyl)-4-hydroxynaphthalen-1-yl]pyridin-3-yl]phenyl]methyl]piperazin-1-yl]pyridazine-3-carboxylate | 644.3 | 645.3 | 7.27 | |
| | AG-C-005-01R | | AG-C-005 | tert-Butyl 6-[4-[[4-[4-[3-(dimethylcarbamoyl)-4-hydroxynaphthalen-1-yl]phenyl]naphthalen-1-yl]methyl]piperazin-1-yl]pyridazine-3-carboxylate | 693.3 | 694.3 | 9.39 | |
| | AG-C-006-01R | | AG-C-006 | tert-Butyl 6-[4-[[4-[6-[3-(dimethylcarbamoyl)-4-hydroxynaphthalen-1-yl]pyridin-3-yl]naphthalen-1-yl]methyl]piperazin-1-yl]pyridazine-3-carboxylate | 694.3 | 695.3 | 8.04 | |

| | | | | | | | | |
|---|---|---|---|---|---|---|---|---|
| mixEC04-01R | AG-C-007-01R | mixEC04 | AG-C-007 | tert-Butyl 6-[4-[[4-[4-[3-(dimethylcarbamoyl)-4-hydroxynaphthalen-1-yl]phenyl]thiophen-2-yl]methyl]piperazin-1-yl]pyridazine-3-carboxylate | 649.3 | 650.3 | 8.57 | SMD-FA05-long25min |
| | AG-C-008-01R | | AG-C-008 | tert-Butyl 6-[4-[[4-[6-[3-(dimethylcarbamoyl)-4-hydroxynaphthalen-1-yl]pyridin-3-yl]thiophen-2-yl]methyl]piperazin-1-yl]pyridazine-3-carboxylate | 650.3 | 651.3 | 7.33 | |
| | AG-C-009-01R | | AG-C-009 | tert-Butyl 6-[4-[[4-[4-[3-(dimethylcarbamoyl)-4-hydroxynaphthalen-1-yl]phenyl]-2-ethylphenyl]methyl]piperazin-1-yl]pyridazine-3-carboxylate | 671.3 | 672.3 | 9.97 | |
| | AG-C-010-01R | | AG-C-010 | tert-Butyl 6-[4-[[4-[6-[3-(dimethylcarbamoyl)-4-hydroxynaphthalen-1-yl]pyridin-3-yl]-2-ethylphenyl]methyl]piperazin-1-yl]pyridazine-3-carboxylate | 672.3 | 673.3 | 9.12 | |
| | AG-C-011-01R | | AG-C-011 | tert-Butyl 6-[4-[[4-[4-[3-(dimethylcarbamoyl)-4-hydroxynaphthalen-1-yl]phenyl]-2-methoxyphenyl]methyl]piperazin-1-yl]pyridazine-3-carboxylate | 673.3 | 674.3 | 8.63 | |
| | AG-C-012-01R | | AG-C-012 | tert-Butyl 6-[4-[[4-[6-[3-(dimethylcarbamoyl)-4-hydroxynaphthalen-1-yl]pyridin-3-yl]-2-methoxyphenyl]methyl]piperazin-1-yl]pyridazine-3-carboxylate | 674.3 | 675.3 | 8.39 | |
| | AG-C-013-01R | | AG-C-013 | tert-Butyl 6-[4-[[3-[4-[3-(dimethylcarbamoyl)-4-hydroxynaphthalen-1-yl]phenyl]-5-ethoxyphenyl]methyl]piperazin-1-yl]pyridazine-3-carboxylate | 687.3 | 688.3 | 8.81 | |
| mixEC04-01R | AG-C-014-01R | mixEC04 | AG-C-014 | tert-Butyl 6-[4-[[3-[6-[3-(dimethylcarbamoyl)-4-hydroxynaphthalen-1-yl]pyridin-3-yl]-5-ethoxyphenyl]methyl]piperazin-1-yl]pyridazine-3-carboxylate | 688.3 | 689.3 | 7.98 | SMD-FA05-long25min |
| | AG-C-015-01R | | AG-C-015 | tert-Butyl 6-[4-[[3-[4-[3-(dimethylcarbamoyl)-4-hydroxynaphthalen-1-yl]phenyl]-5-propan-2-yloxyphenyl]methyl]piperazin-1-yl]pyridazine-3-carboxylate | 701.4 | 702.4 | 9.11 | |
| | AG-C-016-01R | | AG-C-016 | tert-Butyl 6-[4-[[3-[6-[3-(dimethylcarbamoyl)-4-hydroxynaphthalen-1-yl]pyridin-3-yl]-5-propan-2-yloxyphenyl]methyl]piperazin-1-yl]pyridazine-3-carboxylate | 702.4 | 703.4 | 7.92 | |
| | AG-C-017-01R | | AG-C-017 | tert-Butyl 6-[4-[[4-[4-[3-(dimethylcarbamoyl)-4-hydroxynaphthalen-1-yl]phenyl]-2,6-dimethoxyphenyl]methyl]piperazin-1-yl]pyridazine-3-carboxylate | 703.3 | 704.3 | 8.55 | |

| | | | | | | | | |
|---|---|---|---|---|---|---|---|---|
| | AG-C-018-01R | | AG-C-018 | tert-Butyl 6-[4-[[4-[6-[3-(dimethylcarbamoyl)-4-hydroxynaphthalen-1-yl]pyridin-3-yl]-2,6-dimethoxyphenyl]methyl]piperazin-1-yl]pyridazine-3-carboxylate | 704.3 | 705.3 | 7.58 | |
| | AG-C-019-01R | | AG-C-019 | tert-Butyl 6-[4-[[4-[4-[3-(dimethylcarbamoyl)-4-hydroxynaphthalen-1-yl]phenyl]-3-fluorophenyl]methyl]piperazin-1-yl]pyridazine-3-carboxylate | 661.3 | 662.3 | 8.53 | |
| | AG-C-020-01R | | AG-C-020 | tert-Butyl 6-[4-[[4-[6-[3-(dimethylcarbamoyl)-4-hydroxynaphthalen-1-yl]pyridin-3-yl]-3-fluorophenyl]methyl]piperazin-1-yl]pyridazine-3-carboxylate | 662.3 | 663.3 | 7.42 | |
| mixEC04-01R | AG-C-021-01R | mixEC04 | AG-C-021 | tert-Butyl 6-[4-[[3-[4-[3-(dimethylcarbamoyl)-4-hydroxynaphthalen-1-yl]phenyl]-5-(trifluoromethyl)phenyl]methyl]piperazin-1-yl]pyridazine-3-carboxylate | 711.3 | 712.3 | 9.81 | SMD-FA05-long25min |
| | AG-C-022-01R | | AG-C-022 | tert-Butyl 6-[4-[[3-[6-[3-(dimethylcarbamoyl)-4-hydroxynaphthalen-1-yl]pyridin-3-yl]-5-(trifluoromethyl)phenyl]methyl]piperazin-1-yl]pyridazine-3-carboxylate | 712.3 | 713.3 | 8.43 | |
| | AG-C-023-01R | | AG-C-023 | tert-Butyl 6-[4-[[3-[4-[3-(dimethylcarbamoyl)-4-hydroxynaphthalen-1-yl]phenyl]-5-(trifluoromethoxy)phenyl]methyl]piperazin-1-yl]pyridazine-3-carboxylate | 727.3 | 728.3 | 9.96 | |
| | AG-C-024-01R | | AG-C-024 | tert-Butyl 6-[4-[[3-[6-[3-(dimethylcarbamoyl)-4-hydroxynaphthalen-1-yl]pyridin-3-yl]-5-(trifluoromethoxy)phenyl]methyl]piperazin-1-yl]pyridazine-3-carboxylate | 728.3 | 729.3 | 8.72 | |
| | AG-C-025-01R | | AG-C-025 | tert-Butyl 6-[4-[[2-[2-[4-[3-(dimethylcarbamoyl)-4-hydroxynaphthalen-1-yl]phenyl]ethynyl]phenyl]methyl]piperazin-1-yl]pyridazine-3-carboxylate | 667.3 | 668.3 | 9.30 | |
| | AG-C-026-01R | | AG-C-026 | tert-Butyl 6-[4-[[2-[2-[6-[3-(dimethylcarbamoyl)-4-hydroxynaphthalen-1-yl]pyridin-3-yl]ethynyl]phenyl]methyl]piperazin-1-yl]pyridazine-3-carboxylate | 668.3 | 669.3 | 7.94 | |

Example 3-3-3: Preparation ofmixAG-C03-01R

Mixtures mixEB07-0lR, mixEB08-01R, mixEB09-01R, mixEB10-01R, mixEB13- 01R, mixEB14-01R, mixEB15-01R, mixEB16-01R, mixEB17-01R, mixEB19-01R, mixEB21-01R, mixEB22-01R, and mixEB23-01R shown in Table AG-C-03 were transferred into a glass vial to prepare mixture mixAG-C03-01R for use in Example 3-3-4. The amount of each mixture used and the numbers of compounds contained therein are shown in Table AG-C-03.

[Table 90]

| [Table AG-C-03] | | | |
|---|---|---|---|
| No. of prepared mixture | No. of mixture used | Amount of mixture used (mg) | No. of contained compound |
| mixAG-C03-01R | mixEB07-01R | 20.8 | EB07-01-01R |
| | | | EB07-02-01R |
| | mixEB08-01R | 21.1 | EB08-01-01R |
| | | | EB08-02-01R |
| | mixEB09-01R | 20.0 | EB08-03-01R |
| | | | EB08-04-01R |
| | mixEB10-01R | 19.9 | EB08-05-01R |
| | | | EB08-06-01R |
| | mixEB13-01R | 20.2 | EB08-11-01R |
| | | | EB08-12-01R |
| | mixEB14-01R | 20.8 | EB08-13-01R |
| | | | EB08-14-01R |

[Table 91]

| | | | |
|---|---|---|---|
| mixAG-C03-01R | mixEB15-01R | 20.3 | EB08-15-01R |
| | | | EB08-16-01R |
| | mixEB16-01R | 20.0 | EB08-17-01R |
| | | | EB08-18-01R |
| | mixEB17-01R | 20.3 | EB08-19-01R |
| | | | EB08-20-01R |
| | mixEB19-01R | 20.2 | EB19-01-01R |
| | | | EB19-02-01R |
| | mixEB21-01R | 20.7 | EB21-01-01R |
| | | | EB21-02-01R |
| | mixEB22-01R | 19.8 | EB22-01-01R |
| | | | EB22-02-01R |
| | mixEB23-01R | 21.3 | EB23-01-01R |
| | | | EB23-02-01R |

Example 3-3-4: Synthesis of mixEC05-01R

[Formula 511]

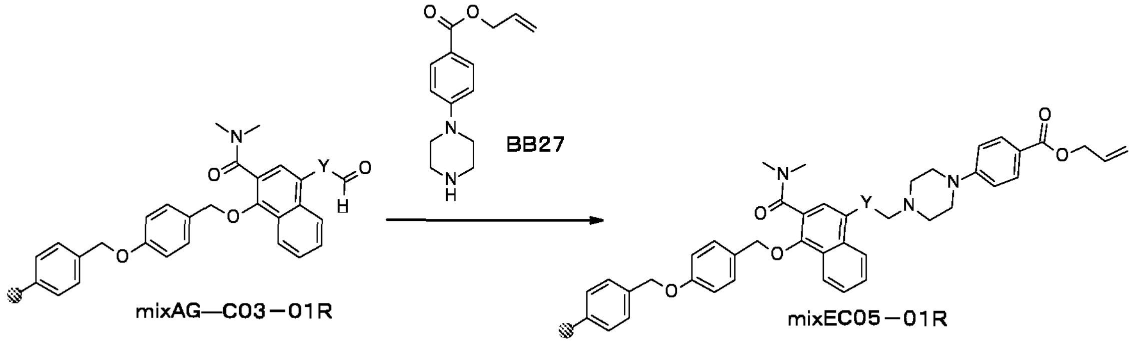

Under a nitrogen atmosphere, solid-phase supported Ar-CHO mixture mixAG-C03- 01R (0.385 mmol/g, 265 mg) and DCE (3.98 mL, 15 v/w) were added to an 8.0 mL screw- cap vial and shaken at room temperature for 1 hour. Under a nitrogen atmosphere, prop-2- enyl 6-piperazin-1-ylpyridazine-3-carboxylate (BB27) (76 mg) and $Ti(OtBu)_4$ (197 μL) were added thereto, and the mixture was shaken at 60°C for 1 hour. Then, under a nitrogen atmosphere, sodium triacetoxyborohydride (260 mg) and a 2 v/v% solution of AcOH in DCE (1.33 mL) were added thereto, and the mixture was shaken at 50°C for 3 hours. After cooling to room temperature, the resin suspension was transferred to a syringe with a filter using MeOH (1 mL) (this operation was repeated a total of six times), repetitively washed with MeOH (5 mL), three times with NMP (5 mL), three times with a NaHCOa solution (0.15 M, NMP:water = 1:5 solution, 5 mL), three times with water (5 mL), three times with MeOH (5 mL), three times with DCM (5 mL), and three times with heptane (5 mL), and then dried under reduced pressure to obtain the title mixture (mixEC05-01R). The structures of compounds contained in the obtained mixture are shown in Table AG-C-04.

The reaction was monitored as follows. Under a nitrogen atmosphere, 10 μL of the resin suspension was sampled, then transferred to a pipette tip with a filter, repetitively washed three times with MeOH (100 μL) and three times with DCM (100 μL), and then dipped in a 10% solution of TFA in DCM containing 0.05 M trimethylbenzene (50 μL) for 5 minutes. The cleaving solution was filtered, and the filtrate was dissolved in NMP (250 μL). The solution (50 μL) was diluted with MeCN (250 μL) to prepare an LC sample. The degree of progression of the reaction was measured by LC-MS analysis.

The analysis data (LCMS and retention times of the products) obtained after a lapse of 21 hours in reaction is shown in Table AG-C-04.

[Table 92]

[Table AG-C-04]

| No. of mixture | Compound No. | No. of mixture after cleavage | Compound No. after cleavage | Compound name after cleavage | Exact MS of compound after cleavage | Detected MS | Retention time of MS peak | Analysis method |
|---|---|---|---|---|---|---|---|---|

| | | | | | | | | |
|---|---|---|---|---|---|---|---|---|
| mixEC05-01R | AG-C-027-01R | mixEC05-01 | AG-C-027 | Prop-2-enyl 4-[4-[[4-[4-[3-(dimethylcarbamoyl)-4-hydroxynaphthalen-1-yl]phenyl]-3-methylphenyl]methyl]piperazin-1-yl]benzoate | 639.3 | 640.3 | 9.12 | SMD-FA05-long25min |
| | AG-C-028-01R | | AG-C-028 | Prop-2-enyl 4-[4-[[4-[6-[3-(dimethylcarbamoyl)-4-hydroxynaphthalen-1-yl]pyridin-3-yl]-3-methylphenyl]methyl]piperazin-1-yl]benzoate | 640.3 | 641.3 | 7.75 | |
| | AG-C-029-01R | | AG-C-029 | Prop-2-enyl 4-[4-[[2-[4-[3-(dimethylcarbamoyl)-4-hydroxynaphthalen-1-yl]phenyl]phenyl]methyl]piperazin-1-yl]benzoate | 625.3 | 626.3 | 9.04 | |
| | AG-C-030-01R | | AG-C-030 | Prop-2-enyl 4-[4-[[2-[6-[3-(dimethylcarbamoyl)-4-hydroxynaphthalen-1-yl]pyridin-3-yl]phenyl]methyl]piperazin-1-yl]benzoate | 626.3 | 627.3 | 8.06 | |
| | AG-C-031-01R | | AG-C-031 | Prop-2-enyl 4-[4-[[4-[4-[3-(dimethylcarbamoyl)-4-hydroxynaphthalen-1-yl]phenyl]naphthalen-1-yl]methyl]piperazin-1-yl]benzoate | 675.3 | 676.3 | 10.35 | |
| mixEC05-01R | AG-C-032-01R | mixEC05-01 | AG-C-032 | Prop-2-enyl 4-[4-[[4-[6-[3-(dimethylcarbamoyl)-4-hydroxynaphthalen-1-yl]pyridin-3-yl]naphthalen-1-yl]methyl]piperazin-1-yl]benzoate | 676.3 | 677.3 | 8.83 | SMD-FA05-long25min |
| | AG-C-033-01R | | AG-C-033 | Prop-2-enyl 4-[4-[[4-[4-[3-(dimethylcarbamoyl)-4-hydroxynaphthalen-1-yl]phenyl]thiophen-2-yl]methyl]piperazin-1-yl]benzoate | 631.3 | 632.3 | 9.31 | |
| | AG-C-034-01R | | AG-C-034 | Prop-2-enyl 4-[4-[[4-[6-[3-(dimethylcarbamoyl)-4-hydroxynaphthalen-1-yl]pyridin-3-yl]thiophen-2-yl]methyl]piperazin-1-yl]benzoate | 632.2 | 633.2 | 7.77 | |
| | AG-C-035-01R | | AG-C-035 | Prop-2-enyl 4-[4-[[4-[4-[3-(dimethylcarbamoyl)-4-hydroxynaphthalen-1-yl]phenyl]-2-ethylphenyl]methyl]piperazin-1-yl]benzoate | 653.3 | 654.3 | 10.28 | |
| | AG-C-036-01R | | AG-C-036 | Prop-2-enyl 4-[4-[[4-[6-[3-(dimethylcarbamoyl)-4-hydroxynaphthalen-1-yl]pyridin-3-yl]-2-ethylphenyl]methyl]piperazin-1-yl]benzoate | 654.3 | 655.3 | 7.77 | |
| | AG-C-037-01R | | AG-C-037 | Prop-2-enyl 4-[4-[[4-[4-[3-(dimethylcarbamoyl)-4-hydroxynaphthalen-1-yl]phenyl]-2-methoxyphenyl]methyl]piperazin-1-yl]benzoate | 655.3 | 656.3 | 8.99 | |
| mixEC05-01R | AG-C-038-01R | mixEC05-01 | AG-C-038 | Prop-2-enyl 4-[4-[[4-[6-[3-(dimethylcarbamoyl)-4-hydroxynaphthalen-1-yl]pyridin-3-yl]-2-methoxyphenyl]methyl]piperazin-1-yl]benzoate | 656.3 | 657.3 | 7.76 | SMD-FA05-long25min |
| | AG-C-039-01R | | AG-C-039 | Prop-2-enyl 4-[4-[[3-[4-[3-(dimethylcarbamoyl)-4-hydroxynaphthalen-1-yl]phenyl]-5-ethoxyphenyl]methyl]piperazin-1-yl]benzoate | 669.3 | 670.3 | 9.48 | |

| | | | | | | | | |
|---|---|---|---|---|---|---|---|---|
| | AG-C-040-01R | | AG-C-040 | Prop-2-enyl 4-[4-[[3-[6-[3-(dimethylcarbamoyl)-4-hydroxynaphthalen-1-yl]pyridin-3-yl]-5-ethoxyphenyl]methyl]piperazin-1-yl]benzoate | 670.3 | 671.3 | 8.29 | |
| | AG-C-041-01R | | AG-C-041 | Prop-2-enyl 4-[4-[[3-[4-[3-(dimethylcarbamoyl)-4-hydroxynaphthalen-1-yl]phenyl]-5-propan-2-yloxyphenyl]methyl]piperazin-1-yl]benzoate | 683.3 | 684.3 | 9.79 | |
| | AG-C-042-01R | | AG-C-042 | Prop-2-enyl 4-[4-[[3-[6-[3-(dimethylcarbamoyl)-4-hydroxynaphthalen-1-yl]pyridin-3-yl]-5-propan-2-yloxyphenyl]methyl]piperazin-1-yl]benzoate | 684.3 | 685.3 | 8.65 | |
| | AG-C-043-01R | | AG-C-043 | Prop-2-enyl 4-[4-[[4-[4-[3-(dimethylcarbamoyl)-4-hydroxynaphthalen-1-yl]phenyl]-2,6-dimethoxyphenyl]methyl]piperazin-1-yl]benzoate | 685.3 | 686.3 | 9.23 | |
| mixEC05-01R | AG-C-044-01R | mixEC05-01 | AG-C-044 | Prop-2-enyl 4-[4-[[4-[6-[3-(dimethylcarbamoyl)-4-hydroxynaphthalen-1-yl]pyridin-3-yl]-2,6-dimethoxyphenyl]methyl]piperazin-1-yl]benzoate | 686.3 | 687.3 | 8.11 | SMD-FA05-long25min |
| | AG-C-045-01R | | AG-C-045 | Prop-2-enyl 4-[4-[[4-[4-[3-(dimethylcarbamoyl)-4-hydroxynaphthalen-1-yl]phenyl]-3-fluorophenyl]methyl]piperazin-1-yl]benzoate | 643.3 | 644.3 | 9.34 | |
| | AG-C-046-01R | | AG-C-046 | Prop-2-enyl 4-[4-[[4-[6-[3-(dimethylcarbamoyl)-4-hydroxynaphthalen-1-yl]pyridin-3-yl]-3-fluorophenyl]methyl]piperazin-1-yl]benzoate | 644.3 | 645.3 | 9.34 | |
| | AG-C-047-01R | | AG-C-047 | Prop-2-enyl 4-[4-[[3-[4-[3-(dimethylcarbamoyl)-4-hydroxynaphthalen-1-yl]phenyl]-5-(trifluoromethyl)phenyl]methyl]piperazin-1-yl]benzoate | 693.3 | 694.3 | 10.71 | |
| | AG-C-048-01R | | AG-C-048 | Prop-2-enyl 4-[4-[[3-[6-[3-(dimethylcarbamoyl)-4-hydroxynaphthalen-1-yl]pyridin-3-yl]-5-(trifluoromethyl)phenyl]methyl]piperazin-1-yl]benzoate | 694.3 | 695.3 | 9.28 | |
| | AG-C-049-01R | | AG-C-049 | Prop-2-enyl 4-[4-[[3-[4-[3-(dimethylcarbamoyl)-4-hydroxynaphthalen-1-yl]phenyl]-5-(trifluoromethoxy)phenyl]methyl]piperazin-1-yl]benzoate | 709.3 | 710.3 | 10.86 | |
| mixEC05-01R | AG-C-050-01R | mixEC05-01 | AG-C-050 | Prop-2-enyl 4-[4-[[3-[6-[3-(dimethylcarbamoyl)-4-hydroxynaphthalen-1-yl]pyridin-3-yl]-5-(trifluoromethoxy)phenyl]methyl]piperazin-1-yl]benzoate | 710.3 | 711.3 | 9.45 | SMD-FA05-long25min |
| | AG-C-051-01R | | AG-C-051 | Prop-2-enyl 4-[4-[[2-[2-[4-[3-(dimethylcarbamoyl)-4-hydroxynaphthalen-1-yl]phenyl]ethynyl]phenyl]methyl]piperazin-1-yl]benzoate | 649.3 | 650.3 | 9.65 | |

| | | | | | | | | |
|---|---|---|---|---|---|---|---|---|
| | AG-C-052-01R | | AG-C-052 | Prop-2-enyl 4-[4-[[2-[2-[6-[3-(dimethylcarbamoyl)-4-hydroxynaphthalen-1-yl]pyridin-3-yl]ethynyl]phenyl]methyl]piperazin-1-yl]benzoate | 650. | 651.3 | 8.67 | |

Example 3-4: Practice of mixture library synthesis to which bond formation reaction is applied through reductive amination reaction of ketone

Example 3-4-1: Synthesis of mixture mixEC06-01R

[Formula 512]

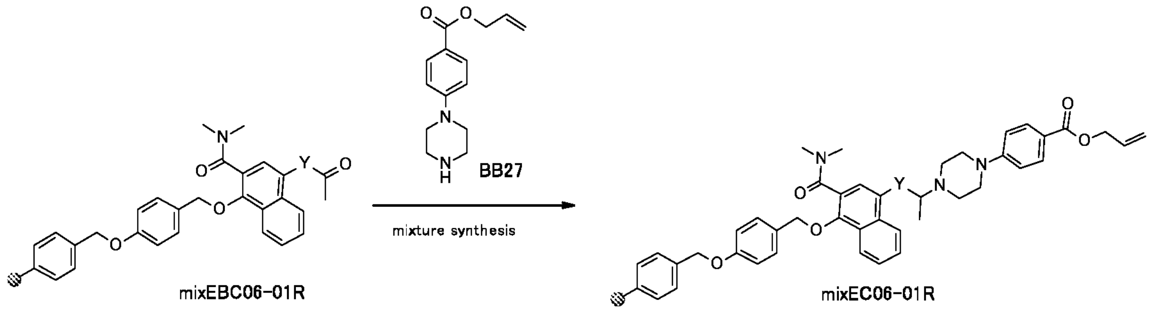


Under a nitrogen atmosphere, mixture mixEBC06-01R (mixture of mixEB12-01R and mixEB18-01R, a total of 42 mg, loading rate: 0.38 mmol/g, 0.016 mmol) shown in Table EBC06 and anisole (1.05 mL) were added to a 3 mL glass vial and shaken at room temperature for 1 hour. Under a nitrogen atmosphere, allyl 4-(piperazin-1-yl)benzoate (BB27, 47.0 mg, 0.192 mmol) and $Ti(OtBu)_4$ (123 μL, 0.319 mmol) were added thereto, and the mixture was shaken at 140°C for 22 hours. The reaction solution was cooled to room temperature. Then, under a nitrogen atmosphere, sodium triacetoxyborohydride (101 mg, 0.479 mmol) was added thereto, and the mixture was shaken at 140°C for 22.5 hours. The reaction container was cooled to room temperature, and MeOH (4 mL) was added to the reaction solution. The obtained reaction solution and the resin suspension were transferred to a syringe with a filter using MeOH (4 mL) (MeOH was used three times for transfer), repetitively washed three times with NMP (4 mL), three times with a Rochelle salt solution (0.05 M, NMP:water = 1:1 solution, 4 mL), three times with water (4 mL), three times with a half-saturated $NaHCO_3$ solution (4 mL), three times with water (4 mL), three times with MeOH (4 mL), three times with DCM (4 mL), and three times with heptane (4 mL), and then dried under reduced pressure to obtain the title mixture mixEC06-01R shown in Table EC06.

[Table 93]

[Table EBC06]

| No. of prepared mixture | No. of mixture used | No. of contained compound | No. of mixture after cleavage | No. of contained compound after cleavage | Compound name |
|---|---|---|---|---|---|
| mixEBC06-01R | mixEB12-01R | EB08-09-01R | mixEB12 | EB08-09 | 4-[4-(4-Acetyl-3-fluorophenyl)phenyl]-1-hydroxy-N,N-dimethylnaphthalene-2-carboxamide |
| | | EB08-10-01R | | EB08-10 | 4-[5-(4-Acetyl-3-fluorophenyl)pyridin-2-yl]-1-hydroxy-N,N-dimethylnaphthalene-2-carboxamide |
| | mixEB18-01R | EB08-21-01R | mixEB18 | EB08-21 | 4-[4-(4-Acetylphenyl)phenyl]-1-hydroxy-N,N-dimethylnaphthalene-2-carboxamide |
| | | EB08-22-01R | | EB08-22 | 4-[5-(4-Acetylphenyl)pyridin-2-yl]-1-hydroxy-N,N-dimethylnaphthalene-2-carboxamide |

[Table 94]

[Table EC06]

| No. of mixture | No. of contained compound | No. of contained compound after cleavage. | Compound name | Exact MS | Detected MS :m/z [M+H]+ | LC retention time | Analysis method |
|---|---|---|---|---|---|---|---|
| mixEC06-01R | EC06-01-01R | EC06-01 | Prop-2-enyl 4-[4-[1-[4-[4-[3-(dimethylcarbamoyl)-4-hydroxynaphthalen-1-yl]phenyl]-2-fluorophenyl]ethyl]piperazin-1-yl]benzoate | 657.3 | 658 | 1.092 | SMD-FA05-1 |
| | EC06-02-01R | EC06-02 | Prop-2-enyl 4-[4-[1-[4-[6-[3-(dimethylcarbamoyl)-4-hydroxynaphthalen-1-yl]pyridin-3-yl]-2-fluorophenyl]ethyl]piperazin-1-yl]benzoate | 658.3 | 659 | 0.961 | |
| | EC06-03-01R | EC06-03 | Prop-2-enyl 4-[4-[1-[4-[4-[3-(dimethylcarbamoyl)-4-hydroxynaphthalen-1-yl]phenyl]phenyl]ethyl]piperazin-1-yl]benzoate | 639.3 | 640 | 1.033 | |
| | EC06-04-01R | EC06-04 | Prop-2-enyl 4-[4-[1-[4-[6-[3-(dimethylcarbamoyl)-4-hydroxynaphthalen-1-yl]pyridin-3-yl]phenyl]ethyl]piperazin-1-yl]benzoate | 640.3 | 641 | 0.905 | |

Example 3-5: Practice of mixture library synthesis to which bond formation reaction is applied through amide coupling reaction

The deprotection of ester was carried out for the obtained ester compounds to synthesize carboxylic acid compounds.

Example 3-5-1: Synthesis of mixEC01-01R

[Formula 513]

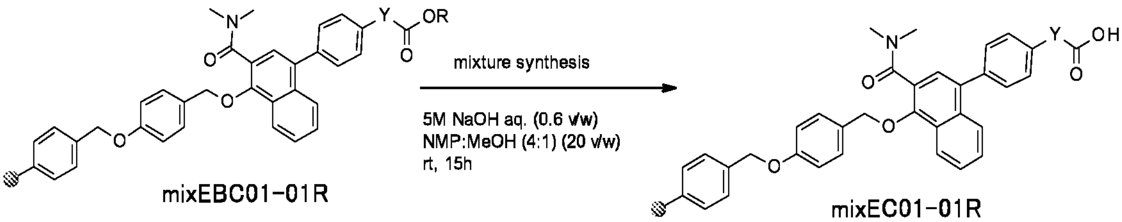


mixEBC01-01R

mixEC01-01R

NMP (3.52 mL) and methanol (0.88 mL) were added to mixEBC01-01R (a mixture of 22 compounds, a total of 226 mg, loading rate: 0.38 mmol/g, 0.0859 mmol) shown in Table EBC01 in a 10 mL glass vial, and the mixture was shaken at room temperature for 1 hour. An aqueous sodium hydroxide solution (5 M, 132 μL, 0.660 mmol) was added thereto, and the mixture was shaken at room temperature for 15 hours. The suspension of the reaction solution and the resin was transferred to a syringe with a filter using NMP and repetitively washed three times with NMP (4 mL), three times with water (4 mL), three times with NMP (4 mL), three times with a solution of HOAt in NMP (0.2 M, 4 mL), three times with NMP (4 mL), three times with MeOH (4 mL), and three times with DCM (3 mL), and the resin was then dried under reduced pressure to obtain the title mixture (mixEC01-01R) shown in Table EC01.

[Table 95]

[Table EBC01]

| No. of prepared mixture | No. of mixture used | Amount of mixture used (mg) | No. of contained compound | No. of mixture after cleavage | No. of contained compound after cleavage | Compound name |
|---|---|---|---|---|---|---|
| mixEBC01-01R | mixEB01-01R | 21.3 | + No. of prepared mixture | mixEB01 | EB01-01 | Methyl 4-[4-[3-(dimethylcarbamoyl)-4-hydroxynaphthalen-1-yl]phenyl]-3-methylbenzoate |
| | | | EB01-02-01R | | EB01-02 | Methyl 4-[6-[3-(dimethylcarbamoyl)-4-hydroxynaphthalen-1-yl]pyridin-3-yl]-3-methylbenzoate |
| | mixEB02-01R | 20.3 | EB02-01-01R | mixEB02 | EB02-01 | Ethyl 3-[4-[3-(dimethylcarbamoyl)-4-hydroxynaphthalen-1-yl]phenyl]-5-(trifluoromethyl)benzoate |
| | | | EB02-02-01R | | EB02-02 | Ethyl 3-[6-[3-(dimethylcarbamoyl)-4-hydroxynaphthalen-1-yl]pyridin-3-yl]-5-(trifluoromethyl)benzoate |
| | mixEB03-01R | 20.5 | EB02-03-01R | mixEB03 | EB02-03 | Methyl 3-tert-butyl-5-[4-[3-(dimethylcarbamoyl)-4-hydroxynaphthalen-1-yl]phenyl]benzoate |

| | | | | | | |
|---|---|---|---|---|---|---|
| | | | EB02-04-01R | | EB02-04 | Methyl 3-tert-butyl-5-[6-[3-(dimethylcarbamoyl)-4-hydroxynaphthalen-1-yl]pyridin-3-yl]benzoate |
| | mixEB04-01R | 20.3 | EB02-05-01R | mixEB04 | EB02-05 | Ethyl 3-[2-[4-[3-(dimethylcarbamoyl)-4-hydroxynaphthalen-1-yl]phenyl]phenyl]propanoate |
| | | | EB02-06-01R | | EB02-06 | Ethyl 3-[2-[6-[3-(dimethylcarbamoyl)-4-hydroxynaphthalen-1-yl]pyridin-3-yl]phenyl]propanoate |
| mixEBC01-01R | mixEB05-01R | 20.9 | EB02-07-01R | mixEB05 | EB02-07 | Methyl 4-[4-[3-(dimethylcarbamoyl)-4-hydroxynaphthalen-1-yl]phenyl]naphthalene-1-carboxylate |
| | | | EB02-08-01R | | EB02-08 | Methyl 4-[6-[3-(dimethylcarbamoyl)-4-hydroxynaphthalen-1-yl]pyridin-3-yl]naphthalene-1-carboxylate |
| | mixEB06-01R | 20.7 | EB02-09-01R | mixEB06 | EB02-09 | Ethyl 3-[4-[4-[3-(dimethylcarbamoyl)-4-hydroxynaphthalen-1-yl]phenyl]pyrazol-1-yl]-5-(trifluoromethyl)benzoate |
| | | | EB02-10-01R | | EB02-10 | Ethyl 3-[4-[6-[3-(dimethylcarbamoyl)-4-hydroxynaphthalen-1-yl]pyridin-3-yl]pyrazol-1-yl]-5-(trifluoromethyl)benzoate |
| | mixEB11-01R | 20.5 | EB08-07-01R | mixEB11 | EB08-07 | Methyl 4-[4-[3-(dimethylcarbamoyl)-4-hydroxynaphthalen-1-yl]phenyl]thiophene-2-carboxylate |
| | | | EB08-08-01R | | EB08-08 | Methyl 4-[6-[3-(dimethylcarbamoyl)-4-hydroxynaphthalen-1-yl]pyridin-3-yl]thiophene-2-carboxylate |
| | mixEB20-01R | 20.3 | EB20-01-01R | mixEB20 | EB20-01 | Methyl 4-[4-[3-(dimethylcarbamoyl)-4-hydroxynaphthalen-1-yl]phenyl]-3-fluorobenzoate |
| | | | EB20-02-01R | | EB20-02 | Methyl 4-[6-[3-(dimethylcarbamoyl)-4-hydroxynaphthalen-1-yl]pyridin-3-yl]-3-fluorobenzoate |
| mixEBC01-01R | mixEB24-01R | 20.0 | EB24-01-01R | mixEB24 | EB24-01 | Methyl 2-((4-(3-(dimethylcarbamoyl)-4-hydroxynaphthalen-1-yl)phenyl)ethynyl)benzoate |
| | | | EB24-02-01R | | EB24-02 | Methyl 2-((6-(3-(dimethylcarbamoyl)-4-hydroxynaphthalen-1-yl)pyridin-3-yl)ethynyl)benzoate |
| | mixEB25-01R | 20.9 | EB25-01-01R | mixEB25 | EB25-01 | Ethyl 3-[2-[4-[3-(dimethylcarbamoyl)-4-hydroxynaphthalen-1-yl]phenyl]ethynyl]-5-(trifluoromethyl)benzoate |
| | | | EB25-02-01R | | EB25-02 | Ethyl 3-[2-[6-[3-(dimethylcarbamoyl)-4-hydroxynaphthalen-1-yl]pyridin-3-yl]ethynyl]-5-(trifluoromethyl)benzoate |
| | mixEB26-01R | 20.6 | EB25-03-01R | mixEB26 | EB25-03 | Ethyl 5-[2-[4-[3-(dimethylcarbamoyl)-4-hydroxynaphthalen-1-yl]phenyl]ethynyl]pyridine-3-carboxylate |
| | | | EB25-04-01R | | EB25-04 | Ethyl 5-[2-[6-[3-(dimethylcarbamoyl)-4-hydroxynaphthalen-1-yl]pyridin-3-yl]ethynyl]pyridine-3-carboxylate |

[Table 96]

[Table EC01]

| No. of mixture | No. of contained compound | Compound No .after cleavage | Compound name | Exact MS | Detected MS: m/z [M+H]+ | LC retention time | Analysis method |
|---|---|---|---|---|---|---|---|
| mixEC01-01R | EC01-01-01R | EC01-01 | 4-[4-[3-(Dimethylcarbamoyl)-4-hydroxynaphthalen-1-yl]phenyl]thiophene-2-carboxylic acid | 417.1 | 418.1 | 1.16 | SMD-FA05-1 |
| | EC01-02-01R | EC01-02 | 4-[6-[3-(Dimethylcarbamoyl)-4-hydroxynaphthalen-1-yl]pyridin-3-yl]thiophene-2-carboxylic acid | 418.1 | 419.1 | 0.89 | |
| | EC01-03-01R | EC01-03 | 4-[4-[3-(Dimethylcarbamoyl)-4-hydroxynaphthalen-1-yl]phenyl]-3-methylbenzoic acid | 425.2 | 426.2 | 1.23 | |
| | EC01-04-01R | EC01-04 | 4-[6-[3-(Dimethylcarbamoyl)-4-hydroxynaphthalen-1-yl]pyridin-3-yl]-3-methylbenzoic acid | 426.2 | 427.2 | 0.95 | |
| | EC01-05-01R | EC01-05 | 4-[4-[3-(Dimethylcarbamoyl)-4-hydroxynaphthalen-1-yl]phenyl]-3-fluorobenzoic acid | 429.1 | 430.1 | 1.21 | |
| | EC01-06-01R | EC01-06 | 4-[6-[3-(Dimethylcarbamoyl)-4-hydroxynaphthalen-1-yl]pyridin-3-yl]-3-fluorobenzoic acid | 430.1 | 431.1 | 0.99 | |
| | EC01-07-01R | EC01-07 | 2-[2-[4-[3-(Dimethylcarbamoyl)-4-hydroxynaphthalen-1-yl]phenyl]ethynyl]benzoic acid | 435.1 | 436.1 | 1.22 | |
| mixEC01-01R | EC01-08-01R | EC01-08 | 2-[2-[6-[3-(Dimethylcarbamoyl)-4-hydroxynaphthalen-1-yl]pyridin-3-yl]ethynyl]benzoic acid | 436.1 | 437.1, 437.1 | 0.86, 1.15 | SMD-FA05-1 |
| | EC01-09-01R | EC01-09 | 5-[2-[4-[3-(Dimethylcarbamoyl)-4-hydroxynaphthalen-1-yl]phenyl]ethynyl]pyridine-3-carboxylic acid | 436.1 | | | |
| | EC01-10-01R | EC01-10 | 5-[2-[6-[3-(Dimethylcarbamoyl)-4-hydroxynaphthalen-1-yl]pyridin-3-yl]ethynyl]pyridine-3-carboxylic acid | 437.1 | 438.1 | 0.97 | |
| | EC01-11-01R | EC01-11 | 3-[2-[4-[3-(Dimethylcarbamoyl)-4-hydroxynaphthalen-1-yl]phenyl]phenyl]propanoic acid | 439.2 | 440.2 | 1.26 | |
| | EC01-12-01R | EC01-12 | 3-[2-[6-[3-(Dimethylcarbamoyl)-4-hydroxynaphthalen-1-yl]pyridin-3-yl]phenyl]propanoic acid | 440.2 | 441.2 | 0.95 | |
| | EC01-13-01R | EC01-13 | 4-[4-[3-(Dimethylcarbamoyl)-4-hydroxynaphthalen-1-yl]phenyl]naphthalene-1-carboxylic acid | 461.2 | 462.2 | 1.31 | |
| | EC01-14-01R | EC01-14 | 4-[6-[3-(Dimethylcarbamoyl)-4-hydroxynaphthalen-1-yl]pyridin-3-yl]naphthalene-1-carboxylic acid | 462.2 | 463.2 | 1.05 | |
| | EC01-15-01R | EC01-15 | 3-tert-Butyl-5-[4-[3-(dimethylcarbamoyl)-4-hydroxynaphthalen-1-yl]phenyl]benzoic acid | 467.2 | 468.2 | 1.43 | |
| mixEC01-01R | EC01-16-01R | EC01-16 | 3-tert-Butyl-5-[6-[3-(dimethylcarbamoyl)-4-hydroxynaphthalen-1-yl]pyridin-3-yl]benzoic acid | 468.2 | 469.2 | 1.17 | SMD-FA05-1 |
| | EC01-17-01R | EC01-17 | 3-[4-[3-(Dimethylcarbamoyl)-4-hydroxynaphthalen-1-yl]phenyl]-5-(trifluoromethyl)benzoic acid | 479.1 | 480.1 | 1.35 | |
| | EC01-18-01R | EC01-18 | 3-[6-[3-(Dimethylcarbamoyl)-4-hydroxynaphthalen-1-yl]pyridin-3-yl]-5-(trifluoromethyl)benzoic acid | 480.1 | 481.1 | 1.12 | |

| | | | | | | | |
|---|---|---|---|---|---|---|---|
| | EC01-19-01R | EC01-19 | 3-[2-[4-[3-(Dimethylcarbamoyl)-4-hydroxynaphthalen-1-yl]phenyl]ethynyl]-5-(trifluoromethyl)benzoic acid | 503.1 | 504.1 | 1.45 | |
| | EC01-20-01R | EC01-20 | 3-[2-[6-[3-(Dimethylcarbamoyl)-4-hydroxynaphthalen-1-yl]pyridin-3-yl]ethynyl]-5-(trifluoromethyl)benzoic acid | 504.1 | 505.1 | 1.29 | |
| | EC01-21-01R | EC01-21 | 3-[4-[4-[3-(Dimethylcarbamoyl)-4-hydroxynaphthalen-1-yl]phenyl]pyrazol-1-yl]-5-(trifluoromethyl)benzoic acid | 545.2 | 546.2 | 1.39 | |
| | EC01-22-01R | EC01-22 | 3-[4-[6-[3-(Dimethylcarbamoyl)-4-hydroxynaphthalen-1-yl]pyridin-3-yl]pyrazol-1-yl]-5-(trifluoromethyl)benzoic acid | 546.2 | 547.2 | 1.15 | |

Bond formation reaction was carried out for the carboxylic acid mixture synthesized in Example 3-5-1 through amide coupling reaction by the following method.

Example 3-5-2: Synthesis ofmixEC02-01R

[Formula 514]

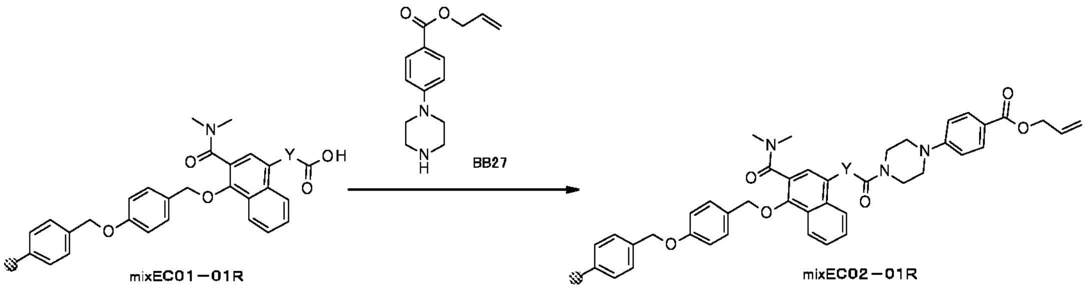


mixEC01-01R (mixture of 22 compounds, a total of 210 mg, loading rate: 0.38 mmol/g, 0.079 mmol) shown in Table EC01 and NMP (3.6 mL) were added to a 10 mL glass vial and shaken at room temperature for 1 hour. Allyl 4-(piperazin-1-yl)benzoate (BB27, 59.0 mg, 0.239 mmol), DIC (75 µL, 0.479 mmol), and HOAt (65.2 mg, 0.479 mmol) were added thereto, and the mixture was shaken at room temperature for 20 hours. The suspension of the reaction solution and the resin was transferred to a syringe with a filter using NMP and repetitively washed three times with NMP (4 mL), three times with MeOH (4 mL), three times with DCM (4 mL), and three times with heptane (4 mL), and the resin was then dried under reduced pressure to obtain the title mixture (mixEC02-01R) shown in Table EC02.

As described in Table EC02, the absence of synthesis of two compounds (EC02-07- 01R and EC02-08-01R) in this amidation reaction was identified by LC-MS analysis. Exemplary supply of a "high-quality" library by carrying out analysis in each step to confirm the presence or absence of formation of the target compound will be illustrated.

[Table 97]

[Table EC02]

| No. of mixture | No. of contained compound | No. of contained compound after cleavage | Compound name | Exact MS | Detected MS: m/z [M+H]+ | LC retention time | Analysis method |
|---|---|---|---|---|---|---|---|
| mixEC02-01R | EC02-01-01R | EC02-01 | Prop-2-enyl 4-[4-[4-[4-[3-(dimethylcarbamoyl)-4-hydroxynaphthalen-1-yl]phenyl]thiophene-2-carbonyl]piperazin-1-yl]benzoate | 645.2 | 646.2 | 1.49 | SMD-AC05-1 |
| | EC02-02-01R | EC02-02 | Prop-2-enyl 4-[4-[4-[6-[3-(dimethylcarbamoyl)-4-hydroxynaphthalen-1-yl]pyridin-3-yl]thiophene-2-carbonyl]piperazin-1-yl]benzoate | 646.2 | 647.2 | 1.31 | |
| | EC02-03-01R | EC02-03 | Prop-2-enyl 4-[4-[4-[4-[3-(dimethylcarbamoyl)-4-hydroxynaphthalen-1-yl]phenyl]-3-methylbenzoyl]piperazin-1-yl]benzoate | 653.3 | 654.3 | 1.53 | |
| | EC02-04-01R | EC02-04 | Prop-2-enyl 4-[4-[4-[6-[3-(dimethylcarbamoyl)-4-hydroxynaphthalen-1-yl]pyridin-3-yl]-3-methylbenzoyl]piperazin-1-yl]benzoate | 654.3 | 655.3 | 1.33 | |
| | EC02-05-01R | EC02-05 | Prop-2-enyl 4-[4-[4-[4-[3-(dimethylcarbamoyl)-4-hydroxynaphthalen-1-yl]phenyl]-3-fluorobenzoyl]piperazin-1-yl]benzoate | 657.3 | 658.3 | 1.5 | |
| | EC02-06-01R | EC02-06 | Prop-2-enyl 4-[4-[4-[6-[3-(dimethylcarbamoyl)-4-hydroxynaphthalen-1-yl]pyridin-3-yl]-3-fluorobenzoyl]piperazin-1-yl]benzoate | 658.3 | 659.3 | 1.32 | |
| mixEC02-01R | EC02-07-01R | EC02-07 | Prop-2-enyl 4-[4-[2-[2-[4-[3-(dimethylcarbamoyl)-4-hydroxynaphthalen-1-yl]phenyl]ethynyl]benzoyl]piperazin-1-yl]benzoate | 663.3 | na | na | SMD-AC05-1 |
| | EC02-08-01R | EC02-08 | Prop-2-enyl 4-[4-[2-[2-[6-[3-(dimethylcarbamoyl)-4-hydroxynaphthalen-1-yl]pyridin-3-yl]ethynyl]benzoyl]piperazin-1-yl]benzoate | 664.3 | na | na | |
| | EC02-09-01R | EC02-09 | Prop-2-enyl 4-[4-[5-[2-[4-[3-(dimethylcarbamoyl)-4-hydroxynaphthalen-1-yl]phenyl]ethynyl]pyridine-3-carbonyl]piperazin-1-yl]benzoate | 664.3 | 665.3 | 1.44 | |
| | EC02-10-01R | EC02-10 | Prop-2-enyl 4-[4-[5-[2-[6-[3-(dimethylcarbamoyl)-4-hydroxynaphthalen-1-yl]pyridin-3-yl]ethynyl]pyridine-3-carbonyl]piperazin-1-yl]benzoate | 665.3 | 666.3 | 1.26 | |
| | EC02-11-01R | EC02-11 | Prop-2-enyl 4-[4-[3-[2-[4-[3-(dimethylcarbamoyl)-4-hydroxynaphthalen-1-yl]phenyl]phenyl]propanoyl]piperazin-1-yl]benzoate | 667.3 | 668.3 | 1.53 | |
| | EC02-12-01R | EC02-12 | Prop-2-enyl 4-[4-[3-[2-[6-[3-(dimethylcarbamoyl)-4-hydroxynaphthalen-1-yl]pyridin-3-yl]phenyl]propanoyl]piperazin-1-yl]benzoate | 668.3 | 669.3 | 1.35 | |
| | EC02-13-01R | EC02-13 | Prop-2-enyl 4-[4-[4-[4-[3-(dimethylcarbamoyl)-4-hydroxynaphthalen-1-yl]phenyl]naphthalene-1-carbonyl]piperazin-1-yl]benzoate | 689.3 | 690.3 | 1.58 | |
| mixEC02-01R | EC02-14-01R | EC02-14 | Prop-2-enyl 4-[4-[4-[6-[3-(dimethylcarbamoyl)-4-hydroxynaphthalen-1-yl]pyridin-3-yl]naphthalene-1-carbonyl]piperazin-1-yl]benzoate | 690.3 | 691.3 | 1.39 | SMD-AC05-1 |

| | | | | | | | |
|---|---|---|---|---|---|---|---|
| | EC02-15-01R | EC02-15 | Prop-2-enyl 4-[4-[3-tert-butyl-5-[4-[3-(dimethylcarbamoyl)-4-hydroxynaphthalen-1-yl]phenyl]benzoyl]piperazin-1-yl]benzoate | 695.3 | 696.3 | 1.69 | |
| | EC02-16-01R | EC02-16 | Prop-2-enyl 4-[4-[3-tert-butyl-5-[6-[3-(dimethylcarbamoyl)-4-hydroxynaphthalen-1-yl]pyridin-3-yl]benzoyl]piperazin-1-yl]benzoate | 696.3 | 697.3 | 1.53 | |
| | EC02-17-01R | EC02-17 | Prop-2-enyl 4-[4-[3-[4-[3-(dimethylcarbamoyl)-4-hydroxynaphthalen-1-yl]phenyl]-5-(trifluoromethyl)benzoyl]piperazin-1-yl]benzoate | 707.3 | 708.3 | 1.59 | |
| | EC02-18-01R | EC02-18 | Prop-2-enyl 4-[4-[3-[6-[3-(dimethylcarbamoyl)-4-hydroxynaphthalen-1-yl]pyridin-3-yl]-5-(trifluoromethyl)benzoyl]piperazin-1-yl]benzoate | 708.3 | 709.3 | 1.42 | |
| | EC02-19-01R | EC02-19 | Prop-2-enyl 4-[4-[3-[2-[4-[3-(dimethylcarbamoyl)-4-hydroxynaphthalen-1-yl]phenyl]ethynyl]-5-(trifluoromethyl)benzoyl]piperazin-1-yl]benzoate | 731.3 | 732.3 | 1.67 | |
| | EC02-20-01R | EC02-20 | Prop-2-enyl 4-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]benzoate | 732.3 | 733.3 | 1.53 | |
| mixEC02-01R | EC02-21-01R | EC02-21 | Prop-2-enyl 4-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]benzoate | 773.3 | 774.3 | 1.62 | SMD-AC05-1 |
| | EC02-22-01R | EC02-22 | Prop-2-enyl 4-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]benzoate | 774.3 | 775.3 | 1.47 | |

na = not available

Example 3-5-3: Synthesis of mixEC03-01R

[Formula 515]

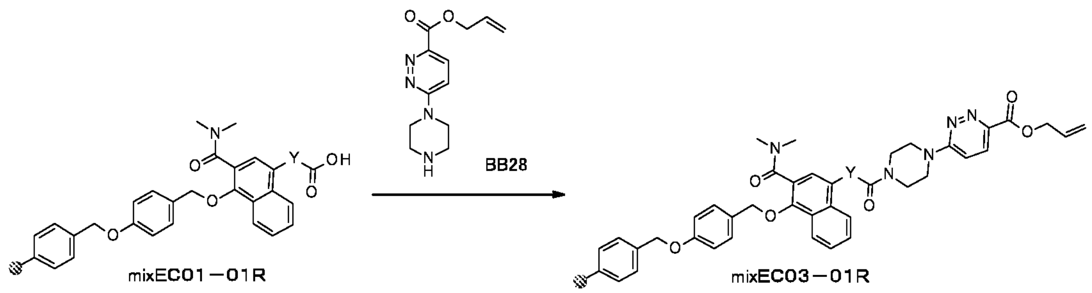


mixEC01-01R mixEC03-01R

The title mixture mixEC03-01R) shown in Table EC03 was obtained in the same manner as in Example 3-5-2 using allyl 6-(piperazin-1-yl)pyridazine-3-carboxylate (BB28) and mixture mixEC01-01R shown in Table EC01.

As described in Table EC03, the absence of synthesis of two compounds (EC03-07- 01R and EC03-08-01R) in this amidation reaction was identified by LC-MS analysis. Exemplary supply of a "high-quality" library by carrying out analysis in each step to confirm the presence or absence of formation of the target compound will be illustrated.

[Table 98]

[Table EC03]

| No. of mixture | No. of contained compound | No. of contained compound after cleavage | Compound name | Exact MS | Detected MS :m/z [M+H]+ | LC retention time | Analysis method |
|---|---|---|---|---|---|---|---|
| mixEC03-01R | EC03-01-01R | EC03-01 | Prop-2-enyl 6-[4-[4-[4-[3-(dimethylcarbamoyl)-4-hydroxynaphthalen-1-yl]phenyl]thiophene-2-carbonyl]piperazin-1-yl]pyridazine-3-carboxylate | 647.2 | 648.2 | 1.25 | SMD-AC05-1 |
| | EC03-02-01R | EC03-02 | Prop-2-enyl 6-[4-[4-[6-[3-(dimethylcarbamoyl)-4-hydroxynaphthalen-1-yl]pyridin-3-yl]thiophene-2-carbonyl]piperazin-1-yl]pyridazine-3-carboxylate | 648.2 | 649.2 | 1.06 | |
| | EC03-03-01R | EC03-03 | Prop-2-enyl 6-[4-[4-[4-[3-(dimethylcarbamoyl)-4-hydroxynaphthalen-1-yl]phenyl]-3-methylbenzoyl]piperazin-1-yl]pyridazine-3-carboxylate | 655.3 | 656.3 | 1.28 | |
| | EC03-04-01R | EC03-04 | Prop-2-enyl 6-[4-[4-[6-[3-(dimethylcarbamoyl)-4-hydroxynaphthalen-1-yl]pyridin-3-yl]-3-methylbenzoyl]piperazin-1-yl]pyridazine-3-carboxylate | 656.3 | 657.3 | 1.09 | |
| | EC03-05-01R | EC03-05 | Prop-2-enyl 6-[4-[4-[4-[3-(dimethylcarbamoyl)-4-hydroxynaphthalen-1-yl]phenyl]-3-fluorobenzoyl]piperazin-1-yl]pyridazine-3-carboxylate | 659.3 | 660.3 | 1.26 | |
| mixEC03-01R | EC03-06-01R | EC03-06 | Prop-2-enyl 6-[4-[4-[6-[3-(dimethylcarbamoyl)-4-hydroxynaphthalen-1-yl]pyridin-3-yl]-3-fluorobenzoyl]piperazin-1-yl]pyridazine-3-carboxylate | 660.3 | 661.3 | 1.08 | SMD-AC05-1 |
| | EC03-07-01R | EC03-07 | Prop-2-enyl 6-[4-[2-[2-[4-[3-(dimethylcarbamoyl)-4-hydroxynaphthalen-1-yl]phenyl]ethynyl]benzoyl]piperazin-1-yl]pyridazine-3-carboxylate | 665.3 | na | na | |
| | EC03-08-01R | EC03-08 | Prop-2-enyl 6-[4-[2-[2-[6-[3-(dimethylcarbamoyl)-4-hydroxynaphthalen-1-yl]pyridin-3-yl]ethynyl]benzoyl]piperazin-1-yl]pyridazine-3-carboxylate | 666.3 | na | na | |
| | EC03-09-01R | EC03-09 | Prop-2-enyl 6-[4-[5-[2-[4-[3-(dimethylcarbamoyl)-4-hydroxynaphthalen-1-yl]phenyl]ethynyl]pyridine-3-carbonyl]piperazin-1-yl]pyridazine-3-carboxylate | 666.3 | 667.3 | 1.20 | |
| | EC03-10-01R | EC03-10 | Prop-2-enyl 6-[4-[5-[2-[6-[3-(dimethylcarbamoyl)-4-hydroxynaphthalen-1-yl]pyridin-3-yl]ethynyl]pyridine-3-carbonyl]piperazin-1-yl]pyridazine-3-carboxylate | 667.3 | 668.3 | 1.53 | |
| | EC03-11-01R | EC03-11 | Prop-2-enyl 6-[4-[3-[2-[4-[3-(dimethylcarbamoyl)-4-hydroxynaphthalen-1-yl]phenyl]phenyl]propanoyl]piperazin-1-yl]pyridazine-3-carboxylate | 669.3 | 670.3 | 1.31 | |

| | | | | | | | |
|---|---|---|---|---|---|---|---|
| mixEC03-01R | EC03-12-01R | EC03-12 | Prop-2-enyl 6-[4-[3-[2-[6-[3-(dimethylcarbamoyl)-4-hydroxynaphthalen-1-yl]pyridin-3-yl]phenyl]propanoyl]piperazin-1-yl]pyridazine-3-carboxylate | 670.3 | 671.3 | 1.12 | SMD-AC05-1 |
| | EC03-13-01R | EC03-13 | Prop-2-enyl 6-[4-[4-[4-[3-(dimethylcarbamoyl)-4-hydroxynaphthalen-1-yl]phenyl]naphthalene-1-carbonyl]piperazin-1-yl]pyridazine-3-carboxylate | 691.3 | 692.3 | 1.33 | |
| | EC03-14-01R | EC03-14 | Prop-2-enyl 6-[4-[4-[6-[3-(dimethylcarbamoyl)-4-hydroxynaphthalen-1-yl]pyridin-3-yl]naphthalene-1-carbonyl]piperazin-1-yl]pyridazine-3-carboxylate | 692.3 | 693.3 | 1.15 | |
| | EC03-15-01R | EC03-15 | Prop-2-enyl 6-[4-[3-tert-butyl-5-[4-[3-(dimethylcarbamoyl)-4-hydroxynaphthalen-1-yl]phenyl]benzoyl]piperazin-1-yl]pyridazine-3-carboxylate | 697.3 | 698.3 | 1.46 | |
| | EC03-16-01R | EC03-16 | Prop-2-enyl 6-[4-[3-tert-butyl-5-[6-[3-(dimethylcarbamoyl)-4-hydroxynaphthalen-1-yl]pyridin-3-yl]benzoyl]piperazin-1-yl]pyridazine-3-carboxylate | 698.3 | 699.3 | 1.27 | |
| | EC03-17-01R | EC03-17 | Prop-2-enyl 6-[4-[3-[4-[3-(dimethylcarbamoyl)-4-hydroxynaphthalen-1-yl]phenyl]-5-(trifluoromethyl)benzoyl]piperazin-1-yl]pyridazine-3-carboxylate | 709.3 | 710.3 | 1.37 | |
| mixEC03-01R | EC03-18-01R | EC03-18 | Prop-2-enyl 6-[4-[3-[6-[3-(dimethylcarbamoyl)-4-hydroxynaphthalen-1-yl]pyridin-3-yl]-5-(trifluoromethyl)benzoyl]piperazin-1-yl]pyridazine-3-carboxylate | 710.2 | 711.2 | 1.19 | SMD-AC05-1 |
| | EC03-19-01R | EC03-19 | Prop-2-enyl 6-[4-[3-[2-[4-[3-(dimethylcarbamoyl)-4-hydroxynaphthalen-1-yl]phenyl]ethynyl]-5-(trifluoromethyl)benzoyl]piperazin-1-yl]pyridazine-3-carboxylate | 733.3 | 734.3 | 1.46 | |
| | EC03-20-01R | EC03-20 | Prop-2-enyl 6-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]pyridazine-3-carboxylate | 734.2 | 735.2 | 1.29 | |
| | EC03-21-01R | EC03-21 | Prop-2-enyl 6-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]pyridazine-3-carboxylate | 775.3 | 776.3 | 1.41 | |
| | EC03-22-01R | EC03-22 | Prop-2-enyl 6-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]pyridazine-3-carboxylate | 776.3 | 777.3 | 1.24 | |

na = not available

Example 3-6: Practice of mixture library synthesis to which bond formation reaction is applied through amide coupling reaction and acylsulfonamide coupling reaction

The deprotection of ester was carried out by the following method for the ester compounds synthesized in Example 3-4 and Example 3-5 to synthesize carboxylic acid compounds.

Example 3-6-1: Preparation of mixAG-D01-01R

Mixtures mixEC02-01R (200 mg), mixEC03-01R (200 mg), and mixEC05-01R (236 mg) shown in Table AG-D-01 were transferred into a glass vial to obtain mixture mixAG-D01-01R for use in Example 3-6-2.

[Table 99]

| [Table AG-D-01] | | | |
|---|---|---|---|
| No. of prepared mixture | No. of mixture used | Amount of mixture used (mg) | No. of compound that may be contained |
| mixAG-D01-01R | mixEC02-01R | 200.0 | EC02-01-01R |
| | | | EC02-02-01R |
| | | | EC02-03-01R |
| | | | EC02-04-01R |
| | | | EC02-05-01R |
| | | | EC02-06-01R |
| | | | EC02-07-01R |
| | | | EC02-08-01R |
| | | | EC02-09-01R |
| | | | EC02-10-01R |
| | | | EC02-11-01R |
| | | | EC02-12-01R |
| | | | EC02-13-01R |
| | | | EC02-14-01R |
| | | | EC02-15-01R |
| | | | EC02-16-01R |
| | | | EC02-17-01R |
| | | | EC02-18-01R |
| | | | EC02-19-01R |
| | | | EC02-20-01R |
| | | | EC02-21-01R |
| | | | EC02-22-01R |
| mixAG-D01-01R | mixEC03-01R | 200.0 | EC03-01-01R |
| | | | EC03-02-01R |
| | | | EC03-03-01R |
| | | | EC03-04-01R |
| | | | EC03-05-01R |
| | | | EC03-06-01R |
| | | | EC03-07-01R |

(continued)

| [Table AG-D-01] | | | |
|---|---|---|---|
| No. of prepared mixture | No. of mixture used | Amount of mixture used (mg) | No. of compound that may be contained |
| | | | EC03-08-01R |
| | | | EC03-09-01R |
| | | | EC03-10-01R |
| | | | EC03-11-01R |
| | | | EC03-12-01R |
| | | | EC03-13-01R |
| | | | EC03-14-01R |
| | | | EC03-15-01R |
| | | | EC03-16-01R |
| | | | EC03-17-01R |
| | | | EC03-18-01R |
| | | | EC03-19-01R |
| | | | EC03-20-01R |
| | | | EC03-21-01R |
| | | | EC03-22-01R |

(continued)

| [Table AG-D-01] | | | |
|---|---|---|---|
| No. of prepared mixture | No. of mixture used | Amount of mixture used (mg) | No. of compound that may be contained |
| mixAG-D01-01R | mixEC05-01R | 236.0 | AG-C-027-01R |
| | | | AG-C-028-01R |
| | | | AG-C-029-01R |
| | | | AG-C-030-01R |
| | | | AG-C-031-01R |
| | | | AG-C-032-01R |
| | | | AG-C-033-01R |
| | | | AG-C-034-01R |
| | | | AG-C-035-01R |
| | | | AG-C-036-01R |
| | | | AG-C-037-01R |
| | | | AG-C-038-01R |
| | | | AG-C-039-01R |
| | | | AG-C-040-01R |
| | | | AG-C-041-01R |
| | | | AG-C-042-01R |
| | | | AG-C-043-01R |
| | | | AG-C-044-01R |
| | | | AG-C-045-01R |
| | | | AG-C-046-01R |
| | | | AG-C-047-01R |
| | | | AG-C-048-01R |
| | | | AG-C-049-01R |
| | | | AG-C-050-01R |
| | | | AG-C-051-01R |
| | | | AG-C-052-01R |

Example 3-6-2: Synthesis of mixED01-01R

[Formula 516]

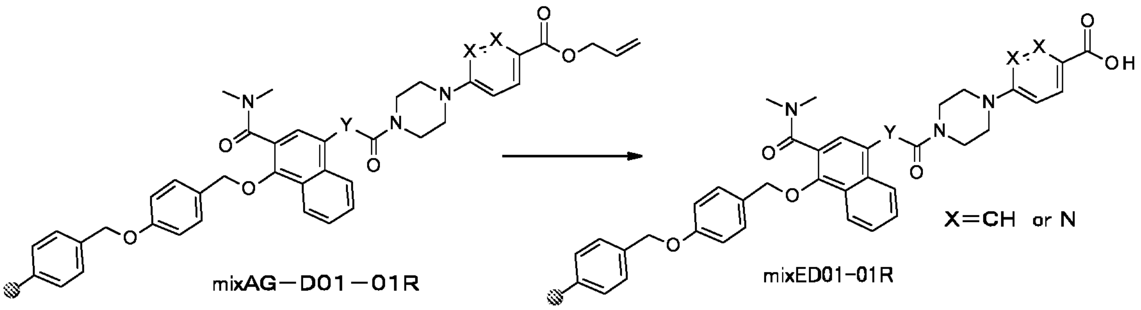

Under a nitrogen atmosphere, mixture mixAG-D01-01R (0.38 mmol/g, 636 mg) shown in Table AG-D-01 and THF (12.7 mL, 20 v/w) were added to a 30 mL screw-cap vial and shaken at room temperature for 1 hour. Under a nitrogen atmosphere, tetrakistriphenylphosphine palladium(0) (84 mg) and morpholine (104 μL) were added thereto, and the mixture was shaken at room temperature for 3 hours. The resin suspension was transferred to a column with a filter using NMP, repetitively washed three times with NMP (12.7 mL), three times with MeOH (12.7 mL), three times with a NAC solution (0.3 M, NMP:water = 5:1 solution, 12.7 mL), three times with a solution of malonic acid in NMP (0.05 M, 12.7 mL), three times with a solution of HOAt in NMP (0.2 M, 12.7 mL), three times with NMP (12.7 mL), three times with MeOH (12.7 mL), three times with DCM (12.7 mL), and three times with heptane (12.7 mL), and then dried under reduced pressure to obtain the title mixture (mixED01-01R). The structures of compounds contained in the obtained mixture are shown in Table AG-D-02.

The reaction was monitored as follows. Under a nitrogen atmosphere, 10 μL of the resin suspension was sampled, then transferred to a pipette tip with a filter, repetitively washed three times with NMP (100 μL), three times with MeOH (100 μL), and three times with DCM (100 μL), and then dipped in a 10% solution of TFA in DCM containing 0.05 M trimethylbenzene (50 μL) for 5 minutes. The cleaving solution was filtered, and the filtrate was dissolved in NMP (250 μL). The solution (50 μL) was diluted with MeCN (250 μL) to prepare an LC sample. The degree of progression of the reaction was measured by LC-MS analysis.

The analysis data (LCMS and retention times of the products) obtained after a lapse of 3 hours in reaction is shown in Table AG-D-02.

[Table 100]

[Table AG-D-02]

| No. of mixture | No. of compound that may be contained | No. of mixture after cleavage | Compound No. after cleavage | Compound name after cleavage | Exact MS of compound after cleavage | Detected MS (M+H)+ | Retention time of MS peak | Analysis method |
|---|---|---|---|---|---|---|---|---|
| mixED01-01R | AG-D-001-01R | mixED01 | AG-D-001 | 4-[4-[4-[4-[3-(Dimethylcarbamoyl)-4-hydroxynaphthalen-1-yl]phenyl]thiophene-2-carbonyl]piperazin-1-yl]benzoic acid | 605.2 | 606.2 | 1.21 | SMD-FA05-01 |
| | AG-D-002-01R | | AG-D-002 | 4-[4-[4-[6-[3-(Dimethylcarbamoyl)-4-hydroxynaphthalen-1-yl]pyridin-3-yl]thiophene-2-carbonyl]piperazin-1-yl]benzoic acid | 606.2 | 607.2 | 0.98 | |
| | AG-D-003-01R | | AG-D-003 | 4-[4-[4-[4-[3-(Dimethylcarbamoyl)-4-hydroxynaphthalen-1-yl]phenyl]-3-methylbenzoyl]piperazin-1-yl]benzoic acid | 613.3 | 614.3 | 1.22 | |
| | AG-D-004-01R | | AG-D-004 | 4-[4-[4-[6-[3-(Dimethylcarbamoyl)-4-hydroxynaphthalen-1-yl]pyridin-3-yl]-3-methylbenzoyl]piperazin-1-yl]benzoic acid | 614.3 | 615.3 | 0.96 | |
| | AG-D-005-01R | | AG-D-005 | 4-[4-[4-[4-[3-(Dimethylcarbamoyl)-4-hydroxynaphthalen-1-yl]phenyl]-3-fluorobenzoyl]piperazin-1-yl]benzoic acid | 617.2 | 618.2 | 1.21 | |
| | AG-D-006-01R | | AG-D-006 | 4-[4-[4-[6-[3-(Dimethylcarbamoyl)-4-hydroxynaphthalen-1-yl]pyridin-3-yl]-3-fluorobenzoyl]piperazin-1-yl]benzoic acid | 618.2 | 619.2 | 1.02 | |
| mixED01-01R | AG-D-007-01R | mixED01 | AG-D-007 | 4-[4-[2-[2-[4-[3-(Dimethylcarbamoyl)-4-hydroxynaphthalen-1-yl]phenyl]ethynyl]benzoyl]piperazin-1-yl]benzoic acid | 623.2 | 624.2 | na | SMD-FA05-01 |
| | AG-D-008-01R | | AG-D-008 | 4-[4-[2-[2-[6-[3-(Dimethylcarbamoyl)-4-hydroxynaphthalen-1-yl]pyridin-3-yl]ethynyl]benzoyl]piperazin-1-yl]benzoic acid | 624.2 | 625.2 | na | |
| | AG-D-009-01R | | AG-D-009 | 4-[4-[5-[2-[4-[3-(Dimethylcarbamoyl)-4-hydroxynaphthalen-1-yl]phenyl]ethynyl]pyridine-3-carbonyl]piperazin-1-yl]benzoic acid | 624.2 | 625.2 | 1.16 | |
| | AG-D-010-01R | | AG-D-010 | 4-[4-[5-[2-[6-[3-(Dimethylcarbamoyl)-4-hydroxynaphthalen-1-yl]pyridin-3-yl]ethynyl]pyridine-3-carbonyl]piperazin-1-yl]benzoic acid | 625.2 | 626.2 | 1.02 | |

| | | | | | | | | |
|---|---|---|---|---|---|---|---|---|
| | AG-D-011-01R | | AG-D-011 | 4-[4-[3-[2-[4-[3-(Dimethylcarbamoyl)-4-hydroxynaphthalen-1-yl]phenyl]phenyl]propanoyl]piperazin-1-yl]benzoic acid | 627.3 | 628.3 | 1.24 | |
| | AG-D-012-01R | | AG-D-012 | 4-[4-[3-[2-[6-[3-(Dimethylcarbamoyl)-4-hydroxynaphthalen-1-yl]pyridin-3-yl]phenyl]propanoyl]piperazin-1-yl]benzoic acid | 628.3 | 629.3 | 1.01 | |
| | AG-D-013-01R | | AG-D-013 | 4-[4-[4-[4-[3-(Dimethylcarbamoyl)-4-hydroxynaphthalen-1-yl]phenyl]naphthalene-1-carbonyl]piperazin-1-yl]benzoic acid | 649.3 | 650.3 | 1.28 | |
| mixED01-01R | AG-D-014-01R | mixED01 | AG-D-014 | 4-[4-[4-[6-[3-(Dimethylcarbamoyl)-4-hydroxynaphthalen-1-yl]pyridin-3-yl]naphthalene-1-carbonyl]piperazin-1-yl]benzoic acid | 650.3 | 651.3 | 1.06 | SMD-FA05-01 |
| | AG-D-015-01R | | AG-D-015 | 4-[4-[3-tert-Butyl-5-[4-[3-(dimethylcarbamoyl)-4-hydroxynaphthalen-1-yl]phenyl]benzoyl]piperazin-1-yl]benzoic acid | 655.3 | 656.3 | 1.39 | |
| | AG-D-016-01R | | AG-D-016 | 4-[4-[3-tert-Butyl-5-[6-[3-(dimethylcarbamoyl)-4-hydroxynaphthalen-1-yl]pyridin-3-yl]benzoyl]piperazin-1-yl]benzoic acid | 656.3 | 657.3 | 1.17 | |
| | AG-D-017-01R | | AG-D-017 | 4-[4-[3-[4-[3-(Dimethylcarbamoyl)-4-hydroxynaphthalen-1-yl]phenyl]-5-(trifluoromethyl)benzoyl]piperazin-1-yl]benzoic acid | 667.2 | 668.2 | 1.31 | |
| | AG-D-018-01R | | AG-D-018 | 4-[4-[3-[6-[3-(Dimethylcarbamoyl)-4-hydroxynaphthalen-1-yl]pyridin-3-yl]-5-(trifluoromethyl)benzoyl]piperazin-1-yl]benzoic acid | 668.2 | 669.2 | 1.13 | |
| | AG-D-019-01R | | AG-D-019 | 4-[4-[3-[2-[4-[3-(Dimethylcarbamoyl)-4-hydroxynaphthalen-1-yl]phenyl]ethynyl]-5-(trifluoromethyl)benzoyl]piperazin-1-yl]benzoic acid | 691.2 | 692.2 | 1.41 | |
| | AG-D-020-01R | | AG-D-020 | 4-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]benzoic acid | 692.2 | 693.2 | 1.27 | |
| mixED01-01R | AG-D-021-01R | mixED01 | AG-D-021 | 4-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]benzoic acid | 733.3 | 734.3 | 1.36 | SMD-FA05-01 |

| | | | | | | | | |
|---|---|---|---|---|---|---|---|---|
| | AG-D-022-01R | | AG-D-022 | 4-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]benzoic acid | 734.2 | 735.2 | 1.16 | |
| | AG-D-023-01R | | AG-D-023 | 6-[4-[4-[4-[3-(Dimethylcarbamoyl)-4-hydroxynaphthalen-1-yl]phenyl]thiophene-2-carbonyl]piperazin-1-yl]pyridazine-3-carboxylic acid | 607.2 | 608.2 | 0.98 | |
| | AG-D-024-01R | | AG-D-024 | 6-[4-[4-[6-[3-(Dimethylcarbamoyl)-4-hydroxynaphthalen-1-yl]pyridin-3-yl]thiophene-2-carbonyl]piperazin-1-yl]pyridazine-3-carboxylic acid | 608.2 | 609.2 | 0.79 | |
| | AG-D-025-01R | | AG-D-025 | 6-[4-[4-[4-[3-(Dimethylcarbamoyl)-4-hydroxynaphthalen-1-yl]phenyl]-3-methylbenzoyl]piperazin-1-yl]pyridazine-3-carboxylic acid | 615.2 | 616.2 | 0.90 | |
| | AG-D-026-01R | | AG-D-026 | 6-[4-[4-[6-[3-(Dimethylcarbamoyl)-4-hydroxynaphthalen-1-yl]pyridin-3-yl]-3-methylbenzoyl]piperazin-1-yl]pyridazine-3-carboxylic acid | 616.2 | 617.2 | 0.77 | |
| | AG-D-027-01R | | AG-D-027 | 6-[4-[4-[4-[3-(Dimethylcarbamoyl)-4-hydroxynaphthalen-1-yl]phenyl]-3-fluorobenzoyl]piperazin-1-yl]pyridazine-3-carboxylic acid | 619.223 | 620.2 | 1.03 | |
| mixED01-01R | AG-D-028-01R | mixED01 | AG-D-028 | 6-[4-[4-[6-[3-(Dimethylcarbamoyl)-4-hydroxynaphthalen-1-yl]pyridin-3-yl]-3-fluorobenzoyl]piperazin-1-yl]pyridazine-3-carboxylic acid | 620.218 | 621.2 | 0.85 | SMD-FA05-01 |
| | AG-D-029-01R | | AG-D-029 | 6-[4-[2-[2-[4-[3-(Dimethylcarbamoyl)-4-hydroxynaphthalen-1-yl]phenyl]ethynyl]benzoyl]piperazin-1-yl]pyridazine-3-carboxylic acid | 625.233 | 626.2 | na | |
| | AG-D-030-01R | | AG-D-030 | 6-[4-[2-[2-[6-[3-(Dimethylcarbamoyl)-4-hydroxynaphthalen-1-yl]pyridin-3-yl]ethynyl]benzoyl]piperazin-1-yl]pyridazine-3-carboxylic acid | 626.228 | 627.2 | na | |
| | AG-D-031-01R | | AG-D-031 | 6-[4-[5-[2-[4-[3-(Dimethylcarbamoyl)-4-hydroxynaphthalen-1-yl]phenyl]ethynyl]pyridine-3-carbonyl]piperazin-1-yl]pyridazine-3-carboxylic acid | 626.228 | 627.2 | 1.01 | |
| | AG-D-032-01R | | AG-D-032 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]pyridazine-3-carboxylic acid | 627.223 | 628.2 | 0.86 | |

| | | | | | | | | |
|---|---|---|---|---|---|---|---|---|
| | AG-D-033-01R | | AG-D-033 | 6-[4-[3-[2-[4-[3-(Dimethylcarbamoyl)-4-hydroxynaphthalen-1-yl]phenyl]phenyl]propanoyl]piperazin-1-yl]pyridazine-3-carboxylic acid | 629.264 | 630.2 | 0.94 | |
| | AG-D-034-01R | | AG-D-034 | 6-[4-[3-[2-[6-[3-(Dimethylcarbamoyl)-4-hydroxynaphthalen-1-yl]pyridin-3-yl]phenyl]propanoyl]piperazin-1-yl]pyridazine-3-carboxylic acid | 630.259 | 631.3 | 0.86 | |
| mixED01-01R | AG-D-035-01R | mixED01 | AG-D-035 | 6-[4-[4-[4-[3-(Dimethylcarbamoyl)-4-hydroxynaphthalen-1-yl]phenyl]naphthalene-1-carbonyl]piperazin-1-yl]pyridazine-3-carboxylic acid | 651.248 | 652.3 | 1.06 | SMD-FA05-01 |
| | AG-D-036-01R | | AG-D-036 | 6-[4-[4-[6-[3-(Dimethylcarbamoyl)-4-hydroxynaphthalen-1-yl]pyridin-3-yl]naphthalene-1-carbonyl]piperazin-1-yl]pyridazine-3-carboxylic acid | 652.243 | 653.3 | 0.89 | |
| | AG-D-037-01R | | AG-D-037 | 6-[4-[3-tert-Butyl-5-[4-[3-(dimethylcarbamoyl)-4-hydroxynaphthalen-1-yl]phenyl]benzoyl]piperazin-1-yl]pyridazine-3-carboxylic acid | 657.295 | 658.3 | 1.17 | |
| | AG-D-038-01R | | AG-D-038 | 6-[4-[3-tert-Butyl-5-[6-[3-(dimethylcarbamoyl)-4-hydroxynaphthalen-1-yl]pyridin-3-yl]benzoyl]piperazin-1-yl]pyridazine-3-carboxylic acid | 658.29 | 659.3 | 1.00 | |
| | AG-D-039-01R | | AG-D-039 | 6-[4-[3-[4-[3-(Dimethylcarbamoyl)-4-hydroxynaphthalen-1-yl]phenyl]-5-(trifluoromethyl)benzoyl]piperazin-1-yl]pyridazine-3-carboxylic acid | 669.22 | 670.2 | 1.02 | |
| | AG-D-040-01R | | AG-D-040 | 6-[4-[3-[6-[3-(Dimethylcarbamoyl)-4-hydroxynaphthalen-1-yl]pyridin-3-yl]-5-(trifluoromethyl)benzoyl]piperazin-1-yl]pyridazine-3-carboxylic acid | 670.215 | 671.2 | 0.90 | |
| | AG-D-041-01R | | AG-D-041 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]pyridazine-3-carboxylic acid | 693.22 | 694.2 | 1.26 | |
| mixED01-01R | AG-D-042-01R | mixED01 | AG-D-042 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]pyridazine-3-carboxylic acid | 694.215 | 695.2 | 1.11 | SMD-FA05-01 |
| | AG-D-043-01R | | AG-D-043 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]pyridazine-3-carboxylic acid | 735.242 | 736.2 | 1.15 | |

| | | | | | | | | |
|---|---|---|---|---|---|---|---|---|
| | AG-D-044-01R | | AG-D-044 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]pyridazine-3-carboxylic acid | 736.237 | 737.2 | 1.00 | |
| | AG-D-045-01R | | AG-D-045 | 4-[4-[[4-[4-[3-(Dimethylcarbamoyl)-4-hydroxynaphthalen-1-yl]phenyl]-3-methylphenyl]methyl]piperazin-1-yl]benzoic acid | 599.278 | 600.3 | 0.90 | |
| | AG-D-046-01R | | AG-D-046 | 4-[4-[[4-[6-[3-(Dimethylcarbamoyl)-4-hydroxynaphthalen-1-yl]pyridin-3-yl]-3-methylphenyl]methyl]piperazin-1-yl]benzoic acid | 600.274 | 601.3 | 0.76 | |
| | AG-D-047-01R | | AG-D-047 | 4-[4-[[2-[4-[3-(Dimethylcarbamoyl)-4-hydroxynaphthalen-1-yl]phenyl]phenyl]methyl]piperazin-1-yl]benzoic acid | 585.263 | 586.3 | 0.88 | |
| | AG-D-048-01R | | AG-D-048 | 4-[4-[[2-[6-[3-(Dimethylcarbamoyl)-4-hydroxynaphthalen-1-yl]pyridin-3-yl]phenyl]methyl]piperazin-1-yl]benzoic acid | 586.258 | 587.3 | 0.77 | |
| mixED01-01R | AG-D-049-01R | mixED01 | AG-D-049 | 4-[4-[[4-[4-[3-(Dimethylcarbamoyl)-4-hydroxynaphthalen-1-yl]phenyl]naphthalen-1-yl]methyl]piperazin-1-yl]benzoic acid | 635.278 | 636.2 | 0.97 | SMD-FA05-01 |
| | AG-D-050-01R | | AG-D-050 | 4-[4-[[4-[6-[3-(Dimethylcarbamoyl)-4-hydroxynaphthalen-1-yl]pyridin-3-yl]naphthalen-1-yl]methyl]piperazin-1-yl]benzoic acid | 636.274 | 637.2 | 0.84 | |
| | AG-D-051-01R | | AG-D-051 | 4-[4-[[4-[4-[3-(Dimethylcarbamoyl)-4-hydroxynaphthalen-1-yl]phenyl]thiophen-2-yl]methyl]piperazin-1-yl]benzoic acid | 591.219 | 592.2 | 0.88 | |
| | AG-D-052-01R | | AG-D-052 | 4-[4-[[4-[6-[3-(Dimethylcarbamoyl)-4-hydroxynaphthalen-1-yl]pyridin-3-yl]thiophen-2-yl]methyl]piperazin-1-yl]benzoic acid | 592.214 | 593.2 | 0.74 | |
| | AG-D-053-01R | | AG-D-053 | 4-[4-[[4-[4-[3-(Dimethylcarbamoyl)-4-hydroxynaphthalen-1-yl]phenyl]-2-ethylphenyl]methyl]piperazin-1-yl]benzoic acid | 613.294 | 614.3 | 0.96 | |
| | AG-D-054-01R | | AG-D-054 | 4-[4-[[4-[6-[3-(Dimethylcarbamoyl)-4-hydroxynaphthalen-1-yl]pyridin-3-yl]-2-ethylphenyl]methyl]piperazin-1-yl]benzoic acid | 614.289 | 615.3 | 0.82 | |

| | | | | | | | | |
|---|---|---|---|---|---|---|---|---|
| | AG-D-055-01R | | AG-D-055 | 4-[4-[[4-[4-[3-(Dimethylcarbamoyl)-4-hydroxynaphthalen-1-yl]phenyl]-2-methoxyphenyl]methyl]piperazin-1-yl]benzoic acid | 615.273 | 616.3 | 0.90 | |
| mixED01-01R | AG-D-056-01R | mixED01 | AG-D-056 | 4-[4-[[4-[6-[3-(Dimethylcarbamoyl)-4-hydroxynaphthalen-1-yl]pyridin-3-yl]-2-methoxyphenyl]methyl]piperazin-1-yl]benzoic acid | 616.269 | 617.3 | 0.77 | SMD-FA05-01 |
| | AG-D-057-01R | | AG-D-057 | 4-[4-[[3-[4-[3-(Dimethylcarbamoyl)-4-hydroxynaphthalen-1-yl]phenyl]-5-ethoxyphenyl]methyl]piperazin-1-yl]benzoic acid | 629.289 | 630.3 | 0.94 | |
| | AG-D-058-01R | | AG-D-058 | 4-[4-[[3-[6-[3-(Dimethylcarbamoyl)-4-hydroxynaphthalen-1-yl]pyridin-3-yl]-5-ethoxyphenyl]methyl]piperazin-1-yl]benzoic acid | 630.284 | 631.3 | 0.86 | |
| | AG-D-059-01R | | AG-D-059 | 4-[4-[[3-[4-[3-(Dimethylcarbamoyl)-4-hydroxynaphthalen-1-yl]phenyl]-5-propan-2-yloxyphenyl]methyl]piperazin-1-yl]benzoic acid | 643.305 | 644.3 | 0.97 | |
| | AG-D-060-01R | | AG-D-060 | 4-[4-[[3-[6-[3-(Dimethylcarbamoyl)-4-hydroxynaphthalen-1-yl]pyridin-3-yl]-5-propan-2-yloxyphenyl]methyl]piperazin-1-yl]benzoic acid | 644.3 | 645.3 | 0.85 | |
| | AG-D-061-01R | | AG-D-061 | 4-[4-[[4-[4-[3-(Dimethylcarbamoyl)-4-hydroxynaphthalen-1-yl]phenyl]-2,6-dimethoxyphenyl]methyl]piperazin-1-yl]benzoic acid | 645.284 | 646.3 | 0.93 | |
| | AG-D-062-01R | | AG-D-062 | 4-[4-[[4-[6-[3-(Dimethylcarbamoyl)-4-hydroxynaphthalen-1-yl]pyridin-3-yl]-2,6-dimethoxyphenyl]methyl]piperazin-1-yl]benzoic acid | 646.279 | 647.3 | 0.81 | |
| mixED01-01R | AG-D-063-01R | mixED01 | AG-D-063 | 4-[4-[[4-[4-[3-(Dimethylcarbamoyl)-4-hydroxynaphthalen-1-yl]phenyl]-3-fluorophenyl]methyl]piperazin-1-yl]benzoic acid | 603.253 | 604.3 | 0.90 | SMD-FA05-01 |
| | AG-D-064-01R | | AG-D-064 | 4-[4-[[4-[6-[3-(Dimethylcarbamoyl)-4-hydroxynaphthalen-1-yl]pyridin-3-yl]-3-fluorophenyl]methyl]piperazin-1-yl]benzoic acid | 604.249 | 605.3 | 0.78 | |

| | | | | | | | | |
|---|---|---|---|---|---|---|---|---|
| | AG-D-065-01R | | AG-D-065 | 4-[4-[[3-[4-[3-(Dimethylcarbamoyl)-4-hydroxynaphthalen-1-yl]phenyl]-5-(trifluoromethyl)phenyl]methyl]piperazin-1-yl]benzoic acid | 653.25 | 654.3 | 1.00 | |
| | AG-D-066-01R | | AG-D-066 | 4-[4-[[3-[6-[3-(Dimethylcarbamoyl)-4-hydroxynaphthalen-1-yl]pyridin-3-yl]-5-(trifluoromethyl)phenyl]methyl]piperazin-1-yl]benzoic acid | 654.245 | 655.3 | 0.88 | |
| | AG-D-067-01R | | AG-D-067 | 4-[4-[[3-[4-[3-(Dimethylcarbamoyl)-4-hydroxynaphthalen-1-yl]phenyl]-5-(trifluoromethoxy)phenyl]methyl]piperazin-1-yl]benzoic acid | 669.245 | 670.3 | 1.15 | |
| | AG-D-068-01R | | AG-D-068 | 4-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]benzoic acid | 670.24 | 671.2 | 0.97 | |
| | AG-D-069-01R | | AG-D-069 | 4-[4-[[2-[2-[4-[3-(Dimethylcarbamoyl)-4-hydroxynaphthalen-1-yl]phenyl]ethynyl]phenyl]methyl]piperazin-1-yl]benzoic acid | 609.263 | 610.3 | 1.24 | |
| mixED01-01R | AG-D-070-01R | mixED01 | AG-D-070 | 4-[4-[[2-[2-[6-[3-(Dimethylcarbamoyl)-4-hydroxynaphthalen-1-yl]pyridin-3-yl]ethynyl]phenyl]methyl]piperazin-1-yl]benzoic acid | 610.258 | 611.3 | 0.85 | SMD-FA05-01 |

Example 3-6-3: Synthesis of mixture mixED02-01R

[Formula 517]

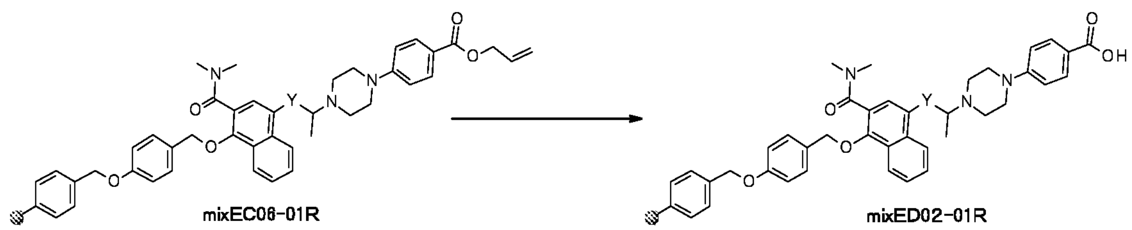

The title mixture mixED02-01R shown in Table ED02 was obtained in the same manner as in Example 3-6-2 using mixture mixEC06-01R (32 mg, 0.38 mmol/g) shown in Table EC06.

[Table 101]

[Table ED02]

| No. of mixture | No. of contained compound | No. of contained compound after cleavage | Compound name | Exact MS | Detected MS: m/z [M+H]+ | LC retention time | Analysis method |
|---|---|---|---|---|---|---|---|
| mixED02-01R | ED02-01-01R | ED02-01 | 4-[4-[1-[4-[4-[3-(Dimethylcarbamoyl)-4-hydroxynaphthalen-1-yl]phenyl]-2-fluorophenyl]ethyl]piperazin-1-yl]benzoic acid | 617.3 | 618 | 0.92 | SMD-FA05-1 |
| | ED02-02-01R | ED02-02 | 4-[4-[1-[4-[6-[3-(Dimethylcarbamoyl)-4-hydroxynaphthalen-1-yl]pyridin-3-yl]-2-fluorophenyl]ethyl]piperazin-1-yl]benzoic acid | 618.3 | 619 | 0.80 | |
| | ED02-03-01R | ED02-03 | 4-[4-[1-[4-[4-[3-(Dimethylcarbamoyl)-4-hydroxynaphthalen-1-yl]phenyl]phenyl]ethyl]piperazin-1-yl]benzoic acid | 599.3 | 600 | 0.90 | |
| | ED02-04-01R | ED02-04 | 4-[4-[1-[4-[6-[3-(Dimethylcarbamoyl)-4-hydroxynaphthalen-1-yl]pyridin-3-yl]phenyl]ethyl]piperazin-1-yl]benzoic acid | 600.3 | 601 | 0.76 | |

Example 3-6-4: Synthesis of mixture mixED03-01R

[Formula 518]

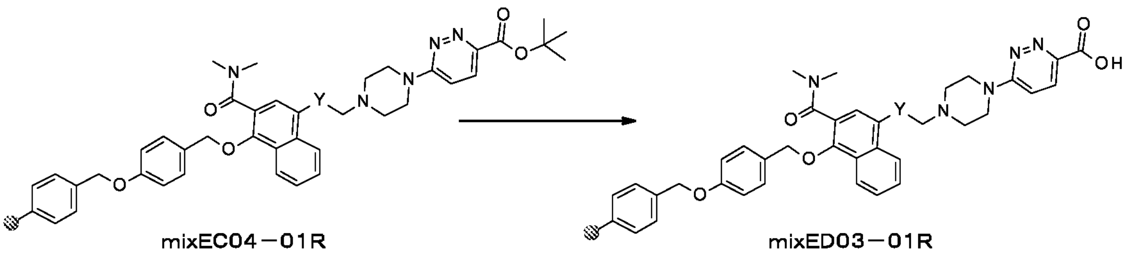

mixEC04-01R mixED03-01R

Under a nitrogen atmosphere, solid-phase supported tBu ester mixture mixEC04- 01R (0.37 mmol/g, 276 mg), THF (4.14 mL, 15 v/w), and MeOH (414 μL, 1.5 v/w) were added to an 8.0 mL screw-cap vial and shaken at room temperature for 1 hour. Under a nitrogen atmosphere, an aqueous LiOH solution (2 M, 383 μL) was added thereto, and the mixture was shaken at room temperature for 8 hours. The resin suspension was transferred to a column with a filter using MeOH, repetitively washed three times with NMP (5.5 mL), three times with water (5.5 mL), three times with NMP (5.5 mL), three times with a solution of HOAt in NMP (0.2 M, 5.5 mL), three times with NMP (5.5 mL), three times with MeOH (5.5 mL), three times with DCM (5.5 mL), and three times with heptane (5.5 mL), and then dried under reduced pressure to obtain the title mixture (mixED03-01R). The structures of compounds contained in the obtained mixture are shown in Table AG-D-03.

The reaction was monitored as follows. Under a nitrogen atmosphere, 10 μL of the resin suspension was sampled, then transferred to a pipette tip with a filter, repetitively washed three times with NMP (100 μL), three times with MeOH (100 μL), and three times with DCM (100 μL), and then dipped in a 10% solution of TFA in DCM containing 0.05 M trimethylbenzene (50 μL) for 5 minutes. The cleaving solution was filtered, and the filtrate was dissolved in NMP (250 μL). The solution (50 μL) was diluted with MeCN (250 μL) to prepare an LC sample. The degree of progression of the reaction was measured by LC-MS analysis.

[Table 102]

[Table AG-D-03]

| No. of mixture | No. of compound that may be contained | No. of mixture after cleavage | Compound No. after cleavage | Compound name after cleavage | Exact MS of compound after cleavage | Detected MS (M+H)+ | Retention time of MS peak | Analysis method |
|---|---|---|---|---|---|---|---|---|
| mixED03-01R | AG-D-071-01R | mixED03 | AG-D-071 | 6-[4-[[4-[4-[3-(Dimethylcarbamoyl)-4-hydroxynaphthalen-1-yl]phenyl]-3-methylphenyl]methyl]piperazin-1-yl]pyridazine-3-carboxylic acid | 601.3 | 602.3 | 7.17 | SMD-FA05-1 |
| | AG-D-072-01R | | AG-D-072 | 6-[4-[[4-[6-[3-(Dimethylcarbamoyl)-4-hydroxynaphthalen-1-yl]pyridin-3-yl]-3-methylphenyl]methyl]piperazin-1-yl]pyridazine-3-carboxylic acid | 602.3 | 603.3 | 5.78 | |
| | AG-D-073-01R | | AG-D-073 | 6-[4-[[2-[4-[3-(Dimethylcarbamoyl)-4-hydroxynaphthalen-1-yl]phenyl]phenyl]methyl]piperazin-1-yl]pyridazine-3-carboxylic acid | 587.3 | 588.3 | 6.90 | |
| | AG-D-074-01R | | AG-D-074 | 6-[4-[[2-[6-[3-(Dimethylcarbamoyl)-4-hydroxynaphthalen-1-yl]pyridin-3-yl]phenyl]methyl]piperazin-1-yl]pyridazine-3-carboxylic acid | 588.2 | 589.2 | 5.76 | |
| | AG-D-075-01R | | AG-D-075 | 6-[4-[[4-[4-[3-(Dimethylcarbamoyl)-4-hydroxynaphthalen-1-yl]phenyl]naphthalen-1-yl]methyl]piperazin-1-yl]pyridazine-3-carboxylic acid | 637.3 | 638.3 | 7.73 | |
| | AG-D-076-01R | | AG-D-076 | 6-[4-[[4-[6-[3-(Dimethylcarbamoyl)-4-hydroxynaphthalen-1-yl]pyridin-3-yl]naphthalen-1-yl]methyl]piperazin-1-yl]pyridazine-3-carboxylic acid | 638.3 | 639.3 | 6.37 | |
| mixED03-01R | AG-D-077-01R | mixED03 | AG-D-077 | 6-[4-[[4-[4-[3-(Dimethylcarbamoyl)-4-hydroxynaphthalen-1-yl]phenyl]thiophen-2-yl]methyl]piperazin-1-yl]pyridazine-3-carboxylic acid | 593.2 | 594.2 | 6.91 | SMD-FA05-1 |
| | AG-D-078-01R | | AG-D-078 | 6-[4-[[4-[6-[3-(Dimethylcarbamoyl)-4-hydroxynaphthalen-1-yl]pyridin-3-yl]thiophen-2-yl]methyl]piperazin-1-yl]pyridazine-3-carboxylic acid | 594.2 | 595.2 | 5.31 | |
| | AG-D-079-01R | | AG-D-079 | 6-[4-[[4-[4-[3-(Dimethylcarbamoyl)-4-hydroxynaphthalen-1-yl]phenyl]-2-ethylphenyl]methyl]piperazin-1-yl]pyridazine-3-carboxylic acid | 615.3 | 616.3 | 7.58 | |

| | | | | | | | | |
|---|---|---|---|---|---|---|---|---|
| | AG-D-080-01R | | AG-D-080 | 6-[4-[[4-[6-[3-(Dimethylcarbamoyl)-4-hydroxynaphthalen-1-yl]pyridin-3-yl]-2-ethylphenyl]methyl]piperazin-1-yl]pyridazine-3-carboxylic acid | 616.3 | 617.3 | 6.22 | |
| | AG-D-081-01R | | AG-D-081 | 6-[4-[[4-[4-[3-(Dimethylcarbamoyl)-4-hydroxynaphthalen-1-yl]phenyl]-2-methoxyphenyl]methyl]piperazin-1-yl]pyridazine-3-carboxylic acid | 617.3 | 618.3 | 7.16 | |
| | AG-D-082-01R | | AG-D-082 | 6-[4-[[4-[6-[3-(Dimethylcarbamoyl)-4-hydroxynaphthalen-1-yl]pyridin-3-yl]-2-methoxyphenyl]methyl]piperazin-1-yl]pyridazine-3-carboxylic acid | 618.3 | 619.3 | 7.15 | |
| | AG-D-083-01R | | AG-D-083 | 6-[4-[[3-[4-[3-(Dimethylcarbamoyl)-4-hydroxynaphthalen-1-yl]phenyl]-5-ethoxyphenyl]methyl]piperazin-1-yl]pyridazine-3-carboxylic acid | 631.3 | 632.3 | 7.57 | |
| mixED03-01R | AG-D-084-01R | mixED03 | AG-D-084 | 6-[4-[[3-[6-[3-(Dimethylcarbamoyl)-4-hydroxynaphthalen-1-yl]pyridin-3-yl]-5-ethoxyphenyl]methyl]piperazin-1-yl]pyridazine-3-carboxylic acid | 632.3 | 633.3 | 7.57 | SMD-FA05-1 |
| | AG-D-085-01R | | AG-D-085 | 6-[4-[[3-[4-[3-(Dimethylcarbamoyl)-4-hydroxynaphthalen-1-yl]phenyl]-5-propan-2-yloxyphenyl]methyl]piperazin-1-yl]pyridazine-3-carboxylic acid | 645.3 | 646.3 | 7.83 | |
| | AG-D-086-01R | | AG-D-086 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]pyridazine-3-carboxylic acid | 646.3 | 647.3 | 6.60 | |
| | AG-D-087-01R | | AG-D-087 | 6-[4-[[4-[4-[3-(Dimethylcarbamoyl)-4-hydroxynaphthalen-1-yl]phenyl]-2,6-dimethoxyphenyl]methyl]piperazin-1-yl]pyridazine-3-carboxylic acid | 647.3 | 648.3 | 7.57 | |
| | AG-D-088-01R | | AG-D-088 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]pyridazine-3-carboxylic acid | 648.3 | 649.3 | 7.40 | |
| | AG-D-089-01R | | AG-D-089 | 6-[4-[[4-[4-[3-(Dimethylcarbamoyl)-4-hydroxynaphthalen-1-yl]phenyl]-3-fluorophenyl]methyl]piperazin-1-yl]pyridazine-3-carboxylic acid | 605.2 | 606.2 | 7.14 | |
| | AG-D-090-01R | | AG-D-090 | 6-[4-[[4-[6-[3-(Dimethylcarbamoyl)-4-hydroxynaphthalen-1-yl]pyridin-3-yl]-3-fluorophenyl]methyl]piperazin-1-yl]pyridazine-3-carboxylic acid | 606.2 | 607.2 | 5.90 | |
| mixED03-01R | AG-D-091-01R | mixED03 | AG-D-091 | 6-[4-[[3-[4-[3-(Dimethylcarbamoyl)-4-hydroxynaphthalen-1-yl]phenyl]-5-(trifluoromethyl)phenyl]methyl] piperazin-1-yl]pyridazine-3-carboxylic acid | 655.2 | 656.2 | 8.06 | SMD-FA05-1 |
| | AG-D-092-01R | | AG-D-092 | 6-[4-[[3-[6-[3-(Dimethylcarbamoyl)-4-hydroxynaphthalen-1-yl]pyridin-3-yl]-5-(trifluoromethyl)phenyl]methyl] piperazin-1-yl]pyridazine-3-carboxylic acid | 656.2 | 657.2 | 6.88 | |

| | | | | | | | | |
|---|---|---|---|---|---|---|---|---|
| | AG-D-093-01R | | AG-D-093 | 6-[4-[[3-[4-[3-(Dimethylcarbamoyl)-4-hydroxynaphthalen-1-yl]phenyl]-5-(trifluoromethoxy)phenyl]methyl] piperazin-1-yl]pyridazine-3-carboxylic acid | 671.2 | 672.2 | 8.21 | |
| | AG-D-094-01R | | AG-D-094 | 6-[4-[[3-[6-[3-(Dimethylcarbamoyl)-4-hydroxynaphthalen-1-yl]pyridin-3-yl]-5-(trifluoromethoxy)phenyl]methyl] piperazin-1-yl]pyridazine-3-carboxylic acid | 672.2 | 673.2 | 7.15 | |
| | AG-D-095-01R | | AG-D-095 | 6-[4-[[2-[2-[4-[3-(Dimethylcarbamoyl)-4-hydroxynaphthalen-1-yl]phenyl]ethynyl]phenyl]methyl] piperazin-1-yl]pyridazine-3-carboxylic acid | 611.3 | 612.3 | 7.56 | |
| | AG-D-096-01R | | AG-D-096 | 6-[4-[[2-[2-[6-[3-(Dimethylcarbamoyl)-4-hydroxynaphthalen-1-yl]pyridin-3-yl]ethynyl]phenyl]methyl]piperazin-1-yl]pyridazine-3-carboxylic acid | 612.2 | 613.2 | 6.66 | |

The carboxylic acid mixtures synthesized in Example 3-6-2, Example 3-6-3, and Example 3-6-4 were further mixed by the following operation.

Example 3-6-5: Preparation of mixture mixED04-01R

Mixtures mixED01-01R (421 mg), mixED02-01R (24 mg), mixED03-01R (156 mg) shown in Table AG-D-04 were transferred to a column with a filter, repetitively washed three times with NMP (12 mL), three times with MeOH (12 mL), three times with DCM (12 mL), and three times with heptane (12 mL), and then dried under reduced pressure to obtain mixture mixED04-01R (601 mg) for use in Example 3-6-6 to Example 3-6-11.

[Formula 519]

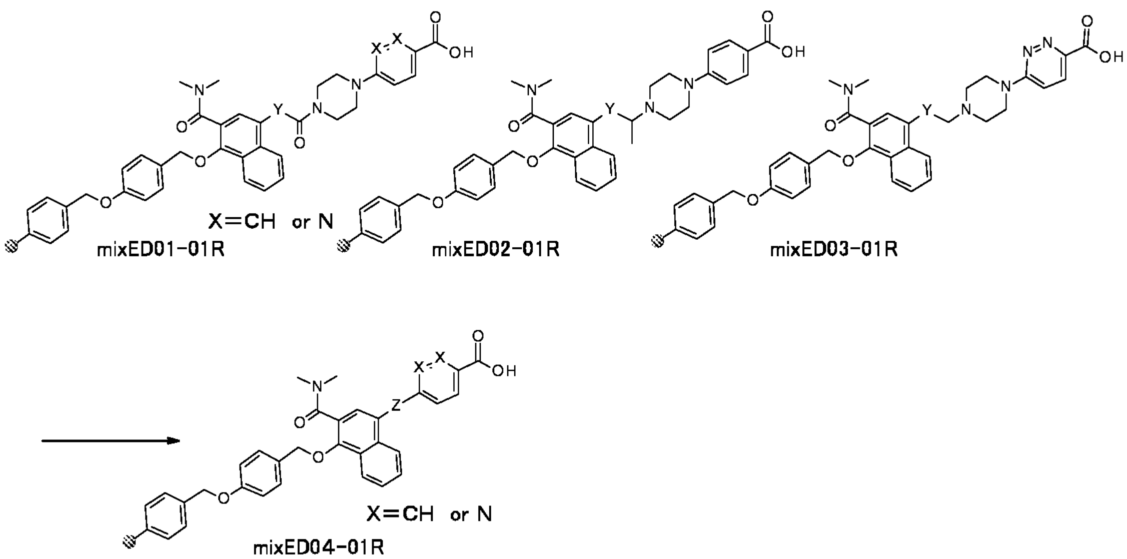

X=CH or N

mixED01-01R

mixED02-01R

mixED03-01R

X=CH or N

mixED04-01R

[Table 103]

| [Table AG-D-04] | | | | | |
|---|---|---|---|---|---|
| No. of prepared mixture | mixED04-01R | | | | |
| No. of mixture used | Amount of mixture used (mg) | No. of compound that may be contained | No. of mixture used | Amount of mixture used (mg) | No. of compound that may be contained |
| mixED01-01R | 421 | AG-D-001-01R | mixED02-01R | 24.0 | ED02-01-01R |
| | | AG-D-002-01R | | | ED02-02-01R |
| | | AG-D-003-01R | | | ED02-03-01R |
| | | AG-D-004-01R | | | ED02-04-01R |
| | | AG-D-005-01R | mixED03-01R | 156 | AG-D-071-01R |
| | | AG-D-006-01R | | | AG-D-072-01R |
| | | AG-D-007-01R | | | AG-D-073-01R |
| | | AG-D-008-01R | | | AG-D-074-01R |
| | | AG-D-009-01R | | | AG-D-075-01R |
| | | AG-D-010-01R | | | AG-D-076-01R |
| | | AG-D-011-01R | | | AG-D-077-01R |
| | | AG-D-012-01R | | | AG-D-078-01R |
| | | AG-D-013-01R | | | AG-D-079-01R |
| | | AG-D-014-01R | | | AG-D-080-01R |
| | | AG-D-015-01R | | | AG-D-081-01R |
| | | AG-D-016-01R | | | AG-D-082-01R |
| | | AG-D-017-01R | | | AG-D-083-01R |
| | | AG-D-018-01R | | | AG-D-084-01R |
| | | AG-D-019-01R | | | AG-D-085-01R |
| | | AG-D-020-01R | | | AG-D-086-01R |
| | | AG-D-021-01R | | | AG-D-087-01R |
| | | AG-D-022-01R | | | AG-D-088-01R |
| | | AG-D-023-01R | | | AG-D-089-01R |
| | | AG-D-024-01R | | | AG-D-090-01R |
| | | AG-D-025-01R | | | AG-D-091-01R |
| | | AG-D-026-01R | | | AG-D-092-01R |
| | | AG-D-027-01R | | | AG-D-093-01R |
| | | AG-D-028-01R | | | AG-D-094-01R |
| | | AG-D-029-01R | | | AG-D-095-01R |
| | | AG-D-030-01R | | | AG-D-096-01R |
| | | AG-D-031-01R | | | |
| | | AG-D-032-01R | | | |
| | | AG-D-033-01R | | | |

(continued)

| [Table AG-D-04] | | | | | |
|---|---|---|---|---|---|
| No. of prepared mixture | mixED04-01R | | | | |
| No. of mixture used | Amount of mixture used (mg) | No. of compound that may be contained | No. of mixture used | Amount of mixture used (mg) | No. of compound that may be contained |
| | | AG-D-034-01R | | | |
| | | AG-D-035-01R | | | |
| | | AG-D-036-01R | | | |
| | | AG-D-037-01R | | | |
| | | AG-D-038-01R | | | |
| | | AG-D-039-01R | | | |
| | | AG-D-040-01R | | | |
| | | AG-D-041-01R | | | |
| | | AG-D-042-01R | | | |
| | | AG-D-043-01R | | | |
| | | AG-D-044-01R | | | |
| | | AG-D-045-01R | | | |
| | | AG-D-046-01R | | | |
| | | AG-D-047-01R | | | |
| | | AG-D-048-01R | | | |
| | | AG-D-049-01R | | | |
| | | AG-D-050-01R | | | |
| | | AG-D-051-01R | | | |
| | | AG-D-052-01R | | | |
| | | AG-D-053-01R | | | |
| | | AG-D-054-01R | | | |
| | | AG-D-055-01R | | | |
| | | AG-D-056-01R | | | |
| | | AG-D-057-01R | | | |
| | | AG-D-058-01R | | | |
| | | AG-D-059-01R | | | |
| | | AG-D-060-01R | | | |
| | | AG-D-061-01R | | | |
| | | AG-D-062-01R | | | |
| | | AG-D-063-01R | | | |
| | | AG-D-064-01R | | | |
| | | AG-D-065-01R | | | |
| | | AG-D-066-01R | | | |

(continued)

| [Table AG-D-04] | | | | | |
|---|---|---|---|---|---|
| No. of prepared mixture | mixED04-01R | | | | |
| No. of mixture used | Amount of mixture used (mg) | No. of compound that may be contained | No. of mixture used | Amount of mixture used (mg) | No. of compound that may be contained |
| | | AG-D-067-01R | | | |
| | | AG-D-068-01R | | | |
| | | AG-D-069-01R | | | |
| | | AG-D-070-01R | | | |

Bond formation reaction was carried out for carboxylic acid compound mixture mixED04-01R prepared in Example 3-6-5 through amide coupling reaction by the following method.

Example 3-6-6: Synthesis of mixED05-01R

[Formula 520]

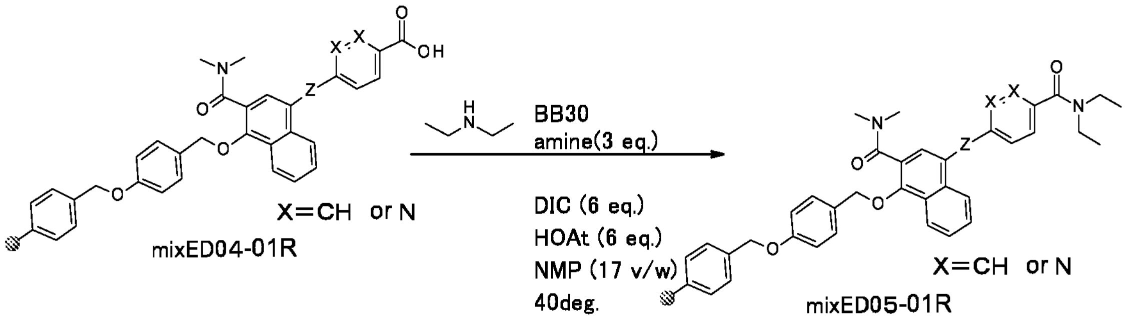


Mixture mixED04-01R (mixture that could contain 100 compounds, 50 mg, loading rate: 0.38 mmol/g, 0.019 mmol) shown in Table AG-D-04 and NMP (0.85 mL) were added to a 1.5 mL glass vial and shaken at room temperature for 1 hour. Diethylamine (BB30, 5.9 μL, 0.057 mmol), DIC (17.8 μL, 0.114 mmol), and HOAt (15.5 mg, 0.114 mmol) were added thereto, and the mixture was shaken at 40°C for 20 hours. The suspension of the reaction solution and the resin was transferred to a syringe with a filter using NMP and repetitively washed three times with NMP (1.2 mL), three times with MeOH (1.2 mL), three times with DCM (1.2 mL), and three times with heptane (1.2 mL), and the resin was then dried under reduced pressure to obtain the title mixture (mixED05-01R) shown in Table ED05.

[Table 104]

[Table ED05]

| No. of mixture | No. of compound that may be contained | Compound No. after cleavage | Compound name after cleavage |
|---|---|---|---|
| mixED05-01R | ED05-01-01R | ED05-01 | 4-[4-[4-[1-[4-[4-(Diethylcarbamoyl)phenyl]piperazin-1-yl]ethyl]phenyl]phenyl]-1-hydroxy-N,N-dimethylnaphthalene-2-carboxamide |
| | ED05-02-01R | ED05-02 | 4-[5-[4-[1-[4-[4-(Diethylcarbamoyl)phenyl]piperazin-1-yl]ethyl]phenyl]pyridin-2-yl]-1-hydroxy-N,N-dimethylnaphthalene-2-carboxamide |
| mixED05-01R | ED05-03-01R | ED05-03 | 4-[4-[4-[1-[4-[4-(Diethylcarbamoyl)phenyl]piperazin-1-yl]ethyl]-3-fluorophenyl]phenyl]-1-hydroxy-N,N-dimethylnaphthalene-2-carboxamide |
| | ED05-04-01R | ED05-04 | 4-[5-[4-[1-[4-[4-(Diethylcarbamoyl)phenyl]piperazin-1-yl]ethyl]-3-fluorophenyl]pyridin-2-yl]-1-hydroxy-N,N-dimethylnaphthalene-2-carboxamide |
| | ED05-05-01R | ED05-05 | 6-[4-[4-[4-[3-(Dimethylcarbamoyl)-4-hydroxynaphthalen-1-yl]phenyl]-3-methylbenzoyl]piperazin-1-yl]-N,N-diethylpyridazine-3-carboxamide |
| | ED05-06-01R | ED05-06 | 6-[4-[4-[6-[3-(Dimethylcarbamoyl)-4-hydroxynaphthalen-1-yl]pyridin-3-yl]-3-methylbenzoyl]piperazin-1-yl]-N,N-diethylpyridazine-3-carboxamide |
| | ED05-07-01R | ED05-07 | 6-[4-[4-[4-[3-(Dimethylcarbamoyl)-4-hydroxynaphthalen-1-yl]phenyl]-3-fluorobenzoyl]piperazin-1-yl]-N,N-diethylpyridazine-3-carboxamide |
| | ED05-08-01R | ED05-08 | 6-[4-[4-[6-[3-(Dimethylcarbamoyl)-4-hydroxynaphthalen-1-yl]pyridin-3-yl]-3-fluorobenzoyl]piperazin-1-yl]-N,N-diethylpyridazine-3-carboxamide |
| | ED05-09-01R | ED05-09 | 6-[4-[3-[4-[3-(Dimethylcarbamoyl)-4-hydroxynaphthalen-1-yl]phenyl]-5-(trifluoromethyl)benzoyl]piperazin-1-yl]-N,N-diethylpyridazine-3-carboxamide |
| | ED05-10-01R | ED05-10 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N,N-diethylpyridazine-3-carboxamide |
| | ED05-11-01R | ED05-11 | 6-[4-[3-tert-Butyl-5-[4-[3-(dimethylcarbamoyl)-4-hydroxynaphthalen-1-yl]phenyl]benzoyl]piperazin-1-yl]-N,N-diethylpyridazine-3-carboxamide |
| | ED05-12-01R | ED05-12 | 6-[4-[3-tert-Butyl-5-[6-[3-(dimethylcarbamoyl)-4-hydroxynaphthalen-1-yl]pyridin-3-yl]benzoyl]piperazin-1-yl]-N,N-diethylpyridazine-3-carboxamide |
| | ED05-13-01R | ED05-13 | 6-[4-[4-[4-[3-(Dimethylcarbamoyl)-4-hydroxynaphthalen-1-yl]phenyl]naphthalene-1-carbonyl]piperazin-1-yl]-N,N-diethylpyridazine-3-carboxamide |
| | ED05-14-01R | ED05-14 | 6-[4-[4-[6-[3-(Dimethylcarbamoyl)-4-hydroxynaphthalen-1-yl]pyridin-3-yl]naphthalene-1-carbonyl]piperazin-1-yl]-N,N-diethylpyridazine-3-carboxamide |
| | ED05-15-01R | ED05-15 | 6-[4-[3-[2-[4-[3-(Dimethylcarbamoyl)-4-hydroxynaphthalen-1-yl]phenyl]phenyl]propanoyl]piperazin-1-yl]-N,N-diethylpyridazine-3-carboxamide |
| | ED05-16-01R | ED05-16 | 6-[4-[3-[2-[6-[3-(Dimethylcarbamoyl)-4-hydroxynaphthalen-1-yl]pyridin-3-yl]phenyl]propanoyl]piperazin-1-yl]-N,N-diethylpyridazine-3-carboxamide |

| | | | |
|---|---|---|---|
| | ED05-17-01R | ED05-17 | 6-[4-[4-[4-[3-(Dimethylcarbamoyl)-4-hydroxynaphthalen-1-yl]phenyl]thiophene-2-carbonyl]piperazin-1-yl]-N,N-diethylpyridazine-3-carboxamide |
| | ED05-18-01R | ED05-18 | 6-[4-[4-[6-[3-(Dimethylcarbamoyl)-4-hydroxynaphthalen-1-yl]pyridin-3-yl]thiophene-2-carbonyl]piperazin-1-yl]-N,N-diethylpyridazine-3-carboxamide |
| mixED05-01R | ED05-19-01R | ED05-19 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N,N-diethylpyridazine-3-carboxamide |
| | ED05-20-01R | ED05-20 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N,N-diethylpyridazine-3-carboxamide |
| | ED05-21-01R | ED05-21 | 6-[4-[2-[2-[4-[3-(Dimethylcarbamoyl)-4-hydroxynaphthalen-1-yl]phenyl]ethynyl]benzoyl]piperazin-1-yl]-N,N-diethylpyridazine-3-carboxamide |
| | ED05-22-01R | ED05-22 | 6-[4-[2-[2-[6-[3-(Dimethylcarbamoyl)-4-hydroxynaphthalen-1-yl]pyridin-3-yl]ethynyl]benzoyl]piperazin-1-yl]-N,N-diethylpyridazine-3-carboxamide |
| | ED05-23-01R | ED05-23 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N,N-diethylpyridazine-3-carboxamide |
| | ED05-24-01R | ED05-24 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N,N-diethylpyridazine-3-carboxamide |
| | ED05-25-01R | ED05-25 | 6-[4-[5-[2-[4-[3-(Dimethylcarbamoyl)-4-hydroxynaphthalen-1-yl]phenyl]ethynyl]pyridine-3-carbonyl]piperazin-1-yl]-N,N-diethylpyridazine-3-carboxamide |
| | ED05-26-01R | ED05-26 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N,N-diethylpyridazine-3-carboxamide |
| | ED05-27-01R | ED05-27 | 4-[4-[4-[4-[4-(Diethylcarbamoyl)phenyl]piperazine-1-carbonyl]-2-methylphenyl]phenyl]-1-hydroxy-N,N-dimethylnaphthalene-2-carboxamide |
| | ED05-28-01R | ED05-28 | 4-[5-[4-[4-[4-(Diethylcarbamoyl)phenyl]piperazine-1-carbonyl]-2-methylphenyl]pyridin-2-yl]-1-hydroxy-N,N-dimethylnaphthalene-2-carboxamide |
| | ED05-29-01R | ED05-29 | 4-[4-[4-[4-[4-(Diethylcarbamoyl)phenyl]piperazine-1-carbonyl]-2-fluorophenyl]phenyl]-1-hydroxy-N,N-dimethylnaphthalene-2-carboxamide |
| | ED05-30-01R | ED05-30 | 4-[5-[4-[4-[4-(Diethylcarbamoyl)phenyl]piperazine-1-carbonyl]-2-fluorophenyl]pyridin-2-yl]-1-hydroxy-N,N-dimethylnaphthalene-2-carboxamide |
| | ED05-31-01R | ED05-31 | 4-[4-[3-[4-[4-(Diethylcarbamoyl)phenyl]piperazine-1-carbonyl]-5-(trifluoromethyl)phenyl]phenyl]-1-hydroxy-N,N-dimethylnaphthalene-2-carboxamide |
| | ED05-32-01R | ED05-32 | 4-[5-[3-[4-[4-(Diethylcarbamoyl)phenyl]piperazine-1-carbonyl]-5-(trifluoromethyl)phenyl]pyridin-2-yl]-1-hydroxy-N,N-dimethylnaphthalene-2-carboxamide |
| | ED05-33-01R | ED05-33 | 4-[4-[3-tert-Butyl-5-[4-[4-(diethylcarbamoyl)phenyl]piperazine-1-carbonyl]phenyl]phenyl]-1-hydroxy-N,N-dimethylnaphthalene-2-carboxamide |
| | ED05-34-01R | ED05-34 | 4-[5-[3-tert-Butyl-5-[4-[4-(diethylcarbamoyl)phenyl]piperazine-1-carbonyl]phenyl]pyridin-2-yl]-1-hydroxy-N,N-dimethylnaphthalene-2-carboxamide |
| mixED05-01R | ED05-35-01R | ED05-35 | 4-[4-[4-[4-[4-(Diethylcarbamoyl)phenyl]piperazine-1-carbonyl]naphthalen-1-yl]phenyl]-1-hydroxy-N,N-dimethylnaphthalene-2-carboxamide |
| | ED05-36-01R | ED05-36 | 4-[5-[4-[4-[4-(Diethylcarbamoyl)phenyl]piperazine-1-carbonyl]naphthalen-1-yl]pyridin-2-yl]-1-hydroxy-N,N-dimethylnaphthalene-2-carboxamide |

| | | | |
|---|---|---|---|
| | ED05-37-01R | ED05-37 | 4-[4-[2-[3-[4-[4-(Diethylcarbamoyl)phenyl]piperazin-1-yl]-3-oxopropyl]phenyl]phenyl]-1-hydroxy-N,N-dimethylnaphthalene-2-carboxamide |
| | ED05-38-01R | ED05-38 | 4-[5-[2-[3-[4-[4-(Diethylcarbamoyl)phenyl]piperazin-1-yl]-3-oxopropyl]phenyl]pyridin-2-yl]-1-hydroxy-N,N-dimethylnaphthalene-2-carboxamide |
| | ED05-39-01R | ED05-39 | 4-[4-[5-[4-[4-(Diethylcarbamoyl)phenyl]piperazine-1-carbonyl]thiophen-3-yl]phenyl]-1-hydroxy-N,N-dimethylnaphthalene-2-carboxamide |
| | ED05-40-01R | ED05-40 | 4-[5-[5-[4-[4-(Diethylcarbamoyl)phenyl]piperazine-1-carbonyl]thiophen-3-yl]pyridin-2-yl]-1-hydroxy-N,N-dimethylnaphthalene-2-carboxamide |
| | ED05-41-01R | ED05-41 | 4-[4-[1-[3-[4-[4-(Diethylcarbamoyl)phenyl]piperazine-1-carbonyl]-5-(trifluoromethyl)phenyl]pyrazol-4-yl]phenyl]-1-hydroxy-N,N-dimethylnaphthalene-2-carboxamide |
| | ED05-42-01R | ED05-42 | 4-[5-[1-[3-[4-[4-(Diethylcarbamoyl)phenyl]piperazine-1-carbonyl]-5-(trifluoromethyl)phenyl]pyrazol-4-yl]pyridin-2-yl]-1-hydroxy-N,N-dimethylnaphthalene-2-carboxamide |
| | ED05-43-01R | ED05-43 | 4-[4-[2-[2-[4-[4-(Diethylcarbamoyl)phenyl]piperazine-1-carbonyl]phenyl]ethynyl]phenyl]-1-hydroxy-N,N-dimethylnaphthalene-2-carboxamide |
| | ED05-44-01R | ED05-44 | 4-[5-[2-[2-[4-[4-(Diethylcarbamoyl)phenyl]piperazine-1-carbonyl]phenyl]ethynyl]pyridin-2-yl]-1-hydroxy-N,N-dimethylnaphthalene-2-carboxamide |
| | ED05-45-01R | ED05-45 | 4-[4-[2-[3-[4-[4-(Diethylcarbamoyl)phenyl]piperazine-1-carbonyl]-5-(trifluoromethyl)phenyl]ethynyl]phenyl]-1-hydroxy-N,N-dimethylnaphthalene-2-carboxamide |
| | ED05-46-01R | ED05-46 | 4-[5-[2-[3-[4-[4-(Diethylcarbamoyl)phenyl]piperazine-1-carbonyl]-5-(trifluoromethyl)phenyl]ethynyl]pyridin-2-yl]-1-hydroxy-N,N-dimethylnaphthalene-2-carboxamide |
| | ED05-47-01R | ED05-47 | 4-[4-[2-[5-[4-[4-(Diethylcarbamoyl)phenyl]piperazine-1-carbonyl]pyridin-3-yl]ethynyl]phenyl]-1-hydroxy-N,N-dimethylnaphthalene-2-carboxamide |
| | ED05-48-01R | ED05-48 | 4-[5-[2-[5-[4-[4-(Diethylcarbamoyl)phenyl]piperazine-1-carbonyl]pyridin-3-yl]ethynyl]pyridin-2-yl]-1-hydroxy-N,N-dimethylnaphthalene-2-carboxamide |
| | ED05-49-01R | ED05-49 | 4-[4-[2-[[4-[4-(Diethylcarbamoyl)phenyl]piperazin-1-yl]methyl]phenyl]phenyl]-1-hydroxy-N,N-dimethylnaphthalene-2-carboxamide |
| | ED05-50-01R | ED05-50 | 4-[5-[2-[[4-[4-(Diethylcarbamoyl)phenyl]piperazin-1-yl]methyl]phenyl]pyridin-2-yl]-1-hydroxy-N,N-dimethylnaphthalene-2-carboxamide |
| mixED05-01R | ED05-51-01R | ED05-51 | 4-[4-[4-[[4-[4-(Diethylcarbamoyl)phenyl]piperazin-1-yl]methyl]-2-methylphenyl]phenyl]-1-hydroxy-N,N-dimethylnaphthalene-2-carboxamide |
| | ED05-52-01R | ED05-52 | 4-[5-[4-[[4-[4-(Diethylcarbamoyl)phenyl]piperazin-1-yl]methyl]-2-methylphenyl]pyridin-2-yl]-1-hydroxy-N,N-dimethylnaphthalene-2-carboxamide |
| | ED05-53-01R | ED05-53 | 4-[4-[4-[[4-[4-(Diethylcarbamoyl)phenyl]piperazin-1-yl]methyl]-3-ethylphenyl]phenyl]-1-hydroxy-N,N-dimethylnaphthalene-2-carboxamide |
| | ED05-54-01R | ED05-54 | 4-[5-[4-[[4-[4-(Diethylcarbamoyl)phenyl]piperazin-1-yl]methyl]-3-ethylphenyl]pyridin-2-yl]-1-hydroxy-N,N-dimethylnaphthalene-2-carboxamide |
| | ED05-55-01R | ED05-55 | 4-[4-[4-[[4-[4-(Diethylcarbamoyl)phenyl]piperazin-1-yl]methyl]-2-fluorophenyl]phenyl]-1-hydroxy-N,N-dimethylnaphthalene-2-carboxamide |
| | ED05-56-01R | ED05-56 | 4-[5-[4-[[4-[4-(Diethylcarbamoyl)phenyl]piperazin-1-yl]methyl]-2-fluorophenyl]pyridin-2-yl]-1-hydroxy-N,N-dimethylnaphthalene-2-carboxamide |
| | ED05-57-01R | ED05-57 | 4-[4-[4-[[4-[4-(Diethylcarbamoyl)phenyl]piperazin-1-yl]methyl]-3-methoxyphenyl]phenyl]-1-hydroxy-N,N-dimethylnaphthalene-2-carboxamide |

| | | | |
|---|---|---|---|
| | ED05-58-01R | ED05-58 | 4-[5-[4-[[4-[4-(Diethylcarbamoyl)phenyl]piperazin-1-yl]methyl]-3-methoxyphenyl]pyridin-2-yl]-1-hydroxy-N,N-dimethylnaphthalene-2-carboxamide |
| | ED05-59-01R | ED05-59 | 4-[4-[3-[[4-[4-(Diethylcarbamoyl)phenyl]piperazin-1-yl]methyl]-5-ethoxyphenyl]phenyl]-1-hydroxy-N,N-dimethylnaphthalene-2-carboxamide |
| | ED05-60-01R | ED05-60 | 4-[5-[3-[[4-[4-(Diethylcarbamoyl)phenyl]piperazin-1-yl]methyl]-5-ethoxyphenyl]pyridin-2-yl]-1-hydroxy-N,N-dimethylnaphthalene-2-carboxamide |
| | ED05-61-01R | ED05-61 | 4-[4-[3-[[4-[4-(Diethylcarbamoyl)phenyl]piperazin-1-yl]methyl]-5-propan-2-yloxyphenyl]phenyl]-1-hydroxy-N,N-dimethylnaphthalene-2-carboxamide |
| | ED05-62-01R | ED05-62 | 4-[5-[3-[[4-[4-(Diethylcarbamoyl)phenyl]piperazin-1-yl]methyl]-5-propan-2-yloxyphenyl]pyridin-2-yl]-1-hydroxy-N,N-dimethylnaphthalene-2-carboxamide |
| | ED05-63-01R | ED05-63 | 4-[4-[4-[[4-[4-(Diethylcarbamoyl)phenyl]piperazin-1-yl]methyl]-3,5-dimethoxyphenyl]phenyl]-1-hydroxy-N,N-dimethylnaphthalene-2-carboxamide |
| | ED05-64-01R | ED05-64 | 4-[5-[4-[[4-[4-(Diethylcarbamoyl)phenyl]piperazin-1-yl]methyl]-3,5-dimethoxyphenyl]pyridin-2-yl]-1-hydroxy-N,N-dimethylnaphthalene-2-carboxamide |
| | ED05-65-01R | ED05-65 | 4-[4-[3-[[4-[4-(Diethylcarbamoyl)phenyl]piperazin-1-yl]methyl]-5-(trifluoromethyl)phenyl]phenyl]-1-hydroxy-N,N-dimethylnaphthalene-2-carboxamide |
| | ED05-66-01R | ED05-66 | 4-[5-[3-[[4-[4-(Diethylcarbamoyl)phenyl]piperazin-1-yl]methyl]-5-(trifluoromethyl)phenyl]pyridin-2-yl]-1-hydroxy-N,N-dimethylnaphthalene-2-carboxamide |
| mixED05-01R | ED05-67-01R | ED05-67 | 4-[4-[3-[[4-[4-(Diethylcarbamoyl)phenyl]piperazin-1-yl]methyl]-5-(trifluoromethoxy)phenyl]phenyl]-1-hydroxy-N,N-dimethylnaphthalene-2-carboxamide |
| | ED05-68-01R | ED05-68 | 4-[5-[3-[[4-[4-(Diethylcarbamoyl)phenyl]piperazin-1-yl]methyl]-5-(trifluoromethoxy)phenyl]pyridin-2-yl]-1-hydroxy-N,N-dimethylnaphthalene-2-carboxamide |
| | ED05-69-01R | ED05-69 | 4-[4-[4-[[4-[4-(Diethylcarbamoyl)phenyl]piperazin-1-yl]methyl]naphthalen-1-yl]phenyl]-1-hydroxy-N,N-dimethylnaphthalene-2-carboxamide |
| | ED05-70-01R | ED05-70 | 4-[5-[4-[[4-[4-(Diethylcarbamoyl)phenyl]piperazin-1-yl]methyl]naphthalen-1-yl]pyridin-2-yl]-1-hydroxy-N,N-dimethylnaphthalene-2-carboxamide |
| | ED05-71-01R | ED05-71 | 4-[4-[5-[[4-[4-(Diethylcarbamoyl)phenyl]piperazin-1-yl]methyl]thiophen-3-yl]phenyl]-1-hydroxy-N,N-dimethylnaphthalene-2-carboxamide |
| | ED05-72-01R | ED05-72 | 4-[5-[5-[[4-[4-(Diethylcarbamoyl)phenyl]piperazin-1-yl]methyl]thiophen-3-yl]pyridin-2-yl]-1-hydroxy-N,N-dimethylnaphthalene-2-carboxamide |
| | ED05-73-01R | ED05-73 | 4-[4-[2-[2-[[4-[4-(Diethylcarbamoyl)phenyl]piperazin-1-yl]methyl]phenyl]ethynyl]phenyl]-1-hydroxy-N,N-dimethylnaphthalene-2-carboxamide |
| | ED05-74-01R | ED05-74 | 4-[5-[2-[2-[[4-[4-(Diethylcarbamoyl)phenyl]piperazin-1-yl]methyl]phenyl]ethynyl]pyridin-2-yl]-1-hydroxy-N,N-dimethylnaphthalene-2-carboxamide |
| | ED05-75-01R | ED05-75 | 6-[4-[[2-[4-[3-(Dimethylcarbamoyl)-4-hydroxynaphthalen-1-yl]phenyl]phenyl]methyl]piperazin-1-yl]-N,N-diethylpyridazine-3-carboxamide |
| | ED05-76-01R | ED05-76 | 6-[4-[[2-[6-[3-(Dimethylcarbamoyl)-4-hydroxynaphthalen-1-yl]pyridin-3-yl]phenyl]methyl]piperazin-1-yl]-N,N-diethylpyridazine-3-carboxamide |
| | ED05-77-01R | ED05-77 | 6-[4-[[4-[4-[3-(Dimethylcarbamoyl)-4-hydroxynaphthalen-1-yl]phenyl]-3-methylphenyl]methyl]piperazin-1-yl]-N,N-diethylpyridazine-3-carboxamide |
| | ED05-78-01R | ED05-78 | 6-[4-[[4-[6-[3-(Dimethylcarbamoyl)-4-hydroxynaphthalen-1-yl]pyridin-3-yl]-3-methylphenyl]methyl]piperazin-1-yl]-N,N-diethylpyridazine-3-carboxamide |
| | ED05-79-01R | ED05-79 | 6-[4-[[4-[4-[3-(Dimethylcarbamoyl)-4-hydroxynaphthalen-1-yl]phenyl]-2-ethylphenyl]methyl]piperazin-1-yl]-N,N-diethylpyridazine-3-carboxamide |

| | | | |
|---|---|---|---|
| | ED05-80-01R | ED05-80 | 6-[4-[[4-[6-[3-(Dimethylcarbamoyl)-4-hydroxynaphthalen-1-yl]pyridin-3-yl]-2-ethylphenyl]methyl]piperazin-1-yl]-N,N-diethylpyridazine-3-carboxamide |
| | ED05-81-01R | ED05-81 | 6-[4-[[4-[4-[3-(Dimethylcarbamoyl)-4-hydroxynaphthalen-1-yl]phenyl]-3-fluorophenyl]methyl]piperazin-1-yl]-N,N-diethylpyridazine-3-carboxamide |
| | ED05-82-01R | ED05-82 | 6-[4-[[4-[6-[3-(Dimethylcarbamoyl)-4-hydroxynaphthalen-1-yl]pyridin-3-yl]-3-fluorophenyl]methyl]piperazin-1-yl]-N,N-diethylpyridazine-3-carboxamide |
| mixED05-01R | ED05-83-01R | ED05-83 | 6-[4-[[4-[4-[3-(Dimethylcarbamoyl)-4-hydroxynaphthalen-1-yl]phenyl]-2-methoxyphenyl]methyl]piperazin-1-yl]-N,N-diethylpyridazine-3-carboxamide |
| | ED05-84-01R | ED05-84 | 6-[4-[[4-[6-[3-(Dimethylcarbamoyl)-4-hydroxynaphthalen-1-yl]pyridin-3-yl]-2-methoxyphenyl]methyl]piperazin-1-yl]-N,N-diethylpyridazine-3-carboxamide |
| | ED05-85-01R | ED05-85 | 6-[4-[[3-[4-[3-(Dimethylcarbamoyl)-4-hydroxynaphthalen-1-yl]phenyl]-5-ethoxyphenyl]methyl]piperazin-1-yl]-N,N-diethylpyridazine-3-carboxamide |
| | ED05-86-01R | ED05-86 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N,N-diethylpyridazine-3-carboxamide |
| | ED05-87-01R | ED05-87 | 6-[4-[[3-[4-[3-(Dimethylcarbamoyl)-4-hydroxynaphthalen-1-yl]phenyl]-5-propan-2-yloxyphenyl]methyl]piperazin-1-yl]-N,N-diethylpyridazine-3-carboxamide |
| | ED05-88-01R | ED05-88 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N,N-diethylpyridazine-3-carboxamide |
| | ED05-89-01R | ED05-89 | 6-[4-[[4-[4-[3-(Dimethylcarbamoyl)-4-hydroxynaphthalen-1-yl]phenyl]-2,6-dimethoxyphenyl]methyl]piperazin-1-yl]-N,N-diethylpyridazine-3-carboxamide |
| | ED05-90-01R | ED05-90 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N,N-diethylpyridazine-3-carboxamide |
| | ED05-91-01R | ED05-91 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N,N-diethylpyridazine-3-carboxamide |
| | ED05-92-01R | ED05-92 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N,N-diethylpyridazine-3-carboxamide |
| | ED05-93-01R | ED05-93 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N,N-diethylpyridazine-3-carboxamide |
| | ED05-94-01R | ED05-94 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N,N-diethylpyridazine-3-carboxamide |
| | ED05-95-01R | ED05-95 | 6-[4-[[4-[4-[3-(Dimethylcarbamoyl)-4-hydroxynaphthalen-1-yl]phenyl]naphthalen-1-yl]methyl]piperazin-1-yl]-N,N-diethylpyridazine-3-carboxamide |
| | ED05-96-01R | ED05-96 | 6-[4-[[4-[6-[3-(Dimethylcarbamoyl)-4-hydroxynaphthalen-1-yl]pyridin-3-yl]naphthalen-1-yl]methyl]piperazin-1-yl]-N,N-diethylpyridazine-3-carboxamide |
| | ED05-97-01R | ED05-97 | 6-[4-[[4-[4-[3-(Dimethylcarbamoyl)-4-hydroxynaphthalen-1-yl]phenyl]thiophen-2-yl]methyl]piperazin-1-yl]-N,N-diethylpyridazine-3-carboxamide |
| | ED05-98-01R | ED05-98 | 6-[4-[[4-[6-[3-(Dimethylcarbamoyl)-4-hydroxynaphthalen-1-yl]pyridin-3-yl]thiophen-2-yl]methyl]piperazin-1-yl]-N,N-diethylpyridazine-3-carboxamide |
| | ED05-99-01R | ED05-99 | 6-[4-[[2-[2-[4-[3-(Dimethylcarbamoyl)-4-hydroxynaphthalen-1-yl]phenyl]ethynyl]phenyl]methyl]piperazin-1-yl]-N,N-diethylpyridazine-3-carboxamide |

| | ED05-100-01R | ED05-100 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N,N-diethylpyridazine-3-carboxamide |
|---|---|---|---|

Example 3-6-7: Synthesis of mixED06-01R to mixED08-01R

In the same manner as in Example 3-6-6, the title mixture (mixED06-01) shown in Table ED06 was obtained using mixED04-01R, the amine reagents shown in Table LBB01, and BB31; the title mixture (mixED07-01) shown in Table ED07 was obtained using BB32; and the title mixture (mixED08-01) shown in Table ED08 was obtained using BB33.

[Table 105]

[Table ED06]

| No. of mixture | No. of compound that may be contained | Compound No. after cleavage | Compound name after cleavage |
|---|---|---|---|
| mixED06-01R | ED06-01-01R | ED06-01 | 1-Hydroxy-N,N-dimethyl-4-[4-[4-[1-[4-[4-(propylcarbamoyl)phenyl]piperazin-1-yl]ethyl]phenyl]phenyl]naphthalene-2-carboxamide |
| | ED06-02-01R | ED06-02 | 1-Hydroxy-N,N-dimethyl-4-[5-[4-[1-[4-[4-(propylcarbamoyl)phenyl]piperazin-1-yl]ethyl]phenyl]pyridin-2-yl]naphthalene-2-carboxamide |
| | ED06-03-01R | ED06-03 | 4-[4-[3-Fluoro-4-[1-[4-[4-(propylcarbamoyl)phenyl]piperazin-1-yl]ethyl]phenyl]phenyl]-1-hydroxy-N,N-dimethylnaphthalene-2-carboxamide |
| | ED06-04-01R | ED06-04 | 4-[5-[3-Fluoro-4-[1-[4-[4-(propylcarbamoyl)phenyl]piperazin-1-yl]ethyl]phenyl]pyridin-2-yl]-1-hydroxy-N,N-dimethylnaphthalene-2-carboxamide |
| | ED06-05-01R | ED06-05 | 6-[4-[4-[4-[3-(Dimethylcarbamoyl)-4-hydroxynaphthalen-1-yl]phenyl]-3-methylbenzoyl]piperazin-1-yl]-N-propylpyridazine-3-carboxamide |
| | ED06-06-01R | ED06-06 | 6-[4-[4-[6-[3-(Dimethylcarbamoyl)-4-hydroxynaphthalen-1-yl]pyridin-3-yl]-3-methylbenzoyl]piperazin-1-yl]-N-propylpyridazine-3-carboxamide |
| | ED06-07-01R | ED06-07 | 6-[4-[4-[4-[3-(Dimethylcarbamoyl)-4-hydroxynaphthalen-1-yl]phenyl]-3-fluorobenzoyl]piperazin-1-yl]-N-propylpyridazine-3-carboxamide |
| | ED06-08-01R | ED06-08 | 6-[4-[4-[6-[3-(Dimethylcarbamoyl)-4-hydroxynaphthalen-1-yl]pyridin-3-yl]-3-fluorobenzoyl]piperazin-1-yl]-N-propylpyridazine-3-carboxamide |
| | ED06-09-01R | ED06-09 | 6-[4-[3-[4-[3-(Dimethylcarbamoyl)-4-hydroxynaphthalen-1-yl]phenyl]-5-(trifluoromethyl)benzoyl]piperazin-1-yl]-N-propylpyridazine-3-carboxamide |
| mixED06-01R | ED06-10-01R | ED06-10 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-propylpyridazine-3-carboxamide |
| | ED06-11-01R | ED06-11 | 6-[4-[3-tert-Butyl-5-[4-[3-(dimethylcarbamoyl)-4-hydroxynaphthalen-1-yl]phenyl]benzoyl]piperazin-1-yl]-N-propylpyridazine-3-carboxamide |
| | ED06-12-01R | ED06-12 | 6-[4-[3-tert-Butyl-5-[6-[3-(dimethylcarbamoyl)-4-hydroxynaphthalen-1-yl]pyridin-3-yl]benzoyl]piperazin-1-yl]-N-propylpyridazine-3-carboxamide |
| | ED06-13-01R | ED06-13 | 6-[4-[4-[4-[3-(Dimethylcarbamoyl)-4-hydroxynaphthalen-1-yl]phenyl]naphthalene-1-carbonyl]piperazin-1-yl]-N-propylpyridazine-3-carboxamide |
| | ED06-14-01R | ED06-14 | 6-[4-[4-[6-[3-(Dimethylcarbamoyl)-4-hydroxynaphthalen-1-yl]pyridin-3-yl]naphthalene-1-carbonyl]piperazin-1-yl]-N-propylpyridazine-3-carboxamide |

| | | | |
|---|---|---|---|
| | ED06-15-01R | ED06-15 | 6-[4-[3-[2-[4-[3-(Dimethylcarbamoyl)-4-hydroxynaphthalen-1-yl]phenyl]phenyl]propanoyl]piperazin-1-yl]-N-propylpyridazine-3-carboxamide |
| | ED06-16-01R | ED06-16 | 6-[4-[3-[2-[6-[3-(Dimethylcarbamoyl)-4-hydroxynaphthalen-1-yl]pyridin-3-yl]phenyl]propanoyl]piperazin-1-yl]-N-propylpyridazine-3-carboxamide |
| | ED06-17-01R | ED06-17 | 6-[4-[4-[4-[3-(Dimethylcarbamoyl)-4-hydroxynaphthalen-1-yl]phenyl]thiophene-2-carbonyl]piperazin-1-yl]-N-propylpyridazine-3-carboxamide |
| | ED06-18-01R | ED06-18 | 6-[4-[4-[6-[3-(Dimethylcarbamoyl)-4-hydroxynaphthalen-1-yl]pyridin-3-yl]thiophene-2-carbonyl]piperazin-1-yl]-N-propylpyridazine-3-carboxamide |
| | ED06-19-01R | ED06-19 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-propylpyridazine-3-carboxamide |
| | ED06-20-01R | ED06-20 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-propylpyridazine-3-carboxamide |
| | ED06-21-01R | ED06-21 | 6-[4-[2-[2-[4-[3-(Dimethylcarbamoyl)-4-hydroxynaphthalen-1-yl]phenyl]ethynyl]benzoyl]piperazin-1-yl]-N-propylpyridazine-3-carboxamide |
| | ED06-22-01R | ED06-22 | 6-[4-[2-[2-[6-[3-(Dimethylcarbamoyl)-4-hydroxynaphthalen-1-yl]pyridin-3-yl]ethynyl]benzoyl]piperazin-1-yl]-N-propylpyridazine-3-carboxamide |
| | ED06-23-01R | ED06-23 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-propylpyridazine-3-carboxamide |
| | ED06-24-01R | ED06-24 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-propylpyridazine-3-carboxamide |
| | ED06-25-01R | ED06-25 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-propylpyridazine-3-carboxamide |
| mixED06-01R | ED06-26-01R | ED06-26 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-propylpyridazine-3-carboxamide |
| | ED06-27-01R | ED06-27 | 1-Hydroxy-N,N-dimethyl-4-[4-[2-methyl-4-[4-[4-(propylcarbamoyl)phenyl]piperazine-1-carbonyl]phenyl]phenyl]naphthalene-2-carboxamide |
| | ED06-28-01R | ED06-28 | 1-Hydroxy-N,N-dimethyl-4-[5-[2-methyl-4-[4-[4-(propylcarbamoyl)phenyl]piperazine-1-carbonyl]phenyl]pyridin-2-yl]naphthalene-2-carboxamide |
| | ED06-29-01R | ED06-29 | 4-[4-[2-Fluoro-4-[4-[4-(propylcarbamoyl)phenyl]piperazine-1-carbonyl]phenyl]phenyl]-1-hydroxy-N,N-dimethylnaphthalene-2-carboxamide |
| | ED06-30-01R | ED06-30 | 4-[5-[2-Fluoro-4-[4-[4-(propylcarbamoyl)phenyl]piperazine-1-carbonyl]phenyl]pyridin-2-yl]-1-hydroxy-N,N-dimethylnaphthalene-2-carboxamide |
| | ED06-31-01R | ED06-31 | 1-Hydroxy-N,N-dimethyl-4-[4-[3-[4-[4-(propylcarbamoyl)phenyl]piperazine-1-carbonyl]-5-(trifluoromethyl)phenyl]phenyl]naphthalene-2-carboxamide |
| | ED06-32-01R | ED06-32 | 1-Hydroxy-N,N-dimethyl-4-[5-[3-[4-[4-(propylcarbamoyl)phenyl]piperazine-1-carbonyl]-5-(trifluoromethyl)phenyl]pyridin-2-yl]naphthalene-2-carboxamide |
| | ED06-33-01R | ED06-33 | 4-[4-[3-tert-Butyl-5-[4-[4-(propylcarbamoyl)phenyl]piperazine-1-carbonyl]phenyl]phenyl]-1-hydroxy-N,N-dimethylnaphthalene-2-carboxamide |
| | ED06-34-01R | ED06-34 | 4-[5-[3-tert-Butyl-5-[4-[4-(propylcarbamoyl)phenyl]piperazine-1-carbonyl]phenyl]pyridin-2-yl]-1-hydroxy-N,N-dimethylnaphthalene-2-carboxamide |

| | | | |
|---|---|---|---|
| | ED06-35-01R | ED06-35 | 1-Hydroxy-N,N-dimethyl-4-[4-[4-[4-[4-(propylcarbamoyl)phenyl]piperazine-1-carbonyl]naphthalen-1-yl]phenyl]naphthalene-2-carboxamide |
| | ED06-36-01R | ED06-36 | 1-Hydroxy-N,N-dimethyl-4-[5-[4-[4-[4-(propylcarbamoyl)phenyl]piperazine-1-carbonyl]naphthalen-1-yl]pyridin-2-yl]naphthalene-2-carboxamide |
| | ED06-37-01R | ED06-37 | 1-Hydroxy-N,N-dimethyl-4-[4-[2-[3-oxo-3-[4-[4-(propylcarbamoyl)phenyl]piperazin-1-yl]propyl]phenyl]phenyl]naphthalene-2-carboxamide |
| | ED06-38-01R | ED06-38 | 1-Hydroxy-N,N-dimethyl-4-[5-[2-[3-oxo-3-[4-[4-(propylcarbamoyl)phenyl]piperazin-1-yl]propyl]phenyl]pyridin-2-yl]naphthalene-2-carboxamide |
| | ED06-39-01R | ED06-39 | 1-Hydroxy-N,N-dimethyl-4-[4-[5-[4-[4-(propylcarbamoyl)phenyl]piperazine-1-carbonyl]thiophen-3-yl]phenyl]naphthalene-2-carboxamide |
| | ED06-40-01R | ED06-40 | 1-Hydroxy-N,N-dimethyl-4-[5-[5-[4-[4-(propylcarbamoyl)phenyl]piperazine-1-carbonyl]thiophen-3-yl]pyridin-2-yl]naphthalene-2-carboxamide |
| | ED06-41-01R | ED06-41 | 1-Hydroxy-N,N-dimethyl-4-[4-[1-[3-[4-[4-(propylcarbamoyl)phenyl]piperazine-1-carbonyl]-5-(trifluoromethyl)phenyl]pyrazol-4-yl]phenyl]naphthalene-2-carboxamide |
| mixED06-01R | ED06-42-01R | ED06-42 | 1-Hydroxy-N,N-dimethyl-4-[5-[1-[3-[4-[4-(propylcarbamoyl)phenyl]piperazine-1-carbonyl]-5-(trifluoromethyl)phenyl]pyrazol-4-yl]pyridin-2-yl]naphthalene-2-carboxamide |
| | ED06-43-01R | ED06-43 | 1-Hydroxy-N,N-dimethyl-4-[4-[2-[2-[4-[4-(propylcarbamoyl)phenyl]piperazine-1-carbonyl]phenyl]ethynyl]phenyl]naphthalene-2-carboxamide |
| | ED06-44-01R | ED06-44 | 1-Hydroxy-N,N-dimethyl-4-[5-[2-[2-[4-[4-(propylcarbamoyl)phenyl]piperazine-1-carbonyl]phenyl]ethynyl]pyridin-2-yl]naphthalene-2-carboxamide |
| | ED06-45-01R | ED06-45 | 1-Hydroxy-N,N-dimethyl-4-[4-[2-[3-[4-[4-(propylcarbamoyl)phenyl]piperazine-1-carbonyl]-5-(trifluoromethyl)phenyl]ethynyl]phenyl]naphthalene-2-carboxamide |
| | ED06-46-01R | ED06-46 | 1-Hydroxy-N,N-dimethyl-4-[5-[2-[3-[4-[4-(propylcarbamoyl)phenyl]piperazine-1-carbonyl]-5-(trifluoromethyl)phenyl]ethynyl]pyridin-2-yl]naphthalene-2-carboxamide |
| | ED06-47-01R | ED06-47 | 1-Hydroxy-N,N-dimethyl-4-[4-[2-[5-[4-[4-(propylcarbamoyl)phenyl]piperazine-1-carbonyl]pyridin-3-yl]ethynyl]phenyl]naphthalene-2-carboxamide |
| | ED06-48-01R | ED06-48 | 1-Hydroxy-N,N-dimethyl-4-[5-[2-[5-[4-[4-(propylcarbamoyl)phenyl]piperazine-1-carbonyl]pyridin-3-yl]ethynyl]pyridin-2-yl]naphthalene-2-carboxamide |
| | ED06-49-01R | ED06-49 | 1-Hydroxy-N,N-dimethyl-4-[4-[2-[[4-[4-(propylcarbamoyl)phenyl]piperazin-1-yl]methyl]phenyl]phenyl]naphthalene-2-carboxamide |
| | ED06-50-01R | ED06-50 | 1-Hydroxy-N,N-dimethyl-4-[5-[2-[[4-[4-(propylcarbamoyl)phenyl]piperazin-1-yl]methyl]phenyl]pyridin-2-yl]naphthalene-2-carboxamide |
| | ED06-51-01R | ED06-51 | 1-Hydroxy-N,N-dimethyl-4-[4-[2-methyl-4-[[4-[4-(propylcarbamoyl)phenyl]piperazin-1-yl]methyl]phenyl]phenyl]naphthalene-2-carboxamide |
| | ED06-52-01R | ED06-52 | 1-Hydroxy-N,N-dimethyl-4-[5-[2-methyl-4-[[4-[4-(propylcarbamoyl)phenyl]piperazin-1-yl]methyl]phenyl]pyridin-2-yl]naphthalene-2-carboxamide |
| | ED06-53-01R | ED06-53 | 4-[4-[3-Ethyl-4-[[4-[4-(propylcarbamoyl)phenyl]piperazin-1-yl]methyl]phenyl]phenyl]-1-hydroxy-N,N-dimethylnaphthalene-2-carboxamide |

| | | | |
|---|---|---|---|
| | ED06-54-01R | ED06-54 | 4-[5-[3-Ethyl-4-[[4-[4-(propylcarbamoyl)phenyl]piperazin-1-yl]methyl]phenyl]pyridin-2-yl]-1-hydroxy-N,N-dimethylnaphthalene-2-carboxamide |
| | ED06-55-01R | ED06-55 | 4-[4-[2-Fluoro-4-[[4-[4-(propylcarbamoyl)phenyl]piperazin-1-yl]methyl]phenyl]phenyl]-1-hydroxy-N,N-dimethylnaphthalene-2-carboxamide |
| | ED06-56-01R | ED06-56 | 4-[5-[2-Fluoro-4-[[4-[4-(propylcarbamoyl)phenyl]piperazin-1-yl]methyl]phenyl]pyridin-2-yl]-1-hydroxy-N,N-dimethylnaphthalene-2-carboxamide |
| mixED06-01R | ED06-57-01R | ED06-57 | 1-Hydroxy-4-[4-[3-methoxy-4-[[4-[4-(propylcarbamoyl)phenyl]piperazin-1-yl]methyl]phenyl]phenyl]-N,N-dimethylnaphthalene-2-carboxamide |
| | ED06-58-01R | ED06-58 | 1-Hydroxy-4-[5-[3-methoxy-4-[[4-[4-(propylcarbamoyl)phenyl]piperazin-1-yl]methyl]phenyl]pyridin-2-yl]-N,N-dimethylnaphthalene-2-carboxamide |
| | ED06-59-01R | ED06-59 | 4-[4-[3-Ethoxy-5-[[4-[4-(propylcarbamoyl)phenyl]piperazin-1-yl]methyl]phenyl]phenyl]-1-hydroxy-N,N-dimethylnaphthalene-2-carboxamide |
| | ED06-60-01R | ED06-60 | 4-[5-[3-Ethoxy-5-[[4-[4-(propylcarbamoyl)phenyl]piperazin-1-yl]methyl]phenyl]pyridin-2-yl]-1-hydroxy-N,N-dimethylnaphthalene-2-carboxamide |
| | ED06-61-01R | ED06-61 | 1-Hydroxy-N,N-dimethyl-4-[4-[3-propan-2-yloxy-5-[[4-[4-(propylcarbamoyl)phenyl]piperazin-1-yl]methyl]phenyl]phenyl]naphthalene-2-carboxamide |
| | ED06-62-01R | ED06-62 | 1-Hydroxy-N,N-dimethyl-4-[5-[3-propan-2-yloxy-5-[[4-[4-(propylcarbamoyl)phenyl]piperazin-1-yl]methyl]phenyl]pyridin-2-yl]naphthalene-2-carboxamide |
| | ED06-63-01R | ED06-63 | 4-[4-[3,5-Dimethoxy-4-[[4-[4-(propylcarbamoyl)phenyl]piperazin-1-yl]methyl]phenyl]phenyl]-1-hydroxy-N,N-dimethylnaphthalene-2-carboxamide |
| | ED06-64-01R | ED06-64 | 4-[5-[3,5-Dimethoxy-4-[[4-[4-(propylcarbamoyl)phenyl]piperazin-1-yl]methyl]phenyl]pyridin-2-yl]-1-hydroxy-N,N-dimethylnaphthalene-2-carboxamide |
| | ED06-65-01R | ED06-65 | 1-Hydroxy-N,N-dimethyl-4-[4-[3-[[4-[4-(propylcarbamoyl)phenyl]piperazin-1-yl]methyl]-5-(trifluoromethyl)phenyl]phenyl]naphthalene-2-carboxamide |
| | ED06-66-01R | ED06-66 | 1-Hydroxy-N,N-dimethyl-4-[5-[3-[[4-[4-(propylcarbamoyl)phenyl]piperazin-1-yl]methyl]-5-(trifluoromethyl)phenyl]pyridin-2-yl]naphthalene-2-carboxamide |
| | ED06-67-01R | ED06-67 | 1-Hydroxy-N,N-dimethyl-4-[4-[3-[[4-[4-(propylcarbamoyl)phenyl]piperazin-1-yl]methyl]-5-(trifluoromethoxy)phenyl]phenyl]naphthalene-2-carboxamide |
| | ED06-68-01R | ED06-68 | 1-Hydroxy-N,N-dimethyl-4-[5-[3-[[4-[4-(propylcarbamoyl)phenyl]piperazin-1-yl]methyl]-5-(trifluoromethoxy)phenyl]pyridin-2-yl]naphthalene-2-carboxamide |
| | ED06-69-01R | ED06-69 | 1-Hydroxy-N,N-dimethyl-4-[4-[4-[[4-[4-(propylcarbamoyl)phenyl]piperazin-1-yl]methyl]naphthalen-1-yl]phenyl]naphthalene-2-carboxamide |
| | ED06-70-01R | ED06-70 | 1-Hydroxy-N,N-dimethyl-4-[5-[4-[[4-[4-(propylcarbamoyl)phenyl]piperazin-1-yl]methyl]naphthalen-1-yl]pyridin-2-yl]naphthalene-2-carboxamide |
| | ED06-71-01R | ED06-71 | 1-Hydroxy-N,N-dimethyl-4-[4-[5-[[4-[4-(propylcarbamoyl)phenyl]piperazin-1-yl]methyl]thiophen-3-yl]phenyl]naphthalene-2-carboxamide |
| | ED06-72-01R | ED06-72 | 1-Hydroxy-N,N-dimethyl-4-[5-[5-[[4-[4-(propylcarbamoyl)phenyl]piperazin-1-yl]methyl]thiophen-3-yl]pyridin-2-yl]naphthalene-2-carboxamide |
| mixED06-01R | ED06-73-01R | ED06-73 | 1-Hydroxy-N,N-dimethyl-4-[4-[2-[2-[[4-[4-(propylcarbamoyl)phenyl]piperazin-1-yl]methyl]phenyl]ethynyl]phenyl]naphthalene-2-carboxamide |
| | ED06-74-01R | ED06-74 | 1-Hydroxy-N,N-dimethyl-4-[5-[2-[2-[[4-[4-(propylcarbamoyl)phenyl]piperazin-1-yl]methyl]phenyl]ethynyl]pyridin-2-yl]naphthalene-2-carboxamide |

| | | | |
|---|---|---|---|
| | ED06-75-01R | ED06-75 | 6-[4-[[2-[4-[3-(Dimethylcarbamoyl)-4-hydroxynaphthalen-1-yl]phenyl]phenyl]methyl]piperazin-1-yl]-N-propylpyridazine-3-carboxamide |
| | ED06-76-01R | ED06-76 | 6-[4-[[2-[6-[3-(Dimethylcarbamoyl)-4-hydroxynaphthalen-1-yl]pyridin-3-yl]phenyl]methyl]piperazin-1-yl]-N-propylpyridazine-3-carboxamide |
| | ED06-77-01R | ED06-77 | 6-[4-[[4-[4-[3-(Dimethylcarbamoyl)-4-hydroxynaphthalen-1-yl]phenyl]-3-methylphenyl]methyl]piperazin-1-yl]-N-propylpyridazine-3-carboxamide |
| | ED06-78-01R | ED06-78 | 6-[4-[[4-[6-[3-(Dimethylcarbamoyl)-4-hydroxynaphthalen-1-yl]pyridin-3-yl]-3-methylphenyl]methyl]piperazin-1-yl]-N-propylpyridazine-3-carboxamide |
| | ED06-79-01R | ED06-79 | 6-[4-[[4-[4-[3-(Dimethylcarbamoyl)-4-hydroxynaphthalen-1-yl]phenyl]-2-ethylphenyl]methyl]piperazin-1-yl]-N-propylpyridazine-3-carboxamide |
| | ED06-80-01R | ED06-80 | 6-[4-[[4-[6-[3-(Dimethylcarbamoyl)-4-hydroxynaphthalen-1-yl]pyridin-3-yl]-2-ethylphenyl]methyl]piperazin-1-yl]-N-propylpyridazine-3-carboxamide |
| | ED06-81-01R | ED06-81 | 6-[4-[[4-[4-[3-(Dimethylcarbamoyl)-4-hydroxynaphthalen-1-yl]phenyl]-3-fluorophenyl]methyl]piperazin-1-yl]-N-propylpyridazine-3-carboxamide |
| | ED06-82-01R | ED06-82 | 6-[4-[[4-[6-[3-(Dimethylcarbamoyl)-4-hydroxynaphthalen-1-yl]pyridin-3-yl]-3-fluorophenyl]methyl]piperazin-1-yl]-N-propylpyridazine-3-carboxamide |
| | ED06-83-01R | ED06-83 | 6-[4-[[4-[4-[3-(Dimethylcarbamoyl)-4-hydroxynaphthalen-1-yl]phenyl]-2-methoxyphenyl]methyl]piperazin-1-yl]-N-propylpyridazine-3-carboxamide |
| | ED06-84-01R | ED06-84 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-propylpyridazine-3-carboxamide |
| | ED06-85-01R | ED06-85 | 6-[4-[[3-[4-[3-(Dimethylcarbamoyl)-4-hydroxynaphthalen-1-yl]phenyl]-5-ethoxyphenyl]methyl]piperazin-1-yl]-N-propylpyridazine-3-carboxamide |
| | ED06-86-01R | ED06-86 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-propylpyridazine-3-carboxamide |
| | ED06-87-01R | ED06-87 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-propylpyridazine-3-carboxamide |
| | ED06-88-01R | ED06-88 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-propylpyridazine-3-carboxamide |
| mixED06-01R | ED06-89-01R | ED06-89 | 6-[4-[[4-[4-[3-(Dimethylcarbamoyl)-4-hydroxynaphthalen-1-yl]phenyl]-2,6-dimethoxyphenyl]methyl]piperazin-1-yl]-N-propylpyridazine-3-carboxamide |
| | ED06-90-01R | ED06-90 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-propylpyridazine-3-carboxamide |
| | ED06-91-01R | ED06-91 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-propylpyridazine-3-carboxamide |
| | ED06-92-01R | ED06-92 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-propylpyridazine-3-carboxamide |
| | ED06-93-01R | ED06-93 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-propylpyridazine-3-carboxamide |
| | ED06-94-01R | ED06-94 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-propylpyridazine-3-carboxamide |
| | ED06-95-01R | ED06-95 | 6-[4-[[4-[4-[3-(Dimethylcarbamoyl)-4-hydroxynaphthalen-1-yl]phenyl]naphthalen-1-yl]methyl]piperazin-1-yl]-N-propylpyridazine-3-carboxamide |
| | ED06-96-01R | ED06-96 | 6-[4-[[4-[6-[3-(Dimethylcarbamoyl)-4-hydroxynaphthalen-1-yl]pyridin-3-yl]naphthalen-1-yl]methyl]piperazin-1-yl]-N-propylpyridazine-3-carboxamide |

| | | | |
|---|---|---|---|
| | ED06-97-01R | ED06-97 | 6-[4-[[4-[4-[3-(Dimethylcarbamoyl)-4-hydroxynaphthalen-1-yl]phenyl]thiophen-2-yl]methyl]piperazin-1-yl]-N-propylpyridazine-3-carboxamide |
| | ED06-98-01R | ED06-98 | 6-[4-[[4-[6-[3-(Dimethylcarbamoyl)-4-hydroxynaphthalen-1-yl]pyridin-3-yl]thiophen-2-yl]methyl]piperazin-1-yl]-N-propylpyridazine-3-carboxamide |
| | ED06-99-01R | ED06-99 | 6-[4-[[2-[2-[4-[3-(Dimethylcarbamoyl)-4-hydroxynaphthalen-1-yl]phenyl]ethynyl]phenyl]methyl]piperazin-1-yl]-N-propylpyridazine-3-carboxamide |
| | ED06-100-01R | ED06-100 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-propylpyridazine-3-carboxamide |

[Table 106]

[Table ED07]

| No. of mixture | No. of compound that may be contained | Compound No. after cleavage | Compound name after cleavage |
|---|---|---|---|
| mixED07-01R | ED07-01-01R | ED07-01 | 1-Hydroxy-N,N-dimethyl-4-[4-[4-[1-[4-[4-(2,2,2-trifluoroethylcarbamoyl)phenyl]piperazin-1-yl]ethyl]phenyl]phenyl]naphthalene-2-carboxamide |
| mixED07-01R | ED07-02-01R | ED07-02 | 1-Hydroxy-N,N-dimethyl-4-[5-[4-[1-[4-[4-(2,2,2-trifluoroethylcarbamoyl)phenyl]piperazin-1-yl]ethyl]phenyl]pyridin-2-yl]naphthalene-2-carboxamide |
| | ED07-03-01R | ED07-03 | 4-[4-[3-Fluoro-4-[1-[4-[4-(2,2,2-trifluoroethylcarbamoyl)phenyl]piperazin-1-yl]ethyl]phenyl]phenyl]-1-hydroxy-N,N-dimethylnaphthalene-2-carboxamide |
| | ED07-04-01R | ED07-04 | 4-[5-[3-Fluoro-4-[1-[4-[4-(2,2,2-trifluoroethylcarbamoyl)phenyl]piperazin-1-yl]ethyl]phenyl]pyridin-2-yl]-1-hydroxy-N,N-dimethylnaphthalene-2-carboxamide |
| | ED07-05-01R | ED07-05 | 6-[4-[4-[4-[3-(Dimethylcarbamoyl)-4-hydroxynaphthalen-1-yl]phenyl]-3-methylbenzoyl]piperazin-1-yl]-N-(2,2,2-trifluoroethyl)pyridazine-3-carboxamide |
| | ED07-06-01R | ED07-06 | 6-[4-[4-[6-[3-(Dimethylcarbamoyl)-4-hydroxynaphthalen-1-yl]pyridin-3-yl]-3-methylbenzoyl]piperazin-1-yl]-N-(2,2,2-trifluoroethyl)pyridazine-3-carboxamide |
| | ED07-07-01R | ED07-07 | 6-[4-[4-[4-[3-(Dimethylcarbamoyl)-4-hydroxynaphthalen-1-yl]phenyl]-3-fluorobenzoyl]piperazin-1-yl]-N-(2,2,2-trifluoroethyl)pyridazine-3-carboxamide |
| | ED07-08-01R | ED07-08 | 6-[4-[4-[6-[3-(Dimethylcarbamoyl)-4-hydroxynaphthalen-1-yl]pyridin-3-yl]-3-fluorobenzoyl]piperazin-1-yl]-N-(2,2,2-trifluoroethyl)pyridazine-3-carboxamide |
| | ED07-09-01R | ED07-09 | 6-[4-[3-[4-[3-(Dimethylcarbamoyl)-4-hydroxynaphthalen-1-yl]phenyl]-5-(trifluoromethyl)benzoyl]piperazin-1-yl]-N-(2,2,2-trifluoroethyl)pyridazine-3-carboxamide |
| | ED07-10-01R | ED07-10 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-(2,2,2-trifluoroethyl)pyridazine-3-carboxamide |
| | ED07-11-01R | ED07-11 | 6-[4-[3-tert-Butyl-5-[4-[3-(dimethylcarbamoyl)-4-hydroxynaphthalen-1-yl]phenyl]benzoyl]piperazin-1-yl]-N-(2,2,2-trifluoroethyl)pyridazine-3-carboxamide |
| | ED07-12-01R | ED07-12 | 6-[4-[3-tert-Butyl-5-[6-[3-(dimethylcarbamoyl)-4-hydroxynaphthalen-1-yl]pyridin-3-yl]benzoyl]piperazin-1-yl]-N-(2,2,2-trifluoroethyl)pyridazine-3-carboxamide |
| | ED07-13-01R | ED07-13 | 6-[4-[4-[4-[3-(Dimethylcarbamoyl)-4-hydroxynaphthalen-1-yl]phenyl]naphthalene-1-carbonyl]piperazin-1-yl]-N-(2,2,2-trifluoroethyl)pyridazine-3-carboxamide |

| | | | |
|---|---|---|---|
| | ED07-14-01R | ED07-14 | 6-[4-[4-[6-[3-(Dimethylcarbamoyl)-4-hydroxynaphthalen-1-yl]pyridin-3-yl]naphthalene-1-carbonyl]piperazin-1-yl]-N-(2,2,2-trifluoroethyl)pyridazine-3-carboxamide |
| | ED07-15-01R | ED07-15 | 6-[4-[3-[2-[4-[3-(Dimethylcarbamoyl)-4-hydroxynaphthalen-1-yl]phenyl]phenyl]propanoyl]piperazin-1-yl]-N-(2,2,2-trifluoroethyl)pyridazine-3-carboxamide |
| | ED07-16-01R | ED07-16 | 6-[4-[3-[2-[6-[3-(Dimethylcarbamoyl)-4-hydroxynaphthalen-1-yl]pyridin-3-yl]phenyl]propanoyl]piperazin-1-yl]-N-(2,2,2-trifluoroethyl)pyridazine-3-carboxamide |
| | ED07-17-01R | ED07-17 | 6-[4-[4-[4-[3-(Dimethylcarbamoyl)-4-hydroxynaphthalen-1-yl]phenyl]thiophene-2-carbonyl]piperazin-1-yl]-N-(2,2,2-trifluoroethyl)pyridazine-3-carboxamide |
| mixED07-01R | ED07-18-01R | ED07-18 | 6-[4-[4-[6-[3-(Dimethylcarbamoyl)-4-hydroxynaphthalen-1-yl]pyridin-3-yl]thiophene-2-carbonyl]piperazin-1-yl]-N-(2,2,2-trifluoroethyl)pyridazine-3-carboxamide |
| | ED07-19-01R | ED07-19 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-(2,2,2-trifluoroethyl)pyridazine-3-carboxamide |
| | ED07-20-01R | ED07-20 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-(2,2,2-trifluoroethyl)pyridazine-3-carboxamide |
| | ED07-21-01R | ED07-21 | 6-[4-[2-[2-[4-[3-(Dimethylcarbamoyl)-4-hydroxynaphthalen-1-yl]phenyl]ethynyl]benzoyl]piperazin-1-yl]-N-(2,2,2-trifluoroethyl)pyridazine-3-carboxamide |
| | ED07-22-01R | ED07-22 | 6-[4-[2-[2-[6-[3-(Dimethylcarbamoyl)-4-hydroxynaphthalen-1-yl]pyridin-3-yl]ethynyl]benzoyl]piperazin-1-yl]-N-(2,2,2-trifluoroethyl)pyridazine-3-carboxamide |
| | ED07-23-01R | ED07-23 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-(2,2,2-trifluoroethyl)pyridazine-3-carboxamide |
| | ED07-24-01R | ED07-24 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-(2,2,2-trifluoroethyl)pyridazine-3-carboxamide |
| | ED07-25-01R | ED07-25 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-(2,2,2-trifluoroethyl)pyridazine-3-carboxamide |
| | ED07-26-01R | ED07-26 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-(2,2,2-trifluoroethyl)pyridazine-3-carboxamide |
| | ED07-27-01R | ED07-27 | 1-Hydroxy-N,N-dimethyl-4-[4-[2-methyl-4-[4-[4-(2,2,2-trifluoroethylcarbamoyl)phenyl]piperazine-1-carbonyl]phenyl]phenyl]naphthalene-2-carboxamide |
| | ED07-28-01R | ED07-28 | 1-Hydroxy-N,N-dimethyl-4-[5-[2-methyl-4-[4-[4-(2,2,2-trifluoroethylcarbamoyl)phenyl]piperazine-1-carbonyl]phenyl]pyridin-2-yl]naphthalene-2-carboxamide |
| | ED07-29-01R | ED07-29 | 4-[4-[2-Fluoro-4-[4-[4-(2,2,2-trifluoroethylcarbamoyl)phenyl]piperazine-1-carbonyl]phenyl]phenyl]-1-hydroxy-N,N-dimethylnaphthalene-2-carboxamide |
| | ED07-30-01R | ED07-30 | 4-[5-[2-Fluoro-4-[4-[4-(2,2,2-trifluoroethylcarbamoyl)phenyl]piperazine-1-carbonyl]phenyl]pyridin-2-yl]-1-hydroxy-N,N-dimethylnaphthalene-2-carboxamide |
| | ED07-31-01R | ED07-31 | 1-Hydroxy-N,N-dimethyl-4-[4-[3-[4-[4-(2,2,2-trifluoroethylcarbamoyl)phenyl]piperazine-1-carbonyl]-5-(trifluoromethyl)phenyl]phenyl]naphthalene-2-carboxamide |
| | ED07-32-01R | ED07-32 | 1-Hydroxy-N,N-dimethyl-4-[5-[3-[4-[4-(2,2,2-trifluoroethylcarbamoyl)phenyl]piperazine-1-carbonyl]-5-(trifluoromethyl)phenyl]pyridin-2-yl]naphthalene-2-carboxamide |

| | | | |
|---|---|---|---|
| mixED07-01R | ED07-33-01R | ED07-33 | 4-[4-[3-tert-Butyl-5-[4-[4-(2,2,2-trifluoroethylcarbamoyl)phenyl]piperazine-1-carbonyl]phenyl]phenyl]-1-hydroxy-N,N-dimethylnaphthalene-2-carboxamide |
| | ED07-34-01R | ED07-34 | 4-[5-[3-tert-Butyl-5-[4-[4-(2,2,2-trifluoroethylcarbamoyl)phenyl]piperazine-1-carbonyl]phenyl]pyridin-2-yl]-1-hydroxy-N,N-dimethylnaphthalene-2-carboxamide |
| | ED07-35-01R | ED07-35 | 1-Hydroxy-N,N-dimethyl-4-[4-[4-[4-[4-(2,2,2-trifluoroethylcarbamoyl)phenyl]piperazine-1-carbonyl]naphthalen-1-yl]phenyl]naphthalene-2-carboxamide |
| | ED07-36-01R | ED07-36 | 1-Hydroxy-N,N-dimethyl-4-[5-[4-[4-[4-(2,2,2-trifluoroethylcarbamoyl)phenyl]piperazine-1-carbonyl]naphthalen-1-yl]pyridin-2-yl]naphthalene-2-carboxamide |
| | ED07-37-01R | ED07-37 | 1-Hydroxy-N,N-dimethyl-4-[4-[2-[3-oxo-3-[4-[4-(2,2,2-trifluoroethylcarbamoyl)phenyl]piperazin-1-yl]propyl]phenyl]phenyl]naphthalene-2-carboxamide |
| | ED07-38-01R | ED07-38 | 1-Hydroxy-N,N-dimethyl-4-[5-[2-[3-oxo-3-[4-[4-(2,2,2-trifluoroethylcarbamoyl)phenyl]piperazin-1-yl]propyl]phenyl]pyridin-2-yl]naphthalene-2-carboxamide |
| | ED07-39-01R | ED07-39 | 1-Hydroxy-N,N-dimethyl-4-[4-[5-[4-[4-(2,2,2-trifluoroethylcarbamoyl)phenyl]piperazine-1-carbonyl]thiophen-3-yl]phenyl]naphthalene-2-carboxamide |
| | ED07-40-01R | ED07-40 | 1-Hydroxy-N,N-dimethyl-4-[5-[5-[4-[4-(2,2,2-trifluoroethylcarbamoyl)phenyl]piperazine-1-carbonyl]thiophen-3-yl]pyridin-2-yl]naphthalene-2-carboxamide |
| | ED07-41-01R | ED07-41 | 1-Hydroxy-N,N-dimethyl-4-[4-[1-[3-[4-[4-(2,2,2-trifluoroethylcarbamoyl)phenyl]piperazine-1-carbonyl]-5-(trifluoromethyl)phenyl]pyrazol-4-yl]phenyl]naphthalene-2-carboxamide |
| | ED07-42-01R | ED07-42 | 1-Hydroxy-N,N-dimethyl-4-[5-[1-[3-[4-[4-(2,2,2-trifluoroethylcarbamoyl)phenyl]piperazine-1-carbonyl]-5-(trifluoromethyl)phenyl]pyrazol-4-yl]pyridin-2-yl]naphthalene-2-carboxamide |
| | ED07-43-01R | ED07-43 | 1-Hydroxy-N,N-dimethyl-4-[4-[2-[2-[4-[4-(2,2,2-trifluoroethylcarbamoyl)phenyl]piperazine-1-carbonyl]phenyl]ethynyl]phenyl]naphthalene-2-carboxamide |
| | ED07-44-01R | ED07-44 | 1-Hydroxy-N,N-dimethyl-4-[5-[2-[2-[4-[4-(2,2,2-trifluoroethylcarbamoyl)phenyl]piperazine-1-carbonyl]phenyl]ethynyl]pyridin-2-yl]naphthalene-2-carboxamide |
| | ED07-45-01R | ED07-45 | 1-Hydroxy-N,N-dimethyl-4-[4-[2-[3-[4-[4-(2,2,2-trifluoroethylcarbamoyl)phenyl]piperazine-1-carbonyl]-5-(trifluoromethyl)phenyl]ethynyl]phenyl]naphthalene-2-carboxamide |
| | ED07-46-01R | ED07-46 | 1-Hydroxy-N,N-dimethyl-4-[5-[2-[3-[4-[4-(2,2,2-trifluoroethylcarbamoyl)phenyl]piperazine-1-carbonyl]-5-(trifluoromethyl)phenyl]ethynyl]pyridin-2-yl]naphthalene-2-carboxamide |
| | ED07-47-01R | ED07-47 | 1-Hydroxy-N,N-dimethyl-4-[4-[2-[5-[4-[4-(2,2,2-trifluoroethylcarbamoyl)phenyl]piperazine-1-carbonyl]pyridin-3-yl]ethynyl]phenyl]naphthalene-2-carboxamide |
| mixED07-01R | ED07-48-01R | ED07-48 | 1-Hydroxy-N,N-dimethyl-4-[5-[2-[5-[4-[4-(2,2,2-trifluoroethylcarbamoyl)phenyl]piperazine-1-carbonyl]pyridin-3-yl]ethynyl]pyridin-2-yl]naphthalene-2-carboxamide |
| | ED07-49-01R | ED07-49 | 1-Hydroxy-N,N-dimethyl-4-[4-[2-[[4-[4-(2,2,2-trifluoroethylcarbamoyl)phenyl]piperazin-1-yl]methyl]phenyl]phenyl]naphthalene-2-carboxamide |
| | ED07-50-01R | ED07-50 | 1-Hydroxy-N,N-dimethyl-4-[5-[2-[[4-[4-(2,2,2-trifluoroethylcarbamoyl)phenyl]piperazin-1-yl]methyl]phenyl]pyridin-2-yl]naphthalene-2-carboxamide |

| | | | |
|---|---|---|---|
| | ED07-51-01R | ED07-51 | 1-Hydroxy-N,N-dimethyl-4-[4-[2-methyl-4-[[4-[4-(2,2,2-trifluoroethylcarbamoyl)phenyl]piperazin-1-yl]methyl]phenyl]phenyl]naphthalene-2-carboxamide |
| | ED07-52-01R | ED07-52 | 1-Hydroxy-N,N-dimethyl-4-[5-[2-methyl-4-[[4-[4-(2,2,2-trifluoroethylcarbamoyl)phenyl]piperazin-1-yl]methyl]phenyl]pyridin-2-yl]naphthalene-2-carboxamide |
| | ED07-53-01R | ED07-53 | 4-[4-[3-Ethyl-4-[[4-[4-(2,2,2-trifluoroethylcarbamoyl)phenyl]piperazin-1-yl]methyl]phenyl]phenyl]-1-hydroxy-N,N-dimethylnaphthalene-2-carboxamide |
| | ED07-54-01R | ED07-54 | 4-[5-[3-Ethyl-4-[[4-[4-(2,2,2-trifluoroethylcarbamoyl)phenyl]piperazin-1-yl]methyl]phenyl]pyridin-2-yl]-1-hydroxy-N,N-dimethylnaphthalene-2-carboxamide |
| | ED07-55-01R | ED07-55 | 4-[4-[2-Fluoro-4-[[4-[4-(2,2,2-trifluoroethylcarbamoyl)phenyl]piperazin-1-yl]methyl]phenyl]phenyl]-1-hydroxy-N,N-dimethylnaphthalene-2-carboxamide |
| | ED07-56-01R | ED07-56 | 4-[5-[2-Fluoro-4-[[4-[4-(2,2,2-trifluoroethylcarbamoyl)phenyl]piperazin-1-yl]methyl]phenyl]pyridin-2-yl]-1-hydroxy-N,N-dimethylnaphthalene-2-carboxamide |
| | ED07-57-01R | ED07-57 | 1-Hydroxy-4-[4-[3-methoxy-4-[[4-[4-(2,2,2-trifluoroethylcarbamoyl)phenyl]piperazin-1-yl]methyl]phenyl]phenyl]-N,N-dimethylnaphthalene-2-carboxamide |
| | ED07-58-01R | ED07-58 | 1-Hydroxy-4-[5-[3-methoxy-4-[[4-[4-(2,2,2-trifluoroethylcarbamoyl)phenyl]piperazin-1-yl]methyl]phenyl]pyridin-2-yl]-N,N-dimethylnaphthalene-2-carboxamide |
| | ED07-59-01R | ED07-59 | 4-[4-[3-Ethoxy-5-[[4-[4-(2,2,2-trifluoroethylcarbamoyl)phenyl]piperazin-1-yl]methyl]phenyl]phenyl]-1-hydroxy-N,N-dimethylnaphthalene-2-carboxamide |
| | ED07-60-01R | ED07-60 | 4-[5-[3-Ethoxy-5-[[4-[4-(2,2,2-trifluoroethylcarbamoyl)phenyl]piperazin-1-yl]methyl]phenyl]pyridin-2-yl]-1-hydroxy-N,N-dimethylnaphthalene-2-carboxamide |
| | ED07-61-01R | ED07-61 | 1-Hydroxy-N,N-dimethyl-4-[4-[3-propan-2-yloxy-5-[[4-[4-(2,2,2-trifluoroethylcarbamoyl)phenyl]piperazin-1-yl]methyl]phenyl]phenyl]naphthalene-2-carboxamide |
| mixED07-01R | ED07-62-01R | ED07-62 | 1-Hydroxy-N,N-dimethyl-4-[5-[3-propan-2-yloxy-5-[[4-[4-(2,2,2-trifluoroethylcarbamoyl)phenyl]piperazin-1-yl]methyl]phenyl]pyridin-2-yl]naphthalene-2-carboxamide |
| | ED07-63-01R | ED07-63 | 4-[4-[3,5-Dimethoxy-4-[[4-[4-(2,2,2-trifluoroethylcarbamoyl)phenyl]piperazin-1-yl]methyl]phenyl]phenyl]-1-hydroxy-N,N-dimethylnaphthalene-2-carboxamide |
| | ED07-64-01R | ED07-64 | 4-[5-[3,5-Dimethoxy-4-[[4-[4-(2,2,2-trifluoroethylcarbamoyl)phenyl]piperazin-1-yl]methyl]phenyl]pyridin-2-yl]-1-hydroxy-N,N-dimethylnaphthalene-2-carboxamide |
| | ED07-65-01R | ED07-65 | 1-Hydroxy-N,N-dimethyl-4-[4-[3-[[4-[4-(2,2,2-trifluoroethylcarbamoyl)phenyl]piperazin-1-yl]methyl]-5-(trifluoromethyl)phenyl]phenyl]naphthalene-2-carboxamide |
| | ED07-66-01R | ED07-66 | 1-Hydroxy-N,N-dimethyl-4-[5-[3-[[4-[4-(2,2,2-trifluoroethylcarbamoyl)phenyl]piperazin-1-yl]methyl]-5-(trifluoromethyl)phenyl]pyridin-2-yl]naphthalene-2-carboxamide |
| | ED07-67-01R | ED07-67 | 1-Hydroxy-N,N-dimethyl-4-[4-[3-[[4-[4-(2,2,2-trifluoroethylcarbamoyl)phenyl]piperazin-1-yl]methyl]-5-(trifluoromethoxy)phenyl]phenyl]naphthalene-2-carboxamide |
| | ED07-68-01R | ED07-68 | 1-Hydroxy-N,N-dimethyl-4-[5-[3-[[4-[4-(2,2,2-trifluoroethylcarbamoyl)phenyl]piperazin-1-yl]methyl]-5-(trifluoromethoxy)phenyl]pyridin-2-yl]naphthalene-2-carboxamide |
| | ED07-69-01R | ED07-69 | 1-Hydroxy-N,N-dimethyl-4-[4-[4-[[4-[4-(2,2,2-trifluoroethylcarbamoyl)phenyl]piperazin-1-yl]methyl]naphthalen-1-yl]phenyl]naphthalene-2-carboxamide |

| | | | |
|---|---|---|---|
| | ED07-70-01R | ED07-70 | 1-Hydroxy-N,N-dimethyl-4-[5-[4-[[4-[4-(2,2,2-trifluoroethylcarbamoyl)phenyl]piperazin-1-yl]methyl]naphthalen-1-yl]pyridin-2-yl]naphthalene-2-carboxamide |
| | ED07-71-01R | ED07-71 | 1-Hydroxy-N,N-dimethyl-4-[4-[5-[[4-[4-(2,2,2-trifluoroethylcarbamoyl)phenyl]piperazin-1-yl]methyl]thiophen-3-yl]phenyl]naphthalene-2-carboxamide |
| | ED07-72-01R | ED07-72 | 1-Hydroxy-N,N-dimethyl-4-[5-[5-[[4-[4-(2,2,2-trifluoroethylcarbamoyl)phenyl]piperazin-1-yl]methyl]thiophen-3-yl]pyridin-2-yl]naphthalene-2-carboxamide |
| | ED07-73-01R | ED07-73 | 1-Hydroxy-N,N-dimethyl-4-[4-[2-[2-[[4-[4-(2,2,2-trifluoroethylcarbamoyl)phenyl]piperazin-1-yl]methyl]phenyl]ethynyl]phenyl]naphthalene-2-carboxamide |
| | ED07-74-01R | ED07-74 | 1-Hydroxy-N,N-dimethyl-4-[5-[2-[2-[[4-[4-(2,2,2-trifluoroethylcarbamoyl)phenyl]piperazin-1-yl]methyl]phenyl]ethynyl]pyridin-2-yl]naphthalene-2-carboxamide |
| | ED07-75-01R | ED07-75 | 6-[4-[[2-[4-[3-(Dimethylcarbamoyl)-4-hydroxynaphthalen-1-yl]phenyl]phenyl]methyl]piperazin-1-yl]-N-(2,2,2-trifluoroethyl)pyridazine-3-carboxamide |
| | ED07-76-01R | ED07-76 | 6-[4-[[2-[6-[3-(Dimethylcarbamoyl)-4-hydroxynaphthalen-1-yl]pyridin-3-yl]phenyl]methyl]piperazin-1-yl]-N-(2,2,2-trifluoroethyl)pyridazine-3-carboxamide |
| mixED07-01R | ED07-77-01R | ED07-77 | 6-[4-[[4-[4-[3-(Dimethylcarbamoyl)-4-hydroxynaphthalen-1-yl]phenyl]-3-methylphenyl]methyl]piperazin-1-yl]-N-(2,2,2-trifluoroethyl)pyridazine-3-carboxamide |
| | ED07-78-01R | ED07-78 | 6-[4-[[4-[6-[3-(Dimethylcarbamoyl)-4-hydroxynaphthalen-1-yl]pyridin-3-yl]-3-methylphenyl]methyl]piperazin-1-yl]-N-(2,2,2-trifluoroethyl)pyridazine-3-carboxamide |
| | ED07-79-01R | ED07-79 | 6-[4-[[4-[4-[3-(Dimethylcarbamoyl)-4-hydroxynaphthalen-1-yl]phenyl]-2-ethylphenyl]methyl]piperazin-1-yl]-N-(2,2,2-trifluoroethyl)pyridazine-3-carboxamide |
| | ED07-80-01R | ED07-80 | 6-[4-[[4-[6-[3-(Dimethylcarbamoyl)-4-hydroxynaphthalen-1-yl]pyridin-3-yl]-2-ethylphenyl]methyl]piperazin-1-yl]-N-(2,2,2-trifluoroethyl)pyridazine-3-carboxamide |
| | ED07-81-01R | ED07-81 | 6-[4-[[4-[4-[3-(Dimethylcarbamoyl)-4-hydroxynaphthalen-1-yl]phenyl]-3-fluorophenyl]methyl]piperazin-1-yl]-N-(2,2,2-trifluoroethyl)pyridazine-3-carboxamide |
| | ED07-82-01R | ED07-82 | 6-[4-[[4-[6-[3-(Dimethylcarbamoyl)-4-hydroxynaphthalen-1-yl]pyridin-3-yl]-3-fluorophenyl]methyl]piperazin-1-yl]-N-(2,2,2-trifluoroethyl)pyridazine-3-carboxamide |
| | ED07-83-01R | ED07-83 | 6-[4-[[4-[4-[3-(Dimethylcarbamoyl)-4-hydroxynaphthalen-1-yl]phenyl]-2-methoxyphenyl]methyl]piperazin-1-yl]-N-(2,2,2-trifluoroethyl)pyridazine-3-carboxamide |
| | ED07-84-01R | ED07-84 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-(2,2,2-trifluoroethyl)pyridazine-3-carboxamide |
| | ED07-85-01R | ED07-85 | 6-[4-[[3-[4-[3-(Dimethylcarbamoyl)-4-hydroxynaphthalen-1-yl]phenyl]-5-ethoxyphenyl]methyl]piperazin-1-yl]-N-(2,2,2-trifluoroethyl)pyridazine-3-carboxamide |
| | ED07-86-01R | ED07-86 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-(2,2,2-trifluoroethyl)pyridazine-3-carboxamide |
| | ED07-87-01R | ED07-87 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-(2,2,2-trifluoroethyl)pyridazine-3-carboxamide |
| | ED07-88-01R | ED07-88 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-(2,2,2-trifluoroethyl)pyridazine-3-carboxamide |

| | | | |
|---|---|---|---|
| | ED07-89-01R | ED07-89 | 6-[4-[[4-[4-[3-(Dimethylcarbamoyl)-4-hydroxynaphthalen-1-yl]phenyl]-2,6-dimethoxyphenyl]methyl]piperazin-1-yl]-N-(2,2,2-trifluoroethyl)pyridazine-3-carboxamide |
| | ED07-90-01R | ED07-90 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-(2,2,2-trifluoroethyl)pyridazine-3-carboxamide |
| | ED07-91-01R | ED07-91 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-(2,2,2-trifluoroethyl)pyridazine-3-carboxamide |
| | ED07-92-01R | ED07-92 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-(2,2,2-trifluoroethyl)pyridazine-3-carboxamide |
| mixED07-01R | ED07-93-01R | ED07-93 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-(2,2,2-trifluoroethyl)pyridazine-3-carboxamide |
| | ED07-94-01R | ED07-94 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-(2,2,2-trifluoroethyl)pyridazine-3-carboxamide |
| | ED07-95-01R | ED07-95 | 6-[4-[[4-[4-[3-(Dimethylcarbamoyl)-4-hydroxynaphthalen-1-yl]phenyl]naphthalen-1-yl]methyl]piperazin-1-yl]-N-(2,2,2-trifluoroethyl)pyridazine-3-carboxamide |
| | ED07-96-01R | ED07-96 | 6-[4-[[4-[6-[3-(Dimethylcarbamoyl)-4-hydroxynaphthalen-1-yl]pyridin-3-yl]naphthalen-1-yl]methyl]piperazin-1-yl]-N-(2,2,2-trifluoroethyl)pyridazine-3-carboxamide |
| | ED07-97-01R | ED07-97 | 6-[4-[[4-[4-[3-(Dimethylcarbamoyl)-4-hydroxynaphthalen-1-yl]phenyl]thiophen-2-yl]methyl]piperazin-1-yl]-N-(2,2,2-trifluoroethyl)pyridazine-3-carboxamide |
| | ED07-98-01R | ED07-98 | 6-[4-[[4-[6-[3-(Dimethylcarbamoyl)-4-hydroxynaphthalen-1-yl]pyridin-3-yl]thiophen-2-yl]methyl]piperazin-1-yl]-N-(2,2,2-trifluoroethyl)pyridazine-3-carboxamide |
| | ED07-99-01R | ED07-99 | 6-[4-[[2-[2-[4-[3-(Dimethylcarbamoyl)-4-hydroxynaphthalen-1-yl]phenyl]ethynyl]phenyl]methyl]piperazin-1-yl]-N-(2,2,2-trifluoroethyl)pyridazine-3-carboxamide |
| | ED07-100-01R | ED07-100 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-(2,2,2-trifluoroethyl)pyridazine-3-carboxamide |

[Table 107]

[Table ED08]

| No. of mixture | No. of compound that may be contained | Compound No. after cleavage | Compound name after cleavage |
|---|---|---|---|
| mixED08-01R | ED08-01-01R | ED08-01 | 1-Hydroxy-4-[4-[4-[1-[4-[4-[(4-methoxyphenyl)carbamoyl]phenyl]piperazin-1-yl]ethyl]phenyl]phenyl]-N,N-dimethylnaphthalene-2-carboxamide |
| | ED08-02-01R | ED08-02 | 1-Hydroxy-4-[5-[4-[1-[4-[4-[(4-methoxyphenyl)carbamoyl]phenyl]piperazin-1-yl]ethyl]phenyl]pyridin-2-yl]-N,N-dimethylnaphthalene-2-carboxamide |
| | ED08-03-01R | ED08-03 | 4-[4-[3-Fluoro-4-[1-[4-[4-[(4-methoxyphenyl)carbamoyl]phenyl]piperazin-1-yl]ethyl]phenyl]phenyl]-1-hydroxy-N,N-dimethylnaphthalene-2-carboxamide |
| | ED08-04-01R | ED08-04 | 4-[5-[3-Fluoro-4-[1-[4-[4-[(4-methoxyphenyl)carbamoyl]phenyl]piperazin-1-yl]ethyl]phenyl]pyridin-2-yl]-1-hydroxy-N,N-dimethylnaphthalene-2-carboxamide |

| | | | |
|---|---|---|---|
| mixED08-01R | ED08-05-01R | ED08-05 | 6-[4-[4-[4-[3-(Dimethylcarbamoyl)-4-hydroxynaphthalen-1-yl]phenyl]-3-methylbenzoyl]piperazin-1-yl]-N-(4-methoxyphenyl)pyridazine-3-carboxamide |
| | ED08-06-01R | ED08-06 | 6-[4-[4-[6-[3-(Dimethylcarbamoyl)-4-hydroxynaphthalen-1-yl]pyridin-3-yl]-3-methylbenzoyl]piperazin-1-yl]-N-(4-methoxyphenyl)pyridazine-3-carboxamide |
| | ED08-07-01R | ED08-07 | 6-[4-[4-[4-[3-(Dimethylcarbamoyl)-4-hydroxynaphthalen-1-yl]phenyl]-3-fluorobenzoyl]piperazin-1-yl]-N-(4-methoxyphenyl)pyridazine-3-carboxamide |
| | ED08-08-01R | ED08-08 | 6-[4-[4-[6-[3-(Dimethylcarbamoyl)-4-hydroxynaphthalen-1-yl]pyridin-3-yl]-3-fluorobenzoyl]piperazin-1-yl]-N-(4-methoxyphenyl)pyridazine-3-carboxamide |
| | ED08-09-01R | ED08-09 | 6-[4-[3-[4-[3-(Dimethylcarbamoyl)-4-hydroxynaphthalen-1-yl]phenyl]-5-(trifluoromethyl)benzoyl]piperazin-1-yl]-N-(4-methoxyphenyl)pyridazine-3-carboxamide |
| | ED08-10-01R | ED08-10 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-(4-methoxyphenyl)pyridazine-3-carboxamide |
| | ED08-11-01R | ED08-11 | 6-[4-[3-tert-Butyl-5-[4-[3-(dimethylcarbamoyl)-4-hydroxynaphthalen-1-yl]phenyl]benzoyl]piperazin-1-yl]-N-(4-methoxyphenyl)pyridazine-3-carboxamide |
| | ED08-12-01R | ED08-12 | 6-[4-[3-tert-Butyl-5-[6-[3-(dimethylcarbamoyl)-4-hydroxynaphthalen-1-yl]pyridin-3-yl]benzoyl]piperazin-1-yl]-N-(4-methoxyphenyl)pyridazine-3-carboxamide |
| | ED08-13-01R | ED08-13 | 6-[4-[4-[4-[3-(Dimethylcarbamoyl)-4-hydroxynaphthalen-1-yl]phenyl]naphthalene-1-carbonyl]piperazin-1-yl]-N-(4-methoxyphenyl)pyridazine-3-carboxamide |
| | ED08-14-01R | ED08-14 | 6-[4-[4-[6-[3-(Dimethylcarbamoyl)-4-hydroxynaphthalen-1-yl]pyridin-3-yl]naphthalene-1-carbonyl]piperazin-1-yl]-N-(4-methoxyphenyl)pyridazine-3-carboxamide |
| | ED08-15-01R | ED08-15 | 6-[4-[3-[2-[4-[3-(Dimethylcarbamoyl)-4-hydroxynaphthalen-1-yl]phenyl]phenyl]propanoyl]piperazin-1-yl]-N-(4-methoxyphenyl)pyridazine-3-carboxamide |
| | ED08-16-01R | ED08-16 | 6-[4-[3-[2-[6-[3-(Dimethylcarbamoyl)-4-hydroxynaphthalen-1-yl]pyridin-3-yl]phenyl]propanoyl]piperazin-1-yl]-N-(4-methoxyphenyl)pyridazine-3-carboxamide |
| | ED08-17-01R | ED08-17 | 6-[4-[4-[4-[3-(Dimethylcarbamoyl)-4-hydroxynaphthalen-1-yl]phenyl]thiophene-2-carbonyl]piperazin-1-yl]-N-(4-methoxyphenyl)pyridazine-3-carboxamide |
| | ED08-18-01R | ED08-18 | 6-[4-[4-[6-[3-(Dimethylcarbamoyl)-4-hydroxynaphthalen-1-yl]pyridin-3-yl]thiophene-2-carbonyl]piperazin-1-yl]-N-(4-methoxyphenyl)pyridazine-3-carboxamide |
| mixED08-01R | ED08-19-01R | ED08-19 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-(4-methoxyphenyl)pyridazine-3-carboxamide |
| | ED08-20-01R | ED08-20 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-(4-methoxyphenyl)pyridazine-3-carboxamide |
| | ED08-21-01R | ED08-21 | 6-[4-[2-[2-[4-[3-(Dimethylcarbamoyl)-4-hydroxynaphthalen-1-yl]phenyl]ethynyl]benzoyl]piperazin-1-yl]-N-(4-methoxyphenyl)pyridazine-3-carboxamide |
| | ED08-22-01R | ED08-22 | 6-[4-[2-[2-[6-[3-(Dimethylcarbamoyl)-4-hydroxynaphthalen-1-yl]pyridin-3-yl]ethynyl]benzoyl]piperazin-1-yl]-N-(4-methoxyphenyl)pyridazine-3-carboxamide |
| | ED08-23-01R | ED08-23 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-(4-methoxyphenyl)pyridazine-3-carboxamide |

| | | | |
|---|---|---|---|
| | ED08-24-01R | ED08-24 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-(4-methoxyphenyl)pyridazine-3-carboxamide |
| | ED08-25-01R | ED08-25 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-(4-methoxyphenyl)pyridazine-3-carboxamide |
| | ED08-26-01R | ED08-26 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-(4-methoxyphenyl)pyridazine-3-carboxamide |
| | ED08-27-01R | ED08-27 | 1-Hydroxy-4-[4-[4-[4-[4-[(4-methoxyphenyl)carbamoyl]phenyl]piperazine-1-carbonyl]-2-methylphenyl]phenyl]-N,N-dimethylnaphthalene-2-carboxamide |
| | ED08-28-01R | ED08-28 | 1-Hydroxy-4-[5-[4-[4-[4-[(4-methoxyphenyl)carbamoyl]phenyl]piperazine-1-carbonyl]-2-methylphenyl]pyridin-2-yl]-N,N-dimethylnaphthalene-2-carboxamide |
| | ED08-29-01R | ED08-29 | 4-[4-[2-Fluoro-4-[4-[4-[(4-methoxyphenyl)carbamoyl]phenyl]piperazine-1-carbonyl]phenyl]phenyl]-1-hydroxy-N,N-dimethylnaphthalene-2-carboxamide |
| | ED08-30-01R | ED08-30 | 4-[5-[2-Fluoro-4-[4-[4-[(4-methoxyphenyl)carbamoyl]phenyl]piperazine-1-carbonyl]phenyl]pyridin-2-yl]-1-hydroxy-N,N-dimethylnaphthalene-2-carboxamide |
| | ED08-31-01R | ED08-31 | 1-Hydroxy-4-[4-[3-[4-[4-[(4-methoxyphenyl)carbamoyl]phenyl]piperazine-1-carbonyl]-5-(trifluoromethyl)phenyl]phenyl]-N,N-dimethylnaphthalene-2-carboxamide |
| | ED08-32-01R | ED08-32 | 1-Hydroxy-4-[5-[3-[4-[4-[(4-methoxyphenyl)carbamoyl]phenyl]piperazine-1-carbonyl]-5-(trifluoromethyl)phenyl]pyridin-2-yl]-N,N-dimethylnaphthalene-2-carboxamide |
| mixED08-01R | ED08-33-01R | ED08-33 | 4-[4-[3-tert-Butyl-5-[4-[4-[(4-methoxyphenyl)carbamoyl]phenyl]piperazine-1-carbonyl]phenyl]phenyl]-1-hydroxy-N,N-dimethylnaphthalene-2-carboxamide |
| | ED08-34-01R | ED08-34 | 4-[5-[3-tert-Butyl-5-[4-[4-[(4-methoxyphenyl)carbamoyl]phenyl]piperazine-1-carbonyl]phenyl]pyridin-2-yl]-1-hydroxy-N,N-dimethylnaphthalene-2-carboxamide |
| | ED08-35-01R | ED08-35 | 1-Hydroxy-4-[4-[4-[4-[4-[(4-methoxyphenyl)carbamoyl]phenyl]piperazine-1-carbonyl]naphthalen-1-yl]phenyl]-N,N-dimethylnaphthalene-2-carboxamide |
| | ED08-36-01R | ED08-36 | 1-Hydroxy-4-[5-[4-[4-[4-[(4-methoxyphenyl)carbamoyl]phenyl]piperazine-1-carbonyl]naphthalen-1-yl]pyridin-2-yl]-N,N-dimethylnaphthalene-2-carboxamide |
| | ED08-37-01R | ED08-37 | 1-Hydroxy-4-[4-[2-[3-[4-[4-[(4-methoxyphenyl)carbamoyl]phenyl]piperazin-1-yl]-3-oxopropyl]phenyl]phenyl]-N,N-dimethylnaphthalene-2-carboxamide |
| | ED08-38-01R | ED08-38 | 1-Hydroxy-4-[5-[2-[3-[4-[4-[(4-methoxyphenyl)carbamoyl]phenyl]piperazin-1-yl]-3-oxopropyl]phenyl]pyridin-2-yl]-N,N-dimethylnaphthalene-2-carboxamide |
| | ED08-39-01R | ED08-39 | 1-Hydroxy-4-[4-[5-[4-[4-[(4-methoxyphenyl)carbamoyl]phenyl]piperazine-1-carbonyl]thiophen-3-yl]phenyl]-N,N-dimethylnaphthalene-2-carboxamide |
| | ED08-40-01R | ED08-40 | 1-Hydroxy-4-[5-[5-[4-[4-[(4-methoxyphenyl)carbamoyl]phenyl]piperazine-1-carbonyl]thiophen-3-yl]pyridin-2-yl]-N,N-dimethylnaphthalene-2-carboxamide |
| | ED08-41-01R | ED08-41 | 1-Hydroxy-4-[4-[1-[3-[4-[4-[(4-methoxyphenyl)carbamoyl]phenyl]piperazine-1-carbonyl]-5-(trifluoromethyl)phenyl]pyrazol-4-yl]phenyl]-N,N-dimethylnaphthalene-2-carboxamide |

| | | | |
|---|---|---|---|
| | ED08-42-01R | ED08-42 | 1-Hydroxy-4-[5-[1-[3-[4-[4-[(4-methoxyphenyl)carbamoyl]phenyl]piperazine-1-carbonyl]-5-(trifluoromethyl)phenyl]pyrazol-4-yl]pyridin-2-yl]-N,N-dimethylnaphthalene-2-carboxamide |
| | ED08-43-01R | ED08-43 | 1-Hydroxy-4-[4-[2-[2-[4-[4-[(4-methoxyphenyl)carbamoyl]phenyl]piperazine-1-carbonyl]phenyl]ethynyl]phenyl]-N,N-dimethylnaphthalene-2-carboxamide |
| | ED08-44-01R | ED08-44 | 1-Hydroxy-4-[5-[2-[2-[4-[4-[(4-methoxyphenyl)carbamoyl]phenyl]piperazine-1-carbonyl]phenyl]ethynyl]pyridin-2-yl]-N,N-dimethylnaphthalene-2-carboxamide |
| | ED08-45-01R | ED08-45 | 1-Hydroxy-4-[4-[2-[3-[4-[4-[(4-methoxyphenyl)carbamoyl]phenyl]piperazine-1-carbonyl]-5-(trifluoromethyl)phenyl]ethynyl]phenyl]-N,N-dimethylnaphthalene-2-carboxamide |
| | ED08-46-01R | ED08-46 | 1-Hydroxy-4-[5-[2-[3-[4-[4-[(4-methoxyphenyl)carbamoyl]phenyl]piperazine-1-carbonyl]-5-(trifluoromethyl)phenyl]ethynyl]pyridin-2-yl]-N,N-dimethylnaphthalene-2-carboxamide |
| mixED08-01R | ED08-47-01R | ED08-47 | 1-Hydroxy-4-[4-[2-[5-[4-[4-[(4-methoxyphenyl)carbamoyl]phenyl]piperazine-1-carbonyl]pyridin-3-yl]ethynyl]phenyl]-N,N-dimethylnaphthalene-2-carboxamide |
| | ED08-48-01R | ED08-48 | 1-Hydroxy-4-[5-[2-[5-[4-[4-[(4-methoxyphenyl)carbamoyl]phenyl]piperazine-1-carbonyl]pyridin-3-yl]ethynyl]pyridin-2-yl]-N,N-dimethylnaphthalene-2-carboxamide |
| | ED08-49-01R | ED08-49 | 1-Hydroxy-4-[4-[2-[[4-[4-[(4-methoxyphenyl)carbamoyl]phenyl]piperazin-1-yl]methyl]phenyl]phenyl]-N,N-dimethylnaphthalene-2-carboxamide |
| | ED08-50-01R | ED08-50 | 1-Hydroxy-4-[5-[2-[[4-[4-[(4-methoxyphenyl)carbamoyl]phenyl]piperazin-1-yl]methyl]phenyl]pyridin-2-yl]-N,N-dimethylnaphthalene-2-carboxamide |
| | ED08-51-01R | ED08-51 | 1-Hydroxy-4-[4-[4-[[4-[4-[(4-methoxyphenyl)carbamoyl]phenyl]piperazin-1-yl]methyl]-2-methylphenyl]phenyl]-N,N-dimethylnaphthalene-2-carboxamide |
| | ED08-52-01R | ED08-52 | 1-Hydroxy-4-[5-[4-[[4-[4-[(4-methoxyphenyl)carbamoyl]phenyl]piperazin-1-yl]methyl]-2-methylphenyl]pyridin-2-yl]-N,N-dimethylnaphthalene-2-carboxamide |
| | ED08-53-01R | ED08-53 | 4-[4-[3-Ethyl-4-[[4-[4-[(4-methoxyphenyl)carbamoyl]phenyl]piperazin-1-yl]methyl]phenyl]phenyl]-1-hydroxy-N,N-dimethylnaphthalene-2-carboxamide |
| | ED08-54-01R | ED08-54 | 4-[5-[3-Ethyl-4-[[4-[4-[(4-methoxyphenyl)carbamoyl]phenyl]piperazin-1-yl]methyl]phenyl]pyridin-2-yl]-1-hydroxy-N,N-dimethylnaphthalene-2-carboxamide |
| | ED08-55-01R | ED08-55 | 4-[4-[2-Fluoro-4-[[4-[4-[(4-methoxyphenyl)carbamoyl]phenyl]piperazin-1-yl]methyl]phenyl]phenyl]-1-hydroxy-N,N-dimethylnaphthalene-2-carboxamide |
| | ED08-56-01R | ED08-56 | 4-[5-[2-Fluoro-4-[[4-[4-[(4-methoxyphenyl)carbamoyl]phenyl]piperazin-1-yl]methyl]phenyl]pyridin-2-yl]-1-hydroxy-N,N-dimethylnaphthalene-2-carboxamide |
| | ED08-57-01R | ED08-57 | 1-Hydroxy-4-[4-[3-methoxy-4-[[4-[4-[(4-methoxyphenyl)carbamoyl]phenyl]piperazin-1-yl]methyl]phenyl]phenyl]-N,N-dimethylnaphthalene-2-carboxamide |
| | ED08-58-01R | ED08-58 | 1-Hydroxy-4-[5-[3-methoxy-4-[[4-[4-[(4-methoxyphenyl)carbamoyl]phenyl]piperazin-1-yl]methyl]phenyl]pyridin-2-yl]-N,N-dimethylnaphthalene-2-carboxamide |
| | ED08-59-01R | ED08-59 | 4-[4-[3-Ethoxy-5-[[4-[4-[(4-methoxyphenyl)carbamoyl]phenyl]piperazin-1-yl]methyl]phenyl]phenyl]-1-hydroxy-N,N-dimethylnaphthalene-2-carboxamide |

| | | | |
|---|---|---|---|
| | ED08-60-01R | ED08-60 | 4-[5-[3-Ethoxy-5-[[4-[4-[(4-methoxyphenyl)carbamoyl]phenyl]piperazin-1-yl]methyl]phenyl]pyridin-2-yl]-1-hydroxy-N,N-dimethylnaphthalene-2-carboxamide |
| mixED08-01R | ED08-61-01R | ED08-61 | 1-Hydroxy-4-[4-[3-[[4-[4-[(4-methoxyphenyl)carbamoyl]phenyl]piperazin-1-yl]methyl]-5-propan-2-yloxyphenyl]phenyl]-N,N-dimethylnaphthalene-2-carboxamide |
| | ED08-62-01R | ED08-62 | 1-Hydroxy-4-[5-[3-[[4-[4-[(4-methoxyphenyl)carbamoyl]phenyl]piperazin-1-yl]methyl]-5-propan-2-yloxyphenyl]pyridin-2-yl]-N,N-dimethylnaphthalene-2-carboxamide |
| | ED08-63-01R | ED08-63 | 4-[4-[3,5-Dimethoxy-4-[[4-[4-[(4-methoxyphenyl)carbamoyl]phenyl]piperazin-1-yl]methyl]phenyl]phenyl]-1-hydroxy-N,N-dimethylnaphthalene-2-carboxamide |
| | ED08-64-01R | ED08-64 | 4-[5-[3,5-Dimethoxy-4-[[4-[4-[(4-methoxyphenyl)carbamoyl]phenyl]piperazin-1-yl]methyl]phenyl]pyridin-2-yl]-1-hydroxy-N,N-dimethylnaphthalene-2-carboxamide |
| | ED08-65-01R | ED08-65 | 1-Hydroxy-4-[4-[3-[[4-[4-[(4-methoxyphenyl)carbamoyl]phenyl]piperazin-1-yl]methyl]-5-(trifluoromethyl)phenyl]phenyl]-N,N-dimethylnaphthalene-2-carboxamide |
| | ED08-66-01R | ED08-66 | 1-Hydroxy-4-[5-[3-[[4-[4-[(4-methoxyphenyl)carbamoyl]phenyl]piperazin-1-yl]methyl]-5-(trifluoromethyl)phenyl]pyridin-2-yl]-N,N-dimethylnaphthalene-2-carboxamide |
| | ED08-67-01R | ED08-67 | 1-Hydroxy-4-[4-[3-[[4-[4-[(4-methoxyphenyl)carbamoyl]phenyl]piperazin-1-yl]methyl]-5-(trifluoromethoxy)phenyl]phenyl]-N,N-dimethylnaphthalene-2-carboxamide |
| | ED08-68-01R | ED08-68 | 1-Hydroxy-4-[5-[3-[[4-[4-[(4-methoxyphenyl)carbamoyl]phenyl]piperazin-1-yl]methyl]-5-(trifluoromethoxy)phenyl]pyridin-2-yl]-N,N-dimethylnaphthalene-2-carboxamide |
| | ED08-69-01R | ED08-69 | 1-Hydroxy-4-[4-[4-[[4-[4-[(4-methoxyphenyl)carbamoyl]phenyl]piperazin-1-yl]methyl]naphthalen-1-yl]phenyl]-N,N-dimethylnaphthalene-2-carboxamide |
| | ED08-70-01R | ED08-70 | 1-Hydroxy-4-[5-[4-[[4-[4-[(4-methoxyphenyl)carbamoyl]phenyl]piperazin-1-yl]methyl]naphthalen-1-yl]pyridin-2-yl]-N,N-dimethylnaphthalene-2-carboxamide |
| | ED08-71-01R | ED08-71 | 1-Hydroxy-4-[4-[5-[[4-[4-[(4-methoxyphenyl)carbamoyl]phenyl]piperazin-1-yl]methyl]thiophen-3-yl]phenyl]-N,N-dimethylnaphthalene-2-carboxamide |
| | ED08-72-01R | ED08-72 | 1-Hydroxy-4-[5-[5-[[4-[4-[(4-methoxyphenyl)carbamoyl]phenyl]piperazin-1-yl]methyl]thiophen-3-yl]pyridin-2-yl]-N,N-dimethylnaphthalene-2-carboxamide |
| | ED08-73-01R | ED08-73 | 1-Hydroxy-4-[4-[2-[2-[[4-[4-[(4-methoxyphenyl)carbamoyl]phenyl]piperazin-1-yl]methyl]phenyl]ethynyl]phenyl]-N,N-dimethylnaphthalene-2-carboxamide |
| | ED08-74-01R | ED08-74 | 1-Hydroxy-4-[5-[2-[2-[[4-[4-[(4-methoxyphenyl)carbamoyl]phenyl]piperazin-1-yl]methyl]phenyl]ethynyl]pyridin-2-yl]-N,N-dimethylnaphthalene-2-carboxamide |
| mixED08-01R | ED08-75-01R | ED08-75 | 6-[4-[[2-[4-[3-(Dimethylcarbamoyl)-4-hydroxynaphthalen-1-yl]phenyl]phenyl]methyl]piperazin-1-yl]-N-(4-methoxyphenyl)pyridazine-3-carboxamide |
| | ED08-76-01R | ED08-76 | 6-[4-[[2-[6-[3-(Dimethylcarbamoyl)-4-hydroxynaphthalen-1-yl]pyridin-3-yl]phenyl]methyl]piperazin-1-yl]-N-(4-methoxyphenyl)pyridazine-3-carboxamide |
| | ED08-77-01R | ED08-77 | 6-[4-[[4-[4-[3-(Dimethylcarbamoyl)-4-hydroxynaphthalen-1-yl]phenyl]-3-methylphenyl]methyl]piperazin-1-yl]-N-(4-methoxyphenyl)pyridazine-3-carboxamide |

| | | | |
|---|---|---|---|
| | ED08-78-01R | ED08-78 | 6-[4-[[4-[6-[3-(dimethylcarbamoyl)-4-hydroxynaphthalen-1-yl]pyridin-3-yl]-3-methylphenyl]methyl]piperazin-1-yl]-N-(4-methoxyphenyl)pyridazine-3-carboxamide |
| | ED08-79-01R | ED08-79 | 6-[4-[[4-[4-[3-(Dimethylcarbamoyl)-4-hydroxynaphthalen-1-yl]phenyl]-2-ethylphenyl]methyl]piperazin-1-yl]-N-(4-methoxyphenyl)pyridazine-3-carboxamide |
| | ED08-80-01R | ED08-80 | 6-[4-[[4-[6-[3-(Dimethylcarbamoyl)-4-hydroxynaphthalen-1-yl]pyridin-3-yl]-2-ethylphenyl]methyl]piperazin-1-yl]-N-(4-methoxyphenyl)pyridazine-3-carboxamide |
| | ED08-81-01R | ED08-81 | 6-[4-[[4-[4-[3-(Dimethylcarbamoyl)-4-hydroxynaphthalen-1-yl]phenyl]-3-fluorophenyl]methyl]piperazin-1-yl]-N-(4-methoxyphenyl)pyridazine-3-carboxamide |
| | ED08-82-01R | ED08-82 | 6-[4-[[4-[6-[3-(Dimethylcarbamoyl)-4-hydroxynaphthalen-1-yl]pyridin-3-yl]-3-fluorophenyl]methyl]piperazin-1-yl]-N-(4-methoxyphenyl)pyridazine-3-carboxamide |
| | ED08-83-01R | ED08-83 | 6-[4-[[4-[4-[3-(Dimethylcarbamoyl)-4-hydroxynaphthalen-1-yl]phenyl]-2-methoxyphenyl]methyl]piperazin-1-yl]-N-(4-methoxyphenyl)pyridazine-3-carboxamide |
| | ED08-84-01R | ED08-84 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-(4-methoxyphenyl)pyridazine-3-carboxamide |
| | ED08-85-01R | ED08-85 | 6-[4-[[3-[4-[3-(Dimethylcarbamoyl)-4-hydroxynaphthalen-1-yl]phenyl]-5-ethoxyphenyl]methyl]piperazin-1-yl]-N-(4-methoxyphenyl)pyridazine-3-carboxamide |
| | ED08-86-01R | ED08-86 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-(4-methoxyphenyl)pyridazine-3-carboxamide |
| | ED08-87-01R | ED08-87 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-(4-methoxyphenyl)pyridazine-3-carboxamide |
| | ED08-88-01R | ED08-88 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-(4-methoxyphenyl)pyridazine-3-carboxamide |
| mixED08-01R | ED08-89-01R | ED08-89 | 6-[4-[[4-[4-[3-(Dimethylcarbamoyl)-4-hydroxynaphthalen-1-yl]phenyl]-2,6-dimethoxyphenyl]methyl]piperazin-1-yl]-N-(4-methoxyphenyl)pyridazine-3-carboxamide |
| | ED08-90-01R | ED08-90 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-(4-methoxyphenyl)pyridazine-3-carboxamide |
| | ED08-91-01R | ED08-91 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-(4-methoxyphenyl)pyridazine-3-carboxamide |
| | ED08-92-01R | ED08-92 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-(4-methoxyphenyl)pyridazine-3-carboxamide |
| | ED08-93-01R | ED08-93 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-(4-methoxyphenyl)pyridazine-3-carboxamide |
| | ED08-94-01R | ED08-94 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-(4-methoxyphenyl)pyridazine-3-carboxamide |
| | ED08-95-01R | ED08-95 | 6-[4-[[4-[4-[3-(Dimethylcarbamoyl)-4-hydroxynaphthalen-1-yl]phenyl]naphthalen-1-yl]methyl]piperazin-1-yl]-N-(4-methoxyphenyl)pyridazine-3-carboxamide |
| | ED08-96-01R | ED08-96 | 6-[4-[[4-[6-[3-(Dimethylcarbamoyl)-4-hydroxynaphthalen-1-yl]pyridin-3-yl]naphthalen-1-yl]methyl]piperazin-1-yl]-N-(4-methoxyphenyl)pyridazine-3-carboxamide |

| | | | |
|---|---|---|---|
| | ED08-97-01R | ED08-97 | 6-[4-[[4-[4-[3-(Dimethylcarbamoyl)-4-hydroxynaphthalen-1-yl]phenyl]thiophen-2-yl]methyl]piperazin-1-yl]-N-(4-methoxyphenyl)pyridazine-3-carboxamide |
| | ED08-98-01R | ED08-98 | 6-[4-[[4-[6-[3-(Dimethylcarbamoyl)-4-hydroxynaphthalen-1-yl]pyridin-3-yl]thiophen-2-yl]methyl]piperazin-1-yl]-N-(4-methoxyphenyl)pyridazine-3-carboxamide |
| | ED08-99-01R | ED08-99 | 6-[4-[[2-[2-[4-[3-(Dimethylcarbamoyl)-4-hydroxynaphthalen-1-yl]phenyl]ethynyl]phenyl]methyl]piperazin-1-yl]-N-(4-methoxyphenyl)pyridazine-3-carboxamide |
| | ED08-100-01R | ED08-100 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-(4-methoxyphenyl)pyridazine-3-carboxamide |

Example 3-6-8: Synthesis of mixED09-01R

[Formula 521]

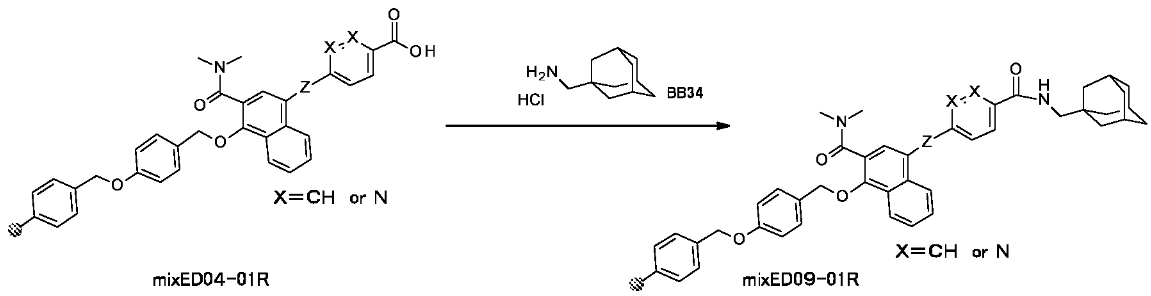


Mixture mixED04-01R (mixture that could contain 100 compounds, 50 mg, loading rate: 0.38 mmol/g, 0.019 mmol) shown in Table AG-D-04 and NMP (0.85 mL) were added to a 1.5 mL glass vial and shaken at room temperature for 1 hour. 1-Adamantylmethanamine hydrochloride (BB34, 11.5 mg, 57 μmol), DIC (17.8 μL, 0.114 mmol), and HOAt (15.5 mg, 0.114 mmol) were added thereto, and the mixture was shaken at 40°C for 20 hours. Further, DIPEA (10 μL, 57 μmol) was added to the reaction solution, and the mixture was shaken at 40°C for 2 hours. The suspension of the reaction solution and the resin was transferred to a syringe with a filter using NMP. The resultant was repetitively washed three times with NMP (1.2 mL), three times with MeOH (1.2 mL), three times with DCM (1.2 mL), and three times with heptane (1.2 mL), and the resin was then dried under reduced pressure to obtain the title mixture (mixED09-01R) shown in Table ED09.

[Table 108]

[Table ED09]

| No. of mixture | No. of compound that may be contained | Compound No. after cleavage | Compound name after cleavage |
|---|---|---|---|

| | | | |
|---|---|---|---|
| mixED09-01R | ED09-01-01R | ED09-01 | 4-[4-[4-[1-[4-[4-(1-Adamantylmethylcarbamoyl)phenyl]piperazin-1-yl]ethyl]phenyl]phenyl]-1-hydroxy-N,N-dimethylnaphthalene-2-carboxamide |
| | ED09-02-01R | ED09-02 | 4-[5-[4-[1-[4-[4-(1-Adamantylmethylcarbamoyl)phenyl]piperazin-1-yl]ethyl]phenyl]pyridin-2-yl]-1-hydroxy-N,N-dimethylnaphthalene-2-carboxamide |
| | ED09-03-01R | ED09-03 | 4-[4-[4-[1-[4-[4-(1-Adamantylmethylcarbamoyl)phenyl]piperazin-1-yl]ethyl]-3-fluorophenyl]phenyl]-1-hydroxy-N,N-dimethylnaphthalene-2-carboxamide |
| | ED09-04-01R | ED09-04 | 4-[5-[4-[1-[4-[4-(1-Adamantylmethylcarbamoyl)phenyl]piperazin-1-yl]ethyl]-3-fluorophenyl]pyridin-2-yl]-1-hydroxy-N,N-dimethylnaphthalene-2-carboxamide |
| | ED09-05-01R | ED09-05 | N-(1-Adamantylmethyl)-6-[4-[4-[4-[3-(dimethylcarbamoyl)-4-hydroxynaphthalen-1-yl]phenyl]-3-methylbenzoyl]piperazin-1-yl]pyridazine-3-carboxamide |
| mixED09-01R | ED09-06-01R | ED09-06 | N-(1-Adamantylmethyl)-6-[4-[4-[6-[3-(dimethylcarbamoyl)-4-hydroxynaphthalen-1-yl]pyridin-3-yl]-3-methylbenzoyl]piperazin-1-yl]pyridazine-3-carboxamide |
| | ED09-07-01R | ED09-07 | N-(1-Adamantylmethyl)-6-[4-[4-[4-[3-(dimethylcarbamoyl)-4-hydroxynaphthalen-1-yl]phenyl]-3-fluorobenzoyl]piperazin-1-yl]pyridazine-3-carboxamide |
| | ED09-08-01R | ED09-08 | N-(1-Adamantylmethyl)-6-[4-[4-[6-[3-(dimethylcarbamoyl)-4-hydroxynaphthalen-1-yl]pyridin-3-yl]-3-fluorobenzoyl]piperazin-1-yl]pyridazine-3-carboxamide |
| | ED09-09-01R | ED09-09 | N-(1-Adamantylmethyl)-6-[4-[3-[4-[3-(dimethylcarbamoyl)-4-hydroxynaphthalen-1-yl]phenyl]-5-(trifluoromethyl)benzoyl]piperazin-1-yl]pyridazine-3-carboxamide |
| | ED09-10-01R | ED09-10 | N-(1-Adamantylmethyl)-6-[4-[3-[6-[3-(dimethylcarbamoyl)-4-hydroxynaphthalen-1-yl]pyridin-3-yl]-5-(trifluoromethyl)benzoyl]piperazin-1-yl]pyridazine-3-carboxamide |
| | ED09-11-01R | ED09-11 | N-(1-Adamantylmethyl)-6-[4-[3-tert-butyl-5-[4-[3-(dimethylcarbamoyl)-4-hydroxynaphthalen-1-yl]phenyl]benzoyl]piperazin-1-yl]pyridazine-3-carboxamide |
| | ED09-12-01R | ED09-12 | N-(1-Adamantylmethyl)-6-[4-[3-tert-butyl-5-[6-[3-(dimethylcarbamoyl)-4-hydroxynaphthalen-1-yl]pyridin-3-yl]benzoyl]piperazin-1-yl]pyridazine-3-carboxamide |
| | ED09-13-01R | ED09-13 | N-(1-Adamantylmethyl)-6-[4-[4-[4-[3-(dimethylcarbamoyl)-4-hydroxynaphthalen-1-yl]phenyl]naphthalene-1-carbonyl]piperazin-1-yl]pyridazine-3-carboxamide |
| | ED09-14-01R | ED09-14 | N-(1-Adamantylmethyl)-6-[4-[4-[6-[3-(dimethylcarbamoyl)-4-hydroxynaphthalen-1-yl]pyridin-3-yl]naphthalene-1-carbonyl]piperazin-1-yl]pyridazine-3-carboxamide |
| | ED09-15-01R | ED09-15 | N-(1-Adamantylmethyl)-6-[4-[3-[2-[4-[3-(dimethylcarbamoyl)-4-hydroxynaphthalen-1-yl]phenyl]phenyl]propanoyl]piperazin-1-yl]pyridazine-3-carboxamide |
| | ED09-16-01R | ED09-16 | N-(1-Adamantylmethyl)-6-[4-[3-[2-[6-[3-(dimethylcarbamoyl)-4-hydroxynaphthalen-1-yl]pyridin-3-yl]phenyl]propanoyl]piperazin-1-yl]pyridazine-3-carboxamide |
| | ED09-17-01R | ED09-17 | N-(1-Adamantylmethyl)-6-[4-[4-[4-[3-(dimethylcarbamoyl)-4-hydroxynaphthalen-1-yl]phenyl]thiophene-2-carbonyl]piperazin-1-yl]pyridazine-3-carboxamide |
| | ED09-18-01R | ED09-18 | N-(1-Adamantylmethyl)-6-[4-[4-[6-[3-(dimethylcarbamoyl)-4-hydroxynaphthalen-1-yl]pyridin-3-yl]thiophene-2-carbonyl]piperazin-1-yl]pyridazine-3-carboxamide |
| | ED09-19-01R | ED09-19 | N-(1-Adamantylmethyl)-6-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]pyridazine-3-carboxamide |

| | | | |
|---|---|---|---|
| | ED09-20-01R | ED09-20 | N-(1-Adamantylmethyl)-6-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]pyridazine-3-carboxamide |
| mixED09-01R | ED09-21-01R | ED09-21 | N-(1-Adamantylmethyl)-6-[4-[2-[2-[4-[3-(dimethylcarbamoyl)-4-hydroxynaphthalen-1-yl]phenyl]ethynyl]benzoyl]piperazin-1-yl]pyridazine-3-carboxamide |
| | ED09-22-01R | ED09-22 | N-(1-Adamantylmethyl)-6-[4-[2-[2-[6-[3-(dimethylcarbamoyl)-4-hydroxynaphthalen-1-yl]pyridin-3-yl]ethynyl]benzoyl]piperazin-1-yl]pyridazine-3-carboxamide |
| | ED09-23-01R | ED09-23 | N-(1-Adamantylmethyl)-6-[4-[3-[2-[4-[3-(dimethylcarbamoyl)-4-hydroxynaphthalen-1-yl]phenyl]ethynyl]-5-(trifluoromethyl)benzoyl]piperazin-1-yl]pyridazine-3-carboxamide |
| | ED09-24-01R | ED09-24 | N-(1-Adamantylmethyl)-6-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]pyridazine-3-carboxamide |
| | ED09-25-01R | ED09-25 | N-(1-Adamantylmethyl)-6-[4-[5-[2-[4-[3-(dimethylcarbamoyl)-4-hydroxynaphthalen-1-yl]phenyl]ethynyl]pyridine-3-carbonyl]piperazin-1-yl]pyridazine-3-carboxamide |
| | ED09-26-01R | ED09-26 | N-(1-Adamantylmethyl)-6-[4-[5-[2-[6-[3-(dimethylcarbamoyl)-4-hydroxynaphthalen-1-yl]pyridin-3-yl]ethynyl]pyridine-3-carbonyl]piperazin-1-yl]pyridazine-3-carboxamide |
| | ED09-27-01R | ED09-27 | 4-[4-[4-[4-[4-(1-Adamantylmethylcarbamoyl)phenyl]piperazine-1-carbonyl]-2-methylphenyl]phenyl]-1-hydroxy-N,N-dimethylnaphthalene-2-carboxamide |
| | ED09-28-01R | ED09-28 | 4-[5-[4-[4-[4-(1-Adamantylmethylcarbamoyl)phenyl]piperazine-1-carbonyl]-2-methylphenyl]pyridin-2-yl]-1-hydroxy-N,N-dimethylnaphthalene-2-carboxamide |
| | ED09-29-01R | ED09-29 | 4-[4-[4-[4-[4-(1-Adamantylmethylcarbamoyl)phenyl]piperazine-1-carbonyl]-2-fluorophenyl]phenyl]-1-hydroxy-N,N-dimethylnaphthalene-2-carboxamide |
| | ED09-30-01R | ED09-30 | 4-[5-[4-[4-[4-(1-Adamantylmethylcarbamoyl)phenyl]piperazine-1-carbonyl]-2-fluorophenyl]pyridin-2-yl]-1-hydroxy-N,N-dimethylnaphthalene-2-carboxamide |
| | ED09-31-01R | ED09-31 | 4-[4-[3-[4-[4-(1-Adamantylmethylcarbamoyl)phenyl]piperazine-1-carbonyl]-5-(trifluoromethyl)phenyl]phenyl]-1-hydroxy-N,N-dimethylnaphthalene-2-carboxamide |
| | ED09-32-01R | ED09-32 | 4-[5-[3-[4-[4-(1-Adamantylmethylcarbamoyl)phenyl]piperazine-1-carbonyl]-5-(trifluoromethyl)phenyl]pyridin-2-yl]-1-hydroxy-N,N-dimethylnaphthalene-2-carboxamide |
| | ED09-33-01R | ED09-33 | 4-[4-[3-[4-[4-(1-Adamantylmethylcarbamoyl)phenyl]piperazine-1-carbonyl]-5-tert-butylphenyl]phenyl]-1-hydroxy-N,N-dimethylnaphthalene-2-carboxamide |
| | ED09-34-01R | ED09-34 | 4-[5-[3-[4-[4-(1-Adamantylmethylcarbamoyl)phenyl]piperazine-1-carbonyl]-5-tert-butylphenyl]pyridin-2-yl]-1-hydroxy-N,N-dimethylnaphthalene-2-carboxamide |
| | ED09-35-01R | ED09-35 | 4-[4-[4-[4-[4-(1-Adamantylmethylcarbamoyl)phenyl]piperazine-1-carbonyl]naphthalen-1-yl]phenyl]-1-hydroxy-N,N-dimethylnaphthalene-2-carboxamide |
| | ED09-36-01R | ED09-36 | 4-[5-[4-[4-[4-(1-Adamantylmethylcarbamoyl)phenyl]piperazine-1-carbonyl]naphthalen-1-yl]pyridin-2-yl]-1-hydroxy-N,N-dimethylnaphthalene-2-carboxamide |
| mixED09-01R | ED09-37-01R | ED09-37 | 4-[4-[2-[3-[4-[4-(1-Adamantylmethylcarbamoyl)phenyl]piperazin-1-yl]-3-oxopropyl]phenyl]phenyl]-1-hydroxy-N,N-dimethylnaphthalene-2-carboxamide |
| | ED09-38-01R | ED09-38 | 4-[5-[2-[3-[4-[4-(1-Adamantylmethylcarbamoyl)phenyl]piperazin-1-yl]-3-oxopropyl]phenyl]pyridin-2-yl]-1-hydroxy-N,N-dimethylnaphthalene-2-carboxamide |

| | | | |
|---|---|---|---|
| | ED09-39-01R | ED09-39 | 4-[4-[5-[4-[4-(1-Adamantylmethylcarbamoyl)phenyl]piperazine-1-carbonyl]thiophen-3-yl]phenyl]-1-hydroxy-N,N-dimethylnaphthalene-2-carboxamide |
| | ED09-40-01R | ED09-40 | 4-[5-[5-[4-[4-(1-Adamantylmethylcarbamoyl)phenyl]piperazine-1-carbonyl]thiophen-3-yl]pyridin-2-yl]-1-hydroxy-N,N-dimethylnaphthalene-2-carboxamide |
| | ED09-41-01R | ED09-41 | 4-[4-[1-[3-[4-[4-(1-Adamantylmethylcarbamoyl)phenyl]piperazine-1-carbonyl]-5-(trifluoromethyl)phenyl]pyrazol-4-yl]phenyl]-1-hydroxy-N,N-dimethylnaphthalene-2-carboxamide |
| | ED09-42-01R | ED09-42 | 4-[5-[1-[3-[4-[4-(1-Adamantylmethylcarbamoyl)phenyl]piperazine-1-carbonyl]-5-(trifluoromethyl)phenyl]pyrazol-4-yl]pyridin-2-yl]-1-hydroxy-N,N-dimethylnaphthalene-2-carboxamide |
| | ED09-43-01R | ED09-43 | 4-[4-[2-[2-[4-[4-(1-Adamantylmethylcarbamoyl)phenyl]piperazine-1-carbonyl]phenyl]ethynyl]phenyl]-1-hydroxy-N,N-dimethylnaphthalene-2-carboxamide |
| | ED09-44-01R | ED09-44 | 4-[5-[2-[2-[4-[4-(1-Adamantylmethylcarbamoyl)phenyl]piperazine-1-carbonyl]phenyl]ethynyl]pyridin-2-yl]-1-hydroxy-N,N-dimethylnaphthalene-2-carboxamide |
| | ED09-45-01R | ED09-45 | 4-[4-[2-[3-[4-[4-(1-Adamantylmethylcarbamoyl)phenyl]piperazine-1-carbonyl]-5-(trifluoromethyl)phenyl]ethynyl]phenyl]-1-hydroxy-N,N-dimethylnaphthalene-2-carboxamide |
| | ED09-46-01R | ED09-46 | 4-[5-[2-[3-[4-[4-(1-Adamantylmethylcarbamoyl)phenyl]piperazine-1-carbonyl]-5-(trifluoromethyl)phenyl]ethynyl]pyridin-2-yl]-1-hydroxy-N,N-dimethylnaphthalene-2-carboxamide |
| | ED09-47-01R | ED09-47 | 4-[4-[2-[5-[4-[4-(1-Adamantylmethylcarbamoyl)phenyl]piperazine-1-carbonyl]pyridin-3-yl]ethynyl]phenyl]-1-hydroxy-N,N-dimethylnaphthalene-2-carboxamide |
| | ED09-48-01R | ED09-48 | 4-[5-[2-[5-[4-[4-(1-Adamantylmethylcarbamoyl)phenyl]piperazine-1-carbonyl]pyridin-3-yl]ethynyl]pyridin-2-yl]-1-hydroxy-N,N-dimethylnaphthalene-2-carboxamide |
| | ED09-49-01R | ED09-49 | 4-[4-[2-[[4-[4-(1-Adamantylmethylcarbamoyl)phenyl]piperazin-1-yl]methyl]phenyl]phenyl]-1-hydroxy-N,N-dimethylnaphthalene-2-carboxamide |
| | ED09-50-01R | ED09-50 | 4-[5-[2-[[4-[4-(1-Adamantylmethylcarbamoyl)phenyl]piperazin-1-yl]methyl]phenyl]pyridin-2-yl]-1-hydroxy-N,N-dimethylnaphthalene-2-carboxamide |
| | ED09-51-01R | ED09-51 | 4-[4-[4-[[4-[4-(1-Adamantylmethylcarbamoyl)phenyl]piperazin-1-yl]methyl]-2-methylphenyl]phenyl]-1-hydroxy-N,N-dimethylnaphthalene-2-carboxamide |
| mixED09-01R | ED09-52-01R | ED09-52 | 4-[5-[4-[[4-[4-(1-Adamantylmethylcarbamoyl)phenyl]piperazin-1-yl]methyl]-2-methylphenyl]pyridin-2-yl]-1-hydroxy-N,N-dimethylnaphthalene-2-carboxamide |
| | ED09-53-01R | ED09-53 | 4-[4-[4-[[4-[4-(1-Adamantylmethylcarbamoyl)phenyl]piperazin-1-yl]methyl]-3-ethylphenyl]phenyl]-1-hydroxy-N,N-dimethylnaphthalene-2-carboxamide |
| | ED09-54-01R | ED09-54 | 4-[5-[4-[[4-[4-(1-Adamantylmethylcarbamoyl)phenyl]piperazin-1-yl]methyl]-3-ethylphenyl]pyridin-2-yl]-1-hydroxy-N,N-dimethylnaphthalene-2-carboxamide |
| | ED09-55-01R | ED09-55 | 4-[4-[4-[[4-[4-(1-Adamantylmethylcarbamoyl)phenyl]piperazin-1-yl]methyl]-2-fluorophenyl]phenyl]-1-hydroxy-N,N-dimethylnaphthalene-2-carboxamide |
| | ED09-56-01R | ED09-56 | 4-[5-[4-[[4-[4-(1-Adamantylmethylcarbamoyl)phenyl]piperazin-1-yl]methyl]-2-fluorophenyl]pyridin-2-yl]-1-hydroxy-N,N-dimethylnaphthalene-2-carboxamide |
| | ED09-57-01R | ED09-57 | 4-[4-[4-[[4-[4-(1-Adamantylmethylcarbamoyl)phenyl]piperazin-1-yl]methyl]-3-methoxyphenyl]phenyl]-1-hydroxy-N,N-dimethylnaphthalene-2-carboxamide |

| | | | |
|---|---|---|---|
| | ED09-58-01R | ED09-58 | 4-[5-[4-[[4-[4-(1-Adamantylmethylcarbamoyl)phenyl]piperazin-1-yl]methyl]-3-methoxyphenyl]pyridin-2-yl]-1-hydroxy-N,N-dimethylnaphthalene-2-carboxamide |
| | ED09-59-01R | ED09-59 | 4-[4-[3-[[4-[4-(1-Adamantylmethylcarbamoyl)phenyl]piperazin-1-yl]methyl]-5-ethoxyphenyl]phenyl]-1-hydroxy-N,N-dimethylnaphthalene-2-carboxamide |
| | ED09-60-01R | ED09-60 | 4-[5-[3-[[4-[4-(1-Adamantylmethylcarbamoyl)phenyl]piperazin-1-yl]methyl]-5-ethoxyphenyl]pyridin-2-yl]-1-hydroxy-N,N-dimethylnaphthalene-2-carboxamide |
| | ED09-61-01R | ED09-61 | 4-[4-[3-[[4-[4-(1-Adamantylmethylcarbamoyl)phenyl]piperazin-1-yl]methyl]-5-propan-2-yloxyphenyl]phenyl]-1-hydroxy-N,N-dimethylnaphthalene-2-carboxamide |
| | ED09-62-01R | ED09-62 | 4-[5-[3-[[4-[4-(1-Adamantylmethylcarbamoyl)phenyl]piperazin-1-yl]methyl]-5-propan-2-yloxyphenyl]pyridin-2-yl]-1-hydroxy-N,N-dimethylnaphthalene-2-carboxamide |
| | ED09-63-01R | ED09-63 | 4-[4-[4-[[4-[4-(1-Adamantylmethylcarbamoyl)phenyl]piperazin-1-yl]methyl]-3,5-dimethoxyphenyl]phenyl]-1-hydroxy-N,N-dimethylnaphthalene-2-carboxamide |
| | ED09-64-01R | ED09-64 | 4-[5-[4-[[4-[4-(1-Adamantylmethylcarbamoyl)phenyl]piperazin-1-yl]methyl]-3,5-dimethoxyphenyl]pyridin-2-yl]-1-hydroxy-N,N-dimethylnaphthalene-2-carboxamide |
| | ED09-65-01R | ED09-65 | 4-[4-[3-[[4-[4-(1-Adamantylmethylcarbamoyl)phenyl]piperazin-1-yl]methyl]-5-(trifluoromethyl)phenyl]phenyl]-1-hydroxy-N,N-dimethylnaphthalene-2-carboxamide |
| | ED09-66-01R | ED09-66 | 4-[5-[3-[[4-[4-(1-Adamantylmethylcarbamoyl)phenyl]piperazin-1-yl]methyl]-5-(trifluoromethyl)phenyl]pyridin-2-yl]-1-hydroxy-N,N-dimethylnaphthalene-2-carboxamide |
| | ED09-67-01R | ED09-67 | 4-[4-[3-[[4-[4-(1-Adamantylmethylcarbamoyl)phenyl]piperazin-1-yl]methyl]-5-(trifluoromethoxy)phenyl]phenyl]-1-hydroxy-N,N-dimethylnaphthalene-2-carboxamide |
| mixED09-01R | ED09-68-01R | ED09-68 | 4-[5-[3-[[4-[4-(1-Adamantylmethylcarbamoyl)phenyl]piperazin-1-yl]methyl]-5-(trifluoromethoxy)phenyl]pyridin-2-yl]-1-hydroxy-N,N-dimethylnaphthalene-2-carboxamide |
| | ED09-69-01R | ED09-69 | 4-[4-[4-[[4-[4-(1-Adamantylmethylcarbamoyl)phenyl]piperazin-1-yl]methyl]naphthalen-1-yl]phenyl]-1-hydroxy-N,N-dimethylnaphthalene-2-carboxamide |
| | ED09-70-01R | ED09-70 | 4-[5-[4-[[4-[4-(1-Adamantylmethylcarbamoyl)phenyl]piperazin-1-yl]methyl]naphthalen-1-yl]pyridin-2-yl]-1-hydroxy-N,N-dimethylnaphthalene-2-carboxamide |
| | ED09-71-01R | ED09-71 | 4-[4-[5-[[4-[4-(1-Adamantylmethylcarbamoyl)phenyl]piperazin-1-yl]methyl]thiophen-3-yl]phenyl]-1-hydroxy-N,N-dimethylnaphthalene-2-carboxamide |
| | ED09-72-01R | ED09-72 | 4-[5-[5-[[4-[4-(1-Adamantylmethylcarbamoyl)phenyl]piperazin-1-yl]methyl]thiophen-3-yl]pyridin-2-yl]-1-hydroxy-N,N-dimethylnaphthalene-2-carboxamide |
| | ED09-73-01R | ED09-73 | 4-[4-[2-[2-[[4-[4-(1-Adamantylmethylcarbamoyl)phenyl]piperazin-1-yl]methyl]phenyl]ethynyl]phenyl]-1-hydroxy-N,N-dimethylnaphthalene-2-carboxamide |
| | ED09-74-01R | ED09-74 | 4-[5-[2-[2-[[4-[4-(1-Adamantylmethylcarbamoyl)phenyl]piperazin-1-yl]methyl]phenyl]ethynyl]pyridin-2-yl]-1-hydroxy-N,N-dimethylnaphthalene-2-carboxamide |
| | ED09-75-01R | ED09-75 | N-(1-Adamantylmethyl)-6-[4-[[2-[4-[3-(dimethylcarbamoyl)-4-hydroxynaphthalen-1-yl]phenyl]phenyl]methyl]piperazin-1-yl]pyridazine-3-carboxamide |
| | ED09-76-01R | ED09-76 | N-(1-Adamantylmethyl)-6-[4-[[2-[6-[3-(dimethylcarbamoyl)-4-hydroxynaphthalen-1-yl]pyridin-3-yl]phenyl]methyl]piperazin-1-yl]pyridazine-3-carboxamide |

| | | | |
|---|---|---|---|
| | ED09-77-01R | ED09-77 | N-(1-Adamantylmethyl)-6-[4-[[4-[4-[3-(dimethylcarbamoyl)-4-hydroxynaphthalen-1-yl]phenyl]-3-methylphenyl]methyl]piperazin-1-yl]pyridazine-3-carboxamide |
| | ED09-78-01R | ED09-78 | N-(1-Adamantylmethyl)-6-[4-[[4-[6-[3-(dimethylcarbamoyl)-4-hydroxynaphthalen-1-yl]pyridin-3-yl]-3-methylphenyl]methyl]piperazin-1-yl]pyridazine-3-carboxamide |
| | ED09-79-01R | ED09-79 | N-(1-Adamantylmethyl)-6-[4-[[4-[4-[3-(dimethylcarbamoyl)-4-hydroxynaphthalen-1-yl]phenyl]-2-ethylphenyl]methyl]piperazin-1-yl]pyridazine-3-carboxamide |
| | ED09-80-01R | ED09-80 | N-(1-Adamantylmethyl)-6-[4-[[4-[6-[3-(dimethylcarbamoyl)-4-hydroxynaphthalen-1-yl]pyridin-3-yl]-2-ethylphenyl]methyl]piperazin-1-yl]pyridazine-3-carboxamide |
| | ED09-81-01R | ED09-81 | N-(1-Adamantylmethyl)-6-[4-[[4-[4-[3-(dimethylcarbamoyl)-4-hydroxynaphthalen-1-yl]phenyl]-3-fluorophenyl]methyl]piperazin-1-yl]pyridazine-3-carboxamide |
| | ED09-82-01R | ED09-82 | N-(1-Adamantylmethyl)-6-[4-[[4-[6-[3-(dimethylcarbamoyl)-4-hydroxynaphthalen-1-yl]pyridin-3-yl]-3-fluorophenyl]methyl]piperazin-1-yl]pyridazine-3-carboxamide |
| | ED09-83-01R | ED09-83 | N-(1-Adamantylmethyl)-6-[4-[[4-[4-[3-(dimethylcarbamoyl)-4-hydroxynaphthalen-1-yl]phenyl]-2-methoxyphenyl]methyl]piperazin-1-yl]pyridazine-3-carboxamide |
| mixED09-01R | ED09-84-01R | ED09-84 | N-(1-Adamantylmethyl)-6-[4-[[4-[6-[3-(dimethylcarbamoyl)-4-hydroxynaphthalen-1-yl]pyridin-3-yl]-2-methoxyphenyl]methyl]piperazin-1-yl]pyridazine-3-carboxamide |
| | ED09-85-01R | ED09-85 | N-(1-Adamantylmethyl)-6-[4-[[3-[4-[3-(dimethylcarbamoyl)-4-hydroxynaphthalen-1-yl]phenyl]-5-ethoxyphenyl]methyl]piperazin-1-yl]pyridazine-3-carboxamide |
| | ED09-86-01R | ED09-86 | N-(1-Adamantylmethyl)-6-[4-[[3-[6-[3-(dimethylcarbamoyl)-4-hydroxynaphthalen-1-yl]pyridin-3-yl]-5-ethoxyphenyl]methyl]piperazin-1-yl]pyridazine-3-carboxamide |
| | ED09-87-01R | ED09-87 | N-(1-Adamantylmethyl)-6-[4-[[3-[4-[3-(dimethylcarbamoyl)-4-hydroxynaphthalen-1-yl]phenyl]-5-propan-2-yloxyphenyl]methyl]piperazin-1-yl]pyridazine-3-carboxamide |
| | ED09-88-01R | ED09-88 | N-(1-Adamantylmethyl)-6-[4-[[3-[6-[3-(dimethylcarbamoyl)-4-hydroxynaphthalen-1-yl]pyridin-3-yl]-5-propan-2-yloxyphenyl]methyl]piperazin-1-yl]pyridazine-3-carboxamide |
| | ED09-89-01R | ED09-89 | N-(1-Adamantylmethyl)-6-[4-[[4-[4-[3-(dimethylcarbamoyl)-4-hydroxynaphthalen-1-yl]phenyl]-2,6-dimethoxyphenyl]methyl]piperazin-1-yl]pyridazine-3-carboxamide |
| | ED09-90-01R | ED09-90 | N-(1-Adamantylmethyl)-6-[4-[[4-[6-[3-(dimethylcarbamoyl)-4-hydroxynaphthalen-1-yl]pyridin-3-yl]-2,6-dimethoxyphenyl]methyl]piperazin-1-yl]pyridazine-3-carboxamide |
| | ED09-91-01R | ED09-91 | N-(1-Adamantylmethyl)-6-[4-[[3-[4-[3-(dimethylcarbamoyl)-4-hydroxynaphthalen-1-yl]phenyl]-5-(trifluoromethyl)phenyl]methyl]piperazin-1-yl]pyridazine-3-carboxamide |
| | ED09-92-01R | ED09-92 | N-(1-Adamantylmethyl)-6-[4-[[3-[6-[3-(dimethylcarbamoyl)-4-hydroxynaphthalen-1-yl]pyridin-3-yl]-5-(trifluoromethyl)phenyl]methyl]piperazin-1-yl]pyridazine-3-carboxamide |
| | ED09-93-01R | ED09-93 | N-(1-Adamantylmethyl)-6-[4-[[3-[4-[3-(dimethylcarbamoyl)-4-hydroxynaphthalen-1-yl]phenyl]-5-(trifluoromethoxy)phenyl]methyl]piperazin-1-yl]pyridazine-3-carboxamide |
| | ED09-94-01R | ED09-94 | N-(1-Adamantylmethyl)-6-[4-[[3-[6-[3-(dimethylcarbamoyl)-4-hydroxynaphthalen-1-yl]pyridin-3-yl]-5-(trifluoromethoxy)phenyl]methyl]piperazin-1-yl]pyridazine-3-carboxamide |
| | ED09-95-01R | ED09-95 | N-(1-Adamantylmethyl)-6-[4-[[4-[4-[3-(dimethylcarbamoyl)-4-hydroxynaphthalen-1-yl]phenyl]naphthalen-1-yl]methyl]piperazin-1-yl]pyridazine-3-carboxamide |

| | | | |
|---|---|---|---|
| | ED09-96-01R | ED09-96 | N-(1-Adamantylmethyl)-6-[4-[[4-[6-[3-(dimethylcarbamoyl)-4-hydroxynaphthalen-1-yl]pyridin-3-yl]naphthalen-1-yl]methyl]piperazin-1-yl]pyridazine-3-carboxamide |
| | ED09-97-01R | ED09-97 | N-(1-Adamantylmethyl)-6-[4-[[4-[4-[3-(dimethylcarbamoyl)-4-hydroxynaphthalen-1-yl]phenyl]thiophen-2-yl]methyl]piperazin-1-yl]pyridazine-3-carboxamide |
| | ED09-98-01R | ED09-98 | N-(1-Adamantylmethyl)-6-[4-[[4-[6-[3-(dimethylcarbamoyl)-4-hydroxynaphthalen-1-yl]pyridin-3-yl]thiophen-2-yl]methyl]piperazin-1-yl]pyridazine-3-carboxamide |
| mixED09-01R | ED09-99-01R | ED09-99 | N-(1-Adamantylmethyl)-6-[4-[[2-[2-[4-[3-(dimethylcarbamoyl)-4-hydroxynaphthalen-1-yl]phenyl]ethynyl]phenyl]methyl]piperazin-1-yl]pyridazine-3-carboxamide |
| | ED09-100-01R | ED09-100 | N-(1-Adamantylmethyl)-6-[4-[[2-[2-[6-[3-(dimethylcarbamoyl)-4-hydroxynaphthalen-1-yl]pyridin-3-yl]ethynyl]phenyl]methyl]piperazin-1-yl]pyridazine-3-carboxamide |

Example 3-6-9: Synthesis of mixture mixED10-01R

The title mixture (mixED10-01R) shown in Table ED10 was obtained in the same manner as in Example 3-6-8 using mixED04-01R and 1-(1-adamantyl)-N- methylmethanamine hydrochloride (BB35).

[Table 109]

[Table ED10]

| No. of mixture | No. of compound that may be contained | Compound No. after cleavage | Compound name after cleavage |
|---|---|---|---|
| mixED10-01R | ED10-01-01R | ED10-01 | 4-[4-[4-[1-[4-[4-[1-Adamantylmethyl(methyl)carbamoyl]phenyl]piperazin-1-yl]ethyl]phenyl]phenyl]-1-hydroxy-N,N-dimethylnaphthalene-2-carboxamide |
| | ED10-02-01R | ED10-02 | 4-[5-[4-[1-[4-[4-[1-Adamantylmethyl(methyl)carbamoyl]phenyl]piperazin-1-yl]ethyl]phenyl]pyridin-2-yl]-1-hydroxy-N,N-dimethylnaphthalene-2-carboxamide |
| | ED10-03-01R | ED10-03 | 4-[4-[4-[1-[4-[4-[1-Adamantylmethyl(methyl)carbamoyl]phenyl]piperazin-1-yl]ethyl]-3-fluorophenyl]phenyl]-1-hydroxy-N,N-dimethylnaphthalene-2-carboxamide |
| | ED10-04-01R | ED10-04 | 4-[5-[4-[1-[4-[4-[1-Adamantylmethyl(methyl)carbamoyl]phenyl]piperazin-1-yl]ethyl]-3-fluorophenyl]pyridin-2-yl]-1-hydroxy-N,N-dimethylnaphthalene-2-carboxamide |
| | ED10-05-01R | ED10-05 | N-(1-Adamantylmethyl)-6-[4-[4-[4-[3-(dimethylcarbamoyl)-4-hydroxynaphthalen-1-yl]phenyl]-3-methylbenzoyl]piperazin-1-yl]-N-methylpyridazine-3-carboxamide |
| | ED10-06-01R | ED10-06 | N-(1-Adamantylmethyl)-6-[4-[4-[6-[3-(dimethylcarbamoyl)-4-hydroxynaphthalen-1-yl]pyridin-3-yl]-3-methylbenzoyl]piperazin-1-yl]-N-methylpyridazine-3-carboxamide |
| | ED10-07-01R | ED10-07 | N-(1-Adamantylmethyl)-6-[4-[4-[4-[3-(dimethylcarbamoyl)-4-hydroxynaphthalen-1-yl]phenyl]-3-fluorobenzoyl]piperazin-1-yl]-N-methylpyridazine-3-carboxamide |

| | | | |
|---|---|---|---|
| | ED10-08-01R | ED10-08 | N-(1-Adamantylmethyl)-6-[4-[4-[6-[3-(dimethylcarbamoyl)-4-hydroxynaphthalen-1-yl]pyridin-3-yl]-3-fluorobenzoyl]piperazin-1-yl]-N-methylpyridazine-3-carboxamide |
| mixED10-01R | ED10-09-01R | ED10-09 | N-(1-Adamantylmethyl)-6-[4-[3-[4-[3-(dimethylcarbamoyl)-4-hydroxynaphthalen-1-yl]phenyl]-5-(trifluoromethyl)benzoyl]piperazin-1-yl]-N-methylpyridazine-3-carboxamide |
| | ED10-10-01R | ED10-10 | N-(1-Adamantylmethyl)-6-[4-[3-[6-[3-(dimethylcarbamoyl)-4-hydroxynaphthalen-1-yl]pyridin-3-yl]-5-(trifluoromethyl)benzoyl]piperazin-1-yl]-N-methylpyridazine-3-carboxamide |
| | ED10-11-01R | ED10-11 | N-(1-Adamantylmethyl)-6-[4-[3-tert-butyl-5-[4-[3-(dimethylcarbamoyl)-4-hydroxynaphthalen-1-yl]phenyl]benzoyl]piperazin-1-yl]-N-methylpyridazine-3-carboxamide |
| | ED10-12-01R | ED10-12 | N-(1-Adamantylmethyl)-6-[4-[3-tert-butyl-5-[6-[3-(dimethylcarbamoyl)-4-hydroxynaphthalen-1-yl]pyridin-3-yl]benzoyl]piperazin-1-yl]-N-methylpyridazine-3-carboxamide |
| | ED10-13-01R | ED10-13 | N-(1-Adamantylmethyl)-6-[4-[4-[4-[3-(dimethylcarbamoyl)-4-hydroxynaphthalen-1-yl]phenyl]naphthalene-1-carbonyl]piperazin-1-yl]-N-methylpyridazine-3-carboxamide |
| | ED10-14-01R | ED10-14 | N-(1-Adamantylmethyl)-6-[4-[4-[6-[3-(dimethylcarbamoyl)-4-hydroxynaphthalen-1-yl]pyridin-3-yl]naphthalene-1-carbonyl]piperazin-1-yl]-N-methylpyridazine-3-carboxamide |
| | ED10-15-01R | ED10-15 | N-(1-Adamantylmethyl)-6-[4-[3-[2-[4-[3-(dimethylcarbamoyl)-4-hydroxynaphthalen-1-yl]phenyl]phenyl]propanoyl]piperazin-1-yl]-N-methylpyridazine-3-carboxamide |
| | ED10-16-01R | ED10-16 | N-(1-Adamantylmethyl)-6-[4-[3-[2-[6-[3-(dimethylcarbamoyl)-4-hydroxynaphthalen-1-yl]pyridin-3-yl]phenyl]propanoyl]piperazin-1-yl]-N-methylpyridazine-3-carboxamide |
| | ED10-17-01R | ED10-17 | N-(1-Adamantylmethyl)-6-[4-[4-[4-[3-(dimethylcarbamoyl)-4-hydroxynaphthalen-1-yl]phenyl]thiophene-2-carbonyl]piperazin-1-yl]-N-methylpyridazine-3-carboxamide |
| | ED10-18-01R | ED10-18 | N-(1-Adamantylmethyl)-6-[4-[4-[6-[3-(dimethylcarbamoyl)-4-hydroxynaphthalen-1-yl]pyridin-3-yl]thiophene-2-carbonyl]piperazin-1-yl]-N-methylpyridazine-3-carboxamide |
| | ED10-19-01R | ED10-19 | N-(1-Adamantylmethyl)-6-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]-N-methylpyridazine-3-carboxamide |
| | ED10-20-01R | ED10-20 | N-(1-Adamantylmethyl)-6-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]-N-methylpyridazine-3-carboxamide |
| | ED10-21-01R | ED10-21 | N-(1-Adamantylmethyl)-6-[4-[2-[2-[4-[3-(dimethylcarbamoyl)-4-hydroxynaphthalen-1-yl]phenyl]ethynyl]benzoyl]piperazin-1-yl]-N-methylpyridazine-3-carboxamide |
| | ED10-22-01R | ED10-22 | N-(1-Adamantylmethyl)-6-[4-[2-[2-[6-[3-(dimethylcarbamoyl)-4-hydroxynaphthalen-1-yl]pyridin-3-yl]ethynyl]benzoyl]piperazin-1-yl]-N-methylpyridazine-3-carboxamide |

| | | | |
|---|---|---|---|
| | ED10-23-01R | ED10-23 | N-(1-Adamantylmethyl)-6-[4-[3-[2-[4-[3-(dimethylcarbamoyl)-4-hydroxynaphthalen-1-yl]phenyl]ethynyl]-5-(trifluoromethyl)benzoyl]piperazin-1-yl]-N-methylpyridazine-3-carboxamide |
| mixED10-01R | ED10-24-01R | ED10-24 | N-(1-Adamantylmethyl)-6-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]-N-methylpyridazine-3-carboxamide |
| | ED10-25-01R | ED10-25 | N-(1-Adamantylmethyl)-6-[4-[5-[2-[4-[3-(dimethylcarbamoyl)-4-hydroxynaphthalen-1-yl]phenyl]ethynyl]pyridine-3-carbonyl]piperazin-1-yl]-N-methylpyridazine-3-carboxamide |
| | ED10-26-01R | ED10-26 | N-(1-Adamantylmethyl)-6-[4-[5-[2-[6-[3-(dimethylcarbamoyl)-4-hydroxynaphthalen-1-yl]pyridin-3-yl]ethynyl]pyridine-3-carbonyl]piperazin-1-yl]-N-methylpyridazine-3-carboxamide |
| | ED10-27-01R | ED10-27 | 4-[4-[4-[4-[4-[1-Adamantylmethyl(methyl)carbamoyl]phenyl]piperazine-1-carbonyl]-2-methylphenyl]phenyl]-1-hydroxy-N,N-dimethylnaphthalene-2-carboxamide |
| | ED10-28-01R | ED10-28 | 4-[5-[4-[4-[4-[1-Adamantylmethyl(methyl)carbamoyl]phenyl]piperazine-1-carbonyl]-2-methylphenyl]pyridin-2-yl]-1-hydroxy-N,N-dimethylnaphthalene-2-carboxamide |
| | ED10-29-01R | ED10-29 | 4-[4-[4-[4-[4-[1-Adamantylmethyl(methyl)carbamoyl]phenyl]piperazine-1-carbonyl]-2-fluorophenyl]phenyl]-1-hydroxy-N,N-dimethylnaphthalene-2-carboxamide |
| | ED10-30-01R | ED10-30 | 4-[5-[4-[4-[4-[1-Adamantylmethyl(methyl)carbamoyl]phenyl]piperazine-1-carbonyl]-2-fluorophenyl]pyridin-2-yl]-1-hydroxy-N,N-dimethylnaphthalene-2-carboxamide |
| | ED10-31-01R | ED10-31 | 4-[4-[3-[4-[4-[1-Adamantylmethyl(methyl)carbamoyl]phenyl]piperazine-1-carbonyl]-5-(trifluoromethyl)phenyl]phenyl]-1-hydroxy-N,N-dimethylnaphthalene-2-carboxamide |
| | ED10-32-01R | ED10-32 | 4-[5-[3-[4-[4-[1-Adamantylmethyl(methyl)carbamoyl]phenyl]piperazine-1-carbonyl]-5-(trifluoromethyl)phenyl]pyridin-2-yl]-1-hydroxy-N,N-dimethylnaphthalene-2-carboxamide |
| | ED10-33-01R | ED10-33 | 4-[4-[3-[4-[4-[1-Adamantylmethyl(methyl)carbamoyl]phenyl]piperazine-1-carbonyl]-5-tert-butylphenyl]phenyl]-1-hydroxy-N,N-dimethylnaphthalene-2-carboxamide |
| | ED10-34-01R | ED10-34 | 4-[5-[3-[4-[4-[1-Adamantylmethyl(methyl)carbamoyl]phenyl]piperazine-1-carbonyl]-5-tert-butylphenyl]pyridin-2-yl]-1-hydroxy-N,N-dimethylnaphthalene-2-carboxamide |
| | ED10-35-01R | ED10-35 | 4-[4-[4-[4-[4-[1-Adamantylmethyl(methyl)carbamoyl]phenyl]piperazine-1-carbonyl]naphthalen-1-yl]phenyl]-1-hydroxy-N,N-dimethylnaphthalene-2-carboxamide |
| | ED10-36-01R | ED10-36 | 4-[5-[4-[4-[4-[1-Adamantylmethyl(methyl)carbamoyl]phenyl]piperazine-1-carbonyl]naphthalen-1-yl]pyridin-2-yl]-1-hydroxy-N,N-dimethylnaphthalene-2-carboxamide |

| | | | |
|---|---|---|---|
| | ED10-37-01R | ED10-37 | 4-[4-[2-[3-[4-[4-[1-Adamantylmethyl(methyl)carbamoyl]phenyl]piperazin-1-yl]-3-oxopropyl]phenyl]phenyl]-1-hydroxy-N,N-dimethylnaphthalene-2-carboxamide |
| mixED10-01R | ED10-38-01R | ED10-38 | 4-[5-[2-[3-[4-[4-[1-Adamantylmethyl(methyl)carbamoyl]phenyl]piperazin-1-yl]-3-oxopropyl]phenyl]pyridin-2-yl]-1-hydroxy-N,N-dimethylnaphthalene-2-carboxamide |
| | ED10-39-01R | ED10-39 | 4-[4-[5-[4-[4-[1-Adamantylmethyl(methyl)carbamoyl]phenyl]piperazine-1-carbonyl]thiophen-3-yl]phenyl]-1-hydroxy-N,N-dimethylnaphthalene-2-carboxamide |
| | ED10-40-01R | ED10-40 | 4-[5-[5-[4-[4-[1-Adamantylmethyl(methyl)carbamoyl]phenyl]piperazine-1-carbonyl]thiophen-3-yl]pyridin-2-yl]-1-hydroxy-N,N-dimethylnaphthalene-2-carboxamide |
| | ED10-41-01R | ED10-41 | 4-[4-[1-[3-[4-[4-[1-Adamantylmethyl(methyl)carbamoyl]phenyl]piperazine-1-carbonyl]-5-(trifluoromethyl)phenyl]pyrazol-4-yl]phenyl]-1-hydroxy-N,N-dimethylnaphthalene-2-carboxamide |
| | ED10-42-01R | ED10-42 | 4-[5-[1-[3-[4-[4-[1-Adamantylmethyl(methyl)carbamoyl]phenyl]piperazine-1-carbonyl]-5-(trifluoromethyl)phenyl]pyrazol-4-yl]pyridin-2-yl]-1-hydroxy-N,N-dimethylnaphthalene-2-carboxamide |
| | ED10-43-01R | ED10-43 | 4-[4-[2-[2-[4-[4-[1-Adamantylmethyl(methyl)carbamoyl]phenyl]piperazine-1-carbonyl]phenyl]ethynyl]phenyl]-1-hydroxy-N,N-dimethylnaphthalene-2-carboxamide |
| | ED10-44-01R | ED10-44 | 4-[5-[2-[2-[4-[4-[1-Adamantylmethyl(methyl)carbamoyl]phenyl]piperazine-1-carbonyl]phenyl]ethynyl]pyridin-2-yl]-1-hydroxy-N,N-dimethylnaphthalene-2-carboxamide |
| | ED10-45-01R | ED10-45 | 4-[4-[2-[3-[4-[4-[1-Adamantylmethyl(methyl)carbamoyl]phenyl]piperazine-1-carbonyl]-5-(trifluoromethyl)phenyl]ethynyl]phenyl]-1-hydroxy-N,N-dimethylnaphthalene-2-carboxamide |
| | ED10-46-01R | ED10-46 | 4-[5-[2-[3-[4-[4-[1-Adamantylmethyl(methyl)carbamoyl]phenyl]piperazine-1-carbonyl]-5-(trifluoromethyl)phenyl]ethynyl]pyridin-2-yl]-1-hydroxy-N,N-dimethylnaphthalene-2-carboxamide |
| | ED10-47-01R | ED10-47 | 4-[4-[2-[5-[4-[4-[1-Adamantylmethyl(methyl)carbamoyl]phenyl]piperazine-1-carbonyl]pyridin-3-yl]ethynyl]phenyl]-1-hydroxy-N,N-dimethylnaphthalene-2-carboxamide |
| | ED10-48-01R | ED10-48 | 4-[5-[2-[5-[4-[4-[1-Adamantylmethyl(methyl)carbamoyl]phenyl]piperazine-1-carbonyl]pyridin-3-yl]ethynyl]pyridin-2-yl]-1-hydroxy-N,N-dimethylnaphthalene-2-carboxamide |
| | ED10-49-01R | ED10-49 | 4-[4-[2-[[4-[4-[1-Adamantylmethyl(methyl)carbamoyl]phenyl]piperazin-1-yl]methyl]phenyl]phenyl]-1-hydroxy-N,N-dimethylnaphthalene-2-carboxamide |
| | ED10-50-01R | ED10-50 | 4-[5-[2-[[4-[4-[1-Adamantylmethyl(methyl)carbamoyl]phenyl]piperazin-1-yl]methyl]phenyl]pyridin-2-yl]-1-hydroxy-N,N-dimethylnaphthalene-2-carboxamide |

| | | | |
|---|---|---|---|
| | ED10-51-01R | ED10-51 | 4-[4-[4-[[4-[4-[1-Adamantylmethyl(methyl)carbamoyl]phenyl]piperazin-1-yl]methyl]-2-methylphenyl]phenyl]-1-hydroxy-N,N-dimethylnaphthalene-2-carboxamide |
| mixED10-01R | ED10-52-01R | ED10-52 | 4-[5-[4-[[4-[4-[1-Adamantylmethyl(methyl)carbamoyl]phenyl]piperazin-1-yl]methyl]-2-methylphenyl]pyridin-2-yl]-1-hydroxy-N,N-dimethylnaphthalene-2-carboxamide |
| | ED10-53-01R | ED10-53 | 4-[4-[4-[[4-[4-[1-Adamantylmethyl(methyl)carbamoyl]phenyl]piperazin-1-yl]methyl]-3-ethylphenyl]phenyl]-1-hydroxy-N,N-dimethylnaphthalene-2-carboxamide |
| | ED10-54-01R | ED10-54 | 4-[5-[4-[[4-[4-[1-Adamantylmethyl(methyl)carbamoyl]phenyl]piperazin-1-yl]methyl]-3-ethylphenyl]pyridin-2-yl]-1-hydroxy-N,N-dimethylnaphthalene-2-carboxamide |
| | ED10-55-01R | ED10-55 | 4-[4-[4-[[4-[4-[1-Adamantylmethyl(methyl)carbamoyl]phenyl]piperazin-1-yl]methyl]-2-fluorophenyl]phenyl]-1-hydroxy-N,N-dimethylnaphthalene-2-carboxamide |
| | ED10-56-01R | ED10-56 | 4-[5-[4-[[4-[4-[1-Adamantylmethyl(methyl)carbamoyl]phenyl]piperazin-1-yl]methyl]-2-fluorophenyl]pyridin-2-yl]-1-hydroxy-N,N-dimethylnaphthalene-2-carboxamide |
| | ED10-57-01R | ED10-57 | 4-[4-[4-[[4-[4-[1-Adamantylmethyl(methyl)carbamoyl]phenyl]piperazin-1-yl]methyl]-3-methoxyphenyl]phenyl]-1-hydroxy-N,N-dimethylnaphthalene-2-carboxamide |
| | ED10-58-01R | ED10-58 | 4-[5-[4-[[4-[4-[1-Adamantylmethyl(methyl)carbamoyl]phenyl]piperazin-1-yl]methyl]-3-methoxyphenyl]pyridin-2-yl]-1-hydroxy-N,N-dimethylnaphthalene-2-carboxamide |
| | ED10-59-01R | ED10-59 | 4-[4-[3-[[4-[4-[1-Adamantylmethyl(methyl)carbamoyl]phenyl]piperazin-1-yl]methyl]-5-ethoxyphenyl]phenyl]-1-hydroxy-N,N-dimethylnaphthalene-2-carboxamide |
| | ED10-60-01R | ED10-60 | 4-[5-[3-[[4-[4-[1-Adamantylmethyl(methyl)carbamoyl]phenyl]piperazin-1-yl]methyl]-5-ethoxyphenyl]pyridin-2-yl]-1-hydroxy-N,N-dimethylnaphthalene-2-carboxamide |
| | ED10-61-01R | ED10-61 | 4-[4-[3-[[4-[4-[1-Adamantylmethyl(methyl)carbamoyl]phenyl]piperazin-1-yl]methyl]-5-propan-2-yloxyphenyl]phenyl]-1-hydroxy-N,N-dimethylnaphthalene-2-carboxamide |
| | ED10-62-01R | ED10-62 | 4-[5-[3-[[4-[4-[1-Adamantylmethyl(methyl)carbamoyl]phenyl]piperazin-1-yl]methyl]-5-propan-2-yloxyphenyl]pyridin-2-yl]-1-hydroxy-N,N-dimethylnaphthalene-2-carboxamide |
| | ED10-63-01R | ED10-63 | 4-[4-[4-[[4-[4-[1-Adamantylmethyl(methyl)carbamoyl]phenyl]piperazin-1-yl]methyl]-3,5-dimethoxyphenyl]phenyl]-1-hydroxy-N,N-dimethylnaphthalene-2-carboxamide |
| | ED10-64-01R | ED10-64 | 4-[5-[4-[[4-[4-[1-Adamantylmethyl(methyl)carbamoyl]phenyl]piperazin-1-yl]methyl]-3,5-dimethoxyphenyl]pyridin-2-yl]-1-hydroxy-N,N-dimethylnaphthalene-2-carboxamide |

| | | | |
|---|---|---|---|
| | ED10-65-01R | ED10-65 | 4-[4-[3-[[4-[4-[1-Adamantylmethyl(methyl)carbamoyl]phenyl]piperazin-1-yl]methyl]-5-(trifluoromethyl)phenyl]phenyl]-1-hydroxy-N,N-dimethylnaphthalene-2-carboxamide |
| mixED10-01R | ED10-66-01R | ED10-66 | 4-[5-[3-[[4-[4-[1-Adamantylmethyl(methyl)carbamoyl]phenyl]piperazin-1-yl]methyl]-5-(trifluoromethyl)phenyl]pyridin-2-yl]-1-hydroxy-N,N-dimethylnaphthalene-2-carboxamide |
| | ED10-67-01R | ED10-67 | 4-[4-[3-[[4-[4-[1-Adamantylmethyl(methyl)carbamoyl]phenyl]piperazin-1-yl]methyl]-5-(trifluoromethoxy)phenyl]phenyl]-1-hydroxy-N,N-dimethylnaphthalene-2-carboxamide |
| | ED10-68-01R | ED10-68 | 4-[5-[3-[[4-[4-[1-Adamantylmethyl(methyl)carbamoyl]phenyl]piperazin-1-yl]methyl]-5-(trifluoromethoxy)phenyl]pyridin-2-yl]-1-hydroxy-N,N-dimethylnaphthalene-2-carboxamide |
| | ED10-69-01R | ED10-69 | 4-[4-[4-[[4-[4-[1-Adamantylmethyl(methyl)carbamoyl]phenyl]piperazin-1-yl]methyl]naphthalen-1-yl]phenyl]-1-hydroxy-N,N-dimethylnaphthalene-2-carboxamide |
| | ED10-70-01R | ED10-70 | 4-[5-[4-[[4-[4-[1-Adamantylmethyl(methyl)carbamoyl]phenyl]piperazin-1-yl]methyl]naphthalen-1-yl]pyridin-2-yl]-1-hydroxy-N,N-dimethylnaphthalene-2-carboxamide |
| | ED10-71-01R | ED10-71 | 4-[4-[5-[[4-[4-[1-Adamantylmethyl(methyl)carbamoyl]phenyl]piperazin-1-yl]methyl]thiophen-3-yl]phenyl]-1-hydroxy-N,N-dimethylnaphthalene-2-carboxamide |
| | ED10-72-01R | ED10-72 | 4-[5-[5-[[4-[4-[1-Adamantylmethyl(methyl)carbamoyl]phenyl]piperazin-1-yl]methyl]thiophen-3-yl]pyridin-2-yl]-1-hydroxy-N,N-dimethylnaphthalene-2-carboxamide |
| | ED10-73-01R | ED10-73 | 4-[4-[2-[2-[[4-[4-[1-Adamantylmethyl(methyl)carbamoyl]phenyl]piperazin-1-yl]methyl]phenyl]ethynyl]phenyl]-1-hydroxy-N,N-dimethylnaphthalene-2-carboxamide |
| | ED10-74-01R | ED10-74 | 4-[5-[2-[2-[[4-[4-[1-Adamantylmethyl(methyl)carbamoyl]phenyl]piperazin-1-yl]methyl]phenyl]ethynyl]pyridin-2-yl]-1-hydroxy-N,N-dimethylnaphthalene-2-carboxamide |
| | ED10-75-01R | ED10-75 | N-(1-Adamantylmethyl)-6-[4-[[2-[4-[3-(dimethylcarbamoyl)-4-hydroxynaphthalen-1-yl]phenyl]phenyl]methyl]piperazin-1-yl]-N-methylpyridazine-3-carboxamide |
| | ED10-76-01R | ED10-76 | N-(1-Adamantylmethyl)-6-[4-[[2-[6-[3-(dimethylcarbamoyl)-4-hydroxynaphthalen-1-yl]pyridin-3-yl]phenyl]methyl]piperazin-1-yl]-N-methylpyridazine-3-carboxamide |
| | ED10-77-01R | ED10-77 | N-(1-Adamantylmethyl)-6-[4-[[4-[4-[3-(dimethylcarbamoyl)-4-hydroxynaphthalen-1-yl]phenyl]-3-methylphenyl]methyl]piperazin-1-yl]-N-methylpyridazine-3-carboxamide |
| | ED10-78-01R | ED10-78 | N-(1-Adamantylmethyl)-6-[4-[[4-[6-[3-(dimethylcarbamoyl)-4-hydroxynaphthalen-1-yl]pyridin-3-yl]-3-methylphenyl]methyl]piperazin-1-yl]-N-methylpyridazine-3-carboxamide |

| | | | |
|---|---|---|---|
| | ED10-79-01R | ED10-79 | N-(1-Adamantylmethyl)-6-[4-[[4-[4-[3-(dimethylcarbamoyl)-4-hydroxynaphthalen-1-yl]phenyl]-2-ethylphenyl]methyl]piperazin-1-yl]-N-methylpyridazine-3-carboxamide |
| mixED10-01R | ED10-80-01R | ED10-80 | N-(1-Adamantylmethyl)-6-[4-[[4-[6-[3-(dimethylcarbamoyl)-4-hydroxynaphthalen-1-yl]pyridin-3-yl]-2-ethylphenyl]methyl]piperazin-1-yl]-N-methylpyridazine-3-carboxamide |
| | ED10-81-01R | ED10-81 | N-(1-Adamantylmethyl)-6-[4-[[4-[4-[3-(dimethylcarbamoyl)-4-hydroxynaphthalen-1-yl]phenyl]-3-fluorophenyl]methyl]piperazin-1-yl]-N-methylpyridazine-3-carboxamide |
| | ED10-82-01R | ED10-82 | N-(1-Adamantylmethyl)-6-[4-[[4-[6-[3-(dimethylcarbamoyl)-4-hydroxynaphthalen-1-yl]pyridin-3-yl]-3-fluorophenyl]methyl]piperazin-1-yl]-N-methylpyridazine-3-carboxamide |
| | ED10-83-01R | ED10-83 | N-(1-Adamantylmethyl)-6-[4-[[4-[4-[3-(dimethylcarbamoyl)-4-hydroxynaphthalen-1-yl]phenyl]-2-methoxyphenyl]methyl]piperazin-1-yl]-N-methylpyridazine-3-carboxamide |
| | ED10-84-01R | ED10-84 | N-(1-Adamantylmethyl)-6-[4-[[4-[6-[3-(dimethylcarbamoyl)-4-hydroxynaphthalen-1-yl]pyridin-3-yl]-2-methoxyphenyl]methyl]piperazin-1-yl]-N-methylpyridazine-3-carboxamide |
| | ED10-85-01R | ED10-85 | N-(1-Adamantylmethyl)-6-[4-[[3-[4-[3-(dimethylcarbamoyl)-4-hydroxynaphthalen-1-yl]phenyl]-5-ethoxyphenyl]methyl]piperazin-1-yl]-N-methylpyridazine-3-carboxamide |
| | ED10-86-01R | ED10-86 | N-(1-Adamantylmethyl)-6-[4-[[3-[6-[3-(dimethylcarbamoyl)-4-hydroxynaphthalen-1-yl]pyridin-3-yl]-5-ethoxyphenyl]methyl]piperazin-1-yl]-N-methylpyridazine-3-carboxamide |
| | ED10-87-01R | ED10-87 | N-(1-Adamantylmethyl)-6-[4-[[3-[4-[3-(dimethylcarbamoyl)-4-hydroxynaphthalen-1-yl]phenyl]-5-propan-2-yloxyphenyl]methyl]piperazin-1-yl]-N-methylpyridazine-3-carboxamide |
| | ED10-88-01R | ED10-88 | N-(1-Adamantylmethyl)-6-[4-[[3-[6-[3-(dimethylcarbamoyl)-4-hydroxynaphthalen-1-yl]pyridin-3-yl]-5-propan-2-yloxyphenyl]methyl]piperazin-1-yl]-N-methylpyridazine-3-carboxamide |
| | ED10-89-01R | ED10-89 | N-(1-Adamantylmethyl)-6-[4-[[4-[4-[3-(dimethylcarbamoyl)-4-hydroxynaphthalen-1-yl]phenyl]-2,6-dimethoxyphenyl]methyl]piperazin-1-yl]-N-methylpyridazine-3-carboxamide |
| | ED10-90-01R | ED10-90 | N-(1-Adamantylmethyl)-6-[4-[[4-[6-[3-(dimethylcarbamoyl)-4-hydroxynaphthalen-1-yl]pyridin-3-yl]-2,6-dimethoxyphenyl]methyl]piperazin-1-yl]-N-methylpyridazine-3-carboxamide |
| | ED10-91-01R | ED10-91 | N-(1-Adamantylmethyl)-6-[4-[[3-[4-[3-(dimethylcarbamoyl)-4-hydroxynaphthalen-1-yl]phenyl]-5-(trifluoromethyl)phenyl]methyl]piperazin-1-yl]-N-methylpyridazine-3-carboxamide |
| | ED10-92-01R | ED10-92 | N-(1-Adamantylmethyl)-6-[4-[[3-[6-[3-(dimethylcarbamoyl)-4-hydroxynaphthalen-1-yl]pyridin-3-yl]-5-(trifluoromethyl)phenyl]methyl]piperazin-1-yl]-N-methylpyridazine-3-carboxamide |

| | | | |
|---|---|---|---|
| | ED10-93-01R | ED10-93 | N-(1-Adamantylmethyl)-6-[4-[[3-[4-[3-(dimethylcarbamoyl)-4-hydroxynaphthalen-1-yl]phenyl]-5-(trifluoromethoxy)phenyl]methyl]piperazin-1-yl]-N-methylpyridazine-3-carboxamide |
| mixED10-01R | ED10-94-01R | ED10-94 | N-(1-Adamantylmethyl)-6-[4-[[3-[6-[3-(dimethylcarbamoyl)-4-hydroxynaphthalen-1-yl]pyridin-3-yl]-5-(trifluoromethoxy)phenyl]methyl]piperazin-1-yl]-N-methylpyridazine-3-carboxamide |
| | ED10-95-01R | ED10-95 | N-(1-Adamantylmethyl)-6-[4-[[4-[4-[3-(dimethylcarbamoyl)-4-hydroxynaphthalen-1-yl]phenyl]naphthalen-1-yl]methyl]piperazin-1-yl]-N-methylpyridazine-3-carboxamide |
| | ED10-96-01R | ED10-96 | N-(1-Adamantylmethyl)-6-[4-[[4-[6-[3-(dimethylcarbamoyl)-4-hydroxynaphthalen-1-yl]pyridin-3-yl]naphthalen-1-yl]methyl]piperazin-1-yl]-N-methylpyridazine-3-carboxamide |
| | ED10-97-01R | ED10-97 | N-(1-Adamantylmethyl)-6-[4-[[4-[4-[3-(dimethylcarbamoyl)-4-hydroxynaphthalen-1-yl]phenyl]thiophen-2-yl]methyl]piperazin-1-yl]-N-methylpyridazine-3-carboxamide |
| | ED10-98-01R | ED10-98 | N-(1-Adamantylmethyl)-6-[4-[[4-[6-[3-(dimethylcarbamoyl)-4-hydroxynaphthalen-1-yl]pyridin-3-yl]thiophen-2-yl]methyl]piperazin-1-yl]-N-methylpyridazine-3-carboxamide |
| | ED10-99-01R | ED10-99 | N-(1-Adamantylmethyl)-6-[4-[[2-[2-[4-[3-(dimethylcarbamoyl)-4-hydroxynaphthalen-1-yl]phenyl]ethynyl]phenyl]methyl]piperazin-1-yl]-N-methylpyridazine-3-carboxamide |
| | ED10-100-01R | ED10-100 | N-(1-Adamantylmethyl)-6-[4-[[2-[2-[6-[3-(dimethylcarbamoyl)-4-hydroxynaphthalen-1-yl]pyridin-3-yl]ethynyl]phenyl]methyl]piperazin-1-yl]-N-methylpyridazine-3-carboxamide |

Bond formation reaction was carried out for carboxylic acid compound mixture mixED04-01R prepared in Example 3-6-5 through acylsulfonamide coupling reaction by the following method.

Example 3-6-10: Synthesis of mixture mixED11-01R

[Formula 522]

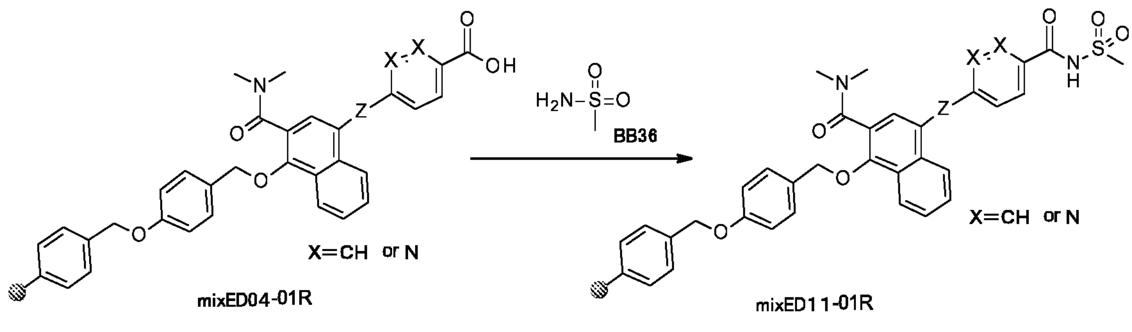


Mixture mixED04-01R (mixture that could contain 100 compounds, 50 mg, loading rate: 0.39 mmol/g, 0.019 mmol) shown in Table AG-D-04 and NMP (0.75 mL) were added to a 1.5 mL glass vial and shaken at room temperature for 1 hour. HATU (43.9 mg, 0.116 mmol) and 2,6-lutidine (13.5 μL, 0.116 mmol) were added thereto at room temperature, and the mixture was shaken at 40°C for 3 hours. Methanesulfonamide (BB36, 12.8 mg, 0.135 mmol) and phosphazene PltBu (73.5 μL, 0.289 mmol) were added thereto at room temperature, and the mixture was shaken at 40°C for 3 hours. n-Propylamine (16 μL, 0.193 mmol) was added thereto at room temperature, and the mixture was shaken for 1 hour. The suspension of the reaction solution and the resin was transferred to a syringe with a filter using NMP, repetitively washed three times with NMP (1 mL), three times with HOAt/NMP (0.2 M, 1 mL), three times with NMP (1 mL), three

times with MeOH (1 mL), three times with DCM (1 mL), and three times with heptane (1 mL), and then dried under reduced pressure to obtain the title mixture (mixED11-01R) shown in Table ED11.

[Table 110]

[Table ED11]

| No. of mixture | No. of compound that may be contained | Compound No. after cleavage | Compound name after cleavage |
|---|---|---|---|
| mixED11-01R | ED11-01-01R | ED11-01 | 1-Hydroxy-N,N-dimethyl-4-[4-[4-[1-[4-[4-(methylsulfonylcarbamoyl)phenyl]piperazin-1-yl]ethyl]phenyl]phenyl]naphthalene-2-carboxamide |
| | ED11-02-01R | ED11-02 | 1-Hydroxy-N,N-dimethyl-4-[5-[4-[1-[4-[4-(methylsulfonylcarbamoyl)phenyl]piperazin-1-yl]ethyl]phenyl]pyridin-2-yl]naphthalene-2-carboxamide |
| | ED11-03-01R | ED11-03 | 4-[4-[3-Fluoro-4-[1-[4-[4-(methylsulfonylcarbamoyl)phenyl]piperazin-1-yl]ethyl]phenyl]phenyl]-1-hydroxy-N,N-dimethylnaphthalene-2-carboxamide |
| | ED11-04-01R | ED11-04 | 4-[5-[3-Fluoro-4-[1-[4-[4-(methylsulfonylcarbamoyl)phenyl]piperazin-1-yl]ethyl]phenyl]pyridin-2-yl]-1-hydroxy-N,N-dimethylnaphthalene-2-carboxamide |
| | ED11-05-01R | ED11-05 | 6-[4-[4-[4-[3-(Dimethylcarbamoyl)-4-hydroxynaphthalen-1-yl]phenyl]-3-methylbenzoyl]piperazin-1-yl]-N-methylsulfonylpyridazine-3-carboxamide |
| | ED11-06-01R | ED11-06 | 6-[4-[4-[6-[3-(Dimethylcarbamoyl)-4-hydroxynaphthalen-1-yl]pyridin-3-yl]-3-methylbenzoyl]piperazin-1-yl]-N-methylsulfonylpyridazine-3-carboxamide |
| | ED11-07-01R | ED11-07 | 6-[4-[4-[4-[3-(Dimethylcarbamoyl)-4-hydroxynaphthalen-1-yl]phenyl]-3-fluorobenzoyl]piperazin-1-yl]-N-methylsulfonylpyridazine-3-carboxamide |
| | ED11-08-01R | ED11-08 | 6-[4-[4-[6-[3-(Dimethylcarbamoyl)-4-hydroxynaphthalen-1-yl]pyridin-3-yl]-3-fluorobenzoyl]piperazin-1-yl]-N-methylsulfonylpyridazine-3-carboxamide |
| | ED11-09-01R | ED11-09 | 6-[4-[3-[4-[3-(Dimethylcarbamoyl)-4-hydroxynaphthalen-1-yl]phenyl]-5-(trifluoromethyl)benzoyl]piperazin-1-yl]-N-methylsulfonylpyridazine-3-carboxamide |
| | ED11-10-01R | ED11-10 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-methylsulfonylpyridazine-3-carboxamide |
| mixED11-01R | ED11-11-01R | ED11-11 | 6-[4-[3-tert-Butyl-5-[4-[3-(dimethylcarbamoyl)-4-hydroxynaphthalen-1-yl]phenyl]benzoyl]piperazin-1-yl]-N-methylsulfonylpyridazine-3-carboxamide |

| | | | |
|---|---|---|---|
| | ED11-12-01R | ED11-12 | 6-[4-[3-tert-Butyl-5-[6-[3-(dimethylcarbamoyl)-4-hydroxynaphthalen-1-yl]pyridin-3-yl]benzoyl]piperazin-1-yl]-N-methylsulfonylpyridazine-3-carboxamide |
| | ED11-13-01R | ED11-13 | 6-[4-[4-[4-[3-(Dimethylcarbamoyl)-4-hydroxynaphthalen-1-yl]phenyl]naphthalene-1-carbonyl]piperazin-1-yl]-N-methylsulfonylpyridazine-3-carboxamide |
| | ED11-14-01R | ED11-14 | 6-[4-[4-[6-[3-(Dimethylcarbamoyl)-4-hydroxynaphthalen-1-yl]pyridin-3-yl]naphthalene-1-carbonyl]piperazin-1-yl]-N-methylsulfonylpyridazine-3-carboxamide |
| | ED11-15-01R | ED11-15 | 6-[4-[3-[2-[4-[3-(Dimethylcarbamoyl)-4-hydroxynaphthalen-1-yl]phenyl]phenyl]propanoyl]piperazin-1-yl]-N-methylsulfonylpyridazine-3-carboxamide |
| | ED11-16-01R | ED11-16 | 6-[4-[3-[2-[6-[3-(Dimethylcarbamoyl)-4-hydroxynaphthalen-1-yl]pyridin-3-yl]phenyl]propanoyl]piperazin-1-yl]-N-methylsulfonylpyridazine-3-carboxamide |
| | ED11-17-01R | ED11-17 | 6-[4-[4-[4-[3-(Dimethylcarbamoyl)-4-hydroxynaphthalen-1-yl]phenyl]thiophene-2-carbonyl]piperazin-1-yl]-N-methylsulfonylpyridazine-3-carboxamide |
| | ED11-18-01R | ED11-18 | 6-[4-[4-[6-[3-(Dimethylcarbamoyl)-4-hydroxynaphthalen-1-yl]pyridin-3-yl]thiophene-2-carbonyl]piperazin-1-yl]-N-methylsulfonylpyridazine-3-carboxamide |
| | ED11-19-01R | ED11-19 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-methylsulfonylpyridazine-3-carboxamide |
| | ED11-20-01R | ED11-20 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-methylsulfonylpyridazine-3-carboxamide |
| | ED11-21-01R | ED11-21 | 6-[4-[2-[2-[4-[3-(Dimethylcarbamoyl)-4-hydroxynaphthalen-1-yl]phenyl]ethynyl]benzoyl]piperazin-1-yl]-N-methylsulfonylpyridazine-3-carboxamide |
| | ED11-22-01R | ED11-22 | 6-[4-[2-[2-[6-[3-(Dimethylcarbamoyl)-4-hydroxynaphthalen-1-yl]pyridin-3-yl]ethynyl]benzoyl]piperazin-1-yl]-N-methylsulfonylpyridazine-3-carboxamide |
| | ED11-23-01R | ED11-23 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-methylsulfonylpyridazine-3-carboxamide |
| | ED11-24-01R | ED11-24 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-methylsulfonylpyridazine-3-carboxamide |
| | ED11-25-01R | ED11-25 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-methylsulfonylpyridazine-3-carboxamide |
| | ED11-26-01R | ED11-26 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-methylsulfonylpyridazine-3-carboxamide |
| mixED11-01R | ED11-27-01R | ED11-27 | 1-Hydroxy-N,N-dimethyl-4-[4-[2-methyl-4-[4-[4-(methylsulfonylcarbamoyl)phenyl]piperazine-1-carbonyl]phenyl]phenyl]naphthalene-2-carboxamide |
| | ED11-28-01R | ED11-28 | 1-Hydroxy-N,N-dimethyl-4-[5-[2-methyl-4-[4-[4-(methylsulfonylcarbamoyl)phenyl]piperazine-1-carbonyl]phenyl]pyridin-2-yl]naphthalene-2-carboxamide |
| | ED11-29-01R | ED11-29 | 4-[4-[2-Fluoro-4-[4-[4-(methylsulfonylcarbamoyl)phenyl]piperazine-1-carbonyl]phenyl]phenyl]-1-hydroxy-N,N-dimethylnaphthalene-2-carboxamide |
| | ED11-30-01R | ED11-30 | 4-[5-[2-Fluoro-4-[4-[4-(methylsulfonylcarbamoyl)phenyl]piperazine-1-carbonyl]phenyl]pyridin-2-yl]-1-hydroxy-N,N-dimethylnaphthalene-2-carboxamide |

| | | | |
|---|---|---|---|
| | ED11-31-01R | ED11-31 | 1-Hydroxy-N,N-dimethyl-4-[4-[3-[4-[4-(methylsulfonylcarbamoyl)phenyl]piperazine-1-carbonyl]-5-(trifluoromethyl)phenyl]phenyl]naphthalene-2-carboxamide |
| | ED11-32-01R | ED11-32 | 1-Hydroxy-N,N-dimethyl-4-[5-[3-[4-[4-(methylsulfonylcarbamoyl)phenyl]piperazine-1-carbonyl]-5-(trifluoromethyl)phenyl]pyridin-2-yl]naphthalene-2-carboxamide |
| | ED11-33-01R | ED11-33 | 4-[4-[3-tert-Butyl-5-[4-[4-(methylsulfonylcarbamoyl)phenyl]piperazine-1-carbonyl]phenyl]phenyl]-1-hydroxy-N,N-dimethylnaphthalene-2-carboxamide |
| | ED11-34-01R | ED11-34 | 4-[5-[3-tert-Butyl-5-[4-[4-(methylsulfonylcarbamoyl)phenyl]piperazine-1-carbonyl]phenyl]pyridin-2-yl]-1-hydroxy-N,N-dimethylnaphthalene-2-carboxamide |
| | ED11-35-01R | ED11-35 | 1-Hydroxy-N,N-dimethyl-4-[4-[4-[4-[4-(methylsulfonylcarbamoyl)phenyl]piperazine-1-carbonyl]naphthalen-1-yl]phenyl]naphthalene-2-carboxamide |
| | ED11-36-01R | ED11-36 | 1-Hydroxy-N,N-dimethyl-4-[5-[4-[4-[4-(methylsulfonylcarbamoyl)phenyl]piperazine-1-carbonyl]naphthalen-1-yl]pyridin-2-yl]naphthalene-2-carboxamide |
| | ED11-37-01R | ED11-37 | 1-Hydroxy-N,N-dimethyl-4-[4-[2-[3-[4-[4-(methylsulfonylcarbamoyl)phenyl]piperazin-1-yl]-3-oxopropyl]phenyl]phenyl]naphthalene-2-carboxamide |
| | ED11-38-01R | ED11-38 | 1-Hydroxy-N,N-dimethyl-4-[5-[2-[3-[4-[4-(methylsulfonylcarbamoyl)phenyl]piperazin-1-yl]-3-oxopropyl]phenyl]pyridin-2-yl]naphthalene-2-carboxamide |
| | ED11-39-01R | ED11-39 | 1-Hydroxy-N,N-dimethyl-4-[4-[5-[4-[4-(methylsulfonylcarbamoyl)phenyl]piperazine-1-carbonyl]thiophen-3-yl]phenyl]naphthalene-2-carboxamide |
| | ED11-40-01R | ED11-40 | 1-Hydroxy-N,N-dimethyl-4-[5-[5-[4-[4-(methylsulfonylcarbamoyl)phenyl]piperazine-1-carbonyl]thiophen-3-yl]pyridin-2-yl]naphthalene-2-carboxamide |
| | ED11-41-01R | ED11-41 | 1-Hydroxy-N,N-dimethyl-4-[4-[1-[3-[4-[4-(methylsulfonylcarbamoyl)phenyl]piperazine-1-carbonyl]-5-(trifluoromethyl)phenyl]pyrazol-4-yl]phenyl]naphthalene-2-carboxamide |
| mixED11-01R | ED11-42-01R | ED11-42 | 1-Hydroxy-N,N-dimethyl-4-[5-[1-[3-[4-[4-(methylsulfonylcarbamoyl)phenyl]piperazine-1-carbonyl]-5-(trifluoromethyl)phenyl]pyrazol-4-yl]pyridin-2-yl]naphthalene-2-carboxamide |
| | ED11-43-01R | ED11-43 | 1-Hydroxy-N,N-dimethyl-4-[4-[2-[2-[4-[4-(methylsulfonylcarbamoyl)phenyl]piperazine-1-carbonyl]phenyl]ethynyl]phenyl]naphthalene-2-carboxamide |
| | ED11-44-01R | ED11-44 | 1-Hydroxy-N,N-dimethyl-4-[5-[2-[2-[4-[4-(methylsulfonylcarbamoyl)phenyl]piperazine-1-carbonyl]phenyl]ethynyl]pyridin-2-yl]naphthalene-2-carboxamide |
| | ED11-45-01R | ED11-45 | 1-Hydroxy-N,N-dimethyl-4-[4-[2-[3-[4-[4-(methylsulfonylcarbamoyl)phenyl]piperazine-1-carbonyl]-5-(trifluoromethyl)phenyl]ethynyl]phenyl]naphthalene-2-carboxamide |
| | ED11-46-01R | ED11-46 | 1-Hydroxy-N,N-dimethyl-4-[5-[2-[3-[4-[4-(methylsulfonylcarbamoyl)phenyl]piperazine-1-carbonyl]-5-(trifluoromethyl)phenyl]ethynyl]pyridin-2-yl]naphthalene-2-carboxamide |
| | ED11-47-01R | ED11-47 | 1-Hydroxy-N,N-dimethyl-4-[4-[2-[5-[4-[4-(methylsulfonylcarbamoyl)phenyl]piperazine-1-carbonyl]pyridin-3-yl]ethynyl]phenyl]naphthalene-2-carboxamide |
| | ED11-48-01R | ED11-48 | 1-Hydroxy-N,N-dimethyl-4-[5-[2-[5-[4-[4-(methylsulfonylcarbamoyl)phenyl]piperazine-1-carbonyl]pyridin-3-yl]ethynyl]pyridin-2-yl]naphthalene-2-carboxamide |

| | | | |
|---|---|---|---|
| | ED11-49-01R | ED11-49 | 1-Hydroxy-N,N-dimethyl-4-[4-[2-[[4-[4-(methylsulfonylcarbamoyl)phenyl]piperazin-1-yl]methyl]phenyl]phenyl]naphthalene-2-carboxamide |
| | ED11-50-01R | ED11-50 | 1-Hydroxy-N,N-dimethyl-4-[5-[2-[[4-[4-(methylsulfonylcarbamoyl)phenyl]piperazin-1-yl]methyl]phenyl]pyridin-2-yl]naphthalene-2-carboxamide |
| | ED11-51-01R | ED11-51 | 1-Hydroxy-N,N-dimethyl-4-[4-[2-methyl-4-[[4-[4-(methylsulfonylcarbamoyl)phenyl]piperazin-1-yl]methyl]phenyl]phenyl]naphthalene-2-carboxamide |
| | ED11-52-01R | ED11-52 | 1-Hydroxy-N,N-dimethyl-4-[5-[2-methyl-4-[[4-[4-(methylsulfonylcarbamoyl)phenyl]piperazin-1-yl]methyl]phenyl]pyridin-2-yl]naphthalene-2-carboxamide |
| | ED11-53-01R | ED11-53 | 4-[4-[3-Ethyl-4-[[4-[4-(methylsulfonylcarbamoyl)phenyl]piperazin-1-yl]methyl]phenyl]phenyl]-1-hydroxy-N,N-dimethylnaphthalene-2-carboxamide |
| | ED11-54-01R | ED11-54 | 4-[5-[3-Ethyl-4-[[4-[4-(methylsulfonylcarbamoyl)phenyl]piperazin-1-yl]methyl]phenyl]pyridin-2-yl]-1-hydroxy-N,N-dimethylnaphthalene-2-carboxamide |
| | ED11-55-01R | ED11-55 | 4-[4-[2-Fluoro-4-[[4-[4-(methylsulfonylcarbamoyl)phenyl]piperazin-1-yl]methyl]phenyl]phenyl]-1-hydroxy-N,N-dimethylnaphthalene-2-carboxamide |
| | ED11-56-01R | ED11-56 | 4-[5-[2-Fluoro-4-[[4-[4-(methylsulfonylcarbamoyl)phenyl]piperazin-1-yl]methyl]phenyl]pyridin-2-yl]-1-hydroxy-N,N-dimethylnaphthalene-2-carboxamide |
| mixED11-01R | ED11-57-01R | ED11-57 | 1-Hydroxy-4-[4-[3-methoxy-4-[[4-[4-(methylsulfonylcarbamoyl)phenyl]piperazin-1-yl]methyl]phenyl]phenyl]-N,N-dimethylnaphthalene-2-carboxamide |
| | ED11-58-01R | ED11-58 | 1-Hydroxy-4-[5-[3-methoxy-4-[[4-[4-(methylsulfonylcarbamoyl)phenyl]piperazin-1-yl]methyl]phenyl]pyridin-2-yl]-N,N-dimethylnaphthalene-2-carboxamide |
| | ED11-59-01R | ED11-59 | 4-[4-[3-Ethoxy-5-[[4-[4-(methylsulfonylcarbamoyl)phenyl]piperazin-1-yl]methyl]phenyl]phenyl]-1-hydroxy-N,N-dimethylnaphthalene-2-carboxamide |
| | ED11-60-01R | ED11-60 | 4-[5-[3-Ethoxy-5-[[4-[4-(methylsulfonylcarbamoyl)phenyl]piperazin-1-yl]methyl]phenyl]pyridin-2-yl]-1-hydroxy-N,N-dimethylnaphthalene-2-carboxamide |
| | ED11-61-01R | ED11-61 | 1-Hydroxy-N,N-dimethyl-4-[4-[3-[[4-[4-(methylsulfonylcarbamoyl)phenyl]piperazin-1-yl]methyl]-5-propan-2-yloxyphenyl]phenyl]naphthalene-2-carboxamide |
| | ED11-62-01R | ED11-62 | 1-Hydroxy-N,N-dimethyl-4-[5-[3-[[4-[4-(methylsulfonylcarbamoyl)phenyl]piperazin-1-yl]methyl]-5-propan-2-yloxyphenyl]pyridin-2-yl]naphthalene-2-carboxamide |
| | ED11-63-01R | ED11-63 | 4-[4-[3,5-Dimethoxy-4-[[4-[4-(methylsulfonylcarbamoyl)phenyl]piperazin-1-yl]methyl]phenyl]phenyl]-1-hydroxy-N,N-dimethylnaphthalene-2-carboxamide |
| | ED11-64-01R | ED11-64 | 4-[5-[3,5-Dimethoxy-4-[[4-[4-(methylsulfonylcarbamoyl)phenyl]piperazin-1-yl]methyl]phenyl]pyridin-2-yl]-1-hydroxy-N,N-dimethylnaphthalene-2-carboxamide |
| | ED11-65-01R | ED11-65 | 1-Hydroxy-N,N-dimethyl-4-[4-[3-[[4-[4-(methylsulfonylcarbamoyl)phenyl]piperazin-1-yl]methyl]-5-(trifluoromethyl)phenyl]phenyl]naphthalene-2-carboxamide |
| | ED11-66-01R | ED11-66 | 1-Hydroxy-N,N-dimethyl-4-[5-[3-[[4-[4-(methylsulfonylcarbamoyl)phenyl]piperazin-1-yl]methyl]-5-(trifluoromethyl)phenyl]pyridin-2-yl]naphthalene-2-carboxamide |
| | ED11-67-01R | ED11-67 | 1-Hydroxy-N,N-dimethyl-4-[4-[3-[[4-[4-(methylsulfonylcarbamoyl)phenyl]piperazin-1-yl]methyl]-5-(trifluoromethoxy)phenyl]phenyl]naphthalene-2-carboxamide |

| | | | |
|---|---|---|---|
| | ED11-68-01R | ED11-68 | 1-Hydroxy-N,N-dimethyl-4-[5-[3-[[4-[4-(methylsulfonylcarbamoyl)phenyl]piperazin-1-yl]methyl]-5-(trifluoromethoxy)phenyl]pyridin-2-yl]naphthalene-2-carboxamide |
| | ED11-69-01R | ED11-69 | 1-Hydroxy-N,N-dimethyl-4-[4-[4-[[4-[4-(methylsulfonylcarbamoyl)phenyl]piperazin-1-yl]methyl]naphthalen-1-yl]phenyl]naphthalene-2-carboxamide |
| | ED11-70-01R | ED11-70 | 1-Hydroxy-N,N-dimethyl-4-[5-[4-[[4-[4-(methylsulfonylcarbamoyl)phenyl]piperazin-1-yl]methyl]naphthalen-1-yl]pyridin-2-yl]naphthalene-2-carboxamide |
| | ED11-71-01R | ED11-71 | 1-Hydroxy-N,N-dimethyl-4-[4-[5-[[4-[4-(methylsulfonylcarbamoyl)phenyl]piperazin-1-yl]methyl]thiophen-3-yl]phenyl]naphthalene-2-carboxamide |
| mixED11-01R | ED11-72-01R | ED11-72 | 1-Hydroxy-N,N-dimethyl-4-[5-[5-[[4-[4-(methylsulfonylcarbamoyl)phenyl]piperazin-1-yl]methyl]thiophen-3-yl]pyridin-2-yl]naphthalene-2-carboxamide |
| | ED11-73-01R | ED11-73 | 1-Hydroxy-N,N-dimethyl-4-[4-[2-[2-[[4-[4-(methylsulfonylcarbamoyl)phenyl]piperazin-1-yl]methyl]phenyl]ethynyl]phenyl]naphthalene-2-carboxamide |
| | ED11-74-01R | ED11-74 | 1-Hydroxy-N,N-dimethyl-4-[5-[2-[2-[[4-[4-(methylsulfonylcarbamoyl)phenyl]piperazin-1-yl]methyl]phenyl]ethynyl]pyridin-2-yl]naphthalene-2-carboxamide |
| | ED11-75-01R | ED11-75 | 6-[4-[[2-[4-[3-(Dimethylcarbamoyl)-4-hydroxynaphthalen-1-yl]phenyl]phenyl]methyl]piperazin-1-yl]-N-methylsulfonylpyridazine-3-carboxamide |
| | ED11-76-01R | ED11-76 | 6-[4-[[2-[6-[3-(Dimethylcarbamoyl)-4-hydroxynaphthalen-1-yl]pyridin-3-yl]phenyl]methyl]piperazin-1-yl]-N-methylsulfonylpyridazine-3-carboxamide |
| | ED11-77-01R | ED11-77 | 6-[4-[[4-[4-[3-(Dimethylcarbamoyl)-4-hydroxynaphthalen-1-yl]phenyl]-3-methylphenyl]methyl]piperazin-1-yl]-N-methylsulfonylpyridazine-3-carboxamide |
| | ED11-78-01R | ED11-78 | 6-[4-[[4-[6-[3-(Dimethylcarbamoyl)-4-hydroxynaphthalen-1-yl]pyridin-3-yl]-3-methylphenyl]methyl]piperazin-1-yl]-N-methylsulfonylpyridazine-3-carboxamide |
| | ED11-79-01R | ED11-79 | 6-[4-[[4-[4-[3-(Dimethylcarbamoyl)-4-hydroxynaphthalen-1-yl]phenyl]-2-ethylphenyl]methyl]piperazin-1-yl]-N-methylsulfonylpyridazine-3-carboxamide |
| | ED11-80-01R | ED11-80 | 6-[4-[[4-[6-[3-(Dimethylcarbamoyl)-4-hydroxynaphthalen-1-yl]pyridin-3-yl]-2-ethylphenyl]methyl]piperazin-1-yl]-N-methylsulfonylpyridazine-3-carboxamide |
| | ED11-81-01R | ED11-81 | 6-[4-[[4-[4-[3-(Dimethylcarbamoyl)-4-hydroxynaphthalen-1-yl]phenyl]-3-fluorophenyl]methyl]piperazin-1-yl]-N-methylsulfonylpyridazine-3-carboxamide |
| | ED11-82-01R | ED11-82 | 6-[4-[[4-[6-[3-(Dimethylcarbamoyl)-4-hydroxynaphthalen-1-yl]pyridin-3-yl]-3-fluorophenyl]methyl]piperazin-1-yl]-N-methylsulfonylpyridazine-3-carboxamide |
| | ED11-83-01R | ED11-83 | 6-[4-[[4-[4-[3-(Dimethylcarbamoyl)-4-hydroxynaphthalen-1-yl]phenyl]-2-methoxyphenyl]methyl]piperazin-1-yl]-N-methylsulfonylpyridazine-3-carboxamide |
| | ED11-84-01R | ED11-84 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-methylsulfonylpyridazine-3-carboxamide |
| | ED11-85-01R | ED11-85 | 6-[4-[[3-[4-[3-(Dimethylcarbamoyl)-4-hydroxynaphthalen-1-yl]phenyl]-5-ethoxyphenyl]methyl]piperazin-1-yl]-N-methylsulfonylpyridazine-3-carboxamide |
| | ED11-86-01R | ED11-86 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-methylsulfonylpyridazine-3-carboxamide |

| | | | |
|---|---|---|---|
| | ED11-87-01R | ED11-87 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-methylsulfonylpyridazine-3-carboxamide |
| mixED11-01R | ED11-88-01R | ED11-88 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-methylsulfonylpyridazine-3-carboxamide |
| | ED11-89-01R | ED11-89 | 6-[4-[[4-[4-[3-(Dimethylcarbamoyl)-4-hydroxynaphthalen-1-yl]phenyl]-2,6-dimethoxyphenyl]methyl]piperazin-1-yl]-N-methylsulfonylpyridazine-3-carboxamide |
| | ED11-90-01R | ED11-90 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-methylsulfonylpyridazine-3-carboxamide |
| | ED11-91-01R | ED11-91 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-methylsulfonylpyridazine-3-carboxamide |
| | ED11-92-01R | ED11-92 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-methylsulfonylpyridazine-3-carboxamide |
| | ED11-93-01R | ED11-93 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-methylsulfonylpyridazine-3-carboxamide |
| | ED11-94-01R | ED11-94 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-methylsulfonylpyridazine-3-carboxamide |
| | ED11-95-01R | ED11-95 | 6-[4-[[4-[4-[3-(Dimethylcarbamoyl)-4-hydroxynaphthalen-1-yl]phenyl]naphthalen-1-yl]methyl]piperazin-1-yl]-N-methylsulfonylpyridazine-3-carboxamide |
| | ED11-96-01R | ED11-96 | 6-[4-[[4-[6-[3-(Dimethylcarbamoyl)-4-hydroxynaphthalen-1-yl]pyridin-3-yl]naphthalen-1-yl]methyl]piperazin-1-yl]-N-methylsulfonylpyridazine-3-carboxamide |
| | ED11-97-01R | ED11-97 | 6-[4-[[4-[4-[3-(Dimethylcarbamoyl)-4-hydroxynaphthalen-1-yl]phenyl]thiophen-2-yl]methyl]piperazin-1-yl]-N-methylsulfonylpyridazine-3-carboxamide |
| | ED11-98-01R | ED11-98 | 6-[4-[[4-[6-[3-(Dimethylcarbamoyl)-4-hydroxynaphthalen-1-yl]pyridin-3-yl]thiophen-2-yl]methyl]piperazin-1-yl]-N-methylsulfonylpyridazine-3-carboxamide |
| | ED11-99-01R | ED11-99 | 6-[4-[[2-[2-[4-[3-(Dimethylcarbamoyl)-4-hydroxynaphthalen-1-yl]phenyl]ethynyl]phenyl]methyl]piperazin-1-yl]-N-methylsulfonylpyridazine-3-carboxamide |
| | ED11-100-01R | ED11-100 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-methylsulfonylpyridazine-3-carboxamide |

Example 3-6-11: Synthesis of mixED12-01R to mixED16-01R

In the same manner as in Example 3-6-10, the title mixture (mixED12-01) shown in Table ED12 was obtained using mixED04-01R and sulfonamide reagent BB37 shown in Table LBB02; the title mixture (mixED13-01) shown in Table ED 13 was obtained using BB38; the title mixture (mixED14-01) shown in Table ED14 was obtained using BB39; the title mixture (mixED15-01) shown in Table ED15 was obtained using BB40; and the title mixture (mixED16-01) shown in Table ED16 was obtained using BB41.

[Table 111]

[Table ED12]

| No. of mixture | No. of compound that may be contained | Compound No. after cleavage | Compound name after cleavage |
|---|---|---|---|
| mixED12-01R | ED12-01-01R | ED12-01 | 4-[4-[4-[1-[4-[4-(tert-Butylsulfonylcarbamoyl)phenyl]piperazin-1-yl]ethyl]phenyl]phenyl]-1-hydroxy-N,N-dimethylnaphthalene-2-carboxamide |
| | ED12-02-01R | ED12-02 | 4-[5-[4-[1-[4-[4-(tert-Butylsulfonylcarbamoyl)phenyl]piperazin-1-yl]ethyl]phenyl]pyridin-2-yl]-1-hydroxy-N,N-dimethylnaphthalene-2-carboxamide |
| | ED12-03-01R | ED12-03 | 4-[4-[4-[1-[4-[4-(tert-Butylsulfonylcarbamoyl)phenyl]piperazin-1-yl]ethyl]-3-fluorophenyl]phenyl]-1-hydroxy-N,N-dimethylnaphthalene-2-carboxamide |
| | ED12-04-01R | ED12-04 | 4-[5-[4-[1-[4-[4-(tert-Butylsulfonylcarbamoyl)phenyl]piperazin-1-yl]ethyl]-3-fluorophenyl]pyridin-2-yl]-1-hydroxy-N,N-dimethylnaphthalene-2-carboxamide |
| | ED12-05-01R | ED12-05 | N-tert-Butylsulfonyl-6-[4-[4-[4-[3-(dimethylcarbamoyl)-4-hydroxynaphthalen-1-yl]phenyl]-3-methylbenzoyl]piperazin-1-yl]pyridazine-3-carboxamide |
| | ED12-06-01R | ED12-06 | N-tert-Butylsulfonyl-6-[4-[4-[6-[3-(dimethylcarbamoyl)-4-hydroxynaphthalen-1-yl]pyridin-3-yl]-3-methylbenzoyl]piperazin-1-yl]pyridazine-3-carboxamide |
| | ED12-07-01R | ED12-07 | N-tert-Butylsulfonyl-6-[4-[4-[4-[3-(dimethylcarbamoyl)-4-hydroxynaphthalen-1-yl]phenyl]-3-fluorobenzoyl]piperazin-1-yl]pyridazine-3-carboxamide |
| | ED12-08-01R | ED12-08 | N-tert-Butylsulfonyl-6-[4-[4-[6-[3-(dimethylcarbamoyl)-4-hydroxynaphthalen-1-yl]pyridin-3-yl]-3-fluorobenzoyl]piperazin-1-yl]pyridazine-3-carboxamide |
| | ED12-09-01R | ED12-09 | N-tert-Butylsulfonyl-6-[4-[3-[4-[3-(dimethylcarbamoyl)-4-hydroxynaphthalen-1-yl]phenyl]-5-(trifluoromethyl)benzoyl]piperazin-1-yl]pyridazine-3-carboxamide |
| | ED12-10-01R | ED12-10 | N-tert-Butylsulfonyl-6-[4-[3-[6-[3-(dimethylcarbamoyl)-4-hydroxynaphthalen-1-yl]pyridin-3-yl]-5-(trifluoromethyl)benzoyl]piperazin-1-yl]pyridazine-3-carboxamide |
| | ED12-11-01R | ED12-11 | 6-[4-[3-tert-Butyl-5-[4-[3-(dimethylcarbamoyl)-4-hydroxynaphthalen-1-yl]phenyl]benzoyl]piperazin-1-yl]-N-tert-butylsulfonylpyridazine-3-carboxamide |
| | ED12-12-01R | ED12-12 | 6-[4-[3-tert-Butyl-5-[6-[3-(dimethylcarbamoyl)-4-hydroxynaphthalen-1-yl]pyridin-3-yl]benzoyl]piperazin-1-yl]-N-tert-butylsulfonylpyridazine-3-carboxamide |
| mixED12-01R | ED12-13-01R | ED12-13 | N-tert-Butylsulfonyl-6-[4-[4-[4-[3-(dimethylcarbamoyl)-4-hydroxynaphthalen-1-yl]phenyl]naphthalene-1-carbonyl]piperazin-1-yl]pyridazine-3-carboxamide |
| | ED12-14-01R | ED12-14 | N-tert-Butylsulfonyl-6-[4-[4-[6-[3-(dimethylcarbamoyl)-4-hydroxynaphthalen-1-yl]pyridin-3-yl]naphthalene-1-carbonyl]piperazin-1-yl]pyridazine-3-carboxamide |
| | ED12-15-01R | ED12-15 | N-tert-Butylsulfonyl-6-[4-[3-[2-[4-[3-(dimethylcarbamoyl)-4-hydroxynaphthalen-1-yl]phenyl]phenyl]propanoyl]piperazin-1-yl]pyridazine-3-carboxamide |
| | ED12-16-01R | ED12-16 | N-tert-Butylsulfonyl-6-[4-[3-[2-[6-[3-(dimethylcarbamoyl)-4-hydroxynaphthalen-1-yl]pyridin-3-yl]phenyl]propanoyl]piperazin-1-yl]pyridazine-3-carboxamide |
| | ED12-17-01R | ED12-17 | N-tert-Butylsulfonyl-6-[4-[4-[4-[3-(dimethylcarbamoyl)-4-hydroxynaphthalen-1-yl]phenyl]thiophene-2-carbonyl]piperazin-1-yl]pyridazine-3-carboxamide |

| | | | |
|---|---|---|---|
| | ED12-18-01R | ED12-18 | N-tert-Butylsulfonyl-6-[4-[4-[6-[3-(dimethylcarbamoyl)-4-hydroxynaphthalen-1-yl]pyridin-3-yl]thiophene-2-carbonyl]piperazin-1-yl]pyridazine-3-carboxamide |
| | ED12-19-01R | ED12-19 | N-tert-Butylsulfonyl-6-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]pyridazine-3-carboxamide |
| | ED12-20-01R | ED12-20 | N-tert-Butylsulfonyl-6-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]pyridazine-3-carboxamide |
| | ED12-21-01R | ED12-21 | N-tert-Butylsulfonyl-6-[4-[2-[2-[4-[3-(dimethylcarbamoyl)-4-hydroxynaphthalen-1-yl]phenyl]ethynyl]benzoyl]piperazin-1-yl]pyridazine-3-carboxamide |
| | ED12-22-01R | ED12-22 | N-tert-Butylsulfonyl-6-[4-[2-[2-[6-[3-(dimethylcarbamoyl)-4-hydroxynaphthalen-1-yl]pyridin-3-yl]ethynyl]benzoyl]piperazin-1-yl]pyridazine-3-carboxamide |
| | ED12-23-01R | ED12-23 | N-tert-Butylsulfonyl-6-[4-[3-[2-[4-[3-(dimethylcarbamoyl)-4-hydroxynaphthalen-1-yl]phenyl]ethynyl]-5-(trifluoromethyl)benzoyl]piperazin-1-yl]pyridazine-3-carboxamide |
| | ED12-24-01R | ED12-24 | N-tert-Butylsulfonyl-6-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]pyridazine-3-carboxamide |
| | ED12-25-01R | ED12-25 | N-tert-Butylsulfonyl-6-[4-[5-[2-[4-[3-(dimethylcarbamoyl)-4-hydroxynaphthalen-1-yl]phenyl]ethynyl]pyridine-3-carbonyl]piperazin-1-yl]pyridazine-3-carboxamide |
| | ED12-26-01R | ED12-26 | N-tert-Butylsulfonyl-6-[4-[5-[2-[6-[3-(dimethylcarbamoyl)-4-hydroxynaphthalen-1-yl]pyridin-3-yl]ethynyl]pyridine-3-carbonyl]piperazin-1-yl]pyridazine-3-carboxamide |
| | ED12-27-01R | ED12-27 | 4-[4-[4-[4-[4-(tert-Butylsulfonylcarbamoyl)phenyl]piperazine-1-carbonyl]-2-methylphenyl]phenyl]-1-hydroxy-N,N-dimethylnaphthalene-2-carboxamide |
| mixED12-01R | ED12-28-01R | ED12-28 | 4-[5-[4-[4-[4-(tert-Butylsulfonylcarbamoyl)phenyl]piperazine-1-carbonyl]-2-methylphenyl]pyridin-2-yl]-1-hydroxy-N,N-dimethylnaphthalene-2-carboxamide |
| | ED12-29-01R | ED12-29 | 4-[4-[4-[4-[4-(tert-Butylsulfonylcarbamoyl)phenyl]piperazine-1-carbonyl]-2-fluorophenyl]phenyl]-1-hydroxy-N,N-dimethylnaphthalene-2-carboxamide |
| | ED12-30-01R | ED12-30 | 4-[5-[4-[4-[4-(tert-Butylsulfonylcarbamoyl)phenyl]piperazine-1-carbonyl]-2-fluorophenyl]pyridin-2-yl]-1-hydroxy-N,N-dimethylnaphthalene-2-carboxamide |
| | ED12-31-01R | ED12-31 | 4-[4-[3-[4-[4-(tert-Butylsulfonylcarbamoyl)phenyl]piperazine-1-carbonyl]-5-(trifluoromethyl)phenyl]phenyl]-1-hydroxy-N,N-dimethylnaphthalene-2-carboxamide |
| | ED12-32-01R | ED12-32 | 4-[5-[3-[4-[4-(tert-Butylsulfonylcarbamoyl)phenyl]piperazine-1-carbonyl]-5-(trifluoromethyl)phenyl]pyridin-2-yl]-1-hydroxy-N,N-dimethylnaphthalene-2-carboxamide |
| | ED12-33-01R | ED12-33 | 4-[4-[3-tert-Butyl-5-[4-[4-(tert-butylsulfonylcarbamoyl)phenyl]piperazine-1-carbonyl]phenyl]phenyl]-1-hydroxy-N,N-dimethylnaphthalene-2-carboxamide |
| | ED12-34-01R | ED12-34 | 4-[5-[3-tert-Butyl-5-[4-[4-(tert-butylsulfonylcarbamoyl)phenyl]piperazine-1-carbonyl]phenyl]pyridin-2-yl]-1-hydroxy-N,N-dimethylnaphthalene-2-carboxamide |
| | ED12-35-01R | ED12-35 | 4-[4-[4-[4-[4-(tert-Butylsulfonylcarbamoyl)phenyl]piperazine-1-carbonyl]naphthalen-1-yl]phenyl]-1-hydroxy-N,N-dimethylnaphthalene-2-carboxamide |
| | ED12-36-01R | ED12-36 | 4-[5-[4-[4-[4-(tert-Butylsulfonylcarbamoyl)phenyl]piperazine-1-carbonyl]naphthalen-1-yl]pyridin-2-yl]-1-hydroxy-N,N-dimethylnaphthalene-2-carboxamide |

| | | | |
|---|---|---|---|
| | ED12-37-01R | ED12-37 | 4-[4-[2-[3-[4-[4-(tert-Butylsulfonylcarbamoyl)phenyl]piperazin-1-yl]-3-oxopropyl]phenyl]phenyl]-1-hydroxy-N,N-dimethylnaphthalene-2-carboxamide |
| | ED12-38-01R | ED12-38 | 4-[5-[2-[3-[4-[4-(tert-Butylsulfonylcarbamoyl)phenyl]piperazin-1-yl]-3-oxopropyl]phenyl]pyridin-2-yl]-1-hydroxy-N,N-dimethylnaphthalene-2-carboxamide |
| | ED12-39-01R | ED12-39 | 4-[4-[5-[4-[4-(tert-Butylsulfonylcarbamoyl)phenyl]piperazine-1-carbonyl]thiophen-3-yl]phenyl]-1-hydroxy-N,N-dimethylnaphthalene-2-carboxamide |
| | ED12-40-01R | ED12-40 | 4-[5-[5-[4-[4-(tert-Butylsulfonylcarbamoyl)phenyl]piperazine-1-carbonyl]thiophen-3-yl]pyridin-2-yl]-1-hydroxy-N,N-dimethylnaphthalene-2-carboxamide |
| | ED12-41-01R | ED12-41 | 4-[4-[1-[3-[4-[4-(tert-Butylsulfonylcarbamoyl)phenyl]piperazine-1-carbonyl]-5-(trifluoromethyl)phenyl]pyrazol-4-yl]phenyl]-1-hydroxy-N,N-dimethylnaphthalene-2-carboxamide |
| | ED12-42-01R | ED12-42 | 4-[5-[1-[3-[4-[4-(tert-Butylsulfonylcarbamoyl)phenyl]piperazine-1-carbonyl]-5-(trifluoromethyl)phenyl]pyrazol-4-yl]pyridin-2-yl]-1-hydroxy-N,N-dimethylnaphthalene-2-carboxamide |
| | ED12-43-01R | ED12-43 | 4-[4-[2-[2-[4-[4-(tert-Butylsulfonylcarbamoyl)phenyl]piperazine-1-carbonyl]phenyl]ethynyl]phenyl]-1-hydroxy-N,N-dimethylnaphthalene-2-carboxamide |
| mixED12-01R | ED12-44-01R | ED12-44 | 4-[5-[2-[2-[4-[4-(tert-Butylsulfonylcarbamoyl)phenyl]piperazine-1-carbonyl]phenyl]ethynyl]pyridin-2-yl]-1-hydroxy-N,N-dimethylnaphthalene-2-carboxamide |
| | ED12-45-01R | ED12-45 | 4-[4-[2-[3-[4-[4-(tert-Butylsulfonylcarbamoyl)phenyl]piperazine-1-carbonyl]-5-(trifluoromethyl)phenyl]ethynyl]phenyl]-1-hydroxy-N,N-dimethylnaphthalene-2-carboxamide |
| | ED12-46-01R | ED12-46 | 4-[5-[2-[3-[4-[4-(tert-Butylsulfonylcarbamoyl)phenyl]piperazine-1-carbonyl]-5-(trifluoromethyl)phenyl]ethynyl]pyridin-2-yl]-1-hydroxy-N,N-dimethylnaphthalene-2-carboxamide |
| | ED12-47-01R | ED12-47 | 4-[4-[2-[5-[4-[4-(tert-Butylsulfonylcarbamoyl)phenyl]piperazine-1-carbonyl]pyridin-3-yl]ethynyl]phenyl]-1-hydroxy-N,N-dimethylnaphthalene-2-carboxamide |
| | ED12-48-01R | ED12-48 | 4-[5-[2-[5-[4-[4-(tert-Butylsulfonylcarbamoyl)phenyl]piperazine-1-carbonyl]pyridin-3-yl]ethynyl]pyridin-2-yl]-1-hydroxy-N,N-dimethylnaphthalene-2-carboxamide |
| | ED12-49-01R | ED12-49 | 4-[4-[2-[[4-[4-(tert-Butylsulfonylcarbamoyl)phenyl]piperazin-1-yl]methyl]phenyl]phenyl]-1-hydroxy-N,N-dimethylnaphthalene-2-carboxamide |
| | ED12-50-01R | ED12-50 | 4-[5-[2-[[4-[4-(tert-Butylsulfonylcarbamoyl)phenyl]piperazin-1-yl]methyl]phenyl]pyridin-2-yl]-1-hydroxy-N,N-dimethylnaphthalene-2-carboxamide |
| | ED12-51-01R | ED12-51 | 4-[4-[4-[[4-[4-(tert-Butylsulfonylcarbamoyl)phenyl]piperazin-1-yl]methyl]-2-methylphenyl]phenyl]-1-hydroxy-N,N-dimethylnaphthalene-2-carboxamide |
| | ED12-52-01R | ED12-52 | 4-[5-[4-[[4-[4-(tert-Butylsulfonylcarbamoyl)phenyl]piperazin-1-yl]methyl]-2-methylphenyl]pyridin-2-yl]-1-hydroxy-N,N-dimethylnaphthalene-2-carboxamide |
| | ED12-53-01R | ED12-53 | 4-[4-[4-[[4-[4-(tert-Butylsulfonylcarbamoyl)phenyl]piperazin-1-yl]methyl]-3-ethylphenyl]phenyl]-1-hydroxy-N,N-dimethylnaphthalene-2-carboxamide |
| | ED12-54-01R | ED12-54 | 4-[5-[4-[[4-[4-(tert-Butylsulfonylcarbamoyl)phenyl]piperazin-1-yl]methyl]-3-ethylphenyl]pyridin-2-yl]-1-hydroxy-N,N-dimethylnaphthalene-2-carboxamide |
| | ED12-55-01R | ED12-55 | 4-[4-[4-[[4-[4-(tert-Butylsulfonylcarbamoyl)phenyl]piperazin-1-yl]methyl]-2-fluorophenyl]phenyl]-1-hydroxy-N,N-dimethylnaphthalene-2-carboxamide |
| | ED12-56-01R | ED12-56 | 4-[5-[4-[[4-[4-(tert-Butylsulfonylcarbamoyl)phenyl]piperazin-1-yl]methyl]-2-fluorophenyl]pyridin-2-yl]-1-hydroxy-N,N-dimethylnaphthalene-2-carboxamide |

| | | | |
|---|---|---|---|
| | ED12-57-01R | ED12-57 | 4-[4-[4-[[4-[4-(tert-Butylsulfonylcarbamoyl)phenyl]piperazin-1-yl]methyl]-3-methoxyphenyl]phenyl]-1-hydroxy-N,N-dimethylnaphthalene-2-carboxamide |
| | ED12-58-01R | ED12-58 | 4-[5-[4-[[4-[4-(tert-Butylsulfonylcarbamoyl)phenyl]piperazin-1-yl]methyl]-3-methoxyphenyl]pyridin-2-yl]-1-hydroxy-N,N-dimethylnaphthalene-2-carboxamide |
| | ED12-59-01R | ED12-59 | 4-[4-[3-[[4-[4-(tert-Butylsulfonylcarbamoyl)phenyl]piperazin-1-yl]methyl]-5-ethoxyphenyl]phenyl]-1-hydroxy-N,N-dimethylnaphthalene-2-carboxamide |
| mixED12-01R | ED12-60-01R | ED12-60 | 4-[5-[3-[[4-[4-(tert-Butylsulfonylcarbamoyl)phenyl]piperazin-1-yl]methyl]-5-ethoxyphenyl]pyridin-2-yl]-1-hydroxy-N,N-dimethylnaphthalene-2-carboxamide |
| | ED12-61-01R | ED12-61 | 4-[4-[3-[[4-[4-(tert-Butylsulfonylcarbamoyl)phenyl]piperazin-1-yl]methyl]-5-propan-2-yloxyphenyl]phenyl]-1-hydroxy-N,N-dimethylnaphthalene-2-carboxamide |
| | ED12-62-01R | ED12-62 | 4-[5-[3-[[4-[4-(tert-Butylsulfonylcarbamoyl)phenyl]piperazin-1-yl]methyl]-5-propan-2-yloxyphenyl]pyridin-2-yl]-1-hydroxy-N,N-dimethylnaphthalene-2-carboxamide |
| | ED12-63-01R | ED12-63 | 4-[4-[4-[[4-[4-(tert-Butylsulfonylcarbamoyl)phenyl]piperazin-1-yl]methyl]-3,5-dimethoxyphenyl]phenyl]-1-hydroxy-N,N-dimethylnaphthalene-2-carboxamide |
| | ED12-64-01R | ED12-64 | 4-[5-[4-[[4-[4-(tert-Butylsulfonylcarbamoyl)phenyl]piperazin-1-yl]methyl]-3,5-dimethoxyphenyl]pyridin-2-yl]-1-hydroxy-N,N-dimethylnaphthalene-2-carboxamide |
| | ED12-65-01R | ED12-65 | 4-[4-[3-[[4-[4-(tert-Butylsulfonylcarbamoyl)phenyl]piperazin-1-yl]methyl]-5-(trifluoromethyl)phenyl]phenyl]-1-hydroxy-N,N-dimethylnaphthalene-2-carboxamide |
| | ED12-66-01R | ED12-66 | 4-[5-[3-[[4-[4-(tert-Butylsulfonylcarbamoyl)phenyl]piperazin-1-yl]methyl]-5-(trifluoromethyl)phenyl]pyridin-2-yl]-1-hydroxy-N,N-dimethylnaphthalene-2-carboxamide |
| | ED12-67-01R | ED12-67 | 4-[4-[3-[[4-[4-(tert-Butylsulfonylcarbamoyl)phenyl]piperazin-1-yl]methyl]-5-(trifluoromethoxy)phenyl]phenyl]-1-hydroxy-N,N-dimethylnaphthalene-2-carboxamide |
| | ED12-68-01R | ED12-68 | 4-[5-[3-[[4-[4-(tert-Butylsulfonylcarbamoyl)phenyl]piperazin-1-yl]methyl]-5-(trifluoromethoxy)phenyl]pyridin-2-yl]-1-hydroxy-N,N-dimethylnaphthalene-2-carboxamide |
| | ED12-69-01R | ED12-69 | 4-[4-[4-[[4-[4-(tert-Butylsulfonylcarbamoyl)phenyl]piperazin-1-yl]methyl]naphthalen-1-yl]phenyl]-1-hydroxy-N,N-dimethylnaphthalene-2-carboxamide |
| | ED12-70-01R | ED12-70 | 4-[5-[4-[[4-[4-(tert-Butylsulfonylcarbamoyl)phenyl]piperazin-1-yl]methyl]naphthalen-1-yl]pyridin-2-yl]-1-hydroxy-N,N-dimethylnaphthalene-2-carboxamide |
| | ED12-71-01R | ED12-71 | 4-[4-[5-[[4-[4-(tert-Butylsulfonylcarbamoyl)phenyl]piperazin-1-yl]methyl]thiophen-3-yl]phenyl]-1-hydroxy-N,N-dimethylnaphthalene-2-carboxamide |
| | ED12-72-01R | ED12-72 | 4-[5-[5-[[4-[4-(tert-Butylsulfonylcarbamoyl)phenyl]piperazin-1-yl]methyl]thiophen-3-yl]pyridin-2-yl]-1-hydroxy-N,N-dimethylnaphthalene-2-carboxamide |
| | ED12-73-01R | ED12-73 | 4-[4-[2-[2-[[4-[4-(tert-Butylsulfonylcarbamoyl)phenyl]piperazin-1-yl]methyl]phenyl]ethynyl]phenyl]-1-hydroxy-N,N-dimethylnaphthalene-2-carboxamide |
| | ED12-74-01R | ED12-74 | 4-[5-[2-[2-[[4-[4-(tert-Butylsulfonylcarbamoyl)phenyl]piperazin-1-yl]methyl]phenyl]ethynyl]pyridin-2-yl]-1-hydroxy-N,N-dimethylnaphthalene-2-carboxamide |
| | ED12-75-01R | ED12-75 | N-tert-Butylsulfonyl-6-[4-[[2-[4-[3-(dimethylcarbamoyl)-4-hydroxynaphthalen-1-yl]phenyl]phenyl]methyl]piperazin-1-yl]pyridazine-3-carboxamide |

| | | | |
|---|---|---|---|
| mixED12-01R | ED12-76-01R | ED12-76 | N-tert-Butylsulfonyl-6-[4-[[2-[6-[3-(dimethylcarbamoyl)-4-hydroxynaphthalen-1-yl]pyridin-3-yl]phenyl]methyl]piperazin-1-yl]pyridazine-3-carboxamide |
| | ED12-77-01R | ED12-77 | N-tert-Butylsulfonyl-6-[4-[[4-[4-[3-(dimethylcarbamoyl)-4-hydroxynaphthalen-1-yl]phenyl]-3-methylphenyl]methyl]piperazin-1-yl]pyridazine-3-carboxamide |
| | ED12-78-01R | ED12-78 | N-tert-Butylsulfonyl-6-[4-[[4-[6-[3-(dimethylcarbamoyl)-4-hydroxynaphthalen-1-yl]pyridin-3-yl]-3-methylphenyl]methyl]piperazin-1-yl]pyridazine-3-carboxamide |
| | ED12-79-01R | ED12-79 | N-tert-Butylsulfonyl-6-[4-[[4-[4-[3-(dimethylcarbamoyl)-4-hydroxynaphthalen-1-yl]phenyl]-2-ethylphenyl]methyl]piperazin-1-yl]pyridazine-3-carboxamide |
| | ED12-80-01R | ED12-80 | N-tert-Butylsulfonyl-6-[4-[[4-[6-[3-(dimethylcarbamoyl)-4-hydroxynaphthalen-1-yl]pyridin-3-yl]-2-ethylphenyl]methyl]piperazin-1-yl]pyridazine-3-carboxamide |
| | ED12-81-01R | ED12-81 | N-tert-Butylsulfonyl-6-[4-[[4-[4-[3-(dimethylcarbamoyl)-4-hydroxynaphthalen-1-yl]phenyl]-3-fluorophenyl]methyl]piperazin-1-yl]pyridazine-3-carboxamide |
| | ED12-82-01R | ED12-82 | N-tert-Butylsulfonyl-6-[4-[[4-[6-[3-(dimethylcarbamoyl)-4-hydroxynaphthalen-1-yl]pyridin-3-yl]-3-fluorophenyl]methyl]piperazin-1-yl]pyridazine-3-carboxamide |
| | ED12-83-01R | ED12-83 | N-tert-Butylsulfonyl-6-[4-[[4-[4-[3-(dimethylcarbamoyl)-4-hydroxynaphthalen-1-yl]phenyl]-2-methoxyphenyl]methyl]piperazin-1-yl]pyridazine-3-carboxamide |
| | ED12-84-01R | ED12-84 | N-tert-Butylsulfonyl-6-[4-[[4-[6-[3-(dimethylcarbamoyl)-4-hydroxynaphthalen-1-yl]pyridin-3-yl]-2-methoxyphenyl]methyl]piperazin-1-yl]pyridazine-3-carboxamide |
| | ED12-85-01R | ED12-85 | N-tert-Butylsulfonyl-6-[4-[[3-[4-[3-(dimethylcarbamoyl)-4-hydroxynaphthalen-1-yl]phenyl]-5-ethoxyphenyl]methyl]piperazin-1-yl]pyridazine-3-carboxamide |
| | ED12-86-01R | ED12-86 | N-tert-Butylsulfonyl-6-[4-[[3-[6-[3-(dimethylcarbamoyl)-4-hydroxynaphthalen-1-yl]pyridin-3-yl]-5-ethoxyphenyl]methyl]piperazin-1-yl]pyridazine-3-carboxamide |
| | ED12-87-01R | ED12-87 | N-tert-Butylsulfonyl-6-[4-[[3-[4-[3-(dimethylcarbamoyl)-4-hydroxynaphthalen-1-yl]phenyl]-5-propan-2-yloxyphenyl]methyl]piperazin-1-yl]pyridazine-3-carboxamide |
| | ED12-88-01R | ED12-88 | N-tert-Butylsulfonyl-6-[4-[[3-[6-[3-(dimethylcarbamoyl)-4-hydroxynaphthalen-1-yl]pyridin-3-yl]-5-propan-2-yloxyphenyl]methyl]piperazin-1-yl]pyridazine-3-carboxamide |
| | ED12-89-01R | ED12-89 | N-tert-Butylsulfonyl-6-[4-[[4-[4-[3-(dimethylcarbamoyl)-4-hydroxynaphthalen-1-yl]phenyl]-2,6-dimethoxyphenyl]methyl]piperazin-1-yl]pyridazine-3-carboxamide |
| | ED12-90-01R | ED12-90 | N-tert-Butylsulfonyl-6-[4-[[4-[6-[3-(dimethylcarbamoyl)-4-hydroxynaphthalen-1-yl]pyridin-3-yl]-2,6-dimethoxyphenyl]methyl]piperazin-1-yl]pyridazine-3-carboxamide |
| mixED12-01R | ED12-91-01R | ED12-91 | N-tert-Butylsulfonyl-6-[4-[[3-[4-[3-(dimethylcarbamoyl)-4-hydroxynaphthalen-1-yl]phenyl]-5-(trifluoromethyl)phenyl]methyl]piperazin-1-yl]pyridazine-3-carboxamide |
| | ED12-92-01R | ED12-92 | N-tert-Butylsulfonyl-6-[4-[[3-[6-[3-(dimethylcarbamoyl)-4-hydroxynaphthalen-1-yl]pyridin-3-yl]-5-(trifluoromethyl)phenyl]methyl]piperazin-1-yl]pyridazine-3-carboxamide |
| | ED12-93-01R | ED12-93 | N-tert-Butylsulfonyl-6-[4-[[3-[4-[3-(dimethylcarbamoyl)-4-hydroxynaphthalen-1-yl]phenyl]-5-(trifluoromethoxy)phenyl]methyl]piperazin-1-yl]pyridazine-3-carboxamide |
| | ED12-94-01R | ED12-94 | N-tert-Butylsulfonyl-6-[4-[[3-[6-[3-(dimethylcarbamoyl)-4-hydroxynaphthalen-1-yl]pyridin-3-yl]-5-(trifluoromethoxy)phenyl]methyl]piperazin-1-yl]pyridazine-3-carboxamide |

| | | | |
|---|---|---|---|
| | ED12-95-01R | ED12-95 | N-tert-Butylsulfonyl-6-[4-[[4-[4-[3-(dimethylcarbamoyl)-4-hydroxynaphthalen-1-yl]phenyl]naphthalen-1-yl]methyl]piperazin-1-yl]pyridazine-3-carboxamide |
| | ED12-96-01R | ED12-96 | N-tert-Butylsulfonyl-6-[4-[[4-[6-[3-(dimethylcarbamoyl)-4-hydroxynaphthalen-1-yl]pyridin-3-yl]naphthalen-1-yl]methyl]piperazin-1-yl]pyridazine-3-carboxamide |
| | ED12-97-01R | ED12-97 | N-tert-Butylsulfonyl-6-[4-[[4-[4-[3-(dimethylcarbamoyl)-4-hydroxynaphthalen-1-yl]phenyl]thiophen-2-yl]methyl]piperazin-1-yl]pyridazine-3-carboxamide |
| | ED12-98-01R | ED12-98 | N-tert-Butylsulfonyl-6-[4-[[4-[6-[3-(dimethylcarbamoyl)-4-hydroxynaphthalen-1-yl]pyridin-3-yl]thiophen-2-yl]methyl]piperazin-1-yl]pyridazine-3-carboxamide |
| | ED12-99-01R | ED12-99 | N-tert-Butylsulfonyl-6-[4-[[2-[2-[4-[3-(dimethylcarbamoyl)-4-hydroxynaphthalen-1-yl]phenyl]ethynyl]phenyl]methyl]piperazin-1-yl]pyridazine-3-carboxamide |
| | ED12-100-01R | ED12-100 | N-tert-Butylsulfonyl-6-[4-[[2-[2-[6-[3-(dimethylcarbamoyl)-4-hydroxynaphthalen-1-yl]pyridin-3-yl]ethynyl]phenyl]methyl]piperazin-1-yl]pyridazine-3-carboxamide |

[Table 112]

[Table ED13]

| No. of mixture | No. of compound that may be contained | Compound No. after cleavage | Compound name after cleavage |
|---|---|---|---|
| mixED13-01R | ED13-01-01R | ED13-01 | 1-Hydroxy-N,N-dimethyl-4-[4-[4-[1-[4-[4-(3,3,3-trifluoropropylsulfonylcarbamoyl)phenyl]piperazin-1-yl]ethyl]phenyl]phenyl]naphthalene-2-carboxamide |
| | ED13-02-01R | ED13-02 | 1-Hydroxy-N,N-dimethyl-4-[5-[4-[1-[4-[4-(3,3,3-trifluoropropylsulfonylcarbamoyl)phenyl]piperazin-1-yl]ethyl]phenyl]pyridin-2-yl]naphthalene-2-carboxamide |
| mixED13-01R | ED13-03-01R | ED13-03 | 4-[4-[3-Fluoro-4-[1-[4-[4-(3,3,3-trifluoropropylsulfonylcarbamoyl)phenyl]piperazin-1-yl]ethyl]phenyl]phenyl]-1-hydroxy-N,N-dimethylnaphthalene-2-carboxamide |
| | ED13-04-01R | ED13-04 | 4-[5-[3-Fluoro-4-[1-[4-[4-(3,3,3-trifluoropropylsulfonylcarbamoyl)phenyl]piperazin-1-yl]ethyl]phenyl]pyridin-2-yl]-1-hydroxy-N,N-dimethylnaphthalene-2-carboxamide |
| | ED13-05-01R | ED13-05 | 6-[4-[4-[4-[3-(Dimethylcarbamoyl)-4-hydroxynaphthalen-1-yl]phenyl]-3-methylbenzoyl]piperazin-1-yl]-N-(3,3,3-trifluoropropylsulfonyl)pyridazine-3-carboxamide |
| | ED13-06-01R | ED13-06 | 6-[4-[4-[6-[3-(Dimethylcarbamoyl)-4-hydroxynaphthalen-1-yl]pyridin-3-yl]-3-methylbenzoyl]piperazin-1-yl]-N-(3,3,3-trifluoropropylsulfonyl)pyridazine-3-carboxamide |
| | ED13-07-01R | ED13-07 | 6-[4-[4-[4-[3-(Dimethylcarbamoyl)-4-hydroxynaphthalen-1-yl]phenyl]-3-fluorobenzoyl]piperazin-1-yl]-N-(3,3,3-trifluoropropylsulfonyl)pyridazine-3-carboxamide |
| | ED13-08-01R | ED13-08 | 6-[4-[4-[6-[3-(Dimethylcarbamoyl)-4-hydroxynaphthalen-1-yl]pyridin-3-yl]-3-fluorobenzoyl]piperazin-1-yl]-N-(3,3,3-trifluoropropylsulfonyl)pyridazine-3-carboxamide |
| | ED13-09-01R | ED13-09 | 6-[4-[3-[4-[3-(Dimethylcarbamoyl)-4-hydroxynaphthalen-1-yl]phenyl]-5-(trifluoromethyl)benzoyl]piperazin-1-yl]-N-(3,3,3-trifluoropropylsulfonyl)pyridazine-3-carboxamide |

| | | | |
|---|---|---|---|
| | ED13-10-01R | ED13-10 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-(3,3,3-trifluoropropylsulfonyl)pyridazine-3-carboxamide |
| | ED13-11-01R | ED13-11 | 6-[4-[3-tert-Butyl-5-[4-[3-(dimethylcarbamoyl)-4-hydroxynaphthalen-1-yl]phenyl]benzoyl]piperazin-1-yl]-N-(3,3,3-trifluoropropylsulfonyl)pyridazine-3-carboxamide |
| | ED13-12-01R | ED13-12 | 6-[4-[3-tert-Butyl-5-[6-[3-(dimethylcarbamoyl)-4-hydroxynaphthalen-1-yl]pyridin-3-yl]benzoyl]piperazin-1-yl]-N-(3,3,3-trifluoropropylsulfonyl)pyridazine-3-carboxamide |
| | ED13-13-01R | ED13-13 | 6-[4-[4-[4-[3-(Dimethylcarbamoyl)-4-hydroxynaphthalen-1-yl]phenyl]naphthalene-1-carbonyl]piperazin-1-yl]-N-(3,3,3-trifluoropropylsulfonyl)pyridazine-3-carboxamide |
| | ED13-14-01R | ED13-14 | 6-[4-[4-[6-[3-(Dimethylcarbamoyl)-4-hydroxynaphthalen-1-yl]pyridin-3-yl]naphthalene-1-carbonyl]piperazin-1-yl]-N-(3,3,3-trifluoropropylsulfonyl)pyridazine-3-carboxamide |
| | ED13-15-01R | ED13-15 | 6-[4-[3-[2-[4-[3-(Dimethylcarbamoyl)-4-hydroxynaphthalen-1-yl]phenyl]phenyl]propanoyl]piperazin-1-yl]-N-(3,3,3-trifluoropropylsulfonyl)pyridazine-3-carboxamide |
| | ED13-16-01R | ED13-16 | 6-[4-[3-[2-[6-[3-(Dimethylcarbamoyl)-4-hydroxynaphthalen-1-yl]pyridin-3-yl]phenyl]propanoyl]piperazin-1-yl]-N-(3,3,3-trifluoropropylsulfonyl)pyridazine-3-carboxamide |
| | ED13-17-01R | ED13-17 | 6-[4-[4-[4-[3-(Dimethylcarbamoyl)-4-hydroxynaphthalen-1-yl]phenyl]thiophene-2-carbonyl]piperazin-1-yl]-N-(3,3,3-trifluoropropylsulfonyl)pyridazine-3-carboxamide |
| | ED13-18-01R | ED13-18 | 6-[4-[4-[6-[3-(Dimethylcarbamoyl)-4-hydroxynaphthalen-1-yl]pyridin-3-yl]thiophene-2-carbonyl]piperazin-1-yl]-N-(3,3,3-trifluoropropylsulfonyl)pyridazine-3-carboxamide |
| mixED13-01R | ED13-19-01R | ED13-19 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-(3,3,3-trifluoropropylsulfonyl)pyridazine-3-carboxamide |
| | ED13-20-01R | ED13-20 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-(3,3,3-trifluoropropylsulfonyl)pyridazine-3-carboxamide |
| | ED13-21-01R | ED13-21 | 6-[4-[2-[2-[4-[3-(Dimethylcarbamoyl)-4-hydroxynaphthalen-1-yl]phenyl]ethynyl]benzoyl]piperazin-1-yl]-N-(3,3,3-trifluoropropylsulfonyl)pyridazine-3-carboxamide |
| | ED13-22-01R | ED13-22 | 6-[4-[2-[2-[6-[3-(Dimethylcarbamoyl)-4-hydroxynaphthalen-1-yl]pyridin-3-yl]ethynyl]benzoyl]piperazin-1-yl]-N-(3,3,3-trifluoropropylsulfonyl)pyridazine-3-carboxamide |
| | ED13-23-01R | ED13-23 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-(3,3,3-trifluoropropylsulfonyl)pyridazine-3-carboxamide |
| | ED13-24-01R | ED13-24 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-(3,3,3-trifluoropropylsulfonyl)pyridazine-3-carboxamide |
| | ED13-25-01R | ED13-25 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-(3,3,3-trifluoropropylsulfonyl)pyridazine-3-carboxamide |
| | ED13-26-01R | ED13-26 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-(3,3,3-trifluoropropylsulfonyl)pyridazine-3-carboxamide |
| | ED13-27-01R | ED13-27 | 1-Hydroxy-N,N-dimethyl-4-[4-[2-methyl-4-[4-[4-(3,3,3-trifluoropropylsulfonylcarbamoyl)phenyl]piperazine-1-carbonyl]phenyl]phenyl]naphthalene-2-carboxamide |
| | ED13-28-01R | ED13-28 | 1-Hydroxy-N,N-dimethyl-4-[5-[2-methyl-4-[4-[4-(3,3,3-trifluoropropylsulfonylcarbamoyl)phenyl]piperazine-1-carbonyl]phenyl]pyridin-2-yl]naphthalene-2-carboxamide |

| | | | |
|---|---|---|---|
| | ED13-29-01R | ED13-29 | 4-[4-[2-Fluoro-4-[4-[4-(3,3,3-trifluoropropylsulfonylcarbamoyl)phenyl]piperazine-1-carbonyl]phenyl]phenyl]-1-hydroxy-N,N-dimethylnaphthalene-2-carboxamide |
| | ED13-30-01R | ED13-30 | 4-[5-[2-Fluoro-4-[4-[4-(3,3,3-trifluoropropylsulfonylcarbamoyl)phenyl]piperazine-1-carbonyl]phenyl]pyridin-2-yl]-1-hydroxy-N,N-dimethylnaphthalene-2-carboxamide |
| | ED13-31-01R | ED13-31 | 1-Hydroxy-N,N-dimethyl-4-[4-[3-(trifluoromethyl)-5-[4-[4-(3,3,3-trifluoropropylsulfonylcarbamoyl)phenyl]piperazine-1-carbonyl]phenyl]phenyl]naphthalene-2-carboxamide |
| | ED13-32-01R | ED13-32 | 1-Hydroxy-N,N-dimethyl-4-[5-[3-(trifluoromethyl)-5-[4-[4-(3,3,3-trifluoropropylsulfonylcarbamoyl)phenyl]piperazine-1-carbonyl]phenyl]pyridin-2-yl]naphthalene-2-carboxamide |
| | ED13-33-01R | ED13-33 | 4-[4-[3-tert-Butyl-5-[4-[4-(3,3,3-trifluoropropylsulfonylcarbamoyl)phenyl]piperazine-1-carbonyl]phenyl]phenyl]-1-hydroxy-N,N-dimethylnaphthalene-2-carboxamide |
| mixED13-01R | ED13-34-01R | ED13-34 | 4-[5-[3-tert-Butyl-5-[4-[4-(3,3,3-trifluoropropylsulfonylcarbamoyl)phenyl]piperazine-1-carbonyl]phenyl]pyridin-2-yl]-1-hydroxy-N,N-dimethylnaphthalene-2-carboxamide |
| | ED13-35-01R | ED13-35 | 1-Hydroxy-N,N-dimethyl-4-[4-[4-[4-[4-(3,3,3-trifluoropropylsulfonylcarbamoyl)phenyl]piperazine-1-carbonyl]naphthalen-1-yl]phenyl]naphthalene-2-carboxamide |
| | ED13-36-01R | ED13-36 | 1-Hydroxy-N,N-dimethyl-4-[5-[4-[4-[4-(3,3,3-trifluoropropylsulfonylcarbamoyl)phenyl]piperazine-1-carbonyl]naphthalen-1-yl]pyridin-2-yl]naphthalene-2-carboxamide |
| | ED13-37-01R | ED13-37 | 1-Hydroxy-N,N-dimethyl-4-[4-[2-[3-oxo-3-[4-[4-(3,3,3-trifluoropropylsulfonylcarbamoyl)phenyl]piperazin-1-yl]propyl]phenyl]phenyl]naphthalene-2-carboxamide |
| | ED13-38-01R | ED13-38 | 1-Hydroxy-N,N-dimethyl-4-[5-[2-[3-oxo-3-[4-[4-(3,3,3-trifluoropropylsulfonylcarbamoyl)phenyl]piperazin-1-yl]propyl]phenyl]pyridin-2-yl]naphthalene-2-carboxamide |
| | ED13-39-01R | ED13-39 | 1-Hydroxy-N,N-dimethyl-4-[4-[5-[4-[4-(3,3,3-trifluoropropylsulfonylcarbamoyl)phenyl]piperazine-1-carbonyl]thiophen-3-yl]phenyl]naphthalene-2-carboxamide |
| | ED13-40-01R | ED13-40 | 1-Hydroxy-N,N-dimethyl-4-[5-[5-[4-[4-(3,3,3-trifluoropropylsulfonylcarbamoyl)phenyl]piperazine-1-carbonyl]thiophen-3-yl]pyridin-2-yl]naphthalene-2-carboxamide |
| | ED13-41-01R | ED13-41 | 1-Hydroxy-N,N-dimethyl-4-[4-[1-[3-(trifluoromethyl)-5-[4-[4-(3,3,3-trifluoropropylsulfonylcarbamoyl)phenyl]piperazine-1-carbonyl]phenyl]pyrazol-4-yl]phenyl]naphthalene-2-carboxamide |
| | ED13-42-01R | ED13-42 | 1-Hydroxy-N,N-dimethyl-4-[5-[1-[3-(trifluoromethyl)-5-[4-[4-(3,3,3-trifluoropropylsulfonylcarbamoyl)phenyl]piperazine-1-carbonyl]phenyl]pyrazol-4-yl]pyridin-2-yl]naphthalene-2-carboxamide |
| | ED13-43-01R | ED13-43 | 1-Hydroxy-N,N-dimethyl-4-[4-[2-[2-[4-[4-(3,3,3-trifluoropropylsulfonylcarbamoyl)phenyl]piperazine-1-carbonyl]phenyl]ethynyl]phenyl]naphthalene-2-carboxamide |
| | ED13-44-01R | ED13-44 | 1-Hydroxy-N,N-dimethyl-4-[5-[2-[2-[4-[4-(3,3,3-trifluoropropylsulfonylcarbamoyl)phenyl]piperazine-1-carbonyl]phenyl]ethynyl]pyridin-2-yl]naphthalene-2-carboxamide |
| | ED13-45-01R | ED13-45 | 1-Hydroxy-N,N-dimethyl-4-[4-[2-[3-(trifluoromethyl)-5-[4-[4-(3,3,3-trifluoropropylsulfonylcarbamoyl)phenyl]piperazine-1-carbonyl]phenyl]ethynyl]phenyl]naphthalene-2-carboxamide |

| | | | |
|---|---|---|---|
| | ED13-46-01R | ED13-46 | 1-Hydroxy-N,N-dimethyl-4-[5-[2-[3-(trifluoromethyl)-5-[4-[4-(3,3,3-trifluoropropylsulfonylcarbamoyl)phenyl]piperazine-1-carbonyl]phenyl]ethynyl]pyridin-2-yl]naphthalene-2-carboxamide |
| | ED13-47-01R | ED13-47 | 1-Hydroxy-N,N-dimethyl-4-[4-[2-[5-[4-[4-(3,3,3-trifluoropropylsulfonylcarbamoyl)phenyl]piperazine-1-carbonyl]pyridin-3-yl]ethynyl]phenyl]naphthalene-2-carboxamide |
| | ED13-48-01R | ED13-48 | 1-Hydroxy-N,N-dimethyl-4-[5-[2-[5-[4-[4-(3,3,3-trifluoropropylsulfonylcarbamoyl)phenyl]piperazine-1-carbonyl]pyridin-3-yl]ethynyl]pyridin-2-yl]naphthalene-2-carboxamide |
| mixED13-01R | ED13-49-01R | ED13-49 | 1-Hydroxy-N,N-dimethyl-4-[4-[2-[[4-[4-(3,3,3-trifluoropropylsulfonylcarbamoyl)phenyl]piperazin-1-yl]methyl]phenyl]phenyl]naphthalene-2-carboxamide |
| | ED13-50-01R | ED13-50 | 1-Hydroxy-N,N-dimethyl-4-[5-[2-[[4-[4-(3,3,3-trifluoropropylsulfonylcarbamoyl)phenyl]piperazin-1-yl]methyl]phenyl]pyridin-2-yl]naphthalene-2-carboxamide |
| | ED13-51-01R | ED13-51 | 1-Hydroxy-N,N-dimethyl-4-[4-[2-methyl-4-[[4-[4-(3,3,3-trifluoropropylsulfonylcarbamoyl)phenyl]piperazin-1-yl]methyl]phenyl]phenyl]naphthalene-2-carboxamide |
| | ED13-52-01R | ED13-52 | 1-Hydroxy-N,N-dimethyl-4-[5-[2-methyl-4-[[4-[4-(3,3,3-trifluoropropylsulfonylcarbamoyl)phenyl]piperazin-1-yl]methyl]phenyl]pyridin-2-yl]naphthalene-2-carboxamide |
| | ED13-53-01R | ED13-53 | 4-[4-[3-Ethyl-4-[[4-[4-(3,3,3-trifluoropropylsulfonylcarbamoyl)phenyl]piperazin-1-yl]methyl]phenyl]phenyl]-1-hydroxy-N,N-dimethylnaphthalene-2-carboxamide |
| | ED13-54-01R | ED13-54 | 4-[5-[3-Ethyl-4-[[4-[4-(3,3,3-trifluoropropylsulfonylcarbamoyl)phenyl]piperazin-1-yl]methyl]phenyl]pyridin-2-yl]-1-hydroxy-N,N-dimethylnaphthalene-2-carboxamide |
| | ED13-55-01R | ED13-55 | 4-[4-[2-Fluoro-4-[[4-[4-(3,3,3-trifluoropropylsulfonylcarbamoyl)phenyl]piperazin-1-yl]methyl]phenyl]phenyl]-1-hydroxy-N,N-dimethylnaphthalene-2-carboxamide |
| | ED13-56-01R | ED13-56 | 4-[5-[2-Fluoro-4-[[4-[4-(3,3,3-trifluoropropylsulfonylcarbamoyl)phenyl]piperazin-1-yl]methyl]phenyl]pyridin-2-yl]-1-hydroxy-N,N-dimethylnaphthalene-2-carboxamide |
| | ED13-57-01R | ED13-57 | 1-Hydroxy-4-[4-[3-methoxy-4-[[4-[4-(3,3,3-trifluoropropylsulfonylcarbamoyl)phenyl]piperazin-1-yl]methyl]phenyl]phenyl]-N,N-dimethylnaphthalene-2-carboxamide |
| | ED13-58-01R | ED13-58 | 1-Hydroxy-4-[5-[3-methoxy-4-[[4-[4-(3,3,3-trifluoropropylsulfonylcarbamoyl)phenyl]piperazin-1-yl]methyl]phenyl]pyridin-2-yl]-N,N-dimethylnaphthalene-2-carboxamide |
| | ED13-59-01R | ED13-59 | 4-[4-[3-Ethoxy-5-[[4-[4-(3,3,3-trifluoropropylsulfonylcarbamoyl)phenyl]piperazin-1-yl]methyl]phenyl]phenyl]-1-hydroxy-N,N-dimethylnaphthalene-2-carboxamide |
| | ED13-60-01R | ED13-60 | 4-[5-[3-Ethoxy-5-[[4-[4-(3,3,3-trifluoropropylsulfonylcarbamoyl)phenyl]piperazin-1-yl]methyl]phenyl]pyridin-2-yl]-1-hydroxy-N,N-dimethylnaphthalene-2-carboxamide |
| | ED13-61-01R | ED13-61 | 1-Hydroxy-N,N-dimethyl-4-[4-[3-propan-2-yloxy-5-[[4-[4-(3,3,3-trifluoropropylsulfonylcarbamoyl)phenyl]piperazin-1-yl]methyl]phenyl]phenyl]naphthalene-2-carboxamide |
| | ED13-62-01R | ED13-62 | 1-Hydroxy-N,N-dimethyl-4-[5-[3-propan-2-yloxy-5-[[4-[4-(3,3,3-trifluoropropylsulfonylcarbamoyl)phenyl]piperazin-1-yl]methyl]phenyl]pyridin-2-yl]naphthalene-2-carboxamide |

| | | | |
|---|---|---|---|
| | ED13-63-01R | ED13-63 | 4-[4-[3,5-Dimethoxy-4-[[4-[4-(3,3,3-trifluoropropylsulfonylcarbamoyl)phenyl]piperazin-1-yl]methyl]phenyl]phenyl]-1-hydroxy-N,N-dimethylnaphthalene-2-carboxamide |
| mixED13-01R | ED13-64-01R | ED13-64 | 4-[5-[3,5-Dimethoxy-4-[[4-[4-(3,3,3-trifluoropropylsulfonylcarbamoyl)phenyl]piperazin-1-yl]methyl]phenyl]pyridin-2-yl]-1-hydroxy-N,N-dimethylnaphthalene-2-carboxamide |
| | ED13-65-01R | ED13-65 | 1-Hydroxy-N,N-dimethyl-4-[4-[3-(trifluoromethyl)-5-[[4-[4-(3,3,3-trifluoropropylsulfonylcarbamoyl)phenyl]piperazin-1-yl]methyl]phenyl]phenyl]naphthalene-2-carboxamide |
| | ED13-66-01R | ED13-66 | 1-Hydroxy-N,N-dimethyl-4-[5-[3-(trifluoromethyl)-5-[[4-[4-(3,3,3-trifluoropropylsulfonylcarbamoyl)phenyl]piperazin-1-yl]methyl]phenyl]pyridin-2-yl]naphthalene-2-carboxamide |
| | ED13-67-01R | ED13-67 | 1-Hydroxy-N,N-dimethyl-4-[4-[3-(trifluoromethoxy)-5-[[4-[4-(3,3,3-trifluoropropylsulfonylcarbamoyl)phenyl]piperazin-1-yl]methyl]phenyl]phenyl]naphthalene-2-carboxamide |
| | ED13-68-01R | ED13-68 | 1-Hydroxy-N,N-dimethyl-4-[5-[3-(trifluoromethoxy)-5-[[4-[4-(3,3,3-trifluoropropylsulfonylcarbamoyl)phenyl]piperazin-1-yl]methyl]phenyl]pyridin-2-yl]naphthalene-2-carboxamide |
| | ED13-69-01R | ED13-69 | 1-Hydroxy-N,N-dimethyl-4-[4-[4-[[4-[4-(3,3,3-trifluoropropylsulfonylcarbamoyl)phenyl]piperazin-1-yl]methyl]naphthalen-1-yl]phenyl]naphthalene-2-carboxamide |
| | ED13-70-01R | ED13-70 | 1-Hydroxy-N,N-dimethyl-4-[5-[4-[[4-[4-(3,3,3-trifluoropropylsulfonylcarbamoyl)phenyl]piperazin-1-yl]methyl]naphthalen-1-yl]pyridin-2-yl]naphthalene-2-carboxamide |
| | ED13-71-01R | ED13-71 | 1-Hydroxy-N,N-dimethyl-4-[4-[5-[[4-[4-(3,3,3-trifluoropropylsulfonylcarbamoyl)phenyl]piperazin-1-yl]methyl]thiophen-3-yl]phenyl]naphthalene-2-carboxamide |
| | ED13-72-01R | ED13-72 | 1-Hydroxy-N,N-dimethyl-4-[5-[5-[[4-[4-(3,3,3-trifluoropropylsulfonylcarbamoyl)phenyl]piperazin-1-yl]methyl]thiophen-3-yl]pyridin-2-yl]naphthalene-2-carboxamide |
| | ED13-73-01R | ED13-73 | 1-Hydroxy-N,N-dimethyl-4-[4-[2-[2-[[4-[4-(3,3,3-trifluoropropylsulfonylcarbamoyl)phenyl]piperazin-1-yl]methyl]phenyl]ethynyl]phenyl]naphthalene-2-carboxamide |
| | ED13-74-01R | ED13-74 | 1-Hydroxy-N,N-dimethyl-4-[5-[2-[2-[[4-[4-(3,3,3-trifluoropropylsulfonylcarbamoyl)phenyl]piperazin-1-yl]methyl]phenyl]ethynyl]pyridin-2-yl]naphthalene-2-carboxamide |
| | ED13-75-01R | ED13-75 | 6-[4-[[2-[4-[3-(Dimethylcarbamoyl)-4-hydroxynaphthalen-1-yl]phenyl]phenyl]methyl]piperazin-1-yl]-N-(3,3,3-trifluoropropylsulfonyl)pyridazine-3-carboxamide |
| | ED13-76-01R | ED13-76 | 6-[4-[[2-[6-[3-(Dimethylcarbamoyl)-4-hydroxynaphthalen-1-yl]pyridin-3-yl]phenyl]methyl]piperazin-1-yl]-N-(3,3,3-trifluoropropylsulfonyl)pyridazine-3-carboxamide |
| | ED13-77-01R | ED13-77 | 6-[4-[[4-[4-[3-(Dimethylcarbamoyl)-4-hydroxynaphthalen-1-yl]phenyl]-3-methylphenyl]methyl]piperazin-1-yl]-N-(3,3,3-trifluoropropylsulfonyl)pyridazine-3-carboxamide |
| | ED13-78-01R | ED13-78 | 6-[4-[[4-[6-[3-(Dimethylcarbamoyl)-4-hydroxynaphthalen-1-yl]pyridin-3-yl]-3-methylphenyl]methyl]piperazin-1-yl]-N-(3,3,3-trifluoropropylsulfonyl)pyridazine-3-carboxamide |
| | ED13-79-01R | ED13-79 | 6-[4-[[4-[4-[3-(Dimethylcarbamoyl)-4-hydroxynaphthalen-1-yl]phenyl]-2-ethylphenyl]methyl]piperazin-1-yl]-N-(3,3,3-trifluoropropylsulfonyl)pyridazine-3-carboxamide |
| mixED13-01R | ED13-80-01R | ED13-80 | 6-[4-[[4-[6-[3-(Dimethylcarbamoyl)-4-hydroxynaphthalen-1-yl]pyridin-3-yl]-2-ethylphenyl]methyl]piperazin-1-yl]-N-(3,3,3-trifluoropropylsulfonyl)pyridazine-3-carboxamide |

| | | | |
|---|---|---|---|
| | ED13-81-01R | ED13-81 | 6-[4-[[4-[4-[3-(Dimethylcarbamoyl)-4-hydroxynaphthalen-1-yl]phenyl]-3-fluorophenyl]methyl]piperazin-1-yl]-N-(3,3,3-trifluoropropylsulfonyl)pyridazine-3-carboxamide |
| | ED13-82-01R | ED13-82 | 6-[4-[[4-[6-[3-(Dimethylcarbamoyl)-4-hydroxynaphthalen-1-yl]pyridin-3-yl]-3-fluorophenyl]methyl]piperazin-1-yl]-N-(3,3,3-trifluoropropylsulfonyl)pyridazine-3-carboxamide |
| | ED13-83-01R | ED13-83 | 6-[4-[[4-[4-[3-(Dimethylcarbamoyl)-4-hydroxynaphthalen-1-yl]phenyl]-2-methoxyphenyl]methyl]piperazin-1-yl]-N-(3,3,3-trifluoropropylsulfonyl)pyridazine-3-carboxamide |
| | ED13-84-01R | ED13-84 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-(3,3,3-trifluoropropylsulfonyl)pyridazine-3-carboxamide |
| | ED13-85-01R | ED13-85 | 6-[4-[[3-[4-[3-(Dimethylcarbamoyl)-4-hydroxynaphthalen-1-yl]phenyl]-5-ethoxyphenyl]methyl]piperazin-1-yl]-N-(3,3,3-trifluoropropylsulfonyl)pyridazine-3-carboxamide |
| | ED13-86-01R | ED13-86 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-(3,3,3-trifluoropropylsulfonyl)pyridazine-3-carboxamide |
| | ED13-87-01R | ED13-87 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-(3,3,3-trifluoropropylsulfonyl)pyridazine-3-carboxamide |
| | ED13-88-01R | ED13-88 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-(3,3,3-trifluoropropylsulfonyl)pyridazine-3-carboxamide |
| | ED13-89-01R | ED13-89 | 6-[4-[[4-[4-[3-(Dimethylcarbamoyl)-4-hydroxynaphthalen-1-yl]phenyl]-2,6-dimethoxyphenyl]methyl]piperazin-1-yl]-N-(3,3,3-trifluoropropylsulfonyl)pyridazine-3-carboxamide |
| | ED13-90-01R | ED13-90 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-(3,3,3-trifluoropropylsulfonyl)pyridazine-3-carboxamide |
| | ED13-91-01R | ED13-91 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-(3,3,3-trifluoropropylsulfonyl)pyridazine-3-carboxamide |
| | ED13-92-01R | ED13-92 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-(3,3,3-trifluoropropylsulfonyl)pyridazine-3-carboxamide |
| | ED13-93-01R | ED13-93 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-(3,3,3-trifluoropropylsulfonyl)pyridazine-3-carboxamide |
| | ED13-94-01R | ED13-94 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-(3,3,3-trifluoropropylsulfonyl)pyridazine-3-carboxamide |
| | ED13-95-01R | ED13-95 | 6-[4-[[4-[4-[3-(Dimethylcarbamoyl)-4-hydroxynaphthalen-1-yl]phenyl]naphthalen-1-yl]methyl]piperazin-1-yl]-N-(3,3,3-trifluoropropylsulfonyl)pyridazine-3-carboxamide |
| mixED13-01R | ED13-96-01R | ED13-96 | 6-[4-[[4-[6-[3-(Dimethylcarbamoyl)-4-hydroxynaphthalen-1-yl]pyridin-3-yl]naphthalen-1-yl]methyl]piperazin-1-yl]-N-(3,3,3-trifluoropropylsulfonyl)pyridazine-3-carboxamide |
| | ED13-97-01R | ED13-97 | 6-[4-[[4-[4-[3-(Dimethylcarbamoyl)-4-hydroxynaphthalen-1-yl]phenyl]thiophen-2-yl]methyl]piperazin-1-yl]-N-(3,3,3-trifluoropropylsulfonyl)pyridazine-3-carboxamide |
| | ED13-98-01R | ED13-98 | 6-[4-[[4-[6-[3-(Dimethylcarbamoyl)-4-hydroxynaphthalen-1-yl]pyridin-3-yl]thiophen-2-yl]methyl]piperazin-1-yl]-N-(3,3,3-trifluoropropylsulfonyl)pyridazine-3-carboxamide |
| | ED13-99-01R | ED13-99 | 6-[4-[[2-[2-[4-[3-(Dimethylcarbamoyl)-4-hydroxynaphthalen-1-yl]phenyl]ethynyl]phenyl]methyl]piperazin-1-yl]-N-(3,3,3-trifluoropropylsulfonyl)pyridazine-3-carboxamide |

| | | | |
|---|---|---|---|
| | ED13-100-01R | ED13-100 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-(3,3,3-trifluoropropylsulfonyl)pyridazine-3-carboxamide |

[Table 113]

[Table ED14]

| No. of mixture | No. of compound that may be contained | Compound No. after cleavage | Compound name after cleavage |
|---|---|---|---|
| mixED14-01R | ED14-01-01R | ED14-01 | 1-Hydroxy-N,N-dimethyl-4-[4-[4-[1-[4-[4-[(4-nitrophenyl)sulfonylcarbamoyl]phenyl]piperazin-1-yl]ethyl]phenyl]phenyl]naphthalene-2-carboxamide |
| | ED14-02-01R | ED14-02 | 1-Hydroxy-N,N-dimethyl-4-[5-[4-[1-[4-[4-[(4-nitrophenyl)sulfonylcarbamoyl]phenyl]piperazin-1-yl]ethyl]phenyl]pyridin-2-yl]naphthalene-2-carboxamide |
| | ED14-03-01R | ED14-03 | 4-[4-[3-Fluoro-4-[1-[4-[4-[(4-nitrophenyl)sulfonylcarbamoyl]phenyl]piperazin-1-yl]ethyl]phenyl]phenyl]-1-hydroxy-N,N-dimethylnaphthalene-2-carboxamide |
| | ED14-04-01R | ED14-04 | 4-[5-[3-Fluoro-4-[1-[4-[4-[(4-nitrophenyl)sulfonylcarbamoyl]phenyl]piperazin-1-yl]ethyl]phenyl]pyridin-2-yl]-1-hydroxy-N,N-dimethylnaphthalene-2-carboxamide |
| | ED14-05-01R | ED14-05 | 6-[4-[4-[4-[3-(Dimethylcarbamoyl)-4-hydroxynaphthalen-1-yl]phenyl]-3-methylbenzoyl]piperazin-1-yl]-N-(4-nitrophenyl)sulfonylpyridazine-3-carboxamide |
| | ED14-06-01R | ED14-06 | 6-[4-[4-[6-[3-(Dimethylcarbamoyl)-4-hydroxynaphthalen-1-yl]pyridin-3-yl]-3-methylbenzoyl]piperazin-1-yl]-N-(4-nitrophenyl)sulfonylpyridazine-3-carboxamide |
| | ED14-07-01R | ED14-07 | 6-[4-[4-[4-[3-(Dimethylcarbamoyl)-4-hydroxynaphthalen-1-yl]phenyl]-3-fluorobenzoyl]piperazin-1-yl]-N-(4-nitrophenyl)sulfonylpyridazine-3-carboxamide |
| | ED14-08-01R | ED14-08 | 6-[4-[4-[6-[3-(Dimethylcarbamoyl)-4-hydroxynaphthalen-1-yl]pyridin-3-yl]-3-fluorobenzoyl]piperazin-1-yl]-N-(4-nitrophenyl)sulfonylpyridazine-3-carboxamide |
| mixED14-01R | ED14-09-01R | ED14-09 | 6-[4-[3-[4-[3-(Dimethylcarbamoyl)-4-hydroxynaphthalen-1-yl]phenyl]-5-(trifluoromethyl)benzoyl]piperazin-1-yl]-N-(4-nitrophenyl)sulfonylpyridazine-3-carboxamide |
| | ED14-10-01R | ED14-10 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-(4-nitrophenyl)sulfonylpyridazine-3-carboxamide |
| | ED14-11-01R | ED14-11 | 6-[4-[3-tert-Butyl-5-[4-[3-(dimethylcarbamoyl)-4-hydroxynaphthalen-1-yl]phenyl]benzoyl]piperazin-1-yl]-N-(4-nitrophenyl)sulfonylpyridazine-3-carboxamide |
| | ED14-12-01R | ED14-12 | 6-[4-[3-tert-Butyl-5-[6-[3-(dimethylcarbamoyl)-4-hydroxynaphthalen-1-yl]pyridin-3-yl]benzoyl]piperazin-1-yl]-N-(4-nitrophenyl)sulfonylpyridazine-3-carboxamide |
| | ED14-13-01R | ED14-13 | 6-[4-[4-[4-[3-(Dimethylcarbamoyl)-4-hydroxynaphthalen-1-yl]phenyl]naphthalene-1-carbonyl]piperazin-1-yl]-N-(4-nitrophenyl)sulfonylpyridazine-3-carboxamide |
| | ED14-14-01R | ED14-14 | 6-[4-[4-[6-[3-(Dimethylcarbamoyl)-4-hydroxynaphthalen-1-yl]pyridin-3-yl]naphthalene-1-carbonyl]piperazin-1-yl]-N-(4-nitrophenyl)sulfonylpyridazine-3-carboxamide |
| | ED14-15-01R | ED14-15 | 6-[4-[3-[2-[4-[3-(Dimethylcarbamoyl)-4-hydroxynaphthalen-1-yl]phenyl]phenyl]propanoyl]piperazin-1-yl]-N-(4-nitrophenyl)sulfonylpyridazine-3-carboxamide |

| | | | |
|---|---|---|---|
| | ED14-16-01R | ED14-16 | 6-[4-[3-[2-[6-[3-(Dimethylcarbamoyl)-4-hydroxynaphthalen-1-yl]pyridin-3-yl]phenyl]propanoyl]piperazin-1-yl]-N-(4-nitrophenyl)sulfonylpyridazine-3-carboxamide |
| | ED14-17-01R | ED14-17 | 6-[4-[4-[4-[3-(Dimethylcarbamoyl)-4-hydroxynaphthalen-1-yl]phenyl]thiophene-2-carbonyl]piperazin-1-yl]-N-(4-nitrophenyl)sulfonylpyridazine-3-carboxamide |
| | ED14-18-01R | ED14-18 | 6-[4-[4-[6-[3-(Dimethylcarbamoyl)-4-hydroxynaphthalen-1-yl]pyridin-3-yl]thiophene-2-carbonyl]piperazin-1-yl]-N-(4-nitrophenyl)sulfonylpyridazine-3-carboxamide |
| | ED14-19-01R | ED14-19 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-(4-nitrophenyl)sulfonylpyridazine-3-carboxamide |
| | ED14-20-01R | ED14-20 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-(4-nitrophenyl)sulfonylpyridazine-3-carboxamide |
| | ED14-21-01R | ED14-21 | 6-[4-[2-[2-[4-[3-(Dimethylcarbamoyl)-4-hydroxynaphthalen-1-yl]phenyl]ethynyl]benzoyl]piperazin-1-yl]-N-(4-nitrophenyl)sulfonylpyridazine-3-carboxamide |
| | ED14-22-01R | ED14-22 | 6-[4-[2-[2-[6-[3-(Dimethylcarbamoyl)-4-hydroxynaphthalen-1-yl]pyridin-3-yl]ethynyl]benzoyl]piperazin-1-yl]-N-(4-nitrophenyl)sulfonylpyridazine-3-carboxamide |
| | ED14-23-01R | ED14-23 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-(4-nitrophenyl)sulfonylpyridazine-3-carboxamide |
| | ED14-24-01R | ED14-24 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-(4-nitrophenyl)sulfonylpyridazine-3-carboxamide |
| mixED14-01R | ED14-25-01R | ED14-25 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-(4-nitrophenyl)sulfonylpyridazine-3-carboxamide |
| | ED14-26-01R | ED14-26 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-(4-nitrophenyl)sulfonylpyridazine-3-carboxamide |
| | ED14-27-01R | ED14-27 | 1-Hydroxy-N,N-dimethyl-4-[4-[2-methyl-4-[4-[4-[(4-nitrophenyl)sulfonylcarbamoyl]phenyl]piperazine-1-carbonyl]phenyl]phenyl]naphthalene-2-carboxamide |
| | ED14-28-01R | ED14-28 | 1-Hydroxy-N,N-dimethyl-4-[5-[2-methyl-4-[4-[4-[(4-nitrophenyl)sulfonylcarbamoyl]phenyl]piperazine-1-carbonyl]phenyl]pyridin-2-yl]naphthalene-2-carboxamide |
| | ED14-29-01R | ED14-29 | 4-[4-[2-Fluoro-4-[4-[4-[(4-nitrophenyl)sulfonylcarbamoyl]phenyl]piperazine-1-carbonyl]phenyl]phenyl]-1-hydroxy-N,N-dimethylnaphthalene-2-carboxamide |
| | ED14-30-01R | ED14-30 | 4-[5-[2-Fluoro-4-[4-[4-[(4-nitrophenyl)sulfonylcarbamoyl]phenyl]piperazine-1-carbonyl]phenyl]pyridin-2-yl]-1-hydroxy-N,N-dimethylnaphthalene-2-carboxamide |
| | ED14-31-01R | ED14-31 | 1-Hydroxy-N,N-dimethyl-4-[4-[3-[4-[4-[(4-nitrophenyl)sulfonylcarbamoyl]phenyl]piperazine-1-carbonyl]-5-(trifluoromethyl)phenyl]phenyl]naphthalene-2-carboxamide |
| | ED14-32-01R | ED14-32 | 1-Hydroxy-N,N-dimethyl-4-[5-[3-[4-[4-[(4-nitrophenyl)sulfonylcarbamoyl]phenyl]piperazine-1-carbonyl]-5-(trifluoromethyl)phenyl]pyridin-2-yl]naphthalene-2-carboxamide |
| | ED14-33-01R | ED14-33 | 4-[4-[3-tert-Butyl-5-[4-[4-[(4-nitrophenyl)sulfonylcarbamoyl]phenyl]piperazine-1-carbonyl]phenyl]phenyl]-1-hydroxy-N,N-dimethylnaphthalene-2-carboxamide |

| | | | |
|---|---|---|---|
| | ED14-34-01R | ED14-34 | 4-[5-[3-tert-Butyl-5-[4-[4-[(4-nitrophenyl)sulfonylcarbamoyl]phenyl]piperazine-1-carbonyl]phenyl]pyridin-2-yl]-1-hydroxy-N,N-dimethylnaphthalene-2-carboxamide |
| | ED14-35-01R | ED14-35 | 1-Hydroxy-N,N-dimethyl-4-[4-[4-[4-[4-[(4-nitrophenyl)sulfonylcarbamoyl]phenyl]piperazine-1-carbonyl]naphthalen-1-yl]phenyl]naphthalene-2-carboxamide |
| | ED14-36-01R | ED14-36 | 1-Hydroxy-N,N-dimethyl-4-[5-[4-[4-[4-[(4-nitrophenyl)sulfonylcarbamoyl]phenyl]piperazine-1-carbonyl]naphthalen-1-yl]pyridin-2-yl]naphthalene-2-carboxamide |
| | ED14-37-01R | ED14-37 | 1-Hydroxy-N,N-dimethyl-4-[4-[2-[3-[4-[4-[(4-nitrophenyl)sulfonylcarbamoyl]phenyl]piperazin-1-yl]-3-oxopropyl]phenyl]phenyl]naphthalene-2-carboxamide |
| | ED14-38-01R | ED14-38 | 1-Hydroxy-N,N-dimethyl-4-[5-[2-[3-[4-[4-[(4-nitrophenyl)sulfonylcarbamoyl]phenyl]piperazin-1-yl]-3-oxopropyl]phenyl]pyridin-2-yl]naphthalene-2-carboxamide |
| | ED14-39-01R | ED14-39 | 1-Hydroxy-N,N-dimethyl-4-[4-[5-[4-[4-[(4-nitrophenyl)sulfonylcarbamoyl]phenyl]piperazine-1-carbonyl]thiophen-3-yl]phenyl]naphthalene-2-carboxamide |
| mixED14-01R | ED14-40-01R | ED14-40 | 1-Hydroxy-N,N-dimethyl-4-[5-[5-[4-[4-[(4-nitrophenyl)sulfonylcarbamoyl]phenyl]piperazine-1-carbonyl]thiophen-3-yl]pyridin-2-yl]naphthalene-2-carboxamide |
| | ED14-41-01R | ED14-41 | 1-Hydroxy-N,N-dimethyl-4-[4-[1-[3-[4-[4-[(4-nitrophenyl)sulfonylcarbamoyl]phenyl]piperazine-1-carbonyl]-5-(trifluoromethyl)phenyl]pyrazol-4-yl]phenyl]naphthalene-2-carboxamide |
| | ED14-42-01R | ED14-42 | 1-Hydroxy-N,N-dimethyl-4-[5-[1-[3-[4-[4-[(4-nitrophenyl)sulfonylcarbamoyl]phenyl]piperazine-1-carbonyl]-5-(trifluoromethyl)phenyl]pyrazol-4-yl]pyridin-2-yl]naphthalene-2-carboxamide |
| | ED14-43-01R | ED14-43 | 1-Hydroxy-N,N-dimethyl-4-[4-[2-[2-[4-[4-[(4-nitrophenyl)sulfonylcarbamoyl]phenyl]piperazine-1-carbonyl]phenyl]ethynyl]phenyl]naphthalene-2-carboxamide |
| | ED14-44-01R | ED14-44 | 1-Hydroxy-N,N-dimethyl-4-[5-[2-[2-[4-[4-[(4-nitrophenyl)sulfonylcarbamoyl]phenyl]piperazine-1-carbonyl]phenyl]ethynyl]pyridin-2-yl]naphthalene-2-carboxamide |
| | ED14-45-01R | ED14-45 | 1-Hydroxy-N,N-dimethyl-4-[4-[2-[3-[4-[4-[(4-nitrophenyl)sulfonylcarbamoyl]phenyl]piperazine-1-carbonyl]-5-(trifluoromethyl)phenyl]ethynyl]phenyl]naphthalene-2-carboxamide |
| | ED14-46-01R | ED14-46 | 1-Hydroxy-N,N-dimethyl-4-[5-[2-[3-[4-[4-[(4-nitrophenyl)sulfonylcarbamoyl]phenyl]piperazine-1-carbonyl]-5-(trifluoromethyl)phenyl]ethynyl]pyridin-2-yl]naphthalene-2-carboxamide |
| | ED14-47-01R | ED14-47 | 1-Hydroxy-N,N-dimethyl-4-[4-[2-[5-[4-[4-[(4-nitrophenyl)sulfonylcarbamoyl]phenyl]piperazine-1-carbonyl]pyridin-3-yl]ethynyl]phenyl]naphthalene-2-carboxamide |
| | ED14-48-01R | ED14-48 | 1-Hydroxy-N,N-dimethyl-4-[5-[2-[5-[4-[4-[(4-nitrophenyl)sulfonylcarbamoyl]phenyl]piperazine-1-carbonyl]pyridin-3-yl]ethynyl]pyridin-2-yl]naphthalene-2-carboxamide |
| | ED14-49-01R | ED14-49 | 1-Hydroxy-N,N-dimethyl-4-[4-[2-[[4-[4-[(4-nitrophenyl)sulfonylcarbamoyl]phenyl]piperazin-1-yl]methyl]phenyl]phenyl]naphthalene-2-carboxamide |
| | ED14-50-01R | ED14-50 | 1-Hydroxy-N,N-dimethyl-4-[5-[2-[[4-[4-[(4-nitrophenyl)sulfonylcarbamoyl]phenyl]piperazin-1-yl]methyl]phenyl]pyridin-2-yl]naphthalene-2-carboxamide |
| | ED14-51-01R | ED14-51 | 1-Hydroxy-N,N-dimethyl-4-[4-[2-methyl-4-[[4-[4-[(4-nitrophenyl)sulfonylcarbamoyl]phenyl]piperazin-1-yl]methyl]phenyl]phenyl]naphthalene-2-carboxamide |

| | | | |
|---|---|---|---|
| | ED14-52-01R | ED14-52 | 1-Hydroxy-N,N-dimethyl-4-[5-[2-methyl-4-[[4-[4-[(4-nitrophenyl)sulfonylcarbamoyl]phenyl]piperazin-1-yl]methyl]phenyl]pyridin-2-yl]naphthalene-2-carboxamide |
| | ED14-53-01R | ED14-53 | 4-[4-[3-Ethyl-4-[[4-[4-[(4-nitrophenyl)sulfonylcarbamoyl]phenyl]piperazin-1-yl]methyl]phenyl]phenyl]-1-hydroxy-N,N-dimethylnaphthalene-2-carboxamide |
| | ED14-54-01R | ED14-54 | 4-[5-[3-Ethyl-4-[[4-[4-[(4-nitrophenyl)sulfonylcarbamoyl]phenyl]piperazin-1-yl]methyl]phenyl]pyridin-2-yl]-1-hydroxy-N,N-dimethylnaphthalene-2-carboxamide |
| mixED14-01R | ED14-55-01R | ED14-55 | 4-[4-[2-Fluoro-4-[[4-[4-[(4-nitrophenyl)sulfonylcarbamoyl]phenyl]piperazin-1-yl]methyl]phenyl]phenyl]-1-hydroxy-N,N-dimethylnaphthalene-2-carboxamide |
| | ED14-56-01R | ED14-56 | 4-[5-[2-Fluoro-4-[[4-[4-[(4-nitrophenyl)sulfonylcarbamoyl]phenyl]piperazin-1-yl]methyl]phenyl]pyridin-2-yl]-1-hydroxy-N,N-dimethylnaphthalene-2-carboxamide |
| | ED14-57-01R | ED14-57 | 1-Hydroxy-4-[4-[3-methoxy-4-[[4-[4-[(4-nitrophenyl)sulfonylcarbamoyl]phenyl]piperazin-1-yl]methyl]phenyl]phenyl]-N,N-dimethylnaphthalene-2-carboxamide |
| | ED14-58-01R | ED14-58 | 1-Hydroxy-4-[5-[3-methoxy-4-[[4-[4-[(4-nitrophenyl)sulfonylcarbamoyl]phenyl]piperazin-1-yl]methyl]phenyl]pyridin-2-yl]-N,N-dimethylnaphthalene-2-carboxamide |
| | ED14-59-01R | ED14-59 | 4-[4-[3-Ethoxy-5-[[4-[4-[(4-nitrophenyl)sulfonylcarbamoyl]phenyl]piperazin-1-yl]methyl]phenyl]phenyl]-1-hydroxy-N,N-dimethylnaphthalene-2-carboxamide |
| | ED14-60-01R | ED14-60 | 4-[5-[3-Ethoxy-5-[[4-[4-[(4-nitrophenyl)sulfonylcarbamoyl]phenyl]piperazin-1-yl]methyl]phenyl]pyridin-2-yl]-1-hydroxy-N,N-dimethylnaphthalene-2-carboxamide |
| | ED14-61-01R | ED14-61 | 1-Hydroxy-N,N-dimethyl-4-[4-[3-[[4-[4-[(4-nitrophenyl)sulfonylcarbamoyl]phenyl]piperazin-1-yl]methyl]-5-propan-2-yloxyphenyl]phenyl]naphthalene-2-carboxamide |
| | ED14-62-01R | ED14-62 | 1-Hydroxy-N,N-dimethyl-4-[5-[3-[[4-[4-[(4-nitrophenyl)sulfonylcarbamoyl]phenyl]piperazin-1-yl]methyl]-5-propan-2-yloxyphenyl]pyridin-2-yl]naphthalene-2-carboxamide |
| | ED14-63-01R | ED14-63 | 4-[4-[3,5-Dimethoxy-4-[[4-[4-[(4-nitrophenyl)sulfonylcarbamoyl]phenyl]piperazin-1-yl]methyl]phenyl]phenyl]-1-hydroxy-N,N-dimethylnaphthalene-2-carboxamide |
| | ED14-64-01R | ED14-64 | 4-[5-[3,5-Dimethoxy-4-[[4-[4-[(4-nitrophenyl)sulfonylcarbamoyl]phenyl]piperazin-1-yl]methyl]phenyl]pyridin-2-yl]-1-hydroxy-N,N-dimethylnaphthalene-2-carboxamide |
| | ED14-65-01R | ED14-65 | 1-Hydroxy-N,N-dimethyl-4-[4-[3-[[4-[4-[(4-nitrophenyl)sulfonylcarbamoyl]phenyl]piperazin-1-yl]methyl]-5-(trifluoromethyl)phenyl]phenyl]naphthalene-2-carboxamide |
| | ED14-66-01R | ED14-66 | 1-Hydroxy-N,N-dimethyl-4-[5-[3-[[4-[4-[(4-nitrophenyl)sulfonylcarbamoyl]phenyl]piperazin-1-yl]methyl]-5-(trifluoromethyl)phenyl]pyridin-2-yl]naphthalene-2-carboxamide |
| | ED14-67-01R | ED14-67 | 1-Hydroxy-N,N-dimethyl-4-[4-[3-[[4-[4-[(4-nitrophenyl)sulfonylcarbamoyl]phenyl]piperazin-1-yl]methyl]-5-(trifluoromethoxy)phenyl]phenyl]naphthalene-2-carboxamide |
| | ED14-68-01R | ED14-68 | 1-Hydroxy-N,N-dimethyl-4-[5-[3-[[4-[4-[(4-nitrophenyl)sulfonylcarbamoyl]phenyl]piperazin-1-yl]methyl]-5-(trifluoromethoxy)phenyl]pyridin-2-yl]naphthalene-2-carboxamide |
| | ED14-69-01R | ED14-69 | 1-Hydroxy-N,N-dimethyl-4-[4-[4-[[4-[4-[(4-nitrophenyl)sulfonylcarbamoyl]phenyl]piperazin-1-yl]methyl]naphthalen-1-yl]phenyl]naphthalene-2-carboxamide |

| | | | |
|---|---|---|---|
| mixED14-01R | ED14-70-01R | ED14-70 | 1-Hydroxy-N,N-dimethyl-4-[5-[4-[[4-[4-[(4-nitrophenyl)sulfonylcarbamoyl]phenyl]piperazin-1-yl]methyl]naphthalen-1-yl]pyridin-2-yl]naphthalene-2-carboxamide |
| | ED14-71-01R | ED14-71 | 1-Hydroxy-N,N-dimethyl-4-[4-[5-[[4-[4-[(4-nitrophenyl)sulfonylcarbamoyl]phenyl]piperazin-1-yl]methyl]thiophen-3-yl]phenyl]naphthalene-2-carboxamide |
| | ED14-72-01R | ED14-72 | 1-Hydroxy-N,N-dimethyl-4-[5-[5-[[4-[4-[(4-nitrophenyl)sulfonylcarbamoyl]phenyl]piperazin-1-yl]methyl]thiophen-3-yl]pyridin-2-yl]naphthalene-2-carboxamide |
| | ED14-73-01R | ED14-73 | 1-Hydroxy-N,N-dimethyl-4-[4-[2-[2-[[4-[4-[(4-nitrophenyl)sulfonylcarbamoyl]phenyl]piperazin-1-yl]methyl]phenyl]ethynyl]phenyl]naphthalene-2-carboxamide |
| | ED14-74-01R | ED14-74 | 1-Hydroxy-N,N-dimethyl-4-[5-[2-[2-[[4-[4-[(4-nitrophenyl)sulfonylcarbamoyl]phenyl]piperazin-1-yl]methyl]phenyl]ethynyl]pyridin-2-yl]naphthalene-2-carboxamide |
| | ED14-75-01R | ED14-75 | 6-[4-[[2-[4-[3-(Dimethylcarbamoyl)-4-hydroxynaphthalen-1-yl]phenyl]phenyl]methyl]piperazin-1-yl]-N-(4-nitrophenyl)sulfonylpyridazine-3-carboxamide |
| | ED14-76-01R | ED14-76 | 6-[4-[[2-[6-[3-(Dimethylcarbamoyl)-4-hydroxynaphthalen-1-yl]pyridin-3-yl]phenyl]methyl]piperazin-1-yl]-N-(4-nitrophenyl)sulfonylpyridazine-3-carboxamide |
| | ED14-77-01R | ED14-77 | 6-[4-[[4-[4-[3-(Dimethylcarbamoyl)-4-hydroxynaphthalen-1-yl]phenyl]-3-methylphenyl]methyl]piperazin-1-yl]-N-(4-nitrophenyl)sulfonylpyridazine-3-carboxamide |
| | ED14-78-01R | ED14-78 | 6-[4-[[4-[6-[3-(Dimethylcarbamoyl)-4-hydroxynaphthalen-1-yl]pyridin-3-yl]-3-methylphenyl]methyl]piperazin-1-yl]-N-(4-nitrophenyl)sulfonylpyridazine-3-carboxamide |
| | ED14-79-01R | ED14-79 | 6-[4-[[4-[4-[3-(Dimethylcarbamoyl)-4-hydroxynaphthalen-1-yl]phenyl]-2-ethylphenyl]methyl]piperazin-1-yl]-N-(4-nitrophenyl)sulfonylpyridazine-3-carboxamide |
| | ED14-80-01R | ED14-80 | 6-[4-[[4-[6-[3-(Dimethylcarbamoyl)-4-hydroxynaphthalen-1-yl]pyridin-3-yl]-2-ethylphenyl]methyl]piperazin-1-yl]-N-(4-nitrophenyl)sulfonylpyridazine-3-carboxamide |
| | ED14-81-01R | ED14-81 | 6-[4-[[4-[4-[3-(Dimethylcarbamoyl)-4-hydroxynaphthalen-1-yl]phenyl]-3-fluorophenyl]methyl]piperazin-1-yl]-N-(4-nitrophenyl)sulfonylpyridazine-3-carboxamide |
| | ED14-82-01R | ED14-82 | 6-[4-[[4-[6-[3-(Dimethylcarbamoyl)-4-hydroxynaphthalen-1-yl]pyridin-3-yl]-3-fluorophenyl]methyl]piperazin-1-yl]-N-(4-nitrophenyl)sulfonylpyridazine-3-carboxamide |
| | ED14-83-01R | ED14-83 | 6-[4-[[4-[4-[3-(Dimethylcarbamoyl)-4-hydroxynaphthalen-1-yl]phenyl]-2-methoxyphenyl]methyl]piperazin-1-yl]-N-(4-nitrophenyl)sulfonylpyridazine-3-carboxamide |
| | ED14-84-01R | ED14-84 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-(4-nitrophenyl)sulfonylpyridazine-3-carboxamide |
| | ED14-85-01R | ED14-85 | 6-[4-[[3-[4-[3-(Dimethylcarbamoyl)-4-hydroxynaphthalen-1-yl]phenyl]-5-ethoxyphenyl]methyl]piperazin-1-yl]-N-(4-nitrophenyl)sulfonylpyridazine-3-carboxamide |
| mixED14-01R | ED14-86-01R | ED14-86 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-(4-nitrophenyl)sulfonylpyridazine-3-carboxamide |
| | ED14-87-01R | ED14-87 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-(4-nitrophenyl)sulfonylpyridazine-3-carboxamide |
| | ED14-88-01R | ED14-88 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-(4-nitrophenyl)sulfonylpyridazine-3-carboxamide |

| | | | |
|---|---|---|---|
| | ED14-89-01R | ED14-89 | 6-[4-[[4-[4-[3-(Dimethylcarbamoyl)-4-hydroxynaphthalen-1-yl]phenyl]-2,6-dimethoxyphenyl]methyl]piperazin-1-yl]-N-(4-nitrophenyl)sulfonylpyridazine-3-carboxamide |
| | ED14-90-01R | ED14-90 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-(4-nitrophenyl)sulfonylpyridazine-3-carboxamide |
| | ED14-91-01R | ED14-91 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-(4-nitrophenyl)sulfonylpyridazine-3-carboxamide |
| | ED14-92-01R | ED14-92 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-(4-nitrophenyl)sulfonylpyridazine-3-carboxamide |
| | ED14-93-01R | ED14-93 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-(4-nitrophenyl)sulfonylpyridazine-3-carboxamide |
| | ED14-94-01R | ED14-94 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-(4-nitrophenyl)sulfonylpyridazine-3-carboxamide |
| | ED14-95-01R | ED14-95 | 6-[4-[[4-[4-[3-(Dimethylcarbamoyl)-4-hydroxynaphthalen-1-yl]phenyl]naphthalen-1-yl]methyl]piperazin-1-yl]-N-(4-nitrophenyl)sulfonylpyridazine-3-carboxamide |
| | ED14-96-01R | ED14-96 | 6-[4-[[4-[6-[3-(Dimethylcarbamoyl)-4-hydroxynaphthalen-1-yl]pyridin-3-yl]naphthalen-1-yl]methyl]piperazin-1-yl]-N-(4-nitrophenyl)sulfonylpyridazine-3-carboxamide |
| | ED14-97-01R | ED14-97 | 6-[4-[[4-[4-[3-(Dimethylcarbamoyl)-4-hydroxynaphthalen-1-yl]phenyl]thiophen-2-yl]methyl]piperazin-1-yl]-N-(4-nitrophenyl)sulfonylpyridazine-3-carboxamide |
| | ED14-98-01R | ED14-98 | 6-[4-[[4-[6-[3-(Dimethylcarbamoyl)-4-hydroxynaphthalen-1-yl]pyridin-3-yl]thiophen-2-yl]methyl]piperazin-1-yl]-N-(4-nitrophenyl)sulfonylpyridazine-3-carboxamide |
| | ED14-99-01R | ED14-99 | 6-[4-[[2-[2-[4-[3-(Dimethylcarbamoyl)-4-hydroxynaphthalen-1-yl]phenyl]ethynyl]phenyl]methyl]piperazin-1-yl]-N-(4-nitrophenyl)sulfonylpyridazine-3-carboxamide |
| | ED14-100-01R | ED14-100 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-(4-nitrophenyl)sulfonylpyridazine-3-carboxamide |

[Table 114]

[Table ED15]

| No. of mixture | No. of compound that may be contained | Compound No. after cleavage | Compound name after cleavage |
|---|---|---|---|
| mixED15-01R | ED15-01-01R | ED15-01 | 4-[4-[4-[1-[4-[4-(1-Adamantylmethylsulfonylcarbamoyl)phenyl]piperazin-1-yl]ethyl]phenyl]phenyl]-1-hydroxy-N,N-dimethylnaphthalene-2-carboxamide |
| | ED15-02-01R | ED15-02 | 4-[5-[4-[1-[4-[4-(1-Adamantylmethylsulfonylcarbamoyl)phenyl]piperazin-1-yl]ethyl]phenyl]pyridin-2-yl]-1-hydroxy-N,N-dimethylnaphthalene-2-carboxamide |
| | ED15-03-01R | ED15-03 | 4-[4-[4-[1-[4-[4-(1-Adamantylmethylsulfonylcarbamoyl)phenyl]piperazin-1-yl]ethyl]-3-fluorophenyl]phenyl]-1-hydroxy-N,N-dimethylnaphthalene-2-carboxamide |
| | ED15-04-01R | ED15-04 | 4-[5-[4-[1-[4-[4-(1-Adamantylmethylsulfonylcarbamoyl)phenyl]piperazin-1-yl]ethyl]-3-fluorophenyl]pyridin-2-yl]-1-hydroxy-N,N-dimethylnaphthalene-2-carboxamide |

| | | | |
|---|---|---|---|
| | ED15-05-01R | ED15-05 | N-(1-Adamantylmethylsulfonyl)-6-[4-[4-[4-[3-(dimethylcarbamoyl)-4-hydroxynaphthalen-1-yl]phenyl]-3-methylbenzoyl]piperazin-1-yl]pyridazine-3-carboxamide |
| | ED15-06-01R | ED15-06 | N-(1-Adamantylmethylsulfonyl)-6-[4-[4-[6-[3-(dimethylcarbamoyl)-4-hydroxynaphthalen-1-yl]pyridin-3-yl]-3-methylbenzoyl]piperazin-1-yl]pyridazine-3-carboxamide |
| | ED15-07-01R | ED15-07 | N-(1-Adamantylmethylsulfonyl)-6-[4-[4-[4-[3-(dimethylcarbamoyl)-4-hydroxynaphthalen-1-yl]phenyl]-3-fluorobenzoyl]piperazin-1-yl]pyridazine-3-carboxamide |
| | ED15-08-01R | ED15-08 | N-(1-Adamantylmethylsulfonyl)-6-[4-[4-[6-[3-(dimethylcarbamoyl)-4-hydroxynaphthalen-1-yl]pyridin-3-yl]-3-fluorobenzoyl]piperazin-1-yl]pyridazine-3-carboxamide |
| | ED15-09-01R | ED15-09 | N-(1-Adamantylmethylsulfonyl)-6-[4-[3-[4-[3-(dimethylcarbamoyl)-4-hydroxynaphthalen-1-yl]phenyl]-5-(trifluoromethyl)benzoyl]piperazin-1-yl]pyridazine-3-carboxamide |
| | ED15-10-01R | ED15-10 | N-(1-Adamantylmethylsulfonyl)-6-[4-[3-[6-[3-(dimethylcarbamoyl)-4-hydroxynaphthalen-1-yl]pyridin-3-yl]-5-(trifluoromethyl)benzoyl]piperazin-1-yl]pyridazine-3-carboxamide |
| | ED15-11-01R | ED15-11 | N-(1-Adamantylmethylsulfonyl)-6-[4-[3-tert-butyl-5-[4-[3-(dimethylcarbamoyl)-4-hydroxynaphthalen-1-yl]phenyl]benzoyl]piperazin-1-yl]pyridazine-3-carboxamide |
| | ED15-12-01R | ED15-12 | N-(1-Adamantylmethylsulfonyl)-6-[4-[3-tert-butyl-5-[6-[3-(dimethylcarbamoyl)-4-hydroxynaphthalen-1-yl]pyridin-3-yl]benzoyl]piperazin-1-yl]pyridazine-3-carboxamide |
| | ED15-13-01R | ED15-13 | N-(1-Adamantylmethylsulfonyl)-6-[4-[4-[4-[3-(dimethylcarbamoyl)-4-hydroxynaphthalen-1-yl]phenyl]naphthalene-1-carbonyl]piperazin-1-yl]pyridazine-3-carboxamide |
| | ED15-14-01R | ED15-14 | N-(1-Adamantylmethylsulfonyl)-6-[4-[4-[6-[3-(dimethylcarbamoyl)-4-hydroxynaphthalen-1-yl]pyridin-3-yl]naphthalene-1-carbonyl]piperazin-1-yl]pyridazine-3-carboxamide |
| | ED15-15-01R | ED15-15 | N-(1-Adamantylmethylsulfonyl)-6-[4-[3-[2-[4-[3-(dimethylcarbamoyl)-4-hydroxynaphthalen-1-yl]phenyl]phenyl]propanoyl]piperazin-1-yl]pyridazine-3-carboxamide |
| mixED15-01R | ED15-16-01R | ED15-16 | N-(1-Adamantylmethylsulfonyl)-6-[4-[3-[2-[6-[3-(dimethylcarbamoyl)-4-hydroxynaphthalen-1-yl]pyridin-3-yl]phenyl]propanoyl]piperazin-1-yl]pyridazine-3-carboxamide |
| | ED15-17-01R | ED15-17 | N-(1-Adamantylmethylsulfonyl)-6-[4-[4-[4-[3-(dimethylcarbamoyl)-4-hydroxynaphthalen-1-yl]phenyl]thiophene-2-carbonyl]piperazin-1-yl]pyridazine-3-carboxamide |
| | ED15-18-01R | ED15-18 | N-(1-Adamantylmethylsulfonyl)-6-[4-[4-[6-[3-(dimethylcarbamoyl)-4-hydroxynaphthalen-1-yl]pyridin-3-yl]thiophene-2-carbonyl]piperazin-1-yl]pyridazine-3-carboxamide |
| | ED15-19-01R | ED15-19 | N-(1-Adamantylmethylsulfonyl)-6-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]pyridazine-3-carboxamide |
| | ED15-20-01R | ED15-20 | N-(1-Adamantylmethylsulfonyl)-6-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]pyridazine-3-carboxamide |
| | ED15-21-01R | ED15-21 | N-(1-Adamantylmethylsulfonyl)-6-[4-[2-[2-[4-[3-(dimethylcarbamoyl)-4-hydroxynaphthalen-1-yl]phenyl]ethynyl]benzoyl]piperazin-1-yl]pyridazine-3-carboxamide |
| | ED15-22-01R | ED15-22 | N-(1-Adamantylmethylsulfonyl)-6-[4-[2-[2-[6-[3-(dimethylcarbamoyl)-4-hydroxynaphthalen-1-yl]pyridin-3-yl]ethynyl]benzoyl]piperazin-1-yl]pyridazine-3-carboxamide |

| | | | |
|---|---|---|---|
| | ED15-23-01R | ED15-23 | N-(1-Adamantylmethylsulfonyl)-6-[4-[3-[2-[4-[3-(dimethylcarbamoyl)-4-hydroxynaphthalen-1-yl]phenyl]ethynyl]-5-(trifluoromethyl)benzoyl]piperazin-1-yl]pyridazine-3-carboxamide |
| | ED15-24-01R | ED15-24 | N-(1-Adamantylmethylsulfonyl)-6-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]pyridazine-3-carboxamide |
| | ED15-25-01R | ED15-25 | N-(1-Adamantylmethylsulfonyl)-6-[4-[5-[2-[4-[3-(dimethylcarbamoyl)-4-hydroxynaphthalen-1-yl]phenyl]ethynyl]pyridine-3-carbonyl]piperazin-1-yl]pyridazine-3-carboxamide |
| | ED15-26-01R | ED15-26 | N-(1-Adamantylmethylsulfonyl)-6-[4-[5-[2-[6-[3-(dimethylcarbamoyl)-4-hydroxynaphthalen-1-yl]pyridin-3-yl]ethynyl]pyridine-3-carbonyl]piperazin-1-yl]pyridazine-3-carboxamide |
| | ED15-27-01R | ED15-27 | 4-[4-[4-[4-[4-(1-Adamantylmethylsulfonylcarbamoyl)phenyl]piperazine-1-carbonyl]-2-methylphenyl]phenyl]-1-hydroxy-N,N-dimethylnaphthalene-2-carboxamide |
| | ED15-28-01R | ED15-28 | 4-[5-[4-[4-[4-(1-Adamantylmethylsulfonylcarbamoyl)phenyl]piperazine-1-carbonyl]-2-methylphenyl]pyridin-2-yl]-1-hydroxy-N,N-dimethylnaphthalene-2-carboxamide |
| | ED15-29-01R | ED15-29 | 4-[4-[4-[4-[4-(1-Adamantylmethylsulfonylcarbamoyl)phenyl]piperazine-1-carbonyl]-2-fluorophenyl]phenyl]-1-hydroxy-N,N-dimethylnaphthalene-2-carboxamide |
| | ED15-30-01R | ED15-30 | 4-[5-[4-[4-[4-(1-Adamantylmethylsulfonylcarbamoyl)phenyl]piperazine-1-carbonyl]-2-fluorophenyl]pyridin-2-yl]-1-hydroxy-N,N-dimethylnaphthalene-2-carboxamide |
| | ED15-31-01R | ED15-31 | 4-[4-[3-[4-[4-(1-Adamantylmethylsulfonylcarbamoyl)phenyl]piperazine-1-carbonyl]-5-(trifluoromethyl)phenyl]phenyl]-1-hydroxy-N,N-dimethylnaphthalene-2-carboxamide |
| | ED15-32-01R | ED15-32 | 4-[5-[3-[4-[4-(1-Adamantylmethylsulfonylcarbamoyl)phenyl]piperazine-1-carbonyl]-5-(trifluoromethyl)phenyl]pyridin-2-yl]-1-hydroxy-N,N-dimethylnaphthalene-2-carboxamide |
| | ED15-33-01R | ED15-33 | 4-[4-[3-[4-[4-(1-Adamantylmethylsulfonylcarbamoyl)phenyl]piperazine-1-carbonyl]-5-tert-butylphenyl]phenyl]-1-hydroxy-N,N-dimethylnaphthalene-2-carboxamide |
| | ED15-34-01R | ED15-34 | 4-[5-[3-[4-[4-(1-Adamantylmethylsulfonylcarbamoyl)phenyl]piperazine-1-carbonyl]-5-tert-butylphenyl]pyridin-2-yl]-1-hydroxy-N,N-dimethylnaphthalene-2-carboxamide |
| | ED15-35-01R | ED15-35 | 4-[4-[4-[4-[4-(1-Adamantylmethylsulfonylcarbamoyl)phenyl]piperazine-1-carbonyl]naphthalen-1-yl]phenyl]-1-hydroxy-N,N-dimethylnaphthalene-2-carboxamide |
| | ED15-36-01R | ED15-36 | 4-[5-[4-[4-[4-(1-Adamantylmethylsulfonylcarbamoyl)phenyl]piperazine-1-carbonyl]naphthalen-1-yl]pyridin-2-yl]-1-hydroxy-N,N-dimethylnaphthalene-2-carboxamide |
| | ED15-37-01R | ED15-37 | 4-[4-[2-[3-[4-[4-(1-Adamantylmethylsulfonylcarbamoyl)phenyl]piperazin-1-yl]-3-oxopropyl]phenyl]phenyl]-1-hydroxy-N,N-dimethylnaphthalene-2-carboxamide |
| | ED15-38-01R | ED15-38 | 4-[5-[2-[3-[4-[4-(1-Adamantylmethylsulfonylcarbamoyl)phenyl]piperazin-1-yl]-3-oxopropyl]phenyl]pyridin-2-yl]-1-hydroxy-N,N-dimethylnaphthalene-2-carboxamide |
| | ED15-39-01R | ED15-39 | 4-[4-[5-[4-[4-(1-Adamantylmethylsulfonylcarbamoyl)phenyl]piperazine-1-carbonyl]thiophen-3-yl]phenyl]-1-hydroxy-N,N-dimethylnaphthalene-2-carboxamide |
| | ED15-40-01R | ED15-40 | 4-[5-[5-[4-[4-(1-Adamantylmethylsulfonylcarbamoyl)phenyl]piperazine-1-carbonyl]thiophen-3-yl]pyridin-2-yl]-1-hydroxy-N,N-dimethylnaphthalene-2-carboxamide |

| | | | |
|---|---|---|---|
| | ED15-41-01R | ED15-41 | 4-[4-[1-[3-[4-[4-(1-Adamantylmethylsulfonylcarbamoyl)phenyl]piperazine-1-carbonyl]-5-(trifluoromethyl)phenyl]pyrazol-4-yl]phenyl]-1-hydroxy-N,N-dimethylnaphthalene-2-carboxamide |
| | ED15-42-01R | ED15-42 | 4-[5-[1-[3-[4-[4-(1-Adamantylmethylsulfonylcarbamoyl)phenyl]piperazine-1-carbonyl]-5-(trifluoromethyl)phenyl]pyrazol-4-yl]pyridin-2-yl]-1-hydroxy-N,N-dimethylnaphthalene-2-carboxamide |
| | ED15-43-01R | ED15-43 | 4-[4-[2-[2-[4-[4-(1-Adamantylmethylsulfonylcarbamoyl)phenyl]piperazine-1-carbonyl]phenyl]ethynyl]phenyl]-1-hydroxy-N,N-dimethylnaphthalene-2-carboxamide |
| | ED15-44-01R | ED15-44 | 4-[5-[2-[2-[4-[4-(1-Adamantylmethylsulfonylcarbamoyl)phenyl]piperazine-1-carbonyl]phenyl]ethynyl]pyridin-2-yl]-1-hydroxy-N,N-dimethylnaphthalene-2-carboxamide |
| | ED15-45-01R | ED15-45 | 4-[4-[2-[3-[4-[4-(1-Adamantylmethylsulfonylcarbamoyl)phenyl]piperazine-1-carbonyl]-5-(trifluoromethyl)phenyl]ethynyl]phenyl]-1-hydroxy-N,N-dimethylnaphthalene-2-carboxamide |
| | ED15-46-01R | ED15-46 | 4-[5-[2-[3-[4-[4-(1-Adamantylmethylsulfonylcarbamoyl)phenyl]piperazine-1-carbonyl]-5-(trifluoromethyl)phenyl]ethynyl]pyridin-2-yl]-1-hydroxy-N,N-dimethylnaphthalene-2-carboxamide |
| | ED15-47-01R | ED15-47 | 4-[4-[2-[5-[4-[4-(1-Adamantylmethylsulfonylcarbamoyl)phenyl]piperazine-1-carbonyl]pyridin-3-yl]ethynyl]phenyl]-1-hydroxy-N,N-dimethylnaphthalene-2-carboxamide |
| mixED15-01R | ED15-48-01R | ED15-48 | 4-[5-[2-[5-[4-[4-(1-Adamantylmethylsulfonylcarbamoyl)phenyl]piperazine-1-carbonyl]pyridin-3-yl]ethynyl]pyridin-2-yl]-1-hydroxy-N,N-dimethylnaphthalene-2-carboxamide |
| | ED15-49-01R | ED15-49 | 4-[4-[2-[[4-[4-(1-Adamantylmethylsulfonylcarbamoyl)phenyl]piperazin-1-yl]methyl]phenyl]phenyl]-1-hydroxy-N,N-dimethylnaphthalene-2-carboxamide |
| | ED15-50-01R | ED15-50 | 4-[5-[2-[[4-[4-(1-Adamantylmethylsulfonylcarbamoyl)phenyl]piperazin-1-yl]methyl]phenyl]pyridin-2-yl]-1-hydroxy-N,N-dimethylnaphthalene-2-carboxamide |
| | ED15-51-01R | ED15-51 | 4-[4-[4-[[4-[4-(1-Adamantylmethylsulfonylcarbamoyl)phenyl]piperazin-1-yl]methyl]-2-methylphenyl]phenyl]-1-hydroxy-N,N-dimethylnaphthalene-2-carboxamide |
| | ED15-52-01R | ED15-52 | 4-[5-[4-[[4-[4-(1-Adamantylmethylsulfonylcarbamoyl)phenyl]piperazin-1-yl]methyl]-2-methylphenyl]pyridin-2-yl]-1-hydroxy-N,N-dimethylnaphthalene-2-carboxamide |
| | ED15-53-01R | ED15-53 | 4-[4-[4-[[4-[4-(1-Adamantylmethylsulfonylcarbamoyl)phenyl]piperazin-1-yl]methyl]-3-ethylphenyl]phenyl]-1-hydroxy-N,N-dimethylnaphthalene-2-carboxamide |
| | ED15-54-01R | ED15-54 | 4-[5-[4-[[4-[4-(1-Adamantylmethylsulfonylcarbamoyl)phenyl]piperazin-1-yl]methyl]-3-ethylphenyl]pyridin-2-yl]-1-hydroxy-N,N-dimethylnaphthalene-2-carboxamide |
| | ED15-55-01R | ED15-55 | 4-[4-[4-[[4-[4-(1-Adamantylmethylsulfonylcarbamoyl)phenyl]piperazin-1-yl]methyl]-2-fluorophenyl]phenyl]-1-hydroxy-N,N-dimethylnaphthalene-2-carboxamide |
| | ED15-56-01R | ED15-56 | 4-[5-[4-[[4-[4-(1-Adamantylmethylsulfonylcarbamoyl)phenyl]piperazin-1-yl]methyl]-2-fluorophenyl]pyridin-2-yl]-1-hydroxy-N,N-dimethylnaphthalene-2-carboxamide |
| | ED15-57-01R | ED15-57 | 4-[4-[4-[[4-[4-(1-Adamantylmethylsulfonylcarbamoyl)phenyl]piperazin-1-yl]methyl]-3-methoxyphenyl]phenyl]-1-hydroxy-N,N-dimethylnaphthalene-2-carboxamide |
| | ED15-58-01R | ED15-58 | 4-[5-[4-[[4-[4-(1-Adamantylmethylsulfonylcarbamoyl)phenyl]piperazin-1-yl]methyl]-3-methoxyphenyl]pyridin-2-yl]-1-hydroxy-N,N-dimethylnaphthalene-2-carboxamide |

| | | | |
|---|---|---|---|
| | ED15-59-01R | ED15-59 | 4-[4-[3-[[4-[4-(1-Adamantylmethylsulfonylcarbamoyl)phenyl]piperazin-1-yl]methyl]-5-ethoxyphenyl]phenyl]-1-hydroxy-N,N-dimethylnaphthalene-2-carboxamide |
| | ED15-60-01R | ED15-60 | 4-[5-[3-[[4-[4-(1-Adamantylmethylsulfonylcarbamoyl)phenyl]piperazin-1-yl]methyl]-5-ethoxyphenyl]pyridin-2-yl]-1-hydroxy-N,N-dimethylnaphthalene-2-carboxamide |
| | ED15-61-01R | ED15-61 | 4-[4-[3-[[4-[4-(1-Adamantylmethylsulfonylcarbamoyl)phenyl]piperazin-1-yl]methyl]-5-propan-2-yloxyphenyl]phenyl]-1-hydroxy-N,N-dimethylnaphthalene-2-carboxamide |
| | ED15-62-01R | ED15-62 | 4-[5-[3-[[4-[4-(1-Adamantylmethylsulfonylcarbamoyl)phenyl]piperazin-1-yl]methyl]-5-propan-2-yloxyphenyl]pyridin-2-yl]-1-hydroxy-N,N-dimethylnaphthalene-2-carboxamide |
| | ED15-63-01R | ED15-63 | 4-[4-[4-[[4-[4-(1-Adamantylmethylsulfonylcarbamoyl)phenyl]piperazin-1-yl]methyl]-3,5-dimethoxyphenyl]phenyl]-1-hydroxy-N,N-dimethylnaphthalene-2-carboxamide |
| mixED15-01R | ED15-64-01R | ED15-64 | 4-[5-[4-[[4-[4-(1-Adamantylmethylsulfonylcarbamoyl)phenyl]piperazin-1-yl]methyl]-3,5-dimethoxyphenyl]pyridin-2-yl]-1-hydroxy-N,N-dimethylnaphthalene-2-carboxamide |
| | ED15-65-01R | ED15-65 | 4-[4-[3-[[4-[4-(1-Adamantylmethylsulfonylcarbamoyl)phenyl]piperazin-1-yl]methyl]-5-(trifluoromethyl)phenyl]phenyl]-1-hydroxy-N,N-dimethylnaphthalene-2-carboxamide |
| | ED15-66-01R | ED15-66 | 4-[5-[3-[[4-[4-(1-Adamantylmethylsulfonylcarbamoyl)phenyl]piperazin-1-yl]methyl]-5-(trifluoromethyl)phenyl]pyridin-2-yl]-1-hydroxy-N,N-dimethylnaphthalene-2-carboxamide |
| | ED15-67-01R | ED15-67 | 4-[4-[3-[[4-[4-(1-Adamantylmethylsulfonylcarbamoyl)phenyl]piperazin-1-yl]methyl]-5-(trifluoromethoxy)phenyl]phenyl]-1-hydroxy-N,N-dimethylnaphthalene-2-carboxamide |
| | ED15-68-01R | ED15-68 | 4-[5-[3-[[4-[4-(1-Adamantylmethylsulfonylcarbamoyl)phenyl]piperazin-1-yl]methyl]-5-(trifluoromethoxy)phenyl]pyridin-2-yl]-1-hydroxy-N,N-dimethylnaphthalene-2-carboxamide |
| | ED15-69-01R | ED15-69 | 4-[4-[4-[[4-[4-(1-Adamantylmethylsulfonylcarbamoyl)phenyl]piperazin-1-yl]methyl]naphthalen-1-yl]phenyl]-1-hydroxy-N,N-dimethylnaphthalene-2-carboxamide |
| | ED15-70-01R | ED15-70 | 4-[5-[4-[[4-[4-(1-Adamantylmethylsulfonylcarbamoyl)phenyl]piperazin-1-yl]methyl]naphthalen-1-yl]pyridin-2-yl]-1-hydroxy-N,N-dimethylnaphthalene-2-carboxamide |
| | ED15-71-01R | ED15-71 | 4-[4-[5-[[4-[4-(1-Adamantylmethylsulfonylcarbamoyl)phenyl]piperazin-1-yl]methyl]thiophen-3-yl]phenyl]-1-hydroxy-N,N-dimethylnaphthalene-2-carboxamide |
| | ED15-72-01R | ED15-72 | 4-[5-[5-[[4-[4-(1-Adamantylmethylsulfonylcarbamoyl)phenyl]piperazin-1-yl]methyl]thiophen-3-yl]pyridin-2-yl]-1-hydroxy-N,N-dimethylnaphthalene-2-carboxamide |
| | ED15-73-01R | ED15-73 | 4-[4-[2-[2-[[4-[4-(1-Adamantylmethylsulfonylcarbamoyl)phenyl]piperazin-1-yl]methyl]phenyl]ethynyl]phenyl]-1-hydroxy-N,N-dimethylnaphthalene-2-carboxamide |
| | ED15-74-01R | ED15-74 | 4-[5-[2-[2-[[4-[4-(1-Adamantylmethylsulfonylcarbamoyl)phenyl]piperazin-1-yl]methyl]phenyl]ethynyl]pyridin-2-yl]-1-hydroxy-N,N-dimethylnaphthalene-2-carboxamide |
| | ED15-75-01R | ED15-75 | N-(1-Adamantylmethylsulfonyl)-6-[4-[[2-[4-[3-(dimethylcarbamoyl)-4-hydroxynaphthalen-1-yl]phenyl]phenyl]methyl]piperazin-1-yl]pyridazine-3-carboxamide |
| | ED15-76-01R | ED15-76 | N-(1-Adamantylmethylsulfonyl)-6-[4-[[2-[6-[3-(dimethylcarbamoyl)-4-hydroxynaphthalen-1-yl]pyridin-3-yl]phenyl]methyl]piperazin-1-yl]pyridazine-3-carboxamide |

| | | | |
|---|---|---|---|
| | ED15-77-01R | ED15-77 | N-(1-Adamantylmethylsulfonyl)-6-[4-[[4-[4-[3-(dimethylcarbamoyl)-4-hydroxynaphthalen-1-yl]phenyl]-3-methylphenyl]methyl]piperazin-1-yl]pyridazine-3-carboxamide |
| | ED15-78-01R | ED15-78 | N-(1-Adamantylmethylsulfonyl)-6-[4-[[4-[6-[3-(dimethylcarbamoyl)-4-hydroxynaphthalen-1-yl]pyridin-3-yl]-3-methylphenyl]methyl]piperazin-1-yl]pyridazine-3-carboxamide |
| | ED15-79-01R | ED15-79 | N-(1-Adamantylmethylsulfonyl)-6-[4-[[4-[4-[3-(dimethylcarbamoyl)-4-hydroxynaphthalen-1-yl]phenyl]-2-ethylphenyl]methyl]piperazin-1-yl]pyridazine-3-carboxamide |
| mixED15-01R | ED15-80-01R | ED15-80 | N-(1-Adamantylmethylsulfonyl)-6-[4-[[4-[6-[3-(dimethylcarbamoyl)-4-hydroxynaphthalen-1-yl]pyridin-3-yl]-2-ethylphenyl]methyl]piperazin-1-yl]pyridazine-3-carboxamide |
| | ED15-81-01R | ED15-81 | N-(1-Adamantylmethylsulfonyl)-6-[4-[[4-[4-[3-(dimethylcarbamoyl)-4-hydroxynaphthalen-1-yl]phenyl]-3-fluorophenyl]methyl]piperazin-1-yl]pyridazine-3-carboxamide |
| | ED15-82-01R | ED15-82 | N-(1-Adamantylmethylsulfonyl)-6-[4-[[4-[6-[3-(dimethylcarbamoyl)-4-hydroxynaphthalen-1-yl]pyridin-3-yl]-3-fluorophenyl]methyl]piperazin-1-yl]pyridazine-3-carboxamide |
| | ED15-83-01R | ED15-83 | N-(1-Adamantylmethylsulfonyl)-6-[4-[[4-[4-[3-(dimethylcarbamoyl)-4-hydroxynaphthalen-1-yl]phenyl]-2-methoxyphenyl]methyl]piperazin-1-yl]pyridazine-3-carboxamide |
| | ED15-84-01R | ED15-84 | N-(1-Adamantylmethylsulfonyl)-6-[4-[[4-[6-[3-(dimethylcarbamoyl)-4-hydroxynaphthalen-1-yl]pyridin-3-yl]-2-methoxyphenyl]methyl]piperazin-1-yl]pyridazine-3-carboxamide |
| | ED15-85-01R | ED15-85 | N-(1-Adamantylmethylsulfonyl)-6-[4-[[3-[4-[3-(dimethylcarbamoyl)-4-hydroxynaphthalen-1-yl]phenyl]-5-ethoxyphenyl]methyl]piperazin-1-yl]pyridazine-3-carboxamide |
| | ED15-86-01R | ED15-86 | N-(1-Adamantylmethylsulfonyl)-6-[4-[[3-[6-[3-(dimethylcarbamoyl)-4-hydroxynaphthalen-1-yl]pyridin-3-yl]-5-ethoxyphenyl]methyl]piperazin-1-yl]pyridazine-3-carboxamide |
| | ED15-87-01R | ED15-87 | N-(1-Adamantylmethylsulfonyl)-6-[4-[[3-[4-[3-(dimethylcarbamoyl)-4-hydroxynaphthalen-1-yl]phenyl]-5-propan-2-yloxyphenyl]methyl]piperazin-1-yl]pyridazine-3-carboxamide |
| | ED15-88-01R | ED15-88 | N-(1-Adamantylmethylsulfonyl)-6-[4-[[3-[6-[3-(dimethylcarbamoyl)-4-hydroxynaphthalen-1-yl]pyridin-3-yl]-5-propan-2-yloxyphenyl]methyl]piperazin-1-yl]pyridazine-3-carboxamide |
| | ED15-89-01R | ED15-89 | N-(1-Adamantylmethylsulfonyl)-6-[4-[[4-[4-[3-(dimethylcarbamoyl)-4-hydroxynaphthalen-1-yl]phenyl]-2,6-dimethoxyphenyl]methyl]piperazin-1-yl]pyridazine-3-carboxamide |
| | ED15-90-01R | ED15-90 | N-(1-Adamantylmethylsulfonyl)-6-[4-[[4-[6-[3-(dimethylcarbamoyl)-4-hydroxynaphthalen-1-yl]pyridin-3-yl]-2,6-dimethoxyphenyl]methyl]piperazin-1-yl]pyridazine-3-carboxamide |
| | ED15-91-01R | ED15-91 | N-(1-Adamantylmethylsulfonyl)-6-[4-[[3-[4-[3-(dimethylcarbamoyl)-4-hydroxynaphthalen-1-yl]phenyl]-5-(trifluoromethyl)phenyl]methyl]piperazin-1-yl]pyridazine-3-carboxamide |
| | ED15-92-01R | ED15-92 | N-(1-Adamantylmethylsulfonyl)-6-[4-[[3-[6-[3-(dimethylcarbamoyl)-4-hydroxynaphthalen-1-yl]pyridin-3-yl]-5-(trifluoromethyl)phenyl]methyl]piperazin-1-yl]pyridazine-3-carboxamide |
| | ED15-93-01R | ED15-93 | N-(1-Adamantylmethylsulfonyl)-6-[4-[[3-[4-[3-(dimethylcarbamoyl)-4-hydroxynaphthalen-1-yl]phenyl]-5-(trifluoromethoxy)phenyl]methyl]piperazin-1-yl]pyridazine-3-carboxamide |
| | ED15-94-01R | ED15-94 | N-(1-Adamantylmethylsulfonyl)-6-[4-[[3-[6-[3-(dimethylcarbamoyl)-4-hydroxynaphthalen-1-yl]pyridin-3-yl]-5-(trifluoromethoxy)phenyl]methyl]piperazin-1-yl]pyridazine-3-carboxamide |

| | | | |
|---|---|---|---|
| | ED15-95-01R | ED15-95 | N-(1-Adamantylmethylsulfonyl)-6-[4-[[4-[4-[3-(dimethylcarbamoyl)-4-hydroxynaphthalen-1-yl]phenyl]naphthalen-1-yl]methyl]piperazin-1-yl]pyridazine-3-carboxamide |
| mixED15-01R | ED15-96-01R | ED15-96 | N-(1-Adamantylmethylsulfonyl)-6-[4-[[4-[6-[3-(dimethylcarbamoyl)-4-hydroxynaphthalen-1-yl]pyridin-3-yl]naphthalen-1-yl]methyl]piperazin-1-yl]pyridazine-3-carboxamide |
| | ED15-97-01R | ED15-97 | N-(1-Adamantylmethylsulfonyl)-6-[4-[[4-[4-[3-(dimethylcarbamoyl)-4-hydroxynaphthalen-1-yl]phenyl]thiophen-2-yl]methyl]piperazin-1-yl]pyridazine-3-carboxamide |
| | ED15-98-01R | ED15-98 | N-(1-Adamantylmethylsulfonyl)-6-[4-[[4-[6-[3-(dimethylcarbamoyl)-4-hydroxynaphthalen-1-yl]pyridin-3-yl]thiophen-2-yl]methyl]piperazin-1-yl]pyridazine-3-carboxamide |
| | ED15-99-01R | ED15-99 | N-(1-Adamantylmethylsulfonyl)-6-[4-[[2-[2-[4-[3-(dimethylcarbamoyl)-4-hydroxynaphthalen-1-yl]phenyl]ethynyl]phenyl]methyl]piperazin-1-yl]pyridazine-3-carboxamide |
| | ED15-100-01R | ED15-100 | N-(1-Adamantylmethylsulfonyl)-6-[4-[[2-[2-[6-[3-(dimethylcarbamoyl)-4-hydroxynaphthalen-1-yl]pyridin-3-yl]ethynyl]phenyl]methyl]piperazin-1-yl]pyridazine-3-carboxamide |

[Table 115]

[Table ED16]

| No. of mixture | No. of compound that may be contained | Compound No. after cleavage | Compound name |
|---|---|---|---|
| mixED16-01R | ED16-01-01R | ED16-01 | 1-Hydroxy-N,N-dimethyl-4-[4-[4-[rac-(1R)-1-[4-[4-[[3-nitro-4-(2-phenylsulfanylethylamino)phenyl]sulfonylcarbamoyl]phenyl]piperazin-1-yl]ethyl]phenyl]phenyl]naphthalene-2-carboxamide |
| | ED16-02-01R | ED16-02 | 1-Hydroxy-N,N-dimethyl-4-[5-[4-[1-[4-[4-[[3-nitro-4-(2-phenylsulfanylethylamino)phenyl]sulfonylcarbamoyl]phenyl]piperazin-1-yl]ethyl]phenyl]pyridin-2-yl]naphthalene-2-carboxamide |
| | ED16-03-01R | ED16-03 | 4-[4-[3-Fluoro-4-[1-[4-[4-[[3-nitro-4-(2-phenylsulfanylethylamino)phenyl]sulfonylcarbamoyl]phenyl]piperazin-1-yl]ethyl]phenyl]phenyl]-1-hydroxy-N,N-dimethylnaphthalene-2-carboxamide |
| | ED16-04-01R | ED16-04 | 4-[5-[3-Fluoro-4-[1-[4-[4-[[3-nitro-4-(2-phenylsulfanylethylamino)phenyl]sulfonylcarbamoyl]phenyl]piperazin-1-yl]ethyl]phenyl]pyridin-2-yl]-1-hydroxy-N,N-dimethylnaphthalene-2-carboxamide |
| | ED16-05-01R | ED16-05 | 6-[4-[4-[4-[3-(Dimethylcarbamoyl)-4-hydroxynaphthalen-1-yl]phenyl]-3-methylbenzoyl]piperazin-1-yl]-N-[3-nitro-4-(2-phenylsulfanylethylamino)phenyl]sulfonylpyridazine-3-carboxamide |
| | ED16-06-01R | ED16-06 | 6-[4-[4-[6-[3-(Dimethylcarbamoyl)-4-hydroxynaphthalen-1-yl]pyridin-3-yl]-3-methylbenzoyl]piperazin-1-yl]-N-[3-nitro-4-(2-phenylsulfanylethylamino)phenyl]sulfonylpyridazine-3-carboxamide |
| | ED16-07-01R | ED16-07 | 6-[4-[4-[4-[3-(Dimethylcarbamoyl)-4-hydroxynaphthalen-1-yl]phenyl]-3-fluorobenzoyl]piperazin-1-yl]-N-[3-nitro-4-(2-phenylsulfanylethylamino)phenyl]sulfonylpyridazine-3-carboxamide |
| | ED16-08-01R | ED16-08 | 6-[4-[4-[6-[3-(Dimethylcarbamoyl)-4-hydroxynaphthalen-1-yl]pyridin-3-yl]-3-fluorobenzoyl]piperazin-1-yl]-N-[3-nitro-4-(2-phenylsulfanylethylamino)phenyl]sulfonylpyridazine-3-carboxamide |

| | | | |
|---|---|---|---|
| mixED16-01R | ED16-09-01R | ED16-09 | 6-[4-[3-[4-[3-(Dimethylcarbamoyl)-4-hydroxynaphthalen-1-yl]phenyl]-5-(trifluoromethyl)benzoyl]piperazin-1-yl]-N-[3-nitro-4-(2-phenylsulfanylethylamino)phenyl]sulfonylpyridazine-3-carboxamide |
| | ED16-10-01R | ED16-10 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-[3-nitro-4-(2-phenylsulfanylethylamino)phenyl]sulfonylpyridazine-3-carboxamide |
| | ED16-11-01R | ED16-11 | 6-[4-[3-tert-Butyl-5-[4-[3-(dimethylcarbamoyl)-4-hydroxynaphthalen-1-yl]phenyl]benzoyl]piperazin-1-yl]-N-[3-nitro-4-(2-phenylsulfanylethylamino)phenyl]sulfonylpyridazine-3-carboxamide |
| | ED16-12-01R | ED16-12 | 6-[4-[3-tert-Butyl-5-[6-[3-(dimethylcarbamoyl)-4-hydroxynaphthalen-1-yl]pyridin-3-yl]benzoyl]piperazin-1-yl]-N-[3-nitro-4-(2-phenylsulfanylethylamino)phenyl]sulfonylpyridazine-3-carboxamide |
| | ED16-13-01R | ED16-13 | 6-[4-[4-[4-[3-(Dimethylcarbamoyl)-4-hydroxynaphthalen-1-yl]phenyl]naphthalene-1-carbonyl]piperazin-1-yl]-N-[3-nitro-4-(2-phenylsulfanylethylamino)phenyl]sulfonylpyridazine-3-carboxamide |
| | ED16-14-01R | ED16-14 | 6-[4-[4-[6-[3-(Dimethylcarbamoyl)-4-hydroxynaphthalen-1-yl]pyridin-3-yl]naphthalene-1-carbonyl]piperazin-1-yl]-N-[3-nitro-4-(2-phenylsulfanylethylamino)phenyl]sulfonylpyridazine-3-carboxamide |
| | ED16-15-01R | ED16-15 | 6-[4-[3-[2-[4-[3-(Dimethylcarbamoyl)-4-hydroxynaphthalen-1-yl]phenyl]phenyl]propanoyl]piperazin-1-yl]-N-[3-nitro-4-(2-phenylsulfanylethylamino)phenyl]sulfonylpyridazine-3-carboxamide |
| | ED16-16-01R | ED16-16 | 6-[4-[3-[2-[6-[3-(Dimethylcarbamoyl)-4-hydroxynaphthalen-1-yl]pyridin-3-yl]phenyl]propanoyl]piperazin-1-yl]-N-[3-nitro-4-(2-phenylsulfanylethylamino)phenyl]sulfonylpyridazine-3-carboxamide |
| | ED16-17-01R | ED16-17 | 6-[4-[4-[4-[3-(Dimethylcarbamoyl)-4-hydroxynaphthalen-1-yl]phenyl]thiophene-2-carbonyl]piperazin-1-yl]-N-[3-nitro-4-(2-phenylsulfanylethylamino)phenyl]sulfonylpyridazine-3-carboxamide |
| | ED16-18-01R | ED16-18 | 6-[4-[4-[6-[3-(Dimethylcarbamoyl)-4-hydroxynaphthalen-1-yl]pyridin-3-yl]thiophene-2-carbonyl]piperazin-1-yl]-N-[3-nitro-4-(2-phenylsulfanylethylamino)phenyl]sulfonylpyridazine-3-carboxamide |
| | ED16-19-01R | ED16-19 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| | ED16-20-01R | ED16-20 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| | ED16-21-01R | ED16-21 | 6-[4-[2-[2-[4-[3-(Dimethylcarbamoyl)-4-hydroxynaphthalen-1-yl]phenyl]ethynyl]benzoyl]piperazin-1-yl]-N-[3-nitro-4-(2-phenylsulfanylethylamino)phenyl]sulfonylpyridazine-3-carboxamide |
| | ED16-22-01R | ED16-22 | 6-[4-[2-[2-[6-[3-(Dimethylcarbamoyl)-4-hydroxynaphthalen-1-yl]pyridin-3-yl]ethynyl]benzoyl]piperazin-1-yl]-N-[3-nitro-4-(2-phenylsulfanylethylamino)phenyl]sulfonylpyridazine-3-carboxamide |
| | ED16-23-01R | ED16-23 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-[3-nitro-4-(2-phenylsulfanylethylamino)phenyl]sulfonylpyridazine-3-carboxamide |
| | ED16-24-01R | ED16-24 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-[3-nitro-4-(2-phenylsulfanylethylamino)phenyl]sulfonylpyridazine-3-carboxamide |

| | | | |
|---|---|---|---|
| mixED16-01R | ED16-25-01R | ED16-25 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-[3-nitro-4-(2-phenylsulfanylethylamino)phenyl]sulfonylpyridazine-3-carboxamide |
| | ED16-26-01R | ED16-26 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-[3-nitro-4-(2-phenylsulfanylethylamino)phenyl]sulfonylpyridazine-3-carboxamide |
| | ED16-27-01R | ED16-27 | 1-Hydroxy-N,N-dimethyl-4-[4-[2-methyl-4-[4-[4-[[3-nitro-4-(2-phenylsulfanylethylamino)phenyl]sulfonylcarbamoyl]phenyl]piperazine-1-carbonyl]phenyl]phenyl]naphthalene-2-carboxamide |
| | ED16-28-01R | ED16-28 | 1-Hydroxy-N,N-dimethyl-4-[5-[2-methyl-4-[4-[4-[[3-nitro-4-(2-phenylsulfanylethylamino)phenyl]sulfonylcarbamoyl]phenyl]piperazine-1-carbonyl]phenyl]pyridin-2-yl]naphthalene-2-carboxamide |
| | ED16-29-01R | ED16-29 | 4-[4-[2-Fluoro-4-[4-[4-[[3-nitro-4-(2-phenylsulfanylethylamino)phenyl]sulfonylcarbamoyl]phenyl]piperazine-1-carbonyl]phenyl]phenyl]-1-hydroxy-N,N-dimethylnaphthalene-2-carboxamide |
| | ED16-30-01R | ED16-30 | 4-[5-[2-Fluoro-4-[4-[4-[[3-nitro-4-(2-phenylsulfanylethylamino)phenyl]sulfonylcarbamoyl]phenyl]piperazine-1-carbonyl]phenyl]pyridin-2-yl]-1-hydroxy-N,N-dimethylnaphthalene-2-carboxamide |
| | ED16-31-01R | ED16-31 | 1-Hydroxy-N,N-dimethyl-4-[4-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]phenyl]naphthalene-2-carboxamide |
| | ED16-32-01R | ED16-32 | 1-Hydroxy-N,N-dimethyl-4-[5-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]pyridin-2-yl]naphthalene-2-carboxamide |
| | ED16-33-01R | ED16-33 | 4-[4-[3-tert-Butyl-5-[4-[4-[[3-nitro-4-(2-phenylsulfanylethylamino)phenyl]sulfonylcarbamoyl]phenyl]piperazine-1-carbonyl]phenyl]phenyl]-1-hydroxy-N,N-dimethylnaphthalene-2-carboxamide |
| | ED16-34-01R | ED16-34 | 4-[5-[3-tert-Butyl-5-[4-[4-[[3-nitro-4-(2-phenylsulfanylethylamino)phenyl]sulfonylcarbamoyl]phenyl]piperazine-1-carbonyl]phenyl]pyridin-2-yl]-1-hydroxy-N,N-dimethylnaphthalene-2-carboxamide |
| | ED16-35-01R | ED16-35 | 1-Hydroxy-N,N-dimethyl-4-[4-[4-[4-[4-[[3-nitro-4-(2-phenylsulfanylethylamino)phenyl]sulfonylcarbamoyl]phenyl]piperazine-1-carbonyl]naphthalen-1-yl]phenyl]naphthalene-2-carboxamide |
| | ED16-36-01R | ED16-36 | 1-Hydroxy-N,N-dimethyl-4-[5-[4-[4-[4-[[3-nitro-4-(2-phenylsulfanylethylamino)phenyl]sulfonylcarbamoyl]phenyl]piperazine-1-carbonyl]naphthalen-1-yl]pyridin-2-yl]naphthalene-2-carboxamide |
| | ED16-37-01R | ED16-37 | 1-Hydroxy-N,N-dimethyl-4-[4-[2-[3-[4-[4-[[3-nitro-4-(2-phenylsulfanylethylamino)phenyl]sulfonylcarbamoyl]phenyl]piperazin-1-yl]-3-oxopropyl]phenyl]phenyl]naphthalene-2-carboxamide |
| | ED16-38-01R | ED16-38 | 1-Hydroxy-N,N-dimethyl-4-[5-[2-[3-[4-[4-[[3-nitro-4-(2-phenylsulfanylethylamino)phenyl]sulfonylcarbamoyl]phenyl]piperazin-1-yl]-3-oxopropyl]phenyl]pyridin-2-yl]naphthalene-2-carboxamide |
| | ED16-39-01R | ED16-39 | 1-Hydroxy-N,N-dimethyl-4-[4-[5-[4-[4-[[3-nitro-4-(2-phenylsulfanylethylamino)phenyl]sulfonylcarbamoyl]phenyl]piperazine-1-carbonyl]thiophen-3-yl]phenyl]naphthalene-2-carboxamide |

| | | | |
|---|---|---|---|
| | ED16-40-01R | ED16-40 | 1-Hydroxy-N,N-dimethyl-4-[5-[5-[4-[4-[[3-nitro-4-(2-phenylsulfanylethylamino)phenyl]sulfonylcarbamoyl]phenyl]piperazine-1-carbonyl]thiophen-3-yl]pyridin-2-yl]naphthalene-2-carboxamide |
| mixED16-01R | ED16-41-01R | ED16-41 | 1-Hydroxy-N,N-dimethyl-4-[4-[1-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]pyrazol-4-yl]phenyl]naphthalene-2-carboxamide |
| | ED16-42-01R | ED16-42 | 1-Hydroxy-N,N-dimethyl-4-[5-[1-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]pyrazol-4-yl]pyridin-2-yl]naphthalene-2-carboxamide |
| | ED16-43-01R | ED16-43 | 1-Hydroxy-N,N-dimethyl-4-[4-[2-[2-[4-[4-[[3-nitro-4-(2-phenylsulfanylethylamino)phenyl]sulfonylcarbamoyl]phenyl]piperazine-1-carbonyl]phenyl]ethynyl]phenyl]naphthalene-2-carboxamide |
| | ED16-44-01R | ED16-44 | 1-Hydroxy-N,N-dimethyl-4-[5-[2-[2-[4-[4-[[3-nitro-4-(2-phenylsulfanylethylamino)phenyl]sulfonylcarbamoyl]phenyl]piperazine-1-carbonyl]phenyl]ethynyl]pyridin-2-yl]naphthalene-2-carboxamide |
| | ED16-45-01R | ED16-45 | 1-Hydroxy-N,N-dimethyl-4-[4-[2-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]ethynyl]phenyl]naphthalene-2-carboxamide |
| | ED16-46-01R | ED16-46 | 1-Hydroxy-N,N-dimethyl-4-[5-[2-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]ethynyl]pyridin-2-yl]naphthalene-2-carboxamide |
| | ED16-47-01R | ED16-47 | 1-Hydroxy-N,N-dimethyl-4-[4-[2-[5-[4-[4-[[3-nitro-4-(2-phenylsulfanylethylamino)phenyl]sulfonylcarbamoyl]phenyl]piperazine-1-carbonyl]pyridin-3-yl]ethynyl]phenyl]naphthalene-2-carboxamide |
| | ED16-48-01R | ED16-48 | 1-Hydroxy-N,N-dimethyl-4-[5-[2-[5-[4-[4-[[3-nitro-4-(2-phenylsulfanylethylamino)phenyl]sulfonylcarbamoyl]phenyl]piperazine-1-carbonyl]pyridin-3-yl]ethynyl]pyridin-2-yl]naphthalene-2-carboxamide |
| | ED16-49-01R | ED16-49 | 1-Hydroxy-N,N-dimethyl-4-[4-[2-[[4-[4-[[3-nitro-4-(2-phenylsulfanylethylamino)phenyl]sulfonylcarbamoyl]phenyl]piperazin-1-yl]methyl]phenyl]phenyl]naphthalene-2-carboxamide |
| | ED16-50-01R | ED16-50 | 1-Hydroxy-N,N-dimethyl-4-[5-[2-[[4-[4-[[3-nitro-4-(2-phenylsulfanylethylamino)phenyl]sulfonylcarbamoyl]phenyl]piperazin-1-yl]methyl]phenyl]pyridin-2-yl]naphthalene-2-carboxamide |
| | ED16-51-01R | ED16-51 | 1-Hydroxy-N,N-dimethyl-4-[4-[2-methyl-4-[[4-[4-[[3-nitro-4-(2-phenylsulfanylethylamino)phenyl]sulfonylcarbamoyl]phenyl]piperazin-1-yl]methyl]phenyl]phenyl]naphthalene-2-carboxamide |
| | ED16-52-01R | ED16-52 | 1-Hydroxy-N,N-dimethyl-4-[5-[2-methyl-4-[[4-[4-[[3-nitro-4-(2-phenylsulfanylethylamino)phenyl]sulfonylcarbamoyl]phenyl]piperazin-1-yl]methyl]phenyl]pyridin-2-yl]naphthalene-2-carboxamide |
| | ED16-53-01R | ED16-53 | 4-[4-[3-Ethyl-4-[[4-[4-[[3-nitro-4-(2-phenylsulfanylethylamino)phenyl]sulfonylcarbamoyl]phenyl]piperazin-1-yl]methyl]phenyl]phenyl]-1-hydroxy-N,N-dimethylnaphthalene-2-carboxamide |
| | ED16-54-01R | ED16-54 | 4-[5-[3-Ethyl-4-[[4-[4-[[3-nitro-4-(2-phenylsulfanylethylamino)phenyl]sulfonylcarbamoyl]phenyl]piperazin-1-yl]methyl]phenyl]pyridin-2-yl]-1-hydroxy-N,N-dimethylnaphthalene-2-carboxamide |

| | | | |
|---|---|---|---|
| | ED16-55-01R | ED16-55 | 4-[4-[2-Fluoro-4-[[4-[4-[[3-nitro-4-(2-phenylsulfanylethylamino)phenyl]sulfonylcarbamoyl]phenyl]piperazin-1-yl]methyl]phenyl]phenyl]-1-hydroxy-N,N-dimethylnaphthalene-2-carboxamide |
| | ED16-56-01R | ED16-56 | 4-[5-[2-Fluoro-4-[[4-[4-[[3-nitro-4-(2-phenylsulfanylethylamino)phenyl]sulfonylcarbamoyl]phenyl]piperazin-1-yl]methyl]phenyl]pyridin-2-yl]-1-hydroxy-N,N-dimethylnaphthalene-2-carboxamide |
| mixED16-01R | ED16-57-01R | ED16-57 | 1-Hydroxy-4-[4-[3-methoxy-4-[[4-[4-[[3-nitro-4-(2-phenylsulfanylethylamino)phenyl]sulfonylcarbamoyl]phenyl]piperazin-1-yl]methyl]phenyl]phenyl]-N,N-dimethylnaphthalene-2-carboxamide |
| | ED16-58-01R | ED16-58 | 1-Hydroxy-4-[5-[3-methoxy-4-[[4-[4-[[3-nitro-4-(2-phenylsulfanylethylamino)phenyl]sulfonylcarbamoyl]phenyl]piperazin-1-yl]methyl]phenyl]pyridin-2-yl]-N,N-dimethylnaphthalene-2-carboxamide |
| | ED16-59-01R | ED16-59 | 4-[4-[3-Ethoxy-5-[[4-[4-[[3-nitro-4-(2-phenylsulfanylethylamino)phenyl]sulfonylcarbamoyl]phenyl]piperazin-1-yl]methyl]phenyl]phenyl]-1-hydroxy-N,N-dimethylnaphthalene-2-carboxamide |
| | ED16-60-01R | ED16-60 | 4-[5-[3-Ethoxy-5-[[4-[4-[[3-nitro-4-(2-phenylsulfanylethylamino)phenyl]sulfonylcarbamoyl]phenyl]piperazin-1-yl]methyl]phenyl]pyridin-2-yl]-1-hydroxy-N,N-dimethylnaphthalene-2-carboxamide |
| | ED16-61-01R | ED16-61 | 1-Hydroxy-N,N-dimethyl-4-[4-[3-[[4-[4-[[3-nitro-4-(2-phenylsulfanylethylamino)phenyl]sulfonylcarbamoyl]phenyl]piperazin-1-yl]methyl]-5-propan-2-yloxyphenyl]phenyl]naphthalene-2-carboxamide |
| | ED16-62-01R | ED16-62 | 1-Hydroxy-N,N-dimethyl-4-[5-[3-[[4-[4-[[3-nitro-4-(2-phenylsulfanylethylamino)phenyl]sulfonylcarbamoyl]phenyl]piperazin-1-yl]methyl]-5-propan-2-yloxyphenyl]pyridin-2-yl]naphthalene-2-carboxamide |
| | ED16-63-01R | ED16-63 | 4-[4-[3,5-Dimethoxy-4-[[4-[4-[[3-nitro-4-(2-phenylsulfanylethylamino)phenyl]sulfonylcarbamoyl]phenyl]piperazin-1-yl]methyl]phenyl]phenyl]-1-hydroxy-N,N-dimethylnaphthalene-2-carboxamide |
| | ED16-64-01R | ED16-64 | 4-[5-[3,5-Dimethoxy-4-[[4-[4-[[3-nitro-4-(2-phenylsulfanylethylamino)phenyl]sulfonylcarbamoyl]phenyl]piperazin-1-yl]methyl]phenyl]pyridin-2-yl]-1-hydroxy-N,N-dimethylnaphthalene-2-carboxamide |
| | ED16-65-01R | ED16-65 | 1-Hydroxy-N,N-dimethyl-4-[4-[3-[[4-[4-[[3-nitro-4-(2-phenylsulfanylethylamino)phenyl]sulfonylcarbamoyl]phenyl]piperazin-1-yl]methyl]-5-(trifluoromethyl)phenyl]phenyl]naphthalene-2-carboxamide |
| | ED16-66-01R | ED16-66 | 1-Hydroxy-N,N-dimethyl-4-[5-[3-[[4-[4-[[3-nitro-4-(2-phenylsulfanylethylamino)phenyl]sulfonylcarbamoyl]phenyl]piperazin-1-yl]methyl]-5-(trifluoromethyl)phenyl]pyridin-2-yl]naphthalene-2-carboxamide |
| | ED16-67-01R | ED16-67 | 1-Hydroxy-N,N-dimethyl-4-[4-[3-[[4-[4-[[3-nitro-4-(2-phenylsulfanylethylamino)phenyl]sulfonylcarbamoyl]phenyl]piperazin-1-yl]methyl]-5-(trifluoromethoxy)phenyl]phenyl]naphthalene-2-carboxamide |
| | ED16-68-01R | ED16-68 | 1-Hydroxy-N,N-dimethyl-4-[5-[3-[[4-[4-[[3-nitro-4-(2-phenylsulfanylethylamino)phenyl]sulfonylcarbamoyl]phenyl]piperazin-1-yl]methyl]-5-(trifluoromethoxy)phenyl]pyridin-2-yl]naphthalene-2-carboxamide |
| | ED16-69-01R | ED16-69 | 1-Hydroxy-N,N-dimethyl-4-[4-[4-[[4-[4-[[3-nitro-4-(2-phenylsulfanylethylamino)phenyl]sulfonylcarbamoyl]phenyl]piperazin-1-yl]methyl]naphthalen-1-yl]phenyl]naphthalene-2-carboxamide |

| | | | |
|---|---|---|---|
| | ED16-70-01R | ED16-70 | 1-Hydroxy-N,N-dimethyl-4-[5-[4-[[4-[4-[[3-nitro-4-(2-phenylsulfanylethylamino)phenyl]sulfonylcarbamoyl]phenyl]piperazin-1-yl]methyl]naphthalen-1-yl]pyridin-2-yl]naphthalene-2-carboxamide |
| | ED16-71-01R | ED16-71 | 1-Hydroxy-N,N-dimethyl-4-[4-[5-[[4-[4-[[3-nitro-4-(2-phenylsulfanylethylamino)phenyl]sulfonylcarbamoyl]phenyl]piperazin-1-yl]methyl]thiophen-3-yl]phenyl]naphthalene-2-carboxamide |
| | ED16-72-01R | ED16-72 | 1-Hydroxy-N,N-dimethyl-4-[5-[5-[[4-[4-[[3-nitro-4-(2-phenylsulfanylethylamino)phenyl]sulfonylcarbamoyl]phenyl]piperazin-1-yl]methyl]thiophen-3-yl]pyridin-2-yl]naphthalene-2-carboxamide |
| mixED16-01R | ED16-73-01R | ED16-73 | 1-Hydroxy-N,N-dimethyl-4-[4-[2-[2-[[4-[4-[[3-nitro-4-(2-phenylsulfanylethylamino)phenyl]sulfonylcarbamoyl]phenyl]piperazin-1-yl]methyl]phenyl]ethynyl]phenyl]naphthalene-2-carboxamide |
| | ED16-74-01R | ED16-74 | 1-Hydroxy-N,N-dimethyl-4-[5-[2-[2-[[4-[4-[[3-nitro-4-(2-phenylsulfanylethylamino)phenyl]sulfonylcarbamoyl]phenyl]piperazin-1-yl]methyl]phenyl]ethynyl]pyridin-2-yl]naphthalene-2-carboxamide |
| | ED16-75-01R | ED16-75 | 6-[4-[[2-[4-[3-(Dimethylcarbamoyl)-4-hydroxynaphthalen-1-yl]phenyl]phenyl]methyl]piperazin-1-yl]-N-[3-nitro-4-(2-phenylsulfanylethylamino)phenyl]sulfonylpyridazine-3-carboxamide |
| | ED16-76-01R | ED16-76 | 6-[4-[[2-[6-[3-(Dimethylcarbamoyl)-4-hydroxynaphthalen-1-yl]pyridin-3-yl]phenyl]methyl]piperazin-1-yl]-N-[3-nitro-4-(2-phenylsulfanylethylamino)phenyl]sulfonylpyridazine-3-carboxamide |
| | ED16-77-01R | ED16-77 | 6-[4-[[4-[4-[3-(Dimethylcarbamoyl)-4-hydroxynaphthalen-1-yl]phenyl]-3-methylphenyl]methyl]piperazin-1-yl]-N-[3-nitro-4-(2-phenylsulfanylethylamino)phenyl]sulfonylpyridazine-3-carboxamide |
| | ED16-78-01R | ED16-78 | 6-[4-[[4-[6-[3-(Dimethylcarbamoyl)-4-hydroxynaphthalen-1-yl]pyridin-3-yl]-3-methylphenyl]methyl]piperazin-1-yl]-N-[3-nitro-4-(2-phenylsulfanylethylamino)phenyl]sulfonylpyridazine-3-carboxamide |
| | ED16-79-01R | ED16-79 | 6-[4-[[4-[4-[3-(Dimethylcarbamoyl)-4-hydroxynaphthalen-1-yl]phenyl]-2-ethylphenyl]methyl]piperazin-1-yl]-N-[3-nitro-4-(2-phenylsulfanylethylamino)phenyl]sulfonylpyridazine-3-carboxamide |
| | ED16-80-01R | ED16-80 | 6-[4-[[4-[6-[3-(Dimethylcarbamoyl)-4-hydroxynaphthalen-1-yl]pyridin-3-yl]-2-ethylphenyl]methyl]piperazin-1-yl]-N-[3-nitro-4-(2-phenylsulfanylethylamino)phenyl]sulfonylpyridazine-3-carboxamide |
| | ED16-81-01R | ED16-81 | 6-[4-[[4-[4-[3-(Dimethylcarbamoyl)-4-hydroxynaphthalen-1-yl]phenyl]-3-fluorophenyl]methyl]piperazin-1-yl]-N-[3-nitro-4-(2-phenylsulfanylethylamino)phenyl]sulfonylpyridazine-3-carboxamide |
| | ED16-82-01R | ED16-82 | 6-[4-[[4-[6-[3-(Dimethylcarbamoyl)-4-hydroxynaphthalen-1-yl]pyridin-3-yl]-3-fluorophenyl]methyl]piperazin-1-yl]-N-[3-nitro-4-(2-phenylsulfanylethylamino)phenyl]sulfonylpyridazine-3-carboxamide |
| | ED16-83-01R | ED16-83 | 6-[4-[[4-[4-[3-(Dimethylcarbamoyl)-4-hydroxynaphthalen-1-yl]phenyl]-2-methoxyphenyl]methyl]piperazin-1-yl]-N-[3-nitro-4-(2-phenylsulfanylethylamino)phenyl]sulfonylpyridazine-3-carboxamide |
| | ED16-84-01R | ED16-84 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-[3-nitro-4-(2-phenylsulfanylethylamino)phenyl]sulfonylpyridazine-3-carboxamide |
| | ED16-85-01R | ED16-85 | 6-[4-[[3-[4-[3-(Dimethylcarbamoyl)-4-hydroxynaphthalen-1-yl]phenyl]-5-ethoxyphenyl]methyl]piperazin-1-yl]-N-[3-nitro-4-(2-phenylsulfanylethylamino)phenyl]sulfonylpyridazine-3-carboxamide |

| | | | |
|---|---|---|---|
| | ED16-86-01R | ED16-86 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-[3-nitro-4-(2-phenylsulfanylethylamino)phenyl]sulfonylpyridazine-3-carboxamide |
| | ED16-87-01R | ED16-87 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-[3-nitro-4-(2-phenylsulfanylethylamino)phenyl]sulfonylpyridazine-3-carboxamide |
| | ED16-88-01R | ED16-88 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-[3-nitro-4-(2-phenylsulfanylethylamino)phenyl]sulfonylpyridazine-3-carboxamide |
| mixED16-01R | ED16-89-01R | ED16-89 | 6-[4-[[4-[4-[3-(Dimethylcarbamoyl)-4-hydroxynaphthalen-1-yl]phenyl]-2,6-dimethoxyphenyl]methyl]piperazin-1-yl]-N-[3-nitro-4-(2-phenylsulfanylethylamino)phenyl]sulfonylpyridazine-3-carboxamide |
| | ED16-90-01R | ED16-90 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-[3-nitro-4-(2-phenylsulfanylethylamino)phenyl]sulfonylpyridazine-3-carboxamide |
| | ED16-91-01R | ED16-91 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-[3-nitro-4-(2-phenylsulfanylethylamino)phenyl]sulfonylpyridazine-3-carboxamide |
| | ED16-92-01R | ED16-92 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-[3-nitro-4-(2-phenylsulfanylethylamino)phenyl]sulfonylpyridazine-3-carboxamide |
| | ED16-93-01R | ED16-93 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-[3-nitro-4-(2-phenylsulfanylethylamino)phenyl]sulfonylpyridazine-3-carboxamide |
| | ED16-94-01R | ED16-94 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-[3-nitro-4-(2-phenylsulfanylethylamino)phenyl]sulfonylpyridazine-3-carboxamide |
| | ED16-95-01R | ED16-95 | 6-[4-[[4-[4-[3-(Dimethylcarbamoyl)-4-hydroxynaphthalen-1-yl]phenyl]naphthalen-1-yl]methyl]piperazin-1-yl]-N-[3-nitro-4-(2-phenylsulfanylethylamino)phenyl]sulfonylpyridazine-3-carboxamide |
| | ED16-96-01R | ED16-96 | 6-[4-[[4-[6-[3-(Dimethylcarbamoyl)-4-hydroxynaphthalen-1-yl]pyridin-3-yl]naphthalen-1-yl]methyl]piperazin-1-yl]-N-[3-nitro-4-(2-phenylsulfanylethylamino)phenyl]sulfonylpyridazine-3-carboxamide |
| | ED16-97-01R | ED16-97 | 6-[4-[[4-[4-[3-(Dimethylcarbamoyl)-4-hydroxynaphthalen-1-yl]phenyl]thiophen-2-yl]methyl]piperazin-1-yl]-N-[3-nitro-4-(2-phenylsulfanylethylamino)phenyl]sulfonylpyridazine-3-carboxamide |
| | ED16-98-01R | ED16-98 | 6-[4-[[4-[6-[3-(Dimethylcarbamoyl)-4-hydroxynaphthalen-1-yl]pyridin-3-yl]thiophen-2-yl]methyl]piperazin-1-yl]-N-[3-nitro-4-(2-phenylsulfanylethylamino)phenyl]sulfonylpyridazine-3-carboxamide |
| | ED16-99-01R | ED16-99 | 6-[4-[[2-[2-[4-[3-(Dimethylcarbamoyl)-4-hydroxynaphthalen-1-yl]phenyl]ethynyl]phenyl]methyl]piperazin-1-yl]-N-[3-nitro-4-(2-phenylsulfanylethylamino)phenyl]sulfonylpyridazine-3-carboxamide |
| | ED16-100-01R | ED16-100 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-[3-nitro-4-(2-phenylsulfanylethylamino)phenyl]sulfonylpyridazine-3-carboxamide |

Example 3-7: Cleavage from solid phase

A cleavage experiment from the solid phase was carried out, and each mixture was analyzed by LC/MS to evaluate the synthesized library.

Example 3-7-1: Synthesis of mixED05 (A4J01 to A4J100)

[Formula 523]

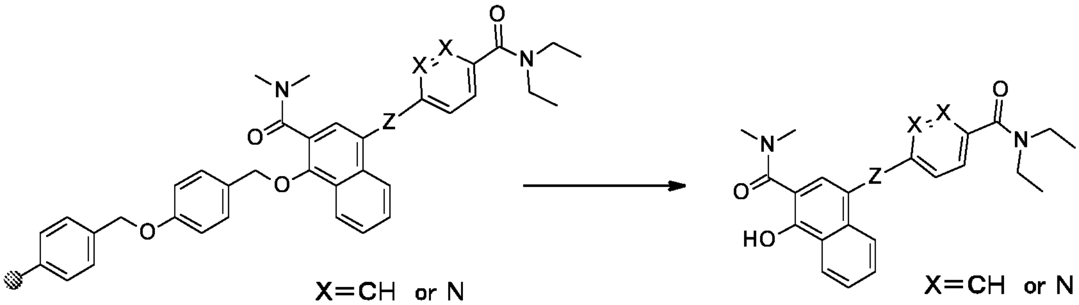

X=CH or N

mixED05-01R

X=CH or N

mixED05

Under a nitrogen atmosphere, mixture mixED05-01R (mixture that could contain 100 compounds, 43.4 mg, loading rate: 0.38 mmol/g, 16.7 μmol) shown in Table ED05 and 1,2,3-trimethylbenzene/DCM (0.056 M, 1085 μL) were added to a 2 mL glass vial and shaken at room temperature for 1 hour. TFA (109 μL, 1.41 mmol) was added thereto, and the mixture was shaken at room temperature for 3 hours. The suspension of the reaction solution and the resin was transferred to a syringe with a filter using a DCM solution, filtered under nitrogen pressure, and discharged into DMI (0.5 mL). This operation was repeated twice with DCM (0.5 mL), twice with DMI (0.5 mL), twice with DCM (0.5 mL), and twice with DMI (0.5 mL). The solvent in the filtrate was distilled off under reduced pressure by Genevac (40°C, reduced pressure at 50-200 mbar until the completion of distilling off of a low-boiling solvent was detected, and then, 40°C, <0.5 mbar for 16 hours) to obtain the title mixture mixED05 shown in Table EE01.

[1482] [Table 116]

[Table EE01]

| Table EE01 | | | | |
|---|---|---|---|---|
| Mixture No. | No. of compound that may be contained | Compound name | Compositional formula | Exact MS |
| mixED 05 | A4J001 | 6-[4-[[2-[4-[3-(Dimethylcarbamoyl)-4-hydroxynaphthalen-1-yl]phenyl]phenyl]methyl]piperazin-1-yl]-N,N-diethylpyridazine-3-carboxamide | C39H42N6O3 | 642.33 |
| | A4J010 | 6-[4-[2-[2-[4-[3-(Dimethylcarbamoyl)-4-hydroxynaphthalen-1-yl]phenyl]ethynyl]benzoyl]piperazin-1-yl]-N,N-diethylpyridazine-3-carboxamide | C43H44N6O3 | 692.35 |
| | A4J011 | 6-[4-[2-[2-[6-[3-(Dimethylcarbamoyl)-4-hydroxynaphthalen-1-yl]pyridin-3-yl]ethynyl]benzoyl]piperazin-1-yl]-N,N-diethylpyridazine-3-carboxamide | C43H42N6O4 | 706.33 |

| | | | | |
|---|---|---|---|---|
| | A4J012 | 6-[4-[[4-[4-[3-(Dimethylcarbamoyl)-4-hydroxynaphthalen-1-yl]phenyl]naphthalen-1-yl]methyl]piperazin-1-yl]-N,N-diethylpyridazine-3-carboxamide | C37H40N6O3S | 648.29 |
| | A4J013 | 6-[4-[3-tert-Butyl-5-[4-[3-(dimethylcarbamoyl)-4-hydroxynaphthalen-1-yl]phenyl]benzoyl]piperazin-1-yl]-N,N-diethylpyridazine-3-carboxamide | C37H38N6O4S | 662.27 |
| | A4J014 | 6-[4-[3-[2-[4-[3-(Dimethylcarbamoyl)-4-hydroxynaphthalen-1-yl]phenyl]phenyl]propanoyl]piperazin-1-yl]-N,N-diethylpyridazine-3-carboxamide | C40H41F3N6O3 | 710.32 |
| | A4J015 | 6-[4-[[2-[2-[4-[3-(Dimethylcarbamoyl)-4-hydroxynaphthalen-1-yl]phenyl]ethynyl]phenyl]methyl]piperazin-1-yl]-N,N-diethylpyridazine-3-carboxamide | C40H39F3N6O4 | 724.3 |
| | A4J016 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N,N-diethylpyridazine-3-carboxamide | C40H41F3N6O4 | 726.31 |
| | A4J017 | 6-[4-[5-[2-[4-[3-(Dimethylcarbamoyl)-4-hydroxynaphthalen-1-yl]phenyl]ethynyl]pyridine-3-carbonyl]piperazin-1-yl]-N,N-diethylpyridazine-3-carboxamide | C41H46N6O4 | 686.36 |
| mixED 05 | A4J018 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N,N-diethylpyridazine-3-carboxamide | C42H48N6O4 | 700.37 |
| | A4J019 | 6-[4-[[2-[6-[3-(Dimethylcarbamoyl)-4-hydroxynaphthalen-1-yl]pyridin-3-yl]phenyl]methyl]piperazin-1-yl]-N,N-diethylpyridazine-3-carboxamide | C43H48N6O4 | 712.37 |
| | A4J002 | 4-[4-[2-[2-[4-[4-(Diethylcarbamoyl)phenyl]piperazine-1-carbonyl]phenyl]ethynyl]phenyl]-1-hydroxy-N,N-dimethylnaphthalene-2-carboxamide | C41H44N6O4 | 684.34 |
| | A4J020 | 6-[4-[3-[2-[6-[3-(Dimethylcarbamoyl)-4-hydroxynaphthalen-1-yl]pyridin-3-yl]phenyl]propanoyl]piperazin-1-yl]-N,N-diethylpyridazine-3-carboxamide | C41H42N6O3 | 666.33 |
| | A4J021 | 4-[5-[2-[2-[4-[4-(Diethylcarbamoyl)phenyl]piperazine-1-carbonyl]phenyl]ethynyl]pyridin-2-yl]-1-hydroxy-N,N-dimethylnaphthalene-2-carboxamide | C42H39F3N6O4 | 748.3 |
| | A4J022 | 6-[4-[[4-[6-[3-(Dimethylcarbamoyl)-4-hydroxynaphthalen-1-yl]pyridin-3-yl]-3-methylphenyl]methyl]piperazin-1-yl]-N,N-diethylpyridazine-3-carboxamide | C40H39N7O4 | 681.31 |
| | A4J023 | 6-[4-[4-[6-[3-(Dimethylcarbamoyl)-4-hydroxynaphthalen-1-yl]pyridin-3-yl]-3-methylbenzoyl]piperazin-1-yl]-N,N-diethylpyridazine-3-carboxamide | C43H41F3N8O4 | 790.32 |
| | A4J024 | 6-[4-[4-[4-[3-(Dimethylcarbamoyl)-4-hydroxynaphthalen-1-yl]phenyl]naphthalene-1-carbonyl]piperazin-1-yl]-N,N-diethylpyridazine-3-carboxamide | C38H41N7O3 | 643.33 |
| | A4J025 | 6-[4-[[4-[6-[3-(Dimethylcarbamoyl)-4-hydroxynaphthalen-1-yl]pyridin-3-yl]-2-ethylphenyl]methyl]piperazin-1-yl]-N,N-diethylpyridazine-3-carboxamide | C40H43N7O4 | 685.34 |
| | A4J026 | 6-[4-[[4-[6-[3-(Dimethylcarbamoyl)-4-hydroxynaphthalen-1-yl]pyridin-3-yl]-3-fluorophenyl]methyl]piperazin-1-yl]-N,N-diethylpyridazine-3-carboxamide | C39H43N7O3 | 657.34 |
| | A4J027 | 6-[4-[[4-[4-[3-(Dimethylcarbamoyl)-4-hydroxynaphthalen-1-yl]phenyl]-3-methylphenyl]methyl]piperazin-1-yl]-N,N-diethylpyridazine-3-carboxamide | C39H41N7O4 | 671.32 |
| | A4J028 | 6-[4-[4-[6-[3-(Dimethylcarbamoyl)-4-hydroxynaphthalen-1-yl]pyridin-3-yl]-3-fluorobenzoyl]piperazin-1-yl]-N,N-diethylpyridazine-3-carboxamide | C40H45N7O3 | 671.36 |
| | A4J029 | 6-[4-[[4-[6-[3-(Dimethylcarbamoyl)-4-hydroxynaphthalen-1-yl]pyridin-3-yl]-2-methoxyphenyl]methyl]piperazin-1-yl]-N,N-diethylpyridazine-3-carboxamide | C38H40FN7O3 | 661.32 |
| | A4J003 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N,N-diethylpyridazine-3-carboxamide | C40H44N6O3 | 656.35 |
| | A4J030 | 6-[4-[[4-[6-[3-(Dimethylcarbamoyl)-4-hydroxynaphthalen-1-yl]pyridin-3-yl]naphthalen-1-yl]methyl]piperazin-1-yl]-N,N-diethylpyridazine-3-carboxamide | C38H38FN7O4 | 675.3 |
| | A4J031 | 6-[4-[4-[6-[3-(Dimethylcarbamoyl)-4-hydroxynaphthalen-1-yl]pyridin-3-yl]naphthalene-1-carbonyl]piperazin-1-yl]-N,N-diethylpyridazine-3-carboxamide | C39H43N7O4 | 673.34 |
| | A4J032 | 6-[4-[[4-[6-[3-(Dimethylcarbamoyl)-4-hydroxynaphthalen-1-yl]pyridin-3-yl]thiophen-2-yl]methyl]piperazin-1-yl]-N,N-diethylpyridazine-3-carboxamide | C40H45N7O5 | 703.35 |
| | A4J033 | 6-[4-[4-[6-[3-(Dimethylcarbamoyl)-4-hydroxynaphthalen-1-yl]pyridin-3-yl]thiophene-2-carbonyl]piperazin-1-yl]-N,N-diethylpyridazine-3-carboxamide | C42H43N7O3 | 693.34 |
| | A4J034 | 6-[4-[[4-[4-[3-(Dimethylcarbamoyl)-4-hydroxynaphthalen-1-yl]phenyl]thiophen-2-yl]methyl]piperazin-1-yl]-N,N-diethylpyridazine-3-carboxamide | C42H41N7O4 | 707.32 |
| | A4J035 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N,N-diethylpyridazine-3-carboxamide | C36H39N7O3S | 649.28 |
| | A4J036 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N,N-diethylpyridazine-3-carboxamide | C36H37N7O4S | 663.26 |
| | A4J037 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N,N-diethylpyridazine-3-carboxamide | C39H40F3N7O3 | 711.31 |

| | | | | |
|---|---|---|---|---|
| | A4J038 | 6-[4-[4-[4-[3-(Dimethylcarbamoyl)-4-hydroxynaphthalen-1-yl]phenyl]-3-methylbenzoyl]piperazin-1-yl]-N,N-diethylpyridazine-3-carboxamide | C39H38F3N7O4 | 725.29 |
| | A4J039 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N,N-diethylpyridazine-3-carboxamide | C39H40F3N7O4 | 727.31 |

[Table 117]

| | | | | |
|---|---|---|---|---|
| mixED 05 | A4J004 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N,N-diethylpyridazine-3-carboxamide | C40H42N6O4 | 670.33 |
| | A4J040 | 6-[4-[3-tert-Butyl-5-[6-[3-(dimethylcarbamoyl)-4-hydroxynaphthalen-1-yl]pyridin-3-yl]benzoyl]piperazin-1-yl]-N,N-diethylpyridazine-3-carboxamide | C40H45N7O4 | 687.35 |
| | A4J041 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N,N-diethylpyridazine-3-carboxamide | C41H47N7O4 | 701.37 |
| mixED 05 | A4J042 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N,N-diethylpyridazine-3-carboxamide | C42H47N7O4 | 713.37 |
| | A4J043 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N,N-diethylpyridazine-3-carboxamide | C40H41N7O3 | 667.33 |
| | A4J044 | 6-[4-[4-[4-[3-(Dimethylcarbamoyl)-4-hydroxynaphthalen-1-yl]phenyl]thiophene-2-carbonyl]piperazin-1-yl]-N,N-diethylpyridazine-3-carboxamide | C41H38F3N7O4 | 749.29 |
| | A4J045 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N,N-diethylpyridazine-3-carboxamide | C39H38N8O4 | 682.3 |
| | A4J046 | 4-[4-[2-[[4-[4-(Diethylcarbamoyl)phenyl]piperazin-1-yl]methyl]phenyl]phenyl]-1-hydroxy-N,N-dimethylnaphthalene-2-carboxamide | C42H40F3N9O4 | 791.32 |
| | A4J047 | 4-[4-[2-[3-[4-[4-(Diethylcarbamoyl)phenyl]piperazin-1-yl]-3-oxopropyl]phenyl]phenyl]-1-hydroxy-N,N-dimethylnaphthalene-2-carboxamide | C41H44N4O3 | 640.34 |
| | A4J048 | 4-[4-[4-[1-[4-[4-(Diethylcarbamoyl)phenyl]piperazin-1-yl]ethyl]phenyl]phenyl]-1-hydroxy-N,N-dimethylnaphthalene-2-carboxamide | C43H46N4O4 | 682.35 |
| | A4J049 | 6-[4-[[4-[4-[3-(Dimethylcarbamoyl)-4-hydroxynaphthalen-1-yl]phenyl]-2-ethylphenyl]methyl]piperazin-1-yl]-N,N-diethylpyridazine-3-carboxamide | C42H46N4O3 | 654.36 |
| | A4J005 | 4-[4-[4-[[4-[4-(Diethylcarbamoyl)phenyl]piperazin-1-yl]methyl]-2-methylphenyl]phenyl]-1-hydroxy-N,N-dimethylnaphthalene-2-carboxamide | C41H46N6O3 | 670.36 |
| | A4J050 | 4-[4-[4-[4-[4-(Diethylcarbamoyl)phenyl]piperazine-1-carbonyl]-2-methylphenyl]phenyl]-1-hydroxy-N,N-dimethylnaphthalene-2-carboxamide | C42H46N4O3 | 654.36 |
| | A4J051 | 4-[4-[4-[[4-[4-(Diethylcarbamoyl)phenyl]piperazin-1-yl]methyl]-3-ethylphenyl]phenyl]-1-hydroxy-N,N-dimethylnaphthalene-2-carboxamide | C42H44N4O4 | 668.34 |
| | A4J052 | 4-[4-[4-[[4-[4-(Diethylcarbamoyl)phenyl]piperazin-1-yl]methyl]-2-fluorophenyl]phenyl]-1-hydroxy-N,N-dimethylnaphthalene-2-carboxamide | C43H48N4O3 | 668.37 |
| | A4J053 | 4-[4-[4-[4-[4-(Diethylcarbamoyl)phenyl]piperazine-1-carbonyl]-2-fluorophenyl]phenyl]-1-hydroxy-N,N-dimethylnaphthalene-2-carboxamide | C41H43FN4O3 | 658.33 |
| | A4J054 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N,N-diethylpyridazine-3-carboxamide | C41H41FN4O4 | 672.31 |
| | A4J055 | 4-[4-[4-[[4-[4-(Diethylcarbamoyl)phenyl]piperazin-1-yl]methyl]-3-methoxyphenyl]phenyl]-1-hydroxy-N,N-dimethylnaphthalene-2-carboxamide | C42H46N4O4 | 670.35 |
| | A4J056 | 4-[4-[4-[[4-[4-(Diethylcarbamoyl)phenyl]piperazin-1-yl]methyl]-3,5-dimethoxyphenyl]phenyl]-1-hydroxy-N,N-dimethylnaphthalene-2-carboxamide | C43H48N4O5 | 700.36 |
| | A4J057 | 4-[4-[4-[[4-[4-(Diethylcarbamoyl)phenyl]piperazin-1-yl]methyl]naphthalen-1-yl]phenyl]-1-hydroxy-N,N-dimethylnaphthalene-2-carboxamide | C45H46N4O3 | 690.36 |
| | A4J058 | 4-[4-[4-[4-[4-(Diethylcarbamoyl)phenyl]piperazine-1-carbonyl]naphthalen-1-yl]phenyl]-1-hydroxy-N,N-dimethylnaphthalene-2-carboxamide | C45H44N4O4 | 704.34 |
| | A4J059 | 4-[4-[5-[[4-[4-(Diethylcarbamoyl)phenyl]piperazin-1-yl]methyl]thiophen-3-yl]phenyl]-1-hydroxy-N,N-dimethylnaphthalene-2-carboxamide | C39H42N4O3S | 646.3 |
| | A4J006 | 6-[4-[[4-[4-[3-(Dimethylcarbamoyl)-4-hydroxynaphthalen-1-yl]phenyl]-3-fluorophenyl]methyl]piperazin-1-yl]-N,N-diethylpyridazine-3-carboxamide | C39H41FN6O3 | 660.32 |
| | A4J060 | 4-[4-[5-[4-[4-(Diethylcarbamoyl)phenyl]piperazine-1-carbonyl]thiophen-3-yl]phenyl]-1-hydroxy-N,N-dimethylnaphthalene-2-carboxamide | C39H40N4O4S | 660.28 |
| | A4J061 | 4-[4-[3-[[4-[4-(Diethylcarbamoyl)phenyl]piperazin-1-yl]methyl]-5-(trifluoromethyl)phenyl]phenyl]-1-hydroxy-N,N-dimethylnaphthalene-2-carboxamide | C42H43F3N4O3 | 708.33 |

| | | | | |
|---|---|---|---|---|
| | A4J062 | 4-[4-[3-[4-[4-(Diethylcarbamoyl)phenyl]piperazine-1-carbonyl]-5-(trifluoromethyl)phenyl]phenyl]-1-hydroxy-N,N-dimethylnaphthalene-2-carboxamide | C42H41F3N4O4 | 722.31 |
| | A4J063 | 4-[4-[3-[[4-[4-(Diethylcarbamoyl)phenyl]piperazin-1-yl]methyl]-5-(trifluoromethoxy)phenyl]phenyl]-1-hydroxy-N,N-dimethylnaphthalene-2-carboxamide | C42H43F3N4O4 | 724.32 |
| | A4J064 | 6-[4-[3-[4-[3-(Dimethylcarbamoyl)-4-hydroxynaphthalen-1-yl]phenyl]-5-(trifluoromethyl)benzoyl]piperazin-1-yl]-N,N-diethylpyridazine-3-carboxamide | C43H48N4O4 | 684.37 |
| | A4J065 | 4-[4-[3-[[4-[4-(Diethylcarbamoyl)phenyl]piperazin-1-yl]methyl]-5-ethoxyphenyl]phenyl]-1-hydroxy-N,N-dimethylnaphthalene-2-carboxamide | C44H50N4O4 | 698.38 |
| | A4J066 | 4-[4-[3-[[4-[4-(Diethylcarbamoyl)phenyl]piperazin-1-yl]methyl]-5-propan-2-yloxyphenyl]phenyl]-1-hydroxy-N,N-dimethylnaphthalene-2-carboxamide | C45H50N4O4 | 710.38 |
| | A4J067 | 4-[4-[3-tert-Butyl-5-[4-[4-(diethylcarbamoyl)phenyl]piperazine-1-carbonyl]phenyl]phenyl]-1-hydroxy-N,N-dimethylnaphthalene-2-carboxamide | C42H45FN4O3 | 672.35 |
| mixED 05 | A4J068 | 4-[4-[4-[1-[4-[4-(Diethylcarbamoyl)phenyl]piperazin-1-yl]ethyl]-3-fluorophenyl]phenyl]-1-hydroxy-N,N-dimethylnaphthalene-2-carboxamide | C43H44N4O3 | 664.34 |
| | A4J069 | 4-[4-[2-[2-[[4-[4-(Diethylcarbamoyl)phenyl]piperazin-1-yl]methyl]phenyl]ethynyl]phenyl]-1-hydroxy-N,N-dimethylnaphthalene-2-carboxamide | C44H41F3N4O4 | 746.31 |

[Table 118]

| | | | | |
|---|---|---|---|---|
| mixED 05 | A4J007 | 4-[4-[2-[3-[4-[4-(Diethylcarbamoyl)phenyl]piperazine-1-carbonyl]-5-(trifluoromethyl)phenyl]ethynyl]phenyl]-1-hydroxy-N,N-dimethylnaphthalene-2-carboxamide | C39H39FN6O4 | 674.3 |
| | A4J070 | 6-[4-[4-[4-[3-(Dimethylcarbamoyl)-4-hydroxynaphthalen-1-yl]phenyl]-3-fluorobenzoyl]piperazin-1-yl]-N,N-diethylpyridazine-3-carboxamide | C42H41N5O4 | 679.32 |
| | A4J071 | 4-[4-[2-[5-[4-[4-(Diethylcarbamoyl)phenyl]piperazine-1-carbonyl]pyridin-3-yl]ethynyl]phenyl]-1-hydroxy-N,N-dimethylnaphthalene-2-carboxamide | C45H43F3N6O4 | 788.33 |
| | A4J072 | 4-[4-[1-[3-[4-[4-(Diethylcarbamoyl)phenyl]piperazine-1-carbonyl]-5-(trifluoromethyl)phenyl]pyrazol-4-yl]phenyl]-1-hydroxy-N,N-dimethylnaphthalene-2-carboxamide | C40H43N5O3 | 641.34 |
| | A4J073 | 4-[5-[2-[[4-[4-(Diethylcarbamoyl)phenyl]piperazin-1-yl]methyl]phenyl]pyridin-2-yl]-1-hydroxy-N,N-dimethylnaphthalene-2-carboxamide | C42H45N5O4 | 683.35 |
| | A4J074 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N,N-diethylpyridazine-3-carboxamide | C41H45N5O3 | 655.35 |
| | A4J075 | 4-[5-[2-[3-[4-[4-(Diethylcarbamoyl)phenyl]piperazin-1-yl]-3-oxopropyl]phenyl]pyridin-2-yl]-1-hydroxy-N,N-dimethylnaphthalene-2-carboxamide | C41H45N5O3 | 655.35 |
| | A4J076 | 4-[5-[4-[1-[4-[4-(Diethylcarbamoyl)phenyl]piperazin-1-yl]ethyl]phenyl]pyridin-2-yl]-1-hydroxy-N,N-dimethylnaphthalene-2-carboxamide | C41H43N5O4 | 669.33 |
| | A4J077 | 4-[5-[4-[[4-[4-(Diethylcarbamoyl)phenyl]piperazin-1-yl]methyl]-2-methylphenyl]pyridin-2-yl]-1-hydroxy-N,N-dimethylnaphthalene-2-carboxamide | C42H47N5O3 | 669.37 |
| | A4J078 | 4-[5-[4-[4-[4-(Diethylcarbamoyl)phenyl]piperazine-1-carbonyl]-2-methylphenyl]pyridin-2-yl]-1-hydroxy-N,N-dimethylnaphthalene-2-carboxamide | C40H42FN5O3 | 659.33 |
| | A4J079 | 4-[5-[4-[[4-[4-(Diethylcarbamoyl)phenyl]piperazin-1-yl]methyl]-3-ethylphenyl]pyridin-2-yl]-1-hydroxy-N,N-dimethylnaphthalene-2-carboxamide | C40H40FN5O4 | 673.31 |
| | A4J008 | 4-[5-[4-[[4-[4-(Diethylcarbamoyl)phenyl]piperazin-1-yl]methyl]-2-fluorophenyl]pyridin-2-yl]-1-hydroxy-N,N-dimethylnaphthalene-2-carboxamide | C40H44N6O4 | 672.34 |
| | A4J080 | 4-[5-[4-[4-[4-(Diethylcarbamoyl)phenyl]piperazine-1-carbonyl]-2-fluorophenyl]pyridin-2-yl]-1-hydroxy-N,N-dimethylnaphthalene-2-carboxamide | C41H45N5O4 | 671.35 |
| | A4J081 | 6-[4-[[4-[4-[3-(Dimethylcarbamoyl)-4-hydroxynaphthalen-1-yl]phenyl]-2-methoxyphenyl]methyl]piperazin-1-yl]-N,N-diethylpyridazine-3-carboxamide | C42H47N5O5 | 701.36 |
| | A4J082 | 4-[5-[4-[[4-[4-(Diethylcarbamoyl)phenyl]piperazin-1-yl]methyl]-3-methoxyphenyl]pyridin-2-yl]-1-hydroxy-N,N-dimethylnaphthalene-2-carboxamide | C44H45N5O3 | 691.35 |
| | A4J083 | 4-[5-[4-[[4-[4-(Diethylcarbamoyl)phenyl]piperazin-1-yl]methyl]-3,5-dimethoxyphenyl]pyridin-2-yl]-1-hydroxy-N,N-dimethylnaphthalene-2-carboxamide | C44H43N5O4 | 705.33 |
| | A4J084 | 6-[4-[[3-[4-[3-(Dimethylcarbamoyl)-4-hydroxynaphthalen-1-yl]phenyl]-5-ethoxyphenyl]methyl]piperazin-1-yl]-N,N-diethylpyridazine-3-carboxamide | C38H41N5O3S | 647.29 |
| | A4J085 | 4-[5-[4-[[4-[4-(Diethylcarbamoyl)phenyl]piperazin-1-yl]methyl]naphthalen-1-yl]pyridin-2-yl]-1-hydroxy-N,N-dimethylnaphthalene-2-carboxamide | C38H39N5O4S | 661.27 |
| | A4J086 | 4-[5-[4-[4-[4-(Diethylcarbamoyl)phenyl]piperazine-1-carbonyl]naphthalen-1-yl]pyridin-2-yl]-1-hydroxy-N,N-dimethylnaphthalene-2-carboxamide | C41H42F3N5O3 | 709.32 |

| | | | | |
|---|---|---|---|---|
| | A4J087 | 4-[5-[5-[[4-[4-(Diethylcarbamoyl)phenyl]piperazin-1-yl]methyl]thiophen-3-yl]pyridin-2-yl]-1-hydroxy-N,N-dimethylnaphthalene-2-carboxamide | C41H40F3N5O4 | 723.3 |
| | A4J088 | 4-[5-[5-[4-[4-(Diethylcarbamoyl)phenyl]piperazine-1-carbonyl]thiophen-3-yl]pyridin-2-yl]-1-hydroxy-N,N-dimethylnaphthalene-2-carboxamide | C41H42F3N5O4 | 725.32 |
| | A4J089 | 4-[5-[3-[[4-[4-(Diethylcarbamoyl)phenyl]piperazin-1-yl]methyl]-5-(trifluoromethyl)phenyl]pyridin-2-yl]-1-hydroxy-N,N-dimethylnaphthalene-2-carboxamide | C42H47N5O4 | 685.36 |
| | A4J009 | 4-[5-[3-[4-[4-(Diethylcarbamoyl)phenyl]piperazine-1-carbonyl]-5-(trifluoromethyl)phenyl]pyridin-2-yl]-1-hydroxy-N,N-dimethylnaphthalene-2-carboxamide | C41H46N6O5 | 702.35 |
| mixED05 | A4J090 | 4-[5-[3-[[4-[4-(Diethylcarbamoyl)phenyl]piperazin-1-yl]methyl]-5-(trifluoromethoxy)phenyl]pyridin-2-yl]-1-hydroxy-N,N-dimethylnaphthalene-2-carboxamide | C43H49N5O4 | 699.38 |
| | A4J091 | 4-[5-[3-[[4-[4-(Diethylcarbamoyl)phenyl]piperazin-1-yl]methyl]-5-ethoxyphenyl]pyridin-2-yl]-1-hydroxy-N,N-dimethylnaphthalene-2-carboxamide | C44H49N5O4 | 711.38 |
| | A4J092 | 6-[4-[[4-[4-[3-(Dimethylcarbamoyl)-4-hydroxynaphthalen-1-yl]phenyl]-2,6-dimethoxyphenyl]methyl]piperazin-1-yl]-N,N-diethylpyridazine-3-carboxamide | C41H44FN5O3 | 673.34 |
| | A4J093 | 4-[5-[3-[[4-[4-(Diethylcarbamoyl)phenyl]piperazin-1-yl]methyl]-5-propan-2-yloxyphenyl]pyridin-2-yl]-1-hydroxy-N,N-dimethylnaphthalene-2-carboxamide | C42H43N5O3 | 665.34 |
| | A4J094 | 6-[4-[[3-[4-[3-(Dimethylcarbamoyl)-4-hydroxynaphthalen-1-yl]phenyl]-5-propan-2-yloxyphenyl]methyl]piperazin-1-yl]-N,N-diethylpyridazine-3-carboxamide | C43H40F3N5O4 | 747.3 |
| | A4J095 | 4-[5-[3-tert-Butyl-5-[4-[4-(diethylcarbamoyl)phenyl]piperazine-1-carbonyl]phenyl]pyridin-2-yl]-1-hydroxy-N,N-dimethylnaphthalene-2-carboxamide | C41H40N6O4 | 680.31 |
| | A4J096 | 4-[5-[4-[1-[4-[4-(Diethylcarbamoyl)phenyl]piperazin-1-yl]ethyl]-3-fluorophenyl]pyridin-2-yl]-1-hydroxy-N,N-dimethylnaphthalene-2-carboxamide | C44H42F3N7O4 | 789.33 |
| | A4J097 | 4-[5-[2-[2-[[4-[4-(Diethylcarbamoyl)phenyl]piperazin-1-yl]methyl]phenyl]ethynyl]pyridin-2-yl]-1-hydroxy-N,N-dimethylnaphthalene-2-carboxamide | C41H40N6O4 | 680.311 |
| | A4J098 | 4-[5-[2-[3-[4-[4-(Diethylcarbamoyl)phenyl]piperazine-1-carbonyl]-5-(trifluoromethyl)phenyl]ethynyl]pyridin-2-yl]-1-hydroxy-N,N-dimethylnaphthalene-2-carboxamide | C40H39 N7O4 | 681.306 |
| | A4J099 | 4-[5-[2-[5-[4-[4-(Diethylcarbamoyl)phenyl]piperazine-1-carbonyl]pyridin-3-yl]ethynyl]pyridin-2-yl]-1-hydroxy-N,N-dimethylnaphthalene-2-carboxamide | C43H42N4O4 | 678.321 |
| | A4J100 | 4-[5-[1-[3-[4-[4-(Diethylcarbamoyl)phenyl]piperazine-1-carbonyl]-5-(trifluoromethyl)phenyl]pyrazol-4-yl]pyridin-2-yl]-1-hydroxy-N,N-dimethylnaphthalene-2-carboxamide | C42H41N5O4 | 679.316 |

Example 3-7-2: Synthesis of mixtures mixED02 to mixED12

**[2347]** The mixtures shown in Table EE02 to Table EE12 were obtained in the same manner as in Example 3-7-1 using the mixtures shown in Table ED06 to Table ED16. Specifically, mixED06 was obtained using mixED06-01R; mixED07 was obtained using mixED07-01R; mixED08 was obtained using mixED08-01R; mixED09 was obtained using mixED09-01R; mixED10 was obtained using mixED10-01R; mixED 11 was obtained using mixED11-01R; mixED12 was obtained using mixED12-01R; mixED13 was obtained using mixED13-01R; mixED14 was obtained using mixED14-01R; mixED15 was obtained using mixED15-01R; and mixED 16 was obtained using mixED16-01R.

[Table 119]

[Table EE02]

| Table EE02 |
|---|

| Mixture No. | No. of compound that may be contained | Compound name | Compositional formula | Exact MS |
|---|---|---|---|---|
| mixED 06 | A1M001 | 6-[4-[[2-[4-[3-(Dimethylcarbamoyl)-4-hydroxynaphthalen-1-yl]phenyl]phenyl]methyl]piperazin-1-yl]-N-propylpyridazine-3-carboxamide | C38H40N6O3 | 628.32 |
| | A1M010 | 6-[4-[[4-[4-[3-(Dimethylcarbamoyl)-4-hydroxynaphthalen-1-yl]phenyl]naphthalen-1-yl]methyl]piperazin-1-yl]-N-propylpyridazine-3-carboxamide | C42H42N6O3 | 678.33 |
| | A1M011 | 6-[4-[4-[4-[3-(Dimethylcarbamoyl)-4-hydroxynaphthalen-1-yl]phenyl]naphthalene-1-carbonyl]piperazin-1-yl]-N-propylpyridazine-3-carboxamide | C42H40N6O4 | 692.31 |
| | A1M012 | 6-[4-[[4-[4-[3-(Dimethylcarbamoyl)-4-hydroxynaphthalen-1-yl]phenyl]thiophen-2-yl]methyl]piperazin-1-yl]-N-propylpyridazine-3-carboxamide | C36H38N6O3S | 634.27 |
| mixED 06 | A1M013 | 6-[4-[4-[4-[3-(Dimethylcarbamoyl)-4-hydroxynaphthalen-1-yl]phenyl]thiophene-2-carbonyl]piperazin-1-yl]-N-propylpyridazine-3-carboxamide | C36H36N6O4S | 648.25 |
| | A1M014 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-propylpyridazine-3-carboxamide | C39H39F3N6O3 | 696.3 |
| | A1M015 | 6-[4-[3-[4-[3-(Dimethylcarbamoyl)-4-hydroxynaphthalen-1-yl]phenyl]-5-(trifluoromethyl)benzoyl]piperazin-1-yl]-N-propylpyridazine-3-carboxamide | C39H37F3N6O4 | 710.28 |
| | A1M016 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-propylpyridazine-3-carboxamide | C39H39F3N6O4 | 712.3 |
| | A1M017 | 6-[4-[[3-[4-[3-(Dimethylcarbamoyl)-4-hydroxynaphthalen-1-yl]phenyl]-5-ethoxyphenyl]methyl]piperazin-1-yl]-N-propylpyridazine-3-carboxamide | C40H44N6O4 | 672.34 |
| | A1M018 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-propylpyridazine-3-carboxamide | C41H46N6O4 | 686.36 |
| | A1M019 | 6-[4-[3-tert-Butyl-5-[4-[3-(dimethylcarbamoyl)-4-hydroxynaphthalen-1-yl]phenyl]benzoyl]piperazin-1-yl]-N-propylpyridazine-3-carboxamide | C42H46N6O4 | 698.36 |
| | A1M002 | 6-[4-[3-[2-[4-[3-(Dimethylcarbamoyl)-4-hydroxynaphthalen-1-yl]phenyl]phenyl]propanoyl]piperazin-1-yl]-N-propylpyridazine-3-carboxamide | C40H42N6O4 | 670.33 |
| | A1M020 | 6-[4-[[2-[2-[4-[3-(Dimethylcarbamoyl)-4-hydroxynaphthalen-1-yl]phenyl]ethynyl]phenyl]methyl]piperazin-1-yl]-N-propylpyridazine-3-carboxamide | C40H40N6O3 | 652.32 |
| | A1M021 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-propylpyridazine-3-carboxamide | C41H37F3N6O4 | 734.28 |
| | A1M022 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-propylpyridazine-3-carboxamide | C39H37N7O4 | 667.29 |
| | A1M023 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-propylpyridazine-3-carboxamide | C42H39F3N8O4 | 776.3 |
| | A1M024 | 6-[4-[[2-[6-[3-(Dimethylcarbamoyl)-4-hydroxynaphthalen-1-yl]pyridin-3-yl]phenyl]methyl]piperazin-1-yl]-N-propylpyridazine-3-carboxamide | C37H39N7O3 | 629.31 |
| | A1M025 | 6-[4-[3-[2-[6-[3-(Dimethylcarbamoyl)-4-hydroxynaphthalen-1-yl]pyridin-3-yl]phenyl]propanoyl]piperazin-1-yl]-N-propylpyridazine-3-carboxamide | C39H41N7O4 | 671.32 |
| | A1M026 | 6-[4-[[4-[6-[3-(Dimethylcarbamoyl)-4-hydroxynaphthalen-1-yl]pyridin-3-yl]-3-methylphenyl]methyl]piperazin-1-yl]-N-propylpyridazine-3-carboxamide | C38H41N7O3 | 643.33 |
| | A1M027 | 6-[4-[4-[6-[3-(Dimethylcarbamoyl)-4-hydroxynaphthalen-1-yl]pyridin-3-yl]-3-methylbenzoyl]piperazin-1-yl]-N-propylpyridazine-3-carboxamide | C38H39N7O4 | 657.31 |
| | A1M028 | 6-[4-[[4-[6-[3-(Dimethylcarbamoyl)-4-hydroxynaphthalen-1-yl]pyridin-3-yl]-2-ethylphenyl]methyl]piperazin-1-yl]-N-propylpyridazine-3-carboxamide | C39H43N7O3 | 657.34 |
| | A1M029 | 6-[4-[[4-[6-[3-(Dimethylcarbamoyl)-4-hydroxynaphthalen-1-yl]pyridin-3-yl]-3-fluorophenyl]methyl]piperazin-1-yl]-N-propylpyridazine-3-carboxamide | C37H38FN7O3 | 647.3 |
| | A1M003 | 6-[4-[[4-[4-[3-(Dimethylcarbamoyl)-4-hydroxynaphthalen-1-yl]phenyl]-3-methylphenyl]methyl]piperazin-1-yl]-N-propylpyridazine-3-carboxamide | C39H42N6O3 | 642.33 |
| | A1M030 | 6-[4-[4-[6-[3-(Dimethylcarbamoyl)-4-hydroxynaphthalen-1-yl]pyridin-3-yl]-3-fluorobenzoyl]piperazin-1-yl]-N-propylpyridazine-3-carboxamide | C37H36FN7O4 | 661.28 |
| | A1M031 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-propylpyridazine-3-carboxamide | C38H41N7O4 | 659.32 |
| | A1M032 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-propylpyridazine-3-carboxamide | C39H43N7O5 | 689.33 |
| | A1M033 | 6-[4-[[4-[6-[3-(Dimethylcarbamoyl)-4-hydroxynaphthalen-1-yl]pyridin-3-yl]naphthalen-1-yl]methyl]piperazin-1-yl]-N-propylpyridazine-3-carboxamide | C41H41N7O3 | 679.33 |
| | A1M034 | 6-[4-[4-[6-[3-(Dimethylcarbamoyl)-4-hydroxynaphthalen-1-yl]pyridin-3-yl]naphthalene-1-carbonyl]piperazin-1-yl]-N-propylpyridazine-3-carboxamide | C41H39N7O4 | 693.31 |

| | | | | |
|---|---|---|---|---|
| | A1M035 | 6-[4-[[4-[6-[3-(Dimethylcarbamoyl)-4-hydroxynaphthalen-1-yl]pyridin-3-yl]thiophen-2-yl]methyl]piperazin-1-yl]-N-propylpyridazine-3-carboxamide | C35H37N7O3S | 635.27 |
| | A1M036 | 6-[4-[4-[6-[3-(Dimethylcarbamoyl)-4-hydroxynaphthalen-1-yl]pyridin-3-yl]thiophene-2-carbonyl]piperazin-1-yl]-N-propylpyridazine-3-carboxamide | C35H35N7O4S | 649.25 |
| | A1M037 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-propylpyridazine-3-carboxamide | C38H38F3N7O3 | 697.3 |
| | A1M038 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-propylpyridazine-3-carboxamide | C38H36F3N7O4 | 711.28 |
| | A1M039 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-propylpyridazine-3-carboxamide | C38H38F3N7O4 | 713.29 |

[Table 120]

| | | | | |
|---|---|---|---|---|
| mixED 06 | A1M004 | 6-[4-[4-[4-[3-(Dimethylcarbamoyl)-4-hydroxynaphthalen-1-yl]phenyl]-3-methylbenzoyl]piperazin-1-yl]-N-propylpyridazine-3-carboxamide | C39H40N6O4 | 656.31 |
| | A1M040 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-propylpyridazine-3-carboxamide | C39H43N7O4 | 673.34 |
| | A1M041 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-propylpyridazine-3-carboxamide | C40H45N7O4 | 687.35 |
| | A1M042 | 6-[4-[3-tert-Butyl-5-[6-[3-(dimethylcarbamoyl)-4-hydroxynaphthalen-1-yl]pyridin-3-yl]benzoyl]piperazin-1-yl]-N-propylpyridazine-3-carboxamide | C41H45N7O4 | 699.35 |
| | A1M043 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-propylpyridazine-3-carboxamide | C39H39N7O3 | 653.31 |
| | A1M044 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-propylpyridazine-3-carboxamide | C40H36F3N7O4 | 735.28 |
| | A1M045 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-propylpyridazine-3-carboxamide | C38H36N8O4 | 668.29 |
| | A1M046 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-propylpyridazine-3-carboxamide | C41H38F3N9O4 | 777.3 |
| | A1M047 | 1-Hydroxy-N,N-dimethyl-4-[4-[2-[[4-[4-(propylcarbamoyl)phenyl]piperazin-1-yl]methyl]phenyl]phenyl]naphthalene-2-carboxamide | C40H42N4O3 | 626.33 |
| | A1M048 | 1-Hydroxy-N,N-dimethyl-4-[4-[2-[3-oxo-3-[4-[4-(propylcarbamoyl)phenyl]piperazin-1-yl]propyl]phenyl]phenyl]naphthalene-2-carboxamide | C42H44N4O4 | 668.34 |
| | A1M049 | 1-Hydroxy-N,N-dimethyl-4-[4-[4-[1-[4-[4-(propylcarbamoyl)phenyl]piperazin-1-yl]ethyl]phenyl]phenyl]naphthalene-2-carboxamide | C41H44N4O3 | 640.34 |
| | A1M005 | 6-[4-[[4-[4-[3-(Dimethylcarbamoyl)-4-hydroxynaphthalen-1-yl]phenyl]-2-ethylphenyl]methyl]piperazin-1-yl]-N-propylpyridazine-3-carboxamide | C40H44N6O3 | 656.35 |
| | A1M050 | 1-Hydroxy-N,N-dimethyl-4-[4-[2-methyl-4-[[4-[4-(propylcarbamoyl)phenyl]piperazin-1-yl]methyl]phenyl]phenyl]naphthalene-2-carboxamide | C41H44N4O3 | 640.34 |
| | A1M051 | 1-Hydroxy-N,N-dimethyl-4-[4-[2-methyl-4-[4-[4-(propylcarbamoyl)phenyl]piperazine-1-carbonyl]phenyl]phenyl]naphthalene-2-carboxamide | C41H42N4O4 | 654.32 |
| | A1M052 | 4-[4-[3-Ethyl-4-[[4-[4-(propylcarbamoyl)phenyl]piperazin-1-yl]methyl]phenyl]phenyl]-1-hydroxy-N,N-dimethylnaphthalene-2-carboxamide | C42H46N4O3 | 654.36 |
| | A1M053 | 4-[4-[2-Fluoro-4-[[4-[4-(propylcarbamoyl)phenyl]piperazin-1-yl]methyl]phenyl]phenyl]-1-hydroxy-N,N-dimethylnaphthalene-2-carboxamide | C40H41FN4O3 | 644.32 |
| | A1M054 | 4-[4-[2-Fluoro-4-[4-[4-(propylcarbamoyl)phenyl]piperazine-1-carbonyl]phenyl]phenyl]-1-hydroxy-N,N-dimethylnaphthalene-2-carboxamide | C40H39FN4O4 | 658.3 |
| | A1M055 | 1-Hydroxy-4-[4-[3-methoxy-4-[[4-[4-(propylcarbamoyl)phenyl]piperazin-1-yl]methyl]phenyl]phenyl]-N,N-dimethylnaphthalene-2-carboxamide | C41H44N4O4 | 656.34 |
| | A1M056 | 4-[4-[3,5-Dimethoxy-4-[[4-[4-(propylcarbamoyl)phenyl]piperazin-1-yl]methyl]phenyl]phenyl]-1-hydroxy-N,N-dimethylnaphthalene-2-carboxamide | C42H46N4O5 | 686.35 |
| | A1M057 | 1-Hydroxy-N,N-dimethyl-4-[4-[4-[[4-[4-(propylcarbamoyl)phenyl]piperazin-1-yl]methyl]naphthalen-1-yl]phenyl]naphthalene-2-carboxamide | C44H44N4O3 | 676.34 |
| | A1M058 | 1-Hydroxy-N,N-dimethyl-4-[4-[4-[4-[4-(propylcarbamoyl)phenyl]piperazine-1-carbonyl]naphthalen-1-yl]phenyl]naphthalene-2-carboxamide | C44H42N4O4 | 690.32 |
| | A1M059 | 1-Hydroxy-N,N-dimethyl-4-[4-[5-[[4-[4-(propylcarbamoyl)phenyl]piperazin-1-yl]methyl]thiophen-3-yl]phenyl]naphthalene-2-carboxamide | C38H40N4O3S | 632.28 |
| | A1M006 | 6-[4-[[4-[4-[3-(Dimethylcarbamoyl)-4-hydroxynaphthalen-1-yl]phenyl]-3-fluorophenyl]methyl]piperazin-1-yl]-N-propylpyridazine-3-carboxamide | C38H39FN6O3 | 646.31 |
| | A1M060 | 1-Hydroxy-N,N-dimethyl-4-[4-[5-[4-[4-(propylcarbamoyl)phenyl]piperazine-1-carbonyl]thiophen-3-yl]phenyl]naphthalene-2-carboxamide | C38H38N4O4S | 646.26 |

| | | | | |
|---|---|---|---|---|
| | A1M061 | 1-Hydroxy-N,N-dimethyl-4-[4-[3-[[4-[4-(propylcarbamoyl)phenyl]piperazin-1-yl]methyl]-5-(trifluoromethyl)phenyl]phenyl]naphthalene-2-carboxamide | C41H41F3N4O3 | 694.31 |
| | A1M062 | 1-Hydroxy-N,N-dimethyl-4-[4-[3-[4-[4-(propylcarbamoyl)phenyl]piperazine-1-carbonyl]-5-(trifluoromethyl)phenyl]phenyl]naphthalene-2-carboxamide | C41H39F3N4O4 | 708.29 |
| | A1M063 | 1-Hydroxy-N,N-dimethyl-4-[4-[3-[[4-[4-(propylcarbamoyl)phenyl]piperazin-1-yl]methyl]-5-(trifluoromethoxy)phenyl]phenyl]naphthalene-2-carboxamide | C41H41F3N4O4 | 710.31 |
| | A1M064 | 4-[4-[3-Ethoxy-5-[[4-[4-(propylcarbamoyl)phenyl]piperazin-1-yl]methyl]phenyl]phenyl]-1-hydroxy-N,N-dimethylnaphthalene-2-carboxamide | C42H46N4O4 | 670.35 |
| mixED 06 | A1M065 | 1-Hydroxy-N,N-dimethyl-4-[4-[3-propan-2-yloxy-5-[[4-[4-(propylcarbamoyl)phenyl]piperazin-1-yl]methyl]phenyl]phenyl]naphthalene-2-carboxamide | C43H48N4O4 | 684.37 |
| | A1M066 | 4-[4-[3-tert-Butyl-5-[4-[4-(propylcarbamoyl)phenyl]piperazine-1-carbonyl]phenyl]phenyl]-1-hydroxy-N,N-dimethylnaphthalene-2-carboxamide | C44H48N4O4 | 696.37 |
| | A1M067 | 4-[4-[3-Fluoro-4-[1-[4-[4-(propylcarbamoyl)phenyl]piperazin-1-yl]ethyl]phenyl]phenyl]-1-hydroxy-N,N-dimethylnaphthalene-2-carboxamide | C41H43FN4O3 | 658.33 |
| | A1M068 | 1-Hydroxy-N,N-dimethyl-4-[4-[2-[2-[[4-[4-(propylcarbamoyl)phenyl]piperazin-1-yl]methyl]phenyl]ethynyl]phenyl]naphthalene-2-carboxamide | C42H42N4O3 | 650.33 |
| | A1M069 | 1-Hydroxy-N,N-dimethyl-4-[4-[2-[3-[4-[4-(propylcarbamoyl)phenyl]piperazine-1-carbonyl]-5-(trifluoromethyl)phenyl]ethynyl]phenyl]naphthalene-2-carboxamide | C43H39F3N4O4 | 732.29 |

[Table 121]

| | | | | |
|---|---|---|---|---|
| mixED 06 | A1M007 | 6-[4-[4-[4-[3-(Dimethylcarbamoyl)-4-hydroxynaphthalen-1-yl]phenyl]-3-fluorobenzoyl]piperazin-1-yl]-N-propylpyridazine-3-carboxamide | C38H37FN6O4 | 660.29 |
| | A1M070 | 1-Hydroxy-N,N-dimethyl-4-[4-[2-[5-[4-[4-(propylcarbamoyl)phenyl]piperazine-1-carbonyl]pyridin-3-yl]ethynyl]phenyl]naphthalene-2-carboxamide | C41H39N5O4 | 665.3 |
| | A1M071 | 1-Hydroxy-N,N-dimethyl-4-[4-[1-[3-[4-[4-(propylcarbamoyl)phenyl]piperazine-1-carbonyl]-5-(trifluoromethyl)phenyl]pyrazol-4-yl]phenyl]naphthalene-2-carboxamide | C44H41F3N6O4 | 774.31 |
| | A1M072 | 1-Hydroxy-N,N-dimethyl-4-[5-[2-[[4-[4-(propylcarbamoyl)phenyl]piperazin-1-yl]methyl]phenyl]pyridin-2-yl]naphthalene-2-carboxamide | C39H41N5O3 | 627.32 |
| | A1M073 | 1-Hydroxy-N,N-dimethyl-4-[5-[2-[3-oxo-3-[4-[4-(propylcarbamoyl)phenyl]piperazin-1-yl]propyl]phenyl]pyridin-2-yl]naphthalene-2-carboxamide | C41H43N5O4 | 669.33 |
| | A1M074 | 1-Hydroxy-N,N-dimethyl-4-[5-[4-[1-[4-[4-(propylcarbamoyl)phenyl]piperazin-1-yl]ethyl]phenyl]pyridin-2-yl]naphthalene-2-carboxamide | C40H43N5O3 | 641.34 |
| | A1M075 | 1-Hydroxy-N,N-dimethyl-4-[5-[2-methyl-4-[[4-[4-(propylcarbamoyl)phenyl]piperazin-1-yl]methyl]phenyl]pyridin-2-yl]naphthalene-2-carboxamide | C40H43N5O3 | 641.34 |
| | A1M076 | 1-Hydroxy-N,N-dimethyl-4-[5-[2-methyl-4-[4-[4-(propylcarbamoyl)phenyl]piperazine-1-carbonyl]phenyl]pyridin-2-yl]naphthalene-2-carboxamide | C40H41N5O4 | 655.32 |
| | A1M077 | 4-[5-[3-Ethyl-4-[[4-[4-(propylcarbamoyl)phenyl]piperazin-1-yl]methyl]phenyl]pyridin-2-yl]-1-hydroxy-N,N-dimethylnaphthalene-2-carboxamide | C41H45N5O3 | 655.35 |
| | A1M078 | 4-[5-[2-Fluoro-4-[[4-[4-(propylcarbamoyl)phenyl]piperazin-1-yl]methyl]phenyl]pyridin-2-yl]-1-hydroxy-N,N-dimethylnaphthalene-2-carboxamide | C39H40FN5O3 | 645.31 |
| | A1M079 | 4-[5-[2-Fluoro-4-[4-[4-(propylcarbamoyl)phenyl]piperazine-1-carbonyl]phenyl]pyridin-2-yl]-1-hydroxy-N,N-dimethylnaphthalene-2-carboxamide | C39H38FN5O4 | 659.29 |
| | A1M008 | 6-[4-[[4-[4-[3-(Dimethylcarbamoyl)-4-hydroxynaphthalen-1-yl]phenyl]-2-methoxyphenyl]methyl]piperazin-1-yl]-N-propylpyridazine-3-carboxamide | C39H42N6O4 | 658.33 |
| | A1M080 | 1-Hydroxy-4-[5-[3-methoxy-4-[[4-[4-(propylcarbamoyl)phenyl]piperazin-1-yl]methyl]phenyl]pyridin-2-yl]-N,N-dimethylnaphthalene-2-carboxamide | C40H43N5O4 | 657.33 |
| | A1M081 | 4-[5-[3,5-Dimethoxy-4-[[4-[4-(propylcarbamoyl)phenyl]piperazin-1-yl]methyl]phenyl]pyridin-2-yl]-1-hydroxy-N,N-dimethylnaphthalene-2-carboxamide | C41H45N5O5 | 687.34 |
| | A1M082 | 1-Hydroxy-N,N-dimethyl-4-[5-[4-[[4-[4-(propylcarbamoyl)phenyl]piperazin-1-yl]methyl]naphthalen-1-yl]pyridin-2-yl]naphthalene-2-carboxamide | C43H43N5O3 | 677.34 |
| | A1M083 | 1-Hydroxy-N,N-dimethyl-4-[5-[4-[4-[4-(propylcarbamoyl)phenyl]piperazine-1-carbonyl]naphthalen-1-yl]pyridin-2-yl]naphthalene-2-carboxamide | C43H41N5O4 | 691.32 |
| | A1M084 | 1-Hydroxy-N,N-dimethyl-4-[5-[5-[[4-[4-(propylcarbamoyl)phenyl]piperazin-1-yl]methyl]thiophen-3-yl]pyridin-2-yl]naphthalene-2-carboxamide | C37H39N5O3S | 633.28 |
| | A1M085 | 1-Hydroxy-N,N-dimethyl-4-[5-[5-[4-[4-(propylcarbamoyl)phenyl]piperazine-1-carbonyl]thiophen-3-yl]pyridin-2-yl]naphthalene-2-carboxamide | C37H37N5O4S | 647.26 |
| | A1M086 | 1-Hydroxy-N,N-dimethyl-4-[5-[3-[[4-[4-(propylcarbamoyl)phenyl]piperazin-1-yl]methyl]-5-(trifluoromethyl)phenyl]pyridin-2-yl]naphthalene-2-carboxamide | C40H40F3N5O3 | 695.31 |
| | A1M087 | 1-Hydroxy-N,N-dimethyl-4-[5-[3-[4-[4-(propylcarbamoyl)phenyl]piperazine-1-carbonyl]-5-(trifluoromethyl)phenyl]pyridin-2-yl]naphthalene-2-carboxamide | C40H38F3N5O4 | 709.29 |

| | | | | |
|---|---|---|---|---|
| | A1M088 | 1-Hydroxy-N,N-dimethyl-4-[5-[3-[[4-[4-(propylcarbamoyl)phenyl]piperazin-1-yl]methyl]-5-(trifluoromethoxy)phenyl]pyridin-2-yl]naphthalene-2-carboxamide | C40H40F3N5O4 | 711.3 |
| | A1M089 | 4-[5-[3-Ethoxy-5-[[4-[4-(propylcarbamoyl)phenyl]piperazin-1-yl]methyl]phenyl]pyridin-2-yl]-1-hydroxy-N,N-dimethylnaphthalene-2-carboxamide | C41H45N5O4 | 671.35 |
| mixED 06 | A1M009 | 6-[4-[[4-[4-[3-(Dimethylcarbamoyl)-4-hydroxynaphthalen-1-yl]phenyl]-2,6-dimethoxyphenyl]methyl]piperazin-1-yl]-N-propylpyridazine-3-carboxamide | C40H44N6O5 | 688.34 |
| | A1M090 | 1-Hydroxy-N,N-dimethyl-4-[5-[3-propan-2-yloxy-5-[[4-[4-(propylcarbamoyl)phenyl]piperazin-1-yl]methyl]phenyl]pyridin-2-yl]naphthalene-2-carboxamide | C42H47N5O4 | 685.36 |
| | A1M091 | 4-[5-[3-tert-Butyl-5-[4-[4-(propylcarbamoyl)phenyl]piperazine-1-carbonyl]phenyl]pyridin-2-yl]-1-hydroxy-N,N-dimethylnaphthalene-2-carboxamide | C43H47N5O4 | 697.36 |
| | A1M092 | 4-[5-[3-Fluoro-4-[1-[4-[4-(propylcarbamoyl)phenyl]piperazin-1-yl]ethyl]phenyl]pyridin-2-yl]-1-hydroxy-N,N-dimethylnaphthalene-2-carboxamide | C40H42FN5O3 | 659.33 |
| | A1M093 | 1-Hydroxy-N,N-dimethyl-4-[5-[2-[2-[[4-[4-(propylcarbamoyl)phenyl]piperazin-1-yl]methyl]phenyl]ethynyl]pyridin-2-yl]naphthalene-2-carboxamide | C41H41N5O3 | 651.32 |
| | A1M094 | 1-Hydroxy-N,N-dimethyl-4-[5-[2-[3-[4-[4-(propylcarbamoyl)phenyl]piperazine-1-carbonyl]-5-(trifluoromethyl)phenyl]ethynyl]pyridin-2-yl]naphthalene-2-carboxamide | C42H38F3N5O4 | 733.29 |
| | A1M095 | 1-Hydroxy-N,N-dimethyl-4-[5-[2-[5-[4-[4-(propylcarbamoyl)phenyl]piperazine-1-carbonyl]pyridin-3-yl]ethynyl]pyridin-2-yl]naphthalene-2-carboxamide | C40H38N6O4 | 666.3 |
| | A1M096 | 1-Hydroxy-N,N-dimethyl-4-[5-[1-[3-[4-[4-(propylcarbamoyl)phenyl]piperazine-1-carbonyl]-5-(trifluoromethyl)phenyl]pyrazol-4-yl]pyridin-2-yl]naphthalene-2-carboxamide | C43H40F3N7O4 | 775.31 |
| | A1M097 | 6-[4-[2-[2-[4-[3-(Dimethylcarbamoyl)-4-hydroxynaphthalen-1-yl]phenyl]ethynyl]benzoyl]piperazin-1-yl]-N-propylpyridazine-3-carboxamide | C40H38N6O4 | 666.295 |
| | A1M098 | 6-[4-[2-[2-[6-[3-(Dimethylcarbamoyl)-4-hydroxynaphthalen-1-yl]pyridin-3-yl]ethynyl]benzoyl]piperazin-1-yl]-N-propylpyridazine-3-carboxamide | C39H37N7O4 | 667.291 |
| | A1M099 | 1-Hydroxy-N,N-dimethyl-4-[4-[2-[2-[4-[4-(propylcarbamoyl)phenyl]piperazine-1-carbonyl]phenyl]ethynyl]phenyl]naphthalene-2-carboxamide | C42H40N4O4 | 664.305 |
| | A1M100 | 1-Hydroxy-N,N-dimethyl-4-[5-[2-[2-[4-[4-(propylcarbamoyl)phenyl]piperazine-1-carbonyl]phenyl]ethynyl]pyridin-2-yl]naphthalene-2-carboxamide | C41H39N5O4 | 665.3 |

[Table 122]

[Table EE03]

Table EE03

| Mixture No. | No. of compound that may be contained | Compound name | Compositional formula | Exact MS |
|---|---|---|---|---|
| mixED 07 | A1K001 | 6-[4-[[2-[4-[3-(Dimethylcarbamoyl)-4-hydroxynaphthalen-1-yl]phenyl]phenyl]methyl]piperazin-1-yl]-N-(2,2,2-trifluoroethyl)pyridazine-3-carboxamide | C37H35F3N6O3 | 668.27 |
| | A1K010 | 6-[4-[[4-[4-[3-(Dimethylcarbamoyl)-4-hydroxynaphthalen-1-yl]phenyl]naphthalen-1-yl]methyl]piperazin-1-yl]-N-(2,2,2-trifluoroethyl)pyridazine-3-carboxamide | C41H37F3N6O3 | 718.29 |
| | A1K011 | 6-[4-[4-[4-[3-(Dimethylcarbamoyl)-4-hydroxynaphthalen-1-yl]phenyl]naphthalene-1-carbonyl]piperazin-1-yl]-N-(2,2,2-trifluoroethyl)pyridazine-3-carboxamide | C41H35F3N6O4 | 732.27 |
| | A1K012 | 6-[4-[[4-[4-[3-(Dimethylcarbamoyl)-4-hydroxynaphthalen-1-yl]phenyl]thiophen-2-yl]methyl]piperazin-1-yl]-N-(2,2,2-trifluoroethyl)pyridazine-3-carboxamide | C35H33F3N6O3S | 674.23 |
| | A1K013 | 6-[4-[4-[4-[3-(Dimethylcarbamoyl)-4-hydroxynaphthalen-1-yl]phenyl]thiophene-2-carbonyl]piperazin-1-yl]-N-(2,2,2-trifluoroethyl)pyridazine-3-carboxamide | C35H31F3N6O4S | 688.21 |
| | A1K014 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-(2,2,2-trifluoroethyl)pyridazine-3-carboxamide | C38H34F6N6O3 | 736.26 |
| | A1K015 | 6-[4-[3-[4-[3-(Dimethylcarbamoyl)-4-hydroxynaphthalen-1-yl]phenyl]-5-(trifluoromethyl)benzoyl]piperazin-1-yl]-N-(2,2,2-trifluoroethyl)pyridazine-3-carboxamide | C38H32F6N6O4 | 750.24 |
| | A1K016 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-(2,2,2-trifluoroethyl)pyridazine-3-carboxamide | C38H34F6N6O4 | 752.25 |

| | | | | |
|---|---|---|---|---|
| | A1K017 | 6-[4-[[3-[4-[3-(Dimethylcarbamoyl)-4-hydroxynaphthalen-1-yl]phenyl]-5-ethoxyphenyl]methyl]piperazin-1-yl]-N-(2,2,2-trifluoroethyl)pyridazine-3-carboxamide | C39H39F3N6O4 | 712.3 |
| | A1K018 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-(2,2,2-trifluoroethyl)pyridazine-3-carboxamide | C40H41F3N6O4 | 726.31 |
| mixED 07 | A1K019 | 6-[4-[3-tert-Butyl-5-[4-[3-(dimethylcarbamoyl)-4-hydroxynaphthalen-1-yl]phenyl]benzoyl]piperazin-1-yl]-N-(2,2,2-trifluoroethyl)pyridazine-3-carboxamide | C41H41F3N6O4 | 738.31 |
| | A1K002 | 6-[4-[3-[2-[4-[3-(Dimethylcarbamoyl)-4-hydroxynaphthalen-1-yl]phenyl]phenyl]propanoyl]piperazin-1-yl]-N-(2,2,2-trifluoroethyl)pyridazine-3-carboxamide | C39H37F3N6O4 | 710.28 |
| | A1K020 | 6-[4-[[2-[2-[4-[3-(Dimethylcarbamoyl)-4-hydroxynaphthalen-1-yl]phenyl]ethynyl]phenyl]methyl]piperazin-1-yl]-N-(2,2,2-trifluoroethyl)pyridazine-3-carboxamide | C39H35F3N6O3 | 692.27 |
| | A1K021 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-(2,2,2-trifluoroethyl)pyridazine-3-carboxamide | C40H32F6N6O4 | 774.24 |
| | A1K022 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-(2,2,2-trifluoroethyl)pyridazine-3-carboxamide | C38H32F3N7O4 | 707.25 |
| | A1K023 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-(2,2,2-trifluoroethyl)pyridazine-3-carboxamide | C41H34F6N8O4 | 816.26 |
| | A1K024 | 6-[4-[[2-[6-[3-(Dimethylcarbamoyl)-4-hydroxynaphthalen-1-yl]pyridin-3-yl]phenyl]methyl]piperazin-1-yl]-N-(2,2,2-trifluoroethyl)pyridazine-3-carboxamide | C36H34F3N7O3 | 669.27 |
| | A1K025 | 6-[4-[3-[2-[6-[3-(Dimethylcarbamoyl)-4-hydroxynaphthalen-1-yl]pyridin-3-yl]phenyl]propanoyl]piperazin-1-yl]-N-(2,2,2-trifluoroethyl)pyridazine-3-carboxamide | C38H36F3N7O4 | 711.28 |
| | A1K026 | 6-[4-[[4-[6-[3-(Dimethylcarbamoyl)-4-hydroxynaphthalen-1-yl]pyridin-3-yl]-3-methylphenyl]methyl]piperazin-1-yl]-N-(2,2,2-trifluoroethyl)pyridazine-3-carboxamide | C37H36F3N7O3 | 683.28 |
| | A1K027 | 6-[4-[4-[6-[3-(Dimethylcarbamoyl)-4-hydroxynaphthalen-1-yl]pyridin-3-yl]-3-methylbenzoyl]piperazin-1-yl]-N-(2,2,2-trifluoroethyl)pyridazine-3-carboxamide | C37H34F3N7O4 | 697.26 |
| | A1K028 | 6-[4-[[4-[6-[3-(Dimethylcarbamoyl)-4-hydroxynaphthalen-1-yl]pyridin-3-yl]-2-ethylphenyl]methyl]piperazin-1-yl]-N-(2,2,2-trifluoroethyl)pyridazine-3-carboxamide | C38H38F3N7O3 | 697.3 |
| | A1K029 | 6-[4-[[4-[6-[3-(Dimethylcarbamoyl)-4-hydroxynaphthalen-1-yl]pyridin-3-yl]-3-fluorophenyl]methyl]piperazin-1-yl]-N-(2,2,2-trifluoroethyl)pyridazine-3-carboxamide | C36H33F4N7O3 | 687.26 |
| | A1K003 | 6-[4-[[4-[4-[3-(Dimethylcarbamoyl)-4-hydroxynaphthalen-1-yl]phenyl]-3-methylphenyl]methyl]piperazin-1-yl]-N-(2,2,2-trifluoroethyl)pyridazine-3-carboxamide | C38H37F3N6O3 | 682.29 |
| | A1K030 | 6-[4-[4-[6-[3-(Dimethylcarbamoyl)-4-hydroxynaphthalen-1-yl]pyridin-3-yl]-3-fluorobenzoyl]piperazin-1-yl]-N-(2,2,2-trifluoroethyl)pyridazine-3-carboxamide | C36H31F4N7O4 | 701.24 |
| | A1K031 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-(2,2,2-trifluoroethyl)pyridazine-3-carboxamide | C37H36F3N7O4 | 699.28 |
| | A1K032 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-(2,2,2-trifluoroethyl)pyridazine-3-carboxamide | C38H38F3N7O5 | 729.29 |
| | A1K033 | 6-[4-[[4-[6-[3-(Dimethylcarbamoyl)-4-hydroxynaphthalen-1-yl]pyridin-3-yl]naphthalen-1-yl]methyl]piperazin-1-yl]-N-(2,2,2-trifluoroethyl)pyridazine-3-carboxamide | C40H36F3N7O3 | 719.28 |
| | A1K034 | 6-[4-[4-[6-[3-(Dimethylcarbamoyl)-4-hydroxynaphthalen-1-yl]pyridin-3-yl]naphthalene-1-carbonyl]piperazin-1-yl]-N-(2,2,2-trifluoroethyl)pyridazine-3-carboxamide | C40H34F3N7O4 | 733.26 |
| | A1K035 | 6-[4-[[4-[6-[3-(Dimethylcarbamoyl)-4-hydroxynaphthalen-1-yl]pyridin-3-yl]thiophen-2-yl]methyl]piperazin-1-yl]-N-(2,2,2-trifluoroethyl)pyridazine-3-carboxamide | C34H32F3N7O3S | 675.22 |
| | A1K036 | 6-[4-[4-[6-[3-(Dimethylcarbamoyl)-4-hydroxynaphthalen-1-yl]pyridin-3-yl]thiophene-2-carbonyl]piperazin-1-yl]-N-(2,2,2-trifluoroethyl)pyridazine-3-carboxamide | C34H30F3N7O4S | 689.2 |
| | A1K037 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-(2,2,2-trifluoroethyl)pyridazine-3-carboxamide | C37H33F6N7O3 | 737.25 |
| | A1K038 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-(2,2,2-trifluoroethyl)pyridazine-3-carboxamide | C37H31F6N7O4 | 751.23 |

| | | | | |
|---|---|---|---|---|
| | A1K039 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-(2,2,2-trifluoroethyl)pyridazine-3-carboxamide | C37H33F6N7O4 | 753.25 |

[Table 123]

| | | | | |
|---|---|---|---|---|
| mixED 07 | A1K004 | 6-[4-[4-[4-[3-(Dimethylcarbamoyl)-4-hydroxynaphthalen-1-yl]phenyl]-3-methylbenzoyl]piperazin-1-yl]-N-(2,2,2-trifluoroethyl)pyridazine-3-carboxamide | C38H35F3N6O4 | 696.27 |
| | A1K040 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-(2,2,2-trifluoroethyl)pyridazine-3-carboxamide | C38H38F3N7O4 | 713.29 |
| | A1K041 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-(2,2,2-trifluoroethyl)pyridazine-3-carboxamide | C39H40F3N7O4 | 727.31 |
| | A1K042 | 6-[4-[3-tert-Butyl-5-[6-[3-(dimethylcarbamoyl)-4-hydroxynaphthalen-1-yl]pyridin-3-yl]benzoyl]piperazin-1-yl]-N-(2,2,2-trifluoroethyl)pyridazine-3-carboxamide | C40H40F3N7O4 | 739.31 |
| | A1K043 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-(2,2,2-trifluoroethyl)pyridazine-3-carboxamide | C38H34F3N7O3 | 693.27 |
| | A1K044 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-(2,2,2-trifluoroethyl)pyridazine-3-carboxamide | C39H31F6N7O4 | 775.23 |
| | A1K045 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-(2,2,2-trifluoroethyl)pyridazine-3-carboxamide | C37H31F3N8O4 | 708.24 |
| | A1K046 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-(2,2,2-trifluoroethyl)pyridazine-3-carboxamide | C40H33F6N9O4 | 817.26 |
| | A1K047 | 1-Hydroxy-N,N-dimethyl-4-[4-[2-[[4-[4-(2,2,2-trifluoroethylcarbamoyl)phenyl]piperazin-1-yl]methyl]phenyl]phenyl]naphthalene-2-carboxamide | C39H37F3N4O3 | 666.28 |
| | A1K048 | 1-Hydroxy-N,N-dimethyl-4-[4-[2-[3-oxo-3-[4-[4-(2,2,2-trifluoroethylcarbamoyl)phenyl]piperazin-1-yl]propyl]phenyl]phenyl]naphthalene-2-carboxamide | C41H39F3N4O4 | 708.29 |
| | A1K049 | 1-Hydroxy-N,N-dimethyl-4-[4-[4-[1-[4-[4-(2,2,2-trifluoroethylcarbamoyl)phenyl]piperazin-1-yl]ethyl]phenyl]phenyl]naphthalene-2-carboxamide | C40H39F3N4O3 | 680.3 |
| | A1K005 | 6-[4-[[4-[4-[3-(Dimethylcarbamoyl)-4-hydroxynaphthalen-1-yl]phenyl]-2-ethylphenyl]methyl]piperazin-1-yl]-N-(2,2,2-trifluoroethyl)pyridazine-3-carboxamide | C39H39F3N6O3 | 696.3 |
| | A1K050 | 1-Hydroxy-N,N-dimethyl-4-[4-[2-methyl-4-[[4-[4-(2,2,2-trifluoroethylcarbamoyl)phenyl]piperazin-1-yl]methyl]phenyl]phenyl]naphthalene-2-carboxamide | C40H39F3N4O3 | 680.3 |
| | A1K051 | 1-Hydroxy-N,N-dimethyl-4-[4-[2-methyl-4-[4-[4-(2,2,2-trifluoroethylcarbamoyl)phenyl]piperazine-1-carbonyl]phenyl]phenyl]naphthalene-2-carboxamide | C40H37F3N4O4 | 694.28 |
| | A1K052 | 4-[4-[3-Ethyl-4-[[4-[4-(2,2,2-trifluoroethylcarbamoyl)phenyl]piperazin-1-yl]methyl]phenyl]phenyl]-1-hydroxy-N,N-dimethylnaphthalene-2-carboxamide | C41H41F3N4O3 | 694.31 |
| | A1K053 | 4-[4-[2-Fluoro-4-[[4-[4-(2,2,2-trifluoroethylcarbamoyl)phenyl]piperazin-1-yl]methyl]phenyl]phenyl]-1-hydroxy-N,N-dimethylnaphthalene-2-carboxamide | C39H36F4N4O3 | 684.27 |
| | A1K054 | 4-[4-[2-Fluoro-4-[4-[4-(2,2,2-trifluoroethylcarbamoyl)phenyl]piperazine-1-carbonyl]phenyl]phenyl]-1-hydroxy-N,N-dimethylnaphthalene-2-carboxamide | C39H34F4N4O4 | 698.25 |
| | A1K055 | 1-Hydroxy-4-[4-[3-methoxy-4-[[4-[4-(2,2,2-trifluoroethylcarbamoyl)phenyl]piperazin-1-yl]methyl]phenyl]phenyl]-N,N-dimethylnaphthalene-2-carboxamide | C40H39F3N4O4 | 696.29 |
| | A1K056 | 4-[4-[3,5-Dimethoxy-4-[[4-[4-(2,2,2-trifluoroethylcarbamoyl)phenyl]piperazin-1-yl]methyl]phenyl]phenyl]-1-hydroxy-N,N-dimethylnaphthalene-2-carboxamide | C41H41F3N4O5 | 726.3 |
| | A1K057 | 1-Hydroxy-N,N-dimethyl-4-[4-[4-[[4-[4-(2,2,2-trifluoroethylcarbamoyl)phenyl]piperazin-1-yl]methyl]naphthalen-1-yl]phenyl]naphthalene-2-carboxamide | C43H39F3N4O3 | 716.3 |
| | A1K058 | 1-Hydroxy-N,N-dimethyl-4-[4-[4-[4-[4-(2,2,2-trifluoroethylcarbamoyl)phenyl]piperazine-1-carbonyl]naphthalen-1-yl]phenyl]naphthalene-2-carboxamide | C43H37F3N4O4 | 730.28 |

| | | | | |
|---|---|---|---|---|
| | A1K059 | 1-Hydroxy-N,N-dimethyl-4-[4-[5-[[4-[4-(2,2,2-trifluoroethylcarbamoyl)phenyl]piperazin-1-yl]methyl]thiophen-3-yl]phenyl]naphthalene-2-carboxamide | C37H35F3N4O3S | 672.24 |
| | A1K006 | 6-[4-[[4-[4-[3-(Dimethylcarbamoyl)-4-hydroxynaphthalen-1-yl]phenyl]-3-fluorophenyl]methyl]piperazin-1-yl]-N-(2,2,2-trifluoroethyl)pyridazine-3-carboxamide | C37H34F4N6O3 | 686.26 |
| | A1K060 | 1-Hydroxy-N,N-dimethyl-4-[4-[5-[4-[4-(2,2,2-trifluoroethylcarbamoyl)phenyl]piperazine-1-carbonyl]thiophen-3-yl]phenyl]naphthalene-2-carboxamide | C37H33F3N4O4S | 686.22 |
| | A1K061 | 1-Hydroxy-N,N-dimethyl-4-[4-[3-[[4-[4-(2,2,2-trifluoroethylcarbamoyl)phenyl]piperazin-1-yl]methyl]-5-(trifluoromethyl)phenyl]phenyl]naphthalene-2-carboxamide | C40H36F6N4O3 | 734.27 |
| mixED 07 | A1K062 | 1-Hydroxy-N,N-dimethyl-4-[4-[3-[4-[4-(2,2,2-trifluoroethylcarbamoyl)phenyl]piperazine-1-carbonyl]-5-(trifluoromethyl)phenyl]phenyl]naphthalene-2-carboxamide | C40H34F6N4O4 | 748.25 |
| | A1K063 | 1-Hydroxy-N,N-dimethyl-4-[4-[3-[[4-[4-(2,2,2-trifluoroethylcarbamoyl)phenyl]piperazin-1-yl]methyl]-5-(trifluoromethoxy)phenyl]phenyl]naphthalene-2-carboxamide | C40H36F6N4O4 | 750.26 |
| | A1K064 | 4-[4-[3-Ethoxy-5-[[4-[4-(2,2,2-trifluoroethylcarbamoyl)phenyl]piperazin-1-yl]methyl]phenyl]phenyl]-1-hydroxy-N,N-dimethylnaphthalene-2-carboxamide | C41H41F3N4O4 | 710.31 |
| | A1K065 | 1-Hydroxy-N,N-dimethyl-4-[4-[3-propan-2-yloxy-5-[[4-[4-(2,2,2-trifluoroethylcarbamoyl)phenyl]piperazin-1-yl]methyl]phenyl]phenyl]naphthalene-2-carboxamide | C42H43F3N4O4 | 724.32 |
| | A1K066 | 4-[4-[3-tert-Butyl-5-[4-[4-(2,2,2-trifluoroethylcarbamoyl)phenyl]piperazine-1-carbonyl]phenyl]phenyl]-1-hydroxy-N,N-dimethylnaphthalene-2-carboxamide | C43H43F3N4O4 | 736.32 |
| | A1K067 | 4-[4-[3-Fluoro-4-[1-[4-[4-(2,2,2-trifluoroethylcarbamoyl)phenyl]piperazin-1-yl]ethyl]phenyl]phenyl]-1-hydroxy-N,N-dimethylnaphthalene-2-carboxamide | C40H38F4N4O3 | 698.29 |
| | A1K068 | 1-Hydroxy-N,N-dimethyl-4-[4-[2-[2-[[4-[4-(2,2,2-trifluoroethylcarbamoyl)phenyl]piperazin-1-yl]methyl]phenyl]ethynyl]phenyl]naphthalene-2-carboxamide | C41H37F3N4O3 | 690.28 |
| | A1K069 | 1-Hydroxy-N,N-dimethyl-4-[4-[2-[3-[4-[4-(2,2,2-trifluoroethylcarbamoyl)phenyl]piperazine-1-carbonyl]-5-(trifluoromethyl)phenyl]ethynyl]phenyl]naphthalene-2-carboxamide | C42H34F6N4O4 | 772.25 |

[Table 124]

| | | | | |
|---|---|---|---|---|
| mixED 07 | A1K007 | 6-[4-[4-[4-[3-(Dimethylcarbamoyl)-4-hydroxynaphthalen-1-yl]phenyl]-3-fluorobenzoyl]piperazin-1-yl]-N-(2,2,2-trifluoroethyl)pyridazine-3-carboxamide | C37H32F4N6O4 | 700.24 |
| | A1K070 | 1-Hydroxy-N,N-dimethyl-4-[4-[2-[5-[4-[4-(2,2,2-trifluoroethylcarbamoyl)phenyl]piperazine-1-carbonyl]pyridin-3-yl]ethynyl]phenyl]naphthalene-2-carboxamide | C40H34F3N5O4 | 705.26 |
| | A1K071 | 1-Hydroxy-N,N-dimethyl-4-[4-[1-[3-[4-[4-(2,2,2-trifluoroethylcarbamoyl)phenyl]piperazine-1-carbonyl]-5-(trifluoromethyl)phenyl]pyrazol-4-yl]phenyl]naphthalene-2-carboxamide | C43H36F6N6O4 | 814.27 |
| | A1K072 | 1-Hydroxy-N,N-dimethyl-4-[5-[2-[[4-[4-(2,2,2-trifluoroethylcarbamoyl)phenyl]piperazin-1-yl]methyl]phenyl]pyridin-2-yl]naphthalene-2-carboxamide | C38H36F3N5O3 | 667.28 |
| | A1K073 | 1-Hydroxy-N,N-dimethyl-4-[5-[2-[3-oxo-3-[4-[4-(2,2,2-trifluoroethylcarbamoyl)phenyl]piperazin-1-yl]propyl]phenyl]pyridin-2-yl]naphthalene-2-carboxamide | C40H38F3N5O4 | 709.29 |
| | A1K074 | 1-Hydroxy-N,N-dimethyl-4-[5-[4-[1-[4-[4-(2,2,2-trifluoroethylcarbamoyl)phenyl]piperazin-1-yl]ethyl]phenyl]pyridin-2-yl]naphthalene-2-carboxamide | C39H38F3N5O3 | 681.29 |
| | A1K075 | 1-Hydroxy-N,N-dimethyl-4-[5-[2-methyl-4-[[4-[4-(2,2,2-trifluoroethylcarbamoyl)phenyl]piperazin-1-yl]methyl]phenyl]pyridin-2-yl]naphthalene-2-carboxamide | C39H38F3N5O3 | 681.29 |
| | A1K076 | 1-Hydroxy-N,N-dimethyl-4-[5-[2-methyl-4-[4-[4-(2,2,2-trifluoroethylcarbamoyl)phenyl]piperazine-1-carbonyl]phenyl]pyridin-2-yl]naphthalene-2-carboxamide | C39H36F3N5O4 | 695.27 |
| | A1K077 | 4-[5-[3-Ethyl-4-[[4-[4-(2,2,2-trifluoroethylcarbamoyl)phenyl]piperazin-1-yl]methyl]phenyl]pyridin-2-yl]-1-hydroxy-N,N-dimethylnaphthalene-2-carboxamide | C40H40F3N5O3 | 695.31 |
| | A1K078 | 4-[5-[2-Fluoro-4-[[4-[4-(2,2,2-trifluoroethylcarbamoyl)phenyl]piperazin-1-yl]methyl]phenyl]pyridin-2-yl]-1-hydroxy-N,N-dimethylnaphthalene-2-carboxamide | C38H35F4N5O3 | 685.27 |

| | | | | |
|---|---|---|---|---|
| | A1K079 | 4-[5-[2-Fluoro-4-[4-[4-(2,2,2-trifluoroethylcarbamoyl)phenyl]piperazine-1-carbonyl]phenyl]pyridin-2-yl]-1-hydroxy-N,N-dimethylnaphthalene-2-carboxamide | C38H33F4N5O4 | 699.25 |
| | A1K008 | 6-[4-[[4-[4-[3-(Dimethylcarbamoyl)-4-hydroxynaphthalen-1-yl]phenyl]-2-methoxyphenyl]methyl]piperazin-1-yl]-N-(2,2,2-trifluoroethyl)pyridazine-3-carboxamide | C38H37F3N6O4 | 698.28 |
| | A1K080 | 1-Hydroxy-4-[5-[3-methoxy-4-[[4-[4-(2,2,2-trifluoroethylcarbamoyl)phenyl]piperazin-1-yl]methyl]phenyl]pyridin-2-yl]-N,N-dimethylnaphthalene-2-carboxamide | C39H38F3N5O4 | 697.29 |
| | A1K081 | 4-[5-[3,5-Dimethoxy-4-[[4-[4-(2,2,2-trifluoroethylcarbamoyl)phenyl]piperazin-1-yl]methyl]phenyl]pyridin-2-yl]-1-hydroxy-N,N-dimethylnaphthalene-2-carboxamide | C40H40F3N5O5 | 727.3 |
| | A1K082 | 1-Hydroxy-N,N-dimethyl-4-[5-[4-[[4-[4-(2,2,2-trifluoroethylcarbamoyl)phenyl]piperazin-1-yl]methyl]naphthalen-1-yl]pyridin-2-yl]naphthalene-2-carboxamide | C42H38F3N5O3 | 717.29 |
| mixED 07 | A1K083 | 1-Hydroxy-N,N-dimethyl-4-[5-[4-[4-[4-(2,2,2-trifluoroethylcarbamoyl)phenyl]piperazine-1-carbonyl]naphthalen-1-yl]pyridin-2-yl]naphthalene-2-carboxamide | C42H36F3N5O4 | 731.27 |
| | A1K084 | 1-Hydroxy-N,N-dimethyl-4-[5-[5-[[4-[4-(2,2,2-trifluoroethylcarbamoyl)phenyl]piperazin-1-yl]methyl]thiophen-3-yl]pyridin-2-yl]naphthalene-2-carboxamide | C36H34F3N5O3S | 673.23 |
| | A1K085 | 1-Hydroxy-N,N-dimethyl-4-[5-[5-[4-[4-(2,2,2-trifluoroethylcarbamoyl)phenyl]piperazine-1-carbonyl]thiophen-3-yl]pyridin-2-yl]naphthalene-2-carboxamide | C36H32F3N5O4S | 687.21 |
| | A1K086 | 1-Hydroxy-N,N-dimethyl-4-[5-[3-[[4-[4-(2,2,2-trifluoroethylcarbamoyl)phenyl]piperazin-1-yl]methyl]-5-(trifluoromethyl)phenyl]pyridin-2-yl]naphthalene-2-carboxamide | C39H35F6N5O3 | 735.26 |
| | A1K087 | 1-Hydroxy-N,N-dimethyl-4-[5-[3-[4-[4-(2,2,2-trifluoroethylcarbamoyl)phenyl]piperazine-1-carbonyl]-5-(trifluoromethyl)phenyl]pyridin-2-yl]naphthalene-2-carboxamide | C39H33F6N5O4 | 749.24 |
| | A1K088 | 1-Hydroxy-N,N-dimethyl-4-[5-[3-[[4-[4-(2,2,2-trifluoroethylcarbamoyl)phenyl]piperazin-1-yl]methyl]-5-(trifluoromethoxy)phenyl]pyridin-2-yl]naphthalene-2-carboxamide | C39H35F6N5O4 | 751.26 |
| | A1K089 | 4-[5-[3-Ethoxy-5-[[4-[4-(2,2,2-trifluoroethylcarbamoyl)phenyl]piperazin-1-yl]methyl]phenyl]pyridin-2-yl]-1-hydroxy-N,N-dimethylnaphthalene-2-carboxamide | C40H40F3N5O4 | 711.3 |
| | A1K009 | 6-[4-[[4-[4-[3-(Dimethylcarbamoyl)-4-hydroxynaphthalen-1-yl]phenyl]-2,6-dimethoxyphenyl]methyl]piperazin-1-yl]-N-(2,2,2-trifluoroethyl)pyridazine-3-carboxamide | C39H39F3N6O5 | 728.29 |
| | A1K090 | 1-Hydroxy-N,N-dimethyl-4-[5-[3-propan-2-yloxy-5-[[4-[4-(2,2,2-trifluoroethylcarbamoyl)phenyl]piperazin-1-yl]methyl]phenyl]pyridin-2-yl]naphthalene-2-carboxamide | C41H42F3N5O4 | 725.32 |
| | A1K091 | 4-[5-[3-tert-Butyl-5-[4-[4-(2,2,2-trifluoroethylcarbamoyl)phenyl]piperazine-1-carbonyl]phenyl]pyridin-2-yl]-1-hydroxy-N,N-dimethylnaphthalene-2-carboxamide | C42H42F3N5O4 | 737.32 |
| | A1K092 | 4-[5-[3-Fluoro-4-[1-[4-[4-(2,2,2-trifluoroethylcarbamoyl)phenyl]piperazin-1-yl]ethyl]phenyl]pyridin-2-yl]-1-hydroxy-N,N-dimethylnaphthalene-2-carboxamide | C39H37F4N5O3 | 699.28 |
| | A1K093 | 1-Hydroxy-N,N-dimethyl-4-[5-[2-[2-[[4-[4-(2,2,2-trifluoroethylcarbamoyl)phenyl]piperazin-1-yl]methyl]phenyl]ethynyl]pyridin-2-yl]naphthalene-2-carboxamide | C40H36F3N5O3 | 691.28 |
| | A1K094 | 1-Hydroxy-N,N-dimethyl-4-[5-[2-[3-[4-[4-(2,2,2-trifluoroethylcarbamoyl)phenyl]piperazine-1-carbonyl]-5-(trifluoromethyl)phenyl]ethynyl]pyridin-2-yl]naphthalene-2-carboxamide | C41H33F6N5O4 | 773.24 |
| | A1K095 | 1-Hydroxy-N,N-dimethyl-4-[5-[2-[5-[4-[4-(2,2,2-trifluoroethylcarbamoyl)phenyl]piperazine-1-carbonyl]pyridin-3-yl]ethynyl]pyridin-2-yl]naphthalene-2-carboxamide | C39H33F3N6O4 | 706.25 |
| | A1K096 | 1-Hydroxy-N,N-dimethyl-4-[5-[1-[3-[4-[4-(2,2,2-trifluoroethylcarbamoyl)phenyl]piperazine-1-carbonyl]-5-(trifluoromethyl)phenyl]pyrazol-4-yl]pyridin-2-yl]naphthalene-2-carboxamide | C42H35F6N7O4 | 815.27 |
| | A1K097 | 6-[4-[2-[2-[4-[3-(Dimethylcarbamoyl)-4-hydroxynaphthalen-1-yl]phenyl]ethynyl]benzoyl]piperazin-1-yl]-N-(2,2,2-trifluoroethyl)pyridazine-3-carboxamide | C39H33F3N6O4 | 706.252 |
| | A1K098 | 6-[4-[2-[2-[6-[3-(Dimethylcarbamoyl)-4-hydroxynaphthalen-1-yl]pyridin-3-yl]ethynyl]benzoyl]piperazin-1-yl]-N-(2,2,2-trifluoroethyl)pyridazine-3-carboxamide | C38H32F3N7O4 | 707.247 |
| | A1K099 | 1-Hydroxy-N,N-dimethyl-4-[4-[2-[2-[4-[4-(2,2,2-trifluoroethylcarbamoyl)phenyl]piperazine-1-carbonyl]phenyl]ethynyl]phenyl]naphthalene-2-carboxamide | C41H35F3N4O4 | 704.261 |

| | | | | |
|---|---|---|---|---|
| | A1K100 | 1-Hydroxy-N,N-dimethyl-4-[5-[2-[2-[4-[4-(2,2,2-trifluoroethylcarbamoyl)phenyl]piperazine-1-carbonyl]phenyl]ethynyl]pyridin-2-yl]naphthalene-2-carboxamide | C40H34F3N5O4 | 705.256 |

[Table 125]

[Table EE04]

| Table EE04 | | | | |
|---|---|---|---|---|
| Mixture No. | No. of compound that may be contained | Compound name | Compositional formula | Exact MS |
| mixED 08 | A1O001 | 6-[4-[[2-[4-[3-(Dimethylcarbamoyl)-4-hydroxynaphthalen-1-yl]phenyl]phenyl]methyl]piperazin-1-yl]-N-(4-methoxyphenyl)pyridazine-3-carboxamide | C42H40N6O4 | 692.31 |
| | A1O010 | 6-[4-[[4-[4-[3-(Dimethylcarbamoyl)-4-hydroxynaphthalen-1-yl]phenyl]naphthalen-1-yl]methyl]piperazin-1-yl]-N-(4-methoxyphenyl)pyridazine-3-carboxamide | C46H42N6O4 | 742.33 |
| | A1O011 | 6-[4-[4-[4-[3-(Dimethylcarbamoyl)-4-hydroxynaphthalen-1-yl]phenyl]naphthalene-1-carbonyl]piperazin-1-yl]-N-(4-methoxyphenyl)pyridazine-3-carboxamide | C46H40N6O5 | 756.31 |
| | A1O012 | 6-[4-[[4-[4-[3-(Dimethylcarbamoyl)-4-hydroxynaphthalen-1-yl]phenyl]thiophen-2-yl]methyl]piperazin-1-yl]-N-(4-methoxyphenyl)pyridazine-3-carboxamide | C40H38N6O4S | 698.27 |
| | A1O013 | 6-[4-[4-[4-[3-(Dimethylcarbamoyl)-4-hydroxynaphthalen-1-yl]phenyl]thiophene-2-carbonyl]piperazin-1-yl]-N-(4-methoxyphenyl)pyridazine-3-carboxamide | C40H36N6O5S | 712.25 |
| | A1O014 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-(4-methoxyphenyl)pyridazine-3-carboxamide | C43H39F3N6O4 | 760.3 |
| | A1O015 | 6-[4-[3-[4-[3-(Dimethylcarbamoyl)-4-hydroxynaphthalen-1-yl]phenyl]-5-(trifluoromethyl)benzoyl]piperazin-1-yl]-N-(4-methoxyphenyl)pyridazine-3-carboxamide | C43H37F3N6O5 | 774.28 |
| | A1O016 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-(4-methoxyphenyl)pyridazine-3-carboxamide | C43H39F3N6O5 | 776.29 |
| | A1O017 | 6-[4-[[3-[4-[3-(Dimethylcarbamoyl)-4-hydroxynaphthalen-1-yl]phenyl]-5-ethoxyphenyl]methyl]piperazin-1-yl]-N-(4-methoxyphenyl)pyridazine-3-carboxamide | C44H44N6O5 | 736.34 |
| | A1O018 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-(4-methoxyphenyl)pyridazine-3-carboxamide | C45H46N6O5 | 750.35 |
| | A1O019 | 6-[4-[3-tert-Butyl-5-[4-[3-(dimethylcarbamoyl)-4-hydroxynaphthalen-1-yl]phenyl]benzoyl]piperazin-1-yl]-N-(4-methoxyphenyl)pyridazine-3-carboxamide | C46H46N6O5 | 762.35 |
| | A1O002 | 6-[4-[3-[2-[4-[3-(Dimethylcarbamoyl)-4-hydroxynaphthalen-1-yl]phenyl]phenyl]propanoyl]piperazin-1-yl]-N-(4-methoxyphenyl)pyridazine-3-carboxamide | C44H42N6O5 | 734.32 |
| | A1O020 | 6-[4-[[2-[2-[4-[3-(Dimethylcarbamoyl)-4-hydroxynaphthalen-1-yl]phenyl]ethynyl]phenyl]methyl]piperazin-1-yl]-N-(4-methoxyphenyl)pyridazine-3-carboxamide | C44H40N6O4 | 716.31 |
| | A1O021 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-(4-methoxyphenyl)pyridazine-3-carboxamide | C45H37F3N6O5 | 798.28 |
| | A1O022 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-(4-methoxyphenyl)pyridazine-3-carboxamide | C43H37N7O5 | 731.29 |
| | A1O023 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-(4-methoxyphenyl)pyridazine-3-carboxamide | C46H39F3N8O5 | 840.3 |
| | A1O024 | 6-[4-[[2-[6-[3-(Dimethylcarbamoyl)-4-hydroxynaphthalen-1-yl]pyridin-3-yl]phenyl]methyl]piperazin-1-yl]-N-(4-methoxyphenyl)pyridazine-3-carboxamide | C41H39N7O4 | 693.31 |
| | A1O025 | 6-[4-[3-[2-[6-[3-(Dimethylcarbamoyl)-4-hydroxynaphthalen-1-yl]pyridin-3-yl]phenyl]propanoyl]piperazin-1-yl]-N-(4-methoxyphenyl)pyridazine-3-carboxamide | C43H41N7O5 | 735.32 |
| | A1O026 | 6-[4-[[4-[6-[3-(Dimethylcarbamoyl)-4-hydroxynaphthalen-1-yl]pyridin-3-yl]-3-methylphenyl]methyl]piperazin-1-yl]-N-(4-methoxyphenyl)pyridazine-3-carboxamide | C42H41N7O4 | 707.32 |
| | A1O027 | 6-[4-[4-[6-[3-(Dimethylcarbamoyl)-4-hydroxynaphthalen-1-yl]pyridin-3-yl]-3-methylbenzoyl]piperazin-1-yl]-N-(4-methoxyphenyl)pyridazine-3-carboxamide | C42H39N7O5 | 721.3 |

| | | | | |
|---|---|---|---|---|
| | A1O028 | 6-[4-[[4-[6-[3-(Dimethylcarbamoyl)-4-hydroxynaphthalen-1-yl]pyridin-3-yl]-2-ethylphenyl]methyl]piperazin-1-yl]-N-(4-methoxyphenyl)pyridazine-3-carboxamide | C43H43N7O4 | 721.34 |
| | A1O029 | 6-[4-[[4-[6-[3-(Dimethylcarbamoyl)-4-hydroxynaphthalen-1-yl]pyridin-3-yl]-3-fluorophenyl]methyl]piperazin-1-yl]-N-(4-methoxyphenyl)pyridazine-3-carboxamide | C41H38FN7O4 | 711.3 |
| | A1O003 | 6-[4-[[4-[4-[3-(Dimethylcarbamoyl)-4-hydroxynaphthalen-1-yl]phenyl]-3-methylphenyl]methyl]piperazin-1-yl]-N-(4-methoxyphenyl)pyridazine-3-carboxamide | C43H42N6O4 | 706.33 |
| mixED 08 | A1O030 | 6-[4-[4-[6-[3-(Dimethylcarbamoyl)-4-hydroxynaphthalen-1-yl]pyridin-3-yl]-3-fluorobenzoyl]piperazin-1-yl]-N-(4-methoxyphenyl)pyridazine-3-carboxamide | C41H36FN7O5 | 725.28 |
| | A1O031 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-(4-methoxyphenyl)pyridazine-3-carboxamide | C42H41N7O5 | 723.32 |
| | A1O032 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-(4-methoxyphenyl)pyridazine-3-carboxamide | C43H43N7O6 | 753.33 |
| | A1O033 | 6-[4-[[4-[6-[3-(Dimethylcarbamoyl)-4-hydroxynaphthalen-1-yl]pyridin-3-yl]naphthalen-1-yl]methyl]piperazin-1-yl]-N-(4-methoxyphenyl)pyridazine-3-carboxamide | C45H41N7O4 | 743.32 |
| | A1O034 | 6-[4-[4-[6-[3-(Dimethylcarbamoyl)-4-hydroxynaphthalen-1-yl]pyridin-3-yl]naphthalene-1-carbonyl]piperazin-1-yl]-N-(4-methoxyphenyl)pyridazine-3-carboxamide | C45H39N7O5 | 757.3 |
| | A1O035 | 6-[4-[[4-[6-[3-(Dimethylcarbamoyl)-4-hydroxynaphthalen-1-yl]pyridin-3-yl]thiophen-2-yl]methyl]piperazin-1-yl]-N-(4-methoxyphenyl)pyridazine-3-carboxamide | C39H37N7O4S | 699.26 |
| | A1O036 | 6-[4-[4-[6-[3-(Dimethylcarbamoyl)-4-hydroxynaphthalen-1-yl]pyridin-3-yl]thiophene-2-carbonyl]piperazin-1-yl]-N-(4-methoxyphenyl)pyridazine-3-carboxamide | C39H35N7O5S | 713.24 |
| | A1O037 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-(4-methoxyphenyl)pyridazine-3-carboxamide | C42H38F3N7O4 | 761.29 |
| | A1O038 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-(4-methoxyphenyl)pyridazine-3-carboxamide | C42H36F3N7O5 | 775.27 |
| | A1O039 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-(4-methoxyphenyl)pyridazine-3-carboxamide | C42H38F3N7O5 | 777.29 |

[Table 126]

| | | | | |
|---|---|---|---|---|
| mixED 08 | A1O004 | 6-[4-[4-[4-[3-(Dimethylcarbamoyl)-4-hydroxynaphthalen-1-yl]phenyl]-3-methylbenzoyl]piperazin-1-yl]-N-(4-methoxyphenyl)pyridazine-3-carboxamide | C43H40N6O5 | 720.31 |
| | A1O040 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-(4-methoxyphenyl)pyridazine-3-carboxamide | C43H43N7O5 | 737.33 |
| | A1O041 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-(4-methoxyphenyl)pyridazine-3-carboxamide | C44H45N7O5 | 751.35 |
| | A1O042 | 6-[4-[3-tert-Butyl-5-[6-[3-(dimethylcarbamoyl)-4-hydroxynaphthalen-1-yl]pyridin-3-yl]benzoyl]piperazin-1-yl]-N-(4-methoxyphenyl)pyridazine-3-carboxamide | C45H45N7O5 | 763.35 |
| | A1O043 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-(4-methoxyphenyl)pyridazine-3-carboxamide | C43H39N7O4 | 717.31 |
| | A1O044 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-(4-methoxyphenyl)pyridazine-3-carboxamide | C44H36F3N7O5 | 799.27 |
| | A1O045 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-(4-methoxyphenyl)pyridazine-3-carboxamide | C42H36N8O5 | 732.28 |
| | A1O046 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-(4-methoxyphenyl)pyridazine-3-carboxamide | C45H38F3N9O5 | 841.29 |
| | A1O047 | 1-Hydroxy-4-[4-[2-[[4-[4-[(4-methoxyphenyl)carbamoyl]phenyl]piperazin-1-yl]methyl]phenyl]phenyl]-N,N-dimethylnaphthalene-2-carboxamide | C44H42N4O4 | 690.32 |
| | A1O048 | 1-Hydroxy-4-[4-[2-[3-[4-[4-[(4-methoxyphenyl)carbamoyl]phenyl]piperazin-1-yl]-3-oxopropyl]phenyl]phenyl]-N,N-dimethylnaphthalene-2-carboxamide | C46H44N4O5 | 732.33 |
| | A1O049 | 1-Hydroxy-4-[4-[4-[1-[4-[4-[(4-methoxyphenyl)carbamoyl]phenyl]piperazin-1-yl]ethyl]phenyl]phenyl]-N,N-dimethylnaphthalene-2-carboxamide | C45H44N4O4 | 704.34 |

| | | | | |
|---|---|---|---|---|
| | A1O005 | 6-[4-[[4-[4-[3-(Dimethylcarbamoyl)-4-hydroxynaphthalen-1-yl]phenyl]-2-ethylphenyl]methyl]piperazin-1-yl]-N-(4-methoxyphenyl)pyridazine-3-carboxamide | C44H44N6O4 | 720.34 |
| | A1O050 | 1-Hydroxy-4-[4-[4-[[4-[4-[(4-methoxyphenyl)carbamoyl]phenyl]piperazin-1-yl]methyl]-2-methylphenyl]phenyl]-N,N-dimethylnaphthalene-2-carboxamide | C45H44N4O4 | 704.34 |
| | A1O051 | 1-Hydroxy-4-[4-[4-[4-[4-[(4-methoxyphenyl)carbamoyl]phenyl]piperazine-1-carbonyl]-2-methylphenyl]phenyl]-N,N-dimethylnaphthalene-2-carboxamide | C45H42N4O5 | 718.32 |
| mixED 08 | A1O052 | 4-[4-[3-Ethyl-4-[[4-[4-[(4-methoxyphenyl)carbamoyl]phenyl]piperazin-1-yl]methyl]phenyl]phenyl]-1-hydroxy-N,N-dimethylnaphthalene-2-carboxamide | C46H46N4O4 | 718.35 |
| | A1O053 | 4-[4-[2-Fluoro-4-[[4-[4-[(4-methoxyphenyl)carbamoyl]phenyl]piperazin-1-yl]methyl]phenyl]phenyl]-1-hydroxy-N,N-dimethylnaphthalene-2-carboxamide | C44H41FN4O4 | 708.31 |
| | A1O054 | 4-[4-[2-Fluoro-4-[4-[4-[(4-methoxyphenyl)carbamoyl]phenyl]piperazine-1-carbonyl]phenyl]phenyl]-1-hydroxy-N,N-dimethylnaphthalene-2-carboxamide | C44H39FN4O5 | 722.29 |
| | A1O055 | 1-Hydroxy-4-[4-[3-methoxy-4-[[4-[4-[(4-methoxyphenyl)carbamoyl]phenyl]piperazin-1-yl]methyl]phenyl]phenyl]-N,N-dimethylnaphthalene-2-carboxamide | C45H44N4O5 | 720.33 |
| | A1O056 | 4-[4-[3,5-Dimethoxy-4-[[4-[4-[(4-methoxyphenyl)carbamoyl]phenyl]piperazin-1-yl]methyl]phenyl]phenyl]-1-hydroxy-N,N-dimethylnaphthalene-2-carboxamide | C46H46N4O6 | 750.34 |
| | A1O057 | 1-Hydroxy-4-[4-[4-[[4-[4-[(4-methoxyphenyl)carbamoyl]phenyl]piperazin-1-yl]methyl]naphthalen-1-yl]phenyl]-N,N-dimethylnaphthalene-2-carboxamide | C48H44N4O4 | 740.34 |
| | A1O058 | 1-Hydroxy-4-[4-[4-[4-[4-[(4-methoxyphenyl)carbamoyl]phenyl]piperazine-1-carbonyl]naphthalen-1-yl]phenyl]-N,N-dimethylnaphthalene-2-carboxamide | C48H42N4O5 | 754.32 |
| | A1O059 | 1-Hydroxy-4-[4-[5-[[4-[4-[(4-methoxyphenyl)carbamoyl]phenyl]piperazin-1-yl]methyl]thiophen-3-yl]phenyl]-N,N-dimethylnaphthalene-2-carboxamide | C42H40N4O4S | 696.28 |
| | A1O006 | 6-[4-[[4-[4-[3-(Dimethylcarbamoyl)-4-hydroxynaphthalen-1-yl]phenyl]-3-fluorophenyl]methyl]piperazin-1-yl]-N-(4-methoxyphenyl)pyridazine-3-carboxamide | C42H39FN6O4 | 710.3 |
| | A1O060 | 1-Hydroxy-4-[4-[5-[4-[4-[(4-methoxyphenyl)carbamoyl]phenyl]piperazine-1-carbonyl]thiophen-3-yl]phenyl]-N,N-dimethylnaphthalene-2-carboxamide | C42H38N4O5S | 710.26 |
| | A1O061 | 1-Hydroxy-4-[4-[3-[[4-[4-[(4-methoxyphenyl)carbamoyl]phenyl]piperazin-1-yl]methyl]-5-(trifluoromethyl)phenyl]phenyl]-N,N-dimethylnaphthalene-2-carboxamide | C45H41F3N4O4 | 758.31 |
| | A1O062 | 1-Hydroxy-4-[4-[3-[4-[4-[(4-methoxyphenyl)carbamoyl]phenyl]piperazine-1-carbonyl]-5-(trifluoromethyl)phenyl]phenyl]-N,N-dimethylnaphthalene-2-carboxamide | C45H39F3N4O5 | 772.29 |
| | A1O063 | 1-Hydroxy-4-[4-[3-[[4-[4-[(4-methoxyphenyl)carbamoyl]phenyl]piperazin-1-yl]methyl]-5-(trifluoromethoxy)phenyl]phenyl]-N,N-dimethylnaphthalene-2-carboxamide | C45H41F3N4O5 | 774.3 |
| | A1O064 | 4-[4-[3-Ethoxy-5-[[4-[4-[(4-methoxyphenyl)carbamoyl]phenyl]piperazin-1-yl]methyl]phenyl]phenyl]-1-hydroxy-N,N-dimethylnaphthalene-2-carboxamide | C46H46N4O5 | 734.35 |
| | A1O065 | 1-Hydroxy-4-[4-[3-[[4-[4-[(4-methoxyphenyl)carbamoyl]phenyl]piperazin-1-yl]methyl]-5-propan-2-yloxyphenyl]phenyl]-N,N-dimethylnaphthalene-2-carboxamide | C47H48N4O5 | 748.36 |
| | A1O066 | 4-[4-[3-tert-Butyl-5-[4-[4-[(4-methoxyphenyl)carbamoyl]phenyl]piperazine-1-carbonyl]phenyl]phenyl]-1-hydroxy-N,N-dimethylnaphthalene-2-carboxamide | C48H48N4O5 | 760.36 |
| | A1O067 | 4-[4-[3-Fluoro-4-[1-[4-[4-[(4-methoxyphenyl)carbamoyl]phenyl]piperazin-1-yl]ethyl]phenyl]phenyl]-1-hydroxy-N,N-dimethylnaphthalene-2-carboxamide | C45H43FN4O4 | 722.33 |
| | A1O068 | 1-Hydroxy-4-[4-[2-[2-[[4-[4-[(4-methoxyphenyl)carbamoyl]phenyl]piperazin-1-yl]methyl]phenyl]ethynyl]phenyl]-N,N-dimethylnaphthalene-2-carboxamide | C46H42N4O4 | 714.32 |
| | A1O069 | 1-Hydroxy-4-[4-[2-[3-[4-[4-[(4-methoxyphenyl)carbamoyl]phenyl]piperazine-1-carbonyl]-5-(trifluoromethyl)phenyl]ethynyl]phenyl]-N,N-dimethylnaphthalene-2-carboxamide | C47H39F3N4O5 | 796.29 |

[Table 127]

| | | | | |
|---|---|---|---|---|
| mixED 08 | A1O007 | 6-[4-[4-[4-[3-(Dimethylcarbamoyl)-4-hydroxynaphthalen-1-yl]phenyl]-3-fluorobenzoyl]piperazin-1-yl]-N-(4-methoxyphenyl)pyridazine-3-carboxamide | C42H37FN6O5 | 724.28 |
| | A1O070 | 1-Hydroxy-4-[4-[2-[5-[4-[4-[(4-methoxyphenyl)carbamoyl]phenyl]piperazine-1-carbonyl]pyridin-3-yl]ethynyl]phenyl]-N,N-dimethylnaphthalene-2-carboxamide | C45H39N5O5 | 729.3 |
| | A1O071 | 1-Hydroxy-4-[4-[1-[3-[4-[4-[(4-methoxyphenyl)carbamoyl]phenyl]piperazine-1-carbonyl]-5-(trifluoromethyl)phenyl]pyrazol-4-yl]phenyl]-N,N-dimethylnaphthalene-2-carboxamide | C48H41F3N6O5 | 838.31 |
| | A1O072 | 1-Hydroxy-4-[5-[2-[[4-[4-[(4-methoxyphenyl)carbamoyl]phenyl]piperazin-1-yl]methyl]phenyl]pyridin-2-yl]-N,N-dimethylnaphthalene-2-carboxamide | C43H41N5O4 | 691.32 |
| | A1O073 | 1-Hydroxy-4-[5-[2-[3-[4-[4-[(4-methoxyphenyl)carbamoyl]phenyl]piperazin-1-yl]-3-oxopropyl]phenyl]pyridin-2-yl]-N,N-dimethylnaphthalene-2-carboxamide | C45H43N5O5 | 733.33 |
| | A1O074 | 1-Hydroxy-4-[5-[4-[1-[4-[4-[(4-methoxyphenyl)carbamoyl]phenyl]piperazin-1-yl]ethyl]phenyl]pyridin-2-yl]-N,N-dimethylnaphthalene-2-carboxamide | C44H43N5O4 | 705.33 |

| | | | | |
|---|---|---|---|---|
| | A1O075 | 1-Hydroxy-4-[5-[4-[[4-[4-[(4-methoxyphenyl)carbamoyl]phenyl]piperazin-1-yl]methyl]-2-methylphenyl]pyridin-2-yl]-N,N-dimethylnaphthalene-2-carboxamide | C44H43N5O4 | 705.33 |
| mixED 08 | A1O076 | 1-Hydroxy-4-[5-[4-[4-[4-[(4-methoxyphenyl)carbamoyl]phenyl]piperazine-1-carbonyl]-2-methylphenyl]pyridin-2-yl]-N,N-dimethylnaphthalene-2-carboxamide | C44H41N5O5 | 719.31 |
| | A1O077 | 4-[5-[3-Ethyl-4-[[4-[4-[(4-methoxyphenyl)carbamoyl]phenyl]piperazin-1-yl]methyl]phenyl]pyridin-2-yl]-1-hydroxy-N,N-dimethylnaphthalene-2-carboxamide | C45H45N5O4 | 719.35 |
| | A1O078 | 4-[5-[2-Fluoro-4-[[4-[4-[(4-methoxyphenyl)carbamoyl]phenyl]piperazin-1-yl]methyl]phenyl]pyridin-2-yl]-1-hydroxy-N,N-dimethylnaphthalene-2-carboxamide | C43H40FN5O4 | 709.31 |
| | A1O079 | 4-[5-[2-Fluoro-4-[4-[4-[(4-methoxyphenyl)carbamoyl]phenyl]piperazine-1-carbonyl]phenyl]pyridin-2-yl]-1-hydroxy-N,N-dimethylnaphthalene-2-carboxamide | C43H38FN5O5 | 723.29 |
| | A1O008 | 6-[4-[[4-[4-[3-(Dimethylcarbamoyl)-4-hydroxynaphthalen-1-yl]phenyl]-2-methoxyphenyl]methyl]piperazin-1-yl]-N-(4-methoxyphenyl)pyridazine-3-carboxamide | C43H42N6O5 | 722.32 |
| | A1O080 | 1-Hydroxy-4-[5-[3-methoxy-4-[[4-[4-[(4-methoxyphenyl)carbamoyl]phenyl]piperazin-1-yl]methyl]phenyl]pyridin-2-yl]-N,N-dimethylnaphthalene-2-carboxamide | C44H43N5O5 | 721.33 |
| | A1O081 | 4-[5-[3,5-Dimethoxy-4-[[4-[4-[(4-methoxyphenyl)carbamoyl]phenyl]piperazin-1-yl]methyl]phenyl]pyridin-2-yl]-1-hydroxy-N,N-dimethylnaphthalene-2-carboxamide | C45H45N5O6 | 751.34 |
| | A1O082 | 1-Hydroxy-4-[5-[4-[[4-[4-[(4-methoxyphenyl)carbamoyl]phenyl]piperazin-1-yl]methyl]naphthalen-1-yl]pyridin-2-yl]-N,N-dimethylnaphthalene-2-carboxamide | C47H43N5O4 | 741.33 |
| | A1O083 | 1-Hydroxy-4-[5-[4-[4-[4-[(4-methoxyphenyl)carbamoyl]phenyl]piperazine-1-carbonyl]naphthalen-1-yl]pyridin-2-yl]-N,N-dimethylnaphthalene-2-carboxamide | C47H41N5O5 | 755.31 |
| | A1O084 | 1-Hydroxy-4-[5-[5-[[4-[4-[(4-methoxyphenyl)carbamoyl]phenyl]piperazin-1-yl]methyl]thiophen-3-yl]pyridin-2-yl]-N,N-dimethylnaphthalene-2-carboxamide | C41H39N5O4S | 697.27 |
| | A1O085 | 1-Hydroxy-4-[5-[5-[4-[4-[(4-methoxyphenyl)carbamoyl]phenyl]piperazine-1-carbonyl]thiophen-3-yl]pyridin-2-yl]-N,N-dimethylnaphthalene-2-carboxamide | C41H37N5O5S | 711.25 |
| | A1O086 | 1-Hydroxy-4-[5-[3-[[4-[4-[(4-methoxyphenyl)carbamoyl]phenyl]piperazin-1-yl]methyl]-5-(trifluoromethyl)phenyl]pyridin-2-yl]-N,N-dimethylnaphthalene-2-carboxamide | C44H40F3N5O4 | 759.3 |
| | A1O087 | 1-Hydroxy-4-[5-[3-[4-[4-[(4-methoxyphenyl)carbamoyl]phenyl]piperazine-1-carbonyl]-5-(trifluoromethyl)phenyl]pyridin-2-yl]-N,N-dimethylnaphthalene-2-carboxamide | C44H38F3N5O5 | 773.28 |
| | A1O088 | 1-Hydroxy-4-[5-[3-[[4-[4-[(4-methoxyphenyl)carbamoyl]phenyl]piperazin-1-yl]methyl]-5-(trifluoromethoxy)phenyl]pyridin-2-yl]-N,N-dimethylnaphthalene-2-carboxamide | C44H40F3N5O5 | 775.3 |
| | A1O089 | 4-[5-[3-Ethoxy-5-[[4-[4-[(4-methoxyphenyl)carbamoyl]phenyl]piperazin-1-yl]methyl]phenyl]pyridin-2-yl]-1-hydroxy-N,N-dimethylnaphthalene-2-carboxamide | C45H45N5O5 | 735.34 |
| | A1O009 | 6-[4-[[4-[4-[3-(Dimethylcarbamoyl)-4-hydroxynaphthalen-1-yl]phenyl]-2,6-dimethoxyphenyl]methyl]piperazin-1-yl]-N-(4-methoxyphenyl)pyridazine-3-carboxamide | C44H44N6O6 | 752.33 |
| | A1O090 | 1-Hydroxy-4-[5-[3-[[4-[4-[(4-methoxyphenyl)carbamoyl]phenyl]piperazin-1-yl]methyl]-5-propan-2-yloxyphenyl]pyridin-2-yl]-N,N-dimethylnaphthalene-2-carboxamide | C46H47N5O5 | 749.36 |
| | A1O091 | 4-[5-[3-tert-Butyl-5-[4-[4-[(4-methoxyphenyl)carbamoyl]phenyl]piperazine-1-carbonyl]phenyl]pyridin-2-yl]-1-hydroxy-N,N-dimethylnaphthalene-2-carboxamide | C47H47N5O5 | 761.36 |
| | A1O092 | 4-[5-[3-Fluoro-4-[1-[4-[4-[(4-methoxyphenyl)carbamoyl]phenyl]piperazin-1-yl]ethyl]phenyl]pyridin-2-yl]-1-hydroxy-N,N-dimethylnaphthalene-2-carboxamide | C44H42FN5O4 | 723.32 |
| | A1O093 | 1-Hydroxy-4-[5-[2-[2-[[4-[4-[(4-methoxyphenyl)carbamoyl]phenyl]piperazin-1-yl]methyl]phenyl]ethynyl]pyridin-2-yl]-N,N-dimethylnaphthalene-2-carboxamide | C45H41N5O4 | 715.32 |
| | A1O094 | 1-Hydroxy-4-[5-[2-[3-[4-[4-[(4-methoxyphenyl)carbamoyl]phenyl]piperazine-1-carbonyl]-5-(trifluoromethyl)phenyl]ethynyl]pyridin-2-yl]-N,N-dimethylnaphthalene-2-carboxamide | C46H38F3N5O5 | 797.28 |
| | A1O095 | 1-Hydroxy-4-[5-[2-[5-[4-[4-[(4-methoxyphenyl)carbamoyl]phenyl]piperazine-1-carbonyl]pyridin-3-yl]ethynyl]pyridin-2-yl]-N,N-dimethylnaphthalene-2-carboxamide | C44H38N6O5 | 730.29 |
| | A1O096 | 1-Hydroxy-4-[5-[1-[3-[4-[4-[(4-methoxyphenyl)carbamoyl]phenyl]piperazine-1-carbonyl]-5-(trifluoromethyl)phenyl]pyrazol-4-yl]pyridin-2-yl]-N,N-dimethylnaphthalene-2-carboxamide | C47H40F3N7O5 | 839.3 |
| | A1O097 | 6-[4-[2-[2-[4-[3-(Dimethylcarbamoyl)-4-hydroxynaphthalen-1-yl]phenyl]ethynyl]benzoyl]piperazin-1-yl]-N-(4-methoxyphenyl)pyridazine-3-carboxamide | C44H38N6O5 | 730.29 |
| | A1O098 | 6-[4-[2-[2-[6-[3-(Dimethylcarbamoyl)-4-hydroxynaphthalen-1-yl]pyridin-3-yl]ethynyl]benzoyl]piperazin-1-yl]-N-(4-methoxyphenyl)pyridazine-3-carboxamide | C43H37N7O5 | 731.286 |
| | A1O099 | 1-Hydroxy-4-[4-[2-[2-[4-[4-[(4-methoxyphenyl)carbamoyl]phenyl]piperazine-1-carbonyl]phenyl]ethynyl]phenyl]-N,N-dimethylnaphthalene-2-carboxamide | C46H40N4O5 | 728.3 |
| | A1O100 | 1-Hydroxy-4-[5-[2-[2-[4-[4-[(4-methoxyphenyl)carbamoyl]phenyl]piperazine-1-carbonyl]phenyl]ethynyl]pyridin-2-yl]-N,N-dimethylnaphthalene-2-carboxamide | C45H39N5O5 | 729.295 |

[Table 128]

[Table EE05]

| Table EE05 | | | | |
|---|---|---|---|---|
| Mixture No. | No. of compound that may be contained | Compound name | Compositional formula | Exact MS |
| mixED 09 | A1A001 | N-(1-Adamantylmethyl)-6-[4-[[2-[4-[3-(dimethylcarbamoyl)-4-hydroxynaphthalen-1-yl]phenyl]phenyl]methyl]piperazin-1-yl]pyridazine-3-carboxamide | C46H50N6O3 | 734.39 |

| | | | | |
|---|---|---|---|---|
| | A1A047 | 4-[4-[2-[[4-[4-(1-Adamantylmethylcarbamoyl)phenyl]piperazin-1-yl]methyl]phenyl]phenyl]-1-hydroxy-N,N-dimethylnaphthalene-2-carboxamide | C48H52N4O3 | 732.4 |
| | A1A002 | N-(1-Adamantylmethyl)-6-[4-[3-[2-[4-[3-(dimethylcarbamoyl)-4-hydroxynaphthalen-1-yl]phenyl]phenyl]propanoyl]piperazin-1-yl]pyridazine-3-carboxamide | C48H52N6O4 | 776.41 |
| | A1A048 | 4-[4-[2-[3-[4-[4-(1-Adamantylmethylcarbamoyl)phenyl]piperazin-1-yl]-3-oxopropyl]phenyl]phenyl]-1-hydroxy-N,N-dimethylnaphthalene-2-carboxamide | C50H54N4O4 | 774.41 |
| | A1A049 | 4-[4-[4-[1-[4-[4-(1-Adamantylmethylcarbamoyl)phenyl]piperazin-1-yl]ethyl]phenyl]phenyl]-1-hydroxy-N,N-dimethylnaphthalene-2-carboxamide | C49H54N4O3 | 746.42 |
| | A1A003 | N-(1-Adamantylmethyl)-6-[4-[[4-[4-[3-(dimethylcarbamoyl)-4-hydroxynaphthalen-1-yl]phenyl]-3-methylphenyl]methyl]piperazin-1-yl]pyridazine-3-carboxamide | C47H52N6O3 | 748.41 |
| | A1A050 | 4-[4-[4-[[4-[4-(1-Adamantylmethylcarbamoyl)phenyl]piperazin-1-yl]methyl]-2-methylphenyl]phenyl]-1-hydroxy-N,N-dimethylnaphthalene-2-carboxamide | C49H54N4O3 | 746.42 |
| | A1A004 | N-(1-Adamantylmethyl)-6-[4-[4-[4-[3-(dimethylcarbamoyl)-4-hydroxynaphthalen-1-yl]phenyl]-3-methylbenzoyl]piperazin-1-yl]pyridazine-3-carboxamide | C47H50N6O4 | 762.39 |
| | A1A051 | 4-[4-[4-[4-[4-(1-Adamantylmethylcarbamoyl)phenyl]piperazine-1-carbonyl]-2-methylphenyl]phenyl]-1-hydroxy-N,N-dimethylnaphthalene-2-carboxamide | C49H52N4O4 | 760.4 |
| | A1A005 | N-(1-Adamantylmethyl)-6-[4-[[4-[4-[3-(dimethylcarbamoyl)-4-hydroxynaphthalen-1-yl]phenyl]-2-ethylphenyl]methyl]piperazin-1-yl]pyridazine-3-carboxamide | C48H54N6O3 | 762.43 |
| | A1A052 | 4-[4-[4-[[4-[4-(1-Adamantylmethylcarbamoyl)phenyl]piperazin-1-yl]methyl]-3-ethylphenyl]phenyl]-1-hydroxy-N,N-dimethylnaphthalene-2-carboxamide | C50H56N4O3 | 760.44 |
| | A1A006 | N-(1-Adamantylmethyl)-6-[4-[[4-[4-[3-(dimethylcarbamoyl)-4-hydroxynaphthalen-1-yl]phenyl]-3-fluorophenyl]methyl]piperazin-1-yl]pyridazine-3-carboxamide | C46H49FN6O3 | 752.39 |
| | A1A053 | 4-[4-[4-[[4-[4-(1-Adamantylmethylcarbamoyl)phenyl]piperazin-1-yl]methyl]-2-fluorophenyl]phenyl]-1-hydroxy-N,N-dimethylnaphthalene-2-carboxamide | C48H51FN4O3 | 750.39 |
| | A1A007 | N-(1-Adamantylmethyl)-6-[4-[4-[4-[3-(dimethylcarbamoyl)-4-hydroxynaphthalen-1-yl]phenyl]-3-fluorobenzoyl]piperazin-1-yl]pyridazine-3-carboxamide | C46H47FN6O4 | 766.36 |
| | A1A054 | 4-[4-[4-[4-[4-(1-Adamantylmethylcarbamoyl)phenyl]piperazine-1-carbonyl]-2-fluorophenyl]phenyl]-1-hydroxy-N,N-dimethylnaphthalene-2-carboxamide | C48H49FN4O4 | 764.37 |
| | A1A008 | N-(1-Adamantylmethyl)-6-[4-[[4-[4-[3-(dimethylcarbamoyl)-4-hydroxynaphthalen-1-yl]phenyl]-2-methoxyphenyl]methyl]piperazin-1-yl]pyridazine-3-carboxamide | C47H52N6O4 | 764.41 |
| | A1A055 | 4-[4-[4-[[4-[4-(1-Adamantylmethylcarbamoyl)phenyl]piperazin-1-yl]methyl]-3-methoxyphenyl]phenyl]-1-hydroxy-N,N-dimethylnaphthalene-2-carboxamide | C49H54N4O4 | 762.41 |
| | A1A009 | N-(1-Adamantylmethyl)-6-[4-[[4-[4-[3-(dimethylcarbamoyl)-4-hydroxynaphthalen-1-yl]phenyl]-2,6-dimethoxyphenyl]methyl]piperazin-1-yl]pyridazine-3-carboxamide | C48H54N6O5 | 794.42 |
| | A1A056 | 4-[4-[4-[[4-[4-(1-Adamantylmethylcarbamoyl)phenyl]piperazin-1-yl]methyl]-3,5-dimethoxyphenyl]phenyl]-1-hydroxy-N,N-dimethylnaphthalene-2-carboxamide | C50H56N4O5 | 792.43 |
| | A1A010 | N-(1-Adamantylmethyl)-6-[4-[[4-[4-[3-(dimethylcarbamoyl)-4-hydroxynaphthalen-1-yl]phenyl]naphthalen-1-yl]methyl]piperazin-1-yl]pyridazine-3-carboxamide | C50H52N6O3 | 784.41 |
| | A1A057 | 4-[4-[4-[[4-[4-(1-Adamantylmethylcarbamoyl)phenyl]piperazin-1-yl]methyl]naphthalen-1-yl]phenyl]-1-hydroxy-N,N-dimethylnaphthalene-2-carboxamide | C52H54N4O3 | 782.42 |
| | A1A011 | N-(1-Adamantylmethyl)-6-[4-[4-[4-[3-(dimethylcarbamoyl)-4-hydroxynaphthalen-1-yl]phenyl]naphthalene-1-carbonyl]piperazin-1-yl]pyridazine-3-carboxamide | C50H50N6O4 | 798.39 |
| | A1A058 | 4-[4-[4-[4-[4-(1-Adamantylmethylcarbamoyl)phenyl]piperazine-1-carbonyl]naphthalen-1-yl]phenyl]-1-hydroxy-N,N-dimethylnaphthalene-2-carboxamide | C52H52N4O4 | 796.4 |
| | A1A012 | N-(1-Adamantylmethyl)-6-[4-[[4-[4-[3-(dimethylcarbamoyl)-4-hydroxynaphthalen-1-yl]phenyl]thiophen-2-yl]methyl]piperazin-1-yl]pyridazine-3-carboxamide | C44H48N6O3S | 740.35 |
| mixED 09 | A1A059 | 4-[4-[5-[[4-[4-(1-Adamantylmethylcarbamoyl)phenyl]piperazin-1-yl]methyl]thiophen-3-yl]phenyl]-1-hydroxy-N,N-dimethylnaphthalene-2-carboxamide | C46H50N4O3S | 738.36 |
| | A1A013 | N-(1-Adamantylmethyl)-6-[4-[4-[4-[3-(dimethylcarbamoyl)-4-hydroxynaphthalen-1-yl]phenyl]thiophene-2-carbonyl]piperazin-1-yl]pyridazine-3-carboxamide | C44H46N6O4S | 754.33 |
| | A1A060 | 4-[4-[5-[4-[4-(1-Adamantylmethylcarbamoyl)phenyl]piperazine-1-carbonyl]thiophen-3-yl]phenyl]-1-hydroxy-N,N-dimethylnaphthalene-2-carboxamide | C46H48N4O4S | 752.34 |
| | A1A014 | N-(1-Adamantylmethyl)-6-[4-[[3-[4-[3-(dimethylcarbamoyl)-4-hydroxynaphthalen-1-yl]phenyl]-5-(trifluoromethyl)phenyl]methyl]piperazin-1-yl]pyridazine-3-carboxamide | C47H49F3N6O3 | 802.38 |
| | A1A061 | 4-[4-[3-[[4-[4-(1-Adamantylmethylcarbamoyl)phenyl]piperazin-1-yl]methyl]-5-(trifluoromethyl)phenyl]phenyl]-1-hydroxy-N,N-dimethylnaphthalene-2-carboxamide | C49H51F3N4O3 | 800.39 |
| | A1A015 | N-(1-Adamantylmethyl)-6-[4-[3-[4-[3-(dimethylcarbamoyl)-4-hydroxynaphthalen-1-yl]phenyl]-5-(trifluoromethyl)benzoyl]piperazin-1-yl]pyridazine-3-carboxamide | C47H47F3N6O4 | 816.36 |
| | A1A062 | 4-[4-[3-[4-[4-(1-Adamantylmethylcarbamoyl)phenyl]piperazine-1-carbonyl]-5-(trifluoromethyl)phenyl]phenyl]-1-hydroxy-N,N-dimethylnaphthalene-2-carboxamide | C49H49F3N4O4 | 814.37 |

| | | | | |
|---|---|---|---|---|
| | A1A016 | N-(1-Adamantylmethyl)-6-[4-[[3-[4-[3-(dimethylcarbamoyl)-4-hydroxynaphthalen-1-yl]phenyl]-5-(trifluoromethoxy)phenyl]methyl]piperazin-1-yl]pyridazine-3-carboxamide | C47H49F3N6O4 | 818.38 |
| | A1A063 | 4-[4-[3-[[4-[4-(1-Adamantylmethylcarbamoyl)phenyl]piperazin-1-yl]methyl]-5-(trifluoromethoxy)phenyl]phenyl]-1-hydroxy-N,N-dimethylnaphthalene-2-carboxamide | C49H51F3N4O4 | 816.39 |

[Table 129]

| | | | | |
|---|---|---|---|---|
| mixED 09 | A1A017 | N-(1-Adamantylmethyl)-6-[4-[[3-[4-[3-(dimethylcarbamoyl)-4-hydroxynaphthalen-1-yl]phenyl]-5-ethoxyphenyl]methyl]piperazin-1-yl]pyridazine-3-carboxamide | C48H54N6O4 | 778.42 |
| | A1A064 | 4-[4-[3-[[4-[4-(1-Adamantylmethylcarbamoyl)phenyl]piperazin-1-yl]methyl]-5-ethoxyphenyl]phenyl]-1-hydroxy-N,N-dimethylnaphthalene-2-carboxamide | C50H56N4O4 | 776.43 |
| | A1A018 | N-(1-Adamantylmethyl)-6-[4-[[3-[4-[3-(dimethylcarbamoyl)-4-hydroxynaphthalen-1-yl]phenyl]-5-propan-2-yloxyphenyl]methyl]piperazin-1-yl]pyridazine-3-carboxamide | C49H56N6O4 | 792.44 |
| | A1A065 | 4-[4-[3-[[4-[4-(1-Adamantylmethylcarbamoyl)phenyl]piperazin-1-yl]methyl]-5-propan-2-yloxyphenyl]phenyl]-1-hydroxy-N,N-dimethylnaphthalene-2-carboxamide | C51H58N4O4 | 790.45 |
| | A1A019 | N-(1-Adamantylmethyl)-6-[4-[3-tert-butyl-5-[4-[3-(dimethylcarbamoyl)-4-hydroxynaphthalen-1-yl]phenyl]benzoyl]piperazin-1-yl]pyridazine-3-carboxamide | C50H56N6O4 | 804.44 |
| | A1A066 | 4-[4-[3-[4-[4-(1-Adamantylmethylcarbamoyl)phenyl]piperazine-1-carbonyl]-5-tert-butylphenyl]phenyl]-1-hydroxy-N,N-dimethylnaphthalene-2-carboxamide | C52H58N4O4 | 802.45 |
| | A1A067 | 4-[4-[4-[1-[4-[4-(1-Adamantylmethylcarbamoyl)phenyl]piperazin-1-yl]ethyl]-3-fluorophenyl]phenyl]-1-hydroxy-N,N-dimethylnaphthalene-2-carboxamide | C49H53FN4O3 | 764.41 |
| | A1A020 | N-(1-Adamantylmethyl)-6-[4-[[2-[2-[4-[3-(dimethylcarbamoyl)-4-hydroxynaphthalen-1-yl]phenyl]ethynyl]phenyl]methyl]piperazin-1-yl]pyridazine-3-carboxamide | C48H50N6O3 | 758.39 |
| | A1A068 | 4-[4-[2-[2-[[4-[4-(1-Adamantylmethylcarbamoyl)phenyl]piperazin-1-yl]methyl]phenyl]ethynyl]phenyl]-1-hydroxy-N,N-dimethylnaphthalene-2-carboxamide | C50H52N4O3 | 756.4 |
| | A1A021 | N-(1-Adamantylmethyl)-6-[4-[3-[2-[4-[3-(dimethylcarbamoyl)-4-hydroxynaphthalen-1-yl]phenyl]ethynyl]-5-(trifluoromethyl)benzoyl]piperazin-1-yl]pyridazine-3-carboxamide | C49H47F3N6O4 | 840.36 |
| | A1A069 | 4-[4-[2-[3-[4-[4-(1-Adamantylmethylcarbamoyl)phenyl]piperazine-1-carbonyl]-5-(trifluoromethyl)phenyl]ethynyl]phenyl]-1-hydroxy-N,N-dimethylnaphthalene-2-carboxamide | C51H49F3N4O4 | 838.37 |
| | A1A022 | N-(1-Adamantylmethyl)-6-[4-[5-[2-[4-[3-(dimethylcarbamoyl)-4-hydroxynaphthalen-1-yl]phenyl]ethynyl]pyridine-3-carbonyl]piperazin-1-yl]pyridazine-3-carboxamide | C47H47N7O4 | 773.37 |
| | A1A070 | 4-[4-[2-[5-[4-[4-(1-Adamantylmethylcarbamoyl)phenyl]piperazine-1-carbonyl]pyridin-3-yl]ethynyl]phenyl]-1-hydroxy-N,N-dimethylnaphthalene-2-carboxamide | C49H49N5O4 | 771.38 |
| | A1A023 | N-(1-Adamantylmethyl)-6-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]pyridazine-3-carboxamide | C50H49F3N8O4 | 882.38 |
| | A1A071 | 4-[4-[1-[3-[4-[4-(1-Adamantylmethylcarbamoyl)phenyl]piperazine-1-carbonyl]-5-(trifluoromethyl)phenyl]pyrazol-4-yl]phenyl]-1-hydroxy-N,N-dimethylnaphthalene-2-carboxamide | C52H51F3N6O4 | 880.39 |
| | A1A024 | N-(1-Adamantylmethyl)-6-[4-[[2-[6-[3-(dimethylcarbamoyl)-4-hydroxynaphthalen-1-yl]pyridin-3-yl]phenyl]methyl]piperazin-1-yl]pyridazine-3-carboxamide | C45H49N7O3 | 735.39 |
| mixED 09 | A1A072 | 4-[5-[2-[[4-[4-(1-Adamantylmethylcarbamoyl)phenyl]piperazin-1-yl]methyl]phenyl]pyridin-2-yl]-1-hydroxy-N,N-dimethylnaphthalene-2-carboxamide | C47H51N5O3 | 733.4 |
| | A1A025 | N-(1-Adamantylmethyl)-6-[4-[3-[2-[6-[3-(dimethylcarbamoyl)-4-hydroxynaphthalen-1-yl]pyridin-3-yl]phenyl]propanoyl]piperazin-1-yl]pyridazine-3-carboxamide | C47H51N7O4 | 777.4 |
| | A1A073 | 4-[5-[2-[3-[4-[4-(1-Adamantylmethylcarbamoyl)phenyl]piperazin-1-yl]-3-oxopropyl]phenyl]pyridin-2-yl]-1-hydroxy-N,N-dimethylnaphthalene-2-carboxamide | C49H53N5O4 | 775.41 |
| | A1A074 | 4-[5-[4-[1-[4-[4-(1-Adamantylmethylcarbamoyl)phenyl]piperazin-1-yl]ethyl]phenyl]pyridin-2-yl]-1-hydroxy-N,N-dimethylnaphthalene-2-carboxamide | C48H53N5O3 | 747.41 |
| | A1A026 | N-(1-Adamantylmethyl)-6-[4-[[4-[6-[3-(dimethylcarbamoyl)-4-hydroxynaphthalen-1-yl]pyridin-3-yl]-3-methylphenyl]methyl]piperazin-1-yl]pyridazine-3-carboxamide | C46H51N7O3 | 749.41 |
| | A1A075 | 4-[5-[4-[[4-[4-(1-Adamantylmethylcarbamoyl)phenyl]piperazin-1-yl]methyl]-2-methylphenyl]pyridin-2-yl]-1-hydroxy-N,N-dimethylnaphthalene-2-carboxamide | C48H53N5O3 | 747.41 |
| | A1A027 | N-(1-Adamantylmethyl)-6-[4-[4-[6-[3-(dimethylcarbamoyl)-4-hydroxynaphthalen-1-yl]pyridin-3-yl]-3-methylbenzoyl]piperazin-1-yl]pyridazine-3-carboxamide | C46H49N7O4 | 763.38 |
| | A1A076 | 4-[5-[4-[4-[4-(1-Adamantylmethylcarbamoyl)phenyl]piperazine-1-carbonyl]-2-methylphenyl]pyridin-2-yl]-1-hydroxy-N,N-dimethylnaphthalene-2-carboxamide | C48H51N5O4 | 761.39 |

| | | | | |
|---|---|---|---|---|
| | A1A028 | N-(1-Adamantylmethyl)-6-[4-[[4-[6-[3-(dimethylcarbamoyl)-4-hydroxynaphthalen-1-yl]pyridin-3-yl]-2-ethylphenyl]methyl]piperazin-1-yl]pyridazine-3-carboxamide | C47H53N7O3 | 763.42 |
| | A1A077 | 4-[5-[4-[[4-[4-(1-Adamantylmethylcarbamoyl)phenyl]piperazin-1-yl]methyl]-3-ethylphenyl]pyridin-2-yl]-1-hydroxy-N,N-dimethylnaphthalene-2-carboxamide | C49H55N5O3 | 761.43 |
| | A1A029 | N-(1-Adamantylmethyl)-6-[4-[[4-[6-[3-(dimethylcarbamoyl)-4-hydroxynaphthalen-1-yl]pyridin-3-yl]-3-fluorophenyl]methyl]piperazin-1-yl]pyridazine-3-carboxamide | C45H48FN7O3 | 753.38 |
| | A1A078 | 4-[5-[4-[[4-[4-(1-Adamantylmethylcarbamoyl)phenyl]piperazin-1-yl]methyl]-2-fluorophenyl]pyridin-2-yl]-1-hydroxy-N,N-dimethylnaphthalene-2-carboxamide | C47H50FN5O3 | 751.39 |
| | A1A030 | N-(1-Adamantylmethyl)-6-[4-[4-[6-[3-(dimethylcarbamoyl)-4-hydroxynaphthalen-1-yl]pyridin-3-yl]-3-fluorobenzoyl]piperazin-1-yl]pyridazine-3-carboxamide | C45H46FN7O4 | 767.36 |
| | A1A079 | 4-[5-[4-[4-[4-(1-Adamantylmethylcarbamoyl)phenyl]piperazine-1-carbonyl]-2-fluorophenyl]pyridin-2-yl]-1-hydroxy-N,N-dimethylnaphthalene-2-carboxamide | C47H48FN5O4 | 765.37 |
| | A1A031 | N-(1-Adamantylmethyl)-6-[4-[[4-[6-[3-(dimethylcarbamoyl)-4-hydroxynaphthalen-1-yl]pyridin-3-yl]-2-methoxyphenyl]methyl]piperazin-1-yl]pyridazine-3-carboxamide | C46H51N7O4 | 765.4 |
| | A1A080 | 4-[5-[4-[[4-[4-(1-Adamantylmethylcarbamoyl)phenyl]piperazin-1-yl]methyl]-3-methoxyphenyl]pyridin-2-yl]-1-hydroxy-N,N-dimethylnaphthalene-2-carboxamide | C48H53N5O4 | 763.41 |
| | A1A032 | N-(1-Adamantylmethyl)-6-[4-[[4-[6-[3-(dimethylcarbamoyl)-4-hydroxynaphthalen-1-yl]pyridin-3-yl]-2,6-dimethoxyphenyl]methyl]piperazin-1-yl]pyridazine-3-carboxamide | C47H53N7O5 | 795.41 |

[Table 130]

| | | | | |
|---|---|---|---|---|
| mixED 09 | A1A081 | 4-[5-[4-[[4-[4-(1-Adamantylmethylcarbamoyl)phenyl]piperazin-1-yl]methyl]-3,5-dimethoxyphenyl]pyridin-2-yl]-1-hydroxy-N,N-dimethylnaphthalene-2-carboxamide | C49H55N5O5 | 793.42 |
| | A1A033 | N-(1-Adamantylmethyl)-6-[4-[[4-[6-[3-(dimethylcarbamoyl)-4-hydroxynaphthalen-1-yl]pyridin-3-yl]naphthalen-1-yl]methyl]piperazin-1-yl]pyridazine-3-carboxamide | C49H51N7O3 | 785.41 |
| | A1A082 | 4-[5-[4-[[4-[4-(1-Adamantylmethylcarbamoyl)phenyl]piperazin-1-yl]methyl]naphthalen-1-yl]pyridin-2-yl]-1-hydroxy-N,N-dimethylnaphthalene-2-carboxamide | C51H53N5O3 | 783.41 |
| | A1A034 | N-(1-Adamantylmethyl)-6-[4-[4-[6-[3-(dimethylcarbamoyl)-4-hydroxynaphthalen-1-yl]pyridin-3-yl]naphthalene-1-carbonyl]piperazin-1-yl]pyridazine-3-carboxamide | C49H49N7O4 | 799.38 |
| | A1A083 | 4-[5-[4-[4-[4-(1-Adamantylmethylcarbamoyl)phenyl]piperazine-1-carbonyl]naphthalen-1-yl]pyridin-2-yl]-1-hydroxy-N,N-dimethylnaphthalene-2-carboxamide | C51H51N5O4 | 797.39 |
| | A1A035 | N-(1-Adamantylmethyl)-6-[4-[[4-[6-[3-(dimethylcarbamoyl)-4-hydroxynaphthalen-1-yl]pyridin-3-yl]thiophen-2-yl]methyl]piperazin-1-yl]pyridazine-3-carboxamide | C43H47N7O3S | 741.35 |
| | A1A084 | 4-[5-[5-[[4-[4-(1-Adamantylmethylcarbamoyl)phenyl]piperazin-1-yl]methyl]thiophen-3-yl]pyridin-2-yl]-1-hydroxy-N,N-dimethylnaphthalene-2-carboxamide | C45H49N5O3S | 739.36 |
| | A1A036 | N-(1-Adamantylmethyl)-6-[4-[4-[6-[3-(dimethylcarbamoyl)-4-hydroxynaphthalen-1-yl]pyridin-3-yl]thiophene-2-carbonyl]piperazin-1-yl]pyridazine-3-carboxamide | C43H45N7O4S | 755.33 |
| | A1A085 | 4-[5-[5-[4-[4-(1-Adamantylmethylcarbamoyl)phenyl]piperazine-1-carbonyl]thiophen-3-yl]pyridin-2-yl]-1-hydroxy-N,N-dimethylnaphthalene-2-carboxamide | C45H47N5O4S | 753.33 |
| | A1A037 | N-(1-Adamantylmethyl)-6-[4-[[3-[6-[3-(dimethylcarbamoyl)-4-hydroxynaphthalen-1-yl]pyridin-3-yl]-5-(trifluoromethyl)phenyl]methyl]piperazin-1-yl]pyridazine-3-carboxamide | C46H48F3N7O3 | 803.38 |
| | A1A086 | 4-[5-[3-[[4-[4-(1-Adamantylmethylcarbamoyl)phenyl]piperazin-1-yl]methyl]-5-(trifluoromethyl)phenyl]pyridin-2-yl]-1-hydroxy-N,N-dimethylnaphthalene-2-carboxamide | C48H50F3N5O3 | 801.39 |
| | A1A038 | N-(1-Adamantylmethyl)-6-[4-[3-[6-[3-(dimethylcarbamoyl)-4-hydroxynaphthalen-1-yl]pyridin-3-yl]-5-(trifluoromethyl)benzoyl]piperazin-1-yl]pyridazine-3-carboxamide | C46H46F3N7O4 | 817.36 |
| | A1A087 | 4-[5-[3-[4-[4-(1-Adamantylmethylcarbamoyl)phenyl]piperazine-1-carbonyl]-5-(trifluoromethyl)phenyl]pyridin-2-yl]-1-hydroxy-N,N-dimethylnaphthalene-2-carboxamide | C48H48F3N5O4 | 815.37 |
| | A1A039 | N-(1-Adamantylmethyl)-6-[4-[[3-[6-[3-(dimethylcarbamoyl)-4-hydroxynaphthalen-1-yl]pyridin-3-yl]-5-(trifluoromethoxy)phenyl]methyl]piperazin-1-yl]pyridazine-3-carboxamide | C46H48F3N7O4 | 819.37 |
| | A1A088 | 4-[5-[3-[[4-[4-(1-Adamantylmethylcarbamoyl)phenyl]piperazin-1-yl]methyl]-5-(trifluoromethoxy)phenyl]pyridin-2-yl]-1-hydroxy-N,N-dimethylnaphthalene-2-carboxamide | C48H50F3N5O4 | 817.38 |
| | A1A040 | N-(1-Adamantylmethyl)-6-[4-[[3-[6-[3-(dimethylcarbamoyl)-4-hydroxynaphthalen-1-yl]pyridin-3-yl]-5-ethoxyphenyl]methyl]piperazin-1-yl]pyridazine-3-carboxamide | C47H53N7O4 | 779.42 |
| | A1A089 | 4-[5-[3-[[4-[4-(1-Adamantylmethylcarbamoyl)phenyl]piperazin-1-yl]methyl]-5-ethoxyphenyl]pyridin-2-yl]-1-hydroxy-N,N-dimethylnaphthalene-2-carboxamide | C49H55N5O4 | 777.43 |

| | | | | |
|---|---|---|---|---|
| | A1A041 | N-(1-Adamantylmethyl)-6-[4-[[3-[6-[3-(dimethylcarbamoyl)-4-hydroxynaphthalen-1-yl]pyridin-3-yl]-5-propan-2-yloxyphenyl]methyl]piperazin-1-yl]pyridazine-3-carboxamide | C48H55N7O4 | 793.43 |
| | A1A090 | 4-[5-[3-[[4-[4-(1-Adamantylmethylcarbamoyl)phenyl]piperazin-1-yl]methyl]-5-propan-2-yloxyphenyl]pyridin-2-yl]-1-hydroxy-N,N-dimethylnaphthalene-2-carboxamide | C50H57N5O4 | 791.44 |
| | A1A042 | N-(1-Adamantylmethyl)-6-[4-[3-tert-butyl-5-[6-[3-(dimethylcarbamoyl)-4-hydroxynaphthalen-1-yl]pyridin-3-yl]benzoyl]piperazin-1-yl]pyridazine-3-carboxamide | C49H55N7O4 | 805.43 |
| | A1A091 | 4-[5-[3-[4-[4-(1-Adamantylmethylcarbamoyl)phenyl]piperazine-1-carbonyl]-5-tert-butylphenyl]pyridin-2-yl]-1-hydroxy-N,N-dimethylnaphthalene-2-carboxamide | C51H57N5O4 | 803.44 |
| | A1A092 | 4-[5-[4-[1-[4-[4-(1-Adamantylmethylcarbamoyl)phenyl]piperazin-1-yl]ethyl]-3-fluorophenyl]pyridin-2-yl]-1-hydroxy-N,N-dimethylnaphthalene-2-carboxamide | C48H52FN5O3 | 765.41 |
| | A1A043 | N-(1-Adamantylmethyl)-6-[4-[[2-[2-[6-[3-(dimethylcarbamoyl)-4-hydroxynaphthalen-1-yl]pyridin-3-yl]ethynyl]phenyl]methyl]piperazin-1-yl]pyridazine-3-carboxamide | C47H49N7O3 | 759.39 |
| | A1A093 | 4-[5-[2-[2-[[4-[4-(1-Adamantylmethylcarbamoyl)phenyl]piperazin-1-yl]methyl]phenyl]ethynyl]pyridin-2-yl]-1-hydroxy-N,N-dimethylnaphthalene-2-carboxamide | C49H51N5O3 | 757.4 |
| | A1A044 | N-(1-Adamantylmethyl)-6-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]pyridazine-3-carboxamide | C48H46F3N7O4 | 841.36 |
| | A1A094 | 4-[5-[2-[3-[4-[4-(1-Adamantylmethylcarbamoyl)phenyl]piperazine-1-carbonyl]-5-(trifluoromethyl)phenyl]ethynyl]pyridin-2-yl]-1-hydroxy-N,N-dimethylnaphthalene-2-carboxamide | C50H48F3N5O4 | 839.37 |
| mixED 09 | A1A045 | N-(1-Adamantylmethyl)-6-[4-[5-[2-[6-[3-(dimethylcarbamoyl)-4-hydroxynaphthalen-1-yl]pyridin-3-yl]ethynyl]pyridine-3-carbonyl]piperazin-1-yl]pyridazine-3-carboxamide | C46H46N8O4 | 774.36 |
| | A1A095 | 4-[5-[2-[5-[4-[4-(1-Adamantylmethylcarbamoyl)phenyl]piperazine-1-carbonyl]pyridin-3-yl]ethynyl]pyridin-2-yl]-1-hydroxy-N,N-dimethylnaphthalene-2-carboxamide | C48H48N6O4 | 772.37 |
| | A1A046 | N-(1-Adamantylmethyl)-6-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]pyridazine-3-carboxamide | C49H48F3N9O4 | 883.38 |
| | A1A096 | 4-[5-[1-[3-[4-[4-(1-Adamantylmethylcarbamoyl)phenyl]piperazine-1-carbonyl]-5-(trifluoromethyl)phenyl]pyrazol-4-yl]pyridin-2-yl]-1-hydroxy-N,N-dimethylnaphthalene-2-carboxamide | C51H50F3N7O4 | 881.39 |
| | A1A097 | N-(1-Adamantylmethyl)-6-[4-[2-[2-[4-[3-(dimethylcarbamoyl)-4-hydroxynaphthalen-1-yl]phenyl]ethynyl]benzoyl]piperazin-1-yl]pyridazine-3-carboxamide | C48H48N6O4 | 772.374 |
| | A1A098 | N-(1-Adamantylmethyl)-6-[4-[2-[2-[6-[3-(dimethylcarbamoyl)-4-hydroxynaphthalen-1-yl]pyridin-3-yl]ethynyl]benzoyl]piperazin-1-yl]pyridazine-3-carboxamide | C47H47N7O4 | 773.369 |
| | A1A099 | 4-[4-[2-[2-[4-[4-(1-Adamantylmethylcarbamoyl)phenyl]piperazine-1-carbonyl]phenyl]ethynyl]phenyl]-1-hydroxy-N,N-dimethylnaphthalene-2-carboxamide | C50H50N4O4 | 770.383 |
| | A1A100 | 4-[5-[2-[2-[4-[4-(1-Adamantylmethylcarbamoyl)phenyl]piperazine-1-carbonyl]phenyl]ethynyl]pyridin-2-yl]-1-hydroxy-N,N-dimethylnaphthalene-2-carboxamide | C49H49N5O4 | 771.378 |

[Table 131]

[Table EE06]

Table EE06

| Mixture No. | No. of compound that may be contained | Compound name | Compositional formula | Exact MS |
|---|---|---|---|---|
| mixED 10 | A3A001 | N-(1-Adamantylmethyl)-6-[4-[[2-[4-[3-(dimethylcarbamoyl)-4-hydroxynaphthalen-1-yl]phenyl]phenyl]methyl]piperazin-1-yl]-N-methylpyridazine-3-carboxamide | C47H52N6O3 | 748.41 |
| | A3A010 | N-(1-Adamantylmethyl)-6-[4-[[4-[4-[3-(dimethylcarbamoyl)-4-hydroxynaphthalen-1-yl]phenyl]naphthalen-1-yl]methyl]piperazin-1-yl]-N-methylpyridazine-3-carboxamide | C51H54N6O3 | 798.43 |
| | A3A011 | N-(1-Adamantylmethyl)-6-[4-[4-[4-[3-(dimethylcarbamoyl)-4-hydroxynaphthalen-1-yl]phenyl]naphthalene-1-carbonyl]piperazin-1-yl]-N-methylpyridazine-3-carboxamide | C51H52N6O4 | 812.41 |
| | A3A012 | N-(1-Adamantylmethyl)-6-[4-[[4-[4-[3-(dimethylcarbamoyl)-4-hydroxynaphthalen-1-yl]phenyl]thiophen-2-yl]methyl]piperazin-1-yl]-N-methylpyridazine-3-carboxamide | C45H50N6O3S | 754.37 |

| | | | | |
|---|---|---|---|---|
| | A3A013 | N-(1-Adamantylmethyl)-6-[4-[4-[4-[3-(dimethylcarbamoyl)-4-hydroxynaphthalen-1-yl]phenyl]thiophene-2-carbonyl]piperazin-1-yl]-N-methylpyridazine-3-carboxamide | C45H48N6O4S | 768.35 |
| | A3A014 | N-(1-Adamantylmethyl)-6-[4-[[3-[4-[3-(dimethylcarbamoyl)-4-hydroxynaphthalen-1-yl]phenyl]-5-(trifluoromethyl)phenyl]methyl]piperazin-1-yl]-N-methylpyridazine-3-carboxamide | C48H51F3N6O3 | 816.4 |
| | A3A015 | N-(1-Adamantylmethyl)-6-[4-[3-[4-[3-(dimethylcarbamoyl)-4-hydroxynaphthalen-1-yl]phenyl]-5-(trifluoromethyl)benzoyl]piperazin-1-yl]-N-methylpyridazine-3-carboxamide | C48H49F3N6O4 | 830.38 |
| | A3A016 | N-(1-Adamantylmethyl)-6-[4-[[3-[4-[3-(dimethylcarbamoyl)-4-hydroxynaphthalen-1-yl]phenyl]-5-(trifluoromethoxy)phenyl]methyl]piperazin-1-yl]-N-methylpyridazine-3-carboxamide | C48H51F3N6O4 | 832.39 |
| | A3A017 | N-(1-Adamantylmethyl)-6-[4-[[3-[4-[3-(dimethylcarbamoyl)-4-hydroxynaphthalen-1-yl]phenyl]-5-ethoxyphenyl]methyl]piperazin-1-yl]-N-methylpyridazine-3-carboxamide | C49H56N6O4 | 792.44 |
| | A3A018 | N-(1-Adamantylmethyl)-6-[4-[[3-[4-[3-(dimethylcarbamoyl)-4-hydroxynaphthalen-1-yl]phenyl]-5-propan-2-yloxyphenyl]methyl]piperazin-1-yl]-N-methylpyridazine-3-carboxamide | C50H58N6O4 | 806.45 |
| | A3A019 | N-(1-Adamantylmethyl)-6-[4-[3-tert-butyl-5-[4-[3-(dimethylcarbamoyl)-4-hydroxynaphthalen-1-yl]phenyl]benzoyl]piperazin-1-yl]-N-methylpyridazine-3-carboxamide | C51H58N6O4 | 818.45 |
| | A3A002 | N-(1-Adamantylmethyl)-6-[4-[3-[2-[4-[3-(dimethylcarbamoyl)-4-hydroxynaphthalen-1-yl]phenyl]phenyl]propanoyl]piperazin-1-yl]-N-methylpyridazine-3-carboxamide | C49H54N6O4 | 790.42 |
| | A3A020 | N-(1-Adamantylmethyl)-6-[4-[[2-[2-[4-[3-(dimethylcarbamoyl)-4-hydroxynaphthalen-1-yl]phenyl]ethynyl]phenyl]methyl]piperazin-1-yl]-N-methylpyridazine-3-carboxamide | C49H52N6O3 | 772.41 |
| | A3A021 | N-(1-Adamantylmethyl)-6-[4-[3-[2-[4-[3-(dimethylcarbamoyl)-4-hydroxynaphthalen-1-yl]phenyl]ethynyl]-5-(trifluoromethyl)benzoyl]piperazin-1-yl]-N-methylpyridazine-3-carboxamide | C50H49F3N6O4 | 854.38 |
| | A3A022 | N-(1-Adamantylmethyl)-6-[4-[5-[2-[4-[3-(dimethylcarbamoyl)-4-hydroxynaphthalen-1-yl]phenyl]ethynyl]pyridine-3-carbonyl]piperazin-1-yl]-N-methylpyridazine-3-carboxamide | C48H49N7O4 | 787.38 |
| | A3A023 | N-(1-Adamantylmethyl)-6-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]-N-methylpyridazine-3-carboxamide | C51H51F3N8O4 | 896.4 |
| | A3A024 | N-(1-Adamantylmethyl)-6-[4-[[2-[6-[3-(dimethylcarbamoyl)-4-hydroxynaphthalen-1-yl]pyridin-3-yl]phenyl]methyl]piperazin-1-yl]-N-methylpyridazine-3-carboxamide | C46H51N7O3 | 749.41 |
| | A3A025 | N-(1-Adamantylmethyl)-6-[4-[3-[2-[6-[3-(dimethylcarbamoyl)-4-hydroxynaphthalen-1-yl]pyridin-3-yl]phenyl]propanoyl]piperazin-1-yl]-N-methylpyridazine-3-carboxamide | C48H53N7O4 | 791.42 |
| | A3A026 | N-(1-Adamantylmethyl)-6-[4-[[4-[6-[3-(dimethylcarbamoyl)-4-hydroxynaphthalen-1-yl]pyridin-3-yl]-3-methylphenyl]methyl]piperazin-1-yl]-N-methylpyridazine-3-carboxamide | C47H53N7O3 | 763.42 |
| | A3A027 | N-(1-Adamantylmethyl)-6-[4-[4-[6-[3-(dimethylcarbamoyl)-4-hydroxynaphthalen-1-yl]pyridin-3-yl]-3-methylbenzoyl]piperazin-1-yl]-N-methylpyridazine-3-carboxamide | C47H51N7O4 | 777.4 |
| | A3A028 | N-(1-Adamantylmethyl)-6-[4-[[4-[6-[3-(dimethylcarbamoyl)-4-hydroxynaphthalen-1-yl]pyridin-3-yl]-2-ethylphenyl]methyl]piperazin-1-yl]-N-methylpyridazine-3-carboxamide | C48H55N7O3 | 777.44 |
| | A3A029 | N-(1-Adamantylmethyl)-6-[4-[[4-[6-[3-(dimethylcarbamoyl)-4-hydroxynaphthalen-1-yl]pyridin-3-yl]-3-fluorophenyl]methyl]piperazin-1-yl]-N-methylpyridazine-3-carboxamide | C46H50FN7O3 | 767.4 |
| | A3A003 | N-(1-Adamantylmethyl)-6-[4-[[4-[4-[3-(dimethylcarbamoyl)-4-hydroxynaphthalen-1-yl]phenyl]-3-methylphenyl]methyl]piperazin-1-yl]-N-methylpyridazine-3-carboxamide | C48H54N6O3 | 762.43 |
| mixED 10 | A3A030 | N-(1-Adamantylmethyl)-6-[4-[4-[6-[3-(dimethylcarbamoyl)-4-hydroxynaphthalen-1-yl]pyridin-3-yl]-3-fluorobenzoyl]piperazin-1-yl]-N-methylpyridazine-3-carboxamide | C46H48FN7O4 | 781.38 |
| | A3A031 | N-(1-Adamantylmethyl)-6-[4-[[4-[6-[3-(dimethylcarbamoyl)-4-hydroxynaphthalen-1-yl]pyridin-3-yl]-2-methoxyphenyl]methyl]piperazin-1-yl]-N-methylpyridazine-3-carboxamide | C47H53N7O4 | 779.42 |
| | A3A032 | N-(1-Adamantylmethyl)-6-[4-[[4-[6-[3-(dimethylcarbamoyl)-4-hydroxynaphthalen-1-yl]pyridin-3-yl]-2,6-dimethoxyphenyl]methyl]piperazin-1-yl]-N-methylpyridazine-3-carboxamide | C48H55N7O5 | 809.43 |
| | A3A033 | N-(1-Adamantylmethyl)-6-[4-[[4-[6-[3-(dimethylcarbamoyl)-4-hydroxynaphthalen-1-yl]pyridin-3-yl]naphthalen-1-yl]methyl]piperazin-1-yl]-N-methylpyridazine-3-carboxamide | C50H53N7O3 | 799.42 |

| | | | | |
|---|---|---|---|---|
| | A3A034 | N-(1-Adamantylmethyl)-6-[4-[4-[6-[3-(dimethylcarbamoyl)-4-hydroxynaphthalen-1-yl]pyridin-3-yl]naphthalene-1-carbonyl]piperazin-1-yl]-N-methylpyridazine-3-carboxamide | C50H51N7O4 | 813.4 |
| | A3A035 | N-(1-Adamantylmethyl)-6-[4-[[4-[6-[3-(dimethylcarbamoyl)-4-hydroxynaphthalen-1-yl]pyridin-3-yl]thiophen-2-yl]methyl]piperazin-1-yl]-N-methylpyridazine-3-carboxamide | C44H49N7O3S | 755.36 |
| | A3A036 | N-(1-Adamantylmethyl)-6-[4-[4-[6-[3-(dimethylcarbamoyl)-4-hydroxynaphthalen-1-yl]pyridin-3-yl]thiophene-2-carbonyl]piperazin-1-yl]-N-methylpyridazine-3-carboxamide | C44H47N7O4S | 769.34 |
| | A3A037 | N-(1-Adamantylmethyl)-6-[4-[[3-[6-[3-(dimethylcarbamoyl)-4-hydroxynaphthalen-1-yl]pyridin-3-yl]-5-(trifluoromethyl)phenyl]methyl]piperazin-1-yl]-N-methylpyridazine-3-carboxamide | C47H50F3N7O3 | 817.39 |
| | A3A038 | N-(1-Adamantylmethyl)-6-[4-[3-[6-[3-(dimethylcarbamoyl)-4-hydroxynaphthalen-1-yl]pyridin-3-yl]-5-(trifluoromethyl)benzoyl]piperazin-1-yl]-N-methylpyridazine-3-carboxamide | C47H48F3N7O4 | 831.37 |
| | A3A039 | N-(1-Adamantylmethyl)-6-[4-[[3-[6-[3-(dimethylcarbamoyl)-4-hydroxynaphthalen-1-yl]pyridin-3-yl]-5-(trifluoromethoxy)phenyl]methyl]piperazin-1-yl]-N-methylpyridazine-3-carboxamide | C47H50F3N7O4 | 833.39 |

[Table 132]

| | | | | |
|---|---|---|---|---|
| mixED 10 | A3A004 | N-(1-Adamantylmethyl)-6-[4-[4-[4-[3-(dimethylcarbamoyl)-4-hydroxynaphthalen-1-yl]phenyl]-3-methylbenzoyl]piperazin-1-yl]-N-methylpyridazine-3-carboxamide | C48H52N6O4 | 776.41 |
| | A3A040 | N-(1-Adamantylmethyl)-6-[4-[[3-[6-[3-(dimethylcarbamoyl)-4-hydroxynaphthalen-1-yl]pyridin-3-yl]-5-ethoxyphenyl]methyl]piperazin-1-yl]-N-methylpyridazine-3-carboxamide | C48H55N7O4 | 793.43 |
| | A3A041 | N-(1-Adamantylmethyl)-6-[4-[[3-[6-[3-(dimethylcarbamoyl)-4-hydroxynaphthalen-1-yl]pyridin-3-yl]-5-propan-2-yloxyphenyl]methyl]piperazin-1-yl]-N-methylpyridazine-3-carboxamide | C49H57N7O4 | 807.45 |
| | A3A042 | N-(1-Adamantylmethyl)-6-[4-[3-tert-butyl-5-[6-[3-(dimethylcarbamoyl)-4-hydroxynaphthalen-1-yl]pyridin-3-yl]benzoyl]piperazin-1-yl]-N-methylpyridazine-3-carboxamide | C50H57N7O4 | 819.45 |
| | A3A043 | N-(1-Adamantylmethyl)-6-[4-[[2-[2-[6-[3-(dimethylcarbamoyl)-4-hydroxynaphthalen-1-yl]pyridin-3-yl]ethynyl]phenyl]methyl]piperazin-1-yl]-N-methylpyridazine-3-carboxamide | C48H51N7O3 | 773.41 |
| | A3A044 | N-(1-Adamantylmethyl)-6-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]-N-methylpyridazine-3-carboxamide | C49H48F3N7O4 | 855.37 |
| | A3A045 | N-(1-Adamantylmethyl)-6-[4-[5-[2-[6-[3-(dimethylcarbamoyl)-4-hydroxynaphthalen-1-yl]pyridin-3-yl]ethynyl]pyridine-3-carbonyl]piperazin-1-yl]-N-methylpyridazine-3-carboxamide | C47H48N8O4 | 788.38 |
| | A3A046 | N-(1-Adamantylmethyl)-6-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]-N-methylpyridazine-3-carboxamide | C50H50F3N9O4 | 897.39 |
| | A3A047 | 4-[4-[2-[[4-[4-[1-Adamantylmethyl(methyl)carbamoyl]phenyl]piperazin-1-yl]methyl]phenyl]phenyl]-1-hydroxy-N,N-dimethylnaphthalene-2-carboxamide | C49H54N4O3 | 746.42 |
| | A3A048 | 4-[4-[2-[3-[4-[4-[1-Adamantylmethyl(methyl)carbamoyl]phenyl]piperazin-1-yl]-3-oxopropyl]phenyl]phenyl]-1-hydroxy-N,N-dimethylnaphthalene-2-carboxamide | C51H56N4O4 | 788.43 |
| | A3A049 | 4-[4-[4-[1-[4-[4-[1-Adamantylmethyl(methyl)carbamoyl]phenyl]piperazin-1-yl]ethyl]phenyl]phenyl]-1-hydroxy-N,N-dimethylnaphthalene-2-carboxamide | C50H56N4O3 | 760.44 |
| | A3A005 | N-(1-Adamantylmethyl)-6-[4-[[4-[4-[3-(dimethylcarbamoyl)-4-hydroxynaphthalen-1-yl]phenyl]-2-ethylphenyl]methyl]piperazin-1-yl]-N-methylpyridazine-3-carboxamide | C49H56N6O3 | 776.44 |
| | A3A050 | 4-[4-[4-[[4-[4-[1-Adamantylmethyl(methyl)carbamoyl]phenyl]piperazin-1-yl]methyl]-2-methylphenyl]phenyl]-1-hydroxy-N,N-dimethylnaphthalene-2-carboxamide | C50H56N4O3 | 760.44 |
| | A3A051 | 4-[4-[4-[4-[4-[1-Adamantylmethyl(methyl)carbamoyl]phenyl]piperazine-1-carbonyl]-2-methylphenyl]phenyl]-1-hydroxy-N,N-dimethylnaphthalene-2-carboxamide | C50H54N4O4 | 774.41 |
| | A3A052 | 4-[4-[4-[[4-[4-[1-Adamantylmethyl(methyl)carbamoyl]phenyl]piperazin-1-yl]methyl]-3-ethylphenyl]phenyl]-1-hydroxy-N,N-dimethylnaphthalene-2-carboxamide | C51H58N4O3 | 774.45 |
| | A3A053 | 4-[4-[4-[[4-[4-[1-Adamantylmethyl(methyl)carbamoyl]phenyl]piperazin-1-yl]methyl]-2-fluorophenyl]phenyl]-1-hydroxy-N,N-dimethylnaphthalene-2-carboxamide | C49H53FN4O3 | 764.41 |
| | A3A054 | 4-[4-[4-[4-[4-[1-Adamantylmethyl(methyl)carbamoyl]phenyl]piperazine-1-carbonyl]-2-fluorophenyl]phenyl]-1-hydroxy-N,N-dimethylnaphthalene-2-carboxamide | C49H51FN4O4 | 778.39 |

| | | | | |
|---|---|---|---|---|
| | A3A055 | 4-[4-[4-[[4-[4-[1-Adamantylmethyl(methyl)carbamoyl]phenyl]piperazin-1-yl]methyl]-3-methoxyphenyl]phenyl]-1-hydroxy-N,N-dimethylnaphthalene-2-carboxamide | C50H56N4O4 | 776.43 |
| | A3A056 | 4-[4-[4-[[4-[4-[1-Adamantylmethyl(methyl)carbamoyl]phenyl]piperazin-1-yl]methyl]-3,5-dimethoxyphenyl]phenyl]-1-hydroxy-N,N-dimethylnaphthalene-2-carboxamide | C51H58N4O5 | 806.44 |
| | A3A057 | 4-[4-[4-[[4-[4-[1-Adamantylmethyl(methyl)carbamoyl]phenyl]piperazin-1-yl]methyl]naphthalen-1-yl]phenyl]-1-hydroxy-N,N-dimethylnaphthalene-2-carboxamide | C53H56N4O3 | 796.44 |
| | A3A058 | 4-[4-[4-[4-[4-[1-Adamantylmethyl(methyl)carbamoyl]phenyl]piperazine-1-carbonyl]naphthalen-1-yl]phenyl]-1-hydroxy-N,N-dimethylnaphthalene-2-carboxamide | C53H54N4O4 | 810.41 |
| | A3A059 | 4-[4-[5-[[4-[4-[1-Adamantylmethyl(methyl)carbamoyl]phenyl]piperazin-1-yl]methyl]thiophen-3-yl]phenyl]-1-hydroxy-N,N-dimethylnaphthalene-2-carboxamide | C47H52N4O3S | 752.38 |
| | A3A006 | N-(1-Adamantylmethyl)-6-[4-[[4-[4-[3-(dimethylcarbamoyl)-4-hydroxynaphthalen-1-yl]phenyl]-3-fluorophenyl]methyl]piperazin-1-yl]-N-methylpyridazine-3-carboxamide | C47H51FN6O3 | 766.4 |
| | A3A060 | 4-[4-[5-[4-[4-[1-Adamantylmethyl(methyl)carbamoyl]phenyl]piperazine-1-carbonyl]thiophen-3-yl]phenyl]-1-hydroxy-N,N-dimethylnaphthalene-2-carboxamide | C47H50N4O4S | 766.36 |
| | A3A061 | 4-[4-[3-[[4-[4-[1-Adamantylmethyl(methyl)carbamoyl]phenyl]piperazin-1-yl]methyl]-5-(trifluoromethyl)phenyl]phenyl]-1-hydroxy-N,N-dimethylnaphthalene-2-carboxamide | C50H53F3N4O3 | 814.41 |
| | A3A062 | 4-[4-[3-[4-[4-[1-Adamantylmethyl(methyl)carbamoyl]phenyl]piperazine-1-carbonyl]-5-(trifluoromethyl)phenyl]phenyl]-1-hydroxy-N,N-dimethylnaphthalene-2-carboxamide | C50H51F3N4O4 | 828.39 |
| mixED 10 | A3A063 | 4-[4-[3-[[4-[4-[1-Adamantylmethyl(methyl)carbamoyl]phenyl]piperazin-1-yl]methyl]-5-(trifluoromethoxy)phenyl]phenyl]-1-hydroxy-N,N-dimethylnaphthalene-2-carboxamide | C50H53F3N4O4 | 830.4 |
| | A3A064 | 4-[4-[3-[[4-[4-[1-Adamantylmethyl(methyl)carbamoyl]phenyl]piperazin-1-yl]methyl]-5-ethoxyphenyl]phenyl]-1-hydroxy-N,N-dimethylnaphthalene-2-carboxamide | C51H58N4O4 | 790.45 |
| | A3A065 | 4-[4-[3-[[4-[4-[1-Adamantylmethyl(methyl)carbamoyl]phenyl]piperazin-1-yl]methyl]-5-propan-2-yloxyphenyl]phenyl]-1-hydroxy-N,N-dimethylnaphthalene-2-carboxamide | C52H60N4O4 | 804.46 |
| | A3A066 | 4-[4-[3-[4-[4-[1-Adamantylmethyl(methyl)carbamoyl]phenyl]piperazine-1-carbonyl]-5-tert-butylphenyl]phenyl]-1-hydroxy-N,N-dimethylnaphthalene-2-carboxamide | C53H60N4O4 | 816.46 |
| | A3A067 | 4-[4-[4-[1-[4-[4-[1-Adamantylmethyl(methyl)carbamoyl]phenyl]piperazin-1-yl]ethyl]-3-fluorophenyl]phenyl]-1-hydroxy-N,N-dimethylnaphthalene-2-carboxamide | C50H55FN4O3 | 778.43 |
| | A3A068 | 4-[4-[2-[2-[[4-[4-[1-Adamantylmethyl(methyl)carbamoyl]phenyl]piperazin-1-yl]methyl]phenyl]ethynyl]phenyl]-1-hydroxy-N,N-dimethylnaphthalene-2-carboxamide | C51H54N4O3 | 770.42 |
| | A3A069 | 4-[4-[2-[3-[4-[4-[1-Adamantylmethyl(methyl)carbamoyl]phenyl]piperazine-1-carbonyl]-5-(trifluoromethyl)phenyl]ethynyl]phenyl]-1-hydroxy-N,N-dimethylnaphthalene-2-carboxamide | C52H51F3N4O4 | 852.39 |

[Table 133]

| | | | | |
|---|---|---|---|---|
| mixED 10 | A3A007 | N-(1-Adamantylmethyl)-6-[4-[4-[4-[3-(dimethylcarbamoyl)-4-hydroxynaphthalen-1-yl]phenyl]-3-fluorobenzoyl]piperazin-1-yl]-N-methylpyridazine-3-carboxamide | C47H49FN6O4 | 780.38 |
| | A3A070 | 4-[4-[2-[5-[4-[4-[1-Adamantylmethyl(methyl)carbamoyl]phenyl]piperazine-1-carbonyl]pyridin-3-yl]ethynyl]phenyl]-1-hydroxy-N,N-dimethylnaphthalene-2-carboxamide | C50H51N5O4 | 785.39 |
| | A3A071 | 4-[4-[1-[3-[4-[4-[1-Adamantylmethyl(methyl)carbamoyl]phenyl]piperazine-1-carbonyl]-5-(trifluoromethyl)phenyl]pyrazol-4-yl]phenyl]-1-hydroxy-N,N-dimethylnaphthalene-2-carboxamide | C53H53F3N6O4 | 894.41 |
| | A3A072 | 4-[5-[2-[[4-[4-[1-Adamantylmethyl(methyl)carbamoyl]phenyl]piperazin-1-yl]methyl]phenyl]pyridin-2-yl]-1-hydroxy-N,N-dimethylnaphthalene-2-carboxamide | C48H53N5O3 | 747.41 |
| | A3A073 | 4-[5-[2-[3-[4-[4-[1-Adamantylmethyl(methyl)carbamoyl]phenyl]piperazin-1-yl]-3-oxopropyl]phenyl]pyridin-2-yl]-1-hydroxy-N,N-dimethylnaphthalene-2-carboxamide | C50H55N5O4 | 789.43 |
| | A3A074 | 4-[5-[4-[1-[4-[4-[1-Adamantylmethyl(methyl)carbamoyl]phenyl]piperazin-1-yl]ethyl]phenyl]pyridin-2-yl]-1-hydroxy-N,N-dimethylnaphthalene-2-carboxamide | C49H55N5O3 | 761.43 |
| | A3A075 | 4-[5-[4-[[4-[4-[1-Adamantylmethyl(methyl)carbamoyl]phenyl]piperazin-1-yl]methyl]-2-methylphenyl]pyridin-2-yl]-1-hydroxy-N,N-dimethylnaphthalene-2-carboxamide | C49H55N5O3 | 761.43 |
| | A3A076 | 4-[5-[4-[4-[4-[1-Adamantylmethyl(methyl)carbamoyl]phenyl]piperazine-1-carbonyl]-2-methylphenyl]pyridin-2-yl]-1-hydroxy-N,N-dimethylnaphthalene-2-carboxamide | C49H53N5O4 | 775.41 |
| | A3A077 | 4-[5-[4-[[4-[4-[1-Adamantylmethyl(methyl)carbamoyl]phenyl]piperazin-1-yl]methyl]-3-ethylphenyl]pyridin-2-yl]-1-hydroxy-N,N-dimethylnaphthalene-2-carboxamide | C50H57N5O3 | 775.45 |

| | | | | |
|---|---|---|---|---|
| | A3A078 | 4-[5-[4-[[4-[4-[1-Adamantylmethyl(methyl)carbamoyl]phenyl]piperazin-1-yl]methyl]-2-fluorophenyl]pyridin-2-yl]-1-hydroxy-N,N-dimethylnaphthalene-2-carboxamide | C48H52FN5O3 | 765.41 |
| | A3A079 | 4-[5-[4-[4-[4-[1-Adamantylmethyl(methyl)carbamoyl]phenyl]piperazine-1-carbonyl]-2-fluorophenyl]pyridin-2-yl]-1-hydroxy-N,N-dimethylnaphthalene-2-carboxamide | C48H50FN5O4 | 779.38 |
| | A3A008 | N-(1-Adamantylmethyl)-6-[4-[[4-[4-[3-(dimethylcarbamoyl)-4-hydroxynaphthalen-1-yl]phenyl]-2-methoxyphenyl]methyl]piperazin-1-yl]-N-methylpyridazine-3-carboxamide | C48H54N6O4 | 778.42 |
| | A3A080 | 4-[5-[4-[[4-[4-[1-Adamantylmethyl(methyl)carbamoyl]phenyl]piperazin-1-yl]methyl]-3-methoxyphenyl]pyridin-2-yl]-1-hydroxy-N,N-dimethylnaphthalene-2-carboxamide | C49H55N5O4 | 777.43 |
| | A3A081 | 4-[5-[4-[[4-[4-[1-Adamantylmethyl(methyl)carbamoyl]phenyl]piperazin-1-yl]methyl]-3,5-dimethoxyphenyl]pyridin-2-yl]-1-hydroxy-N,N-dimethylnaphthalene-2-carboxamide | C50H57N5O5 | 807.44 |
| | A3A082 | 4-[5-[4-[[4-[4-[1-Adamantylmethyl(methyl)carbamoyl]phenyl]piperazin-1-yl]methyl]naphthalen-1-yl]pyridin-2-yl]-1-hydroxy-N,N-dimethylnaphthalene-2-carboxamide | C52H55N5O3 | 797.43 |
| | A3A083 | 4-[5-[4-[4-[4-[1-Adamantylmethyl(methyl)carbamoyl]phenyl]piperazine-1-carbonyl]naphthalen-1-yl]pyridin-2-yl]-1-hydroxy-N,N-dimethylnaphthalene-2-carboxamide | C52H53N5O4 | 811.41 |
| | A3A084 | 4-[5-[5-[[4-[4-[1-Adamantylmethyl(methyl)carbamoyl]phenyl]piperazin-1-yl]methyl]thiophen-3-yl]pyridin-2-yl]-1-hydroxy-N,N-dimethylnaphthalene-2-carboxamide | C46H51N5O3S | 753.37 |
| mixED 10 | A3A085 | 4-[5-[5-[4-[4-[1-Adamantylmethyl(methyl)carbamoyl]phenyl]piperazine-1-carbonyl]thiophen-3-yl]pyridin-2-yl]-1-hydroxy-N,N-dimethylnaphthalene-2-carboxamide | C46H49N5O4S | 767.35 |
| | A3A086 | 4-[5-[3-[[4-[4-[1-Adamantylmethyl(methyl)carbamoyl]phenyl]piperazin-1-yl]methyl]-5-(trifluoromethyl)phenyl]pyridin-2-yl]-1-hydroxy-N,N-dimethylnaphthalene-2-carboxamide | C49H52F3N5O3 | 815.4 |
| | A3A087 | 4-[5-[3-[4-[4-[1-Adamantylmethyl(methyl)carbamoyl]phenyl]piperazine-1-carbonyl]-5-(trifluoromethyl)phenyl]pyridin-2-yl]-1-hydroxy-N,N-dimethylnaphthalene-2-carboxamide | C49H50F3N5O4 | 829.38 |
| | A3A088 | 4-[5-[3-[[4-[4-[1-Adamantylmethyl(methyl)carbamoyl]phenyl]piperazin-1-yl]methyl]-5-(trifluoromethoxy)phenyl]pyridin-2-yl]-1-hydroxy-N,N-dimethylnaphthalene-2-carboxamide | C49H52F3N5O4 | 831.4 |
| | A3A089 | 4-[5-[3-[[4-[4-[1-Adamantylmethyl(methyl)carbamoyl]phenyl]piperazin-1-yl]methyl]-5-ethoxyphenyl]pyridin-2-yl]-1-hydroxy-N,N-dimethylnaphthalene-2-carboxamide | C50H57N5O4 | 791.44 |
| | A3A009 | N-(1-Adamantylmethyl)-6-[4-[[4-[4-[3-(dimethylcarbamoyl)-4-hydroxynaphthalen-1-yl]phenyl]-2,6-dimethoxyphenyl]methyl]piperazin-1-yl]-N-methylpyridazine-3-carboxamide | C49H56N6O5 | 808.43 |
| | A3A090 | 4-[5-[3-[[4-[4-[1-Adamantylmethyl(methyl)carbamoyl]phenyl]piperazin-1-yl]methyl]-5-propan-2-yloxyphenyl]pyridin-2-yl]-1-hydroxy-N,N-dimethylnaphthalene-2-carboxamide | C51H59N5O4 | 805.46 |
| | A3A091 | 4-[5-[3-[4-[4-[1-Adamantylmethyl(methyl)carbamoyl]phenyl]piperazine-1-carbonyl]-5-tert-butylphenyl]pyridin-2-yl]-1-hydroxy-N,N-dimethylnaphthalene-2-carboxamide | C52H59N5O4 | 817.46 |
| | A3A092 | 4-[5-[4-[1-[4-[4-[1-Adamantylmethyl(methyl)carbamoyl]phenyl]piperazin-1-yl]ethyl]-3-fluorophenyl]pyridin-2-yl]-1-hydroxy-N,N-dimethylnaphthalene-2-carboxamide | C49H54FN5O3 | 779.42 |
| | A3A093 | 4-[5-[2-[2-[[4-[4-[1-Adamantylmethyl(methyl)carbamoyl]phenyl]piperazin-1-yl]methyl]phenyl]ethynyl]pyridin-2-yl]-1-hydroxy-N,N-dimethylnaphthalene-2-carboxamide | C50H53N5O3 | 771.41 |
| | A3A094 | 4-[5-[2-[3-[4-[4-[1-Adamantylmethyl(methyl)carbamoyl]phenyl]piperazine-1-carbonyl]-5-(trifluoromethyl)phenyl]ethynyl]pyridin-2-yl]-1-hydroxy-N,N-dimethylnaphthalene-2-carboxamide | C51H50F3N5O4 | 853.38 |
| | A3A095 | 4-[5-[2-[5-[4-[4-[1-Adamantylmethyl(methyl)carbamoyl]phenyl]piperazine-1-carbonyl]pyridin-3-yl]ethynyl]pyridin-2-yl]-1-hydroxy-N,N-dimethylnaphthalene-2-carboxamide | C49H50N6O4 | 786.39 |
| | A3A096 | 4-[5-[1-[3-[4-[4-[1-Adamantylmethyl(methyl)carbamoyl]phenyl]piperazine-1-carbonyl]-5-(trifluoromethyl)phenyl]pyrazol-4-yl]pyridin-2-yl]-1-hydroxy-N,N-dimethylnaphthalene-2-carboxamide | C52H52F3N7O4 | 895.4 |
| | A3A097 | N-(1-Adamantylmethyl)-6-[4-[2-[2-[4-[3-(dimethylcarbamoyl)-4-hydroxynaphthalen-1-yl]phenyl]ethynyl]benzoyl]piperazin-1-yl]-N-methylpyridazine-3-carboxamide | C49H50N6O4 | 786.389 |
| | A3A098 | N-(1-Adamantylmethyl)-6-[4-[2-[2-[6-[3-(dimethylcarbamoyl)-4-hydroxynaphthalen-1-yl]pyridin-3-yl]ethynyl]benzoyl]piperazin-1-yl]-N-methylpyridazine-3-carboxamide | C48H49N7O4 | 787.385 |

| | | | | |
|---|---|---|---|---|
| | A3A099 | 4-[4-[2-[2-[4-[4-[1-Adamantylmethyl(methyl)carbamoyl]phenyl]piperazine-1-carbonyl]phenyl]ethynyl]phenyl]-1-hydroxy-N,N-dimethylnaphthalene-2-carboxamide | C51H52N4O4 | 784.399 |
| | A3A100 | 4-[5-[2-[2-[4-[4-[1-Adamantylmethyl(methyl)carbamoyl]phenyl]piperazine-1-carbonyl]phenyl]ethynyl]pyridin-2-yl]-1-hydroxy-N,N-dimethylnaphthalene-2-carboxamide | C50H51N5O4 | 785.394 |

[Table 134]

[Table EE07]

Table EE07

| Mixture No. | No. of compound that may be contained | Compound name | Compositional formula | Exact MS |
|---|---|---|---|---|
| mixED 11 | A2I001 | 6-[4-[[2-[4-[3-(Dimethylcarbamoyl)-4-hydroxynaphthalen-1-yl]phenyl]phenyl]methyl]piperazin-1-yl]-N-methylsulfonylpyridazine-3-carboxamide | C36H36N6O5S | 664.25 |
| | A2I010 | 6-[4-[[4-[4-[3-(Dimethylcarbamoyl)-4-hydroxynaphthalen-1-yl]phenyl]naphthalen-1-yl]methyl]piperazin-1-yl]-N-methylsulfonylpyridazine-3-carboxamide | C40H38N6O5S | 714.26 |
| | A2I011 | 6-[4-[4-[4-[3-(Dimethylcarbamoyl)-4-hydroxynaphthalen-1-yl]phenyl]naphthalene-1-carbonyl]piperazin-1-yl]-N-methylsulfonylpyridazine-3-carboxamide | C40H36N6O6S | 728.24 |
| mixED 11 | A2I012 | 6-[4-[[4-[4-[3-(Dimethylcarbamoyl)-4-hydroxynaphthalen-1-yl]phenyl]thiophen-2-yl]methyl]piperazin-1-yl]-N-methylsulfonylpyridazine-3-carboxamide | C34H34N6O5S2 | 670.2 |
| | A2I013 | 6-[4-[4-[4-[3-(Dimethylcarbamoyl)-4-hydroxynaphthalen-1-yl]phenyl]thiophene-2-carbonyl]piperazin-1-yl]-N-methylsulfonylpyridazine-3-carboxamide | C34H32N6O6S2 | 684.18 |
| | A2I014 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-methylsulfonylpyridazine-3-carboxamide | C37H35F3N6O5S | 732.23 |
| | A2I015 | 6-[4-[3-[4-[3-(Dimethylcarbamoyl)-4-hydroxynaphthalen-1-yl]phenyl]-5-(trifluoromethyl)benzoyl]piperazin-1-yl]-N-methylsulfonylpyridazine-3-carboxamide | C37H33F3N6O6S | 746.21 |
| | A2I016 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-methylsulfonylpyridazine-3-carboxamide | C37H35F3N6O6S | 748.23 |
| | A2I017 | 6-[4-[[3-[4-[3-(Dimethylcarbamoyl)-4-hydroxynaphthalen-1-yl]phenyl]-5-ethoxyphenyl]methyl]piperazin-1-yl]-N-methylsulfonylpyridazine-3-carboxamide | C38H40N6O6S | 708.27 |
| | A2I018 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-methylsulfonylpyridazine-3-carboxamide | C39H42N6O6S | 722.29 |
| | A2I019 | 6-[4-[3-tert-Butyl-5-[4-[3-(dimethylcarbamoyl)-4-hydroxynaphthalen-1-yl]phenyl]benzoyl]piperazin-1-yl]-N-methylsulfonylpyridazine-3-carboxamide | C40H42N6O6S | 734.29 |
| | A2I002 | 6-[4-[3-[2-[4-[3-(Dimethylcarbamoyl)-4-hydroxynaphthalen-1-yl]phenyl]phenyl]propanoyl]piperazin-1-yl]-N-methylsulfonylpyridazine-3-carboxamide | C38H38N6O6S | 706.26 |
| | A2I020 | 6-[4-[[2-[2-[4-[3-(Dimethylcarbamoyl)-4-hydroxynaphthalen-1-yl]phenyl]ethynyl]phenyl]methyl]piperazin-1-yl]-N-methylsulfonylpyridazine-3-carboxamide | C38H36N6O5S | 688.25 |
| | A2I021 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-methylsulfonylpyridazine-3-carboxamide | C39H33F3N6O6S | 770.21 |
| | A2I022 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-methylsulfonylpyridazine-3-carboxamide | C37H33N7O6S | 703.22 |
| | A2I023 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-methylsulfonylpyridazine-3-carboxamide | C40H35F3N8O6S | 812.24 |
| | A2I024 | 6-[4-[[2-[6-[3-(Dimethylcarbamoyl)-4-hydroxynaphthalen-1-yl]pyridin-3-yl]phenyl]methyl]piperazin-1-yl]-N-methylsulfonylpyridazine-3-carboxamide | C35H35N7O5S | 665.24 |
| | A2I025 | 6-[4-[3-[2-[6-[3-(Dimethylcarbamoyl)-4-hydroxynaphthalen-1-yl]pyridin-3-yl]phenyl]propanoyl]piperazin-1-yl]-N-methylsulfonylpyridazine-3-carboxamide | C37H37N7O6S | 707.25 |
| | A2I026 | 6-[4-[[4-[6-[3-(Dimethylcarbamoyl)-4-hydroxynaphthalen-1-yl]pyridin-3-yl]-3-methylphenyl]methyl]piperazin-1-yl]-N-methylsulfonylpyridazine-3-carboxamide | C36H37N7O5S | 679.26 |
| | A2I027 | 6-[4-[4-[6-[3-(Dimethylcarbamoyl)-4-hydroxynaphthalen-1-yl]pyridin-3-yl]-3-methylbenzoyl]piperazin-1-yl]-N-methylsulfonylpyridazine-3-carboxamide | C36H35N7O6S | 693.24 |

| | | | | |
|---|---|---|---|---|
| | A2I028 | 6-[4-[[4-[6-[3-(Dimethylcarbamoyl)-4-hydroxynaphthalen-1-yl]pyridin-3-yl]-2-ethylphenyl]methyl]piperazin-1-yl]-N-methylsulfonylpyridazine-3-carboxamide | C37H39N7O5S | 693.27 |
| | A2I029 | 6-[4-[[4-[6-[3-(Dimethylcarbamoyl)-4-hydroxynaphthalen-1-yl]pyridin-3-yl]-3-fluorophenyl]methyl]piperazin-1-yl]-N-methylsulfonylpyridazine-3-carboxamide | C35H34FN7O5S | 683.23 |
| | A2I003 | 6-[4-[[4-[4-[3-(Dimethylcarbamoyl)-4-hydroxynaphthalen-1-yl]phenyl]-3-methylphenyl]methyl]piperazin-1-yl]-N-methylsulfonylpyridazine-3-carboxamide | C37H38N6O5S | 678.26 |
| | A2I030 | 6-[4-[4-[6-[3-(Dimethylcarbamoyl)-4-hydroxynaphthalen-1-yl]pyridin-3-yl]-3-fluorobenzoyl]piperazin-1-yl]-N-methylsulfonylpyridazine-3-carboxamide | C35H32FN7O6S | 697.21 |
| | A2I031 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-methylsulfonylpyridazine-3-carboxamide | C36H37N7O6S | 695.25 |
| | A2I032 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-methylsulfonylpyridazine-3-carboxamide | C37H39N7O7S | 725.26 |
| | A2I033 | 6-[4-[[4-[6-[3-(Dimethylcarbamoyl)-4-hydroxynaphthalen-1-yl]pyridin-3-yl]naphthalen-1-yl]methyl]piperazin-1-yl]-N-methylsulfonylpyridazine-3-carboxamide | C39H37N7O5S | 715.26 |
| | A2I034 | 6-[4-[4-[6-[3-(Dimethylcarbamoyl)-4-hydroxynaphthalen-1-yl]pyridin-3-yl]naphthalene-1-carbonyl]piperazin-1-yl]-N-methylsulfonylpyridazine-3-carboxamide | C39H35N7O6S | 729.24 |
| | A2I035 | 6-[4-[[4-[6-[3-(Dimethylcarbamoyl)-4-hydroxynaphthalen-1-yl]pyridin-3-yl]thiophen-2-yl]methyl]piperazin-1-yl]-N-methylsulfonylpyridazine-3-carboxamide | C33H33N7O5S2 | 671.2 |
| mixED 11 | A2I036 | 6-[4-[4-[6-[3-(Dimethylcarbamoyl)-4-hydroxynaphthalen-1-yl]pyridin-3-yl]thiophene-2-carbonyl]piperazin-1-yl]-N-methylsulfonylpyridazine-3-carboxamide | C33H31N7O6S2 | 685.18 |
| | A2I037 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-methylsulfonylpyridazine-3-carboxamide | C36H34F3N7O5S | 733.23 |
| | A2I038 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-methylsulfonylpyridazine-3-carboxamide | C36H32F3N7O6S | 747.21 |
| | A2I039 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-methylsulfonylpyridazine-3-carboxamide | C36H34F3N7O6S | 749.22 |

[Table 135]

| | | | | |
|---|---|---|---|---|
| mixED 11 | A2I004 | 6-[4-[4-[4-[3-(Dimethylcarbamoyl)-4-hydroxynaphthalen-1-yl]phenyl]-3-methylbenzoyl]piperazin-1-yl]-N-methylsulfonylpyridazine-3-carboxamide | C37H36N6O6S | 692.24 |
| | A2I040 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-methylsulfonylpyridazine-3-carboxamide | C37H39N7O6S | 709.27 |
| | A2I041 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-methylsulfonylpyridazine-3-carboxamide | C38H41N7O6S | 723.28 |
| | A2I042 | 6-[4-[3-tert-Butyl-5-[6-[3-(dimethylcarbamoyl)-4-hydroxynaphthalen-1-yl]pyridin-3-yl]benzoyl]piperazin-1-yl]-N-methylsulfonylpyridazine-3-carboxamide | C39H41N7O6S | 735.28 |
| | A2I043 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-methylsulfonylpyridazine-3-carboxamide | C37H35N7O5S | 689.24 |
| | A2I044 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-methylsulfonylpyridazine-3-carboxamide | C38H32F3N7O6S | 771.21 |
| | A2I045 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-methylsulfonylpyridazine-3-carboxamide | C36H32N8O6S | 704.22 |
| | A2I046 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-methylsulfonylpyridazine-3-carboxamide | C39H34F3N9O6S | 813.23 |
| | A2I047 | 1-Hydroxy-N,N-dimethyl-4-[4-[2-[[4-[4-(methylsulfonylcarbamoyl)phenyl]piperazin-1-yl]methyl]phenyl]phenyl]naphthalene-2-carboxamide | C38H38N4O5S | 662.26 |
| | A2I048 | 1-Hydroxy-N,N-dimethyl-4-[4-[2-[3-[4-[4-(methylsulfonylcarbamoyl)phenyl]piperazin-1-yl]-3-oxopropyl]phenyl]phenyl]naphthalene-2-carboxamide | C40H40N4O6S | 704.27 |
| | A2I049 | 1-Hydroxy-N,N-dimethyl-4-[4-[4-[1-[4-[4-(methylsulfonylcarbamoyl)phenyl]piperazin-1-yl]ethyl]phenyl]phenyl]naphthalene-2-carboxamide | C39H40N4O5S | 676.27 |
| | A2I005 | 6-[4-[[4-[4-[3-(Dimethylcarbamoyl)-4-hydroxynaphthalen-1-yl]phenyl]-2-ethylphenyl]methyl]piperazin-1-yl]-N-methylsulfonylpyridazine-3-carboxamide | C38H40N6O5S | 692.28 |
| | A2I050 | 1-Hydroxy-N,N-dimethyl-4-[4-[2-methyl-4-[[4-[4-(methylsulfonylcarbamoyl)phenyl]piperazin-1-yl]methyl]phenyl]phenyl]naphthalene-2-carboxamide | C39H40N4O5S | 676.27 |

| | | | | |
|---|---|---|---|---|
| | A2I051 | 1-Hydroxy-N,N-dimethyl-4-[4-[2-methyl-4-[4-[4-(methylsulfonylcarbamoyl)phenyl]piperazine-1-carbonyl]phenyl]phenyl]naphthalene-2-carboxamide | C39H38N4O6S | 690.25 |
| | A2I052 | 4-[4-[3-Ethyl-4-[[4-[4-(methylsulfonylcarbamoyl)phenyl]piperazin-1-yl]methyl]phenyl]phenyl]-1-hydroxy-N,N-dimethylnaphthalene-2-carboxamide | C40H42N4O5S | 690.29 |
| | A2I053 | 4-[4-[2-Fluoro-4-[[4-[4-(methylsulfonylcarbamoyl)phenyl]piperazin-1-yl]methyl]phenyl]phenyl]-1-hydroxy-N,N-dimethylnaphthalene-2-carboxamide | C38H37FN4O5S | 680.25 |
| | A2I054 | 4-[4-[2-Fluoro-4-[4-[4-(methylsulfonylcarbamoyl)phenyl]piperazine-1-carbonyl]phenyl]phenyl]-1-hydroxy-N,N-dimethylnaphthalene-2-carboxamide | C38H35FN4O6S | 694.23 |
| | A2I055 | 1-Hydroxy-4-[4-[3-methoxy-4-[[4-[4-(methylsulfonylcarbamoyl)phenyl]piperazin-1-yl]methyl]phenyl]phenyl]-N,N-dimethylnaphthalene-2-carboxamide | C39H40N4O6S | 692.27 |
| | A2I056 | 4-[4-[3,5-Dimethoxy-4-[[4-[4-(methylsulfonylcarbamoyl)phenyl]piperazin-1-yl]methyl]phenyl]phenyl]-1-hydroxy-N,N-dimethylnaphthalene-2-carboxamide | C40H42N4O7S | 722.28 |
| | A2I057 | 1-Hydroxy-N,N-dimethyl-4-[4-[4-[[4-[4-(methylsulfonylcarbamoyl)phenyl]piperazin-1-yl]methyl]naphthalen-1-yl]phenyl]naphthalene-2-carboxamide | C42H40N4O5S | 712.27 |
| mixED 11 | A2I058 | 1-Hydroxy-N,N-dimethyl-4-[4-[4-[4-[4-(methylsulfonylcarbamoyl)phenyl]piperazine-1-carbonyl]naphthalen-1-yl]phenyl]naphthalene-2-carboxamide | C42H38N4O6S | 726.25 |
| | A2I059 | 1-Hydroxy-N,N-dimethyl-4-[4-[5-[[4-[4-(methylsulfonylcarbamoyl)phenyl]piperazin-1-yl]methyl]thiophen-3-yl]phenyl]naphthalene-2-carboxamide | C36H36N4O5S2 | 668.21 |
| | A2I006 | 6-[4-[[4-[4-[3-(Dimethylcarbamoyl)-4-hydroxynaphthalen-1-yl]phenyl]-3-fluorophenyl]methyl]piperazin-1-yl]-N-methylsulfonylpyridazine-3-carboxamide | C36H35FN6O5S | 682.24 |
| | A2I060 | 1-Hydroxy-N,N-dimethyl-4-[4-[5-[4-[4-(methylsulfonylcarbamoyl)phenyl]piperazine-1-carbonyl]thiophen-3-yl]phenyl]naphthalene-2-carboxamide | C36H34N4O6S2 | 682.19 |
| | A2I061 | 1-Hydroxy-N,N-dimethyl-4-[4-[3-[[4-[4-(methylsulfonylcarbamoyl)phenyl]piperazin-1-yl]methyl]-5-(trifluoromethyl)phenyl]phenyl]naphthalene-2-carboxamide | C39H37F3N4O5S | 730.24 |
| | A2I062 | 1-Hydroxy-N,N-dimethyl-4-[4-[3-[4-[4-(methylsulfonylcarbamoyl)phenyl]piperazine-1-carbonyl]-5-(trifluoromethyl)phenyl]phenyl]naphthalene-2-carboxamide | C39H35F3N4O6S | 744.22 |
| | A2I063 | 1-hydroxy-N,N-dimethyl-4-[4-[3-[[4-[4-(methylsulfonylcarbamoyl)phenyl]piperazin-1-yl]methyl]-5-(trifluoromethoxy)phenyl]phenyl]naphthalene-2-carboxamide | C39H37F3N4O6S | 746.24 |
| | A2I064 | 4-[4-[3-Ethoxy-5-[[4-[4-(methylsulfonylcarbamoyl)phenyl]piperazin-1-yl]methyl]phenyl]phenyl]-1-hydroxy-N,N-dimethylnaphthalene-2-carboxamide | C40H42N4O6S | 706.28 |
| | A2I065 | 1-Hydroxy-N,N-dimethyl-4-[4-[3-[[4-[4-(methylsulfonylcarbamoyl)phenyl]piperazin-1-yl]methyl]-5-propan-2-yloxyphenyl]phenyl]naphthalene-2-carboxamide | C41H44N4O6S | 720.3 |
| | A2I066 | 4-[4-[3-tert-Butyl-5-[4-[4-(methylsulfonylcarbamoyl)phenyl]piperazine-1-carbonyl]phenyl]phenyl]-1-hydroxy-N,N-dimethylnaphthalene-2-carboxamide | C42H44N4O6S | 732.3 |
| | A2I067 | 4-[4-[3-Fluoro-4-[1-[4-[4-(methylsulfonylcarbamoyl)phenyl]piperazin-1-yl]ethyl]phenyl]phenyl]-1-hydroxy-N,N-dimethylnaphthalene-2-carboxamide | C39H39FN4O5S | 694.26 |
| | A2I068 | 1-Hydroxy-N,N-dimethyl-4-[4-[2-[2-[[4-[4-(methylsulfonylcarbamoyl)phenyl]piperazin-1-yl]methyl]phenyl]ethynyl]phenyl]naphthalene-2-carboxamide | C40H38N4O5S | 686.26 |
| | A2I069 | 1-Hydroxy-N,N-dimethyl-4-[4-[2-[3-[4-[4-(methylsulfonylcarbamoyl)phenyl]piperazine-1-carbonyl]-5-(trifluoromethyl)phenyl]ethynyl]phenyl]naphthalene-2-carboxamide | C41H35F3N4O6S | 768.22 |

[Table 136]

| | | | | |
|---|---|---|---|---|
| mixED 11 | A2I007 | 6-[4-[4-[4-[3-(Dimethylcarbamoyl)-4-hydroxynaphthalen-1-yl]phenyl]-3-fluorobenzoyl]piperazin-1-yl]-N-methylsulfonylpyridazine-3-carboxamide | C36H33FN6O6S | 696.22 |
| | A2I070 | 1-Hydroxy-N,N-dimethyl-4-[4-[2-[5-[4-[4-(methylsulfonylcarbamoyl)phenyl]piperazine-1-carbonyl]pyridin-3-yl]ethynyl]phenyl]naphthalene-2-carboxamide | C39H35N5O6S | 701.23 |
| | A2I071 | 1-Hydroxy-N,N-dimethyl-4-[4-[1-[3-[4-[4-(methylsulfonylcarbamoyl)phenyl]piperazine-1-carbonyl]-5-(trifluoromethyl)phenyl]pyrazol-4-yl]phenyl]naphthalene-2-carboxamide | C42H37F3N6O6S | 810.24 |
| | A2I072 | 1-Hydroxy-N,N-dimethyl-4-[5-[2-[[4-[4-(methylsulfonylcarbamoyl)phenyl]piperazin-1-yl]methyl]phenyl]pyridin-2-yl]naphthalene-2-carboxamide | C37H37N5O5S | 663.25 |
| | A2I073 | 1-Hydroxy-N,N-dimethyl-4-[5-[2-[3-[4-[4-(methylsulfonylcarbamoyl)phenyl]piperazin-1-yl]-3-oxopropyl]phenyl]pyridin-2-yl]naphthalene-2-carboxamide | C39H39N5O6S | 705.26 |
| | A2I074 | 1-Hydroxy-N,N-dimethyl-4-[5-[4-[1-[4-[4-(methylsulfonylcarbamoyl)phenyl]piperazin-1-yl]ethyl]phenyl]pyridin-2-yl]naphthalene-2-carboxamide | C38H39N5O5S | 677.27 |
| | A2I075 | 1-Hydroxy-N,N-dimethyl-4-[5-[2-methyl-4-[[4-[4-(methylsulfonylcarbamoyl)phenyl]piperazin-1-yl]methyl]phenyl]pyridin-2- | C38H39N5O5S | 677.27 |

| | | | | |
|---|---|---|---|---|
| | | yl]naphthalene-2-carboxamide | | |
| | A2I076 | 1-Hydroxy-N,N-dimethyl-4-[5-[2-methyl-4-[4-[4-(methylsulfonylcarbamoyl)phenyl]piperazine-1-carbonyl]phenyl]pyridin-2-yl]naphthalene-2-carboxamide | C38H37N5O6S | 691.25 |
| | A2I077 | 4-[5-[3-Ethyl-4-[[4-[4-(methylsulfonylcarbamoyl)phenyl]piperazin-1-yl]methyl]phenyl]pyridin-2-yl]-1-hydroxy-N,N-dimethylnaphthalene-2-carboxamide | C39H41N5O5S | 691.28 |
| | A2I078 | 4-[5-[2-Fluoro-4-[[4-[4-(methylsulfonylcarbamoyl)phenyl]piperazin-1-yl]methyl]phenyl]pyridin-2-yl]-1-hydroxy-N,N-dimethylnaphthalene-2-carboxamide | C37H36FN5O5S | 681.24 |
| | A2I079 | 4-[5-[2-Fluoro-4-[4-[4-(methylsulfonylcarbamoyl)phenyl]piperazine-1-carbonyl]phenyl]pyridin-2-yl]-1-hydroxy-N,N-dimethylnaphthalene-2-carboxamide | C37H34FN5O6S | 695.22 |
| mixED 11 | A2I008 | 6-[4-[[4-[4-[3-(Dimethylcarbamoyl)-4-hydroxynaphthalen-1-yl]phenyl]-2-methoxyphenyl]methyl]piperazin-1-yl]-N-methylsulfonylpyridazine-3-carboxamide | C37H38N6O6S | 694.26 |
| | A2I080 | 1-Hydroxy-4-[5-[3-methoxy-4-[[4-[4-(methylsulfonylcarbamoyl)phenyl]piperazin-1-yl]methyl]phenyl]pyridin-2-yl]-N,N-dimethylnaphthalene-2-carboxamide | C38H39N5O6S | 693.26 |
| | A2I081 | 4-[5-[3,5-Dimethoxy-4-[[4-[4-(methylsulfonylcarbamoyl)phenyl]piperazin-1-yl]methyl]phenyl]pyridin-2-yl]-1-hydroxy-N,N-dimethylnaphthalene-2-carboxamide | C39H41N5O7S | 723.27 |
| | A2I082 | 1-Hydroxy-N,N-dimethyl-4-[5-[4-[[4-[4-(methylsulfonylcarbamoyl)phenyl]piperazin-1-yl]methyl]naphthalen-1-yl]pyridin-2-yl]naphthalene-2-carboxamide | C41H39N5O5S | 713.27 |
| | A2I083 | 1-Hydroxy-N,N-dimethyl-4-[5-[4-[4-[4-(methylsulfonylcarbamoyl)phenyl]piperazine-1-carbonyl]naphthalen-1-yl]pyridin-2-yl]naphthalene-2-carboxamide | C41H37N5O6S | 727.25 |
| | A2I084 | 1-Hydroxy-N,N-dimethyl-4-[5-[5-[[4-[4-(methylsulfonylcarbamoyl)phenyl]piperazin-1-yl]methyl]thiophen-3-yl]pyridin-2-yl]naphthalene-2-carboxamide | C35H35N5O5S2 | 669.21 |
| | A2I085 | 1-Hydroxy-N,N-dimethyl-4-[5-[5-[4-[4-(methylsulfonylcarbamoyl)phenyl]piperazine-1-carbonyl]thiophen-3-yl]pyridin-2-yl]naphthalene-2-carboxamide | C35H33N5O6S2 | 683.19 |
| | A2I086 | 1-Hydroxy-N,N-dimethyl-4-[5-[3-[[4-[4-(methylsulfonylcarbamoyl)phenyl]piperazin-1-yl]methyl]-5-(trifluoromethyl)phenyl]pyridin-2-yl]naphthalene-2-carboxamide | C38H36F3N5O5S | 731.24 |
| | A2I087 | 1-Hydroxy-N,N-dimethyl-4-[5-[3-[4-[4-(methylsulfonylcarbamoyl)phenyl]piperazine-1-carbonyl]-5-(trifluoromethyl)phenyl]pyridin-2-yl]naphthalene-2-carboxamide | C38H34F3N5O6S | 745.22 |
| | A2I088 | 1-Hydroxy-N,N-dimethyl-4-[5-[3-[[4-[4-(methylsulfonylcarbamoyl)phenyl]piperazin-1-yl]methyl]-5-(trifluoromethoxy)phenyl]pyridin-2-yl]naphthalene-2-carboxamide | C38H36F3N5O6S | 747.23 |
| | A2I089 | 4-[5-[3-Ethoxy-5-[[4-[4-(methylsulfonylcarbamoyl)phenyl]piperazin-1-yl]methyl]phenyl]pyridin-2-yl]-1-hydroxy-N,N-dimethylnaphthalene-2-carboxamide | C39H41N5O6S | 707.28 |
| | A2I009 | 6-[4-[[4-[4-[3-(Dimethylcarbamoyl)-4-hydroxynaphthalen-1-yl]phenyl]-2,6-dimethoxyphenyl]methyl]piperazin-1-yl]-N-methylsulfonylpyridazine-3-carboxamide | C38H40N6O7S | 724.27 |
| | A2I090 | 1-Hydroxy-N,N-dimethyl-4-[5-[3-[[4-[4-(methylsulfonylcarbamoyl)phenyl]piperazin-1-yl]methyl]-5-propan-2-yloxyphenyl]pyridin-2-yl]naphthalene-2-carboxamide | C40H43N5O6S | 721.29 |
| | A2I091 | 4-[5-[3-tert-Butyl-5-[4-[4-(methylsulfonylcarbamoyl)phenyl]piperazine-1-carbonyl]phenyl]pyridin-2-yl]-1-hydroxy-N,N-dimethylnaphthalene-2-carboxamide | C41H43N5O6S | 733.29 |
| | A2I092 | 4-[5-[3-Fluoro-4-[1-[4-[4-(methylsulfonylcarbamoyl)phenyl]piperazin-1-yl]ethyl]phenyl]pyridin-2-yl]-1-hydroxy-N,N-dimethylnaphthalene-2-carboxamide | C38H38FN5O5S | 695.26 |
| | A2I093 | 1-Hydroxy-N,N-dimethyl-4-[5-[2-[2-[[4-[4-(methylsulfonylcarbamoyl)phenyl]piperazin-1-yl]methyl]phenyl]ethynyl]pyridin-2-yl]naphthalene-2-carboxamide | C39H37N5O5S | 687.25 |
| | A2I094 | 1-Hydroxy-N,N-dimethyl-4-[5-[2-[3-[4-[4-(methylsulfonylcarbamoyl)phenyl]piperazine-1-carbonyl]-5-(trifluoromethyl)phenyl]ethynyl]pyridin-2-yl]naphthalene-2-carboxamide | C40H34F3N5O6S | 769.22 |
| | A2I095 | 1-Hydroxy-N,N-dimethyl-4-[5-[2-[5-[4-[4-(methylsulfonylcarbamoyl)phenyl]piperazine-1-carbonyl]pyridin-3-yl]ethynyl]pyridin-2-yl]naphthalene-2-carboxamide | C38H34N6O6S | 702.23 |
| | A2I096 | 1-Hydroxy-N,N-dimethyl-4-[5-[1-[3-[4-[4-(methylsulfonylcarbamoyl)phenyl]piperazine-1-carbonyl]-5-(trifluoromethyl)phenyl]pyrazol-4-yl]pyridin-2-yl]naphthalene-2-carboxamide | C41H36F3N7O6S | 811.24 |
| | A2I097 | 6-[4-[2-[2-[4-[3-(Dimethylcarbamoyl)-4-hydroxynaphthalen-1-yl]phenyl]ethynyl]benzoyl]piperazin-1-yl]-N-methylsulfonylpyridazine-3-carboxamide | C38H34N6O6S | 702.226 |
| | A2I098 | 6-[4-[2-[2-[6-[3-(Dimethylcarbamoyl)-4-hydroxynaphthalen-1-yl]pyridin-3-yl]ethynyl]benzoyl]piperazin-1-yl]-N-methylsulfonylpyridazine-3-carboxamide | C37H33N7O6S | 703.221 |
| | A2I099 | 1-Hydroxy-N,N-dimethyl-4-[4-[2-[2-[4-[4-(methylsulfonylcarbamoyl)phenyl]piperazine-1-carbonyl]phenyl]ethynyl]phenyl]naphthalene-2-carboxamide | C40H36N4O6S | 700.236 |

| | | | | |
|---|---|---|---|---|
| | A2I100 | 1-Hydroxy-N,N-dimethyl-4-[5-[2-[2-[4-[4-(methylsulfonylcarbamoyl)phenyl]piperazine-1-carbonyl]phenyl]ethynyl]pyridin-2-yl]naphthalene-2-carboxamide | C39H35N5O6S | 701.231 |

[Table 137]

[Table EE08]

| Table EE08 | | | | |
|---|---|---|---|---|
| Mixture No. | No. of compound that may be contained | Compound name | Compositional formula | Exact MS |
| mixED 12 | A2L001 | N-tert-Butylsulfonyl-6-[4-[[2-[4-[3-(dimethylcarbamoyl)-4-hydroxynaphthalen-1-yl]phenyl]phenyl]methyl]piperazin-1-yl]pyridazine-3-carboxamide | C39H42N6O5S | 706.29 |
| | A2L010 | N-tert-Butylsulfonyl-6-[4-[[4-[4-[3-(dimethylcarbamoyl)-4-hydroxynaphthalen-1-yl]phenyl]naphthalen-1-yl]methyl]piperazin-1-yl]pyridazine-3-carboxamide | C43H44N6O5S | 756.31 |
| | A2L011 | N-tert-Butylsulfonyl-6-[4-[4-[4-[3-(dimethylcarbamoyl)-4-hydroxynaphthalen-1-yl]phenyl]naphthalene-1-carbonyl]piperazin-1-yl]pyridazine-3-carboxamide | C43H42N6O6S | 770.29 |
| | A2L012 | N-tert-Butylsulfonyl-6-[4-[[4-[4-[3-(dimethylcarbamoyl)-4-hydroxynaphthalen-1-yl]phenyl]thiophen-2-yl]methyl]piperazin-1-yl]pyridazine-3-carboxamide | C37H40N6O5S2 | 712.25 |
| | A2L013 | N-tert-Butylsulfonyl-6-[4-[4-[4-[3-(dimethylcarbamoyl)-4-hydroxynaphthalen-1-yl]phenyl]thiophene-2-carbonyl]piperazin-1-yl]pyridazine-3-carboxamide | C37H38N6O6S2 | 726.23 |
| | A2L014 | N-tert-Butylsulfonyl-6-[4-[[3-[4-[3-(dimethylcarbamoyl)-4-hydroxynaphthalen-1-yl]phenyl]-5-(trifluoromethyl)phenyl]methyl]piperazin-1-yl]pyridazine-3-carboxamide | C40H41F3N6O5S | 774.28 |
| | A2L015 | N-tert-Butylsulfonyl-6-[4-[3-[4-[3-(dimethylcarbamoyl)-4-hydroxynaphthalen-1-yl]phenyl]-5-(trifluoromethyl)benzoyl]piperazin-1-yl]pyridazine-3-carboxamide | C40H39F3N6O6S | 788.26 |
| | A2L016 | N-tert-Butylsulfonyl-6-[4-[[3-[4-[3-(dimethylcarbamoyl)-4-hydroxynaphthalen-1-yl]phenyl]-5-(trifluoromethoxy)phenyl]methyl]piperazin-1-yl]pyridazine-3-carboxamide | C40H41F3N6O6S | 790.28 |
| | A2L017 | N-tert-Butylsulfonyl-6-[4-[[3-[4-[3-(dimethylcarbamoyl)-4-hydroxynaphthalen-1-yl]phenyl]-5-Ethoxyphenyl]methyl]piperazin-1-yl]pyridazine-3-carboxamide | C41H46N6O6S | 750.32 |
| | A2L018 | N-tert-Butylsulfonyl-6-[4-[[3-[4-[3-(dimethylcarbamoyl)-4-hydroxynaphthalen-1-yl]phenyl]-5-propan-2-yloxyphenyl]methyl]piperazin-1-yl]pyridazine-3-carboxamide | C42H48N6O6S | 764.34 |
| | A2L019 | 6-[4-[3-tert-Butyl-5-[4-[3-(dimethylcarbamoyl)-4-hydroxynaphthalen-1-yl]phenyl]benzoyl]piperazin-1-yl]-N-tert-Butylsulfonylpyridazine-3-carboxamide | C43H48N6O6S | 776.34 |
| | A2L002 | N-tert-Butylsulfonyl-6-[4-[3-[2-[4-[3-(dimethylcarbamoyl)-4-hydroxynaphthalen-1-yl]phenyl]phenyl]propanoyl]piperazin-1-yl]pyridazine-3-carboxamide | C41H44N6O6S | 748.3 |
| | A2L020 | N-tert-Butylsulfonyl-6-[4-[[2-[2-[4-[3-(dimethylcarbamoyl)-4-hydroxynaphthalen-1-yl]phenyl]ethynyl]phenyl]methyl]piperazin-1-yl]pyridazine-3-carboxamide | C41H42N6O5S | 730.29 |
| | A2L021 | N-tert-Butylsulfonyl-6-[4-[3-[2-[4-[3-(dimethylcarbamoyl)-4-hydroxynaphthalen-1-yl]phenyl]ethynyl]-5-(trifluoromethyl)benzoyl]piperazin-1-yl]pyridazine-3-carboxamide | C42H39F3N6O6S | 812.26 |
| | A2L022 | N-tert-Butylsulfonyl-6-[4-[5-[2-[4-[3-(dimethylcarbamoyl)-4-hydroxynaphthalen-1-yl]phenyl]ethynyl]pyridine-3-carbonyl]piperazin-1-yl]pyridazine-3-carboxamide | C40H39N7O6S | 745.27 |
| | A2L023 | N-tert-Butylsulfonyl-6-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]pyridazine-3-carboxamide | C43H41F3N8O6S | 854.28 |
| | A2L024 | N-tert-Butylsulfonyl-6-[4-[[2-[6-[3-(dimethylcarbamoyl)-4-hydroxynaphthalen-1-yl]pyridin-3-yl]phenyl]methyl]piperazin-1-yl]pyridazine-3-carboxamide | C38H41N7O5S | 707.29 |
| | A2L025 | N-tert-Butylsulfonyl-6-[4-[3-[2-[6-[3-(dimethylcarbamoyl)-4-hydroxynaphthalen-1-yl]pyridin-3-yl]phenyl]propanoyl]piperazin-1-yl]pyridazine-3-carboxamide | C40H43N7O6S | 749.3 |
| | A2L026 | N-tert-Butylsulfonyl-6-[4-[[4-[6-[3-(dimethylcarbamoyl)-4-hydroxynaphthalen-1-yl]pyridin-3-yl]-3-methylphenyl]methyl]piperazin-1-yl]pyridazine-3-carboxamide | C39H43N7O5S | 721.3 |
| | A2L027 | N-tert-Butylsulfonyl-6-[4-[4-[6-[3-(dimethylcarbamoyl)-4-hydroxynaphthalen-1-yl]pyridin-3-yl]-3-methylbenzoyl]piperazin-1-yl]pyridazine-3-carboxamide | C39H41N7O6S | 735.28 |
| | A2L028 | N-tert-Butylsulfonyl-6-[4-[[4-[6-[3-(dimethylcarbamoyl)-4-hydroxynaphthalen-1-yl]pyridin-3-yl]-2-ethylphenyl]methyl]piperazin-1-yl]pyridazine-3-carboxamide | C40H45N7O5S | 735.32 |
| | A2L029 | N-tert-Butylsulfonyl-6-[4-[[4-[6-[3-(dimethylcarbamoyl)-4-hydroxynaphthalen-1-yl]pyridin-3-yl]-3-fluorophenyl]methyl]piperazin-1-yl]pyridazine-3-carboxamide | C38H40FN7O5S | 725.28 |
| | A2L003 | N-tert-Butylsulfonyl-6-[4-[[4-[4-[3-(dimethylcarbamoyl)-4-hydroxynaphthalen-1-yl]phenyl]-3-methylphenyl]methyl]piperazin-1-yl]pyridazine-3-carboxamide | C40H44N6O5S | 720.31 |

| | | | | |
|---|---|---|---|---|
| | A2L030 | N-tert-Butylsulfonyl-6-[4-[4-[6-[3-(dimethylcarbamoyl)-4-hydroxynaphthalen-1-yl]pyridin-3-yl]-3-fluorobenzoyl]piperazin-1-yl]pyridazine-3-carboxamide | C38H38FN7O6S | 739.26 |
| | A2L031 | N-tert-Butylsulfonyl-6-[4-[[4-[6-[3-(dimethylcarbamoyl)-4-hydroxynaphthalen-1-yl]pyridin-3-yl]-2-methoxyphenyl]methyl]piperazin-1-yl]pyridazine-3-carboxamide | C39H43N7O6S | 737.3 |
| | A2L032 | N-tert-Butylsulfonyl-6-[4-[[4-[6-[3-(dimethylcarbamoyl)-4-hydroxynaphthalen-1-yl]pyridin-3-yl]-2,6-dimethoxyphenyl]methyl]piperazin-1-yl]pyridazine-3-carboxamide | C40H45N7O7S | 767.31 |
| mixED 12 | A2L033 | N-tert-Butylsulfonyl-6-[4-[[4-[6-[3-(dimethylcarbamoyl)-4-hydroxynaphthalen-1-yl]pyridin-3-yl]naphthalen-1-yl]methyl]piperazin-1-yl]pyridazine-3-carboxamide | C42H43N7O5S | 757.3 |
| | A2L034 | N-tert-Butylsulfonyl-6-[4-[4-[6-[3-(dimethylcarbamoyl)-4-hydroxynaphthalen-1-yl]pyridin-3-yl]naphthalene-1-carbonyl]piperazin-1-yl]pyridazine-3-carboxamide | C42H41N7O6S | 771.28 |
| | A2L035 | N-tert-Butylsulfonyl-6-[4-[[4-[6-[3-(dimethylcarbamoyl)-4-hydroxynaphthalen-1-yl]pyridin-3-yl]thiophen-2-yl]methyl]piperazin-1-yl]pyridazine-3-carboxamide | C36H39N7O5S2 | 713.25 |
| | A2L036 | N-tert-Butylsulfonyl-6-[4-[4-[6-[3-(dimethylcarbamoyl)-4-hydroxynaphthalen-1-yl]pyridin-3-yl]thiophene-2-carbonyl]piperazin-1-yl]pyridazine-3-carboxamide | C36H37N7O6S2 | 727.22 |
| | A2L037 | N-tert-Butylsulfonyl-6-[4-[[3-[6-[3-(dimethylcarbamoyl)-4-hydroxynaphthalen-1-yl]pyridin-3-yl]-5-(trifluoromethyl)phenyl]methyl]piperazin-1-yl]pyridazine-3-carboxamide | C39H40F3N7O5S | 775.28 |
| | A2L038 | N-tert-Butylsulfonyl-6-[4-[3-[6-[3-(dimethylcarbamoyl)-4-hydroxynaphthalen-1-yl]pyridin-3-yl]-5-(trifluoromethyl)benzoyl]piperazin-1-yl]pyridazine-3-carboxamide | C39H38F3N7O6S | 789.26 |
| | A2L039 | N-tert-Butylsulfonyl-6-[4-[[3-[6-[3-(dimethylcarbamoyl)-4-hydroxynaphthalen-1-yl]pyridin-3-yl]-5-(trifluoromethoxy)phenyl]methyl]piperazin-1-yl]pyridazine-3-carboxamide | C39H40F3N7O6S | 791.27 |

[Table 138]

| | | | | |
|---|---|---|---|---|
| mixED 12 | A2L004 | N-tert-Butylsulfonyl-6-[4-[4-[4-[3-(dimethylcarbamoyl)-4-hydroxynaphthalen-1-yl]phenyl]-3-methylbenzoyl]piperazin-1-yl]pyridazine-3-carboxamide | C40H42N6O6S | 734.29 |
| | A2L040 | N-tert-Butylsulfonyl-6-[4-[[3-[6-[3-(dimethylcarbamoyl)-4-hydroxynaphthalen-1-yl]pyridin-3-yl]-5-ethoxyphenyl]methyl]piperazin-1-yl]pyridazine-3-carboxamide | C40H45N7O6S | 751.32 |
| | A2L041 | N-tert-Butylsulfonyl-6-[4-[[3-[6-[3-(dimethylcarbamoyl)-4-hydroxynaphthalen-1-yl]pyridin-3-yl]-5-propan-2-yloxyphenyl]methyl]piperazin-1-yl]pyridazine-3-carboxamide | C41H47N7O6S | 765.33 |
| | A2L042 | 6-[4-[3-tert-Butyl-5-[6-[3-(dimethylcarbamoyl)-4-hydroxynaphthalen-1-yl]pyridin-3-yl]benzoyl]piperazin-1-yl]-N-tert-butylsulfonylpyridazine-3-carboxamide | C42H47N7O6S | 777.33 |
| | A2L043 | N-tert-Butylsulfonyl-6-[4-[[2-[2-[6-[3-(dimethylcarbamoyl)-4-hydroxynaphthalen-1-yl]pyridin-3-yl]ethynyl]phenyl]methyl]piperazin-1-yl]pyridazine-3-carboxamide | C40H41N7O5S | 731.29 |
| | A2L044 | N-tert-Butylsulfonyl-6-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]pyridazine-3-carboxamide | C41H38F3N7O6S | 813.26 |
| | A2L045 | N-tert-Butylsulfonyl-6-[4-[5-[2-[6-[3-(dimethylcarbamoyl)-4-hydroxynaphthalen-1-yl]pyridin-3-yl]ethynyl]pyridine-3-carbonyl]piperazin-1-yl]pyridazine-3-carboxamide | C39H38N8O6S | 746.26 |
| | A2L046 | N-tert-Butylsulfonyl-6-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]pyridazine-3-carboxamide | C42H40F3N9O6S | 855.28 |
| | A2L047 | 4-[4-[2-[[4-[4-(tert-Butylsulfonylcarbamoyl)phenyl]piperazin-1-yl]methyl]phenyl]phenyl]-1-hydroxy-N,N-dimethylnaphthalene-2-carboxamide | C41H44N4O5S | 704.3 |
| | A2L048 | 4-[4-[2-[3-[4-[4-(tert-Butylsulfonylcarbamoyl)phenyl]piperazin-1-yl]-3-oxopropyl]phenyl]phenyl]-1-hydroxy-N,N-dimethylnaphthalene-2-carboxamide | C43H46N4O6S | 746.31 |
| | A2L049 | 4-[4-[4-[1-[4-[4-(tert-Butylsulfonylcarbamoyl)phenyl]piperazin-1-yl]ethyl]phenyl]phenyl]-1-hydroxy-N,N-dimethylnaphthalene-2-carboxamide | C42H46N4O5S | 718.32 |
| | A2L005 | N-tert-Butylsulfonyl-6-[4-[[4-[4-[3-(dimethylcarbamoyl)-4-hydroxynaphthalen-1-yl]phenyl]-2-ethylphenyl]methyl]piperazin-1-yl]pyridazine-3-carboxamide | C41H46N6O5S | 734.33 |
| | A2L050 | 4-[4-[4-[[4-[4-(tert-Butylsulfonylcarbamoyl)phenyl]piperazin-1-yl]methyl]-2-methylphenyl]phenyl]-1-hydroxy-N,N-dimethylnaphthalene-2-carboxamide | C42H46N4O5S | 718.32 |
| | A2L051 | 4-[4-[4-[4-[4-(tert-Butylsulfonylcarbamoyl)phenyl]piperazine-1-carbonyl]-2-methylphenyl]phenyl]-1-hydroxy-N,N-dimethylnaphthalene-2-carboxamide | C42H44N4O6S | 732.3 |
| | A2L052 | 4-[4-[4-[[4-[4-(tert-Butylsulfonylcarbamoyl)phenyl]piperazin-1-yl]methyl]-3-ethylphenyl]phenyl]-1-hydroxy-N,N-dimethylnaphthalene-2-carboxamide | C43H48N4O5S | 732.33 |
| | A2L053 | 4-[4-[4-[[4-[4-(tert-Butylsulfonylcarbamoyl)phenyl]piperazin-1-yl]methyl]-2-fluorophenyl]phenyl]-1-hydroxy-N,N-dimethylnaphthalene-2-carboxamide | C41H43FN4O5S | 722.29 |
| | A2L054 | 4-[4-[4-[4-[4-(tert-Butylsulfonylcarbamoyl)phenyl]piperazine-1-carbonyl]-2-fluorophenyl]phenyl]-1-hydroxy-N,N-dimethylnaphthalene-2-carboxamide | C41H41FN4O6S | 736.27 |

| | | | | |
|---|---|---|---|---|
| | A2L055 | 4-[4-[4-[[4-[4-(tert-Butylsulfonylcarbamoyl)phenyl]piperazin-1-yl]methyl]-3-methoxyphenyl]phenyl]-1-hydroxy-N,N-dimethylnaphthalene-2-carboxamide | C42H46N4O6S | 734.31 |
| mixED 12 | A2L056 | 4-[4-[4-[[4-[4-(tert-Butylsulfonylcarbamoyl)phenyl]piperazin-1-yl]methyl]-3,5-dimethoxyphenyl]phenyl]-1-hydroxy-N,N-dimethylnaphthalene-2-carboxamide | C43H48N4O7S | 764.32 |
| | A2L057 | 4-[4-[4-[[4-[4-(tert-Butylsulfonylcarbamoyl)phenyl]piperazin-1-yl]methyl]naphthalen-1-yl]phenyl]-1-hydroxy-N,N-dimethylnaphthalene-2-carboxamide | C45H46N4O5S | 754.32 |
| | A2L058 | 4-[4-[4-[4-[4-(tert-Butylsulfonylcarbamoyl)phenyl]piperazine-1-carbonyl]naphthalen-1-yl]phenyl]-1-hydroxy-N,N-dimethylnaphthalene-2-carboxamide | C45H44N4O6S | 768.3 |
| | A2L059 | 4-[4-[5-[[4-[4-(tert-Butylsulfonylcarbamoyl)phenyl]piperazin-1-yl]methyl]thiophen-3-yl]phenyl]-1-hydroxy-N,N-dimethylnaphthalene-2-carboxamide | C39H42N4O5S2 | 710.26 |
| | A2L006 | N-tert-Butylsulfonyl-6-[4-[[4-[4-[3-(dimethylcarbamoyl)-4-hydroxynaphthalen-1-yl]phenyl]-3-fluorophenyl]methyl]piperazin-1-yl]pyridazine-3-carboxamide | C39H41FN6O5S | 724.28 |
| | A2L060 | 4-[4-[5-[4-[4-(tert-Butylsulfonylcarbamoyl)phenyl]piperazine-1-carbonyl]thiophen-3-yl]phenyl]-1-hydroxy-N,N-dimethylnaphthalene-2-carboxamide | C39H40N4O6S2 | 724.24 |
| | A2L061 | 4-[4-[3-[[4-[4-(tert-Butylsulfonylcarbamoyl)phenyl]piperazin-1-yl]methyl]-5-(trifluoromethyl)phenyl]phenyl]-1-hydroxy-N,N-dimethylnaphthalene-2-carboxamide | C42H43F3N4O5S | 772.29 |
| | A2L062 | 4-[4-[3-[4-[4-(tert-Butylsulfonylcarbamoyl)phenyl]piperazine-1-carbonyl]-5-(trifluoromethyl)phenyl]phenyl]-1-hydroxy-N,N-dimethylnaphthalene-2-carboxamide | C42H41F3N4O6S | 786.27 |
| | A2L063 | 4-[4-[3-[[4-[4-(tert-Butylsulfonylcarbamoyl)phenyl]piperazin-1-yl]methyl]-5-(trifluoromethoxy)phenyl]phenyl]-1-hydroxy-N,N-dimethylnaphthalene-2-carboxamide | C42H43F3N4O6S | 788.29 |
| | A2L064 | 4-[4-[3-[[4-[4-(tert-Butylsulfonylcarbamoyl)phenyl]piperazin-1-yl]methyl]-5-ethoxyphenyl]phenyl]-1-hydroxy-N,N-dimethylnaphthalene-2-carboxamide | C43H48N4O6S | 748.33 |
| | A2L065 | 4-[4-[3-[[4-[4-(tert-Butylsulfonylcarbamoyl)phenyl]piperazin-1-yl]methyl]-5-propan-2-yloxyphenyl]phenyl]-1-hydroxy-N,N-dimethylnaphthalene-2-carboxamide | C44H50N4O6S | 762.35 |
| | A2L066 | 4-[4-[3-tert-Butyl-5-[4-[4-(tert-butylsulfonylcarbamoyl)phenyl]piperazine-1-carbonyl]phenyl]phenyl]-1-hydroxy-N,N-dimethylnaphthalene-2-carboxamide | C45H50N4O6S | 774.35 |
| | A2L067 | 4-[4-[4-[1-[4-[4-(tert-Butylsulfonylcarbamoyl)phenyl]piperazin-1-yl]ethyl]-3-fluorophenyl]phenyl]-1-hydroxy-N,N-dimethylnaphthalene-2-carboxamide | C42H45FN4O5S | 736.31 |
| | A2L068 | 4-[4-[2-[2-[[4-[4-(tert-Butylsulfonylcarbamoyl)phenyl]piperazin-1-yl]methyl]phenyl]ethynyl]phenyl]-1-hydroxy-N,N-dimethylnaphthalene-2-carboxamide | C43H44N4O5S | 728.3 |
| | A2L069 | 4-[4-[2-[3-[4-[4-(tert-Butylsulfonylcarbamoyl)phenyl]piperazine-1-carbonyl]-5-(trifluoromethyl)phenyl]ethynyl]phenyl]-1-hydroxy-N,N-dimethylnaphthalene-2-carboxamide | C44H41F3N4O6S | 810.27 |

[Table 139]

| | | | | |
|---|---|---|---|---|
| mixED 12 | A2L007 | N-tert-Butylsulfonyl-6-[4-[4-[4-[3-(dimethylcarbamoyl)-4-hydroxynaphthalen-1-yl]phenyl]-3-fluorobenzoyl]piperazin-1-yl]pyridazine-3-carboxamide | C39H39FN6O6S | 738.26 |
| | A2L070 | 4-[4-[2-[5-[4-[4-(tert-Butylsulfonylcarbamoyl)phenyl]piperazine-1-carbonyl]pyridin-3-yl]ethynyl]phenyl]-1-hydroxy-N,N-dimethylnaphthalene-2-carboxamide | C42H41N5O6S | 743.28 |
| | A2L071 | 4-[4-[1-[3-[4-[4-(tert-Butylsulfonylcarbamoyl)phenyl]piperazine-1-carbonyl]-5-(trifluoromethyl)phenyl]pyrazol-4-yl]phenyl]-1-hydroxy-N,N-dimethylnaphthalene-2-carboxamide | C45H43F3N6O6S | 852.29 |
| | A2L072 | 4-[5-[2-[[4-[4-(tert-Butylsulfonylcarbamoyl)phenyl]piperazin-1-yl]methyl]phenyl]pyridin-2-yl]-1-hydroxy-N,N-dimethylnaphthalene-2-carboxamide | C40H43N5O5S | 705.3 |
| | A2L073 | 4-[5-[2-[3-[4-[4-(tert-Butylsulfonylcarbamoyl)phenyl]piperazin-1-yl]-3-oxopropyl]phenyl]pyridin-2-yl]-1-hydroxy-N,N-dimethylnaphthalene-2-carboxamide | C42H45N5O6S | 747.31 |
| | A2L074 | 4-[5-[4-[1-[4-[4-(tert-Butylsulfonylcarbamoyl)phenyl]piperazin-1-yl]ethyl]phenyl]pyridin-2-yl]-1-hydroxy-N,N-dimethylnaphthalene-2-carboxamide | C41H45N5O5S | 719.31 |
| | A2L075 | 4-[5-[4-[[4-[4-(tert-Butylsulfonylcarbamoyl)phenyl]piperazin-1-yl]methyl]-2-methylphenyl]pyridin-2-yl]-1-hydroxy-N,N-dimethylnaphthalene-2-carboxamide | C41H45N5O5S | 719.31 |
| | A2L076 | 4-[5-[4-[4-[4-(tert-Butylsulfonylcarbamoyl)phenyl]piperazine-1-carbonyl]-2-methylphenyl]pyridin-2-yl]-1-hydroxy-N,N-dimethylnaphthalene-2-carboxamide | C41H43N5O6S | 733.29 |
| | A2L077 | 4-[5-[4-[[4-[4-(tert-Butylsulfonylcarbamoyl)phenyl]piperazin-1-yl]methyl]-3-ethylphenyl]pyridin-2-yl]-1-hydroxy-N,N-dimethylnaphthalene-2-carboxamide | C42H47N5O5S | 733.33 |
| | A2L078 | 4-[5-[4-[[4-[4-(tert-Butylsulfonylcarbamoyl)phenyl]piperazin-1-yl]methyl]-2-fluorophenyl]pyridin-2-yl]-1-hydroxy-N,N-dimethylnaphthalene-2-carboxamide | C40H42FN5O5S | 723.29 |
| | A2L079 | 4-[5-[4-[4-[4-(tert-Butylsulfonylcarbamoyl)phenyl]piperazine-1-carbonyl]-2-fluorophenyl]pyridin-2-yl]-1-hydroxy-N,N-dimethylnaphthalene-2-carboxamide | C40H40FN5O6S | 737.27 |

| | | | | |
|---|---|---|---|---|
| mixED 12 | A2L008 | N-tert-Butylsulfonyl-6-[4-[[4-[4-[3-(dimethylcarbamoyl)-4-hydroxynaphthalen-1-yl]phenyl]-2-methoxyphenyl]methyl]piperazin-1-yl]pyridazine-3-carboxamide | C40H44N6O6S | 736.3 |
| | A2L080 | 4-[5-[4-[[4-[4-(tert-Butylsulfonylcarbamoyl)phenyl]piperazin-1-yl]methyl]-3-methoxyphenyl]pyridin-2-yl]-1-hydroxy-N,N-dimethylnaphthalene-2-carboxamide | C41H45N5O6S | 735.31 |
| | A2L081 | 4-[5-[4-[[4-[4-(tert-Butylsulfonylcarbamoyl)phenyl]piperazin-1-yl]methyl]-3,5-dimethoxyphenyl]pyridin-2-yl]-1-hydroxy-N,N-dimethylnaphthalene-2-carboxamide | C42H47N5O7S | 765.32 |
| | A2L082 | 4-[5-[4-[[4-[4-(tert-Butylsulfonylcarbamoyl)phenyl]piperazin-1-yl]methyl]naphthalen-1-yl]pyridin-2-yl]-1-hydroxy-N,N-dimethylnaphthalene-2-carboxamide | C44H45N5O5S | 755.31 |
| | A2L083 | 4-[5-[4-[4-[4-(tert-Butylsulfonylcarbamoyl)phenyl]piperazine-1-carbonyl]naphthalen-1-yl]pyridin-2-yl]-1-hydroxy-N,N-dimethylnaphthalene-2-carboxamide | C44H43N5O6S | 769.29 |
| | A2L084 | 4-[5-[5-[[4-[4-(tert-Butylsulfonylcarbamoyl)phenyl]piperazin-1-yl]methyl]thiophen-3-yl]pyridin-2-yl]-1-hydroxy-N,N-dimethylnaphthalene-2-carboxamide | C38H41N5O5S2 | 711.25 |
| | A2L085 | 4-[5-[5-[4-[4-(tert-Butylsulfonylcarbamoyl)phenyl]piperazine-1-carbonyl]thiophen-3-yl]pyridin-2-yl]-1-hydroxy-N,N-dimethylnaphthalene-2-carboxamide | C38H39N5O6S2 | 725.23 |
| | A2L086 | 4-[5-[3-[[4-[4-(tert-Butylsulfonylcarbamoyl)phenyl]piperazin-1-yl]methyl]-5-(trifluoromethyl)phenyl]pyridin-2-yl]-1-hydroxy-N,N-dimethylnaphthalene-2-carboxamide | C41H42F3N5O5S | 773.29 |
| | A2L087 | 4-[5-[3-[4-[4-(tert-Butylsulfonylcarbamoyl)phenyl]piperazine-1-carbonyl]-5-(trifluoromethyl)phenyl]pyridin-2-yl]-1-hydroxy-N,N-dimethylnaphthalene-2-carboxamide | C41H40F3N5O6S | 787.27 |
| | A2L088 | 4-[5-[3-[[4-[4-(tert-Butylsulfonylcarbamoyl)phenyl]piperazin-1-yl]methyl]-5-(trifluoromethoxy)phenyl]pyridin-2-yl]-1-hydroxy-N,N-dimethylnaphthalene-2-carboxamide | C41H42F3N5O6S | 789.28 |
| | A2L089 | 4-[5-[3-[[4-[4-(tert-Butylsulfonylcarbamoyl)phenyl]piperazin-1-yl]methyl]-5-ethoxyphenyl]pyridin-2-yl]-1-hydroxy-N,N-dimethylnaphthalene-2-carboxamide | C42H47N5O6S | 749.32 |
| | A2L009 | N-tert-Butylsulfonyl-6-[4-[[4-[4-[3-(dimethylcarbamoyl)-4-hydroxynaphthalen-1-yl]phenyl]-2,6-dimethoxyphenyl]methyl]piperazin-1-yl]pyridazine-3-carboxamide | C41H46N6O7S | 766.31 |
| | A2L090 | 4-[5-[3-[[4-[4-(tert-Butylsulfonylcarbamoyl)phenyl]piperazin-1-yl]methyl]-5-propan-2-yloxyphenyl]pyridin-2-yl]-1-hydroxy-N,N-dimethylnaphthalene-2-carboxamide | C43H49N5O6S | 763.34 |
| | A2L091 | 4-[5-[3-tert-Butyl-5-[4-[4-(tert-butylsulfonylcarbamoyl)phenyl]piperazine-1-carbonyl]phenyl]pyridin-2-yl]-1-hydroxy-N,N-dimethylnaphthalene-2-carboxamide | C44H49N5O6S | 775.34 |
| | A2L092 | 4-[5-[4-[1-[4-[4-(tert-Butylsulfonylcarbamoyl)phenyl]piperazin-1-yl]ethyl]-3-fluorophenyl]pyridin-2-yl]-1-hydroxy-N,N-dimethylnaphthalene-2-carboxamide | C41H44FN5O5S | 737.3 |
| | A2L093 | 4-[5-[2-[2-[[4-[4-(tert-Butylsulfonylcarbamoyl)phenyl]piperazin-1-yl]methyl]phenyl]ethynyl]pyridin-2-yl]-1-hydroxy-N,N-dimethylnaphthalene-2-carboxamide | C42H43N5O5S | 729.3 |
| | A2L094 | 4-[5-[2-[3-[4-[4-(tert-Butylsulfonylcarbamoyl)phenyl]piperazine-1-carbonyl]-5-(trifluoromethyl)phenyl]ethynyl]pyridin-2-yl]-1-hydroxy-N,N-dimethylnaphthalene-2-carboxamide | C43H40F3N5O6S | 811.27 |
| | A2L095 | 4-[5-[2-[5-[4-[4-(tert-Butylsulfonylcarbamoyl)phenyl]piperazine-1-carbonyl]pyridin-3-yl]ethynyl]pyridin-2-yl]-1-hydroxy-N,N-dimethylnaphthalene-2-carboxamide | C41H40N6O6S | 744.27 |
| | A2L096 | 4-[5-[1-[3-[4-[4-(tert-Butylsulfonylcarbamoyl)phenyl]piperazine-1-carbonyl]-5-(trifluoromethyl)phenyl]pyrazol-4-yl]pyridin-2-yl]-1-hydroxy-N,N-dimethylnaphthalene-2-carboxamide | C44H42F3N7O6S | 853.29 |
| | A2L097 | N-tert-Butylsulfonyl-6-[4-[2-[2-[4-[3-(dimethylcarbamoyl)-4-hydroxynaphthalen-1-yl]phenyl]ethynyl]benzoyl]piperazin-1-yl]pyridazine-3-carboxamide | C41H40N6O6S | 744.273 |
| | A2L098 | N-tert-Butylsulfonyl-6-[4-[2-[2-[6-[3-(dimethylcarbamoyl)-4-hydroxynaphthalen-1-yl]pyridin-3-yl]ethynyl]benzoyl]piperazin-1-yl]pyridazine-3-carboxamide | C40H39N7O6S | 745.268 |
| | A2L099 | 4-[4-[2-[2-[4-[4-(tert-Butylsulfonylcarbamoyl)phenyl]piperazine-1-carbonyl]phenyl]ethynyl]phenyl]-1-hydroxy-N,N-dimethylnaphthalene-2-carboxamide | C43H42N4O6S | 742.283 |
| | A2L100 | 4-[5-[2-[2-[4-[4-(tert-Butylsulfonylcarbamoyl)phenyl]piperazine-1-carbonyl]phenyl]ethynyl]pyridin-2-yl]-1-hydroxy-N,N-dimethylnaphthalene-2-carboxamide | C42H41N5O6S | 743.278 |

[Table 140]

[Table EE09]

| Table EE09 |
|---|

| Mixture No. | No. of compound that may be contained | Compound name | Compositional formula | Exact MS |
|---|---|---|---|---|
| mixED 13 | A2N001 | 6-[4-[[2-[4-[3-(Dimethylcarbamoyl)-4-hydroxynaphthalen-1-yl]phenyl]phenyl]methyl]piperazin-1-yl]-N-(3,3,3-trifluoropropylsulfonyl)pyridazine-3-carboxamide | C38H37F3N6O5S | 746.25 |
| | A2N010 | 6-[4-[[4-[4-[3-(Dimethylcarbamoyl)-4-hydroxynaphthalen-1-yl]phenyl]naphthalen-1-yl]methyl]piperazin-1-yl]-N-(3,3,3-trifluoropropylsulfonyl)pyridazine-3-carboxamide | C42H39F3N6O5S | 796.27 |
| | A2N011 | 6-[4-[4-[4-[3-(Dimethylcarbamoyl)-4-hydroxynaphthalen-1-yl]phenyl]naphthalene-1-carbonyl]piperazin-1-yl]-N-(3,3,3-trifluoropropylsulfonyl)pyridazine-3-carboxamide | C42H37F3N6O6S | 810.24 |
| | A2N012 | 6-[4-[[4-[4-[3-(Dimethylcarbamoyl)-4-hydroxynaphthalen-1-yl]phenyl]thiophen-2-yl]methyl]piperazin-1-yl]-N-(3,3,3-trifluoropropylsulfonyl)pyridazine-3-carboxamide | C36H35F3N6O5S2 | 752.21 |
| | A2N013 | 6-[4-[4-[4-[3-(Dimethylcarbamoyl)-4-hydroxynaphthalen-1-yl]phenyl]thiophene-2-carbonyl]piperazin-1-yl]-N-(3,3,3-trifluoropropylsulfonyl)pyridazine-3-carboxamide | C36H33F3N6O6S2 | 766.19 |
| | A2N014 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-(3,3,3-trifluoropropylsulfonyl)pyridazine-3-carboxamide | C39H36F6N6O5S | 814.24 |
| | A2N015 | 6-[4-[3-[4-[3-(Dimethylcarbamoyl)-4-hydroxynaphthalen-1-yl]phenyl]-5-(trifluoromethyl)benzoyl]piperazin-1-yl]-N-(3,3,3-trifluoropropylsulfonyl)pyridazine-3-carboxamide | C39H34F6N6O6S | 828.22 |
| | A2N016 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-(3,3,3-trifluoropropylsulfonyl)pyridazine-3-carboxamide | C39H36F6N6O6S | 830.23 |
| | A2N017 | 6-[4-[[3-[4-[3-(Dimethylcarbamoyl)-4-hydroxynaphthalen-1-yl]phenyl]-5-ethoxyphenyl]methyl]piperazin-1-yl]-N-(3,3,3-trifluoropropylsulfonyl)pyridazine-3-carboxamide | C40H41F3N6O6S | 790.28 |
| | A2N018 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-(3,3,3-trifluoropropylsulfonyl)pyridazine-3-carboxamide | C41H43F3N6O6S | 804.29 |
| | A2N019 | 6-[4-[3-tert-Butyl-5-[4-[3-(dimethylcarbamoyl)-4-hydroxynaphthalen-1-yl]phenyl]benzoyl]piperazin-1-yl]-N-(3,3,3-trifluoropropylsulfonyl)pyridazine-3-carboxamide | C42H43F3N6O6S | 816.29 |
| | A2N002 | 6-[4-[3-[2-[4-[3-(Dimethylcarbamoyl)-4-hydroxynaphthalen-1-yl]phenyl]phenyl]propanoyl]piperazin-1-yl]-N-(3,3,3-trifluoropropylsulfonyl)pyridazine-3-carboxamide | C40H39F3N6O6S | 788.26 |
| | A2N020 | 6-[4-[[2-[2-[4-[3-(Dimethylcarbamoyl)-4-hydroxynaphthalen-1-yl]phenyl]ethynyl]phenyl]methyl]piperazin-1-yl]-N-(3,3,3-trifluoropropylsulfonyl)pyridazine-3-carboxamide | C40H37F3N6O5S | 770.25 |
| | A2N021 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-(3,3,3-trifluoropropylsulfonyl)pyridazine-3-carboxamide | C41H34F6N6O6S | 852.22 |
| | A2N022 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-(3,3,3-trifluoropropylsulfonyl)pyridazine-3-carboxamide | C39H34F3N7O6S | 785.22 |
| | A2N023 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-(3,3,3-trifluoropropylsulfonyl)pyridazine-3-carboxamide | C42H36F6N8O6S | 894.24 |
| | A2N024 | 6-[4-[[2-[6-[3-(Dimethylcarbamoyl)-4-hydroxynaphthalen-1-yl]pyridin-3-yl]phenyl]methyl]piperazin-1-yl]-N-(3,3,3-trifluoropropylsulfonyl)pyridazine-3-carboxamide | C37H36F3N7O5S | 747.25 |
| | A2N025 | 6-[4-[3-[2-[6-[3-(Dimethylcarbamoyl)-4-hydroxynaphthalen-1-yl]pyridin-3-yl]phenyl]propanoyl]piperazin-1-yl]-N-(3,3,3-trifluoropropylsulfonyl)pyridazine-3-carboxamide | C39H38F3N7O6S | 789.26 |
| | A2N026 | 6-[4-[[4-[6-[3-(Dimethylcarbamoyl)-4-hydroxynaphthalen-1-yl]pyridin-3-yl]-3-methylphenyl]methyl]piperazin-1-yl]-N-(3,3,3-trifluoropropylsulfonyl)pyridazine-3-carboxamide | C38H38F3N7O5S | 761.26 |
| | A2N027 | 6-[4-[4-[6-[3-(Dimethylcarbamoyl)-4-hydroxynaphthalen-1-yl]pyridin-3-yl]-3-methylbenzoyl]piperazin-1-yl]-N-(3,3,3-trifluoropropylsulfonyl)pyridazine-3-carboxamide | C38H36F3N7O6S | 775.24 |

| | | | | |
|---|---|---|---|---|
| | A2N028 | 6-[4-[[4-[6-[3-(Dimethylcarbamoyl)-4-hydroxynaphthalen-1-yl]pyridin-3-yl]-2-ethylphenyl]methyl]piperazin-1-yl]-N-(3,3,3-trifluoropropylsulfonyl)pyridazine-3-carboxamide | C39H40F3N7O5S | 775.28 |
| | A2N029 | 6-[4-[[4-[6-[3-(Dimethylcarbamoyl)-4-hydroxynaphthalen-1-yl]pyridin-3-yl]-3-fluorophenyl]methyl]piperazin-1-yl]-N-(3,3,3-trifluoropropylsulfonyl)pyridazine-3-carboxamide | C37H35F4N7O5S | 765.24 |
| mixED 13 | A2N003 | 6-[4-[[4-[4-[3-(Dimethylcarbamoyl)-4-hydroxynaphthalen-1-yl]phenyl]-3-methylphenyl]methyl]piperazin-1-yl]-N-(3,3,3-trifluoropropylsulfonyl)pyridazine-3-carboxamide | C39H39F3N6O5S | 760.27 |
| | A2N030 | 6-[4-[4-[6-[3-(Dimethylcarbamoyl)-4-hydroxynaphthalen-1-yl]pyridin-3-yl]-3-fluorobenzoyl]piperazin-1-yl]-N-(3,3,3-trifluoropropylsulfonyl)pyridazine-3-carboxamide | C37H33F4N7O6S | 779.21 |
| | A2N031 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-(3,3,3-trifluoropropylsulfonyl)pyridazine-3-carboxamide | C38H38F3N7O6S | 777.26 |
| | A2N032 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-(3,3,3-trifluoropropylsulfonyl)pyridazine-3-carboxamide | C39H40F3N7O7S | 807.27 |
| | A2N033 | 6-[4-[[4-[6-[3-(Dimethylcarbamoyl)-4-hydroxynaphthalen-1-yl]pyridin-3-yl]naphthalen-1-yl]methyl]piperazin-1-yl]-N-(3,3,3-trifluoropropylsulfonyl)pyridazine-3-carboxamide | C41H38F3N7O5S | 797.26 |
| | A2N034 | 6-[4-[4-[6-[3-(Dimethylcarbamoyl)-4-hydroxynaphthalen-1-yl]pyridin-3-yl]naphthalene-1-carbonyl]piperazin-1-yl]-N-(3,3,3-trifluoropropylsulfonyl)pyridazine-3-carboxamide | C41H36F3N7O6S | 811.24 |
| | A2N035 | 6-[4-[[4-[6-[3-(Dimethylcarbamoyl)-4-hydroxynaphthalen-1-yl]pyridin-3-yl]thiophen-2-yl]methyl]piperazin-1-yl]-N-(3,3,3-trifluoropropylsulfonyl)pyridazine-3-carboxamide | C35H34F3N7O5S2 | 753.2 |
| | A2N036 | 6-[4-[4-[6-[3-(Dimethylcarbamoyl)-4-hydroxynaphthalen-1-yl]pyridin-3-yl]thiophene-2-carbonyl]piperazin-1-yl]-N-(3,3,3-trifluoropropylsulfonyl)pyridazine-3-carboxamide | C35H32F3N7O6S2 | 767.18 |
| | A2N037 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-(3,3,3-trifluoropropylsulfonyl)pyridazine-3-carboxamide | C38H35F6N7O5S | 815.23 |
| | A2N038 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-(3,3,3-trifluoropropylsulfonyl)pyridazine-3-carboxamide | C38H33F6N7O6S | 829.21 |
| | A2N039 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-(3,3,3-trifluoropropylsulfonyl)pyridazine-3-carboxamide | C38H35F6N7O6S | 831.23 |

[Table 141]

| | | | | |
|---|---|---|---|---|
| mixED 13 | A2N004 | 6-[4-[4-[4-[3-(Dimethylcarbamoyl)-4-hydroxynaphthalen-1-yl]phenyl]-3-methylbenzoyl]piperazin-1-yl]-N-(3,3,3-trifluoropropylsulfonyl)pyridazine-3-carboxamide | C39H37F3N6O6S | 774.24 |
| | A2N040 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-(3,3,3-trifluoropropylsulfonyl)pyridazine-3-carboxamide | C39H40F3N7O6S | 791.27 |
| | A2N041 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-(3,3,3-trifluoropropylsulfonyl)pyridazine-3-carboxamide | C40H42F3N7O6S | 805.29 |
| | A2N042 | 6-[4-[3-tert-Butyl-5-[6-[3-(dimethylcarbamoyl)-4-hydroxynaphthalen-1-yl]pyridin-3-yl]benzoyl]piperazin-1-yl]-N-(3,3,3-trifluoropropylsulfonyl)pyridazine-3-carboxamide | C41H42F3N7O6S | 817.29 |
| | A2N043 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-(3,3,3-trifluoropropylsulfonyl)pyridazine-3-carboxamide | C39H36F3N7O5S | 771.25 |
| | A2N044 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-(3,3,3-trifluoropropylsulfonyl)pyridazine-3-carboxamide | C40H33F6N7O6S | 853.21 |
| | A2N045 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-(3,3,3-trifluoropropylsulfonyl)pyridazine-3-carboxamide | C38H33F3N8O6S | 786.22 |

| | | | | |
|---|---|---|---|---|
| | A2N046 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-(3,3,3-trifluoropropylsulfonyl)pyridazine-3-carboxamide | C41H35F6N9O6S | 895.23 |
| | A2N047 | 1-Hydroxy-N,N-dimethyl-4-[4-[2-[[4-[4-(3,3,3-trifluoropropylsulfonylcarbamoyl)phenyl]piperazin-1-yl]methyl]phenyl]phenyl]naphthalene-2-carboxamide | C40H39F3N4O5S | 744.26 |
| | A2N048 | 1-Hydroxy-N,N-dimethyl-4-[4-[2-[3-oxo-3-[4-[4-(3,3,3-trifluoropropylsulfonylcarbamoyl)phenyl]piperazin-1-yl]propyl]phenyl]phenyl]naphthalene-2-carboxamide | C42H41F3N4O6S | 786.27 |
| mixED 13 | A2N049 | 1-Hydroxy-N,N-dimethyl-4-[4-[4-[1-[4-[4-(3,3,3-trifluoropropylsulfonylcarbamoyl)phenyl]piperazin-1-yl]ethyl]phenyl]phenyl]naphthalene-2-carboxamide | C41H41F3N4O5S | 758.27 |
| | A2N005 | 6-[4-[[4-[4-[3-(Dimethylcarbamoyl)-4-hydroxynaphthalen-1-yl]phenyl]-2-ethylphenyl]methyl]piperazin-1-yl]-N-(3,3,3-trifluoropropylsulfonyl)pyridazine-3-carboxamide | C40H41F3N6O5S | 774.28 |
| | A2N050 | 1-Hydroxy-N,N-dimethyl-4-[4-[2-methyl-4-[[4-[4-(3,3,3-trifluoropropylsulfonylcarbamoyl)phenyl]piperazin-1-yl]methyl]phenyl]phenyl]naphthalene-2-carboxamide | C41H41F3N4O5S | 758.27 |
| | A2N051 | 1-Hydroxy-N,N-dimethyl-4-[4-[2-methyl-4-[4-[4-(3,3,3-trifluoropropylsulfonylcarbamoyl)phenyl]piperazine-1-carbonyl]phenyl]phenyl]naphthalene-2-carboxamide | C41H39F3N4O6S | 772.25 |
| | A2N052 | 4-[4-[3-Ethyl-4-[[4-[4-(3,3,3-trifluoropropylsulfonylcarbamoyl)phenyl]piperazin-1-yl]methyl]phenyl]phenyl]-1-hydroxy-N,N-dimethylnaphthalene-2-carboxamide | C42H43F3N4O5S | 772.29 |
| | A2N053 | 4-[4-[2-Fluoro-4-[[4-[4-(3,3,3-trifluoropropylsulfonylcarbamoyl)phenyl]piperazin-1-yl]methyl]phenyl]phenyl]-1-hydroxy-N,N-dimethylnaphthalene-2-carboxamide | C40H38F4N4O5S | 762.25 |
| | A2N054 | 4-[4-[2-Fluoro-4-[4-[4-(3,3,3-trifluoropropylsulfonylcarbamoyl)phenyl]piperazine-1-carbonyl]phenyl]phenyl]-1-hydroxy-N,N-dimethylnaphthalene-2-carboxamide | C40H36F4N4O6S | 776.23 |
| | A2N055 | 1-Hydroxy-4-[4-[3-methoxy-4-[[4-[4-(3,3,3-trifluoropropylsulfonylcarbamoyl)phenyl]piperazin-1-yl]methyl]phenyl]phenyl]-N,N-dimethylnaphthalene-2-carboxamide | C41H41F3N4O6S | 774.27 |
| | A2N056 | 4-[4-[3,5-Dimethoxy-4-[[4-[4-(3,3,3-trifluoropropylsulfonylcarbamoyl)phenyl]piperazin-1-yl]methyl]phenyl]phenyl]-1-hydroxy-N,N-dimethylnaphthalene-2-carboxamide | C42H43F3N4O7S | 804.28 |
| | A2N057 | 1-Hydroxy-N,N-dimethyl-4-[4-[4-[[4-[4-(3,3,3-trifluoropropylsulfonylcarbamoyl)phenyl]piperazin-1-yl]methyl]naphthalen-1-yl]phenyl]naphthalene-2-carboxamide | C44H41F3N4O5S | 794.27 |
| | A2N058 | 1-Hydroxy-N,N-dimethyl-4-[4-[4-[4-[4-(3,3,3-trifluoropropylsulfonylcarbamoyl)phenyl]piperazine-1-carbonyl]naphthalen-1-yl]phenyl]naphthalene-2-carboxamide | C44H39F3N4O6S | 808.25 |
| | A2N059 | 1-Hydroxy-N,N-dimethyl-4-[4-[5-[[4-[4-(3,3,3-trifluoropropylsulfonylcarbamoyl)phenyl]piperazin-1-yl]methyl]thiophen-3-yl]phenyl]naphthalene-2-carboxamide | C38H37F3N4O5S2 | 750.22 |
| | A2N006 | 6-[4-[[4-[4-[3-(Dimethylcarbamoyl)-4-hydroxynaphthalen-1-yl]phenyl]-3-fluorophenyl]methyl]piperazin-1-yl]-N-(3,3,3-trifluoropropylsulfonyl)pyridazine-3-carboxamide | C38H36F4N6O5S | 764.24 |
| | A2N060 | 1-Hydroxy-N,N-dimethyl-4-[4-[5-[4-[4-(3,3,3-trifluoropropylsulfonylcarbamoyl)phenyl]piperazine-1-carbonyl]thiophen-3-yl]phenyl]naphthalene-2-carboxamide | C38H35F3N4O6S2 | 764.2 |
| | A2N061 | 1-Hydroxy-N,N-dimethyl-4-[4-[3-(trifluoromethyl)-5-[[4-[4-(3,3,3-trifluoropropylsulfonylcarbamoyl)phenyl]piperazin-1-yl]methyl]phenyl]phenyl]naphthalene-2-carboxamide | C41H38F6N4O5S | 812.25 |
| | A2N062 | 1-Hydroxy-N,N-dimethyl-4-[4-[3-(trifluoromethyl)-5-[4-[4-(3,3,3-trifluoropropylsulfonylcarbamoyl)phenyl]piperazine-1-carbonyl]phenyl]phenyl]naphthalene-2-carboxamide | C41H36F6N4O6S | 826.23 |
| | A2N063 | 1-Hydroxy-N,N-dimethyl-4-[4-[3-(trifluoromethoxy)-5-[[4-[4-(3,3,3-trifluoropropylsulfonylcarbamoyl)phenyl]piperazin-1-yl]methyl]phenyl]phenyl]naphthalene-2-carboxamide | C41H38F6N4O6S | 828.24 |
| | A2N064 | 4-[4-[3-Ethoxy-5-[[4-[4-(3,3,3-trifluoropropylsulfonylcarbamoyl)phenyl]piperazin-1-yl]methyl]phenyl]phenyl]-1-hydroxy-N,N-dimethylnaphthalene-2-carboxamide | C42H43F3N4O6S | 788.29 |
| | A2N065 | 1-Hydroxy-N,N-dimethyl-4-[4-[3-propan-2-yloxy-5-[[4-[4-(3,3,3-trifluoropropylsulfonylcarbamoyl)phenyl]piperazin-1-yl]methyl]phenyl]phenyl]naphthalene-2-carboxamide | C43H45F3N4O6S | 802.3 |

| | | | | |
|---|---|---|---|---|
| | A2N066 | 4-[4-[3-tert-Butyl-5-[4-[4-(3,3,3-trifluoropropylsulfonylcarbamoyl)phenyl]piperazine-1-carbonyl]phenyl]phenyl]-1-hydroxy-N,N-dimethylnaphthalene-2-carboxamide | C44H45F3N4O6S | 814.3 |
| | A2N067 | 4-[4-[3-Fluoro-4-[1-[4-[4-(3,3,3-trifluoropropylsulfonylcarbamoyl)phenyl]piperazin-1-yl]ethyl]phenyl]phenyl]-1-hydroxy-N,N-dimethylnaphthalene-2-carboxamide | C41H40F4N4O5S | 776.27 |
| | A2N068 | 1-Hydroxy-N,N-dimethyl-4-[4-[2-[2-[[4-[4-(3,3,3-trifluoropropylsulfonylcarbamoyl)phenyl]piperazin-1-yl]methyl]phenyl]ethynyl]phenyl]naphthalene-2-carboxamide | C42H39F3N4O5S | 768.26 |
| | A2N069 | 1-Hydroxy-N,N-dimethyl-4-[4-[2-[3-(trifluoromethyl)-5-[4-[4-(3,3,3-trifluoropropylsulfonylcarbamoyl)phenyl]piperazine-1-carbonyl]phenyl]ethynyl]phenyl]naphthalene-2-carboxamide | C43H36F6N4O6S | 850.23 |

[Table 142]

| | | | | |
|---|---|---|---|---|
| mixED 13 | A2N007 | 6-[4-[4-[4-[3-(Dimethylcarbamoyl)-4-hydroxynaphthalen-1-yl]phenyl]-3-fluorobenzoyl]piperazin-1-yl]-N-(3,3,3-trifluoropropylsulfonyl)pyridazine-3-carboxamide | C38H34F4N6O6S | 778.22 |
| | A2N070 | 1-Hydroxy-N,N-dimethyl-4-[4-[2-[5-[4-[4-(3,3,3-trifluoropropylsulfonylcarbamoyl)phenyl]piperazine-1-carbonyl]pyridin-3-yl]ethynyl]phenyl]naphthalene-2-carboxamide | C41H36F3N5O6S | 783.23 |
| | A2N071 | 1-Hydroxy-N,N-dimethyl-4-[4-[1-[3-(trifluoromethyl)-5-[4-[4-(3,3,3-trifluoropropylsulfonylcarbamoyl)phenyl]piperazine-1-carbonyl]phenyl]pyrazol-4-yl]phenyl]naphthalene-2-carboxamide | C44H38F6N6O6S | 892.25 |
| | A2N072 | 1-Hydroxy-N,N-dimethyl-4-[5-[2-[[4-[4-(3,3,3-trifluoropropylsulfonylcarbamoyl)phenyl]piperazin-1-yl]methyl]phenyl]pyridin-2-yl]naphthalene-2-carboxamide | C39H38F3N5O5S | 745.25 |
| | A2N073 | 1-Hydroxy-N,N-dimethyl-4-[5-[2-[3-oxo-3-[4-[4-(3,3,3-trifluoropropylsulfonylcarbamoyl)phenyl]piperazin-1-yl]propyl]phenyl]pyridin-2-yl]naphthalene-2-carboxamide | C41H40F3N5O6S | 787.27 |
| | A2N074 | 1-Hydroxy-N,N-dimethyl-4-[5-[4-[1-[4-[4-(3,3,3-trifluoropropylsulfonylcarbamoyl)phenyl]piperazin-1-yl]ethyl]phenyl]pyridin-2-yl]naphthalene-2-carboxamide | C40H40F3N5O5S | 759.27 |
| | A2N075 | 1-Hydroxy-N,N-dimethyl-4-[5-[2-methyl-4-[[4-[4-(3,3,3-trifluoropropylsulfonylcarbamoyl)phenyl]piperazin-1-yl]methyl]phenyl]pyridin-2-yl]naphthalene-2-carboxamide | C40H40F3N5O5S | 759.27 |
| | A2N076 | 1-Hydroxy-N,N-dimethyl-4-[5-[2-methyl-4-[4-[4-(3,3,3-trifluoropropylsulfonylcarbamoyl)phenyl]piperazine-1-carbonyl]phenyl]pyridin-2-yl]naphthalene-2-carboxamide | C40H38F3N5O6S | 773.25 |
| | A2N077 | 4-[5-[3-Ethyl-4-[[4-[4-(3,3,3-trifluoropropylsulfonylcarbamoyl)phenyl]piperazin-1-yl]methyl]phenyl]pyridin-2-yl]-1-hydroxy-N,N-dimethylnaphthalene-2-carboxamide | C41H42F3N5O5S | 773.29 |
| | A2N078 | 4-[5-[2-Fluoro-4-[[4-[4-(3,3,3-trifluoropropylsulfonylcarbamoyl)phenyl]piperazin-1-yl]methyl]phenyl]pyridin-2-yl]-1-hydroxy-N,N-dimethylnaphthalene-2-carboxamide | C39H37F4N5O5S | 763.25 |
| | A2N079 | 4-[5-[2-Fluoro-4-[4-[4-(3,3,3-trifluoropropylsulfonylcarbamoyl)phenyl]piperazine-1-carbonyl]phenyl]pyridin-2-yl]-1-hydroxy-N,N-dimethylnaphthalene-2-carboxamide | C39H35F4N5O6S | 777.22 |
| | A2N008 | 6-[4-[[4-[4-[3-(Dimethylcarbamoyl)-4-hydroxynaphthalen-1-yl]phenyl]-2-methoxyphenyl]methyl]piperazin-1-yl]-N-(3,3,3-trifluoropropylsulfonyl)pyridazine-3-carboxamide | C39H39F3N6O6S | 776.26 |
| | A2N080 | 1-Hydroxy-4-[5-[3-methoxy-4-[[4-[4-(3,3,3-trifluoropropylsulfonylcarbamoyl)phenyl]piperazin-1-yl]methyl]phenyl]pyridin-2-yl]-N,N-dimethylnaphthalene-2-carboxamide | C40H40F3N5O6S | 775.27 |
| | A2N081 | 4-[5-[3,5-Dimethoxy-4-[[4-[4-(3,3,3-trifluoropropylsulfonylcarbamoyl)phenyl]piperazin-1-yl]methyl]phenyl]pyridin-2-yl]-1-hydroxy-N,N-dimethylnaphthalene-2-carboxamide | C41H42F3N5O7S | 805.28 |
| | A2N082 | 1-Hydroxy-N,N-dimethyl-4-[5-[4-[[4-[4-(3,3,3-trifluoropropylsulfonylcarbamoyl)phenyl]piperazin-1-yl]methyl]naphthalen-1-yl]pyridin-2-yl]naphthalene-2-carboxamide | C43H40F3N5O5S | 795.27 |
| | A2N083 | 1-Hydroxy-N,N-dimethyl-4-[5-[4-[4-[4-(3,3,3-trifluoropropylsulfonylcarbamoyl)phenyl]piperazine-1-carbonyl]naphthalen-1-yl]pyridin-2-yl]naphthalene-2-carboxamide | C43H38F3N5O6S | 809.25 |
| | A2N084 | 1-Hydroxy-N,N-dimethyl-4-[5-[5-[[4-[4-(3,3,3-trifluoropropylsulfonylcarbamoyl)phenyl]piperazin-1-yl]methyl]thiophen-3-yl]pyridin-2-yl]naphthalene-2-carboxamide | C37H36F3N5O5S2 | 751.21 |

| | | | | |
|---|---|---|---|---|
| | A2N085 | 1-Hydroxy-N,N-dimethyl-4-[5-[5-[4-[4-(3,3,3-trifluoropropylsulfonylcarbamoyl)phenyl]piperazine-1-carbonyl]thiophen-3-yl]pyridin-2-yl]naphthalene-2-carboxamide | C37H34F3N5O6S2 | 765.19 |
| | A2N086 | 1-Hydroxy-N,N-dimethyl-4-[5-[3-(trifluoromethyl)-5-[[4-[4-(3,3,3-trifluoropropylsulfonylcarbamoyl)phenyl]piperazin-1-yl]methyl]phenyl]pyridin-2-yl]naphthalene-2-carboxamide | C40H37F6N5O5S | 813.24 |
| | A2N087 | 1-Hydroxy-N,N-dimethyl-4-[5-[3-(trifluoromethyl)-5-[4-[4-(3,3,3-trifluoropropylsulfonylcarbamoyl)phenyl]piperazine-1-carbonyl]phenyl]pyridin-2-yl]naphthalene-2-carboxamide | C40H35F6N5O6S | 827.22 |
| | A2N088 | 1-Hydroxy-N,N-dimethyl-4-[5-[3-(trifluoromethoxy)-5-[[4-[4-(3,3,3-trifluoropropylsulfonylcarbamoyl)phenyl]piperazin-1-yl]methyl]phenyl]pyridin-2-yl]naphthalene-2-carboxamide | C40H37F6N5O6S | 829.24 |
| | A2N089 | 4-[5-[3-Ethoxy-5-[[4-[4-(3,3,3-trifluoropropylsulfonylcarbamoyl)phenyl]piperazin-1-yl]methyl]phenyl]pyridin-2-yl]-1-hydroxy-N,N-dimethylnaphthalene-2-carboxamide | C41H42F3N5O6S | 789.28 |
| | A2N009 | 6-[4-[[4-[4-[3-(Dimethylcarbamoyl)-4-hydroxynaphthalen-1-yl]phenyl]-2,6-dimethoxyphenyl]methyl]piperazin-1-yl]-N-(3,3,3-trifluoropropylsulfonyl)pyridazine-3-carboxamide | C40H41F3N6O7S | 806.27 |
| mixED 13 | A2N090 | 1-Hydroxy-N,N-dimethyl-4-[5-[3-propan-2-yloxy-5-[[4-[4-(3,3,3-trifluoropropylsulfonylcarbamoyl)phenyl]piperazin-1-yl]methyl]phenyl]pyridin-2-yl]naphthalene-2-carboxamide | C42H44F3N5O6S | 803.3 |
| | A2N091 | 4-[5-[3-tert-Butyl-5-[4-[4-(3,3,3-trifluoropropylsulfonylcarbamoyl)phenyl]piperazine-1-carbonyl]phenyl]pyridin-2-yl]-1-hydroxy-N,N-dimethylnaphthalene-2-carboxamide | C43H44F3N5O6S | 815.3 |
| | A2N092 | 4-[5-[3-Fluoro-4-[1-[4-[4-(3,3,3-trifluoropropylsulfonylcarbamoyl)phenyl]piperazin-1-yl]ethyl]phenyl]pyridin-2-yl]-1-hydroxy-N,N-dimethylnaphthalene-2-carboxamide | C40H39F4N5O5S | 777.26 |
| | A2N093 | 1-Hydroxy-N,N-dimethyl-4-[5-[2-[2-[[4-[4-(3,3,3-trifluoropropylsulfonylcarbamoyl)phenyl]piperazin-1-yl]methyl]phenyl]ethynyl]pyridin-2-yl]naphthalene-2-carboxamide | C41H38F3N5O5S | 769.25 |
| | A2N094 | 1-Hydroxy-N,N-dimethyl-4-[5-[2-[3-(trifluoromethyl)-5-[4-[4-(3,3,3-trifluoropropylsulfonylcarbamoyl)phenyl]piperazine-1-carbonyl]phenyl]ethynyl]pyridin-2-yl]naphthalene-2-carboxamide | C42H35F6N5O6S | 851.22 |
| | A2N095 | 1-Hydroxy-N,N-dimethyl-4-[5-[2-[5-[4-[4-(3,3,3-trifluoropropylsulfonylcarbamoyl)phenyl]piperazine-1-carbonyl]pyridin-3-yl]ethynyl]pyridin-2-yl]naphthalene-2-carboxamide | C40H35F3N6O6S | 784.23 |
| | A2N096 | 1-Hydroxy-N,N-dimethyl-4-[5-[1-[3-(trifluoromethyl)-5-[4-[4-(3,3,3-trifluoropropylsulfonylcarbamoyl)phenyl]piperazine-1-carbonyl]phenyl]pyrazol-4-yl]pyridin-2-yl]naphthalene-2-carboxamide | C43H37F6N7O6S | 893.24 |
| | A2N097 | 6-[4-[2-[2-[4-[3-(Dimethylcarbamoyl)-4-hydroxynaphthalen-1-yl]phenyl]ethynyl]benzoyl]piperazin-1-yl]-N-(3,3,3-trifluoropropylsulfonyl)pyridazine-3-carboxamide | C40H35F3N6O6S | 784.229 |
| | A2N098 | 6-[4-[2-[2-[6-[3-(Dimethylcarbamoyl)-4-hydroxynaphthalen-1-yl]pyridin-3-yl]ethynyl]benzoyl]piperazin-1-yl]-N-(3,3,3-trifluoropropylsulfonyl)pyridazine-3-carboxamide | C39H34F3N7O6S | 785.224 |
| | A2N099 | 1-Hydroxy-N,N-dimethyl-4-[4-[2-[2-[4-[4-(3,3,3-trifluoropropylsulfonylcarbamoyl)phenyl]piperazine-1-carbonyl]phenyl]ethynyl]phenyl]naphthalene-2-carboxamide | C42H37F3N4O6S | 782.239 |
| | A2N100 | 1-Hydroxy-N,N-dimethyl-4-[5-[2-[2-[4-[4-(3,3,3-trifluoropropylsulfonylcarbamoyl)phenyl]piperazine-1-carbonyl]phenyl]ethynyl]pyridin-2-yl]naphthalene-2-carboxamide | C41H36F3N5O6S | 783.234 |

[Table 143]

[Table EE10]

Table EE10

| Mixture No. | No. of compound that may be contained | Compound name | Compositional formula | Exact MS |
|---|---|---|---|---|
| mixED 14 | A2P001 | 6-[4-[[2-[4-[3-(Dimethylcarbamoyl)-4-hydroxynaphthalen-1-yl]phenyl]phenyl]methyl]piperazin-1-yl]-N-(4-nitrophenyl)sulfonylpyridazine-3-carboxamide | C41H37N7O7S | 771.25 |

| | | | | |
|---|---|---|---|---|
| | A2P010 | 6-[4-[[4-[4-[3-(Dimethylcarbamoyl)-4-hydroxynaphthalen-1-yl]phenyl]naphthalen-1-yl]methyl]piperazin-1-yl]-N-(4-nitrophenyl)sulfonylpyridazine-3-carboxamide | C45H39N7O7S | 821.26 |
| | A2P011 | 6-[4-[4-[4-[3-(Dimethylcarbamoyl)-4-hydroxynaphthalen-1-yl]phenyl]naphthalene-1-carbonyl]piperazin-1-yl]-N-(4-nitrophenyl)sulfonylpyridazine-3-carboxamide | C45H37N7O8S | 835.24 |
| | A2P012 | 6-[4-[[4-[4-[3-(Dimethylcarbamoyl)-4-hydroxynaphthalen-1-yl]phenyl]thiophen-2-yl]methyl]piperazin-1-yl]-N-(4-nitrophenyl)sulfonylpyridazine-3-carboxamide | C39H35N7O7S2 | 777.2 |
| | A2P013 | 6-[4-[4-[4-[3-(Dimethylcarbamoyl)-4-hydroxynaphthalen-1-yl]phenyl]thiophene-2-carbonyl]piperazin-1-yl]-N-(4-nitrophenyl)sulfonylpyridazine-3-carboxamide | C39H33N7O8S2 | 791.18 |
| | A2P014 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-(4-nitrophenyl)sulfonylpyridazine-3-carboxamide | C42H36F3N7O7S | 839.23 |
| | A2P015 | 6-[4-[3-[4-[3-(Dimethylcarbamoyl)-4-hydroxynaphthalen-1-yl]phenyl]-5-(trifluoromethyl)benzoyl]piperazin-1-yl]-N-(4-nitrophenyl)sulfonylpyridazine-3-carboxamide | C42H34F3N7O8S | 853.21 |
| | A2P016 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-(4-nitrophenyl)sulfonylpyridazine-3-carboxamide | C42H36F3N7O8S | 855.23 |
| mixED 14 | A2P017 | 6-[4-[[3-[4-[3-(Dimethylcarbamoyl)-4-hydroxynaphthalen-1-yl]phenyl]-5-ethoxyphenyl]methyl]piperazin-1-yl]-N-(4-nitrophenyl)sulfonylpyridazine-3-carboxamide | C43H41N7O8S | 815.27 |
| | A2P018 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-(4-nitrophenyl)sulfonylpyridazine-3-carboxamide | C44H43N7O8S | 829.29 |
| | A2P019 | 6-[4-[3-tert-Butyl-5-[4-[3-(dimethylcarbamoyl)-4-hydroxynaphthalen-1-yl]phenyl]benzoyl]piperazin-1-yl]-N-(4-nitrophenyl)sulfonylpyridazine-3-carboxamide | C45H43N7O8S | 841.29 |
| | A2P002 | 6-[4-[3-[2-[4-[3-(Dimethylcarbamoyl)-4-hydroxynaphthalen-1-yl]phenyl]phenyl]propanoyl]piperazin-1-yl]-N-(4-nitrophenyl)sulfonylpyridazine-3-carboxamide | C43H39N7O8S | 813.26 |
| | A2P020 | 6-[4-[[2-[2-[4-[3-(Dimethylcarbamoyl)-4-hydroxynaphthalen-1-yl]phenyl]ethynyl]phenyl]methyl]piperazin-1-yl]-N-(4-nitrophenyl)sulfonylpyridazine-3-carboxamide | C43H37N7O7S | 795.25 |
| | A2P021 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-(4-nitrophenyl)sulfonylpyridazine-3-carboxamide | C44H34F3N7O8S | 877.21 |
| | A2P022 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-(4-nitrophenyl)sulfonylpyridazine-3-carboxamide | C42H34N8O8S | 810.22 |
| | A2P023 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-(4-nitrophenyl)sulfonylpyridazine-3-carboxamide | C45H36F3N9O8S | 919.24 |
| | A2P024 | 6-[4-[[2-[6-[3-(Dimethylcarbamoyl)-4-hydroxynaphthalen-1-yl]pyridin-3-yl]phenyl]methyl]piperazin-1-yl]-N-(4-nitrophenyl)sulfonylpyridazine-3-carboxamide | C40H36N8O7S | 772.24 |
| | A2P025 | 6-[4-[3-[2-[6-[3-(Dimethylcarbamoyl)-4-hydroxynaphthalen-1-yl]pyridin-3-yl]phenyl]propanoyl]piperazin-1-yl]-N-(4-nitrophenyl)sulfonylpyridazine-3-carboxamide | C42H38N8O8S | 814.25 |
| | A2P026 | 6-[4-[[4-[6-[3-(Dimethylcarbamoyl)-4-hydroxynaphthalen-1-yl]pyridin-3-yl]-3-methylphenyl]methyl]piperazin-1-yl]-N-(4-nitrophenyl)sulfonylpyridazine-3-carboxamide | C41H38N8O7S | 786.26 |
| | A2P027 | 6-[4-[4-[6-[3-(Dimethylcarbamoyl)-4-hydroxynaphthalen-1-yl]pyridin-3-yl]-3-methylbenzoyl]piperazin-1-yl]-N-(4-nitrophenyl)sulfonylpyridazine-3-carboxamide | C41H36N8O8S | 800.24 |
| | A2P028 | 6-[4-[[4-[6-[3-(Dimethylcarbamoyl)-4-hydroxynaphthalen-1-yl]pyridin-3-yl]-2-ethylphenyl]methyl]piperazin-1-yl]-N-(4-nitrophenyl)sulfonylpyridazine-3-carboxamide | C42H40N8O7S | 800.27 |
| | A2P029 | 6-[4-[[4-[6-[3-(Dimethylcarbamoyl)-4-hydroxynaphthalen-1-yl]pyridin-3-yl]-3-fluorophenyl]methyl]piperazin-1-yl]-N-(4-nitrophenyl)sulfonylpyridazine-3-carboxamide | C40H35FN8O7S | 790.23 |
| | A2P003 | 6-[4-[[4-[4-[3-(Dimethylcarbamoyl)-4-hydroxynaphthalen-1-yl]phenyl]-3-methylphenyl]methyl]piperazin-1-yl]-N-(4-nitrophenyl)sulfonylpyridazine-3-carboxamide | C42H39N7O7S | 785.26 |

| | | | | |
|---|---|---|---|---|
| | A2P030 | 6-[4-[4-[6-[3-(Dimethylcarbamoyl)-4-hydroxynaphthalen-1-yl]pyridin-3-yl]-3-fluorobenzoyl]piperazin-1-yl]-N-(4-nitrophenyl)sulfonylpyridazine-3-carboxamide | C40H33FN8O8S | 804.21 |
| | A2P031 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-(4-nitrophenyl)sulfonylpyridazine-3-carboxamide | C41H38N8O8S | 802.25 |
| | A2P032 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-(4-nitrophenyl)sulfonylpyridazine-3-carboxamide | C42H40N8O9S | 832.26 |
| | A2P033 | 6-[4-[[4-[6-[3-(Dimethylcarbamoyl)-4-hydroxynaphthalen-1-yl]pyridin-3-yl]naphthalen-1-yl]methyl]piperazin-1-yl]-N-(4-nitrophenyl)sulfonylpyridazine-3-carboxamide | C44H38N8O7S | 822.26 |
| | A2P034 | 6-[4-[4-[6-[3-(Dimethylcarbamoyl)-4-hydroxynaphthalen-1-yl]pyridin-3-yl]naphthalene-1-carbonyl]piperazin-1-yl]-N-(4-nitrophenyl)sulfonylpyridazine-3-carboxamide | C44H36N8O8S | 836.24 |
| | A2P035 | 6-[4-[[4-[6-[3-(Dimethylcarbamoyl)-4-hydroxynaphthalen-1-yl]pyridin-3-yl]thiophen-2-yl]methyl]piperazin-1-yl]-N-(4-nitrophenyl)sulfonylpyridazine-3-carboxamide | C38H34N8O7S2 | 778.2 |
| | A2P036 | 6-[4-[4-[6-[3-(Dimethylcarbamoyl)-4-hydroxynaphthalen-1-yl]pyridin-3-yl]thiophene-2-carbonyl]piperazin-1-yl]-N-(4-nitrophenyl)sulfonylpyridazine-3-carboxamide | C38H32N8O8S2 | 792.18 |
| | A2P037 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-(4-nitrophenyl)sulfonylpyridazine-3-carboxamide | C41H35F3N8O7S | 840.23 |
| mixED 14 | A2P038 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-(4-nitrophenyl)sulfonylpyridazine-3-carboxamide | C41H33F3N8O8S | 854.21 |
| | A2P039 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-(4-nitrophenyl)sulfonylpyridazine-3-carboxamide | C41H35F3N8O8S | 856.23 |

[Table 144]

| | | | | |
|---|---|---|---|---|
| mixED 14 | A2P004 | 6-[4-[4-[4-[3-(Dimethylcarbamoyl)-4-hydroxynaphthalen-1-yl]phenyl]-3-methylbenzoyl]piperazin-1-yl]-N-(4-nitrophenyl)sulfonylpyridazine-3-carboxamide | C42H37N7O8S | 799.24 |
| | A2P040 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-(4-nitrophenyl)sulfonylpyridazine-3-carboxamide | C42H40N8O8S | 816.27 |
| | A2P041 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-(4-nitrophenyl)sulfonylpyridazine-3-carboxamide | C43H42N8O8S | 830.28 |
| | A2P042 | 6-[4-[3-tert-Butyl-5-[6-[3-(dimethylcarbamoyl)-4-hydroxynaphthalen-1-yl]pyridin-3-yl]benzoyl]piperazin-1-yl]-N-(4-nitrophenyl)sulfonylpyridazine-3-carboxamide | C44H42N8O8S | 842.28 |
| | A2P043 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-(4-nitrophenyl)sulfonylpyridazine-3-carboxamide | C42H36N8O7S | 796.24 |
| | A2P044 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-(4-nitrophenyl)sulfonylpyridazine-3-carboxamide | C43H33F3N8O8S | 878.21 |
| | A2P045 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-(4-nitrophenyl)sulfonylpyridazine-3-carboxamide | C41H33N9O8S | 811.22 |
| | A2P046 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-(4-nitrophenyl)sulfonylpyridazine-3-carboxamide | C44H35F3N10O8S | 920.23 |
| | A2P047 | 1-Hydroxy-N,N-dimethyl-4-[4-[2-[[4-[4-[(4-nitrophenyl)sulfonylcarbamoyl]phenyl]piperazin-1-yl]methyl]phenyl]phenyl]naphthalene-2-carboxamide | C43H39N5O7S | 769.26 |
| | A2P048 | 1-Hydroxy-N,N-dimethyl-4-[4-[2-[3-[4-[4-[(4-nitrophenyl)sulfonylcarbamoyl]phenyl]piperazin-1-yl]-3-oxopropyl]phenyl]phenyl]naphthalene-2-carboxamide | C45H41N5O8S | 811.27 |
| | A2P049 | 1-Hydroxy-N,N-dimethyl-4-[4-[4-[1-[4-[4-[(4-nitrophenyl)sulfonylcarbamoyl]phenyl]piperazin-1-yl]ethyl]phenyl]phenyl]naphthalene-2-carboxamide | C44H41N5O7S | 783.27 |
| | A2P005 | 6-[4-[[4-[4-[3-(Dimethylcarbamoyl)-4-hydroxynaphthalen-1-yl]phenyl]-2-ethylphenyl]methyl]piperazin-1-yl]-N-(4-nitrophenyl)sulfonylpyridazine-3-carboxamide | C43H41N7O7S | 799.28 |
| | A2P050 | 1-Hydroxy-N,N-dimethyl-4-[4-[2-methyl-4-[[4-[4-[(4-nitrophenyl)sulfonylcarbamoyl]phenyl]piperazin-1-yl]methyl]phenyl]phenyl]naphthalene-2-carboxamide | C44H41N5O7S | 783.27 |
| | A2P051 | 1-Hydroxy-N,N-dimethyl-4-[4-[2-methyl-4-[4-[4-[(4-nitrophenyl)sulfonylcarbamoyl]phenyl]piperazine-1-carbonyl]phenyl]phenyl]naphthalene-2-carboxamide | C44H39N5O8S | 797.25 |
| | A2P052 | 4-[4-[3-Ethyl-4-[[4-[4-[(4-nitrophenyl)sulfonylcarbamoyl]phenyl]piperazin-1-yl]methyl]phenyl]phenyl]-1-hydroxy-N,N-dimethylnaphthalene-2-carboxamide | C45H43N5O7S | 797.29 |
| | A2P053 | 4-[4-[2-Fluoro-4-[[4-[4-[(4-nitrophenyl)sulfonylcarbamoyl]phenyl]piperazin-1-yl]methyl]phenyl]phenyl]-1-hydroxy-N,N-dimethylnaphthalene-2-carboxamide | C43H38FN5O7S | 787.25 |

| | | | | |
|---|---|---|---|---|
| | A2P054 | 4-[4-[2-Fluoro-4-[4-[4-[(4-nitrophenyl)sulfonylcarbamoyl]phenyl]piperazine-1-carbonyl]phenyl]phenyl]-1-hydroxy-N,N-dimethylnaphthalene-2-carboxamide | C43H36FN5O8S | 801.23 |
| | A2P055 | 1-Hydroxy-4-[4-[3-methoxy-4-[[4-[4-[(4-nitrophenyl)sulfonylcarbamoyl]phenyl]piperazin-1-yl]methyl]phenyl]phenyl]-N,N-dimethylnaphthalene-2-carboxamide | C44H41N5O8S | 799.27 |
| | A2P056 | 4-[4-[3,5-Dimethoxy-4-[[4-[4-[(4-nitrophenyl)sulfonylcarbamoyl]phenyl]piperazin-1-yl]methyl]phenyl]phenyl]-1-hydroxy-N,N-dimethylnaphthalene-2-carboxamide | C45H43N5O9S | 829.28 |
| | A2P057 | 1-Hydroxy-N,N-dimethyl-4-[4-[4-[[4-[4-[(4-nitrophenyl)sulfonylcarbamoyl]phenyl]piperazin-1-yl]methyl]naphthalen-1-yl]phenyl]naphthalene-2-carboxamide | C47H41N5O7S | 819.27 |
| mixED 14 | A2P058 | 1-Hydroxy-N,N-dimethyl-4-[4-[4-[4-[4-[(4-nitrophenyl)sulfonylcarbamoyl]phenyl]piperazine-1-carbonyl]naphthalen-1-yl]phenyl]naphthalene-2-carboxamide | C47H39N5O8S | 833.25 |
| | A2P059 | 1-Hydroxy-N,N-dimethyl-4-[4-[5-[[4-[4-[(4-nitrophenyl)sulfonylcarbamoyl]phenyl]piperazin-1-yl]methyl]thiophen-3-yl]phenyl]naphthalene-2-carboxamide | C41H37N5O7S2 | 775.21 |
| | A2P006 | 6-[4-[[4-[4-[3-(Dimethylcarbamoyl)-4-hydroxynaphthalen-1-yl]phenyl]-3-fluorophenyl]methyl]piperazin-1-yl]-N-(4-nitrophenyl)sulfonylpyridazine-3-carboxamide | C41H36FN7O7S | 789.24 |
| | A2P060 | 1-Hydroxy-N,N-dimethyl-4-[4-[5-[4-[4-[(4-nitrophenyl)sulfonylcarbamoyl]phenyl]piperazine-1-carbonyl]thiophen-3-yl]phenyl]naphthalene-2-carboxamide | C41H35N5O8S2 | 789.19 |
| | A2P061 | 1-Hydroxy-N,N-dimethyl-4-[4-[3-[[4-[4-[(4-nitrophenyl)sulfonylcarbamoyl]phenyl]piperazin-1-yl]methyl]-5-(trifluoromethyl)phenyl]phenyl]naphthalene-2-carboxamide | C44H38F3N5O7S | 837.24 |
| | A2P062 | 1-Hydroxy-N,N-dimethyl-4-[4-[3-[4-[4-[(4-nitrophenyl)sulfonylcarbamoyl]phenyl]piperazine-1-carbonyl]-5-(trifluoromethyl)phenyl]phenyl]naphthalene-2-carboxamide | C44H36F3N5O8S | 851.22 |
| | A2P063 | 1-Hydroxy-N,N-dimethyl-4-[4-[3-[[4-[4-[(4-nitrophenyl)sulfonylcarbamoyl]phenyl]piperazin-1-yl]methyl]-5-(trifluoromethoxy)phenyl]phenyl]naphthalene-2-carboxamide | C44H38F3N5O8S | 853.24 |
| | A2P064 | 4-[4-[3-Ethoxy-5-[[4-[4-[(4-nitrophenyl)sulfonylcarbamoyl]phenyl]piperazin-1-yl]methyl]phenyl]phenyl]-1-hydroxy-N,N-dimethylnaphthalene-2-carboxamide | C45H43N5O8S | 813.28 |
| | A2P065 | 1-Hydroxy-N,N-dimethyl-4-[4-[3-[[4-[4-[(4-nitrophenyl)sulfonylcarbamoyl]phenyl]piperazin-1-yl]methyl]-5-propan-2-yloxyphenyl]phenyl]naphthalene-2-carboxamide | C46H45N5O8S | 827.3 |
| | A2P066 | 4-[4-[3-tert-Butyl-5-[4-[4-[(4-nitrophenyl)sulfonylcarbamoyl]phenyl]piperazine-1-carbonyl]phenyl]phenyl]-1-hydroxy-N,N-dimethylnaphthalene-2-carboxamide | C47H45N5O8S | 839.3 |
| | A2P067 | 4-[4-[3-Fluoro-4-[1-[4-[4-[(4-nitrophenyl)sulfonylcarbamoyl]phenyl]piperazin-1-yl]ethyl]phenyl]phenyl]-1-hydroxy-N,N-dimethylnaphthalene-2-carboxamide | C44H40FN5O7S | 801.26 |
| | A2P068 | 1-Hydroxy-N,N-dimethyl-4-[4-[2-[2-[[4-[4-[(4-nitrophenyl)sulfonylcarbamoyl]phenyl]piperazin-1-yl]methyl]phenyl]ethynyl]phenyl]naphthalene-2-carboxamide | C45H39N5O7S | 793.26 |
| | A2P069 | 1-Hydroxy-N,N-dimethyl-4-[4-[2-[3-[4-[4-[(4-nitrophenyl)sulfonylcarbamoyl]phenyl]piperazine-1-carbonyl]-5-(trifluoromethyl)phenyl]ethynyl]phenyl]naphthalene-2-carboxamide | C46H36F3N5O8S | 875.22 |

[Table 145]

| | | | | |
|---|---|---|---|---|
| mixED 14 | A2P007 | 6-[4-[4-[4-[3-(Dimethylcarbamoyl)-4-hydroxynaphthalen-1-yl]phenyl]-3-fluorobenzoyl]piperazin-1-yl]-N-(4-nitrophenyl)sulfonylpyridazine-3-carboxamide | C41H34FN7O8S | 803.22 |
| | A2P070 | 1-Hydroxy-N,N-dimethyl-4-[4-[2-[5-[4-[4-[(4-nitrophenyl)sulfonylcarbamoyl]phenyl]piperazine-1-carbonyl]pyridin-3-yl]ethynyl]phenyl]naphthalene-2-carboxamide | C44H36N6O8S | 808.23 |
| | A2P071 | 1-Hydroxy-N,N-dimethyl-4-[4-[1-[3-[4-[4-[(4-nitrophenyl)sulfonylcarbamoyl]phenyl]piperazine-1-carbonyl]-5-(trifluoromethyl)phenyl]pyrazol-4-yl]phenyl]naphthalene-2-carboxamide | C47H38F3N7O8S | 917.25 |
| | A2P072 | 1-Hydroxy-N,N-dimethyl-4-[5-[2-[[4-[4-[(4-nitrophenyl)sulfonylcarbamoyl]phenyl]piperazin-1-yl]methyl]phenyl]pyridin-2-yl]naphthalene-2-carboxamide | C42H38N6O7S | 770.25 |
| | A2P073 | 1-Hydroxy-N,N-dimethyl-4-[5-[2-[3-[4-[4-[(4-nitrophenyl)sulfonylcarbamoyl]phenyl]piperazin-1-yl]-3-oxopropyl]phenyl]pyridin-2-yl]naphthalene-2-carboxamide | C44H40N6O8S | 812.26 |
| | A2P074 | 1-Hydroxy-N,N-dimethyl-4-[5-[4-[1-[4-[4-[(4-nitrophenyl)sulfonylcarbamoyl]phenyl]piperazin-1-yl]ethyl]phenyl]pyridin-2-yl]naphthalene-2-carboxamide | C43H40N6O7S | 784.27 |
| | A2P075 | 1-Hydroxy-N,N-dimethyl-4-[5-[2-methyl-4-[[4-[4-[(4-nitrophenyl)sulfonylcarbamoyl]phenyl]piperazin-1-yl]methyl]phenyl]pyridin-2-yl]naphthalene-2-carboxamide | C43H40N6O7S | 784.27 |
| | A2P076 | 1-Hydroxy-N,N-dimethyl-4-[5-[2-methyl-4-[4-[4-[(4-nitrophenyl)sulfonylcarbamoyl]phenyl]piperazine-1-carbonyl]phenyl]pyridin-2-yl]naphthalene-2-carboxamide | C43H38N6O8S | 798.25 |
| | A2P077 | 4-[5-[3-Ethyl-4-[[4-[4-[(4-nitrophenyl)sulfonylcarbamoyl]phenyl]piperazin-1-yl]methyl]phenyl]pyridin-2-yl]-1-hydroxy-N,N-dimethylnaphthalene-2-carboxamide | C44H42N6O7S | 798.28 |
| mixED 14 | A2P078 | 4-[5-[2-Fluoro-4-[[4-[4-[(4-nitrophenyl)sulfonylcarbamoyl]phenyl]piperazin-1-yl]methyl]phenyl]pyridin-2-yl]-1-hydroxy-N,N-dimethylnaphthalene-2-carboxamide | C42H37FN6O7S | 788.24 |

| | | | | |
|---|---|---|---|---|
| | A2P079 | 4-[5-[2-Fluoro-4-[4-[4-[(4-nitrophenyl)sulfonylcarbamoyl]phenyl]piperazine-1-carbonyl]phenyl]pyridin-2-yl]-1-hydroxy-N,N-dimethylnaphthalene-2-carboxamide | C42H35FN6O8S | 802.22 |
| | A2P008 | 6-[4-[[4-[4-[3-(Dimethylcarbamoyl)-4-hydroxynaphthalen-1-yl]phenyl]-2-methoxyphenyl]methyl]piperazin-1-yl]-N-(4-nitrophenyl)sulfonylpyridazine-3-carboxamide | C42H39N7O8S | 801.26 |
| | A2P080 | 1-Hydroxy-4-[5-[3-methoxy-4-[[4-[4-[(4-nitrophenyl)sulfonylcarbamoyl]phenyl]piperazin-1-yl]methyl]phenyl]pyridin-2-yl]-N,N-dimethylnaphthalene-2-carboxamide | C43H40N6O8S | 800.26 |
| | A2P081 | 4-[5-[3,5-Dimethoxy-4-[[4-[4-[(4-nitrophenyl)sulfonylcarbamoyl]phenyl]piperazin-1-yl]methyl]phenyl]pyridin-2-yl]-1-hydroxy-N,N-dimethylnaphthalene-2-carboxamide | C44H42N6O9S | 830.27 |
| | A2P082 | 1-Hydroxy-N,N-dimethyl-4-[5-[4-[[4-[4-[(4-nitrophenyl)sulfonylcarbamoyl]phenyl]piperazin-1-yl]methyl]naphthalen-1-yl]pyridin-2-yl]naphthalene-2-carboxamide | C46H40N6O7S | 820.27 |
| | A2P083 | 1-Hydroxy-N,N-dimethyl-4-[5-[4-[4-[4-[(4-nitrophenyl)sulfonylcarbamoyl]phenyl]piperazine-1-carbonyl]naphthalen-1-yl]pyridin-2-yl]naphthalene-2-carboxamide | C46H38N6O8S | 834.25 |
| | A2P084 | 1-Hydroxy-N,N-dimethyl-4-[5-[5-[[4-[4-[(4-nitrophenyl)sulfonylcarbamoyl]phenyl]piperazin-1-yl]methyl]thiophen-3-yl]pyridin-2-yl]naphthalene-2-carboxamide | C40H36N6O7S2 | 776.21 |
| | A2P085 | 1-Hydroxy-N,N-dimethyl-4-[5-[5-[4-[4-[(4-nitrophenyl)sulfonylcarbamoyl]phenyl]piperazine-1-carbonyl]thiophen-3-yl]pyridin-2-yl]naphthalene-2-carboxamide | C40H34N6O8S2 | 790.19 |
| | A2P086 | 1-Hydroxy-N,N-dimethyl-4-[5-[3-[[4-[4-[(4-nitrophenyl)sulfonylcarbamoyl]phenyl]piperazin-1-yl]methyl]-5-(trifluoromethyl)phenyl]pyridin-2-yl]naphthalene-2-carboxamide | C43H37F3N6O7S | 838.24 |
| | A2P087 | 1-Hydroxy-N,N-dimethyl-4-[5-[3-[4-[4-[(4-nitrophenyl)sulfonylcarbamoyl]phenyl]piperazine-1-carbonyl]-5-(trifluoromethyl)phenyl]pyridin-2-yl]naphthalene-2-carboxamide | C43H35F3N6O8S | 852.22 |
| | A2P088 | 1-Hydroxy-N,N-dimethyl-4-[5-[3-[[4-[4-[(4-nitrophenyl)sulfonylcarbamoyl]phenyl]piperazin-1-yl]methyl]-5-(trifluoromethoxy)phenyl]pyridin-2-yl]naphthalene-2-carboxamide | C43H37F3N6O8S | 854.23 |
| | A2P089 | 4-[5-[3-Ethoxy-5-[[4-[4-[(4-nitrophenyl)sulfonylcarbamoyl]phenyl]piperazin-1-yl]methyl]phenyl]pyridin-2-yl]-1-hydroxy-N,N-dimethylnaphthalene-2-carboxamide | C44H42N6O8S | 814.28 |
| | A2P009 | 6-[4-[[4-[4-[3-(Dimethylcarbamoyl)-4-hydroxynaphthalen-1-yl]phenyl]-2,6-dimethoxyphenyl]methyl]piperazin-1-yl]-N-(4-nitrophenyl)sulfonylpyridazine-3-carboxamide | C43H41N7O9S | 831.27 |
| | A2P090 | 1-Hydroxy-N,N-dimethyl-4-[5-[3-[[4-[4-[(4-nitrophenyl)sulfonylcarbamoyl]phenyl]piperazin-1-yl]methyl]-5-propan-2-yloxyphenyl]pyridin-2-yl]naphthalene-2-carboxamide | C45H44N6O8S | 828.29 |
| | A2P091 | 4-[5-[3-tert-Butyl-5-[4-[4-[(4-nitrophenyl)sulfonylcarbamoyl]phenyl]piperazine-1-carbonyl]phenyl]pyridin-2-yl]-1-hydroxy-N,N-dimethylnaphthalene-2-carboxamide | C46H44N6O8S | 840.29 |
| | A2P092 | 4-[5-[3-Fluoro-4-[1-[4-[4-[(4-nitrophenyl)sulfonylcarbamoyl]phenyl]piperazin-1-yl]ethyl]phenyl]pyridin-2-yl]-1-hydroxy-N,N-dimethylnaphthalene-2-carboxamide | C43H39FN6O7S | 802.26 |
| | A2P093 | 1-Hydroxy-N,N-dimethyl-4-[5-[2-[2-[[4-[4-[(4-nitrophenyl)sulfonylcarbamoyl]phenyl]piperazin-1-yl]methyl]phenyl]ethynyl]pyridin-2-yl]naphthalene-2-carboxamide | C44H38N6O7S | 794.25 |
| | A2P094 | 1-Hydroxy-N,N-dimethyl-4-[5-[2-[3-[4-[4-[(4-nitrophenyl)sulfonylcarbamoyl]phenyl]piperazine-1-carbonyl]-5-(trifluoromethyl)phenyl]ethynyl]pyridin-2-yl]naphthalene-2-carboxamide | C45H35F3N6O8S | 876.22 |
| | A2P095 | 1-Hydroxy-N,N-dimethyl-4-[5-[2-[5-[4-[4-[(4-nitrophenyl)sulfonylcarbamoyl]phenyl]piperazine-1-carbonyl]pyridin-3-yl]ethynyl]pyridin-2-yl]naphthalene-2-carboxamide | C43H35N7O8S | 809.23 |
| | A2P096 | 1-Hydroxy-N,N-dimethyl-4-[5-[1-[3-[4-[4-[(4-nitrophenyl)sulfonylcarbamoyl]phenyl]piperazine-1-carbonyl]-5-(trifluoromethyl)phenyl]pyrazol-4-yl]pyridin-2-yl]naphthalene-2-carboxamide | C46H37F3N8O8S | 918.24 |
| | A2P097 | 6-[4-[2-[2-[4-[3-(Dimethylcarbamoyl)-4-hydroxynaphthalen-1-yl]phenyl]ethynyl]benzoyl]piperazin-1-yl]-N-(4-nitrophenyl)sulfonylpyridazine-3-carboxamide | C43H35N7O8S | 809.227 |
| | A2P098 | 6-[4-[2-[2-[6-[3-(Dimethylcarbamoyl)-4-hydroxynaphthalen-1-yl]pyridin-3-yl]ethynyl]benzoyl]piperazin-1-yl]-N-(4-nitrophenyl)sulfonylpyridazine-3-carboxamide | C42H34N8O8S | 810.222 |

| | | | | |
|---|---|---|---|---|
| mixED 14 | A2P099 | 1-Hydroxy-N,N-dimethyl-4-[4-[2-[2-[4-[4-[(4-nitrophenyl)sulfonylcarbamoyl]phenyl]piperazine-1-carbonyl]phenyl]ethynyl]phenyl]naphthalene-2-carboxamide | C45H37N5O8S | 807.236 |
| | A2P100 | 1-Hydroxy-N,N-dimethyl-4-[5-[2-[2-[4-[4-[(4-nitrophenyl)sulfonylcarbamoyl]phenyl]piperazine-1-carbonyl]phenyl]ethynyl]pyridin-2-yl]naphthalene-2-carboxamide | C44H36N6O8S | 808.232 |

[Table 146]

[Table EE11]

| Table EE11 | | | | |
|---|---|---|---|---|
| Mixture No. | No. of compound that may be contained | Compound name | Compositional formula | Exact MS |
| mixED 15 | A2A001 | N-(1-Adamantylmethylsulfonyl)-6-[4-[[2-[4-[3-(dimethylcarbamoyl)-4-hydroxynaphthalen-1-yl]phenyl]phenyl]methyl]piperazin-1-yl]pyridazine-3-carboxamide | C46H50N6O5S | 798.36 |
| | A2A010 | N-(1-Adamantylmethylsulfonyl)-6-[4-[[4-[4-[3-(dimethylcarbamoyl)-4-hydroxynaphthalen-1-yl]phenyl]naphthalen-1-yl]methyl]piperazin-1-yl]pyridazine-3-carboxamide | C50H52N6O5S | 848.37 |
| | A2A011 | N-(1-Adamantylmethylsulfonyl)-6-[4-[4-[4-[3-(dimethylcarbamoyl)-4-hydroxynaphthalen-1-yl]phenyl]naphthalene-1-carbonyl]piperazin-1-yl]pyridazine-3-carboxamide | C50H50N6O6S | 862.35 |
| | A2A012 | N-(1-Adamantylmethylsulfonyl)-6-[4-[[4-[4-[3-(dimethylcarbamoyl)-4-hydroxynaphthalen-1-yl]phenyl]thiophen-2-yl]methyl]piperazin-1-yl]pyridazine-3-carboxamide | C44H48N6O5S2 | 804.31 |
| | A2A013 | N-(1-Adamantylmethylsulfonyl)-6-[4-[4-[4-[3-(dimethylcarbamoyl)-4-hydroxynaphthalen-1-yl]phenyl]thiophene-2-carbonyl]piperazin-1-yl]pyridazine-3-carboxamide | C44H46N6O6S2 | 818.29 |
| | A2A014 | N-(1-Adamantylmethylsulfonyl)-6-[4-[[3-[4-[3-(dimethylcarbamoyl)-4-hydroxynaphthalen-1-yl]phenyl]-5-(trifluoromethyl)phenyl]methyl]piperazin-1-yl]pyridazine-3-carboxamide | C47H49F3N6O5S | 866.34 |
| | A2A015 | N-(1-Adamantylmethylsulfonyl)-6-[4-[3-[4-[3-(dimethylcarbamoyl)-4-hydroxynaphthalen-1-yl]phenyl]-5-(trifluoromethyl)benzoyl]piperazin-1-yl]pyridazine-3-carboxamide | C47H47F3N6O6S | 880.32 |
| | A2A016 | N-(1-Adamantylmethylsulfonyl)-6-[4-[[3-[4-[3-(dimethylcarbamoyl)-4-hydroxynaphthalen-1-yl]phenyl]-5-(trifluoromethoxy)phenyl]methyl]piperazin-1-yl]pyridazine-3-carboxamide | C47H49F3N6O6S | 882.34 |
| | A2A017 | N-(1-Adamantylmethylsulfonyl)-6-[4-[[3-[4-[3-(dimethylcarbamoyl)-4-hydroxynaphthalen-1-yl]phenyl]-5-ethoxyphenyl]methyl]piperazin-1-yl]pyridazine-3-carboxamide | C48H54N6O6S | 842.38 |
| | A2A018 | N-(1-Adamantylmethylsulfonyl)-6-[4-[[3-[4-[3-(dimethylcarbamoyl)-4-hydroxynaphthalen-1-yl]phenyl]-5-propan-2-yloxyphenyl]methyl]piperazin-1-yl]pyridazine-3-carboxamide | C49H56N6O6S | 856.4 |
| | A2A019 | N-(1-Adamantylmethylsulfonyl)-6-[4-[3-tert-butyl-5-[4-[3-(dimethylcarbamoyl)-4-hydroxynaphthalen-1-yl]phenyl]benzoyl]piperazin-1-yl]pyridazine-3-carboxamide | C50H56N6O6S | 868.4 |
| | A2A002 | N-(1-Adamantylmethylsulfonyl)-6-[4-[3-[2-[4-[3-(dimethylcarbamoyl)-4-hydroxynaphthalen-1-yl]phenyl]phenyl]propanoyl]piperazin-1-yl]pyridazine-3-carboxamide | C48H52N6O6S | 840.37 |
| | A2A020 | N-(1-Adamantylmethylsulfonyl)-6-[4-[[2-[2-[4-[3-(dimethylcarbamoyl)-4-hydroxynaphthalen-1-yl]phenyl]ethynyl]phenyl]methyl]piperazin-1-yl]pyridazine-3-carboxamide | C48H50N6O5S | 822.36 |
| | A2A021 | N-(1-Adamantylmethylsulfonyl)-6-[4-[3-[2-[4-[3-(dimethylcarbamoyl)-4-hydroxynaphthalen-1-yl]phenyl]ethynyl]-5-(trifluoromethyl)benzoyl]piperazin-1-yl]pyridazine-3-carboxamide | C49H47F3N6O6S | 904.32 |
| | A2A022 | N-(1-Adamantylmethylsulfonyl)-6-[4-[5-[2-[4-[3-(dimethylcarbamoyl)-4-hydroxynaphthalen-1-yl]phenyl]ethynyl]pyridine-3-carbonyl]piperazin-1-yl]pyridazine-3-carboxamide | C47H47N7O6S | 837.33 |
| | A2A023 | N-(1-Adamantylmethylsulfonyl)-6-[4-[3-[4-[4-[3-(dimethylcarbamoyl)-4-hydroxynaphthalen-1-yl]phenyl]pyrazol-1-yl]-5-(trifluoromethyl)benzoyl]piperazin-1-yl]pyridazine-3-carboxamide | C50H49F3N8O6S | 946.34 |

| | | | | |
|---|---|---|---|---|
| mixED 15 | A2A024 | N-(1-Adamantylmethylsulfonyl)-6-[4-[[2-[6-[3-(dimethylcarbamoyl)-4-hydroxynaphthalen-1-yl]pyridin-3-yl]phenyl]methyl]piperazin-1-yl]pyridazine-3-carboxamide | C45H49N7O5S | 799.35 |
| | A2A025 | N-(1-Adamantylmethylsulfonyl)-6-[4-[3-[2-[6-[3-(dimethylcarbamoyl)-4-hydroxynaphthalen-1-yl]pyridin-3-yl]phenyl]propanoyl]piperazin-1-yl]pyridazine-3-carboxamide | C47H51N7O6S | 841.36 |
| | A2A026 | N-(1-Adamantylmethylsulfonyl)-6-[4-[[4-[6-[3-(dimethylcarbamoyl)-4-hydroxynaphthalen-1-yl]pyridin-3-yl]-3-methylphenyl]methyl]piperazin-1-yl]pyridazine-3-carboxamide | C46H51N7O5S | 813.37 |
| | A2A027 | N-(1-Adamantylmethylsulfonyl)-6-[4-[4-[6-[3-(dimethylcarbamoyl)-4-hydroxynaphthalen-1-yl]pyridin-3-yl]-3-methylbenzoyl]piperazin-1-yl]pyridazine-3-carboxamide | C46H49N7O6S | 827.35 |
| | A2A028 | N-(1-Adamantylmethylsulfonyl)-6-[4-[[4-[6-[3-(dimethylcarbamoyl)-4-hydroxynaphthalen-1-yl]pyridin-3-yl]-2-ethylphenyl]methyl]piperazin-1-yl]pyridazine-3-carboxamide | C47H53N7O5S | 827.38 |
| | A2A029 | N-(1-Adamantylmethylsulfonyl)-6-[4-[[4-[6-[3-(dimethylcarbamoyl)-4-hydroxynaphthalen-1-yl]pyridin-3-yl]-3-fluorophenyl]methyl]piperazin-1-yl]pyridazine-3-carboxamide | C45H48FN7O5S | 817.34 |
| | A2A003 | N-(1-Adamantylmethylsulfonyl)-6-[4-[[4-[4-[3-(dimethylcarbamoyl)-4-hydroxynaphthalen-1-yl]phenyl]-3-methylphenyl]methyl]piperazin-1-yl]pyridazine-3-carboxamide | C47H52N6O5S | 812.37 |
| | A2A030 | N-(1-Adamantylmethylsulfonyl)-6-[4-[4-[6-[3-(dimethylcarbamoyl)-4-hydroxynaphthalen-1-yl]pyridin-3-yl]-3-fluorobenzoyl]piperazin-1-yl]pyridazine-3-carboxamide | C45H46FN7O6S | 831.32 |
| | A2A031 | N-(1-Adamantylmethylsulfonyl)-6-[4-[[4-[6-[3-(dimethylcarbamoyl)-4-hydroxynaphthalen-1-yl]pyridin-3-yl]-2-methoxyphenyl]methyl]piperazin-1-yl]pyridazine-3-carboxamide | C46H51N7O6S | 829.36 |
| | A2A032 | N-(1-Adamantylmethylsulfonyl)-6-[4-[[4-[6-[3-(dimethylcarbamoyl)-4-hydroxynaphthalen-1-yl]pyridin-3-yl]-2,6-dimethoxyphenyl]methyl]piperazin-1-yl]pyridazine-3-carboxamide | C47H53N7O7S | 859.37 |
| | A2A033 | N-(1-Adamantylmethylsulfonyl)-6-[4-[[4-[6-[3-(dimethylcarbamoyl)-4-hydroxynaphthalen-1-yl]pyridin-3-yl]naphthalen-1-yl]methyl]piperazin-1-yl]pyridazine-3-carboxamide | C49H51N7O5S | 849.37 |
| | A2A034 | N-(1-Adamantylmethylsulfonyl)-6-[4-[4-[6-[3-(dimethylcarbamoyl)-4-hydroxynaphthalen-1-yl]pyridin-3-yl]naphthalene-1-carbonyl]piperazin-1-yl]pyridazine-3-carboxamide | C49H49N7O6S | 863.35 |
| | A2A035 | N-(1-Adamantylmethylsulfonyl)-6-[4-[[4-[6-[3-(dimethylcarbamoyl)-4-hydroxynaphthalen-1-yl]pyridin-3-yl]thiophen-2-yl]methyl]piperazin-1-yl]pyridazine-3-carboxamide | C43H47N7O5S2 | 805.31 |
| | A2A036 | N-(1-Adamantylmethylsulfonyl)-6-[4-[4-[6-[3-(dimethylcarbamoyl)-4-hydroxynaphthalen-1-yl]pyridin-3-yl]thiophene-2-carbonyl]piperazin-1-yl]pyridazine-3-carboxamide | C43H45N7O6S2 | 819.29 |
| | A2A037 | N-(1-Adamantylmethylsulfonyl)-6-[4-[[3-[6-[3-(dimethylcarbamoyl)-4-hydroxynaphthalen-1-yl]pyridin-3-yl]-5-(trifluoromethyl)phenyl]methyl]piperazin-1-yl]pyridazine-3-carboxamide | C46H48F3N7O5S | 867.34 |
| | A2A038 | N-(1-Adamantylmethylsulfonyl)-6-[4-[3-[6-[3-(dimethylcarbamoyl)-4-hydroxynaphthalen-1-yl]pyridin-3-yl]-5-(trifluoromethyl)benzoyl]piperazin-1-yl]pyridazine-3-carboxamide | C46H46F3N7O6S | 881.32 |
| | A2A039 | N-(1-Adamantylmethylsulfonyl)-6-[4-[[3-[6-[3-(dimethylcarbamoyl)-4-hydroxynaphthalen-1-yl]pyridin-3-yl]-5-(trifluoromethoxy)phenyl]methyl]piperazin-1-yl]pyridazine-3-carboxamide | C46H48F3N7O6S | 883.33 |

[Table 147]

| | | | | |
|---|---|---|---|---|
| mixED 15 | A2A004 | N-(1-Adamantylmethylsulfonyl)-6-[4-[4-[4-[3-(dimethylcarbamoyl)-4-hydroxynaphthalen-1-yl]phenyl]-3-methylbenzoyl]piperazin-1-yl]pyridazine-3-carboxamide | C47H50N6O6S | 826.35 |
| | A2A040 | N-(1-Adamantylmethylsulfonyl)-6-[4-[[3-[6-[3-(dimethylcarbamoyl)-4-hydroxynaphthalen-1-yl]pyridin-3-yl]-5-ethoxyphenyl]methyl]piperazin-1-yl]pyridazine-3-carboxamide | C47H53N7O6S | 843.38 |
| | A2A041 | N-(1-Adamantylmethylsulfonyl)-6-[4-[[3-[6-[3-(dimethylcarbamoyl)-4-hydroxynaphthalen-1-yl]pyridin-3-yl]-5-propan-2-yloxyphenyl]methyl]piperazin-1-yl]pyridazine-3-carboxamide | C48H55N7O6S | 857.39 |

| | | | | |
|---|---|---|---|---|
| | A2A042 | N-(1-Adamantylmethylsulfonyl)-6-[4-[3-tert-butyl-5-[6-[3-(dimethylcarbamoyl)-4-hydroxynaphthalen-1-yl]pyridin-3-yl]benzoyl]piperazin-1-yl]pyridazine-3-carboxamide | C49H55N7O6S | 869.39 |
| mixED 15 | A2A043 | N-(1-Adamantylmethylsulfonyl)-6-[4-[[2-[2-[6-[3-(dimethylcarbamoyl)-4-hydroxynaphthalen-1-yl]pyridin-3-yl]ethynyl]phenyl]methyl]piperazin-1-yl]pyridazine-3-carboxamide | C47H49N7O5S | 823.35 |
| | A2A044 | N-(1-Adamantylmethylsulfonyl)-6-[4-[3-[2-[6-[3-(dimethylcarbamoyl)-4-hydroxynaphthalen-1-yl]pyridin-3-yl]ethynyl]-5-(trifluoromethyl)benzoyl]piperazin-1-yl]pyridazine-3-carboxamide | C48H46F3N7O6S | 905.32 |
| | A2A045 | N-(1-Adamantylmethylsulfonyl)-6-[4-[5-[2-[6-[3-(dimethylcarbamoyl)-4-hydroxynaphthalen-1-yl]pyridin-3-yl]ethynyl]pyridine-3-carbonyl]piperazin-1-yl]pyridazine-3-carboxamide | C46H46N8O6S | 838.33 |
| | A2A046 | N-(1-Adamantylmethylsulfonyl)-6-[4-[3-[4-[6-[3-(dimethylcarbamoyl)-4-hydroxynaphthalen-1-yl]pyridin-3-yl]pyrazol-1-yl]-5-(trifluoromethyl)benzoyl]piperazin-1-yl]pyridazine-3-carboxamide | C49H48F3N9O6S | 947.34 |
| | A2A047 | 4-[4-[2-[[4-[4-(1-Adamantylmethylsulfonylcarbamoyl)phenyl]piperazin-1-yl]methyl]phenyl]phenyl]-1-hydroxy-N,N-dimethylnaphthalene-2-carboxamide | C48H52N4O5S | 796.37 |
| | A2A048 | 4-[4-[2-[3-[4-[4-(1-Adamantylmethylsulfonylcarbamoyl)phenyl]piperazin-1-yl]-3-oxopropyl]phenyl]phenyl]-1-hydroxy-N,N-dimethylnaphthalene-2-carboxamide | C50H54N4O6S | 838.38 |
| | A2A049 | 4-[4-[4-[1-[4-[4-(1-Adamantylmethylsulfonylcarbamoyl)phenyl]piperazin-1-yl]ethyl]phenyl]phenyl]-1-hydroxy-N,N-dimethylnaphthalene-2-carboxamide | C49H54N4O5S | 810.38 |
| | A2A005 | N-(1-Adamantylmethylsulfonyl)-6-[4-[[4-[4-[3-(dimethylcarbamoyl)-4-hydroxynaphthalen-1-yl]phenyl]-2-ethylphenyl]methyl]piperazin-1-yl]pyridazine-3-carboxamide | C48H54N6O5S | 826.39 |
| | A2A050 | 4-[4-[4-[[4-[4-(1-Adamantylmethylsulfonylcarbamoyl)phenyl]piperazin-1-yl]methyl]-2-methylphenyl]phenyl]-1-hydroxy-N,N-dimethylnaphthalene-2-carboxamide | C49H54N4O5S | 810.38 |
| | A2A051 | 4-[4-[4-[4-[4-(1-Adamantylmethylsulfonylcarbamoyl)phenyl]piperazine-1-carbonyl]-2-methylphenyl]phenyl]-1-hydroxy-N,N-dimethylnaphthalene-2-carboxamide | C49H52N4O6S | 824.36 |
| | A2A052 | 4-[4-[4-[[4-[4-(1-Adamantylmethylsulfonylcarbamoyl)phenyl]piperazin-1-yl]methyl]-3-ethylphenyl]phenyl]-1-hydroxy-N,N-dimethylnaphthalene-2-carboxamide | C50H56N4O5S | 824.4 |
| | A2A053 | 4-[4-[4-[[4-[4-(1-Adamantylmethylsulfonylcarbamoyl)phenyl]piperazin-1-yl]methyl]-2-fluorophenyl]phenyl]-1-hydroxy-N,N-dimethylnaphthalene-2-carboxamide | C48H51FN4O5S | 814.36 |
| | A2A054 | 4-[4-[4-[4-[4-(1-Adamantylmethylsulfonylcarbamoyl)phenyl]piperazine-1-carbonyl]-2-fluorophenyl]phenyl]-1-hydroxy-N,N-dimethylnaphthalene-2-carboxamide | C48H49FN4O6S | 828.34 |
| | A2A055 | 4-[4-[4-[[4-[4-(1-Adamantylmethylsulfonylcarbamoyl)phenyl]piperazin-1-yl]methyl]-3-methoxyphenyl]phenyl]-1-hydroxy-N,N-dimethylnaphthalene-2-carboxamide | C49H54N4O6S | 826.38 |
| | A2A056 | 4-[4-[4-[[4-[4-(1-Adamantylmethylsulfonylcarbamoyl)phenyl]piperazin-1-yl]methyl]-3,5-dimethoxyphenyl]phenyl]-1-hydroxy-N,N-dimethylnaphthalene-2-carboxamide | C50H56N4O7S | 856.39 |
| | A2A057 | 4-[4-[4-[[4-[4-(1-Adamantylmethylsulfonylcarbamoyl)phenyl]piperazin-1-yl]methyl]naphthalen-1-yl]phenyl]-1-hydroxy-N,N-dimethylnaphthalene-2-carboxamide | C52H54N4O5S | 846.38 |
| | A2A058 | 4-[4-[4-[4-[4-(1-Adamantylmethylsulfonylcarbamoyl)phenyl]piperazine-1-carbonyl]naphthalen-1-yl]phenyl]-1-hydroxy-N,N-dimethylnaphthalene-2-carboxamide | C52H52N4O6S | 860.36 |
| | A2A059 | 4-[4-[5-[[4-[4-(1-Adamantylmethylsulfonylcarbamoyl)phenyl]piperazin-1-yl]methyl]thiophen-3-yl]phenyl]-1-hydroxy-N,N-dimethylnaphthalene-2-carboxamide | C46H50N4O5S2 | 802.32 |
| | A2A006 | N-(1-Adamantylmethylsulfonyl)-6-[4-[[4-[4-[3-(dimethylcarbamoyl)-4-hydroxynaphthalen-1-yl]phenyl]-3-fluorophenyl]methyl]piperazin-1-yl]pyridazine-3-carboxamide | C46H49FN6O5S | 816.35 |
| | A2A060 | 4-[4-[5-[4-[4-(1-Adamantylmethylsulfonylcarbamoyl)phenyl]piperazine-1-carbonyl]thiophen-3-yl]phenyl]-1-hydroxy-N,N-dimethylnaphthalene-2-carboxamide | C46H48N4O6S2 | 816.3 |

| | | | | |
|---|---|---|---|---|
| | A2A061 | 4-[4-[3-[[4-[4-(1-Adamantylmethylsulfonylcarbamoyl)phenyl]piperazin-1-yl]methyl]-5-(trifluoromethyl)phenyl]phenyl]-1-hydroxy-N,N-dimethylnaphthalene-2-carboxamide | C49H51F3N4O5S | 864.35 |
| | A2A062 | 4-[4-[3-[4-[4-(1-Adamantylmethylsulfonylcarbamoyl)phenyl]piperazine-1-carbonyl]-5-(trifluoromethyl)phenyl]phenyl]-1-hydroxy-N,N-dimethylnaphthalene-2-carboxamide | C49H49F3N4O6S | 878.33 |
| | A2A063 | 4-[4-[3-[[4-[4-(1-Adamantylmethylsulfonylcarbamoyl)phenyl]piperazin-1-yl]methyl]-5-(trifluoromethoxy)phenyl]phenyl]-1-hydroxy-N,N-dimethylnaphthalene-2-carboxamide | C49H51F3N4O6S | 880.35 |
| | A2A064 | 4-[4-[3-[[4-[4-(1-Adamantylmethylsulfonylcarbamoyl)phenyl]piperazin-1-yl]methyl]-5-ethoxyphenyl]phenyl]-1-hydroxy-N,N-dimethylnaphthalene-2-carboxamide | C50H56N4O6S | 840.39 |
| | A2A065 | 4-[4-[3-[[4-[4-(1-Adamantylmethylsulfonylcarbamoyl)phenyl]piperazin-1-yl]methyl]-5-propan-2-yloxyphenyl]phenyl]-1-hydroxy-N,N-dimethylnaphthalene-2-carboxamide | C51H58N4O6S | 854.41 |
| | A2A066 | 4-[4-[3-[4-[4-(1-Adamantylmethylsulfonylcarbamoyl)phenyl]piperazine-1-carbonyl]-5-tert-butylphenyl]phenyl]-1-hydroxy-N,N-dimethylnaphthalene-2-carboxamide | C52H58N4O6S | 866.41 |
| mixED 15 | A2A067 | 4-[4-[4-[1-[4-[4-(1-Adamantylmethylsulfonylcarbamoyl)phenyl]piperazin-1-yl]ethyl]-3-fluorophenyl]phenyl]-1-hydroxy-N,N-dimethylnaphthalene-2-carboxamide | C49H53FN4O5S | 828.37 |
| | A2A068 | 4-[4-[2-[2-[[4-[4-(1-Adamantylmethylsulfonylcarbamoyl)phenyl]piperazin-1-yl]methyl]phenyl]ethynyl]phenyl]-1-hydroxy-N,N-dimethylnaphthalene-2-carboxamide | C50H52N4O5S | 820.37 |
| | A2A069 | 4-[4-[2-[3-[4-[4-(1-Adamantylmethylsulfonylcarbamoyl)phenyl]piperazine-1-carbonyl]-5-(trifluoromethyl)phenyl]ethynyl]phenyl]-1-hydroxy-N,N-dimethylnaphthalene-2-carboxamide | C51H49F3N4O6S | 902.33 |

[Table 148]

| | | | | |
|---|---|---|---|---|
| mixED 15 | A2A007 | N-(1-Adamantylmethylsulfonyl)-6-[4-[4-[4-[3-(dimethylcarbamoyl)-4-hydroxynaphthalen-1-yl]phenyl]-3-fluorobenzoyl]piperazin-1-yl]pyridazine-3-carboxamide | C46H47FN6O6S | 830.33 |
| | A2A070 | 4-[4-[2-[5-[4-[4-(1-Adamantylmethylsulfonylcarbamoyl)phenyl]piperazine-1-carbonyl]pyridin-3-yl]ethynyl]phenyl]-1-hydroxy-N,N-dimethylnaphthalene-2-carboxamide | C49H49N5O6S | 835.34 |
| | A2A071 | 4-[4-[1-[3-[4-[4-(1-Adamantylmethylsulfonylcarbamoyl)phenyl]piperazine-1-carbonyl]-5-(trifluoromethyl)phenyl]pyrazol-4-yl]phenyl]-1-hydroxy-N,N-dimethylnaphthalene-2-carboxamide | C52H51F3N6O6S | 944.35 |
| | A2A072 | 4-[5-[2-[[4-[4-(1-Adamantylmethylsulfonylcarbamoyl)phenyl]piperazin-1-yl]methyl]phenyl]pyridin-2-yl]-1-hydroxy-N,N-dimethylnaphthalene-2-carboxamide | C47H51N5O5S | 797.36 |
| | A2A073 | 4-[5-[2-[3-[4-[4-(1-Adamantylmethylsulfonylcarbamoyl)phenyl]piperazin-1-yl]-3-oxopropyl]phenyl]pyridin-2-yl]-1-hydroxy-N,N-dimethylnaphthalene-2-carboxamide | C49H53N5O6S | 839.37 |
| | A2A074 | 4-[5-[4-[1-[4-[4-(1-Adamantylmethylsulfonylcarbamoyl)phenyl]piperazin-1-yl]ethyl]phenyl]pyridin-2-yl]-1-hydroxy-N,N-dimethylnaphthalene-2-carboxamide | C48H53N5O5S | 811.38 |
| | A2A075 | 4-[5-[4-[[4-[4-(1-Adamantylmethylsulfonylcarbamoyl)phenyl]piperazin-1-yl]methyl]-2-methylphenyl]pyridin-2-yl]-1-hydroxy-N,N-dimethylnaphthalene-2-carboxamide | C48H53N5O5S | 811.38 |
| | A2A076 | 4-[5-[4-[4-[4-(1-Adamantylmethylsulfonylcarbamoyl)phenyl]piperazine-1-carbonyl]-2-methylphenyl]pyridin-2-yl]-1-hydroxy-N,N-dimethylnaphthalene-2-carboxamide | C48H51N5O6S | 825.36 |
| | A2A077 | 4-[5-[4-[[4-[4-(1-Adamantylmethylsulfonylcarbamoyl)phenyl]piperazin-1-yl]methyl]-3-ethylphenyl]pyridin-2-yl]-1-hydroxy-N,N-dimethylnaphthalene-2-carboxamide | C49H55N5O5S | 825.39 |
| | A2A078 | 4-[5-[4-[[4-[4-(1-Adamantylmethylsulfonylcarbamoyl)phenyl]piperazin-1-yl]methyl]-2-fluorophenyl]pyridin-2-yl]-1-hydroxy-N,N-dimethylnaphthalene-2-carboxamide | C47H50FN5O5S | 815.35 |
| | A2A079 | 4-[5-[4-[4-[4-(1-Adamantylmethylsulfonylcarbamoyl)phenyl]piperazine-1-carbonyl]-2-fluorophenyl]pyridin-2-yl]-1-hydroxy-N,N-dimethylnaphthalene-2-carboxamide | C47H48FN5O6S | 829.33 |

| | | | | |
|---|---|---|---|---|
| | A2A008 | N-(1-Adamantylmethylsulfonyl)-6-[4-[[4-[4-[3-(dimethylcarbamoyl)-4-hydroxynaphthalen-1-yl]phenyl]-2-methoxyphenyl]methyl]piperazin-1-yl]pyridazine-3-carboxamide | C47H52N6O6S | 828.37 |
| | A2A080 | 4-[5-[4-[[4-[4-(1-Adamantylmethylsulfonylcarbamoyl)phenyl]piperazin-1-yl]methyl]-3-methoxyphenyl]pyridin-2-yl]-1-hydroxy-N,N-dimethylnaphthalene-2-carboxamide | C48H53N5O6S | 827.37 |
| | A2A081 | 4-[5-[4-[[4-[4-(1-Adamantylmethylsulfonylcarbamoyl)phenyl]piperazin-1-yl]methyl]-3,5-dimethoxyphenyl]pyridin-2-yl]-1-hydroxy-N,N-dimethylnaphthalene-2-carboxamide | C49H55N5O7S | 857.38 |
| | A2A082 | 4-[5-[4-[[4-[4-(1-Adamantylmethylsulfonylcarbamoyl)phenyl]piperazin-1-yl]methyl]naphthalen-1-yl]pyridin-2-yl]-1-hydroxy-N,N-dimethylnaphthalene-2-carboxamide | C51H53N5O5S | 847.38 |
| | A2A083 | 4-[5-[4-[4-[4-(1-Adamantylmethylsulfonylcarbamoyl)phenyl]piperazine-1-carbonyl]naphthalen-1-yl]pyridin-2-yl]-1-hydroxy-N,N-dimethylnaphthalene-2-carboxamide | C51H51N5O6S | 861.36 |
| | A2A084 | 4-[5-[5-[[4-[4-(1-Adamantylmethylsulfonylcarbamoyl)phenyl]piperazin-1-yl]methyl]thiophen-3-yl]pyridin-2-yl]-1-hydroxy-N,N-dimethylnaphthalene-2-carboxamide | C45H49N5O5S2 | 803.32 |
| | A2A085 | 4-[5-[5-[4-[4-(1-Adamantylmethylsulfonylcarbamoyl)phenyl]piperazine-1-carbonyl]thiophen-3-yl]pyridin-2-yl]-1-hydroxy-N,N-dimethylnaphthalene-2-carboxamide | C45H47N5O6S2 | 817.3 |
| | A2A086 | 4-[5-[3-[[4-[4-(1-Adamantylmethylsulfonylcarbamoyl)phenyl]piperazin-1-yl]methyl]-5-(trifluoromethyl)phenyl]pyridin-2-yl]-1-hydroxy-N,N-dimethylnaphthalene-2-carboxamide | C48H50F3N5O5S | 865.35 |
| | A2A087 | 4-[5-[3-[4-[4-(1-Adamantylmethylsulfonylcarbamoyl)phenyl]piperazine-1-carbonyl]-5-(trifluoromethyl)phenyl]pyridin-2-yl]-1-hydroxy-N,N-dimethylnaphthalene-2-carboxamide | C48H48F3N5O6S | 879.33 |
| mixED 15 | A2A088 | 4-[5-[3-[[4-[4-(1-Adamantylmethylsulfonylcarbamoyl)phenyl]piperazin-1-yl]methyl]-5-(trifluoromethoxy)phenyl]pyridin-2-yl]-1-hydroxy-N,N-dimethylnaphthalene-2-carboxamide | C48H50F3N5O6S | 881.34 |
| | A2A089 | 4-[5-[3-[[4-[4-(1-Adamantylmethylsulfonylcarbamoyl)phenyl]piperazin-1-yl]methyl]-5-ethoxyphenyl]pyridin-2-yl]-1-hydroxy-N,N-dimethylnaphthalene-2-carboxamide | C49H55N5O6S | 841.39 |
| | A2A009 | N-(1-Adamantylmethylsulfonyl)-6-[4-[[4-[4-[3-(dimethylcarbamoyl)-4-hydroxynaphthalen-1-yl]phenyl]-2,6-dimethoxyphenyl]methyl]piperazin-1-yl]pyridazine-3-carboxamide | C48H54N6O7S | 858.38 |
| | A2A090 | 4-[5-[3-[[4-[4-(1-Adamantylmethylsulfonylcarbamoyl)phenyl]piperazin-1-yl]methyl]-5-propan-2-yloxyphenyl]pyridin-2-yl]-1-hydroxy-N,N-dimethylnaphthalene-2-carboxamide | C50H57N5O6S | 855.4 |
| | A2A091 | 4-[5-[3-[4-[4-(1-Adamantylmethylsulfonylcarbamoyl)phenyl]piperazine-1-carbonyl]-5-tert-butylphenyl]pyridin-2-yl]-1-hydroxy-N,N-dimethylnaphthalene-2-carboxamide | C51H57N5O6S | 867.4 |
| | A2A092 | 4-[5-[4-[1-[4-[4-(1-Adamantylmethylsulfonylcarbamoyl)phenyl]piperazin-1-yl]ethyl]-3-fluorophenyl]pyridin-2-yl]-1-hydroxy-N,N-dimethylnaphthalene-2-carboxamide | C48H52FN5O5S | 829.37 |
| | A2A093 | 4-[5-[2-[2-[[4-[4-(1-Adamantylmethylsulfonylcarbamoyl)phenyl]piperazin-1-yl]methyl]phenyl]ethynyl]pyridin-2-yl]-1-hydroxy-N,N-dimethylnaphthalene-2-carboxamide | C49H51N5O5S | 821.36 |
| | A2A094 | 4-[5-[2-[3-[4-[4-(1-Adamantylmethylsulfonylcarbamoyl)phenyl]piperazine-1-carbonyl]-5-(trifluoromethyl)phenyl]ethynyl]pyridin-2-yl]-1-hydroxy-N,N-dimethylnaphthalene-2-carboxamide | C50H48F3N5O6S | 903.33 |
| | A2A095 | 4-[5-[2-[5-[4-[4-(1-Adamantylmethylsulfonylcarbamoyl)phenyl]piperazine-1-carbonyl]pyridin-3-yl]ethynyl]pyridin-2-yl]-1-hydroxy-N,N-dimethylnaphthalene-2-carboxamide | C48H48N6O6S | 836.34 |
| | A2A096 | 4-[5-[1-[3-[4-[4-(1-Adamantylmethylsulfonylcarbamoyl)phenyl]piperazine-1-carbonyl]-5-(trifluoromethyl)phenyl]pyrazol-4-yl]pyridin-2-yl]-1-hydroxy-N,N-dimethylnaphthalene-2-carboxamide | C51H50F3N7O6S | 945.35 |
| | A2A097 | N-(1-Adamantylmethylsulfonyl)-6-[4-[2-[2-[4-[3-(dimethylcarbamoyl)-4-hydroxynaphthalen-1-yl]phenyl]ethynyl]benzoyl]piperazin-1-yl]pyridazine-3-carboxamide | C48H48N6O6S | 836.336 |
| | A2A098 | N-(1-Adamantylmethylsulfonyl)-6-[4-[2-[2-[6-[3-(dimethylcarbamoyl)-4-hydroxynaphthalen-1-yl]pyridin-3-yl]ethynyl]benzoyl]piperazin-1-yl]pyridazine-3-carboxamide | C47H47N7O6S | 837.331 |

| | | | | |
|---|---|---|---|---|
| | A2A099 | 4-[4-[2-[2-[4-[4-(1-Adamantylmethylsulfonylcarbamoyl)phenyl]piperazine-1-carbonyl]phenyl]ethynyl]phenyl]-1-hydroxy-N,N-dimethylnaphthalene-2-carboxamide | C50H50N4O6S | 834.345 |
| | A2A100 | 4-[5-[2-[2-[4-[4-(1-Adamantylmethylsulfonylcarbamoyl)phenyl]piperazine-1-carbonyl]phenyl]ethynyl]pyridin-2-yl]-1-hydroxy-N,N-dimethylnaphthalene-2-carboxamide | C49H49N5O6S | 835.34 |

[Table 149]

[Table EE12]

Table EE12

| Mixture No. | No. of compound that may be contained | Compound name | Compositional formula | Exact MS |
|---|---|---|---|---|
| mixED 16 | A2Q001 | 6-[4-[[2-[4-[3-(Dimethylcarbamoyl)-4-hydroxynaphthalen-1-yl]phenyl]phenyl]methyl]piperazin-1-yl]-N-[3-nitro-4-(2-phenylsulfanylethylamino)phenyl]sulfonylpyridazine-3-carboxamide | C49H46N8O7S2 | 922.29 |
| | A2Q010 | 6-[4-[[4-[4-[3-(Dimethylcarbamoyl)-4-hydroxynaphthalen-1-yl]phenyl]naphthalen-1-yl]methyl]piperazin-1-yl]-N-[3-nitro-4-(2-phenylsulfanylethylamino)phenyl]sulfonylpyridazine-3-carboxamide | C53H48N8O7S2 | 972.31 |
| | A2Q011 | 6-[4-[4-[4-[3-(Dimethylcarbamoyl)-4-hydroxynaphthalen-1-yl]phenyl]naphthalene-1-carbonyl]piperazin-1-yl]-N-[3-nitro-4-(2-phenylsulfanylethylamino)phenyl]sulfonylpyridazine-3-carboxamide | C53H46N8O8S2 | 986.29 |
| | A2Q012 | 6-[4-[[4-[4-[3-(Dimethylcarbamoyl)-4-hydroxynaphthalen-1-yl]phenyl]thiophen-2-yl]methyl]piperazin-1-yl]-N-[3-nitro-4-(2-phenylsulfanylethylamino)phenyl]sulfonylpyridazine-3-carboxamide | C47H44N8O7S3 | 928.25 |
| | A2Q013 | 6-[4-[4-[4-[3-(Dimethylcarbamoyl)-4-hydroxynaphthalen-1-yl]phenyl]thiophene-2-carbonyl]piperazin-1-yl]-N-[3-nitro-4-(2-phenylsulfanylethylamino)phenyl]sulfonylpyridazine-3-carboxamide | C47H42N8O8S3 | 942.23 |
| mixED 16 | A2Q014 | 6-[4-[[3-[4-[3-(Dimethylcarbamoyl)-4-hydroxynaphthalen-1-yl]phenyl]-5-(trifluoromethyl)phenyl]methyl]piperazin-1-yl]-N-[3-nitro-4-(2-phenylsulfanylethylamino)phenyl]sulfonylpyridazine-3-carboxamide | C50H45F3N8O7S2 | 990.28 |
| | A2Q015 | 6-[4-[3-[4-[3-(Dimethylcarbamoyl)-4-hydroxynaphthalen-1-yl]phenyl]-5-(trifluoromethyl)benzoyl]piperazin-1-yl]-N-[3-nitro-4-(2-phenylsulfanylethylamino)phenyl]sulfonylpyridazine-3-carboxamide | C50H43F3N8O8S2 | 1004.26 |
| | A2Q016 | 6-[4-[[3-[4-[3-(Dimethylcarbamoyl)-4-hydroxynaphthalen-1-yl]phenyl]-5-(trifluoromethoxy)phenyl]methyl]piperazin-1-yl]-N-[3-nitro-4-(2-phenylsulfanylethylamino)phenyl]sulfonylpyridazine-3-carboxamide | C50H45F3N8O8S2 | 1006.28 |
| | A2Q017 | 6-[4-[[3-[4-[3-(Dimethylcarbamoyl)-4-hydroxynaphthalen-1-yl]phenyl]-5-ethoxyphenyl]methyl]piperazin-1-yl]-N-[3-nitro-4-(2-phenylsulfanylethylamino)phenyl]sulfonylpyridazine-3-carboxamide | C51H50N8O8S2 | 966.32 |
| | A2Q018 | 6-[4-[[3-[4-[3-(Dimethylcarbamoyl)-4-hydroxynaphthalen-1-yl]phenyl]-5-propan-2-yloxyphenyl]methyl]piperazin-1-yl]-N-[3-nitro-4-(2-phenylsulfanylethylamino)phenyl]sulfonylpyridazine-3-carboxamide | C52H52N8O8S2 | 980.33 |
| | A2Q019 | 6-[4-[3-tert-Butyl-5-[4-[3-(dimethylcarbamoyl)-4-hydroxynaphthalen-1-yl]phenyl]benzoyl]piperazin-1-yl]-N-[3-nitro-4-(2-phenylsulfanylethylamino)phenyl]sulfonylpyridazine-3-carboxamide | C53H52N8O8S2 | 992.33 |
| | A2Q002 | 6-[4-[3-[2-[4-[3-(Dimethylcarbamoyl)-4-hydroxynaphthalen-1-yl]phenyl]phenyl]propanoyl]piperazin-1-yl]-N-[3-nitro-4-(2-phenylsulfanylethylamino)phenyl]sulfonylpyridazine-3-carboxamide | C51H48N8O8S2 | 964.3 |
| | A2Q020 | 6-[4-[[2-[2-[4-[3-(Dimethylcarbamoyl)-4-hydroxynaphthalen-1-yl]phenyl]ethynyl]phenyl]methyl]piperazin-1-yl]-N-[3-nitro-4-(2-phenylsulfanylethylamino)phenyl]sulfonylpyridazine-3-carboxamide | C51H46N8O7S2 | 946.29 |
| | A2Q021 | 6-[4-[3-[2-[4-[3-(Dimethylcarbamoyl)-4-hydroxynaphthalen-1-yl]phenyl]ethynyl]-5-(trifluoromethyl)benzoyl]piperazin-1-yl]-N-[3-nitro-4-(2-phenylsulfanylethylamino)phenyl]sulfonylpyridazine-3-carboxamide | C52H43F3N8O8S2 | 1028.26 |
| | A2Q022 | 6-[4-[5-[2-[4-[3-(Dimethylcarbamoyl)-4-hydroxynaphthalen-1-yl]phenyl]ethynyl]pyridine-3-carbonyl]piperazin-1-yl]-N-[3-nitro-4-(2-phenylsulfanylethylamino)phenyl]sulfonylpyridazine-3-carboxamide | C50H43N9O8S2 | 961.27 |

| | | | | |
|---|---|---|---|---|
| | A2Q023 | 6-[4-[3-[4-[4-[3-(Dimethylcarbamoyl)-4-hydroxynaphthalen-1-yl]phenyl]pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide | C53H45F3N10O8S2 | 1070.28 |
| | A2Q024 | 6-[4-[[2-[6-[3-(Dimethylcarbamoyl)-4-hydroxynaphthalen-1-yl]pyridin-3-yl]phenyl]methyl]piperazin-1-yl]-N-[3-nitro-4-(2-phenylsulfanylethylamino)phenyl]sulfonylpyridazine-3-carboxamide | C48H45N9O7S2 | 923.29 |
| | A2Q025 | 6-[4-[3-[2-[6-[3-(Dimethylcarbamoyl)-4-hydroxynaphthalen-1-yl]pyridin-3-yl]phenyl]propanoyl]piperazin-1-yl]-N-[3-nitro-4-(2-phenylsulfanylethylamino)phenyl]sulfonylpyridazine-3-carboxamide | C50H47N9O8S2 | 965.3 |
| | A2Q026 | 6-[4-[[4-[6-[3-(Dimethylcarbamoyl)-4-hydroxynaphthalen-1-yl]pyridin-3-yl]-3-methylphenyl]methyl]piperazin-1-yl]-N-[3-nitro-4-(2-phenylsulfanylethylamino)phenyl]sulfonylpyridazine-3-carboxamide | C49H47N9O7S2 | 937.3 |
| | A2Q027 | 6-[4-[4-[6-[3-(Dimethylcarbamoyl)-4-hydroxynaphthalen-1-yl]pyridin-3-yl]-3-methylbenzoyl]piperazin-1-yl]-N-[3-nitro-4-(2-phenylsulfanylethylamino)phenyl]sulfonylpyridazine-3-carboxamide | C49H45N9O8S2 | 951.28 |
| | A2Q028 | 6-[4-[[4-[6-[3-(Dimethylcarbamoyl)-4-hydroxynaphthalen-1-yl]pyridin-3-yl]-2-ethylphenyl]methyl]piperazin-1-yl]-N-[3-nitro-4-(2-phenylsulfanylethylamino)phenyl]sulfonylpyridazine-3-carboxamide | C50H49N9O7S2 | 951.32 |
| | A2Q029 | 6-[4-[[4-[6-[3-(Dimethylcarbamoyl)-4-hydroxynaphthalen-1-yl]pyridin-3-yl]-3-fluorophenyl]methyl]piperazin-1-yl]-N-[3-nitro-4-(2-phenylsulfanylethylamino)phenyl]sulfonylpyridazine-3-carboxamide | C48H44FN9O7S2 | 941.28 |
| | A2Q003 | 6-[4-[[4-[4-[3-(Dimethylcarbamoyl)-4-hydroxynaphthalen-1-yl]phenyl]-3-methylphenyl]methyl]piperazin-1-yl]-N-[3-nitro-4-(2-phenylsulfanylethylamino)phenyl]sulfonylpyridazine-3-carboxamide | C50H48N8O7S2 | 936.31 |
| | A2Q030 | 6-[4-[4-[6-[3-(Dimethylcarbamoyl)-4-hydroxynaphthalen-1-yl]pyridin-3-yl]-3-fluorobenzoyl]piperazin-1-yl]-N-[3-nitro-4-(2-phenylsulfanylethylamino)phenyl]sulfonylpyridazine-3-carboxamide | C48H42FN9O8S2 | 955.26 |
| | A2Q031 | 6-[4-[[4-[6-[3-(Dimethylcarbamoyl)-4-hydroxynaphthalen-1-yl]pyridin-3-yl]-2-methoxyphenyl]methyl]piperazin-1-yl]-N-[3-nitro-4-(2-phenylsulfanylethylamino)phenyl]sulfonylpyridazine-3-carboxamide | C49H47N9O8S2 | 953.3 |

[Table 150]

| | | | | |
|---|---|---|---|---|
| mixED 16 | A2Q032 | 6-[4-[[4-[6-[3-(Dimethylcarbamoyl)-4-hydroxynaphthalen-1-yl]pyridin-3-yl]-2,6-dimethoxyphenyl]methyl]piperazin-1-yl]-N-[3-nitro-4-(2-phenylsulfanylethylamino)phenyl]sulfonylpyridazine-3-carboxamide | C50H49N9O9S2 | 983.31 |
| | A2Q033 | 6-[4-[[4-[6-[3-(Dimethylcarbamoyl)-4-hydroxynaphthalen-1-yl]pyridin-3-yl]naphthalen-1-yl]methyl]piperazin-1-yl]-N-[3-nitro-4-(2-phenylsulfanylethylamino)phenyl]sulfonylpyridazine-3-carboxamide | C52H47N9O7S2 | 973.3 |
| | A2Q034 | 6-[4-[4-[6-[3-(Dimethylcarbamoyl)-4-hydroxynaphthalen-1-yl]pyridin-3-yl]naphthalene-1-carbonyl]piperazin-1-yl]-N-[3-nitro-4-(2-phenylsulfanylethylamino)phenyl]sulfonylpyridazine-3-carboxamide | C52H45N9O8S2 | 987.28 |
| | A2Q035 | 6-[4-[[4-[6-[3-(Dimethylcarbamoyl)-4-hydroxynaphthalen-1-yl]pyridin-3-yl]thiophen-2-yl]methyl]piperazin-1-yl]-N-[3-nitro-4-(2-phenylsulfanylethylamino)phenyl]sulfonylpyridazine-3-carboxamide | C46H43N9O7S3 | 929.24 |
| | A2Q036 | 6-[4-[4-[6-[3-(Dimethylcarbamoyl)-4-hydroxynaphthalen-1-yl]pyridin-3-yl]thiophene-2-carbonyl]piperazin-1-yl]-N-[3-nitro-4-(2-phenylsulfanylethylamino)phenyl]sulfonylpyridazine-3-carboxamide | C46H41N9O8S3 | 943.22 |
| | A2Q037 | 6-[4-[[3-[6-[3-(Dimethylcarbamoyl)-4-hydroxynaphthalen-1-yl]pyridin-3-yl]-5-(trifluoromethyl)phenyl]methyl]piperazin-1-yl]-N-[3-nitro-4-(2-phenylsulfanylethylamino)phenyl]sulfonylpyridazine-3-carboxamide | C49H44F3N9O7S2 | 991.28 |
| | A2Q038 | 6-[4-[3-[6-[3-(Dimethylcarbamoyl)-4-hydroxynaphthalen-1-yl]pyridin-3-yl]-5-(trifluoromethyl)benzoyl]piperazin-1-yl]-N-[3-nitro-4-(2-phenylsulfanylethylamino)phenyl]sulfonylpyridazine-3-carboxamide | C49H42F3N9O8S2 | 1005.25 |
| | A2Q039 | 6-[4-[[3-[6-[3-(Dimethylcarbamoyl)-4-hydroxynaphthalen-1-yl]pyridin-3-yl]-5-(trifluoromethoxy)phenyl]methyl]piperazin-1-yl]-N-[3-nitro-4-(2-phenylsulfanylethylamino)phenyl]sulfonylpyridazine-3-carboxamide | C49H44F3N9O8S2 | 1007.27 |
| | A2Q004 | 6-[4-[4-[4-[3-(Dimethylcarbamoyl)-4-hydroxynaphthalen-1-yl]phenyl]-3-methylbenzoyl]piperazin-1-yl]-N-[3-nitro-4-(2-phenylsulfanylethylamino)phenyl]sulfonylpyridazine-3-carboxamide | C50H46N8O8S2 | 950.29 |
| | A2Q040 | 6-[4-[[3-[6-[3-(Dimethylcarbamoyl)-4-hydroxynaphthalen-1-yl]pyridin-3-yl]-5-ethoxyphenyl]methyl]piperazin-1-yl]-N-[3-nitro-4-(2-phenylsulfanylethylamino)phenyl]sulfonylpyridazine-3-carboxamide | C50H49N9O8S2 | 967.31 |

| | | | | |
|---|---|---|---|---|
| | A2Q041 | 6-[4-[[3-[6-[3-(Dimethylcarbamoyl)-4-hydroxynaphthalen-1-yl]pyridin-3-yl]-5-propan-2-yloxyphenyl]methyl]piperazin-1-yl]-N-[3-nitro-4-(2-phenylsulfanylethylamino)phenyl]sulfonylpyridazine-3-carboxamide | C51H51N9O8S2 | 981.33 |
| | A2Q042 | 6-[4-[3-tert-Butyl-5-[6-[3-(dimethylcarbamoyl)-4-hydroxynaphthalen-1-yl]pyridin-3-yl]benzoyl]piperazin-1-yl]-N-[3-nitro-4-(2-phenylsulfanylethylamino)phenyl]sulfonylpyridazine-3-carboxamide | C52H51N9O8S2 | 993.33 |
| | A2Q043 | 6-[4-[[2-[2-[6-[3-(Dimethylcarbamoyl)-4-hydroxynaphthalen-1-yl]pyridin-3-yl]ethynyl]phenyl]methyl]piperazin-1-yl]-N-[3-nitro-4-(2-phenylsulfanylethylamino)phenyl]sulfonylpyridazine-3-carboxamide | C50H45N9O7S2 | 947.29 |
| | A2Q044 | 6-[4-[3-[2-[6-[3-(Dimethylcarbamoyl)-4-hydroxynaphthalen-1-yl]pyridin-3-yl]ethynyl]-5-(trifluoromethyl)benzoyl]piperazin-1-yl]-N-[3-nitro-4-(2-phenylsulfanylethylamino)phenyl]sulfonylpyridazine-3-carboxamide | C51H42F3N9O8S2 | 1029.25 |
| | A2Q045 | 6-[4-[5-[2-[6-[3-(Dimethylcarbamoyl)-4-hydroxynaphthalen-1-yl]pyridin-3-yl]ethynyl]pyridine-3-carbonyl]piperazin-1-yl]-N-[3-nitro-4-(2-phenylsulfanylethylamino)phenyl]sulfonylpyridazine-3-carboxamide | C49H42N10O8S2 | 962.26 |
| | A2Q046 | 6-[4-[3-[4-[6-[3-(Dimethylcarbamoyl)-4-hydroxynaphthalen-1-yl]pyridin-3-yl]pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide | C52H44F3N11O8S2 | 1071.28 |
| | A2Q047 | 1-Hydroxy-N,N-dimethyl-4-[4-[2-[[4-[4-[[3-nitro-4-(2-phenylsulfanylethylamino)phenyl]sulfonylcarbamoyl]phenyl]piperazin-1-yl]methyl]phenyl]phenyl]naphthalene-2-carboxamide | C51H48N6O7S2 | 920.3 |
| | A2Q048 | 1-Hydroxy-N,N-dimethyl-4-[4-[2-[3-[4-[4-[[3-nitro-4-(2-phenylsulfanylethylamino)phenyl]sulfonylcarbamoyl]phenyl]piperazin-1-yl]-3-oxopropyl]phenyl]phenyl]naphthalene-2-carboxamide | C53H50N6O8S2 | 962.31 |
| | A2Q049 | 1-Hydroxy-N,N-dimethyl-4-[4-[4-[1-[4-[4-[[3-nitro-4-(2-phenylsulfanylethylamino)phenyl]sulfonylcarbamoyl]phenyl]piperazin-1-yl]ethyl]phenyl]phenyl]naphthalene-2-carboxamide | C52H50N6O7S2 | 934.32 |
| | A2Q005 | 6-[4-[[4-[4-[3-(Dimethylcarbamoyl)-4-hydroxynaphthalen-1-yl]phenyl]-2-ethylphenyl]methyl]piperazin-1-yl]-N-[3-nitro-4-(2-phenylsulfanylethylamino)phenyl]sulfonylpyridazine-3-carboxamide | C51H50N8O7S2 | 950.32 |
| mixED 16 | A2Q050 | 1-Hydroxy-N,N-dimethyl-4-[4-[2-methyl-4-[[4-[4-[[3-nitro-4-(2-phenylsulfanylethylamino)phenyl]sulfonylcarbamoyl]phenyl]piperazin-1-yl]methyl]phenyl]phenyl]naphthalene-2-carboxamide | C52H50N6O7S2 | 934.32 |
| | A2Q051 | 1-Hydroxy-N,N-dimethyl-4-[4-[2-methyl-4-[4-[4-[[3-nitro-4-(2-phenylsulfanylethylamino)phenyl]sulfonylcarbamoyl]phenyl]piperazine-1-carbonyl]phenyl]phenyl]naphthalene-2-carboxamide | C52H48N6O8S2 | 948.3 |
| | A2Q052 | 4-[4-[3-Ethyl-4-[[4-[4-[[3-nitro-4-(2-phenylsulfanylethylamino)phenyl]sulfonylcarbamoyl]phenyl]piperazin-1-yl]methyl]phenyl]phenyl]-1-hydroxy-N,N-dimethylnaphthalene-2-carboxamide | C53H52N6O7S2 | 948.33 |
| | A2Q053 | 4-[4-[2-Fluoro-4-[[4-[4-[[3-nitro-4-(2-phenylsulfanylethylamino)phenyl]sulfonylcarbamoyl]phenyl]piperazin-1-yl]methyl]phenyl]phenyl]-1-hydroxy-N,N-dimethylnaphthalene-2-carboxamide | C51H47FN6O7S2 | 938.29 |
| | A2Q054 | 4-[4-[2-Fluoro-4-[4-[4-[[3-nitro-4-(2-phenylsulfanylethylamino)phenyl]sulfonylcarbamoyl]phenyl]piperazine-1-carbonyl]phenyl]phenyl]-1-hydroxy-N,N-dimethylnaphthalene-2-carboxamide | C51H45FN6O8S2 | 952.27 |

[Table 151]

| | | | | |
|---|---|---|---|---|
| mixED 16 | A2Q055 | 1-Hydroxy-4-[4-[3-methoxy-4-[[4-[4-[[3-nitro-4-(2-phenylsulfanylethylamino)phenyl]sulfonylcarbamoyl]phenyl]piperazin-1-yl]methyl]phenyl]phenyl]-N,N-dimethylnaphthalene-2-carboxamide | C52H50N6O8S2 | 950.31 |
| | A2Q056 | 4-[4-[3,5-Dimethoxy-4-[[4-[4-[[3-nitro-4-(2-phenylsulfanylethylamino)phenyl]sulfonylcarbamoyl]phenyl]piperazin-1-yl]methyl]phenyl]phenyl]-1-hydroxy-N,N-dimethylnaphthalene-2-carboxamide | C53H52N6O9S2 | 980.32 |
| | A2Q057 | 1-Hydroxy-N,N-dimethyl-4-[4-[4-[[4-[4-[[3-nitro-4-(2-phenylsulfanylethylamino)phenyl]sulfonylcarbamoyl]phenyl]piperazin-1-yl]methyl]naphthalen-1-yl]phenyl]naphthalene-2-carboxamide | C55H50N6O7S2 | 970.32 |
| | A2Q058 | 1-Hydroxy-N,N-dimethyl-4-[4-[4-[4-[4-[[3-nitro-4-(2-phenylsulfanylethylamino)phenyl]sulfonylcarbamoyl]phenyl]piperazine-1-carbonyl]naphthalen-1-yl]phenyl]naphthalene-2-carboxamide | C55H48N6O8S2 | 984.3 |
| | A2Q059 | 1-Hydroxy-N,N-dimethyl-4-[4-[5-[[4-[4-[[3-nitro-4-(2-phenylsulfanylethylamino)phenyl]sulfonylcarbamoyl]phenyl]piperazin-1-yl]methyl]thiophen-3-yl]phenyl]naphthalene-2-carboxamide | C49H46N6O7S3 | 926.26 |

| | | | | |
|---|---|---|---|---|
| | A2Q006 | 6-[4-[[4-[4-[3-(Dimethylcarbamoyl)-4-hydroxynaphthalen-1-yl]phenyl]-3-fluorophenyl]methyl]piperazin-1-yl]-N-[3-nitro-4-(2-phenylsulfanylethylamino)phenyl]sulfonylpyridazine-3-carboxamide | C49H45FN8O7S2 | 940.28 |
| | A2Q060 | 1-Hydroxy-N,N-dimethyl-4-[4-[5-[4-[4-[[3-nitro-4-(2-phenylsulfanylethylamino)phenyl]sulfonylcarbamoyl]phenyl]piperazine-1-carbonyl]thiophen-3-yl]phenyl]naphthalene-2-carboxamide | C49H44N6O8S3 | 940.24 |
| | A2Q061 | 1-Hydroxy-N,N-dimethyl-4-[4-[3-[[4-[4-[[3-nitro-4-(2-phenylsulfanylethylamino)phenyl]sulfonylcarbamoyl]phenyl]piperazin-1-yl]methyl]-5-(trifluoromethyl)phenyl]phenyl]naphthalene-2-carboxamide | C52H47F3N6O7S2 | 988.29 |
| | A2Q062 | 1-Hydroxy-N,N-dimethyl-4-[4-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]phenyl]naphthalene-2-carboxamide | C52H45F3N6O8S2 | 1002.27 |
| | A2Q063 | 1-Hydroxy-N,N-dimethyl-4-[4-[3-[[4-[4-[[3-nitro-4-(2-phenylsulfanylethylamino)phenyl]sulfonylcarbamoyl]phenyl]piperazin-1-yl]methyl]-5-(trifluoromethoxy)phenyl]phenyl]naphthalene-2-carboxamide | C52H47F3N6O8S2 | 1004.28 |
| | A2Q064 | 4-[4-[3-Ethoxy-5-[[4-[4-[[3-nitro-4-(2-phenylsulfanylethylamino)phenyl]sulfonylcarbamoyl]phenyl]piperazin-1-yl]methyl]phenyl]phenyl]-1-hydroxy-N,N-dimethylnaphthalene-2-carboxamide | C53H52N6O8S2 | 964.33 |
| | A2Q065 | 1-Hydroxy-N,N-dimethyl-4-[4-[3-[[4-[4-[[3-nitro-4-(2-phenylsulfanylethylamino)phenyl]sulfonylcarbamoyl]phenyl]piperazin-1-yl]methyl]-5-propan-2-yloxyphenyl]phenyl]naphthalene-2-carboxamide | C54H54N6O8S2 | 978.34 |
| | A2Q066 | 4-[4-[3-tert-Butyl-5-[4-[4-[[3-nitro-4-(2-phenylsulfanylethylamino)phenyl]sulfonylcarbamoyl]phenyl]piperazine-1-carbonyl]phenyl]phenyl]-1-hydroxy-N,N-dimethylnaphthalene-2-carboxamide | C55H54N6O8S2 | 990.34 |
| | A2Q067 | 4-[4-[3-Fluoro-4-[1-[4-[4-[[3-nitro-4-(2-phenylsulfanylethylamino)phenyl]sulfonylcarbamoyl]phenyl]piperazin-1-yl]ethyl]phenyl]phenyl]-1-hydroxy-N,N-dimethylnaphthalene-2-carboxamide | C52H49FN6O7S2 | 952.31 |
| | A2Q068 | 1-Hydroxy-N,N-dimethyl-4-[4-[2-[2-[[4-[4-[[3-nitro-4-(2-phenylsulfanylethylamino)phenyl]sulfonylcarbamoyl]phenyl]piperazin-1-yl]methyl]phenyl]ethynyl]phenyl]naphthalene-2-carboxamide | C53H48N6O7S2 | 944.3 |
| mixED 16 | A2Q069 | 1-Hydroxy-N,N-dimethyl-4-[4-[2-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]ethynyl]phenyl]naphthalene-2-carboxamide | C54H45F3N6O8S2 | 1026.27 |
| | A2Q007 | 6-[4-[4-[4-[3-(Dimethylcarbamoyl)-4-hydroxynaphthalen-1-yl]phenyl]-3-fluorobenzoyl]piperazin-1-yl]-N-[3-nitro-4-(2-phenylsulfanylethylamino)phenyl]sulfonylpyridazine-3-carboxamide | C49H43FN8O8S2 | 954.26 |
| | A2Q070 | 1-Hydroxy-N,N-dimethyl-4-[4-[2-[5-[4-[4-[[3-nitro-4-(2-phenylsulfanylethylamino)phenyl]sulfonylcarbamoyl]phenyl]piperazine-1-carbonyl]pyridin-3-yl]ethynyl]phenyl]naphthalene-2-carboxamide | C52H45N7O8S2 | 959.28 |
| | A2Q071 | 1-Hydroxy-N,N-dimethyl-4-[4-[1-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]pyrazol-4-yl]phenyl]naphthalene-2-carboxamide | C55H47F3N8O8S2 | 1068.29 |
| | A2Q072 | 1-Hydroxy-N,N-dimethyl-4-[5-[2-[[4-[4-[[3-nitro-4-(2-phenylsulfanylethylamino)phenyl]sulfonylcarbamoyl]phenyl]piperazin-1-yl]methyl]phenyl]pyridin-2-yl]naphthalene-2-carboxamide | C50H47N7O7S2 | 921.3 |
| | A2Q073 | 1-Hydroxy-N,N-dimethyl-4-[5-[2-[3-[4-[4-[[3-nitro-4-(2-phenylsulfanylethylamino)phenyl]sulfonylcarbamoyl]phenyl]piperazin-1-yl]-3-oxopropyl]phenyl]pyridin-2-yl]naphthalene-2-carboxamide | C52H49N7O8S2 | 963.31 |
| | A2Q074 | 1-Hydroxy-N,N-dimethyl-4-[5-[4-[1-[4-[4-[[3-nitro-4-(2-phenylsulfanylethylamino)phenyl]sulfonylcarbamoyl]phenyl]piperazin-1-yl]ethyl]phenyl]pyridin-2-yl]naphthalene-2-carboxamide | C51H49N7O7S2 | 935.31 |
| | A2Q075 | 1-Hydroxy-N,N-dimethyl-4-[5-[2-methyl-4-[[4-[4-[[3-nitro-4-(2-phenylsulfanylethylamino)phenyl]sulfonylcarbamoyl]phenyl]piperazin-1-yl]methyl]phenyl]pyridin-2-yl]naphthalene-2-carboxamide | C51H49N7O7S2 | 935.31 |
| | A2Q076 | 1-Hydroxy-N,N-dimethyl-4-[5-[2-methyl-4-[4-[4-[[3-nitro-4-(2-phenylsulfanylethylamino)phenyl]sulfonylcarbamoyl]phenyl]piperazine-1-carbonyl]phenyl]pyridin-2-yl]naphthalene-2-carboxamide | C51H47N7O8S2 | 949.29 |
| | A2Q077 | 4-[5-[3-Ethyl-4-[[4-[4-[[3-nitro-4-(2-phenylsulfanylethylamino)phenyl]sulfonylcarbamoyl]phenyl]piperazin-1-yl]methyl]phenyl]pyridin-2-yl]-1-hydroxy-N,N-dimethylnaphthalene-2-carboxamide | C52H51N7O7S2 | 949.33 |

[1519] [Table 152]

| | | | | |
|---|---|---|---|---|
| mixED 16 | A2Q078 | 4-[5-[2-Fluoro-4-[[4-[4-[[3-nitro-4-(2-phenylsulfanylethylamino)phenyl]sulfonylcarbamoyl]phenyl]piperazin-1-yl]methyl]phenyl]pyridin-2-yl]-1-hydroxy-N,N-dimethylnaphthalene-2-carboxamide | C50H46FN7O7S2 | 939.29 |
| | A2Q079 | 4-[5-[2-Fluoro-4-[4-[4-[[3-nitro-4-(2-phenylsulfanylethylamino)phenyl]sulfonylcarbamoyl]phenyl]piperazine-1-carbonyl]phenyl]pyridin-2-yl]-1-hydroxy-N,N-dimethylnaphthalene-2-carboxamide | C50H44FN7O8S2 | 953.27 |
| | A2Q008 | 6-[4-[[4-[4-[3-(Dimethylcarbamoyl)-4-hydroxynaphthalen-1-yl]phenyl]-2-methoxyphenyl]methyl]piperazin-1-yl]-N-[3-nitro-4-(2-phenylsulfanylethylamino)phenyl]sulfonylpyridazine-3-carboxamide | C50H48N8O8S2 | 952.3 |
| | A2Q080 | 1-Hydroxy-4-[5-[3-methoxy-4-[[4-[4-[[3-nitro-4-(2-phenylsulfanylethylamino)phenyl]sulfonylcarbamoyl]phenyl]piperazin-1-yl]methyl]phenyl]pyridin-2-yl]-N,N-dimethylnaphthalene-2-carboxamide | C51H49N7O8S2 | 951.31 |
| | A2Q081 | 4-[5-[3,5-Dimethoxy-4-[[4-[4-[[3-nitro-4-(2-phenylsulfanylethylamino)phenyl]sulfonylcarbamoyl]phenyl]piperazin-1-yl]methyl]phenyl]pyridin-2-yl]-1-hydroxy-N,N-dimethylnaphthalene-2-carboxamide | C52H51N7O9S2 | 981.32 |
| | A2Q082 | 1-Hydroxy-N,N-dimethyl-4-[5-[4-[[4-[4-[[3-nitro-4-(2-phenylsulfanylethylamino)phenyl]sulfonylcarbamoyl]phenyl]piperazin-1-yl]methyl]naphthalen-1-yl]pyridin-2-yl]naphthalene-2-carboxamide | C54H49N7O7S2 | 971.31 |
| | A2Q083 | 1-Hydroxy-N,N-dimethyl-4-[5-[4-[4-[4-[[3-nitro-4-(2-phenylsulfanylethylamino)phenyl]sulfonylcarbamoyl]phenyl]piperazine-1-carbonyl]naphthalen-1-yl]pyridin-2-yl]naphthalene-2-carboxamide | C54H47N7O8S2 | 985.29 |
| | A2Q084 | 1-Hydroxy-N,N-dimethyl-4-[5-[5-[[4-[4-[[3-nitro-4-(2-phenylsulfanylethylamino)phenyl]sulfonylcarbamoyl]phenyl]piperazin-1-yl]methyl]thiophen-3-yl]pyridin-2-yl]naphthalene-2-carboxamide | C48H45N7O7S3 | 927.25 |
| | A2Q085 | 1-Hydroxy-N,N-dimethyl-4-[5-[5-[4-[4-[[3-nitro-4-(2-phenylsulfanylethylamino)phenyl]sulfonylcarbamoyl]phenyl]piperazine-1-carbonyl]thiophen-3-yl]pyridin-2-yl]naphthalene-2-carboxamide | C48H43N7O8S3 | 941.23 |
| mixED 16 | A2Q086 | 1-Hydroxy-N,N-dimethyl-4-[5-[3-[[4-[4-[[3-nitro-4-(2-phenylsulfanylethylamino)phenyl]sulfonylcarbamoyl]phenyl]piperazin-1-yl]methyl]-5-(trifluoromethyl)phenyl]pyridin-2-yl]naphthalene-2-carboxamide | C51H46F3N7O7S2 | 989.29 |
| | A2Q087 | 1-Hydroxy-N,N-dimethyl-4-[5-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]pyridin-2-yl]naphthalene-2-carboxamide | C51H44F3N7O8S2 | 1003.26 |
| | A2Q088 | 1-Hydroxy-N,N-dimethyl-4-[5-[3-[[4-[4-[[3-nitro-4-(2-phenylsulfanylethylamino)phenyl]sulfonylcarbamoyl]phenyl]piperazin-1-yl]methyl]-5-(trifluoromethoxy)phenyl]pyridin-2-yl]naphthalene-2-carboxamide | C51H46F3N7O8S2 | 1005.28 |
| | A2Q089 | 4-[5-[3-Ethoxy-5-[[4-[4-[[3-nitro-4-(2-phenylsulfanylethylamino)phenyl]sulfonylcarbamoyl]phenyl]piperazin-1-yl]methyl]phenyl]pyridin-2-yl]-1-hydroxy-N,N-dimethylnaphthalene-2-carboxamide | C52H51N7O8S2 | 965.32 |
| | A2Q009 | 6-[4-[[4-[4-[3-(Dimethylcarbamoyl)-4-hydroxynaphthalen-1-yl]phenyl]-2,6-dimethoxyphenyl]methyl]piperazin-1-yl]-N-[3-nitro-4-(2-phenylsulfanylethylamino)phenyl]sulfonylpyridazine-3-carboxamide | C51H50N8O9S2 | 982.31 |
| | A2Q090 | 1-Hydroxy-N,N-dimethyl-4-[5-[3-[[4-[4-[[3-nitro-4-(2-phenylsulfanylethylamino)phenyl]sulfonylcarbamoyl]phenyl]piperazin-1-yl]methyl]-5-propan-2-yloxyphenyl]pyridin-2-yl]naphthalene-2-carboxamide | C53H53N7O8S2 | 979.34 |
| | A2Q091 | 4-[5-[3-tert-Butyl-5-[4-[4-[[3-nitro-4-(2-phenylsulfanylethylamino)phenyl]sulfonylcarbamoyl]phenyl]piperazine-1-carbonyl]phenyl]pyridin-2-yl]-1-hydroxy-N,N-dimethylnaphthalene-2-carboxamide | C54H53N7O8S2 | 991.34 |
| | A2Q092 | 4-[5-[3-Fluoro-4-[1-[4-[4-[[3-nitro-4-(2-phenylsulfanylethylamino)phenyl]sulfonylcarbamoyl]phenyl]piperazin-1-yl]ethyl]phenyl]pyridin-2-yl]-1-hydroxy-N,N-dimethylnaphthalene-2-carboxamide | C51H48FN7O7S2 | 953.3 |
| | A2Q093 | 1-Hydroxy-N,N-dimethyl-4-[5-[2-[2-[[4-[4-[[3-nitro-4-(2-phenylsulfanylethylamino)phenyl]sulfonylcarbamoyl]phenyl]piperazin-1-yl]methyl]phenyl]ethynyl]pyridin-2-yl]naphthalene-2-carboxamide | C52H47N7O7S2 | 945.3 |
| | A2Q094 | 1-Hydroxy-N,N-dimethyl-4-[5-[2-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]ethynyl]pyridin-2-yl]naphthalene-2-carboxamide | C53H44F3N7O8S2 | 1027.26 |

| | | | | |
|---|---|---|---|---|
| | A2Q095 | 1-Hydroxy-N,N-dimethyl-4-[5-[2-[5-[4-[4-[[3-nitro-4-(2-phenylsulfanylethylamino)phenyl]sulfonylcarbamoyl]phenyl]piperazine-1-carbonyl]pyridin-3-yl]ethynyl]pyridin-2-yl]naphthalene-2-carboxamide | C51H44N8O8S2 | 960.27 |
| | A2Q096 | 1-Hydroxy-N,N-dimethyl-4-[5-[1-[3-[4-[4-[[3-nitro-4-(2-phenylsulfanylethylamino)phenyl]sulfonylcarbamoyl]phenyl]piperazine-1-carbonyl]-5-(trifluoromethyl)phenyl]pyrazol-4-yl]pyridin-2-yl]naphthalene-2-carboxamide | C54H46F3N9O8S2 | 1069.29 |
| | A2Q097 | 6-[4-[2-[2-[4-[3-(Dimethylcarbamoyl)-4-hydroxynaphthalen-1-yl]phenyl]ethynyl]benzoyl]piperazin-1-yl]-N-[3-nitro-4-(2-phenylsulfanylethylamino)phenyl]sulfonylpyridazine-3-carboxamide | C51H44N8O8S2 | 960.272 |
| | A2Q098 | 6-[4-[2-[2-[6-[3-(Dimethylcarbamoyl)-4-hydroxynaphthalen-1-yl]pyridin-3-yl]ethynyl]benzoyl]piperazin-1-yl]-N-[3-nitro-4-(2-phenylsulfanylethylamino)phenyl]sulfonylpyridazine-3-carboxamide | C50H43N9O8S2 | 961.268 |
| | A2Q099 | 1-Hydroxy-N,N-dimethyl-4-[4-[2-[2-[4-[4-[[3-nitro-4-(2-phenylsulfanylethylamino)phenyl]sulfonylcarbamoyl]phenyl]piperazine-1-carbonyl]phenyl]ethynyl]phenyl]naphthalene-2-carboxamide | C53H46N6O8S2 | 958.282 |
| | A2Q100 | 1-Hydroxy-N,N-dimethyl-4-[5-[2-[2-[4-[4-[[3-nitro-4-(2-phenylsulfanylethylamino)phenyl]sulfonylcarbamoyl]phenyl]piperazine-1-carbonyl]phenyl]ethynyl]pyridin-2-yl]naphthalene-2-carboxamide | C52H45N7O8S2 | 959.277 |

The weights of the mixtures obtained in Examples 3-7-1 and 3-7-2 are shown in Table AG-E-00.

[Table 153]

[Table AG-E-00]

| Mixture No. of starting material | Mixture No. of target compound | No. of compound that may be contained in mixture of target compound | Weight of obtained mixture (mg) |
|---|---|---|---|
| mixED05-01R | mixED05 | A4J001 to A4J100 | 17.6 |
| mixED06-01R | mixED06 | A1M001 to A1M100 | 16.8 |
| mixED07-01R | mixED07 | A1K001 to A1K100 | 23.1 |
| mixED08-01R | mixED08 | A1O001 to A1O100 | 23.0 |
| mixED09-01R | mixED09 | A1A001 to A1A100 | 15.8 |
| mixED10-01R | mixED10 | A3A001 to A3A100 | 14.0 |
| mixED11-01R | mixED11 | A2I001 to A2I100 | 25.2 |
| mixED12-01R | mixED12 | A2L001 to A2L100 | 26.2 |
| mixED13-01R | mixED13 | A2N001 to A2N100 | 28.3 |
| mixED14-01R | mixED14 | A2P001 to A2P100 | 28.0 |
| mixED15-01R | mixED15 | A2A001 to A2A100 | 27.3 |
| mixED16-01R | mixED16 | A2Q001 to A2Q100 | 27.1 |

Results of analyzing the obtained mixtures mixEE01 to mixEE12 by LC/MS are shown in Tables AG-E-01 to AG-E-12. The mixtures were analyzed under analysis conditions SMD-FA25-long-25 min, and the UV areas were confirmed at a UV wavelength of 319 nm. The items described in Tables AG-E-01 to AG-E-12 will be described below by taking Table AG-E-01 as an example.

The table describes the retention times (min) of observed UV peaks, UV areas determined by calculation (Cal_UV_area), $(M+H)^+$ observed in the UV peaks, the retention times of MS peaks in MS chromatograms, and the numbers of compounds assigned to the UV peaks and the MS peaks. When a plurality of compounds were assigned to the same UV peak, the UV area was described as a value obtained by equal division by the number of assigned compounds.

In Table AG-E-01, $(M+H)^+$ = 650.2 was observed in a peak at a retention time of 2.84 minutes, and only

compound A4J035 was assigned thereto. Since a single compound was assigned, the numeric value described in Cal_UV_area was a UV area that corresponded to one compound.

A mixture that could contain 100 compounds may contain a plurality of compounds having the same compositional formula and exact mass. As one example, three compounds, A4J031, A4J079, and A4J092, corresponded to a compound that exhibited $(M+H)^+$ = 674.3 observed in a peak at a retention time of 3.32 minutes in Table AG-E-01. Therefore, three compound numbers were described in the column "No. of assigned compound". Further, A4J31, A4J79, and A4J92 were described in all of 3.32 minutes, 4.61 minutes, and 8.51 minutes because $(M+H)^+$ = 674.3 was also observed in UV peaks at 4.61 minutes and 8.51 minutes.

On the other hand, a plurality of compounds may be frequently eluted at a certain retention time. In this case, the same retention time was described as different cells on a detected MS basis.

For example, two $(M+H)^+$ values, 704.3 and 648.2, were detected in a UV peak at 3.85 minutes in Table AG-E-01. Therefore, the UV peak at a retention time of 3.85 minutes was described over two cells, and A4J32 and A4J84 were assigned to $(M+H)^+$ = 704.3 and $(M+H)^+$ = 648.2, respectively. In this case, as for a UV area, a numeric value determined by dividing the UV area at the UV peak by 2 was described in Cal_UV_area because two compounds were assigned to the UV peak at 3.85 minutes. As another example, four compounds (A4J009, A4J036, A4J047, and A4J086) were assigned to a UV peak at 5.81 minutes. Therefore, a numeric value obtained by dividing the UV area at the UV peak by 4 was described in Cal_UV_area.

As for the numeric values in Cal_UV, a median, a mean, standard deviation, 75 percentile, 25 percentile, an upper boundary value, a lower boundary value, the number of outliers, a maximum value, and a minimum value were described by statistical processing. The upper boundary value was calculated according to formula AH01, the lower boundary value was calculated according to formula AH02, and the number of outliers was calculated according to formula AH03.

[Formula AH01]

$$\text{Upper boundary value} = \text{75 percentile} + 1.5 \times (\text{75 percentile} - \text{25 percentile})$$

[Formula AH02]

$$\text{Lower boundary value} = \text{25 percentile} - 1.5 \times (\text{75 percentile} - \text{25 percentile})$$

[Formula AH03]

$$\text{The number of outliers} = \text{The number of data on } (\text{Cal_UV} > \text{Upper boundary value}) + \text{The number of data on } (\text{Cal_UV} < \text{Lower boundary value})$$

[Table 154]

[Table AG-E-01]

| Mixture No. | Retention time of UV peak (min) | Cal_UV_area | (M+H) observed at MS peak top | Retention time of MS peak (min) | No. of assigned compound |
|---|---|---|---|---|---|
| mixED05 | 2.84 | 38578512 | 650.2 | 2.88 | A4J035 |
| | 3.21 | 21882518 | 658.3 | 3.26 | A4J026 |
| | 3.32 | 27112434 | 674.3 | 3.36 | A4J031, A4J079, A4J092 |
| | 3.40 | 28501332 | 644.3 | 3.44 | A4J024 |
| | 3.52 | 22282806 | 662.3 | 3.55 | A4J029, A4J085 |

(continued)

[Table AG-E-01]

| Mixture No. | Retention time of UV peak (min) | Cal_UV_area | (M+H) observed at MS peak top | Retention time of MS peak (min) | No. of assigned compound |
|---|---|---|---|---|---|
| | 3.85 | 23525756 | 704.3 | 3.91 | A4J032 |
| | 3.85 | 23525756 | 648.2 | 3.87 | A4J084 |
| | 3.99 | 24858522 | 672.3 | 4.02 | A4J027, A4J028, A4J080 |
| | 4.09 | 9406083 | 656.3 | 4.13 | A4J074, A4J075 |
| | 4.16 | 26508828 | 688.3 | 4.20 | A4J040 |
| | 4.16 | 26508828 | 656.3 | 4.20 | A4J074, A4J075 |
| | 4.28 | 24536036 | 672.3 | 4.33 | A4J027, A4J028, A4J080 |
| | 4.28 | 24536036 | 642.3 | 4.32 | A4J072 |
| | 4.37 | 30163262 | 694.3 | 4.40 | A4J033 |
| | 4.42 | 25584672 | 660.3 | 4.45 | A4J078 |
| | 4.61 | 16863900 | 674.3 | 4.64 | A4J031, A4J079, A4J092 |
| | 4.65 | 22776422 | 702.3 | 4.68 | A4J041, A4J081 |
| | 4.65 | 22776422 | 668.3 | 4.70 | A4J043 |
| | 4.79 | 27149836 | 702.3 | 4.83 | A4J041, A4J081 |
| | 4.88 | 12654044 | 672.3 | 4.90 | A4J027, A4J028, A4J080 |
| | 4.88 | 12654044 | 670.3 | 4.92 | A4J076, A4J077 |
| | 5.01 | 22268604 | 686.3 | 5.04 | A4J025, A4J089 |
| | 5.01 | 22268604 | 712.3 | 5.07 | A4J037, A4J091 |
| | 5.07 | 19396998 | 643.3 | 5.12 | A4J001 |
| | 5.14 | 53829068 | 649.2 | 5.19 | A4J012 |
| | 5.23 | 31290640 | 692.3 | 5.27 | A4J082 |
| | 5.27 | 25037152 | 728.2 | 5.30 | A4J039 |
| | 5.40 | 22663282.67 | 661.3 | 5.44 | A4J006, A4J060 |

(continued)

[Table AG-E-01]

| Mixture No. | Retention time of UV peak (min) | Cal_UV_area | (M+H) observed at MS peak top | Retention time of MS peak (min) | No. of assigned compound |
|---|---|---|---|---|---|
| | 5.40 | 22663282.67 | 673.3 | 5.42 | A4J008, A4J054, A4J067 |
| | 5.40 | 22663282.67 | 666.3 | 5.44 | A4J093 |
| | 5.45 | 18642414 | 657.3 | 5.50 | A4J003 |
| | 5.45 | 18642414 | 700.3 | 5.49 | A4J090 |
| | 5.81 | 20372094 | 703.3 | 5.82 | A4J009 |
| | 5.81 | 20372094 | 664.2 | 5.86 | A4J036 |
| | 5.81 | 20372094 | 641.3 | 5.88 | A4J047 |
| | 5.81 | 20372094 | 710.3 | 5.84 | A4J086 |
| | 5.95 | 20016940 | 647.2 | 5.98 | A4J059 |
| | 6.03 | 21288100 | 687.3 | 6.07 | A4J017 |
| | 6.03 | 21288100 | 726.3 | 6.07 | A4J038, A4J088 |
| | 6.10 | 36868312 | 667.3 | 6.13 | A4J020 |
| | 6.10 | 36868312 | 655.3 | 6.14 | A4J049, A4J050 |
| | 6.19 | 36247184 | 671.3 | 6.24 | A4J004, A4J005, A4J055 |
| | 6.19 | 36247184 | 659.3 | 6.23 | A4J053 |
| | 6.25 | 20122220 | 655.3 | 6.29 | A4J049, A4J050 |
| | 6.45 | 25252357.33 | 673.3 | 6.48 | A4J008, A4J054, A4J067 |
| | 6.45 | 25252357.33 | 693.3 | 6.45 | A4J010 |
| | 6.45 | 25252357.33 | 701.3 | 6.50 | A4J018, A4J056 |
| | 6.61 | 22423498.67 | 701.3 | 6.66 | A4J018, A4J056 |
| | 6.61 | 22423498.67 | 686.3 | 6.59 | A4J025, A4J089 |
| | 6.61 | 22423498.67 | 676.2 | 6.63 | A4J030 |
| | 6.65 | 43338036 | 683.2 | 6.71 | A4J045, A4J048 |
| | 6.76 | 28766030 | 685.3 | 6.81 | A4J002, A4J064 |
| | 6.76 | 28766030 | 665.3 | 6.79 | A4J068 |

(continued)

| [Table AG-E-01] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| | 6.88 | 18964904 | 711.3 | 6.92 | A4J014, A4J066 |
| | 6.95 | 24832622 | 669.3 | 6.98 | A4J051, A4J052 |

[Table 155]

| | | | | | |
|---|---|---|---|---|---|
| mixED05 | 7.10 | 21914540 | 727.3 | 7.15 | A4J016 |
| | 7.19 | 22671564 | 691.3 | 7.24 | A4J057 |
| | 7.19 | 22671564 | 699.3 | 7.22 | A4J065 |
| | 7.23 | 35033852 | 708.3 | 7.27 | A4J034 |
| | 7.57 | 17722016 | 709.3 | 7.61 | A4J061 |
| | 7.78 | 19834852 | 725.3 | 7.83 | A4J015, A4J063 |
| | 7.83 | 26834614 | 662.2 | 7.88 | A4J029, A4J085 |
| | 8.04 | 17133932 | 670.3 | 8.08 | A4J076, A4J077 |
| | 8.28 | 28816384 | 726.2 | 8.33 | A4J038, A4J088 |
| | 8.42 | 21193554 | 684.3 | 8.46 | A4J073 |
| | 8.42 | 21193554 | 681.3 | 8.46 | A4J095 |
| | 8.51 | 25013614 | 674.3 | 8.54 | A4J031, A4J079, A4J092 |
| | 8.65 | 34074688 | 792.3 | 8.70 | A4J046 |
| | 8.73 | 41205888 | 682.2 | 8.76 | A4J022 |
| | 8.79 | 25539308 | 714.3 | 8.82 | A4J042 |
| | 9.12 | 30254220 | 663.2 | 9.17 | A4J013 |
| | 9.12 | 30254220 | 706.3 | 9.15 | A4J083 |
| | 9.38 | 31145200 | 675.2 | 9.42 | A4J007 |
| | 9.61 | 12520713 | 671.3 | 9.61 | A4J004, A4J005, A4J055 |
| | 9.61 | 12520713 | 671.3 | 9.66 | A4J004, A4J005, A4J055 |
| | 10.00 | 12268558 | 685.3 | 10.03 | A4J002, A4J064 |
| | 10.08 | 21677866 | 724.2 | 10.12 | A4J087 |
| | 10.19 | 38445688 | 750.2 | 10.24 | A4J044 |
| | 10.41 | 29615534 | 707.3 | 10.45 | A4J011 |
| | 10.41 | 29615534 | 790.3 | 10.44 | A4J096 |
| | 10.49 | 34758108 | 680.3 | 10.53 | A4J070 |
| | 10.70 | 23061596 | 712.3 | 10.75 | A4J037, A4J091 |
| | 10.92 | 28263052 | 725.2 | 10.96 | A4J015, A4J063 |

(continued)

| | | | | | | |
|---|---|---|---|---|---|---|
| | | 11.09 | 23361484 | 661.2 | 11.13 | A4J006, A4J060 |
| | | 11.25 | 21841936 | 673.2 | 11.29 | A4J008, A4J054, A4J067 |
| | | 11.53 | 19129198 | 669.3 | 11.57 | A4J051, A4J052 |
| | | 11.65 | 39446420 | 791.3 | 11.68 | A4J023 |
| | | 11.73 | 14338908 | 683.3 | 11.76 | A4J045, A4J048 |
| | | 11.95 | 27342416 | 748.2 | 11.99 | A4J094 |
| | | 12.06 | 21639162 | 713.3 | 12.10 | A4J019 |
| | | 12.27 | 35168108 | 749.2 | 12.31 | A4J021 |
| | | 12.27 | 35168108 | 705.3 | 12.29 | A4J058 |
| | | 12.65 | 19289504 | 723.2 | 12.69 | A4J062 |
| | | 13.27 | 37275540 | 789.3 | 13.31 | A4J071 |
| | | 13.87 | 18397536 | 711.3 | 13.90 | A4J014, A4J066 |
| | | 13.92 | 31062448 | 747.3 | 13.96 | A4J069 |
| Statistical analysis | The number of samples of Cal_UV_area | | 96 | | | |
| | Median | | 23443620 | | | |
| | Mean | | 25297890 | | | |
| | Standard deviation | | 7519950 | | | |
| | 75 percentile | | 29016172 | | | |
| | 25 percentile | | 20988189 | | | |
| | Upper boundary value | | 41058145 | | | |
| | Lower boundary value | | 8946215 | | | |
| | The number of outliers | | 3 | | | |
| | Maximum value | | 53829068 | | | |
| | Minimum value | | 9406083 | | | |

[Table 156]

| [Table AG-E-02] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| mixED06 | 3.23 | 52217460 | 636.2 | 3.26 | A1M035 |
| | 3.38 | 21501496 | 634.2 | 3.41 | A1M084 |
| | 3.51 | 16955306 | 644.3 | 3.55 | A1M026 |
| | 3.60 | 22368584 | 660.2 | 3.63 | A1M031, A1M079, A1M092 |
| | 3.69 | 24532360 | 630.2 | 3.74 | A1M024 |

(continued)

[Table AG-E-02]

| Mixture No. | Retention time of UV peak (min) | Cal_UV_area | (M+H) observed at MS peak top | Retention time of MS peak (min) | No. of assigned compound |
|---|---|---|---|---|---|
| | 3.74 | 10951016 | 642.3 | 3.79 | A1M074, A1M075 |
| | 3.74 | 10951016 | 642.3 | 3.83 | A1M074, A1M075 |
| | 3.84 | 27005864 | 628.3 | 3.87 | A1M072 |
| | 3.87 | 40903832 | 658.3 | 3.91 | A1M027, A1M028, A1M080 |
| | 4.12 | 19996548 | 690.3 | 4.16 | A1M032 |
| | 4.18 | 16600186 | 660.3 | 4.20 | A1M031, A1M079, A1M092 |
| | 4.35 | 15997075 | 658.3 | 4.40 | A1M027, A1M028, A1M080 |
| | 4.40 | 19930604 | 688.3 | 4.44 | A1M041, A1M081 |
| | 4.45 | 26163226 | 674.3 | 4.48 | A1M040 |
| | 4.45 | 26163226 | 656.3 | 4.51 | A1M076, A1M077 |
| | 4.65 | 25677560 | 672.3 | 4.69 | A1M025, A1M089 |
| | 4.76 | 21775242 | 680.3 | 4.80 | A1M033 |
| | 4.83 | 29420302 | 678.3 | 4.87 | A1M082 |
| | 4.94 | 21273824 | 688.3 | 4.95 | A1M041, A1M081 |
| | 4.94 | 21273824 | 654.3 | 4.99 | A1M043 |
| | 5.01 | 18409000 | 652.2 | 5.04 | A1M093 |
| | 5.35 | 16828138 | 629.2 | 5.39 | A1M001 |
| | 5.44 | 15939154.67 | 698.2 | 5.48 | A1M037, A1M091 |
| | 5.44 | 15939154.67 | 627.3 | 5.49 | A1M047 |
| | 5.44 | 15939154.67 | 696.3 | 5.46 | A1M086 |
| | 5.55 | 24698106 | 635.2 | 5.58 | A1M012 |
| | 5.55 | 24698106 | 633.2 | 5.60 | A1M059 |
| | 5.63 | 7491812.5 | 659.3 | 5.67 | A1M008, A1M054, A1M067 |
| | 5.63 | 7491812.5 | 712.2 | 5.71 | A1M038, A1M088 |

(continued)

[Table AG-E-02]

| Mixture No. | Retention time of UV peak (min) | Cal_UV_area | (M+H) observed at MS peak top | Retention time of MS peak (min) | No. of assigned compound |
|---|---|---|---|---|---|
| | 5.69 | 25165404 | 643.3 | 5.77 | A1M003 |
| | 5.69 | 25165404 | 714.2 | 5.74 | A1M039 |
| | 5.76 | 19782242 | 647.2 | 5.80 | A1M006, A1M060 |
| | 5.76 | 19782242 | 641.3 | 5.80 | A1M049, A1M050 |
| | 5.83 | 10172710 | 645.3 | 5.86 | A1M053 |
| | 5.83 | 10172710 | 646.2 | 5.86 | A1M078 |
| | 5.87 | 20395902 | 657.3 | 5.90 | A1M004, A1M005, A1M055 |
| | 5.87 | 20395902 | 641.3 | 5.94 | A1M049, A1M050 |
| | 6.02 | 19069538 | 689.3 | 6.06 | A1M009 |
| | 6.06 | 21898480 | 659.3 | 6.10 | A1M008, A1M054, A1M067 |
| | 6.30 | 21205670 | 673.3 | 6.34 | A1M017 |
| | 6.30 | 21205670 | 687.3 | 6.31 | A1M018, A1M056 |
| | 6.33 | 26034290 | 651.3 | 6.37 | A1M068 |
| | 6.37 | 30061744 | 653.3 | 6.42 | A1M020 |
| | 6.44 | 22791804 | 671.3 | 6.50 | A1M002, A1M064 |
| | 6.44 | 22791804 | 650.2 | 6.47 | A1M036 |
| | 6.57 | 40235684 | 655.3 | 6.61 | A1M051, A1M052 |
| | 6.74 | 16925500 | 687.3 | 6.78 | A1M018, A1M056 |
| | 6.74 | 16925500 | 658.3 | 6.76 | A1 M027, A1 M028, A1 M080 |
| | 6.80 | 35075172 | 677.3 | 6.85 | A1M057 |
| | 6.88 | 17292164 | 679.3 | 6.92 | A1M010 |
| | 6.88 | 17292164 | 685.3 | 6.91 | A1M065 |
| | 6.88 | 17292164 | 686.3 | 6.91 | A1M090 |
| | 7.10 | 23106708 | 672.3 | 7.18 | A1M025, A1M089 |
| | 7.10 | 23106708 | 648.2 | 7.13 | A1M029, A1M085 |

(continued)

| [Table AG-E-02] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| | 7.19 | 25255197.33 | 662.2 | 7.23 | A1M030 |

[Table 157]

| | | | | | |
|---|---|---|---|---|---|
| | 7.19 | 25255197.33 | 669.3 | 7.25 | A1M045, A1M048 |
| | 7.19 | 25255197.33 | 695.3 | 7.22 | A1M061 |
| | 7.29 | 12347282 | 657.3 | 7.32 | A1M004, A1M005, A1M055 |
| | 7.29 | 12347282 | 656.3 | 7.34 | A1M076, A1M077 |
| | 7.40 | 16607620 | 697.2 | 7.43 | A1M014, A1M066 |
| | 7.40 | 16607620 | 711.2 | 7.44 | A1M015, A1M063 |
| | 7.62 | 22156286 | 713.2 | 7.67 | A1M016 |
| | 7.77 | 22624480 | 660.2 | 7.82 | A1M031, A1M079, A1M092 |
| | 7.77 | 22624480 | 670.3 | 7.82 | A1M073 |
| | 7.77 | 22624480 | 667.3 | 7.79 | A1M095 |
| | 7.85 | 36635312 | 694.3 | 7.90 | A1M034 |
| | 8.33 | 29578408 | 692.3 | 8.37 | A1M083 |
| | 8.87 | 25709756 | 712.2 | 8.91 | A1M038, A1M088 |
| | 9.21 | 28571980 | 778.2 | 9.26 | A1M046 |
| | 9.30 | 27855746 | 668.3 | 9.34 | A1M022 |
| | 9.30 | 27855746 | 710.2 | 9.37 | A1M087 |
| | 9.42 | 29309702 | 700.3 | 9.46 | A1M042 |
| | 9.67 | 32400960 | 776.3 | 9.70 | A1M096 |
| | 9.76 | 33709276 | 649.2 | 9.81 | A1M013 |
| | 9.76 | 33709276 | 666.3 | 9.81 | A1M070 |
| | 9.85 | 23715128 | 698.3 | 9.89 | A1M037, A1M091 |
| | 10.00 | 26818024 | 661.2 | 10.04 | A1M007 |
| | 10.27 | 16117688 | 657.3 | 10.30 | A1M004, A1M005, A1M055 |
| | 10.27 | 16117688 | 647.2 | 10.32 | A1M006, A1M060 |
| | 10.27 | 16117688 | 648.2 | 10.31 | A1M029, A1M085 |
| | 10.44 | 25374254 | 659.3 | 10.48 | A1M008, A1M054, A1M067 |
| | 10.68 | 20452822 | 655.3 | 10.72 | A1M051, A1M052 |
| | 10.76 | 21710204 | 671.3 | 10.80 | A1M002, A1M064 |
| | 10.76 | 21710204 | 736.2 | 10.81 | A1M044 |

(continued)

| | | | | | |
|---|---|---|---|---|---|
| | 11.04 | 27548338 | 693.3 | 11.08 | A1M011 |
| | 11.04 | 27548338 | 669.3 | 11.13 | A1M045, A1M048 |
| | 11.16 | 26972188 | 734.2 | 11.20 | A1M094 |
| | 11.37 | 29541632 | 691.3 | | 11.40A1M058 |
| | 11.48 | 27663890 | 711.2 | | 11.53A1M015, A1M063 |
| | 11.82 | 27406210 | 709.2 | 11.86 | A1M062 |
| | 12.17 | 53990416 | 777.3 | | 12.20A1M023 |
| | 12.48 | 39330948 | 775.3 | 12.52 | A1M071 |
| | 12.65 | 22059418 | 699.3 | | 12.68A1M019 |
| | 12.80 | 39837748 | 735.2 | | 12.84A1M021 |
| | 12.92 | 17940780 | 697.3 | | 12.96 A1M014, A1M066 |
| | 13.09 | 31319776 | 733.2 | | 13.13 A1M069 |
| Statistical analysis | The number of samples of Cal_UV_area | 96 | | | |
| | Median | 22624480 | | | |
| | Mean | 23445531 | | | |
| | Standard deviation | 8145668 | | | |
| | 75 percentile | 27105951 | | | |
| | 25 percentile | 17292164 | | | |
| | Upper boundary value | 41826630 | | | |
| | Lower boundary value | 2571484 | | | |
| | The number of outliers | 2 | | | |
| | Maximum value | 53990416 | | | |
| | Minimum value | 7491813 | | | |

[Table 158]

| [Table AG-E-03] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| mixED07 | 3.63 | 50898764 | 676.151 | 3.657 | A1K035 |
| | 3.83 | 24530820 | 684.237 | 3.877 | A1K026 |
| | 3.83 | 24530820 | 674.15 | 3.863 | A1K084 |
| | 3.91 | 33646700 | 700.191 | 3.947 | A1K031, A1K079, A1K092 |
| | 4.07 | 18972792 | 670.218 | 4.123 | A1K024 |
| | 4.07 | 18972792 | 682.228 | 4.077 | A1K074, A1K075 |

(continued)

[Table AG-E-03]

| Mixture No. | Retention time of UV peak (min) | Cal_UV_area | (M+H) observed at MS peak top | Retention time of MS peak (min) | No. of assigned compound |
|---|---|---|---|---|---|
| | 4.14 | 26273452 | 682.2 | 4.173 | A1K074, A1K075 |
| | 4.21 | 18809310 | 688.207 | 4.25 | A1K029, A1K085 |
| | 4.25 | 37017708 | 698.211 | 4.297 | A1K027, A1K028, A1K080 |
| | 4.25 | 37017708 | 668.229 | 4.3 | A1K072 |
| | 4.42 | 29486682 | 730.204 | 4.45 | A1K032 |
| | 4.42 | 29486682 | 686.199 | 4.463 | A1K078 |
| | 4.59 | 23067146 | 700.2 | 4.627 | A1K031, A1K079, A1K092 |
| | 4.76 | 28591176 | 698.22 | 4.763 | A1K027, A1K028, A1K080 |
| | 4.76 | 28591176 | 714.24 | 4.803 | A1K040 |
| | 4.76 | 28591176 | 728.227 | 4.803 | A1K041, A1K081 |
| | 4.9 | 36509220 | 696.246 | 4.93 | A1K076, A1K077 |
| | 5.02 | 32155254 | 712.245 | 5.057 | A1K025, A1K089 |
| | 5.16 | 31250364 | 720.229 | 5.19 | A1K033 |
| | 5.26 | 26963448 | 728.23 | 5.273 | A1K041, A1K081 |
| | 5.26 | 26963448 | 694.217 | 5.33 | A1K043 |
| | 5.26 | 26963448 | 718.239 | 5.297 | A1K082 |
| | 5.41 | 23194472 | 692.207 | 5.447 | A1K093 |
| | 5.47 | 37987296 | 726.245 | 5.51 | A1K090 |
| | 5.7 | 18110408 | 669.242 | 5.743 | A1K001 |
| | 5.86 | 22315698.67 | 699.22 | 5.927 | A1K008, A1K054, A1K067 |
| | 5.86 | 22315698.67 | 667.235 | 5.863 | A1K047 |
| | 5.86 | 22315698.67 | 736.207 | 5.893 | A1K086 |
| | 5.91 | 71570272 | 675.15 | 5.95 | A1K012 |
| | 5.98 | 21119532 | 673.166 | 6.013 | A1K059 |
| | 6.02 | 16629562 | 683.218 | 6.057 | A1K003 |

(continued)

[Table AG-E-03]

| Mixture No. | Retention time of UV peak (min) | Cal_UV_area | (M+H) observed at MS peak top | Retention time of MS peak (min) | No. of assigned compound |
|---|---|---|---|---|---|
| | 6.1 | 32430440 | 687.225 | 6.15 | A1K006, A1K060 |
| | 6.1 | 32430440 | 752.142 | 6.14 | A1K038, A1K088 |
| | 6.1 | 32430440 | 681.24 | 6.13 | A1K049, A1K050 |
| | 6.21 | 30244316 | 697.227 | 6.227 | A1K004, A1K005, A1K055 |
| | 6.21 | 30244316 | 729.232 | 6.303 | A1K009 |
| | 6.21 | 30244316 | 681.231 | 6.283 | A1K049, A1K050 |
| | 6.21 | 30244316 | 685.217 | 6.247 | A1K053 |
| | 6.45 | 29217098 | 699.214 | 6.48 | A1K008, A1K054, A1K067 |
| | 6.6 | 23879158 | 713.232 | 6.647 | A1K017 |
| | 6.6 | 23879158 | 727.25 | 6.63 | A1K018, A1K056 |
| | 6.75 | 95816800 | 691.242 | 6.79 | A1K068 |
| | 6.92 | 26106816 | 697.216 | 6.977 | A1K004, A1K005, A1K055 |
| | 6.92 | 26106816 | 690.154 | 6.96 | A1K036 |
| | 6.97 | 33111614 | 695.26 | 7.017 | A1K051, A1K052 |
| | 7.04 | 29065936 | 727.25 | 7.08 | A1K018, A1K056 |
| | 7.19 | 22660470 | 698.186 | 7.23 | A1K027, A1K028, A1K080 |
| | 7.19 | 22660470 | 725.258 | 7.247 | A1K065 |
| | 7.23 | 33943344 | 717.259 | 7.277 | A1K057 |
| | 7.27 | 30852568 | 719.246 | 7.313 | A1K010 |
| | 7.65 | 34338275.2 | 712.203 | 7.71 | A1K025, A1K089 |
| | 7.65 | 34338275.2 | 688.16 | 7.723 | A1K029, A1K085 |
| | 7.65 | 34338275.2 | 702.161 | 7.69 | A1K030 |
| | 7.65 | 34338275.2 | 709.18 | 7.677 | A1K045, A1K048 |

(continued)

| [Table AG-E-03] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| | 7.65 | 34338275.2 | 735.203 | 7.677 | A1K061 |

[Table 159]

| | | | | | |
|---|---|---|---|---|---|
| | 7.81 | 23375534 | 737.201 | 7.847 | A1K014, A1K066 |
| | 7.86 | 28824240 | 751.226 | 7.893 | A1K015, A1K063 |
| | 7.86 | 28824240 | 696.213 | 7.91 | A1K076, A1K077 |
| | 8.02 | 12411670 | 753.161 | 8.05 | A1K016 |
| | 8.02 | 12411670 | 754.17 | 8.067 | A1K039 |
| | 8.27 | 36805524 | 734.205 | 8.297 | A1K034 |
| | 8.27 | 36805524 | 707.184 | 8.317 | A1K095 |
| | 8.33 | 17091272 | 711.202 | 8.363 | A1K002, A1K064 |
| | 8.33 | 17091272 | 700.171 | 8.37 | A1K031, A1K079, A1K092 |
| | 8.33 | 17091272 | 710.215 | 8.353 | A1K073 |
| | 8.87 | 35356664 | 732.204 | 8.92 | A1K083 |
| | 9.23 | 37560096 | 752.09 | 9.277 | A1K038, A1K088 |
| | 9.54 | 49097400 | 818.216 | 9.587 | A1K046 |
| | 9.64 | 54620052 | 708.19 | 9.673 | A1K022 |
| | 9.76 | 36156820 | 740.25 | 9.8 | A1K042 |
| | 9.82 | 37685616 | 750.164 | 9.86 | A1K087 |
| | 10.12 | 41589812 | 689.15 | 10.143 | A1K013 |
| | 10.12 | 41589812 | 816.216 | 10.157 | A1K096 |
| | 10.21 | 45706948 | 706.185 | 10.243 | A1K070 |
| | 10.31 | 18528710 | 701.172 | 10.35 | A1K007 |
| | 10.31 | 18528710 | 693.25 | 10.387 | A1K020 |
| | 10.31 | 18528710 | 738.27 | 10.387 | A1K037, A1K091 |
| | 10.31 | 18528710 | 738.284 | 10.41 | A1K037, A1K091 |
| | 10.55 | 36584936 | 697.2 | 10.587 | A1K004, A1K005, A1K055 |
| | 10.72 | 29055928 | 687.154 | 10.767 | A1K006, A1K060 |
| | 10.87 | 37653880 | 699.184 | 10.913 | A1K008, A1K054, A1K067 |
| | 11.01 | 48862520 | 776.147 | 11.047 | A1K044 |
| | 11.11 | 22607274 | 711.214 | 11.157 | A1K002, A1K064 |
| | 11.11 | 22607274 | 695.243 | 11.15 | A1K051, A1K052 |
| | 11.27 | 42840448 | 733.23 | 11.31 | A1K011 |

(continued)

| | | | | | | |
|---|---|---|---|---|---|---|
| | | 11.46 | 20769962 | 709.236 | 11.507 | A1K045, A1K048 |
| | | 11.55 | 27660616 | 774.173 | 11.58 | A1K094 |
| | | 11.69 | 34665088 | 751.197 | 11.73 | A1K015, A1K063 |
| | | 11.76 | 40357340 | 731.254 | 11.793 | A1K058 |
| | | 12.18 | 45677444 | 749.174 | 12.223 | A1K062 |
| | | 12.32 | 51522140 | 817.256 | 12.357 | A1K023 |
| | | 12.77 | 42461292 | 739.25 | 12.793 | A1K019 |
| | | 12.77 | 42461292 | 815.243 | 12.807 | A1K071 |
| | | 12.91 | 61530564 | 775.188 | 12.94 | A1K021 |
| | | 13.23 | 31594380 | 737.253 | 13.263 | A1K014, A1K066 |
| | | 13.37 | 46009544 | 773.183 | 13.41 | A1K069 |
| Statistical analysis | The number of samples of Cal_UV_area | | 96 | | | |
| | Median | | 30244316 | | | |
| | Mean | | 31783762 | | | |
| | Standard deviation | | 12329284 | | | |
| | 75 percentile | | 36805524 | | | |
| | 25 percentile | | 23162641 | | | |
| | Upper boundary value | | 57269849 | | | |
| | Lower boundary value | | 2698315 | | | |
| | The number of outliers | | 3 | | | |
| | Maximum value | | 95816800 | | | |
| | Minimum value | | 12411670 | | | |

[Table 160]

| [Table AG-E-04] | | | | | |
|---|---|---|---|---|---|
| Mixture No. | Retention time of UV peak (min) | Cal UV area | (M+H) observed at MS peak top | Retention time of MS peak (min) | No. of assigned compound |
| mixED08 | 6.45 | 19551298 | 693.3 | 6.54 | A1O001 |
| | 7.27 | 34213972 | 735.3 | 7.28 | A1O002, A1O064 |
| | 12.21 | 54174040 | 735.3 | 12.24 | A1O002, A1O064 |
| | 6.67 | 38439269.33 | 707.3 | 6.75 | A1O003 |
| | 6.67 | 38439269.33 | 721.3 | 6.71 | A1O004, A1O005, A1O055 |
| | 7.77 | 37256400 | 721.3 | 7.82 | A1O004, A1O005, A1O055 |

(continued)

[Table AG-E-04]

| Mixture No. | Retention time of UV peak (min) | Cal UV area | (M+H) observed at MS peak top | Retention time of MS peak (min) | No. of assigned compound |
|---|---|---|---|---|---|
| | 11.70 | 59706916 | 721.3 | 11.75 | A1O004, A1O005, A1O055 |
| | 11.43 | 52992112 | 711.2 | 11.49 | A1O006, A1O060 |
| | 6.93 | 31424776 | 711.3 | 6.98 | A1O006, A1O060 |
| | 11.43 | 52992112 | 725.2 | 11.46 | A1O007 |
| | 4.88 | 29230486 | 723.2 | 4.91 | A1O008, A1O054, A1O067 |
| | 6.99 | 39295628 | 723.3 | 7.02 | A1O008, A1O054, A1O067 |
| | 11.60 | 57263780 | 723.2 | 11.65 | A1O008, A1O054, A1O067 |
| | 6.90 | 25049634 | 753.3 | 6.93 | A1O009 |
| | 8.17 | 41554472 | 743.3 | 8.24 | A1O010 |
| | 12.41 | 68002112 | 757.3 | 12.45 | A1O011 |
| | 6.82 | 58815044 | 699.2 | 6.87 | A1O012 |
| | 11.23 | 57958888 | 713.2 | 11.28 | A1O013 |
| | 13.87 | 57362725.33 | 761.3 | 13.95 | A1O014, A1O066 |
| | 8.74 | 45294709.33 | 761.3 | 8.77 | A1O014, A1O066 |
| | 12.71 | 55114360 | 775.2 | 12.74 | A1O015, A1O063 |
| | 8.37 | 37742596 | 775.2 | 8.41 | A1O015, A1O063 |
| | 8.94 | 27195590 | 777.2 | 8.98 | A1O016 |
| | 7.27 | 34213972 | 737.2 | 7.30 | A1O017 |
| | 7.06 | 39301508 | 751.3 | 7.10 | A1O018, A1O056 |
| | 7.70 | 41424452 | 751.3 | 7.74 | A1O018, A1O056 |
| | 13.87 | 57362725.33 | 763.3 | 13.89 | A1O019 |
| | 7.39 | 38376572 | 717.3 | 7.42 | A1O020 |
| | 13.87 | 57362725.33 | 799.2 | 13.91 | A1O021 |
| | 10.71 | 68789056 | 732.2 | 10.74 | A1O022 |

(continued)

[Table AG-E-04]

| Mixture No. | Retention time of UV peak (min) | Cal UV area | (M+H) observed at MS peak top | Retention time of MS peak (min) | No. of assigned compound |
|---|---|---|---|---|---|
| | 13.26 | 66322132 | 841.3 | 13.30 | A1O023 |
| | 5.01 | 29028554 | 694.2 | 5.04 | A1O024 |
| | 5.59 | 50393796 | 736.3 | 5.63 | A1O025, A1O089 |
| | 8.80 | 41752580 | 736.3 | 8.83 | A1O025, A1O089 |
| | 4.70 | 46242068 | 708.3 | 4.73 | A1O026 |
| | 8.42 | 52644824 | 722.2 | 8.46 | A1O027, A1O028, A1O080 |
| | 4.88 | 29230486 | 722.3 | 4.92 | A1O027, A1O028, A1O080 |
| | 5.63 | 39401564 | 722.3 | 5.66 | A1O027, A1O028, A1O080 |
| | 8.55 | 58122208 | 712.2 | 8.58 | A1O029, A1O085 |
| | 5.12 | 36086028 | 712.2 | 5.16 | A1O029, A1O085 |
| | 8.84 | 66334352 | 726.2 | 8.89 | A1O030 |
| | 4.70 | 46242068 | 724.3 | 4.75 | A1O031, A1O079, A1O092 |
| | 5.24 | 34877004 | 724.2 | 5.27 | A1O031, A1O079, A1O092 |
| | 9.16 | 48436416 | 724.2 | 9.19 | A1O031, A1O079, A1O092 |
| | 5.16 | 39415848 | 754.3 | 5.21 | A1O032 |
| | 6.09 | 43422584 | 744.3 | 6.14 | A1O033 |
| | 9.47 | 71752280 | 758.3 | 9.51 | A1O034 |
| | 4.63 | 65095832 | 700.2 | 4.66 | A1O035 |
| | 8.17 | 41554472 | 714.2 | 8.20 | A1O036 |
| | 11.13 | 53610308 | 762.3 | 11.18 | A1O037, A1O091 |
| | 6.75 | 30266972 | 762.2 | 6.80 | A1O037, A1O091 |
| | 10.32 | 56633712 | 776.2 | 10.36 | A1O038, A1O088 |

(continued)

| [Table AG-E-04] | | | | | |
|---|---|---|---|---|---|
| Mixture No. | Retention time of UV peak (min) | Cal UV area | (M+H) observed at MS peak top | Retention time of MS peak (min) | No. of assigned compound |
| | 6.67 | 38439269.33 | 776.2 | 6.71 | A1O038, A1O088 |
| | 6.99 | 39295628 | 778.2 | 7.04 | A1O039 |
| | 5.52 | 42700536 | 738.3 | 5.57 | A1O040 |

[Table 161]

| | | | | | |
|---|---|---|---|---|---|
| | 5.34 | 69857400 | 752.2 | 5.37 | A1O041, A1O081 |
| | 6.02 | 33388928 | 752.2 | 6.01 | A1O041, A1O081 |
| | 10.94 | 57768632 | 764.3 | 10.98 | A1O042 |
| | 6.02 | 33388928 | 718.2 | 6.05 | A1O043 |
| | 12.03 | 56219764 | 800.2 | 12.07 | A1O044 |
| | 12.33 | 39179936 | 733.3 | 12.36 | A1O045, A1O048 |
| | 8.74 | 45294709.33 | 733.2 | 8.77 | A1O045, A1O048 |
| | 10.57 | 61802308 | 842.2 | 10.64 | A1O046 |
| | 6.39 | 37348468 | 691.3 | 6.43 | A1O047 |
| | 6.56 | 61163520 | 705.3 | 6.64 | A1O049, A1O050 |
| | 6.75 | 30266972 | 705.3 | 6.79 | A1O049, A1O050 |
| | 11.86 | 53028456 | 719.3 | 11.89 | A1O051, A1O052 |
| | 7.50 | 46231768 | 719.3 | 7.54 | A1O051, A1O052 |
| | 6./5 | 30266972 | 709.3 | 6.79 | A1O053 |
| | 7.77 | 37256400 | 741.3 | 7.80 | A1O057 |
| | 12.50 | 59171212 | 755.3 | 12.53 | A1O058 |
| | 6.56 | 61163520 | 697.2 | 6.59 | A1O059 |
| | 8.17 | 41554472 | 759.3 | 8.21 | A1O061 |
| | 12.84 | 47905164 | 773.2 | 12.88 | A1O062 |
| | 7.64 | 40871804 | 749.3 | 7.69 | A1O065 |
| | 7.12 | 51233772 | 715.3 | 7.16 | A1O068 |
| | 13.98 | 53284168 | 797.2 | 14.02 | A1O069 |
| | 10.94 | 57768632 | 730.2 | 10.98 | A1O070 |
| | 13.39 | 78239032 | 839.3 | 13.43 | A1O071 |

(continued)

| | | | | | | |
|---|---|---|---|---|---|---|
| | | 4.93 | 64874972 | 692.3 | 4.97 | A1O072 |
| | | 9.16 | 48436416 | 734.3 | 9.23 | A1O073 |
| | | 4.79 | 49259992 | 706.3 | 4.83 | A1O074, A1O075 |
| | | 6.75 | 30266972 | 706.3 | 6.79 | A1O074, A1O075 |
| | | 5.52 | 42700536 | 720.3 | 5.55 | A1O076, A1O077 |
| | | 8.74 | 45294709.33 | 720.3 | 8.78 | A1O076, A1O077 |
| | | 5.08 | 47039380 | 710.2 | 5.12 | A1O078 |
| | | 5.87 | 48998572 | 742.3 | 5.92 | A1O082 |
| | | 9.69 | 62237512 | 756.3 | 9.73 | A1O083 |
| | | 4.56 | 48105028 | 699.2 | 4.59 | A1O084 |
| | | 6.45 | 19551298 | 760.3 | 6.48 | A1O086 |
| | | 10.57 | 61802308 | 774.2 | 10.60 | A1O087 |
| | | 6.02 | 33388928 | 750.3 | 6.07 | A1O090 |
| | | 5.87 | 48998572 | 716.3 | 5.88 | A1O093 |
| | | 12.21 | 54174040 | 798.2 | 12.25 | A1O094 |
| | | 9.06 | 55018692 | 731.2 | 9.09 | A1O095 |
| | | 10.84 | 60167656 | 840.3 | 10.88 | A1O096 |
| Statistical analysis | The number of samples of Cal UV area | | 96 | | | |
| | Median | | 46640724 | | | |
| | Mean | | 47158055 | | | |
| | Standard deviation | | 12419874 | | | |
| | 75 percentile | | 57362725 | | | |
| | 25 percentile | | 38423595 | | | |
| | Upper boundary value | | 85771421 | | | |
| | Lower boundary value | | 10014900 | | | |
| | The number of outliers | | 0 | | | |
| | Maximum value | | 78239032 | | | |
| | Minimum value | | 19551298 | | | |

[Table 162]

| [Table AG-E-05] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| mixED09 | 6.73 | 6521323.5 | 748.3 | 6.76 | A1A074, A1A075 |
| | 6.80 | 21696045.33 | 764.4 | 6.88 | A1A027, A1A028, A1A080 |
| | 6.80 | 21696045.33 | 748.3 | 6.84 | A1A074, A1A075 |
| | 6.80 | 21696045.33 | 740.3 | 6.82 | A1A084 |
| | 6.91 | 18098106 | 750.3 | 6.95 | A1A026 |
| | 7.17 | 17601616 | 796.4 | 7.25 | A1A032 |
| | 7.17 | 17601616 | 742.3 | 7.19 | A1A035 |
| | 7.17 | 17601616 | 734.3 | 7.21 | A1A072 |
| | 7.17 | 17601616 | 752.3 | 7.23 | A1A078 |
| | 7.25 | 20812320 | 794.4 | 7.28 | A1A041, A1A081 |
| | 7.34 | 5459982 | 768.4 | 7.36 | A1A030 |
| | 7.34 | 5459982 | 766.4 | 7.37 | A1A031, A1A079, A1A092 |
| | 7.52 | 7440725 | 778.4 | 7.55 | A1A025, A1A089 |
| | 7.52 | 7440725 | 754.3 | 7.59 | A1A029, A1A085 |
| | 7.56 | 33334970 | 736.3 | 7.61 | A1A024 |
| | 7.66 | 7220617 | 763.3 | 7.70 | A1A004, A1A005, A1A055 |
| | 7.66 | 7220617 | 762.4 | 7.69 | A1A076, A1A077 |
| | 7.71 | 20788122 | 780.4 | 7.74 | A1A040 |
| | 7.91 | 15641841 | 792.4 | 7.95 | A1A090 |
| | 7.97 | 14616274 | 758.3 | 8.01 | A1A093 |
| | 8.05 | 17042192 | 784.4 | 8.09 | A1A082 |
| | 8.17 | 31427886 | 764.4 | 8.21 | A1A027, A1A028, A1A080 |
| | 8.45 | 12426432 | 763.4 | 8.49 | A1A004, A1A005, A1A055 |
| | 8.45 | 12426432 | 760.3 | 8.47 | A1A043 |

(continued)

[Table AG-E-05]

| Mixture No. | Retention time of UV peak (min) | Cal_UV_area | (M+H) observed at MS peak top | Retention time of MS peak (min) | No. of assigned compound |
|---|---|---|---|---|---|
| | 8.45 | 12426432 | 733.3 | 8.50 | A1A047 |
| | 8.45 | 12426432 | 747.3 | 8.49 | A1A049, A1A050 |
| | 8.51 | 18098142 | 765.3 | 8.57 | A1A008, A1A054, A1A067 |
| | 8.62 | 11954533 | 747.3 | 8.64 | A1A049, A1A050 |
| | 8.62 | 11954533 | 802.3 | 8.66 | A1A086 |
| | 8.73 | 9621298 | 786.3 | 8.77 | A1A033 |
| | 8.73 | 9621298 | 751.4 | 8.81 | A1A053 |
| | 8.73 | 9621298 | 739.3 | 8.73 | A1A059 |
| | 8.82 | 13280061 | 795.4 | 8.85 | A1A009 |
| | 8.82 | 13280061 | 819.3 | 8.85 | A1A016 |
| | 8.82 | 13280061 | 793.4 | 8.84 | A1A018, A1A056 |
| | 8.82 | 13280061 | 818.3 | 8.86 | A1A038, A1A088 |
| | 8.89 | 13155822 | 749.3 | 8.93 | A1A003 |
| | 9.01 | 6697830 | 735.3 | 9.02 | A1A001 |
| | 9.01 | 6697830 | 765.4 | 9.04 | A1A008, A1A054, A1A067 |
| | 9.01 | 6697830 | 766.3 | 9.06 | A1A031, A1A079, A1A092 |
| | 9.08 | 14489100 | 777.4 | 9.12 | A1A002, A1A064 |
| | 9.24 | 19742716 | 757.3 | 9.28 | A1A068 |
| | 9.42 | 17175283.2 | 753.3 | 9.44 | A1A006, A1A060 |
| | 9.42 | 17175283.2 | 741.3 | 9.46 | A1A012 |
| | 9.42 | 17175283.2 | 779.4 | 9.49 | A1A017 |
| | 9.42 | 17175283.2 | 804.3 | 9.47 | A1A037, A1A091 |
| | 9.42 | 17175283.2 | 791.4 | 9.51 | A1A065 |
| | 9.56 | 15230110 | 761.4 | 9.60 | A1A051, A1A052 |
| | 9.68 | 18556672 | 820.2 | 9.71 | A1A039 |

(continued)

| [Table AG-E-05] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| | 9.90 | 11418254.67 | 793.4 | 9.93 | A1A018, A1A056 |
| | 9.90 | 11418254.67 | 759.3 | 9.93 | A1A020 |
| | 9.90 | 11418254.67 | 794.4 | 9.94 | A1A041, A1A081 |
| | 9.95 | 14665040 | 783.4 | 10.00 | A1A057 |
| | 10.39 | 22866388 | 801.3 | 10.43 | A1A061 |
| | 10.591 | 136068601 | 817.3 | 10.62 | A1A015, A1A063 |

[Table 163]

| | | | | | |
|---|---|---|---|---|---|
| | 10.99 | 15992650 | 785.4 | 11.03 | A1A010 |
| | 11.58 | 12584731 | 803.3 | 11.62 | A1A014, A1A066 |
| | 11.74 | 22262696 | 756.3 | 11.79 | A1A036 |
| | 11.81 | 30677942 | 754.3 | 11.86 | A1A029, A1A085 |
| | 12.03 | 13814776 | 764.3 | 12.09 | A1A027, A1A028, A1A080 |
| | 12.03 | 13814776 | 762.4 | 12.05 | A1A076, A1A077 |
| | 12.14 | 43567424 | 773.3 | 12.20 | A1A095 |
| | 12.38 | 26247936 | 778.3 | 12.43 | A1A025, A1A089 |
| | 12.38 | 26247936 | 766.3 | 12.40 | A1A031, A1A079, A1A092 |
| | 12.65 | 10568778 | 776.4 | 12.69 | A1A073 |
| | 12.97 | 18936552 | 798.4 | 13.01 | A1A083 |
| | 13.11 | 22602728 | 800.3 | 13.15 | A1A034 |
| | 13.65 | 15909670 | 816.3 | 13.69 | A1A087 |
| | 13.79 | 19820142 | 818.3 | 13.83 | A1A038, A1A088 |
| | 13.88 | 20749738 | 882.4 | 13.93 | A1A096 |
| | 14.03 | 24652698 | 884.3 | 14.07 | A1A046 |
| | 14.03 | 24652698 | 772.3 | 14.08 | A1A070 |
| | 14.16 | 24907016 | 774.3 | 14.20 | A1A022 |
| | 14.45 | 16191392 | 804.4 | 14.49 | A1A037, A1A091 |
| | 14.59 | 15335381 | 753.2 | 14.63 | A1A006, A1A060 |
| | 14.72 | 16676426.67 | 765.4 | 14.74 | A1A008, A1A054, A1A067 |
| | 14.72 | 16676426.67 | 755.3 | 14.79 | A1A013 |
| | 14.72 | 16676426.67 | 806.4 | 14.75 | A1A042 |

(continued)

| | | | | | | |
|---|---|---|---|---|---|---|
| | 14.90 | 23429060 | 767.3 | 14.94 | A1A007 |
| | 15.04 | 11112592 | 761.4 | 15.08 | A1A051, A1A052 |
| | 15.16 | 20437354 | 840.3 | 15.20 | A1A094 |
| | 15.28 | 17512042 | 763.3 | 15.33 | A1A004, A1A005, A1A055 |
| | 15.39 | 25709904 | 842.3 | 15.42 | A1A044 |
| | 15.63 | 19699368 | 797.4 | 15.66 | A1A058 |
| | 15.76 | 8582890 | 777.3 | 15.82 | A1A002, A1A064 |
| | 15.76 | 8582890 | 775.4 | 15.79 | A1A045, A1A048 |
| | 15.76 | 8582890 | 775.4 | 15.76 | A1A045, A1A048 |
| | 15.76 | 8582890 | 815.3 | 15.80 | A1A062 |
| | 15.95 | 23289136 | 799.3 | 16.00 | A1A011 |
| | 16.04 | 16923292 | 817.3 | 16.08 | A1A015, A1A063 |
| | 16.14 | 23893510 | 881.3 | 16.18 | A1A071 |
| | 16.40 | 23908930 | 883.3 | 16.44 | A1A023 |
| | 16.72 | 24195148 | 839.3 | 16.76 | A1A069 |
| | 16.90 | 14564632 | 803.4 | 16.94 | A1A014, A1A066 |
| | 17.02 | 30593126 | 841.3 | 17.06 | A1A021 |
| | 17.28 | 15214962 | 805.4 | 17.33 | A1A019 |
| Statistical analysis | The number of samples of Cal_UV_area | 96 | | | |
| | Median | 16433909 | | | |
| | Mean | 16610795 | | | |
| | Standard deviation | 6784277 | | | |
| | 75 percentile | 20759334 | | | |
| | 25 percentile | 12308457 | | | |
| | Upper boundary value | 33435649 | | | |
| | Lower boundary value | -367858 | | | |
| | The number of outliers | 1 | | | |
| | Maximum value | | | 43567424 | |
| | Minimum value | | | 5459982 | |

[Table 164]

| [Table AG-E-06] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| mixED10 | 6.39 | 25473116 | 764.4 | 6.42 | A3A026 |
| | 6.39 | 25473116 | 756.3 | 6.42 | A3A035 |

(continued)

[Table AG-E-06]

| Mixture No. | Retention time of UV peak (min) | Cal_UV_area | (M+H) observed at MS peak top | Retention time of MS peak (min) | No. of assigned compound |
|---|---|---|---|---|---|
| | 6.83 | 18036014 | 750.3 | 6.87 | A3A024 |
| | 6.83 | 18036014 | 768.3 | 6.86 | A3A029, A3A085 |
| | 7.13 | 17484436 | 794.4 | 7.16 | A3A040 |
| | 7.34 | 14498445 | 778.4 | 7.38 | A3A027, A3A028, A3A080 |
| | 7.34 | 14498445 | 762.4 | 7.38 | A3A074, A3A075 |
| | 7.34 | 14498445 | 762.4 | 7.35 | A3A074, A3A075 |
| | 7.34 | 14498445 | 754.3 | 7.42 | A3A084 |
| | 7.56 | 19932650 | 808.4 | 7.59 | A3A041, A3A081 |
| | 7.69 | 12538824 | 774.3 | 7.74 | A3A043 |
| | 7.76 | 16135441 | 810.3 | 7.79 | A3A032 |
| | 7.76 | 16135441 | 800.4 | 7.79 | A3A033 |
| | 7.76 | 16135441 | 748.3 | 7.85 | A3A072 |
| | 7.76 | 16135441 | 766.4 | 7.79 | A3A078 |
| | 7.94 | 11202737 | 780.4 | 7.97 | A3A031, A3A079, A3A092 |
| | 8.05 | 9219283 | 779.4 | 8.11 | A3A008, A3A054, A3A067 |
| | 8.05 | 9219283 | 793.4 | 8.08 | A3A017 |
| | 8.05 | 9219283 | 792.4 | 8.08 | A3A025, A3A089 |
| | 8.05 | 9219283 | 780.3 | 8.13 | A3A031, A3A079, A3A092 |
| | 8.22 | 8422062 | 749.4 | 8.27 | A3A001 |
| | 8.29 | 12703374 | 763.4 | 8.34 | A3A003 |
| | 8.29 | 12703374 | 776.4 | 8.34 | A3A076, A3A077 |
| | 8.45 | 15588431 | 809.4 | 8.47 | A3A009 |
| | 8.45 | 15588431 | 755.3 | 8.51 | A3A012 |
| | 8.45 | 15588431 | 818.3 | 8.45 | A3A037, A3A091 |
| | 8.45 | 15588431 | 806.4 | 8.49 | A3A090 |

(continued)

[Table AG-E-06]

| Mixture No. | Retention time of UV peak (min) | Cal_UV_area | (M+H) observed at MS peak top | Retention time of MS peak (min) | No. of assigned compound |
|---|---|---|---|---|---|
| | 8.53 | 8838063 | 767.4 | 8.57 | A3A006, A3A060 |
| | 8.58 | 16392433 | 772.4 | 8.62 | A3A093 |
| | 8.65 | 13610193 | 834.4 | 8.69 | A3A039 |
| | 8.73 | 17829832 | 798.4 | 8.77 | A3A082 |
| | 8.99 | 13246016 | 777.4 | 9.02 | A3A004, A3A005, A3A055 |
| | 8.99 | 13246016 | 761.4 | 9.04 | A3A049, A3A050 |
| | 9.07 | 12184194 | 773.4 | 9.11 | A3A020 |
| | 9.07 | 12184194 | 747.4 | 9.15 | A3A047 |
| | 9.20 | 10185116 | 807.4 | 9.27 | A3A018, A3A056 |
| | 9.20 | 10185116 | 761.4 | 9.22 | A3A049, A3A050 |
| | 9.31 | 12630108 | 816.4 | 9.34 | A3A086 |
| | 9.37 | 8375784.8 | 777.4 | 9.41 | A3A004, A3A005, A3A055 |
| | 9.37 | 8375784.8 | 807.4 | 9.39 | A3A018, A3A056 |
| | 9.37 | 8375784.8 | 778.4 | 9.45 | A3A027, A3A028, A3A080 |
| | 9.37 | 8375784.8 | 808.4 | 9.39 | A3A041, A3A081 |
| | 9.37 | 8375784.8 | 753.3 | 9.42 | A3A059 |
| | 9.43 | 9281853 | 765.4 | 9.47 | A3A053 |
| | 9.49 | 30725904 | 832.3 | 9.56 | A3A038, A3A088 |
| | 9.69 | 13184912 | 791.4 | 9.70 | A3A002, A3A064 |
| | 9.69 | 13184912 | 779.4 | 9.73 | A3A008, A3A054, A3A067 |
| | 9.84 | 13134785 | 799.4 | 9.86 | A3A010 |
| | 9.91 | 19156480 | 771.4 | 9.94 | A3A068 |
| | 10.06 | 10637488 | 805.4 | 10.09 | A3A065 |

(continued)

| [Table AG-E-06] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| | 10.28 | 14368371 | 775.4 | 10.33 | A3A051, A3A052 |
| | 10.37 | 10689766 | 817.3 | 10.41 | A3A014, A3A066 |
| | 10.58 | 13484412 | 833.3 | 10.62 | A3A016 |
| | 10.67 | 18025236 | 770.3 | 10.71 | A3A036 |
| | 10.74 | 12782068 | 797.4 | 10.77 | A3A057 |

[Table 165]

| | | | | | |
|---|---|---|---|---|---|
| | 10.97 | 13379735 | 778.4 | 11.01 | A3A027, A3A028, A3A080 |
| | 11.18 | 16950852 | 789.3 | 11.21 | A3A045, A3A048 |
| | 11.18 | 16950852 | 815.4 | 11.23 | A3A061 |
| | 11.38 | 15578374.67 | 831.3 | 11.44 | A3A015, A3A063 |
| | 11.38 | 15578374.67 | 792.4 | 11.38 | A3A025, A3A089 |
| | 11.38 | 15578374.67 | 782.4 | 11.41 | A3A030 |
| | 12.07 | 20211304 | 814.4 | 12.11 | A3A034 |
| | 12.84 | 19650880 | 832.3 | 12.89 | A3A038, A3A088 |
| | 13.10 | 35913156 | 768.3 | 13.17 | A3A029, A3A085 |
| | 13.22 | 26901160 | 788.4 | 13.25 | A3A022 |
| | 13.35 | 13941529 | 776.4 | 13.38 | A3A076, A3A077 |
| | 13.35 | 13941529 | 787.3 | 13.40 | A3A095 |
| | 13.65 | 21166714 | 780.4 | 13.69 | A3A031, A3A079, A3A092 |
| | 13.65 | 21166714 | 820.3 | 13.69 | A3A042 |
| | 13.77 | 12353684 | 769.3 | 13.81 | A3A013 |
| | 13.77 | 12353684 | 790.4 | 13.80 | A3A073 |
| | 13.97 | 19001492 | 781.3 | 14.00 | A3A007 |
| | 14.33 | 15077196 | 777.3 | 14.37 | A3A004, A3A005, A3A055 |
| | 14.33 | 15077196 | 812.4 | 14.38 | A3A083 |
| | 14.51 | 20511500 | 856.3 | 14.55 | A3A044 |
| | 14.72 | 8119551 | 791.4 | 14.77 | A3A002, A3A064 |
| | 14.92 | 18802878 | 830.3 | 14.96 | A3A087 |
| | 15.05 | 22231706 | 813.4 | 15.09 | A3A011 |
| | 15.17 | 18388590 | 831.3 | 15.24 | A3A015, A3A063 |
| | 15.17 | 18388590 | 896.4 | 15.21 | A3A096 |

(continued)

| | | | | | | |
|---|---|---|---|---|---|---|
| | | 15.32 | 20434604 | 786.3 | 15.37 | A3A070 |
| | | 15.65 | 11041447 | 897.4 | 15.70 | A3A023 |
| | | 15.65 | 11041447 | 898.3 | 15.68 | A3A046 |
| | | 15.90 | 13672987 | 767.3 | 15.97 | A3A006, A3A060 |
| | | 15.90 | 13672987 | 818.4 | 15.93 | A3A037, A3A091 |
| | | 16.02 | 13811971 | 779.3 | 16.07 | A3A008, A3A054, A3A067 |
| | | 16.27 | 27411232 | 855.3 | 16.31 | A3A021 |
| | | 16.42 | 14523381.33 | 819.4 | 16.50 | A3A019 |
| | | 16.42 | 14523381.33 | 775.4 | 16.45 | A3A051, A3A052 |
| | | 16.42 | 14523381.33 | 854.3 | 16.46 | A3A094 |
| | | 16.65 | 9352761 | 789.4 | 16.68 | A3A045, A3A048 |
| | | 17.00 | 15690817 | 811.4 | 17.04 | A3A058 |
| | | 17.00 | 15690817 | 829.3 | 17.04 | A3A062 |
| | | 17.30 | 25269282 | 895.4 | 17.34 | A3A071 |
| | | 17.89 | 18815194 | 853.4 | 17.92 | A3A069 |
| | | 18.19 | 12829847 | 817.4 | 18.23 | A3A014, A3A066 |
| Statistical analysis | The number of samples of Cal UV area | | 96 | | | |
| | Median | | 14498445 | | | |
| | Mean | | 15253746 | | | |
| | Standard deviation | | 5078034 | | | |
| | 75 percentile | | | | 17878683 | |
| | 25 percentile | | | | 12353684 | |
| | Upper boundary value | | | | 26166182 | |
| | Lower boundary value | | | | 4066186 | |
| | The number of outliers | | | | 4 | |
| | Maximum value | | | | 35913156 | |
| | Minimum value | | | | 8119551 | |

[Table 166]

| [Table AG-E-07] | | | | | |
|---|---|---|---|---|---|
| Mixture No. | Retention time of UV peak (min) | Cal UV area | (M+H) observed at MS peak top | Retention time of MS peak (min) | No. of assigned compound |
| mixED11 | 2.29 | 67481640 | 672.1 | 2.33 | A2I035 |
| | 2.63 | 35173516 | 680.2 | 2.66 | A2I026 |
| | 2.74 | 47866836 | 696.2 | 2.78 | A2I031, A2I079, A2I092 |

(continued)

[Table AG-E-07]

| Mixture No. | Retention time of UV peak (min) | Cal UV area | (M+H) observed at MS peak top | Retention time of MS peak (min) | No. of assigned compound |
|---|---|---|---|---|---|
| | 2.80 | 65691300 | 670.2 | 2.85 | A2I084 |
| | 2.97 | 32218582 | 666.2 | 3.00 | A2I024 |
| | 3.05 | 30888820 | 684.2 | 3.09 | A2I029, A2I085 |
| | 3.11 | 23932646 | 678.2 | 3.13 | A2I074, A2I075 |
| | 3.20 | 57277624 | 678.2 | 3.23 | A2I074, A2I075 |
| | 3.33 | 54913840 | 694.2 | 3.36 | A2I027, A2I028, A2I080 |
| | 3.33 | 54913840 | 726.2 | 3.38 | A2I032 |
| | 3.33 | 54913840 | 664.2 | 3.38 | A2I072 |
| | 3.50 | 59848748 | 682.2 | 3.54 | A2I078 |
| | 3.59 | 18223620 | 695.2 | 3.63 | A2I008, A2I054, A2I067 |
| | 3.59 | 18223620 | 694.2 | 3.63 | A2I027, A2I028, A2I080 |
| | 3.69 | 38645248 | 696.2 | 3.72 | A2I031, A2I079, A2I092 |
| | 3.77 | 36581256 | 710.2 | 3.81 | A2I040 |
| | 3.90 | 60760916 | 724.2 | 3.93 | A2I041, A2I081 |
| | 4.04 | 54101656 | 716.2 | 4.10 | A2I033 |
| | 4.04 | 54101656 | 692.2 | 4.05 | A2I076, A2I077 |
| | 4.22 | 66428768 | 708.2 | 4.25 | A2I025, A2I089 |
| | 4.29 | 31270938 | 724.2 | 4.32 | A2I041, A2I081 |
| | 4.35 | 30436712 | 690.2 | 4.39 | A2I043 |
| | 4.41 | 73266616 | 714.2 | 4.45 | A2I082 |
| | 4.71 | 42223020 | 665.2 | 4.76 | A2I001 |
| | 4.71 | 42223020 | 722.2 | 4.75 | A2I090 |
| | 4.82 | 31588062 | 734.2 | 4.86 | A2I037, A2I091 |
| | 4.88 | 70498288 | 671.1 | 4.92 | A2I012 |

(continued)

[Table AG-E-07]

| Mixture No. | Retention time of UV peak (min) | Cal UV area | (M+H) observed at MS peak top | Retention time of MS peak (min) | No. of assigned compound |
|---|---|---|---|---|---|
| | 5.01 | 69602032 | 663.2 | 5.06 | A2I047 |
| | 5.09 | 39055204 | 679.2 | 5.14 | A2I003 |
| | 5.09 | 39055204 | 683.2 | 5.16 | A2I006, A2I060 |
| | 5.09 | 39055204 | 750.2 | 5.13 | A2I039 |
| | 5.09 | 39055204 | 732.2 | 5.11 | A2I086 |
| | 5.17 | 48201132 | 686.1 | 5.25 | A2I036 |
| | 5.17 | 48201132 | 669.1 | 5.20 | A2I059 |
| | 5.33 | 45478552 | 748.2 | 5.38 | A2I038, A2I088 |
| | 5.33 | 45478552 | 677.2 | 5.36 | A2I049, A2I050 |
| | 5.38 | 29745146 | 725.2 | 5.43 | A2I009 |
| | 5.44 | 58539624 | 693.2 | 5.47 | A2I004, A2I005, A2I055 |
| | 5.44 | 58539624 | 694.2 | 5.51 | A2I027, A2I028, A2I080 |
| | 5.44 | 58539624 | 677.2 | 5.53 | A2I049, A2I050 |
| | 5.44 | 58539624 | 681.2 | 5.47 | A2I053 |
| | 5.67 | 53117676 | 695.2 | 5.70 | A2I008, A2I054, A2I067 |
| | 5.75 | 33561700 | 709.2 | 5.79 | A2I017 |
| | 5.85 | 49357472 | 723.2 | 5.89 | A2I018, A2I056 |
| | 5.85 | 49357472 | 689.2 | 5.92 | A2I020 |
| | 5.95 | 51319004 | 693.2 | 5.97 | A2I004, A2I005, A2I055 |
| | 5.95 | 51319004 | 698.1 | 6.00 | A2I030 |
| | 6.10 | 38244224 | 707.2 | 6.16 | A2I002, A2I064 |
| | 6.10 | 38244224 | 708.2 | 6.16 | A2I025, A2I089 |
| | 6.10 | 38244224 | 705.2 | 6.14 | A2I045, A2I048 |
| | 6.10 | 38244224 | 687.2 | 6.09 | A2I068 |

(continued)

| [Table AG-E-07] | | | | | |
|---|---|---|---|---|---|
| Mixture No. | Retention time of UV peak (min) | Cal UV area | (M+H) observed at MS peak top | Retention time of MS peak (min) | No. of assigned compound |
| | 6.10 | 38244224 | 688.2 | 6.09 | A2I093 |
| | 6.20 | 45932032 | 723.2 | 6.24 | A2I018, A2I056 |
| | 6.20 | 45932032 | 691.2 | 6.25 | A2I051, A2I052 |
| | 6.27 | 66431988 | 715.2 | 6.30 | A2I010 |

[Table 167]

| | | | | | |
|---|---|---|---|---|---|
| | 6.27 | 66431988 | 684.1 | 6.31 | A2I029, A2I085 |
| | 6.46 | 60512272 | 713.2 | 6.50 | A2I057 |
| | 6.46 | 60512272 | 692.2 | 6.49 | A2I076, A2I077 |
| | 6.51 | 51956008 | 721.2 | 6.55 | A2I065 |
| | 6.58 | 57807108 | 730.2 | 6.62 | A2I034 |
| | 6.85 | 35068084 | 733.2 | 6.88 | A2I014, A2I066 |
| | 6.94 | 61825204 | 696.2 | 6.97 | A2I031, A2I079, A2I092 |
| | 6.94 | 61825204 | 731.2 | 6.95 | A2I061 |
| | 6.94 | 61825204 | 706.2 | 6.99 | A2I073 |
| | 6.94 | 61825204 | 703.2 | 7.02 | A2I095 |
| | 7.07 | 40865944 | 749.2 | 7.11 | A2I016 |
| | 7.13 | 55828980 | 747.2 | 7.17 | A2I015, A2I063 |
| | 7.48 | 63452284 | 728.2 | 7.52 | A2I083 |
| | 7.70 | 55736076 | 748.1 | 7.74 | A2I038, A2I088 |
| | 8.07 | 64497252 | 814.2 | 8.11 | A2I046 |
| | 8.15 | 55553028 | 704.2 | 8.20 | A2I022 |
| | 8.15 | 55553028 | 736.2 | 8.18 | A2I042 |
| | 8.54 | 63208440 | 685.1 | 8.56 | A2I013 |
| | 8.54 | 63208440 | 746.2 | 8.58 | A2I087 |
| | 8.74 | 57364476 | 697.2 | 8.77 | A2I007 |
| | 8.94 | 60652298.67 | 693.2 | 8.98 | A2I004, A2I005, A2I055 |
| | 8.94 | 60652298.67 | 702.2 | 8.98 | A2I070 |
| | 8.94 | 60652298.67 | 812.2 | 8.94 | A2I096 |
| | 9.01 | 69047976 | 734.2 | 9.05 | A2I037, A2I091 |
| | 9.40 | 82785544 | 683.1 | 9.43 | A2I006, A2I060 |
| | 9.57 | 54788890.67 | 707.2 | 9.56 | A2I002, A2I064 |

(continued)

| | | | | | |
|---|---|---|---|---|---|
| | 9.57 | 54788890.67 | 695.2 | 9.60 | A2I008, A2I054, A2I067 |
| | 9.57 | 54788890.67 | 772.2 | 9.62 | A2I044 |
| | 9.68 | 76824648 | 729.2 | 9.72 | A2I011 |
| | 9.76 | 80226648 | 691.2 | 9.81 | A2I051, A2I052 |
| | 10.11 | 63105556 | 705.2 | 10.14 | A2I045, A2I048 |
| | 10.27 | 63868792 | 747.2 | 10.30 | A2I015, A2I063 |
| | 10.35 | 68423376 | 770.2 | 10.40 | A2I094 |
| | 10.44 | 72473392 | 727.2 | 10.48 | A2I058 |
| | 10.99 | 73222424 | 813.2 | 11.05 | A2I023 |
| | 10.99 | 73222424 | 745.2 | 11.03 | A2I062 |
| | 11.28 | 49932336 | 735.2 | 11.32 | A2I019 |
| | 11.58 | 72426304 | 771.2 | 11.62 | A2I021 |
| | 11.68 | 95247616 | 811.2 | 11.71 | A2I071 |
| | 12.01 | 76128288 | 733.2 | 12.06 | A2I014, A2I066 |
| | 12.27 | 83337712 | 769.2 | 12.30 | A2I069 |
| Statistical analysis | The number of samples of Cal_UV_area | 96 | | | |
| | Median | 55233434 | | | |
| | Mean | 53851907 | | | |
| | Standard deviation | 14855220 | | | |
| | 75 percentile | 63208440 | | | |
| | 25 percentile | 41883751 | | | |
| | Upper boundary value | 95195474 | | | |
| | Lower boundary value | 9896718 | | | |
| | The number of outliers | 1 | | | |
| | Maximum value | 95247616 | | | |
| | Minimum value | 18223620 | | | |

[Table 168]

[Table AG-E-08]

| Mixture No. | Retention time of UV peak (min) | Cal_UV_area | (M+H) observed at MS peak top | Retention time of MS peak (min) | No. of assigned compound |
|---|---|---|---|---|---|
| mixED12 | 3.76 | 48933224 | 714.2 | 3.79 | A2L035 |
| | 3.86 | 30790928 | 722.2 | 3.89 | A2L026 |
| | 3.86 | 30790928 | 738.3 | 3.94 | A2L031, A2L079, A2L092 |
| | 3.86 | 30790928 | 712.2 | 3.90 | A2L084 |

(continued)

[Table AG-E-08]

| Mixture No. | Retention time of UV peak (min) | Cal_UV_area | (M+H) observed at MS peak top | Retention time of MS peak (min) | No. of assigned compound |
|---|---|---|---|---|---|
| | 4.13 | 33332864 | 720.3 | 4.16 | A2L074, A2L075 |
| | 4.22 | 25094790 | 708.2 | 4.32 | A2L024 |
| | 4.22 | 25094790 | 736.2 | 4.26 | A2L027, A2L028, A2L080 |
| | 4.32 | 39966372 | 726.2 | 4.36 | A2L029, A2L085 |
| | 4.32 | 39966372 | 706.2 | 4.35 | A2L072 |
| | 4.38 | 24936008 | 768.3 | 4.41 | A2L032 |
| | 4.44 | 41272416 | 724.2 | 4.48 | A2L078 |
| | 4.61 | 28210136 | 738.3 | 4.64 | A2L031, A2L079, A2L092 |
| | 4.71 | 36824632 | 766.3 | 4.75 | A2L041, A2L081 |
| | 4.80 | 29561926 | 752.2 | 4.84 | A2L040 |
| | 4.85 | 28715998 | 736.3 | 4.88 | A2L027, A2L028, A2L080 |
| | 4.93 | 32435874 | 734.3 | 4.96 | A2L076, A2L077 |
| | 5.01 | 36561376 | 750.2 | 5.05 | A2L025, A2L089 |
| | 5.30 | 23505344 | 767.3 | 5.32 | A2L009 |
| | 5.30 | 23505344 | 758.3 | 5.38 | A2L033 |
| | 5.30 | 23505344 | 766.3 | 5.32 | A2L041, A2L081 |
| | 5.30 | 23505344 | 756.3 | 5.34 | A2L082 |
| | 5.46 | 38618804 | 764.3 | 5.50 | A2L090 |
| | 5.46 | 38618804 | 730.2 | 5.48 | A2L093 |
| | 5.87 | 31931268 | 707.2 | 5.91 | A2L001 |
| | 5.87 | 31931268 | 705.2 | 5.88 | A2L047 |
| | 5.93 | 33550906 | 774.2 | 5.96 | A2L086 |
| | 6.03 | 10467827 | 721.3 | 6.09 | A2L003 |
| | 6.03 | 10467827 | 711.2 | 6.07 | A2L059 |
| | 6.13 | 40049037.33 | 735.3 | 6.17 | A2L004, A2L005, A2L055 |
| | 6.13 | 40049037.33 | 713.2 | 6.16 | A2L012 |

(continued)

[Table AG-E-08]

| Mixture No. | Retention time of UV peak (min) | Cal_UV_area | (M+H) observed at MS peak top | Retention time of MS peak (min) | No. of assigned compound |
|---|---|---|---|---|---|
| | 6.13 | 40049037.33 | 790.2 | 6.20 | A2L038, A2L088 |
| | 6.23 | 28802058 | 725.2 | 6.30 | A2L006, A2L060 |
| | 6.23 | 28802058 | 719.3 | 6.25 | A2L049, A2L050 |
| | 6.23 | 28802058 | 723.3 | 6.26 | A2L053 |
| | 6.23 | 28802058 | 720.3 | 6.25 | A2L074, A2L075 |
| | 6.34 | 29349924 | 792.2 | 6.38 | A2L039 |
| | 6.47 | 31464386 | 737.3 | 6.51 | A2L008, A2L054, A2L067 |
| | 6.52 | 29595294 | 765.3 | 6.56 | A2L018, A2L056 |
| | 6.68 | 24423594 | 751.3 | 6.72 | A2L017 |
| | 6.77 | 26024412 | 749.3 | 6.80 | A2L002, A2L064 |
| | 6.81 | 34312620 | 729.2 | 6.84 | A2L068 |
| | 6.97 | 25462804 | 731.2 | 7.01 | A2L020 |
| | 6.97 | 25462804 | 728.2 | 7.00 | A2L036 |
| | 6.97 | 25462804 | 732.2 | 7.06 | A2L043 |
| | 6.97 | 25462804 | 733.3 | 7.05 | A2L051, A2L052 |
| | 7.13 | 15344350.67 | 735.3 | 7.16 | A2L004, A2L005, A2L055 |
| | 7.13 | 15344350.67 | 765.3 | 7.17 | A2L018, A2L056 |
| | 7.13 | 15344350.67 | 763.3 | 7.21 | A2L065 |
| | 7.20 | 67420944 | 736.2 | 7.24 | A2L027, A2L028, A2L080 |
| | 7.30 | 35784344 | 755.2 | 7.33 | A2L057 |
| | 7.55 | 27468000 | 757.2 | 7.58 | A2L010 |
| | 7.61 | 41892128 | 726.2 | 7.66 | A2L029, A2L085 |
| | 7.67 | 30957184 | 740.2 | 7.71 | A2L030 |
| | 7.67 | 30957184 | 747.2 | 7.70 | A2L045, A2L048 |

(continued)

| [Table AG-E-08] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| | 7.75 | 28762816 | 773.2 | 7.79 | A2L061 |

[Table 169]

| | | | | | |
|---|---|---|---|---|---|
| | 7.80 | 38770296 | 734.3 | 7.85 | A2L076, A2L077 |
| | 7.85 | 24480668 | 750.2 | 7.89 | A2L025, A2L089 |
| | 7.96 | 25931244 | 789.3 | 8.01 | A2L015, A2L063 |
| | 8.16 | 19612526 | 775.2 | 8.19 | A2L014, A2L066 |
| | 8.20 | 20183866 | 738.2 | 8.28 | A2L031, A2L079, A2L092 |
| | 8.20 | 20183866 | 745.3 | 8.24 | A2L095 |
| | 8.28 | 100559440 | 772.2 | 8.33 | A2L034 |
| | 8.35 | 23633182 | 791.2 | 8.42 | A2L016 |
| | 8.35 | 23633182 | 748.2 | 8.39 | A2L073 |
| | 8.79 | 46037464 | 770.3 | 8.83 | A2L083 |
| | 9.28 | 34349488 | 790.2 | 9.32 | A2L038, A2L088 |
| | 9.59 | 51052136 | 856.2 | 9.63 | A2L046 |
| | 9.67 | 39909620 | 746.2 | 9.71 | A2L022 |
| | 9.75 | 44861012 | 788.2 | 9.80 | A2L087 |
| | 9.82 | 35197748 | 778.3 | 9.86 | A2L042 |
| | 10.06 | 40547244 | 854.2 | 10.10 | A2L096 |
| | 10.13 | 36731184 | 727.2 | 10.17 | A2L013 |
| | 10.13 | 36731184 | 744.2 | 10.16 | A2L070 |
| | 10.30 | 26633317.33 | 739.3 | 10.36 | A2L007 |
| | 10.30 | 26633317.33 | 776.3 | 10.37 | A2L037, A2L091 |
| | 10.30 | 26633317.33 | 776.3 | 10.34 | A2L037, A2L091 |
| | 10.58 | 43202688 | 735.3 | 10.61 | A2L004, A2L005, A2L055 |
| | 10.63 | 53500208 | 725.2 | 10.67 | A2L006, A2L060 |
| | 10.78 | 19936300 | 737.3 | 10.81 | A2L008, A2L054, A2L067 |
| | 10.78 | 19936300 | 737.3 | 10.82 | A2L008, A2L054, A2L067 |
| | 11.03 | 42275100 | 814.2 | 11.09 | A2L044 |
| | 11.03 | 42275100 | 733.3 | 11.06 | A2L051,A2L052 |
| | 11.29 | 38083568 | 749.2 | 11.31 | A2L002, A2L064 |
| | 11.29 | 38083568 | 771.3 | 11.33 | A2L011 |
| | 11.48 | 32924176 | 747.3 | 11.54 | A2L045, A2L048 |
| | 11.48 | 32924176 | 812.2 | 11.51 | A2L094 |
| | 11.68 | 41059796 | 769.3 | 11.72 | A2L058 |

(continued)

| | | | | | |
|---|---|---|---|---|---|
| | 11.74 | 42044100 | 789.2 | 11.78 | A2L015, A2L063 |
| | 12.11 | 46845748 | 787.2 | 12.16 | A2L062 |
| | 12.37 | 48478844 | 855.2 | 12.41 | A2L023 |
| | 12.72 | 30367760 | 719.1 | 12.60 | A2L049, A2L050 |
| | 12.72 | 30367760 | 853.2 | 12.75 | A2L071 |
| | 12.83 | 27201032 | 777.3 | 12.88 | A2L019 |
| | 12.97 | 55412296 | 813.2 | 13.00 | A2L021 |
| | 13.17 | 38915592 | 775.3 | 13.21 | A2L014, A2L066 |
| | 13.31 | 50208356 | 811.2 | 13.35 | A2L069 |
| Statistical analysis | The number of samples of Cal_UV_area | 96 | | | |
| | Median | 30957184 | | | |
| | Mean | 33238415 | | | |
| | Standard deviation | 12012103 | | | |
| | 75 percentile | 39923808 | | | |
| | 25 percentile | 25462804 | | | |
| | Upper boundary value | 61615314 | | | |
| | Lower boundary value | 3771298 | | | |
| | The number of outliers | 2 | | | |
| | Maximum value | 100559440 | | | |
| | Minimum value | 10467827 | | | |

[Table 170]

| [Table AG-E-09] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| mixED13 | 4.20 | 60839392 | 754.2 | 4.23 | A2N035 |
| | 4.32 | 60541808 | 762.2 | 4.34 | A2N026 |
| | 4.68 | 60283288 | 752.1 | 4.72 | A2N084 |
| | 4.77 | 27993150 | 748.2 | 4.78 | A2N024 |
| | 4.77 | 27993150 | 766.2 | 4.80 | A2N029, A2N085 |
| | 4.77 | 27993150 | 808.3 | 4.81 | A2N032 |
| | 4.77 | 27993150 | 760.2 | 4.80 | A2N074, A2N075 |
| | 4.87 | 55294972 | 760.2 | 4.91 | A2N074, A2N075 |

(continued)

[Table AG-E-09]

| Mixture No. | Retention time of UV peak (min) | Cal_UV_area | (M+H) observed at MS peak top | Retention time of MS peak (min) | No. of assigned compound |
|---|---|---|---|---|---|
| | 4.93 | 62404008 | 776.2 | 4.96 | A2N027, A2N028, A2N080 |
| | 5.13 | 59299088 | 746.2 | 5.17 | A2N072 |
| | 5.22 | 45418828 | 792.3 | 5.27 | A2N040 |
| | 5.22 | 45418828 | 764.2 | 5.27 | A2N078 |
| | 5.27 | 43612960 | 776.2 | 5.31 | A2N027, A2N028, A2N080 |
| | 5.38 | 49254024 | 778.2 | 5.41 | A2N031, A2N079, A2N092 |
| | 5.38 | 49254024 | 806.2 | 5.42 | A2N041, A2N081 |
| | 5.70 | 37343796 | 807.2 | 5.73 | A2N009 |
| | 5.70 | 37343796 | 790.3 | 5.75 | A2N025, A2N089 |
| | 5.70 | 37343796 | 806.2 | 5.74 | A2N041, A2N081 |
| | 5.70 | 37343796 | 774.2 | 5.72 | A2N076, A2N077 |
| | 5.75 | 47417692 | 798.2 | 5.79 | A2N033 |
| | 5.91 | 32045940 | 772.2 | 5.94 | A2N043 |
| | 6.09 | 52848072 | 796.3 | 6.13 | A2N082 |
| | 6.14 | 55612840 | 804.3 | 6.19 | A2N090 |
| | 6.14 | 55612840 | 770.2 | 6.22 | A2N093 |
| | 6.31 | 56229048 | 747.2 | 6.35 | A2N001 |
| | 6.48 | 25412380 | 761.2 | 6.51 | A2N003 |
| | 6.55 | 44531464 | 753.1 | 6.59 | A2N012 |
| | 6.55 | 44531464 | 816.2 | 6.57 | A2N037, A2N091 |
| | 6.55 | 44531464 | 745.2 | 6.63 | A2N047 |
| | 6.63 | 22597136 | 765.2 | 6.72 | A2N006, A2N060 |
| | 6.71 | 52155760 | 759.3 | 6.75 | A2N049, A2N050 |
| | 6.71 | 52155760 | 814.2 | 6.76 | A2N086 |
| | 6.77 | 42446704 | 775.2 | 6.79 | A2N004, A2N005, A2N055 |

(continued)

[Table AG-E-09]

| Mixture No. | Retention time of UV peak (min) | Cal_UV_area | (M+H) observed at MS peak top | Retention time of MS peak (min) | No. of assigned compound |
|---|---|---|---|---|---|
| | 6.77 | 42446704 | 777.2 | 6.79 | A2N008, A2N054, A2N067 |
| | 6.77 | 42446704 | 832.2 | 6.81 | A2N039 |
| | 6.77 | 42446704 | 751.2 | 6.80 | A2N059 |
| | 6.87 | 71991288 | 759.2 | 6.92 | A2N049, A2N050 |
| | 6.94 | 52284492 | 830.2 | 6.98 | A2N038, A2N088 |
| | 6.94 | 52284492 | 763.2 | 6.98 | A2N053 |
| | 7.11 | 46495044 | 791.3 | 7.14 | A2N017 |
| | 7.11 | 46495044 | 805.2 | 7.15 | A2N018, A2N056 |
| | 7.16 | 57097712 | 777.2 | 7.21 | A2N008, A2N054, A2N067 |
| | 7.40 | 46717600 | 789.3 | 7.45 | A2N002, A2N064 |
| | 7.40 | 46717600 | 771.2 | 7.44 | A2N020 |
| | 7.40 | 46717600 | 768.2 | 7.41 | A2N036 |
| | 7.52 | 40382986.67 | 775.2 | 7.56 | A2N004, A2N005, A2N055 |
| | 7.52 | 40382986.67 | 805.3 | 7.57 | A2N018, A2N056 |
| | 7.52 | 40382986.67 | 769.2 | 7.55 | A2N068 |
| | 7.60 | 41427080 | 776.2 | 7.64 | A2N027, A2N028, A2N080 |
| | 7.72 | 61014260 | 773.2 | 7.76 | A2N051, A2N052 |
| | 7.81 | 54312652 | 803.2 | 7.85 | A2N065 |
| | 7.93 | 42104600 | 797.2 | 7.97 | A2N010 |
| | 8.06 | 47985781.33 | 780.2 | 8.10 | A2N030 |
| | 8.06 | 47985781.33 | 787.2 | 8.12 | A2N045, A2N048 |
| | 8.06 | 47985781.33 | 795.2 | 8.06 | A2N057 |

[Table 171]

| | | | | | |
|---|---|---|---|---|---|
| | 8.34 | 43730388 | 790.2 | 8.36 | A2N025, A2N089 |

(continued)

| | | | | | | |
|---|---|---|---|---|---|---|
| | | 8.51 | 44470624 | 815.2 | 8.59 | A2N014, A2N066 |
| | | 8.51 | 44470624 | 766.2 | 8.55 | A2N029, A2N085 |
| | | 8.51 | 44470624 | 813.2 | 8.53 | A2N061 |
| | | 8.68 | 50848122.67 | 829.2 | 8.73 | A2N015, A2N063 |
| | | 8.68 | 50848122.67 | 812.2 | 8.67 | A2N034 |
| | | 8.68 | 50848122.67 | 774.2 | 8.71 | A2N076, A2N077 |
| | | 8.75 | 30903284 | 831.2 | 8.79 | A2N016 |
| | | 9.02 | 46739080 | 785.2 | 9.07 | A2N095 |
| | | 9.09 | 36251308 | 778.2 | 9.12 | A2N031, A2N079, A2N092 |
| | | 9.09 | 36251308 | 778.2 | 9.15 | A2N031, A2N079, A2N092 |
| | | 9.17 | 35427656 | 788.2 | 9.21 | A2N073 |
| | | 9.59 | 54633300 | 830.2 | 9.64 | A2N038, A2N088 |
| | | 9.59 | 54633300 | 810.2 | 9.63 | A2N083 |
| | | 9.88 | 57173064 | 896.2 | 9.92 | A2N046 |
| | | 9.97 | 56022196 | 786.2 | 10.01 | A2N022 |
| | | 10.09 | 47011272 | 818.3 | 10.12 | A2N042 |
| | | 10.37 | 59059688 | 767.1 | 10.41 | A2N013 |
| | | 10.49 | 62667596 | 828.2 | 10.52 | A2N087 |
| | | 10.56 | 50469040 | 779.2 | 10.60 | A2N007 |
| | | 10.77 | 48238661.33 | 775.2 | 10.81 | A2N004, A2N005, A2N055 |
| | | 10.77 | 48238661.33 | 784.2 | 10.86 | A2N070 |
| | | 10.77 | 48238661.33 | 894.2 | 10.81 | A2N096 |
| | | 11.02 | 49699804 | 816.3 | 11.05 | A2N037, A2N091 |
| | | 11.27 | 51487320 | 765.2 | 11.32 | A2N006, A2N060 |
| | | 11.27 | 51487320 | 854.2 | 11.29 | A2N044 |
| | | 11.45 | 56752812 | 777.2 | 11.45 | A2N008, A2N054, A2N067 |
| | | 11.45 | 56752812 | 811.2 | 11.49 | A2N011 |
| | | 11.59 | 26328638 | 789.2 | 11.63 | A2N002, A2N064 |
| | | 11.64 | 57896964 | 773.2 | 11.68 | A2N051, A2N052 |
| | | 11.90 | 45409956 | 829.2 | 11.94 | A2N015, A2N063 |
| | | 12.05 | 48921892 | 787.2 | 12.09 | A2N045, A2N048 |
| | | 12.05 | 48921892 | 852.2 | 12.10 | A2N094 |
| | | 12.24 | 62763756 | 809.2 | 12.28 | A2N058 |
| | | 12.49 | 52923748 | 895.2 | 12.53 | A2N023 |
| | | 12.64 | 64631112 | 827.2 | 12.68 | A2N062 |
| | | 12.89 | 42498460 | 817.3 | 12.93 | A2N019 |

(continued)

| | | | | | |
|---|---|---|---|---|---|
| | 13.04 | 66302240 | 853.2 | 13.07 | A2N021 |
| | 13.18 | 72510888 | 893.2 | 13.23 | A2N071 |
| | 13.63 | 65019676 | 815.3 | 13.68 | A2N014, A2N066 |
| | 13.73 | 69645080 | 851.2 | 13.77 | A2N069 |
| Statistical analysis | The number of samples of Cal_UV_area | 96 | | | |
| | Median | 48112221 | | | |
| | Mean | 48237526 | | | |
| | Standard deviation | 10364636 | | | |
| | 75 percentile | 55374439 | | | |
| | 25 percentile | 42446704 | | | |
| | Upper boundary value | 74766042 | | | |
| | Lower boundary value | 23055102 | | | |
| | The number of outliers | 1 | | | |
| | Maximum value | 72510888 | | | |
| | Minimum value | 22597136 | | | |

[Table 172]

[Table AG-E-10]

| Mixture No. | Retention time of UV peak (min) | Cal_UV_area | (M+H) observed at MS peak top | Retention time of MS peak (min) | No. of assigned compound |
|---|---|---|---|---|---|
| mixED14 | 4.41 | 66178900 | 779.1 | 4.45 | A2P035 |
| | 4.53 | 23067820 | 787.2 | 4.57 | A2P026 |
| | 4.56 | 45554908 | 803.2 | 4.60 | A2P031, A2P079, A2P092 |
| | 4.97 | 30779986 | 773.2 | 4.96 | A2P024 |
| | 4.97 | 30779986 | 791.2 | 5.03 | A2P029, A2P085 |
| | 4.97 | 30779986 | 833.2 | 5.04 | A2P032 |
| | 4.97 | 30779986 | 777.2 | 5.00 | A2P084 |
| | 5.07 | 23867112 | 785.2 | 5.10 | A2P074, A2P075 |
| | 5.16 | 61739496 | 785.2 | 5.19 | A2P074, A2P075 |
| | 5.23 | 31020216 | 802.2 | 5.28 | A2P008, A2P054, A2P067 |

(continued)

[Table AG-E-10]

| Mixture No. | Retention time of UV peak (min) | Cal_UV_area | (M+H) observed at MS peak top | Retention time of MS peak (min) | No. of assigned compound |
|---|---|---|---|---|---|
| | 5.23 | 31020216 | 801.2 | 5.27 | A2P027, A2P028, A2P080 |
| | 5.39 | 48819784 | 771.2 | 5.43 | A2P072 |
| | 5.47 | 33645524 | 801.2 | 5.50 | A2P027, A2P028, A2P080 |
| | 5.47 | 33645524 | 817.2 | 5.50 | A2P040 |
| | 5.51 | 56040428 | 789.2 | 5.55 | A2P078 |
| | 5.68 | 90396760 | 831.3 | 5.71 | A2P041, A2P081 |
| | 5.95 | 24674830 | 832.2 | 5.98 | A2P009 |
| | 5.95 | 24674830 | 823.2 | 5.98 | A2P033 |
| | 5.95 | 24674830 | 831.2 | 5.98 | A2P041, A2P081 |
| | 5.95 | 24674830 | 799.2 | 6.01 | A2P076, A2P077 |
| | 6.00 | 70135872 | 815.3 | 6.04 | A2P025, A2P089 |
| | 6.11 | 20545866 | 797.2 | 6.15 | A2P043 |
| | 6.36 | 27105652 | 822.1 | 6.39 | A2P010 |
| | 6.36 | 27105652 | 821.2 | 6.39 | A2P082 |
| | 6.45 | 37489764 | 829.2 | 6.48 | A2P090 |
| | 6.45 | 37489764 | 795.2 | 6.50 | A2P093 |
| | 6.56 | 47517988 | 772.2 | 6.58 | A2P001 |
| | 6.77 | 38707213.33 | 786.2 | 6.77 | A2P003 |
| | 6.77 | 38707213.33 | 778.2 | 6.82 | A2P012 |
| | 6.77 | 38707213.33 | 841.2 | 6.77 | A2P037, A2P091 |
| | 6.84 | 43585240 | 770.2 | 6.87 | A2P047 |
| | 6.91 | 46757824 | 790.2 | 6.95 | A2P006, A2P060 |
| | 6.99 | 22520850.67 | 857.2 | 7.01 | A2P039 |
| | 6.99 | 22520850.67 | 784.3 | 7.06 | A2P049, A2P050 |
| | 6.99 | 22520850.67 | 839.2 | 7.03 | A2P086 |
| | 7.03 | 31106152 | 776.2 | 7.09 | A2P059 |

(continued)

| [Table AG-E-10] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| | 7.08 | 96168088 | 800.2 | 7.12 | A2P004, A2P005, A2P055 |
| | 7.23 | 32768796 | 856.2 | 7.27 | A2P016 |
| | 7.23 | 32768796 | 855.2 | 7.25 | A2P038, A2P088 |
| | 7.23 | 32768796 | 784.2 | 7.23 | A2P049, A2P050 |
| | 7.23 | 32768796 | 788.2 | 7.28 | A2P053 |
| | 7.37 | 26748314 | 816.2 | 7.40 | A2P017 |
| | 7.45 | 41814196 | 802.2 | 7.49 | A2P008, A2P054, A2P067 |
| | 7.45 | 41814196 | 830.2 | 7.48 | A2P018, A2P056 |
| | 7.51 | 40994660 | 793.1 | 7.55 | A2P036 |
| | 7.60 | 33927812 | 796.2 | 7.64 | A2P020 |
| | 7.74 | 55500188 | 800.2 | 7.75 | A2P004, A2P005, A2P055 |
| | 7.74 | 55500188 | 801.2 | 7.79 | A2P027, A2P028, A2P080 |
| | 7.80 | 36060700 | 830.2 | 7.85 | A2P018, A2P056 |
| | 7.80 | 36060700 | 794.2 | 7.84 | A2P068 |
| | 8.01 | 45574920 | 798.2 | 8.05 | A2P051, A2P052 |
| | 8.15 | 64999540 | 828.3 | 8.19 | A2P065 |
| | 8.23 | 42959384 | 805.2 | 8.26 | A2P030 |
| | 8.23 | 42959384 | 812.2 | 8.28 | A2P045, A2P048 |
| | 8.31 | 44899832 | 820.1 | 8.35 | A2P057 |

[Table 173]

| | | | | | | |
|---|---|---|---|---|---|---|
| | | 8.50 | 36311896 | 815.2 | 8.54 | A2P025, A2P089 |
| | | 8.82 | 56789808 | 840.2 | 8.78 | A2P014, A2P066 |
| | | 8.82 | 56789808 | 791.2 | 8.88 | A2P029, A2P085 |
| | | 8.82 | 56789808 | 837.2 | 8.83 | A2P034 |
| | | 8.96 | 62762448 | 854.2 | 8.99 | A2P015, A2P063 |
| | | 8.96 | 62762448 | 799.2 | 9.03 | A2P076, A2P077 |
| | | 9.35 | 46380792 | 810.2 | 9.39 | A2P095 |
| | | 9.41 | 24824284 | 803.2 | 9.46 | A2P031, A2P079, A2P092 |
| | | 9.41 | 24824284 | 803.2 | 9.48 | A2P031, A2P079, A2P092 |
| | | 9.47 | 19893666 | 814.2 | 9.52 | A2P002, A2P064 |
| | | 9.47 | 19893666 | 813.2 | 9.50 | A2P073 |
| | | 9.76 | 48705348 | 855.2 | 9.80 | A2P038, A2P088 |
| | | 9.90 | 25688952 | 838.1 | 9.95 | A2P061 |
| | | 9.90 | 25688952 | 835.2 | 9.93 | A2P083 |
| | | 10.04 | 43584844 | 921.2 | 10.08 | A2P046 |
| | | 10.14 | 57414760 | 811.2 | 10.18 | A2P022 |
| | | 10.24 | 55198448 | 843.3 | 10.29 | A2P042 |
| | | 10.52 | 42976804 | 792.1 | 10.55 | A2P013 |
| | | 10.72 | 40223052 | 804.2 | 10.76 | A2P007 |
| | | 10.77 | 52700484 | 853.2 | 10.81 | A2P087 |
| | | 10.93 | 40899124 | 800.2 | 10.98 | A2P004, A2P005, A2P055 |
| | | 11.03 | 51500412 | 919.2 | 11.07 | A2P096 |
| | | 11.12 | 56163312 | 809.2 | 11.15 | A2P070 |
| | | 11.30 | 45874840 | 841.3 | 11.33 | A2P037, A2P091 |
| | | 11.43 | 53897728 | 879.2 | 11.47 | A2P044 |
| | | 11.57 | 52791644 | 790.1 | 11.62 | A2P006, A2P060 |
| | | 11.63 | 46195040 | 836.2 | 11.68 | A2P011 |
| | | 11.71 | 57262520 | 802.2 | 11.75 | A2P008, A2P054, A2P067 |
| | | 11.83 | 27168948 | 814.2 | 11.87 | A2P002, A2P064 |
| | | 11.93 | 55257724 | 798.2 | 11.97 | A2P051, A2P052 |
| | | 12.07 | 44237484 | 854.1 | 12.10 | A2P015, A2P063 |
| | | 12.33 | 47630616 | 812.2 | 12.34 | A2P045, A2P048 |
| | | 12.33 | 47630616 | 877.2 | 12.38 | A2P094 |
| | | 12.53 | 51293644 | 834.2 | 12.57 | A2P058 |

(continued)

| | | | | | |
|---|---|---|---|---|---|
| | 12.68 | 53673796 | 920.2 | 12.71 | A2P023 |
| | 12.91 | 43548340 | 852.2 | 12.96 | A2P062 |
| | 13.07 | 38310780 | 842.3 | 13.12 | A2P019 |
| | 13.23 | 58541928 | 878.2 | 13.26 | A2P021 |
| | 13.45 | 58667924 | 918.2 | 13.49 | A2P071 |
| | 13.90 | 42480884 | 840.3 | 13.95 | A2P014, A2P066 |
| | 14.00 | 52490796 | 876.2 | 14.04 | A2P069 |
| Statistical analysis | The number of samples of Cal_UV_area | 96 | | | |
| | Median | 42147540 | | | |
| | Mean | 42313749 | | | |
| | Standard deviation | 14579283 | | | |
| | 75 percentile | 52723274 | | | |
| | 25 percentile | 30960159 | | | |
| | Upper boundary value | 85367947 | | | |
| | Lower boundary value | -1684515 | | | |
| | The number of outliers | 2 | | | |
| | Maximum value | 96168088 | | | |
| | Minimum value | 19893666 | | | |

[Table 174]

| [Table AG-E-11] | | | | | |
|---|---|---|---|---|---|
| Mixture No. | Retention time of UV peak (min) | Cal_UV_area | (M+H) observed at MS peak top | Retention time of MS peak (min) | No. of assigned compound |
| mixED15 | 7.00 | 20096332 | 814.4 | 7.03 | A2A026 |
| | 7.00 | 20096332 | 828.3 | 7.08 | A2A027, A2A028, A2A080 |
| | 7.08 | 105088824 | 812.3 | 7.12 | A2A074, A2A075 |
| | 7.14 | 35874948 | 860.3 | 7.18 | A2A032 |
| | 7.25 | 61543456 | 804.3 | 7.28 | A2A084 |
| | 7.41 | 35498429.33 | 859.3 | 7.46 | A2A009 |
| | 7.41 | 35498429.33 | 806.3 | 7.46 | A2A035 |
| | 7.41 | 35498429.33 | 858.4 | 7.44 | A2A041, A2A081 |
| | 7.60 | 59436680 | 816.3 | 7.64 | A2A078 |
| | 7.66 | 52311000 | 798.3 | 7.69 | A2A072 |

(continued)

[Table AG-E-11]

| Mixture No. | Retention time of UV peak (min) | Cal_UV_area | (M+H) observed at MS peak top | Retention time of MS peak (min) | No. of assigned compound |
|---|---|---|---|---|---|
| | 7.75 | 31618300 | 818.3 | 7.79 | A2A029, A2A085 |
| | 7.75 | 31618300 | 830.3 | 7.78 | A2A031, A2A079, A2A092 |
| | 7.79 | 104168280 | 844.3 | 7.86 | A2A040 |
| | 7.88 | 27155970 | 800.3 | 7.92 | A2A024 |
| | 8.11 | 69074896 | 826.3 | 8.15 | A2A076, A2A077 |
| | 8.20 | 40641164 | 858.3 | 8.27 | A2A041, A2A081 |
| | 8.20 | 40641164 | 856.4 | 8.22 | A2A090 |
| | 8.36 | 19989150 | 829.3 | 8.38 | A2A008, A2A054, A2A067 |
| | 8.36 | 19989150 | 828.3 | 8.40 | A2A027, A2A028, A2A080 |
| | 8.42 | 31731016 | 829.3 | 8.49 | A2A008, A2A054, A2A067 |
| | 8.42 | 31731016 | 822.3 | 8.45 | A2A093 |
| | 8.57 | 57948744 | 827.3 | 8.62 | A2A004, A2A005, A2A055 |
| | 8.57 | 57948744 | 848.3 | 8.61 | A2A082 |
| | 8.65 | 42369420 | 824.3 | 8.71 | A2A043 |
| | 8.65 | 42369420 | 811.4 | 8.68 | A2A049, A2A050 |
| | 8.85 | 29076755.2 | 813.3 | 8.90 | A2A003 |
| | 8.85 | 29076755.2 | 857.4 | 8.93 | A2A018, A2A056 |
| | 8.85 | 29076755.2 | 797.3 | 8.89 | A2A047 |
| | 8.85 | 29076755.2 | 811.4 | 8.88 | A2A049, A2A050 |
| | 8.85 | 29076755.2 | 812.3 | 8.93 | A2A074, A2A075 |
| | 8.96 | 40977020 | 850.3 | 9.00 | A2A033 |
| | 9.18 | 37345096 | 799.3 | 9.21 | A2A001 |
| | 9.18 | 37345096 | 815.4 | 9.24 | A2A053 |

(continued)

[Table AG-E-11]

| Mixture No. | Retention time of UV peak (min) | Cal_UV_area | (M+H) observed at MS peak top | Retention time of MS peak (min) | No. of assigned compound |
|---|---|---|---|---|---|
| | 9.18 | 37345096 | 803.3 | 9.22 | A2A059 |
| | 9.18 | 37345096 | 866.3 | 9.22 | A2A086 |
| | 9.33 | 45683120 | 841.4 | 9.36 | A2A002, A2A064 |
| | 9.40 | 87523968 | 882.3 | 9.42 | A2A038, A2A088 |
| | 9.48 | 32803066 | 843.4 | 9.51 | A2A017 |
| | 9.55 | 30577822 | 817.3 | 9.59 | A2A006, A2A060 |
| | 9.63 | 58607452 | 805.3 | 9.67 | A2A012 |
| | 9.71 | 38832554.67 | 868.3 | 9.74 | A2A037, A2A091 |
| | 9.71 | 38832554.67 | 855.4 | 9.77 | A2A065 |
| | 9.71 | 38832554.67 | 821.3 | 9.76 | A2A068 |
| | 9.93 | 31842692 | 857.4 | 9.97 | A2A018, A2A056 |
| | 9.93 | 31842692 | 884.3 | 9.97 | A2A039 |
| | 10.00 | 54683512 | 825.4 | 10.04 | A2A051, A2A052 |
| | 10.17 | 34565112 | 823.3 | 10.22 | A2A020 |
| | 10.49 | 41297356 | 827.3 | 10.54 | A2A004, A2A005, A2A055 |
| | 10.49 | 41297356 | 847.3 | 10.53 | A2A057 |
| | 10.99 | 36709884 | 865.3 | 11.04 | A2A061 |
| | 11.17 | 40531116 | 849.3 | 11.20 | A2A010 |
| | 11.17 | 40531116 | 881.3 | 11.22 | A2A015, A2A063 |
| | 11.34 | 55694496 | 820.1 | 11.37 | A2A036 |
| | 11.57 | 41086072 | 828.3 | 11.61 | A2A027, A2A028, A2A080 |
| | 11.72 | 40158764 | 839.3 | 11.75 | A2A045, A2A048 |

[Table 175]

| | | | | | |
|---|---|---|---|---|---|
| | 11.79 | 28599920 | 867.3 | 11.83 | A2A014, A2A066 |
| | 11.91 | 60295828 | 818.3 | 11.94 | A2A029, A2A085 |
| | 11.91 | 60295828 | 832.3 | 11.94 | A2A030 |

(continued)

| | | | | | | |
|---|---|---|---|---|---|---|
| | | 11.99 | 30269572 | 883.3 | 12.03 | A2A016 |
| | | 12.09 | 59707012 | 826.3 | 12.13 | A2A076, A2A077 |
| | | 12.20 | 44148692 | 837.3 | 12.24 | A2A095 |
| | | 12.28 | 41496656 | 842.3 | 12.32 | A2A025, A2A089 |
| | | 12.40 | 43834256 | 830.3 | 12.45 | A2A031, A2A079, A2A092 |
| | | 12.40 | 43834256 | 830.3 | 12.46 | A2A031, A2A079, A2A092 |
| | | 12.51 | 68983064 | 864.3 | 12.55 | A2A034 |
| | | 12.59 | 37311000 | 840.3 | 12.64 | A2A073 |
| | | 12.95 | 88810584 | 862.3 | 12.98 | A2A083 |
| | | 13.17 | 59470480 | 882.3 | 13.21 | A2A038, A2A088 |
| | | 13.37 | 50377560 | 948.3 | 13.40 | A2A046 |
| | | 13.51 | 45587716 | 838.3 | 13.55 | A2A022 |
| | | 13.59 | 70875816 | 880.3 | 13.63 | A2A087 |
| | | 13.80 | 36270576 | 947.3 | 13.84 | A2A023 |
| | | 13.80 | 36270576 | 946.4 | 13.84 | A2A096 |
| | | 13.90 | 61671256 | 870.4 | 13.93 | A2A042 |
| | | 13.90 | 61671256 | 836.3 | 13.96 | A2A070 |
| | | 14.01 | 54498220 | 819.2 | 14.05 | A2A013 |
| | | 14.15 | 54529500 | 831.3 | 14.19 | A2A007 |
| | | 14.32 | 71369232 | 868.4 | 14.36 | A2A037, A2A091 |
| | | 14.42 | 58971688 | 827.3 | 14.50 | A2A004, A2A005, A2A055 |
| | | 14.42 | 58971688 | 817.3 | 14.47 | A2A006, A2A060 |
| | | 14.53 | 72984120 | 829.3 | 14.54 | A2A008, A2A054, A2A067 |
| | | 14.60 | 37894344 | 906.3 | 14.63 | A2A044 |
| | | 14.83 | 61425944 | 825.3 | 14.87 | A2A051, A2A052 |
| | | 14.96 | 56721096 | 904.3 | 15.01 | A2A094 |
| | | 15.05 | 52770188 | 863.3 | 15.08 | A2A011 |
| | | 15.20 | 50659716 | 881.3 | 15.23 | A2A015, A2A063 |
| | | 15.30 | 28545517.33 | 841.4 | 15.36 | A2A002, A2A064 |
| | | 15.30 | 28545517.33 | 842.3 | 15.34 | A2A025, A2A089 |
| | | 15.30 | 28545517.33 | 839.3 | 15.35 | A2A045, A2A048 |
| | | 15.37 | 73282864 | 861.4 | 15.41 | A2A058 |
| | | 15.51 | 69303744 | 879.3 | 15.55 | A2A062 |
| | | 15.88 | 91924456 | 945.4 | 15.92 | A2A071 |
| | | 16.12 | 66314372 | 905.3 | 16.16 | A2A021 |
| | | 16.27 | 42635920 | 869.4 | 16.30 | A2A019 |

(continued)

| | | | | | |
|---|---|---|---|---|---|
| | 16.40 | 76037944 | 903.3 | 16.44 | A2A069 |
| | 16.55 | 57294200 | 867.4 | 16.58 | A2A014, A2A066 |
| Statistical analysis | The number of samples of Cal_UV_area | 96 | | | |
| | Median | 41297356 | | | |
| | Mean | 47193955 | | | |
| | Standard deviation | 17906715 | | | |
| | 75 percentile | 58971688 | | | |
| | 25 percentile | 35265100 | | | |
| | Upper boundary value | 94531570 | | | |
| | Lower boundary value | -294782 | | | |
| | The number of outliers | 2 | | | |
| | Maximum value | 105088824 | | | |
| | Minimum value | 19989150 | | | |

[Table 176]

[Table AG-E-12]

| Mixture No. | Retention time of UV peak (min) | Cal UV area | (M+H) observed at MS peak top | Retention time of MS peak (min) | No. of assigned compound |
|---|---|---|---|---|---|
| mixED16 | 6.98 | 23707672 | 954.2 | 7.03 | A2Q031, A2Q079, A2Q092 |
| | 7.10 | 17014196 | 938.3 | 7.13 | A2Q026 |
| | 7.23 | 36049965.33 | 952.3 | 7.26 | A2Q027, A2Q028, A2Q080 |
| | 7.23 | 36049965.33 | 936.3 | 7.31 | A2Q074, A2Q075 |
| | 7.23 | 36049965.33 | 936.3 | 7.27 | A2Q074, A2Q075 |
| | 7.34 | 13205205 | 929.2 | 7.36 | A2Q012 |
| | 7.34 | 13205205 | 928.2 | 7.37 | A2Q084 |
| | 7.40 | 40022696 | 930.2 | 7.43 | A2Q035 |
| | 7.54 | 39397204 | 982.2 | 7.57 | A2Q041, A2Q081 |
| | 7.69 | 18400370 | 943.1 | 7.69 | A2Q013 |
| | 7.69 | 18400370 | 942.2 | 7.74 | A2Q029, A2Q085 |
| | 7.69 | 18400370 | 922.3 | 7.72 | A2Q072 |
| | 7.69 | 18400370 | 940.3 | 7.71 | A2Q078 |

(continued)

[Table AG-E-12]

| Mixture No. | Retention time of UV peak (min) | Cal UV area | (M+H) observed at MS peak top | Retention time of MS peak (min) | No. of assigned compound |
|---|---|---|---|---|---|
| | 7.79 | 19967706.67 | 924.2 | 7.81 | A2Q024 |
| | 7.79 | 19967706.67 | 954.3 | 7.82 | A2Q031, A2Q079, A2Q092 |
| | 7.79 | 19967706.67 | 968.4 | 7.85 | A2Q040 |
| | 7.89 | 17128012 | 967.3 | 7.93 | A2Q017 |
| | 7.89 | 17128012 | 966.3 | 7.92 | A2Q025, A2Q089 |
| | 8.12 | 25956676 | 950.3 | 8.16 | A2Q076, A2Q077 |
| | 8.20 | 11034306 | 953.2 | 8.23 | A2Q008, A2Q054, A2Q067 |
| | 8.20 | 11034306 | 952.3 | 8.23 | A2Q027, A2Q028, A2Q080 |
| | 8.20 | 11034306 | 984.2 | 8.27 | A2Q032 |
| | 8.20 | 11034306 | 982.2 | 8.25 | A2Q041, A2Q081 |
| | 8.25 | 36600836 | 980.3 | 8.28 | A2Q090 |
| | 8.40 | 20912664 | 953.3 | 8.47 | A2Q008, A2Q054, A2Q067 |
| | 8.40 | 20912664 | 946.3 | 8.43 | A2Q093 |
| | 8.50 | 25740568 | 973.2 | 8.56 | A2Q010 |
| | 8.50 | 25740568 | 972.3 | 8.53 | A2Q082 |
| | 8.64 | 24987850.67 | 951.3 | 8.66 | A2Q004, A2Q005, A2Q055 |
| | 8.64 | 24987850.67 | 974.4 | 8.70 | A2Q033 |
| | 8.64 | 24987850.67 | 935.2 | 8.67 | A2Q049, A2Q050 |
| | 8.73 | 32226358 | 937.3 | 8.81 | A2Q003 |
| | 8.73 | 32226358 | 921.3 | 8.77 | A2Q047 |
| | 8.83 | 25354158 | 935.3 | 8.87 | A2Q049, A2Q050 |
| | 8.90 | 18120409.33 | 923.3 | 8.95 | A2Q001 |
| | 8.90 | 18120409.33 | 983.2 | 8.94 | A2Q009 |
| | 8.90 | 18120409.33 | 981.3 | 8.94 | A2Q018, A2Q056 |

(continued)

| [Table AG-E-12] | | | | | |
|---|---|---|---|---|---|
| Mixture No. | Retention time of UV peak (min) | Cal UV area | (M+H) observed at MS peak top | Retention time of MS peak (min) | No. of assigned compound |
| | 9.03 | 29772288 | 927.2 | 9.05 | A2Q059 |
| | 9.03 | 29772288 | 990.3 | 9.08 | A2Q086 |
| | 9.08 | 28501738 | 939.2 | 9.11 | A2Q053 |
| | 9.27 | 51704090.67 | 941.2 | 9.29 | A2Q006, A2Q060 |
| | 9.27 | 51704090.67 | 953.2 | 9.30 | A2Q008, A2Q054, A2Q067 |
| | 9.27 | 51704090.67 | 1006.2 | 9.25 | A2Q038, A2Q088 |
| | 9.37 | 19613754 | 992.2 | 9.40 | A2Q037, A2Q091 |
| | 9.56 | 23337522 | 1008.2 | 9.60 | A2Q039 |
| | 9.56 | 23337522 | 945.3 | 9.59 | A2Q068 |
| | 9.63 | 28536014 | 979.3 | 9.67 | A2Q065 |
| | 9.69 | 22151034 | 981.3 | 9.72 | A2Q018, A2Q056 |
| | 9.79 | 18805396 | 947.3 | 9.85 | A2Q020 |
| | 9.79 | 18805396 | 948.2 | 9.85 | A2Q043 |
| | 9.79 | 18805396 | 949.2 | 9.84 | A2Q051, A2Q052 |
| | 10.04 | 15567021 | 951.3 | 10.09 | A2Q004, A2Q005, A2Q055 |
| | 10.18 | 36859196 | 971.3 | 10.22 | A2Q057 |
| | 10.63 | 27994258 | 989.2 | 10.66 | A2Q061 |
| | 10.70 | 32998406 | 944.2 | 10.75 | A2Q036 |

[Table 177]

| | | | | | |
|---|---|---|---|---|---|
| | 10.78 | 25031776 | 1005.2 | 10.82 | A2Q015, A2Q063 |
| | 10.86 | 26066518 | 952.3 | 10.90 | A2Q027, A2Q028, A2Q080 |
| | 11.11 | 23158678 | 991.3 | 11.19 | A2Q014, A2Q066 |
| | 11.11 | 23158678 | 963.2 | 11.15 | A2Q045, A2Q048 |
| | 11.20 | 30476906 | 956.2 | 11.23 | A2Q030 |
| | 11.27 | 29544118 | 1007.2 | 11.36 | A2Q016 |
| | 11.27 | 29544118 | 942.2 | 11.32 | A2Q029, A2Q085 |

(continued)

| | | | | | |
|---|---|---|---|---|---|
| | 11.39 | 17686612 | 951.2 | 11.44 | A2Q004, A2Q005, A2Q055 |
| | 11.39 | 17686612 | 950.2 | 11.42 | A2Q076, A2Q077 |
| | 11.51 | 24829456 | 966.3 | 11.55 | A2Q025, A2Q089 |
| | 11.59 | 19834348 | 962.3 | 11.63 | A2Q022 |
| | 11.59 | 19834348 | 961.2 | 11.63 | A2Q095 |
| | 11.70 | 36922068 | 954.2 | 11.74 | A2Q031, A2Q079, A2Q092 |
| | 11.70 | 36922068 | 988.3 | 11.70 | A2Q034 |
| | 11.87 | 16746562 | 965.2 | 11.91 | A2Q002, A2Q064 |
| | 11.87 | 16746562 | 964.3 | 11.92 | A2Q073 |
| | 12.13 | 39260800 | 986.3 | 12.17 | A2Q083 |
| | 12.37 | 36954760 | 1006.2 | 12.40 | A2Q038, A2Q088 |
| | 12.56 | 34230344 | 1072.3 | 12.60 | A2Q046 |
| | 12.80 | 36048268 | 1004.2 | 12.84 | A2Q087 |
| | 12.91 | 31154304 | 994.3 | 12.95 | A2Q042 |
| | 13.04 | 72988080 | 1070.3 | 13.05 | A2Q096 |
| | 13.09 | 46282816 | 960.2 | 13.13 | A2Q070 |
| | 13.16 | 33429302 | 955.1 | 13.21 | A2Q007 |
| | 13.38 | 70814560 | 992.3 | 13.42 | A2Q037, A2Q091 |
| | 13.51 | 39793924 | 941.2 | 13.53 | A2Q006, A2Q060 |
| | 13.67 | 29033824 | 1030.3 | 13.70 | A2Q044 |
| | 13.82 | 36388344 | 949.3 | 13.85 | A2Q051, A2Q052 |
| | 13.92 | 32792780 | 987.2 | 13.96 | A2Q011 |
| | 14.08 | 37192716 | 1028.2 | 14.12 | A2Q094 |
| | 14.13 | 18850790 | 965.3 | 14.18 | A2Q002, A2Q064 |
| | 14.20 | 33817512 | 1005.3 | 14.23 | A2Q015, A2Q063 |
| | 14.25 | 24661582 | 963.2 | 14.31 | A2Q045, A2Q048 |
| | 14.30 | 35729592 | 985.2 | 14.35 | A2Q058 |
| | 14.58 | 35156544 | 1071.2 | 14.65 | A2Q023 |
| | 14.58 | 35156544 | 1003.2 | 14.61 | A2Q062 |
| | 14.96 | 41031164 | 1069.4 | 15.00 | A2Q071 |
| | 15.10 | 39165600 | 993.3 | 15.15 | A2Q019 |
| | 15.10 | 39165600 | 1029.2 | 15.13 | A2Q021 |
| | 15.45 | 37583188 | 991.3 | 15.51 | A2Q014, A2Q066 |
| | 15.45 | 37583188 | 1027.2 | 15.48 | A2Q069 |

(continued)

| | | | |
|---|---|---|---|
| Statistical analysis | The number of samples of Cal_UV_area | 96 | |
| | Median | 25848622 | |
| | Mean | 28333548 | |
| | Standard deviation | 11402051 | |
| | 75 percentile | 36049965 | |
| | 25 percentile | 18839442 | |
| | Upper boundary value | 61865751 | |
| | Lower boundary value | -6976344 | |
| | The number of outliers | 2 | |
| | Maximum value | 72988080 | |
| | Minimum value | 11034306 | |

From the analysis data shown in Tables AG-E-01 to AG-E-12, it was confirmed that 1,152 compounds among 1,200 compounds set as library compounds, i.e., 96% of compounds, were synthesized and existed in the mixture library. As shown in Table AG-E- 13, the results of comparing the UV areas at a specific wavelength (specifically, 319 nm) of the synthesized compounds revealed that the difference in area between a compound having the largest area and a compound having the smallest area in each mixture was suppressed within 9.6 times. Outliers were identified by statistical processing, and a similar ratio was determined by excluding compounds that exhibited the outliers, was consequently 6.1 times at the maximum. A similar ratio was determined targeting 90 compounds excluding three top compounds having a larger UV area and three top compounds excluding a smaller UV area, and was consequently 4.7 times at the maximum. In short, 1,080 compounds that corresponded to 90% of 1,200 compounds set as library compounds were synthesized while the UV area (319 nm) ratio fell within 4.7 times. These results are results obtained by one analysis approach to analysis data and are a mere illustration of analysis results because the UV area of overlapping compounds, if present, in a UV peak is equally divided.

However, it was revealed that compound library synthesis involving a UV tag can be carried out to thereby confirm that "intended library compounds are synthesized and exist in a mixture library" as well as "difference in content among the library compounds is small". This was able to be confirmed to become a solution to the challenge related to the ensuring of quality.

[Table 178]

| [Table AG-E-13] | | | | | | | | | | | | | |
|---|---|---|---|---|---|---|---|---|---|---|---|---|---|
| | | Mixture No. | | | | | | | | | | | |
| | Mixture No. | mixED 05 | mixED O6 | mixED O7 | mixED O8 | mixED O9 | mixED 10 | mixED 11 | mixED 12 | mixED 13 | mixED 14 | mixED 15 | mixED 16 |
| | The number of outliers | 3 | 2 | 3 | 0 | 1 | 4 | 1 | 2 | 1 | 2 | 2 | 2 |
| Ratio between peak with largest UV area and peak with smallest UV area (times) | Targeting 96 compounds whose presence was confirmed | 6.7 | 7.2 | 7.7 | 4 | 8 | 4.4 | 5.2 | 9.6 | 3.2 | 4.8 | 5.3 | 6.6 |
| | Targeting compound other than compound exhibiting outlier | 4.2 | 5.5 | 4.4 | 4.0 | 6.1 | 3.1 | 4.6 | 5.3 | 2.9 | 3.5 | 4.6 | 4.7 |
| | Targeting 90 compounds excluding 3 top compounds with largest UV area and 3 top compounds with smallest UV area | 3.2 | 4.0 | 3.2 | 2.5 | 4.6 | 3.2 | 2.7 | 3.5 | 2.4 | 2.9 | 4.4 | 4.7 |

The approach of the present invention was found capable of synthesizing, with quality ensured, a library having the diversity of core blocks resulting from the reaction between building blocks, and the diversity of linking moieties (linkers) that link them, as conceived by the present invention, unlike the diversity of building blocks taken into consideration in general library synthesis.

The synthesis example of a mixture library illustrated here is one unit group to be subjected to a biological evaluation assay. A similar operation can be carried out in parallel therewith using another building block group or another linker formation reaction to thereby increase the diversity of compounds as a library assembly while ensuring the quality of one library.

Example 4; Practice example of compound library synthesis for AS-MS

In Example 3, the approach of the present invention was found capable of producing a library also having the diversity of linking moieties (linkers) that link building blocks, while confirming the quality of each compound constituting the library. A conventional library generates the diversity of pharmacophores derived only from the structural diversity of building blocks. By contrast, the structural diversity is markedly expanded as a library for which not only core blocks that form pharmacophores but linkers located therebetween can be freely selected.

Example 4 illustrates exemplary synthesis of a library in which the "diversity of core blocks" that form pharmacophores and the "diversity of linkers" are multiplied, by exploiting the findings obtained in Example 3. The library was subjected to the measurement of affinity for Bcl-2 selected as one example of a target protein by AS-MS, a screening approach of hit generation (the affinity measurement by AS-MS is described in Example 5). A compound having affinity was identified by AS-MS to confirm that the method for producing a library according to the present invention is an approach that satisfies the challenges mentioned above, i.e., the "securing of library diversity" and the "ensuring of library quality". Figure 2 shows the "diversity of core blocks" and the "diversity of linkers" contained in the library.

From among diverse compounds brought about by the combination of "diversity of core blocks" and the "diversity of linkers" shown in Figure 2, 1,200 compounds were set as one example of library compounds to carry out library synthesis. For the purpose of confirming that the library provided by this approach functioned suitably in evaluation by AS-MS, this library was set so as to contain four compounds, B2Q045-01 ($K_D$ = 1.2 $\mu$M), B2Q046-01 ($K_D$ = 8.1 $\mu$M), B2QR045-01 ($K_D \geq$ 20 $\mu$M), and B2R046-01 ($K_D \geq$ 20 $\mu$M), which were synthesized beforehand by a method described in Example 5 and evaluated for their binding by SPR described in Example 6. The compounds B2Q045-01, B2Q046-01, B2R045-01, and B2R046-01 evaluated beforehand for their ability to bind are compounds presumed to maintain the ability to bind to Bcl-2 according to literatures (Expert Opinion on Drug Discovery, Volume 3, Issue 9, 2008, 1123-1143; and J. Med. Chem., 2008, 51 (21), pp 6902-6915), and their binding were actually able to be confirmed.

The library synthesis was performed by the following method: after introduction of each building block, i.e., after linker formation reaction, a mixture was prepared on a type basis of reactive functional groups present on the building blocks, followed by linker formation reaction. Hereinafter, the method will be shown as an exemplary method for producing a library in which the "diversity of core blocks" and the "diversity of linkers" are multiplied. In the library synthesis given below, the building blocks shown in Table LBB02 were used.

[Table 179]

| [Table LBB02] | | |
|---|---|---|
| Compound No. | Structural formula | Compound name |
| BB01 | 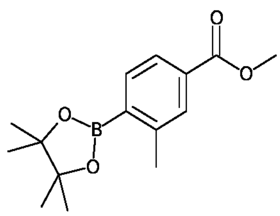 | Methyl 3-methyl-4-(4,4,5,5-tetramethyl- 1,3,2-dioxaboloran-2-yl)benzoate |

(continued)

| [Table LBB02] | | |
|---|---|---|
| Compound No. | Structural formula | Compound name |
| BB02 | 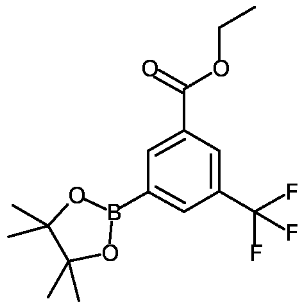 | Ethyl 3-(4,4,5,5-tetramethyl-1,3,2-dioxaboloran-2-yl)-5- (trifluoromethyl)benzoate |
| BB03 | 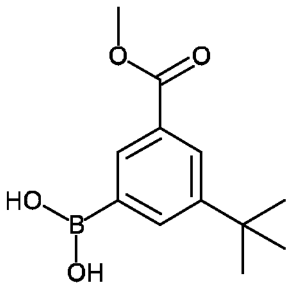 | (3-tert-Butyl-5- methoxycarbonylphenyl)boronic acid |
| BB04 | 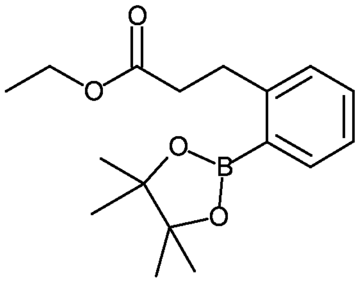 | Ethyl 3-[2-(4,4,5,5-tetramethyl-1,3,2-dioxaboloran-2-yl)phenyl]propanoate |
| BB05 | 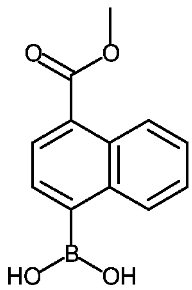 | (4-Methoxycarbonylnaphthalen-1-yl)boronic acid |
| BB06 | 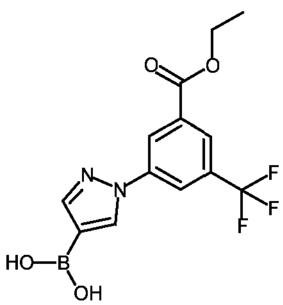 | {1-[3-Ethoxycarbonyl-5-(trifluoromethyl)phenyl]pyrazol-4- yl}boronic acid |

(continued)

| [Table LBB02] | | |
|---|---|---|
| Compound No. | Structural formula | Compound name |
| BB07 | 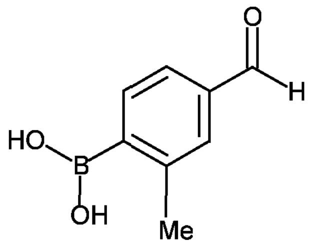 | (4-Formyl-2-methylphenyl)boronic acid |
| BB08 | 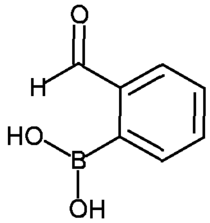 | (2-Formylphenyl)boronic acid |
| BB09 | 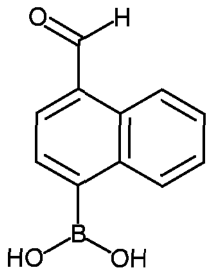 | (4-Formylnaphthalen-1-yl)boronic acid |
| BB10 | 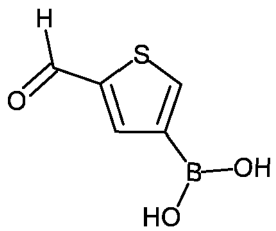 | (5-Formylthiophen-3-yl)boronic acid |
| BB11 | 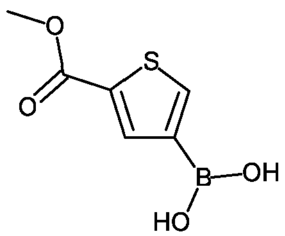 | (5-(Methoxycarbonyl)thiophen-3- yl)boronic acid |
| BB12 | 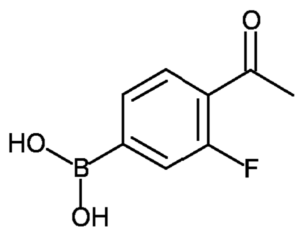 | (4-Acetyl-3-fluorophenyl)boronic acid |

(continued)

| [Table LBB02] | | |
|---|---|---|
| Compound No. | Structural formula | Compound name |
| BB13 | 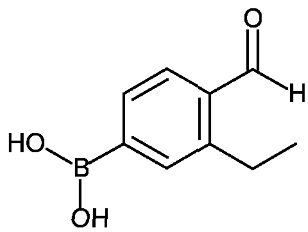 | (3-Ethyl-4-formylphenyl)boronic acid |
| BB14 | 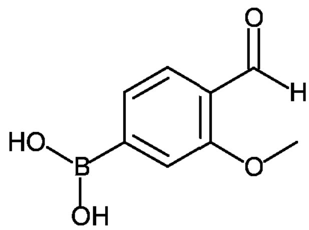 | (4-Formyl-3-methoxyphenyl)boronic acid |
| BB15 | 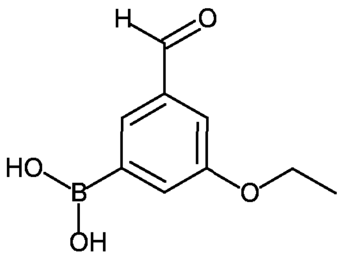 | (3-Ethoxy-5-formylphenyl)boronic acid |
| BB16 | 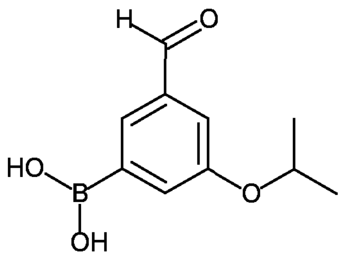 | (3-Formyl-5-propan-2- yloxyphenyl)boronic acid |
| BB17 | 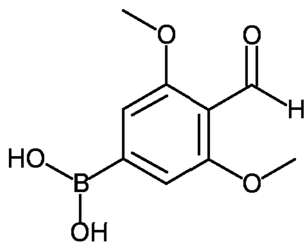 | (4-Formyl-3,5-dimethoxyphenyl)boronic acid |
| BB18 | 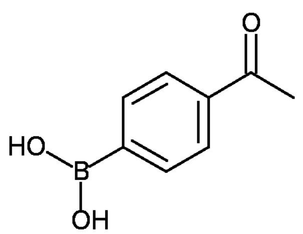 | (4-Acetylphenyl)boronic acid |

(continued)

| [Table LBB02] | | |
|---|---|---|
| Compound No. | Structural formula | Compound name |
| BB19 | 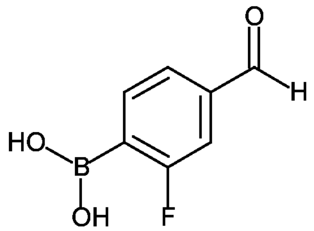 | (2-Fluoro-4-formylphenyl)boronic acid |
| BB20 | 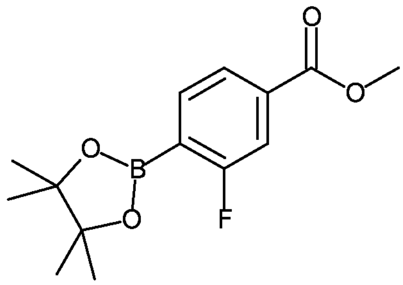 | Methyl 3-fluoro-4-(4,4,5,5-tetramethyl- 1,3,2-dioxaboloran-2-yl)benzoate |
| BB21 | 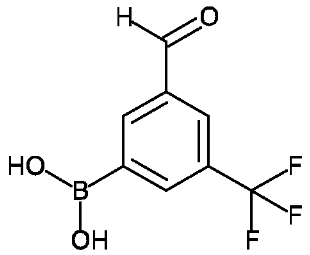 | [3-Formyl-5- (trifluoromethyl)phenyl]boronic acid |
| BB22 | 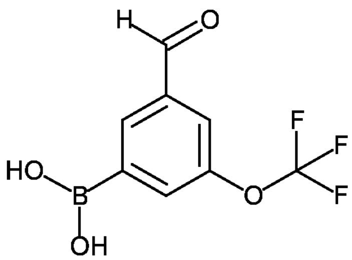 | [3-Formyl-5- (trifluoromethoxy)phenyl]boronic acid |
| BB23 | 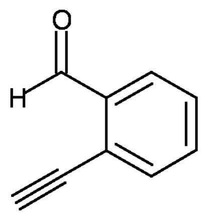 | 2-Ethynylbenzaldehyde |
| BB24 | 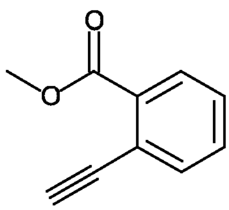 | Methyl 2-ethynylbenzoate |

(continued)

| [Table LBB02] | | |
|---|---|---|
| Compound No. | Structural formula | Compound name |
| BB25 | 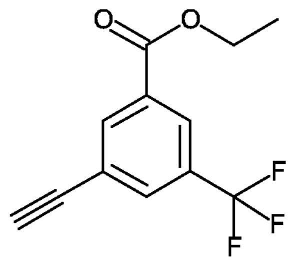 | Ethyl 3-ethynyl-5- (trifluoromethyl)benzoate |
| BB26 | 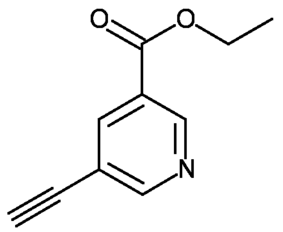 | Ethyl 5-ethynylpyridine-3 -carboxylate |
| BB27 | 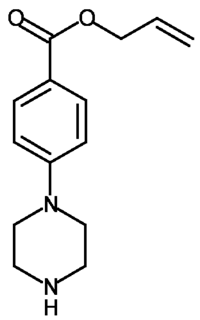 | Allyl 4-(piperazin-1-yl)benzoate |
| BB28 | 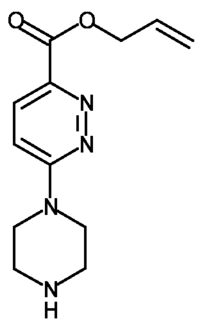 | Allyl 6-(piperazin-1-yl)pyridazine-3- carboxylate |
| BB29 | 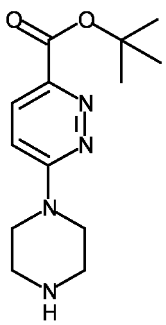 | tert-Butyl 6-(piperazin-1-yl)pyridazine-3- carboxylate |

(continued)

[Table LBB02]

| Compound No. | Structural formula | Compound name |
|---|---|---|
| BB30 | 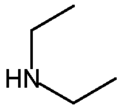 | N-Ethylethanamine |
| BB31 | 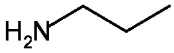 | Propane-1-amine |
| BB32 | 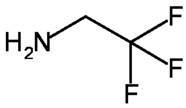 | 2,2,2-Trifluoroethanamine |
| BB33 | 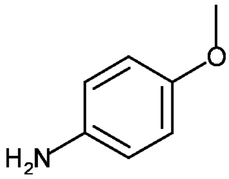 | 4-Methoxyaniline |
| BB34 | 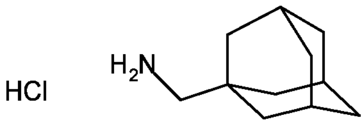 | 1-Adamantylmethanamine hydrochloride |
| BB35 | 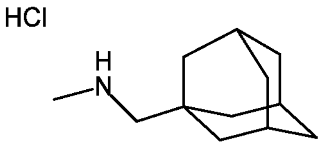 | 1-(1-Adamantyl)-N-methylmethanamine hydrochloride |
| BB36 | 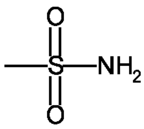 | Methanesulfonamide |
| BB37 | 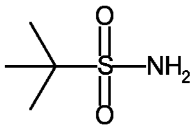 | 2-Methylpropane-2-sulfonamide |
| BB38 | 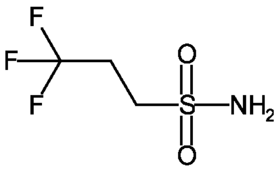 | 3,3,3-Trifluoropropane-1-sulfonamide |
| BB40 | 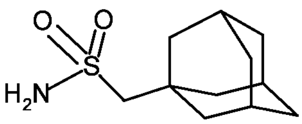 | 1-Adamantylmethanesulfonamide |

(continued)

| [Table LBB02] | | |
|---|---|---|
| Compound No. | Structural formula | Compound name |
| BB41 | 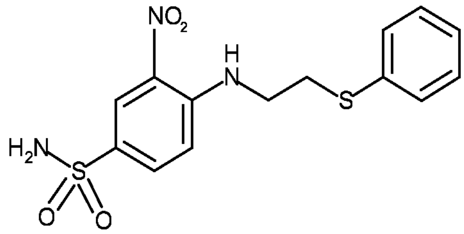 | 3-Nitro-4- {[2- (phenylthio)ethyl] amino} benzenesulfona mide |
| BB42 | 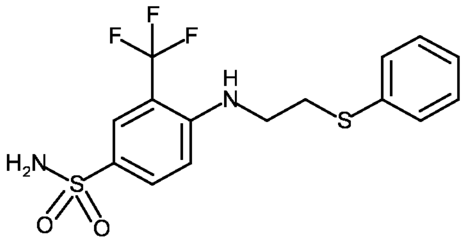 | 4-(2-Phenylsulfanylethylamino)-3-(trifluoromethyl)benzenesulfonamide |

In this Example 4, a compound supported on brominated modified Wang resin (4- (bromomethyl)phenoxyethylpolystyrene) (e.g., Merck KGaA, 2-(4- bromomethylphenoxy)ethylpolystyrene) was used, and "-02R" was described at the end of the compound number of the compound supported on the solid phase. In the tables in Example 4, the solid-phase supported compound may be indicated by a compound number excluding "-02R", also because only a structural formula after cleavage is described in conversion reaction.

Example 4-1-1-A: Synthesis of mixtures mixEG01-02R to mixEG04-02R and mixEG18-02R

[Formula 524]

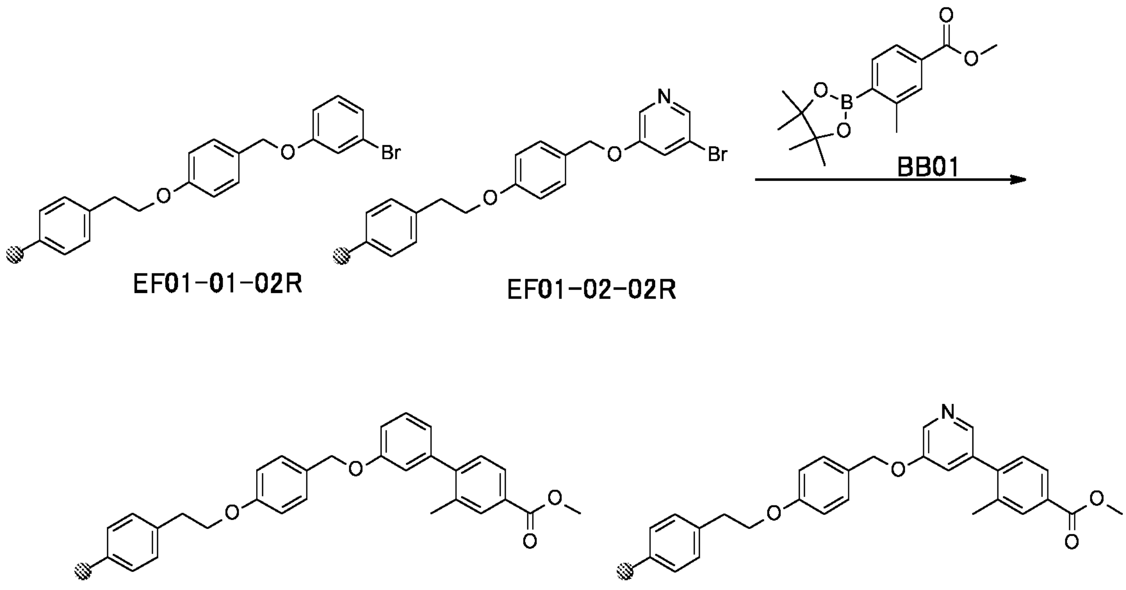

EF01-01-02R
EF01-02-02R
BB01
EG01-01-02R
EG01-02-02R

Under a nitrogen atmosphere, compound EF01-01-02R (57 mg, loading rate: 0.556 mmol/g, 0.032 mmol), compound EF01-02-02R (43 mg, loading rate: 0.733 mmol/g, 0.032 mmol), and NMP (1.95 mL) were added to a 4 mL glass vial and shaken at room temperature for 1 hour. Under a nitrogen atmosphere, methyl 3-methyl-4-(4,4,5,5- tetramethyl-1,3,2-dioxaborolan-2-yl)benzoate (BB01) (107 mg, 0.387 mmol), an aqueous $K_3PO_4$ solution (1.7 M, 228 μL, 0.387 mmol), and a solution of $P(Cy)_3$ Pd G3 in NMP (0.04 M, 323 μL, 13 μmol) were added thereto, and the mixture was shaken at 90°C for 20 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), three times with DCM (2 mL), and three times with heptane (2 mL), and the resin was then dried under reduced pressure to obtain the title mixture shown in Table EG01. The building blocks used, compounds contained in the obtained mixtures, and the reaction time are also shown in Table EG01.

The 1.7 M aqueous $K_3PO_4$ solution was prepared by diluting, under a nitrogen atmosphere, $K_3PO_4$ (1.8 g, 8.48 mmol) with distilled water using a 5 mL measuring flask.

Example 4-1-1-B: Synthesis of mixtures mixEG02-02R to mixEG04-02R and mixEG18-02R

The title mixtures shown in Table EG01 were synthesized in the same manner as in Example 4-1-1-A using compound EF01-01-02R (57 mg, loading rate: 0.556 mmol/g, 0.032 mmol), compound EF01-02-02R (43 mg, loading rate: 0.733 mmol/g, 0.032 mmol), and the boronic acid or boronic acid ester reagents (BB02 to BB04 and BB18) shown in Table LBB02. The building blocks used, compounds contained in the obtained mixtures, and the reaction times are also shown in Table EG01.

[Table 180]

[Table EG01]

| No. of BB used | No. of synthesized mixture | Resin compound No. | No. of mixture after cleavage | Compound No. after cleavage | Compound name | Reaction time (hr) |
|---|---|---|---|---|---|---|
| BB01 | mixEG01-02R | EG01-01-02R | mixEG01 | EG01-01 | Methyl 4-(3-hydroxyphenyl)-3-methylbenzoate | 20 |
| | | EG01-02-02R | | EG01-02 | Methyl 4-(5-hydroxypyridin-3-yl)-3-methylbenzoate | |
| BB02 | mixEG02-02R | EG01-03-02R | mixEG02 | EG01-03 | Ethyl 3-(3-hydroxyphenyl)-5-(trifluoromethyl)benzoate | 20 |
| | | EG01-04-02R | | EG01-04 | Ethyl 3-(5-hydroxypyridin-3-yl)-5-(trifluoromethyl)benzoate | |
| BB03 | mixEG03-02R | EG01-05-02R | mixEG03 | EG01-05 | Methyl 3-tert-butyl-5-(3-hydroxyphenyl)benzoate | 20 |
| | | EG01-06-02R | | EG01-06 | Methyl 3-tert-butyl-5-(5-hydroxypyridin-3-yl)benzoate | |
| BB04 | mixEG04-02R | EG01-07-02R | mixEG04 | EG01-07 | Ethyl 3-[2-(3-hydroxyphenyl)phenyl]propanoate | 20 |
| | | EG01-08-02R | | EG01-08 | Ethyl 3-[2-(5-hydroxypyridin-3-yl)phenyl]propanoate | |
| BB18 | mixEG18-02R | EG01-09-02R | mixEG18 | EG01-09 | 1-[4-(3-Hydroxyphenyl)phenyl]ethanone | 20 |
| | | EG01-10-02R | | EG01-10 | 1-[4-(5-hydroxypyridin-3-yl)phenyl]ethanone | |

Example 4-1-2-A: Synthesis of mixture mixEG05-02R

[Formula 525]

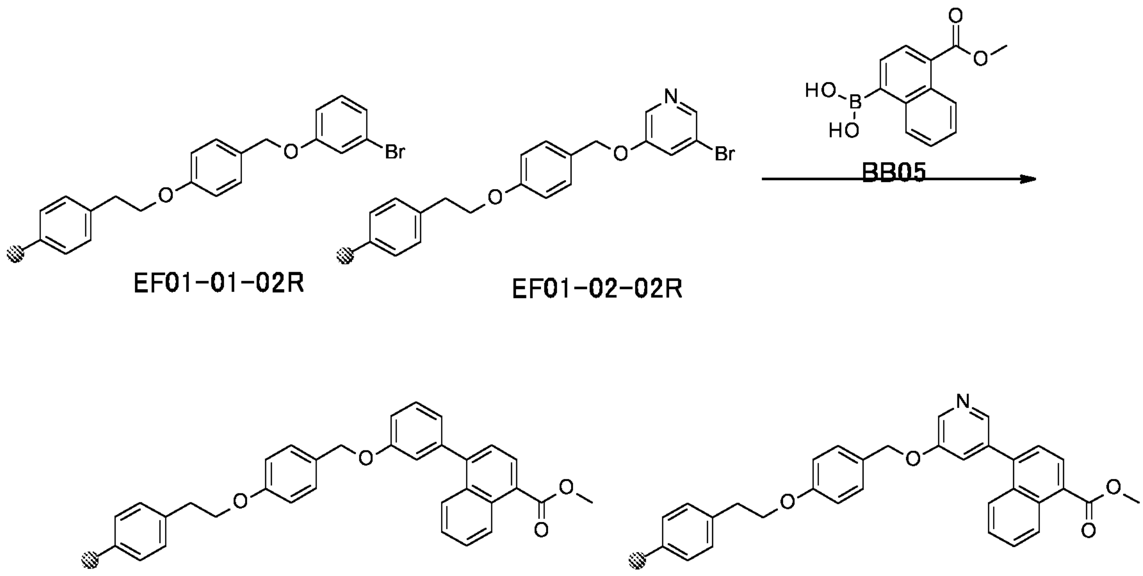


EF01-01-02R EF01-02-02R

EG05-01-02R EG05-02-02R

Under a nitrogen atmosphere, compound EF01-01-02R (57 mg, loading rate: 0.556 mmol/g, 0.032 mmol), compound EF01-02-02R (43 mg, loading rate: 0.733 mmol/g, 0.032 mmol), and NMP (1.95 mL) were added to a 4 mL glass vial and shaken at room temperature for 1 hour. Under a nitrogen atmosphere, (4-methoxycarbonylnaphthalen-1- yl)boronic acid (BB05) (178 mg, 0.774 mmol), an aqueous $K_3PO_4$ solution (1.7 M, 228 $\mu$L, 0.387 mmol), and a solution of $P(Cy)_3$ Pd G3 in NMP (0.04 M, 323 $\mu$L, 13 $\mu$mol) were added thereto, and the mixture was shaken at 90°C for 20 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), three times with DCM (2 mL), and three times with heptane (2 mL), and the resin was then dried under reduced pressure to obtain the title mixture shown in Table EG05. The building block used, compounds contained in the obtained mixture, and the reaction time are also shown in Table EG05.

Example 4-1-2-B: Synthesis of mixture mixEG06-02R

The title mixture shown in Table EG05 was synthesized in the same manner as in Example 4-1-2-A using compound EF01-01-02R (57 mg, loading rate: 0.556 mmol/g, 0.032 mmol), compound EF01-02-02R (43 mg, loading rate: 0.733 mmol/g, 0.032 mmol), and (1-(3-ethoxycarbonyl-5-(trifluoromethyl)phenyl]pyrazol-4-yl]boronic acid (BB06) shown in LBB02. The building block used, compounds contained in the obtained mixture, and the reaction time are also shown in Table EG05.

[Table 181]

[Table EG05]

| No. of BB used | No. of synthesized mixture | Resin compound No. | No. of mixture after cleavage | Compound No. after cleavage | Compound name | Reaction time (hr) |
|---|---|---|---|---|---|---|
| BB05 | mixEG05-02R | EG05-01-02R | mixEG05 | EG05-01 | Methyl 4-(3-hydroxyphenyl)naphthalene-1-carboxylate | 20 |
| | | EG05-02-02R | | EG05-02 | Methyl 4-(5-hydroxypyridin-3-yl)naphthalene-1-carboxylate | |
| BB06 | mixEG06-02R | EG05-03-02R | mixEG06 | EG05-03 | Ethyl 3-[4-(3-hydroxyphenyl)pyrazol-1-yl]-5-(trifluoromethyl)benzoate | 20 |
| | | EG05-04-02R | | EG05-04 | Ethyl 3-[4-(5-hydroxypyridin-3-yl)pyrazol-1-yl]-5-(trifluoromethyl)benzoate | |

Example 4-1-3-A: Synthesis of mixture mixEG07-02R

[Formula 526]

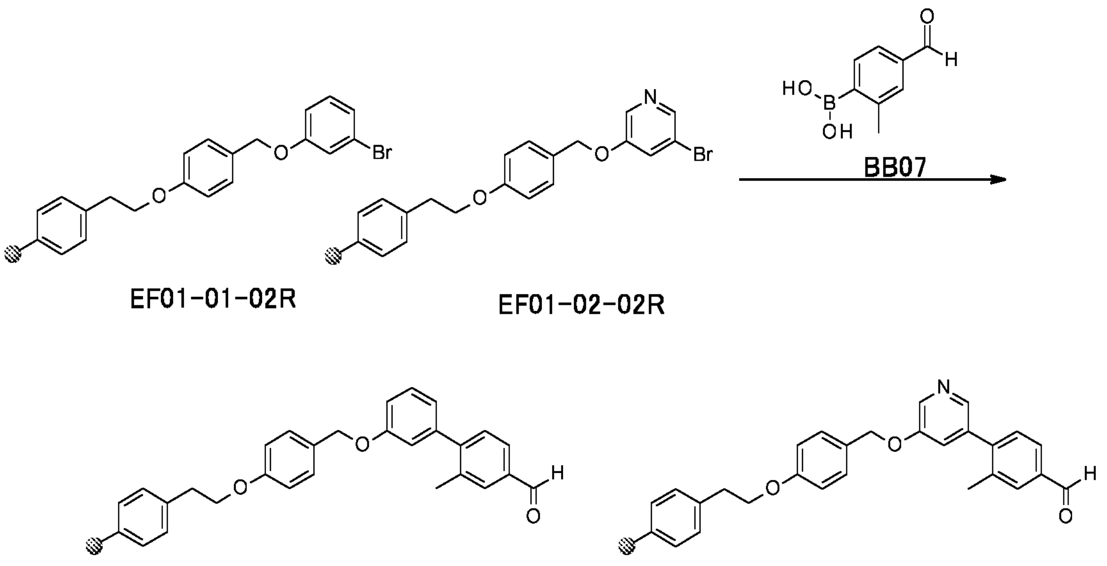


EF01-01-02R EF01-02-02R

EG07-01-02R EG07-02-02R

Under a nitrogen atmosphere, compound EF01-01-02R (57 mg, loading rate: 0.556 mmol/g, 0.032 mmol), compound EF01-02-02R (43 mg, loading rate: 0.733 mmol/g, 0.032 mmol), NMP (1.4 mL), and water (72 μL) were added to a 3 mL glass vial and shaken at room temperature for 1 hour. Under a nitrogen atmosphere, (4-formyl-2-methylphenyl)boronic acid (BB07) (63.5 mg, 0.387 mmol), $Na_2CO_3$ (41 mg, 0.387 mmol), and a solution of $P(Cy)_3$ Pd G3 in NMP (0.04 M, 323 μL, 13 μmol) were added thereto, and the mixture was shaken at 90°C for 21 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), three times with DCM (2 mL), and three times with heptane (2 mL), and the resin was then dried under reduced pressure to obtain the title mixture shown in Table EG07. The building block used, compounds contained in the obtained mixture, and the reaction time are also shown in Table EG07.

Example 4-1-3-B: Synthesis of mixtures mixEG08-02R to mixEG17-02R

The title mixtures shown in Table EG07 were synthesized in the same manner as in Example 4-1-3-A using compound EF01-01-02R (57 mg, loading rate: 0.556 mmol/g, 0.032 mmol), compound EF01-02-02R (43 mg, loading rate: 0.733 mmol/g, 0.032 mmol), and the boronic acid or boronic acid ester reagents (BB08 to BB17) shown in Table LBB02. The building blocks used, compounds contained in the obtained mixtures, and the reaction times are also shown in Table EG07.

[Table 182]

[Table EG07]

| No. of BB used | No. of synthesized mixture | Resin compound No. | No. of mixture after cleavage | Compound No. after cleavage | Compound name | Reaction time (hr) |
|---|---|---|---|---|---|---|
| BB07 | mixEG07-02R | EG07-0102R | mixEG07 | EG07-01 | 4-(3-Hydroxyphenyl)-3-methylbenzaldehyde | 21 |
| | | EG07-0202R | | EG07-02 | 4-(5-Hydroxypyridin-3-yl)-3-methylbenzaldehyde | |
| BB08 | mixEG08-02R | EG07-0302R | mixEG08 | EG07-03 | 2-(3-Hydroxyphenyl)benzaldehyde | 21 |
| | | EG07-0402R | | EG07-04 | 2-(5-Hydroxypyridin-3-yl)benzaldehyde | |
| BB09 | mixEG09-02R | EG07-0502R | mixEG09 | EG07-05 | 4-(3-Hydroxyphenyl)naphthalene-1-carbaldehyde | 21 |
| | | EG07-0602R | | EG07-06 | 4-(5-Hydroxypyridin-3-yl)naphthalene-1-carbaldehyde | |
| BB10 | mixEG10-02R | EG07-0702R | mixEG10 | EG07-07 | 4-(3-Hydroxyphenyl)thiophene-2-carbaldehyde | 21 |

| | | | | | | |
|---|---|---|---|---|---|---|
| | | EG07-0802R | | EG07-08 | 4-(5-Hydroxypyridin-3-yl)thiophene-2-carbaldehyde | |
| BB11 | mixEG11-02R | EG07-0902R | mixEG11 | EG07-09 | Methyl 4-(3-hydroxyphenyl)thiophene-2-carboxylate | 21 |
| | | EG07-1002R | | EG07-10 | Methyl 4-(5-hydroxypyridin-3-yl)thiophene-2-carboxylate | |
| BB12 | mixEG12-02R | EG07-1102R | mixEG12 | EG07-11 | 1-[2-Fluoro-4-(3-hydroxyphenyl)phenyl]ethanone | 27 |
| | | EG07-1202R | | EG07-12 | 1-[2-Fluoro-4-(5-hydroxypyridin-3-yl)phenyl]ethanone | |
| BB13 | mixEG13-02R | EG07-1302R | mixEG13 | EG07-13 | 2-Ethyl-4-(3-hydroxyphenyl)benzaldehyde | 66 |
| | | EG07-1402R | | EG07-14 | 2-Ethyl-4-(5-hydroxypyridin-3-yl)benzaldehyde | |
| BB14 | mixEG14-02R | EG07-1502R | mixEG14 | EG07-15 | 4-(3-Hydroxyphenyl)-2-methoxybenzaldehyde | 66 |
| | | EG07-1602R | | EG07-16 | 4-(5-Hydroxypyridin-3-yl)-2-methoxybenzaldehyde | |
| BB15 | mixEG15-02R | EG07-1702R | mixEG15 | EG07-17 | 3-Ethoxy-5-(3-hydroxyphenyl)benzaldehyde | 66 |
| | | EG07-1802R | | EG07-18 | 3-Ethoxy-5-(5-hydroxypyridin-3-yl)benzaldehyde | |
| BB16 | mixEG16-02R | EG07-1902R | mixEG16 | EG07-19 | 3-(3-Hydroxyphenyl)-5-propan-2-yloxybenzaldehyde | 66 |
| | | EG07-2002R | | EG07-20 | 3-(5-Hydroxypyridin-3-yl)-5-propan-2-yloxybenzaldehyde | |
| BB17 | mixEG17-02R | EG07-2102R | mixEG17 | EG07-21 | 4-(3-Hydroxyphenyl)-2,6-dimethoxybenzaldehyde | 66 |
| | | EG07-2202R | | EG07-22 | 4-(5-Hydroxypyridin-3-yl)-2,6-dimethoxybenzaldehyde | |

Example 4-1-4-A: Synthesis of mixture mixEG 19-02R

[Formula 527]

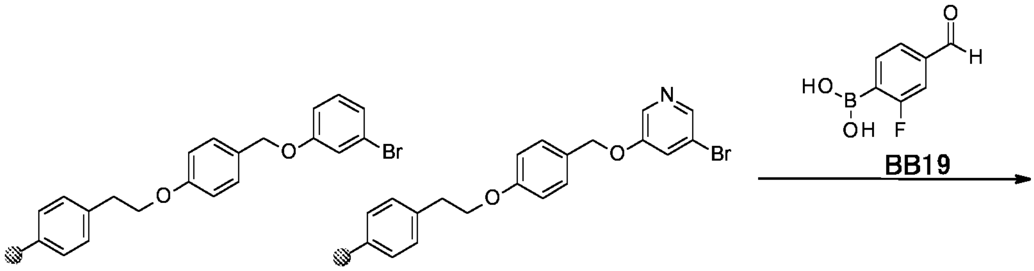

BB19

EF01-01-02R EF01-02-02R

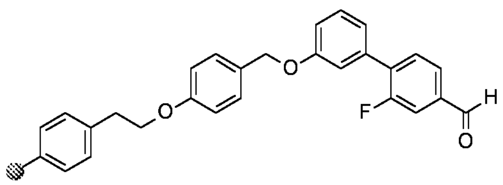

EG19-01-02R

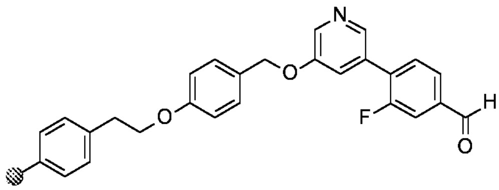

EG19-02-02R

Under a nitrogen atmosphere, compound EF01-01-02R (57 mg, loading rate: 0.556 mmol/g, 0.032 mmol), compound EF01-02-02R (43 mg, loading rate: 0.733 mmol/g, 0.032 mmol), and 1,4-dioxane (1.8 mL) were added to a 4 mL glass vial and shaken at room temperature for 1 hour. Under a nitrogen atmosphere, (2-fluoro-4-formylphenyl)boronic acid (BB19) (65 mg, 0.387 mmol), $K_3PO_4$-$H_2O$ (89 mg, 0.387 mmol), pinacol (45.7 mg, 0.387 mmol), and a solution of $P(Cy)_3$ Pd G3 in 1,4-dioxane (0.04 M, 323 μL, 13 μmol) were added thereto, and the mixture was shaken at 90°C for 22 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), three times with DCM (2 mL), and three times with heptane (2 mL), and the resin was then dried under reduced pressure to obtain the title mixture shown in Table EG19. The building block used, compounds contained in the obtained mixture and the reaction time are also shown in Table EG19.

Example 4-1-4-B: Synthesis of mixture mixEG20-02R

The title mixture shown in Table EG19 was synthesized in the same manner as in Example 4-1-4-A using compound EF01-01-02R (57 mg, loading rate: 0.556 mmol/g, 0.032 mmol), compound EF01-02-02R (43 mg, loading rate: 0.733 mmol/g, 0.032 mmol), and methyl 3-fluoro-4-(4,4,5,5-tetramethyl-1,3,2-dioxaborolan-2-yl)benzoate (BB20) shown in Table LBB02. The building block used, compounds contained in the obtained mixture, and the reaction time are also shown in Table EG 19.

[Table 183]

[Table EG19]

| No. of BB used | No. of synthesized mixture | Resin compound No. | No. of mixture after cleavage | Compound No. after cleavage | Compound name | Reaction time (hr) |
|---|---|---|---|---|---|---|
| BB19 | mixEG19-02R | EG19-01-02R | mixEG19 | EG19-01 | 3-Fluoro-4-(3-hydroxyphenyl)benzaldehyde | 22 |
| | | EG19-02-02R | | EG19-02 | 3-Fluoro-4-(5-hydroxypyridin-3-yl)benzaldehyde | |
| BB20 | mixEG20-02R | EG19-03-02R | mixEG20 | EG19-03 | Methyl 3-fluoro-4-(3-hydroxyphenyl)benzoate | 22 |

| | | | | | | |
|---|---|---|---|---|---|---|
| | | EG19-04-02R | | EG19-04 | Methyl 3-fluoro-4-(5-hydroxypyridin-3-yl)benzoate | |

Example 4-1-5: Synthesis of mixture mixEG21-02R

[Formula 528]

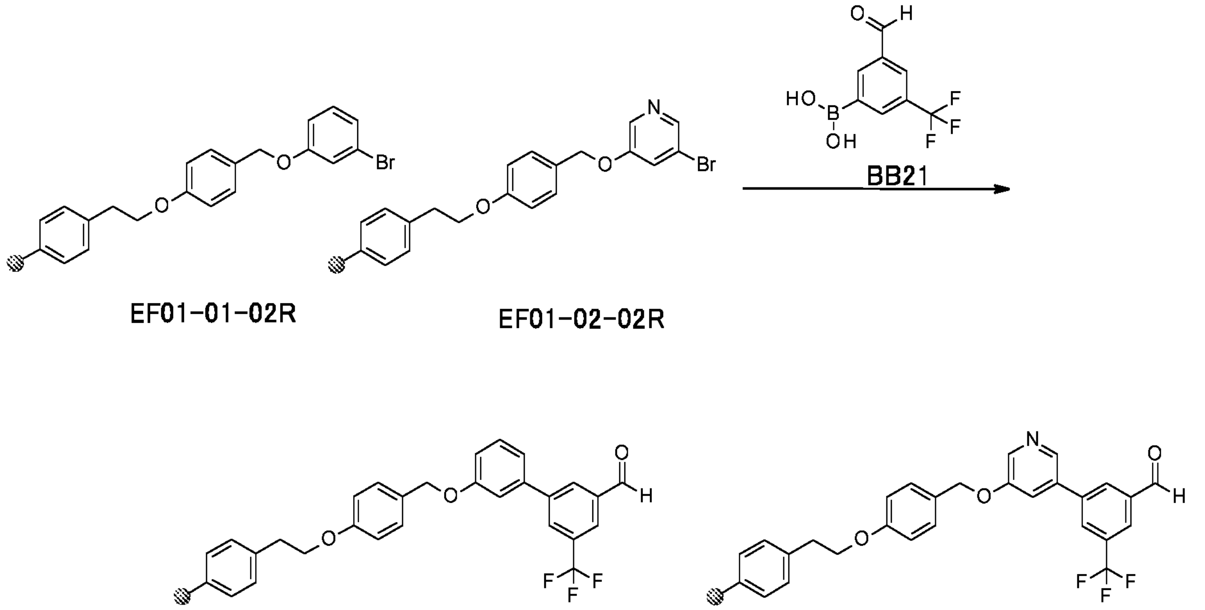


EF01-01-02R EF01-02-02R

EG21-01-02R EG21-02-02R

Under a nitrogen atmosphere, compound EF01-01-02R (57 mg, loading rate: 0.556 mmol/g, 0.032 mmol), compound EF01-02-02R (43 mg, loading rate: 0.733 mmol/g, 0.032 mmol), and 4-MeTHP (1.8 mL) were added to a 4 mL glass vial and shaken at room temperature for 1 hour. Under a nitrogen atmosphere, [3-formyl-5- (trifluoromethyl)phenyl]boronic acid (BB21) (84 mg, 0.387 mmol), $Na_2CO_3$ (41 mg, 0.387 mmol), a solution of $P(Cy)_3$ Pd G3 in 4-MeTHP (0.04 M, 323 μL, 13 μmol), and water (75 μL, 4.16 mmol) were added thereto, and the mixture was shaken at 90°C for 22 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), three times with DCM (2 mL), and three times with heptane (2 mL), and the resin was then dried under reduced pressure to obtain the title mixture mixEG21-02R shown in Table EG21. The building block used, compounds contained in the obtained mixture and the reaction time are also shown in Table EG21.

[Table 184]

[Table EG21]

| No. of BB used | No. of synthesized mixture | Resin compound No. | No. of mixture after cleavage | Compound No. after cleavage | Compound name | Reaction time (hr) |
|---|---|---|---|---|---|---|
| BB21 | mixEG21-02R | EG21-01-02R | mixEG21 | EG21-01 | 3-(3-Hydroxyphenyl)-5-(trifluoromethyl)benzaldehyde | 22 |
| | | EG21-02-02R | | EG21-02 | 3-(5-Hydroxypyridin-3-yl)-5-(trifluoromethyl)benzaldehyde | |

Example 4-1-6: Synthesis of mixture mixEG22-02R

The title mixture mixEG22-02R shown in Table EG22 was obtained by synthesis in the same manner as in Example 4-1-5 using compound EF01-01-02R (57 mg, loading rate: 0.556 mmol/g, 0.032 mmol), compound EF01-02-02R (43 mg, loading rate: 0.733 mmol/g, 0.032 mmol), and the boronic acid reagent (BB22) shown in Table LBB02. The building block used, compounds contained in the obtained mixture, and the reaction time are also shown in Table EG22.

[Table 185]

[Table EG22]

| No. of BB used | No. of synthesized mixture | Resin compound No. | No. of mixture after cleavage | Compound No. after cleavage | Compound name | Reaction time (hr) |
|---|---|---|---|---|---|---|
| BB22 | mixEG22-02R | EG21-03-02R | mixEG22 | EG21-03 | 3-(3-Hydroxyphenyl)-5-(trifluoromethoxy)benzaldehyde | 22 |
| | | EG21-04-02R | | EG21-04 | 3-(5-Hydroxypyridin-3-yl)-5-(trifluoromethoxy)benzaldehyde | |

Example 4-1-7-A: Synthesis of mixture mixEG23-02R

[Formula 529]

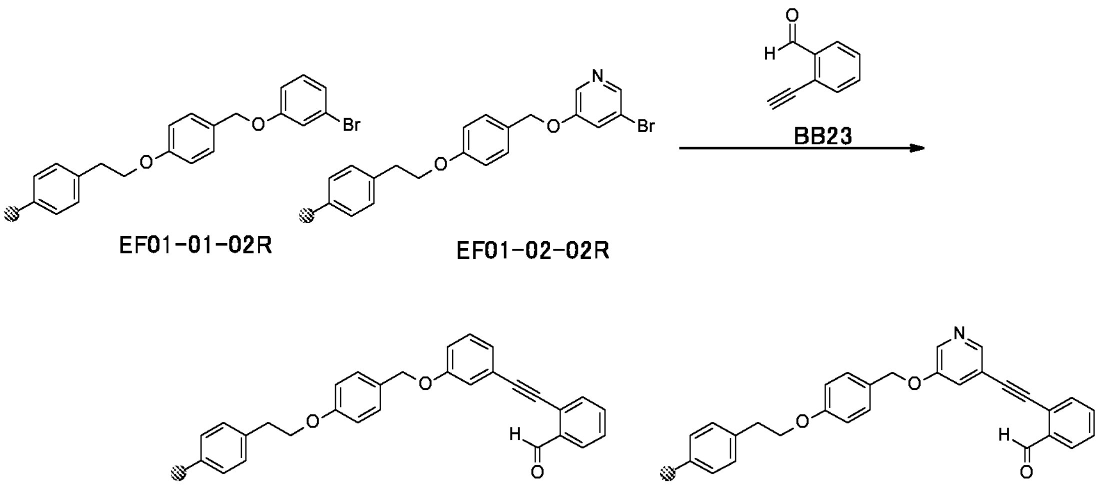

EF01-01-02R EF01-02-02R BB23

EG23-01-02R EG23-02-02R

Under a nitrogen atmosphere, compound EF01-01-02R (57 mg, loading rate: 0.556 mmol/g, 0.032 mmol), compound EF01-02-02R (43 mg, loading rate: 0.733 mmol/g, 0.032 mmol), NMP (1.11 mL), and diisopropylamine (188 μL) were added to a 3 mL glass vial and shaken at room temperature for 1 hour. Under a nitrogen atmosphere, 2-ethynylbenzaldehyde (BB23) (174 mg, 1.34 mmol) and XPhos Pd G4 (0.1 M, 200 μL, 20 μmol) were added thereto, and the mixture was then shaken at 80°C for 39 hours. The suspension of the reaction solution and the resin was transferred to a syringe with a filter using NMP and repetitively washed three times with NMP (2 mL), three times with a NMP:water (1:1) solution containing NAC (10 mg/mL) (2 mL), three times with water (2 mL), three times with MeOH (2 mL), three times with DCM (2 mL), and three times with heptane (2 mL), and the resin was then dried under reduced pressure to obtain the title mixture shown in Table EG23. The building block used, compounds contained in the obtained mixture, and the reaction time are also shown in Table EG23.

The solution of XPhos Pd G4 in NMP (0.1 M) was prepared by adding, under a nitrogen atmosphere, NMP (1 mL) to XPhos Pd G4 (86 mg, 0.1 mmol) in a 3 mL glass vial, and shaking the mixture for dissolution.

Example 4-1-7-B: Synthesis of mixtures mixEG24-02R to mixEG26-02R

The title mixtures shown in Table EG23 were synthesized in the same manner as in Example 4-1-7-A using compound EF01-01-02R (57 mg, loading rate: 0.556 mmol/g, 0.032 mmol), compound EF01-02-02R (43 mg, loading rate: 0.733 mmol/g, 0.032 mmol), and the acetylene reagents (BB23 to BB26) shown in Table LBB02. The building blocks used, compounds contained in the obtained mixtures, and the reaction times are also shown in Table EG23.

[Table 186]

[Table EG23]

| No. of BB used | No. of synthesized mixture | Resin compound No. | No. of mixture after cleavage | Compound No. after cleavage | Compound name | Reaction time (hr) |
|---|---|---|---|---|---|---|
| BB23 | mixEG23-02R | EG23-01-02R | mixEG23 | EG23-01 | 2-[2-(3-Hydroxyphenyl)ethynyl]benzaldehyde | 39 |
| | | EG23-02-02R | | EG23-02 | 2-[2-(5-Hydroxypyridin-3-yl)ethynyl]benzaldehyde | |
| BB24 | mixEG24-02R | EG23-03-02R | mixEG24 | EG23-03 | Methyl 2-((3-hydroxyphenyl)ethynyl)benzoate | 22 |
| | | EG23-04-02R | | EG23-04 | Methyl 2-((5-hydroxypyridin-3-yl)ethynyl)benzoate | |
| BB25 | mixEG25-02R | EG23-05-02R | mixEG25 | EG23-05 | Ethyl 3-[2-(3-hydroxyphenyl)ethynyl]-5-(trifluoromethyl)benzoate | 39 |
| | | EG23-06-02R | | EG23-06 | Ethyl 3-[2-(5-hydroxypyridin-3-yl)ethynyl]-5-(trifluoromethyl)benzoate | |
| BB26 | mixEG26-02R | EG23-07-02R | mixEG26 | EG23-07 | Ethyl 5-[2-(3-hydroxyphenyl)ethynyl]pyridine-3-carboxylate | 39 |
| | | EG23-08-02R | | EG23-08 | Ethyl 5-[2-(5-hydroxypyridin-3-yl)ethynyl]pyridine-3-carboxylate | |

Example 4-2-1: Synthesis of mixture mixEH01-02R

[Formula 530]

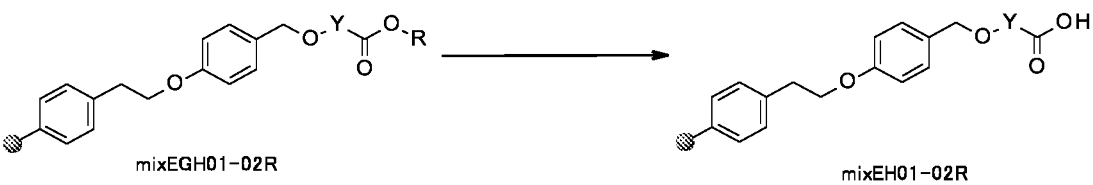

mixEGH01-02R mixEH01-02R

Mixture mixEGH01-02R (220 mg, loading rate: 0.651 mmol/g, 0.143 mmol) shown in Table EGH01, NMP (5.3 mL), and methanol (1.3 mL) were added to a 10 mL glass vial and shaken at room temperature for 1 hour. An aqueous sodium hydroxide solution (5 M, 198 μL, 0.992 mmol) was added thereto, and the mixture was shaken at room temperature for 17 hours. The suspension of the reaction solution and the resin was transferred to a syringe with a filter using NMP and repetitively washed three times with NMP (4 mL), three times with water (4 mL), three times with NMP (4 mL), three times with a solution of HOAt in NMP (0.2 M, 4 mL), three times with NMP (4 mL), three times with MeOH (4 mL), three times with DCM (3 mL), and three times with heptane (4 mL), and the resin was then dried under reduced pressure to obtain the title mixture (mixEH01-02R) shown in Table EH01. Compounds contained in the obtained mixture are shown in Table EH01.

[Table 187]

[Table EGH01] Mixture mixEGH01-02R

| No. of mixture used | Amount of mixture used (mg) | Resin compound No. | Compound No. after cleavage | Compound name |
|---|---|---|---|---|
| mixEG01-02R | 20.7 | EG01-01-02R | EG01-01 | Methyl 4-(3-hydroxyphenyl)-3-methylbenzoate |
| | | EG01-02-02R | EG01-02 | Methyl 4-(5-hydroxypyridin-3-yl)-3-methylbenzoate |
| mixEG01-03R | 20.0 | EG01-03-02R | EG01-03 | Ethyl 3-(3-hydroxyphenyl)-5-(trifluoromethyl)benzoate |
| | | EG01-04-02R | EG01-04 | Ethyl 3-(5-hydroxypyridin-3-yl)-5-(trifluoromethyl)benzoate |
| mixEG01-04R | 20.4 | EG01-05-02R | EG01-05 | Methyl 3-tert-butyl-5-(3-hydroxyphenyl)benzoate |
| | | EG01-06-02R | EG01-06 | Methyl 3-tert-butyl-5-(5-hydroxypyridin-3-yl)benzoate |
| mixEG01-05R | 20.3 | EG01-07-02R | EG01-07 | Ethyl 3-[2-(3-hydroxyphenyl)phenyl]propanoate |
| | | EG01-08-02R | EG01-08 | Ethyl 3-[2-(5-hydroxypyridin-3-yl)phenyl]propanoate |
| mixEG01-06R | 19.9 | EG01-09-02R | EG01-09 | Methyl 4-(3-hydroxyphenyl)naphthalene-1-carboxylate |
| | | EG01-10-02R | EG01-10 | Methyl 4-(5-hydroxypyridin-3-yl)naphthalene-1-carboxylate |
| mixEG01-07R | 20.3 | EG01-11-02R | EG01-11 | Ethyl 3-[4-(3-hydroxyphenyl)pyrazol-1-yl]-5-(trifluoromethyl)benzoate |
| | | EG01-12-02R | EG01-12 | Ethyl 3-[4-(5-hydroxypyridin-3-yl)pyrazol-1-yl]-5-(trifluoromethyl)benzoate |
| mixEG12-02R | 20.0 | EG08-09-02R | EG08-09 | Methyl 4-(3-hydroxyphenyl)thiophene-2-carboxylate |
| | | EG08-10-02R | EG08-10 | Methyl 4-(5-hydroxypyridin-3-yl)thiophene-2-carboxylate |
| mixEG20-02R | 20.4 | EG19-03-02R | EG19-03 | Methyl 3-fluoro-4-(3-hydroxyphenyl)benzoate |
| | | EG19-04-02R | EG19-04 | Methyl 3-fluoro-4-(5-hydroxypyridin-3-yl)benzoate |
| mixEG24-02R | 19.5 | EG23-03-02R | EG23-03 | Methyl 2-((3-hydroxyphenyl)ethynyl)benzoate |
| | | EG23-04-02R | EG23-04 | Methyl 2-((5-hydroxypyridin-3-yl)ethynyl)benzoate |
| mixEG25-02R | 19.5 | EG23-05-02R | EG23-05 | Ethyl 3-[2-(3-hydroxyphenyl)ethynyl]-5-(trifluoromethyl)benzoate |
| | | EG23-06-02R | EG23-06 | Ethyl 3-[2-(5-hydroxypyridin-3-yl)ethynyl]-5-(trifluoromethyl)benzoate |
| mixEG26-02R | 19.4 | EG23-07-02R | EG23-07 | Ethyl 5-[2-(3-hydroxyphenyl)ethynyl]pyridine-3-carboxylate |
| | | EG23-08-02R | EG23-08 | Ethyl 5-[2-(5-hydroxypyridin-3-yl)ethynyl]pyridine-3-carboxylate |

[Table 188]

[Table EH01] Mixture No: mixEH01-02R, Mixture No. after cleavage: mixEH01

| Resin compound No. | Compound No. after cleavage | Compound name |
|---|---|---|
| EH01-01-02R | EH01-01 | 4-(3-Hydroxyphenyl)thiophene-2-carboxylic acid |
| EH01-02-02R | EH01-02 | 4-(5-Hydroxypyridin-3-yl)thiophene-2-carboxylic acid |
| EH01-03-02R | EH01-03 | 4-(3-Hydroxyphenyl)-3-methylbenzoic acid |
| EH01-04-02R | EH01-04 | 4-(5-Hydroxypyridin-3-yl)-3-methylbenzoic acid |
| EH01-05-02R | EH01-05 | 3-Fluoro-4-(3-hydroxyphenyl)benzoic acid |
| EH01-06-02R | EH01-06 | 3-Fluoro-4-(5-hydroxypyridin-3-yl)benzoic acid |
| EH01-07-02R | EH01-07 | 2-[2-(3-Hydroxyphenyl)ethynyl]benzoic acid |
| EH01-08-02R | EH01-08 | 2-[2-(5-Hydroxypyridin-3-yl)ethynyl]benzoic acid |
| EH01-09-02R | EH01-09 | 5-[2-(3-Hydroxyphenyl)ethynyl]pyridine-3-carboxylic acid |
| EH01-10-02R | EH01-10 | 5-[2-(5-Hydroxypyridin-3-yl)ethynyl]pyridine-3-carboxylic acid |
| EH01-11-02R | EH01-11 | 3-[2-(3-Hydroxyphenyl)phenyl]propanoic acid |
| EH01-12-02R | EH01-12 | 3-[2-(5-Hydroxypyridin-3-yl)phenyl]propanoic acid |
| EH01-13-02R | EH01-13 | 4-(3-Hydroxyphenyl)naphthalene-1-carboxylic acid |
| EH01-14-02R | EH01-14 | 4-(5-Hydroxypyridin-3-yl)naphthalene-1-carboxylic acid |
| EH01-15-02R | EH01-15 | 3-tert-Butyl-5-(3-hydroxyphenyl)benzoic acid |
| EH01-16-02R | EH01-16 | 3-tert-Butyl-5-(5-hydroxypyridin-3-yl)benzoic acid |
| EH01-17-02R | EH01-17 | 3-(3-Hydroxyphenyl)-5-(trifluoromethyl)benzoic acid |
| EH01-18-02R | EH01-18 | 3-(5-Hydroxypyridin-3-yl)-5-(trifluoromethyl)benzoic acid |
| EH01-19-02R | EH01-19 | 3-[2-(3-Hydroxyphenyl)ethynyl]-5-(trifluoromethyl)benzoic acid |
| EH01-20-02R | EH01-20 | 3-[2-(5-Hydroxypyridin-3-yl)ethynyl]-5-(trifluoromethyl)benzoic acid |
| EH01-21-02R | EH01-21 | 3-[4-(3-Hydroxyphenyl)pyrazol-1-yl]-5-(trifluoromethyl)benzoic acid |
| EH01-22-02R | EH01-22 | 3-[4-(5-Hydroxypyridin-3-yl)pyrazol-1-yl]-5-(trifluoromethyl)benzoic acid |

Example 4-2-2: Synthesis of mixture mixEH02-02R

[Formula 531]

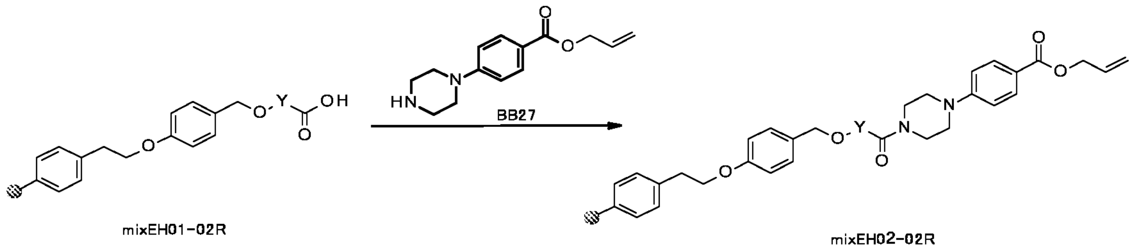


mixEH01-02R

mixEH02-02R

Mixture mixEH01-02R (173 mg, loading rate: 0.651 mmol/g, 0.113 mmol) shown in Table EH01 and NMP (4.4 mL) were added to an 8 mL glass vial and shaken at room temperature for 1 hour. Allyl 4-(piperazin-1-yl)benzoate (BB27, 83 mg, 0.339 mmol), DIC (106 μL, 0.677 mmol), and HOAt (92 mg, 0.677 mmol) were added thereto, and the mixture was shaken at 40°C for 20 hours. The suspension of the reaction solution and the resin was transferred to a syringe with a filter using NMP and repetitively washed three times with NMP (4 mL), three times with MeOH (4 mL), three times with DCM (4 mL), and three times with heptane (4 mL), and the resin was then dried under reduced pressure to obtain the title mixture shown in Table EH02. Compounds contained in the obtained mixture are shown in Table EH02.

The results of Example 3-5-2 suggest the possibility that this amidation reaction lacked two compounds (EH02-07-02R and EH02-08-02R) among the compounds described in Table EH02.

[Table 189]

[Table EH02] Mixture No: mixEH02-02R; Mixture No. after cleavage: mixEH02

| Resin compound No. | Compound No. after cleavage | Compound name |
|---|---|---|
| EH02-01-02R | EH02-01 | Prop-2-enyl 4-[4-[4-(3-hydroxyphenyl)thiophene-2-carbonyl]piperazin-1-yl]benzoate |
| EH02-02-02R | EH02-02 | Prop-2-enyl 4-[4-[4-(5-hydroxypyridin-3-yl)thiophene-2-carbonyl]piperazin-1-yl]benzoate |
| EH02-03-02R | EH02-03 | Prop-2-enyl 4-[4-[4-(3-hydroxyphenyl)-3-methylbenzoyl]piperazin-1-yl]benzoate |
| EH02-04-02R | EH02-04 | Prop-2-enyl 4-[4-[4-(5-hydroxypyridin-3-yl)-3-methylbenzoyl]piperazin-1-yl]benzoate |
| EH02-05-02R | EH02-05 | Prop-2-enyl 4-[4-[3-fluoro-4-(3-hydroxyphenyl)benzoyl]piperazin-1-yl]benzoate |
| EH02-06-02R | EH02-06 | Prop-2-enyl 4-[4-[3-fluoro-4-(5-hydroxypyridin-3-yl)benzoyl]piperazin-1-yl]benzoate |
| EH02-07-02R | EH02-07 | Prop-2-enyl 4-[4-[2-[2-(3-hydroxyphenyl)ethynyl]benzoyl]piperazin-1-yl]benzoate |
| EH02-08-02R | EH02-08 | Prop-2-enyl 4-[4-[2-[2-(5-hydroxypyridin-3-yl)ethynyl]benzoyl]piperazin-1-yl]benzoate |
| EH02-09-02R | EH02-09 | Prop-2-enyl 4-[4-[5-[2-(3-hydroxyphenyl)ethynyl]pyridine-3-carbonyl]piperazin-1-yl]benzoate |
| EH02-10-02R | EH02-10 | Prop-2-enyl 4-[4-[5-[2-(5-hydroxypyridin-3-yl)ethynyl]pyridine-3-carbonyl]piperazin-1-yl]benzoate |
| EH02-11-02R | EH02-11 | Prop-2-enyl 4-[4-[3-[2-(3-hydroxyphenyl)phenyl]propanoyl]piperazin-1-yl]benzoate |
| EH02-12-02R | EH02-12 | Prop-2-enyl 4-[4-[3-[2-(5-hydroxypyridin-3-yl)phenyl]propanoyl]piperazin-1-yl]benzoate |
| EH02-13-02R | EH02-13 | Prop-2-enyl 4-[4-[4-(3-hydroxyphenyl)naphthalene-1-carbonyl]piperazin-1-yl]benzoate |
| EH02-14-02R | EH02-14 | Prop-2-enyl 4-[4-[4-(5-hydroxypyridin-3-yl)naphthalene-1-carbonyl]piperazin-1-yl]benzoate |
| EH02-15-02R | EH02-15 | Prop-2-enyl 4-[4-[3-tert-butyl-5-(3-hydroxyphenyl)benzoyl]piperazin-1-yl]benzoate |
| EH02-16-02R | EH02-16 | Prop-2-enyl 4-[4-[3-tert-butyl-5-(5-hydroxypyridin-3-yl)benzoyl]piperazin-1-yl]benzoate |
| EH02-17-02R | EH02-17 | Prop-2-enyl 4-[4-[3-(3-hydroxyphenyl)-5-(trifluoromethyl)benzoyl]piperazin-1-yl]benzoate |
| EH02-18-02R | EH02-18 | Prop-2-enyl 4-[4-[3-(5-hydroxypyridin-3-yl)-5-(trifluoromethyl)benzoyl]piperazin-1-yl]benzoate |
| EH02-19-02R | EH02-19 | Prop-2-enyl 4-[4-[3-[2-(3-hydroxyphenyl)ethynyl]-5-(trifluoromethyl)benzoyl]piperazin-1-yl]benzoate |
| EH02-20-02R | EH02-20 | Prop-2-enyl 4-[4-[3-[2-(5-hydroxypyridin-3-yl)ethynyl]-5-(trifluoromethyl)benzoyl]piperazin-1-yl]benzoate |
| EH02-21-02R | EH02-21 | Prop-2-enyl 4-[4-[3-[4-(3-hydroxyphenyl)pyrazol-1-yl]-5-(trifluoromethyl)benzoyl]piperazin-1-yl]benzoate |
| EH02-22-02R | EH02-22 | Prop-2-enyl 4-[4-[3-[4-(5-hydroxypyridin-3-yl)pyrazol-1-yl]-5-(trifluoromethyl)benzoyl]piperazin-1-yl]benzoate |

Example 4-2-3: Synthesis of mixture mixEH03-02R

[Formula 532]

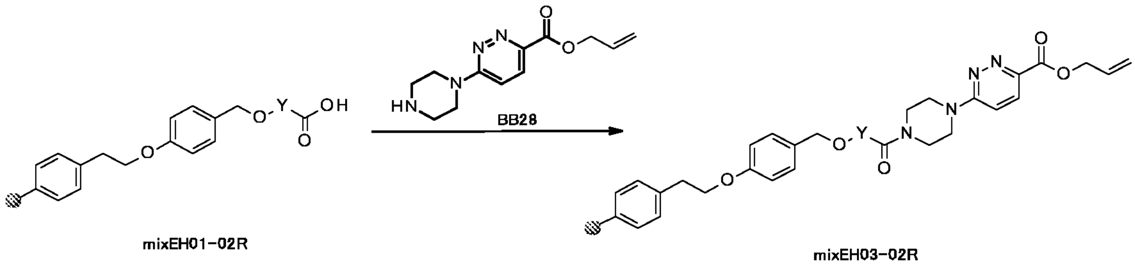


mixEH01-02R

mixEH03-02R

The title mixture shown in Table EH03 was obtained in the same manner as in Example 4-2-2 using allyl 6-(piperazin-1-yl)pyridazine-3-carboxylate (BB28) and mixture mixEH01-02R (175 mg, loading rate: 0.651 mmol/g, 0.114 mmol) shown in Table EH01. Compounds contained in the obtained mixture are shown in Table EH03.

The results of Example 3-5-3 suggest the possibility that this amidation reaction lacked two compounds (EH03-07-02R and EH03-08-02R) among the compounds described in Table EH03.

[Table 190]

[Table EH03] Mixture No: mixEH03-02R; Mixture No. after cleavage: mixEH03

| Resin compound No. | Compound No. after cleavage | Compound name |
|---|---|---|
| EH03-01-02R | EH03-01 | Prop-2-enyl 6-[4-[4-(3-hydroxyphenyl)thiophene-2-carbonyl]piperazin-1-yl]pyridazine-3-carboxylate |
| EH03-02-02R | EH03-02 | Prop-2-enyl 6-[4-[4-(5-hydroxypyridin-3-yl)thiophene-2-carbonyl]piperazin-1-yl]pyridazine-3-carboxylate |
| EH03-03-02R | EH03-03 | Prop-2-enyl 6-[4-[4-(3-hydroxyphenyl)-3-methylbenzoyl]piperazin-1-yl]pyridazine-3-carboxylate |
| EH03-04-02R | EH03-04 | Prop-2-enyl 6-[4-[4-(5-hydroxypyridin-3-yl)-3-methylbenzoyl]piperazin-1-yl]pyridazine-3-carboxylate |

| | | |
|---|---|---|
| EH03-05-02R | EH03-05 | Prop-2-enyl 6-[4-[3-fluoro-4-(3-hydroxyphenyl)benzoyl]piperazin-1-yl]pyridazine-3-carboxylate |
| EH03-06-02R | EH03-06 | Prop-2-enyl 6-[4-[3-fluoro-4-(5-hydroxypyridin-3-yl)benzoyl]piperazin-1-yl]pyridazine-3-carboxylate |
| EH03-07-02R | EH03-07 | Prop-2-enyl 6-[4-[2-[2-(3-hydroxyphenyl)ethynyl]benzoyl]piperazin-1-yl]pyridazine-3-carboxylate |
| EH03-08-02R | EH03-08 | Prop-2-enyl 6-[4-[2-[2-(5-hydroxypyridin-3-yl)ethynyl]benzoyl]piperazin-1-yl]pyridazine-3-carboxylate |
| EH03-09-02R | EH03-09 | Prop-2-enyl 6-[4-[5-[2-(3-hydroxyphenyl)ethynyl]pyridine-3-carbonyl]piperazin-1-yl]pyridazine-3-carboxylate |
| EH03-10-02R | EH03-10 | Prop-2-enyl 6-[4-[5-[2-(5-hydroxypyridin-3-yl)ethynyl]pyridine-3-carbonyl]piperazin-1-yl]pyridazine-3-carboxylate |
| EH03-11-02R | EH03-11 | Prop-2-enyl 6-[4-[3-[2-(3-hydroxyphenyl)phenyl]propanoyl]piperazin-1-yl]pyridazine-3-carboxylate |
| EH03-12-02R | EH03-12 | Prop-2-enyl 6-[4-[3-[2-(5-hydroxypyridin-3-yl)phenyl]propanoyl]piperazin-1-yl]pyridazine-3-carboxylate |
| EH03-13-02R | EH03-13 | Prop-2-enyl 6-[4-[4-(3-hydroxyphenyl)naphthalene-1-carbonyl]piperazin-1-yl]pyridazine-3-carboxylate |
| EH03-14-02R | EH03-14 | Prop-2-enyl 6-[4-[4-(5-hydroxypyridin-3-yl)naphthalene-1-carbonyl]piperazin-1-yl]pyridazine-3-carboxylate |
| EH03-15-02R | EH03-15 | Prop-2-enyl 6-[4-[3-tert-butyl-5-(3-hydroxyphenyl)benzoyl]piperazin-1-yl]pyridazine-3-carboxylate |
| EH03-16-02R | EH03-16 | Prop-2-enyl 6-[4-[3-tert-butyl-5-(5-hydroxypyridin-3-yl)benzoyl]piperazin-1-yl]pyridazine-3-carboxylate |
| EH03-17-02R | EH03-17 | Prop-2-enyl 6-[4-[3-(3-hydroxyphenyl)-5-(trifluoromethyl)benzoyl]piperazin-1-yl]pyridazine-3-carboxylate |
| EH03-18-02R | EH03-18 | Prop-2-enyl 6-[4-[3-(5-hydroxypyridin-3-yl)-5-(trifluoromethyl)benzoyl]piperazin-1-yl]pyridazine-3-carboxylate |
| EH03-19-02R | EH03-19 | Prop-2-enyl 6-[4-[3-[2-(3-hydroxyphenyl)ethynyl]-5-(trifluoromethyl)benzoyl]piperazin-1-yl]pyridazine-3-carboxylate |
| EH03-20-02R | EH03-20 | Prop-2-enyl 6-[4-[3-[2-(5-hydroxypyridin-3-yl)ethynyl]-5-(trifluoromethyl)benzoyl]piperazin-1-yl]pyridazine-3-carboxylate |
| EH03-21-02R | EH03-21 | Prop-2-enyl 6-[4-[3-[4-(3-hydroxyphenyl)pyrazol-1-yl]-5-(trifluoromethyl)benzoyl]piperazin-1-yl]pyridazine-3-carboxylate |
| EH03-22-02R | EH03-22 | Prop-2-enyl 6-[4-[3-[4-(5-hydroxypyridin-3-yl)pyrazol-1-yl]-5-(trifluoromethyl)benzoyl]piperazin-1-yl]pyridazine-3-carboxylate |

Example 4-2-4: Synthesis of mixture mixEH04-02R

[Formula 533]

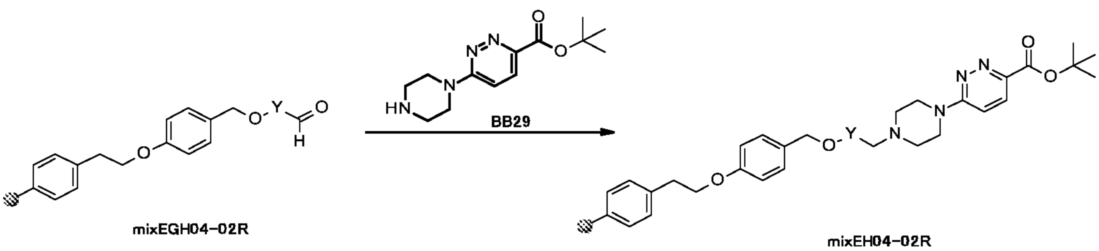


mixEGH04-02R

mixEH04-02R

Under a nitrogen atmosphere, mixEG07-02R (20.3 mg), mixEG08-02R (21.0 mg), mixEG09-02R (21.6 mg), mixEG10-02R (19.8 mg), mixEG13-02R (20.6 mg), mixEG14- 02R (21.1 mg), mixEG15-02R (20.5 mg), mixEG16-02R (20.5 mg), mixEG17-02R (20.5 mg), mixEG19-02R (20.1 mg), mixEG21-02R (21.9 mg), mixEG22-02R (20.4 mg), and

mixEG23-02R (20.6 mg) shown in Table EGH04 were transferred to a 10 mL screw-cap vial to prepare solid-phase supported aromatic aldehyde mixture mixEGH04-02R (265 mg, loading rate: 0.651 mmol/g, 0.172 mmol) for use in this Example. DCE (5.57 mL, 21 v/w) was added thereto, and the mixture was shaken at room temperature for 1 hour. Under a nitrogen atmosphere, tert-butyl 6-piperazin-1-ylpyridazine-3-carboxylate (BB29) (137 mg) and $Ti(OtBu)_4$ (333 μL) were added thereto, and the mixture was shaken at 60°C for 1 hour. Then, under a nitrogen atmosphere, sodium triacetoxyborohydride (439 mg) and a 2 v/v% solution of AcOH in DCE (2.39 mL) were added thereto, and the mixture was shaken at room temperature for 20 hours. MeOH (1 mL) was added to the reaction solution, and then, the resin suspension was transferred to a syringe with a filter using MeOH (1 mL) (this operation was repeated a total of six times), repetitively washed with MeOH (5.3 mL), three times with NMP (5.3 mL), three times with a $NaHCO_3$ solution (0.15 M, NMP:HzO = 1:5 solution, 5.3 mL), three times with water (5.3 mL), three times with MeOH (5.3 mL), three times with DCM (5.3 mL), and three times with heptane (5.3 mL), and then dried under reduced pressure to obtain the title mixture mixEH04-02R. Compounds contained in the mixture are shown in Table EH04.

[Table 191]

[Table EGH04] Mixture No: mixEGH04-02R; Mixture No. after cleavage: mixEGH04

| No. of mixture used | Resin compound No. | Compound No. after cleavage | Compound name |
|---|---|---|---|
| mixEG07-02R | EG07-0102R | EG07-01 | 4-(3-Hydroxyphenyl)-3-methylbenzaldehyde |
| | EG07-0202R | EG07-02 | 4-(5-Hydroxypyridin-3-yl)-3-methylbenzaldehyde |
| mixEG08-02R | EG07-0302R | EG07-03 | 2-(3-Hydroxyphenyl)benzaldehyde |
| | EG07-0402R | EG07-04 | 2-(5-Hydroxypyridin-3-yl)benzaldehyde |
| mixEG09-02R | EG07-0502R | EG07-05 | 4-(3-Hydroxyphenyl)naphthalene-1-carbaldehyde |
| | EG07-0602R | EG07-06 | 4-(5-Hydroxypyridin-3-yl)naphthalene-1-carbaldehyde |
| mixEG10-02R | EG07-0702R | EG07-07 | 4-(3-Hydroxyphenyl)thiophene-2-carbaldehyde |
| | EG07-0802R | EG07-08 | 4-(5-Hydroxypyridin-3-yl)thiophene-2-carbaldehyde |
| mixEG13-02R | EG07-1302R | EG07-13 | 2-Ethyl-4-(3-hydroxyphenyl)benzaldehyde |
| | EG07-1402R | EG07-14 | 2-Ethyl-4-(5-hydroxypyridin-3-yl)benzaldehyde |
| mixEG14-02R | EG07-1502R | EG07-15 | 4-(3-Hydroxyphenyl)-2-methoxybenzaldehyde |
| | EG07-1602R | EG07-16 | 4-(5-Hydroxypyridin-3-yl)-2-methoxybenzaldehyde |
| mixEG15-02R | EG07-1702R | EG07-17 | 3-Ethoxy-5-(3-hydroxyphenyl)benzaldehyde |
| | EG07-1802R | EG07-18 | 3-Ethoxy-5-(5-hydroxypyridin-3-yl)benzaldehyde |
| mixEG16-02R | EG07-1902R | EG07-19 | 3-(3-Hydroxyphenyl)-5-propan-2-yloxybenzaldehyde |
| | EG07-2002R | EG07-20 | 3-(5-Hydroxypyridin-3-yl)-5-propan-2-yloxybenzaldehyde |
| mixEG17-02R | EG07-2102R | EG07-21 | 4-(3-Hydroxyphenyl)-2,6-dimethoxybenzaldehyde |
| | EG07-2202R | EG07-22 | 4-(5-Hydroxypyridin-3-yl)-2,6-dimethoxybenzaldehyde |
| mixEG19-02R | EG19-01-02R | EG19-01 | 3-Fluoro-4-(3-hydroxyphenyl)benzaldehyde |
| | EG19-02-02R | EG19-02 | 3-Fluoro-4-(5-hydroxypyridin-3-yl)benzaldehyde |
| mixEG21-02R | EG21-01-02R | EG21-01 | 3-(3-Hydroxyphenyl)-5-(trifluoromethyl)benzaldehyde |
| | EG21-02-02R | EG21-02 | 3-(5-Hydroxypyridin-3-yl)-5-(trifluoromethyl)benzaldehyde |
| mixEG22-02R | EG21-03-02R | EG21-03 | 3-(3-Hydroxyphenyl)-5-(trifluoromethoxy)benzaldehyde |
| | EG21-04-02R | EG21-04 | 3-(5-Hydroxypyridin-3-yl)-5-(trifluoromethoxy)benzaldehyde |
| mixEG23-02R | EG23-01-02R | EG23-01 | 2-[2-(3-Hydroxyphenyl)ethynyl]benzaldehyde |
| | EG23-02-02R | EG23-02 | 2-[2-(5-Hydroxypyridin-3-yl)ethynyl]benzaldehyde |

[Table 192]

[Table EH04] Mixture No: mixEH04-02R; Mixture No. after cleavage: mixEH04

| Resin compound No. | Compound No. after cleavage | Compound name |
|---|---|---|
| EH04-01-02R | EH04-01 | tert-Butyl 6-[4-[[2-(3-hydroxyphenyl)phenyl]methyl]piperazin-1-yl]pyridazine-3-carboxylate |
| EH04-02-02R | EH04-02 | tert-Butyl 6-[4-[[2-(5-hydroxypyridin-3-yl)phenyl]methyl]piperazin-1-yl]pyridazine-3-carboxylate |
| EH04-03-02R | EH04-03 | tert-Butyl 6-[4-[[4-(3-hydroxyphenyl)-3-methylphenyl]methyl]piperazin-1-yl]pyridazine-3-carboxylate |
| EH04-04-02R | EH04-04 | tert-Butyl 6-[4-[[4-(5-hydroxypyridin-3-yl)-3-methylphenyl]methyl]piperazin-1-yl]pyridazine-3-carboxylate |
| EH04-05-02R | EH04-05 | tert-Butyl 6-[4-[[2-ethyl-4-(3-hydroxyphenyl)phenyl]methyl]piperazin-1-yl]pyridazine-3-carboxylate |
| EH04-06-02R | EH04-06 | tert-Butyl 6-[4-[[2-ethyl-4-(5-hydroxypyridin-3-yl)phenyl]methyl]piperazin-1-yl]pyridazine-3-carboxylate |
| EH04-07-02R | EH04-07 | tert-Butyl 6-[4-[[3-fluoro-4-(3-hydroxyphenyl)phenyl]methyl]piperazin-1-yl]pyridazine-3-carboxylate |
| EH04-08-02R | EH04-08 | tert-Butyl 6-[4-[[3-fluoro-4-(5-hydroxypyridin-3-yl)phenyl]methyl]piperazin-1-yl]pyridazine-3-carboxylate |
| EH04-09-02R | EH04-09 | tert-Butyl 6-[4-[[4-(3-hydroxyphenyl)-2-methoxyphenyl]methyl]piperazin-1-yl]pyridazine-3-carboxylate |
| EH04-10-02R | EH04-10 | tert-Butyl 6-[4-[[4-(5-hydroxypyridin-3-yl)-2-methoxyphenyl]methyl]piperazin-1-yl]pyridazine-3-carboxylate |
| EH04-11-02R | EH04-11 | tert-Butyl 6-[4-[[3-ethoxy-5-(3-hydroxyphenyl)phenyl]methyl]piperazin-1-yl]pyridazine-3-carboxylate |
| EH04-12-02R | EH04-12 | tert-Butyl 6-[4-[[3-ethoxy-5-(5-hydroxypyridin-3-yl)phenyl]methyl]piperazin-1-yl]pyridazine-3-carboxylate |
| EH04-13-02R | EH04-13 | tert-Butyl 6-[4-[[3-(3-hydroxyphenyl)-5-propan-2-yloxyphenyl]methyl]piperazin-1-yl]pyridazine-3-carboxylate |
| EH04-14-02R | EH04-14 | tert-Butyl 6-[4-[[3-(5-hydroxypyridin-3-yl)-5-propan-2-yloxyphenyl]methyl]piperazin-1-yl]pyridazine-3-carboxylate |
| EH04-15-02R | EH04-15 | tert-Butyl 6-[4-[[4-(3-hydroxyphenyl)-2,6-dimethoxyphenyl]methyl]piperazin-1-yl]pyridazine-3-carboxylate |
| EH04-16-02R | EH04-16 | tert-Butyl 6-[4-[[4-(5-hydroxypyridin-3-yl)-2,6-dimethoxyphenyl]methyl]piperazin-1-yl]pyridazine-3-carboxylate |
| EH04-17-02R | EH04-17 | tert-Butyl 6-[4-[[3-(3-hydroxyphenyl)-5-(trifluoromethyl)phenyl]methyl]piperazin-1-yl]pyridazine-3-carboxylate |
| EH04-18-02R | EH04-18 | tert-Butyl 6-[4-[[3-(5-hydroxypyridin-3-yl)-5-(trifluoromethyl)phenyl]methyl]piperazin-1-yl]pyridazine-3-carboxylate |
| EH04-19-02R | EH04-19 | tert-Butyl 6-[4-[[3-(3-hydroxyphenyl)-5-(trifluoromethoxy)phenyl]methyl]piperazin-1-yl]pyridazine-3-carboxylate |

| | | |
|---|---|---|
| EH04-20-02R | EH04-20 | tert-Butyl 6-[4-[[3-(5-hydroxypyridin-3-yl)-5-(trifluoromethoxy)phenyl]methyl]piperazin-1-yl]pyridazine-3-carboxylate |
| EH04-21-02R | EH04-21 | tert-Butyl 6-[4-[[4-(3-hydroxyphenyl)naphthalen-1-yl]methyl]piperazin-1-yl]pyridazine-3-carboxylate |
| EH04-22-02R | EH04-22 | tert-Butyl 6-[4-[[4-(5-hydroxypyridin-3-yl)naphthalen-1-yl]methyl]piperazin-1-yl]pyridazine-3-carboxylate |
| EH04-23-02R | EH04-23 | tert-Butyl 6-[4-[[4-(3-hydroxyphenyl)thiophen-2-yl]methyl]piperazin-1-yl]pyridazine-3-carboxylate |
| EH04-24-02R | EH04-24 | tert-Butyl 6-[4-[[4-(5-hydroxypyridin-3-yl)thiophen-2-yl]methyl]piperazin-1-yl]pyridazine-3-carboxylate |
| EH04-25-02R | EH04-25 | tert-Butyl 6-[4-[[2-[2-(3-hydroxyphenyl)ethynyl]phenyl]methyl]piperazin-1-yl]pyridazine-3-carboxylate |
| EH04-26-02R | EH04-26 | tert-Butyl 6-[4-[[2-[2-(5-hydroxypyridin-3-yl)ethynyl]phenyl]methyl]piperazin-1-yl]pyridazine-3-carboxylate |

Example 4-2-5: Synthesis of mixture mixEH05-02R

[Formula 534]

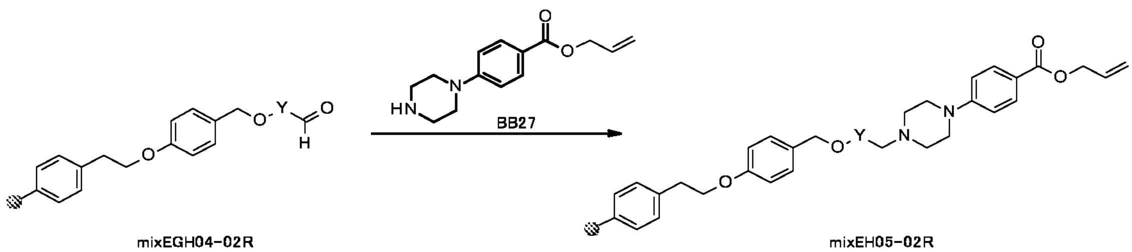


mixEGH04-02R

mixEH05-02R

Under a nitrogen atmosphere, mixEG07-02R (20.1 mg), mixEG08-02R (20.3 mg), mixEG09-02R (20.4 mg), mixEG10-02R (20.3 mg), mixEG13-02R (20.8 mg), mixEG14- 02R (20.6 mg), mixEG15-02R (20.1 mg), mixEG16-02R (20.0 mg), mixEG17-02R (20.3 mg), mixEG19-02R (20.8 mg), mixEG21-02R (20.5 mg), mixEG22-02R (20.8 mg), and mixEG23-02R (20.6 mg) shown in Table EGH04 were transferred to a 10 mL screw-cap vial to prepare solid-phase supported aromatic aldehyde mixture mixEGH04-02R (260 mg, loading rate: 0.651 mmol/g) for use in this Example. DCE (5.46 mL, 21 v/w) was added thereto, and the mixture was shaken at room temperature for 1 hour. Under a nitrogen atmosphere, allyl 6-piperazin-1-ylpyridazine-3-carboxylate (BB27) (125 mg) and $Ti(OtBu)_4$ (317 μL) were added thereto, and the mixture was shaken at 60°C for 1 hour. Then, under a nitrogen atmosphere, sodium triacetoxyborohydride (430 mg) and a 2 v/v% solution of AcOH in DCE (2.34 mL) were added thereto, and the mixture was shaken at 50°C for 3 hours. After cooling to room temperature, the resin suspension was transferred to a column with a filter using MeOH (1 mL) (this operation was repeated a total of six times), repetitively washed with MeOH (5.3 mL), three times with NMP (5.3 mL), three times with a $NaHCO_3$ solution (0.15 M, NMP:H2O = 1:5 solution, 5.3 mL), three times with water (5.3 mL), three times with MeOH (5.3 mL), three times with DCM (5.3 mL), and three times with heptane (5.3 mL), and then dried under reduced pressure to obtain the title mixture mixEH05-02R shown in Table EH05. Compounds contained in the mixture are shown in Table EH05.

[Table 193]

[Table EH05] Mixture No: mixEH05-02R; Mixture No. after cleavage: mixEH05

| Resin compound No. | Compound No. after cleavage | Compound name |
|---|---|---|
| EH05-01-02R | EH05-01 | Prop-2-enyl 4-[4-[[2-(3-hydroxyphenyl)phenyl]methyl]piperazin-1-yl]benzoate |
| EH05-02-02R | EH05-02 | Prop-2-enyl 4-[4-[[2-(5-hydroxypyridin-3-yl)phenyl]methyl]piperazin-1-yl]benzoate |
| EH05-03-02R | EH05-03 | Prop-2-enyl 4-[4-[[4-(3-hydroxyphenyl)-3-methylphenyl]methyl]piperazin-1-yl]benzoate |
| EH05-04-02R | EH05-04 | Prop-2-enyl 4-[4-[[4-(5-hydroxypyridin-3-yl)-3-methylphenyl]methyl]piperazin-1-yl]benzoate |
| EH05-05-02R | EH05-05 | Prop-2-enyl 4-[4-[[2-ethyl-4-(3-hydroxyphenyl)phenyl]methyl]piperazin-1-yl]benzoate |
| EH05-06-02R | EH05-06 | Prop-2-enyl 4-[4-[[2-ethyl-4-(5-hydroxypyridin-3-yl)phenyl]methyl]piperazin-1-yl]benzoate |
| EH05-07-02R | EH05-07 | Prop-2-enyl 4-[4-[[3-fluoro-4-(3-hydroxyphenyl)phenyl]methyl]piperazin-1-yl]benzoate |
| EH05-08-02R | EH05-08 | Prop-2-enyl 4-[4-[[3-fluoro-4-(5-hydroxypyridin-3-yl)phenyl]methyl]piperazin-1-yl]benzoate |
| EH05-09-02R | EH05-09 | Prop-2-enyl 4-[4-[[4-(3-hydroxyphenyl)-2-methoxyphenyl]methyl]piperazin-1-yl]benzoate |
| EH05-10-02R | EH05-10 | Prop-2-enyl 4-[4-[[4-(5-hydroxypyridin-3-yl)-2-methoxyphenyl]methyl]piperazin-1-yl]benzoate |
| EH05-11-02R | EH05-11 | Prop-2-enyl 4-[4-[[3-ethoxy-5-(3-hydroxyphenyl)phenyl]methyl]piperazin-1-yl]benzoate |
| EH05-12-02R | EH05-12 | Prop-2-enyl 4-[4-[[3-ethoxy-5-(5-hydroxypyridin-3-yl)phenyl]methyl]piperazin-1-yl]benzoate |
| EH05-13-02R | EH05-13 | Prop-2-enyl 4-[4-[[3-(3-hydroxyphenyl)-5-propan-2-yloxyphenyl]methyl]piperazin-1-yl]benzoate |
| EH05-14-02R | EH05-14 | Prop-2-enyl 4-[4-[[3-(5-hydroxypyridin-3-yl)-5-propan-2-yloxyphenyl]methyl]piperazin-1-yl]benzoate |
| EH05-15-02R | EH05-15 | Prop-2-enyl 4-[4-[[4-(3-hydroxyphenyl)-2,6-dimethoxyphenyl]methyl]piperazin-1-yl]benzoate |
| EH05-16-02R | EH05-16 | Prop-2-enyl 4-[4-[[4-(5-hydroxypyridin-3-yl)-2,6-dimethoxyphenyl]methyl]piperazin-1-yl]benzoate |
| EH05-17-02R | EH05-17 | Prop-2-enyl 4-[4-[[3-(3-hydroxyphenyl)-5-(trifluoromethyl)phenyl]methyl]piperazin-1-yl]benzoate |
| EH05-18-02R | EH05-18 | Prop-2-enyl 4-[4-[[3-(5-hydroxypyridin-3-yl)-5-(trifluoromethyl)phenyl]methyl]piperazin-1-yl]benzoate |
| EH05-19-02R | EH05-19 | Prop-2-enyl 4-[4-[[3-(3-hydroxyphenyl)-5-(trifluoromethoxy)phenyl]methyl]piperazin-1-yl]benzoate |
| EH05-20-02R | EH05-20 | Prop-2-enyl 4-[4-[[3-(5-hydroxypyridin-3-yl)-5-(trifluoromethoxy)phenyl]methyl]piperazin-1-yl]benzoate |

| | | |
|---|---|---|
| EH05-21-02R | EH05-21 | Prop-2-enyl 4-[4-[[4-(3-hydroxyphenyl)naphthalen-1-yl]methyl]piperazin-1-yl]benzoate |
| EH05-22-02R | EH05-22 | Prop-2-enyl 4-[4-[[4-(5-hydroxypyridin-3-yl)naphthalen-1-yl]methyl]piperazin-1-yl]benzoate |
| EH05-23-02R | EH05-23 | Prop-2-enyl 4-[4-[[4-(3-hydroxyphenyl)thiophen-2-yl]methyl]piperazin-1-yl]benzoate |
| EH05-24-02R | EH05-24 | Prop-2-enyl 4-[4-[[4-(5-hydroxypyridin-3-yl)thiophen-2-yl]methyl]piperazin-1-yl]benzoate |
| EH05-25-02R | EH05-25 | Prop-2-enyl 4-[4-[[2-[2-(3-hydroxyphenyl)ethynyl]phenyl]methyl]piperazin-1-yl]benzoate |
| EH05-26-02R | EH05-26 | Prop-2-enyl 4-[4-[[2-[2-(5-hydroxypyridin-3-yl)ethynyl]phenyl]methyl]piperazin-1-yl]benzoate |

Example 4-2-6: Synthesis of mixture mixEH06-02R

[Formula 535]

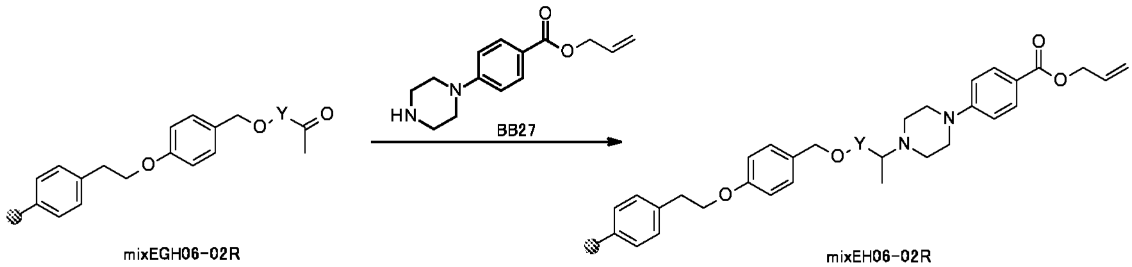


mixEGH06-02R

mixEH06-02R

Under a nitrogen atmosphere, mixture mixEGH06-02R (60 mg, loading rate: 0.645 mmol/g, 0.039 mmol) shown in Table EGH06 and anisole (2.25 mL) were added to a 5 mL glass vial and shaken at room temperature for 1 hour. Under a nitrogen atmosphere, allyl 4-(piperazin-1-yl)benzoate (BB27, 114 mg, 0.464 mmol) and $Ti(OtBu)_4$ (299 μL, 0.774 mmol) were added thereto, and the mixture was shaken at 140°C for 20 hours. The reaction solution was cooled to room temperature. Then, under a nitrogen atmosphere, sodium triacetoxyborohydride (246 mg, 1.16 mmol) was added thereto, and the mixture was shaken at 140°C for 20 hours. The reaction container was cooled to room temperature, and MeOH (1.2 mL) was added to the reaction solution. The obtained reaction solution and the resin suspension were transferred to a syringe with a filter using MeOH, repetitively washed three times with NMP (1.2 mL), three times with a Rochelle salt solution (0.05 M, NMP:water =1:1 solution, 1.2 mL), three times with water (1.2 mL), three times with a half saturated $NaHCO_3$ solution (1.2 mL), three times with water (1.2 mL), 69 times with NMP (1.2 mL), three times with MeOH (1.2 mL), three times with DCM (1.2 mL), and three times with heptane (1.2 mL), and then dried under reduced pressure to obtain the title mixture shown in Table EH06.

Compounds contained in the obtained mixture are shown in Table EH06.

[Table 194]

[Table EGH06] Mixture No: mixEGH06-02R; Mixture No. after cleavage: mixEGH06

| No. of mixture used | Amount of mixture used (mg) | Resin compound No. | Compound No. after cleavage | Compound name |
|---|---|---|---|---|
| mixEG13-02R | 30 | EG07-13-02R | EG07-13 | 4-[4-(4-Acetyl-3-fluorophenyl)phenyl]-1-hydroxy-N,N-dimethylnaphthalene-2-carboxamide |
| | | EG07-14-02R | EG07-14 | 4-[5-(4-Acetyl-3-fluorophenyl)pyridin-2-yl]-1-hydroxy-N,N-dimethylnaphthalene-2-carboxamide |
| mixEG18-02R | 30 | EG01-13-02R | EG01-13 | 4-[4-(4-Acetylphenyl)phenyl]-1-hydroxy-N,N-dimethylnaphthalene-2-carboxamide |
| | | EG01-14-02R | EG01-14 | 4-[5-(4-Acetylphenyl)pyridin-2-yl]-1-hydroxy-N,N-dimethylnaphthalene-2-carboxamide |

[Table 195]

[Table EH06] Mixture No: mixEH06-02R; Mixture No. after cleavage: mixEH06

| Resin compound No. | Compound No. after cleavage | Compound name |
|---|---|---|
| EH06-01-02R | EH06-01 | Prop-2-enyl 4-[4-[1-[2-fluoro-4-(3-hydroxyphenyl)phenyl]ethyl]piperazin-1-yl]benzoate |
| EH06-02-02R | EH06-02 | Prop-2-enyl 4-[4-[1-[2-fluoro-4-(5-hydroxypyridin-3-yl)phenyl]ethyl]piperazin-1-yl]benzoate |
| EH06-03-02R | EH06-03 | Prop-2-enyl 4-[4-[1-[4-(3-hydroxyphenyl)phenyl]ethyl]piperazin-1-yl]benzoate |
| EH06-04-02R | EH06-04 | Prop-2-enyl 4-[4-[1-[4-(5-hydroxypyridin-3-yl)phenyl]ethyl]piperazin-1-yl]benzoate |

Example 4-3-1: Synthesis of mixture mixEI01-02R

[Formula 536]

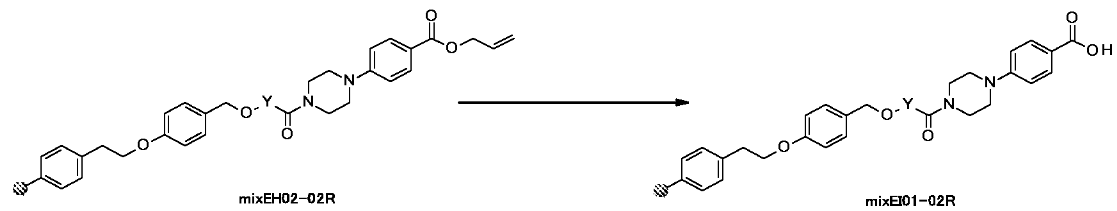

mixEH02-02R mixEI01-02R

Under a nitrogen atmosphere, mixture mixEH02-02R (174 mg, loading rate: 0.651 mmol/g, 0.113 mmol) shown in Table EH02 and THF (5.2 mL) were added to an 8 mL screw-cap vial and shaken at room temperature for 1 hour. Under a nitrogen atmosphere, tetrakistriphenylphosphine palladium(0) (39.3 mg, 34 μmol) and morpholine (49.4 μL, 0.566 mmol) were added thereto, and the mixture was shaken at room temperature for 2 hours and 45 minutes. The resin suspension was transferred to a column with a filter using NMP, repetitively washed three times with NMP (4 mL), three times with MeOH (4 mL), three times with a NAC solution (0.3 M, NMP:water = 5:1 solution, 4 mL), three times with a solution of malonic acid in NMP (0.05 M, 4 mL), three times with a solution of HOAt in NMP (0.2 M, 4 mL), three times with NMP (4 mL), three times with MeOH (4 mL), three times with DCM (4 mL), and three times with heptane (4 mL), and then dried under reduced pressure to obtain the title mixture. Compounds contained in the obtained mixture are shown in Table EI01.

[Table 196]

[Table EI01] Mixture No: mixEI01-02R; Mixture No. after cleavage: mixEI01

| Resin compound No. | Compound No. after cleavage | Compound name |
|---|---|---|
| EI01-01-02R | EI01-01 | 4-[4-[4-(3-Hydroxyphenyl)thiophene-2-carbonyl]piperazin-1-yl]benzoic acid |
| EI01-02-02R | EI01-02 | 4-[4-[4-(5-Hydroxypyridin-3-yl)thiophene-2-carbonyl]piperazin-1-yl]benzoic acid |
| EI01-03-02R | EI01-03 | 4-[4-[4-(3-Hydroxyphenyl)-3-methylbenzoyl]piperazin-1-yl]benzoic acid |
| EI01-04-02R | EI01-04 | 4-[4-[4-(5-Hydroxypyridin-3-yl)-3-methylbenzoyl]piperazin-1-yl]benzoic acid |
| EI01-05-02R | EI01-05 | 4-[4-[3-Fluoro-4-(3-hydroxyphenyl)benzoyl]piperazin-1-yl]benzoic acid |
| EI01-06-02R | EI01-06 | 4-[4-[3-Fluoro-4-(5-hydroxypyridin-3-yl)benzoyl]piperazin-1-yl]benzoic acid |
| EI01-07-02R | EI01-07 | 4-[4-[2-[2-(3-Hydroxyphenyl)ethynyl]benzoyl]piperazin-1-yl]benzoic acid |
| EI01-08-02R | EI01-08 | 4-[4-[2-[2-(5-Hydroxypyridin-3-yl)ethynyl]benzoyl]piperazin-1-yl]benzoic acid |
| EI01-09-02R | EI01-09 | 4-[4-[5-[2-(3-Hydroxyphenyl)ethynyl]pyridine-3-carbonyl]piperazin-1-yl]benzoic acid |
| EI01-10-02R | EI01-10 | 4-[4-[5-[2-(5-Hydroxypyridin-3-yl)ethynyl]pyridine-3-carbonyl]piperazin-1-yl]benzoic acid |
| EI01-11-02R | EI01-11 | 4-[4-[3-[2-(3-Hydroxyphenyl)phenyl]propanoyl]piperazin-1-yl]benzoic acid |
| EI01-12-02R | EI01-12 | 4-[4-[3-[2-(5-Hydroxypyridin-3-yl)phenyl]propanoyl]piperazin-1-yl]benzoic acid |
| EI01-13-02R | EI01-13 | 4-[4-[4-(3-Hydroxyphenyl)naphthalene-1-carbonyl]piperazin-1-yl]benzoic acid |
| EI01-14-02R | EI01-14 | 4-[4-[4-(5-Hydroxypyridin-3-yl)naphthalene-1-carbonyl]piperazin-1-yl]benzoic acid |
| EI01-15-02R | EI01-15 | 4-[4-[3-tert-Butyl-5-(3-hydroxyphenyl)benzoyl]piperazin-1-yl]benzoic acid |
| EI01-16-02R | EI01-16 | 4-[4-[3-tert-Butyl-5-(5-hydroxypyridin-3-yl)benzoyl]piperazin-1-yl]benzoic acid |
| EI01-17-02R | EI01-17 | 4-[4-[3-(3-Hydroxyphenyl)-5-(trifluoromethyl)benzoyl]piperazin-1-yl]benzoic acid |
| EI01-18-02R | EI01-18 | 4-[4-[3-(5-Hydroxypyridin-3-yl)-5-(trifluoromethyl)benzoyl]piperazin-1-yl]benzoic acid |
| EI01-19-02R | EI01-19 | 4-[4-[3-[2-(3-Hydroxyphenyl)ethynyl]-5-(trifluoromethyl)benzoyl]piperazin-1-yl]benzoic acid |
| EI01-20-02R | EI01-20 | 4-[4-[3-[2-(5-Hydroxypyridin-3-yl)ethynyl]-5-(trifluoromethyl)benzoyl]piperazin-1-yl]benzoic acid |
| EI01-21-02R | EI01-21 | 4-[4-[3-[4-(3-Hydroxyphenyl)pyrazol-1-yl]-5-(trifluoromethyl)benzoyl]piperazin-1-yl]benzoic acid |
| EI01-22-02R | EI01-22 | 4-[4-[3-[4-(5-Hydroxypyridin-3-yl)pyrazol-1-yl]-5-(trifluoromethyl)benzoyl]piperazin-1-yl]benzoic acid |

Example 4-3-2: Synthesis of mixture mixEI02-02R

[Formula 537]

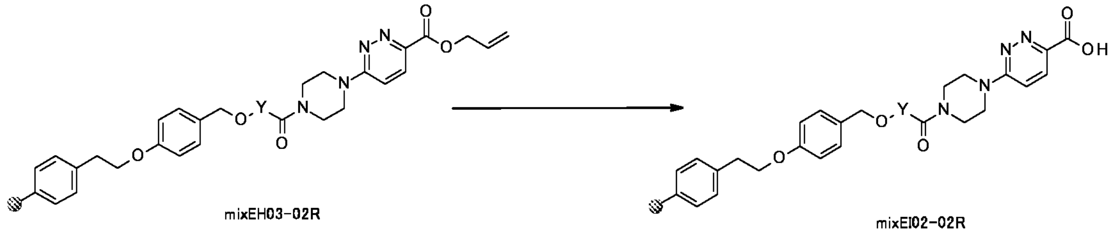

mixEH03-02R mixEI02-02R

Under a nitrogen atmosphere, mixture mixEH03-02R (174 mg, loading rate: 0.651 mmol/g, 0.113 mmol) shown

in Table EH03 and THF (5.2 mL) were added to an 8 mL glass vial and shaken at room temperature for 1 hour. Under a nitrogen atmosphere, $Pd(PPh_3)_4$ (6.6 mg, 5.66 μmol) and morpholine (34.5 μL, 0.397 mmol) were added thereto, and the mixture was shaken at room temperature for 2 hours and 45 minutes. The resin suspension was transferred to a column with a filter using NMP, repetitively washed three times with NMP (4 mL), three times with MeOH (4 mL), three times with a NAC solution (0.3 M, NMP:water = 5:1 solution, 4 mL), three times with a solution of malonic acid in NMP (0.05 M, 4 mL), three times with a solution of HOAt in NMP (0.2 M, 4 mL), three times with NMP (4 mL), three times with MeOH (4 mL), three times with DCM (4 mL), and three times with heptane (4 mL), and then dried under reduced pressure to obtain the title mixture shown in Table EI02. Compounds contained in the obtained mixture are shown in Table EI02.

[Table 197]

[Table EI02] Mixture No: mixEI02-02R; Mixture No. after cleavage: mixEI02

| Resin compound No. | Compound No. after cleavage | Compound name |
|---|---|---|
| EI02-01-02R | EI02-01 | 6-[4-[4-(3-Hydroxyphenyl)thiophene-2-carbonyl]piperazin-1-yl]pyridazine-3-carboxylic acid |
| EI02-02-02R | EI02-02 | 6-[4-[4-(5-Hydroxypyridin-3-yl)thiophene-2-carbonyl]piperazin-1-yl]pyridazine-3-carboxylic acid |
| EI02-03-02R | EI02-03 | 6-[4-[4-(3-Hydroxyphenyl)-3-methylbenzoyl]piperazin-1-yl]pyridazine-3-carboxylic acid |
| EI02-04-02R | EI02-04 | 6-[4-[4-(5-Hydroxypyridin-3-yl)-3-methylbenzoyl]piperazin-1-yl]pyridazine-3-carboxylic acid |
| EI02-05-02R | EI02-05 | 6-[4-[3-Fluoro-4-(3-hydroxyphenyl)benzoyl]piperazin-1-yl]pyridazine-3-carboxylic acid |
| EI02-06-02R | EI02-06 | 6-[4-[3-Fluoro-4-(5-hydroxypyridin-3-yl)benzoyl]piperazin-1-yl]pyridazine-3-carboxylic acid |
| EI02-07-02R | EI02-07 | 6-[4-[2-[2-(3-Hydroxyphenyl)ethynyl]benzoyl]piperazin-1-yl]pyridazine-3-carboxylic acid |

| | | |
|---|---|---|
| EI02-08-02R | EI02-08 | 6-[4-[2-[2-(5-Hydroxypyridin-3-yl)ethynyl]benzoyl]piperazin-1-yl]pyridazine-3-carboxylic acid |
| EI02-09-02R | EI02-09 | 6-[4-[5-[2-(3-Hydroxyphenyl)ethynyl]pyridine-3-carbonyl]piperazin-1-yl]pyridazine-3-carboxylic acid |
| EI02-10-02R | EI02-10 | 6-[4-[5-[2-(5-Hydroxypyridin-3-yl)ethynyl]pyridine-3-carbonyl]piperazin-1-yl]pyridazine-3-carboxylic acid |
| EI02-11-02R | EI02-11 | 6-[4-[3-[2-(3-Hydroxyphenyl)phenyl]propanoyl]piperazin-1-yl]pyridazine-3-carboxylic acid |
| EI02-12-02R | EI02-12 | 6-[4-[3-[2-(5-Hydroxypyridin-3-yl)phenyl]propanoyl]piperazin-1-yl]pyridazine-3-carboxylic acid |
| EI02-13-02R | EI02-13 | 6-[4-[4-(3-Hydroxyphenyl)naphthalene-1-carbonyl]piperazin-1-yl]pyridazine-3-carboxylic acid |
| EI02-14-02R | EI02-14 | 6-[4-[4-(5-Hydroxypyridin-3-yl)naphthalene-1-carbonyl]piperazin-1-yl]pyridazine-3-carboxylic acid |
| EI02-15-02R | EI02-15 | 6-[4-[3-tert-Butyl-5-(3-hydroxyphenyl)benzoyl]piperazin-1-yl]pyridazine-3-carboxylic acid |
| EI02-16-02R | EI02-16 | 6-[4-[3-tert-Butyl-5-(5-hydroxypyridin-3-yl)benzoyl]piperazin-1-yl]pyridazine-3-carboxylic acid |
| EI02-17-02R | EI02-17 | 6-[4-[3-(3-Hydroxyphenyl)-5-(trifluoromethyl)benzoyl]piperazin-1-yl]pyridazine-3-carboxylic acid |
| EI02-18-02R | EI02-18 | 6-[4-[3-(5-Hydroxypyridin-3-yl)-5-(trifluoromethyl)benzoyl]piperazin-1-yl]pyridazine-3-carboxylic acid |
| EI02-19-02R | EI02-19 | 6-[4-[3-[2-(3-Hydroxyphenyl)ethynyl]-5-(trifluoromethyl)benzoyl]piperazin-1-yl]pyridazine-3-carboxylic acid |
| EI02-20-02R | EI02-20 | 6-[4-[3-[2-(5-Hydroxypyridin-3-yl)ethynyl]-5-(trifluoromethyl)benzoyl]piperazin-1-yl]pyridazine-3-carboxylic acid |
| EI02-21-02R | EI02-21 | 6-[4-[3-[4-(3-Hydroxyphenyl)pyrazol-1-yl]-5-(trifluoromethyl)benzoyl]piperazin-1-yl]pyridazine-3-carboxylic acid |
| EI02-22-02R | EI02-22 | 6-[4-[3-[4-(5-Hydroxypyridin-3-yl)pyrazol-1-yl]-5-(trifluoromethyl)benzoyl]piperazin-1-yl]pyridazine-3-carboxylic acid |

Example 4-3-3: Synthesis of mixture mixEI03-02R

[Formula 538]

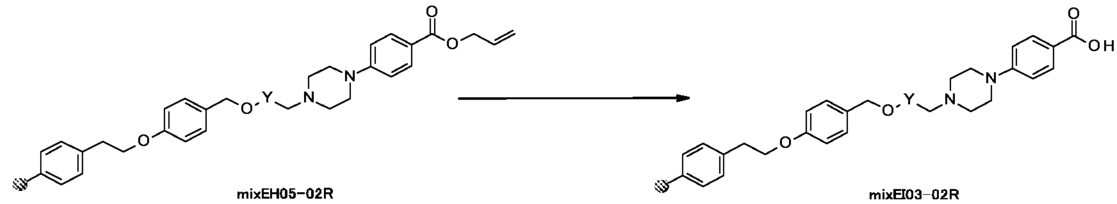

Under a nitrogen atmosphere, mixture mixEH05-02R (260 mg, loading rate: 0.651 mmol/g, 0.169 mmol) shown in Table EH05 and THF (7.8 mL) were added to a 10 mL screw-cap vial and shaken at room temperature for 1 hour. Under a nitrogen atmosphere, tetrakistriphenylphosphine palladium(0) (58.7 mg, 51 µmol) and morpholine (73.7 µL, 0.846 mmol) were added thereto, and the mixture was shaken at room temperature for 2 hours and 45 minutes. The resin suspension was transferred to a column with a filter using NMP, repetitively washed three times with NMP (6 mL), three times with MeOH (6 mL), three times with a NAC solution (0.3 M, NMP:water = 5:1 solution, 6 mL), three times with a solution of malonic acid in NMP (0.05 M, 6 mL), three times with a solution of HOAt in NMP (0.2 M, 6 mL), three times with NMP (6 mL), three times with MeOH (6 mL), three times with DCM (6 mL), and three times with heptane (6 mL), and then dried under reduced pressure to obtain the title mixture. Compounds contained in the obtained mixture are shown in Table EI03.

[Table 198]

[Table EI03] Mixture No: mixEI03-02R; Mixture No. after cleavage: mixEI02

| Resin compound No. | Compound No. after cleavage | Compound name |
|---|---|---|
| EI03-01-02R | EI03-01 | 4-[4-[[2-(3-Hydroxyphenyl)phenyl]methyl]piperazin-1-yl]benzoic acid |
| EI03-02-02R | EI03-02 | 4-[4-[[2-(5-Hydroxypyridin-3-yl)phenyl]methyl]piperazin-1-yl]benzoic acid |
| EI03-03-02R | EI03-03 | 4-[4-[[4-(3-Hydroxyphenyl)-3-methylphenyl]methyl]piperazin-1-yl]benzoic acid |
| EI03-04-02R | EI03-04 | 4-[4-[[4-(5-Hydroxypyridin-3-yl)-3-methylphenyl]methyl]piperazin-1-yl]benzoic acid |
| EI03-05-02R | EI03-05 | 4-[4-[[2-Ethyl-4-(3-hydroxyphenyl)phenyl]methyl]piperazin-1-yl]benzoic acid |
| EI03-06-02R | EI03-06 | 4-[4-[[2-Ethyl-4-(5-hydroxypyridin-3-yl)phenyl]methyl]piperazin-1-yl]benzoic acid |
| EI03-07-02R | EI03-07 | 4-[4-[[3-Fluoro-4-(3-hydroxyphenyl)phenyl]methyl]piperazin-1-yl]benzoic acid |
| EI03-08-02R | EI03-08 | 4-[4-[[3-Fluoro-4-(5-hydroxypyridin-3-yl)phenyl]methyl]piperazin-1-yl]benzoic acid |
| EI03-09-02R | EI03-09 | 4-[4-[[4-(3-Hydroxyphenyl)-2-methoxyphenyl]methyl]piperazin-1-yl]benzoic acid |
| EI03-10-02R | EI03-10 | 4-[4-[[4-(5-Hydroxypyridin-3-yl)-2-methoxyphenyl]methyl]piperazin-1-yl]benzoic acid |
| EI03-11-02R | EI03-11 | 4-[4-[[3-Ethoxy-5-(3-hydroxyphenyl)phenyl]methyl]piperazin-1-yl]benzoic acid |
| EI03-12-02R | EI03-12 | 4-[4-[[3-Ethoxy-5-(5-hydroxypyridin-3-yl)phenyl]methyl]piperazin-1-yl]benzoic acid |
| EI03-13-02R | EI03-13 | 4-[4-[[3-(3-Hydroxyphenyl)-5-propan-2-yloxyphenyl]methyl]piperazin-1-yl]benzoic acid |
| EI03-14-02R | EI03-14 | 4-[4-[[3-(5-Hydroxypyridin-3-yl)-5-propan-2-yloxyphenyl]methyl]piperazin-1-yl]benzoic acid |
| EI03-15-02R | EI03-15 | 4-[4-[[4-(3-Hydroxyphenyl)-2,6-dimethoxyphenyl]methyl]piperazin-1-yl]benzoic acid |
| EI03-16-02R | EI03-16 | 4-[4-[[4-(5-Hydroxypyridin-3-yl)-2,6-dimethoxyphenyl]methyl]piperazin-1-yl]benzoic acid |
| EI03-17-02R | EI03-17 | 4-[4-[[3-(3-Hydroxyphenyl)-5-(trifluoromethyl)phenyl]methyl]piperazin-1-yl]benzoic acid |
| EI03-18-02R | EI03-18 | 4-[4-[[3-(5-Hydroxypyridin-3-yl)-5-(trifluoromethyl)phenyl]methyl]piperazin-1-yl]benzoic acid |
| EI03-19-02R | EI03-19 | 4-[4-[[3-(3-Hydroxyphenyl)-5-(trifluoromethoxy)phenyl]methyl]piperazin-1-yl]benzoic acid |
| EI03-20-02R | EI03-20 | 4-[4-[[3-(5-Hydroxypyridin-3-yl)-5-(trifluoromethoxy)phenyl]methyl]piperazin-1-yl]benzoic acid |
| EI03-21-02R | EI03-21 | 4-[4-[[4-(3-Hydroxyphenyl)naphthalen-1-yl]methyl]piperazin-1-yl]benzoic acid |
| EI03-22-02R | EI03-22 | 4-[4-[[4-(5-Hydroxypyridin-3-yl)naphthalen-1-yl]methyl]piperazin-1-yl]benzoic acid |
| EI03-23-02R | EI03-23 | 4-[4-[[4-(3-Hydroxyphenyl)thiophen-2-yl]methyl]piperazin-1-yl]benzoic acid |
| EI03-24-02R | EI03-24 | 4-[4-[[4-(5-Hydroxypyridin-3-yl)thiophen-2-yl]methyl]piperazin-1-yl]benzoic acid |
| EI03-25-02R | EI03-25 | 4-[4-[[2-[2-(3-Hydroxyphenyl)ethynyl]phenyl]methyl]piperazin-1-yl]benzoic acid |
| EI03-26-02R | EI03-26 | 4-[4-[[2-[2-(5-Hydroxypyridin-3-yl)ethynyl]phenyl]methyl]piperazin-1-yl]benzoic acid |

Example 4-3-4: Synthesis of mixture mixEI04-02R

[Formula 539]

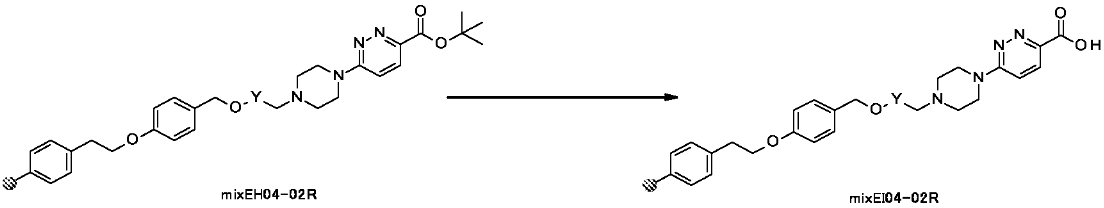

Under a nitrogen atmosphere, mixture mixEH04-02R (300 mg, loading rate: 0.651 mmol/g) shown in Table EH04, THF (8.07 mL, 27 v/w), and MeOH (807 μL, 2.7 v/w) were added to an 8.0 mL screw-cap vial and shaken at room temperature for 1 hour. Under a nitrogen atmosphere, an aqueous LiOH solution (2 M, 781 μL) was added thereto, and the mixture was shaken at room temperature for 8 hours. The resin suspension was transferred to a column with a filter using MeOH, repetitively washed three times with NMP (6.0 mL), three times with $H_2O$ (6.0 mL), three times with NMP (6.0 mL), three times with a solution of HOAt in NMP (0.2 M, 6.0 mL), three times with NMP (6.0 mL), three times with MeOH (6.0 mL), three times with DCM (6.0 mL), and three times with heptane (6.0 mL), and then dried under reduced pressure to obtain the title mixture mixEI04-02R. Compounds contained in the obtained mixture are shown in Table EI04.

[Table 199]

[Table EI04] Mixture No: mixEI04-02R; Mixture No. after cleavage: mixEI04

| Resin compound No. | Compound No. after cleavage | Compound name |
|---|---|---|
| EI04-01-02R | EI04-01 | 6-[4-[[2-(3-Hydroxyphenyl)phenyl]methyl]piperazin-1-yl]pyridazine-3-carboxylic acid |
| EI04-02-02R | EI04-02 | 6-[4-[[2-(5-Hydroxypyridin-3-yl)phenyl]methyl]piperazin-1-yl]pyridazine-3-carboxylic acid |
| EI04-03-02R | EI04-03 | 6-[4-[[4-(3-Hydroxyphenyl)-3-methylphenyl]methyl]piperazin-1-yl]pyridazine-3-carboxylic acid |

| | | |
|---|---|---|
| EI04-04-02R | EI04-04 | 6-[4-[[4-(5-Hydroxypyridin-3-yl)-3-methylphenyl]methyl]piperazin-1-yl]pyridazine-3-carboxylic acid |
| EI04-05-02R | EI04-05 | 6-[4-[[2-Ethyl-4-(3-hydroxyphenyl)phenyl]methyl]piperazin-1-yl]pyridazine-3-carboxylic acid |
| EI04-06-02R | EI04-06 | 6-[4-[[2-Ethyl-4-(5-hydroxypyridin-3-yl)phenyl]methyl]piperazin-1-yl]pyridazine-3-carboxylic acid |
| EI04-07-02R | EI04-07 | 6-[4-[[3-Fluoro-4-(3-hydroxyphenyl)phenyl]methyl]piperazin-1-yl]pyridazine-3-carboxylic acid |
| EI04-08-02R | EI04-08 | 6-[4-[[3-Fluoro-4-(5-hydroxypyridin-3-yl)phenyl]methyl]piperazin-1-yl]pyridazine-3-carboxylic acid |
| EI04-09-02R | EI04-09 | 6-[4-[[4-(3-Hydroxyphenyl)-2-methoxyphenyl]methyl]piperazin-1-yl]pyridazine-3-carboxylic acid |
| EI04-10-02R | EI04-10 | 6-[4-[[4-(5-Hydroxypyridin-3-yl)-2-methoxyphenyl]methyl]piperazin-1-yl]pyridazine-3-carboxylic acid |
| EI04-11-02R | EI04-11 | 6-[4-[[3-Ethoxy-5-(3-hydroxyphenyl)phenyl]methyl]piperazin-1-yl]pyridazine-3-carboxylic acid |
| EI04-12-02R | EI04-12 | 6-[4-[[3-Ethoxy-5-(5-hydroxypyridin-3-yl)phenyl]methyl]piperazin-1-yl]pyridazine-3-carboxylic acid |
| EI04-13-02R | EI04-13 | 6-[4-[[3-(3-Hydroxyphenyl)-5-propan-2-yloxyphenyl]methyl]piperazin-1-yl]pyridazine-3-carboxylic acid |
| EI04-14-02R | EI04-14 | tert-Butyl 6-[4-[[3-(5-hydroxypyridin-3-yl)-5-propan-2-yloxyphenyl]methyl]piperazin-1-yl]pyridazine-3-carboxylate |
| EI04-15-02R | EI04-15 | 6-[4-[[4-(3-Hydroxyphenyl)-2,6-dimethoxyphenyl]methyl]piperazin-1-yl]pyridazine-3-carboxylic acid |
| EI04-16-02R | EI04-16 | 6-[4-[[4-(5-Hydroxypyridin-3-yl)-2,6-dimethoxyphenyl]methyl]piperazin-1-yl]pyridazine-3-carboxylic acid |
| EI04-17-02R | EI04-17 | 6-[4-[[3-(3-Hydroxyphenyl)-5-(trifluoromethyl)phenyl]methyl]piperazin-1-yl]pyridazine-3-carboxylic acid |
| EI04-18-02R | EI04-18 | 6-[4-[[3-(5-Hydroxypyridin-3-yl)-5-(trifluoromethyl)phenyl]methyl]piperazin-1-yl]pyridazine-3-carboxylic acid |
| EI04-19-02R | EI04-19 | 6-[4-[[3-(3-Hydroxyphenyl)-5-(trifluoromethoxy)phenyl]methyl]piperazin-1-yl]pyridazine-3-carboxylic acid |
| EI04-20-02R | EI04-20 | 6-[4-[[3-(5-Hydroxypyridin-3-yl)-5-(trifluoromethoxy)phenyl]methyl]piperazin-1-yl]pyridazine-3-carboxylic acid |
| EI04-21-02R | EI04-21 | 6-[4-[[4-(3-Hydroxyphenyl)naphthalen-1-yl]methyl]piperazin-1-yl]pyridazine-3-carboxylic acid |
| EI04-22-02R | EI04-22 | 6-[4-[[4-(5-Hydroxypyridin-3-yl)naphthalen-1-yl]methyl]piperazin-1-yl]pyridazine-3-carboxylic acid |
| EI04-23-02R | EI04-23 | 6-[4-[[4-(3-Hydroxyphenyl)thiophen-2-yl]methyl]piperazin-1-yl]pyridazine-3-carboxylic acid |
| EI04-24-02R | EI04-24 | 6-[4-[[4-(5-Hydroxypyridin-3-yl)thiophen-2-yl]methyl]piperazin-1-yl]pyridazine-3-carboxylic acid |
| EI04-25-02R | EI04-25 | 6-[4-[[2-[2-(3-Hydroxyphenyl)ethynyl]phenyl]methyl]piperazin-1-yl]pyridazine-3-carboxylic acid |
| EI04-26-02R | EI04-26 | 6-[4-[[2-[2-(5-Hydroxypyridin-3-yl)ethynyl]phenyl]methyl]piperazin-1-yl]pyridazine-3-carboxylic acid |

Example 4-3-5: Synthesis of mixture mixEI05-02R

[Formula 540]

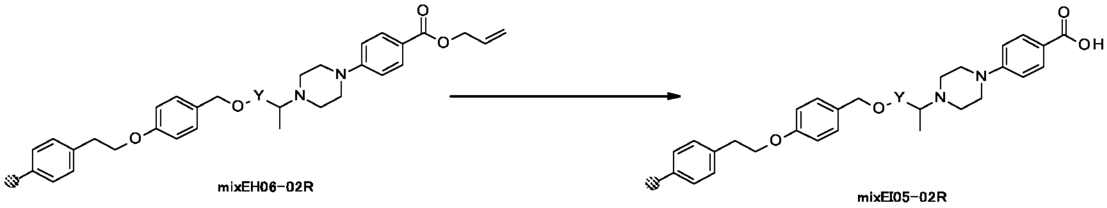

Under a nitrogen atmosphere, mixture mixEH06-02R (50 mg, loading rate: 0.645 mmol/g, 32 μmol) shown in Table EH06 and THF (1.5 mL) were added to a 2 mL screw-cap vial and shaken at room temperature for 1 hour. Under a nitrogen atmosphere, tetrakistriphenylphosphine palladium(0) (11.2 mg, 9.68 μmol) and morpholine (14.1 μL, 0.161 mmol) were added thereto, and the mixture was shaken at room temperature for 3 hours. The resin suspension was transferred to a column with a filter using NMP, repetitively washed three times with NMP (1 mL), three times with MeOH (1 mL), three times with a NAC solution (0.3 M, NMP:water = 5:1 solution, 1 mL), three times with a solution of malonic acid in NMP (0.05 M, 1 mL), three times with a solution of HOAt in NMP (0.2 M, 1 mL), three times with NMP (1 mL), three times with MeOH (1 mL), three times with DCM (1 mL), and three times with heptane (1 mL), and then dried under reduced pressure to obtain the title mixture. Compounds contained in the obtained mixture are shown in Table EI05.

[Table 200]

[Table EI05] Mixture No: mixEI05-02R; Mixture No. after cleavage: mixEI05

| Resin compound No. | Compound No. after cleavage | Compound name |
|---|---|---|
| EI05-01-02R | EI05-01 | 4-[4-[1-[2-Fluoro-4-(3-hydroxyphenyl)phenyl]ethyl]piperazin-1-yl]benzoic acid |
| EI05-02-02R | EI05-02 | 4-[4-[1-[2-Fluoro-4-(5-hydroxypyridin-3-yl)phenyl]ethyl]piperazin-1-yl]benzoic acid |
| EI05-03-02R | EI05-03 | 4-[4-[1-[4-(3-Hydroxyphenyl)phenyl]ethyl]piperazin-1-yl]benzoic acid |
| EI05-04-02R | EI05-04 | 4-[4-[1-[4-(5-Hydroxypyridin-3-yl)phenyl]ethyl]piperazin-1-yl]benzoic acid |

Example 4-3-6: Synthesis of mixture mixEI06-02R

[Formula 541]

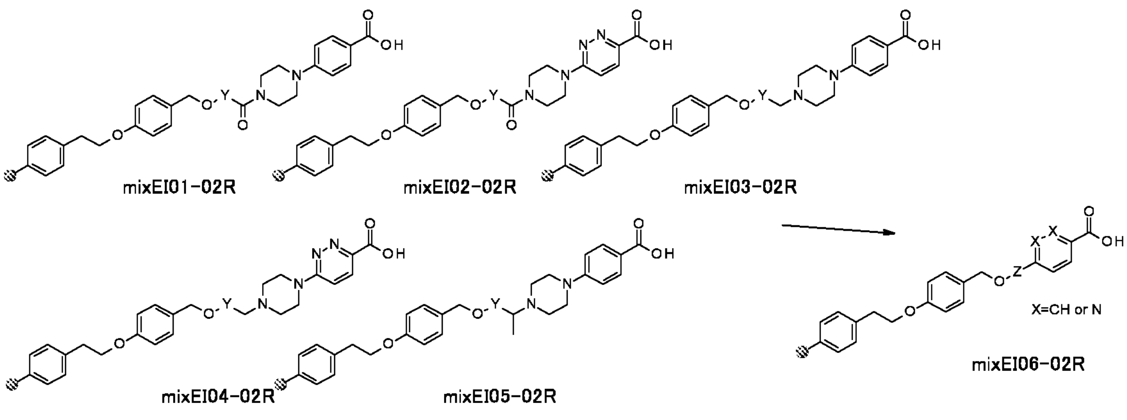

The title mixture mixEI06-02R (703 mg, loading rate: 0.648 mmol/g, 0.456 mmol) shown in Table EI06 was obtained by preparation in the same manner as in Example 3-6-5 using mixtures mixEI01-02R (155.9 mg, loading rate: 0.651 mmol/g, 0.101 mmol), mixEI02- 02R (153.5 mg, loading rate: 0.651 mmol/g, 99.9 μmol), mixEI03-02R (182.5 mg, loading rate: 0.651 mmol/g, 0.119 mmol), mixEI04-02R (182.9 mg, loading rate: 0.651 mmol/g, 0.119 mmol), and mixEI05-02R (28.6 mg, loading rate: 0.645 mmol/g, 18.4 μmol) shown in Table EI01, Table EI02, Table EI03, Table

EH04, and Table EI05.

[Table 201]

[Table EI06] Mixture No: mixEI06-02R; Mixture No. after cleavage: mixEI06

| Resin compound No. | Compound No. after cleavage | Compound name |
|---|---|---|
| EI01-01-02R | EI01-01 | 4-[4-[4-(3-Hydroxyphenyl)thiophene-2-carbonyl]piperazin-1-yl]benzoic acid |
| EI01-02-02R | EI01-02 | 4-[4-[4-(5-Hydroxypyridin-3-yl)thiophene-2-carbonyl]piperazin-1-yl]benzoic acid |
| EI01-03-02R | EI01-03 | 4-[4-[4-(3-Hydroxyphenyl)-3-methylbenzoyl]piperazin-1-yl]benzoic acid |
| EI01-04-02R | EI01-04 | 4-[4-[4-(5-Hydroxypyridin-3-yl)-3-methylbenzoyl]piperazin-1-yl]benzoic acid |

| | | |
|---|---|---|
| EI01-05-02R | EI01-05 | 4-[4-[3-Fluoro-4-(3-hydroxyphenyl)benzoyl]piperazin-1-yl]benzoic acid |
| EI01-06-02R | EI01-06 | 4-[4-[3-Fluoro-4-(5-hydroxypyridin-3-yl)benzoyl]piperazin-1-yl]benzoic acid |
| EI01-07-02R | EI01-07 | 4-[4-[2-[2-(3-Hydroxyphenyl)ethynyl]benzoyl]piperazin-1-yl]benzoic acid |
| EI01-08-02R | EI01-08 | 4-[4-[2-[2-(5-Hydroxypyridin-3-yl)ethynyl]benzoyl]piperazin-1-yl]benzoic acid |
| EI01-09-02R | EI01-09 | 4-[4-[5-[2-(3-Hydroxyphenyl)ethynyl]pyridine-3-carbonyl]piperazin-1-yl]benzoic acid |
| EI01-10-02R | EI01-10 | 4-[4-[5-[2-(5-Hydroxypyridin-3-yl)ethynyl]pyridine-3-carbonyl]piperazin-1-yl]benzoic acid |
| EI01-11-02R | EI01-11 | 4-[4-[3-[2-(3-Hydroxyphenyl)phenyl]propanoyl]piperazin-1-yl]benzoic acid |
| EI01-12-02R | EI01-12 | 4-[4-[3-[2-(5-Hydroxypyridin-3-yl)phenyl]propanoyl]piperazin-1-yl]benzoic acid |
| EI01-13-02R | EI01-13 | 4-[4-[4-(3-Hydroxyphenyl)naphthalene-1-carbonyl]piperazin-1-yl]benzoic acid |
| EI01-14-02R | EI01-14 | 4-[4-[4-(5-Hydroxypyridin-3-yl)naphthalene-1-carbonyl]piperazin-1-yl]benzoic acid |
| EI01-15-02R | EI01-15 | 4-[4-[3-tert-Butyl-5-(3-hydroxyphenyl)benzoyl]piperazin-1-yl]benzoic acid |
| EI01-16-02R | EI01-16 | 4-[4-[3-tert-Butyl-5-(5-hydroxypyridin-3-yl)benzoyl]piperazin-1-yl]benzoic acid |
| EI01-17-02R | EI01-17 | 4-[4-[3-(3-Hydroxyphenyl)-5-(trifluoromethyl)benzoyl]piperazin-1-yl]benzoic acid |
| EI01-18-02R | EI01-18 | 4-[4-[3-(5-Hydroxypyridin-3-yl)-5-(trifluoromethyl)benzoyl]piperazin-1-yl]benzoic acid |
| EI01-19-02R | EI01-19 | 4-[4-[3-[2-(3-Hydroxyphenyl)ethynyl]-5-(trifluoromethyl)benzoyl]piperazin-1-yl]benzoic acid |
| EI01-20-02R | EI01-20 | 4-[4-[3-[2-(5-Hydroxypyridin-3-yl)ethynyl]-5-(trifluoromethyl)benzoyl]piperazin-1-yl]benzoic acid |
| EI01-21-02R | EI01-21 | 4-[4-[3-[4-(3-Hydroxyphenyl)pyrazol-1-yl]-5-(trifluoromethyl)benzoyl]piperazin-1-yl]benzoic acid |
| EI01-22-02R | EI01-22 | 4-[4-[3-[4-(5-Hydroxypyridin-3-yl)pyrazol-1-yl]-5-(trifluoromethyl)benzoyl]piperazin-1-yl]benzoic acid |
| EI02-01-02R | EI02-01 | 6-[4-[4-(3-Hydroxyphenyl)thiophene-2-carbonyl]piperazin-1-yl]pyridazine-3-carboxylic acid |
| EI02-02-02R | EI02-02 | 6-[4-[4-(5-Hydroxypyridin-3-yl)thiophene-2-carbonyl]piperazin-1-yl]pyridazine-3-carboxylic acid |
| EI02-03-02R | EI02-03 | 6-[4-[4-(3-Hydroxyphenyl)-3-methylbenzoyl]piperazin-1-yl]pyridazine-3-carboxylic acid |
| EI02-04-02R | EI02-04 | 6-[4-[4-(5-Hydroxypyridin-3-yl)-3-methylbenzoyl]piperazin-1-yl]pyridazine-3-carboxylic acid |
| EI02-05-02R | EI02-05 | 6-[4-[3-Fluoro-4-(3-hydroxyphenyl)benzoyl]piperazin-1-yl]pyridazine-3-carboxylic acid |
| EI02-06-02R | EI02-06 | 6-[4-[3-Fluoro-4-(5-hydroxypyridin-3-yl)benzoyl]piperazin-1-yl]pyridazine-3-carboxylic acid |
| EI02-07-02R | EI02-07 | 6-[4-[2-[2-(3-Hydroxyphenyl)ethynyl]benzoyl]piperazin-1-yl]pyridazine-3-carboxylic acid |
| EI02-08-02R | EI02-08 | 6-[4-[2-[2-(5-Hydroxypyridin-3-yl)ethynyl]benzoyl]piperazin-1-yl]pyridazine-3-carboxylic acid |
| EI02-09-02R | EI02-09 | 6-[4-[5-[2-(3-Hydroxyphenyl)ethynyl]pyridine-3-carbonyl]piperazin-1-yl]pyridazine-3-carboxylic acid |
| EI02-10-02R | EI02-10 | 6-[4-[5-[2-(5-Hydroxypyridin-3-yl)ethynyl]pyridine-3-carbonyl]piperazin-1-yl]pyridazine-3-carboxylic acid |
| EI02-11-02R | EI02-11 | 6-[4-[3-[2-(3-Hydroxyphenyl)phenyl]propanoyl]piperazin-1-yl]pyridazine-3-carboxylic acid |
| EI02-12-02R | EI02-12 | 6-[4-[3-[2-(5-Hydroxypyridin-3-yl)phenyl]propanoyl]piperazin-1-yl]pyridazine-3-carboxylic acid |
| EI02-13-02R | EI02-13 | 6-[4-[4-(3-Hydroxyphenyl)naphthalene-1-carbonyl]piperazin-1-yl]pyridazine-3-carboxylic acid |

| | | |
|---|---|---|
| EI02-14-02R | EI02-14 | 6-[4-[4-(5-Hydroxypyridin-3-yl)naphthalene-1-carbonyl]piperazin-1-yl]pyridazine-3-carboxylic acid |
| EI02-15-02R | EI02-15 | 6-[4-[3-tert-Butyl-5-(3-hydroxyphenyl)benzoyl]piperazin-1-yl]pyridazine-3-carboxylic acid |
| EI02-16-02R | EI02-16 | 6-[4-[3-tert-Butyl-5-(5-hydroxypyridin-3-yl)benzoyl]piperazin-1-yl]pyridazine-3-carboxylic acid |
| EI02-17-02R | EI02-17 | 6-[4-[3-(3-Hydroxyphenyl)-5-(trifluoromethyl)benzoyl]piperazin-1-yl]pyridazine-3-carboxylic acid |
| EI02-18-02R | EI02-18 | 6-[4-[3-(5-Hydroxypyridin-3-yl)-5-(trifluoromethyl)benzoyl]piperazin-1-yl]pyridazine-3-carboxylic acid |
| EI02-19-02R | EI02-19 | 6-[4-[3-[2-(3-Hydroxyphenyl)ethynyl]-5-(trifluoromethyl)benzoyl]piperazin-1-yl]pyridazine-3-carboxylic acid |
| EI02-20-02R | EI02-20 | 6-[4-[3-[2-(5-Hydroxypyridin-3-yl)ethynyl]-5-(trifluoromethyl)benzoyl]piperazin-1-yl]pyridazine-3-carboxylic acid |
| EI02-21-02R | EI02-21 | 6-[4-[3-[4-(3-Hydroxyphenyl)pyrazol-1-yl]-5-(trifluoromethyl)benzoyl]piperazin-1-yl]pyridazine-3-carboxylic acid |
| EI02-22-02R | EI02-22 | 6-[4-[3-[4-(5-Hydroxypyridin-3-yl)pyrazol-1-yl]-5-(trifluoromethyl)benzoyl]piperazin-1-yl]pyridazine-3-carboxylic acid |
| EI03-01-02R | EI03-01 | 4-[4-[[2-(3-Hydroxyphenyl)phenyl]methyl]piperazin-1-yl]benzoic acid |
| EI03-02-02R | EI03-02 | 4-[4-[[2-(5-Hydroxypyridin-3-yl)phenyl]methyl]piperazin-1-yl]benzoic acid |
| EI03-03-02R | EI03-03 | 4-[4-[[4-(3-Hydroxyphenyl)-3-methylphenyl]methyl]piperazin-1-yl]benzoic acid |
| EI03-04-02R | EI03-04 | 4-[4-[[4-(5-Hydroxypyridin-3-yl)-3-methylphenyl]methyl]piperazin-1-yl]benzoic acid |
| EI03-05-02R | EI03-05 | 4-[4-[[2-Ethyl-4-(3-hydroxyphenyl)phenyl]methyl]piperazin-1-yl]benzoic acid |
| EI03-06-02R | EI03-06 | 4-[4-[[2-Ethyl-4-(5-hydroxypyridin-3-yl)phenyl]methyl]piperazin-1-yl]benzoic acid |
| EI03-07-02R | EI03-07 | 4-[4-[[3-Fluoro-4-(3-hydroxyphenyl)phenyl]methyl]piperazin-1-yl]benzoic acid |
| EI03-08-02R | EI03-08 | 4-[4-[[3-Fluoro-4-(5-hydroxypyridin-3-yl)phenyl]methyl]piperazin-1-yl]benzoic acid |
| EI03-09-02R | EI03-09 | 4-[4-[[4-(3-Hydroxyphenyl)-2-methoxyphenyl]methyl]piperazin-1-yl]benzoic acid |
| EI03-10-02R | EI03-10 | 4-[4-[[4-(5-Hydroxypyridin-3-yl)-2-methoxyphenyl]methyl]piperazin-1-yl]benzoic acid |
| EI03-11-02R | EI03-11 | 4-[4-[[3-Ethoxy-5-(3-hydroxyphenyl)phenyl]methyl]piperazin-1-yl]benzoic acid |
| EI03-12-02R | EI03-12 | 4-[4-[[3-Ethoxy-5-(5-hydroxypyridin-3-yl)phenyl]methyl]piperazin-1-yl]benzoic acid |
| EI03-13-02R | EI03-13 | 4-[4-[[3-(3-Hydroxyphenyl)-5-propan-2-yloxyphenyl]methyl]piperazin-1-yl]benzoic acid |
| EI03-14-02R | EI03-14 | 4-[4-[[3-(5-Hydroxypyridin-3-yl)-5-propan-2-yloxyphenyl]methyl]piperazin-1-yl]benzoic acid |
| EI03-15-02R | EI03-15 | 4-[4-[[4-(3-Hydroxyphenyl)-2,6-dimethoxyphenyl]methyl]piperazin-1-yl]benzoic acid |
| EI03-16-02R | EI03-16 | 4-[4-[[4-(5-Hydroxypyridin-3-yl)-2,6-dimethoxyphenyl]methyl]piperazin-1-yl]benzoic acid |
| EI03-17-02R | EI03-17 | 4-[4-[[3-(3-Hydroxyphenyl)-5-(trifluoromethyl)phenyl]methyl]piperazin-1-yl]benzoic acid |
| EI03-18-02R | EI03-18 | 4-[4-[[3-(5-Hydroxypyridin-3-yl)-5-(trifluoromethyl)phenyl]methyl]piperazin-1-yl]benzoic acid |
| EI03-19-02R | EI03-19 | 4-[4-[[3-(3-Hydroxyphenyl)-5-(trifluoromethoxy)phenyl]methyl]piperazin-1-yl]benzoic acid |
| EI03-20-02R | EI03-20 | 4-[4-[[3-(5-Hydroxypyridin-3-yl)-5-(trifluoromethoxy)phenyl]methyl]piperazin-1-yl]benzoic acid |
| EI03-21-02R | EI03-21 | 4-[4-[[4-(3-Hydroxyphenyl)naphthalen-1-yl]methyl]piperazin-1-yl]benzoic acid |

| | | |
|---|---|---|
| EI03-22-02R | EI03-22 | 4-[4-[[4-(5-Hydroxypyridin-3-yl)naphthalen-1-yl]methyl]piperazin-1-yl]benzoic acid |
| EI03-23-02R | EI03-23 | 4-[4-[[4-(3-Hydroxyphenyl)thiophen-2-yl]methyl]piperazin-1-yl]benzoic acid |
| EI03-24-02R | EI03-24 | 4-[4-[[4-(5-Hydroxypyridin-3-yl)thiophen-2-yl]methyl]piperazin-1-yl]benzoic acid |
| EI03-25-02R | EI03-25 | 4-[4-[[2-[2-(3-Hydroxyphenyl)ethynyl]phenyl]methyl]piperazin-1-yl]benzoic acid |
| EI03-26-02R | EI03-26 | 4-[4-[[2-[2-(5-Hydroxypyridin-3-yl)ethynyl]phenyl]methyl]piperazin-1-yl]benzoic acid |
| EI04-01-02R | EI04-01 | 6-[4-[[2-(3-Hydroxyphenyl)phenyl]methyl]piperazin-1-yl]pyridazine-3-carboxylic acid |
| EI04-02-02R | EI04-02 | 6-[4-[[2-(5-Hydroxypyridin-3-yl)phenyl]methyl]piperazin-1-yl]pyridazine-3-carboxylic acid |
| EI04-03-02R | EI04-03 | 6-[4-[[4-(3-Hydroxyphenyl)-3-methylphenyl]methyl]piperazin-1-yl]pyridazine-3-carboxylic acid |
| EI04-04-02R | EI04-04 | 6-[4-[[4-(5-Hydroxypyridin-3-yl)-3-methylphenyl]methyl]piperazin-1-yl]pyridazine-3-carboxylic acid |
| EI04-05-02R | EI04-05 | 6-[4-[[2-Ethyl-4-(3-hydroxyphenyl)phenyl]methyl]piperazin-1-yl]pyridazine-3-carboxylic acid |
| EI04-06-02R | EI04-06 | 6-[4-[[2-Ethyl-4-(5-hydroxypyridin-3-yl)phenyl]methyl]piperazin-1-yl]pyridazine-3-carboxylic acid |
| EI04-07-02R | EI04-07 | 6-[4-[[3-Fluoro-4-(3-hydroxyphenyl)phenyl]methyl]piperazin-1-yl]pyridazine-3-carboxylic acid |
| EI04-08-02R | EI04-08 | 6-[4-[[3-Fluoro-4-(5-hydroxypyridin-3-yl)phenyl]methyl]piperazin-1-yl]pyridazine-3-carboxylic acid |
| EI04-09-02R | EI04-09 | 6-[4-[[4-(3-Hydroxyphenyl)-2-methoxyphenyl]methyl]piperazin-1-yl]pyridazine-3-carboxylic acid |
| EI04-10-02R | EI04-10 | 6-[4-[[4-(5-Hydroxypyridin-3-yl)-2-methoxyphenyl]methyl]piperazin-1-yl]pyridazine-3-carboxylic acid |
| EI04-11-02R | EI04-11 | 6-[4-[[3-Ethoxy-5-(3-hydroxyphenyl)phenyl]methyl]piperazin-1-yl]pyridazine-3-carboxylic acid |
| EI04-12-02R | EI04-12 | 6-[4-[[3-Ethoxy-5-(5-hydroxypyridin-3-yl)phenyl]methyl]piperazin-1-yl]pyridazine-3-carboxylic acid |
| EI04-13-02R | EI04-13 | 6-[4-[[3-(3-Hydroxyphenyl)-5-propan-2-yloxyphenyl]methyl]piperazin-1-yl]pyridazine-3-carboxylic acid |
| EI04-14-02R | EI04-14 | 6-[4-[[3-(5-Hydroxypyridin-3-yl)-5-propan-2-yloxyphenyl]methyl]piperazin-1-yl]pyridazine-3-carboxylic acid |
| EI04-15-02R | EI04-15 | 6-[4-[[4-(3-Hydroxyphenyl)-2,6-dimethoxyphenyl]methyl]piperazin-1-yl]pyridazine-3-carboxylic acid |
| EI04-16-02R | EI04-16 | 6-[4-[[4-(5-Hydroxypyridin-3-yl)-2,6-dimethoxyphenyl]methyl]piperazin-1-yl]pyridazine-3-carboxylic acid |
| EI04-17-02R | EI04-17 | 6-[4-[[3-(3-Hydroxyphenyl)-5-(trifluoromethyl)phenyl]methyl]piperazin-1-yl]pyridazine-3-carboxylic acid |
| EI04-18-02R | EI04-18 | 6-[4-[[3-(5-Hydroxypyridin-3-yl)-5-(trifluoromethyl)phenyl]methyl]piperazin-1-yl]pyridazine-3-carboxylic acid |
| EI04-19-02R | EI04-19 | 6-[4-[[3-(3-Hydroxyphenyl)-5-(trifluoromethoxy)phenyl]methyl]piperazin-1-yl]pyridazine-3-carboxylic acid |
| EI04-20-02R | EI04-20 | 6-[4-[[3-(5-Hydroxypyridin-3-yl)-5-(trifluoromethoxy)phenyl]methyl]piperazin-1-yl]pyridazine-3-carboxylic acid |
| EI04-21-02R | EI04-21 | 6-[4-[[4-(3-Hydroxyphenyl)naphthalen-1-yl]methyl]piperazin-1-yl]pyridazine-3-carboxylic acid |
| EI04-22-02R | EI04-22 | 6-[4-[[4-(5-Hydroxypyridin-3-yl)naphthalen-1-yl]methyl]piperazin-1-yl]pyridazine-3-carboxylic acid |
| EI04-23-02R | EI04-23 | 6-[4-[[4-(3-Hydroxyphenyl)thiophen-2-yl]methyl]piperazin-1-yl]pyridazine-3-carboxylic acid |
| EI04-24-02R | EI04-24 | 6-[4-[[4-(5-Hydroxypyridin-3-yl)thiophen-2-yl]methyl]piperazin-1-yl]pyridazine-3-carboxylic acid |

| | | |
|---|---|---|
| EI04-25-02R | EI04-25 | 6-[4-[[2-[2-(3-Hydroxyphenyl)ethynyl]phenyl]methyl]piperazin-1-yl]pyridazine-3-carboxylic acid |
| EI04-26-02R | EI04-26 | 6-[4-[[2-[2-(5-Hydroxypyridin-3-yl)ethynyl]phenyl]methyl]piperazin-1-yl]pyridazine-3-carboxylic acid |
| EI05-01-02R | EI05-01 | 4-[4-[1-[2-Fluoro-4-(3-hydroxyphenyl)phenyl]ethyl]piperazin-1-yl]benzoic acid |
| EI05-02-02R | EI05-02 | 4-[4-[1-[2-Fluoro-4-(5-hydroxypyridin-3-yl)phenyl]ethyl]piperazin-1-yl]benzoic acid |
| EI05-03-02R | EI05-03 | 4-[4-[1-[4-(3-Hydroxyphenyl)phenyl]ethyl]piperazin-1-yl]benzoic acid |
| EI05-04-02R | EI05-04 | 4-[4-[1-[4-(5-Hydroxypyridin-3-yl)phenyl]ethyl]piperazin-1-yl]benzoic acid |

Example 4-3-7: Synthesis of mixture mixEI07-02R

[Formula 542]

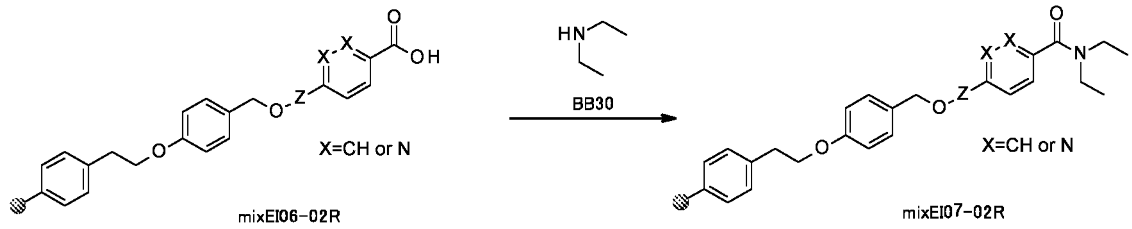


Mixture mixEI06-02R (50 mg, loading rate: 0.648 mmol/g, 0.0324 mmol) shown in Table EI06 and NMP (1.28 mL) were added to a 2 mL glass vial and shaken at room temperature for 1 hour. Diethylamine (BB30, 10.1 μL, 0.097 mmol), DIC (30.3 μL, 0.194 mmol), and HOAt (26.5 mg, 0.194 mmol) were added thereto, and the mixture was shaken at 40°C for 22 hours. The reaction solution and the resin suspension were transferred to a syringe with a filter using NMP and repetitively washed three times with NMP (1.2 mL), three times with MeOH (1.2 mL), three times with DCM (1.2 mL), and three times with heptane (1.2 mL), and the resin was then dried under reduced pressure to obtain the title mixture shown in Table EI07. Compounds contained in the obtained mixture are shown in Table EI07.

[Table 202]

[Table EI07] Mixture No: mixEI07-02R; Amin reagent used: BB30; Mixture No. after cleavage: mixEI07

| Resin compound No. | Compound No. after cleavage | Another name of compound after cleavage in Table EJ01 | Compound name |
|---|---|---|---|
| EI07-01-02R | EI07-01 | 4J01 | N,N-Diethyl-6-[4-[4-(3-hydroxyphenyl)thiophene-2-carbonyl]piperazin-1-yl]pyridazine-3-carboxamide |

| | | | |
|---|---|---|---|
| EI07-02-02R | EI07-02 | 4J02 | N,N-Diethyl-6-[4-[4-(5-hydroxypyridin-3-yl)thiophene-2-carbonyl]piperazin-1-yl]pyridazine-3-carboxamide |
| EI07-03-02R | EI07-03 | 4J03 | N,N-Diethyl-6-[4-[4-(3-hydroxyphenyl)-3-methylbenzoyl]piperazin-1-yl]pyridazine-3-carboxamide |
| EI07-04-02R | EI07-04 | 4J04 | N,N-Diethyl-6-[4-[4-(5-hydroxypyridin-3-yl)-3-methylbenzoyl]piperazin-1-yl]pyridazine-3-carboxamide |
| EI07-05-02R | EI07-05 | 4J05 | N,N-Diethyl-6-[4-[3-fluoro-4-(3-hydroxyphenyl)benzoyl]piperazin-1-yl]pyridazine-3-carboxamide |
| EI07-06-02R | EI07-06 | 4J06 | N,N-Diethyl-6-[4-[3-fluoro-4-(5-hydroxypyridin-3-yl)benzoyl]piperazin-1-yl]pyridazine-3-carboxamide |
| EI07-07-02R | EI07-07 | 4J07 | N,N-Diethyl-6-[4-[2-[2-(3-hydroxyphenyl)ethynyl]benzoyl]piperazin-1-yl]pyridazine-3-carboxamide |
| EI07-08-02R | EI07-08 | 4J08 | N,N-Diethyl-6-[4-[2-[2-(5-hydroxypyridin-3-yl)ethynyl]benzoyl]piperazin-1-yl]pyridazine-3-carboxamide |
| EI07-09-02R | EI07-09 | 4J09 | N,N-Diethyl-6-[4-[5-[2-(3-hydroxyphenyl)ethynyl]pyridine-3-carbonyl]piperazin-1-yl]pyridazine-3-carboxamide |
| EI07-10-02R | EI07-10 | 4J10 | N,N-Diethyl-6-[4-[5-[2-(5-hydroxypyridin-3-yl)ethynyl]pyridine-3-carbonyl]piperazin-1-yl]pyridazine-3-carboxamide |
| EI07-11-02R | EI07-11 | 4J11 | N,N-Diethyl-6-[4-[3-[2-(3-hydroxyphenyl)phenyl]propanoyl]piperazin-1-yl]pyridazine-3-carboxamide |
| EI07-12-02R | EI07-12 | 4J12 | N,N-Diethyl-6-[4-[3-[2-(5-hydroxypyridin-3-yl)phenyl]propanoyl]piperazin-1-yl]pyridazine-3-carboxamide |
| EI07-13-02R | EI07-13 | 4J13 | N,N-Diethyl-6-[4-[4-(3-hydroxyphenyl)naphthalene-1-carbonyl]piperazin-1-yl]pyridazine-3-carboxamide |
| EI07-14-02R | EI07-14 | 4J14 | N,N-Diethyl-6-[4-[4-(5-hydroxypyridin-3-yl)naphthalene-1-carbonyl]piperazin-1-yl]pyridazine-3-carboxamide |
| EI07-15-02R | EI07-15 | 4J15 | 6-[4-[3-tert-Butyl-5-(3-hydroxyphenyl)benzoyl]piperazin-1-yl]-N,N-diethylpyridazine-3-carboxamide |
| EI07-16-02R | EI07-16 | 4J16 | 6-[4-[3-tert-Butyl-5-(5-hydroxypyridin-3-yl)benzoyl]piperazin-1-yl]-N,N-diethylpyridazine-3-carboxamide |
| EI07-17-02R | EI07-17 | 4J17 | N,N-Diethyl-6-[4-[3-(3-hydroxyphenyl)-5-(trifluoromethyl)benzoyl]piperazin-1-yl]pyridazine-3-carboxamide |
| EI07-18-02R | EI07-18 | 4J18 | N,N-Diethyl-6-[4-[3-(5-hydroxypyridin-3-yl)-5-(trifluoromethyl)benzoyl]piperazin-1-yl]pyridazine-3-carboxamide |
| EI07-19-02R | EI07-19 | 4J19 | N,N-Diethyl-6-[4-[3-[2-(3-hydroxyphenyl)ethynyl]-5-(trifluoromethyl)benzoyl]piperazin-1-yl]pyridazine-3-carboxamide |
| EI07-20-02R | EI07-20 | 4J20 | N,N-Diethyl-6-[4-[3-[2-(5-hydroxypyridin-3-yl)ethynyl]-5-(trifluoromethyl)benzoyl]piperazin-1-yl]pyridazine-3-carboxamide |
| EI07-21-02R | EI07-21 | 4J21 | N,N-Diethyl-6-[4-[3-[4-(3-hydroxyphenyl)pyrazol-1-yl]-5-(trifluoromethyl)benzoyl]piperazin-1-yl]pyridazine-3-carboxamide |
| EI07-22-02R | EI07-22 | 4J22 | N,N-Diethyl-6-[4-[3-[4-(5-hydroxypyridin-3-yl)pyrazol-1-yl]-5-(trifluoromethyl)benzoyl]piperazin-1-yl]pyridazine-3-carboxamide |
| EI07-23-02R | EI07-23 | 4J23 | N,N-Diethyl-4-[4-[4-(3-hydroxyphenyl)thiophene-2-carbonyl]piperazin-1-yl]benzamide |
| EI07-24-02R | EI07-24 | 4J24 | N,N-Diethyl-4-[4-[4-(5-hydroxypyridin-3-yl)thiophene-2-carbonyl]piperazin-1-yl]benzamide |
| EI07-25-02R | EI07-25 | 4J25 | N,N-Diethyl-4-[4-[4-(3-hydroxyphenyl)-3-methylbenzoyl]piperazin-1-yl]benzamide |
| EI07-26-02R | EI07-26 | 4J26 | N,N-Diethyl-4-[4-[4-(5-hydroxypyridin-3-yl)-3-methylbenzoyl]piperazin-1-yl]benzamide |
| EI07-27-02R | EI07-27 | 4J27 | N,N-Diethyl-4-[4-[3-fluoro-4-(3-hydroxyphenyl)benzoyl]piperazin-1-yl]benzamide |
| EI07-28-02R | EI07-28 | 4J28 | N,N-Diethyl-4-[4-[3-fluoro-4-(5-hydroxypyridin-3-yl)benzoyl]piperazin-1-yl]benzamide |

| | | | |
|---|---|---|---|
| EI07-29-02R | EI07-29 | 4J29 | N,N-Diethyl-4-[4-[2-[2-(3-hydroxyphenyl)ethynyl]benzoyl]piperazin-1-yl]benzamide |
| EI07-30-02R | EI07-30 | 4J30 | N,N-Diethyl-4-[4-[2-[2-(5-hydroxypyridin-3-yl)ethynyl]benzoyl]piperazin-1-yl]benzamide |
| EI07-31-02R | EI07-31 | 4J31 | N,N-Diethyl-4-[4-[5-[2-(3-hydroxyphenyl)ethynyl]pyridine-3-carbonyl]piperazin-1-yl]benzamide |
| EI07-32-02R | EI07-32 | 4J32 | N,N-Diethyl-4-[4-[5-[2-(5-hydroxypyridin-3-yl)ethynyl]pyridine-3-carbonyl]piperazin-1-yl]benzamide |
| EI07-33-02R | EI07-33 | 4J33 | N,N-Diethyl-4-[4-[3-[2-(3-hydroxyphenyl)phenyl]propanoyl]piperazin-1-yl]benzamide |
| EI07-34-02R | EI07-34 | 4J34 | N,N-Diethyl-4-[4-[3-[2-(5-hydroxypyridin-3-yl)phenyl]propanoyl]piperazin-1-yl]benzamide |
| EI07-35-02R | EI07-35 | 4J35 | N,N-Diethyl-4-[4-[4-(3-hydroxyphenyl)naphthalene-1-carbonyl]piperazin-1-yl]benzamide |
| EI07-36-02R | EI07-36 | 4J36 | N,N-Diethyl-4-[4-[4-(5-hydroxypyridin-3-yl)naphthalene-1-carbonyl]piperazin-1-yl]benzamide |
| EI07-37-02R | EI07-37 | 4J37 | 4-[4-[3-tert-Butyl-5-(3-hydroxyphenyl)benzoyl]piperazin-1-yl]-N,N-diethylbenzamide |
| EI07-38-02R | EI07-38 | 4J38 | 4-[4-[3-tert-Butyl-5-(5-hydroxypyridin-3-yl)benzoyl]piperazin-1-yl]-N,N-diethylbenzamide |
| EI07-39-02R | EI07-39 | 4J39 | N,N-Diethyl-4-[4-[3-(3-hydroxyphenyl)-5-(trifluoromethyl)benzoyl]piperazin-1-yl]benzamide |
| EI07-40-02R | EI07-40 | 4J40 | N,N-Diethyl-4-[4-[3-(5-hydroxypyridin-3-yl)-5-(trifluoromethyl)benzoyl]piperazin-1-yl]benzamide |
| EI07-41-02R | EI07-41 | 4J41 | N,N-Diethyl-4-[4-[3-[2-(3-hydroxyphenyl)ethynyl]-5-(trifluoromethyl)benzoyl]piperazin-1-yl]benzamide |
| EI07-42-02R | EI07-42 | 4J42 | N,N-Diethyl-4-[4-[3-[2-(5-hydroxypyridin-3-yl)ethynyl]-5-(trifluoromethyl)benzoyl]piperazin-1-yl]benzamide |
| EI07-43-02R | EI07-43 | 4J43 | N,N-Diethyl-4-[4-[3-[4-(3-hydroxyphenyl)pyrazol-1-yl]-5-(trifluoromethyl)benzoyl]piperazin-1-yl]benzamide |
| EI07-44-02R | EI07-44 | 4J44 | N,N-Diethyl-4-[4-[3-[4-(5-hydroxypyridin-3-yl)pyrazol-1-yl]-5-(trifluoromethyl)benzoyl]piperazin-1-yl]benzamide |
| EI07-45-02R | EI07-45 | 4J45 | N,N-Diethyl-4-[4-[[2-(3-hydroxyphenyl)phenyl]methyl]piperazin-1-yl]benzamide |
| EI07-46-02R | EI07-46 | 4J46 | N,N-Diethyl-4-[4-[[2-(5-hydroxypyridin-3-yl)phenyl]methyl]piperazin-1-yl]benzamide |
| EI07-47-02R | EI07-47 | 4J47 | N,N-Diethyl-4-[4-[[4-(3-hydroxyphenyl)-3-methylphenyl]methyl]piperazin-1-yl]benzamide |
| EI07-48-02R | EI07-48 | 4J48 | N,N-Diethyl-4-[4-[[4-(5-hydroxypyridin-3-yl)-3-methylphenyl]methyl]piperazin-1-yl]benzamide |
| EI07-49-02R | EI07-49 | 4J49 | N,N-Diethyl-4-[4-[[2-ethyl-4-(3-hydroxyphenyl)phenyl]methyl]piperazin-1-yl]benzamide |
| EI07-50-02R | EI07-50 | 4J50 | N,N-Diethyl-4-[4-[[2-ethyl-4-(5-hydroxypyridin-3-yl)phenyl]methyl]piperazin-1-yl]benzamide |
| EI07-51-02R | EI07-51 | 4J51 | N,N-Diethyl-4-[4-[[3-fluoro-4-(3-hydroxyphenyl)phenyl]methyl]piperazin-1-yl]benzamide |
| EI07-52-02R | EI07-52 | 4J52 | N,N-Diethyl-4-[4-[[3-fluoro-4-(5-hydroxypyridin-3-yl)phenyl]methyl]piperazin-1-yl]benzamide |
| EI07-53-02R | EI07-53 | 4J53 | N,N-Diethyl-4-[4-[[4-(3-hydroxyphenyl)-2-methoxyphenyl]methyl]piperazin-1-yl]benzamide |
| EI07-54-02R | EI07-54 | 4J54 | N,N-Diethyl-4-[4-[[4-(5-hydroxypyridin-3-yl)-2-methoxyphenyl]methyl]piperazin-1-yl]benzamide |
| EI07-55-02R | EI07-55 | 4J55 | 4-[4-[[3-Ethoxy-5-(3-hydroxyphenyl)phenyl]methyl]piperazin-1-yl]-N,N-diethylbenzamide |
| EI07-56-02R | EI07-56 | 4J56 | 4-[4-[[3-Ethoxy-5-(5-hydroxypyridin-3-yl)phenyl]methyl]piperazin-1-yl]-N,N-diethylbenzamide |

| | | | |
|---|---|---|---|
| EI07-57-02R | EI07-57 | 4J57 | N,N-Diethyl-4-[4-[[3-(3-hydroxyphenyl)-5-propan-2-yloxyphenyl]methyl]piperazin-1-yl]benzamide |
| EI07-58-02R | EI07-58 | 4J58 | N,N-Diethyl-4-[4-[[3-(5-hydroxypyridin-3-yl)-5-propan-2-yloxyphenyl]methyl]piperazin-1-yl]benzamide |
| EI07-59-02R | EI07-59 | 4J59 | N,N-Diethyl-4-[4-[[4-(3-hydroxyphenyl)-2,6-dimethoxyphenyl]methyl]piperazin-1-yl]benzamide |
| EI07-60-02R | EI07-60 | 4J60 | N,N-Diethyl-4-[4-[[4-(5-hydroxypyridin-3-yl)-2,6-dimethoxyphenyl]methyl]piperazin-1-yl]benzamide |
| EI07-61-02R | EI07-61 | 4J61 | N,N-Diethyl-4-[4-[[3-(3-hydroxyphenyl)-5-(trifluoromethyl)phenyl]methyl]piperazin-1-yl]benzamide |
| EI07-62-02R | EI07-62 | 4J62 | N,N-Diethyl-4-[4-[[3-(5-hydroxypyridin-3-yl)-5-(trifluoromethyl)phenyl]methyl]piperazin-1-yl]benzamide |
| EI07-63-02R | EI07-63 | 4J63 | N,N-Diethyl-4-[4-[[3-(3-hydroxyphenyl)-5-(trifluoromethoxy)phenyl]methyl]piperazin-1-yl]benzamide |
| EI07-64-02R | EI07-64 | 4J64 | N,N-Diethyl-4-[4-[[3-(5-hydroxypyridin-3-yl)-5-(trifluoromethoxy)phenyl]methyl]piperazin-1-yl]benzamide |
| EI07-65-02R | EI07-65 | 4J65 | N,N-Diethyl-4-[4-[[4-(3-hydroxyphenyl)naphthalen-1-yl]methyl]piperazin-1-yl]benzamide |
| EI07-66-02R | EI07-66 | 4J66 | N,N-Diethyl-4-[4-[[4-(5-hydroxypyridin-3-yl)naphthalen-1-yl]methyl]piperazin-1-yl]benzamide |
| EI07-67-02R | EI07-67 | 4J67 | N,N-Diethyl-4-[4-[[4-(3-hydroxyphenyl)thiophen-2-yl]methyl]piperazin-1-yl]benzamide |
| EI07-68-02R | EI07-68 | 4J68 | N,N-Diethyl-4-[4-[[4-(5-hydroxypyridin-3-yl)thiophen-2-yl]methyl]piperazin-1-yl]benzamide |
| EI07-69-02R | EI07-69 | 4J69 | N,N-Diethyl-4-[4-[[2-[2-(3-hydroxyphenyl)ethynyl]phenyl]methyl]piperazin-1-yl]benzamide |
| EI07-70-02R | EI07-70 | 4J70 | N,N-Diethyl-4-[4-[[2-[2-(5-hydroxypyridin-3-yl)ethynyl]phenyl]methyl]piperazin-1-yl]benzamide |
| EI07-71-02R | EI07-71 | 4J71 | N,N-Diethyl-6-[4-[[2-(3-hydroxyphenyl)phenyl]methyl]piperazin-1-yl]pyridazine-3-carboxamide |
| EI07-72-02R | EI07-72 | 4J72 | N,N-Diethyl-6-[4-[[2-(5-hydroxypyridin-3-yl)phenyl]methyl]piperazin-1-yl]pyridazine-3-carboxamide |
| EI07-73-02R | EI07-73 | 4J73 | N,N-Diethyl-6-[4-[[4-(3-hydroxyphenyl)-3-methylphenyl]methyl]piperazin-1-yl]pyridazine-3-carboxamide |
| EI07-74-02R | EI07-74 | 4J74 | N,N-Diethyl-6-[4-[[4-(5-hydroxypyridin-3-yl)-3-methylphenyl]methyl]piperazin-1-yl]pyridazine-3-carboxamide |
| EI07-75-02R | EI07-75 | 4J75 | N,N-Diethyl-6-[4-[[2-ethyl-4-(3-hydroxyphenyl)phenyl]methyl]piperazin-1-yl]pyridazine-3-carboxamide |
| EI07-76-02R | EI07-76 | 4J76 | N,N-Diethyl-6-[4-[[2-ethyl-4-(5-hydroxypyridin-3-yl)phenyl]methyl]piperazin-1-yl]pyridazine-3-carboxamide |
| EI07-77-02R | EI07-77 | 4J77 | N,N-Diethyl-6-[4-[[3-fluoro-4-(3-hydroxyphenyl)phenyl]methyl]piperazin-1-yl]pyridazine-3-carboxamide |
| EI07-78-02R | EI07-78 | 4J78 | N,N-Diethyl-6-[4-[[3-fluoro-4-(5-hydroxypyridin-3-yl)phenyl]methyl]piperazin-1-yl]pyridazine-3-carboxamide |
| EI07-79-02R | EI07-79 | 4J79 | N,N-Diethyl-6-[4-[[4-(3-hydroxyphenyl)-2-methoxyphenyl]methyl]piperazin-1-yl]pyridazine-3-carboxamide |
| EI07-80-02R | EI07-80 | 4J80 | N,N-Diethyl-6-[4-[[4-(5-hydroxypyridin-3-yl)-2-methoxyphenyl]methyl]piperazin-1-yl]pyridazine-3-carboxamide |
| EI07-81-02R | EI07-81 | 4J81 | 6-[4-[[3-Ethoxy-5-(3-hydroxyphenyl)phenyl]methyl]piperazin-1-yl]-N,N-diethylpyridazine-3-carboxamide |
| EI07-82-02R | EI07-82 | 4J82 | 6-[4-[[3-Ethoxy-5-(5-hydroxypyridin-3-yl)phenyl]methyl]piperazin-1-yl]-N,N-diethylpyridazine-3-carboxamide |
| EI07-83-02R | EI07-83 | 4J83 | N,N-Diethyl-6-[4-[[3-(3-hydroxyphenyl)-5-propan-2-yloxyphenyl]methyl]piperazin-1-yl]pyridazine-3-carboxamide |

| | | | |
|---|---|---|---|
| EI07-84-02R | EI07-84 | 4J84 | N,N-Diethyl-6-[4-[[3-(5-hydroxypyridin-3-yl)-5-propan-2-yloxyphenyl]methyl]piperazin-1-yl]pyridazine-3-carboxamide |
| EI07-85-02R | EI07-85 | 4J85 | N,N-Diethyl-6-[4-[[4-(3-hydroxyphenyl)-2,6-dimethoxyphenyl]methyl]piperazin-1-yl]pyridazine-3-carboxamide |
| EI07-86-02R | EI07-86 | 4J86 | N,N-Diethyl-6-[4-[[4-(5-hydroxypyridin-3-yl)-2,6-dimethoxyphenyl]methyl]piperazin-1-yl]pyridazine-3-carboxamide |
| EI07-87-02R | EI07-87 | 4J87 | N,N-Diethyl-6-[4-[[3-(3-hydroxyphenyl)-5-(trifluoromethyl)phenyl]methyl]piperazin-1-yl]pyridazine-3-carboxamide |
| EI07-88-02R | EI07-88 | 4J88 | N,N-Diethyl-6-[4-[[3-(5-hydroxypyridin-3-yl)-5-(trifluoromethyl)phenyl]methyl]piperazin-1-yl]pyridazine-3-carboxamide |
| EI07-89-02R | EI07-89 | 4J89 | N,N-Diethyl-6-[4-[[3-(3-hydroxyphenyl)-5-(trifluoromethoxy)phenyl]methyl]piperazin-1-yl]pyridazine-3-carboxamide |
| EI07-90-02R | EI07-90 | 4J90 | N,N-Diethyl-6-[4-[[3-(5-hydroxypyridin-3-yl)-5-(trifluoromethoxy)phenyl]methyl]piperazin-1-yl]pyridazine-3-carboxamide |
| EI07-91-02R | EI07-91 | 4J91 | N,N-Diethyl-6-[4-[[4-(3-hydroxyphenyl)naphthalen-1-yl]methyl]piperazin-1-yl]pyridazine-3-carboxamide |
| EI07-92-02R | EI07-92 | 4J92 | N,N-Diethyl-6-[4-[[4-(5-hydroxypyridin-3-yl)naphthalen-1-yl]methyl]piperazin-1-yl]pyridazine-3-carboxamide |
| EI07-93-02R | EI07-93 | 4J93 | N,N-Diethyl-6-[4-[[4-(3-hydroxyphenyl)thiophen-2-yl]methyl]piperazin-1-yl]pyridazine-3-carboxamide |
| EI07-94-02R | EI07-94 | 4J94 | N,N-Diethyl-6-[4-[[4-(5-hydroxypyridin-3-yl)thiophen-2-yl]methyl]piperazin-1-yl]pyridazine-3-carboxamide |
| EI07-95-02R | EI07-95 | 4J95 | N,N-Diethyl-6-[4-[[2-[2-(3-hydroxyphenyl)ethynyl]phenyl]methyl]piperazin-1-yl]pyridazine-3-carboxamide |
| EI07-96-02R | EI07-96 | 4J96 | N,N-Diethyl-6-[4-[[2-[2-(5-hydroxypyridin-3-yl)ethynyl]phenyl]methyl]piperazin-1-yl]pyridazine-3-carboxamide |
| EI07-97-02R | EI07-97 | 4J97 | N,N-Diethyl-4-[4-[1-[4-(3-hydroxyphenyl)phenyl]ethyl]piperazin-1-yl]benzamide |
| EI07-98-02R | EI07-98 | 4J98 | N,N-Diethyl-4-[4-[1-[4-(5-hydroxypyridin-3-yl)phenyl]ethyl]piperazin-1-yl]benzamide |
| EI07-99-02R | EI07-99 | 4J99 | N,N-Diethyl-4-[4-[1-[2-fluoro-4-(3-hydroxyphenyl)phenyl]ethyl]piperazin-1-yl]benzamide |
| EI07-100-02R | EI07-100 | 4J100 | N,N-Diethyl-4-[4-[1-[2-fluoro-4-(5-hydroxypyridin-3-yl)phenyl]ethyl]piperazin-1-yl]benzamide |

Example 4-3-8: Synthesis of mixtures mixEI08-02R to mixEI10-02R

The title mixtures shown in Table EI08 to Table EI10 were obtained by synthesis in the same manner as in Example 4-3-7 using mixture mixEI06-02R (50 mg, loading rate: 0.648 mmol/g, 0.0324 mmol) shown in Table EI06 and amine reagents (BB31 to BB33). Compounds contained in the obtained mixtures are shown in Table EI08 to Table EI10.

[Table 203]

[Table EI08] Mixture No: mixEI08-02R; Amin reagent used: BB31; Mixture No. after cleavage: mixEI08

| Resin compound No. | Compound No. after cleavage | Another name of compound after cleavage in Table EJ02 | Compound name |
|---|---|---|---|
| EI08-01-02R | EI08-01 | 1M01 | 6-[4-[4-(3-Hydroxyphenyl)thiophene-2-carbonyl]piperazin-1-yl]-N-propylpyridazine-3-carboxamide |
| EI08-02-02R | EI08-02 | 1M02 | 6-[4-[4-(5-Hydroxypyridin-3-yl)thiophene-2-carbonyl]piperazin-1-yl]-N-propylpyridazine-3-carboxamide |
| EI08-03-02R | EI08-03 | 1M03 | 6-[4-[4-(3-Hydroxyphenyl)-3-methylbenzoyl]piperazin-1-yl]-N-propylpyridazine-3-carboxamide |
| EI08-04-02R | EI08-04 | 1M04 | 6-[4-[4-(5-Hydroxypyridin-3-yl)-3-methylbenzoyl]piperazin-1-yl]-N-propylpyridazine-3-carboxamide |
| EI08-05-02R | EI08-05 | 1M05 | 6-[4-[3-Fluoro-4-(3-hydroxyphenyl)benzoyl]piperazin-1-yl]-N-propylpyridazine-3-carboxamide |
| EI08-06-02R | EI08-06 | 1M06 | 6-[4-[3-Fluoro-4-(5-hydroxypyridin-3-yl)benzoyl]piperazin-1-yl]-N-propylpyridazine-3-carboxamide |
| EI08-07-02R | EI08-07 | 1M07 | 6-[4-[2-[2-(3-Hydroxyphenyl)ethynyl]benzoyl]piperazin-1-yl]-N-propylpyridazine-3-carboxamide |
| EI08-08-02R | EI08-08 | 1M08 | 6-[4-[2-[2-(5-Hydroxypyridin-3-yl)ethynyl]benzoyl]piperazin-1-yl]-N-propylpyridazine-3-carboxamide |
| EI08-09-02R | EI08-09 | 1M09 | 6-[4-[5-[2-(3-Hydroxyphenyl)ethynyl]pyridine-3-carbonyl]piperazin-1-yl]-N-propylpyridazine-3-carboxamide |
| EI08-10-02R | EI08-10 | 1M10 | 6-[4-[5-[2-(5-Hydroxypyridin-3-yl)ethynyl]pyridine-3-carbonyl]piperazin-1-yl]-N-propylpyridazine-3-carboxamide |
| EI08-11-02R | EI08-11 | 1M11 | 6-[4-[3-[2-(3-Hydroxyphenyl)phenyl]propanoyl]piperazin-1-yl]-N-propylpyridazine-3-carboxamide |
| EI08-12-02R | EI08-12 | 1M12 | 6-[4-[3-[2-(5-Hydroxypyridin-3-yl)phenyl]propanoyl]piperazin-1-yl]-N-propylpyridazine-3-carboxamide |
| EI08-13-02R | EI08-13 | 1M13 | 6-[4-[4-(3-Hydroxyphenyl)naphthalene-1-carbonyl]piperazin-1-yl]-N-propylpyridazine-3-carboxamide |
| EI08-14-02R | EI08-14 | 1M14 | 6-[4-[4-(5-Hydroxypyridin-3-yl)naphthalene-1-carbonyl]piperazin-1-yl]-N-propylpyridazine-3-carboxamide |
| EI08-15-02R | EI08-15 | 1M15 | 6-[4-[3-tert-Butyl-5-(3-hydroxyphenyl)benzoyl]piperazin-1-yl]-N-propylpyridazine-3-carboxamide |
| EI08-16-02R | EI08-16 | 1M16 | 6-[4-[3-tert-Butyl-5-(5-hydroxypyridin-3-yl)benzoyl]piperazin-1-yl]-N-propylpyridazine-3-carboxamide |
| EI08-17-02R | EI08-17 | 1M17 | 6-[4-[3-(3-Hydroxyphenyl)-5-(trifluoromethyl)benzoyl]piperazin-1-yl]-N-propylpyridazine-3-carboxamide |
| EI08-18-02R | EI08-18 | 1M18 | 6-[4-[3-(5-Hydroxypyridin-3-yl)-5-(trifluoromethyl)benzoyl]piperazin-1-yl]-N-propylpyridazine-3-carboxamide |
| EI08-19-02R | EI08-19 | 1M19 | 6-[4-[3-[2-(3-Hydroxyphenyl)ethynyl]-5-(trifluoromethyl)benzoyl]piperazin-1-yl]-N-propylpyridazine-3-carboxamide |
| EI08-20-02R | EI08-20 | 1M20 | 6-[4-[3-[2-(5-Hydroxypyridin-3-yl)ethynyl]-5-(trifluoromethyl)benzoyl]piperazin-1-yl]-N-propylpyridazine-3-carboxamide |
| EI08-21-02R | EI08-21 | 1M21 | 6-[4-[3-[4-(3-Hydroxyphenyl)pyrazol-1-yl]-5-(trifluoromethyl)benzoyl]piperazin-1-yl]-N-propylpyridazine-3-carboxamide |
| EI08-22-02R | EI08-22 | 1M22 | 6-[4-[3-[4-(5-Hydroxypyridin-3-yl)pyrazol-1-yl]-5-(trifluoromethyl)benzoyl]piperazin-1-yl]-N-propylpyridazine-3-carboxamide |
| EI08-23-02R | EI08-23 | 1M23 | 4-[4-[4-(3-Hydroxyphenyl)thiophene-2-carbonyl]piperazin-1-yl]-N-propylbenzamide |

| | | | |
|---|---|---|---|
| EI08-24-02R | EI08-24 | 1M24 | 4-[4-[4-(5-Hydroxypyridin-3-yl)thiophene-2-carbonyl]piperazin-1-yl]-N-propylbenzamide |
| EI08-25-02R | EI08-25 | 1M25 | 4-[4-[4-(3-Hydroxyphenyl)-3-methylbenzoyl]piperazin-1-yl]-N-propylbenzamide |
| EI08-26-02R | EI08-26 | 1M26 | 4-[4-[4-(5-Hydroxypyridin-3-yl)-3-methylbenzoyl]piperazin-1-yl]-N-propylbenzamide |
| EI08-27-02R | EI08-27 | 1M27 | 4-[4-[3-Fluoro-4-(3-hydroxyphenyl)benzoyl]piperazin-1-yl]-N-propylbenzamide |
| EI08-28-02R | EI08-28 | 1M28 | 4-[4-[3-Fluoro-4-(5-hydroxypyridin-3-yl)benzoyl]piperazin-1-yl]-N-propylbenzamide |
| EI08-29-02R | EI08-29 | 1M29 | 4-[4-[2-[2-(3-Hydroxyphenyl)ethynyl]benzoyl]piperazin-1-yl]-N-propylbenzamide |
| EI08-30-02R | EI08-30 | 1M30 | 4-[4-[2-[2-(5-Hydroxypyridin-3-yl)ethynyl]benzoyl]piperazin-1-yl]-N-propylbenzamide |
| EI08-31-02R | EI08-31 | 1M31 | 4-[4-[5-[2-(3-Hydroxyphenyl)ethynyl]pyridine-3-carbonyl]piperazin-1-yl]-N-propylbenzamide |
| EI08-32-02R | EI08-32 | 1M32 | 4-[4-[5-[2-(5-Hydroxypyridin-3-yl)ethynyl]pyridine-3-carbonyl]piperazin-1-yl]-N-propylbenzamide |
| EI08-33-02R | EI08-33 | 1M33 | 4-[4-[3-[2-(3-Hydroxyphenyl)phenyl]propanoyl]piperazin-1-yl]-N-propylbenzamide |
| EI08-34-02R | EI08-34 | 1M34 | 4-[4-[3-[2-(5-Hydroxypyridin-3-yl)phenyl]propanoyl]piperazin-1-yl]-N-propylbenzamide |
| EI08-35-02R | EI08-35 | 1M35 | 4-[4-[4-(3-Hydroxyphenyl)naphthalene-1-carbonyl]piperazin-1-yl]-N-propylbenzamide |
| EI08-36-02R | EI08-36 | 1M36 | 4-[4-[4-(5-Hydroxypyridin-3-yl)naphthalene-1-carbonyl]piperazin-1-yl]-N-propylbenzamide |
| EI08-37-02R | EI08-37 | 1M37 | 4-[4-[3-tert-Butyl-5-(3-hydroxyphenyl)benzoyl]piperazin-1-yl]-N-propylbenzamide |
| EI08-38-02R | EI08-38 | 1M38 | 4-[4-[3-tert-Butyl-5-(5-hydroxypyridin-3-yl)benzoyl]piperazin-1-yl]-N-propylbenzamide |
| EI08-39-02R | EI08-39 | 1M39 | 4-[4-[3-(3-Hydroxyphenyl)-5-(trifluoromethyl)benzoyl]piperazin-1-yl]-N-propylbenzamide |
| EI08-40-02R | EI08-40 | 1M40 | 4-[4-[3-(5-Hydroxypyridin-3-yl)-5-(trifluoromethyl)benzoyl]piperazin-1-yl]-N-propylbenzamide |
| EI08-41-02R | EI08-41 | 1M41 | 4-[4-[3-[2-(3-Hydroxyphenyl)ethynyl]-5-(trifluoromethyl)benzoyl]piperazin-1-yl]-N-propylbenzamide |
| EI08-42-02R | EI08-42 | 1M42 | 4-[4-[3-[2-(5-Hydroxypyridin-3-yl)ethynyl]-5-(trifluoromethyl)benzoyl]piperazin-1-yl]-N-propylbenzamide |
| EI08-43-02R | EI08-43 | 1M43 | 4-[4-[3-[4-(3-Hydroxyphenyl)pyrazol-1-yl]-5-(trifluoromethyl)benzoyl]piperazin-1-yl]-N-propylbenzamide |
| EI08-44-02R | EI08-44 | 1M44 | 4-[4-[3-[4-(5-Hydroxypyridin-3-yl)pyrazol-1-yl]-5-(trifluoromethyl)benzoyl]piperazin-1-yl]-N-propylbenzamide |
| EI08-45-02R | EI08-45 | 1M45 | 4-[4-[[2-(3-Hydroxyphenyl)phenyl]methyl]piperazin-1-yl]-N-propylbenzamide |
| EI08-46-02R | EI08-46 | 1M46 | 4-[4-[[2-(5-Hydroxypyridin-3-yl)phenyl]methyl]piperazin-1-yl]-N-propylbenzamide |
| EI08-47-02R | EI08-47 | 1M47 | 4-[4-[[4-(3-Hydroxyphenyl)-3-methylphenyl]methyl]piperazin-1-yl]-N-propylbenzamide |
| EI08-48-02R | EI08-48 | 1M48 | 4-[4-[[4-(5-Hydroxypyridin-3-yl)-3-methylphenyl]methyl]piperazin-1-yl]-N-propylbenzamide |
| EI08-49-02R | EI08-49 | 1M49 | 4-[4-[[2-Ethyl-4-(3-hydroxyphenyl)phenyl]methyl]piperazin-1-yl]-N-propylbenzamide |
| EI08-50-02R | EI08-50 | 1M50 | 4-[4-[[2-Ethyl-4-(5-hydroxypyridin-3-yl)phenyl]methyl]piperazin-1-yl]-N-propylbenzamide |
| EI08-51-02R | EI08-51 | 1M51 | 4-[4-[[3-Fluoro-4-(3-hydroxyphenyl)phenyl]methyl]piperazin-1-yl]-N-propylbenzamide |

| | | | |
|---|---|---|---|
| EI08-52-02R | EI08-52 | 1M52 | 4-[4-[[3-Fluoro-4-(5-hydroxypyridin-3-yl)phenyl]methyl]piperazin-1-yl]-N-propylbenzamide |
| EI08-53-02R | EI08-53 | 1M53 | 4-[4-[[4-(3-Hydroxyphenyl)-2-methoxyphenyl]methyl]piperazin-1-yl]-N-propylbenzamide |
| EI08-54-02R | EI08-54 | 1M54 | 4-[4-[[4-(5-Hydroxypyridin-3-yl)-2-methoxyphenyl]methyl]piperazin-1-yl]-N-propylbenzamide |
| EI08-55-02R | EI08-55 | 1M55 | 4-[4-[[3-Ethoxy-5-(3-hydroxyphenyl)phenyl]methyl]piperazin-1-yl]-N-propylbenzamide |
| EI08-56-02R | EI08-56 | 1M56 | 4-[4-[[3-Ethoxy-5-(5-hHydroxypyridin-3-yl)phenyl]methyl]piperazin-1-yl]-N-propylbenzamide |
| EI08-57-02R | EI08-57 | 1M57 | 4-[4-[[3-(3-Hydroxyphenyl)-5-propan-2-yloxyphenyl]methyl]piperazin-1-yl]-N-propylbenzamide |
| EI08-58-02R | EI08-58 | 1M58 | 4-[4-[[3-(5-Hydroxypyridin-3-yl)-5-propan-2-yloxyphenyl]methyl]piperazin-1-yl]-N-propylbenzamide |
| EI08-59-02R | EI08-59 | 1M59 | 4-[4-[[4-(3-Hydroxyphenyl)-2,6-dimethoxyphenyl]methyl]piperazin-1-yl]-N-propylbenzamide |
| EI08-60-02R | EI08-60 | 1M60 | 4-[4-[[4-(5-Hydroxypyridin-3-yl)-2,6-dimethoxyphenyl]methyl]piperazin-1-yl]-N-propylbenzamide |
| EI08-61-02R | EI08-61 | 1M61 | 4-[4-[[3-(3-Hydroxyphenyl)-5-(trifluoromethyl)phenyl]methyl]piperazin-1-yl]-N-propylbenzamide |
| EI08-62-02R | EI08-62 | 1M62 | 4-[4-[[3-(5-Hydroxypyridin-3-yl)-5-(trifluoromethyl)phenyl]methyl]piperazin-1-yl]-N-propylbenzamide |
| EI08-63-02R | EI08-63 | 1M63 | 4-[4-[[3-(3-Hydroxyphenyl)-5-(trifluoromethoxy)phenyl]methyl]piperazin-1-yl]-N-propylbenzamide |
| EI08-64-02R | EI08-64 | 1M64 | 4-[4-[[3-(5-Hydroxypyridin-3-yl)-5-(trifluoromethoxy)phenyl]methyl]piperazin-1-yl]-N-propylbenzamide |
| EI08-65-02R | EI08-65 | 1M65 | 4-[4-[[4-(3-Hydroxyphenyl)naphthalen-1-yl]methyl]piperazin-1-yl]-N-propylbenzamide |
| EI08-66-02R | EI08-66 | 1M66 | 4-[4-[[4-(5-Hydroxypyridin-3-yl)naphthalen-1-yl]methyl]piperazin-1-yl]-N-propylbenzamide |
| EI08-67-02R | EI08-67 | 1M67 | 4-[4-[[4-(3-Hydroxyphenyl)thiophen-2-yl]methyl]piperazin-1-yl]-N-propylbenzamide |
| EI08-68-02R | EI08-68 | 1M68 | 4-[4-[[4-(5-Hydroxypyridin-3-yl)thiophen-2-yl]methyl]piperazin-1-yl]-N-propylbenzamide |
| EI08-69-02R | EI08-69 | 1M69 | 4-[4-[[2-[2-(3-Hydroxyphenyl)ethynyl]phenyl]methyl]piperazin-1-yl]-N-propylbenzamide |
| EI08-70-02R | EI08-70 | 1M70 | 4-[4-[[2-[2-(5-Hydroxypyridin-3-yl)ethynyl]phenyl]methyl]piperazin-1-yl]-N-propylbenzamide |
| EI08-71-02R | EI08-71 | 1M71 | 6-[4-[[2-(3-Hydroxyphenyl)phenyl]methyl]piperazin-1-yl]-N-propylpyridazine-3-carboxamide |
| EI08-72-02R | EI08-72 | 1M72 | 6-[4-[[2-(5-Hydroxypyridin-3-yl)phenyl]methyl]piperazin-1-yl]-N-propylpyridazine-3-carboxamide |
| EI08-73-02R | EI08-73 | 1M73 | 6-[4-[[4-(3-Hydroxyphenyl)-3-methylphenyl]methyl]piperazin-1-yl]-N-propylpyridazine-3-carboxamide |
| EI08-74-02R | EI08-74 | 1M74 | 6-[4-[[4-(5-Hydroxypyridin-3-yl)-3-methylphenyl]methyl]piperazin-1-yl]-N-propylpyridazine-3-carboxamide |
| EI08-75-02R | EI08-75 | 1M75 | 6-[4-[[2-Ethyl-4-(3-hydroxyphenyl)phenyl]methyl]piperazin-1-yl]-N-propylpyridazine-3-carboxamide |
| EI08-76-02R | EI08-76 | 1M76 | 6-[4-[[2-Ethyl-4-(5-hydroxypyridin-3-yl)phenyl]methyl]piperazin-1-yl]-N-propylpyridazine-3-carboxamide |
| EI08-77-02R | EI08-77 | 1M77 | 6-[4-[[3-Fluoro-4-(3-hydroxyphenyl)phenyl]methyl]piperazin-1-yl]-N-propylpyridazine-3-carboxamide |
| EI08-78-02R | EI08-78 | 1M78 | 6-[4-[[3-Fluoro-4-(5-hydroxypyridin-3-yl)phenyl]methyl]piperazin-1-yl]-N-propylpyridazine-3-carboxamide |

| | | | |
|---|---|---|---|
| EI08-79-02R | EI08-79 | 1M79 | 6-[4-[[4-(3-Hydroxyphenyl)-2-methoxyphenyl]methyl]piperazin-1-yl]-N-propylpyridazine-3-carboxamide |
| EI08-80-02R | EI08-80 | 1M80 | 6-[4-[[4-(5-Hydroxypyridin-3-yl)-2-methoxyphenyl]methyl]piperazin-1-yl]-N-propylpyridazine-3-carboxamide |
| EI08-81-02R | EI08-81 | 1M81 | 6-[4-[[3-Ethoxy-5-(3-hydroxyphenyl)phenyl]methyl]piperazin-1-yl]-N-propylpyridazine-3-carboxamide |
| EI08-82-02R | EI08-82 | 1M82 | 6-[4-[[3-Ethoxy-5-(5-hydroxypyridin-3-yl)phenyl]methyl]piperazin-1-yl]-N-propylpyridazine-3-carboxamide |
| EI08-83-02R | EI08-83 | 1M83 | 6-[4-[[3-(3-Hydroxyphenyl)-5-propan-2-yloxyphenyl]methyl]piperazin-1-yl]-N-propylpyridazine-3-carboxamide |
| EI08-84-02R | EI08-84 | 1M84 | 6-[4-[[3-(5-Hydroxypyridin-3-yl)-5-propan-2-yloxyphenyl]methyl]piperazin-1-yl]-N-propylpyridazine-3-carboxamide |
| EI08-85-02R | EI08-85 | 1M85 | 6-[4-[[4-(3-Hydroxyphenyl)-2,6-dimethoxyphenyl]methyl]piperazin-1-yl]-N-propylpyridazine-3-carboxamide |
| EI08-86-02R | EI08-86 | 1M86 | 6-[4-[[4-(5-Hydroxypyridin-3-yl)-2,6-dimethoxyphenyl]methyl]piperazin-1-yl]-N-propylpyridazine-3-carboxamide |
| EI08-87-02R | EI08-87 | 1M87 | 6-[4-[[3-(3-Hydroxyphenyl)-5-(trifluoromethyl)phenyl]methyl]piperazin-1-yl]-N-propylpyridazine-3-carboxamide |
| EI08-88-02R | EI08-88 | 1M88 | 6-[4-[[3-(5-Hydroxypyridin-3-yl)-5-(trifluoromethyl)phenyl]methyl]piperazin-1-yl]-N-propylpyridazine-3-carboxamide |
| EI08-89-02R | EI08-89 | 1M89 | 6-[4-[[3-(3-Hydroxyphenyl)-5-(trifluoromethoxy)phenyl]methyl]piperazin-1-yl]-N-propylpyridazine-3-carboxamide |
| EI08-90-02R | EI08-90 | 1M90 | 6-[4-[[3-(5-Hydroxypyridin-3-yl)-5-(trifluoromethoxy)phenyl]methyl]piperazin-1-yl]-N-propylpyridazine-3-carboxamide |
| EI08-91-02R | EI08-91 | 1M91 | 6-[4-[[4-(3-Hydroxyphenyl)naphthalen-1-yl]methyl]piperazin-1-yl]-N-propylpyridazine-3-carboxamide |
| EI08-92-02R | EI08-92 | 1M92 | 6-[4-[[4-(5-Hydroxypyridin-3-yl)naphthalen-1-yl]methyl]piperazin-1-yl]-N-propylpyridazine-3-carboxamide |
| EI08-93-02R | EI08-93 | 1M93 | 6-[4-[[4-(3-Hydroxyphenyl)thiophen-2-yl]methyl]piperazin-1-yl]-N-propylpyridazine-3-carboxamide |
| EI08-94-02R | EI08-94 | 1M94 | 6-[4-[[4-(5-Hydroxypyridin-3-yl)thiophen-2-yl]methyl]piperazin-1-yl]-N-propylpyridazine-3-carboxamide |
| EI08-95-02R | EI08-95 | 1M95 | 6-[4-[[2-[2-(3-Hydroxyphenyl)ethynyl]phenyl]methyl]piperazin-1-yl]-N-propylpyridazine-3-carboxamide |
| EI08-96-02R | EI08-96 | 1M96 | 6-[4-[[2-[2-(5-Hydroxypyridin-3-yl)ethynyl]phenyl]methyl]piperazin-1-yl]-N-propylpyridazine-3-carboxamide |
| EI08-97-02R | EI08-97 | 1M97 | 4-[4-[1-[4-(3-Hydroxyphenyl)phenyl]ethyl]piperazin-1-yl]-N-propylbenzamide |
| EI08-98-02R | EI08-98 | 1M98 | 4-[4-[1-[4-(5-Hydroxypyridin-3-yl)phenyl]ethyl]piperazin-1-yl]-N-propylbenzamide |
| EI08-99-02R | EI08-99 | 1M99 | 4-[4-[1-[2-Fluoro-4-(3-hydroxyphenyl)phenyl]ethyl]piperazin-1-yl]-N-propylbenzamide |
| EI08-100-02R | EI08-100 | 1M100 | 4-[4-[1-[2-Fluoro-4-(5-hydroxypyridin-3-yl)phenyl]ethyl]piperazin-1-yl]-N-propylbenzamide |

[Table 204]

[Table EI09] Mixture No: mixEI09-02R; Amin reagent used: BB32; Mixture No. after

cleavage: mixEI09

| Resin compound No. | Compound No. after cleavage | Another name of compound after cleavage in Table EJ03 | Compound name |
|---|---|---|---|
| EI09-01-02R | EI09-01 | 1K01 | 6-[4-[4-(3-Hydroxyphenyl)thiophene-2-carbonyl]piperazin-1-yl]-N-(2,2,2-trifluoroethyl)pyridazine-3-carboxamide |
| EI09-02-02R | EI09-02 | 1K02 | 6-[4-[4-(5-Hydroxypyridin-3-yl)thiophene-2-carbonyl]piperazin-1-yl]-N-(2,2,2-trifluoroethyl)pyridazine-3-carboxamide |
| EI09-03-02R | EI09-03 | 1K03 | 6-[4-[4-(3-Hydroxyphenyl)-3-methylbenzoyl]piperazin-1-yl]-N-(2,2,2-trifluoroethyl)pyridazine-3-carboxamide |
| EI09-04-02R | EI09-04 | 1K04 | 6-[4-[4-(5-Hydroxypyridin-3-yl)-3-methylbenzoyl]piperazin-1-yl]-N-(2,2,2-trifluoroethyl)pyridazine-3-carboxamide |
| EI09-05-02R | EI09-05 | 1K05 | 6-[4-[3-Fluoro-4-(3-hydroxyphenyl)benzoyl]piperazin-1-yl]-N-(2,2,2-trifluoroethyl)pyridazine-3-carboxamide |
| EI09-06-02R | EI09-06 | 1K06 | 6-[4-[3-Fluoro-4-(5-hydroxypyridin-3-yl)benzoyl]piperazin-1-yl]-N-(2,2,2-trifluoroethyl)pyridazine-3-carboxamide |
| EI09-07-02R | EI09-07 | 1K07 | 6-[4-[2-[2-(3-Hydroxyphenyl)ethynyl]benzoyl]piperazin-1-yl]-N-(2,2,2-trifluoroethyl)pyridazine-3-carboxamide |
| EI09-08-02R | EI09-08 | 1K08 | 6-[4-[2-[2-(5-Hydroxypyridin-3-yl)ethynyl]benzoyl]piperazin-1-yl]-N-(2,2,2-trifluoroethyl)pyridazine-3-carboxamide |
| EI09-09-02R | EI09-09 | 1K09 | 6-[4-[5-[2-(3-Hydroxyphenyl)ethynyl]pyridine-3-carbonyl]piperazin-1-yl]-N-(2,2,2-trifluoroethyl)pyridazine-3-carboxamide |
| EI09-10-02R | EI09-10 | 1K10 | 6-[4-[5-[2-(5-Hydroxypyridin-3-yl)ethynyl]pyridine-3-carbonyl]piperazin-1-yl]-N-(2,2,2-trifluoroethyl)pyridazine-3-carboxamide |
| EI09-11-02R | EI09-11 | 1K11 | 6-[4-[3-[2-(3-Hydroxyphenyl)phenyl]propanoyl]piperazin-1-yl]-N-(2,2,2-trifluoroethyl)pyridazine-3-carboxamide |
| EI09-12-02R | EI09-12 | 1K12 | 6-[4-[3-[2-(5-Hydroxypyridin-3-yl)phenyl]propanoyl]piperazin-1-yl]-N-(2,2,2-trifluoroethyl)pyridazine-3-carboxamide |
| EI09-13-02R | EI09-13 | 1K13 | 6-[4-[4-(3-Hydroxyphenyl)naphthalene-1-carbonyl]piperazin-1-yl]-N-(2,2,2-trifluoroethyl)pyridazine-3-carboxamide |
| EI09-14-02R | EI09-14 | 1K14 | 6-[4-[4-(5-Hydroxypyridin-3-yl)naphthalene-1-carbonyl]piperazin-1-yl]-N-(2,2,2-trifluoroethyl)pyridazine-3-carboxamide |
| EI09-15-02R | EI09-15 | 1K15 | 6-[4-[3-tert-Butyl-5-(3-hydroxyphenyl)benzoyl]piperazin-1-yl]-N-(2,2,2-trifluoroethyl)pyridazine-3-carboxamide |
| EI09-16-02R | EI09-16 | 1K16 | 6-[4-[3-tert-Butyl-5-(5-hydroxypyridin-3-yl)benzoyl]piperazin-1-yl]-N-(2,2,2-trifluoroethyl)pyridazine-3-carboxamide |
| EI09-17-02R | EI09-17 | 1K17 | 6-[4-[3-(3-Hydroxyphenyl)-5-(trifluoromethyl)benzoyl]piperazin-1-yl]-N-(2,2,2-trifluoroethyl)pyridazine-3-carboxamide |
| EI09-18-02R | EI09-18 | 1K18 | 6-[4-[3-(5-Hydroxypyridin-3-yl)-5-(trifluoromethyl)benzoyl]piperazin-1-yl]-N-(2,2,2-trifluoroethyl)pyridazine-3-carboxamide |
| EI09-19-02R | EI09-19 | 1K19 | 6-[4-[3-[2-(3-Hydroxyphenyl)ethynyl]-5-(trifluoromethyl)benzoyl]piperazin-1-yl]-N-(2,2,2-trifluoroethyl)pyridazine-3-carboxamide |
| EI09-20-02R | EI09-20 | 1K20 | 6-[4-[3-[2-(5-Hydroxypyridin-3-yl)ethynyl]-5-(trifluoromethyl)benzoyl]piperazin-1-yl]-N-(2,2,2-trifluoroethyl)pyridazine-3-carboxamide |
| EI09-21-02R | EI09-21 | 1K21 | 6-[4-[3-[4-(3-Hydroxyphenyl)pyrazol-1-yl]-5-(trifluoromethyl)benzoyl]piperazin-1-yl]-N-(2,2,2-trifluoroethyl)pyridazine-3-carboxamide |

| | | | |
|---|---|---|---|
| EI09-22-02R | EI09-22 | 1K22 | 6-[4-[3-[4-(5-Hydroxypyridin-3-yl)pyrazol-1-yl]-5-(trifluoromethyl)benzoyl]piperazin-1-yl]-N-(2,2,2-trifluoroethyl)pyridazine-3-carboxamide |
| EI09-23-02R | EI09-23 | 1K23 | 4-[4-[4-(3-Hydroxyphenyl)thiophene-2-carbonyl]piperazin-1-yl]-N-(2,2,2-trifluoroethyl)benzamide |
| EI09-24-02R | EI09-24 | 1K24 | 4-[4-[4-(5-Hydroxypyridin-3-yl)thiophene-2-carbonyl]piperazin-1-yl]-N-(2,2,2-trifluoroethyl)benzamide |
| EI09-25-02R | EI09-25 | 1K25 | 4-[4-[4-(3-Hydroxyphenyl)-3-methylbenzoyl]piperazin-1-yl]-N-(2,2,2-trifluoroethyl)benzamide |
| EI09-26-02R | EI09-26 | 1K26 | 4-[4-[4-(5-Hydroxypyridin-3-yl)-3-methylbenzoyl]piperazin-1-yl]-N-(2,2,2-trifluoroethyl)benzamide |
| EI09-27-02R | EI09-27 | 1K27 | 4-[4-[3-Fluoro-4-(3-hydroxyphenyl)benzoyl]piperazin-1-yl]-N-(2,2,2-trifluoroethyl)benzamide |
| EI09-28-02R | EI09-28 | 1K28 | 4-[4-[3-Fluoro-4-(5-hydroxypyridin-3-yl)benzoyl]piperazin-1-yl]-N-(2,2,2-trifluoroethyl)benzamide |
| EI09-29-02R | EI09-29 | 1K29 | 4-[4-[2-[2-(3-Hydroxyphenyl)ethynyl]benzoyl]piperazin-1-yl]-N-(2,2,2-trifluoroethyl)benzamide |
| EI09-30-02R | EI09-30 | 1K30 | 4-[4-[2-[2-(5-Hydroxypyridin-3-yl)ethynyl]benzoyl]piperazin-1-yl]-N-(2,2,2-trifluoroethyl)benzamide |
| EI09-31-02R | EI09-31 | 1K31 | 4-[4-[5-[2-(3-Hydroxyphenyl)ethynyl]pyridine-3-carbonyl]piperazin-1-yl]-N-(2,2,2-trifluoroethyl)benzamide |
| EI09-32-02R | EI09-32 | 1K32 | 4-[4-[5-[2-(5-Hydroxypyridin-3-yl)ethynyl]pyridine-3-carbonyl]piperazin-1-yl]-N-(2,2,2-trifluoroethyl)benzamide |
| EI09-33-02R | EI09-33 | 1K33 | 4-[4-[3-[2-(3-Hydroxyphenyl)phenyl]propanoyl]piperazin-1-yl]-N-(2,2,2-trifluoroethyl)benzamide |
| EI09-34-02R | EI09-34 | 1K34 | 4-[4-[3-[2-(5-Hydroxypyridin-3-yl)phenyl]propanoyl]piperazin-1-yl]-N-(2,2,2-trifluoroethyl)benzamide |
| EI09-35-02R | EI09-35 | 1K35 | 4-[4-[4-(3-Hydroxyphenyl)naphthalene-1-carbonyl]piperazin-1-yl]-N-(2,2,2-trifluoroethyl)benzamide |
| EI09-36-02R | EI09-36 | 1K36 | 4-[4-[4-(5-Hydroxypyridin-3-yl)naphthalene-1-carbonyl]piperazin-1-yl]-N-(2,2,2-trifluoroethyl)benzamide |
| EI09-37-02R | EI09-37 | 1K37 | 4-[4-[3-tert-Butyl-5-(3-hydroxyphenyl)benzoyl]piperazin-1-yl]-N-(2,2,2-trifluoroethyl)benzamide |
| EI09-38-02R | EI09-38 | 1K38 | 4-[4-[3-tert-Butyl-5-(5-hydroxypyridin-3-yl)benzoyl]piperazin-1-yl]-N-(2,2,2-trifluoroethyl)benzamide |
| EI09-39-02R | EI09-39 | 1K39 | 4-[4-[3-(3-Hydroxyphenyl)-5-(trifluoromethyl)benzoyl]piperazin-1-yl]-N-(2,2,2-trifluoroethyl)benzamide |
| EI09-40-02R | EI09-40 | 1K40 | 4-[4-[3-(5-Hydroxypyridin-3-yl)-5-(trifluoromethyl)benzoyl]piperazin-1-yl]-N-(2,2,2-trifluoroethyl)benzamide |
| EI09-41-02R | EI09-41 | 1K41 | 4-[4-[3-[2-(3-Hydroxyphenyl)ethynyl]-5-(trifluoromethyl)benzoyl]piperazin-1-yl]-N-(2,2,2-trifluoroethyl)benzamide |
| EI09-42-02R | EI09-42 | 1K42 | 4-[4-[3-[2-(5-Hydroxypyridin-3-yl)ethynyl]-5-(trifluoromethyl)benzoyl]piperazin-1-yl]-N-(2,2,2-trifluoroethyl)benzamide |
| EI09-43-02R | EI09-43 | 1K43 | 4-[4-[3-[4-(3-Hydroxyphenyl)pyrazol-1-yl]-5-(trifluoromethyl)benzoyl]piperazin-1-yl]-N-(2,2,2-trifluoroethyl)benzamide |
| EI09-44-02R | EI09-44 | 1K44 | 4-[4-[3-[4-(5-Hydroxypyridin-3-yl)pyrazol-1-yl]-5-(trifluoromethyl)benzoyl]piperazin-1-yl]-N-(2,2,2-trifluoroethyl)benzamide |
| EI09-45-02R | EI09-45 | 1K45 | 4-[4-[[2-(3-Hydroxyphenyl)phenyl]methyl]piperazin-1-yl]-N-(2,2,2-trifluoroethyl)benzamide |
| EI09-46-02R | EI09-46 | 1K46 | 4-[4-[[2-(5-Hydroxypyridin-3-yl)phenyl]methyl]piperazin-1-yl]-N-(2,2,2-trifluoroethyl)benzamide |

| | | | |
|---|---|---|---|
| EI09-47-02R | EI09-47 | 1K47 | 4-[4-[[4-(3-Hydroxyphenyl)-3-methylphenyl]methyl]piperazin-1-yl]-N-(2,2,2-trifluoroethyl)benzamide |
| EI09-48-02R | EI09-48 | 1K48 | 4-[4-[[4-(5-Hydroxypyridin-3-yl)-3-methylphenyl]methyl]piperazin-1-yl]-N-(2,2,2-trifluoroethyl)benzamide |
| EI09-49-02R | EI09-49 | 1K49 | 4-[4-[[2-Ethyl-4-(3-hydroxyphenyl)phenyl]methyl]piperazin-1-yl]-N-(2,2,2-trifluoroethyl)benzamide |
| EI09-50-02R | EI09-50 | 1K50 | 4-[4-[[2-Ethyl-4-(5-hydroxypyridin-3-yl)phenyl]methyl]piperazin-1-yl]-N-(2,2,2-trifluoroethyl)benzamide |
| EI09-51-02R | EI09-51 | 1K51 | 4-[4-[[3-Fluoro-4-(3-hydroxyphenyl)phenyl]methyl]piperazin-1-yl]-N-(2,2,2-trifluoroethyl)benzamide |
| EI09-52-02R | EI09-52 | 1K52 | 4-[4-[[3-Fluoro-4-(5-hydroxypyridin-3-yl)phenyl]methyl]piperazin-1-yl]-N-(2,2,2-trifluoroethyl)benzamide |
| EI09-53-02R | EI09-53 | 1K53 | 4-[4-[[4-(3-Hydroxyphenyl)-2-methoxyphenyl]methyl]piperazin-1-yl]-N-(2,2,2-trifluoroethyl)benzamide |
| EI09-54-02R | EI09-54 | 1K54 | 4-[4-[[4-(5-Hydroxypyridin-3-yl)-2-methoxyphenyl]methyl]piperazin-1-yl]-N-(2,2,2-trifluoroethyl)benzamide |
| EI09-55-02R | EI09-55 | 1K55 | 4-[4-[[3-Ethoxy-5-(3-hydroxyphenyl)phenyl]methyl]piperazin-1-yl]-N-(2,2,2-trifluoroethyl)benzamide |
| EI09-56-02R | EI09-56 | 1K56 | 4-[4-[[3-Ethoxy-5-(5-hydroxypyridin-3-yl)phenyl]methyl]piperazin-1-yl]-N-(2,2,2-trifluoroethyl)benzamide |
| EI09-57-02R | EI09-57 | 1K57 | 4-[4-[[3-(3-Hydroxyphenyl)-5-propan-2-yloxyphenyl]methyl]piperazin-1-yl]-N-(2,2,2-trifluoroethyl)benzamide |
| EI09-58-02R | EI09-58 | 1K58 | 4-[4-[[3-(5-Hydroxypyridin-3-yl)-5-propan-2-yloxyphenyl]methyl]piperazin-1-yl]-N-(2,2,2-trifluoroethyl)benzamide |
| EI09-59-02R | EI09-59 | 1K59 | 4-[4-[[4-(3-Hydroxyphenyl)-2,6-dimethoxyphenyl]methyl]piperazin-1-yl]-N-(2,2,2-trifluoroethyl)benzamide |
| EI09-60-02R | EI09-60 | 1K60 | 4-[4-[[4-(5-Hydroxypyridin-3-yl)-2,6-dimethoxyphenyl]methyl]piperazin-1-yl]-N-(2,2,2-trifluoroethyl)benzamide |
| EI09-61-02R | EI09-61 | 1K61 | 4-[4-[[3-(3-Hydroxyphenyl)-5-(trifluoromethyl)phenyl]methyl]piperazin-1-yl]-N-(2,2,2-trifluoroethyl)benzamide |
| EI09-62-02R | EI09-62 | 1K62 | 4-[4-[[3-(5-Hydroxypyridin-3-yl)-5-(trifluoromethyl)phenyl]methyl]piperazin-1-yl]-N-(2,2,2-trifluoroethyl)benzamide |
| EI09-63-02R | EI09-63 | 1K63 | 4-[4-[[3-(3-Hydroxyphenyl)-5-(trifluoromethoxy)phenyl]methyl]piperazin-1-yl]-N-(2,2,2-trifluoroethyl)benzamide |
| EI09-64-02R | EI09-64 | 1K64 | 4-[4-[[3-(5-Hydroxypyridin-3-yl)-5-(trifluoromethoxy)phenyl]methyl]piperazin-1-yl]-N-(2,2,2-trifluoroethyl)benzamide |
| EI09-65-02R | EI09-65 | 1K65 | 4-[4-[[4-(3-Hydroxyphenyl)naphthalen-1-yl]methyl]piperazin-1-yl]-N-(2,2,2-trifluoroethyl)benzamide |
| EI09-66-02R | EI09-66 | 1K66 | 4-[4-[[4-(5-Hydroxypyridin-3-yl)naphthalen-1-yl]methyl]piperazin-1-yl]-N-(2,2,2-trifluoroethyl)benzamide |
| EI09-67-02R | EI09-67 | 1K67 | 4-[4-[[4-(3-Hydroxyphenyl)thiophen-2-yl]methyl]piperazin-1-yl]-N-(2,2,2-trifluoroethyl)benzamide |
| EI09-68-02R | EI09-68 | 1K68 | 4-[4-[[4-(5-Hydroxypyridin-3-yl)thiophen-2-yl]methyl]piperazin-1-yl]-N-(2,2,2-trifluoroethyl)benzamide |
| EI09-69-02R | EI09-69 | 1K69 | 4-[4-[[2-[2-(3-Hydroxyphenyl)ethynyl]phenyl]methyl]piperazin-1-yl]-N-(2,2,2-trifluoroethyl)benzamide |
| EI09-70-02R | EI09-70 | 1K70 | 4-[4-[[2-[2-(5-Hydroxypyridin-3-yl)ethynyl]phenyl]methyl]piperazin-1-yl]-N-(2,2,2-trifluoroethyl)benzamide |

| | | | |
|---|---|---|---|
| EI09-71-02R | EI09-71 | 1K71 | 6-[4-[[2-(3-Hydroxyphenyl)phenyl]methyl]piperazin-1-yl]-N-(2,2,2-trifluoroethyl)pyridazine-3-carboxamide |
| EI09-72-02R | EI09-72 | 1K72 | 6-[4-[[2-(5-Hydroxypyridin-3-yl)phenyl]methyl]piperazin-1-yl]-N-(2,2,2-trifluoroethyl)pyridazine-3-carboxamide |
| EI09-73-02R | EI09-73 | 1K73 | 6-[4-[[4-(3-Hydroxyphenyl)-3-methylphenyl]methyl]piperazin-1-yl]-N-(2,2,2-trifluoroethyl)pyridazine-3-carboxamide |
| EI09-74-02R | EI09-74 | 1K74 | 6-[4-[[4-(5-Hydroxypyridin-3-yl)-3-methylphenyl]methyl]piperazin-1-yl]-N-(2,2,2-trifluoroethyl)pyridazine-3-carboxamide |
| EI09-75-02R | EI09-75 | 1K75 | 6-[4-[[2-Ethyl-4-(3-hydroxyphenyl)phenyl]methyl]piperazin-1-yl]-N-(2,2,2-trifluoroethyl)pyridazine-3-carboxamide |
| EI09-76-02R | EI09-76 | 1K76 | 6-[4-[[2-Ethyl-4-(5-hydroxypyridin-3-yl)phenyl]methyl]piperazin-1-yl]-N-(2,2,2-trifluoroethyl)pyridazine-3-carboxamide |
| EI09-77-02R | EI09-77 | 1K77 | 6-[4-[[3-Fluoro-4-(3-hydroxyphenyl)phenyl]methyl]piperazin-1-yl]-N-(2,2,2-trifluoroethyl)pyridazine-3-carboxamide |
| EI09-78-02R | EI09-78 | 1K78 | 6-[4-[[3-Fluoro-4-(5-hydroxypyridin-3-yl)phenyl]methyl]piperazin-1-yl]-N-(2,2,2-trifluoroethyl)pyridazine-3-carboxamide |
| EI09-79-02R | EI09-79 | 1K79 | 6-[4-[[4-(3-Hydroxyphenyl)-2-methoxyphenyl]methyl]piperazin-1-yl]-N-(2,2,2-trifluoroethyl)pyridazine-3-carboxamide |
| EI09-80-02R | EI09-80 | 1K80 | 6-[4-[[4-(5-Hydroxypyridin-3-yl)-2-methoxyphenyl]methyl]piperazin-1-yl]-N-(2,2,2-trifluoroethyl)pyridazine-3-carboxamide |
| EI09-81-02R | EI09-81 | 1K81 | 6-[4-[[3-Ethoxy-5-(3-hydroxyphenyl)phenyl]methyl]piperazin-1-yl]-N-(2,2,2-trifluoroethyl)pyridazine-3-carboxamide |
| EI09-82-02R | EI09-82 | 1K82 | 6-[4-[[3-Ethoxy-5-(5-hydroxypyridin-3-yl)phenyl]methyl]piperazin-1-yl]-N-(2,2,2-trifluoroethyl)pyridazine-3-carboxamide |
| EI09-83-02R | EI09-83 | 1K83 | 6-[4-[[3-(3-Hydroxyphenyl)-5-propan-2-yloxyphenyl]methyl]piperazin-1-yl]-N-(2,2,2-trifluoroethyl)pyridazine-3-carboxamide |
| EI09-84-02R | EI09-84 | 1K84 | 6-[4-[[3-(5-Hydroxypyridin-3-yl)-5-propan-2-yloxyphenyl]methyl]piperazin-1-yl]-N-(2,2,2-trifluoroethyl)pyridazine-3-carboxamide |
| EI09-85-02R | EI09-85 | 1K85 | 6-[4-[[4-(3-Hydroxyphenyl)-2,6-dimethoxyphenyl]methyl]piperazin-1-yl]-N-(2,2,2-trifluoroethyl)pyridazine-3-carboxamide |
| EI09-86-02R | EI09-86 | 1K86 | 6-[4-[[4-(5-Hydroxypyridin-3-yl)-2,6-dimethoxyphenyl]methyl]piperazin-1-yl]-N-(2,2,2-trifluoroethyl)pyridazine-3-carboxamide |
| EI09-87-02R | EI09-87 | 1K87 | 6-[4-[[3-(3-Hydroxyphenyl)-5-(trifluoromethyl)phenyl]methyl]piperazin-1-yl]-N-(2,2,2-trifluoroethyl)pyridazine-3-carboxamide |
| EI09-88-02R | EI09-88 | 1K88 | 6-[4-[[3-(5-Hydroxypyridin-3-yl)-5-(trifluoromethyl)phenyl]methyl]piperazin-1-yl]-N-(2,2,2-trifluoroethyl)pyridazine-3-carboxamide |
| EI09-89-02R | EI09-89 | 1K89 | 6-[4-[[3-(3-Hydroxyphenyl)-5-(trifluoromethoxy)phenyl]methyl]piperazin-1-yl]-N-(2,2,2-trifluoroethyl)pyridazine-3-carboxamide |
| EI09-90-02R | EI09-90 | 1K90 | 6-[4-[[3-(5-Hydroxypyridin-3-yl)-5-(trifluoromethoxy)phenyl]methyl]piperazin-1-yl]-N-(2,2,2-trifluoroethyl)pyridazine-3-carboxamide |
| EI09-91-02R | EI09-91 | 1K91 | 6-[4-[[4-(3-Hydroxyphenyl)naphthalen-1-yl]methyl]piperazin-1-yl]-N-(2,2,2-trifluoroethyl)pyridazine-3-carboxamide |
| EI09-92-02R | EI09-92 | 1K92 | 6-[4-[[4-(5-Hydroxypyridin-3-yl)naphthalen-1-yl]methyl]piperazin-1-yl]-N-(2,2,2-trifluoroethyl)pyridazine-3-carboxamide |
| EI09-93-02R | EI09-93 | 1K93 | 6-[4-[[4-(3-Hydroxyphenyl)thiophen-2-yl]methyl]piperazin-1-yl]-N-(2,2,2-trifluoroethyl)pyridazine-3-carboxamide |
| EI09-94-02R | EI09-94 | 1K94 | 6-[4-[[4-(5-Hydroxypyridin-3-yl)thiophen-2-yl]methyl]piperazin-1-yl]-N-(2,2,2-trifluoroethyl)pyridazine-3-carboxamide |

| | | | |
|---|---|---|---|
| EI09-95-02R | EI09-95 | 1K95 | 6-[4-[[2-[2-(3-Hydroxyphenyl)ethynyl]phenyl]methyl]piperazin-1-yl]-N-(2,2,2-trifluoroethyl)pyridazine-3-carboxamide |
| EI09-96-02R | EI09-96 | 1K96 | 6-[4-[[2-[2-(5-Hydroxypyridin-3-yl)ethynyl]phenyl]methyl]piperazin-1-yl]-N-(2,2,2-trifluoroethyl)pyridazine-3-carboxamide |
| EI09-97-02R | EI09-97 | 1K97 | 4-[4-[1-[4-(3-Hydroxyphenyl)phenyl]ethyl]piperazin-1-yl]-N-(2,2,2-trifluoroethyl)benzamide |
| EI09-98-02R | EI09-98 | 1K98 | 4-[4-[1-[4-(5-Hydroxypyridin-3-yl)phenyl]ethyl]piperazin-1-yl]-N-(2,2,2-trifluoroethyl)benzamide |
| EI09-99-02R | EI09-99 | 1K99 | 4-[4-[1-[2-Fluoro-4-(3-hydroxyphenyl)phenyl]ethyl]piperazin-1-yl]-N-(2,2,2-trifluoroethyl)benzamide |
| EI09-100-02R | EI09-100 | 1K100 | 4-[4-[1-[2-Fluoro-4-(5-hydroxypyridin-3-yl)phenyl]ethyl]piperazin-1-yl]-N-(2,2,2-trifluoroethyl)benzamide |

[Table 205]

[Table EI10] Mixture No: mixEI10-02R; Amin reagent used: BB33; Mixture No. after cleavage: mixEI10

| Resin compound No. | Compound No. after cleavage | Another name of compound after cleavage in Table EJ01 | Compound name |
|---|---|---|---|
| EI10-01-02R | EI10-01 | 1O01 | 6-[4-[4-(3-Hydroxyphenyl)thiophene-2-carbonyl]piperazin-1-yl]-N-(4-methoxyphenyl)pyridazine-3-carboxamide |
| EI10-02-02R | EI10-02 | 1O02 | 6-[4-[4-(5-Hydroxypyridin-3-yl)thiophene-2-carbonyl]piperazin-1-yl]-N-(4-methoxyphenyl)pyridazine-3-carboxamide |
| EI10-03-02R | EI10-03 | 1O03 | 6-[4-[4-(3-Hydroxyphenyl)-3-methylbenzoyl]piperazin-1-yl]-N-(4-methoxyphenyl)pyridazine-3-carboxamide |
| EI10-04-02R | EI10-04 | 1O04 | 6-[4-[4-(5-Hydroxypyridin-3-yl)-3-methylbenzoyl]piperazin-1-yl]-N-(4-methoxyphenyl)pyridazine-3-carboxamide |
| EI10-05-02R | EI10-05 | 1O05 | 6-[4-[3-Fluoro-4-(3-hydroxyphenyl)benzoyl]piperazin-1-yl]-N-(4-methoxyphenyl)pyridazine-3-carboxamide |
| EI10-06-02R | EI10-06 | 1O06 | 6-[4-[3-Fluoro-4-(5-hydroxypyridin-3-yl)benzoyl]piperazin-1-yl]-N-(4-methoxyphenyl)pyridazine-3-carboxamide |
| EI10-07-02R | EI10-07 | 1O07 | 6-[4-[2-[2-(3-Hydroxyphenyl)ethynyl]benzoyl]piperazin-1-yl]-N-(4-methoxyphenyl)pyridazine-3-carboxamide |
| EI10-08-02R | EI10-08 | 1O08 | 6-[4-[2-[2-(5-Hydroxypyridin-3-yl)ethynyl]benzoyl]piperazin-1-yl]-N-(4-methoxyphenyl)pyridazine-3-carboxamide |
| EI10-09-02R | EI10-09 | 1O09 | 6-[4-[5-[2-(3-Hydroxyphenyl)ethynyl]pyridine-3-carbonyl]piperazin-1-yl]-N-(4-methoxyphenyl)pyridazine-3-carboxamide |
| EI10-10-02R | EI10-10 | 1O10 | 6-[4-[5-[2-(5-Hydroxypyridin-3-yl)ethynyl]pyridine-3-carbonyl]piperazin-1-yl]-N-(4-methoxyphenyl)pyridazine-3-carboxamide |
| EI10-11-02R | EI10-11 | 1O11 | 6-[4-[3-[2-(3-Hydroxyphenyl)phenyl]propanoyl]piperazin-1-yl]-N-(4-methoxyphenyl)pyridazine-3-carboxamide |
| EI10-12-02R | EI10-12 | 1O12 | 6-[4-[3-[2-(5-Hydroxypyridin-3-yl)phenyl]propanoyl]piperazin-1-yl]-N-(4-methoxyphenyl)pyridazine-3-carboxamide |
| EI10-13-02R | EI10-13 | 1O13 | 6-[4-[4-(3-Hydroxyphenyl)naphthalene-1-carbonyl]piperazin-1-yl]-N-(4-methoxyphenyl)pyridazine-3-carboxamide |
| EI10-14-02R | EI10-14 | 1O14 | 6-[4-[4-(5-Hydroxypyridin-3-yl)naphthalene-1-carbonyl]piperazin-1-yl]-N-(4-methoxyphenyl)pyridazine-3-carboxamide |
| EI10-15-02R | EI10-15 | 1O15 | 6-[4-[3-tert-Butyl-5-(3-hydroxyphenyl)benzoyl]piperazin-1-yl]-N-(4-methoxyphenyl)pyridazine-3-carboxamide |

| | | | |
|---|---|---|---|
| EI10-16-02R | EI10-16 | 1O16 | 6-[4-[3-tert-Butyl-5-(5-hydroxypyridin-3-yl)benzoyl]piperazin-1-yl]-N-(4-methoxyphenyl)pyridazine-3-carboxamide |
| EI10-17-02R | EI10-17 | 1O17 | 6-[4-[3-(3-Hydroxyphenyl)-5-(trifluoromethyl)benzoyl]piperazin-1-yl]-N-(4-methoxyphenyl)pyridazine-3-carboxamide |
| EI10-18-02R | EI10-18 | 1O18 | 6-[4-[3-(5-Hydroxypyridin-3-yl)-5-(trifluoromethyl)benzoyl]piperazin-1-yl]-N-(4-methoxyphenyl)pyridazine-3-carboxamide |
| EI10-19-02R | EI10-19 | 1O19 | 6-[4-[3-[2-(3-Hydroxyphenyl)ethynyl]-5-(trifluoromethyl)benzoyl]piperazin-1-yl]-N-(4-methoxyphenyl)pyridazine-3-carboxamide |
| EI10-20-02R | EI10-20 | 1O20 | 6-[4-[3-[2-(5-Hydroxypyridin-3-yl)ethynyl]-5-(trifluoromethyl)benzoyl]piperazin-1-yl]-N-(4-methoxyphenyl)pyridazine-3-carboxamide |
| EI10-21-02R | EI10-21 | 1O21 | 6-[4-[3-[4-(3-Hydroxyphenyl)pyrazol-1-yl]-5-(trifluoromethyl)benzoyl]piperazin-1-yl]-N-(4-methoxyphenyl)pyridazine-3-carboxamide |
| EI10-22-02R | EI10-22 | 1O22 | 6-[4-[3-[4-(5-Hydroxypyridin-3-yl)pyrazol-1-yl]-5-(trifluoromethyl)benzoyl]piperazin-1-yl]-N-(4-methoxyphenyl)pyridazine-3-carboxamide |
| EI10-23-02R | EI10-23 | 1O23 | 4-[4-[4-(3-Hydroxyphenyl)thiophene-2-carbonyl]piperazin-1-yl]-N-(4-methoxyphenyl)benzamide |
| EI10-24-02R | EI10-24 | 1O24 | 4-[4-[4-(5-Hydroxypyridin-3-yl)thiophene-2-carbonyl]piperazin-1-yl]-N-(4-methoxyphenyl)benzamide |
| EI10-25-02R | EI10-25 | 1O25 | 4-[4-[4-(3-Hydroxyphenyl)-3-methylbenzoyl]piperazin-1-yl]-N-(4-methoxyphenyl)benzamide |
| EI10-26-02R | EI10-26 | 1O26 | 4-[4-[4-(5-Hydroxypyridin-3-yl)-3-methylbenzoyl]piperazin-1-yl]-N-(4-methoxyphenyl)benzamide |
| EI10-27-02R | EI10-27 | 1O27 | 4-[4-[3-Fluoro-4-(3-hydroxyphenyl)benzoyl]piperazin-1-yl]-N-(4-methoxyphenyl)benzamide |
| EI10-28-02R | EI10-28 | 1O28 | 4-[4-[3-Fluoro-4-(5-hydroxypyridin-3-yl)benzoyl]piperazin-1-yl]-N-(4-methoxyphenyl)benzamide |
| EI10-29-02R | EI10-29 | 1O29 | 4-[4-[2-[2-(3-Hydroxyphenyl)ethynyl]benzoyl]piperazin-1-yl]-N-(4-methoxyphenyl)benzamide |
| EI10-30-02R | EI10-30 | 1O30 | 4-[4-[2-[2-(5-Hydroxypyridin-3-yl)ethynyl]benzoyl]piperazin-1-yl]-N-(4-methoxyphenyl)benzamide |
| EI10-31-02R | EI10-31 | 1O31 | 4-[4-[5-[2-(3-Hydroxyphenyl)ethynyl]pyridine-3-carbonyl]piperazin-1-yl]-N-(4-methoxyphenyl)benzamide |
| EI10-32-02R | EI10-32 | 1O32 | 4-[4-[5-[2-(5-Hydroxypyridin-3-yl)ethynyl]pyridine-3-carbonyl]piperazin-1-yl]-N-(4-methoxyphenyl)benzamide |
| EI10-33-02R | EI10-33 | 1O33 | 4-[4-[3-[2-(3-Hydroxyphenyl)phenyl]propanoyl]piperazin-1-yl]-N-(4-methoxyphenyl)benzamide |
| EI10-34-02R | EI10-34 | 1O34 | 4-[4-[3-[2-(5-Hydroxypyridin-3-yl)phenyl]propanoyl]piperazin-1-yl]-N-(4-methoxyphenyl)benzamide |
| EI10-35-02R | EI10-35 | 1O35 | 4-[4-[4-(3-Hydroxyphenyl)naphthalene-1-carbonyl]piperazin-1-yl]-N-(4-methoxyphenyl)benzamide |
| EI10-36-02R | EI10-36 | 1O36 | 4-[4-[4-(5-Hydroxypyridin-3-yl)naphthalene-1-carbonyl]piperazin-1-yl]-N-(4-methoxyphenyl)benzamide |
| EI10-37-02R | EI10-37 | 1O37 | 4-[4-[3-tert-Butyl-5-(3-hydroxyphenyl)benzoyl]piperazin-1-yl]-N-(4-methoxyphenyl)benzamide |
| EI10-38-02R | EI10-38 | 1O38 | 4-[4-[3-tert-Butyl-5-(5-hydroxypyridin-3-yl)benzoyl]piperazin-1-yl]-N-(4-methoxyphenyl)benzamide |
| EI10-39-02R | EI10-39 | 1O39 | 4-[4-[3-(3-Hydroxyphenyl)-5-(trifluoromethyl)benzoyl]piperazin-1-yl]-N-(4-methoxyphenyl)benzamide |
| EI10-40-02R | EI10-40 | 1O40 | 4-[4-[3-(5-Hydroxypyridin-3-yl)-5-(trifluoromethyl)benzoyl]piperazin-1-yl]-N-(4-methoxyphenyl)benzamide |

| | | | |
|---|---|---|---|
| EI10-41-02R | EI10-41 | 1O41 | 4-[4-[3-[2-(3-Hydroxyphenyl)ethynyl]-5-(trifluoromethyl)benzoyl]piperazin-1-yl]-N-(4-methoxyphenyl)benzamide |
| EI10-42-02R | EI10-42 | 1O42 | 4-[4-[3-[2-(5-Hydroxypyridin-3-yl)ethynyl]-5-(trifluoromethyl)benzoyl]piperazin-1-yl]-N-(4-methoxyphenyl)benzamide |
| EI10-43-02R | EI10-43 | 1O43 | 4-[4-[3-[4-(3-Hydroxyphenyl)pyrazol-1-yl]-5-(trifluoromethyl)benzoyl]piperazin-1-yl]-N-(4-methoxyphenyl)benzamide |
| EI10-44-02R | EI10-44 | 1O44 | 4-[4-[3-[4-(5-Hydroxypyridin-3-yl)pyrazol-1-yl]-5-(trifluoromethyl)benzoyl]piperazin-1-yl]-N-(4-methoxyphenyl)benzamide |
| EI10-45-02R | EI10-45 | 1O45 | 4-[4-[[2-(3-Hydroxyphenyl)phenyl]methyl]piperazin-1-yl]-N-(4-methoxyphenyl)benzamide |
| EI10-46-02R | EI10-46 | 1O46 | 4-[4-[[2-(5-Hydroxypyridin-3-yl)phenyl]methyl]piperazin-1-yl]-N-(4-methoxyphenyl)benzamide |
| EI10-47-02R | EI10-47 | 1O47 | 4-[4-[[4-(3-Hydroxyphenyl)-3-methylphenyl]methyl]piperazin-1-yl]-N-(4-methoxyphenyl)benzamide |
| EI10-48-02R | EI10-48 | 1O48 | 4-[4-[[4-(5-Hydroxypyridin-3-yl)-3-methylphenyl]methyl]piperazin-1-yl]-N-(4-methoxyphenyl)benzamide |
| EI10-49-02R | EI10-49 | 1O49 | 4-[4-[[2-Ethyl-4-(3-hydroxyphenyl)phenyl]methyl]piperazin-1-yl]-N-(4-methoxyphenyl)benzamide |
| EI10-50-02R | EI10-50 | 1O50 | 4-[4-[[2-Ethyl-4-(5-hydroxypyridin-3-yl)phenyl]methyl]piperazin-1-yl]-N-(4-methoxyphenyl)benzamide |
| EI10-51-02R | EI10-51 | 1O51 | 4-[4-[[3-Fluoro-4-(3-hydroxyphenyl)phenyl]methyl]piperazin-1-yl]-N-(4-methoxyphenyl)benzamide |
| EI10-52-02R | EI10-52 | 1O52 | 4-[4-[[3-Fluoro-4-(5-hydroxypyridin-3-yl)phenyl]methyl]piperazin-1-yl]-N-(4-methoxyphenyl)benzamide |
| EI10-53-02R | EI10-53 | 1O53 | 4-[4-[[4-(3-Hydroxyphenyl)-2-methoxyphenyl]methyl]piperazin-1-yl]-N-(4-methoxyphenyl)benzamide |
| EI10-54-02R | EI10-54 | 1O54 | 4-[4-[[4-(5-Hydroxypyridin-3-yl)-2-methoxyphenyl]methyl]piperazin-1-yl]-N-(4-methoxyphenyl)benzamide |
| EI10-55-02R | EI10-55 | 1O55 | 4-[4-[[3-Ethoxy-5-(3-hydroxyphenyl)phenyl]methyl]piperazin-1-yl]-N-(4-methoxyphenyl)benzamide |
| EI10-56-02R | EI10-56 | 1O56 | 4-[4-[[3-Ethoxy-5-(5-Hydroxypyridin-3-yl)phenyl]methyl]piperazin-1-yl]-N-(4-methoxyphenyl)benzamide |
| EI10-57-02R | EI10-57 | 1O57 | 4-[4-[[3-(3-Hydroxyphenyl)-5-propan-2-yloxyphenyl]methyl]piperazin-1-yl]-N-(4-methoxyphenyl)benzamide |
| EI10-58-02R | EI10-58 | 1O58 | 4-[4-[[3-(5-Hydroxypyridin-3-yl)-5-propan-2-yloxyphenyl]methyl]piperazin-1-yl]-N-(4-methoxyphenyl)benzamide |
| EI10-59-02R | EI10-59 | 1O59 | 4-[4-[[4-(3-Hydroxyphenyl)-2,6-dimethoxyphenyl]methyl]piperazin-1-yl]-N-(4-methoxyphenyl)benzamide |
| EI10-60-02R | EI10-60 | 1O60 | 4-[4-[[4-(5-Hydroxypyridin-3-yl)-2,6-dimethoxyphenyl]methyl]piperazin-1-yl]-N-(4-methoxyphenyl)benzamide |
| EI10-61-02R | EI10-61 | 1O61 | 4-[4-[[3-(3-Hydroxyphenyl)-5-(trifluoromethyl)phenyl]methyl]piperazin-1-yl]-N-(4-methoxyphenyl)benzamide |
| EI10-62-02R | EI10-62 | 1O62 | 4-[4-[[3-(5-Hydroxypyridin-3-yl)-5-(trifluoromethyl)phenyl]methyl]piperazin-1-yl]-N-(4-methoxyphenyl)benzamide |
| EI10-63-02R | EI10-63 | 1O63 | 4-[4-[[3-(3-Hydroxyphenyl)-5-(trifluoromethoxy)phenyl]methyl]piperazin-1-yl]-N-(4-methoxyphenyl)benzamide |

| | | | |
|---|---|---|---|
| EI10-64-02R | EI10-64 | 1O64 | 4-[4-[[3-(5-Hydroxypyridin-3-yl)-5-(trifluoromethoxy)phenyl]methyl]piperazin-1-yl]-N-(4-methoxyphenyl)benzamide |
| EI10-65-02R | EI10-65 | 1O65 | 4-[4-[[4-(3-Hydroxyphenyl)naphthalen-1-yl]methyl]piperazin-1-yl]-N-(4-methoxyphenyl)benzamide |
| EI10-66-02R | EI10-66 | 1O66 | 4-[4-[[4-(5-Hydroxypyridin-3-yl)naphthalen-1-yl]methyl]piperazin-1-yl]-N-(4-methoxyphenyl)benzamide |
| EI10-67-02R | EI10-67 | 1O67 | 4-[4-[[4-(3-Hydroxyphenyl)thiophen-2-yl]methyl]piperazin-1-yl]-N-(4-methoxyphenyl)benzamide |
| EI10-68-02R | EI10-68 | 1O68 | 4-[4-[[4-(5-Hydroxypyridin-3-yl)thiophen-2-yl]methyl]piperazin-1-yl]-N-(4-methoxyphenyl)benzamide |
| EI10-69-02R | EI10-69 | 1O69 | 4-[4-[[2-[2-(3-Hydroxyphenyl)ethynyl]phenyl]methyl]piperazin-1-yl]-N-(4-methoxyphenyl)benzamide |
| EI10-70-02R | EI10-70 | 1O70 | 4-[4-[[2-[2-(5-Hydroxypyridin-3-yl)ethynyl]phenyl]methyl]piperazin-1-yl]-N-(4-methoxyphenyl)benzamide |
| EI10-71-02R | EI10-71 | 1O71 | 6-[4-[[2-(3-Hydroxyphenyl)phenyl]methyl]piperazin-1-yl]-N-(4-methoxyphenyl)pyridazine-3-carboxamide |
| EI10-72-02R | EI10-72 | 1O72 | 6-[4-[[2-(5-Hydroxypyridin-3-yl)phenyl]methyl]piperazin-1-yl]-N-(4-methoxyphenyl)pyridazine-3-carboxamide |
| EI10-73-02R | EI10-73 | 1O73 | 6-[4-[[4-(3-Hydroxyphenyl)-3-methylphenyl]methyl]piperazin-1-yl]-N-(4-methoxyphenyl)pyridazine-3-carboxamide |
| EI10-74-02R | EI10-74 | 1O74 | 6-[4-[[4-(5-Hydroxypyridin-3-yl)-3-methylphenyl]methyl]piperazin-1-yl]-N-(4-methoxyphenyl)pyridazine-3-carboxamide |
| EI10-75-02R | EI10-75 | 1O75 | 6-[4-[[2-Ethyl-4-(3-hydroxyphenyl)phenyl]methyl]piperazin-1-yl]-N-(4-methoxyphenyl)pyridazine-3-carboxamide |
| EI10-76-02R | EI10-76 | 1O76 | 6-[4-[[2-Ethyl-4-(5-hydroxypyridin-3-yl)phenyl]methyl]piperazin-1-yl]-N-(4-methoxyphenyl)pyridazine-3-carboxamide |
| EI10-77-02R | EI10-77 | 1O77 | 6-[4-[[3-Fluoro-4-(3-hydroxyphenyl)phenyl]methyl]piperazin-1-yl]-N-(4-methoxyphenyl)pyridazine-3-carboxamide |
| EI10-78-02R | EI10-78 | 1O78 | 6-[4-[[3-Fluoro-4-(5-hydroxypyridin-3-yl)phenyl]methyl]piperazin-1-yl]-N-(4-methoxyphenyl)pyridazine-3-carboxamide |
| EI10-79-02R | EI10-79 | 1O79 | 6-[4-[[4-(3-Hydroxyphenyl)-2-methoxyphenyl]methyl]piperazin-1-yl]-N-(4-methoxyphenyl)pyridazine-3-carboxamide |
| EI10-80-02R | EI10-80 | 1O80 | 6-[4-[[4-(5-Hydroxypyridin-3-yl)-2-methoxyphenyl]methyl]piperazin-1-yl]-N-(4-methoxyphenyl)pyridazine-3-carboxamide |
| EI10-81-02R | EI10-81 | 1O81 | 6-[4-[[3-Ethoxy-5-(3-hydroxyphenyl)phenyl]methyl]piperazin-1-yl]-N-(4-methoxyphenyl)pyridazine-3-carboxamide |
| EI10-82-02R | EI10-82 | 1O82 | 6-[4-[[3-Ethoxy-5-(5-hydroxypyridin-3-yl)phenyl]methyl]piperazin-1-yl]-N-(4-methoxyphenyl)pyridazine-3-carboxamide |
| EI10-83-02R | EI10-83 | 1O83 | 6-[4-[[3-(3-Hydroxyphenyl)-5-propan-2-yloxyphenyl]methyl]piperazin-1-yl]-N-(4-methoxyphenyl)pyridazine-3-carboxamide |
| EI10-84-02R | EI10-84 | 1O84 | 6-[4-[[3-(5-Hydroxypyridin-3-yl)-5-propan-2-yloxyphenyl]methyl]piperazin-1-yl]-N-(4-methoxyphenyl)pyridazine-3-carboxamide |
| EI10-85-02R | EI10-85 | 1O85 | 6-[4-[[4-(3-Hydroxyphenyl)-2,6-dimethoxyphenyl]methyl]piperazin-1-yl]-N-(4-methoxyphenyl)pyridazine-3-carboxamide |
| EI10-86-02R | EI10-86 | 1O86 | 6-[4-[[4-(5-Hydroxypyridin-3-yl)-2,6-dimethoxyphenyl]methyl]piperazin-1-yl]-N-(4-methoxyphenyl)pyridazine-3-carboxamide |
| EI10-87-02R | EI10-87 | 1O87 | 6-[4-[[3-(3-Hydroxyphenyl)-5-(trifluoromethyl)phenyl]methyl]piperazin-1-yl]-N-(4-methoxyphenyl)pyridazine-3-carboxamide |

| | | | |
|---|---|---|---|
| EI10-88-02R | EI10-88 | 1O88 | 6-[4-[[3-(5-Hydroxypyridin-3-yl)-5-(trifluoromethyl)phenyl]methyl]piperazin-1-yl]-N-(4-methoxyphenyl)pyridazine-3-carboxamide |
| EI10-89-02R | EI10-89 | 1O89 | 6-[4-[[3-(3-Hydroxyphenyl)-5-(trifluoromethoxy)phenyl]methyl]piperazin-1-yl]-N-(4-methoxyphenyl)pyridazine-3-carboxamide |
| EI10-90-02R | EI10-90 | 1O90 | 6-[4-[[3-(5-Hydroxypyridin-3-yl)-5-(trifluoromethoxy)phenyl]methyl]piperazin-1-yl]-N-(4-methoxyphenyl)pyridazine-3-carboxamide |
| EI10-91-02R | EI10-91 | 1O91 | 6-[4-[[4-(3-Hydroxyphenyl)naphthalen-1-yl]methyl]piperazin-1-yl]-N-(4-methoxyphenyl)pyridazine-3-carboxamide |
| EI10-92-02R | EI10-92 | 1O92 | 6-[4-[[4-(5-Hydroxypyridin-3-yl)naphthalen-1-yl]methyl]piperazin-1-yl]-N-(4-methoxyphenyl)pyridazine-3-carboxamide |
| EI10-93-02R | EI10-93 | 1O93 | 6-[4-[[4-(3-Hydroxyphenyl)thiophen-2-yl]methyl]piperazin-1-yl]-N-(4-methoxyphenyl)pyridazine-3-carboxamide |
| EI10-94-02R | EI10-94 | 1O94 | 6-[4-[[4-(5-Hydroxypyridin-3-yl)thiophen-2-yl]methyl]piperazin-1-yl]-N-(4-methoxyphenyl)pyridazine-3-carboxamide |
| EI10-95-02R | EI10-95 | 1O95 | 6-[4-[[2-[2-(3-Hydroxyphenyl)ethynyl]phenyl]methyl]piperazin-1-yl]-N-(4-methoxyphenyl)pyridazine-3-carboxamide |
| EI10-96-02R | EI10-96 | 1O96 | 6-[4-[[2-[2-(5-Hydroxypyridin-3-yl)ethynyl]phenyl]methyl]piperazin-1-yl]-N-(4-methoxyphenyl)pyridazine-3-carboxamide |
| EI10-97-02R | EI10-97 | 1O97 | 4-[4-[1-[4-(3-Hydroxyphenyl)phenyl]ethyl]piperazin-1-yl]-N-(4-methoxyphenyl)benzamide |
| EI10-98-02R | EI10-98 | 1O98 | 4-[4-[1-[4-(5-Hydroxypyridin-3-yl)phenyl]ethyl]piperazin-1-yl]-N-(4-methoxyphenyl)benzamide |
| EI10-99-02R | EI10-99 | 1O99 | 4-[4-[1-[2-Fluoro-4-(3-hydroxyphenyl)phenyl]ethyl]piperazin-1-yl]-N-(4-methoxyphenyl)benzamide |
| EI10-100-02R | EI10-100 | 1O100 | 4-[4-[1-[2-Fluoro-4-(5-hydroxypyridin-3-yl)phenyl]ethyl]piperazin-1-yl]-N-(4-methoxyphenyl)benzamide |

Example 4-3-9: Synthesis of mixture mixEII 1-02R

[Formula 543]

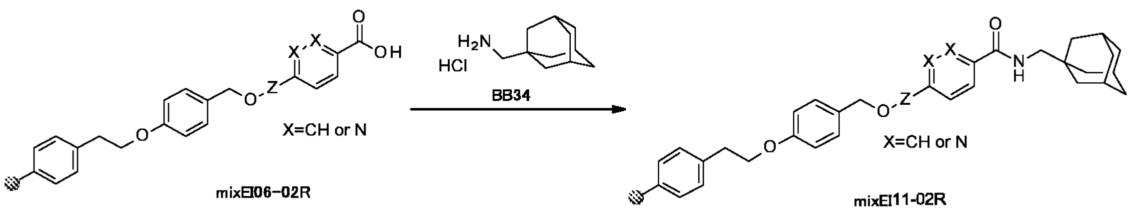


Mixture mixEI06-02R (50 mg, loading rate: 0.648 mmol/g, 0.0324 mmol) shown in Table EI06 and NMP (1.28 mL) were added to a 2 mL glass vial and shaken at room temperature for 1 hour. 1-Adamantylmethanamine hydrochloride (BB34, 19.6 mg, 97 μmol), DIC (30.3 μL, 0.194 mmol), HOAt (26.5 mg, 0.194 mmol), and DIPEA (17 μL, 97 μmol) were added thereto, and the mixture was shaken at 40°C for 22 hours. The reaction solution and the resin suspension were transferred to a syringe with a filter using a NMP solution. The resultant was repetitively washed three times with NMP (1.2 mL), three times with MeOH (1.2 mL), three times with DCM (1.2 mL), and three times with heptane (1.2 mL), and the resin was then dried under reduced pressure to obtain the title mixture shown in Table EI11. Compounds contained in the obtained mixture are shown in Table EI11.

[Table 206]

[Table EI11] Mixture No: mixEI11-02R; Amin reagent used: BB34; Mixture No. after cleavage: mixEI11

| Resin compound No. | Compound No. after cleavage | Another name of compound after cleavage in Table EJ05 | Compound name |
|---|---|---|---|
| EI11-01-02R | EI11-01 | 1A01 | N-(1-Adamantylmethyl)-6-[4-[4-(3-hydroxyphenyl)thiophene-2-carbonyl]piperazin-1-yl]pyridazine-3-carboxamide |
| EI11-02-02R | EI11-02 | 1A02 | N-(1-Adamantylmethyl)-6-[4-[4-(5-hydroxypyridin-3-yl)thiophene-2-carbonyl]piperazin-1-yl]pyridazine-3-carboxamide |
| EI11-03-02R | EI11-03 | 1A03 | N-(1-Adamantylmethyl)-6-[4-[4-(3-hydroxyphenyl)-3-methylbenzoyl]piperazin-1-yl]pyridazine-3-carboxamide |
| EI11-04-02R | EI11-04 | 1A04 | N-(1-Adamantylmethyl)-6-[4-[4-(5-hydroxypyridin-3-yl)-3-methylbenzoyl]piperazin-1-yl]pyridazine-3-carboxamide |
| EI11-05-02R | EI11-05 | 1A05 | N-(1-Adamantylmethyl)-6-[4-[3-fluoro-4-(3-hydroxyphenyl)benzoyl]piperazin-1-yl]pyridazine-3-carboxamide |
| EI11-06-02R | EI11-06 | 1A06 | N-(1-Adamantylmethyl)-6-[4-[3-fluoro-4-(5-hydroxypyridin-3-yl)benzoyl]piperazin-1-yl]pyridazine-3-carboxamide |
| EI11-07-02R | EI11-07 | 1A07 | N-(1-Adamantylmethyl)-6-[4-[2-[2-(3-hydroxyphenyl)ethynyl]benzoyl]piperazin-1-yl]pyridazine-3-carboxamide |
| EI11-08-02R | EI11-08 | 1A08 | N-(1-Adamantylmethyl)-6-[4-[2-[2-(5-hydroxypyridin-3-yl)ethynyl]benzoyl]piperazin-1-yl]pyridazine-3-carboxamide |
| EI11-09-02R | EI11-09 | 1A09 | N-(1-Adamantylmethyl)-6-[4-[5-[2-(3-hydroxyphenyl)ethynyl]pyridine-3-carbonyl]piperazin-1-yl]pyridazine-3-carboxamide |
| EI11-10-02R | EI11-10 | 1A10 | N-(1-Adamantylmethyl)-6-[4-[5-[2-(5-hydroxypyridin-3-yl)ethynyl]pyridine-3-carbonyl]piperazin-1-yl]pyridazine-3-carboxamide |
| EI11-11-02R | EI11-11 | 1A11 | N-(1-Adamantylmethyl)-6-[4-[3-[2-(3-hydroxyphenyl)phenyl]propanoyl]piperazin-1-yl]pyridazine-3-carboxamide |
| EI11-12-02R | EI11-12 | 1A12 | N-(1-Adamantylmethyl)-6-[4-[3-[2-(5-hydroxypyridin-3-yl)phenyl]propanoyl]piperazin-1-yl]pyridazine-3-carboxamide |
| EI11-13-02R | EI11-13 | 1A13 | N-(1-Adamantylmethyl)-6-[4-[4-(3-hydroxyphenyl)naphthalene-1-carbonyl]piperazin-1-yl]pyridazine-3-carboxamide |
| EI11-14-02R | EI11-14 | 1A14 | N-(1-Adamantylmethyl)-6-[4-[4-(5-hydroxypyridin-3-yl)naphthalene-1-carbonyl]piperazin-1-yl]pyridazine-3-carboxamide |
| EI11-15-02R | EI11-15 | 1A15 | N-(1-Adamantylmethyl)-6-[4-[3-tert-butyl-5-(3-hydroxyphenyl)benzoyl]piperazin-1-yl]pyridazine-3-carboxamide |
| EI11-16-02R | EI11-16 | 1A16 | N-(1-Adamantylmethyl)-6-[4-[3-tert-butyl-5-(5-hydroxypyridin-3-yl)benzoyl]piperazin-1-yl]pyridazine-3-carboxamide |
| EI11-17-02R | EI11-17 | 1A17 | N-(1-Adamantylmethyl)-6-[4-[3-(3-hydroxyphenyl)-5-(trifluoromethyl)benzoyl]piperazin-1-yl]pyridazine-3-carboxamide |
| EI11-18-02R | EI11-18 | 1A18 | N-(1-Adamantylmethyl)-6-[4-[3-(5-hydroxypyridin-3-yl)-5-(trifluoromethyl)benzoyl]piperazin-1-yl]pyridazine-3-carboxamide |

| | | | |
|---|---|---|---|
| EI11-19-02R | EI11-19 | 1A19 | N-(1-Adamantylmethyl)-6-[4-[3-[2-(3-hydroxyphenyl)ethynyl]-5-(trifluoromethyl)benzoyl]piperazin-1-yl]pyridazine-3-carboxamide |
| EI11-20-02R | EI11-20 | 1A20 | N-(1-Adamantylmethyl)-6-[4-[3-[2-(5-hydroxypyridin-3-yl)ethynyl]-5-(trifluoromethyl)benzoyl]piperazin-1-yl]pyridazine-3-carboxamide |
| EI11-21-02R | EI11-21 | 1A21 | N-(1-Adamantylmethyl)-6-[4-[3-[4-(3-hydroxyphenyl)pyrazol-1-yl]-5-(trifluoromethyl)benzoyl]piperazin-1-yl]pyridazine-3-carboxamide |
| EI11-22-02R | EI11-22 | 1A22 | N-(1-Adamantylmethyl)-6-[4-[3-[4-(5-hydroxypyridin-3-yl)pyrazol-1-yl]-5-(trifluoromethyl)benzoyl]piperazin-1-yl]pyridazine-3-carboxamide |
| EI11-23-02R | EI11-23 | 1A23 | N-(1-Adamantylmethyl)-4-[4-[4-(3-hydroxyphenyl)thiophene-2-carbonyl]piperazin-1-yl]benzamide |
| EI11-24-02R | EI11-24 | 1A24 | N-(1-Adamantylmethyl)-4-[4-[4-(5-hydroxypyridin-3-yl)thiophene-2-carbonyl]piperazin-1-yl]benzamide |
| EI11-25-02R | EI11-25 | 1A25 | N-(1-Adamantylmethyl)-4-[4-[4-(3-hydroxyphenyl)-3-methylbenzoyl]piperazin-1-yl]benzamide |
| EI11-26-02R | EI11-26 | 1A26 | N-(1-Adamantylmethyl)-4-[4-[4-(5-hydroxypyridin-3-yl)-3-methylbenzoyl]piperazin-1-yl]benzamide |
| EI11-27-02R | EI11-27 | 1A27 | N-(1-Adamantylmethyl)-4-[4-[3-fluoro-4-(3-hydroxyphenyl)benzoyl]piperazin-1-yl]benzamide |
| EI11-28-02R | EI11-28 | 1A28 | N-(1-Adamantylmethyl)-4-[4-[3-fluoro-4-(5-hydroxypyridin-3-yl)benzoyl]piperazin-1-yl]benzamide |
| EI11-29-02R | EI11-29 | 1A29 | N-(1-Adamantylmethyl)-4-[4-[2-[2-(3-hydroxyphenyl)ethynyl]benzoyl]piperazin-1-yl]benzamide |
| EI11-30-02R | EI11-30 | 1A30 | N-(1-Adamantylmethyl)-4-[4-[2-[2-(5-hydroxypyridin-3-yl)ethynyl]benzoyl]piperazin-1-yl]benzamide |
| EI11-31-02R | EI11-31 | 1A31 | N-(1-Adamantylmethyl)-4-[4-[5-[2-(3-hydroxyphenyl)ethynyl]pyridine-3-carbonyl]piperazin-1-yl]benzamide |
| EI11-32-02R | EI11-32 | 1A32 | N-(1-Adamantylmethyl)-4-[4-[5-[2-(5-hydroxypyridin-3-yl)ethynyl]pyridine-3-carbonyl]piperazin-1-yl]benzamide |
| EI11-33-02R | EI11-33 | 1A33 | N-(1-Adamantylmethyl)-4-[4-[3-[2-(3-hydroxyphenyl)phenyl]propanoyl]piperazin-1-yl]benzamide |
| EI11-34-02R | EI11-34 | 1A34 | N-(1-Adamantylmethyl)-4-[4-[3-[2-(5-hydroxypyridin-3-yl)phenyl]propanoyl]piperazin-1-yl]benzamide |
| EI11-35-02R | EI11-35 | 1A35 | N-(1-Adamantylmethyl)-4-[4-[4-(3-hydroxyphenyl)naphthalene-1-carbonyl]piperazin-1-yl]benzamide |
| EI11-36-02R | EI11-36 | 1A36 | N-(1-Adamantylmethyl)-4-[4-[4-(5-hydroxypyridin-3-yl)naphthalene-1-carbonyl]piperazin-1-yl]benzamide |
| EI11-37-02R | EI11-37 | 1A37 | N-(1-Adamantylmethyl)-4-[4-[3-tert-butyl-5-(3-hydroxyphenyl)benzoyl]piperazin-1-yl]benzamide |
| EI11-38-02R | EI11-38 | 1A38 | N-(1-Adamantylmethyl)-4-[4-[3-tert-butyl-5-(5-hydroxypyridin-3-yl)benzoyl]piperazin-1-yl]benzamide |
| EI11-39-02R | EI11-39 | 1A39 | N-(1-Adamantylmethyl)-4-[4-[3-(3-hydroxyphenyl)-5-(trifluoromethyl)benzoyl]piperazin-1-yl]benzamide |
| EI11-40-02R | EI11-40 | 1A40 | N-(1-Adamantylmethyl)-4-[4-[3-(5-hydroxypyridin-3-yl)-5-(trifluoromethyl)benzoyl]piperazin-1-yl]benzamide |
| EI11-41-02R | EI11-41 | 1A41 | N-(1-Adamantylmethyl)-4-[4-[3-[2-(3-hydroxyphenyl)ethynyl]-5-(trifluoromethyl)benzoyl]piperazin-1-yl]benzamide |
| EI11-42-02R | EI11-42 | 1A42 | N-(1-Adamantylmethyl)-4-[4-[3-[2-(5-hydroxypyridin-3-yl)ethynyl]-5-(trifluoromethyl)benzoyl]piperazin-1-yl]benzamide |
| EI11-43-02R | EI11-43 | 1A43 | N-(1-Adamantylmethyl)-4-[4-[3-[4-(3-hydroxyphenyl)pyrazol-1-yl]-5-(trifluoromethyl)benzoyl]piperazin-1-yl]benzamide |
| EI11-44-02R | EI11-44 | 1A44 | N-(1-Adamantylmethyl)-4-[4-[3-[4-(5-hydroxypyridin-3-yl)pyrazol-1-yl]-5-(trifluoromethyl)benzoyl]piperazin-1-yl]benzamide |
| EI11-45-02R | EI11-45 | 1A45 | N-(1-Adamantylmethyl)-4-[4-[[2-(3-hydroxyphenyl)phenyl]methyl]piperazin-1-yl]benzamide |

| | | | |
|---|---|---|---|
| EI11-46-02R | EI11-46 | 1A46 | N-(1-Adamantylmethyl)-4-[4-[[2-(5-hydroxypyridin-3-yl)phenyl]methyl]piperazin-1-yl]benzamide |
| EI11-47-02R | EI11-47 | 1A47 | N-(1-Adamantylmethyl)-4-[4-[[4-(3-hydroxyphenyl)-3-methylphenyl]methyl]piperazin-1-yl]benzamide |
| EI11-48-02R | EI11-48 | 1A48 | N-(1-Adamantylmethyl)-4-[4-[[4-(5-hydroxypyridin-3-yl)-3-methylphenyl]methyl]piperazin-1-yl]benzamide |
| EI11-49-02R | EI11-49 | 1A49 | N-(1-Adamantylmethyl)-4-[4-[[2-ethyl-4-(3-hydroxyphenyl)phenyl]methyl]piperazin-1-yl]benzamide |
| EI11-50-02R | EI11-50 | 1A50 | N-(1-Adamantylmethyl)-4-[4-[[2-ethyl-4-(5-hydroxypyridin-3-yl)phenyl]methyl]piperazin-1-yl]benzamide |
| EI11-51-02R | EI11-51 | 1A51 | N-(1-Adamantylmethyl)-4-[4-[[3-fluoro-4-(3-hydroxyphenyl)phenyl]methyl]piperazin-1-yl]benzamide |
| EI11-52-02R | EI11-52 | 1A52 | N-(1-Adamantylmethyl)-4-[4-[[3-fluoro-4-(5-hydroxypyridin-3-yl)phenyl]methyl]piperazin-1-yl]benzamide |
| EI11-53-02R | EI11-53 | 1A53 | N-(1-Adamantylmethyl)-4-[4-[[4-(3-hydroxyphenyl)-2-methoxyphenyl]methyl]piperazin-1-yl]benzamide |
| EI11-54-02R | EI11-54 | 1A54 | N-(1-Adamantylmethyl)-4-[4-[[4-(5-hydroxypyridin-3-yl)-2-methoxyphenyl]methyl]piperazin-1-yl]benzamide |
| EI11-55-02R | EI11-55 | 1A55 | N-(1-Adamantylmethyl)-4-[4-[[3-ethoxy-5-(3-hydroxyphenyl)phenyl]methyl]piperazin-1-yl]benzamide |
| EI11-56-02R | EI11-56 | 1A56 | N-(1-Adamantylmethyl)-4-[4-[[3-ethoxy-5-(5-hydroxypyridin-3-yl)phenyl]methyl]piperazin-1-yl]benzamide |
| EI11-57-02R | EI11-57 | 1A57 | N-(1-Adamantylmethyl)-4-[4-[[3-(3-hydroxyphenyl)-5-propan-2-yloxyphenyl]methyl]piperazin-1-yl]benzamide |
| EI11-58-02R | EI11-58 | 1A58 | N-(1-Adamantylmethyl)-4-[4-[[3-(5-hydroxypyridin-3-yl)-5-propan-2-yloxyphenyl]methyl]piperazin-1-yl]benzamide |
| EI11-59-02R | EI11-59 | 1A59 | N-(1-Adamantylmethyl)-4-[4-[[4-(3-hydroxyphenyl)-2,6-dimethoxyphenyl]methyl]piperazin-1-yl]benzamide |
| EI11-60-02R | EI11-60 | 1A60 | N-(1-Adamantylmethyl)-4-[4-[[4-(5-hydroxypyridin-3-yl)-2,6-dimethoxyphenyl]methyl]piperazin-1-yl]benzamide |
| EI11-61-02R | EI11-61 | 1A61 | N-(1-Adamantylmethyl)-4-[4-[[3-(3-hydroxyphenyl)-5-(trifluoromethyl)phenyl]methyl]piperazin-1-yl]benzamide |
| EI11-62-02R | EI11-62 | 1A62 | N-(1-Adamantylmethyl)-4-[4-[[3-(5-hydroxypyridin-3-yl)-5-(trifluoromethyl)phenyl]methyl]piperazin-1-yl]benzamide |
| EI11-63-02R | EI11-63 | 1A63 | N-(1-Adamantylmethyl)-4-[4-[[3-(3-hydroxyphenyl)-5-(trifluoromethoxy)phenyl]methyl]piperazin-1-yl]benzamide |
| EI11-64-02R | EI11-64 | 1A64 | N-(1-Adamantylmethyl)-4-[4-[[3-(5-hydroxypyridin-3-yl)-5-(trifluoromethoxy)phenyl]methyl]piperazin-1-yl]benzamide |
| EI11-65-02R | EI11-65 | 1A65 | N-(1-Adamantylmethyl)-4-[4-[[4-(3-hydroxyphenyl)naphthalen-1-yl]methyl]piperazin-1-yl]benzamide |
| EI11-66-02R | EI11-66 | 1A66 | N-(1-Adamantylmethyl)-4-[4-[[4-(5-hydroxypyridin-3-yl)naphthalen-1-yl]methyl]piperazin-1-yl]benzamide |
| EI11-67-02R | EI11-67 | 1A67 | N-(1-Adamantylmethyl)-4-[4-[[4-(3-hydroxyphenyl)thiophen-2-yl]methyl]piperazin-1-yl]benzamide |
| EI11-68-02R | EI11-68 | 1A68 | N-(1-Adamantylmethyl)-4-[4-[[4-(5-hydroxypyridin-3-yl)thiophen-2-yl]methyl]piperazin-1-yl]benzamide |
| EI11-69-02R | EI11-69 | 1A69 | N-(1-Adamantylmethyl)-4-[4-[[2-[2-(3-hydroxyphenyl)ethynyl]phenyl]methyl]piperazin-1-yl]benzamide |
| EI11-70-02R | EI11-70 | 1A70 | N-(1-Adamantylmethyl)-4-[4-[[2-[2-(5-hydroxypyridin-3-yl)ethynyl]phenyl]methyl]piperazin-1-yl]benzamide |
| EI11-71-02R | EI11-71 | 1A71 | N-(1-Adamantylmethyl)-6-[4-[[2-(3-hydroxyphenyl)phenyl]methyl]piperazin-1-yl]pyridazine-3-carboxamide |
| EI11-72-02R | EI11-72 | 1A72 | N-(1-Adamantylmethyl)-6-[4-[[2-(5-hydroxypyridin-3-yl)phenyl]methyl]piperazin-1-yl]pyridazine-3-carboxamide |
| EI11-73-02R | EI11-73 | 1A73 | N-(1-Adamantylmethyl)-6-[4-[[4-(3-hydroxyphenyl)-3-methylphenyl]methyl]piperazin-1-yl]pyridazine-3-carboxamide |

| | | | |
|---|---|---|---|
| EI11-74-02R | EI11-74 | 1A74 | N-(1-Adamantylmethyl)-6-[4-[[4-(5-hydroxypyridin-3-yl)-3-methylphenyl]methyl]piperazin-1-yl]pyridazine-3-carboxamide |
| EI11-75-02R | EI11-75 | 1A75 | N-(1-Adamantylmethyl)-6-[4-[[2-ethyl-4-(3-hydroxyphenyl)phenyl]methyl]piperazin-1-yl]pyridazine-3-carboxamide |
| EI11-76-02R | EI11-76 | 1A76 | N-(1-Adamantylmethyl)-6-[4-[[2-ethyl-4-(5-hydroxypyridin-3-yl)phenyl]methyl]piperazin-1-yl]pyridazine-3-carboxamide |
| EI11-77-02R | EI11-77 | 1A77 | N-(1-Adamantylmethyl)-6-[4-[[3-fluoro-4-(3-hydroxyphenyl)phenyl]methyl]piperazin-1-yl]pyridazine-3-carboxamide |
| EI11-78-02R | EI11-78 | 1A78 | N-(1-Adamantylmethyl)-6-[4-[[3-fluoro-4-(5-hydroxypyridin-3-yl)phenyl]methyl]piperazin-1-yl]pyridazine-3-carboxamide |
| EI11-79-02R | EI11-79 | 1A79 | N-(1-Adamantylmethyl)-6-[4-[[4-(3-hydroxyphenyl)-2-methoxyphenyl]methyl]piperazin-1-yl]pyridazine-3-carboxamide |
| EI11-80-02R | EI11-80 | 1A80 | N-(1-Adamantylmethyl)-6-[4-[[4-(5-hydroxypyridin-3-yl)-2-methoxyphenyl]methyl]piperazin-1-yl]pyridazine-3-carboxamide |
| EI11-81-02R | EI11-81 | 1A81 | N-(1-Adamantylmethyl)-6-[4-[[3-ethoxy-5-(3-hydroxyphenyl)phenyl]methyl]piperazin-1-yl]pyridazine-3-carboxamide |
| EI11-82-02R | EI11-82 | 1A82 | N-(1-Adamantylmethyl)-6-[4-[[3-ethoxy-5-(5-hydroxypyridin-3-yl)phenyl]methyl]piperazin-1-yl]pyridazine-3-carboxamide |
| EI11-83-02R | EI11-83 | 1A83 | N-(1-Adamantylmethyl)-6-[4-[[3-(3-hydroxyphenyl)-5-propan-2-yloxyphenyl]methyl]piperazin-1-yl]pyridazine-3-carboxamide |
| EI11-84-02R | EI11-84 | 1A84 | N-(1-Adamantylmethyl)-6-[4-[[3-(5-hydroxypyridin-3-yl)-5-propan-2-yloxyphenyl]methyl]piperazin-1-yl]pyridazine-3-carboxamide |
| EI11-85-02R | EI11-85 | 1A85 | N-(1-Adamantylmethyl)-6-[4-[[4-(3-hydroxyphenyl)-2,6-dimethoxyphenyl]methyl]piperazin-1-yl]pyridazine-3-carboxamide |
| EI11-86-02R | EI11-86 | 1A86 | N-(1-Adamantylmethyl)-6-[4-[[4-(5-hydroxypyridin-3-yl)-2,6-dimethoxyphenyl]methyl]piperazin-1-yl]pyridazine-3-carboxamide |
| EI11-87-02R | EI11-87 | 1A87 | N-(1-Adamantylmethyl)-6-[4-[[3-(3-hydroxyphenyl)-5-(trifluoromethyl)phenyl]methyl]piperazin-1-yl]pyridazine-3-carboxamide |
| EI11-88-02R | EI11-88 | 1A88 | N-(1-Adamantylmethyl)-6-[4-[[3-(5-hydroxypyridin-3-yl)-5-(trifluoromethyl)phenyl]methyl]piperazin-1-yl]pyridazine-3-carboxamide |
| EI11-89-02R | EI11-89 | 1A89 | N-(1-Adamantylmethyl)-6-[4-[[3-(3-hydroxyphenyl)-5-(trifluoromethoxy)phenyl]methyl]piperazin-1-yl]pyridazine-3-carboxamide |
| EI11-90-02R | EI11-90 | 1A90 | N-(1-Adamantylmethyl)-6-[4-[[3-(5-hydroxypyridin-3-yl)-5-(trifluoromethoxy)phenyl]methyl]piperazin-1-yl]pyridazine-3-carboxamide |
| EI11-91-02R | EI11-91 | 1A91 | N-(1-Adamantylmethyl)-6-[4-[[4-(3-hydroxyphenyl)naphthalen-1-yl]methyl]piperazin-1-yl]pyridazine-3-carboxamide |
| EI11-92-02R | EI11-92 | 1A92 | N-(1-Adamantylmethyl)-6-[4-[[4-(5-hydroxypyridin-3-yl)naphthalen-1-yl]methyl]piperazin-1-yl]pyridazine-3-carboxamide |
| EI11-93-02R | EI11-93 | 1A93 | N-(1-Adamantylmethyl)-6-[4-[[4-(3-hydroxyphenyl)thiophen-2-yl]methyl]piperazin-1-yl]pyridazine-3-carboxamide |
| EI11-94-02R | EI11-94 | 1A94 | N-(1-Adamantylmethyl)-6-[4-[[4-(5-hydroxypyridin-3-yl)thiophen-2-yl]methyl]piperazin-1-yl]pyridazine-3-carboxamide |
| EI11-95-02R | EI11-95 | 1A95 | N-(1-Adamantylmethyl)-6-[4-[[2-[2-(3-hydroxyphenyl)ethynyl]phenyl]methyl]piperazin-1-yl]pyridazine-3-carboxamide |
| EI11-96-02R | EI11-96 | 1A96 | N-(1-Adamantylmethyl)-6-[4-[[2-[2-(5-hydroxypyridin-3-yl)ethynyl]phenyl]methyl]piperazin-1-yl]pyridazine-3-carboxamide |
| EI11-97-02R | EI11-97 | 1A97 | N-(1-Adamantylmethyl)-4-[4-[1-[4-(3-hydroxyphenyl)phenyl]ethyl]piperazin-1-yl]benzamide |

| | | | |
|---|---|---|---|
| EI11-98-02R | EI11-98 | 1A98 | N-(1-Adamantylmethyl)-4-[4-[1-[4-(5-hydroxypyridin-3-yl)phenyl]ethyl]piperazin-1-yl]benzamide |
| EI11-99-02R | EI11-99 | 1A99 | N-(1-Adamantylmethyl)-4-[4-[1-[2-fluoro-4-(3-hydroxyphenyl)phenyl]ethyl]piperazin-1-yl]benzamide |
| EI11-100-02R | EI11-100 | 1A100 | N-(1-Adamantylmethyl)-4-[4-[1-[2-fluoro-4-(5-hydroxypyridin-3-yl)phenyl]ethyl]piperazin-1-yl]benzamide |

Example 4-3-10: Synthesis of mixture mixEI12-02R

The title mixture shown in Table EI12 was obtained by synthesis in the same manner as in Example 4-3-9 using mixture mixEI06-02R (50 mg, loading rate: 0.648 mmol/g, 0.0324 mmol) shown in Table EI06 and 1-(1-adamantyl)-N-methylmethanamine hydrochloride (BB35). Compounds contained in the obtained mixture are shown in Table EI12.

[Table 207]

[Table EI12] Mixture No: mixEI12-02R; Amin reagent used: BB35; Mixture No. after cleavage: mixEI12

| Resin compound No. | Compound No. after cleavage | Another name of compound after cleavage in Table EJ06 | Compound name |
|---|---|---|---|
| EI12-01-02R | EI12-01 | 3A01 | N-(1-Adamantylmethyl)-6-[4-[4-(3-hydroxyphenyl)thiophene-2-carbonyl]piperazin-1-yl]-N-methylpyridazine-3-carboxamide |
| EI12-02-02R | EI12-02 | 3A02 | N-(1-Adamantylmethyl)-6-[4-[4-(5-hydroxypyridin-3-yl)thiophene-2-carbonyl]piperazin-1-yl]-N-methylpyridazine-3-carboxamide |
| EI12-03-02R | EI12-03 | 3A03 | N-(1-Adamantylmethyl)-6-[4-[4-(3-hydroxyphenyl)-3-methylbenzoyl]piperazin-1-yl]-N-methylpyridazine-3-carboxamide |
| EI12-04-02R | EI12-04 | 3A04 | N-(1-Adamantylmethyl)-6-[4-[4-(5-hydroxypyridin-3-yl)-3-methylbenzoyl]piperazin-1-yl]-N-methylpyridazine-3-carboxamide |
| EI12-05-02R | EI12-05 | 3A05 | N-(1-Adamantylmethyl)-6-[4-[3-fluoro-4-(3-hydroxyphenyl)benzoyl]piperazin-1-yl]-N-methylpyridazine-3-carboxamide |
| EI12-06-02R | EI12-06 | 3A06 | N-(1-Adamantylmethyl)-6-[4-[3-fluoro-4-(5-hydroxypyridin-3-yl)benzoyl]piperazin-1-yl]-N-methylpyridazine-3-carboxamide |
| EI12-07-02R | EI12-07 | 3A07 | N-(1-Adamantylmethyl)-6-[4-[2-[2-(3-hydroxyphenyl)ethynyl]benzoyl]piperazin-1-yl]-N-methylpyridazine-3-carboxamide |
| EI12-08-02R | EI12-08 | 3A08 | N-(1-Adamantylmethyl)-6-[4-[2-[2-(5-hydroxypyridin-3-yl)ethynyl]benzoyl]piperazin-1-yl]-N-methylpyridazine-3-carboxamide |
| EI12-09-02R | EI12-09 | 3A09 | N-(1-Adamantylmethyl)-6-[4-[5-[2-(3-hydroxyphenyl)ethynyl]pyridine-3-carbonyl]piperazin-1-yl]-N-methylpyridazine-3-carboxamide |
| EI12-10-02R | EI12-10 | 3A10 | N-(1-Adamantylmethyl)-6-[4-[5-[2-(5-hydroxypyridin-3-yl)ethynyl]pyridine-3-carbonyl]piperazin-1-yl]-N-methylpyridazine-3-carboxamide |

| | | | |
|---|---|---|---|
| EI12-11-02R | EI12-11 | 3A11 | N-(1-Adamantylmethyl)-6-[4-[3-[2-(3-hydroxyphenyl)phenyl]propanoyl]piperazin-1-yl]-N-methylpyridazine-3-carboxamide |
| EI12-12-02R | EI12-12 | 3A12 | N-(1-Adamantylmethyl)-6-[4-[3-[2-(5-hydroxypyridin-3-yl)phenyl]propanoyl]piperazin-1-yl]-N-methylpyridazine-3-carboxamide |
| EI12-13-02R | EI12-13 | 3A13 | N-(1-Adamantylmethyl)-6-[4-[4-(3-hydroxyphenyl)naphthalene-1-carbonyl]piperazin-1-yl]-N-methylpyridazine-3-carboxamide |
| EI12-14-02R | EI12-14 | 3A14 | N-(1-Adamantylmethyl)-6-[4-[4-(5-hydroxypyridin-3-yl)naphthalene-1-carbonyl]piperazin-1-yl]-N-methylpyridazine-3-carboxamide |
| EI12-15-02R | EI12-15 | 3A15 | N-(1-Adamantylmethyl)-6-[4-[3-tert-butyl-5-(3-hydroxyphenyl)benzoyl]piperazin-1-yl]-N-methylpyridazine-3-carboxamide |
| EI12-16-02R | EI12-16 | 3A16 | N-(1-Adamantylmethyl)-6-[4-[3-tert-butyl-5-(5-hydroxypyridin-3-yl)benzoyl]piperazin-1-yl]-N-methylpyridazine-3-carboxamide |
| EI12-17-02R | EI12-17 | 3A17 | N-(1-Adamantylmethyl)-6-[4-[3-(3-hydroxyphenyl)-5-(trifluoromethyl)benzoyl]piperazin-1-yl]-N-methylpyridazine-3-carboxamide |
| EI12-18-02R | EI12-18 | 3A18 | N-(1-Adamantylmethyl)-6-[4-[3-(5-hydroxypyridin-3-yl)-5-(trifluoromethyl)benzoyl]piperazin-1-yl]-N-methylpyridazine-3-carboxamide |
| EI12-19-02R | EI12-19 | 3A19 | N-(1-Adamantylmethyl)-6-[4-[3-[2-(3-hydroxyphenyl)ethynyl]-5-(trifluoromethyl)benzoyl]piperazin-1-yl]-N-methylpyridazine-3-carboxamide |
| EI12-20-02R | EI12-20 | 3A20 | N-(1-Adamantylmethyl)-6-[4-[3-[2-(5-hydroxypyridin-3-yl)ethynyl]-5-(trifluoromethyl)benzoyl]piperazin-1-yl]-N-methylpyridazine-3-carboxamide |
| EI12-21-02R | EI12-21 | 3A21 | N-(1-Adamantylmethyl)-6-[4-[3-[4-(3-hydroxyphenyl)pyrazol-1-yl]-5-(trifluoromethyl)benzoyl]piperazin-1-yl]-N-methylpyridazine-3-carboxamide |
| EI12-22-02R | EI12-22 | 3A22 | N-(1-Adamantylmethyl)-6-[4-[3-[4-(5-hydroxypyridin-3-yl)pyrazol-1-yl]-5-(trifluoromethyl)benzoyl]piperazin-1-yl]-N-methylpyridazine-3-carboxamide |
| EI12-23-02R | EI12-23 | 3A23 | N-(1-Adamantylmethyl)-4-[4-[4-(3-hydroxyphenyl)thiophene-2-carbonyl]piperazin-1-yl]-N-methylbenzamide |
| EI12-24-02R | EI12-24 | 3A24 | N-(1-Adamantylmethyl)-4-[4-[4-(5-hydroxypyridin-3-yl)thiophene-2-carbonyl]piperazin-1-yl]-N-methylbenzamide |
| EI12-25-02R | EI12-25 | 3A25 | N-(1-Adamantylmethyl)-4-[4-[4-(3-hydroxyphenyl)-3-methylbenzoyl]piperazin-1-yl]-N-methylbenzamide |
| EI12-26-02R | EI12-26 | 3A26 | N-(1-Adamantylmethyl)-4-[4-[4-(5-hydroxypyridin-3-yl)-3-methylbenzoyl]piperazin-1-yl]-N-methylbenzamide |
| EI12-27-02R | EI12-27 | 3A27 | N-(1-Adamantylmethyl)-4-[4-[3-fluoro-4-(3-hydroxyphenyl)benzoyl]piperazin-1-yl]-N-methylbenzamide |
| EI12-28-02R | EI12-28 | 3A28 | N-(1-Adamantylmethyl)-4-[4-[3-fluoro-4-(5-hydroxypyridin-3-yl)benzoyl]piperazin-1-yl]-N-methylbenzamide |
| EI12-29-02R | EI12-29 | 3A29 | N-(1-Adamantylmethyl)-4-[4-[2-[2-(3-hydroxyphenyl)ethynyl]benzoyl]piperazin-1-yl]-N-methylbenzamide |
| EI12-30-02R | EI12-30 | 3A30 | N-(1-Adamantylmethyl)-4-[4-[2-[2-(5-hydroxypyridin-3-yl)ethynyl]benzoyl]piperazin-1-yl]-N-methylbenzamide |
| EI12-31-02R | EI12-31 | 3A31 | N-(1-Adamantylmethyl)-4-[4-[5-[2-(3-hydroxyphenyl)ethynyl]pyridine-3-carbonyl]piperazin-1-yl]-N-methylbenzamide |
| EI12-32-02R | EI12-32 | 3A32 | N-(1-Adamantylmethyl)-4-[4-[5-[2-(5-hydroxypyridin-3-yl)ethynyl]pyridine-3-carbonyl]piperazin-1-yl]-N-methylbenzamide |

| | | | |
|---|---|---|---|
| EI12-33-02R | EI12-33 | 3A33 | N-(1-Adamantylmethyl)-4-[4-[3-[2-(3-hydroxyphenyl)phenyl]propanoyl]piperazin-1-yl]-N-methylbenzamide |
| EI12-34-02R | EI12-34 | 3A34 | N-(1-Adamantylmethyl)-4-[4-[3-[2-(5-hydroxypyridin-3-yl)phenyl]propanoyl]piperazin-1-yl]-N-methylbenzamide |
| EI12-35-02R | EI12-35 | 3A35 | N-(1-Adamantylmethyl)-4-[4-[4-(3-hydroxyphenyl)naphthalene-1-carbonyl]piperazin-1-yl]-N-methylbenzamide |
| EI12-36-02R | EI12-36 | 3A36 | N-(1-Adamantylmethyl)-4-[4-[4-(5-hydroxypyridin-3-yl)naphthalene-1-carbonyl]piperazin-1-yl]-N-methylbenzamide |
| EI12-37-02R | EI12-37 | 3A37 | N-(1-Adamantylmethyl)-4-[4-[3-tert-butyl-5-(3-hydroxyphenyl)benzoyl]piperazin-1-yl]-N-methylbenzamide |
| EI12-38-02R | EI12-38 | 3A38 | N-(1-Adamantylmethyl)-4-[4-[3-tert-butyl-5-(5-hydroxypyridin-3-yl)benzoyl]piperazin-1-yl]-N-methylbenzamide |
| EI12-39-02R | EI12-39 | 3A39 | N-(1-Adamantylmethyl)-4-[4-[3-(3-hydroxyphenyl)-5-(trifluoromethyl)benzoyl]piperazin-1-yl]-N-methylbenzamide |
| EI12-40-02R | EI12-40 | 3A40 | N-(1-Adamantylmethyl)-4-[4-[3-(5-hydroxypyridin-3-yl)-5-(trifluoromethyl)benzoyl]piperazin-1-yl]-N-methylbenzamide |
| EI12-41-02R | EI12-41 | 3A41 | N-(1-Adamantylmethyl)-4-[4-[3-[2-(3-hydroxyphenyl)ethynyl]-5-(trifluoromethyl)benzoyl]piperazin-1-yl]-N-methylbenzamide |
| EI12-42-02R | EI12-42 | 3A42 | N-(1-Adamantylmethyl)-4-[4-[3-[2-(5-hydroxypyridin-3-yl)ethynyl]-5-(trifluoromethyl)benzoyl]piperazin-1-yl]-N-methylbenzamide |
| EI12-43-02R | EI12-43 | 3A43 | N-(1-Adamantylmethyl)-4-[4-[3-[4-(3-hydroxyphenyl)pyrazol-1-yl]-5-(trifluoromethyl)benzoyl]piperazin-1-yl]-N-methylbenzamide |
| EI12-44-02R | EI12-44 | 3A44 | N-(1-Adamantylmethyl)-4-[4-[3-[4-(5-hydroxypyridin-3-yl)pyrazol-1-yl]-5-(trifluoromethyl)benzoyl]piperazin-1-yl]-N-methylbenzamide |
| EI12-45-02R | EI12-45 | 3A45 | N-(1-Adamantylmethyl)-4-[4-[[2-(3-hydroxyphenyl)phenyl]methyl]piperazin-1-yl]-N-methylbenzamide |
| EI12-46-02R | EI12-46 | 3A46 | N-(1-Adamantylmethyl)-4-[4-[[2-(5-hydroxypyridin-3-yl)phenyl]methyl]piperazin-1-yl]-N-methylbenzamide |
| EI12-47-02R | EI12-47 | 3A47 | N-(1-Adamantylmethyl)-4-[4-[[4-(3-hydroxyphenyl)-3-methylphenyl]methyl]piperazin-1-yl]-N-methylbenzamide |
| EI12-48-02R | EI12-48 | 3A48 | N-(1-Adamantylmethyl)-4-[4-[[4-(5-hydroxypyridin-3-yl)-3-methylphenyl]methyl]piperazin-1-yl]-N-methylbenzamide |
| EI12-49-02R | EI12-49 | 3A49 | N-(1-Adamantylmethyl)-4-[4-[[2-ethyl-4-(3-hydroxyphenyl)phenyl]methyl]piperazin-1-yl]-N-methylbenzamide |
| EI12-50-02R | EI12-50 | 3A50 | N-(1-Adamantylmethyl)-4-[4-[[2-ethyl-4-(5-hydroxypyridin-3-yl)phenyl]methyl]piperazin-1-yl]-N-methylbenzamide |
| EI12-51-02R | EI12-51 | 3A51 | N-(1-Adamantylmethyl)-4-[4-[[3-fluoro-4-(3-hydroxyphenyl)phenyl]methyl]piperazin-1-yl]-N-methylbenzamide |
| EI12-52-02R | EI12-52 | 3A52 | N-(1-Adamantylmethyl)-4-[4-[[3-fluoro-4-(5-hydroxypyridin-3-yl)phenyl]methyl]piperazin-1-yl]-N-methylbenzamide |
| EI12-53-02R | EI12-53 | 3A53 | N-(1-Adamantylmethyl)-4-[4-[[4-(3-hydroxyphenyl)-2-methoxyphenyl]methyl]piperazin-1-yl]-N-methylbenzamide |
| EI12-54-02R | EI12-54 | 3A54 | N-(1-Adamantylmethyl)-4-[4-[[4-(5-hydroxypyridin-3-yl)-2-methoxyphenyl]methyl]piperazin-1-yl]-N-methylbenzamide |
| EI12-55-02R | EI12-55 | 3A55 | N-(1-Adamantylmethyl)-4-[4-[[3-ethoxy-5-(3-hydroxyphenyl)phenyl]methyl]piperazin-1-yl]-N-methylbenzamide |
| EI12-56-02R | EI12-56 | 3A56 | N-(1-Adamantylmethyl)-4-[4-[[3-ethoxy-5-(5-hydroxypyridin-3-yl)phenyl]methyl]piperazin-1-yl]-N-methylbenzamide |
| EI12-57-02R | EI12-57 | 3A57 | N-(1-Adamantylmethyl)-4-[4-[[3-(3-hydroxyphenyl)-5-propan-2-yloxyphenyl]methyl]piperazin-1-yl]-N-methylbenzamide |
| EI12-58-02R | EI12-58 | 3A58 | N-(1-Adamantylmethyl)-4-[4-[[3-(5-hydroxypyridin-3-yl)-5-propan-2-yloxyphenyl]methyl]piperazin-1-yl]-N-methylbenzamide |
| EI12-59-02R | EI12-59 | 3A59 | N-(1-Adamantylmethyl)-4-[4-[[4-(3-hydroxyphenyl)-2,6-dimethoxyphenyl]methyl]piperazin-1-yl]-N-methylbenzamide |

| | | | |
|---|---|---|---|
| EI12-60-02R | EI12-60 | 3A60 | N-(1-Adamantylmethyl)-4-[4-[[4-(5-hydroxypyridin-3-yl)-2,6-dimethoxyphenyl]methyl]piperazin-1-yl]-N-methylbenzamide |
| EI12-61-02R | EI12-61 | 3A61 | N-(1-Adamantylmethyl)-4-[4-[[3-(3-hydroxyphenyl)-5-(trifluoromethyl)phenyl]methyl]piperazin-1-yl]-N-methylbenzamide |
| EI12-62-02R | EI12-62 | 3A62 | N-(1-Adamantylmethyl)-4-[4-[[3-(5-hydroxypyridin-3-yl)-5-(trifluoromethyl)phenyl]methyl]piperazin-1-yl]-N-methylbenzamide |
| EI12-63-02R | EI12-63 | 3A63 | N-(1-Adamantylmethyl)-4-[4-[[3-(3-hydroxyphenyl)-5-(trifluoromethoxy)phenyl]methyl]piperazin-1-yl]-N-methylbenzamide |
| EI12-64-02R | EI12-64 | 3A64 | N-(1-Adamantylmethyl)-4-[4-[[3-(5-hydroxypyridin-3-yl)-5-(trifluoromethoxy)phenyl]methyl]piperazin-1-yl]-N-methylbenzamide |
| EI12-65-02R | EI12-65 | 3A65 | N-(1-Adamantylmethyl)-4-[4-[[4-(3-hydroxyphenyl)naphthalen-1-yl]methyl]piperazin-1-yl]-N-methylbenzamide |
| EI12-66-02R | EI12-66 | 3A66 | N-(1-Adamantylmethyl)-4-[4-[[4-(5-hydroxypyridin-3-yl)naphthalen-1-yl]methyl]piperazin-1-yl]-N-methylbenzamide |
| EI12-67-02R | EI12-67 | 3A67 | N-(1-Adamantylmethyl)-4-[4-[[4-(3-hydroxyphenyl)thiophen-2-yl]methyl]piperazin-1-yl]-N-methylbenzamide |
| EI12-68-02R | EI12-68 | 3A68 | N-(1-Adamantylmethyl)-4-[4-[[4-(5-hydroxypyridin-3-yl)thiophen-2-yl]methyl]piperazin-1-yl]-N-methylbenzamide |
| EI12-69-02R | EI12-69 | 3A69 | N-(1-Adamantylmethyl)-4-[4-[[2-[2-(3-hydroxyphenyl)ethynyl]phenyl]methyl]piperazin-1-yl]-N-methylbenzamide |
| EI12-70-02R | EI12-70 | 3A70 | N-(1-Adamantylmethyl)-4-[4-[[2-[2-(5-hydroxypyridin-3-yl)ethynyl]phenyl]methyl]piperazin-1-yl]-N-methylbenzamide |
| EI12-71-02R | EI12-71 | 3A71 | N-(1-Adamantylmethyl)-6-[4-[[2-(3-hydroxyphenyl)phenyl]methyl]piperazin-1-yl]-N-methylpyridazine-3-carboxamide |
| EI12-72-02R | EI12-72 | 3A72 | N-(1-Adamantylmethyl)-6-[4-[[2-(5-hydroxypyridin-3-yl)phenyl]methyl]piperazin-1-yl]-N-methylpyridazine-3-carboxamide |
| EI12-73-02R | EI12-73 | 3A73 | N-(1-Adamantylmethyl)-6-[4-[[4-(3-hydroxyphenyl)-3-methylphenyl]methyl]piperazin-1-yl]-N-methylpyridazine-3-carboxamide |
| EI12-74-02R | EI12-74 | 3A74 | N-(1-Adamantylmethyl)-6-[4-[[4-(5-hydroxypyridin-3-yl)-3-methylphenyl]methyl]piperazin-1-yl]-N-methylpyridazine-3-carboxamide |
| EI12-75-02R | EI12-75 | 3A75 | N-(1-Adamantylmethyl)-6-[4-[[2-ethyl-4-(3-hydroxyphenyl)phenyl]methyl]piperazin-1-yl]-N-methylpyridazine-3-carboxamide |
| EI12-76-02R | EI12-76 | 3A76 | N-(1-Adamantylmethyl)-6-[4-[[2-ethyl-4-(5-hydroxypyridin-3-yl)phenyl]methyl]piperazin-1-yl]-N-methylpyridazine-3-carboxamide |
| EI12-77-02R | EI12-77 | 3A77 | N-(1-Adamantylmethyl)-6-[4-[[3-fluoro-4-(3-hydroxyphenyl)phenyl]methyl]piperazin-1-yl]-N-methylpyridazine-3-carboxamide |
| EI12-78-02R | EI12-78 | 3A78 | N-(1-Adamantylmethyl)-6-[4-[[3-fluoro-4-(5-hydroxypyridin-3-yl)phenyl]methyl]piperazin-1-yl]-N-methylpyridazine-3-carboxamide |
| EI12-79-02R | EI12-79 | 3A79 | N-(1-Adamantylmethyl)-6-[4-[[4-(3-hydroxyphenyl)-2-methoxyphenyl]methyl]piperazin-1-yl]-N-methylpyridazine-3-carboxamide |
| EI12-80-02R | EI12-80 | 3A80 | N-(1-Adamantylmethyl)-6-[4-[[4-(5-hydroxypyridin-3-yl)-2-methoxyphenyl]methyl]piperazin-1-yl]-N-methylpyridazine-3-carboxamide |

| | | | |
|---|---|---|---|
| EI12-81-02R | EI12-81 | 3A81 | N-(1-Adamantylmethyl)-6-[4-[[3-ethoxy-5-(3-hydroxyphenyl)phenyl]methyl]piperazin-1-yl]-N-methylpyridazine-3-carboxamide |
| EI12-82-02R | EI12-82 | 3A82 | N-(1-Adamantylmethyl)-6-[4-[[3-ethoxy-5-(5-hydroxypyridin-3-yl)phenyl]methyl]piperazin-1-yl]-N-methylpyridazine-3-carboxamide |
| EI12-83-02R | EI12-83 | 3A83 | N-(1-Adamantylmethyl)-6-[4-[[3-(3-hydroxyphenyl)-5-propan-2-yloxyphenyl]methyl]piperazin-1-yl]-N-methylpyridazine-3-carboxamide |
| EI12-84-02R | EI12-84 | 3A84 | N-(1-Adamantylmethyl)-6-[4-[[3-(5-hydroxypyridin-3-yl)-5-propan-2-yloxyphenyl]methyl]piperazin-1-yl]-N-methylpyridazine-3-carboxamide |
| EI12-85-02R | EI12-85 | 3A85 | N-(1-Adamantylmethyl)-6-[4-[[4-(3-hydroxyphenyl)-2,6-dimethoxyphenyl]methyl]piperazin-1-yl]-N-methylpyridazine-3-carboxamide |
| EI12-86-02R | EI12-86 | 3A86 | N-(1-Adamantylmethyl)-6-[4-[[4-(5-hydroxypyridin-3-yl)-2,6-dimethoxyphenyl]methyl]piperazin-1-yl]-N-methylpyridazine-3-carboxamide |
| EI12-87-02R | EI12-87 | 3A87 | N-(1-Adamantylmethyl)-6-[4-[[3-(3-hydroxyphenyl)-5-(trifluoromethyl)phenyl]methyl]piperazin-1-yl]-N-methylpyridazine-3-carboxamide |
| EI12-88-02R | EI12-88 | 3A88 | N-(1-Adamantylmethyl)-6-[4-[[3-(5-hydroxypyridin-3-yl)-5-(trifluoromethyl)phenyl]methyl]piperazin-1-yl]-N-methylpyridazine-3-carboxamide |
| EI12-89-02R | EI12-89 | 3A89 | N-(1-Adamantylmethyl)-6-[4-[[3-(3-hydroxyphenyl)-5-(trifluoromethoxy)phenyl]methyl]piperazin-1-yl]-N-methylpyridazine-3-carboxamide |
| EI12-90-02R | EI12-90 | 3A90 | N-(1-Adamantylmethyl)-6-[4-[[3-(5-hydroxypyridin-3-yl)-5-(trifluoromethoxy)phenyl]methyl]piperazin-1-yl]-N-methylpyridazine-3-carboxamide |
| EI12-91-02R | EI12-91 | 3A91 | N-(1-Adamantylmethyl)-6-[4-[[4-(3-hydroxyphenyl)naphthalen-1-yl]methyl]piperazin-1-yl]-N-methylpyridazine-3-carboxamide |
| EI12-92-02R | EI12-92 | 3A92 | N-(1-Adamantylmethyl)-6-[4-[[4-(5-hydroxypyridin-3-yl)naphthalen-1-yl]methyl]piperazin-1-yl]-N-methylpyridazine-3-carboxamide |
| EI12-93-02R | EI12-93 | 3A93 | N-(1-Adamantylmethyl)-6-[4-[[4-(3-hydroxyphenyl)thiophen-2-yl]methyl]piperazin-1-yl]-N-methylpyridazine-3-carboxamide |
| EI12-94-02R | EI12-94 | 3A94 | N-(1-Adamantylmethyl)-6-[4-[[4-(5-hydroxypyridin-3-yl)thiophen-2-yl]methyl]piperazin-1-yl]-N-methylpyridazine-3-carboxamide |
| EI12-95-02R | EI12-95 | 3A95 | N-(1-Adamantylmethyl)-6-[4-[[2-[2-(3-hydroxyphenyl)ethynyl]phenyl]methyl]piperazin-1-yl]-N-methylpyridazine-3-carboxamide |
| EI12-96-02R | EI12-96 | 3A96 | N-(1-Adamantylmethyl)-6-[4-[[2-[2-(5-hydroxypyridin-3-yl)ethynyl]phenyl]methyl]piperazin-1-yl]-N-methylpyridazine-3-carboxamide |
| EI12-97-02R | EI12-97 | 3A97 | N-(1-Adamantylmethyl)-4-[4-[1-[4-(3-hydroxyphenyl)phenyl]ethyl]piperazin-1-yl]-N-methylbenzamide |
| EI12-98-02R | EI12-98 | 3A98 | N-(1-Adamantylmethyl)-4-[4-[1-[4-(5-hydroxypyridin-3-yl)phenyl]ethyl]piperazin-1-yl]-N-methylbenzamide |
| EI12-99-02R | EI12-99 | 3A99 | N-(1-Adamantylmethyl)-4-[4-[1-[2-fluoro-4-(3-hydroxyphenyl)phenyl]ethyl]piperazin-1-yl]-N-methylbenzamide |
| EI12-100-02R | EI12-100 | 3A100 | N-(1-Adamantylmethyl)-4-[4-[1-[2-fluoro-4-(5-hydroxypyridin-3-yl)phenyl]ethyl]piperazin-1-yl]-N-methylbenzamide |

Example 4-3-11: Synthesis of mixture mixEI13-02R

[Formula 544]

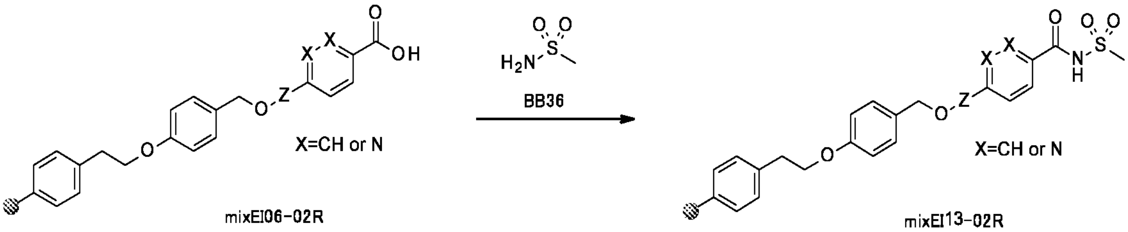


Mixture mixEI06-02R (50 mg, loading rate: 0.648 mmol/g, 0.0324 mmol) shown in Table EI06 and NMP (1.13 mL) were added to a 2 mL glass vial and shaken at room temperature for 1 hour. HATU (73.9 mg, 0.194 mmol) and 2,6-lutidine (22.6 μL, 0.194 mmol) were added thereto at room temperature, and the mixture was shaken at 40°C for 3 hours. Methanesulfonamide (BB36, 21.6 mg, 0.227 mmol) and phosphazene P1tBu (124 μL, 0.486 mmol) were added thereto at room temperature, and the mixture was shaken at 40°C for 18 hours. n-Propylamine (27 μL, 0.324 mmol) was added thereto at room temperature, and the mixture was shaken for 1 hour. The reaction solution and the resin suspension were transferred to a syringe with a filter using NMP, repetitively washed three times with NMP (1.2 mL), three times with HOAt/NMP (0.2 M, 1.2 mL), three times with NMP (1.2 mL), three times with MeOH (1.2 mL), three times with DCM (1.2 mL), and three times with heptane (1.2 mL), and then dried under reduced pressure to obtain the title mixture shown in Table EI13. Compounds contained in the obtained mixture are shown in Table EI13.

[Table 208]

[Table EI13] Mixture No: mixEI13-02R; Sulfonamide reagent used: BB36; Mixture No. after cleavage: mixEI13

| Resin compound No. | Compound No. after cleavage | Another name of compound after cleavage in Table EJ07 | Compound name |
|---|---|---|---|
| EI13-01-02R | EI13-01 | 2I01 | 6-[4-[4-(3-Hydroxyphenyl)thiophene-2-carbonyl]piperazin-1-yl]-N-methylsulfonylpyridazine-3-carboxamide |
| EI13-02-02R | EI13-02 | 2I02 | 6-[4-[4-(5-Hydroxypyridin-3-yl)thiophene-2-carbonyl]piperazin-1-yl]-N-methylsulfonylpyridazine-3-carboxamide |
| EI13-03-02R | EI13-03 | 2I03 | 6-[4-[4-(3-Hydroxyphenyl)-3-methylbenzoyl]piperazin-1-yl]-N-methylsulfonylpyridazine-3-carboxamide |
| EI13-04-02R | EI13-04 | 2I04 | 6-[4-[4-(5-Hydroxypyridin-3-yl)-3-methylbenzoyl]piperazin-1-yl]-N-methylsulfonylpyridazine-3-carboxamide |

| | | | |
|---|---|---|---|
| EI13-05-02R | EI13-05 | 2I05 | 6-[4-[3-Fluoro-4-(3-hydroxyphenyl)benzoyl]piperazin-1-yl]-N-methylsulfonylpyridazine-3-carboxamide |
| EI13-06-02R | EI13-06 | 2I06 | 6-[4-[3-Fluoro-4-(5-hydroxypyridin-3-yl)benzoyl]piperazin-1-yl]-N-methylsulfonylpyridazine-3-carboxamide |
| EI13-07-02R | EI13-07 | 2I07 | 6-[4-[2-[2-(3-Hydroxyphenyl)ethynyl]benzoyl]piperazin-1-yl]-N-methylsulfonylpyridazine-3-carboxamide |
| EI13-08-02R | EI13-08 | 2I08 | 6-[4-[2-[2-(5-Hydroxypyridin-3-yl)ethynyl]benzoyl]piperazin-1-yl]-N-methylsulfonylpyridazine-3-carboxamide |
| EI13-09-02R | EI13-09 | 2I09 | 6-[4-[5-[2-(3-Hydroxyphenyl)ethynyl]pyridine-3-carbonyl]piperazin-1-yl]-N-methylsulfonylpyridazine-3-carboxamide |
| EI13-10-02R | EI13-10 | 2I10 | 6-[4-[5-[2-(5-Hydroxypyridin-3-yl)ethynyl]pyridine-3-carbonyl]piperazin-1-yl]-N-methylsulfonylpyridazine-3-carboxamide |
| EI13-11-02R | EI13-11 | 2I11 | 6-[4-[3-[2-(3-Hydroxyphenyl)phenyl]propanoyl]piperazin-1-yl]-N-methylsulfonylpyridazine-3-carboxamide |
| EI13-12-02R | EI13-12 | 2I12 | 6-[4-[3-[2-(5-Hydroxypyridin-3-yl)phenyl]propanoyl]piperazin-1-yl]-N-methylsulfonylpyridazine-3-carboxamide |
| EI13-13-02R | EI13-13 | 2I13 | 6-[4-[4-(3-Hydroxyphenyl)naphthalene-1-carbonyl]piperazin-1-yl]-N-methylsulfonylpyridazine-3-carboxamide |
| EI13-14-02R | EI13-14 | 2I14 | 6-[4-[4-(5-Hydroxypyridin-3-yl)naphthalene-1-carbonyl]piperazin-1-yl]-N-methylsulfonylpyridazine-3-carboxamide |
| EI13-15-02R | EI13-15 | 2I15 | 6-[4-[3-tert-Butyl-5-(3-hydroxyphenyl)benzoyl]piperazin-1-yl]-N-methylsulfonylpyridazine-3-carboxamide |
| EI13-16-02R | EI13-16 | 2I16 | 6-[4-[3-tert-Butyl-5-(5-hydroxypyridin-3-yl)benzoyl]piperazin-1-yl]-N-methylsulfonylpyridazine-3-carboxamide |
| EI13-17-02R | EI13-17 | 2I17 | 6-[4-[3-(3-Hydroxyphenyl)-5-(trifluoromethyl)benzoyl]piperazin-1-yl]-N-methylsulfonylpyridazine-3-carboxamide |
| EI13-18-02R | EI13-18 | 2I18 | 6-[4-[3-(5-Hydroxypyridin-3-yl)-5-(trifluoromethyl)benzoyl]piperazin-1-yl]-N-methylsulfonylpyridazine-3-carboxamide |
| EI13-19-02R | EI13-19 | 2I19 | 6-[4-[3-[2-(3-Hydroxyphenyl)ethynyl]-5-(trifluoromethyl)benzoyl]piperazin-1-yl]-N-methylsulfonylpyridazine-3-carboxamide |
| EI13-20-02R | EI13-20 | 2I20 | 6-[4-[3-[2-(5-Hydroxypyridin-3-yl)ethynyl]-5-(trifluoromethyl)benzoyl]piperazin-1-yl]-N-methylsulfonylpyridazine-3-carboxamide |
| EI13-21-02R | EI13-21 | 2I21 | 6-[4-[3-[4-(3-Hydroxyphenyl)pyrazol-1-yl]-5-(trifluoromethyl)benzoyl]piperazin-1-yl]-N-methylsulfonylpyridazine-3-carboxamide |
| EI13-22-02R | EI13-22 | 2I22 | 6-[4-[3-[4-(5-Hydroxypyridin-3-yl)pyrazol-1-yl]-5-(trifluoromethyl)benzoyl]piperazin-1-yl]-N-methylsulfonylpyridazine-3-carboxamide |
| EI13-23-02R | EI13-23 | 2I23 | 4-[4-[4-(3-Hydroxyphenyl)thiophene-2-carbonyl]piperazin-1-yl]-N-methylsulfonylbenzamide |
| EI13-24-02R | EI13-24 | 2I24 | 4-[4-[4-(5-Hydroxypyridin-3-yl)thiophene-2-carbonyl]piperazin-1-yl]-N-methylsulfonylbenzamide |
| EI13-25-02R | EI13-25 | 2I25 | 4-[4-[4-(3-Hydroxyphenyl)-3-methylbenzoyl]piperazin-1-yl]-N-methylsulfonylbenzamide |
| EI13-26-02R | EI13-26 | 2I26 | 4-[4-[4-(5-Hydroxypyridin-3-yl)-3-methylbenzoyl]piperazin-1-yl]-N-methylsulfonylbenzamide |
| EI13-27-02R | EI13-27 | 2I27 | 4-[4-[3-Fluoro-4-(3-hydroxyphenyl)benzoyl]piperazin-1-yl]-N-methylsulfonylbenzamide |
| EI13-28-02R | EI13-28 | 2I28 | 4-[4-[3-Fluoro-4-(5-hydroxypyridin-3-yl)benzoyl]piperazin-1-yl]-N-methylsulfonylbenzamide |
| EI13-29-02R | EI13-29 | 2I29 | 4-[4-[2-[2-(3-Hydroxyphenyl)ethynyl]benzoyl]piperazin-1-yl]-N-methylsulfonylbenzamide |

| | | | |
|---|---|---|---|
| EI13-30-02R | EI13-30 | 2I30 | 4-[4-[2-[2-(5-Hydroxypyridin-3-yl)ethynyl]benzoyl]piperazin-1-yl]-N-methylsulfonylbenzamide |
| EI13-31-02R | EI13-31 | 2I31 | 4-[4-[5-[2-(3-Hydroxyphenyl)ethynyl]pyridine-3-carbonyl]piperazin-1-yl]-N-methylsulfonylbenzamide |
| EI13-32-02R | EI13-32 | 2I32 | 4-[4-[5-[2-(5-Hydroxypyridin-3-yl)ethynyl]pyridine-3-carbonyl]piperazin-1-yl]-N-methylsulfonylbenzamide |
| EI13-33-02R | EI13-33 | 2I33 | 4-[4-[3-[2-(3-Hydroxyphenyl)phenyl]propanoyl]piperazin-1-yl]-N-methylsulfonylbenzamide |
| EI13-34-02R | EI13-34 | 2I34 | 4-[4-[3-[2-(5-Hydroxypyridin-3-yl)phenyl]propanoyl]piperazin-1-yl]-N-methylsulfonylbenzamide |
| EI13-35-02R | EI13-35 | 2I35 | 4-[4-[4-(3-Hydroxyphenyl)naphthalene-1-carbonyl]piperazin-1-yl]-N-methylsulfonylbenzamide |
| EI13-36-02R | EI13-36 | 2I36 | 4-[4-[4-(5-Hydroxypyridin-3-yl)naphthalene-1-carbonyl]piperazin-1-yl]-N-methylsulfonylbenzamide |
| EI13-37-02R | EI13-37 | 2I37 | 4-[4-[3-tert-Butyl-5-(3-hydroxyphenyl)benzoyl]piperazin-1-yl]-N-methylsulfonylbenzamide |
| EI13-38-02R | EI13-38 | 2I38 | 4-[4-[3-tert-Butyl-5-(5-hydroxypyridin-3-yl)benzoyl]piperazin-1-yl]-N-methylsulfonylbenzamide |
| EI13-39-02R | EI13-39 | 2I39 | 4-[4-[3-(3-Hydroxyphenyl)-5-(trifluoromethyl)benzoyl]piperazin-1-yl]-N-methylsulfonylbenzamide |
| EI13-40-02R | EI13-40 | 2I40 | 4-[4-[3-(5-Hydroxypyridin-3-yl)-5-(trifluoromethyl)benzoyl]piperazin-1-yl]-N-methylsulfonylbenzamide |
| EI13-41-02R | EI13-41 | 2I41 | 4-[4-[3-[2-(3-Hydroxyphenyl)ethynyl]-5-(trifluoromethyl)benzoyl]piperazin-1-yl]-N-methylsulfonylbenzamide |
| EI13-42-02R | EI13-42 | 2I42 | 4-[4-[3-[2-(5-Hydroxypyridin-3-yl)ethynyl]-5-(trifluoromethyl)benzoyl]piperazin-1-yl]-N-methylsulfonylbenzamide |
| EI13-43-02R | EI13-43 | 2I43 | 4-[4-[3-[4-(3-Hydroxyphenyl)pyrazol-1-yl]-5-(trifluoromethyl)benzoyl]piperazin-1-yl]-N-methylsulfonylbenzamide |
| EI13-44-02R | EI13-44 | 2I44 | 4-[4-[3-[4-(5-Hydroxypyridin-3-yl)pyrazol-1-yl]-5-(trifluoromethyl)benzoyl]piperazin-1-yl]-N-methylsulfonylbenzamide |
| EI13-45-02R | EI13-45 | 2I45 | 4-[4-[[2-(3-Hydroxyphenyl)phenyl]methyl]piperazin-1-yl]-N-methylsulfonylbenzamide |
| EI13-46-02R | EI13-46 | 2I46 | 4-[4-[[2-(5-Hydroxypyridin-3-yl)phenyl]methyl]piperazin-1-yl]-N-methylsulfonylbenzamide |
| EI13-47-02R | EI13-47 | 2I47 | 4-[4-[[4-(3-Hydroxyphenyl)-3-methylphenyl]methyl]piperazin-1-yl]-N-methylsulfonylbenzamide |
| EI13-48-02R | EI13-48 | 2I48 | 4-[4-[[4-(5-Hydroxypyridin-3-yl)-3-methylphenyl]methyl]piperazin-1-yl]-N-methylsulfonylbenzamide |
| EI13-49-02R | EI13-49 | 2I49 | 4-[4-[[2-Eethyl-4-(3-hydroxyphenyl)phenyl]methyl]piperazin-1-yl]-N-methylsulfonylbenzamide |
| EI13-50-02R | EI13-50 | 2I50 | 4-[4-[[2-Ethyl-4-(5-hydroxypyridin-3-yl)phenyl]methyl]piperazin-1-yl]-N-methylsulfonylbenzamide |
| EI13-51-02R | EI13-51 | 2I51 | 4-[4-[[3-Fluoro-4-(3-hydroxyphenyl)phenyl]methyl]piperazin-1-yl]-N-methylsulfonylbenzamide |
| EI13-52-02R | EI13-52 | 2I52 | 4-[4-[[3-Fluoro-4-(5-hydroxypyridin-3-yl)phenyl]methyl]piperazin-1-yl]-N-methylsulfonylbenzamide |
| EI13-53-02R | EI13-53 | 2I53 | 4-[4-[[4-(3-Hydroxyphenyl)-2-methoxyphenyl]methyl]piperazin-1-yl]-N-methylsulfonylbenzamide |
| EI13-54-02R | EI13-54 | 2I54 | 4-[4-[[4-(5-Hydroxypyridin-3-yl)-2-methoxyphenyl]methyl]piperazin-1-yl]-N-methylsulfonylbenzamide |
| EI13-55-02R | EI13-55 | 2I55 | 4-[4-[[3-Ethoxy-5-(3-hydroxyphenyl)phenyl]methyl]piperazin-1-yl]-N-methylsulfonylbenzamide |

| | | | |
|---|---|---|---|
| EI13-56-02R | EI13-56 | 2I56 | 4-[4-[[3-Ethoxy-5-(5-hydroxypyridin-3-yl)phenyl]methyl]piperazin-1-yl]-N-methylsulfonylbenzamide |
| EI13-57-02R | EI13-57 | 2I57 | 4-[4-[[3-(3-Hydroxyphenyl)-5-propan-2-yloxyphenyl]methyl]piperazin-1-yl]-N-methylsulfonylbenzamide |
| EI13-58-02R | EI13-58 | 2I58 | 4-[4-[[3-(5-Hydroxypyridin-3-yl)-5-propan-2-yloxyphenyl]methyl]piperazin-1-yl]-N-methylsulfonylbenzamide |
| EI13-59-02R | EI13-59 | 2I59 | 4-[4-[[4-(3-Hydroxyphenyl)-2,6-dimethoxyphenyl]methyl]piperazin-1-yl]-N-methylsulfonylbenzamide |
| EI13-60-02R | EI13-60 | 2I60 | 4-[4-[[4-(5-Hydroxypyridin-3-yl)-2,6-dimethoxyphenyl]methyl]piperazin-1-yl]-N-methylsulfonylbenzamide |
| EI13-61-02R | EI13-61 | 2I61 | 4-[4-[[3-(3-Hydroxyphenyl)-5-(trifluoromethyl)phenyl]methyl]piperazin-1-yl]-N-methylsulfonylbenzamide |
| EI13-62-02R | EI13-62 | 2I62 | 4-[4-[[3-(5-Hydroxypyridin-3-yl)-5-(trifluoromethyl)phenyl]methyl]piperazin-1-yl]-N-methylsulfonylbenzamide |
| EI13-63-02R | EI13-63 | 2I63 | 4-[4-[[3-(3-Hydroxyphenyl)-5-(trifluoromethoxy)phenyl]methyl]piperazin-1-yl]-N-methylsulfonylbenzamide |
| EI13-64-02R | EI13-64 | 2I64 | 4-[4-[[3-(5-Hydroxypyridin-3-yl)-5-(trifluoromethoxy)phenyl]methyl]piperazin-1-yl]-N-methylsulfonylbenzamide |
| EI13-65-02R | EI13-65 | 2I65 | 4-[4-[[4-(3-Hydroxyphenyl)naphthalen-1-yl]methyl]piperazin-1-yl]-N-methylsulfonylbenzamide |
| EI13-66-02R | EI13-66 | 2I66 | 4-[4-[[4-(5-Hydroxypyridin-3-yl)naphthalen-1-yl]methyl]piperazin-1-yl]-N-methylsulfonylbenzamide |
| EI13-67-02R | EI13-67 | 2I67 | 4-[4-[[4-(3-Hydroxyphenyl)thiophen-2-yl]methyl]piperazin-1-yl]-N-methylsulfonylbenzamide |
| EI13-68-02R | EI13-68 | 2I68 | 4-[4-[[4-(5-Hydroxypyridin-3-yl)thiophen-2-yl]methyl]piperazin-1-yl]-N-methylsulfonylbenzamide |
| EI13-69-02R | EI13-69 | 2I69 | 4-[4-[[2-[2-(3-Hydroxyphenyl)ethynyl]phenyl]methyl]piperazin-1-yl]-N-methylsulfonylbenzamide |
| EI13-70-02R | EI13-70 | 2I70 | 4-[4-[[2-[2-(5-Hydroxypyridin-3-yl)ethynyl]phenyl]methyl]piperazin-1-yl]-N-methylsulfonylbenzamide |
| EI13-71-02R | EI13-71 | 2I71 | 6-[4-[[2-(3-Hydroxyphenyl)phenyl]methyl]piperazin-1-yl]-N-methylsulfonylpyridazine-3-carboxamide |
| EI13-72-02R | EI13-72 | 2I72 | 6-[4-[[2-(5-Hydroxypyridin-3-yl)phenyl]methyl]piperazin-1-yl]-N-methylsulfonylpyridazine-3-carboxamide |
| EI13-73-02R | EI13-73 | 2I73 | 6-[4-[[4-(3-Hydroxyphenyl)-3-methylphenyl]methyl]piperazin-1-yl]-N-methylsulfonylpyridazine-3-carboxamide |
| EI13-74-02R | EI13-74 | 2I74 | 6-[4-[[4-(5-Hydroxypyridin-3-yl)-3-methylphenyl]methyl]piperazin-1-yl]-N-methylsulfonylpyridazine-3-carboxamide |
| EI13-75-02R | EI13-75 | 2I75 | 6-[4-[[2-Ethyl-4-(3-hydroxyphenyl)phenyl]methyl]piperazin-1-yl]-N-methylsulfonylpyridazine-3-carboxamide |
| EI13-76-02R | EI13-76 | 2I76 | 6-[4-[[2-Ethyl-4-(5-hydroxypyridin-3-yl)phenyl]methyl]piperazin-1-yl]-N-methylsulfonylpyridazine-3-carboxamide |
| EI13-77-02R | EI13-77 | 2I77 | 6-[4-[[3-Fluoro-4-(3-hydroxyphenyl)phenyl]methyl]piperazin-1-yl]-N-methylsulfonylpyridazine-3-carboxamide |
| EI13-78-02R | EI13-78 | 2I78 | 6-[4-[[3-Fluoro-4-(5-hydroxypyridin-3-yl)phenyl]methyl]piperazin-1-yl]-N-methylsulfonylpyridazine-3-carboxamide |
| EI13-79-02R | EI13-79 | 2I79 | 6-[4-[[4-(3-Hydroxyphenyl)-2-methoxyphenyl]methyl]piperazin-1-yl]-N-methylsulfonylpyridazine-3-carboxamide |
| EI13-80-02R | EI13-80 | 2I80 | 6-[4-[[4-(5-Hydroxypyridin-3-yl)-2-methoxyphenyl]methyl]piperazin-1-yl]-N-methylsulfonylpyridazine-3-carboxamide |

| | | | |
|---|---|---|---|
| EI13-81-02R | EI13-81 | 2I81 | 6-[4-[[3-Ethoxy-5-(3-hydroxyphenyl)phenyl]methyl]piperazin-1-yl]-N-methylsulfonylpyridazine-3-carboxamide |
| EI13-82-02R | EI13-82 | 2I82 | 6-[4-[[3-Ethoxy-5-(5-hydroxypyridin-3-yl)phenyl]methyl]piperazin-1-yl]-N-methylsulfonylpyridazine-3-carboxamide |
| EI13-83-02R | EI13-83 | 2I83 | 6-[4-[[3-(3-Hydroxyphenyl)-5-propan-2-yloxyphenyl]methyl]piperazin-1-yl]-N-methylsulfonylpyridazine-3-carboxamide |
| EI13-84-02R | EI13-84 | 2I84 | 6-[4-[[3-(5-Hydroxypyridin-3-yl)-5-propan-2-yloxyphenyl]methyl]piperazin-1-yl]-N-methylsulfonylpyridazine-3-carboxamide |
| EI13-85-02R | EI13-85 | 2I85 | 6-[4-[[4-(3-Hydroxyphenyl)-2,6-dimethoxyphenyl]methyl]piperazin-1-yl]-N-methylsulfonylpyridazine-3-carboxamide |
| EI13-86-02R | EI13-86 | 2I86 | 6-[4-[[4-(5-Hydroxypyridin-3-yl)-2,6-dimethoxyphenyl]methyl]piperazin-1-yl]-N-methylsulfonylpyridazine-3-carboxamide |
| EI13-87-02R | EI13-87 | 2I87 | 6-[4-[[3-(3-Hydroxyphenyl)-5-(trifluoromethyl)phenyl]methyl]piperazin-1-yl]-N-methylsulfonylpyridazine-3-carboxamide |
| EI13-88-02R | EI13-88 | 2I88 | 6-[4-[[3-(5-Hydroxypyridin-3-yl)-5-(trifluoromethyl)phenyl]methyl]piperazin-1-yl]-N-methylsulfonylpyridazine-3-carboxamide |
| EI13-89-02R | EI13-89 | 2I89 | 6-[4-[[3-(3-Hydroxyphenyl)-5-(trifluoromethoxy)phenyl]methyl]piperazin-1-yl]-N-methylsulfonylpyridazine-3-carboxamide |
| EI13-90-02R | EI13-90 | 2I90 | 6-[4-[[3-(5-Hydroxypyridin-3-yl)-5-(trifluoromethoxy)phenyl]methyl]piperazin-1-yl]-N-methylsulfonylpyridazine-3-carboxamide |
| EI13-91-02R | EI13-91 | 2I91 | 6-[4-[[4-(3-Hydroxyphenyl)naphthalen-1-yl]methyl]piperazin-1-yl]-N-methylsulfonylpyridazine-3-carboxamide |
| EI13-92-02R | EI13-92 | 2I92 | 6-[4-[[4-(5-Hydroxypyridin-3-yl)naphthalen-1-yl]methyl]piperazin-1-yl]-N-methylsulfonylpyridazine-3-carboxamide |
| EI13-93-02R | EI13-93 | 2I93 | 6-[4-[[4-(3-Hydroxyphenyl)thiophen-2-yl]methyl]piperazin-1-yl]-N-methylsulfonylpyridazine-3-carboxamide |
| EI13-94-02R | EI13-94 | 2I94 | 6-[4-[[4-(5-Hydroxypyridin-3-yl)thiophen-2-yl]methyl]piperazin-1-yl]-N-methylsulfonylpyridazine-3-carboxamide |
| EI13-95-02R | EI13-95 | 2I95 | 6-[4-[[2-[2-(3-Hydroxyphenyl)ethynyl]phenyl]methyl]piperazin-1-yl]-N-methylsulfonylpyridazine-3-carboxamide |
| EI13-96-02R | EI13-96 | 2I96 | 6-[4-[[2-[2-(5-Hydroxypyridin-3-yl)ethynyl]phenyl]methyl]piperazin-1-yl]-N-methylsulfonylpyridazine-3-carboxamide |
| EI13-97-02R | EI13-97 | 2I97 | 4-[4-[1-[4-(3-Hydroxyphenyl)phenyl]ethyl]piperazin-1-yl]-N-methylsulfonylbenzamide |
| EI13-98-02R | EI13-98 | 2I98 | 4-[4-[1-[4-(5-Hydroxypyridin-3-yl)phenyl]ethyl]piperazin-1-yl]-N-methylsulfonylbenzamide |
| EI13-99-02R | EI13-99 | 2I99 | 4-[4-[1-[2-Fluoro-4-(3-hydroxyphenyl)phenyl]ethyl]piperazin-1-yl]-N-methylsulfonylbenzamide |
| EI13-100-02R | EI13-100 | 2I100 | 4-[4-[1-[2-Fluoro-4-(5-hydroxypyridin-3-yl)phenyl]ethyl]piperazin-1-yl]-N-methylsulfonylbenzamide |

Example 4-3-12: Synthesis of mixtures mixEI14-02R to mixEI18-02R

The title mixtures shown in Table EI14 to Table EI18 were obtained by synthesis in the same manner as in Example 4-3-11 using mixture mixEI06-02R (50 mg, loading rate: 0.648 mmol/g, 0.0324 mmol) shown in Table EI06 and amine reagents (BB37 to BB38 and BB40 to BB42). Compounds contained in the obtained mixtures are shown in Table EI14 to Table EI18.

[Table 209]

[Table EI14] mixEI14-02R; Sulfonamide reagent used: BB37; Mixture No. after cleavage: mixEI14

| Resin compound No. | Compound No. after cleavage | Another name of compound after cleavage in Table EJ08 | Compound name |
|---|---|---|---|
| EI14-01-02R | EI14-01 | 2L01 | N-tert-Butylsulfonyl-6-[4-[4-(3-hydroxyphenyl)thiophene-2-carbonyl]piperazin-1-yl]pyridazine-3-carboxamide |
| EI14-02-02R | EI14-02 | 2L02 | N-tert-Butylsulfonyl-6-[4-[4-(5-hydroxypyridin-3-yl)thiophene-2-carbonyl]piperazin-1-yl]pyridazine-3-carboxamide |
| EI14-03-02R | EI14-03 | 2L03 | N-tert-Butylsulfonyl-6-[4-[4-(3-hydroxyphenyl)-3-methylbenzoyl]piperazin-1-yl]pyridazine-3-carboxamide |
| EI14-04-02R | EI14-04 | 2L04 | N-tert-Butylsulfonyl-6-[4-[4-(5-hydroxypyridin-3-yl)-3-methylbenzoyl]piperazin-1-yl]pyridazine-3-carboxamide |
| EI14-05-02R | EI14-05 | 2L05 | N-tert-Butylsulfonyl-6-[4-[3-fluoro-4-(3-hydroxyphenyl)benzoyl]piperazin-1-yl]pyridazine-3-carboxamide |
| EI14-06-02R | EI14-06 | 2L06 | N-tert-Butylsulfonyl-6-[4-[3-fluoro-4-(5-hydroxypyridin-3-yl)benzoyl]piperazin-1-yl]pyridazine-3-carboxamide |
| EI14-07-02R | EI14-07 | 2L07 | N-tert-Butylsulfonyl-6-[4-[2-[2-(3-hydroxyphenyl)ethynyl]benzoyl]piperazin-1-yl]pyridazine-3-carboxamide |
| EI14-08-02R | EI14-08 | 2L08 | N-tert-Butylsulfonyl-6-[4-[2-[2-(5-hydroxypyridin-3-yl)ethynyl]benzoyl]piperazin-1-yl]pyridazine-3-carboxamide |
| EI14-09-02R | EI14-09 | 2L09 | N-tert-Butylsulfonyl-6-[4-[5-[2-(3-hydroxyphenyl)ethynyl]pyridine-3-carbonyl]piperazin-1-yl]pyridazine-3-carboxamide |
| EI14-10-02R | EI14-10 | 2L10 | N-tert-Butylsulfonyl-6-[4-[5-[2-(5-hydroxypyridin-3-yl)ethynyl]pyridine-3-carbonyl]piperazin-1-yl]pyridazine-3-carboxamide |
| EI14-11-02R | EI14-11 | 2L11 | N-tert-Butylsulfonyl-6-[4-[3-[2-(3-hydroxyphenyl)phenyl]propanoyl]piperazin-1-yl]pyridazine-3-carboxamide |
| EI14-12-02R | EI14-12 | 2L12 | N-tert-Butylsulfonyl-6-[4-[3-[2-(5-hydroxypyridin-3-yl)phenyl]propanoyl]piperazin-1-yl]pyridazine-3-carboxamide |
| EI14-13-02R | EI14-13 | 2L13 | N-tert-Butylsulfonyl-6-[4-[4-(3-hydroxyphenyl)naphthalene-1-carbonyl]piperazin-1-yl]pyridazine-3-carboxamide |
| EI14-14-02R | EI14-14 | 2L14 | N-tert-Butylsulfonyl-6-[4-[4-(5-hydroxypyridin-3-yl)naphthalene-1-carbonyl]piperazin-1-yl]pyridazine-3-carboxamide |
| EI14-15-02R | EI14-15 | 2L15 | 6-[4-[3-tert-Butyl-5-(3-hydroxyphenyl)benzoyl]piperazin-1-yl]-N-tert-butylsulfonylpyridazine-3-carboxamide |
| EI14-16-02R | EI14-16 | 2L16 | 6-[4-[3-tert-Butyl-5-(5-hydroxypyridin-3-yl)benzoyl]piperazin-1-yl]-N-tert-butylsulfonylpyridazine-3-carboxamide |
| EI14-17-02R | EI14-17 | 2L17 | N-tert-Butylsulfonyl-6-[4-[3-(3-hydroxyphenyl)-5-(trifluoromethyl)benzoyl]piperazin-1-yl]pyridazine-3-carboxamide |
| EI14-18-02R | EI14-18 | 2L18 | N-tert-Butylsulfonyl-6-[4-[3-(5-hydroxypyridin-3-yl)-5-(trifluoromethyl)benzoyl]piperazin-1-yl]pyridazine-3-carboxamide |
| EI14-19-02R | EI14-19 | 2L19 | N-tert-Butylsulfonyl-6-[4-[3-[2-(3-hydroxyphenyl)ethynyl]-5-(trifluoromethyl)benzoyl]piperazin-1-yl]pyridazine-3-carboxamide |

| | | | |
|---|---|---|---|
| EI14-20-02R | EI14-20 | 2L20 | N-tert-Butylsulfonyl-6-[4-[3-[2-(5-hydroxypyridin-3-yl)ethynyl]-5-(trifluoromethyl)benzoyl]piperazin-1-yl]pyridazine-3-carboxamide |
| EI14-21-02R | EI14-21 | 2L21 | N-tert-Butylsulfonyl-6-[4-[3-[4-(3-hydroxyphenyl)pyrazol-1-yl]-5-(trifluoromethyl)benzoyl]piperazin-1-yl]pyridazine-3-carboxamide |
| EI14-22-02R | EI14-22 | 2L22 | N-tert-Butylsulfonyl-6-[4-[3-[4-(5-hydroxypyridin-3-yl)pyrazol-1-yl]-5-(trifluoromethyl)benzoyl]piperazin-1-yl]pyridazine-3-carboxamide |
| EI14-23-02R | EI14-23 | 2L23 | N-tert-Butylsulfonyl-4-[4-[4-(3-hydroxyphenyl)thiophene-2-carbonyl]piperazin-1-yl]benzamide |
| EI14-24-02R | EI14-24 | 2L24 | N-tert-Butylsulfonyl-4-[4-[4-(5-hydroxypyridin-3-yl)thiophene-2-carbonyl]piperazin-1-yl]benzamide |
| EI14-25-02R | EI14-25 | 2L25 | N-tert-Butylsulfonyl-4-[4-[4-(3-hydroxyphenyl)-3-methylbenzoyl]piperazin-1-yl]benzamide |
| EI14-26-02R | EI14-26 | 2L26 | N-tert-Butylsulfonyl-4-[4-[4-(5-hydroxypyridin-3-yl)-3-methylbenzoyl]piperazin-1-yl]benzamide |
| EI14-27-02R | EI14-27 | 2L27 | N-tert-Butylsulfonyl-4-[4-[3-fluoro-4-(3-hydroxyphenyl)benzoyl]piperazin-1-yl]benzamide |
| EI14-28-02R | EI14-28 | 2L28 | N-tert-Butylsulfonyl-4-[4-[3-fluoro-4-(5-hydroxypyridin-3-yl)benzoyl]piperazin-1-yl]benzamide |
| EI14-29-02R | EI14-29 | 2L29 | N-tert-Butylsulfonyl-4-[4-[2-[2-(3-hydroxyphenyl)ethynyl]benzoyl]piperazin-1-yl]benzamide |
| EI14-30-02R | EI14-30 | 2L30 | N-tert-Butylsulfonyl-4-[4-[2-[2-(5-hydroxypyridin-3-yl)ethynyl]benzoyl]piperazin-1-yl]benzamide |
| EI14-31-02R | EI14-31 | 2L31 | N-tert-Butylsulfonyl-4-[4-[5-[2-(3-hydroxyphenyl)ethynyl]pyridine-3-carbonyl]piperazin-1-yl]benzamide |
| EI14-32-02R | EI14-32 | 2L32 | N-tert-Butylsulfonyl-4-[4-[5-[2-(5-hydroxypyridin-3-yl)ethynyl]pyridine-3-carbonyl]piperazin-1-yl]benzamide |
| EI14-33-02R | EI14-33 | 2L33 | N-tert-Butylsulfonyl-4-[4-[3-[2-(3-hydroxyphenyl)phenyl]propanoyl]piperazin-1-yl]benzamide |
| EI14-34-02R | EI14-34 | 2L34 | N-tert-Butylsulfonyl-4-[4-[3-[2-(5-hydroxypyridin-3-yl)phenyl]propanoyl]piperazin-1-yl]benzamide |
| EI14-35-02R | EI14-35 | 2L35 | N-tert-Butylsulfonyl-4-[4-[4-(3-hydroxyphenyl)naphthalene-1-carbonyl]piperazin-1-yl]benzamide |
| EI14-36-02R | EI14-36 | 2L36 | N-tert-Butylsulfonyl-4-[4-[4-(5-hydroxypyridin-3-yl)naphthalene-1-carbonyl]piperazin-1-yl]benzamide |
| EI14-37-02R | EI14-37 | 2L37 | 4-[4-[3-tert-Butyl-5-(3-hydroxyphenyl)benzoyl]piperazin-1-yl]-N-tert-butylsulfonylbenzamide |
| EI14-38-02R | EI14-38 | 2L38 | 4-[4-[3-tert-Butyl-5-(5-hydroxypyridin-3-yl)benzoyl]piperazin-1-yl]-N-tert-butylsulfonylbenzamide |
| EI14-39-02R | EI14-39 | 2L39 | N-tert-Butylsulfonyl-4-[4-[3-(3-hydroxyphenyl)-5-(trifluoromethyl)benzoyl]piperazin-1-yl]benzamide |
| EI14-40-02R | EI14-40 | 2L40 | N-tert-Butylsulfonyl-4-[4-[3-(5-hydroxypyridin-3-yl)-5-(trifluoromethyl)benzoyl]piperazin-1-yl]benzamide |
| EI14-41-02R | EI14-41 | 2L41 | N-tert-Butylsulfonyl-4-[4-[3-[2-(3-hydroxyphenyl)ethynyl]-5-(trifluoromethyl)benzoyl]piperazin-1-yl]benzamide |
| EI14-42-02R | EI14-42 | 2L42 | N-tert-Butylsulfonyl-4-[4-[3-[2-(5-hydroxypyridin-3-yl)ethynyl]-5-(trifluoromethyl)benzoyl]piperazin-1-yl]benzamide |
| EI14-43-02R | EI14-43 | 2L43 | N-tert-Butylsulfonyl-4-[4-[3-[4-(3-hydroxyphenyl)pyrazol-1-yl]-5-(trifluoromethyl)benzoyl]piperazin-1-yl]benzamide |
| EI14-44-02R | EI14-44 | 2L44 | N-tert-Butylsulfonyl-4-[4-[3-[4-(5-hydroxypyridin-3-yl)pyrazol-1-yl]-5-(trifluoromethyl)benzoyl]piperazin-1-yl]benzamide |
| EI14-45-02R | EI14-45 | 2L45 | N-tert-Butylsulfonyl-4-[4-[[2-(3-hydroxyphenyl)phenyl]methyl]piperazin-1-yl]benzamide |
| EI14-46-02R | EI14-46 | 2L46 | N-tert-Butylsulfonyl-4-[4-[[2-(5-hydroxypyridin-3-yl)phenyl]methyl]piperazin-1-yl]benzamide |
| EI14-47-02R | EI14-47 | 2L47 | N-tert-Butylsulfonyl-4-[4-[[4-(3-hydroxyphenyl)-3-methylphenyl]methyl]piperazin-1-yl]benzamide |

| | | | |
|---|---|---|---|
| EI14-48-02R | EI14-48 | 2L48 | N-tert-Butylsulfonyl-4-[4-[[4-(5-hydroxypyridin-3-yl)-3-methylphenyl]methyl]piperazin-1-yl]benzamide |
| EI14-49-02R | EI14-49 | 2L49 | N-tert-Butylsulfonyl-4-[4-[[2-ethyl-4-(3-hydroxyphenyl)phenyl]methyl]piperazin-1-yl]benzamide |
| EI14-50-02R | EI14-50 | 2L50 | N-tert-Butylsulfonyl-4-[4-[[2-ethyl-4-(5-hydroxypyridin-3-yl)phenyl]methyl]piperazin-1-yl]benzamide |
| EI14-51-02R | EI14-51 | 2L51 | N-tert-Butylsulfonyl-4-[4-[[3-fluoro-4-(3-hydroxyphenyl)phenyl]methyl]piperazin-1-yl]benzamide |
| EI14-52-02R | EI14-52 | 2L52 | N-tert-Butylsulfonyl-4-[4-[[3-fluoro-4-(5-hydroxypyridin-3-yl)phenyl]methyl]piperazin-1-yl]benzamide |
| EI14-53-02R | EI14-53 | 2L53 | N-tert-Butylsulfonyl-4-[4-[[4-(3-hydroxyphenyl)-2-methoxyphenyl]methyl]piperazin-1-yl]benzamide |
| EI14-54-02R | EI14-54 | 2L54 | N-tert-Butylsulfonyl-4-[4-[[4-(5-hydroxypyridin-3-yl)-2-methoxyphenyl]methyl]piperazin-1-yl]benzamide |
| EI14-55-02R | EI14-55 | 2L55 | N-tert-Butylsulfonyl-4-[4-[[3-ethoxy-5-(3-hydroxyphenyl)phenyl]methyl]piperazin-1-yl]benzamide |
| EI14-56-02R | EI14-56 | 2L56 | N-tert-Butylsulfonyl-4-[4-[[3-ethoxy-5-(5-hydroxypyridin-3-yl)phenyl]methyl]piperazin-1-yl]benzamide |
| EI14-57-02R | EI14-57 | 2L57 | N-tert-Butylsulfonyl-4-[4-[[3-(3-hydroxyphenyl)-5-propan-2-yloxyphenyl]methyl]piperazin-1-yl]benzamide |
| EI14-58-02R | EI14-58 | 2L58 | N-tert-Butylsulfonyl-4-[4-[[3-(5-hydroxypyridin-3-yl)-5-propan-2-yloxyphenyl]methyl]piperazin-1-yl]benzamide |
| EI14-59-02R | EI14-59 | 2L59 | N-tert-Butylsulfonyl-4-[4-[[4-(3-hydroxyphenyl)-2,6-dimethoxyphenyl]methyl]piperazin-1-yl]benzamide |
| EI14-60-02R | EI14-60 | 2L60 | N-tert-Butylsulfonyl-4-[4-[[4-(5-hydroxypyridin-3-yl)-2,6-dimethoxyphenyl]methyl]piperazin-1-yl]benzamide |
| EI14-61-02R | EI14-61 | 2L61 | N-tert-Butylsulfonyl-4-[4-[[3-(3-hydroxyphenyl)-5-(trifluoromethyl)phenyl]methyl]piperazin-1-yl]benzamide |
| EI14-62-02R | EI14-62 | 2L62 | N-tert-Butylsulfonyl-4-[4-[[3-(5-hydroxypyridin-3-yl)-5-(trifluoromethyl)phenyl]methyl]piperazin-1-yl]benzamide |
| EI14-63-02R | EI14-63 | 2L63 | N-tert-Butylsulfonyl-4-[4-[[3-(3-hydroxyphenyl)-5-(trifluoromethoxy)phenyl]methyl]piperazin-1-yl]benzamide |
| EI14-64-02R | EI14-64 | 2L64 | N-tert-Butylsulfonyl-4-[4-[[3-(5-hydroxypyridin-3-yl)-5-(trifluoromethoxy)phenyl]methyl]piperazin-1-yl]benzamide |
| EI14-65-02R | EI14-65 | 2L65 | N-tert-Butylsulfonyl-4-[4-[[4-(3-hydroxyphenyl)naphthalen-1-yl]methyl]piperazin-1-yl]benzamide |
| EI14-66-02R | EI14-66 | 2L66 | N-tert-Butylsulfonyl-4-[4-[[4-(5-hydroxypyridin-3-yl)naphthalen-1-yl]methyl]piperazin-1-yl]benzamide |
| EI14-67-02R | EI14-67 | 2L67 | N-tert-Butylsulfonyl-4-[4-[[4-(3-hydroxyphenyl)thiophen-2-yl]methyl]piperazin-1-yl]benzamide |
| EI14-68-02R | EI14-68 | 2L68 | N-tert-Butylsulfonyl-4-[4-[[4-(5-hydroxypyridin-3-yl)thiophen-2-yl]methyl]piperazin-1-yl]benzamide |
| EI14-69-02R | EI14-69 | 2L69 | N-tert-Butylsulfonyl-4-[4-[[2-[2-(3-hydroxyphenyl)ethynyl]phenyl]methyl]piperazin-1-yl]benzamide |
| EI14-70-02R | EI14-70 | 2L70 | N-tert-Butylsulfonyl-4-[4-[[2-[2-(5-hydroxypyridin-3-yl)ethynyl]phenyl]methyl]piperazin-1-yl]benzamide |
| EI14-71-02R | EI14-71 | 2L71 | N-tert-Butylsulfonyl-6-[4-[[2-(3-hydroxyphenyl)phenyl]methyl]piperazin-1-yl]pyridazine-3-carboxamide |
| EI14-72-02R | EI14-72 | 2L72 | N-tert-Butylsulfonyl-6-[4-[[2-(5-hydroxypyridin-3-yl)phenyl]methyl]piperazin-1-yl]pyridazine-3-carboxamide |
| EI14-73-02R | EI14-73 | 2L73 | N-tert-Butylsulfonyl-6-[4-[[4-(3-hydroxyphenyl)-3-methylphenyl]methyl]piperazin-1-yl]pyridazine-3-carboxamide |
| EI14-74-02R | EI14-74 | 2L74 | N-tert-Butylsulfonyl-6-[4-[[4-(5-hydroxypyridin-3-yl)-3-methylphenyl]methyl]piperazin-1-yl]pyridazine-3-carboxamide |

| | | | |
|---|---|---|---|
| EI14-75-02R | EI14-75 | 2L75 | N-tert-Butylsulfonyl-6-[4-[[2-ethyl-4-(3-hydroxyphenyl)phenyl]methyl]piperazin-1-yl]pyridazine-3-carboxamide |
| EI14-76-02R | EI14-76 | 2L76 | N-tert-Butylsulfonyl-6-[4-[[2-ethyl-4-(5-hydroxypyridin-3-yl)phenyl]methyl]piperazin-1-yl]pyridazine-3-carboxamide |
| EI14-77-02R | EI14-77 | 2L77 | N-tert-Butylsulfonyl-6-[4-[[3-fluoro-4-(3-hydroxyphenyl)phenyl]methyl]piperazin-1-yl]pyridazine-3-carboxamide |
| EI14-78-02R | EI14-78 | 2L78 | N-tert-Butylsulfonyl-6-[4-[[3-fluoro-4-(5-hydroxypyridin-3-yl)phenyl]methyl]piperazin-1-yl]pyridazine-3-carboxamide |
| EI14-79-02R | EI14-79 | 2L79 | N-tert-Butylsulfonyl-6-[4-[[4-(3-hydroxyphenyl)-2-methoxyphenyl]methyl]piperazin-1-yl]pyridazine-3-carboxamide |
| EI14-80-02R | EI14-80 | 2L80 | N-tert-Butylsulfonyl-6-[4-[[4-(5-hydroxypyridin-3-yl)-2-methoxyphenyl]methyl]piperazin-1-yl]pyridazine-3-carboxamide |
| EI14-81-02R | EI14-81 | 2L81 | N-tert-Butylsulfonyl-6-[4-[[3-ethoxy-5-(3-hydroxyphenyl)phenyl]methyl]piperazin-1-yl]pyridazine-3-carboxamide |
| EI14-82-02R | EI14-82 | 2L82 | N-tert-Butylsulfonyl-6-[4-[[3-ethoxy-5-(5-hydroxypyridin-3-yl)phenyl]methyl]piperazin-1-yl]pyridazine-3-carboxamide |
| EI14-83-02R | EI14-83 | 2L83 | N-tert-Butylsulfonyl-6-[4-[[3-(3-hydroxyphenyl)-5-propan-2-yloxyphenyl]methyl]piperazin-1-yl]pyridazine-3-carboxamide |
| EI14-84-02R | EI14-84 | 2L84 | N-tert-Butylsulfonyl-6-[4-[[3-(5-hydroxypyridin-3-yl)-5-propan-2-yloxyphenyl]methyl]piperazin-1-yl]pyridazine-3-carboxamide |
| EI14-85-02R | EI14-85 | 2L85 | N-tert-Butylsulfonyl-6-[4-[[4-(3-hydroxyphenyl)-2,6-dimethoxyphenyl]methyl]piperazin-1-yl]pyridazine-3-carboxamide |
| EI14-86-02R | EI14-86 | 2L86 | N-tert-Butylsulfonyl-6-[4-[[4-(5-hydroxypyridin-3-yl)-2,6-dimethoxyphenyl]methyl]piperazin-1-yl]pyridazine-3-carboxamide |
| EI14-87-02R | EI14-87 | 2L87 | N-tert-Butylsulfonyl-6-[4-[[3-(3-hydroxyphenyl)-5-(trifluoromethyl)phenyl]methyl]piperazin-1-yl]pyridazine-3-carboxamide |
| EI14-88-02R | EI14-88 | 2L88 | N-tert-Butylsulfonyl-6-[4-[[3-(5-hydroxypyridin-3-yl)-5-(trifluoromethyl)phenyl]methyl]piperazin-1-yl]pyridazine-3-carboxamide |
| EI14-89-02R | EI14-89 | 2L89 | N-tert-Butylsulfonyl-6-[4-[[3-(3-hydroxyphenyl)-5-(trifluoromethoxy)phenyl]methyl]piperazin-1-yl]pyridazine-3-carboxamide |
| EI14-90-02R | EI14-90 | 2L90 | N-tert-Butylsulfonyl-6-[4-[[3-(5-hydroxypyridin-3-yl)-5-(trifluoromethoxy)phenyl]methyl]piperazin-1-yl]pyridazine-3-carboxamide |
| EI14-91-02R | EI14-91 | 2L91 | N-tert-Butylsulfonyl-6-[4-[[4-(3-hydroxyphenyl)naphthalen-1-yl]methyl]piperazin-1-yl]pyridazine-3-carboxamide |
| EI14-92-02R | EI14-92 | 2L92 | N-tert-Butylsulfonyl-6-[4-[[4-(5-hydroxypyridin-3-yl)naphthalen-1-yl]methyl]piperazin-1-yl]pyridazine-3-carboxamide |
| EI14-93-02R | EI14-93 | 2L93 | N-tert-Butylsulfonyl-6-[4-[[4-(3-hydroxyphenyl)thiophen-2-yl]methyl]piperazin-1-yl]pyridazine-3-carboxamide |
| EI14-94-02R | EI14-94 | 2L94 | N-tert-Butylsulfonyl-6-[4-[[4-(5-hydroxypyridin-3-yl)thiophen-2-yl]methyl]piperazin-1-yl]pyridazine-3-carboxamide |
| EI14-95-02R | EI14-95 | 2L95 | N-tert-Butylsulfonyl-6-[4-[[2-[2-(3-hydroxyphenyl)ethynyl]phenyl]methyl]piperazin-1-yl]pyridazine-3-carboxamide |
| EI14-96-02R | EI14-96 | 2L96 | N-tert-Butylsulfonyl-6-[4-[[2-[2-(5-hydroxypyridin-3-yl)ethynyl]phenyl]methyl]piperazin-1-yl]pyridazine-3-carboxamide |
| EI14-97-02R | EI14-97 | 2L97 | N-tert-Butylsulfonyl-4-[4-[1-[4-(3-hydroxyphenyl)phenyl]ethyl]piperazin-1-yl]benzamide |
| EI14-98-02R | EI14-98 | 2L98 | N-tert-Butylsulfonyl-4-[4-[1-[4-(5-hydroxypyridin-3-yl)phenyl]ethyl]piperazin-1-yl]benzamide |

| | | | |
|---|---|---|---|
| EI14-99-02R | EI14-99 | 2L99 | N-tert-Butylsulfonyl-4-[4-[1-[2-fluoro-4-(3-hydroxyphenyl)phenyl]ethyl]piperazin-1-yl]benzamide |
| EI14-100-02R | EI14-100 | 2L100 | N-tert-Butylsulfonyl-4-[4-[1-[2-fluoro-4-(5-hydroxypyridin-3-yl)phenyl]ethyl]piperazin-1-yl]benzamide |

[Table 210]

[Table EI15] mixEI15-02R; Sulfonamide reagent used: BB38; Mixture No. after cleavage: mixEI15

| Resin compound No. | Compound No. after cleavage | Another name of compound after cleavage in Table EJ09 | Compound name |
|---|---|---|---|
| EI15-01-02R | EI15-01 | 2K01 | 6-[4-[4-(3-Hydroxyphenyl)thiophene-2-carbonyl]piperazin-1-yl]-N-(3,3,3-trifluoropropylsulfonyl)pyridazine-3-carboxamide |
| EI15-02-02R | EI15-02 | 2K02 | 6-[4-[4-(5-Hydroxypyridin-3-yl)thiophene-2-carbonyl]piperazin-1-yl]-N-(3,3,3-trifluoropropylsulfonyl)pyridazine-3-carboxamide |
| EI15-03-02R | EI15-03 | 2K03 | 6-[4-[4-(3-Hydroxyphenyl)-3-methylbenzoyl]piperazin-1-yl]-N-(3,3,3-trifluoropropylsulfonyl)pyridazine-3-carboxamide |
| EI15-04-02R | EI15-04 | 2K04 | 6-[4-[4-(5-Hydroxypyridin-3-yl)-3-methylbenzoyl]piperazin-1-yl]-N-(3,3,3-trifluoropropylsulfonyl)pyridazine-3-carboxamide |
| EI15-05-02R | EI15-05 | 2K05 | 6-[4-[3-Fluoro-4-(3-hydroxyphenyl)benzoyl]piperazin-1-yl]-N-(3,3,3-trifluoropropylsulfonyl)pyridazine-3-carboxamide |
| EI15-06-02R | EI15-06 | 2K06 | 6-[4-[3-Fluoro-4-(5-hydroxypyridin-3-yl)benzoyl]piperazin-1-yl]-N-(3,3,3-trifluoropropylsulfonyl)pyridazine-3-carboxamide |
| EI15-07-02R | EI15-07 | 2K07 | 6-[4-[2-[2-(3-Hydroxyphenyl)ethynyl]benzoyl]piperazin-1-yl]-N-(3,3,3-trifluoropropylsulfonyl)pyridazine-3-carboxamide |
| EI15-08-02R | EI15-08 | 2K08 | 6-[4-[2-[2-(5-Hydroxypyridin-3-yl)ethynyl]benzoyl]piperazin-1-yl]-N-(3,3,3-trifluoropropylsulfonyl)pyridazine-3-carboxamide |
| EI15-09-02R | EI15-09 | 2K09 | 6-[4-[5-[2-(3-Hydroxyphenyl)ethynyl]pyridine-3-carbonyl]piperazin-1-yl]-N-(3,3,3-trifluoropropylsulfonyl)pyridazine-3-carboxamide |
| EI15-10-02R | EI15-10 | 2K10 | 6-[4-[5-[2-(5-Hydroxypyridin-3-yl)ethynyl]pyridine-3-carbonyl]piperazin-1-yl]-N-(3,3,3-trifluoropropylsulfonyl)pyridazine-3-carboxamide |
| EI15-11-02R | EI15-11 | 2K11 | 6-[4-[3-[2-(3-Hydroxyphenyl)phenyl]propanoyl]piperazin-1-yl]-N-(3,3,3-trifluoropropylsulfonyl)pyridazine-3-carboxamide |
| EI15-12-02R | EI15-12 | 2K12 | 6-[4-[3-[2-(5-Hydroxypyridin-3-yl)phenyl]propanoyl]piperazin-1-yl]-N-(3,3,3-trifluoropropylsulfonyl)pyridazine-3-carboxamide |
| EI15-13-02R | EI15-13 | 2K13 | 6-[4-[4-(3-Hydroxyphenyl)naphthalene-1-carbonyl]piperazin-1-yl]-N-(3,3,3-trifluoropropylsulfonyl)pyridazine-3-carboxamide |
| EI15-14-02R | EI15-14 | 2K14 | 6-[4-[4-(5-Hydroxypyridin-3-yl)naphthalene-1-carbonyl]piperazin-1-yl]-N-(3,3,3-trifluoropropylsulfonyl)pyridazine-3-carboxamide |
| EI15-15-02R | EI15-15 | 2K15 | 6-[4-[3-tert-Butyl-5-(3-hydroxyphenyl)benzoyl]piperazin-1-yl]-N-(3,3,3-trifluoropropylsulfonyl)pyridazine-3-carboxamide |
| EI15-16-02R | EI15-16 | 2K16 | 6-[4-[3-tert-Butyl-5-(5-hydroxypyridin-3-yl)benzoyl]piperazin-1-yl]-N-(3,3,3-trifluoropropylsulfonyl)pyridazine-3-carboxamide |
| EI15-17-02R | EI15-17 | 2K17 | 6-[4-[3-(3-Hydroxyphenyl)-5-(trifluoromethyl)benzoyl]piperazin-1-yl]-N-(3,3,3-trifluoropropylsulfonyl)pyridazine-3-carboxamide |
| EI15-18-02R | EI15-18 | 2K18 | 6-[4-[3-(5-Hydroxypyridin-3-yl)-5-(trifluoromethyl)benzoyl]piperazin-1-yl]-N-(3,3,3-trifluoropropylsulfonyl)pyridazine-3-carboxamide |

| | | | |
|---|---|---|---|
| EI15-19-02R | EI15-19 | 2K19 | 6-[4-[3-[2-(3-Hydroxyphenyl)ethynyl]-5-(trifluoromethyl)benzoyl]piperazin-1-yl]-N-(3,3,3-trifluoropropylsulfonyl)pyridazine-3-carboxamide |
| EI15-20-02R | EI15-20 | 2K20 | 6-[4-[3-[2-(5-Hydroxypyridin-3-yl)ethynyl]-5-(trifluoromethyl)benzoyl]piperazin-1-yl]-N-(3,3,3-trifluoropropylsulfonyl)pyridazine-3-carboxamide |
| EI15-21-02R | EI15-21 | 2K21 | 6-[4-[3-[4-(3-Hydroxyphenyl)pyrazol-1-yl]-5-(trifluoromethyl)benzoyl]piperazin-1-yl]-N-(3,3,3-trifluoropropylsulfonyl)pyridazine-3-carboxamide |
| EI15-22-02R | EI15-22 | 2K22 | 6-[4-[3-[4-(5-Hydroxypyridin-3-yl)pyrazol-1-yl]-5-(trifluoromethyl)benzoyl]piperazin-1-yl]-N-(3,3,3-trifluoropropylsulfonyl)pyridazine-3-carboxamide |
| EI15-23-02R | EI15-23 | 2K23 | 4-[4-[4-(3-Hydroxyphenyl)thiophene-2-carbonyl]piperazin-1-yl]-N-(3,3,3-trifluoropropylsulfonyl)benzamide |
| EI15-24-02R | EI15-24 | 2K24 | 4-[4-[4-(5-Hydroxypyridin-3-yl)thiophene-2-carbonyl]piperazin-1-yl]-N-(3,3,3-trifluoropropylsulfonyl)benzamide |
| EI15-25-02R | EI15-25 | 2K25 | 4-[4-[4-(3-Hydroxyphenyl)-3-methylbenzoyl]piperazin-1-yl]-N-(3,3,3-trifluoropropylsulfonyl)benzamide |
| EI15-26-02R | EI15-26 | 2K26 | 4-[4-[4-(5-Hydroxypyridin-3-yl)-3-methylbenzoyl]piperazin-1-yl]-N-(3,3,3-trifluoropropylsulfonyl)benzamide |
| EI15-27-02R | EI15-27 | 2K27 | 4-[4-[3-Fluoro-4-(3-hydroxyphenyl)benzoyl]piperazin-1-yl]-N-(3,3,3-trifluoropropylsulfonyl)benzamide |
| EI15-28-02R | EI15-28 | 2K28 | 4-[4-[3-Fluoro-4-(5-hydroxypyridin-3-yl)benzoyl]piperazin-1-yl]-N-(3,3,3-trifluoropropylsulfonyl)benzamide |
| EI15-29-02R | EI15-29 | 2K29 | 4-[4-[2-[2-(3-Hydroxyphenyl)ethynyl]benzoyl]piperazin-1-yl]-N-(3,3,3-trifluoropropylsulfonyl)benzamide |
| EI15-30-02R | EI15-30 | 2K30 | 4-[4-[2-[2-(5-Hydroxypyridin-3-yl)ethynyl]benzoyl]piperazin-1-yl]-N-(3,3,3-trifluoropropylsulfonyl)benzamide |
| EI15-31-02R | EI15-31 | 2K31 | 4-[4-[5-[2-(3-Hydroxyphenyl)ethynyl]pyridine-3-carbonyl]piperazin-1-yl]-N-(3,3,3-trifluoropropylsulfonyl)benzamide |
| EI15-32-02R | EI15-32 | 2K32 | 4-[4-[5-[2-(5-Hydroxypyridin-3-yl)ethynyl]pyridine-3-carbonyl]piperazin-1-yl]-N-(3,3,3-trifluoropropylsulfonyl)benzamide |
| EI15-33-02R | EI15-33 | 2K33 | 4-[4-[3-[2-(3-Hydroxyphenyl)phenyl]propanoyl]piperazin-1-yl]-N-(3,3,3-trifluoropropylsulfonyl)benzamide |
| EI15-34-02R | EI15-34 | 2K34 | 4-[4-[3-[2-(5-Hydroxypyridin-3-yl)phenyl]propanoyl]piperazin-1-yl]-N-(3,3,3-trifluoropropylsulfonyl)benzamide |
| EI15-35-02R | EI15-35 | 2K35 | 4-[4-[4-(3-Hydroxyphenyl)naphthalene-1-carbonyl]piperazin-1-yl]-N-(3,3,3-trifluoropropylsulfonyl)benzamide |
| EI15-36-02R | EI15-36 | 2K36 | 4-[4-[4-(5-Hydroxypyridin-3-yl)naphthalene-1-carbonyl]piperazin-1-yl]-N-(3,3,3-trifluoropropylsulfonyl)benzamide |
| EI15-37-02R | EI15-37 | 2K37 | 4-[4-[3-tert-Butyl-5-(3-hydroxyphenyl)benzoyl]piperazin-1-yl]-N-(3,3,3-trifluoropropylsulfonyl)benzamide |
| EI15-38-02R | EI15-38 | 2K38 | 4-[4-[3-tert-Butyl-5-(5-hydroxypyridin-3-yl)benzoyl]piperazin-1-yl]-N-(3,3,3-trifluoropropylsulfonyl)benzamide |
| EI15-39-02R | EI15-39 | 2K39 | 4-[4-[3-(3-Hydroxyphenyl)-5-(trifluoromethyl)benzoyl]piperazin-1-yl]-N-(3,3,3-trifluoropropylsulfonyl)benzamide |
| EI15-40-02R | EI15-40 | 2K40 | 4-[4-[3-(5-Hydroxypyridin-3-yl)-5-(trifluoromethyl)benzoyl]piperazin-1-yl]-N-(3,3,3-trifluoropropylsulfonyl)benzamide |
| EI15-41-02R | EI15-41 | 2K41 | 4-[4-[3-[2-(3-Hydroxyphenyl)ethynyl]-5-(trifluoromethyl)benzoyl]piperazin-1-yl]-N-(3,3,3-trifluoropropylsulfonyl)benzamide |
| EI15-42-02R | EI15-42 | 2K42 | 4-[4-[3-[2-(5-Hydroxypyridin-3-yl)ethynyl]-5-(trifluoromethyl)benzoyl]piperazin-1-yl]-N-(3,3,3-trifluoropropylsulfonyl)benzamide |

| | | | |
|---|---|---|---|
| EI15-43-02R | EI15-43 | 2K43 | 4-[4-[3-[4-(3-Hydroxyphenyl)pyrazol-1-yl]-5-(trifluoromethyl)benzoyl]piperazin-1-yl]-N-(3,3,3-trifluoropropylsulfonyl)benzamide |
| EI15-44-02R | EI15-44 | 2K44 | 4-[4-[3-[4-(5-Hydroxypyridin-3-yl)pyrazol-1-yl]-5-(trifluoromethyl)benzoyl]piperazin-1-yl]-N-(3,3,3-trifluoropropylsulfonyl)benzamide |
| EI15-45-02R | EI15-45 | 2K45 | 4-[4-[[2-(3-Hydroxyphenyl)phenyl]methyl]piperazin-1-yl]-N-(3,3,3-trifluoropropylsulfonyl)benzamide |
| EI15-46-02R | EI15-46 | 2K46 | 4-[4-[[2-(5-Hydroxypyridin-3-yl)phenyl]methyl]piperazin-1-yl]-N-(3,3,3-trifluoropropylsulfonyl)benzamide |
| EI15-47-02R | EI15-47 | 2K47 | 4-[4-[[4-(3-Hydroxyphenyl)-3-methylphenyl]methyl]piperazin-1-yl]-N-(3,3,3-trifluoropropylsulfonyl)benzamide |
| EI15-48-02R | EI15-48 | 2K48 | 4-[4-[[4-(5-Hydroxypyridin-3-yl)-3-methylphenyl]methyl]piperazin-1-yl]-N-(3,3,3-trifluoropropylsulfonyl)benzamide |
| EI15-49-02R | EI15-49 | 2K49 | 4-[4-[[2-Ethyl-4-(3-hydroxyphenyl)phenyl]methyl]piperazin-1-yl]-N-(3,3,3-trifluoropropylsulfonyl)benzamide |
| EI15-50-02R | EI15-50 | 2K50 | 4-[4-[[2-Ethyl-4-(5-hydroxypyridin-3-yl)phenyl]methyl]piperazin-1-yl]-N-(3,3,3-trifluoropropylsulfonyl)benzamide |
| EI15-51-02R | EI15-51 | 2K51 | 4-[4-[[3-Fluoro-4-(3-hydroxyphenyl)phenyl]methyl]piperazin-1-yl]-N-(3,3,3-trifluoropropylsulfonyl)benzamide |
| EI15-52-02R | EI15-52 | 2K52 | 4-[4-[[3-Fluoro-4-(5-hydroxypyridin-3-yl)phenyl]methyl]piperazin-1-yl]-N-(3,3,3-trifluoropropylsulfonyl)benzamide |
| EI15-53-02R | EI15-53 | 2K53 | 4-[4-[[4-(3-Hydroxyphenyl)-2-methoxyphenyl]methyl]piperazin-1-yl]-N-(3,3,3-trifluoropropylsulfonyl)benzamide |
| EI15-54-02R | EI15-54 | 2K54 | 4-[4-[[4-(5-Hydroxypyridin-3-yl)-2-methoxyphenyl]methyl]piperazin-1-yl]-N-(3,3,3-trifluoropropylsulfonyl)benzamide |
| EI15-55-02R | EI15-55 | 2K55 | 4-[4-[[3-Ethoxy-5-(3-hydroxyphenyl)phenyl]methyl]piperazin-1-yl]-N-(3,3,3-trifluoropropylsulfonyl)benzamide |
| EI15-56-02R | EI15-56 | 2K56 | 4-[4-[[3-Ethoxy-5-(5-hydroxypyridin-3-yl)phenyl]methyl]piperazin-1-yl]-N-(3,3,3-trifluoropropylsulfonyl)benzamide |
| EI15-57-02R | EI15-57 | 2K57 | 4-[4-[[3-(3-Hydroxyphenyl)-5-propan-2-yloxyphenyl]methyl]piperazin-1-yl]-N-(3,3,3-trifluoropropylsulfonyl)benzamide |
| EI15-58-02R | EI15-58 | 2K58 | 4-[4-[[3-(5-Hydroxypyridin-3-yl)-5-propan-2-yloxyphenyl]methyl]piperazin-1-yl]-N-(3,3,3-trifluoropropylsulfonyl)benzamide |
| EI15-59-02R | EI15-59 | 2K59 | 4-[4-[[4-(3-Hydroxyphenyl)-2,6-dimethoxyphenyl]methyl]piperazin-1-yl]-N-(3,3,3-trifluoropropylsulfonyl)benzamide |
| EI15-60-02R | EI15-60 | 2K60 | 4-[4-[[4-(5-Hydroxypyridin-3-yl)-2,6-dimethoxyphenyl]methyl]piperazin-1-yl]-N-(3,3,3-trifluoropropylsulfonyl)benzamide |
| EI15-61-02R | EI15-61 | 2K61 | 4-[4-[[3-(3-Hydroxyphenyl)-5-(trifluoromethyl)phenyl]methyl]piperazin-1-yl]-N-(3,3,3-trifluoropropylsulfonyl)benzamide |
| EI15-62-02R | EI15-62 | 2K62 | 4-[4-[[3-(5-Hydroxypyridin-3-yl)-5-(trifluoromethyl)phenyl]methyl]piperazin-1-yl]-N-(3,3,3-trifluoropropylsulfonyl)benzamide |
| EI15-63-02R | EI15-63 | 2K63 | 4-[4-[[3-(3-Hydroxyphenyl)-5-(trifluoromethoxy)phenyl]methyl]piperazin-1-yl]-N-(3,3,3-trifluoropropylsulfonyl)benzamide |
| EI15-64-02R | EI15-64 | 2K64 | 4-[4-[[3-(5-Hydroxypyridin-3-yl)-5-(trifluoromethoxy)phenyl]methyl]piperazin-1-yl]-N-(3,3,3-trifluoropropylsulfonyl)benzamide |
| EI15-65-02R | EI15-65 | 2K65 | 4-[4-[[4-(3-Hydroxyphenyl)naphthalen-1-yl]methyl]piperazin-1-yl]-N-(3,3,3-trifluoropropylsulfonyl)benzamide |

| | | | |
|---|---|---|---|
| EI15-66-02R | EI15-66 | 2K66 | 4-[4-[[4-(5-Hydroxypyridin-3-yl)naphthalen-1-yl]methyl]piperazin-1-yl]-N-(3,3,3-trifluoropropylsulfonyl)benzamide |
| EI15-67-02R | EI15-67 | 2K67 | 4-[4-[[4-(3-Hydroxyphenyl)thiophen-2-yl]methyl]piperazin-1-yl]-N-(3,3,3-trifluoropropylsulfonyl)benzamide |
| EI15-68-02R | EI15-68 | 2K68 | 4-[4-[[4-(5-Hydroxypyridin-3-yl)thiophen-2-yl]methyl]piperazin-1-yl]-N-(3,3,3-trifluoropropylsulfonyl)benzamide |
| EI15-69-02R | EI15-69 | 2K69 | 4-[4-[[2-[2-(3-Hydroxyphenyl)ethynyl]phenyl]methyl]piperazin-1-yl]-N-(3,3,3-trifluoropropylsulfonyl)benzamide |
| EI15-70-02R | EI15-70 | 2K70 | 4-[4-[[2-[2-(5-Hydroxypyridin-3-yl)ethynyl]phenyl]methyl]piperazin-1-yl]-N-(3,3,3-trifluoropropylsulfonyl)benzamide |
| EI15-71-02R | EI15-71 | 2K71 | 6-[4-[[2-(3-Hydroxyphenyl)phenyl]methyl]piperazin-1-yl]-N-(3,3,3-trifluoropropylsulfonyl)pyridazine-3-carboxamide |
| EI15-72-02R | EI15-72 | 2K72 | 6-[4-[[2-(5-Hydroxypyridin-3-yl)phenyl]methyl]piperazin-1-yl]-N-(3,3,3-trifluoropropylsulfonyl)pyridazine-3-carboxamide |
| EI15-73-02R | EI15-73 | 2K73 | 6-[4-[[4-(3-Hydroxyphenyl)-3-methylphenyl]methyl]piperazin-1-yl]-N-(3,3,3-trifluoropropylsulfonyl)pyridazine-3-carboxamide |
| EI15-74-02R | EI15-74 | 2K74 | 6-[4-[[4-(5-Hydroxypyridin-3-yl)-3-methylphenyl]methyl]piperazin-1-yl]-N-(3,3,3-trifluoropropylsulfonyl)pyridazine-3-carboxamide |
| EI15-75-02R | EI15-75 | 2K75 | 6-[4-[[2-Ethyl-4-(3-hydroxyphenyl)phenyl]methyl]piperazin-1-yl]-N-(3,3,3-trifluoropropylsulfonyl)pyridazine-3-carboxamide |
| EI15-76-02R | EI15-76 | 2K76 | 6-[4-[[2-Ethyl-4-(5-hydroxypyridin-3-yl)phenyl]methyl]piperazin-1-yl]-N-(3,3,3-trifluoropropylsulfonyl)pyridazine-3-carboxamide |
| EI15-77-02R | EI15-77 | 2K77 | 6-[4-[[3-Fluoro-4-(3-Hydroxyphenyl)phenyl]methyl]piperazin-1-yl]-N-(3,3,3-trifluoropropylsulfonyl)pyridazine-3-carboxamide |
| EI15-78-02R | EI15-78 | 2K78 | 6-[4-[[3-Fluoro-4-(5-Hydroxypyridin-3-yl)phenyl]methyl]piperazin-1-yl]-N-(3,3,3-trifluoropropylsulfonyl)pyridazine-3-carboxamide |
| EI15-79-02R | EI15-79 | 2K79 | 6-[4-[[4-(3-Hydroxyphenyl)-2-methoxyphenyl]methyl]piperazin-1-yl]-N-(3,3,3-trifluoropropylsulfonyl)pyridazine-3-carboxamide |
| EI15-80-02R | EI15-80 | 2K80 | 6-[4-[[4-(5-Hydroxypyridin-3-yl)-2-methoxyphenyl]methyl]piperazin-1-yl]-N-(3,3,3-trifluoropropylsulfonyl)pyridazine-3-carboxamide |
| EI15-81-02R | EI15-81 | 2K81 | 6-[4-[[3-Ethoxy-5-(3-hydroxyphenyl)phenyl]methyl]piperazin-1-yl]-N-(3,3,3-trifluoropropylsulfonyl)pyridazine-3-carboxamide |
| EI15-82-02R | EI15-82 | 2K82 | 6-[4-[[3-Ethoxy-5-(5-hydroxypyridin-3-yl)phenyl]methyl]piperazin-1-yl]-N-(3,3,3-trifluoropropylsulfonyl)pyridazine-3-carboxamide |
| EI15-83-02R | EI15-83 | 2K83 | 6-[4-[[3-(3-Hydroxyphenyl)-5-propan-2-yloxyphenyl]methyl]piperazin-1-yl]-N-(3,3,3-trifluoropropylsulfonyl)pyridazine-3-carboxamide |
| EI15-84-02R | EI15-84 | 2K84 | 6-[4-[[3-(5-Hydroxypyridin-3-yl)-5-propan-2-yloxyphenyl]methyl]piperazin-1-yl]-N-(3,3,3-trifluoropropylsulfonyl)pyridazine-3-carboxamide |
| EI15-85-02R | EI15-85 | 2K85 | 6-[4-[[4-(3-Hydroxyphenyl)-2,6-dimethoxyphenyl]methyl]piperazin-1-yl]-N-(3,3,3-trifluoropropylsulfonyl)pyridazine-3-carboxamide |
| EI15-86-02R | EI15-86 | 2K86 | 6-[4-[[4-(5-Hydroxypyridin-3-yl)-2,6-dimethoxyphenyl]methyl]piperazin-1-yl]-N-(3,3,3-trifluoropropylsulfonyl)pyridazine-3-carboxamide |
| EI15-87-02R | EI15-87 | 2K87 | 6-[4-[[3-(3-Hydroxyphenyl)-5-(trifluoromethyl)phenyl]methyl]piperazin-1-yl]-N-(3,3,3-trifluoropropylsulfonyl)pyridazine-3-carboxamide |
| EI15-88-02R | EI15-88 | 2K88 | 6-[4-[[3-(5-Hydroxypyridin-3-yl)-5-(trifluoromethyl)phenyl]methyl]piperazin-1-yl]-N-(3,3,3-trifluoropropylsulfonyl)pyridazine-3-carboxamide |
| EI15-89-02R | EI15-89 | 2K89 | 6-[4-[[3-(3-Hydroxyphenyl)-5-(trifluoromethoxy)phenyl]methyl]piperazin-1-yl]-N-(3,3,3-trifluoropropylsulfonyl)pyridazine-3-carboxamide |

| | | | |
|---|---|---|---|
| EI15-90-02R | EI15-90 | 2K90 | 6-[4-[[3-(5-Hydroxypyridin-3-yl)-5-(trifluoromethoxy)phenyl]methyl]piperazin-1-yl]-N-(3,3,3-trifluoropropylsulfonyl)pyridazine-3-carboxamide |
| EI15-91-02R | EI15-91 | 2K91 | 6-[4-[[4-(3-Hydroxyphenyl)naphthalen-1-yl]methyl]piperazin-1-yl]-N-(3,3,3-trifluoropropylsulfonyl)pyridazine-3-carboxamide |
| EI15-92-02R | EI15-92 | 2K92 | 6-[4-[[4-(5-Hydroxypyridin-3-yl)naphthalen-1-yl]methyl]piperazin-1-yl]-N-(3,3,3-trifluoropropylsulfonyl)pyridazine-3-carboxamide |
| EI15-93-02R | EI15-93 | 2K93 | 6-[4-[[4-(3-Hydroxyphenyl)thiophen-2-yl]methyl]piperazin-1-yl]-N-(3,3,3-trifluoropropylsulfonyl)pyridazine-3-carboxamide |
| EI15-94-02R | EI15-94 | 2K94 | 6-[4-[[4-(5-Hydroxypyridin-3-yl)thiophen-2-yl]methyl]piperazin-1-yl]-N-(3,3,3-trifluoropropylsulfonyl)pyridazine-3-carboxamide |
| EI15-95-02R | EI15-95 | 2K95 | 6-[4-[[2-[2-(3-Hydroxyphenyl)ethynyl]phenyl]methyl]piperazin-1-yl]-N-(3,3,3-trifluoropropylsulfonyl)pyridazine-3-carboxamide |
| EI15-96-02R | EI15-96 | 2K96 | 6-[4-[[2-[2-(5-Hydroxypyridin-3-yl)ethynyl]phenyl]methyl]piperazin-1-yl]-N-(3,3,3-trifluoropropylsulfonyl)pyridazine-3-carboxamide |
| EI15-97-02R | EI15-97 | 2K97 | 4-[4-[1-[4-(3-Hydroxyphenyl)phenyl]ethyl]piperazin-1-yl]-N-(3,3,3-trifluoropropylsulfonyl)benzamide |
| EI15-98-02R | EI15-98 | 2K98 | 4-[4-[1-[4-(5-Hydroxypyridin-3-yl)phenyl]ethyl]piperazin-1-yl]-N-(3,3,3-trifluoropropylsulfonyl)benzamide |
| EI15-99-02R | EI15-99 | 2K99 | 4-[4-[1-[2-Fluoro-4-(3-hydroxyphenyl)phenyl]ethyl]piperazin-1-yl]-N-(3,3,3-trifluoropropylsulfonyl)benzamide |
| EI15-100-02R | EI15-100 | 2K100 | 4-[4-[1-[2-Fluoro-4-(5-hydroxypyridin-3-yl)phenyl]ethyl]piperazin-1-yl]-N-(3,3,3-trifluoropropylsulfonyl)benzamide |

[Table 211]

[Table EI16] mixEI16-02R; Sulfonamide reagent used: BB40; Mixture No. after cleavage: mixEI16

| Resin compound No. | Compound No. after cleavage | Another name of compound after cleavage in Table EJ10 | Compound name |
|---|---|---|---|
| EI16-01-02R | EI16-01 | 2A01 | N-(1-Adamantylmethylsulfonyl)-6-[4-[4-(3-hydroxyphenyl)thiophene-2-carbonyl]piperazin-1-yl]pyridazine-3-carboxamide |
| EI16-02-02R | EI16-02 | 2A02 | N-(1-Adamantylmethylsulfonyl)-6-[4-[4-(5-hydroxypyridin-3-yl)thiophene-2-carbonyl]piperazin-1-yl]pyridazine-3-carboxamide |
| EI16-03-02R | EI16-03 | 2A03 | N-(1-Adamantylmethylsulfonyl)-6-[4-[4-(3-hydroxyphenyl)-3-methylbenzoyl]piperazin-1-yl]pyridazine-3-carboxamide |
| EI16-04-02R | EI16-04 | 2A04 | N-(1-Adamantylmethylsulfonyl)-6-[4-[4-(5-hydroxypyridin-3-yl)-3-methylbenzoyl]piperazin-1-yl]pyridazine-3-carboxamide |
| EI16-05-02R | EI16-05 | 2A05 | N-(1-Adamantylmethylsulfonyl)-6-[4-[3-fluoro-4-(3-hydroxyphenyl)benzoyl]piperazin-1-yl]pyridazine-3-carboxamide |
| EI16-06-02R | EI16-06 | 2A06 | N-(1-Adamantylmethylsulfonyl)-6-[4-[3-fluoro-4-(5-hydroxypyridin-3-yl)benzoyl]piperazin-1-yl]pyridazine-3-carboxamide |
| EI16-07-02R | EI16-07 | 2A07 | N-(1-Adamantylmethylsulfonyl)-6-[4-[2-[2-(3-hydroxyphenyl)ethynyl]benzoyl]piperazin-1-yl]pyridazine-3-carboxamide |
| EI16-08-02R | EI16-08 | 2A08 | N-(1-Adamantylmethylsulfonyl)-6-[4-[2-[2-(5-hydroxypyridin-3-yl)ethynyl]benzoyl]piperazin-1-yl]pyridazine-3-carboxamide |

| | | | |
|---|---|---|---|
| EI16-09-02R | EI16-09 | 2A09 | N-(1-Adamantylmethylsulfonyl)-6-[4-[5-[2-(3-hydroxyphenyl)ethynyl]pyridine-3-carbonyl]piperazin-1-yl]pyridazine-3-carboxamide |
| EI16-10-02R | EI16-10 | 2A10 | N-(1-Adamantylmethylsulfonyl)-6-[4-[5-[2-(5-hydroxypyridin-3-yl)ethynyl]pyridine-3-carbonyl]piperazin-1-yl]pyridazine-3-carboxamide |
| EI16-11-02R | EI16-11 | 2A11 | N-(1-Adamantylmethylsulfonyl)-6-[4-[3-[2-(3-hydroxyphenyl)phenyl]propanoyl]piperazin-1-yl]pyridazine-3-carboxamide |
| EI16-12-02R | EI16-12 | 2A12 | N-(1-Adamantylmethylsulfonyl)-6-[4-[3-[2-(5-hydroxypyridin-3-yl)phenyl]propanoyl]piperazin-1-yl]pyridazine-3-carboxamide |
| EI16-13-02R | EI16-13 | 2A13 | N-(1-Adamantylmethylsulfonyl)-6-[4-[4-(3-hydroxyphenyl)naphthalene-1-carbonyl]piperazin-1-yl]pyridazine-3-carboxamide |
| EI16-14-02R | EI16-14 | 2A14 | N-(1-Adamantylmethylsulfonyl)-6-[4-[4-(5-hydroxypyridin-3-yl)naphthalene-1-carbonyl]piperazin-1-yl]pyridazine-3-carboxamide |
| EI16-15-02R | EI16-15 | 2A15 | N-(1-Adamantylmethylsulfonyl)-6-[4-[3-tert-butyl-5-(3-hydroxyphenyl)benzoyl]piperazin-1-yl]pyridazine-3-carboxamide |
| EI16-16-02R | EI16-16 | 2A16 | N-(1-Adamantylmethylsulfonyl)-6-[4-[3-tert-butyl-5-(5-hydroxypyridin-3-yl)benzoyl]piperazin-1-yl]pyridazine-3-carboxamide |
| EI16-17-02R | EI16-17 | 2A17 | N-(1-Adamantylmethylsulfonyl)-6-[4-[3-(3-hydroxyphenyl)-5-(trifluoromethyl)benzoyl]piperazin-1-yl]pyridazine-3-carboxamide |
| EI16-18-02R | EI16-18 | 2A18 | N-(1-Adamantylmethylsulfonyl)-6-[4-[3-(5-hydroxypyridin-3-yl)-5-(trifluoromethyl)benzoyl]piperazin-1-yl]pyridazine-3-carboxamide |
| EI16-19-02R | EI16-19 | 2A19 | N-(1-Adamantylmethylsulfonyl)-6-[4-[3-[2-(3-hydroxyphenyl)ethynyl]-5-(trifluoromethyl)benzoyl]piperazin-1-yl]pyridazine-3-carboxamide |
| EI16-20-02R | EI16-20 | 2A20 | N-(1-Adamantylmethylsulfonyl)-6-[4-[3-[2-(5-hydroxypyridin-3-yl)ethynyl]-5-(trifluoromethyl)benzoyl]piperazin-1-yl]pyridazine-3-carboxamide |
| EI16-21-02R | EI16-21 | 2A21 | N-(1-Adamantylmethylsulfonyl)-6-[4-[3-[4-(3-hydroxyphenyl)pyrazol-1-yl]-5-(trifluoromethyl)benzoyl]piperazin-1-yl]pyridazine-3-carboxamide |
| EI16-22-02R | EI16-22 | 2A22 | N-(1-Adamantylmethylsulfonyl)-6-[4-[3-[4-(5-hydroxypyridin-3-yl)pyrazol-1-yl]-5-(trifluoromethyl)benzoyl]piperazin-1-yl]pyridazine-3-carboxamide |
| EI16-23-02R | EI16-23 | 2A23 | N-(1-Adamantylmethylsulfonyl)-4-[4-[4-(3-hydroxyphenyl)thiophene-2-carbonyl]piperazin-1-yl]benzamide |
| EI16-24-02R | EI16-24 | 2A24 | N-(1-Adamantylmethylsulfonyl)-4-[4-[4-(5-hydroxypyridin-3-yl)thiophene-2-carbonyl]piperazin-1-yl]benzamide |
| EI16-25-02R | EI16-25 | 2A25 | N-(1-Adamantylmethylsulfonyl)-4-[4-[4-(3-hydroxyphenyl)-3-methylbenzoyl]piperazin-1-yl]benzamide |
| EI16-26-02R | EI16-26 | 2A26 | N-(1-Adamantylmethylsulfonyl)-4-[4-[4-(5-hydroxypyridin-3-yl)-3-methylbenzoyl]piperazin-1-yl]benzamide |
| EI16-27-02R | EI16-27 | 2A27 | N-(1-Adamantylmethylsulfonyl)-4-[4-[3-fluoro-4-(3-hydroxyphenyl)benzoyl]piperazin-1-yl]benzamide |
| EI16-28-02R | EI16-28 | 2A28 | N-(1-Adamantylmethylsulfonyl)-4-[4-[3-fluoro-4-(5-hydroxypyridin-3-yl)benzoyl]piperazin-1-yl]benzamide |
| EI16-29-02R | EI16-29 | 2A29 | N-(1-Adamantylmethylsulfonyl)-4-[4-[2-[2-(3-hydroxyphenyl)ethynyl]benzoyl]piperazin-1-yl]benzamide |
| EI16-30-02R | EI16-30 | 2A30 | N-(1-Adamantylmethylsulfonyl)-4-[4-[2-[2-(5-hydroxypyridin-3-yl)ethynyl]benzoyl]piperazin-1-yl]benzamide |
| EI16-31-02R | EI16-31 | 2A31 | N-(1-Adamantylmethylsulfonyl)-4-[4-[5-[2-(3-hydroxyphenyl)ethynyl]pyridine-3-carbonyl]piperazin-1-yl]benzamide |

| | | | |
|---|---|---|---|
| EI16-32-02R | EI16-32 | 2A32 | N-(1-Adamantylmethylsulfonyl)-4-[4-[5-[2-(5-hydroxypyridin-3-yl)ethynyl]pyridine-3-carbonyl]piperazin-1-yl]benzamide |
| EI16-33-02R | EI16-33 | 2A33 | N-(1-Adamantylmethylsulfonyl)-4-[4-[3-[2-(3-hydroxyphenyl)phenyl]propanoyl]piperazin-1-yl]benzamide |
| EI16-34-02R | EI16-34 | 2A34 | N-(1-Adamantylmethylsulfonyl)-4-[4-[3-[2-(5-hydroxypyridin-3-yl)phenyl]propanoyl]piperazin-1-yl]benzamide |
| EI16-35-02R | EI16-35 | 2A35 | N-(1-Adamantylmethylsulfonyl)-4-[4-[4-(3-hydroxyphenyl)naphthalene-1-carbonyl]piperazin-1-yl]benzamide |
| EI16-36-02R | EI16-36 | 2A36 | N-(1-Adamantylmethylsulfonyl)-4-[4-[4-(5-hydroxypyridin-3-yl)naphthalene-1-carbonyl]piperazin-1-yl]benzamide |
| EI16-37-02R | EI16-37 | 2A37 | N-(1-Adamantylmethylsulfonyl)-4-[4-[3-tert-butyl-5-(3-hydroxyphenyl)benzoyl]piperazin-1-yl]benzamide |
| EI16-38-02R | EI16-38 | 2A38 | N-(1-Adamantylmethylsulfonyl)-4-[4-[3-tert-butyl-5-(5-hydroxypyridin-3-yl)benzoyl]piperazin-1-yl]benzamide |
| EI16-39-02R | EI16-39 | 2A39 | N-(1-Adamantylmethylsulfonyl)-4-[4-[3-(3-hydroxyphenyl)-5-(trifluoromethyl)benzoyl]piperazin-1-yl]benzamide |
| EI16-40-02R | EI16-40 | 2A40 | N-(1-Adamantylmethylsulfonyl)-4-[4-[3-(5-hydroxypyridin-3-yl)-5-(trifluoromethyl)benzoyl]piperazin-1-yl]benzamide |
| EI16-41-02R | EI16-41 | 2A41 | N-(1-Adamantylmethylsulfonyl)-4-[4-[3-[2-(3-hydroxyphenyl)ethynyl]-5-(trifluoromethyl)benzoyl]piperazin-1-yl]benzamide |
| EI16-42-02R | EI16-42 | 2A42 | N-(1-Adamantylmethylsulfonyl)-4-[4-[3-[2-(5-hydroxypyridin-3-yl)ethynyl]-5-(trifluoromethyl)benzoyl]piperazin-1-yl]benzamide |
| EI16-43-02R | EI16-43 | 2A43 | N-(1-Adamantylmethylsulfonyl)-4-[4-[3-[4-(3-hydroxyphenyl)pyrazol-1-yl]-5-(trifluoromethyl)benzoyl]piperazin-1-yl]benzamide |
| EI16-44-02R | EI16-44 | 2A44 | N-(1-Adamantylmethylsulfonyl)-4-[4-[3-[4-(5-hydroxypyridin-3-yl)pyrazol-1-yl]-5-(trifluoromethyl)benzoyl]piperazin-1-yl]benzamide |
| EI16-45-02R | EI16-45 | 2A45 | N-(1-Adamantylmethylsulfonyl)-4-[4-[[2-(3-hydroxyphenyl)phenyl]methyl]piperazin-1-yl]benzamide |
| EI16-46-02R | EI16-46 | 2A46 | N-(1-Adamantylmethylsulfonyl)-4-[4-[[2-(5-hydroxypyridin-3-yl)phenyl]methyl]piperazin-1-yl]benzamide |
| EI16-47-02R | EI16-47 | 2A47 | N-(1-Adamantylmethylsulfonyl)-4-[4-[[4-(3-hydroxyphenyl)-3-methylphenyl]methyl]piperazin-1-yl]benzamide |
| EI16-48-02R | EI16-48 | 2A48 | N-(1-Adamantylmethylsulfonyl)-4-[4-[[4-(5-hydroxypyridin-3-yl)-3-methylphenyl]methyl]piperazin-1-yl]benzamide |
| EI16-49-02R | EI16-49 | 2A49 | N-(1-Adamantylmethylsulfonyl)-4-[4-[[2-ethyl-4-(3-hydroxyphenyl)phenyl]methyl]piperazin-1-yl]benzamide |
| EI16-50-02R | EI16-50 | 2A50 | N-(1-Adamantylmethylsulfonyl)-4-[4-[[2-ethyl-4-(5-hydroxypyridin-3-yl)phenyl]methyl]piperazin-1-yl]benzamide |
| EI16-51-02R | EI16-51 | 2A51 | N-(1-Adamantylmethylsulfonyl)-4-[4-[[3-fluoro-4-(3-hydroxyphenyl)phenyl]methyl]piperazin-1-yl]benzamide |
| EI16-52-02R | EI16-52 | 2A52 | N-(1-Adamantylmethylsulfonyl)-4-[4-[[3-fluoro-4-(5-hydroxypyridin-3-yl)phenyl]methyl]piperazin-1-yl]benzamide |
| EI16-53-02R | EI16-53 | 2A53 | N-(1-Adamantylmethylsulfonyl)-4-[4-[[4-(3-hydroxyphenyl)-2-methoxyphenyl]methyl]piperazin-1-yl]benzamide |
| EI16-54-02R | EI16-54 | 2A54 | N-(1-Adamantylmethylsulfonyl)-4-[4-[[4-(5-hydroxypyridin-3-yl)-2-methoxyphenyl]methyl]piperazin-1-yl]benzamide |
| EI16-55-02R | EI16-55 | 2A55 | N-(1-Adamantylmethylsulfonyl)-4-[4-[[3-ethoxy-5-(3-hydroxyphenyl)phenyl]methyl]piperazin-1-yl]benzamide |
| EI16-56-02R | EI16-56 | 2A56 | N-(1-Adamantylmethylsulfonyl)-4-[4-[[3-ethoxy-5-(5-hydroxypyridin-3-yl)phenyl]methyl]piperazin-1-yl]benzamide |
| EI16-57-02R | EI16-57 | 2A57 | N-(1-Adamantylmethylsulfonyl)-4-[4-[[3-(3-hydroxyphenyl)-5-propan-2-yloxyphenyl]methyl]piperazin-1-yl]benzamide |
| EI16-58-02R | EI16-58 | 2A58 | N-(1-Adamantylmethylsulfonyl)-4-[4-[[3-(5-hydroxypyridin-3-yl)-5-propan-2-yloxyphenyl]methyl]piperazin-1-yl]benzamide |

| | | | |
|---|---|---|---|
| EI16-59-02R | EI16-59 | 2A59 | N-(1-Adamantylmethylsulfonyl)-4-[4-[[4-(3-hydroxyphenyl)-2,6-dimethoxyphenyl]methyl]piperazin-1-yl]benzamide |
| EI16-60-02R | EI16-60 | 2A60 | N-(1-Adamantylmethylsulfonyl)-4-[4-[[4-(5-hydroxypyridin-3-yl)-2,6-dimethoxyphenyl]methyl]piperazin-1-yl]benzamide |
| EI16-61-02R | EI16-61 | 2A61 | N-(1-Adamantylmethylsulfonyl)-4-[4-[[3-(3-hydroxyphenyl)-5-(trifluoromethyl)phenyl]methyl]piperazin-1-yl]benzamide |
| EI16-62-02R | EI16-62 | 2A62 | N-(1-Adamantylmethylsulfonyl)-4-[4-[[3-(5-hydroxypyridin-3-yl)-5-(trifluoromethyl)phenyl]methyl]piperazin-1-yl]benzamide |
| EI16-63-02R | EI16-63 | 2A63 | N-(1-Adamantylmethylsulfonyl)-4-[4-[[3-(3-hydroxyphenyl)-5-(trifluoromethoxy)phenyl]methyl]piperazin-1-yl]benzamide |
| EI16-64-02R | EI16-64 | 2A64 | N-(1-Adamantylmethylsulfonyl)-4-[4-[[3-(5-hydroxypyridin-3-yl)-5-(trifluoromethoxy)phenyl]methyl]piperazin-1-yl]benzamide |
| EI16-65-02R | EI16-65 | 2A65 | N-(1-Adamantylmethylsulfonyl)-4-[4-[[4-(3-hydroxyphenyl)naphthalen-1-yl]methyl]piperazin-1-yl]benzamide |
| EI16-66-02R | EI16-66 | 2A66 | N-(1-Adamantylmethylsulfonyl)-4-[4-[[4-(5-hydroxypyridin-3-yl)naphthalen-1-yl]methyl]piperazin-1-yl]benzamide |
| EI16-67-02R | EI16-67 | 2A67 | N-(1-Adamantylmethylsulfonyl)-4-[4-[[4-(3-hydroxyphenyl)thiophen-2-yl]methyl]piperazin-1-yl]benzamide |
| EI16-68-02R | EI16-68 | 2A68 | N-(1-Adamantylmethylsulfonyl)-4-[4-[[4-(5-hydroxypyridin-3-yl)thiophen-2-yl]methyl]piperazin-1-yl]benzamide |
| EI16-69-02R | EI16-69 | 2A69 | N-(1-Adamantylmethylsulfonyl)-4-[4-[[2-[2-(3-hydroxyphenyl)ethynyl]phenyl]methyl]piperazin-1-yl]benzamide |
| EI16-70-02R | EI16-70 | 2A70 | N-(1-Adamantylmethylsulfonyl)-4-[4-[[2-[2-(5-hydroxypyridin-3-yl)ethynyl]phenyl]methyl]piperazin-1-yl]benzamide |
| EI16-71-02R | EI16-71 | 2A71 | N-(1-Adamantylmethylsulfonyl)-6-[4-[[2-(3-hydroxyphenyl)phenyl]methyl]piperazin-1-yl]pyridazine-3-carboxamide |
| EI16-72-02R | EI16-72 | 2A72 | N-(1-Adamantylmethylsulfonyl)-6-[4-[[2-(5-hydroxypyridin-3-yl)phenyl]methyl]piperazin-1-yl]pyridazine-3-carboxamide |
| EI16-73-02R | EI16-73 | 2A73 | N-(1-Adamantylmethylsulfonyl)-6-[4-[[4-(3-hydroxyphenyl)-3-methylphenyl]methyl]piperazin-1-yl]pyridazine-3-carboxamide |
| EI16-74-02R | EI16-74 | 2A74 | N-(1-Adamantylmethylsulfonyl)-6-[4-[[4-(5-hydroxypyridin-3-yl)-3-methylphenyl]methyl]piperazin-1-yl]pyridazine-3-carboxamide |
| EI16-75-02R | EI16-75 | 2A75 | N-(1-Adamantylmethylsulfonyl)-6-[4-[[2-ethyl-4-(3-hydroxyphenyl)phenyl]methyl]piperazin-1-yl]pyridazine-3-carboxamide |
| EI16-76-02R | EI16-76 | 2A76 | N-(1-Adamantylmethylsulfonyl)-6-[4-[[2-ethyl-4-(5-hydroxypyridin-3-yl)phenyl]methyl]piperazin-1-yl]pyridazine-3-carboxamide |
| EI16-77-02R | EI16-77 | 2A77 | N-(1-Adamantylmethylsulfonyl)-6-[4-[[3-fluoro-4-(3-hydroxyphenyl)phenyl]methyl]piperazin-1-yl]pyridazine-3-carboxamide |
| EI16-78-02R | EI16-78 | 2A78 | N-(1-Adamantylmethylsulfonyl)-6-[4-[[3-fluoro-4-(5-hydroxypyridin-3-yl)phenyl]methyl]piperazin-1-yl]pyridazine-3-carboxamide |
| EI16-79-02R | EI16-79 | 2A79 | N-(1-Adamantylmethylsulfonyl)-6-[4-[[4-(3-hydroxyphenyl)-2-methoxyphenyl]methyl]piperazin-1-yl]pyridazine-3-carboxamide |
| EI16-80-02R | EI16-80 | 2A80 | N-(1-Adamantylmethylsulfonyl)-6-[4-[[4-(5-hydroxypyridin-3-yl)-2-methoxyphenyl]methyl]piperazin-1-yl]pyridazine-3-carboxamide |
| EI16-81-02R | EI16-81 | 2A81 | N-(1-Adamantylmethylsulfonyl)-6-[4-[[3-ethoxy-5-(3-hydroxyphenyl)phenyl]methyl]piperazin-1-yl]pyridazine-3-carboxamide |
| EI16-82-02R | EI16-82 | 2A82 | N-(1-Adamantylmethylsulfonyl)-6-[4-[[3-ethoxy-5-(5-hydroxypyridin-3-yl)phenyl]methyl]piperazin-1-yl]pyridazine-3-carboxamide |
| EI16-83-02R | EI16-83 | 2A83 | N-(1-Adamantylmethylsulfonyl)-6-[4-[[3-(3-hydroxyphenyl)-5-propan-2-yloxyphenyl]methyl]piperazin-1-yl]pyridazine-3-carboxamide |

| | | | |
|---|---|---|---|
| EI16-84-02R | EI16-84 | 2A84 | N-(1-Adamantylmethylsulfonyl)-6-[4-[[3-(5-hydroxypyridin-3-yl)-5-propan-2-yloxyphenyl]methyl]piperazin-1-yl]pyridazine-3-carboxamide |
| EI16-85-02R | EI16-85 | 2A85 | N-(1-Adamantylmethylsulfonyl)-6-[4-[[4-(3-hydroxyphenyl)-2,6-dimethoxyphenyl]methyl]piperazin-1-yl]pyridazine-3-carboxamide |
| EI16-86-02R | EI16-86 | 2A86 | N-(1-Adamantylmethylsulfonyl)-6-[4-[[4-(5-hydroxypyridin-3-yl)-2,6-dimethoxyphenyl]methyl]piperazin-1-yl]pyridazine-3-carboxamide |
| EI16-87-02R | EI16-87 | 2A87 | N-(1-Adamantylmethylsulfonyl)-6-[4-[[3-(3-hydroxyphenyl)-5-(trifluoromethyl)phenyl]methyl]piperazin-1-yl]pyridazine-3-carboxamide |
| EI16-88-02R | EI16-88 | 2A88 | N-(1-Adamantylmethylsulfonyl)-6-[4-[[3-(5-hydroxypyridin-3-yl)-5-(trifluoromethyl)phenyl]methyl]piperazin-1-yl]pyridazine-3-carboxamide |
| EI16-89-02R | EI16-89 | 2A89 | N-(1-Adamantylmethylsulfonyl)-6-[4-[[3-(3-hydroxyphenyl)-5-(trifluoromethoxy)phenyl]methyl]piperazin-1-yl]pyridazine-3-carboxamide |
| EI16-90-02R | EI16-90 | 2A90 | N-(1-Adamantylmethylsulfonyl)-6-[4-[[3-(5-hydroxypyridin-3-yl)-5-(trifluoromethoxy)phenyl]methyl]piperazin-1-yl]pyridazine-3-carboxamide |
| EI16-91-02R | EI16-91 | 2A91 | N-(1-Adamantylmethylsulfonyl)-6-[4-[[4-(3-hydroxyphenyl)naphthalen-1-yl]methyl]piperazin-1-yl]pyridazine-3-carboxamide |
| EI16-92-02R | EI16-92 | 2A92 | N-(1-Adamantylmethylsulfonyl)-6-[4-[[4-(5-hydroxypyridin-3-yl)naphthalen-1-yl]methyl]piperazin-1-yl]pyridazine-3-carboxamide |
| EI16-93-02R | EI16-93 | 2A93 | N-(1-Adamantylmethylsulfonyl)-6-[4-[[4-(3-hydroxyphenyl)thiophen-2-yl]methyl]piperazin-1-yl]pyridazine-3-carboxamide |
| EI16-94-02R | EI16-94 | 2A94 | N-(1-Adamantylmethylsulfonyl)-6-[4-[[4-(5-hydroxypyridin-3-yl)thiophen-2-yl]methyl]piperazin-1-yl]pyridazine-3-carboxamide |
| EI16-95-02R | EI16-95 | 2A95 | N-(1-Adamantylmethylsulfonyl)-6-[4-[[2-[2-(3-hydroxyphenyl)ethynyl]phenyl]methyl]piperazin-1-yl]pyridazine-3-carboxamide |
| EI16-96-02R | EI16-96 | 2A96 | N-(1-Adamantylmethylsulfonyl)-6-[4-[[2-[2-(5-hydroxypyridin-3-yl)ethynyl]phenyl]methyl]piperazin-1-yl]pyridazine-3-carboxamide |
| EI16-97-02R | EI16-97 | 2A97 | N-(1-Adamantylmethylsulfonyl)-4-[4-[1-[4-(3-hydroxyphenyl)phenyl]ethyl]piperazin-1-yl]benzamide |
| EI16-98-02R | EI16-98 | 2A98 | N-(1-Adamantylmethylsulfonyl)-4-[4-[1-[4-(5-hydroxypyridin-3-yl)phenyl]ethyl]piperazin-1-yl]benzamide |
| EI16-99-02R | EI16-99 | 2A99 | N-(1-Adamantylmethylsulfonyl)-4-[4-[1-[2-fluoro-4-(3-hydroxyphenyl)phenyl]ethyl]piperazin-1-yl]benzamide |
| EI16-100-02R | EI16-100 | 2A100 | N-(1-Adamantylmethylsulfonyl)-4-[4-[1-[2-fluoro-4-(5-hydroxypyridin-3-yl)phenyl]ethyl]piperazin-1-yl]benzamide |

[Table 212]

[Table EI17] mixEI17-02R; Sulfonamide reagent used: BB41; Mixture No. after cleavage: mixEI17

| Resin compound No. | Compound No. after cleavage | Another name of compound after cleavage in Table EJ11 | Compound name |
|---|---|---|---|

| | | | |
|---|---|---|---|
| EI17-01-02R | EI17-01 | 2Q01 | 6-[4-[4-(3-Hydroxyphenyl)thiophene-2-carbonyl]piperazin-1-yl]-N-[3-nitro-4-(2-phenylsulfanylethylamino)phenyl]sulfonylpyridazine-3-carboxamide |
| EI17-02-02R | EI17-02 | 2Q02 | 6-[4-[4-(5-Hydroxypyridin-3-yl)thiophene-2-carbonyl]piperazin-1-yl]-N-[3-nitro-4-(2-phenylsulfanylethylamino)phenyl]sulfonylpyridazine-3-carboxamide |
| EI17-03-02R | EI17-03 | 2Q03 | 6-[4-[4-(3-Hydroxyphenyl)-3-methylbenzoyl]piperazin-1-yl]-N-[3-nitro-4-(2-phenylsulfanylethylamino)phenyl]sulfonylpyridazine-3-carboxamide |
| EI17-04-02R | EI17-04 | 2Q04 | 6-[4-[4-(5-Hydroxypyridin-3-yl)-3-methylbenzoyl]piperazin-1-yl]-N-[3-nitro-4-(2-phenylsulfanylethylamino)phenyl]sulfonylpyridazine-3-carboxamide |
| EI17-05-02R | EI17-05 | 2Q05 | 6-[4-[3-Fluoro-4-(3-hydroxyphenyl)benzoyl]piperazin-1-yl]-N-[3-nitro-4-(2-phenylsulfanylethylamino)phenyl]sulfonylpyridazine-3-carboxamide |
| EI17-06-02R | EI17-06 | 2Q06 | 6-[4-[3-Fluoro-4-(5-hydroxypyridin-3-yl)benzoyl]piperazin-1-yl]-N-[3-nitro-4-(2-phenylsulfanylethylamino)phenyl]sulfonylpyridazine-3-carboxamide |
| EI17-07-02R | EI17-07 | 2Q07 | 6-[4-[2-[2-(3-Hydroxyphenyl)ethynyl]benzoyl]piperazin-1-yl]-N-[3-nitro-4-(2-phenylsulfanylethylamino)phenyl]sulfonylpyridazine-3-carboxamide |
| EI17-08-02R | EI17-08 | 2Q08 | 6-[4-[2-[2-(5-Hydroxypyridin-3-yl)ethynyl]benzoyl]piperazin-1-yl]-N-[3-nitro-4-(2-phenylsulfanylethylamino)phenyl]sulfonylpyridazine-3-carboxamide |
| EI17-09-02R | EI17-09 | 2Q09 | 6-[4-[5-[2-(3-Hydroxyphenyl)ethynyl]pyridine-3-carbonyl]piperazin-1-yl]-N-[3-nitro-4-(2-phenylsulfanylethylamino)phenyl]sulfonylpyridazine-3-carboxamide |
| EI17-10-02R | EI17-10 | 2Q10 | 6-[4-[5-[2-(5-Hydroxypyridin-3-yl)ethynyl]pyridine-3-carbonyl]piperazin-1-yl]-N-[3-nitro-4-(2-phenylsulfanylethylamino)phenyl]sulfonylpyridazine-3-carboxamide |
| EI17-11-02R | EI17-11 | 2Q11 | 6-[4-[3-[2-(3-Hydroxyphenyl)phenyl]propanoyl]piperazin-1-yl]-N-[3-nitro-4-(2-phenylsulfanylethylamino)phenyl]sulfonylpyridazine-3-carboxamide |
| EI17-12-02R | EI17-12 | 2Q12 | 6-[4-[3-[2-(5-Hydroxypyridin-3-yl)phenyl]propanoyl]piperazin-1-yl]-N-[3-nitro-4-(2-phenylsulfanylethylamino)phenyl]sulfonylpyridazine-3-carboxamide |
| EI17-13-02R | EI17-13 | 2Q13 | 6-[4-[4-(3-Hydroxyphenyl)naphthalene-1-carbonyl]piperazin-1-yl]-N-[3-nitro-4-(2-phenylsulfanylethylamino)phenyl]sulfonylpyridazine-3-carboxamide |
| EI17-14-02R | EI17-14 | 2Q14 | 6-[4-[4-(5-Hydroxypyridin-3-yl)naphthalene-1-carbonyl]piperazin-1-yl]-N-[3-nitro-4-(2-phenylsulfanylethylamino)phenyl]sulfonylpyridazine-3-carboxamide |
| EI17-15-02R | EI17-15 | 2Q15 | 6-[4-[3-tert-Butyl-5-(3-hydroxyphenyl)benzoyl]piperazin-1-yl]-N-[3-nitro-4-(2-phenylsulfanylethylamino)phenyl]sulfonylpyridazine-3-carboxamide |
| EI17-16-02R | EI17-16 | 2Q16 | 6-[4-[3-tert-Butyl-5-(5-hydroxypyridin-3-yl)benzoyl]piperazin-1-yl]-N-[3-nitro-4-(2-phenylsulfanylethylamino)phenyl]sulfonylpyridazine-3-carboxamide |
| EI17-17-02R | EI17-17 | 2Q17 | 6-[4-[3-(3-Hydroxyphenyl)-5-(trifluoromethyl)benzoyl]piperazin-1-yl]-N-[3-nitro-4-(2-phenylsulfanylethylamino)phenyl]sulfonylpyridazine-3-carboxamide |
| EI17-18-02R | EI17-18 | 2Q18 | 6-[4-[3-(5-Hydroxypyridin-3-yl)-5-(trifluoromethyl)benzoyl]piperazin-1-yl]-N-[3-nitro-4-(2-phenylsulfanylethylamino)phenyl]sulfonylpyridazine-3-carboxamide |
| EI17-19-02R | EI17-19 | 2Q19 | 6-[4-[3-[2-(3-Hydroxyphenyl)ethynyl]-5-(trifluoromethyl)benzoyl]piperazin-1-yl]-N-[3-nitro-4-(2-phenylsulfanylethylamino)phenyl]sulfonylpyridazine-3-carboxamide |

| | | | |
|---|---|---|---|
| EI17-20-02R | EI17-20 | 2Q20 | 6-[4-[3-[2-(5-Hydroxypyridin-3-yl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| EI17-21-02R | EI17-21 | 2Q21 | 6-[4-[3-[4-(3-Hydroxyphenyl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| EI17-22-02R | EI17-22 | 2Q22 | 6-[4-[3-[4-(5-Hydroxypyridin-3-yl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| EI17-23-02R | EI17-23 | 2Q23 | 4-[4-[4-(3-Hydroxyphenyl)thiophene-2-carbonyl]piperazin-1-yl]-N-[3-nitro-4-(2-phenylsulfanylethylamino)phenyl]sulfonylbenzamide |
| EI17-24-02R | EI17-24 | 2Q24 | 4-[4-[4-(5-Hydroxypyridin-3-yl)thiophene-2-carbonyl]piperazin-1-yl]-N-[3-nitro-4-(2-phenylsulfanylethylamino)phenyl]sulfonylbenzamide |
| EI17-25-02R | EI17-25 | 2Q25 | 4-[4-[4-(3-Hydroxyphenyl)-3-methylbenzoyl]piperazin-1-yl]-N-[3-nitro-4-(2-phenylsulfanylethylamino)phenyl]sulfonylbenzamide |
| EI17-26-02R | EI17-26 | 2Q26 | 4-[4-[4-(5-Hydroxypyridin-3-yl)-3-methylbenzoyl]piperazin-1-yl]-N-[3-nitro-4-(2-phenylsulfanylethylamino)phenyl]sulfonylbenzamide |
| EI17-27-02R | EI17-27 | 2Q27 | 4-[4-[3-Fluoro-4-(3-hydroxyphenyl)benzoyl]piperazin-1-yl]-N-[3-nitro-4-(2-phenylsulfanylethylamino)phenyl]sulfonylbenzamide |
| EI17-28-02R | EI17-28 | 2Q28 | 4-[4-[3-Fluoro-4-(5-hydroxypyridin-3-yl)benzoyl]piperazin-1-yl]-N-[3-nitro-4-(2-phenylsulfanylethylamino)phenyl]sulfonylbenzamide |
| EI17-29-02R | EI17-29 | 2Q29 | 4-[4-[2-[2-(3-Hydroxyphenyl)ethynyl]benzoyl]piperazin-1-yl]-N-[3-nitro-4-(2-phenylsulfanylethylamino)phenyl]sulfonylbenzamide |
| EI17-30-02R | EI17-30 | 2Q30 | 4-[4-[2-[2-(5-Hydroxypyridin-3-yl)ethynyl]benzoyl]piperazin-1-yl]-N-[3-nitro-4-(2-phenylsulfanylethylamino)phenyl]sulfonylbenzamide |
| EI17-31-02R | EI17-31 | 2Q31 | 4-[4-[5-[2-(3-Hydroxyphenyl)ethynyl]pyridine-3-carbonyl]piperazin-1-yl]-N-[3-nitro-4-(2-phenylsulfanylethylamino)phenyl]sulfonylbenzamide |
| EI17-32-02R | EI17-32 | 2Q32 | 4-[4-[5-[2-(5-Hydroxypyridin-3-yl)ethynyl]pyridine-3-carbonyl]piperazin-1-yl]-N-[3-nitro-4-(2-phenylsulfanylethylamino)phenyl]sulfonylbenzamide |
| EI17-33-02R | EI17-33 | 2Q33 | 4-[4-[3-[2-(3-Hydroxyphenyl)phenyl]propanoyl]piperazin-1-yl]-N-[3-nitro-4-(2-phenylsulfanylethylamino)phenyl]sulfonylbenzamide |
| EI17-34-02R | EI17-34 | 2Q34 | 4-[4-[3-[2-(5-Hydroxypyridin-3-yl)phenyl]propanoyl]piperazin-1-yl]-N-[3-nitro-4-(2-phenylsulfanylethylamino)phenyl]sulfonylbenzamide |
| EI17-35-02R | EI17-35 | 2Q35 | 4-[4-[4-(3-Hydroxyphenyl)naphthalene-1-carbonyl]piperazin-1-yl]-N-[3-nitro-4-(2-phenylsulfanylethylamino)phenyl]sulfonylbenzamide |
| EI17-36-02R | EI17-36 | 2Q36 | 4-[4-[4-(5-Hydroxypyridin-3-yl)naphthalene-1-carbonyl]piperazin-1-yl]-N-[3-nitro-4-(2-phenylsulfanylethylamino)phenyl]sulfonylbenzamide |
| EI17-37-02R | EI17-37 | 2Q37 | 4-[4-[3-tert-Butyl-5-(3-hydroxyphenyl)benzoyl]piperazin-1-yl]-N-[3-nitro-4-(2-phenylsulfanylethylamino)phenyl]sulfonylbenzamide |
| EI17-38-02R | EI17-38 | 2Q38 | 4-[4-[3-tert-Butyl-5-(5-hydroxypyridin-3-yl)benzoyl]piperazin-1-yl]-N-[3-nitro-4-(2-phenylsulfanylethylamino)phenyl]sulfonylbenzamide |
| EI17-39-02R | EI17-39 | 2Q39 | 4-[4-[3-(3-Hydroxyphenyl)-5-(trifluoromethyl)benzoyl]piperazin-1-yl]-N-[3-nitro-4-(2-phenylsulfanylethylamino)phenyl]sulfonylbenzamide |
| EI17-40-02R | EI17-40 | 2Q40 | 4-[4-[3-(5-Hydroxypyridin-3-yl)-5-(trifluoromethyl)benzoyl]piperazin-1-yl]-N-[3-nitro-4-(2-phenylsulfanylethylamino)phenyl]sulfonylbenzamide |

| | | | |
|---|---|---|---|
| EI17-41-02R | EI17-41 | 2Q41 | 4-[4-[3-[2-(3-Hydroxyphenyl)ethynyl]-5-(trifluoromethyl)benzoyl]piperazin-1-yl]-N-[3-nitro-4-(2-phenylsulfanylethylamino)phenyl]sulfonylbenzamide |
| EI17-42-02R | EI17-42 | 2Q42 | 4-[4-[3-[2-(5-Hydroxypyridin-3-yl)ethynyl]-5-(trifluoromethyl)benzoyl]piperazin-1-yl]-N-[3-nitro-4-(2-phenylsulfanylethylamino)phenyl]sulfonylbenzamide |
| EI17-43-02R | EI17-43 | 2Q43 | 4-[4-[3-[4-(3-Hydroxyphenyl)pyrazol-1-yl]-5-(trifluoromethyl)benzoyl]piperazin-1-yl]-N-[3-nitro-4-(2-phenylsulfanylethylamino)phenyl]sulfonylbenzamide |
| EI17-44-02R | EI17-44 | 2Q44 | 4-[4-[3-[4-(5-Hydroxypyridin-3-yl)pyrazol-1-yl]-5-(trifluoromethyl)benzoyl]piperazin-1-yl]-N-[3-nitro-4-(2-phenylsulfanylethylamino)phenyl]sulfonylbenzamide |
| EI17-45-02R | EI17-45 | 2Q45 | 4-[4-[[2-(3-Hydroxyphenyl)phenyl]methyl]piperazin-1-yl]-N-[3-nitro-4-(2-phenylsulfanylethylamino)phenyl]sulfonylbenzamide |
| EI17-46-02R | EI17-46 | 2Q46 | 4-[4-[[2-(5-Hydroxypyridin-3-yl)phenyl]methyl]piperazin-1-yl]-N-[3-nitro-4-(2-phenylsulfanylethylamino)phenyl]sulfonylbenzamide |
| EI17-47-02R | EI17-47 | 2Q47 | 4-[4-[[4-(3-Hydroxyphenyl)-3-methylphenyl]methyl]piperazin-1-yl]-N-[3-nitro-4-(2-phenylsulfanylethylamino)phenyl]sulfonylbenzamide |
| EI17-48-02R | EI17-48 | 2Q48 | 4-[4-[[4-(5-Hydroxypyridin-3-yl)-3-methylphenyl]methyl]piperazin-1-yl]-N-[3-nitro-4-(2-phenylsulfanylethylamino)phenyl]sulfonylbenzamide |
| EI17-49-02R | EI17-49 | 2Q49 | 4-[4-[[2-Ethyl-4-(3-hydroxyphenyl)phenyl]methyl]piperazin-1-yl]-N-[3-nitro-4-(2-phenylsulfanylethylamino)phenyl]sulfonylbenzamide |
| EI17-50-02R | EI17-50 | 2Q50 | 4-[4-[[2-Ethyl-4-(5-hydroxypyridin-3-yl)phenyl]methyl]piperazin-1-yl]-N-[3-nitro-4-(2-phenylsulfanylethylamino)phenyl]sulfonylbenzamide |
| EI17-51-02R | EI17-51 | 2Q51 | 4-[4-[[3-Fluoro-4-(3-hydroxyphenyl)phenyl]methyl]piperazin-1-yl]-N-[3-nitro-4-(2-phenylsulfanylethylamino)phenyl]sulfonylbenzamide |
| EI17-52-02R | EI17-52 | 2Q52 | 4-[4-[[3-Fluoro-4-(5-hydroxypyridin-3-yl)phenyl]methyl]piperazin-1-yl]-N-[3-nitro-4-(2-phenylsulfanylethylamino)phenyl]sulfonylbenzamide |
| EI17-53-02R | EI17-53 | 2Q53 | 4-[4-[[4-(3-Hydroxyphenyl)-2-methoxyphenyl]methyl]piperazin-1-yl]-N-[3-nitro-4-(2-phenylsulfanylethylamino)phenyl]sulfonylbenzamide |
| EI17-54-02R | EI17-54 | 2Q54 | 4-[4-[[4-(5-Hydroxypyridin-3-yl)-2-methoxyphenyl]methyl]piperazin-1-yl]-N-[3-nitro-4-(2-phenylsulfanylethylamino)phenyl]sulfonylbenzamide |
| EI17-55-02R | EI17-55 | 2Q55 | 4-[4-[[3-Ethoxy-5-(3-hydroxyphenyl)phenyl]methyl]piperazin-1-yl]-N-[3-nitro-4-(2-phenylsulfanylethylamino)phenyl]sulfonylbenzamide |
| EI17-56-02R | EI17-56 | 2Q56 | 4-[4-[[3-Ethoxy-5-(5-hydroxypyridin-3-yl)phenyl]methyl]piperazin-1-yl]-N-[3-nitro-4-(2-phenylsulfanylethylamino)phenyl]sulfonylbenzamide |
| EI17-57-02R | EI17-57 | 2Q57 | 4-[4-[[3-(3-Hydroxyphenyl)-5-propan-2-yloxyphenyl]methyl]piperazin-1-yl]-N-[3-nitro-4-(2-phenylsulfanylethylamino)phenyl]sulfonylbenzamide |
| EI17-58-02R | EI17-58 | 2Q58 | 4-[4-[[3-(5-Hydroxypyridin-3-yl)-5-propan-2-yloxyphenyl]methyl]piperazin-1-yl]-N-[3-nitro-4-(2-phenylsulfanylethylamino)phenyl]sulfonylbenzamide |
| EI17-59-02R | EI17-59 | 2Q59 | 4-[4-[[4-(3-Hydroxyphenyl)-2,6-dimethoxyphenyl]methyl]piperazin-1-yl]-N-[3-nitro-4-(2-phenylsulfanylethylamino)phenyl]sulfonylbenzamide |

| | | | |
|---|---|---|---|
| EI17-60-02R | EI17-60 | 2Q60 | 4-[4-[[4-(5-Hydroxypyridin-3-yl)-2,6-dimethoxyphenyl]methyl]piperazin-1-yl]-N-[3-nitro-4-(2-phenylsulfanylethylamino)phenyl]sulfonylbenzamide |
| EI17-61-02R | EI17-61 | 2Q61 | 4-[4-[[3-(3-Hydroxyphenyl)-5-(trifluoromethyl)phenyl]methyl]piperazin-1-yl]-N-[3-nitro-4-(2-phenylsulfanylethylamino)phenyl]sulfonylbenzamide |
| EI17-62-02R | EI17-62 | 2Q62 | 4-[4-[[3-(5-Hydroxypyridin-3-yl)-5-(trifluoromethyl)phenyl]methyl]piperazin-1-yl]-N-[3-nitro-4-(2-phenylsulfanylethylamino)phenyl]sulfonylbenzamide |
| EI17-63-02R | EI17-63 | 2Q63 | 4-[4-[[3-(3-Hydroxyphenyl)-5-(trifluoromethoxy)phenyl]methyl]piperazin-1-yl]-N-[3-nitro-4-(2-phenylsulfanylethylamino)phenyl]sulfonylbenzamide |
| EI17-64-02R | EI17-64 | 2Q64 | 4-[4-[[3-(5-Hydroxypyridin-3-yl)-5-(trifluoromethoxy)phenyl]methyl]piperazin-1-yl]-N-[3-nitro-4-(2-phenylsulfanylethylamino)phenyl]sulfonylbenzamide |
| EI17-65-02R | EI17-65 | 2Q65 | 4-[4-[[4-(3-Hydroxyphenyl)naphthalen-1-yl]methyl]piperazin-1-yl]-N-[3-nitro-4-(2-phenylsulfanylethylamino)phenyl]sulfonylbenzamide |
| EI17-66-02R | EI17-66 | 2Q66 | 4-[4-[[4-(5-Hydroxypyridin-3-yl)naphthalen-1-yl]methyl]piperazin-1-yl]-N-[3-nitro-4-(2-phenylsulfanylethylamino)phenyl]sulfonylbenzamide |
| EI17-67-02R | EI17-67 | 2Q67 | 4-[4-[[4-(3-Hydroxyphenyl)thiophen-2-yl]methyl]piperazin-1-yl]-N-[3-nitro-4-(2-phenylsulfanylethylamino)phenyl]sulfonylbenzamide |
| EI17-68-02R | EI17-68 | 2Q68 | 4-[4-[[4-(5-Hydroxypyridin-3-yl)thiophen-2-yl]methyl]piperazin-1-yl]-N-[3-nitro-4-(2-phenylsulfanylethylamino)phenyl]sulfonylbenzamide |
| EI17-69-02R | EI17-69 | 2Q69 | 4-[4-[[2-[2-(3-Hydroxyphenyl)ethynyl]phenyl]methyl]piperazin-1-yl]-N-[3-nitro-4-(2-phenylsulfanylethylamino)phenyl]sulfonylbenzamide |
| EI17-70-02R | EI17-70 | 2Q70 | 4-[4-[[2-[2-(5-Hydroxypyridin-3-yl)ethynyl]phenyl]methyl]piperazin-1-yl]-N-[3-nitro-4-(2-phenylsulfanylethylamino)phenyl]sulfonylbenzamide |
| EI17-71-02R | EI17-71 | 2Q71 | 6-[4-[[2-(3-Hydroxyphenyl)phenyl]methyl]piperazin-1-yl]-N-[3-nitro-4-(2-phenylsulfanylethylamino)phenyl]sulfonylpyridazine-3-carboxamide |
| EI17-72-02R | EI17-72 | 2Q72 | 6-[4-[[2-(5-Hydroxypyridin-3-yl)phenyl]methyl]piperazin-1-yl]-N-[3-nitro-4-(2-phenylsulfanylethylamino)phenyl]sulfonylpyridazine-3-carboxamide |
| EI17-73-02R | EI17-73 | 2Q73 | 6-[4-[[4-(3-Hydroxyphenyl)-3-methylphenyl]methyl]piperazin-1-yl]-N-[3-nitro-4-(2-phenylsulfanylethylamino)phenyl]sulfonylpyridazine-3-carboxamide |
| EI17-74-02R | EI17-74 | 2Q74 | 6-[4-[[4-(5-Hydroxypyridin-3-yl)-3-methylphenyl]methyl]piperazin-1-yl]-N-[3-nitro-4-(2-phenylsulfanylethylamino)phenyl]sulfonylpyridazine-3-carboxamide |
| EI17-75-02R | EI17-75 | 2Q75 | 6-[4-[[2-Ethyl-4-(3-hydroxyphenyl)phenyl]methyl]piperazin-1-yl]-N-[3-nitro-4-(2-phenylsulfanylethylamino)phenyl]sulfonylpyridazine-3-carboxamide |
| EI17-76-02R | EI17-76 | 2Q76 | 6-[4-[[2-Ethyl-4-(5-hydroxypyridin-3-yl)phenyl]methyl]piperazin-1-yl]-N-[3-nitro-4-(2-phenylsulfanylethylamino)phenyl]sulfonylpyridazine-3-carboxamide |
| EI17-77-02R | EI17-77 | 2Q77 | 6-[4-[[3-Fluoro-4-(3-hydroxyphenyl)phenyl]methyl]piperazin-1-yl]-N-[3-nitro-4-(2-phenylsulfanylethylamino)phenyl]sulfonylpyridazine-3-carboxamide |
| EI17-78-02R | EI17-78 | 2Q78 | 6-[4-[[3-Fluoro-4-(5-hydroxypyridin-3-yl)phenyl]methyl]piperazin-1-yl]-N-[3-nitro-4-(2-phenylsulfanylethylamino)phenyl]sulfonylpyridazine-3-carboxamide |

| | | | |
|---|---|---|---|
| EI17-79-02R | EI17-79 | 2Q79 | 6-[4-[[4-(3-Hydroxyphenyl)-2-methoxyphenyl]methyl]piperazin-1-yl]-N-[3-nitro-4-(2-phenylsulfanylethylamino)phenyl]sulfonylpyridazine-3-carboxamide |
| EI17-80-02R | EI17-80 | 2Q80 | 6-[4-[[4-(5-Hydroxypyridin-3-yl)-2-methoxyphenyl]methyl]piperazin-1-yl]-N-[3-nitro-4-(2-phenylsulfanylethylamino)phenyl]sulfonylpyridazine-3-carboxamide |
| EI17-81-02R | EI17-81 | 2Q81 | 6-[4-[[3-Ethoxy-5-(3-hydroxyphenyl)phenyl]methyl]piperazin-1-yl]-N-[3-nitro-4-(2-phenylsulfanylethylamino)phenyl]sulfonylpyridazine-3-carboxamide |
| EI17-82-02R | EI17-82 | 2Q82 | 6-[4-[[3-Ethoxy-5-(5-hydroxypyridin-3-yl)phenyl]methyl]piperazin-1-yl]-N-[3-nitro-4-(2-phenylsulfanylethylamino)phenyl]sulfonylpyridazine-3-carboxamide |
| EI17-83-02R | EI17-83 | 2Q83 | 6-[4-[[3-(3-Hydroxyphenyl)-5-propan-2-yloxyphenyl]methyl]piperazin-1-yl]-N-[3-nitro-4-(2-phenylsulfanylethylamino)phenyl]sulfonylpyridazine-3-carboxamide |
| EI17-84-02R | EI17-84 | 2Q84 | 6-[4-[[3-(5-Hydroxypyridin-3-yl)-5-propan-2-yloxyphenyl]methyl]piperazin-1-yl]-N-[3-nitro-4-(2-phenylsulfanylethylamino)phenyl]sulfonylpyridazine-3-carboxamide |
| EI17-85-02R | EI17-85 | 2Q85 | 6-[4-[[4-(3-Hydroxyphenyl)-2,6-dimethoxyphenyl]methyl]piperazin-1-yl]-N-[3-nitro-4-(2-phenylsulfanylethylamino)phenyl]sulfonylpyridazine-3-carboxamide |
| EI17-86-02R | EI17-86 | 2Q86 | 6-[4-[[4-(5-Hydroxypyridin-3-yl)-2,6-dimethoxyphenyl]methyl]piperazin-1-yl]-N-[3-nitro-4-(2-phenylsulfanylethylamino)phenyl]sulfonylpyridazine-3-carboxamide |
| EI17-87-02R | EI17-87 | 2Q87 | 6-[4-[[3-(3-Hydroxyphenyl)-5-(trifluoromethyl)phenyl]methyl]piperazin-1-yl]-N-[3-nitro-4-(2-phenylsulfanylethylamino)phenyl]sulfonylpyridazine-3-carboxamide |
| EI17-88-02R | EI17-88 | 2Q88 | 6-[4-[[3-(5-Hydroxypyridin-3-yl)-5-(trifluoromethyl)phenyl]methyl]piperazin-1-yl]-N-[3-nitro-4-(2-phenylsulfanylethylamino)phenyl]sulfonylpyridazine-3-carboxamide |
| EI17-89-02R | EI17-89 | 2Q89 | 6-[4-[[3-(3-Hydroxyphenyl)-5-(trifluoromethoxy)phenyl]methyl]piperazin-1-yl]-N-[3-nitro-4-(2-phenylsulfanylethylamino)phenyl]sulfonylpyridazine-3-carboxamide |
| EI17-90-02R | EI17-90 | 2Q90 | 6-[4-[[3-(5-Hydroxypyridin-3-yl)-5-(trifluoromethoxy)phenyl]methyl]piperazin-1-yl]-N-[3-nitro-4-(2-phenylsulfanylethylamino)phenyl]sulfonylpyridazine-3-carboxamide |
| EI17-91-02R | EI17-91 | 2Q91 | 6-[4-[[4-(3-Hydroxyphenyl)naphthalen-1-yl]methyl]piperazin-1-yl]-N-[3-nitro-4-(2-phenylsulfanylethylamino)phenyl]sulfonylpyridazine-3-carboxamide |
| EI17-92-02R | EI17-92 | 2Q92 | 6-[4-[[4-(5-Hydroxypyridin-3-yl)naphthalen-1-yl]methyl]piperazin-1-yl]-N-[3-nitro-4-(2-phenylsulfanylethylamino)phenyl]sulfonylpyridazine-3-carboxamide |
| EI17-93-02R | EI17-93 | 2Q93 | 6-[4-[[4-(3-Hydroxyphenyl)thiophen-2-yl]methyl]piperazin-1-yl]-N-[3-nitro-4-(2-phenylsulfanylethylamino)phenyl]sulfonylpyridazine-3-carboxamide |
| EI17-94-02R | EI17-94 | 2Q94 | 6-[4-[[4-(5-Hydroxypyridin-3-yl)thiophen-2-yl]methyl]piperazin-1-yl]-N-[3-nitro-4-(2-phenylsulfanylethylamino)phenyl]sulfonylpyridazine-3-carboxamide |
| EI17-95-02R | EI17-95 | 2Q95 | 6-[4-[[2-[2-(3-Hydroxyphenyl)ethynyl]phenyl]methyl]piperazin-1-yl]-N-[3-nitro-4-(2-phenylsulfanylethylamino)phenyl]sulfonylpyridazine-3-carboxamide |
| EI17-96-02R | EI17-96 | 2Q96 | 6-[4-[[2-[2-(5-Hydroxypyridin-3-yl)ethynyl]phenyl]methyl]piperazin-1-yl]-N-[3-nitro-4-(2-phenylsulfanylethylamino)phenyl]sulfonylpyridazine-3-carboxamide |
| EI17-97-02R | EI17-97 | 2Q97 | 4-[4-[1-[4-(3-Hydroxyphenyl)phenyl]ethyl]piperazin-1-yl]-N-[3-nitro-4-(2-phenylsulfanylethylamino)phenyl]sulfonylbenzamide |

| | | | |
|---|---|---|---|
| EI17-98-02R | EI17-98 | 2Q98 | 4-[4-[1-[4-(5-Hydroxypyridin-3-yl)phenyl]ethyl]piperazin-1-yl]-N-[3-nitro-4-(2-phenylsulfanylethylamino)phenyl]sulfonylbenzamide |
| EI17-99-02R | EI17-99 | 2Q99 | 4-[4-[1-[2-Fluoro-4-(3-hydroxyphenyl)phenyl]ethyl]piperazin-1-yl]-N-[3-nitro-4-(2-phenylsulfanylethylamino)phenyl]sulfonylbenzamide |
| EI17-100-02R | EI17-100 | 2Q100 | 4-[4-[1-[2-Fluoro-4-(5-hydroxypyridin-3-yl)phenyl]ethyl]piperazin-1-yl]-N-[3-nitro-4-(2-phenylsulfanylethylamino)phenyl]sulfonylbenzamide |

[Table 213]

[Table EI18] mixEI18-02R; Sulfonamide reagent used: BB42; Mixture No. after cleavage: mixEI18

| Resin compound No. | Compound No. after cleavage | Another name of compound after cleavage in Table EJ12 | Compound name |
|---|---|---|---|
| EI18-01-02R | EI18-01 | 2R01 | 6-[4-[4-(3-Hydroxyphenyl)thiophene-2-carbonyl]piperazin-1-yl]-N-[4-(2-phenylsulfanylethylamino)-3-(trifluoromethyl)phenyl]sulfonylpyridazine-3-carboxamide |
| EI18-02-02R | EI18-02 | 2R02 | 6-[4-[4-(5-Hydroxypyridin-3-yl)thiophene-2-carbonyl]piperazin-1-yl]-N-[4-(2-phenylsulfanylethylamino)-3-(trifluoromethyl)phenyl]sulfonylpyridazine-3-carboxamide |
| EI18-03-02R | EI18-03 | 2R03 | 6-[4-[4-(3-Hydroxyphenyl)-3-methylbenzoyl]piperazin-1-yl]-N-[4-(2-phenylsulfanylethylamino)-3-(trifluoromethyl)phenyl]sulfonylpyridazine-3-carboxamide |
| EI18-04-02R | EI18-04 | 2R04 | 6-[4-[4-(5-Hydroxypyridin-3-yl)-3-methylbenzoyl]piperazin-1-yl]-N-[4-(2-phenylsulfanylethylamino)-3-(trifluoromethyl)phenyl]sulfonylpyridazine-3-carboxamide |
| EI18-05-02R | EI18-05 | 2R05 | 6-[4-[3-Fluoro-4-(3-hydroxyphenyl)benzoyl]piperazin-1-yl]-N-[4-(2-phenylsulfanylethylamino)-3-(trifluoromethyl)phenyl]sulfonylpyridazine-3-carboxamide |
| EI18-06-02R | EI18-06 | 2R06 | 6-[4-[3-Fluoro-4-(5-hydroxypyridin-3-yl)benzoyl]piperazin-1-yl]-N-[4-(2-phenylsulfanylethylamino)-3-(trifluoromethyl)phenyl]sulfonylpyridazine-3-carboxamide |
| EI18-07-02R | EI18-07 | 2R07 | 6-[4-[2-[2-(3-Hydroxyphenyl)ethynyl]benzoyl]piperazin-1-yl]-N-[4-(2-phenylsulfanylethylamino)-3-(trifluoromethyl)phenyl]sulfonylpyridazine-3-carboxamide |
| EI18-08-02R | EI18-08 | 2R08 | 6-[4-[2-[2-(5-Hydroxypyridin-3-yl)ethynyl]benzoyl]piperazin-1-yl]-N-[4-(2-phenylsulfanylethylamino)-3-(trifluoromethyl)phenyl]sulfonylpyridazine-3-carboxamide |
| EI18-09-02R | EI18-09 | 2R09 | 6-[4-[5-[2-(3-Hydroxyphenyl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| EI18-10-02R | EI18-10 | 2R10 | 6-[4-[5-[2-(5-Hydroxypyridin-3-yl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| EI18-11-02R | EI18-11 | 2R11 | 6-[4-[3-[2-(3-Hydroxyphenyl)phenyl]propanoyl]piperazin-1-yl]-N-[4-(2-phenylsulfanylethylamino)-3-(trifluoromethyl)phenyl]sulfonylpyridazine-3-carboxamide |
| EI18-12-02R | EI18-12 | 2R12 | 6-[4-[3-[2-(5-Hydroxypyridin-3-yl)phenyl]propanoyl]piperazin-1-yl]-N-[4-(2-phenylsulfanylethylamino)-3-(trifluoromethyl)phenyl]sulfonylpyridazine-3-carboxamide |

| | | | |
|---|---|---|---|
| EI18-13-02R | EI18-13 | 2R13 | 6-[4-[4-(3-Hydroxyphenyl)naphthalene-1-carbonyl]piperazin-1-yl]-N-[4-(2-phenylsulfanylethylamino)-3-(trifluoromethyl)phenyl]sulfonylpyridazine-3-carboxamide |
| EI18-14-02R | EI18-14 | 2R14 | 6-[4-[4-(5-Hydroxypyridin-3-yl)naphthalene-1-carbonyl]piperazin-1-yl]-N-[4-(2-phenylsulfanylethylamino)-3-(trifluoromethyl)phenyl]sulfonylpyridazine-3-carboxamide |
| EI18-15-02R | EI18-15 | 2R15 | 6-[4-[3-tert-Butyl-5-(3-hydroxyphenyl)benzoyl]piperazin-1-yl]-N-[4-(2-phenylsulfanylethylamino)-3-(trifluoromethyl)phenyl]sulfonylpyridazine-3-carboxamide |
| EI18-16-02R | EI18-16 | 2R16 | 6-[4-[3-tert-Butyl-5-(5-hydroxypyridin-3-yl)benzoyl]piperazin-1-yl]-N-[4-(2-phenylsulfanylethylamino)-3-(trifluoromethyl)phenyl]sulfonylpyridazine-3-carboxamide |
| EI18-17-02R | EI18-17 | 2R17 | 6-[4-[3-(3-Hydroxyphenyl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-18-02R | EI18-18 | 2R18 | 6-[4-[3-(5-Hydroxypyridin-3-yl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-19-02R | EI18-19 | 2R19 | 6-[4-[3-[2-(3-Hydroxyphenyl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-20-02R | EI18-20 | 2R20 | 6-[4-[3-[2-(5-Hydroxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-21-02R | EI18-21 | 2R21 | 6-[4-[3-[4-(3-Hydroxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-22-02R | EI18-22 | 2R22 | 6-[4-[3-[4-(5-Hydroxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-23-02R | EI18-23 | 2R23 | 4-[4-[4-(3-Hydroxyphenyl)thiophene-2-carbonyl]piperazin-1-yl]-N-[4-(2-phenylsulfanylethylamino)-3-(trifluoromethyl)phenyl]sulfonylbenzamide |
| EI18-24-02R | EI18-24 | 2R24 | 4-[4-[4-(5-Hydroxypyridin-3-yl)thiophene-2-carbonyl]piperazin-1-yl]-N-[4-(2-phenylsulfanylethylamino)-3-(trifluoromethyl)phenyl]sulfonylbenzamide |
| EI18-25-02R | EI18-25 | 2R25 | 4-[4-[4-(3-Hydroxyphenyl)-3-methylbenzoyl]piperazin-1-yl]-N-[4-(2-phenylsulfanylethylamino)-3-(trifluoromethyl)phenyl]sulfonylbenzamide |
| EI18-26-02R | EI18-26 | 2R26 | 4-[4-[4-(5-Hydroxypyridin-3-yl)-3-methylbenzoyl]piperazin-1-yl]-N-[4-(2-phenylsulfanylethylamino)-3-(trifluoromethyl)phenyl]sulfonylbenzamide |
| EI18-27-02R | EI18-27 | 2R27 | 4-[4-[3-Fluoro-4-(3-hydroxyphenyl)benzoyl]piperazin-1-yl]-N-[4-(2-phenylsulfanylethylamino)-3-(trifluoromethyl)phenyl]sulfonylbenzamide |
| EI18-28-02R | EI18-28 | 2R28 | 4-[4-[3-Fluoro-4-(5-hydroxypyridin-3-yl)benzoyl]piperazin-1-yl]-N-[4-(2-phenylsulfanylethylamino)-3-(trifluoromethyl)phenyl]sulfonylbenzamide |
| EI18-29-02R | EI18-29 | 2R29 | 4-[4-[2-[2-(3-Hydroxyphenyl)ethynyl]benzoyl]piperazin-1-yl]-N-[4-(2-phenylsulfanylethylamino)-3-(trifluoromethyl)phenyl]sulfonylbenzamide |

| | | | |
|---|---|---|---|
| EI18-30-02R | EI18-30 | 2R30 | 4-[4-[2-[2-(5-Hydroxypyridin-3-yl)ethynyl]benzoyl]piperazin-1-yl]-N-[4-(2-phenylsulfanylethylamino)-3-(trifluoromethyl)phenyl]sulfonylbenzamide |
| EI18-31-02R | EI18-31 | 2R31 | 4-[4-[5-[2-(3-Hydroxyphenyl)ethynyl]pyridine-3-carbonyl]piperazin-1-yl]-N-[4-(2-phenylsulfanylethylamino)-3-(trifluoromethyl)phenyl]sulfonylbenzamide |
| EI18-32-02R | EI18-32 | 2R32 | 4-[4-[5-[2-(5-Hydroxypyridin-3-yl)ethynyl]pyridine-3-carbonyl]piperazin-1-yl]-N-[4-(2-phenylsulfanylethylamino)-3-(trifluoromethyl)phenyl]sulfonylbenzamide |
| EI18-33-02R | EI18-33 | 2R33 | 4-[4-[3-[2-(3-Hydroxyphenyl)phenyl]propanoyl]piperazin-1-yl]-N-[4-(2-phenylsulfanylethylamino)-3-(trifluoromethyl)phenyl]sulfonylbenzamide |
| EI18-34-02R | EI18-34 | 2R34 | 4-[4-[3-[2-(5-Hydroxypyridin-3-yl)phenyl]propanoyl]piperazin-1-yl]-N-[4-(2-phenylsulfanylethylamino)-3-(trifluoromethyl)phenyl]sulfonylbenzamide |
| EI18-35-02R | EI18-35 | 2R35 | 4-[4-[4-(3-Hydroxyphenyl)naphthalene-1-carbonyl]piperazin-1-yl]-N-[4-(2-phenylsulfanylethylamino)-3-(trifluoromethyl)phenyl]sulfonylbenzamide |
| EI18-36-02R | EI18-36 | 2R36 | 4-[4-[4-(5-Hydroxypyridin-3-yl)naphthalene-1-carbonyl]piperazin-1-yl]-N-[4-(2-phenylsulfanylethylamino)-3-(trifluoromethyl)phenyl]sulfonylbenzamide |
| EI18-37-02R | EI18-37 | 2R37 | 4-[4-[3-tert-Butyl-5-(3-hydroxyphenyl)benzoyl]piperazin-1-yl]-N-[4-(2-phenylsulfanylethylamino)-3-(trifluoromethyl)phenyl]sulfonylbenzamide |
| EI18-38-02R | EI18-38 | 2R38 | 4-[4-[3-tert-Butyl-5-(5-hydroxypyridin-3-yl)benzoyl]piperazin-1-yl]-N-[4-(2-phenylsulfanylethylamino)-3-(trifluoromethyl)phenyl]sulfonylbenzamide |
| EI18-39-02R | EI18-39 | 2R39 | 4-[4-[3-(3-Hydroxyphenyl)-5-(trifluoromethyl)benzoyl]piperazin-1-yl]-N-[4-(2-phenylsulfanylethylamino)-3-(trifluoromethyl)phenyl]sulfonylbenzamide |
| EI18-40-02R | EI18-40 | 2R40 | 4-[4-[3-(5-Hydroxypyridin-3-yl)-5-(trifluoromethyl)benzoyl]piperazin-1-yl]-N-[4-(2-phenylsulfanylethylamino)-3-(trifluoromethyl)phenyl]sulfonylbenzamide |
| EI18-41-02R | EI18-41 | 2R41 | 4-[4-[3-[2-(3-Hydroxyphenyl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| EI18-42-02R | EI18-42 | 2R42 | 4-[4-[3-[2-(5-Hydroxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| EI18-43-02R | EI18-43 | 2R43 | 4-[4-[3-[4-(3-Hydroxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |
| EI18-44-02R | EI18-44 | 2R44 | 4-[4-[3-[4-(5-Hydroxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |
| EI18-45-02R | EI18-45 | 2R45 | 4-[4-[[2-(3-Hydroxyphenyl)phenyl]methyl]piperazin-1-yl]-N-[4-(2-phenylsulfanylethylamino)-3-(trifluoromethyl)phenyl]sulfonylbenzamide |
| EI18-46-02R | EI18-46 | 2R46 | 4-[4-[[2-(5-Hydroxypyridin-3-yl)phenyl]methyl]piperazin-1-yl]-N-[4-(2-phenylsulfanylethylamino)-3-(trifluoromethyl)phenyl]sulfonylbenzamide |

| | | | |
|---|---|---|---|
| EI18-47-02R | EI18-47 | 2R47 | 4-[4-[[4-(3-Hydroxyphenyl)-3-methylphenyl]methyl]piperazin-1-yl]-N-[4-(2-phenylsulfanylethylamino)-3-(trifluoromethyl)phenyl]sulfonylbenzamide |
| EI18-48-02R | EI18-48 | 2R48 | 4-[4-[[4-(5-Hydroxypyridin-3-yl)-3-methylphenyl]methyl]piperazin-1-yl]-N-[4-(2-phenylsulfanylethylamino)-3-(trifluoromethyl)phenyl]sulfonylbenzamide |
| EI18-49-02R | EI18-49 | 2R49 | 4-[4-[[2-Ethyl-4-(3-hydroxyphenyl)phenyl]methyl]piperazin-1-yl]-N-[4-(2-phenylsulfanylethylamino)-3-(trifluoromethyl)phenyl]sulfonylbenzamide |
| EI18-50-02R | EI18-50 | 2R50 | 4-[4-[[2-Ethyl-4-(5-hydroxypyridin-3-yl)phenyl]methyl]piperazin-1-yl]-N-[4-(2-phenylsulfanylethylamino)-3-(trifluoromethyl)phenyl]sulfonylbenzamide |
| EI18-51-02R | EI18-51 | 2R51 | 4-[4-[[3-Fluoro-4-(3-hydroxyphenyl)phenyl]methyl]piperazin-1-yl]-N-[4-(2-phenylsulfanylethylamino)-3-(trifluoromethyl)phenyl]sulfonylbenzamide |
| EI18-52-02R | EI18-52 | 2R52 | 4-[4-[[3-Fluoro-4-(5-hydroxypyridin-3-yl)phenyl]methyl]piperazin-1-yl]-N-[4-(2-phenylsulfanylethylamino)-3-(trifluoromethyl)phenyl]sulfonylbenzamide |
| EI18-53-02R | EI18-53 | 2R53 | 4-[4-[[4-(3-Hydroxyphenyl)-2-methoxyphenyl]methyl]piperazin-1-yl]-N-[4-(2-phenylsulfanylethylamino)-3-(trifluoromethyl)phenyl]sulfonylbenzamide |
| EI18-54-02R | EI18-54 | 2R54 | 4-[4-[[4-(5-Hydroxypyridin-3-yl)-2-methoxyphenyl]methyl]piperazin-1-yl]-N-[4-(2-phenylsulfanylethylamino)-3-(trifluoromethyl)phenyl]sulfonylbenzamide |
| EI18-55-02R | EI18-55 | 2R55 | 4-[4-[[3-Ethoxy-5-(3-hydroxyphenyl)phenyl]methyl]piperazin-1-yl]-N-[4-(2-phenylsulfanylethylamino)-3-(trifluoromethyl)phenyl]sulfonylbenzamide |
| EI18-56-02R | EI18-56 | 2R56 | 4-[4-[[3-Ethoxy-5-(5-hydroxypyridin-3-yl)phenyl]methyl]piperazin-1-yl]-N-[4-(2-phenylsulfanylethylamino)-3-(trifluoromethyl)phenyl]sulfonylbenzamide |
| EI18-57-02R | EI18-57 | 2R57 | 4-[4-[[3-(3-Hydroxyphenyl)-5-propan-2-yloxyphenyl]methyl]piperazin-1-yl]-N-[4-(2-phenylsulfanylethylamino)-3-(trifluoromethyl)phenyl]sulfonylbenzamide |
| EI18-58-02R | EI18-58 | 2R58 | 4-[4-[[3-(5-Hydroxypyridin-3-yl)-5-propan-2-yloxyphenyl]methyl]piperazin-1-yl]-N-[4-(2-phenylsulfanylethylamino)-3-(trifluoromethyl)phenyl]sulfonylbenzamide |
| EI18-59-02R | EI18-59 | 2R59 | 4-[4-[[4-(3-Hydroxyphenyl)-2,6-dimethoxyphenyl]methyl]piperazin-1-yl]-N-[4-(2-phenylsulfanylethylamino)-3-(trifluoromethyl)phenyl]sulfonylbenzamide |
| EI18-60-02R | EI18-60 | 2R60 | 4-[4-[[4-(5-Hydroxypyridin-3-yl)-2,6-dimethoxyphenyl]methyl]piperazin-1-yl]-N-[4-(2-phenylsulfanylethylamino)-3-(trifluoromethyl)phenyl]sulfonylbenzamide |
| EI18-61-02R | EI18-61 | 2R61 | 4-[4-[[3-(3-Hydroxyphenyl)-5-(trifluoromethyl)phenyl]methyl]piperazin-1-yl]-N-[4-(2-phenylsulfanylethylamino)-3-(trifluoromethyl)phenyl]sulfonylbenzamide |
| EI18-62-02R | EI18-62 | 2R62 | 4-[4-[[3-(5-Hydroxypyridin-3-yl)-5-(trifluoromethyl)phenyl]methyl]piperazin-1-yl]-N-[4-(2-phenylsulfanylethylamino)-3-(trifluoromethyl)phenyl]sulfonylbenzamide |

| | | | |
|---|---|---|---|
| EI18-63-02R | EI18-63 | 2R63 | 4-[4-[[3-(3-Hydroxyphenyl)-5-(trifluoromethoxy)phenyl]methyl]piperazin-1-yl]-N-[4-(2-phenylsulfanylethylamino)-3-(trifluoromethyl)phenyl]sulfonylbenzamide |
| EI18-64-02R | EI18-64 | 2R64 | 4-[4-[[3-(5-Hydroxypyridin-3-yl)-5-(trifluoromethoxy)phenyl]methyl]piperazin-1-yl]-N-[4-(2-phenylsulfanylethylamino)-3-(trifluoromethyl)phenyl]sulfonylbenzamide |
| EI18-65-02R | EI18-65 | 2R65 | 4-[4-[[4-(3-Hydroxyphenyl)naphthalen-1-yl]methyl]piperazin-1-yl]-N-[4-(2-phenylsulfanylethylamino)-3-(trifluoromethyl)phenyl]sulfonylbenzamide |
| EI18-66-02R | EI18-66 | 2R66 | 4-[4-[[4-(5-Hydroxypyridin-3-yl)naphthalen-1-yl]methyl]piperazin-1-yl]-N-[4-(2-phenylsulfanylethylamino)-3-(trifluoromethyl)phenyl]sulfonylbenzamide |
| EI18-67-02R | EI18-67 | 2R67 | 4-[4-[[4-(3-Hydroxyphenyl)thiophen-2-yl]methyl]piperazin-1-yl]-N-[4-(2-phenylsulfanylethylamino)-3-(trifluoromethyl)phenyl]sulfonylbenzamide |
| EI18-68-02R | EI18-68 | 2R68 | 4-[4-[[4-(5-Hydroxypyridin-3-yl)thiophen-2-yl]methyl]piperazin-1-yl]-N-[4-(2-phenylsulfanylethylamino)-3-(trifluoromethyl)phenyl]sulfonylbenzamide |
| EI18-69-02R | EI18-69 | 2R69 | 4-[4-[[2-[2-(3-Hydroxyphenyl)ethynyl]phenyl]methyl]piperazin-1-yl]-N-[4-(2-phenylsulfanylethylamino)-3-(trifluoromethyl)phenyl]sulfonylbenzamide |
| EI18-70-02R | EI18-70 | 2R70 | 4-[4-[[2-[2-(5-Hydroxypyridin-3-yl)ethynyl]phenyl]methyl]piperazin-1-yl]-N-[4-(2-phenylsulfanylethylamino)-3-(trifluoromethyl)phenyl]sulfonylbenzamide |
| EI18-71-02R | EI18-71 | 2R71 | 6-[4-[[2-(3-Hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| EI18-72-02R | EI18-72 | 2R72 | 6-[4-[[2-(5-Hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| EI18-73-02R | EI18-73 | 2R73 | 6-[4-[[4-(3-Hydroxyphenyl)-3-methylphenyl]methyl]piperazin-1-yl]-N-[4-(2-phenylsulfanylethylamino)-3-(trifluoromethyl)phenyl]sulfonylpyridazine-3-carboxamide |
| EI18-74-02R | EI18-74 | 2R74 | 6-[4-[[4-(5-Hydroxypyridin-3-yl)-3-methylphenyl]methyl]piperazin-1-yl]-N-[4-(2-phenylsulfanylethylamino)-3-(trifluoromethyl)phenyl]sulfonylpyridazine-3-carboxamide |
| EI18-75-02R | EI18-75 | 2R75 | 6-[4-[[2-Ethyl-4-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| EI18-76-02R | EI18-76 | 2R76 | 6-[4-[[2-Ethyl-4-(5-hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| EI18-77-02R | EI18-77 | 2R77 | 6-[4-[[3-Fluoro-4-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| EI18-78-02R | EI18-78 | 2R78 | 6-[4-[[3-Fluoro-4-(5-hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| EI18-79-02R | EI18-79 | 2R79 | 6-[4-[[4-(3-Hydroxyphenyl)-2-methoxyphenyl]methyl]piperazin-1-yl]-N-[4-(2-phenylsulfanylethylamino)-3-(trifluoromethyl)phenyl]sulfonylpyridazine-3-carboxamide |

| | | | |
|---|---|---|---|
| EI18-80-02R | EI18-80 | 2R80 | 6-[4-[[4-(5-Hydroxypyridin-3-yl)-2-methoxyphenyl]methyl]piperazin-1-yl]-N-[4-(2-phenylsulfanylethylamino)-3-(trifluoromethyl)phenyl]sulfonylpyridazine-3-carboxamide |
| EI18-81-02R | EI18-81 | 2R81 | 6-[4-[[3-Ethoxy-5-(3-Hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| EI18-82-02R | EI18-82 | 2R82 | 6-[4-[[3-Ethoxy-5-(5-Hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| EI18-83-02R | EI18-83 | 2R83 | 6-[4-[[3-(3-Hydroxyphenyl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| EI18-84-02R | EI18-84 | 2R84 | 6-[4-[[3-(5-Hydroxypyridin-3-yl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| EI18-85-02R | EI18-85 | 2R85 | 6-[4-[[4-(3-Hydroxyphenyl)-2,6-dimethoxyphenyl]methyl]piperazin-1-yl]-N-[4-(2-phenylsulfanylethylamino)-3-(trifluoromethyl)phenyl]sulfonylpyridazine-3-carboxamide |
| EI18-86-02R | EI18-86 | 2R86 | 6-[4-[[4-(5-Hydroxypyridin-3-yl)-2,6-dimethoxyphenyl]methyl]piperazin-1-yl]-N-[4-(2-phenylsulfanylethylamino)-3-(trifluoromethyl)phenyl]sulfonylpyridazine-3-carboxamide |
| EI18-87-02R | EI18-87 | 2R87 | 6-[4-[[3-(3-Hydroxyphenyl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| EI18-88-02R | EI18-88 | 2R88 | 6-[4-[[3-(5-Hydroxypyridin-3-yl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| EI18-89-02R | EI18-89 | 2R89 | 6-[4-[[3-(3-Hydroxyphenyl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| EI18-90-02R | EI18-90 | 2R90 | 6-[4-[[3-(5-Hydroxypyridin-3-yl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| EI18-91-02R | EI18-91 | 2R91 | 6-[4-[[4-(3-Hydroxyphenyl)naphthalen-1-yl]methyl]piperazin-1-yl]-N-[4-(2-phenylsulfanylethylamino)-3-(trifluoromethyl)phenyl]sulfonylpyridazine-3-carboxamide |
| EI18-92-02R | EI18-92 | 2R92 | 6-[4-[[4-(5-Hydroxypyridin-3-yl)naphthalen-1-yl]methyl]piperazin-1-yl]-N-[4-(2-phenylsulfanylethylamino)-3-(trifluoromethyl)phenyl]sulfonylpyridazine-3-carboxamide |
| EI18-93-02R | EI18-93 | 2R93 | 6-[4-[[4-(3-Hydroxyphenyl)thiophen-2-yl]methyl]piperazin-1-yl]-N-[4-(2-phenylsulfanylethylamino)-3-(trifluoromethyl)phenyl]sulfonylpyridazine-3-carboxamide |
| EI18-94-02R | EI18-94 | 2R94 | 6-[4-[[4-(5-Hydroxypyridin-3-yl)thiophen-2-yl]methyl]piperazin-1-yl]-N-[4-(2-phenylsulfanylethylamino)-3-(trifluoromethyl)phenyl]sulfonylpyridazine-3-carboxamide |
| EI18-95-02R | EI18-95 | 2R95 | 6-[4-[[2-[2-(3-Hydroxyphenyl)ethynyl]phenyl]methyl]piperazin-1-yl]-N-[4-(2-phenylsulfanylethylamino)-3-(trifluoromethyl)phenyl]sulfonylpyridazine-3-carboxamide |

| | | | |
|---|---|---|---|
| EI18-96-02R | EI18-96 | 2R96 | 6-[4-[[2-[2-(5-Hydroxypyridin-3-yl)ethynyl]phenyl]methyl]piperazin-1-yl]-N-[4-(2-phenylsulfanylethylamino)-3-(trifluoromethyl)phenyl]sulfonylpyridazine-3-carboxamide |
| EI18-97-02R | EI18-97 | 2R97 | 4-[4-[1-[4-(3-Hydroxyphenyl)phenyl]ethyl]piperazin-1-yl]-N-[4-(2-phenylsulfanylethylamino)-3-(trifluoromethyl)phenyl]sulfonylbenzamide |
| EI18-98-02R | EI18-98 | 2R98 | 4-[4-[1-[4-(5-Hydroxypyridin-3-yl)phenyl]ethyl]piperazin-1-yl]-N-[4-(2-Pphenylsulfanylethylamino)-3-(trifluoromethyl)phenyl]sulfonylbenzamide |
| EI18-99-02R | EI18-99 | 2R99 | 4-[4-[1-[2-Fluoro-4-(3-hydroxyphenyl)phenyl]ethyl]piperazin-1-yl]-N-[4-(2-phenylsulfanylethylamino)-3-(trifluoromethyl)phenyl]sulfonylbenzamide |
| EI18-100-02R | EI18-100 | 2R100 | 4-[4-[1-[2-Fluoro-4-(5-hydroxypyridin-3-yl)phenyl]ethyl]piperazin-1-yl]-N-[4-(2-phenylsulfanylethylamino)-3-(trifluoromethyl)phenyl]sulfonylbenzamide |

Example 4-4-1: Synthesis of mixture mixEJ01

[Formula 545]

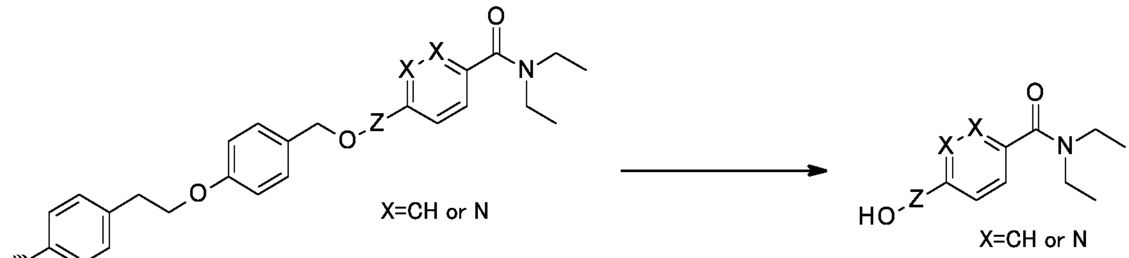


mixEI07-02R

mixEJ01

Under a nitrogen atmosphere, mixture mixEI07-02R (mixture that could contain 100 compounds, 49.2 mg, loading rate: 0.648 mmol/g, 31.9 μmol) shown in Table EI07 and 1,2,3-trimethylbenzene/DCM (0.23 M, 700 μL) were added to a 1.6 mL glass vial and shaken at room temperature for 1 hour. TFA (300 μL, 3.89 mmol) was added thereto, and the mixture was shaken at room temperature for 3 hours. The reaction solution and the resin suspension were transferred to a syringe with a filter using a DCM solution and filtered under nitrogen pressure into a test tube containing DMI (0.5 mL). This operation was repeated twice with DCM (0.5 mL), twice with DMI (0.5 mL), twice with DCM (0.5 mL), and twice with DMI (0.5 mL). The solvent in the filtrate was distilled off under reduced pressure by Genevac (40°C, reduced pressure at 50-200 mbar until the completion of distilling off of a low-boiling solvent was detected, and then, 40°C, <0.5 mbar for 16 hours) to obtain the title mixture mixEJ01 shown in Table EIJ01. The mixture was analyzed under analysis conditions SMD-FA05-long-25 min, and the UV areas were confirmed at a UV wavelength of 290 nm. The results of analyzing the mixture are shown in Table EJ01.

[Table 214]

[Table EIJ01]

| No. of mixture used | mixEI07-02R |
|---|---|
| Mixture No after cleavage | mixEJ01 |
| No. of compound in Table EJ01 | Compound name |
| 4J01 | N,N-Diethyl-6-[4-[4-(3-hydroxyphenyl)thiophene-2-carbonyl]piperazin-1-yl]pyridazine-3-carboxamide |
| 4J02 | N,N-Diethyl-6-[4-[4-(5-hydroxypyridin-3-yl)thiophene-2-carbonyl]piperazin-1-yl]pyridazine-3-carboxamide |
| 4J03 | N,N-Diethyl-6-[4-[4-(3-hydroxyphenyl)-3-methylbenzoyl]piperazin-1-yl]pyridazine-3-carboxamide |
| 4J04 | N,N-Diethyl-6-[4-[4-(5-hydroxypyridin-3-yl)-3-methylbenzoyl]piperazin-1-yl]pyridazine-3-carboxamide |
| 4J05 | N,N-Diethyl-6-[4-[3-fluoro-4-(3-hydroxyphenyl)benzoyl]piperazin-1-yl]pyridazine-3-carboxamide |
| 4J06 | N,N-Diethyl-6-[4-[3-fluoro-4-(5-hydroxypyridin-3-yl)benzoyl]piperazin-1-yl]pyridazine-3-carboxamide |
| 4J07 | N,N-Diethyl-6-[4-[2-[2-(3-hydroxyphenyl)ethynyl]benzoyl]piperazin-1-yl]pyridazine-3-carboxamide |
| 4J08 | N,N-Diethyl-6-[4-[2-[2-(5-hydroxypyridin-3-yl)ethynyl]benzoyl]piperazin-1-yl]pyridazine-3-carboxamide |
| 4J09 | N,N-Diethyl-6-[4-[5-[2-(3-hydroxyphenyl)ethynyl]pyridine-3-carbonyl]piperazin-1-yl]pyridazine-3-carboxamide |
| 4J10 | N,N-Diethyl-6-[4-[5-[2-(5-hydroxypyridin-3-yl)ethynyl]pyridine-3-carbonyl]piperazin-1-yl]pyridazine-3-carboxamide |
| 4J11 | N,N-Diethyl-6-[4-[3-[2-(3-hydroxyphenyl)phenyl]propanoyl]piperazin-1-yl]pyridazine-3-carboxamide |
| 4J12 | N,N-Diethyl-6-[4-[3-[2-(5-hydroxypyridin-3-yl)phenyl]propanoyl]piperazin-1-yl]pyridazine-3-carboxamide |
| 4J13 | N,N-Diethyl-6-[4-[4-(3-hydroxyphenyl)naphthalene-1-carbonyl]piperazin-1-yl]pyridazine-3-carboxamide |
| 4J14 | N,N-Diethyl-6-[4-[4-(5-hydroxypyridin-3-yl)naphthalene-1-carbonyl]piperazin-1-yl]pyridazine-3-carboxamide |
| 4J15 | 6-[4-[3-tert-Butyl-5-(3-hydroxyphenyl)benzoyl]piperazin-1-yl]-N,N-diethylpyridazine-3-carboxamide |
| 4J16 | 6-[4-[3-tert-Butyl-5-(5-hydroxypyridin-3-yl)benzoyl]piperazin-1-yl]-N,N-diethylpyridazine-3-carboxamide |
| 4J17 | N,N-Diethyl-6-[4-[3-(3-hydroxyphenyl)-5-(trifluoromethyl)benzoyl]piperazin-1-yl]pyridazine-3-carboxamide |
| 4J18 | N,N-Diethyl-6-[4-[3-(5-hydroxypyridin-3-yl)-5-(trifluoromethyl)benzoyl]piperazin-1-yl]pyridazine-3-carboxamide |
| 4J19 | N,N-Diethyl-6-[4-[3-[2-(3-hydroxyphenyl)ethynyl]-5-(trifluoromethyl)benzoyl]piperazin-1-yl]pyridazine-3-carboxamide |
| 4J20 | N,N-Diethyl-6-[4-[3-[2-(5-hydroxypyridin-3-yl)ethynyl]-5-(trifluoromethyl)benzoyl]piperazin-1-yl]pyridazine-3-carboxamide |
| 4J21 | N,N-Diethyl-6-[4-[3-[4-(3-hydroxyphenyl)pyrazol-1-yl]-5-(trifluoromethyl)benzoyl]piperazin-1-yl]pyridazine-3-carboxamide |
| 4J22 | N,N-Diethyl-6-[4-[3-[4-(5-hydroxypyridin-3-yl)pyrazol-1-yl]-5-(trifluoromethyl)benzoyl]piperazin-1-yl]pyridazine-3-carboxamide |

| | |
|---|---|
| 4J23 | N,N-Diethyl-4-[4-[4-(3-hydroxyphenyl)thiophene-2-carbonyl]piperazin-1-yl]benzamide |
| 4J24 | N,N-Diethyl-4-[4-[4-(5-hydroxypyridin-3-yl)thiophene-2-carbonyl]piperazin-1-yl]benzamide |
| 4J25 | N,N-Diethyl-4-[4-[4-(3-hydroxyphenyl)-3-methylbenzoyl]piperazin-1-yl]benzamide |
| 4J26 | N,N-Diethyl-4-[4-[4-(5-hydroxypyridin-3-yl)-3-methylbenzoyl]piperazin-1-yl]benzamide |
| 4J27 | N,N-Diethyl-4-[4-[3-fluoro-4-(3-hydroxyphenyl)benzoyl]piperazin-1-yl]benzamide |
| 4J28 | N,N-Diethyl-4-[4-[3-fluoro-4-(5-hydroxypyridin-3-yl)benzoyl]piperazin-1-yl]benzamide |
| 4J29 | N,N-Diethyl-4-[4-[2-[2-(3-hydroxyphenyl)ethynyl]benzoyl]piperazin-1-yl]benzamide |
| 4J30 | N,N-Diethyl-4-[4-[2-[2-(5-hydroxypyridin-3-yl)ethynyl]benzoyl]piperazin-1-yl]benzamide |
| 4J31 | N,N-Diethyl-4-[4-[5-[2-(3-hydroxyphenyl)ethynyl]pyridine-3-carbonyl]piperazin-1-yl]benzamide |
| 4J32 | N,N-Diethyl-4-[4-[5-[2-(5-hydroxypyridin-3-yl)ethynyl]pyridine-3-carbonyl]piperazin-1-yl]benzamide |
| 4J33 | N,N-Diethyl-4-[4-[3-[2-(3-hydroxyphenyl)phenyl]propanoyl]piperazin-1-yl]benzamide |
| 4J34 | N,N-Diethyl-4-[4-[3-[2-(5-hydroxypyridin-3-yl)phenyl]propanoyl]piperazin-1-yl]benzamide |
| 4J35 | N,N-Diethyl-4-[4-[4-(3-hydroxyphenyl)naphthalene-1-carbonyl]piperazin-1-yl]benzamide |
| 4J36 | N,N-Diethyl-4-[4-[4-(5-hydroxypyridin-3-yl)naphthalene-1-carbonyl]piperazin-1-yl]benzamide |
| 4J37 | 4-[4-[3-tert-Butyl-5-(3-hydroxyphenyl)benzoyl]piperazin-1-yl]-N,N-diethylbenzamide |
| 4J38 | 4-[4-[3-tert-Butyl-5-(5-hydroxypyridin-3-yl)benzoyl]piperazin-1-yl]-N,N-diethylbenzamide |
| 4J39 | N,N-Diethyl-4-[4-[3-(3-hydroxyphenyl)-5-(trifluoromethyl)benzoyl]piperazin-1-yl]benzamide |
| 4J40 | N,N-Diethyl-4-[4-[3-(5-hydroxypyridin-3-yl)-5-(trifluoromethyl)benzoyl]piperazin-1-yl]benzamide |
| 4J41 | N,N-Diethyl-4-[4-[3-[2-(3-hydroxyphenyl)ethynyl]-5-(trifluoromethyl)benzoyl]piperazin-1-yl]benzamide |
| 4J42 | N,N-Diethyl-4-[4-[3-[2-(5-hydroxypyridin-3-yl)ethynyl]-5-(trifluoromethyl)benzoyl]piperazin-1-yl]benzamide |
| 4J43 | N,N-Diethyl-4-[4-[3-[4-(3-hydroxyphenyl)pyrazol-1-yl]-5-(trifluoromethyl)benzoyl]piperazin-1-yl]benzamide |
| 4J44 | N,N-Diethyl-4-[4-[3-[4-(5-hydroxypyridin-3-yl)pyrazol-1-yl]-5-(trifluoromethyl)benzoyl]piperazin-1-yl]benzamide |
| 4J45 | N,N-Diethyl-4-[4-[[2-(3-hydroxyphenyl)phenyl]methyl]piperazin-1-yl]benzamide |
| 4J46 | N,N-Diethyl-4-[4-[[2-(5-hydroxypyridin-3-yl)phenyl]methyl]piperazin-1-yl]benzamide |
| 4J47 | N,N-Diethyl-4-[4-[[4-(3-hydroxyphenyl)-3-methylphenyl]methyl]piperazin-1-yl]benzamide |
| 4J48 | N,N-Diethyl-4-[4-[[4-(5-hydroxypyridin-3-yl)-3-methylphenyl]methyl]piperazin-1-yl]benzamide |
| 4J49 | N,N-Diethyl-4-[4-[[2-ethyl-4-(3-hydroxyphenyl)phenyl]methyl]piperazin-1-yl]benzamide |
| 4J50 | N,N-Diethyl-4-[4-[[2-ethyl-4-(5-hydroxypyridin-3-yl)phenyl]methyl]piperazin-1-yl]benzamide |
| 4J51 | N,N-Diethyl-4-[4-[[3-fluoro-4-(3-hydroxyphenyl)phenyl]methyl]piperazin-1-yl]benzamide |
| 4J52 | N,N-Diethyl-4-[4-[[3-fluoro-4-(5-hydroxypyridin-3-yl)phenyl]methyl]piperazin-1-yl]benzamide |

| | |
|---|---|
| 4J53 | N,N-Diethyl-4-[4-[[4-(3-hydroxyphenyl)-2-methoxyphenyl]methyl]piperazin-1-yl]benzamide |
| 4J54 | N,N-Diethyl-4-[4-[[4-(5-hydroxypyridin-3-yl)-2-methoxyphenyl]methyl]piperazin-1-yl]benzamide |
| 4J55 | 4-[4-[[3-Ethoxy-5-(3-hydroxyphenyl)phenyl]methyl]piperazin-1-yl]-N,N-diethylbenzamide |
| 4J56 | 4-[4-[[3-Ethoxy-5-(5-hydroxypyridin-3-yl)phenyl]methyl]piperazin-1-yl]-N,N-diethylbenzamide |
| 4J57 | N,N-Diethyl-4-[4-[[3-(3-hydroxyphenyl)-5-propan-2-yloxyphenyl]methyl]piperazin-1-yl]benzamide |
| 4J58 | N,N-Diethyl-4-[4-[[3-(5-hydroxypyridin-3-yl)-5-propan-2-yloxyphenyl]methyl]piperazin-1-yl]benzamide |
| 4J59 | N,N-Diethyl-4-[4-[[4-(3-hydroxyphenyl)-2,6-dimethoxyphenyl]methyl]piperazin-1-yl]benzamide |
| 4J60 | N,N-Diethyl-4-[4-[[4-(5-hydroxypyridin-3-yl)-2,6-dimethoxyphenyl]methyl]piperazin-1-yl]benzamide |
| 4J61 | N,N-Diethyl-4-[4-[[3-(3-hydroxyphenyl)-5-(trifluoromethyl)phenyl]methyl]piperazin-1-yl]benzamide |
| 4J62 | N,N-Diethyl-4-[4-[[3-(5-hydroxypyridin-3-yl)-5-(trifluoromethyl)phenyl]methyl]piperazin-1-yl]benzamide |
| 4J63 | N,N-Diethyl-4-[4-[[3-(3-hydroxyphenyl)-5-(trifluoromethoxy)phenyl]methyl]piperazin-1-yl]benzamide |
| 4J64 | N,N-Diethyl-4-[4-[[3-(5-hydroxypyridin-3-yl)-5-(trifluoromethoxy)phenyl]methyl]piperazin-1-yl]benzamide |
| 4J65 | N,N-Diethyl-4-[4-[[4-(3-hydroxyphenyl)naphthalen-1-yl]methyl]piperazin-1-yl]benzamide |
| 4J66 | N,N-Diethyl-4-[4-[[4-(5-hydroxypyridin-3-yl)naphthalen-1-yl]methyl]piperazin-1-yl]benzamide |
| 4J67 | N,N-Diethyl-4-[4-[[4-(3-hydroxyphenyl)thiophen-2-yl]methyl]piperazin-1-yl]benzamide |
| 4J68 | N,N-Diethyl-4-[4-[[4-(5-hydroxypyridin-3-yl)thiophen-2-yl]methyl]piperazin-1-yl]benzamide |
| 4J69 | N,N-Diethyl-4-[4-[[2-[2-(3-hydroxyphenyl)ethynyl]phenyl]methyl]piperazin-1-yl]benzamide |
| 4J70 | N,N-Diethyl-4-[4-[[2-[2-(5-hydroxypyridin-3-yl)ethynyl]phenyl]methyl]piperazin-1-yl]benzamide |
| 4J71 | N,N-Diethyl-6-[4-[[2-(3-hydroxyphenyl)phenyl]methyl]piperazin-1-yl]pyridazine-3-carboxamide |
| 4J72 | N,N-Diethyl-6-[4-[[2-(5-hydroxypyridin-3-yl)phenyl]methyl]piperazin-1-yl]pyridazine-3-carboxamide |
| 4J73 | N,N-Diethyl-6-[4-[[4-(3-hydroxyphenyl)-3-methylphenyl]methyl]piperazin-1-yl]pyridazine-3-carboxamide |
| 4J74 | N,N-Diethyl-6-[4-[[4-(5-hydroxypyridin-3-yl)-3-methylphenyl]methyl]piperazin-1-yl]pyridazine-3-carboxamide |
| 4J75 | N,N-Diethyl-6-[4-[[2-ethyl-4-(3-hydroxyphenyl)phenyl]methyl]piperazin-1-yl]pyridazine-3-carboxamide |
| 4J76 | N,N-Diethyl-6-[4-[[2-ethyl-4-(5-hydroxypyridin-3-yl)phenyl]methyl]piperazin-1-yl]pyridazine-3-carboxamide |
| 4J77 | N,N-Diethyl-6-[4-[[3-fluoro-4-(3-hydroxyphenyl)phenyl]methyl]piperazin-1-yl]pyridazine-3-carboxamide |
| 4J78 | N,N-Diethyl-6-[4-[[3-fluoro-4-(5-hydroxypyridin-3-yl)phenyl]methyl]piperazin-1-yl]pyridazine-3-carboxamide |
| 4J79 | N,N-Diethyl-6-[4-[[4-(3-hydroxyphenyl)-2-methoxyphenyl]methyl]piperazin-1-yl]pyridazine-3-carboxamide |
| 4J80 | N,N-Diethyl-6-[4-[[4-(5-hydroxypyridin-3-yl)-2-methoxyphenyl]methyl]piperazin-1-yl]pyridazine-3-carboxamide |

| | |
|---|---|
| 4J81 | 6-[4-[[3-Ethoxy-5-(3-hydroxyphenyl)phenyl]methyl]piperazin-1-yl]-N,N-diethylpyridazine-3-carboxamide |
| 4J82 | 6-[4-[[3-Ethoxy-5-(5-hydroxypyridin-3-yl)phenyl]methyl]piperazin-1-yl]-N,N-diethylpyridazine-3-carboxamide |
| 4J83 | N,N-Diethyl-6-[4-[[3-(3-hydroxyphenyl)-5-propan-2-yloxyphenyl]methyl]piperazin-1-yl]pyridazine-3-carboxamide |
| 4J84 | N,N-Diethyl-6-[4-[[3-(5-hydroxypyridin-3-yl)-5-propan-2-yloxyphenyl]methyl]piperazin-1-yl]pyridazine-3-carboxamide |
| 4J85 | N,N-Diethyl-6-[4-[[4-(3-hydroxyphenyl)-2,6-dimethoxyphenyl]methyl]piperazin-1-yl]pyridazine-3-carboxamide |
| 4J86 | N,N-Diethyl-6-[4-[[4-(5-hydroxypyridin-3-yl)-2,6-dimethoxyphenyl]methyl]piperazin-1-yl]pyridazine-3-carboxamide |
| 4J87 | N,N-Diethyl-6-[4-[[3-(3-hydroxyphenyl)-5-(trifluoromethyl)phenyl]methyl]piperazin-1-yl]pyridazine-3-carboxamide |
| 4J88 | N,N-Diethyl-6-[4-[[3-(5-hydroxypyridin-3-yl)-5-(trifluoromethyl)phenyl]methyl]piperazin-1-yl]pyridazine-3-carboxamide |
| 4J89 | N,N-Diethyl-6-[4-[[3-(3-hydroxyphenyl)-5-(trifluoromethoxy)phenyl]methyl]piperazin-1-yl]pyridazine-3-carboxamide |
| 4J90 | N,N-Diethyl-6-[4-[[3-(5-hydroxypyridin-3-yl)-5-(trifluoromethoxy)phenyl]methyl]piperazin-1-yl]pyridazine-3-carboxamide |
| 4J91 | N,N-Diethyl-6-[4-[[4-(3-hydroxyphenyl)naphthalen-1-yl]methyl]piperazin-1-yl]pyridazine-3-carboxamide |
| 4J92 | N,N-Diethyl-6-[4-[[4-(5-hydroxypyridin-3-yl)naphthalen-1-yl]methyl]piperazin-1-yl]pyridazine-3-carboxamide |
| 4J93 | N,N-Diethyl-6-[4-[[4-(3-hydroxyphenyl)thiophen-2-yl]methyl]piperazin-1-yl]pyridazine-3-carboxamide |
| 4J94 | N,N-Diethyl-6-[4-[[4-(5-hydroxypyridin-3-yl)thiophen-2-yl]methyl]piperazin-1-yl]pyridazine-3-carboxamide |
| 4J95 | N,N-Diethyl-6-[4-[[2-[2-(3-hydroxyphenyl)ethynyl]phenyl]methyl]piperazin-1-yl]pyridazine-3-carboxamide |
| 4J96 | N,N-Diethyl-6-[4-[[2-[2-(5-hydroxypyridin-3-yl)ethynyl]phenyl]methyl]piperazin-1-yl]pyridazine-3-carboxamide |
| 4J97 | N,N-Diethyl-4-[4-[1-[4-(3-hydroxyphenyl)phenyl]ethyl]piperazin-1-yl]benzamide |
| 4J98 | N,N-Diethyl-4-[4-[1-[4-(5-hydroxypyridin-3-yl)phenyl]ethyl]piperazin-1-yl]benzamide |
| 4J99 | N,N-Diethyl-4-[4-[1-[2-fluoro-4-(3-hydroxyphenyl)phenyl]ethyl]piperazin-1-yl]benzamide |
| 4J100 | N,N-Diethyl-4-[4-[1-[2-fluoro-4-(5-hydroxypyridin-3-yl)phenyl]ethyl]piperazin-1-yl]benzamide |

Table EJ01 describes the retention times (min) of observed UV peaks, $(M+H)^+$ observed in the UV peaks, the retention times of MS peaks in MS chromatograms, and the numbers of compounds assigned to the UV peaks and the MS peaks. From the possibility that two compounds were lacked in Example 4-2-2 and two compounds were lacked in Example 4-2-3, there is a possibility that four compounds were lacked among 100 compounds shown in Table EIJ01. Although m/z of the MS peaks was confirmed down to three decimal places, 0 at the decimal level may be omitted in the description in Table EJ01. For example, "587.000" is also referred to as "587", "587.100" is also referred to as "587.1 ", and "587.120" is also referred to as "587.12". Although the retention times of the MS peaks were confirmed down to three decimal places and the retention times of the UV peaks were confirmed down to two decimal places, 0 at the decimal level may be omitted in the description in Table EJ01. For example, "15.000" is also referred to as "15", "15.100" is also referred to as "15.1", and "15.120" is also referred to as "15.12".

[Table 215]

[Table EJ01]

| Mixture No. | mixEJ01 | | | | | | |
|---|---|---|---|---|---|---|---|
| m/z [M+H]+ | Retention time of MS peak (min) | Retention time of UV peak (min) | No. of assigned compound | m/z [M+H]+ | Retention time of MS peak (min) | Retention time of UV pea (min) | No. of assigned compound |
| 453.207 | 3.457 | 3.41 | 4J94 | 458.28 | 6.217 | 6.17 | 4J47, 4J97 |
| 461.266 | 3.58 | 3.54 | 4J74 | 529.217 | 6.29 | 6.25 | 4J18, 4J64 |
| 465.195 | 3.667 | 3.63 | 4J24, 4J78 | 504.286 | 6.297 | 6.25 | 4J59, 4J83 |
| 477.23 | 3.763 | 3.73 | 4J100, 4J28, 4J80 | 487.27 | 6.37 | 6.34 | 4J34 |
| 447.25 | 3.883 | 3.85 | 4J72 | 514.306 | 6.383 | 6.34 | 4J37, 4J87 |
| 475.245 | 4.037 | 3.99 | 4J04, 4J54, 4J76 | 517.292 | 6.5 | 6.5 | 4J16 |
| 451.216 | 4.113 | 4.07 | 4J68 | 509.255 | 6.517 | 6.5 | 4J36 |
| 507.271 | 4.17 | 4.13 | 4J86 | 504.286 | 6.533 | 6.5 | 4J59, 4J83 |
| 497.266 | 4.22 | 4.13 | 4J92 | 595.239 | 6.543 | 6.5 | 4J22 |
| 459.275 | 4.257 | 4.22 | 4J48, 4J98 | 472.259 | 6.55 | 6.5 | 4J25, 4J49 |
| 491.277 | 4.27 | 4.22 | 4J82 | 468.265 | 6.563 | 6.5 | 4J69 |
| 463.25 | 4.36 | 4.32 | 4J52 | 530.237 | 6.573 | 6.5 | 4J89 |
| 445.26 | 4.527 | 4.48 | 4J46 | 488.266 | 6.6 | 6.5 | 4J11, 4J55 |
| 505.281 | 4.617 | 4.56 | 4J60, 4J84 | 484.234 | 6.703 | 6.67 | 4J07, 4J32 |
| 473.255 | 4.683 | 4.65 | 4J26, 4J50 | 494.28 | 6.71 | 6.67 | 4J65 |
| 467.186 | 4.8 | 4.77 | 4J02 | 502.306 | 6.897 | 6.86 | 4J57 |
| 515.302 | 4.81 | 4.77 | 4J38, 4J88 | 512.252 | 6.977 | 6.94 | 4J61 |
| 475.245 | 4.843 | 4.81 | 4J04, 4J54, 4J76 | 485.23 | 7.05 | 7.02 | 4J08, 4J09 |
| 505.281 | 4.85 | 4.81 | 4J60, 4J84 | 486.225 | 7.07 | 7.02 | 4J10, 4J33 |
| 471.25 | 4.883 | 4.81 | 4J96 | 528.222 | 7.15 | 7.11 | 4J17, 4J63 |
| 495.275 | 4.89 | 4.86 | 4J66 | 466.191 | 7.217 | 7.18 | 4J01 |
| 489.261 | 4.953 | 4.93 | 4J12, 4J56 | 478.225 | 7.42 | 7.38 | 4J05 |
| 479.22 | 4.957 | 4.93 | 4J06 | 527.226 | 7.48 | 7.44 | 4J40 |
| 531.233 | 4.997 | 4.96 | 4J90 | 515.302 | 7.547 | 7.52 | 4J38, 4J88 |
| 452.211 | 5.15 | 5.11 | 4J93 | 474.25 | 7.57 | 7.52 | 4J03, 4J53, 4J75 |
| 446.255 | 5.257 | 5.23 | 4J71 | 593.248 | 7.6 | 7.57 | 4J44 |
| 503.302 | 5.263 | 5.23 | 4J58 | 484.234 | 7.82 | 7.78 | 4J07, 4J32 |
| 489.261 | 5.367 | 5.34 | 4J12, 4J56 | 553.217 | 7.82 | 7.78 | 4J20 |
| 464.2 | 5.38 | 5.34 | 4J23, 4J77 | 488.266 | 8.027 | 7.99 | 4J11, 4J55 |
| 511.245 | 5.423 | 5.39 | 4J14 | 510.25 | 8.133 | 8.1 | 4J13 |
| 513.247 | 5.493 | 5.47 | 4J62 | 483.239 | 8.26 | 8.25 | 4J30, 4J31 |
| 469.26 | 5.497 | 5.47 | 4J70 | 483.239 | 8.293 | 8.25 | 4J30, 4J31 |
| 477.23 | 5.513 | 5.47 | 4J100, 4J28, 4J80 | 464.2 | 8.61 | 8.57 | 4J23, 4J77 |
| 476.234 | 5.517 | 5.47 | 4J27, 4J79, 4J99 | 528.222 | 8.657 | 8.61 | 4J17, 4J63 |
| 460.271 | 5.527 | 5.47 | 4J73 | 476.234 | 8.727 | 8.69 | 4J27, 4J79, 4J99 |

| | | | | | | | |
|---|---|---|---|---|---|---|---|
| 459.275 | 5.567 | 5.47 | 4J48, 4J98 | 476.234 | 8.76 | 8.69 | 4J27, 4J79, 4J99 |
| 529.217 | 5.667 | 5.64 | 4J18, 4J64 | 472.259 | 8.893 | 8.86 | 4J25, 4J49 |
| 506.276 | 5.83 | 5.8 | 4J85 | 474.25 | 8.9 | 8.86 | 4J03, 4J53, 4J75 |
| 450.221 | 5.857 | 5.8 | 4J67 | 482.244 | 9.05 | 9.02 | 4J29 |
| 475.245 | 5.903 | 5.87 | 4J04, 4J54, 4J76 | 551.226 | 9.06 | 9.02 | 4J42 |
| 473.255 | 5.907 | 5.87 | 4J26, 4J50 | 594.243 | 9.093 | 9.05 | 4J21 |
| 465.195 | 5.913 | 5.87 | 4J24, 4J78 | 485.23 | 9.24 | 9.23 | 4J08, 4J09 |
| 444.265 | 5.947 | 5.87 | 4J45 | 486.225 | 9.257 | 9.23 | 4J10, 4J33 |
| 490.281 | 5.977 | 5.97 | 4J81 | 516.297 | 9.277 | 9.23 | 4J15 |
| 470.255 | 6.01 | 5.97 | 4J95 | 508.259 | 9.407 | 9.37 | 4J35 |
| 462.255 | 6.057 | 6.04 | 4J51 | 552.222 | 9.457 | 9.41 | 4J19 |
| 458.28 | 6.07 | 6.04 | 4J47, 4J97 | 526.231 | 9.87 | 9.83 | 4J39 |
| 496.271 | 6.077 | 6.04 | 4J91 | 592.253 | 10.25 | 10.21 | 4J43 |
| 477.23 | 6.15 | 6.1 | 4J100, 4J28, 4J80 | 514.306 | 10.503 | 10.47 | 4J37, 4J87 |
| 474.25 | 6.207 | 6.17 | 4J03, 4J53, 4J75 | 550.231 | 10.623 | 10.58 | 4J41 |

Example 4-4-2: Synthesis of mixtures mixEJ02 to mixEJ12

The title mixtures mixEJ02 to mixEJ12 shown in Table EIJ02 to Table EIJ12 were obtained by synthesis in the same manner as in Example 4-4-1 using mixtures mixEI08-02R to mixEI18-02R shown in Table EI08 to Table EI18. The mixtures were analyzed under analysis conditions SMD-FA05-long-25 min, and the UV areas were confirmed at a UV wavelength of 290 nm. The results of analyzing the mixtures are shown in Table EJ02 to Table EJ12.

[Table 216]

[Table EIJ02]

| No. of mixture used | mixEI08-02R |
|---|---|
| Mixture No after cleavage | mixEJ02 |
| No. of compound in Table EJ02 | Compound name |
| 1M01 | 6-[4-[4-(3-Hydroxyphenyl)thiophene-2-carbonyl]piperazin-1-yl]-N-propylpyridazine-3-carboxamide |
| 1M02 | 6-[4-[4-(5-Hydroxypyridin-3-yl)thiophene-2-carbonyl]piperazin-1-yl]-N-propylpyridazine-3-carboxamide |
| 1M03 | 6-[4-[4-(3-Hydroxyphenyl)-3-methylbenzoyl]piperazin-1-yl]-N-propylpyridazine-3-carboxamide |
| 1M04 | 6-[4-[4-(5-Hydroxypyridin-3-yl)-3-methylbenzoyl]piperazin-1-yl]-N-propylpyridazine-3-carboxamide |
| 1M05 | 6-[4-[3-Fluoro-4-(3-hydroxyphenyl)benzoyl]piperazin-1-yl]-N-propylpyridazine-3-carboxamide |
| 1M06 | 6-[4-[3-Fluoro-4-(5-hydroxypyridin-3-yl)benzoyl]piperazin-1-yl]-N-propylpyridazine-3-carboxamide |
| 1M07 | 6-[4-[2-[2-(3-Hydroxyphenyl)ethynyl]benzoyl]piperazin-1-yl]-N-propylpyridazine-3-carboxamide |

| | |
|---|---|
| 1M08 | 6-[4-[2-[2-(5-Hydroxypyridin-3-yl)ethynyl]benzoyl]piperazin-1-yl]-N-propylpyridazine-3-carboxamide |
| 1M09 | 6-[4-[5-[2-(3-Hydroxyphenyl)ethynyl]pyridine-3-carbonyl]piperazin-1-yl]-N-propylpyridazine-3-carboxamide |
| 1M10 | 6-[4-[5-[2-(5-Hydroxypyridin-3-yl)ethynyl]pyridine-3-carbonyl]piperazin-1-yl]-N-propylpyridazine-3-carboxamide |
| 1M11 | 6-[4-[3-[2-(3-Hydroxyphenyl)phenyl]propanoyl]piperazin-1-yl]-N-propylpyridazine-3-carboxamide |
| 1M12 | 6-[4-[3-[2-(5-Hydroxypyridin-3-yl)phenyl]propanoyl]piperazin-1-yl]-N-propylpyridazine-3-carboxamide |
| 1M13 | 6-[4-[4-(3-Hydroxyphenyl)naphthalene-1-carbonyl]piperazin-1-yl]-N-propylpyridazine-3-carboxamide |
| 1M14 | 6-[4-[4-(5-Hydroxypyridin-3-yl)naphthalene-1-carbonyl]piperazin-1-yl]-N-propylpyridazine-3-carboxamide |
| 1M15 | 6-[4-[3-tert-Butyl-5-(3-hydroxyphenyl)benzoyl]piperazin-1-yl]-N-propylpyridazine-3-carboxamide |
| 1M16 | 6-[4-[3-tert-Butyl-5-(5-hydroxypyridin-3-yl)benzoyl]piperazin-1-yl]-N-propylpyridazine-3-carboxamide |
| 1M17 | 6-[4-[3-(3-Hydroxyphenyl)-5-(trifluoromethyl)benzoyl]piperazin-1-yl]-N-propylpyridazine-3-carboxamide |
| 1M18 | 6-[4-[3-(5-Hydroxypyridin-3-yl)-5-(trifluoromethyl)benzoyl]piperazin-1-yl]-N-propylpyridazine-3-carboxamide |
| 1M19 | 6-[4-[3-[2-(3-Hydroxyphenyl)ethynyl]-5-(trifluoromethyl)benzoyl]piperazin-1-yl]-N-propylpyridazine-3-carboxamide |
| 1M20 | 6-[4-[3-[2-(5-Hydroxypyridin-3-yl)ethynyl]-5-(trifluoromethyl)benzoyl]piperazin-1-yl]-N-propylpyridazine-3-carboxamide |
| 1M21 | 6-[4-[3-[4-(3-Hydroxyphenyl)pyrazol-1-yl]-5-(trifluoromethyl)benzoyl]piperazin-1-yl]-N-propylpyridazine-3-carboxamide |
| 1M22 | 6-[4-[3-[4-(5-Hydroxypyridin-3-yl)pyrazol-1-yl]-5-(trifluoromethyl)benzoyl]piperazin-1-yl]-N-propylpyridazine-3-carboxamide |
| 1M23 | 4-[4-[4-(3-Hydroxyphenyl)thiophene-2-carbonyl]piperazin-1-yl]-N-propylbenzamide |
| 1M24 | 4-[4-[4-(5-Hydroxypyridin-3-yl)thiophene-2-carbonyl]piperazin-1-yl]-N-propylbenzamide |
| 1M25 | 4-[4-[4-(3-Hydroxyphenyl)-3-methylbenzoyl]piperazin-1-yl]-N-propylbenzamide |
| 1M26 | 4-[4-[4-(5-Hydroxypyridin-3-yl)-3-methylbenzoyl]piperazin-1-yl]-N-propylbenzamide |
| 1M27 | 4-[4-[3-Fluoro-4-(3-hydroxyphenyl)benzoyl]piperazin-1-yl]-N-propylbenzamide |
| 1M28 | 4-[4-[3-Fluoro-4-(5-hydroxypyridin-3-yl)benzoyl]piperazin-1-yl]-N-propylbenzamide |
| 1M29 | 4-[4-[2-[2-(3-Hydroxyphenyl)ethynyl]benzoyl]piperazin-1-yl]-N-propylbenzamide |
| 1M30 | 4-[4-[2-[2-(5-Hydroxypyridin-3-yl)ethynyl]benzoyl]piperazin-1-yl]-N-propylbenzamide |
| 1M31 | 4-[4-[5-[2-(3-Hydroxyphenyl)ethynyl]pyridine-3-carbonyl]piperazin-1-yl]-N-propylbenzamide |
| 1M32 | 4-[4-[5-[2-(5-Hydroxypyridin-3-yl)ethynyl]pyridine-3-carbonyl]piperazin-1-yl]-N-propylbenzamide |
| 1M33 | 4-[4-[3-[2-(3-Hydroxyphenyl)phenyl]propanoyl]piperazin-1-yl]-N-propylbenzamide |
| 1M34 | 4-[4-[3-[2-(5-Hydroxypyridin-3-yl)phenyl]propanoyl]piperazin-1-yl]-N-propylbenzamide |
| 1M35 | 4-[4-[4-(3-Hydroxyphenyl)naphthalene-1-carbonyl]piperazin-1-yl]-N-propylbenzamide |
| 1M36 | 4-[4-[4-(5-Hydroxypyridin-3-yl)naphthalene-1-carbonyl]piperazin-1-yl]-N-propylbenzamide |
| 1M37 | 4-[4-[3-tert-Butyl-5-(3-hydroxyphenyl)benzoyl]piperazin-1-yl]-N-propylbenzamide |
| 1M38 | 4-[4-[3-tert-Butyl-5-(5-hydroxypyridin-3-yl)benzoyl]piperazin-1-yl]-N-propylbenzamide |

| | |
|---|---|
| 1M39 | 4-[4-[3-(3-Hydroxyphenyl)-5-(trifluoromethyl)benzoyl]piperazin-1-yl]-N-propylbenzamide |
| 1M40 | 4-[4-[3-(5-Hydroxypyridin-3-yl)-5-(trifluoromethyl)benzoyl]piperazin-1-yl]-N-propylbenzamide |
| 1M41 | 4-[4-[3-[2-(3-Hydroxyphenyl)ethynyl]-5-(trifluoromethyl)benzoyl]piperazin-1-yl]-N-propylbenzamide |
| 1M42 | 4-[4-[3-[2-(5-Hydroxypyridin-3-yl)ethynyl]-5-(trifluoromethyl)benzoyl]piperazin-1-yl]-N-propylbenzamide |
| 1M43 | 4-[4-[3-[4-(3-Hydroxyphenyl)pyrazol-1-yl]-5-(trifluoromethyl)benzoyl]piperazin-1-yl]-N-propylbenzamide |
| 1M44 | 4-[4-[3-[4-(5-Hydroxypyridin-3-yl)pyrazol-1-yl]-5-(trifluoromethyl)benzoyl]piperazin-1-yl]-N-propylbenzamide |
| 1M45 | 4-[4-[[2-(3-Hydroxyphenyl)phenyl]methyl]piperazin-1-yl]-N-propylbenzamide |
| 1M46 | 4-[4-[[2-(5-Hydroxypyridin-3-yl)phenyl]methyl]piperazin-1-yl]-N-propylbenzamide |
| 1M47 | 4-[4-[[4-(3-Hydroxyphenyl)-3-methylphenyl]methyl]piperazin-1-yl]-N-propylbenzamide |
| 1M48 | 4-[4-[[4-(5-Hydroxypyridin-3-yl)-3-methylphenyl]methyl]piperazin-1-yl]-N-propylbenzamide |
| 1M49 | 4-[4-[[2-Ethyl-4-(3-hydroxyphenyl)phenyl]methyl]piperazin-1-yl]-N-propylbenzamide |
| 1M50 | 4-[4-[[2-Ethyl-4-(5-hydroxypyridin-3-yl)phenyl]methyl]piperazin-1-yl]-N-propylbenzamide |
| 1M51 | 4-[4-[[3-Fluoro-4-(3-hydroxyphenyl)phenyl]methyl]piperazin-1-yl]-N-propylbenzamide |
| 1M52 | 4-[4-[[3-Fluoro-4-(5-hydroxypyridin-3-yl)phenyl]methyl]piperazin-1-yl]-N-propylbenzamide |
| 1M53 | 4-[4-[[4-(3-Hydroxyphenyl)-2-methoxyphenyl]methyl]piperazin-1-yl]-N-propylbenzamide |
| 1M54 | 4-[4-[[4-(5-Hydroxypyridin-3-yl)-2-methoxyphenyl]methyl]piperazin-1-yl]-N-propylbenzamide |
| 1M55 | 4-[4-[[3-Ethoxy-5-(3-hydroxyphenyl)phenyl]methyl]piperazin-1-yl]-N-propylbenzamide |
| 1M56 | 4-[4-[[3-Ethoxy-5-(5-hydroxypyridin-3-yl)phenyl]methyl]piperazin-1-yl]-N-propylbenzamide |
| 1M57 | 4-[4-[[3-(3-Hydroxyphenyl)-5-propan-2-yloxyphenyl]methyl]piperazin-1-yl]-N-propylbenzamide |
| 1M58 | 4-[4-[[3-(5-Hydroxypyridin-3-yl)-5-propan-2-yloxyphenyl]methyl]piperazin-1-yl]-N-propylbenzamide |
| 1M59 | 4-[4-[[4-(3-Hydroxyphenyl)-2,6-dimethoxyphenyl]methyl]piperazin-1-yl]-N-propylbenzamide |
| 1M60 | 4-[4-[[4-(5-Hydroxypyridin-3-yl)-2,6-dimethoxyphenyl]methyl]piperazin-1-yl]-N-propylbenzamide |
| 1M61 | 4-[4-[[3-(3-Hydroxyphenyl)-5-(trifluoromethyl)phenyl]methyl]piperazin-1-yl]-N-propylbenzamide |
| 1M62 | 4-[4-[[3-(5-Hydroxypyridin-3-yl)-5-(trifluoromethyl)phenyl]methyl]piperazin-1-yl]-N-propylbenzamide |
| 1M63 | 4-[4-[[3-(3-Hydroxyphenyl)-5-(trifluoromethoxy)phenyl]methyl]piperazin-1-yl]-N-propylbenzamide |
| 1M64 | 4-[4-[[3-(5-Hydroxypyridin-3-yl)-5-(trifluoromethoxy)phenyl]methyl]piperazin-1-yl]-N-propylbenzamide |
| 1M65 | 4-[4-[[4-(3-Hydroxyphenyl)naphthalen-1-yl]methyl]piperazin-1-yl]-N-propylbenzamide |
| 1M66 | 4-[4-[[4-(5-Hydroxypyridin-3-yl)naphthalen-1-yl]methyl]piperazin-1-yl]-N-propylbenzamide |
| 1M67 | 4-[4-[[4-(3-Hydroxyphenyl)thiophen-2-yl]methyl]piperazin-1-yl]-N-propylbenzamide |

| | |
|---|---|
| 1M68 | 4-[4-[[4-(5-Hydroxypyridin-3-yl)thiophen-2-yl]methyl]piperazin-1-yl]-N-propylbenzamide |
| 1M69 | 4-[4-[[2-[2-(3-Hydroxyphenyl)ethynyl]phenyl]methyl]piperazin-1-yl]-N-propylbenzamide |
| 1M70 | 4-[4-[[2-[2-(5-Hydroxypyridin-3-yl)ethynyl]phenyl]methyl]piperazin-1-yl]-N-propylbenzamide |
| 1M71 | 6-[4-[[2-(3-Hydroxyphenyl)phenyl]methyl]piperazin-1-yl]-N-propylpyridazine-3-carboxamide |
| 1M72 | 6-[4-[[2-(5-Hydroxypyridin-3-yl)phenyl]methyl]piperazin-1-yl]-N-propylpyridazine-3-carboxamide |
| 1M73 | 6-[4-[[4-(3-Hydroxyphenyl)-3-methylphenyl]methyl]piperazin-1-yl]-N-propylpyridazine-3-carboxamide |
| 1M74 | 6-[4-[[4-(5-Hydroxypyridin-3-yl)-3-methylphenyl]methyl]piperazin-1-yl]-N-propylpyridazine-3-carboxamide |
| 1M75 | 6-[4-[[2-Ethyl-4-(3-hydroxyphenyl)phenyl]methyl]piperazin-1-yl]-N-propylpyridazine-3-carboxamide |
| 1M76 | 6-[4-[[2-Ethyl-4-(5-hydroxypyridin-3-yl)phenyl]methyl]piperazin-1-yl]-N-propylpyridazine-3-carboxamide |
| 1M77 | 6-[4-[[3-Fluoro-4-(3-hydroxyphenyl)phenyl]methyl]piperazin-1-yl]-N-propylpyridazine-3-carboxamide |
| 1M78 | 6-[4-[[3-Fluoro-4-(5-hydroxypyridin-3-yl)phenyl]methyl]piperazin-1-yl]-N-propylpyridazine-3-carboxamide |
| 1M79 | 6-[4-[[4-(3-Hydroxyphenyl)-2-methoxyphenyl]methyl]piperazin-1-yl]-N-propylpyridazine-3-carboxamide |
| 1M80 | 6-[4-[[4-(5-Hydroxypyridin-3-yl)-2-methoxyphenyl]methyl]piperazin-1-yl]-N-propylpyridazine-3-carboxamide |
| 1M81 | 6-[4-[[3-Ethoxy-5-(3-hydroxyphenyl)phenyl]methyl]piperazin-1-yl]-N-propylpyridazine-3-carboxamide |
| 1M82 | 6-[4-[[3-Ethoxy-5-(5-hydroxypyridin-3-yl)phenyl]methyl]piperazin-1-yl]-N-propylpyridazine-3-carboxamide |
| 1M83 | 6-[4-[[3-(3-Hydroxyphenyl)-5-propan-2-yloxyphenyl]methyl]piperazin-1-yl]-N-propylpyridazine-3-carboxamide |
| 1M84 | 6-[4-[[3-(5-Hydroxypyridin-3-yl)-5-propan-2-yloxyphenyl]methyl]piperazin-1-yl]-N-propylpyridazine-3-carboxamide |
| 1M85 | 6-[4-[[4-(3-Hydroxyphenyl)-2,6-dimethoxyphenyl]methyl]piperazin-1-yl]-N-propylpyridazine-3-carboxamide |
| 1M86 | 6-[4-[[4-(5-Hydroxypyridin-3-yl)-2,6-dimethoxyphenyl]methyl]piperazin-1-yl]-N-propylpyridazine-3-carboxamide |
| 1M87 | 6-[4-[[3-(3-Hydroxyphenyl)-5-(trifluoromethyl)phenyl]methyl]piperazin-1-yl]-N-propylpyridazine-3-carboxamide |
| 1M88 | 6-[4-[[3-(5-Hydroxypyridin-3-yl)-5-(trifluoromethyl)phenyl]methyl]piperazin-1-yl]-N-propylpyridazine-3-carboxamide |
| 1M89 | 6-[4-[[3-(3-Hydroxyphenyl)-5-(trifluoromethoxy)phenyl]methyl]piperazin-1-yl]-N-propylpyridazine-3-carboxamide |
| 1M90 | 6-[4-[[3-(5-Hydroxypyridin-3-yl)-5-(trifluoromethoxy)phenyl]methyl]piperazin-1-yl]-N-propylpyridazine-3-carboxamide |
| 1M91 | 6-[4-[[4-(3-Hydroxyphenyl)naphthalen-1-yl]methyl]piperazin-1-yl]-N-propylpyridazine-3-carboxamide |
| 1M92 | 6-[4-[[4-(5-Hydroxypyridin-3-yl)naphthalen-1-yl]methyl]piperazin-1-yl]-N-propylpyridazine-3-carboxamide |
| 1M93 | 6-[4-[[4-(3-Hydroxyphenyl)thiophen-2-yl]methyl]piperazin-1-yl]-N-propylpyridazine-3-carboxamide |
| 1M94 | 6-[4-[[4-(5-Hydroxypyridin-3-yl)thiophen-2-yl]methyl]piperazin-1-yl]-N-propylpyridazine-3-carboxamide |
| 1M95 | 6-[4-[[2-[2-(3-Hydroxyphenyl)ethynyl]phenyl]methyl]piperazin-1-yl]-N-propylpyridazine-3-carboxamide |

| | |
|---|---|
| 1M96 | 6-[4-[[2-[2-(5-Hydroxypyridin-3-yl)ethynyl]phenyl]methyl]piperazin-1-yl]-N-propylpyridazine-3-carboxamide |
| 1M97 | 4-[4-[1-[4-(3-Hydroxyphenyl)phenyl]ethyl]piperazin-1-yl]-N-propylbenzamide |
| 1M98 | 4-[4-[1-[4-(5-Hydroxypyridin-3-yl)phenyl]ethyl]piperazin-1-yl]-N-propylbenzamide |
| 1M99 | 4-[4-[1-[2-Fluoro-4-(3-hydroxyphenyl)phenyl]ethyl]piperazin-1-yl]-N-propylbenzamide |
| 1M100 | 4-[4-[1-[2-Fluoro-4-(5-hydroxypyridin-3-yl)phenyl]ethyl]piperazin-1-yl]-N-propylbenzamide |

[Table 217]

[Table EIJ03]

| No. of mixture used | mixEI09-02R |
|---|---|
| Mixture No after cleavage | mixEJ03 |
| No. of compound in Table EJ03 | Compound name |
| 1K01 | 6-[4-[4-(3-Hydroxyphenyl)thiophene-2-carbonyl]piperazin-1-yl]-N-propylpyridazine-3-carboxamide |
| 1K02 | 6-[4-[4-(5-Hydroxypyridin-3-yl)thiophene-2-carbonyl]piperazin-1-yl]-N-propylpyridazine-3-carboxamide |
| 1K03 | 6-[4-[4-(3-Hydroxyphenyl)-3-methylbenzoyl]piperazin-1-yl]-N-propylpyridazine-3-carboxamide |
| 1K04 | 6-[4-[4-(5-Hydroxypyridin-3-yl)-3-methylbenzoyl]piperazin-1-yl]-N-propylpyridazine-3-carboxamide |
| 1K05 | 6-[4-[3-Fluoro-4-(3-hydroxyphenyl)benzoyl]piperazin-1-yl]-N-propylpyridazine-3-carboxamide |
| 1K06 | 6-[4-[3-Fluoro-4-(5-hydroxypyridin-3-yl)benzoyl]piperazin-1-yl]-N-propylpyridazine-3-carboxamide |
| 1K07 | 6-[4-[2-[2-(3-Hydroxyphenyl)ethynyl]benzoyl]piperazin-1-yl]-N-propylpyridazine-3-carboxamide |
| 1K08 | 6-[4-[2-[2-(5-Hydroxypyridin-3-yl)ethynyl]benzoyl]piperazin-1-yl]-N-propylpyridazine-3-carboxamide |
| 1K09 | 6-[4-[5-[2-(3-Hydroxyphenyl)ethynyl]pyridine-3-carbonyl]piperazin-1-yl]-N-propylpyridazine-3-carboxamide |
| 1K10 | 6-[4-[5-[2-(5-Hydroxypyridin-3-yl)ethynyl]pyridine-3-carbonyl]piperazin-1-yl]-N-propylpyridazine-3-carboxamide |
| 1K11 | 6-[4-[3-[2-(3-Hydroxyphenyl)phenyl]propanoyl]piperazin-1-yl]-N-propylpyridazine-3-carboxamide |
| 1K12 | 6-[4-[3-[2-(5-Hydroxypyridin-3-yl)phenyl]propanoyl]piperazin-1-yl]-N-propylpyridazine-3-carboxamide |
| 1K13 | 6-[4-[4-(3-Hydroxyphenyl)naphthalene-1-carbonyl]piperazin-1-yl]-N-propylpyridazine-3-carboxamide |
| 1K14 | 6-[4-[4-(5-Hydroxypyridin-3-yl)naphthalene-1-carbonyl]piperazin-1-yl]-N-propylpyridazine-3-carboxamide |
| 1K15 | 6-[4-[3-tert-Butyl-5-(3-hydroxyphenyl)benzoyl]piperazin-1-yl]-N-propylpyridazine-3-carboxamide |
| 1K16 | 6-[4-[3-tert-Butyl-5-(5-hydroxypyridin-3-yl)benzoyl]piperazin-1-yl]-N-propylpyridazine-3-carboxamide |
| 1K17 | 6-[4-[3-(3-Hydroxyphenyl)-5-(trifluoromethyl)benzoyl]piperazin-1-yl]-N-propylpyridazine-3-carboxamide |
| 1K18 | 6-[4-[3-(5-Hydroxypyridin-3-yl)-5-(trifluoromethyl)benzoyl]piperazin-1-yl]-N-propylpyridazine-3-carboxamide |
| 1K19 | 6-[4-[3-[2-(3-Hydroxyphenyl)ethynyl]-5-(trifluoromethyl)benzoyl]piperazin-1-yl]-N-propylpyridazine-3-carboxamide |

| | |
|---|---|
| 1K20 | 6-[4-[3-[2-(5-Hydroxypyridin-3-yl)ethynyl]-5-(trifluoromethyl)benzoyl]piperazin-1-yl]-N-propylpyridazine-3-carboxamide |
| 1K21 | 6-[4-[3-[4-(3-Hydroxyphenyl)pyrazol-1-yl]-5-(trifluoromethyl)benzoyl]piperazin-1-yl]-N-propylpyridazine-3-carboxamide |
| 1K22 | 6-[4-[3-[4-(5-Hydroxypyridin-3-yl)pyrazol-1-yl]-5-(trifluoromethyl)benzoyl]piperazin-1-yl]-N-propylpyridazine-3-carboxamide |
| 1K23 | 4-[4-[4-(3-Hydroxyphenyl)thiophene-2-carbonyl]piperazin-1-yl]-N-propylbenzamide |
| 1K24 | 4-[4-[4-(5-Hydroxypyridin-3-yl)thiophene-2-carbonyl]piperazin-1-yl]-N-propylbenzamide |
| 1K25 | 4-[4-[4-(3-Hydroxyphenyl)-3-methylbenzoyl]piperazin-1-yl]-N-propylbenzamide |
| 1K26 | 4-[4-[4-(5-Hydroxypyridin-3-yl)-3-methylbenzoyl]piperazin-1-yl]-N-propylbenzamide |
| 1K27 | 4-[4-[3-Fluoro-4-(3-hydroxyphenyl)benzoyl]piperazin-1-yl]-N-propylbenzamide |
| 1K28 | 4-[4-[3-Fluoro-4-(5-hydroxypyridin-3-yl)benzoyl]piperazin-1-yl]-N-propylbenzamide |
| 1K29 | 4-[4-[2-[2-(3-Hydroxyphenyl)ethynyl]benzoyl]piperazin-1-yl]-N-propylbenzamide |
| 1K30 | 4-[4-[2-[2-(5-Hydroxypyridin-3-yl)ethynyl]benzoyl]piperazin-1-yl]-N-propylbenzamide |
| 1K31 | 4-[4-[5-[2-(3-Hydroxyphenyl)ethynyl]pyridine-3-carbonyl]piperazin-1-yl]-N-propylbenzamide |
| 1K32 | 4-[4-[5-[2-(5-Hydroxypyridin-3-yl)ethynyl]pyridine-3-carbonyl]piperazin-1-yl]-N-propylbenzamide |
| 1K33 | 4-[4-[3-[2-(3-Hydroxyphenyl)phenyl]propanoyl]piperazin-1-yl]-N-propylbenzamide |
| 1K34 | 4-[4-[3-[2-(5-Hydroxypyridin-3-yl)phenyl]propanoyl]piperazin-1-yl]-N-propylbenzamide |
| 1K35 | 4-[4-[4-(3-Hydroxyphenyl)naphthalene-1-carbonyl]piperazin-1-yl]-N-propylbenzamide |
| 1K36 | 4-[4-[4-(5-Hydroxypyridin-3-yl)naphthalene-1-carbonyl]piperazin-1-yl]-N-propylbenzamide |
| 1K37 | 4-[4-[3-tert-Butyl-5-(3-hydroxyphenyl)benzoyl]piperazin-1-yl]-N-propylbenzamide |
| 1K38 | 4-[4-[3-tert-Butyl-5-(5-hydroxypyridin-3-yl)benzoyl]piperazin-1-yl]-N-propylbenzamide |
| 1K39 | 4-[4-[3-(3-Hydroxyphenyl)-5-(trifluoromethyl)benzoyl]piperazin-1-yl]-N-propylbenzamide |
| 1K40 | 4-[4-[3-(5-Hydroxypyridin-3-yl)-5-(trifluoromethyl)benzoyl]piperazin-1-yl]-N-propylbenzamide |
| 1K41 | 4-[4-[3-[2-(3-Hydroxyphenyl)ethynyl]-5-(trifluoromethyl)benzoyl]piperazin-1-yl]-N-propylbenzamide |
| 1K42 | 4-[4-[3-[2-(5-Hydroxypyridin-3-yl)ethynyl]-5-(trifluoromethyl)benzoyl]piperazin-1-yl]-N-propylbenzamide |
| 1K43 | 4-[4-[3-[4-(3-Hydroxyphenyl)pyrazol-1-yl]-5-(trifluoromethyl)benzoyl]piperazin-1-yl]-N-propylbenzamide |
| 1K44 | 4-[4-[3-[4-(5-Hydroxypyridin-3-yl)pyrazol-1-yl]-5-(trifluoromethyl)benzoyl]piperazin-1-yl]-N-propylbenzamide |
| 1K45 | 4-[4-[[2-(3-Hydroxyphenyl)phenyl]methyl]piperazin-1-yl]-N-propylbenzamide |
| 1K46 | 4-[4-[[2-(5-Hydroxypyridin-3-yl)phenyl]methyl]piperazin-1-yl]-N-propylbenzamide |
| 1K47 | 4-[4-[[4-(3-Hydroxyphenyl)-3-methylphenyl]methyl]piperazin-1-yl]-N-propylbenzamide |
| 1K48 | 4-[4-[[4-(5-Hydroxypyridin-3-yl)-3-methylphenyl]methyl]piperazin-1-yl]-N-propylbenzamide |
| 1K49 | 4-[4-[[2-Ethyl-4-(3-hydroxyphenyl)phenyl]methyl]piperazin-1-yl]-N-propylbenzamide |
| 1K50 | 4-[4-[[2-Ethyl-4-(5-hydroxypyridin-3-yl)phenyl]methyl]piperazin-1-yl]-N-propylbenzamide |
| 1K51 | 4-[4-[[3-Fluoro-4-(3-hydroxyphenyl)phenyl]methyl]piperazin-1-yl]-N-propylbenzamide |

| | |
|---|---|
| 1K52 | 4-[4-[[3-Fluoro-4-(5-hydroxypyridin-3-yl)phenyl]methyl]piperazin-1-yl]-N-propylbenzamide |
| 1K53 | 4-[4-[[4-(3-Hydroxyphenyl)-2-methoxyphenyl]methyl]piperazin-1-yl]-N-propylbenzamide |
| 1K54 | 4-[4-[[4-(5-Hydroxypyridin-3-yl)-2-methoxyphenyl]methyl]piperazin-1-yl]-N-propylbenzamide |
| 1K55 | 4-[4-[[3-Ethoxy-5-(3-hydroxyphenyl)phenyl]methyl]piperazin-1-yl]-N-propylbenzamide |
| 1K56 | 4-[4-[[3-Ethoxy-5-(5-hydroxypyridin-3-yl)phenyl]methyl]piperazin-1-yl]-N-propylbenzamide |
| 1K57 | 4-[4-[[3-(3-Hydroxyphenyl)-5-propan-2-yloxyphenyl]methyl]piperazin-1-yl]-N-propylbenzamide |
| 1K58 | 4-[4-[[3-(5-Hydroxypyridin-3-yl)-5-propan-2-yloxyphenyl]methyl]piperazin-1-yl]-N-propylbenzamide |
| 1K59 | 4-[4-[[4-(3-Hydroxyphenyl)-2,6-dimethoxyphenyl]methyl]piperazin-1-yl]-N-propylbenzamide |
| 1K60 | 4-[4-[[4-(5-Hydroxypyridin-3-yl)-2,6-dimethoxyphenyl]methyl]piperazin-1-yl]-N-propylbenzamide |
| 1K61 | 4-[4-[[3-(3-Hydroxyphenyl)-5-(trifluoromethyl)phenyl]methyl]piperazin-1-yl]-N-propylbenzamide |
| 1K62 | 4-[4-[[3-(5-Hydroxypyridin-3-yl)-5-(trifluoromethyl)phenyl]methyl]piperazin-1-yl]-N-propylbenzamide |
| 1K63 | 4-[4-[[3-(3-Hydroxyphenyl)-5-(trifluoromethoxy)phenyl]methyl]piperazin-1-yl]-N-propylbenzamide |
| 1K64 | 4-[4-[[3-(5-Hydroxypyridin-3-yl)-5-(trifluoromethoxy)phenyl]methyl]piperazin-1-yl]-N-propylbenzamide |
| 1K65 | 4-[4-[[4-(3-Hydroxyphenyl)naphthalen-1-yl]methyl]piperazin-1-yl]-N-propylbenzamide |
| 1K66 | 4-[4-[[4-(5-Hydroxypyridin-3-yl)naphthalen-1-yl]methyl]piperazin-1-yl]-N-propylbenzamide |
| 1K67 | 4-[4-[[4-(3-Hydroxyphenyl)thiophen-2-yl]methyl]piperazin-1-yl]-N-propylbenzamide |
| 1K68 | 4-[4-[[4-(5-Hydroxypyridin-3-yl)thiophen-2-yl]methyl]piperazin-1-yl]-N-propylbenzamide |
| 1K69 | 4-[4-[[2-[2-(3-Hydroxyphenyl)ethynyl]phenyl]methyl]piperazin-1-yl]-N-propylbenzamide |
| 1K70 | 4-[4-[[2-[2-(5-Hydroxypyridin-3-yl)ethynyl]phenyl]methyl]piperazin-1-yl]-N-propylbenzamide |
| 1K71 | 6-[4-[[2-(3-Hydroxyphenyl)phenyl]methyl]piperazin-1-yl]-N-propylpyridazine-3-carboxamide |
| 1K72 | 6-[4-[[2-(5-Hydroxypyridin-3-yl)phenyl]methyl]piperazin-1-yl]-N-propylpyridazine-3-carboxamide |
| 1K73 | 6-[4-[[4-(3-Hydroxyphenyl)-3-methylphenyl]methyl]piperazin-1-yl]-N-propylpyridazine-3-carboxamide |
| 1K74 | 6-[4-[[4-(5-Hydroxypyridin-3-yl)-3-methylphenyl]methyl]piperazin-1-yl]-N-propylpyridazine-3-carboxamide |
| 1K75 | 6-[4-[[2-Ethyl-4-(3-hydroxyphenyl)phenyl]methyl]piperazin-1-yl]-N-propylpyridazine-3-carboxamide |
| 1K76 | 6-[4-[[2-Ethyl-4-(5-hydroxypyridin-3-yl)phenyl]methyl]piperazin-1-yl]-N-propylpyridazine-3-carboxamide |
| 1K77 | 6-[4-[[3-Fluoro-4-(3-hydroxyphenyl)phenyl]methyl]piperazin-1-yl]-N-propylpyridazine-3-carboxamide |
| 1K78 | 6-[4-[[3-Fluoro-4-(5-hydroxypyridin-3-yl)phenyl]methyl]piperazin-1-yl]-N-propylpyridazine-3-carboxamide |
| 1K79 | 6-[4-[[4-(3-Hydroxyphenyl)-2-methoxyphenyl]methyl]piperazin-1-yl]-N-propylpyridazine-3-carboxamide |

| | |
|---|---|
| 1K80 | 6-[4-[[4-(5-Hydroxypyridin-3-yl)-2-methoxyphenyl]methyl]piperazin-1-yl]-N-propylpyridazine-3-carboxamide |
| 1K81 | 6-[4-[[3-Ethoxy-5-(3-hydroxyphenyl)phenyl]methyl]piperazin-1-yl]-N-propylpyridazine-3-carboxamide |
| 1K82 | 6-[4-[[3-Ethoxy-5-(5-hydroxypyridin-3-yl)phenyl]methyl]piperazin-1-yl]-N-propylpyridazine-3-carboxamide |
| 1K83 | 6-[4-[[3-(3-Hydroxyphenyl)-5-propan-2-yloxyphenyl]methyl]piperazin-1-yl]-N-propylpyridazine-3-carboxamide |
| 1K84 | 6-[4-[[3-(5-Hydroxypyridin-3-yl)-5-propan-2-yloxyphenyl]methyl]piperazin-1-yl]-N-propylpyridazine-3-carboxamide |
| 1K85 | 6-[4-[[4-(3-Hydroxyphenyl)-2,6-dimethoxyphenyl]methyl]piperazin-1-yl]-N-propylpyridazine-3-carboxamide |
| 1K86 | 6-[4-[[4-(5-Hydroxypyridin-3-yl)-2,6-dimethoxyphenyl]methyl]piperazin-1-yl]-N-propylpyridazine-3-carboxamide |
| 1K87 | 6-[4-[[3-(3-Hydroxyphenyl)-5-(trifluoromethyl)phenyl]methyl]piperazin-1-yl]-N-propylpyridazine-3-carboxamide |
| 1K88 | 6-[4-[[3-(5-Hydroxypyridin-3-yl)-5-(trifluoromethyl)phenyl]methyl]piperazin-1-yl]-N-propylpyridazine-3-carboxamide |
| 1K89 | 6-[4-[[3-(3-Hydroxyphenyl)-5-(trifluoromethoxy)phenyl]methyl]piperazin-1-yl]-N-propylpyridazine-3-carboxamide |
| 1K90 | 6-[4-[[3-(5-Hydroxypyridin-3-yl)-5-(trifluoromethoxy)phenyl]methyl]piperazin-1-yl]-N-propylpyridazine-3-carboxamide |
| 1K91 | 6-[4-[[4-(3-Hydroxyphenyl)naphthalen-1-yl]methyl]piperazin-1-yl]-N-propylpyridazine-3-carboxamide |
| 1K92 | 6-[4-[[4-(5-Hydroxypyridin-3-yl)naphthalen-1-yl]methyl]piperazin-1-yl]-N-propylpyridazine-3-carboxamide |
| 1K93 | 6-[4-[[4-(3-Hydroxyphenyl)thiophen-2-yl]methyl]piperazin-1-yl]-N-propylpyridazine-3-carboxamide |
| 1K94 | 6-[4-[[4-(5-Hydroxypyridin-3-yl)thiophen-2-yl]methyl]piperazin-1-yl]-N-propylpyridazine-3-carboxamide |
| 1K95 | 6-[4-[[2-[2-(3-Hydroxyphenyl)ethynyl]phenyl]methyl]piperazin-1-yl]-N-propylpyridazine-3-carboxamide |
| 1K96 | 6-[4-[[2-[2-(5-Hydroxypyridin-3-yl)ethynyl]phenyl]methyl]piperazin-1-yl]-N-propylpyridazine-3-carboxamide |
| 1K97 | 4-[4-[1-[4-(3-Hydroxyphenyl)phenyl]ethyl]piperazin-1-yl]-N-propylbenzamide |
| 1K98 | 4-[4-[1-[4-(5-Hydroxypyridin-3-yl)phenyl]ethyl]piperazin-1-yl]-N-propylbenzamide |
| 1K99 | 4-[4-[1-[2-Fluoro-4-(3-hydroxyphenyl)phenyl]ethyl]piperazin-1-yl]-N-propylbenzamide |
| 1K100 | 4-[4-[1-[2-Fluoro-4-(5-hydroxypyridin-3-yl)phenyl]ethyl]piperazin-1-yl]-N-propylbenzamide |

[Table 218]

[Table EIJ04]

| No. of mixture used | mixEI10-02R |
|---|---|
| Mixture No after cleavage | mixEJ04 |
| No. of compound in Table EJ04 | Compound name |
| 1O01 | 6-[4-[4-(3-Hydroxyphenyl)thiophene-2-carbonyl]piperazin-1-yl]-N-(4-methoxyphenyl)pyridazine-3-carboxamide |
| 1O02 | 6-[4-[4-(5-Hydroxypyridin-3-yl)thiophene-2-carbonyl]piperazin-1-yl]-N-(4-methoxyphenyl)pyridazine-3-carboxamide |
| 1O03 | 6-[4-[4-(3-Hydroxyphenyl)-3-methylbenzoyl]piperazin-1-yl]-N-(4-methoxyphenyl)pyridazine-3-carboxamide |

| | |
|---|---|
| 1O04 | 6-[4-[4-(5-Hydroxypyridin-3-yl)-3-methylbenzoyl]piperazin-1-yl]-N-(4-methoxyphenyl)pyridazine-3-carboxamide |
| 1O05 | 6-[4-[3-Fluoro-4-(3-hydroxyphenyl)benzoyl]piperazin-1-yl]-N-(4-methoxyphenyl)pyridazine-3-carboxamide |
| 1O06 | 6-[4-[3-Fluoro-4-(5-hydroxypyridin-3-yl)benzoyl]piperazin-1-yl]-N-(4-methoxyphenyl)pyridazine-3-carboxamide |
| 1O07 | 6-[4-[2-[2-(3-Hydroxyphenyl)ethynyl]benzoyl]piperazin-1-yl]-N-(4-methoxyphenyl)pyridazine-3-carboxamide |
| 1O08 | 6-[4-[2-[2-(5-Hydroxypyridin-3-yl)ethynyl]benzoyl]piperazin-1-yl]-N-(4-methoxyphenyl)pyridazine-3-carboxamide |
| 1O09 | 6-[4-[5-[2-(3-Hydroxyphenyl)ethynyl]pyridine-3-carbonyl]piperazin-1-yl]-N-(4-methoxyphenyl)pyridazine-3-carboxamide |
| 1O10 | 6-[4-[5-[2-(5-Hydroxypyridin-3-yl)ethynyl]pyridine-3-carbonyl]piperazin-1-yl]-N-(4-methoxyphenyl)pyridazine-3-carboxamide |
| 1O11 | 6-[4-[3-[2-(3-Hydroxyphenyl)phenyl]propanoyl]piperazin-1-yl]-N-(4-methoxyphenyl)pyridazine-3-carboxamide |
| 1O12 | 6-[4-[3-[2-(5-Hydroxypyridin-3-yl)phenyl]propanoyl]piperazin-1-yl]-N-(4-methoxyphenyl)pyridazine-3-carboxamide |
| 1O13 | 6-[4-[4-(3-Hydroxyphenyl)naphthalene-1-carbonyl]piperazin-1-yl]-N-(4-methoxyphenyl)pyridazine-3-carboxamide |
| 1O14 | 6-[4-[4-(5-Hydroxypyridin-3-yl)naphthalene-1-carbonyl]piperazin-1-yl]-N-(4-methoxyphenyl)pyridazine-3-carboxamide |
| 1O15 | 6-[4-[3-tert-Butyl-5-(3-hydroxyphenyl)benzoyl]piperazin-1-yl]-N-(4-methoxyphenyl)pyridazine-3-carboxamide |
| 1O16 | 6-[4-[3-tert-Butyl-5-(5-hydroxypyridin-3-yl)benzoyl]piperazin-1-yl]-N-(4-methoxyphenyl)pyridazine-3-carboxamide |
| 1O17 | 6-[4-[3-(3-Hydroxyphenyl)-5-(trifluoromethyl)benzoyl]piperazin-1-yl]-N-(4-methoxyphenyl)pyridazine-3-carboxamide |
| 1O18 | 6-[4-[3-(5-Hydroxypyridin-3-yl)-5-(trifluoromethyl)benzoyl]piperazin-1-yl]-N-(4-methoxyphenyl)pyridazine-3-carboxamide |
| 1O19 | 6-[4-[3-[2-(3-Hydroxyphenyl)ethynyl]-5-(trifluoromethyl)benzoyl]piperazin-1-yl]-N-(4-methoxyphenyl)pyridazine-3-carboxamide |
| 1O20 | 6-[4-[3-[2-(5-Hydroxypyridin-3-yl)ethynyl]-5-(trifluoromethyl)benzoyl]piperazin-1-yl]-N-(4-methoxyphenyl)pyridazine-3-carboxamide |
| 1O21 | 6-[4-[3-[4-(3-Hydroxyphenyl)pyrazol-1-yl]-5-(trifluoromethyl)benzoyl]piperazin-1-yl]-N-(4-methoxyphenyl)pyridazine-3-carboxamide |
| 1O22 | 6-[4-[3-[4-(5-Hydroxypyridin-3-yl)pyrazol-1-yl]-5-(trifluoromethyl)benzoyl]piperazin-1-yl]-N-(4-methoxyphenyl)pyridazine-3-carboxamide |
| 1O23 | 4-[4-[4-(3-Hydroxyphenyl)thiophene-2-carbonyl]piperazin-1-yl]-N-(4-methoxyphenyl)benzamide |
| 1O24 | 4-[4-[4-(5-Hydroxypyridin-3-yl)thiophene-2-carbonyl]piperazin-1-yl]-N-(4-methoxyphenyl)benzamide |
| 1O25 | 4-[4-[4-(3-Hydroxyphenyl)-3-methylbenzoyl]piperazin-1-yl]-N-(4-methoxyphenyl)benzamide |
| 1O26 | 4-[4-[4-(5-Hydroxypyridin-3-yl)-3-methylbenzoyl]piperazin-1-yl]-N-(4-methoxyphenyl)benzamide |
| 1O27 | 4-[4-[3-Fluoro-4-(3-hydroxyphenyl)benzoyl]piperazin-1-yl]-N-(4-methoxyphenyl)benzamide |
| 1O28 | 4-[4-[3-Fluoro-4-(5-hydroxypyridin-3-yl)benzoyl]piperazin-1-yl]-N-(4-methoxyphenyl)benzamide |
| 1O29 | 4-[4-[2-[2-(3-Hydroxyphenyl)ethynyl]benzoyl]piperazin-1-yl]-N-(4-methoxyphenyl)benzamide |
| 1O30 | 4-[4-[2-[2-(5-Hydroxypyridin-3-yl)ethynyl]benzoyl]piperazin-1-yl]-N-(4-methoxyphenyl)benzamide |
| 1O31 | 4-[4-[5-[2-(3-Hydroxyphenyl)ethynyl]pyridine-3-carbonyl]piperazin-1-yl]-N-(4-methoxyphenyl)benzamide |

| | |
|---|---|
| 1O32 | 4-[4-[5-[2-(5-Hydroxypyridin-3-yl)ethynyl]pyridine-3-carbonyl]piperazin-1-yl]-N-(4-methoxyphenyl)benzamide |
| 1O33 | 4-[4-[3-[2-(3-Hydroxyphenyl)phenyl]propanoyl]piperazin-1-yl]-N-(4-methoxyphenyl)benzamide |
| 1O34 | 4-[4-[3-[2-(5-Hydroxypyridin-3-yl)phenyl]propanoyl]piperazin-1-yl]-N-(4-methoxyphenyl)benzamide |
| 1O35 | 4-[4-[4-(3-Hydroxyphenyl)naphthalene-1-carbonyl]piperazin-1-yl]-N-(4-methoxyphenyl)benzamide |
| 1O36 | 4-[4-[4-(5-Hydroxypyridin-3-yl)naphthalene-1-carbonyl]piperazin-1-yl]-N-(4-methoxyphenyl)benzamide |
| 1O37 | 4-[4-[3-tert-Butyl-5-(3-hydroxyphenyl)benzoyl]piperazin-1-yl]-N-(4-methoxyphenyl)benzamide |
| 1O38 | 4-[4-[3-tert-Butyl-5-(5-hydroxypyridin-3-yl)benzoyl]piperazin-1-yl]-N-(4-methoxyphenyl)benzamide |
| 1O39 | 4-[4-[3-(3-Hydroxyphenyl)-5-(trifluoromethyl)benzoyl]piperazin-1-yl]-N-(4-methoxyphenyl)benzamide |
| 1O40 | 4-[4-[3-(5-Hydroxypyridin-3-yl)-5-(trifluoromethyl)benzoyl]piperazin-1-yl]-N-(4-methoxyphenyl)benzamide |
| 1O41 | 4-[4-[3-[2-(3-Hydroxyphenyl)ethynyl]-5-(trifluoromethyl)benzoyl]piperazin-1-yl]-N-(4-methoxyphenyl)benzamide |
| 1O42 | 4-[4-[3-[2-(5-Hydroxypyridin-3-yl)ethynyl]-5-(trifluoromethyl)benzoyl]piperazin-1-yl]-N-(4-methoxyphenyl)benzamide |
| 1O43 | 4-[4-[3-[4-(3-Hydroxyphenyl)pyrazol-1-yl]-5-(trifluoromethyl)benzoyl]piperazin-1-yl]-N-(4-methoxyphenyl)benzamide |
| 1O44 | 4-[4-[3-[4-(5-Hydroxypyridin-3-yl)pyrazol-1-yl]-5-(trifluoromethyl)benzoyl]piperazin-1-yl]-N-(4-methoxyphenyl)benzamide |
| 1O45 | 4-[4-[[2-(3-Hydroxyphenyl)phenyl]methyl]piperazin-1-yl]-N-(4-methoxyphenyl)benzamide |
| 1O46 | 4-[4-[[2-(5-Hydroxypyridin-3-yl)phenyl]methyl]piperazin-1-yl]-N-(4-methoxyphenyl)benzamide |
| 1O47 | 4-[4-[[4-(3-Hydroxyphenyl)-3-methylphenyl]methyl]piperazin-1-yl]-N-(4-methoxyphenyl)benzamide |
| 1O48 | 4-[4-[[4-(5-Hydroxypyridin-3-yl)-3-methylphenyl]methyl]piperazin-1-yl]-N-(4-methoxyphenyl)benzamide |
| 1O49 | 4-[4-[[2-Ethyl-4-(3-hydroxyphenyl)phenyl]methyl]piperazin-1-yl]-N-(4-methoxyphenyl)benzamide |
| 1O50 | 4-[4-[[2-Ethyl-4-(5-hydroxypyridin-3-yl)phenyl]methyl]piperazin-1-yl]-N-(4-methoxyphenyl)benzamide |
| 1O51 | 4-[4-[[3-Fluoro-4-(3-hydroxyphenyl)phenyl]methyl]piperazin-1-yl]-N-(4-methoxyphenyl)benzamide |
| 1O52 | 4-[4-[[3-Fluoro-4-(5-hydroxypyridin-3-yl)phenyl]methyl]piperazin-1-yl]-N-(4-methoxyphenyl)benzamide |
| 1O53 | 4-[4-[[4-(3-Hydroxyphenyl)-2-methoxyphenyl]methyl]piperazin-1-yl]-N-(4-methoxyphenyl)benzamide |
| 1O54 | 4-[4-[[4-(5-Hydroxypyridin-3-yl)-2-methoxyphenyl]methyl]piperazin-1-yl]-N-(4-methoxyphenyl)benzamide |
| 1O55 | 4-[4-[[3-Ethoxy-5-(3-hydroxyphenyl)phenyl]methyl]piperazin-1-yl]-N-(4-methoxyphenyl)benzamide |
| 1O56 | 4-[4-[[3-Ethoxy-5-(5-hydroxypyridin-3-yl)phenyl]methyl]piperazin-1-yl]-N-(4-methoxyphenyl)benzamide |
| 1O57 | 4-[4-[[3-(3-Hydroxyphenyl)-5-propan-2-yloxyphenyl]methyl]piperazin-1-yl]-N-(4-methoxyphenyl)benzamide |
| 1O58 | 4-[4-[[3-(5-Hydroxypyridin-3-yl)-5-propan-2-yloxyphenyl]methyl]piperazin-1-yl]-N-(4-methoxyphenyl)benzamide |
| 1O59 | 4-[4-[[4-(3-Hydroxyphenyl)-2,6-dimethoxyphenyl]methyl]piperazin-1-yl]-N-(4-methoxyphenyl)benzamide |

| | |
|---|---|
| 1O60 | 4-[4-[[4-(5-Hydroxypyridin-3-yl)-2,6-dimethoxyphenyl]methyl]piperazin-1-yl]-N-(4-methoxyphenyl)benzamide |
| 1O61 | 4-[4-[[3-(3-Hydroxyphenyl)-5-(trifluoromethyl)phenyl]methyl]piperazin-1-yl]-N-(4-methoxyphenyl)benzamide |
| 1O62 | 4-[4-[[3-(5-Hydroxypyridin-3-yl)-5-(trifluoromethyl)phenyl]methyl]piperazin-1-yl]-N-(4-methoxyphenyl)benzamide |
| 1O63 | 4-[4-[[3-(3-Hydroxyphenyl)-5-(trifluoromethoxy)phenyl]methyl]piperazin-1-yl]-N-(4-methoxyphenyl)benzamide |
| 1O64 | 4-[4-[[3-(5-Hydroxypyridin-3-yl)-5-(trifluoromethoxy)phenyl]methyl]piperazin-1-yl]-N-(4-methoxyphenyl)benzamide |
| 1O65 | 4-[4-[[4-(3-Hydroxyphenyl)naphthalen-1-yl]methyl]piperazin-1-yl]-N-(4-methoxyphenyl)benzamide |
| 1O66 | 4-[4-[[4-(5-Hydroxypyridin-3-yl)naphthalen-1-yl]methyl]piperazin-1-yl]-N-(4-methoxyphenyl)benzamide |
| 1O67 | 4-[4-[[4-(3-Hydroxyphenyl)thiophen-2-yl]methyl]piperazin-1-yl]-N-(4-methoxyphenyl)benzamide |
| 1O68 | 4-[4-[[4-(5-Hydroxypyridin-3-yl)thiophen-2-yl]methyl]piperazin-1-yl]-N-(4-methoxyphenyl)benzamide |
| 1O69 | 4-[4-[[2-[2-(3-Hydroxyphenyl)ethynyl]phenyl]methyl]piperazin-1-yl]-N-(4-methoxyphenyl)benzamide |
| 1O70 | 4-[4-[[2-[2-(5-Hydroxypyridin-3-yl)ethynyl]phenyl]methyl]piperazin-1-yl]-N-(4-methoxyphenyl)benzamide |
| 1O71 | 6-[4-[[2-(3-Hydroxyphenyl)phenyl]methyl]piperazin-1-yl]-N-(4-methoxyphenyl)pyridazine-3-carboxamide |
| 1O72 | 6-[4-[[2-(5-Hydroxypyridin-3-yl)phenyl]methyl]piperazin-1-yl]-N-(4-methoxyphenyl)pyridazine-3-carboxamide |
| 1O73 | 6-[4-[[4-(3-Hydroxyphenyl)-3-methylphenyl]methyl]piperazin-1-yl]-N-(4-methoxyphenyl)pyridazine-3-carboxamide |
| 1O74 | 6-[4-[[4-(5-Hydroxypyridin-3-yl)-3-methylphenyl]methyl]piperazin-1-yl]-N-(4-methoxyphenyl)pyridazine-3-carboxamide |
| 1O75 | 6-[4-[[2-Ethyl-4-(3-hydroxyphenyl)phenyl]methyl]piperazin-1-yl]-N-(4-methoxyphenyl)pyridazine-3-carboxamide |
| 1O76 | 6-[4-[[2-Ethyl-4-(5-hydroxypyridin-3-yl)phenyl]methyl]piperazin-1-yl]-N-(4-methoxyphenyl)pyridazine-3-carboxamide |
| 1O77 | 6-[4-[[3-Fluoro-4-(3-hydroxyphenyl)phenyl]methyl]piperazin-1-yl]-N-(4-methoxyphenyl)pyridazine-3-carboxamide |
| 1O78 | 6-[4-[[3-Fluoro-4-(5-hydroxypyridin-3-yl)phenyl]methyl]piperazin-1-yl]-N-(4-methoxyphenyl)pyridazine-3-carboxamide |
| 1O79 | 6-[4-[[4-(3-Hydroxyphenyl)-2-methoxyphenyl]methyl]piperazin-1-yl]-N-(4-methoxyphenyl)pyridazine-3-carboxamide |
| 1O80 | 6-[4-[[4-(5-Hydroxypyridin-3-yl)-2-methoxyphenyl]methyl]piperazin-1-yl]-N-(4-methoxyphenyl)pyridazine-3-carboxamide |
| 1O81 | 6-[4-[[3-Ethoxy-5-(3-hydroxyphenyl)phenyl]methyl]piperazin-1-yl]-N-(4-methoxyphenyl)pyridazine-3-carboxamide |
| 1O82 | 6-[4-[[3-Ethoxy-5-(5-hydroxypyridin-3-yl)phenyl]methyl]piperazin-1-yl]-N-(4-methoxyphenyl)pyridazine-3-carboxamide |
| 1O83 | 6-[4-[[3-(3-Hydroxyphenyl)-5-propan-2-yloxyphenyl]methyl]piperazin-1-yl]-N-(4-methoxyphenyl)pyridazine-3-carboxamide |
| 1O84 | 6-[4-[[3-(5-Hydroxypyridin-3-yl)-5-propan-2-yloxyphenyl]methyl]piperazin-1-yl]-N-(4-methoxyphenyl)pyridazine-3-carboxamide |
| 1O85 | 6-[4-[[4-(3-Hydroxyphenyl)-2,6-dimethoxyphenyl]methyl]piperazin-1-yl]-N-(4-methoxyphenyl)pyridazine-3-carboxamide |
| 1O86 | 6-[4-[[4-(5-Hydroxypyridin-3-yl)-2,6-dimethoxyphenyl]methyl]piperazin-1-yl]-N-(4-methoxyphenyl)pyridazine-3-carboxamide |
| 1O87 | 6-[4-[[3-(3-Hydroxyphenyl)-5-(trifluoromethyl)phenyl]methyl]piperazin-1-yl]-N-(4-methoxyphenyl)pyridazine-3-carboxamide |

| | |
|---|---|
| 1O88 | 6-[4-[[3-(5-Hydroxypyridin-3-yl)-5-(trifluoromethyl)phenyl]methyl]piperazin-1-yl]-N-(4-methoxyphenyl)pyridazine-3-carboxamide |
| 1O89 | 6-[4-[[3-(3-Hydroxyphenyl)-5-(trifluoromethoxy)phenyl]methyl]piperazin-1-yl]-N-(4-methoxyphenyl)pyridazine-3-carboxamide |
| 1O90 | 6-[4-[[3-(5-Hydroxypyridin-3-yl)-5-(trifluoromethoxy)phenyl]methyl]piperazin-1-yl]-N-(4-methoxyphenyl)pyridazine-3-carboxamide |
| 1O91 | 6-[4-[[4-(3-Hydroxyphenyl)naphthalen-1-yl]methyl]piperazin-1-yl]-N-(4-methoxyphenyl)pyridazine-3-carboxamide |
| 1O92 | 6-[4-[[4-(5-Hydroxypyridin-3-yl)naphthalen-1-yl]methyl]piperazin-1-yl]-N-(4-methoxyphenyl)pyridazine-3-carboxamide |
| 1O93 | 6-[4-[[4-(3-Hydroxyphenyl)thiophen-2-yl]methyl]piperazin-1-yl]-N-(4-methoxyphenyl)pyridazine-3-carboxamide |
| 1O94 | 6-[4-[[4-(5-Hydroxypyridin-3-yl)thiophen-2-yl]methyl]piperazin-1-yl]-N-(4-methoxyphenyl)pyridazine-3-carboxamide |
| 1O95 | 6-[4-[[2-[2-(3-Hydroxyphenyl)ethynyl]phenyl]methyl]piperazin-1-yl]-N-(4-methoxyphenyl)pyridazine-3-carboxamide |
| 1O96 | 6-[4-[[2-[2-(5-Hydroxypyridin-3-yl)ethynyl]phenyl]methyl]piperazin-1-yl]-N-(4-methoxyphenyl)pyridazine-3-carboxamide |
| 1O97 | 4-[4-[1-[4-(3-Hydroxyphenyl)phenyl]ethyl]piperazin-1-yl]-N-(4-methoxyphenyl)benzamide |
| 1O98 | 4-[4-[1-[4-(5-Hydroxypyridin-3-yl)phenyl]ethyl]piperazin-1-yl]-N-(4-methoxyphenyl)benzamide |
| 1O99 | 4-[4-[1-[2-Fluoro-4-(3-hydroxyphenyl)phenyl]ethyl]piperazin-1-yl]-N-(4-methoxyphenyl)benzamide |
| 1O100 | 4-[4-[1-[2-Fluoro-4-(5-hydroxypyridin-3-yl)phenyl]ethyl]piperazin-1-yl]-N-(4-methoxyphenyl)benzamide |

[Table 219]

[Table EIJ05]

| No. of mixture used | mixEI11-02R |
|---|---|
| Mixture No after cleavage | mixEJ05 |
| No. of compound in Table EJ05 | Compound name |
| 1A01 | N-(1-Adamantylmethyl)-6-[4-[4-(3-hydroxyphenyl)thiophene-2-carbonyl]piperazin-1-yl]pyridazine-3-carboxamide |
| 1A02 | N-(1-Adamantylmethyl)-6-[4-[4-(5-hydroxypyridin-3-yl)thiophene-2-carbonyl]piperazin-1-yl]pyridazine-3-carboxamide |
| 1A03 | N-(1-Adamantylmethyl)-6-[4-[4-(3-hydroxyphenyl)-3-methylbenzoyl]piperazin-1-yl]pyridazine-3-carboxamide |
| 1A04 | N-(1-Adamantylmethyl)-6-[4-[4-(5-hydroxypyridin-3-yl)-3-methylbenzoyl]piperazin-1-yl]pyridazine-3-carboxamide |
| 1A05 | N-(1-Adamantylmethyl)-6-[4-[3-fluoro-4-(3-hydroxyphenyl)benzoyl]piperazin-1-yl]pyridazine-3-carboxamide |
| 1A06 | N-(1-Adamantylmethyl)-6-[4-[3-fluoro-4-(5-hydroxypyridin-3-yl)benzoyl]piperazin-1-yl]pyridazine-3-carboxamide |
| 1A07 | N-(1-Adamantylmethyl)-6-[4-[2-[2-(3-hydroxyphenyl)ethynyl]benzoyl]piperazin-1-yl]pyridazine-3-carboxamide |
| 1A08 | N-(1-Adamantylmethyl)-6-[4-[2-[2-(5-hydroxypyridin-3-yl)ethynyl]benzoyl]piperazin-1-yl]pyridazine-3-carboxamide |
| 1A09 | N-(1-Adamantylmethyl)-6-[4-[5-[2-(3-hydroxyphenyl)ethynyl]pyridine-3-carbonyl]piperazin-1-yl]pyridazine-3-carboxamide |
| 1A10 | N-(1-Adamantylmethyl)-6-[4-[5-[2-(5-hydroxypyridin-3-yl)ethynyl]pyridine-3-carbonyl]piperazin-1-yl]pyridazine-3-carboxamide |

| | |
|---|---|
| 1A11 | N-(1-Adamantylmethyl)-6-[4-[3-[2-(3-hydroxyphenyl)phenyl]propanoyl]piperazin-1-yl]pyridazine-3-carboxamide |
| 1A12 | N-(1-Adamantylmethyl)-6-[4-[3-[2-(5-hydroxypyridin-3-yl)phenyl]propanoyl]piperazin-1-yl]pyridazine-3-carboxamide |
| 1A13 | N-(1-Adamantylmethyl)-6-[4-[4-(3-hydroxyphenyl)naphthalene-1-carbonyl]piperazin-1-yl]pyridazine-3-carboxamide |
| 1A14 | N-(1-Adamantylmethyl)-6-[4-[4-(5-hydroxypyridin-3-yl)naphthalene-1-carbonyl]piperazin-1-yl]pyridazine-3-carboxamide |
| 1A15 | N-(1-Adamantylmethyl)-6-[4-[3-tert-butyl-5-(3-hydroxyphenyl)benzoyl]piperazin-1-yl]pyridazine-3-carboxamide |
| 1A16 | N-(1-Adamantylmethyl)-6-[4-[3-tert-butyl-5-(5-hydroxypyridin-3-yl)benzoyl]piperazin-1-yl]pyridazine-3-carboxamide |
| 1A17 | N-(1-Adamantylmethyl)-6-[4-[3-(3-hydroxyphenyl)-5-(trifluoromethyl)benzoyl]piperazin-1-yl]pyridazine-3-carboxamide |
| 1A18 | N-(1-Adamantylmethyl)-6-[4-[3-(5-hydroxypyridin-3-yl)-5-(trifluoromethyl)benzoyl]piperazin-1-yl]pyridazine-3-carboxamide |
| 1A19 | N-(1-Adamantylmethyl)-6-[4-[3-[2-(3-hydroxyphenyl)ethynyl]-5-(trifluoromethyl)benzoyl]piperazin-1-yl]pyridazine-3-carboxamide |
| 1A20 | N-(1-Adamantylmethyl)-6-[4-[3-[2-(5-hydroxypyridin-3-yl)ethynyl]-5-(trifluoromethyl)benzoyl]piperazin-1-yl]pyridazine-3-carboxamide |
| 1A21 | N-(1-Adamantylmethyl)-6-[4-[3-[4-(3-hydroxyphenyl)pyrazol-1-yl]-5-(trifluoromethyl)benzoyl]piperazin-1-yl]pyridazine-3-carboxamide |
| 1A22 | N-(1-Adamantylmethyl)-6-[4-[3-[4-(5-hydroxypyridin-3-yl)pyrazol-1-yl]-5-(trifluoromethyl)benzoyl]piperazin-1-yl]pyridazine-3-carboxamide |
| 1A23 | N-(1-Adamantylmethyl)-4-[4-[4-(3-hydroxyphenyl)thiophene-2-carbonyl]piperazin-1-yl]benzamide |
| 1A24 | N-(1-Adamantylmethyl)-4-[4-[4-(5-hydroxypyridin-3-yl)thiophene-2-carbonyl]piperazin-1-yl]benzamide |
| 1A25 | N-(1-Adamantylmethyl)-4-[4-[4-(3-hydroxyphenyl)-3-methylbenzoyl]piperazin-1-yl]benzamide |
| 1A26 | N-(1-Adamantylmethyl)-4-[4-[4-(5-hydroxypyridin-3-yl)-3-methylbenzoyl]piperazin-1-yl]benzamide |
| 1A27 | N-(1-Adamantylmethyl)-4-[4-[3-fluoro-4-(3-hydroxyphenyl)benzoyl]piperazin-1-yl]benzamide |
| 1A28 | N-(1-Adamantylmethyl)-4-[4-[3-fluoro-4-(5-hydroxypyridin-3-yl)benzoyl]piperazin-1-yl]benzamide |
| 1A29 | N-(1-Adamantylmethyl)-4-[4-[2-[2-(3-hydroxyphenyl)ethynyl]benzoyl]piperazin-1-yl]benzamide |
| 1A30 | N-(1-Adamantylmethyl)-4-[4-[2-[2-(5-hydroxypyridin-3-yl)ethynyl]benzoyl]piperazin-1-yl]benzamide |
| 1A31 | N-(1-Adamantylmethyl)-4-[4-[5-[2-(3-hydroxyphenyl)ethynyl]pyridine-3-carbonyl]piperazin-1-yl]benzamide |
| 1A32 | N-(1-Adamantylmethyl)-4-[4-[5-[2-(5-hydroxypyridin-3-yl)ethynyl]pyridine-3-carbonyl]piperazin-1-yl]benzamide |
| 1A33 | N-(1-Adamantylmethyl)-4-[4-[3-[2-(3-hydroxyphenyl)phenyl]propanoyl]piperazin-1-yl]benzamide |
| 1A34 | N-(1-Adamantylmethyl)-4-[4-[3-[2-(5-hydroxypyridin-3-yl)phenyl]propanoyl]piperazin-1-yl]benzamide |
| 1A35 | N-(1-Adamantylmethyl)-4-[4-[4-(3-hydroxyphenyl)naphthalene-1-carbonyl]piperazin-1-yl]benzamide |
| 1A36 | N-(1-Adamantylmethyl)-4-[4-[4-(5-hydroxypyridin-3-yl)naphthalene-1-carbonyl]piperazin-1-yl]benzamide |
| 1A37 | N-(1-Adamantylmethyl)-4-[4-[3-tert-butyl-5-(3-hydroxyphenyl)benzoyl]piperazin-1-yl]benzamide |
| 1A38 | N-(1-Adamantylmethyl)-4-[4-[3-tert-butyl-5-(5-hydroxypyridin-3-yl)benzoyl]piperazin-1-yl]benzamide |

| | |
|---|---|
| 1A39 | N-(1-Adamantylmethyl)-4-[4-[3-(3-hydroxyphenyl)-5-(trifluoromethyl)benzoyl]piperazin-1-yl]benzamide |
| 1A40 | N-(1-Adamantylmethyl)-4-[4-[3-(5-hydroxypyridin-3-yl)-5-(trifluoromethyl)benzoyl]piperazin-1-yl]benzamide |
| 1A41 | N-(1-Adamantylmethyl)-4-[4-[3-[2-(3-hydroxyphenyl)ethynyl]-5-(trifluoromethyl)benzoyl]piperazin-1-yl]benzamide |
| 1A42 | N-(1-Adamantylmethyl)-4-[4-[3-[2-(5-hydroxypyridin-3-yl)ethynyl]-5-(trifluoromethyl)benzoyl]piperazin-1-yl]benzamide |
| 1A43 | N-(1-Adamantylmethyl)-4-[4-[3-[4-(3-hydroxyphenyl)pyrazol-1-yl]-5-(trifluoromethyl)benzoyl]piperazin-1-yl]benzamide |
| 1A44 | N-(1-Adamantylmethyl)-4-[4-[3-[4-(5-hydroxypyridin-3-yl)pyrazol-1-yl]-5-(trifluoromethyl)benzoyl]piperazin-1-yl]benzamide |
| 1A45 | N-(1-Adamantylmethyl)-4-[4-[[2-(3-hydroxyphenyl)phenyl]methyl]piperazin-1-yl]benzamide |
| 1A46 | N-(1-Adamantylmethyl)-4-[4-[[2-(5-hydroxypyridin-3-yl)phenyl]methyl]piperazin-1-yl]benzamide |
| 1A47 | N-(1-Adamantylmethyl)-4-[4-[[4-(3-hydroxyphenyl)-3-methylphenyl]methyl]piperazin-1-yl]benzamide |
| 1A48 | N-(1-Adamantylmethyl)-4-[4-[[4-(5-hydroxypyridin-3-yl)-3-methylphenyl]methyl]piperazin-1-yl]benzamide |
| 1A49 | N-(1-Adamantylmethyl)-4-[4-[[2-ethyl-4-(3-hydroxyphenyl)phenyl]methyl]piperazin-1-yl]benzamide |
| 1A50 | N-(1-Adamantylmethyl)-4-[4-[[2-ethyl-4-(5-hydroxypyridin-3-yl)phenyl]methyl]piperazin-1-yl]benzamide |
| 1A51 | N-(1-Adamantylmethyl)-4-[4-[[3-fluoro-4-(3-hydroxyphenyl)phenyl]methyl]piperazin-1-yl]benzamide |
| 1A52 | N-(1-Adamantylmethyl)-4-[4-[[3-fluoro-4-(5-hydroxypyridin-3-yl)phenyl]methyl]piperazin-1-yl]benzamide |
| 1A53 | N-(1-Adamantylmethyl)-4-[4-[[4-(3-hydroxyphenyl)-2-methoxyphenyl]methyl]piperazin-1-yl]benzamide |
| 1A54 | N-(1-Adamantylmethyl)-4-[4-[[4-(5-hydroxypyridin-3-yl)-2-methoxyphenyl]methyl]piperazin-1-yl]benzamide |
| 1A55 | N-(1-Adamantylmethyl)-4-[4-[[3-ethoxy-5-(3-hydroxyphenyl)phenyl]methyl]piperazin-1-yl]benzamide |
| 1A56 | N-(1-Adamantylmethyl)-4-[4-[[3-ethoxy-5-(5-hydroxypyridin-3-yl)phenyl]methyl]piperazin-1-yl]benzamide |
| 1A57 | N-(1-Adamantylmethyl)-4-[4-[[3-(3-hydroxyphenyl)-5-propan-2-yloxyphenyl]methyl]piperazin-1-yl]benzamide |
| 1A58 | N-(1-Adamantylmethyl)-4-[4-[[3-(5-hydroxypyridin-3-yl)-5-propan-2-yloxyphenyl]methyl]piperazin-1-yl]benzamide |
| 1A59 | N-(1-Adamantylmethyl)-4-[4-[[4-(3-hydroxyphenyl)-2,6-dimethoxyphenyl]methyl]piperazin-1-yl]benzamide |
| 1A60 | N-(1-Adamantylmethyl)-4-[4-[[4-(5-hydroxypyridin-3-yl)-2,6-dimethoxyphenyl]methyl]piperazin-1-yl]benzamide |
| 1A61 | N-(1-Adamantylmethyl)-4-[4-[[3-(3-hydroxyphenyl)-5-(trifluoromethyl)phenyl]methyl]piperazin-1-yl]benzamide |
| 1A62 | N-(1-Adamantylmethyl)-4-[4-[[3-(5-hydroxypyridin-3-yl)-5-(trifluoromethyl)phenyl]methyl]piperazin-1-yl]benzamide |
| 1A63 | N-(1-Adamantylmethyl)-4-[4-[[3-(3-hydroxyphenyl)-5-(trifluoromethoxy)phenyl]methyl]piperazin-1-yl]benzamide |
| 1A64 | N-(1-Adamantylmethyl)-4-[4-[[3-(5-hydroxypyridin-3-yl)-5-(trifluoromethoxy)phenyl]methyl]piperazin-1-yl]benzamide |
| 1A65 | N-(1-Adamantylmethyl)-4-[4-[[4-(3-hydroxyphenyl)naphthalen-1-yl]methyl]piperazin-1-yl]benzamide |
| 1A66 | N-(1-Adamantylmethyl)-4-[4-[[4-(5-hydroxypyridin-3-yl)naphthalen-1-yl]methyl]piperazin-1-yl]benzamide |

| | |
|---|---|
| 1A67 | N-(1-Adamantylmethyl)-4-[4-[[4-(3-hydroxyphenyl)thiophen-2-yl]methyl]piperazin-1-yl]benzamide |
| 1A68 | N-(1-Adamantylmethyl)-4-[4-[[4-(5-hydroxypyridin-3-yl)thiophen-2-yl]methyl]piperazin-1-yl]benzamide |
| 1A69 | N-(1-Adamantylmethyl)-4-[4-[[2-[2-(3-hydroxyphenyl)ethynyl]phenyl]methyl]piperazin-1-yl]benzamide |
| 1A70 | N-(1-Adamantylmethyl)-4-[4-[[2-[2-(5-hydroxypyridin-3-yl)ethynyl]phenyl]methyl]piperazin-1-yl]benzamide |
| 1A71 | N-(1-Adamantylmethyl)-6-[4-[[2-(3-hydroxyphenyl)phenyl]methyl]piperazin-1-yl]pyridazine-3-carboxamide |
| 1A72 | N-(1-Adamantylmethyl)-6-[4-[[2-(5-hydroxypyridin-3-yl)phenyl]methyl]piperazin-1-yl]pyridazine-3-carboxamide |
| 1A73 | N-(1-Adamantylmethyl)-6-[4-[[4-(3-hydroxyphenyl)-3-methylphenyl]methyl]piperazin-1-yl]pyridazine-3-carboxamide |
| 1A74 | N-(1-Adamantylmethyl)-6-[4-[[4-(5-hydroxypyridin-3-yl)-3-methylphenyl]methyl]piperazin-1-yl]pyridazine-3-carboxamide |
| 1A75 | N-(1-Adamantylmethyl)-6-[4-[[2-ethyl-4-(3-hydroxyphenyl)phenyl]methyl]piperazin-1-yl]pyridazine-3-carboxamide |
| 1A76 | N-(1-Adamantylmethyl)-6-[4-[[2-ethyl-4-(5-hydroxypyridin-3-yl)phenyl]methyl]piperazin-1-yl]pyridazine-3-carboxamide |
| 1A77 | N-(1-Adamantylmethyl)-6-[4-[[3-fluoro-4-(3-hydroxyphenyl)phenyl]methyl]piperazin-1-yl]pyridazine-3-carboxamide |
| 1A78 | N-(1-Adamantylmethyl)-6-[4-[[3-fluoro-4-(5-hydroxypyridin-3-yl)phenyl]methyl]piperazin-1-yl]pyridazine-3-carboxamide |
| 1A79 | N-(1-Adamantylmethyl)-6-[4-[[4-(3-hydroxyphenyl)-2-methoxyphenyl]methyl]piperazin-1-yl]pyridazine-3-carboxamide |
| 1A80 | N-(1-Adamantylmethyl)-6-[4-[[4-(5-hydroxypyridin-3-yl)-2-methoxyphenyl]methyl]piperazin-1-yl]pyridazine-3-carboxamide |
| 1A81 | N-(1-Adamantylmethyl)-6-[4-[[3-ethoxy-5-(3-hydroxyphenyl)phenyl]methyl]piperazin-1-yl]pyridazine-3-carboxamide |
| 1A82 | N-(1-Adamantylmethyl)-6-[4-[[3-ethoxy-5-(5-hydroxypyridin-3-yl)phenyl]methyl]piperazin-1-yl]pyridazine-3-carboxamide |
| 1A83 | N-(1-Adamantylmethyl)-6-[4-[[3-(3-hydroxyphenyl)-5-propan-2-yloxyphenyl]methyl]piperazin-1-yl]pyridazine-3-carboxamide |
| 1A84 | N-(1-Adamantylmethyl)-6-[4-[[3-(5-hydroxypyridin-3-yl)-5-propan-2-yloxyphenyl]methyl]piperazin-1-yl]pyridazine-3-carboxamide |
| 1A85 | N-(1-Adamantylmethyl)-6-[4-[[4-(3-hydroxyphenyl)-2,6-dimethoxyphenyl]methyl]piperazin-1-yl]pyridazine-3-carboxamide |
| 1A86 | N-(1-Adamantylmethyl)-6-[4-[[4-(5-hydroxypyridin-3-yl)-2,6-dimethoxyphenyl]methyl]piperazin-1-yl]pyridazine-3-carboxamide |
| 1A87 | N-(1-Adamantylmethyl)-6-[4-[[3-(3-hydroxyphenyl)-5-(trifluoromethyl)phenyl]methyl]piperazin-1-yl]pyridazine-3-carboxamide |
| 1A88 | N-(1-Adamantylmethyl)-6-[4-[[3-(5-hydroxypyridin-3-yl)-5-(trifluoromethyl)phenyl]methyl]piperazin-1-yl]pyridazine-3-carboxamide |
| 1A89 | N-(1-Adamantylmethyl)-6-[4-[[3-(3-hydroxyphenyl)-5-(trifluoromethoxy)phenyl]methyl]piperazin-1-yl]pyridazine-3-carboxamide |
| 1A90 | N-(1-Adamantylmethyl)-6-[4-[[3-(5-hydroxypyridin-3-yl)-5-(trifluoromethoxy)phenyl]methyl]piperazin-1-yl]pyridazine-3-carboxamide |
| 1A91 | N-(1-Adamantylmethyl)-6-[4-[[4-(3-hydroxyphenyl)naphthalen-1-yl]methyl]piperazin-1-yl]pyridazine-3-carboxamide |
| 1A92 | N-(1-Adamantylmethyl)-6-[4-[[4-(5-hydroxypyridin-3-yl)naphthalen-1-yl]methyl]piperazin-1-yl]pyridazine-3-carboxamide |
| 1A93 | N-(1-Adamantylmethyl)-6-[4-[[4-(3-hydroxyphenyl)thiophen-2-yl]methyl]piperazin-1-yl]pyridazine-3-carboxamide |
| 1A94 | N-(1-Adamantylmethyl)-6-[4-[[4-(5-hydroxypyridin-3-yl)thiophen-2-yl]methyl]piperazin-1-yl]pyridazine-3-carboxamide |

| | |
|---|---|
| 1A95 | N-(1-Adamantylmethyl)-6-[4-[[2-[2-(3-hydroxyphenyl)ethynyl]phenyl]methyl]piperazin-1-yl]pyridazine-3-carboxamide |
| 1A96 | N-(1-Adamantylmethyl)-6-[4-[[2-[2-(5-hydroxypyridin-3-yl)ethynyl]phenyl]methyl]piperazin-1-yl]pyridazine-3-carboxamide |
| 1A97 | N-(1-Adamantylmethyl)-4-[4-[1-[4-(3-hydroxyphenyl)phenyl]ethyl]piperazin-1-yl]benzamide |
| 1A98 | N-(1-Adamantylmethyl)-4-[4-[1-[4-(5-hydroxypyridin-3-yl)phenyl]ethyl]piperazin-1-yl]benzamide |
| 1A99 | N-(1-Adamantylmethyl)-4-[4-[1-[2-fluoro-4-(3-hydroxyphenyl)phenyl]ethyl]piperazin-1-yl]benzamide |
| 1A100 | N-(1-Adamantylmethyl)-4-[4-[1-[2-fluoro-4-(5-hydroxypyridin-3-yl)phenyl]ethyl]piperazin-1-yl]benzamide |

[Table 220]

[Table EIJ06]

| No. of mixture used | mixEI12-02R |
|---|---|
| Mixture No: after cleavage | mixEJ06 |
| No. of compound in Table EJ06 | Compound name |
| 3A01 | N-(1-Adamantylmethyl)-6-[4-[4-(3-hydroxyphenyl)thiophene-2-carbonyl]piperazin-1-yl]-N-methylpyridazine-3-carboxamide |
| 3A02 | N-(1-Adamantylmethyl)-6-[4-[4-(5-hydroxypyridin-3-yl)thiophene-2-carbonyl]piperazin-1-yl]-N-methylpyridazine-3-carboxamide |
| 3A03 | N-(1-Adamantylmethyl)-6-[4-[4-(3-hydroxyphenyl)-3-methylbenzoyl]piperazin-1-yl]-N-methylpyridazine-3-carboxamide |
| 3A04 | N-(1-Adamantylmethyl)-6-[4-[4-(5-hydroxypyridin-3-yl)-3-methylbenzoyl]piperazin-1-yl]-N-methylpyridazine-3-carboxamide |
| 3A05 | N-(1-Adamantylmethyl)-6-[4-[3-fluoro-4-(3-hydroxyphenyl)benzoyl]piperazin-1-yl]-N-methylpyridazine-3-carboxamide |
| 3A06 | N-(1-Adamantylmethyl)-6-[4-[3-fluoro-4-(5-hydroxypyridin-3-yl)benzoyl]piperazin-1-yl]-N-methylpyridazine-3-carboxamide |
| 3A07 | N-(1-Adamantylmethyl)-6-[4-[2-[2-(3-hydroxyphenyl)ethynyl]benzoyl]piperazin-1-yl]-N-methylpyridazine-3-carboxamide |
| 3A08 | N-(1-Adamantylmethyl)-6-[4-[2-[2-(5-hydroxypyridin-3-yl)ethynyl]benzoyl]piperazin-1-yl]-N-methylpyridazine-3-carboxamide |
| 3A09 | N-(1-Adamantylmethyl)-6-[4-[5-[2-(3-hydroxyphenyl)ethynyl]pyridine-3-carbonyl]piperazin-1-yl]-N-methylpyridazine-3-carboxamide |
| 3A10 | N-(1-Adamantylmethyl)-6-[4-[5-[2-(5-hydroxypyridin-3-yl)ethynyl]pyridine-3-carbonyl]piperazin-1-yl]-N-methylpyridazine-3-carboxamide |
| 3A11 | N-(1-Adamantylmethyl)-6-[4-[3-[2-(3-hydroxyphenyl)phenyl]propanoyl]piperazin-1-yl]-N-methylpyridazine-3-carboxamide |
| 3A12 | N-(1-Adamantylmethyl)-6-[4-[3-[2-(5-hydroxypyridin-3-yl)phenyl]propanoyl]piperazin-1-yl]-N-methylpyridazine-3-carboxamide |
| 3A13 | N-(1-Adamantylmethyl)-6-[4-[4-(3-hydroxyphenyl)naphthalene-1-carbonyl]piperazin-1-yl]-N-methylpyridazine-3-carboxamide |
| 3A14 | N-(1-Adamantylmethyl)-6-[4-[4-(5-hydroxypyridin-3-yl)naphthalene-1-carbonyl]piperazin-1-yl]-N-methylpyridazine-3-carboxamide |
| 3A15 | N-(1-Adamantylmethyl)-6-[4-[3-tert-butyl-5-(3-hydroxyphenyl)benzoyl]piperazin-1-yl]-N-methylpyridazine-3-carboxamide |
| 3A16 | N-(1-Adamantylmethyl)-6-[4-[3-tert-butyl-5-(5-hydroxypyridin-3-yl)benzoyl]piperazin-1-yl]-N-methylpyridazine-3-carboxamide |
| 3A17 | N-(1-Adamantylmethyl)-6-[4-[3-(3-hydroxyphenyl)-5-(trifluoromethyl)benzoyl]piperazin-1-yl]-N-methylpyridazine-3-carboxamide |

| | |
|---|---|
| 3A18 | N-(1-Adamantylmethyl)-6-[4-[3-(5-hydroxypyridin-3-yl)-5-(trifluoromethyl)benzoyl]piperazin-1-yl]-N-methylpyridazine-3-carboxamide |
| 3A19 | N-(1-Adamantylmethyl)-6-[4-[3-[2-(3-hydroxyphenyl)ethynyl]-5-(trifluoromethyl)benzoyl]piperazin-1-yl]-N-methylpyridazine-3-carboxamide |
| 3A20 | N-(1-Adamantylmethyl)-6-[4-[3-[2-(5-hydroxypyridin-3-yl)ethynyl]-5-(trifluoromethyl)benzoyl]piperazin-1-yl]-N-methylpyridazine-3-carboxamide |
| 3A21 | N-(1-Adamantylmethyl)-6-[4-[3-[4-(3-hydroxyphenyl)pyrazol-1-yl]-5-(trifluoromethyl)benzoyl]piperazin-1-yl]-N-methylpyridazine-3-carboxamide |
| 3A22 | N-(1-Adamantylmethyl)-6-[4-[3-[4-(5-hydroxypyridin-3-yl)pyrazol-1-yl]-5-(trifluoromethyl)benzoyl]piperazin-1-yl]-N-methylpyridazine-3-carboxamide |
| 3A23 | N-(1-Adamantylmethyl)-4-[4-[4-(3-hydroxyphenyl)thiophene-2-carbonyl]piperazin-1-yl]-N-methylbenzamide |
| 3A24 | N-(1-Adamantylmethyl)-4-[4-[4-(5-hydroxypyridin-3-yl)thiophene-2-carbonyl]piperazin-1-yl]-N-methylbenzamide |
| 3A25 | N-(1-Adamantylmethyl)-4-[4-[4-(3-hydroxyphenyl)-3-methylbenzoyl]piperazin-1-yl]-N-methylbenzamide |
| 3A26 | N-(1-Adamantylmethyl)-4-[4-[4-(5-hydroxypyridin-3-yl)-3-methylbenzoyl]piperazin-1-yl]-N-methylbenzamide |
| 3A27 | N-(1-Adamantylmethyl)-4-[4-[3-fluoro-4-(3-hydroxyphenyl)benzoyl]piperazin-1-yl]-N-methylbenzamide |
| 3A28 | N-(1-Adamantylmethyl)-4-[4-[3-fluoro-4-(5-hydroxypyridin-3-yl)benzoyl]piperazin-1-yl]-N-methylbenzamide |
| 3A29 | N-(1-Adamantylmethyl)-4-[4-[2-[2-(3-hydroxyphenyl)ethynyl]benzoyl]piperazin-1-yl]-N-methylbenzamide |
| 3A30 | N-(1-Adamantylmethyl)-4-[4-[2-[2-(5-hydroxypyridin-3-yl)ethynyl]benzoyl]piperazin-1-yl]-N-methylbenzamide |
| 3A31 | N-(1-Adamantylmethyl)-4-[4-[5-[2-(3-hydroxyphenyl)ethynyl]pyridine-3-carbonyl]piperazin-1-yl]-N-methylbenzamide |
| 3A32 | N-(1-Adamantylmethyl)-4-[4-[5-[2-(5-hydroxypyridin-3-yl)ethynyl]pyridine-3-carbonyl]piperazin-1-yl]-N-methylbenzamide |
| 3A33 | N-(1-Adamantylmethyl)-4-[4-[3-[2-(3-hydroxyphenyl)phenyl]propanoyl]piperazin-1-yl]-N-methylbenzamide |
| 3A34 | N-(1-Adamantylmethyl)-4-[4-[3-[2-(5-hydroxypyridin-3-yl)phenyl]propanoyl]piperazin-1-yl]-N-methylbenzamide |
| 3A35 | N-(1-Adamantylmethyl)-4-[4-[4-(3-hydroxyphenyl)naphthalene-1-carbonyl]piperazin-1-yl]-N-methylbenzamide |
| 3A36 | N-(1-Adamantylmethyl)-4-[4-[4-(5-hydroxypyridin-3-yl)naphthalene-1-carbonyl]piperazin-1-yl]-N-methylbenzamide |
| 3A37 | N-(1-Adamantylmethyl)-4-[4-[3-tert-butyl-5-(3-hydroxyphenyl)benzoyl]piperazin-1-yl]-N-methylbenzamide |
| 3A38 | N-(1-Adamantylmethyl)-4-[4-[3-tert-butyl-5-(5-hydroxypyridin-3-yl)benzoyl]piperazin-1-yl]-N-methylbenzamide |
| 3A39 | N-(1-Adamantylmethyl)-4-[4-[3-(3-hydroxyphenyl)-5-(trifluoromethyl)benzoyl]piperazin-1-yl]-N-methylbenzamide |
| 3A40 | N-(1-Adamantylmethyl)-4-[4-[3-(5-hydroxypyridin-3-yl)-5-(trifluoromethyl)benzoyl]piperazin-1-yl]-N-methylbenzamide |
| 3A41 | N-(1-Adamantylmethyl)-4-[4-[3-[2-(3-hydroxyphenyl)ethynyl]-5-(trifluoromethyl)benzoyl]piperazin-1-yl]-N-methylbenzamide |
| 3A42 | N-(1-Adamantylmethyl)-4-[4-[3-[2-(5-hydroxypyridin-3-yl)ethynyl]-5-(trifluoromethyl)benzoyl]piperazin-1-yl]-N-methylbenzamide |
| 3A43 | N-(1-Adamantylmethyl)-4-[4-[3-[4-(3-hydroxyphenyl)pyrazol-1-yl]-5-(trifluoromethyl)benzoyl]piperazin-1-yl]-N-methylbenzamide |
| 3A44 | N-(1-Adamantylmethyl)-4-[4-[3-[4-(5-hydroxypyridin-3-yl)pyrazol-1-yl]-5-(trifluoromethyl)benzoyl]piperazin-1-yl]-N-methylbenzamide |
| 3A45 | N-(1-Adamantylmethyl)-4-[4-[[2-(3-hydroxyphenyl)phenyl]methyl]piperazin-1-yl]-N-methylbenzamide |

| | |
|---|---|
| 3A46 | N-(1-Adamantylmethyl)-4-[4-[[2-(5-hydroxypyridin-3-yl)phenyl]methyl]piperazin-1-yl]-N-methylbenzamide |
| 3A47 | N-(1-Adamantylmethyl)-4-[4-[[4-(3-hydroxyphenyl)-3-methylphenyl]methyl]piperazin-1-yl]-N-methylbenzamide |
| 3A48 | N-(1-Adamantylmethyl)-4-[4-[[4-(5-hydroxypyridin-3-yl)-3-methylphenyl]methyl]piperazin-1-yl]-N-methylbenzamide |
| 3A49 | N-(1-Adamantylmethyl)-4-[4-[[2-ethyl-4-(3-hydroxyphenyl)phenyl]methyl]piperazin-1-yl]-N-methylbenzamide |
| 3A50 | N-(1-Adamantylmethyl)-4-[4-[[2-ethyl-4-(5-hydroxypyridin-3-yl)phenyl]methyl]piperazin-1-yl]-N-methylbenzamide |
| 3A51 | N-(1-Adamantylmethyl)-4-[4-[[3-fluoro-4-(3-hydroxyphenyl)phenyl]methyl]piperazin-1-yl]-N-methylbenzamide |
| 3A52 | N-(1-Adamantylmethyl)-4-[4-[[3-fluoro-4-(5-hydroxypyridin-3-yl)phenyl]methyl]piperazin-1-yl]-N-methylbenzamide |
| 3A53 | N-(1-Adamantylmethyl)-4-[4-[[4-(3-hydroxyphenyl)-2-methoxyphenyl]methyl]piperazin-1-yl]-N-methylbenzamide |
| 3A54 | N-(1-Adamantylmethyl)-4-[4-[[4-(5-hydroxypyridin-3-yl)-2-methoxyphenyl]methyl]piperazin-1-yl]-N-methylbenzamide |
| 3A55 | N-(1-Adamantylmethyl)-4-[4-[[3-ethoxy-5-(3-hydroxyphenyl)phenyl]methyl]piperazin-1-yl]-N-methylbenzamide |
| 3A56 | N-(1-Adamantylmethyl)-4-[4-[[3-ethoxy-5-(5-hydroxypyridin-3-yl)phenyl]methyl]piperazin-1-yl]-N-methylbenzamide |
| 3A57 | N-(1-Adamantylmethyl)-4-[4-[[3-(3-hydroxyphenyl)-5-propan-2-yloxyphenyl]methyl]piperazin-1-yl]-N-methylbenzamide |
| 3A58 | N-(1-Adamantylmethyl)-4-[4-[[3-(5-hydroxypyridin-3-yl)-5-propan-2-yloxyphenyl]methyl]piperazin-1-yl]-N-methylbenzamide |
| 3A59 | N-(1-Adamantylmethyl)-4-[4-[[4-(3-hydroxyphenyl)-2,6-dimethoxyphenyl]methyl]piperazin-1-yl]-N-methylbenzamide |
| 3A60 | N-(1-Adamantylmethyl)-4-[4-[[4-(5-hydroxypyridin-3-yl)-2,6-dimethoxyphenyl]methyl]piperazin-1-yl]-N-methylbenzamide |
| 3A61 | N-(1-Adamantylmethyl)-4-[4-[[3-(3-hydroxyphenyl)-5-(trifluoromethyl)phenyl]methyl]piperazin-1-yl]-N-methylbenzamide |
| 3A62 | N-(1-Adamantylmethyl)-4-[4-[[3-(5-hydroxypyridin-3-yl)-5-(trifluoromethyl)phenyl]methyl]piperazin-1-yl]-N-methylbenzamide |
| 3A63 | N-(1-Adamantylmethyl)-4-[4-[[3-(3-hydroxyphenyl)-5-(trifluoromethoxy)phenyl]methyl]piperazin-1-yl]-N-methylbenzamide |
| 3A64 | N-(1-Adamantylmethyl)-4-[4-[[3-(5-hydroxypyridin-3-yl)-5-(trifluoromethoxy)phenyl]methyl]piperazin-1-yl]-N-methylbenzamide |
| 3A65 | N-(1-Adamantylmethyl)-4-[4-[[4-(3-hydroxyphenyl)naphthalen-1-yl]methyl]piperazin-1-yl]-N-methylbenzamide |
| 3A66 | N-(1-Adamantylmethyl)-4-[4-[[4-(5-hydroxypyridin-3-yl)naphthalen-1-yl]methyl]piperazin-1-yl]-N-methylbenzamide |
| 3A67 | N-(1-Adamantylmethyl)-4-[4-[[4-(3-hydroxyphenyl)thiophen-2-yl]methyl]piperazin-1-yl]-N-methylbenzamide |
| 3A68 | N-(1-Adamantylmethyl)-4-[4-[[4-(5-hydroxypyridin-3-yl)thiophen-2-yl]methyl]piperazin-1-yl]-N-methylbenzamide |
| 3A69 | N-(1-Adamantylmethyl)-4-[4-[[2-[2-(3-hydroxyphenyl)ethynyl]phenyl]methyl]piperazin-1-yl]-N-methylbenzamide |
| 3A70 | N-(1-Adamantylmethyl)-4-[4-[[2-[2-(5-hydroxypyridin-3-yl)ethynyl]phenyl]methyl]piperazin-1-yl]-N-methylbenzamide |
| 3A71 | N-(1-Adamantylmethyl)-6-[4-[[2-(3-hydroxyphenyl)phenyl]methyl]piperazin-1-yl]-N-methylpyridazine-3-carboxamide |
| 3A72 | N-(1-Adamantylmethyl)-6-[4-[[2-(5-hydroxypyridin-3-yl)phenyl]methyl]piperazin-1-yl]-N-methylpyridazine-3-carboxamide |
| 3A73 | N-(1-Adamantylmethyl)-6-[4-[[4-(3-hydroxyphenyl)-3-methylphenyl]methyl]piperazin-1-yl]-N-methylpyridazine-3-carboxamide |

| | |
|---|---|
| 3A74 | N-(1-Adamantylmethyl)-6-[4-[[4-(5-hydroxypyridin-3-yl)-3-methylphenyl]methyl]piperazin-1-yl]-N-methylpyridazine-3-carboxamide |
| 3A75 | N-(1-Adamantylmethyl)-6-[4-[[2-ethyl-4-(3-hydroxyphenyl)phenyl]methyl]piperazin-1-yl]-N-methylpyridazine-3-carboxamide |
| 3A76 | N-(1-Adamantylmethyl)-6-[4-[[2-ethyl-4-(5-hydroxypyridin-3-yl)phenyl]methyl]piperazin-1-yl]-N-methylpyridazine-3-carboxamide |
| 3A77 | N-(1-Adamantylmethyl)-6-[4-[[3-fluoro-4-(3-hydroxyphenyl)phenyl]methyl]piperazin-1-yl]-N-methylpyridazine-3-carboxamide |
| 3A78 | N-(1-Adamantylmethyl)-6-[4-[[3-fluoro-4-(5-hydroxypyridin-3-yl)phenyl]methyl]piperazin-1-yl]-N-methylpyridazine-3-carboxamide |
| 3A79 | N-(1-Adamantylmethyl)-6-[4-[[4-(3-hydroxyphenyl)-2-methoxyphenyl]methyl]piperazin-1-yl]-N-methylpyridazine-3-carboxamide |
| 3A80 | N-(1-Adamantylmethyl)-6-[4-[[4-(5-hydroxypyridin-3-yl)-2-methoxyphenyl]methyl]piperazin-1-yl]-N-methylpyridazine-3-carboxamide |
| 3A81 | N-(1-Adamantylmethyl)-6-[4-[[3-ethoxy-5-(3-hydroxyphenyl)phenyl]methyl]piperazin-1-yl]-N-methylpyridazine-3-carboxamide |
| 3A82 | N-(1-Adamantylmethyl)-6-[4-[[3-ethoxy-5-(5-hydroxypyridin-3-yl)phenyl]methyl]piperazin-1-yl]-N-methylpyridazine-3-carboxamide |
| 3A83 | N-(1-Adamantylmethyl)-6-[4-[[3-(3-hydroxyphenyl)-5-propan-2-yloxyphenyl]methyl]piperazin-1-yl]-N-methylpyridazine-3-carboxamide |
| 3A84 | N-(1-Adamantylmethyl)-6-[4-[[3-(5-hydroxypyridin-3-yl)-5-propan-2-yloxyphenyl]methyl]piperazin-1-yl]-N-methylpyridazine-3-carboxamide |
| 3A85 | N-(1-Adamantylmethyl)-6-[4-[[4-(3-hydroxyphenyl)-2,6-dimethoxyphenyl]methyl]piperazin-1-yl]-N-methylpyridazine-3-carboxamide |
| 3A86 | N-(1-Adamantylmethyl)-6-[4-[[4-(5-hydroxypyridin-3-yl)-2,6-dimethoxyphenyl]methyl]piperazin-1-yl]-N-methylpyridazine-3-carboxamide |
| 3A87 | N-(1-Adamantylmethyl)-6-[4-[[3-(3-hydroxyphenyl)-5-(trifluoromethyl)phenyl]methyl]piperazin-1-yl]-N-methylpyridazine-3-carboxamide |
| 3A88 | N-(1-Adamantylmethyl)-6-[4-[[3-(5-hydroxypyridin-3-yl)-5-(trifluoromethyl)phenyl]methyl]piperazin-1-yl]-N-methylpyridazine-3-carboxamide |
| 3A89 | N-(1-Adamantylmethyl)-6-[4-[[3-(3-hydroxyphenyl)-5-(trifluoromethoxy)phenyl]methyl]piperazin-1-yl]-N-methylpyridazine-3-carboxamide |
| 3A90 | N-(1-Adamantylmethyl)-6-[4-[[3-(5-hydroxypyridin-3-yl)-5-(trifluoromethoxy)phenyl]methyl]piperazin-1-yl]-N-methylpyridazine-3-carboxamide |
| 3A91 | N-(1-Adamantylmethyl)-6-[4-[[4-(3-hydroxyphenyl)naphthalen-1-yl]methyl]piperazin-1-yl]-N-methylpyridazine-3-carboxamide |
| 3A92 | N-(1-Adamantylmethyl)-6-[4-[[4-(5-hydroxypyridin-3-yl)naphthalen-1-yl]methyl]piperazin-1-yl]-N-methylpyridazine-3-carboxamide |
| 3A93 | N-(1-Adamantylmethyl)-6-[4-[[4-(3-hydroxyphenyl)thiophen-2-yl]methyl]piperazin-1-yl]-N-methylpyridazine-3-carboxamide |
| 3A94 | N-(1-Adamantylmethyl)-6-[4-[[4-(5-hydroxypyridin-3-yl)thiophen-2-yl]methyl]piperazin-1-yl]-N-methylpyridazine-3-carboxamide |
| 3A95 | N-(1-Adamantylmethyl)-6-[4-[[2-[2-(3-hydroxyphenyl)ethynyl]phenyl]methyl]piperazin-1-yl]-N-methylpyridazine-3-carboxamide |
| 3A96 | N-(1-Adamantylmethyl)-6-[4-[[2-[2-(5-hydroxypyridin-3-yl)ethynyl]phenyl]methyl]piperazin-1-yl]-N-methylpyridazine-3-carboxamide |
| 3A97 | N-(1-Adamantylmethyl)-4-[4-[1-[4-(3-hydroxyphenyl)phenyl]ethyl]piperazin-1-yl]-N-methylbenzamide |
| 3A98 | N-(1-Adamantylmethyl)-4-[4-[1-[4-(5-hydroxypyridin-3-yl)phenyl]ethyl]piperazin-1-yl]-N-methylbenzamide |
| 3A99 | N-(1-Adamantylmethyl)-4-[4-[1-[2-fluoro-4-(3-hydroxyphenyl)phenyl]ethyl]piperazin-1-yl]-N-methylbenzamide |
| 3A100 | N-(1-Adamantylmethyl)-4-[4-[1-[2-fluoro-4-(5-hydroxypyridin-3-yl)phenyl]ethyl]piperazin-1-yl]-N-methylbenzamide |

[Table 221]

[Table EIJ07]

| No. of mixture used | mixEI13-02R |
|---|---|
| Mixture No after cleavage | mixEJ07 |
| No. of compound in Table EJ07 | Compound name |
| 2I01 | 6-[4-[4-(3-Hydroxyphenyl)thiophene-2-carbonyl]piperazin-1-yl]-N-methylsulfonylpyridazine-3-carboxamide |
| 2I02 | 6-[4-[4-(5-Hydroxypyridin-3-yl)thiophene-2-carbonyl]piperazin-1-yl]-N-methylsulfonylpyridazine-3-carboxamide |
| 2I03 | 6-[4-[4-(3-Hydroxyphenyl)-3-methylbenzoyl]piperazin-1-yl]-N-methylsulfonylpyridazine-3-carboxamide |
| 2I04 | 6-[4-[4-(5-Hydroxypyridin-3-yl)-3-methylbenzoyl]piperazin-1-yl]-N-methylsulfonylpyridazine-3-carboxamide |
| 2I05 | 6-[4-[3-Fluoro-4-(3-hydroxyphenyl)benzoyl]piperazin-1-yl]-N-methylsulfonylpyridazine-3-carboxamide |
| 2I06 | 6-[4-[3-Fluoro-4-(5-hydroxypyridin-3-yl)benzoyl]piperazin-1-yl]-N-methylsulfonylpyridazine-3-carboxamide |
| 2I07 | 6-[4-[2-[2-(3-Hydroxyphenyl)ethynyl]benzoyl]piperazin-1-yl]-N-methylsulfonylpyridazine-3-carboxamide |
| 2I08 | 6-[4-[2-[2-(5-Hydroxypyridin-3-yl)ethynyl]benzoyl]piperazin-1-yl]-N-methylsulfonylpyridazine-3-carboxamide |
| 2I09 | 6-[4-[5-[2-(3-Hydroxyphenyl)ethynyl]pyridine-3-carbonyl]piperazin-1-yl]-N-methylsulfonylpyridazine-3-carboxamide |
| 2I10 | 6-[4-[5-[2-(5-Hydroxypyridin-3-yl)ethynyl]pyridine-3-carbonyl]piperazin-1-yl]-N-methylsulfonylpyridazine-3-carboxamide |
| 2I11 | 6-[4-[3-[2-(3-Hydroxyphenyl)phenyl]propanoyl]piperazin-1-yl]-N-methylsulfonylpyridazine-3-carboxamide |
| 2I12 | 6-[4-[3-[2-(5-Hydroxypyridin-3-yl)phenyl]propanoyl]piperazin-1-yl]-N-methylsulfonylpyridazine-3-carboxamide |
| 2I13 | 6-[4-[4-(3-Hydroxyphenyl)naphthalene-1-carbonyl]piperazin-1-yl]-N-methylsulfonylpyridazine-3-carboxamide |
| 2I14 | 6-[4-[4-(5-Hydroxypyridin-3-yl)naphthalene-1-carbonyl]piperazin-1-yl]-N-methylsulfonylpyridazine-3-carboxamide |
| 2I15 | 6-[4-[3-tert-Butyl-5-(3-hydroxyphenyl)benzoyl]piperazin-1-yl]-N-methylsulfonylpyridazine-3-carboxamide |
| 2I16 | 6-[4-[3-tert-Butyl-5-(5-hydroxypyridin-3-yl)benzoyl]piperazin-1-yl]-N-methylsulfonylpyridazine-3-carboxamide |
| 2I17 | 6-[4-[3-(3-Hydroxyphenyl)-5-(trifluoromethyl)benzoyl]piperazin-1-yl]-N-methylsulfonylpyridazine-3-carboxamide |
| 2I18 | 6-[4-[3-(5-Hydroxypyridin-3-yl)-5-(trifluoromethyl)benzoyl]piperazin-1-yl]-N-methylsulfonylpyridazine-3-carboxamide |
| 2I19 | 6-[4-[3-[2-(3-Hydroxyphenyl)ethynyl]-5-(trifluoromethyl)benzoyl]piperazin-1-yl]-N-methylsulfonylpyridazine-3-carboxamide |
| 2I20 | 6-[4-[3-[2-(5-Hydroxypyridin-3-yl)ethynyl]-5-(trifluoromethyl)benzoyl]piperazin-1-yl]-N-methylsulfonylpyridazine-3-carboxamide |
| 2I21 | 6-[4-[3-[4-(3-Hydroxyphenyl)pyrazol-1-yl]-5-(trifluoromethyl)benzoyl]piperazin-1-yl]-N-methylsulfonylpyridazine-3-carboxamide |
| 2I22 | 6-[4-[3-[4-(5-Hydroxypyridin-3-yl)pyrazol-1-yl]-5-(trifluoromethyl)benzoyl]piperazin-1-yl]-N-methylsulfonylpyridazine-3-carboxamide |
| 2I23 | 4-[4-[4-(3-Hydroxyphenyl)thiophene-2-carbonyl]piperazin-1-yl]-N-methylsulfonylbenzamide |

| | |
|---|---|
| 2I24 | 4-[4-[4-(5-Hydroxypyridin-3-yl)thiophene-2-carbonyl]piperazin-1-yl]-N-methylsulfonylbenzamide |
| 2I25 | 4-[4-[4-(3-Hydroxyphenyl)-3-methylbenzoyl]piperazin-1-yl]-N-methylsulfonylbenzamide |
| 2I26 | 4-[4-[4-(5-Hydroxypyridin-3-yl)-3-methylbenzoyl]piperazin-1-yl]-N-methylsulfonylbenzamide |
| 2I27 | 4-[4-[3-Fluoro-4-(3-hydroxyphenyl)benzoyl]piperazin-1-yl]-N-methylsulfonylbenzamide |
| 2I28 | 4-[4-[3-Fluoro-4-(5-hydroxypyridin-3-yl)benzoyl]piperazin-1-yl]-N-methylsulfonylbenzamide |
| 2I29 | 4-[4-[2-[2-(3-Hydroxyphenyl)ethynyl]benzoyl]piperazin-1-yl]-N-methylsulfonylbenzamide |
| 2I30 | 4-[4-[2-[2-(5-Hydroxypyridin-3-yl)ethynyl]benzoyl]piperazin-1-yl]-N-methylsulfonylbenzamide |
| 2I31 | 4-[4-[5-[2-(3-Hydroxyphenyl)ethynyl]pyridine-3-carbonyl]piperazin-1-yl]-N-methylsulfonylbenzamide |
| 2I32 | 4-[4-[5-[2-(5-Hydroxypyridin-3-yl)ethynyl]pyridine-3-carbonyl]piperazin-1-yl]-N-methylsulfonylbenzamide |
| 2I33 | 4-[4-[3-[2-(3-Hydroxyphenyl)phenyl]propanoyl]piperazin-1-yl]-N-methylsulfonylbenzamide |
| 2I34 | 4-[4-[3-[2-(5-Hydroxypyridin-3-yl)phenyl]propanoyl]piperazin-1-yl]-N-methylsulfonylbenzamide |
| 2I35 | 4-[4-[4-(3-Hydroxyphenyl)naphthalene-1-carbonyl]piperazin-1-yl]-N-methylsulfonylbenzamide |
| 2I36 | 4-[4-[4-(5-Hydroxypyridin-3-yl)naphthalene-1-carbonyl]piperazin-1-yl]-N-methylsulfonylbenzamide |
| 2I37 | 4-[4-[3-tert-Butyl-5-(3-hydroxyphenyl)benzoyl]piperazin-1-yl]-N-methylsulfonylbenzamide |
| 2I38 | 4-[4-[3-tert-Butyl-5-(5-hydroxypyridin-3-yl)benzoyl]piperazin-1-yl]-N-methylsulfonylbenzamide |
| 2I39 | 4-[4-[3-(3-Hydroxyphenyl)-5-(trifluoromethyl)benzoyl]piperazin-1-yl]-N-methylsulfonylbenzamide |
| 2I40 | 4-[4-[3-(5-Hydroxypyridin-3-yl)-5-(trifluoromethyl)benzoyl]piperazin-1-yl]-N-methylsulfonylbenzamide |
| 2I41 | 4-[4-[3-[2-(3-Hydroxyphenyl)ethynyl]-5-(trifluoromethyl)benzoyl]piperazin-1-yl]-N-methylsulfonylbenzamide |
| 2I42 | 4-[4-[3-[2-(5-Hydroxypyridin-3-yl)ethynyl]-5-(trifluoromethyl)benzoyl]piperazin-1-yl]-N-methylsulfonylbenzamide |
| 2I43 | 4-[4-[3-[4-(3-Hydroxyphenyl)pyrazol-1-yl]-5-(trifluoromethyl)benzoyl]piperazin-1-yl]-N-methylsulfonylbenzamide |
| 2I44 | 4-[4-[3-[4-(5-Hydroxypyridin-3-yl)pyrazol-1-yl]-5-(trifluoromethyl)benzoyl]piperazin-1-yl]-N-methylsulfonylbenzamide |
| 2I45 | 4-[4-[[2-(3-Hydroxyphenyl)phenyl]methyl]piperazin-1-yl]-N-methylsulfonylbenzamide |
| 2I46 | 4-[4-[[2-(5-Hydroxypyridin-3-yl)phenyl]methyl]piperazin-1-yl]-N-methylsulfonylbenzamide |
| 2I47 | 4-[4-[[4-(3-Hydroxyphenyl)-3-methylphenyl]methyl]piperazin-1-yl]-N-methylsulfonylbenzamide |
| 2I48 | 4-[4-[[4-(5-Hydroxypyridin-3-yl)-3-methylphenyl]methyl]piperazin-1-yl]-N-methylsulfonylbenzamide |
| 2I49 | 4-[4-[[2-Ethyl-4-(3-hydroxyphenyl)phenyl]methyl]piperazin-1-yl]-N-methylsulfonylbenzamide |
| 2I50 | 4-[4-[[2-Ethyl-4-(5-hydroxypyridin-3-yl)phenyl]methyl]piperazin-1-yl]-N-methylsulfonylbenzamide |
| 2I51 | 4-[4-[[3-Fluoro-4-(3-hydroxyphenyl)phenyl]methyl]piperazin-1-yl]-N-methylsulfonylbenzamide |

| | |
|---|---|
| 2I52 | 4-[4-[[3-Fluoro-4-(5-hydroxypyridin-3-yl)phenyl]methyl]piperazin-1-yl]-N-methylsulfonylbenzamide |
| 2I53 | 4-[4-[[4-(3-Hydroxyphenyl)-2-methoxyphenyl]methyl]piperazin-1-yl]-N-methylsulfonylbenzamide |
| 2I54 | 4-[4-[[4-(5-Hydroxypyridin-3-yl)-2-methoxyphenyl]methyl]piperazin-1-yl]-N-methylsulfonylbenzamide |
| 2I55 | 4-[4-[[3-Ethoxy-5-(3-hydroxyphenyl)phenyl]methyl]piperazin-1-yl]-N-methylsulfonylbenzamide |
| 2I56 | 4-[4-[[3-Ethoxy-5-(5-hydroxypyridin-3-yl)phenyl]methyl]piperazin-1-yl]-N-methylsulfonylbenzamide |
| 2I57 | 4-[4-[[3-(3-Hydroxyphenyl)-5-propan-2-yloxyphenyl]methyl]piperazin-1-yl]-N-methylsulfonylbenzamide |
| 2I58 | 4-[4-[[3-(5-Hydroxypyridin-3-yl)-5-propan-2-yloxyphenyl]methyl]piperazin-1-yl]-N-methylsulfonylbenzamide |
| 2I59 | 4-[4-[[4-(3-Hydroxyphenyl)-2,6-dimethoxyphenyl]methyl]piperazin-1-yl]-N-methylsulfonylbenzamide |
| 2I60 | 4-[4-[[4-(5-Hydroxypyridin-3-yl)-2,6-dimethoxyphenyl]methyl]piperazin-1-yl]-N-methylsulfonylbenzamide |
| 2I61 | 4-[4-[[3-(3-Hydroxyphenyl)-5-(trifluoromethyl)phenyl]methyl]piperazin-1-yl]-N-methylsulfonylbenzamide |
| 2I62 | 4-[4-[[3-(5-Hydroxypyridin-3-yl)-5-(trifluoromethyl)phenyl]methyl]piperazin-1-yl]-N-methylsulfonylbenzamide |
| 2I63 | 4-[4-[[3-(3-Hydroxyphenyl)-5-(trifluoromethoxy)phenyl]methyl]piperazin-1-yl]-N-methylsulfonylbenzamide |
| 2I64 | 4-[4-[[3-(5-Hydroxypyridin-3-yl)-5-(trifluoromethoxy)phenyl]methyl]piperazin-1-yl]-N-methylsulfonylbenzamide |
| 2I65 | 4-[4-[[4-(3-Hydroxyphenyl)naphthalen-1-yl]methyl]piperazin-1-yl]-N-methylsulfonylbenzamide |
| 2I66 | 4-[4-[[4-(5-Hydroxypyridin-3-yl)naphthalen-1-yl]methyl]piperazin-1-yl]-N-methylsulfonylbenzamide |
| 2I67 | 4-[4-[[4-(3-Hydroxyphenyl)thiophen-2-yl]methyl]piperazin-1-yl]-N-methylsulfonylbenzamide |
| 2I68 | 4-[4-[[4-(5-Hydroxypyridin-3-yl)thiophen-2-yl]methyl]piperazin-1-yl]-N-methylsulfonylbenzamide |
| 2I69 | 4-[4-[[2-[2-(3-Hydroxyphenyl)ethynyl]phenyl]methyl]piperazin-1-yl]-N-methylsulfonylbenzamide |
| 2I70 | 4-[4-[[2-[2-(5-Hydroxypyridin-3-yl)ethynyl]phenyl]methyl]piperazin-1-yl]-N-methylsulfonylbenzamide |
| 2I71 | 6-[4-[[2-(3-Hydroxyphenyl)phenyl]methyl]piperazin-1-yl]-N-methylsulfonylpyridazine-3-carboxamide |
| 2I72 | 6-[4-[[2-(5-Hydroxypyridin-3-yl)phenyl]methyl]piperazin-1-yl]-N-methylsulfonylpyridazine-3-carboxamide |
| 2I73 | 6-[4-[[4-(3-Hydroxyphenyl)-3-methylphenyl]methyl]piperazin-1-yl]-N-methylsulfonylpyridazine-3-carboxamide |
| 2I74 | 6-[4-[[4-(5-Hydroxypyridin-3-yl)-3-methylphenyl]methyl]piperazin-1-yl]-N-methylsulfonylpyridazine-3-carboxamide |
| 2I75 | 6-[4-[[2-Ethyl-4-(3-hydroxyphenyl)phenyl]methyl]piperazin-1-yl]-N-methylsulfonylpyridazine-3-carboxamide |
| 2I76 | 6-[4-[[2-Ethyl-4-(5-hydroxypyridin-3-yl)phenyl]methyl]piperazin-1-yl]-N-methylsulfonylpyridazine-3-carboxamide |
| 2I77 | 6-[4-[[3-Fluoro-4-(3-hydroxyphenyl)phenyl]methyl]piperazin-1-yl]-N-methylsulfonylpyridazine-3-carboxamide |
| 2I78 | 6-[4-[[3-Fluoro-4-(5-hydroxypyridin-3-yl)phenyl]methyl]piperazin-1-yl]-N-methylsulfonylpyridazine-3-carboxamide |
| 2I79 | 6-[4-[[4-(3-Hydroxyphenyl)-2-methoxyphenyl]methyl]piperazin-1-yl]-N-methylsulfonylpyridazine-3-carboxamide |

| | |
|---|---|
| 2I80 | 6-[4-[[4-(5-Hydroxypyridin-3-yl)-2-methoxyphenyl]methyl]piperazin-1-yl]-N-methylsulfonylpyridazine-3-carboxamide |
| 2I81 | 6-[4-[[3-Ethoxy-5-(3-hydroxyphenyl)phenyl]methyl]piperazin-1-yl]-N-methylsulfonylpyridazine-3-carboxamide |
| 2I82 | 6-[4-[[3-Ethoxy-5-(5-hydroxypyridin-3-yl)phenyl]methyl]piperazin-1-yl]-N-methylsulfonylpyridazine-3-carboxamide |
| 2I83 | 6-[4-[[3-(3-Hydroxyphenyl)-5-propan-2-yloxyphenyl]methyl]piperazin-1-yl]-N-methylsulfonylpyridazine-3-carboxamide |
| 2I84 | 6-[4-[[3-(5-Hydroxypyridin-3-yl)-5-propan-2-yloxyphenyl]methyl]piperazin-1-yl]-N-methylsulfonylpyridazine-3-carboxamide |
| 2I85 | 6-[4-[[4-(3-Hydroxyphenyl)-2,6-dimethoxyphenyl]methyl]piperazin-1-yl]-N-methylsulfonylpyridazine-3-carboxamide |
| 2I86 | 6-[4-[[4-(5-Hydroxypyridin-3-yl)-2,6-dimethoxyphenyl]methyl]piperazin-1-yl]-N-methylsulfonylpyridazine-3-carboxamide |
| 2I87 | 6-[4-[[3-(3-Hydroxyphenyl)-5-(trifluoromethyl)phenyl]methyl]piperazin-1-yl]-N-methylsulfonylpyridazine-3-carboxamide |
| 2I88 | 6-[4-[[3-(5-Hydroxypyridin-3-yl)-5-(trifluoromethyl)phenyl]methyl]piperazin-1-yl]-N-methylsulfonylpyridazine-3-carboxamide |
| 2I89 | 6-[4-[[3-(3-Hydroxyphenyl)-5-(trifluoromethoxy)phenyl]methyl]piperazin-1-yl]-N-methylsulfonylpyridazine-3-carboxamide |
| 2I90 | 6-[4-[[3-(5-Hydroxypyridin-3-yl)-5-(trifluoromethoxy)phenyl]methyl]piperazin-1-yl]-N-methylsulfonylpyridazine-3-carboxamide |
| 2I91 | 6-[4-[[4-(3-Hydroxyphenyl)naphthalen-1-yl]methyl]piperazin-1-yl]-N-methylsulfonylpyridazine-3-carboxamide |
| 2I92 | 6-[4-[[4-(5-Hydroxypyridin-3-yl)naphthalen-1-yl]methyl]piperazin-1-yl]-N-methylsulfonylpyridazine-3-carboxamide |
| 2I93 | 6-[4-[[4-(3-Hydroxyphenyl)thiophen-2-yl]methyl]piperazin-1-yl]-N-methylsulfonylpyridazine-3-carboxamide |
| 2I94 | 6-[4-[[4-(5-Hydroxypyridin-3-yl)thiophen-2-yl]methyl]piperazin-1-yl]-N-methylsulfonylpyridazine-3-carboxamide |
| 2I95 | 6-[4-[[2-[2-(3-Hydroxyphenyl)ethynyl]phenyl]methyl]piperazin-1-yl]-N-methylsulfonylpyridazine-3-carboxamide |
| 2I96 | 6-[4-[[2-[2-(5-Hydroxypyridin-3-yl)ethynyl]phenyl]methyl]piperazin-1-yl]-N-methylsulfonylpyridazine-3-carboxamide |
| 2I97 | 4-[4-[1-[4-(3-Hydroxyphenyl)phenyl]ethyl]piperazin-1-yl]-N-methylsulfonylbenzamide |
| 2I98 | 4-[4-[1-[4-(5-Hydroxypyridin-3-yl)phenyl]ethyl]piperazin-1-yl]-N-methylsulfonylbenzamide |
| 2I99 | 4-[4-[1-[2-Fluoro-4-(3-hydroxyphenyl)phenyl]ethyl]piperazin-1-yl]-N-methylsulfonylbenzamide |
| 2I100 | 4-[4-[1-[2-Fluoro-4-(5-hydroxypyridin-3-yl)phenyl]ethyl]piperazin-1-yl]-N-methylsulfonylbenzamide |

[Table 222]

[Table EIJ08]

| No. of mixture used | mixEI14-02R |
|---|---|
| Mixture No after cleavage | mixEJ08 |
| No. of compound in Table EJ08 | Compound name |
| 2L01 | N-tert-Butylsulfonyl-6-[4-[4-(3-hydroxyphenyl)thiophene-2-carbonyl]piperazin-1-yl]pyridazine-3-carboxamide |
| 2L02 | N-tert-Butylsulfonyl-6-[4-[4-(5-hydroxypyridin-3-yl)thiophene-2-carbonyl]piperazin-1-yl]pyridazine-3-carboxamide |

| | |
|---|---|
| 2L03 | N-tert-Butylsulfonyl-6-[4-[4-(3-hydroxyphenyl)-3-methylbenzoyl]piperazin-1-yl]pyridazine-3-carboxamide |
| 2L04 | N-tert-Butylsulfonyl-6-[4-[4-(5-hydroxypyridin-3-yl)-3-methylbenzoyl]piperazin-1-yl]pyridazine-3-carboxamide |
| 2L05 | N-tert-Butylsulfonyl-6-[4-[3-fluoro-4-(3-hydroxyphenyl)benzoyl]piperazin-1-yl]pyridazine-3-carboxamide |
| 2L06 | N-tert-Butylsulfonyl-6-[4-[3-fluoro-4-(5-hydroxypyridin-3-yl)benzoyl]piperazin-1-yl]pyridazine-3-carboxamide |
| 2L07 | N-tert-Butylsulfonyl-6-[4-[2-[2-(3-hydroxyphenyl)ethynyl]benzoyl]piperazin-1-yl]pyridazine-3-carboxamide |
| 2L08 | N-tert-Butylsulfonyl-6-[4-[2-[2-(5-hydroxypyridin-3-yl)ethynyl]benzoyl]piperazin-1-yl]pyridazine-3-carboxamide |
| 2L09 | N-tert-Butylsulfonyl-6-[4-[5-[2-(3-hydroxyphenyl)ethynyl]pyridine-3-carbonyl]piperazin-1-yl]pyridazine-3-carboxamide |
| 2L10 | N-tert-Butylsulfonyl-6-[4-[5-[2-(5-hydroxypyridin-3-yl)ethynyl]pyridine-3-carbonyl]piperazin-1-yl]pyridazine-3-carboxamide |
| 2L11 | N-tert-Butylsulfonyl-6-[4-[3-[2-(3-hydroxyphenyl)phenyl]propanoyl]piperazin-1-yl]pyridazine-3-carboxamide |
| 2L12 | N-tert-Butylsulfonyl-6-[4-[3-[2-(5-hydroxypyridin-3-yl)phenyl]propanoyl]piperazin-1-yl]pyridazine-3-carboxamide |
| 2L13 | N-tert-Butylsulfonyl-6-[4-[4-(3-hydroxyphenyl)naphthalene-1-carbonyl]piperazin-1-yl]pyridazine-3-carboxamide |
| 2L14 | N-tert-Butylsulfonyl-6-[4-[4-(5-hydroxypyridin-3-yl)naphthalene-1-carbonyl]piperazin-1-yl]pyridazine-3-carboxamide |
| 2L15 | 6-[4-[3-tert-Butyl-5-(3-hydroxyphenyl)benzoyl]piperazin-1-yl]-N-tert-butylsulfonylpyridazine-3-carboxamide |
| 2L16 | 6-[4-[3-tert-Butyl-5-(5-hydroxypyridin-3-yl)benzoyl]piperazin-1-yl]-N-tert-butylsulfonylpyridazine-3-carboxamide |
| 2L17 | N-tert-Butylsulfonyl-6-[4-[3-(3-hydroxyphenyl)-5-(trifluoromethyl)benzoyl]piperazin-1-yl]pyridazine-3-carboxamide |
| 2L18 | N-tert-Butylsulfonyl-6-[4-[3-(5-hydroxypyridin-3-yl)-5-(trifluoromethyl)benzoyl]piperazin-1-yl]pyridazine-3-carboxamide |
| 2L19 | N-tert-Butylsulfonyl-6-[4-[3-[2-(3-hydroxyphenyl)ethynyl]-5-(trifluoromethyl)benzoyl]piperazin-1-yl]pyridazine-3-carboxamide |
| 2L20 | N-tert-Butylsulfonyl-6-[4-[3-[2-(5-hydroxypyridin-3-yl)ethynyl]-5-(trifluoromethyl)benzoyl]piperazin-1-yl]pyridazine-3-carboxamide |
| 2L21 | N-tert-Butylsulfonyl-6-[4-[3-[4-(3-hydroxyphenyl)pyrazol-1-yl]-5-(trifluoromethyl)benzoyl]piperazin-1-yl]pyridazine-3-carboxamide |
| 2L22 | N-tert-Butylsulfonyl-6-[4-[3-[4-(5-hydroxypyridin-3-yl)pyrazol-1-yl]-5-(trifluoromethyl)benzoyl]piperazin-1-yl]pyridazine-3-carboxamide |
| 2L23 | N-tert-Butylsulfonyl-4-[4-[4-(3-hydroxyphenyl)thiophene-2-carbonyl]piperazin-1-yl]benzamide |
| 2L24 | N-tert-Butylsulfonyl-4-[4-[4-(5-hydroxypyridin-3-yl)thiophene-2-carbonyl]piperazin-1-yl]benzamide |
| 2L25 | N-tert-Butylsulfonyl-4-[4-[4-(3-hydroxyphenyl)-3-methylbenzoyl]piperazin-1-yl]benzamide |
| 2L26 | N-tert-Butylsulfonyl-4-[4-[4-(5-hydroxypyridin-3-yl)-3-methylbenzoyl]piperazin-1-yl]benzamide |
| 2L27 | N-tert-Butylsulfonyl-4-[4-[3-fluoro-4-(3-hydroxyphenyl)benzoyl]piperazin-1-yl]benzamide |
| 2L28 | N-tert-Butylsulfonyl-4-[4-[3-fluoro-4-(5-hydroxypyridin-3-yl)benzoyl]piperazin-1-yl]benzamide |
| 2L29 | N-tert-Butylsulfonyl-4-[4-[2-[2-(3-hydroxyphenyl)ethynyl]benzoyl]piperazin-1-yl]benzamide |
| 2L30 | N-tert-Butylsulfonyl-4-[4-[2-[2-(5-hydroxypyridin-3-yl)ethynyl]benzoyl]piperazin-1-yl]benzamide |

| | |
|---|---|
| 2L31 | N-tert-Butylsulfonyl-4-[4-[5-[2-(3-hydroxyphenyl)ethynyl]pyridine-3-carbonyl]piperazin-1-yl]benzamide |
| 2L32 | N-tert-Butylsulfonyl-4-[4-[5-[2-(5-hydroxypyridin-3-yl)ethynyl]pyridine-3-carbonyl]piperazin-1-yl]benzamide |
| 2L33 | N-tert-Butylsulfonyl-4-[4-[3-[2-(3-hydroxyphenyl)phenyl]propanoyl]piperazin-1-yl]benzamide |
| 2L34 | N-tert-Butylsulfonyl-4-[4-[3-[2-(5-hydroxypyridin-3-yl)phenyl]propanoyl]piperazin-1-yl]benzamide |
| 2L35 | N-tert-Butylsulfonyl-4-[4-[4-(3-hydroxyphenyl)naphthalene-1-carbonyl]piperazin-1-yl]benzamide |
| 2L36 | N-tert-Butylsulfonyl-4-[4-[4-(5-hydroxypyridin-3-yl)naphthalene-1-carbonyl]piperazin-1-yl]benzamide |
| 2L37 | 4-[4-[3-tert-Butyl-5-(3-hydroxyphenyl)benzoyl]piperazin-1-yl]-N-tert-butylsulfonylbenzamide |
| 2L38 | 4-[4-[3-tert-Butyl-5-(5-hydroxypyridin-3-yl)benzoyl]piperazin-1-yl]-N-tert-butylsulfonylbenzamide |
| 2L39 | N-tert-Butylsulfonyl-4-[4-[3-(3-hydroxyphenyl)-5-(trifluoromethyl)benzoyl]piperazin-1-yl]benzamide |
| 2L40 | N-tert-Butylsulfonyl-4-[4-[3-(5-hydroxypyridin-3-yl)-5-(trifluoromethyl)benzoyl]piperazin-1-yl]benzamide |
| 2L41 | N-tert-Butylsulfonyl-4-[4-[3-[2-(3-hydroxyphenyl)ethynyl]-5-(trifluoromethyl)benzoyl]piperazin-1-yl]benzamide |
| 2L42 | N-tert-Butylsulfonyl-4-[4-[3-[2-(5-hydroxypyridin-3-yl)ethynyl]-5-(trifluoromethyl)benzoyl]piperazin-1-yl]benzamide |
| 2L43 | N-tert-Butylsulfonyl-4-[4-[3-[4-(3-hydroxyphenyl)pyrazol-1-yl]-5-(trifluoromethyl)benzoyl]piperazin-1-yl]benzamide |
| 2L44 | N-tert-Butylsulfonyl-4-[4-[3-[4-(5-hydroxypyridin-3-yl)pyrazol-1-yl]-5-(trifluoromethyl)benzoyl]piperazin-1-yl]benzamide |
| 2L45 | N-tert-Butylsulfonyl-4-[4-[[2-(3-hydroxyphenyl)phenyl]methyl]piperazin-1-yl]benzamide |
| 2L46 | N-tert-Butylsulfonyl-4-[4-[[2-(5-hydroxypyridin-3-yl)phenyl]methyl]piperazin-1-yl]benzamide |
| 2L47 | N-tert-Butylsulfonyl-4-[4-[[4-(3-hydroxyphenyl)-3-methylphenyl]methyl]piperazin-1-yl]benzamide |
| 2L48 | N-tert-Butylsulfonyl-4-[4-[[4-(5-hydroxypyridin-3-yl)-3-methylphenyl]methyl]piperazin-1-yl]benzamide |
| 2L49 | N-tert-Butylsulfonyl-4-[4-[[2-ethyl-4-(3-hydroxyphenyl)phenyl]methyl]piperazin-1-yl]benzamide |
| 2L50 | N-tert-Butylsulfonyl-4-[4-[[2-ethyl-4-(5-hydroxypyridin-3-yl)phenyl]methyl]piperazin-1-yl]benzamide |
| 2L51 | N-tert-Butylsulfonyl-4-[4-[[3-fluoro-4-(3-hydroxyphenyl)phenyl]methyl]piperazin-1-yl]benzamide |
| 2L52 | N-tert-Butylsulfonyl-4-[4-[[3-fluoro-4-(5-hydroxypyridin-3-yl)phenyl]methyl]piperazin-1-yl]benzamide |
| 2L53 | N-tert-Butylsulfonyl-4-[4-[[4-(3-hydroxyphenyl)-2-methoxyphenyl]methyl]piperazin-1-yl]benzamide |
| 2L54 | N-tert-Butylsulfonyl-4-[4-[[4-(5-hydroxypyridin-3-yl)-2-methoxyphenyl]methyl]piperazin-1-yl]benzamide |
| 2L55 | N-tert-Butylsulfonyl-4-[4-[[3-ethoxy-5-(3-hydroxyphenyl)phenyl]methyl]piperazin-1-yl]benzamide |
| 2L56 | N-tert-Butylsulfonyl-4-[4-[[3-ethoxy-5-(5-hydroxypyridin-3-yl)phenyl]methyl]piperazin-1-yl]benzamide |
| 2L57 | N-tert-Butylsulfonyl-4-[4-[[3-(3-hydroxyphenyl)-5-propan-2-yloxyphenyl]methyl]piperazin-1-yl]benzamide |
| 2L58 | N-tert-Butylsulfonyl-4-[4-[[3-(5-hydroxypyridin-3-yl)-5-propan-2-yloxyphenyl]methyl]piperazin-1-yl]benzamide |

| | |
|---|---|
| 2L59 | N-tert-Butylsulfonyl-4-[4-[[4-(3-hydroxyphenyl)-2,6-dimethoxyphenyl]methyl]piperazin-1-yl]benzamide |
| 2L60 | N-tert-Butylsulfonyl-4-[4-[[4-(5-hydroxypyridin-3-yl)-2,6-dimethoxyphenyl]methyl]piperazin-1-yl]benzamide |
| 2L61 | N-tert-Butylsulfonyl-4-[4-[[3-(3-hydroxyphenyl)-5-(trifluoromethyl)phenyl]methyl]piperazin-1-yl]benzamide |
| 2L62 | N-tert-Butylsulfonyl-4-[4-[[3-(5-hydroxypyridin-3-yl)-5-(trifluoromethyl)phenyl]methyl]piperazin-1-yl]benzamide |
| 2L63 | N-tert-Butylsulfonyl-4-[4-[[3-(3-hydroxyphenyl)-5-(trifluoromethoxy)phenyl]methyl]piperazin-1-yl]benzamide |
| 2L64 | N-tert-Butylsulfonyl-4-[4-[[3-(5-hydroxypyridin-3-yl)-5-(trifluoromethoxy)phenyl]methyl]piperazin-1-yl]benzamide |
| 2L65 | N-tert-Butylsulfonyl-4-[4-[[4-(3-hydroxyphenyl)naphthalen-1-yl]methyl]piperazin-1-yl]benzamide |
| 2L66 | N-tert-Butylsulfonyl-4-[4-[[4-(5-hydroxypyridin-3-yl)naphthalen-1-yl]methyl]piperazin-1-yl]benzamide |
| 2L67 | N-tert-Butylsulfonyl-4-[4-[[4-(3-hydroxyphenyl)thiophen-2-yl]methyl]piperazin-1-yl]benzamide |
| 2L68 | N-tert-Butylsulfonyl-4-[4-[[4-(5-hydroxypyridin-3-yl)thiophen-2-yl]methyl]piperazin-1-yl]benzamide |
| 2L69 | N-tert-Butylsulfonyl-4-[4-[[2-[2-(3-hydroxyphenyl)ethynyl]phenyl]methyl]piperazin-1-yl]benzamide |
| 2L70 | N-tert-Butylsulfonyl-4-[4-[[2-[2-(5-hydroxypyridin-3-yl)ethynyl]phenyl]methyl]piperazin-1-yl]benzamide |
| 2L71 | N-tert-Butylsulfonyl-6-[4-[[2-(3-hydroxyphenyl)phenyl]methyl]piperazin-1-yl]pyridazine-3-carboxamide |
| 2L72 | N-tert-Butylsulfonyl-6-[4-[[2-(5-hydroxypyridin-3-yl)phenyl]methyl]piperazin-1-yl]pyridazine-3-carboxamide |
| 2L73 | N-tert-Butylsulfonyl-6-[4-[[4-(3-hydroxyphenyl)-3-methylphenyl]methyl]piperazin-1-yl]pyridazine-3-carboxamide |
| 2L74 | N-tert-Butylsulfonyl-6-[4-[[4-(5-hydroxypyridin-3-yl)-3-methylphenyl]methyl]piperazin-1-yl]pyridazine-3-carboxamide |
| 2L75 | N-tert-Butylsulfonyl-6-[4-[[2-ethyl-4-(3-hydroxyphenyl)phenyl]methyl]piperazin-1-yl]pyridazine-3-carboxamide |
| 2L76 | N-tert-Butylsulfonyl-6-[4-[[2-ethyl-4-(5-hydroxypyridin-3-yl)phenyl]methyl]piperazin-1-yl]pyridazine-3-carboxamide |
| 2L77 | N-tert-Butylsulfonyl-6-[4-[[3-fluoro-4-(3-hydroxyphenyl)phenyl]methyl]piperazin-1-yl]pyridazine-3-carboxamide |
| 2L78 | N-tert-Butylsulfonyl-6-[4-[[3-fluoro-4-(5-hydroxypyridin-3-yl)phenyl]methyl]piperazin-1-yl]pyridazine-3-carboxamide |
| 2L79 | N-tert-Butylsulfonyl-6-[4-[[4-(3-hydroxyphenyl)-2-methoxyphenyl]methyl]piperazin-1-yl]pyridazine-3-carboxamide |
| 2L80 | N-tert-Butylsulfonyl-6-[4-[[4-(5-hydroxypyridin-3-yl)-2-methoxyphenyl]methyl]piperazin-1-yl]pyridazine-3-carboxamide |
| 2L81 | N-tert-Butylsulfonyl-6-[4-[[3-ethoxy-5-(3-hydroxyphenyl)phenyl]methyl]piperazin-1-yl]pyridazine-3-carboxamide |
| 2L82 | N-tert-Butylsulfonyl-6-[4-[[3-ethoxy-5-(5-hydroxypyridin-3-yl)phenyl]methyl]piperazin-1-yl]pyridazine-3-carboxamide |
| 2L83 | N-tert-Butylsulfonyl-6-[4-[[3-(3-hydroxyphenyl)-5-propan-2-yloxyphenyl]methyl]piperazin-1-yl]pyridazine-3-carboxamide |
| 2L84 | N-tert-Butylsulfonyl-6-[4-[[3-(5-hydroxypyridin-3-yl)-5-propan-2-yloxyphenyl]methyl]piperazin-1-yl]pyridazine-3-carboxamide |
| 2L85 | N-tert-Butylsulfonyl-6-[4-[[4-(3-hydroxyphenyl)-2,6-dimethoxyphenyl]methyl]piperazin-1-yl]pyridazine-3-carboxamide |
| 2L86 | N-tert-Butylsulfonyl-6-[4-[[4-(5-hydroxypyridin-3-yl)-2,6-dimethoxyphenyl]methyl]piperazin-1-yl]pyridazine-3-carboxamide |

| | |
|---|---|
| 2L87 | N-tert-Butylsulfonyl-6-[4-[[3-(3-hydroxyphenyl)-5-(trifluoromethyl)phenyl]methyl]piperazin-1-yl]pyridazine-3-carboxamide |
| 2L88 | N-tert-Butylsulfonyl-6-[4-[[3-(5-hydroxypyridin-3-yl)-5-(trifluoromethyl)phenyl]methyl]piperazin-1-yl]pyridazine-3-carboxamide |
| 2L89 | N-tert-Butylsulfonyl-6-[4-[[3-(3-hydroxyphenyl)-5-(trifluoromethoxy)phenyl]methyl]piperazin-1-yl]pyridazine-3-carboxamide |
| 2L90 | N-tert-Butylsulfonyl-6-[4-[[3-(5-hydroxypyridin-3-yl)-5-(trifluoromethoxy)phenyl]methyl]piperazin-1-yl]pyridazine-3-carboxamide |
| 2L91 | N-tert-Butylsulfonyl-6-[4-[[4-(3-hydroxyphenyl)naphthalen-1-yl]methyl]piperazin-1-yl]pyridazine-3-carboxamide |
| 2L92 | N-tert-Butylsulfonyl-6-[4-[[4-(5-hydroxypyridin-3-yl)naphthalen-1-yl]methyl]piperazin-1-yl]pyridazine-3-carboxamide |
| 2L93 | N-tert-Butylsulfonyl-6-[4-[[4-(3-hydroxyphenyl)thiophen-2-yl]methyl]piperazin-1-yl]pyridazine-3-carboxamide |
| 2L94 | N-tert-Butylsulfonyl-6-[4-[[4-(5-hydroxypyridin-3-yl)thiophen-2-yl]methyl]piperazin-1-yl]pyridazine-3-carboxamide |
| 2L95 | N-tert-Butylsulfonyl-6-[4-[[2-[2-(3-hydroxyphenyl)ethynyl]phenyl]methyl]piperazin-1-yl]pyridazine-3-carboxamide |
| 2L96 | N-tert-Butylsulfonyl-6-[4-[[2-[2-(5-hydroxypyridin-3-yl)ethynyl]phenyl]methyl]piperazin-1-yl]pyridazine-3-carboxamide |
| 2L97 | N-tert-Butylsulfonyl-4-[4-[1-[4-(3-hydroxyphenyl)phenyl]ethyl]piperazin-1-yl]benzamide |
| 2L98 | N-tert-Butylsulfonyl-4-[4-[1-[4-(5-hydroxypyridin-3-yl)phenyl]ethyl]piperazin-1-yl]benzamide |
| 2L99 | N-tert-Butylsulfonyl-4-[4-[1-[2-fluoro-4-(3-hydroxyphenyl)phenyl]ethyl]piperazin-1-yl]benzamide |
| 2L100 | N-tert-Butylsulfonyl-4-[4-[1-[2-fluoro-4-(5-hydroxypyridin-3-yl)phenyl]ethyl]piperazin-1-yl]benzamide |

[Table 223]

[Table EIJ09]

| No. of mixture used | mixEI15-02R |
|---|---|
| Mixture No after cleavage | mixEJ09 |
| No. of compound in Table EJ09 | Compound name |
| 2K01 | 6-[4-[4-(3-Hydroxyphenyl)thiophene-2-carbonyl]piperazin-1-yl]-N-(3,3,3-trifluoropropylsulfonyl)pyridazine-3-carboxamide |
| 2K02 | 6-[4-[4-(5-Hydroxypyridin-3-yl)thiophene-2-carbonyl]piperazin-1-yl]-N-(3,3,3-trifluoropropylsulfonyl)pyridazine-3-carboxamide |
| 2K03 | 6-[4-[4-(3-Hydroxyphenyl)-3-methylbenzoyl]piperazin-1-yl]-N-(3,3,3-trifluoropropylsulfonyl)pyridazine-3-carboxamide |
| 2K04 | 6-[4-[4-(5-Hydroxypyridin-3-yl)-3-methylbenzoyl]piperazin-1-yl]-N-(3,3,3-trifluoropropylsulfonyl)pyridazine-3-carboxamide |
| 2K05 | 6-[4-[3-Fluoro-4-(3-hydroxyphenyl)benzoyl]piperazin-1-yl]-N-(3,3,3-trifluoropropylsulfonyl)pyridazine-3-carboxamide |
| 2K06 | 6-[4-[3-Fluoro-4-(5-hydroxypyridin-3-yl)benzoyl]piperazin-1-yl]-N-(3,3,3-trifluoropropylsulfonyl)pyridazine-3-carboxamide |
| 2K07 | 6-[4-[2-[2-(3-Hydroxyphenyl)ethynyl]benzoyl]piperazin-1-yl]-N-(3,3,3-trifluoropropylsulfonyl)pyridazine-3-carboxamide |
| 2K08 | 6-[4-[2-[2-(5-Hydroxypyridin-3-yl)ethynyl]benzoyl]piperazin-1-yl]-N-(3,3,3-trifluoropropylsulfonyl)pyridazine-3-carboxamide |
| 2K09 | 6-[4-[5-[2-(3-Hydroxyphenyl)ethynyl]pyridine-3-carbonyl]piperazin-1-yl]-N-(3,3,3-trifluoropropylsulfonyl)pyridazine-3-carboxamide |

| | |
|---|---|
| 2K10 | 6-[4-[5-[2-(5-Hydroxypyridin-3-yl)ethynyl]pyridine-3-carbonyl]piperazin-1-yl]-N-(3,3,3-trifluoropropylsulfonyl)pyridazine-3-carboxamide |
| 2K11 | 6-[4-[3-[2-(3-Hydroxyphenyl)phenyl]propanoyl]piperazin-1-yl]-N-(3,3,3-trifluoropropylsulfonyl)pyridazine-3-carboxamide |
| 2K12 | 6-[4-[3-[2-(5-Hydroxypyridin-3-yl)phenyl]propanoyl]piperazin-1-yl]-N-(3,3,3-trifluoropropylsulfonyl)pyridazine-3-carboxamide |
| 2K13 | 6-[4-[4-(3-Hydroxyphenyl)naphthalene-1-carbonyl]piperazin-1-yl]-N-(3,3,3-trifluoropropylsulfonyl)pyridazine-3-carboxamide |
| 2K14 | 6-[4-[4-(5-Hydroxypyridin-3-yl)naphthalene-1-carbonyl]piperazin-1-yl]-N-(3,3,3-trifluoropropylsulfonyl)pyridazine-3-carboxamide |
| 2K15 | 6-[4-[3-tert-Butyl-5-(3-hydroxyphenyl)benzoyl]piperazin-1-yl]-N-(3,3,3-trifluoropropylsulfonyl)pyridazine-3-carboxamide |
| 2K16 | 6-[4-[3-tert-Butyl-5-(5-hydroxypyridin-3-yl)benzoyl]piperazin-1-yl]-N-(3,3,3-trifluoropropylsulfonyl)pyridazine-3-carboxamide |
| 2K17 | 6-[4-[3-(3-Hydroxyphenyl)-5-(trifluoromethyl)benzoyl]piperazin-1-yl]-N-(3,3,3-trifluoropropylsulfonyl)pyridazine-3-carboxamide |
| 2K18 | 6-[4-[3-(5-Hydroxypyridin-3-yl)-5-(trifluoromethyl)benzoyl]piperazin-1-yl]-N-(3,3,3-trifluoropropylsulfonyl)pyridazine-3-carboxamide |
| 2K19 | 6-[4-[3-[2-(3-Hydroxyphenyl)ethynyl]-5-(trifluoromethyl)benzoyl]piperazin-1-yl]-N-(3,3,3-trifluoropropylsulfonyl)pyridazine-3-carboxamide |
| 2K20 | 6-[4-[3-[2-(5-Hydroxypyridin-3-yl)ethynyl]-5-(trifluoromethyl)benzoyl]piperazin-1-yl]-N-(3,3,3-trifluoropropylsulfonyl)pyridazine-3-carboxamide |
| 2K21 | 6-[4-[3-[4-(3-Hydroxyphenyl)pyrazol-1-yl]-5-(trifluoromethyl)benzoyl]piperazin-1-yl]-N-(3,3,3-trifluoropropylsulfonyl)pyridazine-3-carboxamide |
| 2K22 | 6-[4-[3-[4-(5-Hydroxypyridin-3-yl)pyrazol-1-yl]-5-(trifluoromethyl)benzoyl]piperazin-1-yl]-N-(3,3,3-trifluoropropylsulfonyl)pyridazine-3-carboxamide |
| 2K23 | 4-[4-[4-(3-Hydroxyphenyl)thiophene-2-carbonyl]piperazin-1-yl]-N-(3,3,3-trifluoropropylsulfonyl)benzamide |
| 2K24 | 4-[4-[4-(5-Hydroxypyridin-3-yl)thiophene-2-carbonyl]piperazin-1-yl]-N-(3,3,3-trifluoropropylsulfonyl)benzamide |
| 2K25 | 4-[4-[4-(3-Hydroxyphenyl)-3-methylbenzoyl]piperazin-1-yl]-N-(3,3,3-trifluoropropylsulfonyl)benzamide |
| 2K26 | 4-[4-[4-(5-Hydroxypyridin-3-yl)-3-methylbenzoyl]piperazin-1-yl]-N-(3,3,3-trifluoropropylsulfonyl)benzamide |
| 2K27 | 4-[4-[3-Fluoro-4-(3-hydroxyphenyl)benzoyl]piperazin-1-yl]-N-(3,3,3-trifluoropropylsulfonyl)benzamide |
| 2K28 | 4-[4-[3-Fluoro-4-(5-hydroxypyridin-3-yl)benzoyl]piperazin-1-yl]-N-(3,3,3-trifluoropropylsulfonyl)benzamide |
| 2K29 | 4-[4-[2-[2-(3-Hydroxyphenyl)ethynyl]benzoyl]piperazin-1-yl]-N-(3,3,3-trifluoropropylsulfonyl)benzamide |
| 2K30 | 4-[4-[2-[2-(5-Hydroxypyridin-3-yl)ethynyl]benzoyl]piperazin-1-yl]-N-(3,3,3-trifluoropropylsulfonyl)benzamide |
| 2K31 | 4-[4-[5-[2-(3-Hydroxyphenyl)ethynyl]pyridine-3-carbonyl]piperazin-1-yl]-N-(3,3,3-trifluoropropylsulfonyl)benzamide |
| 2K32 | 4-[4-[5-[2-(5-Hydroxypyridin-3-yl)ethynyl]pyridine-3-carbonyl]piperazin-1-yl]-N-(3,3,3-trifluoropropylsulfonyl)benzamide |
| 2K33 | 4-[4-[3-[2-(3-Hydroxyphenyl)phenyl]propanoyl]piperazin-1-yl]-N-(3,3,3-trifluoropropylsulfonyl)benzamide |
| 2K34 | 4-[4-[3-[2-(5-Hydroxypyridin-3-yl)phenyl]propanoyl]piperazin-1-yl]-N-(3,3,3-trifluoropropylsulfonyl)benzamide |
| 2K35 | 4-[4-[4-(3-Hydroxyphenyl)naphthalene-1-carbonyl]piperazin-1-yl]-N-(3,3,3-trifluoropropylsulfonyl)benzamide |
| 2K36 | 4-[4-[4-(5-Hydroxypyridin-3-yl)naphthalene-1-carbonyl]piperazin-1-yl]-N-(3,3,3-trifluoropropylsulfonyl)benzamide |
| 2K37 | 4-[4-[3-tert-Butyl-5-(3-hydroxyphenyl)benzoyl]piperazin-1-yl]-N-(3,3,3-trifluoropropylsulfonyl)benzamide |

| | |
|---|---|
| 2K38 | 4-[4-[3-tert-Butyl-5-(5-hydroxypyridin-3-yl)benzoyl]piperazin-1-yl]-N-(3,3,3-trifluoropropylsulfonyl)benzamide |
| 2K39 | 4-[4-[3-(3-Hydroxyphenyl)-5-(trifluoromethyl)benzoyl]piperazin-1-yl]-N-(3,3,3-trifluoropropylsulfonyl)benzamide |
| 2K40 | 4-[4-[3-(5-Hydroxypyridin-3-yl)-5-(trifluoromethyl)benzoyl]piperazin-1-yl]-N-(3,3,3-trifluoropropylsulfonyl)benzamide |
| 2K41 | 4-[4-[3-[2-(3-Hydroxyphenyl)ethynyl]-5-(trifluoromethyl)benzoyl]piperazin-1-yl]-N-(3,3,3-trifluoropropylsulfonyl)benzamide |
| 2K42 | 4-[4-[3-[2-(5-Hydroxypyridin-3-yl)ethynyl]-5-(trifluoromethyl)benzoyl]piperazin-1-yl]-N-(3,3,3-trifluoropropylsulfonyl)benzamide |
| 2K43 | 4-[4-[3-[4-(3-Hydroxyphenyl)pyrazol-1-yl]-5-(trifluoromethyl)benzoyl]piperazin-1-yl]-N-(3,3,3-trifluoropropylsulfonyl)benzamide |
| 2K44 | 4-[4-[3-[4-(5-Hydroxypyridin-3-yl)pyrazol-1-yl]-5-(trifluoromethyl)benzoyl]piperazin-1-yl]-N-(3,3,3-trifluoropropylsulfonyl)benzamide |
| 2K45 | 4-[4-[[2-(3-Hydroxyphenyl)phenyl]methyl]piperazin-1-yl]-N-(3,3,3-trifluoropropylsulfonyl)benzamide |
| 2K46 | 4-[4-[[2-(5-Hydroxypyridin-3-yl)phenyl]methyl]piperazin-1-yl]-N-(3,3,3-trifluoropropylsulfonyl)benzamide |
| 2K47 | 4-[4-[[4-(3-Hydroxyphenyl)-3-methylphenyl]methyl]piperazin-1-yl]-N-(3,3,3-trifluoropropylsulfonyl)benzamide |
| 2K48 | 4-[4-[[4-(5-Hydroxypyridin-3-yl)-3-methylphenyl]methyl]piperazin-1-yl]-N-(3,3,3-trifluoropropylsulfonyl)benzamide |
| 2K49 | 4-[4-[[2-Ethyl-4-(3-hydroxyphenyl)phenyl]methyl]piperazin-1-yl]-N-(3,3,3-trifluoropropylsulfonyl)benzamide |
| 2K50 | 4-[4-[[2-Ethyl-4-(5-hydroxypyridin-3-yl)phenyl]methyl]piperazin-1-yl]-N-(3,3,3-trifluoropropylsulfonyl)benzamide |
| 2K51 | 4-[4-[[3-Fluoro-4-(3-hydroxyphenyl)phenyl]methyl]piperazin-1-yl]-N-(3,3,3-trifluoropropylsulfonyl)benzamide |
| 2K52 | 4-[4-[[3-Fluoro-4-(5-hydroxypyridin-3-yl)phenyl]methyl]piperazin-1-yl]-N-(3,3,3-trifluoropropylsulfonyl)benzamide |
| 2K53 | 4-[4-[[4-(3-Hydroxyphenyl)-2-methoxyphenyl]methyl]piperazin-1-yl]-N-(3,3,3-trifluoropropylsulfonyl)benzamide |
| 2K54 | 4-[4-[[4-(5-Hydroxypyridin-3-yl)-2-methoxyphenyl]methyl]piperazin-1-yl]-N-(3,3,3-trifluoropropylsulfonyl)benzamide |
| 2K55 | 4-[4-[[3-Ethoxy-5-(3-hydroxyphenyl)phenyl]methyl]piperazin-1-yl]-N-(3,3,3-trifluoropropylsulfonyl)benzamide |
| 2K56 | 4-[4-[[3-Ethoxy-5-(5-hydroxypyridin-3-yl)phenyl]methyl]piperazin-1-yl]-N-(3,3,3-trifluoropropylsulfonyl)benzamide |
| 2K57 | 4-[4-[[3-(3-Hydroxyphenyl)-5-propan-2-yloxyphenyl]methyl]piperazin-1-yl]-N-(3,3,3-trifluoropropylsulfonyl)benzamide |
| 2K58 | 4-[4-[[3-(5-Hydroxypyridin-3-yl)-5-propan-2-yloxyphenyl]methyl]piperazin-1-yl]-N-(3,3,3-trifluoropropylsulfonyl)benzamide |
| 2K59 | 4-[4-[[4-(3-Hydroxyphenyl)-2,6-dimethoxyphenyl]methyl]piperazin-1-yl]-N-(3,3,3-trifluoropropylsulfonyl)benzamide |
| 2K60 | 4-[4-[[4-(5-Hydroxypyridin-3-yl)-2,6-dimethoxyphenyl]methyl]piperazin-1-yl]-N-(3,3,3-trifluoropropylsulfonyl)benzamide |
| 2K61 | 4-[4-[[3-(3-Hydroxyphenyl)-5-(trifluoromethyl)phenyl]methyl]piperazin-1-yl]-N-(3,3,3-trifluoropropylsulfonyl)benzamide |
| 2K62 | 4-[4-[[3-(5-Hydroxypyridin-3-yl)-5-(trifluoromethyl)phenyl]methyl]piperazin-1-yl]-N-(3,3,3-trifluoropropylsulfonyl)benzamide |
| 2K63 | 4-[4-[[3-(3-Hydroxyphenyl)-5-(trifluoromethoxy)phenyl]methyl]piperazin-1-yl]-N-(3,3,3-trifluoropropylsulfonyl)benzamide |
| 2K64 | 4-[4-[[3-(5-Hydroxypyridin-3-yl)-5-(trifluoromethoxy)phenyl]methyl]piperazin-1-yl]-N-(3,3,3-trifluoropropylsulfonyl)benzamide |
| 2K65 | 4-[4-[[4-(3-Hydroxyphenyl)naphthalen-1-yl]methyl]piperazin-1-yl]-N-(3,3,3-trifluoropropylsulfonyl)benzamide |

| | |
|---|---|
| 2K66 | 4-[4-[[4-(5-Hydroxypyridin-3-yl)naphthalen-1-yl]methyl]piperazin-1-yl]-N-(3,3,3-trifluoropropylsulfonyl)benzamide |
| 2K67 | 4-[4-[[4-(3-Hydroxyphenyl)thiophen-2-yl]methyl]piperazin-1-yl]-N-(3,3,3-trifluoropropylsulfonyl)benzamide |
| 2K68 | 4-[4-[[4-(5-Hydroxypyridin-3-yl)thiophen-2-yl]methyl]piperazin-1-yl]-N-(3,3,3-trifluoropropylsulfonyl)benzamide |
| 2K69 | 4-[4-[[2-[2-(3-Hydroxyphenyl)ethynyl]phenyl]methyl]piperazin-1-yl]-N-(3,3,3-trifluoropropylsulfonyl)benzamide |
| 2K70 | 4-[4-[[2-[2-(5-Hydroxypyridin-3-yl)ethynyl]phenyl]methyl]piperazin-1-yl]-N-(3,3,3-trifluoropropylsulfonyl)benzamide |
| 2K71 | 6-[4-[[2-(3-Hydroxyphenyl)phenyl]methyl]piperazin-1-yl]-N-(3,3,3-trifluoropropylsulfonyl)pyridazine-3-carboxamide |
| 2K72 | 6-[4-[[2-(5-Hydroxypyridin-3-yl)phenyl]methyl]piperazin-1-yl]-N-(3,3,3-trifluoropropylsulfonyl)pyridazine-3-carboxamide |
| 2K73 | 6-[4-[[4-(3-Hydroxyphenyl)-3-methylphenyl]methyl]piperazin-1-yl]-N-(3,3,3-trifluoropropylsulfonyl)pyridazine-3-carboxamide |
| 2K74 | 6-[4-[[4-(5-Hydroxypyridin-3-yl)-3-methylphenyl]methyl]piperazin-1-yl]-N-(3,3,3-trifluoropropylsulfonyl)pyridazine-3-carboxamide |
| 2K75 | 6-[4-[[2-Ethyl-4-(3-hydroxyphenyl)phenyl]methyl]piperazin-1-yl]-N-(3,3,3-trifluoropropylsulfonyl)pyridazine-3-carboxamide |
| 2K76 | 6-[4-[[2-Ethyl-4-(5-hydroxypyridin-3-yl)phenyl]methyl]piperazin-1-yl]-N-(3,3,3-trifluoropropylsulfonyl)pyridazine-3-carboxamide |
| 2K77 | 6-[4-[[3-Fluoro-4-(3-hydroxyphenyl)phenyl]methyl]piperazin-1-yl]-N-(3,3,3-trifluoropropylsulfonyl)pyridazine-3-carboxamide |
| 2K78 | 6-[4-[[3-Fluoro-4-(5-hydroxypyridin-3-yl)phenyl]methyl]piperazin-1-yl]-N-(3,3,3-trifluoropropylsulfonyl)pyridazine-3-carboxamide |
| 2K79 | 6-[4-[[4-(3-Hydroxyphenyl)-2-methoxyphenyl]methyl]piperazin-1-yl]-N-(3,3,3-trifluoropropylsulfonyl)pyridazine-3-carboxamide |
| 2K80 | 6-[4-[[4-(5-Hydroxypyridin-3-yl)-2-methoxyphenyl]methyl]piperazin-1-yl]-N-(3,3,3-trifluoropropylsulfonyl)pyridazine-3-carboxamide |
| 2K81 | 6-[4-[[3-Ethoxy-5-(3-hydroxyphenyl)phenyl]methyl]piperazin-1-yl]-N-(3,3,3-trifluoropropylsulfonyl)pyridazine-3-carboxamide |
| 2K82 | 6-[4-[[3-Ethoxy-5-(5-hydroxypyridin-3-yl)phenyl]methyl]piperazin-1-yl]-N-(3,3,3-trifluoropropylsulfonyl)pyridazine-3-carboxamide |
| 2K83 | 6-[4-[[3-(3-Hydroxyphenyl)-5-propan-2-yloxyphenyl]methyl]piperazin-1-yl]-N-(3,3,3-trifluoropropylsulfonyl)pyridazine-3-carboxamide |
| 2K84 | 6-[4-[[3-(5-Hydroxypyridin-3-yl)-5-propan-2-yloxyphenyl]methyl]piperazin-1-yl]-N-(3,3,3-trifluoropropylsulfonyl)pyridazine-3-carboxamide |
| 2K85 | 6-[4-[[4-(3-Hydroxyphenyl)-2,6-dimethoxyphenyl]methyl]piperazin-1-yl]-N-(3,3,3-trifluoropropylsulfonyl)pyridazine-3-carboxamide |
| 2K86 | 6-[4-[[4-(5-Hydroxypyridin-3-yl)-2,6-dimethoxyphenyl]methyl]piperazin-1-yl]-N-(3,3,3-trifluoropropylsulfonyl)pyridazine-3-carboxamide |
| 2K87 | 6-[4-[[3-(3-Hydroxyphenyl)-5-(trifluoromethyl)phenyl]methyl]piperazin-1-yl]-N-(3,3,3-trifluoropropylsulfonyl)pyridazine-3-carboxamide |
| 2K88 | 6-[4-[[3-(5-Hydroxypyridin-3-yl)-5-(trifluoromethyl)phenyl]methyl]piperazin-1-yl]-N-(3,3,3-trifluoropropylsulfonyl)pyridazine-3-carboxamide |
| 2K89 | 6-[4-[[3-(3-Hydroxyphenyl)-5-(trifluoromethoxy)phenyl]methyl]piperazin-1-yl]-N-(3,3,3-trifluoropropylsulfonyl)pyridazine-3-carboxamide |
| 2K90 | 6-[4-[[3-(5-Hydroxypyridin-3-yl)-5-(trifluoromethoxy)phenyl]methyl]piperazin-1-yl]-N-(3,3,3-trifluoropropylsulfonyl)pyridazine-3-carboxamide |
| 2K91 | 6-[4-[[4-(3-Hydroxyphenyl)naphthalen-1-yl]methyl]piperazin-1-yl]-N-(3,3,3-trifluoropropylsulfonyl)pyridazine-3-carboxamide |
| 2K92 | 6-[4-[[4-(5-Hydroxypyridin-3-yl)naphthalen-1-yl]methyl]piperazin-1-yl]-N-(3,3,3-trifluoropropylsulfonyl)pyridazine-3-carboxamide |
| 2K93 | 6-[4-[[4-(3-Hydroxyphenyl)thiophen-2-yl]methyl]piperazin-1-yl]-N-(3,3,3-trifluoropropylsulfonyl)pyridazine-3-carboxamide |

| | |
|---|---|
| 2K94 | 6-[4-[[4-(5-Hydroxypyridin-3-yl)thiophen-2-yl]methyl]piperazin-1-yl]-N-(3,3,3-trifluoropropylsulfonyl)pyridazine-3-carboxamide |
| 2K95 | 6-[4-[[2-[2-(3-Hydroxyphenyl)ethynyl]phenyl]methyl]piperazin-1-yl]-N-(3,3,3-trifluoropropylsulfonyl)pyridazine-3-carboxamide |
| 2K96 | 6-[4-[[2-[2-(5-Hydroxypyridin-3-yl)ethynyl]phenyl]methyl]piperazin-1-yl]-N-(3,3,3-trifluoropropylsulfonyl)pyridazine-3-carboxamide |
| 2K97 | 4-[4-[1-[4-(3-Hydroxyphenyl)phenyl]ethyl]piperazin-1-yl]-N-(3,3,3-trifluoropropylsulfonyl)benzamide |
| 2K98 | 4-[4-[1-[4-(5-Hydroxypyridin-3-yl)phenyl]ethyl]piperazin-1-yl]-N-(3,3,3-trifluoropropylsulfonyl)benzamide |
| 2K99 | 4-[4-[1-[2-Fluoro-4-(3-hydroxyphenyl)phenyl]ethyl]piperazin-1-yl]-N-(3,3,3-trifluoropropylsulfonyl)benzamide |
| 2K100 | 4-[4-[1-[2-Fluoro-4-(5-hydroxypyridin-3-yl)phenyl]ethyl]piperazin-1-yl]-N-(3,3,3-trifluoropropylsulfonyl)benzamide |

[Table 224]

[Table EIJ10]

| No. of mixture used | mixEI16-02R |
|---|---|
| Mixture No after cleavage | mixEJ10 |
| No. of compound in Table EJ10 | Compound name |
| 2A01 | N-(1-Adamantylmethylsulfonyl)-6-[4-[4-(3-hydroxyphenyl)thiophene-2-carbonyl]piperazin-1-yl]pyridazine-3-carboxamide |
| 2A02 | N-(1-Adamantylmethylsulfonyl)-6-[4-[4-(5-hydroxypyridin-3-yl)thiophene-2-carbonyl]piperazin-1-yl]pyridazine-3-carboxamide |
| 2A03 | N-(1-Adamantylmethylsulfonyl)-6-[4-[4-(3-hydroxyphenyl)-3-methylbenzoyl]piperazin-1-yl]pyridazine-3-carboxamide |
| 2A04 | N-(1-Adamantylmethylsulfonyl)-6-[4-[4-(5-hydroxypyridin-3-yl)-3-methylbenzoyl]piperazin-1-yl]pyridazine-3-carboxamide |
| 2A05 | N-(1-Adamantylmethylsulfonyl)-6-[4-[3-fluoro-4-(3-hydroxyphenyl)benzoyl]piperazin-1-yl]pyridazine-3-carboxamide |
| 2A06 | N-(1-Adamantylmethylsulfonyl)-6-[4-[3-fluoro-4-(5-hydroxypyridin-3-yl)benzoyl]piperazin-1-yl]pyridazine-3-carboxamide |
| 2A07 | N-(1-Adamantylmethylsulfonyl)-6-[4-[2-[2-(3-hydroxyphenyl)ethynyl]benzoyl]piperazin-1-yl]pyridazine-3-carboxamide |
| 2A08 | N-(1-Adamantylmethylsulfonyl)-6-[4-[2-[2-(5-hydroxypyridin-3-yl)ethynyl]benzoyl]piperazin-1-yl]pyridazine-3-carboxamide |
| 2A09 | N-(1-Adamantylmethylsulfonyl)-6-[4-[5-[2-(3-hydroxyphenyl)ethynyl]pyridine-3-carbonyl]piperazin-1-yl]pyridazine-3-carboxamide |
| 2A10 | N-(1-Adamantylmethylsulfonyl)-6-[4-[5-[2-(5-hydroxypyridin-3-yl)ethynyl]pyridine-3-carbonyl]piperazin-1-yl]pyridazine-3-carboxamide |
| 2A11 | N-(1-Adamantylmethylsulfonyl)-6-[4-[3-[2-(3-hydroxyphenyl)phenyl]propanoyl]piperazin-1-yl]pyridazine-3-carboxamide |
| 2A12 | N-(1-Adamantylmethylsulfonyl)-6-[4-[3-[2-(5-hydroxypyridin-3-yl)phenyl]propanoyl]piperazin-1-yl]pyridazine-3-carboxamide |
| 2A13 | N-(1-Adamantylmethylsulfonyl)-6-[4-[4-(3-hydroxyphenyl)naphthalene-1-carbonyl]piperazin-1-yl]pyridazine-3-carboxamide |
| 2A14 | N-(1-Adamantylmethylsulfonyl)-6-[4-[4-(5-hydroxypyridin-3-yl)naphthalene-1-carbonyl]piperazin-1-yl]pyridazine-3-carboxamide |
| 2A15 | N-(1-Adamantylmethylsulfonyl)-6-[4-[3-tert-butyl-5-(3-hydroxyphenyl)benzoyl]piperazin-1-yl]pyridazine-3-carboxamide |
| 2A16 | N-(1-Adamantylmethylsulfonyl)-6-[4-[3-tert-butyl-5-(5-hydroxypyridin-3-yl)benzoyl]piperazin-1-yl]pyridazine-3-carboxamide |

| | |
|---|---|
| 2A17 | N-(1-Adamantylmethylsulfonyl)-6-[4-[3-(3-hydroxyphenyl)-5-(trifluoromethyl)benzoyl]piperazin-1-yl]pyridazine-3-carboxamide |
| 2A18 | N-(1-Adamantylmethylsulfonyl)-6-[4-[3-(5-hydroxypyridin-3-yl)-5-(trifluoromethyl)benzoyl]piperazin-1-yl]pyridazine-3-carboxamide |
| 2A19 | N-(1-Adamantylmethylsulfonyl)-6-[4-[3-[2-(3-hydroxyphenyl)ethynyl]-5-(trifluoromethyl)benzoyl]piperazin-1-yl]pyridazine-3-carboxamide |
| 2A20 | N-(1-Adamantylmethylsulfonyl)-6-[4-[3-[2-(5-hydroxypyridin-3-yl)ethynyl]-5-(trifluoromethyl)benzoyl]piperazin-1-yl]pyridazine-3-carboxamide |
| 2A21 | N-(1-Adamantylmethylsulfonyl)-6-[4-[3-[4-(3-hydroxyphenyl)pyrazol-1-yl]-5-(trifluoromethyl)benzoyl]piperazin-1-yl]pyridazine-3-carboxamide |
| 2A22 | N-(1-Adamantylmethylsulfonyl)-6-[4-[3-[4-(5-hydroxypyridin-3-yl)pyrazol-1-yl]-5-(trifluoromethyl)benzoyl]piperazin-1-yl]pyridazine-3-carboxamide |
| 2A23 | N-(1-Adamantylmethylsulfonyl)-4-[4-[4-(3-hydroxyphenyl)thiophene-2-carbonyl]piperazin-1-yl]benzamide |
| 2A24 | N-(1-Adamantylmethylsulfonyl)-4-[4-[4-(5-hydroxypyridin-3-yl)thiophene-2-carbonyl]piperazin-1-yl]benzamide |
| 2A25 | N-(1-Adamantylmethylsulfonyl)-4-[4-[4-(3-hydroxyphenyl)-3-methylbenzoyl]piperazin-1-yl]benzamide |
| 2A26 | N-(1-Adamantylmethylsulfonyl)-4-[4-[4-(5-hydroxypyridin-3-yl)-3-methylbenzoyl]piperazin-1-yl]benzamide |
| 2A27 | N-(1-Adamantylmethylsulfonyl)-4-[4-[3-fluoro-4-(3-hydroxyphenyl)benzoyl]piperazin-1-yl]benzamide |
| 2A28 | N-(1-Adamantylmethylsulfonyl)-4-[4-[3-fluoro-4-(5-hydroxypyridin-3-yl)benzoyl]piperazin-1-yl]benzamide |
| 2A29 | N-(1-Adamantylmethylsulfonyl)-4-[4-[2-[2-(3-hydroxyphenyl)ethynyl]benzoyl]piperazin-1-yl]benzamide |
| 2A30 | N-(1-Adamantylmethylsulfonyl)-4-[4-[2-[2-(5-hydroxypyridin-3-yl)ethynyl]benzoyl]piperazin-1-yl]benzamide |
| 2A31 | N-(1-Adamantylmethylsulfonyl)-4-[4-[5-[2-(3-hydroxyphenyl)ethynyl]pyridine-3-carbonyl]piperazin-1-yl]benzamide |
| 2A32 | N-(1-Adamantylmethylsulfonyl)-4-[4-[5-[2-(5-hydroxypyridin-3-yl)ethynyl]pyridine-3-carbonyl]piperazin-1-yl]benzamide |
| 2A33 | N-(1-Adamantylmethylsulfonyl)-4-[4-[3-[2-(3-hydroxyphenyl)phenyl]propanoyl]piperazin-1-yl]benzamide |
| 2A34 | N-(1-Adamantylmethylsulfonyl)-4-[4-[3-[2-(5-hydroxypyridin-3-yl)phenyl]propanoyl]piperazin-1-yl]benzamide |
| 2A35 | N-(1-Adamantylmethylsulfonyl)-4-[4-[4-(3-hydroxyphenyl)naphthalene-1-carbonyl]piperazin-1-yl]benzamide |
| 2A36 | N-(1-Adamantylmethylsulfonyl)-4-[4-[4-(5-hydroxypyridin-3-yl)naphthalene-1-carbonyl]piperazin-1-yl]benzamide |
| 2A37 | N-(1-Adamantylmethylsulfonyl)-4-[4-[3-tert-butyl-5-(3-hydroxyphenyl)benzoyl]piperazin-1-yl]benzamide |
| 2A38 | N-(1-Adamantylmethylsulfonyl)-4-[4-[3-tert-butyl-5-(5-hydroxypyridin-3-yl)benzoyl]piperazin-1-yl]benzamide |
| 2A39 | N-(1-Adamantylmethylsulfonyl)-4-[4-[3-(3-hydroxyphenyl)-5-(trifluoromethyl)benzoyl]piperazin-1-yl]benzamide |
| 2A40 | N-(1-Adamantylmethylsulfonyl)-4-[4-[3-(5-hydroxypyridin-3-yl)-5-(trifluoromethyl)benzoyl]piperazin-1-yl]benzamide |
| 2A41 | N-(1-Adamantylmethylsulfonyl)-4-[4-[3-[2-(3-hydroxyphenyl)ethynyl]-5-(trifluoromethyl)benzoyl]piperazin-1-yl]benzamide |
| 2A42 | N-(1-Adamantylmethylsulfonyl)-4-[4-[3-[2-(5-hydroxypyridin-3-yl)ethynyl]-5-(trifluoromethyl)benzoyl]piperazin-1-yl]benzamide |
| 2A43 | N-(1-Adamantylmethylsulfonyl)-4-[4-[3-[4-(3-hydroxyphenyl)pyrazol-1-yl]-5-(trifluoromethyl)benzoyl]piperazin-1-yl]benzamide |
| 2A44 | N-(1-Adamantylmethylsulfonyl)-4-[4-[3-[4-(5-hydroxypyridin-3-yl)pyrazol-1-yl]-5-(trifluoromethyl)benzoyl]piperazin-1-yl]benzamide |

| | |
|---|---|
| 2A45 | N-(1-Adamantylmethylsulfonyl)-4-[4-[[2-(3-hydroxyphenyl)phenyl]methyl]piperazin-1-yl]benzamide |
| 2A46 | N-(1-Adamantylmethylsulfonyl)-4-[4-[[2-(5-hydroxypyridin-3-yl)phenyl]methyl]piperazin-1-yl]benzamide |
| 2A47 | N-(1-Adamantylmethylsulfonyl)-4-[4-[[4-(3-hydroxyphenyl)-3-methylphenyl]methyl]piperazin-1-yl]benzamide |
| 2A48 | N-(1-Adamantylmethylsulfonyl)-4-[4-[[4-(5-hydroxypyridin-3-yl)-3-methylphenyl]methyl]piperazin-1-yl]benzamide |
| 2A49 | N-(1-Adamantylmethylsulfonyl)-4-[4-[[2-ethyl-4-(3-hydroxyphenyl)phenyl]methyl]piperazin-1-yl]benzamide |
| 2A50 | N-(1-Adamantylmethylsulfonyl)-4-[4-[[2-ethyl-4-(5-hydroxypyridin-3-yl)phenyl]methyl]piperazin-1-yl]benzamide |
| 2A51 | N-(1-Adamantylmethylsulfonyl)-4-[4-[[3-fluoro-4-(3-hydroxyphenyl)phenyl]methyl]piperazin-1-yl]benzamide |
| 2A52 | N-(1-Adamantylmethylsulfonyl)-4-[4-[[3-fluoro-4-(5-hydroxypyridin-3-yl)phenyl]methyl]piperazin-1-yl]benzamide |
| 2A53 | N-(1-Adamantylmethylsulfonyl)-4-[4-[[4-(3-hydroxyphenyl)-2-methoxyphenyl]methyl]piperazin-1-yl]benzamide |
| 2A54 | N-(1-Adamantylmethylsulfonyl)-4-[4-[[4-(5-hydroxypyridin-3-yl)-2-methoxyphenyl]methyl]piperazin-1-yl]benzamide |
| 2A55 | N-(1-Adamantylmethylsulfonyl)-4-[4-[[3-ethoxy-5-(3-hydroxyphenyl)phenyl]methyl]piperazin-1-yl]benzamide |
| 2A56 | N-(1-Adamantylmethylsulfonyl)-4-[4-[[3-ethoxy-5-(5-hydroxypyridin-3-yl)phenyl]methyl]piperazin-1-yl]benzamide |
| 2A57 | N-(1-Adamantylmethylsulfonyl)-4-[4-[[3-(3-hydroxyphenyl)-5-propan-2-yloxyphenyl]methyl]piperazin-1-yl]benzamide |
| 2A58 | N-(1-Adamantylmethylsulfonyl)-4-[4-[[3-(5-hydroxypyridin-3-yl)-5-propan-2-yloxyphenyl]methyl]piperazin-1-yl]benzamide |
| 2A59 | N-(1-Adamantylmethylsulfonyl)-4-[4-[[4-(3-hydroxyphenyl)-2,6-dimethoxyphenyl]methyl]piperazin-1-yl]benzamide |
| 2A60 | N-(1-Adamantylmethylsulfonyl)-4-[4-[[4-(5-hydroxypyridin-3-yl)-2,6-dimethoxyphenyl]methyl]piperazin-1-yl]benzamide |
| 2A61 | N-(1-Adamantylmethylsulfonyl)-4-[4-[[3-(3-hydroxyphenyl)-5-(trifluoromethyl)phenyl]methyl]piperazin-1-yl]benzamide |
| 2A62 | N-(1-Adamantylmethylsulfonyl)-4-[4-[[3-(5-hydroxypyridin-3-yl)-5-(trifluoromethyl)phenyl]methyl]piperazin-1-yl]benzamide |
| 2A63 | N-(1-Adamantylmethylsulfonyl)-4-[4-[[3-(3-hydroxyphenyl)-5-(trifluoromethoxy)phenyl]methyl]piperazin-1-yl]benzamide |
| 2A64 | N-(1-Adamantylmethylsulfonyl)-4-[4-[[3-(5-hydroxypyridin-3-yl)-5-(trifluoromethoxy)phenyl]methyl]piperazin-1-yl]benzamide |
| 2A65 | N-(1-Adamantylmethylsulfonyl)-4-[4-[[4-(3-hydroxyphenyl)naphthalen-1-yl]methyl]piperazin-1-yl]benzamide |
| 2A66 | N-(1-Adamantylmethylsulfonyl)-4-[4-[[4-(5-hydroxypyridin-3-yl)naphthalen-1-yl]methyl]piperazin-1-yl]benzamide |
| 2A67 | N-(1-Adamantylmethylsulfonyl)-4-[4-[[4-(3-hydroxyphenyl)thiophen-2-yl]methyl]piperazin-1-yl]benzamide |
| 2A68 | N-(1-Adamantylmethylsulfonyl)-4-[4-[[4-(5-hydroxypyridin-3-yl)thiophen-2-yl]methyl]piperazin-1-yl]benzamide |
| 2A69 | N-(1-Adamantylmethylsulfonyl)-4-[4-[[2-[2-(3-hydroxyphenyl)ethynyl]phenyl]methyl]piperazin-1-yl]benzamide |
| 2A70 | N-(1-Adamantylmethylsulfonyl)-4-[4-[[2-[2-(5-hydroxypyridin-3-yl)ethynyl]phenyl]methyl]piperazin-1-yl]benzamide |
| 2A71 | N-(1-Adamantylmethylsulfonyl)-6-[4-[[2-(3-hydroxyphenyl)phenyl]methyl]piperazin-1-yl]pyridazine-3-carboxamide |
| 2A72 | N-(1-Adamantylmethylsulfonyl)-6-[4-[[2-(5-hydroxypyridin-3-yl)phenyl]methyl]piperazin-1-yl]pyridazine-3-carboxamide |

| | |
|---|---|
| 2A73 | N-(1-Adamantylmethylsulfonyl)-6-[4-[[4-(3-hydroxyphenyl)-3-methylphenyl]methyl]piperazin-1-yl]pyridazine-3-carboxamide |
| 2A74 | N-(1-Adamantylmethylsulfonyl)-6-[4-[[4-(5-hydroxypyridin-3-yl)-3-methylphenyl]methyl]piperazin-1-yl]pyridazine-3-carboxamide |
| 2A75 | N-(1-Adamantylmethylsulfonyl)-6-[4-[[2-ethyl-4-(3-hydroxyphenyl)phenyl]methyl]piperazin-1-yl]pyridazine-3-carboxamide |
| 2A76 | N-(1-Adamantylmethylsulfonyl)-6-[4-[[2-ethyl-4-(5-hydroxypyridin-3-yl)phenyl]methyl]piperazin-1-yl]pyridazine-3-carboxamide |
| 2A77 | N-(1-Adamantylmethylsulfonyl)-6-[4-[[3-fluoro-4-(3-hydroxyphenyl)phenyl]methyl]piperazin-1-yl]pyridazine-3-carboxamide |
| 2A78 | N-(1-Adamantylmethylsulfonyl)-6-[4-[[3-fluoro-4-(5-hydroxypyridin-3-yl)phenyl]methyl]piperazin-1-yl]pyridazine-3-carboxamide |
| 2A79 | N-(1-Adamantylmethylsulfonyl)-6-[4-[[4-(3-hydroxyphenyl)-2-methoxyphenyl]methyl]piperazin-1-yl]pyridazine-3-carboxamide |
| 2A80 | N-(1-Adamantylmethylsulfonyl)-6-[4-[[4-(5-hydroxypyridin-3-yl)-2-methoxyphenyl]methyl]piperazin-1-yl]pyridazine-3-carboxamide |
| 2A81 | N-(1-Adamantylmethylsulfonyl)-6-[4-[[3-ethoxy-5-(3-hydroxyphenyl)phenyl]methyl]piperazin-1-yl]pyridazine-3-carboxamide |
| 2A82 | N-(1-Adamantylmethylsulfonyl)-6-[4-[[3-ethoxy-5-(5-hydroxypyridin-3-yl)phenyl]methyl]piperazin-1-yl]pyridazine-3-carboxamide |
| 2A83 | N-(1-Adamantylmethylsulfonyl)-6-[4-[[3-(3-hydroxyphenyl)-5-propan-2-yloxyphenyl]methyl]piperazin-1-yl]pyridazine-3-carboxamide |
| 2A84 | N-(1-Adamantylmethylsulfonyl)-6-[4-[[3-(5-hydroxypyridin-3-yl)-5-propan-2-yloxyphenyl]methyl]piperazin-1-yl]pyridazine-3-carboxamide |
| 2A85 | N-(1-Adamantylmethylsulfonyl)-6-[4-[[4-(3-hydroxyphenyl)-2,6-dimethoxyphenyl]methyl]piperazin-1-yl]pyridazine-3-carboxamide |
| 2A86 | N-(1-Adamantylmethylsulfonyl)-6-[4-[[4-(5-hydroxypyridin-3-yl)-2,6-dimethoxyphenyl]methyl]piperazin-1-yl]pyridazine-3-carboxamide |
| 2A87 | N-(1-Adamantylmethylsulfonyl)-6-[4-[[3-(3-hydroxyphenyl)-5-(trifluoromethyl)phenyl]methyl]piperazin-1-yl]pyridazine-3-carboxamide |
| 2A88 | N-(1-Adamantylmethylsulfonyl)-6-[4-[[3-(5-hydroxypyridin-3-yl)-5-(trifluoromethyl)phenyl]methyl]piperazin-1-yl]pyridazine-3-carboxamide |
| 2A89 | N-(1-Adamantylmethylsulfonyl)-6-[4-[[3-(3-hydroxyphenyl)-5-(trifluoromethoxy)phenyl]methyl]piperazin-1-yl]pyridazine-3-carboxamide |
| 2A90 | N-(1-Adamantylmethylsulfonyl)-6-[4-[[3-(5-hydroxypyridin-3-yl)-5-(trifluoromethoxy)phenyl]methyl]piperazin-1-yl]pyridazine-3-carboxamide |
| 2A91 | N-(1-Adamantylmethylsulfonyl)-6-[4-[[4-(3-hydroxyphenyl)naphthalen-1-yl]methyl]piperazin-1-yl]pyridazine-3-carboxamide |
| 2A92 | N-(1-Adamantylmethylsulfonyl)-6-[4-[[4-(5-hydroxypyridin-3-yl)naphthalen-1-yl]methyl]piperazin-1-yl]pyridazine-3-carboxamide |
| 2A93 | N-(1-Adamantylmethylsulfonyl)-6-[4-[[4-(3-hydroxyphenyl)thiophen-2-yl]methyl]piperazin-1-yl]pyridazine-3-carboxamide |
| 2A94 | N-(1-Adamantylmethylsulfonyl)-6-[4-[[4-(5-hydroxypyridin-3-yl)thiophen-2-yl]methyl]piperazin-1-yl]pyridazine-3-carboxamide |
| 2A95 | N-(1-Adamantylmethylsulfonyl)-6-[4-[[2-[2-(3-hydroxyphenyl)ethynyl]phenyl]methyl]piperazin-1-yl]pyridazine-3-carboxamide |
| 2A96 | N-(1-Adamantylmethylsulfonyl)-6-[4-[[2-[2-(5-hydroxypyridin-3-yl)ethynyl]phenyl]methyl]piperazin-1-yl]pyridazine-3-carboxamide |
| 2A97 | N-(1-Adamantylmethylsulfonyl)-4-[4-[1-[4-(3-hydroxyphenyl)phenyl]ethyl]piperazin-1-yl]benzamide |
| 2A98 | N-(1-Adamantylmethylsulfonyl)-4-[4-[1-[4-(5-hydroxypyridin-3-yl)phenyl]ethyl]piperazin-1-yl]benzamide |
| 2A99 | N-(1-Adamantylmethylsulfonyl)-4-[4-[1-[2-fluoro-4-(3-hydroxyphenyl)phenyl]ethyl]piperazin-1-yl]benzamide |
| 2A100 | N-(1-Adamantylmethylsulfonyl)-4-[4-[1-[2-fluoro-4-(5-hydroxypyridin-3-yl)phenyl]ethyl]piperazin-1-yl]benzamide |

[Table 225]

[Table EIJ11]

| No. of mixture used | mixEI17-02R |
|---|---|
| Mixture No after cleavage | mixEJ11 |
| No. of compound in Table EJ11 | Compound name |
| 2Q01 | 6-[4-[4-(3-Hydroxyphenyl)thiophene-2-carbonyl]piperazin-1-yl]-N-[3-nitro-4-(2-phenylsulfanylethylamino)phenyl]sulfonylpyridazine-3-carboxamide |
| 2Q02 | 6-[4-[4-(5-Hydroxypyridin-3-yl)thiophene-2-carbonyl]piperazin-1-yl]-N-[3-nitro-4-(2-phenylsulfanylethylamino)phenyl]sulfonylpyridazine-3-carboxamide |
| 2Q03 | 6-[4-[4-(3-Hydroxyphenyl)-3-methylbenzoyl]piperazin-1-yl]-N-[3-nitro-4-(2-phenylsulfanylethylamino)phenyl]sulfonylpyridazine-3-carboxamide |
| 2Q04 | 6-[4-[4-(5-Hydroxypyridin-3-yl)-3-methylbenzoyl]piperazin-1-yl]-N-[3-nitro-4-(2-phenylsulfanylethylamino)phenyl]sulfonylpyridazine-3-carboxamide |
| 2Q05 | 6-[4-[3-Fluoro-4-(3-hydroxyphenyl)benzoyl]piperazin-1-yl]-N-[3-nitro-4-(2-phenylsulfanylethylamino)phenyl]sulfonylpyridazine-3-carboxamide |
| 2Q06 | 6-[4-[3-Fluoro-4-(5-hydroxypyridin-3-yl)benzoyl]piperazin-1-yl]-N-[3-nitro-4-(2-phenylsulfanylethylamino)phenyl]sulfonylpyridazine-3-carboxamide |
| 2Q07 | 6-[4-[2-[2-(3-Hydroxyphenyl)ethynyl]benzoyl]piperazin-1-yl]-N-[3-nitro-4-(2-phenylsulfanylethylamino)phenyl]sulfonylpyridazine-3-carboxamide |
| 2Q08 | 6-[4-[2-[2-(5-Hydroxypyridin-3-yl)ethynyl]benzoyl]piperazin-1-yl]-N-[3-nitro-4-(2-phenylsulfanylethylamino)phenyl]sulfonylpyridazine-3-carboxamide |
| 2Q09 | 6-[4-[5-[2-(3-Hydroxyphenyl)ethynyl]pyridine-3-carbonyl]piperazin-1-yl]-N-[3-nitro-4-(2-phenylsulfanylethylamino)phenyl]sulfonylpyridazine-3-carboxamide |
| 2Q10 | 6-[4-[5-[2-(5-Hydroxypyridin-3-yl)ethynyl]pyridine-3-carbonyl]piperazin-1-yl]-N-[3-nitro-4-(2-phenylsulfanylethylamino)phenyl]sulfonylpyridazine-3-carboxamide |
| 2Q11 | 6-[4-[3-[2-(3-Hydroxyphenyl)phenyl]propanoyl]piperazin-1-yl]-N-[3-nitro-4-(2-phenylsulfanylethylamino)phenyl]sulfonylpyridazine-3-carboxamide |
| 2Q12 | 6-[4-[3-[2-(5-Hydroxypyridin-3-yl)phenyl]propanoyl]piperazin-1-yl]-N-[3-nitro-4-(2-phenylsulfanylethylamino)phenyl]sulfonylpyridazine-3-carboxamide |
| 2Q13 | 6-[4-[4-(3-Hydroxyphenyl)naphthalene-1-carbonyl]piperazin-1-yl]-N-[3-nitro-4-(2-phenylsulfanylethylamino)phenyl]sulfonylpyridazine-3-carboxamide |
| 2Q14 | 6-[4-[4-(5-Hydroxypyridin-3-yl)naphthalene-1-carbonyl]piperazin-1-yl]-N-[3-nitro-4-(2-phenylsulfanylethylamino)phenyl]sulfonylpyridazine-3-carboxamide |
| 2Q15 | 6-[4-[3-tert-Butyl-5-(3-hydroxyphenyl)benzoyl]piperazin-1-yl]-N-[3-nitro-4-(2-phenylsulfanylethylamino)phenyl]sulfonylpyridazine-3-carboxamide |
| 2Q16 | 6-[4-[3-tert-Butyl-5-(5-hydroxypyridin-3-yl)benzoyl]piperazin-1-yl]-N-[3-nitro-4-(2-phenylsulfanylethylamino)phenyl]sulfonylpyridazine-3-carboxamide |
| 2Q17 | 6-[4-[3-(3-Hydroxyphenyl)-5-(trifluoromethyl)benzoyl]piperazin-1-yl]-N-[3-nitro-4-(2-phenylsulfanylethylamino)phenyl]sulfonylpyridazine-3-carboxamide |
| 2Q18 | 6-[4-[3-(5-Hydroxypyridin-3-yl)-5-(trifluoromethyl)benzoyl]piperazin-1-yl]-N-[3-nitro-4-(2-phenylsulfanylethylamino)phenyl]sulfonylpyridazine-3-carboxamide |
| 2Q19 | 6-[4-[3-[2-(3-Hydroxyphenyl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| 2Q20 | 6-[4-[3-[2-(5-Hydroxypyridin-3-yl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| 2Q21 | 6-[4-[3-[4-(3-Hydroxyphenyl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |

| | |
|---|---|
| 2Q22 | 6-[4-[3-[4-(5-Hydroxypyridin-3-yl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| 2Q23 | 4-[4-[4-(3-Hydroxyphenyl)thiophene-2-carbonyl]piperazin-1-yl]-N-[3-nitro-4-(2-phenylsulfanylethylamino)phenyl]sulfonylbenzamide |
| 2Q24 | 4-[4-[4-(5-Hydroxypyridin-3-yl)thiophene-2-carbonyl]piperazin-1-yl]-N-[3-nitro-4-(2-phenylsulfanylethylamino)phenyl]sulfonylbenzamide |
| 2Q25 | 4-[4-[4-(3-Hydroxyphenyl)-3-methylbenzoyl]piperazin-1-yl]-N-[3-nitro-4-(2-phenylsulfanylethylamino)phenyl]sulfonylbenzamide |
| 2Q26 | 4-[4-[4-(5-Hydroxypyridin-3-yl)-3-methylbenzoyl]piperazin-1-yl]-N-[3-nitro-4-(2-phenylsulfanylethylamino)phenyl]sulfonylbenzamide |
| 2Q27 | 4-[4-[3-Fluoro-4-(3-hydroxyphenyl)benzoyl]piperazin-1-yl]-N-[3-nitro-4-(2-phenylsulfanylethylamino)phenyl]sulfonylbenzamide |
| 2Q28 | 4-[4-[3-Fluoro-4-(5-hydroxypyridin-3-yl)benzoyl]piperazin-1-yl]-N-[3-nitro-4-(2-phenylsulfanylethylamino)phenyl]sulfonylbenzamide |
| 2Q29 | 4-[4-[2-[2-(3-Hydroxyphenyl)ethynyl]benzoyl]piperazin-1-yl]-N-[3-nitro-4-(2-phenylsulfanylethylamino)phenyl]sulfonylbenzamide |
| 2Q30 | 4-[4-[2-[2-(5-Hydroxypyridin-3-yl)ethynyl]benzoyl]piperazin-1-yl]-N-[3-nitro-4-(2-phenylsulfanylethylamino)phenyl]sulfonylbenzamide |
| 2Q31 | 4-[4-[5-[2-(3-Hydroxyphenyl)ethynyl]pyridine-3-carbonyl]piperazin-1-yl]-N-[3-nitro-4-(2-phenylsulfanylethylamino)phenyl]sulfonylbenzamide |
| 2Q32 | 4-[4-[5-[2-(5-Hydroxypyridin-3-yl)ethynyl]pyridine-3-carbonyl]piperazin-1-yl]-N-[3-nitro-4-(2-phenylsulfanylethylamino)phenyl]sulfonylbenzamide |
| 2Q33 | 4-[4-[3-[2-(3-Hydroxyphenyl)phenyl]propanoyl]piperazin-1-yl]-N-[3-nitro-4-(2-phenylsulfanylethylamino)phenyl]sulfonylbenzamide |
| 2Q34 | 4-[4-[3-[2-(5-Hydroxypyridin-3-yl)phenyl]propanoyl]piperazin-1-yl]-N-[3-nitro-4-(2-phenylsulfanylethylamino)phenyl]sulfonylbenzamide |
| 2Q35 | 4-[4-[4-(3-Hydroxyphenyl)naphthalene-1-carbonyl]piperazin-1-yl]-N-[3-nitro-4-(2-phenylsulfanylethylamino)phenyl]sulfonylbenzamide |
| 2Q36 | 4-[4-[4-(5-Hydroxypyridin-3-yl)naphthalene-1-carbonyl]piperazin-1-yl]-N-[3-nitro-4-(2-phenylsulfanylethylamino)phenyl]sulfonylbenzamide |
| 2Q37 | 4-[4-[3-tert-Butyl-5-(3-hydroxyphenyl)benzoyl]piperazin-1-yl]-N-[3-nitro-4-(2-phenylsulfanylethylamino)phenyl]sulfonylbenzamide |
| 2Q38 | 4-[4-[3-tert-Butyl-5-(5-hydroxypyridin-3-yl)benzoyl]piperazin-1-yl]-N-[3-nitro-4-(2-phenylsulfanylethylamino)phenyl]sulfonylbenzamide |
| 2Q39 | 4-[4-[3-(3-Hydroxyphenyl)-5-(trifluoromethyl)benzoyl]piperazin-1-yl]-N-[3-nitro-4-(2-phenylsulfanylethylamino)phenyl]sulfonylbenzamide |
| 2Q40 | 4-[4-[3-(5-Hydroxypyridin-3-yl)-5-(trifluoromethyl)benzoyl]piperazin-1-yl]-N-[3-nitro-4-(2-phenylsulfanylethylamino)phenyl]sulfonylbenzamide |
| 2Q41 | 4-[4-[3-[2-(3-Hydroxyphenyl)ethynyl]-5-(trifluoromethyl)benzoyl]piperazin-1-yl]-N-[3-nitro-4-(2-phenylsulfanylethylamino)phenyl]sulfonylbenzamide |
| 2Q42 | 4-[4-[3-[2-(5-Hydroxypyridin-3-yl)ethynyl]-5-(trifluoromethyl)benzoyl]piperazin-1-yl]-N-[3-nitro-4-(2-phenylsulfanylethylamino)phenyl]sulfonylbenzamide |
| 2Q43 | 4-[4-[3-[4-(3-Hydroxyphenyl)pyrazol-1-yl]-5-(trifluoromethyl)benzoyl]piperazin-1-yl]-N-[3-nitro-4-(2-phenylsulfanylethylamino)phenyl]sulfonylbenzamide |
| 2Q44 | 4-[4-[3-[4-(5-Hydroxypyridin-3-yl)pyrazol-1-yl]-5-(trifluoromethyl)benzoyl]piperazin-1-yl]-N-[3-nitro-4-(2-phenylsulfanylethylamino)phenyl]sulfonylbenzamide |
| 2Q45 | 4-[4-[[2-(3-Hydroxyphenyl)phenyl]methyl]piperazin-1-yl]-N-[3-nitro-4-(2-phenylsulfanylethylamino)phenyl]sulfonylbenzamide |
| 2Q46 | 4-[4-[[2-(5-Hydroxypyridin-3-yl)phenyl]methyl]piperazin-1-yl]-N-[3-nitro-4-(2-phenylsulfanylethylamino)phenyl]sulfonylbenzamide |
| 2Q47 | 4-[4-[[4-(3-Hydroxyphenyl)-3-methylphenyl]methyl]piperazin-1-yl]-N-[3-nitro-4-(2-phenylsulfanylethylamino)phenyl]sulfonylbenzamide |
| 2Q48 | 4-[4-[[4-(5-Hydroxypyridin-3-yl)-3-methylphenyl]methyl]piperazin-1-yl]-N-[3-nitro-4-(2-phenylsulfanylethylamino)phenyl]sulfonylbenzamide |

| | |
|---|---|
| 2Q49 | 4-[4-[[2-Ethyl-4-(3-hydroxyphenyl)phenyl]methyl]piperazin-1-yl]-N-[3-nitro-4-(2-phenylsulfanylethylamino)phenyl]sulfonylbenzamide |
| 2Q50 | 4-[4-[[2-Ethyl-4-(5-hydroxypyridin-3-yl)phenyl]methyl]piperazin-1-yl]-N-[3-nitro-4-(2-phenylsulfanylethylamino)phenyl]sulfonylbenzamide |
| 2Q51 | 4-[4-[[3-Fluoro-4-(3-hydroxyphenyl)phenyl]methyl]piperazin-1-yl]-N-[3-nitro-4-(2-phenylsulfanylethylamino)phenyl]sulfonylbenzamide |
| 2Q52 | 4-[4-[[3-Fluoro-4-(5-hydroxypyridin-3-yl)phenyl]methyl]piperazin-1-yl]-N-[3-nitro-4-(2-phenylsulfanylethylamino)phenyl]sulfonylbenzamide |
| 2Q53 | 4-[4-[[4-(3-Hydroxyphenyl)-2-methoxyphenyl]methyl]piperazin-1-yl]-N-[3-nitro-4-(2-phenylsulfanylethylamino)phenyl]sulfonylbenzamide |
| 2Q54 | 4-[4-[[4-(5-Hydroxypyridin-3-yl)-2-methoxyphenyl]methyl]piperazin-1-yl]-N-[3-nitro-4-(2-phenylsulfanylethylamino)phenyl]sulfonylbenzamide |
| 2Q55 | 4-[4-[[3-Ethoxy-5-(3-hydroxyphenyl)phenyl]methyl]piperazin-1-yl]-N-[3-nitro-4-(2-phenylsulfanylethylamino)phenyl]sulfonylbenzamide |
| 2Q56 | 4-[4-[[3-Ethoxy-5-(5-hydroxypyridin-3-yl)phenyl]methyl]piperazin-1-yl]-N-[3-nitro-4-(2-phenylsulfanylethylamino)phenyl]sulfonylbenzamide |
| 2Q57 | 4-[4-[[3-(3-Hydroxyphenyl)-5-propan-2-yloxyphenyl]methyl]piperazin-1-yl]-N-[3-nitro-4-(2-phenylsulfanylethylamino)phenyl]sulfonylbenzamide |
| 2Q58 | 4-[4-[[3-(5-Hydroxypyridin-3-yl)-5-propan-2-yloxyphenyl]methyl]piperazin-1-yl]-N-[3-nitro-4-(2-phenylsulfanylethylamino)phenyl]sulfonylbenzamide |
| 2Q59 | 4-[4-[[4-(3-Hydroxyphenyl)-2,6-dimethoxyphenyl]methyl]piperazin-1-yl]-N-[3-nitro-4-(2-phenylsulfanylethylamino)phenyl]sulfonylbenzamide |
| 2Q60 | 4-[4-[[4-(5-Hydroxypyridin-3-yl)-2,6-dimethoxyphenyl]methyl]piperazin-1-yl]-N-[3-nitro-4-(2-phenylsulfanylethylamino)phenyl]sulfonylbenzamide |
| 2Q61 | 4-[4-[[3-(3-Hydroxyphenyl)-5-(trifluoromethyl)phenyl]methyl]piperazin-1-yl]-N-[3-nitro-4-(2-phenylsulfanylethylamino)phenyl]sulfonylbenzamide |
| 2Q62 | 4-[4-[[3-(5-Hydroxypyridin-3-yl)-5-(trifluoromethyl)phenyl]methyl]piperazin-1-yl]-N-[3-nitro-4-(2-phenylsulfanylethylamino)phenyl]sulfonylbenzamide |
| 2Q63 | 4-[4-[[3-(3-Hydroxyphenyl)-5-(trifluoromethoxy)phenyl]methyl]piperazin-1-yl]-N-[3-nitro-4-(2-phenylsulfanylethylamino)phenyl]sulfonylbenzamide |
| 2Q64 | 4-[4-[[3-(5-Hydroxypyridin-3-yl)-5-(trifluoromethoxy)phenyl]methyl]piperazin-1-yl]-N-[3-nitro-4-(2-phenylsulfanylethylamino)phenyl]sulfonylbenzamide |
| 2Q65 | 4-[4-[[4-(3-Hydroxyphenyl)naphthalen-1-yl]methyl]piperazin-1-yl]-N-[3-nitro-4-(2-phenylsulfanylethylamino)phenyl]sulfonylbenzamide |
| 2Q66 | 4-[4-[[4-(5-Hydroxypyridin-3-yl)naphthalen-1-yl]methyl]piperazin-1-yl]-N-[3-nitro-4-(2-phenylsulfanylethylamino)phenyl]sulfonylbenzamide |
| 2Q67 | 4-[4-[[4-(3-Hydroxyphenyl)thiophen-2-yl]methyl]piperazin-1-yl]-N-[3-nitro-4-(2-phenylsulfanylethylamino)phenyl]sulfonylbenzamide |
| 2Q68 | 4-[4-[[4-(5-Hydroxypyridin-3-yl)thiophen-2-yl]methyl]piperazin-1-yl]-N-[3-nitro-4-(2-phenylsulfanylethylamino)phenyl]sulfonylbenzamide |
| 2Q69 | 4-[4-[[2-[2-(3-Hydroxyphenyl)ethynyl]phenyl]methyl]piperazin-1-yl]-N-[3-nitro-4-(2-phenylsulfanylethylamino)phenyl]sulfonylbenzamide |
| 2Q70 | 4-[4-[[2-[2-(5-Hydroxypyridin-3-yl)ethynyl]phenyl]methyl]piperazin-1-yl]-N-[3-nitro-4-(2-phenylsulfanylethylamino)phenyl]sulfonylbenzamide |
| 2Q71 | 6-[4-[[2-(3-Hydroxyphenyl)phenyl]methyl]piperazin-1-yl]-N-[3-nitro-4-(2-phenylsulfanylethylamino)phenyl]sulfonylpyridazine-3-carboxamide |
| 2Q72 | 6-[4-[[2-(5-Hydroxypyridin-3-yl)phenyl]methyl]piperazin-1-yl]-N-[3-nitro-4-(2-phenylsulfanylethylamino)phenyl]sulfonylpyridazine-3-carboxamide |
| 2Q73 | 6-[4-[[4-(3-Hydroxyphenyl)-3-methylphenyl]methyl]piperazin-1-yl]-N-[3-nitro-4-(2-phenylsulfanylethylamino)phenyl]sulfonylpyridazine-3-carboxamide |
| 2Q74 | 6-[4-[[4-(5-Hydroxypyridin-3-yl)-3-methylphenyl]methyl]piperazin-1-yl]-N-[3-nitro-4-(2-phenylsulfanylethylamino)phenyl]sulfonylpyridazine-3-carboxamide |
| 2Q75 | 6-[4-[[2-Ethyl-4-(3-hydroxyphenyl)phenyl]methyl]piperazin-1-yl]-N-[3-nitro-4-(2-phenylsulfanylethylamino)phenyl]sulfonylpyridazine-3-carboxamide |
| 2Q76 | 6-[4-[[2-Ethyl-4-(5-hydroxypyridin-3-yl)phenyl]methyl]piperazin-1-yl]-N-[3-nitro-4-(2-phenylsulfanylethylamino)phenyl]sulfonylpyridazine-3-carboxamide |

| | |
|---|---|
| 2Q77 | 6-[4-[[3-Fluoro-4-(3-hydroxyphenyl)phenyl]methyl]piperazin-1-yl]-N-[3-nitro-4-(2-phenylsulfanylethylamino)phenyl]sulfonylpyridazine-3-carboxamide |
| 2Q78 | 6-[4-[[3-Fluoro-4-(5-hydroxypyridin-3-yl)phenyl]methyl]piperazin-1-yl]-N-[3-nitro-4-(2-phenylsulfanylethylamino)phenyl]sulfonylpyridazine-3-carboxamide |
| 2Q79 | 6-[4-[[4-(3-Hydroxyphenyl)-2-methoxyphenyl]methyl]piperazin-1-yl]-N-[3-nitro-4-(2-phenylsulfanylethylamino)phenyl]sulfonylpyridazine-3-carboxamide |
| 2Q80 | 6-[4-[[4-(5-Hydroxypyridin-3-yl)-2-methoxyphenyl]methyl]piperazin-1-yl]-N-[3-nitro-4-(2-phenylsulfanylethylamino)phenyl]sulfonylpyridazine-3-carboxamide |
| 2Q81 | 6-[4-[[3-Ethoxy-5-(3-hydroxyphenyl)phenyl]methyl]piperazin-1-yl]-N-[3-nitro-4-(2-phenylsulfanylethylamino)phenyl]sulfonylpyridazine-3-carboxamide |
| 2Q82 | 6-[4-[[3-Ethoxy-5-(5-hydroxypyridin-3-yl)phenyl]methyl]piperazin-1-yl]-N-[3-nitro-4-(2-phenylsulfanylethylamino)phenyl]sulfonylpyridazine-3-carboxamide |
| 2Q83 | 6-[4-[[3-(3-Hydroxyphenyl)-5-propan-2-yloxyphenyl]methyl]piperazin-1-yl]-N-[3-nitro-4-(2-phenylsulfanylethylamino)phenyl]sulfonylpyridazine-3-carboxamide |
| 2Q84 | 6-[4-[[3-(5-Hydroxypyridin-3-yl)-5-propan-2-yloxyphenyl]methyl]piperazin-1-yl]-N-[3-nitro-4-(2-phenylsulfanylethylamino)phenyl]sulfonylpyridazine-3-carboxamide |
| 2Q85 | 6-[4-[[4-(3-Hydroxyphenyl)-2,6-dimethoxyphenyl]methyl]piperazin-1-yl]-N-[3-nitro-4-(2-phenylsulfanylethylamino)phenyl]sulfonylpyridazine-3-carboxamide |
| 2Q86 | 6-[4-[[4-(5-Hydroxypyridin-3-yl)-2,6-dimethoxyphenyl]methyl]piperazin-1-yl]-N-[3-nitro-4-(2-phenylsulfanylethylamino)phenyl]sulfonylpyridazine-3-carboxamide |
| 2Q87 | 6-[4-[[3-(3-Hydroxyphenyl)-5-(trifluoromethyl)phenyl]methyl]piperazin-1-yl]-N-[3-nitro-4-(2-phenylsulfanylethylamino)phenyl]sulfonylpyridazine-3-carboxamide |
| 2Q88 | 6-[4-[[3-(5-Hydroxypyridin-3-yl)-5-(trifluoromethyl)phenyl]methyl]piperazin-1-yl]-N-[3-nitro-4-(2-phenylsulfanylethylamino)phenyl]sulfonylpyridazine-3-carboxamide |
| 2Q89 | 6-[4-[[3-(3-Hydroxyphenyl)-5-(trifluoromethoxy)phenyl]methyl]piperazin-1-yl]-N-[3-nitro-4-(2-phenylsulfanylethylamino)phenyl]sulfonylpyridazine-3-carboxamide |
| 2Q90 | 6-[4-[[3-(5-Hydroxypyridin-3-yl)-5-(trifluoromethoxy)phenyl]methyl]piperazin-1-yl]-N-[3-nitro-4-(2-phenylsulfanylethylamino)phenyl]sulfonylpyridazine-3-carboxamide |
| 2Q91 | 6-[4-[[4-(3-Hydroxyphenyl)naphthalen-1-yl]methyl]piperazin-1-yl]-N-[3-nitro-4-(2-phenylsulfanylethylamino)phenyl]sulfonylpyridazine-3-carboxamide |
| 2Q92 | 6-[4-[[4-(5-Hydroxypyridin-3-yl)naphthalen-1-yl]methyl]piperazin-1-yl]-N-[3-nitro-4-(2-phenylsulfanylethylamino)phenyl]sulfonylpyridazine-3-carboxamide |
| 2Q93 | 6-[4-[[4-(3-Hydroxyphenyl)thiophen-2-yl]methyl]piperazin-1-yl]-N-[3-nitro-4-(2-phenylsulfanylethylamino)phenyl]sulfonylpyridazine-3-carboxamide |
| 2Q94 | 6-[4-[[4-(5-Hydroxypyridin-3-yl)thiophen-2-yl]methyl]piperazin-1-yl]-N-[3-nitro-4-(2-phenylsulfanylethylamino)phenyl]sulfonylpyridazine-3-carboxamide |
| 2Q95 | 6-[4-[[2-[2-(3-Hydroxyphenyl)ethynyl]phenyl]methyl]piperazin-1-yl]-N-[3-nitro-4-(2-phenylsulfanylethylamino)phenyl]sulfonylpyridazine-3-carboxamide |
| 2Q96 | 6-[4-[[2-[2-(5-Hydroxypyridin-3-yl)ethynyl]phenyl]methyl]piperazin-1-yl]-N-[3-nitro-4-(2-phenylsulfanylethylamino)phenyl]sulfonylpyridazine-3-carboxamide |
| 2Q97 | 4-[4-[1-[4-(3-Hydroxyphenyl)phenyl]ethyl]piperazin-1-yl]-N-[3-nitro-4-(2-phenylsulfanylethylamino)phenyl]sulfonylbenzamide |
| 2Q98 | 4-[4-[1-[4-(5-Hydroxypyridin-3-yl)phenyl]ethyl]piperazin-1-yl]-N-[3-nitro-4-(2-phenylsulfanylethylamino)phenyl]sulfonylbenzamide |
| 2Q99 | 4-[4-[1-[2-Fluoro-4-(3-hydroxyphenyl)phenyl]ethyl]piperazin-1-yl]-N-[3-nitro-4-(2-phenylsulfanylethylamino)phenyl]sulfonylbenzamide |
| 2Q100 | 4-[4-[1-[2-Fluoro-4-(5-hydroxypyridin-3-yl)phenyl]ethyl]piperazin-1-yl]-N-[3-nitro-4-(2-phenylsulfanylethylamino)phenyl]sulfonylbenzamide |

[Table 226]

[Table EIJ12]

| | |
|---|---|
| No. of mixture used | mixEI18-02R |
| Mixture No after cleavage | mixEJ12 |

| No. of compound in Table EJ12 | Compound name |
|---|---|
| 2R01 | 6-[4-[4-(3-Hydroxyphenyl)thiophene-2-carbonyl]piperazin-1-yl]-N-[4-(2-phenylsulfanylethylamino)-3-(trifluoromethyl)phenyl]sulfonylpyridazine-3-carboxamide |
| 2R02 | 6-[4-[4-(5-Hydroxypyridin-3-yl)thiophene-2-carbonyl]piperazin-1-yl]-N-[4-(2-phenylsulfanylethylamino)-3-(trifluoromethyl)phenyl]sulfonylpyridazine-3-carboxamide |
| 2R03 | 6-[4-[4-(3-Hydroxyphenyl)-3-methylbenzoyl]piperazin-1-yl]-N-[4-(2-phenylsulfanylethylamino)-3-(trifluoromethyl)phenyl]sulfonylpyridazine-3-carboxamide |
| 2R04 | 6-[4-[4-(5-Hydroxypyridin-3-yl)-3-methylbenzoyl]piperazin-1-yl]-N-[4-(2-phenylsulfanylethylamino)-3-(trifluoromethyl)phenyl]sulfonylpyridazine-3-carboxamide |
| 2R05 | 6-[4-[3-Fluoro-4-(3-hydroxyphenyl)benzoyl]piperazin-1-yl]-N-[4-(2-phenylsulfanylethylamino)-3-(trifluoromethyl)phenyl]sulfonylpyridazine-3-carboxamide |
| 2R06 | 6-[4-[3-Fluoro-4-(5-hydroxypyridin-3-yl)benzoyl]piperazin-1-yl]-N-[4-(2-phenylsulfanylethylamino)-3-(trifluoromethyl)phenyl]sulfonylpyridazine-3-carboxamide |
| 2R07 | 6-[4-[2-[2-(3-Hydroxyphenyl)ethynyl]benzoyl]piperazin-1-yl]-N-[4-(2-phenylsulfanylethylamino)-3-(trifluoromethyl)phenyl]sulfonylpyridazine-3-carboxamide |
| 2R08 | 6-[4-[2-[2-(5-Hydroxypyridin-3-yl)ethynyl]benzoyl]piperazin-1-yl]-N-[4-(2-phenylsulfanylethylamino)-3-(trifluoromethyl)phenyl]sulfonylpyridazine-3-carboxamide |
| 2R09 | 6-[4-[5-[2-(3-Hydroxyphenyl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| 2R10 | 6-[4-[5-[2-(5-Hydroxypyridin-3-yl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| 2R11 | 6-[4-[3-[2-(3-Hydroxyphenyl)phenyl]propanoyl]piperazin-1-yl]-N-[4-(2-phenylsulfanylethylamino)-3-(trifluoromethyl)phenyl]sulfonylpyridazine-3-carboxamide |
| 2R12 | 6-[4-[3-[2-(5-Hydroxypyridin-3-yl)phenyl]propanoyl]piperazin-1-yl]-N-[4-(2-phenylsulfanylethylamino)-3-(trifluoromethyl)phenyl]sulfonylpyridazine-3-carboxamide |
| 2R13 | 6-[4-[4-(3-Hydroxyphenyl)naphthalene-1-carbonyl]piperazin-1-yl]-N-[4-(2-phenylsulfanylethylamino)-3-(trifluoromethyl)phenyl]sulfonylpyridazine-3-carboxamide |
| 2R14 | 6-[4-[4-(5-Hydroxypyridin-3-yl)naphthalene-1-carbonyl]piperazin-1-yl]-N-[4-(2-phenylsulfanylethylamino)-3-(trifluoromethyl)phenyl]sulfonylpyridazine-3-carboxamide |
| 2R15 | 6-[4-[3-tert-Butyl-5-(3-hydroxyphenyl)benzoyl]piperazin-1-yl]-N-[4-(2-phenylsulfanylethylamino)-3-(trifluoromethyl)phenyl]sulfonylpyridazine-3-carboxamide |
| 2R16 | 6-[4-[3-tert-Butyl-5-(5-hydroxypyridin-3-yl)benzoyl]piperazin-1-yl]-N-[4-(2-phenylsulfanylethylamino)-3-(trifluoromethyl)phenyl]sulfonylpyridazine-3-carboxamide |
| 2R17 | 6-[4-[3-(3-Hydroxyphenyl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R18 | 6-[4-[3-(5-Hydroxypyridin-3-yl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |

| | |
|---|---|
| 2R19 | 6-[4-[3-[2-(3-Hydroxyphenyl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R20 | 6-[4-[3-[2-(5-Hydroxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R21 | 6-[4-[3-[4-(3-Hydroxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R22 | 6-[4-[3-[4-(5-Hydroxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R23 | 4-[4-[4-(3-Hydroxyphenyl)thiophene-2-carbonyl]piperazin-1-yl]-N-[4-(2-phenylsulfanylethylamino)-3-(trifluoromethyl)phenyl]sulfonylbenzamide |
| 2R24 | 4-[4-[4-(5-Hydroxypyridin-3-yl)thiophene-2-carbonyl]piperazin-1-yl]-N-[4-(2-phenylsulfanylethylamino)-3-(trifluoromethyl)phenyl]sulfonylbenzamide |
| 2R25 | 4-[4-[4-(3-Hydroxyphenyl)-3-methylbenzoyl]piperazin-1-yl]-N-[4-(2-phenylsulfanylethylamino)-3-(trifluoromethyl)phenyl]sulfonylbenzamide |
| 2R26 | 4-[4-[4-(5-Hydroxypyridin-3-yl)-3-methylbenzoyl]piperazin-1-yl]-N-[4-(2-phenylsulfanylethylamino)-3-(trifluoromethyl)phenyl]sulfonylbenzamide |
| 2R27 | 4-[4-[3-Fluoro-4-(3-hydroxyphenyl)benzoyl]piperazin-1-yl]-N-[4-(2-phenylsulfanylethylamino)-3-(trifluoromethyl)phenyl]sulfonylbenzamide |
| 2R28 | 4-[4-[3-Fluoro-4-(5-hydroxypyridin-3-yl)benzoyl]piperazin-1-yl]-N-[4-(2-phenylsulfanylethylamino)-3-(trifluoromethyl)phenyl]sulfonylbenzamide |
| 2R29 | 4-[4-[2-[2-(3-Hydroxyphenyl)ethynyl]benzoyl]piperazin-1-yl]-N-[4-(2-phenylsulfanylethylamino)-3-(trifluoromethyl)phenyl]sulfonylbenzamide |
| 2R30 | 4-[4-[2-[2-(5-Hydroxypyridin-3-yl)ethynyl]benzoyl]piperazin-1-yl]-N-[4-(2-phenylsulfanylethylamino)-3-(trifluoromethyl)phenyl]sulfonylbenzamide |
| 2R31 | 4-[4-[5-[2-(3-Hydroxyphenyl)ethynyl]pyridine-3-carbonyl]piperazin-1-yl]-N-[4-(2-phenylsulfanylethylamino)-3-(trifluoromethyl)phenyl]sulfonylbenzamide |
| 2R32 | 4-[4-[5-[2-(5-Hydroxypyridin-3-yl)ethynyl]pyridine-3-carbonyl]piperazin-1-yl]-N-[4-(2-phenylsulfanylethylamino)-3-(trifluoromethyl)phenyl]sulfonylbenzamide |
| 2R33 | 4-[4-[3-[2-(3-Hydroxyphenyl)phenyl]propanoyl]piperazin-1-yl]-N-[4-(2-phenylsulfanylethylamino)-3-(trifluoromethyl)phenyl]sulfonylbenzamide |
| 2R34 | 4-[4-[3-[2-(5-Hydroxypyridin-3-yl)phenyl]propanoyl]piperazin-1-yl]-N-[4-(2-phenylsulfanylethylamino)-3-(trifluoromethyl)phenyl]sulfonylbenzamide |
| 2R35 | 4-[4-[4-(3-Hydroxyphenyl)naphthalene-1-carbonyl]piperazin-1-yl]-N-[4-(2-phenylsulfanylethylamino)-3-(trifluoromethyl)phenyl]sulfonylbenzamide |
| 2R36 | 4-[4-[4-(5-Hydroxypyridin-3-yl)naphthalene-1-carbonyl]piperazin-1-yl]-N-[4-(2-phenylsulfanylethylamino)-3-(trifluoromethyl)phenyl]sulfonylbenzamide |
| 2R37 | 4-[4-[3-tert-Butyl-5-(3-hydroxyphenyl)benzoyl]piperazin-1-yl]-N-[4-(2-phenylsulfanylethylamino)-3-(trifluoromethyl)phenyl]sulfonylbenzamide |
| 2R38 | 4-[4-[3-tert-Butyl-5-(5-hydroxypyridin-3-yl)benzoyl]piperazin-1-yl]-N-[4-(2-phenylsulfanylethylamino)-3-(trifluoromethyl)phenyl]sulfonylbenzamide |
| 2R39 | 4-[4-[3-(3-Hydroxyphenyl)-5-(trifluoromethyl)benzoyl]piperazin-1-yl]-N-[4-(2-phenylsulfanylethylamino)-3-(trifluoromethyl)phenyl]sulfonylbenzamide |
| 2R40 | 4-[4-[3-(5-Hydroxypyridin-3-yl)-5-(trifluoromethyl)benzoyl]piperazin-1-yl]-N-[4-(2-phenylsulfanylethylamino)-3-(trifluoromethyl)phenyl]sulfonylbenzamide |
| 2R41 | 4-[4-[3-[2-(3-Hydroxyphenyl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| 2R42 | 4-[4-[3-[2-(5-Hydroxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| 2R43 | 4-[4-[3-[4-(3-Hydroxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |
| 2R44 | 4-[4-[3-[4-(5-Hydroxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |

| | |
|---|---|
| 2R45 | 4-[4-[[2-(3-Hydroxyphenyl)phenyl]methyl]piperazin-1-yl]-N-[4-(2-phenylsulfanylethylamino)-3-(trifluoromethyl)phenyl]sulfonylbenzamide |
| 2R46 | 4-[4-[[2-(5-Hydroxypyridin-3-yl)phenyl]methyl]piperazin-1-yl]-N-[4-(2-phenylsulfanylethylamino)-3-(trifluoromethyl)phenyl]sulfonylbenzamide |
| 2R47 | 4-[4-[[4-(3-Hydroxyphenyl)-3-methylphenyl]methyl]piperazin-1-yl]-N-[4-(2-phenylsulfanylethylamino)-3-(trifluoromethyl)phenyl]sulfonylbenzamide |
| 2R48 | 4-[4-[[4-(5-Hydroxypyridin-3-yl)-3-methylphenyl]methyl]piperazin-1-yl]-N-[4-(2-phenylsulfanylethylamino)-3-(trifluoromethyl)phenyl]sulfonylbenzamide |
| 2R49 | 4-[4-[[2-Ethyl-4-(3-hydroxyphenyl)phenyl]methyl]piperazin-1-yl]-N-[4-(2-phenylsulfanylethylamino)-3-(trifluoromethyl)phenyl]sulfonylbenzamide |
| 2R50 | 4-[4-[[2-Ethyl-4-(5-hydroxypyridin-3-yl)phenyl]methyl]piperazin-1-yl]-N-[4-(2-phenylsulfanylethylamino)-3-(trifluoromethyl)phenyl]sulfonylbenzamide |
| 2R51 | 4-[4-[[3-Fluoro-4-(3-hydroxyphenyl)phenyl]methyl]piperazin-1-yl]-N-[4-(2-phenylsulfanylethylamino)-3-(trifluoromethyl)phenyl]sulfonylbenzamide |
| 2R52 | 4-[4-[[3-Fluoro-4-(5-hydroxypyridin-3-yl)phenyl]methyl]piperazin-1-yl]-N-[4-(2-phenylsulfanylethylamino)-3-(trifluoromethyl)phenyl]sulfonylbenzamide |
| 2R53 | 4-[4-[[4-(3-Hydroxyphenyl)-2-methoxyphenyl]methyl]piperazin-1-yl]-N-[4-(2-phenylsulfanylethylamino)-3-(trifluoromethyl)phenyl]sulfonylbenzamide |
| 2R54 | 4-[4-[[4-(5-Hydroxypyridin-3-yl)-2-methoxyphenyl]methyl]piperazin-1-yl]-N-[4-(2-phenylsulfanylethylamino)-3-(trifluoromethyl)phenyl]sulfonylbenzamide |
| 2R55 | 4-[4-[[3-Ethoxy-5-(3-hydroxyphenyl)phenyl]methyl]piperazin-1-yl]-N-[4-(2-phenylsulfanylethylamino)-3-(trifluoromethyl)phenyl]sulfonylbenzamide |
| 2R56 | 4-[4-[[3-Ethoxy-5-(5-hydroxypyridin-3-yl)phenyl]methyl]piperazin-1-yl]-N-[4-(2-phenylsulfanylethylamino)-3-(trifluoromethyl)phenyl]sulfonylbenzamide |
| 2R57 | 4-[4-[[3-(3-Hydroxyphenyl)-5-propan-2-yloxyphenyl]methyl]piperazin-1-yl]-N-[4-(2-phenylsulfanylethylamino)-3-(trifluoromethyl)phenyl]sulfonylbenzamide |
| 2R58 | 4-[4-[[3-(5-Hydroxypyridin-3-yl)-5-propan-2-yloxyphenyl]methyl]piperazin-1-yl]-N-[4-(2-phenylsulfanylethylamino)-3-(trifluoromethyl)phenyl]sulfonylbenzamide |
| 2R59 | 4-[4-[[4-(3-Hydroxyphenyl)-2,6-dimethoxyphenyl]methyl]piperazin-1-yl]-N-[4-(2-phenylsulfanylethylamino)-3-(trifluoromethyl)phenyl]sulfonylbenzamide |
| 2R60 | 4-[4-[[4-(5-Hydroxypyridin-3-yl)-2,6-dimethoxyphenyl]methyl]piperazin-1-yl]-N-[4-(2-phenylsulfanylethylamino)-3-(trifluoromethyl)phenyl]sulfonylbenzamide |
| 2R61 | 4-[4-[[3-(3-Hydroxyphenyl)-5-(trifluoromethyl)phenyl]methyl]piperazin-1-yl]-N-[4-(2-phenylsulfanylethylamino)-3-(trifluoromethyl)phenyl]sulfonylbenzamide |
| 2R62 | 4-[4-[[3-(5-Hydroxypyridin-3-yl)-5-(trifluoromethyl)phenyl]methyl]piperazin-1-yl]-N-[4-(2-phenylsulfanylethylamino)-3-(trifluoromethyl)phenyl]sulfonylbenzamide |
| 2R63 | 4-[4-[[3-(3-Hydroxyphenyl)-5-(trifluoromethoxy)phenyl]methyl]piperazin-1-yl]-N-[4-(2-phenylsulfanylethylamino)-3-(trifluoromethyl)phenyl]sulfonylbenzamide |
| 2R64 | 4-[4-[[3-(5-Hydroxypyridin-3-yl)-5-(trifluoromethoxy)phenyl]methyl]piperazin-1-yl]-N-[4-(2-phenylsulfanylethylamino)-3-(trifluoromethyl)phenyl]sulfonylbenzamide |
| 2R65 | 4-[4-[[4-(3-Hydroxyphenyl)naphthalen-1-yl]methyl]piperazin-1-yl]-N-[4-(2-phenylsulfanylethylamino)-3-(trifluoromethyl)phenyl]sulfonylbenzamide |
| 2R66 | 4-[4-[[4-(5-Hydroxypyridin-3-yl)naphthalen-1-yl]methyl]piperazin-1-yl]-N-[4-(2-phenylsulfanylethylamino)-3-(trifluoromethyl)phenyl]sulfonylbenzamide |
| 2R67 | 4-[4-[[4-(3-Hydroxyphenyl)thiophen-2-yl]methyl]piperazin-1-yl]-N-[4-(2-phenylsulfanylethylamino)-3-(trifluoromethyl)phenyl]sulfonylbenzamide |
| 2R68 | 4-[4-[[4-(5-Hydroxypyridin-3-yl)thiophen-2-yl]methyl]piperazin-1-yl]-N-[4-(2-phenylsulfanylethylamino)-3-(trifluoromethyl)phenyl]sulfonylbenzamide |
| 2R69 | 4-[4-[[2-[2-(3-Hydroxyphenyl)ethynyl]phenyl]methyl]piperazin-1-yl]-N-[4-(2-phenylsulfanylethylamino)-3-(trifluoromethyl)phenyl]sulfonylbenzamide |
| 2R70 | 4-[4-[[2-[2-(5-Hydroxypyridin-3-yl)ethynyl]phenyl]methyl]piperazin-1-yl]-N-[4-(2-phenylsulfanylethylamino)-3-(trifluoromethyl)phenyl]sulfonylbenzamide |
| 2R71 | 6-[4-[[2-(3-Hydroxyphenyl)phenyl]methyl]piperazin-1-yl]-N-[4-(2-phenylsulfanylethylamino)-3-(trifluoromethyl)phenyl]sulfonylpyridazine-3-carboxamide |

| | |
|---|---|
| 2R72 | 6-[4-[[2-(5-Hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| 2R73 | 6-[4-[[4-(3-Hydroxyphenyl)-3-methylphenyl]methyl]piperazin-1-yl]-N-[4-(2-phenylsulfanylethylamino)-3-(trifluoromethyl)phenyl]sulfonylpyridazine-3-carboxamide |
| 2R74 | 6-[4-[[4-(5-Hydroxypyridin-3-yl)-3-methylphenyl]methyl]piperazin-1-yl]-N-[4-(2-phenylsulfanylethylamino)-3-(trifluoromethyl)phenyl]sulfonylpyridazine-3-carboxamide |
| 2R75 | 6-[4-[[2-Ethyl-4-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| 2R76 | 6-[4-[[2-Ethyl-4-(5-hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| 2R77 | 6-[4-[[3-Fluoro-4-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| 2R78 | 6-[4-[[3-Fluoro-4-(5-hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| 2R79 | 6-[4-[[4-(3-Hydroxyphenyl)-2-methoxyphenyl]methyl]piperazin-1-yl]-N-[4-(2-phenylsulfanylethylamino)-3-(trifluoromethyl)phenyl]sulfonylpyridazine-3-carboxamide |
| 2R80 | 6-[4-[[4-(5-Hydroxypyridin-3-yl)-2-methoxyphenyl]methyl]piperazin-1-yl]-N-[4-(2-phenylsulfanylethylamino)-3-(trifluoromethyl)phenyl]sulfonylpyridazine-3-carboxamide |
| 2R81 | 6-[4-[[3-Ethoxy-5-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| 2R82 | 6-[4-[[3-Ethoxy-5-(5-hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| 2R83 | 6-[4-[[3-(3-Hydroxyphenyl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| 2R84 | 6-[4-[[3-(5-Hydroxypyridin-3-yl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| 2R85 | 6-[4-[[4-(3-Hydroxyphenyl)-2,6-dimethoxyphenyl]methyl]piperazin-1-yl]-N-[4-(2-phenylsulfanylethylamino)-3-(trifluoromethyl)phenyl]sulfonylpyridazine-3-carboxamide |
| 2R86 | 6-[4-[[4-(5-Hydroxypyridin-3-yl)-2,6-dimethoxyphenyl]methyl]piperazin-1-yl]-N-[4-(2-phenylsulfanylethylamino)-3-(trifluoromethyl)phenyl]sulfonylpyridazine-3-carboxamide |
| 2R87 | 6-[4-[[3-(3-Hydroxyphenyl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| 2R88 | 6-[4-[[3-(5-Hydroxypyridin-3-yl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| 2R89 | 6-[4-[[3-(3-Hydroxyphenyl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| 2R90 | 6-[4-[[3-(5-Hydroxypyridin-3-yl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |

| | |
|---|---|
| 2R91 | 6-[4-[[4-(3-Hydroxyphenyl)naphthalen-1-yl]methyl]piperazin-1-yl]-N-[4-(2-phenylsulfanylethylamino)-3-(trifluoromethyl)phenyl]sulfonylpyridazine-3-carboxamide |
| 2R92 | 6-[4-[[4-(5-Hydroxypyridin-3-yl)naphthalen-1-yl]methyl]piperazin-1-yl]-N-[4-(2-phenylsulfanylethylamino)-3-(trifluoromethyl)phenyl]sulfonylpyridazine-3-carboxamide |
| 2R93 | 6-[4-[[4-(3-Hydroxyphenyl)thiophen-2-yl]methyl]piperazin-1-yl]-N-[4-(2-phenylsulfanylethylamino)-3-(trifluoromethyl)phenyl]sulfonylpyridazine-3-carboxamide |
| 2R94 | 6-[4-[[4-(5-Hydroxypyridin-3-yl)thiophen-2-yl]methyl]piperazin-1-yl]-N-[4-(2-phenylsulfanylethylamino)-3-(trifluoromethyl)phenyl]sulfonylpyridazine-3-carboxamide |
| 2R95 | 6-[4-[[2-[2-(3-Hydroxyphenyl)ethynyl]phenyl]methyl]piperazin-1-yl]-N-[4-(2-phenylsulfanylethylamino)-3-(trifluoromethyl)phenyl]sulfonylpyridazine-3-carboxamide |
| 2R96 | 6-[4-[[2-[2-(5-Hydroxypyridin-3-yl)ethynyl]phenyl]methyl]piperazin-1-yl]-N-[4-(2-phenylsulfanylethylamino)-3-(trifluoromethyl)phenyl]sulfonylpyridazine-3-carboxamide |
| 2R97 | 4-[4-[1-[4-(3-Hydroxyphenyl)phenyl]ethyl]piperazin-1-yl]-N-[4-(2-phenylsulfanylethylamino)-3-(trifluoromethyl)phenyl]sulfonylbenzamide |
| 2R98 | 4-[4-[1-[4-(5-Hydroxypyridin-3-yl)phenyl]ethyl]piperazin-1-yl]-N-[4-(2-phenylsulfanylethylamino)-3-(trifluoromethyl)phenyl]sulfonylbenzamide |
| 2R99 | 4-[4-[1-[2-Fluoro-4-(3-hydroxyphenyl)phenyl]ethyl]piperazin-1-yl]-N-[4-(2-phenylsulfanylethylamino)-3-(trifluoromethyl)phenyl]sulfonylbenzamide |
| 2R100 | 4-[4-[1-[2-Fluoro-4-(5-hydroxypyridin-3-yl)phenyl]ethyl]piperazin-1-yl]-N-[4-(2-phenylsulfanylethylamino)-3-(trifluoromethyl)phenyl]sulfonylbenzamide |

Table EJ02 to Table EJ12 describe the retention times (min) of observed UV peaks, $(M+H)^+$ observed in the UV peaks, the retention times of MS peaks in MS chromatograms, and the numbers of compounds assigned to the UV peaks and the MS peaks. From the possibility that two compounds were lacked in Example 4-2-2 and two compounds were lacked in Example 4-2-3, there is a possibility that 44 compounds induced from these four compounds were lacked among 1100 compounds shown in Table EIJ02 to Table EIJ12. Although m/z of the MS peaks was confirmed down to three decimal places, 0 at the decimal level may be omitted in the description in Table EJ02 to Table EJ12. For example, "587.000" is also referred to as "587", "587.100" is also referred to as "587.1", and "587.120" is also referred to as "587.12". Although the retention times of the MS peaks were confirmed down to three decimal places and the retention times of the UV peaks were confirmed down to two decimal places, 0 at the decimal level may be omitted in the description in Table EJ02 to Table EJ12. For example, "15.000" is also referred to as "15", "15.100" is also referred to as "15.1", and "15.120" is also referred to as "15.12".

[Table 227]

| [Table EJ02] | | | | | | | | |
|---|---|---|---|---|---|---|---|---|
| Mixture No. | mixEJ02 | | | | | | | |
| mII [M+H]+ | Retention time of MS peak (min) | Retention time of UV peak (min) | No. of assigned compound | | mII [M+H]+ | Retention time of MS peak (min) | Retention time of UV peak (min) | No. of assigned compound |
| 439.191 | 3.66 | 3.624 | 1M94 | | 490.27 | 6.277 | 6.247 | 1M59, 1M83 |
| 437.201 | 3.76 | 3.716 | 1M68 | | 454.249 | 6.28 | 6.247 | 1M69 |
| 451.18 | 3.88 | 3.847 | 1M24, 1M78 | | 458.244 | 6.303 | 6.247 | 1M25, 1M49 |

(continued)

| [Table EJ02] | | | | | | | | |
|---|---|---|---|---|---|---|---|---|
| Mixture No. | mixEJ02 | | | | | | | |
| mII [M+H]+ | Retention time of MS peak (min) | Retention time of UV peak (min) | No. of assigned compound | | mII [M+H]+ | Retention time of MS peak (min) | Retention time of UV peak (min) | No. of assigned compound |
| 445.26 | 3.917 | 3.872 | 1M48, 1M98 | | 482.255 | 6.343 | 6.313 | 1M91 |
| 463.214 | 3.967 | 3.912 | 1M100, 1M28, 1M80 | | 474.25 | 6.37 | 6.313 | 1M11, 1M55 |
| 449.235 | 3.98 | 3.912 | 1M52 | | 457.235 | 6.433 | 6.393 | 1M96 |
| 433.235 | 4.083 | 4.045 | 1M72 | | 481.26 | 6.463 | 6.393 | 1M66 |
| 461.23 | 4.107 | 4.045 | 1M04, 1M54, 1M76 | | 480.265 | 6.473 | 6.435 | 1M65 |
| 431.244 | 4.17 | 4.133 | 1M46 | | 490.27 | 6.513 | 6.473 | 1M59, 1M83 |
| 459.239 | 4.347 | 4.323 | 1M26, 1M50 | | 500.291 | 6.67 | 6.633 | 1M37, 1M87 |
| 493.256 | 4.357 | 4.323 | 1M86 | | 515.201 | 6.673 | 6.633 | 1M18, 1M64 |
| 483.25 | 4.463 | 4.433 | 1M92 | | 488.291 | 6.68 | 6.633 | 1M57 |
| 477.261 | 4.487 | 4.433 | 1M82 | | 498.236 | 6.75 | 6.708 | 1M61 |
| 491.265 | 4.55 | 4.509 | 1M60, 1M84 | | 503.277 | 6.837 | 6.808 | 1M16 |
| 475.245 | 4.643 | 4.599 | 1M12, 1M56 | | 516.222 | 6.837 | 6.808 | 1M89 |
| 491.265 | 4.807 | 4.777 | 1M60, 1M84 | | 581.223 | 6.88 | 6.825 | 1M22 |
| 489.286 | 4.96 | 4.888 | 1M58 | | 514.206 | 6.923 | 6.88 | 1M17, 1M63 |
| 501.286 | 5.09 | 5.051 | 1M38, 1M88 | | 513.211 | 7.07 | 7.024 | 1M40 |
| 453.17 | 5.093 | 5.051 | 1M02 | | 501.286 | 7.157 | 7.121 | 1M38, 1M88 |
| 461.23 | 5.127 | 5.051 | 1M04, 1M54, 1M76 | | 579.233 | 7.223 | 7.184 | 1M44 |
| 461.23 | 5.147 | 5.109 | 1M04, 1M54, 1M76 | | 445.26 | 7.293 | 7.229 | 1M48, 1M98 |
| 499.232 | 5.193 | 5.152 | 1M62 | | 469.223 | 7.447 | 7.44 | 1M30, 1M31 |
| 455.244 | 5.23 | 5.189 | 1M70 | | 471.214 | 7.477 | 7.44 | 1M08, 1M09 |

(continued)

| [Table EJ02] | | | | | | | | |
|---|---|---|---|---|---|---|---|---|
| Mixture No. | mixEJ02 | | | | | | | |
| mII [M+H]+ | Retention time of MS peak (min) | Retention time of UV peak (min) | No. of assigned compound | | mII [M+H]+ | Retention time of MS peak (min) | Retention time of UV peak (min) | No. of assigned compound |
| 517.217 | 5.27 | 5.231 | 1M90 | | 452.175 | 7.697 | 7.653 | 1M01 |
| 465.204 | 5.297 | 5.261 | 1M06 | | 463.214 | 7.867 | 7.844 | 1M100, 1M28, 1M80 |
| 515.201 | 5.367 | 5.335 | 1M18, 1M64 | | 464.209 | 7.88 | 7.844 | 1M05 |
| 438.196 | 5.403 | 5.368 | 1M93 | | 470.219 | 7.887 | 7.844 | 1M07, 1M32 |
| 432.239 | 5.477 | 5.447 | 1M71 | | 469.223 | 7.89 | 7.844 | 1M30, 1M31 |
| 451.18 | 5.5 | 5.447 | 1M24, 1M78 | | 460.234 | 8.04 | 8.003 | 1M03, 1M53, 1M75 |
| 459.239 | 5.513 | 5.467 | 1M26, 1M50 | | 450.185 | 8.157 | 8.127 | 1M23, 1M77 |
| 436.205 | 5.567 | 5.525 | 1M67 | | 470.219 | 8.21 | 8.127 | 1M07, 1M32 |
| 450.185 | 5.623 | 5.587 | 1M23, 1M77 | | 539.201 | 8.247 | 8.207 | 1M20 |
| 475.245 | 5.657 | 5.619 | 1M12, 1M56 | | 462.219 | 8.283 | 8.251 | 1M27, 1M79, 1M99 |
| 430.249 | 5.66 | 5.619 | 1M45 | | 463.214 | 8.303 | 8.251 | 1M100, 1M28, 1M80 |
| 462.219 | 5.73 | 5.668 | 1M27, 1M79, 1M99 | | 458.244 | 8.447 | 8.407 | 1M25, 1M49 |
| 447.25 | 5.76 | 5.721 | 1M74 | | 474.25 | 8.473 | 8.436 | 1M11, 1M55 |
| 446.255 | 5.76 | 5.721 | 1M73 | | 468.228 | 8.583 | 8.52 | 1M29 |
| 497.23 | 5.763 | 5.721 | 1M14 | | 496.234 | 8.6 | 8.567 | 1M13 |
| 448.239 | 5.783 | 5.733 | 1M51 | | 537.211 | 8.617 | 8.567 | 1M42 |
| 472.209 | 5.843 | 5.801 | 1M10, 1M33 | | 471.214 | 8.813 | 8.792 | 1M08, 1M09 |
| 444.265 | 5.923 | 5.905 | 1M47, 1M97 | | 472.209 | 8.84 | 8.792 | 1M10, 1M33 |
| 460.234 | 5.933 | 5.905 | 1M03, 1M53, 1M75 | | 494.244 | 8.93 | 8.891 | 1M35 |

(continued)

| [Table EJ02] | | | | | | | | |
|---|---|---|---|---|---|---|---|---|
| Mixture No. | mixEJ02 | | | | | | | |
| mII [M+H]+ | Retention time of MS peak (min) | Retention time of UV peak (min) | No. of assigned compound | | mII [M+H]+ | Retention time of MS peak (min) | Retention time of UV peak (min) | No. of assigned compound |
| 444.265 | 5.943 | 5.905 | 1M47, 1M97 | | 514.206 | 9.08 | 9.047 | 1M17, 1M63 |
| 462.219 | 5.967 | 5.905 | 1M27, 1M79, 1M99 | | 512.216 | 9.413 | 9.368 | 1M39 |
| 473.255 | 6.01 | 5.972 | 1M34 | | 580.228 | 9.49 | 9.457 | 1M21 |
| 492.261 | 6.06 | 6.023 | 1M85 | | 502.281 | 9.71 | 9.676 | 1M15 |
| 495.239 | 6.113 | 6.076 | 1M36 | | 578.237 | 9.81 | 9.764 | 1M43 |
| 460.234 | 6.16 | 6.076 | 1M03, 1M53, 1M75 | | 538.206 | 9.863 | 9.824 | 1M19 |
| 456.239 | 6.187 | 6.149 | 1M95 | | 500.291 | 9.99 | 9.956 | 1M37, 1M87 |
| 476.266 | 6.197 | 6.149 | 1M81 | | 536.216 | 10.157 | 10.116 | 1M41 |

[Table 228]

| [Table EJ03] | | | | | | | | |
|---|---|---|---|---|---|---|---|---|
| Mixture No. | mixEJ03 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 479.147 | 3.777 | 3.73 | 1K94 | | 498.2 | 6.57 | 6.54 | 1K25, 1K49 |
| 487.206 | 3.887 | 3.85 | 1K74 | | 522.211 | 6.577 | 6.54 | 1K91 |
| 477.157 | 3.93 | 3.89 | 1K68 | | 514.206 | 6.613 | 6.57 | 1K11, 1K55 |
| 491.136 | 4.01 | 3.98 | 1K24, 1K78 | | 510.175 | 6.653 | 6.62 | 1K07, 1K32 |
| 503.17 | 4.063 | 4.03 | 1K100, 1K28, 1K80 | | 530.226 | 6.717 | 6.69 | 1K59, 1K83 |
| 485.216 | 4.087 | 4.03 | 1K48, 1K98 | | 520.221 | 6.737 | 6.69 | 1K65 |
| 473.191 | 4.23 | 4.2 | 1K72 | | 528.247 | 6.907 | 6.87 | 1K57 |
| 501.186 | 4.297 | 4.26 | 1K04, 1K54, 1K76 | | 540.247 | 6.907 | 6.87 | 1K37, 1K87 |

(continued)

[Table EJ03]

| Mixture No. | mixEJ03 | | | | | | | |
|---|---|---|---|---|---|---|---|---|
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 471.2 | 4.387 | 4.36 | 1K46 | | 555.157 | 6.987 | 6.94 | 1K18, 1K64 |
| 501.186 | 4.42 | 4.36 | 1K04, 1K54, 1K76 | | 538.192 | 7.02 | 6.98 | 1K61 |
| 533.212 | 4.49 | 4.46 | 1K86 | | 556.178 | 7.077 | 7.07 | 1K89 |
| 523.206 | 4.633 | 4.6 | 1K92 | | 484.221 | 7.087 | 7.07 | 1K47, 1K97 |
| 517.217 | 4.637 | 4.6 | 1K82 | | 543.233 | 7.103 | 7.07 | 1K16 |
| 531.221 | 4.737 | 4.71 | 1K60, 1K84 | | 557.173 | 7.107 | 7.07 | 1K90 |
| 521.216 | 4.817 | 4.77 | 1K66 | | 554.162 | 7.187 | 7.15 | 1K17, 1K63 |
| 515.201 | 4.867 | 4.82 | 1K12, 1K56 | | 553.167 | 7.43 | 7.4 | 1K40 |
| 531.221 | 4.99 | 4.95 | 1K60, 1K84 | | 541.242 | 7.487 | 7.45 | 1K38, 1K88 |
| 529.242 | 5.2 | 5.16 | 1K58 | | 619.189 | 7.533 | 7.5 | 1K44 |
| 541.242 | 5.3 | 5.26 | 1K38, 1K88 | | 509.18 | 7.783 | 7.7 | 1K30, 1K31 |
| 493.126 | 5.347 | 5.3 | 1K02 | | 511.17 | 7.81 | 7.77 | 1K08, 1K09 |
| 502.175 | 5.387 | 5.35 | 1K27, 1K79, 1K99 | | 492.131 | 8.05 | 8.01 | 1K01 |
| 501.186 | 5.39 | 5.35 | 1K04, 1K54, 1K76 | | 504.165 | 8.203 | 8.17 | 1K05 |
| 539.188 | 5.46 | 5.42 | 1K62 | | 503.17 | 8.213 | 8.17 | 1K100, 1K28, 1K80 |
| 505.161 | 5.567 | 5.53 | 1K06 | | 510.175 | 8.25 | 8.22 | 1K07, 1K32 |
| 555.157 | 5.647 | 5.6 | 1K18, 1K64 | | 509.18 | 8.253 | 8.22 | 1K30, 1K31 |
| 472.195 | 5.67 | 5.64 | 1K71 | | 511.17 | 8.27 | 8.22 | 1K08, 1K09 |
| 499.195 | 5.82 | 5.79 | 1K26, 1K50 | | 499.195 | 8.36 | 8.33 | 1K26, 1K50 |

(continued)

[Table EJ03]

| Mixture No. | mixEJ03 | | | | | | | |
|---|---|---|---|---|---|---|---|---|
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 476.161 | 5.823 | 5.79 | 1K67 | | 500.19 | 8.363 | 8.33 | 1K03, 1K53, 1K75 |
| 490.141 | 5.837 | 5.79 | 1K23, 1K77 | | 579.157 | 8.533 | 8.51 | 1K20 |
| 491.136 | 5.847 | 5.79 | 1K24, 1K78 | | 490.141 | 8.55 | 8.51 | 1K23, 1K77 |
| 470.205 | 5.903 | 5.88 | 1K45 | | 502.175 | 8.667 | 8.63 | 1K27, 1K79, 1K99 |
| 515.201 | 5.91 | 5.88 | 1K12, 1K56 | | 495.2 | 8.753 | 8.63 | 1K70 |
| 502.175 | 5.923 | 5.88 | 1K27, 1K79, 1K99 | | 514.206 | 8.817 | 8.78 | 1K11, 1K55 |
| 486.211 | 5.96 | 5.92 | 1K73 | | 498.2 | 8.823 | 8.78 | 1K25, 1K49 |
| 537.186 | 6.027 | 5.99 | 1K14 | | 536.19 | 8.89 | 8.85 | 1K13 |
| 488.196 | 6.043 | 5.99 | 1K51 | | 508.184 | 8.937 | 8.85 | 1K29 |
| 503.17 | 6.053 | 6.01 | 1K100, 1K28, 1K80 | | 512.165 | 9.213 | 9.18 | 1K10, 1K33 |
| 489.191 | 6.053 | 6.01 | 1K52 | | 512.165 | 9.24 | 9.18 | 1K10, 1K33 |
| 500.19 | 6.187 | 6.15 | 1K03, 1K53, 1K75 | | 534.2 | 9.29 | 9.25 | 1K35 |
| 484.221 | 6.2 | 6.15 | 1K47, 1K97 | | 554.162 | 9.37 | 9.32 | 1K17, 1K63 |
| 485.216 | 6.207 | 6.15 | 1K48, 1K98 | | 620.184 | 9.733 | 9.7 | 1K21 |
| 532.217 | 6.247 | 6.21 | 1K85 | | 621.179 | 9.733 | 9.7 | 1K22 |
| 513.211 | 6.327 | 6.29 | 1K34 | | 552.172 | 9.74 | 9.7 | 1K39 |
| 497.191 | 6.367 | 6.34 | 1K96 | | 542.237 | 9.96 | 9.92 | 1K15 |
| 500.19 | 6.373 | 6.34 | 1K03, 1K53, 1K75 | | 578.162 | 10.08 | 10.07 | 1K19 |
| 496.195 | 6.38 | 6.34 | 1K95 | | 618.193 | 10.11 | 10.07 | 1K43 |
| 516.222 | 6.4 | 6.34 | 1K81 | | 540.247 | 10.313 | 10.28 | 1K37, 1K87 |

(continued)

| [Table EJ03] | | | | | | | | |
|---|---|---|---|---|---|---|---|---|
| Mixture No. | mixEJ03 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 535.195 | 6.443 | 6.41 | 1K36 | | 577.167 | 10.44 | 10.41 | 1K42 |
| 494.205 | 6.517 | 6.48 | 1K69 | | 576.172 | 10.457 | 10.41 | 1K41 |
| 530.226 | 6.517 | 6.48 | 1K59, 1K83 | | 478.152 | 14.887 | 13.56 | 1K93 |

[Table 229]

| [Table EJ04] | | | | | | | | |
|---|---|---|---|---|---|---|---|---|
| Mixture No. | mixEJ04 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 503.186 | 4.597 | 4.58 | 1O94 | | 538.245 | 7.05 | 7.02 | 1O11, 1O55 |
| 501.195 | 4.62 | 4.58 | 1O68 | | 536.204 | 7.063 | 7.02 | 1O10, 1033 |
| 511.245 | 4.67 | 4.63 | 1O74 | | 546.250 | 7.173 | 7.15 | 1O91 |
| 509.255 | 4.76 | 4.71 | 1O48, 1O98 | | 544.259 | 7.187 | 7.15 | 1O65 |
| 527.209 | 4.82 | 4.78 | 1O100, 1O28, 1080 | | 547.245 | 7.197 | 7.15 | 1O92 |
| 515.175 | 4.847 | 4.81 | 1O24, 1O78 | | 554.265 | 7.247 | 7.22 | 1O59, 1O83 |
| 525.224 | 4.927 | 4.88 | 1O04, 1O54, 1O76 | | 552.286 | 7.33 | 7.29 | 1O57 |
| 495.239 | 5.05 | 5.01 | 1O46 | | 534.214 | 7.35 | 7.29 | 1O07, 1O32 |
| 509.255 | 5.07 | 5.01 | 1O48, 1O98 | | 562.231 | 7.45 | 7.41 | 1O61 |
| 523.234 | 5.187 | 5.15 | 1O26, 1O50 | | 565.281 | 7.507 | 7.49 | 1O38, 1O88 |
| 557.251 | 5.193 | 5.15 | 1O86 | | 564.286 | 7.527 | 7.49 | 1O37, 1O87 |
| 555.260 | 5.313 | 5.28 | 1O60, 1O84 | | 578.201 | 7.61 | 7.56 | 1O17, 1O63 |
| 541.256 | 5.367 | 5.31 | 1O82 | | 580.217 | 7.683 | 7.65 | 1O89 |
| 545.255 | 5.4 | 5.36 | 1O66 | | 579.196 | 7.827 | 7.83 | 1O18, 1O64 |
| 539.240 | 5.44 | 5.40 | 1O12, 1O56 | | 645.218 | 7.85 | 7.83 | 1O22 |
| 555.260 | 5.67 | 5.63 | 1O60, 1O84 | | 567.271 | 7.877 | 7.83 | 1O16 |
| 553.281 | 5.75 | 5.71 | 1O58 | | 565.281 | 8.053 | 8.03 | 1O38, 1O88 |
| 563.226 | 6.037 | 6.01 | 1O62 | | 577.206 | 8.057 | 8.03 | 1O40 |
| 579.196 | 6.203 | 6.18 | 1O18, 1O64 | | 643.228 | 8.077 | 8.03 | 1O44 |
| 581.212 | 6.213 | 6.18 | 1O90 | | 515.175 | 8.43 | 8.28 | 1O24, 1O78 |

| | | | | | | | | |
|---|---|---|---|---|---|---|---|---|
| 517.165 | 6.227 | 6.18 | 1O02 | | 497.230 | 8.443 | 8.28 | 1O72 |
| 525.224 | 6.26 | 6.22 | 1O04, 1O54, 1O76 | | 535.209 | 8.61 | 8.57 | 1O08, 1O09 |
| 502.191 | 6.333 | 6.30 | 1O93 | | 535.209 | 8.637 | 8.57 | 1O08, 1O09 |
| 496.234 | 6.347 | 6.30 | 1O71 | | 533.218 | 8.843 | 8.80 | 1O30, 1O31 |
| 500.200 | 6.37 | 6.30 | 1O67 | | 516.170 | 8.893 | 8.86 | 1O01 |
| 494.244 | 6.427 | 6.39 | 1O45 | | 529.199 | 9.033 | 9.00 | 1O06 |
| 523.234 | 6.503 | 6.46 | 1O26, 1O50 | | 528.204 | 9.037 | 9.00 | 1O05 |
| 514.180 | 6.507 | 6.46 | 1O23, 1O77 | | 514.180 | 9.153 | 9.11 | 1O23, 1O77 |
| 526.214 | 6.543 | 6.52 | 1O27,1O79,1O99 | | 524.229 | 9.207 | 9.16 | 1O03, 1O53, 1O75 |
| 513.230 | 6.553 | 6.52 | 1O52 | | 527.209 | 9.247 | 9.21 | 1O100, 1O28, 1O80 |

(continued)

[Table EJ04]

| Mixture No. | mixEJ04 | | | | | | | |
|---|---|---|---|---|---|---|---|---|
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 534.214 | 6.56 | 6.52 | 1O07,1O32 | | 526.214 | 9.247 | 9.21 | 1O27, 1O79, 1O99 |
| 508.259 | 6.56 | 6.52 | 1O47, 1O97 | | 603.196 | 9.32 | 9.28 | 1O20 |
| 510.250 | 6.567 | 6.52 | 1O73 | | 522.239 | 9.403 | 9.37 | 1O25, 1O49 |
| 512.234 | 6.59 | 6.52 | 1O51 | | 601.206 | 9.513 | 9.48 | 1O42 |
| 524.229 | 6.667 | 6.64 | 1O03, 1O53, 1O75 | | 533.218 | 9.527 | 9.48 | 1O30,1O31 |
| 508.259 | 6.677 | 6.64 | 1O47, 1O97 | | 532.223 | 9.537 | 9.48 | 1O29 |
| 539.240 | 6.703 | 6.64 | 1O12, 1O56 | | 538.245 | 9.597 | 9.56 | 1O11,1055 |
| 526.214 | 6.713 | 6.64 | 1O27, 1O79, 1099 | | 560.229 | 9.7 | 9.67 | 1O13 |
| 527.209 | 6.783 | 6.75 | 1O100, 1O28, 1080 | | 536.204 | 9.767 | 9.73 | 1O10, 1O33 |
| 556.255 | 6.827 | 6.79 | 1O85 | | 558.239 | 9.85 | 9.81 | 1O35 |
| 561.224 | 6.877 | 6.85 | 1O14 | | 559.234 | 9.853 | 9.81 | 1O36 |
| 537.250 | 6.937 | 6.85 | 1O34 | | 578.201 | 10.127 | 10.08 | 1O17, 1O63 |
| 554.265 | 6.957 | 6.94 | 1O59, 1O83 | | 576.210 | 10.26 | 10.23 | 1O39 |
| 540.261 | 6.96 | 6.94 | 1O81 | | 644.223 | 10.443 | 10.40 | 1O21 |
| 520.234 | 6.967 | 6.94 | 1O95 | | 519.239 | 10.587 | 10.55 | 1O70 |
| 518.244 | 6.977 | 6.94 | 1O69 | | 642.232 | 10.6 | 10.55 | 1O43 |
| 525.224 | 6.983 | 6.94 | 1O04, 1O54, 1O76 | | 566.276 | 10.737 | 10.70 | 1O15 |
| 521.230 | 6.983 | 6.94 | 1O96 | | 602.201 | 10.79 | 10.76 | 1O19 |
| 524.229 | 6.993 | 6.94 | 1O03, 1O53, 1O75 | | 564.286 | 10.837 | 10.80 | 1O37, 1 O87 |
| 522.239 | 7.02 | 6.94 | 1O25,1O49 | | 600.210 | 10.93 | 10.89 | 1O41 |

[Table 230]

| [Table EJ05] | | | | | | | | |
|---|---|---|---|---|---|---|---|---|
| Mixture No. | mixEJ05 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 551.338 | 5.62 | 5.62 | 1A48, 1A98 | | 581.323 | 8.607 | 8.607 | 1A12,1A56 |
| 599.334 | 8.333 | 8.333 | 1A86 | | 586.343 | 8.623 | 8.623 | 1A65 |
| 597.344 | 8.37 | 8.37 | 1A60, 1A84 | | 568.297 | 11.383 | 11.383 | 1A27, 1A79, 1A99 |
| 596.348 | 8.363 | 8.363 | 1A59,1A83 | | 570.287 | 11.327 | 11.327 | 1A05 |
| 598.339 | 8.32 | 8.32 | 1A85 | | 571.283 | 11.327 | 11.327 | 1A06 |
| 574.306 | 11.69 | 11.69 | 1A29 | | 568.297 | 11.357 | 11.357 | 1A27, 1A79, 1A99 |
| 576.297 | 11.71 | 11.71 | 1A07, 1A32 | | 645.28 | 11.567 | 11.567 | 1A20 |
| 563.313 | 7.57 | 7.57 | 1A96 | | 643.289 | 11.523 | 11.523 | 1A42 |
| 623.295 | 7.907 | 7.907 | 1A90 | | 565.317 | 11.533 | 11.533 | 1A26, 1A50 |
| 542.284 | 7.933 | 7.933 | 1A67 | | 564.322 | 11.55 | 11.55 | 1A25, 1A49 |
| 558.253 | 11.207 | 11.207 | 1A01 | | 589.329 | 7.063 | 7.063 | 1A92 |
| 566.313 | 8.593 | 8.593 | 1A03, 1A53, 1A75 | | 606.369 | 12.933 | 12.933 | 1A37, 1A87 |
| 562.318 | 8.54 | 8.54 | 1A95 | | 642.294 | 12.873 | 12.873 | 1A41 |
| 577.292 | 11.89 | 11.89 | 1A08,1A09 | | 569.292 | 8.88 | 8.88 | 1A100, 1A28, 1A80 |
| 580.328 | 11.883 | 11.883 | 1A11, 1A55 | | 604.315 | 8.873 | 8.873 | 1A61 |
| 578.287 | 11.917 | 11.917 | 1A10,1A33 | | 607.364 | 9.957 | 9.957 | 1A38, 1A88 |
| 581.323 | 11.907 | 11.907 | 1A12,1A56 | | 603.308 | 8.943 | 8.943 | 1A14 |
| 578.287 | 11.89 | 11.89 | 1A10,1A33 | | 619.289 | 10.1 | 10.1 | 1A40 |
| 583.339 | 6.98 | 6.98 | 1A82 | | 545.269 | 6.337 | 6.337 | 1A94 |
| 544.274 | 8.017 | 8.017 | 1A93 | | 543.279 | 6.357 | 6.357 | 1A68 |
| 550.343 | 8.117 | 8.117 | 1A47, 1A97 | | 687.301 | 9.807 | 9.807 | 1A22 |
| 553.329 | 8.123 | 8.123 | 1A74 | | 557.258 | 8.46 | 8.46 | 1A24, 1A78 |
| 557.258 | 8.117 | 8.117 | 1A24, 1A78 | | 565.317 | 8.453 | 8.453 | 1A26, 1A50 |
| 556.263 | 8.123 | 8.123 | 1A23, 1A77 | | 582.344 | 8.46 | 8.46 | 1A81 |
| 550.343 | 8.133 | 8.133 | 1A47, 1A97 | | 564.322 | 8.473 | 8.473 | 1A25, 1A49 |
| 566.313 | 8.127 | 8.127 | 1A03, 1A53, 1A75 | | 594.369 | 8.68 | 8.68 | 1A57 |
| 569.292 | 6,487 | 6.487 | 1A100, 1A28, 1A80 | | 588.333 | 8.823 | 8.823 | 1A91 |
| 552.333 | 6.43 | 6.43 | 1A73 | | 559.249 | 8.237 | 8.237 | 1A02 |

(continued)

[Table EJ05]

| Mixture No. | mixEJ05 | | | | | | | | |
|---|---|---|---|---|---|---|---|---|---|
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 551.338 | 6.427 | 6.427 | 1A48, 1A98 | | 567.308 | 8.273 | 8.273 | 1A04, 1A54, 1A76 |
| 567.308 | 6.59 | 6.59 | 1A04, 1A54, 1A76 | | 609.355 | 9.917 | 9.917 | 1A16 |
| 569.292 | 8.073 | 8.073 | 1A100, 1A28, 1A80 | | 685.311 | 9.89 | 9.89 | 1A44 |
| 568.297 | 8.073 | 8.073 | 1A27, 1A79, 1A99 | | 577.292 | 10.817 | 10.817 | 1A08, 1A09 |
| 554.318 | 8.07 | 8.07 | 1A51 | | 620.284 | 9.01 | 9.01 | 1A17, 1A63 |
| 580.328 | 8.433 | 8.433 | 1A11, 1A55 | | 606.369 | 9.18 | 9.18 | 1A37, 1A87 |
| 560.327 | 8.407 | 8.407 | 1A69 | | 567.308 | 6.823 | 6.823 | 1A04, 1A54, 1A76 |
| 596.348 | 8.727 | 8.727 | 1A59, 1A83 | | 587.338 | 7.013 | 7.013 | 1A66 |
| 575.302 | 10.913 | 10.913 | 1A30, 1A31 | | 538.318 | 7.967 | 7.967 | 1 A71 |
| 576.297 | 10.903 | 10.903 | 1A07, 1A32 | | 536.327 | 7.94 | 7.94 | 1A45 |
| 575.302 | 10.89 | 10.89 | 1A30, 1A31 | | 555.313 | 6.66 | 6.66 | 1A52 |
| 561.322 | 7.543 | 7.543 | 1A70 | | 595.364 | 7.26 | 7.26 | 1A58 |
| 608.36 | 13.08 | 13.08 | 1A15 | | 686.306 | 12.583 | 12.583 | 1A21 |
| 579.333 | 8.787 | 8.787 | 1A34 | | 620.284 | 12.363 | 12.363 | 1A17, 1A63 |
| 597.344 | 6.897 | 6.897 | 1A60, 1A84 | | 601.317 | 9.07 | 9.07 | 1A36 |
| 605.31 | 7.63 | 7.63 | 1A62 | | 602.313 | 12.03 | 12.03 | 1A13 |
| 539.313 | 6.747 | 6.747 | 1A72 | | 622.3 | 9.32 | 9.32 | 1A89 |
| 537.322 | 6.733 | 6.733 | 1A46 | | 556.263 | 11.283 | 11.283 | 1A23, 1A77 |
| 621.28 | 7.763 | 7.763 | 1A18, 1A64 | | 684.316 | 12.527 | 12.527 | 1A43 |
| 607.364 | 7.757 | 7.757 | 1A38, 1A88 | | 644.284 | 12.973 | 12.973 | 1A19 |
| 621.28 | 10.007 | 10.007 | 1A18, 1A64 | | 600.322 | 11.967 | 11.967 | 1A35 |
| 566.313 | 8.613 | 8.613 | 1A03, 1A53, 1A75 | | 618.294 | 12.29 | 12.29 | 1A39 |

[Table 231]

| [Table EJ06] | | | | | | | | |
|---|---|---|---|---|---|---|---|---|
| Mixture No. | mixEJ06 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 559.285 | 6.01 | 5.96 | 3A94 | | 578.338 | 8.843 | 8.8 | 3A25, 3A49 |
| 567.344 | 6.063 | 6.01 | 3A74 | | 579.333 | 8.853 | 8.8 | 3A26, 3A50 |
| 571.274 | 6.3 | 6.25 | 3A24, 3A78 | | 600.358 | 9.013 | 8.99 | 3A65 |
| 553.329 | 6.4 | 6.37 | 3A72 | | 608.385 | 9.02 | 8.99 | 3A57 |
| 613.35 | 6.5 | 6.44 | 3A86 | | 579.333 | 9.04 | 8.99 | 3A26, 3A50 |
| 597.355 | 6.66 | 6.63 | 3A82 | | 571.274 | 9.08 | 8.99 | 3A24, 3A78 |
| 603.344 | 6.677 | 6.63 | 3A92 | | 618.33 | 9.293 | 9.25 | 3A61 |
| 557.294 | 6.713 | 6.67 | 3A68 | | 701.317 | 9.303 | 9.25 | 3A22 |
| 565.354 | 6.767 | 6.73 | 3A48, 3A98 | | 623.37 | 9.343 | 9.25 | 3A16 |
| 565.354 | 6.78 | 6.73 | 3A48, 3A98 | | 593.349 | 9.38 | 9.34 | 3A34 |
| 581.323 | 6.933 | 6.89 | 3A04, 3A54, 3A76 | | 635.295 | 9.407 | 9.34 | 3A18, 3A64 |
| 611.359 | 6.933 | 6.89 | 3A60, 3A84 | | 634.3 | 9.43 | 9.39 | 3A17, 3A63 |
| 551.338 | 7.083 | 7.05 | 3A46 | | 583.308 | 9.55 | 9.51 | 3A100, 3A28, 3A80 |
| 611.359 | 7.25 | 7.21 | 3A60, 3A84 | | 583.308 | 9.573 | 9.51 | 3A100, 3A28, 3A80 |
| 595.339 | 7.34 | 7.3 | 3A12, 3A56 | | 615.333 | 9.757 | 9.72 | 3A36 |
| 621.38 | 7.34 | 7.3 | 3A38, 3A88 | | 590.313 | 10.13 | 10.09 | 3A07, 3A32 |
| 601.354 | 7.393 | 7.35 | 3A66 | | 591.308 | 10.213 | 10.18 | 3A08, 3A09 |
| 637.311 | 7.473 | 7.44 | 3A90 | | 591.308 | 10.237 | 10.18 | 3A08, 3A09 |
| 609.38 | 7.6 | 7.57 | 3A58 | | 564.358 | 10.467 | 10.4 | 3A47, 3A97 |
| 558.29 | 7.613 | 7.57 | 3A93 | | 572.269 | 10.553 | 10.57 | 3A01 |

(continued)

| [Table EJ06] | | | | | | | | |
|---|---|---|---|---|---|---|---|---|
| Mixture No. | mixEJ06 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 552.333 | 7.627 | 7.57 | 3A71 | | 621.38 | 10.707 | 10.68 | 3A38, 3A88 |
| 573.264 | 7.737 | 7.71 | 3A02 | | 584.303 | 10.71 | 10.68 | 3A05 |
| 570.278 | 7.75 | 7.71 | 3A23, 3A77 | | 633.305 | 10.847 | 10.82 | 3A40 |
| 581.323 | 7.76 | 7.71 | 3A04, 3A54, 3A76 | | 580.328 | 10.883 | 10.82 | 3A03, 3A53, 3A75 |
| 582.313 | 7.773 | 7.71 | 3A27, 3A79, 3A99 | | 659.295 | 10.93 | 10.88 | 3A20 |
| 566.349 | 7.8 | 7.71 | 3A73 | | 590.313 | 11.147 | 11.09 | 3A07, 3A32 |
| 575.338 | 7.88 | 7.85 | 3A70 | | 596.36 | 11.293 | 11.25 | 3A81 |
| 619.325 | 8.013 | 7.98 | 3A62 | | 594.344 | 11.303 | 11.25 | 3A11, 3A55 |
| 612.354 | 8.023 | 7.98 | 3A85 | | 616.328 | 11.393 | 11.35 | 3A13 |
| 595.339 | 8.14 | 8.11 | 3A12, 3A56 | | 589.317 | 11.623 | 11.6 | 3A30, 3A31 |
| 635.295 | 8.147 | 8.11 | 3A18, 3A64 | | 634.3 | 11.77 | 11.73 | 3A17, 3A63 |
| 585.298 | 8.157 | 8.11 | 3A06 | | 700.322 | 12.037 | 11.99 | 3A21 |
| 580.328 | 8.197 | 8.11 | 3A03, 3A53, 3A75 | | 589.317 | 12.06 | 11.99 | 3A30, 3A31 |
| 550.343 | 8.293 | 8.26 | 3A45 | | 570.278 | 12.087 | 12.05 | 3A23, 3A77 |
| 556.299 | 8.297 | 8.26 | 3A67 | | 582.313 | 12.153 | 12.11 | 3A27, 3A79, 3A99 |
| 577.329 | 8.32 | 8.26 | 3A96 | | 582.313 | 12.167 | 12.11 | 3A27, 3A79, 3A99 |
| 576.333 | 8.323 | 8.26 | 3A95 | | 583.308 | 12.183 | 12.11 | 3A100, 3A28, 3A80 |
| 602.349 | 8.363 | 8.33 | 3A91 | | 657.305 | 12.347 | 12.34 | 3A42 |
| 610.364 | 8.4 | 8.33 | 3A59, 3A83 | | 578.338 | 12.367 | 12.34 | 3A25, 3A49 |
| 617.323 | 8.413 | 8.37 | 3A14 | | 658.3 | 12.393 | 12.34 | 3A19 |

(continued)

| [Table EJ06] | | | | | | | | |
|---|---|---|---|---|---|---|---|---|
| Mixture No. | mixEJ06 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 569.329 | 8.417 | 8.37 | 3A52 | | 622.375 | 12.45 | 12.34 | 3A15 |
| 568.333 | 8.42 | 8.37 | 3A51 | | 588.322 | 12.557 | 12.52 | 3A29 |
| 581.323 | 8.45 | 8.42 | 3A04, 3A54, 3A76 | | 592.303 | 12.703 | 12.67 | 3A10, 3A33 |
| 580.328 | 8.46 | 8.42 | 3A03, 3A53, 3A75 | | 592.303 | 12.737 | 12.67 | 3A10, 3A33 |
| 564.358 | 8.473 | 8.42 | 3A47, 3A97 | | 614.338 | 12.833 | 12.79 | 3A35 |
| 620.385 | 8.69 | 8.67 | 3A37, 3A87 | | 632.309 | 13.12 | 13.08 | 3A39 |
| 610.364 | 8.72 | 8.67 | 3A59, 3A83 | | 698.331 | 13.33 | 13.29 | 3A43 |
| 594.344 | 8.77 | 8.75 | 3A11, 3A55 | | 699.327 | 13.337 | 13.29 | 3A44 |
| 574.343 | 8.79 | 8.75 | 3A69 | | 656.309 | 13.693 | 13.66 | 3A41 |
| 636.316 | 8.833 | 8.8 | 3A89 | | 620.385 | 13.84 | 13.79 | 3A37, 3A87 |

[Table 232]

| [Table EJ07] | | | | | | | | |
|---|---|---|---|---|---|---|---|---|
| Mixture No. | mixEJ07 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 475.122 | 2.943 | 2.9 | 2I94 | | 526.201 | 5.99 | 5.96 | 2I59, 2I83 |
| 483.181 | 3.043 | 3 | 2I74 | | 511.176 | 5.99 | 5.96 | 2I12, 2156 |
| 481.19 | 3.423 | 3.36 | 2I48, 2I98 | | 539.207 | 6.087 | 6.05 | 2I16 |
| 469.165 | 3.44 | 3.4 | 2I72 | | 516.195 | 6.117 | 6.05 | 2I65 |
| 485.165 | 3.5 | 3.46 | 2I52 | | 617.154 | 6.14 | 6.11 | 2I22 |
| 497.16 | 3.61 | 3.57 | 2I04, 2I54, 2I76 | | 536.221 | 6.157 | 6.11 | 2I37, 2I87 |
| 467.175 | 3.743 | 3.7 | 2I46 | | 524.221 | 6.327 | 6.31 | 2I57 |
| 529.186 | 3.743 | 3.7 | 2I86 | | 552.152 | 6.35 | 6.31 | 2I89 |
| 513.191 | 3.817 | 3.79 | 2I82 | | 534.167 | 6.42 | 6.39 | 2I61 |

(continued)

| [Table EJ07] | | | | | | | | |
|---|---|---|---|---|---|---|---|---|
| Mixture No. | mixEJ07 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 495.17 | 3.937 | 3.9 | 2I26, 2I50 | | 549.141 | 6.51 | 6.47 | 2I40 |
| 527.196 | 4.147 | 4.12 | 2I60, 2I84 | | 551.132 | 6.6 | 6.57 | 2I18, 2I64 |
| 517.19 | 4.15 | 4.12 | 2I66 | | 473.131 | 6.603 | 6.57 | 2I68 |
| 489.101 | 4.307 | 4.27 | 2I02 | | 550.137 | 6.607 | 6.57 | 2I17, 2I63 |
| 497.16 | 4.353 | 4.32 | 2I04, 2I54, 2I76 | | 487.11 | 6.66 | 6.63 | 2I24, 2I78 |
| 537.217 | 4.41 | 4.38 | 2I38, 2I88 | | 537.217 | 6.667 | 6.63 | 2I38, 2I66 |
| 501.135 | 4.453 | 4.41 | 2I06 | | 507.145 | 6.667 | 6.63 | 2I08, 2I09 |
| 525.217 | 4.53 | 4.5 | 2I58 | | 615.163 | 6.743 | 6.7 | 2I44 |
| 553.148 | 4.61 | 4.58 | 2I90 | | 488.106 | 6.83 | 6.79 | 2I01 |
| 535.162 | 4.757 | 4.69 | 2I62 | | 500.14 | 7.007 | 6.97 | 2I05 |
| 468.17 | 4.847 | 4.81 | 2I71 | | 496.165 | 7.16 | 7.13 | 2I03, 2I53, 2I75 |
| 474.126 | 4.92 | 4.92 | 2I93 | | 497.16 | 7,167 | 7.13 | 2I04, 2I54, 2I76 |
| 533.16 | 4.95 | 4.92 | 2I14 | | 496.165 | 7,187 | 7.13 | 2I03, 2I53, 2I75 |
| 511.176 | 4.96 | 4.92 | 2I12, 2I56 | | 507.145 | 7.32 | 7.29 | 2I08, 2I09 |
| 495.17 | 4.97 | 4.92 | 2I26, 2I50 | | 505.154 | 7.327 | 7.29 | 2I30, 2131 |
| 486.115 | 4.983 | 4.92 | 2I23, 2I77 | | 506.149 | 7.333 | 7.29 | 2I07, 2I32 |
| 499.145 | 5.093 | 5.09 | 2I100, 2I28, 2I80 | | 505.154 | 7.377 | 7.29 | 2130, 2131 |
| 498.149 | 5.11 | 5.09 | 2I27, 2179, 2I99 | | 575.132 | 7.44 | 7.39 | 2I20 |
| 472.136 | 5.113 | 5.09 | 2I67 | | 486.115 | 7.567 | 7.54 | 2I23, 2I77 |
| 482.186 | 5.14 | 5.09 | 2I73 | | 487.11 | 7.57 | 7.54 | 2I24, 2I78 |
| 466.18 | 5.23 | 5.19 | 2I45 | | 498.149 | 7.677 | 7.66 | 2127, 2179, 2I99 |
| 484.17 | 5.36 | 5.32 | 2I51 | | 498.149 | 7.697 | 7.66 | 2I27, 2179, 2I99 |
| 528.191 | 5.467 | 5.43 | 2I85 | | 510.181 | 7.7 | 7.66 | 2I11,2155 |
| 480.195 | 5.517 | 5.49 | 2I47, 2I97 | | 499.145 | 7.717 | 7.66 | 2I100, 2I28, 2180 |
| 496.165 | 5.52 | 5.49 | 2I03, 2I53, 2I75 | | 532.165 | 7.73 | 7.66 | 2I13 |
| 481.19 | 5.52 | 5.49 | 2I48, 2I98 | | 494.174 | 7.843 | 7.81 | 2I25, 2I49 |
| 509.185 | 5.52 | 5.49 | 2I34 | | 504.159 | 8 | 7.97 | 2I29 |

(continued)

| [Table EJ07] | | | | | | | | |
|---|---|---|---|---|---|---|---|---|
| Mixture No. | mixEJ07 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 531.17 | 5.573 | 5.53 | 2I36 | | 573.141 | 8.07 | 8.04 | 2I42 |
| 493.165 | 5.627 | 5.6 | 2I96 | | 508.14 | 8.263 | 8.25 | 2I10, 2133 |
| 492.17 | 5.64 | 5.6 | 2I95 | | 499.145 | 8.273 | 8.25 | 2I100, 2I28, 2I80 |
| 512.196 | 5.643 | 5.6 | 2I81 | | 550.137 | 8.277 | 8.25 | 2I17, 2I63 |
| 506.149 | 5.687 | 5.65 | 2I07, 2I32 | | 508.14 | 8.283 | 8.25 | 2I10, 2I33 |
| 519.181 | 5.793 | 5.77 | 2I92 | | 530.174 | 8.343 | 8.3 | 2I35 |
| 518.186 | 5.797 | 5.77 | 2I91 | | 480.195 | 8.347 | 8.3 | 2I47, 2I97 |
| 551.132 | 5.823 | 5.77 | 2I18, 2I64 | | 616.158 | 8.74 | 8.7 | 2I21 |
| 526.201 | 5.847 | 5.82 | 2I59, 2I83 | | 548.146 | 8.86 | 8.82 | 2I39 |
| 527.196 | 5.863 | 5.82 | 2I60, 2I84 | | 538.212 | 8.877 | 8.82 | 2I15 |
| 491.175 | 5.903 | 5.87 | 2I70 | | 574.137 | 9.09 | 9.06 | 2I19 |
| 490.18 | 5.92 | 5.87 | 2I69 | | 614.168 | 9.277 | 9.25 | 2I43 |
| 494.174 | 5.92 | 5.87 | 2I25, 2I49 | | 536.221 | 9.43 | 9.39 | 2I37, 2I87 |
| 510.181 | 5.99 | 5.96 | 2I11, 2I55 | | 572.146 | 9.627 | 9.59 | 2I41 |

[Table 233]

| [Table EJ08] | | | | | | | | |
|---|---|---|---|---|---|---|---|---|
| Mixture No. | mixEJ08 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 517.169 | 3.873 | 3.83 | 2L94 | | 532.226 | 6.513 | 6.48 | 2L69 |
| 525.228 | 3.92 | 3.88 | 2L74 | | 536.221 | 6.56 | 6.52 | 2L25, 2L49 |
| 515.178 | 4.04 | 4 | 2L68 | | 552.228 | 6.587 | 6.52 | 2L11,2L55 |
| 541.192 | 4.073 | 4.04 | 2L100, 2L28, 2L80 | | 560.233 | 6.633 | 6.59 | 2L91 |
| 529.157 | 4.077 | 4.04 | 2L24, 2L78 | | 548.196 | 6.643 | 6.59 | 2L07, 2L32 |
| 523.237 | 4.17 | 4.12 | 2L48, 2L98 | | 568.248 | 6.723 | 6.69 | 2L59, 2L83 |
| 527.212 | 4.287 | 4.24 | 2L52 | | 558.242 | 6.737 | 6.69 | 2L65 |
| 511.212 | 4.367 | 4.32 | 2L72 | | 566.268 | 6.887 | 6.86 | 2L57 |
| 539.207 | 4.437 | 4.41 | 2L04, 2L54, 2L76 | | 593.179 | 6.997 | 6.97 | 2L18, 2L64 |

(continued)

| [Table EJ08] | | | | | | | | |
|---|---|---|---|---|---|---|---|---|
| Mixture No. | mixEJ08 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 571.233 | 4.47 | 4.41 | 2L86 | | 578.268 | 7.01 | 6.97 | 2L37, 2L87 |
| 509,222 | 4.483 | 4.44 | 2L46 | | 576.214 | 7.037 | 6.97 | 2L61 |
| 555.238 | 4.673 | 4.65 | 2L82 | | 581.254 | 7.123 | 7.1 | 2L16 |
| 561.228 | 4.697 | 4.65 | 2L92 | | 659.201 | 7.137 | 7.1 | 2L22 |
| 559.237 | 4.853 | 4.81 | 2L66 | | 539.207 | 7.143 | 7.1 | 2L04, 2L54, 2L76 |
| 569.243 | 5 | 4.97 | 2L60, 2L84 | | 594,199 | 7.18 | 7.15 | 2L89 |
| 567,264 | 5.213 | 5.18 | 2L58 | | 592.184 | 7.207 | 7.15 | 2L17, 2L63 |
| 533.222 | 5.36 | 5.34 | 2L70 | | 591.188 | 7.423 | 7.38 | 2L40 |
| 531.148 | 5.367 | 5.34 | 2L02 | | 579.264 | 7.473 | 7.44 | 2L38, 2L88 |
| 579.264 | 5.38 | 5.34 | 2L38, 2L88 | | 657.21 | 7.513 | 7.48 | 2L44 |
| 540.196 | 5.397 | 5.34 | 2L27, 2L79, 2L99 | | 549.191 | 7.833 | 7.79 | 2L08,2L09 |
| 539.207 | 5.417 | 5.34 | 2L04, 2L54, 2L76 | | 530.153 | 8.07 | 8.03 | 2L01 |
| 577.209 | 5.493 | 5.47 | 2L62 | | 547.201 | 8.213 | 8.19 | 2L30, 2L31 |
| 595.194 | 5.57 | 5.53 | 2L90 | | 548.196 | 8.227 | 8.19 | 2L07,2L32 |
| 543.182 | 5.597 | 5.53 | 2L06 | | 542.187 | 8.227 | 8.19 | 2L05 |
| 516.173 | 5.673 | 5.63 | 2L93 | | 546.206 | 8.243 | 8.19 | 2L29 |
| 593.179 | 5.683 | 5.63 | 2L18, 2L64 | | 538.212 | 8.39 | 8.35 | 2L03, 2L53, 2L75 |
| 510.217 | 5.707 | 5.67 | 2L71 | | 528.162 | 8.503 | 8.47 | 2L23, 2L77 |
| 537.217 | 5.823 | 5.79 | 2L26, 2L50 | | 617.179 | 8.56 | 8.52 | 2L20 |
| 514.183 | 5.837 | 5.79 | 2L67 | | 541.192 | 8.607 | 8.59 | 2L100, 2L28, 2L80 |
| 537.217 | 5.84 | 5.79 | 2L26, 2L50 | | 540.196 | 8.627 | 8.59 | 2L27, 2L79, 2L99 |
| 528.162 | 5.863 | 5.83 | 2L23, 2L77 | | 535.212 | 8.77 | 8.74 | 2L96 |
| 529.157 | 5.863 | 5.83 | 2L24, 2L78 | | 536.221 | 8.78 | 8.74 | 2L25, 2L49 |
| 508.226 | 5.897 | 5.86 | 2L45 | | 552.228 | 8.857 | 8.82 | 2L11, 2L55 |
| 540.196 | 5.913 | 5.86 | 2L27, 2L79, 2L99 | | 553.223 | 8.867 | 8.82 | 2L12, 2L56 |
| 524.233 | 5.947 | 5.91 | 2L73 | | 574.212 | 8.903 | 8.88 | 2L13 |
| 553.223 | 5.953 | 5.91 | 2L12,2L56 | | 547.201 | 8.933 | 8.88 | 2L30, 2L31 |
| 575.207 | 6.023 | 6 | 2L14 | | 615.188 | 8.937 | 8.88 | 2L42 |
| 526.217 | 6.043 | 6 | 2L51 | | 549.191 | 9.163 | 9.14 | 2L08, 2L09 |

(continued)

| [Table EJ08] | | | | | | | | |
|---|---|---|---|---|---|---|---|---|
| Mixture No. | mixEJ08 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 541.192 | 6.06 | 6 | 2L100, 2L28, 2L80 | | 550.187 | 9.183 | 9.14 | 2L10, 2L33 |
| 538.212 | 6.163 | 6.13 | 2L03, 2L53, 2L75 | | 572.221 | 9.247 | 9.2 | 2L35 |
| 550.187 | 6.167 | 6.13 | 2L10,2L33 | | 592.184 | 9.397 | 9.36 | 2L17, 2L63 |
| 522.242 | 6.183 | 6.13 | 2L47, 2L97 | | 522.242 | 9.68 | 9.67 | 2L47, 2L97 |
| 570.238 | 6.223 | 6.19 | 2L85 | | 590.193 | 9.707 | 9.67 | 2L39 |
| 551.232 | 6.327 | 6.3 | 2L34 | | 658.205 | 9.78 | 9.74 | 2L21 |
| 538.212 | 6.403 | 6.36 | 2L03, 2L53, 2L75 | | 580.259 | 10.01 | 9.97 | 2L15 |
| 554.243 | 6.407 | 6.36 | 2L81 | | 656.215 | 10.077 | 10.03 | 2L43 |
| 534.217 | 6.433 | 6.39 | 2L95 | | 616.184 | 10.13 | 10.09 | 2L19 |
| 573.217 | 6.437 | 6.39 | 2L36 | | 578.268 | 10.293 | 10.25 | 2L37, 2L87 |
| 568.248 | 6.467 | 6.39 | 2L59, 2L83 | | 614.193 | 10.413 | 10.38 | 2L41 |
| 569.243 | 6.477 | 6.39 | 2L60, 2L84 | | 523.237 | 12.043 | 12 | 2L48, 2L98 |

[Table 234]

| [Table EJ09] | | | | | | | | |
|---|---|---|---|---|---|---|---|---|
| Table EJ09 | | | | | | | | |
| Mixture No. | mixEJ09 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 557.125 | 4.003 | 3.95 | 2N94 | | 600.189 | 6.923 | 6.89 | 2N91 |
| 565.184 | 4.067 | 4.03 | 2N74 | | 591.188 | 6.933 | 6.89 | 2N34 |
| 581.148 | 4.223 | 4.2 | 2N100, 2N28, 2N80 | | 608.204 | 6.947 | 6.89 | 2N59, 2N83 |
| 555.134 | 4.487 | 4.45 | 2N68 | | 608.204 | 7.01 | 6.98 | 2N59, 2N83 |
| 551.168 | 4.523 | 4.49 | 2N72 | | 572.183 | 7.027 | 6.98 | 2N69 |
| 563.193 | 4.603 | 4.6 | 2N48, 2N98 | | 613.173 | 7.053 | 6.98 | 2N36 |
| 579.163 | 4.783 | 4.75 | 2N04, 2N54, 2N76 | | 576.177 | 7.067 | 7.04 | 2N25, 2N49 |
| 595.194 | 4.867 | 4.84 | 2N82 | | 592.184 | 7.08 | 7.04 | 2N11, 2N55 |

(continued)

| [Table EJ09] | | | | | | | | |
|---|---|---|---|---|---|---|---|---|
| Table EJ09 | | | | | | | | |
| Mixture No. | mixEJ09 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 549.178 | 4.987 | 4.94 | 2N46 | | 598.198 | 7.25 | 7.21 | 2N65 |
| 577.173 | 5.11 | 5.07 | 2N26, 2N50 | | 633.135 | 7.277 | 7.21 | 2N18, 2N64 |
| 609.199 | 5.213 | 5.18 | 2N60, 2N84 | | 618.224 | 7.31 | 7.27 | 2N37,2N87 |
| 609.199 | 5.237 | 5.18 | 2N60, 2N84 | | 588.152 | 7.337 | 7.27 | 2N07, 2N32 |
| 599.193 | 5.347 | 5.33 | 2N66 | | 621.21 | 7.35 | 7.32 | 2N16 |
| 593.179 | 5.37 | 5.33 | 2N12,2N56 | | 699.157 | 7.353 | 7.32 | 2N22 |
| 571.104 | 5.56 | 5.52 | 2N02 | | 606.224 | 7.373 | 7.32 | 2N57 |
| 575.168 | 5.577 | 5.52 | 2N96 | | 588.152 | 7.373 | 7.32 | 2N07, 2N32 |
| 579.163 | 5.607 | 5.58 | 2N04, 2N54, 2N76 | | 634.155 | 7.48 | 7.44 | 2N89 |
| 619.22 | 5.63 | 5.58 | 2N38, 2N88 | | 616.17 | 7.557 | 7.52 | 2N61 |
| 607.22 | 5.703 | 5.67 | 2N58 | | 632.14 | 7.71 | 7.67 | 2N17, 2N63 |
| 635.151 | 5.813 | 5.78 | 2N90 | | 631.144 | 8.033 | 8.01 | 2N40 |
| 583.138 | 5.827 | 5.78 | 2N06 | | 619.22 | 8.037 | 8.01 | 2N38, 2N88 |
| 556.129 | 5.977 | 5.94 | 2N93 | | 697.166 | 8.05 | 8.01 | 2N44 |
| 573.178 | 6.01 | 5.97 | 2N70 | | 589.148 | 8.133 | 8.09 | 2N08, 2N09 |
| 550.173 | 6.017 | 5.97 | 2N71 | | 570.109 | 8.35 | 8.32 | 2N01 |
| 617.165 | 6.053 | 5.97 | 2N62 | | 582.143 | 8.507 | 8.47 | 2N05 |
| 568.118 | 6.173 | 6.15 | 2N23, 2N77 | | 578.168 | 8.647 | 8.61 | 2N03, 2N53, 2N75 |
| 593.179 | 6.177 | 6.15 | 2N12, 2N56 | | 579.163 | 8.66 | 8.61 | 2N04, 2N54, 2N76 |
| 580.152 | 6.197 | 6.15 | 2N27, 2N79, 2N99 | | 657.135 | 8.8 | 8.79 | 2N20 |
| 580.152 | 6.217 | 6.15 | 2N27, 2N79, 2N99 | | 587.157 | 8.823 | 8.79 | 2N30, 2N31 |
| 633.135 | 6.23 | 6.15 | 2N18, 2N64 | | 589.148 | 8.823 | 8.79 | 2N08, 2N09 |
| 564.189 | 6.25 | 6.22 | 2N73 | | 568.118 | 9.113 | 9.1 | 2N23, 2N77 |
| 615.163 | 6.273 | 6.22 | 2N14 | | 569.113 | 9.113 | 9.1 | 2N24, 2N78 |
| 554.139 | 6.39 | 6.36 | 2N67 | | 592.184 | 9.117 | 9.1 | 2N11, 2N55 |
| 577.173 | 6.44 | 6.41 | 2N26, 2N50 | | 614.168 | 9.143 | 9.1 | 2N13 |
| 548.183 | 6.443 | 6.41 | 2N45 | | 580.152 | 9.22 | 9.18 | 2N27, 2N79, 2N99 |

(continued)

| [Table EJ09] | | | | | | | | |
|---|---|---|---|---|---|---|---|---|
| Table EJ09 | | | | | | | | |
| Mixture No. | mixEJ09 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 569.113 | 6.45 | 6.41 | 2N24, 2N78 | | 581.148 | 9.223 | 9.18 | 2N100, 2N28, 2N80 |
| 590,143 | 6.46 | 6.41 | 2N10, 2N33 | | 576.177 | 9.353 | 9.32 | 2N25, 2N49 |
| 610.194 | 6.5 | 6.46 | 2N85 | | 655.144 | 9.473 | 9.44 | 2N42 |
| 611.189 | 6.507 | 6.46 | 2N86 | | 586.162 | 9.52 | 9.44 | 2N29 |
| 567.168 | 6.573 | 6.55 | 2N52 | | 632.14 | 9.62 | 9.59 | 2N17, 2N63 |
| 566.173 | 6.587 | 6.55 | 2N51 | | 590.143 | 9.76 | 9.74 | 2N10, 2N33 |
| 563.193 | 6.663 | 6.66 | 2N48, 2N98 | | 612.177 | 9.777 | 9.74 | 2N35 |
| 578.168 | 6.683 | 6.66 | 2N03, 2N53, 2N75 | | 698.161 | 9.977 | 9.94 | 2N21 |
| 562.198 | 6.69 | 6.66 | 2N47,2N97 | | 620.215 | 10.19 | 10.17 | 2N15 |
| 594.199 | 6.693 | 6.66 | 2N81 | | 630.149 | 10.213 | 10.17 | 2N39 |
| 578.168 | 6.707 | 6.66 | 2N03, 2N53, 2N75 | | 656.14 | 10.3 | 10.27 | 2N19 |
| 562.198 | 6.717 | 6.66 | 2N47, 2N97 | | 696.171 | 10.53 | 10.49 | 2N43 |
| 581.148 | 6.733 | 6.7 | 2N100, 2N28, 2N80 | | 618.224 | 10.76 | 10.72 | 2N37, 2N87 |
| 574.173 | 6.747 | 6.7 | 2N95 | | 654.149 | 10.847 | 10.82 | 2N41 |
| 601.184 | 6.91 | 6.89 | 2N92 | | 587.157 | 11.25 | 10.82 | 2N30, 2N31 |

[Table 235]

| [Table EJ10] | | | | | | | | |
|---|---|---|---|---|---|---|---|---|
| Mixture No. | mixEJ10 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 617.29 | 6.513 | 6.47 | 2A74 | | 621.22 | 8.75 | 8.68 | 2A24, 2A78 |
| 609.231 | 6.557 | 6.52 | 2A94 | | 660.31 | 8.787 | 8.76 | 2A59, 2A83 |
| 607.241 | 6.683 | 6.65 | 2A68 | | 658.331 | 8.853 | 8.82 | 2A57 |
| 615.3 | 6.7 | 6.65 | 2A48, 2A98 | | 650.305 | 8.893 | 8.86 | 2A65 |
| 621.22 | 6.843 | 6.8 | 2A24, 2A78 | | 652.295 | 8.967 | 8.94 | 2A91 |

(continued)

| [Table EJ10] | | | | | | | | |
|---|---|---|---|---|---|---|---|---|
| Mixture No. | mixEJ10 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 632.259 | 6.983 | 6.96 | 2A27, 2A79, 2A99 | | 667.27 | 8,997 | 8.94 | 2A14 |
| 631.27 | 6.987 | 6.96 | 2A04, 2A54, 2A76 | | 643.295 | 9.073 | 9.03 | 2A34 |
| 603.275 | 6.997 | 6.96 | 2A72 | | 633.254 | 9.187 | 9.16 | 2A100, 2A28, 2A80 |
| 601.284 | 7.063 | 7.03 | 2A46 | | 668.276 | 9.197 | 9.16 | 2A61 |
| 647.301 | 7.103 | 7.07 | 2A82 | | 669.272 | 9.197 | 9.16 | 2A62 |
| 661.305 | 7.12 | 7.07 | 2A60, 2A84 | | 684.246 | 9.327 | 9.29 | 2A17, 2A63 |
| 651.3 | 7.34 | 7.29 | 2A66 | | 665.279 | 9.343 | 9.29 | 2A36 |
| 661.305 | 7.367 | 7.33 | 2A60, 2A84 | | 642.249 | 9.357 | 9.29 | 2A10, 2A33 |
| 659.326 | 7.513 | 7.48 | 2A58 | | 670.331 | 9.387 | 9.35 | 2A37, 2A87 |
| 671.326 | 7.993 | 7.95 | 2A38, 2A88 | | 686.262 | 9.517 | 9.48 | 2A89 |
| 685.241 | 8.087 | 8.07 | 2A18, 2A64 | | 640.259 | 9.717 | 9.64 | 2A07, 2A32 |
| 633.254 | 8.097 | 8.07 | 2A100, 2A28, 2A80 | | 751.263 | 9.753 | 9.71 | 2A22 |
| 602.28 | 8.097 | 8.07 | 2A71 | | 673.317 | 9.853 | 9.82 | 2A16 |
| 632.259 | 8.103 | 8.07 | 2A27, 2A79, 2A99 | | 685.241 | 9.977 | 9.95 | 2A18, 2A64 |
| 633.254 | 8.117 | 8.07 | 2A100, 2A28, 2A80 | | 749.273 | 10.073 | 10.05 | 2A44 |
| 687.257 | 8.12 | 8.07 | 2A90 | | 671.326 | 10.167 | 10.13 | 2A38, 2A88 |
| 600.289 | 8.173 | 8.15 | 2A45 | | 683.251 | 10.31 | 10.28 | 2A40 |
| 616.295 | 8.183 | 8.15 | 2A73 | | 641.254 | 10.717 | 10.68 | 2A08, 2A09 |
| 608.236 | 8.203 | 8.15 | 2A93 | | 641.254 | 10.743 | 10.68 | 2A08, 2A09 |
| 606.245 | 8.21 | 8.15 | 2A67 | | 639.264 | 11.013 | 10.98 | 2A30, 2A31 |
| 620.225 | 8.257 | 8.23 | 2A23, 2A77 | | 638.268 | 11.033 | 10.98 | 2A29 |
| 653.29 | 8.257 | 8.23 | 2A92 | | 622.215 | 11.06 | 10.98 | 2A01 |

(continued)

| [Table EJ10] | | | | | | | | |
|---|---|---|---|---|---|---|---|---|
| Mixture No. | mixEJ10 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 630.274 | 8.287 | 8.23 | 2A03, 2A53, 2A75 | | 635.245 | 11.143 | 11.11 | 2A06 |
| 663.296 | 8.31 | 8.28 | 2A86 | | 634.249 | 11.153 | 11.11 | 2A05 |
| 618.28 | 8.313 | 8.28 | 2A51 | | 709.241 | 11.303 | 11.27 | 2A20 |
| 619.275 | 8.313 | 8.28 | 2A52 | | 630.274 | 11.313 | 11.27 | 2A03, 2A53, 2A75 |
| 662.301 | 8.317 | 8.28 | 2A85 | | 630.274 | 11.34 | 11.27 | 2A03, 2A53, 2A75 |
| 614.305 | 8.32 | 8.28 | 2A47, 2A97 | | 620.225 | 11.377 | 11.33 | 2A23, 2A77 |
| 615.3 | 8.323 | 8.28 | 2A48, 2A98 | | 632.259 | 11.44 | 11.4 | 2A27, 2A79, 2A99 |
| 614.305 | 8.343 | 8.28 | 2A47,2A97 | | 640.259 | 11.46 | 11.4 | 2A07, 2A32 |
| 623.21 | 8.36 | 8.33 | 2A02 | | 707.251 | 11.597 | 11.57 | 2A42 |
| 631.27 | 8.37 | 8.33 | 2A04, 2A54, 2A76 | | 628.284 | 11.607 | 11.57 | 2A25, 2A49 |
| 625.284 | 8.373 | 8.33 | 2A70 | | 644.29 | 11.703 | 11.68 | 2A11, 2A55 |
| 660.31 | 8.52 | 8.5 | 2A59,2A83 | | 666.274 | 11.74 | 11.68 | 2A13 |
| 646.306 | 8.537 | 8.5 | 2A81 | | 639.264 | 11.947 | 11.92 | 2A30, 2A31 |
| 644.29 | 8.627 | 8.59 | 2A11, 2A55 | | 642.249 | 11.953 | 11.92 | 2A10, 2A33 |
| 624.289 | 8.637 | 8.59 | 2A69 | | 664.284 | 11.987 | 11.95 | 2A35 |
| 626.28 | 8.687 | 8.68 | 2A95 | | 684.246 | 12.05 | 12.02 | 2A17, 2A63 |
| 631.27 | 8.707 | 8.68 | 2A04, 2A54, 2A76 | | 750.268 | 12.253 | 12.23 | 2A21 |
| 627.275 | 8.71 | 8.68 | 2A96 | | 682.256 | 12.297 | 12.26 | 2A39 |
| 628.284 | 8.71 | 8.68 | 2A25, 2A49 | | 748.278 | 12.513 | 12.47 | 2A43 |
| 629.279 | 8.717 | 8.68 | 2A26, 2A50 | | 708.246 | 12.59 | 12.55 | 2A19 |
| 645.285 | 8.727 | 8.68 | 2A12, 2A56 | | 672.321 | 12.663 | 12.62 | 2A15 |
| 629.279 | 8.733 | 8.68 | 2A26, 2A50 | | 706.256 | 12.82 | 12.79 | 2A41 |

(continued)

| [Table EJ10] | | | | | | | | |
|---|---|---|---|---|---|---|---|---|
| Mixture No. | mixEJ10 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 645.285 | 8.743 | 8.68 | 2A12, 2A56 | | 670.331 | 12.883 | 12.85 | 2A37, 2A87 |

[Table 236]

| [Table EJ11] | | | | | | | | |
|---|---|---|---|---|---|---|---|---|
| Mixture No. | mixEJ11 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 763.2 | 5.617 | 5.59 | 2Q30, 2Q31 | | 745.157 | 8.743 | 8.71 | 2Q24, 2Q78 |
| 741.227 | 6.79 | 6.75 | 2Q74 | | 752.221 | 8.75 | 8.71 | 2Q25, 2Q49 |
| 757.191 | 6.883 | 6.83 | 2Q100, 2Q28, | | 2Q80 759.181 | 8.763 | 8.71 | 2Q06 |
| 731.177 | 6.943 | 6.89 | 2Q68 | | 785.242 | 8.803 | 8.76 | 2Q60, 2Q84 |
| 739.237 | 6.953 | 6.89 | 2Q48, 2Q98 | | 784.247 | 8.81 | 8.76 | 2Q59, 2Q83 |
| 739.237 | 6.97 | 6.89 | 2Q48, 2Q98 | | 791.206 | 8.873 | 8.87 | 2Q14 |
| 755.206 | 7.063 | 7.05 | 2Q04, 2Q54, | | 2Q76 782.268 | 8.897 | 8.87 | 2Q57 |
| 745.157 | 7.087 | 7.05 | 2Q24, 2Q78 | | 774.241 | 8.9 | 8.87 | 2Q65 |
| 787.233 | 7.09 | 7.05 | 2Q86 | | 776.232 | 8.913 | 8.87 | 2Q91 |
| 755.206 | 7.167 | 7.13 | 2Q04, 2Q54, | | 2Q76 767.232 | 9.043 | 9 | 2Q34 |
| 756.196 | 7.17 | 7.13 | 2Q27, 2Q79, | | 2Q99 757.191 | 9.097 | 9.07 | 2Q100, 2Q28, 2Q80 |
| 744.161 | 7.203 | 7.13 | 2Q23, 2Q77 | | 792.213 | 9.163 | 9.15 | 2Q61 |
| 743.212 | 7.22 | 7.19 | 2Q52 | | 793.208 | 9.177 | 9.15 | 2Q62 |
| 727.212 | 7.223 | 7.19 | 2Q72 | | 789.216 | 9.197 | 9.15 | 2Q36 |
| 753.216 | 7.287 | 7.26 | 2Q26, 2Q50 | | 808.183 | 9.273 | 9.24 | 2Q17, 2Q63 |

(continued)

| [Table EJ11] | | | | | | | | |
|---|---|---|---|---|---|---|---|---|
| Mixture No. | mixEJ11 | | | | | | | |
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 785.242 | 7.31 | 7.26 | 2Q60, 2Q84 | | 794.268 | 9.287 | 9.24 | 2Q37, 2Q87 |
| 725.221 | 7.32 | 7.26 | 2Q46 | | 766.186 | 9.287 | 9.24 | 2010, 2Q33 |
| 777.227 | 7.41 | 7.37 | 2Q92 | | 811.194 | 9.363 | 9.35 | 2Q90 |
| 769.222 | 7.447 | 7.41 | 2Q12, 2Q56 | | 810.199 | 9.387 | 9.35 | 2Q89 |
| 775.237 | 7.503 | 7.48 | 2Q66 | | 875.2 | 9.543 | 9.51 | 2Q22 |
| 783.263 | 7.65 | 7.62 | 2Q58 | | 764.196 | 9.59 | 9.51 | 2Q07, 2Q32 |
| 751.212 | 7.907 | 7.87 | 2Q96 | | 797.253 | 9.597 | 9.57 | 2Q16 |
| 749.221 | 7.98 | 7.94 | 2Q70 | | 765.191 | 9.623 | 9.57 | 2Q08, 2Q09 |
| 795.263 | 8.057 | 8.02 | 2Q38, 2Q88 | | 764.196 | 9.627 | 9.57 | 2Q07, 2Q32 |
| 809.178 | 8.167 | 8.13 | 2Q18, 2Q64 | | 809.178 | 9.763 | 9.72 | 2018, 2Q64 |
| 726.216 | 8.203 | 8.17 | 2Q71 | | 873.209 | 9.86 | 9.83 | 2Q44 |
| 756.196 | 8.203 | 8.17 | 2Q27, 2Q79, | | 2Q99 874.205 | 9.87 | 9.83 | 2Q21 |
| 757.191 | 8.21 | 8.17 | 2Q100, 2Q28, 2Q80 | | 795.263 | 9.91 | 9.87 | 2Q38, 2Q88 |
| 732.173 | 8.26 | 8.17 | 2Q93 | | 807.188 | 10.08 | 10.04 | 2Q40 |
| 740.232 | 8.27 | 8.25 | 2Q73 | | 765.191 | 10.44 | 10.4 | 2Q08, 2Q09 |
| 724.226 | 8.287 | 8.25 | 2Q45 | | 872.214 | 10.523 | 10.48 | 2Q43 |
| 730.182 | 8.293 | 8.25 | 2Q67 | | 763.2 | 10.747 | 10.7 | 2Q30, 2Q31 |
| 744.161 | 8.33 | 8.29 | 2Q23, 2Q77 | | 758.186 | 10.79 | 10.73 | 2Q05 |
| 746.152 | 8.337 | 8.29 | 2Q01 | | 754.211 | 10.873 | 10.87 | 2Q03, 2Q53, 2Q75 |
| 754.211 | 8.363 | 8.29 | 2Q03, 2Q53, 2Q75 | | 833.178 | 10.907 | 10.87 | 2Q20 |

(continued)

[Table EJ11]

| Mixture No. | mixEJ11 | | | | | | | |
|---|---|---|---|---|---|---|---|---|
| m/z [M+H]+ | Retention time of MS peak (min) | Retention time of UV peak (min) | No. of assigned compound | | m/z [M+H]+ | Retention time of MS peak (min) | Retention time of UV peak (min) | No. of assigned compound |
| 747.147 | 8.367 | 8.34 | 2Q02 | | 756.196 | 11.063 | 11.02 | 2Q27, 2Q79, 2Q99 |
| 786.237 | 8.383 | 8.34 | 2Q85 | | 752.221 | 11.173 | 11.19 | 2Q25, 2Q49 |
| 754.211 | 8.383 | 8.34 | 2Q03, 2Q53, 2Q75 | | 831.188 | 11.227 | 11.19 | 2Q42 |
| 742.216 | 8.407 | 8.34 | 2Q51 | | 790.211 | 11.243 | 11.19 | 2Q13 |
| 738.241 | 8.423 | 8.34 | 2Q47, 2Q97 | | 768.227 | 11.29 | 11.24 | 2011, 2Q55 |
| 738.241 | 8.44 | 8.34 | 2Q47, 2Q97 | | 762.205 | 11.343 | 11.29 | 2Q29 |
| 771.238 | 8.563 | 8.54 | 2Q82 | | 788.221 | 11.497 | 11.47 | 2Q35 |
| 770.243 | 8.577 | 8.54 | 2Q81 | | 766.186 | 11.563 | 11.53 | 2Q10, 2Q33 |
| 784.247 | 8.583 | 8.54 | 2Q59, 2Q83 | | 808.183 | 11.57 | 11.53 | 2Q17, 2Q63 |
| 768.227 | 8.673 | 8.66 | 2Q11, 2Q55 | | 806.192 | 11.837 | 11.8 | 2Q39 |
| 755.206 | 8.683 | 8.66 | 2Q04, 2Q54, 2Q76 | | 832.183 | 12.057 | 12.01 | 2Q19 |
| 748.226 | 8.687 | 8.66 | 2Q69 | | 796.258 | 12.067 | 12.01 | 2Q15 |
| 750.216 | 8.693 | 8.66 | 2Q95 | | 794.268 | 12.327 | 12.29 | 2Q37, 2Q87 |
| 753.216 | 8.697 | 8.66 | 2Q26, 2Q50 | | 830.192 | 12.347 | 12.29 | 2Q41 |
| 769.222 | 8.707 | 8.66 | 2Q12, 2Q56 | | 733.168 | 14.537 | 14.6 | 2Q94 |

[Table 237]

| [Table EJ12] | | | | | | | | |
|---|---|---|---|---|---|---|---|---|
| Mixture No. | mixEJ12 | | | | | | | |
| m/z [M+H]+ | Retention time of MS peak (min) | Retention time of UV peak (min) | No. of assigned m/z compound | | [M+H]+ | Retention time of MS peak (min) | Retention time of UV peak (min) | No. of assigned compound |
| 788.193 | 3.34 | 3.34 | 2R08, 2R09 | | 807.249 | 9.27 | 9.23 | 2R59, 2R83 |
| 810.235 | 6.247 | 6.21 | 2R86 | | 805.27 | 9.293 | 9.23 | 2R57 |
| 816.211 | 6.3 | 6.25 | 2R62 | | 769.154 | 9.31 | 9.23 | 2R01 |
| 786.203 | 7.023 | 6.99 | 2R30, 2R31 | | 768.159 | 9.33 | 9.29 | 2R24, 2R78 |
| 767.164 | 7.127 | 7.09 | 2R23, 2R77 | | 792.224 | 9.33 | 9.29 | 2R12,2R56 |
| 763.234 | 7.24 | 7.2 | 2R73 | | 798.239 | 9.337 | 9.29 | 2R66 |
| 764.23 | 7.347 | 7.32 | 2R74 | | 797.244 | 9.34 | 9.29 | 2R65 |
| 756.17 | 7.43 | 7.39 | 2R94 | | 799.234 | 9.423 | 9.39 | 2R91 |
| 780.193 | 7.443 | 7.39 | 2R100, 2R28, 2R80 | | 782.184 | 9.427 | 9.39 | 2R06 |
| 762.239 | 7.493 | 7.44 | 2R48, 2R98 | | 814.209 | 9.527 | 9.48 | 2R14 |
| 754.18 | 7.497 | 7.44 | 2R68 | | 790.234 | 9.593 | 9.57 | 2R34 |
| 778.209 | 7.74 | 7.71 | 2R04, 2R54, 2R76 | | 815.215 | 9.607 | 9.57 | 2R61 |
| 766.214 | 7.753 | 7.71 | 2R52 | | 780.193 | 9.703 | 9.67 | 2R100, 2R28, 2R80 |
| 808.244 | 7.803 | 7.78 | 2R60, 2R84 | | 831.185 | 9.703 | 9.67 | 2R17,2R63 |
| 776.218 | 7.807 | 7.78 | 2R26, 2R50 | | 812.218 | 9.77 | 9.76 | 2R36 |
| 750.214 | 7.817 | 7.78 | 2R72 | | 817.27 | 9.793 | 9.76 | 2R37, 2R87 |
| 794.24 | 7.827 | 7.78 | 2R82 | | 833.201 | 9.903 | 9.86 | 2R89 |
| 800.23 | 7.997 | 7.96 | 2R92 | | 898.202 | 10.133 | 10.1 | 2R22 |
| 806.265 | 8.137 | 8.1 | 2R58 | | 787.198 | 10.197 | 10.17 | 2R07, 2R32 |
| 772.223 | 8.463 | 8.41 | 2R70 | | 820.256 | 10.207 | 10.17 | 2R16 |
| 834.196 | 8.61 | 8.58 | 2R90 | | 832.181 | 10.383 | 10.35 | 2R18, 2R64 |
| 818.265 | 8.613 | 8.58 | 2R38, 2R88 | | 896.212 | 10.39 | 10.35 | 2R44 |
| 779.198 | 8.677 | 8.58 | 2R27, 2R79, 2R99 | | 818.265 | 10.443 | 10.41 | 2R38, 2R88 |

(continued)

| [Table EJ12] | | | | | | | | |
|---|---|---|---|---|---|---|---|---|
| Mixture No. | mixEJ12 | | | | | | | |
| m/z [M+H]+ | Retention time of MS peak (min) | Retention time of UV peak (min) | No. of assigned m/z compound | | [M+H]+ | Retention time of MS peak (min) | Retention time of UV peak (min) | No. of assigned compound |
| 779.198 | 8.703 | 8.7 | 2R27, 2R79, 2R99 | | 830.19 | 10.637 | 10.6 | 2R40 |
| 749.219 | 8.717 | 8.7 | 2R71 | | 895.217 | 10.913 | 10.78 | 2R43 |
| 747.228 | 8.74 | 8.7 | 2R45 | | 789.188 | 11.027 | 10.99 | 2R10, 2R33 |
| 748.223 | 8.747 | 8.7 | 2R46 | | 788.193 | 11.037 | 10.99 | 2R08, 2R09 |
| 753.185 | 8.763 | 8.7 | 2R67 | | 786.203 | 11.257 | 11.22 | 2R30, 2R31 |
| 755.175 | 8.793 | 8.7 | 2R93 | | 787.198 | 11.257 | 11.22 | 2R07,2R32 |
| 777.214 | 8.803 | 8.7 | 2R03, 2R53, 2R75 | | 781.188 | 11.36 | 11.32 | 2R05 |
| 778.209 | 8.823 | 8.81 | 2R04, 2R54, 2R76 | | 856.181 | 11.487 | 11.45 | 2R20 |
| 761.244 | 8.83 | 8.81 | 2R47, 2R97 | | 777.214 | 11.487 | 11.45 | 2R03, 2R53, 2R75 |
| 809.24 | 8.84 | 8.81 | 2R85 | | 767.164 | 11.533 | 11.45 | 2R23,2R77 |
| 761.244 | 8.847 | 8.81 | 2R47, 2R97 | | 780.193 | 11.557 | 11.54 | 2R1 00, 2R28, 2R80 |
| 765.219 | 8.85 | 8.81 | 2R51 | | 779.198 | 11.57 | 11.54 | 2R27, 2R79, 2R99 |
| 768.159 | 8.85 | 8.81 | 2R24, 2R78 | | 775.223 | 11.687 | 11.61 | 2R25, 2R49 |
| 762.239 | 8.86 | 8.81 | 2R48, 2R98 | | 854.19 | 11.72 | 11.67 | 2R42 |
| 807.249 | 8.98 | 8.97 | 2R59, 2R83 | | 813.214 | 11.82 | 11.77 | 2R13 |
| 778.209 | 9.003 | 8.97 | 2R04, 2R54, 2R76 | | 785.207 | 11.86 | 11.83 | 2R29 |
| 770.15 | 9.033 | 8.97 | 2R02 | | 791.229 | 11.867 | 11.83 | 2R11, 2R55 |
| 793.245 | 9.05 | 9.01 | 2R81 | | 811.223 | 11.983 | 11.97 | 2R35 |
| 775.223 | 9.057 | 9.01 | 2R25, 2R49 | | 789.188 | 12.067 | 12.03 | 2R10, 2R33 |
| 792.224 | 9.09 | 9.01 | 2R12, 2R56 | | 832.181 | 12.117 | 12.09 | 2R18, 2R64 |
| 791.229 | 9.1 | 9.07 | 2R11, 2R55 | | 831.185 | 12.12 | 12.09 | 2R17, 2R63 |

(continued)

| [Table EJ12] | | | | | | | | |
|---|---|---|---|---|---|---|---|---|
| Mixture No. | mixEJ12 | | | | | | | |
| m/z [M+H]+ | Retention time of MS peak (min) | Retention time of UV peak (min) | No. of assigned m/z compound | | [M+H]+ | Retention time of MS peak (min) | Retention time of UV peak (min) | No. of assigned compound |
| 771.228 | 9.107 | 9.07 | 2R69 | | 897.207 | 12.297 | 12.27 | 2R21 |
| 774.214 | 9.183 | 9.15 | 2R96 | | 829.195 | 12.317 | 12.27 | 2R39 |
| 773.219 | 9.19 | 9.15 | 2R95 | | 855.185 | 12.57 | 12.54 | 2R19 |
| 777.214 | 9.193 | 9.15 | 2R03, 2R53, 2R75 | | 819.26 | 12.607 | 12.54 | 2R15 |
| 808.244 | 9.263 | 9.23 | 2R60, 2R84 | | 853.195 | 12.76 | 12.73 | 2R41 |
| 776.218 | 9.267 | 9.23 | 2R26, 2R50 | | 817.27 | 12.79 | 12.73 | 2R37,2R87 |

Example 4-5-1: Synthesis of mixture mixAI-B2Q-01

[Formula 546]

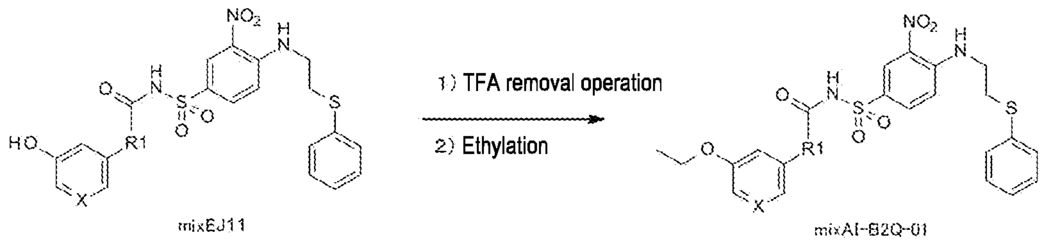


mixEJ11 mixAI-B2Q-01

DMI (1 mL) and fluorobenzene (30 μL as an internal standard) were added to mixEJ11 (35.3 μmol, putative), and the mixture was completely dissolved by vortex stirring. This solution was transferred to a syringe column packed with solid-phase supported morpholine (Merck KGaA, catalog No. 493813, morpholine, polymer-bound, 200-400 mesh, extent of labeling: 2.75-3.25 mmol/g loading, 1% cross-linked, 300 mg), thoroughly washed twice with MeCN (250 μL), and then left standing for 10 minutes. The filtrate was recovered under pressure into a test tube from below the column. The solid-phase supported morpholine was washed three times with MeCN (1 mL) and filtered under pressure, and all the filtrates were combined and collected. The solvent in the collected solution was distilled off under reduced pressure by Genevac (40°C, reduced pressure at 50-200 mbar until the completion of distilling off of a low-boiling solvent was detected, and then, 40°C, <0.5 mbar for 16 hours). DMI (500 μL) was added to the residue, which was then dissolved by stirring. Then, the solution was transferred to a glass vial and thoroughly washed twice with DMI (250 μL). The solvent in the obtained solution was distilled off under reduced pressure by Genevac (40°C, reduced pressure at 50-200 mbar until the completion of distilling off of a low-boiling solvent was detected, and then, 40°C, <0.5 mbar for 16 hours) to obtain a concentrate for use in the subsequent ethylation.

Under a nitrogen atmosphere, DMI (550 μL), BTPP (151 μL), and $Et_3PO_4$ (151 μL) were added to the glass vial containing the obtained concentrate (31.9 μmol, putative), and the mixture was stirred at 80°C for 30 hours. After cooling to room temperature, the reaction solution was adsorbed to a silica gel column with carboxylic acid supported thereon (ISOLUTE(R) CBA SPE column manufactured by Biotage Japan Ltd., 1 g/6 mL, 0.7 meq/g, product No: 520-0100-C) and left standing for 10 minutes. MeCN (1 mL) was added thereto, and the filtrate was recovered under pressure into a test tube. The silica gel with carboxylic acid supported thereon was washed twice with MeCN (1 mL) and filtered under

pressure to collect a filtrate. After addition of DMSO (1 mL), the solvent in the mixture was distilled off under reduced pressure by Genevac (40°C, reduced pressure at 50-200 mbar until the completion of distilling off of a low-boiling solvent was detected, and then, 40°C, <0.5 mbar for 16 hours) to obtain the title mixture mixAI-B2Q-01 (25.0 mg) shown in Table AI-03- B2Q-01 as an oil substance.

Example 4-5-2: Implementation of TFA removal and ethylation for other 11 types of mixtures

The same operation as in Example 4-5-1 was also carried out as to mixtures mixEJ01 to mixEJ10 and mixEJ12 shown in Table EIJ01 to Table EIJ10 and Table EIJ12 to obtain the mixtures shown in Table AI-03-B4J-01, Table AI-03-B1M-01, Table AI-03-B1K- 01, Table AI-03-B10-01, Table AI-03-B1A-01, Table AI-03-B3A-01, Table AI-03-B2I-01, Table AI-03-B2L-01, Table AI-03-B2N-01, Table AI-03-B2A-01, and Table AI-03-B2R-01.
The mixture numbers of the starting materials used, the mixture numbers of the compounds of interest obtained from the individual starting materials, the numbers of compounds that may be contained in the mixtures of the compounds of interest, and the weights of the obtained mixtures are shown in Table AI-03-01.
[Table 238]

| [Table AI-03-01] | | | |
|---|---|---|---|
| Table AI-03-01 | | | |
| Mixture No. of starting material | Mixture No. of target compound | No. of compound that may be contained in mixture of target compound | Weight of obtained mixture (mg) |
| mixEJ01 | mixAI-B4J-01 | B4J001-01 to B4J100-01 | 21.9 |
| mixEJ02 | mixAI-B1M-01 | B1M001-01 to B1M100-01 | 22.2 |
| mixEJ03 | mixAI-B1K-01 | B1K001-01 to B1K100-01 | 24.3 |
| mixEJ04 | mixAI-B1O-01 | B1O001-01 to B1O100-01 | 22.3 |
| mixEJ05 | mixAI-B1A-01 | B1A001-01 to B1A100-01 | 18.3 |
| mixEJ06 | mixAI-B3A-01 | B3A001-01 to B3A100-01 | 27.5 |
| mixEJ07 | mixAI-B2I-01 | B2I001-01 to B2I100-01 | 30.9 |
| mixEJ08 | mixAI-B2L-01 | B2L001-01 to B2L100-01 | 28.1 |
| mixEJ09 | | mixAI-B2N-01 B2N001-01 to B2N100-01 | 25.6 |
| mixEJ10 | | mixAI-B2A-01 B2A001-01 to B2A100-01 | 23.3 |
| mixEJ11 | | mixAI-B2Q-01 B2Q001-01 to B2Q100-01 | 25.0 |
| mixEJ12 | | mixAI-B2R-01 B2R001-01 to B2R100-01 | 28.2 |

The compounds that may be contained in each mixture of the compounds of interest are shown in each table given below. In the table, ×, A, a, B, b, C, c, and D represent a core block, a linker and a terminal group (×) of a compound represented by the following formula:

[Formula 547]

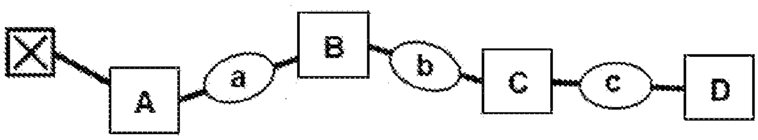


each of which is as described in Figure 2. For example, when the label is B2Q001-01, the core block, the linker and the terminal group are as shown in the table given below, and mean a compound represented by the following structural formula.
[

Table 239]

| Label | Exact | | A | a | B | b | C | c | D |
|---|---|---|---|---|---|---|---|---|---|
| B2Q001-01 | 773.18 | OEt | A01 | a1 | B10 | b3 | C02 | c2 | D11 |

[Formula 548]

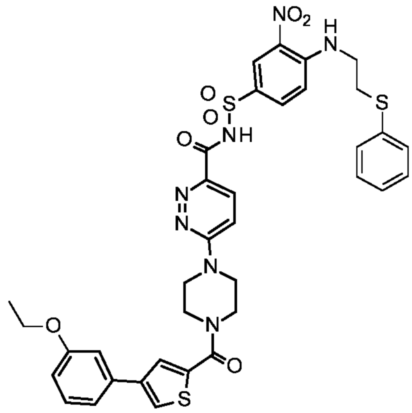

[Table 240]

[Table AI-03-B2Q-01]

| Mixture No. | mixAI-B2Q-01 |
|---|---|
| No. of compound that may be contained | Compound name |
| B2Q001-01 | 6-[4-[4-(3-Ethoxyphenyl)thiophene-2-carbonyl]piperazin-1-yl]-N-[3-nitro-4-(2-phenylsulfanylethylamino)phenyl]sulfonylpyridazine-3-carboxamide |
| B2Q002-01 | 6-[4-[4-(5-Ethoxypyridin-3-yl)thiophene-2-carbonyl]piperazin-1-yl]-N-[3-nitro-4-(2-phenylsulfanylethylamino)phenyl]sulfonylpyridazine-3-carboxamide |

| | |
|---|---|
| B2Q003-01 | 6-[4-[4-(3-Ethoxyphenyl)-3-methylbenzoyl]piperazin-1-yl]-N-[3-nitro-4-(2-phenylsulfanylethylamino)phenyl]sulfonylpyridazine-3-carboxamide |
| B2Q004-01 | 6-[4-[4-(5-Ethoxypyridin-3-yl)-3-methylbenzoyl]piperazin-1-yl]-N-[3-nitro-4-(2-phenylsulfanylethylamino)phenyl]sulfonylpyridazine-3-carboxamide |
| B2Q005-01 | 6-[4-[4-(3-Ethoxyphenyl)-3-fluorobenzoyl]piperazin-1-yl]-N-[3-nitro-4-(2-phenylsulfanylethylamino)phenyl]sulfonylpyridazine-3-carboxamide |
| B2Q006-01 | 6-[4-[4-(5-Ethoxypyridin-3-yl)-3-fluorobenzoyl]piperazin-1-yl]-N-[3-nitro-4-(2-phenylsulfanylethylamino)phenyl]sulfonylpyridazine-3-carboxamide |
| B2Q007-01 | 6-[4-[2-[2-(3-Ethoxyphenyl)ethynyl]benzoyl]piperazin-1-yl]-N-[3-nitro-4-(2-phenylsulfanylethylamino)phenyl]sulfonylpyridazine-3-carboxamide |
| B2Q008-01 | 6-[4-[2-[2-(5-Ethoxypyridin-3-yl)ethynyl]benzoyl]piperazin-1-yl]-N-[3-nitro-4-(2-phenylsulfanylethylamino)phenyl]sulfonylpyridazine-3-carboxamide |
| B2Q009-01 | 6-[4-[5-[2-(3-Ethoxyphenyl)ethynyl]pyridine-3-carbonyl]piperazin-1-yl]-N-[3-nitro-4-(2-phenylsulfanylethylamino)phenyl]sulfonylpyridazine-3-carboxamide |
| B2Q010-01 | 6-[4-[5-[2-(5-Ethoxypyridin-3-yl)ethynyl]pyridine-3-carbonyl]piperazin-1-yl]-N-[3-nitro-4-(2-phenylsulfanylethylamino)phenyl]sulfonylpyridazine-3-carboxamide |
| B2Q011-01 | 6-[4-[3-[2-(3-Ethoxyphenyl)phenyl]propanoyl]piperazin-1-yl]-N-[3-nitro-4-(2-phenylsulfanylethylamino)phenyl]sulfonylpyridazine-3-carboxamide |
| B2Q012-01 | 6-[4-[3-[2-(5-Ethoxypyridin-3-yl)phenyl]propanoyl]piperazin-1-yl]-N-[3-nitro-4-(2-phenylsulfanylethylamino)phenyl]sulfonylpyridazine-3-carboxamide |
| B2Q013-01 | 6-[4-[4-(3-Ethoxyphenyl)naphthalene-1-carbonyl]piperazin-1-yl]-N-[3-nitro-4-(2-phenylsulfanylethylamino)phenyl]sulfonylpyridazine-3-carboxamide |
| B2Q014-01 | 6-[4-[4-(5-Ethoxypyridin-3-yl)naphthalene-1-carbonyl]piperazin-1-yl]-N-[3-nitro-4-(2-phenylsulfanylethylamino)phenyl]sulfonylpyridazine-3-carboxamide |
| B2Q015-01 | 6-[4-[3-tert-Butyl-5-(3-ethoxyphenyl)benzoyl]piperazin-1-yl]-N-[3-nitro-4-(2-phenylsulfanylethylamino)phenyl]sulfonylpyridazine-3-carboxamide |
| B2Q016-01 | 6-[4-[3-tert-Butyl-5-(5-ethoxypyridin-3-yl)benzoyl]piperazin-1-yl]-N-[3-nitro-4-(2-phenylsulfanylethylamino)phenyl]sulfonylpyridazine-3-carboxamide |
| B2Q017-01 | 6-[4-[3-(3-Ethoxyphenyl)-5-(trifluoromethyl)benzoyl]piperazin-1-yl]-N-[3-nitro-4-(2-phenylsulfanylethylamino)phenyl]sulfonylpyridazine-3-carboxamide |
| B2Q018-01 | 6-[4-[3-(5-Ethoxypyridin-3-yl)-5-(trifluoromethyl)benzoyl]piperazin-1-yl]-N-[3-nitro-4-(2-phenylsulfanylethylamino)phenyl]sulfonylpyridazine-3-carboxamide |
| B2Q019-01 | 6-[4-[3-[2-(3-Ethoxyphenyl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| B2Q020-01 | 6-[4-[3-[2-(5-Ethoxypyridin-3-yl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| B2Q021-01 | 6-[4-[3-[4-(3-Ethoxyphenyl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| B2Q022-01 | 6-[4-[3-[4-(5-Ethoxypyridin-3-yl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| B2Q023-01 | 4-[4-[4-(3-Ethoxyphenyl)thiophene-2-carbonyl]piperazin-1-yl]-N-[3-nitro-4-(2-phenylsulfanylethylamino)phenyl]sulfonylbenzamide |
| B2Q024-01 | 4-[4-[4-(5-Ethoxypyridin-3-yl)thiophene-2-carbonyl]piperazin-1-yl]-N-[3-nitro-4-(2-phenylsulfanylethylamino)phenyl]sulfonylbenzamide |
| B2Q025-01 | 4-[4-[4-(3-Ethoxyphenyl)-3-methylbenzoyl]piperazin-1-yl]-N-[3-nitro-4-(2-phenylsulfanylethylamino)phenyl]sulfonylbenzamide |
| B2Q026-01 | 4-[4-[4-(5-Ethoxypyridin-3-yl)-3-methylbenzoyl]piperazin-1-yl]-N-[3-nitro-4-(2-phenylsulfanylethylamino)phenyl]sulfonylbenzamide |
| B2Q027-01 | 4-[4-[4-(3-Ethoxyphenyl)-3-fluorobenzoyl]piperazin-1-yl]-N-[3-nitro-4-(2-phenylsulfanylethylamino)phenyl]sulfonylbenzamide |
| B2Q028-01 | 4-[4-[4-(5-Ethoxypyridin-3-yl)-3-fluorobenzoyl]piperazin-1-yl]-N-[3-nitro-4-(2-phenylsulfanylethylamino)phenyl]sulfonylbenzamide |
| B2Q029-01 | 4-[4-[2-[2-(3-Ethoxyphenyl)ethynyl]benzoyl]piperazin-1-yl]-N-[3-nitro-4-(2-phenylsulfanylethylamino)phenyl]sulfonylbenzamide |

| | |
|---|---|
| B2Q030-01 | 4-[4-[2-[2-(5-Ethoxypyridin-3-yl)ethynyl]benzoyl]piperazin-1-yl]-N-[3-nitro-4-(2-phenylsulfanylethylamino)phenyl]sulfonylbenzamide |
| B2Q031-01 | 4-[4-[5-[2-(3-Ethoxyphenyl)ethynyl]pyridine-3-carbonyl]piperazin-1-yl]-N-[3-nitro-4-(2-phenylsulfanylethylamino)phenyl]sulfonylbenzamide |
| B2Q032-01 | 4-[4-[5-[2-(5-Ethoxypyridin-3-yl)ethynyl]pyridine-3-carbonyl]piperazin-1-yl]-N-[3-nitro-4-(2-phenylsulfanylethylamino)phenyl]sulfonylbenzamide |
| B2Q033-01 | 4-[4-[3-[2-(3-Ethoxyphenyl)phenyl]propanoyl]piperazin-1-yl]-N-[3-nitro-4-(2-phenylsulfanylethylamino)phenyl]sulfonylbenzamide |
| B2Q034-01 | 4-[4-[3-[2-(5-Ethoxypyridin-3-yl)phenyl]propanoyl]piperazin-1-yl]-N-[3-nitro-4-(2-phenylsulfanylethylamino)phenyl]sulfonylbenzamide |
| B2Q035-01 | 4-[4-[4-(3-Ethoxyphenyl)naphthalene-1-carbonyl]piperazin-1-yl]-N-[3-nitro-4-(2-phenylsulfanylethylamino)phenyl]sulfonylbenzamide |
| B2Q036-01 | 4-[4-[4-(5-Ethoxypyridin-3-yl)naphthalene-1-carbonyl]piperazin-1-yl]-N-[3-nitro-4-(2-phenylsulfanylethylamino)phenyl]sulfonylbenzamide |
| B2Q037-01 | 4-[4-[3-tert-Butyl-5-(3-ethoxyphenyl)benzoyl]piperazin-1-yl]-N-[3-nitro-4-(2-phenylsulfanylethylamino)phenyl]sulfonylbenzamide |
| B2Q038-01 | 4-[4-[3-tert-Butyl-5-(5-ethoxypyridin-3-yl)benzoyl]piperazin-1-yl]-N-[3-nitro-4-(2-phenylsulfanylethylamino)phenyl]sulfonylbenzamide |
| B2Q039-01 | 4-[4-[3-(3-Ethoxyphenyl)-5-(trifluoromethyl)benzoyl]piperazin-1-yl]-N-[3-nitro-4-(2-phenylsulfanylethylamino)phenyl]sulfonylbenzamide |
| B2Q040-01 | 4-[4-[3-(5-Ethoxypyridin-3-yl)-5-(trifluoromethyl)benzoyl]piperazin-1-yl]-N-[3-nitro-4-(2-phenylsulfanylethylamino)phenyl]sulfonylbenzamide |
| B2Q041-01 | 4-[4-[3-[2-(3-Ethoxyphenyl)ethynyl]-5-(trifluoromethyl)benzoyl]piperazin-1-yl]-N-[3-nitro-4-(2-phenylsulfanylethylamino)phenyl]sulfonylbenzamide |
| B2Q042-01 | 4-[4-[3-[2-(5-Ethoxypyridin-3-yl)ethynyl]-5-(trifluoromethyl)benzoyl]piperazin-1-yl]-N-[3-nitro-4-(2-phenylsulfanylethylamino)phenyl]sulfonylbenzamide |
| B2Q043-01 | 4-[4-[3-[4-(3-Ethoxyphenyl)pyrazol-1-yl]-5-(trifluoromethyl)benzoyl]piperazin-1-yl]-N-[3-nitro-4-(2-phenylsulfanylethylamino)phenyl]sulfonylbenzamide |
| B2Q044-01 | 4-[4-[3-[4-(5-Ethoxypyridin-3-yl)pyrazol-1-yl]-5-(trifluoromethyl)benzoyl]piperazin-1-yl]-N-[3-nitro-4-(2-phenylsulfanylethylamino)phenyl]sulfonylbenzamide |
| B2Q045-01 | 4-[4-[[2-(3-Ethoxyphenyl)phenyl]methyl]piperazin-1-yl]-N-[3-nitro-4-(2-phenylsulfanylethylamino)phenyl]sulfonylbenzamide |
| B2Q046-01 | 4-[4-[[2-(5-Ethoxypyridin-3-yl)phenyl]methyl]piperazin-1-yl]-N-[3-nitro-4-(2-phenylsulfanylethylamino)phenyl]sulfonylbenzamide |
| B2Q047-01 | 4-[4-[[4-(3-Ethoxyphenyl)-3-methylphenyl]methyl]piperazin-1-yl]-N-[3-nitro-4-(2-phenylsulfanylethylamino)phenyl]sulfonylbenzamide |
| B2Q048-01 | 4-[4-[[4-(5-Ethoxypyridin-3-yl)-3-methylphenyl]methyl]piperazin-1-yl]-N-[3-nitro-4-(2-phenylsulfanylethylamino)phenyl]sulfonylbenzamide |
| B2Q049-01 | 4-[4-[[4-(3-Ethoxyphenyl)-2-ethylphenyl]methyl]piperazin-1-yl]-N-[3-nitro-4-(2-phenylsulfanylethylamino)phenyl]sulfonylbenzamide |
| B2Q050-01 | 4-[4-[[4-(5-Ethoxypyridin-3-yl)-2-ethylphenyl]methyl]piperazin-1-yl]-N-[3-nitro-4-(2-phenylsulfanylethylamino)phenyl]sulfonylbenzamide |
| B2Q051-01 | 4-[4-[[4-(3-Ethoxyphenyl)-3-fluorophenyl]methyl]piperazin-1-yl]-N-[3-nitro-4-(2-phenylsulfanylethylamino)phenyl]sulfonylbenzamide |
| B2Q052-01 | 4-[4-[[4-(5-Ethoxypyridin-3-yl)-3-fluorophenyl]methyl]piperazin-1-yl]-N-[3-nitro-4-(2-phenylsulfanylethylamino)phenyl]sulfonylbenzamide |
| B2Q053-01 | 4-[4-[[4-(3-Ethoxyphenyl)-2-methoxyphenyl]methyl]piperazin-1-yl]-N-[3-nitro-4-(2-phenylsulfanylethylamino)phenyl]sulfonylbenzamide |
| B2Q054-01 | 4-[4-[[4-(5-Ethoxypyridin-3-yl)-2-methoxyphenyl]methyl]piperazin-1-yl]-N-[3-nitro-4-(2-phenylsulfanylethylamino)phenyl]sulfonylbenzamide |
| B2Q055-01 | 4-[4-[[3-Ethoxy-5-(3-ethoxyphenyl)phenyl]methyl]piperazin-1-yl]-N-[3-nitro-4-(2-phenylsulfanylethylamino)phenyl]sulfonylbenzamide |
| B2Q056-01 | 4-[4-[[3-Ethoxy-5-(5-ethoxypyridin-3-yl)phenyl]methyl]piperazin-1-yl]-N-[3-nitro-4-(2-phenylsulfanylethylamino)phenyl]sulfonylbenzamide |
| B2Q057-01 | 4-[4-[[3-(3-Ethoxyphenyl)-5-propan-2-yloxyphenyl]methyl]piperazin-1-yl]-N-[3-nitro-4-(2-phenylsulfanylethylamino)phenyl]sulfonylbenzamide |

| | |
|---|---|
| B2Q058-01 | 4-[4-[[3-(5-Ethoxypyridin-3-yl)-5-propan-2-yloxyphenyl]methyl]piperazin-1-yl]-N-[3-nitro-4-(2-phenylsulfanylethylamino)phenyl]sulfonylbenzamide |
| B2Q059-01 | 4-[4-[[4-(3-Ethoxyphenyl)-2,6-dimethoxyphenyl]methyl]piperazin-1-yl]-N-[3-nitro-4-(2-phenylsulfanylethylamino)phenyl]sulfonylbenzamide |
| B2Q060-01 | 4-[4-[[4-(5-Ethoxypyridin-3-yl)-2,6-dimethoxyphenyl]methyl]piperazin-1-yl]-N-[3-nitro-4-(2-phenylsulfanylethylamino)phenyl]sulfonylbenzamide |
| B2Q061-01 | 4-[4-[[3-(3-Ethoxyphenyl)-5-(trifluoromethyl)phenyl]methyl]piperazin-1-yl]-N-[3-nitro-4-(2-phenylsulfanylethylamino)phenyl]sulfonylbenzamide |
| B2Q062-01 | 4-[4-[[3-(5-Ethoxypyridin-3-yl)-5-(trifluoromethyl)phenyl]methyl]piperazin-1-yl]-N-[3-nitro-4-(2-phenylsulfanylethylamino)phenyl]sulfonylbenzamide |
| B2Q063-01 | 4-[4-[[3-(3-Ethoxyphenyl)-5-(trifluoromethoxy)phenyl]methyl]piperazin-1-yl]-N-[3-nitro-4-(2-phenylsulfanylethylamino)phenyl]sulfonylbenzamide |
| B2Q064-01 | 4-[4-[[3-(5-Ethoxypyridin-3-yl)-5-(trifluoromethoxy)phenyl]methyl]piperazin-1-yl]-N-[3-nitro-4-(2-phenylsulfanylethylamino)phenyl]sulfonylbenzamide |
| B2Q065-01 | 4-[4-[[4-(3-Ethoxyphenyl)naphthalen-1-yl]methyl]piperazin-1-yl]-N-[3-nitro-4-(2-phenylsulfanylethylamino)phenyl]sulfonylbenzamide |
| B2Q066-01 | 4-[4-[[4-(5-Ethoxypyridin-3-yl)naphthalen-1-yl]methyl]piperazin-1-yl]-N-[3-nitro-4-(2-phenylsulfanylethylamino)phenyl]sulfonylbenzamide |
| B2Q067-01 | 4-[4-[[4-(3-Ethoxyphenyl)thiophen-2-yl]methyl]piperazin-1-yl]-N-[3-nitro-4-(2-phenylsulfanylethylamino)phenyl]sulfonylbenzamide |
| B2Q068-01 | 4-[4-[[4-(5-Ethoxypyridin-3-yl)thiophen-2-yl]methyl]piperazin-1-yl]-N-[3-nitro-4-(2-phenylsulfanylethylamino)phenyl]sulfonylbenzamide |
| B2Q069-01 | 4-[4-[[2-[2-(3-Ethoxyphenyl)ethynyl]phenyl]methyl]piperazin-1-yl]-N-[3-nitro-4-(2-phenylsulfanylethylamino)phenyl]sulfonylbenzamide |
| B2Q070-01 | 4-[4-[[2-[2-(5-Ethoxypyridin-3-yl)ethynyl]phenyl]methyl]piperazin-1-yl]-N-[3-nitro-4-(2-phenylsulfanylethylamino)phenyl]sulfonylbenzamide |
| B2Q071-01 | 6-[4-[[2-(3-Ethoxyphenyl)phenyl]methyl]piperazin-1-yl]-N-[3-nitro-4-(2-phenylsulfanylethylamino)phenyl]sulfonylpyridazine-3-carboxamide |
| B2Q072-01 | 6-[4-[[2-(5-Ethoxypyridin-3-yl)phenyl]methyl]piperazin-1-yl]-N-[3-nitro-4-(2-phenylsulfanylethylamino)phenyl]sulfonylpyridazine-3-carboxamide |
| B2Q073-01 | 6-[4-[[4-(3-Ethoxyphenyl)-3-methylphenyl]methyl]piperazin-1-yl]-N-[3-nitro-4-(2-phenylsulfanylethylamino)phenyl]sulfonylpyridazine-3-carboxamide |
| B2Q074-01 | 6-[4-[[4-(5-Ethoxypyridin-3-yl)-3-methylphenyl]methyl]piperazin-1-yl]-N-[3-nitro-4-(2-phenylsulfanylethylamino)phenyl]sulfonylpyridazine-3-carboxamide |
| B2Q075-01 | 6-[4-[[4-(3-Ethoxyphenyl)-2-ethylphenyl]methyl]piperazin-1-yl]-N-[3-nitro-4-(2-phenylsulfanylethylamino)phenyl]sulfonylpyridazine-3-carboxamide |
| B2Q076-01 | 6-[4-[[4-(5-Ethoxypyridin-3-yl)-2-ethylphenyl]methyl]piperazin-1-yl]-N-[3-nitro-4-(2-phenylsulfanylethylamino)phenyl]sulfonylpyridazine-3-carboxamide |
| B2Q077-01 | 6-[4-[[4-(3-Ethoxyphenyl)-3-fluorophenyl]methyl]piperazin-1-yl]-N-[3-nitro-4-(2-phenylsulfanylethylamino)phenyl]sulfonylpyridazine-3-carboxamide |
| B2Q078-01 | 6-[4-[[4-(5-Ethoxypyridin-3-yl)-3-fluorophenyl]methyl]piperazin-1-yl]-N-[3-nitro-4-(2-phenylsulfanylethylamino)phenyl]sulfonylpyridazine-3-carboxamide |
| B2Q079-01 | 6-[4-[[4-(3-Ethoxyphenyl)-2-methoxyphenyl]methyl]piperazin-1-yl]-N-[3-nitro-4-(2-phenylsulfanylethylamino)phenyl]sulfonylpyridazine-3-carboxamide |
| B2Q080-01 | 6-[4-[[4-(5-Ethoxypyridin-3-yl)-2-methoxyphenyl]methyl]piperazin-1-yl]-N-[3-nitro-4-(2-phenylsulfanylethylamino)phenyl]sulfonylpyridazine-3-carboxamide |
| B2Q081-01 | 6-[4-[[3-Ethoxy-5-(3-ethoxyphenyl)phenyl]methyl]piperazin-1-yl]-N-[3-nitro-4-(2-phenylsulfanylethylamino)phenyl]sulfonylpyridazine-3-carboxamide |
| B2Q082-01 | 6-[4-[[3-Ethoxy-5-(5-ethoxypyridin-3-yl)phenyl]methyl]piperazin-1-yl]-N-[3-nitro-4-(2-phenylsulfanylethylamino)phenyl]sulfonylpyridazine-3-carboxamide |
| B2Q083-01 | 6-[4-[[3-(3-Ethoxyphenyl)-5-propan-2-yloxyphenyl]methyl]piperazin-1-yl]-N-[3-nitro-4-(2-phenylsulfanylethylamino)phenyl]sulfonylpyridazine-3-carboxamide |
| B2Q084-01 | 6-[4-[[3-(5-Ethoxypyridin-3-yl)-5-propan-2-yloxyphenyl]methyl]piperazin-1-yl]-N-[3-nitro-4-(2-phenylsulfanylethylamino)phenyl]sulfonylpyridazine-3-carboxamide |
| B2Q085-01 | 6-[4-[[4-(3-Ethoxyphenyl)-2,6-dimethoxyphenyl]methyl]piperazin-1-yl]-N-[3-nitro-4-(2-phenylsulfanylethylamino)phenyl]sulfonylpyridazine-3-carboxamide |

| | |
|---|---|
| B2Q086-01 | 6-[4-[[4-(5-Ethoxypyridin-3-yl)-2,6-dimethoxyphenyl]methyl]piperazin-1-yl]-N-[3-nitro-4-(2-phenylsulfanylethylamino)phenyl]sulfonylpyridazine-3-carboxamide |
| B2Q087-01 | 6-[4-[[3-(3-Ethoxyphenyl)-5-(trifluoromethyl)phenyl]methyl]piperazin-1-yl]-N-[3-nitro-4-(2-phenylsulfanylethylamino)phenyl]sulfonylpyridazine-3-carboxamide |
| B2Q088-01 | 6-[4-[[3-(5-Ethoxypyridin-3-yl)-5-(trifluoromethyl)phenyl]methyl]piperazin-1-yl]-N-[3-nitro-4-(2-phenylsulfanylethylamino)phenyl]sulfonylpyridazine-3-carboxamide |
| B2Q089-01 | 6-[4-[[3-(3-Ethoxyphenyl)-5-(trifluoromethoxy)phenyl]methyl]piperazin-1-yl]-N-[3-nitro-4-(2-phenylsulfanylethylamino)phenyl]sulfonylpyridazine-3-carboxamide |
| B2Q090-01 | 6-[4-[[3-(5-Ethoxypyridin-3-yl)-5-(trifluoromethoxy)phenyl]methyl]piperazin-1-yl]-N-[3-nitro-4-(2-phenylsulfanylethylamino)phenyl]sulfonylpyridazine-3-carboxamide |
| B2Q091-01 | 6-[4-[[4-(3-Ethoxyphenyl)naphthalen-1-yl]methyl]piperazin-1-yl]-N-[3-nitro-4-(2-phenylsulfanylethylamino)phenyl]sulfonylpyridazine-3-carboxamide |
| B2Q092-01 | 6-[4-[[4-(5-Ethoxypyridin-3-yl)naphthalen-1-yl]methyl]piperazin-1-yl]-N-[3-nitro-4-(2-phenylsulfanylethylamino)phenyl]sulfonylpyridazine-3-carboxamide |
| B2Q093-01 | 6-[4-[[4-(3-Ethoxyphenyl)thiophen-2-yl]methyl]piperazin-1-yl]-N-[3-nitro-4-(2-phenylsulfanylethylamino)phenyl]sulfonylpyridazine-3-carboxamide |
| B2Q094-01 | 6-[4-[[4-(5-Ethoxypyridin-3-yl)thiophen-2-yl]methyl]piperazin-1-yl]-N-[3-nitro-4-(2-phenylsulfanylethylamino)phenyl]sulfonylpyridazine-3-carboxamide |
| B2Q095-01 | 6-[4-[[2-[2-(3-Ethoxyphenyl)ethynyl]phenyl]methyl]piperazin-1-yl]-N-[3-nitro-4-(2-phenylsulfanylethylamino)phenyl]sulfonylpyridazine-3-carboxamide |
| B2Q096-01 | 6-[4-[[2-[2-(5-Ethoxypyridin-3-yl)ethynyl]phenyl]methyl]piperazin-1-yl]-N-[3-nitro-4-(2-phenylsulfanylethylamino)phenyl]sulfonylpyridazine-3-carboxamide |
| B2Q097-01 | 4-[4-[1-[4-(3-Ethoxyphenyl)phenyl]ethyl]piperazin-1-yl]-N-[3-nitro-4-(2-phenylsulfanylethylamino)phenyl]sulfonylbenzamide |
| B2Q098-01 | 4-[4-[1-[4-(5-Ethoxypyridin-3-yl)phenyl]ethyl]piperazin-1-yl]-N-[3-nitro-4-(2-phenylsulfanylethylamino)phenyl]sulfonylbenzamide |
| B2Q099-01 | 4-[4-[1-[4-(3-Ethoxyphenyl)-2-fluorophenyl]ethyl]piperazin-1-yl]-N-[3-nitro-4-(2-phenylsulfanylethylamino)phenyl]sulfonylbenzamide |
| B2Q100-01 | 4-[4-[1-[4-(5-Ethoxypyridin-3-yl)-2-fluorophenyl]ethyl]piperazin-1-yl]-N-[3-nitro-4-(2-phenylsulfanylethylamino)phenyl]sulfonylbenzamide |

[Table 241]

[Table AI-03-B2Q-01]

| Label | Exact | ☒ | A | a | B | b | C | c | D |
|---|---|---|---|---|---|---|---|---|---|
| B2Q001-01 | 773.18 | OEt | A01 | a1 | B10 | b3 | C02 | c2 | D11 |
| B2Q002-01 | 774.17 | OEt | A02 | a1 | B10 | b3 | C02 | c2 | D11 |
| B2Q003-01 | 781.24 | OEt | A01 | a1 | B04 | b3 | C02 | c2 | D11 |
| B2Q004-01 | 782.23 | OEt | A02 | a1 | B04 | b3 | C02 | c2 | D11 |
| B2Q005-01 | 785.21 | OEt | A01 | a1 | B06 | b3 | C02 | c2 | D11 |
| B2Q006-01 | 786.21 | OEt | A02 | a1 | B06 | b3 | C02 | c2 | D11 |
| B2Q007-01 | 791.22 | OEt | A01 | a2 | B01 | b3 | C02 | c2 | D11 |
| B2Q008-01 | 792.22 | OEt | A02 | a2 | B01 | b3 | C02 | c2 | D11 |
| B2Q009-01 | 792.22 | OEt | A01 | a2 | B17 | b3 | C02 | c2 | D11 |
| B2Q010-01 | 793.21 | OEt | A02 | a2 | B17 | b3 | C02 | c2 | D11 |
| B2Q011-01 | 795.25 | OEt | A01 | a1 | B02 | b3 | C02 | c2 | D11 |
| B2Q012-01 | 796.25 | OEt | A02 | a1 | B02 | b3 | C02 | c2 | D11 |
| B2Q013-01 | 817.24 | OEt | A01 | a1 | B09 | b3 | C02 | c2 | D11 |
| B2Q014-01 | 818.23 | OEt | A02 | a1 | B09 | b3 | C02 | c2 | D11 |
| B2Q015-01 | 823.28 | OEt | A01 | a1 | B16 | b3 | C02 | c2 | D11 |
| B2Q016-01 | 824.28 | OEt | A02 | a1 | B16 | b3 | C02 | c2 | D11 |
| B2Q017-01 | 835.21 | OEt | A01 | a1 | B11 | b3 | C02 | c2 | D11 |

| | | | | | | | | | |
|---|---|---|---|---|---|---|---|---|---|
| B2Q018-01 | 836.2 | OEt | A02 | a1 | B11 | b3 | C02 | c2 | D11 |
| B2Q019-01 | 859.21 | OEt | A01 | a2 | B11 | b3 | C02 | c2 | D11 |
| B2Q020-01 | 860.2 | OEt | A02 | a2 | B11 | b3 | C02 | c2 | D11 |
| B2Q021-01 | 901.23 | OEt | A01 | a1 | B12 | b3 | C02 | c2 | D11 |
| B2Q022-01 | 902.22 | OEt | A02 | a1 | B12 | b3 | C02 | c2 | D11 |
| B2Q023-01 | 771.19 | OEt | A01 | a1 | B10 | b3 | C01 | c2 | D11 |
| B2Q024-01 | 772.18 | OEt | A02 | a1 | B10 | b3 | C01 | c2 | D11 |
| B2Q025-01 | 779.25 | OEt | A01 | a1 | B04 | b3 | C01 | c2 | D11 |
| B2Q026-01 | 780.24 | OEt | A02 | a1 | B04 | b3 | C01 | c2 | D11 |
| B2Q027-01 | 783.22 | OEt | A01 | a1 | B06 | b3 | C01 | c2 | D11 |
| B2Q028-01 | 784.22 | OEt | A02 | a1 | B06 | b3 | C01 | c2 | D11 |
| B2Q029-01 | 789.23 | OEt | A01 | a2 | B01 | b3 | C01 | c2 | D11 |
| B2Q030-01 | 790.22 | OEt | A02 | a2 | B01 | b3 | C01 | c2 | D11 |
| B2Q031-01 | 790.22 | OEt | A01 | a2 | B17 | b3 | C01 | c2 | D11 |
| B2Q032-01 | 791.22 | OEt | A02 | a2 | B17 | b3 | C01 | c2 | D11 |
| B2Q033-01 | 793.26 | OEt | A01 | a1 | B02 | b3 | C01 | c2 | D11 |
| B2Q034-01 | 794.26 | OEt | A02 | a1 | B02 | b3 | C01 | c2 | D11 |
| B2Q035-01 | 815.25 | OEt | A01 | a1 | B09 | b3 | C01 | c2 | D11 |
| B2Q036-01 | 816.24 | OEt | A02 | a1 | B09 | b3 | C01 | c2 | D11 |
| B2Q037-01 | 821.29 | OEt | A01 | a1 | B16 | b3 | C01 | c2 | D11 |
| B2Q038-01 | 822.29 | OEt | A02 | a1 | B16 | b3 | C01 | c2 | D11 |
| B2Q039-01 | 833.22 | OEt | A01 | a1 | B11 | b3 | C01 | c2 | D11 |
| B2Q040-01 | 834.21 | OEt | A02 | a1 | B11 | b3 | C01 | c2 | D11 |
| B2Q041-01 | 857.22 | OEt | A01 | a2 | B11 | b3 | C01 | c2 | D11 |
| B2Q042-01 | 858.21 | OEt | A02 | a2 | B11 | b3 | C01 | c2 | D11 |
| B2Q043-01 | 899.24 | OEt | A01 | a1 | B12 | b3 | C01 | c2 | D11 |
| B2Q044-01 | 900.23 | OEt | A02 | a1 | B12 | b3 | C01 | c2 | D11 |
| B2Q045-01 | 751.25 | OEt | A01 | a1 | B01 | b1 | C01 | c2 | D11 |
| B2Q046-01 | 752.25 | OEt | A02 | a1 | B01 | b1 | C01 | c2 | D11 |
| B2Q047-01 | 765.27 | OEt | A01 | a1 | B04 | b1 | C01 | c2 | D11 |
| B2Q048-01 | 766.26 | OEt | A02 | a1 | B04 | b1 | C01 | c2 | D11 |
| B2Q049-01 | 779.28 | OEt | A01 | a1 | B05 | b1 | C01 | c2 | D11 |
| B2Q050-01 | 780.28 | OEt | A02 | a1 | B05 | b1 | C01 | c2 | D11 |
| B2Q051-01 | 769.24 | OEt | A01 | a1 | B06 | b1 | C01 | c2 | D11 |
| B2Q052-01 | 770.24 | OEt | A02 | a1 | B06 | b1 | C01 | c2 | D11 |
| B2Q053-01 | 781.26 | OEt | A01 | a1 | B07 | b1 | C01 | c2 | D11 |
| B2Q054-01 | 782.26 | OEt | A02 | a1 | B07 | b1 | C01 | c2 | D11 |
| B2Q055-01 | 795.28 | OEt | A01 | a1 | B14 | b1 | C01 | c2 | D11 |
| B2Q056-01 | 796.27 | OEt | A02 | a1 | B14 | b1 | C01 | c2 | D11 |
| B2Q057-01 | 809.29 | OEt | A01 | a1 | B15 | b1 | C01 | c2 | D11 |
| B2Q058-01 | 810.29 | OEt | A02 | a1 | B15 | b1 | C01 | c2 | D11 |
| B2Q059-01 | 811.27 | OEt | A01 | a1 | B08 | b1 | C01 | c2 | D11 |
| B2Q060-01 | 812.27 | OEt | A02 | a1 | B08 | b1 | C01 | c2 | D11 |
| B2Q061-01 | 819.24 | OEt | A01 | a1 | B11 | b1 | C01 | c2 | D11 |
| B2Q062-01 | 820.23 | OEt | A02 | a1 | B11 | b1 | C01 | c2 | D11 |
| B2Q063-01 | 835.23 | OEt | A01 | a1 | B13 | b1 | C01 | c2 | D11 |
| B2Q064-01 | 836.23 | OEt | A02 | a1 | B13 | b1 | C01 | c2 | D11 |

| | | | | | | | | | |
|---|---|---|---|---|---|---|---|---|---|
| B2Q065-01 | 801.27 | OEt | A01 | a1 | B09 | b1 | C01 | c2 | D11 |
| B2Q066-01 | 802.26 | OEt | A02 | a1 | B09 | b1 | C01 | c2 | D11 |
| B2Q067-01 | 757.21 | OEt | A01 | a1 | B10 | b1 | C01 | c2 | D11 |
| B2Q068-01 | 758.2 | OEt | A02 | a1 | B10 | b1 | C01 | c2 | D11 |
| B2Q069-01 | 775.25 | OEt | A01 | a2 | B01 | b1 | C01 | c2 | D11 |
| B2Q070-01 | 776.25 | OEt | A02 | a2 | B01 | b1 | C01 | c2 | D11 |
| B2Q071-01 | 753.24 | OEt | A01 | a1 | B01 | b1 | C02 | c2 | D11 |
| B2Q072-01 | 754.24 | OEt | A02 | a1 | B01 | b1 | C02 | c2 | D11 |
| B2Q073-01 | 767.26 | OEt | A01 | a1 | B04 | b1 | C02 | c2 | D11 |
| B2Q074-01 | 768.25 | OEt | A02 | a1 | B04 | b1 | C02 | c2 | D11 |
| B2Q075-01 | 781.27 | OEt | A01 | a1 | B05 | b1 | C02 | c2 | D11 |
| B2Q076-01 | 782.27 | OEt | A02 | a1 | B05 | b1 | C02 | c2 | D11 |
| B2Q077-01 | 771.23 | OEt | A01 | a1 | B06 | b1 | C02 | c2 | D11 |
| B2Q078-01 | 772.23 | OEt | A02 | a1 | B06 | b1 | C02 | c2 | D11 |
| B2Q079-01 | 783.25 | OEt | A01 | a1 | B07 | b1 | C02 | c2 | D11 |
| B2Q080-01 | 784.25 | OEt | A02 | a1 | B07 | b1 | C02 | c2 | D11 |
| B2Q081-01 | 797.27 | OEt | A01 | a1 | B14 | b1 | C02 | c2 | D11 |
| B2Q082-01 | 798.26 | OEt | A02 | a1 | B14 | b1 | C02 | c2 | D11 |
| B2Q083-01 | 811.28 | OEt | A01 | a1 | B15 | b1 | C02 | c2 | D11 |
| B2Q084-01 | 812.28 | OEt | A02 | a1 | B15 | b1 | C02 | c2 | D11 |
| B2Q085-01 | 813.26 | OEt | A01 | a1 | B08 | b1 | C02 | c2 | D11 |
| B2Q086-01 | 814.26 | OEt | A02 | a1 | B08 | b1 | C02 | c2 | D11 |
| B2Q087-01 | 821.23 | OEt | A01 | a1 | B11 | b1 | C02 | c2 | D11 |
| B2Q088-01 | 822.22 | OEt | A02 | a1 | B11 | b1 | C02 | c2 | D11 |
| B2Q089-01 | 837.22 | OEt | A01 | a1 | B13 | b1 | C02 | c2 | D11 |
| B2Q090-01 | 838.22 | OEt | A02 | a1 | B13 | b1 | C02 | c2 | D11 |
| B2Q091-01 | 803.26 | OEt | A01 | a1 | B09 | b1 | C02 | c2 | D11 |
| B2Q092-01 | 804.25 | OEt | A02 | a1 | B09 | b1 | C02 | c2 | D11 |
| B2Q093-01 | 759.2 | OEt | A01 | a1 | B10 | b1 | C02 | c2 | D11 |
| B2Q094-01 | 760.19 | OEt | A02 | a1 | B10 | b1 | C02 | c2 | D11 |
| B2Q095-01 | 777.24 | OEt | A01 | a2 | B01 | b1 | C02 | c2 | D11 |
| B2Q096-01 | 778.24 | OEt | A02 | a2 | B01 | b1 | C02 | c2 | D11 |
| B2Q097-01 | 765.27 | OEt | A01 | a1 | B03 | b2 | C01 | c2 | D11 |
| B2Q098-01 | 766.26 | OEt | A02 | a1 | B03 | b2 | C01 | c2 | D11 |
| B2Q099-01 | 783.26 | OEt | A01 | a1 | B18 | b2 | C01 | c2 | D11 |
| B2Q100-01 | 784.25 | OEt | A02 | a1 | B18 | b2 | C01 | c2 | D11 |

[Table 242]

[Table AI-03-B4J-01]

| Mixture No. | mixAI-B4J-01 |
| --- | --- |
| No. of compound that may be contained | Compound name |
| B4J001-01 | 6-[4-[4-(3-Ethoxyphenyl)thiophene-2-carbonyl]piperazin-1-yl]-N,N-diethylpyridazine-3-carboxamide |
| B4J002-01 | 6-[4-[4-(5-Ethoxypyridin-3-yl)thiophene-2-carbonyl]piperazin-1-yl]-N,N-diethylpyridazine-3-carboxamide |

| | |
|---|---|
| B4J003-01 | 6-[4-[4-(3-Ethoxyphenyl)-3-methylbenzoyl]piperazin-1-yl]-N,N-diethylpyridazine-3-carboxamide |
| B4J004-01 | 6-[4-[4-(5-Ethoxypyridin-3-yl)-3-methylbenzoyl]piperazin-1-yl]-N,N-diethylpyridazine-3-carboxamide |
| B4J005-01 | 6-[4-[4-(3-Ethoxyphenyl)-3-fluorobenzoyl]piperazin-1-yl]-N,N-diethylpyridazine-3-carboxamide |
| B4J006-01 | 6-[4-[4-(5-Ethoxypyridin-3-yl)-3-fluorobenzoyl]piperazin-1-yl]-N,N-diethylpyridazine-3-carboxamide |
| B4J007-01 | 6-[4-[2-[2-(3-Ethoxyphenyl)ethynyl]benzoyl]piperazin-1-yl]-N,N-diethylpyridazine-3-carboxamide |
| B4J008-01 | 6-[4-[2-[2-(5-Ethoxypyridin-3-yl)ethynyl]benzoyl]piperazin-1-yl]-N,N-diethylpyridazine-3-carboxamide |
| B4J009-01 | 6-[4-[5-[2-(3-Ethoxyphenyl)ethynyl]pyridine-3-carbonyl]piperazin-1-yl]-N,N-diethylpyridazine-3-carboxamide |
| B4J010-01 | 6-[4-[5-[2-(5-Ethoxypyridin-3-yl)ethynyl]pyridine-3-carbonyl]piperazin-1-yl]-N,N-diethylpyridazine-3-carboxamide |
| B4J011-01 | 6-[4-[3-[2-(3-Ethoxyphenyl)phenyl]propanoyl]piperazin-1-yl]-N,N-diethylpyridazine-3-carboxamide |
| B4J012-01 | 6-[4-[3-[2-(5-Ethoxypyridin-3-yl)phenyl]propanoyl]piperazin-1-yl]-N,N-diethylpyridazine-3-carboxamide |
| B4J013-01 | 6-[4-[4-(3-Ethoxyphenyl)naphthalene-1-carbonyl]piperazin-1-yl]-N,N-diethylpyridazine-3-carboxamide |
| B4J014-01 | 6-[4-[4-(5-Ethoxypyridin-3-yl)naphthalene-1-carbonyl]piperazin-1-yl]-N,N-diethylpyridazine-3-carboxamide |
| B4J015-01 | 6-[4-[3-tert-Butyl-5-(3-ethoxyphenyl)benzoyl]piperazin-1-yl]-N,N-diethylpyridazine-3-carboxamide |
| B4J016-01 | 6-[4-[3-tert-Butyl-5-(5-ethoxypyridin-3-yl)benzoyl]piperazin-1-yl]-N,N-diethylpyridazine-3-carboxamide |
| B4J017-01 | 6-[4-[3-(3-Ethoxyphenyl)-5-(trifluoromethyl)benzoyl]piperazin-1-yl]-N,N-diethylpyridazine-3-carboxamide |
| B4J018-01 | 6-[4-[3-(5-Ethoxypyridin-3-yl)-5-(trifluoromethyl)benzoyl]piperazin-1-yl]-N,N-diethylpyridazine-3-carboxamide |
| B4J019-01 | 6-[4-[3-[2-(3-Ethoxyphenyl)ethynyl]-5-(trifluoromethyl)benzoyl]piperazin-1-yl]-N,N-diethylpyridazine-3-carboxamide |
| B4J020-01 | 6-[4-[3-[2-(5-Ethoxypyridin-3-yl)ethynyl]-5-(trifluoromethyl)benzoyl]piperazin-1-yl]-N,N-diethylpyridazine-3-carboxamide |
| B4J021-01 | 6-[4-[3-[4-(3-Ethoxyphenyl)pyrazol-1-yl]-5-(trifluoromethyl)benzoyl]piperazin-1-yl]-N,N-diethylpyridazine-3-carboxamide |
| B4J022-01 | 6-[4-[3-[4-(5-Ethoxypyridin-3-yl)pyrazol-1-yl]-5-(trifluoromethyl)benzoyl]piperazin-1-yl]-N,N-diethylpyridazine-3-carboxamide |
| B4J023-01 | 4-[4-[4-(3-Ethoxyphenyl)thiophene-2-carbonyl]piperazin-1-yl]-N,N-diethylbenzamide |
| B4J024-01 | 4-[4-[4-(5-Ethoxypyridin-3-yl)thiophene-2-carbonyl]piperazin-1-yl]-N,N-diethylbenzamide |
| B4J025-01 | 4-[4-[4-(3-Ethoxyphenyl)-3-methylbenzoyl]piperazin-1-yl]-N,N-diethylbenzamide |
| B4J026-01 | 4-[4-[4-(5-Ethoxypyridin-3-yl)-3-methylbenzoyl]piperazin-1-yl]-N,N-diethylbenzamide |
| B4J027-01 | 4-[4-[4-(3-Ethoxyphenyl)-3-fluorobenzoyl]piperazin-1-yl]-N,N-diethylbenzamide |
| B4J028-01 | 4-[4-[4-(5-Ethoxypyridin-3-yl)-3-fluorobenzoyl]piperazin-1-yl]-N,N-diethylbenzamide |
| B4J029-01 | 4-[4-[2-[2-(3-Ethoxyphenyl)ethynyl]benzoyl]piperazin-1-yl]-N,N-diethylbenzamide |
| B4J030-01 | 4-[4-[2-[2-(5-Ethoxypyridin-3-yl)ethynyl]benzoyl]piperazin-1-yl]-N,N-diethylbenzamide |
| B4J031-01 | 4-[4-[5-[2-(3-Ethoxyphenyl)ethynyl]pyridine-3-carbonyl]piperazin-1-yl]-N,N-diethylbenzamide |
| B4J032-01 | 4-[4-[5-[2-(5-Ethoxypyridin-3-yl)ethynyl]pyridine-3-carbonyl]piperazin-1-yl]-N,N-diethylbenzamide |

| | |
|---|---|
| B4J033-01 | 4-[4-[3-[2-(3-Ethoxyphenyl)phenyl]propanoyl]piperazin-1-yl]-N,N-diethylbenzamide |
| B4J034-01 | 4-[4-[3-[2-(5-Ethoxypyridin-3-yl)phenyl]propanoyl]piperazin-1-yl]-N,N-diethylbenzamide |
| B4J035-01 | 4-[4-[4-(3-Ethoxyphenyl)naphthalene-1-carbonyl]piperazin-1-yl]-N,N-diethylbenzamide |
| B4J036-01 | 4-[4-[4-(5-Ethoxypyridin-3-yl)naphthalene-1-carbonyl]piperazin-1-yl]-N,N-diethylbenzamide |
| B4J037-01 | 4-[4-[3-tert-Butyl-5-(3-ethoxyphenyl)benzoyl]piperazin-1-yl]-N,N-diethylbenzamide |
| B4J038-01 | 4-[4-[3-tert-Butyl-5-(5-ethoxypyridin-3-yl)benzoyl]piperazin-1-yl]-N,N-diethylbenzamide |
| B4J039-01 | 4-[4-[3-(3-Ethoxyphenyl)-5-(trifluoromethyl)benzoyl]piperazin-1-yl]-N,N-diethylbenzamide |
| B4J040-01 | 4-[4-[3-(5-Ethoxypyridin-3-yl)-5-(trifluoromethyl)benzoyl]piperazin-1-yl]-N,N-diethylbenzamide |
| B4J041-01 | 4-[4-[3-[2-(3-Ethoxyphenyl)ethynyl]-5-(trifluoromethyl)benzoyl]piperazin-1-yl]-N,N-diethylbenzamide |
| B4J042-01 | 4-[4-[3-[2-(5-Ethoxypyridin-3-yl)ethynyl]-5-(trifluoromethyl)benzoyl]piperazin-1-yl]-N,N-diethylbenzamide |
| B4J043-01 | 4-[4-[3-[4-(3-Ethoxyphenyl)pyrazol-1-yl]-5-(trifluoromethyl)benzoyl]piperazin-1-yl]-N,N-diethylbenzamide |
| B4J044-01 | 4-[4-[3-[4-(5-Ethoxypyridin-3-yl)pyrazol-1-yl]-5-(trifluoromethyl)benzoyl]piperazin-1-yl]-N,N-diethylbenzamide |
| B4J045-01 | 4-[4-[[2-(3-Ethoxyphenyl)phenyl]methyl]piperazin-1-yl]-N,N-diethylbenzamide |
| B4J046-01 | 4-[4-[[2-(5-Ethoxypyridin-3-yl)phenyl]methyl]piperazin-1-yl]-N,N-diethylbenzamide |
| B4J047-01 | 4-[4-[[4-(3-Ethoxyphenyl)-3-methylphenyl]methyl]piperazin-1-yl]-N,N-diethylbenzamide |
| B4J048-01 | 4-[4-[[4-(5-Ethoxypyridin-3-yl)-3-methylphenyl]methyl]piperazin-1-yl]-N,N-diethylbenzamide |
| B4J049-01 | 4-[4-[[4-(3-Ethoxyphenyl)-2-ethylphenyl]methyl]piperazin-1-yl]-N,N-diethylbenzamide |
| B4J050-01 | 4-[4-[[4-(5-Ethoxypyridin-3-yl)-2-ethylphenyl]methyl]piperazin-1-yl]-N,N-diethylbenzamide |
| B4J051-01 | 4-[4-[[4-(3-Ethoxyphenyl)-3-fluorophenyl]methyl]piperazin-1-yl]-N,N-diethylbenzamide |
| B4J052-01 | 4-[4-[[4-(5-Ethoxypyridin-3-yl)-3-fluorophenyl]methyl]piperazin-1-yl]-N,N-diethylbenzamide |
| B4J053-01 | 4-[4-[[4-(3-Ethoxyphenyl)-2-methoxyphenyl]methyl]piperazin-1-yl]-N,N-diethylbenzamide |
| B4J054-01 | 4-[4-[[4-(5-Ethoxypyridin-3-yl)-2-methoxyphenyl]methyl]piperazin-1-yl]-N,N-diethylbenzamide |
| B4J055-01 | 4-[4-[[3-Ethoxy-5-(3-ethoxyphenyl)phenyl]methyl]piperazin-1-yl]-N,N-diethylbenzamide |
| B4J056-01 | 4-[4-[[3-Ethoxy-5-(5-ethoxypyridin-3-yl)phenyl]methyl]piperazin-1-yl]-N,N-diethylbenzamide |
| B4J057-01 | 4-[4-[[3-(3-Ethoxyphenyl)-5-propan-2-yloxyphenyl]methyl]piperazin-1-yl]-N,N-diethylbenzamide |
| B4J058-01 | 4-[4-[[3-(5-Ethoxypyridin-3-yl)-5-propan-2-yloxyphenyl]methyl]piperazin-1-yl]-N,N-diethylbenzamide |
| B4J059-01 | 4-[4-[[4-(3-Ethoxyphenyl)-2,6-dimethoxyphenyl]methyl]piperazin-1-yl]-N,N-diethylbenzamide |
| B4J060-01 | 4-[4-[[4-(5-Ethoxypyridin-3-yl)-2,6-dimethoxyphenyl]methyl]piperazin-1-yl]-N,N-diethylbenzamide |
| B4J061-01 | 4-[4-[[3-(3-Ethoxyphenyl)-5-(trifluoromethyl)phenyl]methyl]piperazin-1-yl]-N,N-diethylbenzamide |

| | |
|---|---|
| B4J062-01 | 4-[4-[[3-(5-Ethoxypyridin-3-yl)-5-(trifluoromethyl)phenyl]methyl]piperazin-1-yl]-N,N-diethylbenzamide |
| B4J063-01 | 4-[4-[[3-(3-Ethoxyphenyl)-5-(trifluoromethoxy)phenyl]methyl]piperazin-1-yl]-N,N-diethylbenzamide |
| B4J064-01 | 4-[4-[[3-(5-Ethoxypyridin-3-yl)-5-(trifluoromethoxy)phenyl]methyl]piperazin-1-yl]-N,N-diethylbenzamide |
| B4J065-01 | 4-[4-[[4-(3-Ethoxyphenyl)naphthalen-1-yl]methyl]piperazin-1-yl]-N,N-diethylbenzamide |
| B4J066-01 | 4-[4-[[4-(5-Ethoxypyridin-3-yl)naphthalen-1-yl]methyl]piperazin-1-yl]-N,N-diethylbenzamide |
| B4J067-01 | 4-[4-[[4-(3-Ethoxyphenyl)thiophen-2-yl]methyl]piperazin-1-yl]-N,N-diethylbenzamide |
| B4J068-01 | 4-[4-[[4-(5-Ethoxypyridin-3-yl)thiophen-2-yl]methyl]piperazin-1-yl]-N,N-diethylbenzamide |
| B4J069-01 | 4-[4-[[2-[2-(3-Ethoxyphenyl)ethynyl]phenyl]methyl]piperazin-1-yl]-N,N-diethylbenzamide |
| B4J070-01 | 4-[4-[[2-[2-(5-Ethoxypyridin-3-yl)ethynyl]phenyl]methyl]piperazin-1-yl]-N,N-diethylbenzamide |
| B4J071-01 | 6-[4-[[2-(3-Ethoxyphenyl)phenyl]methyl]piperazin-1-yl]-N,N-diethylpyridazine-3-carboxamide |
| B4J072-01 | 6-[4-[[2-(5-Ethoxypyridin-3-yl)phenyl]methyl]piperazin-1-yl]-N,N-diethylpyridazine-3-carboxamide |
| B4J073-01 | 6-[4-[[4-(3-Ethoxyphenyl)-3-methylphenyl]methyl]piperazin-1-yl]-N,N-diethylpyridazine-3-carboxamide |
| B4J074-01 | 6-[4-[[4-(5-Ethoxypyridin-3-yl)-3-methylphenyl]methyl]piperazin-1-yl]-N,N-diethylpyridazine-3-carboxamide |
| B4J075-01 | 6-[4-[[4-(3-Ethoxyphenyl)-2-ethylphenyl]methyl]piperazin-1-yl]-N,N-diethylpyridazine-3-carboxamide |
| B4J076-01 | 6-[4-[[4-(5-Ethoxypyridin-3-yl)-2-ethylphenyl]methyl]piperazin-1-yl]-N,N-diethylpyridazine-3-carboxamide |
| B4J077-01 | 6-[4-[[4-(3-Ethoxyphenyl)-3-fluorophenyl]methyl]piperazin-1-yl]-N,N-diethylpyridazine-3-carboxamide |
| B4J078-01 | 6-[4-[[4-(5-Ethoxypyridin-3-yl)-3-fluorophenyl]methyl]piperazin-1-yl]-N,N-diethylpyridazine-3-carboxamide |
| B4J079-01 | 6-[4-[[4-(3-Ethoxyphenyl)-2-methoxyphenyl]methyl]piperazin-1-yl]-N,N-diethylpyridazine-3-carboxamide |
| B4J080-01 | 6-[4-[[4-(5-Ethoxypyridin-3-yl)-2-methoxyphenyl]methyl]piperazin-1-yl]-N,N-diethylpyridazine-3-carboxamide |
| B4J081-01 | 6-[4-[[3-Ethoxy-5-(3-ethoxyphenyl)phenyl]methyl]piperazin-1-yl]-N,N-diethylpyridazine-3-carboxamide |
| B4J082-01 | 6-[4-[[3-Ethoxy-5-(5-ethoxypyridin-3-yl)phenyl]methyl]piperazin-1-yl]-N,N-diethylpyridazine-3-carboxamide |
| B4J083-01 | 6-[4-[[3-(3-Ethoxyphenyl)-5-propan-2-yloxyphenyl]methyl]piperazin-1-yl]-N,N-diethylpyridazine-3-carboxamide |
| B4J084-01 | 6-[4-[[3-(5-Ethoxypyridin-3-yl)-5-propan-2-yloxyphenyl]methyl]piperazin-1-yl]-N,N-diethylpyridazine-3-carboxamide |
| B4J085-01 | 6-[4-[[4-(3-Ethoxyphenyl)-2,6-dimethoxyphenyl]methyl]piperazin-1-yl]-N,N-diethylpyridazine-3-carboxamide |
| B4J086-01 | 6-[4-[[4-(5-Ethoxypyridin-3-yl)-2,6-dimethoxyphenyl]methyl]piperazin-1-yl]-N,N-diethylpyridazine-3-carboxamide |
| B4J087-01 | 6-[4-[[3-(3-Ethoxyphenyl)-5-(trifluoromethyl)phenyl]methyl]piperazin-1-yl]-N,N-diethylpyridazine-3-carboxamide |
| B4J088-01 | 6-[4-[[3-(5-Ethoxypyridin-3-yl)-5-(trifluoromethyl)phenyl]methyl]piperazin-1-yl]-N,N-diethylpyridazine-3-carboxamide |
| B4J089-01 | 6-[4-[[3-(3-Ethoxyphenyl)-5-(trifluoromethoxy)phenyl]methyl]piperazin-1-yl]-N,N-diethylpyridazine-3-carboxamide |

| | |
|---|---|
| B4J090-01 | 6-[4-[[3-(5-Ethoxypyridin-3-yl)-5-(trifluoromethoxy)phenyl]methyl]piperazin-1-yl]-N,N-diethylpyridazine-3-carboxamide |
| B4J091-01 | 6-[4-[[4-(3-Ethoxyphenyl)naphthalen-1-yl]methyl]piperazin-1-yl]-N,N-diethylpyridazine-3-carboxamide |
| B4J092-01 | 6-[4-[[4-(5-Ethoxypyridin-3-yl)naphthalen-1-yl]methyl]piperazin-1-yl]-N,N-diethylpyridazine-3-carboxamide |
| B4J093-01 | 6-[4-[[4-(3-Ethoxyphenyl)thiophen-2-yl]methyl]piperazin-1-yl]-N,N-diethylpyridazine-3-carboxamide |
| B4J094-01 | 6-[4-[[4-(5-Ethoxypyridin-3-yl)thiophen-2-yl]methyl]piperazin-1-yl]-N,N-diethylpyridazine-3-carboxamide |
| B4J095-01 | 6-[4-[[2-[2-(3-Ethoxyphenyl)ethynyl]phenyl]methyl]piperazin-1-yl]-N,N-diethylpyridazine-3-carboxamide |
| B4J096-01 | 6-[4-[[2-[2-(5-Ethoxypyridin-3-yl)ethynyl]phenyl]methyl]piperazin-1-yl]-N,N-diethylpyridazine-3-carboxamide |
| B4J097-01 | 4-[4-[1-[4-(3-Ethoxyphenyl)phenyl]ethyl]piperazin-1-yl]-N,N-diethylbenzamide |
| B4J098-01 | 4-[4-[1-[4-(5-Ethoxypyridin-3-yl)phenyl]ethyl]piperazin-1-yl]-N,N-diethylbenzamide |
| B4J099-01 | 4-[4-[1-[4-(3-Ethoxyphenyl)-2-fluorophenyl]ethyl]piperazin-1-yl]-N,N-diethylbenzamide |
| B4J100-01 | 4-[4-[1-[4-(5-Ethoxypyridin-3-yl)-2-fluorophenyl]ethyl]piperazin-1-yl]-N,N-diethylbenzamide |

[Table 243]

[Table AI-03-B4J-01]

| Label | Exact | ☒ | A | a | B | b | C | c | D |
|---|---|---|---|---|---|---|---|---|---|
| B4J001-01 | 493.22 | OEt | A01 | a1 | B10 | b3 | C02 | c1 | D03 |
| B4J002-01 | 494.21 | OEt | A02 | a1 | B10 | b3 | C02 | c1 | D03 |
| B4J003-01 | 501.27 | OEt | A01 | a1 | B04 | b3 | C02 | c1 | D03 |
| B4J004-01 | 502.27 | OEt | A02 | a1 | B04 | b3 | C02 | c1 | D03 |
| B4J005-01 | 505.25 | OEt | A01 | a1 | B06 | b3 | C02 | c1 | D03 |
| B4J006-01 | 506.24 | OEt | A02 | a1 | B06 | b3 | C02 | c1 | D03 |
| B4J007-01 | 511.26 | OEt | A01 | a2 | B01 | b3 | C02 | c1 | D03 |
| B4J008-01 | 512.25 | OEt | A02 | a2 | B01 | b3 | C02 | c1 | D03 |
| B4J009-01 | 512.25 | OEt | A01 | a2 | B17 | b3 | C02 | c1 | D03 |
| B4J010-01 | 513.25 | OEt | A02 | a2 | B17 | b3 | C02 | c1 | D03 |
| B4J011-01 | 515.29 | OEt | A01 | a1 | B02 | b3 | C02 | c1 | D03 |
| B4J012-01 | 516.29 | OEt | A02 | a1 | B02 | b3 | C02 | c1 | D03 |
| B4J013-01 | 537.27 | OEt | A01 | a1 | B09 | b3 | C02 | c1 | D03 |
| B4J014-01 | 538.27 | OEt | A02 | a1 | B09 | b3 | C02 | c1 | D03 |
| B4J015-01 | 543.32 | OEt | A01 | a1 | B16 | b3 | C02 | c1 | D03 |
| B4J016-01 | 544.32 | OEt | A02 | a1 | B16 | b3 | C02 | c1 | D03 |
| B4J017-01 | 555.25 | OEt | A01 | a1 | B11 | b3 | C02 | c1 | D03 |
| B4J018-01 | 556.24 | OEt | A02 | a1 | B11 | b3 | C02 | c1 | D03 |
| B4J019-01 | 579.25 | OEt | A01 | a2 | B11 | b3 | C02 | c1 | D03 |
| B4J020-01 | 580.24 | OEt | A02 | a2 | B11 | b3 | C02 | c1 | D03 |
| B4J021-01 | 621.27 | OEt | A01 | a1 | B12 | b3 | C02 | c1 | D03 |
| B4J022-01 | 622.26 | OEt | A02 | a1 | B12 | b3 | C02 | c1 | D03 |
| B4J023-01 | 491.22 | OEt | A01 | a1 | B10 | b3 | C01 | c1 | D03 |
| B4J024-01 | 492.22 | OEt | A02 | a1 | B10 | b3 | C01 | c1 | D03 |
| B4J025-01 | 499.28 | OEt | A01 | a1 | B04 | b3 | C01 | c1 | D03 |

| | | | | | | | | | |
|---|---|---|---|---|---|---|---|---|---|
| B4J026-01 | 500.28 | OEt | A02 | a1 | B04 | b3 | C01 | c1 | D03 |
| B4J027-01 | 503.26 | OEt | A01 | a1 | B06 | b3 | C01 | c1 | D03 |
| B4J028-01 | 504.25 | OEt | A02 | a1 | B06 | b3 | C01 | c1 | D03 |
| B4J029-01 | 509.27 | OEt | A01 | a2 | B01 | b3 | C01 | c1 | D03 |
| B4J030-01 | 510.26 | OEt | A02 | a2 | B01 | b3 | C01 | c1 | D03 |
| B4J031-01 | 510.26 | OEt | A01 | a2 | B17 | b3 | C01 | c1 | D03 |
| B4J032-01 | 511.26 | OEt | A02 | a2 | B17 | b3 | C01 | c1 | D03 |
| B4J033-01 | 513.3 | OEt | A01 | a1 | B02 | b3 | C01 | c1 | D03 |
| B4J034-01 | 514.29 | OEt | A02 | a1 | B02 | b3 | C01 | c1 | D03 |
| B4J035-01 | 535.28 | OEt | A01 | a1 | B09 | b3 | C01 | c1 | D03 |
| B4J036-01 | 536.28 | OEt | A02 | a1 | B09 | b3 | C01 | c1 | D03 |
| B4J037-01 | 541.33 | OEt | A01 | a1 | B16 | b3 | C01 | c1 | D03 |
| B4J038-01 | 542.33 | OEt | A02 | a1 | B16 | b3 | C01 | c1 | D03 |
| B4J039-01 | 553.26 | OEt | A01 | a1 | B11 | b3 | C01 | c1 | D03 |
| B4J040-01 | 554.25 | OEt | A02 | a1 | B11 | b3 | C01 | c1 | D03 |
| B4J041-01 | 577.26 | OEt | A01 | a2 | B11 | b3 | C01 | c1 | D03 |
| B4J042-01 | 578.25 | OEt | A02 | a2 | B11 | b3 | C01 | c1 | D03 |
| B4J043-01 | 619.28 | OEt | A01 | a1 | B12 | b3 | C01 | c1 | D03 |
| B4J044-01 | 620.27 | OEt | A02 | a1 | B12 | b3 | C01 | c1 | D03 |
| B4J045-01 | 471.29 | OEt | A01 | a1 | B01 | b1 | C01 | c1 | D03 |
| B4J046-01 | 472.28 | OEt | A02 | a1 | B01 | b1 | C01 | c1 | D03 |
| B4J047-01 | 485.3 | OEt | A01 | a1 | B04 | b1 | C01 | c1 | D03 |
| B4J048-01 | 486.3 | OEt | A02 | a1 | B04 | b1 | C01 | c1 | D03 |
| B4J049-01 | 499.32 | OEt | A01 | a1 | B05 | b1 | C01 | c1 | D03 |
| B4J050-01 | 500.32 | OEt | A02 | a1 | B05 | b1 | C01 | c1 | D03 |
| B4J051-01 | 489.28 | OEt | A01 | a1 | B06 | b1 | C01 | c1 | D03 |
| B4J052-01 | 490.27 | OEt | A02 | a1 | B06 | b1 | C01 | c1 | D03 |
| B4J053-01 | 501.3 | OEt | A01 | a1 | B07 | b1 | C01 | c1 | D03 |
| B4J054-01 | 502.29 | OEt | A02 | a1 | B07 | b1 | C01 | c1 | D03 |
| B4J055-01 | 515.32 | OEt | A01 | a1 | B14 | b1 | C01 | c1 | D03 |
| B4J056-01 | 516.31 | OEt | A02 | a1 | B14 | b1 | C01 | c1 | D03 |
| B4J057-01 | 529.33 | OEt | A01 | a1 | B15 | b1 | C01 | c1 | D03 |
| B4J058-01 | 530.33 | OEt | A02 | a1 | B15 | b1 | C01 | c1 | D03 |
| B4J059-01 | 531.31 | OEt | A01 | a1 | B08 | b1 | C01 | c1 | D03 |
| B4J060-01 | 532.31 | OEt | A02 | a1 | B08 | b1 | C01 | c1 | D03 |
| B4J061-01 | 539.28 | OEt | A01 | a1 | B11 | b1 | C01 | c1 | D03 |
| B4J062-01 | 540.27 | OEt | A02 | a1 | B11 | b1 | C01 | c1 | D03 |
| B4J063-01 | 555.27 | OEt | A01 | a1 | B13 | b1 | C01 | c1 | D03 |
| B4J064-01 | 556.27 | OEt | A02 | a1 | B13 | b1 | C01 | c1 | D03 |
| B4J065-01 | 521.3 | OEt | A01 | a1 | B09 | b1 | C01 | c1 | D03 |
| B4J066-01 | 522.3 | OEt | A02 | a1 | B09 | b1 | C01 | c1 | D03 |
| B4J067-01 | 477.25 | OEt | A01 | a1 | B10 | b1 | C01 | c1 | D03 |
| B4J068-01 | 478.24 | OEt | A02 | a1 | B10 | b1 | C01 | c1 | D03 |
| B4J069-01 | 495.29 | OEt | A01 | a2 | B01 | b1 | C01 | c1 | D03 |
| B4J070-01 | 496.28 | OEt | A02 | a2 | B01 | b1 | C01 | c1 | D03 |
| B4J071-01 | 473.28 | OEt | A01 | a1 | B01 | b1 | C02 | c1 | D03 |
| B4J072-01 | 474.27 | OEt | A02 | a1 | B01 | b1 | C02 | c1 | D03 |

| | | | | | | | | | |
|---|---|---|---|---|---|---|---|---|---|
| B4J073-01 | 487.3 | OEt | A01 | a1 | B04 | b1 | C02 | c1 | D03 |
| B4J074-01 | 488.29 | OEt | A02 | a1 | B04 | b1 | C02 | c1 | D03 |
| B4J075-01 | 501.31 | OEt | A01 | a1 | B05 | b1 | C02 | c1 | D03 |
| B4J076-01 | 502.31 | OEt | A02 | a1 | B05 | b1 | C02 | c1 | D03 |
| B4J077-01 | 491.27 | OEt | A01 | a1 | B06 | b1 | C02 | c1 | D03 |
| B4J078-01 | 492.27 | OEt | A02 | a1 | B06 | b1 | C02 | c1 | D03 |
| B4J079-01 | 503.29 | OEt | A01 | a1 | B07 | b1 | C02 | c1 | D03 |
| B4J080-01 | 504.29 | OEt | A02 | a1 | B07 | b1 | C02 | c1 | D03 |
| B4J081-01 | 517.31 | OEt | A01 | a1 | B14 | b1 | C02 | c1 | D03 |
| B4J082-01 | 518.3 | OEt | A02 | a1 | B14 | b1 | C02 | c1 | D03 |
| B4J083-01 | 531.32 | OEt | A01 | a1 | B15 | b1 | C02 | c1 | D03 |
| B4J084-01 | 532.32 | OEt | A02 | a1 | B15 | b1 | C02 | c1 | D03 |
| B4J085-01 | 533.3 | OEt | A01 | a1 | B08 | b1 | C02 | c1 | D03 |
| B4J086-01 | 534.3 | OEt | A02 | a1 | B08 | b1 | C02 | c1 | D03 |
| B4J087-01 | 541.27 | OEt | A01 | a1 | B11 | b1 | C02 | c1 | D03 |
| B4J088-01 | 542.26 | OEt | A02 | a1 | B11 | b1 | C02 | c1 | D03 |
| B4J089-01 | 557.26 | OEt | A01 | a1 | B13 | b1 | C02 | c1 | D03 |
| B4J090-01 | 558.26 | OEt | A02 | a1 | B13 | b1 | C02 | c1 | D03 |
| B4J091-01 | 523.3 | OEt | A01 | a1 | B09 | b1 | C02 | c1 | D03 |
| B4J092-01 | 524.29 | OEt | A02 | a1 | B09 | b1 | C02 | c1 | D03 |
| B4J093-01 | 479.24 | OEt | A01 | a1 | B10 | b1 | C02 | c1 | D03 |
| B4J094-01 | 480.23 | OEt | A02 | a1 | B10 | b1 | C02 | c1 | D03 |
| B4J095-01 | 497.28 | OEt | A01 | a2 | B01 | b1 | C02 | c1 | D03 |
| B4J096-01 | 498.27 | OEt | A02 | a2 | B01 | b1 | C02 | c1 | D03 |
| B4J097-01 | 485.3 | OEt | A01 | a1 | B03 | b2 | C01 | c1 | D03 |
| B4J098-01 | 486.3 | OEt | A02 | a1 | B03 | b2 | C01 | c1 | D03 |
| B4J099-01 | 503.3 | OEt | A01 | a1 | B18 | b2 | C01 | c1 | D03 |
| B4J100-01 | 504.29 | OEt | A02 | a1 | B18 | b2 | C01 | c1 | D03 |

[Table 244]

[Table AI-03-B1M-01]

| Mixture No. | mixAI-B1M-01 |
|---|---|
| No. of compound that may be contained | Compound name |
| B1M001-01 | 6-[4-[5-[2-(3-Ethoxyphenyl)ethynyl]pyridine-3-carbonyl]piperazin-1-yl]-N-propylpyridazine-3-carboxamide |
| B1M002-01 | 4-[4-[5-[2-(3-Ethoxyphenyl)ethynyl]pyridine-3-carbonyl]piperazin-1-yl]-N-propylbenzamide |
| B1M003-01 | 4-[4-[1-[4-(3-Ethoxyphenyl)phenyl]ethyl]piperazin-1-yl]-N-propylbenzamide |
| B1M004-01 | 4-[4-[1-[4-(5-Ethoxypyridin-3-yl)phenyl]ethyl]piperazin-1-yl]-N-propylbenzamide |
| B1M005-01 | 4-[4-[1-[4-(3-Ethoxyphenyl)-2-fluorophenyl]ethyl]piperazin-1-yl]-N-propylbenzamide |
| B1M006-01 | 4-[4-[1-[4-(5-Ethoxypyridin-3-yl)-2-fluorophenyl]ethyl]piperazin-1-yl]-N-propylbenzamide |
| B1M007-01 | 6-[4-[3-(5-Ethoxypyridin-3-yl)-5-(trifluoromethyl)benzoyl]piperazin-1-yl]-N-propylpyridazine-3-carboxamide |
| B1M008-01 | 4-[4-[3-(5-Ethoxypyridin-3-yl)-5-(trifluoromethyl)benzoyl]piperazin-1-yl]-N-propylbenzamide |

| | |
|---|---|
| B1M009-01 | 4-[4-[[3-Ethoxy-5-(3-ethoxyphenyl)phenyl]methyl]piperazin-1-yl]-N-propylbenzamide |
| B1M010-01 | 6-[4-[[3-Ethoxy-5-(3-ethoxyphenyl)phenyl]methyl]piperazin-1-yl]-N-propylpyridazine-3-carboxamide |
| B1M011-01 | 4-[4-[[3-Ethoxy-5-(5-ethoxypyridin-3-yl)phenyl]methyl]piperazin-1-yl]-N-propylbenzamide |
| B1M012-01 | 6-[4-[[3-Ethoxy-5-(5-ethoxypyridin-3-yl)phenyl]methyl]piperazin-1-yl]-N-propylpyridazine-3-carboxamide |
| B1M013-01 | 6-[4-[3-[2-(3-Ethoxyphenyl)ethynyl]-5-(trifluoromethyl)benzoyl]piperazin-1-yl]-N-propylpyridazine-3-carboxamide |
| B1M014-01 | 4-[4-[3-[2-(3-Ethoxyphenyl)ethynyl]-5-(trifluoromethyl)benzoyl]piperazin-1-yl]-N-propylbenzamide |
| B1M015-01 | 4-[4-[[3-(3-Ethoxyphenyl)-5-propan-2-yloxyphenyl]methyl]piperazin-1-yl]-N-propylbenzamide |
| B1M016-01 | 6-[4-[[3-(3-Ethoxyphenyl)-5-propan-2-yloxyphenyl]methyl]piperazin-1-yl]-N-propylpyridazine-3-carboxamide |
| B1M017-01 | 4-[4-[[3-(5-Ethoxypyridin-3-yl)-5-propan-2-yloxyphenyl]methyl]piperazin-1-yl]-N-propylbenzamide |
| B1M018-01 | 6-[4-[3-[2-(5-Ethoxypyridin-3-yl)ethynyl]-5-(trifluoromethyl)benzoyl]piperazin-1-yl]-N-propylpyridazine-3-carboxamide |
| B1M019-01 | 4-[4-[3-[2-(5-Ethoxypyridin-3-yl)ethynyl]-5-(trifluoromethyl)benzoyl]piperazin-1-yl]-N-propylbenzamide |
| B1M020-01 | 4-[4-[[4-(3-Ethoxyphenyl)-2,6-dimethoxyphenyl]methyl]piperazin-1-yl]-N-propylbenzamide |
| B1M021-01 | 6-[4-[[4-(3-Ethoxyphenyl)-2,6-dimethoxyphenyl]methyl]piperazin-1-yl]-N-propylpyridazine-3-carboxamide |
| B1M022-01 | 4-[4-[[4-(5-Ethoxypyridin-3-yl)-2,6-dimethoxyphenyl]methyl]piperazin-1-yl]-N-propylbenzamide |
| B1M023-01 | 6-[4-[[4-(5-Ethoxypyridin-3-yl)-2,6-dimethoxyphenyl]methyl]piperazin-1-yl]-N-propylpyridazine-3-carboxamide |
| B1M024-01 | 6-[4-[3-[4-(3-Ethoxyphenyl)pyrazol-1-yl]-5-(trifluoromethyl)benzoyl]piperazin-1-yl]-N-propylpyridazine-3-carboxamide |
| B1M025-01 | 4-[4-[3-[4-(3-Ethoxyphenyl)pyrazol-1-yl]-5-(trifluoromethyl)benzoyl]piperazin-1-yl]-N-propylbenzamide |
| B1M026-01 | 4-[4-[[3-(3-Ethoxyphenyl)-5-(trifluoromethyl)phenyl]methyl]piperazin-1-yl]-N-propylbenzamide |
| B1M027-01 | 6-[4-[[3-(3-Ethoxyphenyl)-5-(trifluoromethyl)phenyl]methyl]piperazin-1-yl]-N-propylpyridazine-3-carboxamide |
| B1M028-01 | 4-[4-[[3-(5-Ethoxypyridin-3-yl)-5-(trifluoromethyl)phenyl]methyl]piperazin-1-yl]-N-propylbenzamide |
| B1M029-01 | 6-[4-[[3-(5-Ethoxypyridin-3-yl)-5-(trifluoromethyl)phenyl]methyl]piperazin-1-yl]-N-propylpyridazine-3-carboxamide |
| B1M030-01 | 6-[4-[3-[4-(5-Ethoxypyridin-3-yl)pyrazol-1-yl]-5-(trifluoromethyl)benzoyl]piperazin-1-yl]-N-propylpyridazine-3-carboxamide |
| B1M031-01 | 4-[4-[3-[4-(5-Ethoxypyridin-3-yl)pyrazol-1-yl]-5-(trifluoromethyl)benzoyl]piperazin-1-yl]-N-propylbenzamide |
| B1M032-01 | 4-[4-[[3-(3-Ethoxyphenyl)-5-(trifluoromethoxy)phenyl]methyl]piperazin-1-yl]-N-propylbenzamide |
| B1M033-01 | 6-[4-[[3-(3-Ethoxyphenyl)-5-(trifluoromethoxy)phenyl]methyl]piperazin-1-yl]-N-propylpyridazine-3-carboxamide |
| B1M034-01 | 4-[4-[[3-(5-Ethoxypyridin-3-yl)-5-(trifluoromethoxy)phenyl]methyl]piperazin-1-yl]-N-propylbenzamide |
| B1M035-01 | 6-[4-[[3-(5-Ethoxypyridin-3-yl)-5-(trifluoromethoxy)phenyl]methyl]piperazin-1-yl]-N-propylpyridazine-3-carboxamide |
| B1M036-01 | 6-[4-[4-(3-Ethoxyphenyl)-3-methylbenzoyl]piperazin-1-yl]-N-propylpyridazine-3-carboxamide |

| | |
|---|---|
| B1M037-01 | 6-[4-[4-(5-Ethoxypyridin-3-yl)-3-methylbenzoyl]piperazin-1-yl]-N-propylpyridazine-3-carboxamide |
| B1M038-01 | 4-[4-[4-(3-Ethoxyphenyl)-3-methylbenzoyl]piperazin-1-yl]-N-propylbenzamide |
| B1M039-01 | 4-[4-[4-(5-Ethoxypyridin-3-yl)-3-methylbenzoyl]piperazin-1-yl]-N-propylbenzamide |
| B1M040-01 | 4-[4-[[2-(3-Ethoxyphenyl)phenyl]methyl]piperazin-1-yl]-N-propylbenzamide |
| B1M041-01 | 4-[4-[[2-(5-Ethoxypyridin-3-yl)phenyl]methyl]piperazin-1-yl]-N-propylbenzamide |
| B1M042-01 | 6-[4-[[2-(3-Ethoxyphenyl)phenyl]methyl]piperazin-1-yl]-N-propylpyridazine-3-carboxamide |
| B1M043-01 | 6-[4-[[2-(5-Ethoxypyridin-3-yl)phenyl]methyl]piperazin-1-yl]-N-propylpyridazine-3-carboxamide |
| B1M044-01 | 4-[4-[[4-(3-Ethoxyphenyl)naphthalen-1-yl]methyl]piperazin-1-yl]-N-propylbenzamide |
| B1M045-01 | 6-[4-[[4-(3-Ethoxyphenyl)naphthalen-1-yl]methyl]piperazin-1-yl]-N-propylpyridazine-3-carboxamide |
| B1M046-01 | 4-[4-[[4-(5-Ethoxypyridin-3-yl)naphthalen-1-yl]methyl]piperazin-1-yl]-N-propylbenzamide |
| B1M047-01 | 6-[4-[[4-(5-Ethoxypyridin-3-yl)naphthalen-1-yl]methyl]piperazin-1-yl]-N-propylpyridazine-3-carboxamide |
| B1M048-01 | 4-[4-[[4-(3-Ethoxyphenyl)thiophen-2-yl]methyl]piperazin-1-yl]-N-propylbenzamide |
| B1M049-01 | 6-[4-[[4-(3-Ethoxyphenyl)thiophen-2-yl]methyl]piperazin-1-yl]-N-propylpyridazine-3-carboxamide |
| B1M050-01 | 4-[4-[[4-(5-Ethoxypyridin-3-yl)thiophen-2-yl]methyl]piperazin-1-yl]-N-propylbenzamide |
| B1M051-01 | 6-[4-[[4-(5-Ethoxypyridin-3-yl)thiophen-2-yl]methyl]piperazin-1-yl]-N-propylpyridazine-3-carboxamide |
| B1M052-01 | 4-[4-[[2-[2-(3-Ethoxyphenyl)ethynyl]phenyl]methyl]piperazin-1-yl]-N-propylbenzamide |
| B1M053-01 | 6-[4-[[2-[2-(3-Ethoxyphenyl)ethynyl]phenyl]methyl]piperazin-1-yl]-N-propylpyridazine-3-carboxamide |
| B1M054-01 | 4-[4-[[2-[2-(5-Ethoxypyridin-3-yl)ethynyl]phenyl]methyl]piperazin-1-yl]-N-propylbenzamide |
| B1M055-01 | 6-[4-[[2-[2-(5-Ethoxypyridin-3-yl)ethynyl]phenyl]methyl]piperazin-1-yl]-N-propylpyridazine-3-carboxamide |
| B1M056-01 | 6-[4-[5-[2-(5-Ethoxypyridin-3-yl)ethynyl]pyridine-3-carbonyl]piperazin-1-yl]-N-propylpyridazine-3-carboxamide |
| B1M057-01 | 4-[4-[5-[2-(5-Ethoxypyridin-3-yl)ethynyl]pyridine-3-carbonyl]piperazin-1-yl]-N-propylbenzamide |
| B1M058-01 | 4-[4-[[4-(3-Ethoxyphenyl)-3-methylphenyl]methyl]piperazin-1-yl]-N-propylbenzamide |
| B1M059-01 | 6-[4-[[4-(3-Ethoxyphenyl)-3-methylphenyl]methyl]piperazin-1-yl]-N-propylpyridazine-3-carboxamide |
| B1M060-01 | 6-[4-[3-tert-Butyl-5-(3-ethoxyphenyl)benzoyl]piperazin-1-yl]-N-propylpyridazine-3-carboxamide |
| B1M061-01 | 6-[4-[3-tert-Butyl-5-(5-ethoxypyridin-3-yl)benzoyl]piperazin-1-yl]-N-propylpyridazine-3-carboxamide |
| B1M062-01 | 4-[4-[3-tert-Butyl-5-(3-ethoxyphenyl)benzoyl]piperazin-1-yl]-N-propylbenzamide |
| B1M063-01 | 4-[4-[3-tert-Butyl-5-(5-ethoxypyridin-3-yl)benzoyl]piperazin-1-yl]-N-propylbenzamide |
| B1M064-01 | 4-[4-[[4-(5-Ethoxypyridin-3-yl)-3-methylphenyl]methyl]piperazin-1-yl]-N-propylbenzamide |
| B1M065-01 | 6-[4-[[4-(5-Ethoxypyridin-3-yl)-3-methylphenyl]methyl]piperazin-1-yl]-N-propylpyridazine-3-carboxamide |
| B1M066-01 | 6-[4-[[3-(5-Ethoxypyridin-3-yl)-5-propan-2-yloxyphenyl]methyl]piperazin-1-yl]-N-propylpyridazine-3-carboxamide |
| B1M067-01 | 6-[4-[3-[2-(3-Ethoxyphenyl)phenyl]propanoyl]piperazin-1-yl]-N-propylpyridazine-3-carboxamide |
| B1M068-01 | 4-[4-[3-[2-(3-Ethoxyphenyl)phenyl]propanoyl]piperazin-1-yl]-N-propylbenzamide |

| | |
|---|---|
| B1M069-01 | 4-[4-[[4-(3-Ethoxyphenyl)-2-ethylphenyl]methyl]piperazin-1-yl]-N-propylbenzamide |
| B1M070-01 | 6-[4-[[4-(3-Ethoxyphenyl)-2-ethylphenyl]methyl]piperazin-1-yl]-N-propylpyridazine-3-carboxamide |
| B1M071-01 | 6-[4-[4-(3-Ethoxyphenyl)naphthalene-1-carbonyl]piperazin-1-yl]-N-propylpyridazine-3-carboxamide |
| B1M072-01 | 6-[4-[4-(5-Ethoxypyridin-3-yl)naphthalene-1-carbonyl]piperazin-1-yl]-N-propylpyridazine-3-carboxamide |
| B1M073-01 | 4-[4-[4-(3-Ethoxyphenyl)naphthalene-1-carbonyl]piperazin-1-yl]-N-propylbenzamide |
| B1M074-01 | 4-[4-[4-(5-Ethoxypyridin-3-yl)naphthalene-1-carbonyl]piperazin-1-yl]-N-propylbenzamide |
| B1M075-01 | 4-[4-[[4-(5-Ethoxypyridin-3-yl)-2-ethylphenyl]methyl]piperazin-1-yl]-N-propylbenzamide |
| B1M076-01 | 6-[4-[[4-(5-Ethoxypyridin-3-yl)-2-ethylphenyl]methyl]piperazin-1-yl]-N-propylpyridazine-3-carboxamide |
| B1M077-01 | 6-[4-[3-[2-(5-Ethoxypyridin-3-yl)phenyl]propanoyl]piperazin-1-yl]-N-propylpyridazine-3-carboxamide |
| B1M078-01 | 4-[4-[3-[2-(5-Ethoxypyridin-3-yl)phenyl]propanoyl]piperazin-1-yl]-N-propylbenzamide |
| B1M079-01 | 4-[4-[[4-(3-Ethoxyphenyl)-3-fluorophenyl]methyl]piperazin-1-yl]-N-propylbenzamide |
| B1M080-01 | 6-[4-[[4-(3-Ethoxyphenyl)-3-fluorophenyl]methyl]piperazin-1-yl]-N-propylpyridazine-3-carboxamide |
| B1M081-01 | 6-[4-[4-(3-Ethoxyphenyl)thiophene-2-carbonyl]piperazin-1-yl]-N-propylpyridazine-3-carboxamide |
| B1M082-01 | 6-[4-[4-(5-Ethoxypyridin-3-yl)thiophene-2-carbonyl]piperazin-1-yl]-N-propylpyridazine-3-carboxamide |
| B1M083-01 | 4-[4-[4-(3-Ethoxyphenyl)thiophene-2-carbonyl]piperazin-1-yl]-N-propylbenzamide |
| B1M084-01 | 4-[4-[4-(5-Ethoxypyridin-3-yl)thiophene-2-carbonyl]piperazin-1-yl]-N-propylbenzamide |
| B1M085-01 | 4-[4-[[4-(5-Ethoxypyridin-3-yl)-3-fluorophenyl]methyl]piperazin-1-yl]-N-propylbenzamide |
| B1M086-01 | 6-[4-[[4-(5-Ethoxypyridin-3-yl)-3-fluorophenyl]methyl]piperazin-1-yl]-N-propylpyridazine-3-carboxamide |
| B1M087-01 | 6-[4-[4-(3-Ethoxyphenyl)-3-fluorobenzoyl]piperazin-1-yl]-N-propylpyridazine-3-carboxamide |
| B1M088-01 | 6-[4-[4-(5-Ethoxypyridin-3-yl)-3-fluorobenzoyl]piperazin-1-yl]-N-propylpyridazine-3-carboxamide |
| B1M089-01 | 6-[4-[3-(3-Ethoxyphenyl)-5-(trifluoromethyl)benzoyl]piperazin-1-yl]-N-propylpyridazine-3-carboxamide |
| B1M090-01 | 4-[4-[4-(3-Ethoxyphenyl)-3-fluorobenzoyl]piperazin-1-yl]-N-propylbenzamide |
| B1M091-01 | 4-[4-[4-(5-Ethoxypyridin-3-yl)-3-fluorobenzoyl]piperazin-1-yl]-N-propylbenzamide |
| B1M092-01 | 4-[4-[3-(3-Ethoxyphenyl)-5-(trifluoromethyl)benzoyl]piperazin-1-yl]-N-propylbenzamide |
| B1M093-01 | 4-[4-[[4-(3-Ethoxyphenyl)-2-methoxyphenyl]methyl]piperazin-1-yl]-N-propylbenzamide |
| B1M094-01 | 6-[4-[[4-(3-Ethoxyphenyl)-2-methoxyphenyl]methyl]piperazin-1-yl]-N-propylpyridazine-3-carboxamide |
| B1M095-01 | 6-[4-[2-[2-(3-Ethoxyphenyl)ethynyl]benzoyl]piperazin-1-yl]-N-propylpyridazine-3-carboxamide |
| B1M096-01 | 6-[4-[2-[2-(5-Ethoxypyridin-3-yl)ethynyl]benzoyl]piperazin-1-yl]-N-propylpyridazine-3-carboxamide |
| B1M097-01 | 4-[4-[2-[2-(3-Ethoxyphenyl)ethynyl]benzoyl]piperazin-1-yl]-N-propylbenzamide |
| B1M098-01 | 4-[4-[2-[2-(5-Ethoxypyridin-3-yl)ethynyl]benzoyl]piperazin-1-yl]-N-propylbenzamide |
| B1M099-01 | 4-[4-[[4-(5-Ethoxypyridin-3-yl)-2-methoxyphenyl]methyl]piperazin-1-yl]-N-propylbenzamide |

| | |
|---|---|
| B1M100-01 | 6-[4-[[4-(5-Ethoxypyridin-3-yl)-2-methoxyphenyl]methyl]piperazin-1-yl]-N-propylpyridazine-3-carboxamide |

[Table 245]

[Table AI-03-B1M-01]

| Label | Exact | ☒ | A | a | B | b | C | c | D |
|---|---|---|---|---|---|---|---|---|---|
| B1M001-01 | 479.2 | OEt | A01 | a1 | B10 | b3 | C02 | c1 | D01 |
| B1M002-01 | 480.19 | OEt | A02 | a1 | B10 | b3 | C02 | c1 | D01 |
| B1M003-01 | 487.26 | OEt | A01 | a1 | B04 | b3 | C02 | c1 | D01 |
| B1M004-01 | 488.25 | OEt | A02 | a1 | B04 | b3 | C02 | c1 | D01 |
| B1M005-01 | 491.23 | OEt | A01 | a1 | B06 | b3 | C02 | c1 | D01 |
| B1M006-01 | 492.23 | OEt | A02 | a1 | B06 | b3 | C02 | c1 | D01 |
| B1M007-01 | 497.24 | OEt | A01 | a2 | B01 | b3 | C02 | c1 | D01 |
| B1M008-01 | 498.24 | OEt | A02 | a2 | B01 | b3 | C02 | c1 | D01 |
| B1M009-01 | 498.24 | OEt | A01 | a2 | B17 | b3 | C02 | c1 | D01 |
| B1M010-01 | 499.23 | OEt | A02 | a2 | B17 | b3 | C02 | c1 | D01 |
| B1M011-01 | 501.27 | OEt | A01 | a1 | B02 | b3 | C02 | c1 | D01 |
| B1M012-01 | 502.27 | OEt | A02 | a1 | B02 | b3 | C02 | c1 | D01 |
| B1M013-01 | 523.26 | OEt | A01 | a1 | B09 | b3 | C02 | c1 | D01 |
| B1M014-01 | 524.25 | OEt | A02 | a1 | B09 | b3 | C02 | c1 | D01 |
| B1M015-01 | 529.31 | OEt | A01 | a1 | B16 | b3 | C02 | c1 | D01 |
| B1M016-01 | 530.3 | OEt | A02 | a1 | B16 | b3 | C02 | c1 | D01 |
| B1M017-01 | 541.23 | OEt | A01 | a1 | B11 | b3 | C02 | c1 | D01 |
| B1M018-01 | 542.23 | OEt | A02 | a1 | B11 | b3 | C02 | c1 | D01 |
| B1M019-01 | 565.23 | OEt | A01 | a2 | B11 | b3 | C02 | c1 | D01 |
| B1M020-01 | 566.23 | OEt | A02 | a2 | B11 | b3 | C02 | c1 | D01 |
| B1M021-01 | 607.25 | OEt | A01 | a1 | B12 | b3 | C02 | c1 | D01 |
| B1M022-01 | 608.25 | OEt | A02 | a1 | B12 | b3 | C02 | c1 | D01 |
| B1M023-01 | 477.21 | OEt | A01 | a1 | B10 | b3 | C01 | c1 | D01 |
| B1M024-01 | 478.2 | OEt | A02 | a1 | B10 | b3 | C01 | c1 | D01 |
| B1M025-01 | 485.27 | OEt | A01 | a1 | B04 | b3 | C01 | c1 | D01 |
| B1M026-01 | 486.26 | OEt | A02 | a1 | B04 | b3 | C01 | c1 | D01 |
| B1M027-01 | 489.24 | OEt | A01 | a1 | B06 | b3 | C01 | c1 | D01 |
| B1M028-01 | 490.24 | OEt | A02 | a1 | B06 | b3 | C01 | c1 | D01 |
| B1M029-01 | 495.25 | OEt | A01 | a2 | B01 | b3 | C01 | c1 | D01 |
| B1M030-01 | 496.25 | OEt | A02 | a2 | B01 | b3 | C01 | c1 | D01 |
| B1M031-01 | 496.25 | OEt | A01 | a2 | B17 | b3 | C01 | c1 | D01 |
| B1M032-01 | 497.24 | OEt | A02 | a2 | B17 | b3 | C01 | c1 | D01 |
| B1M033-01 | 499.28 | OEt | A01 | a1 | B02 | b3 | C01 | c1 | D01 |
| B1M034-01 | 500.28 | OEt | A02 | a1 | B02 | b3 | C01 | c1 | D01 |
| B1M035-01 | 521.27 | OEt | A01 | a1 | B09 | b3 | C01 | c1 | D01 |
| B1M036-01 | 522.26 | OEt | A02 | a1 | B09 | b3 | C01 | c1 | D01 |
| B1M037-01 | 527.32 | OEt | A01 | a1 | B16 | b3 | C01 | c1 | D01 |
| B1M038-01 | 528.31 | OEt | A02 | a1 | B16 | b3 | C01 | c1 | D01 |
| B1M039-01 | 539.24 | OEt | A01 | a1 | B11 | b3 | C01 | c1 | D01 |
| B1M040-01 | 540.24 | OEt | A02 | a1 | B11 | b3 | C01 | c1 | D01 |

| | | | | | | | | | |
|---|---|---|---|---|---|---|---|---|---|
| B1M041-01 | 563.24 | OEt | A01 | a2 | B11 | b3 | C01 | c1 | D01 |
| B1M042-01 | 564.24 | OEt | A02 | a2 | B11 | b3 | C01 | c1 | D01 |
| B1M043-01 | 605.26 | OEt | A01 | a1 | B12 | b3 | C01 | c1 | D01 |
| B1M044-01 | 606.26 | OEt | A02 | a1 | B12 | b3 | C01 | c1 | D01 |
| B1M045-01 | 457.27 | OEt | A01 | a1 | B01 | b1 | C01 | c1 | D01 |
| B1M046-01 | 458.27 | OEt | A02 | a1 | B01 | b1 | C01 | c1 | D01 |
| B1M047-01 | 471.29 | OEt | A01 | a1 | B04 | b1 | C01 | c1 | D01 |
| B1M048-01 | 472.28 | OEt | A02 | a1 | B04 | b1 | C01 | c1 | D01 |
| B1M049-01 | 485.3 | OEt | A01 | a1 | B05 | b1 | C01 | c1 | D01 |
| B1M050-01 | 486.3 | OEt | A02 | a1 | B05 | b1 | C01 | c1 | D01 |
| B1M051-01 | 475.26 | OEt | A01 | a1 | B06 | b1 | C01 | c1 | D01 |
| B1M052-01 | 476.26 | OEt | A02 | a1 | B06 | b1 | C01 | c1 | D01 |
| B1M053-01 | 487.28 | OEt | A01 | a1 | B07 | b1 | C01 | c1 | D01 |
| B1M054-01 | 488.28 | OEt | A02 | a1 | B07 | b1 | C01 | c1 | D01 |
| B1M055-01 | 501.3 | OEt | A01 | a1 | B14 | b1 | C01 | c1 | D01 |
| B1M056-01 | 502.29 | OEt | A02 | a1 | B14 | b1 | C01 | c1 | D01 |
| B1M057-01 | 515.32 | OEt | A01 | a1 | B15 | b1 | C01 | c1 | D01 |
| B1M058-01 | 516.31 | OEt | A02 | a1 | B15 | b1 | C01 | c1 | D01 |
| B1M059-01 | 517.29 | OEt | A01 | a1 | B08 | b1 | C01 | c1 | D01 |
| B1M060-01 | 518.29 | OEt | A02 | a1 | B08 | b1 | C01 | c1 | D01 |
| B1M061-01 | 525.26 | OEt | A01 | a1 | B11 | b1 | C01 | c1 | D01 |
| B1M062-01 | 526.26 | OEt | A02 | a1 | B11 | b1 | C01 | c1 | D01 |
| B1M063-01 | 541.26 | OEt | A01 | a1 | B13 | b1 | C01 | c1 | D01 |
| B1M064-01 | 542.25 | OEt | A02 | a1 | B13 | b1 | C01 | c1 | D01 |
| B1M065-01 | 507.29 | OEt | A01 | a1 | B09 | b1 | C01 | c1 | D01 |
| B1M066-01 | 508.28 | OEt | A02 | a1 | B09 | b1 | C01 | c1 | D01 |
| B1M067-01 | 463.23 | OEt | A01 | a1 | B10 | b1 | C01 | c1 | D01 |
| B1M068-01 | 464.23 | OEt | A02 | a1 | B10 | b1 | C01 | c1 | D01 |
| B1M069-01 | 481.27 | OEt | A01 | a2 | B01 | b1 | C01 | c1 | D01 |
| B1M070-01 | 482.27 | OEt | A02 | a2 | B01 | b1 | C01 | c1 | D01 |
| B1M071-01 | 459.26 | OEt | A01 | a1 | B01 | b1 | C02 | c1 | D01 |
| B1M072-01 | 460.26 | OEt | A02 | a1 | B01 | b1 | C02 | c1 | D01 |
| B1M073-01 | 473.28 | OEt | A01 | a1 | B04 | b1 | C02 | c1 | D01 |
| B1M074-01 | 474.27 | OEt | A02 | a1 | B04 | b1 | C02 | c1 | D01 |
| B1M075-01 | 487.3 | OEt | A01 | a1 | B05 | b1 | C02 | c1 | D01 |
| B1M076-01 | 488.29 | OEt | A02 | a1 | B05 | b1 | C02 | c1 | D01 |
| B1M077-01 | 477.25 | OEt | A01 | a1 | B06 | b1 | C02 | c1 | D01 |
| B1M078-01 | 478.25 | OEt | A02 | a1 | B06 | b1 | C02 | c1 | D01 |
| B1M079-01 | 489.27 | OEt | A01 | a1 | B07 | b1 | C02 | c1 | D01 |
| B1M080-01 | 490.27 | OEt | A02 | a1 | B07 | b1 | C02 | c1 | D01 |
| B1M081-01 | 503.29 | OEt | A01 | a1 | B14 | b1 | C02 | c1 | D01 |
| B1M082-01 | 504.29 | OEt | A02 | a1 | B14 | b1 | C02 | c1 | D01 |
| B1M083-01 | 517.31 | OEt | A01 | a1 | B15 | b1 | C02 | c1 | D01 |
| B1M084-01 | 518.3 | OEt | A02 | a1 | B15 | b1 | C02 | c1 | D01 |
| B1M085-01 | 519.29 | OEt | A01 | a1 | B08 | b1 | C02 | c1 | D01 |
| B1M086-01 | 520.28 | OEt | A02 | a1 | B08 | b1 | C02 | c1 | D01 |
| B1M087-01 | 527.25 | OEt | A01 | a1 | B11 | b1 | C02 | c1 | D01 |

| | | | | | | | | | |
|---|---|---|---|---|---|---|---|---|---|
| B1M088-01 | 528.25 | OEt | A02 | a1 | B11 | b1 | C02 | c1 | D01 |
| B1M089-01 | 543.25 | OEt | A01 | a1 | B13 | b1 | C02 | c1 | D01 |
| B1M090-01 | 544.24 | OEt | A02 | a1 | B13 | b1 | C02 | c1 | D01 |
| B1M091-01 | 509.28 | OEt | A01 | a1 | B09 | b1 | C02 | c1 | D01 |
| B1M092-01 | 510.27 | OEt | A02 | a1 | B09 | b1 | C02 | c1 | D01 |
| B1M093-01 | 465.22 | OEt | A01 | a1 | B10 | b1 | C02 | c1 | D01 |
| B1M094-01 | 466.22 | OEt | A02 | a1 | B10 | b1 | C02 | c1 | D01 |
| B1M095-01 | 483.26 | OEt | A01 | a2 | B01 | b1 | C02 | c1 | D01 |
| B1M096-01 | 484.26 | OEt | A02 | a2 | B01 | b1 | C02 | c1 | D01 |
| B1M097-01 | 471.29 | OEt | A01 | a1 | B03 | b2 | C01 | c1 | D01 |
| B1M098-01 | 472.28 | OEt | A02 | a1 | B03 | b2 | C01 | c1 | D01 |
| B1M099-01 | 489.28 | OEt | A01 | a1 | B18 | b2 | C01 | c1 | D01 |
| B1M100-01 | 490.27 | OEt | A02 | a1 | B18 | b2 | C01 | c1 | D01 |

[Table 246]

[Table AI-03-B1K-01]

| Mixture No. | mixAI-B1K-01 |
|---|---|
| No. of compound that may be contained | Compound name |
| B1K001-01 | 6-[4-[4-(3-Ethoxyphenyl)thiophene-2-carbonyl]piperazin-1-yl]-N-(2,2,2-trifluoroethyl)pyridazine-3-carboxamide |
| B1K002-01 | 6-[4-[4-(5-Ethoxypyridin-3-yl)thiophene-2-carbonyl]piperazin-1-yl]-N-(2,2,2-trifluoroethyl)pyridazine-3-carboxamide |
| B1K003-01 | 6-[4-[4-(3-Ethoxyphenyl)-3-methylbenzoyl]piperazin-1-yl]-N-(2,2,2-trifluoroethyl)pyridazine-3-carboxamide |
| B1K004-01 | 6-[4-[4-(5-Ethoxypyridin-3-yl)-3-methylbenzoyl]piperazin-1-yl]-N-(2,2,2-trifluoroethyl)pyridazine-3-carboxamide |
| B1K005-01 | 6-[4-[4-(3-Ethoxyphenyl)-3-fluorobenzoyl]piperazin-1-yl]-N-(2,2,2-trifluoroethyl)pyridazine-3-carboxamide |
| B1K006-01 | 6-[4-[4-(5-Ethoxypyridin-3-yl)-3-fluorobenzoyl]piperazin-1-yl]-N-(2,2,2-trifluoroethyl)pyridazine-3-carboxamide |
| B1K007-01 | 6-[4-[2-[2-(3-Ethoxyphenyl)ethynyl]benzoyl]piperazin-1-yl]-N-(2,2,2-trifluoroethyl)pyridazine-3-carboxamide |
| B1K008-01 | 6-[4-[2-[2-(5-Ethoxypyridin-3-yl)ethynyl]benzoyl]piperazin-1-yl]-N-(2,2,2-trifluoroethyl)pyridazine-3-carboxamide |
| B1K009-01 | 6-[4-[5-[2-(3-Ethoxyphenyl)ethynyl]pyridine-3-carbonyl]piperazin-1-yl]-N-(2,2,2-trifluoroethyl)pyridazine-3-carboxamide |
| B1K010-01 | 6-[4-[5-[2-(5-Ethoxypyridin-3-yl)ethynyl]pyridine-3-carbonyl]piperazin-1-yl]-N-(2,2,2-trifluoroethyl)pyridazine-3-carboxamide |
| B1K011-01 | 6-[4-[3-[2-(3-Ethoxyphenyl)phenyl]propanoyl]piperazin-1-yl]-N-(2,2,2-trifluoroethyl)pyridazine-3-carboxamide |
| B1K012-01 | 6-[4-[3-[2-(5-Ethoxypyridin-3-yl)phenyl]propanoyl]piperazin-1-yl]-N-(2,2,2-trifluoroethyl)pyridazine-3-carboxamide |
| B1K013-01 | 6-[4-[4-(3-Ethoxyphenyl)naphthalene-1-carbonyl]piperazin-1-yl]-N-(2,2,2-trifluoroethyl)pyridazine-3-carboxamide |
| B1K014-01 | 6-[4-[4-(5-Ethoxypyridin-3-yl)naphthalene-1-carbonyl]piperazin-1-yl]-N-(2,2,2-trifluoroethyl)pyridazine-3-carboxamide |
| B1K015-01 | 6-[4-[3-tert-Butyl-5-(3-ethoxyphenyl)benzoyl]piperazin-1-yl]-N-(2,2,2-trifluoroethyl)pyridazine-3-carboxamide |
| B1K016-01 | 6-[4-[3-tert-Butyl-5-(5-ethoxypyridin-3-yl)benzoyl]piperazin-1-yl]-N-(2,2,2-trifluoroethyl)pyridazine-3-carboxamide |

| | |
|---|---|
| B1K017-01 | 6-[4-[3-(3-Ethoxyphenyl)-5-(trifluoromethyl)benzoyl]piperazin-1-yl]-N-(2,2,2-trifluoroethyl)pyridazine-3-carboxamide |
| B1K018-01 | 6-[4-[3-(5-Ethoxypyridin-3-yl)-5-(trifluoromethyl)benzoyl]piperazin-1-yl]-N-(2,2,2-trifluoroethyl)pyridazine-3-carboxamide |
| B1K019-01 | 6-[4-[3-[2-(3-Ethoxyphenyl)ethynyl]-5-(trifluoromethyl)benzoyl]piperazin-1-yl]-N-(2,2,2-trifluoroethyl)pyridazine-3-carboxamide |
| B1K020-01 | 6-[4-[3-[2-(5-Ethoxypyridin-3-yl)ethynyl]-5-(trifluoromethyl)benzoyl]piperazin-1-yl]-N-(2,2,2-trifluoroethyl)pyridazine-3-carboxamide |
| B1K021-01 | 6-[4-[3-[4-(3-Ethoxyphenyl)pyrazol-1-yl]-5-(trifluoromethyl)benzoyl]piperazin-1-yl]-N-(2,2,2-trifluoroethyl)pyridazine-3-carboxamide |
| B1K022-01 | 6-[4-[3-[4-(5-Ethoxypyridin-3-yl)pyrazol-1-yl]-5-(trifluoromethyl)benzoyl]piperazin-1-yl]-N-(2,2,2-trifluoroethyl)pyridazine-3-carboxamide |
| B1K023-01 | 4-[4-[4-(3-Ethoxyphenyl)thiophene-2-carbonyl]piperazin-1-yl]-N-(2,2,2-trifluoroethyl)benzamide |
| B1K024-01 | 4-[4-[4-(5-Ethoxypyridin-3-yl)thiophene-2-carbonyl]piperazin-1-yl]-N-(2,2,2-trifluoroethyl)benzamide |
| B1K025-01 | 4-[4-[4-(3-Ethoxyphenyl)-3-methylbenzoyl]piperazin-1-yl]-N-(2,2,2-trifluoroethyl)benzamide |
| B1K026-01 | 4-[4-[4-(5-Ethoxypyridin-3-yl)-3-methylbenzoyl]piperazin-1-yl]-N-(2,2,2-trifluoroethyl)benzamide |
| B1K027-01 | 4-[4-[4-(3-Ethoxyphenyl)-3-fluorobenzoyl]piperazin-1-yl]-N-(2,2,2-trifluoroethyl)benzamide |
| B1K028-01 | 4-[4-[4-(5-Ethoxypyridin-3-yl)-3-fluorobenzoyl]piperazin-1-yl]-N-(2,2,2-trifluoroethyl)benzamide |
| B1K029-01 | 4-[4-[2-[2-(3-Ethoxyphenyl)ethynyl]benzoyl]piperazin-1-yl]-N-(2,2,2-trifluoroethyl)benzamide |
| B1K030-01 | 4-[4-[2-[2-(5-Ethoxypyridin-3-yl)ethynyl]benzoyl]piperazin-1-yl]-N-(2,2,2-trifluoroethyl)benzamide |
| B1K031-01 | 4-[4-[5-[2-(3-Ethoxyphenyl)ethynyl]pyridine-3-carbonyl]piperazin-1-yl]-N-(2,2,2-trifluoroethyl)benzamide |
| B1K032-01 | 4-[4-[5-[2-(5-Ethoxypyridin-3-yl)ethynyl]pyridine-3-carbonyl]piperazin-1-yl]-N-(2,2,2-trifluoroethyl)benzamide |
| B1K033-01 | 4-[4-[3-[2-(3-Ethoxyphenyl)phenyl]propanoyl]piperazin-1-yl]-N-(2,2,2-trifluoroethyl)benzamide |
| B1K034-01 | 4-[4-[3-[2-(5-Ethoxypyridin-3-yl)phenyl]propanoyl]piperazin-1-yl]-N-(2,2,2-trifluoroethyl)benzamide |
| B1K035-01 | 4-[4-[4-(3-Ethoxyphenyl)naphthalene-1-carbonyl]piperazin-1-yl]-N-(2,2,2-trifluoroethyl)benzamide |
| B1K036-01 | 4-[4-[4-(5-Ethoxypyridin-3-yl)naphthalene-1-carbonyl]piperazin-1-yl]-N-(2,2,2-trifluoroethyl)benzamide |
| B1K037-01 | 4-[4-[3-tert-Butyl-5-(3-ethoxyphenyl)benzoyl]piperazin-1-yl]-N-(2,2,2-trifluoroethyl)benzamide |
| B1K038-01 | 4-[4-[3-tert-Butyl-5-(5-ethoxypyridin-3-yl)benzoyl]piperazin-1-yl]-N-(2,2,2-trifluoroethyl)benzamide |
| B1K039-01 | 4-[4-[3-(3-Ethoxyphenyl)-5-(trifluoromethyl)benzoyl]piperazin-1-yl]-N-(2,2,2-trifluoroethyl)benzamide |
| B1K040-01 | 4-[4-[3-(5-Ethoxypyridin-3-yl)-5-(trifluoromethyl)benzoyl]piperazin-1-yl]-N-(2,2,2-trifluoroethyl)benzamide |
| B1K041-01 | 4-[4-[3-[2-(3-Ethoxyphenyl)ethynyl]-5-(trifluoromethyl)benzoyl]piperazin-1-yl]-N-(2,2,2-trifluoroethyl)benzamide |
| B1K042-01 | 4-[4-[3-[2-(5-Ethoxypyridin-3-yl)ethynyl]-5-(trifluoromethyl)benzoyl]piperazin-1-yl]-N-(2,2,2-trifluoroethyl)benzamide |
| B1K043-01 | 4-[4-[3-[4-(3-Ethoxyphenyl)pyrazol-1-yl]-5-(trifluoromethyl)benzoyl]piperazin-1-yl]-N-(2,2,2-trifluoroethyl)benzamide |
| B1K044-01 | 4-[4-[3-[4-(5-Ethoxypyridin-3-yl)pyrazol-1-yl]-5-(trifluoromethyl)benzoyl]piperazin-1-yl]-N-(2,2,2-trifluoroethyl)benzamide |

| | |
|---|---|
| B1K045-01 | 4-[4-[[2-(3-Ethoxyphenyl)phenyl]methyl]piperazin-1-yl]-N-(2,2,2-trifluoroethyl)benzamide |
| B1K046-01 | 4-[4-[[2-(5-Ethoxypyridin-3-yl)phenyl]methyl]piperazin-1-yl]-N-(2,2,2-trifluoroethyl)benzamide |
| B1K047-01 | 4-[4-[[4-(3-Ethoxyphenyl)-3-methylphenyl]methyl]piperazin-1-yl]-N-(2,2,2-trifluoroethyl)benzamide |
| B1K048-01 | 4-[4-[[4-(5-Ethoxypyridin-3-yl)-3-methylphenyl]methyl]piperazin-1-yl]-N-(2,2,2-trifluoroethyl)benzamide |
| B1K049-01 | 4-[4-[[4-(3-Ethoxyphenyl)-2-ethylphenyl]methyl]piperazin-1-yl]-N-(2,2,2-trifluoroethyl)benzamide |
| B1K050-01 | 4-[4-[[4-(5-Ethoxypyridin-3-yl)-2-ethylphenyl]methyl]piperazin-1-yl]-N-(2,2,2-trifluoroethyl)benzamide |
| B1K051-01 | 4-[4-[[4-(3-Ethoxyphenyl)-3-fluorophenyl]methyl]piperazin-1-yl]-N-(2,2,2-trifluoroethyl)benzamide |
| B1K052-01 | 4-[4-[[4-(5-Ethoxypyridin-3-yl)-3-fluorophenyl]methyl]piperazin-1-yl]-N-(2,2,2-trifluoroethyl)benzamide |
| B1K053-01 | 4-[4-[[4-(3-Ethoxyphenyl)-2-methoxyphenyl]methyl]piperazin-1-yl]-N-(2,2,2-trifluoroethyl)benzamide |
| B1K054-01 | 4-[4-[[4-(5-Ethoxypyridin-3-yl)-2-methoxyphenyl]methyl]piperazin-1-yl]-N-(2,2,2-trifluoroethyl)benzamide |
| B1K055-01 | 4-[4-[[3-Ethoxy-5-(3-ethoxyphenyl)phenyl]methyl]piperazin-1-yl]-N-(2,2,2-trifluoroethyl)benzamide |
| B1K056-01 | 4-[4-[[3-Ethoxy-5-(5-ethoxypyridin-3-yl)phenyl]methyl]piperazin-1-yl]-N-(2,2,2-trifluoroethyl)benzamide |
| B1K057-01 | 4-[4-[[3-(3-Ethoxyphenyl)-5-propan-2-yloxyphenyl]methyl]piperazin-1-yl]-N-(2,2,2-trifluoroethyl)benzamide |
| B1K058-01 | 4-[4-[[3-(5-Ethoxypyridin-3-yl)-5-propan-2-yloxyphenyl]methyl]piperazin-1-yl]-N-(2,2,2-trifluoroethyl)benzamide |
| B1K059-01 | 4-[4-[[4-(3-Ethoxyphenyl)-2,6-dimethoxyphenyl]methyl]piperazin-1-yl]-N-(2,2,2-trifluoroethyl)benzamide |
| B1K060-01 | 4-[4-[[4-(5-Ethoxypyridin-3-yl)-2,6-dimethoxyphenyl]methyl]piperazin-1-yl]-N-(2,2,2-trifluoroethyl)benzamide |
| B1K061-01 | 4-[4-[[3-(3-Ethoxyphenyl)-5-(trifluoromethyl)phenyl]methyl]piperazin-1-yl]-N-(2,2,2-trifluoroethyl)benzamide |
| B1K062-01 | 4-[4-[[3-(5-Ethoxypyridin-3-yl)-5-(trifluoromethyl)phenyl]methyl]piperazin-1-yl]-N-(2,2,2-trifluoroethyl)benzamide |
| B1K063-01 | 4-[4-[[3-(3-Ethoxyphenyl)-5-(trifluoromethoxy)phenyl]methyl]piperazin-1-yl]-N-(2,2,2-trifluoroethyl)benzamide |
| B1K064-01 | 4-[4-[[3-(5-Ethoxypyridin-3-yl)-5-(trifluoromethoxy)phenyl]methyl]piperazin-1-yl]-N-(2,2,2-trifluoroethyl)benzamide |
| B1K065-01 | 4-[4-[[4-(3-Ethoxyphenyl)naphthalen-1-yl]methyl]piperazin-1-yl]-N-(2,2,2-trifluoroethyl)benzamide |
| B1K066-01 | 4-[4-[[4-(5-Ethoxypyridin-3-yl)naphthalen-1-yl]methyl]piperazin-1-yl]-N-(2,2,2-trifluoroethyl)benzamide |
| B1K067-01 | 4-[4-[[4-(3-Ethoxyphenyl)thiophen-2-yl]methyl]piperazin-1-yl]-N-(2,2,2-trifluoroethyl)benzamide |
| B1K068-01 | 4-[4-[[4-(5-Ethoxypyridin-3-yl)thiophen-2-yl]methyl]piperazin-1-yl]-N-(2,2,2-trifluoroethyl)benzamide |
| B1K069-01 | 4-[4-[[2-[2-(3-Ethoxyphenyl)ethynyl]phenyl]methyl]piperazin-1-yl]-N-(2,2,2-trifluoroethyl)benzamide |
| B1K070-01 | 4-[4-[[2-[2-(5-Ethoxypyridin-3-yl)ethynyl]phenyl]methyl]piperazin-1-yl]-N-(2,2,2-trifluoroethyl)benzamide |
| B1K071-01 | 6-[4-[[2-(3-Ethoxyphenyl)phenyl]methyl]piperazin-1-yl]-N-(2,2,2-trifluoroethyl)pyridazine-3-carboxamide |
| B1K072-01 | 6-[4-[[2-(5-Ethoxypyridin-3-yl)phenyl]methyl]piperazin-1-yl]-N-(2,2,2-trifluoroethyl)pyridazine-3-carboxamide |

| | |
|---|---|
| B1K073-01 | 6-[4-[[4-(3-Ethoxyphenyl)-3-methylphenyl]methyl]piperazin-1-yl]-N-(2,2,2-trifluoroethyl)pyridazine-3-carboxamide |
| B1K074-01 | 6-[4-[[4-(5-Ethoxypyridin-3-yl)-3-methylphenyl]methyl]piperazin-1-yl]-N-(2,2,2-trifluoroethyl)pyridazine-3-carboxamide |
| B1K075-01 | 6-[4-[[4-(3-Ethoxyphenyl)-2-ethylphenyl]methyl]piperazin-1-yl]-N-(2,2,2-trifluoroethyl)pyridazine-3-carboxamide |
| B1K076-01 | 6-[4-[[4-(5-Ethoxypyridin-3-yl)-2-ethylphenyl]methyl]piperazin-1-yl]-N-(2,2,2-trifluoroethyl)pyridazine-3-carboxamide |
| B1K077-01 | 6-[4-[[4-(3-Ethoxyphenyl)-3-fluorophenyl]methyl]piperazin-1-yl]-N-(2,2,2-trifluoroethyl)pyridazine-3-carboxamide |
| B1K078-01 | 6-[4-[[4-(5-Ethoxypyridin-3-yl)-3-fluorophenyl]methyl]piperazin-1-yl]-N-(2,2,2-trifluoroethyl)pyridazine-3-carboxamide |
| B1K079-01 | 6-[4-[[4-(3-Ethoxyphenyl)-2-methoxyphenyl]methyl]piperazin-1-yl]-N-(2,2,2-trifluoroethyl)pyridazine-3-carboxamide |
| B1K080-01 | 6-[4-[[4-(5-Ethoxypyridin-3-yl)-2-methoxyphenyl]methyl]piperazin-1-yl]-N-(2,2,2-trifluoroethyl)pyridazine-3-carboxamide |
| B1K081-01 | 6-[4-[[3-Ethoxy-5-(3-ethoxyphenyl)phenyl]methyl]piperazin-1-yl]-N-(2,2,2-trifluoroethyl)pyridazine-3-carboxamide |
| B1K082-01 | 6-[4-[[3-Ethoxy-5-(5-ethoxypyridin-3-yl)phenyl]methyl]piperazin-1-yl]-N-(2,2,2-trifluoroethyl)pyridazine-3-carboxamide |
| B1K083-01 | 6-[4-[[3-(3-Ethoxyphenyl)-5-propan-2-yloxyphenyl]methyl]piperazin-1-yl]-N-(2,2,2-trifluoroethyl)pyridazine-3-carboxamide |
| B1K084-01 | 6-[4-[[3-(5-Ethoxypyridin-3-yl)-5-propan-2-yloxyphenyl]methyl]piperazin-1-yl]-N-(2,2,2-trifluoroethyl)pyridazine-3-carboxamide |
| B1K085-01 | 6-[4-[[4-(3-Ethoxyphenyl)-2,6-dimethoxyphenyl]methyl]piperazin-1-yl]-N-(2,2,2-trifluoroethyl)pyridazine-3-carboxamide |
| B1K086-01 | 6-[4-[[4-(5-Ethoxypyridin-3-yl)-2,6-dimethoxyphenyl]methyl]piperazin-1-yl]-N-(2,2,2-trifluoroethyl)pyridazine-3-carboxamide |
| B1K087-01 | 6-[4-[[3-(3-Ethoxyphenyl)-5-(trifluoromethyl)phenyl]methyl]piperazin-1-yl]-N-(2,2,2-trifluoroethyl)pyridazine-3-carboxamide |
| B1K088-01 | 6-[4-[[3-(5-Ethoxypyridin-3-yl)-5-(trifluoromethyl)phenyl]methyl]piperazin-1-yl]-N-(2,2,2-trifluoroethyl)pyridazine-3-carboxamide |
| B1K089-01 | 6-[4-[[3-(3-Ethoxyphenyl)-5-(trifluoromethoxy)phenyl]methyl]piperazin-1-yl]-N-(2,2,2-trifluoroethyl)pyridazine-3-carboxamide |
| B1K090-01 | 6-[4-[[3-(5-Ethoxypyridin-3-yl)-5-(trifluoromethoxy)phenyl]methyl]piperazin-1-yl]-N-(2,2,2-trifluoroethyl)pyridazine-3-carboxamide |
| B1K091-01 | 6-[4-[[4-(3-Ethoxyphenyl)naphthalen-1-yl]methyl]piperazin-1-yl]-N-(2,2,2-trifluoroethyl)pyridazine-3-carboxamide |
| B1K092-01 | 6-[4-[[4-(5-Ethoxypyridin-3-yl)naphthalen-1-yl]methyl]piperazin-1-yl]-N-(2,2,2-trifluoroethyl)pyridazine-3-carboxamide |
| B1K093-01 | 6-[4-[[4-(3-Ethoxyphenyl)thiophen-2-yl]methyl]piperazin-1-yl]-N-(2,2,2-trifluoroethyl)pyridazine-3-carboxamide |
| B1K094-01 | 6-[4-[[4-(5-Ethoxypyridin-3-yl)thiophen-2-yl]methyl]piperazin-1-yl]-N-(2,2,2-trifluoroethyl)pyridazine-3-carboxamide |
| B1K095-01 | 6-[4-[[2-[2-(3-Ethoxyphenyl)ethynyl]phenyl]methyl]piperazin-1-yl]-N-(2,2,2-trifluoroethyl)pyridazine-3-carboxamide |
| B1K096-01 | 6-[4-[[2-[2-(5-Ethoxypyridin-3-yl)ethynyl]phenyl]methyl]piperazin-1-yl]-N-(2,2,2-trifluoroethyl)pyridazine-3-carboxamide |
| B1K097-01 | 4-[4-[1-[4-(3-Ethoxyphenyl)phenyl]ethyl]piperazin-1-yl]-N-(2,2,2-trifluoroethyl)benzamide |
| B1K098-01 | 4-[4-[1-[4-(5-Ethoxypyridin-3-yl)phenyl]ethyl]piperazin-1-yl]-N-(2,2,2-trifluoroethyl)benzamide |
| B1K099-01 | 4-[4-[1-[4-(3-Ethoxyphenyl)-2-fluorophenyl]ethyl]piperazin-1-yl]-N-(2,2,2-trifluoroethyl)benzamide |
| B1K100-01 | 4-[4-[1-[4-(5-Ethoxypyridin-3-yl)-2-fluorophenyl]ethyl]piperazin-1-yl]-N-(2,2,2-trifluoroethyl)benzamide |

[Table 247]

[Table AI-03-B1K-01]

| Label | Exact | ☒ | A | a | B | b | C | c | D |
|---|---|---|---|---|---|---|---|---|---|
| B1K001-01 | 519.16 | OEt | A01 | a1 | B10 | b3 | C02 | c1 | D02 |
| B1K002-01 | 520.15 | OEt | A02 | a1 | B10 | b3 | C02 | c1 | D02 |
| B1K003-01 | 527.21 | OEt | A01 | a1 | B04 | b3 | C02 | c1 | D02 |
| B1K004-01 | 528.21 | OEt | A02 | a1 | B04 | b3 | C02 | c1 | D02 |
| B1K005-01 | 531.19 | OEt | A01 | a1 | B06 | b3 | C02 | c1 | D02 |
| B1K006-01 | 532.19 | OEt | A02 | a1 | B06 | b3 | C02 | c1 | D02 |
| B1K007-01 | 537.2 | OEt | A01 | a2 | B01 | b3 | C02 | c1 | D02 |
| B1K008-01 | 538.19 | OEt | A02 | a2 | B01 | b3 | C02 | c1 | D02 |
| B1K009-01 | 538.19 | OEt | A01 | a2 | B17 | b3 | C02 | c1 | D02 |
| B1K010-01 | 539.19 | OEt | A02 | a2 | B17 | b3 | C02 | c1 | D02 |
| B1K011-01 | 541.23 | OEt | A01 | a1 | B02 | b3 | C02 | c1 | D02 |
| B1K012-01 | 542.23 | OEt | A02 | a1 | B02 | b3 | C02 | c1 | D02 |
| B1K013-01 | 563.21 | OEt | A01 | a1 | B09 | b3 | C02 | c1 | D02 |
| B1K014-01 | 564.21 | OEt | A02 | a1 | B09 | b3 | C02 | c1 | D02 |
| B1K015-01 | 569.26 | OEt | A01 | a1 | B16 | b3 | C02 | c1 | D02 |
| B1K016-01 | 570.26 | OEt | A02 | a1 | B16 | b3 | C02 | c1 | D02 |
| B1K017-01 | 581.19 | OEt | A01 | a1 | B11 | b3 | C02 | c1 | D02 |
| B1K018-01 | 582.18 | OEt | A02 | a1 | B11 | b3 | C02 | c1 | D02 |
| B1K019-01 | 605.19 | OEt | A01 | a2 | B11 | b3 | C02 | c1 | D02 |
| B1K020-01 | 606.18 | OEt | A02 | a2 | B11 | b3 | C02 | c1 | D02 |
| B1K021-01 | 647.21 | OEt | A01 | a1 | B12 | b3 | C02 | c1 | D02 |
| B1K022-01 | 648.2 | OEt | A02 | a1 | B12 | b3 | C02 | c1 | D02 |
| B1K023-01 | 517.17 | OEt | A01 | a1 | B10 | b3 | C01 | c1 | D02 |
| B1K024-01 | 518.16 | OEt | A02 | a1 | B10 | b3 | C01 | c1 | D02 |
| B1K025-01 | 525.22 | OEt | A01 | a1 | B04 | b3 | C01 | c1 | D02 |
| B1K026-01 | 526.22 | OEt | A02 | a1 | B04 | b3 | C01 | c1 | D02 |
| B1K027-01 | 529.2 | OEt | A01 | a1 | B06 | b3 | C01 | c1 | D02 |
| B1K028-01 | 530.19 | OEt | A02 | a1 | B06 | b3 | C01 | c1 | D02 |
| B1K029-01 | 535.21 | OEt | A01 | a2 | B01 | b3 | C01 | c1 | D02 |
| B1K030-01 | 536.2 | OEt | A02 | a2 | B01 | b3 | C01 | c1 | D02 |
| B1K031-01 | 536.2 | OEt | A01 | a2 | B17 | b3 | C01 | c1 | D02 |
| B1K032-01 | 537.2 | OEt | A02 | a2 | B17 | b3 | C01 | c1 | D02 |
| B1K033-01 | 539.24 | OEt | A01 | a1 | B02 | b3 | C01 | c1 | D02 |
| B1K034-01 | 540.24 | OEt | A02 | a1 | B02 | b3 | C01 | c1 | D02 |
| B1K035-01 | 561.22 | OEt | A01 | a1 | B09 | b3 | C01 | c1 | D02 |
| B1K036-01 | 562.22 | OEt | A02 | a1 | B09 | b3 | C01 | c1 | D02 |
| B1K037-01 | 567.27 | OEt | A01 | a1 | B16 | b3 | C01 | c1 | D02 |
| B1K038-01 | 568.27 | OEt | A02 | a1 | B16 | b3 | C01 | c1 | D02 |
| B1K039-01 | 579.2 | OEt | A01 | a1 | B11 | b3 | C01 | c1 | D02 |
| B1K040-01 | 580.19 | OEt | A02 | a1 | B11 | b3 | C01 | c1 | D02 |
| B1K041-01 | 603.2 | OEt | A01 | a2 | B11 | b3 | C01 | c1 | D02 |
| B1K042-01 | 604.19 | OEt | A02 | a2 | B11 | b3 | C01 | c1 | D02 |

| | | | | | | | | | |
|---|---|---|---|---|---|---|---|---|---|
| B1K043-01 | 645.22 | OEt | A01 | a1 | B12 | b3 | C01 | c1 | D02 |
| B1K044-01 | 646.21 | OEt | A02 | a1 | B12 | b3 | C01 | c1 | D02 |
| B1K045-01 | 497.23 | OEt | A01 | a1 | B01 | b1 | C01 | c1 | D02 |
| B1K046-01 | 498.22 | OEt | A02 | a1 | B01 | b1 | C01 | c1 | D02 |
| B1K047-01 | 511.25 | OEt | A01 | a1 | B04 | b1 | C01 | c1 | D02 |
| B1K048-01 | 512.24 | OEt | A02 | a1 | B04 | b1 | C01 | c1 | D02 |
| B1K049-01 | 525.26 | OEt | A01 | a1 | B05 | b1 | C01 | c1 | D02 |
| B1K050-01 | 526.26 | OEt | A02 | a1 | B05 | b1 | C01 | c1 | D02 |
| B1K051-01 | 515.22 | OEt | A01 | a1 | B06 | b1 | C01 | c1 | D02 |
| B1K052-01 | 516.22 | OEt | A02 | a1 | B06 | b1 | C01 | c1 | D02 |
| B1K053-01 | 527.24 | OEt | A01 | a1 | B07 | b1 | C01 | c1 | D02 |
| B1K054-01 | 528.24 | OEt | A02 | a1 | B07 | b1 | C01 | c1 | D02 |
| B1K055-01 | 541.26 | OEt | A01 | a1 | B14 | b1 | C01 | c1 | D02 |
| B1K056-01 | 542.25 | OEt | A02 | a1 | B14 | b1 | C01 | c1 | D02 |
| B1K057-01 | 555.27 | OEt | A01 | a1 | B15 | b1 | C01 | c1 | D02 |
| B1K058-01 | 556.27 | OEt | A02 | a1 | B15 | b1 | C01 | c1 | D02 |
| B1K059-01 | 557.25 | OEt | A01 | a1 | B08 | b1 | C01 | c1 | D02 |
| B1K060-01 | 558.25 | OEt | A02 | a1 | B08 | b1 | C01 | c1 | D02 |
| B1K061-01 | 565.22 | OEt | A01 | a1 | B11 | b1 | C01 | c1 | D02 |
| B1K062-01 | 566.21 | OEt | A02 | a1 | B11 | b1 | C01 | c1 | D02 |
| B1K063-01 | 581.21 | OEt | A01 | a1 | B13 | b1 | C01 | c1 | D02 |
| B1K064-01 | 582.21 | OEt | A02 | a1 | B13 | b1 | C01 | c1 | D02 |
| B1K065-01 | 547.25 | OEt | A01 | a1 | B09 | b1 | C01 | c1 | D02 |
| B1K066-01 | 548.24 | OEt | A02 | a1 | B09 | b1 | C01 | c1 | D02 |
| B1K067-01 | 503.19 | OEt | A01 | a1 | B10 | b1 | C01 | c1 | D02 |
| B1K068-01 | 504.18 | OEt | A02 | a1 | B10 | b1 | C01 | c1 | D02 |
| B1K069-01 | 521.23 | OEt | A01 | a2 | B01 | b1 | C01 | c1 | D02 |
| B1K070-01 | 522.22 | OEt | A02 | a2 | B01 | b1 | C01 | c1 | D02 |
| B1K071-01 | 499.22 | OEt | A01 | a1 | B01 | b1 | C02 | c1 | D02 |
| B1K072-01 | 500.22 | OEt | A02 | a1 | B01 | b1 | C02 | c1 | D02 |
| B1K073-01 | 513.24 | OEt | A01 | a1 | B04 | b1 | C02 | c1 | D02 |
| B1K074-01 | 514.23 | OEt | A02 | a1 | B04 | b1 | C02 | c1 | D02 |
| B1K075-01 | 527.25 | OEt | A01 | a1 | B05 | b1 | C02 | c1 | D02 |
| B1K076-01 | 528.25 | OEt | A02 | a1 | B05 | b1 | C02 | c1 | D02 |
| B1K077-01 | 517.21 | OEt | A01 | a1 | B06 | b1 | C02 | c1 | D02 |
| B1K078-01 | 518.21 | OEt | A02 | a1 | B06 | b1 | C02 | c1 | D02 |
| B1K079-01 | 529.23 | OEt | A01 | a1 | B07 | b1 | C02 | c1 | D02 |
| B1K080-01 | 530.23 | OEt | A02 | a1 | B07 | b1 | C02 | c1 | D02 |
| B1K081-01 | 543.25 | OEt | A01 | a1 | B14 | b1 | C02 | c1 | D02 |
| B1K082-01 | 544.24 | OEt | A02 | a1 | B14 | b1 | C02 | c1 | D02 |
| B1K083-01 | 557.26 | OEt | A01 | a1 | B15 | b1 | C02 | c1 | D02 |
| B1K084-01 | 558.26 | OEt | A02 | a1 | B15 | b1 | C02 | c1 | D02 |
| B1K085-01 | 559.24 | OEt | A01 | a1 | B08 | b1 | C02 | c1 | D02 |
| B1K086-01 | 560.24 | OEt | A02 | a1 | B08 | b1 | C02 | c1 | D02 |
| B1K087-01 | 567.21 | OEt | A01 | a1 | B11 | b1 | C02 | c1 | D02 |
| B1K088-01 | 568.2 | OEt | A02 | a1 | B11 | b1 | C02 | c1 | D02 |
| B1K089-01 | 583.2 | OEt | A01 | a1 | B13 | b1 | C02 | c1 | D02 |

| | | | | | | | | | |
|---|---|---|---|---|---|---|---|---|---|
| B1K090-01 | 584.2 | OEt | A02 | a1 | B13 | b1 | C02 | c1 | D02 |
| B1K091-01 | 549.24 | OEt | A01 | a1 | B09 | b1 | C02 | c1 | D02 |
| B1K092-01 | 550.23 | OEt | A02 | a1 | B09 | b1 | C02 | c1 | D02 |
| B1K093-01 | 505.18 | OEt | A01 | a1 | B10 | b1 | C02 | c1 | D02 |
| B1K094-01 | 506.17 | OEt | A02 | a1 | B10 | b1 | C02 | c1 | D02 |
| B1K095-01 | 523.22 | OEt | A01 | a2 | B01 | b1 | C02 | c1 | D02 |
| B1K096-01 | 524.22 | OEt | A02 | a2 | B01 | b1 | C02 | c1 | D02 |
| B1K097-01 | 511.25 | OEt | A01 | a1 | B03 | b2 | C01 | c1 | D02 |
| B1K098-01 | 512.24 | OEt | A02 | a1 | B03 | b2 | C01 | c1 | D02 |
| B1K099-01 | 529.24 | OEt | A01 | a1 | B18 | b2 | C01 | c1 | D02 |
| B1K100-01 | 530.23 | OEt | A02 | a1 | B18 | b2 | C01 | c1 | D02 |

[Table 248]

[Table AI-03-B1O-01]

| Mixture No. | mixAI-B1O-01 |
|---|---|
| No. of compound that may be contained | Compound name |
| B1O001-01 | 6-[4-[4-(3-Ethoxyphenyl)thiophene-2-carbonyl]piperazin-1-yl]-N-(4-methoxyphenyl)pyridazine-3-carboxamide |
| B1O002-01 | 6-[4-[4-(5-Ethoxypyridin-3-yl)thiophene-2-carbonyl]piperazin-1-yl]-N-(4-methoxyphenyl)pyridazine-3-carboxamide |
| B1O003-01 | 6-[4-[4-(3-Ethoxyphenyl)-3-methylbenzoyl]piperazin-1-yl]-N-(4-methoxyphenyl)pyridazine-3-carboxamide |
| B1O004-01 | 6-[4-[4-(5-Ethoxypyridin-3-yl)-3-methylbenzoyl]piperazin-1-yl]-N-(4-methoxyphenyl)pyridazine-3-carboxamide |
| B1O005-01 | 6-[4-[4-(3-Ethoxyphenyl)-3-fluorobenzoyl]piperazin-1-yl]-N-(4-methoxyphenyl)pyridazine-3-carboxamide |
| B1O006-01 | 6-[4-[4-(5-Ethoxypyridin-3-yl)-3-fluorobenzoyl]piperazin-1-yl]-N-(4-methoxyphenyl)pyridazine-3-carboxamide |
| B1O007-01 | 6-[4-[2-[2-(3-Ethoxyphenyl)ethynyl]benzoyl]piperazin-1-yl]-N-(4-methoxyphenyl)pyridazine-3-carboxamide |
| B1O008-01 | 6-[4-[2-[2-(5-Ethoxypyridin-3-yl)ethynyl]benzoyl]piperazin-1-yl]-N-(4-methoxyphenyl)pyridazine-3-carboxamide |
| B1O009-01 | 6-[4-[5-[2-(3-Ethoxyphenyl)ethynyl]pyridine-3-carbonyl]piperazin-1-yl]-N-(4-methoxyphenyl)pyridazine-3-carboxamide |
| B1O010-01 | 6-[4-[5-[2-(5-Ethoxypyridin-3-yl)ethynyl]pyridine-3-carbonyl]piperazin-1-yl]-N-(4-methoxyphenyl)pyridazine-3-carboxamide |
| B1O011-01 | 6-[4-[3-[2-(3-Ethoxyphenyl)phenyl]propanoyl]piperazin-1-yl]-N-(4-methoxyphenyl)pyridazine-3-carboxamide |
| B1O012-01 | 6-[4-[3-[2-(5-Ethoxypyridin-3-yl)phenyl]propanoyl]piperazin-1-yl]-N-(4-methoxyphenyl)pyridazine-3-carboxamide |
| B1O013-01 | 6-[4-[4-(3-Ethoxyphenyl)naphthalene-1-carbonyl]piperazin-1-yl]-N-(4-methoxyphenyl)pyridazine-3-carboxamide |
| B1O014-01 | 6-[4-[4-(5-Ethoxypyridin-3-yl)naphthalene-1-carbonyl]piperazin-1-yl]-N-(4-methoxyphenyl)pyridazine-3-carboxamide |
| B1O015-01 | 6-[4-[3-tert-Butyl-5-(3-ethoxyphenyl)benzoyl]piperazin-1-yl]-N-(4-methoxyphenyl)pyridazine-3-carboxamide |
| B1O016-01 | 6-[4-[3-tert-Butyl-5-(5-ethoxypyridin-3-yl)benzoyl]piperazin-1-yl]-N-(4-methoxyphenyl)pyridazine-3-carboxamide |
| B1O017-01 | 6-[4-[3-(3-Ethoxyphenyl)-5-(trifluoromethyl)benzoyl]piperazin-1-yl]-N-(4-methoxyphenyl)pyridazine-3-carboxamide |

| | |
|---|---|
| B1O018-01 | 6-[4-[3-(5-Ethoxypyridin-3-yl)-5-(trifluoromethyl)benzoyl]piperazin-1-yl]-N-(4-methoxyphenyl)pyridazine-3-carboxamide |
| B1O019-01 | 6-[4-[3-[2-(3-Ethoxyphenyl)ethynyl]-5-(trifluoromethyl)benzoyl]piperazin-1-yl]-N-(4-methoxyphenyl)pyridazine-3-carboxamide |
| B1O020-01 | 6-[4-[3-[2-(5-Ethoxypyridin-3-yl)ethynyl]-5-(trifluoromethyl)benzoyl]piperazin-1-yl]-N-(4-methoxyphenyl)pyridazine-3-carboxamide |
| B1O021-01 | 6-[4-[3-[4-(3-Ethoxyphenyl)pyrazol-1-yl]-5-(trifluoromethyl)benzoyl]piperazin-1-yl]-N-(4-methoxyphenyl)pyridazine-3-carboxamide |
| B1O022-01 | 6-[4-[3-[4-(5-Ethoxypyridin-3-yl)pyrazol-1-yl]-5-(trifluoromethyl)benzoyl]piperazin-1-yl]-N-(4-methoxyphenyl)pyridazine-3-carboxamide |
| B1O023-01 | 4-[4-[4-(3-Ethoxyphenyl)thiophene-2-carbonyl]piperazin-1-yl]-N-(4-methoxyphenyl)benzamide |
| B1O024-01 | 4-[4-[4-(5-Ethoxypyridin-3-yl)thiophene-2-carbonyl]piperazin-1-yl]-N-(4-methoxyphenyl)benzamide |
| B1O025-01 | 4-[4-[4-(3-Ethoxyphenyl)-3-methylbenzoyl]piperazin-1-yl]-N-(4-methoxyphenyl)benzamide |
| B1O026-01 | 4-[4-[4-(5-Ethoxypyridin-3-yl)-3-methylbenzoyl]piperazin-1-yl]-N-(4-methoxyphenyl)benzamide |
| B1O027-01 | 4-[4-[4-(3-Ethoxyphenyl)-3-fluorobenzoyl]piperazin-1-yl]-N-(4-methoxyphenyl)benzamide |
| B1O028-01 | 4-[4-[4-(5-Ethoxypyridin-3-yl)-3-fluorobenzoyl]piperazin-1-yl]-N-(4-methoxyphenyl)benzamide |
| B1O029-01 | 4-[4-[2-[2-(3-Ethoxyphenyl)ethynyl]benzoyl]piperazin-1-yl]-N-(4-methoxyphenyl)benzamide |
| B1O030-01 | 4-[4-[2-[2-(5-Ethoxypyridin-3-yl)ethynyl]benzoyl]piperazin-1-yl]-N-(4-methoxyphenyl)benzamide |
| B1O031-01 | 4-[4-[5-[2-(3-Ethoxyphenyl)ethynyl]pyridine-3-carbonyl]piperazin-1-yl]-N-(4-methoxyphenyl)benzamide |
| B1O032-01 | 4-[4-[5-[2-(5-Ethoxypyridin-3-yl)ethynyl]pyridine-3-carbonyl]piperazin-1-yl]-N-(4-methoxyphenyl)benzamide |
| B1O033-01 | 4-[4-[3-[2-(3-Ethoxyphenyl)phenyl]propanoyl]piperazin-1-yl]-N-(4-methoxyphenyl)benzamide |
| B1O034-01 | 4-[4-[3-[2-(5-Ethoxypyridin-3-yl)phenyl]propanoyl]piperazin-1-yl]-N-(4-methoxyphenyl)benzamide |
| B1O035-01 | 4-[4-[4-(3-Ethoxyphenyl)naphthalene-1-carbonyl]piperazin-1-yl]-N-(4-methoxyphenyl)benzamide |
| B1O036-01 | 4-[4-[4-(5-Ethoxypyridin-3-yl)naphthalene-1-carbonyl]piperazin-1-yl]-N-(4-methoxyphenyl)benzamide |
| B1O037-01 | 4-[4-[3-tert-Butyl-5-(3-ethoxyphenyl)benzoyl]piperazin-1-yl]-N-(4-methoxyphenyl)benzamide |
| B1O038-01 | 4-[4-[3-tert-Butyl-5-(5-ethoxypyridin-3-yl)benzoyl]piperazin-1-yl]-N-(4-methoxyphenyl)benzamide |
| B1O039-01 | 4-[4-[3-(3-Ethoxyphenyl)-5-(trifluoromethyl)benzoyl]piperazin-1-yl]-N-(4-methoxyphenyl)benzamide |
| B1O040-01 | 4-[4-[3-(5-Ethoxypyridin-3-yl)-5-(trifluoromethyl)benzoyl]piperazin-1-yl]-N-(4-methoxyphenyl)benzamide |
| B1O041-01 | 4-[4-[3-[2-(3-Ethoxyphenyl)ethynyl]-5-(trifluoromethyl)benzoyl]piperazin-1-yl]-N-(4-methoxyphenyl)benzamide |
| B1O042-01 | 4-[4-[3-[2-(5-Ethoxypyridin-3-yl)ethynyl]-5-(trifluoromethyl)benzoyl]piperazin-1-yl]-N-(4-methoxyphenyl)benzamide |
| B1O043-01 | 4-[4-[3-[4-(3-Ethoxyphenyl)pyrazol-1-yl]-5-(trifluoromethyl)benzoyl]piperazin-1-yl]-N-(4-methoxyphenyl)benzamide |
| B1O044-01 | 4-[4-[3-[4-(5-Ethoxypyridin-3-yl)pyrazol-1-yl]-5-(trifluoromethyl)benzoyl]piperazin-1-yl]-N-(4-methoxyphenyl)benzamide |
| B1O045-01 | 4-[4-[[2-(3-Ethoxyphenyl)phenyl]methyl]piperazin-1-yl]-N-(4-methoxyphenyl)benzamide |

| | |
|---|---|
| B1O046-01 | 4-[4-[[2-(5-Ethoxypyridin-3-yl)phenyl]methyl]piperazin-1-yl]-N-(4-methoxyphenyl)benzamide |
| B1O047-01 | 4-[4-[[4-(3-Ethoxyphenyl)-3-methylphenyl]methyl]piperazin-1-yl]-N-(4-methoxyphenyl)benzamide |
| B1O048-01 | 4-[4-[[4-(5-Ethoxypyridin-3-yl)-3-methylphenyl]methyl]piperazin-1-yl]-N-(4-methoxyphenyl)benzamide |
| B1O049-01 | 4-[4-[[4-(3-Ethoxyphenyl)-2-ethylphenyl]methyl]piperazin-1-yl]-N-(4-methoxyphenyl)benzamide |
| B1O050-01 | 4-[4-[[4-(5-Ethoxypyridin-3-yl)-2-ethylphenyl]methyl]piperazin-1-yl]-N-(4-methoxyphenyl)benzamide |
| B1O051-01 | 4-[4-[[4-(3-Ethoxyphenyl)-3-fluorophenyl]methyl]piperazin-1-yl]-N-(4-methoxyphenyl)benzamide |
| B1O052-01 | 4-[4-[[4-(5-Ethoxypyridin-3-yl)-3-fluorophenyl]methyl]piperazin-1-yl]-N-(4-methoxyphenyl)benzamide |
| B1O053-01 | 4-[4-[[4-(3-Ethoxyphenyl)-2-methoxyphenyl]methyl]piperazin-1-yl]-N-(4-methoxyphenyl)benzamide |
| B1O054-01 | 4-[4-[[4-(5-Ethoxypyridin-3-yl)-2-methoxyphenyl]methyl]piperazin-1-yl]-N-(4-methoxyphenyl)benzamide |
| B1O055-01 | 4-[4-[[3-Ethoxy-5-(3-ethoxyphenyl)phenyl]methyl]piperazin-1-yl]-N-(4-methoxyphenyl)benzamide |
| B1O056-01 | 4-[4-[[3-Ethoxy-5-(5-ethoxypyridin-3-yl)phenyl]methyl]piperazin-1-yl]-N-(4-methoxyphenyl)benzamide |
| B1O057-01 | 4-[4-[[3-(3-Ethoxyphenyl)-5-propan-2-yloxyphenyl]methyl]piperazin-1-yl]-N-(4-methoxyphenyl)benzamide |
| B1O058-01 | 4-[4-[[3-(5-Ethoxypyridin-3-yl)-5-propan-2-yloxyphenyl]methyl]piperazin-1-yl]-N-(4-methoxyphenyl)benzamide |
| B1O059-01 | 4-[4-[[4-(3-Ethoxyphenyl)-2,6-dimethoxyphenyl]methyl]piperazin-1-yl]-N-(4-methoxyphenyl)benzamide |
| B1O060-01 | 4-[4-[[4-(5-Ethoxypyridin-3-yl)-2,6-dimethoxyphenyl]methyl]piperazin-1-yl]-N-(4-methoxyphenyl)benzamide |
| B1O061-01 | 4-[4-[[3-(3-Ethoxyphenyl)-5-(trifluoromethyl)phenyl]methyl]piperazin-1-yl]-N-(4-methoxyphenyl)benzamide |
| B1O062-01 | 4-[4-[[3-(5-Ethoxypyridin-3-yl)-5-(trifluoromethyl)phenyl]methyl]piperazin-1-yl]-N-(4-methoxyphenyl)benzamide |
| B1O063-01 | 4-[4-[[3-(3-Ethoxyphenyl)-5-(trifluoromethoxy)phenyl]methyl]piperazin-1-yl]-N-(4-methoxyphenyl)benzamide |
| B1O064-01 | 4-[4-[[3-(5-Ethoxypyridin-3-yl)-5-(trifluoromethoxy)phenyl]methyl]piperazin-1-yl]-N-(4-methoxyphenyl)benzamide |
| B1O065-01 | 4-[4-[[4-(3-Ethoxyphenyl)naphthalen-1-yl]methyl]piperazin-1-yl]-N-(4-methoxyphenyl)benzamide |
| B1O066-01 | 4-[4-[[4-(5-Ethoxypyridin-3-yl)naphthalen-1-yl]methyl]piperazin-1-yl]-N-(4-methoxyphenyl)benzamide |
| B1O067-01 | 4-[4-[[4-(3-Ethoxyphenyl)thiophen-2-yl]methyl]piperazin-1-yl]-N-(4-methoxyphenyl)benzamide |
| B1O068-01 | 4-[4-[[4-(5-Ethoxypyridin-3-yl)thiophen-2-yl]methyl]piperazin-1-yl]-N-(4-methoxyphenyl)benzamide |
| B1O069-01 | 4-[4-[[2-[2-(3-Ethoxyphenyl)ethynyl]phenyl]methyl]piperazin-1-yl]-N-(4-methoxyphenyl)benzamide |
| B1O070-01 | 4-[4-[[2-[2-(5-Ethoxypyridin-3-yl)ethynyl]phenyl]methyl]piperazin-1-yl]-N-(4-methoxyphenyl)benzamide |
| B1O071-01 | 6-[4-[[2-(3-Ethoxyphenyl)phenyl]methyl]piperazin-1-yl]-N-(4-methoxyphenyl)pyridazine-3-carboxamide |
| B1O072-01 | 6-[4-[[2-(5-Ethoxypyridin-3-yl)phenyl]methyl]piperazin-1-yl]-N-(4-methoxyphenyl)pyridazine-3-carboxamide |
| B1O073-01 | 6-[4-[[4-(3-Ethoxyphenyl)-3-methylphenyl]methyl]piperazin-1-yl]-N-(4-methoxyphenyl)pyridazine-3-carboxamide |

| | |
|---|---|
| B1O074-01 | 6-[4-[[4-(5-Ethoxypyridin-3-yl)-3-methylphenyl]methyl]piperazin-1-yl]-N-(4-methoxyphenyl)pyridazine-3-carboxamide |
| B1O075-01 | 6-[4-[[4-(3-Ethoxyphenyl)-2-ethylphenyl]methyl]piperazin-1-yl]-N-(4-methoxyphenyl)pyridazine-3-carboxamide |
| B1O076-01 | 6-[4-[[4-(5-Ethoxypyridin-3-yl)-2-ethylphenyl]methyl]piperazin-1-yl]-N-(4-methoxyphenyl)pyridazine-3-carboxamide |
| B1O077-01 | 6-[4-[[4-(3-Ethoxyphenyl)-3-fluorophenyl]methyl]piperazin-1-yl]-N-(4-methoxyphenyl)pyridazine-3-carboxamide |
| B1O078-01 | 6-[4-[[4-(5-Ethoxypyridin-3-yl)-3-fluorophenyl]methyl]piperazin-1-yl]-N-(4-methoxyphenyl)pyridazine-3-carboxamide |
| B1O079-01 | 6-[4-[[4-(3-Ethoxyphenyl)-2-methoxyphenyl]methyl]piperazin-1-yl]-N-(4-methoxyphenyl)pyridazine-3-carboxamide |
| B1O080-01 | 6-[4-[[4-(5-Ethoxypyridin-3-yl)-2-methoxyphenyl]methyl]piperazin-1-yl]-N-(4-methoxyphenyl)pyridazine-3-carboxamide |
| B1O081-01 | 6-[4-[[3-Ethoxy-5-(3-ethoxyphenyl)phenyl]methyl]piperazin-1-yl]-N-(4-methoxyphenyl)pyridazine-3-carboxamide |
| B1O082-01 | 6-[4-[[3-Ethoxy-5-(5-ethoxypyridin-3-yl)phenyl]methyl]piperazin-1-yl]-N-(4-methoxyphenyl)pyridazine-3-carboxamide |
| B1O083-01 | 6-[4-[[3-(3-Ethoxyphenyl)-5-propan-2-yloxyphenyl]methyl]piperazin-1-yl]-N-(4-methoxyphenyl)pyridazine-3-carboxamide |
| B1O084-01 | 6-[4-[[3-(5-Ethoxypyridin-3-yl)-5-propan-2-yloxyphenyl]methyl]piperazin-1-yl]-N-(4-methoxyphenyl)pyridazine-3-carboxamide |
| B1O085-01 | 6-[4-[[4-(3-Ethoxyphenyl)-2,6-dimethoxyphenyl]methyl]piperazin-1-yl]-N-(4-methoxyphenyl)pyridazine-3-carboxamide |
| B1O086-01 | 6-[4-[[4-(5-Ethoxypyridin-3-yl)-2,6-dimethoxyphenyl]methyl]piperazin-1-yl]-N-(4-methoxyphenyl)pyridazine-3-carboxamide |
| B1O087-01 | 6-[4-[[3-(3-Ethoxyphenyl)-5-(trifluoromethyl)phenyl]methyl]piperazin-1-yl]-N-(4-methoxyphenyl)pyridazine-3-carboxamide |
| B1O088-01 | 6-[4-[[3-(5-Ethoxypyridin-3-yl)-5-(trifluoromethyl)phenyl]methyl]piperazin-1-yl]-N-(4-methoxyphenyl)pyridazine-3-carboxamide |
| B1O089-01 | 6-[4-[[3-(3-Ethoxyphenyl)-5-(trifluoromethoxy)phenyl]methyl]piperazin-1-yl]-N-(4-methoxyphenyl)pyridazine-3-carboxamide |
| B1O090-01 | 6-[4-[[3-(5-Ethoxypyridin-3-yl)-5-(trifluoromethoxy)phenyl]methyl]piperazin-1-yl]-N-(4-methoxyphenyl)pyridazine-3-carboxamide |
| B1O091-01 | 6-[4-[[4-(3-Ethoxyphenyl)naphthalen-1-yl]methyl]piperazin-1-yl]-N-(4-methoxyphenyl)pyridazine-3-carboxamide |
| B1O092-01 | 6-[4-[[4-(5-Ethoxypyridin-3-yl)naphthalen-1-yl]methyl]piperazin-1-yl]-N-(4-methoxyphenyl)pyridazine-3-carboxamide |
| B1O093-01 | 6-[4-[[4-(3-Ethoxyphenyl)thiophen-2-yl]methyl]piperazin-1-yl]-N-(4-methoxyphenyl)pyridazine-3-carboxamide |
| B1O094-01 | 6-[4-[[4-(5-Ethoxypyridin-3-yl)thiophen-2-yl]methyl]piperazin-1-yl]-N-(4-methoxyphenyl)pyridazine-3-carboxamide |
| B1O095-01 | 6-[4-[[2-[2-(3-Ethoxyphenyl)ethynyl]phenyl]methyl]piperazin-1-yl]-N-(4-methoxyphenyl)pyridazine-3-carboxamide |
| B1O096-01 | 6-[4-[[2-[2-(5-Ethoxypyridin-3-yl)ethynyl]phenyl]methyl]piperazin-1-yl]-N-(4-methoxyphenyl)pyridazine-3-carboxamide |
| B1O097-01 | 4-[4-[1-[4-(3-Ethoxyphenyl)phenyl]ethyl]piperazin-1-yl]-N-(4-methoxyphenyl)benzamide |
| B1O098-01 | 4-[4-[1-[4-(5-Ethoxypyridin-3-yl)phenyl]ethyl]piperazin-1-yl]-N-(4-methoxyphenyl)benzamide |
| B1O099-01 | 4-[4-[1-[4-(3-Ethoxyphenyl)-2-fluorophenyl]ethyl]piperazin-1-yl]-N-(4-methoxyphenyl)benzamide |
| B1O100-01 | 4-[4-[1-[4-(5-Ethoxypyridin-3-yl)-2-fluorophenyl]ethyl]piperazin-1-yl]-N-(4-methoxyphenyl)benzamide |

[Table 249]

[Table AI-03-B1O-01]

| Label | Exact | ☒ | A | a | B | b | C | c | D |
|---|---|---|---|---|---|---|---|---|---|
| B1O001-01 | 543.19 | OEt | A01 | a1 | B10 | b3 | C02 | c1 | D04 |
| B1O002-01 | 544.19 | OEt | A02 | a1 | B10 | b3 | C02 | c1 | D04 |
| B1O003-01 | 551.25 | OEt | A01 | a1 | B04 | b3 | C02 | c1 | D04 |
| B1O004-01 | 552.25 | OEt | A02 | a1 | B04 | b3 | C02 | c1 | D04 |
| B1O005-01 | 555.23 | OEt | A01 | a1 | B06 | b3 | C02 | c1 | D04 |
| B1O006-01 | 556.22 | OEt | A02 | a1 | B06 | b3 | C02 | c1 | D04 |
| B1O007-01 | 561.24 | OEt | A01 | a2 | B01 | b3 | C02 | c1 | D04 |
| B1O008-01 | 562.23 | OEt | A02 | a2 | B01 | b3 | C02 | c1 | D04 |
| B1O009-01 | 562.23 | OEt | A01 | a2 | B17 | b3 | C02 | c1 | D04 |
| B1O010-01 | 563.23 | OEt | A02 | a2 | B17 | b3 | C02 | c1 | D04 |
| B1O011-01 | 565.27 | OEt | A01 | a1 | B02 | b3 | C02 | c1 | D04 |
| B1O012-01 | 566.26 | OEt | A02 | a1 | B02 | b3 | C02 | c1 | D04 |
| B1O013-01 | 587.25 | OEt | A01 | a1 | B09 | b3 | C02 | c1 | D04 |
| B1O014-01 | 588.25 | OEt | A02 | a1 | B09 | b3 | C02 | c1 | D04 |
| B1O015-01 | 593.3 | OEt | A01 | a1 | B16 | b3 | C02 | c1 | D04 |
| B1O016-01 | 594.3 | OEt | A02 | a1 | B16 | b3 | C02 | c1 | D04 |
| B1O017-01 | 605.23 | OEt | A01 | a1 | B11 | b3 | C02 | c1 | D04 |
| B1O018-01 | 606.22 | OEt | A02 | a1 | B11 | b3 | C02 | c1 | D04 |
| B1O019-01 | 629.23 | OEt | A01 | a2 | B11 | b3 | C02 | c1 | D04 |
| B1O020-01 | 630.22 | OEt | A02 | a2 | B11 | b3 | C02 | c1 | D04 |
| B1O021-01 | 671.25 | OEt | A01 | a1 | B12 | b3 | C02 | c1 | D04 |
| B1O022-01 | 672.24 | OEt | A02 | a1 | B12 | b3 | C02 | c1 | D04 |
| B1O023-01 | 541.2 | OEt | A01 | a1 | B10 | b3 | C01 | c1 | D04 |
| B1O024-01 | 542.2 | OEt | A02 | a1 | B10 | b3 | C01 | c1 | D04 |
| B1O025-01 | 549.26 | OEt | A01 | a1 | B04 | b3 | C01 | c1 | D04 |
| B1O026-01 | 550.26 | OEt | A02 | a1 | B04 | b3 | C01 | c1 | D04 |
| B1O027-01 | 553.24 | OEt | A01 | a1 | B06 | b3 | C01 | c1 | D04 |
| B1O028-01 | 554.23 | OEt | A02 | a1 | B06 | b3 | C01 | c1 | D04 |
| B1O029-01 | 559.25 | OEt | A01 | a2 | B01 | b3 | C01 | c1 | D04 |
| B1O030-01 | 560.24 | OEt | A02 | a2 | B01 | b3 | C01 | c1 | D04 |
| B1O031-01 | 560.24 | OEt | A01 | a2 | B17 | b3 | C01 | c1 | D04 |
| B1O032-01 | 561.24 | OEt | A02 | a2 | B17 | b3 | C01 | c1 | D04 |
| B1O033-01 | 563.28 | OEt | A01 | a1 | B02 | b3 | C01 | c1 | D04 |
| B1O034-01 | 564.27 | OEt | A02 | a1 | B02 | b3 | C01 | c1 | D04 |
| B1O035-01 | 585.26 | OEt | A01 | a1 | B09 | b3 | C01 | c1 | D04 |
| B1O036-01 | 586.26 | OEt | A02 | a1 | B09 | b3 | C01 | c1 | D04 |
| B1O037-01 | 591.31 | OEt | A01 | a1 | B16 | b3 | C01 | c1 | D04 |
| B1O038-01 | 592.31 | OEt | A02 | a1 | B16 | b3 | C01 | c1 | D04 |
| B1O039-01 | 603.23 | OEt | A01 | a1 | B11 | b3 | C01 | c1 | D04 |
| B1O040-01 | 604.23 | OEt | A02 | a1 | B11 | b3 | C01 | c1 | D04 |
| B1O041-01 | 627.23 | OEt | A01 | a2 | B11 | b3 | C01 | c1 | D04 |
| B1O042-01 | 628.23 | OEt | A02 | a2 | B11 | b3 | C01 | c1 | D04 |
| B1O043-01 | 669.26 | OEt | A01 | a1 | B12 | b3 | C01 | c1 | D04 |
| B1O044-01 | 670.25 | OEt | A02 | a1 | B12 | b3 | C01 | c1 | D04 |

| | | | | | | | | | |
|---|---|---|---|---|---|---|---|---|---|
| B1O045-01 | 521.27 | OEt | A01 | a1 | B01 | b1 | C01 | c1 | D04 |
| B1O046-01 | 522.26 | OEt | A02 | a1 | B01 | b1 | C01 | c1 | D04 |
| B1O047-01 | 535.28 | OEt | A01 | a1 | B04 | b1 | C01 | c1 | D04 |
| B1O048-01 | 536.28 | OEt | A02 | a1 | B04 | b1 | C01 | c1 | D04 |
| B1O049-01 | 549.3 | OEt | A01 | a1 | B05 | b1 | C01 | c1 | D04 |
| B1O050-01 | 550.29 | OEt | A02 | a1 | B05 | b1 | C01 | c1 | D04 |
| B1O051-01 | 539.26 | OEt | A01 | a1 | B06 | b1 | C01 | c1 | D04 |
| B1O052-01 | 540.25 | OEt | A02 | a1 | B06 | b1 | C01 | c1 | D04 |
| B1O053-01 | 551.28 | OEt | A01 | a1 | B07 | b1 | C01 | c1 | D04 |
| B1O054-01 | 552.27 | OEt | A02 | a1 | B07 | b1 | C01 | c1 | D04 |
| B1O055-01 | 565.29 | OEt | A01 | a1 | B14 | b1 | C01 | c1 | D04 |
| B1O056-01 | 566.29 | OEt | A02 | a1 | B14 | b1 | C01 | c1 | D04 |
| B1O057-01 | 579.31 | OEt | A01 | a1 | B15 | b1 | C01 | c1 | D04 |
| B1O058-01 | 580.31 | OEt | A02 | a1 | B15 | b1 | C01 | c1 | D04 |
| B1O059-01 | 581.29 | OEt | A01 | a1 | B08 | b1 | C01 | c1 | D04 |
| B1O060-01 | 582.28 | OEt | A02 | a1 | B08 | b1 | C01 | c1 | D04 |
| B1O061-01 | 589.26 | OEt | A01 | a1 | B11 | b1 | C01 | c1 | D04 |
| B1O062-01 | 590.25 | OEt | A02 | a1 | B11 | b1 | C01 | c1 | D04 |
| B1O063-01 | 605.25 | OEt | A01 | a1 | B13 | b1 | C01 | c1 | D04 |
| B1O064-01 | 606.25 | OEt | A02 | a1 | B13 | b1 | C01 | c1 | D04 |
| B1O065-01 | 571.28 | OEt | A01 | a1 | B09 | b1 | C01 | c1 | D04 |
| B1O066-01 | 572.28 | OEt | A02 | a1 | B09 | b1 | C01 | c1 | D04 |
| B1O067-01 | 527.22 | OEt | A01 | a1 | B10 | b1 | C01 | c1 | D04 |
| B1O068-01 | 528.22 | OEt | A02 | a1 | B10 | b1 | C01 | c1 | D04 |
| B1O069-01 | 545.27 | OEt | A01 | a2 | B01 | b1 | C01 | c1 | D04 |
| B1O070-01 | 546.26 | OEt | A02 | a2 | B01 | b1 | C01 | c1 | D04 |
| B1O071-01 | 523.26 | OEt | A01 | a1 | B01 | b1 | C02 | c1 | D04 |
| B1O072-01 | 524.25 | OEt | A02 | a1 | B01 | b1 | C02 | c1 | D04 |
| B1O073-01 | 537.27 | OEt | A01 | a1 | B04 | b1 | C02 | c1 | D04 |
| B1O074-01 | 538.27 | OEt | A02 | a1 | B04 | b1 | C02 | c1 | D04 |
| B1O075-01 | 551.29 | OEt | A01 | a1 | B05 | b1 | C02 | c1 | D04 |
| B1O076-01 | 552.29 | OEt | A02 | a1 | B05 | b1 | C02 | c1 | D04 |
| B1O077-01 | 541.25 | OEt | A01 | a1 | B06 | b1 | C02 | c1 | D04 |
| B1O078-01 | 542.24 | OEt | A02 | a1 | B06 | b1 | C02 | c1 | D04 |
| B1O079-01 | 553.27 | OEt | A01 | a1 | B07 | b1 | C02 | c1 | D04 |
| B1O080-01 | 554.26 | OEt | A02 | a1 | B07 | b1 | C02 | c1 | D04 |
| B1O081-01 | 567.29 | OEt | A01 | a1 | B14 | b1 | C02 | c1 | D04 |
| B1O082-01 | 568.28 | OEt | A02 | a1 | B14 | b1 | C02 | c1 | D04 |
| B1O083-01 | 581.3 | OEt | A01 | a1 | B15 | b1 | C02 | c1 | D04 |
| B1O084-01 | 582.3 | OEt | A02 | a1 | B15 | b1 | C02 | c1 | D04 |
| B1O085-01 | 583.28 | OEt | A01 | a1 | B08 | b1 | C02 | c1 | D04 |
| B1O086-01 | 584.28 | OEt | A02 | a1 | B08 | b1 | C02 | c1 | D04 |
| B1O087-01 | 591.25 | OEt | A01 | a1 | B11 | b1 | C02 | c1 | D04 |
| B1O088-01 | 592.24 | OEt | A02 | a1 | B11 | b1 | C02 | c1 | D04 |
| B1O089-01 | 607.24 | OEt | A01 | a1 | B13 | b1 | C02 | c1 | D04 |
| B1O090-01 | 608.24 | OEt | A02 | a1 | B13 | b1 | C02 | c1 | D04 |
| B1O091-01 | 573.27 | OEt | A01 | a1 | B09 | b1 | C02 | c1 | D04 |

| | | | | | | | | | |
|---|---|---|---|---|---|---|---|---|---|
| B1O092-01 | 574.27 | OEt | A02 | a1 | B09 | b1 | C02 | c1 | D04 |
| B1O093-01 | 529.22 | OEt | A01 | a1 | B10 | b1 | C02 | c1 | D04 |
| B1O094-01 | 530.21 | OEt | A02 | a1 | B10 | b1 | C02 | c1 | D04 |
| B1O095-01 | 547.26 | OEt | A01 | a2 | B01 | b1 | C02 | c1 | D04 |
| B1O096-01 | 548.25 | OEt | A02 | a2 | B01 | b1 | C02 | c1 | D04 |
| B1O097-01 | 535.28 | OEt | A01 | a1 | B03 | b2 | C01 | c1 | D04 |
| B1O098-01 | 536.28 | OEt | A02 | a1 | B03 | b2 | C01 | c1 | D04 |
| B1O099-01 | 553.27 | OEt | A01 | a1 | B18 | b2 | C01 | c1 | D04 |
| B1O100-01 | 554.27 | OEt | A02 | a1 | B18 | b2 | C01 | c1 | D04 |

[Table 250]

[Table AI-03-B1A-01]

| Mixture No. | mixAI-B1A-01 |
|---|---|
| No. of compound that may be contained | Compound name |
| B1A001-01 | N-(1-Adamantylmethyl)-6-[4-[4-(3-ethoxyphenyl)thiophene-2-carbonyl]piperazin-1-yl]pyridazine-3-carboxamide |
| B1A002-01 | N-(1-Adamantylmethyl)-6-[4-[4-(5-ethoxypyridin-3-yl)thiophene-2-carbonyl]piperazin-1-yl]pyridazine-3-carboxamide |
| B1A003-01 | N-(1-Adamantylmethyl)-6-[4-[4-(3-ethoxyphenyl)-3-methylbenzoyl]piperazin-1-yl]pyridazine-3-carboxamide |
| B1A004-01 | N-(1-Adamantylmethyl)-6-[4-[4-(5-ethoxypyridin-3-yl)-3-methylbenzoyl]piperazin-1-yl]pyridazine-3-carboxamide |
| B1A005-01 | N-(1-Adamantylmethyl)-6-[4-[4-(3-ethoxyphenyl)-3-fluorobenzoyl]piperazin-1-yl]pyridazine-3-carboxamide |
| B1A006-01 | N-(1-Adamantylmethyl)-6-[4-[4-(5-ethoxypyridin-3-yl)-3-fluorobenzoyl]piperazin-1-yl]pyridazine-3-carboxamide |
| B1A007-01 | N-(1-Adamantylmethyl)-6-[4-[2-[2-(3-ethoxyphenyl)ethynyl]benzoyl]piperazin-1-yl]pyridazine-3-carboxamide |
| B1A008-01 | N-(1-Adamantylmethyl)-6-[4-[2-[2-(5-ethoxypyridin-3-yl)ethynyl]benzoyl]piperazin-1-yl]pyridazine-3-carboxamide |
| B1A009-01 | N-(1-Adamantylmethyl)-6-[4-[5-[2-(3-ethoxyphenyl)ethynyl]pyridine-3-carbonyl]piperazin-1-yl]pyridazine-3-carboxamide |
| B1A010-01 | N-(1-Adamantylmethyl)-6-[4-[5-[2-(5-ethoxypyridin-3-yl)ethynyl]pyridine-3-carbonyl]piperazin-1-yl]pyridazine-3-carboxamide |
| B1A011-01 | N-(1-Adamantylmethyl)-6-[4-[3-[2-(3-ethoxyphenyl)phenyl]propanoyl]piperazin-1-yl]pyridazine-3-carboxamide |
| B1A012-01 | N-(1-Adamantylmethyl)-6-[4-[3-[2-(5-ethoxypyridin-3-yl)phenyl]propanoyl]piperazin-1-yl]pyridazine-3-carboxamide |
| B1A013-01 | N-(1-Adamantylmethyl)-6-[4-[4-(3-ethoxyphenyl)naphthalene-1-carbonyl]piperazin-1-yl]pyridazine-3-carboxamide |
| B1A014-01 | N-(1-Adamantylmethyl)-6-[4-[4-(5-ethoxypyridin-3-yl)naphthalene-1-carbonyl]piperazin-1-yl]pyridazine-3-carboxamide |
| B1A015-01 | N-(1-Adamantylmethyl)-6-[4-[3-tert-butyl-5-(3-ethoxyphenyl)benzoyl]piperazin-1-yl]pyridazine-3-carboxamide |
| B1A016-01 | N-(1-Adamantylmethyl)-6-[4-[3-tert-butyl-5-(5-ethoxypyridin-3-yl)benzoyl]piperazin-1-yl]pyridazine-3-carboxamide |
| B1A017-01 | N-(1-Adamantylmethyl)-6-[4-[3-(3-ethoxyphenyl)-5-(trifluoromethyl)benzoyl]piperazin-1-yl]pyridazine-3-carboxamide |
| B1A018-01 | N-(1-Adamantylmethyl)-6-[4-[3-(5-ethoxypyridin-3-yl)-5-(trifluoromethyl)benzoyl]piperazin-1-yl]pyridazine-3-carboxamide |

| | |
|---|---|
| B1A019-01 | N-(1-Adamantylmethyl)-6-[4-[3-[2-(3-ethoxyphenyl)ethynyl]-5-(trifluoromethyl)benzoyl]piperazin-1-yl]pyridazine-3-carboxamide |
| B1A020-01 | N-(1-Adamantylmethyl)-6-[4-[3-[2-(5-ethoxypyridin-3-yl)ethynyl]-5-(trifluoromethyl)benzoyl]piperazin-1-yl]pyridazine-3-carboxamide |
| B1A021-01 | N-(1-Adamantylmethyl)-6-[4-[3-[4-(3-ethoxyphenyl)pyrazol-1-yl]-5-(trifluoromethyl)benzoyl]piperazin-1-yl]pyridazine-3-carboxamide |
| B1A022-01 | N-(1-Adamantylmethyl)-6-[4-[3-[4-(5-ethoxypyridin-3-yl)pyrazol-1-yl]-5-(trifluoromethyl)benzoyl]piperazin-1-yl]pyridazine-3-carboxamide |
| B1A023-01 | N-(1-Adamantylmethyl)-4-[4-[4-(3-ethoxyphenyl)thiophene-2-carbonyl]piperazin-1-yl]benzamide |
| B1A024-01 | N-(1-Adamantylmethyl)-4-[4-[4-(5-ethoxypyridin-3-yl)thiophene-2-carbonyl]piperazin-1-yl]benzamide |
| B1A025-01 | N-(1-Adamantylmethyl)-4-[4-[4-(3-ethoxyphenyl)-3-methylbenzoyl]piperazin-1-yl]benzamide |
| B1A026-01 | N-(1-Adamantylmethyl)-4-[4-[4-(5-ethoxypyridin-3-yl)-3-methylbenzoyl]piperazin-1-yl]benzamide |
| B1A027-01 | N-(1-Adamantylmethyl)-4-[4-[4-(3-ethoxyphenyl)-3-fluorobenzoyl]piperazin-1-yl]benzamide |
| B1A028-01 | N-(1-Adamantylmethyl)-4-[4-[4-(5-ethoxypyridin-3-yl)-3-fluorobenzoyl]piperazin-1-yl]benzamide |
| B1A029-01 | N-(1-Adamantylmethyl)-4-[4-[2-[2-(3-ethoxyphenyl)ethynyl]benzoyl]piperazin-1-yl]benzamide |
| B1A030-01 | N-(1-Adamantylmethyl)-4-[4-[2-[2-(5-ethoxypyridin-3-yl)ethynyl]benzoyl]piperazin-1-yl]benzamide |
| B1A031-01 | N-(1-Adamantylmethyl)-4-[4-[5-[2-(3-ethoxyphenyl)ethynyl]pyridine-3-carbonyl]piperazin-1-yl]benzamide |
| B1A032-01 | N-(1-Adamantylmethyl)-4-[4-[5-[2-(5-ethoxypyridin-3-yl)ethynyl]pyridine-3-carbonyl]piperazin-1-yl]benzamide |
| B1A033-01 | N-(1-Adamantylmethyl)-4-[4-[3-[2-(3-ethoxyphenyl)phenyl]propanoyl]piperazin-1-yl]benzamide |
| B1A034-01 | N-(1-Adamantylmethyl)-4-[4-[3-[2-(5-ethoxypyridin-3-yl)phenyl]propanoyl]piperazin-1-yl]benzamide |
| B1A035-01 | N-(1-Adamantylmethyl)-4-[4-[4-(3-ethoxyphenyl)naphthalene-1-carbonyl]piperazin-1-yl]benzamide |
| B1A036-01 | N-(1-Adamantylmethyl)-4-[4-[4-(5-ethoxypyridin-3-yl)naphthalene-1-carbonyl]piperazin-1-yl]benzamide |
| B1A037-01 | N-(1-Adamantylmethyl)-4-[4-[3-tert-butyl-5-(3-ethoxyphenyl)benzoyl]piperazin-1-yl]benzamide |
| B1A038-01 | N-(1-Adamantylmethyl)-4-[4-[3-tert-butyl-5-(5-ethoxypyridin-3-yl)benzoyl]piperazin-1-yl]benzamide |
| B1A039-01 | N-(1-Adamantylmethyl)-4-[4-[3-(3-ethoxyphenyl)-5-(trifluoromethyl)benzoyl]piperazin-1-yl]benzamide |
| B1A040-01 | N-(1-Adamantylmethyl)-4-[4-[3-(5-ethoxypyridin-3-yl)-5-(trifluoromethyl)benzoyl]piperazin-1-yl]benzamide |
| B1A041-01 | N-(1-Adamantylmethyl)-4-[4-[3-[2-(3-ethoxyphenyl)ethynyl]-5-(trifluoromethyl)benzoyl]piperazin-1-yl]benzamide |
| B1A042-01 | N-(1-Adamantylmethyl)-4-[4-[3-[2-(5-ethoxypyridin-3-yl)ethynyl]-5-(trifluoromethyl)benzoyl]piperazin-1-yl]benzamide |
| B1A043-01 | N-(1-Adamantylmethyl)-4-[4-[3-[4-(3-ethoxyphenyl)pyrazol-1-yl]-5-(trifluoromethyl)benzoyl]piperazin-1-yl]benzamide |
| B1A044-01 | N-(1-Adamantylmethyl)-4-[4-[3-[4-(5-ethoxypyridin-3-yl)pyrazol-1-yl]-5-(trifluoromethyl)benzoyl]piperazin-1-yl]benzamide |
| B1A045-01 | N-(1-Adamantylmethyl)-4-[4-[[2-(3-ethoxyphenyl)phenyl]methyl]piperazin-1-yl]benzamide |
| B1A046-01 | N-(1-Adamantylmethyl)-4-[4-[[2-(5-ethoxypyridin-3-yl)phenyl]methyl]piperazin-1-yl]benzamide |

| | |
|---|---|
| B1A047-01 | N-(1-Adamantylmethyl)-4-[4-[[4-(3-ethoxyphenyl)-3-methylphenyl]methyl]piperazin-1-yl]benzamide |
| B1A048-01 | N-(1-Adamantylmethyl)-4-[4-[[4-(5-ethoxypyridin-3-yl)-3-methylphenyl]methyl]piperazin-1-yl]benzamide |
| B1A049-01 | N-(1-Adamantylmethyl)-4-[4-[[4-(3-ethoxyphenyl)-2-ethylphenyl]methyl]piperazin-1-yl]benzamide |
| B1A050-01 | N-(1-Adamantylmethyl)-4-[4-[[4-(5-ethoxypyridin-3-yl)-2-ethylphenyl]methyl]piperazin-1-yl]benzamide |
| B1A051-01 | N-(1-Adamantylmethyl)-4-[4-[[4-(3-ethoxyphenyl)-3-fluorophenyl]methyl]piperazin-1-yl]benzamide |
| B1A052-01 | N-(1-Adamantylmethyl)-4-[4-[[4-(5-ethoxypyridin-3-yl)-3-fluorophenyl]methyl]piperazin-1-yl]benzamide |
| B1A053-01 | N-(1-Adamantylmethyl)-4-[4-[[4-(3-ethoxyphenyl)-2-methoxyphenyl]methyl]piperazin-1-yl]benzamide |
| B1A054-01 | N-(1-Adamantylmethyl)-4-[4-[[4-(5-ethoxypyridin-3-yl)-2-methoxyphenyl]methyl]piperazin-1-yl]benzamide |
| B1A055-01 | N-(1-Adamantylmethyl)-4-[4-[[3-ethoxy-5-(3-ethoxyphenyl)phenyl]methyl]piperazin-1-yl]benzamide |
| B1A056-01 | N-(1-Adamantylmethyl)-4-[4-[[3-ethoxy-5-(5-ethoxypyridin-3-yl)phenyl]methyl]piperazin-1-yl]benzamide |
| B1A057-01 | N-(1-Adamantylmethyl)-4-[4-[[3-(3-ethoxyphenyl)-5-propan-2-yloxyphenyl]methyl]piperazin-1-yl]benzamide |
| B1A058-01 | N-(1-Adamantylmethyl)-4-[4-[[3-(5-ethoxypyridin-3-yl)-5-propan-2-yloxyphenyl]methyl]piperazin-1-yl]benzamide |
| B1A059-01 | N-(1-Adamantylmethyl)-4-[4-[[4-(3-ethoxyphenyl)-2,6-dimethoxyphenyl]methyl]piperazin-1-yl]benzamide |
| B1A060-01 | N-(1-Adamantylmethyl)-4-[4-[[4-(5-ethoxypyridin-3-yl)-2,6-dimethoxyphenyl]methyl]piperazin-1-yl]benzamide |
| B1A061-01 | N-(1-Adamantylmethyl)-4-[4-[[3-(3-ethoxyphenyl)-5-(trifluoromethyl)phenyl]methyl]piperazin-1-yl]benzamide |
| B1A062-01 | N-(1-Adamantylmethyl)-4-[4-[[3-(5-ethoxypyridin-3-yl)-5-(trifluoromethyl)phenyl]methyl]piperazin-1-yl]benzamide |
| B1A063-01 | N-(1-Adamantylmethyl)-4-[4-[[3-(3-ethoxyphenyl)-5-(trifluoromethoxy)phenyl]methyl]piperazin-1-yl]benzamide |
| B1A064-01 | N-(1-Adamantylmethyl)-4-[4-[[3-(5-ethoxypyridin-3-yl)-5-(trifluoromethoxy)phenyl]methyl]piperazin-1-yl]benzamide |
| B1A065-01 | N-(1-Adamantylmethyl)-4-[4-[[4-(3-ethoxyphenyl)naphthalen-1-yl]methyl]piperazin-1-yl]benzamide |
| B1A066-01 | N-(1-Adamantylmethyl)-4-[4-[[4-(5-ethoxypyridin-3-yl)naphthalen-1-yl]methyl]piperazin-1-yl]benzamide |
| B1A067-01 | N-(1-Adamantylmethyl)-4-[4-[[4-(3-ethoxyphenyl)thiophen-2-yl]methyl]piperazin-1-yl]benzamide |
| B1A068-01 | N-(1-Adamantylmethyl)-4-[4-[[4-(5-ethoxypyridin-3-yl)thiophen-2-yl]methyl]piperazin-1-yl]benzamide |
| B1A069-01 | N-(1-Adamantylmethyl)-4-[4-[[2-[2-(3-ethoxyphenyl)ethynyl]phenyl]methyl]piperazin-1-yl]benzamide |
| B1A070-01 | N-(1-Adamantylmethyl)-4-[4-[[2-[2-(5-ethoxypyridin-3-yl)ethynyl]phenyl]methyl]piperazin-1-yl]benzamide |
| B1A071-01 | N-(1-Adamantylmethyl)-6-[4-[[2-(3-ethoxyphenyl)phenyl]methyl]piperazin-1-yl]pyridazine-3-carboxamide |
| B1A072-01 | N-(1-Adamantylmethyl)-6-[4-[[2-(5-ethoxypyridin-3-yl)phenyl]methyl]piperazin-1-yl]pyridazine-3-carboxamide |
| B1A073-01 | N-(1-Adamantylmethyl)-6-[4-[[4-(3-ethoxyphenyl)-3-methylphenyl]methyl]piperazin-1-yl]pyridazine-3-carboxamide |
| B1A074-01 | N-(1-Adamantylmethyl)-6-[4-[[4-(5-ethoxypyridin-3-yl)-3-methylphenyl]methyl]piperazin-1-yl]pyridazine-3-carboxamide |

| | |
|---|---|
| B1A075-01 | N-(1-Adamantylmethyl)-6-[4-[[4-(3-ethoxyphenyl)-2-ethylphenyl]methyl]piperazin-1-yl]pyridazine-3-carboxamide |
| B1A076-01 | N-(1-Adamantylmethyl)-6-[4-[[4-(5-ethoxypyridin-3-yl)-2-ethylphenyl]methyl]piperazin-1-yl]pyridazine-3-carboxamide |
| B1A077-01 | N-(1-Adamantylmethyl)-6-[4-[[4-(3-ethoxyphenyl)-3-fluorophenyl]methyl]piperazin-1-yl]pyridazine-3-carboxamide |
| B1A078-01 | N-(1-Adamantylmethyl)-6-[4-[[4-(5-ethoxypyridin-3-yl)-3-fluorophenyl]methyl]piperazin-1-yl]pyridazine-3-carboxamide |
| B1A079-01 | N-(1-Adamantylmethyl)-6-[4-[[4-(3-ethoxyphenyl)-2-methoxyphenyl]methyl]piperazin-1-yl]pyridazine-3-carboxamide |
| B1A080-01 | N-(1-Adamantylmethyl)-6-[4-[[4-(5-ethoxypyridin-3-yl)-2-methoxyphenyl]methyl]piperazin-1-yl]pyridazine-3-carboxamide |
| B1A081-01 | N-(1-Adamantylmethyl)-6-[4-[[3-ethoxy-5-(3-ethoxyphenyl)phenyl]methyl]piperazin-1-yl]pyridazine-3-carboxamide |
| B1A082-01 | N-(1-Adamantylmethyl)-6-[4-[[3-ethoxy-5-(5-ethoxypyridin-3-yl)phenyl]methyl]piperazin-1-yl]pyridazine-3-carboxamide |
| B1A083-01 | N-(1-Adamantylmethyl)-6-[4-[[3-(3-ethoxyphenyl)-5-propan-2-yloxyphenyl]methyl]piperazin-1-yl]pyridazine-3-carboxamide |
| B1A084-01 | N-(1-Adamantylmethyl)-6-[4-[[3-(5-ethoxypyridin-3-yl)-5-propan-2-yloxyphenyl]methyl]piperazin-1-yl]pyridazine-3-carboxamide |
| B1A085-01 | N-(1-Adamantylmethyl)-6-[4-[[4-(3-ethoxyphenyl)-2,6-dimethoxyphenyl]methyl]piperazin-1-yl]pyridazine-3-carboxamide |
| B1A086-01 | N-(1-Adamantylmethyl)-6-[4-[[4-(5-ethoxypyridin-3-yl)-2,6-dimethoxyphenyl]methyl]piperazin-1-yl]pyridazine-3-carboxamide |
| B1A087-01 | N-(1-Adamantylmethyl)-6-[4-[[3-(3-ethoxyphenyl)-5-(trifluoromethyl)phenyl]methyl]piperazin-1-yl]pyridazine-3-carboxamide |
| B1A088-01 | N-(1-Adamantylmethyl)-6-[4-[[3-(5-ethoxypyridin-3-yl)-5-(trifluoromethyl)phenyl]methyl]piperazin-1-yl]pyridazine-3-carboxamide |
| B1A089-01 | N-(1-Adamantylmethyl)-6-[4-[[3-(3-ethoxyphenyl)-5-(trifluoromethoxy)phenyl]methyl]piperazin-1-yl]pyridazine-3-carboxamide |
| B1A090-01 | N-(1-Adamantylmethyl)-6-[4-[[3-(5-ethoxypyridin-3-yl)-5-(trifluoromethoxy)phenyl]methyl]piperazin-1-yl]pyridazine-3-carboxamide |
| B1A091-01 | N-(1-Adamantylmethyl)-6-[4-[[4-(3-ethoxyphenyl)naphthalen-1-yl]methyl]piperazin-1-yl]pyridazine-3-carboxamide |
| B1A092-01 | N-(1-Adamantylmethyl)-6-[4-[[4-(5-ethoxypyridin-3-yl)naphthalen-1-yl]methyl]piperazin-1-yl]pyridazine-3-carboxamide |
| B1A093-01 | N-(1-Adamantylmethyl)-6-[4-[[4-(3-ethoxyphenyl)thiophen-2-yl]methyl]piperazin-1-yl]pyridazine-3-carboxamide |
| B1A094-01 | N-(1-Adamantylmethyl)-6-[4-[[4-(5-ethoxypyridin-3-yl)thiophen-2-yl]methyl]piperazin-1-yl]pyridazine-3-carboxamide |
| B1A095-01 | N-(1-Adamantylmethyl)-6-[4-[[2-[2-(3-ethoxyphenyl)ethynyl]phenyl]methyl]piperazin-1-yl]pyridazine-3-carboxamide |
| B1A096-01 | N-(1-Adamantylmethyl)-6-[4-[[2-[2-(5-ethoxypyridin-3-yl)ethynyl]phenyl]methyl]piperazin-1-yl]pyridazine-3-carboxamide |
| B1A097-01 | N-(1-Adamantylmethyl)-4-[4-[1-[4-(3-ethoxyphenyl)phenyl]ethyl]piperazin-1-yl]benzamide |
| B1A098-01 | N-(1-Adamantylmethyl)-4-[4-[1-[4-(5-ethoxypyridin-3-yl)phenyl]ethyl]piperazin-1-yl]benzamide |
| B1A099-01 | N-(1-Adamantylmethyl)-4-[4-[1-[4-(3-ethoxyphenyl)-2-fluorophenyl]ethyl]piperazin-1-yl]benzamide |
| B1A100-01 | N-(1-Adamantylmethyl)-4-[4-[1-[4-(5-ethoxypyridin-3-yl)-2-fluorophenyl]ethyl]piperazin-1-yl]benzamide |

[Table 251]

[Table AI-03-B1A-01]

| Label | Exact | ☒ | A | a | B | b | C | c | D |
|---|---|---|---|---|---|---|---|---|---|
| B1A001-01 | 585.28 | OEt | A01 | a1 | B10 | b3 | C02 | c1 | D05 |
| B1A002-01 | 586.27 | OEt | A02 | a1 | B10 | b3 | C02 | c1 | D05 |
| B1A003-01 | 593.34 | OEt | A01 | a1 | B04 | b3 | C02 | c1 | D05 |
| B1A004-01 | 594.33 | OEt | A02 | a1 | B04 | b3 | C02 | c1 | D05 |
| B1A005-01 | 597.31 | OEt | A01 | a1 | B06 | b3 | C02 | c1 | D05 |
| B1A006-01 | 598.31 | OEt | A02 | a1 | B06 | b3 | C02 | c1 | D05 |
| B1A007-01 | 603.32 | OEt | A01 | a2 | B01 | b3 | C02 | c1 | D05 |
| B1A008-01 | 604.32 | OEt | A02 | a2 | B01 | b3 | C02 | c1 | D05 |
| B1A009-01 | 604.32 | OEt | A01 | a2 | B17 | b3 | C02 | c1 | D05 |
| B1A010-01 | 605.31 | OEt | A02 | a2 | B17 | b3 | C02 | c1 | D05 |
| B1A011-01 | 607.35 | OEt | A01 | a1 | B02 | b3 | C02 | c1 | D05 |
| B1A012-01 | 608.35 | OEt | A02 | a1 | B02 | b3 | C02 | c1 | D05 |
| B1A013-01 | 629.34 | OEt | A01 | a1 | B09 | b3 | C02 | c1 | D05 |
| B1A014-01 | 630.33 | OEt | A02 | a1 | B09 | b3 | C02 | c1 | D05 |
| B1A015-01 | 635.38 | OEt | A01 | a1 | B16 | b3 | C02 | c1 | D05 |
| B1A016-01 | 636.38 | OEt | A02 | a1 | B16 | b3 | C02 | c1 | D05 |
| B1A017-01 | 647.31 | OEt | A01 | a1 | B11 | b3 | C02 | c1 | D05 |
| B1A018-01 | 648.3 | OEt | A02 | a1 | B11 | b3 | C02 | c1 | D05 |
| B1A019-01 | 671.31 | OEt | A01 | a2 | B11 | b3 | C02 | c1 | D05 |
| B1A020-01 | 672.3 | OEt | A02 | a2 | B11 | b3 | C02 | c1 | D05 |
| B1A021-01 | 713.33 | OEt | A01 | a1 | B12 | b3 | C02 | c1 | D05 |
| B1A022-01 | 714.33 | OEt | A02 | a1 | B12 | b3 | C02 | c1 | D05 |
| B1A023-01 | 583.29 | OEt | A01 | a1 | B10 | b3 | C01 | c1 | D05 |
| B1A024-01 | 584.28 | OEt | A02 | a1 | B10 | b3 | C01 | c1 | D05 |
| B1A025-01 | 591.35 | OEt | A01 | a1 | B04 | b3 | C01 | c1 | D05 |
| B1A026-01 | 592.34 | OEt | A02 | a1 | B04 | b3 | C01 | c1 | D05 |
| B1A027-01 | 595.32 | OEt | A01 | a1 | B06 | b3 | C01 | c1 | D05 |
| B1A028-01 | 596.32 | OEt | A02 | a1 | B06 | b3 | C01 | c1 | D05 |
| B1A029-01 | 601.33 | OEt | A01 | a2 | B01 | b3 | C01 | c1 | D05 |
| B1A030-01 | 602.33 | OEt | A02 | a2 | B01 | b3 | C01 | c1 | D05 |
| B1A031-01 | 602.33 | OEt | A01 | a2 | B17 | b3 | C01 | c1 | D05 |
| B1A032-01 | 603.32 | OEt | A02 | a2 | B17 | b3 | C01 | c1 | D05 |
| B1A033-01 | 605.36 | OEt | A01 | a1 | B02 | b3 | C01 | c1 | D05 |
| B1A034-01 | 606.36 | OEt | A02 | a1 | B02 | b3 | C01 | c1 | D05 |
| B1A035-01 | 627.35 | OEt | A01 | a1 | B09 | b3 | C01 | c1 | D05 |
| B1A036-01 | 628.34 | OEt | A02 | a1 | B09 | b3 | C01 | c1 | D05 |
| B1A037-01 | 633.39 | OEt | A01 | a1 | B16 | b3 | C01 | c1 | D05 |
| B1A038-01 | 634.39 | OEt | A02 | a1 | B16 | b3 | C01 | c1 | D05 |
| B1A039-01 | 645.32 | OEt | A01 | a1 | B11 | b3 | C01 | c1 | D05 |
| B1A040-01 | 646.31 | OEt | A02 | a1 | B11 | b3 | C01 | c1 | D05 |
| B1A041-01 | 669.32 | OEt | A01 | a2 | B11 | b3 | C01 | c1 | D05 |
| B1A042-01 | 670.31 | OEt | A02 | a2 | B11 | b3 | C01 | c1 | D05 |
| B1A043-01 | 711.34 | OEt | A01 | a1 | B12 | b3 | C01 | c1 | D05 |
| B1A044-01 | 712.34 | OEt | A02 | a1 | B12 | b3 | C01 | c1 | D05 |
| B1A045-01 | 563.35 | OEt | A01 | a1 | B01 | b1 | C01 | c1 | D05 |

| | | | | | | | | | |
|---|---|---|---|---|---|---|---|---|---|
| B1A046-01 | 564.35 | OEt | A02 | a1 | B01 | b1 | C01 | c1 | D05 |
| B1A047-01 | 577.37 | OEt | A01 | a1 | B04 | b1 | C01 | c1 | D05 |
| B1A048-01 | 578.36 | OEt | A02 | a1 | B04 | b1 | C01 | c1 | D05 |
| B1A049-01 | 591.38 | OEt | A01 | a1 | B05 | b1 | C01 | c1 | D05 |
| B1A050-01 | 592.38 | OEt | A02 | a1 | B05 | b1 | C01 | c1 | D05 |
| B1A051-01 | 581.34 | OEt | A01 | a1 | B06 | b1 | C01 | c1 | D05 |
| B1A052-01 | 582.34 | OEt | A02 | a1 | B06 | b1 | C01 | c1 | D05 |
| B1A053-01 | 593.36 | OEt | A01 | a1 | B07 | b1 | C01 | c1 | D05 |
| B1A054-01 | 594.36 | OEt | A02 | a1 | B07 | b1 | C01 | c1 | D05 |
| B1A055-01 | 607.38 | OEt | A01 | a1 | B14 | b1 | C01 | c1 | D05 |
| B1A056-01 | 608.37 | OEt | A02 | a1 | B14 | b1 | C01 | c1 | D05 |
| B1A057-01 | 621.39 | OEt | A01 | a1 | B15 | b1 | C01 | c1 | D05 |
| B1A058-01 | 622.39 | OEt | A02 | a1 | B15 | b1 | C01 | c1 | D05 |
| B1A059-01 | 623.37 | OEt | A01 | a1 | B08 | b1 | C01 | c1 | D05 |
| B1A060-01 | 624.37 | OEt | A02 | a1 | B08 | b1 | C01 | c1 | D05 |
| B1A061-01 | 631.34 | OEt | A01 | a1 | B11 | b1 | C01 | c1 | D05 |
| B1A062-01 | 632.33 | OEt | A02 | a1 | B11 | b1 | C01 | c1 | D05 |
| B1A063-01 | 647.33 | OEt | A01 | a1 | B13 | b1 | C01 | c1 | D05 |
| B1A064-01 | 648.33 | OEt | A02 | a1 | B13 | b1 | C01 | c1 | D05 |
| B1A065-01 | 613.37 | OEt | A01 | a1 | B09 | b1 | C01 | c1 | D05 |
| B1A066-01 | 614.36 | OEt | A02 | a1 | B09 | b1 | C01 | c1 | D05 |
| B1A067-01 | 569.31 | OEt | A01 | a1 | B10 | b1 | C01 | c1 | D05 |
| B1A068-01 | 570.3 | OEt | A02 | a1 | B10 | b1 | C01 | c1 | D05 |
| B1A069-01 | 587.35 | OEt | A01 | a2 | B01 | b1 | C01 | c1 | D05 |
| B1A070-01 | 588.35 | OEt | A02 | a2 | B01 | b1 | C01 | c1 | D05 |
| B1A071-01 | 565.34 | OEt | A01 | a1 | B01 | b1 | C02 | c1 | D05 |
| B1A072-01 | 566.34 | OEt | A02 | a1 | B01 | b1 | C02 | c1 | D05 |
| B1A073-01 | 579.36 | OEt | A01 | a1 | B04 | b1 | C02 | c1 | D05 |
| B1A074-01 | 580.35 | OEt | A02 | a1 | B04 | b1 | C02 | c1 | D05 |
| B1A075-01 | 593.37 | OEt | A01 | a1 | B05 | b1 | C02 | c1 | D05 |
| B1A076-01 | 594.37 | OEt | A02 | a1 | B05 | b1 | C02 | c1 | D05 |
| B1A077-01 | 583.33 | OEt | A01 | a1 | B06 | b1 | C02 | c1 | D05 |
| B1A078-01 | 584.33 | OEt | A02 | a1 | B06 | b1 | C02 | c1 | D05 |
| B1A079-01 | 595.35 | OEt | A01 | a1 | B07 | b1 | C02 | c1 | D05 |
| B1A080-01 | 596.35 | OEt | A02 | a1 | B07 | b1 | C02 | c1 | D05 |
| B1A081-01 | 609.37 | OEt | A01 | a1 | B14 | b1 | C02 | c1 | D05 |
| B1A082-01 | 610.36 | OEt | A02 | a1 | B14 | b1 | C02 | c1 | D05 |
| B1A083-01 | 623.38 | OEt | A01 | a1 | B15 | b1 | C02 | c1 | D05 |
| B1A084-01 | 624.38 | OEt | A02 | a1 | B15 | b1 | C02 | c1 | D05 |
| B1A085-01 | 625.36 | OEt | A01 | a1 | B08 | b1 | C02 | c1 | D05 |
| B1A086-01 | 626.36 | OEt | A02 | a1 | B08 | b1 | C02 | c1 | D05 |
| B1A087-01 | 633.33 | OEt | A01 | a1 | B11 | b1 | C02 | c1 | D05 |
| B1A088-01 | 634.32 | OEt | A02 | a1 | B11 | b1 | C02 | c1 | D05 |
| B1A089-01 | 649.32 | OEt | A01 | a1 | B13 | b1 | C02 | c1 | D05 |
| B1A090-01 | 650.32 | OEt | A02 | a1 | B13 | b1 | C02 | c1 | D05 |
| B1A091-01 | 615.36 | OEt | A01 | a1 | B09 | b1 | C02 | c1 | D05 |
| B1A092-01 | 616.35 | OEt | A02 | a1 | B09 | b1 | C02 | c1 | D05 |

| | | | | | | | | | |
|---|---|---|---|---|---|---|---|---|---|
| B1A093-01 | 571.3 | OEt | A01 | a1 | B10 | b1 | C02 | c1 | D05 |
| B1A094-01 | 572.29 | OEt | A02 | a1 | B10 | b1 | C02 | c1 | D05 |
| B1A095-01 | 589.34 | OEt | A01 | a2 | B01 | b1 | C02 | c1 | D05 |
| B1A096-01 | 590.34 | OEt | A02 | a2 | B01 | b1 | C02 | c1 | D05 |
| B1A097-01 | 577.37 | OEt | A01 | a1 | B03 | b2 | C01 | c1 | D05 |
| B1A098-01 | 578.36 | OEt | A02 | a1 | B03 | b2 | C01 | c1 | D05 |
| B1A099-01 | 595.36 | OEt | A01 | a1 | B18 | b2 | C01 | c1 | D05 |
| B1A100-01 | 596.35 | OEt | A02 | a1 | B18 | b2 | C01 | c1 | D05 |

[Table 252]

[Table AI-03-B3A-01]

| Mixture No. | mixAI-B3A-01 |
| --- | --- |
| No. of compound that may be contained | Compound name |
| B3A001-01 | N-(1-Adamantylmethyl)-6-[4-[4-(3-ethoxyphenyl)thiophene-2-carbonyl]piperazin-1-yl]-N-methylpyridazine-3-carboxamide |
| B3A002-01 | N-(1-Adamantylmethyl)-6-[4-[4-(5-ethoxypyridin-3-yl)thiophene-2-carbonyl]piperazin-1-yl]-N-methylpyridazine-3-carboxamide |
| B3A003-01 | N-(1-Adamantylmethyl)-6-[4-[4-(3-ethoxyphenyl)-3-methylbenzoyl]piperazin-1-yl]-N-methylpyridazine-3-carboxamide |
| B3A004-01 | N-(1-Adamantylmethyl)-6-[4-[4-(5-ethoxypyridin-3-yl)-3-methylbenzoyl]piperazin-1-yl]-N-methylpyridazine-3-carboxamide |
| B3A005-01 | N-(1-Adamantylmethyl)-6-[4-[4-(3-ethoxyphenyl)-3-fluorobenzoyl]piperazin-1-yl]-N-methylpyridazine-3-carboxamide |
| B3A006-01 | N-(1-Adamantylmethyl)-6-[4-[4-(5-ethoxypyridin-3-yl)-3-fluorobenzoyl]piperazin-1-yl]-N-methylpyridazine-3-carboxamide |
| B3A007-01 | N-(1-Adamantylmethyl)-6-[4-[2-[2-(3-ethoxyphenyl)ethynyl]benzoyl]piperazin-1-yl]-N-methylpyridazine-3-carboxamide |
| B3A008-01 | N-(1-Adamantylmethyl)-6-[4-[2-[2-(5-ethoxypyridin-3-yl)ethynyl]benzoyl]piperazin-1-yl]-N-methylpyridazine-3-carboxamide |
| B3A009-01 | N-(1-Adamantylmethyl)-6-[4-[5-[2-(3-ethoxyphenyl)ethynyl]pyridine-3-carbonyl]piperazin-1-yl]-N-methylpyridazine-3-carboxamide |
| B3A010-01 | N-(1-Adamantylmethyl)-6-[4-[5-[2-(5-ethoxypyridin-3-yl)ethynyl]pyridine-3-carbonyl]piperazin-1-yl]-N-methylpyridazine-3-carboxamide |
| B3A011-01 | N-(1-Adamantylmethyl)-6-[4-[3-[2-(3-ethoxyphenyl)phenyl]propanoyl]piperazin-1-yl]-N-methylpyridazine-3-carboxamide |
| B3A012-01 | N-(1-Adamantylmethyl)-6-[4-[3-[2-(5-ethoxypyridin-3-yl)phenyl]propanoyl]piperazin-1-yl]-N-methylpyridazine-3-carboxamide |
| B3A013-01 | N-(1-Adamantylmethyl)-6-[4-[4-(3-ethoxyphenyl)naphthalene-1-carbonyl]piperazin-1-yl]-N-methylpyridazine-3-carboxamide |
| B3A014-01 | N-(1-Adamantylmethyl)-6-[4-[4-(5-ethoxypyridin-3-yl)naphthalene-1-carbonyl]piperazin-1-yl]-N-methylpyridazine-3-carboxamide |
| B3A015-01 | N-(1-Adamantylmethyl)-6-[4-[3-tert-butyl-5-(3-ethoxyphenyl)benzoyl]piperazin-1-yl]-N-methylpyridazine-3-carboxamide |
| B3A016-01 | N-(1-Adamantylmethyl)-6-[4-[3-tert-butyl-5-(5-ethoxypyridin-3-yl)benzoyl]piperazin-1-yl]-N-methylpyridazine-3-carboxamide |
| B3A017-01 | N-(1-Adamantylmethyl)-6-[4-[3-(3-ethoxyphenyl)-5-(trifluoromethyl)benzoyl]piperazin-1-yl]-N-methylpyridazine-3-carboxamide |
| B3A018-01 | N-(1-Adamantylmethyl)-6-[4-[3-(5-ethoxypyridin-3-yl)-5-(trifluoromethyl)benzoyl]piperazin-1-yl]-N-methylpyridazine-3-carboxamide |
| B3A019-01 | N-(1-Adamantylmethyl)-6-[4-[3-[2-(3-ethoxyphenyl)ethynyl]-5-(trifluoromethyl)benzoyl]piperazin-1-yl]-N-methylpyridazine-3-carboxamide |

| | |
|---|---|
| B3A020-01 | N-(1-Adamantylmethyl)-6-[4-[3-[2-(5-ethoxypyridin-3-yl)ethynyl]-5-(trifluoromethyl)benzoyl]piperazin-1-yl]-N-methylpyridazine-3-carboxamide |
| B3A021-01 | N-(1-Adamantylmethyl)-6-[4-[3-[4-(3-ethoxyphenyl)pyrazol-1-yl]-5-(trifluoromethyl)benzoyl]piperazin-1-yl]-N-methylpyridazine-3-carboxamide |
| B3A022-01 | N-(1-Adamantylmethyl)-6-[4-[3-[4-(5-ethoxypyridin-3-yl)pyrazol-1-yl]-5-(trifluoromethyl)benzoyl]piperazin-1-yl]-N-methylpyridazine-3-carboxamide |
| B3A023-01 | N-(1-Adamantylmethyl)-4-[4-[4-(3-ethoxyphenyl)thiophene-2-carbonyl]piperazin-1-yl]-N-methylbenzamide |
| B3A024-01 | N-(1-Adamantylmethyl)-4-[4-[4-(5-ethoxypyridin-3-yl)thiophene-2-carbonyl]piperazin-1-yl]-N-methylbenzamide |
| B3A025-01 | N-(1-Adamantylmethyl)-4-[4-[4-(3-ethoxyphenyl)-3-methylbenzoyl]piperazin-1-yl]-N-methylbenzamide |
| B3A026-01 | N-(1-Adamantylmethyl)-4-[4-[4-(5-ethoxypyridin-3-yl)-3-methylbenzoyl]piperazin-1-yl]-N-methylbenzamide |
| B3A027-01 | N-(1-Adamantylmethyl)-4-[4-[4-(3-ethoxyphenyl)-3-fluorobenzoyl]piperazin-1-yl]-N-methylbenzamide |
| B3A028-01 | N-(1-Adamantylmethyl)-4-[4-[4-(5-ethoxypyridin-3-yl)-3-fluorobenzoyl]piperazin-1-yl]-N-methylbenzamide |
| B3A029-01 | N-(1-Adamantylmethyl)-4-[4-[2-[2-(3-ethoxyphenyl)ethynyl]benzoyl]piperazin-1-yl]-N-methylbenzamide |
| B3A030-01 | N-(1-Adamantylmethyl)-4-[4-[2-[2-(5-ethoxypyridin-3-yl)ethynyl]benzoyl]piperazin-1-yl]-N-methylbenzamide |
| B3A031-01 | N-(1-Adamantylmethyl)-4-[4-[5-[2-(3-ethoxyphenyl)ethynyl]pyridine-3-carbonyl]piperazin-1-yl]-N-methylbenzamide |
| B3A032-01 | N-(1-Adamantylmethyl)-4-[4-[5-[2-(5-ethoxypyridin-3-yl)ethynyl]pyridine-3-carbonyl]piperazin-1-yl]-N-methylbenzamide |
| B3A033-01 | N-(1-Adamantylmethyl)-4-[4-[3-[2-(3-ethoxyphenyl)phenyl]propanoyl]piperazin-1-yl]-N-methylbenzamide |
| B3A034-01 | N-(1-Adamantylmethyl)-4-[4-[3-[2-(5-ethoxypyridin-3-yl)phenyl]propanoyl]piperazin-1-yl]-N-methylbenzamide |
| B3A035-01 | N-(1-Adamantylmethyl)-4-[4-[4-(3-ethoxyphenyl)naphthalene-1-carbonyl]piperazin-1-yl]-N-methylbenzamide |
| B3A036-01 | N-(1-Adamantylmethyl)-4-[4-[4-(5-ethoxypyridin-3-yl)naphthalene-1-carbonyl]piperazin-1-yl]-N-methylbenzamide |
| B3A037-01 | N-(1-Adamantylmethyl)-4-[4-[3-tert-butyl-5-(3-ethoxyphenyl)benzoyl]piperazin-1-yl]-N-methylbenzamide |
| B3A038-01 | N-(1-Adamantylmethyl)-4-[4-[3-tert-butyl-5-(5-ethoxypyridin-3-yl)benzoyl]piperazin-1-yl]-N-methylbenzamide |
| B3A039-01 | N-(1-Adamantylmethyl)-4-[4-[3-(3-ethoxyphenyl)-5-(trifluoromethyl)benzoyl]piperazin-1-yl]-N-methylbenzamide |
| B3A040-01 | N-(1-Adamantylmethyl)-4-[4-[3-(5-ethoxypyridin-3-yl)-5-(trifluoromethyl)benzoyl]piperazin-1-yl]-N-methylbenzamide |
| B3A041-01 | N-(1-Adamantylmethyl)-4-[4-[3-[2-(3-ethoxyphenyl)ethynyl]-5-(trifluoromethyl)benzoyl]piperazin-1-yl]-N-methylbenzamide |
| B3A042-01 | N-(1-Adamantylmethyl)-4-[4-[3-[2-(5-ethoxypyridin-3-yl)ethynyl]-5-(trifluoromethyl)benzoyl]piperazin-1-yl]-N-methylbenzamide |
| B3A043-01 | N-(1-Adamantylmethyl)-4-[4-[3-[4-(3-ethoxyphenyl)pyrazol-1-yl]-5-(trifluoromethyl)benzoyl]piperazin-1-yl]-N-methylbenzamide |
| B3A044-01 | N-(1-Adamantylmethyl)-4-[4-[3-[4-(5-ethoxypyridin-3-yl)pyrazol-1-yl]-5-(trifluoromethyl)benzoyl]piperazin-1-yl]-N-methylbenzamide |
| B3A045-01 | N-(1-Adamantylmethyl)-4-[4-[[2-(3-ethoxyphenyl)phenyl]methyl]piperazin-1-yl]-N-methylbenzamide |
| B3A046-01 | N-(1-Adamantylmethyl)-4-[4-[[2-(5-ethoxypyridin-3-yl)phenyl]methyl]piperazin-1-yl]-N-methylbenzamide |
| B3A047-01 | N-(1-Adamantylmethyl)-4-[4-[[4-(3-ethoxyphenyl)-3-methylphenyl]methyl]piperazin-1-yl]-N-methylbenzamide |

| | |
|---|---|
| B3A048-01 | N-(1-Adamantylmethyl)-4-[4-[[4-(5-ethoxypyridin-3-yl)-3-methylphenyl]methyl]piperazin-1-yl]-N-methylbenzamide |
| B3A049-01 | N-(1-Adamantylmethyl)-4-[4-[[4-(3-ethoxyphenyl)-2-ethylphenyl]methyl]piperazin-1-yl]-N-methylbenzamide |
| B3A050-01 | N-(1-Adamantylmethyl)-4-[4-[[4-(5-ethoxypyridin-3-yl)-2-ethylphenyl]methyl]piperazin-1-yl]-N-methylbenzamide |
| B3A051-01 | N-(1-Adamantylmethyl)-4-[4-[[4-(3-ethoxyphenyl)-3-fluorophenyl]methyl]piperazin-1-yl]-N-methylbenzamide |
| B3A052-01 | N-(1-Adamantylmethyl)-4-[4-[[4-(5-ethoxypyridin-3-yl)-3-fluorophenyl]methyl]piperazin-1-yl]-N-methylbenzamide |
| B3A053-01 | N-(1-Adamantylmethyl)-4-[4-[[4-(3-ethoxyphenyl)-2-methoxyphenyl]methyl]piperazin-1-yl]-N-methylbenzamide |
| B3A054-01 | N-(1-Adamantylmethyl)-4-[4-[[4-(5-ethoxypyridin-3-yl)-2-methoxyphenyl]methyl]piperazin-1-yl]-N-methylbenzamide |
| B3A055-01 | N-(1-Adamantylmethyl)-4-[4-[[3-ethoxy-5-(3-ethoxyphenyl)phenyl]methyl]piperazin-1-yl]-N-methylbenzamide |
| B3A056-01 | N-(1-Adamantylmethyl)-4-[4-[[3-ethoxy-5-(5-ethoxypyridin-3-yl)phenyl]methyl]piperazin-1-yl]-N-methylbenzamide |
| B3A057-01 | N-(1-Adamantylmethyl)-4-[4-[[3-(3-ethoxyphenyl)-5-propan-2-yloxyphenyl]methyl]piperazin-1-yl]-N-methylbenzamide |
| B3A058-01 | N-(1-Adamantylmethyl)-4-[4-[[3-(5-ethoxypyridin-3-yl)-5-propan-2-yloxyphenyl]methyl]piperazin-1-yl]-N-methylbenzamide |
| B3A059-01 | N-(1-Adamantylmethyl)-4-[4-[[4-(3-ethoxyphenyl)-2,6-dimethoxyphenyl]methyl]piperazin-1-yl]-N-methylbenzamide |
| B3A060-01 | N-(1-Adamantylmethyl)-4-[4-[[4-(5-ethoxypyridin-3-yl)-2,6-dimethoxyphenyl]methyl]piperazin-1-yl]-N-methylbenzamide |
| B3A061-01 | N-(1-Adamantylmethyl)-4-[4-[[3-(3-ethoxyphenyl)-5-(trifluoromethyl)phenyl]methyl]piperazin-1-yl]-N-methylbenzamide |
| B3A062-01 | N-(1-Adamantylmethyl)-4-[4-[[3-(5-ethoxypyridin-3-yl)-5-(trifluoromethyl)phenyl]methyl]piperazin-1-yl]-N-methylbenzamide |
| B3A063-01 | N-(1-Adamantylmethyl)-4-[4-[[3-(3-ethoxyphenyl)-5-(trifluoromethoxy)phenyl]methyl]piperazin-1-yl]-N-methylbenzamide |
| B3A064-01 | N-(1-Adamantylmethyl)-4-[4-[[3-(5-ethoxypyridin-3-yl)-5-(trifluoromethoxy)phenyl]methyl]piperazin-1-yl]-N-methylbenzamide |
| B3A065-01 | N-(1-Adamantylmethyl)-4-[4-[[4-(3-ethoxyphenyl)naphthalen-1-yl]methyl]piperazin-1-yl]-N-methylbenzamide |
| B3A066-01 | N-(1-Adamantylmethyl)-4-[4-[[4-(5-ethoxypyridin-3-yl)naphthalen-1-yl]methyl]piperazin-1-yl]-N-methylbenzamide |
| B3A067-01 | N-(1-Adamantylmethyl)-4-[4-[[4-(3-ethoxyphenyl)thiophen-2-yl]methyl]piperazin-1-yl]-N-methylbenzamide |
| B3A068-01 | N-(1-Adamantylmethyl)-4-[4-[[4-(5-ethoxypyridin-3-yl)thiophen-2-yl]methyl]piperazin-1-yl]-N-methylbenzamide |
| B3A069-01 | N-(1-Adamantylmethyl)-4-[4-[[2-[2-(3-ethoxyphenyl)ethynyl]phenyl]methyl]piperazin-1-yl]-N-methylbenzamide |
| B3A070-01 | N-(1-Adamantylmethyl)-4-[4-[[2-[2-(5-ethoxypyridin-3-yl)ethynyl]phenyl]methyl]piperazin-1-yl]-N-methylbenzamide |
| B3A071-01 | N-(1-Adamantylmethyl)-6-[4-[[2-(3-ethoxyphenyl)phenyl]methyl]piperazin-1-yl]-N-methylpyridazine-3-carboxamide |
| B3A072-01 | N-(1-Adamantylmethyl)-6-[4-[[2-(5-ethoxypyridin-3-yl)phenyl]methyl]piperazin-1-yl]-N-methylpyridazine-3-carboxamide |
| B3A073-01 | N-(1-Adamantylmethyl)-6-[4-[[4-(3-ethoxyphenyl)-3-methylphenyl]methyl]piperazin-1-yl]-N-methylpyridazine-3-carboxamide |
| B3A074-01 | N-(1-Adamantylmethyl)-6-[4-[[4-(5-ethoxypyridin-3-yl)-3-methylphenyl]methyl]piperazin-1-yl]-N-methylpyridazine-3-carboxamide |
| B3A075-01 | N-(1-Adamantylmethyl)-6-[4-[[4-(3-ethoxyphenyl)-2-ethylphenyl]methyl]piperazin-1-yl]-N-methylpyridazine-3-carboxamide |

| | |
|---|---|
| B3A076-01 | N-(1-Adamantylmethyl)-6-[4-[[4-(5-ethoxypyridin-3-yl)-2-ethylphenyl]methyl]piperazin-1-yl]-N-methylpyridazine-3-carboxamide |
| B3A077-01 | N-(1-Adamantylmethyl)-6-[4-[[4-(3-ethoxyphenyl)-3-fluorophenyl]methyl]piperazin-1-yl]-N-methylpyridazine-3-carboxamide |
| B3A078-01 | N-(1-Adamantylmethyl)-6-[4-[[4-(5-ethoxypyridin-3-yl)-3-fluorophenyl]methyl]piperazin-1-yl]-N-methylpyridazine-3-carboxamide |
| B3A079-01 | N-(1-Adamantylmethyl)-6-[4-[[4-(3-ethoxyphenyl)-2-methoxyphenyl]methyl]piperazin-1-yl]-N-methylpyridazine-3-carboxamide |
| B3A080-01 | N-(1-Adamantylmethyl)-6-[4-[[4-(5-ethoxypyridin-3-yl)-2-methoxyphenyl]methyl]piperazin-1-yl]-N-methylpyridazine-3-carboxamide |
| B3A081-01 | N-(1-Adamantylmethyl)-6-[4-[[3-ethoxy-5-(3-ethoxyphenyl)phenyl]methyl]piperazin-1-yl]-N-methylpyridazine-3-carboxamide |
| B3A082-01 | N-(1-Adamantylmethyl)-6-[4-[[3-ethoxy-5-(5-ethoxypyridin-3-yl)phenyl]methyl]piperazin-1-yl]-N-methylpyridazine-3-carboxamide |
| B3A083-01 | N-(1-Adamantylmethyl)-6-[4-[[3-(3-ethoxyphenyl)-5-propan-2-yloxyphenyl]methyl]piperazin-1-yl]-N-methylpyridazine-3-carboxamide |
| B3A084-01 | N-(1-Adamantylmethyl)-6-[4-[[3-(5-ethoxypyridin-3-yl)-5-propan-2-yloxyphenyl]methyl]piperazin-1-yl]-N-methylpyridazine-3-carboxamide |
| B3A085-01 | N-(1-Adamantylmethyl)-6-[4-[[4-(3-ethoxyphenyl)-2,6-dimethoxyphenyl]methyl]piperazin-1-yl]-N-methylpyridazine-3-carboxamide |
| B3A086-01 | N-(1-Adamantylmethyl)-6-[4-[[4-(5-ethoxypyridin-3-yl)-2,6-dimethoxyphenyl]methyl]piperazin-1-yl]-N-methylpyridazine-3-carboxamide |
| B3A087-01 | N-(1-Adamantylmethyl)-6-[4-[[3-(3-ethoxyphenyl)-5-(trifluoromethyl)phenyl]methyl]piperazin-1-yl]-N-methylpyridazine-3-carboxamide |
| B3A088-01 | N-(1-Adamantylmethyl)-6-[4-[[3-(5-ethoxypyridin-3-yl)-5-(trifluoromethyl)phenyl]methyl]piperazin-1-yl]-N-methylpyridazine-3-carboxamide |
| B3A089-01 | N-(1-Adamantylmethyl)-6-[4-[[3-(3-ethoxyphenyl)-5-(trifluoromethoxy)phenyl]methyl]piperazin-1-yl]-N-methylpyridazine-3-carboxamide |
| B3A090-01 | N-(1-Adamantylmethyl)-6-[4-[[3-(5-ethoxypyridin-3-yl)-5-(trifluoromethoxy)phenyl]methyl]piperazin-1-yl]-N-methylpyridazine-3-carboxamide |
| B3A091-01 | N-(1-Adamantylmethyl)-6-[4-[[4-(3-ethoxyphenyl)naphthalen-1-yl]methyl]piperazin-1-yl]-N-methylpyridazine-3-carboxamide |
| B3A092-01 | N-(1-Adamantylmethyl)-6-[4-[[4-(5-ethoxypyridin-3-yl)naphthalen-1-yl]methyl]piperazin-1-yl]-N-methylpyridazine-3-carboxamide |
| B3A093-01 | N-(1-Adamantylmethyl)-6-[4-[[4-(3-ethoxyphenyl)thiophen-2-yl]methyl]piperazin-1-yl]-N-methylpyridazine-3-carboxamide |
| B3A094-01 | N-(1-Adamantylmethyl)-6-[4-[[4-(5-ethoxypyridin-3-yl)thiophen-2-yl]methyl]piperazin-1-yl]-N-methylpyridazine-3-carboxamide |
| B3A095-01 | N-(1-Adamantylmethyl)-6-[4-[[2-[2-(3-ethoxyphenyl)ethynyl]phenyl]methyl]piperazin-1-yl]-N-methylpyridazine-3-carboxamide |
| B3A096-01 | N-(1-Adamantylmethyl)-6-[4-[[2-[2-(5-ethoxypyridin-3-yl)ethynyl]phenyl]methyl]piperazin-1-yl]-N-methylpyridazine-3-carboxamide |
| B3A097-01 | N-(1-Adamantylmethyl)-4-[4-[1-[4-(3-ethoxyphenyl)phenyl]ethyl]piperazin-1-yl]-N-methylbenzamide |
| B3A098-01 | N-(1-Adamantylmethyl)-4-[4-[1-[4-(5-ethoxypyridin-3-yl)phenyl]ethyl]piperazin-1-yl]-N-methylbenzamide |
| B3A099-01 | N-(1-Adamantylmethyl)-4-[4-[1-[4-(3-ethoxyphenyl)-2-fluorophenyl]ethyl]piperazin-1-yl]-N-methylbenzamide |
| B3A100-01 | N-(1-Adamantylmethyl)-4-[4-[1-[4-(5-ethoxypyridin-3-yl)-2-fluorophenyl]ethyl]piperazin-1-yl]-N-methylbenzamide |

[Table 253]

[Table AI-03-B3A-01]

| Label | Exact | ☒ | A | a | B | b | C | c | D |
|---|---|---|---|---|---|---|---|---|---|
| B3A001-01 | 599.29 | OEt | A01 | a1 | B10 | b3 | C02 | c1 | D06 |
| B3A002-01 | 600.29 | OEt | A02 | a1 | B10 | b3 | C02 | c1 | D06 |
| B3A003-01 | 607.35 | OEt | A01 | a1 | B04 | b3 | C02 | c1 | D06 |
| B3A004-01 | 608.35 | OEt | A02 | a1 | B04 | b3 | C02 | c1 | D06 |
| B3A005-01 | 611.33 | OEt | A01 | a1 | B06 | b3 | C02 | c1 | D06 |
| B3A006-01 | 612.32 | OEt | A02 | a1 | B06 | b3 | C02 | c1 | D06 |
| B3A007-01 | 617.34 | OEt | A01 | a2 | B01 | b3 | C02 | c1 | D06 |
| B3A008-01 | 618.33 | OEt | A02 | a2 | B01 | b3 | C02 | c1 | D06 |
| B3A009-01 | 618.33 | OEt | A01 | a2 | B17 | b3 | C02 | c1 | D06 |
| B3A010-01 | 619.33 | OEt | A02 | a2 | B17 | b3 | C02 | c1 | D06 |
| B3A011-01 | 621.37 | OEt | A01 | a1 | B02 | b3 | C02 | c1 | D06 |
| B3A012-01 | 622.36 | OEt | A02 | a1 | B02 | b3 | C02 | c1 | D06 |
| B3A013-01 | 643.35 | OEt | A01 | a1 | B09 | b3 | C02 | c1 | D06 |
| B3A014-01 | 644.35 | OEt | A02 | a1 | B09 | b3 | C02 | c1 | D06 |
| B3A015-01 | 649.4 | OEt | A01 | a1 | B16 | b3 | C02 | c1 | D06 |
| B3A016-01 | 650.39 | OEt | A02 | a1 | B16 | b3 | C02 | c1 | D06 |
| B3A017-01 | 661.32 | OEt | A01 | a1 | B11 | b3 | C02 | c1 | D06 |
| B3A018-01 | 662.32 | OEt | A02 | a1 | B11 | b3 | C02 | c1 | D06 |
| B3A019-01 | 685.32 | OEt | A01 | a2 | B11 | b3 | C02 | c1 | D06 |
| B3A020-01 | 686.32 | OEt | A02 | a2 | B11 | b3 | C02 | c1 | D06 |
| B3A021-01 | 727.35 | OEt | A01 | a1 | B12 | b3 | C02 | c1 | D06 |
| B3A022-01 | 728.34 | OEt | A02 | a1 | B12 | b3 | C02 | c1 | D06 |
| B3A023-01 | 597.3 | OEt | A01 | a1 | B10 | b3 | C01 | c1 | D06 |
| B3A024-01 | 598.3 | OEt | A02 | a1 | B10 | b3 | C01 | c1 | D06 |
| B3A025-01 | 605.36 | OEt | A01 | a1 | B04 | b3 | C01 | c1 | D06 |
| B3A026-01 | 606.36 | OEt | A02 | a1 | B04 | b3 | C01 | c1 | D06 |
| B3A027-01 | 609.34 | OEt | A01 | a1 | B06 | b3 | C01 | c1 | D06 |
| B3A028-01 | 610.33 | OEt | A02 | a1 | B06 | b3 | C01 | c1 | D06 |
| B3A029-01 | 615.35 | OEt | A01 | a2 | B01 | b3 | C01 | c1 | D06 |
| B3A030-01 | 616.34 | OEt | A02 | a2 | B01 | b3 | C01 | c1 | D06 |
| B3A031-01 | 616.34 | OEt | A01 | a2 | B17 | b3 | C01 | c1 | D06 |
| B3A032-01 | 617.34 | OEt | A02 | a2 | B17 | b3 | C01 | c1 | D06 |
| B3A033-01 | 619.38 | OEt | A01 | a1 | B02 | b3 | C01 | c1 | D06 |
| B3A034-01 | 620.37 | OEt | A02 | a1 | B02 | b3 | C01 | c1 | D06 |
| B3A035-01 | 641.36 | OEt | A01 | a1 | B09 | b3 | C01 | c1 | D06 |
| B3A036-01 | 642.36 | OEt | A02 | a1 | B09 | b3 | C01 | c1 | D06 |
| B3A037-01 | 647.41 | OEt | A01 | a1 | B16 | b3 | C01 | c1 | D06 |
| B3A038-01 | 648.4 | OEt | A02 | a1 | B16 | b3 | C01 | c1 | D06 |
| B3A039-01 | 659.33 | OEt | A01 | a1 | B11 | b3 | C01 | c1 | D06 |
| B3A040-01 | 660.33 | OEt | A02 | a1 | B11 | b3 | C01 | c1 | D06 |
| B3A041-01 | 683.33 | OEt | A01 | a2 | B11 | b3 | C01 | c1 | D06 |
| B3A042-01 | 684.33 | OEt | A02 | a2 | B11 | b3 | C01 | c1 | D06 |
| B3A043-01 | 725.36 | OEt | A01 | a1 | B12 | b3 | C01 | c1 | D06 |
| B3A044-01 | 726.35 | OEt | A02 | a1 | B12 | b3 | C01 | c1 | D06 |
| B3A045-01 | 577.37 | OEt | A01 | a1 | B01 | b1 | C01 | c1 | D06 |

| | | | | | | | | | |
|---|---|---|---|---|---|---|---|---|---|
| B3A046-01 | 578.36 | OEt | A02 | a1 | B01 | b1 | C01 | c1 | D06 |
| B3A047-01 | 591.38 | OEt | A01 | a1 | B04 | b1 | C01 | c1 | D06 |
| B3A048-01 | 592.38 | OEt | A02 | a1 | B04 | b1 | C01 | c1 | D06 |
| B3A049-01 | 605.4 | OEt | A01 | a1 | B05 | b1 | C01 | c1 | D06 |
| B3A050-01 | 606.39 | OEt | A02 | a1 | B05 | b1 | C01 | c1 | D06 |
| B3A051-01 | 595.36 | OEt | A01 | a1 | B06 | b1 | C01 | c1 | D06 |
| B3A052-01 | 596.35 | OEt | A02 | a1 | B06 | b1 | C01 | c1 | D06 |
| B3A053-01 | 607.38 | OEt | A01 | a1 | B07 | b1 | C01 | c1 | D06 |
| B3A054-01 | 608.37 | OEt | A02 | a1 | B07 | b1 | C01 | c1 | D06 |
| B3A055-01 | 621.39 | OEt | A01 | a1 | B14 | b1 | C01 | c1 | D06 |
| B3A056-01 | 622.39 | OEt | A02 | a1 | B14 | b1 | C01 | c1 | D06 |
| B3A057-01 | 635.41 | OEt | A01 | a1 | B15 | b1 | C01 | c1 | D06 |
| B3A058-01 | 636.4 | OEt | A02 | a1 | B15 | b1 | C01 | c1 | D06 |
| B3A059-01 | 637.39 | OEt | A01 | a1 | B08 | b1 | C01 | c1 | D06 |
| B3A060-01 | 638.38 | OEt | A02 | a1 | B08 | b1 | C01 | c1 | D06 |
| B3A061-01 | 645.35 | OEt | A01 | a1 | B11 | b1 | C01 | c1 | D06 |
| B3A062-01 | 646.35 | OEt | A02 | a1 | B11 | b1 | C01 | c1 | D06 |
| B3A063-01 | 661.35 | OEt | A01 | a1 | B13 | b1 | C01 | c1 | D06 |
| B3A064-01 | 662.34 | OEt | A02 | a1 | B13 | b1 | C01 | c1 | D06 |
| B3A065-01 | 627.38 | OEt | A01 | a1 | B09 | b1 | C01 | c1 | D06 |
| B3A066-01 | 628.38 | OEt | A02 | a1 | B09 | b1 | C01 | c1 | D06 |
| B3A067-01 | 583.32 | OEt | A01 | a1 | B10 | b1 | C01 | c1 | D06 |
| B3A068-01 | 584.32 | OEt | A02 | a1 | B10 | b1 | C01 | c1 | D06 |
| B3A069-01 | 601.37 | OEt | A01 | a2 | B01 | b1 | C01 | c1 | D06 |
| B3A070-01 | 602.36 | OEt | A02 | a2 | B01 | b1 | C01 | c1 | D06 |
| B3A071-01 | 579.36 | OEt | A01 | a1 | B01 | b1 | C02 | c1 | D06 |
| B3A072-01 | 580.35 | OEt | A02 | a1 | B01 | b1 | C02 | c1 | D06 |
| B3A073-01 | 593.37 | OEt | A01 | a1 | B04 | b1 | C02 | c1 | D06 |
| B3A074-01 | 594.37 | OEt | A02 | a1 | B04 | b1 | C02 | c1 | D06 |
| B3A075-01 | 607.39 | OEt | A01 | a1 | B05 | b1 | C02 | c1 | D06 |
| B3A076-01 | 608.38 | OEt | A02 | a1 | B05 | b1 | C02 | c1 | D06 |
| B3A077-01 | 597.35 | OEt | A01 | a1 | B06 | b1 | C02 | c1 | D06 |
| B3A078-01 | 598.34 | OEt | A02 | a1 | B06 | b1 | C02 | c1 | D06 |
| B3A079-01 | 609.37 | OEt | A01 | a1 | B07 | b1 | C02 | c1 | D06 |
| B3A080-01 | 610.36 | OEt | A02 | a1 | B07 | b1 | C02 | c1 | D06 |
| B3A081-01 | 623.38 | OEt | A01 | a1 | B14 | b1 | C02 | c1 | D06 |
| B3A082-01 | 624.38 | OEt | A02 | a1 | B14 | b1 | C02 | c1 | D06 |
| B3A083-01 | 637.4 | OEt | A01 | a1 | B15 | b1 | C02 | c1 | D06 |
| B3A084-01 | 638.39 | OEt | A02 | a1 | B15 | b1 | C02 | c1 | D06 |
| B3A085-01 | 639.38 | OEt | A01 | a1 | B08 | b1 | C02 | c1 | D06 |
| B3A086-01 | 640.37 | OEt | A02 | a1 | B08 | b1 | C02 | c1 | D06 |
| B3A087-01 | 647.35 | OEt | A01 | a1 | B11 | b1 | C02 | c1 | D06 |
| B3A088-01 | 648.34 | OEt | A02 | a1 | B11 | b1 | C02 | c1 | D06 |
| B3A089-01 | 663.34 | OEt | A01 | a1 | B13 | b1 | C02 | c1 | D06 |
| B3A090-01 | 664.34 | OEt | A02 | a1 | B13 | b1 | C02 | c1 | D06 |
| B3A091-01 | 629.37 | OEt | A01 | a1 | B09 | b1 | C02 | c1 | D06 |
| B3A092-01 | 630.37 | OEt | A02 | a1 | B09 | b1 | C02 | c1 | D06 |

| | | | | | | | | | |
|---|---|---|---|---|---|---|---|---|---|
| B3A093-01 | 585.31 | OEt | A01 | a1 | B10 | b1 | C02 | c1 | D06 |
| B3A094-01 | 586.31 | OEt | A02 | a1 | B10 | b1 | C02 | c1 | D06 |
| B3A095-01 | 603.36 | OEt | A01 | a2 | B01 | b1 | C02 | c1 | D06 |
| B3A096-01 | 604.35 | OEt | A02 | a2 | B01 | b1 | C02 | c1 | D06 |
| B3A097-01 | 591.38 | OEt | A01 | a1 | B03 | b2 | C01 | c1 | D06 |
| B3A098-01 | 592.38 | OEt | A02 | a1 | B03 | b2 | C01 | c1 | D06 |
| B3A099-01 | 609.37 | OEt | A01 | a1 | B18 | b2 | C01 | c1 | D06 |
| B3A100-01 | 610.37 | OEt | A02 | a1 | B18 | b2 | C01 | c1 | D06 |

[Table 254]

[Table AI-03-B2I-01]

| Mixture No. | mixAI-B2I-01 |
|---|---|
| No. of compound that may be contained | Compound name |
| B2I001-01 | 6-[4-[4-(3-Ethoxyphenyl)thiophene-2-carbonyl]piperazin-1-yl]-N-methylsulfonylpyridazine-3-carboxamide |
| B2I002-01 | 6-[4-[4-(5-Ethoxypyridin-3-yl)thiophene-2-carbonyl]piperazin-1-yl]-N-methylsulfonylpyridazine-3-carboxamide |
| B2I003-01 | 6-[4-[4-(3-Ethoxyphenyl)-3-methylbenzoyl]piperazin-1-yl]-N-methylsulfonylpyridazine-3-carboxamide |
| B2I004-01 | 6-[4-[4-(5-Ethoxypyridin-3-yl)-3-methylbenzoyl]piperazin-1-yl]-N-methylsulfonylpyridazine-3-carboxamide |
| B2I005-01 | 6-[4-[4-(3-Ethoxyphenyl)-3-fluorobenzoyl]piperazin-1-yl]-N-methylsulfonylpyridazine-3-carboxamide |
| B2I006-01 | 6-[4-[4-(5-Ethoxypyridin-3-yl)-3-fluorobenzoyl]piperazin-1-yl]-N-methylsulfonylpyridazine-3-carboxamide |
| B2I007-01 | 6-[4-[2-[2-(3-Ethoxyphenyl)ethynyl]benzoyl]piperazin-1-yl]-N-methylsulfonylpyridazine-3-carboxamide |
| B2I008-01 | 6-[4-[2-[2-(5-Ethoxypyridin-3-yl)ethynyl]benzoyl]piperazin-1-yl]-N-methylsulfonylpyridazine-3-carboxamide |
| B2I009-01 | 6-[4-[5-[2-(3-Ethoxyphenyl)ethynyl]pyridine-3-carbonyl]piperazin-1-yl]-N-methylsulfonylpyridazine-3-carboxamide |
| B2I010-01 | 6-[4-[5-[2-(5-Ethoxypyridin-3-yl)ethynyl]pyridine-3-carbonyl]piperazin-1-yl]-N-methylsulfonylpyridazine-3-carboxamide |
| B2I011-01 | 6-[4-[3-[2-(3-Ethoxyphenyl)phenyl]propanoyl]piperazin-1-yl]-N-methylsulfonylpyridazine-3-carboxamide |
| B2I012-01 | 6-[4-[3-[2-(5-Ethoxypyridin-3-yl)phenyl]propanoyl]piperazin-1-yl]-N-methylsulfonylpyridazine-3-carboxamide |
| B2I013-01 | 6-[4-[4-(3-Ethoxyphenyl)naphthalene-1-carbonyl]piperazin-1-yl]-N-methylsulfonylpyridazine-3-carboxamide |
| B2I014-01 | 6-[4-[4-(5-Ethoxypyridin-3-yl)naphthalene-1-carbonyl]piperazin-1-yl]-N-methylsulfonylpyridazine-3-carboxamide |
| B2I015-01 | 6-[4-[3-tert-Butyl-5-(3-ethoxyphenyl)benzoyl]piperazin-1-yl]-N-methylsulfonylpyridazine-3-carboxamide |
| B2I016-01 | 6-[4-[3-tert-Butyl-5-(5-ethoxypyridin-3-yl)benzoyl]piperazin-1-yl]-N-methylsulfonylpyridazine-3-carboxamide |
| B2I017-01 | 6-[4-[3-(3-Ethoxyphenyl)-5-(trifluoromethyl)benzoyl]piperazin-1-yl]-N-methylsulfonylpyridazine-3-carboxamide |
| B2I018-01 | 6-[4-[3-(5-Ethoxypyridin-3-yl)-5-(trifluoromethyl)benzoyl]piperazin-1-yl]-N-methylsulfonylpyridazine-3-carboxamide |
| B2I019-01 | 6-[4-[3-[2-(3-Ethoxyphenyl)ethynyl]-5-(trifluoromethyl)benzoyl]piperazin-1-yl]-N-methylsulfonylpyridazine-3-carboxamide |

| | |
|---|---|
| B2I020-01 | 6-[4-[3-[2-(5-Ethoxypyridin-3-yl)ethynyl]-5-(trifluoromethyl)benzoyl]piperazin-1-yl]-N-methylsulfonylpyridazine-3-carboxamide |
| B2I021-01 | 6-[4-[3-[4-(3-Ethoxyphenyl)pyrazol-1-yl]-5-(trifluoromethyl)benzoyl]piperazin-1-yl]-N-methylsulfonylpyridazine-3-carboxamide |
| B2I022-01 | 6-[4-[3-[4-(5-Ethoxypyridin-3-yl)pyrazol-1-yl]-5-(trifluoromethyl)benzoyl]piperazin-1-yl]-N-methylsulfonylpyridazine-3-carboxamide |
| B2I023-01 | 4-[4-[4-(3-Ethoxyphenyl)thiophene-2-carbonyl]piperazin-1-yl]-N-methylsulfonylbenzamide |
| B2I024-01 | 4-[4-[4-(5-Ethoxypyridin-3-yl)thiophene-2-carbonyl]piperazin-1-yl]-N-methylsulfonylbenzamide |
| B2I025-01 | 4-[4-[4-(3-Ethoxyphenyl)-3-methylbenzoyl]piperazin-1-yl]-N-methylsulfonylbenzamide |
| B2I026-01 | 4-[4-[4-(5-Ethoxypyridin-3-yl)-3-methylbenzoyl]piperazin-1-yl]-N-methylsulfonylbenzamide |
| B2I027-01 | 4-[4-[4-(3-Ethoxyphenyl)-3-fluorobenzoyl]piperazin-1-yl]-N-methylsulfonylbenzamide |
| B2I028-01 | 4-[4-[4-(5-Ethoxypyridin-3-yl)-3-fluorobenzoyl]piperazin-1-yl]-N-methylsulfonylbenzamide |
| B2I029-01 | 4-[4-[2-[2-(3-Ethoxyphenyl)ethynyl]benzoyl]piperazin-1-yl]-N-methylsulfonylbenzamide |
| B2I030-01 | 4-[4-[2-[2-(5-Ethoxypyridin-3-yl)ethynyl]benzoyl]piperazin-1-yl]-N-methylsulfonylbenzamide |
| B2I031-01 | 4-[4-[5-[2-(3-Ethoxyphenyl)ethynyl]pyridine-3-carbonyl]piperazin-1-yl]-N-methylsulfonylbenzamide |
| B2I032-01 | 4-[4-[5-[2-(5-Ethoxypyridin-3-yl)ethynyl]pyridine-3-carbonyl]piperazin-1-yl]-N-methylsulfonylbenzamide |
| B2I033-01 | 4-[4-[3-[2-(3-Ethoxyphenyl)phenyl]propanoyl]piperazin-1-yl]-N-methylsulfonylbenzamide |
| B2I034-01 | 4-[4-[3-[2-(5-Ethoxypyridin-3-yl)phenyl]propanoyl]piperazin-1-yl]-N-methylsulfonylbenzamide |
| B2I035-01 | 4-[4-[4-(3-Ethoxyphenyl)naphthalene-1-carbonyl]piperazin-1-yl]-N-methylsulfonylbenzamide |
| B2I036-01 | 4-[4-[4-(5-Ethoxypyridin-3-yl)naphthalene-1-carbonyl]piperazin-1-yl]-N-methylsulfonylbenzamide |
| B2I037-01 | 4-[4-[3-tert-Butyl-5-(3-ethoxyphenyl)benzoyl]piperazin-1-yl]-N-methylsulfonylbenzamide |
| B2I038-01 | 4-[4-[3-tert-Butyl-5-(5-ethoxypyridin-3-yl)benzoyl]piperazin-1-yl]-N-methylsulfonylbenzamide |
| B2I039-01 | 4-[4-[3-(3-Ethoxyphenyl)-5-(trifluoromethyl)benzoyl]piperazin-1-yl]-N-methylsulfonylbenzamide |
| B2I040-01 | 4-[4-[3-(5-Ethoxypyridin-3-yl)-5-(trifluoromethyl)benzoyl]piperazin-1-yl]-N-methylsulfonylbenzamide |
| B2I041-01 | 4-[4-[3-[2-(3-Ethoxyphenyl)ethynyl]-5-(trifluoromethyl)benzoyl]piperazin-1-yl]-N-methylsulfonylbenzamide |
| B2I042-01 | 4-[4-[3-[2-(5-Ethoxypyridin-3-yl)ethynyl]-5-(trifluoromethyl)benzoyl]piperazin-1-yl]-N-methylsulfonylbenzamide |
| B2I043-01 | 4-[4-[3-[4-(3-Ethoxyphenyl)pyrazol-1-yl]-5-(trifluoromethyl)benzoyl]piperazin-1-yl]-N-methylsulfonylbenzamide |
| B2I044-01 | 4-[4-[3-[4-(5-Ethoxypyridin-3-yl)pyrazol-1-yl]-5-(trifluoromethyl)benzoyl]piperazin-1-yl]-N-methylsulfonylbenzamide |
| B2I045-01 | 4-[4-[[2-(3-Ethoxyphenyl)phenyl]methyl]piperazin-1-yl]-N-methylsulfonylbenzamide |
| B2I046-01 | 4-[4-[[2-(5-Ethoxypyridin-3-yl)phenyl]methyl]piperazin-1-yl]-N-methylsulfonylbenzamide |
| B2I047-01 | 4-[4-[[4-(3-Ethoxyphenyl)-3-methylphenyl]methyl]piperazin-1-yl]-N-methylsulfonylbenzamide |

| | |
|---|---|
| B2I048-01 | 4-[4-[[4-(5-Ethoxypyridin-3-yl)-3-methylphenyl]methyl]piperazin-1-yl]-N-methylsulfonylbenzamide |
| B2I049-01 | 4-[4-[[4-(3-Ethoxyphenyl)-2-ethylphenyl]methyl]piperazin-1-yl]-N-methylsulfonylbenzamide |
| B2I050-01 | 4-[4-[[4-(5-Ethoxypyridin-3-yl)-2-ethylphenyl]methyl]piperazin-1-yl]-N-methylsulfonylbenzamide |
| B2I051-01 | 4-[4-[[4-(3-Ethoxyphenyl)-3-fluorophenyl]methyl]piperazin-1-yl]-N-methylsulfonylbenzamide |
| B2I052-01 | 4-[4-[[4-(5-Ethoxypyridin-3-yl)-3-fluorophenyl]methyl]piperazin-1-yl]-N-methylsulfonylbenzamide |
| B2I053-01 | 4-[4-[[4-(3-Ethoxyphenyl)-2-methoxyphenyl]methyl]piperazin-1-yl]-N-methylsulfonylbenzamide |
| B2I054-01 | 4-[4-[[4-(5-Ethoxypyridin-3-yl)-2-methoxyphenyl]methyl]piperazin-1-yl]-N-methylsulfonylbenzamide |
| B2I055-01 | 4-[4-[[3-Ethoxy-5-(3-ethoxyphenyl)phenyl]methyl]piperazin-1-yl]-N-methylsulfonylbenzamide |
| B2I056-01 | 4-[4-[[3-Ethoxy-5-(5-ethoxypyridin-3-yl)phenyl]methyl]piperazin-1-yl]-N-methylsulfonylbenzamide |
| B2I057-01 | 4-[4-[[3-(3-Ethoxyphenyl)-5-propan-2-yloxyphenyl]methyl]piperazin-1-yl]-N-methylsulfonylbenzamide |
| B2I058-01 | 4-[4-[[3-(5-Ethoxypyridin-3-yl)-5-propan-2-yloxyphenyl]methyl]piperazin-1-yl]-N-methylsulfonylbenzamide |
| B2I059-01 | 4-[4-[[4-(3-Ethoxyphenyl)-2,6-dimethoxyphenyl]methyl]piperazin-1-yl]-N-methylsulfonylbenzamide |
| B2I060-01 | 4-[4-[[4-(5-Ethoxypyridin-3-yl)-2,6-dimethoxyphenyl]methyl]piperazin-1-yl]-N-methylsulfonylbenzamide |
| B2I061-01 | 4-[4-[[3-(3-Ethoxyphenyl)-5-(trifluoromethyl)phenyl]methyl]piperazin-1-yl]-N-methylsulfonylbenzamide |
| B2I062-01 | 4-[4-[[3-(5-Ethoxypyridin-3-yl)-5-(trifluoromethyl)phenyl]methyl]piperazin-1-yl]-N-methylsulfonylbenzamide |
| B2I063-01 | 4-[4-[[3-(3-Ethoxyphenyl)-5-(trifluoromethoxy)phenyl]methyl]piperazin-1-yl]-N-methylsulfonylbenzamide |
| B2I064-01 | 4-[4-[[3-(5-Ethoxypyridin-3-yl)-5-(trifluoromethoxy)phenyl]methyl]piperazin-1-yl]-N-methylsulfonylbenzamide |
| B2I065-01 | 4-[4-[[4-(3-Ethoxyphenyl)naphthalen-1-yl]methyl]piperazin-1-yl]-N-methylsulfonylbenzamide |
| B2I066-01 | 4-[4-[[4-(5-Ethoxypyridin-3-yl)naphthalen-1-yl]methyl]piperazin-1-yl]-N-methylsulfonylbenzamide |
| B2I067-01 | 4-[4-[[4-(3-Ethoxyphenyl)thiophen-2-yl]methyl]piperazin-1-yl]-N-methylsulfonylbenzamide |
| B2I068-01 | 4-[4-[[4-(5-Ethoxypyridin-3-yl)thiophen-2-yl]methyl]piperazin-1-yl]-N-methylsulfonylbenzamide |
| B2I069-01 | 4-[4-[[2-[2-(3-Ethoxyphenyl)ethynyl]phenyl]methyl]piperazin-1-yl]-N-methylsulfonylbenzamide |
| B2I070-01 | 4-[4-[[2-[2-(5-Ethoxypyridin-3-yl)ethynyl]phenyl]methyl]piperazin-1-yl]-N-methylsulfonylbenzamide |
| B2I071-01 | 6-[4-[[2-(3-Ethoxyphenyl)phenyl]methyl]piperazin-1-yl]-N-methylsulfonylpyridazine-3-carboxamide |
| B2I072-01 | 6-[4-[[2-(5-Ethoxypyridin-3-yl)phenyl]methyl]piperazin-1-yl]-N-methylsulfonylpyridazine-3-carboxamide |
| B2I073-01 | 6-[4-[[4-(3-Ethoxyphenyl)-3-methylphenyl]methyl]piperazin-1-yl]-N-methylsulfonylpyridazine-3-carboxamide |
| B2I074-01 | 6-[4-[[4-(5-Ethoxypyridin-3-yl)-3-methylphenyl]methyl]piperazin-1-yl]-N-methylsulfonylpyridazine-3-carboxamide |
| B2I075-01 | 6-[4-[[4-(3-Ethoxyphenyl)-2-ethylphenyl]methyl]piperazin-1-yl]-N-methylsulfonylpyridazine-3-carboxamide |

| | |
|---|---|
| B2I076-01 | 6-[4-[[4-(5-Ethoxypyridin-3-yl)-2-ethylphenyl]methyl]piperazin-1-yl]-N-methylsulfonylpyridazine-3-carboxamide |
| B2I077-01 | 6-[4-[[4-(3-Ethoxyphenyl)-3-fluorophenyl]methyl]piperazin-1-yl]-N-methylsulfonylpyridazine-3-carboxamide |
| B2I078-01 | 6-[4-[[4-(5-Ethoxypyridin-3-yl)-3-fluorophenyl]methyl]piperazin-1-yl]-N-methylsulfonylpyridazine-3-carboxamide |
| B2I079-01 | 6-[4-[[4-(3-Ethoxyphenyl)-2-methoxyphenyl]methyl]piperazin-1-yl]-N-methylsulfonylpyridazine-3-carboxamide |
| B2I080-01 | 6-[4-[[4-(5-Ethoxypyridin-3-yl)-2-methoxyphenyl]methyl]piperazin-1-yl]-N-methylsulfonylpyridazine-3-carboxamide |
| B2I081-01 | 6-[4-[[3-Ethoxy-5-(3-ethoxyphenyl)phenyl]methyl]piperazin-1-yl]-N-methylsulfonylpyridazine-3-carboxamide |
| B2I082-01 | 6-[4-[[3-Ethoxy-5-(5-ethoxypyridin-3-yl)phenyl]methyl]piperazin-1-yl]-N-methylsulfonylpyridazine-3-carboxamide |
| B2I083-01 | 6-[4-[[3-(3-Ethoxyphenyl)-5-propan-2-yloxyphenyl]methyl]piperazin-1-yl]-N-methylsulfonylpyridazine-3-carboxamide |
| B2I084-01 | 6-[4-[[3-(5-Ethoxypyridin-3-yl)-5-propan-2-yloxyphenyl]methyl]piperazin-1-yl]-N-methylsulfonylpyridazine-3-carboxamide |
| B2I085-01 | 6-[4-[[4-(3-Ethoxyphenyl)-2,6-dimethoxyphenyl]methyl]piperazin-1-yl]-N-methylsulfonylpyridazine-3-carboxamide |
| B2I086-01 | 6-[4-[[4-(5-Ethoxypyridin-3-yl)-2,6-dimethoxyphenyl]methyl]piperazin-1-yl]-N-methylsulfonylpyridazine-3-carboxamide |
| B2I087-01 | 6-[4-[[3-(3-Ethoxyphenyl)-5-(trifluoromethyl)phenyl]methyl]piperazin-1-yl]-N-methylsulfonylpyridazine-3-carboxamide |
| B2I088-01 | 6-[4-[[3-(5-Ethoxypyridin-3-yl)-5-(trifluoromethyl)phenyl]methyl]piperazin-1-yl]-N-methylsulfonylpyridazine-3-carboxamide |
| B2I089-01 | 6-[4-[[3-(3-Ethoxyphenyl)-5-(trifluoromethoxy)phenyl]methyl]piperazin-1-yl]-N-methylsulfonylpyridazine-3-carboxamide |
| B2I090-01 | 6-[4-[[3-(5-Ethoxypyridin-3-yl)-5-(trifluoromethoxy)phenyl]methyl]piperazin-1-yl]-N-methylsulfonylpyridazine-3-carboxamide |
| B2I091-01 | 6-[4-[[4-(3-Ethoxyphenyl)naphthalen-1-yl]methyl]piperazin-1-yl]-N-methylsulfonylpyridazine-3-carboxamide |
| B2I092-01 | 6-[4-[[4-(5-Ethoxypyridin-3-yl)naphthalen-1-yl]methyl]piperazin-1-yl]-N-methylsulfonylpyridazine-3-carboxamide |
| B2I093-01 | 6-[4-[[4-(3-Ethoxyphenyl)thiophen-2-yl]methyl]piperazin-1-yl]-N-methylsulfonylpyridazine-3-carboxamide |
| B2I094-01 | 6-[4-[[4-(5-Ethoxypyridin-3-yl)thiophen-2-yl]methyl]piperazin-1-yl]-N-methylsulfonylpyridazine-3-carboxamide |
| B2I095-01 | 6-[4-[[2-[2-(3-Ethoxyphenyl)ethynyl]phenyl]methyl]piperazin-1-yl]-N-methylsulfonylpyridazine-3-carboxamide |
| B2I096-01 | 6-[4-[[2-[2-(5-Ethoxypyridin-3-yl)ethynyl]phenyl]methyl]piperazin-1-yl]-N-methylsulfonylpyridazine-3-carboxamide |
| B2I097-01 | 4-[4-[1-[4-(3-Ethoxyphenyl)phenyl]ethyl]piperazin-1-yl]-N-methylsulfonylbenzamide |
| B2I098-01 | 4-[4-[1-[4-(5-Ethoxypyridin-3-yl)phenyl]ethyl]piperazin-1-yl]-N-methylsulfonylbenzamide |
| B2I099-01 | 4-[4-[1-[4-(3-Ethoxyphenyl)-2-fluorophenyl]ethyl]piperazin-1-yl]-N-methylsulfonylbenzamide |
| B2I100-01 | 4-[4-[1-[4-(5-Ethoxypyridin-3-yl)-2-fluorophenyl]ethyl]piperazin-1-yl]-N-methylsulfonylbenzamide |

[Table 255]

[Table AI-03-B2I-01]

| Label | Exact | ⊠ | A | a | B | b | C | c | D |
|---|---|---|---|---|---|---|---|---|---|
| B2I001-01 | 515.13 | OEt | A01 | a1 | B10 | b3 | C02 | c2 | D07 |

| | | | | | | | | | |
|---|---|---|---|---|---|---|---|---|---|
| B2I002-01 | 516.13 | OEt | A02 | a1 | B10 | b3 | C02 | c2 | D07 |
| B2I003-01 | 523.19 | OEt | A01 | a1 | B04 | b3 | C02 | c2 | D07 |
| B2I004-01 | 524.18 | OEt | A02 | a1 | B04 | b3 | C02 | c2 | D07 |
| B2I005-01 | 527.16 | OEt | A01 | a1 | B06 | b3 | C02 | c2 | D07 |
| B2I006-01 | 528.16 | OEt | A02 | a1 | B06 | b3 | C02 | c2 | D07 |
| B2I007-01 | 533.17 | OEt | A01 | a2 | B01 | b3 | C02 | c2 | D07 |
| B2I008-01 | 534.17 | OEt | A02 | a2 | B01 | b3 | C02 | c2 | D07 |
| B2I009-01 | 534.17 | OEt | A01 | a2 | B17 | b3 | C02 | c2 | D07 |
| B2I010-01 | 535.16 | OEt | A02 | a2 | B17 | b3 | C02 | c2 | D07 |
| B2I011-01 | 537.21 | OEt | A01 | a1 | B02 | b3 | C02 | c2 | D07 |
| B2I012-01 | 538.2 | OEt | A02 | a1 | B02 | b3 | C02 | c2 | D07 |
| B2I013-01 | 559.19 | OEt | A01 | a1 | B09 | b3 | C02 | c2 | D07 |
| B2I014-01 | 560.18 | OEt | A02 | a1 | B09 | b3 | C02 | c2 | D07 |
| B2I015-01 | 565.24 | OEt | A01 | a1 | B16 | b3 | C02 | c2 | D07 |
| B2I016-01 | 566.23 | OEt | A02 | a1 | B16 | b3 | C02 | c2 | D07 |
| B2I017-01 | 577.16 | OEt | A01 | a1 | B11 | b3 | C02 | c2 | D07 |
| B2I018-01 | 578.16 | OEt | A02 | a1 | B11 | b3 | C02 | c2 | D07 |
| B2I019-01 | 601.16 | OEt | A01 | a2 | B11 | b3 | C02 | c2 | D07 |
| B2I020-01 | 602.16 | OEt | A02 | a2 | B11 | b3 | C02 | c2 | D07 |
| B2I021-01 | 643.18 | OEt | A01 | a1 | B12 | b3 | C02 | c2 | D07 |
| B2I022-01 | 644.18 | OEt | A02 | a1 | B12 | b3 | C02 | c2 | D07 |
| B2I023-01 | 513.14 | OEt | A01 | a1 | B10 | b3 | C01 | c2 | D07 |
| B2I024-01 | 514.13 | OEt | A02 | a1 | B10 | b3 | C01 | c2 | D07 |
| B2I025-01 | 521.2 | OEt | A01 | a1 | B04 | b3 | C01 | c2 | D07 |
| B2I026-01 | 522.19 | OEt | A02 | a1 | B04 | b3 | C01 | c2 | D07 |
| B2I027-01 | 525.17 | OEt | A01 | a1 | B06 | b3 | C01 | c2 | D07 |
| B2I028-01 | 526.17 | OEt | A02 | a1 | B06 | b3 | C01 | c2 | D07 |
| B2I029-01 | 531.18 | OEt | A01 | a2 | B01 | b3 | C01 | c2 | D07 |
| B2I030-01 | 532.18 | OEt | A02 | a2 | B01 | b3 | C01 | c2 | D07 |
| B2I031-01 | 532.18 | OEt | A01 | a2 | B17 | b3 | C01 | c2 | D07 |
| B2I032-01 | 533.17 | OEt | A02 | a2 | B17 | b3 | C01 | c2 | D07 |
| B2I033-01 | 535.21 | OEt | A01 | a1 | B02 | b3 | C01 | c2 | D07 |
| B2I034-01 | 536.21 | OEt | A02 | a1 | B02 | b3 | C01 | c2 | D07 |
| B2I035-01 | 557.2 | OEt | A01 | a1 | B09 | b3 | C01 | c2 | D07 |
| B2I036-01 | 558.19 | OEt | A02 | a1 | B09 | b3 | C01 | c2 | D07 |
| B2I037-01 | 563.25 | OEt | A01 | a1 | B16 | b3 | C01 | c2 | D07 |
| B2I038-01 | 564.24 | OEt | A02 | a1 | B16 | b3 | C01 | c2 | D07 |
| B2I039-01 | 575.17 | OEt | A01 | a1 | B11 | b3 | C01 | c2 | D07 |
| B2I040-01 | 576.17 | OEt | A02 | a1 | B11 | b3 | C01 | c2 | D07 |
| B2I041-01 | 599.17 | OEt | A01 | a2 | B11 | b3 | C01 | c2 | D07 |
| B2I042-01 | 600.17 | OEt | A02 | a2 | B11 | b3 | C01 | c2 | D07 |
| B2I043-01 | 641.19 | OEt | A01 | a1 | B12 | b3 | C01 | c2 | D07 |
| B2I044-01 | 642.19 | OEt | A02 | a1 | B12 | b3 | C01 | c2 | D07 |
| B2I045-01 | 493.2 | OEt | A01 | a1 | B01 | b1 | C01 | c2 | D07 |
| B2I046-01 | 494.2 | OEt | A02 | a1 | B01 | b1 | C01 | c2 | D07 |
| B2I047-01 | 507.22 | OEt | A01 | a1 | B04 | b1 | C01 | c2 | D07 |
| B2I048-01 | 508.21 | OEt | A02 | a1 | B04 | b1 | C01 | c2 | D07 |

| | | | | | | | | | |
|---|---|---|---|---|---|---|---|---|---|
| B2I049-01 | 521.24 | OEt | A01 | a1 | B05 | b1 | C01 | c2 | D07 |
| B2I050-01 | 522.23 | OEt | A02 | a1 | B05 | b1 | C01 | c2 | D07 |
| B2I051-01 | 511.19 | OEt | A01 | a1 | B06 | b1 | C01 | c2 | D07 |
| B2I052-01 | 512.19 | OEt | A02 | a1 | B06 | b1 | C01 | c2 | D07 |
| B2I053-01 | 523.21 | OEt | A01 | a1 | B07 | b1 | C01 | c2 | D07 |
| B2I054-01 | 524.21 | OEt | A02 | a1 | B07 | b1 | C01 | c2 | D07 |
| B2I055-01 | 537.23 | OEt | A01 | a1 | B14 | b1 | C01 | c2 | D07 |
| B2I056-01 | 538.23 | OEt | A02 | a1 | B14 | b1 | C01 | c2 | D07 |
| B2I057-01 | 551.25 | OEt | A01 | a1 | B15 | b1 | C01 | c2 | D07 |
| B2I058-01 | 552.24 | OEt | A02 | a1 | B15 | b1 | C01 | c2 | D07 |
| B2I059-01 | 553.23 | OEt | A01 | a1 | B08 | b1 | C01 | c2 | D07 |
| B2I060-01 | 554.22 | OEt | A02 | a1 | B08 | b1 | C01 | c2 | D07 |
| B2I061-01 | 561.19 | OEt | A01 | a1 | B11 | b1 | C01 | c2 | D07 |
| B2I062-01 | 562.19 | OEt | A02 | a1 | B11 | b1 | C01 | c2 | D07 |
| B2I063-01 | 577.19 | OEt | A01 | a1 | B13 | b1 | C01 | c2 | D07 |
| B2I064-01 | 578.18 | OEt | A02 | a1 | B13 | b1 | C01 | c2 | D07 |
| B2I065-01 | 543.22 | OEt | A01 | a1 | B09 | b1 | C01 | c2 | D07 |
| B2I066-01 | 544.21 | OEt | A02 | a1 | B09 | b1 | C01 | c2 | D07 |
| B2I067-01 | 499.16 | OEt | A01 | a1 | B10 | b1 | C01 | c2 | D07 |
| B2I068-01 | 500.16 | OEt | A02 | a1 | B10 | b1 | C01 | c2 | D07 |
| B2I069-01 | 517.2 | OEt | A01 | a2 | B01 | b1 | C01 | c2 | D07 |
| B2I070-01 | 518.2 | OEt | A02 | a2 | B01 | b1 | C01 | c2 | D07 |
| B2I071-01 | 495.19 | OEt | A01 | a1 | B01 | b1 | C02 | c2 | D07 |
| B2I072-01 | 496.19 | OEt | A02 | a1 | B01 | b1 | C02 | c2 | D07 |
| B2I073-01 | 509.21 | OEt | A01 | a1 | B04 | b1 | C02 | c2 | D07 |
| B2I074-01 | 510.21 | OEt | A02 | a1 | B04 | b1 | C02 | c2 | D07 |
| B2I075-01 | 523.23 | OEt | A01 | a1 | B05 | b1 | C02 | c2 | D07 |
| B2I076-01 | 524.22 | OEt | A02 | a1 | B05 | b1 | C02 | c2 | D07 |
| B2I077-01 | 513.19 | OEt | A01 | a1 | B06 | b1 | C02 | c2 | D07 |
| B2I078-01 | 514.18 | OEt | A02 | a1 | B06 | b1 | C02 | c2 | D07 |
| B2I079-01 | 525.21 | OEt | A01 | a1 | B07 | b1 | C02 | c2 | D07 |
| B2I080-01 | 526.2 | OEt | A02 | a1 | B07 | b1 | C02 | c2 | D07 |
| B2I081-01 | 539.22 | OEt | A01 | a1 | B14 | b1 | C02 | c2 | D07 |
| B2I082-01 | 540.22 | OEt | A02 | a1 | B14 | b1 | C02 | c2 | D07 |
| B2I083-01 | 553.24 | OEt | A01 | a1 | B15 | b1 | C02 | c2 | D07 |
| B2I084-01 | 554.23 | OEt | A02 | a1 | B15 | b1 | C02 | c2 | D07 |
| B2I085-01 | 555.22 | OEt | A01 | a1 | B08 | b1 | C02 | c2 | D07 |
| B2I086-01 | 556.21 | OEt | A02 | a1 | B08 | b1 | C02 | c2 | D07 |
| B2I087-01 | 563.18 | OEt | A01 | a1 | B11 | b1 | C02 | c2 | D07 |
| B2I088-01 | 564.18 | OEt | A02 | a1 | B11 | b1 | C02 | c2 | D07 |
| B2I089-01 | 579.18 | OEt | A01 | a1 | B13 | b1 | C02 | c2 | D07 |
| B2I090-01 | 580.17 | OEt | A02 | a1 | B13 | b1 | C02 | c2 | D07 |
| B2I091-01 | 545.21 | OEt | A01 | a1 | B09 | b1 | C02 | c2 | D07 |
| B2I092-01 | 546.21 | OEt | A02 | a1 | B09 | b1 | C02 | c2 | D07 |
| B2I093-01 | 501.15 | OEt | A01 | a1 | B10 | b1 | C02 | c2 | D07 |
| B2I094-01 | 502.15 | OEt | A02 | a1 | B10 | b1 | C02 | c2 | D07 |
| B2I095-01 | 519.19 | OEt | A01 | a2 | B01 | b1 | C02 | c2 | D07 |

| | | | | | | | | | |
|---|---|---|---|---|---|---|---|---|---|
| B2I096-01 | 520.19 | OEt | A02 | a2 | B01 | b1 | C02 | c2 | D07 |
| B2I097-01 | 507.22 | OEt | A01 | a1 | B03 | b2 | C01 | c2 | D07 |
| B2I098-01 | 508.21 | OEt | A02 | a1 | B03 | b2 | C01 | c2 | D07 |
| B2I099-01 | 525.21 | OEt | A01 | a1 | B18 | b2 | C01 | c2 | D07 |
| B2I100-01 | 526.21 | OEt | A02 | a1 | B18 | b2 | C01 | c2 | D07 |

[Table 256]

[Table AI-03-B2L-01]

| Mixture No. | mixAI-B2L-01 |
|---|---|
| No. of compound that may be contained | Compound name |
| B2L001-01 | N-tert-Butylsulfonyl-6-[4-[4-(3-ethoxyphenyl)thiophene-2-carbonyl]piperazin-1-yl]pyridazine-3-carboxamide |
| B2L002-01 | N-tert-Butylsulfonyl-6-[4-[4-(5-ethoxypyridin-3-yl)thiophene-2-carbonyl]piperazin-1-yl]pyridazine-3-carboxamide |
| B2L003-01 | N-tert-Butylsulfonyl-6-[4-[4-(3-ethoxyphenyl)-3-methylbenzoyl]piperazin-1-yl]pyridazine-3-carboxamide |
| B2L004-01 | N-tert-Butylsulfonyl-6-[4-[4-(5-ethoxypyridin-3-yl)-3-methylbenzoyl]piperazin-1-yl]pyridazine-3-carboxamide |
| B2L005-01 | N-tert-Butylsulfonyl-6-[4-[4-(3-ethoxyphenyl)-3-fluorobenzoyl]piperazin-1-yl]pyridazine-3-carboxamide |
| B2L006-01 | N-tert-Butylsulfonyl-6-[4-[4-(5-ethoxypyridin-3-yl)-3-fluorobenzoyl]piperazin-1-yl]pyridazine-3-carboxamide |
| B2L007-01 | N-tert-Butylsulfonyl-6-[4-[2-[2-(3-ethoxyphenyl)ethynyl]benzoyl]piperazin-1-yl]pyridazine-3-carboxamide |
| B2L008-01 | N-tert-Butylsulfonyl-6-[4-[2-[2-(5-ethoxypyridin-3-yl)ethynyl]benzoyl]piperazin-1-yl]pyridazine-3-carboxamide |
| B2L009-01 | N-tert-Butylsulfonyl-6-[4-[5-[2-(3-ethoxyphenyl)ethynyl]pyridine-3-carbonyl]piperazin-1-yl]pyridazine-3-carboxamide |
| B2L010-01 | N-tert-Butylsulfonyl-6-[4-[5-[2-(5-ethoxypyridin-3-yl)ethynyl]pyridine-3-carbonyl]piperazin-1-yl]pyridazine-3-carboxamide |
| B2L011-01 | N-tert-Butylsulfonyl-6-[4-[3-[2-(3-ethoxyphenyl)phenyl]propanoyl]piperazin-1-yl]pyridazine-3-carboxamide |
| B2L012-01 | N-tert-Butylsulfonyl-6-[4-[3-[2-(5-ethoxypyridin-3-yl)phenyl]propanoyl]piperazin-1-yl]pyridazine-3-carboxamide |
| B2L013-01 | N-tert-Butylsulfonyl-6-[4-[4-(3-ethoxyphenyl)naphthalene-1-carbonyl]piperazin-1-yl]pyridazine-3-carboxamide |
| B2L014-01 | N-tert-Butylsulfonyl-6-[4-[4-(5-ethoxypyridin-3-yl)naphthalene-1-carbonyl]piperazin-1-yl]pyridazine-3-carboxamide |
| B2L015-01 | 6-[4-[3-tert-Butyl-5-(3-ethoxyphenyl)benzoyl]piperazin-1-yl]-N-tert-butylsulfonylpyridazine-3-carboxamide |
| B2L016-01 | 6-[4-[3-tert-Butyl-5-(5-ethoxypyridin-3-yl)benzoyl]piperazin-1-yl]-N-tert-butylsulfonylpyridazine-3-carboxamide |
| B2L017-01 | N-tert-Butylsulfonyl-6-[4-[3-(3-ethoxyphenyl)-5-(trifluoromethyl)benzoyl]piperazin-1-yl]pyridazine-3-carboxamide |
| B2L018-01 | N-tert-Butylsulfonyl-6-[4-[3-(5-ethoxypyridin-3-yl)-5-(trifluoromethyl)benzoyl]piperazin-1-yl]pyridazine-3-carboxamide |
| B2L019-01 | N-tert-Butylsulfonyl-6-[4-[3-[2-(3-ethoxyphenyl)ethynyl]-5-(trifluoromethyl)benzoyl]piperazin-1-yl]pyridazine-3-carboxamide |
| B2L020-01 | N-tert-Butylsulfonyl-6-[4-[3-[2-(5-ethoxypyridin-3-yl)ethynyl]-5-(trifluoromethyl)benzoyl]piperazin-1-yl]pyridazine-3-carboxamide |
| B2L021-01 | N-tert-Butylsulfonyl-6-[4-[3-[4-(3-ethoxyphenyl)pyrazol-1-yl]-5-(trifluoromethyl)benzoyl]piperazin-1-yl]pyridazine-3-carboxamide |

| | |
|---|---|
| B2L022-01 | N-tert-Butylsulfonyl-6-[4-[3-[4-(5-ethoxypyridin-3-yl)pyrazol-1-yl]-5-(trifluoromethyl)benzoyl]piperazin-1-yl]pyridazine-3-carboxamide |
| B2L023-01 | N-tert-Butylsulfonyl-4-[4-[4-(3-ethoxyphenyl)thiophene-2-carbonyl]piperazin-1-yl]benzamide |
| B2L024-01 | N-tert-Butylsulfonyl-4-[4-[4-(5-ethoxypyridin-3-yl)thiophene-2-carbonyl]piperazin-1-yl]benzamide |
| B2L025-01 | N-tert-Butylsulfonyl-4-[4-[4-(3-ethoxyphenyl)-3-methylbenzoyl]piperazin-1-yl]benzamide |
| B2L026-01 | N-tert-Butylsulfonyl-4-[4-[4-(5-ethoxypyridin-3-yl)-3-methylbenzoyl]piperazin-1-yl]benzamide |
| B2L027-01 | N-tert-Butylsulfonyl-4-[4-[4-(3-ethoxyphenyl)-3-fluorobenzoyl]piperazin-1-yl]benzamide |
| B2L028-01 | N-tert-Butylsulfonyl-4-[4-[4-(5-ethoxypyridin-3-yl)-3-fluorobenzoyl]piperazin-1-yl]benzamide |
| B2L029-01 | N-tert-Butylsulfonyl-4-[4-[2-[2-(3-ethoxyphenyl)ethynyl]benzoyl]piperazin-1-yl]benzamide |
| B2L030-01 | N-tert-Butylsulfonyl-4-[4-[2-[2-(5-ethoxypyridin-3-yl)ethynyl]benzoyl]piperazin-1-yl]benzamide |
| B2L031-01 | N-tert-Butylsulfonyl-4-[4-[5-[2-(3-ethoxyphenyl)ethynyl]pyridine-3-carbonyl]piperazin-1-yl]benzamide |
| B2L032-01 | N-tert-Butylsulfonyl-4-[4-[5-[2-(5-ethoxypyridin-3-yl)ethynyl]pyridine-3-carbonyl]piperazin-1-yl]benzamide |
| B2L033-01 | N-tert-Butylsulfonyl-4-[4-[3-[2-(3-ethoxyphenyl)phenyl]propanoyl]piperazin-1-yl]benzamide |
| B2L034-01 | N-tert-Butylsulfonyl-4-[4-[3-[2-(5-ethoxypyridin-3-yl)phenyl]propanoyl]piperazin-1-yl]benzamide |
| B2L035-01 | N-tert-Butylsulfonyl-4-[4-[4-(3-ethoxyphenyl)naphthalene-1-carbonyl]piperazin-1-yl]benzamide |
| B2L036-01 | N-tert-Butylsulfonyl-4-[4-[4-(5-ethoxypyridin-3-yl)naphthalene-1-carbonyl]piperazin-1-yl]benzamide |
| B2L037-01 | 4-[4-[3-tert-Butyl-5-(3-ethoxyphenyl)benzoyl]piperazin-1-yl]-N-tert-butylsulfonylbenzamide |
| B2L038-01 | 4-[4-[3-tert-Butyl-5-(5-ethoxypyridin-3-yl)benzoyl]piperazin-1-yl]-N-tert-butylsulfonylbenzamide |
| B2L039-01 | N-tert-Butylsulfonyl-4-[4-[3-(3-ethoxyphenyl)-5-(trifluoromethyl)benzoyl]piperazin-1-yl]benzamide |
| B2L040-01 | N-tert-Butylsulfonyl-4-[4-[3-(5-ethoxypyridin-3-yl)-5-(trifluoromethyl)benzoyl]piperazin-1-yl]benzamide |
| B2L041-01 | N-tert-Butylsulfonyl-4-[4-[3-[2-(3-ethoxyphenyl)ethynyl]-5-(trifluoromethyl)benzoyl]piperazin-1-yl]benzamide |
| B2L042-01 | N-tert-Butylsulfonyl-4-[4-[3-[2-(5-ethoxypyridin-3-yl)ethynyl]-5-(trifluoromethyl)benzoyl]piperazin-1-yl]benzamide |
| B2L043-01 | N-tert-Butylsulfonyl-4-[4-[3-[4-(3-ethoxyphenyl)pyrazol-1-yl]-5-(trifluoromethyl)benzoyl]piperazin-1-yl]benzamide |
| B2L044-01 | N-tert-Butylsulfonyl-4-[4-[3-[4-(5-ethoxypyridin-3-yl)pyrazol-1-yl]-5-(trifluoromethyl)benzoyl]piperazin-1-yl]benzamide |
| B2L045-01 | N-tert-Butylsulfonyl-4-[4-[[2-(3-ethoxyphenyl)phenyl]methyl]piperazin-1-yl]benzamide |
| B2L046-01 | N-tert-Butylsulfonyl-4-[4-[[2-(5-ethoxypyridin-3-yl)phenyl]methyl]piperazin-1-yl]benzamide |
| B2L047-01 | N-tert-Butylsulfonyl-4-[4-[[4-(3-ethoxyphenyl)-3-methylphenyl]methyl]piperazin-1-yl]benzamide |
| B2L048-01 | N-tert-Butylsulfonyl-4-[4-[[4-(5-ethoxypyridin-3-yl)-3-methylphenyl]methyl]piperazin-1-yl]benzamide |
| B2L049-01 | N-tert-Butylsulfonyl-4-[4-[[4-(3-ethoxyphenyl)-2-ethylphenyl]methyl]piperazin-1-yl]benzamide |

| | |
|---|---|
| B2L050-01 | N-tert-Butylsulfonyl-4-[4-[[4-(5-ethoxypyridin-3-yl)-2-ethylphenyl]methyl]piperazin-1-yl]benzamide |
| B2L051-01 | N-tert-Butylsulfonyl-4-[4-[[4-(3-ethoxyphenyl)-3-fluorophenyl]methyl]piperazin-1-yl]benzamide |
| B2L052-01 | N-tert-Butylsulfonyl-4-[4-[[4-(5-ethoxypyridin-3-yl)-3-fluorophenyl]methyl]piperazin-1-yl]benzamide |
| B2L053-01 | N-tert-Butylsulfonyl-4-[4-[[4-(3-ethoxyphenyl)-2-methoxyphenyl]methyl]piperazin-1-yl]benzamide |
| B2L054-01 | N-tert-Butylsulfonyl-4-[4-[[4-(5-ethoxypyridin-3-yl)-2-methoxyphenyl]methyl]piperazin-1-yl]benzamide |
| B2L055-01 | N-tert-Butylsulfonyl-4-[4-[[3-ethoxy-5-(3-ethoxyphenyl)phenyl]methyl]piperazin-1-yl]benzamide |
| B2L056-01 | N-tert-Butylsulfonyl-4-[4-[[3-ethoxy-5-(5-ethoxypyridin-3-yl)phenyl]methyl]piperazin-1-yl]benzamide |
| B2L057-01 | N-tert-Butylsulfonyl-4-[4-[[3-(3-ethoxyphenyl)-5-propan-2-yloxyphenyl]methyl]piperazin-1-yl]benzamide |
| B2L058-01 | N-tert-Butylsulfonyl-4-[4-[[3-(5-ethoxypyridin-3-yl)-5-propan-2-yloxyphenyl]methyl]piperazin-1-yl]benzamide |
| B2L059-01 | N-tert-Butylsulfonyl-4-[4-[[4-(3-ethoxyphenyl)-2,6-dimethoxyphenyl]methyl]piperazin-1-yl]benzamide |
| B2L060-01 | N-tert-Butylsulfonyl-4-[4-[[4-(5-ethoxypyridin-3-yl)-2,6-dimethoxyphenyl]methyl]piperazin-1-yl]benzamide |
| B2L061-01 | N-tert-Butylsulfonyl-4-[4-[[3-(3-ethoxyphenyl)-5-(trifluoromethyl)phenyl]methyl]piperazin-1-yl]benzamide |
| B2L062-01 | N-tert-Butylsulfonyl-4-[4-[[3-(5-ethoxypyridin-3-yl)-5-(trifluoromethyl)phenyl]methyl]piperazin-1-yl]benzamide |
| B2L063-01 | N-tert-Butylsulfonyl-4-[4-[[3-(3-ethoxyphenyl)-5-(trifluoromethoxy)phenyl]methyl]piperazin-1-yl]benzamide |
| B2L064-01 | N-tert-Butylsulfonyl-4-[4-[[3-(5-ethoxypyridin-3-yl)-5-(trifluoromethoxy)phenyl]methyl]piperazin-1-yl]benzamide |
| B2L065-01 | N-tert-Butylsulfonyl-4-[4-[[4-(3-ethoxyphenyl)naphthalen-1-yl]methyl]piperazin-1-yl]benzamide |
| B2L066-01 | N-tert-Butylsulfonyl-4-[4-[[4-(5-ethoxypyridin-3-yl)naphthalen-1-yl]methyl]piperazin-1-yl]benzamide |
| B2L067-01 | N-tert-Butylsulfonyl-4-[4-[[4-(3-ethoxyphenyl)thiophen-2-yl]methyl]piperazin-1-yl]benzamide |
| B2L068-01 | N-tert-Butylsulfonyl-4-[4-[[4-(5-ethoxypyridin-3-yl)thiophen-2-yl]methyl]piperazin-1-yl]benzamide |
| B2L069-01 | N-tert-Butylsulfonyl-4-[4-[[2-[2-(3-ethoxyphenyl)ethynyl]phenyl]methyl]piperazin-1-yl]benzamide |
| B2L070-01 | N-tert-Butylsulfonyl-4-[4-[[2-[2-(5-ethoxypyridin-3-yl)ethynyl]phenyl]methyl]piperazin-1-yl]benzamide |
| B2L071-01 | N-tert-Butylsulfonyl-6-[4-[[2-(3-ethoxyphenyl)phenyl]methyl]piperazin-1-yl]pyridazine-3-carboxamide |
| B2L072-01 | N-tert-Butylsulfonyl-6-[4-[[2-(5-ethoxypyridin-3-yl)phenyl]methyl]piperazin-1-yl]pyridazine-3-carboxamide |
| B2L073-01 | N-tert-Butylsulfonyl-6-[4-[[4-(3-ethoxyphenyl)-3-methylphenyl]methyl]piperazin-1-yl]pyridazine-3-carboxamide |
| B2L074-01 | N-tert-Butylsulfonyl-6-[4-[[4-(5-ethoxypyridin-3-yl)-3-methylphenyl]methyl]piperazin-1-yl]pyridazine-3-carboxamide |
| B2L075-01 | N-tert-Butylsulfonyl-6-[4-[[4-(3-ethoxyphenyl)-2-ethylphenyl]methyl]piperazin-1-yl]pyridazine-3-carboxamide |
| B2L076-01 | N-tert-Butylsulfonyl-6-[4-[[4-(5-ethoxypyridin-3-yl)-2-ethylphenyl]methyl]piperazin-1-yl]pyridazine-3-carboxamide |
| B2L077-01 | N-tert-Butylsulfonyl-6-[4-[[4-(3-ethoxyphenyl)-3-fluorophenyl]methyl]piperazin-1-yl]pyridazine-3-carboxamide |

| | |
|---|---|
| B2L078-01 | N-tert-Butylsulfonyl-6-[4-[[4-(5-ethoxypyridin-3-yl)-3-fluorophenyl]methyl]piperazin-1-yl]pyridazine-3-carboxamide |
| B2L079-01 | N-tert-Butylsulfonyl-6-[4-[[4-(3-ethoxyphenyl)-2-methoxyphenyl]methyl]piperazin-1-yl]pyridazine-3-carboxamide |
| B2L080-01 | N-tert-Butylsulfonyl-6-[4-[[4-(5-ethoxypyridin-3-yl)-2-methoxyphenyl]methyl]piperazin-1-yl]pyridazine-3-carboxamide |
| B2L081-01 | N-tert-Butylsulfonyl-6-[4-[[3-ethoxy-5-(3-ethoxyphenyl)phenyl]methyl]piperazin-1-yl]pyridazine-3-carboxamide |
| B2L082-01 | N-tert-Butylsulfonyl-6-[4-[[3-ethoxy-5-(5-ethoxypyridin-3-yl)phenyl]methyl]piperazin-1-yl]pyridazine-3-carboxamide |
| B2L083-01 | N-tert-Butylsulfonyl-6-[4-[[3-(3-ethoxyphenyl)-5-propan-2-yloxyphenyl]methyl]piperazin-1-yl]pyridazine-3-carboxamide |
| B2L084-01 | N-tert-Butylsulfonyl-6-[4-[[3-(5-ethoxypyridin-3-yl)-5-propan-2-yloxyphenyl]methyl]piperazin-1-yl]pyridazine-3-carboxamide |
| B2L085-01 | N-tert-Butylsulfonyl-6-[4-[[4-(3-ethoxyphenyl)-2,6-dimethoxyphenyl]methyl]piperazin-1-yl]pyridazine-3-carboxamide |
| B2L086-01 | N-tert-Butylsulfonyl-6-[4-[[4-(5-ethoxypyridin-3-yl)-2,6-dimethoxyphenyl]methyl]piperazin-1-yl]pyridazine-3-carboxamide |
| B2L087-01 | N-tert-Butylsulfonyl-6-[4-[[3-(3-ethoxyphenyl)-5-(trifluoromethyl)phenyl]methyl]piperazin-1-yl]pyridazine-3-carboxamide |
| B2L088-01 | N-tert-Butylsulfonyl-6-[4-[[3-(5-ethoxypyridin-3-yl)-5-(trifluoromethyl)phenyl]methyl]piperazin-1-yl]pyridazine-3-carboxamide |
| B2L089-01 | N-tert-Butylsulfonyl-6-[4-[[3-(3-ethoxyphenyl)-5-(trifluoromethoxy)phenyl]methyl]piperazin-1-yl]pyridazine-3-carboxamide |
| B2L090-01 | N-tert-Butylsulfonyl-6-[4-[[3-(5-ethoxypyridin-3-yl)-5-(trifluoromethoxy)phenyl]methyl]piperazin-1-yl]pyridazine-3-carboxamide |
| B2L091-01 | N-tert-Butylsulfonyl-6-[4-[[4-(3-ethoxyphenyl)naphthalen-1-yl]methyl]piperazin-1-yl]pyridazine-3-carboxamide |
| B2L092-01 | N-tert-Butylsulfonyl-6-[4-[[4-(5-ethoxypyridin-3-yl)naphthalen-1-yl]methyl]piperazin-1-yl]pyridazine-3-carboxamide |
| B2L093-01 | N-tert-Butylsulfonyl-6-[4-[[4-(3-ethoxyphenyl)thiophen-2-yl]methyl]piperazin-1-yl]pyridazine-3-carboxamide |
| B2L094-01 | N-tert-Butylsulfonyl-6-[4-[[4-(5-ethoxypyridin-3-yl)thiophen-2-yl]methyl]piperazin-1-yl]pyridazine-3-carboxamide |
| B2L095-01 | N-tert-Butylsulfonyl-6-[4-[[2-[2-(3-ethoxyphenyl)ethynyl]phenyl]methyl]piperazin-1-yl]pyridazine-3-carboxamide |
| B2L096-01 | N-tert-Butylsulfonyl-6-[4-[[2-[2-(5-ethoxypyridin-3-yl)ethynyl]phenyl]methyl]piperazin-1-yl]pyridazine-3-carboxamide |
| B2L097-01 | N-tert-Butylsulfonyl-4-[4-[1-[4-(3-ethoxyphenyl)phenyl]ethyl]piperazin-1-yl]benzamide |
| B2L098-01 | N-tert-Butylsulfonyl-4-[4-[1-[4-(5-ethoxypyridin-3-yl)phenyl]ethyl]piperazin-1-yl]benzamide |
| B2L099-01 | N-tert-Butylsulfonyl-4-[4-[1-[4-(3-ethoxyphenyl)-2-fluorophenyl]ethyl]piperazin-1-yl]benzamide |
| B2L100-01 | N-tert-Butylsulfonyl-4-[4-[1-[4-(5-ethoxypyridin-3-yl)-2-fluorophenyl]ethyl]piperazin-1-yl]benzamide |

[Table 257]

[Table AI-03-B2L-01]

| Label | Exact | ⊠ | A | a | B | b | C | c | D |
|---|---|---|---|---|---|---|---|---|---|
| B2L001-01 | 557.18 | OEt | A01 | a1 | B10 | b3 | C02 | c2 | D08 |
| B2L002-01 | 558.17 | OEt | A02 | a1 | B10 | b3 | C02 | c2 | D08 |
| B2L003-01 | 565.24 | OEt | A01 | a1 | B04 | b3 | C02 | c2 | D08 |
| B2L004-01 | 566.23 | OEt | A02 | a1 | B04 | b3 | C02 | c2 | D08 |

| | | | | | | | | | |
|---|---|---|---|---|---|---|---|---|---|
| B2L005-01 | 569.21 | OEt | A01 | a1 | B06 | b3 | C02 | c2 | D08 |
| B2L006-01 | 570.21 | OEt | A02 | a1 | B06 | b3 | C02 | c2 | D08 |
| B2L007-01 | 575.22 | OEt | A01 | a2 | B01 | b3 | C02 | c2 | D08 |
| B2L008-01 | 576.22 | OEt | A02 | a2 | B01 | b3 | C02 | c2 | D08 |
| B2L009-01 | 576.22 | OEt | A01 | a2 | B17 | b3 | C02 | c2 | D08 |
| B2L010-01 | 577.21 | OEt | A02 | a2 | B17 | b3 | C02 | c2 | D08 |
| B2L011-01 | 579.25 | OEt | A01 | a1 | B02 | b3 | C02 | c2 | D08 |
| B2L012-01 | 580.25 | OEt | A02 | a1 | B02 | b3 | C02 | c2 | D08 |
| B2L013-01 | 601.24 | OEt | A01 | a1 | B09 | b3 | C02 | c2 | D08 |
| B2L014-01 | 602.23 | OEt | A02 | a1 | B09 | b3 | C02 | c2 | D08 |
| B2L015-01 | 607.28 | OEt | A01 | a1 | B16 | b3 | C02 | c2 | D08 |
| B2L016-01 | 608.28 | OEt | A02 | a1 | B16 | b3 | C02 | c2 | D08 |
| B2L017-01 | 619.21 | OEt | A01 | a1 | B11 | b3 | C02 | c2 | D08 |
| B2L018-01 | 620.2 | OEt | A02 | a1 | B11 | b3 | C02 | c2 | D08 |
| B2L019-01 | 643.21 | OEt | A01 | a2 | B11 | b3 | C02 | c2 | D08 |
| B2L020-01 | 644.2 | OEt | A02 | a2 | B11 | b3 | C02 | c2 | D08 |
| B2L021-01 | 685.23 | OEt | A01 | a1 | B12 | b3 | C02 | c2 | D08 |
| B2L022-01 | 686.23 | OEt | A02 | a1 | B12 | b3 | C02 | c2 | D08 |
| B2L023-01 | 555.19 | OEt | A01 | a1 | B10 | b3 | C01 | c2 | D08 |
| B2L024-01 | 556.18 | OEt | A02 | a1 | B10 | b3 | C01 | c2 | D08 |
| B2L025-01 | 563.25 | OEt | A01 | a1 | B04 | b3 | C01 | c2 | D08 |
| B2L026-01 | 564.24 | OEt | A02 | a1 | B04 | b3 | C01 | c2 | D08 |
| B2L027-01 | 567.22 | OEt | A01 | a1 | B06 | b3 | C01 | c2 | D08 |
| B2L028-01 | 568.22 | OEt | A02 | a1 | B06 | b3 | C01 | c2 | D08 |
| B2L029-01 | 573.23 | OEt | A01 | a2 | B01 | b3 | C01 | c2 | D08 |
| B2L030-01 | 574.23 | OEt | A02 | a2 | B01 | b3 | C01 | c2 | D08 |
| B2L031-01 | 574.23 | OEt | A01 | a2 | B17 | b3 | C01 | c2 | D08 |
| B2L032-01 | 575.22 | OEt | A02 | a2 | B17 | b3 | C01 | c2 | D08 |
| B2L033-01 | 577.26 | OEt | A01 | a1 | B02 | b3 | C01 | c2 | D08 |
| B2L034-01 | 578.26 | OEt | A02 | a1 | B02 | b3 | C01 | c2 | D08 |
| B2L035-01 | 599.25 | OEt | A01 | a1 | B09 | b3 | C01 | c2 | D08 |
| B2L036-01 | 600.24 | OEt | A02 | a1 | B09 | b3 | C01 | c2 | D08 |
| B2L037-01 | 605.29 | OEt | A01 | a1 | B16 | b3 | C01 | c2 | D08 |
| B2L038-01 | 606.29 | OEt | A02 | a1 | B16 | b3 | C01 | c2 | D08 |
| B2L039-01 | 617.22 | OEt | A01 | a1 | B11 | b3 | C01 | c2 | D08 |
| B2L040-01 | 618.21 | OEt | A02 | a1 | B11 | b3 | C01 | c2 | D08 |
| B2L041-01 | 641.22 | OEt | A01 | a2 | B11 | b3 | C01 | c2 | D08 |
| B2L042-01 | 642.21 | OEt | A02 | a2 | B11 | b3 | C01 | c2 | D08 |
| B2L043-01 | 683.24 | OEt | A01 | a1 | B12 | b3 | C01 | c2 | D08 |
| B2L044-01 | 684.23 | OEt | A02 | a1 | B12 | b3 | C01 | c2 | D08 |
| B2L045-01 | 535.25 | OEt | A01 | a1 | B01 | b1 | C01 | c2 | D08 |
| B2L046-01 | 536.25 | OEt | A02 | a1 | B01 | b1 | C01 | c2 | D08 |
| B2L047-01 | 549.27 | OEt | A01 | a1 | B04 | b1 | C01 | c2 | D08 |
| B2L048-01 | 550.26 | OEt | A02 | a1 | B04 | b1 | C01 | c2 | D08 |
| B2L049-01 | 563.28 | OEt | A01 | a1 | B05 | b1 | C01 | c2 | D08 |
| B2L050-01 | 564.28 | OEt | A02 | a1 | B05 | b1 | C01 | c2 | D08 |
| B2L051-01 | 553.24 | OEt | A01 | a1 | B06 | b1 | C01 | c2 | D08 |

| | | | | | | | | | |
|---|---|---|---|---|---|---|---|---|---|
| B2L052-01 | 554.24 | OEt | A02 | a1 | B06 | b1 | C01 | c2 | D08 |
| B2L053-01 | 565.26 | OEt | A01 | a1 | B07 | b1 | C01 | c2 | D08 |
| B2L054-01 | 566.26 | OEt | A02 | a1 | B07 | b1 | C01 | c2 | D08 |
| B2L055-01 | 579.28 | OEt | A01 | a1 | B14 | b1 | C01 | c2 | D08 |
| B2L056-01 | 580.27 | OEt | A02 | a1 | B14 | b1 | C01 | c2 | D08 |
| B2L057-01 | 593.29 | OEt | A01 | a1 | B15 | b1 | C01 | c2 | D08 |
| B2L058-01 | 594.29 | OEt | A02 | a1 | B15 | b1 | C01 | c2 | D08 |
| B2L059-01 | 595.27 | OEt | A01 | a1 | B08 | b1 | C01 | c2 | D08 |
| B2L060-01 | 596.27 | OEt | A02 | a1 | B08 | b1 | C01 | c2 | D08 |
| B2L061-01 | 603.24 | OEt | A01 | a1 | B11 | b1 | C01 | c2 | D08 |
| B2L062-01 | 604.23 | OEt | A02 | a1 | B11 | b1 | C01 | c2 | D08 |
| B2L063-01 | 619.23 | OEt | A01 | a1 | B13 | b1 | C01 | c2 | D08 |
| B2L064-01 | 620.23 | OEt | A02 | a1 | B13 | b1 | C01 | c2 | D08 |
| B2L065-01 | 585.27 | OEt | A01 | a1 | B09 | b1 | C01 | c2 | D08 |
| B2L066-01 | 586.26 | OEt | A02 | a1 | B09 | b1 | C01 | c2 | D08 |
| B2L067-01 | 541.21 | OEt | A01 | a1 | B10 | b1 | C01 | c2 | D08 |
| B2L068-01 | 542.2 | OEt | A02 | a1 | B10 | b1 | C01 | c2 | D08 |
| B2L069-01 | 559.25 | OEt | A01 | a2 | B01 | b1 | C01 | c2 | D08 |
| B2L070-01 | 560.25 | OEt | A02 | a2 | B01 | b1 | C01 | c2 | D08 |
| B2L071-01 | 537.24 | OEt | A01 | a1 | B01 | b1 | C02 | c2 | D08 |
| B2L072-01 | 538.24 | OEt | A02 | a1 | B01 | b1 | C02 | c2 | D08 |
| B2L073-01 | 551.26 | OEt | A01 | a1 | B04 | b1 | C02 | c2 | D08 |
| B2L074-01 | 552.25 | OEt | A02 | a1 | B04 | b1 | C02 | c2 | D08 |
| B2L075-01 | 565.27 | OEt | A01 | a1 | B05 | b1 | C02 | c2 | D08 |
| B2L076-01 | 566.27 | OEt | A02 | a1 | B05 | b1 | C02 | c2 | D08 |
| B2L077-01 | 555.23 | OEt | A01 | a1 | B06 | b1 | C02 | c2 | D08 |
| B2L078-01 | 556.23 | OEt | A02 | a1 | B06 | b1 | C02 | c2 | D08 |
| B2L079-01 | 567.25 | OEt | A01 | a1 | B07 | b1 | C02 | c2 | D08 |
| B2L080-01 | 568.25 | OEt | A02 | a1 | B07 | b1 | C02 | c2 | D08 |
| B2L081-01 | 581.27 | OEt | A01 | a1 | B14 | b1 | C02 | c2 | D08 |
| B2L082-01 | 582.26 | OEt | A02 | a1 | B14 | b1 | C02 | c2 | D08 |
| B2L083-01 | 595.28 | OEt | A01 | a1 | B15 | b1 | C02 | c2 | D08 |
| B2L084-01 | 596.28 | OEt | A02 | a1 | B15 | b1 | C02 | c2 | D08 |
| B2L085-01 | 597.26 | OEt | A01 | a1 | B08 | b1 | C02 | c2 | D08 |
| B2L086-01 | 598.26 | OEt | A02 | a1 | B08 | b1 | C02 | c2 | D08 |
| B2L087-01 | 605.23 | OEt | A01 | a1 | B11 | b1 | C02 | c2 | D08 |
| B2L088-01 | 606.22 | OEt | A02 | a1 | B11 | b1 | C02 | c2 | D08 |
| B2L089-01 | 621.22 | OEt | A01 | a1 | B13 | b1 | C02 | c2 | D08 |
| B2L090-01 | 622.22 | OEt | A02 | a1 | B13 | b1 | C02 | c2 | D08 |
| B2L091-01 | 587.26 | OEt | A01 | a1 | B09 | b1 | C02 | c2 | D08 |
| B2L092-01 | 588.25 | OEt | A02 | a1 | B09 | b1 | C02 | c2 | D08 |
| B2L093-01 | 543.2 | OEt | A01 | a1 | B10 | b1 | C02 | c2 | D08 |
| B2L094-01 | 544.19 | OEt | A02 | a1 | B10 | b1 | C02 | c2 | D08 |
| B2L095-01 | 561.24 | OEt | A01 | a2 | B01 | b1 | C02 | c2 | D08 |
| B2L096-01 | 562.24 | OEt | A02 | a2 | B01 | b1 | C02 | c2 | D08 |
| B2L097-01 | 549.27 | OEt | A01 | a1 | B03 | b2 | C01 | c2 | D08 |
| B2L098-01 | 550.26 | OEt | A02 | a1 | B03 | b2 | C01 | c2 | D08 |

| | | | | | | | | | |
|---|---|---|---|---|---|---|---|---|---|
| B2L099-01 | 567.26 | OEt | A01 | a1 | B18 | b2 | C01 | c2 | D08 |
| B2L100-01 | 568.25 | OEt | A02 | a1 | B18 | b2 | C01 | c2 | D08 |

[Table 258]

[Table AI-03-B2N-01]

| Mixture No. | mixAI-B2N-01 |
|---|---|
| No. of compound that may be contained | Compound name |
| B2N001-01 | 6-[4-[4-(3-Ethoxyphenyl)thiophene-2-carbonyl]piperazin-1-yl]-N-(3,3,3-trifluoropropylsulfonyl)pyridazine-3-carboxamide |
| B2N002-01 | 6-[4-[4-(5-Ethoxypyridin-3-yl)thiophene-2-carbonyl]piperazin-1-yl]-N-(3,3,3-trifluoropropylsulfonyl)pyridazine-3-carboxamide |
| B2N003-01 | 6-[4-[4-(3-Ethoxyphenyl)-3-methylbenzoyl]piperazin-1-yl]-N-(3,3,3-trifluoropropylsulfonyl)pyridazine-3-carboxamide |
| B2N004-01 | 6-[4-[4-(5-Ethoxypyridin-3-yl)-3-methylbenzoyl]piperazin-1-yl]-N-(3,3,3-trifluoropropylsulfonyl)pyridazine-3-carboxamide |
| B2N005-01 | 6-[4-[4-(3-Ethoxyphenyl)-3-fluorobenzoyl]piperazin-1-yl]-N-(3,3,3-trifluoropropylsulfonyl)pyridazine-3-carboxamide |
| B2N006-01 | 6-[4-[4-(5-Ethoxypyridin-3-yl)-3-fluorobenzoyl]piperazin-1-yl]-N-(3,3,3-trifluoropropylsulfonyl)pyridazine-3-carboxamide |
| B2N007-01 | 6-[4-[2-[2-(3-Ethoxyphenyl)ethynyl]benzoyl]piperazin-1-yl]-N-(3,3,3-trifluoropropylsulfonyl)pyridazine-3-carboxamide |
| B2N008-01 | 6-[4-[2-[2-(5-Ethoxypyridin-3-yl)ethynyl]benzoyl]piperazin-1-yl]-N-(3,3,3-trifluoropropylsulfonyl)pyridazine-3-carboxamide |
| B2N009-01 | 6-[4-[5-[2-(3-Ethoxyphenyl)ethynyl]pyridine-3-carbonyl]piperazin-1-yl]-N-(3,3,3-trifluoropropylsulfonyl)pyridazine-3-carboxamide |
| B2N010-01 | 6-[4-[5-[2-(5-Ethoxypyridin-3-yl)ethynyl]pyridine-3-carbonyl]piperazin-1-yl]-N-(3,3,3-trifluoropropylsulfonyl)pyridazine-3-carboxamide |
| B2N011-01 | 6-[4-[3-[2-(3-Ethoxyphenyl)phenyl]propanoyl]piperazin-1-yl]-N-(3,3,3-trifluoropropylsulfonyl)pyridazine-3-carboxamide |
| B2N012-01 | 6-[4-[3-[2-(5-Ethoxypyridin-3-yl)phenyl]propanoyl]piperazin-1-yl]-N-(3,3,3-trifluoropropylsulfonyl)pyridazine-3-carboxamide |
| B2N013-01 | 6-[4-[4-(3-Ethoxyphenyl)naphthalene-1-carbonyl]piperazin-1-yl]-N-(3,3,3-trifluoropropylsulfonyl)pyridazine-3-carboxamide |
| B2N014-01 | 6-[4-[4-(5-Ethoxypyridin-3-yl)naphthalene-1-carbonyl]piperazin-1-yl]-N-(3,3,3-trifluoropropylsulfonyl)pyridazine-3-carboxamide |
| B2N015-01 | 6-[4-[3-tert-Butyl-5-(3-ethoxyphenyl)benzoyl]piperazin-1-yl]-N-(3,3,3-trifluoropropylsulfonyl)pyridazine-3-carboxamide |
| B2N016-01 | 6-[4-[3-tert-Butyl-5-(5-ethoxypyridin-3-yl)benzoyl]piperazin-1-yl]-N-(3,3,3-trifluoropropylsulfonyl)pyridazine-3-carboxamide |
| B2N017-01 | 6-[4-[3-(3-Ethoxyphenyl)-5-(trifluoromethyl)benzoyl]piperazin-1-yl]-N-(3,3,3-trifluoropropylsulfonyl)pyridazine-3-carboxamide |
| B2N018-01 | 6-[4-[3-(5-Ethoxypyridin-3-yl)-5-(trifluoromethyl)benzoyl]piperazin-1-yl]-N-(3,3,3-trifluoropropylsulfonyl)pyridazine-3-carboxamide |
| B2N019-01 | 6-[4-[3-[2-(3-Ethoxyphenyl)ethynyl]-5-(trifluoromethyl)benzoyl]piperazin-1-yl]-N-(3,3,3-trifluoropropylsulfonyl)pyridazine-3-carboxamide |
| B2N020-01 | 6-[4-[3-[2-(5-Ethoxypyridin-3-yl)ethynyl]-5-(trifluoromethyl)benzoyl]piperazin-1-yl]-N-(3,3,3-trifluoropropylsulfonyl)pyridazine-3-carboxamide |
| B2N021-01 | 6-[4-[3-[4-(3-Ethoxyphenyl)pyrazol-1-yl]-5-(trifluoromethyl)benzoyl]piperazin-1-yl]-N-(3,3,3-trifluoropropylsulfonyl)pyridazine-3-carboxamide |
| B2N022-01 | 6-[4-[3-[4-(5-Ethoxypyridin-3-yl)pyrazol-1-yl]-5-(trifluoromethyl)benzoyl]piperazin-1-yl]-N-(3,3,3-trifluoropropylsulfonyl)pyridazine-3-carboxamide |

| | |
|---|---|
| B2N023-01 | 4-[4-[4-(3-Ethoxyphenyl)thiophene-2-carbonyl]piperazin-1-yl]-N-(3,3,3-trifluoropropylsulfonyl)benzamide |
| B2N024-01 | 4-[4-[4-(5-Ethoxypyridin-3-yl)thiophene-2-carbonyl]piperazin-1-yl]-N-(3,3,3-trifluoropropylsulfonyl)benzamide |
| B2N025-01 | 4-[4-[4-(3-Ethoxyphenyl)-3-methylbenzoyl]piperazin-1-yl]-N-(3,3,3-trifluoropropylsulfonyl)benzamide |
| B2N026-01 | 4-[4-[4-(5-Ethoxypyridin-3-yl)-3-methylbenzoyl]piperazin-1-yl]-N-(3,3,3-trifluoropropylsulfonyl)benzamide |
| B2N027-01 | 4-[4-[4-(3-Ethoxyphenyl)-3-fluorobenzoyl]piperazin-1-yl]-N-(3,3,3-trifluoropropylsulfonyl)benzamide |
| B2N028-01 | 4-[4-[4-(5-Ethoxypyridin-3-yl)-3-fluorobenzoyl]piperazin-1-yl]-N-(3,3,3-trifluoropropylsulfonyl)benzamide |
| B2N029-01 | 4-[4-[2-[2-(3-Ethoxyphenyl)ethynyl]benzoyl]piperazin-1-yl]-N-(3,3,3-trifluoropropylsulfonyl)benzamide |
| B2N030-01 | 4-[4-[2-[2-(5-Ethoxypyridin-3-yl)ethynyl]benzoyl]piperazin-1-yl]-N-(3,3,3-trifluoropropylsulfonyl)benzamide |
| B2N031-01 | 4-[4-[5-[2-(3-Ethoxyphenyl)ethynyl]pyridine-3-carbonyl]piperazin-1-yl]-N-(3,3,3-trifluoropropylsulfonyl)benzamide |
| B2N032-01 | 4-[4-[5-[2-(5-Ethoxypyridin-3-yl)ethynyl]pyridine-3-carbonyl]piperazin-1-yl]-N-(3,3,3-trifluoropropylsulfonyl)benzamide |
| B2N033-01 | 4-[4-[3-[2-(3-Ethoxyphenyl)phenyl]propanoyl]piperazin-1-yl]-N-(3,3,3-trifluoropropylsulfonyl)benzamide |
| B2N034-01 | 4-[4-[3-[2-(5-Ethoxypyridin-3-yl)phenyl]propanoyl]piperazin-1-yl]-N-(3,3,3-trifluoropropylsulfonyl)benzamide |
| B2N035-01 | 4-[4-[4-(3-Ethoxyphenyl)naphthalene-1-carbonyl]piperazin-1-yl]-N-(3,3,3-trifluoropropylsulfonyl)benzamide |
| B2N036-01 | 4-[4-[4-(5-Ethoxypyridin-3-yl)naphthalene-1-carbonyl]piperazin-1-yl]-N-(3,3,3-trifluoropropylsulfonyl)benzamide |
| B2N037-01 | 4-[4-[3-tert-Butyl-5-(3-ethoxyphenyl)benzoyl]piperazin-1-yl]-N-(3,3,3-trifluoropropylsulfonyl)benzamide |
| B2N038-01 | 4-[4-[3-tert-Butyl-5-(5-ethoxypyridin-3-yl)benzoyl]piperazin-1-yl]-N-(3,3,3-trifluoropropylsulfonyl)benzamide |
| B2N039-01 | 4-[4-[3-(3-Ethoxyphenyl)-5-(trifluoromethyl)benzoyl]piperazin-1-yl]-N-(3,3,3-trifluoropropylsulfonyl)benzamide |
| B2N040-01 | 4-[4-[3-(5-Ethoxypyridin-3-yl)-5-(trifluoromethyl)benzoyl]piperazin-1-yl]-N-(3,3,3-trifluoropropylsulfonyl)benzamide |
| B2N041-01 | 4-[4-[3-[2-(3-Ethoxyphenyl)ethynyl]-5-(trifluoromethyl)benzoyl]piperazin-1-yl]-N-(3,3,3-trifluoropropylsulfonyl)benzamide |
| B2N042-01 | 4-[4-[3-[2-(5-Ethoxypyridin-3-yl)ethynyl]-5-(trifluoromethyl)benzoyl]piperazin-1-yl]-N-(3,3,3-trifluoropropylsulfonyl)benzamide |
| B2N043-01 | 4-[4-[3-[4-(3-Ethoxyphenyl)pyrazol-1-yl]-5-(trifluoromethyl)benzoyl]piperazin-1-yl]-N-(3,3,3-trifluoropropylsulfonyl)benzamide |
| B2N044-01 | 4-[4-[3-[4-(5-Ethoxypyridin-3-yl)pyrazol-1-yl]-5-(trifluoromethyl)benzoyl]piperazin-1-yl]-N-(3,3,3-trifluoropropylsulfonyl)benzamide |
| B2N045-01 | 4-[4-[[2-(3-Ethoxyphenyl)phenyl]methyl]piperazin-1-yl]-N-(3,3,3-trifluoropropylsulfonyl)benzamide |
| B2N046-01 | 4-[4-[[2-(5-Ethoxypyridin-3-yl)phenyl]methyl]piperazin-1-yl]-N-(3,3,3-trifluoropropylsulfonyl)benzamide |
| B2N047-01 | 4-[4-[[4-(3-Ethoxyphenyl)-3-methylphenyl]methyl]piperazin-1-yl]-N-(3,3,3-trifluoropropylsulfonyl)benzamide |
| B2N048-01 | 4-[4-[[4-(5-Ethoxypyridin-3-yl)-3-methylphenyl]methyl]piperazin-1-yl]-N-(3,3,3-trifluoropropylsulfonyl)benzamide |
| B2N049-01 | 4-[4-[[4-(3-Ethoxyphenyl)-2-ethylphenyl]methyl]piperazin-1-yl]-N-(3,3,3-trifluoropropylsulfonyl)benzamide |
| B2N050-01 | 4-[4-[[4-(5-Ethoxypyridin-3-yl)-2-ethylphenyl]methyl]piperazin-1-yl]-N-(3,3,3-trifluoropropylsulfonyl)benzamide |

| | |
|---|---|
| B2N051-01 | 4-[4-[[4-(3-Ethoxyphenyl)-3-fluorophenyl]methyl]piperazin-1-yl]-N-(3,3,3-trifluoropropylsulfonyl)benzamide |
| B2N052-01 | 4-[4-[[4-(5-Ethoxypyridin-3-yl)-3-fluorophenyl]methyl]piperazin-1-yl]-N-(3,3,3-trifluoropropylsulfonyl)benzamide |
| B2N053-01 | 4-[4-[[4-(3-Ethoxyphenyl)-2-methoxyphenyl]methyl]piperazin-1-yl]-N-(3,3,3-trifluoropropylsulfonyl)benzamide |
| B2N054-01 | 4-[4-[[4-(5-Ethoxypyridin-3-yl)-2-methoxyphenyl]methyl]piperazin-1-yl]-N-(3,3,3-trifluoropropylsulfonyl)benzamide |
| B2N055-01 | 4-[4-[[3-Ethoxy-5-(3-ethoxyphenyl)phenyl]methyl]piperazin-1-yl]-N-(3,3,3-trifluoropropylsulfonyl)benzamide |
| B2N056-01 | 4-[4-[[3-Ethoxy-5-(5-ethoxypyridin-3-yl)phenyl]methyl]piperazin-1-yl]-N-(3,3,3-trifluoropropylsulfonyl)benzamide |
| B2N057-01 | 4-[4-[[3-(3-Ethoxyphenyl)-5-propan-2-yloxyphenyl]methyl]piperazin-1-yl]-N-(3,3,3-trifluoropropylsulfonyl)benzamide |
| B2N058-01 | 4-[4-[[3-(5-Ethoxypyridin-3-yl)-5-propan-2-yloxyphenyl]methyl]piperazin-1-yl]-N-(3,3,3-trifluoropropylsulfonyl)benzamide |
| B2N059-01 | 4-[4-[[4-(3-Ethoxyphenyl)-2,6-dimethoxyphenyl]methyl]piperazin-1-yl]-N-(3,3,3-trifluoropropylsulfonyl)benzamide |
| B2N060-01 | 4-[4-[[4-(5-Ethoxypyridin-3-yl)-2,6-dimethoxyphenyl]methyl]piperazin-1-yl]-N-(3,3,3-trifluoropropylsulfonyl)benzamide |
| B2N061-01 | 4-[4-[[3-(3-Ethoxyphenyl)-5-(trifluoromethyl)phenyl]methyl]piperazin-1-yl]-N-(3,3,3-trifluoropropylsulfonyl)benzamide |
| B2N062-01 | 4-[4-[[3-(5-Ethoxypyridin-3-yl)-5-(trifluoromethyl)phenyl]methyl]piperazin-1-yl]-N-(3,3,3-trifluoropropylsulfonyl)benzamide |
| B2N063-01 | 4-[4-[[3-(3-Ethoxyphenyl)-5-(trifluoromethoxy)phenyl]methyl]piperazin-1-yl]-N-(3,3,3-trifluoropropylsulfonyl)benzamide |
| B2N064-01 | 4-[4-[[3-(5-Ethoxypyridin-3-yl)-5-(trifluoromethoxy)phenyl]methyl]piperazin-1-yl]-N-(3,3,3-trifluoropropylsulfonyl)benzamide |
| B2N065-01 | 4-[4-[[4-(3-Ethoxyphenyl)naphthalen-1-yl]methyl]piperazin-1-yl]-N-(3,3,3-trifluoropropylsulfonyl)benzamide |
| B2N066-01 | 4-[4-[[4-(5-Ethoxypyridin-3-yl)naphthalen-1-yl]methyl]piperazin-1-yl]-N-(3,3,3-trifluoropropylsulfonyl)benzamide |
| B2N067-01 | 4-[4-[[4-(3-Ethoxyphenyl)thiophen-2-yl]methyl]piperazin-1-yl]-N-(3,3,3-trifluoropropylsulfonyl)benzamide |
| B2N068-01 | 4-[4-[[4-(5-Ethoxypyridin-3-yl)thiophen-2-yl]methyl]piperazin-1-yl]-N-(3,3,3-trifluoropropylsulfonyl)benzamide |
| B2N069-01 | 4-[4-[[2-[2-(3-Ethoxyphenyl)ethynyl]phenyl]methyl]piperazin-1-yl]-N-(3,3,3-trifluoropropylsulfonyl)benzamide |
| B2N070-01 | 4-[4-[[2-[2-(5-Ethoxypyridin-3-yl)ethynyl]phenyl]methyl]piperazin-1-yl]-N-(3,3,3-trifluoropropylsulfonyl)benzamide |
| B2N071-01 | 6-[4-[[2-(3-Ethoxyphenyl)phenyl]methyl]piperazin-1-yl]-N-(3,3,3-trifluoropropylsulfonyl)pyridazine-3-carboxamide |
| B2N072-01 | 6-[4-[[2-(5-Ethoxypyridin-3-yl)phenyl]methyl]piperazin-1-yl]-N-(3,3,3-trifluoropropylsulfonyl)pyridazine-3-carboxamide |
| B2N073-01 | 6-[4-[[4-(3-Ethoxyphenyl)-3-methylphenyl]methyl]piperazin-1-yl]-N-(3,3,3-trifluoropropylsulfonyl)pyridazine-3-carboxamide |
| B2N074-01 | 6-[4-[[4-(5-Ethoxypyridin-3-yl)-3-methylphenyl]methyl]piperazin-1-yl]-N-(3,3,3-trifluoropropylsulfonyl)pyridazine-3-carboxamide |
| B2N075-01 | 6-[4-[[4-(3-Ethoxyphenyl)-2-ethylphenyl]methyl]piperazin-1-yl]-N-(3,3,3-trifluoropropylsulfonyl)pyridazine-3-carboxamide |
| B2N076-01 | 6-[4-[[4-(5-Ethoxypyridin-3-yl)-2-ethylphenyl]methyl]piperazin-1-yl]-N-(3,3,3-trifluoropropylsulfonyl)pyridazine-3-carboxamide |
| B2N077-01 | 6-[4-[[4-(3-Ethoxyphenyl)-3-fluorophenyl]methyl]piperazin-1-yl]-N-(3,3,3-trifluoropropylsulfonyl)pyridazine-3-carboxamide |
| B2N078-01 | 6-[4-[[4-(5-Ethoxypyridin-3-yl)-3-fluorophenyl]methyl]piperazin-1-yl]-N-(3,3,3-trifluoropropylsulfonyl)pyridazine-3-carboxamide |

| | |
|---|---|
| B2N079-01 | 6-[4-[[4-(3-Ethoxyphenyl)-2-methoxyphenyl]methyl]piperazin-1-yl]-N-(3,3,3-trifluoropropylsulfonyl)pyridazine-3-carboxamide |
| B2N080-01 | 6-[4-[[4-(5-Ethoxypyridin-3-yl)-2-methoxyphenyl]methyl]piperazin-1-yl]-N-(3,3,3-trifluoropropylsulfonyl)pyridazine-3-carboxamide |
| B2N081-01 | 6-[4-[[3-Ethoxy-5-(3-ethoxyphenyl)phenyl]methyl]piperazin-1-yl]-N-(3,3,3-trifluoropropylsulfonyl)pyridazine-3-carboxamide |
| B2N082-01 | 6-[4-[[3-Ethoxy-5-(5-ethoxypyridin-3-yl)phenyl]methyl]piperazin-1-yl]-N-(3,3,3-trifluoropropylsulfonyl)pyridazine-3-carboxamide |
| B2N083-01 | 6-[4-[[3-(3-Ethoxyphenyl)-5-propan-2-yloxyphenyl]methyl]piperazin-1-yl]-N-(3,3,3-trifluoropropylsulfonyl)pyridazine-3-carboxamide |
| B2N084-01 | 6-[4-[[3-(5-Ethoxypyridin-3-yl)-5-propan-2-yloxyphenyl]methyl]piperazin-1-yl]-N-(3,3,3-trifluoropropylsulfonyl)pyridazine-3-carboxamide |
| B2N085-01 | 6-[4-[[4-(3-Ethoxyphenyl)-2,6-dimethoxyphenyl]methyl]piperazin-1-yl]-N-(3,3,3-trifluoropropylsulfonyl)pyridazine-3-carboxamide |
| B2N086-01 | 6-[4-[[4-(5-Ethoxypyridin-3-yl)-2,6-dimethoxyphenyl]methyl]piperazin-1-yl]-N-(3,3,3-trifluoropropylsulfonyl)pyridazine-3-carboxamide |
| B2N087-01 | 6-[4-[[3-(3-Ethoxyphenyl)-5-(trifluoromethyl)phenyl]methyl]piperazin-1-yl]-N-(3,3,3-trifluoropropylsulfonyl)pyridazine-3-carboxamide |
| B2N088-01 | 6-[4-[[3-(5-Ethoxypyridin-3-yl)-5-(trifluoromethyl)phenyl]methyl]piperazin-1-yl]-N-(3,3,3-trifluoropropylsulfonyl)pyridazine-3-carboxamide |
| B2N089-01 | 6-[4-[[3-(3-Ethoxyphenyl)-5-(trifluoromethoxy)phenyl]methyl]piperazin-1-yl]-N-(3,3,3-trifluoropropylsulfonyl)pyridazine-3-carboxamide |
| B2N090-01 | 6-[4-[[3-(5-Ethoxypyridin-3-yl)-5-(trifluoromethoxy)phenyl]methyl]piperazin-1-yl]-N-(3,3,3-trifluoropropylsulfonyl)pyridazine-3-carboxamide |
| B2N091-01 | 6-[4-[[4-(3-Ethoxyphenyl)naphthalen-1-yl]methyl]piperazin-1-yl]-N-(3,3,3-trifluoropropylsulfonyl)pyridazine-3-carboxamide |
| B2N092-01 | 6-[4-[[4-(5-Ethoxypyridin-3-yl)naphthalen-1-yl]methyl]piperazin-1-yl]-N-(3,3,3-trifluoropropylsulfonyl)pyridazine-3-carboxamide |
| B2N093-01 | 6-[4-[[4-(3-Ethoxyphenyl)thiophen-2-yl]methyl]piperazin-1-yl]-N-(3,3,3-trifluoropropylsulfonyl)pyridazine-3-carboxamide |
| B2N094-01 | 6-[4-[[4-(5-Ethoxypyridin-3-yl)thiophen-2-yl]methyl]piperazin-1-yl]-N-(3,3,3-trifluoropropylsulfonyl)pyridazine-3-carboxamide |
| B2N095-01 | 6-[4-[[2-[2-(3-Ethoxyphenyl)ethynyl]phenyl]methyl]piperazin-1-yl]-N-(3,3,3-trifluoropropylsulfonyl)pyridazine-3-carboxamide |
| B2N096-01 | 6-[4-[[2-[2-(5-Ethoxypyridin-3-yl)ethynyl]phenyl]methyl]piperazin-1-yl]-N-(3,3,3-trifluoropropylsulfonyl)pyridazine-3-carboxamide |
| B2N097-01 | 4-[4-[1-[4-(3-Ethoxyphenyl)phenyl]ethyl]piperazin-1-yl]-N-(3,3,3-trifluoropropylsulfonyl)benzamide |
| B2N098-01 | 4-[4-[1-[4-(5-Ethoxypyridin-3-yl)phenyl]ethyl]piperazin-1-yl]-N-(3,3,3-trifluoropropylsulfonyl)benzamide |
| B2N099-01 | 4-[4-[1-[4-(3-Ethoxyphenyl)-2-fluorophenyl]ethyl]piperazin-1-yl]-N-(3,3,3-trifluoropropylsulfonyl)benzamide |
| B2N100-01 | 4-[4-[1-[4-(5-Ethoxypyridin-3-yl)-2-fluorophenyl]ethyl]piperazin-1-yl]-N-(3,3,3-trifluoropropylsulfonyl)benzamide |

[Table 259]

[Table AI-03-B2N-01]

| Label | Exact | ☒ | A | a | B | b | C | c | D |
|---|---|---|---|---|---|---|---|---|---|
| B2N001-01 | 597.13 | OEt | A01 | a1 | B10 | b3 | C02 | c2 | D09 |
| B2N002-01 | 598.13 | OEt | A02 | a1 | B10 | b3 | C02 | c2 | D09 |
| B2N003-01 | 605.19 | OEt | A01 | a1 | B04 | b3 | C02 | c2 | D09 |
| B2N004-01 | 606.19 | OEt | A02 | a1 | B04 | b3 | C02 | c2 | D09 |
| B2N005-01 | 609.17 | OEt | A01 | a1 | B06 | b3 | C02 | c2 | D09 |
| B2N006-01 | 610.16 | OEt | A02 | a1 | B06 | b3 | C02 | c2 | D09 |

| | | | | | | | | | |
|---|---|---|---|---|---|---|---|---|---|
| B2N007-01 | 615.18 | OEt | A01 | a2 | B01 | b3 | C02 | c2 | D09 |
| B2N008-01 | 616.17 | OEt | A02 | a2 | B01 | b3 | C02 | c2 | D09 |
| B2N009-01 | 616.17 | OEt | A01 | a2 | B17 | b3 | C02 | c2 | D09 |
| B2N010-01 | 617.17 | OEt | A02 | a2 | B17 | b3 | C02 | c2 | D09 |
| B2N011-01 | 619.21 | OEt | A01 | a1 | B02 | b3 | C02 | c2 | D09 |
| B2N012-01 | 620.2 | OEt | A02 | a1 | B02 | b3 | C02 | c2 | D09 |
| B2N013-01 | 641.19 | OEt | A01 | a1 | B09 | b3 | C02 | c2 | D09 |
| B2N014-01 | 642.19 | OEt | A02 | a1 | B09 | b3 | C02 | c2 | D09 |
| B2N015-01 | 647.24 | OEt | A01 | a1 | B16 | b3 | C02 | c2 | D09 |
| B2N016-01 | 648.23 | OEt | A02 | a1 | B16 | b3 | C02 | c2 | D09 |
| B2N017-01 | 659.16 | OEt | A01 | a1 | B11 | b3 | C02 | c2 | D09 |
| B2N018-01 | 660.16 | OEt | A02 | a1 | B11 | b3 | C02 | c2 | D09 |
| B2N019-01 | 683.16 | OEt | A01 | a2 | B11 | b3 | C02 | c2 | D09 |
| B2N020-01 | 684.16 | OEt | A02 | a2 | B11 | b3 | C02 | c2 | D09 |
| B2N021-01 | 725.19 | OEt | A01 | a1 | B12 | b3 | C02 | c2 | D09 |
| B2N022-01 | 726.18 | OEt | A02 | a1 | B12 | b3 | C02 | c2 | D09 |
| B2N023-01 | 595.14 | OEt | A01 | a1 | B10 | b3 | C01 | c2 | D09 |
| B2N024-01 | 596.14 | OEt | A02 | a1 | B10 | b3 | C01 | c2 | D09 |
| B2N025-01 | 603.2 | OEt | A01 | a1 | B04 | b3 | C01 | c2 | D09 |
| B2N026-01 | 604.2 | OEt | A02 | a1 | B04 | b3 | C01 | c2 | D09 |
| B2N027-01 | 607.18 | OEt | A01 | a1 | B06 | b3 | C01 | c2 | D09 |
| B2N028-01 | 608.17 | OEt | A02 | a1 | B06 | b3 | C01 | c2 | D09 |
| B2N029-01 | 613.19 | OEt | A01 | a2 | B01 | b3 | C01 | c2 | D09 |
| B2N030-01 | 614.18 | OEt | A02 | a2 | B01 | b3 | C01 | c2 | D09 |
| B2N031-01 | 614.18 | OEt | A01 | a2 | B17 | b3 | C01 | c2 | D09 |
| B2N032-01 | 615.18 | OEt | A02 | a2 | B17 | b3 | C01 | c2 | D09 |
| B2N033-01 | 617.22 | OEt | A01 | a1 | B02 | b3 | C01 | c2 | D09 |
| B2N034-01 | 618.21 | OEt | A02 | a1 | B02 | b3 | C01 | c2 | D09 |
| B2N035-01 | 639.2 | OEt | A01 | a1 | B09 | b3 | C01 | c2 | D09 |
| B2N036-01 | 640.2 | OEt | A02 | a1 | B09 | b3 | C01 | c2 | D09 |
| B2N037-01 | 645.25 | OEt | A01 | a1 | B16 | b3 | C01 | c2 | D09 |
| B2N038-01 | 646.24 | OEt | A02 | a1 | B16 | b3 | C01 | c2 | D09 |
| B2N039-01 | 657.17 | OEt | A01 | a1 | B11 | b3 | C01 | c2 | D09 |
| B2N040-01 | 658.17 | OEt | A02 | a1 | B11 | b3 | C01 | c2 | D09 |
| B2N041-01 | 681.17 | OEt | A01 | a2 | B11 | b3 | C01 | c2 | D09 |
| B2N042-01 | 682.17 | OEt | A02 | a2 | B11 | b3 | C01 | c2 | D09 |
| B2N043-01 | 723.2 | OEt | A01 | a1 | B12 | b3 | C01 | c2 | D09 |
| B2N044-01 | 724.19 | OEt | A02 | a1 | B12 | b3 | C01 | c2 | D09 |
| B2N045-01 | 575.21 | OEt | A01 | a1 | B01 | b1 | C01 | c2 | D09 |
| B2N046-01 | 576.2 | OEt | A02 | a1 | B01 | b1 | C01 | c2 | D09 |
| B2N047-01 | 589.22 | OEt | A01 | a1 | B04 | b1 | C01 | c2 | D09 |
| B2N048-01 | 590.22 | OEt | A02 | a1 | B04 | b1 | C01 | c2 | D09 |
| B2N049-01 | 603.24 | OEt | A01 | a1 | B05 | b1 | C01 | c2 | D09 |
| B2N050-01 | 604.23 | OEt | A02 | a1 | B05 | b1 | C01 | c2 | D09 |
| B2N051-01 | 593.2 | OEt | A01 | a1 | B06 | b1 | C01 | c2 | D09 |
| B2N052-01 | 594.19 | OEt | A02 | a1 | B06 | b1 | C01 | c2 | D09 |
| B2N053-01 | 605.22 | OEt | A01 | a1 | B07 | b1 | C01 | c2 | D09 |

| | | | | | | | | | |
|---|---|---|---|---|---|---|---|---|---|
| B2N054-01 | 606.21 | OEt | A02 | a1 | B07 | b1 | C01 | c2 | D09 |
| B2N055-01 | 619.23 | OEt | A01 | a1 | B14 | b1 | C01 | c2 | D09 |
| B2N056-01 | 620.23 | OEt | A02 | a1 | B14 | b1 | C01 | c2 | D09 |
| B2N057-01 | 633.25 | OEt | A01 | a1 | B15 | b1 | C01 | c2 | D09 |
| B2N058-01 | 634.24 | OEt | A02 | a1 | B15 | b1 | C01 | c2 | D09 |
| B2N059-01 | 635.23 | OEt | A01 | a1 | B08 | b1 | C01 | c2 | D09 |
| B2N060-01 | 636.22 | OEt | A02 | a1 | B08 | b1 | C01 | c2 | D09 |
| B2N061-01 | 643.19 | OEt | A01 | a1 | B11 | b1 | C01 | c2 | D09 |
| B2N062-01 | 644.19 | OEt | A02 | a1 | B11 | b1 | C01 | c2 | D09 |
| B2N063-01 | 659.19 | OEt | A01 | a1 | B13 | b1 | C01 | c2 | D09 |
| B2N064-01 | 660.18 | OEt | A02 | a1 | B13 | b1 | C01 | c2 | D09 |
| B2N065-01 | 625.22 | OEt | A01 | a1 | B09 | b1 | C01 | c2 | D09 |
| B2N066-01 | 626.22 | OEt | A02 | a1 | B09 | b1 | C01 | c2 | D09 |
| B2N067-01 | 581.16 | OEt | A01 | a1 | B10 | b1 | C01 | c2 | D09 |
| B2N068-01 | 582.16 | OEt | A02 | a1 | B10 | b1 | C01 | c2 | D09 |
| B2N069-01 | 599.21 | OEt | A01 | a2 | B01 | b1 | C01 | c2 | D09 |
| B2N070-01 | 600.2 | OEt | A02 | a2 | B01 | b1 | C01 | c2 | D09 |
| B2N071-01 | 577.2 | OEt | A01 | a1 | B01 | b1 | C02 | c2 | D09 |
| B2N072-01 | 578.19 | OEt | A02 | a1 | B01 | b1 | C02 | c2 | D09 |
| B2N073-01 | 591.21 | OEt | A01 | a1 | B04 | b1 | C02 | c2 | D09 |
| B2N074-01 | 592.21 | OEt | A02 | a1 | B04 | b1 | C02 | c2 | D09 |
| B2N075-01 | 605.23 | OEt | A01 | a1 | B05 | b1 | C02 | c2 | D09 |
| B2N076-01 | 606.22 | OEt | A02 | a1 | B05 | b1 | C02 | c2 | D09 |
| B2N077-01 | 595.19 | OEt | A01 | a1 | B06 | b1 | C02 | c2 | D09 |
| B2N078-01 | 596.18 | OEt | A02 | a1 | B06 | b1 | C02 | c2 | D09 |
| B2N079-01 | 607.21 | OEt | A01 | a1 | B07 | b1 | C02 | c2 | D09 |
| B2N080-01 | 608.2 | OEt | A02 | a1 | B07 | b1 | C02 | c2 | D09 |
| B2N081-01 | 621.22 | OEt | A01 | a1 | B14 | b1 | C02 | c2 | D09 |
| B2N082-01 | 622.22 | OEt | A02 | a1 | B14 | b1 | C02 | c2 | D09 |
| B2N083-01 | 635.24 | OEt | A01 | a1 | B15 | b1 | C02 | c2 | D09 |
| B2N084-01 | 636.23 | OEt | A02 | a1 | B15 | b1 | C02 | c2 | D09 |
| B2N085-01 | 637.22 | OEt | A01 | a1 | B08 | b1 | C02 | c2 | D09 |
| B2N086-01 | 638.21 | OEt | A02 | a1 | B08 | b1 | C02 | c2 | D09 |
| B2N087-01 | 645.18 | OEt | A01 | a1 | B11 | b1 | C02 | c2 | D09 |
| B2N088-01 | 646.18 | OEt | A02 | a1 | B11 | b1 | C02 | c2 | D09 |
| B2N089-01 | 661.18 | OEt | A01 | a1 | B13 | b1 | C02 | c2 | D09 |
| B2N090-01 | 662.18 | OEt | A02 | a1 | B13 | b1 | C02 | c2 | D09 |
| B2N091-01 | 627.21 | OEt | A01 | a1 | B09 | b1 | C02 | c2 | D09 |
| B2N092-01 | 628.21 | OEt | A02 | a1 | B09 | b1 | C02 | c2 | D09 |
| B2N093-01 | 583.15 | OEt | A01 | a1 | B10 | b1 | C02 | c2 | D09 |
| B2N094-01 | 584.15 | OEt | A02 | a1 | B10 | b1 | C02 | c2 | D09 |
| B2N095-01 | 601.2 | OEt | A01 | a2 | B01 | b1 | C02 | c2 | D09 |
| B2N096-01 | 602.19 | OEt | A02 | a2 | B01 | b1 | C02 | c2 | D09 |
| B2N097-01 | 589.22 | OEt | A01 | a1 | B03 | b2 | C01 | c2 | D09 |
| B2N098-01 | 590.22 | OEt | A02 | a1 | B03 | b2 | C01 | c2 | D09 |
| B2N099-01 | 607.21 | OEt | A01 | a1 | B18 | b2 | C01 | c2 | D09 |
| B2N100-01 | 608.21 | OEt | A02 | a1 | B18 | b2 | C01 | c2 | D09 |

[Table 260]

[Table AI-03-B2A-01]

| Mixture No. | mixAI-B2A-01 |
|---|---|
| No. of compound that may be contained | Compound name |
| B2A001-01 | N-(1-Adamantylmethylsulfonyl)-6-[4-[4-(3-ethoxyphenyl)thiophene-2-carbonyl]piperazin-1-yl]pyridazine-3-carboxamide |
| B2A002-01 | N-(1-Adamantylmethylsulfonyl)-6-[4-[4-(5-ethoxypyridin-3-yl)thiophene-2-carbonyl]piperazin-1-yl]pyridazine-3-carboxamide |
| B2A003-01 | N-(1-Adamantylmethylsulfonyl)-6-[4-[4-(3-ethoxyphenyl)-3-methylbenzoyl]piperazin-1-yl]pyridazine-3-carboxamide |
| B2A004-01 | N-(1-Adamantylmethylsulfonyl)-6-[4-[4-(5-ethoxypyridin-3-yl)-3-methylbenzoyl]piperazin-1-yl]pyridazine-3-carboxamide |
| B2A005-01 | N-(1-Adamantylmethylsulfonyl)-6-[4-[4-(3-ethoxyphenyl)-3-fluorobenzoyl]piperazin-1-yl]pyridazine-3-carboxamide |
| B2A006-01 | N-(1-Adamantylmethylsulfonyl)-6-[4-[4-(5-ethoxypyridin-3-yl)-3-fluorobenzoyl]piperazin-1-yl]pyridazine-3-carboxamide |
| B2A007-01 | N-(1-Adamantylmethylsulfonyl)-6-[4-[2-[2-(3-ethoxyphenyl)ethynyl]benzoyl]piperazin-1-yl]pyridazine-3-carboxamide |
| B2A008-01 | N-(1-Adamantylmethylsulfonyl)-6-[4-[2-[2-(5-ethoxypyridin-3-yl)ethynyl]benzoyl]piperazin-1-yl]pyridazine-3-carboxamide |
| B2A009-01 | N-(1-Adamantylmethylsulfonyl)-6-[4-[5-[2-(3-ethoxyphenyl)ethynyl]pyridine-3-carbonyl]piperazin-1-yl]pyridazine-3-carboxamide |
| B2A010-01 | N-(1-Adamantylmethylsulfonyl)-6-[4-[5-[2-(5-ethoxypyridin-3-yl)ethynyl]pyridine-3-carbonyl]piperazin-1-yl]pyridazine-3-carboxamide |
| B2A011-01 | N-(1-Adamantylmethylsulfonyl)-6-[4-[3-[2-(3-ethoxyphenyl)phenyl]propanoyl]piperazin-1-yl]pyridazine-3-carboxamide |
| B2A012-01 | N-(1-Adamantylmethylsulfonyl)-6-[4-[3-[2-(5-ethoxypyridin-3-yl)phenyl]propanoyl]piperazin-1-yl]pyridazine-3-carboxamide |
| B2A013-01 | N-(1-Adamantylmethylsulfonyl)-6-[4-[4-(3-ethoxyphenyl)naphthalene-1-carbonyl]piperazin-1-yl]pyridazine-3-carboxamide |
| B2A014-01 | N-(1-Adamantylmethylsulfonyl)-6-[4-[4-(5-ethoxypyridin-3-yl)naphthalene-1-carbonyl]piperazin-1-yl]pyridazine-3-carboxamide |
| B2A015-01 | N-(1-Adamantylmethylsulfonyl)-6-[4-[3-tert-butyl-5-(3-ethoxyphenyl)benzoyl]piperazin-1-yl]pyridazine-3-carboxamide |
| B2A016-01 | N-(1-Adamantylmethylsulfonyl)-6-[4-[3-tert-butyl-5-(5-ethoxypyridin-3-yl)benzoyl]piperazin-1-yl]pyridazine-3-carboxamide |
| B2A017-01 | N-(1-Adamantylmethylsulfonyl)-6-[4-[3-(3-ethoxyphenyl)-5-(trifluoromethyl)benzoyl]piperazin-1-yl]pyridazine-3-carboxamide |
| B2A018-01 | N-(1-Adamantylmethylsulfonyl)-6-[4-[3-(5-ethoxypyridin-3-yl)-5-(trifluoromethyl)benzoyl]piperazin-1-yl]pyridazine-3-carboxamide |
| B2A019-01 | N-(1-Adamantylmethylsulfonyl)-6-[4-[3-[2-(3-ethoxyphenyl)ethynyl]-5-(trifluoromethyl)benzoyl]piperazin-1-yl]pyridazine-3-carboxamide |
| B2A020-01 | N-(1-Adamantylmethylsulfonyl)-6-[4-[3-[2-(5-ethoxypyridin-3-yl)ethynyl]-5-(trifluoromethyl)benzoyl]piperazin-1-yl]pyridazine-3-carboxamide |
| B2A021-01 | N-(1-Adamantylmethylsulfonyl)-6-[4-[3-[4-(3-ethoxyphenyl)pyrazol-1-yl]-5-(trifluoromethyl)benzoyl]piperazin-1-yl]pyridazine-3-carboxamide |
| B2A022-01 | N-(1-Adamantylmethylsulfonyl)-6-[4-[3-[4-(5-ethoxypyridin-3-yl)pyrazol-1-yl]-5-(trifluoromethyl)benzoyl]piperazin-1-yl]pyridazine-3-carboxamide |
| B2A023-01 | N-(1-Adamantylmethylsulfonyl)-4-[4-[4-(3-ethoxyphenyl)thiophene-2-carbonyl]piperazin-1-yl]benzamide |
| B2A024-01 | N-(1-Adamantylmethylsulfonyl)-4-[4-[4-(5-ethoxypyridin-3-yl)thiophene-2-carbonyl]piperazin-1-yl]benzamide |

| | |
|---|---|
| B2A025-01 | N-(1-Adamantylmethylsulfonyl)-4-[4-[4-(3-ethoxyphenyl)-3-methylbenzoyl]piperazin-1-yl]benzamide |
| B2A026-01 | N-(1-Adamantylmethylsulfonyl)-4-[4-[4-(5-ethoxypyridin-3-yl)-3-methylbenzoyl]piperazin-1-yl]benzamide |
| B2A027-01 | N-(1-Adamantylmethylsulfonyl)-4-[4-[4-(3-ethoxyphenyl)-3-fluorobenzoyl]piperazin-1-yl]benzamide |
| B2A028-01 | N-(1-Adamantylmethylsulfonyl)-4-[4-[4-(5-ethoxypyridin-3-yl)-3-fluorobenzoyl]piperazin-1-yl]benzamide |
| B2A029-01 | N-(1-Adamantylmethylsulfonyl)-4-[4-[2-[2-(3-ethoxyphenyl)ethynyl]benzoyl]piperazin-1-yl]benzamide |
| B2A030-01 | N-(1-Adamantylmethylsulfonyl)-4-[4-[2-[2-(5-ethoxypyridin-3-yl)ethynyl]benzoyl]piperazin-1-yl]benzamide |
| B2A031-01 | N-(1-Adamantylmethylsulfonyl)-4-[4-[5-[2-(3-ethoxyphenyl)ethynyl]pyridine-3-carbonyl]piperazin-1-yl]benzamide |
| B2A032-01 | N-(1-Adamantylmethylsulfonyl)-4-[4-[5-[2-(5-ethoxypyridin-3-yl)ethynyl]pyridine-3-carbonyl]piperazin-1-yl]benzamide |
| B2A033-01 | N-(1-Adamantylmethylsulfonyl)-4-[4-[3-[2-(3-ethoxyphenyl)phenyl]propanoyl]piperazin-1-yl]benzamide |
| B2A034-01 | N-(1-Adamantylmethylsulfonyl)-4-[4-[3-[2-(5-ethoxypyridin-3-yl)phenyl]propanoyl]piperazin-1-yl]benzamide |
| B2A035-01 | N-(1-Adamantylmethylsulfonyl)-4-[4-[4-(3-ethoxyphenyl)naphthalene-1-carbonyl]piperazin-1-yl]benzamide |
| B2A036-01 | N-(1-Adamantylmethylsulfonyl)-4-[4-[4-(5-ethoxypyridin-3-yl)naphthalene-1-carbonyl]piperazin-1-yl]benzamide |
| B2A037-01 | N-(1-Adamantylmethylsulfonyl)-4-[4-[3-tert-butyl-5-(3-ethoxyphenyl)benzoyl]piperazin-1-yl]benzamide |
| B2A038-01 | N-(1-Adamantylmethylsulfonyl)-4-[4-[3-tert-butyl-5-(5-ethoxypyridin-3-yl)benzoyl]piperazin-1-yl]benzamide |
| B2A039-01 | N-(1-Adamantylmethylsulfonyl)-4-[4-[3-(3-ethoxyphenyl)-5-(trifluoromethyl)benzoyl]piperazin-1-yl]benzamide |
| B2A040-01 | N-(1-Adamantylmethylsulfonyl)-4-[4-[3-(5-ethoxypyridin-3-yl)-5-(trifluoromethyl)benzoyl]piperazin-1-yl]benzamide |
| B2A041-01 | N-(1-Adamantylmethylsulfonyl)-4-[4-[3-[2-(3-ethoxyphenyl)ethynyl]-5-(trifluoromethyl)benzoyl]piperazin-1-yl]benzamide |
| B2A042-01 | N-(1-Adamantylmethylsulfonyl)-4-[4-[3-[2-(5-ethoxypyridin-3-yl)ethynyl]-5-(trifluoromethyl)benzoyl]piperazin-1-yl]benzamide |
| B2A043-01 | N-(1-Adamantylmethylsulfonyl)-4-[4-[3-[4-(3-ethoxyphenyl)pyrazol-1-yl]-5-(trifluoromethyl)benzoyl]piperazin-1-yl]benzamide |
| B2A044-01 | N-(1-Adamantylmethylsulfonyl)-4-[4-[3-[4-(5-ethoxypyridin-3-yl)pyrazol-1-yl]-5-(trifluoromethyl)benzoyl]piperazin-1-yl]benzamide |
| B2A045-01 | N-(1-Adamantylmethylsulfonyl)-4-[4-[[2-(3-ethoxyphenyl)phenyl]methyl]piperazin-1-yl]benzamide |
| B2A046-01 | N-(1-Adamantylmethylsulfonyl)-4-[4-[[2-(5-ethoxypyridin-3-yl)phenyl]methyl]piperazin-1-yl]benzamide |
| B2A047-01 | N-(1-Adamantylmethylsulfonyl)-4-[4-[[4-(3-ethoxyphenyl)-3-methylphenyl]methyl]piperazin-1-yl]benzamide |
| B2A048-01 | N-(1-Adamantylmethylsulfonyl)-4-[4-[[4-(5-ethoxypyridin-3-yl)-3-methylphenyl]methyl]piperazin-1-yl]benzamide |
| B2A049-01 | N-(1-Adamantylmethylsulfonyl)-4-[4-[[4-(3-ethoxyphenyl)-2-ethylphenyl]methyl]piperazin-1-yl]benzamide |
| B2A050-01 | N-(1-Adamantylmethylsulfonyl)-4-[4-[[4-(5-ethoxypyridin-3-yl)-2-ethylphenyl]methyl]piperazin-1-yl]benzamide |
| B2A051-01 | N-(1-Adamantylmethylsulfonyl)-4-[4-[[4-(3-ethoxyphenyl)-3-fluorophenyl]methyl]piperazin-1-yl]benzamide |
| B2A052-01 | N-(1-Adamantylmethylsulfonyl)-4-[4-[[4-(5-ethoxypyridin-3-yl)-3-fluorophenyl]methyl]piperazin-1-yl]benzamide |

| | |
|---|---|
| B2A053-01 | N-(1-Adamantylmethylsulfonyl)-4-[4-[[4-(3-ethoxyphenyl)-2-methoxyphenyl]methyl]piperazin-1-yl]benzamide |
| B2A054-01 | N-(1-Adamantylmethylsulfonyl)-4-[4-[[4-(5-ethoxypyridin-3-yl)-2-methoxyphenyl]methyl]piperazin-1-yl]benzamide |
| B2A055-01 | N-(1-Adamantylmethylsulfonyl)-4-[4-[[3-ethoxy-5-(3-ethoxyphenyl)phenyl]methyl]piperazin-1-yl]benzamide |
| B2A056-01 | N-(1-Adamantylmethylsulfonyl)-4-[4-[[3-ethoxy-5-(5-ethoxypyridin-3-yl)phenyl]methyl]piperazin-1-yl]benzamide |
| B2A057-01 | N-(1-Adamantylmethylsulfonyl)-4-[4-[[3-(3-ethoxyphenyl)-5-propan-2-yloxyphenyl]methyl]piperazin-1-yl]benzamide |
| B2A058-01 | N-(1-Adamantylmethylsulfonyl)-4-[4-[[3-(5-ethoxypyridin-3-yl)-5-propan-2-yloxyphenyl]methyl]piperazin-1-yl]benzamide |
| B2A059-01 | N-(1-Adamantylmethylsulfonyl)-4-[4-[[4-(3-ethoxyphenyl)-2,6-dimethoxyphenyl]methyl]piperazin-1-yl]benzamide |
| B2A060-01 | N-(1-Adamantylmethylsulfonyl)-4-[4-[[4-(5-ethoxypyridin-3-yl)-2,6-dimethoxyphenyl]methyl]piperazin-1-yl]benzamide |
| B2A061-01 | N-(1-Adamantylmethylsulfonyl)-4-[4-[[3-(3-ethoxyphenyl)-5-(trifluoromethyl)phenyl]methyl]piperazin-1-yl]benzamide |
| B2A062-01 | N-(1-Adamantylmethylsulfonyl)-4-[4-[[3-(5-ethoxypyridin-3-yl)-5-(trifluoromethyl)phenyl]methyl]piperazin-1-yl]benzamide |
| B2A063-01 | N-(1-Adamantylmethylsulfonyl)-4-[4-[[3-(3-ethoxyphenyl)-5-(trifluoromethoxy)phenyl]methyl]piperazin-1-yl]benzamide |
| B2A064-01 | N-(1-Adamantylmethylsulfonyl)-4-[4-[[3-(5-ethoxypyridin-3-yl)-5-(trifluoromethoxy)phenyl]methyl]piperazin-1-yl]benzamide |
| B2A065-01 | N-(1-Adamantylmethylsulfonyl)-4-[4-[[4-(3-ethoxyphenyl)naphthalen-1-yl]methyl]piperazin-1-yl]benzamide |
| B2A066-01 | N-(1-Adamantylmethylsulfonyl)-4-[4-[[4-(5-ethoxypyridin-3-yl)naphthalen-1-yl]methyl]piperazin-1-yl]benzamide |
| B2A067-01 | N-(1-Adamantylmethylsulfonyl)-4-[4-[[4-(3-ethoxyphenyl)thiophen-2-yl]methyl]piperazin-1-yl]benzamide |
| B2A068-01 | N-(1-Adamantylmethylsulfonyl)-4-[4-[[4-(5-ethoxypyridin-3-yl)thiophen-2-yl]methyl]piperazin-1-yl]benzamide |
| B2A069-01 | N-(1-Adamantylmethylsulfonyl)-4-[4-[[2-[2-(3-ethoxyphenyl)ethynyl]phenyl]methyl]piperazin-1-yl]benzamide |
| B2A070-01 | N-(1-Adamantylmethylsulfonyl)-4-[4-[[2-[2-(5-ethoxypyridin-3-yl)ethynyl]phenyl]methyl]piperazin-1-yl]benzamide |
| B2A071-01 | N-(1-Adamantylmethylsulfonyl)-6-[4-[[2-(3-ethoxyphenyl)phenyl]methyl]piperazin-1-yl]pyridazine-3-carboxamide |
| B2A072-01 | N-(1-Adamantylmethylsulfonyl)-6-[4-[[2-(5-ethoxypyridin-3-yl)phenyl]methyl]piperazin-1-yl]pyridazine-3-carboxamide |
| B2A073-01 | N-(1-Adamantylmethylsulfonyl)-6-[4-[[4-(3-ethoxyphenyl)-3-methylphenyl]methyl]piperazin-1-yl]pyridazine-3-carboxamide |
| B2A074-01 | N-(1-Adamantylmethylsulfonyl)-6-[4-[[4-(5-ethoxypyridin-3-yl)-3-methylphenyl]methyl]piperazin-1-yl]pyridazine-3-carboxamide |
| B2A075-01 | N-(1-Adamantylmethylsulfonyl)-6-[4-[[4-(3-ethoxyphenyl)-2-ethylphenyl]methyl]piperazin-1-yl]pyridazine-3-carboxamide |
| B2A076-01 | N-(1-Adamantylmethylsulfonyl)-6-[4-[[4-(5-ethoxypyridin-3-yl)-2-ethylphenyl]methyl]piperazin-1-yl]pyridazine-3-carboxamide |
| B2A077-01 | N-(1-Adamantylmethylsulfonyl)-6-[4-[[4-(3-ethoxyphenyl)-3-fluorophenyl]methyl]piperazin-1-yl]pyridazine-3-carboxamide |
| B2A078-01 | N-(1-Adamantylmethylsulfonyl)-6-[4-[[4-(5-ethoxypyridin-3-yl)-3-fluorophenyl]methyl]piperazin-1-yl]pyridazine-3-carboxamide |
| B2A079-01 | N-(1-Adamantylmethylsulfonyl)-6-[4-[[4-(3-ethoxyphenyl)-2-methoxyphenyl]methyl]piperazin-1-yl]pyridazine-3-carboxamide |
| B2A080-01 | N-(1-Adamantylmethylsulfonyl)-6-[4-[[4-(5-ethoxypyridin-3-yl)-2-methoxyphenyl]methyl]piperazin-1-yl]pyridazine-3-carboxamide |

| | |
|---|---|
| B2A081-01 | N-(1-Adamantylmethylsulfonyl)-6-[4-[[3-ethoxy-5-(3-ethoxyphenyl)phenyl]methyl]piperazin-1-yl]pyridazine-3-carboxamide |
| B2A082-01 | N-(1-Adamantylmethylsulfonyl)-6-[4-[[3-ethoxy-5-(5-ethoxypyridin-3-yl)phenyl]methyl]piperazin-1-yl]pyridazine-3-carboxamide |
| B2A083-01 | N-(1-Adamantylmethylsulfonyl)-6-[4-[[3-(3-ethoxyphenyl)-5-propan-2-yloxyphenyl]methyl]piperazin-1-yl]pyridazine-3-carboxamide |
| B2A084-01 | N-(1-Adamantylmethylsulfonyl)-6-[4-[[3-(5-ethoxypyridin-3-yl)-5-propan-2-yloxyphenyl]methyl]piperazin-1-yl]pyridazine-3-carboxamide |
| B2A085-01 | N-(1-Adamantylmethylsulfonyl)-6-[4-[[4-(3-ethoxyphenyl)-2,6-dimethoxyphenyl]methyl]piperazin-1-yl]pyridazine-3-carboxamide |
| B2A086-01 | N-(1-Adamantylmethylsulfonyl)-6-[4-[[4-(5-ethoxypyridin-3-yl)-2,6-dimethoxyphenyl]methyl]piperazin-1-yl]pyridazine-3-carboxamide |
| B2A087-01 | N-(1-Adamantylmethylsulfonyl)-6-[4-[[3-(3-ethoxyphenyl)-5-(trifluoromethyl)phenyl]methyl]piperazin-1-yl]pyridazine-3-carboxamide |
| B2A088-01 | N-(1-Adamantylmethylsulfonyl)-6-[4-[[3-(5-ethoxypyridin-3-yl)-5-(trifluoromethyl)phenyl]methyl]piperazin-1-yl]pyridazine-3-carboxamide |
| B2A089-01 | N-(1-Adamantylmethylsulfonyl)-6-[4-[[3-(3-ethoxyphenyl)-5-(trifluoromethoxy)phenyl]methyl]piperazin-1-yl]pyridazine-3-carboxamide |
| B2A090-01 | N-(1-Adamantylmethylsulfonyl)-6-[4-[[3-(5-ethoxypyridin-3-yl)-5-(trifluoromethoxy)phenyl]methyl]piperazin-1-yl]pyridazine-3-carboxamide |
| B2A091-01 | N-(1-Adamantylmethylsulfonyl)-6-[4-[[4-(3-ethoxyphenyl)naphthalen-1-yl]methyl]piperazin-1-yl]pyridazine-3-carboxamide |
| B2A092-01 | N-(1-Adamantylmethylsulfonyl)-6-[4-[[4-(5-ethoxypyridin-3-yl)naphthalen-1-yl]methyl]piperazin-1-yl]pyridazine-3-carboxamide |
| B2A093-01 | N-(1-Adamantylmethylsulfonyl)-6-[4-[[4-(3-ethoxyphenyl)thiophen-2-yl]methyl]piperazin-1-yl]pyridazine-3-carboxamide |
| B2A094-01 | N-(1-Adamantylmethylsulfonyl)-6-[4-[[4-(5-ethoxypyridin-3-yl)thiophen-2-yl]methyl]piperazin-1-yl]pyridazine-3-carboxamide |
| B2A095-01 | N-(1-Adamantylmethylsulfonyl)-6-[4-[[2-[2-(3-ethoxyphenyl)ethynyl]phenyl]methyl]piperazin-1-yl]pyridazine-3-carboxamide |
| B2A096-01 | N-(1-Adamantylmethylsulfonyl)-6-[4-[[2-[2-(5-ethoxypyridin-3-yl)ethynyl]phenyl]methyl]piperazin-1-yl]pyridazine-3-carboxamide |
| B2A097-01 | N-(1-Adamantylmethylsulfonyl)-4-[4-[1-[4-(3-ethoxyphenyl)phenyl]ethyl]piperazin-1-yl]benzamide |
| B2A098-01 | N-(1-Adamantylmethylsulfonyl)-4-[4-[1-[4-(5-ethoxypyridin-3-yl)phenyl]ethyl]piperazin-1-yl]benzamide |
| B2A099-01 | N-(1-Adamantylmethylsulfonyl)-4-[4-[1-[4-(3-ethoxyphenyl)-2-fluorophenyl]ethyl]piperazin-1-yl]benzamide |
| B2A100-01 | N-(1-Adamantylmethylsulfonyl)-4-[4-[1-[4-(5-ethoxypyridin-3-yl)-2-fluorophenyl]ethyl]piperazin-1-yl]benzamide |

[Table 261]

[Table AI-03-B2A-01]

| Label | Exact | ☒ | A | a | B | b | C | c | D |
|---|---|---|---|---|---|---|---|---|---|
| B2A001-01 | 649.24 | OEt | A01 | a1 | B10 | b3 | C02 | c2 | D10 |
| B2A002-01 | 650.24 | OEt | A02 | a1 | B10 | b3 | C02 | c2 | D10 |
| B2A003-01 | 657.3 | OEt | A01 | a1 | B04 | b3 | C02 | c2 | D10 |
| B2A004-01 | 658.29 | OEt | A02 | a1 | B04 | b3 | C02 | c2 | D10 |
| B2A005-01 | 661.27 | OEt | A01 | a1 | B06 | b3 | C02 | c2 | D10 |
| B2A006-01 | 662.27 | OEt | A02 | a1 | B06 | b3 | C02 | c2 | D10 |
| B2A007-01 | 667.28 | OEt | A01 | a2 | B01 | b3 | C02 | c2 | D10 |
| B2A008-01 | 668.28 | OEt | A02 | a2 | B01 | b3 | C02 | c2 | D10 |
| B2A009-01 | 668.28 | OEt | A01 | a2 | B17 | b3 | C02 | c2 | D10 |

| | | | | | | | | | |
|---|---|---|---|---|---|---|---|---|---|
| B2A010-01 | 669.27 | OEt | A02 | a2 | B17 | b3 | C02 | c2 | D10 |
| B2A011-01 | 671.31 | OEt | A01 | a1 | B02 | b3 | C02 | c2 | D10 |
| B2A012-01 | 672.31 | OEt | A02 | a1 | B02 | b3 | C02 | c2 | D10 |
| B2A013-01 | 693.3 | OEt | A01 | a1 | B09 | b3 | C02 | c2 | D10 |
| B2A014-01 | 694.29 | OEt | A02 | a1 | B09 | b3 | C02 | c2 | D10 |
| B2A015-01 | 699.35 | OEt | A01 | a1 | B16 | b3 | C02 | c2 | D10 |
| B2A016-01 | 700.34 | OEt | A02 | a1 | B16 | b3 | C02 | c2 | D10 |
| B2A017-01 | 711.27 | OEt | A01 | a1 | B11 | b3 | C02 | c2 | D10 |
| B2A018-01 | 712.27 | OEt | A02 | a1 | B11 | b3 | C02 | c2 | D10 |
| B2A019-01 | 735.27 | OEt | A01 | a2 | B11 | b3 | C02 | c2 | D10 |
| B2A020-01 | 736.27 | OEt | A02 | a2 | B11 | b3 | C02 | c2 | D10 |
| B2A021-01 | 777.29 | OEt | A01 | a1 | B12 | b3 | C02 | c2 | D10 |
| B2A022-01 | 778.29 | OEt | A02 | a1 | B12 | b3 | C02 | c2 | D10 |
| B2A023-01 | 647.25 | OEt | A01 | a1 | B10 | b3 | C01 | c2 | D10 |
| B2A024-01 | 648.24 | OEt | A02 | a1 | B10 | b3 | C01 | c2 | D10 |
| B2A025-01 | 655.31 | OEt | A01 | a1 | B04 | b3 | C01 | c2 | D10 |
| B2A026-01 | 656.3 | OEt | A02 | a1 | B04 | b3 | C01 | c2 | D10 |
| B2A027-01 | 659.28 | OEt | A01 | a1 | B06 | b3 | C01 | c2 | D10 |
| B2A028-01 | 660.28 | OEt | A02 | a1 | B06 | b3 | C01 | c2 | D10 |
| B2A029-01 | 665.29 | OEt | A01 | a2 | B01 | b3 | C01 | c2 | D10 |
| B2A030-01 | 666.29 | OEt | A02 | a2 | B01 | b3 | C01 | c2 | D10 |
| B2A031-01 | 666.29 | OEt | A01 | a2 | B17 | b3 | C01 | c2 | D10 |
| B2A032-01 | 667.28 | OEt | A02 | a2 | B17 | b3 | C01 | c2 | D10 |
| B2A033-01 | 669.32 | OEt | A01 | a1 | B02 | b3 | C01 | c2 | D10 |
| B2A034-01 | 670.32 | OEt | A02 | a1 | B02 | b3 | C01 | c2 | D10 |
| B2A035-01 | 691.31 | OEt | A01 | a1 | B09 | b3 | C01 | c2 | D10 |
| B2A036-01 | 692.3 | OEt | A02 | a1 | B09 | b3 | C01 | c2 | D10 |
| B2A037-01 | 697.36 | OEt | A01 | a1 | B16 | b3 | C01 | c2 | D10 |
| B2A038-01 | 698.35 | OEt | A02 | a1 | B16 | b3 | C01 | c2 | D10 |
| B2A039-01 | 709.28 | OEt | A01 | a1 | B11 | b3 | C01 | c2 | D10 |
| B2A040-01 | 710.28 | OEt | A02 | a1 | B11 | b3 | C01 | c2 | D10 |
| B2A041-01 | 733.28 | OEt | A01 | a2 | B11 | b3 | C01 | c2 | D10 |
| B2A042-01 | 734.28 | OEt | A02 | a2 | B11 | b3 | C01 | c2 | D10 |
| B2A043-01 | 775.3 | OEt | A01 | a1 | B12 | b3 | C01 | c2 | D10 |
| B2A044-01 | 776.3 | OEt | A02 | a1 | B12 | b3 | C01 | c2 | D10 |
| B2A045-01 | 627.31 | OEt | A01 | a1 | B01 | b1 | C01 | c2 | D10 |
| B2A046-01 | 628.31 | OEt | A02 | a1 | B01 | b1 | C01 | c2 | D10 |
| B2A047-01 | 641.33 | OEt | A01 | a1 | B04 | b1 | C01 | c2 | D10 |
| B2A048-01 | 642.32 | OEt | A02 | a1 | B04 | b1 | C01 | c2 | D10 |
| B2A049-01 | 655.34 | OEt | A01 | a1 | B05 | b1 | C01 | c2 | D10 |
| B2A050-01 | 656.34 | OEt | A02 | a1 | B05 | b1 | C01 | c2 | D10 |
| B2A051-01 | 645.3 | OEt | A01 | a1 | B06 | b1 | C01 | c2 | D10 |
| B2A052-01 | 646.3 | OEt | A02 | a1 | B06 | b1 | C01 | c2 | D10 |
| B2A053-01 | 657.32 | OEt | A01 | a1 | B07 | b1 | C01 | c2 | D10 |
| B2A054-01 | 658.32 | OEt | A02 | a1 | B07 | b1 | C01 | c2 | D10 |
| B2A055-01 | 671.34 | OEt | A01 | a1 | B14 | b1 | C01 | c2 | D10 |
| B2A056-01 | 672.34 | OEt | A02 | a1 | B14 | b1 | C01 | c2 | D10 |

| | | | | | | | | | |
|---|---|---|---|---|---|---|---|---|---|
| B2A057-01 | 685.36 | OEt | A01 | a1 | B15 | b1 | C01 | c2 | D10 |
| B2A058-01 | 686.35 | OEt | A02 | a1 | B15 | b1 | C01 | c2 | D10 |
| B2A059-01 | 687.33 | OEt | A01 | a1 | B08 | b1 | C01 | c2 | D10 |
| B2A060-01 | 688.33 | OEt | A02 | a1 | B08 | b1 | C01 | c2 | D10 |
| B2A061-01 | 695.3 | OEt | A01 | a1 | B11 | b1 | C01 | c2 | D10 |
| B2A062-01 | 696.3 | OEt | A02 | a1 | B11 | b1 | C01 | c2 | D10 |
| B2A063-01 | 711.3 | OEt | A01 | a1 | B13 | b1 | C01 | c2 | D10 |
| B2A064-01 | 712.29 | OEt | A02 | a1 | B13 | b1 | C01 | c2 | D10 |
| B2A065-01 | 677.33 | OEt | A01 | a1 | B09 | b1 | C01 | c2 | D10 |
| B2A066-01 | 678.32 | OEt | A02 | a1 | B09 | b1 | C01 | c2 | D10 |
| B2A067-01 | 633.27 | OEt | A01 | a1 | B10 | b1 | C01 | c2 | D10 |
| B2A068-01 | 634.27 | OEt | A02 | a1 | B10 | b1 | C01 | c2 | D10 |
| B2A069-01 | 651.31 | OEt | A01 | a2 | B01 | b1 | C01 | c2 | D10 |
| B2A070-01 | 652.31 | OEt | A02 | a2 | B01 | b1 | C01 | c2 | D10 |
| B2A071-01 | 629.3 | OEt | A01 | a1 | B01 | b1 | C02 | c2 | D10 |
| B2A072-01 | 630.3 | OEt | A02 | a1 | B01 | b1 | C02 | c2 | D10 |
| B2A073-01 | 643.32 | OEt | A01 | a1 | B04 | b1 | C02 | c2 | D10 |
| B2A074-01 | 644.31 | OEt | A02 | a1 | B04 | b1 | C02 | c2 | D10 |
| B2A075-01 | 657.34 | OEt | A01 | a1 | B05 | b1 | C02 | c2 | D10 |
| B2A076-01 | 658.33 | OEt | A02 | a1 | B05 | b1 | C02 | c2 | D10 |
| B2A077-01 | 647.29 | OEt | A01 | a1 | B06 | b1 | C02 | c2 | D10 |
| B2A078-01 | 648.29 | OEt | A02 | a1 | B06 | b1 | C02 | c2 | D10 |
| B2A079-01 | 659.31 | OEt | A01 | a1 | B07 | b1 | C02 | c2 | D10 |
| B2A080-01 | 660.31 | OEt | A02 | a1 | B07 | b1 | C02 | c2 | D10 |
| B2A081-01 | 673.33 | OEt | A01 | a1 | B14 | b1 | C02 | c2 | D10 |
| B2A082-01 | 674.33 | OEt | A02 | a1 | B14 | b1 | C02 | c2 | D10 |
| B2A083-01 | 687.35 | OEt | A01 | a1 | B15 | b1 | C02 | c2 | D10 |
| B2A084-01 | 688.34 | OEt | A02 | a1 | B15 | b1 | C02 | c2 | D10 |
| B2A085-01 | 689.33 | OEt | A01 | a1 | B08 | b1 | C02 | c2 | D10 |
| B2A086-01 | 690.32 | OEt | A02 | a1 | B08 | b1 | C02 | c2 | D10 |
| B2A087-01 | 697.29 | OEt | A01 | a1 | B11 | b1 | C02 | c2 | D10 |
| B2A088-01 | 698.29 | OEt | A02 | a1 | B11 | b1 | C02 | c2 | D10 |
| B2A089-01 | 713.29 | OEt | A01 | a1 | B13 | b1 | C02 | c2 | D10 |
| B2A090-01 | 714.28 | OEt | A02 | a1 | B13 | b1 | C02 | c2 | D10 |
| B2A091-01 | 679.32 | OEt | A01 | a1 | B09 | b1 | C02 | c2 | D10 |
| B2A092-01 | 680.31 | OEt | A02 | a1 | B09 | b1 | C02 | c2 | D10 |
| B2A093-01 | 635.26 | OEt | A01 | a1 | B10 | b1 | C02 | c2 | D10 |
| B2A094-01 | 636.26 | OEt | A02 | a1 | B10 | b1 | C02 | c2 | D10 |
| B2A095-01 | 653.3 | OEt | A01 | a2 | B01 | b1 | C02 | c2 | D10 |
| B2A096-01 | 654.3 | OEt | A02 | a2 | B01 | b1 | C02 | c2 | D10 |
| B2A097-01 | 641.33 | OEt | A01 | a1 | B03 | b2 | C01 | c2 | D10 |
| B2A098-01 | 642.32 | OEt | A02 | a1 | B03 | b2 | C01 | c2 | D10 |
| B2A099-01 | 659.32 | OEt | A01 | a1 | B18 | b2 | C01 | c2 | D10 |
| B2A100-01 | 660.32 | OEt | A02 | a1 | B18 | b2 | C01 | c2 | D10 |

[Table 262]

[Table AI-03-B2R-01]

| Mixture No. | mixAI-B2R-01 |
|---|---|
| No. of compound that may be contained | Compound name |
| B2R001-01 | 6-[4-[4-(3-Ethoxyphenyl)thiophene-2-carbonyl]piperazin-1-yl]-N-[4-(2-phenylsulfanylethylamino)-3-(trifluoromethyl)phenyl]sulfonylpyridazine-3-carboxamide |
| B2R002-01 | 6-[4-[4-(5-Ethoxypyridin-3-yl)thiophene-2-carbonyl]piperazin-1-yl]-N-[4-(2-phenylsulfanylethylamino)-3-(trifluoromethyl)phenyl]sulfonylpyridazine-3-carboxamide |
| B2R003-01 | 6-[4-[4-(3-Ethoxyphenyl)-3-methylbenzoyl]piperazin-1-yl]-N-[4-(2-phenylsulfanylethylamino)-3-(trifluoromethyl)phenyl]sulfonylpyridazine-3-carboxamide |
| B2R004-01 | 6-[4-[4-(5-Ethoxypyridin-3-yl)-3-methylbenzoyl]piperazin-1-yl]-N-[4-(2-phenylsulfanylethylamino)-3-(trifluoromethyl)phenyl]sulfonylpyridazine-3-carboxamide |
| B2R005-01 | 6-[4-[4-(3-Ethoxyphenyl)-3-fluorobenzoyl]piperazin-1-yl]-N-[4-(2-phenylsulfanylethylamino)-3-(trifluoromethyl)phenyl]sulfonylpyridazine-3-carboxamide |
| B2R006-01 | 6-[4-[4-(5-Ethoxypyridin-3-yl)-3-fluorobenzoyl]piperazin-1-yl]-N-[4-(2-phenylsulfanylethylamino)-3-(trifluoromethyl)phenyl]sulfonylpyridazine-3-carboxamide |
| B2R007-01 | 6-[4-[2-[2-(3-Ethoxyphenyl)ethynyl]benzoyl]piperazin-1-yl]-N-[4-(2-phenylsulfanylethylamino)-3-(trifluoromethyl)phenyl]sulfonylpyridazine-3-carboxamide |
| B2R008-01 | 6-[4-[2-[2-(5-Ethoxypyridin-3-yl)ethynyl]benzoyl]piperazin-1-yl]-N-[4-(2-phenylsulfanylethylamino)-3-(trifluoromethyl)phenyl]sulfonylpyridazine-3-carboxamide |
| B2R009-01 | 6-[4-[5-[2-(3-Ethoxyphenyl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| B2R010-01 | 6-[4-[5-[2-(5-Ethoxypyridin-3-yl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| B2R011-01 | 6-[4-[3-[2-(3-Ethoxyphenyl)phenyl]propanoyl]piperazin-1-yl]-N-[4-(2-phenylsulfanylethylamino)-3-(trifluoromethyl)phenyl]sulfonylpyridazine-3-carboxamide |
| B2R012-01 | 6-[4-[3-[2-(5-Ethoxypyridin-3-yl)phenyl]propanoyl]piperazin-1-yl]-N-[4-(2-phenylsulfanylethylamino)-3-(trifluoromethyl)phenyl]sulfonylpyridazine-3-carboxamide |
| B2R013-01 | 6-[4-[4-(3-Ethoxyphenyl)naphthalene-1-carbonyl]piperazin-1-yl]-N-[4-(2-phenylsulfanylethylamino)-3-(trifluoromethyl)phenyl]sulfonylpyridazine-3-carboxamide |
| B2R014-01 | 6-[4-[4-(5-Ethoxypyridin-3-yl)naphthalene-1-carbonyl]piperazin-1-yl]-N-[4-(2-phenylsulfanylethylamino)-3-(trifluoromethyl)phenyl]sulfonylpyridazine-3-carboxamide |
| B2R015-01 | 6-[4-[3-tert-Butyl-5-(3-ethoxyphenyl)benzoyl]piperazin-1-yl]-N-[4-(2-phenylsulfanylethylamino)-3-(trifluoromethyl)phenyl]sulfonylpyridazine-3-carboxamide |
| B2R016-01 | 6-[4-[3-tert-Butyl-5-(5-ethoxypyridin-3-yl)benzoyl]piperazin-1-yl]-N-[4-(2-phenylsulfanylethylamino)-3-(trifluoromethyl)phenyl]sulfonylpyridazine-3-carboxamide |

| | |
|---|---|
| B2R017-01 | 6-[4-[3-(3-Ethoxyphenyl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R018-01 | 6-[4-[3-(5-Ethoxypyridin-3-yl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R019-01 | 6-[4-[3-[2-(3-Ethoxyphenyl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R020-01 | 6-[4-[3-[2-(5-Ethoxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R021-01 | 6-[4-[3-[4-(3-Ethoxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R022-01 | 6-[4-[3-[4-(5-Ethoxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R023-01 | 4-[4-[4-(3-Ethoxyphenyl)thiophene-2-carbonyl]piperazin-1-yl]-N-[4-(2-phenylsulfanylethylamino)-3-(trifluoromethyl)phenyl]sulfonylbenzamide |
| B2R024-01 | 4-[4-[4-(5-Ethoxypyridin-3-yl)thiophene-2-carbonyl]piperazin-1-yl]-N-[4-(2-phenylsulfanylethylamino)-3-(trifluoromethyl)phenyl]sulfonylbenzamide |
| B2R025-01 | 4-[4-[4-(3-Ethoxyphenyl)-3-methylbenzoyl]piperazin-1-yl]-N-[4-(2-phenylsulfanylethylamino)-3-(trifluoromethyl)phenyl]sulfonylbenzamide |
| B2R026-01 | 4-[4-[4-(5-Ethoxypyridin-3-yl)-3-methylbenzoyl]piperazin-1-yl]-N-[4-(2-phenylsulfanylethylamino)-3-(trifluoromethyl)phenyl]sulfonylbenzamide |
| B2R027-01 | 4-[4-[4-(3-Ethoxyphenyl)-3-fluorobenzoyl]piperazin-1-yl]-N-[4-(2-phenylsulfanylethylamino)-3-(trifluoromethyl)phenyl]sulfonylbenzamide |
| B2R028-01 | 4-[4-[4-(5-Ethoxypyridin-3-yl)-3-fluorobenzoyl]piperazin-1-yl]-N-[4-(2-phenylsulfanylethylamino)-3-(trifluoromethyl)phenyl]sulfonylbenzamide |
| B2R029-01 | 4-[4-[2-[2-(3-Ethoxyphenyl)ethynyl]benzoyl]piperazin-1-yl]-N-[4-(2-phenylsulfanylethylamino)-3-(trifluoromethyl)phenyl]sulfonylbenzamide |
| B2R030-01 | 4-[4-[2-[2-(5-Ethoxypyridin-3-yl)ethynyl]benzoyl]piperazin-1-yl]-N-[4-(2-phenylsulfanylethylamino)-3-(trifluoromethyl)phenyl]sulfonylbenzamide |
| B2R031-01 | 4-[4-[5-[2-(3-Ethoxyphenyl)ethynyl]pyridine-3-carbonyl]piperazin-1-yl]-N-[4-(2-phenylsulfanylethylamino)-3-(trifluoromethyl)phenyl]sulfonylbenzamide |
| B2R032-01 | 4-[4-[5-[2-(5-Ethoxypyridin-3-yl)ethynyl]pyridine-3-carbonyl]piperazin-1-yl]-N-[4-(2-phenylsulfanylethylamino)-3-(trifluoromethyl)phenyl]sulfonylbenzamide |
| B2R033-01 | 4-[4-[3-[2-(3-Ethoxyphenyl)phenyl]propanoyl]piperazin-1-yl]-N-[4-(2-phenylsulfanylethylamino)-3-(trifluoromethyl)phenyl]sulfonylbenzamide |
| B2R034-01 | 4-[4-[3-[2-(5-Ethoxypyridin-3-yl)phenyl]propanoyl]piperazin-1-yl]-N-[4-(2-phenylsulfanylethylamino)-3-(trifluoromethyl)phenyl]sulfonylbenzamide |
| B2R035-01 | 4-[4-[4-(3-Ethoxyphenyl)naphthalene-1-carbonyl]piperazin-1-yl]-N-[4-(2-phenylsulfanylethylamino)-3-(trifluoromethyl)phenyl]sulfonylbenzamide |
| B2R036-01 | 4-[4-[4-(5-Ethoxypyridin-3-yl)naphthalene-1-carbonyl]piperazin-1-yl]-N-[4-(2-phenylsulfanylethylamino)-3-(trifluoromethyl)phenyl]sulfonylbenzamide |
| B2R037-01 | 4-[4-[3-tert-Butyl-5-(3-ethoxyphenyl)benzoyl]piperazin-1-yl]-N-[4-(2-phenylsulfanylethylamino)-3-(trifluoromethyl)phenyl]sulfonylbenzamide |
| B2R038-01 | 4-[4-[3-tert-Butyl-5-(5-ethoxypyridin-3-yl)benzoyl]piperazin-1-yl]-N-[4-(2-phenylsulfanylethylamino)-3-(trifluoromethyl)phenyl]sulfonylbenzamide |
| B2R039-01 | 4-[4-[3-(3-Ethoxyphenyl)-5-(trifluoromethyl)benzoyl]piperazin-1-yl]-N-[4-(2-phenylsulfanylethylamino)-3-(trifluoromethyl)phenyl]sulfonylbenzamide |
| B2R040-01 | 4-[4-[3-(5-Ethoxypyridin-3-yl)-5-(trifluoromethyl)benzoyl]piperazin-1-yl]-N-[4-(2-phenylsulfanylethylamino)-3-(trifluoromethyl)phenyl]sulfonylbenzamide |
| B2R041-01 | 4-[4-[3-[2-(3-Ethoxyphenyl)ethynyl]-5-(trifluoromethyl)benzoyl]piperazin-1-yl]-N-[4-(2-phenylsulfanylethylamino)-3-(trifluoromethyl)phenyl]sulfonylbenzamide |

| | |
|---|---|
| B2R042-01 | 4-[4-[3-[2-(5-Ethoxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| B2R043-01 | 4-[4-[3-[4-(3-Ethoxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |
| B2R044-01 | 4-[4-[3-[4-(5-Ethoxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |
| B2R045-01 | 4-[4-[[2-(3-Ethoxyphenyl)phenyl]methyl]piperazin-1-yl]-N-[4-(2-phenylsulfanylethylamino)-3-(trifluoromethyl)phenyl]sulfonylbenzamide |
| B2R046-01 | 4-[4-[[2-(5-Ethoxypyridin-3-yl)phenyl]methyl]piperazin-1-yl]-N-[4-(2-phenylsulfanylethylamino)-3-(trifluoromethyl)phenyl]sulfonylbenzamide |
| B2R047-01 | 4-[4-[[4-(3-Ethoxyphenyl)-3-methylphenyl]methyl]piperazin-1-yl]-N-[4-(2-phenylsulfanylethylamino)-3-(trifluoromethyl)phenyl]sulfonylbenzamide |
| B2R048-01 | 4-[4-[[4-(5-Ethoxypyridin-3-yl)-3-methylphenyl]methyl]piperazin-1-yl]-N-[4-(2-phenylsulfanylethylamino)-3-(trifluoromethyl)phenyl]sulfonylbenzamide |
| B2R049-01 | 4-[4-[[4-(3-Ethoxyphenyl)-2-ethylphenyl]methyl]piperazin-1-yl]-N-[4-(2-phenylsulfanylethylamino)-3-(trifluoromethyl)phenyl]sulfonylbenzamide |
| B2R050-01 | 4-[4-[[4-(5-Ethoxypyridin-3-yl)-2-ethylphenyl]methyl]piperazin-1-yl]-N-[4-(2-phenylsulfanylethylamino)-3-(trifluoromethyl)phenyl]sulfonylbenzamide |
| B2R051-01 | 4-[4-[[4-(3-Ethoxyphenyl)-3-fluorophenyl]methyl]piperazin-1-yl]-N-[4-(2-phenylsulfanylethylamino)-3-(trifluoromethyl)phenyl]sulfonylbenzamide |
| B2R052-01 | 4-[4-[[4-(5-Ethoxypyridin-3-yl)-3-fluorophenyl]methyl]piperazin-1-yl]-N-[4-(2-phenylsulfanylethylamino)-3-(trifluoromethyl)phenyl]sulfonylbenzamide |
| B2R053-01 | 4-[4-[[4-(3-Ethoxyphenyl)-2-methoxyphenyl]methyl]piperazin-1-yl]-N-[4-(2-phenylsulfanylethylamino)-3-(trifluoromethyl)phenyl]sulfonylbenzamide |
| B2R054-01 | 4-[4-[[4-(5-Ethoxypyridin-3-yl)-2-methoxyphenyl]methyl]piperazin-1-yl]-N-[4-(2-phenylsulfanylethylamino)-3-(trifluoromethyl)phenyl]sulfonylbenzamide |
| B2R055-01 | 4-[4-[[3-Ethoxy-5-(3-ethoxyphenyl)phenyl]methyl]piperazin-1-yl]-N-[4-(2-phenylsulfanylethylamino)-3-(trifluoromethyl)phenyl]sulfonylbenzamide |
| B2R056-01 | 4-[4-[[3-Ethoxy-5-(5-ethoxypyridin-3-yl)phenyl]methyl]piperazin-1-yl]-N-[4-(2-phenylsulfanylethylamino)-3-(trifluoromethyl)phenyl]sulfonylbenzamide |
| B2R057-01 | 4-[4-[[3-(3-Ethoxyphenyl)-5-propan-2-yloxyphenyl]methyl]piperazin-1-yl]-N-[4-(2-phenylsulfanylethylamino)-3-(trifluoromethyl)phenyl]sulfonylbenzamide |
| B2R058-01 | 4-[4-[[3-(5-Ethoxypyridin-3-yl)-5-propan-2-yloxyphenyl]methyl]piperazin-1-yl]-N-[4-(2-phenylsulfanylethylamino)-3-(trifluoromethyl)phenyl]sulfonylbenzamide |
| B2R059-01 | 4-[4-[[4-(3-Ethoxyphenyl)-2,6-dimethoxyphenyl]methyl]piperazin-1-yl]-N-[4-(2-phenylsulfanylethylamino)-3-(trifluoromethyl)phenyl]sulfonylbenzamide |
| B2R060-01 | 4-[4-[[4-(5-Ethoxypyridin-3-yl)-2,6-dimethoxyphenyl]methyl]piperazin-1-yl]-N-[4-(2-phenylsulfanylethylamino)-3-(trifluoromethyl)phenyl]sulfonylbenzamide |
| B2R061-01 | 4-[4-[[3-(3-Ethoxyphenyl)-5-(trifluoromethyl)phenyl]methyl]piperazin-1-yl]-N-[4-(2-phenylsulfanylethylamino)-3-(trifluoromethyl)phenyl]sulfonylbenzamide |
| B2R062-01 | 4-[4-[[3-(5-Ethoxypyridin-3-yl)-5-(trifluoromethyl)phenyl]methyl]piperazin-1-yl]-N-[4-(2-phenylsulfanylethylamino)-3-(trifluoromethyl)phenyl]sulfonylbenzamide |
| B2R063-01 | 4-[4-[[3-(3-Ethoxyphenyl)-5-(trifluoromethoxy)phenyl]methyl]piperazin-1-yl]-N-[4-(2-phenylsulfanylethylamino)-3-(trifluoromethyl)phenyl]sulfonylbenzamide |
| B2R064-01 | 4-[4-[[3-(5-Ethoxypyridin-3-yl)-5-(trifluoromethoxy)phenyl]methyl]piperazin-1-yl]-N-[4-(2-phenylsulfanylethylamino)-3-(trifluoromethyl)phenyl]sulfonylbenzamide |
| B2R065-01 | 4-[4-[[4-(3-Ethoxyphenyl)naphthalen-1-yl]methyl]piperazin-1-yl]-N-[4-(2-phenylsulfanylethylamino)-3-(trifluoromethyl)phenyl]sulfonylbenzamide |
| B2R066-01 | 4-[4-[[4-(5-Ethoxypyridin-3-yl)naphthalen-1-yl]methyl]piperazin-1-yl]-N-[4-(2-phenylsulfanylethylamino)-3-(trifluoromethyl)phenyl]sulfonylbenzamide |
| B2R067-01 | 4-[4-[[4-(3-Ethoxyphenyl)thiophen-2-yl]methyl]piperazin-1-yl]-N-[4-(2-phenylsulfanylethylamino)-3-(trifluoromethyl)phenyl]sulfonylbenzamide |
| B2R068-01 | 4-[4-[[4-(5-Ethoxypyridin-3-yl)thiophen-2-yl]methyl]piperazin-1-yl]-N-[4-(2-phenylsulfanylethylamino)-3-(trifluoromethyl)phenyl]sulfonylbenzamide |
| B2R069-01 | 4-[4-[[2-[2-(3-Ethoxyphenyl)ethynyl]phenyl]methyl]piperazin-1-yl]-N-[4-(2-phenylsulfanylethylamino)-3-(trifluoromethyl)phenyl]sulfonylbenzamide |

| | |
|---|---|
| B2R070-01 | 4-[4-[[2-[2-(5-Ethoxypyridin-3-yl)ethynyl]phenyl]methyl]piperazin-1-yl]-N-[4-(2-phenylsulfanylethylamino)-3-(trifluoromethyl)phenyl]sulfonylbenzamide |
| B2R071-01 | 6-[4-[[2-(3-Ethoxyphenyl)phenyl]methyl]piperazin-1-yl]-N-[4-(2-phenylsulfanylethylamino)-3-(trifluoromethyl)phenyl]sulfonylpyridazine-3-carboxamide |
| B2R072-01 | 6-[4-[[2-(5-Ethoxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| B2R073-01 | 6-[4-[[4-(3-Ethoxyphenyl)-3-methylphenyl]methyl]piperazin-1-yl]-N-[4-(2-phenylsulfanylethylamino)-3-(trifluoromethyl)phenyl]sulfonylpyridazine-3-carboxamide |
| B2R074-01 | 6-[4-[[4-(5-Ethoxypyridin-3-yl)-3-methylphenyl]methyl]piperazin-1-yl]-N-[4-(2-phenylsulfanylethylamino)-3-(trifluoromethyl)phenyl]sulfonylpyridazine-3-carboxamide |
| B2R075-01 | 6-[4-[[4-(3-Ethoxyphenyl)-2-ethylphenyl]methyl]piperazin-1-yl]-N-[4-(2-phenylsulfanylethylamino)-3-(trifluoromethyl)phenyl]sulfonylpyridazine-3-carboxamide |
| B2R076-01 | 6-[4-[[4-(5-Ethoxypyridin-3-yl)-2-ethylphenyl]methyl]piperazin-1-yl]-N-[4-(2-phenylsulfanylethylamino)-3-(trifluoromethyl)phenyl]sulfonylpyridazine-3-carboxamide |
| B2R077-01 | 6-[4-[[4-(3-Ethoxyphenyl)-3-fluorophenyl]methyl]piperazin-1-yl]-N-[4-(2-phenylsulfanylethylamino)-3-(trifluoromethyl)phenyl]sulfonylpyridazine-3-carboxamide |
| B2R078-01 | 6-[4-[[4-(5-Ethoxypyridin-3-yl)-3-fluorophenyl]methyl]piperazin-1-yl]-N-[4-(2-phenylsulfanylethylamino)-3-(trifluoromethyl)phenyl]sulfonylpyridazine-3-carboxamide |
| B2R079-01 | 6-[4-[[4-(3-Ethoxyphenyl)-2-methoxyphenyl]methyl]piperazin-1-yl]-N-[4-(2-phenylsulfanylethylamino)-3-(trifluoromethyl)phenyl]sulfonylpyridazine-3-carboxamide |
| B2R080-01 | 6-[4-[[4-(5-Ethoxypyridin-3-yl)-2-methoxyphenyl]methyl]piperazin-1-yl]-N-[4-(2-phenylsulfanylethylamino)-3-(trifluoromethyl)phenyl]sulfonylpyridazine-3-carboxamide |
| B2R081-01 | 6-[4-[[3-Ethoxy-5-(3-ethoxyphenyl)phenyl]methyl]piperazin-1-yl]-N-[4-(2-phenylsulfanylethylamino)-3-(trifluoromethyl)phenyl]sulfonylpyridazine-3-carboxamide |
| B2R082-01 | 6-[4-[[3-Ethoxy-5-(5-ethoxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| B2R083-01 | 6-[4-[[3-(3-Ethoxyphenyl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| B2R084-01 | 6-[4-[[3-(5-Ethoxypyridin-3-yl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| B2R085-01 | 6-[4-[[4-(3-Ethoxyphenyl)-2,6-dimethoxyphenyl]methyl]piperazin-1-yl]-N-[4-(2-phenylsulfanylethylamino)-3-(trifluoromethyl)phenyl]sulfonylpyridazine-3-carboxamide |
| B2R086-01 | 6-[4-[[4-(5-Ethoxypyridin-3-yl)-2,6-dimethoxyphenyl]methyl]piperazin-1-yl]-N-[4-(2-phenylsulfanylethylamino)-3-(trifluoromethyl)phenyl]sulfonylpyridazine-3-carboxamide |
| B2R087-01 | 6-[4-[[3-(3-Ethoxyphenyl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| B2R088-01 | 6-[4-[[3-(5-Ethoxypyridin-3-yl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |

| | |
|---|---|
| B2R089-01 | 6-[4-[[3-(3-Ethoxyphenyl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| B2R090-01 | 6-[4-[[3-(5-Ethoxypyridin-3-yl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| B2R091-01 | 6-[4-[[4-(3-Ethoxyphenyl)naphthalen-1-yl]methyl]piperazin-1-yl]-N-[4-(2-phenylsulfanylethylamino)-3-(trifluoromethyl)phenyl]sulfonylpyridazine-3-carboxamide |
| B2R092-01 | 6-[4-[[4-(5-Ethoxypyridin-3-yl)naphthalen-1-yl]methyl]piperazin-1-yl]-N-[4-(2-phenylsulfanylethylamino)-3-(trifluoromethyl)phenyl]sulfonylpyridazine-3-carboxamide |
| B2R093-01 | 6-[4-[[4-(3-Ethoxyphenyl)thiophen-2-yl]methyl]piperazin-1-yl]-N-[4-(2-phenylsulfanylethylamino)-3-(trifluoromethyl)phenyl]sulfonylpyridazine-3-carboxamide |
| B2R094-01 | 6-[4-[[4-(5-Ethoxypyridin-3-yl)thiophen-2-yl]methyl]piperazin-1-yl]-N-[4-(2-phenylsulfanylethylamino)-3-(trifluoromethyl)phenyl]sulfonylpyridazine-3-carboxamide |
| B2R095-01 | 6-[4-[[2-[2-(3-Ethoxyphenyl)ethynyl]phenyl]methyl]piperazin-1-yl]-N-[4-(2-phenylsulfanylethylamino)-3-(trifluoromethyl)phenyl]sulfonylpyridazine-3-carboxamide |
| B2R096-01 | 6-[4-[[2-[2-(5-Ethoxypyridin-3-yl)ethynyl]phenyl]methyl]piperazin-1-yl]-N-[4-(2-phenylsulfanylethylamino)-3-(trifluoromethyl)phenyl]sulfonylpyridazine-3-carboxamide |
| B2R097-01 | 4-[4-[1-[4-(3-Ethoxyphenyl)phenyl]ethyl]piperazin-1-yl]-N-[4-(2-phenylsulfanylethylamino)-3-(trifluoromethyl)phenyl]sulfonylbenzamide |
| B2R098-01 | 4-[4-[1-[4-(5-Ethoxypyridin-3-yl)phenyl]ethyl]piperazin-1-yl]-N-[4-(2-phenylsulfanylethylamino)-3-(trifluoromethyl)phenyl]sulfonylbenzamide |
| B2R099-01 | 4-[4-[1-[4-(3-Ethoxyphenyl)-2-fluorophenyl]ethyl]piperazin-1-yl]-N-[4-(2-phenylsulfanylethylamino)-3-(trifluoromethyl)phenyl]sulfonylbenzamide |
| B2R100-01 | 4-[4-[1-[4-(5-Ethoxypyridin-3-yl)-2-fluorophenyl]ethyl]piperazin-1-yl]-N-[4-(2-phenylsulfanylethylamino)-3-(trifluoromethyl)phenyl]sulfonylbenzamide |

[Table 263]

[Table AI-03-B2R-01]

| Label | Exact | ☒ | A | a | B | b | C | c | D |
|---|---|---|---|---|---|---|---|---|---|
| B2R001-01 | 796.18 | OEt | A01 | a1 | B10 | b3 | C02 | c2 | D12 |
| B2R002-01 | 797.17 | OEt | A02 | a1 | B10 | b3 | C02 | c2 | D12 |
| B2R003-01 | 804.24 | OEt | A01 | a1 | B04 | b3 | C02 | c2 | D12 |
| B2R004-01 | 805.23 | OEt | A02 | a1 | B04 | b3 | C02 | c2 | D12 |
| B2R005-01 | 808.21 | OEt | A01 | a1 | B06 | b3 | C02 | c2 | D12 |
| B2R006-01 | 809.21 | OEt | A02 | a1 | B06 | b3 | C02 | c2 | D12 |
| B2R007-01 | 814.22 | OEt | A01 | a2 | B01 | b3 | C02 | c2 | D12 |
| B2R008-01 | 815.22 | OEt | A02 | a2 | B01 | b3 | C02 | c2 | D12 |
| B2R009-01 | 815.22 | OEt | A01 | a2 | B17 | b3 | C02 | c2 | D12 |
| B2R010-01 | 816.21 | OEt | A02 | a2 | B17 | b3 | C02 | c2 | D12 |
| B2R011-01 | 818.25 | OEt | A01 | a1 | B02 | b3 | C02 | c2 | D12 |
| B2R012-01 | 819.25 | OEt | A02 | a1 | B02 | b3 | C02 | c2 | D12 |
| B2R013-01 | 840.24 | OEt | A01 | a1 | B09 | b3 | C02 | c2 | D12 |
| B2R014-01 | 841.23 | OEt | A02 | a1 | B09 | b3 | C02 | c2 | D12 |
| B2R015-01 | 846.28 | OEt | A01 | a1 | B16 | b3 | C02 | c2 | D12 |
| B2R016-01 | 847.28 | OEt | A02 | a1 | B16 | b3 | C02 | c2 | D12 |

| | | | | | | | | | |
|---|---|---|---|---|---|---|---|---|---|
| B2R017-01 | 858.21 | OEt | A01 | a1 | B11 | b3 | C02 | c2 | D12 |
| B2R018-01 | 859.21 | OEt | A02 | a1 | B11 | b3 | C02 | c2 | D12 |
| B2R019-01 | 882.21 | OEt | A01 | a2 | B11 | b3 | C02 | c2 | D12 |
| B2R020-01 | 883.21 | OEt | A02 | a2 | B11 | b3 | C02 | c2 | D12 |
| B2R021-01 | 924.23 | OEt | A01 | a1 | B12 | b3 | C02 | c2 | D12 |
| B2R022-01 | 925.23 | OEt | A02 | a1 | B12 | b3 | C02 | c2 | D12 |
| B2R023-01 | 794.19 | OEt | A01 | a1 | B10 | b3 | C01 | c2 | D12 |
| B2R024-01 | 795.18 | OEt | A02 | a1 | B10 | b3 | C01 | c2 | D12 |
| B2R025-01 | 802.25 | OEt | A01 | a1 | B04 | b3 | C01 | c2 | D12 |
| B2R026-01 | 803.24 | OEt | A02 | a1 | B04 | b3 | C01 | c2 | D12 |
| B2R027-01 | 806.22 | OEt | A01 | a1 | B06 | b3 | C01 | c2 | D12 |
| B2R028-01 | 807.22 | OEt | A02 | a1 | B06 | b3 | C01 | c2 | D12 |
| B2R029-01 | 812.23 | OEt | A01 | a2 | B01 | b3 | C01 | c2 | D12 |
| B2R030-01 | 813.23 | OEt | A02 | a2 | B01 | b3 | C01 | c2 | D12 |
| B2R031-01 | 813.23 | OEt | A01 | a2 | B17 | b3 | C01 | c2 | D12 |
| B2R032-01 | 814.22 | OEt | A02 | a2 | B17 | b3 | C01 | c2 | D12 |
| B2R033-01 | 816.26 | OEt | A01 | a1 | B02 | b3 | C01 | c2 | D12 |
| B2R034-01 | 817.26 | OEt | A02 | a1 | B02 | b3 | C01 | c2 | D12 |
| B2R035-01 | 838.25 | OEt | A01 | a1 | B09 | b3 | C01 | c2 | D12 |
| B2R036-01 | 839.24 | OEt | A02 | a1 | B09 | b3 | C01 | c2 | D12 |
| B2R037-01 | 844.29 | OEt | A01 | a1 | B16 | b3 | C01 | c2 | D12 |
| B2R038-01 | 845.29 | OEt | A02 | a1 | B16 | b3 | C01 | c2 | D12 |
| B2R039-01 | 856.22 | OEt | A01 | a1 | B11 | b3 | C01 | c2 | D12 |
| B2R040-01 | 857.21 | OEt | A02 | a1 | B11 | b3 | C01 | c2 | D12 |
| B2R041-01 | 880.22 | OEt | A01 | a2 | B11 | b3 | C01 | c2 | D12 |
| B2R042-01 | 881.21 | OEt | A02 | a2 | B11 | b3 | C01 | c2 | D12 |
| B2R043-01 | 922.24 | OEt | A01 | a1 | B12 | b3 | C01 | c2 | D12 |
| B2R044-01 | 923.24 | OEt | A02 | a1 | B12 | b3 | C01 | c2 | D12 |
| B2R045-01 | 774.25 | OEt | A01 | a1 | B01 | b1 | C01 | c2 | D12 |
| B2R046-01 | 775.25 | OEt | A02 | a1 | B01 | b1 | C01 | c2 | D12 |
| B2R047-01 | 788.27 | OEt | A01 | a1 | B04 | b1 | C01 | c2 | D12 |
| B2R048-01 | 789.26 | OEt | A02 | a1 | B04 | b1 | C01 | c2 | D12 |
| B2R049-01 | 802.28 | OEt | A01 | a1 | B05 | b1 | C01 | c2 | D12 |
| B2R050-01 | 803.28 | OEt | A02 | a1 | B05 | b1 | C01 | c2 | D12 |
| B2R051-01 | 792.24 | OEt | A01 | a1 | B06 | b1 | C01 | c2 | D12 |
| B2R052-01 | 793.24 | OEt | A02 | a1 | B06 | b1 | C01 | c2 | D12 |
| B2R053-01 | 804.26 | OEt | A01 | a1 | B07 | b1 | C01 | c2 | D12 |
| B2R054-01 | 805.26 | OEt | A02 | a1 | B07 | b1 | C01 | c2 | D12 |
| B2R055-01 | 818.28 | OEt | A01 | a1 | B14 | b1 | C01 | c2 | D12 |
| B2R056-01 | 819.27 | OEt | A02 | a1 | B14 | b1 | C01 | c2 | D12 |
| B2R057-01 | 832.29 | OEt | A01 | a1 | B15 | b1 | C01 | c2 | D12 |
| B2R058-01 | 833.29 | OEt | A02 | a1 | B15 | b1 | C01 | c2 | D12 |
| B2R059-01 | 834.27 | OEt | A01 | a1 | B08 | b1 | C01 | c2 | D12 |
| B2R060-01 | 835.27 | OEt | A02 | a1 | B08 | b1 | C01 | c2 | D12 |
| B2R061-01 | 842.24 | OEt | A01 | a1 | B11 | b1 | C01 | c2 | D12 |
| B2R062-01 | 843.24 | OEt | A02 | a1 | B11 | b1 | C01 | c2 | D12 |
| B2R063-01 | 858.23 | OEt | A01 | a1 | B13 | b1 | C01 | c2 | D12 |

| | | | | | | | | | |
|---|---|---|---|---|---|---|---|---|---|
| B2R064-01 | 859.23 | OEt | A02 | a1 | B13 | b1 | C01 | c2 | D12 |
| B2R065-01 | 824.27 | OEt | A01 | a1 | B09 | b1 | C01 | c2 | D12 |
| B2R066-01 | 825.26 | OEt | A02 | a1 | B09 | b1 | C01 | c2 | D12 |
| B2R067-01 | 780.21 | OEt | A01 | a1 | B10 | b1 | C01 | c2 | D12 |
| B2R068-01 | 781.2 | OEt | A02 | a1 | B10 | b1 | C01 | c2 | D12 |
| B2R069-01 | 798.25 | OEt | A01 | a2 | B01 | b1 | C01 | c2 | D12 |
| B2R070-01 | 799.25 | OEt | A02 | a2 | B01 | b1 | C01 | c2 | D12 |
| B2R071-01 | 776.24 | OEt | A01 | a1 | B01 | b1 | C02 | c2 | D12 |
| B2R072-01 | 777.24 | OEt | A02 | a1 | B01 | b1 | C02 | c2 | D12 |
| B2R073-01 | 790.26 | OEt | A01 | a1 | B04 | b1 | C02 | c2 | D12 |
| B2R074-01 | 791.25 | OEt | A02 | a1 | B04 | b1 | C02 | c2 | D12 |
| B2R075-01 | 804.27 | OEt | A01 | a1 | B05 | b1 | C02 | c2 | D12 |
| B2R076-01 | 805.27 | OEt | A02 | a1 | B05 | b1 | C02 | c2 | D12 |
| B2R077-01 | 794.23 | OEt | A01 | a1 | B06 | b1 | C02 | c2 | D12 |
| B2R078-01 | 795.23 | OEt | A02 | a1 | B06 | b1 | C02 | c2 | D12 |
| B2R079-01 | 806.25 | OEt | A01 | a1 | B07 | b1 | C02 | c2 | D12 |
| B2R080-01 | 807.25 | OEt | A02 | a1 | B07 | b1 | C02 | c2 | D12 |
| B2R081-01 | 820.27 | OEt | A01 | a1 | B14 | b1 | C02 | c2 | D12 |
| B2R082-01 | 821.26 | OEt | A02 | a1 | B14 | b1 | C02 | c2 | D12 |
| B2R083-01 | 834.28 | OEt | A01 | a1 | B15 | b1 | C02 | c2 | D12 |
| B2R084-01 | 835.28 | OEt | A02 | a1 | B15 | b1 | C02 | c2 | D12 |
| B2R085-01 | 836.26 | OEt | A01 | a1 | B08 | b1 | C02 | c2 | D12 |
| B2R086-01 | 837.26 | OEt | A02 | a1 | B08 | b1 | C02 | c2 | D12 |
| B2R087-01 | 844.23 | OEt | A01 | a1 | B11 | b1 | C02 | c2 | D12 |
| B2R088-01 | 845.23 | OEt | A02 | a1 | B11 | b1 | C02 | c2 | D12 |
| B2R089-01 | 860.23 | OEt | A01 | a1 | B13 | b1 | C02 | c2 | D12 |
| B2R090-01 | 861.22 | OEt | A02 | a1 | B13 | b1 | C02 | c2 | D12 |
| B2R091-01 | 826.26 | OEt | A01 | a1 | B09 | b1 | C02 | c2 | D12 |
| B2R092-01 | 827.25 | OEt | A02 | a1 | B09 | b1 | C02 | c2 | D12 |
| B2R093-01 | 782.2 | OEt | A01 | a1 | B10 | b1 | C02 | c2 | D12 |
| B2R094-01 | 783.19 | OEt | A02 | a1 | B10 | b1 | C02 | c2 | D12 |
| B2R095-01 | 800.24 | OEt | A01 | a2 | B01 | b1 | C02 | c2 | D12 |
| B2R096-01 | 801.24 | OEt | A02 | a2 | B01 | b1 | C02 | c2 | D12 |
| B2R097-01 | 788.27 | OEt | A01 | a1 | B03 | b2 | C01 | c2 | D12 |
| B2R098-01 | 789.26 | OEt | A02 | a1 | B03 | b2 | C01 | c2 | D12 |
| B2R099-01 | 806.26 | OEt | A01 | a1 | B18 | b2 | C01 | c2 | D12 |
| B2R100-01 | 807.25 | OEt | A02 | a1 | B18 | b2 | C01 | c2 | D12 |

Example 4-5-3: Preparation of mixture solution and analysis by LCMS

DMSO was added to each of 12 types of mixtures obtained in Example 4-5-1 and Example 4-5-2 to prepare 12 types of approximately 10 mM solutions in DMSO. 100 μL each of the solutions was subjected to the experiment to evaluate binding to Bcl-2 by AS-MS described in Example 5. Also, a portion of each of the 12 types of 10 mM solutions in DMSO was diluted with MeCN and subjected to LCMS. The solutions were analyzed under analysis conditions SMD-FA05-long-25 min, and the UV areas were confirmed at a UV wavelength of 280 nm. The analysis results are shown in Table AI-03-02. Table AI-03-02 shows m/z and retention times of observed MS peaks, and the number of a compound that can be assigned to each MS-UV peak. Although m/z of the MS peaks was confirmed down to three decimal places, 0 at the decimal level may be omitted in the description in Table AI- 03-02. For example, "587.000" is also referred to as "587", "587.100" is also referred to as "587.1", and "587.120" is also referred to as "587.12". Although the retention times of the MS peaks were confirmed down to three decimal places and the retention times of the UV peaks were confirmed down to two decimal places, 0 at the decimal level may be omitted in the description in Table AI-03-02. For

example, "15.000" is also referred to as "15", "15.100" is also referred to as "15.1", and "15.120" is also referred to as "15.12".

[Table 264]

| [Table AI-03-02] | | | |
|---|---|---|---|
| Mixture No. | mixAI-B1A-01 | | |
| m/z | Retention time of MS peak (min) | Retention time of UV peak (min) | No. of assigned compound |
| 712.347 | 14.453 | 14.41 | B1A043-01 |
| 628.353 | 14.22 | 14.17 | B1A035-01 |
| 646.325 | 14.2 | 14.17 | B1A039-01 |
| 714.337 | 14.637 | 14.59 | B1A021-01 |
| 634.4 | 11.127 | 11.09 | B1A037-01, B1A087-01 |
| 584.294 | 9.7 | 9.65 | B1A023-01, B1A077-01 |
| 603.333 | 13.047 | 13.01 | B1A031-01 |
| 564.358 | 9.197 | 9.15 | B1A045-01 |
| 648.316 | 10.537 | 10.5 | B1A017-01, B1A063-01 |
| 632.346 | 10.39 | 10.36 | B1A061-01 |
| 605.323 | 13.127 | 13.08 | B1A009-01 |
| 637.386 | 12.733 | 12.71 | B1A016-01 |
| 606.319 | 13.793 | 13.75 | B1A100-01, B1A033-01 |
| 629.349 | 11.683 | 11.65 | B1A036-01 |
| 634.4 | 14.913 | 14.88 | B1A037-01, B1A087-01 |
| 670.325 | 14.91 | 14.88 | B1A041-01 |
| 672.316 | 15.117 | 15.08 | B1A019-01 |
| 647.32 | 12.327 | 12.31 | B1A040-01 |
| 713.342 | 12.353 | 12.31 | B1A044-01 |
| 649.311 | 12.4 | 12.37 | B1A018-01, B1A064-01 |
| 715.333 | 12.4 | 12.37 | B1A022-01 |
| 586.285 | 13.477 | 13.42 | B1A001-01 |
| 596.328 | 13.447 | 13.42 | B1A027-01, B1A079-01, B1A099-01 |
| 587.28 | 10.673 | 10.63 | B1A002-01 |
| 593.349 | 10.903 | 10.87 | B1A026-01, B1A050-01 |
| 585.289 | 10.78 | 10.75 | B1A024-01, B1A078-01 |
| 616.365 | 10.793 | 10.75 | B1A091-01 |
| 583.344 | 7.963 | 7.92 | B1A052-01 |
| 594.344 | 13.927 | 13.89 | B1A003-01, B1A053-01, B1A075-01 |
| 635.396 | 12.607 | 12.57 | B1A038-01, B1A088-01 |

(continued)

| [Table AI-03-02] | | | |
|---|---|---|---|
| Mixture No. | mixAI-B1A-01 | | |
| m/z | Retention time of MS peak (min) | Retention time of UV peak (min) | No. of assigned compound |
| 590.349 | 10.177 | 10.13 | B1A095-01 |
| 614.374 | 10.177 | 10.13 | B1A065-01 |
| 608.36 | 14.02 | 13.98 | B1A011-01, B1A055-01 |
| 631.339 | 11.737 | 11.69 | B1A014-01 |
| 617.36 | 8.833 | 8.79 | B1A092-01 |
| 579.369 | 7.68 | 7.65 | B1A048-01, B1A098-01 |
| 573.301 | 7.75 | 7.71 | B1A094-01 |
| 597.324 | 7.733 | 7.71 | B1A100-01, B1A028-01, B1A080-01 |
| 611.37 | 8.313 | 8.28 | B1A082-01 |
| 630.344 | 14.43 | 14.36 | B1A013-01 |
| 648.316 | 14.397 | 14.36 | B1A017-01, B1A063-01 |
| 615.369 | 8.493 | 8.48 | B1A066-01 |
| 623.396 | 8.527 | 8.48 | B1A058-01 |
| 597.324 | 11.3 | 11.25 | B1A100-01, B1A028-01, B1A080-01 |
| 599.314 | 11.24 | 11.25 | B1A006-01 |
| 650.331 | 11.277 | 11.25 | B1A089-01 |
| 609.355 | 11.033 | 11 | B1A012-01, B1A056-01 |
| 588.358 | 9.77 | 9.73 | B1A069-01 |
| 595.339 | 10.857 | 10.81 | B1A004-01, B1A054-01, B1A076-01 |
| 635.396 | 9.397 | 9.36 | B1A038-01, B1A088-01 |
| 565.354 | 8.003 | 7.97 | B1A046-01 |
| 585.289 | 8.107 | 8.07 | B1A024-01, B1A078-01 |
| 625.375 | 8.093 | 8.07 | B1A060-01, B1A084-01 |
| 571.31 | 7.607 | 7.57 | B1A068-01 |
| 597.324 | 13.583 | 13.54 | B1A100-01, B1A028-01, B1A080-01 |
| 598.319 | 13.577 | 13.54 | B1A005-01 |
| 671.32 | 13.57 | 13.54 | B1A042-01 |
| 592.353 | 9.887 | 9.86 | B1A025-01, B1A049-01 |
| 622.4 | 9.9 | 9.86 | B1A057-01 |
| 607.364 | 11.08 | 11.04 | B1A034-01 |
| 609.355 | 8.247 | 8.21 | B1A012-01, B1A056-01 |
| 578.374 | 9.35 | 9.31 | B1A047-01, B1A097-01 |

(continued)

| [Table AI-03-02] | | | |
|---|---|---|---|
| Mixture No. | mixAI-B1A-01 | | |
| m/z | Retention time of MS peak (min) | Retention time of UV peak (min) | No. of assigned compound |
| 578.374 | 9.383 | 9.31 | B1A047-01, B1A097-01 |
| 582.349 | 9.347 | 9.31 | B1A051-01 |
| 570.315 | 9.273 | 9.23 | B1A067-01 |
| 594.344 | 9.247 | 9.23 | B1A003-01, B1A053-01, B1A075-01 |
| 595.339 | 9.263 | 9.23 | B1A004-01, B1A054-01, B1A076-01 |
| 636.391 | 15.18 | 15.14 | B1A015-01 |
| 625.375 | 8.633 | 8.59 | B1A060-01, B1A084-01 |
| 595.339 | 8.41 | 8.38 | B1A004-01, B1A054-01, B1A076-01 |
| 567.344 | 8.203 | 8.17 | B1A072-01 |
| 593.349 | 8.197 | 8.17 | B1A026-01, B1A050-01 |
| 610.375 | 9.83 | 9.8 | B1A081-01 |
| 596.328 | 9.603 | 9.53 | B1A027-01, B1A079-01, B1A099-01 |
| 651.327 | 9.57 | 9.53 | B1A090-01 |
| 566.349 | 9.47 | 9.43 | B1A071-01 |
| 581.36 | 9.49 | 9.43 | B1A074-01 |
| 592.353 | 13.753 | 13.72 | B1A025-01, B1A049-01 |
| 604.328 | 13.763 | 13.72 | B1A032-01 |
| 606.319 | 13.773 | 13.72 | B1A100-01, B1A033-01 |
| 649.311 | 9.123 | 9.09 | B1A018-01, B1A064-01 |
| 594.344 | 10.353 | 10.31 | B1A003-01, B1A053-01, B1A075-01 |
| 580.365 | 9.5 | 9.46 | B1A073-01 |
| 624.38 | 9.513 | 9.46 | B1A059-01, B1A083-01 |
| 626.37 | 9.527 | 9.46 | B1A085-01 |
| 627.365 | 9.507 | 9.46 | B1A086-01 |
| 591.344 | 8.973 | 8.94 | B1A096-01 |
| 633.341 | 8.977 | 8.94 | B1A062-01 |
| 624.38 | 10.13 | 10.09 | B1A059-01, B1A083-01 |
| 589.354 | 8.737 | 8.7 | B1A070-01 |
| 584.294 | 13.37 | 13.34 | B1A023-01, B1A077-01 |
| 596.328 | 13.43 | 13.34 | B1A027-01, B1A079-01, B1A099-01 |
| 673.311 | 13.717 | 13.67 | B1A020-01 |

(continued)

| [Table AI-03-02] | | | |
|---|---|---|---|
| Mixture No. | mixAI-B1A-01 | | |
| m/z | Retention time of MS peak (min) | Retention time of UV peak (min) | No. of assigned compound |
| 572.305 | 9.67 | 9.6 | B1A093-01 |
| 608.36 | 9.63 | 9.6 | B1A011-01, B1A055-01 |
| 579.369 | 10.067 | 9.98 | B1A048-01, B1A098-01 |

[Table 265]

| Mixture No. | mixAI-B1K-01 | | |
|---|---|---|---|
| m/z | Retention time of MS peak (min) | Retention time of UV peak (min) | No. of assigned compound |
| 539.201 | 9.893 | 9.86 | B1K009-01 |
| 580.203 | 11.68 | 11.64 | B1K039-01 |
| 646.225 | 12.083 | 12.05 | B1K043-01 |
| 582.193 | 11.327 | 11.29 | B1K017-01, B1K063-01 |
| 648.215 | 11.767 | 11.73 | B1K021-01 |
| 528.222 | 10.577 | 10.54 | B1K003-01, B1K053-01, B1K075-01 |
| 532.197 | 10.34 | 10.29 | B1K005-01 |
| 537.211 | 10.317 | 10.29 | B1K031-01 |
| 568.278 | 8.507 | 8.46 | B1K037-01, B1K087-01 |
| 542.237 | 10.673 | 10.63 | B1K011-01, B1K055-01 |
| 606.193 | 12.23 | 12.2 | B1K019-01 |
| 564.222 | 11.133 | 11.1 | B1K013-01 |
| 518.172 | 7.36 | 7.31 | B1K023-01, B1K077-01 |
| 540.197 | 11.047 | 10.98 | B1K100-01, B1K033-01 |
| 540.197 | 11.023 | 10.98 | B1K100-01, B1K033-01 |
| 563.226 | 8.68 | 8.64 | B1K036-01 |
| 584.209 | 8.673 | 8.64 | B1K089-01 |
| 550.242 | 8.22 | 8.18 | B1K091-01 |
| 556.278 | 8.233 | 8.18 | B1K057-01 |
| 562.231 | 11.513 | 11.47 | B1K035-01 |
| 545.248 | 5.987 | 5.96 | B1K082-01 |
| 500.227 | 7.09 | 7.05 | B1K071-01 |
| 583.189 | 7.09 | 7.05 | B1K018-01, B1K064-01 |
| 571.264 | 9.203 | 9.14 | B1K016-01 |
| 649.21 | 9.177 | 9.14 | B1K022-01 |
| 570.269 | 12.01 | 11.98 | B1K015-01 |

(continued)

| Mixture No. | mixAI-B1K-01 | | |
|---|---|---|---|
| m/z | Retention time of MS peak (min) | Retention time of UV peak (min) | No. of assigned compound |
| 529.217 | 7.317 | 7.27 | B1K004-01, B1K054-01, B1K076-01 |
| 531.201 | 7.3 | 7.27 | B1K100-01, B1K028-01, B1K080-01 |
| 505.188 | 12.567 | 12.52 | B1K068-01 |
| 604.203 | 12.557 | 12.52 | B1K041-01 |
| 582.193 | 8.617 | 8.59 | B1K017-01, B1K063-01 |
| 583.189 | 9.07 | 9.03 | B1K018-01, B1K064-01 |
| 506.183 | 7.17 | 7.11 | B1K093-01 |
| 521.158 | 7.143 | 7.11 | B1K002-01 |
| 530.206 | 10.75 | 10.72 | B1K027-01, B1K079-01, B1K099-01 |
| 530.206 | 10.763 | 10.72 | B1K027-01, B1K079-01, B1K099-01 |
| 541.242 | 8.163 | 8.14 | B1K034-01 |
| 565.217 | 8.187 | 8.14 | B1K014-01 |
| 522.236 | 7.877 | 7.83 | B1K069-01 |
| 527.226 | 7.867 | 7.83 | B1K026-01, B1K050-01 |
| 519.167 | 7.74 | 7.71 | B1K024-01, B1K078-01 |
| 533.192 | 7.727 | 7.71 | B1K006-01 |
| 544.253 | 7.76 | 7.71 | 7.71 B1K081-01 |
| 568.278 | 12.347 | 12.31 | B1K037-01, B1K087-01 |
| 529.217 | 9.63 | 9.59 | B1K004-01, B1K054-01, B1K076-01 |
| 569.273 | 9.657 | 9.59 | B1K038-01, B1K088-01 |
| 647.22 | 9.633 | 9.59 | B1K044-01 |
| 543.233 | 7.667 | 7.62 | B1K012-01, B1K056-01 |
| 585.204 | 7.027 | 6.99 | B1K090-01 |
| 501.222 | 5.597 | 5.56 | B1K072-01 |
| 551.238 | 6.157 | 6.13 | B1K092-01 |
| 581.198 | 9.533 | 9.5 | B1K040-01 |
| 528.222 | 7.543 | 7.51 | B1K003-01, B1K053-01, B1K075-01 |
| 499.232 | 5.757 | 5.71 | B1K046-01 |
| 513.247 | 7.407 | 7.36 | B1K048-01, B1K098-01 |
| 514.242 | 7.407 | 7.36 | B1K073-01 |
| 518.172 | 10.62 | 10.58 | B1K023-01, B1K077-01 |

(continued)

| Mixture No. | mixAI-B1K-01 | | |
|---|---|---|---|
| m/z | Retention time of MS peak (min) | Retention time of UV peak (min) | No. of assigned compound |
| 529.217 | 5.847 | 5.8 | B1K004-01, B1K054-01, B1K076-01 |
| 567.219 | 6.93 | 6.89 | B1K062-01 |
| 566.224 | 8.453 | 8.42 | B1K061-01 |
| 569.273 | 8.48 | 8.42 | B1K038-01, B1K088-01 |
| 523.232 | 8.023 | 8.03 | B1K070-01 |
| 526.231 | 8.05 | 8.03 | B1K025-01, B1K049-01 |
| 557.273 | 8.077 | 8.03 | B1K058-01 |
| 558.257 | 8.077 | 8.03 | B1K059-01, B1K083-01 |
| 512.252 | 7.627 | 7.56 | B1K047-01, B1K097-01 |
| 512.252 | 7.6 | 7.56 | B1 K047-01, B1 K097-01 |
| 560.248 | 7.597 | 7.56 | B1K085-01 |
| 531.201 | 5.37 | 5.34 | B1K100-01, B1K028-01, B1K080-01 |
| 528.222 | 7.957 | 7.91 | B1K003-01, B1K053-01, B1K075-01 |
| 530.206 | 7.953 | 7.91 | B1K027-01, B1K079-01, B1K099-01 |
| 542.237 | 7.937 | 7.91 | B1K011-01, B1K055-01 |
| 524.227 | 7.817 | 7.78 | B1K095-01 |
| 525.222 | 7.8 | 7.78 | B1K096-01 |
| 558.257 | 7.853 | 7.78 | B1K059-01, B1K083-01 |
| 520.162 | 10.163 | 10.13 | B1K001-01 |
| 543.233 | 10.203 | 10.13 | B1K012-01, B1K056-01 |
| 549.247 | 6.297 | 6.28 | B1K066-01 |
| 559.253 | 6.32 | 6.28 | B1K060-01, B1K084-01 |
| 561.243 | 5.783 | 5.75 | B1K086-01 |
| 531.201 | 8.293 | 8.26 | B1K100-01, B1K028-01, B1K080-01 |
| 498.236 | 7.23 | 7.2 | B1K045-01 |
| 504.193 | 7.277 | 7.2 | B1K067-01 |
| 515.238 | 7.26 | 7.2 | B1K074-01 |
| 538.206 | 8.583 | 8.54 | B1K032-01 |
| 513.247 | 9.463 | 9.43 | B1K048-01, B1K098-01 |
| 507.178 | 5.017 | 4.98 | B1K094-01 |
| 516.227 | 7.493 | 7.46 | 7.46 B1K051-01 |
| 517.222 | 7.503 | 7.46 | B1K052-01 |

(continued)

| Mixture No. | mixAI-B1K-01 | | |
|---|---|---|---|
| m/z | Retention time of MS peak (min) | Retention time of UV peak (min) | No. of assigned compound |
| 519.167 | 7.487 | 7.46 | B1K024-01, B1K078-01 |
| 526.231 | 11 | 10.92 | B1K025-01, B1K049-01 |
| 527.226 | 10.983 | 10.92 | B1K026-01, B1K050-01 |
| 605.198 | 10.96 | 10.92 | B1K042-01 |
| 548.252 | 8.26 | 8.22 | B1K065-01 |
| 559.253 | 6.04 | 6.01 | B1K060-01, B1K084-01 |
| 607.189 | 10.563 | 10.51 | B1K020-01 |

[Table 266]

| Mixture No. | mixAI-B1M-01 | | |
|---|---|---|---|
| mlz | Retention time of MS peak (min) | Retention time of UV peak (min) | No. of assigned compound |
| 497.255 | 9.977 | 9.94 | B1M031-01 |
| 531.308 | 8.92 | 8.87 | B1M016-01 |
| 606.269 | 11.847 | 11.81 | B1M043-01 |
| 543.233 | 6.817 | 6.76 | B1M018-01, B1M064-01 |
| 542.237 | 11.143 | 11.11 | B1M017-01, B1M063-01 |
| 526.268 | 8.153 | 8.12 | B1M061-01 |
| 460.271 | 6.86 | 6.82 | B1M071-01 |
| 564.247 | 12.347 | 12.31 | B1M041-01 |
| 542.237 | 8.32 | 8.29 | B1M017-01, B1M063-01 |
| 544.253 | 8.397 | 8.36 | B1M089-01 |
| 484.271 | 7.593 | 7.55 | B1M095-01 |
| 458.28 | 6.973 | 6.94 | B1M045-01 |
| 489.261 | 6.97 | 6.94 | B1M004-01, B1M054-01, B1M076-01 |
| 524.266 | 10.953 | 10.91 | B1M013-01 |
| 490.25 | 10.427 | 10.39 | B1M027-01, B1M079-01, B1M099-01 |
| 502.281 | 10.41 | 10.39 | B1M011-01, B1M055-01 |
| 528.322 | 8.227 | 8.19 | B1M037-01, B1M087-01 |
| 489.261 | 5.403 | 5.34 | B1M004-01, B1M054-01, B1M076-01 |
| 529.317 | 9.26 | 9.23 | B1M038-01, B1M088-01 |
| 607.264 | 9.263 | 9.23 | B1M044-01 |
| 522.275 | 11.207 | 11.17 | B1M035-01 |

(continued)

| Mixture No. | mixAI-B1M-01 | | |
|---|---|---|---|
| mlz | Retention time of MS peak (min) | Retention time of UV peak (min) | No. of assigned compound |
| 481.202 | 11.407 | 11.37 | B1M002-01 |
| 540.247 | 11.403 | 11.37 | B1M039-01 |
| 505.292 | 5.8 | 5.77 | B1M082-01 |
| 478.216 | 7.127 | 7.09 | B1M023-01, B1M077-01 |
| 490.25 | 7.127 | 7.09 | B1M027-01, B1M079-01, B1M099-01 |
| 467.222 | 4.867 | 4.83 | B1M094-01 |
| 545.248 | 6.75 | 6.71 | B1M090-01 |
| 499.245 | 9.61 | 9.58 | B1M009-01 |
| 529.317 | 9.653 | 9.58 | B1M038-01, B1M088-01 |
| 519.297 | 6.14 | 6.11 | B1M060-01, B1M084-01 |
| 509.291 | 6.03 | 6 | B1M066-01 |
| 488.266 | 10.337 | 10.3 | B1M003-01, B1M053-01, B1M075-01 |
| 567.233 | 10.327 | 10.3 | B1M020-01 |
| 486.275 | 7.797 | 7.75 | B1M025-01, B1M049-01 |
| 541.242 | 9.14 | 9.1 | B1M040-01 |
| 508.296 | 7.977 | 7.94 | B1M065-01 |
| 510.286 | 7.97 | 7.94 | B1M091-01 |
| 517.317 | 7.997 | 7.94 | B1M058-01 |
| 479.211 | 7.32 | 7.27 | B1M024-01, B1M078-01 |
| 488.266 | 7.333 | 7.27 | B1M003-01, B1M053-01, B1M075-01 |
| 503.277 | 7.31 | 7.27 | B1M012-01, B1M056-01 |
| 492.241 | 10.08 | 10.04 | B1M005-01 |
| 498.25 | 10.077 | 10.04 | B1M032-01 |
| 527.263 | 10.057 | 10.04 | B1M062-01 |
| 478.216 | 10.273 | 10.23 | B1M023-01, B1M077-01 |
| 491.245 | 7.86 | 7.82 | B1M100-01, B1M028-01, B1M080-01 |
| 518.301 | 7.883 | 7.82 | B1M059-01, B1M083-01 |
| 525.261 | 7.86 | 7.82 | B1M014-01 |
| 480.206 | 9.89 | 9.85 | B1M001-01 |
| 482.28 | 7.63 | 7.59 | B1M069-01 |
| 518.301 | 7.637 | 7.59 | B1M059-01, B1M083-01 |
| 608.259 | 11.623 | 11.59 | B1M021-01 |

(continued)

| Mixture No. | mixAI-B1M-01 | | |
|---|---|---|---|
| mlz | Retention time of MS peak (min) | Retention time of UV peak (min) | No. of assigned compound |
| 609.254 | 11.637 | 11.59 | B1M022-01 |
| 491.245 | 5.197 | 5.17 | B1M100-01, B1M028-01, B1M080-01 |
| 459.275 | 5.477 | 5.43 | B1M046-01 |
| 477.266 | 5.457 | 5.43 | B1M052-01 |
| 487.27 | 5.753 | 5.71 | B1M026-01, B1M050-01 |
| 511.282 | 5.937 | 5.9 | B1M092-01 |
| 464.237 | 8.267 | 8.22 | B1M067-01 |
| 523.27 | 8.26 | 8.22 | B1M036-01 |
| 472.296 | 7.383 | 7.34 | B1M047-01, B1M097-01 |
| 493.236 | 7.377 | 7.34 | B1M006-01 |
| 543.233 | 8.763 | 8.72 | B1M018-01, B1M064-01 |
| 485.266 | 10.667 | 10.64 | B1M096-01 |
| 486.275 | 10.677 | 10.64 | B1M025-01, B1M049-01 |
| 500.24 | 10.663 | 10.64 | B1M100-01, B1 M033-01 |
| 500.24 | 10.677 | 10.64 | B1M100-01, B1M033-01 |
| 565.242 | 10.65 | 10.64 | B1M042-01 |
| 473.291 | 5.26 | 5.22 | B1M048-01, B1M098-01 |
| 465.232 | 4.99 | 4.96 | B1M068-01 |
| 487.27 | 7.437 | 7.4 | B1M026-01, B1M050-01 |
| 490.25 | 7.463 | 7.4 | B1M027-01, B1M079-01, B1M099-01 |
| 519.297 | 7.5 | 7.4 | B1M060-01, B1M084-01 |
| 520.292 | 7.433 | 7.4 | B1M085-01 |
| 466.227 | 6.907 | 6.87 | B1M093-01 |
| 530.313 | 11.903 | 11.86 | B1M015-01 |
| 473.291 | 7.197 | 7.19 | B1M048-01, B1M098-01 |
| 474.286 | 7.203 | 7.19 | B1M073-01 |
| 475.282 | 7.227 | 7.19 | B1M074-01 |
| 476.271 | 7.217 | 7.19 | B1M051-01 |
| 488.266 | 7.723 | 7.7 | B1M003-01, B1M053-01, B1M075-01 |
| 501.286 | 7.737 | 7.7 | B1M034-01 |
| 502.281 | 7.713 | 7.7 | B1M011-01, B1M055-01 |
| 503.277 | 7.707 | 7.7 | B1M012-01, B1M056-01 |

(continued)

| Mixture No. | mixAI-B1M-01 | | |
|---|---|---|---|
| mlz | Retention time of MS peak (min) | Retention time of UV peak (min) | No. of assigned compound |
| 489.261 | 5.623 | 5.58 | B1M004-01, B1M054-01, B1M076-01 |
| 491.245 | 5.633 | 5.58 | B1M100-01, B1M028-01, B1M080-01 |
| 521.287 | 5.61 | 5.58 | B1M086-01 |
| 483.275 | 6.223 | 6.15 | B1M070-01 |
| 461.266 | 5.363 | 5.29 | B1M072-01 |
| 472.296 | 5.343 | 5.29 | B1M047-01, B1M097-01 |
| 479.211 | 5.33 | 5.29 | B1M024-01, B1M078-01 |
| 504.297 | 7.573 | 7.53 | B1M081-01 |
| 516.322 | 8.013 | 7.97 | B1M057-01 |
| 528.322 | 12.107 | 12.07 | B1M037-01, B1M087-01 |
| 566.237 | 12.13 | 12.07 | B1M019-01 |

[Table 267]

| Mixture No. | mixAI-B2Q-01 | | |
|---|---|---|---|
| mlz | Retention time of MS peak (min) | Retention time of UV peak (min) | No. of assigned compound |
| 834.224 | 13.403 | 13.37 | B2Q039-01 |
| 792.227 | 11.153 | 11.11 | B2Q032-01 |
| 771.243 | 8.4 | 8.33 | B2Q052-01 |
| 793.222 | 12.13 | 12.1 | B2Q009-01 |
| 786.217 | 12.46 | 12.41 | B2Q005-01 |
| 791.232 | 12.44 | 12.41 | B2Q031-01 |
| 817.247 | 11.3 | 11.26 | B2Q036-01 |
| 776.257 | 9.887 | 9.84 | B2Q069-01 |
| 861.209 | 12.583 | 12.55 | B2Q020-01 |
| 816.252 | 13.337 | 13.31 | B2Q035-01 |
| 794.217 | 13.023 | 12.99 | B2Q100-01, B2Q033-01 |
| 900.246 | 13.653 | 13.61 | B2Q043-01 |
| 781.247 | 10.717 | 10.68 | B2Q026-01, B2Q050-01 |
| 836.214 | 10.717 | 10.68 | B2Q017-01, B2Q063-01 |
| 858.224 | 14.037 | 14 | B2Q041-01 |
| 836.214 | 13.157 | 13.11 | B2Q017-01, B2Q063-01 |
| 837.209 | 11.56 | 11.52 | B2Q018-01, B2Q064-01 |
| 903.231 | 11.55 | 11.52 | B2Q022-01 |

(continued)

| Mixture No. | mixAl-B2Q-01 | | |
|---|---|---|---|
| mlz | Retention time of MS peak (min) | Retention time of UV peak (min) | No. of assigned compound |
| 752.257 | 9.413 | 9.38 | B2Q045-01 |
| 782.243 | 9.4 | 9.38 | B2Q003-01, B2Q053-01, B2Q075-01 |
| 782.243 | 12.677 | 12.64 | B2Q003-01, B2Q053-01, B2Q075-01 |
| 775.179 | 10.22 | 10.18 | B2Q002-01 |
| 802.273 | 10.387 | 10.35 | B2Q065-01 |
| 761.199 | 8.06 | 8.02 | B2Q094-01 |
| 780.252 | 12.983 | 12.94 | B2Q025-01, B2Q049-01 |
| 811.294 | 8.833 | 8.79 | B2Q058-01 |
| 803.268 | 8.923 | 8.87 | B2Q066-01 |
| 835.219 | 11.88 | 11.84 | B2Q040-01 |
| 901.241 | 11.887 | 11.84 | B2Q044-01 |
| 902.236 | 11.863 | 11.84 | B2Q021-01 |
| 784.227 | 12.757 | 12.72 | B2Q027-01, B2Q079-01, B2Q099-01 |
| 796.258 | 12.783 | 12.72 | 820011-01, B2Q055-01 |
| 773.188 | 10.627 | 10.57 | B2Q024-01, B2Q078-01 |
| 787.213 | 10.65 | 10.57 | B2Q006-01 |
| 804.263 | 10.6 | 10.57 | B2Q091-01 |
| 820.244 | 10.597 | 10.57 | B2Q061-01 |
| 794.217 | 10.807 | 10.77 | B2Q100-01, B2Q033-01 |
| 818.243 | 13.083 | 13.03 | B2Q013-01 |
| 779.243 | 9.097 | 9.06 | B2Q096-01 |
| 777.252 | 9.04 | 9 | B2Q070-01 |
| 795.263 | 10.923 | 10.89 | B2Q034-01 |
| 819.238 | 10.94 | 10.89 | B2Q014-01 |
| 822.299 | 10.933 | 10.89 | B24037-01, B2Q087-01 |
| 824.289 | 10.927 | 10.89 | B2Q015-01 |
| 784.227 | 9.247 | 9.21 | B2Q027-01, B2Q079-01, B2Q099-01 |
| 754.248 | 9.463 | 9.43 | B2Q071-01 |
| 768.263 | 9.46 | 9.43 | B2Q073-01 |
| 823.294 | 9.5 | 9.43 | B2Q038-01, B2Q088-01 |
| 837.209 | 9.467 | 9.43 | B2Q018-01, B2Q064-01 |
| 753.252 | 8.463 | 8.44 | B2Q046-01 |
| 755.243 | 8.477 | 8.44 | B2Q072-01 |

(continued)

| Mixture No. | mixAl-B2Q-01 | | |
|---|---|---|---|
| mlz | Retention time of MS peak (min) | Retention time of UV peak (min) | No. of assigned compound |
| 785.222 | 8.51 | 8.44 | B2Q100-01, 820028-01, 820080-01 |
| 785.222 | 11.033 | 11.01 | B2Q100-01, 820028-01, 820080-01 |
| 838.23 | 11.057 | 11.01 | B2Q089-01 |
| 839.225 | 11.037 | 11.01 | B2Q090-01 |
| 797.253 | 10.543 | 10.51 | B2Q012-01, B2Q056-01 |
| 783.238 | 8.603 | 8.56 | B2Q004-01, B2Q054-01, B2Q076-01 |
| 797.253 | 8.587 | 8.56 | B2Q012-01, B2Q056-01 |
| 798.274 | 8.587 | 8.56 | B2Q081-01 |
| 778.248 | 10.047 | 10 | B2Q095-01 |
| 810.299 | 10.027 | 10 | B2Q057-01 |
| 813.273 | 10.03 | 10 | B2Q060-01, B2Q084-01 |
| 770.248 | 9.617 | 9.59 | B2Q051-01 |
| 812.278 | 9.607 | 9.59 | B2Q059-01, B2Q083-01 |
| 813.273 | 9.593 | 9.59 | B2Q060-01, B2Q084-01 |
| 814.269 | 9.613 | 9.59 | B2Q085-01 |
| 805.258 | 8.967 | 8.93 | B2Q092-01 |
| 821.24 | 9.35 | 9.31 | B2Q062-01 |
| 815.264 | 12.927 | 12.84 | B2Q086-01 |
| 859.219 | 12.883 | 12.84 | B2Q042-01 |
| 860.214 | 12.877 | 12.84 | B2Q019-01 |
| 822.299 | 12.05 | 12.01 | B2Q037-01, B2Q087-01 |
| 823.294 | 12.043 | 12.01 | B2Q038-01, B2Q088-01 |
| 825.285 | 12.037 | 12.01 | B2Q016-01 |
| 784.227 | 9.793 | 9.74 | B2Q027-01, B2Q079-01, B2Q099-01 |
| 796.258 | 9.783 | 9.74 | 820011-01, B2Q055-01 |
| 799.269 | 9.77 | 9.74 | B2Q082-01 |
| 767.268 | 6.52 | 7.74 | B2Q048-01, B2Q098-01 |
| 769.258 | 6.837 | 7.74 | B2Q074-01 |
| 772.193 | 9.693 | 9.66 | B2Q023-01, B2Q077-01 |
| 772.193 | 9.717 | 9.66 | B2Q023-01, B2Q077-01 |
| 774.183 | 9.697 | 9.66 | B2Q001-01 |
| 783.238 | 10.3 | 10.27 | B2Q004-01, B2Q054-01, B2Q076-01 |

(continued)

| Mixture No. | mixAl-B2Q-01 | | |
|---|---|---|---|
| mlz | Retention time of MS peak (min) | Retention time of UV peak (min) | No. of assigned compound |
| 785.222 | 10.313 | 10.27 | B2Q100-01, B2Q028-01, B2Q080-01 |
| 759.209 | 8.1 | 8.05 | B2Q068-01 |
| 760.204 | 8.113 | 8.05 | B2Q093-01 |
| 767.268 | 8.087 | 8.05 | B2Q048-01, B2Q098-01 |
| 783.238 | 8.143 | 8.05 | B2Q004-01, 820054-01, 820076-01 |
| 758.214 | 9.547 | 9.49 | B2Q067-01 |
| 766.273 | 9.537 | 9.49 | B2Q047-01, B2Q097-01 |
| 766.273 | 9.567 | 9.49 | B2Q047-01, B2Q097-01 |
| 812.278 | 9.59 | 9.49 | B2Q059-01, B2Q083-01 |
| 773.188 | 14.453 | 14.36 | B2Q024-01, B2Q078-01 |
| 780.252 | 10.097 | 10.06 | B2Q025-01, B2Q049-01 |
| 781.247 | 10.093 | 10.06 | B2Q026-01, B2Q050-01 |
| 782.243 | 10.113 | 10.06 | B2Q003-01, B2Q053-01, B2Q075-01 |

[Table 268]

| Mixture No. | mixAl-B1O-01 | | |
|---|---|---|---|
| mlz | Retention time of MS peak (min) | Retention time of UV peak (min) | No. of assigned compound |
| 606.232 | 12.077 | 12.03 | B1O017-01, B1O063-01 |
| 564.235 | 10.68 | 10.64 | B1O100-01, B1O033-01 |
| 574.281 | 8.87 | 8.83 | B1O091-01 |
| 524.266 | 7.723 | 7.68 | B1O071-01 |
| 528.232 | 7.74 | 7.68 | B1O067-01 |
| 604.242 | 12.157 | 12.12 | B1O039-01 |
| 561.25 | 10.857 | 10.81 | B1O031-01 |
| 673.249 | 9.983 | 9.94 | B1O022-01 |
| 606.232 | 9.003 | 8.97 | B1O017-01, B1O063-01 |
| 671.259 | 10.183 | 10.14 | B1O044-01 |
| 672.254 | 12.44 | 12.4 | B1O021-01 |
| 628.242 | 12.993 | 12.96 | B1O041-01 |
| 630.232 | 12.93 | 12.89 | B1O019-01 |
| 556.235 | 11.127 | 11.09 | B10005-01 |
| 562.245 | 11.153 | 11.09 | B1O032-01 |

(continued)

| Mixture No. | mixAI-B1O-01 | | |
|---|---|---|---|
| mlz | Retention time of MS peak (min) | Retention time of UV peak (min) | No. of assigned compound |
| 554.245 | 11.283 | 11.26 | B1O027-01, B1O079-01, B1O099-01 |
| 631.228 | 11.31 | 11.26 | B1O020-01 |
| 588.261 | 11.953 | 11.91 | B1O013-01 |
| 592.317 | 9.173 | 9.14 | B1O037-01, B1O087-01 |
| 593.312 | 7.54 | 7.5 | B1O038-01, B1O088-01 |
| 589.256 | 9.127 | 9.08 | B1O014-01 |
| 523.27 | 6.337 | 6.31 | B1O046-01 |
| 525.261 | 6.353 | 6.31 | B1O072-01 |
| 607.228 | 7.57 | 7.53 | B1O018-01, B1O064-01 |
| 557.231 | 8.693 | 8.62 | B1O006-01 |
| 572.291 | 8.653 | 8.62 | B1O065-01 |
| 607.228 | 9.917 | 9.88 | B1O018-01, B1O064-01 |
| 536.291 | 8.003 | 7.97 | B1O047-01, B1O097-01 |
| 575.277 | 6.947 | 6.91 | B1O092-01 |
| 583.291 | 7.013 | 6.98 | B1O060-01, B1O084-01 |
| 581.312 | 7.09 | 7.04 | B1O058-01 |
| 536.291 | 7.983 | 7.92 | B1O047-01, B1O097-01 |
| 538.281 | 7.947 | 7.92 | B1O073-01 |
| 542.211 | 7.957 | 7.92 | B1O023-01, B1O077-01 |
| 553.256 | 6.2 | 6.16 | B1O004-01, B1O054-01, B1O076-01 |
| 550.27 | 11.543 | 11.52 | B1O025-01, B1O049-01 |
| 564.235 | 11.56 | 11.52 | B1O100-01, B1O033-01 |
| 552.261 | 11.4 | 11.37 | B1O003-01, B1O053-01, B1O075-01 |
| 573.286 | 6.887 | 6.85 | B1O066-01 |
| 593.312 | 10.257 | 10.22 | B1O038-01, B1O088-01 |
| 595.303 | 10.09 | 10.07 | B1O016-01 |
| 605.237 | 10.12 | 10.07 | B1O040-01 |
| 591.258 | 7.413 | 7.37 | B1O062-01 |
| 587.265 | 9.33 | 9.3 | B1O036-01 |
| 608.248 | 9.347 | 9.3 | B1O089-01 |
| 609.243 | 9.33 | 9.3 | B1O090-01 |
| 544.201 | 10.997 | 10.95 | B1O001-01 |
| 566.276 | 11.487 | 11.44 | B1O011-01, B1O055-01 |
| 629.237 | 11.457 | 11.44 | B1O042-01 |
| 543.206 | 8.43 | 8.4 | B1O024-01, B1O078-01 |

(continued)

| Mixture No. | mixAI-B1O-01 | | |
|---|---|---|---|
| mlz | Retention time of MS peak (min) | Retention time of UV peak (min) | No. of assigned compound |
| 548.266 | 8.413 | 8.4 | B1O095-01 |
| 549.261 | 8.44 | 8.4 | B1O096-01 |
| 550.27 | 8.447 | 8.4 | B1O025-01, B1O049-01 |
| 547.27 | 12.53 | 12.49 | B1O070-01 |
| 670.264 | 12.52 | 12.49 | B1O043-01 |
| 537.286 | 6.077 | 6.03 | B1O048-01, B1O098-01 |
| 555.24 | 6.067 | 6.03 | B1O100-01, B1O028-01, B1O080-01 |
| 539.277 | 5.973 | 5.93 | B1O074-01 |
| 567.271 | 6.757 | 6.72 | B1O012-01, B1O056-01 |
| 545.197 | 8.11 | 8.07 | B1O002-01 |
| 554.245 | 8.14 | 8.07 | B1O027-01, B1O079-01, B1O099-01 |
| 584.287 | 8.1 | 8.07 | B1O085-01 |
| 594.307 | 12.79 | 12.76 | B1O015-01 |
| 546.275 | 8.297 | 8.24 | B1O069-01 |
| 553.256 | 8.277 | 8.24 | B1O004-01, B1O054-01, B1O076-01 |
| 553.256 | 8.297 | 8.24 | B1O004-01, B1O054-01, B1O076-01 |
| 566.276 | 8.307 | 8.24 | B1O011-01, B1O055-01 |
| 568.292 | 8.27 | 8.24 | B1O081-01 |
| 569.287 | 8.297 | 8.24 | B1O082-01 |
| 530.222 | 7.843 | 7.78 | B1O093-01 |
| 554.245 | 7.82 | 7.78 | B1O027-01, B1O079-01, B1O099-01 |
| 551.265 | 8.56 | 8.54 | B1O026-01, B1O050-01 |
| 551.265 | 8.543 | 8.54 | B1O026-01, B1O050-01 |
| 552.261 | 8.61 | 8.54 | B1O003-01, B1O053-01, B1O075-01 |
| 567.271 | 8.537 | 8.54 | B1O012-01, B1O056-01 |
| 580.317 | 8.58 | 8.54 | B1O057-01 |
| 582.296 | 8.573 | 8.54 | B1O059-01, B1O083-01 |
| 583.291 | 8.583 | 8.54 | B1O060-01, B1O084-01 |
| 541.261 | 6.307 | 6.25 | B1O052-01 |
| 543.206 | 6.287 | 6.25 | B1O024-01, B1O078-01 |
| 592.317 | 12.837 | 12.8 | B1O037-01, B1O087-01 |
| 531.217 | 8.227 | 8.17 | B1O094-01 |
| 582.296 | 8.21 | 8.17 | B1O059-01, B1O083-01 |
| 565.281 | 8.8 | 8.77 | B1O034-01 |
| 590.263 | 8.85 | 8.77 | B1O061-01 |

(continued)

| Mixture No. | mixAI-B1O-01 | | |
|---|---|---|---|
| mlz | Retention time of MS peak (min) | Retention time of UV peak (min) | No. of assigned compound |
| 537.286 | 7.943 | 7.87 | B1O048-01, B1O098-01 |
| 540.266 | 7.91 | 7.87 | B1O051-01 |
| 552.261 | 7.94 | 7.87 | B1O003-01, B1O053-01, B1O075-01 |
| 555.24 | 8.97 | 8.93 | B1O100-01, B1O028-01, B1O080-01 |
| 555.24 | 6.467 | 6.39 | B1O100-01, B1O028-01, B1O0080-01 |
| 585.282 | 6.437 | 6.39 | B1O086-01 |
| 529.227 | 9.657 | 9.62 | B1O068-01 |
| 522.275 | 7.697 | 7.65 | B1O045-01 |
| 586.27 | 12.047 | 11.99 | B1O035-01 |
| 542.211 | 11.173 | 11.13 | B1O023-01, B1O077-01 |

[Table 269]

| Mixture No. | mixAI-B3A-01 | | |
|---|---|---|---|
| m/z | Retention time of MS peak (min) | Retention time of UV peak (min) | No. of assigned compound |
| 617.349 | 13.957 | 13.92 | B3A031-01 |
| 660.341 | 15.067 | 15.02 | B3A039-01 |
| 726.363 | 15.26 | 15.21 | B3A043-01 |
| 598.31 | 9.143 | 9.1 | B3A023-01, B3A077-01 |
| 729.348 | 11.76 | 11.72 | B3A022-01 |
| 684.341 | 15.713 | 15.67 | B3A041-01 |
| 728.353 | 14.123 | 14.09 | B3A021-01 |
| 612.334 | 12.937 | 12.9 | B3A005-01 |
| 643.364 | 12.69 | 12.65 | B3A036-01 |
| 644.36 | 13.813 | 13.79 | B3A013-01 |
| 662.331 | 13.833 | 13.79 | B3A017-01, B3A063-01 |
| 649.411 | 13.663 | 13.61 | B3A038-01, B3A088-01 |
| 607.364 | 11.853 | 11.82 | B3A026-01, B3A050-01 |
| 628.39 | 10.723 | 10.68 | B3A065-01 |
| 642.369 | 15.15 | 15.11 | B3A035-01 |
| 599.305 | 11.703 | 11.67 | B3A024-01, B3A078-01 |
| 646.361 | 10.953 | 10.92 | B3A061-01 |
| 606.369 | 14.703 | 14.68 | B3A025-01, B3A049-01 |
| 620.334 | 14.72 | 14.68 | B3A100-01, B3A033-01 |
| 601.296 | 9.933 | 9.9 | B3A002-01 |

(continued)

| Mixture No. | mixAl-B3A-01 | | |
|---|---|---|---|
| m/z | Retention time of MS peak (min) | Retention time of UV peak (min) | No. of assigned compound |
| 609.355 | 10.133 | 10.08 | B3A004-01, B3A054-01, B3A076-01 |
| 647.357 | 9.447 | 9.4 | B3A062-01 |
| 610.344 | 14.367 | 14.33 | B3A027-01, B3A079-01, B3A099-01 |
| 611.339 | 14.387 | 14.33 | B3A100-01, B3A028-01, B3A080-01 |
| 685.336 | 14.47 | 14.44 | B3A042-01 |
| 596.365 | 9.787 | 9.75 | B3A051-01 |
| 687.327 | 13.103 | 13.06 | B3A020-01 |
| 650.406 | 14.62 | 14.58 | B3A015-01 |
| 686.331 | 14.613 | 14.58 | B3A019-01 |
| 608.36 | 13.263 | 13.25 | B3A003-01, B3A053-01, B3A075-01 |
| 609.355 | 13.267 | 13.25 | B3A004-01, B3A054-01, B3A076-01 |
| 661.336 | 13.25 | 13.25 | B3A040-01 |
| 727.358 | 13.297 | 13.25 | B3A044-01 |
| 618.344 | 12.27 | 12.23 | B3A032-01 |
| 619.339 | 12.493 | 12.45 | B3A009-01 |
| 620.334 | 12.48 | 12.45 | B3A100-01, B3A033-01 |
| 622.375 | 13.373 | 13.34 | B3A011-01, B3A055-01 |
| 600.3 | 12.803 | 12.78 | B3A001-01 |
| 640.386 | 9.193 | 9.16 | B3A085-01 |
| 638.395 | 9.867 | 9.83 | B3A059-01, B3A083-01 |
| 645.355 | 11.01 | 10.96 | B3A014-01 |
| 610.344 | 10.067 | 10.03 | B3A027-01, B3A079-01, B3A099-01 |
| 630.38 | 10.07 | 10.03 | B3A091-01 |
| 621.38 | 11.98 | 11.95 | B3A034-01 |
| 651.402 | 12.023 | 11.95 | B3A016-01 |
| 599.305 | 7.64 | 7.6 | B3A024-01, B3A078-01 |
| 648.416 | 15.803 | 15.76 | B3A037-01, B3A087-01 |
| 649.411 | 8.793 | 8.75 | B3A038-01, B3A088-01 |
| 606.369 | 10.39 | 10.33 | B3A025-01, B3A049-01 |
| 623.37 | 10.34 | 10.33 | B3A012-01, B3A056-01 |
| 648.416 | 10.37 | 10.33 | B3A037-01, B3A087-01 |
| 578.374 | 9.637 | 9.61 | B3A045-01 |

(continued)

| Mixture No. | mixAl-B3A-01 | | |
|---|---|---|---|
| m/z | Retention time of MS peak (min) | Retention time of UV peak (min) | No. of assigned compound |
| 604.365 | 9.647 | 9.61 | B3A095-01 |
| 638.395 | 9.69 | 9.61 | B3A059-01, B3A083-01 |
| 611.339 | 12.213 | 12.18 | B3A100-01, B3A028-01, B3A080-01 |
| 609.355 | 8.12 | 8.08 | B3A004-01, B3A054-01, B3A076-01 |
| 631.376 | 8.237 | 8.19 | B3A092-01 |
| 639.39 | 8.23 | 8.19 | B3A060-01, B3A084-01 |
| 625.386 | 7.92 | 7.87 | B3A082-01 |
| 611.339 | 7.39 | 7.35 | B3A100-01, B3A028-01, B3A080-01 |
| 595.376 | 7.327 | 7.28 | B3A074-01 |
| 602.374 | 10.267 | 10.23 | B3A069-01 |
| 636.416 | 10.31 | 10.23 | B3A057-01 |
| 662.331 | 11.103 | 11.06 | B3A017-01, B3A063-01 |
| 663.327 | 11.113 | 11.06 | B3A018-01, B3A064-01 |
| 580.365 | 8.977 | 8.92 | B3A071-01 |
| 610.344 | 8.947 | 8.92 | B3A027-01, B3A079-01, B3A099-01 |
| 629.385 | 8.947 | 8.92 | B3A066-01 |
| 665.342 | 8.95 | 8.92 | B3A090-01 |
| 587.316 | 7.28 | 7.23 | B3A094-01 |
| 597.36 | 8.36 | 8.32 | B3A052-01 |
| 598.31 | 14.31 | 14.27 | B3A023-01, B3A077-01 |
| 593.385 | 8.05 | 8.01 | B3A048-01, B3A098-01 |
| 585.326 | 8.01 | 7.98 | B3A068-01 |
| 637.411 | 8.883 | 8.85 | B3A058-01 |
| 593.385 | 10.59 | 10.49 | B3A048-01, B3A098-01 |
| 613.33 | 10.557 | 10.49 | B3A006-01 |
| 664.347 | 10.52 | 10.49 | B3A089-01 |
| 608.36 | 9.597 | 9.56 | B3A003-01, B3A053-01, B3A075-01 |
| 663.327 | 9.597 | 9.56 | B3A018-01, B3A064-01 |
| 581.36 | 7.723 | 7.68 | B3A072-01 |
| 641.381 | 7.707 | 7.68 | B3A086-01 |
| 607.364 | 8.637 | 8.59 | B3A026-01, B3A050-01 |
| 623.37 | 8.603 | 8.59 | B3A012-01, B3A056-01 |

(continued)

| Mixture No. | mixAI-B3A-01 | | |
|---|---|---|---|
| m/z | Retention time of MS peak (min) | Retention time of UV peak (min) | No. of assigned compound |
| 579.369 | 8.44 | 8.41 | B3A046-01 |
| 605.36 | 8.487 | 8.41 | B3A096-01 |
| 639.39 | 8.453 | 8.41 | B3A060-01, B3A084-01 |
| 584.331 | 9.73 | 9.69 | B3A067-01 |
| 592.39 | 9.74 | 9.69 | B3A047-01, B3A097-01 |
| 592.39 | 9.72 | 9.69 | B3A047-01, B3A097-01 |
| 608.36 | 9.72 | 9.69 | B3A003-01, B3A053-01, B3A075-01 |
| 622.375 | 10.023 | 9.99 | B3A011-01, B3A055-01 |
| 624.391 | 9.4 | 9.36 | B3A081-01 |
| 586.321 | 9.073 | 9.04 | B3A093-01 |
| 594.38 | 9.09 | 9.04 | B3A073-01 |
| 603.369 | 9.11 | 9.04 | B3A070-01 |

[Table 270]

| Mixture No. | mixAI-B4J-01 | | |
|---|---|---|---|
| m/z | Retention time of MS peak (min) | Retention time of UV peak (min) | No. of assigned compound |
| 623.27 | 8.463 | 8.42 | B4J022-01 |
| 621.28 | 9.76 | 9.72 | B4J044-01 |
| 511.27 | 10.47 | 10.43 | B4J031-01 |
| 538.281 | 10.473 | 10.43 | B4J013-01 |
| 506.256 | 9.6 | 9.56 | B4J005-01 |
| 620.284 | 12.403 | 12.36 | B4J043-01 |
| 513.261 | 9.163 | 9.12 | B4J009-01 |
| 578.263 | 12.937 | 12.9 | B4J041-01 |
| 556.253 | 10.73 | 10.69 | B4J017-01, B4J063-01 |
| 495.217 | 6.33 | 6.29 | B4J002-01 |
| 556.253 | 8.57 | 8.52 | B4J017-01, B4J063-01 |
| 580.253 | 11.743 | 11.71 | B4J019-01 |
| 502.281 | 9.847 | 9.81 | B4J003-01, B4J053-01, B4J075-01 |
| 537.286 | 8.777 | 8.74 | B4J036-01 |
| 500.291 | 11.263 | 11.21 | B4J025-01, B4J049-01 |
| 514.256 | 11.25 | 11.21 | B4J100-01, B4J033-01 |
| 622.275 | 11.247 | 11.21 | B4J021-01 |
| 539.277 | 7.373 | 7.33 | B4J014-01 |

(continued)

| Mixture No. | mixAI-B4J-01 | | |
|---|---|---|---|
| m/z | Retention time of MS peak (min) | Retention time of UV peak (min) | No. of assigned compound |
| 481.238 | 11.973 | 11.94 | B4J094-01 |
| 554.263 | 11.987 | 11.94 | B4J039-01 |
| 516.297 | 9.933 | 9.9 | B4J011-01, B4J055-01 |
| 487.307 | 5.553 | 5.52 | B4J048-01, B4J098-01 |
| 503.277 | 6.497 | 6.47 | B4J004-01, B4J054-01, B4J076-01 |
| 492.232 | 10.833 | 10.8 | B4J023-01, B4J077-01 |
| 475.282 | 8.12 | 8.13 | B4J072-01 |
| 522.312 | 8.223 | 8.18 | B4J065-01 |
| 530.338 | 8.21 | 8.18 | B4J057-01 |
| 501.286 | 6.027 | 5.99 | B4J026-01, B4J050-01 |
| 555.258 | 9.64 | 9.6 | B4J040-01 |
| 494.222 | 9.37 | 9.34 | B4J001-01 |
| 581.248 | 9.9 | 9.85 | B4J020-01 |
| 498.286 | 7.34 | 7.29 | B4J095-01 |
| 518.313 | 7.327 | 7.29 | B4J081-01 |
| 533.312 | 5.913 | 5.87 | B4J060-01, B4J084-01 |
| 532.317 | 7.847 | 7.8 | B4J059-01, B4J083-01 |
| 542.338 | 7.85 | 7.8 | B4J037-01, B4J087-01 |
| 492.232 | 6.84 | 6.8 | B4J023-01, B4J077-01 |
| 496.296 | 7.903 | 7.88 | B4J069-01 |
| 501.286 | 7.927 | 7.88 | B4J026-01, B4J050-01 |
| 516.297 | 7.913 | 7.88 | B4J011-01, B4J055-01 |
| 517.292 | 6.21 | 6.15 | B4J012-01, B4J056-01 |
| 543.333 | 6.183 | 6.15 | B4J038-01, B4J088-01 |
| 557.248 | 7.05 | 7.01 | B4J018-01, B4J064-01 |
| 488.302 | 6.973 | 6.93 | B4J073-01 |
| 512.266 | 8.627 | 8.58 | B4J032-01 |
| 557.248 | 8.28 | 8.24 | B4J018-01, B4J064-01 |
| 489.297 | 4.833 | 4.8 | B4J074-01 |
| 505.261 | 8.35 | 8.31 | B4J100-01, B4J028-01, B4J080-01 |
| 543.333 | 9.817 | 9.77 | B4J038-01, B4J088-01 |
| 579.258 | 11.187 | 11.15 | B4J042-01 |
| 503.277 | 10.963 | 10.94 | B4J004-01, B4J054-01, B4J076-01 |
| 504.266 | 10.973 | 10.94 | B4J027-01, B4J079-01, B4J099-01 |

(continued)

| Mixture No. | mixAI-B4J-01 | | |
|---|---|---|---|
| m/z | Retention time of MS peak (min) | Retention time of UV peak (min) | No. of assigned compound |
| 505.261 | 10.953 | 10.94 | B4J100-01, B4J028-01, B4J080-01 |
| 499.282 | 6.097 | 6.05 | B4J096-01 |
| 533.312 | 6.083 | 6.05 | B4J060-01, B4J084-01 |
| 534.307 | 7.197 | 7.16 | B4J085-01 |
| 542.338 | 12.76 | 12.73 | B4J037-01, B4J087-01 |
| 474.286 | 6.553 | 6.53 | B4J071-01 |
| 480.243 | 6.553 | 6.53 | B4J093-01 |
| 531.333 | 6.573 | 6.53 | B4J058-01 |
| 490.286 | 7.45 | 7.42 | B4J051-01 |
| 519.308 | 7.467 | 7.42 | B4J082-01 |
| 504.266 | 6.903 | 6.85 | B4J027-01, B4J079-01, B4J099-01 |
| 507.251 | 6.897 | 6.85 | B4J006-01 |
| 517.292 | 6.887 | 6.85 | B4J012-01, B4J056-01 |
| 541.278 | 6.877 | 6.85 | B4J062-01 |
| 473.291 | 5.777 | 5.73 | B4J046-01 |
| 491.282 | 5.747 | 5.73 | B4J052-01 |
| 535.303 | 11.83 | 11.8 | B4J086-01 |
| 536.291 | 11.84 | 11.8 | B4J035-01 |
| 540.283 | 8.387 | 8.35 | B4J061-01 |
| 545.323 | 8.433 | 8.35 | B4J016-01 |
| 500.291 | 8.027 | 7.98 | B4J025-01, B4J049-01 |
| 558.269 | 8.01 | 7.98 | B4J089-01 |
| 559.264 | 8.023 | 7.98 | B4J090-01 |
| 502.281 | 7.41 | 7.37 | B4J003-01, B4J053-01, B4J075-01 |
| 472.296 | 7.247 | 7.2 | B4J045-01 |
| 478.252 | 7.247 | 7.2 | B4J067-01 |
| 515.302 | 7.25 | 7.2 | B4J034-01 |
| 479.248 | 5.323 | 5.3 | B4J068-01 |
| 503.277 | 5.34 | 5.3 | B4J004-01, B4J054-01, B4J076-01 |
| 525.297 | 5.643 | 5.6 | B4J092-01 |
| 486.312 | 7.597 | 7.57 | B4J047-01, B4J097-01 |
| 487.307 | 7.603 | 7.57 | B4J048-01, B4J098-01 |
| 524.302 | 7.62 | 7.57 | B4J091-01 |
| 532.317 | 7.637 | 7.57 | B4J059-01, B4J083-01 |

(continued)

| Mixture No. | mixAI-B4J-01 | | |
|---|---|---|---|
| m/z | Retention time of MS peak (min) | Retention time of UV peak (min) | No. of assigned compound |
| 493.227 | 7.79 | 7.75 | B4J024-01, B4J078-01 |
| 497.291 | 7.74 | 7.75 | B4J070-01 |
| 504.266 | 7.72 | 7.75 | B4J027-01, B4J079-01, B4J099-01 |
| 514.256 | 7.76 | 7.75 | B4J100-01, B4J033-01 |
| 544.328 | 11.46 | 11.43 | B4J015-01 |
| 486.312 | 7.577 | 7.5 | B4J047-01, B4J097-01 |
| 502.281 | 7.537 | 7.5 | B4J003-01, B4J053-01, B4J075-01 |
| 493.227 | 5.027 | 4.95 | B4J024-01, B4J078-01 |
| 505.261 | 4.953 | 4.88 | B4J100-01, B4J028-01, B4J080-01 |
| 523.307 | 6.3 | 6.26 | B4J066-01 |

[Table 271]

| Mixture No. | mixAI-B2A-01 | | |
|---|---|---|---|
| m/z | Retention time of MS peak (min) | Retention time of UV peak (min) | No. of assigned compound |
| 647.306 | 8.303 | 8.26 | B2A052-01 |
| 698.362 | 11.333 | 11.3 | B2A037-01, B2A087-01 |
| 662.281 | 13.107 | 13.06 | B2A005-01 |
| 669.285 | 12.713 | 12.68 | B2A009-01 |
| 776.309 | 14.293 | 14.25 | B2A043-01 |
| 778.299 | 14.103 | 14.06 | B2A021-01 |
| 694.306 | 13.833 | 13.8 | B2A013-01 |
| 712.278 | 13.853 | 13.8 | B2A017-01, B2A063-01 |
| 692.315 | 14.05 | 14.01 | B2A035-01 |
| 710.287 | 14.053 | 14.01 | B2A039-01 |
| 696.308 | 10.823 | 10.8 | B2A061-01 |
| 630.311 | 9.62 | 9.58 | B2A071-01 |
| 634.277 | 9.627 | 9.58 | B2A067-01 |
| 688.341 | 9.623 | 9.58 | B2A059-01, B2A083-01 |
| 650.247 | 13.01 | 12.96 | B2A001-01 |
| 667.295 | 12.997 | 12.96 | B2A031-01 |
| 636.267 | 9.873 | 9.81 | B2A093-01 |
| 648.256 | 9.847 | 9.81 | B2A023-01, B2A077-01 |
| 649.251 | 9.86 | 9.81 | B2A024-01, B2A078-01 |
| 689.337 | 8.733 | 8.69 | B2A060-01, B2A084-01 |

(continued)

| Mixture No. | mixAl-B2A-01 | | |
|---|---|---|---|
| m/z | Retention time of MS peak (min) | Retention time of UV peak (min) | No. of assigned compound |
| 654.311 | 10.337 | 10.3 | B2A095-01 |
| 668.29 | 11.513 | 11.44 | B2A032-01 |
| 714.293 | 11.483 | 11.44 | B2A089-01 |
| 628.32 | 9.49 | 9.44 | B2A045-01 |
| 698.362 | 14.7 | 14.66 | B2A037-01, B2A087-01 |
| 734.287 | 14.697 | 14.66 | B2A041-01 |
| 670.281 | 13.703 | 13.66 | B2A100-01, B2A033-01 |
| 711.282 | 12.367 | 12.34 | B2A040-01 |
| 777.304 | 12.387 | 12.34 | B2A044-01 |
| 693.311 | 11.777 | 11.74 | B2A036-01 |
| 701.348 | 12.313 | 12.27 | B2A016-01 |
| 656.315 | 13.647 | 13.6 | B2A025-01, B2A049-01 |
| 700.353 | 14.517 | 14.48 | B2A015-01 |
| 736.278 | 14.52 | 14.48 | B2A019-01 |
| 652.32 | 10.057 | 10.02 | B2A069-01 |
| 713.273 | 12.06 | 12.02 | B2A018-01, B2A064-01 |
| 779.295 | 12.063 | 12.02 | B2A022-01 |
| 659.301 | 10.67 | 10.63 | B2A004-01, B2A054-01, B2A076-01 |
| 658.306 | 13.383 | 13.33 | B2A003-01, B2A053-01, B2A075-01 |
| 659.301 | 13.387 | 13.33 | B2A004-01, B2A054-01, B2A076-01 |
| 660.29 | 13.36 | 13.33 | B2A027-01, B2A079-01, B2A099-01 |
| 678.336 | 10.563 | 10.53 | B2A065-01 |
| 672.321 | 13.503 | 13.47 | B2A011-01, B2A055-01 |
| 651.242 | 10.53 | 10.49 | B2A002-01 |
| 659.301 | 8.603 | 8.57 | B2A004-01, B2A054-01, B2A076-01 |
| 686.362 | 10.103 | 10.07 | B2A057-01 |
| 631.306 | 8.45 | 8.41 | B2A072-01 |
| 675.332 | 8.43 | 8.41 | B2A082-01 |
| 735.282 | 13.473 | 13.43 | B2A042-01 |
| 673.317 | 10.9 | 10.86 | B2A012-01, B2A056-01 |
| 642.336 | 7.79 | 7.77 | B2A047-01, B2A097-01 |
| 661.285 | 7.807 | 7.77 | B2A100-01, B2A028-01, B2A080-01 |

(continued)

| Mixture No. | mixAl-B2A-01 | | |
|---|---|---|---|
| m/z | Retention time of MS peak (min) | Retention time of UV peak (min) | No. of assigned compound |
| 637.263 | 10.957 | 10.93 | B2A094-01 |
| 649.251 | 10.957 | 10.93 | B2A024-01, B2A078-01 |
| 680.327 | 10.96 | 10.93 | B2A091-01 |
| 712.278 | 10.96 | 10.93 | B2A017-01, B2A063-01 |
| 699.357 | 12.62 | 12.59 | B2A038-01, B2A088-01 |
| 737.273 | 13.21 | 13.17 | B2A020-01 |
| 658.306 | 10.487 | 10.45 | B2A003-01, B2A053-01, B2A075-01 |
| 660.29 | 9.263 | 9.23 | B2A027-01, B2A079-01, B2A099-01 |
| 661.285 | 9.28 | 9.23 | B2A100-01, B2A028-01, B2A080-01 |
| 648.256 | 13.293 | 13.25 | B2A023-01, B2A077-01 |
| 635.272 | 7.977 | 7.94 | B2A068-01 |
| 643.331 | 7.957 | 7.94 | B2A048-01, B2A098-01 |
| 643.331 | 7.923 | 7.94 | B2A048-01, B2A098-01 |
| 679.331 | 8.89 | 8.86 | B2A066-01 |
| 661.285 | 11.427 | 11.39 | B2A100-01, B2A028-01, B2A080-01 |
| 695.301 | 11.42 | 11.39 | B2A014-01 |
| 681.322 | 9.05 | 9.01 | B2A092-01 |
| 658.306 | 9.397 | 9.36 | B2A003-01, B2A053-01, B2A075-01 |
| 697.303 | 9.383 | 9.36 | B2A062-01 |
| 657.311 | 11.077 | 11.04 | B2A026-01, B2A050-01 |
| 663.276 | 11.047 | 11.04 | B2A006-01 |
| 657.311 | 8.563 | 8.52 | B2A026-01, B2A050-01 |
| 642.336 | 9.55 | 9.5 | B2A047-01, B2A097-01 |
| 644.327 | 9.563 | 9.5 | B2A073-01 |
| 645.322 | 9.533 | 9.5 | B2A074-01 |
| 713.273 | 9.53 | 9.5 | B2A018-01, B2A064-01 |
| 671.326 | 11.263 | 11.23 | B2A034-01 |
| 655.306 | 9.19 | 9.16 | B2A096-01 |
| 656.315 | 10.217 | 10.16 | B2A025-01, B2A049-01 |
| 688.341 | 10.19 | 10.16 | B2A059-01, B2A083-01 |
| 689.337 | 10.203 | 10.16 | B2A060-01, B2A084-01 |
| 687.357 | 8.79 | 8.75 | B2A058-01 |

(continued)

| Mixture No. | mixAl-B2A-01 | | |
|---|---|---|---|
| m/z | Retention time of MS peak (min) | Retention time of UV peak (min) | No. of assigned compound |
| 629.316 | 8.38 | 8.34 | B2A046-01 |
| 690.332 | 9.45 | 9.41 | B2A085-01 |
| 691.327 | 9.463 | 9.41 | B2A086-01 |
| 673.317 | 8.513 | 8.47 | B2A012-01, B2A056-01 |
| 670.281 | 11.157 | 11.12 | B2A100-01, B2A033-01 |
| 660.29 | 9.9 | 9.85 | B2A027-01, B2A079-01, B2A099-01 |
| 674.337 | 9.893 | 9.85 | B2A081-01 |
| 646.311 | 9.67 | 9.63 | B2A051-01 |
| 699.357 | 9.66 | 9.63 | B2A038-01, B2A088-01 |
| 653.316 | 9.09 | 9.05 | B2A070-01 |
| 672.321 | 9.84 | 9.77 | B2A011-01, B2A055-01 |
| 715.288 | 9.827 | 9.77 | B2A090-01 |

[Table 272]

| Mixture No. | mixAl-B21-01 | | |
|---|---|---|---|
| m/z | Retention (min) | time of MS peak Retention time of UV peak (min) | No. of assigned compound |
| 578.168 | 10.283 | 10.24 | B2I017-01, B2I063-01 |
| 643.194 | 8.707 | 8.67 | B2I044-01 |
| 539.207 | 6.45 | 6.43 | B2I012-01, B2I056-01 |
| 494.211 | 6.56 | 6.52 | B2I045-01 |
| 514.146 | 6.55 | 6.52 | B2I023-01, B2I077-01 |
| 510.217 | 6.663 | 6.63 | B2I073-01 |
| 524.196 | 9.403 | 9.35 | B2I003-01, B2I053-01, B2I075-01 |
| 533.185 | 9.383 | 9.35 | B2I031-01 |
| 535.176 | 8.773 | 8.74 | B2I009-01 |
| 578.168 | 8.08 | 8.04 | B2I017-01, B2I063-01 |
| 645.185 | 8.043 | 8 | B2I022-01 |
| 495.206 | 9.223 | 9.14 | B2I046-01 |
| 528.171 | 9.177 | 9.14 | B2I005-01 |
| 562.198 | 7.9 | 7.87 | B2I061-01 |
| 560.196 | 9.997 | 9.96 | B2I013-01 |
| 500.167 | 10.833 | 10.78 | B2I067-01 |
| 576.177 | 10.83 | 10.78 | B2I039-01 |
| 644.19 | 10.82 | 10.78 | B2I021-01 |

(continued)

| Mixture No. | mixAl-B21-01 | | |
|---|---|---|---|
| m/z | Retention (min) | time of MS peak Retention time of UV peak (min) | No. of assigned compound |
| 581.179 | 6.163 | 6.13 | B2I090-01 |
| 496.201 | 6.243 | 6.21 | B2I071-01 |
| 563.193 | 6.23 | 6.21 | B2I062-01 |
| 539.207 | 9.527 | 9.49 | B2I012-01, B2I056-01 |
| 497.197 | 7.76 | 7.73 | B2I072-01 |
| 564.253 | 7.767 | 7.73 | B2I037-01, B2I087-01 |
| 558.206 | 10.583 | 10.54 | B2I035-01 |
| 547.212 | 11.293 | 11.25 | B2I092-01 |
| 602.168 | 11.263 | 11.25 | B2I019-01 |
| 642.199 | 11.297 | 11.25 | B2I043-01 |
| 567.238 | 7.99 | 7.92 | B2I016-01 |
| 580.184 | 7.947 | 7.92 | B2I089-01 |
| 559.201 | 7.623 | 7.58 | B2I036-01 |
| 512.201 | 6.897 | 6.86 | B2I051-01 |
| 556.222 | 6.907 | 6.86 | B2I085-01 |
| 557.218 | 6.91 | 6.86 | B2I086-01 |
| 577.173 | 8.553 | 8.52 | B2I040-01 |
| 516.137 | 8.963 | 8.92 | B2I001-01 |
| 523.201 | 11.767 | 11.73 | B2I026-01, B2I050-01 |
| 600.177 | 11.773 | 11.73 | B2I041-01 |
| 523.201 | 6.803 | 6.75 | B2I026-01, B2I050-01 |
| 553.248 | 5.95 | 5.92 | B2I058-01 |
| 565.248 | 5.967 | 5.92 | B2I038-01, B2I088-01 |
| 534.181 | 7.583 | 7.53 | B2I032-01 |
| 565.248 | 8.673 | 8.59 | B2I038-01, B2I088-01 |
| 520.201 | 7.073 | 7.04 | B2I095-01 |
| 540.228 | 7.083 | 7.04 | B2I081-01 |
| 508.226 | 7.033 | 6.99 | B2I047-01, B2I097-01 |
| 524.196 | 6.97 | 6.93 | B2I003-01, B2I053-01, B2I075-01 |
| 502.158 | 7.343 | 7.24 | B2I093-01 |
| 515.142 | 7.257 | 7.24 | B2I024-01, B2I078-01 |
| 518.211 | 7.277 | 7.24 | B2I069-01 |
| 554.232 | 7.283 | 7.24 | B2I059-01, B2I083-01 |
| 538.212 | 7.393 | 7.35 | B2I011-01, B2I055-01 |
| 541.223 | 5.193 | 5.16 | B2I082-01 |

(continued)

| Mixture No. | mixAl-B21-01 | | |
|---|---|---|---|
| m/z | Retention (min) | time of MS peak Retention time of UV peak (min) | No. of assigned compound |
| 526.181 | 10.94 | 10.92 | B2I027-01, B2I079-01, B2I099-01 |
| 566.243 | 10.95 | 10.92 | B2I015-01 |
| 515.142 | 7.233 | 7.17 | B2I024-01, B2I078-01 |
| 524.196 | 7.207 | 7.17 | B2I003-01, B2I053-01, B2I075-01 |
| 509.222 | 4.293 | 4.3 | B2I048-01, B2I098-01 |
| 564.253 | 11.473 | 11.44 | B2I037-01, B2I087-01 |
| 522.206 | 10.05 | 10.03 | B2I025-01, B2I049-01 |
| 527.176 | 10.047 | 10.03 | B2I100-01, B2I028-01, B2I080-01 |
| 536.171 | 10.053 | 10.03 | B2I100-01, B2I033-01 |
| 536.171 | 10.077 | 10.03 | B2I100-01, B2I033-01 |
| 601.173 | 10.073 | 10.03 | B2I042-01 |
| 525.191 | 9.813 | 9.79 | B2I004-01, B2I054-01, B2I076-01 |
| 525.191 | 9.84 | 9.79 | B2I004-01, B2I054-01, B2I076-01 |
| 526.181 | 9.83 | 9.79 | B2I027-01, B2I079-01, B2I099-01 |
| 526.181 | 9.853 | 9.79 | B2I027-01, B2I079-01, B2I099-01 |
| 527.176 | 9.803 | 9.79 | B2I100-01, B2I028-01, B2I080-01 |
| 579.163 | 6.417 | 6.34 | B2I018-01, B2I064-01 |
| 513.197 | 9.477 | 9.44 | B2I052-01 |
| 538.212 | 9.51 | 9.44 | B2I011-01, B2I055-01 |
| 603.163 | 9.48 | 9.44 | B2I020-01 |
| 554.232 | 7.417 | 7.38 | B2I059-01, B2I083-01 |
| 555.227 | 7.427 | 7.38 | B2I060-01, B2I084-01 |
| 501.162 | 4.503 | 4.47 | B2I068-01 |
| 579.163 | 7.847 | 7.81 | B2I018-01, B2I064-01 |
| 544.226 | 7.683 | 7.66 | B2I065-01 |
| 545.222 | 7.7 | 7.66 | B2I066-01 |
| 552.253 | 7.707 | 7.66 | B2I057-01 |
| 514.146 | 9.65 | 9.61 | B2I023-01, B2I077-01 |
| 529.166 | 9.647 | 9.61 | B2I006-01 |
| 503.153 | 8.893 | 8.86 | B2I094-01 |
| 517.132 | 7.47 | 7.44 | B2I002-01 |

(continued)

| Mixture No. | mixAI-B21-01 | | |
|---|---|---|---|
| m/z | Retention (min) | time of MS peak Retention time of UV peak (min) | No. of assigned compound |
| 519.206 | 7.49 | 7.44 | B2I070-01 |
| 521.197 | 7.473 | 7.44 | B2I096-01 |
| 522.206 | 7.493 | 7.44 | B2I025-01, B2I049-01 |
| 546.217 | 7.473 | 7.44 | 821091-01 |
| 527.176 | 7.197 | 7.13 | B2I100-01, B2I028-01, B2I080-01 |
| 537.217 | 7.167 | 7.13 | B2I034-01 |
| 509.222 | 4.033 | 4.1 | B2I048-01, B2I098-01 |
| 511.212 | 10.663 | 10.62 | B2I074-01 |
| 555.227 | 5.387 | 5.35 | B2I060-01, B2I084-01 |
| 561.191 | 6.867 | 6.82 | B2I014-01 |
| 525.191 | 5.987 | 5.95 | B2I004-01, B2I054-01, B2I076-01 |

[Table 273]

| Mixture No. | mixAI-B2L-01 | | |
|---|---|---|---|
| m/z | Retention time of MS peak (min) | Retention time of UV peak (min) | No. of assigned compound |
| 577.223 | 9.913 | 9.87 | B2L009-01 |
| 570.218 | 10.347 | 10.31 | B2L005-01 |
| 622.231 | 8.887 | 8.85 | B2L089-01 |
| 686.237 | 11.807 | 11.77 | B2L021-01 |
| 606.3 | 8.713 | 8.68 | B2L037-01, B2L087-01 |
| 607.295 | 6.94 | 6.91 | B2L038-01, B2L088-01 |
| 562.248 | 7.94 | 7.9 | B2L095-01 |
| 575.232 | 10.247 | 10.21 | B2L031-01 |
| 642.224 | 12.527 | 12.49 | B2L041-01 |
| 687.232 | 9.203 | 9.16 | B2L022-01 |
| 588.264 | 8.39 | 8.35 | B2L091-01 |
| 618.224 | 11.63 | 11.59 | B2L039-01 |
| 538.248 | 7.163 | 7.13 | B2L071-01 |
| 559.179 | 7.153 | 7.13 | B2L002-01 |
| 623.226 | 7.15 | 7.13 | B2L090-01 |
| 602.243 | 11.173 | 11.13 | B2L013-01 |
| 589.259 | 6.277 | 6.24 | B2L092-01 |
| 568.228 | 10.697 | 10.67 | B2L027-01, B2L079-01, B2L099-01 |
| 580.259 | 10.717 | 10.67 | B2L011-01, B2L055-01 |

(continued)

| Mixture No. | mixAI-B2L-01 | | |
|---|---|---|---|
| m/z | Retention time of MS peak (min) | Retention time of UV peak (min) | No. of assigned compound |
| 595.295 | 11.37 | 11.33 | B2L058-01 |
| 620.215 | 11.373 | 11.33 | B2L017-01, B2L063-01 |
| 609.285 | 9.23 | 9.19 | B2L016-01 |
| 551.269 | 7.413 | 7.4 | B2L048-01, B2L098-01 |
| 552.264 | 7.42 | 7.4 | B2L073-01 |
| 556.193 | 7.437 | 7.4 | B2L023-01, B2L077-01 |
| 608.29 | 12.07 | 12.01 | B2L015-01 |
| 684.246 | 12.047 | 12.01 | B2L043-01 |
| 606.3 | 12.317 | 12.24 | B2L037-01, B2L087-01 |
| 644.215 | 12.287 | 12.24 | B2L019-01 |
| 607.295 | 9.607 | 9.54 | B2L038-01, B2L088-01 |
| 685.241 | 9.563 | 9.54 | B2L044-01 |
| 539.244 | 5.697 | 5.66 | B2L072-01 |
| 600.253 | 11.463 | 11.42 | B2L035-01 |
| 563.244 | 6.75 | 6.69 | B2L096-01 |
| 621.21 | 9.093 | 9.05 | B2L018-01, B2L064-01 |
| 553.259 | 5.27 | 5.25 | B2L074-01 |
| 557.189 | 5.603 | 5.56 | B2L024-01, B2L078-01 |
| 603.238 | 8.187 | 8.15 | B2L014-01 |
| 579.264 | 8.127 | 8.09 | B2L034-01 |
| 596.279 | 8.12 | 8.09 | B2L059-01, B2L083-01 |
| 571.213 | 7.733 | 7.7 | B2L006-01 |
| 556.193 | 10.547 | 10.56 | B2L023-01, B2L077-01 |
| 566.243 | 10.603 | 10.56 | B2L003-01, B2L053-01, B2L075-01 |
| 645.21 | 10.587 | 10.56 | B2L020-01 |
| 536.258 | 7.24 | 7.2 | B2L045-01 |
| 544.205 | 7.27 | 7.2 | B2L093-01 |
| 537.253 | 5.807 | 5.75 | B2L046-01 |
| 601.248 | 8.613 | 8.58 | B2L036-01 |
| 564.253 | 10.953 | 10.92 | B2L025-01, B2L049-01 |
| 578.218 | 10.963 | 10.92 | B2L100-01, B2L033-01 |
| 578.218 | 10.973 | 10.92 | B2L100-01, B2L033-01 |
| 581.254 | 6.23 | 6.13 | B2L012-01, B2L056-01 |
| 542.214 | 7.297 | 7.27 | B2L067-01 |
| 567.238 | 7.31 | 7.27 | B2L004-01, B2L054-01, B2L076-01 |

(continued)

| Mixture No. | mixAl-B2L-01 | | |
|---|---|---|---|
| m/z | Retention time of MS peak (min) | Retention time of UV peak (min) | No. of assigned compound |
| 569.223 | 7.303 | 7.27 | B2L100-01, B2L028-01, B2L080-01 |
| 569.223 | 8.233 | 8.18 | B2L100-01, B2L028-01, B2L080-01 |
| 594.3 | 8.22 | 8.18 | B2L057-01 |
| 557.189 | 7.697 | 7.65 | B2L024-01, B2L078-01 |
| 581.254 | 7.693 | 7.65 | B2L012-01, B2L056-01 |
| 567.238 | 5.913 | 5.88 | B2L004-01, B2L054-01, B2L076-01 |
| 545.2 | 5.15 | 5.12 | B2L094-01 |
| 576.228 | 8.517 | 8.48 | B2L032-01 |
| 604.245 | 8.52 | 8.48 | B2L061-01 |
| 586.273 | 8.293 | 8.26 | B2L065-01 |
| 583.27 | 6.03 | 6.01 | B2L082-01 |
| 597.274 | 6.047 | 6.01 | B2L060-01, B2L084-01 |
| 619.22 | 9.473 | 9.43 | B2L040-01 |
| 564.253 | 8.067 | 8.03 | B2L025-01, B2L049-01 |
| 567.238 | 8.06 | 8.03 | B2L004-01, B2L054-01, B2L076-01 |
| 568.228 | 8.073 | 8.03 | B2L027-01, B2L079-01, B2L099-01 |
| 569.223 | 5.383 | 5.34 | B2L100-01, B2L028-01, B2L080-01 |
| 587.269 | 6.36 | 6.36 | B2L066-01 |
| 597.274 | 6.39 | 6.36 | B2L060-01, B2L084-01 |
| 543.209 | 10.21 | 10.14 | B2L068-01 |
| 558.184 | 10.19 | 10.14 | B2L001-01 |
| 565.248 | 7.817 | 7.77 | B2L026-01, B2L050-01 |
| 566.243 | 7.817 | 7.77 | B2L003-01, B2L053-01, B2L075-01 |
| 582.274 | 7.8 | 7.77 | B2L081-01 |
| 596.279 | 7.81 | 7.77 | B2L059-01, B2L083-01 |
| 605.24 | 8.7 | 8.64 | B2L062-01 |
| 620.215 | 8.68 | 8.64 | B2L017-01, B2L063-01 |
| 560.258 | 7.89 | 7.85 | B2L069-01 |
| 580.259 | 7.923 | 7.85 | B2L011-01, B2L055-01 |
| 550.273 | 7.58 | 7.55 | B2L047-01, B2L097-01 |
| 598.269 | 7.597 | 7.55 | B2L085-01 |
| 599.265 | 7.597 | 7.55 | B2L086-01 |

(continued)

| Mixture No. | mixAl-B2L-01 | | |
|---|---|---|---|
| m/z | Retention time of MS peak (min) | Retention time of UV peak (min) | No. of assigned compound |
| 554.248 | 7.493 | 7.47 | B2L051-01 |
| 555.244 | 7.503 | 7.47 | B2L052-01 |
| 566.243 | 7.51 | 7.47 | B2L003-01, B2L053-01, B2L075-01 |
| 568.228 | 7.513 | 7.47 | B2L027-01, B2L079-01, B2L099-01 |
| 561.253 | 6.817 | 6.77 | B2L070-01 |
| 565.248 | 10.933 | 10.86 | B2L026-01, B2L050-01 |
| 643.22 | 10.9 | 10.86 | B2L042-01 |
| 621.21 | 7.127 | 7.05 | B2L018-01, B2L064-01 |
| 550.273 | 11.953 | 11.91 | B2L047-01, B2L097-01 |
| 551.269 | 11.96 | 11.91 | B2L048-01, B2L098-01 |

[Table 274]

| Mixture No. | mixAl-B2N-01 | | |
|---|---|---|---|
| m/z | Retention time of MS peak (min) | Retention time of UV peak (min) | No. of assigned compound |
| 576.214 | 7.78 | 7.73 | B2N045-01 |
| 724.202 | 12.373 | 12.34 | B2N043-01 |
| 634.256 | 8.673 | 8.64 | B2N057-01 |
| 682.18 | 12.813 | 12.78 | B2N041-01 |
| 658.18 | 12.01 | 11.97 | B2N039-01 |
| 607.194 | 6.177 | 6.14 | B2N004-01, B2N054-01, B2N076-01 |
| 608.184 | 11.17 | 11.13 | B2N027-01, B2N079-01, B2N099-01 |
| 609.179 | 6.48 | 6.45 | B2N100-01, B2N028-01, B2N080-01 |
| 644.201 | 9.057 | 9.01 | B2N061-01 |
| 591.225 | 6.07 | 6.02 | B2N048-01, B2N098-01 |
| 635.251 | 7.127 | 7.09 | B2N058-01 |
| 593.215 | 8.077 | 8.03 | B2N074-01 |
| 617.179 | 10.133 | 10.09 | B2N009-01 |
| 725.198 | 10.12 | 10.09 | B2N044-01 |
| 597.145 | 8.41 | 8.35 | B2N024-01, B2N078-01 |
| 600.214 | 8.383 | 8.35 | B2N069-01 |
| 620.215 | 8.39 | 8.35 | B2N011-01, B2N055-01 |
| 636.235 | 8.413 | 8.35 | B2N059-01, B2N083-01 |

(continued)

| Mixture No. | mixAl-B2N-01 | | |
|---|---|---|---|
| m/z | Retention time of MS peak (min) | Retention time of UV peak (min) | No. of assigned compound |
| 641.204 | 9.27 | 9.23 | B2N036-01 |
| 621.21 | 8.01 | 7.95 | B2N012-01, B2N056-01 |
| 605.204 | 8.53 | 8.49 | B2N026-01, B2N050-01 |
| 604.209 | 8.573 | 8.54 | B2N025-01, B2N049-01 |
| 604.209 | 11.407 | 11.36 | B2N025-01, B2N049-01 |
| 627.225 | 6.977 | 6.93 | B2N066-01 |
| 619.22 | 8.813 | 8.78 | B2N034-01 |
| 626.229 | 8.823 | 8.78 | B2N065-01 |
| 606.199 | 10.757 | 10.72 | B2N003-01, B2N053-01, B2N075-01 |
| 615.188 | 10.76 | 10.72 | B2N031-01 |
| 685.166 | 10.753 | 10.72 | B2N020-01 |
| 660.171 | 11.477 | 11.44 | B2N017-01, B2N063-01 |
| 601.209 | 7.33 | 7.29 | B2N070-01 |
| 578.204 | 7.487 | 7.45 | B2N071-01 |
| 577.209 | 6.38 | 6.31 | B2N046-01 |
| 595.2 | 6.35 | 6.31 | B2N052-01 |
| 645.196 | 7.54 | 7.49 | B2N062-01 |
| 621.21 | 6.79 | 6.75 | B2N012-01, B2N056-01 |
| 683.176 | 11.347 | 11.31 | B2N042-01 |
| 590.229 | 8.06 | 7.99 | B2N047-01, B2N097-01 |
| 594.204 | 8.023 | 7.99 | B2N051-01 |
| 611.169 | 8.047 | 7.99 | B2N006-01 |
| 609.179 | 8.933 | 8.89 | B2N100-01, B2N028-01, B2N080-01 |
| 585.156 | 7.603 | 7.57 | B2N094-01 |
| 607.194 | 7.61 | 7.57 | B2N004-01, B2N054-01, B2N076-01 |
| 639.221 | 11.867 | 11.85 | B2N086-01 |
| 726.193 | 11.883 | 11.85 | B2N021-01 |
| 727.188 | 11.89 | 11.85 | B2N022-01 |
| 598.14 | 10.373 | 10.33 | B2N001-01 |
| 602.204 | 8.233 | 8.21 | B2N095-01 |
| 603.2 | 8.247 | 8.21 | B2N096-01 |
| 608.184 | 8.227 | 8.21 | B2N027-01, B2N079-01, B2N099-01 |
| 636.235 | 8.243 | 8.21 | B2N059-01, B2N083-01 |

(continued)

| Mixture No. | mixAl-B2N-01 | | |
|---|---|---|---|
| m/z | Retention time of MS peak (min) | Retention time of UV peak (min) | No. of assigned compound |
| 629.215 | 6.537 | 6.51 | B2N092-01 |
| 637.23 | 6.533 | 6.51 | B2N060-01, B2N084-01 |
| 620.215 | 10.873 | 10.83 | B2N011-01, B2N055-01 |
| 646.256 | 12.643 | 12.61 | B2N037-01, B2N087-01 |
| 661.166 | 9.36 | 9.32 | B2N018-01, B2N064-01 |
| 583.166 | 5.883 | 5.85 | B2N068-01 |
| 597.145 | 5.897 | 5.85 | B2N024-01, B2N078-01 |
| 582.17 | 7.853 | 7.82 | B2N067-01 |
| 638.225 | 7.87 | 7.82 | B2N085-01 |
| 599.135 | 7.19 | 7.17 | B2N002-01 |
| 642.199 | 11.28 | 11.25 | B2N013-01 |
| 618.174 | 11.467 | 11.4 | B2N100-01, B2N033-01 |
| 618.174 | 11.437 | 11.4 | B2N100-01, B2N033-01 |
| 579.2 | 6 | 5.96 | B2N072-01 |
| 646.256 | 9.023 | 8.97 | B2N037-01, B2N087-01 |
| 647.251 | 9.037 | 8.97 | B2N038-01, B2N088-01 |
| 592.22 | 7.73 | 7.69 | B2N073-01 |
| 596.15 | 7.76 | 7.69 | B2N023-01, B2N077-01 |
| 661.166 | 7.717 | 7.69 | B2N018-01, B2N064-01 |
| 647.251 | 10.18 | 10.15 | B2N038-01, B2N088-01 |
| 648.246 | 10.193 | 10.15 | B2N015-01 |
| 591.225 | 11.047 | 11 | B2N048-01, B2N098-01 |
| 596.15 | 11.043 | 11 | B2N023-01, B2N077-01 |
| 637.23 | 10.947 | 10.89 | B2N060-01, B2N084-01 |
| 623.226 | 6.313 | 6.26 | B2N082-01 |
| 590.229 | 7.95 | 7.92 | B2N047-01, B2N097-01 |
| 605.204 | 7.963 | 7.92 | B2N026-01, B2N050-01 |
| 606.199 | 7.977 | 7.92 | B2N003-01, B2N053-01, B2N075-01 |
| 607.194 | 7.957 | 7.92 | B2N004-01, B2N054-01, B2N076-01 |
| 643.194 | 8.48 | 8.42 | B2N014-01 |
| 628.22 | 8.667 | 8.6 | B2N091-01 |
| 622.231 | 8.097 | 8.06 | B2N081-01 |
| 606.199 | 8.35 | 8.28 | B2N003-01, B2N053-01, B2N075-01 |
| 584.161 | 7.59 | 7.53 | B2N093-01 |

(continued)

| Mixture No. | mixAI-B2N-01 | | |
|---|---|---|---|
| m/z | Retention time of MS peak (min) | Retention time of UV peak (min) | No. of assigned compound |
| 608.184 | 7.597 | 7.53 | B2N027-01, B2N079-01, B2N099-01 |
| 609.179 | 7.593 | 7.53 | B2N100-01, B2N028-01, B2N080-01 |
| 640.209 | 11.863 | 11.78 | B2N035-01 |
| 649.241 | 9.477 | 9.43 | B2N016-01 |
| 659.176 | 10.053 | 10.01 | B2N040-01 |
| 660.171 | 9.193 | 9.15 | B2N017-01, B2N063-01 |
| 684.171 | 12.33 | 12.28 | B2N019-01 |
| 610.174 | 10.527 | 10.48 | B2N005-01 |
| 616.184 | 9.16 | 9.13 | B2N032-01 |
| 662.187 | 9.183 | 9.13 | B2N089-01 |
| 663.182 | 9.177 | 9.13 | B2N090-01 |

[Table 275]

| Mixture No. | mixAI-B2R-01 | | |
|---|---|---|---|
| mlz | Retention time of MS peak (min) | Retention time of UV peak (min) | No. of assigned compound |
| 814.234 | 12.897 | 12.87 | B2R031-01 |
| 857.226 | 13.81 | 13.76 | B2R039-01 |
| 775.259 | 9.847 | 9.81 | B2R045-01 |
| 816.224 | 12.68 | 12.65 | B2R009-01 |
| 784.202 | 8.677 | 8.64 | B2R094-01 |
| 923.248 | 14.02 | 13.98 | B2R043-01 |
| 841.245 | 9.233 | 9.2 | B2R013-01 |
| 817.22 | 13.487 | 13.44 | B2R100-01, B2R033-01 |
| 803.254 | 13.403 | 13.38 | B2R025-01, B2R049-01 |
| 881.226 | 14.387 | 14.35 | B2R041-01 |
| 859.217 | 13.63 | 13.59 | B2R017-01, B2R063-01 |
| 840.25 | 11.85 | 11.81 | B2R036-01 |
| 839.254 | 13.77 | 13.73 | B2R035-01 |
| 884.212 | 13.103 | 13.09 | B2R020-01 |
| 846.297 | 12.55 | 12.52 | B2R038-01, B2R088-01 |
| 800.255 | 9.48 | 9.43 | B2R070-01 |
| 776.255 | 8.947 | 8.89 | B2R046-01 |
| 858.221 | 12.367 | 12.33 | B2R040-01 |
| 924.243 | 12.377 | 12.33 | B2R044-01 |

(continued)

| Mixture No. | mixAI-B2R-01 | | |
|---|---|---|---|
| mlz | Retention time of MS peak (min) | Retention time of UV peak (min) | No. of assigned compound |
| 860.212 | 12.14 | 12.1 | B2R018-01, B2R064-01 |
| 926.234 | 12.137 | 12.1 | B2R022-01 |
| 925.238 | 13.863 | 13.82 | B2R021-01 |
| 825.275 | 10.807 | 10.77 | B2R065-01 |
| 848.287 | 12.313 | 12.28 | B2RO16-01 |
| 827.266 | 11.157 | 11.1 | B2R091-01 |
| 828.261 | 11.14 | 11.1 | B2R092-01 |
| 859.217 | 11.133 | 11.1 | B2R017-01, B2R063-01 |
| 819.26 | 10.157 | 10.12 | B2R011-01, B2R055-01 |
| 838.266 | 8.807 | 8.77 | B2R086-01 |
| 807.229 | 9.667 | 9.62 | B2R027-01, B2R079-01, B2R099-01 |
| 808.224 | 9.67 | 9.62 | B2R100-01, B2R028-01, B2R080-01 |
| 799.259 | 10.277 | 10.24 | B2R069-01 |
| 782.211 | 8.62 | 8.51 | B2R068-01 |
| 790.27 | 8.557 | 8.51 | B2R048-01, B2R098-01 |
| 809.22 | 8.537 | 8.51 | B2R005-01 |
| 843.247 | 11.033 | 10.99 | B2R061-01 |
| 844.242 | 11.057 | 10.99 | B2R062-01 |
| 805.245 | 9.773 | 9.74 | B2R003-01, B2R053-01, B2R075-01 |
| 837.271 | 9.813 | 9.74 | B2R085-01 |
| 808.224 | 11.577 | 11.54 | B2R100-01, B2R028-01, B2R080-01 |
| 842.24 | 11.58 | 11.54 | B2R014-01 |
| 861.232 | 11.58 | 11.54 | B2R089-01 |
| 805.245 | 13.207 | 13.16 | B2R003-01, B2R053-01, B2R075-01 |
| 806.24 | 13.2 | 13.16 | B2R004-01, B2R054-01, B2R076-01 |
| 807.229 | 13.193 | 13.16 | B2R027-01, B2R079-01, B2R099-01 |
| 817.22 | 11.417 | 11.38 | B2R100-01, B2R033-01 |
| 805.245 | 10.737 | 10.7 | B2R003-01, B2R053-01, B2R075-01 |
| 798.181 | 10.89 | 10.85 | B2R002-01 |
| 783.206 | 10.217 | 10.17 | B2R093-01 |

(continued)

| Mixture No. | mixAI-B2R-01 | | |
|---|---|---|---|
| mlz | Retention time of MS peak (min) | Retention time of UV peak (min) | No. of assigned compound |
| 795.195 | 10.203 | 10.17 | B2R023-01, B2R077-01 |
| 821.276 | 10.2 | 10.17 | B2R081-01 |
| 862.227 | 10.197 | 10.17 | B2R090-01 |
| 847.292 | 14.183 | 14.19 | B2R015-01 |
| 883.217 | 14.23 | 14.19 | B2R019-01 |
| 804.25 | 9.097 | 9.05 | B2R026-01, B2R050-01 |
| 819.26 | 13.303 | 13.27 | B2R011-01, B2R055-01 |
| 882.221 | 13.307 | 13.27 | B2R042-01 |
| 815.229 | 11.683 | 11.64 | B2R032-01 |
| 801.25 | 10.537 | 10.46 | B2R095-01 |
| 802.245 | 10.537 | 10.46 | B2R096-01 |
| 803.254 | 10.493 | 10.46 | B2R025-01, B2R049-01 |
| 835.281 | 10.467 | 10.46 | B2R059-01, B2R083-01 |
| 777.25 | 9.98 | 9.95 | B2R071-01 |
| 781.216 | 9.987 | 9.95 | B2R067-01 |
| 793.25 | 10.023 | 9.95 | B2R051-01 |
| 835.281 | 9.96 | 9.95 | B2R059-01, B2R083-01 |
| 804.25 | 11.29 | 11.25 | B2R026-01, B2R050-01 |
| 806.24 | 11.283 | 11.25 | B2R004-01, B2R054-01, B2R076-01 |
| 810.215 | 11.307 | 11.25 | B2R006-01 |
| 790.27 | 8.323 | 8.28 | B2R048-01, B2R098-01 |
| 791.266 | 8.32 | 8.28 | B2R073-01 |
| 818.265 | 11.473 | 11.43 | B2R034-01 |
| 845.301 | 11.48 | 11.43 | B2R037-01, B2R087-01 |
| 845.301 | 11.463 | 11.43 | B2R037-01, B2R087-01 |
| 846.297 | 11.457 | 11.43 | B2R038-01, B2R088-01 |
| 797.186 | 8.407 | 8.4 | B2R001-01 |
| 808.224 | 8.453 | 8.4 | B2R100-01, B2R028-01, B2R080-01 |
| 778.245 | 9.05 | 8.98 | B2R072-01 |
| 820.256 | 9.02 | 8.98 | B2R012-01, B2R056-01 |
| 822.271 | 8.997 | 8.98 | B2R082-01 |
| 789.275 | 9.907 | 9.87 | B2R047-01, B2R097-01 |
| 789.275 | 9.933 | 9.87 | B2R047-01, B2R097-01 |
| 792.261 | 9.91 | 9.87 | B2R074-01 |
| 836.276 | 9.94 | 9.87 | B2R060-01, B2R084-01 |

(continued)

| Mixture No. | mixAI-B2R-01 | | |
|---|---|---|---|
| mlz | Retention time of MS peak (min) | Retention time of UV peak (min) | No. of assigned compound |
| 860.212 | 9.903 | 9.87 | B2R018-01, B2R064-01 |
| 806.24 | 10.983 | 10.94 | B2R004-01, B2R054-01, B2R076-01 |
| 807.229 | 10.987 | 10.94 | B2R027-01, B2R079-01, B2R099-01 |
| 796.19 | 11.21 | 11.16 | B2R024-01, B2R078-01 |
| 796.19 | 11.193 | 11.16 | B2R024-01, B2R078-01 |
| 820.256 | 11.197 | 11.16 | B2R012-01, B2R056-01 |
| 826.27 | 9.387 | 9.35 | B2R066-01 |
| 836.276 | 9.393 | 9.35 | B2R060-01, B2R084-01 |
| 833.301 | 10.393 | 10.35 | B2R057-01 |
| 834.297 | 10.393 | 10.35 | B2R058-01 |
| 794.245 | 8.89 | 8.84 | B2R052-01 |
| 795.195 | 8.867 | 8.84 | B2R023-01, B2R077-01 |

Retention times at the UV and MS peaks to which each compound was assigned, and the values of m/z $(M+H)^+$ detected in the time zones are shown in Table AI-03-02. From the possibility that two compounds were lacked in Example 4-2-2 and two compounds were lacked in Example 4-2-3, there is a possibility that 48 compounds induced from these four compounds were also lacked. Hence, as shown in Table AI-03-02, m/z $(M+H)^+$ peaks that corresponded to 1152 compounds among 1200 compounds set as library compounds were able to be confirmed.

The results described above revealed highly robust methods as to reaction conditions for use in diverse bond formations, washing methods, and capping methods for ensuring a purity, and demonstrated that library synthesis can be carried out with a high probability of success by carrying out the step of identifying the scope of application of applicable building blocks.

The results demonstrated that a library can be synthesized in which the "diversity of building blocks" and "diversity imparted to linkers that link the building blocks" are multiplied.

Example 5; Evaluation of binding of synthesized mixture library compounds (mixture library confirmed to contain 1152 compounds, prepared in Example 4) to Bcl-2 using AS-MS

DMSO solutions (prepared into approximately 0.58 mM based on calculation from the average molecular weight of each mixture) of 12 types of mixtures that could each contain 100 compounds, i.e., mixAI-B4J-01, mixAI-BlM-01, mixAI-BlK-01, mixAI-BlO- 01, mixAI-BlA-01, mixAI-B3A-01, mixAI-B2I-01, mixAI-B2L-01, mixAI-B2N-01, mixAI- B2A-01, mixAI-B2Q-01, and mixAI-B2R-01, shown in Table AI-03-B4J-01, Table AI-03- B1M-01, Table AI-03-B1K-01, Table AI-03-B1O-01, Table AI-03-B1A-01, Table AI-03- B3A-01, Table AI-03-B2I-01, Table AI-03-B2L-01, Table AI-03-B2N-01, Table AI-03- B2A-01, Table AI-03-B2Q-01, and Table AI-03-B2R-01 described in Example 4, were respectively dispensed to a total of 12 wells of MICROPLATE, 96-well, PP, V-BOTTOM (Greiner Bio-One International GmbH, 651201). 70 nL each from the 12 wells was collected into any one well using LABCYTE ECHO(R) 555 acoustic dispensing system (Beckman Coulter, Inc.) to prepare a sample solution in a mixed state of 1200 compounds (the presence of 1152 compounds was already identified when the mixtures were prepared in Example 4). Further, Bcl-2 protein (Novus Biologicals, NBP2-34889, Recombinant Human Bcl-2 Protein, Lot E60145051) (19.2 μL) prepared at 20.9 μM in advance using a Tris-HCl buffer (25 mM Tris-HCl, pH 7.4, 150 mM NaCl, 0.025% Tween-20, and 1 mM DTT) was added to the well containing the dispensed 1200 compounds (the presence of 1152 compounds was already identified when the mixtures were prepared in Example 4) (final compound concentration: 20 nM, final Bcl-2 concentration: 20 μM), and the plate was incubated at 23°C for 1 hour in the dark. Subsequently, 200 μL of an assay buffer (25 mM Tris-HCl, pH 7.4, 150 mM NaCl, 0.025% Tween-20, and 1 mM DTT) was centrifuged three times at 2000 × G at 4°C for 2 minutes using 96-well MacroSpin column, P-2 Material (Harvard Bioscience, Inc., 74-5655) that had been allowed to swell to condition

the 96-well MacroSpin column. Subsequently, 20 μL of the sample thus incubated was added to the 96-well MacroSpin column, P-2 Material after the completion of conditioning, and SEC treatment was performed by centrifugation at 2000 × G for 2 minutes. 32 μL of a 1:1 mixed solution of acetonitrile and methanol (LC/MS grade, FUJIFILM Wako Pure Chemical Corp.) was added to a flow- through liquid from SEC, and the mixture was centrifuged at 1800 × G at 4°C for 5 minutes. The supernatant was used as analysis sample ASMS (A). By a similar operation, a sample prepared at 1200 compounds/well and supplemented with only a Tris-HCl buffer containing no Bcl-2 protein was incubated under the same conditions as above, and the same organic solvent as above was added thereto without 96-well MacroSpin column treatment. A supernatant obtained by a centrifugation operation was used as STD (B). Next, by a similar operation, a sample prepared at 1200 compounds/well and supplemented with only a Tris- HCl buffer containing no Bcl-2 protein was incubated under the same conditions as above and treated with 96-well MacroSpin column in the same manner as above, and the same organic solvent as above was added thereto. A supernatant obtained by a centrifugation operation was used as BK (C). These samples were measured by reverse-phase LC/MS.

Then, the samples A, B and C were analyzed for the 1200 compounds using TraceFinder 5.1 (Thermo Fisher Scientific Inc.) and CHANCE Software (in-house), and an ASMS ratio (%) was calculated according to the following expression.

$$\text{Expression: ASMS ratio (\%)} = (A - C) / B * 100$$

[Table 276]

| Reverse-phase LC/MS conditions | |
|---|---|
| Vanquish UHPLC (Thermo Fisher Scientific) | |
| Oven (°C) | 40 |
| Column | Column packed with chemically bonded porous spherical silica gel surface-modified with an ODS octadecylsilyl group as a stationary phase |
| Auto Sampler (°C) | 4 |
| Orbitrap Fusion Lumos Tribrid mass spectrometer (Thermo Fisher Scientific) | |
| Polarity | Positive mode |
| Data Type | profile |

The results are shown in Table AJ-01. Table AJ-01 shows m/z of observed MS peaks and the number of a compound that can be assigned to each MS peak. Although m/z of the MS peaks was confirmed down to four decimal places, 0 at the decimal level may be omitted in the description in Table AJ-01. For example, "587.0000" is also referred to as "587", "587.1000" is also referred to as "587.1", "587.1200" is also referred to as "587.12", and "587.1230" is also referred to as "587.123".

[Table 277]

[Table AJ-01]

| Compound No. | Molecular formula | m/z (expected) | ASMS ratio(%) | |
|---|---|---|---|---|
| | | | n=1 | n=2 |
| B1A001-01 | C33H39N5O3S | 586.2846 | 17.6 | -0.7 |
| B1A002-01 | C32H38N6O3S | 587.2799 | 0.0 | 4.9 |
| B1A003-01 | C36H43N5O3 | 594.3439 | 7.5 | 5.8 |
| B1A004-01 | C35H42N6O3 | 595.3391 | -6.2 | 2.6 |
| B1A005-01 | C35H40FN5O3 | 598.3188 | 7.0 | -10.7 |
| B1A006-01 | C34H39FN6O3 | 599.314 | 2.4 | 9.1 |
| B1A007-01 | C37H41N5O3 | 604.3282 | 0.0 | na |
| B1A008-01 | C36H40N6O3 | 605.3235 | 3.0 | 0.0 |
| B1A009-01 | C36H40N6O3 | 605.3235 | 3.0 | 0.0 |
| B1A010-01 | C35H39N7O3 | 606.3187 | na | 0.0 |
| B1A011-01 | C37H45N5O3 | 608.3595 | 11.2 | -13.2 |
| B1A012-01 | C36H44N6O3 | 609.3548 | 0.0 | 0.0 |
| B1A013-01 | C39H43N5O3 | 630.3439 | -1.7 | 2.4 |
| B1A014-01 | C38H42N6O3 | 631.3391 | 8.1 | 0.0 |
| B1A015-01 | C39H49N5O3 | 636.3908 | -0.9 | -20.6 |
| B1A016-01 | C38H48N6O3 | 637.3861 | 12.1 | -4. |
| B1A017-01 | C36H40F3N5O3 | 648.3156 | 5.4 | 5.4 |
| B1A018-01 | C35H39F3N6O3 | 649.3109 | -6.5 | 9.9 |
| B1A019-01 | C38H40F3N5O3 | 672.3156 | -3.9 | -7.5 |
| B1A020-01 | C37H39F3N6O3 | 673.3109 | -4.3 | -10.5 |
| B1A021-01 | C39H42F3N7O3 | 714.3374 | -1.3 | -3.7 |
| B1A022-01 | C38H41F3N8O3 | 715.3327 | 8.0 | 7.7 |
| B1A023-01 | C35H41N3O3S | 584.2941 | 4.0 | 0.8 |
| B1A024-01 | C34H40N4O3S | 585.2894 | 4.1 | 1.2 |
| B1A025-01 | C38H45N3O3 | 592.3534 | 7.4 | -16.1 |
| B1A026-01 | C37H44N4O3 | 593.3486 | 3.0 | -2.9 |
| B1A027-01 | C37H42FN3O3 | 596.3283 | 6.7 | -7.8 |
| B1A028-01 | C36H41FN4O3 | 597.3236 | -1.6 | -4.7 |
| B1A029-01 | C39H43N3O3 | 602.3377 | -22.3 | -13.0 |
| B1A030-01 | C38H42N4O3 | 603.333 | 6.9 | -1.8 |
| B1A031-01 | C38H42N4O3 | 603.333 | 6.9 | -1.8 |
| B1A032-01 | C37H41N5O3 | 604.3282 | 0.0 | -9.1 |
| B1A033-01 | C39H47N3O3 | 606.369 | 3.3 | -13.3 |
| B1A034-01 | C38H46N4O3 | 607.3643 | 5.2 | -3.2 |
| B1A035-01 | C41H45N3O3 | 628.3534 | 14.9 | -7.0 |
| B1A036-01 | C40H44N4O3 | 629.3486 | 0.4 | 3.1 |
| B1A037-01 | C41H51N3O3 | 634.4003 | 3.3 | -5.0 |
| B1A038-01 | C40H50N4O3 | 635.3956 | 6.5 | -6.4 |
| B1A039-01 | C38H42F3N3O3 | 646.3251 | 6.3 | -8.4 |
| B1A040-01 | C37H41F3N4O3 | 647.3204 | 10.4 | 8.4 |
| B1A041-01 | C40H42F3N3O3 | 670.3251 | -2.4 | -4.0 |
| B1A042-01 | C39H41F3N4O3 | 671.3204 | 12.5 | -8.5 |
| B1A043-01 | C41H44F3N5O3 | 712.3469 | 3.8 | -2.9 |
| B1A044-01 | C40H43F3N6O3 | 713.3422 | 2.6 | -1.9 |
| B1A045-01 | C37H45N3O2 | 564.3585 | 1.1 | -2.2 |
| B1A046-01 | C36H44N4O2 | 565.3537 | 0.0 | 0.0 |
| B1A047-01 | C38H47N3O2 | 578.3741 | 3.2 | 2.5 |
| B1A048-01 | C37H46N4O2 | 579.3694 | 1.5 | 3.6 |
| B1A049-01 | C39H49N3O2 | 592.3898 | 4.1 | -9.1 |
| B1A050-01 | C38H48N4O2 | 593.385 | 0.6 | 3.0 |
| B1A051-01 | C37H44FN3O2 | 582.349 | -5.4 | -4.2 |
| B1A052-01 | C36H43FN4O2 | 583.3443 | 4.2 | -6.2 |
| B1A053-01 | C38H47N3O3 | 594.369 | 13.2 | 4.8 |
| B1A054-01 | C37H46N4O3 | 595.3643 | 0.0 | 0.0 |
| B1A055-01 | C39H49N3O3 | 608.3847 | -0.1 | -14.4 |
| B1A056-01 | C38H48N4O3 | 609.3799 | 9.7 | 4.0 |
| B1A057-01 | C40H51N3O3 | 622.4003 | 0.0 | 0.0 |
| B1A058-01 | C39H50N4O3 | 623.3956 | na | 0.0 |
| B1A059-01 | C39H49N3O4 | 624.3796 | 7.7 | -5.5 |
| B1A060-01 | C38H48N4O4 | 625.3748 | 0.0 | na |
| B1A061-01 | C38H44F3N3O2 | 632.3458 | 5.3 | 1.1 |
| B1A062-01 | C37H43F3N4O2 | 633.3411 | -0.3 | -9.7 |
| B1A063-01 | C38H44F3N3O3 | 648.3408 | -1.0 | -11.0 |
| B1A064-01 | C37H43F3N4O3 | 649.336 | 5.2 | -1.6 |
| B1A065-01 | C41H47N3O2 | 614.3741 | -32.6 | -10.4 |
| B1A066-01 | C40H46N4O2 | 615.3694 | 8.2 | 7.7 |
| B1A067-01 | C35H43N3O2S | 570.3149 | 3.6 | -7.8 |
| B1A068-01 | C34H42N4O2S | 571.3101 | 12.2 | 3.3 |
| B1A069-01 | C39H45N3O2 | 588.3585 | 8.3 | 0.8 |
| B1A070-01 | C38H44N4O2 | 589.3537 | 0.0 | 0.0 |
| B1A071-01 | C35H43N5O2 | 566.349 | -0.3 | -3.3 |
| B1A072-01 | C34H42N6O2 | 567.3442 | 3.9 | -0.2 |
| B1A073-01 | C36H45N5O2 | 580.3646 | 3.2 | -4.7 |
| B1A074-01 | C35H44N6O2 | 581.3599 | 3.2 | -0.1 |
| B1A075-01 | C37H47N5O2 | 594.3803 | 31.3 | 28.6 |
| B1A076-01 | C36H46N6O2 | 595.3755 | na | na |
| B1A077-01 | C35H42FN5O2 | 584.3395 | 6.6 | -1.3 |
| B1A078-01 | C34H41FN6O2 | 585.3348 | 4.7 | 1.2 |
| B1A079-01 | C36H45N5O3 | 596.3595 | 3.9 | -3.9 |
| B1A080-01 | C35H44N6O3 | 597.3548 | 3.4 | 0.0 |
| B1A081-01 | C37H47N5O3 | 610.3752 | -10.9 | -5. |
| B1A082-01 | C36H46N6O3 | 611.3704 | 3.0 | 0.2 |
| B1A083-01 | C38H49N5O3 | 624.3908 | -0.3 | 0.5 |
| B1A084-01 | C37H48N6O3 | 625.3861 | 2.1 | -2.7 |

| Compound No. | Molecular formula | m/z (expected) | ASMS ratio(%) | |
|---|---|---|---|---|
| | | | n=1 | n=2 |
| B1A085-01 | C37H47N5O4 | 626.3701 | 10.6 | 10.8 |
| B1A086-01 | C36H46N6O4 | 627.3653 | 0.0 | -14.8 |
| B1A087-01 | C36H42F3N5O2 | 634.3363 | na | na |
| B1A088-01 | C35H41F3N6O2 | 635.3316 | 18.7 | -7.1 |
| B1A089-01 | C36H42F3N5O3 | 650.3313 | 0.0 | na |
| B1A090-01 | C35H41F3N6O3 | 651.3265 | 3.3 | -1.0 |
| B1A091-01 | C39H45N5O2 | 616.3646 | 0.0 | 15.4 |
| B1A092-01 | C38H44N6O2 | 617.3599 | 2.6 | -2.4 |
| B1A093-01 | C33H41N5O2S | 572.3054 | -5.3 | 0.0 |
| B1A094-01 | C32H40N6O2S | 573.3006 | 6.2 | -0.7 |
| B1A095-01 | C37H43N5O2 | 590.349 | 0.3 | -17.1 |
| B1A096-01 | C36H42N6O2 | 591.3442 | 0.0 | 7.4 |
| B1A097-01 | C38H47N3O2 | 578.3741 | 3.2 | 2.5 |
| B1A098-01 | C37H46N4O2 | 579.3694 | na | na |
| B1A099-01 | C38H46FN3O2 | 596.3647 | 0.9 | -0.9 |
| B1A100-01 | C37H45FN4O2 | 597.3599 | 6.4 | 2.3 |
| B1K001-01 | C24H24F3N5O3S | 520.1625 | 0.0 | 0.0 |
| B1K002-01 | C23H23F3N6O3S | 521.1577 | 0.0 | 0.0 |
| B1K003-01 | C27H28F3N5O3 | 528.2217 | 0.1 | -0.2 |
| B1K004-01 | C26H27F3N6O3 | 529.217 | 0.0 | 0.6 |
| B1K005-01 | C26H25F4N5O3 | 532.1966 | -0.1 | 0.6 |
| B1K006-01 | C25H24F4N6O3 | 533.1919 | -0.6 | 0.0 |
| B1K007-01 | C28H26F3N5O3 | 538.2061 | 0.0 | 1.3 |
| B1K008-01 | C27H25F3N6O3 | 539.2013 | 0.0 | 0.0 |
| B1K009-01 | C27H25F3N6O3 | 539.2013 | na | na |
| B1K010-01 | C26H24F3N7O3 | 540.1966 | 0.0 | 0.0 |
| B1K011-01 | C28H30F3N5O3 | 542.2374 | -0.1 | 0.3 |
| B1K012-01 | C27H29F3N6O3 | 543.2326 | 0.0 | -0.4 |
| B1K013-01 | C30H28F3N5O3 | 564.2217 | 0.0 | 1.4 |
| B1K014-01 | C29H27F3N6O3 | 565.217 | -0.3 | 0.0 |
| B1K015-01 | C30H34F3N5O3 | 570.2687 | 2.9 | -1.8 |
| B1K016-01 | C29H33F3N6O3 | 571.2639 | 0.0 | 0.0 |
| B1K017-01 | C27H25F6N5O3 | 582.1934 | 0.0 | 0.0 |
| B1K018-01 | C26H24F6N6O3 | 583.1887 | -0.6 | 0.5 |
| B1K019-01 | C29H25F6N5O3 | 606.1934 | 0.0 | 0.0 |
| B1K020-01 | C28H24F6N6O3 | 607.1887 | 2.9 | -3.8 |
| B1K021-01 | C30H27F6N7O3 | 648.2152 | -0.5 | -1.9 |
| B1K022-01 | C29H26F6N8O3 | 649.2105 | 1.4 | 0.1 |
| B1K023-01 | C26H26F3N3O3S | 518.172 | 4.3 | 1.3 |
| B1K024-01 | C25H25F3N4O3S | 519.1672 | 0.0 | 0.0 |
| B1K025-01 | C29H30F3N3O3 | 526.2312 | 0.3 | -0.8 |
| B1K026-01 | C28H29F3N4O3 | 527.2265 | 4.2 | 0.2 |
| B1K027-01 | C28H27F4N3O3 | 530.2061 | -0.8 | -3.6 |
| B1K028-01 | C27H26F4N4O3 | 531.2014 | 0.0 | 0.0 |
| B1K029-01 | C30H28F3N3O3 | 536.2156 | na | na |
| B1K030-01 | C29H27F3N4O3 | 537.2108 | 0.0 | 0.9 |
| B1K031-01 | C29H27F3N4O3 | 537.2108 | 0.0 | 0.0 |
| B1K032-01 | C28H26F3N5O3 | 538.2061 | na | na |
| B1K033-01 | C30H32F3N3O3 | 540.2469 | 2.3 | -0.6 |
| B1K034-01 | C29H31F3N4O3 | 541.2421 | -0.3 | 0.0 |
| B1K035-01 | C32H30F3N3O3 | 562.2312 | 4.8 | 4.5 |
| B1K036-01 | C31H29F3N4O3 | 563.2265 | 1.1 | -0.4 |
| B1K037-01 | C32H36F3N3O3 | 568.2782 | 0.8 | 1.9 |
| B1K038-01 | C31H35F3N4O3 | 569.2734 | 0.6 | -1.8 |
| B1K039-01 | C29H27F6N3O3 | 580.2029 | 3.5 | 3.9 |
| B1K040-01 | C28H26F6N4O3 | 581.1982 | 0.0 | 0.0 |

na = not available

[Table 278]

| Compound No. | Molecular formula | m/z (expected) | ASMS ratio(%) | |
|---|---|---|---|---|
| | | | n=1 | n=2 |
| B1K041-01 | C31H27F6N3O3 | 604.2029 | 0.0 | 0.0 |
| B1K042-01 | C30H26F6N4O3 | 605.1982 | 5.0 | -0.1 |
| B1K043-01 | C32H29F6N5O3 | 646.2247 | 3.8 | 1.9 |
| B1K044-01 | C31H28F6N6O3 | 647.22 | 2.6 | 1.0 |
| B1K045-01 | C28H30F3N3O2 | 498.2363 | 1.5 | -0.1 |
| B1K046-01 | C27H29F3N4O2 | 499.2315 | -0.1 | 0.5 |
| B1K047-01 | C29H32F3N3O2 | 512.2519 | 3.3 | 0.5 |
| B1K048-01 | C28H31F3N4O2 | 513.2472 | 0.1 | 1.2 |
| B1K049-01 | C30H34F3N3O2 | 526.2676 | -3.3 | 1.5 |
| B1K050-01 | C29H33F3N4O2 | 527.2628 | 0.0 | 0.0 |
| B1K051-01 | C28H29F4N3O2 | 516.2269 | 2.8 | 2.0 |
| B1K052-01 | C27H28F4N4O2 | 517.2221 | -3.3 | 0.5 |
| B1K053-01 | C29H32F3N3O3 | 528.2469 | -0.5 | -3.3 |
| B1K054-01 | C28H31F3N4O3 | 529.2421 | 0.0 | 0.0 |
| B1K055-01 | C30H34F3N3O3 | 542.2625 | -0.8 | 0.0 |
| B1K056-01 | C29H33F3N4O3 | 543.2578 | 3.3 | 0.2 |
| B1K057-01 | C31H36F3N3O3 | 556.2782 | 1.0 | -2.6 |
| B1K058-01 | C30H35F3N4O3 | 557.2734 | 1.3 | 0.1 |
| B1K059-01 | C30H34F3N3O4 | 558.2574 | 3.0 | -1.9 |
| B1K060-01 | C29H33F3N4O4 | 559.2527 | 0.0 | 0.0 |
| B1K061-01 | C29H29F6N3O2 | 566.2237 | 2.8 | -1.3 |
| B1K062-01 | C28H28F6N4O2 | 567.2189 | 1.2 | -0.3 |
| B1K063-01 | C29H29F6N3O3 | 582.2186 | 3.9 | 0.4 |
| B1K064-01 | C28H28F6N4O3 | 583.2138 | 1.4 | 0.7 |
| B1K065-01 | C32H32F3N3O2 | 548.2519 | 5.9 | -5.6 |
| B1K066-01 | C31H31F3N4O2 | 549.2472 | 1.8 | -0.7 |
| B1K067-01 | C26H28F3N3O2S | 504.1927 | 5.1 | 1.8 |
| B1K068-01 | C25H27F3N4O2S | 505.188 | 2.6 | -1.4 |
| B1K069-01 | C30H30F3N3O2 | 522.2363 | 3.5 | -1.4 |
| B1K070-01 | C29H29F3N4O2 | 523.2315 | 0.0 | 0.0 |
| B1K071-01 | C26H28F3N5O2 | 500.2268 | 1.6 | -0.9 |
| B1K072-01 | C25H27F3N6O2 | 501.222 | -0.6 | 1.7 |
| B1K073-01 | C27H30F3N5O2 | 514.2424 | 3.1 | -1.9 |
| B1K074-01 | C26H29F3N6O2 | 515.2377 | 0.0 | 0.0 |
| B1K075-01 | C28H32F3N5O2 | 528.2581 | 1.4 | -3.8 |
| B1K076-01 | C27H31F3N6O2 | 529.2533 | na | 0.0 |
| B1K077-01 | C26H27F4N5O2 | 518.2174 | 2.4 | -0.2 |
| B1K078-01 | C25H26F4N6O2 | 519.2126 | 0.8 | -0.2 |
| B1K079-01 | C27H30F3N5O3 | 530.2374 | 2.2 | -1.3 |
| B1K080-01 | C26H29F3N6O3 | 531.2326 | 0.0 | 0.0 |
| B1K081-01 | C28H32F3N5O3 | 544.253 | 2.3 | -0.4 |
| B1K082-01 | C27H31F3N6O3 | 545.2483 | 0.0 | 0.0 |
| B1K083-01 | C29H34F3N5O3 | 558.2687 | 0.0 | 0.0 |
| B1K084-01 | C28H33F3N6O3 | 559.2639 | 0.0 | 0.0 |
| B1K085-01 | C28H32F3N5O4 | 560.2479 | 4.2 | -0.2 |
| B1K086-01 | C27H31F3N6O4 | 561.2432 | na | 0.0 |
| B1K087-01 | C27H27F6N5O2 | 568.2142 | 1.8 | -0.1 |
| B1K088-01 | C26H26F6N6O2 | 569.2094 | 2.2 | -0.2 |
| B1K089-01 | C27H27F6N5O3 | 584.2091 | 2.0 | 1.0 |
| B1K090-01 | C26H26F6N6O3 | 585.2043 | -0.6 | -1.9 |

| Compound No. | Molecular formula | m/z (expected) | ASMS ratio(%) | |
|---|---|---|---|---|
| | | | n=1 | n=2 |
| B1K091-01 | C30H30F3N5O2 | 550.2424 | 3.6 | -4.2 |
| B1K092-01 | C29H29F3N6O2 | 551.2377 | 4.1 | -0.7 |
| B1K093-01 | C24H26F3N5O2S | 506.1832 | 2.7 | -0.4 |
| B1K094-01 | C23H25F3N6O2S | 507.1785 | 1.4 | -0.1 |
| B1K095-01 | C28H28F3N5O2 | 524.2268 | 3.5 | -0.6 |
| B1K096-01 | C27H27F3N6O2 | 525.222 | 0.0 | 0.0 |
| B1K097-01 | C29H32F3N3O2 | 512.2519 | -2.1 | -3.1 |
| B1K098-01 | C28H31F3N4O2 | 513.2472 | -1.1 | -2.4 |
| B1K099-01 | C29H31F4N3O2 | 530.2425 | 0.0 | 0.0 |
| B1K100-01 | C28H30F4N4O2 | 531.2378 | 0.0 | 0.0 |
| B1M001-01 | C25H29N5O3S | 480.2064 | 0.0 | 0.0 |
| B1M002-01 | C24H28N6O3S | 481.2016 | -0.7 | -1.5 |
| B1M003-01 | C28H33N5O3 | 488.2656 | -1.9 | 2.0 |
| B1M004-01 | C27H32N6O3 | 489.2609 | 0.0 | 0.0 |
| B1M005-01 | C27H30FN5O3 | 492.2405 | 0.0 | 1.4 |
| B1M006-01 | C26H29FN6O3 | 493.2358 | 0.0 | 0.0 |
| B1M007-01 | C29H31N5O3 | 498.25 | 0.3 | 0.3 |
| B1M008-01 | C28H30N6O3 | 499.2452 | na | 0.0 |
| B1M009-01 | C28H30N6O3 | 499.2452 | na | 0.0 |
| B1M010-01 | C27H29N7O3 | 500.2405 | 0.0 | 0.0 |
| B1M011-01 | C29H35N5O3 | 502.2813 | 18.6 | 7.5 |
| B1M012-01 | C28H34N6O3 | 503.2765 | -3.5 | -2.3 |
| B1M013-01 | C31H33N5O3 | 524.2656 | 2.9 | -1.1 |
| B1M014-01 | C30H32N6O3 | 525.2609 | 4.1 | 0.4 |
| B1M015-01 | C31H39N5O3 | 530.3126 | -2.4 | -3.0 |
| B1M016-01 | C30H38N6O3 | 531.3078 | 0.0 | 0.0 |
| B1M017-01 | C28H30F3N5O3 | 542.2374 | -0.1 | 0.3 |
| B1M018-01 | C27H29F3N6O3 | 543.2326 | 0.0 | 0.0 |
| B1M019-01 | C30H30F3N5O3 | 566.2374 | 1.5 | 0.0 |
| B1M020-01 | C29H29F3N6O3 | 567.2326 | 0.0 | 0.0 |
| B1M021-01 | C31H32F3N7O3 | 608.2592 | 0.0 | 0.0 |
| B1M022-01 | C30H31F3N8O3 | 609.2544 | -13.6 | 6.7 |
| B1M023-01 | C27H31N3O3S | 478.2159 | 1.4 | -3.0 |
| B1M024-01 | C26H30N4O3S | 479.2111 | 0.0 | 0.0 |
| B1M025-01 | C30H35N3O3 | 486.2751 | 3.6 | -1.2 |
| B1M026-01 | C29H34N4O3 | 487.2704 | 0.9 | 0.9 |
| B1M027-01 | C29H32FN3O3 | 490.2501 | 1.0 | 0.4 |
| B1M028-01 | C28H31FN4O3 | 491.2453 | 0.0 | 0.0 |
| B1M029-01 | C31H33N3O3 | 496.2595 | 3.0 | -0.6 |
| B1M030-01 | C30H32N4O3 | 497.2547 | 0.0 | -5.9 |
| B1M031-01 | C30H32N4O3 | 497.2547 | 0.0 | -2.4 |
| B1M032-01 | C29H31N5O3 | 498.25 | 0.3 | 0.3 |
| B1M033-01 | C31H37N3O3 | 500.2908 | 0.3 | 0.0 |
| B1M034-01 | C30H36N4O3 | 501.286 | -0.3 | -0.2 |
| B1M035-01 | C33H35N3O3 | 522.2751 | 2.9 | -1.7 |
| B1M036-01 | C32H34N4O3 | 523.2704 | 6.7 | -1.2 |
| B1M037-01 | C33H41N3O3 | 528.3221 | -1.5 | 2.3 |

| Compound No. | Molecular formula | m/z (expected) | ASMS ratio(%) | |
|---|---|---|---|---|
| | | | n=1 | n=2 |
| B1M038-01 | C32H40N4O3 | 529.3173 | 0.0 | 1.7 |
| B1M039-01 | C30H32F3N3O3 | 540.2469 | 2.3 | -0.6 |
| B1M040-01 | C29H31F3N4O3 | 541.2421 | -0.3 | 0.0 |
| B1M041-01 | C32H32F3N3O3 | 564.2469 | 0.7 | -3.5 |
| B1M042-01 | C31H31F3N4O3 | 565.2421 | 0.0 | 0.0 |
| B1M043-01 | C33H34F3N5O3 | 606.2687 | 2.2 | -3.1 |
| B1M044-01 | C32H33F3N6O3 | 607.2639 | 1.0 | 1.1 |
| B1M045-01 | C29H35N3O2 | 458.2802 | 2.5 | -2.3 |
| B1M046-01 | C28H34N4O2 | 459.2755 | 0.7 | -1.2 |
| B1M047-01 | C30H37N3O2 | 472.2959 | 1.1 | 0.3 |
| B1M048-01 | C29H36N4O2 | 473.2911 | 0.9 | 0.3 |
| B1M049-01 | C31H39N3O2 | 486.3115 | -0.9 | -0.4 |
| B1M050-01 | C30H38N4O2 | 487.3068 | -2.0 | -0.2 |
| B1M051-01 | C29H34FN3O2 | 476.2708 | 0.7 | 1.9 |
| B1M052-01 | C28H33FN4O2 | 477.266 | 27.6 | 20.2 |
| B1M053-01 | C30H37N3O3 | 488.2908 | -3.0 | -3.1 |
| B1M054-01 | C29H36N4O3 | 489.286 | 0.9 | 0.0 |
| B1M055-01 | C31H39N3O3 | 502.3064 | 3.9 | -1.2 |
| B1M056-01 | C30H38N4O3 | 503.3017 | 0.5 | 0.0 |
| B1M057-01 | C32H41N3O3 | 516.3221 | -0.7 | 1.4 |
| B1M058-01 | C31H40N4O3 | 517.3173 | -1.4 | -1.5 |
| B1M059-01 | C31H39N3O4 | 518.3013 | -5.5 | -7.2 |
| B1M060-01 | C30H38N4O4 | 519.2966 | 0.0 | 0.0 |
| B1M061-01 | C30H34F3N3O2 | 526.2676 | -3.3 | -13.1 |
| B1M062-01 | C29H33F3N4O2 | 527.2628 | 1.6 | -1.7 |
| B1M063-01 | C30H34F3N3O3 | 542.2625 | 0.4 | -1.1 |
| B1M064-01 | C29H33F3N4O3 | 543.2578 | 5.4 | -4.3 |
| B1M065-01 | C33H37N3O2 | 508.2959 | 0.2 | -6.4 |
| B1M066-01 | C32H36N4O2 | 509.2911 | 0.0 | 4.4 |
| B1M067-01 | C27H33N3O2S | 464.2366 | -1.6 | -87.4 |
| B1M068-01 | C26H32N4O2S | 465.2319 | 1.1 | 0.0 |
| B1M069-01 | C31H35N3O2 | 482.2802 | -0.2 | -4.4 |
| B1M070-01 | C30H34N4O2 | 483.2755 | 0.0 | 0.0 |
| B1M071-01 | C27H33N5O2 | 460.2707 | 34.0 | 24.4 |
| B1M072-01 | C26H32N6O2 | 461.266 | 0.0 | 1.9 |
| B1M073-01 | C28H35N5O2 | 474.2864 | 0.4 | -0.5 |
| B1M074-01 | C27H34N6O2 | 475.2816 | -1.6 | 0.9 |
| B1M075-01 | C29H37N5O2 | 488.302 | 77.3 | 46.1 |
| B1M076-01 | C28H36N6O2 | 489.2973 | 0.5 | 1.5 |
| B1M077-01 | C27H32FN5O2 | 478.2613 | -0.7 | 3.1 |
| B1M078-01 | C26H31FN6O2 | 479.2565 | 0.0 | 0.0 |
| B1M079-01 | C28H35N5O3 | 490.2813 | 8.1 | 0.0 |
| B1M080-01 | C27H34N6O3 | 491.2765 | 0.0 | 0.0 |

na = not available

[Table 279]

| Compound No. | Molecular formula | m/z (expected) | ASMS ratio(%) | |
|---|---|---|---|---|
| | | | n=1 | n=2 |
| B1M081-01 | C29H37N5O3 | 504.2969 | 0.0 | 0.0 |
| B1M082-01 | C28H36N6O3 | 505.2922 | 0.0 | 0.0 |
| B1M083-01 | C30H39N5O3 | 518.3126 | 0.0 | -0.3 |
| B1M084-01 | C29H38N6O3 | 519.3078 | 0.0 | 0.0 |
| B1M085-01 | C29H37N5O4 | 520.2918 | 0.0 | 0.0 |
| B1M086-01 | C28H36N6O4 | 521.2871 | 0.0 | 0.0 |
| B1M087-01 | C28H32F3N5O2 | 528.2581 | 3.8 | 3.2 |
| B1M088-01 | C27H31F3N6O2 | 529.2533 | 0.0 | 0.0 |
| B1M089-01 | C28H32F3N5O3 | 544.253 | 3.9 | 0.2 |
| B1M090-01 | C27H31F3N6O3 | 545.2483 | 0.0 | 0.0 |
| B1M091-01 | C31H35N5O2 | 510.2864 | 0.0 | 0.0 |
| B1M092-01 | C30H34N6O2 | 511.2816 | na | 0.0 |

| Compound No. | Molecular formula | m/z (expected) | ASMS ratio(%) | |
|---|---|---|---|---|
| | | | n=1 | n=2 |
| B1M093-01 | C25H31N5O2S | 466.2271 | 0.0 | 0.0 |
| B1M094-01 | C24H30N6O2S | 467.2224 | -1.5 | -2.1 |
| B1M095-01 | C29H33N5O2 | 484.2707 | 0.0 | 0.0 |
| B1M096-01 | C28H32N6O2 | 485.266 | na | 0.0 |
| B1M097-01 | C30H37N3O2 | 472.2959 | 1.1 | 0.3 |
| B1M098-01 | C29H36N4O2 | 473.2911 | 0.9 | 0.3 |
| B1M099-01 | C30H36FN3O2 | 490.2864 | -0.7 | -0.3 |
| B1M100-01 | C29H35FN4O2 | 491.2817 | 0.5 | -1.5 |
| B1O001-01 | C29H29N5O4S | 544.2013 | -0.1 | -3.1 |
| B1O002-01 | C28H28N6O4S | 545.1966 | 2.2 | 2.6 |
| B1O003-01 | C32H33N5O4 | 552.2605 | 0.0 | 0.0 |
| B1O004-01 | C31H32N6O4 | 553.2558 | 2.8 | 0.4 |
| B1O005-01 | C31H30FN5O4 | 556.2355 | 3.6 | -2.5 |
| B1O006-01 | C30H29FN6O4 | 557.2307 | 4.7 | 0.8 |
| B1O007-01 | C33H31N5O4 | 562.2449 | 4.5 | 0.9 |
| B1O008-01 | C32H30N6O4 | 563.2401 | 1.1 | 1.3 |
| B1O009-01 | C32H30N6O4 | 563.2401 | 1.1 | 1.3 |
| B1O010-01 | C31H29N7O4 | 564.2354 | 0.0 | 0.0 |
| B1O011-01 | C33H35N5O4 | 566.2762 | 0.0 | 4.3 |
| B1O012-01 | C32H34N6O4 | 567.2714 | 0.8 | -0.9 |
| B1O013-01 | C35H33N5O4 | 588.2605 | 2.2 | -4.0 |
| B1O014-01 | C34H32N6O4 | 589.2558 | 2.5 | 0.2 |
| B1O015-01 | C35H39N5O4 | 594.3075 | 6.0 | -2.7 |
| B1O016-01 | C34H38N6O4 | 595.3027 | 6.6 | 0.7 |
| B1O017-01 | C32H30F3N5O4 | 606.2323 | 2.5 | 0.0 |
| B1O018-01 | C31H29F3N6O4 | 607.2275 | 1.0 | 3.2 |
| B1O019-01 | C34H30F3N5O4 | 630.2323 | 0.6 | 0.2 |
| B1O020-01 | C33H29F3N6O4 | 631.2275 | 8.6 | -0.5 |
| B1O021-01 | C35H32F3N7O4 | 672.2541 | 2.9 | -1.2 |
| B1O022-01 | C34H31F3N8O4 | 673.2493 | 5.6 | -1.2 |
| B1O023-01 | C31H31N3O4S | 542.2108 | 12.4 | -0.3 |
| B1O024-01 | C30H30N4O4S | 543.2061 | 3.6 | 2.3 |
| B1O025-01 | C34H35N3O4 | 550.27 | -0.4 | -4.5 |
| B1O026-01 | C33H34N4O4 | 551.2653 | 1.7 | -1.5 |
| B1O027-01 | C33H32FN3O4 | 554.245 | 3.8 | 2.1 |
| B1O028-01 | C32H31FN4O4 | 555.2402 | 4.9 | 0.4 |
| B1O029-01 | C35H33N3O4 | 560.2544 | 0.0 | 0.0 |
| B1O030-01 | C34H32N4O4 | 561.2496 | 1.8 | -2.0 |
| B1O031-01 | C34H32N4O4 | 561.2496 | -1.3 | -4.1 |
| B1O032-01 | C33H31N5O4 | 562.2449 | 4.5 | 0.9 |
| B1O033-01 | C35H37N3O4 | 564.2857 | 1.9 | 4.2 |
| B1O034-01 | C34H36N4O4 | 565.2809 | 0.1 | 0.2 |
| B1O035-01 | C37H35N3O4 | 586.27 | 4.3 | -3.7 |
| B1O036-01 | C36H34N4O4 | 587.2653 | 4.8 | 0.9 |
| B1O037-01 | C37H41N3O4 | 592.317 | 4.7 | -1.7 |
| B1O038-01 | C36H40N4O4 | 593.3122 | 2.8 | -0.7 |
| B1O039-01 | C34H32F3N3O4 | 604.2418 | 5.1 | 0.2 |
| B1O040-01 | C33H31F3N4O4 | 605.237 | 1.8 | 0.3 |
| B1O041-01 | C36H32F3N3O4 | 628.2418 | 10.4 | -2.8 |
| B1O042-01 | C35H31F3N4O4 | 629.237 | 2.8 | 2.1 |
| B1O043-01 | C37H34F3N5O4 | 670.2636 | 2.2 | -5.0 |
| B1O044-01 | C36H33F3N6O4 | 671.2588 | 5.8 | 0.4 |
| B1O045-01 | C33H35N3O3 | 522.2751 | 0.0 | 0.0 |
| B1O046-01 | C32H34N4O3 | 523.2704 | 0.6 | 1.3 |
| B1O047-01 | C34H37N3O3 | 536.2908 | 4.1 | 1.5 |
| B1O048-01 | C33H36N4O3 | 537.286 | -0.3 | -1.6 |
| B1O049-01 | C35H39N3O3 | 550.3064 | 6.1 | 0.0 |
| B1O050-01 | C34H38N4O3 | 551.3017 | 10.3 | -5.8 |
| B1O051-01 | C33H34FN3O3 | 540.2657 | 3.9 | -1.9 |
| B1O052-01 | C32H33FN4O3 | 541.261 | 1.6 | 0.4 |

| Compound No. | Molecular formula | m/z (expected) | ASMS ratio(%) | |
|---|---|---|---|---|
| | | | n=1 | n=2 |
| B1O053-01 | C34H37N3O4 | 552.2857 | 2.2 | -1.0 |
| B1O054-01 | C33H36N4O4 | 553.2809 | 2.6 | 3.2 |
| B1O055-01 | C35H39N3O4 | 566.3013 | 3.0 | -4.2 |
| B1O056-01 | C34H38N4O4 | 567.2966 | 6.7 | -0.9 |
| B1O057-01 | C36H41N3O4 | 580.317 | 4.1 | -2.2 |
| B1O058-01 | C35H40N4O4 | 581.3122 | -3.5 | 4.4 |
| B1O059-01 | C35H39N3O5 | 582.2963 | 2.0 | -1.9 |
| B1O060-01 | C34H38N4O5 | 583.2915 | 0.6 | -1.8 |
| B1O061-01 | C34H34F3N3O3 | 590.2625 | 1.9 | -1.5 |
| B1O062-01 | C33H33F3N4O3 | 591.2578 | 1.3 | 4.6 |
| B1O063-01 | C34H34F3N3O4 | 606.2574 | 3.5 | -4.1 |
| B1O064-01 | C33H33F3N4O4 | 607.2527 | 0.7 | -4.0 |
| B1O065-01 | C37H37N3O3 | 572.2908 | 7.5 | 0.8 |
| B1O066-01 | C36H36N4O3 | 573.286 | 12.1 | 2.7 |
| B1O067-01 | C31H33N3O3S | 528.2315 | 0.0 | 0.0 |
| B1O068-01 | C30H32N4O3S | 529.2268 | 1.9 | -1.1 |
| B1O069-01 | C35H35N3O3 | 546.2751 | 6.2 | -2.4 |
| B1O070-01 | C34H34N4O3 | 547.2704 | 8.8 | 2.3 |
| B1O071-01 | C31H33N5O3 | 524.2656 | 2.9 | -1.1 |
| B1O072-01 | C30H32N6O3 | 525.2609 | 4.1 | 0.4 |
| B1O073-01 | C32H35N5O3 | 538.2813 | 2.0 | 3.7 |
| B1O074-01 | C31H34N6O3 | 539.2765 | 3.9 | -0.9 |
| B1O075-01 | C33H37N5O3 | 552.2969 | 9.3 | 0.8 |
| B1O076-01 | C32H36N6O3 | 553.2922 | 4.4 | 1.1 |
| B1O077-01 | C31H32FN5O3 | 542.2562 | 4.1 | 0.4 |
| B1O078-01 | C30H31FN6O3 | 543.2514 | 4.8 | 1.8 |
| B1O079-01 | C32H35N5O4 | 554.2762 | 4.5 | 1.3 |
| B1O080-01 | C31H34N6O4 | 555.2714 | 1.4 | 3.4 |
| B1O081-01 | C33H37N5O4 | 568.2918 | 3.2 | -2.2 |
| B1O082-01 | C32H36N6O4 | 569.2871 | 1.5 | 2.6 |
| B1O083-01 | C34H39N5O4 | 582.3075 | 7.2 | 1.0 |
| B1O084-01 | C33H38N6O4 | 583.3027 | 6.8 | 2.0 |
| B1O085-01 | C33H37N5O5 | 584.2868 | -1.1 | -3.1 |
| B1O086-01 | C32H36N6O5 | 585.282 | na | -0.4 |
| B1O087-01 | C32H32F3N5O3 | 592.253 | 2.2 | -0.5 |
| B1O088-01 | C31H31F3N6O3 | 593.2483 | 3.1 | 1.4 |
| B1O089-01 | C32H32F3N5O4 | 608.2479 | 3.8 | -5.3 |
| B1O090-01 | C31H31F3N6O4 | 609.2432 | 3.3 | -2.0 |
| B1O091-01 | C35H35N5O3 | 574.2813 | 4.5 | -2.2 |
| B1O092-01 | C34H34N6O3 | 575.2765 | 6.3 | -1.4 |
| B1O093-01 | C29H31N5O3S | 530.222 | 0.0 | 0.6 |
| B1O094-01 | C28H30N6O3S | 531.2173 | 2.2 | -0.7 |
| B1O095-01 | C33H33N5O3 | 548.2656 | 2.7 | 0.0 |
| B1O096-01 | C32H32N6O3 | 549.2609 | 0.0 | 3.5 |
| B1O097-01 | C34H37N3O3 | 536.2908 | 4.2 | 2.2 |
| B1O098-01 | C33H36N4O3 | 537.286 | -42.7 | -0.2 |
| B1O099-01 | C34H36FN3O3 | 554.2814 | 4.7 | 1.7 |
| B1O100-01 | C33H35FN4O3 | 555.2766 | -19.7 | 11.1 |
| B3A001-01 | C34H41N5O3S | 600.3003 | 0.0 | -8.4 |
| B3A002-01 | C33H40N6O3S | 601.2955 | 1.9 | -1.2 |
| B3A003-01 | C37H45N5O3 | 608.3595 | 6.5 | 4.9 |
| B3A004-01 | C36H44N6O3 | 609.3548 | 3.5 | -0.5 |
| B3A005-01 | C36H42FN5O3 | 612.3344 | -1.5 | -4.8 |
| B3A006-01 | C35H41FN6O3 | 613.3297 | 0.0 | 0.0 |
| B3A007-01 | C38H43N5O3 | 618.3439 | 9.1 | -5.1 |
| B3A008-01 | C37H42N6O3 | 619.3391 | 0.6 | -0.3 |
| B3A009-01 | C37H42N6O3 | 619.3391 | 0.6 | -0.3 |
| B3A010-01 | C36H41N7O3 | 620.3344 | 0.0 | 0.0 |
| B3A011-01 | C38H47N5O3 | 622.3752 | 4.5 | 2.0 |
| B3A012-01 | C37H46N6O3 | 623.3704 | 1.4 | -2.7 |

| Compound No. | Molecular formula | m/z (expected) | ASMS ratio(%) | |
|---|---|---|---|---|
| | | | n=1 | n=2 |
| B3A013-01 | C40H45N5O3 | 644.3595 | 10.2 | -20.0 |
| B3A014-01 | C39H44N6O3 | 645.3548 | 0.6 | 0.8 |
| B3A015-01 | C40H51N5O3 | 650.4065 | 8.9 | 3.0 |
| B3A016-01 | C39H50N6O3 | 651.4017 | 2.8 | 0.1 |
| B3A017-01 | C37H42F3N5O3 | 662.3313 | 6.9 | -6.3 |
| B3A018-01 | C36H41F3N6O3 | 663.3265 | 5.0 | 1.8 |
| B3A019-01 | C39H42F3N5O3 | 686.3313 | 2.6 | -2.3 |
| B3A020-01 | C38H41F3N6O3 | 687.3265 | 7.5 | -3.2 |

na = not available

[Table 280]

| Compound No. | Molecular formula | m/z (expected) | ASMS ratio(%) | |
|---|---|---|---|---|
| | | | n=1 | n=2 |
| B3A021-01 | C40H44F3N7O3 | 728.3531 | 12.5 | -8.4 |
| B3A022-01 | C39H43F3N8O3 | 729.3483 | 4.9 | 7.2 |
| B3A023-01 | C36H43N3O3S | 598.3098 | 2.4 | 1.7 |
| B3A024-01 | C35H42N4O3S | 599.305 | 1.6 | -0.4 |
| B3A025-01 | C39H47N3O3 | 606.369 | 15.7 | -19.6 |
| B3A026-01 | C38H46N4O3 | 607.3643 | 4.4 | 0.6 |
| B3A027-01 | C38H44FN3O3 | 610.344 | -0.7 | -1.6 |
| B3A028-01 | C37H43FN4O3 | 611.3392 | 9.8 | 1.6 |
| B3A029-01 | C40H45N3O3 | 616.3534 | 0.0 | 0.0 |
| B3A030-01 | C39H44N4O3 | 617.3486 | 8.4 | -15.2 |
| B3A031-01 | C39H44N4O3 | 617.3486 | 8.4 | -15.2 |
| B3A032-01 | C38H43N5O3 | 618.3439 | 9.1 | -5.1 |
| B3A033-01 | C40H49N3O3 | 620.3847 | 1.1 | -0.6 |
| B3A034-01 | C39H48N4O3 | 621.3799 | 1.3 | -0.6 |
| B3A035-01 | C42H47N3O3 | 642.369 | 9.7 | 4.4 |
| B3A036-01 | C41H46N4O3 | 643.3643 | 2.8 | -3.3 |
| B3A037-01 | C42H53N3O3 | 648.416 | 0.0 | 0.0 |
| B3A038-01 | C41H52N4O3 | 649.4112 | 2.5 | -2.9 |
| B3A039-01 | C39H44F3N3O3 | 660.3408 | 3.8 | 0.0 |
| B3A040-01 | C38H43F3N4O3 | 661.336 | 3.5 | -6.7 |
| B3A041-01 | C41H44F3N3O3 | 684.3408 | na | 425.9 |
| B3A042-01 | C40H43F3N4O3 | 685.336 | -0.2 | -1.2 |
| B3A043-01 | C42H46F3N5O3 | 726.3626 | 0.5 | -0.3 |
| B3A044-01 | C41H45F3N6O3 | 727.3578 | 12.3 | -12.4 |
| B3A045-01 | C38H47N3O2 | 578.3741 | 3.2 | 2.5 |
| B3A046-01 | C37H46N4O2 | 579.3694 | 1.5 | 3.6 |
| B3A047-01 | C39H49N3O2 | 592.3898 | 1.8 | -5.9 |
| B3A048-01 | C38H48N4O2 | 593.385 | 7.3 | -8.8 |
| B3A049-01 | C40H51N3O2 | 606.4054 | na | na |
| B3A050-01 | C39H50N4O2 | 607.4007 | 6.1 | -4.3 |
| B3A051-01 | C38H46FN3O2 | 596.3647 | 21.3 | -4.2 |
| B3A052-01 | C37H45FN4O2 | 597.3599 | na | na |
| B3A053-01 | C39H49N3O3 | 608.3847 | 12.5 | 9.9 |
| B3A054-01 | C38H48N4O3 | 609.3799 | 0.0 | 0.0 |
| B3A055-01 | C40H51N3O3 | 622.4003 | 10.8 | -12.7 |
| B3A056-01 | C39H50N4O3 | 623.3956 | na | 0.0 |
| B3A057-01 | C41H53N3O3 | 636.416 | 6.3 | 2.5 |
| B3A058-01 | C40H52N4O3 | 637.4112 | 5.9 | -7.6 |
| B3A059-01 | C40H51N3O4 | 638.3952 | -1.9 | -8.0 |
| B3A060-01 | C39H50N4O4 | 639.3905 | 0.9 | 0.0 |
| B3A061-01 | C39H46F3N3O2 | 646.3615 | na | 421.3 |
| B3A062-01 | C38H45F3N4O2 | 647.3567 | 2.2 | 2.8 |
| B3A063-01 | C39H46F3N3O3 | 662.3564 | na | 390.0 |
| B3A064-01 | C38H45F3N4O3 | 663.3517 | -7.5 | -5.5 |
| B3A065-01 | C42H49N3O2 | 628.3898 | 0.0 | 0.0 |
| B3A066-01 | C41H48N4O2 | 629.385 | 6.3 | -2.9 |
| B3A067-01 | C36H45N3O2S | 584.3305 | -7.3 | 4.0 |
| B3A068-01 | C35H44N4O2S | 585.3258 | 1.4 | -3.6 |
| B3A069-01 | C40H47N3O2 | 602.3741 | na | 12872.1 |
| B3A070-01 | C39H46N4O2 | 603.3694 | 7.7 | -8.1 |
| B3A071-01 | C36H45N5O2 | 580.3646 | 3.2 | -4.7 |
| B3A072-01 | C35H44N6O2 | 581.3599 | 11.6 | 3.1 |
| B3A073-01 | C37H47N5O2 | 594.3803 | 31.3 | 28.6 |
| B3A074-01 | C36H46N6O2 | 595.3755 | 10.7 | -17.2 |
| B3A075-01 | C38H49N5O2 | 608.3959 | -5.8 | -16.0 |
| B3A076-01 | C37H48N6O2 | 609.3912 | 6.5 | -0.9 |
| B3A077-01 | C36H44FN5O2 | 598.3552 | 4.1 | -2.8 |
| B3A078-01 | C35H43FN6O2 | 599.3504 | 4.5 | -1.5 |
| B3A079-01 | C37H47N5O3 | 610.3752 | -5.8 | 5.4 |
| B3A080-01 | C36H46N6O3 | 611.3704 | 1.7 | -1.8 |
| B3A081-01 | C38H49N5O3 | 624.3908 | 13.4 | -14.2 |
| B3A082-01 | C37H48N6O3 | 625.3861 | -0.9 | 4.4 |
| B3A083-01 | C39H51N5O3 | 638.4065 | 3.9 | -18.6 |
| B3A084-01 | C38H50N6O3 | 639.4017 | -17.6 | -7.4 |
| B3A085-01 | C38H49N5O4 | 640.3857 | 5.3 | -7.1 |
| B3A086-01 | C37H48N6O4 | 641.381 | -2.7 | -1.9 |
| B3A087-01 | C37H44F3N5O2 | 648.352 | 0.4 | 1.4 |
| B3A088-01 | C36H43F3N6O2 | 649.3472 | 2.5 | 2.4 |
| B3A089-01 | C37H44F3N5O3 | 664.3469 | -5.5 | -7.2 |
| B3A090-01 | C36H43F3N6O3 | 665.3422 | 5.8 | -0.2 |
| B3A091-01 | C40H47N5O2 | 630.3803 | -1.0 | -16.5 |
| B3A092-01 | C39H46N6O2 | 631.3755 | 1.1 | -1.9 |
| B3A093-01 | C34H43N5O2S | 586.321 | -3.8 | 0.0 |
| B3A094-01 | C33H42N6O2S | 587.3163 | 3.7 | 0.5 |
| B3A095-01 | C38H45N5O2 | 604.3646 | -4.9 | -3.5 |
| B3A096-01 | C37H44N6O2 | 605.3599 | 7.7 | 2.4 |
| B3A097-01 | C39H49N3O2 | 592.3898 | 1.8 | -5.9 |
| B3A098-01 | C38H48N4O2 | 593.385 | 0.6 | 3.0 |
| B3A099-01 | C39H48FN3O2 | 610.3803 | -2.6 | 25.2 |
| B3A100-01 | C38H47FN4O2 | 611.3756 | -39.8 | 0.0 |
| B4J001-01 | C26H31N5O3S | 494.222 | 0.0 | 0.0 |
| B4J002-01 | C25H30N6O3S | 495.2173 | -2.7 | 2.1 |
| B4J003-01 | C29H35N5O3 | 502.2813 | 0.0 | 0.0 |
| B4J004-01 | C28H34N6O3 | 503.2765 | -2.2 | 0.4 |
| B4J005-01 | C28H32FN5O3 | 506.2562 | -0.4 | 2.1 |
| B4J006-01 | C27H31FN6O3 | 507.2514 | 0.0 | 0.0 |
| B4J007-01 | C30H33N5O3 | 512.2656 | -1.0 | 1.5 |
| B4J008-01 | C29H32N6O3 | 513.2609 | 0.0 | 0.0 |
| B4J009-01 | C29H32N6O3 | 513.2609 | 0.0 | 0.0 |
| B4J010-01 | C28H31N7O3 | 514.2561 | 0.0 | 2.3 |
| B4J011-01 | C30H37N5O3 | 516.2969 | -0.1 | -0.9 |
| B4J012-01 | C29H36N6O3 | 517.2922 | -1.1 | -0.8 |
| B4J013-01 | C32H35N5O3 | 538.2813 | 2.0 | 3.7 |
| B4J014-01 | C31H34N6O3 | 539.2765 | 0.0 | -0.8 |
| B4J015-01 | C32H41N5O3 | 544.3282 | 0.0 | 0.0 |
| B4J016-01 | C31H40N6O3 | 545.3235 | 0.0 | 0.9 |
| B4J017-01 | C29H32F3N5O3 | 556.253 | -0.3 | -0.5 |
| B4J018-01 | C28H31F3N6O3 | 557.2483 | -0.5 | -1.7 |

| Compound No. | Molecular formula | m/z (expected) | ASMS ratio(%) | | Compound No. | Molecular formula | m/z (expected) | ASMS ratio(%) | |
|---|---|---|---|---|---|---|---|---|---|
| | | | n=1 | n=2 | | | | n=1 | n=2 |
| B4J019-01 | C31H32F3N5O3 | 580.253 | -0.5 | -2.1 | B4J040-01 | C30H33F3N4O3 | 555.2578 | -0.5 | -1.2 |
| B4J020-01 | C30H31F3N6O3 | 581.2483 | 0.0 | 0.0 | B4J041-01 | C33H34F3N3O3 | 578.2625 | 2.8 | 1.7 |
| B4J021-01 | C32H34F3N7O3 | 622.2748 | 0.0 | 0.0 | B4J042-01 | C32H33F3N4O3 | 579.2578 | 1.0 | 0.0 |
| B4J022-01 | C31H33F3N8O3 | 623.2701 | 0.2 | -0.7 | B4J043-01 | C34H36F3N5O3 | 620.2843 | 3.8 | 3.2 |
| B4J023-01 | C28H33N3O3S | 492.2315 | 3.5 | -3.4 | B4J044-01 | C33H35F3N6O3 | 621.2796 | 1.8 | 0.3 |
| B4J024-01 | C27H32N4O3S | 493.2268 | -0.6 | 6.8 | B4J045-01 | C30H37N3O2 | 472.2959 | 1.1 | 0.3 |
| B4J025-01 | C31H37N3O3 | 500.2908 | 3.6 | -0.3 | B4J046-01 | C29H36N4O2 | 473.2911 | 0.0 | 0.0 |
| B4J026-01 | C30H36N4O3 | 501.286 | -3.6 | -5.9 | B4J047-01 | C31H39N3O2 | 486.3115 | -6.6 | 0.0 |
| B4J027-01 | C30H34FN3O3 | 504.2657 | -0.5 | 0.2 | B4J048-01 | C30H38N4O2 | 487.3068 | 1.2 | -2.1 |
| B4J028-01 | C29H33FN4O3 | 505.261 | -1.0 | 0.9 | B4J049-01 | C32H41N3O2 | 500.3272 | 7.1 | -1.4 |
| B4J029-01 | C32H35N3O3 | 510.2751 | 0.0 | 0.0 | B4J050-01 | C31H40N4O2 | 501.3224 | 2.1 | 0.6 |
| B4J030-01 | C31H34N4O3 | 511.2704 | 0.1 | 0.8 | B4J051-01 | C30H36FN3O2 | 490.2864 | -9.6 | -10.2 |
| B4J031-01 | C31H34N4O3 | 511.2704 | 0.1 | 0.8 | B4J052-01 | C29H35FN4O2 | 491.2817 | 0.5 | -1.5 |
| B4J032-01 | C30H33N5O3 | 512.2656 | -7.2 | 1.5 | B4J053-01 | C31H39N3O3 | 502.3064 | -0.1 | -6.0 |
| B4J033-01 | C32H39N3O3 | 514.3064 | 0.0 | -0.9 | B4J054-01 | C30H38N4O3 | 503.3017 | 0.0 | -1.1 |
| B4J034-01 | C31H38N4O3 | 515.3017 | -0.7 | 0.9 | B4J055-01 | C32H41N3O3 | 516.3221 | -0.7 | 1.4 |
| B4J035-01 | C34H37N3O3 | 536.2908 | 4.1 | 1.5 | B4J056-01 | C31H40N4O3 | 517.3173 | -1.4 | -1.5 |
| B4J036-01 | C33H36N4O3 | 537.286 | -0.3 | -1.6 | B4J057-01 | C33H43N3O3 | 530.3377 | 5.3 | -0.1 |
| B4J037-01 | C34H43N3O3 | 542.3377 | 2.7 | -1.5 | B4J058-01 | C32H42N4O3 | 531.333 | 2.2 | 0.5 |
| B4J038-01 | C33H42N4O3 | 543.333 | 0.8 | 0.0 | B4J059-01 | C32H41N3O4 | 532.317 | -0.1 | -6.0 |
| B4J039-01 | C31H34F3N3O3 | 554.2625 | 2.2 | 0.1 | B4J060-01 | C31H40N4O4 | 533.3122 | 0.0 | 0.0 |

na = not available

[Table 281]

| Compound No. | Molecular formula | m/z (expected) | ASMS ratio(%) | | Compound No. | Molecular formula | m/z (expected) | ASMS ratio(%) | |
|---|---|---|---|---|---|---|---|---|---|
| | | | n=1 | n=2 | | | | n=1 | n=2 |
| B4J061-01 | C31H36F3N3O2 | 540.2832 | 1.6 | -1.8 | B4J093-01 | C26H33N5O2S | 480.2428 | -2.1 | 0.6 |
| B4J062-01 | C30H35F3N4O2 | 541.2785 | 0.0 | 0.0 | B4J094-01 | C25H32N6O2S | 481.238 | -1.3 | 0.1 |
| B4J063-01 | C31H36F3N3O3 | 556.2782 | 4.5 | 0.1 | B4J095-01 | C30H35N5O2 | 498.2864 | 1.5 | 0.0 |
| B4J064-01 | C30H35F3N4O3 | 557.2734 | 1.6 | 0.0 | B4J096-01 | C29H34N6O2 | 499.2816 | na | 0.0 |
| B4J065-01 | C34H39N3O2 | 522.3115 | 0.0 | 0.0 | B4J097-01 | C31H39N3O2 | 486.3115 | -0.9 | -0.4 |
| B4J066-01 | C33H38N4O2 | 523.3068 | 0.0 | -1.5 | B4J098-01 | C30H38N4O2 | 487.3068 | -2.0 | -0.2 |
| B4J067-01 | C28H35N3O2S | 478.2523 | -11.0 | -5.5 | B4J099-01 | C31H38FN3O2 | 504.3021 | na | 0.0 |
| B4J068-01 | C27H34N4O2S | 479.2475 | 0.7 | 0.0 | B4J100-01 | C30H37FN4O2 | 505.2973 | 0.0 | 0.0 |
| B4J069-01 | C32H37N3O2 | 496.2959 | 1.8 | -0.8 | B2Q001-01 | C36H35N7O7S3 | 774.1833 | 0.0 | 0.0 |
| B4J070-01 | C31H36N4O2 | 497.2911 | 0.0 | 0.0 | B2Q002-01 | C35H34N8O7S3 | 775.1785 | 24.5 | 17.2 |
| B4J071-01 | C28H35N5O2 | 474.2864 | 0.4 | -0.5 | B2Q003-01 | C39H39N7O7S2 | 782.2425 | 8.2 | 6.9 |
| B4J072-01 | C27H34N6O2 | 475.2816 | -1.6 | 0.9 | B2Q004-01 | C38H38N8O7S2 | 783.2378 | 10.7 | 8.7 |
| B4J073-01 | C29H37N5O2 | 488.302 | 2.1 | 0.0 | B2Q005-01 | C38H36FN7O7S2 | 786.2174 | 12.5 | 15.3 |
| B4J074-01 | C28H36N6O2 | 489.2973 | 0.0 | 0.0 | B2Q006-01 | C37H35FN8O7S2 | 787.2127 | 9.5 | 2.2 |
| B4J075-01 | C30H39N5O2 | 502.3177 | 0.0 | 0.0 | B2Q007-01 | C40H37N7O7S2 | 792.2269 | 4.2 | -8.3 |
| B4J076-01 | C29H38N6O2 | 503.3129 | 0.0 | 0.0 | B2Q008-01 | C39H36N8O7S2 | 793.2221 | 16.5 | 0.5 |
| B4J077-01 | C28H34FN5O2 | 492.2769 | 1.2 | -5.5 | B2Q009-01 | C39H36N8O7S2 | 793.2221 | 16.5 | 0.5 |
| B4J078-01 | C27H33FN6O2 | 493.2722 | -0.5 | 0.9 | B2Q010-01 | C38H35N9O7S2 | 794.2174 | 13.3 | 8.9 |
| B4J079-01 | C29H37N5O3 | 504.2969 | 0.0 | 0.0 | B2Q011-01 | C40H41N7O7S2 | 796.2582 | 25.0 | -1.1 |
| B4J080-01 | C28H36N6O3 | 505.2922 | 0.0 | 0.0 | B2Q012-01 | C39H40N8O7S2 | 797.2534 | 21.4 | 22.5 |
| B4J081-01 | C30H39N5O3 | 518.3126 | 0.0 | 0.0 | B2Q013-01 | C42H39N7O7S2 | 818.2425 | 0.0 | 0.0 |
| B4J082-01 | C29H38N6O3 | 519.3078 | 0.0 | 0.0 | B2Q014-01 | C41H38N8O7S2 | 819.2378 | 9.3 | 14.5 |
| B4J083-01 | C31H41N5O3 | 532.3282 | 0.0 | 0.0 | B2Q015-01 | C42H45N7O7S2 | 824.2895 | 17.1 | 13.2 |
| B4J084-01 | C30H40N6O3 | 533.3235 | 0.0 | -0.6 | B2Q016-01 | C41H44N8O7S2 | 825.2847 | 10.6 | 6.5 |
| B4J085-01 | C30H39N5O4 | 534.3075 | 0.0 | 2.8 | B2Q017-01 | C39H36F3N7O7S2 | 836.2143 | 12.8 | 0.2 |
| B4J086-01 | C29H38N6O4 | 535.3027 | 0.0 | 0.0 | B2Q018-01 | C38H35F3N8O7S2 | 837.2095 | na | 0.0 |
| B4J087-01 | C29H34F3N5O2 | 542.2737 | 0.9 | 0.3 | B2Q019-01 | C41H36F3N7O7S2 | 860.2143 | 0.7 | 14.1 |
| B4J088-01 | C28H33F3N6O2 | 543.269 | 0.0 | 0.0 | B2Q020-01 | C40H35F3N8O7S2 | 861.2095 | 6.1 | 5.6 |
| B4J089-01 | C29H34F3N5O3 | 558.2687 | 3.9 | 0.4 | B2Q021-01 | C42H38F3N9O7S2 | 902.2361 | 0.0 | 0.0 |
| B4J090-01 | C28H33F3N6O3 | 559.2639 | 1.4 | -1.5 | B2Q022-01 | C41H37F3N10O7S2 | 903.2313 | 13.8 | 23.6 |
| B4J091-01 | C32H37N5O2 | 524.302 | 3.7 | 1.5 | B2Q023-01 | C38H37N5O7S3 | 772.1928 | 0.0 | 0.0 |
| B4J092-01 | C31H36N6O2 | 525.2973 | -1.4 | 0.3 | B2Q024-01 | C37H36N6O7S3 | 773.188 | 4.5 | 7.5 |

| Compound No. | Molecular formula | m/z (expected) | ASMS ratio(%) | |
|---|---|---|---|---|
| | | | n=1 | n=2 |
| B2Q025-01 | C41H41N5O7S2 | 780.252 | -5.7 | 0.0 |
| B2Q026-01 | C40H40N6O7S2 | 781.2473 | 4.4 | 0.4 |
| B2Q027-01 | C40H38FN5O7S2 | 784.2269 | 0.2 | -2.5 |
| B2Q028-01 | C39H37FN6O7S2 | 785.2222 | 0.0 | -3.7 |
| B2Q029-01 | C42H39N5O7S2 | 790.2364 | na | na |
| B2Q030-01 | C41H38N6O7S2 | 791.2316 | 6.6 | -4.1 |
| B2Q031-01 | C41H38N6O7S2 | 791.2316 | 6.6 | -4.1 |
| B2Q032-01 | C40H37N7O7S2 | 792.2269 | -0.2 | -374.2 |
| B2Q033-01 | C42H43N5O7S2 | 794.2677 | 4.2 | -2.3 |
| B2Q034-01 | C41H42N6O7S2 | 795.2629 | 0.0 | 0.0 |
| B2Q035-01 | C44H41N5O7S2 | 816.252 | 9.9 | -13.7 |
| B2Q036-01 | C43H40N6O7S2 | 817.2473 | 0.0 | -6.0 |
| B2Q037-01 | C44H47N5O7S2 | 822.299 | 10.9 | -1.5 |
| B2Q038-01 | C43H46N6O7S2 | 823.2942 | 3.3 | 0.4 |
| B2Q039-01 | C41H38F3N5O7S2 | 834.2238 | 7.2 | 4.6 |
| B2Q040-01 | C40H37F3N6O7S2 | 835.219 | 3.9 | 1.5 |
| B2Q041-01 | C43H38F3N5O7S2 | 858.2238 | 2.6 | 0.2 |
| B2Q042-01 | C42H37F3N6O7S2 | 859.219 | 26.1 | 19.5 |
| B2Q043-01 | C44H40F3N7O7S2 | 900.2456 | 12.4 | -13.4 |
| B2Q044-01 | C43H39F3N8O7S2 | 901.2408 | 11.0 | -1.3 |
| B2Q045-01 | C40H41N5O6S2 | 752.2571 | 49.9 | 49.9 |
| B2Q046-01 | C39H40N6O6S2 | 753.2524 | 20.3 | 20.5 |
| B2Q047-01 | C41H43N5O6S2 | 766.2728 | 142.5 | 326.4 |
| B2Q048-01 | C40H42N6O6S2 | 767.268 | 61.6 | 90.8 |
| B2Q049-01 | C42H45N5O6S2 | 780.2884 | -2.4 | -4.8 |
| B2Q050-01 | C41H44N6O6S2 | 781.2837 | 0.0 | 0.0 |
| B2Q051-01 | C40H40FN5O6S2 | 770.2477 | 22.6 | 2.1 |
| B2Q052-01 | C39H39FN6O6S2 | 771.2429 | 22.8 | 13.9 |
| B2Q053-01 | C41H43N5O7S2 | 782.2677 | 22.4 | 15.5 |
| B2Q054-01 | C40H42N6O7S2 | 783.2629 | 44.1 | 65.0 |
| B2Q055-01 | C42H45N5O7S2 | 796.2833 | 9.0 | -8.3 |
| B2Q056-01 | C41H44N6O7S2 | 797.2786 | 7.3 | 2.8 |
| B2Q057-01 | C43H47N5O7S2 | 810.299 | 17.5 | 4.4 |
| B2Q058-01 | C42H46N6O7S2 | 811.2942 | 3.7 | 3.7 |
| B2Q059-01 | C42H45N5O8S2 | 812.2782 | 9.8 | 7.0 |
| B2Q060-01 | C41H44N6O8S2 | 813.2735 | 0.0 | 0.0 |
| B2Q061-01 | C41H40F3N5O6S2 | 820.2445 | 15.6 | -17.9 |
| B2Q062-01 | C40H39F3N6O6S2 | 821.2397 | 9.4 | -3.3 |
| B2Q063-01 | C41H40F3N5O7S2 | 836.2394 | 13.3 | -25.5 |

| Compound No. | Molecular formula | m/z (expected) | ASMS ratio(%) | |
|---|---|---|---|---|
| | | | n=1 | n=2 |
| B2Q064-01 | C40H39F3N6O7S2 | 837.2347 | 8.6 | -1.6 |
| B2Q065-01 | C44H43N5O6S2 | 802.2728 | 30.6 | -1.6 |
| B2Q066-01 | C43H42N6O6S2 | 803.268 | 40.7 | 36.9 |
| B2Q067-01 | C38H39N5O6S3 | 758.2135 | na | na |
| B2Q068-01 | C37H38N6O6S3 | 759.2088 | na | na |
| B2Q069-01 | C42H41N5O6S2 | 776.2571 | 8.8 | 11.0 |
| B2Q070-01 | C41H40N6O6S2 | 777.2524 | 14.3 | -9.0 |
| B2Q071-01 | C38H39N7O6S2 | 754.2476 | 25.0 | 11.2 |
| B2Q072-01 | C37H38N8O6S2 | 755.2429 | 10.2 | 11.4 |
| B2Q073-01 | C39H41N7O6S2 | 768.2633 | 0.0 | 0.0 |
| B2Q074-01 | C38H40N8O6S2 | 769.2585 | 18.8 | 15.4 |
| B2Q075-01 | C40H43N7O6S2 | 782.2789 | 0.0 | 0.0 |
| B2Q076-01 | C39H42N8O6S2 | 783.2742 | 0.0 | na |
| B2Q077-01 | C38H38FN7O6S2 | 772.2382 | 14.7 | 17.2 |
| B2Q078-01 | C37H37FN8O6S2 | 773.2334 | 15.6 | 12.1 |
| B2Q079-01 | C39H41N7O7S2 | 784.2582 | 17.6 | 0.0 |
| B2Q080-01 | C38H40N8O7S2 | 785.2534 | 12.6 | 3.6 |
| B2Q081-01 | C40H43N7O7S2 | 798.2738 | -4.8 | 0.6 |
| B2Q082-01 | C39H42N8O7S2 | 799.2691 | 21.3 | 8.5 |
| B2Q083-01 | C41H45N7O7S2 | 812.2895 | na | 0.0 |
| B2Q084-01 | C40H44N8O7S2 | 813.2847 | 12.6 | -4.9 |
| B2Q085-01 | C40H43N7O8S2 | 814.2687 | 14.9 | 0.7 |
| B2Q086-01 | C39H42N8O8S2 | 815.264 | -39.4 | 0.0 |
| B2Q087-01 | C39H38F3N7O6S2 | 822.235 | 17.6 | -2.1 |
| B2Q088-01 | C38H37F3N8O6S2 | 823.2302 | 13.8 | 9.1 |
| B2Q089-01 | C39H38F3N7O7S2 | 838.2299 | 25.0 | -15.0 |
| B2Q090-01 | C38H37F3N8O7S2 | 839.2252 | 21.8 | 13.2 |
| B2Q091-01 | C42H41N7O6S2 | 804.2633 | 17.2 | -5.0 |
| B2Q092-01 | C41H40N8O6S2 | 805.2585 | 10.4 | 4.8 |
| B2Q093-01 | C36H37N7O6S3 | 760.204 | na | na |
| B2Q094-01 | C35H36N8O6S3 | 761.1993 | 7.2 | 2.5 |
| B2Q095-01 | C40H39N7O6S2 | 778.2476 | 19.9 | 11.9 |
| B2Q096-01 | C39H38N8O6S2 | 779.2429 | na | na |
| B2Q097-01 | C41H43N5O6S2 | 766.2728 | 142.5 | 326.4 |
| B2Q098-01 | C40H42N6O6S2 | 767.268 | 61.6 | 90.8 |
| B2Q099-01 | C41H42FN5O6S2 | 784.2633 | na | 0.0 |
| B2Q100-01 | C40H41FN6O6S2 | 785.2586 | 0.0 | 10.7 |

na = not available

[Table 282]

| Compound No. | Molecular formula | m/z (expected) | ASMS ratio(%) | |
|---|---|---|---|---|
| | | | n=1 | n=2 |
| B2A001-01 | C33H39N5O5S2 | 650.2465 | 0.0 | 0.0 |
| B2A002-01 | C32H38N6O5S2 | 651.2418 | 0.0 | 0.0 |
| B2A003-01 | C36H43N5O5S | 658.3058 | 21.0 | 11.0 |
| B2A004-01 | C35H42N6O5S | 659.301 | 0.0 | 0.0 |
| B2A005-01 | C35H40FN5O5S | 662.2807 | 4.7 | 12.4 |
| B2A006-01 | C34H39FN6O5S | 663.2759 | 0.0 | 0.0 |
| B2A007-01 | C37H41N5O5S | 668.2901 | na | 0.0 |
| B2A008-01 | C36H40N6O5S | 669.2854 | 3.1 | 7.2 |
| B2A009-01 | C36H40N6O5S | 669.2854 | 3.1 | 7.2 |
| B2A010-01 | C35H39N7O5S | 670.2806 | 0.0 | 0.0 |
| B2A011-01 | C37H45N5O5S | 672.3214 | 9.0 | 0.3 |
| B2A012-01 | C36H44N6O5S | 673.3167 | 1.5 | 1.9 |
| B2A013-01 | C39H43N5O5S | 694.3058 | 11.6 | 17.8 |
| B2A014-01 | C38H42N6O5S | 695.301 | 7.7 | 2.2 |

| Compound No. | Molecular formula | m/z (expected) | ASMS ratio(%) | |
|---|---|---|---|---|
| | | | n=1 | n=2 |
| B2A015-01 | C39H49N5O5S | 700.3527 | 5.9 | 9.7 |
| B2A016-01 | C38H48N6O5S | 701.348 | 0.0 | 2.5 |
| B2A017-01 | C36H40F3N5O5S | 712.2775 | 18.6 | 7.5 |
| B2A018-01 | C35H39F3N6O5S | 713.2728 | 5.1 | 0.0 |
| B2A019-01 | C38H40F3N5O5S | 736.2775 | 7.4 | 5.0 |
| B2A020-01 | C37H39F3N6O5S | 737.2728 | 3.4 | 8.9 |
| B2A021-01 | C39H42F3N7O5S | 778.2993 | 14.4 | 9.4 |
| B2A022-01 | C38H41F3N8O5S | 779.2946 | 27.0 | 3.7 |
| B2A023-01 | C35H41N3O5S2 | 648.256 | 0.0 | 0.0 |
| B2A024-01 | C34H40N4O5S2 | 649.2513 | -1.9 | 0.0 |
| B2A025-01 | C38H45N3O5S | 656.3153 | 4.6 | -2.7 |
| B2A026-01 | C37H44N4O5S | 657.3105 | 0.8 | -2.1 |
| B2A027-01 | C37H42FN3O5S | 660.2902 | 5.5 | -3.9 |
| B2A028-01 | C36H41FN4O5S | 661.2855 | -1.0 | 1.2 |

| Compound No. | Molecular formula | m/z (expected) | ASMS ratio(%) | |
|---|---|---|---|---|
| | | | n=1 | n=2 |
| B2A029-01 | C39H43N3O5S | 666.2996 | na | na |
| B2A030-01 | C38H42N4O5S | 667.2949 | 0.0 | 0.3 |
| B2A031-01 | C38H42N4O5S | 667.2949 | 0.0 | 0.3 |
| B2A032-01 | C37H41N5O5S | 668.2901 | 0.0 | 0.0 |
| B2A033-01 | C39H47N3O5S | 670.3309 | 1.7 | -4.5 |
| B2A034-01 | C38H46N4O5S | 671.3262 | 1.6 | 0.3 |
| B2A035-01 | C41H45N3O5S | 692.3153 | 6.2 | -9.8 |
| B2A036-01 | C40H44N4O5S | 693.3105 | 2.0 | -0.4 |
| B2A037-01 | C41H51N3O5S | 698.3622 | 3.0 | 0.0 |
| B2A038-01 | C40H50N4O5S | 699.3575 | 1.8 | -3.5 |
| B2A039-01 | C38H42F3N3O5S | 710.287 | 7.4 | -10.4 |
| B2A040-01 | C37H41F3N4O5S | 711.2823 | 0.0 | 0.0 |
| B2A041-01 | C40H42F3N3O5S | 734.287 | 0.0 | -3.7 |
| B2A042-01 | C39H41F3N4O5S | 735.2823 | 10.1 | -0.9 |
| B2A043-01 | C41H44F3N5O5S | 776.3088 | 14.0 | 4.4 |
| B2A044-01 | C40H43F3N6O5S | 777.3041 | 0.7 | -0.2 |
| B2A045-01 | C37H45N3O4S | 628.3204 | 6.5 | -2.9 |
| B2A046-01 | C36H44N4O4S | 629.3156 | -2.7 | -22.1 |
| B2A047-01 | C38H47N3O4S | 642.336 | 9.7 | 0.4 |
| B2A048-01 | C37H46N4O4S | 643.3313 | 18.2 | 2.1 |
| B2A049-01 | C39H49N3O4S | 656.3517 | -3.0 | 6.2 |
| B2A050-01 | C38H48N4O4S | 657.3469 | -1.1 | 1.6 |
| B2A051-01 | C37H44FN3O4S | 646.3109 | -0.3 | 3.6 |
| B2A052-01 | C36H43FN4O4S | 647.3062 | 0.0 | 0.0 |
| B2A053-01 | C38H47N3O5S | 658.3309 | 0.0 | 0.0 |
| B2A054-01 | C37H46N4O5S | 659.3262 | 17.5 | -0.9 |
| B2A055-01 | C39H49N3O5S | 672.3466 | 5.8 | -7.3 |
| B2A056-01 | C38H48N4O5S | 673.3418 | -3.9 | 0.2 |
| B2A057-01 | C40H51N3O5S | 686.3622 | -4.8 | -5.3 |
| B2A058-01 | C39H50N4O5S | 687.3575 | 4.8 | -10.6 |
| B2A059-01 | C39H49N3O6S | 688.3415 | 0.0 | -42.2 |
| B2A060-01 | C38H48N4O6S | 689.3367 | 0.0 | 0.0 |
| B2A061-01 | C38H44F3N3O4S | 696.3077 | 1.7 | -5.6 |
| B2A062-01 | C37H43F3N4O4S | 697.303 | -5.2 | 3.6 |
| B2A063-01 | C38H44F3N3O5S | 712.3027 | 0.5 | 3.3 |
| B2A064-01 | C37H43F3N4O5S | 713.2979 | 0.0 | -0.3 |
| B2A065-01 | C41H47N3O4S | 678.336 | 10.5 | -2.9 |
| B2A066-01 | C40H46N4O4S | 679.3313 | 3.2 | 0.0 |
| B2A067-01 | C35H43N3O4S2 | 634.2768 | 0.0 | 0.0 |
| B2A068-01 | C34H42N4O4S2 | 635.272 | 0.0 | 0.0 |
| B2A069-01 | C39H45N3O4S | 652.3204 | 4.6 | -4.6 |
| B2A070-01 | C38H44N4O4S | 653.3156 | na | 0.0 |
| B2A071-01 | C35H43N5O4S | 630.3109 | 16.1 | 5.6 |
| B2A072-01 | C34H42N6O4S | 631.3061 | 12.2 | 14.7 |
| B2A073-01 | C36H45N5O4S | 644.3265 | 16.2 | 13.5 |
| B2A074-01 | C35H44N6O4S | 645.3218 | 9.2 | 1.9 |
| B2A075-01 | C37H47N5O4S | 658.3422 | 4.6 | 4.4 |
| B2A076-01 | C36H46N6O4S | 659.3374 | 23.4 | 11.4 |
| B2A077-01 | C35H42FN5O4S | 648.3014 | 14.3 | 21.9 |
| B2A078-01 | C34H41FN6O4S | 649.2967 | 15.2 | 9.3 |
| B2A079-01 | C36H45N5O5S | 660.3214 | na | na |
| B2A080-01 | C35H44N6O5S | 661.3167 | 0.0 | 0.0 |
| B2A081-01 | C37H47N5O5S | 674.3371 | 18.6 | 16.7 |
| B2A082-01 | C36H46N6O5S | 675.3323 | na | na |
| B2A083-01 | C38H49N5O5S | 688.3527 | 10.3 | 0.0 |
| B2A084-01 | C37H48N6O5S | 689.348 | 13.3 | 11.9 |
| B2A085-01 | C37H47N5O6S | 690.332 | 0.0 | 0.0 |
| B2A086-01 | C36H46N6O6S | 691.3272 | 0.0 | 0.0 |

| Compound No. | Molecular formula | m/z (expected) | ASMS ratio(%) | |
|---|---|---|---|---|
| | | | n=1 | n=2 |
| B2A087-01 | C36H42F3N5O4S | 698.2982 | 7.2 | 12.7 |
| B2A088-01 | C35H41F3N6O4S | 699.2935 | 12.0 | 14.0 |
| B2A089-01 | C36H42F3N5O5S | 714.2932 | 10.5 | 0.5 |
| B2A090-01 | C35H41F3N6O5S | 715.2884 | 22.0 | 4.7 |
| B2A091-01 | C39H45N5O4S | 680.3265 | -2.6 | 3.8 |
| B2A092-01 | C38H44N6O4S | 681.3218 | 20.8 | 16.6 |
| B2A093-01 | C33H41N5O4S2 | 636.2673 | 13.0 | 0.0 |
| B2A094-01 | C32H40N6O4S2 | 637.2625 | 0.0 | 0.0 |
| B2A095-01 | C37H43N5O4S | 654.3109 | 1.6 | 13.8 |
| B2A096-01 | C36H42N6O4S | 655.3061 | na | na |
| B2A097-01 | C38H47N3O4S | 642.336 | 9.7 | 0.4 |
| B2A098-01 | C37H46N4O4S | 643.3313 | 18.2 | 2.1 |
| B2A099-01 | C38H46FN3O4S | 660.3266 | 0.0 | 0.0 |
| B2A100-01 | C37H45FN4O4S | 661.3218 | 0.0 | 0.0 |
| B2I001-01 | C23H25N5O5S2 | 516.137 | 0.0 | na |
| B2I002-01 | C22H24N6O5S2 | 517.1322 | 0.0 | 0.0 |
| B2I003-01 | C26H29N5O5S | 524.1962 | 0.0 | 0.0 |
| B2I004-01 | C25H28N6O5S | 525.1915 | 0.9 | -0.3 |
| B2I005-01 | C25H26FN5O5S | 528.1711 | 0.0 | 0.0 |
| B2I006-01 | C24H25FN6O5S | 529.1664 | 0.0 | 0.0 |
| B2I007-01 | C27H27N5O5S | 534.1806 | -1.6 | -1.6 |
| B2I008-01 | C26H26N6O5S | 535.1758 | 0.0 | 0.0 |
| B2I009-01 | C26H26N6O5S | 535.1758 | 0.0 | 0.0 |
| B2I010-01 | C25H25N7O5S | 536.1711 | na | na |
| B2I011-01 | C27H31N5O5S | 538.2119 | 0.0 | 0.0 |
| B2I012-01 | C26H30N6O5S | 539.2071 | -4.0 | -0.2 |
| B2I013-01 | C29H29N5O5S | 560.1962 | 0.0 | 0.0 |
| B2I014-01 | C28H28N6O5S | 561.1915 | 1.0 | 2.3 |
| B2I015-01 | C29H35N5O5S | 566.2432 | 0.0 | 0.0 |
| B2I016-01 | C28H34N6O5S | 567.2384 | 0.0 | 0.0 |
| B2I017-01 | C26H26F3N5O5S | 578.168 | 0.0 | 0.0 |
| B2I018-01 | C25H25F3N6O5S | 579.1632 | 0.0 | 0.0 |
| B2I019-01 | C28H26F3N5O5S | 602.168 | 0.0 | na |
| B2I020-01 | C27H25F3N6O5S | 603.1632 | 0.0 | 0.0 |
| B2I021-01 | C29H28F3N7O5S | 644.1898 | 0.0 | 0.0 |
| B2I022-01 | C28H27F3N8O5S | 645.185 | 0.0 | 0.0 |
| B2I023-01 | C25H27N3O5S2 | 514.1465 | 0.0 | 0.0 |
| B2I024-01 | C24H26N4O5S2 | 515.1417 | 0.0 | 0.0 |
| B2I025-01 | C28H31N3O5S | 522.2057 | 0.0 | 0.0 |
| B2I026-01 | C27H30N4O5S | 523.201 | -2.6 | -0.2 |
| B2I027-01 | C27H28FN3O5S | 526.1807 | 0.0 | 0.0 |
| B2I028-01 | C26H27FN4O5S | 527.1759 | 0.0 | 0.0 |
| B2I029-01 | C29H29N3O5S | 532.1901 | 0.0 | 0.0 |
| B2I030-01 | C28H28N4O5S | 533.1853 | 0.0 | 0.0 |
| B2I031-01 | C28H28N4O5S | 533.1853 | 0.0 | 0.0 |
| B2I032-01 | C27H27N5O5S | 534.1806 | -1.6 | -1.6 |
| B2I033-01 | C29H33N3O5S | 536.2214 | 0.0 | 0.0 |
| B2I034-01 | C28H32N4O5S | 537.2166 | 0.0 | 1.6 |
| B2I035-01 | C31H31N3O5S | 558.2057 | 0.0 | 1.6 |
| B2I036-01 | C30H30N4O5S | 559.201 | -0.7 | 1.5 |
| B2I037-01 | C31H37N3O5S | 564.2527 | 0.0 | 3.3 |
| B2I038-01 | C30H36N4O5S | 565.2479 | 0.0 | 0.0 |
| B2I039-01 | C28H28F3N3O5S | 576.1775 | 0.0 | 0.0 |
| B2I040-01 | C27H27F3N4O5S | 577.1727 | 0.0 | -2.7 |

na = not available

[Table 283]

| Compound No. | Molecular formula | m/z (expected) | ASMS ratio(%) | |
|---|---|---|---|---|
| | | | n=1 | n=2 |
| B2I041-01 | C30H28F3N3O5S | 600.1775 | 0.0 | 0.0 |
| B2I042-01 | C29H27F3N4O5S | 601.1727 | 0.0 | 0.0 |
| B2I043-01 | C31H30F3N5O5S | 642.1993 | 0.0 | 0.0 |
| B2I044-01 | C30H29F3N6O5S | 643.1945 | 0.0 | 0.0 |
| B2I045-01 | C27H31N3O4S | 494.2108 | -0.3 | 0.2 |
| B2I046-01 | C26H30N4O4S | 495.2061 | -0.6 | 1.5 |
| B2I047-01 | C28H33N3O4S | 508.2265 | 0.8 | 1.4 |
| B2I048-01 | C27H32N4O4S | 509.2217 | 0.0 | 0.0 |
| B2I049-01 | C29H35N3O4S | 522.2421 | 0.0 | 0.0 |
| B2I050-01 | C28H34N4O4S | 523.2374 | 0.0 | -1.5 |
| B2I051-01 | C27H30FN3O4S | 512.2014 | 0.0 | 0.0 |
| B2I052-01 | C26H29FN4O4S | 513.1966 | 0.0 | 0.0 |
| B2I053-01 | C28H33N3O5S | 524.2214 | 0.0 | 0.0 |
| B2I054-01 | C27H32N4O5S | 525.2166 | 0.0 | 0.0 |
| B2I055-01 | C29H35N3O5S | 538.237 | 0.0 | 0.0 |
| B2I056-01 | C28H34N4O5S | 539.2323 | 0.0 | 0.0 |
| B2I057-01 | C30H37N3O5S | 552.2527 | 0.0 | 0.0 |
| B2I058-01 | C29H36N4O5S | 553.2479 | 0.0 | 0.0 |
| B2I059-01 | C29H35N3O6S | 554.2319 | 0.0 | 0.0 |
| B2I060-01 | C28H34N4O6S | 555.2272 | 0.0 | 0.0 |
| B2I061-01 | C28H30F3N3O4S | 562.1982 | -0.8 | 0.5 |
| B2I062-01 | C27H29F3N4O4S | 563.1934 | 0.0 | 0.0 |
| B2I063-01 | C28H30F3N3O5S | 578.1931 | 0.9 | 0.0 |
| B2I064-01 | C27H29F3N4O5S | 579.1884 | 0.0 | 0.0 |
| B2I065-01 | C31H33N3O4S | 544.2265 | 0.0 | 0.0 |
| B2I066-01 | C30H32N4O4S | 545.2217 | 0.0 | 0.0 |
| B2I067-01 | C25H29N3O4S2 | 500.1672 | 0.0 | 0.0 |
| B2I068-01 | C24H28N4O4S2 | 501.1625 | 0.0 | 0.0 |
| B2I069-01 | C29H31N3O4S | 518.2108 | 0.0 | -2.2 |
| B2I070-01 | C28H30N4O4S | 519.2061 | na | 0.0 |
| B2I071-01 | C25H29N5O4S | 496.2013 | -1.2 | -1.9 |
| B2I072-01 | C24H28N6O4S | 497.1966 | 0.8 | 1.3 |
| B2I073-01 | C26H31N5O4S | 510.217 | 0.0 | 0.0 |
| B2I074-01 | C25H30N6O4S | 511.2122 | 0.0 | -8.4 |
| B2I075-01 | C27H33N5O4S | 524.2326 | 0.0 | 0.0 |
| B2I076-01 | C26H32N6O4S | 525.2279 | 0.2 | -3.2 |
| B2I077-01 | C25H28FN5O4S | 514.1919 | 0.0 | 0.0 |
| B2I078-01 | C24H27FN6O4S | 515.1871 | 0.0 | 0.0 |
| B2I079-01 | C26H31N5O5S | 526.2119 | 0.0 | 0.0 |
| B2I080-01 | C25H30N6O5S | 527.2071 | 0.0 | na |
| B2I081-01 | C27H33N5O5S | 540.2275 | 0.0 | 0.0 |
| B2I082-01 | C26H32N6O5S | 541.2228 | 0.0 | 1.9 |
| B2I083-01 | C28H35N5O5S | 554.2432 | 0.0 | 0.0 |
| B2I084-01 | C27H34N6O5S | 555.2384 | -0.9 | -1.5 |
| B2I085-01 | C27H33N5O6S | 556.2224 | 0.0 | 0.0 |
| B2I086-01 | C26H32N6O6S | 557.2177 | 0.0 | 0.0 |
| B2I087-01 | C26H28F3N5O4S | 564.1887 | 1.3 | -0.9 |
| B2I088-01 | C25H27F3N6O4S | 565.1839 | 0.0 | 0.0 |
| B2I089-01 | C26H28F3N5O5S | 580.1836 | 1.4 | 0.0 |
| B2I090-01 | C25H27F3N6O5S | 581.1789 | 0.0 | 0.0 |
| B2I091-01 | C29H31N5O4S | 546.217 | na | na |
| B2I092-01 | C28H30N6O4S | 547.2122 | 2.6 | 0.3 |
| B2I093-01 | C23H27N5O4S2 | 502.1577 | na | na |
| B2I094-01 | C22H26N6O4S2 | 503.153 | 0.0 | 0.0 |
| B2I095-01 | C27H29N5O4S | 520.2013 | 0.0 | 0.0 |
| B2I096-01 | C26H28N6O4S | 521.1966 | 0.0 | 0.0 |
| B2I097-01 | C28H33N3O4S | 508.2265 | 0.8 | 1.4 |
| B2I098-01 | C27H32N4O4S | 509.2217 | 0.0 | 0.0 |
| B2I099-01 | C28H32FN3O4S | 526.217 | na | 0.0 |
| B2I100-01 | C27H31FN4O4S | 527.2123 | 0.0 | 0.0 |
| B2L001-01 | C26H31N5O5S2 | 558.1839 | -67.5 | 0.0 |
| B2L002-01 | C25H30N6O5S2 | 559.1792 | 0.0 | 0.0 |
| B2L003-01 | C29H35N5O5S | 566.2432 | 0.0 | 0.0 |
| B2L004-01 | C28H34N6O5S | 567.2384 | 5.5 | 9.3 |
| B2L005-01 | C28H32FN5O5S | 570.2181 | 0.0 | 0.0 |
| B2L006-01 | C27H31FN6O5S | 571.2133 | 0.0 | 0.0 |
| B2L007-01 | C30H33N5O5S | 576.2275 | 0.0 | -1.0 |
| B2L008-01 | C29H32N6O5S | 577.2228 | 0.0 | 0.0 |
| B2L009-01 | C29H32N6O5S | 577.2228 | 0.0 | 0.0 |
| B2L010-01 | C28H31N7O5S | 578.218 | 0.0 | 0.0 |
| B2L011-01 | C30H37N5O5S | 580.2588 | 0.0 | 0.0 |
| B2L012-01 | C29H36N6O5S | 581.2541 | 4.3 | 7.3 |
| B2L013-01 | C32H35N5O5S | 602.2432 | 0.0 | 0.0 |
| B2L014-01 | C31H34N6O5S | 603.2384 | 0.3 | 0.5 |
| B2L015-01 | C32H41N5O5S | 608.2901 | 0.0 | 0.0 |
| B2L016-01 | C31H40N6O5S | 609.2854 | 0.0 | -5.4 |
| B2L017-01 | C29H32F3N5O5S | 620.2149 | 0.0 | 0.0 |
| B2L018-01 | C28H31F3N6O5S | 621.2102 | -1.9 | -2.5 |
| B2L019-01 | C31H32F3N5O5S | 644.2149 | na | na |
| B2L020-01 | C30H31F3N6O5S | 645.2102 | 0.0 | 0.0 |
| B2L021-01 | C32H34F3N7O5S | 686.2367 | 0.0 | -14.9 |
| B2L022-01 | C31H33F3N8O5S | 687.232 | 0.0 | 0.0 |
| B2L023-01 | C28H33N3O5S2 | 556.1934 | 0.0 | 0.0 |
| B2L024-01 | C27H32N4O5S2 | 557.1887 | 0.0 | 0.0 |
| B2L025-01 | C31H37N3O5S | 564.2527 | 0.0 | 0.0 |
| B2L026-01 | C30H36N4O5S | 565.2479 | 0.0 | 0.0 |
| B2L027-01 | C30H34FN3O5S | 568.2276 | 0.0 | 0.0 |
| B2L028-01 | C29H33FN4O5S | 569.2229 | 0.0 | 0.0 |
| B2L029-01 | C32H35N3O5S | 574.237 | 0.0 | na |
| B2L030-01 | C31H34N4O5S | 575.2323 | 0.0 | 0.0 |
| B2L031-01 | C31H34N4O5S | 575.2323 | 0.0 | 0.0 |
| B2L032-01 | C30H33N5O5S | 576.2275 | 0.0 | -1.0 |
| B2L033-01 | C32H39N3O5S | 578.2683 | 0.0 | 0.0 |
| B2L034-01 | C31H38N4O5S | 579.2636 | 1.6 | 3.5 |
| B2L035-01 | C34H37N3O5S | 600.2527 | 0.0 | na |
| B2L036-01 | C33H36N4O5S | 601.2479 | 0.0 | 0.0 |
| B2L037-01 | C34H43N3O5S | 606.2996 | 0.0 | 0.0 |
| B2L038-01 | C33H42N4O5S | 607.2949 | 0.0 | 0.0 |
| B2L039-01 | C31H34F3N3O5S | 618.2244 | 0.0 | 0.0 |
| B2L040-01 | C30H33F3N4O5S | 619.2197 | -0.2 | 1.0 |
| B2L041-01 | C33H34F3N3O5S | 642.2244 | 0.0 | na |
| B2L042-01 | C32H33F3N4O5S | 643.2197 | 0.0 | 0.0 |
| B2L043-01 | C34H36F3N5O5S | 684.2462 | 0.0 | 0.0 |
| B2L044-01 | C33H35F3N6O5S | 685.2415 | 0.0 | 0.0 |
| B2L045-01 | C30H37N3O4S | 536.2578 | 0.0 | 0.0 |
| B2L046-01 | C29H36N4O4S | 537.253 | 0.0 | 0.0 |
| B2L047-01 | C31H39N3O4S | 550.2734 | 0.0 | 0.0 |
| B2L048-01 | C30H38N4O4S | 551.2687 | 3.9 | -5.0 |
| B2L049-01 | C32H41N3O4S | 564.2891 | na | 0.0 |
| B2L050-01 | C31H40N4O4S | 565.2843 | 0.0 | 0.0 |

| Compound No. | Molecular formula | m/z (expected) | ASMS ratio(%) | |
|---|---|---|---|---|
| | | | n=1 | n=2 |
| B2L051-01 | C30H36FN3O4S | 554.2483 | 0.0 | 0.0 |
| B2L052-01 | C29H35FN4O4S | 555.2436 | 2.7 | 1.0 |
| B2L053-01 | C31H39N3O5S | 566.2683 | 0.0 | 0.0 |
| B2L054-01 | C30H38N4O5S | 567.2636 | 0.0 | 0.0 |
| B2L055-01 | C32H41N3O5S | 580.284 | 0.0 | 0.0 |
| B2L056-01 | C31H40N4O5S | 581.2792 | 0.0 | 0.0 |
| B2L057-01 | C33H43N3O5S | 594.2996 | 0.0 | 0.0 |
| B2L058-01 | C32H42N4O5S | 595.2949 | 0.0 | 0.0 |
| B2L059-01 | C32H41N3O6S | 596.2789 | 0.0 | 0.0 |
| B2L060-01 | C31H40N4O6S | 597.2741 | na | 0.0 |
| B2L061-01 | C31H36F3N3O4S | 604.2451 | 4.6 | -11.1 |
| B2L062-01 | C30H35F3N4O4S | 605.2404 | 0.0 | 0.0 |
| B2L063-01 | C31H36F3N3O5S | 620.2401 | 0.0 | 0.0 |
| B2L064-01 | C30H35F3N4O5S | 621.2353 | 0.0 | 0.0 |
| B2L065-01 | C34H39N3O4S | 586.2734 | na | na |

| Compound No. | Molecular formula | m/z (expected) | ASMS ratio(%) | |
|---|---|---|---|---|
| | | | n=1 | n=2 |
| B2L066-01 | C33H38N4O4S | 587.2687 | 0.0 | 0.0 |
| B2L067-01 | C28H35N3O4S2 | 542.2142 | 0.0 | 0.0 |
| B2L068-01 | C27H34N4O4S2 | 543.2094 | 0.0 | 0.0 |
| B2L069-01 | C32H37N3O4S | 560.2578 | 0.0 | 0.0 |
| B2L070-01 | C31H36N4O4S | 561.253 | 4.9 | -4.2 |
| B2L071-01 | C28H35N5O4S | 538.2483 | 1.2 | 0.0 |
| B2L072-01 | C27H34N6O4S | 539.2435 | -10.7 | 8.3 |
| B2L073-01 | C29H37N5O4S | 552.2639 | 0.0 | 0.0 |
| B2L074-01 | C28H36N6O4S | 553.2592 | -1.0 | 1.4 |
| B2L075-01 | C30H39N5O4S | 566.2796 | 0.0 | 33.9 |
| B2L076-01 | C29H38N6O4S | 567.2748 | 1.8 | -0.3 |
| B2L077-01 | C28H34FN5O4S | 556.2388 | 0.0 | 0.0 |
| B2L078-01 | C27H33FN6O4S | 557.2341 | 1.1 | -0.7 |
| B2L079-01 | C29H37N5O5S | 568.2588 | 0.0 | 0.0 |
| B2L080-01 | C28H36N6O5S | 569.2541 | 0.0 | 0.0 |

na = not available

[Table 284]

| Compound No. | Molecular formula | m/z (expected) | ASMS ratio(%) | |
|---|---|---|---|---|
| | | | n=1 | n=2 |
| B2L081-01 | C30H39N5O5S | 582.2745 | 0.0 | 0.0 |
| B2L082-01 | C29H38N6O5S | 583.2697 | 2.6 | -1.2 |
| B2L083-01 | C31H41N5O5S | 596.2901 | 0.0 | 0.0 |
| B2L084-01 | C30H40N6O5S | 597.2854 | -3.0 | -1.9 |
| B2L085-01 | C30H39N5O6S | 598.2694 | 0.0 | 0.0 |
| B2L086-01 | C29H38N6O6S | 599.2646 | na | 0.0 |
| B2L087-01 | C29H34F3N5O4S | 606.2356 | 1.9 | -24.6 |
| B2L088-01 | C28H33F3N6O4S | 607.2309 | 0.0 | 2.8 |
| B2L089-01 | C29H34F3N5O5S | 622.2306 | 0.0 | 0.0 |
| B2L090-01 | C28H33F3N6O5S | 623.2258 | 0.0 | 0.0 |
| B2L091-01 | C32H37N5O4S | 588.2639 | 0.0 | 0.0 |
| B2L092-01 | C31H36N6O4S | 589.2592 | 5.5 | 7.8 |
| B2L093-01 | C26H33N5O4S2 | 544.2047 | na | 0.0 |
| B2L094-01 | C25H32N6O4S2 | 545.1999 | 0.2 | 1.8 |
| B2L095-01 | C30H35N5O4S | 562.2483 | 0.0 | 0.0 |
| B2L096-01 | C29H34N6O4S | 563.2435 | 0.0 | 0.0 |
| B2L097-01 | C31H39N3O4S | 550.2734 | 0.0 | 0.0 |
| B2L098-01 | C30H38N4O4S | 551.2687 | 3.9 | -5.0 |
| B2L099-01 | C31H38FN3O4S | 568.264 | na | na |
| B2L100-01 | C30H37FN4O4S | 569.2592 | 0.0 | 0.0 |
| B2N001-01 | C25H26F3N5O5S2 | 598.14 | na | 0.0 |
| B2N002-01 | C24H25F3N6O5S2 | 599.1353 | 0.0 | 0.0 |
| B2N003-01 | C28H30F3N5O5S | 606.1993 | 0.0 | 0.0 |
| B2N004-01 | C27H29F3N6O5S | 607.1945 | 1.2 | -2.0 |
| B2N005-01 | C27H27F4N5O5S | 610.1742 | na | na |
| B2N006-01 | C26H26F4N6O5S | 611.1694 | na | na |
| B2N007-01 | C29H28F3N5O5S | 616.1836 | 0.0 | 0.0 |
| B2N008-01 | C28H27F3N6O5S | 617.1789 | 0.0 | -0.4 |
| B2N009-01 | C28H27F3N6O5S | 617.1789 | 0.0 | -0.4 |
| B2N010-01 | C27H26F3N7O5S | 618.1741 | 0.0 | 0.0 |
| B2N011-01 | C29H32F3N5O5S | 620.2149 | 0.0 | 0.0 |
| B2N012-01 | C28H31F3N6O5S | 621.2102 | 0.0 | 0.0 |
| B2N013-01 | C31H30F3N5O5S | 642.1993 | 0.0 | 0.0 |
| B2N014-01 | C30H29F3N6O5S | 643.1945 | 0.0 | 0.0 |
| B2N015-01 | C31H36F3N5O5S | 648.2462 | na | na |
| B2N016-01 | C30H35F3N6O5S | 649.2415 | 0.0 | 0.0 |
| B2N017-01 | C28H27F6N5O5S | 660.171 | na | na |
| B2N018-01 | C27H26F6N6O5S | 661.1662 | 0.0 | 1.0 |

| Compound No. | Molecular formula | m/z (expected) | ASMS ratio(%) | |
|---|---|---|---|---|
| | | | n=1 | n=2 |
| B2N019-01 | C30H27F6N5O5S | 684.171 | na | na |
| B2N020-01 | C29H26F6N6O5S | 685.1662 | 0.0 | 0.0 |
| B2N021-01 | C31H29F6N7O5S | 726.1928 | na | 0.0 |
| B2N022-01 | C30H28F6N8O5S | 727.188 | 0.0 | 0.0 |
| B2N023-01 | C27H28F3N3O5S2 | 596.1495 | 0.0 | 0.0 |
| B2N024-01 | C26H27F3N4O5S2 | 597.1448 | 0.0 | 0.0 |
| B2N025-01 | C30H32F3N3O5S | 604.2088 | 0.0 | 0.0 |
| B2N026-01 | C29H31F3N4O5S | 605.204 | -1.5 | -0.7 |
| B2N027-01 | C29H29F4N3O5S | 608.1837 | na | na |
| B2N028-01 | C28H28F4N4O5S | 609.1789 | -0.7 | -0.9 |
| B2N029-01 | C31H30F3N3O5S | 614.1931 | na | na |
| B2N030-01 | C30H29F3N4O5S | 615.1884 | 0.0 | 0.0 |
| B2N031-01 | C30H29F3N4O5S | 615.1884 | 0.0 | 0.0 |
| B2N032-01 | C29H28F3N5O5S | 616.1836 | -0.4 | -1.0 |
| B2N033-01 | C31H34F3N3O5S | 618.2244 | 0.0 | 0.0 |
| B2N034-01 | C30H33F3N4O5S | 619.2197 | -0.2 | 1.0 |
| B2N035-01 | C33H32F3N3O5S | 640.2088 | 0.0 | 0.0 |
| B2N036-01 | C32H31F3N4O5S | 641.204 | 0.0 | 0.4 |
| B2N037-01 | C33H38F3N3O5S | 646.2557 | 0.0 | 0.0 |
| B2N038-01 | C32H37F3N4O5S | 647.251 | 0.0 | 0.0 |
| B2N039-01 | C30H29F6N3O5S | 658.1805 | na | na |
| B2N040-01 | C29H28F6N4O5S | 659.1757 | 0.0 | 0.0 |
| B2N041-01 | C32H29F6N3O5S | 682.1805 | na | na |
| B2N042-01 | C31H28F6N4O5S | 683.1757 | 0.0 | 0.0 |
| B2N043-01 | C33H31F6N5O5S | 724.2023 | -1.4 | 0.1 |
| B2N044-01 | C32H30F6N6O5S | 725.1975 | 0.2 | 1.4 |
| B2N045-01 | C29H32F3N3O4S | 576.2138 | 0.0 | 0.1 |
| B2N046-01 | C28H31F3N4O4S | 577.2091 | 0.0 | -1.2 |
| B2N047-01 | C30H34F3N3O4S | 590.2295 | 0.0 | 0.0 |
| B2N048-01 | C29H33F3N4O4S | 591.2247 | 0.0 | -0.7 |
| B2N049-01 | C31H36F3N3O4S | 604.2451 | 0.0 | 0.0 |
| B2N050-01 | C30H35F3N4O4S | 605.2404 | 0.0 | na |
| B2N051-01 | C29H31F4N3O4S | 594.2044 | 0.0 | 0.0 |
| B2N052-01 | C28H30F4N4O4S | 595.1997 | 0.0 | -0.5 |
| B2N053-01 | C30H34F3N3O5S | 606.2244 | 0.0 | 1.4 |
| B2N054-01 | C29H33F3N4O5S | 607.2197 | 0.0 | 0.0 |
| B2N055-01 | C31H36F3N3O5S | 620.2401 | 0.0 | 0.0 |
| B2N056-01 | C30H35F3N4O5S | 621.2353 | 0.0 | 0.0 |

| Compound No. | Molecular formula | m/z (expected) | ASMS ratio(%) | |
|---|---|---|---|---|
| | | | n=1 | n=2 |
| B2N057-01 | C32H38F3N3O5S | 634.2557 | 0.0 | -1.8 |
| B2N058-01 | C31H37F3N4O5S | 635.251 | 0.0 | 0.0 |
| B2N059-01 | C31H36F3N3O6S | 636.235 | 0.0 | 0.0 |
| B2N060-01 | C30H35F3N4O6S | 637.2302 | 0.0 | 0.0 |
| B2N061-01 | C30H31F6N3O4S | 644.2012 | 0.0 | 0.0 |
| B2N062-01 | C29H30F6N4O4S | 645.1965 | 0.0 | 0.0 |
| B2N063-01 | C30H31F6N3O5S | 660.1961 | -0.3 | -3.8 |
| B2N064-01 | C29H30F6N4O5S | 661.1914 | 0.0 | 0.0 |
| B2N065-01 | C33H34F3N3O4S | 626.2295 | 0.0 | 0.0 |
| B2N066-01 | C32H33F3N4O4S | 627.2247 | 0.0 | 0.0 |
| B2N067-01 | C27H30F3N3O4S2 | 582.1703 | 0.0 | 0.0 |
| B2N068-01 | C26H29F3N4O4S2 | 583.1655 | 0.0 | 0.5 |
| B2N069-01 | C31H32F3N3O4S | 600.2138 | -1.8 | -1.5 |
| B2N070-01 | C30H31F3N4O4S | 601.2091 | 0.0 | 0.0 |
| B2N071-01 | C27H30F3N5O4S | 578.2043 | 0.0 | 0.0 |
| B2N072-01 | C26H29F3N6O4S | 579.1996 | 0.0 | 0.1 |
| B2N073-01 | C28H32F3N5O4S | 592.22 | 0.0 | 0.0 |
| B2N074-01 | C27H31F3N6O4S | 593.2152 | 0.0 | 0.0 |
| B2N075-01 | C29H34F3N5O4S | 606.2356 | na | na |
| B2N076-01 | C28H33F3N6O4S | 607.2309 | 0.0 | 0.0 |
| B2N077-01 | C27H29F4N5O4S | 596.1949 | 0.0 | 0.0 |
| B2N078-01 | C26H28F4N6O4S | 597.1902 | 0.0 | 0.0 |
| B2N079-01 | C28H32F3N5O5S | 608.2149 | 0.0 | 0.0 |
| B2N080-01 | C27H31F3N6O5S | 609.2102 | 0.0 | 0.0 |
| B2N081-01 | C29H34F3N5O5S | 622.2306 | 0.0 | 0.0 |
| B2N082-01 | C28H33F3N6O5S | 623.2258 | 2.5 | 2.8 |
| B2N083-01 | C30H36F3N5O5S | 636.2462 | 0.0 | 0.0 |
| B2N084-01 | C29H35F3N6O5S | 637.2415 | 0.0 | 0.0 |
| B2N085-01 | C29H34F3N5O6S | 638.2255 | 0.0 | 0.0 |
| B2N086-01 | C28H33F3N6O6S | 639.2207 | 0.0 | 0.0 |
| B2N087-01 | C28H29F6N5O4S | 646.1917 | 0.0 | 0.0 |
| B2N088-01 | C27H28F6N6O4S | 647.187 | 0.0 | 0.0 |
| B2N089-01 | C28H29F6N5O5S | 662.1866 | na | 0.0 |

| Compound No. | Molecular formula | m/z (expected) | ASMS ratio(%) | |
|---|---|---|---|---|
| | | | n=1 | n=2 |
| B2N090-01 | C27H28F6N6O5S | 663.1819 | 0.0 | 0.0 |
| B2N091-01 | C31H32F3N5O4S | 628.22 | 0.0 | 0.0 |
| B2N092-01 | C30H31F3N6O4S | 629.2152 | 0.0 | 0.0 |
| B2N093-01 | C25H28F3N5O4S2 | 584.1608 | 0.0 | na |
| B2N094-01 | C24H27F3N6O4S2 | 585.156 | 0.0 | 0.0 |
| B2N095-01 | C29H30F3N5O4S | 602.2043 | 0.0 | 0.0 |
| B2N096-01 | C28H29F3N6O4S | 603.1996 | na | na |
| B2N097-01 | C30H34F3N3O4S | 590.2295 | 0.0 | 0.0 |
| B2N098-01 | C29H33F3N4O4S | 591.2247 | 0.0 | -0.7 |
| B2N099-01 | C30H33F4N3O4S | 608.2201 | 0.0 | 0.0 |
| B2N100-01 | C29H32F4N4O4S | 609.2153 | 0.0 | 0.0 |
| B2R001-01 | C37H35F3N6O5S3 | 797.1856 | na | na |
| B2R002-01 | C36H34F3N7O5S3 | 798.1808 | 0.0 | 0.0 |
| B2R003-01 | C40H39F3N6O5S2 | 805.2448 | 21.6 | 0.0 |
| B2R004-01 | C39H38F3N7O5S2 | 806.2401 | 0.0 | 0.0 |
| B2R005-01 | C39H36F4N6O5S2 | 809.2198 | 44.0 | 39.5 |
| B2R006-01 | C38H35F4N7O5S2 | 810.215 | na | na |
| B2R007-01 | C41H37F3N6O5S2 | 815.2292 | 0.0 | na |
| B2R008-01 | C40H36F3N7O5S2 | 816.2244 | 0.0 | 19.5 |
| B2R009-01 | C40H36F3N7O5S2 | 816.2244 | 0.0 | 19.5 |
| B2R010-01 | C39H35F3N8O5S2 | 817.2197 | 0.0 | 0.0 |
| B2R011-01 | C41H41F3N6O5S2 | 819.2605 | 17.7 | -1.3 |
| B2R012-01 | C40H40F3N7O5S2 | 820.2557 | 15.8 | 13.5 |
| B2R013-01 | C43H39F3N6O5S2 | 841.2448 | 7.5 | 7.7 |
| B2R014-01 | C42H38F3N7O5S2 | 842.2401 | 18.6 | 15.1 |
| B2R015-01 | C43H45F3N6O5S2 | 847.2918 | 9.7 | 0.0 |
| B2R016-01 | C42H44F3N7O5S2 | 848.287 | 9.1 | 2.7 |
| B2R017-01 | C40H36F6N6O5S2 | 859.2166 | 32.9 | 27.2 |
| B2R018-01 | C39H35F6N7O5S2 | 860.2118 | 5.1 | 7.8 |
| B2R019-01 | C42H36F6N6O5S2 | 883.2166 | 5.2 | 14.0 |
| B2R020-01 | C41H35F6N7O5S2 | 884.2118 | 10.8 | -17.7 |

na = not available

[Table 285]

| Compound No. | Molecular formula | m/z (expected) | ASMS ratio(%) | |
|---|---|---|---|---|
| | | | n=1 | n=2 |
| B2R021-01 | C43H38F6N8O5S2 | 925.2384 | 13.0 | 0.0 |
| B2R022-01 | C42H37F6N9O5S2 | 926.2336 | 14.5 | 15.8 |
| B2R023-01 | C39H37F3N4O5S3 | 795.1951 | 0.0 | -15.5 |
| B2R024-01 | C38H36F3N5O5S3 | 796.1903 | 0.0 | -1.2 |
| B2R025-01 | C42H41F3N4O5S2 | 803.2543 | -1.0 | -2.4 |
| B2R026-01 | C41H40F3N5O5S2 | 804.2496 | 1.7 | -4.6 |
| B2R027-01 | C41H38F4N4O5S2 | 807.2293 | -3.7 | 1.2 |
| B2R028-01 | C40H37F4N5O5S2 | 808.2245 | 4.1 | -0.5 |
| B2R029-01 | C43H39F3N4O5S2 | 813.2387 | na | 0.0 |
| B2R030-01 | C42H38F3N5O5S2 | 814.2339 | 0.0 | 4.8 |
| B2R031-01 | C42H38F3N5O5S2 | 814.2339 | 0.0 | 4.8 |
| B2R032-01 | C41H37F3N6O5S2 | 815.2292 | 6.4 | 0.0 |
| B2R033-01 | C43H43F3N4O5S2 | 817.27 | 3.6 | -6.6 |
| B2R034-01 | C42H42F3N5O5S2 | 818.2652 | 0.1 | -1.3 |
| B2R035-01 | C45H41F3N4O5S2 | 839.2543 | 8.3 | 0.9 |
| B2R036-01 | C44H40F3N5O5S2 | 840.2496 | 3.2 | -0.8 |
| B2R037-01 | C45H47F3N4O5S2 | 845.3013 | 6.3 | -7.0 |
| B2R038-01 | C44H46F3N5O5S2 | 846.2965 | 7.1 | -6.0 |
| B2R039-01 | C42H38F6N4O5S2 | 857.2261 | 9.7 | -4.8 |
| B2R040-01 | C41H37F6N5O5S2 | 858.2213 | 2.6 | 0.2 |

| Compound No. | Molecular formula | m/z (expected) | ASMS ratio(%) | |
|---|---|---|---|---|
| | | | n=1 | n=2 |
| B2R041-01 | C44H38F6N4O5S2 | 881.2261 | 18.0 | 0.0 |
| B2R042-01 | C43H37F6N5O5S2 | 882.2213 | 3.4 | 6.0 |
| B2R043-01 | C45H40F6N6O5S2 | 923.2479 | 3.4 | -9.0 |
| B2R044-01 | C44H39F6N7O5S2 | 924.2431 | -3.7 | -4.1 |
| B2R045-01 | C41H41F3N4O4S2 | 775.2594 | 4.8 | -3.4 |
| B2R046-01 | C40H40F3N5O4S2 | 776.2547 | -0.2 | 0.0 |
| B2R047-01 | C42H43F3N4O4S2 | 789.2751 | 6.6 | -7.8 |
| B2R048-01 | C41H42F3N5O4S2 | 790.2703 | 9.2 | 7.5 |
| B2R049-01 | C43H45F3N4O4S2 | 803.2907 | 7.5 | -5.5 |
| B2R050-01 | C42H44F3N5O4S2 | 804.286 | 0.0 | 0.0 |
| B2R051-01 | C41H40F4N4O4S2 | 793.25 | 12.7 | -1.8 |
| B2R052-01 | C40H39F4N5O4S2 | 794.2452 | 0.0 | -7.4 |
| B2R053-01 | C42H43F3N4O5S2 | 805.27 | -15.0 | 0.0 |
| B2R054-01 | C41H42F3N5O5S2 | 806.2652 | 3.3 | -0.8 |
| B2R055-01 | C43H45F3N4O5S2 | 819.2856 | -3.3 | 6.3 |
| B2R056-01 | C42H44F3N5O5S2 | 820.2809 | na | -63.9 |
| B2R057-01 | C44H47F3N4O5S2 | 833.3013 | 5.7 | -5.1 |
| B2R058-01 | C43H46F3N5O5S2 | 834.2965 | 13.0 | 4.8 |
| B2R059-01 | C43H45F3N4O6S2 | 835.2805 | 0.0 | 0.0 |
| B2R060-01 | C42H44F3N5O6S2 | 836.2758 | 3.9 | 0.0 |

| Compound No. | Molecular formula | m/z (expected) | ASMS ratio(%) | |
|---|---|---|---|---|
| | | | n=1 | n=2 |
| B2R061-01 | C42H40F6N4O4S2 | 843.2468 | 9.1 | -6.0 |
| B2R062-01 | C41H39F6N5O4S2 | 844.242 | 0.0 | 0.0 |
| B2R063-01 | C42H40F6N4O5S2 | 859.2417 | 8.2 | 4.6 |
| B2R064-01 | C41H39F6N5O5S2 | 860.237 | 4.2 | -7.4 |
| B2R065-01 | C45H43F3N4O4S2 | 825.2751 | 3.8 | 0.3 |
| B2R066-01 | C44H42F3N5O4S2 | 826.2703 | 19.8 | -10.7 |
| B2R067-01 | C39H39F3N4O4S3 | 781.2158 | 8.0 | 0.0 |
| B2R068-01 | C38H38F3N5O4S3 | 782.2111 | 0.0 | 0.0 |
| B2R069-01 | C43H41F3N4O4S2 | 799.2594 | 12.3 | -18.5 |
| B2R070-01 | C42H40F3N5O4S2 | 800.2547 | na | na |
| B2R071-01 | C39H39F3N6O4S2 | 777.2499 | 14.3 | -9.0 |
| B2R072-01 | C38H38F3N7O4S2 | 778.2452 | 21.4 | 11.4 |
| B2R073-01 | C40H41F3N6O4S2 | 791.2656 | 24.0 | 0.0 |
| B2R074-01 | C39H40F3N7O4S2 | 792.2608 | 22.4 | 25.4 |
| B2R075-01 | C41H43F3N6O4S2 | 805.2812 | 8.6 | 0.0 |
| B2R076-01 | C40H42F3N7O4S2 | 806.2765 | 3.9 | 19.7 |
| B2R077-01 | C39H38F4N6O4S2 | 795.2405 | 0.0 | 0.0 |
| B2R078-01 | C38H37F4N7O4S2 | 796.2357 | 15.8 | 11.7 |
| B2R079-01 | C40H41F3N6O5S2 | 807.2605 | na | na |
| B2R080-01 | C39H40F3N7O5S2 | 808.2557 | 16.7 | 21.2 |

| Compound No. | Molecular formula | m/z (expected) | ASMS ratio(%) | |
|---|---|---|---|---|
| | | | n=1 | n=2 |
| B2R081-01 | C41H43F3N6O5S2 | 821.2761 | 21.1 | -8.8 |
| B2R082-01 | C40H42F3N7O5S2 | 822.2714 | 0.0 | 0.0 |
| B2R083-01 | C42H45F3N6O5S2 | 835.2918 | 31.9 | -11.1 |
| B2R084-01 | C41H44F3N7O5S2 | 836.287 | na | na |
| B2R085-01 | C41H43F3N6O6S2 | 837.271 | na | na |
| B2R086-01 | C40H42F3N7O6S2 | 838.2663 | 0.0 | 0.0 |
| B2R087-01 | C40H38F6N6O4S2 | 845.2373 | 3.8 | 7.1 |
| B2R088-01 | C39H37F6N7O4S2 | 846.2325 | 35.3 | 13.1 |
| B2R089-01 | C40H38F6N6O5S2 | 861.2322 | 3.4 | -9.2 |
| B2R090-01 | C39H37F6N7O5S2 | 862.2275 | 22.4 | 5.3 |
| B2R091-01 | C43H41F3N6O4S2 | 827.2656 | -3.5 | 1.8 |
| B2R092-01 | C42H40F3N7O4S2 | 828.2608 | 16.0 | -1.3 |
| B2R093-01 | C37H37F3N6O4S3 | 783.2063 | 56.5 | 0.0 |
| B2R094-01 | C36H36F3N7O4S3 | 784.2016 | 18.8 | 11.9 |
| B2R095-01 | C41H39F3N6O4S2 | 801.2499 | 42.2 | 5.5 |
| B2R096-01 | C40H38F3N7O4S2 | 802.2452 | na | na |
| B2R097-01 | C42H43F3N4O4S2 | 789.2751 | 6.6 | -7.8 |
| B2R098-01 | C41H42F3N5O4S2 | 790.2703 | 9.2 | 7.5 |
| B2R099-01 | C42H42F4N4O4S2 | 807.2656 | -12.6 | -7.5 |
| B2R100-01 | C41H41F4N5O4S2 | 808.2609 | 0.6 | 10.1 |

na = not available

Binding to Bcl-2 was detected as to two compounds, B2Q045-01 (49.9%, 49.9%) and B2Q046-01 (20.3%, 20.5%), which were involved as positive controls, as well as a plurality of compounds. Of these compounds, 11 compounds, B2Q047-01 (142.5%, 326.4%), B2Q097-01 (142.5%, 326.4%), B2Q048-01 (61.6%, 90.8%), B2Q098-01 (61.6%, 90.8%), B2Q053-01 (22.4%, 15.5%), B2Q054-01 (44.1%, 65.0%), B2Q066-01 (40.7%, 36.9%), B1A075-01 (31.3%, 28.6%), B3A073-01 (31.3%, 28.6%), B2R074-01 (22.4%, 25.4%), and B2Q012-01 (21.4%, 22.5%), were individually synthesized separately (described in Example 6), and a binding confirmation assay was carried out by SPR in Example 7.

Example 6; Individual synthesis of Bcl-2 binding compounds identified by AS-MS experiment

Example 6-1-1: Synthesis of 4-[4-[[4-(3-ethoxyphenyl)-3- methylphenyl]methyl]piperazin-1 -yl]-N-[3-nitro-4-(2- phenylsulfanylethylamino)phenyl]sulfonylbenzamide [AG016 (B2Q047-01)]

[Formula 549]

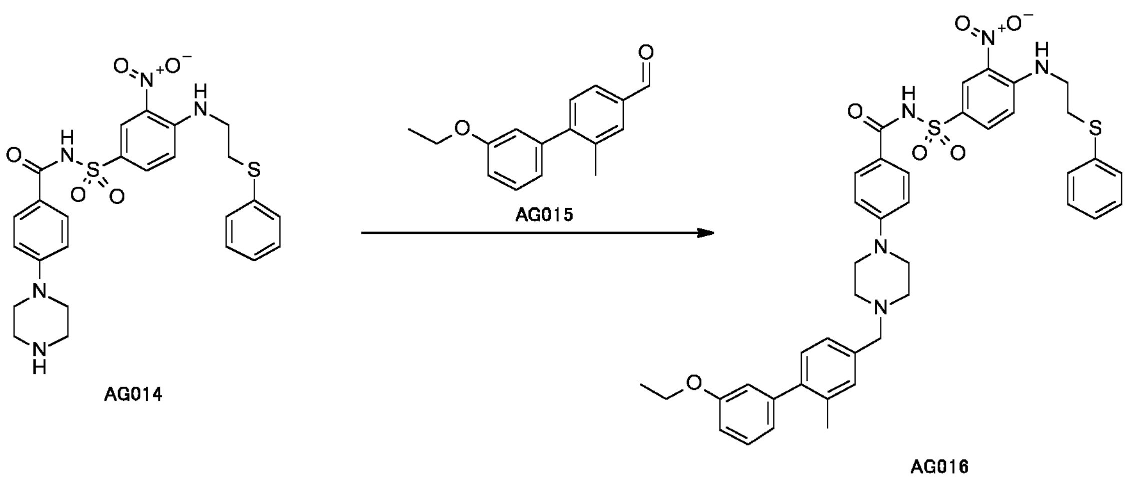

AG014

AG015

AG016

A solution of N-[3-nitro-4-(2-phenylsulfanylethylamino)phenyl]sulfonyl-4- piperazin-1-ylbenzamide (AG014, CAS: 406233-75-8) (150.00 mg, 0.277 mmol), 4-(3- ethoxyphenyl)-3-methylbenzaldehyde (AG015, CAS: 1506205-91-9) (79.86 mg, 0.332 mmol), and acetic acid (0.20 mL, 3.490 mmol) in DMSO/ethanol = 1/1 (3.00 mL) was stirred at room temperature for 2 hours. Sodium cyanoborohydride (34.81 mg, 0.554 mmol) was added thereto, and the mixture was stirred at room temperature for 2 hours. The mixture was concentrated. Then, the obtained residue was purified by reverse-phase column chromatography (C18, acetonitrile/10 mM aqueous ammonium bicarbonate solution, 5-75%). The obtained crude product was purified by preparative HPLC (Xselect CSH OBD column, acetonitrile/10 mM aqueous ammonium bicarbonate solution, 49-59%) to obtain the title compound AG016 (50 mg, 23.1%) as a yellow solid.

$^1$H-NMR (300 MHz, Chloroform-d) δ 8.88 (s, 1H), 8.67 (s, 1H), 8.15 (d, J = 9.0 Hz, 1H), 7.69 (d, J = 8.4 Hz, 2H), 7.45-7.30 (m, 5H), 7.24 (d, J = 12.9 Hz, 4H), 6.92-6.80 (m, 5H), 4.08 (q, J = 7.2 Hz, 2H), 3.66-3.56 (m, 4H), 3.38-3.20 (m, 4H), 3.21 (s, 2H), 2.71 (s, 4H), 2.29 (s, 3H), 1.45 (t, J = 6.9 Hz, 3H).

LRMS (ESI): m/z 766 [M+H]$^+$.

Example 6-2-1: Synthesis of 4-[4-[[4-(5-ethoxypyridin-3-yl)-3- methylphenylmethyl]piperazin-1-yl]-N-[3-nitro-4-(2- phenylsulfanylethylamino)phenyl]sulfonylbenzamide [AG018 (B2Q048-01)]

[Formula 550]

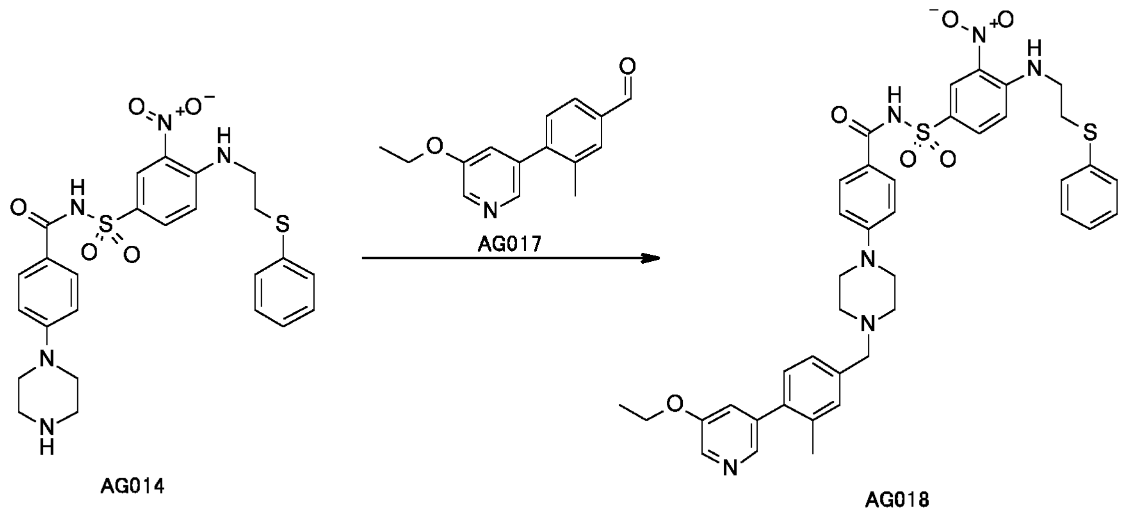


AG014

AG018

A solution of N-[3-nitro-4-(2-phenylsulfanylethylamino)phenyl]sulfonyl-4- piperazin-1-ylbenzamide (AG014, CAS: 406233-75-8) (100.00 mg, 0.185 mmol), 4-(5- ethoxypyridin-3-yl)-3-methylbenzaldehyde (AG017, CAS: 1545014-81-0) (89.10 mg, 0.370 mmol), and acetic acid (0.05 mL, 0.873 mmol) in DMSO (1 mL)/ethanol (1 mL) was stirred at room temperature for 1 hour. Sodium cyanoborohydride (23.20 mg, 0.370 mmol) was added thereto, and the mixture was stirred at room temperature for 16 hours. The mixture was concentrated. Then, the obtained residue was purified by silica gel column chromatography (ethyl acetate/petroleum ether, 0-100%). The obtained crude product was purified by preparative HPLC (XBridge Shield RP18 OBD column, acetonitrile/10 mM ammonium bicarbonate, 42-61% + 5 M aqueous ammonia solution, 0.05%) to obtain the title compound AG018 (64.1 mg, 43.87%) as a yellow solid.

$^1$H-NMR (400 MHz, DMSO-$d_6$) δ 8.68 (s, 1H), 8.55 (d, J = 2.0 Hz, 1H), 8.28 (d, J = 2.8 Hz, 1H), 8.14 (d, J = 2.0 Hz, 1H), 7.91-7.85 (m, 1H), 7.75 (d, J = 8.8 Hz, 2H), 7.42-7.34 (m, 3H), 7.28-7.25 (m, 5H), 7.19-7.09 (m, 1H), 7.11 (d, J = 9.6 Hz, 1H), 6.90 (d, J = 8.8 Hz, 2H), 4.16 (q, J = 8.0 Hz, 2H), 3.68-3.61 (m, 4H), 3.33-3.25 (m, 6H), 2.70-2.51 (m, 4H), 2.26 (s, 3H), 1.32 (t, J = 8.0 Hz, 3H).

LRMS (ESI): m/z 767 [M+H]$^+$.

Example 6-3-1: Synthesis of 4-(5-ethoxypyridin-3-yl)-2-methoxybenzaldehyde (AG021)

[Formula 551]

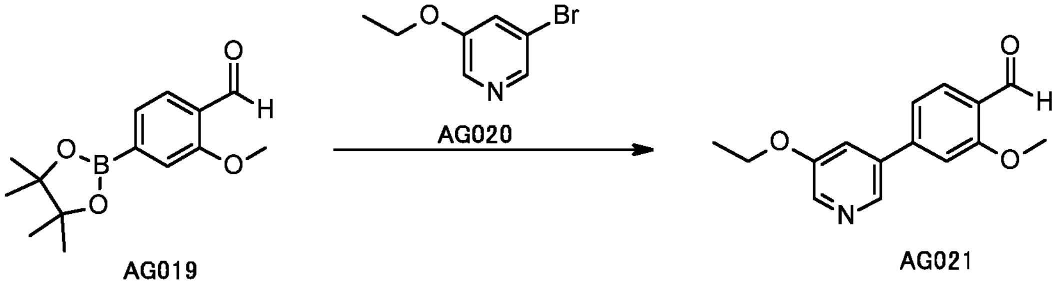


AG019

AG021

Under a nitrogen atmosphere, to a solution of 2-methoxy-4-(4,4,5,5-tetramethyl- 1,3,2-dioxaborolan-2-yl)benzaldehyde (AG019) (300 mg, 1.145 mmol) in dioxane (6 mL)/water (0.6 mL), 3-bromo-5-ethoxypyridine (AG020) (277.5 mg, 1.374 mmol), potassium acetate (224.7 mg, 2.290 mmol), and Pd(dppf)$Cl_2$ (83.8 mg, 0.115 mmol) were added, and the mixture was stirred at 90°C for 3 hours. The mixture was filtered, and the filtrate was concentrated. Then, the obtained residue was purified by silica gel column chromatography (ethyl acetate/petroleum ether, 0-20%) to obtain the title compound AG021 (250 mg, 80.7%) as a white solid.

LRMS (ESI): m/z 258$[M+H]^+$.

Retention time: 0.845 min (analysis conditions PH-TFA05-2).

Example 6-3-2: Synthesis of 4-[4-[[4-(5-ethoxypyridin-3-yl)-2- methoxyphenyl]methyl]piperazin-1-yl]-N-[3-nitro-4-(2-phenylsulfanylethylamino)phenyl]sulfonylbenzamide [AG022 (B2Q054-01)]

[Formula 552]

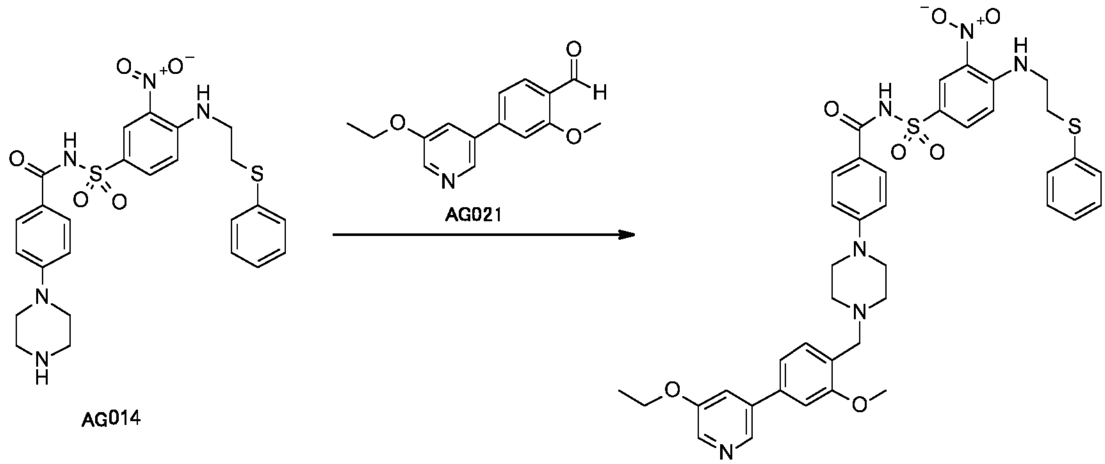


AG014

AG022

A solution of N-[3-nitro-4-(2-phenylsulfanylethylamino)phenyl]sulfonyl-4- piperazin-1-ylbenzamide (AG014, CAS: 406233-75-8) (150 mg, 0.277 mmol), 4-(5- ethoxypyridin-3-yl)-2-methoxybenzaldehyde (AG021) (106.9 mg, 0.416 mmol), and acetic acid (0.20 mL, 3.490 mmol) in DMSO (3 mL)/ethanol (3 mL) was stirred at room temperature for 1 hour. Sodium cyanoborohydride (34.81 mg, 0.554 mmol) was added thereto, and the mixture was stirred at room temperature for 16 hours. The mixture was concentrated. Then, the obtained residue was purified by reverse-phase column chromatography (C18, acetonitrile/water, 0-80%) to obtain the title compound AG022(30 mg, 13.5%) as a white solid.

$^1$H-NMR (300 MHz, DMSO-$d_6$) $\delta$ 11.25 (s, 1H), 8.69 (t, J = 6.0 Hz, 1H), 8.59-8.52 (m, 2H), 8.28 (d, J = 2.7 Hz, 1H), 7.91-7.87 (m, 1H), 7.74 (d, J = 8.7 Hz, 2H), 7.64-7.63 (m, 1H), 7.49-7.46 (m, 1H), 7.39-7.31 (m, 4H), 7.30-7.24 (m, 2H), 7.20-7.09 (m, 2H), 6.91 (d, J = 8.7 Hz, 2H), 4.20 (q, J = 6.9 Hz, 2H), 3.92 (s, 3H), 3.79-3.74 (m, 2H), 3.67-3.60 (m, 2H), 3.39-3.23 (m, 6H), 2.72-2.68 (m, 4H), 1.37 (t, J = 6.9 Hz, 3H).

LRMS (ESI): m/z 783 $[M+H]^+$.

Example 6-4-1: Synthesis of 4-(5-ethoxypyridin-3-yl)naphthalene-1-carbaldehyde (AG024)

[Formula 553]

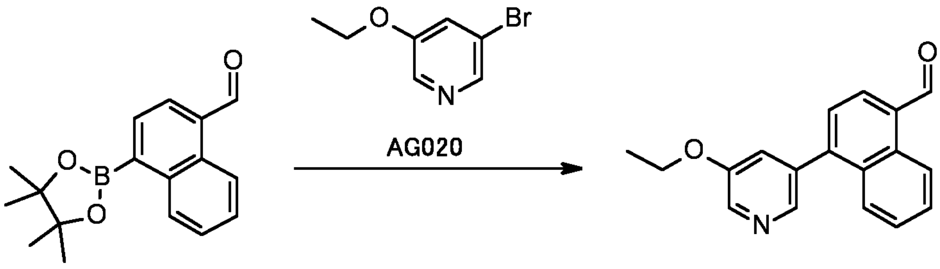


AG024

AG023

Under a nitrogen atmosphere, a solution of 4-(4,4,5,5-tetramethyl-1,3,2- dioxaborolan-2-yl)naphthalene-1-carbaldehyde (AG023) (300 mg, 1.063 mmol), 3-bromo-5- ethoxypyridine (AG020) (179 mg, 0.886 mmol), $Pd(dppf)Cl_2 \cdot DCM$ (72.2 mg, 0.089 mmol), and potassium acetate (173.89 mg, 1.772 mmol) in dioxane (6 mL)/water (0.60 mL) was stirred at 90°C for 2 hours. The mixture was filtered, and the filtrate was concentrated. Then, the obtained residue was purified by silica gel column chromatography (ethyl acetate/petroleum ether, 0-40%) to obtain the title compound AG024 (240 mg, 91%) as a pale yellow liquid.

LRMS (ESI): m/z 278[M+H]$^+$.

Retention time: 0.769 min (analysis conditions PH-FA05-3).

Example 6-4-2: Synthesis of 4-[4-[[4-(5-ethoxypyridin-3-yl)naphthalen-1- yl]methyl]piperazin-1-yl]-N-[3-nitro-4-(2- phenylsulfanylethylamino)phenyl]sulfonylbenzamide [AG025 (B2Q066-01)]

[Formula 554]

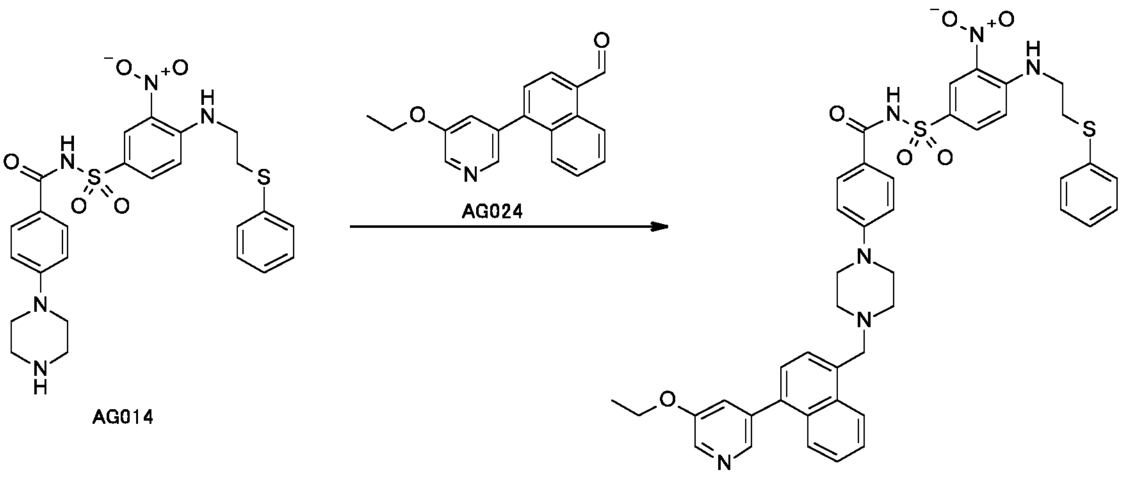


AG025

A solution of N-[3-nitro-4-(2-phenylsulfanylethylamino)phenyl]sulfonyl-4- piperazin-1-ylbenzamide (AG014, CAS: 406233-75-8) (180 mg, 0.332 mmol), 4-(5- ethoxypyridin-3-yl)naphthalene-1-carbaldehyde (AG024) (184 mg, 0.664 mmol), and acetic acid (0.05 mL, 0.873 mmol) in DMSO (2 mL)/ethanol (2 mL) was stirred at room temperature for 1 hour. Sodium cyanoborohydride (41.77 mg, 0.664 mmol) was added thereto, and the mixture was stirred at room temperature for 16 hours. The mixture was concentrated. Then, the obtained residue was purified by silica gel column chromatography (ethyl acetate/petroleum ether, 0-100%, and subsequently methanol/dichloromethane, 0-10%). The obtained crude product was purified by preparative HPLC (XBridge Prep OBD C18 column, acetonitrile/10 mM aqueous ammonium bicarbonate solution, 41-71%) to obtain the title compound AG025 (49.1 mg, 17.9%) as a yellow solid.

$^1H$-NMR (300 MHz, DMSO-$d_6$) δ 8.73 (s, 1H), 8.58 (d, J = 2.3 Hz, 1H), 8.43 (d, J = 8.4 Hz, 1H), 8.38 (d, J = 2.8 Hz, 1H), 8.24 (d, J = 1.7 Hz, 1H), 7.89 (dd, J = 9.2, 2.3 Hz, 1H), 7.76 (dd, J = 12.7, 8.9 Hz, 3H), 7.66-7.49 (m, 3H), 7.50-7.42 (m, 2H), 7.41-7.33 (m, 2H), 7.27 (t, J = 7.6 Hz, 2H), 7.20-7.11 (m, 2H), 6.92 (d, J = 8.7 Hz, 2H), 4.18 (q, J = 7.0 Hz, 2H), 4.07-4.00 (m, 2H), 3.68-3.60 (m, 2H), 3.32-3.21 (m, 6H), 2.68-2.60 (m, 4H), 1.37 (t, J = 6.9 Hz, 3H).

LRMS (ESI): m/z 803 $[M+H]^+$.

Example 6-5-1: Synthesis of tert-butyl 4-[6-[[4-(2-phenylsulfanylethylamino)-3- (trifluoromethyl)phenyl] sulfonylcarbamoyl]pyridazin-3 -yl]piperazine-1 -carboxylate (AG027)

[Formula 555]

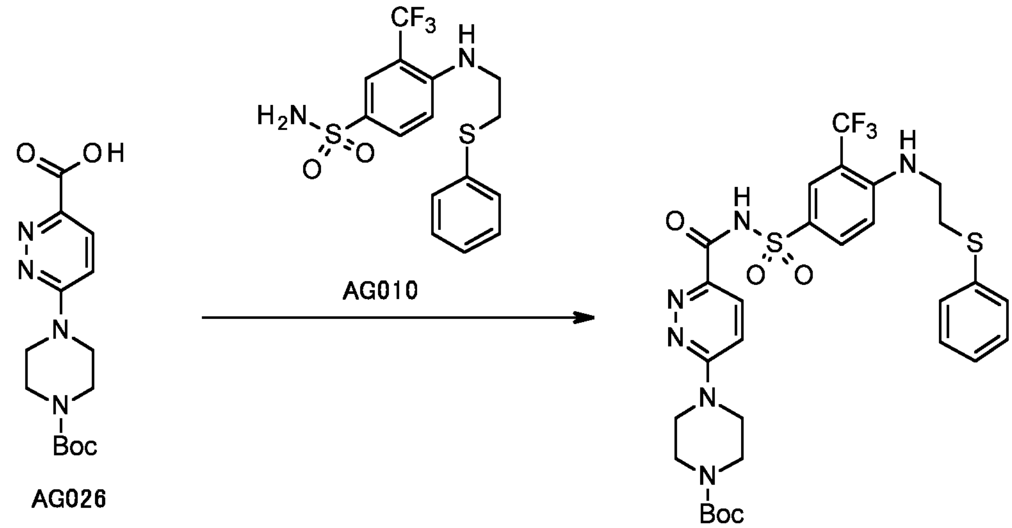

AG026

AG027

To a solution of 6-[4-[(2-methylpropan-2-yl)oxycarbonyl]piperazin-1-yl]pyridazine- 3-carboxylic acid (AG026, CAS: 1690948-00-5) (400 mg, 1.297 mmol), 4-(2- phenylsulfanylethylamino)-3-(trifluoromethyl)benzenesulfonamide (AG010) (488 mg, 1.297 mmol), EDCI (497 mg, 2.594 mmol), and DMAP (158 mg, 1.297 mmol) in dichloromethane (5 mL), DIPEA (671 mg, 5.188 mmol) was added, and the mixture was stirred at room temperature for 16 hours. Water was added to the mixture. An organic layer was extracted with ethyl acetate three times and dried over sodium sulfate. After concentration, the obtained residue was purified by silica gel column chromatography (ethyl acetate/petroleum ether, 0-100%, and subsequently methanol/dichloromethane, 0-10%) to obtain the title compound AG027(400 mg, 43.9%) as a pale yellow solid.

LRMS (ESI): m/z 667$[M+H]^+$.

Retention time: 1.185 min (analysis conditions PH-FA05-4).

Example 6-5-2: Synthesis of N-[4-(2-phenylsulfanylethylamino)-3- (trifluoromethyl)phenyl]sulfonyl-6-piperazin-1 -ylpyridazine-3-carboxamide (AG028)

[Formula 556]

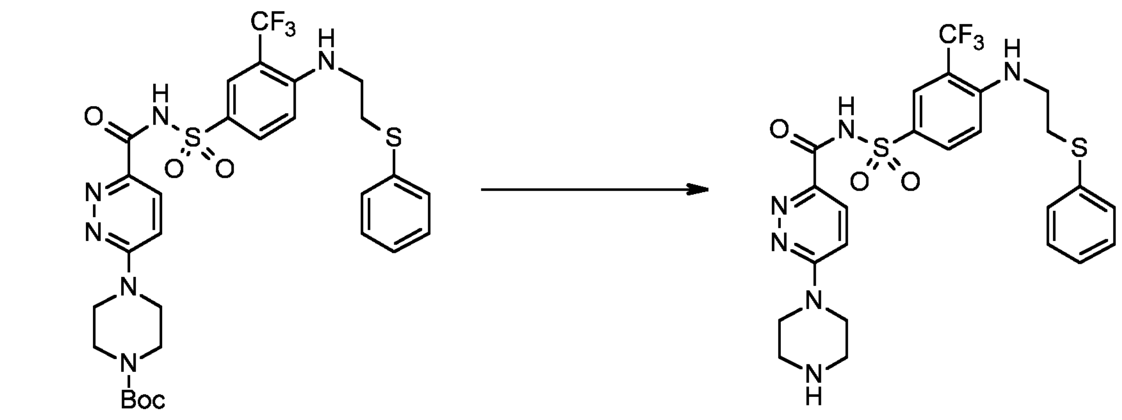

AG027 AG028

To a solution of tert-butyl 4-[6-[[4-(2-phenylsulfanylethylamino)-3- (trifluoromethyl)phenyl] sulfonylcarbamoyl]pyridazin-3 -yl]piperazine-1 -carboxylate (AG027) (200.00 mg, 0.300 mmol) in dichloromethane (4 mL), hydrogen chloride (4 M solution in dioxane, 2 mL) was added, and the mixture was stirred at room temperature for 16 hours. The mixture was concentrated and dissolved in water, and the solution was adjusted to pH 8 by the addition of an aqueous sodium bicarbonate solution. The resulting solid was filtered, then washed with water, and dried at 50°C for 16 hours to obtain the title compound AG028 (150 mg) as a light brown solid.

LRMS (ESI): m/z 567[M+H]+.

Retention time: 0.883 min (analysis conditions PH-FA05-4).

Example 6-5-3: Synthesis of 6-[4-[4-(3-ethoxyphenyl)-3-fluorobenzoyl]piperazin-1- yl] -N- [4-(2-phenylsulfanylethylammo)-3 -(trifluoromethyl)phenyl] sulfonylpyridazine-3 - carboxamide (AG030)

[Formula 557]

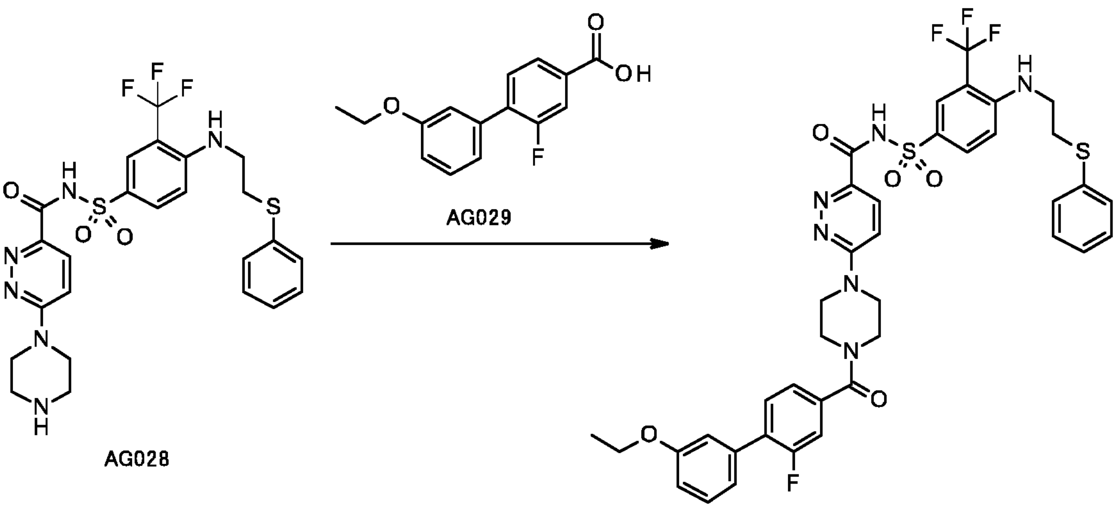

AG029

AG028

AG030

To a solution of N-[4-(2-phenylsulfanylethylamino)-3- (trifluoromethyl)phenyl]sulfonyl-6-piperazin-1-ylpyridazine-3-carboxamide (AG028) (70 mg, 0.124 mmol), 4-(3-ethoxyphenyl)-3-fluorobenzoic acid (AG029, CAS: 1261964-16-2) (32.2 mg, 0.124 mmol), EDCI (47.4 mg, 0.248 mmol), and DMAP (15.1 mg, 0.124 mmol) in dichloromethane (2 mL), DIPEA (63.9 mg, 0.496 mmol) was added, and the mixture was stirred at room temperature for 16 hours. The mixture was concentrated. Then, the obtained residue was purified by reverse-phase column chromatography (C18, acetonitrile/10 mM aqueous ammonium bicarbonate solution, 0-50%). The obtained crude product was purified by

preparative HPLC (XBridge Prep OBD C18 column, acetonitrile/10 mM aqueous ammonium bicarbonate solution, 36-66%) to obtain the title compound AG030 (20 mg, 19.6%) as a white solid.

$^1$H-NMR (300 MHz, DMSO-$d_6$) δ 7.97 (s, 1H), 7.85 (t, J = 12.6 Hz, 2H), 7.64 (t, J = 8.1 Hz, 1H), 7.56-7.20 (m, 8H), 7.24-7.04 (m, 3H), 7.00 (d, J = 8.1 Hz, 1H), 6.86 (d, J = 9.0 Hz, 1H), 6.45 (s, 1H), 4.09 (q, J = 7.2 Hz, 2H), 3.85-3.73 (m, 5H), 3.59-3.45 (m, 5H), 3.20-3.13 (m, 2H), 1.35 (t, J = 6.9 Hz, 3H).

LRMS (ESI): m/z 809 $[M+H]^+$.

Example 6-6-1: Synthesis of 6-[4-[3-(3-ethoxyphenyl)-5- (trifluoromethyl)benzoyl]piperazin-1-yl]-N-[4-(2-phenylsulfanylethylamino)-3- (trifluoromethyl)phenyl]sulfonylpyridazine-3-carboxamide (AG032)

[Formula 558]

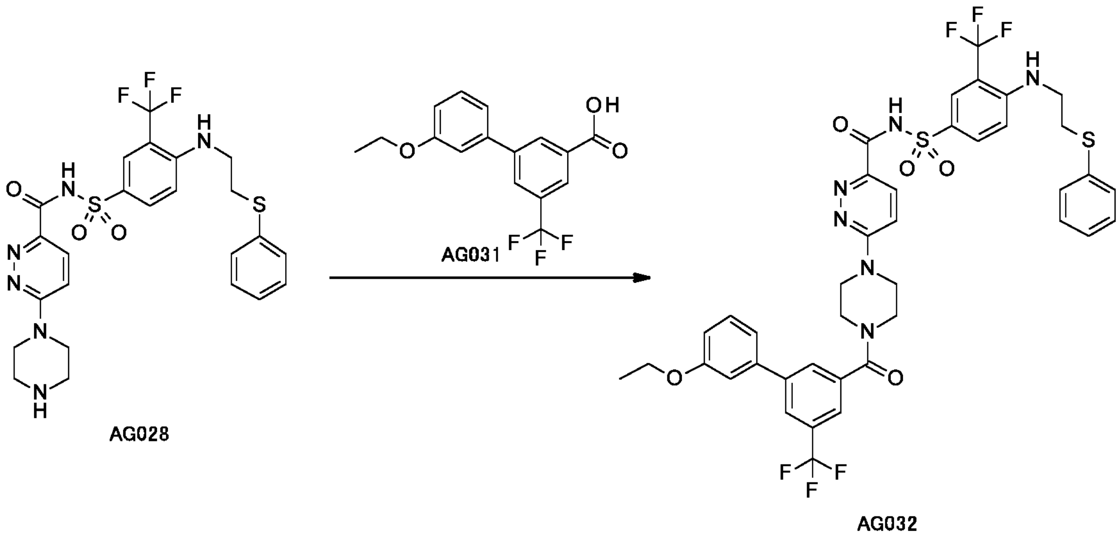

To a solution of N-[4-(2-phenylsulfanylethylamino)-3- (trifluoromethyl)phenyl]sulfonyl-6-piperazin-1-ylpyridazine-3-carboxamide (AG028) (70 mg, 0.124 mmol), 3-(3-ethoxyphenyl)-5-(trifluoromethyl)benzoic acid (AG031, CAS: 1261907- 60-1) (69.00 mg, 0.223 mmol), EDCI (47.37 mg, 0.248 mmol), and DMAP (15.09 mg, 0.124 mmol) in dichloromethane (2 mL), DIPEA (63.87 mg, 0.496 mmol) was added, and the mixture was stirred at room temperature for 16 hours. The mixture was concentrated. Then, the obtained residue was purified by reverse-phase column chromatography (C18, acetonitrile/10 mM aqueous ammonium bicarbonate solution, 0-45%). The obtained crude product was purified by preparative HPLC (Xselect CSH OBD column, acetonitrile/0.1% aqueous formic acid solution, 68-83%) to obtain the title compound AG032(30 mg, 28.2%) as a white solid.

$^1$H-NMR (300 MHz, DMSO-$d_6$) δ 12.22 (s, 1H), 8.12 (d, J = 11.7 Hz, 1H), 8.06 (s, 1H), 8.00 (d, J = 2.1 Hz, 1H), 7.94-7.87 (m, 1H), 7.82 (d, J = 9.6 Hz, 2H), 7.50-7.27 (m, 8H), 7.24-7.17 (m, 1H), 7.05-6.98 (m, 1H), 6.93 (d, J = 9.0 Hz, 1H), 6.64 (s, 1H), 4.14 (q, J = 6.9 Hz, 2H), 3.96-3.76 (m, 6H), 3.59-3.43 (m, 4H), 3.22-3.13 (m, 2H), 1.36 (t, J = 6.9 Hz, 3H).

LRMS (ESI): m/z 859 $[M+H]^+$.

Example 6-7-1: Synthesis of tert-butyl 4-[6-(1- adamantylmethylcarbamoyl)pyridazin-3-yl]piperazine-1-carboxylate (AG034)

[Formula 559]

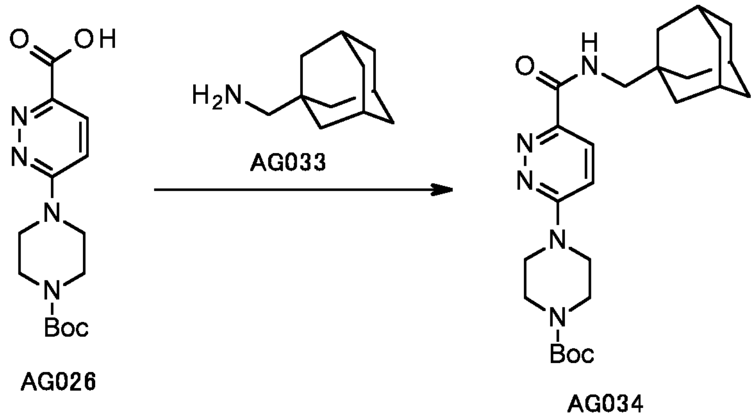


AG026

AG034

To a solution of 6-[4-[(2-methylpropan-2-yl)oxycarbonyl]piperazin-1-yl]pyridazine- 3-carboxylic acid (AG026, CAS: 1690948-00-5) (200 mg, 0.649 mmol), 1- adamantylmethanamine (AG033) (160.8 mg, 0.973 mmol), EDCI (248.7 mg, 1.297 mmol), and DMAP (79.24 mg, 0.649 mmol) in dichloromethane (4 mL), DIPEA (335.3 mg, 2.596 mmol) was added, and the mixture was stirred at room temperature for 16 hours. An aqueous ammonium chloride solution was added to the mixture. An organic layer was extracted with dichloromethane three times and dried over sodium sulfate. After concentration, the obtained residue was purified by silica gel column chromatography (ethyl acetate/petroleum ether, 0-100%, and subsequently methanol/dichloromethane, 0-10%) to obtain the title compound AG034 (180 mg, 60.9%) as a white solid.

LRMS (ESI): m/z 456[M+H]$^+$.

Retention time: 1.238 min (analysis conditions PH-TFA05-2).

Example 6-7-2: Synthesis ofN-(1-adamantylmethyl)-6-piperazin-1-ylpyridazine-3- carboxamide (AG035)

[Formula 560]

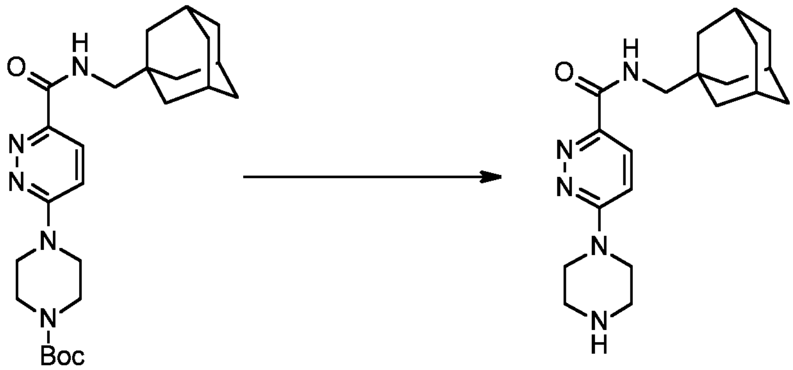

AG034

AG035

To a solution of tert-butyl 4-[6-(1-adamantylmethylcarbamoyl)pyridazin-3- yl]piperazine-1-carboxylate (AG034) (180 mg, 0.395 mmol) in dichloromethane (2 mL), hydrogen chloride (4 M solution in dioxane, 5 mL) was added, and the mixture was stirred at room temperature for 1 hour. The mixture was concentrated and dissolved in water, and the solution was adjusted to pH 8 to 9 by the addition of an aqueous sodium bicarbonate solution. The resulting solid was filtered, then washed three times with water, and dried at 50°C for 16 hours to obtain the title compound AG035(100 mg, 85%) as a gray solid.

LRMS (ESI): m/z 356[M+H]$^+$.

Retention time: 0.917 min (analysis conditions PH-TFA05-2).

Example 6-7-3: Synthesis of 4-(3-ethoxyphenyl)-2-ethylbenzaldehyde (AG037)

[Formula 561]

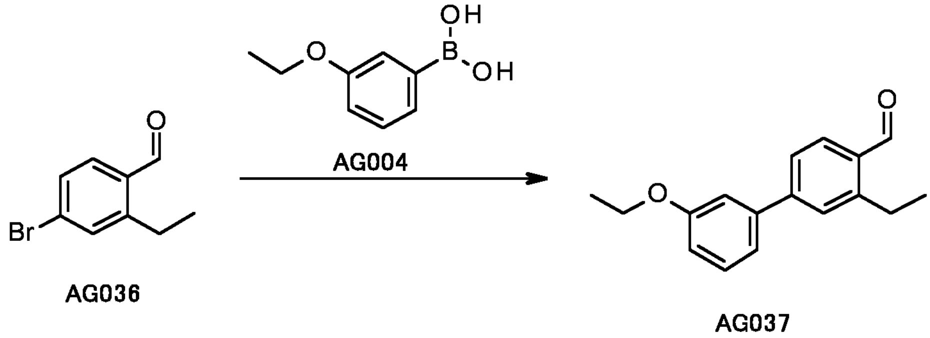

A solution of 4-bromo-2-ethylbenzaldehyde (AG036) (1.00 g, 4.693 mmol), (3- ethoxyphenyl)boronic acid (AG004) (1.01 g, 6.085 mmol), $Pd(PPh_3)_2c1_2$ (329.42 mg, 0.469 mmol), and sodium carbonate (646.65 mg, 6.101 mmol) in dimethoxyethane/ethanol/water = 1/1/1 (20 mL) was stirred at 80°C for 3 hours. An aqueous ammonium chloride solution was added to the mixture. An organic layer was extracted with ethyl acetate three times and dried over sodium sulfate. After concentration, the obtained residue was purified by preparative TLC (ethyl acetate/petroleum ether, 20%) to obtain the title compound AG037 (900 mg, 73.9%) as a pale yellow liquid.

LRMS (ESI): m/z 255$[M+H]^+$.

Retention time: 1.184 min (analysis conditions PH-FA05-4).

Example 6-7-4: Synthesis ofN-(1-adamantylmethyl)-6-[4-[[4-(3-ethoxyphenyl)-2- ethylphenyl]methyl]piperazin-1-yl]pyridazine-3-carboxamide [AG038 (B1A075-01)]

[Formula 562]

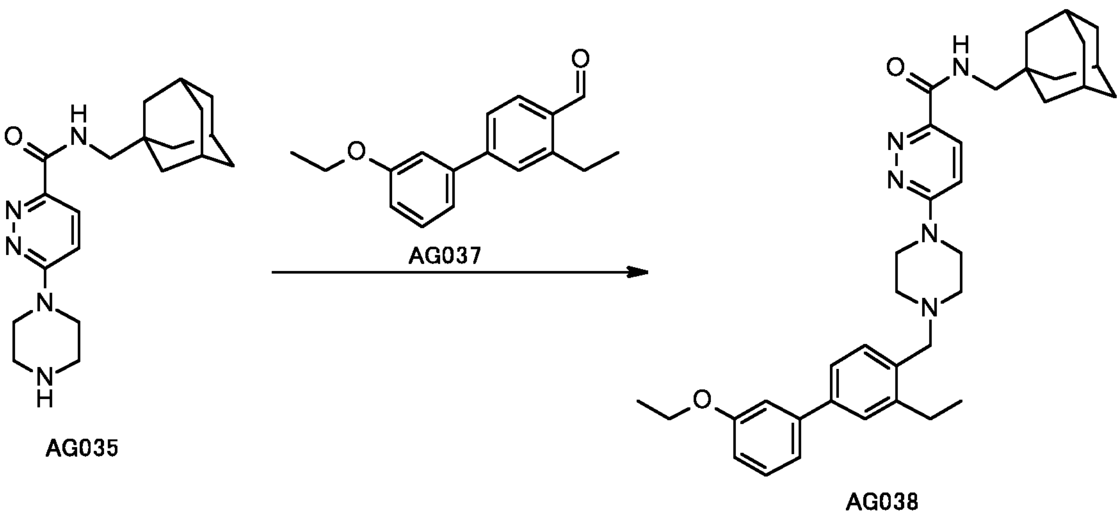

A solution ofN-(1-adamantylmethyl)-6-piperazin-1-ylpyridazine-3-carboxamide (AG035) (150 mg, 0.422 mmol), 4-(3-ethoxyphenyl)-2-ethylbenzaldehyde (AG037) (128.8 mg, 0.506 mmol), and acetic acid (0.2 mL) in DMSO (3 mL)/ethanol (3 mL) was stirred at room temperature for 1 hour. Sodium cyanoborohydride (53.03 mg, 0.844 mmol) was added thereto, and the mixture was stirred at room temperature for 16 hours. The mixture was concentrated. Then, the obtained residue was purified by reverse-phase column chromatography (C18, acetonitrile/water, 0-80%) to obtain the title compound AG038 (38.2 mg, 14.9%) as a white solid.

$^1H$-NMR (300 MHz, DMSO-$d_6$) $\delta$ 8.52 (t, J = 6.6 Hz, 1H), 7.83 (d, J = 9.6 Hz, 1H), 7.50-7.43 (m, 2H), 7.38-7.31

(m, 3H), 7.23-7.15 (m, 2H), 6.92-6.89 (m, 1H), 4.09 (q, J = 6.9 Hz, 2H), 3.72-3.67 (m, 4H), 3.55 (s, 2H), 3.32-3.28 (m, 1H), 3.02 (d, J = 6.6 Hz, 2H), 2.82-2.75 (m, 2H), 2.56-2.50 (m, 3H), 1.92 (s, 3H), 1.68-1.55 (m, 6H), 1.50-1.46 (m, 6H), 1.35 (t, J = 6.9 Hz, 3H), 1.24 (t, J = 7.8 Hz, 3H).

LRMS (ESI): m/z 594 $[M+H]^+$.

Example 6-8-1: tert-Butyl 4-[6-[1-adamantylmethyl(methyl)carbamoyl]pyridazin-3- yl]piperazine-1-carboxylate (AG040)

[Formula 563]

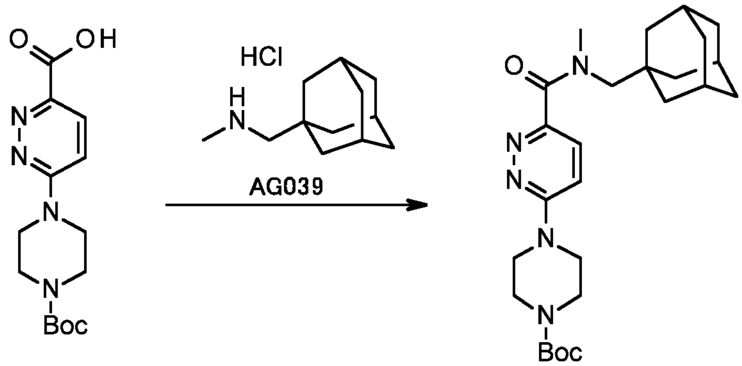


AG026 AG040

To a solution of 6-[4-[(2-methylpropan-2-yl)oxycarbonyl]piperazin-1-yl]pyridazine- 3-carboxylic acid (AG026, CAS: 1690948-00-5) (150 mg, 0.486 mmol), 1-(1-adamantyl)-N- methylmethanamine hydrochloride (AG039) (174.5 mg, 0.973 mmol), EDCI (186.52 mg, 0.973 mmol), and DMAP (59.4 mg, 0.486 mmol) in dichloromethane (6 mL), DIPEA (251.5 mg, 1.946 mmol) was added, and the mixture was stirred at room temperature for 16 hours. An aqueous ammonium chloride solution was added to the mixture. An organic layer was extracted with dichloromethane three times and dried over sodium sulfate. After concentration, the obtained residue was purified by silica gel column chromatography (ethyl acetate/petroleum ether, 0-100%, and subsequently methanol/dichloromethane, 0-10%) to obtain the title compound AG040(150 mg, 63%) as a white solid.

LRMS (ESI): m/z 470$[M+H]^+$.

Retention time: 1.155 min (analysis conditions PH-TFA05-2).

Example 6-8-2: Synthesis ofN-(1-adamantylmethyl)-N-methyl-6-piperazin-1- ylpyridazine-3-carboxamide (AG041)

[Formula 564]

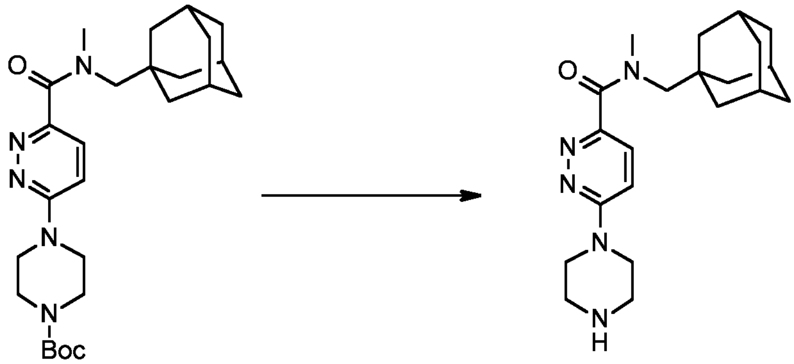

AG040 AG041

To a solution of tert-butyl 4-[6-[1-adamantylmethyl(methyl)carbamoyl]pyridazin-3- yl]piperazine-1-carboxylate (AG040) (150 mg, 0.319 mmol) in dichloromethane (3 mL), hydrogen chloride (4 M solution in dioxane, 6 mL) was added,

and the mixture was stirred at room temperature for 1 hour. The mixture was concentrated and dissolved in water, and the solution was adjusted to pH 8 to 9 by the addition of an aqueous sodium bicarbonate solution. The resulting solid was filtered, then washed three times with water, and dried at 50°C for 16 hours to obtain the title compound AG041 (100 mg) as a yellow solid.

LRMS (ESI): m/z 370$[M+H]^+$.

Retention time: 0.882 min (analysis conditions PH-TFA05-2).

Example 6-8-3: Synthesis ofN-(1-adamantylmethyl)-6-[4-[[4-(3-ethoxyphenyl)-3- methylphenyl]methyl]piperazin-1-yl]-N-methylpyridazine-3-carboxamide [AG042 (B3A073- 01)]

[Formula 565]

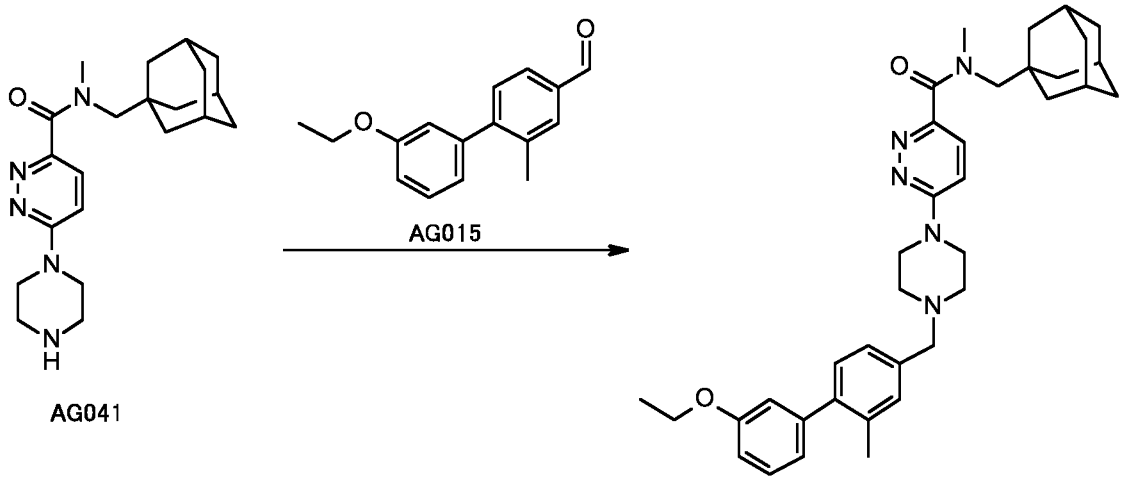


AG041

AG042

A solution of N-(1-adamantylmethyl)-N-methyl-6-piperazin-1-ylpyridazine-3- carboxamide (AG041) (180 mg, 0.487 mmol), 4-(3-ethoxyphenyl)-3-methylbenzaldehyde (AG015, CAS: 1506205-91-9) (175.6 mg, 0.731 mmol), and acetic acid (0.6 mL) in DMSO (3 mL)/ethanol (3 mL) was stirred at room temperature for 1 hour. Sodium cyanoborohydride (61.2 mg, 0.974 mmol) was added thereto, and the mixture was stirred at room temperature for 16 hours. The mixture was concentrated. Then, the obtained residue was purified by reverse-phase column chromatography (C18, acetonitrile/water, 0-80%) to obtain the title compound AG042 (70 mg, 23.7%) as a white solid.

$^1$H-NMR (300 MHz, DMSO-d6) δ 7.56-7.51 (m, 1H), 7.35-7.29 (m, 2H), 7.25-7.23 (m, 1H), 7.21-7.16 (m, 2H), 6.92-6.83 (m, 3H), 4.05 (q, J = 6.9 Hz, 2H), 3.70-3.65 (m, 4H), 3.53 (s, 3H), 3.33 (s, 2H), 3.11-3.06 (m, 3H), 2.53-2.50 (m, 2H), 2.31 (s, 3H), 1.97-1.91 (m, 2H), 1.84 (s, 1H), 1.70-1.45 (m, 11H), 1.33 (t, J = 6.9 Hz, 3H), 1.27-1.23 (m, 2H).

LRMS (ESI): m/z 594 $[M+H]^+$.

Example 6-9-1: Synthesis of 4-(3-ethoxyphenyl)-2-methoxybenzaldehyde (AG044)

[Formula 566]

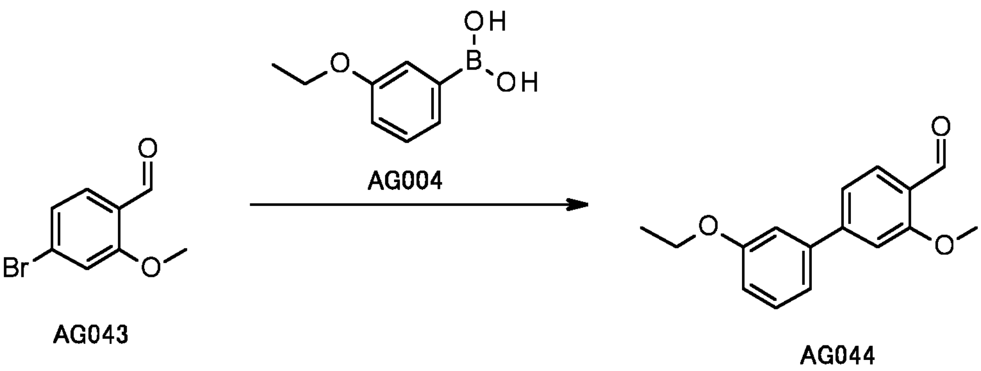

AG004

AG043

AG044

Under a nitrogen atmosphere, a solution of 4-bromo-2-methoxybenzaldehyde (AG043) (200 mg, 0.930 mmol), (3-ethoxyphenyl)boronic acid (AG004) (308.7 mg, 1.860 mmol), $Pd(PPh_3)_2c1_2$ (65.28 mg, 0.093 mmol), and sodium carbonate (128.14 mg, 1.209 mmol) in dimethoxyethane/ethanol/water = 1/1/1 (6 mL) was stirred at 80°C for 3 hours. The mixture was filtered. The filtrate was concentrated. Then, the obtained residue was purified by silica gel column chromatography (ethyl acetate/petroleum ether, 0-20%) to obtain the title compound AG044 (2140 mg, 54.0%) as a yellow liquid.
LRMS (ESI): m/z 257[M+H]+.
Retention time: 0.839 min (analysis conditions PH-FA05-3).

Example 6-9-2: Synthesis of 4-[4-[[4-(3-ethoxyphenyl)-2- methoxyphenyl]methyl]piperazin-1-yl]-N-[3-nitro-4-(2- phenylsulfanylethylamino)phenyl]sulfonylbenzamide [AG045 (B2R074-01)]

[Formula 567]

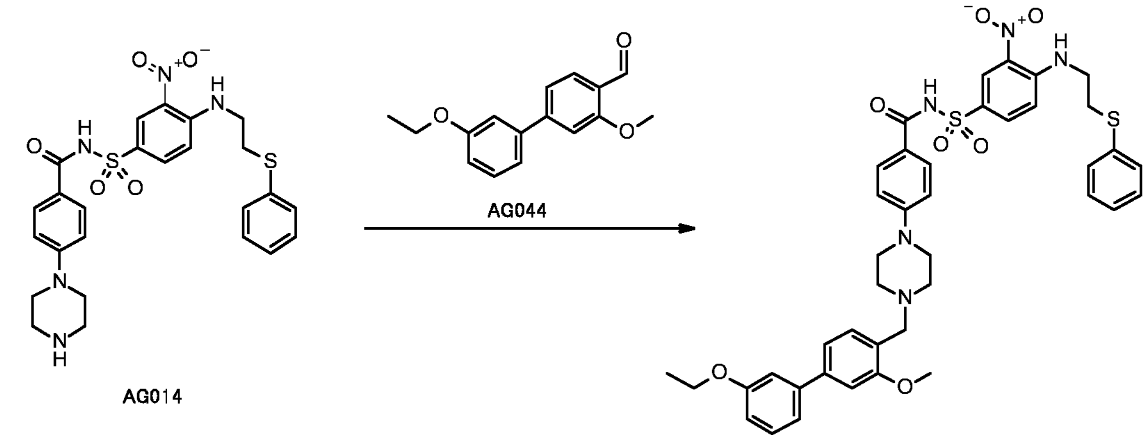

AG044

AG014

AG045

A solution of N-[3-nitro-4-(2-phenylsulfanylethylamino)phenyl]sulfonyl-4- piperazin-1-ylbenzamide (AG014, CAS: 406233-75-8) (100.00 mg, 0.185 mmol), 4-(3- ethoxyphenyl)-2-methoxybenzaldehyde (AG044) (94.64 mg, 0.369 mmol), and acetic acid (0.05 mL) in DMSO (2 mL)/ethanol (2 mL) was stirred at room temperature for 1 hour. Sodium cyanoborohydride (23.2 mg, 0.369 mmol) was added thereto, and the mixture was stirred at room temperature for 16 hours. The mixture was concentrated. Then, the obtained residue was purified by silica gel column chromatography (ethyl acetate/petroleum ether, 0- 95%). The obtained crude product was purified by reverse-phase column chromatography (C18, acetonitrile/water, 10-70%) to obtain the title compound AG045(37 mg, 25.4%) as a yellow solid.
$^1$H-NMR (300 MHz, DMSO-$d_6$) $\delta$ 8.68 (s, 1H), 8.55 (d, J = 2.1 Hz, 1H), 7.89 (d, J = 9.6 Hz, 1H), 7.76 (d, J = 8.7 Hz, 2H), 7.49-7.33 (m, 4H), 7.33-7.14 (m, 7H), 7.11 (d, J = 9.3 Hz, 1H), 6.93-6.87 (m, 3H), 4.11 (q, J = 6.9 Hz, 2H), 3.91 (s, 3H), 3.81-3.78 (m, 2H), 3.77-3.68 (m, 2H), 3.68-3.58 (m, 2H), 3.30-3.25 (m, 4H), 2.78-2.69 (m, 4H), 1.36 (t, J = 6.9 Hz, 3H).
LRMS (ESI): m/z 782 [M+H]+.

Example 6-10-1: Synthesis of methyl 3-[2-(5-ethoxypyridin-3-yl)phenyl]propanoate (AG047)

[Formula 568]

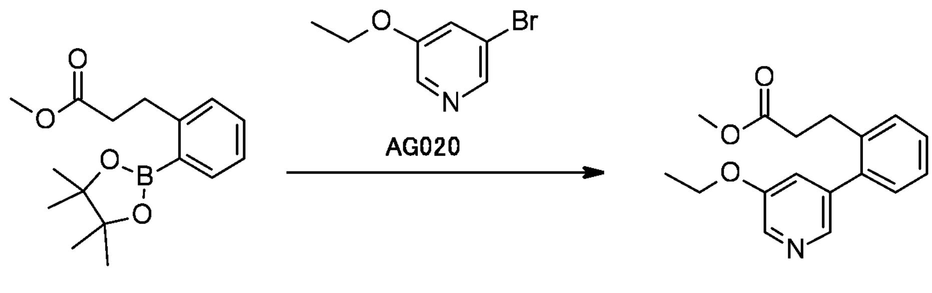

AG046 AG020 AG047

To a solution of methyl 3-[2-(4,4,5,5-tetramethyl-1,3,2-dioxaborolan-2- yl)phenyl]propanoate (AG046) (250 mg, 0.862 mmol) in dioxane (6 mL)/water (0.6 mL), 3- bromo-5-ethoxypyridine (AG020) (191.5 mg, 0.948 mmol), potassium acetate (169.1 mg, 1.724 mmol), and Pd(dppf)$Cl_2$ (63.0 mg, 0.086 mmol) were added, and the mixture was stirred at 90°C for 3 hours. The mixture was filtered, and the filtrate was concentrated. Then, the obtained residue was purified by silica gel column chromatography (ethyl acetate/petroleum ether, 0-20%) to obtain the title compound AG047 (200 mg, 77.2%) as a white solid.

LRMS (ESI): m/z 286 $[M+H]^+$.

Retention time: 0.877 min (analysis conditions PH-FA05-1).

Example 6-10-2: 3-[2-(5-Ethoxypyridin-3-yl)phenyl]propanoic acid (AG048)

[Formula 569]

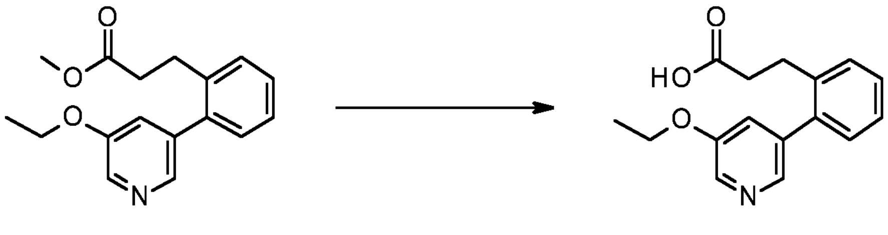

AG047 AG048

To a solution of methyl 3-[2-(5-ethoxypyridin-3-yl)phenyl]propanoate (AG047) (330 mg, 1.157 mmol) in methanol (30 mL)/water (3 mL), lithium hydroxide (276.9 mg, 11.570 mmol) was added, and the mixture was stirred at 35°C for 16 hours. The mixture was concentrated and dissolved in water, and the solution was adjusted to pH 5 to 6 by the addition of citric acid. The resulting solid was filtered, then washed three times with water, and dried at 50°C for 16 hours to obtain the title compound AG048 (200 mg) as a gray solid.

LRMS (ESI): m/z 272 $[M+H]^+$.

Retention time: 0.765 min (analysis conditions PH-FA05-5).

Example 6-10-3: Synthesis of tert-butyl 4-[6-[[3-nitro-4-(2- phenylsulfanylethylamino)phenyl]sulfonylcarbamoyl]pyridazin-3-yl]piperazine-1- carboxylate (AG049)

[Formula 570]

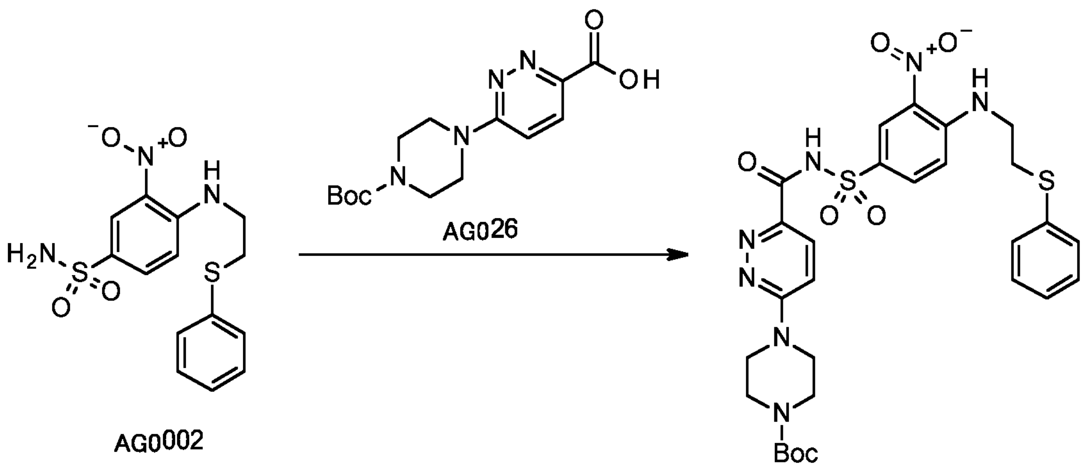


AG0002

AG049

To a solution of 6-[4-[(2-methylpropan-2-yl)oxycarbonyl]piperazin-1-yl]pyridazine- 3-carboxylic acid (AG026, CAS: 1690948-00-5) (200 mg, 0.649 mmol), 3-nitro-4-(2- phenylsulfanylethylamino)benzenesulfonamide (AG002, CAS: 406233-13-4) (344.0 mg, 0.974 mmol), EDCI (249.3 mg, 1.298 mmol), and DMAP (79.2 mg, 0.649 mmol) in dichloromethane (4 mL), DIPEA (334.9 mg, 2.596 mmol) was added, and the mixture was stirred at room temperature for 16 hours. An aqueous ammonium chloride solution was added to the mixture. An organic layer was extracted with dichloromethane three times and dried over sodium sulfate. After concentration, the obtained residue was purified by silica gel column chromatography (ethyl acetate/petroleum ether, 0-100%, and subsequently methanol/dichloromethane, 0-10%) to obtain the title compound AG049 (240 mg, 57.6%) as a white solid.

LRMS (ESI): m/z 644 $[M+H]^+$.

Retention time: 1.211 min (analysis conditions PH-TFA05-2).

Example 6-10-4: Synthesis of N-[3-nitro-4-(2- phenylsulfanylethylamino)phenyl] sulfonyl-6-piperazin-1 -ylpyridazine-3-carboxamide (AG050)

[Formula 571]

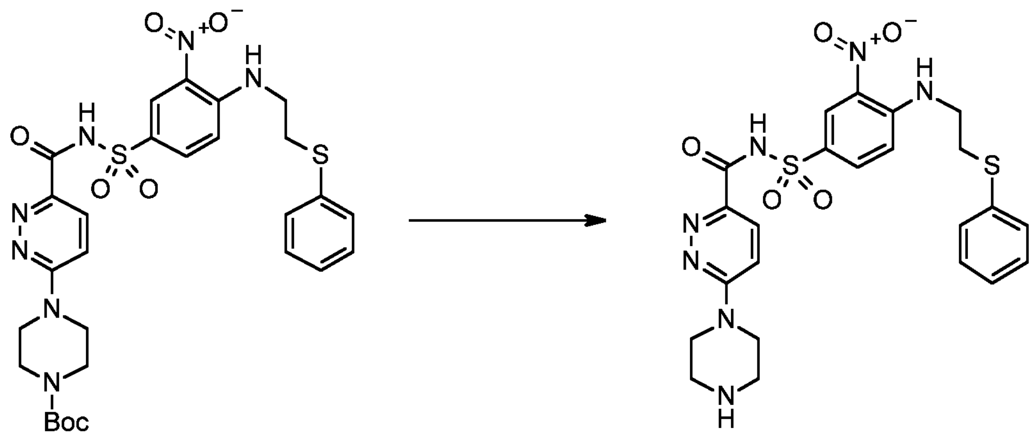

AG049 AG050

To a solution of tert-butyl 4-[6-[[3-nitro-4-(2- phenylsulfanylethylamino)phenyl] sulfonylcarbamoyl]pyridazin-3-yl]piperazine-1- carboxylate (AG049) (50.0 mg, 0.078 mmol) in dichloromethane (2 mL), hydrogen chloride (4 M solution in dioxane, 4 mL) was added, and the mixture was stirred at room temperature for 1 hour. The mixture was concentrated and dissolved in water, and the solution was adjusted to pH 8 to 9 by the addition of an aqueous sodium bicarbonate

solution. The resulting solid was filtered, then washed three times with water, and dried at 50°C for 16 hours to obtain the title compound AG050 (50 mg) as a gray solid.

LRMS (ESI): m/z 544[M+H]+.

Retention time: 0.960 min (analysis conditions PH-FA05-1).

Example 6-10-5: Synthesis of 6-[4-[3-[2-(5-ethoxypyridin-3- yl)phenyl]propanoyl]piperazin-1-yl]-N-[3-nitro-4-(2- phenylsulfanylethylamino)phenyl]sulfonylpyridazine-3-carboxamide [AG051 (B2Q012-01)]

[Formula 572]

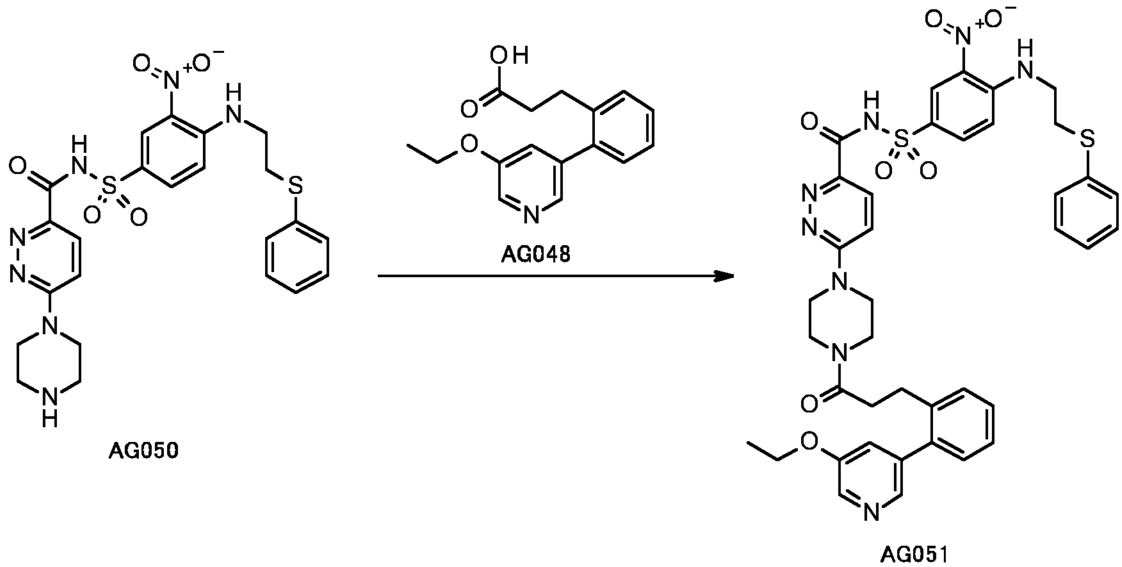


To a solution of N-[3-nitro-4-(2-phenylsulfanylethylamino)phenyl]sulfonyl-6- piperazin-1-ylpyridazine-3-carboxamide (AG050) (100 mg, 0.184 mmol), 3-[2-(5- ethoxypyridin-3-yl)phenyl]propanoic acid (AG048) (74.9 mg, 0.276 mmol), EDCI (70.5 mg, 0.368 mmol), and DMAP (22.5 mg, 0.184 mmol) in dichloromethane (2 mL), DIPEA (95.1 mg, 0.736 mmol) was added, and the mixture was stirred at room temperature for 16 hours. An aqueous ammonium chloride solution was added to the mixture. An organic layer was extracted with dichloromethane three times and dried over sodium sulfate. After concentration, the obtained residue was purified by silica gel column chromatography (ethyl acetate/petroleum ether, 0-100%, and subsequently methanol/dichloromethane, 0-10%) to obtain the title compound AG051 (40 mg, 26.7%) as a white solid.

$^1$H-NMR (300 MHz, DMSO-$d_6$) $\delta$ 8.54-8.48 (m, 1H), 8.29-8.24 (m, 1H), 8.13-8.07 (m, 1H), 7.89-7.76 (m, 1H), 7.45-7.26 (m, 7H), 7.22-7.12 (m, 2H), 7.03-7.92 (m, 3H), 4.18- 4.08 (m, 2H), 3.63 -3.51 (m, 5H), 3.45-3.38 (m, 2H), 3.28-3.22 (m, 2H), 2.81-2.71 (m, 2H), 2.59-2.50 (m, 6H), 1.35-1.25 (m, 3H).

LRMS (ESI): m/z 797 [M+H]+.

Example 6-11-1: Synthesis of 6-[4-[[4-(5-ethoxypyridin-3-yl)-3- methylphenyl] methyl]piperazin-1-yl]-N-[4-(2-phenylsulfanylethylamino)-3- (trifluoromethyl)phenyl]sulfonylpyridazine-3-carboxamide [AG052 (B2R074-01)]

[Formula 573]

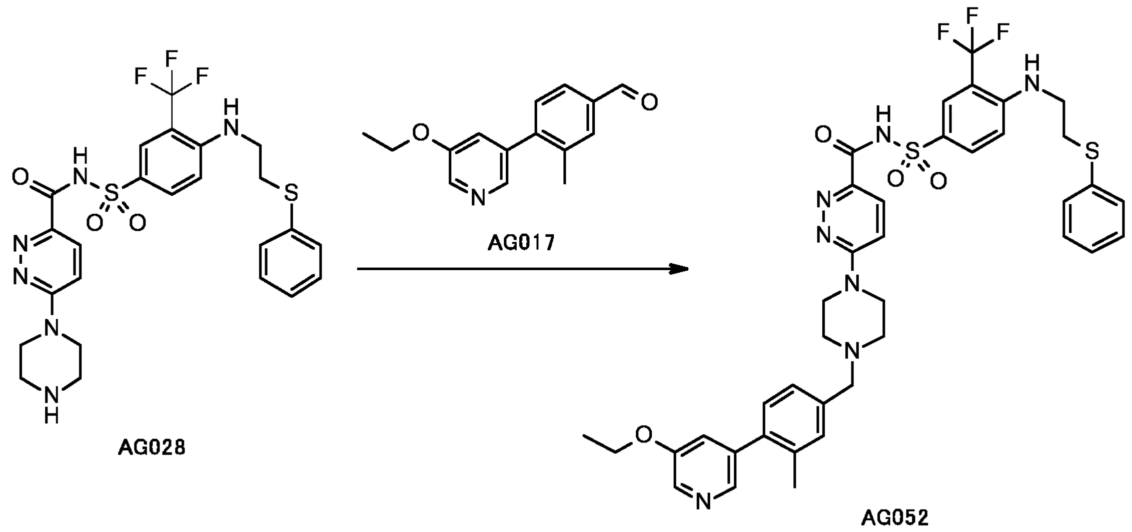

A solution of N-[4-(2-phenylsulfanylethylamino)-3- (trifluoromethyl)phenyl]sulfonyl-6-piperazin-1-ylpyridazine-3-carboxamide (AG028) (310.00 mg, 0.547 mmol), 4-(5-ethoxypyridin-3-yl)-3-methylbenzaldehyde (AG017, CAS: 1545014-81-0) (264.02 mg, 1.094 mmol), and acetic acid (0.05 mL) in DMSO (3 mL)/ethanol (3 mL) was stirred at room temperature for 1 hour. Sodium cyanoborohydride (68.76 mg, 1.094 mmol) was added thereto, and the mixture was stirred at room temperature for 16 hours. The mixture was purified by reverse-phase column chromatography (C18, acetonitrile/water, 5-70%). The obtained crude product was purified by preparative HPLC (XBridge Prep OBD C18 column, acetonitrile/10 mM aqueous ammonium bicarbonate solution, 34-64%) to obtain the title compound AG052 (37.7 mg, 8.6%) as a gray solid.

$^{1}$H-NMR (300 MHz, DMSO-$d_6$) δ 8.26 (d, J = 2.7 Hz, 1H), 8.12 (d, J = 1.8 Hz, 1H), 7.99-7.96 (m, 1H), 7.90-7.85 (m, 1H), 7.80-7.49 (m, 1H), 7.41-7.36 (m, 2H), 7.35-7.26 (m, 5H), 7.25-7.17 (m, 3H), 6.87 (d, J = 9.0 Hz, 1H), 6.55-6.49 (m, 1H), 4.15 (q, J = 6.9 Hz, 2H), 3.78-3.71 (m, 4H), 3.58 (s, 2H), 3.51-3.43 (m, 3H), 3.19-3.12 (m, 2H), 2.59-2.53 (m, 4H), 2.13 (s, 3H), 1.35 (t, J = 6.9 Hz, 3H).

LRMS (ESI): m/z 792 $[M+H]^+$.

Example 6-12-1: Synthesis of solid-phase supported ArBr (compound D043-02R)

[Formula 574]

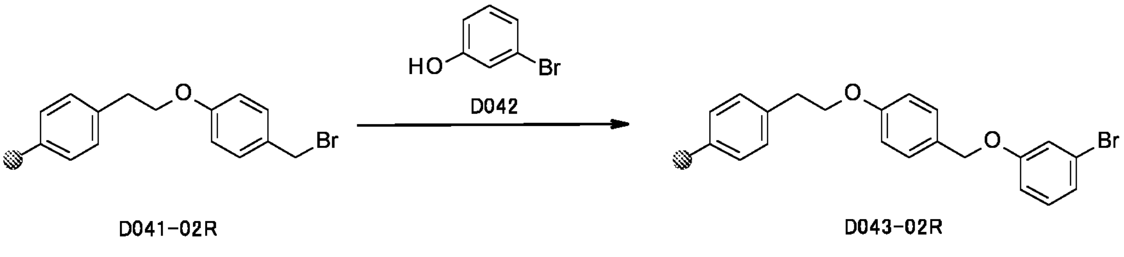

Under a nitrogen atmosphere, 2-(4-bromomethylphenoxy)ethyl polystyrene (compound D041-02R, Sigma-Aldrich Co., LLC, 534471, bromide, 100-200 mesh, loading rate: 1.25 mmol/g, copolymer (styrene-1% DVB), 5.00 g, 6.25 mmol) and NMP (50 mL) were added to a 150 mL glass vial and shaken at room temperature for 1 hour. Under a nitrogen atmosphere, 3-bromophenol (compound D042, 2.16 g, 12.5 mmol) and tBuOK (1.33 g, 11.9 mmol) were added thereto, and the mixture was shaken at room temperature for 20 hours. The reaction solution was transferred to a syringe with a filter using NMP (100 mL), and the resin was washed twice with NMP (100 mL). Further, the resin was repetitively washed three times with NMP/water (1/1, 100 mL), three times with water (100 mL), three times with NMP (100 mL), three times with methanol (100 mL), three times with DCM (100 mL), and three times with heptane (100 mL), and the obtained resin was dried overnight under reduced pressure to obtain the title compound D043-02R (4.95 g).

Example 6-12-2: The loading rate of solid-phase supported ArBr (compound D043- 02R) was determined by

the following method.

[Formula 575]

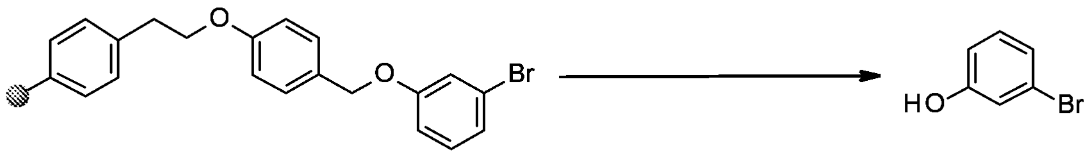

D043-02R D042

Under a nitrogen atmosphere, compound D043-02R (22.6 mg) was dipped in 30% TFA/DCM containing trimethylbenzene (0.08 M, 1 mL) in a 3 mL glass vial for 15 minutes. The cleaving solution was filtered, and the resin was washed with NMP (500 μL). On the basis of the UV area of the obtained 3-bromophenol (D042), the concentration of the 3- bromophenol (D042) was quantified using a calibration curve to determine the amount of the 3-bromophenol (D042) supported on the resin. As a result, the amount of the compound supported in D043-02R was calculated to be 0.532 mmol/g.

Compound D042
Retention time: 0.893 min (analysis conditions SMD-FA05-1, 290 nm).
Wavelength of maximum absorption: 276 nm.

Example 6-12-3: Synthesis of solid-phase supported ArBr (compound D045-02R)

[Formula 576]

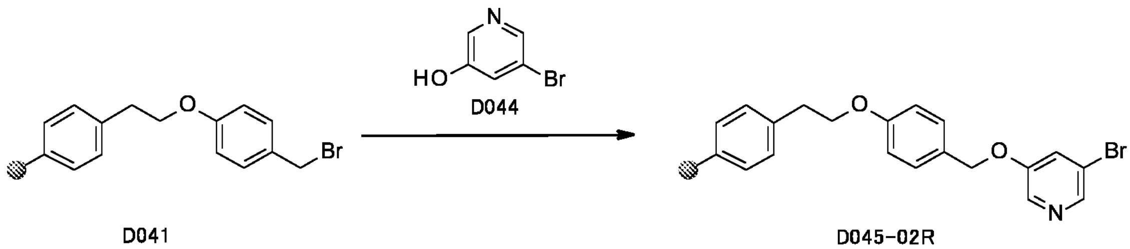


D041 D045-02R

Solid-phase supported ArBr (compound D045-02R) (5.01 g) was obtained by synthesis in the same manner as in compound D043-02R in Example 6-12-1 using 5- bromopyridin-3-ol (compound D044, 2.18 g, 12.5 mmol).

Example 6-12-4: The loading rate of solid-phase supported ArBr (compound D045- 02R) was determined by the following method.

[Formula 577]

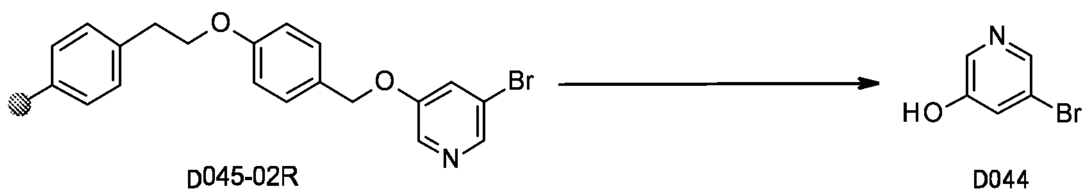

D045-02R D044

The amount of D044 supported was calculated in the same manner as in Example 6- 12-2 (compound D042) using compound D045-02R (22.6 mg). As a result, the amount of the compound supported in D045-02R was calculated to be 0.735 mmol/g.

Compound D044
LCMS: m/z 174[M+H]$^+$.
Retention time: 0.573 min (analysis conditions SMD-FA05-1, 290 nm).

Example 6-12-5: Synthesis of solid-phase supported ketone (compound D047-02R)

[Formula 578]

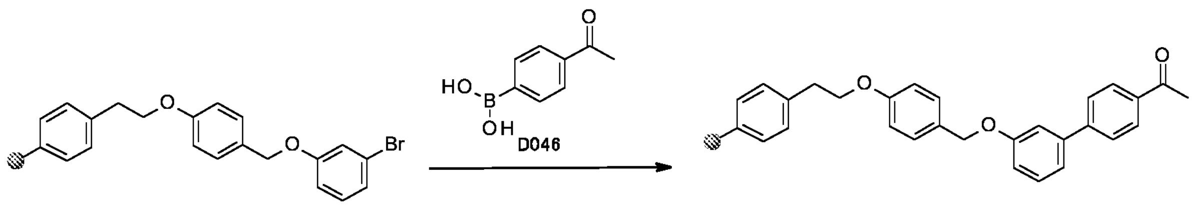


D043-02R D047-02R

Under a nitrogen atmosphere, solid-phase supported ArBr (compound D043-02R) (100 mg, loading rate: 0.532 mmol/g, 0.0532 mmol) and NMP (1.9 mL) were added to a 4 mL glass vial and shaken at room temperature for 1 hour. Under a nitrogen atmosphere, (4- acetylphenyl)boronic acid (D046) (52.3 mg, 0.319 mmol), a 1.7 M aqueous $K_3PO_4$ solution (188 μL, 0.319 mmol), and a 0.04 M solution of $P(Cy)_3$ Pd G3 in NMP (266 μL, 10.6 μmol ) were added thereto, and the mixture was shaken at 90°C for 2 hours. The reaction solution and the resin suspension were transferred to a syringe with a filter using NMP (2 mL) and repetitively washed twice with NMP (2 mL), three times with a NMP:HzO (1:1) solution containing NAC (0.05 M) (2 mL), three times with water (2 mL), three times with NMP (2 mL), three times with MeOH (2 mL), three times with DCM (2 mL), and three times with heptane (2 mL), and the resin was then dried under reduced pressure to obtain the title compound D047-02R (107.6 mg). A portion (resin) of the compound D047-02R was transferred onto a pipette tip with a filter and dipped in a 10% solution of TFA in DCM containing 0.08 M pentamethylbenzene (50 μL) for 5 minutes. The cleavage solution was filtered, and the solid phase was washed with NMP (50 μL). The obtained filtrate was diluted with MeCN (500 μL) to prepare an LC sample. The formation of D047 was confirmed by LC-MS analysis.

[Formula 579]

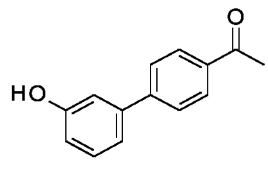

D047

Compound D047
LCMS: m/z 213[M+H]+.
Retention time: 0.927 min (analysis conditions SMD-FA05-1, 290 nm).

Example 6-12-6: Synthesis of solid-phase supported ketone (compound D048-02R)

[Formula 580]

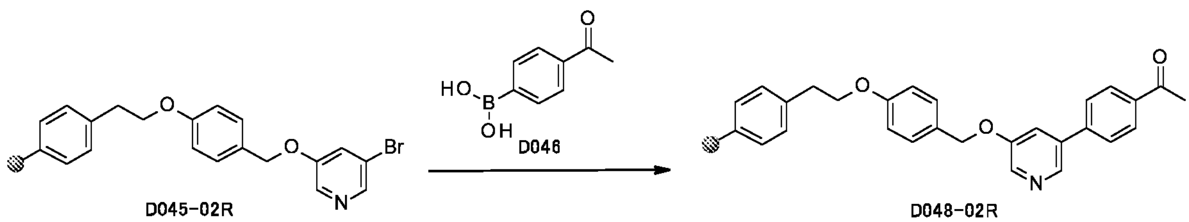


D045-02R D048-02R

The title compound D048-02R (108.3 mg) was obtained by synthesis in the same manner as in Example 6-12-5

(compound D047-02R) using solid-phase supported ArBr (compound D045-02R, loading rate: 0.735 mmol/g, 100 mg). The same cleavage operation as in Example 6-12-5 was performed, so that the formation of D048 was confirmed.

[Formula 581]

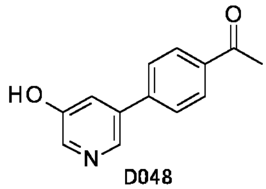

D048

Compound D048

LCMS: m/z 214[M+H]+.

Retention time: 0.534 min (analysis conditions SMD-FA05-1, 290 nm).

Example 6-12-7: Synthesis of solid-phase supported allyl ester (compound D050- 02R)

[Formula 582]

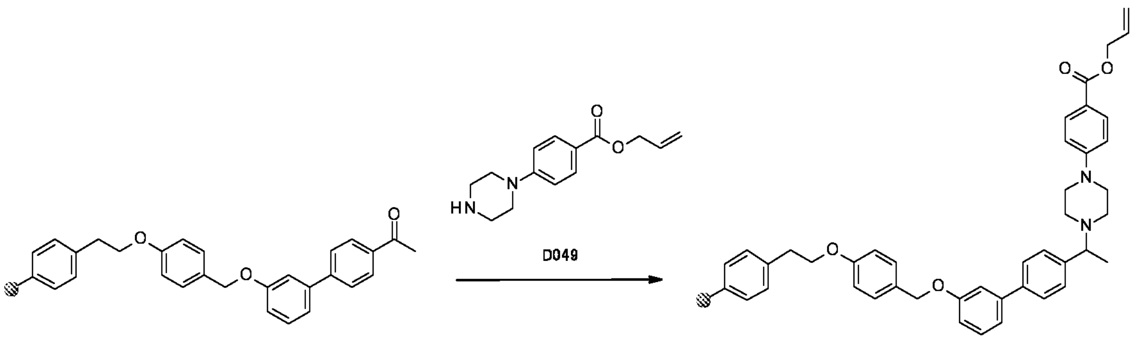


D047-02R D050-02R

Under a nitrogen atmosphere, solid-phase supported ketone (compound D047-02R, 100 mg, loading rate: 0.532 mmol/g, 0.0532 mmol) and anisole (3.75 mL) were added to a 3 mL glass vial and shaken at room temperature for 1 hour. Under a nitrogen atmosphere, allyl 4-piperazin-1-ylbenzoate (D049, 157 mg, 0.638 mmol) and $Ti(OtBu)_4$ (0.411 mL, 1.06 mmol) were added thereto, and the mixture was shaken at 140°C for 19 hours. The reaction solution was cooled to room temperature. Then, under a nitrogen atmosphere, sodium triacetoxyborohydride (338 mg, 1.60 mmol) was added thereto, and the mixture was shaken at 140°C for 17 hours. The reaction container was cooled to room temperature, and MeOH (2 mL) was added to the reaction solution. The obtained reaction solution and the resin suspension were transferred to a syringe with a filter using MeOH(2 mL) and repetitively washed three times with NMP (2 mL), three times with a 0.05 M Rochelle salt solution (NMP:water = 1:1, 2 mL), three times with water (2 mL), three times with NMP (2 mL), three times with a half-saturated $NaHCO_3$ solution (2 mL), three times with water (2 mL), three times with NMP (2 mL), three times with MeOH (2 mL), three times with DCM (2 mL), and three times with heptane (2 mL), and the resin was then dried under reduced pressure to obtain the title compound D050-02R (103.2 mg). The same cleavage operation as in Example 6-12-5 was performed, so that the formation of D050 was confirmed.

[Formula 583]

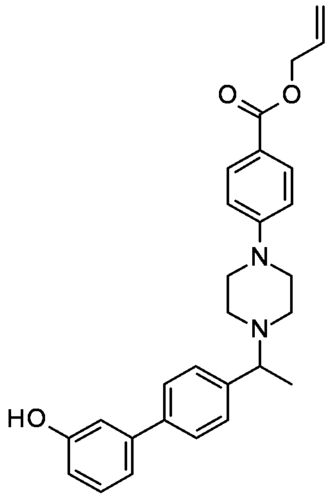

D050

Compound D050

LCMS: m/z 443[M+H]+.

Retention time: 0.797 min (analysis conditions SMD-FA05-1, 290 nm).

Example 6-12-8: Synthesis of solid-phase supported allyl ester (compound D051- 02R)

[Formula 584]

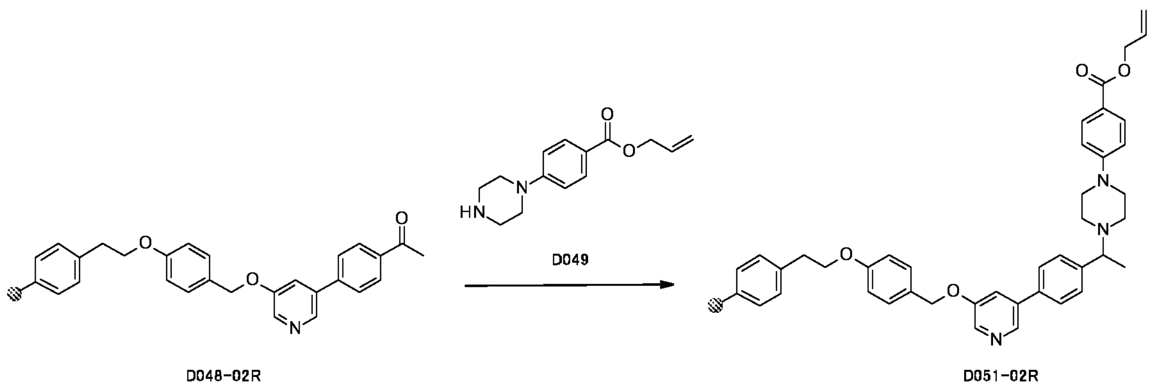


The title compound D051-02R (105.8 mg) was obtained by synthesis in the same manner as in Example 6-12-7 (compound D050-02R) using solid-phase supported ketone (compound D048-02R, loading rate: 0.735 mmol/g, 100 mg). The same cleavage operation as in Example 6-12-5 was performed, so that the formation of D051 was confirmed.

[Formula 585]

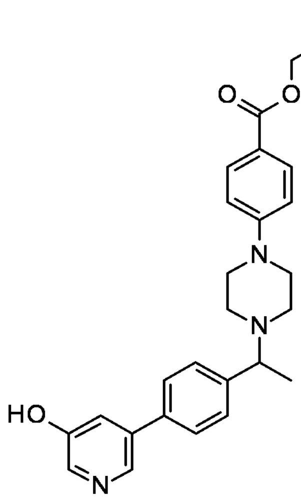

D051

Compound D051

LCMS: m/z 444$[M+H]^+$.

Retention time: 0.610 min (analysis conditions SMD-FA05-1, 290 nm).

Example 6-12-9: Synthesis of solid-phase supported carboxylic acid (compound D052-02R)

[Formula 586]

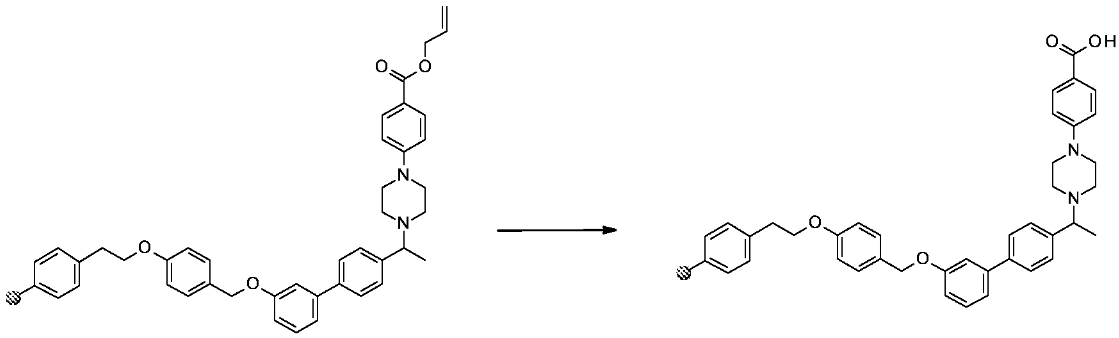

D050-02R D052-02R

Under a nitrogen atmosphere, solid-phase supported allyl ester (compound D050- 02R, 99.4 mg, loading rate: 0.532 mmol/g, 0.0532 mmol), NMP (1.2 mL), and MeOH (0.30 mL) were added to a 3 mL glass vial and shaken at room temperature for 1 hour. A 2 M aqueous sodium hydroxide solution (250 μL, 0.660 mmol) was added thereto, and the mixture was shaken at 80°C for 7 hours. The obtained reaction solution and the resin suspension were transferred to a syringe with a filter using NMP (2 mL) and repetitively washed three times with NMP (2 mL), three times with water (2 mL), three times with NMP (2 mL), three times with a solution of HOAt in NMP (0.2 M, 2 mL), three times with NMP (2 mL), three times with MeOH (2 mL), three times with DCM (2 mL), and three times with heptane (2 mL), and the resin was then dried under reduced pressure to obtain the title compound D052-02R (82.6 mg). The same cleavage operation as in Example 6-12-5 was performed, so that the formation of D052 was confirmed.

[Formula 587]

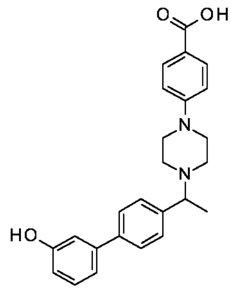

D052

Compound D052

LCMS: m/z 403[M+H]+.

Retention time: 0.649 min (analysis conditions SMD-FA05-1, 290 nm).

Example 6-12-10: Synthesis of solid-phase supported carboxylic acid (compound D053-02R)

[Formula 588]

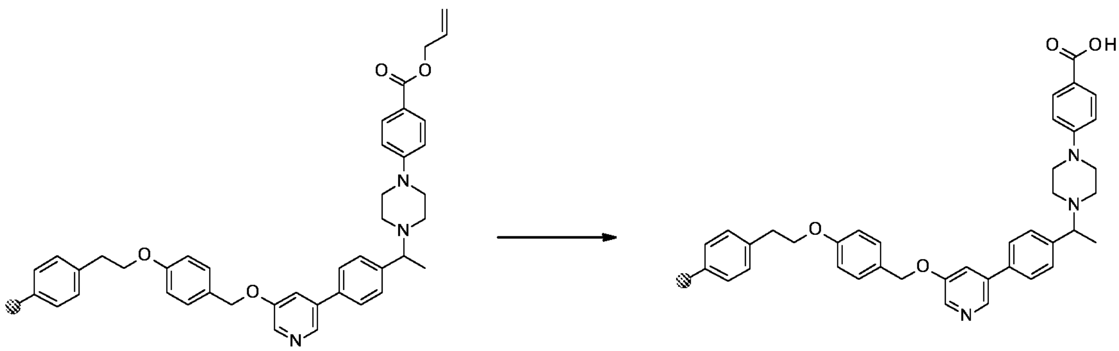

D051-02R D053-02R

Under a nitrogen atmosphere, solid-phase supported allyl ester (compound D051- 02R, 100.2 mg, loading rate: 0.736 mmol/g, 0.0737 mmol), NMP (1.2 mL), and MeOH (0.30 mL) were added to a 3 mL glass vial and shaken at room temperature for 1 hour. A 2 M aqueous sodium hydroxide solution (250 μL, 0.660 mmol) was added thereto, and the mixture was shaken at room temperature for 7 hours. Under a nitrogen atmosphere, a 2 M aqueous sodium hydroxide solution (250 μL, 0.660 mmol) was added to the reaction solution, and the mixture was shaken at room temperature for 11 hours. The obtained reaction solution and the resin suspension were transferred to a syringe with a filter using NMP (2 mL) and repetitively washed three times with NMP (2 mL), three times with water (2 mL), three times with NMP (2 mL), three times with a solution of HOAt in NMP (0.2 M, 2 mL), three times with NMP (2 mL), three times with MeOH (2 mL), three times with DCM (2 mL), and three times with heptane (2 mL), and the resin was then dried under reduced pressure to obtain the title compound D053-02R (90.1 mg). The same cleavage operation as in Example 6-12-5 was performed, so that the formation of D053 was confirmed.

[Formula 589]

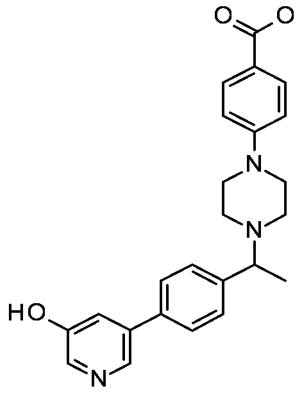

D053

Compound D053

LCMS: m/z 404[M+H]+.

Retention time: 0.457(analysis conditions SMD-FA05-1, 290 nm).

Example 6-12-11: Synthesis of compound D055-02R

[Formula 590]

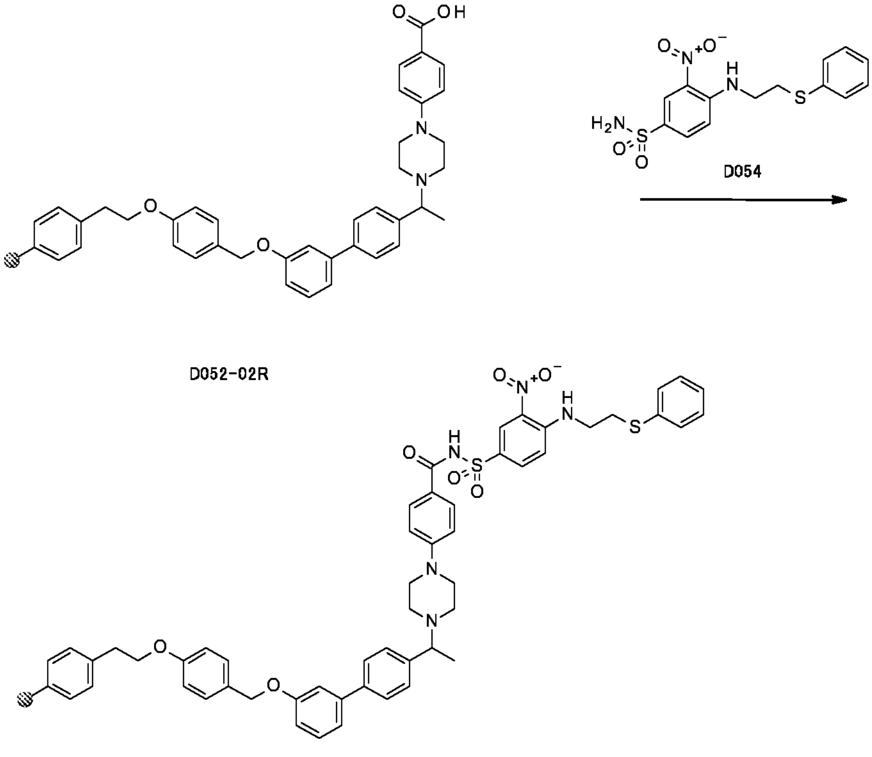

D055-02R

Under a nitrogen atmosphere, solid-phase supported carboxylic acid (compound D052-02R, 81.5 mg, loading rate: 0.532 mmol/g, 0.0434 mmol) and NMP (1.8 mL) were added to a 3 mL glass vial and shaken at room temperature for 1 hour. HATU (99.0 mg, 0.260 mmol) and 2,6-lutidine (30.3 μL, 0.260 mmol) were added thereto at room temperature, and the mixture was shaken at 40°C for 3 hours. 3-Nitro-4-(2- phenylsulfanylethylamino)benzenesulfonamide (D054, 107 mg, 0.304 mmol) and P1-tBu (165 μL, 0.650 mmol) were added thereto at room temperature, and the mixture was shaken at 40°C for 3 hours. The obtained reaction solution and the resin suspension were transferred to a syringe with

a filter using NMP (2 mL), repetitively washed twice with NMP (2 mL), three times with a solution of HOAt in NMP (0.2 M, 2 mL), three times with NMP (2 mL), three times with MeOH (2 mL), three times with DCM (2 mL), and three times with heptane (2 mL), and then dried under reduced pressure to obtain the title compound D055-02R (82.4 mg). The same cleavage operation as in Example 6-12-5 was performed, so that the formation of D055 was confirmed.

[Formula 591]

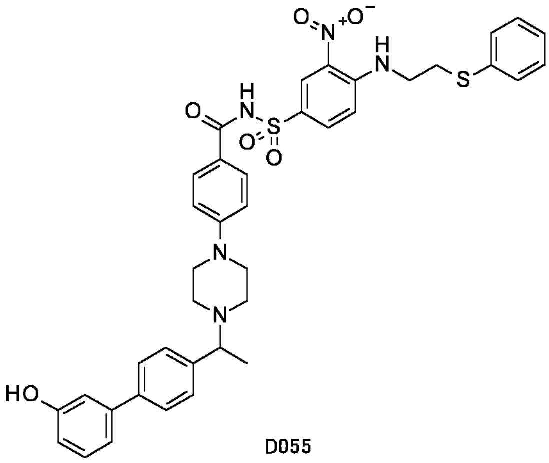

D055

Compound D055

LCMS: m/z 738[M+H]+.

Retention time: 0.932 min (analysis conditions SMD-FA05-1, 290 nm).

Example 6-12-12: Synthesis of compound D056-02R

[Formula 592]

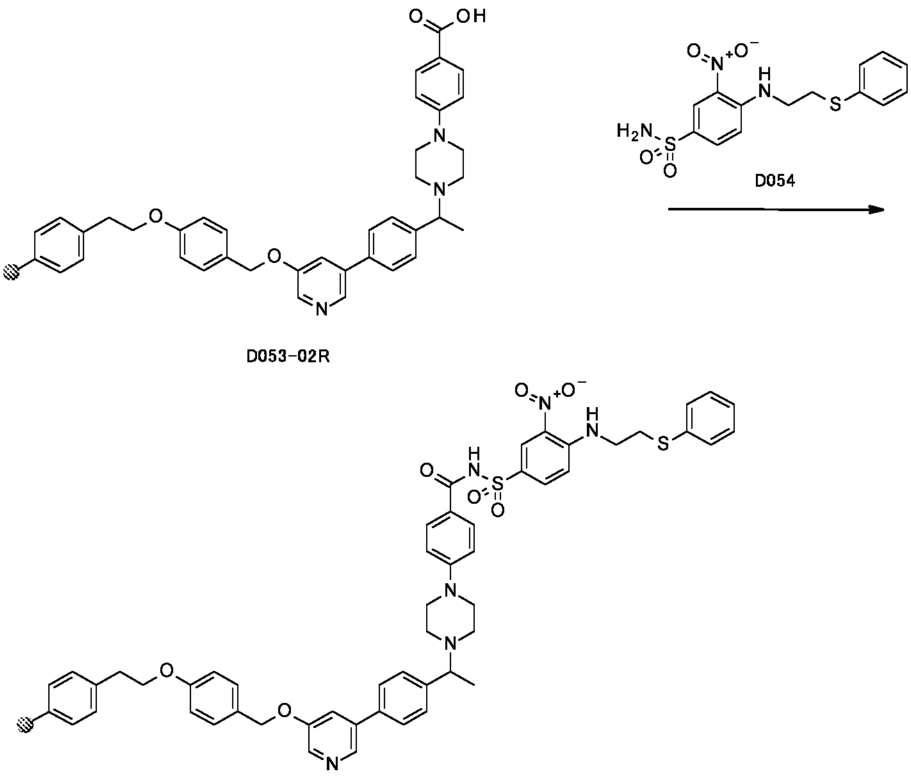

D053-02R

D054

D056-02R

The title compound D056-02R (73.4 mg) was obtained by synthesis in the same manner as in Example 6-12-11 (compound D055-02R) using solid-phase supported carboxylic acid (compound D053-02R, loading rate: 0.735 mmol/g, 67.1 mg). The same cleavage operation as in Example 6-12-5 was performed, so that the formation of D056 was confirmed.

[Formula 593]

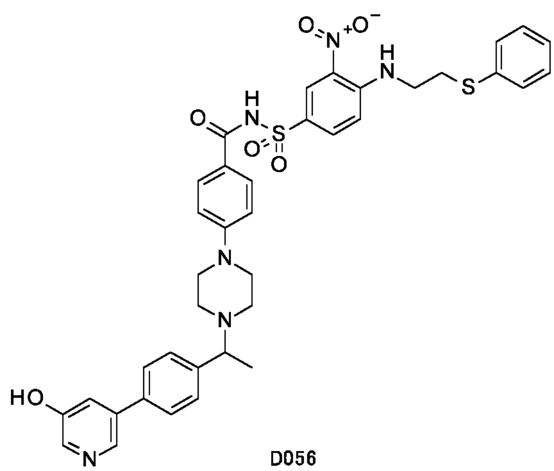

D056

Compound D056

LCMS: m/z 739$[M+H]^+$.

Retention time: 1.866 min (analysis conditions SMD-TFA05-long, 5 min, 290 nm).

Example 6-12-13: Synthesis of 4-[4-[1-[4-(3-hydroxyphenyl)phenyl]ethyl]piperazin- 1-yl]-N-[3-nitro-4-(2-phenylsulfanylethylamino)phenyl]sulfonylbenzamide (compound D055)

[Formula 594]

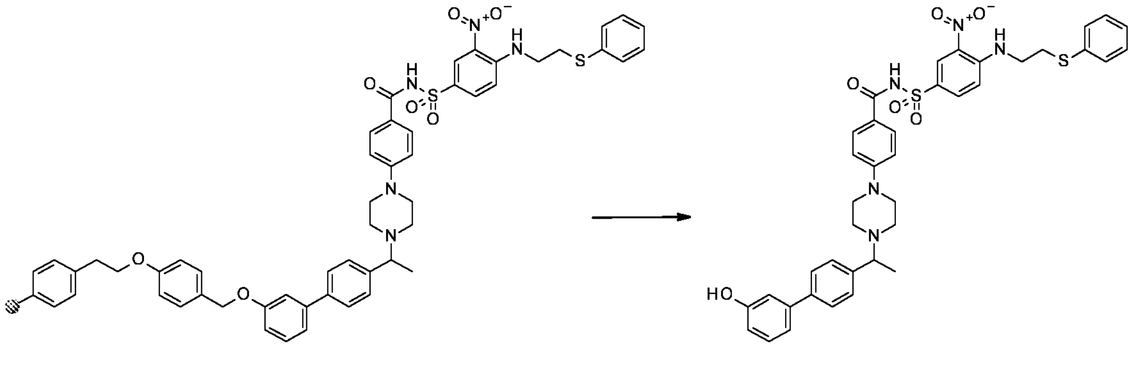

D055-02R D055

Under a nitrogen atmosphere, compound D055-02R (81.2 mg, loading rate: 0.532 mmol/g, 0.0432 mmol) and a solution of 1,2,3-trimethylbenzene in DCM (0.23 M, 1.14 mL) were added to a 3 mL glass vial and shaken at room temperature for 1 hour. TFA (487 μL, 6.32 mmol) and 1-bromo-4-iodobenzene (D057, 12.2 mg, 0.043 mmol) were added thereto, and the mixture was shaken at room temperature for 3 hours. The obtained reaction solution and the resin suspension were transferred to a syringe with a filter using DCM (0.8 mL) and filtered. The resin was washed with DCM (0.8 mL). DMSO (0.8 mL) and $H_2O$ (0.2 mL) were added to the obtained filtrate, and the mixture was concentrated under reduced pressure. The obtained residue was purified by reverse-phase column chromatography (C18, 0.1 % solution of TFA in acetonitrile/0.1% aqueous TFA solution, 0-100%) to obtain the title compound D055 (15.1 mg, 0.020 mmol, 47.4%) as a yellow solid.

Example 6-12-14: Synthesis of 4-[4-[1-[4-(5-hydroxypyridin-3- yl)phenyl]ethyl]piperazin-1-yl]-N-[3-nitro-4-(2- phenylsulfanylethylamino)phenyl]sulfonylbenzamide (compound D056)

[Formula 595]

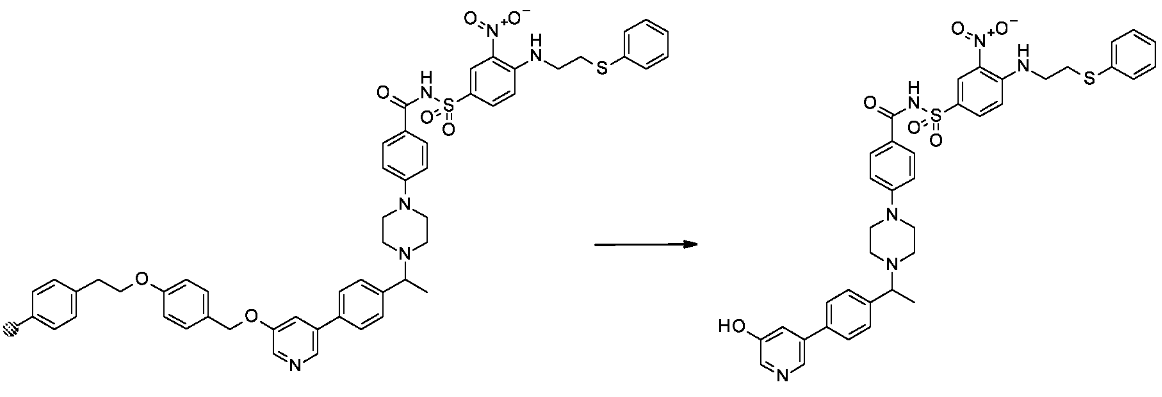

D056-02R D056

Under a nitrogen atmosphere, compound D056-02R (85.9 mg, loading rate: 0.735 mmol/g, 0.0631 mmol) and a solution of 1,2,3-trimethylbenzene in DCM (0.23 M, 1.20 mL) were added to a 3 mL glass vial and shaken at room temperature for 1 hour. TFA (515 μL, 6.69 mmol) and 1-fluoro-4-(4-fluorophenyl)benzene (D058, 12.0 mg, 0.063 mmol) were added thereto, and the mixture was shaken at room temperature for 3 hours. The obtained reaction solution and the resin suspension were transferred to a syringe with a filter using DCM (0.8 mL) and filtered. The resin was washed with DCM (0.8 mL). DMSO (0.8 mL) and water (0.2 mL) were added to the filtrate, and the mixture was concentrated under reduced pressure. The obtained residue was purified by reverse-phase column chromatography (C18, 0.1 % solution of TFA in acetonitrile/0.1% aqueous TFA solution, 0- 100%) to obtain the title compound D056 (13.4 mg, 0.0181 mmol, 28.7%) as a yellow solid.

Example 6-12-15: Synthesis of 4-[4-[1-[4-(3-ethoxyphenyl)phenyl]ethyl]piperazin- 1-yl]-N-[3-nitro-4-(2-phenylsulfanylethylamino)phenyl]sulfonylbenzamide [D059 (B2Q097- 01)]

[Formula 596]

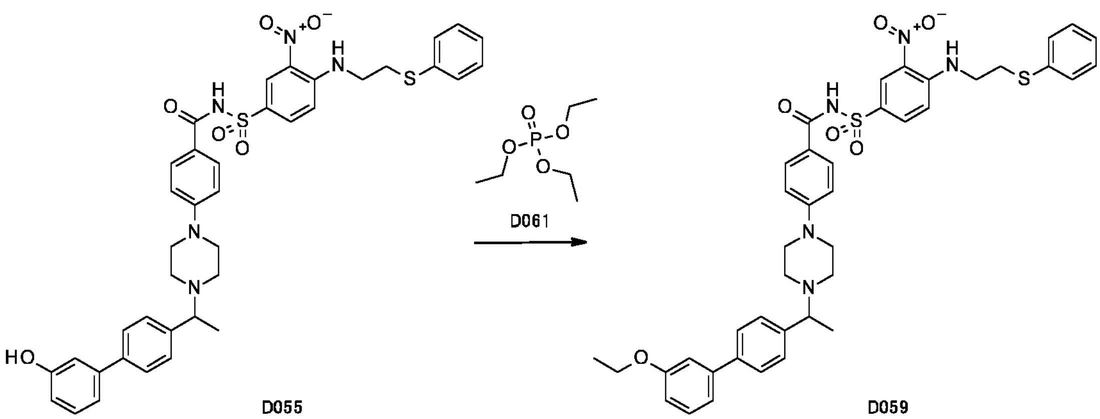


D055 D059

Under a nitrogen atmosphere, 4-[4-[1-[4-(3-hydroxyphenyl)phenyl]ethyl]piperazin- 1-yl]-N-[3-nitro-4-(2-phenylsulfanylethylamino)phenyl]sulfonylbenzamide (D055, 15.1 mg, 0.020 mmol) and DMI (0.29 mL) were added to a 1 mL screw-cap vial. BTPP (94.0 μL, 0.307 mmol) and ethyl phosphate (D061, 52.3 μL, 307 mmol) were added to the solution, and the mixture was stirred at 80°C for 30 hours. Formic acid (17.8 μL, 0.409 mmol) was added to the obtained residue, and the mixture was purified by reverse-phase column chromatography (C18, 0.1% solution of formic acid in acetonitrile/0.1% aqueous formic acid solution, 0-80%) to obtain the title compound D059 (2.0 mg, 2.6 μmol, 13%) as a yellow solid.

[Formula 597]

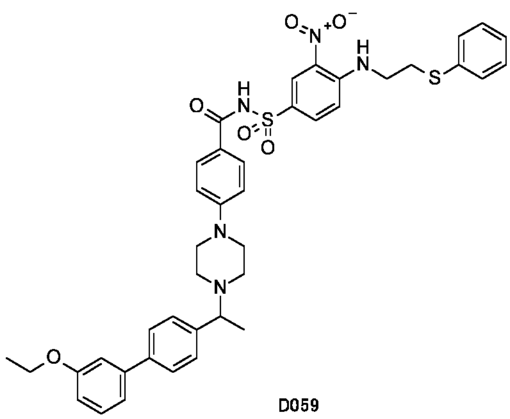

D059

Compound D059

$^1$H-NMR (400 MHz, $CD_2c1_2$) δ 8.81 (d, J = 2.0 Hz, 1H), 8.63 (t, J = 5.2 Hz, 1H), 8.06 (dd, J = 9.2, 2.0 Hz, 1H), 7.64 (d, J = 8.8 Hz, 2H), 7.56 (d, J = 8.0 Hz, 2H), 7.41-7.39 (m, 4H), 7.35-7.22 (m, 4H), 7.16 (d, J = 7.6 Hz, 1H), 7.11 (t, J = 2.0 Hz, 1H), 6.88-6.81 (m, 4H), 4.08 (q, J = 7.2 Hz, 2H), 3.59 (q, J = 6.4 Hz, 2H), 3.50-3.45 (m, 1H), 3.32-3.31 (m, 2H), 3.23 (t, J = 6.4 Hz, 2H), 3.09-3.04 (m, 2H), 2.68-2.64 (m, 2H), 2.58-2.52 (m, 2H), 1.44-1.40 (m, 6H).

LCMS: m/z 766 $[M+H]^+$.

Retention time: 1.044 min (analysis conditions SMD-FA05-1, 290 nm).

Example 6-12-16: Synthesis of 4-[4-[1-[4-(5-ethoxypyridin-3- yl)phenyl]ethyl]piperazin-1-yl]-N-[3-nitro-4-(2- phenylsulfanylethylamino)phenyl]sulfonylbenzamide [D060 (B2Q098-01)]

[Formula 598]

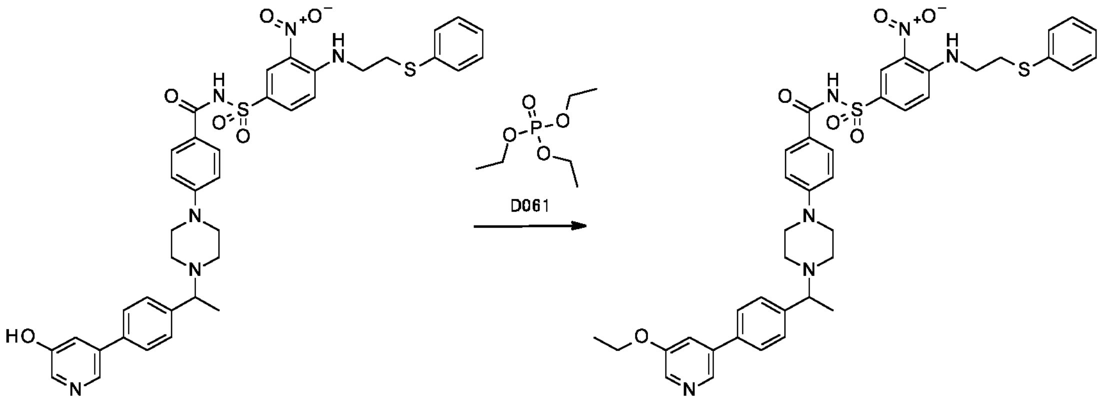


D056 D060

Under a nitrogen atmosphere, 4-[4-[1-[4-(5-hydroxypyridin-3- yl)phenyl]ethyl]piperazin-1-yl]-N-[3-nitro-4-(2-phenylsulfanylethylamino)phenyl]sulfonylbenzamide (D056, 13.4 mg, 0.018 mmol) and DMI (0.26 mL) were added to a 1 mL screw-cap vial. BTPP (83.0 μL, 0.272 mmol) and ethyl phosphate (D061, 46.4 μL, 272 mmol) were added to the solution, and the mixture was stirred at 80°C for 22 hours. Ammonium bicarbonate (28.7 mg, 0.363 mmol) was added to the obtained residue, and the mixture was purified by reverse-phase column chromatography (C18, acetonitrile/10 mM aqueous ammonium bicarbonate solution, 0-60%) to obtain the title compound D060 (6.0 mg, 7.8 μmol, 43%) as a yellow solid.

[Formula 599]

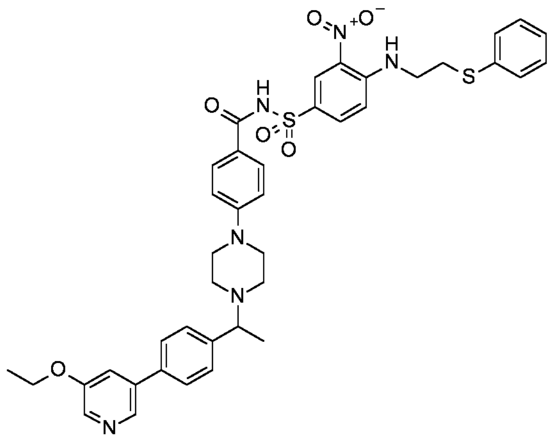

D060

Compound D060

$^1$H-NMR (400 MHz, $CD_2c1_2$) δ 8.69 (d, J = 2.0 Hz, 1H), 8.42 (d, J = 1.6 Hz, 1H), 8.36 (t, J = 5.6 Hz, 1H), 8.23 (d, J = 2.8 Hz, 1H), 8.03 (dd, J = 8.8, 2.0 Hz, 1H), 7.89 (d, J = 8.8 Hz, 2H), 7.56 (d, J = 8.4 Hz, 2H), 7.47-7.37 (m, 5H), 7.33-7.21 (m, 3H), 6.76 (d, J = 9.2 Hz, 2H), 6.76 (d, J = 9.2 Hz, 1H), 4.15 (q, J = 7.2 Hz, 2H), 3.53 (q, J = 6.8 Hz, 2H), 3.45 (q, J = 6.8 Hz, 1H), 3.26-3.18 (m, 2H), 3.13-3.09 (m, 2H), 2.68-2.63 (m, 2H), 2.56-2.51 (m, 2H), 1.81-1.78 (m, 2H), 1.45 (t, J = 6.8 Hz, 3H), 1.40 (d, J = 6.8 Hz, 3H).

LCMS: m/z 767 $[M+H]^+$.

Retention time: 2.020 min (analysis conditions SMD-TFA05-long, 290 nm).

Example 7: Evaluation of binding of compound to Bcl-2 by surface plasmon resonance (SPR)

A surface plasmon resonance method was used in order to analyze the binding affinity of the compounds synthesized in Example 1 and Example 6, which are shown in Table AK-01, for Bcl-2. The apparatus used was Biacore T200 (GE Healthcare Japan Corp.), and the running buffer used was HBS (10 mM HEPES-NaOH and 150 mM NaCl, pH 7.4) supplemented with 1 mM DTT, 3 mM EDTA, 0.01% Tween 20 and 4% DMSO. Human Bcl-2 protein (Novus Biologicals) was biotinylated using EZ-Link Sulfo-NHS-LC-LC-biotin (Thermo Fisher scientific Inc.) and immobilized on the surface of Sensor Chip SA (GE Healthcare Japan Corp.). Then, compound solutions having a plurality of concentrations and the running buffer (blank) were added to the Bcl-2-immobilized surface to obtain the binding response of the compounds. Measurement was performed at 15°C.

The obtained sensorgrams were treated with Biacore T200 Evaluation Software, and the equilibrium dissociation constant $K_D$ of each compound for Bcl-2 was determined as shown in the following Table AK-02 and Table AK-03.

[Table 286]

[Table AK-01] Structural formula of compound used in present Example

| Compound No. in Example 7 | Compound No. in Example 1 | Compound No. in Example 6 | Structure |
|---|---|---|---|
| B2Q045−01 | AG005 | — | 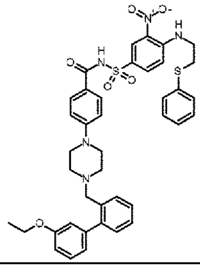 |
| B2Q046−01 | AG007 | — | 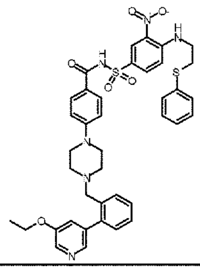 |
| B2R045−01 | AG012 | — | 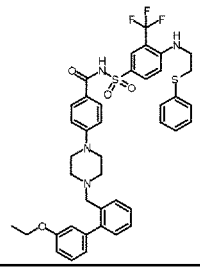 |
| B2R046−01 | AG013 | — | 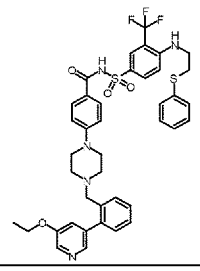 |
| B2Q047−01 | — | AG016 | 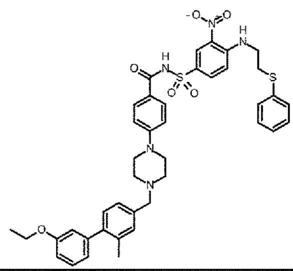 |

[Table 287]

| Compound No. in Example 7 | Compound No. in Example 1 | Compound No. in Example 6 | Structure |
|---|---|---|---|
| B2Q097−01 | — | D059 | 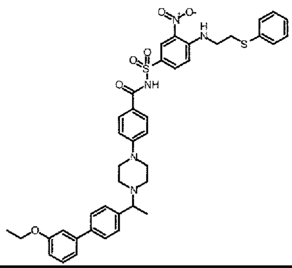 |
| B2Q048−01 | — | AG018 | 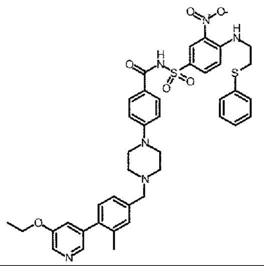 |
| B2Q098−01 | — | D060 | 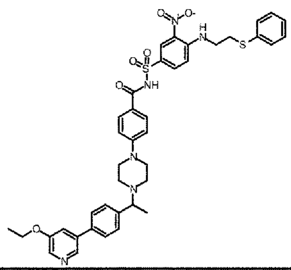 |
| B2Q053−01 | — | AG045 | 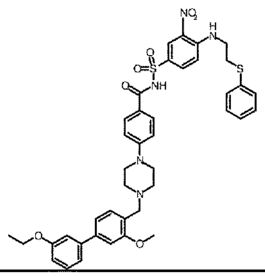 |
| B2Q054−01 | — | AG022 | 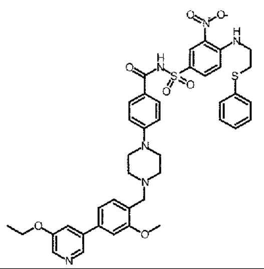 |

[Table 288]

| Compound No. in Example 7 | Compound No. in Example 1 | Compound No. in Example 6 | Structure |
|---|---|---|---|
| B2Q066−01 | — | AG025 | 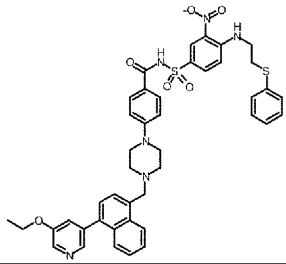 |
| B1A075−01 | — | AG038 | 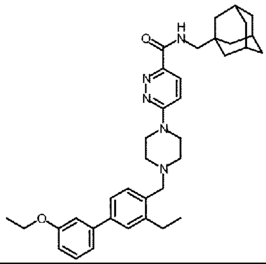 |
| B3A073−01 | — | AG042 | 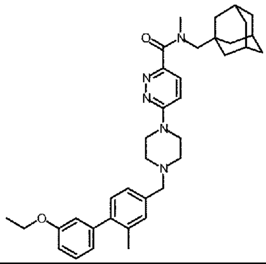 |
| B2R074−01 | — | AG052 | 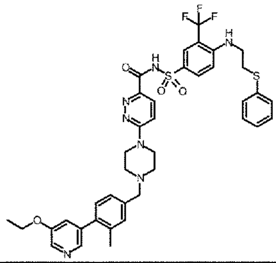 |
| B2Q012−01 | — | AG051 | 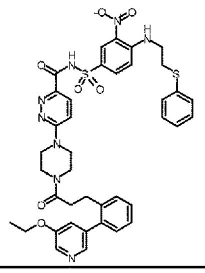 |

[Table 289]

| [Table AK-02] Equilibrium dissociation constant $K_D$ of compound synthesized before carrying out library synthesis | |
|---|---|
| Compound | $K_D$[μmol/L] |
| B2Q045-01 | 1.2 |
| B2Q046-01 | 8.1 |

(continued)

| [Table AK-02] Equilibrium dissociation constant $K_D$ of compound synthesized before carrying out library synthesis | |
|---|---|
| Compound | $K_D$[μmol/L] |
| B2R045-01 | Bound |
| B2R046-01 | Bound |

[Table 290]

| [Table AK-03] Equilibrium dissociation constant $K_D$ of compound found by AS-MS evaluation of synthesized library | | |
|---|---|---|
| Compound | Compositional formula | $K_D$[μmol/L] |
| B2Q047-01 | $C_{41}H_{43}N_5O_6S_2$ | 0.70 |
| B2Q097-01 | $C_{41}H_{43}N_5O_6S_2$ | Binding not observed |
| B2Q048-01 | $C_{40}H_{42}N_6O_6S_2$ | 0.48 |
| B2Q098-01 | $C_{40}H_{42}N_6O_6S_2$ | Binding not observed |
| B2Q053-01 | $C_{41}H_{43}N_5$O7S2 | Binding not observed |
| B2Q054-01 | $C_{40}H_{42}N_6O_7S_2$ | 1.27 |
| B2Q066-01 | $C_{43}H_{42}N_6O_6S_2$ | 0.95 |
| B1A075-01 | $C_{37}H_{47}N_5O_2$ | Binding not observed |
| B3A073-01 | $C_{37}H_{47}N_5O_2$ | Binding not observed |
| B2R074-01 | $C_{39}H_{40}F_3N_7O_4S_2$ | Binding not observed |
| B2Q012-01 | $C_{39}H_{40}N_8O_7S_2$ | Bound |

As shown in Table AK-02 and Table AK-03, compounds (B2Q047-01, B2Q048-01, and B2Q066-01) were found to have a smaller equilibrium dissociation constant $K_D$ and more highly bind to Bcl-2 than compound B2Q045-01 evaluated beforehand for its ability to bind to Bcl-2.

A library that was searched in a wide range at once was newly synthesized by multiplying the "diversity of core blocks" and the "diversity of linkers", and compounds that bound to Bcl-2 were identified by AS-MS (Example 5). Furthermore, the identified compounds were individually synthesized and found by SPR to specifically bind to Bcl-2. As shown in formula AK-01, in both the compounds B2Q047-01 and B2Q048-01 confirmed to bind by SPR, the substitution pattern of the sp3 carbon atom bonded to a piperazine ring and the molecular terminal aromatic ring is in the relationship of p-substitution. Taking a look at the structures of known Bcl-2 inhibitors, 971 compounds have been reported as compounds in the relationship of o-substitution as shown in formula AK-02 whereas only two cases have been reported as p-substituted compounds (results of substructure search using Reaxysis(R) as of November 5, 2020). No report has been confirmed on information on the activity of any p-substituted compound. In short, although not wishing to be bound by any specific theory and presumption, p-substituted compounds may have been recognized as a derivative group whose activity is attenuated in the course of derivatization, and a wide range of derivatization has not been carried out later.

X ray crystal structural information (PDB ID: 6QGG, Figure 3) on an o-substituted form (ABT-737 analogue) and Bcl-2 reported in a literature known in the art (ACS Omega 2019, 4, 5, 8892-8906) does not lead to the idea of introducing a substituent in the p-direction to sp3 carbon bonded to a nitrogen atom of a piperazine ring, and furthermore, shows that the introduction of a substituent in the p-direction may become an inhibitory factor for the purpose of improving activity. Figure 3 shows one example of information on the complex of a compound in the relationship of o-substitution and Bcl family protein and illustrates the binding pattern of the compound in the relationship of o-substitution, though the present invention is not limited by this example.

[Formula 600]

[Formula AK-01]

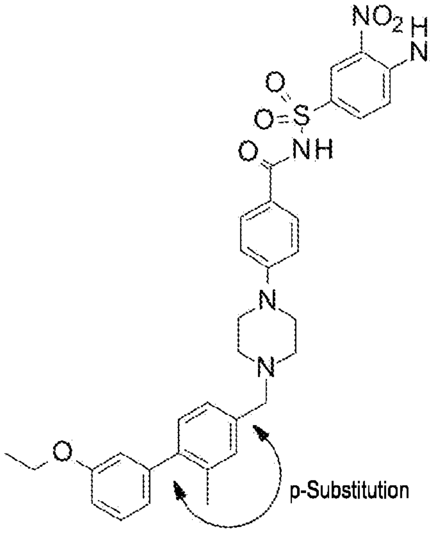

B2Q47-01

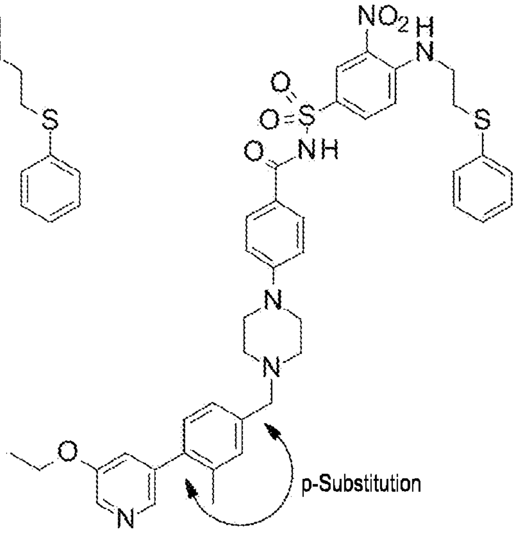

B2Q48-01

[Formula 601]

[Formula AK-02]

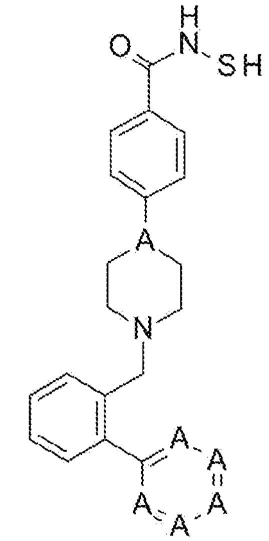

Search formula of o-substituted form

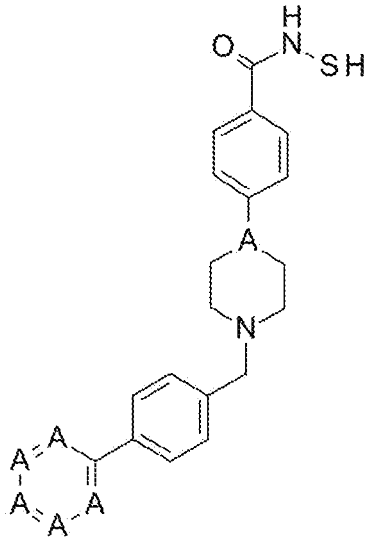

Search formula of p-substituted form

In spite of the presence of such known information, compounds B2Q048-01 ($K_D$ = 0.48 μM), B2Q047-01 ($K_D$ = 0.70 μM), and B2Q066-01 ($K_D$ = 0.95 μM) having higher ability to bind than that of o-substituted forms B2Q045-01 ($K_D$ = 1.2 μM) and B2Q046-01 ($K_D$ = 8.1 μM) were identified by the synthesis of a library searched in a wide range at once by multiplying the "diversity of core blocks", the "diversity of linkers", and also the diversity of substitution patterns (here, o-, m-, and p-) on the core blocks, as carried out in this Example. This indicates the usefulness of the present invention. As shown in Table AK-03, the ability to bind strongly or weakly brought about by difference in the aromatic ring at a molecular terminal moiety between an o--substituted form and a p--substituted form was inverted, and these results also indicate the usefulness of the synthesis of a library searched in a wide range at once.

[Table 291]

[Table AK-04]

| Common structure | 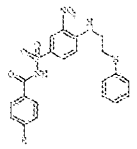 | | | |
|---|---|---|---|---|
| Substitution pattern | o-substituted form | | p-substituted form | |
| Substructure R | 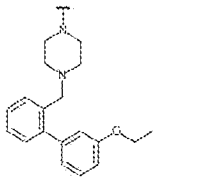 | 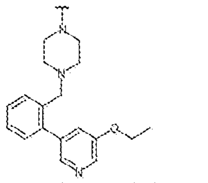 | 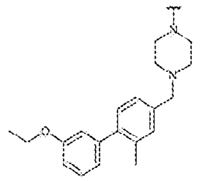 | 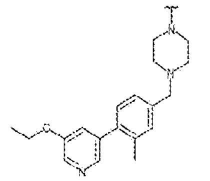 |
| Molecular terminal aromatic ring | Benzene | Pyridine | Benzene | Pyridine |
| Compound No. | B2Q045-01 | B2Q046-01 | B2Q047-01 | B2Q048-01 |
| $K_D$ in SPR experiment (μm) | 1.2 | 8.1 | 0.70 | 0.48 |
| Substructure R | — | — | 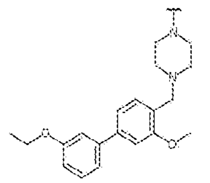 | 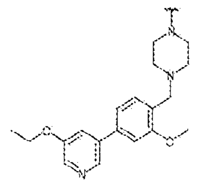 |
| Molecular terminal aromatic ring | — | — | Benzene | Pyridine |
| Compound No. | — | — | B2Q053-01 | B2Q054-01 |
| $K_D$ in SPR experiment (μm) | — | — | Binding not observed | 1.27 |

In search for new compounds aimed at developing medicaments, a compound is generally derivatized while the scaffold structure of the compounds is fixed on a given structure. In other words, the scaffold is fixed, and the other moieties are generally optimized. The search approaches used in many reported library techniques also utilize such manner. Scaffold is same among such library.

Hence, although not only combinations of core blocks but combinations of types of linkers that link the core blocks cover a wide scope in a matrical manner, derivatization is carried out by a method that has the difficulty in searching all of them. As a result, such an approach is very likely to miss a better compound (in this context, the term "better" specifically refers to, for example, the ability to bind, selectivity for a target protein, or physical properties such as lipophilicity or hydrophilicity) that may fall outside the range where limited combinations are searched. The approach of the present invention was found capable of searching diverse compounds at once by multiplying the diversity of core blocks and the diversity of linkers and further combining different substitution patterns, and capable of testing effects of compounds that may fall outside a range searched by conventional approaches. This approach is one aspect to solve challenges required for the drug discovery of hit generation, scaffold hopping and FBDD (fragment-based drug discovery), and the value of the present invention was demonstrated.

Example 8; Practice example of compound library synthesis for AS-MS - 2

In Example 4, 1,200 compounds were set as library compounds from among diverse compounds in which the "diversity of core blocks" that formed pharmacophores and the "diversity of linkers" were multiplied and further, different substitution patterns were combined, by exploiting the findings obtained in Example 3, to carry out library synthesis. After identification of compounds that bound to Bcl-2 by AS-MS, the identified compounds were individually synthesized and found by SPR to specifically bind to Bcl-2. This approach was found capable of testing effects of compounds that may fall outside a range searched by conventional approaches and showed one aspect to solve challenges required for the drug discovery of hit generation, scaffold hopping and FBDD (fragment-based drug discovery).

This Example and Example 9 illustrate an approach of obtaining a new binding compound by setting a library in which the combination of the "diversity of core blocks", the "diversity of linkers", and the "diversity of substitution patterns" is incorporated in a role in linking two pharmacophores. Specifically, as shown in formulas 8-01 and 8-02, these examples illustrate a scaffold hopping approach of mutually combining substructures of two compound classes that exhibit the ability to bind to Bcl family protein to prepare a library of the resulting structures, and obtaining a new binding compound from the hybrid compounds.

In Example 8, a hybrid library of moieties that corresponded to pharmacophore 1 of cpd 22 (ACS Med. Chem. Lett. 2014, 5, 6, 662-667) and moieties of pharmacophore 2 of ABT-737 (J. Med. Chem. 2007, 50, 4, 641-662, SPR Kd value; and ACS Med. Chem. Lett. 2013, 4, 2, 186-190) was planned and implemented. In Example 9, a hybrid library of moieties that corresponded to pharmacophores 1 of cpd 6a and 6b (in the relationship of enantiomers to each other, ACS Med. Chem. Lett. 2014, 5, 6, 662-667) and moieties of pharmacophore 2 of ABT-737 (J. Med. Chem. 2007, 50, 4, 641-662, SPR Kd value; and ACS Med. Chem. Lett. 2013, 4, 2, 186-190) was planned and implemented.

[Formula 602]

[Formula 08-01]

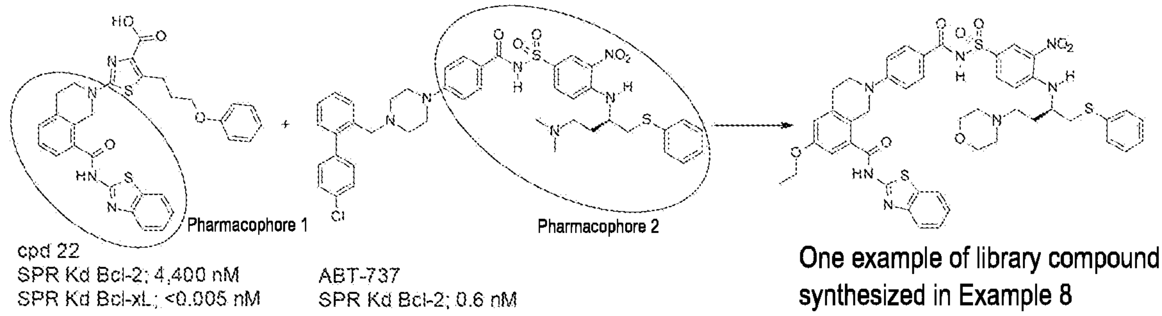


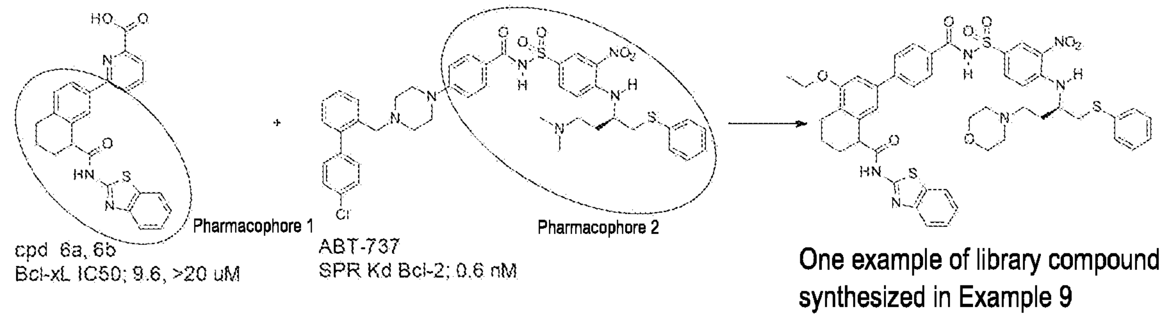


Example 8 shows an example in which a library is constructed by carrying out the reaction of a mixture containing 1000 compounds.

Figure 4 shows the "diversity of core blocks" and "diversity of linkers" involved in the library implemented in Example 8. Figure 5 shows a library synthesis route by split & mix. In Example 8, mixture numbers may be used to indicate synthesized mixtures. For example, "2-1" in "2-1-a1B01-0" represents the type of a library, and "a1B01" represents the type of a mixture. "-0" represents a state supported on the solid phase, and "-1" represents a state cleaved from the solid phase. Also, "-2" represents a state where functional group conversion was performed after cleavage from the solid phase. ID may be used to indicate each compound contained in a mixture. For example, "0001" in ID "2-1-a1B01-0-0001" is a number that defines one arbitrary compound contained in the mixture "2-1-a1B01-0", and the mixture and the contained compounds are defined by ID. In Example 8, core block A in Figure 4 has a structure represented by A01.

In the library synthesis given below, the building blocks shown in Table 8-0 were used.

[Table 292]

[Table 8-0] Building block used in Example 8

| Compound No. | Structural formula | Compound name |
|---|---|---|
| BB2−1−01 | 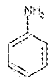 | Aniline |
| BB2−1−02 | 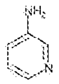 | Pyridin-3-amine |
| BB2−1−03 | 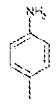 | 4-Methylaniline |
| BB2−1−04 | 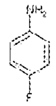 | 4-Fluoroaniline |
| BB2−1−05 | 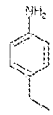 | 4-Ethylaniline |
| BB2−1−06 | 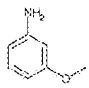 | 3-Methoxyaniline |
| BB2−1−07 | 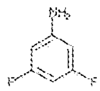 | 3,5-Difluoroaniline |
| BB2−1−08 | 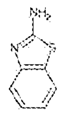 | 2-Aminobenzothiazole |
| BB2−1−09 | 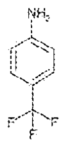 | 4-(Trifluoromethyl)aniline |
| BB2−1−10 | 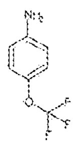 | 4-(Trifluoromethoxy)aniline |

| | | |
|---|---|---|
| BB2−1−11 | 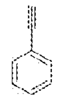 | Ethynylbenzene |
| BB2−1−12 | 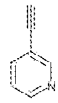 | 3-Ethynylpyridine |
| BB2−1−13 | 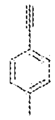 | 1-Ethynyl-4-methylbenzene |
| BB2−1−14 | 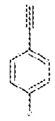 | 1-Ethynyl-4-fluorobenzene |
| BB2−1−15 | 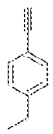 | 1-Ethyl-4-ethynylbenzene |
| BB2−1−16 | 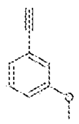 | 1-Ethynyl-3-Methoxybenzene |
| BB2−1−17 | 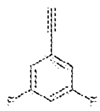 | 1-Ethynyl-3,5-difluorobenzene |
| BB2−1−18 | 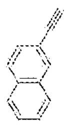 | 2-Ethynylnaphthalene |
| BB2−1−19 | 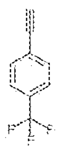 | 1-Ethynyl-4-(trifluoromethyl)benzene |
| BB2−1−20 | 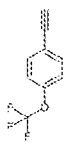 | 1-Ethynyl-4-(trifluoromethoxy)benzene |

| | | |
|---|---|---|
| BB2−1−21 | 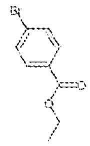 | Ethyl 4-bromobenzoate |
| BB2−1−22 | 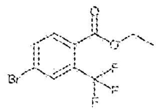 | Ethyl 4-bromo-2-(trifluoromethyl)benzoate |
| BB2−1−23 | 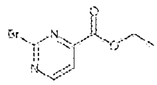 | Ethyl 2-bromopyrimidine-4-carboxylate |
| BB2−1−24 | 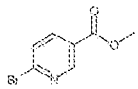 | Methyl 6-bromopyridine-3-carboxylate |
| BB2−1−25 | 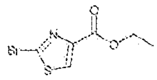 | Ethyl 2-bromo-1,3-thiazole-4-carboxylate |
| BB2−1−26 | 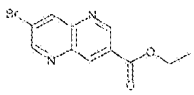 | Ethyl 7-bromo-1,5-naphthyridine-3-carboxylate |
| BB2−1−27 | 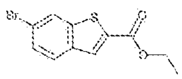 | Ethyl 6-bromo-1-benzothiophene-2-carboxylate |
| BB2−1−28 | 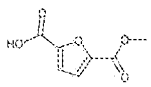 | 5-Methoxycarbonylfuran-2-carboxylic acid |
| BB2−1−29 | 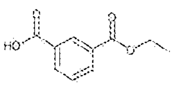 | 3-Ethoxycarbonylbenzoic acid |
| BB2−1−30 | 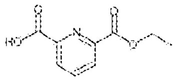 | 6-Ethoxycarbonylpyridine-2-carboxylic acid |

| | | |
|---|---|---|
| BB2−1−31 | 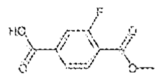 | 3-Fluoro-4-methoxycarbonylbenzoic acid |
| BB2−1−32 | 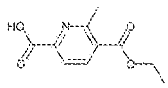 | 5-Ethoxycarbonyl-6-methylpyridine-2-carboxylic acid |
| BB2−1−33 | 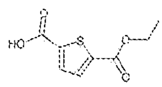 | 5-Ethoxycarbonylthiophene-2-carboxylic acid |
| BB2−1−34 | 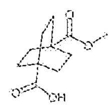 | 4-Methoxycarbonylbicyclo[2.2.2]octane-1-carboxylic acid |
| BB2−1−35 | 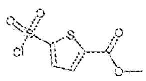 | Methyl 5-chlorosulfonylthiophene-2-carboxylate |
| BB2−1−36 | 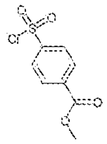 | Methyl 4-chlorosulfonylbenzoate |
| BB2−1−37 | 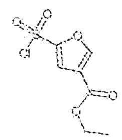 | Ethyl 5-chlorosulfonylfuran-3-carboxylate |
| BB2−1−38 | 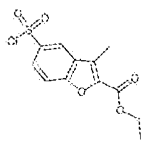 | Ethyl 5-chlorosulfonyl-3-methyl-1-benzofuran-2-carboxylate |
| BB2−1−39 | 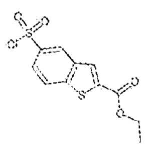 | Ethyl 5-chlorosulfonyl-1-benzothiophene-2-carboxylate |
| BB2−1−40 | 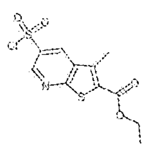 | Ethyl 5-chlorosulfonyl-3-methylthieno[2,3-b]pyridine-2-carboxylate |

| | | |
|---|---|---|
| BB2−1−41 | 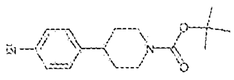 | tert-Butyl 4-(4-bromophenyl)piperidine-1-carboxylate |
| BB2−1−42 | 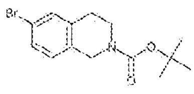 | tert-Butyl 6-bromo-3,4-dihydro-1H-isoquinoline-2-carboxylate |
| BB2−1−43 | 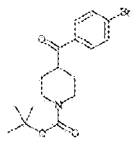 | tert-Butyl 4-(4-bromobenzoyl)piperidine-1-carboxylate |
| BB2−1−44 | 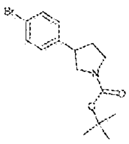 | tert-Butyl 3-(4-bromophenyl)pyrrolidine-1-carboxylate |
| BB2−1−45 | 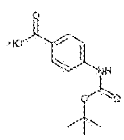 | 4-[(2-Methylpropan-2-yl)oxycarbonylamino]benzoic |
| BB2−1−46 | 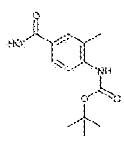 | 3-Methyl-4-[(2-methylpropan-2-yl)oxycarbonylamino] benzoic acid |
| BB2−1−47 | 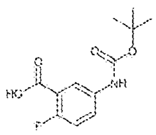 | 2-Fluoro-5-[(2-methylpropan-2-yl)oxycarbonylamino] benzoic acid |
| BB2−1−48 | 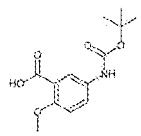 | 2-Methoxy-5-[(2-methylpropan-2-yl)oxycarbonylamino] |
| BB2−1−49 | 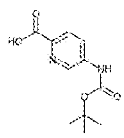 | 5-[(2-Methylpropan-2-yl)oxycarbonylamino]pyridine-2-carboxylic acid |
| BB2−1−50 | 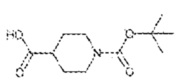 | 1-[(2-Methylpropan-2-yl)oxycarbonyl]piperidine-4-carboxylic acid |

| | | |
|---|---|---|
| BB2−1−51 | 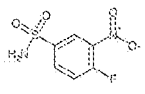 | 4-Fluoro-3-nitrobenzenesulfonamide |
| BB2−1−52 | 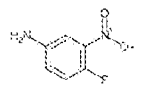 | 4-Fluoro-3-nitroaniline |
| BB2−1−53 | 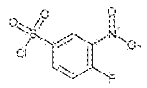 | 4-Fluoro-3-nitrobenzenesulfonyl chloride |
| BB2−1−54 | 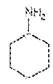 | Cyclohexylamine |
| BB2−1−55 | 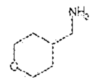 | 4-Aminomethyltetrahydropyran |
| BB2−1−56 | 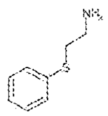 | 2-(Phenylthio)ethanamine |
| BB2−1−57 | 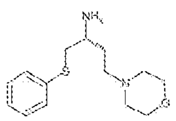 | (R)-4-Morpholino-1-(phenylthio)-2-butanamine |

Example 8-1-1: Formation of linker a1 by step a1

A reaction formula in step a1 performed using BB2-1-01 will be illustrated below.

[Formula 603]

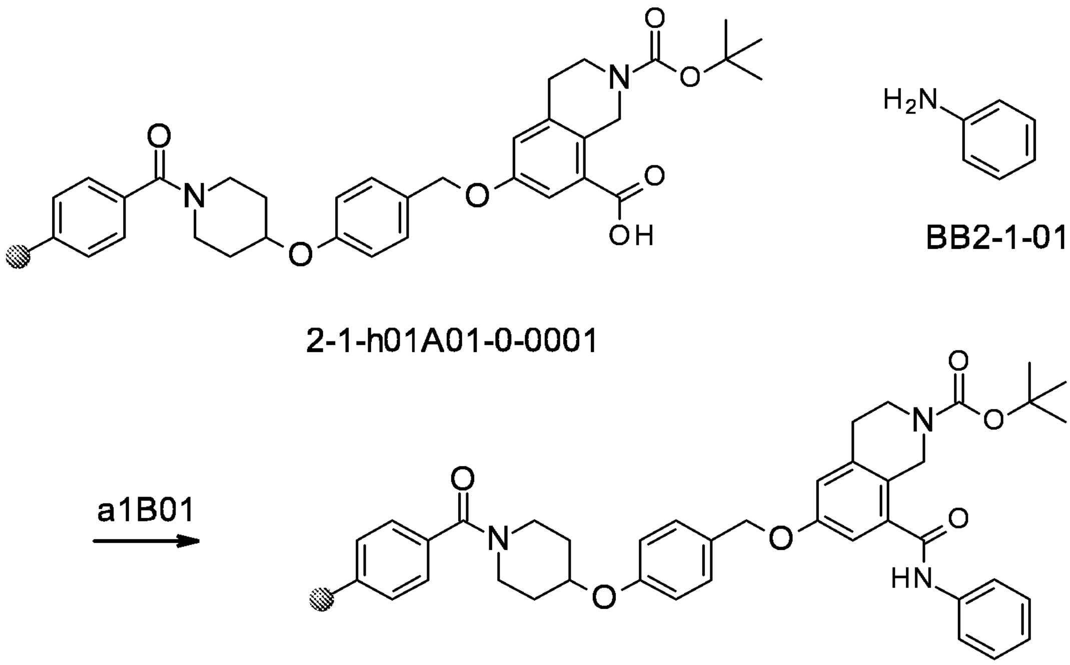


2-1-h01A01-0-0001

BB2-1-01

2-1-a1B01-0-0001

Compounds 2-1-a1B01-0-0001 to 2-1-a1B10-0-0001 were synthesized as follows using BB2-1-01 to BB2-1-10.
Compound 2-1-h01A01-0-0001 (described as D166-03R in Example 1) (240 mg, 0.201 mmol/g) and DCM (15.4 mL) were added to ten 4 mL glass vials. Separate anilines (0.338 mmol) described in Table 8-1-1 were added to the glass vials, respectively, which were then capped so as not to leak the reaction solution, and shaken at room temperature for 1 hour. A solution of PipClU (0.145 mmol) and NMI (0.145 mmol) in MeCN (0.145 mL) (1 M each of PipClU and NMI) was added thereto, and each mixture was shaken at room temperature for 3.5 hours.

Solid-phase purification

The suspension of the reaction solution and the resin was filtered through the filter of the column and washed three times with NMP/water = 1/1 (5 mL), three times with NMP (5 mL), three times with MeOH (5 mL), three times with DCM (5 mL), and three times with heptane (5 mL), and the obtained solid phase was dried under reduced pressure to obtain the compounds described in Table 8-1-1.

Reaction monitoring

Under a nitrogen atmosphere, a small amount of the resin thus washed was transferred to a pipette tip with a filter, washed three times with DMA (100 μL), three times with MeOH (100 μL), and three times with DCM (100 μL), and then dipped in a 10% solution of TFA in DCM containing 0.05 M pentamethylbenzene (50 μL) for 5 minutes. The cleavage solution was filtered, and the solid phase was washed with DMA (50 μL). The obtained filtrate was diluted with MeCN (100 μL) to prepare an LC sample. The formation of compound 2-1-a1B01-1-0001 was confirmed by LC-MS analysis.
[Table 293]

| [Table 8-1-1] Reagent used and obtained compound in step a1 | | | |
|---|---|---|---|
| Aniline reagent used | Amount used | Molar number | Obtained compound |
| BB2-1-01 | 30.8 μL | 0.338 mmol | 2-1-a1B01-0-0001 |
| BB2-1-02 | 31.8 mg | 0.338 mmol | 2-1-a1B02-0-0001 |
| BB2-1-03 | 36.2 mg | 0.338 mmol | 2-1-a1B03-0-0001 |
| BB2-1-04 | 32.0 μL | 0.338 mmol | 2-1-a1B04-0-0001 |
| BB2-1-05 | 42.2 μL | 0.338 mmol | 2-1-a1B05-0-0001 |
| BB2-1-06 | 37.8 μL | 0.338 mmol | 2-1-a1B06-0-0001 |
| BB2-1-07 | 43.6 mg | 0.338 mmol | 2-1-a1B07-0-0001 |
| BB2-1-08 | 50.7 mg | 0.338 mmol | 2-1-a1B08-0-0001 |
| BB2-1-09 | 41.9 μL | 0.338 mmol | 2-1-a1B09-0-0001 |
| BB2-1-10 | 45.3 μL | 0.338 mmol | 2-1-a1B10-0-0001 |

The details of compound 2-1-a1B01-1-0001 to compound 2-1-a1B10-1-0001 are shown in Table 8-1-2.
A notation method in each table will be described. For example, in Table 8-1-2, each compound synthesized in step a1 is indicated by ID, and core blocks and linkers in combination in the structures of compound 2-1-a1B01-1-0001 to compound 2-1-a1B10-1- 0001 are indicated by symbols or chemical formulas. The correspondence between the symbols and specific structures are shown in Figure 4. In Table 8-1-2, "×" and "b" are represented by chemical formulas.

[Formula 604]

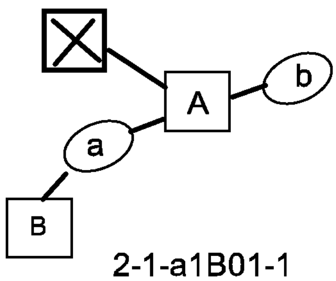

2-1-a1B01-1

For example, when ID is 2-1-a1B01-1-0001, the compound is represented by the following structural formula.

[Formula 605]

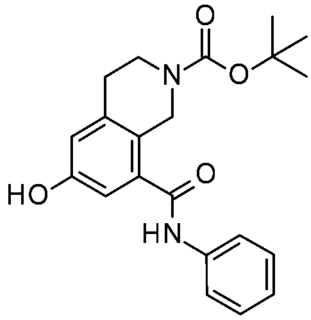

2-1-a1B01-1-0001

2-1-a1B01-1-0001

[Table 294]

| Exemplary notation in [Table 8-1-2] | | | | |
|---|---|---|---|---|
| ID | | a | a | b |
| 2-1-a1B01-1-0001 | OH | a1 | B01 | Boc |

[Table 295]

| [Table 8-1-2] Compound synthesized in step a1 | | | | | |
|---|---|---|---|---|---|
| ID | Exact Mass | | a | B | b |
| 2-1-a1B01-1-0001 | 368.174 | OH | a1 | B01 | Boc |
| 2-1-a1B02-1-0001 | 369.169 | OH | a1 | B02 | Boc |
| 2-1-a1B03-1-0001 | 382.189 | OH | a1 | B03 | Boc |
| 2-1-a1B04-1-0001 | 386.164 | OH | a1 | B04 | Boc |
| 2-1-a1B05-1-0001 | 396.205 | OH | a1 | B05 | Boc |
| 2-1-a1B06-1-0001 | 398.184 | OH | a1 | B06 | Boc |
| 2-1-a1B07-1-0001 | 404.155 | OH | a1 | B07 | Boc |
| 2-1-a1B08-1-0001 | 425.141 | OH | a1 | B08 | Boc |

(continued)

| [Table 8-1-2] Compound synthesized in step a1 | | | | | |
|---|---|---|---|---|---|
| ID | Exact Mass | | a | B | b |
| 2-1-a1B09-1-0001 | 436.161 | OH | a1 | B09 | Boc |
| 2-1-a1B10-1-0001 | 452.156 | OH | a1 | B10 | Boc |

Example 8-1-2: Formation of linker a2 by step a2

A reaction formula in step a2 performed using BB2-1-11 will be illustrated below.

[Formula 606]

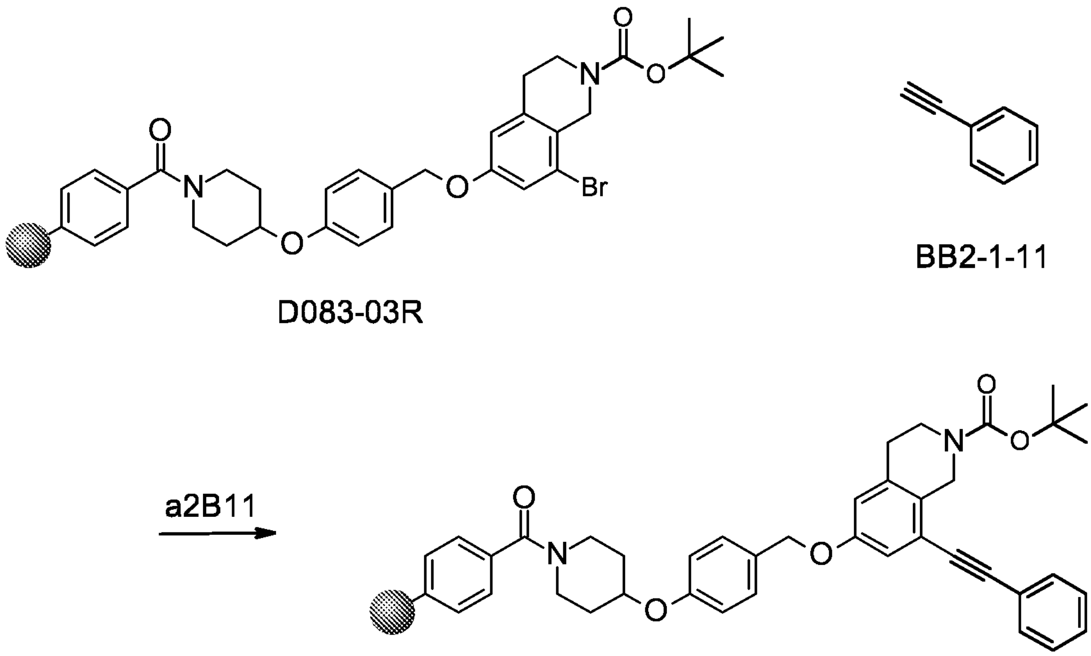


D083-03R

BB2-1-11

2-1-a2B11-0-0001

Compound 2-1-a2B11-0-0001 to compound 2-1-a2B20-0-0001 were synthesized as follows using BB2-1-11 to BB2-1-20.

Compound D083-03R (240 mg, 0.200 mmol/g) and NMP (4.8 mL) were added to ten 8 mL glass vials and shaken at room temperature for 1 hour. Separate alkynes (0.480 mmol) described in Table 8-1-3 were added to the glass vials, respectively. XPhos Pd G4 (41.3 mg, 0.048 mmol) and diisopropylamine (101 μL, 0.720 mmol) were added thereto, and each mixture was shaken at 60°C for 1 hour.

Solid-phase purification

The suspension of the reaction solution and the resin was transferred to a 6 mL column with a filter, filtered, and washed three times with NMP/water = 1/1 (5 mL), three times with NMP (5 mL), three times with 0.2 M NAC in NMP/water = 5/1 (5 mL), three times with 0.05 M $TBAHSO_4$ + DTBP in NMP (5 mL), three times with 0.05 M NMM in NMP (5 mL), three times with NMP/water = 1/1 (5 mL), three times with NMP (5 mL), three times with MeOH (5 mL), three times with DCM (5 mL), and three times with heptane (5 mL), and the obtained solid phase was dried under reduced pressure to obtain the mixtures described in Table 8-1-3.

[Table 296]

| [Table 8-1-3] Reagent used and obtained compound in step a2 | | | |
|---|---|---|---|
| Alkyne reagent used | Amount used | Molar number | Obtained compound |
| BB2-1-11 | 52.7 μl | 0.480mmol | 2-1-a2B11-0-0001 |
| BB2-1-12 | 49.5 mg | 0.480mmol | 2-1-a2B12-0-0001 |
| BB2-1-13 | 55.8 mg | 0.480mmol | 2-1-a2B13-0-0001 |
| BB2-1-14 | 57.7 mg | 0.480mmol | 2-1-a2B14-0-0001 |
| BB2-1-15 | 67.9 μL | 0.480mmol | 2-1-a2B15-0-0001 |
| BB2-1-16 | 61.6 μl | 0.480mmol | 2-1-a2B16-0-0001 |
| BB2-1-17 | 57.2 μl | 0.480mmol | 2-1-a2B17-0-0001 |
| BB2-1-18 | 73.1 mg | 0.480mmol | 2-1-a2B18-0-0001 |
| BB2-1-19 | 68.6 μl | 0.480mmol | 2-1-a2B19-0-0001 |
| BB2-1-20 | 73.8 μl | 0.480mmol | 2-1-a2B20-0-0001 |

Compound 2-1-a2B11-0-0001 to compound 2-1-a2B20-0-0001 were treated by cleavage in the same manner as in Example 8-1-1, so that the formation of compound 2-1- a2B11-1-0001 to compound 2-1-a2B20-1-0001 was confirmed. The details of compound 2- 1-a2B11-1-0001 to compound 2-1-a2B20-1-0001 are shown in Table 8-1-4.

A notation method in each table will be described. For example, in Table 8-1-4, each compound synthesized in step a2 is indicated by ID, and core blocks and linkers in combination in the structures of compound 2-1-a2B1 1-1-0001 to compound 2-1-a2B20-1- 0001 are indicated by symbols or chemical formulas. The correspondence between the symbols and specific structures are shown in Figure 4. In Table 8-1-4, "×" and "b" are represented by chemical formulas.

[Formula 607]

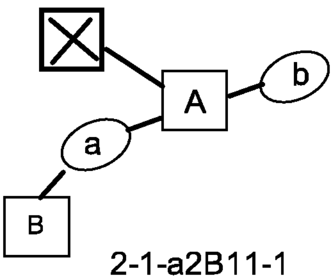


2-1-a2B11-1

[Formula 608]

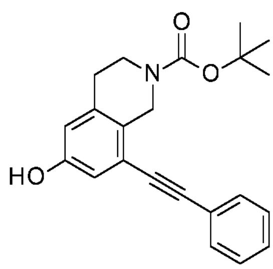

2-1-a2B11-1-0001

2-1-a2B11-1-0001

[Table 297]

| Notation in [Table 8-1-4] | | | | |
|---|---|---|---|---|
| ID | | a | B | b |
| 2-1-a2B11-1-0001 | OH | B11 Boc | a2 | OH |

[Table 298]

| [Table 8-1-4] Compound synthesized in step a2 | | | | | |
|---|---|---|---|---|---|
| ID | Exact Mass | | a | B | b |
| 2-1-a2B11-1-0001 | 349.168 | OH | a2 | B11 | Boc |
| 2-1-a2B12-1-0001 | 350.163 | OH | a2 | B12 | Boc |
| 2-1-a2B13-1-0001 | 363.183 | OH | a2 | B13 | Boc |
| 2-1-a2B14-1-0001 | 367.158 | OH | a2 | B14 | Boc |
| 2-1-a2B15-1-0001 | 377.199 | OH | a2 | B15 | Boc |
| 2-1-a2B16-1-0001 | 379.178 | OH | a2 | B16 | Boc |
| 2-1-a2B17-1-0001 | 385.149 | OH | a2 | B17 | Boc |
| 2-1-a2B18-1-0001 | 399.183 | OH | a2 | B18 | Boc |
| 2-1-a2B19-1-0001 | 417.155 | OH | a2 | B19 | Boc |
| 2-1-a2B20-1-0001 | 433.150 | OH | a2 | B20 | Boc |

Example 8-2: Boc deprotection reaction by steps d01 to d20

A reaction formula to obtain mixture 2-1-C00-0 by performing steps d01 to d20 using compound 2-1-d01B01-0-0001 to compound 2-1-d20B20-0-0001, followed by solid- phase mixing will be illustrated below.

[Formula 609]

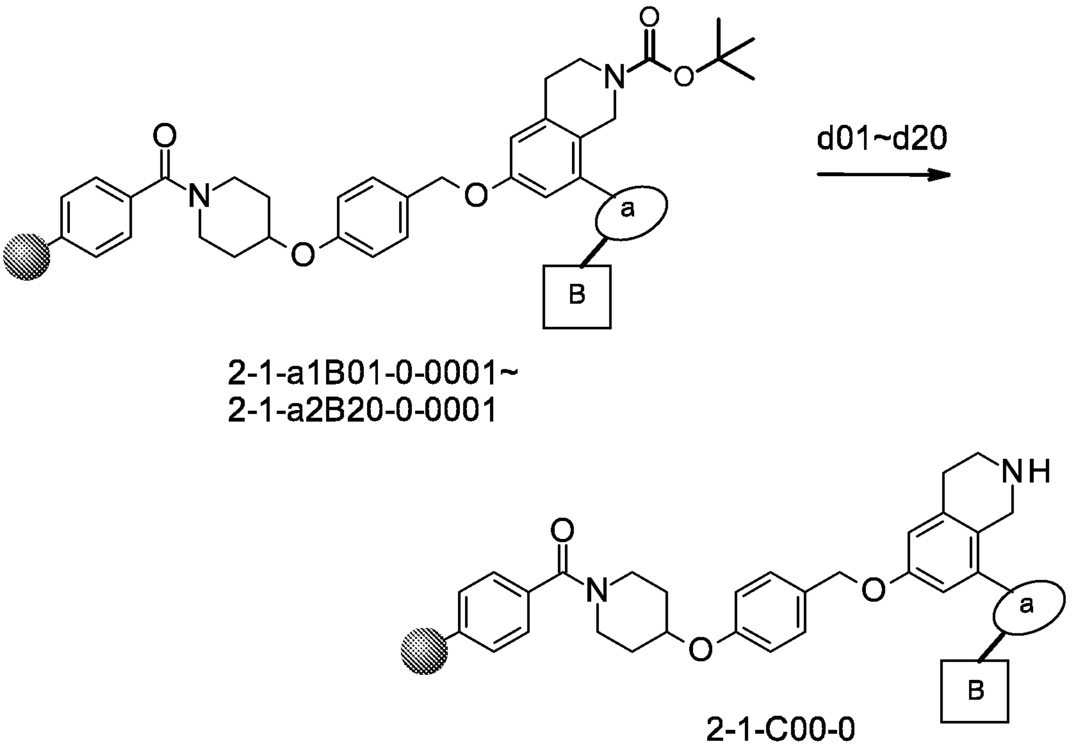


2-1-a1B01-0-0001~
2-1-a2B20-0-0001

2-1-C00-0

Mixture 2-1-C00-0 was synthesized as follows using compound 2-1-a1B01-0-0001 to compound 2-1-a2B20-0-0001 as starting materials.

Separate solid-phase starting materials (180 mg; two vials per type of solid-phase starting material were used; a total of 360 mg was used) described in Table 8-2-1, together with 2-methyltetrahydrofuran (3.6 mL), were added to forty 8 mL glass vials, respectively, and shaken at room temperature for 1 hour. DTBP (0.233 mL, 1.06 mmol) and $Tm(OTf)_3$ (436 mg, 0.707 mmol) were added thereto, and each mixture was shaken at 60°C for 22 hours.

Solid-phase mixing and purification

1 M DMEDA in NMP (1.77 mL) was added to the obtained reaction solution, and the mixture was shaken at room temperature for 1.5 hours. Each of the reaction solutions and the suspensions of the solid phase contained in the forty 8 mL glass vials was transferred to a 200 mL column with a filter using NMP (1.8 mL), filtered, and washed three times with 0.2 M DMEDA in NMP (144 mL), three times with NMP (144 mL), three times with 0.05 M $TBAHSO_4$ + DTBP in NMP (144 mL), three times with 0.05 M BTMG in NMP (144 mL), three times with 0.05 M DTBP in NMP (144 mL), three times with NMP/water = 1/1 (144 mL), three times with NMP (144 mL), three times with MeOH (144 mL), three times with DCM (144 mL), and three times with heptane (144 mL), and the obtained solid phase was dried under reduced pressure to obtain mixture 2-1-C00-0 (7.49 g, amount of the mixture supported on the solid phase: 0.200 mmol/g).
[Table 299]

| [Table 8-2-1] Solid-phase starting material used in steps d01 to d20 | |
|---|---|
| Solid-phase starting material | Amount used |
| 2-1-a1B01-0-0001 | 180 mg |
| 2-1-a1B02-0-0001 | 180 mg |
| 2-1-a1B03-0-0001 | 180 mg |
| 2-1-a1B04-0-0001 | 180 mg |

(continued)

| [Table 8-2-1] Solid-phase starting material used in steps d01 to d20 | |
|---|---|
| Solid-phase starting material | Amount used |
| 2-1-a1B05-0-0001 | 180 mg |
| 2-1-a1B06-0-0001 | 180 mg |
| 2-1-a1B07-0-0001 | 180 mg |
| 2-1-a1B08-0-0001 | 180 mg |
| 2-1-a1B09-0-0001 | 180 mg |
| 2-1-a1B10-0-0001 | 180 mg |
| 2-1-a2B11-0-0001 | 180 mg |
| 2-1-a2B12-0-0001 | 180 mg |
| 2-1-a2B13-0-0001 | 180 mg |
| 2-1-a2B14-0-0001 | 180 mg |
| 2-1-a2B15-0-0001 | 180 mg |
| 2-1-a2B16-0-0001 | 180 mg |
| 2-1-a2B17-0-0001 | 180 mg |
| 2-1-a2B18-0-0001 | 180 mg |
| 2-1-a2B19-0-0001 | 180 mg |
| 2-1-a2B20-0-0001 | 180 mg |

Mixture 2-1-C00-0 can be treated by cleavage in the same manner as in Example 8- 1-1 to obtain mixture 2-1-C00-1. The details of mixture 2-1-C00-1 are shown in Table 8-2-2.

A notation method in each table will be described. For example, in Table 8-2-2, each compound that may be contained in mixture 2-1-C00-1 is indicated by ID, and core blocks and linkers in combination in the structure of mixture 2-1-C00-1 are indicated by symbols or chemical formulas. The correspondence between the symbols and specific structures are shown in Figure 4. In Table 8-2-2, "×" and "b" are represented by chemical formulas.

[Formula 610]

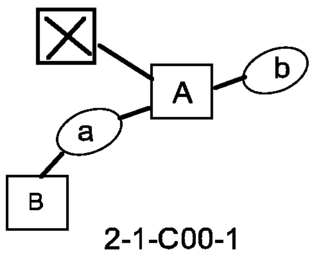


2-1-C00-1

[1869] [Formula 611]

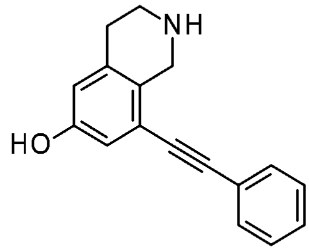

2-1-C00-1-0001

2-1-C00-1-0001

[Table 300]

| Notation in [Table 8-2-2] | | | | |
|---|---|---|---|---|
| ID | | a | B | b |
| 2-1-C00-1-0001 | OH | a2 B11 | b | H |

[Table 301]

| [Table 8-2-2] Compound that may be contained in mixture 2-1-C00-0 | | | | | |
|---|---|---|---|---|---|
| ID | Exact Mass | | a | B | b |
| 2-1-C00-1-0001 | 439.055 | OH | a2 | B11 | H |
| 2-1-C00-1-0002 | 440.050 | OH | a2 | B12 | H |
| 2-1-C00-1-0003 | 453.070 | OH | a2 | B13 | H |
| 2-1-C00-1-0004 | 457.045 | OH | a2 | B14 | H |
| 2-1-C00-1-0005 | 458.061 | OH | a1 | B01 | H |
| 2-1-C00-1-0006 | 459.056 | OH | a1 | B02 | H |
| 2-1-C00-1-0007 | 467.086 | OH | a2 | B15 | H |
| 2-1-C00-1-0008 | 469.065 | OH | a2 | B16 | H |
| 2-1-C00-1-0009 | 472.076 | OH | a1 | B03 | H |
| 2-1-C00-1-0010 | 475.036 | OH | a2 | B17 | H |
| 2-1-C00-1-0011 | 476.051 | OH | a1 | B04 | H |
| 2-1-C00-1-0012 | 486.092 | OH | a1 | B05 | H |
| 2-1-C00-1-0013 | 488.071 | OH | a1 | B06 | H |
| 2-1-C00-1-0014 | 489.070 | OH | a2 | B18 | H |
| 2-1-C00-1-0015 | 494.042 | OH | a1 | B07 | H |
| 2-1-C00-1-0016 | 507.042 | OH | a2 | B19 | H |
| 2-1-C00-1-0017 | 515.028 | OH | a1 | B08 | H |
| 2-1-C00-1-0018 | 523.037 | OH | a2 | B20 | H |
| 2-1-C00-1-0019 | 526.048 | OH | a1 | B09 | H |
| 2-1-C00-1-0020 | 542.043 | OH | a1 | B10 | H |

Example 8-3-1: Formation of linker b1 by step b1

A reaction formula in step b1 performed using BB2-1-21 will be illustrated below.

[Formula 612]

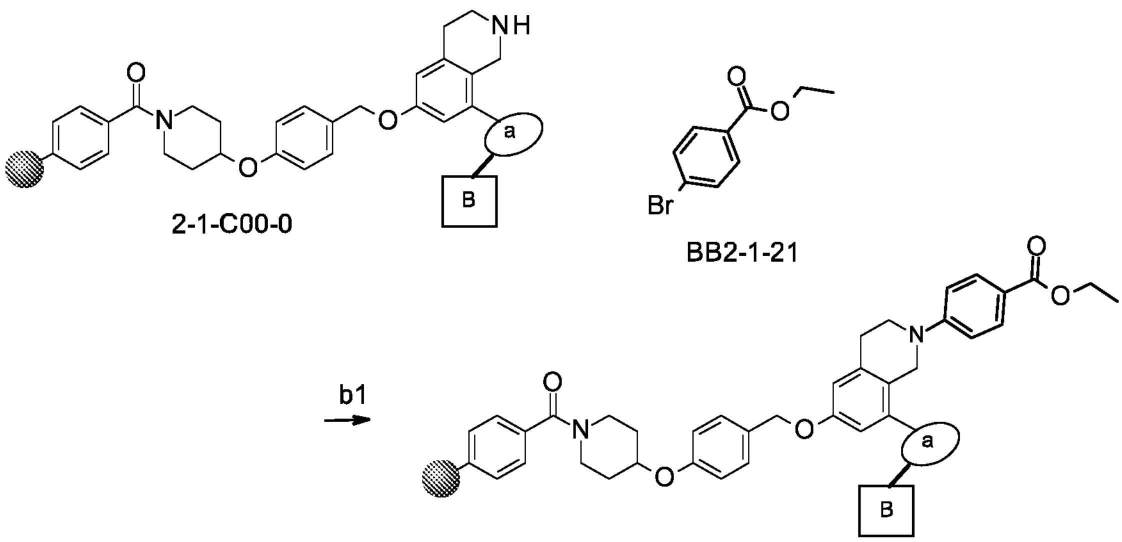


2-1-C00-0

BB2-1-21

2-1-b1C08-0

Mixture 2-1-b1C08-0 to mixture 2-1-b1C14-0 and mixture 2-1-b1C29-0 to mixture 2-1-b1C32-0 were synthesized as follows using BB2-1-21 to BB2-1-27 and BB2-1-41 to BB2-1-44.

Mixture 2-1-c00-0 (140 mg, amount of the mixture supported on the solid phase: 0.200 mmol/g) and NMP (2.8 mL) were added to eleven 4 mL glass vials and shaken at room temperature for 1 hour. Separate aryl bromides (0.280 mmol) described in Table 8-3-1-1 were added to the vials, respectively. RuPhos Pd G4 (23.8 mg, 0.028 mmol) and 2 M PztBu in THF (0.210 mL, 0.420 mmol) were added thereto, and each mixture was shaken at 40°C for 3 hours.

Solid-phase purification

The suspension of the reaction solution and the resin was transferred to a 6 mL column with a filter, filtered, and washed three times with NMP/water = 1/1 (3 mL), three times with NMP (3 mL), three times with 0.2 M NAC in NMP/water = 5/1 (3 mL), three times with 0.05 M $TBAHSO_4$ + DTBP in NMP (3 mL), three times with 0.05 M BTMG in NMP (3 mL), three times with NMP/water = 1/1 (3 mL), three times with NMP (3 mL), three times with MeOH (3 mL), three times with DCM (3 mL), and three times with heptane (3 mL), and the obtained solid phase was dried under reduced pressure.

The reaction and the solid-phase purification mentioned above were performed at the number of times described in Table 8-3-1-1.

Capping

NMP (2.8 mL) was added to eleven 6 mL filters containing the mixtures after the solid-phase purification mentioned above, and shaken at room temperature for 1 hour. Separate capping reagents described in Table 8-3-1-1 were added to the filters, respectively. HOAt (38.1 mg, 0.280 mmol) and DIC (87 μL, 0.560 mmol) were added thereto, and each mixture was shaken at room temperature for 4 hours to cap unreacted amine.

Solid-phase purification

The suspension of the reaction solution and the resin was filtered through the filter of the column and washed three times with NMP/water = 1/1 (3 mL), three times with NMP (3 mL), three times with 0.05 M $TBAHSO_4$ + DTBP in NMP (3 mL), three times with 0.05 M NMM in NMP (3 mL), three times with NMP/water = 1/1 (3 mL), three times with NMP (3 mL), three times with MeOH (3 mL), three times with DCM (3 mL), and three times with heptane (3 mL), and the obtained solid phase was dried under reduced pressure to obtain the mixtures described in the table. The mixtures may contain the components described in Table 8-3-1-1 resulting from capping.

[Table 302]

| [Table 8-3-1-1] Reagent used and obtained compound in step b1 | | | | | | | | |
|---|---|---|---|---|---|---|---|---|
| Ar8r used | Molar number (mmol) | Amount used | The number of times of reaction in step b1 | Capping reagent | Molar number (mmol) | Amount used | Obtained mixture | Mixture that may be contained by capping |
| 882-1-21 | 0.280 | 64.1 mg | 1 | BB2-1-34 | 0.280 | 59.4 mg | 2-1-b1 C08-0 | 2-1-b2C07-0 |
| BB2-1-22 | 0.280 | 83.0 mg | 1 | BB2-1-29 | 0.280 | 54.4 mg | 2-1-b1 C09-0 | 2-1-b2C02-0 |
| BB2-1-23 | 0.280 | 64.7 mg | 2 | BB2-1-29 | 0.280 | 54.4 mg | 2-1-b1C10-0 | 2-1-b2C02-0 |
| BB2-1-24 | 0.280 | 60.5 mg | 2 | BB2-1-28 | 0.280 | 47.6 mg | 2-1-b1C11-0 | 2-1-b2C01-0 |
| BB2-1-25 | 0.280 | 66.1 mg | 2 | BB2-1-31 | 0.280 | 55.5 mg | 2-1-b1 C12-0 | 2-1-b2C04-0 |
| BB2-1-26 | 0.280 | 79.0 mg | 2 | BB2-1-33 | 0.280 | 56.1 mg | 2-1-b1 C13-0 | 2-1-b2C06-0 |
| BB2-1-27 | 0.280 | 80.0 mg | 2 | BB2-1-33 | 0.280 | 56.1 mg | 2-1-b1 C14-0 | 2-1-b2C06-0 |
| 882-1-41 | 0.280 | 95.0 mg | 2 | BB2-1-48 | 0.280 | 74.8 mg | 2-1-b1 C29-0 | 2-1-b2C25-0 |
| BB2-1-42 | 0.280 | 87.0 mg | 2 | BB2-1-45 | 0.280 | 66.4 mg | 2-1-b1 C30-0 | 2-1-b2C19-0 |
| BB2-1-43 | 0.280 | 103 mg | 2 | BB2-1-46 | 0.280 | 70.4 mq | 2-1-b1C31-0 | 2-1-b2C21-0 |
| BB2-1-44 | 0.280 | 91.0 mg | 2 | BB2-1-49 | 0.280 | 66.7 mg | 2-1-b1 C32-0 | 2-1-b2C27-0 |

Mixture 2-1-b 1 C08-0 to mixture 2-1-b1C14-0 and mixture 2-1-b 1 C29-0 to mixture 2-1-b1C32-0 can be treated by cleavage in the same manner as in Example 8-1-1 to obtain mixture 2-1-b1C08-1 to mixture 2-1-b1C14-1 and mixture 2-1-blC29-1 to mixture 2-1- b1C32-1. The details of the mixtures are shown in Table 8-3-1-2 to Table 8-3-1-12.

A notation method in each table will be described. In Table 8-3-1-2 to Table 8-3-1- 12, each compound that may be contained in mixture 2-1-b1C08-1 to mixture 2-1-b1C14-1 and mixture 2-1-b1C29-1 to mixture 2-1-b1C32-1 is indicated by ID, and core blocks and linkers in combination in the structures of mixture 2-1-b1C08-1 to mixture 2-1-b1C14-1 and mixture 2-1-b1C29-1 to mixture 2-1-b1C32-1 are indicated by symbols or chemical formulas. The correspondence between the symbols and specific structures are shown in Figure 4. In Table 8-3-1-2 to Table 8-3-1-12, "×" and "c" are represented by chemical formulas.

[Formula 613]

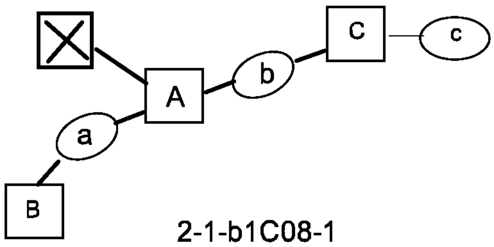


2-1-b1C08-1

For example, when ID is 2-1-b1C08-1-0001, the compound is represented by the following structural formula.

[Formula 614]

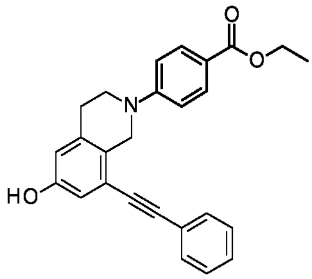

2-1-b1C08-1-0001

2-1-b1C08-1-0001

[Table 303]

| Notation in [Table 8-3-1-2] | | | | | | |
|---|---|---|---|---|---|---|
| ID | | a | B | b | C | c |
| 2-1-b1C08-1-0001 | OH | a2 | B11 | b1 | C08 | COOEt |

[Table 304]

| [Table 8-3-1-2] Compound that may be contained in mixture 2-1-blC08-1 | | | | | | | |
|---|---|---|---|---|---|---|---|
| ID | Exact Mass | | a | B | b | C | c |
| 2-1-b1C08-1-0001 | 369.136 | OH | a2 | B11 | b1 | C08 | COOEt |
| 2-1-b1C08-1-0002 | 370.132 | OH | a2 | B12 | b1 | C08 | COOEt |
| 2-1-b1C08-1-0003 | 383.152 | OH | a2 | B13 | b1 | C08 | COOEt |
| 2-1-b1C08-1-0004 | 387.127 | OH | a2 | B14 | b1 | C08 | COOEt |
| 2-1-b1C08-1-0005 | 388.142 | OH | a1 | B01 | b1 | C08 | COOEt |
| 2-1-b1C08-1-0006 | 389.138 | OH | a1 | B02 | b1 | C08 | COOEt |
| 2-1-b1C08-1-0007 | 397.168 | OH | a2 | B15 | b1 | C08 | COOEt |
| 2-1-b1C08-1-0008 | 399.147 | OH | a2 | B16 | b1 | C08 | COOEt |
| 2-1-b1C08-1-0009 | 402.158 | OH | a1 | B05 | b1 | C08 | COOEt |
| 2-1-b1C08-1-0010 | 405.118 | OH | a2 | B17 | b1 | C08 | COOEt |
| 2-1-b1C08-1-0011 | 406.133 | OH | a1 | B04 | b1 | C08 | COOEt |
| 2-1-b1C08-1-0012 | 416.174 | OH | a1 | B05 | b1 | C08 | COOEt |
| 2-1-b1C08-1-0013 | 418.153 | OH | a1 | B06 | b1 | C08 | COOEt |
| 2-1-b1C08-1-0014 | 419.152 | OH | a2 | B18 | b1 | C08 | COOEt |
| 2-1-b1C08-1-0015 | 424.123 | OH | a1 | B07 | b1 | C08 | COOEt |
| 2-1-b1C08-1-0016 | 437.124 | OH | a2 | B19 | b1 | C08 | COOEt |
| 2-1-b1C08-1-0017 | 445.110 | OH | a1 | B05 | b1 | C08 | COOEt |
| 2-1-b1C08-1-0018 | 453.119 | OH | a2 | B20 | b1 | C08 | COOEt |
| 2-1-b1C08-1-0019 | 456.130 | OH | a1 | B09 | b1 | C08 | COOEt |

(continued)

| [Table 8-3-1-2] Compound that may be contained in mixture 2-1-blC08-1 | | | | | | | |
|---|---|---|---|---|---|---|---|
| ID | Exact Mass | | a | B | b | C | c |
| 2-1-b1C08-1-0020 | 472.125 | OH | a1 | B10 | b1 | C08 | COOEt |

[Table 305]

| [Table 8-3-1-3] Compound that may be contained in mixture 2-1-blC09-1 | | | | | | | |
|---|---|---|---|---|---|---|---|
| ID | Exact Mass | | a | B | b | C | c |
| 2-1-b1C09-1-0001 | 437.124 | OH | a2 | B11 | b1 | C09 | COOEt |
| 2-1-b1C09-1-0002 | 438.119 | OH | a2 | B12 | b1 | C09 | COOEt |
| 2-1-b1C09-1-0003 | 451.140 | OH | a2 | B13 | b1 | C09 | COOEt |
| 2-1-b1C09-1-0004 | 455.114 | OH | a2 | B14 | b1 | C09 | COOEt |
| 2-1-b1C09-1-0005 | 456.130 | OH | a1 | B01 | b1 | C09 | COOEt |
| 2-1-b1C09-1-0006 | 457.125 | OH | a1 | B02 | b1 | C09 | COOEt |
| 2-1-b1C09-1-0007 | 465.155 | OH | a2 | B15 | b1 | C09 | COOEt |
| 2-1-b1C09-1-0008 | 467.134 | OH | a2 | B16 | b1 | C09 | COOEt |
| 2-1-b1C09-1-0009 | 470.145 | OH | a1 | B03 | b1 | C09 | COOEt |
| 2-1-b1C09-1-0010 | 473.105 | OH | a2 | B17 | b1 | C09 | COOEt |
| 2-1-b1C09-1-0011 | 474.120 | OH | a1 | B04 | b1 | C09 | COOEt |
| 2-1-b1C09-1-0012 | 484.161 | OH | a1 | B05 | b1 | C09 | COOEt |
| 2-1-b1C09-1-0013 | 486.140 | OH | a1 | B06 | b1 | C09 | COOEt |
| 2-1-b1C09-1-0014 | 487.140 | OH | a2 | B18 | b1 | C09 | COOEt |
| 2-1-b1C09-1-0015 | 492.111 | OH | a1 | B07 | b1 | C09 | COOEt |
| 2-1-b1C09-1-0016 | 505.111 | OH | a2 | B19 | b1 | C09 | COOEt |
| 2-1-b1C09-1-0017 | 513.097 | OH | a1 | B08 | b1 | C09 | COOEt |
| 2-1-b1C09-1-0018 | 521.106 | OH | a2 | B20 | b1 | C09 | COOEt |
| 2-1-b1C09-1-0019 | 524.117 | OH | a1 | B09 | b1 | C09 | COOEt |
| 2-1-b1C09-1-0020 | 540.112 | OH | a1 | B10 | b1 | C09 | COOEt |

[Table 306]

| [Table 8-3-1-4] Compound that may be contained in mixture 2-1-b1C10-1 | | | | | | | |
|---|---|---|---|---|---|---|---|
| ID | Exact Mass | | a | B | b | C | c |
| 2-1-b1C10-1-0001 | 371.127 | OH | a2 | B11 | b1 | C10 | COOEt |
| 2-1-b1C10-1-0002 | 372.122 | OH | a2 | B12 | b1 | C10 | COOEt |
| 2-1-b1C10-1-0003 | 385.143 | OH | a2 | B13 | b1 | C10 | COOEt |
| 2-1-b1C10-1-0004 | 389.118 | OH | a2 | B14 | b1 | C10 | COOEt |
| 2-1-b1C10-1-0005 | 390.133 | OH | a1 | B01 | b1 | C10 | COOEt |

(continued)

| [Table 8-3-1-4] Compound that may be contained in mixture 2-1-b1C10-1 | | | | | | | |
|---|---|---|---|---|---|---|---|
| ID | Exact Mass | | a | B | b | C | c |
| 2-1-b1C10-1-0006 | 391.128 | OH | a1 | B02 | b1 | C10 | COOEt |
| 2-1-b1C10-1-0007 | 399.158 | OH | a2 | B15 | b1 | C10 | COOEt |
| 2-1-b1C10-1-0008 | 401.138 | OH | a2 | B16 | b1 | C10 | COOEt |
| 2-1-b1C10-1-0009 | 404.148 | OH | a1 | B03 | b1 | C10 | COOEt |
| 2-1-b1C10-1-0010 | 407.108 | OH | a2 | B17 | b1 | C10 | COOEt |
| 2-1-b1C10-1-0011 | 408.123 | OH | a1 | B04 | b1 | C10 | COOEt |
| 2-1-b1C10-1-0012 | 418.164 | OH | a1 | B05 | b1 | C10 | COOEt |
| 2-1-b1C10-1-0013 | 420.143 | OH | a1 | B06 | b1 | C10 | COOEt |
| 2-1-b1C10-1-0014 | 421.143 | OH | a2 | B18 | b1 | C10 | COOEt |
| 2-1-b1C10-1-0015 | 426.114 | OH | a1 | B07 | b1 | C10 | COOEt |
| 2-1-b1C10-1-0016 | 439.114 | OH | a2 | B19 | b1 | C10 | COOEt |
| 2-1-b1C10-1-0017 | 447.100 | OH | a1 | B08 | b1 | C10 | COOEt |
| 2-1-b1C10-1-0018 | 455.109 | OH | a2 | B20 | b1 | C10 | COOEt |
| 2-1-b1C10-1-0019 | 458.120 | OH | a1 | B09 | b1 | C10 | COOEt |
| 2-1-b1C10-1-0020 | 474.115 | OH | a1 | B10 | b1 | C10 | COOEt |

[Table 307]

| [Table 8-3-1-5] Compound that may be contained in mixture 2-1-b1C11-1 | | | | | | | |
|---|---|---|---|---|---|---|---|
| ID | Exact Mass | | a | B | b | C | c |
| 2-1-b1C11-1-0001 | 370.132 | OH | a2 | B11 | b1 | C11 | COOMe |
| 2-1-b1C11-1-0002 | 371.127 | OH | a2 | B12 | b1 | C11 | COOMe |
| 2-1-b1C11-1-0003 | 384.147 | OH | a2 | B13 | b1 | C11 | COOMe |
| 2-1-b1C11-1-0004 | 388.122 | OH | a2 | B14 | b1 | C11 | COOMe |
| 2-1-b1C11-1-0005 | 389.138 | OH | a1 | B01 | b1 | C11 | COOMe |
| 2-1-b1C11-1-0006 | 390.133 | OH | a1 | B02 | b1 | C11 | COOMe |
| 2-1-b1C11-1-0007 | 398.163 | OH | a2 | B15 | b1 | C11 | COOMe |
| 2-1-b1C11-1-0008 | 400.142 | OH | a2 | B16 | b1 | C11 | COOMe |
| 2-1-b1C11-1-0009 | 403.153 | OH | a1 | B03 | b1 | C11 | COOMe |
| 2-1-b1C11-1-0010 | 406.113 | OH | a2 | B17 | b1 | C11 | COOMe |
| 2-1-b1C11-1-0011 | 407.128 | OH | a1 | B04 | b1 | C11 | COOMe |
| 2-1-b1C11-1-0012 | 417.169 | OH | a1 | B05 | b1 | C11 | COOMe |
| 2-1-b1C11-1-0013 | 419.148 | OH | a1 | B06 | b1 | C11 | COOMe |
| 2-1-b1C11-1-0014 | 420.147 | OH | a2 | B18 | b1 | C11 | COOMe |
| 2-1-b1C11-1-0015 | 425.119 | OH | a1 | B07 | b1 | C11 | COOMe |
| 2-1-b1C11-1-0016 | 438.119 | OH | a2 | B19 | b1 | C11 | COOMe |

(continued)

| [Table 8-3-1-5] Compound that may be contained in mixture 2-1-b1C11-1 | | | | | | | |
|---|---|---|---|---|---|---|---|
| ID | Exact Mass | | a | B | b | C | c |
| 2-1-b1C11-1-0017 | 446.105 | OH | a1 | B08 | b1 | C11 | COOMe |
| 2-1-b1C11-1-0018 | 454.114 | OH | a2 | B20 | b1 | C11 | COOMe |
| 2-1-b1C11-1-0019 | 457.125 | OH | a1 | B09 | b1 | C11 | COOMe |
| 2-1-b1C11-1-0020 | 473.120 | OH | a1 | B10 | b1 | C11 | COOMe |

[Table 308]

| [Table 8-3-1-6] Compound that may be contained in mixture 2-1-b1C12-1 | | | | | | | |
|---|---|---|---|---|---|---|---|
| ID | Exact Mass | | a | B | b | C | c |
| 2-1-b1C12-1-0001 | 376.088 | OH | a2 | B11 | b1 | C12 | COOEt |
| 2-1-b1C12-1-0002 | 377.083 | OH | a2 | B12 | b1 | C12 | COOEt |
| 2-1-b1C12-1-0003 | 390.104 | OH | a2 | B13 | b1 | C12 | COOEt |
| 2-1-b1C12-1-0004 | 394.079 | OH | a2 | B14 | b1 | C12 | COOEt |
| 2-1-b1C12-1-0005 | 395.094 | OH | a1 | B01 | b1 | C12 | COOEt |
| 2-1-b1C12-1-0006 | 396.089 | OH | a1 | B02 | b1 | C12 | COOEt |
| 2-1-b1C12-1-0007 | 404.119 | OH | a2 | B15 | b1 | C12 | COOEt |
| 2-1-b1C12-1-0008 | 406.099 | OH | a2 | B16 | b1 | C12 | COOEt |
| 2-1-b1C12-1-0009 | 409.110 | OH | a1 | B03 | b1 | C12 | COOEt |
| 2-1-b1C12-1-0010 | 412.069 | OH | a2 | B17 | b1 | C12 | COOEt |
| 2-1-b1C12-1-0011 | 413.085 | OH | a1 | B04 | b1 | C12 | COOEt |
| 2-1-b1C12-1-0012 | 423.125 | OH | a1 | B05 | b1 | C12 | COOEt |
| 2-1-b1C12-1-0013 | 425.105 | OH | a1 | B06 | b1 | C12 | COOEt |
| 2-1-b1C12-1-0014 | 426.104 | OH | a2 | B18 | b1 | C12 | COOEt |
| 2-1-b1C12-1-0015 | 431.075 | OH | a1 | B07 | b1 | C12 | COOEt |
| 2-1-b1C12-1-0016 | 444.076 | OH | a2 | B19 | b1 | C12 | COOEt |
| 2-1-b1C12-1-0017 | 452.061 | OH | a1 | B08 | b1 | C12 | COOEt |
| 2-1-b1C12-1-0018 | 460.070 | OH | a2 | B20 | b1 | C12 | COOEt |
| 2-1-b1C12-1-0019 | 463.081 | OH | a1 | B09 | b1 | C12 | COOEt |
| 2-1-b1C12-1-0020 | 479.076 | OH | a1 | B10 | b1 | C12 | COOEt |

[Table 309]

| [Table 8-3-1-7] Compound that may be contained in mixture 2-1-b1C13-1 | | | | | | | |
|---|---|---|---|---|---|---|---|
| ID | Exact Mass | | a | B | b | C | c |
| 2-1-b1C13-1-0001 | 421.143 | OH | a2 | B11 | b1 | C13 | COOEt |
| 2-1-b1C13-1-0002 | 422.138 | OH | a2 | B12 | b1 | C13 | COOEt |

(continued)

| [Table 8-3-1-7] Compound that may be contained in mixture 2-1-b1C13-1 | | | | | | | |
|---|---|---|---|---|---|---|---|
| ID | Exact Mass | | a | B | b | C | c |
| 2-1-b1C13-1-0003 | 435.158 | OH | a2 | B13 | b1 | C13 | COOEt |
| 2-1-b1C13-1-0004 | 439.133 | OH | a2 | B14 | b1 | C13 | COOEt |
| 2-1-b1C13-1-0005 | 440.148 | OH | a1 | B01 | b1 | C13 | COOEt |
| 2-1-b1C13-1-0006 | 441.144 | OH | a1 | B02 | b1 | C13 | COOEt |
| 2-1-b1C13-1-0007 | 449.174 | OH | a2 | B15 | b1 | C13 | COOEt |
| 2-1-b1C13-1-0008 | 451.153 | OH | a2 | B16 | b1 | C13 | COOEt |
| 2-1-b1C13-1-0009 | 454.164 | OH | a1 | B03 | b1 | C13 | COOEt |
| 2-1-b1C13-1-0010 | 457.124 | OH | a2 | B17 | b1 | C13 | COOEt |
| 2-1-b1C13-1-0011 | 458.139 | OH | a1 | B04 | b1 | C13 | COOEt |
| 2-1-b1C13-1-0012 | 468.180 | OH | a1 | B05 | b1 | C13 | COOEt |
| 2-1-b1C13-1-0013 | 470.159 | OH | a1 | B06 | b1 | C13 | COOEt |
| 2-1-b1C13-1-0014 | 471.158 | OH | a2 | B18 | b1 | C13 | COOEt |
| 2-1-b1C13-1-0015 | 476.130 | OH | a1 | B07 | b1 | C13 | COOEt |
| 2-1-b1C13-1-0016 | 489.130 | OH | a2 | B19 | b1 | C13 | COOEt |
| 2-1-b1C13-1-0017 | 497.116 | OH | a1 | B08 | b1 | C13 | COOEt |
| 2-1-b1C13-1-0018 | 505.125 | OH | a2 | B20 | b1 | C13 | COOEt |
| 2-1-b1C13-1-0019 | 508.136 | OH | a1 | B09 | b1 | C13 | COOEt |
| 2-1-b1C13-1-0020 | 524.131 | OH | a1 | B10 | b1 | C13 | COOEt |

[Table 310]

| [Table 8-3-1-8] Compound that may be contained in mixture 2-1-b1C14-1 | | | | | | | |
|---|---|---|---|---|---|---|---|
| ID | Exact Mass | | a | B | b | C | c |
| 2-1-b1C14-1-0001 | 425.109 | OH | a2 | B11 | b1 | C14 | COOEt |
| 2-1-b1C14-1-0002 | 426.104 | OH | a2 | B12 | b1 | C14 | COOEt |
| 2-1-b1C14-1-0003 | 439.124 | OH | a2 | B13 | b1 | C14 | COOEt |
| 2-1-b1C14-1-0004 | 443.099 | OH | a2 | B14 | b1 | C14 | COOEt |
| 2-1-b1C14-1-0005 | 444.114 | OH | a1 | B01 | b1 | C14 | COOEt |
| 2-1-b1C14-1-0006 | 445.110 | OH | a1 | B02 | b1 | C14 | COOEt |
| 2-1-b1C14-1-0007 | 453.140 | OH | a2 | B15 | b1 | C14 | COOEt |
| 2-1-b1C14-1-0008 | 455.119 | OH | a2 | B16 | b1 | C14 | COOEt |
| 2-1-b1C14-1-0009 | 458.130 | OH | a1 | B03 | b1 | C14 | COOEt |
| 2-1-b1C14-1-0010 | 461.090 | OH | a2 | B17 | b1 | C14 | COOEt |
| 2-1-b1C14-1-0011 | 462.105 | OH | a1 | B04 | b1 | C14 | COOEt |
| 2-1-b1C14-1-0012 | 472.146 | OH | a1 | B05 | b1 | C14 | COOEt |

| | | | | | | | |
|---|---|---|---|---|---|---|---|
| 2-1-b1C14-1-0013 | 474.125 | OH | a1 | B06 | b1 | C14 | COOEt |

(continued)

| [Table 8-3-1-8] Compound that may be contained in mixture 2-1-b1C14-1 | | | | | | | |
|---|---|---|---|---|---|---|---|
| ID | Exact Mass | | a | B | b | C | c |
| 2-1-b1C14-1-0014 | 475.124 | OH | a2 | B18 | b1 | C14 | COOEt |
| 2-1-b1C14-1-0015 | 480.096 | OH | a1 | B07 | b1 | C14 | COOEt |
| 2-1-b1C14-1-0016 | 493.096 | OH | a2 | B19 | b1 | C14 | COOEt |
| 2-1-b1C14-1-0017 | 501.082 | OH | a1 | B08 | b1 | C14 | COOEt |
| 2-1-b1C14-1-0018 | 509.091 | OH | a2 | B20 | b1 | C14 | COOEt |
| 2-1-b1C14-1-0019 | 512.102 | OH | a1 | B09 | b1 | C14 | COOEt |
| 2-1-b1C14-1-0020 | 528.097 | OH | a1 | B10 | b1 | C14 | COOEt |

[Table 311]

| [Table 8-3-1-9] Compound that may be contained in mixture 2-1-b1C29-1 | | | | | | | |
|---|---|---|---|---|---|---|---|
| ID | Exact Mass | | a | B | b | C | c |
| 2-1-b1C29-1-0001 | 398.179 | OH | a2 | B11 | b1 | C29 | Boc |
| 2-1-b1C29-1-0002 | 399.195 | OH | a2 | B12 | b1 | C29 | Boc |
| 2-1-b1C29-1-0003 | 414.206 | OH | a2 | B13 | b1 | C29 | Boc |
| 2-1-b1C29-1-0004 | 416.170 | OH | a2 | B14 | b1 | C29 | Boc |
| 2-1-b1C29-1-0005 | 417.169 | OH | a1 | B01 | b1 | C29 | Boc |
| 2-1-b1C29-1-0006 | 418.164 | OH | a1 | B02 | b1 | C29 | Boc |
| 2-1-b1C29-1-0007 | 430.186 | OH | a2 | B15 | b1 | C29 | Boc |
| 2-1-b1C29-1-0008 | 432.184 | OH | a2 | B16 | b1 | C29 | Boc |
| 2-1-b1C29-1-0009 | 434.144 | OH | a1 | B03 | b1 | C29 | Boc |
| 2-1-b1C29-1-0010 | 436.159 | OH | a2 | B17 | b1 | C29 | Boc |
| 2-1-b1C29-1-0011 | 437.155 | OH | a1 | B04 | b1 | C29 | Boc |
| 2-1-b1C29-1-0012 | 448.179 | OH | a1 | B05 | b1 | C29 | Boc |
| 2-1-b1C29-1-0013 | 450.231 | OH | a1 | B06 | b1 | C29 | Boc |
| 2-1-b1C29-1-0014 | 453.130 | OH | a2 | B18 | b1 | C29 | Boc |
| 2-1-b1C29-1-0015 | 456.141 | OH | a1 | B07 | b1 | C29 | Boc |
| 2-1-b1C29-1-0016 | 470.125 | OH | a2 | B19 | b1 | C29 | Boc |
| 2-1-b1C29-1-0017 | 478.187 | OH | a1 | B08 | b1 | C29 | Boc |
| 2-1-b1C29-1-0018 | 485.231 | OH | a2 | B20 | b1 | C29 | Boc |
| 2-1-b1C29-1-0019 | 486.231 | OH | a1 | B09 | b1 | C29 | Boc |
| 2-1-b1C29-1-0020 | 504.202 | OH | a1 | B10 | b1 | C29 | Boc |

[Table 312]

| [Table 8-3-1-10] Compound that may be contained in mixture 2-1-b1C30-1 | | | | | | | |
|---|---|---|---|---|---|---|---|
| ID | Exact Mass | | a | B | b | C | c |
| 2-1-b1C30-1-0001 | 374.199 | OH | a2 | B11 | b1 | C30 | Boc |
| 2-1-b1C30-1-0002 | 378.174 | OH | a2 | B12 | b1 | C30 | Boc |
| 2-1-b1C30-1-0003 | 383.163 | OH | a2 | B13 | b1 | C30 | Boc |
| 2-1-b1C30-1-0004 | 389.149 | OH | a2 | B14 | b1 | C30 | Boc |
| 2-1-b1C30-1-0005 | 390.194 | OH | a1 | B01 | b1 | C30 | Boc |
| 2-1-b1C30-1-0006 | 394.205 | OH | a1 | B02 | b1 | C30 | Boc |
| 2-1-b1C30-1-0007 | 398.163 | OH | a2 | B15 | b1 | C30 | Boc |
| 2-1-b1C30-1-0008 | 400.19 | OH | a2 | B16 | b1 | C30 | Boc |
| 2-1-b1C30-1-0009 | 405.149 | OH | a1 | B03 | b1 | C30 | Boc |
| 2-1-b1C30-1-0010 | 409.200 | OH | a2 | B17 | b1 | C30 | Boc |
| 2-1-b1C30-1-0011 | 410.199 | OH | a1 | B04 | b1 | C30 | Boc |
| 2-1-b1C30-1-0012 | 416.185 | OH | a1 | B05 | b1 | C30 | Boc |
| 2-1-b1C30-1-0013 | 419.163 | OH | a1 | B06 | b1 | C30 | Boc |
| 2-1-b1C30-1-0014 | 422.236 | OH | a2 | B18 | b1 | C30 | Boc |
| 2-1-b1C30-1-0015 | 428.174 | OH | a1 | B07 | b1 | C30 | Boc |
| 2-1-b1C30-1-0016 | 441.242 | OH | a2 | B19 | b1 | C30 | Boc |
| 2-1-b1C30-1-0017 | 449.191 | OH | a1 | B08 | b1 | C30 | Boc |
| 2-1-b1C30-1-0018 | 456.216 | OH | a2 | B20 | b1 | C30 | Boc |
| 2-1-b1C30-1-0019 | 463.172 | OH | a1 | B09 | b1 | C30 | Boc |
| 2-1-b1C30-1-0020 | 473.211 | OH | a1 | B10 | b1 | C30 | Boc |

[Table 313]

| [Table 8-3-1-11] Compound that may be contained in mixture 2-1-b1C31-1 | | | | | | | |
|---|---|---|---|---|---|---|---|
| ID | Exact Mass | | a | B | b | C | c |
| 2-1-b1C31-1-0001 | 429.205 | OH | a2 | B11 | b1 | C31 | Boc |
| 2-1-b1C31-1-0002 | 431.185 | OH | a2 | B12 | b1 | C31 | Boc |
| 2-1-b1C31-1-0003 | 445.121 | OH | a2 | B13 | b1 | C31 | Boc |
| 2-1-b1C31-1-0004 | 447.177 | OH | a2 | B14 | b1 | C31 | Boc |
| 2-1-b1C31-1-0005 | 448.176 | OH | a1 | B01 | b1 | C31 | Boc |
| 2-1-b1C31-1-0006 | 450.156 | OH | a1 | B02 | b1 | C31 | Boc |
| 2-1-b1C31-1-0007 | 457.237 | OH | a2 | B15 | b1 | C31 | Boc |
| 2-1-b1C31-1-0008 | 458.236 | OH | a2 | B16 | b1 | C31 | Boc |
| 2-1-b1C31-1-0009 | 464.246 | OH | a1 | B03 | b1 | C31 | Boc |
| 2-1-b1C31-1-0010 | 467.182 | OH | a2 | B17 | b1 | C31 | Boc |
| 2-1-b1C31-1-0011 | 469.161 | OH | a1 | B04 | b1 | C31 | Boc |
| 2-1-b1C31-1-0012 | 474.136 | OH | a1 | B05 | b1 | C31 | Boc |

(continued)

| [Table 8-3-1-11] Compound that may be contained in mixture 2-1-b1C31-1 | | | | | | | |
|---|---|---|---|---|---|---|---|
| ID | Exact Mass | | a | B | b | C | c |
| 2-1-b1C31-1-0013 | 482.145 | OH | a1 | B06 | b1 | C31 | Boc |
| 2-1-b1C31-1-0014 | 483.252 | OH | a2 | B18 | b1 | C31 | Boc |
| 2-1-b1C31-1-0015 | 485.156 | OH | a1 | B07 | b1 | C31 | Boc |
| 2-1-b1C31-1-0016 | 501.151 | OH | a2 | B19 | b1 | C31 | Boc |
| 2-1-b1C31-1-0017 | 511.208 | OH | a1 | B08 | b1 | C31 | Boc |
| 2-1-b1C31-1-0018 | 520.197 | OH | a2 | B20 | b1 | C31 | Boc |
| 2-1-b1C31-1-0019 | 523.208 | OH | a1 | B09 | b1 | C31 | Boc |
| 2-1-b1C31-1-0020 | 539.203 | OH | a1 | B10 | b1 | C31 | Boc |

[Table 314]

| [Table 8-3-1-12] Compound that may be contained in mixture 2-1-b1C32-1 | | | | | | | |
|---|---|---|---|---|---|---|---|
| ID | Exact Mass | | a | B | b | C | c |
| 2-1-b1C32-1-0001 | 386.143 | OH | a2 | B11 | b1 | C32 | Boc |
| 2-1-b1C32-1-0002 | 386.143 | OH | a2 | B12 | b1 | C32 | Boc |
| 2-1-b1C32-1-0003 | 399.158 | OH | a2 | B13 | b1 | C32 | Boc |
| 2-1-b1C32-1-0004 | 404.134 | OH | a2 | B14 | b1 | C32 | Boc |
| 2-1-b1C32-1-0005 | 405.149 | OH | a1 | B01 | b1 | C32 | Boc |
| 2-1-b1C32-1-0006 | 407.221 | OH | a1 | B02 | b1 | C32 | Boc |
| 2-1-b1C32-1-0007 | 415.171 | OH | a2 | B15 | b1 | C32 | Boc |
| 2-1-b1C32-1-0008 | 415.190 | OH | a2 | B16 | b1 | C32 | Boc |
| 2-1-b1C32-1-0009 | 417.169 | OH | a1 | B03 | b1 | C32 | Boc |
| 2-1-b1C32-1-0010 | 422.124 | OH | a2 | B17 | b1 | C32 | Boc |
| 2-1-b1C32-1-0011 | 424.135 | OH | a1 | B04 | b1 | C32 | Boc |
| 2-1-b1C32-1-0012 | 435.159 | OH | a1 | B05 | b1 | C32 | Boc |
| 2-1-b1C32-1-0013 | 436.140 | OH | a1 | B06 | b1 | C32 | Boc |
| 2-1-b1C32-1-0014 | 436.215 | OH | a2 | B18 | b1 | C32 | Boc |
| 2-1-b1C32-1-0015 | 444.166 | OH | a1 | B07 | b1 | C32 | Boc |
| 2-1-b1C32-1-0016 | 455.146 | OH | a2 | B19 | b1 | C32 | Boc |
| 2-1-b1C32-1-0017 | 466.150 | OH | a1 | B08 | b1 | C32 | Boc |
| 2-1-b1C32-1-0018 | 470.178 | OH | a2 | B20 | b1 | C32 | Boc |
| 2-1-b1C32-1-0019 | 472.196 | OH | a1 | B09 | b1 | C32 | Boc |
| 2-1-b1C32-1-0020 | 491.202 | OH | a1 | B10 | b1 | C32 | Boc |

Example 8-3-2: Formation of linker b2 by step b2

A reaction formula in step b2 performed using BB2-1-28 will be illustrated below.

[Formula 615]

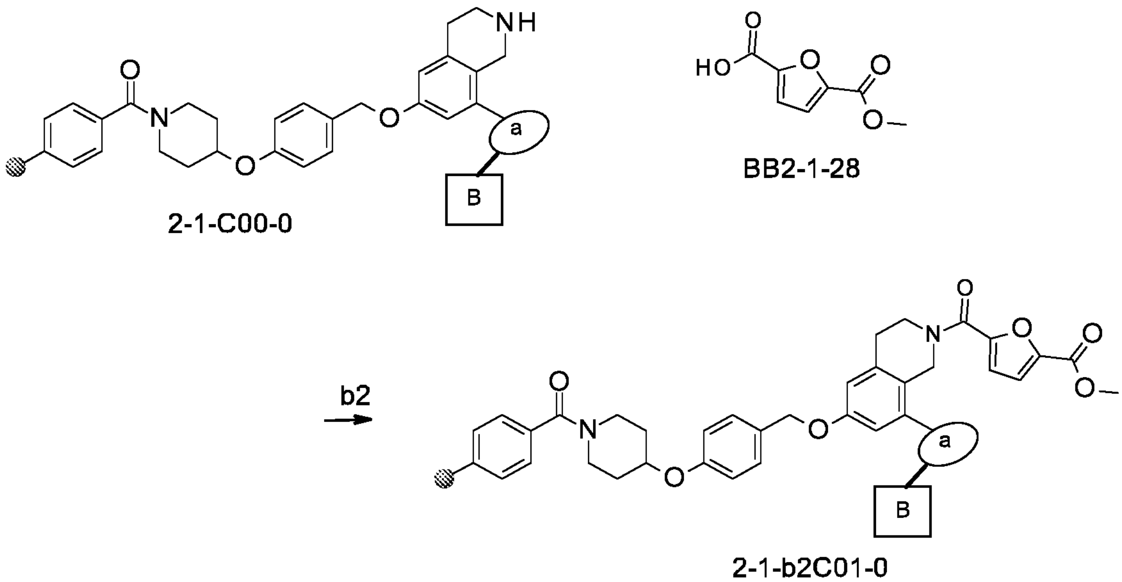


2-1-C00-0

BB2-1-28

2-1-b2C01-0

Mixture 2-1-b2C01-0 to mixture 2-1-b2C07-0 and mixture 2-1-b2C19-0 to mixture 2-1-b2C33-0 were synthesized as follows using BB2-1-28 to BB2-1-34 and BB2-1-45 to BB2-1-50.

Mixture 2-1-c00-0 (135 mg, amount of the mixture supported on the solid phase: 0.200 mmol/g) and NMP (2.7 mL) were added to thirteen 4 mL glass vials and shaken at room temperature for 1 hour. Separate carboxylic acids (0.270 mmol) described in Table 8- 3-2-1 were added to the vials, respectively. HOAt (36.8 mg, 0.270 mmol) and DIC (84 μL, 0.540 mmol) were added thereto, and each mixture was shaken at room temperature for 4 hours.

Solid-phase purification

The suspension of the reaction solution and the resin was transferred to a 6 mL column with a filter, filtered, and washed three times with NMP/water = 1/1 (5 mL), three times with NMP (5 mL), three times with 0.05 M $TBAHSO_4$ + DTBP in NMP (5 mL), three times with 0.05 M NMM in NMP (5 mL), three times with NMP/water = 1/1 (5 mL), three times with NMP (5 mL), three times with MeOH (5 mL), three times with DCM (5 mL), and three times with heptane (5 mL), and the obtained solid phase was dried under reduced pressure to obtain the mixtures described in Table 8-3-2-1.

[Table 314-1]

| [Table 8-3-2-1] Reagent used and obtained compound in step b2 | | | |
|---|---|---|---|
| Carboxylic acid used | Molar number | Amount used | Obtained mixture |
| BB2-1-28 | 0.270 mmol | 45.9 mg | 2-1-b2C01-0 |
| BB2-1-29 | 0.270 mmol | 52.4 mg | 2-1-b2C02-0 |
| BB2-1-30 | 0.270 mmol | 52.7 mg | 2-1-b2C03-0 |
| BB2-1-31 | 0.270 mmol | 53.5 mg | 2-1-b2C04-0 |
| BB2-1-32 | 0.270 mmol | 56.5 mg | 2-1-b2C05-0 |
| BB2-1-33 | 0.270 mmol | 54.1 mg | 2-1-b2C06-0 |
| BB2-1-34 | 0.270 mmol | 57.3 mg | 2-1-b2C07-0 |
| BB2-1-45 | 0.270 mmol | 64.1 mg | 2-1-b2C 19-0 |
| BB2-1-46 | 0.270 mmol | 67.8 mg | 2-1-b2C21-0 |
| BB2-1-47 | 0.270 mmol | 68.9 mg | 2-1-b2C23-0 |
| BB2-1-48 | 0.270 mmol | 72.2 mg | 2-1-b2C25-0 |

(continued)

| [Table 8-3-2-1] Reagent used and obtained compound in step b2 | | | |
|---|---|---|---|
| Carboxylic acid used | Molar number | Amount used | Obtained mixture |
| BB2-1-49 | 0.270 mmol | 64.3 mg | 2-1-b2C27-0 |
| BB2-1-50 | 0.270 mmol | 61.9 mg | 2-1-b2C33-0 |

Mixture 2-1-b2C01-0 to mixture 2-1-b2C07-0 and mixture 2-1 -b2C 19-0 to mixture 2-1-b2C33-0 can be treated by cleavage in the same manner as in Example 8-1-1 to obtain mixture 2-1-b2C01-1 to mixture 2-1-b2C07-1 and mixture 2-1-b2C19-1 to mixture 2-1- b2C33-1. The details of the mixtures are shown in Table 8-3-2-2 to Table 8-3-2-14.

A notation method in each table will be described. In Table 8-3-2-2 to Table 8-3-2- 14, each compound that may be contained in mixture 2-1-b2C01-1 to mixture 2-1-b2C07-1 and mixture 2-1-b2C19-1 to mixture 2-1-b2C33-1 is indicated by ID, and core blocks and linkers in combination in the structures of mixture 2-1-b2C01-1 to mixture 2-1-b2C07-1 and mixture 2-1-b2C19-1 to mixture 2-1-b2C33-1 are indicated by symbols or chemical formulas. The correspondence between the symbols and specific structures are shown in Figure 4. In Table 8-3-2-2 to Table 8-3-2-14, "×" and "c" are represented by chemical formulas.

[Formula 616]

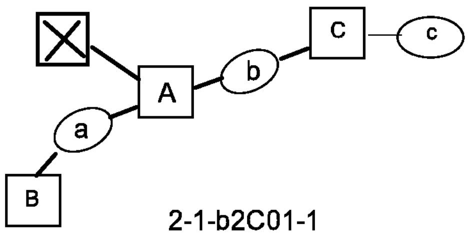


2-1-b2C01-1

For example, when ID is 2-1-b2C01-1-0001, the compound is represented by the following structural formula.

[Formula 617]

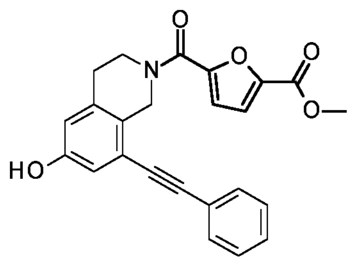

2-1-b2C01-1-0001

2-1-b2C01-1-0001

[Table 315]

| Notation in [Table 8-3-2-2] | | | | | | |
|---|---|---|---|---|---|---|
| ID | | a | B | b | C | c |
| 2-1-b2C01-1-0001 | OH | a2 | B11 | b2 | C01 | COOMe |

[Table 316]

| [Table 8-3-2-2] Compound that may be contained in mixture 2-1-b2C01-1 | | | | | | | |
|---|---|---|---|---|---|---|---|
| ID | Exact Mass | | a | B | b | C | c |
| 2-1-b2C01-1-0001 | 387.111 | OH | a2 | B11 | b2 | C01 | COOMe |
| 2-1-b2C01-1-0002 | 388.106 | OH | a2 | B12 | b2 | C01 | COOMe |
| 2-1-b2C01-1-0003 | 401.126 | OH | a2 | B13 | b2 | C01 | COOMe |
| 2-1-b2C01-1-0004 | 405.101 | OH | a2 | B14 | b2 | C01 | COOMe |
| 2-1-b2C01-1-0005 | 406.116 | OH | a1 | B01 | b2 | C01 | COOMe |
| 2-1-b2C01-1-0006 | 407.112 | OH | a1 | B02 | b2 | C01 | COOMe |
| 2-1-b2C01-1-0007 | 415.142 | OH | a2 | B15 | b2 | C01 | COOMe |
| 2-1-b2C01-1-0008 | 417.121 | OH | a2 | B16 | b2 | C01 | COOMe |
| 2-1-b2C01-1-0009 | 420.132 | OH | a1 | B03 | b2 | C01 | COOMe |
| 2-1-b2C01-1-0010 | 423.092 | OH | a2 | B17 | b2 | C01 | COOMe |
| 2-1-b2C01-1-0011 | 424.107 | OH | a1 | B04 | b2 | C01 | COOMe |
| 2-1-b2C01-1-0012 | 434.148 | OH | a1 | B05 | b2 | C01 | COOMe |
| 2-1-b2C01-1-0013 | 436.127 | OH | a1 | B06 | b2 | C01 | COOMe |
| 2-1-b2C01-1-0014 | 437.126 | OH | a2 | B18 | b2 | C01 | COOMe |
| 2-1-b2C01-1-0015 | 442.098 | OH | a1 | B07 | b2 | C01 | COOMe |
| 2-1-b2C01-1-0016 | 455.098 | OH | a2 | B19 | b2 | C01 | COOMe |
| 2-1-b2C01-1-0017 | 463.084 | OH | a1 | B08 | b2 | C01 | COOMe |
| 2-1-b2C01-1-0018 | 471.093 | OH | a2 | B20 | b2 | C01 | COOMe |
| 2-1-b2C01-1-0019 | 474.104 | OH | a1 | B09 | b2 | C01 | COOMe |
| 2-1-b2C01-1-0020 | 490.099 | OH | a1 | B10 | b2 | C01 | COOMe |

[Table 317]

| [Table 8-3-2-3] Compound that may be contained in mixture 2-1-b2C02-1 | | | | | | | |
|---|---|---|---|---|---|---|---|
| ID | Exact Mass | | a | B | b | C | c |
| 2-1-b2C02-1-0001 | 397.131 | OH | a2 | B11 | b2 | C02 | COOEt |
| 2-1-b2C02-1-0002 | 398.127 | OH | a2 | B12 | b2 | C02 | COOEt |
| 2-1-b2C02-1-0003 | 411.147 | OH | a2 | B13 | b2 | C02 | COOEt |
| 2-1-b2C02-1-0004 | 415.122 | OH | a2 | B14 | b2 | C02 | COOEt |
| 2-1-b2C02-1-0005 | 416.137 | OH | a1 | B01 | b2 | C02 | COOEt |
| 2-1-b2C02-1-0006 | 417.132 | OH | a1 | B02 | b2 | C02 | COOEt |
| 2-1-b2C02-1-0007 | 425.163 | OH | a2 | B15 | b2 | C02 | COOEt |
| 2-1-b2C02-1-0008 | 427.142 | OH | a2 | B16 | b2 | C02 | COOEt |
| 2-1-b2C02-1-0009 | 430.153 | OH | a1 | B03 | b2 | C02 | COOEt |
| 2-1-b2C02-1-0010 | 433.113 | OH | a2 | B17 | b2 | C02 | COOEt |
| 2-1-b2C02-1-0011 | 434.128 | OH | a1 | B04 | b2 | C02 | COOEt |

(continued)

| [Table 8-3-2-3] Compound that may be contained in mixture 2-1-b2C02-1 | | | | | | | |
|---|---|---|---|---|---|---|---|
| ID | Exact Mass | | a | B | b | C | c |
| 2-1-b2C02-1-0012 | 444.169 | OH | a1 | B05 | b2 | C02 | COOEt |
| 2-1-b2C02-1-0013 | 446.148 | OH | a1 | B06 | b2 | C02 | COOEt |
| 2-1-b2C02-1-0014 | 447.147 | OH | a2 | B18 | b2 | C02 | COOEt |
| 2-1-b2C02-1-0015 | 452.118 | OH | a1 | B07 | b2 | C02 | COOEt |
| 2-1-b2C02-1-0016 | 465.119 | OH | a2 | B19 | b2 | C02 | COOEt |
| 2-1-b2C02-1-0017 | 473.105 | OH | a1 | B08 | b2 | C02 | COOEt |
| 2-1-b2C02-1-0018 | 481.114 | OH | a2 | B20 | b2 | C02 | COOEt |
| 2-1-b2C02-1-0019 | 484.125 | OH | a1 | B09 | b2 | C02 | COOEt |
| 2-1-b2C02-1-0020 | 500.120 | OH | a1 | B10 | b2 | C02 | COOEt |

[Table 318]

| Table 8-3-2-4] Compound that may be contained in mixture 2-1-b2C03-1 | | | | | | | |
|---|---|---|---|---|---|---|---|
| ID | Exact Mass | 19 | a | B | b | C | c |
| 2-1-b2C03-1-0001 | 398.127 | OH | a2 | B11 | b2 | C03 | COOEt |
| 2-1-b2C03-1-0002 | 399.122 | OH | a2 | B12 | b2 | C03 | COOEt |
| 2-1-b2C03-1-0003 | 412.142 | OH | a2 | B13 | b2 | C03 | COOEt |
| 2-1-b2C03-1-0004 | 416.117 | OH | a2 | B14 | b2 | C03 | COOEt |
| 2-1-b2C03-1-0005 | 417.132 | OH | a1 | B01 | b2 | C03 | COOEt |
| 2-1-b2C03-1-0006 | 418.128 | OH | a1 | B02 | b2 | C03 | COOEt |
| 2-1-b2C03-1-0007 | 426.158 | OH | a2 | B15 | b2 | C03 | COOEt |
| 2-1-b2C03-1-0008 | 428.137 | OH | a2 | B16 | b2 | C03 | COOEt |
| 2-1-b2C03-1-0009 | 431.148 | OH | a1 | B03 | b2 | C03 | COOEt |
| 2-1-b2C03-1-0010 | 434.108 | OH | a2 | B17 | b2 | C03 | COOEt |
| 2-1-b2C03-1-0011 | 435.123 | OH | a1 | B04 | b2 | C03 | COOEt |
| 2-1-b2C03-1-0012 | 445.164 | OH | a1 | B05 | b2 | C03 | COOEt |
| 2-1-b2C03-1-0013 | 447.143 | OH | a1 | B06 | b2 | C03 | COOEt |
| 2-1-b2C03-1-0014 | 448.142 | OH | a2 | B18 | b2 | C03 | COOEt |
| 2-1-b2C03-1-0015 | 453.114 | OH | a1 | B07 | b2 | C03 | COOEt |
| 2-1-b2C03-1-0016 | 466.114 | OH | a2 | B19 | b2 | C03 | COOEt |
| 2-1-b2C03-1-0017 | 474.100 | OH | a1 | B08 | b2 | C03 | COOEt |
| 2-1-b2C03-1-0018 | 482.109 | OH | a2 | B20 | b2 | C03 | COOEt |
| 2-1-b2C03-1-0019 | 485.120 | OH | a1 | B09 | b2 | C03 | COOEt |
| 2-1-b2C03-1-0020 | 501.115 | OH | a1 | B10 | b2 | C03 | COOEt |

[Table 319]

| [Table 8-3-2-5] Compound that may be contained in mixture 2-1-b2C04-1 | | | | | | | |
|---|---|---|---|---|---|---|---|
| ID | Exact Mass | | a | B | b | C | c |
| 2-1-b2C04-1-0001 | 415.122 | OH | a2 | B11 | b2 | C04 | COOMe |
| 2-1-b2C04-1-0002 | 416.117 | OH | a2 | B12 | b2 | C04 | COOMe |
| 2-1-b2C04-1-0003 | 429.138 | OH | a2 | B13 | b2 | C04 | COOMe |
| 2-1-b2C04-1-0004 | 433.113 | OH | a2 | B14 | b2 | C04 | COOMe |
| 2-1-b2C04-1-0005 | 434.128 | OH | a1 | B01 | b2 | C04 | COOMe |
| 2-1-b2C04-1-0006 | 435.123 | OH | a1 | B02 | b2 | C04 | COOMe |
| 2-1-b2C04-1-0007 | 443.153 | OH | a2 | B15 | b2 | C04 | COOMe |
| 2-1-b2C04-1-0008 | 445.133 | OH | a2 | B16 | b2 | C04 | COOMe |
| 2-1-b2C04-1-0009 | 448.143 | OH | a1 | B03 | b2 | C04 | COOMe |
| 2-1-b2C04-1-0010 | 451.103 | OH | a2 | B17 | b2 | C04 | COOMe |
| 2-1-b2C04-1-0011 | 452.118 | OH | a1 | B04 | b2 | C04 | COOMe |
| 2-1-b2C04-1-0012 | 462.159 | OH | a1 | B05 | b2 | C04 | COOMe |
| 2-1-b2C04-1-0013 | 464.138 | OH | a1 | B06 | b2 | C04 | COOMe |
| 2-1-b2C04-1-0014 | 465.138 | OH | a2 | B18 | b2 | C04 | COOMe |
| 2-1-b2C04-1-0015 | 470.109 | OH | a1 | B07 | b2 | C04 | COOMe |
| 2-1-b2C04-1-0016 | 483.109 | OH | a2 | B19 | b2 | C04 | COOMe |
| 2-1-b2C04-1-0017 | 491.095 | OH | a1 | B08 | b2 | C04 | COOMe |
| 2-1-b2C04-1-0018 | 499.104 | OH | a2 | B20 | b2 | C04 | COOMe |
| 2-1-b2C04-1-0019 | 502.115 | OH | a1 | B09 | b2 | C04 | COOMe |
| 2-1-b2C04-1-0020 | 518.110 | OH | a1 | B10 | b2 | C04 | COOMe |

[Table 320]

| [Table 8-3-2-6] Compound that may be contained in mixture 2-1-b2C05-1 | | | | | | | |
|---|---|---|---|---|---|---|---|
| ID | Exact Mass | | a | B | b | C | c |
| 2-1-b2C05-1-0001 | 412.142 | OH | a2 | B11 | b2 | C05 | COOEt |
| 2-1-b2C05-1-0002 | 413.138 | OH | a2 | B12 | b2 | C05 | COOEt |
| 2-1-b2C05-1-0003 | 426.158 | OH | a2 | B13 | b2 | C05 | COOEt |
| 2-1-b2C05-1-0004 | 430.133 | OH | a2 | B14 | b2 | C05 | COOEt |
| 2-1-b2C05-1-0005 | 431.148 | OH | a1 | B01 | b2 | C05 | COOEt |
| 2-1-b2C05-1-0006 | 432.143 | OH | a1 | B02 | b2 | C05 | COOEt |
| 2-1-b2C05-1-0007 | 440.174 | OH | a2 | B15 | b2 | C05 | COOEt |
| 2-1-b2C05-1-0008 | 442.153 | OH | a2 | B16 | b2 | C05 | COOEt |
| 2-1-b2C05-1-0009 | 445.164 | OH | a1 | B03 | b2 | C05 | COOEt |
| 2-1-b2C05-1-0010 | 448.123 | OH | a2 | B17 | b2 | C05 | COOEt |
| 2-1-b2C05-1-0011 | 449.139 | OH | a1 | B04 | b2 | C05 | COOEt |

(continued)

| [Table 8-3-2-6] Compound that may be contained in mixture 2-1-b2C05-1 | | | | | | | |
|---|---|---|---|---|---|---|---|
| ID | Exact Mass | | a | B | b | C | c |
| 2-1-b2C05-1-0012 | 459.179 | OH | a1 | B05 | b2 | C05 | COOEt |
| 2-1-b2C05-1-0013 | 461.159 | OH | a1 | B06 | b2 | C05 | COOEt |
| 2-1-b2C05-1-0014 | 462.158 | OH | a2 | B18 | b2 | C05 | COOEt |
| 2-1-b2C05-1-0015 | 467.129 | OH | a1 | B07 | b2 | C05 | COOEt |
| 2-1-b2C05-1-0016 | 480.130 | OH | a2 | B19 | b2 | C05 | COOEt |
| 2-1-b2C05-1-0017 | 488.115 | OH | a1 | B08 | b2 | C05 | COOEt |
| 2-1-b2C05-1-0018 | 496.125 | OH | a2 | B20 | b2 | C05 | COOEt |
| 2-1-b2C05-1-0019 | 499.136 | OH | a1 | B09 | b2 | C05 | COOEt |
| 2-1-b2C05-1-0020 | 515.130 | OH | a1 | B10 | b2 | C05 | COOEt |

[Table 321]

| [Table 8-3-2-7] Compound that may be contained in mixture 2-1-b2C06-1 | | | | | | | |
|---|---|---|---|---|---|---|---|
| ID | Exact Mass | | a | B | b | C | c |
| 2-1-b2C06-1-0001 | 403.088 | OH | a2 | B11 | b2 | C06 | COOEt |
| 2-1-b2C06-1-0002 | 404.083 | OH | a2 | B12 | b2 | C06 | COOEt |
| 2-1-b2C06-1-0003 | 417.103 | OH | a2 | B13 | b2 | C06 | COOEt |
| 2-1-b2C06-1-0004 | 421.078 | OH | a2 | B14 | b2 | C06 | COOEt |
| 2-1-b2C06-1-0005 | 422.094 | OH | a1 | B01 | b2 | C06 | COOEt |
| 2-1-b2C06-1-0006 | 423.089 | OH | a1 | B02 | b2 | C06 | COOEt |
| 2-1-b2C06-1-0007 | 431.119 | OH | a2 | B15 | b2 | C06 | COOEt |
| 2-1-b2C06-1-0008 | 433.098 | OH | a2 | B16 | b2 | C06 | COOEt |
| 2-1-b2C06-1-0009 | 436.109 | OH | a1 | B03 | b2 | C06 | COOEt |
| 2-1-b2C06-1-0010 | 439.069 | OH | a2 | B17 | b2 | C06 | COOEt |
| 2-1-b2C06-1-0011 | 440.084 | OH | a1 | B04 | b2 | C06 | COOEt |
| 2-1-b2C06-1-0012 | 450.125 | OH | a1 | B05 | b2 | C06 | COOEt |
| 2-1-b2C06-1-0013 | 452.104 | OH | a1 | B06 | b2 | C06 | COOEt |
| 2-1-b2C06-1-0014 | 453.103 | OH | a2 | B18 | b2 | C06 | COOEt |
| 2-1-b2C06-1-00 15 | 458.075 | OH | a1 | B07 | b2 | C06 | COOEt |
| 2-1-b2C06-1-0016 | 471.075 | OH | a2 | B19 | b2 | C06 | COOEt |
| 2-1-b2C06-1-0017 | 479.061 | OH | a1 | B08 | b2 | C06 | COOEt |
| 2-1-b2C06-1-00 18 | 487.070 | OH | a2 | B20 | b2 | C06 | COOEt |
| 2-1-b2C06-1-0019 | 490.081 | OH | a1 | B09 | b2 | C06 | COOEt |
| 2-1-b2C06-1-0020 | 506.076 | OH | a1 | B10 | b2 | C06 | COOEt |

[Table 322]

| [Table 8-3-2-8] Compound that may be contained in mixture 2-1-b2C07-1 | | | | | | | |
|---|---|---|---|---|---|---|---|
| ID | Exact Mass | | a | B | b | C | c |
| 2-1-b2C07-1-0001 | 429.194 | OH | a2 | B11 | b2 | C07 | COOEt |
| 2-1-b2C07-1-0002 | 430.189 | OH | a2 | B12 | b2 | C07 | COOEt |
| 2-1-b2C07-1-0003 | 443.210 | OH | a2 | B13 | b2 | C07 | COOEt |
| 2-1-b2C07-1-0004 | 447.185 | OH | a2 | B14 | b2 | C07 | COOEt |
| 2-1-b2C07-1-0005 | 448.200 | OH | a1 | B01 | b2 | C07 | COOEt |
| 2-1-b2C07-1-0006 | 449.195 | OH | a1 | B02 | b2 | C07 | COOEt |
| 2-1-b2C07-1-0007 | 457.225 | OH | a2 | B15 | b2 | C07 | COOEt |
| 2-1-b2C07-1-0008 | 459.205 | OH | a2 | B16 | b2 | C07 | COOEt |
| 2-1-b2C07-1-0009 | 462.215 | OH | a1 | B03 | b2 | C07 | COOEt |
| 2-1-b2C07-1-0010 | 465.175 | OH | a2 | B17 | b2 | C07 | COOEt |
| 2-1-b2C07-1-0011 | 466.190 | OH | a1 | B04 | b2 | C07 | COOEt |
| 2-1-b2C07-1-0012 | 476.231 | OH | a1 | B05 | b2 | C07 | COOEt |
| 2-1-b2C07-1-0013 | 478.210 | OH | a1 | B06 | b2 | C07 | COOEt |
| 2-1-b2C07-1-0014 | 479.21 | OH | a2 | B18 | b2 | C07 | COOEt |
| 2-1-b2C07-1-0015 | 484.181 | OH | a1 | B07 | b2 | C07 | COOEt |
| 2-1-b2C07-1-0016 | 497.181 | OH | a2 | B19 | b2 | C07 | COOEt |
| 2-1-b2C07-1-0017 | 505.167 | OH | a1 | B08 | b2 | C07 | COOEt |
| 2-1-b2C07-1-0018 | 513.176 | OH | a2 | B20 | b2 | C07 | COOEt |
| 2-1-b2C07-1-0019 | 516.187 | OH | a1 | B09 | b2 | C07 | COOEt |
| 2-1-b2C07-1-0020 | 532.182 | OH | a1 | B10 | b2 | C07 | COOEt |

[Table 323]

| [Table 8-3-2-9] Compound that may be contained in mixture 2-1-b2C19-1 | | | | | | | |
|---|---|---|---|---|---|---|---|
| ID | Exact Mass | | a | B | b | C | c |
| 2-1-b2C19-1-0001 | 379.190 | OH | a2 | B11 | b2 | C19 | Boc |
| 2-1-b2C19-1-0002 | 380.185 | OH | a2 | B12 | b2 | C19 | Boc |
| 2-1-b2C19-1-0003 | 387.138 | OH | a2 | B13 | b2 | C19 | Boc |
| 2-1-b2C19-1-0004 | 393.205 | OH | a2 | B14 | b2 | C19 | Boc |
| 2-1-b2C19-1-0005 | 396.184 | OH | a1 | B01 | b2 | C19 | Boc |
| 2-1-b2C19-1-0006 | 396.184 | OH | a1 | B02 | b2 | C19 | Boc |
| 2-1-b2C19-1-0007 | 400.159 | OH | a2 | B15 | b2 | C19 | Boc |
| 2-1-b2C19-1-0008 | 402.169 | OH | a2 | B16 | b2 | C19 | Boc |
| 2-1-b2C19-1-0009 | 408.220 | OH | a1 | B03 | b2 | C19 | Boc |
| 2-1-b2C19-1-0010 | 410.199 | OH | a2 | B17 | b2 | C19 | Boc |
| 2-1-b2C19-1-0011 | 412.195 | OH | a1 | B04 | b2 | C19 | Boc |
| 2-1-b2C19-1-0012 | 418.164 | OH | a1 | B05 | b2 | C19 | Boc |
| 2-1-b2C19-1-0013 | 423.139 | OH | a1 | B06 | b2 | C19 | Boc |
| 2-1-b2C19-1-0014 | 427.226 | OH | a2 | B18 | b2 | C19 | Boc |
| 2-1-b2C19-1-0015 | 430.205 | OH | a1 | B07 | b2 | C19 | Boc |
| 2-1-b2C19-1-0016 | 444.201 | OH | a2 | B19 | b2 | C19 | Boc |
| 2-1-b2C19-1-0017 | 452.135 | OH | a1 | B08 | b2 | C19 | Boc |
| 2-1-b2C19-1-0018 | 458.141 | OH | a2 | B20 | b2 | C19 | Boc |
| 2-1-b2C19-1-0019 | 464.171 | OH | a1 | B09 | b2 | C19 | Boc |
| 2-1-b2C19-1-0020 | 481.198 | OH | a1 | B10 | b2 | C19 | Boc |

[Table 324]

| [Table 8-3-2-10] Compound that may be contained in mixture 2-1-b2C21-1 | | | | | | | |
|---|---|---|---|---|---|---|---|
| ID | Exact Mass | | a | B | b | C | c |
| 2-1-b2C21-1-0001 | 387.158 | OH | a2 | B11 | b2 | C21 | Boc |
| 2-1-b2C21-1-0002 | 388.154 | OH | a2 | B12 | b2 | C21 | Boc |
| 2-1-b2C21-1-0003 | 400.159 | OH | a2 | B13 | b2 | C21 | Boc |
| 2-1-b2C21-1-0004 | 406.144 | OH | a2 | B14 | b2 | C21 | Boc |
| 2-1-b2C21-1-0005 | 408.220 | OH | a1 | B01 | b2 | C21 | Boc |
| 2-1-b2C21-1-0006 | 409.215 | OH | a1 | B02 | b2 | C21 | Boc |
| 2-1-b2C21-1-0007 | 415.190 | OH | a2 | B15 | b2 | C21 | Boc |
| 2-1-b2C21-1-0008 | 416.154 | OH | a2 | B16 | b2 | C21 | Boc |
| 2-1-b2C21-1-0009 | 418.168 | OH | a1 | B03 | b2 | C21 | Boc |
| 2-1-b2C21-1-0010 | 424.215 | OH | a2 | B17 | b2 | C21 | Boc |
| 2-1-b2C21-1-0011 | 427.226 | OH | a1 | B04 | b2 | C21 | Boc |
| 2-1-b2C21-1-0012 | 435.176 | OH | a1 | B05 | b2 | C21 | Boc |
| 2-1-b2C21-1-0013 | 436.251 | OH | a1 | B06 | b2 | C21 | Boc |

(continued)

| [Table 8-3-2-10] Compound that may be contained in mixture 2-1-b2C21-1 | | | | | | | |
|---|---|---|---|---|---|---|---|
| ID | Exact Mass | | a | B | b | C | c |
| 2-1-b2C21-1-0014 | 437.210 | OH | a2 | B18 | b2 | C21 | Boc |
| 2-1-b2C21-1-0015 | 445.217 | OH | a1 | B07 | b2 | C21 | Boc |
| 2-1-b2C21-1-0016 | 456.162 | OH | a2 | B19 | b2 | C21 | Boc |
| 2-1-b2C21-1-0017 | 466.226 | OH | a1 | B08 | b2 | C21 | Boc |
| 2-1-b2C21-1-0018 | 471.141 | OH | a2 | B20 | b2 | C21 | Boc |
| 2-1-b2C21-1-0019 | 473.136 | OH | a1 | B09 | b2 | C21 | Boc |
| 2-1-b2C21-1-0020 | 492.202 | OH | a1 | B10 | b2 | C21 | Boc |

[Table 325]

| [Table 8-3-2-11] Compound that may be contained in mixture 2-1-b2C23-1 | | | | | | | |
|---|---|---|---|---|---|---|---|
| ID | Exact Mass | | a | B | b | C | c |
| 2-1-b2C23-1-0001 | 394.205 | OH | a2 | B11 | b2 | C23 | Boc |
| 2-1-b2C23-1-0002 | 395.200 | OH | a2 | B12 | b2 | C23 | Boc |
| 2-1-b2C23-1-0003 | 406.144 | OH | a2 | B13 | b2 | C23 | Boc |
| 2-1-b2C23-1-0004 | 412.179 | OH | a2 | B14 | b2 | C23 | Boc |
| 2-1-b2C23-1-0005 | 413.210 | OH | a1 | B01 | b2 | C23 | Boc |
| 2-1-b2C23-1-0006 | 413.210 | OH | a1 | B02 | b2 | C23 | Boc |
| 2-1-b2C23-1-0007 | 418.149 | OH | a2 | B15 | b2 | C23 | Boc |
| 2-1-b2C23-1-0008 | 419.165 | OH | a2 | B16 | b2 | C23 | Boc |
| 2-1-b2C23-1-0009 | 428.171 | OH | a1 | B03 | b2 | C23 | Boc |
| 2-1-b2C23-1-0010 | 428.221 | OH | a2 | B17 | b2 | C23 | Boc |
| 2-1-b2C23-1-0011 | 431.185 | OH | a1 | B04 | b2 | C23 | Boc |
| 2-1-b2C23-1-0012 | 441.130 | OH | a1 | B05 | b2 | C23 | Boc |
| 2-1-b2C23-1-0013 | 443.221 | OH | a1 | B06 | b2 | C23 | Boc |
| 2-1-b2C23-1-0014 | 444.220 | OH | a2 | B18 | b2 | C23 | Boc |
| 2-1-b2C23-1-0015 | 447.179 | OH | a1 | B07 | b2 | C23 | Boc |
| 2-1-b2C23-1-0016 | 463.207 | OH | a2 | B19 | b2 | C23 | Boc |
| 2-1-b2C23-1-0017 | 469.237 | OH | a1 | B08 | b2 | C23 | Boc |
| 2-1-b2C23-1-0018 | 476.208 | OH | a2 | B20 | b2 | C23 | Boc |
| 2-1-b2C23-1-0019 | 484.193 | OH | a1 | B09 | b2 | C23 | Boc |
| 2-1-b2C23-1-0020 | 497.193 | OH | a1 | B10 | b2 | C23 | Boc |

[Table 326]

| [Table 8-3-2-12] Compound that may be contained in mixture 2-1-b2C25-1 | | | | | | | |
|---|---|---|---|---|---|---|---|
| ID | Exact Mass | | a | B | b | C | c |
| 2-1-b2C25-1-0001 | 402.169 | OH | a2 | B11 | b2 | C25 | Boc |
| 2-1-b2C25-1-0002 | 404.134 | OH | a2 | B12 | b2 | C25 | Boc |
| 2-1-b2C25-1-0003 | 416.154 | OH | a2 | B13 | b2 | C25 | Boc |
| 2-1-b2C25-1-0004 | 419.165 | OH | a2 | B14 | b2 | C25 | Boc |
| 2-1-b2C25-1-0005 | 423.139 | OH | a1 | B01 | b2 | C25 | Boc |
| 2-1-b2C25-1-0006 | 426.194 | OH | a1 | B02 | b2 | C25 | Boc |
| 2-1-b2C25-1-0007 | 433.180 | OH | a2 | B15 | b2 | C25 | Boc |
| 2-1-b2C25-1-0008 | 435.159 | OH | a2 | B16 | b2 | C25 | Boc |
| 2-1-b2C25-1-0009 | 437.135 | OH | a1 | B03 | b2 | C25 | Boc |
| 2-1-b2C25-1-0010 | 441.242 | OH | a2 | B17 | b2 | C25 | Boc |
| 2-1-b2C25-1-0011 | 444.126 | OH | a1 | B04 | b2 | C25 | Boc |
| 2-1-b2C25-1-0012 | 454.130 | OH | a1 | B05 | b2 | C25 | Boc |
| 2-1-b2C25-1-0013 | 454.206 | OH | a1 | B06 | b2 | C25 | Boc |
| 2-1-b2C25-1-0014 | 455.221 | OH | a2 | B18 | b2 | C25 | Boc |
| 2-1-b2C25-1-0015 | 462.192 | OH | a1 | B07 | b2 | C25 | Boc |
| 2-1-b2C25-1-0016 | 472.136 | OH | a2 | B19 | b2 | C25 | Boc |
| 2-1-b2C25-1-0017 | 485.156 | OH | a1 | B08 | b2 | C25 | Boc |
| 2-1-b2C25-1-0018 | 489.131 | OH | a2 | B20 | b2 | C25 | Boc |
| 2-1-b2C25-1-0019 | 495.213 | OH | a1 | B09 | b2 | C25 | Boc |
| 2-1-b2C25-1-0020 | 512.188 | OH | a1 | B10 | b2 | C25 | Boc |

[Table 327]

| [Table 8-3-2-13] Compound that may be contained in mixture 2-1-b2C27-1 | | | | | | | |
|---|---|---|---|---|---|---|---|
| ID | Exact Mass | | a | B | b | C | c |
| 2-1-b2C27-1-0001 | 380.189 | OH | a2 | B11 | b2 | C27 | Boc |
| 2-1-b2C27-1-0002 | 381.184 | OH | a2 | B12 | b2 | C27 | Boc |
| 2-1-b2C27-1-0003 | 388.154 | OH | a2 | B13 | b2 | C27 | Boc |
| 2-1-b2C27-1-0004 | 396.165 | OH | a2 | B14 | b2 | C27 | Boc |
| 2-1-b2C27-1-0005 | 397.179 | OH | a1 | B01 | b2 | C27 | Boc |
| 2-1-b2C27-1-0006 | 397.180 | OH | a1 | B02 | b2 | C27 | Boc |
| 2-1-b2C27-1-0007 | 401.174 | OH | a2 | B15 | b2 | C27 | Boc |
| 2-1-b2C27-1-0008 | 405.129 | OH | a2 | B16 | b2 | C27 | Boc |
| 2-1-b2C27-1-0009 | 410.199 | OH | a1 | B03 | b2 | C27 | Boc |
| 2-1-b2C27-1-0010 | 412.179 | OH | a2 | B17 | b2 | C27 | Boc |
| 2-1-b2C27-1-0011 | 414.174 | OH | a1 | B04 | b2 | C27 | Boc |
| 2-1-b2C27-1-0012 | 422.236 | OH | a1 | B05 | b2 | C27 | Boc |
| 2-1-b2C27-1-0013 | 426.211 | OH | a1 | B06 | b2 | C27 | Boc |

(continued)

| [Table 8-3-2-13] Compound that may be contained in mixture 2-1-b2C27-1 | | | | | | | |
|---|---|---|---|---|---|---|---|
| ID | Exact Mass | | a | B | b | C | c |
| 2-1-b2C27-1-0014 | 427.226 | OH | a2 | B18 | b2 | C27 | Boc |
| 2-1-b2C27-1-0015 | 431.201 | OH | a1 | B07 | b2 | C27 | Boc |
| 2-1-b2C27-1-0016 | 445.200 | OH | a2 | B19 | b2 | C27 | Boc |
| 2-1-b2C27-1-0017 | 453.150 | OH | a1 | B08 | b2 | C27 | Boc |
| 2-1-b2C27-1-0018 | 462.116 | OH | a2 | B20 | b2 | C27 | Boc |
| 2-1-b2C27-1-0019 | 466.150 | OH | a1 | B09 | b2 | C27 | Boc |
| 2-1-b2C27-1-0020 | 483.177 | OH | a1 | B10 | b2 | C27 | Boc |

[Table 328]

| [Table 8-3-2-14] Compound that may be contained in mixture 2-1-b2C33-1 | | | | | | | |
|---|---|---|---|---|---|---|---|
| ID | Exact Mass | | a | B | b | C | c |
| 2-1-b2C33-1-0001 | 360.184 | OH | a2 | B11 | b2 | C33 | Boc |
| 2-1-b2C33-1-0002 | 361.179 | OH | a2 | B12 | b2 | C33 | Boc |
| 2-1-b2C33-1-0003 | 368.152 | OH | a2 | B13 | b2 | C33 | Boc |
| 2-1-b2C33-1-0004 | 369.148 | OH | a2 | B14 | b2 | C33 | Boc |
| 2-1-b2C33-1-0005 | 369.148 | OH | a1 | B01 | b2 | C33 | Boc |
| 2-1-b2C33-1-0006 | 370.143 | OH | a1 | B02 | b2 | C33 | Boc |
| 2-1-b2C33-1-0007 | 382.168 | OH | a2 | B15 | b2 | C33 | Boc |
| 2-1-b2C33-1-0008 | 382.168 | OH | a2 | B16 | b2 | C33 | Boc |
| 2-1-b2C33-1-0009 | 383.163 | OH | a1 | B03 | b2 | C33 | Boc |
| 2-1-b2C33-1-0010 | 387.138 | OH | a2 | B17 | b2 | C33 | Boc |
| 2-1-b2C33-1-0011 | 388.215 | OH | a1 | B04 | b2 | C33 | Boc |
| 2-1-b2C33-1-0012 | 398.163 | OH | a1 | B05 | b2 | C33 | Boc |
| 2-1-b2C33-1-0013 | 399.158 | OH | a1 | B06 | b2 | C33 | Boc |
| 2-1-b2C33-1-0014 | 401.174 | OH | a2 | B18 | b2 | C33 | Boc |
| 2-1-b2C33-1-0015 | 408.220 | OH | a1 | B07 | b2 | C33 | Boc |
| 2-1-b2C33-1-0016 | 417.185 | OH | a2 | B19 | b2 | C33 | Boc |
| 2-1-b2C33-1-0017 | 429.205 | OH | a1 | B08 | b2 | C33 | Boc |
| 2-1-b2C33-1-0018 | 436.157 | OH | a2 | B20 | b2 | C33 | Boc |
| 2-1-b2C33-1-0019 | 438.231 | OH | a1 | B09 | b2 | C33 | Boc |
| 2-1-b2C33-1-0020 | 455.257 | OH | a1 | B10 | b2 | C33 | Boc |

Example 8-3-3: Formation of linker b3 by step b3

A reaction formula in step b3 performed using BB2-1-35 will be illustrated below.

[Formula 618]

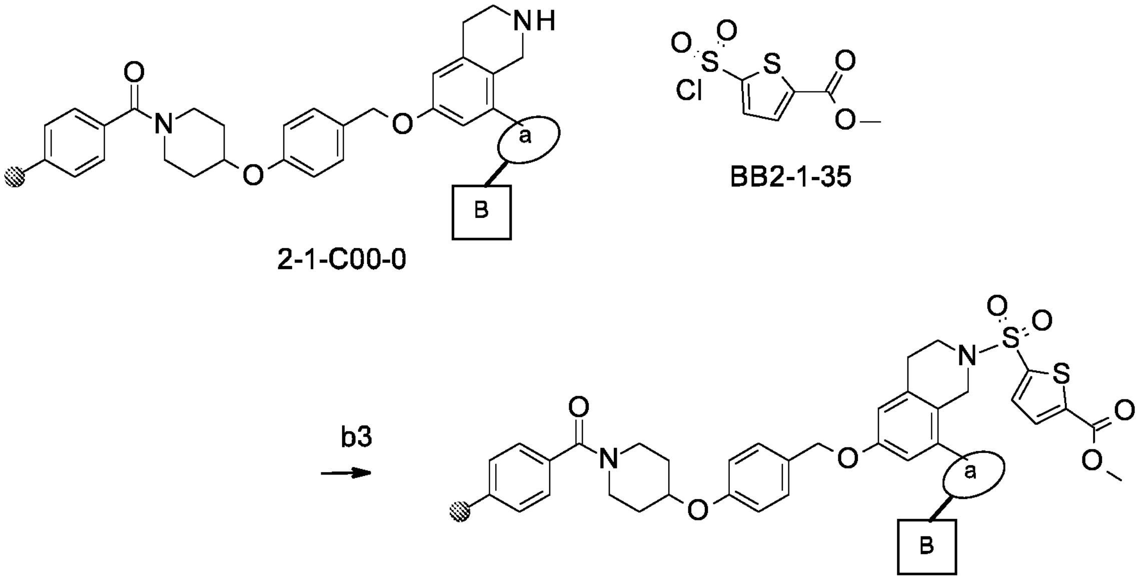


2-1-C00-0

BB2-1-35

2-1-b3C06-0

Mixture 2-1-b3C06-0 to mixture 2-1-b3C18-0 were synthesized as follows using BB2-1-35 to BB2-1-40.

2-1-c00-0 (135 mg, amount of the mixture supported on the solid phase: 0.200 mmol/g), tetrahydrofuran (2.7 mL), and DIPEA (70.7 μL) were added to six 4 mL glass vials and shaken at room temperature for 1 hour. Separate sulfonyl chlorides (0.270 mmol) described in Table 8-3-3-1 were added to the vials, respectively, and each mixture was shaken at 50°C for 15 hours.

Solid-phase purification

The suspension of the reaction solution and the resin was transferred to a 6 mL column with a filter, filtered, and washed three times with NMP/water = 1/1 (2.7 mL), three times with NMP (2.7 mL), three times with 0.05 M $TBAHSO_4$ + DTBP in NMP (2.7 mL), three times with 0.05 M NMM in NMP (2.7 mL), three times with NMP/water = 1/1 (2.7 mL), three times with NMP (2.7 mL), three times with MeOH (2.7 mL), three times with DCM (2.7 mL), and three times with heptane (2.7 mL), and the obtained solid phase was dried under reduced pressure to obtain the compounds described in the table.

[Table 329]

| [Table 8-3-3-1] Reagent used and obtained compound in step b3 | | | |
|---|---|---|---|
| Sulfonyl chloride | Molar number | Amount used | Obtained mixture |
| BB2-1-35 | 0.270 mmol | 65.0 mg | 2-1-b3C06-0 |
| BB2-1-37 | 0.270 mmol | 64.4 mg | 2-1-b3C15-0 |
| BB2-1-38 | 0.270 mmol | 82.0 mg | 2-1-b3C 16-0 |
| BB2-1-39 | 0.270 mmol | 82.0 mg | 2-1-b3C17-0 |
| BB2-1-40 | 0.270 mmol | 86.0 mg | 2-1-b3C18-0 |
| BB2-1-36 | 0.270 mmol | 63.4 mg | 2-1-b3C08-0 |

Mixture 2-1-b3C06-0 to mixture 2-1-b3C18-0 can be treated by cleavage in the same manner as in Example 8-1-1 to obtain mixture 2-1-b3C06-1 to mixture 2-1-b3C 18-1. The details of the mixtures are shown in Table 8-3-3-2 to Table

8-3-3-7.

A notation method in each table will be described. In Table 8-3-3-2 to Table 8-3-3- 7, each compound that may be contained in mixture 2-1-b3C06-1 to mixture 2-1-b3C18-1 is indicated by ID, and core blocks and linkers in combination in the structures of mixture 2-1- b3C06-1 to mixture 2-1-b3C18-1 are indicated by symbols or chemical formulas. The correspondence between the symbols and specific structures are shown in Figure 4. In Table 8-3-3-2 to Table 8-3-3-7, "×" and "c" are represented by chemical formulas.

[Formula 619]

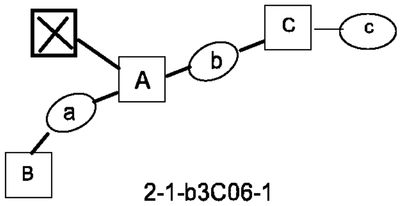


2-1-b3C06-1

For example, when ID is 2-1-b3C06-1-0001, the compound is represented by the following structural formula.

[Formula 620]

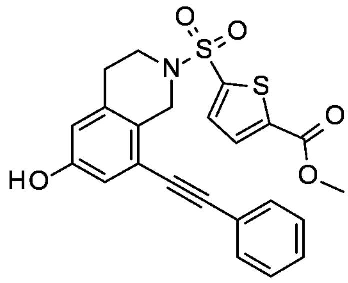

2-1-b3C06-1-0001

2-1-b3C06-1-0001

[Table 330]

| Notation in [Table 8-3-3-2] | | | | | | |
|---|---|---|---|---|---|---|
| ID | | a | B | b | C | c |
| 2-1-b3C06-1-0001 | OH | a2 | B11 | b3 | C06 | COOMe |

[Table 331]

| [Table 8-3-3-2] Compound that may be contained in mixture 2-1-b3C06-1 | | | | | | | |
|---|---|---|---|---|---|---|---|
| ID | Exact Mass | | a | B | b | C | c |
| 2-1-b3C06-1-0001 | 439.055 | OH | a2 | B11 | b3 | C06 | COOMe |
| 2-1-b3C06-1-0002 | 440.050 | OH | a2 | B12 | b3 | C06 | COOMe |
| 2-1-b3C06-1-0003 | 453.070 | OH | a2 | B13 | b3 | C06 | COOMe |
| 2-1-b3C06-1-0004 | 457.045 | OH | a2 | B14 | b3 | C06 | COOMe |
| 2-1-b3C06-1-0005 | 458.061 | OH | a1 | B01 | b3 | C06 | COOMe |

(continued)

| [Table 8-3-3-2] Compound that may be contained in mixture 2-1-b3C06-1 | | | | | | | |
|---|---|---|---|---|---|---|---|
| ID | Exact Mass | | a | B | b | C | c |
| 2-1-b3C06-1-0006 | 459.056 | OH | a1 | B02 | b3 | C06 | COOMe |
| 2-1-b3C06-1-0007 | 467.086 | OH | a2 | B15 | b3 | C06 | COOMe |
| 2-1-b3C06-1-0008 | 469.065 | OH | a2 | B16 | b3 | C06 | COOMe |
| 2-1-b3C06-1-0009 | 472.076 | OH | a1 | B03 | b3 | C06 | COOMe |
| 2-1-b3C06-1-0010 | 475.036 | OH | a2 | B17 | b3 | C06 | COOMe |
| 2-1-b3C06-1-0011 | 476.051 | OH | a1 | B04 | b3 | C06 | COOMe |
| 2-1-b3C06-1-0012 | 486.092 | OH | a1 | B05 | b3 | C06 | COOMe |
| 2-1-b3C06-1-0013 | 488.071 | OH | a1 | B06 | b3 | C06 | COOMe |
| 2-1-b3C06-1-0014 | 489.070 | OH | a2 | B18 | b3 | C06 | COOMe |
| 2-1-b3C06-1-0015 | 494.042 | OH | a1 | B07 | b3 | C06 | COOMe |
| 2-1-b3C06-1-0016 | 507.042 | OH | a2 | B19 | b3 | C06 | COOMe |
| 2-1-b3C06-1-0017 | 515.028 | OH | a1 | B08 | b3 | C06 | COOMe |
| 2-1-b3C06-1-0018 | 523.037 | OH | a2 | B20 | b3 | C06 | COOMe |
| 2-1-b3C06-1-0019 | 526.048 | OH | a1 | B09 | b3 | C06 | COOMe |
| 2-1-b3C06-1-0020 | 542.043 | OH | a1 | B10 | b3 | C06 | COOMe |

[Table 332]

| [Table 8-3-3-3] Compound that may be contained in mixture 2-1-b3C08-1 | | | | | | | |
|---|---|---|---|---|---|---|---|
| ID | Exact Mass | | a | B | b | C | c |
| 2-1-b3C08-1-0001 | 433.098 | OH | a2 | B11 | b3 | C08 | COOMe |
| 2-1-b3C08-1-0002 | 434.094 | OH | a2 | B12 | b3 | C08 | COOMe |
| 2-1-b3C08-1-0003 | 447.114 | OH | a2 | B13 | b3 | C08 | COOMe |
| 2-1-b3C08-1-0004 | 451.089 | OH | a2 | B14 | b3 | C08 | COOMe |
| 2-1-b3C08-1-0005 | 452.104 | OH | a1 | B01 | b3 | C08 | COOMe |
| 2-1-b3C08-1-0006 | 453.099 | OH | a1 | B02 | b3 | C08 | COOMe |
| 2-1-b3C08-1-0007 | 461.130 | OH | a2 | B15 | b3 | C08 | COOMe |
| 2-1-b3C08-1-0008 | 463.109 | OH | a2 | B16 | b3 | C08 | COOMe |
| 2-1-b3C08-1-0009 | 466.12 | OH | a1 | B03 | b3 | C08 | COOMe |
| 2-1-b3C08-1-0010 | 469.080 | OH | a2 | B17 | b3 | C08 | COOMe |
| 2-1-b3C08-1-0011 | 470.095 | OH | a1 | B04 | b3 | C08 | COOMe |
| 2-1-b3C08-1-0012 | 480.136 | OH | a1 | B05 | b3 | C08 | COOMe |
| 2-1-b3C08-1-0013 | 482.115 | OH | a1 | B06 | b3 | C08 | COOMe |
| 2-1-b3C08-1-0014 | 483.114 | OH | a2 | B18 | b3 | C08 | COOMe |
| 2-1-b3C08-1-0015 | 488.085 | OH | a1 | B07 | b3 | C08 | COOMe |
| 2-1-b3C08-1-0016 | 501.086 | OH | a2 | B19 | b3 | C08 | COOMe |
| 2-1-b3C08-1-0017 | 509.072 | OH | a1 | B08 | b3 | C08 | COOMe |
| 2-1-b3C08-1-0018 | 517.081 | OH | a2 | B20 | b3 | C08 | COOMe |

(continued)

| [Table 8-3-3-3] Compound that may be contained in mixture 2-1-b3C08-1 | | | | | | | |
|---|---|---|---|---|---|---|---|
| ID | Exact Mass | | a | B | b | C | c |
| 2-1-b3C08-1-0019 | 520.092 | OH | a1 | B09 | b3 | C08 | COOMe |
| 2-1-b3C08-1-0020 | 536.087 | OH | a1 | B10 | b3 | C08 | COOMe |

[Table 333]

| [Table 8-3-3-4] Compound that may be contained in mixture 2-1-b3C15-1 | | | | | | | |
|---|---|---|---|---|---|---|---|
| ID | Exact Mass | | a | B | b | C | c |
| 2-1-b3C15-1-0001 | 423.078 | OH | a2 | B11 | b3 | C15 | COOEt |
| 2-1-b3C15-1-0002 | 424.073 | OH | a2 | B12 | b3 | C15 | COOEt |
| 2-1-b3C15-1-0003 | 437.093 | OH | a2 | B13 | b3 | C15 | COOEt |
| 2-1-b3C15-1-0004 | 441.068 | OH | a2 | B14 | b3 | C15 | COOEt |
| 2-1-b3C15-1-0005 | 442.083 | OH | a1 | B01 | b3 | C15 | COOEt |
| 2-1-b3C15-1-0006 | 443.079 | OH | a1 | B02 | b3 | C15 | COOEt |
| 2-1-b3C15-1-0007 | 451.109 | OH | a2 | B15 | b3 | C15 | COOEt |
| 2-1-b3C15-1-0008 | 453.088 | OH | a2 | B16 | b3 | C15 | COOEt |
| 2-1-b3C15-1-0009 | 456.099 | OH | a1 | B03 | b3 | C15 | COOEt |
| 2-1-b3C15-1-0010 | 459.059 | OH | a2 | B17 | b3 | C15 | COOEt |
| 2-1-b3C15-1-0011 | 460.074 | OH | a1 | B04 | b3 | C15 | COOEt |
| 2-1-b3C15-1-0012 | 470.115 | OH | a1 | B05 | b3 | C15 | COOEt |
| 2-1-b3C15-1-0013 | 472.094 | OH | a1 | B06 | b3 | C15 | COOEt |
| 2-1-b3C15-1-0014 | 473.093 | OH | a2 | B18 | b3 | C15 | COOEt |
| 2-1-b3C15-1-0015 | 478.065 | OH | a1 | B07 | b3 | C15 | COOEt |
| 2-1-b3C15-1-0016 | 491.065 | OH | a2 | B19 | b3 | C15 | COOEt |
| 2-1-b3C15-1-0017 | 499.051 | OH | a1 | B08 | b3 | C15 | COOEt |
| 2-1-b3C15-1-0018 | 507.060 | OH | a2 | B20 | b3 | C15 | COOEt |
| 2-1-b3C15-1-0019 | 510.071 | OH | a1 | B09 | b3 | C15 | COOEt |
| 2-1-b3C15-1-0020 | 526.066 | OH | a1 | B10 | b3 | C15 | COOEt |

[Table 334]

| [Table 8-3-3-5] Compound that may be contained in mixture 2-1-b3C16-1 | | | | | | | |
|---|---|---|---|---|---|---|---|
| ID | Exact Mass | | a | B | b | C | c |
| 2-1-b3C16-1-0001 | 487.109 | OH | a2 | B11 | b3 | C16 | COOEt |
| 2-1-b3C16-1-0002 | 488.104 | OH | a2 | B12 | b3 | C16 | COOEt |
| 2-1-b3C16-1-0003 | 501.125 | OH | a2 | B13 | b3 | C16 | COOEt |
| 2-1-b3C16-1-0004 | 505.100 | OH | a2 | B14 | b3 | C16 | COOEt |
| 2-1-b3C16-1-0005 | 506.115 | OH | a1 | B01 | b3 | C16 | COOEt |

(continued)

| [Table 8-3-3-5] Compound that may be contained in mixture 2-1-b3C16-1 | | | | | | | |
|---|---|---|---|---|---|---|---|
| ID | Exact Mass | | a | B | b | C | c |
| 2-1-b3C16-1-0006 | 507.110 | OH | a1 | B02 | b3 | C16 | COOEt |
| 2-1-b3C16-1-0007 | 515.140 | OH | a2 | B15 | b3 | C16 | COOEt |
| 2-1-b3C16-1-0008 | 517.120 | OH | a2 | B16 | b3 | C16 | COOEt |
| 2-1-b3C16-1-0009 | 520.130 | OH | a1 | B03 | b3 | C16 | COOEt |
| 2-1-b3C16-1-0010 | 523.090 | OH | a2 | B17 | b3 | C16 | COOEt |
| 2-1-b3C16-1-0011 | 524.105 | OH | a1 | B04 | b3 | C16 | COOEt |
| 2-1-b3C16-1-0012 | 534.146 | OH | a1 | B05 | b3 | C16 | COOEt |
| 2-1-b3C16-1-0013 | 536.125 | OH | a1 | B06 | b3 | C16 | COOEt |
| 2-1-b3C16-1-0014 | 537.125 | OH | a2 | B18 | b3 | C16 | COOEt |
| 2-1-b3C16-1-0015 | 542.096 | OH | a1 | B07 | b3 | C16 | COOEt |
| 2-1-b3C16-1-0016 | 555.096 | OH | a2 | B19 | b3 | C16 | COOEt |
| 2-1-b3C16-1-0017 | 563.082 | OH | a1 | B08 | b3 | C16 | COOEt |
| 2-1-b3C16-1-0018 | 571.091 | OH | a2 | B20 | b3 | C16 | COOEt |
| 2-1-b3C16-1-0019 | 574.102 | OH | a1 | B09 | b3 | C16 | COOEt |
| 2-1-b3C16-1-0020 | 590.097 | OH | a1 | B10 | b3 | C16 | COOEt |

[Table 335]

| [Table 8-3-3-6] Compound that may be contained in mixture 2-1-b3C17-1 | | | | | | | |
|---|---|---|---|---|---|---|---|
| ID | Exact Mass | | a | B | b | C | c |
| 2-1-b3C17-1-0001 | 489.070 | OH | a2 | B11 | b3 | C17 | COOEt |
| 2-1-b3C17-1-0002 | 490.066 | OH | a2 | B12 | b3 | C17 | COOEt |
| 2-1-b3C17-1-0003 | 503.086 | OH | a2 | B13 | b3 | C17 | COOEt |
| 2-1-b3C17-1-0004 | 507.061 | OH | a2 | B14 | b3 | C17 | COOEt |
| 2-1-b3C17-1-0005 | 508.076 | OH | a1 | B01 | b3 | C17 | COOEt |
| 2-1-b3C17-1-0006 | 509.072 | OH | a1 | B02 | b3 | C17 | COOEt |
| 2-1-b3C17-1-0007 | 517.102 | OH | a2 | B15 | b3 | C17 | COOEt |
| 2-1-b3C17-1-0008 | 519.081 | OH | a2 | B16 | b3 | C17 | COOEt |
| 2-1-b3C17-1-0009 | 522.092 | OH | a1 | B03 | b3 | C17 | COOEt |
| 2-1-b3C17-1-0010 | 525.052 | OH | a2 | B17 | b3 | C17 | COOEt |
| 2-1-b3C17-1-0011 | 526.067 | OH | a1 | B04 | b3 | C17 | COOEt |
| 2-1-b3C17-1-0012 | 536.108 | OH | a1 | B05 | b3 | C17 | COOEt |
| 2-1-b3C17-1-0013 | 538.087 | OH | a1 | B06 | b3 | C17 | COOEt |
| 2-1-b3C17-1-0014 | 539.086 | OH | a2 | B18 | b3 | C17 | COOEt |
| 2-1-b3C17-1-0015 | 544.057 | OH | a1 | B07 | b3 | C17 | COOEt |
| 2-1-b3C17-1-0016 | 557.058 | OH | a2 | B19 | b3 | C17 | COOEt |
| 2-1-b3C17-1-0017 | 565.044 | OH | a1 | B08 | b3 | C17 | COOEt |
| 2-1-b3C17-1-0018 | 573.053 | OH | a2 | B20 | b3 | C17 | COOEt |
| 2-1-b3C17-1-0019 | 576.064 | OH | a1 | B09 | b3 | C17 | COOEt |

(continued)

| [Table 8-3-3-6] Compound that may be contained in mixture 2-1-b3C17-1 | | | | | | | |
|---|---|---|---|---|---|---|---|
| ID | Exact Mass | | a | B | b | C | c |
| 2-1-b3C17-1-0020 | 592.059 | OH | a1 | B10 | b3 | C17 | COOEt |

[Table 336]

| [Table 8-3-3-7] Compound that may be contained in mixture 2-1-b3C 18-1 | | | | | | | |
|---|---|---|---|---|---|---|---|
| ID | Exact Mass | | a | B | b | C | c |
| 2-1-b3C18-1-0001 | 504.081 | OH | a2 | B11 | b3 | C18 | COOEt |
| 2-1-b3C18-1-0002 | 505.077 | OH | a2 | B12 | b3 | C18 | COOEt |
| 2-1-b3C18-1-0003 | 518.097 | OH | a2 | B13 | b3 | C18 | COOEt |
| 2-1-b3C18-1-0004 | 522.072 | OH | a2 | B14 | b3 | C18 | COOEt |
| 2-1-b3C18-1-0005 | 523.087 | OH | a1 | B01 | b3 | C18 | COOEt |
| 2-1-b3C18-1-0006 | 524.082 | OH | a1 | B02 | b3 | C18 | COOEt |
| 2-1-b3C18-1-0007 | 532.113 | OH | a2 | B15 | b3 | C18 | COOEt |
| 2-1-b3C18-1-0008 | 534.092 | OH | a2 | B16 | b3 | C18 | COOEt |
| 2-1-b3C18-1-0009 | 537.103 | OH | a1 | B03 | b3 | C18 | COOEt |
| 2-1-b3C18-1-0010 | 540.063 | OH | a2 | B17 | b3 | C18 | COOEt |
| 2-1-b3C18-1-0011 | 541.078 | OH | a1 | B04 | b3 | C18 | COOEt |
| 2-1-b3C18-1-0012 | 551.118 | OH | a1 | B05 | b3 | C18 | COOEt |
| 2-1-b3C18-1-0013 | 553.098 | OH | a1 | B06 | b3 | C18 | COOEt |
| 2-1-b3C18-1-0014 | 554.097 | OH | a2 | B18 | b3 | C18 | COOEt |
| 2-1-b3C18-1-0015 | 559.068 | OH | a1 | B07 | b3 | C18 | COOEt |
| 2-1-b3C18-1-0016 | 572.069 | OH | a2 | B19 | b3 | C18 | COOEt |
| 2-1-b3C18-1-0017 | 580.054 | OH | a1 | B08 | b3 | C18 | COOEt |
| 2-1-b3C18-1-0018 | 588.064 | OH | a2 | B20 | b3 | C18 | COOEt |
| 2-1-b3C18-1-0019 | 591.075 | OH | a1 | B09 | b3 | C18 | COOEt |
| 2-1-b3C18-1-0020 | 607.069 | OH | a1 | B10 | b3 | C18 | COOEt |

Example 8-4-1: Hydrolysis by steps h02 to h21

A reaction formula in step h02 performed using mixture 2-1-b1C08-0 will be illustrated below.

[Formula 621]

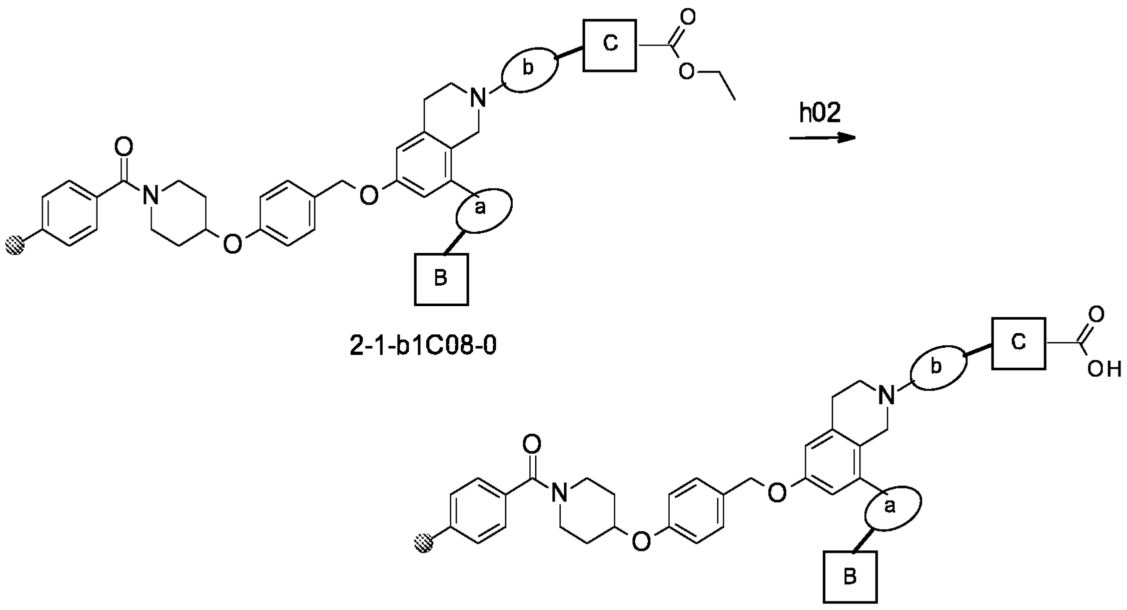


2-1-b1C08-0

2-1-h02C08-0

Mixture h02C08-0 to mixture h21C18-0 were synthesized as follows using the mixtures described in Table 8-4-1-1.

NMP (1.89 mL) and the alcohols (0.540 mL) shown in Table 8-4-1-1 were added to twenty 6 mL columns with a filter containing the mixtures described in Table 8-4-1-1, and shaken at room temperature for 1 hour. A 1 M aqueous TBAOH solution was added thereto in the amounts described in Table 8-4-1-1, and each mixture was shaken using the temperatures and the times described in Table 8-4-1-1.

Solid-phase purification

The suspension of the reaction solution and the resin was filtered through the filter of the column and washed three times with NMP/water = 1/1 (2.7 mL), three times with 0.05 M $TBAHSO_4$ + DTBP in NMP (2.7 mL), three times with NMP/water = 1/1 (2.7 mL), three times with NMP (2.7 mL), three times with MeOH (2.7 mL), three times with DCM (2.7 mL), and three times with heptane (2.7 mL), and the obtained solid phase was dried under reduced pressure to obtain the mixtures described in Table 8-4-1-1.

[Table 337]

| [Table 8-4-1-1] Mixture used and obtained mixture in steps h02 to h21 | | | | | |
|---|---|---|---|---|---|
| Mixture used | Alcohol used | Amount of 1 M TBAOHaq. Used (mL) | Reaction temperature (°C) | Reaction time (h) | Obtained mixture |
| 2-1-b2C08-0 | tAmOH | 0.130 | 40 | 14 | 2-1-h02C08-0 |
| 2-1-b2C09-0 | tAmOH | 0.130 | 40 | 14 | 2-1-h03C09-0 |
| 2-1-b2C10-0 | tAmOH | 0.130 | rt | 7 | 2-1-h04C10-0 |
| 2-1-b2C11-0 | tAmOH | 0.130 | rt | 7 | 2-1-h05C11-0 |
| 2-1-b2C12-0 | tAmOH | 0.130 | rt | 7 | 2-1-h06C12-0 |
| 2-1-b2C13-0 | tAmOH | 0.130 | rt | 7 | 2-1-h07C13-0 |
| 2-1-b2C14-0 | tAmOH | 0.130 | 40 | 14 | 2-1-h08C14-0 |
| 2-1-b2C01-0 | MeOH | 0.259 | rt | 5 | 2-1-h09C01-0 |
| 2-1-b2C02-0 | MeOH | 0.259 | rt | 5 | 2-1-h10C02-0 |
| 2-1-b2C03-0 | MeOH | 0.259 | rt | 5 | 2-1-h11C03-0 |

(continued)

| [Table 8-4-1-1] Mixture used and obtained mixture in steps h02 to h21 | | | | | |
|---|---|---|---|---|---|
| Mixture used | Alcohol used | Amount of 1 M TBAOHaq. Used (mL) | Reaction temperature (°C) | Reaction time (h) | Obtained mixture |
| 2-1-b2C04-0 | MeOH | 0.259 | rt | 5 | 2-1-h12C04-0 |
| 2-1-b2C05-0 | MeOH | 0.259 | rt | 5 | 2-1-h13C05-0 |
| 2-1-b2C06-0 | MeOH | 0.259 | rt | 5 | 2-1-h14C06-0 |
| 2-1-b2C07-0 | tAmOH | 0.130 | rt | 7 | 2-1-h15C07-0 |
| 2-1-b3C06-0 | MeOH | 0.259 | rt | 5 | 2-1-h16C06-0 |
| 2-1-b3C08-0 | MeOH | 0.259 | rt | 5 | 2-1-h17C08-0 |
| 2-1-b3C15-0 | MeOH | 0.259 | rt | 5 | 2-1-h18C15-0 |
| 2-1-b3C16-0 | MeOH | 0.259 | rt | 5 | 2-1-h19C16-0 |
| 2-1-b3C17-0 | MeOH | 0.259 | rt | 5 | 2-1-h20C17-0 |
| 2-1-b3C18-0 | MeOH | 0.259 | rt | 5 | 2-1-h21C18-0 |

Mixture 2-1 -h02C08-0 to mixture h21C18-0 can be treated by cleavage in the same manner as in Example 8-1-1 to obtain mixture 2-1-h02C08-1 to mixture h21C18-l. The details of the mixtures are shown in Table 8-4-1-2 to Table 8-4-1-21.

A notation method in each table will be described. In Table 8-4-1-2 to Table 8-4-1- 21, each compound that may be contained in mixture 2-1-h02C08-1 to mixture h21C18-1-1 is indicated by ID, and core blocks and linkers in combination in the structures of mixture 2- 1-h02C08-1 to mixture h21C18-1 are indicated by symbols or chemical formulas. The correspondence between the symbols and specific structures are shown in Figure 4. In Table 8-4-1-2 to Table 8-4-1-21, "×" and "c" are represented by chemical formulas.

[Formula 622]

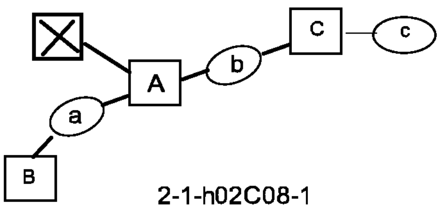


2-1-h02C08-1

For example, when ID is 2-1-h02C08-1-0001, the compound is represented by the following structural formula.

[Formula 623]

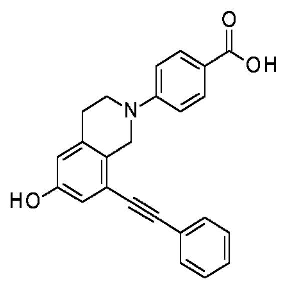

2-1-h02C08-1-0001

2-1-h02C08-1-0001

[Table 338]

| Notation in [Table 8-4-1-2] | | | | | | |
|---|---|---|---|---|---|---|
| ID | | a | B | b | C | c |
| 2-1-h02C08-1-0001 | OH | a2 | B11 | b1 | C08 | COOH |

[Table 339]

| [Table 8-4-1-2] Compound that may be contained in mixture 2-1-h02C08-1 | | | | | | | |
|---|---|---|---|---|---|---|---|
| ID | Exact Mass | | a | B | b | C | c |
| 2-1-h02C08-1-0001 | 369.136 | OH | a2 | B11 | b1 | C08 | COOH |
| 2-1-h02C08-1-0002 | 370.132 | OH | a2 | B12 | b1 | C08 | COOH |
| 2-1-h02C08-1-0003 | 383.152 | OH | a2 | B13 | b1 | C08 | COOH |
| 2-1-h02C08-1-0004 | 387.127 | OH | a2 | B14 | b1 | C08 | COOH |
| 2-1-h02C08-1-0005 | 388.142 | OH | a1 | B01 | b1 | C08 | COOH |
| 2-1-h02C08-1-0006 | 389.138 | OH | a1 | B02 | b1 | C08 | COOH |
| 2-1-h02C08-1-0007 | 397.168 | OH | a2 | B15 | b1 | C08 | COOH |
| 2-1-h02C08-1-0008 | 399.147 | OH | a2 | B16 | b1 | C08 | COOH |
| 2-1-h02C08-1-0009 | 402.158 | OH | a1 | B03 | b1 | C08 | COOH |
| 2-1-h02C08-1-0010 | 405.118 | OH | a2 | B17 | b1 | C08 | COOH |
| 2-1-h02C08-1-0011 | 406.133 | OH | a1 | B04 | b1 | C08 | COOH |
| 2-1-h02C08-1-0012 | 416.174 | OH | a1 | B05 | b1 | C08 | COOH |
| 2-1-h02C08-1-0013 | 418.153 | OH | a1 | B06 | b1 | C08 | COOH |
| 2-1-h02C08-1-0014 | 419.152 | OH | a2 | B18 | b1 | C08 | COOH |
| 2-1-h02C08-1-00 15 | 424.123 | OH | a1 | B07 | b1 | C08 | COOH |
| 2-1-h02C08-1-0016 | 437.124 | OH | a2 | B19 | b1 | C08 | COOH |
| 2-1-h02C08-1-0017 | 445.110 | OH | a1 | B08 | b1 | C08 | COOH |
| 2-1-h02C08-1-0018 | 453.119 | OH | a2 | B20 | b1 | C08 | COOH |
| 2-1-h02C08-1-00 19 | 456.130 | OH | a1 | B09 | b1 | C08 | COOH |
| 2-1-h02C08-1-0020 | 472.125 | OH | a1 | B10 | b1 | C08 | COOH |

[Table 340]

| [Table 8-4-1-3] Compound that may be contained in mixture 2-1-h03C09-1 | | | | | | | |
|---|---|---|---|---|---|---|---|
| ID | Exact Mass | | a | B | b | C | c |
| 2-1-h03C09-1-0001 | 437.124 | OH | a2 | B11 | b1 | C09 | COOH |
| 2-1-h03C09-1-0002 | 438.119 | OH | a2 | B12 | b1 | C09 | COOH |
| 2-1-h03C09-1-0003 | 451.140 | OH | a2 | B13 | b1 | C09 | COOH |
| 2-1-h03C09-1-0004 | 455.114 | OH | a2 | B14 | b1 | C09 | COOH |
| 2-1-h03C09-1-0005 | 456.130 | OH | a1 | B01 | b1 | C09 | COOH |
| 2-1-h03C09-1-0006 | 457.125 | OH | a1 | B02 | b1 | C09 | COOH |
| 2-1-h03C09-1-0007 | 465.155 | OH | a2 | B15 | b1 | C09 | COOH |

(continued)

| [Table 8-4-1-3] Compound that may be contained in mixture 2-1-h03C09-1 | | | | | | | |
|---|---|---|---|---|---|---|---|
| ID | Exact Mass | | a | B | b | C | c |
| 2-1-h03C09-1-0008 | 467.134 | OH | a2 | B16 | b1 | C09 | COOH |
| 2-1-h03C09-1-0009 | 470.145 | OH | a1 | B03 | b1 | C09 | COOH |
| 2-1-h03C09-1-0010 | 473.105 | OH | a2 | B17 | b1 | C09 | COOH |
| 2-1-h03C09-1-0011 | 474.120 | OH | a1 | B04 | b1 | C09 | COOH |
| 2-1-h03C09-1-0012 | 484.161 | OH | a1 | B05 | b1 | C09 | COOH |
| 2-1-h03C09-1-0013 | 486.140 | OH | a1 | B06 | b1 | C09 | COOH |
| 2-1-h03C09-1-0014 | 487.140 | OH | a2 | B18 | b1 | C09 | COOH |
| 2-1-h03 C09-1-0015 | 492.111 | OH | a1 | B07 | b1 | C09 | COOH |
| 2-1-h03C09-1-0016 | 505.111 | OH | a2 | B19 | b1 | C09 | COOH |
| 2-1-h03C09-1-0017 | 513.097 | OH | a1 | B08 | b1 | C09 | COOH |
| 2-1-h03C09-1-0018 | 521.106 | OH | a2 | B20 | b1 | C09 | COOH |
| 2-1-h03C09-1-0019 | 524.117 | OH | a1 | B09 | b1 | C09 | COOH |
| 2-1-h03C09-1-0020 | 540.112 | OH | a1 | B10 | b1 | C09 | COOH |

[Table 341]

| [Table 8-4-1-4] Compound that may be contained in mixture 2-1-h04C10-1 | | | | | | | |
|---|---|---|---|---|---|---|---|
| ID | Exact Mass | | a | B | b | C | c |
| 2-1-h04C 10-1-0001 | 371.127 | OH | a2 | B11 | b1 | C10 | COOH |
| 2-1-h04C10-1-0002 | 372.122 | OH | a2 | B12 | b1 | C10 | COOH |
| 2-1-h04C10-1-0003 | 385.143 | OH | a2 | B13 | b1 | C10 | COOH |
| 2-1-h04C10-1-0004 | 389.118 | OH | a2 | B14 | b1 | C10 | COOH |
| 2-1-h04C10-1-0005 | 390.133 | OH | a1 | B01 | b1 | C10 | COOH |
| 2-1-h04C 10-1-0006 | 391.128 | OH | a1 | B02 | b1 | C10 | COOH |
| 2-1-h04C10-1-0007 | 399.158 | OH | a2 | B15 | b1 | C10 | COOH |
| 2-1-h04C10-1-0008 | 401.138 | OH | a2 | B16 | b1 | C10 | COOH |
| 2-1-h04C10-1-0009 | 404.148 | OH | a1 | B03 | b1 | C10 | COOH |
| 2-1-h04C10-1-0010 | 407.108 | OH | a2 | B17 | b1 | C10 | COOH |
| 2-1-h04C10-1-0011 | 408.123 | OH | a1 | B04 | b1 | C10 | COOH |
| 2-1-h04C10-1-0012 | 418.164 | OH | a1 | B05 | b1 | C10 | COOH |
| 2-1-h04C10-1-0013 | 420.143 | OH | a1 | B06 | b1 | C10 | COOH |
| 2-1-h04C10-1-0014 | 421.143 | OH | a2 | B18 | b1 | C10 | COOH |
| 2-1-h04C10-1-0015 | 426.114 | OH | a1 | B07 | b1 | C10 | COOH |
| 2-1-h04C10-1-0016 | 439.114 | OH | a2 | B19 | b1 | C10 | COOH |
| 2-1-h04C10-1-0017 | 447.100 | OH | a1 | B08 | b1 | C10 | COOH |
| 2-1-h04C10-1-0018 | 455.109 | OH | a2 | B20 | b1 | C10 | COOH |
| 2-1-h04C10-1-0019 | 458.120 | OH | a1 | B09 | b1 | C10 | COOH |
| 2-1-h04C10-1-0020 | 474.115 | OH | a1 | B10 | b1 | C10 | COOH |

[Table 342]

| [Table 8-4-1-5] Compound that may be contained in mixture 2-1-h05C11-1 | | | | | | | |
|---|---|---|---|---|---|---|---|
| ID | Exact Mass | | a | B | b | C | c |
| 2-1-h05C11-1-0001 | 370.132 | OH | a2 | B11 | b1 | C11 | COOH |
| 2-1-h05C11-1-0002 | 371.127 | OH | a2 | B12 | b1 | C11 | COOH |
| 2-1-h05C11-1-0003 | 384.147 | OH | a2 | B13 | b1 | C11 | COOH |
| 2-1-h05C11-1-0004 | 388.122 | OH | a2 | B14 | b1 | C11 | COOH |
| 2-1-h05C11-1-0005 | 389.138 | OH | a1 | B01 | b1 | C11 | COOH |
| 2-1-h05C11-1-0006 | 390.133 | OH | a1 | B02 | b1 | C11 | COOH |
| 2-1-h05C11-1-0007 | 398.163 | OH | a2 | B15 | b1 | C11 | COOH |
| 2-1-h05C11-1-0008 | 400.142 | OH | a2 | B16 | b1 | C11 | COOH |
| 2-1-h05C11-1-0009 | 403.153 | OH | a1 | B03 | b1 | C11 | COOH |
| 2-1-h05C11-1-0010 | 406.113 | OH | a2 | B17 | b1 | C11 | COOH |
| 2-1-h05C11-1-0011 | 407.128 | OH | a1 | B04 | b1 | C11 | COOH |
| 2-1-h05C11-1-0012 | 417.169 | OH | a1 | B05 | b1 | C11 | COOH |
| 2-1-h05C11-1-0013 | 419.148 | OH | a1 | B06 | b1 | C11 | COOH |
| 2-1-h05C11-1-0014 | 420.147 | OH | a2 | B18 | b1 | C11 | COOH |
| 2-1-h05C11-1-0015 | 425.119 | OH | a1 | B07 | b1 | C11 | COOH |
| 2-1-h05C11-1-0016 | 438.119 | OH | a2 | B19 | b1 | C11 | COOH |
| 2-1-h05C11-1-0017 | 446.105 | OH | a1 | B08 | b1 | C11 | COOH |
| 2-1-h05C11-1-0018 | 454.114 | OH | a2 | B20 | b1 | C11 | COOH |
| 2-1-h05C11-1-0019 | 457.125 | OH | a1 | B09 | b1 | C11 | COOH |
| 2-1-h05C11-1-0020 | 473.120 | OH | a1 | B10 | b1 | C11 | COOH |

[Table 343]

| [Table 8-4-1-6] Compound that may be contained in mixture 2-1-h06C12-1 | | | | | | | |
|---|---|---|---|---|---|---|---|
| ID | Exact Mass | | a | B | b | C | c |
| 2-1-h06C12-1-0001 | 376.088 | OH | a2 | B11 | b1 | C12 | COOH |
| 2-1-h06C12-1-0002 | 377.083 | OH | a2 | B12 | b1 | C12 | COOH |
| 2-1-h06C12-1-0003 | 390.104 | OH | a2 | B13 | b1 | C12 | COOH |
| 2-1-h06C12-1-0004 | 394.079 | OH | a2 | B14 | b1 | C12 | COOH |
| 2-1-h06C12-1-0005 | 395.094 | OH | a1 | B01 | b1 | C12 | COOH |
| 2-1-h06C12-1-0006 | 396.089 | OH | a1 | B02 | b1 | C12 | COOH |
| 2-1-h06C12-1-0007 | 404.119 | OH | a2 | B15 | b1 | C12 | COOH |
| 2-1-h06C12-1-0008 | 406.099 | OH | a2 | B16 | b1 | C12 | COOH |
| 2-1-h06C12-1-0009 | 409.110 | OH | a1 | B03 | b1 | C12 | COOH |
| 2-1-h06C12-1-0010 | 412.069 | OH | a2 | B17 | b1 | C12 | COOH |
| 2-1-h06C12-1-0011 | 413.085 | OH | a1 | B04 | b1 | C12 | COOH |
| 2-1-h06C12-1-0012 | 423.125 | OH | a1 | B05 | b1 | C12 | COOH |

(continued)

| [Table 8-4-1-6] Compound that may be contained in mixture 2-1-h06C12-1 | | | | | | | |
|---|---|---|---|---|---|---|---|
| ID | Exact Mass | | a | B | b | C | c |
| 2-1-h06C12-1-0013 | 425.105 | OH | a1 | B06 | b1 | C12 | COOH |
| 2-1-h06C12-1-0014 | 426.104 | OH | a2 | B18 | b1 | C12 | COOH |
| 2-1-h06C12-1-0015 | 431.075 | OH | a1 | B07 | b1 | C12 | COOH |
| 2-1-h06C12-1-0016 | 444.076 | OH | a2 | B19 | b1 | C12 | COOH |
| 2-1-h06C12-1-0017 | 452.061 | OH | a1 | B08 | b1 | C12 | COOH |
| 2-1-h06C12-1-0018 | 460.070 | OH | a2 | B20 | b1 | C12 | COOH |
| 2-1-h06C12-1-0019 | 463.081 | OH | a1 | B09 | b1 | C12 | COOH |
| 2-1-h06C12-1-0020 | 479.076 | OH | a1 | B10 | b1 | C12 | COOH |

[Table 344]

| [Table 8-4-1-7] Compound that may be contained in mixture 2-1-h07C13-1 | | | | | | | |
|---|---|---|---|---|---|---|---|
| ID | Exact Mass | | a | B | b | C | c |
| 2-1-h07C13-1-0001 | 421.143 | OH | a2 | B11 | b1 | C13 | COOH |
| 2-1-h07C13-1-0002 | 422.138 | OH | a2 | B12 | b1 | C13 | COOH |
| 2-1-h07C13-1-0003 | 435.158 | OH | a2 | B13 | b1 | C13 | COOH |
| 2-1-h07C13-1-0004 | 439.133 | OH | a2 | B14 | b1 | C13 | COOH |
| 2-1-h07C13-1-0005 | 440.148 | OH | a1 | B01 | b1 | C13 | COOH |
| 2-1-h07C13-1-0006 | 441.144 | OH | a1 | B02 | b1 | C13 | COOH |
| 2-1-h07C13-1-0007 | 449.174 | OH | a2 | B15 | b1 | C13 | COOH |
| 2-1-h07C13-1-0008 | 451.153 | OH | a2 | B16 | b1 | C13 | COOH |
| 2-1-h07C13-1-0009 | 454.164 | OH | a1 | B03 | b1 | C13 | COOH |
| 2-1-h07C13-1-0010 | 457.124 | OH | a2 | B17 | b1 | C13 | COOH |
| 2-1-h07C13-1-0011 | 458.139 | OH | a1 | B04 | b1 | C13 | COOH |
| 2-1-h07C13-1-0012 | 468.180 | OH | a1 | B05 | b1 | C13 | COOH |
| 2-1-h07C13-1-0013 | 470.159 | OH | a1 | B06 | b1 | C13 | COOH |
| 2-1-h07C13-1-0014 | 471.158 | OH | a2 | B18 | b1 | C13 | COOH |
| 2-1-h07C13-1-0015 | 476.130 | OH | a1 | B07 | b1 | C13 | COOH |
| 2-1-h07C13-1-0016 | 489.130 | OH | a2 | B19 | b1 | C13 | COOH |
| 2-1-h07C13-1-0017 | 497.116 | OH | a1 | B08 | b1 | C13 | COOH |
| 2-1-h07C13-1-0018 | 505.125 | OH | a2 | B20 | b1 | C13 | COOH |
| 2-1-h07C13-1-0019 | 508.136 | OH | a1 | B09 | b1 | C13 | COOH |
| 2-1-h07C13-1-0020 | 524.131 | OH | a1 | B10 | b1 | C13 | COOH |

[Table 345]

| [Table 8-4-1-8] Compound that may be contained in mixture 2-1-h08C14-1 | | | | | | | |
|---|---|---|---|---|---|---|---|
| ID | Exact Mass | | a | B | b | C | c |
| 2-1-h08C14-1-0001 | 425.109 | OH | a2 | B11 | b1 | C14 | COOH |
| 2-1-h08C14-1-0002 | 426.104 | OH | a2 | B12 | b1 | C14 | COOH |
| 2-1-h08C14-1-0003 | 439.124 | OH | a2 | B13 | b1 | C14 | COOH |
| 2-1-h08C14-1-0004 | 443.099 | OH | a2 | B14 | b1 | C14 | COOH |
| 2-1-h08C14-1-0005 | 444.114 | OH | a1 | B01 | b1 | C14 | COOH |
| 2-1-h08C14-1-0006 | 445.110 | OH | a1 | B02 | b1 | C14 | COOH |
| 2-1-h08C14-1-0007 | 453.140 | OH | a2 | B15 | b1 | C14 | COOH |
| 2-1-h08C14-1-0008 | 455.119 | OH | a2 | B16 | b1 | C14 | COOH |
| 2-1-h08C14-1-0009 | 458.130 | OH | a1 | B03 | b1 | C14 | COOH |
| 2-1-h08C14-1-0010 | 461.090 | OH | a2 | B17 | b1 | C14 | COOH |
| 2-1-h08C14-1-0011 | 462.105 | OH | a1 | B04 | b1 | C14 | COOH |
| 2-1-h08C14-1-0012 | 472.146 | OH | a1 | B05 | b1 | C14 | COOH |
| 2-1-M8C14-1-0013 | 474.125 | OH | a1 | B06 | b1 | C14 | COOH |
| 2-1-h08C14-1-0014 | 475.124 | OH | a2 | B18 | b1 | C14 | COOH |
| 2-1-h08C14-1-0015 | 480.096 | OH | a1 | B07 | b1 | C14 | COOH |
| 2-1-h08C14-1-0016 | 493.096 | OH | a2 | B19 | b1 | C14 | COOH |
| 2-1-h08C14-1-0017 | 501.082 | OH | a1 | B08 | b1 | C14 | COOH |
| 2-1-h08C14-1-0018 | 509.091 | OH | a2 | B20 | b1 | C14 | COOH |
| 2-1-h08C14-1-0019 | 512.102 | OH | a1 | B09 | b1 | C14 | COOH |
| 2-1-h08C14-1-0020 | 528.097 | OH | a1 | B10 | b1 | C14 | COOH |

[Table 346]

| [Table 8-4-1-9] Compound that may be contained in mixture 2-1-h09C01-1 | | | | | | | |
|---|---|---|---|---|---|---|---|
| ID | Exact Mass | | a | B | b | C | c |
| 2-1-h09C01-1-0001 | 387.111 | OH | a2 | B11 | b2 | C01 | COOH |
| 2-1-h09C01-1-0002 | 388.106 | OH | a2 | B12 | b2 | C01 | COOH |
| 2-1-h09C01-1-0003 | 401.126 | OH | a2 | B13 | b2 | C01 | COOH |
| 2-1-h09C01-1-0004 | 405.101 | OH | a2 | B14 | b2 | C01 | COOH |
| 2-1-h09C01-1-0005 | 406.116 | OH | a1 | B01 | b2 | C01 | COOH |
| 2-1-h09C01-1-0006 | 407.112 | OH | a1 | B02 | b2 | C01 | COOH |
| 2-1-h09C01-1-0007 | 415.142 | OH | a2 | B15 | b2 | C01 | COOH |
| 2-1-h09C01-1-0008 | 417.121 | OH | a2 | B16 | b2 | C01 | COOH |
| 2-1-h09C01-1-0009 | 420.132 | OH | a1 | B03 | b2 | C01 | COOH |
| 2-1-h09C01-1-0010 | 423.092 | OH | a2 | B17 | b2 | C01 | COOH |
| 2-1-h09C01-1-0011 | 424.107 | OH | a1 | B04 | b2 | C01 | COOH |
| 2-1-h09C01-1-0012 | 434.148 | OH | a1 | B05 | b2 | C01 | COOH |
| 2-1-h09C01-1-0013 | 436.127 | OH | a1 | B06 | b2 | C01 | COOH |

(continued)

| [Table 8-4-1-9] Compound that may be contained in mixture 2-1-h09C01-1 | | | | | | | |
|---|---|---|---|---|---|---|---|
| ID | Exact Mass | | a | B | b | C | c |
| 2-1-h09C01-1-0014 | 437.126 | OH | a2 | B18 | b2 | C01 | COOH |
| 2-1-h09C01-1-0015 | 442.098 | OH | a1 | B07 | b2 | C01 | COOH |
| 2-1-h09C01-1-0016 | 455.098 | OH | a2 | B19 | b2 | C01 | COOH |
| 2-1-h09C01-1-0017 | 463.084 | OH | a1 | B08 | b2 | C01 | COOH |
| 2-1-h09C01-1-0018 | 471.093 | OH | a2 | B20 | b2 | C01 | COOH |
| 2-1-h09C01-1-0019 | 474.104 | OH | a1 | B09 | b2 | C01 | COOH |
| 2-1-h09C01-1-0020 | 490.099 | OH | a1 | B10 | b2 | C01 | COOH |

[Table 347]

| [Table 8-4-1-10] Compound that may be contained in mixture 2-1-h10C02-1 | | | | | | | |
|---|---|---|---|---|---|---|---|
| ID | Exact Mass | | a | B | b | C | c |
| 2-1-h10C02-1-0001 | 397.131 | OH | a2 | B11 | b2 | C02 | COOH |
| 2-1-h10C02-1-0002 | 398.127 | OH | a2 | B12 | b2 | C02 | COOH |
| 2-1-h10C02-1-0003 | 411.147 | OH | a2 | B13 | b2 | C02 | COOH |
| 2-1-h10C02-1-0004 | 415.122 | OH | a2 | B14 | b2 | C02 | COOH |
| 2-1-h10C02-1-0005 | 416.137 | OH | a1 | B01 | b2 | C02 | COOH |
| 2-1-h10C02-1-0006 | 417.132 | OH | a1 | B02 | b2 | C02 | COOH |
| 2-1-h10C02-1-0007 | 425.163 | OH | a2 | B15 | b2 | C02 | COOH |
| 2-1-h10C02-1-0008 | 427.142 | OH | a2 | B16 | b2 | C02 | COOH |
| 2-1-h10C02-1-0009 | 430.153 | OH | a1 | B03 | b2 | C02 | COOH |
| 2-1-h10C02-1-0010 | 433.113 | OH | a2 | B17 | b2 | C02 | COOH |
| 2-1-h10C02-1-0011 | 434.128 | OH | a1 | B04 | b2 | C02 | COOH |
| 2-1-h10C02-1-0012 | 444.169 | OH | a1 | B05 | b2 | C02 | COOH |
| 2-1-h10C02-1-0013 | 446.148 | OH | a1 | B06 | b2 | C02 | COOH |
| 2-1-h10C02-1-0014 | 447.147 | OH | a2 | B18 | b2 | C02 | COOH |
| 2-1-h10C02-1-0015 | 452.118 | OH | a1 | B07 | b2 | C02 | COOH |
| 2-1-h10C02-1-0016 | 465.119 | OH | a2 | B19 | b2 | C02 | COOH |
| 2-1-h10C02-1-0017 | 473.105 | OH | a1 | B08 | b2 | C02 | COOH |
| 2-1-h10C02-1-0018 | 481.114 | OH | a2 | B20 | b2 | C02 | COOH |
| 2-1-h10C02-1-0019 | 484.125 | OH | a1 | B09 | b2 | C02 | COOH |
| 2-1-h10C02-1-0020 | 500.120 | OH | a1 | B10 | b2 | C02 | COOH |

[Table 348]

| [Table 8-4-1-11] Compound that may be contained in mixture 2-1-hl 1C03-1 | | | | | | | |
|---|---|---|---|---|---|---|---|
| ID | Exact Mass | | a | B | b | C | c |
| 2-1-h11C03-1-0001 | 398.127 | OH | a2 | B11 | b2 | C03 | COOH |

(continued)

| [Table 8-4-1-11] Compound that may be contained in mixture 2-1-hl 1C03-1 | | | | | | | |
|---|---|---|---|---|---|---|---|
| ID | Exact Mass | | a | B | b | C | c |
| 2-1-h11C03-1-0002 | 399.122 | OH | a2 | B12 | b2 | C03 | COOH |
| 2-1-h11C03-1-0003 | 412.142 | OH | a2 | B13 | b2 | C03 | COOH |
| 2-1-h11C03-1-0004 | 416.117 | OH | a2 | B14 | b2 | C03 | COOH |
| 2-1-h11C03-1-0005 | 417.132 | OH | a1 | B01 | b2 | C03 | COOH |
| 2-1-h11C03-1-0006 | 418.128 | OH | a1 | B02 | b2 | C03 | COOH |
| 2-1-h11C03-1-0007 | 426.158 | OH | a2 | B15 | b2 | C03 | COOH |
| 2-1-h11C03-1-0008 | 428.137 | OH | a2 | B16 | b2 | C03 | COOH |
| 2-1-h11C03-1-0009 | 431.148 | OH | a1 | B03 | b2 | C03 | COOH |
| 2-1-h11C03-1-0010 | 434.108 | OH | a2 | B17 | b2 | C03 | COOH |
| 2-1-h11C03-1-0011 | 435.123 | OH | a1 | B04 | b2 | C03 | COOH |
| 2-1-h11C03-1-0012 | 445.164 | OH | a1 | B05 | b2 | C03 | COOH |
| 2-1-h11C03-1-0013 | 447.143 | OH | a1 | B06 | b2 | C03 | COOH |
| 2-1-h11C03-1-0014 | 448.142 | OH | a2 | B18 | b2 | C03 | COOH |
| 2-1-h11C03-1-0015 | 453.114 | OH | a1 | B07 | b2 | C03 | COOH |
| 2-1-h11C03-1-0016 | 466.114 | OH | a2 | B19 | b2 | C03 | COOH |
| 2-1-h11C03-1-0017 | 474.100 | OH | a1 | B08 | b2 | C03 | COOH |
| 2-1-h11C03-1-0018 | 482.109 | OH | a2 | B20 | b2 | C03 | COOH |
| 2-1-h11C03-1-0019 | 485.120 | OH | a1 | B09 | b2 | C03 | COOH |
| 2-1-hll C03-1-0020 | 501.115 | OH | a1 | B10 | b2 | C03 | COOH |

[Table 349]

| [Table 8-4-1-12] Compound that may be contained in mixture 2-1-h12C04-1 | | | | | | | |
|---|---|---|---|---|---|---|---|
| ID | Exact Mass | | a | B | b | C | c |
| 2-1-h12C04-1-0001 | 415.122 | OH | a2 | B11 | b2 | C04 | COOH |
| 2-1-h12C04-1-0002 | 416.117 | OH | a2 | B12 | b2 | C04 | COOH |
| 2-1-h12C04-1-0003 | 429.138 | OH | a2 | B13 | b2 | C04 | COOH |
| 2-1-h12C04-1-0004 | 433.113 | OH | a2 | B14 | b2 | C04 | COOH |
| 2-1-h12C04-1-0005 | 434.128 | OH | a1 | B01 | b2 | C04 | COOH |
| 2-1-h12C04-1-0006 | 435.123 | OH | a1 | B02 | b2 | C04 | COOH |
| 2-1-h12C04-1-0007 | 443.153 | OH | a2 | B15 | b2 | C04 | COOH |
| 2-1-h12C04-1-0008 | 445.133 | OH | a2 | B16 | b2 | C04 | COOH |
| 2-1-h12C04-1-0009 | 448.143 | OH | a1 | B03 | b2 | C04 | COOH |
| 2-1-h12C04-1-0010 | 451.103 | OH | a2 | B17 | b2 | C04 | COOH |
| 2-1-h12C04-1-0011 | 452.118 | OH | a1 | B04 | b2 | C04 | COOH |
| 2-1-h12C04-1-0012 | 462.159 | OH | a1 | B05 | b2 | C04 | COOH |
| 2-1-h12C04-1-0013 | 464.138 | OH | a1 | B06 | b2 | C04 | COOH |
| 2-1-h12C04-1-0014 | 465.138 | OH | a2 | B18 | b2 | C04 | COOH |

(continued)

| [Table 8-4-1-12] Compound that may be contained in mixture 2-1-h12C04-1 | | | | | | | |
|---|---|---|---|---|---|---|---|
| ID | Exact Mass | | a | B | b | C | c |
| 2-1-h12C04-1-0015 | 470.109 | OH | a1 | B07 | b2 | C04 | COOH |
| 2-1-h12C04-1-0016 | 483.109 | OH | a2 | B19 | b2 | C04 | COOH |
| 2-1-h12C04-1-0017 | 491.095 | OH | a1 | B08 | b2 | C04 | COOH |
| 2-1-h12C04-1-0018 | 499.104 | OH | a2 | B20 | b2 | C04 | COOH |
| 2-1-h12C04-1-0019 | 502.115 | OH | a1 | B09 | b2 | C04 | COOH |
| 2-1-h12C04-1-0020 | 518.110 | OH | a1 | B10 | b2 | C04 | COOH |

[Table 350]

| [Table 8-4-1-13] Compound that may be contained in mixture 2-1-h13C05-1 | | | | | | | |
|---|---|---|---|---|---|---|---|
| ID | Exact Mass | | a | B | b | C | c |
| 2-1-h13C05-1-0001 | 412.142 | OH | a2 | B11 | b2 | C05 | COOH |
| 2-1-h13C05-1-0002 | 413.138 | OH | a2 | B12 | b2 | C05 | COOH |
| 2-1-h13C05-1-0003 | 426.158 | OH | a2 | B13 | b2 | C05 | COOH |
| 2-1-h13C05-1-0004 | 430.133 | OH | a2 | B14 | b2 | C05 | COOH |
| 2-1-h13C05-1-0005 | 431.148 | OH | a1 | B01 | b2 | C05 | COOH |
| 2-1-h13C05-1-0006 | 432.143 | OH | a1 | B02 | b2 | C05 | COOH |
| 2-1-h13C05-1-0007 | 440.174 | OH | a2 | B15 | b2 | C05 | COOH |
| 2-1-h13 COS-1-0008 | 442.153 | OH | a2 | B16 | b2 | C05 | COOH |
| 2-1-h13 COS-1-0009 | 445.164 | OH | a1 | B03 | b2 | C05 | COOH |
| 2-1-h13 COS-1-0010 | 448.123 | OH | a2 | B17 | b2 | C05 | COOH |
| 2-1-h13 COS-1-0011 | 449.139 | OH | a1 | B04 | b2 | C05 | COOH |
| 2-1-h13 COS-1-0012 | 459.179 | OH | a1 | B05 | b2 | C05 | COOH |
| 2-1-h13 COS-1-0013 | 461.159 | OH | a1 | B06 | b2 | C05 | COOH |
| 2-1-h13C05-1-0014 | 462.158 | OH | a2 | B18 | b2 | C05 | COOH |
| 2-1-h13 COS-1-0015 | 467.129 | OH | a1 | B07 | b2 | C05 | COOH |
| 2-1-h13C05-1-0016 | 480.130 | OH | a2 | B19 | b2 | C05 | COOH |
| 2-1-h13C05-1-0017 | 488.115 | OH | a1 | B08 | b2 | C05 | COOH |
| 2-1-h13C05-1-0018 | 496.125 | OH | a2 | B20 | b2 | C05 | COOH |
| 2-1-h13C05-1-0019 | 499.136 | OH | a1 | B09 | b2 | C05 | COOH |
| 2-1-h13C05-1-0020 | 515.130 | OH | a1 | B10 | b2 | C05 | COOH |

[Table 351]

| [Table 8-4-1-14] Compound that may be contained in mixture 2-1-h14C06-1 | | | | | | | |
|---|---|---|---|---|---|---|---|
| ID | Exact Mass | | a | B | b | C | c |
| 2-1-h14C06-1-0001 | 403.088 | OH | a2 | B11 | b2 | C06 | COOH |
| 2-1-h14C06-1-0002 | 404.083 | OH | a2 | B12 | b2 | C06 | COOH |

(continued)

| [Table 8-4-1-14] Compound that may be contained in mixture 2-1-h14C06-1 | | | | | | | |
|---|---|---|---|---|---|---|---|
| ID | Exact Mass | | a | B | b | C | c |
| 2-1-h14C06-1-0003 | 417.103 | OH | a2 | B13 | b2 | C06 | COOH |
| 2-1-h14C06-1-0004 | 421.078 | OH | a2 | B14 | b2 | C06 | COOH |
| 2-1-h14C06-1-0005 | 422.094 | OH | a1 | B01 | b2 | C06 | COOH |
| 2-1-h14C06-1-0006 | 423.089 | OH | a1 | B02 | b2 | C06 | COOH |
| 2-1-h14C06-1-0007 | 431.119 | OH | a2 | B15 | b2 | C06 | COOH |
| 2-1-h14C06-1-0008 | 433.098 | OH | a2 | B16 | b2 | C06 | COOH |
| 2-1-h14C06-1-0009 | 436.109 | OH | a1 | B03 | b2 | C06 | COOH |
| 2-1-h14C06-1-0010 | 439.069 | OH | a2 | B17 | b2 | C06 | COOH |
| 2-1-h14C06-1-0011 | 440.084 | OH | a1 | B04 | b2 | C06 | COOH |
| 2-1-h14C06-1-0012 | 450.125 | OH | a1 | B05 | b2 | C06 | COOH |
| 2-1-h14C06-1-0013 | 452.104 | OH | a1 | B06 | b2 | C06 | COOH |
| 2-1-h14C06-1-0014 | 453.103 | OH | a2 | B18 | b2 | C06 | COOH |
| 2-1-h14C06-1-0015 | 458.075 | OH | a1 | B07 | b2 | C06 | COOH |
| 2-1-h14C06-1-0016 | 471.075 | OH | a2 | B19 | b2 | C06 | COOH |
| 2-1-h14C06-1-0017 | 479.061 | OH | a1 | B08 | b2 | C06 | COOH |
| 2-1-h14C06-1-0018 | 487.070 | OH | a2 | B20 | b2 | C06 | COOH |
| 2-1-h14C06-1-0019 | 490.081 | OH | a1 | B09 | b2 | C06 | COOH |
| 2-1-h14C06-1-0020 | 506.076 | OH | a1 | B10 | b2 | C06 | COOH |

[Table 352]

| [Table 8-4-1-15] Compound that may be contained in mixture 2-1-h15C07-1 | | | | | | | |
|---|---|---|---|---|---|---|---|
| ID | Exact Mass | | a | B | b | C | c |
| 2-1-h15 C07-1-0001 | 429.194 | OH | a2 | B11 | b2 | C07 | COOH |
| 2-1-h15 C07-1-0002 | 430.189 | OH | a2 | B12 | b2 | C07 | COOH |
| 2-1-h15C07-1-0003 | 443.210 | OH | a2 | B13 | b2 | C07 | COOH |
| 2-1-h15C07-1-0004 | 447.185 | OH | a2 | B14 | b2 | C07 | COOH |
| 2-1-h15C07-1-0005 | 448.200 | OH | a1 | B01 | b2 | C07 | COOH |
| 2-1-h15C07-1-0006 | 449.195 | OH | a1 | B02 | b2 | C07 | COOH |
| 2-1-h15C07-1-0007 | 457.225 | OH | a2 | B15 | b2 | C07 | COOH |
| 2-1-h15C07-1-0008 | 459.205 | OH | a2 | B16 | b2 | C07 | COOH |
| 2-1-h15C07-1-0009 | 462.215 | OH | a1 | B03 | b2 | C07 | COOH |
| 2-1-h15C07-1-0010 | 465.175 | OH | a2 | B17 | b2 | C07 | COOH |
| 2-1-h15C07-1-0011 | 466.190 | OH | a1 | B04 | b2 | C07 | COOH |
| 2-1-h15C07-1-0012 | 476.231 | OH | a1 | B05 | b2 | C07 | COOH |
| 2-1-h15C07-1-0013 | 478.210 | OH | a1 | B06 | b2 | C07 | COOH |
| 2-1-h15 C07-1-0014 | 479.210 | OH | a2 | B18 | b2 | C07 | COOH |

(continued)

| [Table 8-4-1-15] Compound that may be contained in mixture 2-1-h15C07-1 | | | | | | | |
|---|---|---|---|---|---|---|---|
| ID | Exact Mass | | a | B | b | C | c |
| 2-1-h15C07-1-0015 | 484.181 | OH | a1 | B07 | b2 | C07 | COOH |
| 2-1-h15 C07-1-0016 | 497.181 | OH | a2 | B19 | b2 | C07 | COOH |
| 2-1-h15 C07-1-0017 | 505.167 | OH | a1 | B08 | b2 | C07 | COOH |
| 2-1-h15 C07-1-0018 | 513.176 | OH | a2 | B20 | b2 | C07 | COOH |
| 2-1-h15 C07-1-0019 | 516.187 | OH | a1 | B09 | b2 | C07 | COOH |
| 2-1-h15 C07-1-0020 | 532.182 | OH | a1 | B10 | b2 | C07 | COOH |

[Table 353]

| [Table 8-4-1-16] Compound that may be contained in mixture 2-1-h16C06-1 | | | | | | | |
|---|---|---|---|---|---|---|---|
| ID | Exact Mass | | a | B | b | C | c |
| 2-1-h16C06-1-0001 | 439.055 | OH | a2 | B11 | b3 | C06 | COOH |
| 2-1-h16C06-1-0002 | 440.050 | OH | a2 | B12 | b3 | C06 | COOH |
| 2-1-h16C06-1-0003 | 453.070 | OH | a2 | B13 | b3 | C06 | COOH |
| 2-1-h16C06-1-0004 | 457.045 | OH | a2 | B14 | b3 | C06 | COOH |
| 2-1-h16C06-1-0005 | 458.061 | OH | a1 | B01 | b3 | C06 | COOH |
| 2-1-h16C06-1-0006 | 459.056 | OH | a1 | B02 | b3 | C06 | COOH |
| 2-1-h16C06-1-0007 | 467.086 | OH | a2 | B15 | b3 | C06 | COOH |
| 2-1-h16C06-1-0008 | 469.065 | OH | a2 | B16 | b3 | C06 | COOH |
| 2-1-h16C06-1-0009 | 472.076 | OH | a1 | B03 | b3 | C06 | COOH |
| 2-1-h16C06-1-0010 | 475.036 | OH | a2 | B17 | b3 | C06 | COOH |
| 2-1-h16C06-1-0011 | 476.051 | OH | a1 | B04 | b3 | C06 | COOH |
| 2-1-h16C06-1-0012 | 486.092 | OH | a1 | B05 | b3 | C06 | COOH |
| 2-1-h16C06-1-0013 | 488.071 | OH | a1 | B06 | b3 | C06 | COOH |
| 2-1-h16C06-1-0014 | 489.070 | OH | a2 | B18 | b3 | C06 | COOH |
| 2-1-h16C06-1-0015 | 494.042 | OH | a1 | B07 | b3 | C06 | COOH |
| 2-1-h16C06-1-0016 | 507.042 | OH | a2 | B19 | b3 | C06 | COOH |
| 2-1-h16C06-1-0017 | 515.028 | OH | a1 | B08 | b3 | C06 | COOH |
| 2-1-h16C06-1-0018 | 523.037 | OH | a2 | B20 | b3 | C06 | COOH |
| 2-1-h16C06-1-0019 | 526.048 | OH | a1 | B09 | b3 | C06 | COOH |
| 2-1-h16C06-1-0020 | 542.043 | OH | a1 | B10 | b3 | C06 | COOH |

[Table 354]

| [Table 8-4-1-17] Compound that may be contained in mixture 2-1-h17C08-1 | | | | | | | |
|---|---|---|---|---|---|---|---|
| ID | Exact Mass | | a | B | b | C | c |
| 2-1-h17C08-1-0001 | 433.098 | OH | a2 | B11 | b3 | C08 | COOH |
| 2-1-h17C08-1-0002 | 434.094 | OH | a2 | B12 | b3 | C08 | COOH |

(continued)

| [Table 8-4-1-17] Compound that may be contained in mixture 2-1-h17C08-1 | | | | | | | |
|---|---|---|---|---|---|---|---|
| ID | Exact Mass | | a | B | b | C | c |
| 2-1-h17C08-1-0003 | 447.114 | OH | a2 | B13 | b3 | C08 | COOH |
| 2-1-h17C08-1-0004 | 451.089 | OH | a2 | B14 | b3 | C08 | COOH |
| 2-1-h17C08-1-0005 | 452.104 | OH | a1 | B01 | b3 | C08 | COOH |
| 2-1-h17C08-1-0006 | 453.099 | OH | a1 | B02 | b3 | C08 | COOH |
| 2-1-h17C08-1-0007 | 461.130 | OH | a2 | B15 | b3 | C08 | COOH |
| 2-1-h17C08-1-0008 | 463.109 | OH | a2 | B16 | b3 | C08 | COOH |
| 2-1-h17C08-1-0009 | 466.120 | OH | a1 | B03 | b3 | C08 | COOH |
| 2-1-h17C08-1-0010 | 469.080 | OH | a2 | B17 | b3 | C08 | COOH |
| 2-1-h17C08-1-0011 | 470.095 | OH | a1 | B04 | b3 | C08 | COOH |
| 2-1-h17C08-1-0012 | 480.136 | OH | a1 | B05 | b3 | C08 | COOH |
| 2-1-h17C08-1-0013 | 482.115 | OH | a1 | B06 | b3 | C08 | COOH |
| 2-1-h17C08-1-0014 | 483.114 | OH | a2 | B18 | b3 | C08 | COOH |
| 2-1-h17C08-1-0015 | 488.085 | OH | a1 | B07 | b3 | C08 | COOH |
| 2-1-h17C08-1-0016 | 501.086 | OH | a2 | B19 | b3 | C08 | COOH |
| 2-1-h17C08-1-0017 | 509.072 | OH | a1 | B08 | b3 | C08 | COOH |
| 2-1-h17C08-1-0018 | 517.081 | OH | a2 | B20 | b3 | C08 | COOH |
| 2-1-h17C08-1-0019 | 520.092 | OH | a1 | B09 | b3 | C08 | COOH |
| 2-1-h17C08-1-0020 | 536.087 | OH | a1 | B10 | b3 | C08 | COOH |

[Table 355]

| [Table 8-4-1-18] Compound that may be contained in mixture 2-1-h18C15-1 | | | | | | | |
|---|---|---|---|---|---|---|---|
| ID | Exact Mass | | a | B | b | C | c |
| 2-1-h18C15-1-0001 | 423.078 | OH | a2 | B11 | b3 | C15 | COOH |
| 2-1-h18C15-1-0002 | 424.073 | OH | a2 | B12 | b3 | C15 | COOH |
| 2-1-h18C15-1-0003 | 437.093 | OH | a2 | B13 | b3 | C15 | COOH |
| 2-1-h18C15-1-0004 | 441.068 | OH | a2 | B14 | b3 | C15 | COOH |
| 2-1-h18C15-1-0005 | 442.083 | OH | a1 | B01 | b3 | C15 | COOH |
| 2-1-h18C15-1-0006 | 443.079 | OH | a1 | B02 | b3 | C15 | COOH |
| 2-1-h18C15-1-0007 | 451.109 | OH | a2 | B15 | b3 | C15 | COOH |
| 2-1-h18C15-1-0008 | 453.088 | OH | a2 | B16 | b3 | C15 | COOH |
| 2-1-h18C15-1-0009 | 456.099 | OH | a1 | B03 | b3 | C15 | COOH |
| 2-1-h18C15-1-0010 | 459.059 | OH | a2 | B17 | b3 | C15 | COOH |
| 2-1-h18C15-1-0011 | 460.074 | OH | a1 | B04 | b3 | C15 | COOH |
| 2-1-h18C15-1-0012 | 470.115 | OH | a1 | B05 | b3 | C15 | COOH |
| 2-1-h18C15-1-0013 | 472.094 | OH | a1 | B06 | b3 | C15 | COOH |
| 2-1-h18C15-1-0014 | 473.093 | OH | a2 | B18 | b3 | C15 | COOH |
| 2-1-h18C15-1-0015 | 478.065 | OH | a1 | B07 | b3 | C15 | COOH |
| 2-1-h18C15-1-0016 | 491.065 | OH | a2 | B19 | b3 | C15 | COOH |
| 2-1-h18C15-1-0017 | 499.051 | OH | a1 | B08 | b3 | C15 | COOH |
| 2-1-h18C15-1-0018 | 507.060 | OH | a2 | B20 | b3 | C15 | COOH |
| 2-1-h18 C15-1-0019 | 510.071 | OH | a1 | B09 | b3 | C15 | COOH |
| 2-1-h18C15-1-0020 | 526.066 | OH | a1 | B10 | b3 | C15 | COOH |

[Table 356]

| [Table 8-4-1-19] Compound that may be contained in mixture 2-1-h19C16-1 | | | | | | | |
|---|---|---|---|---|---|---|---|
| ID | Exact Mass | | a | B | b | C | c |
| 2-1-h19C16-1-0001 | 487.109 | OH | a2 | B11 | b3 | C16 | COOH |
| 2-1-h19C16-1-0002 | 488.104 | OH | a2 | B12 | b3 | C16 | COOH |
| 2-1-h19C16-1-0003 | 501.125 | OH | a2 | B13 | b3 | C16 | COOH |
| 2-1-h19C16-1-0004 | 505.100 | OH | a2 | B14 | b3 | C16 | COOH |
| 2-1-h19C16-1-0005 | 506.115 | OH | a1 | B01 | b3 | C16 | COOH |
| 2-1-h19C16-1-0006 | 507.110 | OH | a1 | B02 | b3 | C16 | COOH |
| 2-1-h19C16-1-0007 | 515.140 | OH | a2 | B15 | b3 | C16 | COOH |
| 2-1-h19C16-1-0008 | 517.120 | OH | a2 | B16 | b3 | C16 | COOH |
| 2-1-h19C16-1-0009 | 520.130 | OH | a1 | B03 | b3 | C16 | COOH |
| 2-1-h19C16-1-0010 | 523.090 | OH | a2 | B17 | b3 | C16 | COOH |
| 2-1-h19C16-1-0011 | 524.105 | OH | a1 | B04 | b3 | C16 | COOH |
| 2-1-h19C16-1-0012 | 534.146 | OH | a1 | B05 | b3 | C16 | COOH |
| 2-1-h19C16-1-0013 | 536.125 | OH | a1 | B06 | b3 | C16 | COOH |
| 2-1-h19C16-1-0014 | 537.125 | OH | a2 | B18 | b3 | C16 | COOH |

(continued)

| [Table 8-4-1-19] Compound that may be contained in mixture 2-1-h19C16-1 | | | | | | | |
|---|---|---|---|---|---|---|---|
| ID | Exact Mass | | a | B | b | C | c |
| 2-1-h19C16-1-0015 | 542.096 | OH | a1 | B07 | b3 | C16 | COOH |
| 2-1-h19C16-1-0016 | 555.096 | OH | a2 | B19 | b3 | C16 | COOH |
| 2-1-h19C16-1-0017 | 563.082 | OH | a1 | B08 | b3 | C16 | COOH |
| 2-1-h19C16-1-0018 | 571.091 | OH | a2 | B20 | b3 | C16 | COOH |
| 2-1-h19C16-1-0019 | 574.102 | OH | a1 | B09 | b3 | C16 | COOH |
| 2-1-h19C16-1-0020 | 590.097 | OH | a1 | B10 | b3 | C16 | COOH |

[Table 357]

| [Table 8-4-1-20] Compound that may be contained in mixture 2-1-h20C17-1 | | | | | | | |
|---|---|---|---|---|---|---|---|
| ID | Exact Mass | | a | B | b | C | c |
| 2-1-h20C17-1-0001 | 489.070 | OH | a2 | B11 | b3 | C17 | COOH |
| 2-1-h20C17-1-0002 | 490.066 | OH | a2 | B12 | b3 | C17 | COOH |
| 2-1-h20C17-1-0003 | 503.086 | OH | a2 | B13 | b3 | C17 | COOH |
| 2-1-h20C17-1-0004 | 507.061 | OH | a2 | B14 | b3 | C17 | COOH |
| 2-1-h20C17-1-0005 | 508.076 | OH | a1 | B01 | b3 | C17 | COOH |
| 2-1-h20C17-1-0006 | 509.072 | OH | a1 | B02 | b3 | C17 | COOH |
| 2-1-h20C17-1-0007 | 517.102 | OH | a2 | B15 | b3 | C17 | COOH |
| 2-1-h20C17-1-0008 | 519.081 | OH | a2 | B16 | b3 | C17 | COOH |
| 2-1-h20C17-1-0009 | 522.092 | OH | a1 | B03 | b3 | C17 | COOH |
| 2-1-h20C17-1-0010 | 525.052 | OH | a2 | B17 | b3 | C17 | COOH |
| 2-1-h20C17-1-0011 | 526.067 | OH | a1 | B04 | b3 | C17 | COOH |
| 2-1-h20C17-1-0012 | 536.108 | OH | a1 | B05 | b3 | C17 | COOH |
| 2-1-h20C17-1-0013 | 538.087 | OH | a1 | B06 | b3 | C17 | COOH |
| 2-1-h20C17-1-0014 | 539.086 | OH | a2 | B18 | b3 | C17 | COOH |
| 2-1-h20C17-1-0015 | 544.057 | OH | a1 | B07 | b3 | C17 | COOH |
| 2-1-h20C17-1-0016 | 557.058 | OH | a2 | B19 | b3 | C17 | COOH |
| 2-1-h20C17-1-0017 | 565.044 | OH | a1 | B08 | b3 | C17 | COOH |
| 2-1-h20C 17-1-0018 | 573.053 | OH | a2 | B20 | b3 | C17 | COOH |
| 2-1-h20C17-1-0019 | 576.064 | OH | a1 | B09 | b3 | C17 | COOH |
| 2-1-h20C17-1-0020 | 592.059 | OH | a1 | B10 | b3 | C17 | COOH |

[Table 358]

| [Table 8-4-1-21] Compound that may be contained in mixture 2-1-h21C18-1 | | | | | | | |
|---|---|---|---|---|---|---|---|
| ID | Exact Mass | | a | B | b | C | c |
| 2-1-h21C18-1-0001 | 504.081 | OH | a2 | B11 | b3 | C18 | COOH |
| 2-1-h21C18-1-0002 | 505.077 | OH | a2 | B12 | b3 | C18 | COOH |
| 2-1-h21C18-1-0003 | 518.097 | OH | a2 | B13 | b3 | C18 | COOH |
| 2-1-h21C18-1-0004 | 522.072 | OH | a2 | B14 | b3 | C18 | COOH |
| 2-1-h21C18-1-0005 | 523.087 | OH | a1 | B01 | b3 | C18 | COOH |
| 2-1-h21C18-1-0006 | 524.082 | OH | a1 | B02 | b3 | C18 | COOH |
| 2-1-h21C18-1-0007 | 532.113 | OH | a2 | B15 | b3 | C18 | COOH |
| 2-1-h21C18-1-0008 | 534.092 | OH | a2 | B16 | b3 | C18 | COOH |
| 2-1-h21C18-1-0009 | 537.103 | OH | a1 | B03 | b3 | C18 | COOH |
| 2-1-h21C18-1-0010 | 540.063 | OH | a2 | B17 | b3 | C18 | COOH |
| 2-1-h21C18-1-0011 | 541.078 | OH | a1 | B04 | b3 | C18 | COOH |
| 2-1-h21C18-1-0012 | 551.118 | OH | a1 | B05 | b3 | C18 | COOH |
| 2-1-h21C18-1-0013 | 553.098 | OH | a1 | B06 | b3 | C18 | COOH |
| 2-1-h21C18-1-0014 | 554.097 | OH | a2 | B18 | b3 | C18 | COOH |
| 2-1-h21C18-1-0015 | 559.068 | OH | a1 | B07 | b3 | C18 | COOH |
| 2-1-h21C18-1-0016 | 572.069 | OH | a2 | B19 | b3 | C18 | COOH |
| 2-1-h21C18-1-0017 | 580.054 | OH | a1 | B08 | b3 | C18 | COOH |
| 2-1-h21C18-1-0018 | 588.064 | OH | a2 | B20 | b3 | C18 | COOH |
| 2-1-h21C18-1-0019 | 591.075 | OH | a1 | B09 | b3 | C18 | COOH |
| 2-1-h21C18-1-0020 | 607.069 | OH | a1 | B10 | b3 | C18 | COOH |

Example 8-4-2: Boc deprotection by steps d21 to d30

A reaction formula in step d21 performed using mixture 2-1-b1C29-0 will be illustrated below.

[Formula 624]

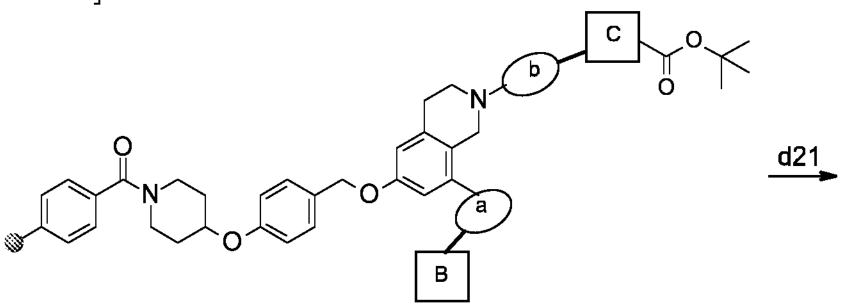


2-1-b1C29-0

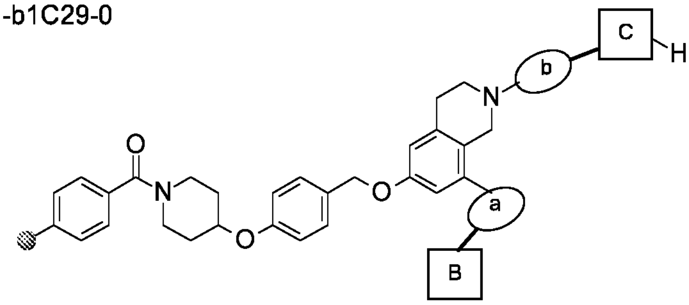

2-1-d21C29-0

Mixture 2-1-d21C29-0 to mixture 2-1-d25C33-0 and mixture 2-1-d26C19-0 to mixture 2-1-d30C27-0 were synthesized as follows using the mixtures described in Table 8- 4-2-1.

Separate mixtures (70 mg) described in Table 8-4-2-1, together with 2- methyltetrahydrofuran (1.4 mL), were added to ten 4 mL glass vials, respectively, and shaken at room temperature for 1 hour. DTBP (88 μL, 0.40 mmol) and $Tm(OTf)_3$ (165 mg, 0.267 mmol) were added thereto, and each mixture was shaken using the temperatures and the times described in Table 8-4-2-1.

Solid-phase purification

1 M DMEDA in NMP (0.669 mL) was added to the obtained reaction solution, and the mixture was shaken at room temperature for 1.5 hours. The suspension of the reaction solution and the resin was transferred to a 3 mL column with a filter using NMP (700 μL), filtered, and washed three times with 0.2 M DMEDA in NMP (1.5 mL), three times with NMP (1.5 mL), three times with 0.05 M $TBAHSO_4$ + DTBP in NMP (1.5 mL), three times with 0.05 M BTMG in NMP (1.5 mL), three times with 0.05 M DTBP in NMP (1.5 mL), three times with NMP/water = 1/1 (1.5 mL), three times with NMP (1.5 mL), three times with MeOH (1.5 mL), three times with DCM (1.5 mL), and three times with heptane (1.5 mL), and the obtained solid phase was dried under reduced pressure to obtain the mixtures described in Table 8-4-2-1.

[Table 359]

| [Table 8-4-2-1] Mixture used and obtained mixture in steps d21 to d30 | | | |
|---|---|---|---|
| Mixture used | Reaction temperature (°C) | Reaction time (h) | Obtained mixture |
| 2-1-b1C29-0 | 60 | 6 | 2-1-d21C29-0 |
| 2-1-b1C30-0 | 60 | 6 | 2-1-d22C30-0 |
| 2-1-b1C31-0 | 60 | 6 | 2-1-d23C31-0 |
| 2-1-b1C32-0 | 60 | 6 | 2-1-d24C32-0 |
| 2-1-b2C33-0 | 60 | 6 | 2-1-d25C33-0 |
| 2-1-b2C19-0 | 50 | 15 | 2-1-d26C19-0 |
| 2-1-b2C21-0 | 50 | 15 | 2-1-d27C21-0 |
| 2-1-b2C23-0 | 50 | 15 | 2-1-d28C23-0 |
| 2-1-b2C25-0 | 50 | 15 | 2-1-d29C25-0 |
| 2-1-b2C27-0 | 50 | 15 | 2-1-d30C27-0 |

Mixture 2-1-d21C29-0 to mixture 2-1-d25C33-0 and mixture 2-1 -d26C 19-0 to mixture 2-1-d30C27-0 can be treated by cleavage in the same manner as in Example 8-1-1 to obtain mixture 2-1-d21C29-1 to mixture 2-1-d25 C3 3-1 and mixture 2-1-d26C 19-1 to mixture 2-1-d30C27-1. The details of the mixtures are shown in Table 8-4-2-2 to Table 8-4-2-11.

A notation method in each table will be described. In Table 8-4-2-2 to Table 8-4-2- 11, each compound that may be contained in mixture 2-1-d21C29-1 to mixture 2-1-d25C33-1 and mixture 2-1-d26C19-1 to mixture 2-1-d30C27-1 is indicated by ID, and core blocks and linkers in combination in the structures of mixture 2-1-d21C29-1 to mixture 2-1-d25C33-1 and mixture 2-1-d26C19-1 to mixture 2-1-d30C27-1 are indicated by symbols or chemical formulas. The correspondence between the symbols and specific structures are shown in Figure 4. In Table 8-4-2-2 to Table 8-4-2-11, "×" and "c" are represented by chemical formulas.

[Formula 625]

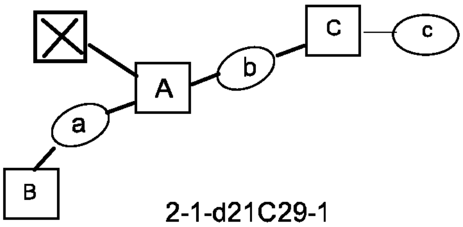


2-1-d21C29-1

For example, when ID is 2-1-d21C29-1-0001, the compound is represented by the following structural formula.

[Formula 626]

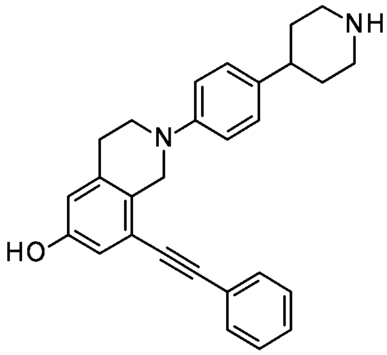

2-1-d21C29--1-0001

2-1-d21C29-1-0001

[Table 360]

| Notation in [Table 8-4-2-2] | | | | | | |
|---|---|---|---|---|---|---|
| ID | | a | B | b | C | c |
| 2-1-d21C29-1-0001 | OH | a2 | B11 | b1 | C29 | H |

[Table 361]

| Table 8-4-2-2] Compound that may be contained in mixture 2-1-d21C29-1 | | | | | | | |
|---|---|---|---|---|---|---|---|
| ID | Exact Mass | | a | B | b | C | c |
| 2-1-d21C29-1-0001 | 398.179 | OH | a2 | B11 | b1 | C29 | H |

(continued)

| Table 8-4-2-2] Compound that may be contained in mixture 2-1-d21C29-1 | | | | | | | |
|---|---|---|---|---|---|---|---|
| ID | Exact Mass | | a | B | b | C | c |
| 2-1-d21C29-1-0002 | 399.195 | OH | a2 | B12 | b1 | C29 | H |
| 2-1-d21C29-1-0003 | 414.206 | OH | a2 | B13 | b1 | C29 | H |
| 2-1-d21C29-1-0004 | 416.170 | OH | a2 | B14 | b1 | C29 | H |
| 2-1-d21C29-1-0005 | 417.169 | OH | a1 | B01 | b1 | C29 | H |
| 2-1-d21C29-1-0006 | 418.164 | OH | a1 | B02 | b1 | C29 | H |
| 2-1-d21C29-1-0007 | 430.186 | OH | a2 | B15 | b1 | C29 | H |
| 2-1-d21C29-1-0008 | 432.184 | OH | a2 | B16 | b1 | C29 | H |
| 2-1-d21C29-1-0009 | 434.144 | OH | a1 | B03 | b1 | C29 | H |
| 2-1-d21C29-1-0010 | 436.159 | OH | a2 | B17 | b1 | C29 | H |
| 2-1-d21C29-1-0011 | 437.155 | OH | a1 | B04 | b1 | C29 | H |
| 2-1-d21C29-1-0012 | 448.179 | OH | a1 | B05 | b1 | C29 | H |
| 2-1-d21C29-1-0013 | 450.231 | OH | a1 | B06 | b1 | C29 | H |
| 2-1-d21C29-1-0014 | 453.130 | OH | a2 | B18 | b1 | C29 | H |
| 2-1-d21C29-1-0015 | 456.141 | OH | a1 | B07 | b1 | C29 | H |
| 2-1-d21C29-1-0016 | 470.125 | OH | a2 | B19 | b1 | C29 | H |
| 2-1-d21C29-1-0017 | 478.187 | OH | a1 | B08 | b1 | C29 | H |
| 2-1-d21C29-1-0018 | 485.231 | OH | a2 | B20 | b1 | C29 | H |
| 2-1-d21C29-1-0019 | 486.231 | OH | a1 | B09 | b1 | C29 | H |
| 2-1-d21C29-1-0020 | 504.202 | OH | a1 | B10 | b1 | C29 | H |

[Table 362]

| [Table 8-4-2-3] Compound that may be contained in mixture 2-1-d22C30-1 | | | | | | | |
|---|---|---|---|---|---|---|---|
| ID | Exact Mass | | a | B | b | C | c |
| 2-1-d22C30-1-0001 | 374.199 | OH | a2 | B11 | b1 | C30 | H |
| 2-1-d22C30-1-0002 | 378.174 | OH | a2 | B12 | b1 | C30 | H |
| 2-1-d22C30-1-0003 | 383.163 | OH | a2 | B13 | b1 | C30 | H |
| 2-1-d22C30-1-0004 | 389.149 | OH | a2 | B14 | b1 | C30 | H |
| 2-1-d22C30-1-0005 | 390.194 | OH | a1 | B01 | b1 | C30 | H |
| 2-1-d22C30-1-0006 | 394.205 | OH | a1 | B02 | b1 | C30 | H |
| 2-1-d22C30-1-0007 | 398.163 | OH | a2 | B15 | b1 | C30 | H |
| 2-1-d22C30-1-0008 | 400.190 | OH | a2 | B16 | b1 | C30 | H |
| 2-1-d22C30-1-0009 | 405.149 | OH | a1 | B03 | b1 | C30 | H |
| 2-1-d22C30-1-0010 | 409.200 | OH | a2 | B17 | b1 | C30 | H |
| 2-1-d22C30-1-0011 | 410.199 | OH | a1 | B04 | b1 | C30 | H |
| 2-1-d22C30-1-0012 | 416.185 | OH | a1 | B05 | b1 | C30 | H |
| 2-1-d22C30-1-0013 | 419.163 | OH | a1 | B06 | b1 | C30 | H |
| 2-1-d22C30-1-0014 | 422.236 | OH | a2 | B18 | b1 | C30 | H |
| 2-1-d22C30-1-0015 | 428.174 | OH | a1 | B07 | b1 | C30 | H |

(continued)

| [Table 8-4-2-3] Compound that may be contained in mixture 2-1-d22C30-1 | | | | | | | |
|---|---|---|---|---|---|---|---|
| ID | Exact Mass | | a | B | b | C | c |
| 2-1-d22C30-1-0016 | 441.242 | OH | a2 | B19 | b1 | C30 | H |
| 2-1-d22C30-1-0017 | 449.191 | OH | a1 | B08 | b1 | C30 | H |
| 2-1-d22C30-1-0018 | 456.216 | OH | a2 | B20 | b1 | C30 | H |
| 2-1-d22C30-1-0019 | 463.172 | OH | a1 | B09 | b1 | C30 | H |
| 2-1-d22C30-1-0020 | 473.211 | OH | a1 | B10 | b1 | C30 | H |

[Table 363]

| [Table 8-4-2-4] Compound that may be contained in mixture 2-1-d23C31-1 | | | | | | | |
|---|---|---|---|---|---|---|---|
| ID | Exact Mass | | a | B | b | C | c |
| 2-1-d23C31-1-0001 | 429.205 | OH | a2 | B11 | b1 | C31 | H |
| 2-1-d23C31-1-0002 | 431.185 | OH | a2 | B12 | b1 | C31 | H |
| 2-1-d23C31-1-0003 | 445.121 | OH | a2 | B13 | b1 | C31 | H |
| 2-1-d23C31-1-0004 | 447.177 | OH | a2 | B14 | b1 | C31 | H |
| 2-1-d23C31-1-0005 | 448.176 | OH | a1 | B01 | b1 | C31 | H |
| 2-1-d23C31-1-0006 | 450.156 | OH | a1 | B02 | b1 | C31 | H |
| 2-1-d23C31-1-0007 | 457.237 | OH | a2 | B15 | b1 | C31 | H |
| 2-1-d23C31-1-0008 | 458.236 | OH | a2 | B16 | b1 | C31 | H |
| 2-1-d23C31-1-0009 | 464.246 | OH | a1 | B03 | b1 | C31 | H |
| 2-1-d23C31-1-0010 | 467.182 | OH | a2 | B17 | b1 | C31 | H |
| 2-1-d23C31-1-0011 | 469.161 | OH | a1 | B04 | b1 | C31 | H |
| 2-1-d23C31-1-0012 | 474.136 | OH | a1 | B05 | b1 | C31 | H |
| 2-1-d23C31-1-0013 | 482.145 | OH | a1 | B06 | b1 | C31 | H |
| 2-1-d23C31-1-0014 | 483.252 | OH | a2 | B18 | b1 | C31 | H |
| 2-1-d23C31-1-0015 | 485.156 | OH | a1 | B07 | b1 | C31 | H |
| 2-1-d23C31-1-0016 | 501.151 | OH | a2 | B19 | b1 | C31 | H |
| 2-1-d23C31-1-0017 | 511.208 | OH | a1 | B08 | b1 | C31 | H |
| 2-1-d23C31-1-0018 | 520.197 | OH | a2 | B20 | b1 | C31 | H |
| 2-1-d23C31-1-0019 | 523.208 | OH | a1 | B09 | b1 | C31 | H |
| 2-1-d23C31-1-0020 | 539.203 | OH | a1 | B10 | b1 | C31 | H |

[Table 364]

| [Table 8-4-2-5] Compound that may be contained in mixture 2-1-d24C32-1 | | | | | | | |
|---|---|---|---|---|---|---|---|
| ID | Exact Mass | | a | B | b | C | c |
| 2-1-d24C32-1-0001 | 386.143 | OH | a2 | B11 | b1 | C32 | H |
| 2-1-d24C32-1-0002 | 386.143 | OH | a2 | B12 | b1 | C32 | H |
| 2-1-d24C32-1-0003 | 399.158 | OH | a2 | B13 | b1 | C32 | H |
| 2-1-d24C32-1-0004 | 404.134 | OH | a2 | B14 | b1 | C32 | H |
| 2-1-d24C32-1-0005 | 405.149 | OH | a1 | B01 | b1 | C32 | H |
| 2-1-d24C32-1-0006 | 407.221 | OH | a1 | B02 | b1 | C32 | H |
| 2-1-d24C32-1-0007 | 415.171 | OH | a2 | B15 | b1 | C32 | H |
| 2-1-d24C32-1-0008 | 415.190 | OH | a2 | B16 | b1 | C32 | H |
| 2-1-d24C32-1-0009 | 417.169 | OH | a1 | B03 | b1 | C32 | H |
| 2-1-d24C32-1-0010 | 422.124 | OH | a2 | B17 | b1 | C32 | H |
| 2-1-d24C32-1-0011 | 424.135 | OH | a1 | B04 | b1 | C32 | H |
| 2-1-d24C32-1-0012 | 435.159 | OH | a1 | B05 | b1 | C32 | H |
| 2-1-d24C32-1-0013 | 436.140 | OH | a1 | B06 | b1 | C32 | H |
| 2-1-d24C32-1-0014 | 436.215 | OH | a2 | B18 | b1 | C32 | H |
| 2-1-d24C32-1-0015 | 444.166 | OH | a1 | B07 | b1 | C32 | H |
| 2-1-d24C32-1-0016 | 455.146 | OH | a2 | B19 | b1 | C32 | H |
| 2-1-d24C32-1-0017 | 466.150 | OH | a1 | B08 | b1 | C32 | H |
| 2-1-d24C32-1-0018 | 470.178 | OH | a2 | B20 | b1 | C32 | H |
| 2-1-d24C32-1-0019 | 472.196 | OH | a1 | B09 | b1 | C32 | H |
| 2-1-d24C32-1-0020 | 491.202 | OH | a1 | B10 | b1 | C32 | H |

[Table 365]

| [Table 8-4-2-6] Compound that may be contained in mixture 2-1-d25C33-1 | | | | | | | |
|---|---|---|---|---|---|---|---|
| ID | Exact Mass | | a | B | b | C | c |
| 2-1-d25C33-1-0001 | 360.184 | OH | a2 | B11 | b2 | C33 | H |
| 2-1-d25C33-1-0002 | 361.179 | OH | a2 | B12 | b2 | C33 | H |
| 2-1-d25C33-1-0003 | 368.152 | OH | a2 | B13 | b2 | C33 | H |
| 2-1-d25C33-1-0004 | 369.148 | OH | a2 | B14 | b2 | C33 | H |
| 2-1-d25C33-1-0005 | 369.148 | OH | a1 | B01 | b2 | C33 | H |
| 2-1-d25C33-1-0006 | 370.143 | OH | a1 | B02 | b2 | C33 | H |
| 2-1-d25C33-1-0007 | 382.168 | OH | a2 | B15 | b2 | C33 | H |
| 2-1-d25C33-1-0008 | 382.168 | OH | a2 | B16 | b2 | C33 | H |
| 2-1-d25C33-1-0009 | 383.163 | OH | a1 | B03 | b2 | C33 | H |
| 2-1-d25C33-1-0010 | 387.138 | OH | a2 | B17 | b2 | C33 | H |
| 2-1-d25C33-1-0011 | 388.215 | OH | a1 | B04 | b2 | C33 | H |
| 2-1-d25C33-1-0012 | 398.163 | OH | a1 | B05 | b2 | C33 | H |
| 2-1-d25C33-1-0013 | 399.158 | OH | a1 | B06 | b2 | C33 | H |
| 2-1-d25C33-1-0014 | 401.174 | OH | a2 | B18 | b2 | C33 | H |

(continued)

| [Table 8-4-2-6] Compound that may be contained in mixture 2-1-d25C33-1 | | | | | | | |
|---|---|---|---|---|---|---|---|
| ID | Exact Mass | | a | B | b | C | c |
| 2-1-d25C33-1-0015 | 408.220 | OH | a1 | B07 | b2 | C33 | H |
| 2-1-d25C33-1-0016 | 417.185 | OH | a2 | B19 | b2 | C33 | H |
| 2-1-d25C33-1-0017 | 429.205 | OH | a1 | B08 | b2 | C33 | H |
| 2-1-d25C33-1-0018 | 436.157 | OH | a2 | B20 | b2 | C33 | H |
| 2-1-d25C33-1-0019 | 438.231 | OH | a1 | B09 | b2 | C33 | H |
| 2-1-d25C33-1-0020 | 455.257 | OH | a1 | B10 | b2 | C33 | H |

[Table 366]

| [Table 8-4-2-7] Compound that may be contained in mixture 2-1-d26C 19-1 | | | | | | | |
|---|---|---|---|---|---|---|---|
| ID | Exact Mass | | a | B | b | C | c |
| 2-1-d26C19-1-0001 | 379.190 | OH | a2 | B11 | b2 | C19 | H |
| 2-1-d26C19-1-0002 | 380.185 | OH | a2 | B12 | b2 | C19 | H |
| 2-1-d26C19-1-0003 | 387.138 | OH | a2 | B13 | b2 | C19 | H |
| 2-1-d26C19-1-0004 | 393.205 | OH | a2 | B14 | b2 | C19 | H |
| 2-1-d26C19-1-0005 | 396.184 | OH | a1 | B01 | b2 | C19 | H |
| 2-1-d26C19-1-0006 | 396.184 | OH | a1 | B02 | b2 | C19 | H |
| 2-1-d26C19-1-0007 | 400.159 | OH | a2 | B15 | b2 | C19 | H |
| 2-1-d26C19-1-0008 | 402.169 | OH | a2 | B16 | b2 | C19 | H |
| 2-1-d26C19-1-0009 | 408.220 | OH | a1 | B03 | b2 | C19 | H |
| 2-1-d26C19-1-0010 | 410.199 | OH | a2 | B17 | b2 | C19 | H |
| 2-1-d26C19-1-0011 | 412.195 | OH | a1 | B04 | b2 | C19 | H |
| 2-1-d26C19-1-0012 | 418.164 | OH | a1 | B05 | b2 | C19 | H |
| 2-1-d26C19-1-0013 | 423.139 | OH | a1 | B06 | b2 | C19 | H |
| 2-1-d26C19-1-0014 | 427.226 | OH | a2 | B18 | b2 | C19 | H |
| 2-1-d26C19-1-0015 | 430.205 | OH | a1 | B07 | b2 | C19 | H |
| 2-1-d26C19-1-0016 | 444.201 | OH | a2 | B19 | b2 | C19 | H |
| 2-1-d26C19-1-0017 | 452.135 | OH | a1 | B08 | b2 | C19 | H |
| 2-1-d26C19-1-0018 | 458.141 | OH | a2 | B20 | b2 | C19 | H |
| 2-1-d26C19-1-0019 | 464.171 | OH | a1 | B09 | b2 | C19 | H |
| 2-1-d26C19-1-0020 | 481.198 | OH | a1 | B10 | b2 | C19 | H |

[Table 367]

| [Table 8-4-2-8] Compound that may be contained in mixture 2-1-d27C21-1 | | | | | | | |
|---|---|---|---|---|---|---|---|
| ID | Exact Mass | | a | B | b | C | c |
| 2-1-d27C21-1-0001 | 387.158 | OH | a2 | B11 | b2 | C21 | H |
| 2-1-d27C21-1-0002 | 388.154 | OH | a2 | B12 | b2 | C21 | H |
| 2-1-d27C21-1-0003 | 400.159 | OH | a2 | B13 | b2 | C21 | H |
| 2-1-d27C21-1-0004 | 406.144 | OH | a2 | B14 | b2 | C21 | H |
| 2-1-d27C21-1-0005 | 408.220 | OH | a1 | B01 | b2 | C21 | H |
| 2-1-d27C21-1-0006 | 409.215 | OH | a1 | B02 | b2 | C21 | H |
| 2-1-d27C21-1-0007 | 415.190 | OH | a2 | B15 | b2 | C21 | H |
| 2-1-d27C21-1-0008 | 416.154 | OH | a2 | B16 | b2 | C21 | H |
| 2-1-d27C21-1-0009 | 418.168 | OH | a1 | B03 | b2 | C21 | H |
| 2-1-d27C21-1-0010 | 424.215 | OH | a2 | B17 | b2 | C21 | H |
| 2-1-d27C21-1-0011 | 427.226 | OH | a1 | B04 | b2 | C21 | H |
| 2-1-d27C21-1-0012 | 435.176 | OH | a1 | B05 | b2 | C21 | H |
| 2-1-d27C21-1-0013 | 436.251 | OH | a1 | B06 | b2 | C21 | H |
| 2-1-d27C21-1-0014 | 437.210 | OH | a2 | B18 | b2 | C21 | H |
| 2-1-d27C21-1-0015 | 445.217 | OH | a1 | B07 | b2 | C21 | H |
| 2-1-d27C21-1-0016 | 456.162 | OH | a2 | B19 | b2 | C21 | H |
| 2-1-d27C21-1-0017 | 466.226 | OH | a1 | B08 | b2 | C21 | H |
| 2-1-d27C21-1-0018 | 471.141 | OH | a2 | B20 | b2 | C21 | H |
| 2-1-d27C21-1-0019 | 473.136 | OH | a1 | B09 | b2 | C21 | H |
| 2-1-d27C21-1-0020 | 492.202 | OH | a1 | B10 | b2 | C21 | H |

[Table 368]

| [Table 8-4-2-9] Compound that may be contained in mixture 2-1-d28C23-1 | | | | | | | |
|---|---|---|---|---|---|---|---|
| ID | Exact Mass | | a | B | b | C | c |
| 2-1-d28C23-1-0001 | 394.205 | OH | a2 | B11 | b2 | C23 | H |
| 2-1-d28C23-1-0002 | 395.200 | OH | a2 | B12 | b2 | C23 | H |
| 2-1-d28C23-1-0003 | 406.144 | OH | a2 | B13 | b2 | C23 | H |
| 2-1-d28C23-1-0004 | 412.179 | OH | a2 | B14 | b2 | C23 | H |
| 2-1-d28C23-1-0005 | 413.210 | OH | a1 | B01 | b2 | C23 | H |
| 2-1-d28C23-1-0006 | 413.210 | OH | a1 | B02 | b2 | C23 | H |
| 2-1-d28C23-1-0007 | 418.149 | OH | a2 | B15 | b2 | C23 | H |
| 2-1-d28C23-1-0008 | 419.165 | OH | a2 | B16 | b2 | C23 | H |
| 2-1-d28C23-1-0009 | 428.171 | OH | a1 | B03 | b2 | C23 | H |
| 2-1-d28C23-1-0010 | 428.221 | OH | a2 | B17 | b2 | C23 | H |
| 2-1-d28C23-1-0011 | 431.185 | OH | a1 | B04 | b2 | C23 | H |
| 2-1-d28C23-1-0012 | 441.130 | OH | a1 | B05 | b2 | C23 | H |
| 2-1-d28C23-1-0013 | 443.221 | OH | a1 | B06 | b2 | C23 | H |
| 2-1-d28C23-1-0014 | 444.220 | OH | a2 | B18 | b2 | C23 | H |

(continued)

| [Table 8-4-2-9] Compound that may be contained in mixture 2-1-d28C23-1 | | | | | | | |
|---|---|---|---|---|---|---|---|
| ID | Exact Mass | | a | B | b | C | c |
| 2-1-d28C23-1-0015 | 447.179 | OH | a1 | B07 | b2 | C23 | H |
| 2-1-d28C23-1-0016 | 463.207 | OH | a2 | B19 | b2 | C23 | H |
| 2-1-d28C23-1-0017 | 469.237 | OH | a1 | B08 | b2 | C23 | H |
| 2-1-d28C23-1-0018 | 476.208 | OH | a2 | B20 | b2 | C23 | H |
| 2-1-d28C23-1-0019 | 484.193 | OH | a1 | B09 | b2 | C23 | H |
| 2-1-d28C23-1-0020 | 497.193 | OH | a1 | B10 | b2 | C23 | H |

[Table 369]

| [Table 8-4-2-10] Compound that may be contained in mixture 2-1-d29C25-1 | | | | | | | |
|---|---|---|---|---|---|---|---|
| ID | Exact Mass | | a | B | b | C | c |
| 2-1-d29C25-1-0001 | 402.169 | OH | a2 | B11 | b2 | C25 | H |
| 2-1-d29C25-1-0002 | 404.134 | OH | a2 | B12 | b2 | C25 | H |
| 2-1-d29C25-1-0003 | 416.154 | OH | a2 | B13 | b2 | C25 | H |
| 2-1-d29C25-1-0004 | 419.165 | OH | a2 | B14 | b2 | C25 | H |
| 2-1-d29C25-1-0005 | 423.139 | OH | a1 | B01 | b2 | C25 | H |
| 2-1-d29C25-1-0006 | 426.194 | OH | a1 | B02 | b2 | C25 | H |
| 2-1-d29C25-1-0007 | 433.180 | OH | a2 | B15 | b2 | C25 | H |
| 2-1-d29C25-1-0008 | 435.159 | OH | a2 | B16 | b2 | C25 | H |
| 2-1-d29C25-1-0009 | 437.135 | OH | a1 | B03 | b2 | C25 | H |
| 2-1-d29C25-1-0010 | 441.242 | OH | a2 | B17 | b2 | C25 | H |
| 2-1-d29C25-1-0011 | 444.126 | OH | a1 | B04 | b2 | C25 | H |
| 2-1-d29C25-1-0012 | 454.130 | OH | a1 | B05 | b2 | C25 | H |
| 2-1-d29C25-1-0013 | 454.206 | OH | a1 | B06 | b2 | C25 | H |
| 2-1-d29C25-1-0014 | 455.221 | OH | a2 | B18 | b2 | C25 | H |
| 2-1-d29C25-1-0015 | 462.192 | OH | a1 | B07 | b2 | C25 | H |
| 2-1-d29C25-1-0016 | 472.136 | OH | a2 | B19 | b2 | C25 | H |
| 2-1-d29C25-1-0017 | 485.156 | OH | a1 | B08 | b2 | C25 | H |
| 2-1-d29C25-1-0018 | 489.131 | OH | a2 | B20 | b2 | C25 | H |
| 2-1-d29C25-1-0019 | 495.213 | OH | a1 | B09 | b2 | C25 | H |
| 2-1-d29C25-1-0020 | 512.188 | OH | a1 | B10 | b2 | C25 | H |

[Table 370]

| [Table 8-4-2-11] Compound that may be contained in mixture 2-1-d30C27-1 | | | | | | | |
|---|---|---|---|---|---|---|---|
| ID | Exact Mass | | a | B | b | C | c |
| 2-1-d30C27-1-0001 | 380.189 | OH | a2 | B11 | b2 | C27 | H |

(continued)

| [Table 8-4-2-11] Compound that may be contained in mixture 2-1-d30C27-1 | | | | | | | |
|---|---|---|---|---|---|---|---|
| ID | Exact Mass | | a | B | b | C | c |
| 2-1-d30C27-1-0002 | 381.184 | OH | a2 | B12 | b2 | C27 | H |
| 2-1-d30C27-1-0003 | 388.154 | OH | a2 | B13 | b2 | C27 | H |
| 2-1-d30C27-1-0004 | 396.165 | OH | a2 | B14 | b2 | C27 | H |
| 2-1-d30C27-1-0005 | 397.179 | OH | a1 | B01 | b2 | C27 | H |
| 2-1-d30C27-1-0006 | 397.180 | OH | a1 | B02 | b2 | C27 | H |
| 2-1-d30C27-1-0007 | 401.174 | OH | a2 | B15 | b2 | C27 | H |
| 2-1-d30C27-1-0008 | 405.129 | OH | a2 | B16 | b2 | C27 | H |
| 2-1-d30C27-1-0009 | 410.199 | OH | a1 | B03 | b2 | C27 | H |
| 2-1-d30C27-1-0010 | 412.179 | OH | a2 | B17 | b2 | C27 | H |
| 2-1-d30C27-1-0011 | 414.174 | OH | a1 | B04 | b2 | C27 | H |
| 2-1-d30C27-1-0012 | 422.236 | OH | a1 | B05 | b2 | C27 | H |
| 2-1-d30C27-1-0013 | 426.211 | OH | a1 | B06 | b2 | C27 | H |
| 2-1-d30C27-1-0014 | 427.226 | OH | a2 | B18 | b2 | C27 | H |
| 2-1-d30C27-1-0015 | 431.201 | OH | a1 | B07 | b2 | C27 | H |
| 2-1-d30C27-1-0016 | 445.200 | OH | a2 | B19 | b2 | C27 | H |
| 2-1-d30C27-1-0017 | 453.150 | OH | a1 | B08 | b2 | C27 | H |
| 2-1-d30C27-1-0018 | 462.116 | OH | a2 | B20 | b2 | C27 | H |
| 2-1-d30C27-1-0019 | 466.150 | OH | a1 | B09 | b2 | C27 | H |
| 2-1-d30C27-1-0020 | 483.177 | OH | a1 | B10 | b2 | C27 | H |

Example 8-5-1: Synthesis of 2-1-c1D01-0 by step c1D01

A reaction formula in step c1D01 performed using mixture 2-1-c1D00-0 will be illustrated below.

[Formula 627]

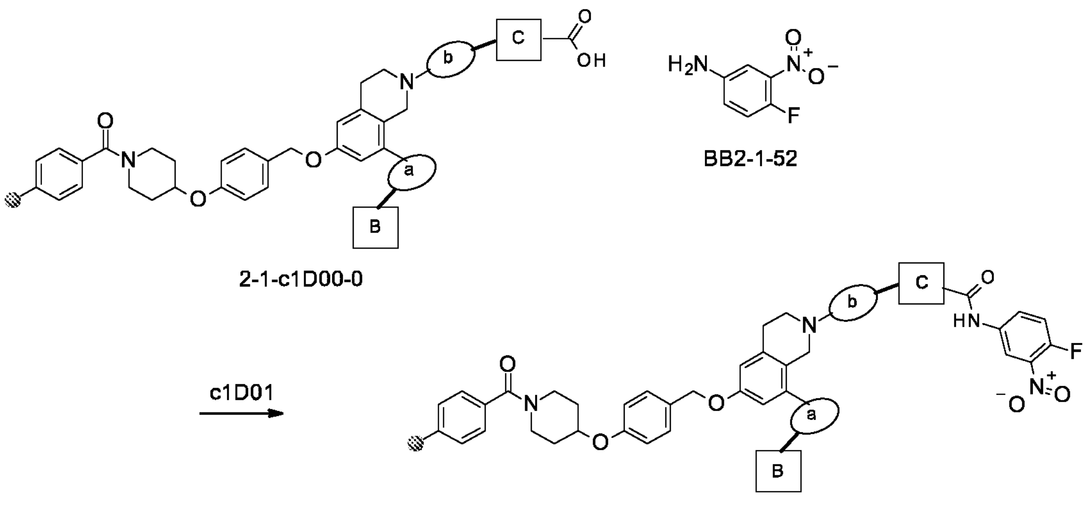


2-1-c1D00-0

BB2-1-52

2-1-c1D01-0

Mixture 2-1-c1D01-0 was synthesized as follows using mixture 2-1-h02C08-0 to mixture 2-1-h21C18-0.

20 types of mixtures described in Table 8-5-1-1 and DCM (10.5 mL) were added to a 20 mL column with a filter and shaken at room temperature for 1 hour. 4-Fluoro-3- nitroaniline (BB2-1-52, 0.211 g, 1.35 mmol) was added thereto. A solution of PipClU (0.195 g, 0.540 mmol) and NMI (42.7 μL, 0.540 mmol) in MeCN (0.54 mL) (1 M each of PipClU and NMI) was added thereto, and the mixture was shaken at room temperature for 20 hours.

Solid-phase purification

The suspension of the reaction solution and the resin was filtered through the filter of the column and washed three times with NMP/water = 1/1 (14 mL), three times with NMP (14 mL), three times with 0.05 M $TBAHSO_4$ + DTBP in NMP (14 mL), three times with 0.05 M NMM in NMP (14 mL), three times with NMP/water = 1/1 (14 mL), three times with NMP (14 mL), three times with MeOH (14 mL), three times with DCM (14 mL), and three times with heptane (14 mL), and the obtained solid phase was dried under reduced pressure to obtain mixture 2-1-c1D01-0.

[Table 371]

| [Table 8-5-1-1] Starting material mixture used in step c1D01 | |
|---|---|
| Mixture | Amount used |
| 2-1-h02C08-0 | 35mg |
| 2-1-h03C09-0 | 35mg |
| 2-1-h04C10-0 | 35mg |
| 2-1-h05C11-0 | 35mg |
| 2-1-h06C12-0 | 35mg |
| 2-1-h07C13-0 | 35mg |
| 2-1-h08C14-0 | 35mg |
| 2-1-h09C01-0 | 35mg |
| 2-1-h10C02-0 | 35mg |
| 2-1-h11C03-0 | 35mg |
| 2-1-h12C04-0 | 35mg |
| 2-1-h13C05-0 | 35mg |
| 2-1-h14C06-0 | 35mg |
| 2-1-h15C07-0 | 35mg |
| 2-1-h16C06-0 | 35mg |
| 2-1-h17C08-0 | 35mg |
| 2-1-h18C15-0 | 35mg |
| 2-1-h19C16-0 | 35mg |
| 2-1-h20C17-0 | 35mg |
| 2-1-h21C18-0 | 35mg |

Mixture 2-1-c1D01-0 can be treated by cleavage in the same manner as in Example 8-1-1 to obtain mixture 2-1-c1D01-1. The details of the mixture are shown in Table 8-5-1-2.

In Table 8-5-1-2, each compound that may be contained in mixture 2-1-c1D01-1 is indicated by ID, and core blocks and linkers in combination in the structure of mixture 2-1- c1D01-1 are indicated by symbols or chemical formulas. The correspondence between the symbols and specific structures are shown in Figure 4. In Table 8-5-1-2, "×" and "d" are represented by chemical formulas.

[Formula 628]

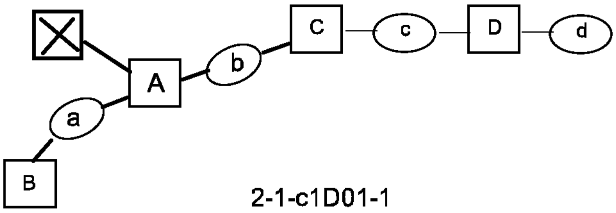

2-1-c1D01-1

For example, when ID is 2-1-c1D01-1-0001, the compound is represented by the following structural formula.

[Formula 629]

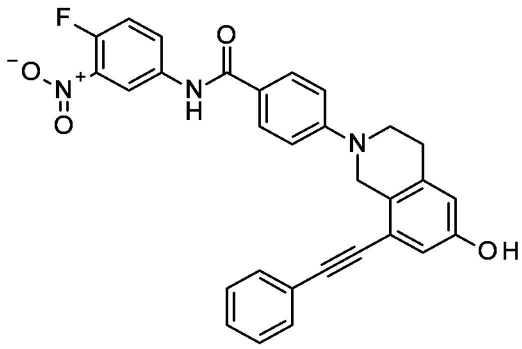

2-1-c1D01-1-0001

[Table 372]

| Notation in Table 8-5-1-2] | | | | | | | | |
|---|---|---|---|---|---|---|---|---|
| ID | | a | B | b | C | c | D | d |
| 2-1-c1D01-1-0001 | OH | a2 | B11 | b1 | C08 | c1 | D01 | F |

[Table 373]

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0001 | 507.159 | OH | a2 | B11 | b1 | C08 | c1 | D01 | F |
| 2-1-c1D01-1-0002 | 508.155 | OH | a2 | B12 | b1 | C08 | c1 | D01 | F |
| 2-1-c1D01-1-0003 | 508.155 | OH | a2 | B11 | b1 | C11 | c1 | D01 | F |
| 2-1-c1D01-1-0004 | 509.150 | OH | a2 | B11 | b1 | C10 | c1 | D01 | F |
| 2-1-c1D01-1-0005 | 509.150 | OH | a2 | B12 | b1 | C11 | c1 | D01 | F |
| 2-1-c1D01-1-0006 | 510.145 | OH | a2 | B12 | b1 | C10 | c1 | D01 | F |
| 2-1-c1D01-1-0007 | 514.111 | OH | a2 | B11 | b1 | C12 | c1 | D01 | F |
| 2-1-c1D01-1-0008 | 515.106 | OH | a2 | B12 | b1 | C12 | c1 | D01 | F |
| 2-1-c1D01-1-0009 | 521.175 | OH | a2 | B13 | b1 | C08 | c1 | D01 | F |
| 2-1-c1D01-1-0010 | 522.170 | OH | a2 | B13 | b1 | C11 | c1 | D01 | F |
| 2-1-c1D01-1-0011 | 523.166 | OH | a2 | B13 | b1 | C10 | c1 | D01 | F |
| 2-1-c1D01-1-0012 | 525.134 | OH | a2 | B11 | b2 | C01 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0013 | 525.150 | OH | a2 | B14 | b1 | C08 | c1 | D01 | F |
| 2-1-c1D01-1-0014 | 526.129 | OH | a2 | B12 | b2 | C01 | c1 | D01 | F |
| 2-1-c1D01-1-0015 | 526.145 | OH | a2 | B14 | b1 | C11 | c1 | D01 | F |
| 2-1-c1D01-1-0016 | 526.165 | OH | a1 | B01 | b1 | C08 | c1 | D01 | F |
| 2-1-c1D01-1-0017 | 527.141 | OH | a2 | B14 | b1 | C10 | c1 | D01 | F |
| 2-1-c1D01-1-0018 | 527.160 | OH | a1 | B02 | b1 | C08 | c1 | D01 | F |
| 2-1-c1D01-1-0019 | 527.160 | OH | a1 | B01 | b1 | C11 | c1 | D01 | F |
| 2-1-c1D01-1-0020 | 528.127 | OH | a2 | B13 | b1 | C12 | c1 | D01 | F |
| 2-1-c1D01-1-0021 | 528.156 | OH | a1 | B01 | b1 | C10 | c1 | D01 | F |
| 2-1-c1D01-1-0022 | 528.156 | OH | a1 | B02 | b1 | C11 | c1 | D01 | F |
| 2-1-c1D01-1-0023 | 529.151 | OH | a1 | B02 | b1 | C10 | c1 | D01 | F |
| 2-1-c1D01-1-0024 | 532.102 | OH | a2 | B14 | b1 | C12 | c1 | D01 | F |
| 2-1-c1D01-1-0025 | 533.117 | OH | a1 | B01 | b1 | C12 | c1 | D01 | F |
| 2-1-c1D01-1-0026 | 534.112 | OH | a1 | B02 | b1 | C12 | c1 | D01 | F |
| 2-1-c1D01-1-0027 | 535.154 | OH | a2 | B11 | b2 | C02 | c1 | D01 | F |
| 2-1-c1D01-1-0028 | 535.191 | OH | a2 | B15 | b1 | C08 | c1 | D01 | F |
| 2-1-c1D01-1-0029 | 536.150 | OH | a2 | B12 | b2 | C02 | c1 | D01 | F |
| 2-1-c1D01-1-0030 | 536.150 | OH | a2 | B11 | b2 | C03 | c1 | D01 | F |
| 2-1-c1D01-1-0031 | 536.186 | OH | a2 | B15 | b1 | C11 | c1 | D01 | F |
| 2-1-c1D01-1-0032 | 537.145 | OH | a2 | B12 | b2 | C03 | c1 | D01 | F |
| 2-1-c1D01-1-0033 | 537.170 | OH | a2 | B16 | b1 | C08 | c1 | D01 | F |
| 2-1-c1D01-1-0034 | 537.181 | OH | a2 | B15 | b1 | C10 | c1 | D01 | F |
| 2-1-c1D01-1-0035 | 538.165 | OH | a2 | B16 | b1 | C11 | c1 | D01 | F |
| 2-1-c1D01-1-0036 | 539.149 | OH | a2 | B13 | b2 | C01 | c1 | D01 | F |
| 2-1-c1D01-1-0037 | 539.160 | OH | a2 | B16 | b1 | C10 | c1 | D01 | F |
| 2-1-c1D01-1-0038 | 540.181 | OH | a1 | B03 | b1 | C08 | c1 | D01 | F |
| 2-1-c1D01-1-0039 | 541.111 | OH | a2 | B11 | b2 | C06 | c1 | D01 | F |
| 2-1-c1D01-1-0040 | 541.176 | OH | a1 | B03 | b1 | C11 | c1 | D01 | F |
| 2-1-c1D01-1-0041 | 542.106 | OH | a2 | B12 | b2 | C06 | c1 | D01 | F |
| 2-1-c1D01-1-0042 | 542.142 | OH | a2 | B15 | b1 | C12 | c1 | D01 | F |
| 2-1-c1D01-1-0043 | 542.171 | OH | a1 | B03 | b1 | C10 | c1 | D01 | F |
| 2-1-c1D01-1-0044 | 543.124 | OH | a2 | B14 | b2 | C01 | c1 | D01 | F |
| 2-1-c1D01-1-0045 | 543.141 | OH | a2 | B17 | b1 | C08 | c1 | D01 | F |
| 2-1-c1D01-1-0046 | 544.122 | OH | a2 | B16 | b1 | C12 | c1 | D01 | F |
| 2-1-c1D01-1-0047 | 544.136 | OH | a2 | B17 | b1 | C11 | c1 | D01 | F |
| 2-1-c1D01-1-0048 | 544.139 | OH | a1 | B01 | b2 | C01 | c1 | D01 | F |
| 2-1-c1D01-1-0049 | 544.156 | OH | a1 | B04 | b1 | C08 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0050 | 545.131 | OH | a2 | B17 | b1 | C10 | c1 | D01 | F |
| 2-1-c1D01-1-0051 | 545.135 | OH | a1 | B02 | b2 | C01 | c1 | D01 | F |
| 2-1-c1D01-1-0052 | 545.151 | OH | a1 | B04 | b1 | C11 | c1 | D01 | F |
| 2-1-c1D01-1-0053 | 546.146 | OH | a1 | B04 | b1 | C10 | c1 | D01 | F |
| 2-1-c1D01-1-0054 | 547.133 | OH | a1 | B03 | b1 | C12 | c1 | D01 | F |
| 2-1-c1D01-1-0055 | 549.170 | OH | a2 | B13 | b2 | C02 | c1 | D01 | F |
| 2-1-c1D01-1-0056 | 550.092 | OH | a2 | B17 | b1 | C12 | c1 | D01 | F |
| 2-1-c1D01-1-0057 | 550.165 | OH | a2 | B13 | b2 | C03 | c1 | D01 | F |
| 2-1-c1D01-1-0058 | 550.165 | OH | a2 | B11 | b2 | C05 | c1 | D01 | F |
| 2-1-c1D01-1-0059 | 551.107 | OH | a1 | B04 | b1 | C12 | c1 | D01 | F |
| 2-1-c1D01-1-0060 | 551.160 | OH | a2 | B12 | b2 | C05 | c1 | D01 | F |
| 2-1-c1D01-1-0061 | 553.145 | OH | a2 | B14 | b2 | C02 | c1 | D01 | F |
| 2-1-c1D01-1-0062 | 553.145 | OH | a2 | B11 | b2 | C04 | c1 | D01 | F |
| 2-1-c1D01-1-0063 | 553.165 | OH | a2 | B15 | b2 | C01 | c1 | D01 | F |
| 2-1-c1D01-1-0064 | 554.140 | OH | a2 | B12 | b2 | C04 | c1 | D01 | F |
| 2-1-c1D01-1-0065 | 554.140 | OH | a2 | B14 | b2 | C03 | c1 | D01 | F |
| 2-1-c1D01-1-0066 | 554.160 | OH | a1 | B01 | b2 | C02 | c1 | D01 | F |
| 2-1-c1D01-1-0067 | 554.197 | OH | a1 | B05 | b1 | C08 | c1 | D01 | F |
| 2-1-c1D01-1-0068 | 555.126 | OH | a2 | B13 | b2 | C06 | c1 | D01 | F |
| 2-1-c1D01-1-0069 | 555.144 | OH | a2 | B16 | b2 | C01 | c1 | D01 | F |
| 2-1-c1D01-1-0070 | 555.155 | OH | a1 | B02 | b2 | C02 | c1 | D01 | F |
| 2-1-c1D01-1-0071 | 555.155 | OH | a1 | B01 | b2 | C03 | c1 | D01 | F |
| 2-1-c1D01-1-0072 | 555.192 | OH | a1 | B05 | b1 | C11 | c1 | D01 | F |
| 2-1-c1D01-1-0073 | 556.151 | OH | a1 | B02 | b2 | C03 | c1 | D01 | F |
| 2-1-c1D01-1-0074 | 556.176 | OH | a1 | B06 | b1 | C08 | c1 | D01 | F |
| 2-1-c1D01-1-0075 | 556.187 | OH | a1 | B05 | b1 | C10 | c1 | D01 | F |
| 2-1-c1D01-1-0076 | 557.171 | OH | a1 | B06 | b1 | C11 | c1 | D01 | F |
| 2-1-c1D01-1-0077 | 557.175 | OH | a2 | B18 | b1 | C08 | c1 | D01 | F |
| 2-1-c1D01-1-0078 | 558.155 | OH | a1 | B03 | b2 | C01 | c1 | D01 | F |
| 2-1-c1D01-1-0079 | 558.166 | OH | a1 | B06 | b1 | C10 | c1 | D01 | F |
| 2-1-c1D01-1-0080 | 558.170 | OH | a2 | B18 | b1 | C11 | c1 | D01 | F |
| 2-1-c1D01-1-0081 | 559.101 | OH | a2 | B14 | b2 | C06 | c1 | D01 | F |
| 2-1-c1D01-1-0082 | 559.166 | OH | a2 | B18 | b1 | C10 | c1 | D01 | F |
| 2-1-c1D01-1-0083 | 559.166 | OH | a2 | B11 | b1 | C13 | c1 | D01 | F |
| 2-1-c1D01-1-0084 | 560.117 | OH | a1 | B01 | b2 | C06 | c1 | D01 | F |
| 2-1-c1D01-1-0085 | 560.161 | OH | a2 | B12 | b1 | C13 | c1 | D01 | F |
| 2-1-c1D01-1-0086 | 561.101 | OH | a2 | B11 | b3 | C15 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0087 | 561.112 | OH | a1 | B02 | b2 | C06 | c1 | D01 | F |
| 2-1-c1D01-1-0088 | 561.115 | OH | a2 | B17 | b2 | C01 | c1 | D01 | F |
| 2-1-c1D01-1-0089 | 561.148 | OH | a1 | B05 | b1 | C12 | c1 | D01 | F |
| 2-1-c1D01-1-0090 | 562.096 | OH | a2 | B12 | b3 | C15 | c1 | D01 | F |
| 2-1-c1D01-1-0091 | 562.130 | OH | a1 | B04 | b2 | C01 | c1 | D01 | F |
| 2-1-c1D01-1-0092 | 562.146 | OH | a1 | B07 | b1 | C08 | c1 | D01 | F |
| 2-1-c1D01-1-0093 | 563.127 | OH | a1 | B06 | b1 | C12 | c1 | D01 | F |
| 2-1-c1D01-1-0094 | 563.132 | OH | a2 | B11 | b1 | C14 | c1 | D01 | F |
| 2-1-c1D01-1-0095 | 563.142 | OH | a1 | B07 | b1 | C11 | c1 | D01 | F |
| 2-1-c1D01-1-0096 | 563.186 | OH | a2 | B15 | b2 | C02 | c1 | D01 | F |
| 2-1-c1D01-1-0097 | 564.127 | OH | a2 | B18 | b1 | C12 | c1 | D01 | F |
| 2-1-c1D01-1-0098 | 564.127 | OH | a2 | B12 | b1 | C14 | c1 | D01 | F |
| 2-1-c1D01-1-0099 | 564.137 | OH | a1 | B07 | b1 | C10 | c1 | D01 | F |
| 2-1-c1D01-1-0100 | 564.181 | OH | a2 | B15 | b2 | C03 | c1 | D01 | F |
| 2-1-c1D01-1-0101 | 564.181 | OH | a2 | B13 | b2 | C05 | c1 | D01 | F |
| 2-1-c1D01-1-0102 | 565.165 | OH | a2 | B16 | b2 | C02 | c1 | D01 | F |
| 2-1-c1D01-1-0103 | 566.160 | OH | a2 | B16 | b2 | C03 | c1 | D01 | F |
| 2-1-c1D01-1-0104 | 567.161 | OH | a2 | B13 | b2 | C04 | c1 | D01 | F |
| 2-1-c1D01-1-0105 | 567.217 | OH | a2 | B11 | b2 | C07 | c1 | D01 | F |
| 2-1-c1D01-1-0106 | 568.156 | OH | a2 | B14 | b2 | COS | c1 | D01 | F |
| 2-1-c1D01-1-0107 | 568.176 | OH | a1 | B03 | b2 | C02 | c1 | D01 | F |
| 2-1-c1D01-1-0108 | 568.212 | OH | a2 | B12 | b2 | C07 | c1 | D01 | F |
| 2-1-c1D01-1-0109 | 569.098 | OH | a1 | B07 | b1 | C12 | c1 | D01 | F |
| 2-1-c1D01-1-0110 | 569.142 | OH | a2 | B15 | b2 | C06 | c1 | D01 | F |
| 2-1-c1D01-1-0111 | 569.171 | OH | a1 | B03 | b2 | C03 | c1 | D01 | F |
| 2-1-c1D01-1-0112 | 569.171 | OH | a1 | B01 | b2 | C05 | c1 | D01 | F |
| 2-1-c1D01-1-0113 | 570.166 | OH | a1 | B02 | b2 | C05 | c1 | D01 | F |
| 2-1-c1D01-1-0114 | 571.121 | OH | a2 | B16 | b2 | C06 | c1 | D01 | F |
| 2-1-c1D01-1-0115 | 571.121 | OH | a2 | B11 | b3 | C08 | c1 | D01 | F |
| 2-1-c1D01-1-0116 | 571.121 | OH | a2 | B17 | b2 | C02 | c1 | D01 | F |
| 2-1-c1D01-1-0117 | 571.136 | OH | a2 | B14 | b2 | C04 | c1 | D01 | F |
| 2-1-c1D01-1-0118 | 571.136 | OH | a2 | B12 | b3 | C08 | c1 | D01 | F |
| 2-1-c1D01-1-0119 | 572.117 | OH | a2 | B17 | b2 | C03 | c1 | D01 | F |
| 2-1-c1D01-1-0120 | 572.117 | OH | a1 | B04 | b2 | C02 | c1 | D01 | F |
| 2-1-c1D01-1-0121 | 572.117 | OH | a1 | B01 | b2 | C04 | c1 | D01 | F |
| 2-1-c1D01-1-0122 | 572.131 | OH | a1 | B05 | b2 | C01 | c1 | D01 | F |
| 2-1-c1D01-1-0123 | 572.171 | OH | a1 | B02 | b2 | C04 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0124 | 573.112 | OH | a1 | B04 | b2 | C03 | c1 | D01 | F |
| 2-1-c1D01-1-0125 | 573.112 | OH | a2 | B13 | b1 | C13 | c1 | D01 | F |
| 2-1-c1D01-1-0126 | 573.146 | OH | a1 | B03 | b2 | C06 | c1 | D01 | F |
| 2-1-c1D01-1-0127 | 573.146 | OH | a1 | B06 | b2 | C01 | c1 | D01 | F |
| 2-1-c1D01-1-0128 | 573.181 | OH | a2 | B13 | b3 | C15 | c1 | D01 | F |
| 2-1-c1D01-1-0129 | 574.107 | OH | a2 | B19 | b1 | C08 | c1 | D01 | F |
| 2-1-c1D01-1-0130 | 574.132 | OH | a2 | B11 | b1 | C09 | c1 | D01 | F |
| 2-1-c1D01-1-0131 | 574.150 | OH | a2 | B18 | b2 | C01 | c1 | D01 | F |
| 2-1-c1D01-1-0132 | 575.116 | OH | a2 | B12 | b1 | C09 | c1 | D01 | F |
| 2-1-c1D01-1-0133 | 575.147 | OH | a2 | B19 | b1 | C11 | c1 | D01 | F |
| 2-1-c1D01-1-0134 | 575.147 | OH | a2 | B11 | b3 | C06 | c1 | D01 | F |
| 2-1-c1D01-1-0135 | 575.149 | OH | a2 | B17 | b2 | C06 | c1 | D01 | F |
| 2-1-c1D01-1-0136 | 576.142 | OH | a2 | B19 | b1 | C10 | c1 | D01 | F |
| 2-1-c1D01-1-0137 | 576.142 | OH | a2 | B13 | b1 | C14 | c1 | D01 | F |
| 2-1-c1D01-1-0138 | 577.078 | OH | a2 | B14 | b1 | C13 | c1 | D01 | F |
| 2-1-c1D01-1-0139 | 577.137 | OH | a2 | B12 | b3 | C06 | c1 | D01 | F |
| 2-1-c1D01-1-0140 | 577.156 | OH | a1 | B04 | b2 | C06 | c1 | D01 | F |
| 2-1-c1D01-1-0141 | 577.168 | OH | a1 | B01 | b1 | C13 | c1 | D01 | F |
| 2-1-c1D01-1-0142 | 578.073 | OH | a2 | B15 | b2 | C05 | c1 | D01 | F |
| 2-1-c1D01-1-0143 | 578.107 | OH | a2 | B14 | b3 | C15 | c1 | D01 | F |
| 2-1-c1D01-1-0144 | 578.171 | OH | a1 | B02 | b1 | C13 | c1 | D01 | F |
| 2-1-c1D01-1-0145 | 578.197 | OH | a1 | B01 | b3 | C15 | c1 | D01 | F |
| 2-1-c1D01-1-0146 | 579.068 | OH | a1 | B07 | b2 | C01 | c1 | D01 | F |
| 2-1-c1D01-1-0147 | 579.091 | OH | a2 | B16 | b2 | C05 | c1 | D01 | F |
| 2-1-c1D01-1-0148 | 579.167 | OH | a1 | B02 | b3 | C15 | c1 | D01 | F |
| 2-1-c1D01-1-0149 | 580.106 | OH | a2 | B14 | b1 | C14 | c1 | D01 | F |
| 2-1-c1D01-1-0150 | 580.176 | OH | a2 | B15 | b2 | C04 | c1 | D01 | F |
| 2-1-c1D01-1-0151 | 581.102 | OH | a2 | B13 | b2 | C07 | c1 | D01 | F |
| 2-1-c1D01-1-0152 | 581.122 | OH | a2 | B19 | b1 | C12 | c1 | D01 | F |
| 2-1-c1D01-1-0153 | 581.143 | OH | a1 | B01 | b1 | C14 | c1 | D01 | F |
| 2-1-c1D01-1-0154 | 581.233 | OH | a1 | B05 | b2 | C02 | c1 | D01 | F |
| 2-1-c1D01-1-0155 | 582.098 | OH | a1 | B08 | b1 | C08 | c1 | D01 | F |
| 2-1-c1D01-1-0156 | 582.137 | OH | a1 | B02 | b1 | C14 | c1 | D01 | F |
| 2-1-c1D01-1-0157 | 582.191 | OH | a2 | B16 | b2 | C04 | c1 | D01 | F |
| 2-1-c1D01-1-0158 | 583.133 | OH | a1 | B05 | b2 | C03 | c1 | D01 | F |
| 2-1-c1D01-1-0159 | 583.154 | OH | a1 | B03 | b2 | C05 | c1 | D01 | F |
| 2-1-c1D01-1-0160 | 583.155 | OH | a1 | B08 | b1 | C11 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0161 | 583.187 | OH | a1 | B06 | b2 | C02 | c1 | D01 | F |
| 2-1-c1D01-1-0162 | 583.187 | OH | a1 | B08 | b1 | C10 | c1 | D01 | F |
| 2-1-c1D01-1-0163 | 584.128 | OH | a2 | B13 | b3 | C08 | c1 | D01 | F |
| 2-1-c1D01-1-0164 | 585.123 | OH | a1 | B06 | b2 | C03 | c1 | D01 | F |
| 2-1-c1D01-1-0165 | 585.137 | OH | a2 | B18 | b2 | C02 | c1 | D01 | F |
| 2-1-c1D01-1-0166 | 585.137 | OH | a2 | B14 | b2 | C07 | c1 | D01 | F |
| 2-1-c1D01-1-0167 | 585.170 | OH | a2 | B17 | b2 | C05 | c1 | D01 | F |
| 2-1-c1D01-1-0168 | 585.208 | OH | a2 | B18 | b2 | C03 | c1 | D01 | F |
| 2-1-c1D01-1-0169 | 586.132 | OH | a1 | B03 | b2 | C04 | c1 | D01 | F |
| 2-1-c1D01-1-0170 | 586.132 | OH | a1 | B01 | b2 | C07 | c1 | D01 | F |
| 2-1-c1D01-1-0171 | 586.165 | OH | a1 | B04 | b2 | C05 | c1 | D01 | F |
| 2-1-c1D01-1-0172 | 586.166 | OH | a2 | B15 | b1 | C13 | c1 | D01 | F |
| 2-1-c1D01-1-0173 | 586.223 | OH | a1 | B02 | b2 | C07 | c1 | D01 | F |
| 2-1-c1D01-1-0174 | 587.127 | OH | a1 | B05 | b2 | C06 | c1 | D01 | F |
| 2-1-c1D01-1-0175 | 587.197 | OH | a2 | B14 | b3 | C08 | c1 | D01 | F |
| 2-1-c1D01-1-0176 | 588.148 | OH | a2 | B17 | b2 | C04 | c1 | D01 | F |
| 2-1-c1D01-1-0177 | 589.096 | OH | a2 | B15 | b3 | C15 | c1 | D01 | F |
| 2-1-c1D01-1-0178 | 589.112 | OH | a2 | B13 | b1 | C09 | c1 | D01 | F |
| 2-1-c1D01-1-0179 | 589.112 | OH | a2 | B16 | b1 | C13 | c1 | D01 | F |
| 2-1-c1D01-1-0180 | 589.112 | OH | a1 | B08 | b1 | C12 | c1 | D01 | F |
| 2-1-c1D01-1-0181 | 589.132 | OH | a1 | B06 | b2 | C06 | c1 | D01 | F |
| 2-1-c1D01-1-0182 | 589.162 | OH | a1 | B01 | b3 | C08 | c1 | D01 | F |
| 2-1-c1D01-1-0183 | 590.084 | OH | a1 | B07 | b2 | C02 | c1 | D01 | F |
| 2-1-c1D01-1-0184 | 590.091 | OH | a1 | B04 | b2 | C04 | c1 | D01 | F |
| 2-1-c1D01-1-0185 | 590.107 | OH | a2 | B13 | b3 | C06 | c1 | D01 | F |
| 2-1-c1D01-1-0186 | 590.107 | OH | a2 | B16 | b3 | C15 | c1 | D01 | F |
| 2-1-c1D01-1-0187 | 590.127 | OH | a1 | B02 | b3 | C08 | c1 | D01 | F |
| 2-1-c1D01-1-0188 | 590.127 | OH | a2 | B18 | b2 | C06 | c1 | D01 | F |
| 2-1-c1D01-1-0189 | 590.141 | OH | a1 | B07 | b2 | C03 | c1 | D01 | F |
| 2-1-c1D01-1-0190 | 590.141 | OH | a2 | B20 | b1 | C08 | c1 | D01 | F |
| 2-1-c1D01-1-0191 | 591.093 | OH | a2 | B15 | b1 | C14 | c1 | D01 | F |
| 2-1-c1D01-1-0192 | 591.122 | OH | a2 | B20 | b1 | C11 | c1 | D01 | F |
| 2-1-c1D01-1-0193 | 591.122 | OH | a1 | B03 | b1 | C13 | c1 | D01 | F |
| 2-1-c1D01-1-0194 | 591.126 | OH | a2 | B19 | b2 | C01 | c1 | D01 | F |
| 2-1-c1D01-1-0195 | 591.137 | OH | a2 | B20 | b1 | C10 | c1 | D01 | F |
| 2-1-c1D01-1-0196 | 591.142 | OH | a2 | B14 | b1 | C09 | c1 | D01 | F |
| 2-1-c1D01-1-0197 | 591.163 | OH | a2 | B16 | b1 | C14 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0198 | 592.089 | OH | a1 | B03 | b3 | C15 | c1 | D01 | F |
| 2-1-c1D01-1-0199 | 592.118 | OH | a1 | B09 | b1 | C08 | c1 | D01 | F |
| 2-1-c1D01-1-0200 | 592.118 | OH | a1 | B01 | b1 | C09 | c1 | D01 | F |
| 2-1-c1D01-1-0201 | 592.118 | OH | a2 | B14 | b3 | C06 | c1 | D01 | F |
| 2-1-c1D01-1-0202 | 592.137 | OH | a2 | B17 | b1 | C13 | c1 | D01 | F |
| 2-1-c1D01-1-0203 | 592.187 | OH | a1 | B02 | b1 | C09 | c1 | D01 | F |
| 2-1-c1D01-1-0204 | 593.113 | OH | a1 | B09 | b1 | C11 | c1 | D01 | F |
| 2-1-c1D01-1-0205 | 593.121 | OH | a2 | B15 | b2 | C07 | c1 | D01 | F |
| 2-1-c1D01-1-0206 | 593.137 | OH | a1 | B01 | b3 | C06 | c1 | D01 | F |
| 2-1-c1D01-1-0207 | 593.142 | OH | a1 | B07 | b2 | C06 | c1 | D01 | F |
| 2-1-c1D01-1-0208 | 593.163 | OH | a1 | B09 | b1 | C10 | c1 | D01 | F |
| 2-1-c1D01-1-0209 | 594.122 | OH | a1 | B03 | b1 | C14 | c1 | D01 | F |
| 2-1-c1D01-1-0210 | 594.153 | OH | a1 | B04 | b1 | C13 | c1 | D01 | F |
| 2-1-c1D01-1-0211 | 595.068 | OH | a1 | B02 | b3 | C06 | c1 | D01 | F |
| 2-1-c1D01-1-0212 | 595.148 | OH | a2 | B17 | b3 | C15 | c1 | D01 | F |
| 2-1-c1D01-1-0213 | 596.064 | OH | a1 | B05 | b2 | C05 | c1 | D01 | F |
| 2-1-c1D01-1-0214 | 596.084 | OH | a2 | B16 | b2 | C07 | c1 | D01 | F |
| 2-1-c1D01-1-0215 | 596.143 | OH | a2 | B20 | b1 | C12 | c1 | D01 | F |
| 2-1-c1D01-1-0216 | 596.153 | OH | a1 | B04 | b3 | C15 | c1 | D01 | F |
| 2-1-c1D01-1-0217 | 596.174 | OH | a2 | B17 | b1 | C14 | c1 | D01 | F |
| 2-1-c1D01-1-0218 | 597.082 | OH | a2 | B15 | b3 | C08 | c1 | D01 | F |
| 2-1-c1D01-1-0219 | 597.228 | OH | a1 | B06 | b2 | C05 | c1 | D01 | F |
| 2-1-c1D01-1-0220 | 598.097 | OH | a1 | B04 | b1 | C14 | c1 | D01 | F |
| 2-1-c1D01-1-0221 | 599.153 | OH | a2 | B18 | b2 | C05 | c1 | D01 | F |
| 2-1-c1D01-1-0222 | 599.153 | OH | a1 | B05 | b2 | C04 | c1 | D01 | F |
| 2-1-c1D01-1-0223 | 599.153 | OH | a1 | B03 | b2 | C07 | c1 | D01 | F |
| 2-1-c1D01-1-0224 | 599.153 | OH | a1 | B09 | b1 | C12 | c1 | D01 | F |
| 2-1-c1D01-1-0225 | 599.182 | OH | a1 | B08 | b2 | C01 | c1 | D01 | F |
| 2-1-c1D01-1-0226 | 600.111 | OH | a2 | B16 | b3 | C08 | c1 | D01 | F |
| 2-1-c1D01-1-0227 | 600.181 | OH | a1 | B06 | b2 | C04 | c1 | D01 | F |
| 2-1-c1D01-1-0228 | 601.132 | OH | a2 | B19 | b2 | C02 | c1 | D01 | F |
| 2-1-c1D01-1-0229 | 601.132 | OH | a2 | B18 | b2 | C04 | c1 | D01 | F |
| 2-1-c1D01-1-0230 | 601.132 | OH | a2 | B15 | b1 | C09 | c1 | D01 | F |
| 2-1-c1D01-1-0231 | 601.143 | OH | a2 | B17 | b2 | C07 | c1 | D01 | F |
| 2-1-c1D01-1-0232 | 602.127 | OH | a2 | B19 | b2 | C03 | c1 | D01 | F |
| 2-1-c1D01-1-0233 | 602.127 | OH | a1 | B03 | b3 | C08 | c1 | D01 | F |
| 2-1-c1D01-1-0234 | 603.111 | OH | a1 | B04 | b2 | C07 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0235 | 603.128 | OH | a2 | B15 | b3 | C06 | c1 | D01 | F |
| 2-1-c1D01-1-0236 | 603.161 | OH | a1 | B07 | b2 | C05 | c1 | D01 | F |
| 2-1-c1D01-1-0237 | 603.178 | OH | a2 | B16 | b1 | C09 | c1 | D01 | F |
| 2-1-c1D01-1-0238 | 604.143 | OH | a1 | B05 | b1 | C13 | c1 | D01 | F |
| 2-1-c1D01-1-0239 | 604.213 | OH | a2 | B16 | b3 | C06 | c1 | D01 | F |
| 2-1-c1D01-1-0240 | 605.073 | OH | a2 | B17 | b3 | C08 | c1 | D01 | F |
| 2-1-c1D01-1-0241 | 605.152 | OH | a1 | B04 | b3 | C08 | c1 | D01 | F |
| 2-1-c1D01-1-0242 | 606.068 | OH | a1 | B07 | b2 | C04 | c1 | D01 | F |
| 2-1-c1D01-1-0243 | 606.104 | OH | a1 | B05 | b3 | C15 | c1 | D01 | F |
| 2-1-c1D01-1-0244 | 606.133 | OH | a1 | B03 | b1 | C09 | c1 | D01 | F |
| 2-1-c1D01-1-0245 | 606.203 | OH | a1 | B06 | b1 | C13 | c1 | D01 | F |
| 2-1-c1D01-1-0246 | 607.102 | OH | a2 | B19 | b2 | C06 | c1 | D01 | F |
| 2-1-c1D01-1-0247 | 607.102 | OH | a2 | B20 | b2 | C01 | c1 | D01 | F |
| 2-1-c1D01-1-0248 | 607.102 | OH | a2 | B18 | b1 | C13 | c1 | D01 | F |
| 2-1-c1D01-1-0249 | 608.084 | OH | a1 | B03 | b3 | C06 | c1 | D01 | F |
| 2-1-c1D01-1-0250 | 608.098 | OH | a1 | B06 | b3 | C15 | c1 | D01 | F |
| 2-1-c1D01-1-0251 | 608.101 | OH | a1 | B10 | b1 | C08 | c1 | D01 | F |
| 2-1-c1D01-1-0252 | 608.118 | OH | a1 | B05 | b1 | C14 | c1 | D01 | F |
| 2-1-c1D01-1-0253 | 608.118 | OH | a2 | B18 | b3 | C15 | c1 | D01 | F |
| 2-1-c1D01-1-0254 | 608.132 | OH | a1 | B08 | b2 | C02 | c1 | D01 | F |
| 2-1-c1D01-1-0255 | 608.138 | OH | a2 | B17 | b1 | C09 | c1 | D01 | F |
| 2-1-c1D01-1-0256 | 608.168 | OH | a1 | B10 | b1 | C11 | c1 | D01 | F |
| 2-1-c1D01-1-0257 | 609.098 | OH | a1 | B08 | b2 | C03 | c1 | D01 | F |
| 2-1-c1D01-1-0258 | 609.113 | OH | a1 | B09 | b2 | C01 | c1 | D01 | F |
| 2-1-c1D01-1-0259 | 609.113 | OH | a1 | B10 | b1 | C10 | c1 | D01 | F |
| 2-1-c1D01-1-0260 | 609.113 | OH | a1 | B04 | b1 | C09 | c1 | D01 | F |
| 2-1-c1D01-1-0261 | 609.116 | OH | a1 | B06 | b1 | C14 | c1 | D01 | F |
| 2-1-c1D01-1-0262 | 610.108 | OH | a2 | B17 | b3 | C06 | c1 | D01 | F |
| 2-1-c1D01-1-0263 | 610.148 | OH | a2 | B18 | b1 | C14 | c1 | D01 | F |
| 2-1-c1D01-1-0264 | 611.128 | OH | a1 | B04 | b3 | C06 | c1 | D01 | F |
| 2-1-c1D01-1-0265 | 611.189 | OH | a1 | B07 | b1 | C13 | c1 | D01 | F |
| 2-1-c1D01-1-0266 | 612.123 | OH | a1 | B05 | b2 | C07 | c1 | D01 | F |
| 2-1-c1D01-1-0267 | 612.184 | OH | a1 | B07 | b3 | C15 | c1 | D01 | F |
| 2-1-c1D01-1-0268 | 613.168 | OH | a1 | B06 | b2 | C07 | c1 | D01 | F |
| 2-1-c1D01-1-0269 | 613.168 | OH | a1 | B08 | b2 | C06 | c1 | D01 | F |
| 2-1-c1D01-1-0270 | 614.054 | OH | a1 | B10 | b1 | C12 | c1 | D01 | F |
| 2-1-c1D01-1-0271 | 615.122 | OH | a2 | B18 | b2 | C07 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0272 | 615.164 | OH | a1 | B07 | b1 | C14 | c1 | D01 | F |
| 2-1-c1D01-1-0273 | 616.179 | OH | a2 | B19 | b2 | C05 | c1 | D01 | F |
| 2-1-c1D01-1-0274 | 616.233 | OH | a1 | B05 | b3 | C08 | c1 | D01 | F |
| 2-1-c1D01-1-0275 | 617.233 | OH | a2 | B20 | b2 | C02 | c1 | D01 | F |
| 2-1-c1D01-1-0276 | 618.153 | OH | a2 | B20 | b2 | C03 | c1 | D01 | F |
| 2-1-c1D01-1-0277 | 618.158 | OH | a1 | B06 | b3 | C08 | c1 | D01 | F |
| 2-1-c1D01-1-0278 | 619.117 | OH | a2 | B19 | b2 | C04 | c1 | D01 | F |
| 2-1-c1D01-1-0279 | 619.122 | OH | a2 | B18 | b3 | C08 | c1 | D01 | F |
| 2-1-c1D01-1-0280 | 620.138 | OH | a1 | B09 | b2 | C02 | c1 | D01 | F |
| 2-1-c1D01-1-0281 | 620.138 | OH | a1 | B05 | b1 | C09 | c1 | D01 | F |
| 2-1-c1D01-1-0282 | 620.138 | OH | a1 | B07 | b2 | C07 | c1 | D01 | F |
| 2-1-c1D01-1-0283 | 621.133 | OH | a1 | B09 | b2 | C03 | c1 | D01 | F |
| 2-1-c1D01-1-0284 | 621.133 | OH | a1 | B05 | b3 | C06 | c1 | D01 | F |
| 2-1-c1D01-1-0285 | 621.137 | OH | a1 | B06 | b1 | C09 | c1 | D01 | F |
| 2-1-c1D01-1-0286 | 622.132 | OH | a2 | B20 | b2 | C06 | c1 | D01 | F |
| 2-1-c1D01-1-0287 | 622.133 | OH | a2 | B11 | b3 | C16 | c1 | D01 | F |
| 2-1-c1D01-1-0288 | 622.133 | OH | a2 | B18 | b1 | C09 | c1 | D01 | F |
| 2-1-c1D01-1-0289 | 623.127 | OH | a1 | B06 | b3 | C06 | c1 | D01 | F |
| 2-1-c1D01-1-0290 | 623.127 | OH | a1 | B07 | b3 | C08 | c1 | D01 | F |
| 2-1-c1D01-1-0291 | 624.078 | OH | a2 | B12 | b3 | C16 | c1 | D01 | F |
| 2-1-c1D01-1-0292 | 624.115 | OH | a1 | B08 | b2 | C05 | c1 | D01 | F |
| 2-1-c1D01-1-0293 | 624.163 | OH | a2 | B18 | b3 | C06 | c1 | D01 | F |
| 2-1-c1D01-1-0294 | 625.062 | OH | a2 | B11 | b3 | C17 | c1 | D01 | F |
| 2-1-c1D01-1-0295 | 625.093 | OH | a2 | B19 | b1 | C13 | c1 | D01 | F |
| 2-1-c1D01-1-0296 | 625.162 | OH | a2 | B12 | b3 | C17 | c1 | D01 | F |
| 2-1-c1D01-1-0297 | 626.092 | OH | a1 | B09 | b2 | C06 | c1 | D01 | F |
| 2-1-c1D01-1-0298 | 626.094 | OH | a1 | B10 | b2 | C01 | c1 | D01 | F |
| 2-1-c1D01-1-0299 | 626.108 | OH | a2 | B19 | b3 | C15 | c1 | D01 | F |
| 2-1-c1D01-1-0300 | 626.108 | OH | a1 | B08 | b2 | C04 | c1 | D01 | F |
| 2-1-c1D01-1-0301 | 627.093 | OH | a1 | B07 | b1 | C09 | c1 | D01 | F |
| 2-1-c1D01-1-0302 | 627.153 | OH | a2 | B19 | b1 | C14 | c1 | D01 | F |
| 2-1-c1D01-1-0303 | 628.104 | OH | a1 | B07 | b3 | C06 | c1 | D01 | F |
| 2-1-c1D01-1-0304 | 631.142 | OH | a2 | B20 | b2 | C05 | c1 | D01 | F |
| 2-1-c1D01-1-0305 | 633.060 | OH | a1 | B08 | b1 | C13 | c1 | D01 | F |
| 2-1-c1D01-1-0306 | 633.155 | OH | a2 | B19 | b2 | C07 | c1 | D01 | F |
| 2-1-c1D01-1-0307 | 635.097 | OH | a1 | B08 | b3 | C15 | c1 | D01 | F |
| 2-1-c1D01-1-0308 | 635.153 | OH | a2 | B20 | b2 | C04 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0309 | 636.078 | OH | a1 | B09 | b2 | C05 | c1 | D01 | F |
| 2-1-c1D01-1-0310 | 636.113 | OH | a1 | B10 | b2 | C02 | c1 | D01 | F |
| 2-1-c1D01-1-0311 | 637.074 | OH | a1 | B08 | b1 | C14 | c1 | D01 | F |
| 2-1-c1D01-1-0312 | 637.108 | OH | a2 | B19 | b3 | C08 | c1 | D01 | F |
| 2-1-c1D01-1-0313 | 638.094 | OH | a1 | B10 | b2 | C03 | c1 | D01 | F |
| 2-1-c1D01-1-0314 | 638.112 | OH | a2 | B13 | b3 | C16 | c1 | D01 | F |
| 2-1-c1D01-1-0315 | 639.109 | OH | a1 | B09 | b2 | C04 | c1 | D01 | F |
| 2-1-c1D01-1-0316 | 639.126 | OH | a2 | B13 | b3 | C17 | c1 | D01 | F |
| 2-1-c1D01-1-0317 | 640.104 | OH | a2 | B11 | b3 | C18 | c1 | D01 | F |
| 2-1-c1D01-1-0318 | 640.179 | OH | a2 | B12 | b3 | C18 | c1 | D01 | F |
| 2-1-c1D01-1-0319 | 641.040 | OH | a2 | B14 | b3 | C16 | c1 | D01 | F |
| 2-1-c1D01-1-0320 | 641.099 | OH | a2 | B19 | b1 | C09 | c1 | D01 | F |
| 2-1-c1D01-1-0321 | 641.109 | OH | a2 | B20 | b1 | C13 | c1 | D01 | F |
| 2-1-c1D01-1-0322 | 641.118 | OH | a1 | B08 | b2 | C07 | c1 | D01 | F |
| 2-1-c1D01-1-0323 | 642.069 | OH | a1 | B10 | b2 | C06 | c1 | D01 | F |
| 2-1-c1D01-1-0324 | 642.104 | OH | a1 | B01 | b3 | C16 | c1 | D01 | F |
| 2-1-c1D01-1-0325 | 643.122 | OH | a2 | B19 | b3 | C06 | c1 | D01 | F |
| 2-1-c1D01-1-0326 | 643.129 | OH | a2 | B20 | b3 | C15 | c1 | D01 | F |
| 2-1-c1D01-1-0327 | 643.134 | OH | a2 | B14 | b3 | C17 | c1 | D01 | F |
| 2-1-c1D01-1-0328 | 643.190 | OH | a1 | B02 | b3 | C16 | c1 | D01 | F |
| 2-1-c1D01-1-0329 | 644.138 | OH | a1 | B01 | b3 | C17 | c1 | D01 | F |
| 2-1-c1D01-1-0330 | 644.210 | OH | a1 | B09 | b1 | C13 | c1 | D01 | F |
| 2-1-c1D01-1-0331 | 645.083 | OH | a1 | B02 | b3 | C17 | c1 | D01 | F |
| 2-1-c1D01-1-0332 | 645.084 | OH | a1 | B08 | b3 | C08 | c1 | D01 | F |
| 2-1-c1D01-1-0333 | 645.138 | OH | a2 | B20 | b1 | C14 | c1 | D01 | F |
| 2-1-c1D01-1-0334 | 647.094 | OH | a1 | B09 | b3 | C15 | c1 | D01 | F |
| 2-1-c1D01-1-0335 | 648.090 | OH | a1 | B09 | b1 | C14 | c1 | D01 | F |
| 2-1-c1D01-1-0336 | 649.085 | OH | a1 | B08 | b1 | C09 | c1 | D01 | F |
| 2-1-c1D01-1-0337 | 650.125 | OH | a2 | B20 | b2 | C07 | c1 | D01 | F |
| 2-1-c1D01-1-0338 | 650.185 | OH | a1 | B08 | b3 | C06 | c1 | D01 | F |
| 2-1-c1D01-1-0339 | 652.110 | OH | a1 | B10 | b2 | C05 | c1 | D01 | F |
| 2-1-c1D01-1-0340 | 652.160 | OH | a2 | B15 | b3 | C16 | c1 | D01 | F |
| 2-1-c1D01-1-0341 | 653.163 | OH | a1 | B09 | b2 | C07 | c1 | D01 | F |
| 2-1-c1D01-1-0342 | 654.103 | OH | a2 | B20 | b3 | C08 | c1 | D01 | F |
| 2-1-c1D01-1-0343 | 655.055 | OH | a2 | B15 | b3 | C17 | c1 | D01 | F |
| 2-1-c1D01-1-0344 | 655.084 | OH | a2 | B16 | b3 | C16 | c1 | D01 | F |
| 2-1-c1D01-1-0345 | 655.098 | OH | a2 | B13 | b3 | C18 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0346 | 655.104 | OH | a1 | B10 | b2 | C04 | c1 | D01 | F |
| 2-1-c1D01-1-0347 | 655.142 | OH | a2 | B16 | b3 | C17 | c1 | D01 | F |
| 2-1-c1D01-1-0348 | 656.120 | OH | a1 | B09 | b3 | C08 | c1 | D01 | F |
| 2-1-c1D01-1-0349 | 657.094 | OH | a1 | B03 | b3 | C16 | c1 | D01 | F |
| 2-1-c1D01-1-0350 | 658.115 | OH | a2 | B20 | b1 | C09 | c1 | D01 | F |
| 2-1-c1D01-1-0351 | 658.226 | OH | a2 | B14 | b3 | C18 | c1 | D01 | F |
| 2-1-c1D01-1-0352 | 659.109 | OH | a1 | B03 | b3 | C17 | c1 | D01 | F |
| 2-1-c1D01-1-0353 | 659.185 | OH | a2 | B20 | b3 | C06 | c1 | D01 | F |
| 2-1-c1D01-1-0354 | 659.210 | OH | a1 | B01 | b3 | C18 | c1 | D01 | F |
| 2-1-c1D01-1-0355 | 660.060 | OH | a2 | B17 | b3 | C16 | c1 | D01 | F |
| 2-1-c1D01-1-0356 | 660.205 | OH | a1 | B02 | b3 | C18 | c1 | D01 | F |
| 2-1-c1D01-1-0357 | 661.044 | OH | a1 | B04 | b3 | C16 | c1 | D01 | F |
| 2-1-c1D01-1-0358 | 661.060 | OH | a1 | B09 | b1 | C09 | c1 | D01 | F |
| 2-1-c1D01-1-0359 | 661.110 | OH | a1 | B10 | b1 | C13 | c1 | D01 | F |
| 2-1-c1D01-1-0360 | 661.113 | OH | a2 | B17 | b3 | C17 | c1 | D01 | F |
| 2-1-c1D01-1-0361 | 662.055 | OH | a1 | B09 | b3 | C06 | c1 | D01 | F |
| 2-1-c1D01-1-0362 | 662.059 | OH | a1 | B10 | b3 | C15 | c1 | D01 | F |
| 2-1-c1D01-1-0363 | 662.105 | OH | a1 | B04 | b3 | C17 | c1 | D01 | F |
| 2-1-c1D01-1-0364 | 664.090 | OH | a1 | B10 | b1 | C14 | c1 | D01 | F |
| 2-1-c1D01-1-0365 | 668.175 | OH | a2 | B15 | b3 | C18 | c1 | D01 | F |
| 2-1-c1D01-1-0366 | 669.119 | OH | a1 | B10 | b2 | C07 | c1 | D01 | F |
| 2-1-c1D01-1-0367 | 670.205 | OH | a2 | B16 | b3 | C18 | c1 | D01 | F |
| 2-1-c1D01-1-0368 | 672.094 | OH | a1 | B05 | b3 | C16 | c1 | D01 | F |
| 2-1-c1D01-1-0369 | 673.078 | OH | a1 | B10 | b3 | C08 | c1 | D01 | F |
| 2-1-c1D01-1-0370 | 673.143 | OH | a1 | B05 | b3 | C17 | c1 | D01 | F |
| 2-1-c1D01-1-0371 | 674.061 | OH | a1 | B06 | b3 | C16 | c1 | D01 | F |
| 2-1-c1D01-1-0372 | 674.131 | OH | a1 | B03 | b3 | C18 | c1 | D01 | F |
| 2-1-c1D01-1-0373 | 674.148 | OH | a2 | B18 | b3 | C16 | c1 | D01 | F |
| 2-1-c1D01-1-0374 | 675.126 | OH | a1 | B06 | b3 | C17 | c1 | D01 | F |
| 2-1-c1D01-1-0375 | 676.089 | OH | a2 | B18 | b3 | C17 | c1 | D01 | F |
| 2-1-c1D01-1-0376 | 676.105 | OH | a2 | B17 | b3 | C18 | c1 | D01 | F |
| 2-1-c1D01-1-0377 | 676.110 | OH | a1 | B10 | b1 | C09 | c1 | D01 | F |
| 2-1-c1D01-1-0378 | 677.021 | OH | a1 | B04 | b3 | C18 | c1 | D01 | F |
| 2-1-c1D01-1-0379 | 678.036 | OH | a1 | B10 | b3 | C06 | c1 | D01 | F |
| 2-1-c1D01-1-0380 | 678.085 | OH | a1 | B07 | b3 | C16 | c1 | D01 | F |
| 2-1-c1D01-1-0381 | 680.049 | OH | a1 | B07 | b3 | C17 | c1 | D01 | F |
| 2-1-c1D01-1-0382 | 688.125 | OH | a1 | B05 | b3 | C18 | c1 | D01 | F |

(continued)

| [Table 8-5-1-2] Compound that may be contained in mixture 2-1-c1D01-1 | | | | | | | | | |
|---|---|---|---|---|---|---|---|---|---|
| ID | Exact Mass | | a | B | b | C | c | D | d |
| 2-1-c1D01-1-0383 | 690.100 | OH | a1 | B06 | b3 | C18 | c1 | D01 | F |
| 2-1-c1D01-1-0384 | 692.051 | OH | a2 | B18 | b3 | C18 | c1 | D01 | F |
| 2-1-c1D01-1-0385 | 692.100 | OH | a2 | B19 | b3 | C16 | c1 | D01 | F |
| 2-1-c1D01-1-0386 | 693.080 | OH | a2 | B19 | b3 | C17 | c1 | D01 | F |
| 2-1-c1D01-1-0387 | 695.081 | OH | a1 | B07 | b3 | C18 | c1 | D01 | F |
| 2-1-c1D01-1-0388 | 700.161 | OH | a1 | B08 | b3 | C16 | c1 | D01 | F |
| 2-1-c1D01-1-0389 | 702.104 | OH | a1 | B08 | b3 | C17 | c1 | D01 | F |
| 2-1-c1D01-1-0390 | 709.095 | OH | a2 | B20 | b3 | C16 | c1 | D01 | F |
| 2-1-c1D01-1-0391 | 710.061 | OH | a2 | B19 | b3 | C18 | c1 | D01 | F |
| 2-1-c1D01-1-0392 | 711.056 | OH | a2 | B20 | b3 | C17 | c1 | D01 | F |
| 2-1-c1D01-1-0393 | 712.056 | OH | a1 | B09 | b3 | C16 | c1 | D01 | F |
| 2-1-c1D01-1-0394 | 712.125 | OH | a1 | B09 | b3 | C17 | c1 | D01 | F |
| 2-1-c1D01-1-0395 | 717.125 | OH | a1 | B08 | b3 | C18 | c1 | D01 | F |
| 2-1-c1D01-1-0396 | 726.087 | OH | a2 | B20 | b3 | C18 | c1 | D01 | F |
| 2-1-c1D01-1-0397 | 728.120 | OH | a1 | B10 | b3 | C16 | c1 | D01 | F |
| 2-1-c1D01-1-0398 | 729.098 | OH | a1 | B09 | b3 | C18 | c1 | D01 | F |
| 2-1-c1D01-1-0399 | 730.082 | OH | a1 | B10 | b3 | C17 | c1 | D01 | F |
| 2-1-c1D01-1-0400 | 745.092 | OH | a1 | B10 | b3 | C18 | c1 | D01 | F |

Example 8-5-2: Synthesis of 2-1-c2D02-0 by step c2D02

A reaction formula in step c2D02 performed using mixture 2-1-c1D00-0 will be illustrated below.

[Formula 630]

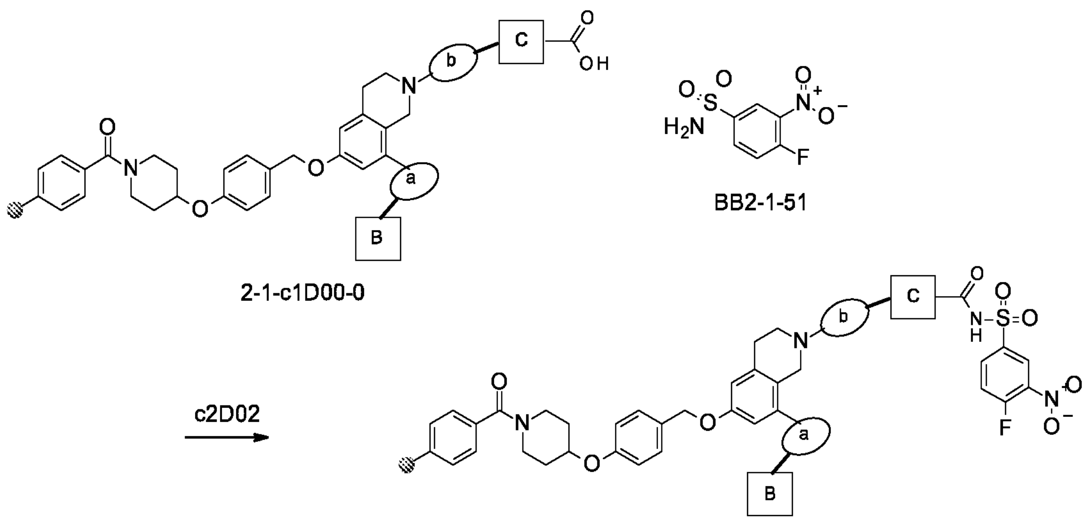


2-1-c1D00-0

BB2-1-51

2-1-c2D02-0

Mixture 2-1-c2D02-0 was synthesized as follows using mixture 2-1-h02C08-0 to mixture 2-1-h21C18-0.

20 types of mixtures described in Table 8-5-2-1 and NMP (10.5 mL) were added to a 20 mL column with a filter and shaken at room temperature for 1 hour. 2,6-Lutidine (0.094 mL, 0.811 mmol) and HATU (0.308 g, 0.811 mmol) were added thereto, and the mixture was shaken at 40°C for 18 hours. 4-Fluoro-3-nitrobenzenesulfonamide (BB2-1-51, 0.208 g, 0.946 mmol) and PltBu (0.516 mL, 2.03 mmol) were added thereto, and the mixture was shaken at room temperature for 24 hours.

Solid-phase purification

The suspension of the reaction solution and the resin was filtered through the filter of the column and washed three times with NMP/water = 1/1 (14 mL), three times with NMP (14 mL), three times with 0.05 M $TBAHSO_4$ + DTBP in NMP (14 mL), three times with NMP/water = 1/1 (14 mL), three times with NMP (14 mL), three times with MeOH (14 mL), three times with DCM (14 mL), and three times with heptane (14 mL), and the obtained solid phase was dried under reduced pressure to obtain mixture 2-1-c2D02-0.

[Table 374]

| [Table 8-5-2-1] Starting material mixture used in step c2D01 | |
|---|---|
| Mixture | Amount used |
| 2-1-h02C08-0 | 35mg |
| 2-1-h03C09-0 | 35mg |
| 2-1-h04C10-0 | 35mg |
| 2-1-h05C11-0 | 35mg |
| 2-1-h06C12-0 | 35mg |
| 2-1-h07C13-0 | 35mg |
| 2-1-h08C14-0 | 35mg |
| 2-1-h09C01-0 | 35mg |
| 2-1-h10C02-0 | 35mg |
| 2-1-h11C03-0 | 35mg |
| 2-1-h12C04-0 | 35mg |
| 2-1-h13C05-0 | 35mg |
| 2-1-h14C06-0 | 35mg |
| 2-1-h15C07-0 | 35mg |
| 2-1-h16C06-0 | 35mg |
| 2-1-h17C08-0 | 35mg |
| 2-1-h18C15-0 | 35mg |
| 2-1-h19C16-0 | 35mg |
| 2-1-h20C17-0 | 35mg |
| 2-1-h21C18-0 | 35mg |

Mixture 2-1-c2D02-0 can be treated by cleavage in the same manner as in Example 8-1-1 to obtain mixture 2-1-c2D02-1. The details of the mixture are shown in Table 8-5-2-2.

A notation method in each table will be described. In Table 8-5-2-2, each compound that may be contained in mixture 2-1-c2D02-1 is indicated by ID, and core blocks and linkers in combination in the structure of mixture 2-1-c2D02-1 are indicated by symbols or chemical formulas. The correspondence between the symbols and specific structures are shown in Figure 4. In Table 8-5-2-2, "×" and "d" are represented by chemical formulas.

[Formula 631]

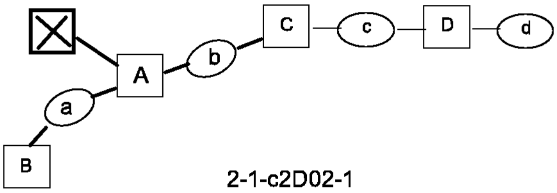

2-1-c2D02-1

For example, when ID is 2-1-c2D02-1-0001, the compound is represented by the following structural formula.

[Formula 632]

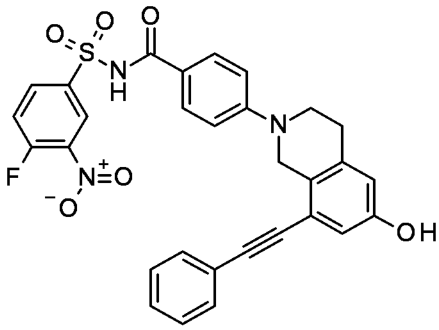

2-1-c2D02-1-0001

[Table 375]

| Notation in [Table 8-5-2-2] | | | | | | | | |
|---|---|---|---|---|---|---|---|---|
| ID | | a | B | b | C | c | D | d |
| 2-1-c2D02-1-0001 | OH | a2 | B11 | b1 | C08 | c2 | D02 | F |

[Table 376]

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0001 | 571.121 | OH | a2 | B11 | b1 | C08 | c2 | D02 | F |
| 2-1-c2D02-1-0002 | 572.117 | OH | a2 | B12 | b1 | C08 | c2 | D02 | F |
| 2-1-c2D02-1-0003 | 572.117 | OH | a2 | B11 | b1 | C11 | c2 | D02 | F |
| 2-1-c2D02-1-0004 | 572.151 | OH | a2 | B11 | b1 | C10 | c2 | D02 | F |
| 2-1-c2D02-1-0005 | 572.151 | OH | a2 | B12 | b1 | C11 | c2 | D02 | F |
| 2-1-c2D02-1-0006 | 573.112 | OH | a2 | B12 | b1 | C10 | c2 | D02 | F |
| 2-1-c2D02-1-0007 | 577.147 | OH | a2 | B11 | b1 | C12 | c2 | D02 | F |
| 2-1-c2D02-1-0008 | 578.073 | OH | a2 | B12 | b1 | C12 | c2 | D02 | F |
| 2-1-c2D02-1-0009 | 584.153 | OH | a2 | B13 | b1 | C08 | c2 | D02 | F |
| 2-1-c2D02-1-0010 | 585.137 | OH | a2 | B13 | b1 | C11 | c2 | D02 | F |
| 2-1-c2D02-1-0011 | 586.132 | OH | a2 | B13 | b1 | C10 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0012 | 587.162 | OH | a2 | B11 | b2 | C01 | c2 | D02 | F |
| 2-1-c2D02-1-0013 | 587.218 | OH | a2 | B14 | b1 | C08 | c2 | D02 | F |
| 2-1-c2D02-1-0014 | 589.112 | OH | a2 | B12 | b2 | C01 | c2 | D02 | F |
| 2-1-c2D02-1-0015 | 589.126 | OH | a2 | B14 | b1 | C11 | c2 | D02 | F |
| 2-1-c2D02-1-0016 | 589.176 | OH | a1 | B01 | b1 | C08 | c2 | D02 | F |
| 2-1-c2D02-1-0017 | 590.107 | OH | a2 | B14 | b1 | C10 | c2 | D02 | F |
| 2-1-c2D02-1-0018 | 590.127 | OH | a1 | B02 | b1 | C08 | c2 | D02 | F |
| 2-1-c2D02-1-0019 | 590.127 | OH | a1 | B01 | b1 | C11 | c2 | D02 | F |
| 2-1-c2D02-1-0020 | 591.111 | OH | a2 | B13 | b1 | C12 | c2 | D02 | F |
| 2-1-c2D02-1-0021 | 591.122 | OH | a1 | B01 | b1 | C10 | c2 | D02 | F |
| 2-1-c2D02-1-0022 | 591.122 | OH | a1 | B02 | b1 | C11 | c2 | D02 | F |
| 2-1-c2D02-1-0023 | 591.122 | OH | a1 | B02 | b1 | C10 | c2 | D02 | F |
| 2-1-c2D02-1-0024 | 593.132 | OH | a2 | B14 | b1 | C12 | c2 | D02 | F |
| 2-1-c2D02-1-0025 | 595.147 | OH | a1 | B01 | b1 | C12 | c2 | D02 | F |
| 2-1-c2D02-1-0026 | 596.098 | OH | a1 | B02 | b1 | C12 | c2 | D02 | F |
| 2-1-c2D02-1-0027 | 597.079 | OH | a2 | B11 | b2 | C02 | c2 | D02 | F |
| 2-1-c2D02-1-0028 | 597.173 | OH | a2 | B15 | b1 | C08 | c2 | D02 | F |
| 2-1-c2D02-1-0029 | 598.074 | OH | a2 | B12 | b2 | C02 | c2 | D02 | F |
| 2-1-c2D02-1-0030 | 598.093 | OH | a2 | B11 | b2 | C03 | c2 | D02 | F |
| 2-1-c2D02-1-0031 | 598.169 | OH | a2 | B15 | b1 | C11 | c2 | D02 | F |
| 2-1-c2D02-1-0032 | 600.111 | OH | a2 | B12 | b2 | C03 | c2 | D02 | F |
| 2-1-c2D02-1-0033 | 600.128 | OH | a2 | B16 | b1 | C08 | c2 | D02 | F |
| 2-1-c2D02-1-0034 | 600.148 | OH | a2 | B15 | b1 | C10 | c2 | D02 | F |
| 2-1-c2D02-1-0035 | 600.149 | OH | a2 | B16 | b1 | C11 | c2 | D02 | F |
| 2-1-c2D02-1-0036 | 600.238 | OH | a2 | B13 | b2 | C01 | c2 | D02 | F |
| 2-1-c2D02-1-0037 | 601.104 | OH | a2 | B16 | b1 | C10 | c2 | D02 | F |
| 2-1-c2D02-1-0038 | 602.161 | OH | a1 | B03 | b1 | C08 | c2 | D02 | F |
| 2-1-c2D02-1-0039 | 603.122 | OH | a2 | B11 | b2 | C06 | c2 | D02 | F |
| 2-1-c2D02-1-0040 | 603.128 | OH | a1 | B03 | b1 | C11 | c2 | D02 | F |
| 2-1-c2D02-1-0041 | 603.198 | OH | a2 | B12 | b2 | C06 | c2 | D02 | F |
| 2-1-c2D02-1-0042 | 604.137 | OH | a2 | B15 | b1 | C12 | c2 | D02 | F |
| 2-1-c2D02-1-0043 | 604.143 | OH | a1 | B03 | b1 | C10 | c2 | D02 | F |
| 2-1-c2D02-1-0044 | 604.143 | OH | a2 | B14 | b2 | C01 | c2 | D02 | F |
| 2-1-c2D02-1-0045 | 605.109 | OH | a2 | B17 | b1 | C08 | c2 | D02 | F |
| 2-1-c2D02-1-0046 | 605.138 | OH | a2 | B16 | b1 | C12 | c2 | D02 | F |
| 2-1-c2D02-1-0047 | 605.138 | OH | a2 | B17 | b1 | C11 | c2 | D02 | F |
| 2-1-c2D02-1-0048 | 605.138 | OH | a1 | B01 | b2 | C01 | c2 | D02 | F |
| 2-1-c2D02-1-0049 | 605.157 | OH | a1 | B04 | b1 | C08 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0050 | 607.086 | OH | a2 | B17 | b1 | C10 | c2 | D02 | F |
| 2-1-c2D02-1-0051 | 607.088 | OH | a1 | B02 | b2 | C01 | c2 | D02 | F |
| 2-1-c2D02-1-0052 | 607.102 | OH | a1 | B04 | b1 | C11 | c2 | D02 | F |
| 2-1-c2D02-1-0053 | 608.098 | OH | a1 | B04 | b1 | C10 | c2 | D02 | F |
| 2-1-c2D02-1-0054 | 608.118 | OH | a1 | B03 | b1 | C12 | c2 | D02 | F |
| 2-1-c2D02-1-0055 | 610.117 | OH | a2 | B13 | b2 | C02 | c2 | D02 | F |
| 2-1-c2D02-1-0056 | 611.127 | OH | a2 | B17 | b1 | C12 | c2 | D02 | F |
| 2-1-c2D02-1-0057 | 611.143 | OH | a2 | B13 | b2 | C03 | c2 | D02 | F |
| 2-1-c2D02-1-0058 | 611.189 | OH | a2 | B11 | b2 | C05 | c2 | D02 | F |
| 2-1-c2D02-1-0059 | 612.127 | OH | a1 | B04 | b1 | C12 | c2 | D02 | F |
| 2-1-c2D02-1-0060 | 612.138 | OH | a2 | B12 | b2 | C05 | c2 | D02 | F |
| 2-1-c2D02-1-0061 | 614.074 | OH | a2 | B14 | b2 | C02 | c2 | D02 | F |
| 2-1-c2D02-1-0062 | 614.127 | OH | a2 | B11 | b2 | C04 | c2 | D02 | F |
| 2-1-c2D02-1-0063 | 614.127 | OH | a2 | B15 | b2 | C01 | c2 | D02 | F |
| 2-1-c2D02-1-0064 | 615.148 | OH | a2 | B12 | b2 | C04 | c2 | D02 | F |
| 2-1-c2D02-1-0065 | 615.148 | OH | a2 | B14 | b2 | C03 | c2 | D02 | F |
| 2-1-c2D02-1-0066 | 616.088 | OH | a1 | B01 | b2 | C02 | c2 | D02 | F |
| 2-1-c2D02-1-0067 | 617.084 | OH | a1 | B05 | b1 | C08 | c2 | D02 | F |
| 2-1-c2D02-1-0068 | 617.107 | OH | a2 | B13 | b2 | C06 | c2 | D02 | F |
| 2-1-c2D02-1-0069 | 617.127 | OH | a2 | B16 | b2 | C01 | c2 | D02 | F |
| 2-1-c2D02-1-0070 | 617.143 | OH | a1 | B02 | b2 | C02 | c2 | D02 | F |
| 2-1-c2D02-1-0071 | 617.174 | OH | a1 | B01 | b2 | C03 | c2 | D02 | F |
| 2-1-c2D02-1-0072 | 618.102 | OH | a1 | B05 | b1 | C11 | c2 | D02 | F |
| 2-1-c2D02-1-0073 | 618.140 | OH | a1 | B02 | b2 | C03 | c2 | D02 | F |
| 2-1-c2D02-1-0074 | 618.158 | OH | a1 | B06 | b1 | C08 | c2 | D02 | F |
| 2-1-c2D02-1-0075 | 618.158 | OH | a1 | B05 | b1 | C10 | c2 | D02 | F |
| 2-1-c2D02-1-0076 | 619.117 | OH | a1 | B06 | b1 | C11 | c2 | D02 | F |
| 2-1-c2D02-1-0077 | 619.122 | OH | a2 | B18 | b1 | C08 | c2 | D02 | F |
| 2-1-c2D02-1-0078 | 619.139 | OH | a1 | B03 | b2 | C01 | c2 | D02 | F |
| 2-1-c2D02-1-0079 | 619.154 | OH | a1 | B06 | b1 | C10 | c2 | D02 | F |
| 2-1-c2D02-1-0080 | 619.154 | OH | a2 | B18 | b1 | C11 | c2 | D02 | F |
| 2-1-c2D02-1-0081 | 620.138 | OH | a2 | B14 | b2 | C06 | c2 | D02 | F |
| 2-1-c2D02-1-0082 | 620.149 | OH | a2 | B18 | b1 | C10 | c2 | D02 | F |
| 2-1-c2D02-1-0083 | 620.154 | OH | a2 | B11 | b1 | C13 | c2 | D02 | F |
| 2-1-c2D02-1-0084 | 621.133 | OH | a1 | B01 | b2 | C06 | c2 | D02 | F |
| 2-1-c2D02-1-0085 | 621.137 | OH | a2 | B12 | b1 | C13 | c2 | D02 | F |
| 2-1-c2D02-1-0086 | 621.137 | OH | a2 | B11 | b3 | C15 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0087 | 622.117 | OH | a1 | B02 | b2 | C06 | c2 | D02 | F |
| 2-1-c2D02-1-0088 | 622.128 | OH | a2 | B17 | b2 | C01 | c2 | D02 | F |
| 2-1-c2D02-1-0089 | 622.132 | OH | a1 | B05 | b1 | C12 | c2 | D02 | F |
| 2-1-c2D02-1-0090 | 622.204 | OH | a2 | B12 | b3 | C15 | c2 | D02 | F |
| 2-1-c2D02-1-0091 | 623.063 | OH | a1 | B04 | b2 | C01 | c2 | D02 | F |
| 2-1-c2D02-1-0092 | 623.143 | OH | a1 | B07 | b1 | C08 | c2 | D02 | F |
| 2-1-c2D02-1-0093 | 624.123 | OH | a1 | B06 | b1 | C12 | c2 | D02 | F |
| 2-1-c2D02-1-0094 | 625.074 | OH | a2 | B11 | b1 | C14 | c2 | D02 | F |
| 2-1-c2D02-1-0095 | 625.077 | OH | a1 | B07 | b1 | C11 | c2 | D02 | F |
| 2-1-c2D02-1-0096 | 625.093 | OH | a2 | B15 | b2 | C02 | c2 | D02 | F |
| 2-1-c2D02-1-0097 | 625.205 | OH | a2 | B18 | b1 | C12 | c2 | D02 | F |
| 2-1-c2D02-1-0098 | 625.205 | OH | a2 | B12 | b1 | C14 | c2 | D02 | F |
| 2-1-c2D02-1-0099 | 626.058 | OH | a1 | B07 | b1 | C10 | c2 | D02 | F |
| 2-1-c2D02-1-0100 | 626.108 | OH | a2 | B15 | b2 | C03 | c2 | D02 | F |
| 2-1-c2D02-1-0101 | 626.108 | OH | a2 | B13 | b2 | C05 | c2 | D02 | F |
| 2-1-c2D02-1-0102 | 626.127 | OH | a2 | B16 | b2 | C02 | c2 | D02 | F |
| 2-1-c2D02-1-0103 | 627.093 | OH | a2 | B16 | b2 | C03 | c2 | D02 | F |
| 2-1-c2D02-1-0104 | 627.184 | OH | a2 | B13 | b2 | C04 | c2 | D02 | F |
| 2-1-c2D02-1-0105 | 628.089 | OH | a2 | B11 | b2 | C07 | c2 | D02 | F |
| 2-1-c2D02-1-0106 | 628.122 | OH | a2 | B14 | b2 | C05 | c2 | D02 | F |
| 2-1-c2D02-1-0107 | 628.143 | OH | a1 | B03 | b2 | C02 | c2 | D02 | F |
| 2-1-c2D02-1-0108 | 628.143 | OH | a2 | B12 | b2 | C07 | c2 | D02 | F |
| 2-1-c2D02-1-0109 | 629.163 | OH | a1 | B07 | b1 | C12 | c2 | D02 | F |
| 2-1-c2D02-1-0110 | 629.180 | OH | a2 | B15 | b2 | C06 | c2 | D02 | F |
| 2-1-c2D02-1-0111 | 630.122 | OH | a1 | B03 | b2 | C03 | c2 | D02 | F |
| 2-1-c2D02-1-0112 | 630.134 | OH | a1 | B01 | b2 | C05 | c2 | D02 | F |
| 2-1-c2D02-1-0113 | 631.140 | OH | a1 | B02 | b2 | C05 | c2 | D02 | F |
| 2-1-c2D02-1-0114 | 632.118 | OH | a2 | B16 | b2 | C06 | c2 | D02 | F |
| 2-1-c2D02-1-0115 | 632.138 | OH | a2 | B11 | b3 | C08 | c2 | D02 | F |
| 2-1-c2D02-1-0116 | 632.174 | OH | a2 | B17 | b2 | C02 | c2 | D02 | F |
| 2-1-c2D02-1-0117 | 632.174 | OH | a2 | B14 | b2 | C04 | c2 | D02 | F |
| 2-1-c2D02-1-0118 | 633.173 | OH | a2 | B12 | b3 | C08 | c2 | D02 | F |
| 2-1-c2DO2-1-0119 | 634.128 | OH | a2 | B17 | b2 | C03 | c2 | D02 | F |
| 2-1-c2D02-1-0120 | 634.148 | OH | a1 | B04 | b2 | C02 | c2 | D02 | F |
| 2-1-c2D02-1-0121 | 634.153 | OH | a1 | B01 | b2 | C04 | c2 | D02 | F |
| 2-1-c2D02-1-0122 | 634.153 | OH | a1 | B05 | b2 | C01 | c2 | D02 | F |
| 2-1-c2D02-1-0123 | 635.097 | OH | a1 | B02 | b2 | C04 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0124 | 635.139 | OH | a1 | B04 | b2 | C03 | c2 | D02 | F |
| 2-1-c2D02-1-0125 | 635.204 | OH | a2 | B13 | b1 | C13 | c2 | D02 | F |
| 2-1-c2D02-1-0126 | 636.093 | OH | a1 | B03 | b2 | C06 | c2 | D02 | F |
| 2-1-c2D02-1-0127 | 636.113 | OH | a1 | B06 | b2 | C01 | c2 | D02 | F |
| 2-1-c2D02-1-0128 | 636.149 | OH | a2 | B13 | b3 | C15 | c2 | D02 | F |
| 2-1-c2D02-1-0129 | 637.108 | OH | a2 | B19 | b1 | C08 | c2 | D02 | F |
| 2-1-c2D02-1-0130 | 637.113 | OH | a2 | B11 | b1 | C09 | c2 | D02 | F |
| 2-1-c2D02-1-0131 | 637.143 | OH | a2 | B18 | b2 | C01 | c2 | D02 | F |
| 2-1-c2D02-1-0132 | 638.142 | OH | a2 | B12 | b1 | C09 | c2 | D02 | F |
| 2-1-c2D02-1-0133 | 638.145 | OH | a2 | B19 | b1 | C11 | c2 | D02 | F |
| 2-1-c2D02-1-0134 | 639.109 | OH | a2 | B11 | b3 | C06 | c2 | D02 | F |
| 2-1-c2D02-1-0135 | 639.109 | OH | a2 | B17 | b2 | C06 | c2 | D02 | F |
| 2-1-c2D02-1-0136 | 639.111 | OH | a2 | B19 | b1 | C10 | c2 | D02 | F |
| 2-1-c2D02-1-0137 | 639.128 | OH | a2 | B13 | b1 | C14 | c2 | D02 | F |
| 2-1-c2D02-1-0138 | 639.138 | OH | a2 | B14 | b1 | C13 | c2 | D02 | F |
| 2-1-c2D02-1-0139 | 639.220 | OH | a2 | B12 | b3 | C06 | c2 | D02 | F |
| 2-1-c2D02-1-0140 | 640.104 | OH | a1 | B04 | b2 | C06 | c2 | D02 | F |
| 2-1-c2D02-1-0141 | 640.104 | OH | a1 | B01 | b1 | C13 | c2 | D02 | F |
| 2-1-c2D02-1-0142 | 640.124 | OH | a2 | B15 | b2 | C05 | c2 | D02 | F |
| 2-1-c2D02-1-0143 | 640.138 | OH | a2 | B14 | b3 | C15 | c2 | D02 | F |
| 2-1-c2D02-1-0144 | 641.054 | OH | a1 | B02 | b1 | C13 | c2 | D02 | F |
| 2-1-c2D02-1-0145 | 641.200 | OH | a1 | B01 | b3 | C15 | c2 | D02 | F |
| 2-1-c2D02-1-0146 | 642.035 | OH | a1 | B07 | b2 | C01 | c2 | D02 | F |
| 2-1-c2D02-1-0147 | 642.133 | OH | a2 | B16 | b2 | C05 | c2 | D02 | F |
| 2-1-c2D02-1-0148 | 643.100 | OH | a1 | B02 | b3 | C15 | c2 | D02 | F |
| 2-1-c2D02-1-0149 | 643.148 | OH | a2 | B14 | b1 | C14 | c2 | D02 | F |
| 2-1-c2D02-1-0150 | 644.068 | OH | a2 | B15 | b2 | C04 | c2 | D02 | F |
| 2-1-c2D02-1-0151 | 644.099 | OH | a2 | B13 | b2 | C07 | c2 | D02 | F |
| 2-1-c2D02-1-0152 | 644.099 | OH | a2 | B19 | b1 | C12 | c2 | D02 | F |
| 2-1-c2D02-1-0153 | 644.138 | OH | a1 | B01 | b1 | C14 | c2 | D02 | F |
| 2-1-c2D02-1-0154 | 644.210 | OH | a1 | B05 | b2 | C02 | c2 | D02 | F |
| 2-1-c2D02-1-0155 | 645.084 | OH | a1 | B08 | b1 | C08 | c2 | D02 | F |
| 2-1-c2D02-1-0156 | 645.133 | OH | a1 | B02 | b1 | C14 | c2 | D02 | F |
| 2-1-c2D02-1-0157 | 645.194 | OH | a2 | B16 | b2 | C04 | c2 | D02 | F |
| 2-1-c2D02-1-0158 | 646.099 | OH | a1 | B05 | b2 | C03 | c2 | D02 | F |
| 2-1-c2D02-1-0159 | 646.099 | OH | a1 | B03 | b2 | C05 | c2 | D02 | F |
| 2-1-c2D02-1-0160 | 646.190 | OH | a1 | B08 | b1 | C11 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0161 | 647.094 | OH | a1 | B06 | b2 | C02 | c2 | D02 | F |
| 2-1-c2D02-1-0162 | 647.114 | OH | a1 | B08 | b1 | C10 | c2 | D02 | F |
| 2-1-c2D02-1-0163 | 647.117 | OH | a2 | B13 | b3 | C08 | c2 | D02 | F |
| 2-1-c2D02-1-0164 | 647.135 | OH | a1 | B06 | b2 | C03 | c2 | D02 | F |
| 2-1-c2D02-1-0165 | 647.149 | OH | a2 | B18 | b2 | C02 | c2 | D02 | F |
| 2-1-c2D02-1-0166 | 647.170 | OH | a2 | B14 | b2 | C07 | c2 | D02 | F |
| 2-1-c2D02-1-0167 | 647.189 | OH | a2 | B17 | b2 | C05 | c2 | D02 | F |
| 2-1-c2D02-1-0168 | 648.090 | OH | a2 | B18 | b2 | C03 | c2 | D02 | F |
| 2-1-c2D02-1-0169 | 648.094 | OH | a1 | B03 | b2 | C04 | c2 | D02 | F |
| 2-1-c2D02-1-0170 | 648.185 | OH | a1 | B01 | b2 | C07 | c2 | D02 | F |
| 2-1-c2D02-1-0171 | 649.099 | OH | a1 | B04 | b2 | C05 | c2 | D02 | F |
| 2-1-c2D02-1-0172 | 649.169 | OH | a2 | B15 | b1 | C13 | c2 | D02 | F |
| 2-1-c2D02-1-0173 | 650.108 | OH | a1 | B02 | b2 | C07 | c2 | D02 | F |
| 2-1-c2D02-1-0174 | 650.127 | OH | a1 | B05 | b2 | C06 | c2 | D02 | F |
| 2-1-c2D02-1-0175 | 651.120 | OH | a2 | B14 | b3 | C08 | c2 | D02 | F |
| 2-1-c2D02-1-0176 | 651.124 | OH | a2 | B17 | b2 | C04 | c2 | D02 | F |
| 2-1-c2D02-1-0177 | 651.145 | OH | a2 | B15 | b3 | C15 | c2 | D02 | F |
| 2-1-c2D02-1-0178 | 651.148 | OH | a2 | B13 | b1 | C09 | c2 | D02 | F |
| 2-1-c2D02-1-0179 | 651.180 | OH | a2 | B16 | b1 | C13 | c2 | D02 | F |
| 2-1-c2D02-1-0180 | 653.074 | OH | a1 | B08 | b1 | C12 | c2 | D02 | F |
| 2-1-c2D02-1-0181 | 653.088 | OH | a1 | B06 | b2 | C06 | c2 | D02 | F |
| 2-1-c2D02-1-0182 | 653.094 | OH | a1 | B01 | b3 | C08 | c2 | D02 | F |
| 2-1-c2D02-1-0183 | 653.124 | OH | a1 | B07 | b2 | C02 | c2 | D02 | F |
| 2-1-c2D02-1-0184 | 653.124 | OH | a1 | B04 | b2 | C04 | c2 | D02 | F |
| 2-1-c2D02-1-0185 | 653.200 | OH | a2 | B13 | b3 | C06 | c2 | D02 | F |
| 2-1-c2D02-1-0186 | 654.046 | OH | a2 | B16 | b3 | C15 | c2 | D02 | F |
| 2-1-c2D02-1-0187 | 654.089 | OH | a1 | B02 | b3 | C08 | c2 | D02 | F |
| 2-1-c2D02-1-0188 | 654.089 | OH | a2 | B18 | b2 | C06 | c2 | D02 | F |
| 2-1-c2D02-1-0189 | 654.103 | OH | a1 | B07 | b2 | C03 | c2 | D02 | F |
| 2-1-c2D02-1-0190 | 654.210 | OH | a2 | B20 | b1 | C08 | c2 | D02 | F |
| 2-1-c2D02-1-0191 | 655.073 | OH | a2 | B15 | b1 | C14 | c2 | D02 | F |
| 2-1-c2D02-1-0192 | 655.088 | OH | a2 | B20 | b1 | C11 | c2 | D02 | F |
| 2-1-c2D02-1-0193 | 655.104 | OH | a1 | B03 | b1 | C13 | c2 | D02 | F |
| 2-1-c2D02-1-0194 | 655.104 | OH | a2 | B19 | b2 | C01 | c2 | D02 | F |
| 2-1-c2D02-1-0195 | 655.125 | OH | a2 | B20 | b1 | C10 | c2 | D02 | F |
| 2-1-c2D02-1-0196 | 655.125 | OH | a2 | B14 | b1 | C09 | c2 | D02 | F |
| 2-1-c2D02-1-0197 | 656.099 | OH | a2 | B16 | b1 | C14 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0198 | 656.119 | OH | a1 | B03 | b3 | C15 | c2 | D02 | F |
| 2-1-c2D02-1-0199 | 656.133 | OH | a1 | B09 | b1 | C08 | c2 | D02 | F |
| 2-1-c2D02-1-0200 | 656.149 | OH | a1 | B01 | b1 | C09 | c2 | D02 | F |
| 2-1-c2D02-1-0201 | 657.104 | OH | a2 | B14 | b3 | C06 | c2 | D02 | F |
| 2-1-c2D02-1-0202 | 657.104 | OH | a2 | B17 | b1 | C13 | c2 | D02 | F |
| 2-1-c2D02-1-0203 | 657.175 | OH | a1 | B02 | b1 | C09 | c2 | D02 | F |
| 2-1-c2D02-1-0204 | 658.084 | OH | a1 | B09 | b1 | C11 | c2 | D02 | F |
| 2-1-c2D02-1-0205 | 658.115 | OH | a2 | B15 | b2 | C07 | c2 | D02 | F |
| 2-1-c2D02-1-0206 | 658.153 | OH | a1 | B01 | b3 | C06 | c2 | D02 | F |
| 2-1-c2D02-1-0207 | 658.190 | OH | a1 | B07 | b2 | C06 | c2 | D02 | F |
| 2-1-c2D02-1-0208 | 659.030 | OH | a1 | B09 | b1 | C10 | c2 | D02 | F |
| 2-1-c2D02-1-0209 | 659.110 | OH | a1 | B03 | b1 | C14 | c2 | D02 | F |
| 2-1-c2D02-1-0210 | 659.110 | OH | a1 | B04 | b1 | C13 | c2 | D02 | F |
| 2-1-c2D02-1-0211 | 659.129 | OH | a1 | B02 | b3 | C06 | c2 | D02 | F |
| 2-1-c2D02-1-0212 | 659.131 | OH | a2 | B17 | b3 | C15 | c2 | D02 | F |
| 2-1-c2D02-1-0213 | 660.095 | OH | a1 | B05 | b2 | C05 | c2 | D02 | F |
| 2-1-c2D02-1-0214 | 660.105 | OH | a2 | B16 | b2 | C07 | c2 | D02 | F |
| 2-1-c2D02-1-0215 | 660.115 | OH | a2 | B20 | b1 | C12 | c2 | D02 | F |
| 2-1-c2D02-1-0216 | 660.124 | OH | a1 | B04 | b3 | C15 | c2 | D02 | F |
| 2-1-c2D02-1-0217 | 661.164 | OH | a2 | B17 | b1 | C14 | c2 | D02 | F |
| 2-1-c2D02-1-0218 | 661.189 | OH | a2 | B15 | b3 | C08 | c2 | D02 | F |
| 2-1-c2D02-1-0219 | 661.205 | OH | a1 | B06 | b2 | C05 | c2 | D02 | F |
| 2-1-c2D02-1-0220 | 662.128 | OH | a1 | B04 | b1 | C14 | c2 | D02 | F |
| 2-1-c2D02-1-0221 | 662.140 | OH | a2 | B18 | b2 | C05 | c2 | D02 | F |
| 2-1-c2D02-1-0222 | 662.154 | OH | a1 | B05 | b2 | C04 | c2 | D02 | F |
| 2-1-c2D02-1-0223 | 663.075 | OH | a1 | B03 | b2 | C07 | c2 | D02 | F |
| 2-1-c2D02-1-0224 | 663.115 | OH | a1 | B09 | b1 | C12 | c2 | D02 | F |
| 2-1-c2D02-1-0225 | 663.144 | OH | a1 | B08 | b2 | C01 | c2 | D02 | F |
| 2-1-c2D02-1-0226 | 664.071 | OH | a2 | B16 | b3 | C08 | c2 | D02 | F |
| 2-1-c2D02-1-0227 | 664.143 | OH | a1 | B06 | b2 | C04 | c2 | D02 | F |
| 2-1-c2D02-1-0228 | 665.066 | OH | a2 | B19 | b2 | C02 | c2 | D02 | F |
| 2-1-c2D02-1-0229 | 665.069 | OH | a2 | B18 | b2 | C04 | c2 | D02 | F |
| 2-1-c2D02-1-0230 | 665.085 | OH | a2 | B15 | b1 | C09 | c2 | D02 | F |
| 2-1-c2D02-1-0231 | 666.120 | OH | a2 | B17 | b2 | C07 | c2 | D02 | F |
| 2-1-c2D02-1-0232 | 666.141 | OH | a2 | B19 | b2 | C03 | c2 | D02 | F |
| 2-1-c2D02-1-0233 | 666.176 | OH | a1 | B03 | b3 | C08 | c2 | D02 | F |
| 2-1-c2D02-1-0234 | 667.104 | OH | a1 | B04 | b2 | C07 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0235 | 667.122 | OH | a2 | B15 | b3 | C06 | c2 | D02 | F |
| 2-1-c2D02-1-0236 | 667.140 | OH | a1 | B07 | b2 | C05 | c2 | D02 | F |
| 2-1-c2D02-1-0237 | 667.140 | OH | a2 | B16 | b1 | C09 | c2 | D02 | F |
| 2-1-c2D02-1-0238 | 669.114 | OH | a1 | B05 | b1 | C13 | c2 | D02 | F |
| 2-1-c2D02-1-0239 | 669.119 | OH | a2 | B16 | b3 | C06 | c2 | D02 | F |
| 2-1-c2D02-1-0240 | 669.119 | OH | a2 | B17 | b3 | C08 | c2 | D02 | F |
| 2-1-c2D02-1-0241 | 670.136 | OH | a1 | B04 | b3 | C08 | c2 | D02 | F |
| 2-1-c2D02-1-0242 | 670.151 | OH | a1 | B07 | b2 | C04 | c2 | D02 | F |
| 2-1-c2D02-1-0243 | 670.165 | OH | a1 | B05 | b3 | C15 | c2 | D02 | F |
| 2-1-c2D02-1-0244 | 671.050 | OH | a1 | B03 | b1 | C09 | c2 | D02 | F |
| 2-1-c2D02-1-0245 | 672.080 | OH | a1 | B06 | b1 | C13 | c2 | D02 | F |
| 2-1-c2D02-1-0246 | 672.115 | OH | a2 | B19 | b2 | C06 | c2 | D02 | F |
| 2-1-c2D02-1-0247 | 672.130 | OH | a2 | B20 | b2 | C01 | c2 | D02 | F |
| 2-1-c2D02-1-0248 | 672.144 | OH | a2 | B18 | b1 | C13 | c2 | D02 | F |
| 2-1-c2D02-1-0249 | 672.205 | OH | a1 | B03 | b3 | C06 | c2 | D02 | F |
| 2-1-c2D02-1-0250 | 673.060 | OH | a1 | B06 | b3 | C15 | c2 | D02 | F |
| 2-1-c2D02-1-0251 | 673.094 | OH | a1 | B10 | b1 | C08 | c2 | D02 | F |
| 2-1-c2D02-1-0252 | 673.147 | OH | a1 | B05 | b1 | C14 | c2 | D02 | F |
| 2-1-c2D02-1-0253 | 674.079 | OH | a2 | B18 | b3 | C15 | c2 | D02 | F |
| 2-1-c2D02-1-0254 | 674.109 | OH | a1 | B08 | b2 | C02 | c2 | D02 | F |
| 2-1-c2D02-1-0255 | 674.109 | OH | a2 | B17 | b1 | C09 | c2 | D02 | F |
| 2-1-c2D02-1-0256 | 674.109 | OH | a1 | B10 | b1 | C11 | c2 | D02 | F |
| 2-1-c2D02-1-0257 | 675.089 | OH | a1 | B08 | b2 | C03 | c2 | D02 | F |
| 2-1-c2D02-1-0258 | 675.090 | OH | a1 | B09 | b2 | C01 | c2 | D02 | F |
| 2-1-c2D02-1-0259 | 675.105 | OH | a1 | B10 | b1 | C10 | c2 | D02 | F |
| 2-1-c2D02-1-0260 | 675.105 | OH | a1 | B04 | b1 | C09 | c2 | D02 | F |
| 2-1-c2D02-1-0261 | 675.148 | OH | a1 | B06 | b1 | C14 | c2 | D02 | F |
| 2-1-c2D02-1-0262 | 676.085 | OH | a2 | B17 | b3 | C06 | c2 | D02 | F |
| 2-1-c2D02-1-0263 | 676.100 | OH | a2 | B18 | b1 | C14 | c2 | D02 | F |
| 2-1-c2D02-1-0264 | 676.105 | OH | a1 | B04 | b3 | C06 | c2 | D02 | F |
| 2-1-c2D02-1-0265 | 676.110 | OH | a1 | B07 | b1 | C13 | c2 | D02 | F |
| 2-1-c2D02-1-0266 | 676.180 | OH | a1 | B05 | b2 | C07 | c2 | D02 | F |
| 2-1-c2D02-1-0267 | 677.109 | OH | a1 | B07 | b3 | C15 | c2 | D02 | F |
| 2-1-c2D02-1-0268 | 678.114 | OH | a1 | B06 | b2 | C07 | c2 | D02 | F |
| 2-1-c2D02-1-0269 | 678.135 | OH | a1 | B08 | b2 | C06 | c2 | D02 | F |
| 2-1-c2D02-1-0270 | 678.216 | OH | a1 | B10 | b1 | C12 | c2 | D02 | F |
| 2-1-c2D02-1-0271 | 679.176 | OH | a2 | B18 | b2 | C07 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0272 | 680.066 | OH | a1 | B07 | b1 | C14 | c2 | D02 | F |
| 2-1-c2D02-1-0273 | 680.119 | OH | a2 | B19 | b2 | C05 | c2 | D02 | F |
| 2-1-c2D02-1-0274 | 680.195 | OH | a1 | B05 | b3 | C08 | c2 | D02 | F |
| 2-1-c2D02-1-0275 | 681.046 | OH | a2 | B20 | b2 | C02 | c2 | D02 | F |
| 2-1-c2D02-1-0276 | 681.194 | OH | a2 | B20 | b2 | C03 | c2 | D02 | F |
| 2-1-c2D02-1-0277 | 682.080 | OH | a1 | B06 | b3 | C08 | c2 | D02 | F |
| 2-1-c2D02-1-0278 | 682.115 | OH | a2 | B19 | b2 | C04 | c2 | D02 | F |
| 2-1-c2D02-1-0279 | 682.120 | OH | a2 | B18 | b3 | C08 | c2 | D02 | F |
| 2-1-c2D02-1-0280 | 683.099 | OH | a1 | B09 | b2 | C02 | c2 | D02 | F |
| 2-1-c2D02-1-0281 | 684.094 | OH | a1 | B05 | b1 | C09 | c2 | D02 | F |
| 2-1-c2D02-1-0282 | 685.094 | OH | a1 | B07 | b2 | C07 | c2 | D02 | F |
| 2-1-c2D02-1-0283 | 685.114 | OH | a1 | B09 | b2 | C03 | c2 | D02 | F |
| 2-1-c2D02-1-0284 | 686.146 | OH | a1 | B05 | b3 | C06 | c2 | D02 | F |
| 2-1-c2D02-1-0285 | 686.166 | OH | a1 | B06 | b1 | C09 | c2 | D02 | F |
| 2-1-c2D02-1-0286 | 687.162 | OH | a2 | B20 | b2 | C06 | c2 | D02 | F |
| 2-1-c2D02-1-0287 | 688.077 | OH | a2 | B11 | b3 | C16 | c2 | D02 | F |
| 2-1-c2D02-1-0288 | 688.125 | OH | a2 | B18 | b1 | C09 | c2 | D02 | F |
| 2-1-c2D02-1-0289 | 689.055 | OH | a1 | B06 | b3 | C06 | c2 | D02 | F |
| 2-1-c2D02-1-0290 | 689.094 | OH | a1 | B07 | b3 | C08 | c2 | D02 | F |
| 2-1-c2D02-1-0291 | 689.124 | OH | a2 | B12 | b3 | C16 | c2 | D02 | F |
| 2-1-c2D02-1-0292 | 689.141 | OH | a1 | B08 | b2 | C05 | c2 | D02 | F |
| 2-1-c2D02-1-0293 | 690.056 | OH | a2 | B18 | b3 | C06 | c2 | D02 | F |
| 2-1-c2D02-1-0294 | 690.070 | OH | a2 | B11 | b3 | C17 | c2 | D02 | F |
| 2-1-c2D02-1-0295 | 690.089 | OH | a2 | B19 | b1 | C13 | c2 | D02 | F |
| 2-1-c2D02-1-0296 | 691.055 | OH | a2 | B12 | b3 | C17 | c2 | D02 | F |
| 2-1-c2D02-1-0297 | 691.115 | OH | a1 | B09 | b2 | C06 | c2 | D02 | F |
| 2-1-c2D02-1-0298 | 691.121 | OH | a1 | B10 | b2 | C01 | c2 | D02 | F |
| 2-1-c2D02-1-0299 | 692.066 | OH | a2 | B19 | b3 | C15 | c2 | D02 | F |
| 2-1-c2D02-1-0300 | 692.084 | OH | a1 | B08 | b2 | C04 | c2 | D02 | F |
| 2-1-c2D02-1-0301 | 692.120 | OH | a1 | B07 | b1 | C09 | c2 | D02 | F |
| 2-1-c2D02-1-0302 | 693.119 | OH | a2 | B19 | b1 | C14 | c2 | D02 | F |
| 2-1-c2D02-1-0303 | 694.171 | OH | a1 | B07 | b3 | C06 | c2 | D02 | F |
| 2-1-c2D02-1-0304 | 695.081 | OH | a2 | B20 | b2 | C05 | c2 | D02 | F |
| 2-1-c2D02-1-0305 | 696.027 | OH | a1 | B08 | b1 | C13 | c2 | D02 | F |
| 2-1-c2D02-1-0306 | 698.109 | OH | a2 | B19 | b2 | C07 | c2 | D02 | F |
| 2-1-c2D02-1-0307 | 699.101 | OH | a1 | B08 | b3 | C15 | c2 | D02 | F |
| 2-1-c2D02-1-0308 | 699.166 | OH | a2 | B20 | b2 | C04 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0309 | 701.036 | OH | a1 | B09 | b2 | C05 | c2 | D02 | F |
| 2-1-c2D02-1-0310 | 701.089 | OH | a1 | B10 | b2 | C02 | c2 | D02 | F |
| 2-1-c2D02-1-0311 | 703.067 | OH | a1 | B08 | b1 | C14 | c2 | D02 | F |
| 2-1-c2D02-1-0312 | 703.067 | OH | a2 | B19 | b3 | C08 | c2 | D02 | F |
| 2-1-c2D02-1-0313 | 703.071 | OH | a1 | B10 | b2 | C03 | c2 | D02 | F |
| 2-1-c2D02-1-0314 | 703.100 | OH | a2 | B13 | b3 | C16 | c2 | D02 | F |
| 2-1-c2D02-1-0315 | 703.109 | OH | a1 | B09 | b2 | C04 | c2 | D02 | F |
| 2-1-c2D02-1-0316 | 704.100 | OH | a2 | B13 | b3 | C17 | c2 | D02 | F |
| 2-1-c2D02-1-0317 | 704.120 | OH | a2 | B11 | b3 | C18 | c2 | D02 | F |
| 2-1-c2D02-1-0318 | 706.066 | OH | a2 | B12 | b3 | C18 | c2 | D02 | F |
| 2-1-c2D02-1-0319 | 707.061 | OH | a2 | B14 | b3 | C16 | c2 | D02 | F |
| 2-1-c2D02-1-0320 | 707.084 | OH | a2 | B19 | b1 | C09 | c2 | D02 | F |
| 2-1-c2D02-1-0321 | 707.096 | OH | a2 | B20 | b1 | C13 | c2 | D02 | F |
| 2-1-c2D02-1-0322 | 707.110 | OH | a1 | B08 | b2 | C07 | c2 | D02 | F |
| 2-1-c2D02-1-0323 | 707.171 | OH | a1 | B10 | b2 | C06 | c2 | D02 | F |
| 2-1-c2D02-1-0324 | 708.061 | OH | a1 | B01 | b3 | C16 | c2 | D02 | F |
| 2-1-c2D02-1-0325 | 708.100 | OH | a2 | B19 | b3 | C06 | c2 | D02 | F |
| 2-1-c2D02-1-0326 | 709.027 | OH | a2 | B20 | b3 | C15 | c2 | D02 | F |
| 2-1-c2D02-1-0327 | 709.045 | OH | a2 | B14 | b3 | C17 | c2 | D02 | F |
| 2-1-c2D02-1-0328 | 709.046 | OH | a1 | B02 | b3 | C16 | c2 | D02 | F |
| 2-1-c2D02-1-0329 | 709.114 | OH | a1 | B01 | b3 | C17 | c2 | D02 | F |
| 2-1-c2D02-1-0330 | 710.092 | OH | a1 | B09 | b1 | C13 | c2 | D02 | F |
| 2-1-c2D02-1-0331 | 710.121 | OH | a1 | B02 | b3 | C17 | c2 | D02 | F |
| 2-1-c2D02-1-0332 | 711.056 | OH | a1 | B08 | b3 | C08 | c2 | D02 | F |
| 2-1-c2D02-1-0333 | 711.076 | OH | a2 | B20 | b1 | C14 | c2 | D02 | F |
| 2-1-c2D02-1-0334 | 711.076 | OH | a1 | B09 | b3 | C15 | c2 | D02 | F |
| 2-1-c2D02-1-0335 | 714.087 | OH | a1 | B09 | b1 | C14 | c2 | D02 | F |
| 2-1-c2D02-1-0336 | 714.177 | OH | a1 | B08 | b1 | C09 | c2 | D02 | F |
| 2-1-c2D02-1-0337 | 715.157 | OH | a2 | B20 | b2 | C07 | c2 | D02 | F |
| 2-1-c2D02-1-0338 | 715.161 | OH | a1 | B08 | b3 | C06 | c2 | D02 | F |
| 2-1-c2D02-1-0339 | 717.013 | OH | a1 | B10 | b2 | C05 | c2 | D02 | F |
| 2-1-c2D02-1-0340 | 717.115 | OH | a2 | B15 | b3 | C16 | c2 | D02 | F |
| 2-1-c2D02-1-0341 | 718.172 | OH | a1 | B09 | b2 | C07 | c2 | D02 | F |
| 2-1-c2D02-1-0342 | 719.066 | OH | a2 | B20 | b3 | C08 | c2 | D02 | F |
| 2-1-c2D02-1-0343 | 719.087 | OH | a2 | B15 | b3 | C17 | c2 | D02 | F |
| 2-1-c2D02-1-0344 | 719.104 | OH | a2 | B16 | b3 | C16 | c2 | D02 | F |
| 2-1-c2D02-1-0345 | 720.082 | OH | a2 | B13 | b3 | C18 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0346 | 720.095 | OH | a1 | B10 | b2 | C04 | c2 | D02 | F |
| 2-1-c2D02-1-0347 | 721.066 | OH | a2 | B16 | b3 | C17 | c2 | D02 | F |
| 2-1-c2D02-1-0348 | 722.076 | OH | a1 | B09 | b3 | C08 | c2 | D02 | F |
| 2-1-c2D02-1-0349 | 722.115 | OH | a1 | B03 | b3 | C16 | c2 | D02 | F |
| 2-1-c2D02-1-0350 | 723.091 | OH | a2 | B20 | b1 | C09 | c2 | D02 | F |
| 2-1-c2D02-1-0351 | 724.057 | OH | a2 | B14 | b3 | C18 | c2 | D02 | F |
| 2-1-c2D02-1-0352 | 724.077 | OH | a1 | B03 | b3 | C17 | c2 | D02 | F |
| 2-1-c2D02-1-0353 | 725.022 | OH | a2 | B20 | b3 | C06 | c2 | D02 | F |
| 2-1-c2D02-1-0354 | 725.072 | OH | a1 | B01 | b3 | C18 | c2 | D02 | F |
| 2-1-c2D02-1-0355 | 725.075 | OH | a2 | B17 | b3 | C16 | c2 | D02 | F |
| 2-1-c2D02-1-0356 | 726.067 | OH | a1 | B02 | b3 | C18 | c2 | D02 | F |
| 2-1-c2D02-1-0357 | 726.090 | OH | a1 | B04 | b3 | C16 | c2 | D02 | F |
| 2-1-c2D02-1-0358 | 726.102 | OH | a1 | B09 | b1 | C09 | c2 | D02 | F |
| 2-1-c2D02-1-0359 | 726.116 | OH | a1 | B10 | b1 | C13 | c2 | D02 | F |
| 2-1-c2D02-1-0360 | 727.036 | OH | a2 | B17 | b3 | C17 | c2 | D02 | F |
| 2-1-c2D02-1-0361 | 728.033 | OH | a1 | B09 | b3 | C06 | c2 | D02 | F |
| 2-1-c2D02-1-0362 | 728.051 | OH | a1 | B10 | b3 | C15 | c2 | D02 | F |
| 2-1-c2D02-1-0363 | 728.052 | OH | a1 | B04 | b3 | C17 | c2 | D02 | F |
| 2-1-c2D02-1-0364 | 730.082 | OH | a1 | B10 | b1 | C14 | c2 | D02 | F |
| 2-1-c2D02-1-0365 | 734.098 | OH | a2 | B15 | b3 | C18 | c2 | D02 | F |
| 2-1-c2D02-1-0366 | 734.167 | OH | a1 | B10 | b2 | C07 | c2 | D02 | F |
| 2-1-c2D02-1-0367 | 736.077 | OH | a2 | B16 | b3 | C18 | c2 | D02 | F |
| 2-1-c2D02-1-0368 | 736.131 | OH | a1 | B05 | b3 | C16 | c2 | D02 | F |
| 2-1-c2D02-1-0369 | 738.071 | OH | a1 | B10 | b3 | C08 | c2 | D02 | F |
| 2-1-c2D02-1-0370 | 738.092 | OH | a1 | B05 | b3 | C17 | c2 | D02 | F |
| 2-1-c2D02-1-0371 | 738.110 | OH | a1 | B06 | b3 | C16 | c2 | D02 | F |
| 2-1-c2D02-1-0372 | 739.088 | OH | a1 | B03 | b3 | C18 | c2 | D02 | F |
| 2-1-c2D02-1-0373 | 739.109 | OH | a2 | B18 | b3 | C16 | c2 | D02 | F |
| 2-1-c2D02-1-0374 | 740.072 | OH | a1 | B06 | b3 | C17 | c2 | D02 | F |
| 2-1-c2D02-1-0375 | 741.071 | OH | a2 | B18 | b3 | C17 | c2 | D02 | F |
| 2-1-c2D02-1-0376 | 742.047 | OH | a2 | B17 | b3 | C18 | c2 | D02 | F |
| 2-1-c2D02-1-0377 | 742.097 | OH | a1 | B10 | b1 | C09 | c2 | D02 | F |
| 2-1-c2D02-1-0378 | 743.063 | OH | a1 | B04 | b3 | C18 | c2 | D02 | F |
| 2-1-c2D02-1-0379 | 744.028 | OH | a1 | B10 | b3 | C06 | c2 | D02 | F |
| 2-1-c2D02-1-0380 | 744.081 | OH | a1 | B07 | b3 | C16 | c2 | D02 | F |
| 2-1-c2D02-1-0381 | 746.042 | OH | a1 | B07 | b3 | C17 | c2 | D02 | F |
| 2-1-c2D02-1-0382 | 753.103 | OH | a1 | B05 | b3 | C18 | c2 | D02 | F |

(continued)

| [Table 8-5-2-2] Compound that may be contained in mixture 2-1-c2D02-1 | | | | | | | | | |
|---|---|---|---|---|---|---|---|---|---|
| ID | Exact Mass | | a | B | b | C | c | D | d |
| 2-1-c2D02-1-0383 | 755.083 | OH | a1 | B06 | b3 | C18 | c2 | D02 | F |
| 2-1-c2D02-1-0384 | 756.082 | OH | a2 | B18 | b3 | C18 | c2 | D02 | F |
| 2-1-c2D02-1-0385 | 757.081 | OH | a2 | B19 | b3 | C16 | c2 | D02 | F |
| 2-1-c2D02-1-0386 | 759.043 | OH | a2 | B19 | b3 | C17 | c2 | D02 | F |
| 2-1-c2D02-1-0387 | 761.053 | OH | a1 | B07 | b3 | C18 | c2 | D02 | F |
| 2-1-c2D02-1-0388 | 765.067 | OH | a1 | B08 | b3 | C16 | c2 | D02 | F |
| 2-1-c2D02-1-0389 | 767.028 | OH | a1 | B08 | b3 | C17 | c2 | D02 | F |
| 2-1-c2D02-1-0390 | 773.076 | OH | a2 | B20 | b3 | C16 | c2 | D02 | F |
| 2-1-c2D02-1-0391 | 774.054 | OH | a2 | B19 | b3 | C18 | c2 | D02 | F |
| 2-1-c2D02-1-0392 | 775.038 | OH | a2 | B20 | b3 | C17 | c2 | D02 | F |
| 2-1-c2D02-1-0393 | 776.087 | OH | a1 | B09 | b3 | C16 | c2 | D02 | F |
| 2-1-c2D02-1-0394 | 778.049 | OH | a1 | B09 | b3 | C17 | c2 | D02 | F |
| 2-1-c2D02-1-0395 | 782.039 | OH | a1 | B08 | b3 | C18 | c2 | D02 | F |
| 2-1-c2D02-1-0396 | 790.049 | OH | a2 | B20 | b3 | C18 | c2 | D02 | F |
| 2-1-c2D02-1-0397 | 792.082 | OH | a1 | B10 | b3 | C16 | c2 | D02 | F |
| 2-1-c2D02-1-0398 | 793.059 | OH | a1 | B09 | b3 | C18 | c2 | D02 | F |
| 2-1-c2D02-1-0399 | 794.043 | OH | a1 | B10 | b3 | C17 | c2 | D02 | F |
| 2-1-c2D02-1-0400 | 809.054 | OH | a1 | B10 | b3 | C18 | c2 | D02 | F |

Example 8-5-3: Synthesis of 2-1-c3aD02-0 by step c3aD02

A reaction formula in step c3aD02 performed using mixture 2-1-c3aD00-0 will be illustrated below.

[Formula 633]

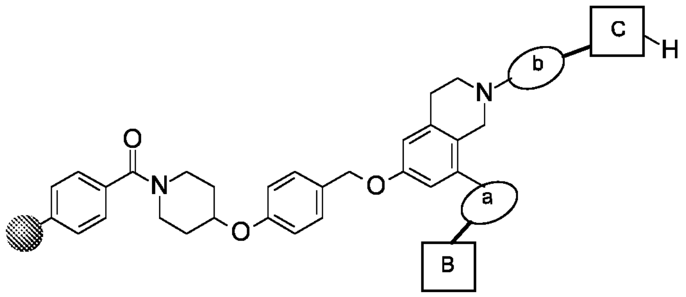

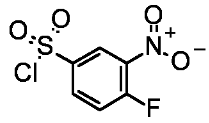

BB2-1-53

2-1-c3aD00-0

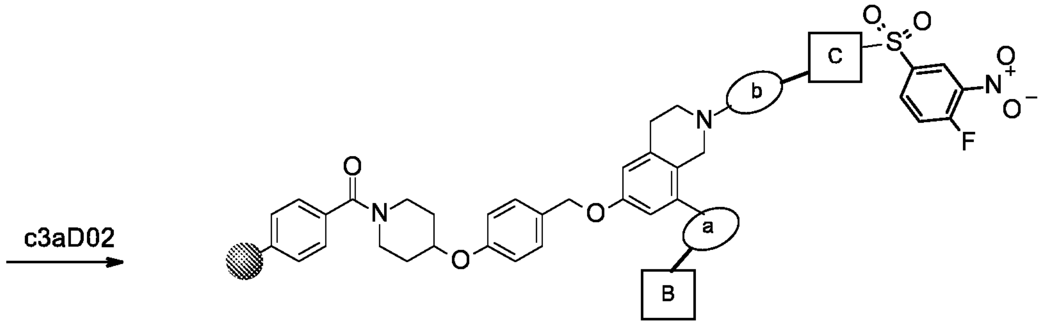


2-1-c3aD02-0

Mixture 2-1-c3aD02-0 was synthesized as follows using mixture 2-1-d21C29-0 to mixture 2-1-d25C33-0.

5 types of mixtures described in Table 8-5-3-1, DCE (2.8 mL), and DTBP (0.439 mL, 2.00 mmol) were added to a 4 mL glass vial and shaken at room temperature for 1 hour. A solution of DIPEA (17.42 μL, 100 μmol) and 4-fluoro-3-nitrobenzenesulfonyl chloride (BB2-1-53, 80.0 mg, 0.333 mmol) in DCE (700 μL) was added thereto, and the mixture was shaken at 60°C for 23 hours.

Solid-phase purification

The suspension of the reaction solution and the resin was transferred to a 6 mL column with a filter, filtered, and washed three times with NMP/water = 1/1 (3.5 mL), three times with NMP (3.5 mL), three times with 0.05 M $TBAHSO_4$ + DTBP in NMP (3.5 mL), three times with 0.05 M NMM in NMP (3.5 mL), three times with NMP/water = 1/1 (3.5 mL), three times with NMP (3.5 mL), three times with MeOH (3.5 mL), three times with DCM (3.5 mL), and three times with heptane (3.5 mL), and the obtained solid phase was dried under reduced pressure to obtain mixture 2-1-c3aD02-0.

[Table 377]

| [Table 8-5-3-1] Starting material mixture used in step c3aD02 | |
|---|---|
| Mixture | Amount used |
| 2-1-d21C29-0 | 35 mg |
| 2-1-d22C30-0 | 35 mg |
| 2-1-d23C31-0 | 35 mg |
| 2-1-d24C32-0 | 35 mg |
| 2-1-d25C33-0 | 35 mg |

Mixture 2-1-c3aD02-0 can be treated by cleavage in the same manner as in Example 2-1-1 to obtain mixture 2-1-c3aD02-1. The details of the mixture are shown in Table 8-5-3-2. A notation method in each table will be described. In Table 8-5-3-2, each compound that may be contained in mixture 2-1-c3aD02-1 is indicated by ID, and core blocks and linkers in combination in the structure of mixture 2-1-c3aD02-1 are indicated by symbols or chemical formulas. The correspondence between the symbols and specific structures are shown in Figure 4. In Table 8-5-3-2, "×" and "d" are represented by chemical formulas.

[Formula 634]

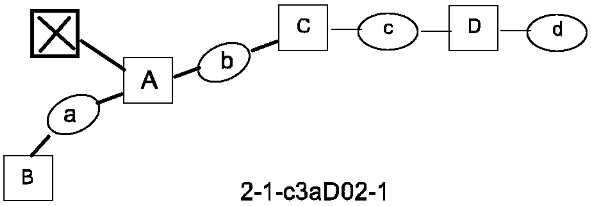

2-1-c3aD02-1

For example, when ID is 2-1-c3aD02-1-0001, the compound is represented by the following structural formula.

[Formula 635]

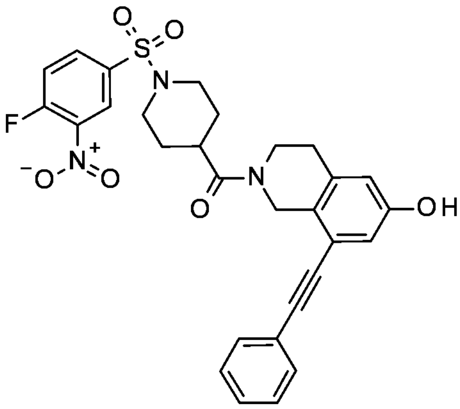

2-1-c3aD02-1-0001

[Table 378]

| Notation in [Table 8-5-3-2] | | | | | | | | |
|---|---|---|---|---|---|---|---|---|
| ID | | a | B | b | C | c | D | d |
| 2-1-c3aD02-1-0001 | OH | a2 | B11 | b2 | C33 | c3 | D02 | F |

[Table 379]

| [Table 8-5-3-2] Compound that may be contained in mixture 2-1-c3aD02-1 | | | | | | | | | |
|---|---|---|---|---|---|---|---|---|---|
| ID | Exact Mass | | a | B | b | C | c | D | d |
| 2-1-c3aD02-1-0001 | 563.153 | OH | a2 | B11 | b2 | C33 | c3 | D02 | F |
| 2-1-c3aD02-1-0002 | 564.148 | OH | a2 | B12 | b2 | C33 | c3 | D02 | F |
| 2-1-c3aD02-1-0003 | 577.092 | OH | a2 | B13 | b2 | C33 | c3 | D02 | F |
| 2-1-c3aD02-1-0004 | 580.121 | OH | a2 | B14 | b2 | C33 | c3 | D02 | F |
| 2-1-c3aD02-1-0005 | 581.176 | OH | a1 | B01 | b2 | C33 | c3 | D02 | F |
| 2-1-c3aD02-1-0006 | 582.158 | OH | a1 | B02 | b2 | C33 | c3 | D02 | F |
| 2-1-c3aD02-1-0007 | 583.133 | OH | a2 | B11 | b1 | C30 | c3 | D02 | F |
| 2-1-c3aD02-1-0008 | 583.158 | OH | a2 | B12 | b1 | C30 | c3 | D02 | F |
| 2-1-c3aD02-1-0009 | 591.102 | OH | a2 | B15 | b2 | C33 | c3 | D02 | F |
| 2-1-c3aD02-1-0010 | 591.184 | OH | a2 | B16 | b2 | C33 | c3 | D02 | F |
| 2-1-c3aD02-1-0011 | 594.153 | OH | a1 | B03 | b2 | C33 | c3 | D02 | F |
| 2-1-c3aD02-1-0012 | 595.148 | OH | a2 | B13 | b1 | C30 | c3 | D02 | F |
| 2-1-c3aD02-1-0013 | 595.248 | OH | a2 | B11 | b1 | C32 | c3 | D02 | F |
| 2-1-c3aD02-1-0014 | 596.162 | OH | a2 | B12 | b1 | C32 | c3 | D02 | F |
| 2-1-c3aD02-1-0015 | 597.079 | OH | a2 | B17 | b2 | C33 | c3 | D02 | F |
| 2-1-c3aD02-1-0016 | 599.134 | OH | a1 | B04 | b2 | C33 | c3 | D02 | F |
| 2-1-c3aD02-1-0017 | 600.148 | OH | a2 | B14 | b1 | C30 | c3 | D02 | F |
| 2-1-c3aD02-1-0018 | 600.182 | OH | a1 | B01 | b1 | C30 | c3 | D02 | F |
| 2-1-c3aD02-1-0019 | 601.132 | OH | a1 | B02 | b1 | C30 | c3 | D02 | F |
| 2-1-c3aD02-1-0020 | 608.118 | OH | a1 | B05 | b2 | C33 | c3 | D02 | F |
| 2-1-c3aD02-1-0021 | 608.182 | OH | a2 | B15 | b1 | C30 | c3 | D02 | F |
| 2-1-c3aD02-1-0022 | 609.093 | OH | a2 | B11 | b1 | C29 | c3 | D02 | F |
| 2-1-c3aD02-1-0023 | 609.097 | OH | a2 | B13 | b1 | C32 | c3 | D02 | F |
| 2-1-c3aD02-1-0024 | 609.181 | OH | a1 | B06 | b2 | C33 | c3 | D02 | F |
| 2-1-c3aD02-1-0025 | 610.099 | OH | a2 | B12 | b1 | C29 | c3 | D02 | F |
| 2-1-c3aD02-1-0026 | 611.094 | OH | a2 | B16 | b1 | C30 | c3 | D02 | F |
| 2-1-c3aD02-1-0027 | 611.116 | OH | a2 | B18 | b2 | C33 | c3 | D02 | F |
| 2-1-c3 aD02-1-0028 | 612.169 | OH | a2 | B14 | b1 | C32 | c3 | D02 | F |
| 2-1-c3aD02-1-0029 | 613.147 | OH | a1 | B03 | b1 | C30 | c3 | D02 | F |
| 2-1-c3aD02-1-0030 | 613.168 | OH | a1 | B01 | b1 | C32 | c3 | D02 | F |
| 2-1-c3aD02-1-0031 | 615.069 | OH | a1 | B02 | b1 | C32 | c3 | D02 | F |
| 2-1-c3aD02-1-0032 | 616.179 | OH | a1 | B07 | b2 | C33 | c3 | D02 | F |
| 2-1-c3aD02-1-0033 | 618.102 | OH | a2 | B17 | b1 | C30 | c3 | D02 | F |
| 2-1-c3aD02-1-0034 | 618.158 | OH | a1 | B04 | b1 | C30 | c3 | D02 | F |
| 2-1-c3aD02-1-0035 | 622.148 | OH | a2 | B13 | b1 | C29 | c3 | D02 | F |
| 2-1-c3aD02-1-0036 | 622.184 | OH | a2 | B15 | b1 | C32 | c3 | D02 | F |

(continued)

| [Table 8-5-3-2] Compound that may be contained in mixture 2-1-c3aD02-1 | | | | | | | | | |
|---|---|---|---|---|---|---|---|---|---|
| ID | Exact Mass | | a | B | b | C | c | D | d |
| 2-1-c3aD02-1-0037 | 625.132 | OH | a2 | B16 | b1 | C32 | c3 | D02 | F |
| 2-1-c3aD02-1-0038 | 627.093 | OH | a2 | B14 | b1 | C29 | c3 | D02 | F |
| 2-1-c3 aD02-1-003 9 | 627.104 | OH | a1 | B05 | b1 | C30 | c3 | D02 | F |
| 2-1-c3aD02-1-0040 | 627.104 | OH | a1 | B01 | b1 | C29 | c3 | D02 | F |
| 2-1-c3aD02-1-0041 | 627.148 | OH | a1 | B03 | b1 | C32 | c3 | D02 | F |
| 2-1-c3aD02-1-0042 | 628.089 | OH | a2 | B19 | b2 | C33 | c3 | D02 | F |
| 2-1-c3aD02-1-0043 | 628.099 | OH | a1 | B02 | b1 | C29 | c3 | D02 | F |
| 2-1-c3aD02-1-0044 | 629.127 | OH | a1 | B06 | b1 | C30 | c3 | D02 | F |
| 2-1-c3aD02-1-0045 | 630.195 | OH | a2 | B17 | b1 | C32 | c3 | D02 | F |
| 2-1-c3aD02-1-0046 | 631.122 | OH | a2 | B18 | b1 | C30 | c3 | D02 | F |
| 2-1-c3aD02-1-0047 | 632.065 | OH | a1 | B04 | b1 | C32 | c3 | D02 | F |
| 2-1-c3 aD02-1-0048 | 636.133 | OH | a1 | B07 | b1 | C30 | c3 | D02 | F |
| 2-1-c3aD02-1-0049 | 637.158 | OH | a1 | B08 | b2 | C33 | c3 | D02 | F |
| 2-1-c3aD02-1-0050 | 638.128 | OH | a2 | B11 | b1 | C31 | c3 | D02 | F |
| 2-1-c3aD02-1-0051 | 638.128 | OH | a2 | B15 | b1 | C29 | c3 | D02 | F |
| 2-1-c3aD02-1-0052 | 639.109 | OH | a2 | B12 | b1 | C31 | c3 | D02 | F |
| 2-1-c3aD02-1-0053 | 639.184 | OH | a2 | B16 | b1 | C29 | c3 | D02 | F |
| 2-1-c3aD02-1-0054 | 642.158 | OH | a1 | B03 | b1 | C29 | c3 | D02 | F |
| 2-1-c3aD02-1-0055 | 643.053 | OH | a1 | B05 | b1 | C32 | c3 | D02 | F |
| 2-1-c3aD02-1-0056 | 645.065 | OH | a1 | B06 | b1 | C32 | c3 | D02 | F |
| 2-1-c3aD02-1-0057 | 646.060 | OH | a2 | B20 | b2 | C33 | c3 | D02 | F |
| 2-1-c3aD02-1-0058 | 646.153 | OH | a2 | B17 | b1 | C29 | c3 | D02 | F |
| 2-1-c3aD02-1-0059 | 646.159 | OH | a2 | B18 | b1 | C32 | c3 | D02 | F |
| 2-1-c3aD02-1-0060 | 647.094 | OH | a1 | B04 | b1 | C29 | c3 | D02 | F |
| 2-1-c3aD02-1-0061 | 648.133 | OH | a1 | B09 | b2 | C33 | c3 | D02 | F |
| 2-1-c3aD02-1-0062 | 649.132 | OH | a2 | B19 | b1 | C30 | c3 | D02 | F |
| 2-1-c3aD02-1-0063 | 650.148 | OH | a1 | B07 | b1 | C32 | c3 | D02 | F |
| 2-1-c3aD02-1-0064 | 653.051 | OH | a2 | B13 | b1 | C31 | c3 | D02 | F |
| 2-1-c3aD02-1-0065 | 656.099 | OH | a2 | B14 | b1 | C31 | c3 | D02 | F |
| 2-1-c3aD02-1-0066 | 657.099 | OH | a1 | B01 | b1 | C31 | c3 | D02 | F |
| 2-1-c3aD02-1-0067 | 657.099 | OH | a1 | B05 | b1 | C29 | c3 | D02 | F |
| 2-1-c3aD02-1-0068 | 658.115 | OH | a1 | B08 | b1 | C30 | c3 | D02 | F |
| 2-1-c3aD02-1-0069 | 658.115 | OH | a1 | B02 | b1 | C31 | c3 | D02 | F |
| 2-1-c3aD02-1-0070 | 659.110 | OH | a1 | B06 | b1 | C29 | c3 | D02 | F |
| 2-1-c3aD02-1-0071 | 660.115 | OH | a2 | B18 | b1 | C29 | c3 | D02 | F |
| 2-1-c3aD02-1-0072 | 664.089 | OH | a2 | B19 | b1 | C32 | c3 | D02 | F |
| 2-1-c3aD02-1-0073 | 664.144 | OH | a1 | B10 | b2 | C33 | c3 | D02 | F |
| 2-1-c3aD02-1-0074 | 664.200 | OH | a1 | B07 | b1 | C29 | c3 | D02 | F |

(continued)

| [Table 8-5-3-2] Compound that may be contained in mixture 2-1-c3aD02-1 | | | | | | | | | |
|---|---|---|---|---|---|---|---|---|---|
| ID | Exact Mass | | a | B | b | C | c | D | d |
| 2-1-c3aD02-1-0075 | 665.161 | OH | a2 | B20 | b1 | C30 | c3 | D02 | F |
| 2-1-c3aD02-1-0076 | 666.123 | OH | a2 | B15 | b1 | C31 | c3 | D02 | F |
| 2-1-c3aD02-1-0077 | 667.215 | OH | a2 | B16 | b1 | C31 | c3 | D02 | F |
| 2-1-c3aD02-1-0078 | 669.071 | OH | a1 | B09 | b1 | C30 | c3 | D02 | F |
| 2-1-c3aD02-1-0079 | 672.100 | OH | a1 | B03 | b1 | C31 | c3 | D02 | F |
| 2-1-c3aD02-1-0080 | 672.169 | OH | a1 | B08 | b1 | C32 | c3 | D02 | F |
| 2-1-c3aD02-1-0081 | 675.078 | OH | a2 | B17 | b1 | C31 | c3 | D02 | F |
| 2-1-c3aD02-1-0082 | 675.165 | OH | a1 | B04 | b1 | C31 | c3 | D02 | F |
| 2-1-c3aD02-1-0083 | 677.109 | OH | a2 | B19 | b1 | C29 | c3 | D02 | F |
| 2-1-c3aD02-1-0084 | 679.101 | OH | a2 | B20 | b1 | C32 | c3 | D02 | F |
| 2-1-c3aD02-1-0085 | 682.080 | OH | a1 | B09 | b1 | C32 | c3 | D02 | F |
| 2-1-c3aD02-1-0086 | 684.167 | OH | a1 | B10 | b1 | C30 | c3 | D02 | F |
| 2-1-c3aD02-1-0087 | 685.099 | OH | a1 | B05 | b1 | C31 | c3 | D02 | F |
| 2-1-c3aD02-1-0088 | 686.146 | OH | a1 | B08 | b1 | C29 | c3 | D02 | F |
| 2-1-c3aD02-1-0089 | 687.105 | OH | a1 | B06 | b1 | C31 | c3 | D02 | F |
| 2-1-c3aD02-1-0090 | 688.200 | OH | a2 | B18 | b1 | C31 | c3 | D02 | F |
| 2-1-c3aD02-1-0091 | 693.050 | OH | a1 | B07 | b1 | C31 | c3 | D02 | F |
| 2-1-c3aD02-1-0092 | 694.096 | OH | a2 | B20 | b1 | C29 | c3 | D02 | F |
| 2-1-c3aD02-1-0093 | 695.171 | OH | a1 | B09 | b1 | C29 | c3 | D02 | F |
| 2-1-c3aD02-1-0094 | 698.182 | OH | a1 | B10 | b1 | C32 | c3 | D02 | F |
| 2-1-c3aD02-1-0095 | 707.152 | OH | a2 | B19 | b1 | C31 | c3 | D02 | F |
| 2-1-c3aD02-1-0096 | 714.087 | OH | a1 | B10 | b1 | C29 | c3 | D02 | F |
| 2-1-c3aD02-1-0097 | 715.082 | OH | a1 | B08 | b1 | C31 | c3 | D02 | F |
| 2-1-c3aD02-1-0098 | 723.166 | OH | a2 | B20 | b1 | C31 | c3 | D02 | F |
| 2-1-c3aD02-1-0099 | 726.177 | OH | a1 | B09 | b1 | C31 | c3 | D02 | F |
| 2-1-c3aD02-1-0 1 00 | 742.172 | OH | a1 | B10 | b1 | C31 | c3 | D02 | F |

Example 8-5-4: Synthesis of 2-1-c3bD02-0 by step c3bD02

A reaction formula in step c3bD02 performed using mixture 2-1-c3bD00-0 will be illustrated below.

[Formula 636]

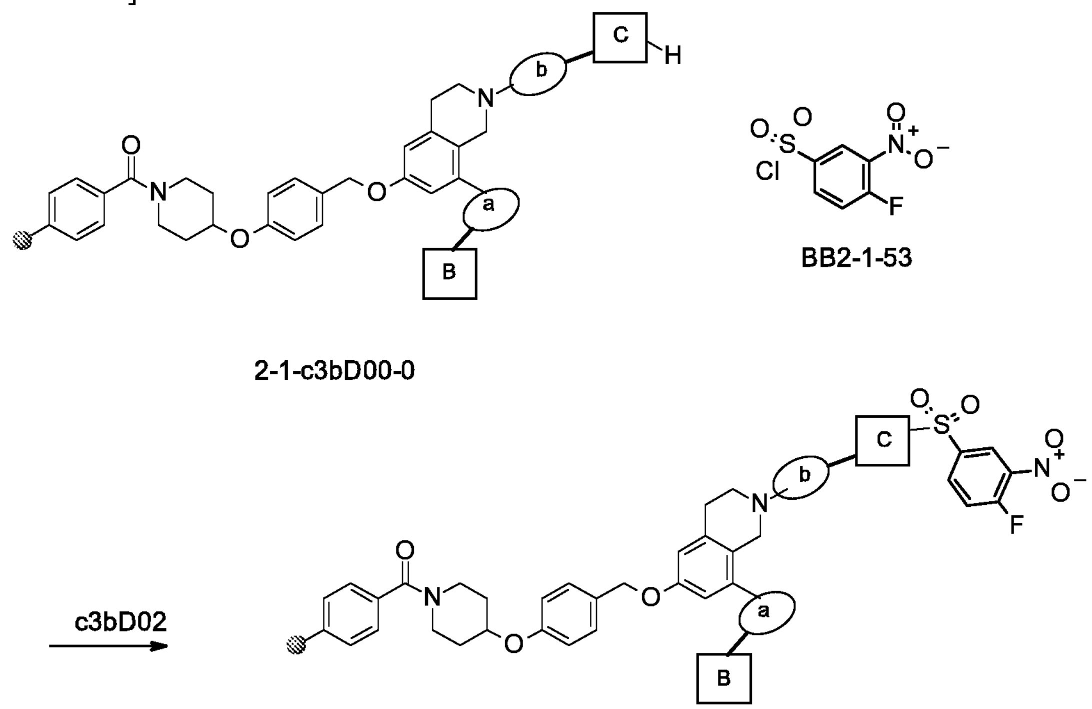


BB2-1-53

2-1-c3bD00-0

2-1-c3bD02-0

Mixture 2-1-c3bD02-0 was synthesized as follows using mixture 2-1 -d26C 19-0 to mixture 2-1-d30C27-0.

5 types of mixtures described in Table 8-5-4-1, DCE (2.8 mL), and DTBP (0.441 mL, 2.01 mmol) were added to a 4 mL glass vial and shaken at room temperature for 1 hour. A solution of 4-fluoro-3-nitrobenzenesulfonyl chloride (BB2-1-53, 80.0 mg, 0.334 mmol) in DCE (700 μL) was added thereto, and the mixture was shaken at 60°C for 23 hours.

Solid-phase purification

The suspension of the reaction solution and the resin was transferred to a 6 mL column with a filter, filtered, and washed three times with NMP/water = 1/1 (3.5 mL), three times with NMP (3.5 mL), three times with 0.05 M $TBAHSO_4$ + DTBP in NMP (3.5 mL), three times with 0.05 M NMM in NMP (3.5 mL), three times with NMP/water = 1/1 (3.5 mL), three times with NMP (3.5 mL), three times with MeOH (3.5 mL), three times with DCM (3.5 mL), and three times with heptane (3.5 mL), and the obtained solid phase was dried under reduced pressure to obtain mixture 2-1-c3bD02-0.

[Table 380]

| [Table 8-5-4-1] Starting material mixture used in step c3bD02 | |
|---|---|
| Mixture | Amount used |
| 2-1-d26C19-0 | 35 mg |
| 2-1-d27C21-0 | 35 mg |
| 2-1-d28C23-0 | 35 mg |
| 2-1-d29C25-0 | 35 mg |
| 2-1-d30C27-0 | 35 mg |

Mixture 2-1-c3bD02-0 can be treated by cleavage in the same manner as in Example 8-1-1 to obtain mixture 2-1-c3bD02-1. The details of the mixture are shown in Table 8-5-4-2.

A notation method in each table will be described. In Table 8-5-4-2, each compound that may be contained in 2-1-c3bD02-1 is indicated by ID, and core blocks and linkers in combination in the structure of mixture 2-1-c3bD02-1

are indicated by symbols or chemical formulas. The correspondence between the symbols and specific structures are shown in Figure 4. In Table 8-5-4-2, "×" and "d" are represented by chemical formulas.

[Formula 637]

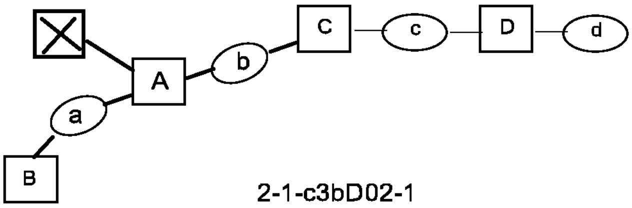

2-1-c3bD02-1

For example, when ID is 2-1-c3bD02-1-0001, the compound is represented by the following structural formula.

[Formula 638]

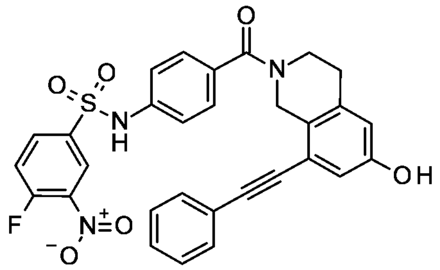

2-1-c3bD02-1-0001

[Table 381]

| Notation in [Table 8-5-4-2] | | | | | | | | |
|---|---|---|---|---|---|---|---|---|
| ID | | a | B | b | C | c | D | d |
| 2-1-c3bD02-1-0001 | OH | a2 | B11 | b2 | C19 | c3 | D02 | F |

[Table 382]

| [Table 8-5-4-2] Compound that may be contained in mixture 2-1-c3bD02-1 | | | | | | | | | |
|---|---|---|---|---|---|---|---|---|---|
| ID | Exact Mass | | a | B | b | C | c | D | d |
| 2-1-c3bD02-1-0001 | 584.171 | OH | a2 | B11 | b2 | C19 | c3 | D02 | F |
| 2-1-c3bD02-1-0002 | 585.137 | OH | a2 | B12 | b2 | C19 | c3 | D02 | F |
| 2-1-c3bD02-1-0003 | 585.166 | OH | a2 | B11 | b2 | C27 | c3 | D02 | F |
| 2-1-c3bD02-1-0004 | 586.146 | OH | a2 | B12 | b2 | C27 | c3 | D02 | F |
| 2-1-c3bD02-1-0005 | 597.173 | OH | a2 | B13 | b2 | C19 | c3 | D02 | F |
| 2-1-c3bD02-1-0006 | 597.202 | OH | a2 | B11 | b2 | C21 | c3 | D02 | F |
| 2-1-c3bD02-1-0007 | 599.113 | OH | a2 | B12 | b2 | C21 | c3 | D02 | F |
| 2-1-c3bD02-1-0008 | 599.116 | OH | a2 | B13 | b2 | C27 | c3 | D02 | F |
| 2-1-c3bD02-1-0009 | 601.107 | OH | a2 | B14 | b2 | C19 | c3 | D02 | F |

(continued)

| [Table 8-5-4-2] Compound that may be contained in mixture 2-1-c3bD02-1 | | | | | | | | | |
|---|---|---|---|---|---|---|---|---|---|
| ID | Exact Mass | | a | B | b | C | c | D | d |
| 2-1-c3bD02-1-0010 | 601.107 | OH | a2 | B11 | b2 | C23 | c3 | D02 | F |
| 2-1-c3bD02-1-0011 | 601.148 | OH | a2 | B12 | b2 | C23 | c3 | D02 | F |
| 2-1-c3bD02-1-0012 | 602.127 | OH | a2 | B14 | b2 | C27 | c3 | D02 | F |
| 2-1-c3bD02-1-0013 | 602.164 | OH | a1 | B01 | b2 | C19 | c3 | D02 | F |
| 2-1-c3bD02-1-0014 | 603.142 | OH | a1 | B02 | b2 | C19 | c3 | D02 | F |
| 2-1-c3bD02-1-0015 | 603.159 | OH | a1 | B01 | b2 | C27 | c3 | D02 | F |
| 2-1-c3bD02-1-0016 | 604.143 | OH | a1 | B02 | b2 | C27 | c3 | D02 | F |
| 2-1-c3bD02-1-0017 | 610.169 | OH | a2 | B15 | b2 | C19 | c3 | D02 | F |
| 2-1-c3bD02-1-0018 | 610.190 | OH | a2 | B13 | b2 | C21 | c3 | D02 | F |
| 2-1-c3bD02-1-0019 | 611.189 | OH | a2 | B15 | b2 | C27 | c3 | D02 | F |
| 2-1-c3bD02-1-0020 | 612.143 | OH | a2 | B16 | b2 | C19 | c3 | D02 | F |
| 2-1-c3bD02-1-0021 | 612.148 | OH | a2 | B11 | b2 | C25 | c3 | D02 | F |
| 2-1-c3bD02-1-0022 | 613.059 | OH | a2 | B12 | b2 | C25 | c3 | D02 | F |
| 2-1-c3bD02-1-0023 | 613.132 | OH | a2 | B16 | b2 | C27 | c3 | D02 | F |
| 2-1-c3bD02-1-0024 | 614.153 | OH | a2 | B14 | b2 | C21 | c3 | D02 | F |
| 2-1-c3bD02-1-0025 | 614.254 | OH | a2 | B13 | b2 | C23 | c3 | D02 | F |
| 2-1-c3bD02-1-0026 | 617.099 | OH | a1 | B03 | b2 | C19 | c3 | D02 | F |
| 2-1-c3bD02-1-0027 | 617.107 | OH | a1 | B01 | b2 | C21 | c3 | D02 | F |
| 2-1-c3bD02-1-0028 | 618.118 | OH | a1 | B02 | b2 | C21 | c3 | D02 | F |
| 2-1-c3bD02-1-0029 | 618.122 | OH | a1 | B03 | b2 | C27 | c3 | D02 | F |
| 2-1-c3bD02-1-0030 | 619.088 | OH | a2 | B17 | b2 | C19 | c3 | D02 | F |
| 2-1-c3bD02-1-0031 | 619.106 | OH | a2 | B14 | b2 | C23 | c3 | D02 | F |
| 2-1-c3bD02-1-0032 | 619.137 | OH | a2 | B17 | b2 | C27 | c3 | D02 | F |
| 2-1-c3bD02-1-0033 | 620.113 | OH | a1 | B04 | b2 | C19 | c3 | D02 | F |
| 2-1-c3bD02-1-0034 | 620.132 | OH | a1 | B01 | b2 | C23 | c3 | D02 | F |
| 2-1-c3bD02-1-0035 | 621.118 | OH | a1 | B02 | b2 | C23 | c3 | D02 | F |
| 2-1-c3bD02-1-0036 | 621.132 | OH | a1 | B04 | b2 | C27 | c3 | D02 | F |
| 2-1-c3bD02-1-0037 | 625.110 | OH | a2 | B15 | b2 | C21 | c3 | D02 | F |
| 2-1-c3bD02-1-0038 | 626.138 | OH | a2 | B16 | b2 | C21 | c3 | D02 | F |
| 2-1-c3bD02-1-0039 | 627.089 | OH | a2 | B13 | b2 | C25 | c3 | D02 | F |
| 2-1-c3bD02-1-0040 | 628.089 | OH | a2 | B15 | b2 | C23 | c3 | D02 | F |
| 2-1-c3bD02-1-0041 | 629.088 | OH | a1 | B05 | b2 | C19 | c3 | D02 | F |
| 2-1-c3bD02-1-0042 | 629.118 | OH | a1 | B03 | b2 | C21 | c3 | D02 | F |
| 2-1-c3bD02-1-0043 | 630.195 | OH | a2 | B16 | b2 | C23 | c3 | D02 | F |
| 2-1-c3bD02-1-0044 | 630.195 | OH | a2 | B14 | b2 | C25 | c3 | D02 | F |
| 2-1-c3bD02-1-0045 | 631.119 | OH | a1 | B05 | b2 | C27 | c3 | D02 | F |
| 2-1-c3bD02-1-0046 | 631.179 | OH | a1 | B06 | b2 | C19 | c3 | D02 | F |
| 2-1-c3bD02-1-0047 | 631.190 | OH | a1 | B01 | b2 | C25 | c3 | D02 | F |

(continued)

| [Table 8-5-4-2] Compound that may be contained in mixture 2-1-c3bD02-1 | | | | | | | | | |
|---|---|---|---|---|---|---|---|---|---|
| ID | Exact Mass | | a | B | b | C | c | D | d |
| 2-1-c3bD02-1-0048 | 632.174 | OH | a2 | B17 | b2 | C21 | c3 | D02 | F |
| 2-1-c3bD02-1-0049 | 633.104 | OH | a1 | B02 | b2 | C25 | c3 | D02 | F |
| 2-1-c3bD02-1-0050 | 633.133 | OH | a1 | B06 | b2 | C27 | c3 | D02 | F |
| 2-1-c3bD02-1-0051 | 633.133 | OH | a2 | B18 | b2 | C19 | c3 | D02 | F |
| 2-1-c3bD02-1-0052 | 634.170 | OH | a2 | B18 | b2 | C27 | c3 | D02 | F |
| 2-1-c3bD02-1-0053 | 635.083 | OH | a1 | B04 | b2 | C21 | c3 | D02 | F |
| 2-1-c3bD02-1-0054 | 635.083 | OH | a1 | B03 | b2 | C23 | c3 | D02 | F |
| 2-1-c3bD02-1-0055 | 637.127 | OH | a2 | B17 | b2 | C23 | c3 | D02 | F |
| 2-1-c3bD02-1-0056 | 639.078 | OH | a1 | B07 | b2 | C19 | c3 | D02 | F |
| 2-1-c3bD02-1-0057 | 639.105 | OH | a1 | B04 | b2 | C23 | c3 | D02 | F |
| 2-1-c3bD02-1-0058 | 639.148 | OH | a1 | B07 | b2 | C27 | c3 | D02 | F |
| 2-1-c3bD02-1-0059 | 641.109 | OH | a2 | B15 | b2 | C25 | c3 | D02 | F |
| 2-1-c3bD02-1-0060 | 644.082 | OH | a2 | B16 | b2 | C25 | c3 | D02 | F |
| 2-1-c3bD02-1-0061 | 645.064 | OH | a1 | B05 | b2 | C21 | c3 | D02 | F |
| 2-1-c3bD02-1-0062 | 647.094 | OH | a1 | B06 | b2 | C21 | c3 | D02 | F |
| 2-1-c3bD02-1-0063 | 647.094 | OH | a1 | B03 | b2 | C25 | c3 | D02 | F |
| 2-1-c3bD02-1-0064 | 647.149 | OH | a2 | B18 | b2 | C21 | c3 | D02 | F |
| 2-1-c3bD02-1-0065 | 648.169 | OH | a1 | B05 | b2 | C23 | c3 | D02 | F |
| 2-1-c3bD02-1-0066 | 649.128 | OH | a2 | B17 | b2 | C25 | c3 | D02 | F |
| 2-1-c3bD02-1-0067 | 650.128 | OH | a1 | B06 | b2 | C23 | c3 | D02 | F |
| 2-1-c3bD02-1-0068 | 650.146 | OH | a1 | B04 | b2 | C25 | c3 | D02 | F |
| 2-1-c3bD02-1-0069 | 651.159 | OH | a2 | B19 | b2 | C19 | c3 | D02 | F |
| 2-1-c3bD02-1-0070 | 651.199 | OH | a2 | B18 | b2 | C23 | c3 | D02 | F |
| 2-1-c3bD02-1-0071 | 653.138 | OH | a2 | B19 | b2 | C27 | c3 | D02 | F |
| 2-1-c3bD02-1-0072 | 653.153 | OH | a1 | B07 | b2 | C21 | c3 | D02 | F |
| 2-1-c3bD02-1-0073 | 657.083 | OH | a1 | B07 | b2 | C23 | c3 | D02 | F |
| 2-1-c3bD02-1-0074 | 660.045 | OH | a1 | B08 | b2 | C19 | c3 | D02 | F |
| 2-1-c3bD02-1-0075 | 661.041 | OH | a1 | B08 | b2 | C27 | c3 | D02 | F |
| 2-1-c3bD02-1-0076 | 661.110 | OH | a1 | B05 | b2 | C25 | c3 | D02 | F |
| 2-1-c3bD02-1-0077 | 663.075 | OH | a1 | B06 | b2 | C25 | c3 | D02 | F |
| 2-1-c3bD02-1-0078 | 664.09 | OH | a2 | B18 | b2 | C25 | c3 | D02 | F |
| 2-1-c3bD02-1-0079 | 665.094 | OH | a2 | B19 | b2 | C21 | c3 | D02 | F |
| 2-1-c3bD02-1-0080 | 667.160 | OH | a2 | B20 | b2 | C19 | c3 | D02 | F |
| 2-1-c3bD02-1-0081 | 668.099 | OH | a2 | B20 | b2 | C27 | c3 | D02 | F |
| 2-1-c3bD02-1-0082 | 668.105 | OH | a1 | B07 | b2 | C25 | c3 | D02 | F |
| 2-1-c3bD02-1-0083 | 669.194 | OH | a2 | B19 | b2 | C23 | c3 | D02 | F |
| 2-1-c3bD02-1-0084 | 671.064 | OH | a1 | B09 | b2 | C19 | c3 | D02 | F |

(continued)

| [Table 8-5-4-2] Compound that may be contained in mixture 2-1-c3bD02-1 | | | | | | | | | |
|---|---|---|---|---|---|---|---|---|---|
| ID | Exact Mass | | a | B | b | C | c | D | d |
| 2-1-c3bD02-1-0085 | 672.130 | OH | a1 | B09 | b2 | C27 | c3 | D02 | F |
| 2-1-c3bD02-1-0086 | 674.131 | OH | a1 | B08 | b2 | C21 | c3 | D02 | F |
| 2-1-c3bD02-1-0087 | 677.105 | OH | a1 | B08 | b2 | C23 | c3 | D02 | F |
| 2-1-c3bD02-1-0088 | 681.061 | OH | a2 | B20 | b2 | C21 | c3 | D02 | F |
| 2-1-c3bD02-1-0089 | 681.156 | OH | a2 | B19 | b2 | C25 | c3 | D02 | F |
| 2-1-c3bD02-1-0090 | 684.100 | OH | a1 | B09 | b2 | C21 | c3 | D02 | F |
| 2-1-c3bD02-1-0091 | 686.109 | OH | a2 | B20 | b2 | C23 | c3 | D02 | F |
| 2-1-c3bD02-1-0092 | 686.221 | OH | a1 | B10 | b2 | C19 | c3 | D02 | F |
| 2-1-c3bD02-1-0093 | 688.125 | OH | a1 | B10 | b2 | C27 | c3 | D02 | F |
| 2-1-c3bD02-1-0094 | 689.200 | OH | a1 | B09 | b2 | C23 | c3 | D02 | F |
| 2-1-c3bD02-1-0095 | 691.055 | OH | a1 | B08 | b2 | C25 | c3 | D02 | F |
| 2-1-c3bD02-1-0096 | 697.091 | OH | a2 | B20 | b2 | C25 | c3 | D02 | F |
| 2-1-c3bD02-1-0097 | 701.105 | OH | a1 | B10 | b2 | C21 | c3 | D02 | F |
| 2-1-c3bD02-1-0098 | 701.120 | OH | a1 | B09 | b2 | C25 | c3 | D02 | F |
| 2-1-c3bD02-1-0099 | 705.071 | OH | a1 | B10 | b2 | C23 | c3 | D02 | F |
| 2-1-c3bD02-1-0100 | 718.077 | OH | a1 | B10 | b2 | C25 | c3 | D02 | F |

Example 8-6: Preparation of 2-1-E00-0 using step d1

4 types of mixtures described in Table 8-6-1 and DCM (15 mL) were added to a 20 mL column with a filter and shaken at room temperature for 1 hour. The solvent and the suspension of the solid phase were filtered through the filter of the column and washed once with dichloromethane (9 mL) and three times with heptane (9 mL), and the obtained solid phase was dried under reduced pressure to obtain mixture 2-1-E00-0 (2.12 g, amount of the mixture supported on the solid phase: 0.186 mmol/g).

[Table 383]

| [Table 8-6-1] Mixture used in preparation of mixture 2-1-E00-0 | |
|---|---|
| Mixture | Amount used |
| 2-1-c1D01-0 | 750mg |
| 2-1-c2D02-0 | 750mg |
| 2-1-c3aD02-0 | 175 mg |
| 2-1-c3bD02-0 | 175 mg |

Mixture 2-1-E00-0 can be treated by cleavage in the same manner as in Example 8- 1-1 to obtain mixture 2-1-E00-1. In Table 8-6-2, each compound that may be contained in 2- 1-E00-1 is indicated by ID, and the structure is represented by the corresponding combination of core blocks and linkers. The details of the mixture are shown in Table 8-6-2

A notation method in each table will be described. In Table 8-6-2, each compound that may be contained in mixture 2-1-E00-1 is indicated by ID, and core blocks and linkers in combination in the structure of mixture 2-1-E00-1 are indicated by symbols or chemical formulas. The correspondence between the symbols and specific structures are shown in Figure 4. In Table 8-6-2, "×" and "d" are represented by chemical formulas.

[Formula 639]

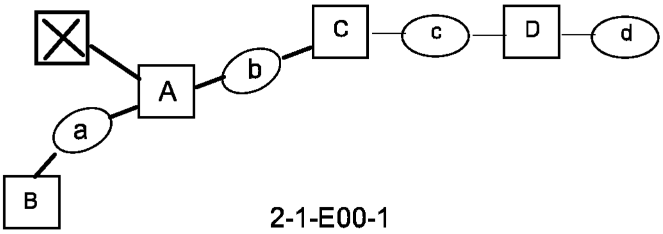

2-1-E00-1

For example, when ID is 2-1-E00-1-0001, the compound is represented by the following structural formula.

[Formula 640]

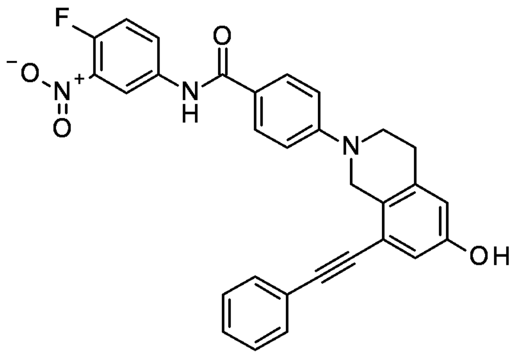

2-1-E00-1-0001

[Table 384]

| Notation in [Table 8-6-2] | | | | | | | | |
|---|---|---|---|---|---|---|---|---|
| ID | | a | B | b | C | c | D | d |
| 2-1-E00-1-0001 | OH | a2 | B11 | b1 | C08 | c1 | D01 | F |

[Table 385]

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0001 | 507.159 | OH | a2 | B11 | b1 | C08 | c1 | D01 | F |
| 2-1-E00-1-0002 | 508.155 | OH | a2 | B12 | b1 | C08 | c1 | D01 | F |
| 2-1-E00-1-0003 | 508.155 | OH | a2 | B11 | b1 | C11 | c1 | D01 | F |
| 2-1-E00-1-0004 | 509.150 | OH | a2 | B11 | b1 | C10 | c1 | D01 | F |
| 2-1-E00-1-0005 | 509.150 | OH | a2 | B12 | b1 | C11 | c1 | D01 | F |
| 2-1-E00-1-0006 | 510.145 | OH | a2 | B12 | b1 | C10 | c1 | D01 | F |
| 2-1-E00-1-0007 | 514.111 | OH | a2 | B11 | b1 | C12 | c1 | D01 | F |
| 2-1-E00-1-0008 | 515.106 | OH | a2 | B12 | b1 | C12 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0009 | 521.175 | OH | a2 | B13 | b1 | C08 | c1 | D01 | F |
| 2-1-E00-1-0010 | 522.170 | OH | a2 | B13 | b1 | C11 | c1 | D01 | F |
| 2-1-E00-1-0011 | 523.166 | OH | a2 | B13 | b1 | C10 | c1 | D01 | F |
| 2-1-E00-1-0012 | 525.134 | OH | a2 | B11 | b2 | C01 | c1 | D01 | F |
| 2-1-E00-1-0013 | 525.150 | OH | a2 | B14 | b1 | C08 | c1 | D01 | F |
| 2-1-E00-1-0014 | 526.129 | OH | a2 | B12 | b2 | C01 | c1 | D01 | F |
| 2-1-E00-1-0015 | 526.145 | OH | a2 | B14 | b1 | C11 | c1 | D01 | F |
| 2-1-E00-1-0016 | 526.165 | OH | a1 | B01 | b1 | C08 | c1 | D01 | F |
| 2-1-E00-1-0017 | 527.141 | OH | a2 | B14 | b1 | C10 | c1 | D01 | F |
| 2-1-E00-1-0018 | 527.160 | OH | a1 | B02 | b1 | C08 | c1 | D01 | F |
| 2-1-E00-1-0019 | 527.160 | OH | a1 | B01 | b1 | C11 | c1 | D01 | F |
| 2-1-E00-1-0020 | 528.127 | OH | a2 | B13 | b1 | C12 | c1 | D01 | F |
| 2-1-E00-1-0021 | 528.156 | OH | a1 | B01 | b1 | C10 | c1 | D01 | F |
| 2-1-E00-1-0022 | 528.156 | OH | a1 | B02 | b1 | C11 | c1 | D01 | F |
| 2-1-E00-1-0023 | 529.151 | OH | a1 | B02 | b1 | C10 | c1 | D01 | F |
| 2-1-E00-1-0024 | 532.102 | OH | a2 | B14 | b1 | C12 | c1 | D01 | F |
| 2-1-E00-1-0025 | 533.117 | OH | a1 | B01 | b1 | C12 | c1 | D01 | F |
| 2-1-E00-1-0026 | 534.112 | OH | a1 | B02 | b1 | C12 | c1 | D01 | F |
| 2-1-E00-1-0027 | 535.154 | OH | a2 | B11 | b2 | C02 | c1 | D01 | F |
| 2-1-E00-1-0028 | 535.191 | OH | a2 | B15 | b1 | C08 | c1 | D01 | F |
| 2-1-E00-1-0029 | 536.150 | OH | a2 | B12 | b2 | C02 | c1 | D01 | F |
| 2-1-E00-1-0030 | 536.150 | OH | a2 | B11 | b2 | C03 | c1 | D01 | F |
| 2-1-E00-1-0031 | 536.186 | OH | a2 | B15 | b1 | C11 | c1 | D01 | F |
| 2-1-E00-1-0032 | 537.145 | OH | a2 | B12 | b2 | C03 | c1 | D01 | F |
| 2-1-E00-1-0033 | 537.170 | OH | a2 | B16 | b1 | C08 | c1 | D01 | F |
| 2-1-E00-1-0034 | 537.181 | OH | a2 | B15 | b1 | C10 | c1 | D01 | F |
| 2-1-E00-1-0035 | 538.165 | OH | a2 | B16 | b1 | C11 | c1 | D01 | F |
| 2-1-E00-1-0036 | 539.149 | OH | a2 | B13 | b2 | C01 | c1 | D01 | F |
| 2-1-E00-1-0037 | 539.160 | OH | a2 | B16 | b1 | C10 | c1 | D01 | F |
| 2-1-E00-1-0038 | 540.181 | OH | a1 | B03 | b1 | C08 | c1 | D01 | F |
| 2-1-E00-1-0039 | 541.111 | OH | a2 | B11 | b2 | C06 | c1 | D01 | F |
| 2-1-E00-1-0040 | 541.176 | OH | a1 | B03 | b1 | C11 | c1 | D01 | F |
| 2-1-E00-1-0041 | 542.106 | OH | a2 | B12 | b2 | C06 | c1 | D01 | F |
| 2-1-E00-1-0042 | 542.142 | OH | a2 | B15 | b1 | C12 | c1 | D01 | F |
| 2-1-E00-1-0043 | 542.171 | OH | a1 | B03 | b1 | C10 | c1 | D01 | F |
| 2-1-E00-1-0044 | 543.124 | OH | a2 | B14 | b2 | C01 | c1 | D01 | F |
| 2-1-E00-1-0045 | 543.141 | OH | a2 | B17 | b1 | C08 | c1 | D01 | F |
| 2-1-E00-1-0046 | 544.122 | OH | a2 | B16 | b1 | C12 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0047 | 544.136 | OH | a2 | B17 | b1 | C11 | c1 | D01 | F |
| 2-1-E00-1-0048 | 544.139 | OH | a1 | B01 | b2 | C01 | c1 | D01 | F |
| 2-1-E00-1-0049 | 544.156 | OH | a1 | B04 | b1 | C08 | c1 | D01 | F |
| 2-1-E00-1-0050 | 545.131 | OH | a2 | B17 | b1 | C10 | c1 | D01 | F |
| 2-1-E00-1-0051 | 545.135 | OH | a1 | B02 | b2 | C01 | c1 | D01 | F |
| 2-1-E00-1-0052 | 545.151 | OH | a1 | B04 | b1 | C11 | c1 | D01 | F |
| 2-1-E00-1-0053 | 546.146 | OH | a1 | B04 | b1 | C10 | c1 | D01 | F |
| 2-1-E00-1-0054 | 547.133 | OH | a1 | B03 | b1 | C12 | c1 | D01 | F |
| 2-1-E00-1-0055 | 549.170 | OH | a2 | B13 | b2 | C02 | c1 | D01 | F |
| 2-1-E00-1-0056 | 550.092 | OH | a2 | B17 | b1 | C12 | c1 | D01 | F |
| 2-1-E00-1-0057 | 550.165 | OH | a2 | B13 | b2 | C03 | c1 | D01 | F |
| 2-1-E00-1-0058 | 550.165 | OH | a2 | B11 | b2 | C05 | c1 | D01 | F |
| 2-1-E00-1-0059 | 551.107 | OH | a1 | B04 | b1 | C12 | c1 | D01 | F |
| 2-1-E00-1-0060 | 551.160 | OH | a2 | B12 | b2 | C05 | c1 | D01 | F |
| 2-1-E00-1-0061 | 553.145 | OH | a2 | B14 | b2 | C02 | c1 | D01 | F |
| 2-1-E00-1-0062 | 553.145 | OH | a2 | B11 | b2 | C04 | c1 | D01 | F |
| 2-1-E00-1-0063 | 553.165 | OH | a2 | B15 | b2 | C01 | c1 | D01 | F |
| 2-1-E00-1-0064 | 554.140 | OH | a2 | B12 | b2 | C04 | c1 | D01 | F |
| 2-1-E00-1-0065 | 554.140 | OH | a2 | B14 | b2 | C03 | c1 | D01 | F |
| 2-1-E00-1-0066 | 554.160 | OH | a1 | B01 | b2 | C02 | c1 | D01 | F |
| 2-1-E00-1-0067 | 554.197 | OH | a1 | B05 | b1 | C08 | c1 | D01 | F |
| 2-1-E00-1-0068 | 555.126 | OH | a2 | B13 | b2 | C06 | c1 | D01 | F |
| 2-1-E00-1-0069 | 555.144 | OH | a2 | B16 | b2 | C01 | c1 | D01 | F |
| 2-1-E00-1-0070 | 555.155 | OH | a1 | B02 | b2 | C02 | c1 | D01 | F |
| 2-1-E00-1-0071 | 555.155 | OH | a1 | B01 | b2 | C03 | c1 | D01 | F |
| 2-1-E00-1-0072 | 555.192 | OH | a1 | B05 | b1 | C11 | c1 | D01 | F |
| 2-1-E00-1-0073 | 556.151 | OH | a1 | B02 | b2 | C03 | c1 | D01 | F |
| 2-1-E00-1-0074 | 556.176 | OH | a1 | B06 | b1 | C08 | c1 | D01 | F |
| 2-1-E00-1-0075 | 556.187 | OH | a1 | B05 | b1 | C10 | c1 | D01 | F |
| 2-1-E00-1-0076 | 557.171 | OH | a1 | B06 | b1 | C11 | c1 | D01 | F |
| 2-1-E00-1-0077 | 557.175 | OH | a2 | B18 | b1 | C08 | c1 | D01 | F |
| 2-1-E00-1-0078 | 558.155 | OH | a1 | B03 | b2 | C01 | c1 | D01 | F |
| 2-1-E00-1-0079 | 558.166 | OH | a1 | B06 | b1 | C10 | c1 | D01 | F |
| 2-1-E00-1-0080 | 558.170 | OH | a2 | B18 | b1 | C11 | c1 | D01 | F |
| 2-1-E00-1-0081 | 559.101 | OH | a2 | B14 | b2 | C06 | c1 | D01 | F |
| 2-1-E00-1-0082 | 559.166 | OH | a2 | B18 | b1 | C10 | c1 | D01 | F |
| 2-1-E00-1-0083 | 559.166 | OH | a2 | B11 | b1 | C13 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0084 | 560.117 | OH | a1 | B01 | b2 | C06 | c1 | D01 | F |
| 2-1-E00-1-0085 | 560.161 | OH | a2 | B12 | b1 | C13 | c1 | D01 | F |
| 2-1-E00-1-0086 | 561.101 | OH | a2 | B11 | b3 | C15 | c1 | D01 | F |
| 2-1-E00-1-0087 | 561.112 | OH | a1 | B02 | b2 | C06 | c1 | D01 | F |
| 2-1-E00-1-0088 | 561.115 | OH | a2 | B17 | b2 | C01 | c1 | D01 | F |
| 2-1-E00-1-0089 | 561.148 | OH | a1 | B05 | b1 | C12 | c1 | D01 | F |
| 2-1-E00-1-0090 | 562.096 | OH | a2 | B12 | b3 | C15 | c1 | D01 | F |
| 2-1-E00-1-0091 | 562.130 | OH | a1 | B04 | b2 | C01 | c1 | D01 | F |
| 2-1-E00-1-0092 | 562.146 | OH | a1 | B07 | b1 | C08 | c1 | D01 | F |
| 2-1-E00-1-0093 | 563.127 | OH | a1 | B06 | b1 | C12 | c1 | D01 | F |
| 2-1-E00-1-0094 | 563.132 | OH | a2 | B11 | b1 | C14 | c1 | D01 | F |
| 2-1-E00-1-0095 | 563.142 | OH | a1 | B07 | b1 | C11 | c1 | D01 | F |
| 2-1-E00-1-0096 | 563.153 | OH | a2 | B11 | b2 | C33 | c3 | D02 | F |
| 2-1-E00-1-0097 | 563.186 | OH | a2 | B15 | b2 | C02 | c1 | D01 | F |
| 2-1-E00-1-0098 | 564.127 | OH | a2 | B18 | b1 | C12 | c1 | D01 | F |
| 2-1-E00-1-0099 | 564.127 | OH | a2 | B12 | b1 | C14 | c1 | D01 | F |
| 2-1-E00-1-0100 | 564.137 | OH | a1 | B07 | b1 | C10 | c1 | D01 | F |
| 2-1-E00-1-0101 | 564.148 | OH | a2 | B12 | b2 | C33 | c3 | D02 | F |
| 2-1-E00-1-0102 | 564.181 | OH | a2 | B15 | b2 | C03 | c1 | D01 | F |
| 2-1-E00-1-0103 | 564.181 | OH | a2 | B13 | b2 | C05 | c1 | D01 | F |
| 2-1-E00-1-0104 | 565.165 | OH | a2 | B16 | b2 | C02 | c1 | D01 | F |
| 2-1-E00-1-0105 | 566.160 | OH | a2 | B16 | b2 | C03 | c1 | D01 | F |
| 2-1-E00-1-0106 | 567.161 | OH | a2 | B13 | b2 | C04 | c1 | D01 | F |
| 2-1-E00-1-0107 | 567.217 | OH | a2 | B11 | b2 | C07 | c1 | D01 | F |
| 2-1-E00-1-0108 | 568.156 | OH | a2 | B14 | b2 | C05 | c1 | D01 | F |
| 2-1-E00-1-0109 | 568.176 | OH | a1 | B03 | b2 | C02 | c1 | D01 | F |
| 2-1-E00-1-0110 | 568.212 | OH | a2 | B12 | b2 | C07 | c1 | D01 | F |
| 2-1-E00-1-0111 | 569.098 | OH | a1 | B07 | b1 | C12 | c1 | D01 | F |
| 2-1-E00-1-0112 | 569.142 | OH | a2 | B15 | b2 | C06 | c1 | D01 | F |
| 2-1-E00-1-0113 | 569.171 | OH | a1 | B03 | b2 | C03 | c1 | D01 | F |
| 2-1-E00-1-0114 | 569.171 | OH | a1 | B01 | b2 | COS | c1 | D01 | F |
| 2-1-E00-1-0115 | 570.166 | OH | a1 | B02 | b2 | C05 | c1 | D01 | F |
| 2-1-E00-1-0116 | 571.121 | OH | a2 | B16 | b2 | C06 | c1 | D01 | F |
| 2-1-E00-1-0117 | 571.121 | OH | a2 | B11 | b3 | C08 | c1 | D01 | F |
| 2-1-E00-1-0118 | 571.121 | OH | a2 | B11 | b1 | C08 | c2 | D02 | F |
| 2-1-E00-1-0119 | 571.121 | OH | a2 | B17 | b2 | C02 | c1 | D01 | F |
| 2-1-E00-1-0120 | 571.136 | OH | a2 | B14 | b2 | C04 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0121 | 571.136 | OH | a2 | B12 | b3 | C08 | c1 | D01 | F |
| 2-1-E00-1-0122 | 572.117 | OH | a2 | B12 | b1 | C08 | c2 | D02 | F |
| 2-1-E00-1-0123 | 572.117 | OH | a2 | B11 | b1 | C11 | c2 | D02 | F |
| 2-1-E00-1-0124 | 572.117 | OH | a2 | B17 | b2 | C03 | c1 | D01 | F |
| 2-1-E00-1-0125 | 572.117 | OH | a1 | B04 | b2 | C02 | c1 | D01 | F |
| 2-1-E00-1-0126 | 572.117 | OH | a1 | B01 | b2 | C04 | c1 | D01 | F |
| 2-1-E00-1-0127 | 572.131 | OH | a1 | B05 | b2 | C01 | c1 | D01 | F |
| 2-1-E00-1-0128 | 572.151 | OH | a2 | B11 | b1 | C10 | c2 | D02 | F |
| 2-1-E00-1-0129 | 572.151 | OH | a2 | B12 | b1 | C11 | c2 | D02 | F |
| 2-1-E00-1-0130 | 572.171 | OH | a1 | B02 | b2 | C04 | c1 | D01 | F |
| 2-1-E00-1-0131 | 573.112 | OH | a1 | B04 | b2 | C03 | c1 | D01 | F |
| 2-1-E00-1-0132 | 573.112 | OH | a2 | B13 | b1 | C13 | c1 | D01 | F |
| 2-1-E00-1-0133 | 573.112 | OH | a2 | B12 | b1 | C10 | c2 | D02 | F |
| 2-1-E00-1-0134 | 573.146 | OH | a1 | B03 | b2 | C06 | c1 | D01 | F |
| 2-1-E00-1-0135 | 573.146 | OH | a1 | B06 | b2 | C01 | c1 | D01 | F |
| 2-1-E00-1-0136 | 573.181 | OH | a2 | B13 | b3 | C15 | c1 | D01 | F |
| 2-1-E00-1-0137 | 574.107 | OH | a2 | B19 | b1 | C08 | c1 | D01 | F |
| 2-1-E00-1-0138 | 574.132 | OH | a2 | B11 | b1 | C09 | c1 | D01 | F |
| 2-1-E00-1-0139 | 574.150 | OH | a2 | B18 | b2 | C01 | c1 | D01 | F |
| 2-1-E00-1-0140 | 575.116 | OH | a2 | B12 | b1 | C09 | c1 | D01 | F |
| 2-1-E00-1-0141 | 575.147 | OH | a2 | B19 | b1 | C11 | c1 | D01 | F |
| 2-1-E00-1-0142 | 575.147 | OH | a2 | B11 | b3 | C06 | c1 | D01 | F |
| 2-1-E00-1-0143 | 575.149 | OH | a2 | B17 | b2 | C06 | c1 | D01 | F |
| 2-1-E00-1-0144 | 576.142 | OH | a2 | B19 | b1 | C10 | c1 | D01 | F |
| 2-1-E00-1-0145 | 576.142 | OH | a2 | B13 | b1 | C14 | c1 | D01 | F |
| 2-1-E00-1-0146 | 577.078 | OH | a2 | B14 | b1 | C13 | c1 | D01 | F |
| 2-1-E00-1-0147 | 577.092 | OH | a2 | B13 | b2 | C33 | c3 | D02 | F |
| 2-1-E00-1-0148 | 577.137 | OH | a2 | B12 | b3 | C06 | c1 | D01 | F |
| 2-1-E00-1-0149 | 577.147 | OH | a2 | B11 | b1 | C12 | c2 | D02 | F |
| 2-1-E00-1-0150 | 577.156 | OH | a1 | B04 | b2 | C06 | c1 | D01 | F |
| 2-1-E00-1-0151 | 577.168 | OH | a1 | B01 | b1 | C13 | c1 | D01 | F |
| 2-1-E00-1-0152 | 578.073 | OH | a2 | B15 | b2 | C05 | c1 | D01 | F |
| 2-1-E00-1-0153 | 578.073 | OH | a2 | B12 | b1 | C12 | c2 | D02 | F |
| 2-1-E00-1-0154 | 578.107 | OH | a2 | B14 | b3 | C15 | c1 | D01 | F |
| 2-1-E00-1-0155 | 578.171 | OH | a1 | B02 | b1 | C13 | c1 | D01 | F |
| 2-1-E00-1-0156 | 578.197 | OH | a1 | B01 | b3 | C15 | c1 | D01 | F |
| 2-1-E00-1-0157 | 579.068 | OH | a1 | B07 | b2 | C01 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0158 | 579.091 | OH | a2 | B16 | b2 | COS | c1 | D01 | F |
| 2-1-E00-1-0159 | 579.167 | OH | a1 | B02 | b3 | C15 | c1 | D01 | F |
| 2-1-E00-1-0160 | 580.106 | OH | a2 | B14 | b1 | C14 | c1 | D01 | F |
| 2-1-E00-1-0161 | 580.121 | OH | a2 | B14 | b2 | C33 | c3 | D02 | F |
| 2-1-E00-1-0162 | 580.176 | OH | a2 | B15 | b2 | C04 | c1 | D01 | F |
| 2-1-E00-1-0163 | 581.102 | OH | a2 | B13 | b2 | C07 | c1 | D01 | F |
| 2-1-E00-1-0164 | 581.122 | OH | a2 | B19 | b1 | C12 | c1 | D01 | F |
| 2-1-E00-1-0165 | 581.143 | OH | a1 | B01 | b1 | C14 | c1 | D01 | F |
| 2-1-E00-1-0166 | 581.176 | OH | a1 | B01 | b2 | C33 | c3 | D02 | F |
| 2-1-E00-1-0167 | 581.233 | OH | a1 | B05 | b2 | C02 | c1 | D01 | F |
| 2-1-E00-1-0168 | 582.098 | OH | a1 | B08 | b1 | C08 | c1 | D01 | F |
| 2-1-E00-1-0169 | 582.137 | OH | a1 | B02 | b1 | C14 | c1 | D01 | F |
| 2-1-E00-1-0170 | 582.158 | OH | a1 | B02 | b2 | C33 | c3 | D02 | F |
| 2-1-E00-1-0171 | 582.191 | OH | a2 | B16 | b2 | C04 | c1 | D01 | F |
| 2-1-E00-1-0172 | 583.133 | OH | a2 | B11 | b1 | C30 | c3 | D02 | F |
| 2-1-E00-1-0173 | 583.133 | OH | a1 | B05 | b2 | C03 | c1 | D01 | F |
| 2-1-E00-1-0174 | 583.154 | OH | a1 | B03 | b2 | C05 | c1 | D01 | F |
| 2-1-E00-1-0175 | 583.155 | OH | a1 | B08 | b1 | C11 | c1 | D01 | F |
| 2-1-E00-1-0176 | 583.158 | OH | a2 | B12 | b1 | C30 | c3 | D02 | F |
| 2-1-E00-1-0177 | 583.187 | OH | a1 | B06 | b2 | C02 | c1 | D01 | F |
| 2-1-E00-1-0178 | 583.187 | OH | a1 | B08 | b1 | C10 | c1 | D01 | F |
| 2-1-E00-1-0179 | 584.128 | OH | a2 | B13 | b3 | C08 | c1 | D01 | F |
| 2-1-E00-1-0180 | 584.153 | OH | a2 | B13 | b1 | C08 | c2 | D02 | F |
| 2-1-E00-1-0181 | 584.171 | OH | a2 | B11 | b2 | C19 | c3 | D02 | F |
| 2-1-E00-1-0182 | 585.123 | OH | a1 | B06 | b2 | C03 | c1 | D01 | F |
| 2-1-E00-1-0183 | 585.137 | OH | a2 | B18 | b2 | C02 | c1 | D01 | F |
| 2-1-E00-1-0184 | 585.137 | OH | a2 | B14 | b2 | C07 | c1 | D01 | F |
| 2-1-E00-1-0185 | 585.137 | OH | a2 | B13 | b1 | C11 | c2 | D02 | F |
| 2-1-E00-1-0186 | 585.137 | OH | a2 | B12 | b2 | C19 | c3 | D02 | F |
| 2-1-E00-1-0187 | 585.166 | OH | a2 | B11 | b2 | C27 | c3 | D02 | F |
| 2-1-E00-1-0188 | 585.170 | OH | a2 | B17 | b2 | COS | c1 | D01 | F |
| 2-1-E00-1-0189 | 585.208 | OH | a2 | B18 | b2 | C03 | c1 | D01 | F |
| 2-1-E00-1-0190 | 586.132 | OH | a1 | B03 | b2 | C04 | c1 | D01 | F |
| 2-1-E00-1-0191 | 586.132 | OH | a1 | B01 | b2 | C07 | c1 | D01 | F |
| 2-1-E00-1-0192 | 586.132 | OH | a2 | B13 | b1 | C10 | c2 | D02 | F |
| 2-1-E00-1-0193 | 586.146 | OH | a2 | B12 | b2 | C27 | c3 | D02 | F |
| 2-1-E00-1-0194 | 586.165 | OH | a1 | B04 | b2 | C05 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0195 | 586.166 | OH | a2 | B15 | b1 | C13 | c1 | D01 | F |
| 2-1-E00-1-0196 | 586.223 | OH | a1 | B02 | b2 | C07 | c1 | D01 | F |
| 2-1-E00-1-0197 | 587.127 | OH | a1 | B05 | b2 | C06 | c1 | D01 | F |
| 2-1-E00-1-0198 | 587.162 | OH | a2 | B11 | b2 | C01 | c2 | D02 | F |
| 2-1-E00-1-0199 | 587.197 | OH | a2 | B14 | b3 | C08 | c1 | D01 | F |
| 2-1-E00-1-0200 | 587.218 | OH | a2 | B14 | b1 | C08 | c2 | D02 | F |
| 2-1-E00-1-0201 | 588.148 | OH | a2 | B17 | b2 | C04 | c1 | D01 | F |
| 2-1-E00-1-0202 | 589.096 | OH | a2 | B15 | b3 | C15 | c1 | D01 | F |
| 2-1-E00-1-0203 | 589.112 | OH | a2 | B13 | b1 | C09 | c1 | D01 | F |
| 2-1-E00-1-0204 | 589.112 | OH | a2 | B16 | b1 | C13 | c1 | D01 | F |
| 2-1-E00-1-0205 | 589.112 | OH | a1 | B08 | b1 | C12 | c1 | D01 | F |
| 2-1-E00-1-0206 | 589.112 | OH | a2 | B12 | b2 | C01 | c2 | D02 | F |
| 2-1-E00-1-0207 | 589.126 | OH | a2 | B14 | b1 | C11 | c2 | D02 | F |
| 2-1-E00-1-0208 | 589.132 | OH | a1 | B06 | b2 | C06 | c1 | D01 | F |
| 2-1-E00-1-0209 | 589.162 | OH | a1 | B01 | b3 | C08 | c1 | D01 | F |
| 2-1-E00-1-0210 | 589.176 | OH | a1 | B01 | b1 | C08 | c2 | D02 | F |
| 2-1-E00-1-0211 | 590.084 | OH | a1 | B07 | b2 | C02 | c1 | D01 | F |
| 2-1-E00-1-0212 | 590.091 | OH | a1 | B04 | b2 | C04 | c1 | D01 | F |
| 2-1-E00-1-0213 | 590.107 | OH | a2 | B13 | b3 | C06 | c1 | D01 | F |
| 2-1-E00-1-0214 | 590.107 | OH | a2 | B14 | b1 | C10 | c2 | D02 | F |
| 2-1-E00-1-0215 | 590.107 | OH | a2 | B16 | b3 | C15 | c1 | D01 | F |
| 2-1-E00-1-0216 | 590.127 | OH | a1 | B02 | b3 | C08 | c1 | D01 | F |
| 2-1-E00-1-0217 | 590.127 | OH | a1 | B02 | b1 | C08 | c2 | D02 | F |
| 2-1-E00-1-0218 | 590.127 | OH | a1 | B01 | b1 | C11 | c2 | D02 | F |
| 2-1-E00-1-0219 | 590.127 | OH | a2 | B18 | b2 | C06 | c1 | D01 | F |
| 2-1-E00-1-0220 | 590.141 | OH | a1 | B07 | b2 | C03 | c1 | D01 | F |
| 2-1-E00-1-0221 | 590.141 | OH | a2 | B20 | b1 | C08 | c1 | D01 | F |
| 2-1-E00-1-0222 | 591.093 | OH | a2 | B15 | b1 | C14 | c1 | D01 | F |
| 2-1-E00-1-0223 | 591.102 | OH | a2 | B15 | b2 | C33 | c3 | D02 | F |
| 2-1-E00-1-0224 | 591.111 | OH | a2 | B13 | b1 | C12 | c2 | D02 | F |
| 2-1-E00-1-0225 | 591.122 | OH | a1 | B01 | b1 | C10 | c2 | D02 | F |
| 2-1-E00-1-0226 | 591.122 | OH | a1 | B02 | b1 | C11 | c2 | D02 | F |
| 2-1-E00-1-0227 | 591.122 | OH | a2 | B20 | b1 | C11 | c1 | D01 | F |
| 2-1-E00-1-0228 | 591.122 | OH | a1 | B03 | b1 | C13 | c1 | D01 | F |
| 2-1-E00-1-0229 | 591.122 | OH | a1 | B02 | b1 | C10 | c2 | D02 | F |
| 2-1-E00-1-0230 | 591.126 | OH | a2 | B19 | b2 | C01 | c1 | D01 | F |
| 2-1-E00-1-0231 | 591.137 | OH | a2 | B20 | b1 | C10 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0232 | 591.142 | OH | a2 | B14 | b1 | C09 | c1 | D01 | F |
| 2-1-E00-1-0233 | 591.163 | OH | a2 | B16 | b1 | C14 | c1 | D01 | F |
| 2-1-E00-1-0234 | 591.184 | OH | a2 | B16 | b2 | C33 | c3 | D02 | F |
| 2-1-E00-1-0235 | 592.089 | OH | a1 | B03 | b3 | C15 | c1 | D01 | F |
| 2-1-E00-1-0236 | 592.118 | OH | a1 | B09 | b1 | C08 | c1 | D01 | F |
| 2-1-E00-1-0237 | 592.118 | OH | a1 | B01 | b1 | C09 | c1 | D01 | F |
| 2-1-E00-1-0238 | 592.118 | OH | a2 | B14 | b3 | C06 | c1 | D01 | F |
| 2-1-E00-1-0239 | 592.137 | OH | a2 | B17 | b1 | C13 | c1 | D01 | F |
| 2-1-E00-1-0240 | 592.187 | OH | a1 | B02 | b1 | C09 | c1 | D01 | F |
| 2-1-E00-1-0241 | 593.113 | OH | a1 | B09 | b1 | C11 | c1 | D01 | F |
| 2-1-E00-1-0242 | 593.121 | OH | a2 | B15 | b2 | C07 | c1 | D01 | F |
| 2-1-E00-1-0243 | 593.132 | OH | a2 | B14 | b1 | C12 | c2 | D02 | F |
| 2-1-E00-1-0244 | 593.137 | OH | a1 | B01 | b3 | C06 | c1 | D01 | F |
| 2-1-E00-1-0245 | 593.142 | OH | a1 | B07 | b2 | C06 | c1 | D01 | F |
| 2-1-E00-1-0246 | 593.163 | OH | a1 | B09 | b1 | C10 | c1 | D01 | F |
| 2-1-E00-1-0247 | 594.122 | OH | a1 | B03 | b1 | C14 | c1 | D01 | F |
| 2-1-E00-1-0248 | 594.153 | OH | a1 | B04 | b1 | C13 | c1 | D01 | F |
| 2-1-E00-1-0249 | 594.153 | OH | a1 | B03 | b2 | C33 | c3 | D02 | F |
| 2-1-E00-1-0250 | 595.068 | OH | a1 | B02 | b3 | C06 | c1 | D01 | F |
| 2-1-E00-1-0251 | 595.147 | OH | a1 | B01 | b1 | C12 | c2 | D02 | F |
| 2-1-E00-1-0252 | 595.148 | OH | a2 | B17 | b3 | C15 | c1 | D01 | F |
| 2-1-E00-1-0253 | 595.148 | OH | a2 | B13 | b1 | C30 | c3 | D02 | F |
| 2-1-E00-1-0254 | 595.248 | OH | a2 | B11 | b1 | C32 | c3 | D02 | F |
| 2-1-E00-1-0255 | 596.064 | OH | a1 | B05 | b2 | COS | c1 | D01 | F |
| 2-1-E00-1-0256 | 596.084 | OH | a2 | B16 | b2 | C07 | c1 | D01 | F |
| 2-1-E00-1-0257 | 596.098 | OH | a1 | B02 | b1 | C12 | c2 | D02 | F |
| 2-1-E00-1-0258 | 596.143 | OH | a2 | B20 | b1 | C12 | c1 | D01 | F |
| 2-1-E00-1-0259 | 596.153 | OH | a1 | B04 | b3 | C15 | c1 | D01 | F |
| 2-1-E00-1-0260 | 596.162 | OH | a2 | B12 | b1 | C32 | c3 | D02 | F |
| 2-1-E00-1-0261 | 596.174 | OH | a2 | B17 | b1 | C14 | c1 | D01 | F |
| 2-1-E00-1-0262 | 597.079 | OH | a2 | B11 | b2 | C02 | c2 | D02 | F |
| 2-1-E00-1-0263 | 597.079 | OH | a2 | B17 | b2 | C33 | c3 | D02 | F |
| 2-1-E00-1-0264 | 597.082 | OH | a2 | B15 | b3 | C08 | c1 | D01 | F |
| 2-1-E00-1-0265 | 597.173 | OH | a2 | B15 | b1 | C08 | c2 | D02 | F |
| 2-1-E00-1-0266 | 597.173 | OH | a2 | B13 | b2 | C19 | c3 | D02 | F |
| 2-1-E00-1-0267 | 597.202 | OH | a2 | B11 | b2 | C21 | c3 | D02 | F |
| 2-1-E00-1-0268 | 597.228 | OH | a1 | B06 | b2 | C05 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0269 | 598.074 | OH | a2 | B12 | b2 | C02 | c2 | D02 | F |
| 2-1-E00-1-0270 | 598.093 | OH | a2 | B11 | b2 | C03 | c2 | D02 | F |
| 2-1-E00-1-0271 | 598.097 | OH | a1 | B04 | b1 | C14 | c1 | D01 | F |
| 2-1-E00-1-0272 | 598.169 | OH | a2 | B15 | b1 | C11 | c2 | D02 | F |
| 2-1-E00-1-0273 | 599.113 | OH | a2 | B12 | b2 | C21 | c3 | D02 | F |
| 2-1-E00-1-0274 | 599.116 | OH | a2 | B13 | b2 | C27 | c3 | D02 | F |
| 2-1-E00-1-0275 | 599.134 | OH | a1 | B04 | b2 | C33 | c3 | D02 | F |
| 2-1-E00-1-0276 | 599.153 | OH | a2 | B18 | b2 | C05 | c1 | D01 | F |
| 2-1-E00-1-0277 | 599.153 | OH | a1 | B05 | b2 | C04 | c1 | D01 | F |
| 2-1-E00-1-0278 | 599.153 | OH | a1 | B03 | b2 | C07 | c1 | D01 | F |
| 2-1-E00-1-0279 | 599.153 | OH | a1 | B09 | b1 | C12 | c1 | D01 | F |
| 2-1-E00-1-0280 | 599.182 | OH | a1 | B08 | b2 | C01 | c1 | D01 | F |
| 2-1-E00-1-0281 | 600.111 | OH | a2 | B12 | b2 | C03 | c2 | D02 | F |
| 2-1-E00-1-0282 | 600.111 | OH | a2 | B16 | b3 | C08 | c1 | D01 | F |
| 2-1-E00-1-0283 | 600.128 | OH | a2 | B16 | b1 | C08 | c2 | D02 | F |
| 2-1-E00-1-0284 | 600.148 | OH | a2 | B15 | b1 | C10 | c2 | D02 | F |
| 2-1-E00-1-0285 | 600.148 | OH | a2 | B14 | b1 | C30 | c3 | D02 | F |
| 2-1-E00-1-0286 | 600.149 | OH | a2 | B16 | b1 | C11 | c2 | D02 | F |
| 2-1-E00-1-0287 | 600.181 | OH | a1 | B06 | b2 | C04 | c1 | D01 | F |
| 2-1-E00-1-0288 | 600.182 | OH | a1 | B01 | b1 | C30 | c3 | D02 | F |
| 2-1-E00-1-0289 | 600.238 | OH | a2 | B13 | b2 | C01 | c2 | D02 | F |
| 2-1-E00-1-0290 | 601.104 | OH | a2 | B16 | b1 | C10 | c2 | D02 | F |
| 2-1-E00-1-0291 | 601.107 | OH | a2 | B14 | b2 | C19 | c3 | D02 | F |
| 2-1-E00-1-0292 | 601.107 | OH | a2 | B11 | b2 | C23 | c3 | D02 | F |
| 2-1-E00-1-0293 | 601.132 | OH | a2 | B19 | b2 | C02 | c1 | D01 | F |
| 2-1-E00-1-0294 | 601.132 | OH | a1 | B02 | b1 | C30 | c3 | D02 | F |
| 2-1-E00-1-0295 | 601.132 | OH | a2 | B18 | b2 | C04 | c1 | D01 | F |
| 2-1-E00-1-0296 | 601.132 | OH | a2 | B15 | b1 | C09 | c1 | D01 | F |
| 2-1-E00-1-0297 | 601.143 | OH | a2 | B17 | b2 | C07 | c1 | D01 | F |
| 2-1-E00-1-0298 | 601.148 | OH | a2 | B12 | b2 | C23 | c3 | D02 | F |
| 2-1-E00-1-0299 | 602.127 | OH | a2 | B14 | b2 | C27 | c3 | D02 | F |
| 2-1-E00-1-0300 | 602.127 | OH | a2 | B19 | b2 | C03 | c1 | D01 | F |
| 2-1-E00-1-0301 | 602.127 | OH | a1 | B03 | b3 | C08 | c1 | D01 | F |
| 2-1-E00-1-0302 | 602.161 | OH | a1 | B03 | b1 | C08 | c2 | D02 | F |
| 2-1-E00-1-0303 | 602.164 | OH | a1 | B01 | b2 | C19 | c3 | D02 | F |
| 2-1-E00-1-0304 | 603.111 | OH | a1 | B04 | b2 | C07 | c1 | D01 | F |
| 2-1-E00-1-0305 | 603.122 | OH | a2 | B11 | b2 | C06 | c2 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0306 | 603.128 | OH | a2 | B15 | b3 | C06 | c1 | D01 | F |
| 2-1-E00-1-0307 | 603.128 | OH | a1 | B03 | b1 | C11 | c2 | D02 | F |
| 2-1-E00-1-0308 | 603.142 | OH | a1 | B02 | b2 | C19 | c3 | D02 | F |
| 2-1-E00-1-0309 | 603.159 | OH | a1 | B01 | b2 | C27 | c3 | D02 | F |
| 2-1-E00-1-0310 | 603.161 | OH | a1 | B07 | b2 | C05 | c1 | D01 | F |
| 2-1-E00-1-0311 | 603.178 | OH | a2 | B16 | b1 | C09 | c1 | D01 | F |
| 2-1-E00-1-0312 | 603.198 | OH | a2 | B12 | b2 | C06 | c2 | D02 | F |
| 2-1-E00-1-0313 | 604.137 | OH | a2 | B15 | b1 | C12 | c2 | D02 | F |
| 2-1-E00-1-0314 | 604.143 | OH | a1 | B03 | b1 | C10 | c2 | D02 | F |
| 2-1-E00-1-0315 | 604.143 | OH | a1 | B02 | b2 | C27 | c3 | D02 | F |
| 2-1-E00-1-0316 | 604.143 | OH | a1 | B05 | b1 | C13 | c1 | D01 | F |
| 2-1-E00-1-0317 | 604.143 | OH | a2 | B14 | b2 | C01 | c2 | D02 | F |
| 2-1-E00-1-0318 | 604.213 | OH | a2 | B16 | b3 | C06 | c1 | D01 | F |
| 2-1-E00-1-0319 | 605.073 | OH | a2 | B17 | b3 | C08 | c1 | D01 | F |
| 2-1-E00-1-0320 | 605.109 | OH | a2 | B17 | b1 | C08 | c2 | D02 | F |
| 2-1-E00-1-0321 | 605.138 | OH | a2 | B16 | b1 | C12 | c2 | D02 | F |
| 2-1-E00-1-0322 | 605.138 | OH | a2 | B17 | b1 | C11 | c2 | D02 | F |
| 2-1-E00-1-0323 | 605.138 | OH | a1 | B01 | b2 | C01 | c2 | D02 | F |
| 2-1-E00-1-0324 | 605.152 | OH | a1 | B04 | b3 | C08 | c1 | D01 | F |
| 2-1-E00-1-0325 | 605.157 | OH | a1 | B04 | b1 | C08 | c2 | D02 | F |
| 2-1-E00-1-0326 | 606.068 | OH | a1 | B07 | b2 | C04 | c1 | D01 | F |
| 2-1-E00-1-0327 | 606.104 | OH | a1 | B05 | b3 | C15 | c1 | D01 | F |
| 2-1-E00-1-0328 | 606.133 | OH | a1 | B03 | b1 | C09 | c1 | D01 | F |
| 2-1-E00-1-0329 | 606.203 | OH | a1 | B06 | b1 | C13 | c1 | D01 | F |
| 2-1-E00-1-0330 | 607.086 | OH | a2 | B17 | b1 | C10 | c2 | D02 | F |
| 2-1-E00-1-0331 | 607.088 | OH | a1 | B02 | b2 | C01 | c2 | D02 | F |
| 2-1-E00-1-0332 | 607.102 | OH | a2 | B19 | b2 | C06 | c1 | D01 | F |
| 2-1-E00-1-0333 | 607.102 | OH | a1 | B04 | b1 | C11 | c2 | D02 | F |
| 2-1-E00-1-0334 | 607.102 | OH | a2 | B20 | b2 | C01 | c1 | D01 | F |
| 2-1-E00-1-0335 | 607.102 | OH | a2 | B18 | b1 | C13 | c1 | D01 | F |
| 2-1-E00-1-0336 | 608.084 | OH | a1 | B03 | b3 | C06 | c1 | D01 | F |
| 2-1-E00-1-0337 | 608.098 | OH | a1 | B04 | b1 | C10 | c2 | D02 | F |
| 2-1-E00-1-0338 | 608.098 | OH | a1 | B06 | b3 | C15 | c1 | D01 | F |
| 2-1-E00-1-0339 | 608.101 | OH | a1 | B10 | b1 | C08 | c1 | D01 | F |
| 2-1-E00-1-0340 | 608.118 | OH | a1 | B05 | b1 | C14 | c1 | D01 | F |
| 2-1-E00-1-0341 | 608.118 | OH | a1 | B05 | b2 | C33 | c3 | D02 | F |
| 2-1-E00-1-0342 | 608.118 | OH | a1 | B03 | b1 | C12 | c2 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0343 | 608.118 | OH | a2 | B18 | b3 | C15 | c1 | D01 | F |
| 2-1-E00-1-0344 | 608.132 | OH | a1 | B08 | b2 | C02 | c1 | D01 | F |
| 2-1-E00-1-0345 | 608.138 | OH | a2 | B17 | b1 | C09 | c1 | D01 | F |
| 2-1-E00-1-0346 | 608.168 | OH | a1 | B10 | b1 | C11 | c1 | D01 | F |
| 2-1-E00-1-0347 | 608.182 | OH | a2 | B15 | b1 | C30 | c3 | D02 | F |
| 2-1-E00-1-0348 | 609.093 | OH | a2 | B11 | b1 | C29 | c3 | D02 | F |
| 2-1-E00-1-0349 | 609.097 | OH | a2 | B13 | b1 | C32 | c3 | D02 | F |
| 2-1-E00-1-0350 | 609.098 | OH | a1 | B08 | b2 | C03 | c1 | D01 | F |
| 2-1-E00-1-0351 | 609.113 | OH | a1 | B09 | b2 | C01 | c1 | D01 | F |
| 2-1-E00-1-0352 | 609.113 | OH | a1 | B10 | b1 | C10 | c1 | D01 | F |
| 2-1-E00-1-0353 | 609.113 | OH | a1 | B04 | b1 | C09 | c1 | D01 | F |
| 2-1-E00-1-0354 | 609.116 | OH | a1 | B06 | b1 | C14 | c1 | D01 | F |
| 2-1-E00-1-0355 | 609.181 | OH | a1 | B06 | b2 | C33 | c3 | D02 | F |
| 2-1-E00-1-0356 | 610.099 | OH | a2 | B12 | b1 | C29 | c3 | D02 | F |
| 2-1-E00-1-0357 | 610.108 | OH | a2 | B17 | b3 | C06 | c1 | D01 | F |
| 2-1-E00-1-0358 | 610.117 | OH | a2 | B13 | b2 | C02 | c2 | D02 | F |
| 2-1-E00-1-0359 | 610.148 | OH | a2 | B18 | b1 | C14 | c1 | D01 | F |
| 2-1-E00-1-0360 | 610.169 | OH | a2 | B15 | b2 | C19 | c3 | D02 | F |
| 2-1-E00-1-0361 | 610.190 | OH | a2 | B13 | b2 | C21 | c3 | D02 | F |
| 2-1-E00-1-0362 | 611.094 | OH | a2 | B16 | b1 | C30 | c3 | D02 | F |
| 2-1-E00-1-0363 | 611.116 | OH | a2 | B18 | b2 | C33 | c3 | D02 | F |
| 2-1-E00-1-0364 | 611.127 | OH | a2 | B17 | b1 | C12 | c2 | D02 | F |
| 2-1-E00-1-0365 | 611.128 | OH | a1 | B04 | b3 | C06 | c1 | D01 | F |
| 2-1-E00-1-0366 | 611.143 | OH | a2 | B13 | b2 | C03 | c2 | D02 | F |
| 2-1-E00-1-0367 | 611.189 | OH | a2 | B11 | b2 | C05 | c2 | D02 | F |
| 2-1-E00-1-0368 | 611.189 | OH | a1 | B07 | b1 | C13 | c1 | D01 | F |
| 2-1-E00-1-0369 | 611.189 | OH | a2 | B15 | b2 | C27 | c3 | D02 | F |
| 2-1-E00-1-0370 | 612.123 | OH | a1 | B05 | b2 | C07 | c1 | D01 | F |
| 2-1-E00-1-0371 | 612.127 | OH | a1 | B04 | b1 | C12 | c2 | D02 | F |
| 2-1-E00-1-0372 | 612.138 | OH | a2 | B12 | b2 | C05 | c2 | D02 | F |
| 2-1-E00-1-0373 | 612.143 | OH | a2 | B16 | b2 | C19 | c3 | D02 | F |
| 2-1-E00-1-0374 | 612.148 | OH | a2 | B11 | b2 | C25 | c3 | D02 | F |
| 2-1-E00-1-0375 | 612.169 | OH | a2 | B14 | b1 | C32 | c3 | D02 | F |
| 2-1-E00-1-0376 | 612.184 | OH | a1 | B07 | b3 | C15 | c1 | D01 | F |
| 2-1-E00-1-0377 | 613.059 | OH | a2 | B12 | b2 | C25 | c3 | D02 | F |
| 2-1-E00-1-0378 | 613.132 | OH | a2 | B16 | b2 | C27 | c3 | D02 | F |
| 2-1-E00-1-0379 | 613.147 | OH | a1 | B03 | b1 | C30 | c3 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0380 | 613.168 | OH | a1 | B01 | b1 | C32 | c3 | D02 | F |
| 2-1-E00-1-0381 | 613.168 | OH | a1 | B06 | b2 | C07 | c1 | D01 | F |
| 2-1-E00-1-0382 | 613.168 | OH | a1 | B08 | b2 | C06 | c1 | D01 | F |
| 2-1-E00-1-0383 | 614.054 | OH | a1 | B10 | b1 | C12 | c1 | D01 | F |
| 2-1-E00-1-0384 | 614.074 | OH | a2 | B14 | b2 | C02 | c2 | D02 | F |
| 2-1-E00-1-0385 | 614.127 | OH | a2 | B11 | b2 | C04 | c2 | D02 | F |
| 2-1-E00-1-0386 | 614.127 | OH | a2 | B15 | b2 | C01 | c2 | D02 | F |
| 2-1-E00-1-0387 | 614.153 | OH | a2 | B14 | b2 | C21 | c3 | D02 | F |
| 2-1-E00-1-0388 | 614.254 | OH | a2 | B13 | b2 | C23 | c3 | D02 | F |
| 2-1-E00-1-0389 | 615.069 | OH | a1 | B02 | b1 | C32 | c3 | D02 | F |
| 2-1-E00-1-0390 | 615.122 | OH | a2 | B18 | b2 | C07 | c1 | D01 | F |
| 2-1-E00-1-0391 | 615.148 | OH | a2 | B12 | b2 | C04 | c2 | D02 | F |
| 2-1-E00-1-0392 | 615.148 | OH | a2 | B14 | b2 | C03 | c2 | D02 | F |
| 2-1-E00-1-0393 | 615.164 | OH | a1 | B07 | b1 | C14 | c1 | D01 | F |
| 2-1-E00-1-0394 | 616.088 | OH | a1 | B01 | b2 | C02 | c2 | D02 | F |
| 2-1-E00-1-0395 | 616.179 | OH | a1 | B07 | b2 | C33 | c3 | D02 | F |
| 2-1-E00-1-0396 | 616.179 | OH | a2 | B19 | b2 | C05 | c1 | D01 | F |
| 2-1-E00-1-0397 | 616.233 | OH | a1 | B05 | b3 | C08 | c1 | D01 | F |
| 2-1-E00-1-0398 | 617.084 | OH | a1 | B05 | b1 | C08 | c2 | D02 | F |
| 2-1-E00-1-0399 | 617.099 | OH | a1 | B03 | b2 | C19 | c3 | D02 | F |
| 2-1-E00-1-0400 | 617.107 | OH | a1 | B01 | b2 | C21 | c3 | D02 | F |
| 2-1-E00-1-0401 | 617.107 | OH | a2 | B13 | b2 | C06 | c2 | D02 | F |
| 2-1-E00-1-0402 | 617.127 | OH | a2 | B16 | b2 | C01 | c2 | D02 | F |
| 2-1-E00-1-0403 | 617.143 | OH | a1 | B02 | b2 | C02 | c2 | D02 | F |
| 2-1-E00-1-0404 | 617.174 | OH | a1 | B01 | b2 | C03 | c2 | D02 | F |
| 2-1-E00-1-0405 | 617.233 | OH | a2 | B20 | b2 | C02 | c1 | D01 | F |
| 2-1-E00-1-0406 | 618.102 | OH | a2 | B17 | b1 | C30 | c3 | D02 | F |
| 2-1-E00-1-0407 | 618.102 | OH | a1 | B05 | b1 | C11 | c2 | D02 | F |
| 2-1-E00-1-0408 | 618.118 | OH | a1 | B02 | b2 | C21 | c3 | D02 | F |
| 2-1-E00-1-0409 | 618.122 | OH | a1 | B03 | b2 | C27 | c3 | D02 | F |
| 2-1-E00-1-0410 | 618.140 | OH | a1 | B02 | b2 | C03 | c2 | D02 | F |
| 2-1-E00-1-0411 | 618.153 | OH | a2 | B20 | b2 | C03 | c1 | D01 | F |
| 2-1-E00-1-0412 | 618.158 | OH | a1 | B06 | b3 | C08 | c1 | D01 | F |
| 2-1-E00-1-0413 | 618.158 | OH | a1 | B06 | b1 | C08 | c2 | D02 | F |
| 2-1-E00-1-0414 | 618.158 | OH | a1 | B05 | b1 | C10 | c2 | D02 | F |
| 2-1-E00-1-0415 | 618.158 | OH | a1 | B04 | b1 | C30 | c3 | D02 | F |
| 2-1-E00-1-0416 | 619.088 | OH | a2 | B17 | b2 | C19 | c3 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0417 | 619.106 | OH | a2 | B14 | b2 | C23 | c3 | D02 | F |
| 2-1-E00-1-0418 | 619.117 | OH | a2 | B19 | b2 | C04 | c1 | D01 | F |
| 2-1-E00-1-0419 | 619.117 | OH | a1 | B06 | b1 | C11 | c2 | D02 | F |
| 2-1-E00-1-0420 | 619.122 | OH | a2 | B18 | b3 | C08 | c1 | D01 | F |
| 2-1-E00-1-0421 | 619.122 | OH | a2 | B18 | b1 | C08 | c2 | D02 | F |
| 2-1-E00-1-0422 | 619.137 | OH | a2 | B17 | b2 | C27 | c3 | D02 | F |
| 2-1-E00-1-0423 | 619.139 | OH | a1 | B03 | b2 | C01 | c2 | D02 | F |
| 2-1-E00-1-0424 | 619.154 | OH | a1 | B06 | b1 | C10 | c2 | D02 | F |
| 2-1-E00-1-0425 | 619.154 | OH | a2 | B18 | b1 | C11 | c2 | D02 | F |
| 2-1-E00-1-0426 | 620.113 | OH | a1 | B04 | b2 | C19 | c3 | D02 | F |
| 2-1-E00-1-0427 | 620.132 | OH | a1 | B01 | b2 | C23 | c3 | D02 | F |
| 2-1-E00-1-0428 | 620.138 | OH | a1 | B09 | b2 | C02 | c1 | D01 | F |
| 2-1-E00-1-0429 | 620.138 | OH | a1 | B05 | b1 | C09 | c1 | D01 | F |
| 2-1-E00-1-0430 | 620.138 | OH | a1 | B07 | b2 | C07 | c1 | D01 | F |
| 2-1-E00-1-0431 | 620.138 | OH | a2 | B14 | b2 | C06 | c2 | D02 | F |
| 2-1-E00-1-0432 | 620.149 | OH | a2 | B18 | b1 | C10 | c2 | D02 | F |
| 2-1-E00-1-0433 | 620.154 | OH | a2 | B11 | b1 | C13 | c2 | D02 | F |
| 2-1-E00-1-0434 | 621.118 | OH | a1 | B02 | b2 | C23 | c3 | D02 | F |
| 2-1-E00-1-0435 | 621.132 | OH | a1 | B04 | b2 | C27 | c3 | D02 | F |
| 2-1-E00-1-0436 | 621.133 | OH | a1 | B09 | b2 | C03 | c1 | D01 | F |
| 2-1-E00-1-0437 | 621.133 | OH | a1 | B01 | b2 | C06 | c2 | D02 | F |
| 2-1-E00-1-0438 | 621.133 | OH | a1 | B05 | b3 | C06 | c1 | D01 | F |
| 2-1-E00-1-0439 | 621.137 | OH | a2 | B12 | b1 | C13 | c2 | D02 | F |
| 2-1-E00-1-0440 | 621.137 | OH | a1 | B06 | b1 | C09 | c1 | D01 | F |
| 2-1-E00-1-0441 | 621.137 | OH | a2 | B11 | b3 | C15 | c2 | D02 | F |
| 2-1-E00-1-0442 | 622.117 | OH | a1 | B02 | b2 | C06 | c2 | D02 | F |
| 2-1-E00-1-0443 | 622.128 | OH | a2 | B17 | b2 | C01 | c2 | D02 | F |
| 2-1-E00-1-0444 | 622.132 | OH | a2 | B20 | b2 | C06 | c1 | D01 | F |
| 2-1-E00-1-0445 | 622.132 | OH | a1 | B05 | b1 | C12 | c2 | D02 | F |
| 2-1-E00-1-0446 | 622.133 | OH | a2 | B11 | b3 | C16 | c1 | D01 | F |
| 2-1-E00-1-0447 | 622.133 | OH | a2 | B18 | b1 | C09 | c1 | D01 | F |
| 2-1-E00-1-0448 | 622.148 | OH | a2 | B13 | b1 | C29 | c3 | D02 | F |
| 2-1-E00-1-0449 | 622.184 | OH | a2 | B15 | b1 | C32 | c3 | D02 | F |
| 2-1-E00-1-0450 | 622.204 | OH | a2 | B12 | b3 | C15 | c2 | D02 | F |
| 2-1-E00-1-0451 | 623.063 | OH | a1 | B04 | b2 | C01 | c2 | D02 | F |
| 2-1-E00-1-0452 | 623.127 | OH | a1 | B06 | b3 | C06 | c1 | D01 | F |
| 2-1-E00-1-0453 | 623.127 | OH | a1 | B07 | b3 | C08 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0454 | 623.143 | OH | a1 | B07 | b1 | C08 | c2 | D02 | F |
| 2-1-E00-1-0455 | 624.078 | OH | a2 | B12 | b3 | C16 | c1 | D01 | F |
| 2-1-E00-1-0456 | 624.115 | OH | a1 | B08 | b2 | C05 | c1 | D01 | F |
| 2-1-E00-1-0457 | 624.123 | OH | a1 | B06 | b1 | C12 | c2 | D02 | F |
| 2-1-E00-1-0458 | 624.163 | OH | a2 | B18 | b3 | C06 | c1 | D01 | F |
| 2-1-E00-1-0459 | 625.062 | OH | a2 | B11 | b3 | C17 | c1 | D01 | F |
| 2-1-E00-1-0460 | 625.074 | OH | a2 | B11 | b1 | C14 | c2 | D02 | F |
| 2-1-E00-1-0461 | 625.077 | OH | a1 | B07 | b1 | C11 | c2 | D02 | F |
| 2-1-E00-1-0462 | 625.093 | OH | a2 | B15 | b2 | C02 | c2 | D02 | F |
| 2-1-E00-1-0463 | 625.093 | OH | a2 | B19 | b1 | C13 | c1 | D01 | F |
| 2-1-E00-1-0464 | 625.110 | OH | a2 | B15 | b2 | C21 | c3 | D02 | F |
| 2-1-E00-1-0465 | 625.132 | OH | a2 | B16 | b1 | C32 | c3 | D02 | F |
| 2-1-E00-1-0466 | 625.162 | OH | a2 | B12 | b3 | C17 | c1 | D01 | F |
| 2-1-E00-1-0467 | 625.205 | OH | a2 | B18 | b1 | C12 | c2 | D02 | F |
| 2-1-E00-1-0468 | 625.205 | OH | a2 | B12 | b1 | C14 | c2 | D02 | F |
| 2-1-E00-1-0469 | 626.058 | OH | a1 | B07 | b1 | C10 | c2 | D02 | F |
| 2-1-E00-1-0470 | 626.092 | OH | a1 | B09 | b2 | C06 | c1 | D01 | F |
| 2-1-E00-1-0471 | 626.094 | OH | a1 | B10 | b2 | C01 | c1 | D01 | F |
| 2-1-E00-1-0472 | 626.108 | OH | a2 | B15 | b2 | C03 | c2 | D02 | F |
| 2-1-E00-1-0473 | 626.108 | OH | a2 | B13 | b2 | C05 | c2 | D02 | F |
| 2-1-E00-1-0474 | 626.108 | OH | a2 | B19 | b3 | C15 | c1 | D01 | F |
| 2-1-E00-1-0475 | 626.108 | OH | a1 | B08 | b2 | C04 | c1 | D01 | F |
| 2-1-E00-1-0476 | 626.127 | OH | a2 | B16 | b2 | C02 | c2 | D02 | F |
| 2-1-E00-1-0477 | 626.138 | OH | a2 | B16 | b2 | C21 | c3 | D02 | F |
| 2-1-E00-1-0478 | 627.089 | OH | a2 | B13 | b2 | C25 | c3 | D02 | F |
| 2-1-E00-1-0479 | 627.093 | OH | a2 | B14 | b1 | C29 | c3 | D02 | F |
| 2-1-E00-1-0480 | 627.093 | OH | a2 | B16 | b2 | C03 | c2 | D02 | F |
| 2-1-E00-1-0481 | 627.093 | OH | a1 | B07 | b1 | C09 | c1 | D01 | F |
| 2-1-E00-1-0482 | 627.104 | OH | a1 | B05 | b1 | C30 | c3 | D02 | F |
| 2-1-E00-1-0483 | 627.104 | OH | a1 | B01 | b1 | C29 | c3 | D02 | F |
| 2-1-E00-1-0484 | 627.148 | OH | a1 | B03 | b1 | C32 | c3 | D02 | F |
| 2-1-E00-1-0485 | 627.153 | OH | a2 | B19 | b1 | C14 | c1 | D01 | F |
| 2-1-E00-1-0486 | 627.184 | OH | a2 | B13 | b2 | C04 | c2 | D02 | F |
| 2-1-E00-1-0487 | 628.089 | OH | a2 | B19 | b2 | C33 | c3 | D02 | F |
| 2-1-E00-1-0488 | 628.089 | OH | a2 | B15 | b2 | C23 | c3 | D02 | F |
| 2-1-E00-1-0489 | 628.089 | OH | a2 | B11 | b2 | C07 | c2 | D02 | F |
| 2-1-E00-1-0490 | 628.099 | OH | a1 | B02 | b1 | C29 | c3 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0491 | 628.104 | OH | a1 | B07 | b3 | C06 | c1 | D01 | F |
| 2-1-E00-1-0492 | 628.122 | OH | a2 | B14 | b2 | C05 | c2 | D02 | F |
| 2-1-E00-1-0493 | 628.143 | OH | a1 | B03 | b2 | C02 | c2 | D02 | F |
| 2-1-E00-1-0494 | 628.143 | OH | a2 | B12 | b2 | C07 | c2 | D02 | F |
| 2-1-E00-1-0495 | 629.088 | OH | a1 | B05 | b2 | C19 | c3 | D02 | F |
| 2-1-E00-1-0496 | 629.118 | OH | a1 | B03 | b2 | C21 | c3 | D02 | F |
| 2-1-E00-1-0497 | 629.127 | OH | a1 | B06 | b1 | C30 | c3 | D02 | F |
| 2-1-E00-1-0498 | 629.163 | OH | a1 | B07 | b1 | C12 | c2 | D02 | F |
| 2-1-E00-1-0499 | 629.18 | OH | a2 | B15 | b2 | C06 | c2 | D02 | F |
| 2-1-E00-1-0500 | 630.122 | OH | a1 | B03 | b2 | C03 | c2 | D02 | F |
| 2-1-E00-1-0501 | 630.134 | OH | a1 | B01 | b2 | C05 | c2 | D02 | F |
| 2-1-E00-1-0502 | 630.195 | OH | a2 | B16 | b2 | C23 | c3 | D02 | F |
| 2-1-E00-1-0503 | 630.195 | OH | a2 | B14 | b2 | C25 | c3 | D02 | F |
| 2-1-E00-1-0504 | 630.195 | OH | a2 | B17 | b1 | C32 | c3 | D02 | F |
| 2-1-E00-1-0505 | 631.119 | OH | a1 | B05 | b2 | C27 | c3 | D02 | F |
| 2-1-E00-1-0506 | 631.122 | OH | a2 | B18 | b1 | C30 | c3 | D02 | F |
| 2-1-E00-1-0507 | 631.140 | OH | a1 | B02 | b2 | C05 | c2 | D02 | F |
| 2-1-E00-1-0508 | 631.142 | OH | a2 | B20 | b2 | C05 | c1 | D01 | F |
| 2-1-E00-1-0509 | 631.179 | OH | a1 | B06 | b2 | C19 | c3 | D02 | F |
| 2-1-E00-1-0510 | 631.190 | OH | a1 | B01 | b2 | C25 | c3 | D02 | F |
| 2-1-E00-1-0511 | 632.065 | OH | a1 | B04 | b1 | C32 | c3 | D02 | F |
| 2-1-E00-1-0512 | 632.118 | OH | a2 | B16 | b2 | C06 | c2 | D02 | F |
| 2-1-E00-1-0513 | 632.138 | OH | a2 | B11 | b3 | C08 | c2 | D02 | F |
| 2-1-E00-1-0514 | 632.174 | OH | a2 | B17 | b2 | C02 | c2 | D02 | F |
| 2-1-E00-1-0515 | 632.174 | OH | a2 | B14 | b2 | C04 | c2 | D02 | F |
| 2-1-E00-1-0516 | 632.174 | OH | a2 | B17 | b2 | C21 | c3 | D02 | F |
| 2-1-E00-1-0517 | 633.060 | OH | a1 | B08 | b1 | C13 | c1 | D01 | F |
| 2-1-E00-1-0518 | 633.104 | OH | a1 | B02 | b2 | C25 | c3 | D02 | F |
| 2-1-E00-1-0519 | 633.133 | OH | a1 | B06 | b2 | C27 | c3 | D02 | F |
| 2-1-E00-1-0520 | 633.133 | OH | a2 | B18 | b2 | C19 | c3 | D02 | F |
| 2-1-E00-1-0521 | 633.155 | OH | a2 | B19 | b2 | C07 | c1 | D01 | F |
| 2-1-E00-1-0522 | 633.173 | OH | a2 | B12 | b3 | C08 | c2 | D02 | F |
| 2-1-E00-1-0523 | 634.128 | OH | a2 | B17 | b2 | C03 | c2 | D02 | F |
| 2-1-E00-1-0524 | 634.148 | OH | a1 | B04 | b2 | C02 | c2 | D02 | F |
| 2-1-E00-1-0525 | 634.153 | OH | a1 | B01 | b2 | C04 | c2 | D02 | F |
| 2-1-E00-1-0526 | 634.153 | OH | a1 | B05 | b2 | C01 | c2 | D02 | F |
| 2-1-E00-1-0527 | 634.170 | OH | a2 | B18 | b2 | C27 | c3 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0528 | 635.083 | OH | a1 | B04 | b2 | C21 | c3 | D02 | F |
| 2-1-E00-1-0529 | 635.083 | OH | a1 | B03 | b2 | C23 | c3 | D02 | F |
| 2-1-E00-1-0530 | 635.097 | OH | a1 | B08 | b3 | C15 | c1 | D01 | F |
| 2-1-E00-1-0531 | 635.097 | OH | a1 | B02 | b2 | C04 | c2 | D02 | F |
| 2-1-E00-1-0532 | 635.139 | OH | a1 | B04 | b2 | C03 | c2 | D02 | F |
| 2-1-E00-1-0533 | 635.153 | OH | a2 | B20 | b2 | C04 | c1 | D01 | F |
| 2-1-E00-1-0534 | 635.204 | OH | a2 | B13 | b1 | C13 | c2 | D02 | F |
| 2-1-E00-1-0535 | 636.078 | OH | a1 | B09 | b2 | C05 | c1 | D01 | F |
| 2-1-E00-1-0536 | 636.093 | OH | a1 | B03 | b2 | C06 | c2 | D02 | F |
| 2-1-E00-1-0537 | 636.113 | OH | a1 | B06 | b2 | C01 | c2 | D02 | F |
| 2-1-E00-1-0538 | 636.113 | OH | a1 | B10 | b2 | C02 | c1 | D01 | F |
| 2-1-E00-1-0539 | 636.133 | OH | a1 | B07 | b1 | C30 | c3 | D02 | F |
| 2-1-E00-1-0540 | 636.149 | OH | a2 | B13 | b3 | C15 | c2 | D02 | F |
| 2-1-E00-1-0541 | 637.074 | OH | a1 | B08 | b1 | C14 | c1 | D01 | F |
| 2-1-E00-1-0542 | 637.108 | OH | a2 | B19 | b3 | C08 | c1 | D01 | F |
| 2-1-E00-1-0543 | 637.108 | OH | a2 | B19 | b1 | C08 | c2 | D02 | F |
| 2-1-E00-1-0544 | 637.113 | OH | a2 | B11 | b1 | C09 | c2 | D02 | F |
| 2-1-E00-1-0545 | 637.127 | OH | a2 | B17 | b2 | C23 | c3 | D02 | F |
| 2-1-E00-1-0546 | 637.143 | OH | a2 | B18 | b2 | C01 | c2 | D02 | F |
| 2-1-E00-1-0547 | 637.158 | OH | a1 | B08 | b2 | C33 | c3 | D02 | F |
| 2-1-E00-1-0548 | 638.094 | OH | a1 | B10 | b2 | C03 | c1 | D01 | F |
| 2-1-E00-1-0549 | 638.112 | OH | a2 | B13 | b3 | C16 | c1 | D01 | F |
| 2-1-E00-1-0550 | 638.128 | OH | a2 | B11 | b1 | C31 | c3 | D02 | F |
| 2-1-E00-1-0551 | 638.128 | OH | a2 | B15 | b1 | C29 | c3 | D02 | F |
| 2-1-E00-1-0552 | 638.142 | OH | a2 | B12 | b1 | C09 | c2 | D02 | F |
| 2-1-E00-1-0553 | 638.145 | OH | a2 | B19 | b1 | C11 | c2 | D02 | F |
| 2-1-E00-1-0554 | 639.078 | OH | a1 | B07 | b2 | C19 | c3 | D02 | F |
| 2-1-E00-1-0555 | 639.105 | OH | a1 | B04 | b2 | C23 | c3 | D02 | F |
| 2-1-E00-1-0556 | 639.109 | OH | a1 | B09 | b2 | C04 | c1 | D01 | F |
| 2-1-E00-1-0557 | 639.109 | OH | a2 | B12 | b1 | C31 | c3 | D02 | F |
| 2-1-E00-1-0558 | 639.109 | OH | a2 | B11 | b3 | C06 | c2 | D02 | F |
| 2-1-E00-1-0559 | 639.109 | OH | a2 | B17 | b2 | C06 | c2 | D02 | F |
| 2-1-E00-1-0560 | 639.111 | OH | a2 | B19 | b1 | C10 | c2 | D02 | F |
| 2-1-E00-1-0561 | 639.126 | OH | a2 | B13 | b3 | C17 | c1 | D01 | F |
| 2-1-E00-1-0562 | 639.128 | OH | a2 | B13 | b1 | C14 | c2 | D02 | F |
| 2-1-E00-1-0563 | 639.138 | OH | a2 | B14 | b1 | C13 | c2 | D02 | F |
| 2-1-E00-1-0564 | 639.148 | OH | a1 | B07 | b2 | C27 | c3 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0565 | 639.184 | OH | a2 | B16 | b1 | C29 | c3 | D02 | F |
| 2-1-E00-1-0566 | 639.220 | OH | a2 | B12 | b3 | C06 | c2 | D02 | F |
| 2-1-E00-1-0567 | 640.104 | OH | a1 | B04 | b2 | C06 | c2 | D02 | F |
| 2-1-E00-1-0568 | 640.104 | OH | a2 | B11 | b3 | C18 | c1 | D01 | F |
| 2-1-E00-1-0569 | 640.104 | OH | a1 | B01 | b1 | C13 | c2 | D02 | F |
| 2-1-E00-1-0570 | 640.124 | OH | a2 | B15 | b2 | C05 | c2 | D02 | F |
| 2-1-E00-1-0571 | 640.138 | OH | a2 | B14 | b3 | C15 | c2 | D02 | F |
| 2-1-E00-1-0572 | 640.179 | OH | a2 | B12 | b3 | C18 | c1 | D01 | F |
| 2-1-E00-1-0573 | 641.040 | OH | a2 | B14 | b3 | C16 | c1 | D01 | F |
| 2-1-E00-1-0574 | 641.054 | OH | a1 | B02 | b1 | C13 | c2 | D02 | F |
| 2-1-E00-1-0575 | 641.099 | OH | a2 | B19 | b1 | C09 | c1 | D01 | F |
| 2-1-E00-1-0576 | 641.109 | OH | a2 | B20 | b1 | C13 | c1 | D01 | F |
| 2-1-E00-1-0577 | 641.109 | OH | a2 | B15 | b2 | C25 | c3 | D02 | F |
| 2-1-E00-1-0578 | 641.118 | OH | a1 | B08 | b2 | C07 | c1 | D01 | F |
| 2-1-E00-1-0579 | 641.200 | OH | a1 | B01 | b3 | C15 | c2 | D02 | F |
| 2-1-E00-1-0580 | 642.035 | OH | a1 | B07 | b2 | C01 | c2 | D02 | F |
| 2-1-E00-1-0581 | 642.069 | OH | a1 | B10 | b2 | C06 | c1 | D01 | F |
| 2-1-E00-1-0582 | 642.104 | OH | a1 | B01 | b3 | C16 | c1 | D01 | F |
| 2-1-E00-1-0583 | 642.133 | OH | a2 | B16 | b2 | C05 | c2 | D02 | F |
| 2-1-E00-1-0584 | 642.158 | OH | a1 | B03 | b1 | C29 | c3 | D02 | F |
| 2-1-E00-1-0585 | 643.053 | OH | a1 | B05 | b1 | C32 | c3 | D02 | F |
| 2-1-E00-1-0586 | 643.100 | OH | a1 | B02 | b3 | C15 | c2 | D02 | F |
| 2-1-E00-1-0587 | 643.122 | OH | a2 | B19 | b3 | C06 | c1 | D01 | F |
| 2-1-E00-1-0588 | 643.129 | OH | a2 | B20 | b3 | C15 | c1 | D01 | F |
| 2-1-E00-1-0589 | 643.134 | OH | a2 | B14 | b3 | C17 | c1 | D01 | F |
| 2-1-E00-1-0590 | 643.148 | OH | a2 | B14 | b1 | C14 | c2 | D02 | F |
| 2-1-E00-1-0591 | 643.190 | OH | a1 | B02 | b3 | C16 | c1 | D01 | F |
| 2-1-E00-1-0592 | 644.068 | OH | a2 | B15 | b2 | C04 | c2 | D02 | F |
| 2-1-E00-1-0593 | 644.082 | OH | a2 | B16 | b2 | C25 | c3 | D02 | F |
| 2-1-E00-1-0594 | 644.099 | OH | a2 | B13 | b2 | C07 | c2 | D02 | F |
| 2-1-E00-1-0595 | 644.099 | OH | a2 | B19 | b1 | C12 | c2 | D02 | F |
| 2-1-E00-1-0596 | 644.138 | OH | a1 | B01 | b3 | C17 | c1 | D01 | F |
| 2-1-E00-1-0597 | 644.138 | OH | a1 | B01 | b1 | C14 | c2 | D02 | F |
| 2-1-E00-1-0598 | 644.210 | OH | a1 | B05 | b2 | C02 | c2 | D02 | F |
| 2-1-E00-1-0599 | 644.210 | OH | a1 | B09 | b1 | C13 | c1 | D01 | F |
| 2-1-E00-1-0600 | 645.064 | OH | a1 | B05 | b2 | C21 | c3 | D02 | F |
| 2-1-E00-1-0601 | 645.065 | OH | a1 | B06 | b1 | C32 | c3 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0602 | 645.083 | OH | a1 | B02 | b3 | C17 | c1 | D01 | F |
| 2-1-E00-1-0603 | 645.084 | OH | a1 | B08 | b3 | C08 | c1 | D01 | F |
| 2-1-E00-1-0604 | 645.084 | OH | a1 | B08 | b1 | C08 | c2 | D02 | F |
| 2-1-E00-1-0605 | 645.133 | OH | a1 | B02 | b1 | C14 | c2 | D02 | F |
| 2-1-E00-1-0606 | 645.138 | OH | a2 | B20 | b1 | C14 | c1 | D01 | F |
| 2-1-E00-1-0607 | 645.194 | OH | a2 | B16 | b2 | C04 | c2 | D02 | F |
| 2-1-E00-1-0608 | 646.060 | OH | a2 | B20 | b2 | C33 | c3 | D02 | F |
| 2-1-E00-1-0609 | 646.099 | OH | a1 | B05 | b2 | C03 | c2 | D02 | F |
| 2-1-E00-1-0610 | 646.099 | OH | a1 | B03 | b2 | C05 | c2 | D02 | F |
| 2-1-E00-1-0611 | 646.153 | OH | a2 | B17 | b1 | C29 | c3 | D02 | F |
| 2-1-E00-1-0612 | 646.159 | OH | a2 | B18 | b1 | C32 | c3 | D02 | F |
| 2-1-E00-1-0613 | 646.190 | OH | a1 | B08 | b1 | C11 | c2 | D02 | F |
| 2-1-E00-1-0614 | 647.094 | OH | a1 | B09 | b3 | C15 | c1 | D01 | F |
| 2-1-E00-1-0615 | 647.094 | OH | a1 | B06 | b2 | C02 | c2 | D02 | F |
| 2-1-E00-1-0616 | 647.094 | OH | a1 | B06 | b2 | C21 | c3 | D02 | F |
| 2-1-E00-1-0617 | 647.094 | OH | a1 | B03 | b2 | C25 | c3 | D02 | F |
| 2-1-E00-1-0618 | 647.094 | OH | a1 | B04 | b1 | C29 | c3 | D02 | F |
| 2-1-E00-1-0619 | 647.114 | OH | a1 | B08 | b1 | C10 | c2 | D02 | F |
| 2-1-E00-1-0620 | 647.117 | OH | a2 | B13 | b3 | C08 | c2 | D02 | F |
| 2-1-E00-1-0621 | 647.135 | OH | a1 | B06 | b2 | C03 | c2 | D02 | F |
| 2-1-E00-1-0622 | 647.149 | OH | a2 | B18 | b2 | C02 | c2 | D02 | F |
| 2-1-E00-1-0623 | 647.149 | OH | a2 | B18 | b2 | C21 | c3 | D02 | F |
| 2-1-E00-1-0624 | 647.170 | OH | a2 | B14 | b2 | C07 | c2 | D02 | F |
| 2-1-E00-1-0625 | 647.189 | OH | a2 | B17 | b2 | C05 | c2 | D02 | F |
| 2-1-E00-1-0626 | 648.090 | OH | a1 | B09 | b1 | C14 | c1 | D01 | F |
| 2-1-E00-1-0627 | 648.090 | OH | a2 | B18 | b2 | C03 | c2 | D02 | F |
| 2-1-E00-1-0628 | 648.094 | OH | a1 | B03 | b2 | C04 | c2 | D02 | F |
| 2-1-E00-1-0629 | 648.133 | OH | a1 | B09 | b2 | C33 | c3 | D02 | F |
| 2-1-E00-1-0630 | 648.169 | OH | a1 | B05 | b2 | C23 | c3 | D02 | F |
| 2-1-E00-1-0631 | 648.185 | OH | a1 | B01 | b2 | C07 | c2 | D02 | F |
| 2-1-E00-1-0632 | 649.085 | OH | a1 | B08 | b1 | C09 | c1 | D01 | F |
| 2-1-E00-1-0633 | 649.099 | OH | a1 | B04 | b2 | C05 | c2 | D02 | F |
| 2-1-E00-1-0634 | 649.128 | OH | a2 | B17 | b2 | C25 | c3 | D02 | F |
| 2-1-E00-1-0635 | 649.132 | OH | a2 | B19 | b1 | C30 | c3 | D02 | F |
| 2-1-E00-1-0636 | 649.169 | OH | a2 | B15 | b1 | C13 | c2 | D02 | F |
| 2-1-E00-1-0637 | 650.108 | OH | a1 | B02 | b2 | C07 | c2 | D02 | F |
| 2-1-E00-1-0638 | 650.125 | OH | a2 | B20 | b2 | C07 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0639 | 650.127 | OH | a1 | B05 | b2 | C06 | c2 | D02 | F |
| 2-1-E00-1-0640 | 650.128 | OH | a1 | B06 | b2 | C23 | c3 | D02 | F |
| 2-1-E00-1-0641 | 650.146 | OH | a1 | B04 | b2 | C25 | c3 | D02 | F |
| 2-1-E00-1-0642 | 650.148 | OH | a1 | B07 | b1 | C32 | c3 | D02 | F |
| 2-1-E00-1-0643 | 650.185 | OH | a1 | B08 | b3 | C06 | c1 | D01 | F |
| 2-1-E00-1-0644 | 651.120 | OH | a2 | B14 | b3 | C08 | c2 | D02 | F |
| 2-1-E00-1-0645 | 651.124 | OH | a2 | B17 | b2 | C04 | c2 | D02 | F |
| 2-1-E00-1-0646 | 651.145 | OH | a2 | B15 | b3 | C15 | c2 | D02 | F |
| 2-1-E00-1-0647 | 651.148 | OH | a2 | B13 | b1 | C09 | c2 | D02 | F |
| 2-1-E00-1-0648 | 651.159 | OH | a2 | B19 | b2 | C19 | c3 | D02 | F |
| 2-1-E00-1-0649 | 651.180 | OH | a2 | B16 | b1 | C13 | c2 | D02 | F |
| 2-1-E00-1-0650 | 651.199 | OH | a2 | B18 | b2 | C23 | c3 | D02 | F |
| 2-1-E00-1-0651 | 652.110 | OH | a1 | B10 | b2 | C05 | c1 | D01 | F |
| 2-1-E00-1-0652 | 652.160 | OH | a2 | B15 | b3 | C16 | c1 | D01 | F |
| 2-1-E00-1-0653 | 653.051 | OH | a2 | B13 | b1 | C31 | c3 | D02 | F |
| 2-1-E00-1-0654 | 653.074 | OH | a1 | B08 | b1 | C12 | c2 | D02 | F |
| 2-1-E00-1-0655 | 653.088 | OH | a1 | B06 | b2 | C06 | c2 | D02 | F |
| 2-1-E00-1-0656 | 653.094 | OH | a1 | B01 | b3 | C08 | c2 | D02 | F |
| 2-1-E00-1-0657 | 653.124 | OH | a1 | B07 | b2 | C02 | c2 | D02 | F |
| 2-1-E00-1-0658 | 653.124 | OH | a1 | B04 | b2 | C04 | c2 | D02 | F |
| 2-1-E00-1-0659 | 653.138 | OH | a2 | B19 | b2 | C27 | c3 | D02 | F |
| 2-1-E00-1-0660 | 653.153 | OH | a1 | B07 | b2 | C21 | c3 | D02 | F |
| 2-1-E00-1-0661 | 653.163 | OH | a1 | B09 | b2 | C07 | c1 | D01 | F |
| 2-1-E00-1-0662 | 653.200 | OH | a2 | B13 | b3 | C06 | c2 | D02 | F |
| 2-1-E00-1-0663 | 654.046 | OH | a2 | B16 | b3 | C15 | c2 | D02 | F |
| 2-1-E00-1-0664 | 654.089 | OH | a1 | B02 | b3 | C08 | c2 | D02 | F |
| 2-1-E00-1-0665 | 654.089 | OH | a2 | B18 | b2 | C06 | c2 | D02 | F |
| 2-1-E00-1-0666 | 654.103 | OH | a1 | B07 | b2 | C03 | c2 | D02 | F |
| 2-1-E00-1-0667 | 654.103 | OH | a2 | B20 | b3 | C08 | c1 | D01 | F |
| 2-1-E00-1-0668 | 654.210 | OH | a2 | B20 | b1 | C08 | c2 | D02 | F |
| 2-1-E00-1-0669 | 655.055 | OH | a2 | B15 | b3 | C17 | c1 | D01 | F |
| 2-1-E00-1-0670 | 655.073 | OH | a2 | B15 | b1 | C14 | c2 | D02 | F |
| 2-1-E00-1-0671 | 655.084 | OH | a2 | B16 | b3 | C16 | c1 | D01 | F |
| 2-1-E00-1-0672 | 655.088 | OH | a2 | B20 | b1 | C11 | c2 | D02 | F |
| 2-1-E00-1-0673 | 655.098 | OH | a2 | B13 | b3 | C18 | c1 | D01 | F |
| 2-1-E00-1-0674 | 655.104 | OH | a1 | B10 | b2 | C04 | c1 | D01 | F |
| 2-1-E00-1-0675 | 655.104 | OH | a1 | B03 | b1 | C13 | c2 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0676 | 655.104 | OH | a2 | B19 | b2 | C01 | c2 | D02 | F |
| 2-1-E00-1-0677 | 655.125 | OH | a2 | B20 | b1 | C10 | c2 | D02 | F |
| 2-1-E00-1-0678 | 655.125 | OH | a2 | B14 | b1 | C09 | c2 | D02 | F |
| 2-1-E00-1-0679 | 655.142 | OH | a2 | B16 | b3 | C17 | c1 | D01 | F |
| 2-1-E00-1-0680 | 656.099 | OH | a2 | B16 | b1 | C14 | c2 | D02 | F |
| 2-1-E00-1-0681 | 656.099 | OH | a2 | B14 | b1 | C31 | c3 | D02 | F |
| 2-1-E00-1-0682 | 656.119 | OH | a1 | B03 | b3 | C15 | c2 | D02 | F |
| 2-1-E00-1-0683 | 656.120 | OH | a1 | B09 | b3 | C08 | c1 | D01 | F |
| 2-1-E00-1-0684 | 656.133 | OH | a1 | B09 | b1 | C08 | c2 | D02 | F |
| 2-1-E00-1-0685 | 656.149 | OH | a1 | B01 | b1 | C09 | c2 | D02 | F |
| 2-1-E00-1-0686 | 657.083 | OH | a1 | B07 | b2 | C23 | c3 | D02 | F |
| 2-1-E00-1-0687 | 657.094 | OH | a1 | B03 | b3 | C16 | c1 | D01 | F |
| 2-1-E00-1-0688 | 657.099 | OH | a1 | B01 | b1 | C31 | c3 | D02 | F |
| 2-1-E00-1-0689 | 657.099 | OH | a1 | B05 | b1 | C29 | c3 | D02 | F |
| 2-1-E00-1-0690 | 657.104 | OH | a2 | B14 | b3 | C06 | c2 | D02 | F |
| 2-1-E00-1-0691 | 657.104 | OH | a2 | B17 | b1 | C13 | c2 | D02 | F |
| 2-1-E00-1-0692 | 657.175 | OH | a1 | B02 | b1 | C09 | c2 | D02 | F |
| 2-1-E00-1-0693 | 658.084 | OH | a1 | B09 | b1 | C11 | c2 | D02 | F |
| 2-1-E00-1-0694 | 658.115 | OH | a2 | B20 | b1 | C09 | c1 | D01 | F |
| 2-1-E00-1-0695 | 658.115 | OH | a1 | B08 | b1 | C30 | c3 | D02 | F |
| 2-1-E00-1-0696 | 658.115 | OH | a1 | B02 | b1 | C31 | c3 | D02 | F |
| 2-1-E00-1-0697 | 658.115 | OH | a2 | B15 | b2 | C07 | c2 | D02 | F |
| 2-1-E00-1-0698 | 658.153 | OH | a1 | B01 | b3 | C06 | c2 | D02 | F |
| 2-1-E00-1-0699 | 658.190 | OH | a1 | B07 | b2 | C06 | c2 | D02 | F |
| 2-1-E00-1-0700 | 658.226 | OH | a2 | B14 | b3 | C18 | c1 | D01 | F |
| 2-1-E00-1-0701 | 659.030 | OH | a1 | B09 | b1 | C10 | c2 | D02 | F |
| 2-1-E00-1-0702 | 659.109 | OH | a1 | B03 | b3 | C17 | c1 | D01 | F |
| 2-1-E00-1-0703 | 659.110 | OH | a1 | B03 | b1 | C14 | c2 | D02 | F |
| 2-1-E00-1-0704 | 659.110 | OH | a1 | B04 | b1 | C13 | c2 | D02 | F |
| 2-1-E00-1-0705 | 659.110 | OH | a1 | B06 | b1 | C29 | c3 | D02 | F |
| 2-1-E00-1-0706 | 659.129 | OH | a1 | B02 | b3 | C06 | c2 | D02 | F |
| 2-1-E00-1-0707 | 659.131 | OH | a2 | B17 | b3 | C15 | c2 | D02 | F |
| 2-1-E00-1-0708 | 659.185 | OH | a2 | B20 | b3 | C06 | c1 | D01 | F |
| 2-1-E00-1-0709 | 659.210 | OH | a1 | B01 | b3 | C18 | c1 | D01 | F |
| 2-1-E00-1-0710 | 660.045 | OH | a1 | B08 | b2 | C19 | c3 | D02 | F |
| 2-1-E00-1-0711 | 660.060 | OH | a2 | B17 | b3 | C16 | c1 | D01 | F |
| 2-1-E00-1-0712 | 660.095 | OH | a1 | B05 | b2 | C05 | c2 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0713 | 660.105 | OH | a2 | B16 | b2 | C07 | c2 | D02 | F |
| 2-1-E00-1-0714 | 660.115 | OH | a2 | B18 | b1 | C29 | c3 | D02 | F |
| 2-1-E00-1-0715 | 660.115 | OH | a2 | B20 | b1 | C12 | c2 | D02 | F |
| 2-1-E00-1-0716 | 660.124 | OH | a1 | B04 | b3 | C15 | c2 | D02 | F |
| 2-1-E00-1-0717 | 660.205 | OH | a1 | B02 | b3 | C18 | c1 | D01 | F |
| 2-1-E00-1-0718 | 661.041 | OH | a1 | B08 | b2 | C27 | c3 | D02 | F |
| 2-1-E00-1-0719 | 661.044 | OH | a1 | B04 | b3 | C16 | c1 | D01 | F |
| 2-1-E00-1-0720 | 661.060 | OH | a1 | B09 | b1 | C09 | c1 | D01 | F |
| 2-1-E00-1-0721 | 661.110 | OH | a1 | B10 | b1 | C13 | c1 | D01 | F |
| 2-1-E00-1-0722 | 661.110 | OH | a1 | B05 | b2 | C25 | c3 | D02 | F |
| 2-1-E00-1-0723 | 661.113 | OH | a2 | B17 | b3 | C17 | c1 | D01 | F |
| 2-1-E00-1-0724 | 661.164 | OH | a2 | B17 | b1 | C14 | c2 | D02 | F |
| 2-1-E00-1-0725 | 661.189 | OH | a2 | B15 | b3 | C08 | c2 | D02 | F |
| 2-1-E00-1-0726 | 661.205 | OH | a1 | B06 | b2 | C05 | c2 | D02 | F |
| 2-1-E00-1-0727 | 662.055 | OH | a1 | B09 | b3 | C06 | c1 | D01 | F |
| 2-1-E00-1-0728 | 662.059 | OH | a1 | B10 | b3 | C15 | c1 | D01 | F |
| 2-1-E00-1-0729 | 662.105 | OH | a1 | B04 | b3 | C17 | c1 | D01 | F |
| 2-1-E00-1-0730 | 662.128 | OH | a1 | B04 | b1 | C14 | c2 | D02 | F |
| 2-1-E00-1-0731 | 662.140 | OH | a2 | B18 | b2 | C05 | c2 | D02 | F |
| 2-1-E00-1-0732 | 662.154 | OH | a1 | B05 | b2 | C04 | c2 | D02 | F |
| 2-1-E00-1-0733 | 663.075 | OH | a1 | B06 | b2 | C25 | c3 | D02 | F |
| 2-1-E00-1-0734 | 663.075 | OH | a1 | B03 | b2 | C07 | c2 | D02 | F |
| 2-1-E00-1-0735 | 663.115 | OH | a1 | B09 | b1 | C12 | c2 | D02 | F |
| 2-1-E00-1-0736 | 663.144 | OH | a1 | B08 | b2 | C01 | c2 | D02 | F |
| 2-1-E00-1-0737 | 664.071 | OH | a2 | B16 | b3 | C08 | c2 | D02 | F |
| 2-1-E00-1-0738 | 664.089 | OH | a2 | B19 | b1 | C32 | c3 | D02 | F |
| 2-1-E00-1-0739 | 664.090 | OH | a2 | B18 | b2 | C25 | c3 | D02 | F |
| 2-1-E00-1-0740 | 664.090 | OH | a1 | B10 | b1 | C14 | c1 | D01 | F |
| 2-1-E00-1-0741 | 664.143 | OH | a1 | B06 | b2 | C04 | c2 | D02 | F |
| 2-1-E00-1-0742 | 664.144 | OH | a1 | B10 | b2 | C33 | c3 | D02 | F |
| 2-1-E00-1-0743 | 664.200 | OH | a1 | B07 | b1 | C29 | c3 | D02 | F |
| 2-1-E00-1-0744 | 665.066 | OH | a2 | B19 | b2 | C02 | c2 | D02 | F |
| 2-1-E00-1-0745 | 665.069 | OH | a2 | B18 | b2 | C04 | c2 | D02 | F |
| 2-1-E00-1-0746 | 665.085 | OH | a2 | B15 | b1 | C09 | c2 | D02 | F |
| 2-1-E00-1-0747 | 665.094 | OH | a2 | B19 | b2 | C21 | c3 | D02 | F |
| 2-1-E00-1-0748 | 665.161 | OH | a2 | B20 | b1 | C30 | c3 | D02 | F |
| 2-1-E00-1-0749 | 666.120 | OH | a2 | B17 | b2 | C07 | c2 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0750 | 666.123 | OH | a2 | B15 | b1 | C31 | c3 | D02 | F |
| 2-1-E00-1-0751 | 666.141 | OH | a2 | B19 | b2 | C03 | c2 | D02 | F |
| 2-1-E00-1-0752 | 666.176 | OH | a1 | B03 | b3 | C08 | c2 | D02 | F |
| 2-1-E00-1-0753 | 667.104 | OH | a1 | B04 | b2 | C07 | c2 | D02 | F |
| 2-1-E00-1-0754 | 667.122 | OH | a2 | B15 | b3 | C06 | c2 | D02 | F |
| 2-1-E00-1-0755 | 667.140 | OH | a1 | B07 | b2 | C05 | c2 | D02 | F |
| 2-1-E00-1-0756 | 667.140 | OH | a2 | B16 | b1 | C09 | c2 | D02 | F |
| 2-1-E00-1-0757 | 667.160 | OH | a2 | B20 | b2 | C19 | c3 | D02 | F |
| 2-1-E00-1-0758 | 667.215 | OH | a2 | B16 | b1 | C31 | c3 | D02 | F |
| 2-1-E00-1-0759 | 668.099 | OH | a2 | B20 | b2 | C27 | c3 | D02 | F |
| 2-1-E00-1-0760 | 668.105 | OH | a1 | B07 | b2 | C25 | c3 | D02 | F |
| 2-1-E00-1-0761 | 668.175 | OH | a2 | B15 | b3 | C18 | c1 | D01 | F |
| 2-1-E00-1-0762 | 669.071 | OH | a1 | B09 | b1 | C30 | c3 | D02 | F |
| 2-1-E00-1-0763 | 669.114 | OH | a1 | B05 | b1 | C13 | c2 | D02 | F |
| 2-1-E00-1-0764 | 669.119 | OH | a1 | B10 | b2 | C07 | c1 | D01 | F |
| 2-1-E00-1-0765 | 669.119 | OH | a2 | B16 | b3 | C06 | c2 | D02 | F |
| 2-1-E00-1-0766 | 669.119 | OH | a2 | B17 | b3 | C08 | c2 | D02 | F |
| 2-1-E00-1-0767 | 669.194 | OH | a2 | B19 | b2 | C23 | c3 | D02 | F |
| 2-1-E00-1-0768 | 670.136 | OH | a1 | B04 | b3 | C08 | c2 | D02 | F |
| 2-1-E00-1-0769 | 670.151 | OH | a1 | B07 | b2 | C04 | c2 | D02 | F |
| 2-1-E00-1-0770 | 670.165 | OH | a1 | B05 | b3 | C15 | c2 | D02 | F |
| 2-1-E00-1-0771 | 670.205 | OH | a2 | B16 | b3 | C18 | c1 | D01 | F |
| 2-1-E00-1-0772 | 671.050 | OH | a1 | B03 | b1 | C09 | c2 | D02 | F |
| 2-1-E00-1-0773 | 671.064 | OH | a1 | B09 | b2 | C19 | c3 | D02 | F |
| 2-1-E00-1-0774 | 672.080 | OH | a1 | B06 | b1 | C13 | c2 | D02 | F |
| 2-1-E00-1-0775 | 672.094 | OH | a1 | B05 | b3 | C16 | c1 | D01 | F |
| 2-1-E00-1-0776 | 672.100 | OH | a1 | B03 | b1 | C31 | c3 | D02 | F |
| 2-1-E00-1-0777 | 672.115 | OH | a2 | B19 | b2 | C06 | c2 | D02 | F |
| 2-1-E00-1-0778 | 672.130 | OH | a2 | B20 | b2 | C01 | c2 | D02 | F |
| 2-1-E00-1-0779 | 672.130 | OH | a1 | B09 | b2 | C27 | c3 | D02 | F |
| 2-1-E00-1-0780 | 672.144 | OH | a2 | B18 | b1 | C13 | c2 | D02 | F |
| 2-1-E00-1-0781 | 672.169 | OH | a1 | B08 | b1 | C32 | c3 | D02 | F |
| 2-1-E00-1-0782 | 672.205 | OH | a1 | B03 | b3 | C06 | c2 | D02 | F |
| 2-1-E00-1-0783 | 673.060 | OH | a1 | B06 | b3 | C15 | c2 | D02 | F |
| 2-1-E00-1-0784 | 673.078 | OH | a1 | B10 | b3 | C08 | c1 | D01 | F |
| 2-1-E00-1-0785 | 673.094 | OH | a1 | B10 | b1 | C08 | c2 | D02 | F |
| 2-1-E00-1-0786 | 673.143 | OH | a1 | B05 | b3 | C17 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0787 | 673.147 | OH | a1 | B05 | b1 | C14 | c2 | D02 | F |
| 2-1-E00-1-0788 | 674.061 | OH | a1 | B06 | b3 | C16 | c1 | D01 | F |
| 2-1-E00-1-0789 | 674.079 | OH | a2 | B18 | b3 | C15 | c2 | D02 | F |
| 2-1-E00-1-0790 | 674.109 | OH | a1 | B08 | b2 | C02 | c2 | D02 | F |
| 2-1-E00-1-0791 | 674.109 | OH | a2 | B17 | b1 | C09 | c2 | D02 | F |
| 2-1-E00-1-0792 | 674.109 | OH | a1 | B10 | b1 | C11 | c2 | D02 | F |
| 2-1-E00-1-0793 | 674.131 | OH | a1 | B03 | b3 | C18 | c1 | D01 | F |
| 2-1-E00-1-0794 | 674.131 | OH | a1 | B08 | b2 | C21 | c3 | D02 | F |
| 2-1-E00-1-0795 | 674.148 | OH | a2 | B18 | b3 | C16 | c1 | D01 | F |
| 2-1-E00-1-0796 | 675.078 | OH | a2 | B17 | b1 | C31 | c3 | D02 | F |
| 2-1-E00-1-0797 | 675.089 | OH | a1 | B08 | b2 | C03 | c2 | D02 | F |
| 2-1-E00-1-0798 | 675.090 | OH | a1 | B09 | b2 | C01 | c2 | D02 | F |
| 2-1-E00-1-0799 | 675.105 | OH | a1 | B10 | b1 | C10 | c2 | D02 | F |
| 2-1-E00-1-0800 | 675.105 | OH | a1 | B04 | b1 | C09 | c2 | D02 | F |
| 2-1-E00-1-0801 | 675.126 | OH | a1 | B06 | b3 | C17 | c1 | D01 | F |
| 2-1-E00-1-0802 | 675.148 | OH | a1 | B06 | b1 | C14 | c2 | D02 | F |
| 2-1-E00-1-0803 | 675.165 | OH | a1 | B04 | b1 | C31 | c3 | D02 | F |
| 2-1-E00-1-0804 | 676.085 | OH | a2 | B17 | b3 | C06 | c2 | D02 | F |
| 2-1-E00-1-0805 | 676.089 | OH | a2 | B18 | b3 | C17 | c1 | D01 | F |
| 2-1-E00-1-0806 | 676.100 | OH | a2 | B18 | b1 | C14 | c2 | D02 | F |
| 2-1-E00-1-0807 | 676.105 | OH | a1 | B04 | b3 | C06 | c2 | D02 | F |
| 2-1-E00-1-0808 | 676.105 | OH | a2 | B17 | b3 | C18 | c1 | D01 | F |
| 2-1-E00-1-0809 | 676.110 | OH | a1 | B07 | b1 | C13 | c2 | D02 | F |
| 2-1-E00-1-0810 | 676.110 | OH | a1 | B10 | b1 | C09 | c1 | D01 | F |
| 2-1-E00-1-0811 | 676.180 | OH | a1 | B05 | b2 | C07 | c2 | D02 | F |
| 2-1-E00-1-0812 | 677.021 | OH | a1 | B04 | b3 | C18 | c1 | D01 | F |
| 2-1-E00-1-0813 | 677.105 | OH | a1 | B08 | b2 | C23 | c3 | D02 | F |
| 2-1-E00-1-0814 | 677.109 | OH | a2 | B19 | b1 | C29 | c3 | D02 | F |
| 2-1-E00-1-0815 | 677.109 | OH | a1 | B07 | b3 | C15 | c2 | D02 | F |
| 2-1-E00-1-0816 | 678.036 | OH | a1 | B10 | b3 | C06 | c1 | D01 | F |
| 2-1-E00-1-0817 | 678.085 | OH | a1 | B07 | b3 | C16 | c1 | D01 | F |
| 2-1-E00-1-0818 | 678.114 | OH | a1 | B06 | b2 | C07 | c2 | D02 | F |
| 2-1-E00-1-0819 | 678.135 | OH | a1 | B08 | b2 | C06 | c2 | D02 | F |
| 2-1-E00-1-0820 | 678.216 | OH | a1 | B10 | b1 | C12 | c2 | D02 | F |
| 2-1-E00-1-0821 | 679.101 | OH | a2 | B20 | b1 | C32 | c3 | D02 | F |
| 2-1-E00-1-0822 | 679.176 | OH | a2 | B18 | b2 | C07 | c2 | D02 | F |
| 2-1-E00-1-0823 | 680.049 | OH | a1 | B07 | b3 | C17 | c1 | D01 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0824 | 680.066 | OH | a1 | B07 | b1 | C14 | c2 | D02 | F |
| 2-1-E00-1-0825 | 680.119 | OH | a2 | B19 | b2 | C05 | c2 | D02 | F |
| 2-1-E00-1-0826 | 680.195 | OH | a1 | B05 | b3 | C08 | c2 | D02 | F |
| 2-1-E00-1-0827 | 681.046 | OH | a2 | B20 | b2 | C02 | c2 | D02 | F |
| 2-1-E00-1-0828 | 681.061 | OH | a2 | B20 | b2 | C21 | c3 | D02 | F |
| 2-1-E00-1-0829 | 681.156 | OH | a2 | B19 | b2 | C25 | c3 | D02 | F |
| 2-1-E00-1-0830 | 681.194 | OH | a2 | B20 | b2 | C03 | c2 | D02 | F |
| 2-1-E00-1-0831 | 682.080 | OH | a1 | B06 | b3 | C08 | c2 | D02 | F |
| 2-1-E00-1-0832 | 682.080 | OH | a1 | B09 | b1 | C32 | c3 | D02 | F |
| 2-1-E00-1-0833 | 682.115 | OH | a2 | B19 | b2 | C04 | c2 | D02 | F |
| 2-1-E00-1-0834 | 682.120 | OH | a2 | B18 | b3 | C08 | c2 | D02 | F |
| 2-1-E00-1-0835 | 683.099 | OH | a1 | B09 | b2 | C02 | c2 | D02 | F |
| 2-1-E00-1-0836 | 684.094 | OH | a1 | B05 | b1 | C09 | c2 | D02 | F |
| 2-1-E00-1-0837 | 684.100 | OH | a1 | B09 | b2 | C21 | c3 | D02 | F |
| 2-1-E00-1-0838 | 684.167 | OH | a1 | B10 | b1 | C30 | c3 | D02 | F |
| 2-1-E00-1-0839 | 685.094 | OH | a1 | B07 | b2 | C07 | c2 | D02 | F |
| 2-1-E00-1-0840 | 685.099 | OH | a1 | B05 | b1 | C31 | c3 | D02 | F |
| 2-1-E00-1-0841 | 685.114 | OH | a1 | B09 | b2 | C03 | c2 | D02 | F |
| 2-1-E00-1-0842 | 686.109 | OH | a2 | B20 | b2 | C23 | c3 | D02 | F |
| 2-1-E00-1-0843 | 686.146 | OH | a1 | B08 | b1 | C29 | c3 | D02 | F |
| 2-1-E00-1-0844 | 686.146 | OH | a1 | B05 | b3 | C06 | c2 | D02 | F |
| 2-1-E00-1-0845 | 686.166 | OH | a1 | B06 | b1 | C09 | c2 | D02 | F |
| 2-1-E00-1-0846 | 686.221 | OH | a1 | B10 | b2 | C19 | c3 | D02 | F |
| 2-1-E00-1-0847 | 687.105 | OH | a1 | B06 | b1 | C31 | c3 | D02 | F |
| 2-1-E00-1-0848 | 687.162 | OH | a2 | B20 | b2 | C06 | c2 | D02 | F |
| 2-1-E00-1-0849 | 688.077 | OH | a2 | B11 | b3 | C16 | c2 | D02 | F |
| 2-1-E00-1-0850 | 688.125 | OH | a1 | B10 | b2 | C27 | c3 | D02 | F |
| 2-1-E00-1-0851 | 688.125 | OH | a2 | B18 | b1 | C09 | c2 | D02 | F |
| 2-1-E00-1-0852 | 688.125 | OH | a1 | B05 | b3 | C18 | c1 | D01 | F |
| 2-1-E00-1-0853 | 688.200 | OH | a2 | B18 | b1 | C31 | c3 | D02 | F |
| 2-1-E00-1-0854 | 689.055 | OH | a1 | B06 | b3 | C06 | c2 | D02 | F |
| 2-1-E00-1-0855 | 689.094 | OH | a1 | B07 | b3 | C08 | c2 | D02 | F |
| 2-1-E00-1-0856 | 689.124 | OH | a2 | B12 | b3 | C16 | c2 | D02 | F |
| 2-1-E00-1-0857 | 689.141 | OH | a1 | B08 | b2 | C05 | c2 | D02 | F |
| 2-1-E00-1-0858 | 689.200 | OH | a1 | B09 | b2 | C23 | c3 | D02 | F |
| 2-1-E00-1-0859 | 690.056 | OH | a2 | B18 | b3 | C06 | c2 | D02 | F |
| 2-1-E00-1-0860 | 690.070 | OH | a2 | B11 | b3 | C17 | c2 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0861 | 690.089 | OH | a2 | B19 | b1 | C13 | c2 | D02 | F |
| 2-1-E00-1-0862 | 690.100 | OH | a1 | B06 | b3 | C18 | c1 | D01 | F |
| 2-1-E00-1-0863 | 691.055 | OH | a1 | B08 | b2 | C25 | c3 | D02 | F |
| 2-1-E00-1-0864 | 691.055 | OH | a2 | B12 | b3 | C17 | c2 | D02 | F |
| 2-1-E00-1-0865 | 691.115 | OH | a1 | B09 | b2 | C06 | c2 | D02 | F |
| 2-1-E00-1-0866 | 691.121 | OH | a1 | B10 | b2 | C01 | c2 | D02 | F |
| 2-1-E00-1-0867 | 692.051 | OH | a2 | B18 | b3 | C18 | c1 | D01 | F |
| 2-1-E00-1-0868 | 692.066 | OH | a2 | B19 | b3 | C15 | c2 | D02 | F |
| 2-1-E00-1-0869 | 692.084 | OH | a1 | B08 | b2 | C04 | c2 | D02 | F |
| 2-1-E00-1-0870 | 692.100 | OH | a2 | B19 | b3 | C16 | c1 | D01 | F |
| 2-1-E00-1-0871 | 692.120 | OH | a1 | B07 | b1 | C09 | c2 | D02 | F |
| 2-1-E00-1-0872 | 693.050 | OH | a1 | B07 | b1 | C31 | c3 | D02 | F |
| 2-1-E00-1-0873 | 693.080 | OH | a2 | B19 | b3 | C17 | c1 | D01 | F |
| 2-1-E00-1-0874 | 693.119 | OH | a2 | B19 | b1 | C14 | c2 | D02 | F |
| 2-1-E00-1-0875 | 694.096 | OH | a2 | B20 | b1 | C29 | c3 | D02 | F |
| 2-1-E00-1-0876 | 694.171 | OH | a1 | B07 | b3 | C06 | c2 | D02 | F |
| 2-1-E00-1-0877 | 695.081 | OH | a1 | B07 | b3 | C18 | c1 | D01 | F |
| 2-1-E00-1-0878 | 695.081 | OH | a2 | B20 | b2 | C05 | c2 | D02 | F |
| 2-1-E00-1-0879 | 695.171 | OH | a1 | B09 | b1 | C29 | c3 | D02 | F |
| 2-1-E00-1-0880 | 696.027 | OH | a1 | B08 | b1 | C13 | c2 | D02 | F |
| 2-1-E00-1-0881 | 697.091 | OH | a2 | B20 | b2 | C25 | c3 | D02 | F |
| 2-1-E00-1-0882 | 698.109 | OH | a2 | B19 | b2 | C07 | c2 | D02 | F |
| 2-1-E00-1-0883 | 698.182 | OH | a1 | B10 | b1 | C32 | c3 | D02 | F |
| 2-1-E00-1-0884 | 699.101 | OH | a1 | B08 | b3 | C15 | c2 | D02 | F |
| 2-1-E00-1-0885 | 699.166 | OH | a2 | B20 | b2 | C04 | c2 | D02 | F |
| 2-1-E00-1-0886 | 700.161 | OH | a1 | B08 | b3 | C16 | c1 | D01 | F |
| 2-1-E00-1-0887 | 701.036 | OH | a1 | B09 | b2 | C05 | c2 | D02 | F |
| 2-1-E00-1-0888 | 701.089 | OH | a1 | B10 | b2 | C02 | c2 | D02 | F |
| 2-1-E00-1-0889 | 701.105 | OH | a1 | B10 | b2 | C21 | c3 | D02 | F |
| 2-1-E00-1-0890 | 701.120 | OH | a1 | B09 | b2 | C25 | c3 | D02 | F |
| 2-1-E00-1-0891 | 702.104 | OH | a1 | B08 | b3 | C17 | c1 | D01 | F |
| 2-1-E00-1-0892 | 703.067 | OH | a1 | B08 | b1 | C14 | c2 | D02 | F |
| 2-1-E00-1-0893 | 703.067 | OH | a2 | B19 | b3 | C08 | c2 | D02 | F |
| 2-1-E00-1-0894 | 703.071 | OH | a1 | B10 | b2 | C03 | c2 | D02 | F |
| 2-1-E00-1-0895 | 703.100 | OH | a2 | B13 | b3 | C16 | c2 | D02 | F |
| 2-1-E00-1-0896 | 703.109 | OH | a1 | B09 | b2 | C04 | c2 | D02 | F |
| 2-1-E00-1-0897 | 704.100 | OH | a2 | B13 | b3 | C17 | c2 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0898 | 704.120 | OH | a2 | B11 | b3 | C18 | c2 | D02 | F |
| 2-1-E00-1-0899 | 705.071 | OH | a1 | B10 | b2 | C23 | c3 | D02 | F |
| 2-1-E00-1-0900 | 706.066 | OH | a2 | B12 | b3 | C18 | c2 | D02 | F |
| 2-1-E00-1-0901 | 707.061 | OH | a2 | B14 | b3 | C16 | c2 | D02 | F |
| 2-1-E00-1-0902 | 707.084 | OH | a2 | B19 | b1 | C09 | c2 | D02 | F |
| 2-1-E00-1-0903 | 707.096 | OH | a2 | B20 | b1 | C13 | c2 | D02 | F |
| 2-1-E00-1-0904 | 707.110 | OH | a1 | B08 | b2 | C07 | c2 | D02 | F |
| 2-1-E00-1-0905 | 707.152 | OH | a2 | B19 | b1 | C31 | c3 | D02 | F |
| 2-1-E00-1-0906 | 707.171 | OH | a1 | B10 | b2 | C06 | c2 | D02 | F |
| 2-1-E00-1-0907 | 708.061 | OH | a1 | B01 | b3 | C16 | c2 | D02 | F |
| 2-1-E00-1-0908 | 708.100 | OH | a2 | B19 | b3 | C06 | c2 | D02 | F |
| 2-1-E00-1-0909 | 709.027 | OH | a2 | B20 | b3 | C15 | c2 | D02 | F |
| 2-1-E00-1-0910 | 709.045 | OH | a2 | B14 | b3 | C17 | c2 | D02 | F |
| 2-1-E00-1-0911 | 709.046 | OH | a1 | B02 | b3 | C16 | c2 | D02 | F |
| 2-1-E00-1-0912 | 709.095 | OH | a2 | B20 | b3 | C16 | c1 | D01 | F |
| 2-1-E00-1-0913 | 709.114 | OH | a1 | B01 | b3 | C17 | c2 | D02 | F |
| 2-1-E00-1-0914 | 710.061 | OH | a2 | B19 | b3 | C18 | c1 | D01 | F |
| 2-1-E00-1-0915 | 710.092 | OH | a1 | B09 | b1 | C13 | c2 | D02 | F |
| 2-1-E00-1-0916 | 710.121 | OH | a1 | B02 | b3 | C17 | c2 | D02 | F |
| 2-1-E00-1-0917 | 711.056 | OH | a1 | B08 | b3 | C08 | c2 | D02 | F |
| 2-1-E00-1-0918 | 711.056 | OH | a2 | B20 | b3 | C17 | c1 | D01 | F |
| 2-1-E00-1-0919 | 711.076 | OH | a2 | B20 | b1 | C14 | c2 | D02 | F |
| 2-1-E00-1-0920 | 711.076 | OH | a1 | B09 | b3 | C15 | c2 | D02 | F |
| 2-1-E00-1-0921 | 712.056 | OH | a1 | B09 | b3 | C16 | c1 | D01 | F |
| 2-1-E00-1-0922 | 712.125 | OH | a1 | B09 | b3 | C17 | c1 | D01 | F |
| 2-1-E00-1-0923 | 714.087 | OH | a1 | B09 | b1 | C14 | c2 | D02 | F |
| 2-1-E00-1-0924 | 714.087 | OH | a1 | B10 | b1 | C29 | c3 | D02 | F |
| 2-1-E00-1-0925 | 714.177 | OH | a1 | B08 | b1 | C09 | c2 | D02 | F |
| 2-1-E00-1-0926 | 715.082 | OH | a1 | B08 | b1 | C31 | c3 | D02 | F |
| 2-1-E00-1-0927 | 715.157 | OH | a2 | B20 | b2 | C07 | c2 | D02 | F |
| 2-1-E00-1-0928 | 715.161 | OH | a1 | B08 | b3 | C06 | c2 | D02 | F |
| 2-1-E00-1-0929 | 717.013 | OH | a1 | B10 | b2 | C05 | c2 | D02 | F |
| 2-1-E00-1-0930 | 717.115 | OH | a2 | B15 | b3 | C16 | c2 | D02 | F |
| 2-1-E00-1-0931 | 717.125 | OH | a1 | B08 | b3 | C18 | c1 | D01 | F |
| 2-1-E00-1-0932 | 718.077 | OH | a1 | B10 | b2 | C25 | c3 | D02 | F |
| 2-1-E00-1-0933 | 718.172 | OH | a1 | B09 | b2 | C07 | c2 | D02 | F |
| 2-1-E00-1-0934 | 719.066 | OH | a2 | B20 | b3 | C08 | c2 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0935 | 719.087 | OH | a2 | B15 | b3 | C17 | c2 | D02 | F |
| 2-1-E00-1-0936 | 719.104 | OH | a2 | B16 | b3 | C16 | c2 | D02 | F |
| 2-1-E00-1-0937 | 720.082 | OH | a2 | B13 | b3 | C18 | c2 | D02 | F |
| 2-1-E00-1-0938 | 720.095 | OH | a1 | B10 | b2 | C04 | c2 | D02 | F |
| 2-1-E00-1-0939 | 721.066 | OH | a2 | B16 | b3 | C17 | c2 | D02 | F |
| 2-1-E00-1-0940 | 722.076 | OH | a1 | B09 | b3 | C08 | c2 | D02 | F |
| 2-1-E00-1-0941 | 722.115 | OH | a1 | B03 | b3 | C16 | c2 | D02 | F |
| 2-1-E00-1-0942 | 723.091 | OH | a2 | B20 | b1 | C09 | c2 | D02 | F |
| 2-1-E00-1-0943 | 723.166 | OH | a2 | B20 | b1 | C31 | c3 | D02 | F |
| 2-1-E00-1-0944 | 724.057 | OH | a2 | B14 | b3 | C18 | c2 | D02 | F |
| 2-1-E00-1-0945 | 724.077 | OH | a1 | B03 | b3 | C17 | c2 | D02 | F |
| 2-1-E00-1-0946 | 725.022 | OH | a2 | B20 | b3 | C06 | c2 | D02 | F |
| 2-1-E00-1-0947 | 725.072 | OH | a1 | B01 | b3 | C18 | c2 | D02 | F |
| 2-1-E00-1-0948 | 725.075 | OH | a2 | B17 | b3 | C16 | c2 | D02 | F |
| 2-1-E00-1-0949 | 726.067 | OH | a1 | B02 | b3 | C18 | c2 | D02 | F |
| 2-1-E00-1-0950 | 726.087 | OH | a2 | B20 | b3 | C18 | c1 | D01 | F |
| 2-1-E00-1-0951 | 726.090 | OH | a1 | B04 | b3 | C16 | c2 | D02 | F |
| 2-1-E00-1-0952 | 726.102 | OH | a1 | B09 | b1 | C09 | c2 | D02 | F |
| 2-1-E00-1-0953 | 726.116 | OH | a1 | B10 | b1 | C13 | c2 | D02 | F |
| 2-1-E00-1-0954 | 726.177 | OH | a1 | B09 | b1 | C31 | c3 | D02 | F |
| 2-1-E00-1-0955 | 727.036 | OH | a2 | B17 | b3 | C17 | c2 | D02 | F |
| 2-1-E00-1-0956 | 728.033 | OH | a1 | B09 | b3 | C06 | c2 | D02 | F |
| 2-1-E00-1-0957 | 728.051 | OH | a1 | B10 | b3 | C15 | c2 | D02 | F |
| 2-1-E00-1-0958 | 728.052 | OH | a1 | B04 | b3 | C17 | c2 | D02 | F |
| 2-1-E00-1-0959 | 728.120 | OH | a1 | B10 | b3 | C16 | c1 | D01 | F |
| 2-1-E00-1-0960 | 729.098 | OH | a1 | B09 | b3 | C18 | c1 | D01 | F |
| 2-1-E00-1-0961 | 730.082 | OH | a1 | B10 | b3 | C17 | c1 | D01 | F |
| 2-1-E00-1-0962 | 730.082 | OH | a1 | B10 | b1 | C14 | c2 | D02 | F |
| 2-1-E00-1-0963 | 734.098 | OH | a2 | B15 | b3 | C18 | c2 | D02 | F |
| 2-1-E00-1-0964 | 734.167 | OH | a1 | B10 | b2 | C07 | c2 | D02 | F |
| 2-1-E00-1-0965 | 736.077 | OH | a2 | B16 | b3 | C18 | c2 | D02 | F |
| 2-1-E00-1-0966 | 736.131 | OH | a1 | B05 | b3 | C16 | c2 | D02 | F |
| 2-1-E00-1-0967 | 738.071 | OH | a1 | B10 | b3 | C08 | c2 | D02 | F |
| 2-1-E00-1-0968 | 738.092 | OH | a1 | B05 | b3 | C17 | c2 | D02 | F |
| 2-1-E00-1-0969 | 738.110 | OH | a1 | B06 | b3 | C16 | c2 | D02 | F |
| 2-1-E00-1-0970 | 739.088 | OH | a1 | B03 | b3 | C18 | c2 | D02 | F |
| 2-1-E00-1-0971 | 739.109 | OH | a2 | B18 | b3 | C16 | c2 | D02 | F |

(continued)

| [Table 8-6-2] Compound that may be contained in mixture 2-1-E00-1 | | | | | | | | | |
|---|---|---|---|---|---|---|---|---|---|
| ID | Exact Mass | | a | B | b | C | c | D | d |
| 2-1-E00-1-0972 | 740.072 | OH | a1 | B06 | b3 | C17 | c2 | D02 | F |
| 2-1-E00-1-0973 | 741.071 | OH | a2 | B18 | b3 | C17 | c2 | D02 | F |
| 2-1-E00-1-0974 | 742.047 | OH | a2 | B17 | b3 | C18 | c2 | D02 | F |
| 2-1-E00-1-0975 | 742.097 | OH | a1 | B10 | b1 | C09 | c2 | D02 | F |
| 2-1-E00-1-0976 | 742.172 | OH | a1 | B10 | b1 | C31 | c3 | D02 | F |
| 2-1-E00-1-0977 | 743.063 | OH | a1 | B04 | b3 | C18 | c2 | D02 | F |
| 2-1-E00-1-0978 | 744.028 | OH | a1 | B10 | b3 | C06 | c2 | D02 | F |
| 2-1-E00-1-0979 | 744.081 | OH | a1 | B07 | b3 | C16 | c2 | D02 | F |
| 2-1-E00-1-0980 | 745.092 | OH | a1 | B10 | b3 | C18 | c1 | D01 | F |
| 2-1-E00-1-0981 | 746.042 | OH | a1 | B07 | b3 | C17 | c2 | D02 | F |
| 2-1-E00-1-0982 | 753.103 | OH | a1 | B05 | b3 | C18 | c2 | D02 | F |
| 2-1-E00-1-0983 | 755.083 | OH | a1 | B06 | b3 | C18 | c2 | D02 | F |
| 2-1-E00-1-0984 | 756.082 | OH | a2 | B18 | b3 | C18 | c2 | D02 | F |
| 2-1-E00-1-0985 | 757.081 | OH | a2 | B19 | b3 | C16 | c2 | D02 | F |
| 2-1-E00-1-0986 | 759.043 | OH | a2 | B19 | b3 | C17 | c2 | D02 | F |
| 2-1-E00-1-0987 | 761.053 | OH | a1 | B07 | b3 | C18 | c2 | D02 | F |
| 2-1-E00-1-0988 | 765.067 | OH | a1 | B08 | b3 | C16 | c2 | D02 | F |
| 2-1-E00-1-0989 | 767.028 | OH | a1 | B08 | b3 | C17 | c2 | D02 | F |
| 2-1-E00-1-0990 | 773.076 | OH | a2 | B20 | b3 | C16 | c2 | D02 | F |
| 2-1-E00-1-0991 | 774.054 | OH | a2 | B19 | b3 | C18 | c2 | D02 | F |
| 2-1-E00-1-0992 | 775.038 | OH | a2 | B20 | b3 | C17 | c2 | D02 | F |
| 2-1-E00-1-0993 | 776.087 | OH | a1 | B09 | b3 | C16 | c2 | D02 | F |
| 2-1-E00-1-0994 | 778.049 | OH | a1 | B09 | b3 | C17 | c2 | D02 | F |
| 2-1-E00-1-0995 | 782.039 | OH | a1 | B08 | b3 | C18 | c2 | D02 | F |
| 2-1-E00-1-0996 | 790.049 | OH | a2 | B20 | b3 | C18 | c2 | D02 | F |
| 2-1-E00-1-0997 | 792.082 | OH | a1 | B10 | b3 | C16 | c2 | D02 | F |
| 2-1-E00-1-0998 | 793.059 | OH | a1 | B09 | b3 | C18 | c2 | D02 | F |
| 2-1-E00-1-0999 | 794.043 | OH | a1 | B10 | b3 | C17 | c2 | D02 | F |
| 2-1-E00-1-1000 | 809.054 | OH | a1 | B10 | b3 | C18 | c2 | D02 | F |

Example 8-7: Synthesis of mixture 2-1-d1E01-0 to mixture 2-1-d1E04-0 by step d1

A reaction formula in step d1E01 performed using BB2-1-54 will be illustrated below.

[Formula 641]

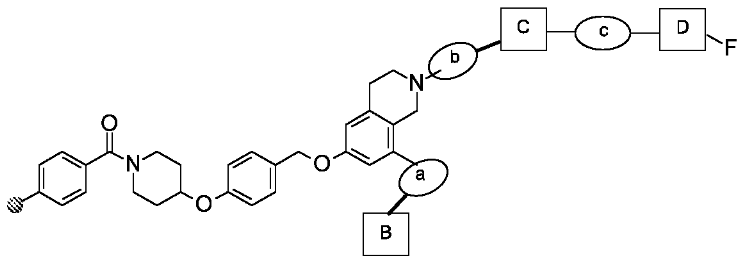

2-1-E00-0

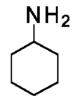

BB2-1-54

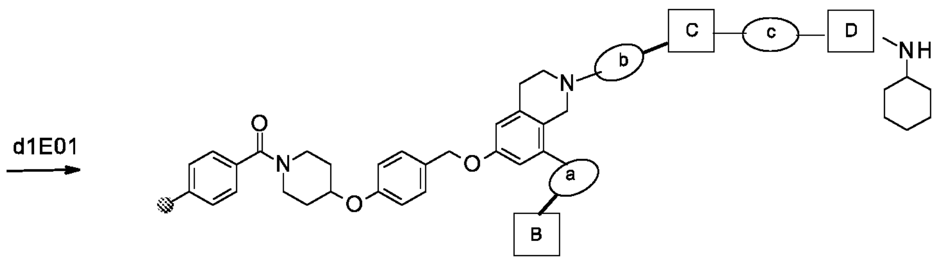


2-1-d1E01-0

Mixture 2-1-d1E01-0 to mixture 2-1-d1E04-0 were synthesized as follows using BB2-1-54 to BB2-1-57.

2-1-E00-0 (240 mg, amount of the mixture supported on the solid phase: 0.186 mmol/g) and NMP (2.9 mL) were added to four 8 mL glass vials and shaken at room temperature for 1 hour. Separate amines (0.446 mmol) described in Table 8-7-1 were added to the glass vials, respectively. 1,8-Bis(dimethylamino)naphthalene (144 mg, 0.670 mmol) was added thereto, and each mixture was shaken at 80°C for 24 hours.

Solid-phase purification

The suspension of the reaction solution and the resin was transferred to a column with a filter, filtered, and washed three times with NMP/water = 1/1 (5 mL), three times with NMP (5 mL), three times with 0.05 M $TBAHSO_4$ + DTBP in NMP (5 mL), three times with 0.05 M NMM in NMP (5 mL), three times with NMP/water = 1/1 (5 mL), three times with NMP (5 mL), three times with MeOH (5 mL), three times with DCM (5 mL), and three times with heptane (5 mL), and the obtained solid phase was dried under reduced pressure to obtain the mixtures described in the table.

[Table 386]

| [Table 8-7-1] Starting material mixture used and obtained mixture in step d1 | | | |
|---|---|---|---|
| Amine used | Molar number | Amount used | Obtained mixture |
| BB2-1-54 | 0.446 mmol | 51.2μL | 2-1-d1E01-0 |
| BB2-1-55 | 0.446 mmol | 53.0μL | 2-1-d1E02-0 |
| BB2-1-56 | 0.446 mmol | 68.4 mg | 2-1-d1E03-0 |
| BB2-1-57 | 0.446 mmol | 119 mg | 2-1-d1E04-0 |

Mixture 2-1-d1E01-0 to mixture 2-1-d1E04-0 can be treated by cleavage in the same manner as in Example 8-1-1 to obtain mixture 2-1-d1E01-1 to mixture 2-1-d1E04-1. The details of the mixtures are shown in Table 8-7-2 to Table 8-7-5.

A notation method in each table will be described. In Table 8-7-2 to Table 8-7-5, each compound that may be contained in mixture 2-1-d1E01-1 to mixture 2-1-d 1 E04-1 is indicated by ID, and core blocks and linkers in combination in the structures of mixture 2-1- d1E01-1 to mixture 2-1-d1E04-1 are indicated by symbols or chemical formulas. The

correspondence between the symbols and specific structures are shown in Figure 4.

In Table 8-7-2 to Table 8-7-5, "×" is represented by a chemical formula.

[Formula 642]

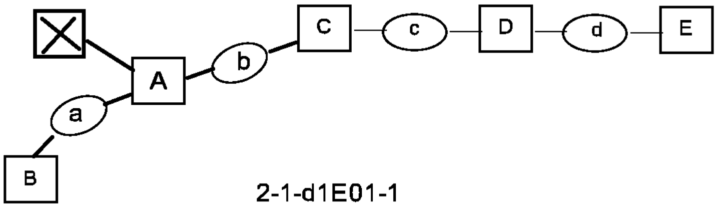


2-1-d1E01-1

For example, when ID is 2-1-d1E01-1-0001, the compound is represented by the following structural formula.

[Formula 643]

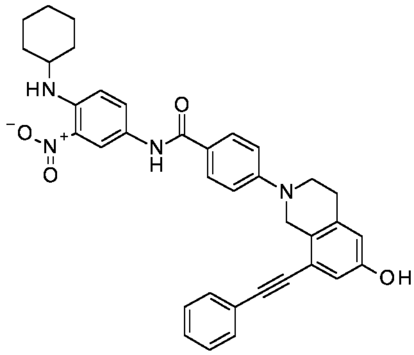

2-1-d1E01-1-0001

[Table 387]

| Notation in [Table 8-7-2] | | | | | | | | | |
|---|---|---|---|---|---|---|---|---|---|
| ID | | a | B | b | C | c | D | d | E |
| 2-1-d1E01-1-0001 | OH | a2 | B11 | b1 | C08 | c1 | D01 | d1 | E01 |

[Table 388]

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0001 | 586.258 | OH | a2 | B11 | b1 | C08 | c1 | D01 | d1 | E01 |
| 2-1-d1E01-1-0002 | 587.253 | OH | a2 | B12 | b1 | C08 | c1 | D01 | d1 | E01 |
| 2-1-d1E01-1-0003 | 587.253 | OH | a2 | B11 | b1 | C11 | c1 | D01 | d1 | E01 |
| 2-1-d1E01-1-0004 | 588.249 | OH | a2 | B11 | b1 | C10 | c1 | D01 | d1 | E01 |
| 2-1-d1E01-1-0005 | 588.249 | OH | a2 | B12 | b1 | C11 | c1 | D01 | d1 | E01 |
| 2-1-d1E01-1-0006 | 589.244 | OH | a2 | B12 | b1 | C10 | c1 | D01 | d1 | E01 |
| 2-1-d1E01-1-0007 | 593.210 | OH | a2 | B11 | b1 | C12 | c1 | D01 | d1 | E01 |
| 2-1-d1E01-1-0008 | 594.205 | OH | a2 | B12 | b1 | C12 | c1 | D01 | d1 | E01 |
| 2-1-d1E01-1-0009 | 600.274 | OH | a2 | B13 | b1 | C08 | c1 | D01 | d1 | E01 |
| 2-1-d1E01-1-0010 | 601.269 | OH | a2 | B13 | b1 | C11 | c1 | D01 | d1 | E01 |
| 2-1-d1E01-1-0011 | 602.264 | OH | a2 | B13 | b1 | C10 | c1 | D01 | d1 | E01 |
| 2-1-d1E01-1-0012 | 604.232 | OH | a2 | B11 | b2 | C01 | c1 | D01 | d1 | E01 |
| 2-1-d1E01-1-0013 | 604.249 | OH | a2 | B14 | b1 | C08 | c1 | D01 | d1 | E01 |
| 2-1-d1E01-1-0014 | 605.227 | OH | a2 | B12 | b2 | C01 | c1 | D01 | d1 | E01 |
| 2-1-d1E01-1-0015 | 605.244 | OH | a2 | B14 | b1 | C11 | c1 | D01 | d1 | E01 |
| 2-1-d1E01-1-0016 | 605.264 | OH | a1 | B01 | b1 | C08 | c1 | D01 | d1 | E01 |
| 2-1-d1E01-1-0017 | 606.239 | OH | a2 | B14 | b1 | C10 | c1 | D01 | d1 | E01 |
| 2-1-d1E01-1-0018 | 606.259 | OH | a1 | B02 | b1 | C08 | c1 | D01 | d1 | E01 |
| 2-1-d1E01-1-0019 | 606.259 | OH | a1 | B01 | b1 | C11 | c1 | D01 | d1 | E01 |
| 2-1-d1E01-1-0020 | 607.225 | OH | a2 | B13 | b1 | C12 | c1 | D01 | d1 | E01 |
| 2-1-d1E01-1-0021 | 607.254 | OH | a1 | B01 | b1 | C10 | c1 | D01 | d1 | E01 |
| 2-1-d1E01-1-0022 | 607.254 | OH | a1 | B02 | b1 | C11 | c1 | D01 | d1 | E01 |
| 2-1-d1E01-1-0023 | 608.250 | OH | a1 | B02 | b1 | C10 | c1 | D01 | d1 | E01 |
| 2-1-d1E01-1-0024 | 611.200 | OH | a2 | B14 | b1 | C12 | c1 | D01 | d1 | E01 |
| 2-1-d1E01-1-0025 | 612.215 | OH | a1 | B01 | b1 | C12 | c1 | D01 | d1 | E01 |
| 2-1-d1E01-1-0026 | 613.211 | OH | a1 | B02 | b1 | C12 | c1 | D01 | d1 | E01 |
| 2-1-d1E01-1-0027 | 614.253 | OH | a2 | B11 | b2 | C02 | c1 | D01 | d1 | E01 |
| 2-1-d1E01-1-0028 | 614.289 | OH | a2 | B15 | b1 | C08 | c1 | D01 | d1 | E01 |
| 2-1-d1E01-1-0029 | 615.248 | OH | a2 | B12 | b2 | C02 | c1 | D01 | d1 | E01 |
| 2-1-d1E01-1-0030 | 615.248 | OH | a2 | B11 | b2 | C03 | c1 | D01 | d1 | E01 |
| 2-1-d1E01-1-0031 | 615.285 | OH | a2 | B15 | b1 | C11 | c1 | D01 | d1 | E01 |
| 2-1-d1E01-1-0032 | 616.243 | OH | a2 | B12 | b2 | C03 | c1 | D01 | d1 | E01 |
| 2-1-d1E01-1-0033 | 616.269 | OH | a2 | B16 | b1 | C08 | c1 | D01 | d1 | E01 |
| 2-1-d1E01-1-0034 | 616.280 | OH | a2 | B15 | b1 | C10 | c1 | D01 | d1 | E01 |
| 2-1-d1E01-1-0035 | 617.264 | OH | a2 | B16 | b1 | C11 | c1 | D01 | d1 | E01 |
| 2-1-d1E01-1-0036 | 618.248 | OH | a2 | B13 | b2 | C01 | c1 | D01 | d1 | E01 |
| 2-1-d1E01-1-0037 | 618.259 | OH | a2 | B16 | b1 | C10 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0038 | 619.279 | OH | a1 | B03 | b1 | C08 | c1 | D01 | d1 | E01 |
| 2-1-d1E01-1-0039 | 620.209 | OH | a2 | B11 | b2 | C06 | c1 | D01 | d1 | E01 |
| 2-1-d1E01-1-0040 | 620.275 | OH | a1 | B03 | b1 | C11 | c1 | D01 | d1 | E01 |
| 2-1-d1E01-1-0041 | 621.205 | OH | a2 | B12 | b2 | C06 | c1 | D01 | d1 | E01 |
| 2-1-d1E01-1-0042 | 621.241 | OH | a2 | B15 | b1 | C12 | c1 | D01 | d1 | E01 |
| 2-1-d1E01-1-0043 | 621.270 | OH | a1 | B03 | b1 | C10 | c1 | D01 | d1 | E01 |
| 2-1-d1E01-1-0044 | 622.223 | OH | a2 | B14 | b2 | C01 | c1 | D01 | d1 | E01 |
| 2-1-d1E01-1-0045 | 622.239 | OH | a2 | B17 | b1 | C08 | c1 | D01 | d1 | E01 |
| 2-1-d1E01-1-0046 | 623.220 | OH | a2 | B16 | b1 | C12 | c1 | D01 | d1 | E01 |
| 2-1-d1E01-1-0047 | 623.234 | OH | a2 | B17 | b1 | C11 | c1 | D01 | d1 | E01 |
| 2-1-d1E01-1-0048 | 623.238 | OH | a1 | B01 | b2 | C01 | c1 | D01 | d1 | E01 |
| 2-1-d1E01-1-0049 | 623.254 | OH | a1 | B04 | b1 | C08 | c1 | D01 | d1 | E01 |
| 2-1-d1E01-1-0050 | 624.230 | OH | a2 | B17 | b1 | C10 | c1 | D01 | d1 | E01 |
| 2-1-d1E01-1-0051 | 624.233 | OH | a1 | B02 | b2 | C01 | c1 | D01 | d1 | E01 |
| 2-1-d1E01-1-0052 | 624.250 | OH | a1 | B04 | b1 | C11 | c1 | D01 | d1 | E01 |
| 2-1-d1E01-1-0053 | 625.245 | OH | a1 | B04 | b1 | C10 | c1 | D01 | d1 | E01 |
| 2-1-d1E01-1-0054 | 626.231 | OH | a1 | B03 | b1 | C12 | c1 | D01 | d1 | E01 |
| 2-1-d1E01-1-0055 | 628.269 | OH | a2 | B13 | b2 | C02 | c1 | D01 | d1 | E01 |
| 2-1-d1E01-1-0056 | 629.191 | OH | a2 | B17 | b1 | C12 | c1 | D01 | d1 | E01 |
| 2-1-d1E01-1-0057 | 629.264 | OH | a2 | B13 | b2 | C03 | c1 | D01 | d1 | E01 |
| 2-1-d1E01-1-0058 | 629.264 | OH | a2 | B11 | b2 | C05 | c1 | D01 | d1 | E01 |
| 2-1-d1E01-1-0059 | 630.206 | OH | a1 | B04 | b1 | C12 | c1 | D01 | d1 | E01 |
| 2-1-d1E01-1-0060 | 630.259 | OH | a2 | B12 | b2 | C05 | c1 | D01 | d1 | E01 |
| 2-1-d1E01-1-0061 | 632.243 | OH | a2 | B14 | b2 | C02 | c1 | D01 | d1 | E01 |
| 2-1-d1E01-1-0062 | 632.243 | OH | a2 | B11 | b2 | C04 | c1 | D01 | d1 | E01 |
| 2-1-d1E01-1-0063 | 632.263 | OH | a2 | B15 | b2 | C01 | c1 | D01 | d1 | E01 |
| 2-1-d1E01-1-0064 | 633.239 | OH | a2 | B12 | b2 | C04 | c1 | D01 | d1 | E01 |
| 2-1-d1E01-1-0065 | 633.239 | OH | a2 | B14 | b2 | C03 | c1 | D01 | d1 | E01 |
| 2-1-d1E01-1-0066 | 633.259 | OH | a1 | B01 | b2 | C02 | c1 | D01 | d1 | E01 |
| 2-1-d1E01-1-0067 | 633.295 | OH | a1 | B05 | b1 | C08 | c1 | D01 | d1 | E01 |
| 2-1-d1E01-1-0068 | 634.225 | OH | a2 | B13 | b2 | C06 | c1 | D01 | d1 | E01 |
| 2-1-d1E01-1-0069 | 634.243 | OH | a2 | B16 | b2 | C01 | c1 | D01 | d1 | E01 |
| 2-1-d1E01-1-0070 | 634.254 | OH | a1 | B02 | b2 | C02 | c1 | D01 | d1 | E01 |
| 2-1-d1E01-1-0071 | 634.254 | OH | a1 | B01 | b2 | C03 | c1 | D01 | d1 | E01 |
| 2-1-d1E01-1-0072 | 634.290 | OH | a1 | B05 | b1 | C11 | c1 | D01 | d1 | E01 |
| 2-1-d1E01-1-0073 | 635.249 | OH | a1 | B02 | b2 | C03 | c1 | D01 | d1 | E01 |
| 2-1-d1E01-1-0074 | 635.274 | OH | a1 | B06 | b1 | C08 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0075 | 635.286 | OH | a1 | B05 | b1 | C10 | c1 | D01 | d1 | E01 |
| 2-1-d1E01-1-0076 | 636.270 | OH | a1 | B06 | b1 | C11 | c1 | D01 | d1 | E01 |
| 2-1-d1E01-1-0077 | 636.274 | OH | a2 | B18 | b1 | C08 | c1 | D01 | d1 | E01 |
| 2-1-d1E01-1-0078 | 637.254 | OH | a1 | B03 | b2 | C01 | c1 | D01 | d1 | E01 |
| 2-1-d1E01-1-0079 | 637.265 | OH | a1 | B06 | b1 | C10 | c1 | D01 | d1 | E01 |
| 2-1-d1E01-1-0080 | 637.269 | OH | a2 | B18 | b1 | C11 | c1 | D01 | d1 | E01 |
| 2-1-d1E01-1-0081 | 638.200 | OH | a2 | B14 | b2 | C06 | c1 | D01 | d1 | E01 |
| 2-1-d1E01-1-0082 | 638.264 | OH | a2 | B18 | b1 | C10 | c1 | D01 | d1 | E01 |
| 2-1-d1E01-1-0083 | 638.264 | OH | a2 | B11 | b1 | C13 | c1 | D01 | d1 | E01 |
| 2-1-d1E01-1-0084 | 639.215 | OH | a1 | B01 | b2 | C06 | c1 | D01 | d1 | E01 |
| 2-1-d1E01-1-0085 | 639.259 | OH | a2 | B12 | b1 | C13 | c1 | D01 | d1 | E01 |
| 2-1-d1E01-1-0086 | 640.199 | OH | a2 | B11 | b3 | C15 | c1 | D01 | d1 | E01 |
| 2-1-d1E01-1-0087 | 640.210 | OH | a1 | B02 | b2 | C06 | c1 | D01 | d1 | E01 |
| 2-1-d1E01-1-0088 | 640.213 | OH | a2 | B17 | b2 | C01 | c1 | D01 | d1 | E01 |
| 2-1-d1E01-1-0089 | 640.247 | OH | a1 | B05 | b1 | C12 | c1 | D01 | d1 | E01 |
| 2-1-d1E01-1-0090 | 641.194 | OH | a2 | B12 | b3 | C15 | c1 | D01 | d1 | E01 |
| 2-1-d1E01-1-0091 | 641.229 | OH | a1 | B04 | b2 | C01 | c1 | D01 | d1 | E01 |
| 2-1-d1E01-1-0092 | 641.245 | OH | a1 | B07 | b1 | C08 | c1 | D01 | d1 | E01 |
| 2-1-d1E01-1-0093 | 642.226 | OH | a1 | B06 | b1 | C12 | c1 | D01 | d1 | E01 |
| 2-1-d1E01-1-0094 | 642.230 | OH | a2 | B11 | b1 | C14 | c1 | D01 | d1 | E01 |
| 2-1-d1E01-1-0095 | 642.240 | OH | a1 | B07 | b1 | C11 | c1 | D01 | d1 | E01 |
| 2-1-d1E01-1-0096 | 642.251 | OH | a2 | B11 | b2 | C33 | c3 | D02 | d1 | E01 |
| 2-1-d1E01-1-0097 | 642.284 | OH | a2 | B15 | b2 | C02 | c1 | D01 | d1 | E01 |
| 2-1-d1E01-1-0098 | 643.225 | OH | a2 | B18 | b1 | C12 | c1 | D01 | d1 | E01 |
| 2-1-d1E01-1-0099 | 643.225 | OH | a2 | B12 | b1 | C14 | c1 | D01 | d1 | E01 |
| 2-1-d1E01-1-0100 | 643.235 | OH | a1 | B07 | b1 | C10 | c1 | D01 | d1 | E01 |
| 2-1-d1E01-1-0101 | 643.246 | OH | a2 | B12 | b2 | C33 | c3 | D02 | d1 | E01 |
| 2-1-d1E01-1-0102 | 643.279 | OH | a2 | B15 | b2 | C03 | c1 | D01 | d1 | E01 |
| 2-1-d1E01-1-0103 | 643.279 | OH | a2 | B13 | b2 | COS | c1 | D01 | d1 | E01 |
| 2-1-d1E01-1-0104 | 644.263 | OH | a2 | B16 | b2 | C02 | c1 | D01 | d1 | E01 |
| 2-1-d1E01-1-0105 | 645.259 | OH | a2 | B16 | b2 | C03 | c1 | D01 | d1 | E01 |
| 2-1-d1E01-1-0106 | 646.259 | OH | a2 | B13 | b2 | C04 | c1 | D01 | d1 | E01 |
| 2-1-d1E01-1-0107 | 646.316 | OH | a2 | B11 | b2 | C07 | c1 | D01 | d1 | E01 |
| 2-1-d1E01-1-0108 | 647.254 | OH | a2 | B14 | b2 | COS | c1 | D01 | d1 | E01 |
| 2-1-d1E01-1-0109 | 647.274 | OH | a1 | B03 | b2 | C02 | c1 | D01 | d1 | E01 |
| 2-1-d1E01-1-0110 | 647.311 | OH | a2 | B12 | b2 | C07 | c1 | D01 | d1 | E01 |
| 2-1-d1E01-1-0111 | 648.197 | OH | a1 | B07 | b1 | C12 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0112 | 648.241 | OH | a2 | B15 | b2 | C06 | c1 | D01 | d1 | E01 |
| 2-1-d1E01-1-0113 | 648.270 | OH | a1 | B03 | b2 | C03 | c1 | D01 | d1 | E01 |
| 2-1-d1E01-1-0114 | 648.270 | OH | a1 | B01 | b2 | COS | c1 | D01 | d1 | E01 |
| 2-1-d1E01-1-0115 | 649.265 | OH | a1 | B02 | b2 | COS | c1 | D01 | d1 | E01 |
| 2-1-d1E01-1-0116 | 650.220 | OH | a2 | B16 | b2 | C06 | c1 | D01 | d1 | E01 |
| 2-1-d1E01-1-0117 | 650.220 | OH | a2 | B11 | b3 | C08 | c1 | D01 | d1 | E01 |
| 2-1-d1E01-1-0118 | 650.220 | OH | a2 | B11 | b1 | C08 | c2 | D02 | d1 | E01 |
| 2-1-d1E01-1-0119 | 650.234 | OH | a2 | B17 | b2 | C02 | c1 | D01 | d1 | E01 |
| 2-1-d1E01-1-0120 | 650.234 | OH | a2 | B14 | b2 | C04 | c1 | D01 | d1 | E01 |
| 2-1-d1E01-1-0121 | 651.215 | OH | a2 | B12 | b3 | C08 | c1 | D01 | d1 | E01 |
| 2-1-d1E01-1-0122 | 651.215 | OH | a2 | B12 | b1 | C08 | c2 | D02 | d1 | E01 |
| 2-1-d1E01-1-0123 | 651.215 | OH | a2 | B11 | b1 | C11 | c2 | D02 | d1 | E01 |
| 2-1-d1E01-1-0124 | 651.229 | OH | a2 | B17 | b2 | C03 | c1 | D01 | d1 | E01 |
| 2-1-d1E01-1-0125 | 651.249 | OH | a1 | B04 | b2 | C02 | c1 | D01 | d1 | E01 |
| 2-1-d1E01-1-0126 | 651.249 | OH | a1 | B01 | b2 | C04 | c1 | D01 | d1 | E01 |
| 2-1-d1E01-1-0127 | 651.269 | OH | a1 | B05 | b2 | C01 | c1 | D01 | d1 | E01 |
| 2-1-d1E01-1-0128 | 652.210 | OH | a2 | B11 | b1 | C10 | c2 | D02 | d1 | E01 |
| 2-1-d1E01-1-0129 | 652.210 | OH | a2 | B12 | b1 | C11 | c2 | D02 | d1 | E01 |
| 2-1-d1E01-1-0130 | 652.245 | OH | a1 | B02 | b2 | C04 | c1 | D01 | d1 | E01 |
| 2-1-d1E01-1-0131 | 652.245 | OH | a1 | B04 | b2 | C03 | c1 | D01 | d1 | E01 |
| 2-1-d1E01-1-0132 | 652.280 | OH | a2 | B13 | b1 | C13 | c1 | D01 | d1 | E01 |
| 2-1-d1E01-1-0133 | 653.206 | OH | a2 | B12 | b1 | C10 | c2 | D02 | d1 | E01 |
| 2-1-d1E01-1-0134 | 653.231 | OH | a1 | B03 | b2 | C06 | c1 | D01 | d1 | E01 |
| 2-1-d1E01-1-0135 | 653.249 | OH | a1 | B06 | b2 | C01 | c1 | D01 | d1 | E01 |
| 2-1-d1E01-1-0136 | 654.215 | OH | a2 | B13 | b3 | C15 | c1 | D01 | d1 | E01 |
| 2-1-d1E01-1-0137 | 654.245 | OH | a2 | B19 | b1 | C08 | c1 | D01 | d1 | E01 |
| 2-1-d1E01-1-0138 | 654.245 | OH | a2 | B11 | b1 | C09 | c1 | D01 | d1 | E01 |
| 2-1-d1E01-1-0139 | 654.248 | OH | a2 | B18 | b2 | C01 | c1 | D01 | d1 | E01 |
| 2-1-d1E01-1-0140 | 655.241 | OH | a2 | B12 | b1 | C09 | c1 | D01 | d1 | E01 |
| 2-1-d1E01-1-0141 | 655.241 | OH | a2 | B19 | b1 | C11 | c1 | D01 | d1 | E01 |
| 2-1-d1E01-1-0142 | 656.176 | OH | a2 | B11 | b3 | C06 | c1 | D01 | d1 | E01 |
| 2-1-d1E01-1-0143 | 656.190 | OH | a2 | B17 | b2 | C06 | c1 | D01 | d1 | E01 |
| 2-1-d1E01-1-0144 | 656.236 | OH | a2 | B19 | b1 | C10 | c1 | D01 | d1 | E01 |
| 2-1-d1E01-1-0145 | 656.246 | OH | a2 | B13 | b1 | C14 | c1 | D01 | d1 | E01 |
| 2-1-d1E01-1-0146 | 656.255 | OH | a2 | B14 | b1 | C13 | c1 | D01 | d1 | E01 |
| 2-1-d1E01-1-0147 | 656.267 | OH | a2 | B13 | b2 | C33 | c3 | D02 | d1 | E01 |
| 2-1-d1E01-1-0148 | 657.172 | OH | a2 | B12 | b3 | C06 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0149 | 657.172 | OH | a2 | B11 | b1 | C12 | c2 | D02 | d1 | E01 |
| 2-1-d1E01-1-0150 | 657.206 | OH | a1 | B04 | b2 | C06 | c1 | D01 | d1 | E01 |
| 2-1-d1E01-1-0151 | 657.270 | OH | a1 | B01 | b1 | C13 | c1 | D01 | d1 | E01 |
| 2-1-d1E01-1-0152 | 657.295 | OH | a2 | B15 | b2 | COS | c1 | D01 | d1 | E01 |
| 2-1-d1E01-1-0153 | 658.167 | OH | a2 | B12 | b1 | C12 | c2 | D02 | d1 | E01 |
| 2-1-d1E01-1-0154 | 658.190 | OH | a2 | B14 | b3 | C15 | c1 | D01 | d1 | E01 |
| 2-1-d1E01-1-0155 | 658.265 | OH | a1 | B02 | b1 | C13 | c1 | D01 | d1 | E01 |
| 2-1-d1E01-1-0156 | 659.205 | OH | a1 | B01 | b3 | C15 | c1 | D01 | d1 | E01 |
| 2-1-d1E01-1-0157 | 659.219 | OH | a1 | B07 | b2 | C01 | c1 | D01 | d1 | E01 |
| 2-1-d1E01-1-0158 | 659.274 | OH | a2 | B16 | b2 | C05 | c1 | D01 | d1 | E01 |
| 2-1-d1E01-1-0159 | 660.200 | OH | a1 | B02 | b3 | C15 | c1 | D01 | d1 | E01 |
| 2-1-d1E01-1-0160 | 660.221 | OH | a2 | B14 | b1 | C14 | c1 | D01 | d1 | E01 |
| 2-1-d1E01-1-0161 | 660.242 | OH | a2 | B14 | b2 | C33 | c3 | D02 | d1 | E01 |
| 2-1-d1E01-1-0162 | 660.275 | OH | a2 | B15 | b2 | C04 | c1 | D01 | d1 | E01 |
| 2-1-d1E01-1-0163 | 660.331 | OH | a2 | B13 | b2 | C07 | c1 | D01 | d1 | E01 |
| 2-1-d1E01-1-0164 | 661.197 | OH | a2 | B19 | b1 | C12 | c1 | D01 | d1 | E01 |
| 2-1-d1E01-1-0165 | 661.236 | OH | a1 | B01 | b1 | C14 | c1 | D01 | d1 | E01 |
| 2-1-d1E01-1-0166 | 661.257 | OH | a1 | B01 | b2 | C33 | c3 | D02 | d1 | E01 |
| 2-1-d1E01-1-0167 | 661.290 | OH | a1 | B05 | b2 | C02 | c1 | D01 | d1 | E01 |
| 2-1-d1E01-1-0168 | 662.231 | OH | a1 | B08 | b1 | C08 | c1 | D01 | d1 | E01 |
| 2-1-d1E01-1-0169 | 662.231 | OH | a1 | B02 | b1 | C14 | c1 | D01 | d1 | E01 |
| 2-1-d1E01-1-0170 | 662.252 | OH | a1 | B02 | b2 | C33 | c3 | D02 | d1 | E01 |
| 2-1-d1E01-1-0171 | 662.254 | OH | a2 | B16 | b2 | C04 | c1 | D01 | d1 | E01 |
| 2-1-d1E01-1-0172 | 662.256 | OH | a2 | B11 | b1 | C30 | c3 | D02 | d1 | E01 |
| 2-1-d1E01-1-0173 | 662.285 | OH | a1 | B05 | b2 | C03 | c1 | D01 | d1 | E01 |
| 2-1-d1E01-1-0174 | 662.285 | OH | a1 | B03 | b2 | COS | c1 | D01 | d1 | E01 |
| 2-1-d1E01-1-0175 | 663.226 | OH | a1 | B08 | b1 | C11 | c1 | D01 | d1 | E01 |
| 2-1-d1E01-1-0176 | 663.252 | OH | a2 | B12 | b1 | C30 | c3 | D02 | d1 | E01 |
| 2-1-d1E01-1-0177 | 663.269 | OH | a1 | B06 | b2 | C02 | c1 | D01 | d1 | E01 |
| 2-1-d1E01-1-0178 | 664.222 | OH | a1 | B08 | b1 | C10 | c1 | D01 | d1 | E01 |
| 2-1-d1E01-1-0179 | 664.236 | OH | a2 | B13 | b3 | C08 | c1 | D01 | d1 | E01 |
| 2-1-d1E01-1-0180 | 664.236 | OH | a2 | B13 | b1 | C08 | c2 | D02 | d1 | E01 |
| 2-1-d1E01-1-0181 | 664.236 | OH | a2 | B11 | b2 | C19 | c3 | D02 | d1 | E01 |
| 2-1-d1E01-1-0182 | 664.265 | OH | a1 | B06 | b2 | C03 | c1 | D01 | d1 | E01 |
| 2-1-d1E01-1-0183 | 664.269 | OH | a2 | B18 | b2 | C02 | c1 | D01 | d1 | E01 |
| 2-1-d1E01-1-0184 | 664.306 | OH | a2 | B14 | b2 | C07 | c1 | D01 | d1 | E01 |
| 2-1-d1E01-1-0185 | 665.231 | OH | a2 | B13 | b1 | C11 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0186 | 665.231 | OH | a2 | B12 | b2 | C19 | c3 | D02 | d1 | E01 |
| 2-1-d1E01-1-0187 | 665.231 | OH | a2 | B11 | b2 | C27 | c3 | D02 | d1 | E01 |
| 2-1-d1E01-1-0188 | 665.245 | OH | a2 | B17 | b2 | C05 | c1 | D01 | d1 | E01 |
| 2-1-d1E01-1-0189 | 665.264 | OH | a2 | B18 | b2 | C03 | c1 | D01 | d1 | E01 |
| 2-1-d1E01-1-0190 | 665.265 | OH | a1 | B03 | b2 | C04 | c1 | D01 | d1 | E01 |
| 2-1-d1E01-1-0191 | 665.321 | OH | a1 | B01 | b2 | C07 | c1 | D01 | d1 | E01 |
| 2-1-d1E01-1-0192 | 666.226 | OH | a2 | B13 | b1 | C10 | c2 | D02 | d1 | E01 |
| 2-1-d1E01-1-0193 | 666.226 | OH | a2 | B12 | b2 | C27 | c3 | D02 | d1 | E01 |
| 2-1-d1E01-1-0194 | 666.260 | OH | a1 | B04 | b2 | C05 | c1 | D01 | d1 | E01 |
| 2-1-d1E01-1-0195 | 666.295 | OH | a2 | B15 | b1 | C13 | c1 | D01 | d1 | E01 |
| 2-1-d1E01-1-0196 | 666.317 | OH | a1 | B02 | b2 | C07 | c1 | D01 | d1 | E01 |
| 2-1-d1E01-1-0197 | 667.246 | OH | a1 | B05 | b2 | C06 | c1 | D01 | d1 | E01 |
| 2-1-d1E01-1-0198 | 668.194 | OH | a2 | B11 | b2 | C01 | c2 | D02 | d1 | E01 |
| 2-1-d1E01-1-0199 | 668.210 | OH | a2 | B14 | b3 | C08 | c1 | D01 | d1 | E01 |
| 2-1-d1E01-1-0200 | 668.210 | OH | a2 | B14 | b1 | C08 | c2 | D02 | d1 | E01 |
| 2-1-d1E01-1-0201 | 668.225 | OH | a2 | B17 | b2 | C04 | c1 | D01 | d1 | E01 |
| 2-1-d1E01-1-0202 | 668.23 | OH | a2 | B15 | b3 | C15 | c1 | D01 | d1 | E01 |
| 2-1-d1E01-1-0203 | 668.261 | OH | a2 | B13 | b1 | C09 | c1 | D01 | d1 | E01 |
| 2-1-d1E01-1-0204 | 668.275 | OH | a2 | B16 | b1 | C13 | c1 | D01 | d1 | E01 |
| 2-1-d1E01-1-0205 | 669.183 | OH | a1 | B08 | b1 | C12 | c1 | D01 | d1 | E01 |
| 2-1-d1E01-1-0206 | 669.189 | OH | a2 | B12 | b2 | C01 | c2 | D02 | d1 | E01 |
| 2-1-d1E01-1-0207 | 669.206 | OH | a2 | B14 | b1 | C11 | c2 | D02 | d1 | E01 |
| 2-1-d1E01-1-0208 | 669.226 | OH | a1 | B06 | b2 | C06 | c1 | D01 | d1 | E01 |
| 2-1-d1E01-1-0209 | 669.226 | OH | a1 | B01 | b3 | C08 | c1 | D01 | d1 | E01 |
| 2-1-d1E01-1-0210 | 669.226 | OH | a1 | B01 | b1 | C08 | c2 | D02 | d1 | E01 |
| 2-1-d1E01-1-0211 | 669.240 | OH | a1 | B07 | b2 | C02 | c1 | D01 | d1 | E01 |
| 2-1-d1E01-1-0212 | 669.240 | OH | a1 | B04 | b2 | C04 | c1 | D01 | d1 | E01 |
| 2-1-d1E01-1-0213 | 670.192 | OH | a2 | B13 | b3 | C06 | c1 | D01 | d1 | E01 |
| 2-1-d1E01-1-0214 | 670.201 | OH | a2 | B14 | b1 | C10 | c2 | D02 | d1 | E01 |
| 2-1-d1E01-1-0215 | 670.210 | OH | a2 | B16 | b3 | C15 | c1 | D01 | d1 | E01 |
| 2-1-d1E01-1-0216 | 670.221 | OH | a1 | B02 | b3 | C08 | c1 | D01 | d1 | E01 |
| 2-1-d1E01-1-0217 | 670.221 | OH | a1 | B02 | b1 | C08 | c2 | D02 | d1 | E01 |
| 2-1-d1E01-1-0218 | 670.221 | OH | a1 | B01 | b1 | C11 | c2 | D02 | d1 | E01 |
| 2-1-d1E01-1-0219 | 670.225 | OH | a2 | B18 | b2 | C06 | c1 | D01 | d1 | E01 |
| 2-1-d1E01-1-0220 | 670.235 | OH | a1 | B07 | b2 | C03 | c1 | D01 | d1 | E01 |
| 2-1-d1E01-1-0221 | 670.240 | OH | a2 | B20 | b1 | C08 | c1 | D01 | d1 | E01 |
| 2-1-d1E01-1-0222 | 670.261 | OH | a2 | B15 | b1 | C14 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0223 | 670.283 | OH | a2 | B15 | b2 | C33 | c3 | D02 | d1 | E01 |
| 2-1-d1E01-1-0224 | 671.187 | OH | a2 | B13 | b1 | C12 | c2 | D02 | d1 | E01 |
| 2-1-d1E01-1-0225 | 671.216 | OH | a1 | B01 | b1 | C10 | c2 | D02 | d1 | E01 |
| 2-1-d1E01-1-0226 | 671.216 | OH | a1 | B02 | b1 | C11 | c2 | D02 | d1 | E01 |
| 2-1-d1E01-1-0227 | 671.236 | OH | a2 | B20 | b1 | C11 | c1 | D01 | d1 | E01 |
| 2-1-d1E01-1-0228 | 671.286 | OH | a1 | B03 | b1 | C13 | c1 | D01 | d1 | E01 |
| 2-1-d1E01-1-0229 | 672.211 | OH | a1 | B02 | b1 | C10 | c2 | D02 | d1 | E01 |
| 2-1-d1E01-1-0230 | 672.220 | OH | a2 | B19 | b2 | C01 | c1 | D01 | d1 | E01 |
| 2-1-d1E01-1-0231 | 672.231 | OH | a2 | B20 | b1 | C10 | c1 | D01 | d1 | E01 |
| 2-1-d1E01-1-0232 | 672.236 | OH | a2 | B14 | b1 | C09 | c1 | D01 | d1 | E01 |
| 2-1-d1E01-1-0233 | 672.241 | OH | a2 | B16 | b1 | C14 | c1 | D01 | d1 | E01 |
| 2-1-d1E01-1-0234 | 672.262 | OH | a2 | B16 | b2 | C33 | c3 | D02 | d1 | E01 |
| 2-1-d1E01-1-0235 | 673.221 | OH | a1 | B03 | b3 | C15 | c1 | D01 | d1 | E01 |
| 2-1-d1E01-1-0236 | 673.251 | OH | a1 | B09 | b1 | C08 | c1 | D01 | d1 | E01 |
| 2-1-d1E01-1-0237 | 673.251 | OH | a1 | B01 | b1 | C09 | c1 | D01 | d1 | E01 |
| 2-1-d1E01-1-0238 | 674.167 | OH | a2 | B14 | b3 | C06 | c1 | D01 | d1 | E01 |
| 2-1-d1E01-1-0239 | 674.245 | OH | a2 | B17 | b1 | C13 | c1 | D01 | d1 | E01 |
| 2-1-d1E01-1-0240 | 674.246 | OH | a1 | B02 | b1 | C09 | c1 | D01 | d1 | E01 |
| 2-1-d1E01-1-0241 | 674.246 | OH | a1 | B09 | b1 | C11 | c1 | D01 | d1 | E01 |
| 2-1-d1E01-1-0242 | 674.347 | OH | a2 | B15 | b2 | C07 | c1 | D01 | d1 | E01 |
| 2-1-d1E01-1-0243 | 675.162 | OH | a2 | B14 | b1 | C12 | c2 | D02 | d1 | E01 |
| 2-1-d1E01-1-0244 | 675.182 | OH | a1 | B01 | b3 | C06 | c1 | D01 | d1 | E01 |
| 2-1-d1E01-1-0245 | 675.196 | OH | a1 | B07 | b2 | C06 | c1 | D01 | d1 | E01 |
| 2-1-d1E01-1-0246 | 675.242 | OH | a1 | B09 | b1 | C10 | c1 | D01 | d1 | E01 |
| 2-1-d1E01-1-0247 | 675.252 | OH | a1 | B03 | b1 | C14 | c1 | D01 | d1 | E01 |
| 2-1-d1E01-1-0248 | 675.261 | OH | a1 | B04 | b1 | C13 | c1 | D01 | d1 | E01 |
| 2-1-d1E01-1-0249 | 675.273 | OH | a1 | B03 | b2 | C33 | c3 | D02 | d1 | E01 |
| 2-1-d1E01-1-0250 | 676.177 | OH | a1 | B02 | b3 | C06 | c1 | D01 | d1 | E01 |
| 2-1-d1E01-1-0251 | 676.177 | OH | a1 | B01 | b1 | C12 | c2 | D02 | d1 | E01 |
| 2-1-d1E01-1-0252 | 676.180 | OH | a2 | B17 | b3 | C15 | c1 | D01 | d1 | E01 |
| 2-1-d1E01-1-0253 | 676.272 | OH | a2 | B13 | b1 | C30 | c3 | D02 | d1 | E01 |
| 2-1-d1E01-1-0254 | 676.272 | OH | a2 | B11 | b1 | C32 | c3 | D02 | d1 | E01 |
| 2-1-d1E01-1-0255 | 676.301 | OH | a1 | B05 | b2 | C05 | c1 | D01 | d1 | E01 |
| 2-1-d1E01-1-0256 | 676.326 | OH | a2 | B16 | b2 | C07 | c1 | D01 | d1 | E01 |
| 2-1-d1E01-1-0257 | 677.173 | OH | a1 | B02 | b1 | C12 | c2 | D02 | d1 | E01 |
| 2-1-d1E01-1-0258 | 677.192 | OH | a2 | B20 | b1 | C12 | c1 | D01 | d1 | E01 |
| 2-1-d1E01-1-0259 | 677.196 | OH | a1 | B04 | b3 | C15 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0260 | 677.267 | OH | a2 | B12 | b1 | C32 | c3 | D02 | d1 | E01 |
| 2-1-d1E01-1-0261 | 678.211 | OH | a2 | B17 | b1 | C14 | c1 | D01 | d1 | E01 |
| 2-1-d1E01-1-0262 | 678.215 | OH | a2 | B11 | b2 | C02 | c2 | D02 | d1 | E01 |
| 2-1-d1E01-1-0263 | 678.232 | OH | a2 | B17 | b2 | C33 | c3 | D02 | d1 | E01 |
| 2-1-d1E01-1-0264 | 678.251 | OH | a2 | B15 | b3 | C08 | c1 | D01 | d1 | E01 |
| 2-1-d1E01-1-0265 | 678.251 | OH | a2 | B15 | b1 | C08 | c2 | D02 | d1 | E01 |
| 2-1-d1E01-1-0266 | 678.251 | OH | a2 | B13 | b2 | C19 | c3 | D02 | d1 | E01 |
| 2-1-d1E01-1-0267 | 678.251 | OH | a2 | B11 | b2 | C21 | c3 | D02 | d1 | E01 |
| 2-1-d1E01-1-0268 | 678.28 | OH | a1 | B06 | b2 | C05 | c1 | D01 | d1 | E01 |
| 2-1-d1E01-1-0269 | 679.21 | OH | a2 | B12 | b2 | C02 | c2 | D02 | d1 | E01 |
| 2-1-d1E01-1-0270 | 679.21 | OH | a2 | B11 | b2 | C03 | c2 | D02 | d1 | E01 |
| 2-1-d1E01-1-0271 | 679.226 | OH | a1 | B04 | b1 | C14 | c1 | D01 | d1 | E01 |
| 2-1-d1E01-1-0272 | 679.246 | OH | a2 | B15 | b1 | C11 | c2 | D02 | d1 | E01 |
| 2-1-d1E01-1-0273 | 679.246 | OH | a2 | B12 | b2 | C21 | c3 | D02 | d1 | E01 |
| 2-1-d1E01-1-0274 | 679.246 | OH | a2 | B13 | b2 | C27 | c3 | D02 | d1 | E01 |
| 2-1-d1E01-1-0275 | 679.248 | OH | a1 | B04 | b2 | C33 | c3 | D02 | d1 | E01 |
| 2-1-d1E01-1-0276 | 679.279 | OH | a2 | B18 | b2 | C05 | c1 | D01 | d1 | E01 |
| 2-1-d1E01-1-0277 | 679.281 | OH | a1 | B05 | b2 | C04 | c1 | D01 | d1 | E01 |
| 2-1-d1E01-1-0278 | 679.337 | OH | a1 | B03 | b2 | C07 | c1 | D01 | d1 | E01 |
| 2-1-d1E01-1-0279 | 680.203 | OH | a1 | B09 | b1 | C12 | c1 | D01 | d1 | E01 |
| 2-1-d1E01-1-0280 | 680.205 | OH | a1 | B08 | b2 | C01 | c1 | D01 | d1 | E01 |
| 2-1-d1E01-1-0281 | 680.205 | OH | a2 | B12 | b2 | C03 | c2 | D02 | d1 | E01 |
| 2-1-d1E01-1-0282 | 680.230 | OH | a2 | B16 | b3 | C08 | c1 | D01 | d1 | E01 |
| 2-1-d1E01-1-0283 | 680.230 | OH | a2 | B16 | b1 | C08 | c2 | D02 | d1 | E01 |
| 2-1-d1E01-1-0284 | 680.242 | OH | a2 | B15 | b1 | C10 | c2 | D02 | d1 | E01 |
| 2-1-d1E01-1-0285 | 680.247 | OH | a2 | B14 | b1 | C30 | c3 | D02 | d1 | E01 |
| 2-1-d1E01-1-0286 | 681.226 | OH | a2 | B16 | b1 | C11 | c2 | D02 | d1 | E01 |
| 2-1-d1E01-1-0287 | 681.260 | OH | a1 | B06 | b2 | C04 | c1 | D01 | d1 | E01 |
| 2-1-d1E01-1-0288 | 681.262 | OH | a1 | B01 | b1 | C30 | c3 | D02 | d1 | E01 |
| 2-1-d1E01-1-0289 | 682.210 | OH | a2 | B13 | b2 | C01 | c2 | D02 | d1 | E01 |
| 2-1-d1E01-1-0290 | 682.221 | OH | a2 | B16 | b1 | C10 | c2 | D02 | d1 | E01 |
| 2-1-d1E01-1-0291 | 682.226 | OH | a2 | B14 | b2 | C19 | c3 | D02 | d1 | E01 |
| 2-1-d1E01-1-0292 | 682.226 | OH | a2 | B11 | b2 | C23 | c3 | D02 | d1 | E01 |
| 2-1-d1E01-1-0293 | 682.240 | OH | a2 | B19 | b2 | C02 | c1 | D01 | d1 | E01 |
| 2-1-d1E01-1-0294 | 682.257 | OH | a1 | B02 | b1 | C30 | c3 | D02 | d1 | E01 |
| 2-1-d1E01-1-0295 | 682.259 | OH | a2 | B18 | b2 | C04 | c1 | D01 | d1 | E01 |
| 2-1-d1E01-1-0296 | 682.277 | OH | a2 | B15 | b1 | C09 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0297 | 682.297 | OH | a2 | B17 | b2 | C07 | c1 | D01 | d1 | E01 |
| 2-1-d1E01-1-0298 | 683.221 | OH | a2 | B12 | b2 | C23 | c3 | D02 | d1 | E01 |
| 2-1-d1E01-1-0299 | 683.221 | OH | a2 | B14 | b2 | C27 | c3 | D02 | d1 | E01 |
| 2-1-d1E01-1-0300 | 683.236 | OH | a2 | B19 | b2 | C03 | c1 | D01 | d1 | E01 |
| 2-1-d1E01-1-0301 | 683.241 | OH | a1 | B03 | b3 | C08 | c1 | D01 | d1 | E01 |
| 2-1-d1E01-1-0302 | 683.241 | OH | a1 | B03 | b1 | C08 | c2 | D02 | d1 | E01 |
| 2-1-d1E01-1-0303 | 683.241 | OH | a1 | B01 | b2 | C19 | c3 | D02 | d1 | E01 |
| 2-1-d1E01-1-0304 | 683.312 | OH | a1 | B04 | b2 | C07 | c1 | D01 | d1 | E01 |
| 2-1-d1E01-1-0305 | 684.171 | OH | a2 | B11 | b2 | C06 | c2 | D02 | d1 | E01 |
| 2-1-d1E01-1-0306 | 684.208 | OH | a2 | B15 | b3 | C06 | c1 | D01 | d1 | E01 |
| 2-1-d1E01-1-0307 | 684.237 | OH | a1 | B03 | b1 | C11 | c2 | D02 | d1 | E01 |
| 2-1-d1E01-1-0308 | 684.237 | OH | a1 | B02 | b2 | C19 | c3 | D02 | d1 | E01 |
| 2-1-d1E01-1-0309 | 684.237 | OH | a1 | B01 | b2 | C27 | c3 | D02 | d1 | E01 |
| 2-1-d1E01-1-0310 | 684.251 | OH | a1 | B07 | b2 | COS | c1 | D01 | d1 | E01 |
| 2-1-d1E01-1-0311 | 684.256 | OH | a2 | B16 | b1 | C09 | c1 | D01 | d1 | E01 |
| 2-1-d1E01-1-0312 | 685.166 | OH | a2 | B12 | b2 | C06 | c2 | D02 | d1 | E01 |
| 2-1-d1E01-1-0313 | 685.203 | OH | a2 | B15 | b1 | C12 | c2 | D02 | d1 | E01 |
| 2-1-d1E01-1-0314 | 685.232 | OH | a1 | B03 | b1 | C10 | c2 | D02 | d1 | E01 |
| 2-1-d1E01-1-0315 | 685.232 | OH | a1 | B02 | b2 | C27 | c3 | D02 | d1 | E01 |
| 2-1-d1E01-1-0316 | 685.301 | OH | a1 | B05 | b1 | C13 | c1 | D01 | d1 | E01 |
| 2-1-d1E01-1-0317 | 686.185 | OH | a2 | B14 | b2 | C01 | c2 | D02 | d1 | E01 |
| 2-1-d1E01-1-0318 | 686.187 | OH | a2 | B16 | b3 | C06 | c1 | D01 | d1 | E01 |
| 2-1-d1E01-1-0319 | 686.201 | OH | a2 | B17 | b3 | C08 | c1 | D01 | d1 | E01 |
| 2-1-d1E01-1-0320 | 686.201 | OH | a2 | B17 | b1 | C08 | c2 | D02 | d1 | E01 |
| 2-1-d1E01-1-0321 | 687.182 | OH | a2 | B16 | b1 | C12 | c2 | D02 | d1 | E01 |
| 2-1-d1E01-1-0322 | 687.196 | OH | a2 | B17 | b1 | C11 | c2 | D02 | d1 | E01 |
| 2-1-d1E01-1-0323 | 687.200 | OH | a1 | B01 | b2 | C01 | c2 | D02 | d1 | E01 |
| 2-1-d1E01-1-0324 | 687.216 | OH | a1 | B04 | b3 | C08 | c1 | D01 | d1 | E01 |
| 2-1-d1E01-1-0325 | 687.216 | OH | a1 | B04 | b1 | C08 | c2 | D02 | d1 | E01 |
| 2-1-d1E01-1-0326 | 687.230 | OH | a1 | B07 | b2 | C04 | c1 | D01 | d1 | E01 |
| 2-1-d1E01-1-0327 | 687.236 | OH | a1 | B05 | b3 | C15 | c1 | D01 | d1 | E01 |
| 2-1-d1E01-1-0328 | 687.267 | OH | a1 | B03 | b1 | C09 | c1 | D01 | d1 | E01 |
| 2-1-d1E01-1-0329 | 687.281 | OH | a1 | B06 | b1 | C13 | c1 | D01 | d1 | E01 |
| 2-1-d1E01-1-0330 | 688.192 | OH | a2 | B17 | b1 | C10 | c2 | D02 | d1 | E01 |
| 2-1-d1E01-1-0331 | 688.195 | OH | a1 | B02 | b2 | C01 | c2 | D02 | d1 | E01 |
| 2-1-d1E01-1-0332 | 688.197 | OH | a2 | B19 | b2 | C06 | c1 | D01 | d1 | E01 |
| 2-1-d1E01-1-0333 | 688.212 | OH | a1 | B04 | b1 | C11 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0334 | 688.214 | OH | a2 | B20 | b2 | C01 | c1 | D01 | d1 | E01 |
| 2-1-d1E01-1-0335 | 688.280 | OH | a2 | B18 | b1 | C13 | c1 | D01 | d1 | E01 |
| 2-1-d1E01-1-0336 | 689.198 | OH | a1 | B03 | b3 | C06 | c1 | D01 | d1 | E01 |
| 2-1-d1E01-1-0337 | 689.207 | OH | a1 | B04 | b1 | C10 | c2 | D02 | d1 | E01 |
| 2-1-d1E01-1-0338 | 689.216 | OH | a1 | B06 | b3 | C15 | c1 | D01 | d1 | E01 |
| 2-1-d1E01-1-0339 | 689.246 | OH | a1 | B10 | b1 | C08 | c1 | D01 | d1 | E01 |
| 2-1-d1E01-1-0340 | 689.267 | OH | a1 | B05 | b1 | C14 | c1 | D01 | d1 | E01 |
| 2-1-d1E01-1-0341 | 689.288 | OH | a1 | B05 | b2 | C33 | c3 | D02 | d1 | E01 |
| 2-1-d1E01-1-0342 | 690.193 | OH | a1 | B03 | b1 | C12 | c2 | D02 | d1 | E01 |
| 2-1-d1E01-1-0343 | 690.215 | OH | a2 | B18 | b3 | C15 | c1 | D01 | d1 | E01 |
| 2-1-d1E01-1-0344 | 690.226 | OH | a1 | B08 | b2 | C02 | c1 | D01 | d1 | E01 |
| 2-1-d1E01-1-0345 | 690.227 | OH | a2 | B17 | b1 | C09 | c1 | D01 | d1 | E01 |
| 2-1-d1E01-1-0346 | 690.241 | OH | a1 | B10 | b1 | C11 | c1 | D01 | d1 | E01 |
| 2-1-d1E01-1-0347 | 690.288 | OH | a2 | B15 | b1 | C30 | c3 | D02 | d1 | E01 |
| 2-1-d1E01-1-0348 | 690.288 | OH | a2 | B11 | b1 | C29 | c3 | D02 | d1 | E01 |
| 2-1-d1E01-1-0349 | 690.288 | OH | a2 | B13 | b1 | C32 | c3 | D02 | d1 | E01 |
| 2-1-d1E01-1-0350 | 691.221 | OH | a1 | B08 | b2 | C03 | c1 | D01 | d1 | E01 |
| 2-1-d1E01-1-0351 | 691.225 | OH | a1 | B09 | b2 | C01 | c1 | D01 | d1 | E01 |
| 2-1-d1E01-1-0352 | 691.237 | OH | a1 | B10 | b1 | C10 | c1 | D01 | d1 | E01 |
| 2-1-d1E01-1-0353 | 691.242 | OH | a1 | B04 | b1 | C09 | c1 | D01 | d1 | E01 |
| 2-1-d1E01-1-0354 | 691.246 | OH | a1 | B06 | b1 | C14 | c1 | D01 | d1 | E01 |
| 2-1-d1E01-1-0355 | 691.268 | OH | a1 | B06 | b2 | C33 | c3 | D02 | d1 | E01 |
| 2-1-d1E01-1-0356 | 691.283 | OH | a2 | B12 | b1 | C29 | c3 | D02 | d1 | E01 |
| 2-1-d1E01-1-0357 | 692.157 | OH | a2 | B17 | b3 | C06 | c1 | D01 | d1 | E01 |
| 2-1-d1E01-1-0358 | 692.230 | OH | a2 | B13 | b2 | C02 | c2 | D02 | d1 | E01 |
| 2-1-d1E01-1-0359 | 692.246 | OH | a2 | B18 | b1 | C14 | c1 | D01 | d1 | E01 |
| 2-1-d1E01-1-0360 | 692.267 | OH | a2 | B15 | b2 | C19 | c3 | D02 | d1 | E01 |
| 2-1-d1E01-1-0361 | 692.267 | OH | a2 | B13 | b2 | C21 | c3 | D02 | d1 | E01 |
| 2-1-d1E01-1-0362 | 692.267 | OH | a2 | B16 | b1 | C30 | c3 | D02 | d1 | E01 |
| 2-1-d1E01-1-0363 | 692.267 | OH | a2 | B18 | b2 | C33 | c3 | D02 | d1 | E01 |
| 2-1-d1E01-1-0364 | 693.153 | OH | a2 | B17 | b1 | C12 | c2 | D02 | d1 | E01 |
| 2-1-d1E01-1-0365 | 693.173 | OH | a1 | B04 | b3 | C06 | c1 | D01 | d1 | E01 |
| 2-1-d1E01-1-0366 | 693.226 | OH | a2 | B13 | b2 | C03 | c2 | D02 | d1 | E01 |
| 2-1-d1E01-1-0367 | 693.226 | OH | a2 | B11 | b2 | C05 | c2 | D02 | d1 | E01 |
| 2-1-d1E01-1-0368 | 693.251 | OH | a1 | B07 | b1 | C13 | c1 | D01 | d1 | E01 |
| 2-1-d1E01-1-0369 | 693.262 | OH | a2 | B15 | b2 | C27 | c3 | D02 | d1 | E01 |
| 2-1-d1E01-1-0370 | 693.353 | OH | a1 | B05 | b2 | C07 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0371 | 694.168 | OH | a1 | B04 | b1 | C12 | c2 | D02 | d1 | E01 |
| 2-1-d1E01-1-0372 | 694.221 | OH | a2 | B12 | b2 | C05 | c2 | D02 | d1 | E01 |
| 2-1-d1E01-1-0373 | 694.246 | OH | a2 | B16 | b2 | C19 | c3 | D02 | d1 | E01 |
| 2-1-d1E01-1-0374 | 694.246 | OH | a2 | B11 | b2 | C25 | c3 | D02 | d1 | E01 |
| 2-1-d1E01-1-0375 | 694.263 | OH | a2 | B14 | b1 | C32 | c3 | D02 | d1 | E01 |
| 2-1-d1E01-1-0376 | 695.186 | OH | a1 | B07 | b3 | C15 | c1 | D01 | d1 | E01 |
| 2-1-d1E01-1-0377 | 695.241 | OH | a2 | B12 | b2 | C25 | c3 | D02 | d1 | E01 |
| 2-1-d1E01-1-0378 | 695.241 | OH | a2 | B16 | b2 | C27 | c3 | D02 | d1 | E01 |
| 2-1-d1E01-1-0379 | 695.278 | OH | a1 | B03 | b1 | C30 | c3 | D02 | d1 | E01 |
| 2-1-d1E01-1-0380 | 695.278 | OH | a1 | B01 | b1 | C32 | c3 | D02 | d1 | E01 |
| 2-1-d1E01-1-0381 | 695.332 | OH | a1 | B06 | b2 | C07 | c1 | D01 | d1 | E01 |
| 2-1-d1E01-1-0382 | 696.182 | OH | a1 | B08 | b2 | C06 | c1 | D01 | d1 | E01 |
| 2-1-d1E01-1-0383 | 696.198 | OH | a1 | B10 | b1 | C12 | c1 | D01 | d1 | E01 |
| 2-1-d1E01-1-0384 | 696.205 | OH | a2 | B14 | b2 | C02 | c2 | D02 | d1 | E01 |
| 2-1-d1E01-1-0385 | 696.205 | OH | a2 | B11 | b2 | C04 | c2 | D02 | d1 | E01 |
| 2-1-d1E01-1-0386 | 696.225 | OH | a2 | B15 | b2 | C01 | c2 | D02 | d1 | E01 |
| 2-1-d1E01-1-0387 | 696.242 | OH | a2 | B14 | b2 | C21 | c3 | D02 | d1 | E01 |
| 2-1-d1E01-1-0388 | 696.242 | OH | a2 | B13 | b2 | C23 | c3 | D02 | d1 | E01 |
| 2-1-d1E01-1-0389 | 696.273 | OH | a1 | B02 | b1 | C32 | c3 | D02 | d1 | E01 |
| 2-1-d1E01-1-0390 | 696.331 | OH | a2 | B18 | b2 | C07 | c1 | D01 | d1 | E01 |
| 2-1-d1E01-1-0391 | 697.201 | OH | a2 | B12 | b2 | C04 | c2 | D02 | d1 | E01 |
| 2-1-d1E01-1-0392 | 697.201 | OH | a2 | B14 | b2 | C03 | c2 | D02 | d1 | E01 |
| 2-1-d1E01-1-0393 | 697.217 | OH | a1 | B07 | b1 | C14 | c1 | D01 | d1 | E01 |
| 2-1-d1E01-1-0394 | 697.221 | OH | a1 | B01 | b2 | C02 | c2 | D02 | d1 | E01 |
| 2-1-d1E01-1-0395 | 697.238 | OH | a1 | B07 | b2 | C33 | c3 | D02 | d1 | E01 |
| 2-1-d1E01-1-0396 | 697.251 | OH | a2 | B19 | b2 | C05 | c1 | D01 | d1 | E01 |
| 2-1-d1E01-1-0397 | 697.257 | OH | a1 | B05 | b3 | C08 | c1 | D01 | d1 | E01 |
| 2-1-d1E01-1-0398 | 697.257 | OH | a1 | B05 | b1 | C08 | c2 | D02 | d1 | E01 |
| 2-1-d1E01-1-0399 | 697.257 | OH | a1 | B03 | b2 | C19 | c3 | D02 | d1 | E01 |
| 2-1-d1E01-1-0400 | 697.257 | OH | a1 | B01 | b2 | C21 | c3 | D02 | d1 | E01 |
| 2-1-d1E01-1-0401 | 698.187 | OH | a2 | B13 | b2 | C06 | c2 | D02 | d1 | E01 |
| 2-1-d1E01-1-0402 | 698.205 | OH | a2 | B16 | b2 | C01 | c2 | D02 | d1 | E01 |
| 2-1-d1E01-1-0403 | 698.216 | OH | a1 | B02 | b2 | C02 | c2 | D02 | d1 | E01 |
| 2-1-d1E01-1-0404 | 698.216 | OH | a1 | B01 | b2 | C03 | c2 | D02 | d1 | E01 |
| 2-1-d1E01-1-0405 | 698.235 | OH | a2 | B20 | b2 | C02 | c1 | D01 | d1 | E01 |
| 2-1-d1E01-1-0406 | 698.237 | OH | a2 | B17 | b1 | C30 | c3 | D02 | d1 | E01 |
| 2-1-d1E01-1-0407 | 698.252 | OH | a1 | B05 | b1 | C11 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0408 | 698.252 | OH | a1 | B02 | b2 | C21 | c3 | D02 | d1 | E01 |
| 2-1-d1E01-1-0409 | 698.252 | OH | a1 | B03 | b2 | C27 | c3 | D02 | d1 | E01 |
| 2-1-d1E01-1-0410 | 699.211 | OH | a1 | B02 | b2 | C03 | c2 | D02 | d1 | E01 |
| 2-1-d1E01-1-0411 | 699.230 | OH | a2 | B20 | b2 | C03 | c1 | D01 | d1 | E01 |
| 2-1-d1E01-1-0412 | 699.236 | OH | a1 | B06 | b3 | C08 | c1 | D01 | d1 | E01 |
| 2-1-d1E01-1-0413 | 699.236 | OH | a1 | B06 | b1 | C08 | c2 | D02 | d1 | E01 |
| 2-1-d1E01-1-0414 | 699.248 | OH | a1 | B05 | b1 | C10 | c2 | D02 | d1 | E01 |
| 2-1-d1E01-1-0415 | 699.253 | OH | a1 | B04 | b1 | C30 | c3 | D02 | d1 | E01 |
| 2-1-d1E01-1-0416 | 700.217 | OH | a2 | B17 | b2 | C19 | c3 | D02 | d1 | E01 |
| 2-1-d1E01-1-0417 | 700.217 | OH | a2 | B14 | b2 | C23 | c3 | D02 | d1 | E01 |
| 2-1-d1E01-1-0418 | 700.231 | OH | a2 | B19 | b2 | C04 | c1 | D01 | d1 | E01 |
| 2-1-d1E01-1-0419 | 700.232 | OH | a1 | B06 | b1 | C11 | c2 | D02 | d1 | E01 |
| 2-1-d1E01-1-0420 | 700.236 | OH | a2 | B18 | b3 | C08 | c1 | D01 | d1 | E01 |
| 2-1-d1E01-1-0421 | 700.236 | OH | a2 | B18 | b1 | C08 | c2 | D02 | d1 | E01 |
| 2-1-d1E01-1-0422 | 701.212 | OH | a2 | B17 | b2 | C27 | c3 | D02 | d1 | E01 |
| 2-1-d1E01-1-0423 | 701.216 | OH | a1 | B03 | b2 | C01 | c2 | D02 | d1 | E01 |
| 2-1-d1E01-1-0424 | 701.227 | OH | a1 | B06 | b1 | C10 | c2 | D02 | d1 | E01 |
| 2-1-d1E01-1-0425 | 701.231 | OH | a2 | B18 | b1 | C11 | c2 | D02 | d1 | E01 |
| 2-1-d1E01-1-0426 | 701.232 | OH | a1 | B04 | b2 | C19 | c3 | D02 | d1 | E01 |
| 2-1-d1E01-1-0427 | 701.232 | OH | a1 | B01 | b2 | C23 | c3 | D02 | d1 | E01 |
| 2-1-d1E01-1-0428 | 701.246 | OH | a1 | B09 | b2 | C02 | c1 | D01 | d1 | E01 |
| 2-1-d1E01-1-0429 | 701.283 | OH | a1 | B05 | b1 | C09 | c1 | D01 | d1 | E01 |
| 2-1-d1E01-1-0430 | 701.302 | OH | a1 | B07 | b2 | C07 | c1 | D01 | d1 | E01 |
| 2-1-d1E01-1-0431 | 702.162 | OH | a2 | B14 | b2 | C06 | c2 | D02 | d1 | E01 |
| 2-1-d1E01-1-0432 | 702.226 | OH | a2 | B18 | b1 | C10 | c2 | D02 | d1 | E01 |
| 2-1-d1E01-1-0433 | 702.226 | OH | a2 | B11 | b1 | C13 | c2 | D02 | d1 | E01 |
| 2-1-d1E01-1-0434 | 702.227 | OH | a1 | B02 | b2 | C23 | c3 | D02 | d1 | E01 |
| 2-1-d1E01-1-0435 | 702.227 | OH | a1 | B04 | b2 | C27 | c3 | D02 | d1 | E01 |
| 2-1-d1E01-1-0436 | 702.241 | OH | a1 | B09 | b2 | C03 | c1 | D01 | d1 | E01 |
| 2-1-d1E01-1-0437 | 703.177 | OH | a1 | B01 | b2 | C06 | c2 | D02 | d1 | E01 |
| 2-1-d1E01-1-0438 | 703.213 | OH | a1 | B05 | b3 | C06 | c1 | D01 | d1 | E01 |
| 2-1-d1E01-1-0439 | 703.221 | OH | a2 | B12 | b1 | C13 | c2 | D02 | d1 | E01 |
| 2-1-d1E01-1-0440 | 703.262 | OH | a1 | B06 | b1 | C09 | c1 | D01 | d1 | E01 |
| 2-1-d1E01-1-0441 | 704.161 | OH | a2 | B11 | b3 | C15 | c2 | D02 | d1 | E01 |
| 2-1-d1E01-1-0442 | 704.172 | OH | a1 | B02 | b2 | C06 | c2 | D02 | d1 | E01 |
| 2-1-d1E01-1-0443 | 704.175 | OH | a2 | B17 | b2 | C01 | c2 | D02 | d1 | E01 |
| 2-1-d1E01-1-0444 | 704.192 | OH | a2 | B20 | b2 | C06 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0445 | 704.209 | OH | a1 | B05 | b1 | C12 | c2 | D02 | d1 | E01 |
| 2-1-d1E01-1-0446 | 704.230 | OH | a2 | B11 | b3 | C16 | c1 | D01 | d1 | E01 |
| 2-1-d1E01-1-0447 | 704.261 | OH | a2 | B18 | b1 | C09 | c1 | D01 | d1 | E01 |
| 2-1-d1E01-1-0448 | 704.303 | OH | a2 | B13 | b1 | C29 | c3 | D02 | d1 | E01 |
| 2-1-d1E01-1-0449 | 704.303 | OH | a2 | B15 | b1 | C32 | c3 | D02 | d1 | E01 |
| 2-1-d1E01-1-0450 | 705.156 | OH | a2 | B12 | b3 | C15 | c2 | D02 | d1 | E01 |
| 2-1-d1E01-1-0451 | 705.190 | OH | a1 | B04 | b2 | C01 | c2 | D02 | d1 | E01 |
| 2-1-d1E01-1-0452 | 705.193 | OH | a1 | B06 | b3 | C06 | c1 | D01 | d1 | E01 |
| 2-1-d1E01-1-0453 | 705.207 | OH | a1 | B07 | b3 | C08 | c1 | D01 | d1 | E01 |
| 2-1-d1E01-1-0454 | 705.207 | OH | a1 | B07 | b1 | C08 | c2 | D02 | d1 | E01 |
| 2-1-d1E01-1-0455 | 705.226 | OH | a2 | B12 | b3 | C16 | c1 | D01 | d1 | E01 |
| 2-1-d1E01-1-0456 | 705.237 | OH | a1 | B08 | b2 | C05 | c1 | D01 | d1 | E01 |
| 2-1-d1E01-1-0457 | 706.188 | OH | a1 | B06 | b1 | C12 | c2 | D02 | d1 | E01 |
| 2-1-d1E01-1-0458 | 706.192 | OH | a2 | B18 | b3 | C06 | c1 | D01 | d1 | E01 |
| 2-1-d1E01-1-0459 | 706.192 | OH | a2 | B11 | b3 | C17 | c1 | D01 | d1 | E01 |
| 2-1-d1E01-1-0460 | 706.192 | OH | a2 | B11 | b1 | C14 | c2 | D02 | d1 | E01 |
| 2-1-d1E01-1-0461 | 706.202 | OH | a1 | B07 | b1 | C11 | c2 | D02 | d1 | E01 |
| 2-1-d1E01-1-0462 | 706.246 | OH | a2 | B15 | b2 | C02 | c2 | D02 | d1 | E01 |
| 2-1-d1E01-1-0463 | 706.252 | OH | a2 | B19 | b1 | C13 | c1 | D01 | d1 | E01 |
| 2-1-d1E01-1-0464 | 706.283 | OH | a2 | B15 | b2 | C21 | c3 | D02 | d1 | E01 |
| 2-1-d1E01-1-0465 | 706.283 | OH | a2 | B16 | b1 | C32 | c3 | D02 | d1 | E01 |
| 2-1-d1E01-1-0466 | 707.187 | OH | a2 | B12 | b3 | C17 | c1 | D01 | d1 | E01 |
| 2-1-d1E01-1-0467 | 707.187 | OH | a2 | B18 | b1 | C12 | c2 | D02 | d1 | E01 |
| 2-1-d1E01-1-0468 | 707.187 | OH | a2 | B12 | b1 | C14 | c2 | D02 | d1 | E01 |
| 2-1-d1E01-1-0469 | 707.197 | OH | a1 | B07 | b1 | C10 | c2 | D02 | d1 | E01 |
| 2-1-d1E01-1-0470 | 707.203 | OH | a1 | B09 | b2 | C06 | c1 | D01 | d1 | E01 |
| 2-1-d1E01-1-0471 | 707.220 | OH | a1 | B10 | b2 | C01 | c1 | D01 | d1 | E01 |
| 2-1-d1E01-1-0472 | 707.241 | OH | a2 | B15 | b2 | C03 | c2 | D02 | d1 | E01 |
| 2-1-d1E01-1-0473 | 707.241 | OH | a2 | B13 | b2 | C05 | c2 | D02 | d1 | E01 |
| 2-1-d1E01-1-0474 | 708.187 | OH | a2 | B19 | b3 | C15 | c1 | D01 | d1 | E01 |
| 2-1-d1E01-1-0475 | 708.217 | OH | a1 | B08 | b2 | C04 | c1 | D01 | d1 | E01 |
| 2-1-d1E01-1-0476 | 708.225 | OH | a2 | B16 | b2 | C02 | c2 | D02 | d1 | E01 |
| 2-1-d1E01-1-0477 | 708.262 | OH | a2 | B16 | b2 | C21 | c3 | D02 | d1 | E01 |
| 2-1-d1E01-1-0478 | 708.262 | OH | a2 | B13 | b2 | C25 | c3 | D02 | d1 | E01 |
| 2-1-d1E01-1-0479 | 708.278 | OH | a2 | B14 | b1 | C29 | c3 | D02 | d1 | E01 |
| 2-1-d1E01-1-0480 | 709.221 | OH | a2 | B16 | b2 | C03 | c2 | D02 | d1 | E01 |
| 2-1-d1E01-1-0481 | 709.232 | OH | a1 | B07 | b1 | C09 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0482 | 709.293 | OH | a1 | B05 | b1 | C30 | c3 | D02 | d1 | E01 |
| 2-1-d1E01-1-0483 | 709.293 | OH | a1 | B01 | b1 | C29 | c3 | D02 | d1 | E01 |
| 2-1-d1E01-1-0484 | 709.293 | OH | a1 | B03 | b1 | C32 | c3 | D02 | d1 | E01 |
| 2-1-d1E01-1-0485 | 710.217 | OH | a2 | B19 | b1 | C14 | c1 | D01 | d1 | E01 |
| 2-1-d1E01-1-0486 | 710.221 | OH | a2 | B13 | b2 | C04 | c2 | D02 | d1 | E01 |
| 2-1-d1E01-1-0487 | 710.239 | OH | a2 | B19 | b2 | C33 | c3 | D02 | d1 | E01 |
| 2-1-d1E01-1-0488 | 710.257 | OH | a2 | B15 | b2 | C23 | c3 | D02 | d1 | E01 |
| 2-1-d1E01-1-0489 | 710.277 | OH | a2 | B11 | b2 | C07 | c2 | D02 | d1 | E01 |
| 2-1-d1E01-1-0490 | 710.289 | OH | a1 | B02 | b1 | C29 | c3 | D02 | d1 | E01 |
| 2-1-d1E01-1-0491 | 711.163 | OH | a1 | B07 | b3 | C06 | c1 | D01 | d1 | E01 |
| 2-1-d1E01-1-0492 | 711.216 | OH | a2 | B14 | b2 | C05 | c2 | D02 | d1 | E01 |
| 2-1-d1E01-1-0493 | 711.236 | OH | a1 | B03 | b2 | C02 | c2 | D02 | d1 | E01 |
| 2-1-d1E01-1-0494 | 711.273 | OH | a2 | B12 | b2 | C07 | c2 | D02 | d1 | E01 |
| 2-1-d1E01-1-0495 | 711.273 | OH | a1 | B05 | b2 | C19 | c3 | D02 | d1 | E01 |
| 2-1-d1E01-1-0496 | 711.273 | OH | a1 | B03 | b2 | C21 | c3 | D02 | d1 | E01 |
| 2-1-d1E01-1-0497 | 711.273 | OH | a1 | B06 | b1 | C30 | c3 | D02 | d1 | E01 |
| 2-1-d1E01-1-0498 | 712.159 | OH | a1 | B07 | b1 | C12 | c2 | D02 | d1 | E01 |
| 2-1-d1E01-1-0499 | 712.203 | OH | a2 | B15 | b2 | C06 | c2 | D02 | d1 | E01 |
| 2-1-d1E01-1-0500 | 712.232 | OH | a1 | B03 | b2 | C03 | c2 | D02 | d1 | E01 |
| 2-1-d1E01-1-0501 | 712.232 | OH | a1 | B01 | b2 | C05 | c2 | D02 | d1 | E01 |
| 2-1-d1E01-1-0502 | 712.237 | OH | a2 | B16 | b2 | C23 | c3 | D02 | d1 | E01 |
| 2-1-d1E01-1-0503 | 712.237 | OH | a2 | B14 | b2 | C25 | c3 | D02 | d1 | E01 |
| 2-1-d1E01-1-0504 | 712.253 | OH | a2 | B17 | b1 | C32 | c3 | D02 | d1 | E01 |
| 2-1-d1E01-1-0505 | 712.268 | OH | a1 | B05 | b2 | C27 | c3 | D02 | d1 | E01 |
| 2-1-d1E01-1-0506 | 712.272 | OH | a2 | B18 | b1 | C30 | c3 | D02 | d1 | E01 |
| 2-1-d1E01-1-0507 | 713.227 | OH | a1 | B02 | b2 | C05 | c2 | D02 | d1 | E01 |
| 2-1-d1E01-1-0508 | 713.246 | OH | a2 | B20 | b2 | C05 | c1 | D01 | d1 | E01 |
| 2-1-d1E01-1-0509 | 713.252 | OH | a1 | B06 | b2 | C19 | c3 | D02 | d1 | E01 |
| 2-1-d1E01-1-0510 | 713.252 | OH | a1 | B01 | b2 | C25 | c3 | D02 | d1 | E01 |
| 2-1-d1E01-1-0511 | 713.268 | OH | a1 | B04 | b1 | C32 | c3 | D02 | d1 | E01 |
| 2-1-d1E01-1-0512 | 714.182 | OH | a2 | B16 | b2 | C06 | c2 | D02 | d1 | E01 |
| 2-1-d1E01-1-0513 | 714.182 | OH | a2 | B11 | b3 | C08 | c2 | D02 | d1 | E01 |
| 2-1-d1E01-1-0514 | 714.196 | OH | a2 | B17 | b2 | C02 | c2 | D02 | d1 | E01 |
| 2-1-d1E01-1-0515 | 714.196 | OH | a2 | B14 | b2 | C04 | c2 | D02 | d1 | E01 |
| 2-1-d1E01-1-0516 | 714.232 | OH | a2 | B17 | b2 | C21 | c3 | D02 | d1 | E01 |
| 2-1-d1E01-1-0517 | 714.237 | OH | a1 | B08 | b1 | C13 | c1 | D01 | d1 | E01 |
| 2-1-d1E01-1-0518 | 714.247 | OH | a1 | B02 | b2 | C25 | c3 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0519 | 714.247 | OH | a1 | B06 | b2 | C27 | c3 | D02 | d1 | E01 |
| 2-1-d1E01-1-0520 | 714.251 | OH | a2 | B18 | b2 | C19 | c3 | D02 | d1 | E01 |
| 2-1-d1E01-1-0521 | 714.303 | OH | a2 | B19 | b2 | C07 | c1 | D01 | d1 | E01 |
| 2-1-d1E01-1-0522 | 715.177 | OH | a2 | B12 | b3 | C08 | c2 | D02 | d1 | E01 |
| 2-1-d1E01-1-0523 | 715.191 | OH | a2 | B17 | b2 | C03 | c2 | D02 | d1 | E01 |
| 2-1-d1E01-1-0524 | 715.211 | OH | a1 | B04 | b2 | C02 | c2 | D02 | d1 | E01 |
| 2-1-d1E01-1-0525 | 715.211 | OH | a1 | B01 | b2 | C04 | c2 | D02 | d1 | E01 |
| 2-1-d1E01-1-0526 | 715.231 | OH | a1 | B05 | b2 | C01 | c2 | D02 | d1 | E01 |
| 2-1-d1E01-1-0527 | 715.246 | OH | a2 | B18 | b2 | C27 | c3 | D02 | d1 | E01 |
| 2-1-d1E01-1-0528 | 715.248 | OH | a1 | B04 | b2 | C21 | c3 | D02 | d1 | E01 |
| 2-1-d1E01-1-0529 | 715.248 | OH | a1 | B03 | b2 | C23 | c3 | D02 | d1 | E01 |
| 2-1-d1E01-1-0530 | 716.172 | OH | a1 | B08 | b3 | C15 | c1 | D01 | d1 | E01 |
| 2-1-d1E01-1-0531 | 716.206 | OH | a1 | B02 | b2 | C04 | c2 | D02 | d1 | E01 |
| 2-1-d1E01-1-0532 | 716.206 | OH | a1 | B04 | b2 | C03 | c2 | D02 | d1 | E01 |
| 2-1-d1E01-1-0533 | 716.226 | OH | a2 | B20 | b2 | C04 | c1 | D01 | d1 | E01 |
| 2-1-d1E01-1-0534 | 716.242 | OH | a2 | B13 | b1 | C13 | c2 | D02 | d1 | E01 |
| 2-1-d1E01-1-0535 | 716.257 | OH | a1 | B09 | b2 | C05 | c1 | D01 | d1 | E01 |
| 2-1-d1E01-1-0536 | 717.193 | OH | a1 | B03 | b2 | C06 | c2 | D02 | d1 | E01 |
| 2-1-d1E01-1-0537 | 717.210 | OH | a1 | B06 | b2 | C01 | c2 | D02 | d1 | E01 |
| 2-1-d1E01-1-0538 | 717.241 | OH | a1 | B10 | b2 | C02 | c1 | D01 | d1 | E01 |
| 2-1-d1E01-1-0539 | 717.243 | OH | a1 | B07 | b1 | C30 | c3 | D02 | d1 | E01 |
| 2-1-d1E01-1-0540 | 718.177 | OH | a2 | B13 | b3 | C15 | c2 | D02 | d1 | E01 |
| 2-1-d1E01-1-0541 | 718.203 | OH | a1 | B08 | b1 | C14 | c1 | D01 | d1 | E01 |
| 2-1-d1E01-1-0542 | 718.207 | OH | a2 | B19 | b3 | C08 | c1 | D01 | d1 | E01 |
| 2-1-d1E01-1-0543 | 718.207 | OH | a2 | B19 | b1 | C08 | c2 | D02 | d1 | E01 |
| 2-1-d1E01-1-0544 | 718.207 | OH | a2 | B11 | b1 | C09 | c2 | D02 | d1 | E01 |
| 2-1-d1E01-1-0545 | 718.207 | OH | a2 | B17 | b2 | C23 | c3 | D02 | d1 | E01 |
| 2-1-d1E01-1-0546 | 718.210 | OH | a2 | B18 | b2 | C01 | c2 | D02 | d1 | E01 |
| 2-1-d1E01-1-0547 | 718.224 | OH | a1 | B08 | b2 | C33 | c3 | D02 | d1 | E01 |
| 2-1-d1E01-1-0548 | 718.236 | OH | a1 | B10 | b2 | C03 | c1 | D01 | d1 | E01 |
| 2-1-d1E01-1-0549 | 718.246 | OH | a2 | B13 | b3 | C16 | c1 | D01 | d1 | E01 |
| 2-1-d1E01-1-0550 | 718.283 | OH | a2 | B11 | b1 | C31 | c3 | D02 | d1 | E01 |
| 2-1-d1E01-1-0551 | 718.319 | OH | a2 | B15 | b1 | C29 | c3 | D02 | d1 | E01 |
| 2-1-d1E01-1-0552 | 719.203 | OH | a2 | B12 | b1 | C09 | c2 | D02 | d1 | E01 |
| 2-1-d1E01-1-0553 | 719.203 | OH | a2 | B19 | b1 | C11 | c2 | D02 | d1 | E01 |
| 2-1-d1E01-1-0554 | 719.223 | OH | a1 | B07 | b2 | C19 | c3 | D02 | d1 | E01 |
| 2-1-d1E01-1-0555 | 719.223 | OH | a1 | B04 | b2 | C23 | c3 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0556 | 719.237 | OH | a1 | B09 | b2 | C04 | c1 | D01 | d1 | E01 |
| 2-1-d1E01-1-0557 | 719.278 | OH | a2 | B12 | b1 | C31 | c3 | D02 | d1 | E01 |
| 2-1-d1E01-1-0558 | 720.138 | OH | a2 | B11 | b3 | C06 | c2 | D02 | d1 | E01 |
| 2-1-d1E01-1-0559 | 720.152 | OH | a2 | B17 | b2 | C06 | c2 | D02 | d1 | E01 |
| 2-1-d1E01-1-0560 | 720.198 | OH | a2 | B19 | b1 | C10 | c2 | D02 | d1 | E01 |
| 2-1-d1E01-1-0561 | 720.208 | OH | a2 | B13 | b3 | C17 | c1 | D01 | d1 | E01 |
| 2-1-d1E01-1-0562 | 720.208 | OH | a2 | B13 | b1 | C14 | c2 | D02 | d1 | E01 |
| 2-1-d1E01-1-0563 | 720.217 | OH | a2 | B14 | b1 | C13 | c2 | D02 | d1 | E01 |
| 2-1-d1E01-1-0564 | 720.218 | OH | a1 | B07 | b2 | C27 | c3 | D02 | d1 | E01 |
| 2-1-d1E01-1-0565 | 720.298 | OH | a2 | B16 | b1 | C29 | c3 | D02 | d1 | E01 |
| 2-1-d1E01-1-0566 | 721.133 | OH | a2 | B12 | b3 | C06 | c2 | D02 | d1 | E01 |
| 2-1-d1E01-1-0567 | 721.168 | OH | a1 | B04 | b2 | C06 | c2 | D02 | d1 | E01 |
| 2-1-d1E01-1-0568 | 721.203 | OH | a2 | B11 | b3 | C18 | c1 | D01 | d1 | E01 |
| 2-1-d1E01-1-0569 | 721.232 | OH | a1 | B01 | b1 | C13 | c2 | D02 | d1 | E01 |
| 2-1-d1E01-1-0570 | 721.257 | OH | a2 | B15 | b2 | C05 | c2 | D02 | d1 | E01 |
| 2-1-d1E01-1-0571 | 722.152 | OH | a2 | B14 | b3 | C15 | c2 | D02 | d1 | E01 |
| 2-1-d1E01-1-0572 | 722.198 | OH | a2 | B12 | b3 | C18 | c1 | D01 | d1 | E01 |
| 2-1-d1E01-1-0573 | 722.221 | OH | a2 | B14 | b3 | C16 | c1 | D01 | d1 | E01 |
| 2-1-d1E01-1-0574 | 722.227 | OH | a1 | B02 | b1 | C13 | c2 | D02 | d1 | E01 |
| 2-1-d1E01-1-0575 | 722.233 | OH | a2 | B19 | b1 | C09 | c1 | D01 | d1 | E01 |
| 2-1-d1E01-1-0576 | 722.246 | OH | a2 | B20 | b1 | C13 | c1 | D01 | d1 | E01 |
| 2-1-d1E01-1-0577 | 722.277 | OH | a2 | B15 | b2 | C25 | c3 | D02 | d1 | E01 |
| 2-1-d1E01-1-0578 | 722.289 | OH | a1 | B08 | b2 | C07 | c1 | D01 | d1 | E01 |
| 2-1-d1E01-1-0579 | 723.167 | OH | a1 | B01 | b3 | C15 | c2 | D02 | d1 | E01 |
| 2-1-d1E01-1-0580 | 723.181 | OH | a1 | B07 | b2 | C01 | c2 | D02 | d1 | E01 |
| 2-1-d1E01-1-0581 | 723.197 | OH | a1 | B10 | b2 | C06 | c1 | D01 | d1 | E01 |
| 2-1-d1E01-1-0582 | 723.236 | OH | a1 | B01 | b3 | C16 | c1 | D01 | d1 | E01 |
| 2-1-d1E01-1-0583 | 723.236 | OH | a2 | B16 | b2 | C05 | c2 | D02 | d1 | E01 |
| 2-1-d1E01-1-0584 | 723.309 | OH | a1 | B03 | b1 | C29 | c3 | D02 | d1 | E01 |
| 2-1-d1E01-1-0585 | 723.309 | OH | a1 | B05 | b1 | C32 | c3 | D02 | d1 | E01 |
| 2-1-d1E01-1-0586 | 724.162 | OH | a1 | B02 | b3 | C15 | c2 | D02 | d1 | E01 |
| 2-1-d1E01-1-0587 | 724.164 | OH | a2 | B19 | b3 | C06 | c1 | D01 | d1 | E01 |
| 2-1-d1E01-1-0588 | 724.181 | OH | a2 | B20 | b3 | C15 | c1 | D01 | d1 | E01 |
| 2-1-d1E01-1-0589 | 724.183 | OH | a2 | B14 | b3 | C17 | c1 | D01 | d1 | E01 |
| 2-1-d1E01-1-0590 | 724.183 | OH | a2 | B14 | b1 | C14 | c2 | D02 | d1 | E01 |
| 2-1-d1E01-1-0591 | 724.232 | OH | a1 | B02 | b3 | C16 | c1 | D01 | d1 | E01 |
| 2-1-d1E01-1-0592 | 724.237 | OH | a2 | B15 | b2 | C04 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0593 | 724.257 | OH | a2 | B16 | b2 | C25 | c3 | D02 | d1 | E01 |
| 2-1-d1E01-1-0594 | 724.293 | OH | a2 | B13 | b2 | C07 | c2 | D02 | d1 | E01 |
| 2-1-d1E01-1-0595 | 725.159 | OH | a2 | B19 | b1 | C12 | c2 | D02 | d1 | E01 |
| 2-1-d1E01-1-0596 | 725.198 | OH | a1 | B01 | b3 | C17 | c1 | D01 | d1 | E01 |
| 2-1-d1E01-1-0597 | 725.198 | OH | a1 | B01 | b1 | C14 | c2 | D02 | d1 | E01 |
| 2-1-d1E01-1-0598 | 725.252 | OH | a1 | B05 | b2 | C02 | c2 | D02 | d1 | E01 |
| 2-1-d1E01-1-0599 | 725.257 | OH | a1 | B09 | b1 | C13 | c1 | D01 | d1 | E01 |
| 2-1-d1E01-1-0600 | 725.288 | OH | a1 | B05 | b2 | C21 | c3 | D02 | d1 | E01 |
| 2-1-d1E01-1-0601 | 725.288 | OH | a1 | B06 | b1 | C32 | c3 | D02 | d1 | E01 |
| 2-1-d1E01-1-0602 | 726.193 | OH | a1 | B02 | b3 | C17 | c1 | D01 | d1 | E01 |
| 2-1-d1E01-1-0603 | 726.193 | OH | a1 | B08 | b3 | C08 | c1 | D01 | d1 | E01 |
| 2-1-d1E01-1-0604 | 726.193 | OH | a1 | B08 | b1 | C08 | c2 | D02 | d1 | E01 |
| 2-1-d1E01-1-0605 | 726.193 | OH | a1 | B02 | b1 | C14 | c2 | D02 | d1 | E01 |
| 2-1-d1E01-1-0606 | 726.212 | OH | a2 | B20 | b1 | C14 | c1 | D01 | d1 | E01 |
| 2-1-d1E01-1-0607 | 726.216 | OH | a2 | B16 | b2 | C04 | c2 | D02 | d1 | E01 |
| 2-1-d1E01-1-0608 | 726.234 | OH | a2 | B20 | b2 | C33 | c3 | D02 | d1 | E01 |
| 2-1-d1E01-1-0609 | 726.247 | OH | a1 | B05 | b2 | C03 | c2 | D02 | d1 | E01 |
| 2-1-d1E01-1-0610 | 726.247 | OH | a1 | B03 | b2 | C05 | c2 | D02 | d1 | E01 |
| 2-1-d1E01-1-0611 | 726.269 | OH | a2 | B17 | b1 | C29 | c3 | D02 | d1 | E01 |
| 2-1-d1E01-1-0612 | 726.288 | OH | a2 | B18 | b1 | C32 | c3 | D02 | d1 | E01 |
| 2-1-d1E01-1-0613 | 727.188 | OH | a1 | B08 | b1 | C11 | c2 | D02 | d1 | E01 |
| 2-1-d1E01-1-0614 | 727.192 | OH | a1 | B09 | b3 | C15 | c1 | D01 | d1 | E01 |
| 2-1-d1E01-1-0615 | 727.231 | OH | a1 | B06 | b2 | C02 | c2 | D02 | d1 | E01 |
| 2-1-d1E01-1-0616 | 727.268 | OH | a1 | B06 | b2 | C21 | c3 | D02 | d1 | E01 |
| 2-1-d1E01-1-0617 | 727.268 | OH | a1 | B03 | b2 | C25 | c3 | D02 | d1 | E01 |
| 2-1-d1E01-1-0618 | 727.284 | OH | a1 | B04 | b1 | C29 | c3 | D02 | d1 | E01 |
| 2-1-d1E01-1-0619 | 728.184 | OH | a1 | B08 | b1 | C10 | c2 | D02 | d1 | E01 |
| 2-1-d1E01-1-0620 | 728.197 | OH | a2 | B13 | b3 | C08 | c2 | D02 | d1 | E01 |
| 2-1-d1E01-1-0621 | 728.226 | OH | a1 | B06 | b2 | C03 | c2 | D02 | d1 | E01 |
| 2-1-d1E01-1-0622 | 728.230 | OH | a2 | B18 | b2 | C02 | c2 | D02 | d1 | E01 |
| 2-1-d1E01-1-0623 | 728.267 | OH | a2 | B18 | b2 | C21 | c3 | D02 | d1 | E01 |
| 2-1-d1E01-1-0624 | 728.268 | OH | a2 | B14 | b2 | C07 | c2 | D02 | d1 | E01 |
| 2-1-d1E01-1-0625 | 729.207 | OH | a2 | B17 | b2 | C05 | c2 | D02 | d1 | E01 |
| 2-1-d1E01-1-0626 | 729.223 | OH | a1 | B09 | b1 | C14 | c1 | D01 | d1 | E01 |
| 2-1-d1E01-1-0627 | 729.226 | OH | a2 | B18 | b2 | C03 | c2 | D02 | d1 | E01 |
| 2-1-d1E01-1-0628 | 729.227 | OH | a1 | B03 | b2 | C04 | c2 | D02 | d1 | E01 |
| 2-1-d1E01-1-0629 | 729.244 | OH | a1 | B09 | b2 | C33 | c3 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0630 | 729.263 | OH | a1 | B05 | b2 | C23 | c3 | D02 | d1 | E01 |
| 2-1-d1E01-1-0631 | 729.283 | OH | a1 | B01 | b2 | C07 | c2 | D02 | d1 | E01 |
| 2-1-d1E01-1-0632 | 730.219 | OH | a1 | B08 | b1 | C09 | c1 | D01 | d1 | E01 |
| 2-1-d1E01-1-0633 | 730.222 | OH | a1 | B04 | b2 | C05 | c2 | D02 | d1 | E01 |
| 2-1-d1E01-1-0634 | 730.227 | OH | a2 | B17 | b2 | C25 | c3 | D02 | d1 | E01 |
| 2-1-d1E01-1-0635 | 730.244 | OH | a2 | B19 | b1 | C30 | c3 | D02 | d1 | E01 |
| 2-1-d1E01-1-0636 | 730.257 | OH | a2 | B15 | b1 | C13 | c2 | D02 | d1 | E01 |
| 2-1-d1E01-1-0637 | 730.278 | OH | a1 | B02 | b2 | C07 | c2 | D02 | d1 | E01 |
| 2-1-d1E01-1-0638 | 730.298 | OH | a2 | B20 | b2 | C07 | c1 | D01 | d1 | E01 |
| 2-1-d1E01-1-0639 | 731.208 | OH | a1 | B05 | b2 | C06 | c2 | D02 | d1 | E01 |
| 2-1-d1E01-1-0640 | 731.243 | OH | a1 | B06 | b2 | C23 | c3 | D02 | d1 | E01 |
| 2-1-d1E01-1-0641 | 731.243 | OH | a1 | B04 | b2 | C25 | c3 | D02 | d1 | E01 |
| 2-1-d1E01-1-0642 | 731.259 | OH | a1 | B07 | b1 | C32 | c3 | D02 | d1 | E01 |
| 2-1-d1E01-1-0643 | 732.149 | OH | a1 | B08 | b3 | C06 | c1 | D01 | d1 | E01 |
| 2-1-d1E01-1-0644 | 732.172 | OH | a2 | B14 | b3 | C08 | c2 | D02 | d1 | E01 |
| 2-1-d1E01-1-0645 | 732.187 | OH | a2 | B17 | b2 | C04 | c2 | D02 | d1 | E01 |
| 2-1-d1E01-1-0646 | 732.192 | OH | a2 | B15 | b3 | C15 | c2 | D02 | d1 | E01 |
| 2-1-d1E01-1-0647 | 732.223 | OH | a2 | B13 | b1 | C09 | c2 | D02 | d1 | E01 |
| 2-1-d1E01-1-0648 | 732.223 | OH | a2 | B19 | b2 | C19 | c3 | D02 | d1 | E01 |
| 2-1-d1E01-1-0649 | 732.237 | OH | a2 | B16 | b1 | C13 | c2 | D02 | d1 | E01 |
| 2-1-d1E01-1-0650 | 732.242 | OH | a2 | B18 | b2 | C23 | c3 | D02 | d1 | E01 |
| 2-1-d1E01-1-0651 | 732.252 | OH | a1 | B10 | b2 | C05 | c1 | D01 | d1 | E01 |
| 2-1-d1E01-1-0652 | 732.262 | OH | a2 | B15 | b3 | C16 | c1 | D01 | d1 | E01 |
| 2-1-d1E01-1-0653 | 732.298 | OH | a2 | B13 | b1 | C31 | c3 | D02 | d1 | E01 |
| 2-1-d1E01-1-0654 | 733.145 | OH | a1 | B08 | b1 | C12 | c2 | D02 | d1 | E01 |
| 2-1-d1E01-1-0655 | 733.188 | OH | a1 | B06 | b2 | C06 | c2 | D02 | d1 | E01 |
| 2-1-d1E01-1-0656 | 733.188 | OH | a1 | B01 | b3 | C08 | c2 | D02 | d1 | E01 |
| 2-1-d1E01-1-0657 | 733.202 | OH | a1 | B07 | b2 | C02 | c2 | D02 | d1 | E01 |
| 2-1-d1E01-1-0658 | 733.202 | OH | a1 | B04 | b2 | C04 | c2 | D02 | d1 | E01 |
| 2-1-d1E01-1-0659 | 733.218 | OH | a2 | B19 | b2 | C27 | c3 | D02 | d1 | E01 |
| 2-1-d1E01-1-0660 | 733.238 | OH | a1 | B07 | b2 | C21 | c3 | D02 | d1 | E01 |
| 2-1-d1E01-1-0661 | 733.309 | OH | a1 | B09 | b2 | C07 | c1 | D01 | d1 | E01 |
| 2-1-d1E01-1-0662 | 734.154 | OH | a2 | B13 | b3 | C06 | c2 | D02 | d1 | E01 |
| 2-1-d1E01-1-0663 | 734.172 | OH | a2 | B16 | b3 | C15 | c2 | D02 | d1 | E01 |
| 2-1-d1E01-1-0664 | 734.183 | OH | a1 | B02 | b3 | C08 | c2 | D02 | d1 | E01 |
| 2-1-d1E01-1-0665 | 734.187 | OH | a2 | B18 | b2 | C06 | c2 | D02 | d1 | E01 |
| 2-1-d1E01-1-0666 | 734.197 | OH | a1 | B07 | b2 | C03 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0667 | 734.202 | OH | a2 | B20 | b3 | C08 | c1 | D01 | d1 | E01 |
| 2-1-d1E01-1-0668 | 734.202 | OH | a2 | B20 | b1 | C08 | c2 | D02 | d1 | E01 |
| 2-1-d1E01-1-0669 | 734.223 | OH | a2 | B15 | b3 | C17 | c1 | D01 | d1 | E01 |
| 2-1-d1E01-1-0670 | 734.223 | OH | a2 | B15 | b1 | C14 | c2 | D02 | d1 | E01 |
| 2-1-d1E01-1-0671 | 734.241 | OH | a2 | B16 | b3 | C16 | c1 | D01 | d1 | E01 |
| 2-1-d1E01-1-0672 | 735.197 | OH | a2 | B20 | b1 | C11 | c2 | D02 | d1 | E01 |
| 2-1-d1E01-1-0673 | 735.219 | OH | a2 | B13 | b3 | C18 | c1 | D01 | d1 | E01 |
| 2-1-d1E01-1-0674 | 735.232 | OH | a1 | B10 | b2 | C04 | c1 | D01 | d1 | E01 |
| 2-1-d1E01-1-0675 | 735.248 | OH | a1 | B03 | b1 | C13 | c2 | D02 | d1 | E01 |
| 2-1-d1E01-1-0676 | 736.181 | OH | a2 | B19 | b2 | C01 | c2 | D02 | d1 | E01 |
| 2-1-d1E01-1-0677 | 736.193 | OH | a2 | B20 | b1 | C10 | c2 | D02 | d1 | E01 |
| 2-1-d1E01-1-0678 | 736.198 | OH | a2 | B14 | b1 | C09 | c2 | D02 | d1 | E01 |
| 2-1-d1E01-1-0679 | 736.203 | OH | a2 | B16 | b3 | C17 | c1 | D01 | d1 | E01 |
| 2-1-d1E01-1-0680 | 736.203 | OH | a2 | B16 | b1 | C14 | c2 | D02 | d1 | E01 |
| 2-1-d1E01-1-0681 | 736.273 | OH | a2 | B14 | b1 | C31 | c3 | D02 | d1 | E01 |
| 2-1-d1E01-1-0682 | 737.183 | OH | a1 | B03 | b3 | C15 | c2 | D02 | d1 | E01 |
| 2-1-d1E01-1-0683 | 737.213 | OH | a1 | B09 | b3 | C08 | c1 | D01 | d1 | E01 |
| 2-1-d1E01-1-0684 | 737.213 | OH | a1 | B09 | b1 | C08 | c2 | D02 | d1 | E01 |
| 2-1-d1E01-1-0685 | 737.213 | OH | a1 | B01 | b1 | C09 | c2 | D02 | d1 | E01 |
| 2-1-d1E01-1-0686 | 737.213 | OH | a1 | B07 | b2 | C23 | c3 | D02 | d1 | E01 |
| 2-1-d1E01-1-0687 | 737.252 | OH | a1 | B03 | b3 | C16 | c1 | D01 | d1 | E01 |
| 2-1-d1E01-1-0688 | 737.288 | OH | a1 | B01 | b1 | C31 | c3 | D02 | d1 | E01 |
| 2-1-d1E01-1-0689 | 737.325 | OH | a1 | B05 | b1 | C29 | c3 | D02 | d1 | E01 |
| 2-1-d1E01-1-0690 | 738.129 | OH | a2 | B14 | b3 | C06 | c2 | D02 | d1 | E01 |
| 2-1-d1E01-1-0691 | 738.207 | OH | a2 | B17 | b1 | C13 | c2 | D02 | d1 | E01 |
| 2-1-d1E01-1-0692 | 738.208 | OH | a1 | B02 | b1 | C09 | c2 | D02 | d1 | E01 |
| 2-1-d1E01-1-0693 | 738.208 | OH | a1 | B09 | b1 | C11 | c2 | D02 | d1 | E01 |
| 2-1-d1E01-1-0694 | 738.228 | OH | a2 | B20 | b1 | C09 | c1 | D01 | d1 | E01 |
| 2-1-d1E01-1-0695 | 738.229 | OH | a1 | B08 | b1 | C30 | c3 | D02 | d1 | E01 |
| 2-1-d1E01-1-0696 | 738.284 | OH | a1 | B02 | b1 | C31 | c3 | D02 | d1 | E01 |
| 2-1-d1E01-1-0697 | 738.309 | OH | a2 | B15 | b2 | C07 | c2 | D02 | d1 | E01 |
| 2-1-d1E01-1-0698 | 739.144 | OH | a1 | B01 | b3 | C06 | c2 | D02 | d1 | E01 |
| 2-1-d1E01-1-0699 | 739.158 | OH | a1 | B07 | b2 | C06 | c2 | D02 | d1 | E01 |
| 2-1-d1E01-1-0700 | 739.193 | OH | a2 | B14 | b3 | C18 | c1 | D01 | d1 | E01 |
| 2-1-d1E01-1-0701 | 739.204 | OH | a1 | B09 | b1 | C10 | c2 | D02 | d1 | E01 |
| 2-1-d1E01-1-0702 | 739.213 | OH | a1 | B03 | b3 | C17 | c1 | D01 | d1 | E01 |
| 2-1-d1E01-1-0703 | 739.213 | OH | a1 | B03 | b1 | C14 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0704 | 739.222 | OH | a1 | B04 | b1 | C13 | c2 | D02 | d1 | E01 |
| 2-1-d1E01-1-0705 | 739.304 | OH | a1 | B06 | b1 | C29 | c3 | D02 | d1 | E01 |
| 2-1-d1E01-1-0706 | 740.139 | OH | a1 | B02 | b3 | C06 | c2 | D02 | d1 | E01 |
| 2-1-d1E01-1-0707 | 740.142 | OH | a2 | B17 | b3 | C15 | c2 | D02 | d1 | E01 |
| 2-1-d1E01-1-0708 | 740.159 | OH | a2 | B20 | b3 | C06 | c1 | D01 | d1 | E01 |
| 2-1-d1E01-1-0709 | 740.209 | OH | a1 | B01 | b3 | C18 | c1 | D01 | d1 | E01 |
| 2-1-d1E01-1-0710 | 740.209 | OH | a1 | B08 | b2 | C19 | c3 | D02 | d1 | E01 |
| 2-1-d1E01-1-0711 | 740.212 | OH | a2 | B17 | b3 | C16 | c1 | D01 | d1 | E01 |
| 2-1-d1E01-1-0712 | 740.263 | OH | a1 | B05 | b2 | C05 | c2 | D02 | d1 | E01 |
| 2-1-d1E01-1-0713 | 740.288 | OH | a2 | B16 | b2 | C07 | c2 | D02 | d1 | E01 |
| 2-1-d1E01-1-0714 | 740.303 | OH | a2 | B18 | b1 | C29 | c3 | D02 | d1 | E01 |
| 2-1-d1E01-1-0715 | 741.154 | OH | a2 | B20 | b1 | C12 | c2 | D02 | d1 | E01 |
| 2-1-d1E01-1-0716 | 741.157 | OH | a1 | B04 | b3 | C15 | c2 | D02 | d1 | E01 |
| 2-1-d1E01-1-0717 | 741.204 | OH | a1 | B02 | b3 | C18 | c1 | D01 | d1 | E01 |
| 2-1-d1E01-1-0718 | 741.204 | OH | a1 | B08 | b2 | C27 | c3 | D02 | d1 | E01 |
| 2-1-d1E01-1-0719 | 741.227 | OH | a1 | B04 | b3 | C16 | c1 | D01 | d1 | E01 |
| 2-1-d1E01-1-0720 | 741.239 | OH | a1 | B09 | b1 | C09 | c1 | D01 | d1 | E01 |
| 2-1-d1E01-1-0721 | 741.252 | OH | a1 | B10 | b1 | C13 | c1 | D01 | d1 | E01 |
| 2-1-d1E01-1-0722 | 741.283 | OH | a1 | B05 | b2 | C25 | c3 | D02 | d1 | E01 |
| 2-1-d1E01-1-0723 | 742.173 | OH | a2 | B17 | b3 | C17 | c1 | D01 | d1 | E01 |
| 2-1-d1E01-1-0724 | 742.173 | OH | a2 | B17 | b1 | C14 | c2 | D02 | d1 | E01 |
| 2-1-d1E01-1-0725 | 742.213 | OH | a2 | B15 | b3 | C08 | c2 | D02 | d1 | E01 |
| 2-1-d1E01-1-0726 | 742.242 | OH | a1 | B06 | b2 | C05 | c2 | D02 | d1 | E01 |
| 2-1-d1E01-1-0727 | 743.170 | OH | a1 | B09 | b3 | C06 | c1 | D01 | d1 | E01 |
| 2-1-d1E01-1-0728 | 743.187 | OH | a1 | B10 | b3 | C15 | c1 | D01 | d1 | E01 |
| 2-1-d1E01-1-0729 | 743.188 | OH | a1 | B04 | b3 | C17 | c1 | D01 | d1 | E01 |
| 2-1-d1E01-1-0730 | 743.188 | OH | a1 | B04 | b1 | C14 | c2 | D02 | d1 | E01 |
| 2-1-d1E01-1-0731 | 743.241 | OH | a2 | B18 | b2 | C05 | c2 | D02 | d1 | E01 |
| 2-1-d1E01-1-0732 | 743.243 | OH | a1 | B05 | b2 | C04 | c2 | D02 | d1 | E01 |
| 2-1-d1E01-1-0733 | 743.262 | OH | a1 | B06 | b2 | C25 | c3 | D02 | d1 | E01 |
| 2-1-d1E01-1-0734 | 743.299 | OH | a1 | B03 | b2 | C07 | c2 | D02 | d1 | E01 |
| 2-1-d1E01-1-0735 | 744.165 | OH | a1 | B09 | b1 | C12 | c2 | D02 | d1 | E01 |
| 2-1-d1E01-1-0736 | 744.167 | OH | a1 | B08 | b2 | C01 | c2 | D02 | d1 | E01 |
| 2-1-d1E01-1-0737 | 744.192 | OH | a2 | B16 | b3 | C08 | c2 | D02 | d1 | E01 |
| 2-1-d1E01-1-0738 | 744.259 | OH | a2 | B19 | b1 | C32 | c3 | D02 | d1 | E01 |
| 2-1-d1E01-1-0739 | 744.262 | OH | a2 | B18 | b2 | C25 | c3 | D02 | d1 | E01 |
| 2-1-d1E01-1-0740 | 745.218 | OH | a1 | B10 | b1 | C14 | c1 | D01 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0741 | 745.222 | OH | a1 | B06 | b2 | C04 | c2 | D02 | d1 | E01 |
| 2-1-d1E01-1-0742 | 745.239 | OH | a1 | B10 | b2 | C33 | c3 | D02 | d1 | E01 |
| 2-1-d1E01-1-0743 | 745.275 | OH | a1 | B07 | b1 | C29 | c3 | D02 | d1 | E01 |
| 2-1-d1E01-1-0744 | 746.202 | OH | a2 | B19 | b2 | C02 | c2 | D02 | d1 | E01 |
| 2-1-d1E01-1-0745 | 746.221 | OH | a2 | B18 | b2 | C04 | c2 | D02 | d1 | E01 |
| 2-1-d1E01-1-0746 | 746.239 | OH | a2 | B15 | b1 | C09 | c2 | D02 | d1 | E01 |
| 2-1-d1E01-1-0747 | 746.239 | OH | a2 | B19 | b2 | C21 | c3 | D02 | d1 | E01 |
| 2-1-d1E01-1-0748 | 746.239 | OH | a2 | B20 | b1 | C30 | c3 | D02 | d1 | E01 |
| 2-1-d1E01-1-0749 | 746.259 | OH | a2 | B17 | b2 | C07 | c2 | D02 | d1 | E01 |
| 2-1-d1E01-1-0750 | 746.314 | OH | a2 | B15 | b1 | C31 | c3 | D02 | d1 | E01 |
| 2-1-d1E01-1-0751 | 747.197 | OH | a2 | B19 | b2 | C03 | c2 | D02 | d1 | E01 |
| 2-1-d1E01-1-0752 | 747.203 | OH | a1 | B03 | b3 | C08 | c2 | D02 | d1 | E01 |
| 2-1-d1E01-1-0753 | 747.274 | OH | a1 | B04 | b2 | C07 | c2 | D02 | d1 | E01 |
| 2-1-d1E01-1-0754 | 748.170 | OH | a2 | B15 | b3 | C06 | c2 | D02 | d1 | E01 |
| 2-1-d1E01-1-0755 | 748.213 | OH | a1 | B07 | b2 | C05 | c2 | D02 | d1 | E01 |
| 2-1-d1E01-1-0756 | 748.218 | OH | a2 | B16 | b1 | C09 | c2 | D02 | d1 | E01 |
| 2-1-d1E01-1-0757 | 748.218 | OH | a2 | B20 | b2 | C19 | c3 | D02 | d1 | E01 |
| 2-1-d1E01-1-0758 | 748.293 | OH | a2 | B16 | b1 | C31 | c3 | D02 | d1 | E01 |
| 2-1-d1E01-1-0759 | 749.213 | OH | a2 | B20 | b2 | C27 | c3 | D02 | d1 | E01 |
| 2-1-d1E01-1-0760 | 749.233 | OH | a1 | B07 | b2 | C25 | c3 | D02 | d1 | E01 |
| 2-1-d1E01-1-0761 | 749.234 | OH | a2 | B15 | b3 | C18 | c1 | D01 | d1 | E01 |
| 2-1-d1E01-1-0762 | 749.249 | OH | a1 | B09 | b1 | C30 | c3 | D02 | d1 | E01 |
| 2-1-d1E01-1-0763 | 749.263 | OH | a1 | B05 | b1 | C13 | c2 | D02 | d1 | E01 |
| 2-1-d1E01-1-0764 | 749.304 | OH | a1 | B10 | b2 | C07 | c1 | D01 | d1 | E01 |
| 2-1-d1E01-1-0765 | 750.149 | OH | a2 | B16 | b3 | C06 | c2 | D02 | d1 | E01 |
| 2-1-d1E01-1-0766 | 750.163 | OH | a2 | B17 | b3 | C08 | c2 | D02 | d1 | E01 |
| 2-1-d1E01-1-0767 | 750.214 | OH | a2 | B19 | b2 | C23 | c3 | D02 | d1 | E01 |
| 2-1-d1E01-1-0768 | 751.178 | OH | a1 | B04 | b3 | C08 | c2 | D02 | d1 | E01 |
| 2-1-d1E01-1-0769 | 751.192 | OH | a1 | B07 | b2 | C04 | c2 | D02 | d1 | E01 |
| 2-1-d1E01-1-0770 | 751.198 | OH | a1 | B05 | b3 | C15 | c2 | D02 | d1 | E01 |
| 2-1-d1E01-1-0771 | 751.213 | OH | a2 | B16 | b3 | C18 | c1 | D01 | d1 | E01 |
| 2-1-d1E01-1-0772 | 751.229 | OH | a1 | B03 | b1 | C09 | c2 | D02 | d1 | E01 |
| 2-1-d1E01-1-0773 | 751.229 | OH | a1 | B09 | b2 | C19 | c3 | D02 | d1 | E01 |
| 2-1-d1E01-1-0774 | 751.242 | OH | a1 | B06 | b1 | C13 | c2 | D02 | d1 | E01 |
| 2-1-d1E01-1-0775 | 751.268 | OH | a1 | B05 | b3 | C16 | c1 | D01 | d1 | E01 |
| 2-1-d1E01-1-0776 | 751.304 | OH | a1 | B03 | b1 | C31 | c3 | D02 | d1 | E01 |
| 2-1-d1E01-1-0777 | 752.159 | OH | a2 | B19 | b2 | C06 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0778 | 752.176 | OH | a2 | B20 | b2 | C01 | c2 | D02 | d1 | E01 |
| 2-1-d1E01-1-0779 | 752.224 | OH | a1 | B09 | b2 | C27 | c3 | D02 | d1 | E01 |
| 2-1-d1E01-1-0780 | 752.242 | OH | a2 | B18 | b1 | C13 | c2 | D02 | d1 | E01 |
| 2-1-d1E01-1-0781 | 752.245 | OH | a1 | B08 | b1 | C32 | c3 | D02 | d1 | E01 |
| 2-1-d1E01-1-0782 | 753.160 | OH | a1 | B03 | b3 | C06 | c2 | D02 | d1 | E01 |
| 2-1-d1E01-1-0783 | 753.177 | OH | a1 | B06 | b3 | C15 | c2 | D02 | d1 | E01 |
| 2-1-d1E01-1-0784 | 753.208 | OH | a1 | B10 | b3 | C08 | c1 | D01 | d1 | E01 |
| 2-1-d1E01-1-0785 | 753.208 | OH | a1 | B10 | b1 | C08 | c2 | D02 | d1 | E01 |
| 2-1-d1E01-1-0786 | 753.229 | OH | a1 | B05 | b3 | C17 | c1 | D01 | d1 | E01 |
| 2-1-d1E01-1-0787 | 753.229 | OH | a1 | B05 | b1 | C14 | c2 | D02 | d1 | E01 |
| 2-1-d1E01-1-0788 | 753.247 | OH | a1 | B06 | b3 | C16 | c1 | D01 | d1 | E01 |
| 2-1-d1E01-1-0789 | 754.177 | OH | a2 | B18 | b3 | C15 | c2 | D02 | d1 | E01 |
| 2-1-d1E01-1-0790 | 754.188 | OH | a1 | B08 | b2 | C02 | c2 | D02 | d1 | E01 |
| 2-1-d1E01-1-0791 | 754.188 | OH | a2 | B17 | b1 | C09 | c2 | D02 | d1 | E01 |
| 2-1-d1E01-1-0792 | 754.203 | OH | a1 | B10 | b1 | C11 | c2 | D02 | d1 | E01 |
| 2-1-d1E01-1-0793 | 754.224 | OH | a1 | B03 | b3 | C18 | c1 | D01 | d1 | E01 |
| 2-1-d1E01-1-0794 | 754.224 | OH | a1 | B08 | b2 | C21 | c3 | D02 | d1 | E01 |
| 2-1-d1E01-1-0795 | 754.246 | OH | a2 | B18 | b3 | C16 | c1 | D01 | d1 | E01 |
| 2-1-d1E01-1-0796 | 754.264 | OH | a2 | B17 | b1 | C31 | c3 | D02 | d1 | E01 |
| 2-1-d1E01-1-0797 | 755.183 | OH | a1 | B08 | b2 | C03 | c2 | D02 | d1 | E01 |
| 2-1-d1E01-1-0798 | 755.187 | OH | a1 | B09 | b2 | C01 | c2 | D02 | d1 | E01 |
| 2-1-d1E01-1-0799 | 755.199 | OH | a1 | B10 | b1 | C10 | c2 | D02 | d1 | E01 |
| 2-1-d1E01-1-0800 | 755.204 | OH | a1 | B04 | b1 | C09 | c2 | D02 | d1 | E01 |
| 2-1-d1E01-1-0801 | 755.208 | OH | a1 | B06 | b3 | C17 | c1 | D01 | d1 | E01 |
| 2-1-d1E01-1-0802 | 755.208 | OH | a1 | B06 | b1 | C14 | c2 | D02 | d1 | E01 |
| 2-1-d1E01-1-0803 | 755.279 | OH | a1 | B04 | b1 | C31 | c3 | D02 | d1 | E01 |
| 2-1-d1E01-1-0804 | 756.119 | OH | a2 | B17 | b3 | C06 | c2 | D02 | d1 | E01 |
| 2-1-d1E01-1-0805 | 756.208 | OH | a2 | B18 | b3 | C17 | c1 | D01 | d1 | E01 |
| 2-1-d1E01-1-0806 | 756.208 | OH | a2 | B18 | b1 | C14 | c2 | D02 | d1 | E01 |
| 2-1-d1E01-1-0807 | 757.135 | OH | a1 | B04 | b3 | C06 | c2 | D02 | d1 | E01 |
| 2-1-d1E01-1-0808 | 757.184 | OH | a2 | B17 | b3 | C18 | c1 | D01 | d1 | E01 |
| 2-1-d1E01-1-0809 | 757.213 | OH | a1 | B07 | b1 | C13 | c2 | D02 | d1 | E01 |
| 2-1-d1E01-1-0810 | 757.234 | OH | a1 | B10 | b1 | C09 | c1 | D01 | d1 | E01 |
| 2-1-d1E01-1-0811 | 757.315 | OH | a1 | B05 | b2 | C07 | c2 | D02 | d1 | E01 |
| 2-1-d1E01-1-0812 | 758.199 | OH | a1 | B04 | b3 | C18 | c1 | D01 | d1 | E01 |
| 2-1-d1E01-1-0813 | 758.199 | OH | a1 | B08 | b2 | C23 | c3 | D02 | d1 | E01 |
| 2-1-d1E01-1-0814 | 758.275 | OH | a2 | B19 | b1 | C29 | c3 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0815 | 759.148 | OH | a1 | B07 | b3 | C15 | c2 | D02 | d1 | E01 |
| 2-1-d1E01-1-0816 | 759.164 | OH | a1 | B10 | b3 | C06 | c1 | D01 | d1 | E01 |
| 2-1-d1E01-1-0817 | 759.217 | OH | a1 | B07 | b3 | C16 | c1 | D01 | d1 | E01 |
| 2-1-d1E01-1-0818 | 759.294 | OH | a1 | B06 | b2 | C07 | c2 | D02 | d1 | E01 |
| 2-1-d1E01-1-0819 | 760.144 | OH | a1 | B08 | b2 | C06 | c2 | D02 | d1 | E01 |
| 2-1-d1E01-1-0820 | 760.160 | OH | a1 | B10 | b1 | C12 | c2 | D02 | d1 | E01 |
| 2-1-d1E01-1-0821 | 760.254 | OH | a2 | B20 | b1 | C32 | c3 | D02 | d1 | E01 |
| 2-1-d1E01-1-0822 | 760.293 | OH | a2 | B18 | b2 | C07 | c2 | D02 | d1 | E01 |
| 2-1-d1E01-1-0823 | 761.179 | OH | a1 | B07 | b3 | C17 | c1 | D01 | d1 | E01 |
| 2-1-d1E01-1-0824 | 761.179 | OH | a1 | B07 | b1 | C14 | c2 | D02 | d1 | E01 |
| 2-1-d1E01-1-0825 | 761.213 | OH | a2 | B19 | b2 | C05 | c2 | D02 | d1 | E01 |
| 2-1-d1E01-1-0826 | 761.219 | OH | a1 | B05 | b3 | C08 | c2 | D02 | d1 | E01 |
| 2-1-d1E01-1-0827 | 762.197 | OH | a2 | B20 | b2 | C02 | c2 | D02 | d1 | E01 |
| 2-1-d1E01-1-0828 | 762.234 | OH | a2 | B20 | b2 | C21 | c3 | D02 | d1 | E01 |
| 2-1-d1E01-1-0829 | 762.234 | OH | a2 | B19 | b2 | C25 | c3 | D02 | d1 | E01 |
| 2-1-d1E01-1-0830 | 763.192 | OH | a2 | B20 | b2 | C03 | c2 | D02 | d1 | E01 |
| 2-1-d1E01-1-0831 | 763.198 | OH | a1 | B06 | b3 | C08 | c2 | D02 | d1 | E01 |
| 2-1-d1E01-1-0832 | 763.265 | OH | a1 | B09 | b1 | C32 | c3 | D02 | d1 | E01 |
| 2-1-d1E01-1-0833 | 764.193 | OH | a2 | B19 | b2 | C04 | c2 | D02 | d1 | E01 |
| 2-1-d1E01-1-0834 | 764.197 | OH | a2 | B18 | b3 | C08 | c2 | D02 | d1 | E01 |
| 2-1-d1E01-1-0835 | 765.208 | OH | a1 | B09 | b2 | C02 | c2 | D02 | d1 | E01 |
| 2-1-d1E01-1-0836 | 765.244 | OH | a1 | B05 | b1 | C09 | c2 | D02 | d1 | E01 |
| 2-1-d1E01-1-0837 | 765.244 | OH | a1 | B09 | b2 | C21 | c3 | D02 | d1 | E01 |
| 2-1-d1E01-1-0838 | 765.244 | OH | a1 | B10 | b1 | C30 | c3 | D02 | d1 | E01 |
| 2-1-d1E01-1-0839 | 765.264 | OH | a1 | B07 | b2 | C07 | c2 | D02 | d1 | E01 |
| 2-1-d1E01-1-0840 | 765.320 | OH | a1 | B05 | b1 | C31 | c3 | D02 | d1 | E01 |
| 2-1-d1E01-1-0841 | 766.203 | OH | a1 | B09 | b2 | C03 | c2 | D02 | d1 | E01 |
| 2-1-d1E01-1-0842 | 766.208 | OH | a2 | B20 | b2 | C23 | c3 | D02 | d1 | E01 |
| 2-1-d1E01-1-0843 | 766.261 | OH | a1 | B08 | b1 | C29 | c3 | D02 | d1 | E01 |
| 2-1-d1E01-1-0844 | 767.175 | OH | a1 | B05 | b3 | C06 | c2 | D02 | d1 | E01 |
| 2-1-d1E01-1-0845 | 767.224 | OH | a1 | B06 | b1 | C09 | c2 | D02 | d1 | E01 |
| 2-1-d1E01-1-0846 | 767.224 | OH | a1 | B10 | b2 | C19 | c3 | D02 | d1 | E01 |
| 2-1-d1E01-1-0847 | 767.299 | OH | a1 | B06 | b1 | C31 | c3 | D02 | d1 | E01 |
| 2-1-d1E01-1-0848 | 768.154 | OH | a2 | B20 | b2 | C06 | c2 | D02 | d1 | E01 |
| 2-1-d1E01-1-0849 | 768.192 | OH | a2 | B11 | b3 | C16 | c2 | D02 | d1 | E01 |
| 2-1-d1E01-1-0850 | 768.219 | OH | a1 | B10 | b2 | C27 | c3 | D02 | d1 | E01 |
| 2-1-d1E01-1-0851 | 768.223 | OH | a2 | B18 | b1 | C09 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0852 | 768.240 | OH | a1 | B05 | b3 | C18 | c1 | D01 | d1 | E01 |
| 2-1-d1E01-1-0853 | 768.298 | OH | a2 | B18 | b1 | C31 | c3 | D02 | d1 | E01 |
| 2-1-d1E01-1-0854 | 769.155 | OH | a1 | B06 | b3 | C06 | c2 | D02 | d1 | E01 |
| 2-1-d1E01-1-0855 | 769.169 | OH | a1 | B07 | b3 | C08 | c2 | D02 | d1 | E01 |
| 2-1-d1E01-1-0856 | 769.188 | OH | a2 | B12 | b3 | C16 | c2 | D02 | d1 | E01 |
| 2-1-d1E01-1-0857 | 769.199 | OH | a1 | B08 | b2 | C05 | c2 | D02 | d1 | E01 |
| 2-1-d1E01-1-0858 | 769.219 | OH | a1 | B09 | b2 | C23 | c3 | D02 | d1 | E01 |
| 2-1-d1E01-1-0859 | 770.154 | OH | a2 | B18 | b3 | C06 | c2 | D02 | d1 | E01 |
| 2-1-d1E01-1-0860 | 770.154 | OH | a2 | B11 | b3 | C17 | c2 | D02 | d1 | E01 |
| 2-1-d1E01-1-0861 | 770.213 | OH | a2 | B19 | b1 | C13 | c2 | D02 | d1 | E01 |
| 2-1-d1E01-1-0862 | 770.219 | OH | a1 | B06 | b3 | C18 | c1 | D01 | d1 | E01 |
| 2-1-d1E01-1-0863 | 770.219 | OH | a1 | B08 | b2 | C25 | c3 | D02 | d1 | E01 |
| 2-1-d1E01-1-0864 | 771.149 | OH | a2 | B12 | b3 | C17 | c2 | D02 | d1 | E01 |
| 2-1-d1E01-1-0865 | 771.164 | OH | a1 | B09 | b2 | C06 | c2 | D02 | d1 | E01 |
| 2-1-d1E01-1-0866 | 771.182 | OH | a1 | B10 | b2 | C01 | c2 | D02 | d1 | E01 |
| 2-1-d1E01-1-0867 | 771.219 | OH | a2 | B18 | b3 | C18 | c1 | D01 | d1 | E01 |
| 2-1-d1E01-1-0868 | 772.148 | OH | a2 | B19 | b3 | C15 | c2 | D02 | d1 | E01 |
| 2-1-d1E01-1-0869 | 772.179 | OH | a1 | B08 | b2 | C04 | c2 | D02 | d1 | E01 |
| 2-1-d1E01-1-0870 | 772.218 | OH | a2 | B19 | b3 | C16 | c1 | D01 | d1 | E01 |
| 2-1-d1E01-1-0871 | 773.194 | OH | a1 | B07 | b1 | C09 | c2 | D02 | d1 | E01 |
| 2-1-d1E01-1-0872 | 773.269 | OH | a1 | B07 | b1 | C31 | c3 | D02 | d1 | E01 |
| 2-1-d1E01-1-0873 | 774.179 | OH | a2 | B19 | b3 | C17 | c1 | D01 | d1 | E01 |
| 2-1-d1E01-1-0874 | 774.179 | OH | a2 | B19 | b1 | C14 | c2 | D02 | d1 | E01 |
| 2-1-d1E01-1-0875 | 774.270 | OH | a2 | B20 | b1 | C29 | c3 | D02 | d1 | E01 |
| 2-1-d1E01-1-0876 | 775.125 | OH | a1 | B07 | b3 | C06 | c2 | D02 | d1 | E01 |
| 2-1-d1E01-1-0877 | 776.190 | OH | a1 | B07 | b3 | C18 | c1 | D01 | d1 | E01 |
| 2-1-d1E01-1-0878 | 777.208 | OH | a2 | B20 | b2 | C05 | c2 | D02 | d1 | E01 |
| 2-1-d1E01-1-0879 | 777.281 | OH | a1 | B09 | b1 | C29 | c3 | D02 | d1 | E01 |
| 2-1-d1E01-1-0880 | 778.199 | OH | a1 | B08 | b1 | C13 | c2 | D02 | d1 | E01 |
| 2-1-d1E01-1-0881 | 778.228 | OH | a2 | B20 | b2 | C25 | c3 | D02 | d1 | E01 |
| 2-1-d1E01-1-0882 | 778.265 | OH | a2 | B19 | b2 | C07 | c2 | D02 | d1 | E01 |
| 2-1-d1E01-1-0883 | 779.260 | OH | a1 | B10 | b1 | C32 | c3 | D02 | d1 | E01 |
| 2-1-d1E01-1-0884 | 780.134 | OH | a1 | B08 | b3 | C15 | c2 | D02 | d1 | E01 |
| 2-1-d1E01-1-0885 | 780.188 | OH | a2 | B20 | b2 | C04 | c2 | D02 | d1 | E01 |
| 2-1-d1E01-1-0886 | 780.204 | OH | a1 | B08 | b3 | C16 | c1 | D01 | d1 | E01 |
| 2-1-d1E01-1-0887 | 780.219 | OH | a1 | B09 | b2 | C05 | c2 | D02 | d1 | E01 |
| 2-1-d1E01-1-0888 | 781.203 | OH | a1 | B10 | b2 | C02 | c2 | D02 | d1 | E01 |
| 2-1-d1E01-1-0889 | 781.239 | OH | a1 | B10 | b2 | C21 | c3 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0890 | 781.239 | OH | a1 | B09 | b2 | C25 | c3 | D02 | d1 | E01 |
| 2-1-d1E01-1-0891 | 782.165 | OH | a1 | B08 | b3 | C17 | c1 | D01 | d1 | E01 |
| 2-1-d1E01-1-0892 | 782.165 | OH | a1 | B08 | b1 | C14 | c2 | D02 | d1 | E01 |
| 2-1-d1E01-1-0893 | 782.169 | OH | a2 | B19 | b3 | C08 | c2 | D02 | d1 | E01 |
| 2-1-d1E01-1-0894 | 782.198 | OH | a1 | B10 | b2 | C03 | c2 | D02 | d1 | E01 |
| 2-1-d1E01-1-0895 | 782.208 | OH | a2 | B13 | b3 | C16 | c2 | D02 | d1 | E01 |
| 2-1-d1E01-1-0896 | 783.199 | OH | a1 | B09 | b2 | C04 | c2 | D02 | d1 | E01 |
| 2-1-d1E01-1-0897 | 784.170 | OH | a2 | B13 | b3 | C17 | c2 | D02 | d1 | E01 |
| 2-1-d1E01-1-0898 | 785.165 | OH | a2 | B11 | b3 | C18 | c2 | D02 | d1 | E01 |
| 2-1-d1E01-1-0899 | 785.214 | OH | a1 | B10 | b2 | C23 | c3 | D02 | d1 | E01 |
| 2-1-d1E01-1-0900 | 786.160 | OH | a2 | B12 | b3 | C18 | c2 | D02 | d1 | E01 |
| 2-1-d1E01-1-0901 | 786.183 | OH | a2 | B14 | b3 | C16 | c2 | D02 | d1 | E01 |
| 2-1-d1E01-1-0902 | 786.195 | OH | a2 | B19 | b1 | C09 | c2 | D02 | d1 | E01 |
| 2-1-d1E01-1-0903 | 786.208 | OH | a2 | B20 | b1 | C13 | c2 | D02 | d1 | E01 |
| 2-1-d1E01-1-0904 | 786.251 | OH | a1 | B08 | b2 | C07 | c2 | D02 | d1 | E01 |
| 2-1-d1E01-1-0905 | 786.270 | OH | a2 | B19 | b1 | C31 | c3 | D02 | d1 | E01 |
| 2-1-d1E01-1-0906 | 787.159 | OH | a1 | B10 | b2 | C06 | c2 | D02 | d1 | E01 |
| 2-1-d1E01-1-0907 | 787.198 | OH | a1 | B01 | b3 | C16 | c2 | D02 | d1 | E01 |
| 2-1-d1E01-1-0908 | 788.126 | OH | a2 | B19 | b3 | C06 | c2 | D02 | d1 | E01 |
| 2-1-d1E01-1-0909 | 788.143 | OH | a2 | B20 | b3 | C15 | c2 | D02 | d1 | E01 |
| 2-1-d1E01-1-0910 | 788.144 | OH | a2 | B14 | b3 | C17 | c2 | D02 | d1 | E01 |
| 2-1-d1E01-1-0911 | 788.193 | OH | a1 | B02 | b3 | C16 | c2 | D02 | d1 | E01 |
| 2-1-d1E01-1-0912 | 788.213 | OH | a2 | B20 | b3 | C16 | c1 | D01 | d1 | E01 |
| 2-1-d1E01-1-0913 | 789.160 | OH | a1 | B01 | b3 | C17 | c2 | D02 | d1 | E01 |
| 2-1-d1E01-1-0914 | 789.190 | OH | a2 | B19 | b3 | C18 | c1 | D01 | d1 | E01 |
| 2-1-d1E01-1-0915 | 789.219 | OH | a1 | B09 | b1 | C13 | c2 | D02 | d1 | E01 |
| 2-1-d1E01-1-0916 | 790.155 | OH | a1 | B02 | b3 | C17 | c2 | D02 | d1 | E01 |
| 2-1-d1E01-1-0917 | 790.155 | OH | a1 | B08 | b3 | C08 | c2 | D02 | d1 | E01 |
| 2-1-d1E01-1-0918 | 790.174 | OH | a2 | B20 | b3 | C17 | c1 | D01 | d1 | E01 |
| 2-1-d1E01-1-0919 | 790.174 | OH | a2 | B20 | b1 | C14 | c2 | D02 | d1 | E01 |
| 2-1-d1E01-1-0920 | 791.154 | OH | a1 | B09 | b3 | C15 | c2 | D02 | d1 | E01 |
| 2-1-d1E01-1-0921 | 791.224 | OH | a1 | B09 | b3 | C16 | c1 | D01 | d1 | E01 |
| 2-1-d1E01-1-0922 | 793.185 | OH | a1 | B09 | b3 | C17 | c1 | D01 | d1 | E01 |
| 2-1-d1E01-1-0923 | 793.185 | OH | a1 | B09 | b1 | C14 | c2 | D02 | d1 | E01 |
| 2-1-d1E01-1-0924 | 793.276 | OH | a1 | B10 | b1 | C29 | c3 | D02 | d1 | E01 |
| 2-1-d1E01-1-0925 | 794.180 | OH | a1 | B08 | b1 | C09 | c2 | D02 | d1 | E01 |
| 2-1-d1E01-1-0926 | 794.256 | OH | a1 | B08 | b1 | C31 | c3 | D02 | d1 | E01 |
| 2-1-d1E01-1-0927 | 794.260 | OH | a2 | B20 | b2 | C07 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0928 | 796.111 | OH | a1 | B08 | b3 | C06 | c2 | D02 | d1 | E01 |
| 2-1-d1E01-1-0929 | 796.214 | OH | a1 | B10 | b2 | C05 | c2 | D02 | d1 | E01 |
| 2-1-d1E01-1-0930 | 796.224 | OH | a2 | B15 | b3 | C16 | c2 | D02 | d1 | E01 |
| 2-1-d1E01-1-0931 | 797.176 | OH | a1 | B08 | b3 | C18 | c1 | D01 | d1 | E01 |
| 2-1-d1E01-1-0932 | 797.234 | OH | a1 | B10 | b2 | C25 | c3 | D02 | d1 | E01 |
| 2-1-d1E01-1-0933 | 797.271 | OH | a1 | B09 | b2 | C07 | c2 | D02 | d1 | E01 |
| 2-1-d1E01-1-0934 | 798.164 | OH | a2 | B20 | b3 | C08 | c2 | D02 | d1 | E01 |
| 2-1-d1E01-1-0935 | 798.185 | OH | a2 | B15 | b3 | C17 | c2 | D02 | d1 | E01 |
| 2-1-d1E01-1-0936 | 798.203 | OH | a2 | B16 | b3 | C16 | c2 | D02 | d1 | E01 |
| 2-1-d1E01-1-0937 | 799.180 | OH | a2 | B13 | b3 | C18 | c2 | D02 | d1 | E01 |
| 2-1-d1E01-1-0938 | 799.194 | OH | a1 | B10 | b2 | C04 | c2 | D02 | d1 | E01 |
| 2-1-d1E01-1-0939 | 800.164 | OH | a2 | B16 | b3 | C17 | c2 | D02 | d1 | E01 |
| 2-1-d1E01-1-0940 | 801.175 | OH | a1 | B09 | b3 | C08 | c2 | D02 | d1 | E01 |
| 2-1-d1E01-1-0941 | 801.214 | OH | a1 | B03 | b3 | C16 | c2 | D02 | d1 | E01 |
| 2-1-d1E01-1-0942 | 802.190 | OH | a2 | B20 | b1 | C09 | c2 | D02 | d1 | E01 |
| 2-1-d1E01-1-0943 | 802.265 | OH | a2 | B20 | b1 | C31 | c3 | D02 | d1 | E01 |
| 2-1-d1E01-1-0944 | 803.155 | OH | a2 | B14 | b3 | C18 | c2 | D02 | d1 | E01 |
| 2-1-d1E01-1-0945 | 803.175 | OH | a1 | B03 | b3 | C17 | c2 | D02 | d1 | E01 |
| 2-1-d1E01-1-0946 | 804.121 | OH | a2 | B20 | b3 | C06 | c2 | D02 | d1 | E01 |
| 2-1-d1E01-1-0947 | 804.171 | OH | a1 | B01 | b3 | C18 | c2 | D02 | d1 | E01 |
| 2-1-d1E01-1-0948 | 804.174 | OH | a2 | B17 | b3 | C16 | c2 | D02 | d1 | E01 |
| 2-1-d1E01-1-0949 | 805.166 | OH | a1 | B02 | b3 | C18 | c2 | D02 | d1 | E01 |
| 2-1-d1E01-1-0950 | 805.185 | OH | a2 | B20 | b3 | C18 | c1 | D01 | d1 | E01 |
| 2-1-d1E01-1-0951 | 805.189 | OH | a1 | B04 | b3 | C16 | c2 | D02 | d1 | E01 |
| 2-1-d1E01-1-0952 | 805.200 | OH | a1 | B09 | b1 | C09 | c2 | D02 | d1 | E01 |
| 2-1-d1E01-1-0953 | 805.214 | OH | a1 | B10 | b1 | C13 | c2 | D02 | d1 | E01 |
| 2-1-d1E01-1-0954 | 805.276 | OH | a1 | B09 | b1 | C31 | c3 | D02 | d1 | E01 |
| 2-1-d1E01-1-0955 | 806.135 | OH | a2 | B17 | b3 | C17 | c2 | D02 | d1 | E01 |
| 2-1-d1E01-1-0956 | 807.131 | OH | a1 | B09 | b3 | C06 | c2 | D02 | d1 | E01 |
| 2-1-d1E01-1-0957 | 807.149 | OH | a1 | B10 | b3 | C15 | c2 | D02 | d1 | E01 |
| 2-1-d1E01-1-0958 | 807.150 | OH | a1 | B04 | b3 | C17 | c2 | D02 | d1 | E01 |
| 2-1-d1E01-1-0959 | 807.219 | OH | a1 | B10 | b3 | C16 | c1 | D01 | d1 | E01 |
| 2-1-d1E01-1-0960 | 808.196 | OH | a1 | B09 | b3 | C18 | c1 | D01 | d1 | E01 |
| 2-1-d1E01-1-0961 | 809.180 | OH | a1 | B10 | b3 | C17 | c1 | D01 | d1 | E01 |
| 2-1-d1E01-1-0962 | 809.180 | OH | a1 | B10 | b1 | C14 | c2 | D02 | d1 | E01 |
| 2-1-d1E01-1-0963 | 813.196 | OH | a2 | B15 | b3 | C18 | c2 | D02 | d1 | E01 |
| 2-1-d1E01-1-0964 | 813.266 | OH | a1 | B10 | b2 | C07 | c2 | D02 | d1 | E01 |

(continued)

| [Table 8-7-2] Compound that may be contained in mixture 2-1-d1E01-1 | | | | | | | | | | |
|---|---|---|---|---|---|---|---|---|---|---|
| ID | Exact Mass | | a | B | b | C | c | D | d | E |
| 2-1-d1E01-1-0965 | 815.175 | OH | a2 | B16 | b3 | C18 | c2 | D02 | d1 | E01 |
| 2-1-d1E01-1-0966 | 815.229 | OH | a1 | B05 | b3 | C16 | c2 | D02 | d1 | E01 |
| 2-1-d1E01-1-0967 | 817.170 | OH | a1 | B10 | b3 | C08 | c2 | D02 | d1 | E01 |
| 2-1-d1E01-1-0968 | 817.191 | OH | a1 | B05 | b3 | C17 | c2 | D02 | d1 | E01 |
| 2-1-d1E01-1-0969 | 817.209 | OH | a1 | B06 | b3 | C16 | c2 | D02 | d1 | E01 |
| 2-1-d1E01-1-0970 | 818.186 | OH | a1 | B03 | b3 | C18 | c2 | D02 | d1 | E01 |
| 2-1-d1E01-1-0971 | 818.208 | OH | a2 | B18 | b3 | C16 | c2 | D02 | d1 | E01 |
| 2-1-d1E01-1-0972 | 819.170 | OH | a1 | B06 | b3 | C17 | c2 | D02 | d1 | E01 |
| 2-1-d1E01-1-0973 | 820.170 | OH | a2 | B18 | b3 | C17 | c2 | D02 | d1 | E01 |
| 2-1-d1E01-1-0974 | 821.146 | OH | a2 | B17 | b3 | C18 | c2 | D02 | d1 | E01 |
| 2-1-d1E01-1-0975 | 821.195 | OH | a1 | B10 | b1 | C09 | c2 | D02 | d1 | E01 |
| 2-1-d1E01-1-0976 | 821.271 | OH | a1 | B10 | b1 | C31 | c3 | D02 | d1 | E01 |
| 2-1-d1E01-1-0977 | 822.161 | OH | a1 | B04 | b3 | C18 | c2 | D02 | d1 | E01 |
| 2-1-d1E01-1-0978 | 823.126 | OH | a1 | B10 | b3 | C06 | c2 | D02 | d1 | E01 |
| 2-1-d1E01-1-0979 | 823.179 | OH | a1 | B07 | b3 | C16 | c2 | D02 | d1 | E01 |
| 2-1-d1E01-1-0980 | 824.191 | OH | a1 | B10 | b3 | C18 | c1 | D01 | d1 | E01 |
| 2-1-d1E01-1-0981 | 825.141 | OH | a1 | B07 | b3 | C17 | c2 | D02 | d1 | E01 |
| 2-1-d1E01-1-0982 | 832.202 | OH | a1 | B05 | b3 | C18 | c2 | D02 | d1 | E01 |
| 2-1-d1E01-1-0983 | 834.181 | OH | a1 | B06 | b3 | C18 | c2 | D02 | d1 | E01 |
| 2-1-d1E01-1-0984 | 835.180 | OH | a2 | B18 | b3 | C18 | c2 | D02 | d1 | E01 |
| 2-1-d1E01-1-0985 | 836.180 | OH | a2 | B19 | b3 | C16 | c2 | D02 | d1 | E01 |
| 2-1-d1E01-1-0986 | 838.141 | OH | a2 | B19 | b3 | C17 | c2 | D02 | d1 | E01 |
| 2-1-d1E01-1-0987 | 840.152 | OH | a1 | B07 | b3 | C18 | c2 | D02 | d1 | E01 |
| 2-1-d1E01-1-0988 | 844.166 | OH | a1 | B08 | b3 | C16 | c2 | D02 | d1 | E01 |
| 2-1-d1E01-1-0989 | 846.127 | OH | a1 | B08 | b3 | C17 | c2 | D02 | d1 | E01 |
| 2-1-d1E01-1-0990 | 852.175 | OH | a2 | B20 | b3 | C16 | c2 | D02 | d1 | E01 |
| 2-1-d1E01-1-0991 | 853.152 | OH | a2 | B19 | b3 | C18 | c2 | D02 | d1 | E01 |
| 2-1-d1E01-1-0992 | 854.136 | OH | a2 | B20 | b3 | C17 | c2 | D02 | d1 | E01 |
| 2-1-d1E01-1-0993 | 855.186 | OH | a1 | B09 | b3 | C16 | c2 | D02 | d1 | E01 |
| 2-1-d1E01-1-0994 | 857.147 | OH | a1 | B09 | b3 | C17 | c2 | D02 | d1 | E01 |
| 2-1-d1E01-1-0995 | 861.138 | OH | a1 | B08 | b3 | C18 | c2 | D02 | d1 | E01 |
| 2-1-d1E01-1-0996 | 869.147 | OH | a2 | B20 | b3 | C18 | c2 | D02 | d1 | E01 |
| 2-1-d1E01-1-0997 | 871.180 | OH | a1 | B10 | b3 | C16 | c2 | D02 | d1 | E01 |
| 2-1-d1E01-1-0998 | 872.158 | OH | a1 | B09 | b3 | C18 | c2 | D02 | d1 | E01 |
| 2-1-d1E01-1-0999 | 873.142 | OH | a1 | B10 | b3 | C17 | c2 | D02 | d1 | E01 |
| 2-1-d1E01-1-1000 | 888.153 | OH | a1 | B10 | b3 | C18 | c2 | D02 | d1 | E01 |

[Table 389]

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0001 | 602.253 | OH | a2 | B11 | b1 | C08 | c1 | D01 | d1 | E02 |
| 2-1-d1E02-1-0002 | 603.248 | OH | a2 | B12 | b1 | C08 | c1 | D01 | d1 | E02 |
| 2-1-d1E02-1-0003 | 603.248 | OH | a2 | B11 | b1 | C11 | c1 | D01 | d1 | E02 |
| 2-1-d1E02-1-0004 | 604.243 | OH | a2 | B11 | b1 | C10 | c1 | D01 | d1 | E02 |
| 2-1-d1E02-1-0005 | 604.243 | OH | a2 | B12 | b1 | C11 | c1 | D01 | d1 | E02 |
| 2-1-d1E02-1-0006 | 605.239 | OH | a2 | B12 | b1 | C10 | c1 | D01 | d1 | E02 |
| 2-1-d1E02-1-0007 | 609.205 | OH | a2 | B11 | b1 | C12 | c1 | D01 | d1 | E02 |
| 2-1-d1E02-1-0008 | 610.200 | OH | a2 | B12 | b1 | C12 | c1 | D01 | d1 | E02 |
| 2-1-d1E02-1-0009 | 616.269 | OH | a2 | B13 | b1 | C08 | c1 | D01 | d1 | E02 |
| 2-1-d1E02-1-0010 | 617.264 | OH | a2 | B13 | b1 | C11 | c1 | D01 | d1 | E02 |
| 2-1-d1E02-1-0011 | 618.259 | OH | a2 | B13 | b1 | C10 | c1 | D01 | d1 | E02 |
| 2-1-d1E02-1-0012 | 620.227 | OH | a2 | B11 | b2 | C01 | c1 | D01 | d1 | E02 |
| 2-1-d1E02-1-0013 | 620.243 | OH | a2 | B14 | b1 | C08 | c1 | D01 | d1 | E02 |
| 2-1-d1E02-1-0014 | 621.222 | OH | a2 | B12 | b2 | C01 | c1 | D01 | d1 | E02 |
| 2-1-d1E02-1-0015 | 621.239 | OH | a2 | B14 | b1 | C11 | c1 | D01 | d1 | E02 |
| 2-1-d1E02-1-0016 | 621.259 | OH | a1 | B01 | b1 | C08 | c1 | D01 | d1 | E02 |
| 2-1-d1E02-1-0017 | 622.234 | OH | a2 | B14 | b1 | C10 | c1 | D01 | d1 | E02 |
| 2-1-d1E02-1-0018 | 622.254 | OH | a1 | B02 | b1 | C08 | c1 | D01 | d1 | E02 |
| 2-1-d1E02-1-0019 | 622.254 | OH | a1 | B01 | b1 | C11 | c1 | D01 | d1 | E02 |
| 2-1-d1E02-1-0020 | 623.220 | OH | a2 | B13 | b1 | C12 | c1 | D01 | d1 | E02 |
| 2-1-d1E02-1-0021 | 623.249 | OH | a1 | B01 | b1 | C10 | c1 | D01 | d1 | E02 |
| 2-1-d1E02-1-0022 | 623.249 | OH | a1 | B02 | b1 | C11 | c1 | D01 | d1 | E02 |
| 2-1-d1E02-1-0023 | 624.244 | OH | a1 | B02 | b1 | C10 | c1 | D01 | d1 | E02 |
| 2-1-d1E02-1-0024 | 627.195 | OH | a2 | B14 | b1 | C12 | c1 | D01 | d1 | E02 |
| 2-1-d1E02-1-0025 | 628.210 | OH | a1 | B01 | b1 | C12 | c1 | D01 | d1 | E02 |
| 2-1-d1E02-1-0026 | 629.206 | OH | a1 | B02 | b1 | C12 | c1 | D01 | d1 | E02 |
| 2-1-d1E02-1-0027 | 630.248 | OH | a2 | B11 | b2 | C02 | c1 | D01 | d1 | E02 |
| 2-1-d1E02-1-0028 | 630.284 | OH | a2 | B15 | b1 | C08 | c1 | D01 | d1 | E02 |
| 2-1-d1E02-1-0029 | 631.243 | OH | a2 | B12 | b2 | C02 | c1 | D01 | d1 | E02 |
| 2-1-d1E02-1-0030 | 631.243 | OH | a2 | B11 | b2 | C03 | c1 | D01 | d1 | E02 |
| 2-1-d1E02-1-0031 | 631.279 | OH | a2 | B15 | b1 | C11 | c1 | D01 | d1 | E02 |
| 2-1-d1E02-1-0032 | 632.238 | OH | a2 | B12 | b2 | C03 | c1 | D01 | d1 | E02 |
| 2-1-d1E02-1-0033 | 632.263 | OH | a2 | B16 | b1 | C08 | c1 | D01 | d1 | E02 |
| 2-1-d1E02-1-0034 | 632.275 | OH | a2 | B15 | b1 | C10 | c1 | D01 | d1 | E02 |
| 2-1-d1E02-1-0035 | 633.259 | OH | a2 | B16 | b1 | C11 | c1 | D01 | d1 | E02 |
| 2-1-d1E02-1-0036 | 634.243 | OH | a2 | B13 | b2 | C01 | c1 | D01 | d1 | E02 |
| 2-1-d1E02-1-0037 | 634.254 | OH | a2 | B16 | b1 | C10 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0038 | 635.274 | OH | a1 | B03 | b1 | C08 | c1 | D01 | d1 | E02 |
| 2-1-d1E02-1-0039 | 636.204 | OH | a2 | B11 | b2 | C06 | c1 | D01 | d1 | E02 |
| 2-1-d1E02-1-0040 | 636.27 | OH | a1 | B03 | b1 | C11 | c1 | D01 | d1 | E02 |
| 2-1-d1E02-1-0041 | 637.200 | OH | a2 | B12 | b2 | C06 | c1 | D01 | d1 | E02 |
| 2-1-d1E02-1-0042 | 637.236 | OH | a2 | B15 | b1 | C12 | c1 | D01 | d1 | E02 |
| 2-1-d1E02-1-0043 | 637.265 | OH | a1 | B03 | b1 | C10 | c1 | D01 | d1 | E02 |
| 2-1-d1E02-1-0044 | 638.218 | OH | a2 | B14 | b2 | C01 | c1 | D01 | d1 | E02 |
| 2-1-d1E02-1-0045 | 638.234 | OH | a2 | B17 | b1 | C08 | c1 | D01 | d1 | E02 |
| 2-1-d1E02-1-0046 | 639.215 | OH | a2 | B16 | b1 | C12 | c1 | D01 | d1 | E02 |
| 2-1-d1E02-1-0047 | 639.229 | OH | a2 | B17 | b1 | C11 | c1 | D01 | d1 | E02 |
| 2-1-d1E02-1-0048 | 639.233 | OH | a1 | B01 | b2 | C01 | c1 | D01 | d1 | E02 |
| 2-1-d1E02-1-0049 | 639.249 | OH | a1 | B04 | b1 | C08 | c1 | D01 | d1 | E02 |
| 2-1-d1E02-1-0050 | 640.225 | OH | a2 | B17 | b1 | C10 | c1 | D01 | d1 | E02 |
| 2-1-d1E02-1-0051 | 640.228 | OH | a1 | B02 | b2 | C01 | c1 | D01 | d1 | E02 |
| 2-1-d1E02-1-0052 | 640.245 | OH | a1 | B04 | b1 | C11 | c1 | D01 | d1 | E02 |
| 2-1-d1E02-1-0053 | 641.240 | OH | a1 | B04 | b1 | C10 | c1 | D01 | d1 | E02 |
| 2-1-d1E02-1-0054 | 642.226 | OH | a1 | B03 | b1 | C12 | c1 | D01 | d1 | E02 |
| 2-1-d1E02-1-0055 | 644.263 | OH | a2 | B13 | b2 | C02 | c1 | D01 | d1 | E02 |
| 2-1-d1E02-1-0056 | 645.186 | OH | a2 | B17 | b1 | C12 | c1 | D01 | d1 | E02 |
| 2-1-d1E02-1-0057 | 645.259 | OH | a2 | B13 | b2 | C03 | c1 | D01 | d1 | E02 |
| 2-1-d1E02-1-0058 | 645.259 | OH | a2 | B11 | b2 | C05 | c1 | D01 | d1 | E02 |
| 2-1-d1E02-1-0059 | 646.201 | OH | a1 | B04 | b1 | C12 | c1 | D01 | d1 | E02 |
| 2-1-d1E02-1-0060 | 646.254 | OH | a2 | B12 | b2 | C05 | c1 | D01 | d1 | E02 |
| 2-1-d1E02-1-0061 | 648.238 | OH | a2 | B14 | b2 | C02 | c1 | D01 | d1 | E02 |
| 2-1-d1E02-1-0062 | 648.238 | OH | a2 | B11 | b2 | C04 | c1 | D01 | d1 | E02 |
| 2-1-d1E02-1-0063 | 648.258 | OH | a2 | B15 | b2 | C01 | c1 | D01 | d1 | E02 |
| 2-1-d1E02-1-0064 | 649.234 | OH | a2 | B12 | b2 | C04 | c1 | D01 | d1 | E02 |
| 2-1-d1E02-1-0065 | 649.234 | OH | a2 | B14 | b2 | C03 | c1 | D01 | d1 | E02 |
| 2-1-d1E02-1-0066 | 649.254 | OH | a1 | B01 | b2 | C02 | c1 | D01 | d1 | E02 |
| 2-1-d1E02-1-0067 | 649.290 | OH | a1 | B05 | b1 | C08 | c1 | D01 | d1 | E02 |
| 2-1-d1E02-1-0068 | 650.220 | OH | a2 | B13 | b2 | C06 | c1 | D01 | d1 | E02 |
| 2-1-d1E02-1-0069 | 650.238 | OH | a2 | B16 | b2 | C01 | c1 | D01 | d1 | E02 |
| 2-1-d1E02-1-0070 | 650.249 | OH | a1 | B02 | b2 | C02 | c1 | D01 | d1 | E02 |
| 2-1-d1E02-1-0071 | 650.249 | OH | a1 | B01 | b2 | C03 | c1 | D01 | d1 | E02 |
| 2-1-d1E02-1-0072 | 650.285 | OH | a1 | B05 | b1 | C11 | c1 | D01 | d1 | E02 |
| 2-1-d1E02-1-0073 | 651.244 | OH | a1 | B02 | b2 | C03 | c1 | D01 | d1 | E02 |
| 2-1-d1E02-1-0074 | 651.269 | OH | a1 | B06 | b1 | C08 | c1 | D01 | d1 | E02 |
| 2-1-dlE02-1-0075 | 651.281 | OH | a1 | B05 | b1 | C10 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0076 | 652.265 | OH | a1 | B06 | b1 | C11 | c1 | D01 | d1 | E02 |
| 2-1-d1E02-1-0077 | 652.269 | OH | a2 | B18 | b1 | C08 | c1 | D01 | d1 | E02 |
| 2-1-dlE02-1-0078 | 653.249 | OH | a1 | B03 | b2 | C01 | c1 | D01 | d1 | E02 |
| 2-1-d1E02-1-0079 | 653.260 | OH | a1 | B06 | b1 | C10 | c1 | D01 | d1 | E02 |
| 2-1-dlE02-1-0080 | 653.264 | OH | a2 | B18 | b1 | C11 | c1 | D01 | d1 | E02 |
| 2-1-d1E02-1-0081 | 654.195 | OH | a2 | B14 | b2 | C06 | c1 | D01 | d1 | E02 |
| 2-1-dlE02-1-0082 | 654.259 | OH | a2 | B18 | b1 | C10 | c1 | D01 | d1 | E02 |
| 2-1-d1E02-1-0083 | 654.259 | OH | a2 | B11 | b1 | C13 | c1 | D01 | d1 | E02 |
| 2-1-d1E02-1-0084 | 655.210 | OH | a1 | B01 | b2 | C06 | c1 | D01 | d1 | E02 |
| 2-1-dlE02-1-0085 | 655.254 | OH | a2 | B12 | b1 | C13 | c1 | D01 | d1 | E02 |
| 2-1-dlE02-1-0086 | 656.194 | OH | a2 | B11 | b3 | C15 | c1 | D01 | d1 | E02 |
| 2-1-d1E02-1-0087 | 656.205 | OH | a1 | B02 | b2 | C06 | c1 | D01 | d1 | E02 |
| 2-1-dlE02-1-0088 | 656.208 | OH | a2 | B17 | b2 | C01 | c1 | D01 | d1 | E02 |
| 2-1-d1E02-1-0089 | 656.242 | OH | a1 | B05 | b1 | C12 | c1 | D01 | d1 | E02 |
| 2-1-d1E02-1-0090 | 657.189 | OH | a2 | B12 | b3 | C15 | c1 | D01 | d1 | E02 |
| 2-1-dlE02-1-0091 | 657.223 | OH | a1 | B04 | b2 | C01 | c1 | D01 | d1 | E02 |
| 2-1-d1E02-1-0092 | 657.240 | OH | a1 | B07 | b1 | C08 | c1 | D01 | d1 | E02 |
| 2-1-d1E02-1-0093 | 658.221 | OH | a1 | B06 | b1 | C12 | c1 | D01 | d1 | E02 |
| 2-1-dlE02-1-0094 | 658.225 | OH | a2 | B11 | b | C14 | c1 | D01 | d1 | E02 |
| 2-1-d1E02-1-0095 | 658.235 | OH | a1 | B07 | b1 | C11 | c1 | D01 | d1 | E02 |
| 2-1-d1E02-1-0096 | 658.246 | OH | a2 | B11 | b2 | C33 | c3 | D02 | d1 | E02 |
| 2-1-d1E02-1-0097 | 658.279 | OH | a2 | B15 | b2 | C02 | c1 | D01 | d1 | E02 |
| 2-1-dlE02-1-0098 | 659.220 | OH | a2 | B18 | 11 b1 | C12 | c1 | D01 | d1 | E02 |
| 2-1-d1E02-1-0099 | 659.220 | OH | a2 | B12 | b1 | C14 | c1 | D01 | d1 | E02 |
| 2-1-d1E02-1-0100 | 659.230 | OH | a1 | B07 | b1 | C10 | c1 | D01 | d1 | E02 |
| 2-1-d1E02-1-0101 | 659.241 | OH | a2 | B12 | b2 | C33 | c3 | D02 | d1 | E02 |
| 2-1-dlE02-1-0102 | 659.274 | OH | a2 | B15 | b2 | C03 | c1 | D01 | d1 | E02 |
| 2-1-d1E02-1-0103 | 659.274 | OH | a2 | B13 | b2 | C05 | c1 | D01 | d1 | E02 |
| 2-1-dlE02-1-0104 | 660.258 | OH | a2 | B16 | b2 | C02 | c1 | D01 | d1 | E02 |
| 2-1-d1E02-1-0105 | 661.254 | OH | a2 | B16 | b2 | C03 | c1 | D01 | d1 | E02 |
| 2-1-d1E02-1-0106 | 662.254 | OH | a2 | B13 | b2 | C04 | c1 | D01 | d1 | E02 |
| 2-1-dlE02-1-0107 | 662.310 | OH | a2 | B11 | b2 | C07 | c1 | D01 | d1 | E02 |
| 2-1-d1E02-1-0108 | 663.249 | OH | a2 | B14 | b2 | C05 | c1 | D01 | d1 | E02 |
| 2-1-dlE02-1-0109 | 663.269 | OH | a1 | B03 | b2 | C02 | c1 | D01 | d1 | E02 |
| 2-1-d1E02-1-0110 | 663.306 | OH | a2 | B12 | b2 | C07 | c1 | D01 | d1 | E02 |
| 2-1-d1E02-1-0111 | 664.192 | OH | a1 | B07 | b1 | C12 | c1 | D01 | d1 | E02 |
| 2-1-d1E02-1-0112 | 664.236 | OH | a2 | B15 | b2 | C06 | c1 | D01 | d1 | E02 |
| 2-1-d1E02-1-0113 | 664.265 | OH | a1 | B03 | b2 | C03 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0114 | 664.265 | OH | a1 | B01 | b2 | C05 | c1 | D01 | d1 | E02 |
| 2-1-d1E02-1-0115 | 665.260 | OH | a1 | B02 | b2 | C05 | c1 | D01 | d1 | E02 |
| 2-1-d1E02-1-0116 | 666.215 | OH | a2 | B16 | b2 | C06 | c1 | D01 | d1 | E02 |
| 2-1-d1E02-1-0117 | 666.215 | OH | a2 | B11 | b3 | C08 | c1 | D01 | d1 | E02 |
| 2-1-d1E02-1-0118 | 666.215 | OH | a2 | B11 | b1 | C08 | c2 | D02 | d1 | E02 |
| 2-1-d1E02-1-0119 | 666.229 | OH | a2 | B17 | b2 | C02 | c1 | D01 | d1 | E02 |
| 2-1-d1E02-1-0120 | 666.229 | OH | a2 | B14 | b2 | C04 | c1 | D01 | d1 | E02 |
| 2-1-d1E02-1-0121 | 667.210 | OH | a2 | B12 | b3 | C08 | c1 | D01 | d1 | E02 |
| 2-1-d1E02-1-0122 | 667.210 | OH | a2 | B12 | b1 | C08 | c2 | D02 | d1 | E02 |
| 2-1-d1E02-1-0123 | 667.210 | OH | a2 | B11 | b1 | C11 | c2 | D02 | d1 | E02 |
| 2-1-d1E02-1-0124 | 667.224 | OH | a2 | B17 | b2 | C03 | c1 | D01 | d1 | E02 |
| 2-1-d1E02-1-0125 | 667.244 | OH | a1 | B04 | b2 | C02 | c1 | D01 | d1 | E02 |
| 2-1-d1E02-1-0126 | 667.244 | OH | a1 | B01 | b2 | C04 | c1 | D01 | d1 | E02 |
| 2-1-d1E02-1-0127 | 667.264 | OH | a1 | B05 | b2 | C01 | c1 | D01 | d1 | E02 |
| 2-1-d1E02-1-0128 | 668.205 | OH | a2 | B11 | b1 | C10 | c2 | D02 | d1 | E02 |
| 2-1-d1E02-1-0129 | 668.205 | OH | a2 | B12 | b1 | C11 | c2 | D02 | d1 | E02 |
| 2-1-dlE02-1-0130 | 668.239 | OH | a1 | B02 | b2 | C04 | c1 | D01 | d1 | E02 |
| 2-1-d1E02-1-013l | 668.239 | OH | a1 | B04 | b2 | C03 | c1 | D01 | d1 | E02 |
| 2-1-d1E02-1-0132 | 668.275 | OH | a2 | B13 | b1 | C13 | c1 | D01 | d1 | E02 |
| 2-1-d1E02-1-0133 | 669.201 | OH | a2 | B12 | b1 | C10 | c2 | D02 | d1 | E02 |
| 2-1-d1E02-1-0134 | 669.226 | OH | a1 | B03 | b2 | C06 | c1 | D01 | d1 | E02 |
| 2-1-d1E02-1-0135 | 669.243 | OH | a1 | B06 | b2 | C01 | c1 | D01 | d1 | E02 |
| 2-1-d1E02-1-0136 | 670.210 | OH | a2 | B13 | b3 | C15 | c1 | DOI | d1 | E02 |
| 2-1-d1E02-1-0137 | 670.240 | OH | a2 | B19 | b1 | C08 | c1 | DOI | d1 | E02 |
| 2-1-d1E02-1-0138 | 670.240 | OH | a2 | B11 | b1 | C09 | c1 | DOI | d1 | E02 |
| 2-1-d1E02-1-0139 | 670.243 | OH | a2 | B18 | b2 | C01 | c1 | D01 | d1 | E02 |
| 2-1-d1E02-1-0140 | 671.236 | OH | a2 | B12 | b1 | C09 | c1 | DOI | d1 | E02 |
| 2-1-d1E02-1-0141 | 671.236 | OH | a2 | B19 | b1 | C11 | c1 | DOI | d1 | E02 |
| 2-1-d1E02-1-0142 | 672.171 | OH | a2 | B11 | b3 | C06 | c1 | DOI | d1 | E02 |
| 2-1-d1E02-1-0143 | 672.185 | OH | a2 | B17 | b2 | C06 | c1 | DOI | d1 | E02 |
| 2-1-d1E02-1-0144 | 672.231 | OH | a2 | B19 | b1 | C10 | c1 | DOI | d1 | E02 |
| 2-1-d1E02-1-0145 | 672.241 | OH | a2 | B13 | b1 | C14 | c1 | DOI | d1 | E02 |
| 2-1-d1E02-1-0146 | 672.250 | OH | a2 | B14 | b1 | C13 | c1 | D01 | d1 | E02 |
| 2-1-d1E02-1-0147 | 672.262 | OH | a2 | B13 | b2 | C33 | c3 | D02 | d1 | E02 |
| 2-1-d1E02-1-0148 | 673.166 | OH | a2 | B12 | b3 | C06 | c1 | D01 | d1 | E02 |
| 2-1-d1E02-1-0149 | 673.166 | OH | a2 | B11 | b1 | C12 | c2 | D02 | d1 | E02 |
| 2-1-d1E02-1-0150 | 673.201 | OH | a1 | B04 | b2 | C06 | c1 | D01 | d1 | E02 |
| 2-1-d1E02-1-0151 | 673.265 | OH | a1 | B01 | b1 | C13 | c1 | DOI | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0152 | 673.290 | OH | a2 | B15 | b2 | C05 | c1 | D01 | d1 | E02 |
| 2-1-d1E02-1-0153 | 674.162 | OH | a2 | B12 | b1 | C12 | c2 | D02 | d1 | E02 |
| 2-1-d1E02-1-0154 | 674.185 | OH | a2 | B14 | b3 | C15 | c1 | D01 | d1 | E02 |
| 2-1-d1E02-1-0155 | 674.260 | OH | a1 | B02 | b1 | C13 | c1 | DOI | d1 | E02 |
| 2-1-d1E02-1-0156 | 675.200 | OH | a1 | B01 | b3 | C15 | c1 | DOI | d1 | E02 |
| 2-1-d1E02-1-0157 | 675.214 | OH | a1 | B07 | b2 | C01 | c1 | D01 | d1 | E02 |
| 2-1-d1E02-1-0158 | 675.269 | OH | a2 | B16 | b2 | COS | c1 | D01 | d1 | E02 |
| 2-1-d1E02-1-0159 | 676.195 | OH | a1 | B02 | b3 | C15 | c1 | D01 | d1 | E02 |
| 2-1-d1E02-1-0160 | 676.216 | OH | a2 | B14 | b1 | C14 | c1 | D01 | d1 | E02 |
| 2-1-d1E02-1-0161 | 676.237 | OH | a2 | B14 | b2 | C33 | c3 | D02 | d1 | E02 |
| 2-1-d1E02-1-0162 | 676.270 | OH | a2 | B15 | b2 | C04 | c1 | D01 | d1 | E02 |
| 2-1-d1E02-1-0163 | 676.326 | OH | a2 | B13 | b2 | C07 | c1 | D01 | d1 | E02 |
| 2-1-d1E02-1-0164 | 677.192 | OH | a2 | B19 | b1 | C12 | c1 | D01 | d1 | E02 |
| 2-1-d1E02-1-0165 | 677.231 | OH | a1 | B01 | b1 | C14 | c1 | D01 | d1 | E02 |
| 2-1-d1E02-1-0166 | 677.252 | OH | a1 | B01 | b2 | C33 | c3 | D02 | d1 | E02 |
| 2-1-d1E02-1-0167 | 677.285 | OH | a1 | B05 | b2 | C02 | c1 | D01 | d1 | E02 |
| 2-1-d1E02-1-0168 | 678.226 | OH | a1 | B08 | b1 | C08 | c1 | D01 | d1 | E02 |
| 2-1-d1E02-1-0169 | 678.226 | OH | a1 | B02 | b1 | C14 | c1 | D01 | d1 | E02 |
| 2-1-d1E02-1-0170 | 678.247 | OH | a1 | B02 | b2 | C33 | c3 | D02 | d1 | E02 |
| 2-1-d1E02-1-0171 | 678.249 | OH | a2 | B16 | b2 | C04 | c1 | D01 | d1 | E02 |
| 2-1-d1E02-1-0172 | 678.251 | OH | a2 | B11 | b1 | C30 | c3 | D02 | d1 | E02 |
| 2-1-d1E02-1-0173 | 678.280 | OH | a1 | B05 | b2 | C03 | c1 | D01 | d1 | E02 |
| 2-1-d1E02-1-0174 | 678.280 | OH | a1 | B03 | b2 | C05 | c1 | D01 | d1 | E02 |
| 2-1-d1E02-1-0175 | 679.221 | OH | a1 | B08 | b1 | C11 | c1 | D01 | d1 | E02 |
| 2-1-d1E02-1-0176 | 679.246 | OH | a2 | B12 | b1 | C30 | c3 | D02 | d1 | E02 |
| 2-1-d1E02-1-0177 | 679.264 | OH | a1 | B06 | b2 | C02 | c1 | D01 | d1 | E02 |
| 2-1-d1E02-1-0178 | 680.217 | OH | a1 | B08 | b1 | C10 | c1 | D01 | d1 | E02 |
| 2-1-d1E02-1-0179 | 680.230 | OH | a2 | B13 | b3 | C08 | c1 | D01 | d1 | E02 |
| 2-1-d1E02-1-0180 | 680.230 | OH | a2 | B13 | b1 | C08 | c2 | D02 | d1 | E02 |
| 2-1-d1E02-1-0181 | 680.230 | OH | a2 | B11 | b2 | C19 | c3 | D02 | d1 | E02 |
| 2-1-d1E02-1-0182 | 680.259 | OH | a1 | B06 | b2 | C03 | c1 | D01 | d1 | E02 |
| 2-1-d1E02-1-0183 | 680.263 | OH | a2 | B18 | b2 | C02 | c1 | D01 | d1 | E02 |
| 2-1-d1E02-1-0184 | 680.301 | OH | a2 | B14 | b2 | C07 | c1 | D01 | d1 | E02 |
| 2-1-d1E02-1-0185 | 681.226 | OH | a2 | B13 | b1 | C11 | c2 | D02 | d1 | E02 |
| 2-1-d1E02-1-0186 | 681.226 | OH | a2 | B12 | b2 | C19 | c3 | D02 | d1 | E02 |
| 2-1-d1E02-1-0187 | 681.226 | OH | a2 | B11 | b2 | C27 | c3 | D02 | d1 | E02 |
| 2-1-d1E02-1-0188 | 681.240 | OH | a2 | B17 | b2 | COS | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0189 | 681.259 | OH | a2 | B18 | b2 | C03 | c1 | D01 | d1 | E02 |
| 2-1-d1E02-1-0190 | 681.26 | OH | a1 | B03 | b2 | C04 | c1 | D01 | d1 | E02 |
| 2-1-d1E02-1-0191 | 681.316 | OH | a1 | B01 | b2 | C07 | c1 | D01 | d1 | E02 |
| 2-1-d1E02-1-0192 | 682.221 | OH | a2 | B13 | b1 | C10 | c2 | D02 | d1 | E02 |
| 2-1-d1E02-1-0193 | 682.221 | OH | a2 | B12 | b2 | C27 | c3 | D02 | d1 | E02 |
| 2-1-d1E02-1-0194 | 682.255 | OH | a1 | B04 | b2 | C05 | c1 | D01 | d1 | E02 |
| 2-1-d1E02-1-0195 | 682.290 | OH | a2 | B15 | b1 | C13 | c1 | D01 | d1 | E02 |
| 2-1-d1E02-1-0196 | 682.311 | OH | a1 | B02 | b2 | C07 | c1 | D01 | d1 | E02 |
| 2-1-d1E02-1-0197 | 683.241 | OH | a1 | B05 | b2 | C06 | c1 | D01 | d1 | E02 |
| 2-1-d1E02-1-0198 | 684.189 | OH | a2 | B11 | b2 | C01 | c2 | D02 | d1 | E02 |
| 2-1-d1E02-1-0199 | 684.205 | OH | a2 | B14 | b3 | C08 | c1 | D01 | d1 | E02 |
| 2-1-d1E02-1-0200 | 684.205 | OH | a2 | B14 | b1 | C08 | c2 | D02 | d1 | E02 |
| 2-1-d1E02-1-0201 | 684.220 | OH | a2 | B17 | b2 | C04 | c1 | D01 | d1 | E02 |
| 2-1-d1E02-1-0202 | 684.225 | OH | a2 | B15 | b3 | C15 | c1 | D01 | d1 | E02 |
| 2-1-d1E02-1-0203 | 684.256 | OH | a2 | B13 | b1 | C09 | c1 | D01 | d1 | E02 |
| 2-1-d1E02-1-0204 | 684.270 | OH | a2 | B16 | b1 | C13 | c1 | D01 | d1 | E02 |
| 2-1-d1E02-1-0205 | 685.178 | OH | a1 | B08 | b1 | C12 | c1 | D01 | d1 | E02 |
| 2-1-d1E02-1-0206 | 685.184 | OH | a2 | B12 | b2 | C01 | c2 | D02 | d1 | E02 |
| 2-1-d1E02-1-0207 | 685.201 | OH | a2 | B14 | b1 | C11 | c2 | D02 | d1 | E02 |
| 2-1-d1E02-1-0208 | 685.221 | OH | a1 | B06 | b2 | C06 | c1 | D01 | d1 | E02 |
| 2-1-d1E02-1-0209 | 685.221 | OH | a1 | B01 | b3 | C08 | c1 | D01 | d1 | E02 |
| 2-1-d1E02-1-0210 | 685.221 | OH | a1 | B01 | b1 | C08 | c2 | D02 | d1 | E02 |
| 2-1-d1E02-1-0211 | 685.235 | OH | a1 | B07 | b2 | C02 | c1 | D01 | d1 | E02 |
| 2-1-d1E02-1-0212 | 685.235 | OH | a1 | B04 | b2 | C04 | c1 | D01 | d1 | E02 |
| 2-1-d1E02-1-0213 | 686.187 | OH | a2 | B13 | b3 | C06 | c1 | D01 | d1 | E02 |
| 2-1-d1E02-1-0214 | 686.196 | OH | a2 | B14 | b1 | C10 | c2 | D02 | d1 | E02 |
| 2-1-d1E02-1-0215 | 686.205 | OH | a2 | B16 | b3 | C15 | c1 | D01 | d1 | E02 |
| 2-1-d1E02-1-0216 | 686.216 | OH | a1 | B02 | b3 | C08 | c1 | D01 | d1 | E02 |
| 2-1-d1E02-1-0217 | 686.216 | OH | a1 | B02 | b1 | C08 | c2 | D02 | d1 | E02 |
| 2-1-d1E02-1-0218 | 686.216 | OH | a1 | B01 | b1 | C11 | c2 | D02 | d1 | E02 |
| 2-1-d1E02-1-0219 | 686.220 | OH | a2 | B18 | b2 | C06 | c1 | D01 | d1 | E02 |
| 2-1-d1E02-1-0220 | 686.230 | OH | a1 | B07 | b2 | C03 | c1 | D01 | d1 | E02 |
| 2-1-d1E02-1-0221 | 686.235 | OH | a2 | B20 | b1 | C08 | c1 | D01 | d1 | E02 |
| 2-1-d1E02-1-0222 | 686.256 | OH | a2 | B15 | b1 | C14 | c1 | D01 | d1 | E02 |
| 2-1-d1E02-1-0223 | 686.277 | OH | a2 | B15 | b2 | C33 | c3 | D02 | d1 | E02 |
| 2-1-d1E02-1-0224 | 687.182 | OH | a2 | B13 | b1 | C12 | c2 | D02 | d1 | E02 |
| 2-1-d1E02-1-0225 | 687.211 | OH | a1 | B01 | b1 | C10 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0226 | 687.211 | OH | a1 | B02 | b1 | C11 | c2 | D02 | d1 | E02 |
| 2-1-d1E02-1-0227 | 687.230 | OH | a2 | B20 | b1 | C11 | c1 | D01 | d1 | E02 |
| 2-1-d1E02-1-0228 | 687.281 | OH | a1 | B03 | b1 | C13 | c1 | D01 | d1 | E02 |
| 2-1-d1E02-1-0229 | 688.206 | OH | a1 | B02 | b1 | C10 | c2 | D02 | d1 | E02 |
| 2-1-d1E02-1-0230 | 688.214 | OH | a2 | B19 | b2 | C01 | c1 | D01 | d1 | E02 |
| 2-1-d1E02-1-0231 | 688.226 | OH | a2 | B20 | b1 | C10 | c1 | D01 | d1 | E02 |
| 2-1-d1E02-1-0232 | 688.231 | OH | a2 | B14 | b1 | C09 | c1 | D01 | d1 | E02 |
| 2-1-d1E02-1-0233 | 688.236 | OH | a2 | B16 | b1 | C14 | c1 | D01 | d1 | E02 |
| 2-1-d1E02-1-0234 | 688.257 | OH | a2 | B16 | b2 | C33 | c3 | D02 | d1 | E02 |
| 2-1-d1E02-1-0235 | 689.216 | OH | a1 | B03 | b3 | C15 | c1 | D01 | d1 | E02 |
| 2-1-d1E02-1-0236 | 689.246 | OH | a1 | B09 | b1 | C08 | c1 | D01 | d1 | E02 |
| 2-1-d1E02-1-0237 | 689.246 | OH | a1 | B01 | b1 | C09 | c1 | D01 | d1 | E02 |
| 2-1-d1E02-1-0238 | 690.162 | OH | a2 | B14 | b3 | C06 | c1 | D01 | d1 | E02 |
| 2-1-d1E02-1-0239 | 690.240 | OH | a2 | B17 | b1 | C13 | c1 | D01 | d1 | E02 |
| 2-1-d1E02-1-0240 | 690.241 | OH | a1 | B02 | b1 | C09 | c1 | D01 | d1 | E02 |
| 2-1-d1E02-1-0241 | 690.241 | OH | a1 | B09 | b1 | C11 | c1 | D01 | d1 | E02 |
| 2-1-d1E02-1-0242 | 690.342 | OH | a2 | B15 | b2 | C07 | c1 | D01 | d1 | E02 |
| 2-1-d1E02-1-0243 | 691.157 | OH | a2 | B14 | b1 | C12 | c2 | D02 | d1 | E02 |
| 2-1-d1E02-1-0244 | 691.177 | OH | a1 | B01 | b3 | C06 | c1 | D01 | d1 | E02 |
| 2-1-d1E02-1-0245 | 691.191 | OH | a1 | B07 | b2 | C06 | c1 | D01 | d1 | E02 |
| 2-1-d1E02-1-0246 | 691.237 | OH | a1 | B09 | b1 | C10 | c1 | D01 | d1 | E02 |
| 2-1-d1E02-1-0247 | 691.246 | OH | a1 | B03 | b1 | C14 | c1 | D01 | d1 | E02 |
| 2-1-d1E02-1-0248 | 691.255 | OH | a1 | B04 | b1 | C13 | c1 | D01 | d1 | E02 |
| 2-1-d1E02-1-0249 | 691.268 | OH | a1 | B03 | b2 | C33 | c3 | D02 | d1 | E02 |
| 2-1-d1E02-1-0250 | 692.172 | OH | a1 | B02 | b3 | C06 | c1 | D01 | d1 | E02 |
| 2-1-d1E02-1-0251 | 692.172 | OH | a1 | B01 | b1 | C12 | c2 | D02 | d1 | E02 |
| 2-1-d1E02-1-0252 | 692.175 | OH | a2 | B17 | b3 | C15 | c1 | D01 | d1 | E02 |
| 2-1-d1E02-1-0253 | 692.267 | OH | a2 | B13 | b1 | C30 | c3 | D02 | d1 | E02 |
| 2-1-d1E02-1-0254 | 692.267 | OH | a2 | B11 | b1 | C32 | c3 | D02 | d1 | E02 |
| 2-1-d1E02-1-0255 | 692.296 | OH | a1 | B05 | b2 | C05 | c1 | D01 | d1 | E02 |
| 2-1-d1E02-1-0256 | 692.321 | OH | a2 | B16 | b2 | C07 | c1 | D01 | d1 | E02 |
| 2-1-d1E02-1-0257 | 693.168 | OH | a1 | B02 | b1 | C12 | c2 | D02 | d1 | E02 |
| 2-1-d1E02-1-0258 | 693.187 | OH | a2 | B20 | b1 | C12 | c1 | D01 | d1 | E02 |
| 2-1-d1E02-1-0259 | 693.190 | OH | a1 | B04 | b3 | C15 | c1 | D01 | d1 | E02 |
| 2-1-d1E02-1-0260 | 693.262 | OH | a2 | B12 | b1 | C32 | c3 | D02 | d1 | E02 |
| 2-1-d1E02-1-0261 | 694.206 | OH | a2 | B17 | b1 | C14 | c1 | D01 | d1 | E02 |
| 2-1-d1E02-1-0262 | 694.210 | OH | a2 | B11 | b2 | C02 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0263 | 694.227 | OH | a2 | B17 | b2 | C33 | c3 | D02 | d1 | E02 |
| 2-1-d1E02-1-0264 | 694.246 | OH | a2 | B15 | b3 | C08 | c1 | D01 | d1 | E02 |
| 21-d1E02-1-0265 | 694.246 | OH | a2 | B15 | b1 | C08 | c2 | D02 | d1 | E02 |
| 2-1-d1E02-1-0266 | 694.246 | OH | a2 | B13 | b2 | C19 | c3 | D02 | d1 | E02 |
| 2-1-d1E02-1-0267 | 694.246 | OH | a2 | B11 | b2 | C21 | c3 | D02 | d1 | E02 |
| 2-1-d1E02-1-0268 | 694.275 | OH | a1 | B06 | b2 | C05 | c1 | D01 | d1 | E02 |
| 2-1-d1E02-1-0269 | 695.205 | OH | a2 | B12 | b2 | C02 | c2 | D02 | d1 | E02 |
| 2-1-d1E02-1-0270 | 695.205 | OH | a2 | B11 | b2 | C03 | c2 | D02 | d1 | E02 |
| 2-1-d1E02-1-0271 | 695.221 | OH | a1 | B04 | b1 | C14 | c1 | D01 | d1 | E02 |
| 2-1-d1E02-1-0272 | 695.241 | OH | a2 | B15 | b1 | C11 | c2 | D02 | d1 | E02 |
| 2-1-d1E02-1-0273 | 695.241 | OH | a2 | B12 | b2 | C21 | c3 | D02 | d1 | E02 |
| 2-1-d1E02-1-0274 | 695.241 | OH | a2 | B13 | b2 | C27 | c3 | D02 | d1 | E02 |
| 2-1-d1E02-1-0275 | 695.243 | OH | a1 | B04 | b2 | C33 | c3 | D02 | d1 | E02 |
| 2-1-d1E02-1-0276 | 695.274 | OH | a2 | B18 | b2 | C05 | c1 | D01 | d1 | E02 |
| 2-1-d1E02-1-0277 | 695.276 | OH | a1 | B05 | b2 | C04 | c1 | D01 | d1 | E02 |
| 2-1-d1E02-1-0278 | 695.332 | OH | a1 | B03 | b2 | C07 | c1 | D01 | d1 | E02 |
| 2-1-d1E02-1-0279 | 696.198 | OH | a1 | B09 | b1 | C12 | c1 | D01 | d1 | E02 |
| 2-1-d1E02-1-0280 | 696.200 | OH | a1 | B08 | b2 | C01 | c1 | D01 | d1 | E02 |
| 2-1-d1E02-1-0281 | 696.200 | OH | a2 | B12 | b2 | C03 | c2 | D02 | d1 | E02 |
| 2-1-d1E02-1-0282 | 696.225 | OH | a2 | B16 | b3 | C08 | c1 | D01 | d1 | E02 |
| 2-1-d1E02-1-0283 | 696.225 | OH | a2 | B16 | b1 | C08 | c2 | D02 | d1 | E02 |
| 2-1-d1E02-1-0284 | 696.237 | OH | a2 | B15 | b1 | C10 | c2 | D02 | d1 | E02 |
| 2-1-d1E02-1-0285 | 696.242 | OH | a2 | B14 | b1 | C30 | c3 | D02 | d1 | E02 |
| 2-1-d1E02-1-0286 | 697.221 | OH | a2 | B16 | b1 | C11 | c2 | D02 | d1 | E02 |
| 2-1-d1E02-1-0287 | 697.255 | OH | a1 | B06 | b2 | C04 | c1 | D01 | d1 | E02 |
| 2-1-d1E02-1-0288 | 697.257 | OH | a1 | B01 | b1 | C30 | c3 | D02 | d1 | E02 |
| 2-1-d1E02-1-0289 | 698.205 | OH | a2 | B13 | b2 | C01 | c2 | D02 | d1 | E02 |
| 2-1-d1E02-1-0290 | 698.216 | OH | a2 | B16 | b1 | C10 | c2 | D02 | d1 | E02 |
| 2-1-d1E02-1-0291 | 698.221 | OH | a2 | B14 | b2 | C19 | c3 | D02 | d1 | E02 |
| 2-1-d1E02-1-0292 | 698.221 | OH | a2 | B11 | b2 | C23 | c3 | D02 | d1 | E02 |
| 2-1-d1E02-1-0293 | 698.235 | OH | a2 | B19 | b2 | C02 | c1 | D01 | d1 | E02 |
| 2-1-d1E02-1-0294 | 698.252 | OH | a1 | B02 | b1 | C30 | c3 | D02 | d1 | E02 |
| 2-1-d1E02-1-0295 | 698.254 | OH | a2 | B18 | b2 | C04 | c1 | D01 | d1 | E02 |
| 2-1-d1E02-1-0296 | 698.272 | OH | a2 | B15 | b1 | C09 | c1 | D01 | d1 | E02 |
| 2-1-d1E02-1-0297 | 698.292 | OH | a2 | B17 | b2 | C07 | c1 | D01 | d1 | E02 |
| 2-1-d1E02-1-0298 | 699.216 | OH | a2 | B12 | b2 | C23 | c3 | D02 | d1 | E02 |
| 2-1-d1E02-1-0299 | 699.216 | OH | a2 | B14 | b2 | C27 | c3 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0300 | 699.230 | OH | a2 | B19 | b2 | C03 | c1 | D01 | d1 | E02 |
| 2-1-d1E02-1-0301 | 699.236 | OH | a1 | B03 | b3 | C08 | c1 | D01 | d1 | E02 |
| 2-1-d1E02-1-0302 | 699.236 | OH | a1 | B03 | b1 | C08 | c2 | D02 | d1 | E02 |
| 2-1-d1E02-1-0303 | 699.236 | OH | a1 | B01 | b2 | C19 | c3 | D02 | d1 | E02 |
| 2-1-d1E02-1-0304 | 699.307 | OH | a1 | B04 | b2 | C07 | c1 | D01 | d1 | E02 |
| 2-1-d1E02-1-0305 | 700.166 | OH | a2 | B11 | b2 | C06 | c2 | D02 | d1 | E02 |
| 2-1-d1E02-1-0306 | 700.203 | OH | a2 | B15 | b3 | C06 | c1 | D01 | d1 | E02 |
| 2-1-d1E02-1-0307 | 700.232 | OH | a1 | B03 | b1 | C11 | c2 | D02 | d1 | E02 |
| 2-1-d1E02-1-0308 | 700.232 | OH | a1 | B02 | b2 | C19 | c3 | D02 | d1 | E02 |
| 2-1-d1E02-1-0309 | 700.232 | OH | a1 | B01 | b2 | C27 | c3 | D02 | d1 | E02 |
| 2-1-d1E02-1-0310 | 700.246 | OH | a1 | B07 | b2 | C05 | c1 | D01 | d1 | E02 |
| 2-1-d1E02-1-0311 | 700.251 | OH | a2 | B16 | b1 | C09 | c1 | D01 | d1 | E02 |
| 2-1-d1E02-1-0312 | 701.161 | OH | a2 | B12 | b2 | C06 | c2 | D02 | d1 | E02 |
| 2-1-d1E02-1-0313 | 701.198 | OH | a2 | B15 | b1 | C12 | c2 | D02 | d1 | E02 |
| 2-1-d1E02-1-0314 | 701.227 | OH | a1 | B03 | b1 | C10 | c2 | D02 | d1 | E02 |
| 2-1-d1E02-1-0315 | 701.227 | OH | a1 | B02 | b2 | C27 | c3 | D02 | d1 | E02 |
| 2-1-d1E02-1-0316 | 701.296 | OH | a1 | B05 | b1 | C13 | c1 | D01 | d1 | E02 |
| 2-1-d1E02-1-0317 | 702.180 | OH | a2 | B14 | b2 | C01 | c2 | D02 | d1 | E02 |
| 2-1-d1E02-1-0318 | 702.182 | OH | a2 | B16 | b3 | C06 | c1 | D01 | d1 | E02 |
| 2-1-d1E02-1-0319 | 702.196 | OH | a2 | B17 | b3 | C08 | c1 | D01 | d1 | E02 |
| 2-1-d1E02-1-0320 | 702.196 | OH | a2 | B17 | b1 | C08 | c2 | D02 | d1 | E02 |
| 2-1-d1E02-1-0321 | 703.177 | OH | a2 | B16 | b1 | C12 | c2 | D02 | d1 | E02 |
| 2-1-d1E02-1-0322 | 703.191 | OH | a2 | B17 | b1 | C11 | c2 | D02 | d1 | E02 |
| 2-1-d1E02-1-0323 | 703.195 | OH | a1 | B01 | b2 | C01 | c2 | D02 | d1 | E02 |
| 2-1-d1E02-1-0324 | 703.211 | OH | a1 | B04 | b3 | C08 | c1 | D01 | d1 | E02 |
| 2-1-d1E02-1-0325 | 703.211 | OH | a1 | B04 | b1 | C08 | c2 | D02 | d1 | E02 |
| 2-1-d1E02-1-0326 | 703.225 | OH | a1 | B07 | b2 | C04 | c1 | D01 | d1 | E02 |
| 2-1-d1E02-1-0327 | 703.231 | OH | a1 | B05 | b3 | C15 | c1 | D01 | d1 | E02 |
| 2-1-d1E02-1-0328 | 703.262 | OH | a1 | B03 | b1 | C09 | c1 | D01 | d1 | E02 |
| 2-1-d1E02-1-0329 | 703.275 | OH | a1 | B06 | b1 | C13 | c1 | D01 | d1 | E02 |
| 2-1-d1E02-1-0330 | 704.186 | OH | a2 | B17 | b1 | C10 | c2 | D02 | d1 | E02 |
| 2-1-d1E02-1-0331 | 704.190 | OH | a1 | B02 | b2 | C01 | c2 | D02 | d1 | E02 |
| 2-1-d1E02-1-0332 | 704.192 | OH | a2 | B19 | b2 | C06 | c1 | D01 | d1 | E02 |
| 2-1-d1E02-1-0333 | 704.206 | OH | a1 | B04 | b1 | C11 | c2 | D02 | d1 | E02 |
| 2-1-d1E02-1-0334 | 704.209 | OH | a2 | B20 | b2 | C01 | c1 | D01 | d1 | E02 |
| 2-1-d1E02-1-0335 | 704.275 | OH | a2 | B18 | b1 | C13 | c1 | D01 | d1 | E02 |
| 2-1-d1E02-1-0336 | 705.193 | OH | a1 | B03 | b3 | C06 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0337 | 705.202 | OH | a1 | B04 | b1 | C10 | c2 | D02 | d1 | E02 |
| 2-1-d1E02-1-0338 | 705.210 | OH | a1 | B06 | b3 | C15 | c1 | D01 | d1 | E02 |
| 2-1-d1E02-1-0339 | 705.241 | OH | a1 | B10 | b1 | C08 | c1 | D01 | d1 | E02 |
| 2-1-d1E02-1-0340 | 705.262 | OH | a1 | B05 | b1 | C14 | c1 | D01 | d1 | E02 |
| 2-1-d1E02-1-0341 | 705.283 | OH | a1 | B05 | b2 | C33 | c3 | D02 | d1 | E02 |
| 2-1-d1E02-1-0342 | 706.188 | OH | a1 | B03 | b1 | C12 | c2 | D02 | d1 | E02 |
| 2-1-d1E02-1-0343 | 706.210 | OH | a2 | B18 | b3 | C15 | c1 | D01 | d1 | E02 |
| 2-1-d1E02-1-0344 | 706.221 | OH | a1 | B08 | b2 | C02 | c1 | D01 | d1 | E02 |
| 2-1-d1E02-1-0345 | 706.221 | OH | a2 | B17 | b1 | C09 | c1 | D01 | d1 | E02 |
| 2-1-d1E02-1-0346 | 706.236 | OH | a1 | B10 | b1 | C11 | c1 | D01 | d1 | E02 |
| 2-1-d1E02-1-0347 | 706.283 | OH | a2 | B15 | b1 | C30 | c3 | D02 | d1 | E02 |
| 2-1-d1E02-1-0348 | 706.283 | OH | a2 | B11 | b1 | C29 | c3 | D02 | d1 | E02 |
| 2-1-d1E02-1-0349 | 706.283 | OH | a2 | B13 | b1 | C32 | c3 | D02 | d1 | E02 |
| 2-1-d1E02-1-0350 | 707.216 | OH | a1 | B08 | b2 | C03 | c1 | D01 | d1 | E02 |
| 2-1-d1E02-1-0351 | 707.220 | OH | a1 | B09 | b2 | C01 | c1 | D01 | d1 | E02 |
| 2-1-d1E02-1-0352 | 707.232 | OH | a1 | B10 | b1 | C10 | c1 | D01 | d1 | E02 |
| 2-1-d1E02-1-0353 | 707.237 | OH | a1 | B04 | b1 | C09 | c1 | D01 | d1 | E02 |
| 2-1-d1E02-1-0354 | 707.241 | OH | a1 | B06 | b1 | C14 | c1 | D01 | d1 | E02 |
| 2-1-d1E02-1-0355 | 707.262 | OH | a1 | B06 | b2 | C33 | c3 | D02 | d1 | E02 |
| 2-1-d1E02-1-0356 | 707.278 | OH | a2 | B12 | b1 | C29 | c3 | D02 | d1 | E02 |
| 2-1-d1E02-1-0357 | 708.152 | OH | a2 | B17 | b3 | C06 | c1 | D01 | d1 | E02 |
| 2-1-d1E02-1-0358 | 708.225 | OH | a2 | B13 | b2 | C02 | c2 | D02 | d1 | E02 |
| 2-1-d1E02-1-0359 | 708.241 | OH | a2 | B18 | b1 | C14 | c1 | D01 | d1 | E02 |
| 2-1-d1E02-1-0360 | 708.262 | OH | a2 | B15 | b2 | C19 | c3 | D02 | d1 | E02 |
| 2-1-d1E02-1-0361 | 708.262 | OH | a2 | B13 | b2 | C21 | c3 | D02 | d1 | E02 |
| 2-1-d1E02-1-0362 | 708.262 | OH | a2 | B16 | b1 | C30 | c3 | D02 | d1 | E02 |
| 2-1-d1E02-1-0363 | 708.262 | OH | a2 | B18 | b2 | C33 | c3 | D02 | d1 | E02 |
| 2-1-d1E02-1-0364 | 709.148 | OH | a2 | B17 | b1 | C12 | c2 | D02 | d1 | E02 |
| 2-1-d1E02-1-0365 | 709.168 | OH | a1 | B04 | b3 | C06 | c1 | D01 | d1 | E02 |
| 2-1-d1E02-1-0366 | 709.221 | OH | a2 | B13 | b2 | C03 | c2 | D02 | d1 | E02 |
| 2-1-d1E02-1-0367 | 709.221 | OH | a2 | B11 | b2 | C05 | c2 | D02 | d1 | E02 |
| 2-1-d1E02-1-0368 | 709.246 | OH | a1 | B07 | b1 | C13 | c1 | D01 | d1 | E02 |
| 2-1-d1E02-1-0369 | 709.257 | OH | a2 | B15 | b2 | C27 | c3 | D02 | d1 | E02 |
| 2-1-d1E02-1-0370 | 709.348 | OH | a1 | B05 | b2 | C07 | c1 | D01 | d1 | E02 |
| 2-1-d1E02-1-0371 | 710.163 | OH | a1 | B04 | b1 | C12 | c2 | D02 | d1 | E02 |
| 2-1-d1E02-1-0372 | 710.216 | OH | a2 | B12 | b2 | C05 | c2 | D02 | d1 | E02 |
| 2-1-d1E02-1-0373 | 710.241 | OH | a2 | B16 | b2 | C19 | c3 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0374 | 710.241 | OH | a2 | B11 | b2 | C25 | c3 | D02 | d1 | E02 |
| 2-1-d1E02-1-0375 | 710.257 | OH | a2 | B14 | b1 | C32 | c3 | D02 | d1 | E02 |
| 2-1-d1E02-1-0376 | 711.181 | OH | a1 | B07 | b3 | C15 | c1 | D01 | d1 | E02 |
| 2-1-d1E02-1-0377 | 711.236 | OH | a2 | B12 | b2 | C25 | c3 | D02 | d1 | E02 |
| 2-1-d1E02-1-0378 | 711.236 | OH | a2 | B16 | b2 | C27 | c3 | D02 | d1 | E02 |
| 2-1-d1E02-1-0379 | 711.273 | OH | a1 | B03 | b1 | C30 | c3 | D02 | d1 | E02 |
| 2-1-d1E02-1-0380 | 711.273 | OH | a1 | B01 | b1 | C32 | c3 | D02 | d1 | E02 |
| 2-1-d1E02-1-0381 | 711.327 | OH | a1 | B06 | b2 | C07 | c1 | D01 | d1 | E02 |
| 2-1-d1E02-1-0382 | 712.177 | OH | a1 | B08 | b2 | C06 | c1 | D01 | d1 | E02 |
| 2-1-d1E02-1-0383 | 712.193 | OH | a1 | B10 | b1 | C12 | c1 | D01 | d1 | E02 |
| 2-1-d1E02-1-0384 | 712.200 | OH | a2 | B14 | b2 | C02 | c2 | D02 | d1 | E02 |
| 2-1-d1E02-1-0385 | 712.200 | OH | a2 | B11 | b2 | C04 | c2 | D02 | d1 | E02 |
| 2-1-d1E02-1-0386 | 712.22 | OH | a2 | B15 | b2 | C01 | c2 | D02 | d1 | E02 |
| 2-1-d1E02-1-0387 | 712.237 | OH | a2 | B14 | b2 | C21 | c3 | D02 | d1 | E02 |
| 2-1-d1E02-1-0388 | 712.237 | OH | a2 | B13 | b2 | C23 | c3 | D02 | d1 | E02 |
| 2-1-d1E02-1-0389 | 712.268 | OH | a1 | B02 | b1 | C32 | c3 | D02 | d1 | E02 |
| 2-1-d1E02-1-0390 | 712.326 | OH | a2 | B18 | b2 | C07 | c1 | D01 | d1 | E02 |
| 2-1-d1E02-1-0391 | 713.196 | OH | a2 | B12 | b2 | C04 | c2 | D02 | d1 | E02 |
| 2-1-d1E02-1-0392 | 713.196 | OH | a2 | B14 | b2 | C03 | c2 | D02 | d1 | E02 |
| 2-1-d1E02-1-0393 | 713.212 | OH | a1 | B07 | b1 | C14 | c1 | D01 | d1 | E02 |
| 2-1-d1E02-1-0394 | 713.216 | OH | a1 | B01 | b2 | C02 | c2 | D02 | d1 | E02 |
| 2-1-d1E02-1-0395 | 713.233 | OH | a1 | B07 | b2 | C33 | c3 | D02 | d1 | E02 |
| 2-1-d1E02-1-0396 | 713.246 | OH | a2 | B19 | b2 | C05 | c1 | D01 | d1 | E02 |
| 2-1-d1E02-1-0397 | 713.252 | OH | a1 | B05 | b3 | C08 | c1 | D01 | d1 | E02 |
| 2-1-d1E02-1-0398 | 713.252 | OH | a1 | B05 | b1 | C08 | c2 | D02 | d1 | E02 |
| 2-1-d1E02-1-0399 | 713.252 | OH | a1 | B03 | b2 | C19 | c3 | D02 | d1 | E02 |
| 2-1-d1E02-1-0400 | 713.252 | OH | a1 | B01 | b2 | C21 | c3 | D02 | d1 | E02 |
| 2-1-d1E02-1-0401 | 714.182 | OH | a2 | B13 | b2 | C06 | c2 | D02 | d1 | E02 |
| 2-1-d1E02-1-0402 | 714.200 | OH | a2 | B16 | b2 | C01 | c2 | D02 | d1 | E02 |
| 2-1-d1E02-1-0403 | 714.211 | OH | a1 | B02 | b2 | C02 | c2 | D02 | d1 | E02 |
| 2-1-d1E02-1-0404 | 714.211 | OH | a1 | B01 | b2 | C03 | c2 | D02 | d1 | E02 |
| 2-1-d1E02-1-0405 | 714.230 | OH | a2 | B20 | b2 | C02 | c1 | D01 | d1 | E02 |
| 2-1-d1E02-1-0406 | 714.232 | OH | a2 | B17 | b1 | C30 | c3 | D02 | d1 | E02 |
| 2-1-d1E02-1-0407 | 714.247 | OH | a1 | B05 | b1 | C11 | c2 | D02 | d1 | E02 |
| 2-1-d1E02-1-0408 | 714.247 | OH | a1 | B02 | b2 | C21 | c3 | D02 | d1 | E02 |
| 2-1-d1E02-1-0409 | 714.247 | OH | a1 | B03 | b2 | C27 | c3 | D02 | d1 | E02 |
| 2-1-d1E02-1-0410 | 715.206 | OH | a1 | B02 | b2 | C03 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0411 | 715.225 | OH | a2 | B20 | b2 | C03 | c1 | D01 | d1 | E02 |
| 2-1-d1E02-1-0412 | 715.231 | OH | a1 | B06 | b3 | C08 | c1 | D01 | d1 | E02 |
| 2-1-d1E02-1-0413 | 715.231 | OH | a1 | B06 | b1 | C08 | c2 | D02 | d1 | E02 |
| 2-1-d1E02-1-0414 | 715.242 | OH | a1 | B05 | b1 | C10 | c2 | D02 | d1 | E02 |
| 2-1-d1E02-1-0415 | 715.248 | OH | a1 | B04 | b1 | C30 | c3 | D02 | d1 | E02 |
| 2-1-d1E02-1-0416 | 716.212 | OH | a2 | B17 | b2 | C19 | c3 | D02 | d1 | E02 |
| 2-1-d1E02-1-0417 | 716.212 | OH | a2 | B14 | b2 | C23 | c3 | D02 | d1 | E02 |
| 2-1-d1E02-1-0418 | 716.226 | OH | a2 | B19 | b2 | C04 | c1 | D01 | d1 | E02 |
| 2-1-d1E02-1-0419 | 716.226 | OH | a1 | B06 | b1 | C11 | c2 | D02 | d1 | E02 |
| 2-1-d1E02-1-0420 | 716.230 | OH | a2 | B18 | b3 | C08 | c1 | D01 | d1 | E02 |
| 2-1-d1E02-1-0421 | 716.230 | OH | a2 | B18 | b1 | C08 | c2 | D02 | d1 | E02 |
| 2-1-d1E02-1-0422 | 717.207 | OH | a2 | B17 | b2 | C27 | c3 | D02 | d1 | E02 |
| 2-1-d1E02-1-0423 | 717.210 | OH | a1 | B03 | b2 | C01 | c2 | D02 | d1 | E02 |
| 2-1-d1E02-1-0424 | 717.222 | OH | a1 | B06 | b1 | C10 | c2 | D02 | d1 | E02 |
| 2-1-d1E02-1-0425 | 717.226 | OH | a2 | B18 | b1 | C11 | c2 | D02 | d1 | E02 |
| 2-1-d1E02-1-0426 | 717.227 | OH | a1 | B04 | b2 | C19 | c3 | D02 | d1 | E02 |
| 2-1-d1E02-1-0427 | 717.227 | OH | a1 | B01 | b2 | C23 | c3 | D02 | d1 | E02 |
| 2-1-d1E02-1-0428 | 717.241 | OH | a1 | B09 | b2 | C02 | c1 | D01 | d1 | E02 |
| 2-1-d1E02-1-0429 | 717.277 | OH | a1 | B05 | b1 | C09 | c1 | D01 | d1 | E02 |
| 2-1-d1E02-1-0430 | 717.297 | OH | a1 | B07 | b2 | C07 | c1 | D01 | d1 | E02 |
| 2-1-d1E02-1-0431 | 718.157 | OH | a2 | B14 | b2 | C06 | c2 | D02 | d1 | E02 |
| 2-1-d1E02-1-0432 | 718.221 | OH | a2 | B18 | b1 | C10 | c2 | D02 | d1 | E02 |
| 2-1-d1E02-1-0433 | 718.221 | OH | a2 | B11 | b1 | C13 | c2 | D02 | d1 | E02 |
| 2-1-d1E02-1-0434 | 718.222 | OH | a1 | B02 | b2 | C23 | c3 | D02 | d1 | E02 |
| 2-1-d1E02-1-0435 | 718.222 | OH | a1 | B04 | b2 | C27 | c3 | D02 | d1 | E02 |
| 2-1-d1E02-1-0436 | 718.236 | OH | a1 | B09 | b2 | C03 | c1 | D01 | d1 | E02 |
| 2-1-d1E02-1-0437 | 719.172 | OH | a1 | B01 | b2 | C06 | c2 | D02 | d1 | E02 |
| 2-1-d1E02-1-0438 | 719.208 | OH | a1 | B05 | b3 | C06 | c1 | D01 | d1 | E02 |
| 2-1-d1E02-1-0439 | 719.216 | OH | a2 | B12 | b1 | C13 | c2 | D02 | d1 | E02 |
| 2-1-d1E02-1-0440 | 719.257 | OH | a1 | B06 | b1 | C09 | c1 | D01 | d1 | E02 |
| 2-1-d1E02-1-0441 | 720.156 | OH | a2 | B11 | b3 | C15 | c2 | D02 | d1 | E02 |
| 2-1-d1E02-1-0442 | 720.167 | OH | a1 | B02 | b2 | C06 | c2 | D02 | d1 | E02 |
| 2-1-d1E02-1-0443 | 720.170 | OH | a2 | B17 | b2 | C01 | c2 | D02 | d1 | E02 |
| 2-1-d1E02-1-0444 | 720.187 | OH | a2 | B20 | b2 | C06 | c1 | D01 | d1 | E02 |
| 2-1-d1E02-1-0445 | 720.204 | OH | a1 | B05 | b1 | C12 | c2 | D02 | d1 | E02 |
| 2-1-d1E02-1-0446 | 720.225 | OH | a2 | B11 | b3 | C16 | c1 | D01 | d1 | E02 |
| 2-1-d1E02-1-0447 | 720.256 | OH | a2 | B18 | b1 | C09 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0448 | 720.298 | OH | a2 | B13 | b1 | C29 | c3 | D02 | d1 | E02 |
| 2-1-d1E02-1-0449 | 720.298 | OH | a2 | B15 | b1 | C32 | c3 | D02 | d1 | E02 |
| 2-1-d1E02-1-0450 | 721.151 | OH | a2 | B12 | b3 | C15 | c2 | D02 | d1 | E02 |
| 2-1-d1E02-1-0451 | 721.185 | OH | a1 | B04 | b2 | C01 | c2 | D02 | d1 | E02 |
| 2-1-d1E02-1-0452 | 721.188 | OH | a1 | B06 | b3 | C06 | c1 | D01 | d1 | E02 |
| 2-1-d1E02-1-0453 | 721.202 | OH | a1 | B07 | b3 | C08 | c1 | D01 | d1 | E02 |
| 2-1-d1E02-1-0454 | 721.202 | OH | a1 | B07 | b1 | C08 | c2 | D02 | d1 | E02 |
| 2-1-d1E02-1-0455 | 721.221 | OH | a2 | B12 | b3 | C16 | c1 | D01 | d1 | E02 |
| 2-1-d1E02-1-0456 | 721.232 | OH | a1 | B08 | b2 | C05 | c1 | D01 | d1 | E02 |
| 2-1-d1E02-1-0457 | 722.183 | OH | a1 | B06 | b1 | C12 | c2 | D02 | d1 | E02 |
| 2-1-d1E02-1-0458 | 722.187 | OH | a2 | B18 | b3 | C06 | c1 | D01 | d1 | E02 |
| 2-1-d1E02-1-0459 | 722.187 | OH | a2 | B11 | b3 | C17 | c1 | D01 | d1 | E02 |
| 2-1-d1E02-1-0460 | 722.187 | OH | a2 | B11 | b1 | C14 | c2 | D02 | d1 | E02 |
| 2-1-d1E02-1-0461 | 722.197 | OH | a1 | B07 | b1 | C11 | c2 | D02 | d1 | E02 |
| 2-1-d1E02-1-0462 | 722.241 | OH | a2 | B15 | b2 | C02 | c2 | D02 | d1 | E02 |
| 2-1-d1E02-1-0463 | 722.246 | OH | a2 | B19 | b1 | C13 | c1 | D01 | d1 | E02 |
| 2-1-d1E02-1-0464 | 722.277 | OH | a2 | B15 | b2 | C21 | c3 | D02 | d1 | E02 |
| 2-1-d1E02-1-0465 | 722.277 | OH | a2 | B16 | b1 | C32 | c3 | D02 | d1 | E02 |
| 2-1-d1E02-1-0466 | 723.182 | OH | a2 | B12 | b3 | C17 | c1 | D01 | d1 | E02 |
| 2-1-d1E02-1-0467 | 723.182 | OH | a2 | B18 | b1 | C12 | c2 | D02 | d1 | E02 |
| 2-1-d1E02-1-0468 | 723.182 | OH | a2 | B12 | b1 | C14 | c2 | D02 | d1 | E02 |
| 2-1-d1E02-1-0469 | 723.192 | OH | a1 | B07 | b1 | C10 | c2 | D02 | d1 | E02 |
| 2-1-d1E02-1-0470 | 723.197 | OH | a1 | B09 | b2 | C06 | c1 | D01 | d1 | E02 |
| 2-1-d1E02-1-0471 | 723.215 | OH | a1 | B10 | b2 | C01 | c1 | D01 | d1 | E02 |
| 2-1-d1E02-1-0472 | 723.236 | OH | a2 | B15 | b2 | C03 | c2 | D02 | d1 | E02 |
| 2-1-d1E02-1-0473 | 723.236 | OH | a2 | B13 | b2 | C05 | c2 | D02 | d1 | E02 |
| 2-1-d1E02-1-0474 | 724.181 | OH | a2 | B19 | b3 | C15 | c1 | D01 | d1 | E02 |
| 2-1-d1E02-1-0475 | 724.212 | OH | a1 | B08 | b2 | C04 | c1 | D01 | d1 | E02 |
| 2-1-d1E02-1-0476 | 724.220 | OH | a2 | B16 | b2 | C02 | c2 | D02 | d1 | E02 |
| 2-1-d1E02-1-0477 | 724.257 | OH | a2 | B16 | b2 | C21 | c3 | D02 | d1 | E02 |
| 2-1-d1E02-1-0478 | 724.257 | OH | a2 | B13 | b2 | C25 | c3 | D02 | d1 | E02 |
| 2-1-d1E02-1-0479 | 724.273 | OH | a2 | B14 | b1 | C29 | c3 | D02 | d1 | E02 |
| 2-1-d1E02-1-0480 | 725.216 | OH | a2 | B16 | b2 | C03 | c2 | D02 | d1 | E02 |
| 2-1-d1E02-1-0481 | 725.227 | OH | a1 | B07 | b1 | C09 | c1 | D01 | d1 | E02 |
| 2-1-d1E02-1-0482 | 725.288 | OH | a1 | B05 | b1 | C30 | c3 | D02 | d1 | E02 |
| 2-1-d1E02-1-0483 | 725.288 | OH | a1 | B01 | b1 | C29 | c3 | D02 | d1 | E02 |
| 2-1-d1E02-1-0484 | 725.288 | OH | a1 | B03 | b1 | C32 | c3 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0485 | 726.212 | OH | a2 | B19 | b1 | C14 | c1 | D01 | d1 | E02 |
| 2-1-d1E02-1-0486 | 726.216 | OH | a2 | B13 | b2 | C04 | c2 | D02 | d1 | E02 |
| 2-1-d1E02-1-0487 | 726.234 | OH | a2 | B19 | b2 | C33 | c3 | D02 | d1 | E02 |
| 2-1-d1E02-1-0488 | 726.252 | OH | a2 | B15 | b2 | C23 | c3 | D02 | d1 | E02 |
| 2-1-d1E02-1-0489 | 726.272 | OH | a2 | B11 | b2 | C07 | c2 | D02 | d1 | E02 |
| 2-1-d1E02-1-0490 | 726.284 | OH | a1 | B02 | b1 | C29 | c3 | D02 | d1 | E02 |
| 2-1-d1E02-1-0491 | 727.158 | OH | a1 | B07 | b3 | C06 | c1 | D01 | d1 | E02 |
| 2-1-d1E02-1-0492 | 727.211 | OH | a2 | B14 | b2 | C05 | c2 | D02 | d1 | E02 |
| 2-1-d1E02-1-0493 | 727.231 | OH | a1 | B03 | b2 | C02 | c2 | D02 | d1 | E02 |
| 2-1-d1E02-1-0494 | 727.268 | OH | a2 | B12 | b2 | C07 | c2 | D02 | d1 | E02 |
| 2-1-d1E02-1-0495 | 727.268 | OH | a1 | B05 | b2 | C19 | c3 | D02 | d1 | E02 |
| 2-1-d1E02-1-0496 | 727.268 | OH | a1 | B03 | b2 | C21 | c3 | D02 | d1 | E02 |
| 2-1-d1E02-1-0497 | 727.268 | OH | a1 | B06 | b1 | C30 | c3 | D02 | d1 | E02 |
| 2-1-d1E02-1-0498 | 728.153 | OH | a1 | B07 | b1 | C12 | c2 | D02 | d1 | E02 |
| 2-1-d1E02-1-0499 | 728.197 | OH | a2 | B15 | b2 | C06 | c2 | D02 | d1 | E02 |
| 2-1-d1E02-1-0500 | 728.226 | OH | a1 | B03 | b2 | C03 | c2 | D02 | d1 | E02 |
| 2-1-d1E02-1-0501 | 728.226 | OH | a1 | B01 | b2 | C05 | c2 | D02 | d1 | E02 |
| 2-1-d1E02-1-0502 | 728.232 | OH | a2 | B16 | b2 | C23 | c3 | D02 | d1 | E02 |
| 2-1-d1E02-1-0503 | 728.232 | OH | a2 | B14 | b2 | C25 | c3 | D02 | d1 | E02 |
| 2-1-d1E02-1-0504 | 728.248 | OH | a2 | B17 | b1 | C32 | c3 | D02 | d1 | E02 |
| 2-1-d1E02-1-0505 | 728.263 | OH | a1 | B05 | b2 | C27 | c3 | D02 | d1 | E02 |
| 2-1-d1E02-1-0506 | 728.267 | OH | a2 | B18 | b1 | C30 | c3 | D02 | d1 | E02 |
| 2-1-d1E02-1-0507 | 729.222 | OH | a1 | B02 | b2 | C05 | c2 | D02 | d1 | E02 |
| 2-1-d1E02-1-0508 | 729.241 | OH | a2 | B20 | b2 | C05 | c1 | D01 | d1 | E02 |
| 2-1-d1E02-1-0509 | 729.247 | OH | a1 | B06 | b2 | C19 | c3 | D02 | d1 | E02 |
| 2-1-d1E02-1-0510 | 729.247 | OH | a1 | B01 | b2 | C25 | c3 | D02 | d1 | E02 |
| 2-1-d1E02-1-0511 | 729.263 | OH | a1 | B04 | b1 | C32 | c3 | D02 | d1 | E02 |
| 2-1-d1E02-1-0512 | 730.177 | OH | a2 | B16 | b2 | C06 | c2 | D02 | d1 | E02 |
| 2-1-d1E02-1-0513 | 730.177 | OH | a2 | B11 | b3 | C08 | c2 | D02 | d1 | E02 |
| 2-1-d1E02-1-0514 | 730.191 | OH | a2 | B17 | b2 | C02 | c2 | D02 | d1 | E02 |
| 2-1-d1E02-1-0515 | 730.191 | OH | a2 | B14 | b2 | C04 | c2 | D02 | d1 | E02 |
| 2-1-d1E02-1-0516 | 730.227 | OH | a2 | B17 | b2 | C21 | c3 | D02 | d1 | E02 |
| 2-1-d1E02-1-0517 | 730.232 | OH | a1 | B08 | b1 | C13 | c1 | D01 | d1 | E02 |
| 2-1-d1E02-1-0518 | 730.242 | OH | a1 | B02 | b2 | C25 | c3 | D02 | d1 | E02 |
| 2-1-d1E02-1-0519 | 730.242 | OH | a1 | B06 | b2 | C27 | c3 | D02 | d1 | E02 |
| 2-1-d1E02-1-0520 | 730.246 | OH | a2 | B18 | b2 | C19 | c3 | D02 | d1 | E02 |
| 2-1-d1E02-1-0521 | 730.298 | OH | a2 | B19 | b2 | C07 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0522 | 731.172 | OH | a2 | B12 | b3 | C08 | c2 | D02 | d1 | E02 |
| 2-1-d1E02-1-0523 | 731.186 | OH | a2 | B17 | b2 | C03 | c2 | D02 | d1 | E02 |
| 2-1-d1E02-1-0524 | 731.206 | OH | a1 | B04 | b2 | C02 | c2 | D02 | d1 | E02 |
| 2-1-d1E02-1-0525 | 731.206 | OH | a1 | B01 | b2 | C04 | c2 | D02 | d1 | E02 |
| 2-1-d1E02-1-0526 | 731.226 | OH | a1 | B05 | b2 | C01 | c2 | D02 | d1 | E02 |
| 2-1-d1E02-1-0527 | 731.241 | OH | a2 | B18 | b2 | C27 | c3 | D02 | d1 | E02 |
| 2-1-d1E02-1-0528 | 731.243 | OH | a1 | B04 | b2 | C21 | c3 | D02 | d1 | E02 |
| 2-1-d1E02-1-0529 | 731.243 | OH | a1 | B03 | b2 | C23 | c3 | D02 | d1 | E02 |
| 2-1-d1E02-1-0530 | 732.167 | OH | a1 | B08 | b3 | C15 | c1 | D01 | d1 | E02 |
| 2-1-d1E02-1-0531 | 732.201 | OH | a1 | B02 | b2 | C04 | c2 | D02 | d1 | E02 |
| 2-1-d1E02-1-0532 | 732.201 | OH | a1 | B04 | b2 | C03 | c2 | D02 | d1 | E02 |
| 2-1-d1E02-1-0533 | 732.221 | OH | a2 | B20 | b2 | C04 | c1 | D01 | d1 | E02 |
| 2-1-d1E02-1-0534 | 732.237 | OH | a2 | B13 | b1 | C13 | c2 | D02 | d1 | E02 |
| 2-1-d1E02-1-0535 | 732.252 | OH | a1 | B09 | b2 | C05 | c1 | D01 | d1 | E02 |
| 2-1-d1E02-1-0536 | 733.188 | OH | a1 | B03 | b2 | C06 | c2 | D02 | d1 | E02 |
| 2-1-d1E02-1-0537 | 733.205 | OH | a1 | B06 | b2 | C01 | c2 | D02 | d1 | E02 |
| 2-1-d1E02-1-0538 | 733.236 | OH | a1 | B10 | b2 | C02 | c1 | D01 | d1 | E02 |
| 2-1-d1E02-1-0539 | 733.238 | OH | a1 | B07 | b1 | C30 | c3 | D02 | d1 | E02 |
| 2-1-d1E02-1-0540 | 734.172 | OH | a2 | B13 | b3 | C15 | c2 | D02 | d1 | E02 |
| 2-1-d1E02-1-0541 | 734.198 | OH | a1 | B08 | b1 | C14 | c1 | D01 | d1 | E02 |
| 2-1-d1E02-1-0542 | 734.202 | OH | a2 | B19 | b3 | C08 | c1 | D01 | d1 | E02 |
| 2-1-d1E02-1-0543 | 734.202 | OH | a2 | B19 | b1 | C08 | c2 | D02 | d1 | E02 |
| 2-1-d1E02-1-0544 | 734.202 | OH | a2 | B11 | b1 | C09 | c2 | D02 | d1 | E02 |
| 2-1-d1E02-1-0545 | 734.202 | OH | a2 | B17 | b2 | C23 | c3 | D02 | d1 | E02 |
| 2-1-d1E02-1-0546 | 734.205 | OH | a2 | B18 | b2 | C01 | c2 | D02 | d1 | E02 |
| 2-1-d1E02-1-0547 | 734.219 | OH | a1 | B08 | b2 | C33 | c3 | D02 | d1 | E02 |
| 2-1-d1E02-1-0548 | 734.231 | OH | a1 | B10 | b2 | C03 | c1 | D01 | d1 | E02 |
| 2-1-d1E02-1-0549 | 734.241 | OH | a2 | B13 | b3 | C16 | c1 | D01 | d1 | E02 |
| 2-1-d1E02-1-0550 | 734.277 | OH | a2 | B11 | b1 | C31 | c3 | D02 | d1 | E02 |
| 2-1-d1E02-1-0551 | 734.314 | OH | a2 | B15 | b1 | C29 | c3 | D02 | d1 | E02 |
| 2-1-d1E02-1-0552 | 735.197 | OH | a2 | B12 | b1 | C09 | c2 | D02 | d1 | E02 |
| 2-1-d1E02-1-0553 | 735.197 | OH | a2 | B19 | b1 | C11 | c2 | D02 | d1 | E02 |
| 2-1-d1E02-1-0554 | 735.217 | OH | a1 | B07 | b2 | C19 | c3 | D02 | d1 | E02 |
| 2-1-d1E02-1-0555 | 735.217 | OH | a1 | B04 | b2 | C23 | c3 | D02 | d1 | E02 |
| 2-1-d1E02-1-0556 | 735.232 | OH | a1 | B09 | b2 | C04 | c1 | D01 | d1 | E02 |
| 2-1-d1E02-1-0557 | 735.273 | OH | a2 | B12 | b1 | C31 | c3 | D02 | d1 | E02 |
| 2-1-d1E02-1-0558 | 736.133 | OH | a2 | B11 | b3 | C06 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0559 | 736.147 | OH | a2 | B17 | b2 | C06 | c2 | D02 | d1 | E02 |
| 2-1-d1E02-1-0560 | 736.193 | OH | a2 | B19 | b1 | C10 | c2 | D02 | d1 | E02 |
| 2-1-d1E02-1-0561 | 736.203 | OH | a2 | B13 | b3 | C17 | c1 | D01 | d1 | E02 |
| 2-1-d1E02-1-0562 | 736.203 | OH | a2 | B13 | b1 | C14 | c2 | D02 | d1 | E02 |
| 2-1-d1E02-1-0563 | 736.212 | OH | a2 | B14 | b1 | C13 | c2 | D02 | d1 | E02 |
| 2-1-d1E02-1-0564 | 736.213 | OH | a1 | B07 | b2 | C27 | c3 | D02 | d1 | E02 |
| 2-1-d1E02-1-0565 | 736.293 | OH | a2 | B16 | b1 | C29 | c3 | D02 | d1 | E02 |
| 2-1-d1E02-1-0566 | 737.128 | OH | a2 | B12 | b3 | C06 | c2 | D02 | d1 | E02 |
| 2-1-d1E02-1-0567 | 737.163 | OH | a1 | B04 | b2 | C06 | c2 | D02 | d1 | E02 |
| 2-1-d1E02-1-0568 | 737.198 | OH | a2 | B11 | b3 | C18 | c1 | D01 | d1 | E02 |
| 2-1-d1E02-1-0569 | 737.227 | OH | a1 | B01 | b1 | C13 | c2 | D02 | d1 | E02 |
| 2-1-d1E02-1-0570 | 737.252 | OH | a2 | B15 | b2 | C05 | c2 | D02 | d1 | E02 |
| 2-1-d1E02-1-0571 | 738.147 | OH | a2 | B14 | b3 | C15 | c2 | D02 | d1 | E02 |
| 2-1-d1E02-1-0572 | 738.193 | OH | a2 | B12 | b3 | C18 | c1 | D01 | d1 | E02 |
| 2-1-d1E02-1-0573 | 738.216 | OH | a2 | B14 | b3 | C16 | c1 | D01 | d1 | E02 |
| 2-1-d1E02-1-0574 | 738.222 | OH | a1 | B02 | b1 | C13 | c2 | D02 | d1 | E02 |
| 2-1-d1E02-1-0575 | 738.228 | OH | a2 | B19 | b1 | C09 | c1 | D01 | d1 | E02 |
| 2-1-d1E02-1-0576 | 738.241 | OH | a2 | B20 | b1 | C13 | c1 | D01 | d1 | E02 |
| 2-1-d1E02-1-0577 | 738.272 | OH | a2 | B15 | b2 | C25 | c3 | D02 | d1 | E02 |
| 2-1-d1E02-1-0578 | 738.284 | OH | a1 | B08 | b2 | C07 | c1 | D01 | d1 | E02 |
| 2-1-d1E02-1-0579 | 739.162 | OH | a1 | B01 | b3 | C15 | c2 | D02 | d1 | E02 |
| 2-1-d1E02-1-0580 | 739.176 | OH | a1 | B07 | b2 | C01 | c2 | D02 | d1 | E02 |
| 2-1-d1E02-1-0581 | 739.192 | OH | a1 | B10 | b2 | C06 | c1 | D01 | d1 | E02 |
| 2-1-d1E02-1-0582 | 739.231 | OH | a1 | B01 | b3 | C16 | c1 | D01 | d1 | E02 |
| 2-1-d1E02-1-0583 | 739.231 | OH | a2 | B16 | b2 | C05 | c2 | D02 | d1 | E02 |
| 2-1-d1E02-1-0584 | 739.304 | OH | a1 | B03 | b1 | C29 | c3 | D02 | d1 | E02 |
| 2-1-d1E02-1-0585 | 739.304 | OH | a1 | B05 | b1 | C32 | c3 | D02 | d1 | E02 |
| 2-1-d1E02-1-0586 | 740.157 | OH | a1 | B02 | b3 | C15 | c2 | D02 | d1 | E02 |
| 2-1-d1E02-1-0587 | 740.159 | OH | a2 | B19 | b3 | C06 | c1 | D01 | d1 | E02 |
| 2-1-d1E02-1-0588 | 740.176 | OH | a2 | B20 | b3 | C15 | c1 | D01 | d1 | E02 |
| 2-1-d1E02-1-0589 | 740.177 | OH | a2 | B14 | b3 | C17 | c1 | D01 | d1 | E02 |
| 2-1-d1E02-1-0590 | 740.177 | OH | a2 | B14 | b1 | C14 | c2 | D02 | d1 | E02 |
| 2-1-d1E02-1-0591 | 740.226 | OH | a1 | B02 | b3 | C16 | c1 | D01 | d1 | E02 |
| 2-1-d1E02-1-0592 | 740.232 | OH | a2 | B15 | b2 | C04 | c2 | D02 | d1 | E02 |
| 2-1-d1E02-1-0593 | 740.252 | OH | a2 | B16 | b2 | C25 | c3 | D02 | d1 | E02 |
| 2-1-d1E02-1-0594 | 740.288 | OH | a2 | B13 | b2 | C07 | c2 | D02 | d1 | E02 |
| 2-1-d1E02-1-0595 | 741.154 | OH | a2 | B19 | b1 | C12 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0596 | 741.193 | OH | a1 | B01 | b3 | C17 | c1 | D01 | d1 | E02 |
| 2-1-d1E02-1-0597 | 741.193 | OH | a1 | B01 | b1 | C14 | c2 | D02 | d1 | E02 |
| 2-1-d1E02-1-0598 | 741.247 | OH | a1 | B05 | b2 | C02 | c2 | D02 | d1 | E02 |
| 2-1-d1E02-1-0599 | 741.252 | OH | a1 | B09 | b1 | C13 | c1 | D01 | d1 | E02 |
| 2-1-d1E02-1-0600 | 741.283 | OH | a1 | B05 | b2 | C21 | c3 | D02 | d1 | E02 |
| 2-1-d1E02-1-0601 | 741.283 | OH | a1 | B06 | b1 | C32 | c3 | D02 | d1 | E02 |
| 2-1-d1E02-1-0602 | 742.188 | OH | a1 | B02 | b3 | C17 | c1 | D01 | d1 | E02 |
| 2-1-d1E02-1-0603 | 742.188 | OH | a1 | B08 | b3 | C08 | c1 | D01 | d1 | E02 |
| 2-1-d1E02-1-0604 | 742.188 | OH | a1 | B08 | b1 | C08 | c2 | D02 | d1 | E02 |
| 2-1-d1E02-1-0605 | 742.188 | OH | a1 | B02 | b1 | C14 | c2 | D02 | d1 | E02 |
| 2-1-d1E02-1-0606 | 742.207 | OH | a2 | B20 | b1 | C14 | c1 | D01 | d1 | E02 |
| 2-1-d1E02-1-0607 | 742.211 | OH | a2 | B16 | b2 | C04 | c2 | D02 | d1 | E02 |
| 2-1-d1E02-1-0608 | 742.228 | OH | a2 | B20 | b2 | C33 | c3 | D02 | d1 | E02 |
| 2-1-d1E02-1-0609 | 742.242 | OH | a1 | B05 | b2 | C03 | c2 | D02 | d1 | E02 |
| 2-1-d1E02-1-0610 | 742.242 | OH | a1 | B03 | b2 | C05 | c2 | D02 | d1 | E02 |
| 2-1-d1E02-1-0611 | 742.264 | OH | a2 | B17 | b1 | C29 | c3 | D02 | d1 | E02 |
| 2-1-d1E02-1-0612 | 742.283 | OH | a2 | B18 | b1 | C32 | c3 | D02 | d1 | E02 |
| 2-1-d1E02-1-0613 | 743.183 | OH | a1 | B08 | b1 | C11 | c2 | D02 | d1 | E02 |
| 2-1-d1E02-1-0614 | 743.187 | OH | a1 | B09 | b3 | C15 | c1 | D01 | d1 | E02 |
| 2-1-d1E02-1-0615 | 743.226 | OH | a1 | B06 | b2 | C02 | c2 | D02 | d1 | E02 |
| 2-1-d1E02-1-0616 | 743.262 | OH | a1 | B06 | b2 | C21 | c3 | D02 | d1 | E02 |
| 2-1-d1E02-1-0617 | 743.262 | OH | a1 | B03 | b2 | C25 | c3 | D02 | d1 | E02 |
| 2-1-d1E02-1-0618 | 743.279 | OH | a1 | B04 | b1 | C29 | c3 | D02 | d1 | E02 |
| 2-1-d1E02-1-0619 | 744.178 | OH | a1 | B08 | b1 | C10 | c2 | D02 | d1 | E02 |
| 2-1-d1E02-1-0620 | 744.192 | OH | a2 | B13 | b3 | C08 | c2 | D02 | d1 | E02 |
| 2-1-d1E02-1-0621 | 744.221 | OH | a1 | B06 | b2 | C03 | c2 | D02 | d1 | E02 |
| 2-1-d1E02-1-0622 | 744.225 | OH | a2 | B18 | b2 | C02 | c2 | D02 | d1 | E02 |
| 2-1-d1E02-1-0623 | 744.262 | OH | a2 | B18 | b2 | C21 | c3 | D02 | d1 | E02 |
| 2-1-d1E02-1-0624 | 744.263 | OH | a2 | B14 | b2 | C07 | c2 | D02 | d1 | E02 |
| 2-1-d1E02-1-0625 | 745.202 | OH | a2 | B17 | b2 | C05 | c2 | D02 | d1 | E02 |
| 2-1-d1E02-1-0626 | 745.218 | OH | a1 | B09 | b1 | C14 | c1 | D01 | d1 | E02 |
| 2-1-d1E02-1-0627 | 745.221 | OH | a2 | B18 | b2 | C03 | c2 | D02 | d1 | E02 |
| 2-1-d1E02-1-0628 | 745.222 | OH | a1 | B03 | b2 | C04 | c2 | D02 | d1 | E02 |
| 2-1-d1E02-1-0629 | 745.239 | OH | a1 | B09 | b2 | C33 | c3 | D02 | d1 | E02 |
| 2-1-d1E02-1-0630 | 745.258 | OH | a1 | B05 | b2 | C23 | c3 | D02 | d1 | E02 |
| 2-1-d1E02-1-0631 | 745.278 | OH | a1 | B01 | b2 | C07 | c2 | D02 | d1 | E02 |
| 2-1-d1E02-1-0632 | 746.213 | OH | a1 | B08 | b1 | C09 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0633 | 746.217 | OH | a1 | B04 | b2 | C05 | c2 | D02 | d1 | E02 |
| 2-1-d1E02-1-0634 | 746.222 | OH | a2 | B17 | b2 | C25 | c3 | D02 | d1 | E02 |
| 2-1-d1E02-1-0635 | 746.239 | OH | a2 | B19 | b1 | C30 | c3 | D02 | d1 | E02 |
| 2-1-d1E02-1-0636 | 746.252 | OH | a2 | B15 | b1 | C13 | c2 | D02 | d1 | E02 |
| 2-1-d1E02-1-0637 | 746.273 | OH | a1 | B02 | b2 | C07 | c2 | D02 | d1 | E02 |
| 2-1-d1E02-1-0638 | 746.293 | OH | a2 | B20 | b2 | C07 | c1 | D01 | d1 | E02 |
| 2-1-d1E02-1-0639 | 747.203 | OH | a1 | B05 | b2 | C06 | c2 | D02 | d1 | E02 |
| 2-1-d1E02-1-0640 | 747.237 | OH | a1 | B06 | b2 | C23 | c3 | D02 | d1 | E02 |
| 2-1-d1E02-1-0641 | 747.237 | OH | a1 | B04 | b2 | C25 | c3 | D02 | d1 | E02 |
| 2-1-d1E02-1-0642 | 747.254 | OH | a1 | B07 | b1 | C32 | c3 | D02 | d1 | E02 |
| 2-1-d1E02-1-0643 | 748.144 | OH | a1 | B08 | b3 | C06 | c1 | D01 | d1 | E02 |
| 2-1-d1E02-1-0644 | 748.167 | OH | a2 | B14 | b3 | C08 | c2 | D02 | d1 | E02 |
| 2-1-dIE02-1-0645 | 748.181 | OH | a2 | B17 | b2 | C04 | c2 | D02 | d1 | E02 |
| 2-1-d1E02-1-0646 | 748.187 | OH | a2 | B15 | b3 | C15 | c2 | D02 | d1 | E02 |
| 2-1-d1E02-1-0647 | 748.218 | OH | a2 | B13 | b1 | C09 | c2 | D02 | d1 | E02 |
| 2-1-d1E02-1-0648 | 748.218 | OH | a2 | B19 | b2 | C19 | c3 | D02 | d1 | E02 |
| 2-1-d1E02-1-0649 | 748.232 | OH | a2 | B16 | b1 | C13 | c2 | D02 | d1 | E02 |
| 2-1-d1E02-1-0650 | 748.237 | OH | a2 | B18 | b2 | C23 | c3 | D02 | d1 | E02 |
| 2-1-d1E02-1-0651 | 748.247 | OH | a1 | B10 | b2 | C05 | c1 | D01 | d1 | E02 |
| 2-1-d1E02-1-0652 | 748.257 | OH | a2 | B15 | b3 | C16 | c1 | D01 | d1 | E02 |
| 2-1-d1E02-1-0653 | 748.293 | OH | a2 | B13 | b1 | C31 | c3 | D02 | d1 | E02 |
| 2-1-dIE02-1-0654 | 749.14 | OH | a1 | B08 | b1 | C12 | c2 | D02 | d1 | E02 |
| 2-1-d1E02-1-0655 | 749.183 | OH | a1 | B06 | b2 | C06 | c2 | D02 | d1 | E02 |
| 2-1-d1E02-1-0656 | 749.183 | OH | a1 | B01 | b3 | C08 | c2 | D02 | d1 | E02 |
| 2-1-d1E02-1-0657 | 749.197 | OH | a1 | B07 | b2 | C02 | c2 | D02 | d1 | E02 |
| 2-1-d1E02-1-0658 | 749.197 | OH | a1 | B04 | b2 | C04 | c2 | D02 | d1 | E02 |
| 2-1-d1E02-1-0659 | 749.213 | OH | a2 | B19 | b2 | C27 | c3 | D02 | d1 | E02 |
| 2-1-d1E02-1-0660 | 749.233 | OH | a1 | B07 | b2 | C21 | c3 | D02 | d1 | E02 |
| 2-1-d1E02-1-0661 | 749.304 | OH | a1 | B09 | b2 | C07 | c1 | D01 | d1 | E02 |
| 2-1-d1E02-1-0662 | 750.149 | OH | a2 | B13 | b3 | C06 | c2 | D02 | d1 | E02 |
| 2-1-d1E02-1-0663 | 750.167 | OH | a2 | B16 | b3 | C15 | c2 | D02 | d1 | E02 |
| 2-1-d1E02-1-0664 | 750.178 | OH | a1 | B02 | b3 | C08 | c2 | D02 | d1 | E02 |
| 2-1-d1E02-1-0665 | 750.182 | OH | a2 | B18 | b2 | C06 | c2 | D02 | d1 | E02 |
| 2-1-d1E02-1-0666 | 750.192 | OH | a1 | B07 | b2 | C03 | c2 | D02 | d1 | E02 |
| 2-1-d1E02-1-0667 | 750.197 | OH | a2 | B20 | b3 | C08 | c1 | D01 | d1 | E02 |
| 2-1-d1E02-1-0668 | 750.197 | OH | a2 | B20 | b1 | C08 | c2 | D02 | d1 | E02 |
| 2-1-d1E02-1-0669 | 750.218 | OH | a2 | B15 | b3 | C17 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0670 | 750.218 | OH | a2 | B15 | b1 | C14 | c2 | D02 | d1 | E02 |
| 2-1-d1E02-1-0671 | 750.236 | OH | a2 | B16 | b3 | C16 | c1 | D01 | d1 | E02 |
| 2-1-d1E02-1-0672 | 751.192 | OH | a2 | B20 | b1 | C11 | c2 | D02 | d1 | E02 |
| 2-1-d1E02-1-0673 | 751.213 | OH | a2 | B13 | b3 | C18 | c1 | D01 | d1 | E02 |
| 2-1-d1E02-1-0674 | 751.227 | OH | a1 | B10 | b2 | C04 | c1 | D01 | d1 | E02 |
| 2-1-d1E02-1-0675 | 751.242 | OH | a1 | B03 | b1 | C13 | c2 | D02 | d1 | E02 |
| 2-1-d1E02-1-0676 | 752.176 | OH | a2 | B19 | b2 | C01 | c2 | D02 | d1 | E02 |
| 2-1-d1E02-1-0677 | 752.188 | OH | a2 | B20 | b1 | C10 | c2 | D02 | d1 | E02 |
| 2-1-d1E02-1-0678 | 752.193 | OH | a2 | B14 | b1 | C09 | c2 | D02 | d1 | E02 |
| 2-1-d1E02-1-0679 | 752.197 | OH | a2 | B16 | b3 | C17 | c1 | D01 | d1 | E02 |
| 2-1-d1E02-1-0680 | 752.197 | OH | a2 | B16 | b1 | C14 | c2 | D02 | d1 | E02 |
| 2-1-d1E02-1-0681 | 752.268 | OH | a2 | B14 | b1 | C31 | c3 | D02 | d1 | E02 |
| 2-1-d1E02-1-0682 | 753.177 | OH | a1 | B03 | b3 | C15 | c2 | D02 | d1 | E02 |
| 2-1-d1E02-1-0683 | 753.208 | OH | a1 | B09 | b3 | C08 | c1 | D01 | d1 | E02 |
| 2-1-d1E02-1-0684 | 753.208 | OH | a1 | B09 | b1 | C08 | c2 | D02 | d1 | E02 |
| 2-1-d1E02-1-0685 | 753.208 | OH | a1 | B01 | b1 | C09 | c2 | D02 | d1 | E02 |
| 2-1-d1E02-1-0686 | 753.208 | OH | a1 | B07 | b2 | C23 | c3 | D02 | d1 | E02 |
| 2-1-d1E02-1-0687 | 753.247 | OH | a1 | B03 | b3 | C16 | c1 | D01 | d1 | E02 |
| 2-1-d1E02-1-0688 | 753.283 | OH | a1 | B01 | b1 | C31 | c3 | D02 | d1 | E02 |
| 2-1-d1E02-1-0689 | 753.320 | OH | a1 | B05 | b1 | C29 | c3 | D02 | d1 | E02 |
| 2-1-d1E02-1-0690 | 754.124 | OH | a2 | B14 | b3 | C06 | c2 | D02 | d1 | E02 |
| 2-1-d1E02-1-0691 | 754.202 | OH | a2 | B17 | b1 | C13 | c2 | D02 | d1 | E02 |
| 2-1-d1E02-1-0692 | 754.203 | OH | a1 | B02 | b1 | C09 | c2 | D02 | d1 | E02 |
| 2-1-d1E02-1-0693 | 754.203 | OH | a1 | B09 | b1 | C11 | c2 | D02 | d1 | E02 |
| 2-1-d1E02-1-0694 | 754.223 | OH | a2 | B20 | b1 | C09 | c1 | D01 | d1 | E02 |
| 2-1-d1E02-1-0695 | 754.224 | OH | a1 | B08 | b1 | C30 | c3 | D02 | d1 | E02 |
| 2-1-d1E02-1-0696 | 754.278 | OH | a1 | B02 | b1 | C31 | c3 | D02 | d1 | E02 |
| 2-1-d1E02-1-0697 | 754.304 | OH | a2 | B15 | b2 | C07 | c2 | D02 | d1 | E02 |
| 2-1-d1E02-1-0698 | 755.139 | OH | a1 | B01 | b3 | C06 | c2 | D02 | d1 | E02 |
| 2-1-d1E02-1-0699 | 755.153 | OH | a1 | B07 | b2 | C06 | c2 | D02 | d1 | E02 |
| 2-1-d1E02-1-0700 | 755.188 | OH | a2 | B14 | b3 | C18 | c1 | D01 | d1 | E02 |
| 2-1-d1E02-1-0701 | 755.199 | OH | a1 | B09 | b1 | C10 | c2 | D02 | d1 | E02 |
| 2-1-d1E02-1-0702 | 755.208 | OH | a1 | B03 | b3 | C17 | c1 | D01 | d1 | E02 |
| 2-1-d1E02-1-0703 | 755.208 | OH | a1 | B03 | b1 | C14 | c2 | D02 | d1 | E02 |
| 2-1-d1E02-1-0704 | 755.217 | OH | a1 | B04 | b1 | C13 | c2 | D02 | d1 | E02 |
| 2-1-d1E02-1-0705 | 755.299 | OH | a1 | B06 | b1 | C29 | c3 | D02 | d1 | E02 |
| 2-1-d1E02-1-0706 | 756.134 | OH | a1 | B02 | b3 | C06 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0707 | 756.137 | OH | a2 | B17 | b3 | C15 | c2 | D02 | d1 | E02 |
| 2-1-d1E02-1-0708 | 756.154 | OH | a2 | B20 | b3 | C06 | c1 | D01 | d1 | E02 |
| 2-1-d1E02-1-0709 | 756.204 | OH | a1 | B01 | b3 | C18 | c1 | D01 | d1 | E02 |
| 2-1-d1E02-1-0710 | 756.204 | OH | a1 | B08 | b2 | C19 | c3 | D02 | d1 | E02 |
| 2-1-d1E02-1-0711 | 756.207 | OH | a2 | B17 | b3 | C16 | c1 | D01 | d1 | E02 |
| 2-1-d1E02-1-0712 | 756.258 | OH | a1 | B05 | b2 | C05 | c2 | D02 | d1 | E02 |
| 2-1-d1E02-1-0713 | 756.283 | OH | a2 | B16 | b2 | C07 | c2 | D02 | d1 | E02 |
| 2-1-d1E02-1-0714 | 756.298 | OH | a2 | B18 | b1 | C29 | c3 | D02 | d1 | E02 |
| 2-1-d1E02-1-0715 | 757.149 | OH | a2 | B20 | b1 | C12 | c2 | D02 | d1 | E02 |
| 2-1-d1E02-1-0716 | 757.152 | OH | a1 | B04 | b3 | C15 | c2 | D02 | d1 | E02 |
| 2-1-d1E02-1-0717 | 757.199 | OH | a1 | B02 | b3 | C18 | c1 | D01 | d1 | E02 |
| 2-1-d1E02-1-0718 | 757.199 | OH | a1 | B08 | b2 | C27 | c3 | D02 | d1 | E02 |
| 2-1-d1E02-1-0719 | 757.222 | OH | a1 | B04 | b3 | C16 | c1 | D01 | d1 | E02 |
| 2-1-d1E02-1-0720 | 757.234 | OH | a1 | B09 | b1 | C09 | c1 | D01 | d1 | E02 |
| 2-1-d1E02-1-0721 | 757.247 | OH | a1 | B10 | b1 | C13 | c1 | D01 | d1 | E02 |
| 2-1-d1E02-1-0722 | 757.278 | OH | a1 | B05 | b2 | C25 | c3 | D02 | d1 | E02 |
| 2-1-d1E02-1-0723 | 758.168 | OH | a2 | B17 | b3 | C17 | c1 | D01 | d1 | E02 |
| 2-1-d1E02-1-0724 | 758.168 | OH | a2 | B17 | b1 | C14 | c2 | D02 | d1 | E02 |
| 2-1-d1E02-1-0725 | 758.208 | OH | a2 | B15 | b3 | C08 | c2 | D02 | d1 | E02 |
| 2-1-d1E02-1-0726 | 758.237 | OH | a1 | B06 | b2 | C05 | c2 | D02 | d1 | E02 |
| 2-1-d1E02-1-0727 | 759.164 | OH | a1 | B09 | b3 | C06 | c1 | D01 | d1 | E02 |
| 2-1-d1E02-1-0728 | 759.182 | OH | a1 | B10 | b3 | C15 | c1 | D01 | d1 | E02 |
| 2-1-d1E02-1-0729 | 759.183 | OH | a1 | B04 | b3 | C17 | c1 | D01 | d1 | E02 |
| 2-1-d1E02-1-0730 | 759.183 | OH | a1 | B04 | b1 | C14 | c2 | D02 | d1 | E02 |
| 2-1-d1E02-1-0731 | 759.236 | OH | a2 | B18 | b2 | C05 | c2 | D02 | d1 | E02 |
| 2-1-d1E02-1-0732 | 759.237 | OH | a1 | B05 | b2 | C04 | c2 | D02 | d1 | E02 |
| 2-1-d1E02-1-0733 | 759.257 | OH | a1 | B06 | b2 | C25 | c3 | D02 | d1 | E02 |
| 2-1-d1E02-1-0734 | 759.294 | OH | a1 | B03 | b2 | C07 | c2 | D02 | d1 | E02 |
| 2-1-d1E02-1-0735 | 760.160 | OH | a1 | B09 | b1 | C12 | c2 | D02 | d1 | E02 |
| 2-1-d1E02-1-0736 | 760.162 | OH | a1 | B08 | b2 | C01 | c2 | D02 | d1 | E02 |
| 2-1-d1E02-1-0737 | 760.187 | OH | a2 | B16 | b3 | C08 | c2 | D02 | d1 | E02 |
| 2-1-d1E02-1-0738 | 760.254 | OH | a2 | B19 | b1 | C32 | c3 | D02 | d1 | E02 |
| 2-1-d1E02-1-0739 | 760.257 | OH | a2 | B18 | b2 | C25 | c3 | D02 | d1 | E02 |
| 2-1-d1E02-1-0740 | 761.213 | OH | a1 | B10 | b1 | C14 | c1 | D01 | d1 | E02 |
| 2-1-d1E02-1-0741 | 761.217 | OH | a1 | B06 | b2 | C04 | c2 | D02 | d1 | E02 |
| 2-1-d1E02-1-0742 | 761.234 | OH | a1 | B10 | b2 | C33 | c3 | D02 | d1 | E02 |
| 2-1-d1E02-1-0743 | 761.269 | OH | a1 | B07 | b1 | C29 | c3 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0744 | 762.197 | OH | a2 | B19 | b2 | C02 | c2 | D02 | d1 | E02 |
| 2-1-d1E02-1-0745 | 762.216 | OH | a2 | B18 | b2 | C04 | c2 | D02 | d1 | E02 |
| 2-1-d1E02-1-0746 | 762.234 | OH | a2 | B15 | b1 | C09 | c2 | D02 | d1 | E02 |
| 2-1-d1E02-1-0747 | 762.234 | OH | a2 | B19 | b2 | C21 | c3 | D02 | d1 | E02 |
| 2-1-d1E02-1-0748 | 762.234 | OH | a2 | B20 | b1 | C30 | c3 | D02 | d1 | E02 |
| 2-1-d1E02-1-0749 | 762.253 | OH | a2 | B17 | b2 | C07 | c2 | D02 | d1 | E02 |
| 2-1-d1E02-1-0750 | 762.309 | OH | a2 | B15 | b1 | C31 | c3 | D02 | d1 | E02 |
| 2-1-d1E02-1-0751 | 763.192 | OH | a2 | B19 | b2 | C03 | c2 | D02 | d1 | E02 |
| 2-1-d1E02-1-0752 | 763.198 | OH | a1 | B03 | b3 | C08 | c2 | D02 | d1 | E02 |
| 2-1-d1E02-1-0753 | 763.269 | OH | a1 | B04 | b2 | C07 | c2 | D02 | d1 | E02 |
| 2-1-d1E02-1-0754 | 764.164 | OH | a2 | B15 | b3 | C06 | c2 | D02 | d1 | E02 |
| 2-1-d1E02-1-0755 | 764.208 | OH | a1 | B07 | b2 | C05 | c2 | D02 | d1 | E02 |
| 2-1-d1E02-1-0756 | 764.213 | OH | a2 | B16 | b1 | C09 | c2 | D02 | d1 | E02 |
| 2-1-d1E02-1-0757 | 764.213 | OH | a2 | B20 | b2 | C19 | c3 | D02 | d1 | E02 |
| 2-1-d1E02-1-0758 | 764.288 | OH | a2 | B16 | b1 | C31 | c3 | D02 | d1 | E02 |
| 2-1-d1E02-1-0759 | 765.208 | OH | a2 | B20 | b2 | C27 | c3 | D02 | d1 | E02 |
| 2-1-d1E02-1-0760 | 765.228 | OH | a1 | B07 | b2 | C25 | c3 | D02 | d1 | E02 |
| 2-1-d1E02-1-0761 | 765.229 | OH | a2 | B15 | b3 | C18 | c1 | D01 | d1 | E02 |
| 2-1-d1E02-1-0762 | 765.244 | OH | a1 | B09 | b1 | C30 | c3 | D02 | d1 | E02 |
| 2-1-d1E02-1-0763 | 765.258 | OH | a1 | B05 | b1 | C13 | c2 | D02 | d1 | E02 |
| 2-1-d1E02-1-0764 | 765.299 | OH | a1 | B10 | b2 | C07 | c1 | D01 | d1 | E02 |
| 2-1-d1E02-1-0765 | 766.144 | OH | a2 | B16 | b3 | C06 | c2 | D02 | d1 | E02 |
| 2-1-d1E02-1-0766 | 766.158 | OH | a2 | B17 | b3 | C08 | c2 | D02 | d1 | E02 |
| 2-1-d1E02-1-0767 | 766.208 | OH | a2 | B19 | b2 | C23 | c3 | D02 | d1 | E02 |
| 2-1-d1E02-1-0768 | 767.173 | OH | a1 | B04 | b3 | C08 | c2 | D02 | d1 | E02 |
| 2-1-d1E02-1-0769 | 767.187 | OH | a1 | B07 | b2 | C04 | c2 | D02 | d1 | E02 |
| 2-1-d1E02-1-0770 | 767.193 | OH | a1 | B05 | b3 | C15 | c2 | D02 | d1 | E02 |
| 2-1-d1E02-1-0771 | 767.208 | OH | a2 | B16 | b3 | C18 | c1 | D01 | d1 | E02 |
| 2-1-d1E02-1-0772 | 767.224 | OH | a1 | B03 | b1 | C09 | c2 | D02 | d1 | E02 |
| 2-1-d1E02-1-0773 | 767.224 | OH | a1 | B09 | b2 | C19 | c3 | D02 | d1 | E02 |
| 2-1-d1E02-1-0774 | 767.237 | OH | a1 | B06 | b1 | C13 | c2 | D02 | d1 | E02 |
| 2-1-d1E02-1-0775 | 767.262 | OH | a1 | B05 | b3 | C16 | c1 | D01 | d1 | E02 |
| 2-1-d1E02-1-0776 | 767.299 | OH | a1 | B03 | b1 | C31 | c3 | D02 | d1 | E02 |
| 2-1-d1E02-1-0777 | 768.154 | OH | a2 | B19 | b2 | C06 | c2 | D02 | d1 | E02 |
| 2-1-d1E02-1-0778 | 768.171 | OH | a2 | B20 | b2 | C01 | c2 | D02 | d1 | E02 |
| 2-1-d1E02-1-0779 | 768.219 | OH | a1 | B09 | b2 | C27 | c3 | D02 | d1 | E02 |
| 2-1-d1E02-1-0780 | 768.237 | OH | a2 | B18 | b1 | C13 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0781 | 768.240 | OH | a1 | B08 | b1 | C32 | c3 | D02 | d1 | E02 |
| 2-1-d1E02-1-0782 | 769.155 | OH | a1 | B03 | b3 | C06 | c2 | D02 | d1 | E02 |
| 2-1-d1E02-1-0783 | 769.172 | OH | a1 | B06 | b3 | C15 | c2 | D02 | d1 | E02 |
| 2-1-d1E02-1-0784 | 769.203 | OH | a1 | B10 | b3 | C08 | c1 | D01 | d1 | E02 |
| 2-1-d1E02-1-0785 | 769.203 | OH | a1 | B10 | b1 | C08 | c2 | D02 | d1 | E02 |
| 2-1-d1E02-1-0786 | 769.224 | OH | a1 | B05 | b3 | C17 | c1 | D01 | d1 | E02 |
| 2-1-d1E02-1-0787 | 769.224 | OH | a1 | B05 | b1 | C14 | c2 | D02 | d1 | E02 |
| 2-1-d1E02-1-0788 | 769.242 | OH | a1 | B06 | b3 | C16 | c1 | D01 | d1 | E02 |
| 2-1-d1E02-1-0789 | 770.172 | OH | a2 | B18 | b3 | C15 | c2 | D02 | d1 | E02 |
| 2-1-d1E02-1-0790 | 770.183 | OH | a1 | B08 | b2 | C02 | c2 | D02 | d1 | E02 |
| 2-1-d1E02-1-0791 | 770.183 | OH | a2 | B17 | b1 | C09 | c2 | D02 | d1 | E02 |
| 2-1-d1E02-1-0792 | 770.198 | OH | a1 | B10 | b1 | C11 | c2 | D02 | d1 | E02 |
| 2-1-d1E02-1-0793 | 770.219 | OH | a1 | B03 | b3 | C18 | c1 | D01 | d1 | E02 |
| 2-1-d1E02-1-0794 | 770.219 | OH | a1 | B08 | b2 | C21 | c3 | D02 | d1 | E02 |
| 2-1-d1E02-1-0795 | 770.241 | OH | a2 | B18 | b3 | C16 | c1 | D01 | d1 | E02 |
| 2-1-d1E02-1-0796 | 770.259 | OH | a2 | B17 | b1 | C31 | c3 | D02 | d1 | E02 |
| 2-1-d1E02-1-0797 | 771.178 | OH | a1 | B08 | b2 | C03 | c2 | D02 | d1 | E02 |
| 2-1-d1E02-1-0798 | 771.182 | OH | a1 | B09 | b2 | C01 | c2 | D02 | d1 | E02 |
| 2-1-d1E02-1-0799 | 771.193 | OH | a1 | B10 | b1 | C10 | c2 | D02 | d1 | E02 |
| 2-1-d1E02-1-0800 | 771.199 | OH | a1 | B04 | b1 | C09 | c2 | D02 | d1 | E02 |
| 2-1-d1E02-1-0801 | 771.203 | OH | a1 | B06 | b3 | C17 | c1 | D01 | d1 | E02 |
| 2-1-d1E02-1-0802 | 771.203 | OH | a1 | B06 | b1 | C14 | c2 | D02 | d1 | E02 |
| 2-1-d1E02-1-0803 | 771.274 | OH | a1 | B04 | b1 | C31 | c3 | D02 | d1 | E02 |
| 2-1-d1E02-1-0804 | 772.114 | OH | a2 | B17 | b3 | C06 | c2 | D02 | d1 | E02 |
| 2-1-d1E02-1-0805 | 772.203 | OH | a2 | B18 | b3 | C17 | c1 | D01 | d1 | E02 |
| 2-1-d1E02-1-0806 | 772.203 | OH | a2 | B18 | b1 | C14 | c2 | D02 | d1 | E02 |
| 2-1-d1E02-1-0807 | 773.130 | OH | a1 | B04 | b3 | C06 | c2 | D02 | d1 | E02 |
| 2-1-d1E02-1-0808 | 773.179 | OH | a2 | B17 | b3 | C18 | c1 | D01 | d1 | E02 |
| 2-1-d1E02-1-0809 | 773.208 | OH | a1 | B07 | b1 | C13 | c2 | D02 | d1 | E02 |
| 2-1-d1E02-1-0810 | 773.228 | OH | a1 | B10 | b1 | C09 | c1 | D01 | d1 | E02 |
| 2-1-d1E02-1-0811 | 773.309 | OH | a1 | B05 | b2 | C07 | c2 | D02 | d1 | E02 |
| 2-1-d1E02-1-0812 | 774.194 | OH | a1 | B04 | b3 | C18 | c1 | D01 | d1 | E02 |
| 2-1-d1E02-1-0813 | 774.194 | OH | a1 | B08 | b2 | C23 | c3 | D02 | d1 | E02 |
| 2-1-d1E02-1-0814 | 774.270 | OH | a2 | B19 | b1 | C29 | c3 | D02 | d1 | E02 |
| 2-1-d1E02-1-0815 | 775.143 | OH | a1 | B07 | b3 | C15 | c2 | D02 | d1 | E02 |
| 2-1-d1E02-1-0816 | 775.159 | OH | a1 | B10 | b3 | C06 | c1 | D01 | d1 | E02 |
| 2-1-d1E02-1-0817 | 775.212 | OH | a1 | B07 | b3 | C16 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0818 | 775.289 | OH | a1 | B06 | b2 | C07 | c2 | D02 | d1 | E02 |
| 2-1-d1E02-1-0819 | 776.139 | OH | a1 | B08 | b2 | C06 | c2 | D02 | d1 | E02 |
| 2-1-d1E02-1-0820 | 776.155 | OH | a1 | B10 | b1 | C12 | c2 | D02 | d1 | E02 |
| 2-1-d1E02-1-0821 | 776.249 | OH | a2 | B20 | b1 | C32 | c3 | D02 | d1 | E02 |
| 2-1-d1E02-1-0822 | 776.288 | OH | a2 | B18 | b2 | C07 | c2 | D02 | d1 | E02 |
| 2-1-d1E02-1-0823 | 777.174 | OH | a1 | B07 | b3 | C17 | c1 | D01 | d1 | E02 |
| 2-1-d1E02-1-0824 | 777.174 | OH | a1 | B07 | b1 | C14 | c2 | D02 | d1 | E02 |
| 2-1-d1E02-1-0825 | 777.208 | OH | a2 | B19 | b2 | C05 | c2 | D02 | d1 | E02 |
| 2-1-d1E02-1-0826 | 777.214 | OH | a1 | B05 | b3 | C08 | c2 | D02 | d1 | E02 |
| 2-1-d1E02-1-0827 | 778.192 | OH | a2 | B20 | b2 | C02 | c2 | D02 | d1 | E02 |
| 2-1-d1E02-1-0828 | 778.228 | OH | a2 | B20 | b2 | C21 | c3 | D02 | d1 | E02 |
| 2-1-d1E02-1-0829 | 778.228 | OH | a2 | B19 | b2 | C25 | c3 | D02 | d1 | E02 |
| 2-1-d1E02-1-0830 | 779.187 | OH | a2 | B20 | b2 | C03 | c2 | D02 | d1 | E02 |
| 2-1-d1E02-1-0831 | 779.193 | OH | a1 | B06 | b3 | C08 | c2 | D02 | d1 | E02 |
| 2-1-d1E02-1-0832 | 779.260 | OH | a1 | B09 | b1 | C32 | c3 | D02 | d1 | E02 |
| 2-1-d1E02-1-0833 | 780.188 | OH | a2 | B19 | b2 | C04 | c2 | D02 | d1 | E02 |
| 2-1-d1E02-1-0834 | 780.192 | OH | a2 | B18 | b3 | C08 | c2 | D02 | d1 | E02 |
| 2-1-d1E02-1-0835 | 781.203 | OH | a1 | B09 | b2 | C02 | c2 | D02 | d1 | E02 |
| 2-1-d1E02-1-0836 | 781.239 | OH | a1 | B05 | b1 | C09 | c2 | D02 | d1 | E02 |
| 2-1-d1E02-1-0837 | 781.239 | OH | a1 | B09 | b2 | C21 | c3 | D02 | d1 | E02 |
| 2-1-d1E02-1-0838 | 781.239 | OH | a1 | B10 | b1 | C30 | c3 | D02 | d1 | E02 |
| 2-1-d1E02-1-0839 | 781.259 | OH | a1 | B07 | b2 | C07 | c2 | D02 | d1 | E02 |
| 2-1-d1E02-1-0840 | 781.315 | OH | a1 | B05 | b1 | C31 | c3 | D02 | d1 | E02 |
| 2-1-d1E02-1-0841 | 782.198 | OH | a1 | B09 | b2 | C03 | c2 | D02 | d1 | E02 |
| 2-1-d1E02-1-0842 | 782.203 | OH | a2 | B20 | b2 | C23 | c3 | D02 | d1 | E02 |
| 2-1-d1E02-1-0843 | 782.256 | OH | a1 | B08 | b1 | C29 | c3 | D02 | d1 | E02 |
| 2-1-d1E02-1-0844 | 783.170 | OH | a1 | B05 | b3 | C06 | c2 | D02 | d1 | E02 |
| 2-1-d1E02-1-0845 | 783.219 | OH | a1 | B06 | b1 | C09 | c2 | D02 | d1 | E02 |
| 2-1-d1E02-1-0846 | 783.219 | OH | a1 | B10 | b2 | C19 | c3 | D02 | d1 | E02 |
| 2-1-d1E02-1-0847 | 783.294 | OH | a1 | B06 | b1 | C31 | c3 | D02 | d1 | E02 |
| 2-1-d1E02-1-0848 | 784.148 | OH | a2 | B20 | b2 | C06 | c2 | D02 | d1 | E02 |
| 2-1-d1E02-1-0849 | 784.187 | OH | a2 | B11 | b3 | C16 | c2 | D02 | d1 | E02 |
| 2-1-d1E02-1-0850 | 784.214 | OH | a1 | B10 | b2 | C27 | c3 | D02 | d1 | E02 |
| 2-1-d1E02-1-0851 | 784.218 | OH | a2 | B18 | b1 | C09 | c2 | D02 | d1 | E02 |
| 2-1-d1E02-1-0852 | 784.235 | OH | a1 | B05 | b3 | C18 | c1 | D01 | d1 | E02 |
| 2-1-d1E02-1-0853 | 784.293 | OH | a2 | B18 | b1 | C31 | c3 | D02 | d1 | E02 |
| 2-1-d1E02-1-0854 | 785.15 | OH | a1 | B06 | b3 | C06 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0855 | 785.164 | OH | a1 | B07 | b3 | C08 | c2 | D02 | d1 | E02 |
| 2-1-d1E02-1-0856 | 785.183 | OH | a2 | B12 | b3 | C16 | c2 | D02 | d1 | E02 |
| 2-1-d1E02-1-0857 | 785.194 | OH | a1 | B08 | b2 | C05 | c2 | D02 | d1 | E02 |
| 2-1-d1E02-1-0858 | 785.214 | OH | a1 | B09 | b2 | C23 | c3 | D02 | d1 | E02 |
| 2-1-d1E02-1-0859 | 786.149 | OH | a2 | B18 | b3 | C06 | c2 | D02 | d1 | E02 |
| 2-1-d1E02-1-0860 | 786.149 | OH | a2 | B11 | b3 | C17 | c2 | D02 | d1 | E02 |
| 2-1-d1E02-1-0861 | 786.208 | OH | a2 | B19 | b1 | C13 | c2 | D02 | d1 | E02 |
| 2-1-d1E02-1-0862 | 786.214 | OH | a1 | B06 | b3 | C18 | c1 | D01 | d1 | E02 |
| 2-1-d1E02-1-0863 | 786.214 | OH | a1 | B08 | b2 | C25 | c3 | D02 | d1 | E02 |
| 2-1-d1E02-1-0864 | 787.144 | OH | a2 | B12 | b3 | C17 | c2 | D02 | d1 | E02 |
| 2-1-d1E02-1-0865 | 787.159 | OH | a1 | B09 | b2 | C06 | c2 | D02 | d1 | E02 |
| 2-1-d1E02-1-0866 | 787.177 | OH | a1 | B10 | b2 | C01 | c2 | D02 | d1 | E02 |
| 2-1-d1E02-1-0867 | 787.213 | OH | a2 | B18 | b3 | C18 | c1 | D01 | d1 | E02 |
| 2-1-d1E02-1-0868 | 788.143 | OH | a2 | B19 | b3 | C15 | c2 | D02 | d1 | E02 |
| 2-1-d1E02-1-0869 | 788.173 | OH | a1 | B08 | b2 | C04 | c2 | D02 | d1 | E02 |
| 2-1-d1E02-1-0870 | 788.213 | OH | a2 | B19 | b3 | C16 | c1 | D01 | d1 | E02 |
| 2-1-d1E02-1-0871 | 789.189 | OH | a1 | B07 | b1 | C09 | c2 | D02 | d1 | E02 |
| 2-1-d1E02-1-0872 | 789.264 | OH | a1 | B07 | b1 | C31 | c3 | D02 | d1 | E02 |
| 2-1-d1E02-1-0873 | 790.174 | OH | a2 | B19 | b3 | C17 | c1 | D01 | d1 | E02 |
| 2-1-d1E02-1-0874 | 790.174 | OH | a2 | B19 | b1 | C14 | c2 | D02 | d1 | E02 |
| 2-1-d1E02-1-0875 | 790.265 | OH | a2 | B20 | b1 | C29 | c3 | D02 | d1 | E02 |
| 2-1-d1E02-1-0876 | 791.120 | OH | a1 | B07 | b3 | C06 | c2 | D02 | d1 | E02 |
| 2-1-d1E02-1-0877 | 792.185 | OH | a1 | B07 | b3 | C18 | c1 | D01 | d1 | E02 |
| 2-1-d1E02-1-0878 | 793.203 | OH | a2 | B20 | b2 | C05 | c2 | D02 | d1 | E02 |
| 2-1-d1E02-1-0879 | 793.276 | OH | a1 | B09 | b1 | C29 | c3 | D02 | d1 | E02 |
| 2-1-d1E02-1-0880 | 794.194 | OH | a1 | B08 | b1 | C13 | c2 | D02 | d1 | E02 |
| 2-1-d1E02-1-0881 | 794.223 | OH | a2 | B20 | b2 | C25 | c3 | D02 | d1 | E02 |
| 2-1-d1E02-1-0882 | 794.260 | OH | a2 | B19 | b2 | C07 | c2 | D02 | d1 | E02 |
| 2-1-d1E02-1-0883 | 795.255 | OH | a1 | B10 | b1 | C32 | c3 | D02 | d1 | E02 |
| 2-1-d1E02-1-0884 | 796.129 | OH | a1 | B08 | b3 | C15 | c2 | D02 | d1 | E02 |
| 2-1-d1E02-1-0885 | 796.183 | OH | a2 | B20 | b2 | C04 | c2 | D02 | d1 | E02 |
| 2-1-d1E02-1-0886 | 796.199 | OH | a1 | B08 | b3 | C16 | c1 | D01 | d1 | E02 |
| 2-1-d1E02-1-0887 | 796.214 | OH | a1 | B09 | b2 | C05 | c2 | D02 | d1 | E02 |
| 2-1-d1E02-1-0888 | 797.198 | OH | a1 | B10 | b2 | C02 | c2 | D02 | d1 | E02 |
| 2-1-d1E02-1-0889 | 797.234 | OH | a1 | B10 | b2 | C21 | c3 | D02 | d1 | E02 |
| 2-1-d1E02-1-0890 | 797.234 | OH | a1 | B09 | b2 | C25 | c3 | D02 | d1 | E02 |
| 2-1-d1E02-1-0891 | 798.160 | OH | a1 | B08 | b3 | C17 | c1 | D01 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0892 | 798.160 | OH | a1 | B08 | b1 | C14 | c2 | D02 | d1 | E02 |
| 2-1-d1E02-1-0893 | 798.164 | OH | a2 | B19 | b3 | C08 | c2 | D02 | d1 | E02 |
| 2-1-d1E02-1-0894 | 798.193 | OH | a1 | B10 | b2 | C03 | c2 | D02 | d1 | E02 |
| 2-1-d1E02-1-0895 | 798.203 | OH | a2 | B13 | b3 | C16 | c2 | D02 | d1 | E02 |
| 2-1-d1E02-1-0896 | 799.194 | OH | a1 | B09 | b2 | C04 | c2 | D02 | d1 | E02 |
| 2-1-d1E02-1-0897 | 800.164 | OH | a2 | B13 | b3 | C17 | c2 | D02 | d1 | E02 |
| 2-1-d1E02-1-0898 | 801.16 | OH | a2 | B11 | b3 | C18 | c2 | D02 | d1 | E02 |
| 2-1-d1E02-1-0899 | 801.209 | OH | a1 | B10 | b2 | C23 | c3 | D02 | d1 | E02 |
| 2-1-d1E02-1-0900 | 802.155 | OH | a2 | B12 | b3 | C18 | c2 | D02 | d1 | E02 |
| 2-1-d1E02-1-0901 | 802.178 | OH | a2 | B14 | b3 | C16 | c2 | D02 | d1 | E02 |
| 2-1-d1E02-1-0902 | 802.190 | OH | a2 | B19 | b1 | C09 | c2 | D02 | d1 | E02 |
| 2-1-d1E02-1-0903 | 802.203 | OH | a2 | B20 | b1 | C13 | c2 | D02 | d1 | E02 |
| 2-1-d1E02-1-0904 | 802.245 | OH | a1 | B08 | b2 | C07 | c2 | D02 | d1 | E02 |
| 2-1-d1E02-1-0905 | 802.265 | OH | a2 | B19 | b1 | C31 | c3 | D02 | d1 | E02 |
| 2-1-d1E02-1-0906 | 803.154 | OH | a1 | B10 | b2 | C06 | c2 | D02 | d1 | E02 |
| 2-1-d1E02-1-0907 | 803.193 | OH | a1 | B01 | b3 | C16 | c2 | D02 | d1 | E02 |
| 2-1-d1E02-1-0908 | 804.121 | OH | a2 | B19 | b3 | C06 | c2 | D02 | d1 | E02 |
| 2-1-d1E02-1-0909 | 804.138 | OH | a2 | B20 | b3 | C15 | c2 | D02 | d1 | E02 |
| 2-1-d1E02-1-0910 | 804.139 | OH | a2 | B14 | b3 | C17 | c2 | D02 | d1 | E02 |
| 2-1-d1E02-1-0911 | 804.188 | OH | a1 | B02 | b3 | C16 | c2 | D02 | d1 | E02 |
| 2-1-d1E02-1-0912 | 804.208 | OH | a2 | B20 | b3 | C16 | c1 | D01 | d1 | E02 |
| 2-1-d1E02-1-0913 | 805.155 | OH | a1 | B01 | b3 | C17 | c2 | D02 | d1 | E02 |
| 2-1-d1E02-1-0914 | 805.185 | OH | a2 | B19 | b3 | C18 | c1 | D01 | d1 | E02 |
| 2-1-d1E02-1-0915 | 805.214 | OH | a1 | B09 | b1 | C13 | c2 | D02 | d1 | E02 |
| 2-1-d1E02-1-0916 | 806.150 | OH | a1 | B02 | b3 | C17 | c2 | D02 | d1 | E02 |
| 2-1-d1E02-1-0917 | 806.150 | OH | a1 | B08 | b3 | C08 | c2 | D02 | d1 | E02 |
| 2-1-d1E02-1-0918 | 806.169 | OH | a2 | B20 | b3 | C17 | c1 | D01 | d1 | E02 |
| 2-1-d1E02-1-0919 | 806.169 | OH | a2 | B20 | b1 | C14 | c2 | D02 | d1 | E02 |
| 2-1-d1E02-1-0920 | 807.149 | OH | a1 | B09 | b3 | C15 | c2 | D02 | d1 | E02 |
| 2-1-d1E02-1-0921 | 807.219 | OH | a1 | B09 | b3 | C16 | c1 | D01 | d1 | E02 |
| 2-1-d1E02-1-0922 | 809.180 | OH | a1 | B09 | b3 | C17 | c1 | D01 | d1 | E02 |
| 2-1-d1E02-1-0923 | 809.180 | OH | a1 | B09 | b1 | C14 | c2 | D02 | d1 | E02 |
| 2-1-d1E02-1-0924 | 809.271 | OH | a1 | B10 | b1 | C29 | c3 | D02 | d1 | E02 |
| 2-1-d1E02-1-0925 | 810.175 | OH | a1 | B08 | b1 | C09 | c2 | D02 | d1 | E02 |
| 2-1-d1E02-1-0926 | 810.251 | OH | a1 | B08 | b1 | C31 | c3 | D02 | d1 | E02 |
| 2-1-d1E02-1-0927 | 810.255 | OH | a2 | B20 | b2 | C07 | c2 | D02 | d1 | E02 |
| 2-1-d1E02-1-0928 | 812.106 | OH | a1 | B08 | b3 | C06 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0929 | 812.209 | OH | a1 | B10 | b2 | C05 | c2 | D02 | d1 | E02 |
| 2-1-d1E02-1-0930 | 812.219 | OH | a2 | B15 | b3 | C16 | c2 | D02 | d1 | E02 |
| 2-1-d1E02-1-0931 | 813.171 | OH | a1 | B08 | b3 | C18 | c1 | D01 | d1 | E02 |
| 2-1-d1E02-1-0932 | 813.229 | OH | a1 | B10 | b2 | C25 | c3 | D02 | d1 | E02 |
| 2-1-d1E02-1-0933 | 813.266 | OH | a1 | B09 | b2 | C07 | c2 | D02 | d1 | E02 |
| 2-1-d1E02-1-0934 | 814.159 | OH | a2 | B20 | b3 | C08 | c2 | D02 | d1 | E02 |
| 2-1-d1E02-1-0935 | 814.180 | OH | a2 | B15 | b3 | C17 | c2 | D02 | d1 | E02 |
| 2-1-d1E02-1-0936 | 814.198 | OH | a2 | B16 | b3 | C16 | c2 | D02 | d1 | E02 |
| 2-1-d1E02-1-0937 | 815.175 | OH | a2 | B13 | b3 | C18 | c2 | D02 | d1 | E02 |
| 2-1-d1E02-1-0938 | 815.188 | OH | a1 | B10 | b2 | C04 | c2 | D02 | d1 | E02 |
| 2-1-d1E02-1-0939 | 816.159 | OH | a2 | B16 | b3 | C17 | c2 | D02 | d1 | E02 |
| 2-1-d1E02-1-0940 | 817.170 | OH | a1 | B09 | b3 | C08 | c2 | D02 | d1 | E02 |
| 2-1-d1E02-1-0941 | 817.209 | OH | a1 | B03 | b3 | C16 | c2 | D02 | d1 | E02 |
| 2-1-d1E02-1-0942 | 818.185 | OH | a2 | B20 | b1 | C09 | c2 | D02 | d1 | E02 |
| 2-1-d1E02-1-0943 | 818.260 | OH | a2 | B20 | b1 | C31 | c3 | D02 | d1 | E02 |
| 2-1-d1E02-1-0944 | 819.150 | OH | a2 | B14 | b3 | C18 | c2 | D02 | d1 | E02 |
| 2-1-d1E02-1-0945 | 819.170 | OH | a1 | B03 | b3 | C17 | c2 | D02 | d1 | E02 |
| 2-1-d1E02-1-0946 | 820.115 | OH | a2 | B20 | b3 | C06 | c2 | D02 | d1 | E02 |
| 2-1-d1E02-1-0947 | 820.166 | OH | a1 | B01 | b3 | C18 | c2 | D02 | d1 | E02 |
| 2-1-d1E02-1-0948 | 820.168 | OH | a2 | B17 | b3 | C16 | c2 | D02 | d1 | E02 |
| 2-1-d1E02-1-0949 | 821.161 | OH | a1 | B02 | b3 | C18 | c2 | D02 | d1 | E02 |
| 2-1-d1E02-1-0950 | 821.180 | OH | a2 | B20 | b3 | C18 | c1 | D01 | d1 | E02 |
| 2-1-d1E02-1-0951 | 821.184 | OH | a1 | B04 | b3 | C16 | c2 | D02 | d1 | E02 |
| 2-1-d1E02-1-0952 | 821.195 | OH | a1 | B09 | b1 | C09 | c2 | D02 | d1 | E02 |
| 2-1-d1E02-1-0953 | 821.209 | OH | a1 | B10 | b1 | C13 | c2 | D02 | d1 | E02 |
| 2-1-d1E02-1-0954 | 821.271 | OH | a1 | B09 | b1 | C31 | c3 | D02 | d1 | E02 |
| 2-1-d1E02-1-0955 | 822.130 | OH | a2 | B17 | b3 | C17 | c2 | D02 | d1 | E02 |
| 2-1-d1E02-1-0956 | 823.126 | OH | a1 | B09 | b3 | C06 | c2 | D02 | d1 | E02 |
| 2-1-d1E02-1-0957 | 823.144 | OH | a1 | B10 | b3 | C15 | c2 | D02 | d1 | E02 |
| 2-1-d1E02-1-0958 | 823.145 | OH | a1 | B04 | b3 | C17 | c2 | D02 | d1 | E02 |
| 2-1-d1E02-1-0959 | 823.213 | OH | a1 | B10 | b3 | C16 | c1 | D01 | d1 | E02 |
| 2-1-d1E02-1-0960 | 824.191 | OH | a1 | B09 | b3 | C18 | c1 | D01 | d1 | E02 |
| 2-1-d1E02-1-0961 | 825.175 | OH | a1 | B10 | b3 | C17 | c1 | D01 | d1 | E02 |
| 2-1-d1E02-1-0962 | 825.175 | OH | a1 | B10 | b1 | C14 | c2 | D02 | d1 | E02 |
| 2-1-d1E02-1-0963 | 829.191 | OH | a2 | B15 | b3 | C18 | c2 | D02 | d1 | E02 |
| 2-1-d1E02-1-0964 | 829.260 | OH | a1 | B10 | b2 | C07 | c2 | D02 | d1 | E02 |
| 2-1-d1E02-1-0965 | 831.170 | OH | a2 | B16 | b3 | C18 | c2 | D02 | d1 | E02 |

(continued)

| [Table 8-7-3] Compound that may be contained in mixture 2-1-d1E02-1 | | | | | | | | | | |
|---|---|---|---|---|---|---|---|---|---|---|
| ID | Exact Mass | | a | B | b | C | c | D | d | E |
| 2-1-d1E02-1-0966 | 831.224 | OH | a1 | B05 | b3 | C16 | c2 | D02 | d1 | E02 |
| 2-1-d1E02-1-0967 | 833.165 | OH | a1 | B10 | b3 | C08 | c2 | D02 | d1 | E02 |
| 2-1-d1E02-1-0968 | 833.186 | OH | a1 | B05 | b3 | C17 | c2 | D02 | d1 | E02 |
| 2-1-d1E02-1-0969 | 833.204 | OH | a1 | B06 | b3 | C16 | c2 | D02 | d1 | E02 |
| 2-1-d1E02-1-0970 | 834.181 | OH | a1 | B03 | b3 | C18 | c2 | D02 | d1 | E02 |
| 2-1-d1E02-1-0971 | 834.203 | OH | a2 | B18 | b3 | C16 | c2 | D02 | d1 | E02 |
| 2-1-d1E02-1-0972 | 835.165 | OH | a1 | B06 | b3 | C17 | c2 | D02 | d1 | E02 |
| 2-1-d1E02-1-0973 | 836.164 | OH | a2 | B18 | b3 | C17 | c2 | D02 | d1 | E02 |
| 2-1-d1E02-1-0974 | 837.141 | OH | a2 | B17 | b3 | C18 | c2 | D02 | d1 | E02 |
| 2-1-d1E02-1-0975 | 837.19 | OH | a1 | B10 | b1 | C09 | c2 | D02 | d1 | E02 |
| 2-1-d1E02-1-0976 | 837.266 | OH | a1 | B10 | b1 | C31 | c3 | D02 | d1 | E02 |
| 2-1-d1E02-1-0977 | 838.156 | OH | a1 | B04 | b3 | C18 | c2 | D02 | d1 | E02 |
| 2-1-d1E02-1-0978 | 839.121 | OH | a1 | B10 | b3 | C06 | c2 | D02 | d1 | E02 |
| 2-1-d1E02-1-0979 | 839.174 | OH | a1 | B07 | b3 | C16 | c2 | D02 | d1 | E02 |
| 2-1-d1E02-1-0980 | 840.186 | OH | a1 | B10 | b3 | C18 | c1 | D01 | d1 | E02 |
| 2-1-d1E02-1-0981 | 841.136 | OH | a1 | B07 | b3 | C17 | c2 | D02 | d1 | E02 |
| 2-1-d1E02-1-0982 | 848.197 | OH | a1 | B05 | b3 | C18 | c2 | D02 | d1 | E02 |
| 2-1-d1E02-1-0983 | 850.176 | OH | a1 | B06 | b3 | C18 | c2 | D02 | d1 | E02 |
| 2-1-d1E02-1-0984 | 851.175 | OH | a2 | B18 | b3 | C18 | c2 | D02 | d1 | E02 |
| 2-1-d1E02-1-0985 | 852.175 | OH | a2 | B19 | b3 | C16 | c2 | D02 | d1 | E02 |
| 2-1-d1E02-1-0986 | 854.136 | OH | a2 | B19 | b3 | C17 | c2 | D02 | d1 | E02 |
| 2-1-d1E02-1-0987 | 856.147 | OH | a1 | B07 | b3 | C18 | c2 | D02 | d1 | E02 |
| 2-1-d1E02-1-0988 | 860.160 | OH | a1 | B08 | b3 | C16 | c2 | D02 | d1 | E02 |
| 2-1-d1E02-1-0989 | 862.122 | OH | a1 | B08 | b3 | C17 | c2 | D02 | d1 | E02 |
| 2-1-d1E02-1-0990 | 868.170 | OH | a2 | B20 | b3 | C16 | c2 | D02 | d1 | E02 |
| 2-1-d1E02-1-0991 | 869.147 | OH | a2 | B19 | b3 | C18 | c2 | D02 | d1 | E02 |
| 2-1-d1E02-1-0992 | 870.131 | OH | a2 | B20 | b3 | C17 | c2 | D02 | d1 | E02 |
| 2-1-d1E02-1-0993 | 871.180 | OH | a1 | B09 | b3 | C16 | c2 | D02 | d1 | E02 |
| 2-1-d1E02-1-0994 | 873.142 | OH | a1 | B09 | b3 | C17 | c2 | D02 | d1 | E02 |
| 2-1-d1E02-1-0995 | 877.133 | OH | a1 | B08 | b3 | C18 | c2 | D02 | d1 | E02 |
| 2-1-d1E02-1-0996 | 885.142 | OH | a2 | B20 | b3 | C18 | c2 | D02 | d1 | E02 |
| 2-1-d1E02-1-0997 | 887.175 | OH | a1 | B10 | b3 | C16 | c2 | D02 | d1 | E02 |
| 2-1-d1E02-1-0998 | 888.153 | OH | a1 | B09 | b3 | C18 | c2 | D02 | d1 | E02 |
| 2-1-d1E02-1-0999 | 889.137 | OH | a1 | B10 | b3 | C17 | c2 | D02 | d1 | E02 |
| 2-1-d1E02-1-1000 | 904.148 | OH | a1 | B10 | b3 | C18 | c2 | D02 | d1 | E02 |

[Table 390]

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0001 | 640.214 | OH | a2 | B11 | b1 | C08 | c1 | D01 | d1 | E03 |
| 2-1-d1E03-1-0002 | 641.210 | OH | a2 | B12 | b1 | C08 | c1 | D01 | d1 | E03 |
| 2-1-d1E03-1-0003 | 641.210 | OH | a2 | B11 | b1 | C11 | c1 | D01 | d1 | E03 |
| 2-1-d1E03-1-0004 | 642.205 | OH | a2 | B11 | b1 | C10 | c1 | D01 | d1 | E03 |
| 2-1-d1E03-1-0005 | 642.205 | OH | a2 | B12 | b1 | C11 | c1 | D01 | d1 | E03 |
| 2-1-d1E03-1-0006 | 643.200 | OH | a2 | B12 | b1 | C10 | c1 | D01 | d1 | E03 |
| 2-1-d1E03-1-0007 | 647.166 | OH | a2 | B11 | b1 | C12 | c1 | D01 | d1 | E03 |
| 2-1-d1E03-1-0008 | 648.161 | OH | a2 | B12 | b1 | C12 | c1 | D01 | d1 | E03 |
| 2-1-d1E03-1-0009 | 654.230 | OH | a2 | B13 | b1 | C08 | c1 | D01 | d1 | E03 |
| 2-1-d1E03-1-0010 | 655.225 | OH | a2 | B13 | b1 | C11 | c1 | D01 | d1 | E03 |
| 2-1-d1E03-1-0011 | 656.221 | OH | a2 | B13 | b1 | C10 | c1 | D01 | d1 | E03 |
| 2-1-d1E03-1-0012 | 658.189 | OH | a2 | B11 | b2 | C01 | c1 | D01 | d1 | E03 |
| 2-1-d1E03-1-0013 | 658.205 | OH | a2 | B14 | b1 | C08 | c1 | D01 | d1 | E03 |
| 2-1-d1E03-1-0014 | 659.184 | OH | a2 | B12 | b2 | C01 | c1 | D01 | d1 | E03 |
| 2-1-d1E03-1-0015 | 659.200 | OH | a2 | B14 | b1 | C11 | c1 | D01 | d1 | E03 |
| 2-1-d1E03-1-0016 | 659.220 | OH | a1 | B01 | b1 | C08 | c1 | D01 | d1 | E03 |
| 2-1-d1E03-1-0017 | 660.196 | OH | a2 | B14 | b1 | C10 | c1 | D01 | d1 | E03 |
| 2-1-d1E03-1-0018 | 660.215 | OH | a1 | B02 | b1 | C08 | c1 | D01 | d1 | E03 |
| 2-1-d1E03-1-0019 | 660.215 | OH | a1 | B01 | b1 | C11 | c1 | D01 | d1 | E03 |
| 2-1-d1E03-1-0020 | 661.182 | OH | a2 | B13 | b1 | C12 | c1 | D01 | d1 | E03 |
| 2-1-d1E03-1-0021 | 661.211 | OH | a1 | B01 | b1 | C10 | c1 | D01 | d1 | E03 |
| 2-1-d1E03-1-0022 | 661.211 | OH | a1 | B02 | b1 | C11 | c1 | D01 | d1 | E03 |
| 2-1-d1E03-1-0023 | 662.206 | OH | a1 | B02 | b1 | C10 | c1 | D01 | d1 | E03 |
| 2-1-d1E03-1-0024 | 665.157 | OH | a2 | B14 | b1 | C12 | c1 | D01 | d1 | E03 |
| 2-1-d1E03-1-0025 | 666.172 | OH | a1 | B01 | b1 | C12 | c1 | D01 | d1 | E03 |
| 2-1-d1E03-1-0026 | 667.167 | OH | a1 | B02 | b1 | C12 | c1 | D01 | d1 | E03 |
| 2-1-d1E03-1-0027 | 668.209 | OH | a2 | B11 | b2 | C02 | c1 | D01 | d1 | E03 |
| 2-1-d1E03-1-0028 | 668.246 | OH | a2 | B15 | b1 | C08 | c1 | D01 | d1 | E03 |
| 2-1-d1E03-1-0029 | 669.205 | OH | a2 | B12 | b2 | C02 | c1 | D01 | d1 | E03 |
| 2-1-d1E03-1-0030 | 669.205 | OH | a2 | B11 | b2 | C03 | c1 | D01 | d1 | E03 |
| 2-1-d1E03-1-0031 | 669.241 | OH | a2 | B15 | b1 | C11 | c1 | D01 | d1 | E03 |
| 2-1-d1E03-1-0032 | 670.200 | OH | a2 | B12 | b2 | C03 | c1 | D01 | d1 | E03 |
| 2-1-d1E03-1-0033 | 670.225 | OH | a2 | B16 | b1 | C08 | c1 | D01 | d1 | E03 |
| 2-1-d1E03-1-0034 | 670.236 | OH | a2 | B15 | b1 | C10 | c1 | D01 | d1 | E03 |
| 2-1-d1E03-1-0035 | 671.220 | OH | a2 | B16 | b1 | C11 | c1 | D01 | d1 | E03 |
| 2-1-d1E03-1-0036 | 672.204 | OH | a2 | B13 | b2 | C01 | c1 | D01 | d1 | E03 |
| 2-1-d1E03-1-0037 | 672.215 | OH | a2 | B16 | b1 | C10 | c1 | D01 | d1 | E03 |
| 2-1-d1E03-1-0038 | 673.236 | OH | a1 | B03 | b1 | C08 | c1 | D01 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0039 | 674.166 | OH | a2 | B11 | b2 | C06 | c1 | D01 | d1 | E03 |
| 2-1-d1E03-1-0040 | 674.231 | OH | a1 | B03 | b1 | C11 | c1 | D01 | d1 | E03 |
| 2-1-d1E03-1-0041 | 675.161 | OH | a2 | B12 | b2 | C06 | c1 | D01 | d1 | E03 |
| 2-1-d1E03-1-0042 | 675.197 | OH | a2 | B15 | b1 | C12 | c1 | D01 | d1 | E03 |
| 2-1-d1E03-1-0043 | 675.226 | OH | a1 | B03 | b1 | C10 | c1 | D01 | d1 | E03 |
| 2-1-d1E03-1-0044 | 676.179 | OH | a2 | B14 | b2 | C01 | c1 | D01 | d1 | E03 |
| 2-1-d1E03-1-0045 | 676.196 | OH | a2 | B17 | b1 | C08 | c1 | D01 | d1 | E03 |
| 2-1-d1E03-1-0046 | 677.177 | OH | a2 | B16 | b1 | C12 | c1 | D01 | d1 | E03 |
| 2-1-d1E03-1-0047 | 677.191 | OH | a2 | B17 | b1 | C11 | c1 | D01 | d1 | E03 |
| 2-1-d1E03-1-0048 | 677.194 | OH | a1 | B01 | b2 | C01 | c1 | D01 | d1 | E03 |
| 2-1-d1E03-1-0049 | 677.211 | OH | a1 | B04 | b1 | C08 | c1 | D01 | d1 | E03 |
| 2-1-d1E03-1-0050 | 678.186 | OH | a2 | B17 | b1 | C10 | c1 | D01 | d1 | E03 |
| 2-1-d1E03-1-0051 | 678.190 | OH | a1 | B02 | b2 | C01 | c1 | D01 | d1 | E03 |
| 2-1-d1E03-1-0052 | 678.206 | OH | a1 | B04 | b1 | C11 | c1 | D01 | d1 | E03 |
| 2-1-d1E03-1-0053 | 679.201 | OH | a1 | B04 | b1 | C10 | c1 | D01 | d1 | E03 |
| 2-1-d1E03-1-0054 | 680.188 | OH | a1 | B03 | b1 | C12 | c1 | D01 | d1 | E03 |
| 2-1-d1E03-1-0055 | 682.225 | OH | a2 | B13 | b2 | C02 | c1 | D01 | d1 | E03 |
| 2-1-d1E03-1-0056 | 683.147 | OH | a2 | B17 | b1 | C12 | c1 | D01 | d1 | E03 |
| 2-1-d1E03-1-0057 | 683.220 | OH | a2 | B13 | b2 | C03 | c1 | D01 | d1 | E03 |
| 2-1-d1E03-1-0058 | 683.220 | OH | a2 | B11 | b2 | C05 | c1 | D01 | d1 | E03 |
| 2-1-d1E03-1-0059 | 684.162 | OH | a1 | B04 | b1 | C12 | c1 | D01 | d1 | E03 |
| 2-1-d1E03-1-0060 | 684.215 | OH | a2 | B12 | b2 | C05 | c1 | D01 | d1 | E03 |
| 2-1-d1E03-1-0061 | 686.200 | OH | a2 | B14 | b2 | C02 | c1 | D01 | d1 | E03 |
| 2-1-d1E03-1-0062 | 686.200 | OH | a2 | B11 | b2 | C04 | c1 | D01 | d1 | E03 |
| 2-1-d1E03-1-0063 | 686.220 | OH | a2 | B15 | b2 | C01 | c1 | D01 | d1 | E03 |
| 2-1-d1E03-1-0064 | 687.195 | OH | a2 | B12 | b2 | C04 | c1 | D01 | d1 | E03 |
| 2-1-d1E03-1-0065 | 687.195 | OH | a2 | B14 | b2 | C03 | c1 | D01 | d1 | E03 |
| 2-1-d1E03-1-0066 | 687.215 | OH | a1 | B01 | b2 | C02 | c1 | D01 | d1 | E03 |
| 2-1-d1E03-1-0067 | 687.252 | OH | a1 | B05 | b1 | C08 | c1 | D01 | d1 | E03 |
| 2-1-d1E03-1-0068 | 688.181 | OH | a2 | B13 | b2 | C06 | c1 | D01 | d1 | E03 |
| 2-1-d1E03-1-0069 | 688.199 | OH | a2 | B16 | b2 | C01 | c1 | D01 | d1 | E03 |
| 2-1-d1E03-1-0070 | 688.210 | OH | a1 | B02 | b2 | C02 | c1 | D01 | d1 | E03 |
| 2-1-d1E03-1-0071 | 688.210 | OH | a1 | B01 | b2 | C03 | c1 | D01 | d1 | E03 |
| 2-1-d1E03-1-0072 | 688.247 | OH | a1 | B05 | b1 | C11 | c1 | D01 | d1 | E03 |
| 2-1-d1E03-1-0073 | 689.206 | OH | a1 | B02 | b2 | C03 | c1 | D01 | d1 | E03 |
| 2-1-d1E03-1-0074 | 689.231 | OH | a1 | B06 | b1 | C08 | c1 | D01 | d1 | E03 |
| 2-1-d1E03-1-0075 | 689.242 | OH | a1 | B05 | b1 | C10 | c1 | D01 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0076 | 690.226 | OH | a1 | B06 | b1 | C11 | c1 | D01 | d1 | E03 |
| 2-1-d1E03-1-0077 | 690.230 | OH | a2 | B18 | b1 | C08 | c1 | D01 | d1 | E03 |
| 2-1-d1E03-1-0078 | 691.210 | OH | a1 | B03 | b2 | C01 | c1 | D01 | d1 | E03 |
| 2-1-d1E03-1-0079 | 691.221 | OH | a1 | B06 | b1 | C10 | c1 | D01 | d1 | E03 |
| 2-1-d1E03-1-0080 | 691.225 | OH | a2 | B18 | b1 | C11 | c1 | D01 | d1 | E03 |
| 2-1-d1E03-1-0081 | 692.156 | OH | a2 | B14 | b2 | C06 | c1 | D01 | d1 | E03 |
| 2-1-d1E03-1-0082 | 692.221 | OH | a2 | B18 | b1 | C10 | c1 | D01 | d1 | E03 |
| 2-1-d1E03-1-0083 | 692.221 | OH | a2 | B11 | b1 | C13 | c1 | D01 | d1 | E03 |
| 2-1-d1E03-1-0084 | 693.172 | OH | a1 | B01 | b2 | C06 | c1 | D01 | d1 | E03 |
| 2-1-d1E03-1-0085 | 693.216 | OH | a2 | B12 | b1 | C13 | c1 | D01 | d1 | E03 |
| 2-1-d1E03-1-0086 | 694.156 | OH | a2 | B11 | b3 | C15 | c1 | D01 | d1 | E03 |
| 2-1-d1E03-1-0087 | 694.167 | OH | a1 | B02 | b2 | C06 | c1 | D01 | d1 | E03 |
| 2-1-d1E03-1-0088 | 694.170 | OH | a2 | B17 | b2 | C01 | c1 | D01 | d1 | E03 |
| 2-1-d1E03-1-0089 | 694.203 | OH | a1 | B05 | b1 | C12 | c1 | D01 | d1 | E03 |
| 2-1-d1E03-1-0090 | 695.151 | OH | a2 | B12 | b3 | C15 | c1 | D01 | d1 | E03 |
| 2-1-d1E03-1-0091 | 695.185 | OH | a1 | B04 | b2 | C01 | c1 | D01 | d1 | E03 |
| 2-1-d1E03-1-0092 | 695.201 | OH | a1 | B07 | b1 | C08 | c1 | D01 | d1 | E03 |
| 2-1-d1E03-1-0093 | 696.182 | OH | a1 | B06 | b1 | C12 | c1 | D01 | d1 | E03 |
| 2-1-d1E03-1-0094 | 696.186 | OH | a2 | B11 | b1 | C14 | c1 | D01 | d1 | E03 |
| 2-1-d1E03-1-0095 | 696.197 | OH | a1 | B07 | b1 | C11 | c1 | D01 | d1 | E03 |
| 2-1-d1E03-1-0096 | 696.208 | OH | a2 | B11 | b2 | C33 | c3 | D02 | d1 | E03 |
| 2-1-d1E03-1-0097 | 696.241 | OH | a2 | B15 | b2 | C02 | c1 | D01 | d1 | E03 |
| 2-1-d1E03-1-0098 | 697.182 | OH | a2 | B18 | b1 | C12 | c1 | D01 | d1 | E03 |
| 2-1-d1E03-1-0099 | 697.182 | OH | a2 | B12 | b1 | C14 | c1 | D01 | d1 | E03 |
| 2-1-d1E03-1-0100 | 697.192 | OH | a1 | B07 | b1 | C10 | c1 | D01 | d1 | E03 |
| 2-1-d1E03-1-0101 | 697.203 | OH | a2 | B12 | b2 | C33 | c3 | D02 | d1 | E03 |
| 2-1-d1E03-1-0102 | 697.236 | OH | a2 | B15 | b2 | C03 | c1 | D01 | d1 | E03 |
| 2-1-d1E03-1-0103 | 697.236 | OH | a2 | B13 | b2 | C05 | c1 | D01 | d1 | E03 |
| 2-1-d1E03-1-0104 | 698.220 | OH | a2 | B16 | b2 | C02 | c1 | D01 | d1 | E03 |
| 2-1-d1E03-1-0105 | 699.215 | OH | a2 | B16 | b2 | C03 | c1 | D01 | d1 | E03 |
| 2-1-d1E03-1-0106 | 700.216 | OH | a2 | B13 | b2 | C04 | c1 | D01 | d1 | E03 |
| 2-1-d1E03-1-0107 | 700.272 | OH | a2 | B11 | b2 | C07 | c1 | D01 | d1 | E03 |
| 2-1-d1E03-1-0108 | 701.211 | OH | a2 | B14 | b2 | C05 | c1 | D01 | d1 | E03 |
| 2-1-d1E03-1-0109 | 701.231 | OH | a1 | B03 | b2 | C02 | c1 | D01 | d1 | E03 |
| 2-1-d1E03-1-0110 | 701.267 | OH | a2 | B12 | b2 | C07 | c1 | D01 | d1 | E03 |
| 2-1-d1E03-1-0111 | 702.153 | OH | a1 | B07 | b1 | C12 | c1 | D01 | d1 | E03 |
| 2-1-d1E03-1-0112 | 702.197 | OH | a2 | B15 | b2 | C06 | c1 | D01 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0113 | 702.226 | OH | a1 | B03 | b2 | C03 | c1 | D01 | d1 | E03 |
| 2-1-d1E03-1-0114 | 702.226 | OH | a1 | B01 | b2 | C05 | c1 | D01 | d1 | E03 |
| 2-1-d1E03-1-0115 | 703.221 | OH | a1 | B02 | b2 | C05 | c1 | D01 | d1 | E03 |
| 2-1-d1E03-1-0116 | 704.176 | OH | a2 | B16 | b2 | C06 | c1 | D01 | d1 | E03 |
| 2-1-d1E03-1-0117 | 704.176 | OH | a2 | B11 | b3 | C08 | c1 | D01 | d1 | E03 |
| 2-1-d1E03-1-0118 | 704.176 | OH | a2 | B11 | b1 | C08 | c2 | D02 | d1 | E03 |
| 2-1-d1E03-1-0119 | 704.190 | OH | a2 | B17 | b2 | C02 | c1 | D01 | d1 | E03 |
| 2-1-d1E03-1-0120 | 704.190 | OH | a2 | B14 | b2 | C04 | c1 | D01 | d1 | E03 |
| 2-1-d1E03-1-0121 | 705.172 | OH | a2 | B12 | b3 | C08 | c1 | D01 | d1 | E03 |
| 2-1-d1E03-1-0122 | 705.172 | OH | a2 | B12 | b1 | C08 | c2 | D02 | d1 | E03 |
| 2-1-d1E03-1-0 123 | 705.172 | OH | a2 | B11 | b1 | C11 | c2 | D02 | d1 | E03 |
| 2-1-d1E03-1-0124 | 705.186 | OH | a2 | B17 | b2 | C03 | c1 | D01 | d1 | E03 |
| 2-1-d1E03-1-0 125 | 705.206 | OH | a1 | B04 | b2 | C02 | c1 | D01 | d1 | E03 |
| 2-1-d1E03-1-0 126 | 705.206 | OH | a1 | B01 | b2 | C04 | c1 | D01 | d1 | E03 |
| 2-1-d1E03-1-0127 | 705.226 | OH | a1 | B05 | b2 | C01 | c1 | D01 | d1 | E03 |
| 2-1-d1E03-1-0 128 | 706.167 | OH | a2 | B11 | b1 | C10 | c2 | D02 | d1 | E03 |
| 2-1-d1E03-1-0129 | 706.167 | OH | a2 | B12 | b1 | C11 | c2 | D02 | d1 | E03 |
| 2-1-d1E03-1-0130 | 706.201 | OH | a1 | B02 | b2 | C04 | c1 | D01 | d1 | E03 |
| 2-1-d1E03-1-0131 | 706.201 | OH | a1 | B04 | b2 | C03 | c1 | D01 | d1 | E03 |
| 2-1-d1E03-1-0132 | 706.236 | OH | a2 | B13 | b1 | C13 | c1 | D01 | d1 | E03 |
| 2-1-d1E03-1-0133 | 707.162 | OH | a2 | B12 | b1 | C10 | c2 | D02 | d1 | E03 |
| 2-1-d1E03-1-0134 | 707.187 | OH | a1 | B03 | b2 | C06 | c1 | D01 | d1 | E03 |
| 2-1-d1E03-1-0135 | 707.205 | OH | a1 | B06 | b2 | C01 | c1 | D01 | d1 | E03 |
| 2-1-d1E03-1-0136 | 708.171 | OH | a2 | B13 | b3 | C15 | c1 | D01 | d1 | E03 |
| 2-1-d1E03-1-0137 | 708.202 | OH | a2 | B19 | b1 | C08 | c1 | D01 | d1 | E03 |
| 2-1-d1E03-1-0138 | 708.202 | OH | a2 | B11 | b1 | C09 | c1 | D01 | d1 | E03 |
| 2-1-d1E03-1-0139 | 708.204 | OH | a2 | B18 | b2 | C01 | c1 | D01 | d1 | E03 |
| 2-1-d1E03-1-0140 | 709.197 | OH | a2 | B12 | b1 | C09 | c1 | D01 | d1 | E03 |
| 2-1-d1E03-1-0141 | 709.197 | OH | a2 | B19 | b1 | C11 | c1 | D01 | d1 | E03 |
| 2-1-d1E03-1-0142 | 710.133 | OH | a2 | B11 | b3 | C06 | c1 | D01 | d1 | E03 |
| 2-1-d1E03-1-0143 | 710.147 | OH | a2 | B17 | b2 | C06 | c1 | D01 | d1 | E03 |
| 2-1-d1E03-1-0144 | 710.192 | OH | a2 | B19 | b1 | C10 | c1 | D01 | d1 | E03 |
| 2-1-d1E03-1-0145 | 710.202 | OH | a2 | B13 | b1 | C14 | c1 | D01 | d1 | E03 |
| 2-1-d1E03-1-0146 | 710.211 | OH | a2 | B14 | b1 | C13 | c1 | D01 | d1 | E03 |
| 2-1-d1E03-1-0147 | 710.223 | OH | a2 | B13 | b2 | C33 | c3 | D02 | d1 | E03 |
| 2-1-d1E03-1-0148 | 711.128 | OH | a2 | B12 | b3 | C06 | c1 | D01 | d1 | E03 |
| 2-1-d1E03-1-0149 | 711.128 | OH | a2 | B11 | b1 | C12 | c2 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0150 | 711.162 | OH | a1 | B04 | b2 | C06 | c1 | D01 | d1 | E03 |
| 2-1-d1E03-1-0151 | 711.226 | OH | a1 | B01 | b1 | C13 | c1 | D01 | d1 | E03 |
| 2-1-d1E03-1-0152 | 711.252 | OH | a2 | B15 | b2 | C05 | c1 | D01 | d1 | E03 |
| 2-1-d1E03-1-0153 | 712.123 | OH | a2 | B12 | b1 | C12 | c2 | D02 | d1 | E03 |
| 2-1-d1E03-1-0154 | 712.146 | OH | a2 | B14 | b3 | C15 | c1 | D01 | d1 | E03 |
| 2-1-d1E03-1-0155 | 712.222 | OH | a1 | B02 | b1 | C13 | c1 | D01 | d1 | E03 |
| 2-1-d1E03-1-0156 | 713.161 | OH | a1 | B01 | b3 | C15 | c1 | D01 | d1 | E03 |
| 2-1-d1E03-1-0157 | 713.176 | OH | a1 | B07 | b2 | C01 | c1 | D01 | d1 | E03 |
| 2-1-d1E03-1-0158 | 713.231 | OH | a2 | B16 | b2 | C05 | c1 | D01 | d1 | E03 |
| 2-1-d1E03-1-0159 | 714.157 | OH | a1 | B02 | b3 | C15 | c1 | D01 | d1 | E03 |
| 2-1-d1E03-1-0160 | 714.177 | OH | a2 | B14 | b1 | C14 | c1 | D01 | d1 | E03 |
| 2-1-d1E03-1-0161 | 714.198 | OH | a2 | B14 | b2 | C33 | c3 | D02 | d1 | E03 |
| 2-1-d1E03-1-0162 | 714.231 | OH | a2 | B15 | b2 | C04 | c1 | D01 | d1 | E03 |
| 2-1-d1E03-1-0163 | 714.288 | OH | a2 | B13 | b2 | C07 | c1 | D01 | d1 | E03 |
| 2-1-d1E03-1-0164 | 715.153 | OH | a2 | B19 | b1 | C12 | c1 | D01 | d1 | E03 |
| 2-1-d1E03-1-0165 | 715.192 | OH | a1 | B01 | b1 | C14 | c1 | D01 | d1 | E03 |
| 2-1-d1E03-1-0166 | 715.213 | OH | a1 | B01 | b2 | C33 | c3 | D02 | d1 | E03 |
| 2-1-d1E03-1-0167 | 715.246 | OH | a1 | B05 | b2 | C02 | c1 | D01 | d1 | E03 |
| 2-1-d1E03-1-0168 | 716.188 | OH | a1 | B08 | b1 | C08 | c1 | D01 | d1 | E03 |
| 2-1-d1E03-1-0169 | 716.188 | OH | a1 | B02 | b1 | C14 | c1 | D01 | d1 | E03 |
| 2-1-d1E03-1-0170 | 716.209 | OH | a1 | B02 | b2 | C33 | c3 | D02 | d1 | E03 |
| 2-1-d1E03-1-0171 | 716.210 | OH | a2 | B16 | b2 | C04 | c1 | D01 | d1 | E03 |
| 2-1-d1E03-1-0172 | 716.213 | OH | a2 | B11 | b1 | C30 | c3 | D02 | d1 | E03 |
| 2-1-d1E03-1-0173 | 716.242 | OH | a1 | B05 | b2 | C03 | c1 | D01 | d1 | E03 |
| 2-1-d1E03-1-0174 | 716.242 | OH | a1 | B03 | b2 | C05 | c1 | D01 | d1 | E03 |
| 2-1-d1E03-1-0175 | 717.183 | OH | a1 | B08 | b1 | C11 | c1 | D01 | d1 | E03 |
| 2-1-d1E03-1-0176 | 717.208 | OH | a2 | B12 | b1 | C30 | c3 | D02 | d1 | E03 |
| 2-1-d1E03-1-0177 | 717.226 | OH | a1 | B06 | b2 | C02 | c1 | D01 | d1 | E03 |
| 2-1-d1E03-1-0178 | 718.178 | OH | a1 | B08 | b1 | C10 | c1 | D01 | d1 | E03 |
| 2-1-d1E03-1-0179 | 718.192 | OH | a2 | B13 | b3 | C08 | c1 | D01 | d1 | E03 |
| 2-1-d1E03-1-0180 | 718.192 | OH | a2 | B13 | b1 | C08 | c2 | D02 | d1 | E03 |
| 2-1-d1E03-1-0181 | 718.192 | OH | a2 | B11 | b2 | C19 | c3 | D02 | d1 | E03 |
| 2-1-d1E03-1-0182 | 718.221 | OH | a1 | B06 | b2 | C03 | c1 | D01 | d1 | E03 |
| 2-1-d1E03-1-0183 | 718.225 | OH | a2 | B18 | b2 | C02 | c1 | D01 | d1 | E03 |
| 2-1-d1E03-1-0184 | 718.263 | OH | a2 | B14 | b2 | C07 | c1 | D01 | d1 | E03 |
| 2-1-d1E03-1-0185 | 719.187 | OH | a2 | B13 | b1 | C11 | c2 | D02 | d1 | E03 |
| 2-1-d1E03-1-0186 | 719.187 | OH | a2 | B12 | b2 | C19 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0187 | 719.187 | OH | a2 | B11 | b2 | C27 | c3 | D02 | d1 | E03 |
| 2-1-d1E03-1-0188 | 719.201 | OH | a2 | B17 | b2 | C05 | c1 | D01 | d1 | E03 |
| 2-1-d1E03-1-0189 | 719.220 | OH | a2 | B18 | b2 | C03 | c1 | D01 | d1 | E03 |
| 2-1-d1E03-1-0190 | 719.221 | OH | a1 | B03 | b2 | C04 | c1 | D01 | d1 | E03 |
| 2-1-d1E03-1-0191 | 719.278 | OH | a1 | B01 | b2 | C07 | c1 | D01 | d1 | E03 |
| 2-1-d1E03-1-0192 | 720.182 | OH | a2 | B13 | b1 | C10 | c2 | D02 | d1 | E03 |
| 2-1-d1E03-1-0193 | 720.182 | OH | a2 | B12 | b2 | C27 | c3 | D02 | d1 | E03 |
| 2-1-d1E03-1-0194 | 720.217 | OH | a1 | B04 | b2 | C05 | c1 | D01 | d1 | E03 |
| 2-1-d1E03-1-0195 | 720.252 | OH | a2 | B15 | b1 | C13 | c1 | D01 | d1 | E03 |
| 2-1-d1E03-1-0196 | 720.273 | OH | a1 | B02 | b2 | C07 | c1 | D01 | d1 | E03 |
| 2-1-d1E03-1-0197 | 721.203 | OH | a1 | B05 | b2 | C06 | c1 | D01 | d1 | E03 |
| 2-1-d1E03-1-0198 | 722.151 | OH | a2 | B11 | b2 | C01 | c2 | D02 | d1 | E03 |
| 2-1-d1E03-1-0199 | 722.167 | OH | a2 | B14 | b3 | C08 | c1 | D01 | d1 | E03 |
| 2-1-d1E03-1-0200 | 722.167 | OH | a2 | B14 | b1 | C08 | c2 | D02 | d1 | E03 |
| 2-1-d1E03-1-0201 | 722.181 | OH | a2 | B17 | b2 | C04 | c1 | D01 | d1 | E03 |
| 2-1-d1E03-1-0202 | 722.187 | OH | a2 | B15 | b3 | C15 | c1 | D01 | d1 | E03 |
| 2-1-d1E03-1-0203 | 722.217 | OH | a2 | B13 | b1 | C09 | c1 | D01 | d1 | E03 |
| 2-1-d1E03-1-0204 | 722.231 | OH | a2 | B16 | b1 | C13 | c1 | D01 | d1 | E03 |
| 2-1-d1E03-1-0205 | 723.139 | OH | a1 | B08 | b1 | C12 | c1 | D01 | d1 | E03 |
| 2-1-d1E03-1-0206 | 723.146 | OH | a2 | B12 | b2 | C01 | c2 | D02 | d1 | E03 |
| 2-1-d1E03-1-0207 | 723.162 | OH | a2 | B14 | b1 | C11 | c2 | D02 | d1 | E03 |
| 2-1-d1E03-1-0208 | 723.182 | OH | a1 | B06 | b2 | C06 | c1 | D01 | d1 | E03 |
| 2-1-d1E03-1-0209 | 723.182 | OH | a1 | B01 | b3 | C08 | c1 | D01 | d1 | E03 |
| 2-1-d1E03-1-0210 | 723.182 | OH | a1 | B01 | b1 | C08 | c2 | D02 | d1 | E03 |
| 2-1-d1E03-1-0211 | 723.196 | OH | a1 | B07 | b2 | C02 | c1 | D01 | d1 | E03 |
| 2-1-d1E03-1-0212 | 723.196 | OH | a1 | B04 | b2 | C04 | c1 | D01 | d1 | E03 |
| 2-1-d1E03-1-0213 | 724.148 | OH | a2 | B13 | b3 | C06 | c1 | D01 | d1 | E03 |
| 2-1-d1E03-1-0214 | 724.157 | OH | a2 | B14 | b1 | C10 | c2 | D02 | d1 | E03 |
| 2-1-d1E03-1-0215 | 724.166 | OH | a2 | B16 | b3 | C15 | c1 | D01 | d1 | E03 |
| 2-1-d1E03-1-0216 | 724.177 | OH | a1 | B02 | b3 | C08 | c1 | D01 | d1 | E03 |
| 2-1-d1E03-1-0217 | 724.177 | OH | a1 | B02 | b1 | C08 | c2 | D02 | d1 | E03 |
| 2-1-d1E03-1-0218 | 724.177 | OH | a1 | B01 | b1 | C11 | c2 | D02 | d1 | E03 |
| 2-1-d1E03-1-0219 | 724.181 | OH | a2 | B18 | b2 | C06 | c1 | D01 | d1 | E03 |
| 2-1-d1E03-1-0220 | 724.192 | OH | a1 | B07 | b2 | C03 | c1 | D01 | d1 | E03 |
| 2-1-d1E03-1-0221 | 724.197 | OH | a2 | B20 | b1 | C08 | c1 | D01 | d1 | E03 |
| 2-1-d1E03-1-0222 | 724.218 | OH | a2 | B15 | b1 | C14 | c1 | D01 | d1 | E03 |
| 2-1-d1E03-1-0223 | 724.239 | OH | a2 | B15 | b2 | C33 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0224 | 725.144 | OH | a2 | B13 | b1 | C12 | c2 | D02 | d1 | E03 |
| 2-1-d1E03-1-0225 | 725.173 | OH | a1 | B01 | b1 | C10 | c2 | D02 | d1 | E03 |
| 2-1-d1E03-1-0226 | 725.173 | OH | a1 | B02 | b1 | C11 | c2 | D02 | d1 | E03 |
| 2-1-d1E03-1-0227 | 725.192 | OH | a2 | B20 | b1 | C11 | c1 | D01 | d1 | E03 |
| 2-1-d1E03-1-0228 | 725.242 | OH | a1 | B03 | b1 | C13 | c1 | D01 | d1 | E03 |
| 2-1-d1E03-1-0229 | 726.168 | OH | a1 | B02 | b1 | C10 | c2 | D02 | d1 | E03 |
| 2-1-d1E03-1-0230 | 726.176 | OH | a2 | B19 | b2 | C01 | c1 | D01 | d1 | E03 |
| 2-1-d1E03-1-0231 | 726.187 | OH | a2 | B20 | b1 | C10 | c1 | D01 | d1 | E03 |
| 2-1-d1E03-1-0232 | 726.192 | OH | a2 | B14 | b1 | C09 | c1 | D01 | d1 | E03 |
| 2-1-d1E03-1-0233 | 726.197 | OH | a2 | B16 | b1 | C14 | c1 | D01 | d1 | E03 |
| 2-1-d1E03-1-0234 | 726.218 | OH | a2 | B16 | b2 | C33 | c3 | D02 | d1 | E03 |
| 2-1-d1E03-1-0235 | 727.177 | OH | a1 | B03 | b3 | C15 | c1 | D01 | d1 | E03 |
| 2-1-d1E03-1-0236 | 727.208 | OH | a1 | B09 | b1 | C08 | c1 | D01 | d1 | E03 |
| 2-1-d1E03-1-0237 | 727.208 | OH | a1 | B01 | b1 | C09 | c1 | D01 | d1 | E03 |
| 2-1-d1E03-1-0238 | 728.123 | OH | a2 | B14 | b3 | C06 | c1 | D01 | d1 | E03 |
| 2-1-d1E03-1-0239 | 728.202 | OH | a2 | B17 | b1 | C13 | c1 | D01 | d1 | E03 |
| 2-1-d1E03-1-0240 | 728.203 | OH | a1 | B02 | b1 | C09 | c1 | D01 | d1 | E03 |
| 2-1-d1E03-1-0241 | 728.203 | OH | a1 | B09 | b1 | C11 | c1 | D01 | d1 | E03 |
| 2-1-d1E03-1-0242 | 728.303 | OH | a2 | B15 | b2 | C07 | c | D01 | d1 | E03 |
| 2-1-d1E03-1-0243 | 729.119 | OH | a2 | B14 | b1 | C12 | c2 | D02 | d1 | E03 |
| 2-1-d1E03-1-0244 | 729.139 | OH | a1 | B01 | b3 | C06 | c1 | D01 | d1 | E03 |
| 2-1-d1E03-1-0245 | 729.153 | OH | a1 | B07 | b2 | C06 | c1 | D01 | d1 | E03 |
| 2-1-d1E03-1-0246 | 729.198 | OH | a1 | B09 | b1 | C10 | c1 | D01 | d1 | E03 |
| 2-1-d1E03-1-0247 | 729.208 | OH | a1 | B03 | b1 | C14 | c1 | D01 | d1 | E03 |
| 2-1-d1E03-1-0248 | 729.217 | OH | a1 | B04 | b1 | C13 | c1 | D01 | d1 | E03 |
| 2-1-d1E03-1-0249 | 729.229 | OH | a1 | B03 | b2 | C33 | c3 | D02 | d1 | E03 |
| 2-1-d1E03-1-0250 | 730.134 | OH | a1 | B02 | b3 | C06 | c1 | D01 | d1 | E03 |
| 2-1-d1E03-1-0251 | 730.134 | OH | a1 | B01 | b1 | C12 | c2 | D02 | d1 | E03 |
| 2-1-d1E03-1-0252 | 730.137 | OH | a2 | B17 | b3 | C15 | c1 | D01 | d1 | E03 |
| 2-1-d1E03-1-0253 | 730.228 | OH | a2 | B13 | b1 | C30 | c3 | D02 | d1 | E03 |
| 2-1-d1E03-1-0254 | 730.228 | OH | a2 | B11 | b1 | C32 | c3 | D02 | d1 | E03 |
| 2-1-d1E03-1-0255 | 730.257 | OH | a1 | B05 | b2 | C05 | c1 | D01 | d1 | E03 |
| 2-1-d1E03-1-0256 | 730.283 | OH | a2 | B16 | b2 | C07 | c1 | D01 | d1 | E03 |
| 2-1-d1E03-1-0257 | 731.129 | OH | a1 | B02 | b1 | C12 | c2 | D02 | d1 | E03 |
| 2-1-d1E03-1-0258 | 731.148 | OH | a2 | B20 | b1 | C12 | c1 | D01 | d1 | E03 |
| 2-1-d1E03-1-0259 | 731.152 | OH | a1 | B04 | b3 | C15 | c1 | D01 | d1 | E03 |
| 2-1-d1E03-1-0260 | 731.224 | OH | a2 | B12 | b1 | C32 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0261 | 732.168 | OH | a2 | B17 | b1 | C14 | c1 | D01 | d1 | E03 |
| 2-1-d1E03-1-0262 | 732.171 | OH | a2 | B11 | b2 | C02 | c2 | D02 | d1 | E03 |
| 2-1-d1E03-1-0263 | 732.189 | OH | a2 | B17 | b2 | C33 | c3 | D02 | d1 | E03 |
| 2-1-d1E03-1-0264 | 732.208 | OH | a2 | B15 | b3 | C08 | c1 | D01 | d1 | E03 |
| 2-1-d1E03-1-0265 | 732.208 | OH | a2 | B15 | b1 | C08 | c2 | D02 | d1 | E03 |
| 2-1-d1E03-1-0266 | 732.208 | OH | a2 | B13 | b2 | C19 | c3 | D02 | d1 | E03 |
| 2-1-d1E03-1-0267 | 732.208 | OH | a2 | B11 | b2 | C21 | c3 | D02 | d1 | E03 |
| 2-1-d1E03-1-0268 | 732.237 | OH | a1 | B06 | b2 | C05 | c1 | D01 | d1 | E03 |
| 2-1-d1E03-1-0269 | 733.166 | OH | a2 | B12 | b2 | C02 | c2 | D02 | d1 | E03 |
| 2-1-d1E03-1-0270 | 733.166 | OH | a2 | B11 | b2 | C03 | c2 | D02 | d1 | E03 |
| 2-1-d1E03-1-0271 | 733.183 | OH | a1 | B04 | b1 | C14 | c1 | D01 | d1 | E03 |
| 2-1-d1E03-1-0272 | 733.203 | OH | a2 | B15 | b1 | C11 | c2 | D02 | d1 | E03 |
| 2-1-dlE03-1-0273 | 733.203 | OH | a2 | B12 | b2 | C21 | c3 | D02 | d1 | E03 |
| 2-1-d1E03-1-0274 | 733.203 | OH | a2 | B13 | b2 | C27 | c3 | D02 | d1 | E03 |
| 2-1-d1E03-1-0275 | 733.204 | OH | a1 | B04 | b2 | C33 | c3 | D02 | d1 | E03 |
| 2-1-d1E03-1-0276 | 733.236 | OH | a2 | B18 | b2 | C05 | c1 | D01 | d1 | E03 |
| 2-1-d1E03-1-0277 | 733.237 | OH | a1 | B05 | b2 | C04 | c1 | D01 | d1 | E03 |
| 2-1-d1E03-1-0278 | 733.293 | OH | a1 | B03 | b2 | C07 | c1 | D01 | d1 | E03 |
| 2-1-d1E03-1-0279 | 734.159 | OH | a1 | B09 | b1 | C12 | c1 | D01 | d1 | E03 |
| 2-1-d1E03-1-0280 | 734.162 | OH | a1 | B08 | b2 | C01 | c1 | D01 | d1 | E03 |
| 2-1-d1E03-1-0281 | 734.162 | OH | a2 | B12 | b2 | C03 | c2 | D02 | d1 | E03 |
| 2-1-d1E03-1-0282 | 734.187 | OH | a2 | B16 | b3 | C08 | c1 | D01 | d1 | E03 |
| 2-1-d1E03-1-0283 | 734.187 | OH | a2 | B16 | b1 | C08 | c2 | D02 | d1 | E03 |
| 2-1-d1E03-1-0284 | 734.198 | OH | a2 | B15 | b1 | C10 | c2 | D02 | d1 | E03 |
| 2-1-d1E03-1-0285 | 734.203 | OH | a2 | B14 | b1 | C30 | c3 | D02 | d1 | E03 |
| 2-1-d1E03-1-0286 | 735.182 | OH | a2 | B16 | b1 | C11 | c2 | D02 | d1 | E03 |
| 2-1-d1E03-1-0287 | 735.216 | OH | a1 | B06 | b2 | C04 | c1 | D01 | d1 | E03 |
| 2-1-d1E03-1-0288 | 735.219 | OH | a1 | B01 | b1 | C30 | c3 | D02 | d1 | E03 |
| 2-1-d1E03-1-0289 | 736.166 | OH | a2 | B13 | b2 | C01 | c2 | D02 | d1 | E03 |
| 2-1-d1E03-1-0290 | 736.177 | OH | a2 | B16 | b1 | C10 | c2 | D02 | d1 | E03 |
| 2-1-d1E03-1-0291 | 736.183 | OH | a2 | B14 | b2 | C19 | c3 | D02 | d1 | E03 |
| 2-1-d1E03-1-0292 | 736.183 | OH | a2 | B11 | b2 | C23 | c3 | D02 | d1 | E03 |
| 2-1-d1E03-1-0293 | 736.197 | OH | a2 | B19 | b2 | C02 | c1 | D01 | d1 | E03 |
| 2-1-d1E03-1-0294 | 736.214 | OH | a1 | B02 | b1 | C30 | c3 | D02 | d1 | E03 |
| 2-1-d1E03-1-0295 | 736.216 | OH | a2 | B18 | b2 | C04 | c1 | D01 | d1 | E03 |
| 2-1-d1E03-1-0296 | 736.233 | OH | a2 | B15 | b1 | C09 | c1 | D01 | d1 | E03 |
| 2-1-d1E03-1-0297 | 736.253 | OH | a2 | B17 | b2 | C07 | c1 | D01 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0298 | 737.178 | OH | a2 | B12 | b2 | C23 | c3 | D02 | d1 | E03 |
| 2-1-d1E03-1-0299 | 737.178 | OH | a2 | B14 | b2 | C27 | c3 | D02 | d1 | E03 |
| 2-1-d1E03-1-0300 | 737.192 | OH | a2 | B19 | b2 | C03 | c1 | D01 | d1 | E03 |
| 2-1-d1E03-1-0301 | 737.198 | OH | a1 | B03 | b3 | C08 | c1 | D01 | d1 | E03 |
| 2-1-d1E03-1-0302 | 737.198 | OH | a1 | B03 | b1 | C08 | c2 | D02 | d1 | E03 |
| 2-1-d1E03-1-0303 | 737.198 | OH | a1 | B01 | b2 | C19 | c3 | D02 | d1 | E03 |
| 2-1-d1E03-1-0304 | 737.268 | OH | a1 | B04 | b2 | C07 | c1 | D01 | d1 | E03 |
| 2-1-d1E03-1-0305 | 738.128 | OH | a2 | B11 | b2 | C06 | c2 | D02 | d1 | E03 |
| 2-1-d1E03-1-0306 | 738.164 | OH | a2 | B15 | b3 | C06 | c1 | D01 | d1 | E03 |
| 2-1-d1E03-1-0307 | 738.193 | OH | a1 | B03 | b1 | C11 | c2 | D02 | d1 | E03 |
| 2-1-d1E03-1-0308 | 738.193 | OH | a1 | B02 | b2 | C19 | c3 | D02 | d1 | E03 |
| 2-1-d1E03-1-0309 | 738.193 | OH | a1 | B01 | b2 | C27 | c3 | D02 | d1 | E03 |
| 2-1-d1E03-1-0310 | 738.207 | OH | a1 | B07 | b2 | C05 | c1 | D01 | d1 | E03 |
| 2-1-d1E03-1-0311 | 738.212 | OH | a2 | B16 | b1 | C09 | c1 | D01 | d1 | E03 |
| 2-1-d1E03-1-0312 | 739.123 | OH | a2 | B12 | b2 | C06 | c2 | D02 | d1 | E03 |
| 2-1-d1E03-1-0313 | 739.159 | OH | a2 | B15 | b1 | C12 | c2 | D02 | d1 | E03 |
| 2-1-d1E03-1-0314 | 739.188 | OH | a1 | B03 | b1 | C10 | c2 | D02 | d1 | E03 |
| 2-1-d1E03-1-0315 | 739.188 | OH | a1 | B02 | b2 | C27 | c3 | D02 | d1 | E03 |
| 2-1-d1E03-1-0316 | 739.258 | OH | a1 | B05 | b1 | C13 | c1 | D01 | d1 | E03 |
| 2-1-d1E03-1-0317 | 740.141 | OH | a2 | B14 | b2 | C01 | c2 | D02 | d1 | E03 |
| 2-1-d1E03-1-0318 | 740.143 | OH | a2 | B16 | b3 | C06 | c1 | D01 | d1 | E03 |
| 2-1-d1E03-1-0319 | 740.157 | OH | a2 | B17 | b3 | C08 | c1 | D01 | d1 | E03 |
| 2-1-d1E03-1-0320 | 740.157 | OH | a2 | B17 | b1 | C08 | c2 | D02 | d1 | E03 |
| 2-1-d1E03-1-0321 | 741.139 | OH | a2 | B16 | b1 | C12 | c2 | D02 | d1 | E03 |
| 2-1-d1E03-1-0322 | 741.153 | OH | a2 | B17 | b1 | C11 | c2 | D02 | d1 | E03 |
| 2-1-d1E03-1-0323 | 741.156 | OH | a1 | B01 | b2 | C01 | c2 | D02 | d1 | E03 |
| 2-1-d1E03-1-0324 | 741.173 | OH | a1 | B04 | b3 | C08 | c1 | D01 | d1 | E03 |
| 2-1-d1E03-1-0325 | 741.173 | OH | a1 | B04 | b1 | C08 | c2 | D02 | d1 | E03 |
| 2-1-d1E03-1-0326 | 741.187 | OH | a1 | B07 | b2 | C04 | c1 | D01 | d1 | E03 |
| 2-1-d1E03-1-0327 | 741.193 | OH | a1 | B05 | b3 | C15 | c1 | D01 | d1 | E03 |
| 2-1-d1E03-1-0328 | 741.223 | OH | a1 | B03 | b1 | C09 | c1 | D01 | d1 | E03 |
| 2-1-d1E03-1-0329 | 741.237 | OH | a1 | B06 | b1 | C13 | c1 | D01 | d1 | E03 |
| 2-1-d1E03-1-0330 | 742.148 | OH | a2 | B17 | b1 | C10 | c2 | D02 | d1 | E03 |
| 2-1-d1E03-1-0331 | 742.152 | OH | a1 | B02 | b2 | C01 | c2 | D02 | d1 | E03 |
| 2-1-d1E03-1-0332 | 742.153 | OH | a2 | B19 | b2 | C06 | c1 | D01 | d1 | E03 |
| 2-1-d1E03-1-0333 | 742.168 | OH | a1 | B04 | b1 | C11 | c2 | D02 | d1 | E03 |
| 2-1-d1E03-1-0334 | 742.171 | OH | a2 | B20 | b2 | C01 | c1 | D01 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0335 | 742.236 | OH | a2 | B18 | b1 | C13 | c1 | D01 | d1 | E03 |
| 2-1-d1E03-1-0336 | 743.154 | OH | a1 | B03 | b3 | C06 | c1 | D01 | d1 | E03 |
| 2-1-d1E03-1-0337 | 743.163 | OH | a1 | B04 | b1 | C10 | c2 | D02 | d1 | E03 |
| 2-1-d1E03-1-0338 | 743.172 | OH | a1 | B06 | b3 | C15 | c1 | D01 | d1 | E03 |
| 2-1-d1E03-1-0339 | 743.203 | OH | a1 | B10 | b1 | C08 | c1 | D01 | d1 | E03 |
| 2-1-d1E03-1-0340 | 743.224 | OH | a1 | B05 | b1 | C14 | c1 | D01 | d1 | E03 |
| 2-1-d1E03-1-0341 | 743.245 | OH | a1 | B05 | b2 | C33 | c3 | D02 | d1 | E03 |
| 2-1-d1E03-1-0342 | 744.149 | OH | a1 | B03 | b1 | C12 | c2 | D02 | d1 | E03 |
| 2-1-d1E03-1-0343 | 744.171 | OH | a2 | B18 | b3 | C15 | c1 | D01 | d1 | E03 |
| 2-1-d1E03-1-0344 | 744.182 | OH | a1 | B08 | b2 | C02 | c1 | D01 | d1 | E03 |
| 2-1-d1E03-1-0345 | 744.183 | OH | a2 | B17 | b1 | C09 | c1 | D01 | d1 | E03 |
| 2-1-d1E03-1-0346 | 744.198 | OH | a1 | B10 | b1 | C11 | c1 | D01 | d1 | E03 |
| 2-1-d1E03-1-0347 | 744.244 | OH | a2 | B15 | b1 | C30 | c3 | D02 | d1 | E03 |
| 2-1-d1E03-1-0348 | 744.244 | OH | a2 | B11 | b1 | C29 | c3 | D02 | d1 | E03 |
| 2-1-d1E03-1-0349 | 744.244 | OH | a2 | B13 | b1 | C32 | c3 | D02 | d1 | E03 |
| 2-1-d1E03-1-0350 | 745.178 | OH | a1 | B08 | b2 | C03 | c1 | D01 | d1 | E03 |
| 2-1-d1E03-1-0351 | 745.182 | OH | a1 | B09 | b2 | C01 | c1 | D01 | d1 | E03 |
| 2-1-d1E03-1-0352 | 745.193 | OH | a1 | B10 | b1 | C10 | c1 | D01 | d1 | E03 |
| 2-1-d1E03-1-0353 | 745.198 | OH | a1 | B04 | b1 | C09 | c1 | D01 | d1 | E03 |
| 2-1-d1E03-1-0354 | 745.203 | OH | a1 | B06 | b1 | C14 | c1 | D01 | d1 | E03 |
| 2-1-d1E03-1-0355 | 745.224 | OH | a1 | B06 | b2 | C33 | c3 | D02 | d1 | E03 |
| 2-1-d1E03-1-0356 | 745.239 | OH | a2 | B12 | b1 | C29 | c3 | D02 | d1 | E03 |
| 2-1-d1E03-1-0357 | 746.114 | OH | a2 | B17 | b3 | C06 | c1 | D01 | d1 | E03 |
| 2-1-d1E03-1-0358 | 746.187 | OH | a2 | B13 | b2 | C02 | c2 | D02 | d1 | E03 |
| 2-1-d1E03-1-0359 | 746.202 | OH | a2 | B18 | b1 | C14 | c1 | D01 | d1 | E03 |
| 2-1-d1E03-1-0360 | 746.223 | OH | a2 | B15 | b2 | C19 | c3 | D02 | d1 | E03 |
| 2-1-d1E03-1-0361 | 746.223 | OH | a2 | B13 | b2 | C21 | c3 | D02 | d1 | E03 |
| 2-1-d1E03-1-0362 | 746.223 | OH | a2 | B16 | b1 | C30 | c3 | D02 | d1 | E03 |
| 2-1-d1E03-1-0363 | 746.223 | OH | a2 | B18 | b2 | C33 | c3 | D02 | d1 | E03 |
| 2-1-d1E03-1-0364 | 747.109 | OH | a2 | B17 | b1 | C12 | c2 | D02 | d1 | E03 |
| 2-1-d1E03-1-0365 | 747.129 | OH | a1 | B04 | b3 | C06 | c1 | D01 | d1 | E03 |
| 2-1-d1E03-1-0366 | 747.182 | OH | a2 | B13 | b2 | C03 | c2 | D02 | d1 | E03 |
| 2-1-d1E03-1-0367 | 747.182 | OH | a2 | B11 | b2 | C05 | c2 | D02 | d1 | E03 |
| 2-1-d1E03-1-0368 | 747.208 | OH | a1 | B07 | b1 | C13 | c1 | D01 | d1 | E03 |
| 2-1-d1E03-1-0369 | 747.219 | OH | a2 | B15 | b2 | C27 | c3 | D02 | d1 | E03 |
| 2-1-d1E03-1-0370 | 747.309 | OH | a1 | B05 | b2 | C07 | c1 | D01 | d1 | E03 |
| 2-1-d1E03-1-0371 | 748.124 | OH | a1 | B04 | b1 | C12 | c2 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0372 | 748.177 | OH | a2 | B12 | b2 | C05 | c2 | D02 | d1 | E03 |
| 2-1-d1E03-1-0373 | 748.203 | OH | a2 | B16 | b2 | C19 | c3 | D02 | d1 | E03 |
| 2-1-d1E03-1-0374 | 748.203 | OH | a2 | B11 | b2 | C25 | c3 | D02 | d1 | E03 |
| 2-1-d1E03-1-0375 | 748.219 | OH | a2 | B14 | b1 | C32 | c3 | D02 | d1 | E03 |
| 2-1-d1E03-1-0376 | 749.143 | OH | a1 | B07 | b3 | C15 | c1 | D01 | d1 | E03 |
| 2-1-d1E03-1-0377 | 749.198 | OH | a2 | B12 | b2 | C25 | c3 | D02 | d1 | E03 |
| 2-1-d1E03-1-0378 | 749.198 | OH | a2 | B16 | b2 | C27 | c3 | D02 | d1 | E03 |
| 2-1-d1E03-1-0379 | 749.234 | OH | a1 | B03 | b1 | C30 | c3 | D02 | d1 | E03 |
| 2-1-d1E03-1-0380 | 749.234 | OH | a1 | B01 | b1 | C32 | c3 | D02 | d1 | E03 |
| 2-1-d1E03-1-0381 | 749.288 | OH | a1 | B06 | b2 | C07 | c1 | D01 | d1 | E03 |
| 2-1-d1E03-1-0382 | 750.139 | OH | a1 | B08 | b2 | C06 | c1 | D01 | d1 | E03 |
| 2-1-d1E03-1-0383 | 750.154 | OH | a1 | B10 | b1 | C12 | c1 | D01 | d1 | E03 |
| 2-1-d1E03-1-0384 | 750.162 | OH | a2 | B14 | b2 | C02 | c2 | D02 | d1 | E03 |
| 2-1-d1E03-1-0385 | 750.162 | OH | a2 | B11 | b2 | C04 | c2 | D02 | d1 | E03 |
| 2-1-d1E03-1-0386 | 750.182 | OH | a2 | B15 | b2 | C01 | c2 | D02 | d1 | E03 |
| 2-1-d1E03-1-0387 | 750.198 | OH | a2 | B14 | b2 | C21 | c3 | D02 | d1 | E03 |
| 2-1-d1E03-1-0388 | 750.198 | OH | a2 | B13 | b2 | C23 | c3 | D02 | d1 | E03 |
| 2-1-d1E03-1-0389 | 750.229 | OH | a1 | B02 | b1 | C32 | c3 | D02 | d1 | E03 |
| 2-1-d1E03-1-0390 | 750.288 | OH | a2 | B18 | b2 | C07 | c1 | D01 | d1 | E03 |
| 2-1-d1E03-1-0391 | 751.157 | OH | a2 | B12 | b2 | C04 | c2 | D02 | d1 | E03 |
| 2-1-d1E03-1-0392 | 751.157 | OH | a2 | B14 | b2 | C03 | c2 | D02 | d1 | E03 |
| 2-1-d1E03-1-0393 | 751.173 | OH | a1 | B07 | b1 | C14 | c1 | D01 | d1 | E03 |
| 2-1-d1E03-1-0394 | 751.177 | OH | a1 | B01 | b2 | C02 | c2 | D02 | d1 | E03 |
| 2-1-d1E03-1-0395 | 751.195 | OH | a1 | B07 | b2 | C33 | c3 | D02 | d1 | E03 |
| 2-1-d1E03-1-0396 | 751.208 | OH | a2 | B19 | b2 | C05 | c1 | D01 | d1 | E03 |
| 2-1-d1E03-1-0397 | 751.213 | OH | a1 | B05 | b3 | C08 | c1 | D01 | d1 | E03 |
| 2-1-d1E03-1-0398 | 751.213 | OH | a1 | B05 | b1 | C08 | c2 | D02 | d1 | E03 |
| 2-1-d1E03-1-0399 | 751.213 | OH | a1 | B03 | b2 | C19 | c3 | D02 | d1 | E03 |
| 2-1-d1E03-1-0400 | 751.213 | OH | a1 | B01 | b2 | C21 | c3 | D02 | d1 | E03 |
| 2-1-d1E03-1-0401 | 752.143 | OH | a2 | B13 | b2 | C06 | c2 | D02 | d1 | E03 |
| 2-1-d1E03-1-0402 | 752.161 | OH | a2 | B16 | b2 | C01 | c2 | D02 | d1 | E03 |
| 2-1-d1E03-1-0403 | 752.172 | OH | a1 | B02 | b2 | C02 | c2 | D02 | d1 | E03 |
| 2-1-d1E03-1-0404 | 752.172 | OH | a1 | B01 | b2 | C03 | c2 | D02 | d1 | E03 |
| 2-1-d1E03-1-0405 | 752.192 | OH | a2 | B20 | b2 | C02 | c1 | D01 | d1 | E03 |
| 2-1-d1E03-1-0406 | 752.194 | OH | a2 | B17 | b1 | C30 | c3 | D02 | d1 | E03 |
| 2-1-d1E03-1-0407 | 752.209 | OH | a1 | B05 | b1 | C11 | c2 | D02 | d1 | E03 |
| 2-1-d1E03-1-0408 | 752.209 | OH | a1 | B02 | b2 | C21 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0409 | 752.209 | OH | a1 | B03 | b2 | C27 | c3 | D02 | d1 | E03 |
| 2-1-d1E03-1-0410 | 753.168 | OH | a1 | B02 | b2 | C03 | c2 | D02 | d1 | E03 |
| 2-1-d1E03-1-0411 | 753.187 | OH | a2 | B20 | b2 | C03 | c1 | D01 | d1 | E03 |
| 2-1-d1E03-1-0412 | 753.193 | OH | a1 | B06 | b3 | C08 | c1 | D01 | d1 | E03 |
| 2-1-d1E03-1-0413 | 753.193 | OH | a1 | B06 | b1 | C08 | c2 | D02 | d1 | E03 |
| 2-1-d1E03-1-0414 | 753.204 | OH | a1 | B05 | b1 | C10 | c2 | D02 | d1 | E03 |
| 2-1-d1E03-1-0415 | 753.209 | OH | a1 | B04 | b1 | C30 | c3 | D02 | d1 | E03 |
| 2-1-d1E03-1-0416 | 754.173 | OH | a2 | B17 | b2 | C19 | c3 | D02 | d1 | E03 |
| 2-1-d1E03-1-0417 | 754.173 | OH | a2 | B14 | b2 | C23 | c3 | D02 | d1 | E03 |
| 2-1-d1E03-1-0418 | 754.187 | OH | a2 | B19 | b2 | C04 | c1 | D01 | d1 | E03 |
| 2-1-d1E03-1-0419 | 754.188 | OH | a1 | B06 | b1 | C11 | c2 | D02 | d1 | E03 |
| 2-1-d1E03-1-0420 | 754.192 | OH | a2 | B18 | b3 | C08 | c1 | D01 | d1 | E03 |
| 2-1-d1E03-1-0421 | 754.192 | OH | a2 | B18 | b1 | C08 | c2 | D02 | d1 | E03 |
| 2-1-d1E03-1-0422 | 755.168 | OH | a2 | B17 | b2 | C27 | c3 | D02 | d1 | E03 |
| 2-1-d1E03-1-0423 | 755.172 | OH | a1 | B03 | b2 | C01 | c2 | D02 | d1 | E03 |
| 2-1-d1E03-1-0424 | 755.183 | OH | a1 | B06 | b1 | C10 | c2 | D02 | d1 | E03 |
| 2-1-d1E03-1-0425 | 755.187 | OH | a2 | B18 | b1 | C11 | c2 | D02 | d1 | E03 |
| 2-1-d1E03-1-0426 | 755.188 | OH | a1 | B04 | b2 | C19 | c3 | D02 | d1 | E03 |
| 2-1-d1E03-1-0427 | 755.188 | OH | a1 | B01 | b2 | C23 | c3 | D02 | d1 | E03 |
| 2-1-d1E03-1-0428 | 755.203 | OH | a1 | B09 | b2 | C02 | c1 | D01 | d1 | E03 |
| 2-1-d1E03-1-0429 | 755.239 | OH | a1 | B05 | b1 | C09 | c1 | D01 | d1 | E03 |
| 2-1-d1E03-1-0430 | 755.259 | OH | a1 | B07 | b2 | C07 | c1 | D01 | d1 | E03 |
| 2-1-d1E03-1-0431 | 756.118 | OH | a2 | B14 | b2 | C06 | c2 | D02 | d1 | E03 |
| 2-1-d1E03-1-0432 | 756.182 | OH | a2 | B18 | b1 | C10 | c2 | D02 | d1 | E03 |
| 2-1-d1E03-1-0433 | 756.182 | OH | a2 | B11 | b1 | C13 | c2 | D02 | d1 | E03 |
| 2-1-d1E03-1-0434 | 756.184 | OH | a1 | B02 | b2 | C23 | c3 | D02 | d1 | E03 |
| 2-1-d1E03-1-0435 | 756.184 | OH | a1 | B04 | b2 | C27 | c3 | D02 | d1 | E03 |
| 2-1-d1E03-1-0436 | 756.198 | OH | a1 | B09 | b2 | C03 | c1 | D01 | d1 | E03 |
| 2-1-d1E03-1-0437 | 757.133 | OH | a1 | B01 | b2 | C06 | c2 | D02 | d1 | E03 |
| 2-1-d1E03-1-0438 | 757.170 | OH | a1 | B05 | b3 | C06 | c1 | D01 | d1 | E03 |
| 2-1-d1E03-1-0439 | 757.178 | OH | a2 | B12 | b1 | C13 | c2 | D02 | d1 | E03 |
| 2-1-d1E03-1-0440 | 757.218 | OH | a1 | B06 | b1 | C09 | c1 | D01 | d1 | E03 |
| 2-1-d1E03-1-0441 | 758.117 | OH | a2 | B11 | b3 | C15 | c2 | D02 | d1 | E03 |
| 2-1-d1E03-1-0442 | 758.129 | OH | a1 | B02 | b2 | C06 | c2 | D02 | d1 | E03 |
| 2-1-d1E03-1-0443 | 758.132 | OH | a2 | B17 | b2 | C01 | c2 | D02 | d1 | E03 |
| 2-1-d1E03-1-0444 | 758.148 | OH | a2 | B20 | b2 | C06 | c1 | D01 | d1 | E03 |
| 2-1-d1E03-1-0445 | 758.165 | OH | a1 | B05 | b1 | C12 | c2 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0446 | 758.187 | OH | a2 | B11 | b3 | C16 | c1 | D01 | d1 | E03 |
| 2-1-d1E03-1-0447 | 758.217 | OH | a2 | B18 | b1 | C09 | c1 | D01 | d1 | E03 |
| 2-1-d1E03-1-0448 | 758.260 | OH | a2 | B13 | b1 | C29 | c3 | D02 | d1 | E03 |
| 2-1-d1E03-1-0449 | 758.260 | OH | a2 | B15 | b1 | C32 | c3 | D02 | d1 | E03 |
| 2-1-d1E03-1-0450 | 759.113 | OH | a2 | B12 | b3 | C15 | c2 | D02 | d1 | E03 |
| 2-1-d1E03-1-0451 | 759.147 | OH | a1 | B04 | b2 | C01 | c2 | D02 | d1 | E03 |
| 2-1-d1E03-1-0452 | 759.149 | OH | a1 | B06 | b3 | C06 | c1 | D01 | d1 | E03 |
| 2-1-d1E03-1-0453 | 759.163 | OH | a1 | B07 | b3 | C08 | c1 | D01 | d1 | E03 |
| 2-1-d1E03-1-0454 | 759.163 | OH | a1 | B07 | b1 | C08 | c2 | D02 | d1 | E03 |
| 2-1-d1E03-1-0455 | 759.182 | OH | a2 | B12 | b3 | C16 | c1 | D01 | d1 | E03 |
| 2-1-d1E03-1-0456 | 759.193 | OH | a1 | B08 | b2 | C05 | c1 | D01 | d1 | E03 |
| 2-1-d1E03-1-0457 | 760.144 | OH | a1 | B06 | b1 | C12 | c2 | D02 | d1 | E03 |
| 2-1-d1E03-1-0458 | 760.148 | OH | a2 | B18 | b3 | C06 | c1 | D01 | d1 | E03 |
| 2-1-d1E03-1-0459 | 760.148 | OH | a2 | B11 | b3 | C17 | c1 | D01 | d1 | E03 |
| 2-1-d1E03-1-0460 | 760.148 | OH | a2 | B11 | b1 | C14 | c2 | D02 | d1 | E03 |
| 2-1-d1E03-1-0461 | 760.159 | OH | a1 | B07 | b1 | C11 | c2 | D02 | d1 | E03 |
| 2-1-d1E03-1-0462 | 760.203 | OH | a2 | B15 | b2 | C02 | c2 | D02 | d1 | E03 |
| 2-1-d1E03-1-0463 | 760.208 | OH | a2 | B19 | b1 | C13 | c1 | D01 | d1 | E03 |
| 2-1-d1E03-1-0464 | 760.239 | OH | a2 | B15 | b2 | C21 | c3 | D02 | d1 | E03 |
| 2-1-d1E03-1-0465 | 760.239 | OH | a2 | B16 | b1 | C32 | c3 | D02 | d1 | E03 |
| 2-1-d1E03-1-0466 | 761.144 | OH | a2 | B12 | b3 | C17 | c1 | D01 | d1 | E03 |
| 2-1-d1E03-1-0467 | 761.144 | OH | a2 | B18 | b1 | C12 | c2 | D02 | d1 | E03 |
| 2-1-d1E03-1-0468 | 761.144 | OH | a2 | B12 | b1 | C14 | c2 | D02 | d1 | E03 |
| 2-1-d1E03-1-0469 | 761.154 | OH | a1 | B07 | b1 | C10 | c2 | D02 | d1 | E03 |
| 2-1-d1E03-1-0470 | 761.159 | OH | a1 | B09 | b2 | C06 | c1 | D01 | d1 | E03 |
| 2-1-d1E03-1-0471 | 761.177 | OH | a1 | B10 | b2 | C01 | c1 | D01 | d1 | E03 |
| 2-1-d1E03-1-0472 | 761.198 | OH | a2 | B15 | b2 | C03 | c2 | D02 | d1 | E03 |
| 2-1-d1E03-1-0473 | 761.198 | OH | a2 | B13 | b2 | C05 | c2 | D02 | d1 | E03 |
| 2-1-d1E03-1-0474 | 762.143 | OH | a2 | B19 | b3 | C15 | c1 | D01 | d1 | E03 |
| 2-1-d1E03-1-0475 | 762.173 | OH | a1 | B08 | b2 | C04 | c1 | D01 | d1 | E03 |
| 2-1-d1E03-1-0476 | 762.182 | OH | a2 | B16 | b2 | C02 | c2 | D02 | d1 | E03 |
| 2-1-d1E03-1-0477 | 762.218 | OH | a2 | B16 | b2 | C21 | c3 | D02 | d1 | E03 |
| 2-1-d1E03-1-0478 | 762.218 | OH | a2 | B13 | b2 | C25 | c3 | D02 | d1 | E03 |
| 2-1-d1E03-1-0479 | 762.235 | OH | a2 | B14 | b1 | C29 | c3 | D02 | d1 | E03 |
| 2-1-d1E03-1-0480 | 763.177 | OH | a2 | B16 | b2 | C03 | c2 | D02 | d1 | E03 |
| 2-1-d1E03-1-0481 | 763.189 | OH | a1 | B07 | b1 | C09 | c1 | D01 | d1 | E03 |
| 2-1-d1E03-1-0482 | 763.250 | OH | a1 | B05 | b1 | C30 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0483 | 763.250 | OH | a1 | B01 | b1 | C29 | c3 | D02 | d1 | E03 |
| 2-1-d1E03-1-0484 | 763.250 | OH | a1 | B03 | b1 | C32 | c3 | D02 | d1 | E03 |
| 2-1-d1E03-1-0485 | 764.174 | OH | a2 | B19 | b1 | C14 | c1 | D01 | d1 | E03 |
| 2-1-d1E03-1-0486 | 764.177 | OH | a2 | B13 | b2 | C04 | c2 | D02 | d1 | E03 |
| 2-1-d1E03-1-0487 | 764.195 | OH | a2 | B19 | b2 | C33 | c3 | D02 | d1 | E03 |
| 2-1-d1E03-1-0488 | 764.214 | OH | a2 | B15 | b2 | C23 | c3 | D02 | d1 | E03 |
| 2-1-d1E03-1-0489 | 764.234 | OH | a2 | B11 | b2 | C07 | c2 | D02 | d1 | E03 |
| 2-1-d1E03-1-0490 | 764.245 | OH | a1 | B02 | b1 | C29 | c3 | D02 | d1 | E03 |
| 2-1-d1E03-1-0491 | 765.120 | OH | a1 | B07 | b3 | C06 | c1 | D01 | d1 | E03 |
| 2-1-d1E03-1-0492 | 765.173 | OH | a2 | B14 | b2 | C05 | c2 | D02 | d1 | E03 |
| 2-1-d1E03-1-0493 | 765.193 | OH | a1 | B03 | b2 | C02 | c2 | D02 | d1 | E03 |
| 2-1-d1E03-1-0494 | 765.229 | OH | a2 | B12 | b2 | C07 | c2 | D02 | d1 | E03 |
| 2-1-d1E03-1-0495 | 765.229 | OH | a1 | B05 | b2 | C19 | c3 | D02 | d1 | E03 |
| 2-1-d1E03-1-0496 | 765.229 | OH | a1 | B03 | b2 | C21 | c3 | D02 | d1 | E03 |
| 2-1-d1E03-1-0497 | 765.229 | OH | a1 | B06 | b1 | C30 | c3 | D02 | d1 | E03 |
| 2-1-d1E03-1-0498 | 766.115 | OH | a1 | B07 | b1 | C12 | c2 | D02 | d1 | E03 |
| 2-1-d1E03-1-0499 | 766.159 | OH | a2 | B15 | b2 | C06 | c2 | D02 | d1 | E03 |
| 2-1-d1E03-1-0500 | 766.188 | OH | a1 | B03 | b2 | C03 | c2 | D02 | d1 | E03 |
| 2-1-d1E03-1-0501 | 766.188 | OH | a1 | B01 | b2 | C05 | c2 | D02 | d1 | E03 |
| 2-1-d1E03-1-0502 | 766.193 | OH | a2 | B16 | b2 | C23 | c3 | D02 | d1 | E03 |
| 2-1-d1E03-1-0503 | 766.193 | OH | a2 | B14 | b2 | C25 | c3 | D02 | d1 | E03 |
| 2-1-d1E03-1-0504 | 766.210 | OH | a2 | B17 | b1 | C32 | c3 | D02 | d1 | E03 |
| 2-1-d1E03-1-0505 | 766.224 | OH | a1 | B05 | b2 | C27 | c3 | D02 | d1 | E03 |
| 2-1-d1E03-1-0506 | 766.228 | OH | a2 | B18 | b1 | C30 | c3 | D02 | d1 | E03 |
| 2-1-d1E03-1-0507 | 767.183 | OH | a1 | B02 | b2 | C05 | c2 | D02 | d1 | E03 |
| 2-1-d1E03-1-0508 | 767.203 | OH | a2 | B20 | b2 | C05 | c1 | D01 | d1 | E03 |
| 2-1-d1E03-1-0509 | 767.208 | OH | a1 | B06 | b2 | C19 | c3 | D02 | d1 | E03 |
| 2-1-d1E03-1-0510 | 767.208 | OH | a1 | B01 | b2 | C25 | c3 | D02 | d1 | E03 |
| 2-1-d1E03-1-0511 | 767.225 | OH | a1 | B04 | b1 | C32 | c3 | D02 | d1 | E03 |
| 2-1-d1E03-1-0512 | 768.138 | OH | a2 | B16 | b2 | C06 | c2 | D02 | d1 | E03 |
| 2-1-d1E03-1-0513 | 768.138 | OH | a2 | B11 | b3 | C08 | c2 | D02 | d1 | E03 |
| 2-1-d1E03-1-0514 | 768.152 | OH | a2 | B17 | b2 | C02 | c2 | D02 | d1 | E03 |
| 2-1-d1E03-1-0515 | 768.152 | OH | a2 | B14 | b2 | C04 | c2 | D02 | d1 | E03 |
| 2-1-d1E03-1-0516 | 768.189 | OH | a2 | B17 | b2 | C21 | c3 | D02 | d1 | E03 |
| 2-1-d1E03-1-0517 | 768.194 | OH | a1 | B08 | b1 | C13 | c1 | D01 | d1 | E03 |
| 2-1-d1E03-1-0518 | 768.204 | OH | a1 | B02 | b2 | C25 | c3 | D02 | d1 | E03 |
| 2-1-d1E03-1-0519 | 768.204 | OH | a1 | B06 | b2 | C27 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0520 | 768.208 | OH | a2 | B18 | b2 | C19 | c3 | D02 | d1 | E03 |
| 2-1-d1E03-1-0521 | 768.259 | OH | a2 | B19 | b2 | C07 | c1 | D01 | d1 | E03 |
| 2-1-d1E03-1-0522 | 769.133 | OH | a2 | B12 | b3 | C08 | c2 | D02 | d1 | E03 |
| 2-1-d1E03-1-0523 | 769.148 | OH | a2 | B17 | b2 | C03 | c2 | D02 | d1 | E03 |
| 2-1-d1E03-1-0524 | 769.168 | OH | a1 | B04 | b2 | C02 | c2 | D02 | d1 | E03 |
| 2-1-d1E03-1-0525 | 769.168 | OH | a1 | B01 | b2 | C04 | c2 | D02 | d1 | E03 |
| 2-1-d1E03-1-0526 | 769.188 | OH | a1 | B05 | b2 | C01 | c2 | D02 | d1 | E03 |
| 2-1-d1E03-1-0527 | 769.203 | OH | a2 | B18 | b2 | C27 | c3 | D02 | d1 | E03 |
| 2-1-d1E03-1-0528 | 769.204 | OH | a1 | B04 | b2 | C21 | c3 | D02 | d1 | E03 |
| 2-1-d1E03-1-0529 | 769.204 | OH | a1 | B03 | b2 | C23 | c3 | D02 | d1 | E03 |
| 2-1-d1E03-1-0530 | 770.129 | OH | a1 | B08 | b3 | C15 | c1 | D01 | d1 | E03 |
| 2-1-d1E03-1-0531 | 770.163 | OH | a1 | B02 | b2 | C04 | c2 | D02 | d1 | E03 |
| 2-1-d1E03-1-0532 | 770.163 | OH | a1 | B04 | b2 | C03 | c2 | D02 | d1 | E03 |
| 2-1-d1E03-1-0533 | 770.182 | OH | a2 | B20 | b2 | C04 | c1 | D01 | d1 | E03 |
| 2-1-d1E03-1-0534 | 770.198 | OH | a2 | B13 | b1 | C13 | c2 | D02 | d1 | E03 |
| 2-1-d1E03-1-0535 | 770.213 | OH | a1 | B09 | b2 | C05 | c1 | D01 | d1 | E03 |
| 2-1-d1E03-1-0536 | 771.149 | OH | a1 | B03 | b2 | C06 | c2 | D02 | d1 | E03 |
| 2-1-d1E03-1-0537 | 771.167 | OH | a1 | B06 | b2 | C01 | c2 | D02 | d1 | E03 |
| 2-1-d1E03-1-0538 | 771.197 | OH | a1 | B10 | b2 | C02 | c1 | D01 | d1 | E03 |
| 2-1-d1E03-1-0539 | 771.200 | OH | a1 | B07 | b1 | C30 | c3 | D02 | d1 | E03 |
| 2-1-d1E03-1-0540 | 772.133 | OH | a2 | B13 | b3 | C15 | c2 | D02 | d1 | E03 |
| 2-1-d1E03-1-0541 | 772.16 | OH | a1 | B08 | b1 | C14 | c1 | D01 | d1 | E03 |
| 2-1-d1E03-1-0542 | 772.164 | OH | a2 | B19 | b3 | C08 | c1 | D01 | d1 | E03 |
| 2-1-d1E03-1-0543 | 772.164 | OH | a2 | B19 | b1 | C08 | c2 | D02 | d1 | E03 |
| 2-1-d1E03-1-0544 | 772.164 | OH | a2 | B11 | b1 | C09 | c2 | D02 | d1 | E03 |
| 2-1-d1E03-1-0545 | 772.164 | OH | a2 | B17 | b2 | C23 | c3 | D02 | d1 | E03 |
| 2-1-d1E03-1-0546 | 772.166 | OH | a2 | B18 | b2 | C01 | c2 | D02 | d1 | E03 |
| 2-1-d1E03-1-0547 | 772.181 | OH | a1 | B08 | b2 | C33 | c3 | D02 | d1 | E03 |
| 2-1-d1E03-1-0548 | 772.193 | OH | a1 | B10 | b2 | C03 | c1 | D01 | d1 | E03 |
| 2-1-d1E03-1-0549 | 772.203 | OH | a2 | B13 | b3 | C16 | c1 | D01 | d1 | E03 |
| 2-1-d1E03-1-0550 | 772.239 | OH | a2 | B11 | b1 | C31 | c3 | D02 | d1 | E03 |
| 2-1-d1E03-1-0551 | 772.275 | OH | a2 | B15 | b1 | C29 | c3 | D02 | d1 | E03 |
| 2-1-d1E03-1-0552 | 773.159 | OH | a2 | B12 | b1 | C09 | c2 | D02 | d1 | E03 |
| 2-1-d1E03-1-0553 | 773.159 | OH | a2 | B19 | b1 | C11 | c2 | D02 | d1 | E03 |
| 2-1-d1E03-1-0554 | 773.179 | OH | a1 | B07 | b2 | C19 | c3 | D02 | d1 | E03 |
| 2-1-d1E03-1-0555 | 773.179 | OH | a1 | B04 | b2 | C23 | c3 | D02 | d1 | E03 |
| 2-1-d1E03-1-0556 | 773.193 | OH | a1 | B09 | b2 | C04 | c1 | D01 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0557 | 773.234 | OH | a2 | B12 | b1 | C31 | c3 | D02 | d1 | E03 |
| 2-1-d1E03-1-0558 | 774.095 | OH | a2 | B11 | b3 | C06 | c2 | D02 | d1 | E03 |
| 2-1-d1E03-1-0559 | 774.109 | OH | a2 | B17 | b2 | C06 | c2 | D02 | d1 | E03 |
| 2-1-d1E03-1-0560 | 774.154 | OH | a2 | B19 | b1 | C10 | c2 | D02 | d1 | E03 |
| 2-1-d1E03-1-0561 | 774.164 | OH | a2 | B13 | b3 | C17 | c1 | D01 | d1 | E03 |
| 2-1-d1E03-1-0562 | 774.164 | OH | a2 | B13 | b1 | C14 | c2 | D02 | d1 | E03 |
| 2-1-d1E03-1-0563 | 774.173 | OH | a2 | B14 | b1 | C13 | c2 | D02 | d1 | E03 |
| 2-1-d1E03-1-0564 | 774.174 | OH | a1 | B07 | b2 | C27 | c3 | D02 | d1 | E03 |
| 2-1-d1E03-1-0565 | 774.255 | OH | a2 | B16 | b1 | C29 | c3 | D02 | d1 | E03 |
| 2-1-d1E03-1-0566 | 775.090 | OH | a2 | B12 | b3 | C06 | c2 | D02 | d1 | E03 |
| 2-1-d1E03-1-0567 | 775.124 | OH | a1 | B04 | b2 | C06 | c2 | D02 | d1 | E03 |
| 2-1-d1E03-1-0568 | 775.159 | OH | a2 | B11 | b3 | C18 | c1 | D01 | d1 | E03 |
| 2-1-d1E03-1-0569 | 775.188 | OH | a1 | B01 | b1 | C13 | c2 | D02 | d1 | E03 |
| 2-1-d1E03-1-0570 | 775.213 | OH | a2 | B15 | b2 | C05 | c2 | D02 | d1 | E03 |
| 2-1-d1E03-1-0571 | 776.108 | OH | a2 | B14 | b3 | C15 | c2 | D02 | d1 | E03 |
| 2-1-d1E03-1-0572 | 776.155 | OH | a2 | B12 | b3 | C18 | c1 | D01 | d1 | E03 |
| 2-1-d1E03-1-0573 | 776.177 | OH | a2 | B14 | b3 | C16 | c1 | D01 | d1 | E03 |
| 2-1-d1E03-1-0574 | 776.184 | OH | a1 | B02 | b1 | C13 | c2 | D02 | d1 | E03 |
| 2-1-d1E03-1-0575 | 776.189 | OH | a2 | B19 | b1 | C09 | c1 | D01 | d1 | E03 |
| 2-1-d1E03-1-0576 | 776.203 | OH | a2 | B20 | b1 | C13 | c1 | D01 | d1 | E03 |
| 2-1-d1E03-1-0577 | 776.234 | OH | a2 | B15 | b2 | C25 | c3 | D02 | d1 | E03 |
| 2-1-d1E03-1-0578 | 776.245 | OH | a1 | B08 | b2 | C07 | c1 | D01 | d1 | E03 |
| 2-1-d1E03-1-0579 | 777.123 | OH | a1 | B01 | b3 | C15 | c2 | D02 | d1 | E03 |
| 2-1-d1E03-1-0580 | 777.137 | OH | a1 | B07 | b2 | C01 | c2 | D02 | d1 | E03 |
| 2-1-d1E03-1-0581 | 777.154 | OH | a1 | B10 | b2 | C06 | c1 | D01 | d1 | E03 |
| 2-1-d1E03-1-0582 | 777.193 | OH | a1 | B01 | b3 | C16 | c1 | D01 | d1 | E03 |
| 2-1-d1E03-1-0583 | 777.193 | OH | a2 | B16 | b2 | C05 | c2 | D02 | d1 | E03 |
| 2-1-d1E03-1-0584 | 777.265 | OH | a1 | B03 | b1 | C29 | c3 | D02 | d1 | E03 |
| 2-1-d1E03-1-0585 | 777.265 | OH | a1 | B05 | b1 | C32 | c3 | D02 | d1 | E03 |
| 2-1-d1E03-1-0586 | 778.119 | OH | a1 | B02 | b3 | C15 | c2 | D02 | d1 | E03 |
| 2-1-d1E03-1-0587 | 778.120 | OH | a2 | B19 | b3 | C06 | c1 | D01 | d1 | E03 |
| 2-1-d1E03-1-0588 | 778.138 | OH | a2 | B20 | b3 | C15 | c1 | D01 | d1 | E03 |
| 2-1-d1E03-1-0589 | 778.139 | OH | a2 | B14 | b3 | C17 | c1 | D01 | d1 | E03 |
| 2-1-d1E03-1-0590 | 778.139 | OH | a2 | B14 | b1 | C14 | c2 | D02 | d1 | E03 |
| 2-1-d1E03-1-0591 | 778.188 | OH | a1 | B02 | b3 | C16 | c1 | D01 | d1 | E03 |
| 2-1-d1E03-1-0592 | 778.193 | OH | a2 | B15 | b2 | C04 | c2 | D02 | d1 | E03 |
| 2-1-d1E03-1-0593 | 778.213 | OH | a2 | B16 | b2 | C25 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0594 | 778.249 | OH | a2 | B13 | b2 | C07 | c2 | D02 | d1 | E03 |
| 2-1-d1E03-1-0595 | 779.115 | OH | a2 | B19 | b1 | C12 | c2 | D02 | d1 | E03 |
| 2-1-d1E03-1-0596 | 779.154 | OH | a1 | B01 | b3 | C17 | c1 | D01 | d1 | E03 |
| 2-1-d1E03-1-0597 | 779.154 | OH | a1 | B01 | b1 | C14 | c2 | D02 | d1 | E03 |
| 2-1-d1E03-1-0598 | 779.208 | OH | a1 | B05 | b2 | C02 | c2 | D02 | d1 | E03 |
| 2-1-d1E03-1-0599 | 779.214 | OH | a1 | B09 | b1 | C13 | c1 | D01 | d1 | E03 |
| 2-1-d1E03-1-0600 | 779.245 | OH | a1 | B05 | b2 | C21 | c3 | D02 | d1 | E03 |
| 2-1-d1E03-1-0601 | 779.245 | OH | a1 | B06 | b1 | C32 | c3 | D02 | d1 | E03 |
| 2-1-d1E03-1-0602 | 780.149 | OH | a1 | B02 | b3 | C17 | c1 | D01 | d1 | E03 |
| 2-1-d1E03-1-0603 | 780.149 | OH | a1 | B08 | b3 | C08 | c1 | D01 | d1 | E03 |
| 2-1-d1E03-1-0604 | 780.149 | OH | a1 | B08 | b1 | C08 | c2 | D02 | d1 | E03 |
| 2-1-d1E03-1-0605 | 780.149 | OH | a1 | B02 | b1 | C14 | c2 | D02 | d1 | E03 |
| 2-1-d1E03-1-0606 | 780.169 | OH | a2 | B20 | b1 | C14 | c1 | D01 | d1 | E03 |
| 2-1-d1E03-1-0607 | 780.172 | OH | a2 | B16 | b2 | C04 | c2 | D02 | d1 | E03 |
| 2-1-d1E03-1-0608 | 780.190 | OH | a2 | B20 | b2 | C33 | c3 | D02 | d1 | E03 |
| 2-1-d1E03-1-0609 | 780.204 | OH | a1 | B05 | b2 | C03 | c2 | D02 | d1 | E03 |
| 2-1-d1E03-1-0610 | 780.204 | OH | a1 | B03 | b2 | C05 | c2 | D02 | d1 | E03 |
| 2-1-d1E03-1-0611 | 780.225 | OH | a2 | B17 | b1 | C29 | c3 | D02 | d1 | E03 |
| 2-1-d1E03-1-0612 | 780.244 | OH | a2 | B18 | b1 | C32 | c3 | D02 | d1 | E03 |
| 2-1-d1E03-1-0613 | 781.145 | OH | a1 | B08 | b1 | C11 | c2 | D02 | d1 | E03 |
| 2-1-d1E03-1-0614 | 781.149 | OH | a1 | B09 | b3 | C15 | c1 | D01 | d1 | E03 |
| 2-1-d1E03-1-0615 | 781.188 | OH | a1 | B06 | b2 | C02 | c2 | D02 | d1 | E03 |
| 2-1-d1E03-1-0616 | 781.224 | OH | a1 | B06 | b2 | C21 | c3 | D02 | d1 | E03 |
| 2-1-d1E03-1-0617 | 781.224 | OH | a1 | B03 | b2 | C25 | c3 | D02 | d1 | E03 |
| 2-1-d1E03-1-0618 | 781.240 | OH | a1 | B04 | b1 | C29 | c3 | D02 | d1 | E03 |
| 2-1-d1E03-1-0619 | 782.140 | OH | a1 | B08 | b1 | C10 | c2 | D02 | d1 | E03 |
| 2-1-d1E03-1-0620 | 782.154 | OH | a2 | B13 | b3 | C08 | c2 | D02 | d1 | E03 |
| 2-1-d1E03-1-0621 | 782.183 | OH | a1 | B06 | b2 | C03 | c2 | D02 | d1 | E03 |
| 2-1-d1E03-1-0622 | 782.187 | OH | a2 | B18 | b2 | C02 | c2 | D02 | d1 | E03 |
| 2-1-d1E03-1-0623 | 782.223 | OH | a2 | B18 | b2 | C21 | c3 | D02 | d1 | E03 |
| 2-1-d1E03-1-0624 | 782.224 | OH | a2 | B14 | b2 | C07 | c2 | D02 | d1 | E03 |
| 2-1-d1E03-1-0625 | 783.163 | OH | a2 | B17 | b2 | C05 | c2 | D02 | d1 | E03 |
| 2-1-d1E03-1-0626 | 783.180 | OH | a1 | B09 | b1 | C14 | c1 | D01 | d1 | E03 |
| 2-1-d1E03-1-0627 | 783.182 | OH | a2 | B18 | b2 | C03 | c2 | D02 | d1 | E03 |
| 2-1-d1E03-1-0628 | 783.183 | OH | a1 | B03 | b2 | C04 | c2 | D02 | d1 | E03 |
| 2-1-d1E03-1-0629 | 783.201 | OH | a1 | B09 | b2 | C33 | c3 | D02 | d1 | E03 |
| 2-1-d1E03-1-0630 | 783.220 | OH | a1 | B05 | b2 | C23 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0631 | 783.240 | OH | a1 | B01 | b2 | C07 | c2 | D02 | d1 | E03 |
| 2-1-d1E03-1-0632 | 784.175 | OH | a1 | B08 | b1 | C09 | c1 | D01 | d1 | E03 |
| 2-1-d1E03-1-0633 | 784.179 | OH | a1 | B04 | b2 | C05 | c2 | D02 | d1 | E03 |
| 2-1-d1E03-1-0634 | 784.184 | OH | a2 | B17 | b2 | C25 | c3 | D02 | d1 | E03 |
| 2-1-d1E03-1-0635 | 784.200 | OH | a2 | B19 | b1 | C30 | c3 | D02 | d1 | E03 |
| 2-1-d1E03-1-0636 | 784.214 | OH | a2 | B15 | b1 | C13 | c2 | D02 | d1 | E03 |
| 2-1-d1E03-1-0637 | 784.235 | OH | a1 | B02 | b2 | C07 | c2 | D02 | d1 | E03 |
| 2-1-d1E03-1-0638 | 784.254 | OH | a2 | B20 | b2 | C07 | c1 | D01 | d1 | E03 |
| 2-1-d1E03-1-0639 | 785.165 | OH | a1 | B05 | b2 | C06 | c2 | D02 | d1 | E03 |
| 2-1-d1E03-1-0640 | 785.199 | OH | a1 | B06 | b2 | C23 | c3 | D02 | d1 | E03 |
| 2-1-d1E03-1-0641 | 785.199 | OH | a1 | B04 | b2 | C25 | c3 | D02 | d1 | E03 |
| 2-1-d1E03-1-0642 | 785.215 | OH | a1 | B07 | b1 | C32 | c3 | D02 | d1 | E03 |
| 2-1-d1E03-1-0643 | 786.106 | OH | a1 | B08 | b3 | C06 | c1 | D01 | d1 | E03 |
| 2-1-d1E03-1-0644 | 786.129 | OH | a2 | B14 | b3 | C08 | c2 | D02 | d1 | E03 |
| 2-1-d1E03-1-0645 | 786.143 | OH | a2 | B17 | b2 | C04 | c2 | D02 | d1 | E03 |
| 2-1-d1E03-1-0646 | 786.149 | OH | a2 | B15 | b3 | C15 | c2 | D02 | d1 | E03 |
| 2-1-d1E03-1-0647 | 786.179 | OH | a2 | B13 | b1 | C09 | c2 | D02 | d1 | E03 |
| 2-1-d1E03-1-0648 | 786.179 | OH | a2 | B19 | b2 | C19 | c3 | D02 | d1 | E03 |
| 2-1-d1E03-1-0649 | 786.193 | OH | a2 | B16 | b1 | C13 | c2 | D02 | d1 | E03 |
| 2-1-d1E03-1-0650 | 786.198 | OH | a2 | B18 | b2 | C23 | c3 | D02 | d1 | E03 |
| 2-1-d1E03-1-0651 | 786.208 | OH | a1 | B10 | b2 | C05 | c1 | D01 | d1 | E03 |
| 2-1-d1E03-1-0652 | 786.218 | OH | a2 | B15 | b3 | C16 | c1 | D01 | d1 | E03 |
| 2-1-d1E03-1-0653 | 786.255 | OH | a2 | B13 | b1 | C31 | c3 | D02 | d1 | E03 |
| 2-1-d1E03-1-0654 | 787.101 | OH | a1 | B08 | b1 | C12 | c2 | D02 | d1 | E03 |
| 2-1-d1E03-1-0655 | 787.144 | OH | a1 | B06 | b2 | C06 | c2 | D02 | d1 | E03 |
| 2-1-d1E03-1-0656 | 787.144 | OH | a1 | B01 | b3 | C08 | c2 | D02 | d1 | E03 |
| 2-1-d1E03-1-0657 | 787.158 | OH | a1 | B07 | b2 | C02 | c2 | D02 | d1 | E03 |
| 2-1-d1E03-1-0658 | 787.158 | OH | a1 | B04 | b2 | C04 | c2 | D02 | d1 | E03 |
| 2-1-d1E03-1-0659 | 787.175 | OH | a2 | B19 | b2 | C27 | c3 | D02 | d1 | E03 |
| 2-1-d1E03-1-0660 | 787.195 | OH | a1 | B07 | b2 | C21 | c3 | D02 | d1 | E03 |
| 2-1-d1E03-1-0661 | 787.265 | OH | a1 | B09 | b2 | C07 | c1 | D01 | d1 | E03 |
| 2-1-d1E03-1-0662 | 788.11 | OH | a2 | B13 | b3 | C06 | c2 | D02 | d1 | E03 |
| 2-1-d1E03-1-0663 | 788.128 | OH | a2 | B16 | b3 | C15 | c2 | D02 | d1 | E03 |
| 2-1-d1E03-1-0664 | 788.139 | OH | a1 | B02 | b3 | C08 | c2 | D02 | d1 | E03 |
| 2-1-d1E03-1-0665 | 788.143 | OH | a2 | B18 | b2 | C06 | c2 | D02 | d1 | E03 |
| 2-1-d1E03-1-0666 | 788.153 | OH | a1 | B07 | b2 | C03 | c2 | D02 | d1 | E03 |
| 2-1-d1E03-1-0667 | 788.159 | OH | a2 | B20 | b3 | C08 | c1 | D01 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0668 | 788.159 | OH | a2 | B20 | b1 | C08 | c2 | D02 | d1 | E03 |
| 2-1-d1E03-1-0669 | 788.180 | OH | a2 | B15 | b3 | C17 | c1 | D01 | d1 | E03 |
| 2-1-d1E03-1-0670 | 788.180 | OH | a2 | B15 | b1 | C14 | c2 | D02 | d1 | E03 |
| 2-1-d1E03-1-0671 | 788.197 | OH | a2 | B16 | b3 | C16 | c1 | D01 | d1 | E03 |
| 2-1-d1E03-1-0672 | 789.154 | OH | a2 | B20 | b1 | C11 | c2 | D02 | d1 | E03 |
| 2-1-d1E03-1-0673 | 789.175 | OH | a2 | B13 | b3 | C18 | c1 | D01 | d1 | E03 |
| 2-1-d1E03-1-0674 | 789.188 | OH | a1 | B10 | b2 | C04 | c1 | D01 | d1 | E03 |
| 2-1-d1E03-1-0675 | 789.204 | OH | a1 | B03 | b1 | C13 | c2 | D02 | d1 | E03 |
| 2-1-d1E03-1-0676 | 790.138 | OH | a2 | B19 | b2 | C01 | c2 | D02 | d1 | E03 |
| 2-1-d1E03-1-0677 | 790.149 | OH | a2 | B20 | b1 | C10 | c2 | D02 | d1 | E03 |
| 2-1-d1E03-1-0678 | 790.154 | OH | a2 | B14 | b1 | C09 | c2 | D02 | d1 | E03 |
| 2-1-d1E03-1-0679 | 790.159 | OH | a2 | B16 | b3 | C17 | c1 | D01 | d1 | E03 |
| 2-1-d1E03-1-0680 | 790.159 | OH | a2 | B16 | b1 | C14 | c2 | D02 | d1 | E03 |
| 2-1-d1E03-1-0681 | 790.230 | OH | a2 | B14 | b1 | C31 | c3 | D02 | d1 | E03 |
| 2-1-d1E03-1-0682 | 791.139 | OH | a1 | B03 | b3 | C15 | c2 | D02 | d1 | E03 |
| 2-1-d1E03-1-0683 | 791.170 | OH | a1 | B09 | b3 | C08 | c1 | D01 | d1 | E03 |
| 2-1-d1E03-1-0684 | 791.170 | OH | a1 | B09 | b1 | C08 | c2 | D02 | d1 | E03 |
| 2-1-d1E03-1-0685 | 791.170 | OH | a1 | B01 | b1 | C09 | c2 | D02 | d1 | E03 |
| 2-1-d1E03-1-0686 | 791.170 | OH | a1 | B07 | b2 | C23 | c3 | D02 | d1 | E03 |
| 2-1-d1E03-1-0687 | 791.208 | OH | a1 | B03 | b3 | C16 | c1 | D01 | d1 | E03 |
| 2-1-d1E03-1-0688 | 791.245 | OH | a1 | B01 | b1 | C31 | c3 | D02 | d1 | E03 |
| 2-1-d1E03-1-0689 | 791.281 | OH | a1 | B05 | b1 | C29 | c3 | D02 | d1 | E03 |
| 2-1-d1E03-1-0690 | 792.085 | OH | a2 | B14 | b3 | C06 | c2 | D02 | d1 | E03 |
| 2-1-d1E03-1-0691 | 792.164 | OH | a2 | B17 | b1 | C13 | c2 | D02 | d1 | E03 |
| 2-1-d1E03-1-0692 | 792.165 | OH | a1 | B02 | b1 | C09 | c2 | D02 | d1 | E03 |
| 2-1-d1E03-1-0693 | 792.165 | OH | a1 | B09 | b1 | C11 | c2 | D02 | d1 | E03 |
| 2-1-d1E03-1-0694 | 792.184 | OH | a2 | B20 | b1 | C09 | c1 | D01 | d1 | E03 |
| 2-1-d1E03-1-0695 | 792.186 | OH | a1 | B08 | b1 | C30 | c3 | D02 | d1 | E03 |
| 2-1-d1E03-1-0696 | 792.240 | OH | a1 | B02 | b1 | C31 | c3 | D02 | d1 | E03 |
| 2-1-d1E03-1-0697 | 792.265 | OH | a2 | B15 | b2 | C07 | c2 | D02 | d1 | E03 |
| 2-1-d1E03-1-0698 | 793.100 | OH | a1 | B01 | b3 | C06 | c2 | D02 | d1 | E03 |
| 2-1-d1E03-1-0699 | 793.115 | OH | a1 | B07 | b2 | C06 | c2 | D02 | d1 | E03 |
| 2-1-d1E03-1-0700 | 793.150 | OH | a2 | B14 | b3 | C18 | c1 | D01 | d1 | E03 |
| 2-1-d1E03-1-0701 | 793.160 | OH | a1 | B09 | b1 | C10 | c2 | D02 | d1 | E03 |
| 2-1-d1E03-1-0702 | 793.170 | OH | a1 | B03 | b3 | C17 | c1 | D01 | d1 | E03 |
| 2-1-d1E03-1-0703 | 793.170 | OH | a1 | B03 | b1 | C14 | c2 | D02 | d1 | E03 |
| 2-1-d1E03-1-0704 | 793.179 | OH | a1 | B04 | b1 | C13 | c2 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0705 | 793.260 | OH | a1 | B06 | b1 | C29 | c3 | D02 | d1 | E03 |
| 2-1-d1E03-1-0706 | 794.096 | OH | a1 | B02 | b3 | C06 | c2 | D02 | d1 | E03 |
| 2-1-d1E03-1-0707 | 794.099 | OH | a2 | B17 | b3 | C15 | c2 | D02 | d1 | E03 |
| 2-1-d1E03-1-0708 | 794.115 | OH | a2 | B20 | b3 | C06 | c1 | D01 | d1 | E03 |
| 2-1-d1E03-1-0709 | 794.165 | OH | a1 | B01 | b3 | C18 | c1 | D01 | d1 | E03 |
| 2-1-d1E03-1-0710 | 794.165 | OH | a1 | B08 | b2 | C19 | c3 | D02 | d1 | E03 |
| 2-1-d1E03-1-0711 | 794.168 | OH | a2 | B17 | b3 | C16 | c1 | D01 | d1 | E03 |
| 2-1-d1E03-1-0712 | 794.219 | OH | a1 | B05 | b2 | C05 | c2 | D02 | d1 | E03 |
| 2-1-d1E03-1-0713 | 794.244 | OH | a2 | B16 | b2 | C07 | c2 | D02 | d1 | E03 |
| 2-1-d1E03-1-0714 | 794.260 | OH | a2 | B18 | b1 | C29 | c3 | D02 | d1 | E03 |
| 2-1-d1E03-1-0715 | 795.110 | OH | a2 | B20 | b1 | C12 | c2 | D02 | d1 | E03 |
| 2-1-d1E03-1-0716 | 795.114 | OH | a1 | B04 | b3 | C15 | c2 | D02 | d1 | E03 |
| 2-1-d1E03-1-0717 | 795.160 | OH | a1 | B02 | b3 | C18 | c1 | D01 | d1 | E03 |
| 2-1-d1E03-1-0718 | 795.160 | OH | a1 | B08 | b2 | C27 | c3 | D02 | d1 | E03 |
| 2-1-d1E03-1-0719 | 795.183 | OH | a1 | B04 | b3 | C16 | c1 | D01 | d1 | E03 |
| 2-1-d1E03-1-0720 | 795.195 | OH | a1 | B09 | b1 | C09 | c1 | D01 | d1 | E03 |
| 2-1-d1E03-1-0721 | 795.209 | OH | a1 | B10 | b1 | C13 | c1 | D01 | d1 | E03 |
| 2-1-d1E03-1-0722 | 795.240 | OH | a1 | B05 | b2 | C25 | c3 | D02 | d1 | E03 |
| 2-1-d1E03-1-0723 | 796.130 | OH | a2 | B17 | b3 | C17 | c1 | D01 | d1 | E03 |
| 2-1-d1E03-1-0724 | 796.130 | OH | a2 | B17 | b1 | C14 | c2 | D02 | d1 | E03 |
| 2-1-d1E03-1-0725 | 796.170 | OH | a2 | B15 | b3 | C08 | c2 | D02 | d1 | E03 |
| 2-1-d1E03-1-0726 | 796.199 | OH | a1 | B06 | b2 | C05 | c2 | D02 | d1 | E03 |
| 2-1-d1E03-1-0727 | 797.126 | OH | a1 | B09 | b3 | C06 | c1 | D01 | d1 | E03 |
| 2-1-d1E03-1-0728 | 797.144 | OH | a1 | B10 | b3 | C15 | c1 | D01 | d1 | E03 |
| 2-1-d1E03-1-0729 | 797.145 | OH | a1 | B04 | b3 | C17 | c1 | D01 | d1 | E03 |
| 2-1-d1E03-1-0730 | 797.145 | OH | a1 | B04 | b1 | C14 | c2 | D02 | d1 | E03 |
| 2-1-d1E03-1-0731 | 797.198 | OH | a2 | B18 | b2 | C05 | c2 | D02 | d1 | E03 |
| 2-1-d1E03-1-0732 | 797.199 | OH | a1 | B05 | b2 | C04 | c2 | D02 | d1 | E03 |
| 2-1-d1E03-1-0733 | 797.219 | OH | a1 | B06 | b2 | C25 | c3 | D02 | d1 | E03 |
| 2-1-d1E03-1-0734 | 797.255 | OH | a1 | B03 | b2 | C07 | c2 | D02 | d1 | E03 |
| 2-1-d1E03-1-0735 | 798.121 | OH | a1 | B09 | b1 | C12 | c2 | D02 | d1 | E03 |
| 2-1-d1E03-1-0736 | 798.124 | OH | a1 | B08 | b2 | C01 | c2 | D02 | d1 | E03 |
| 2-1-d1E03-1-0737 | 798.149 | OH | a2 | B16 | b3 | C08 | c2 | D02 | d1 | E03 |
| 2-1-d1E03-1-0738 | 798.216 | OH | a2 | B19 | b1 | C32 | c3 | D02 | d1 | E03 |
| 2-1-d1E03-1-0739 | 798.218 | OH | a2 | B18 | b2 | C25 | c3 | D02 | d1 | E03 |
| 2-1-d1E03-1-0740 | 799.175 | OH | a1 | B10 | b1 | C14 | c1 | D01 | d1 | E03 |
| 2-1-d1E03-1-0741 | 799.178 | OH | a1 | B06 | b2 | C04 | c2 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0742 | 799.196 | OH | a1 | B10 | b2 | C33 | c3 | D02 | d1 | E03 |
| 2-1-d1E03-1-0743 | 799.231 | OH | a1 | B07 | b1 | C29 | c3 | D02 | d1 | E03 |
| 2-1-d1E03-1-0744 | 800.159 | OH | a2 | B19 | b2 | C02 | c2 | D02 | d1 | E03 |
| 2-1-d1E03-1-0745 | 800.177 | OH | a2 | B18 | b2 | C04 | c2 | D02 | d1 | E03 |
| 2-1-d1E03-1-0746 | 800.195 | OH | a2 | B15 | b1 | C09 | c2 | D02 | d1 | E03 |
| 2-1-d1E03-1-0747 | 800.195 | OH | a2 | B19 | b2 | C21 | c3 | D02 | d1 | E03 |
| 2-1-d1E03-1-0748 | 800.195 | OH | a2 | B20 | b1 | C30 | c3 | D02 | d1 | E03 |
| 2-1-d1E03-1-0749 | 800.215 | OH | a2 | B17 | b2 | C07 | c2 | D02 | d1 | E03 |
| 2-1-d1E03-1-0750 | 800.270 | OH | a2 | B15 | b1 | C31 | c3 | D02 | d1 | E03 |
| 2-1-d1E03-1-0751 | 801.154 | OH | a2 | B19 | b2 | C03 | c2 | D02 | d1 | E03 |
| 2-1-d1E03-1-0752 | 801.160 | OH | a1 | B03 | b3 | C08 | c2 | D02 | d1 | E03 |
| 2-1-d1E03-1-0753 | 801.230 | OH | a1 | B04 | b2 | C07 | c2 | D02 | d1 | E03 |
| 2-1-d1E03-1-0754 | 802.126 | OH | a2 | B15 | b3 | C06 | c2 | D02 | d1 | E03 |
| 2-1-d1E03-1-0755 | 802.169 | OH | a1 | B07 | b2 | C05 | c2 | D02 | d1 | E03 |
| 2-1-d1E03-1-0756 | 802.174 | OH | a2 | B16 | b1 | C09 | c2 | D02 | d1 | E03 |
| 2-1-d1E03-1-0757 | 802.174 | OH | a2 | B20 | b2 | C19 | c3 | D02 | d1 | E03 |
| 2-1-d1E03-1-0758 | 802.249 | OH | a2 | B16 | b1 | C31 | c3 | D02 | d1 | E03 |
| 2-1-d1E03-1-0759 | 803.170 | OH | a2 | B20 | b2 | C27 | c3 | D02 | d1 | E03 |
| 2-1-d1E03-1-0760 | 803.190 | OH | a1 | B07 | b2 | C25 | c3 | D02 | d1 | E03 |
| 2-1-d1E03-1-0761 | 803.191 | OH | a2 | B15 | b3 | C18 | c1 | D01 | d1 | E03 |
| 2-1-d1E03-1-0762 | 803.206 | OH | a1 | B09 | b1 | C30 | c3 | D02 | d1 | E03 |
| 2-1-d1E03-1-0763 | 803.220 | OH | a1 | B05 | b1 | C13 | c2 | D02 | d1 | E03 |
| 2-1-d1E03-1-0764 | 803.260 | OH | a1 | B10 | b2 | C07 | c1 | D01 | d1 | E03 |
| 2-1-d1E03-1-0765 | 804.105 | OH | a2 | B16 | b3 | C06 | c2 | D02 | d1 | E03 |
| 2-1-d1E03-1-0766 | 804.119 | OH | a2 | B17 | b3 | C08 | c2 | D02 | d1 | E03 |
| 2-1-d1E03-1-0767 | 804.170 | OH | a2 | B19 | b2 | C23 | c3 | D02 | d1 | E03 |
| 2-1-d1E03-1-0768 | 805.135 | OH | a1 | B04 | b3 | C08 | c2 | D02 | d1 | E03 |
| 2-1-d1E03-1-0769 | 805.149 | OH | a1 | B07 | b2 | C04 | c2 | D02 | d1 | E03 |
| 2-1-d1E03-1-0770 | 805.155 | OH | a1 | B05 | b3 | C15 | c2 | D02 | d1 | E03 |
| 2-1-d1E03-1-0771 | 805.170 | OH | a2 | B16 | b3 | C18 | c1 | D01 | d1 | E03 |
| 2-1-d1E03-1-0772 | 805.185 | OH | a1 | B03 | b1 | C09 | c2 | D02 | d1 | E03 |
| 2-1-d1E03-1-0773 | 805.185 | OH | a1 | B09 | b2 | C19 | c3 | D02 | d1 | E03 |
| 2-1-d1E03-1-0774 | 805.199 | OH | a1 | B06 | b1 | C13 | c2 | D02 | d1 | E03 |
| 2-1-d1E03-1-0775 | 805.224 | OH | a1 | B05 | b3 | C16 | c1 | D01 | d1 | E03 |
| 2-1-d1E03-1-0776 | 805.260 | OH | a1 | B03 | b1 | C31 | c3 | D02 | d1 | E03 |
| 2-1-d1E03-1-0777 | 806.115 | OH | a2 | B19 | b2 | C06 | c2 | D02 | d1 | E03 |
| 2-1-d1E03-1-0778 | 806.133 | OH | a2 | B20 | b2 | C01 | c2 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0779 | 806.180 | OH | a1 | B09 | b2 | C27 | c3 | D02 | d1 | E03 |
| 2-1-d1E03-1-0780 | 806.198 | OH | a2 | B18 | b1 | C13 | c2 | D02 | d1 | E03 |
| 2-1-d1E03-1-0781 | 806.201 | OH | a1 | B08 | b1 | C32 | c3 | D02 | d1 | E03 |
| 2-1-d1E03-1-0782 | 807.116 | OH | a1 | B03 | b3 | C06 | c2 | D02 | d1 | E03 |
| 2-1-d1E03-1-0783 | 807.134 | OH | a1 | B06 | b3 | C15 | c2 | D02 | d1 | E03 |
| 2-1-d1E03-1-0784 | 807.164 | OH | a1 | B10 | b3 | C08 | c1 | D01 | d1 | E03 |
| 2-1-d1E03-1-0785 | 807.164 | OH | a1 | B10 | b1 | C08 | c2 | D02 | d1 | E03 |
| 2-1-d1E03-1-0786 | 807.186 | OH | a1 | B05 | b3 | C17 | c1 | D01 | d1 | E03 |
| 2-1-d1E03-1-0787 | 807.186 | OH | a1 | B05 | b1 | C14 | c2 | D02 | d1 | E03 |
| 2-1-d1E03-1-0788 | 807.203 | OH | a1 | B06 | b3 | C16 | c1 | D01 | d1 | E03 |
| 2-1-d1E03-1-0789 | 808.133 | OH | a2 | B18 | b3 | C15 | c2 | D02 | d1 | E03 |
| 2-1-d1E03-1-0790 | 808.144 | OH | a1 | B08 | b2 | C02 | c2 | D02 | d1 | E03 |
| 2-1-d1E03-1-0791 | 808.145 | OH | a2 | B17 | b1 | C09 | c2 | D02 | d1 | E03 |
| 2-1-d1E03-1-0792 | 808.160 | OH | a1 | B10 | b1 | C11 | c2 | D02 | d1 | E03 |
| 2-1-d1E03-1-0793 | 808.181 | OH | a1 | B03 | b3 | C18 | c1 | D01 | d1 | E03 |
| 2-1-d1E03-1-0794 | 808.181 | OH | a1 | B08 | b2 | C21 | c3 | D02 | d1 | E03 |
| 2-1-d1E03-1-0795 | 808.203 | OH | a2 | B18 | b3 | C16 | c1 | D01 | d1 | E03 |
| 2-1-d1E03-1-0796 | 808.220 | OH | a2 | B17 | b1 | C31 | c3 | D02 | d1 | E03 |
| 2-1-d1E03-1-0797 | 809.140 | OH | a1 | B08 | b2 | C03 | c2 | D02 | d1 | E03 |
| 2-1-d1E03-1-0798 | 809.144 | OH | a1 | B09 | b2 | C01 | c2 | D02 | d1 | E03 |
| 2-1-d1E03-1-0799 | 809.155 | OH | a1 | B10 | b1 | C10 | c2 | D02 | d1 | E03 |
| 2-1-d1E03-1-0800 | 809.160 | OH | a1 | B04 | b1 | C09 | c2 | D02 | d1 | E03 |
| 2-1-d1E03-1-0801 | 809.165 | OH | a1 | B06 | b3 | C17 | c1 | D01 | d1 | E03 |
| 2-1-d1E03-1-0802 | 809.165 | OH | a1 | B06 | b1 | C14 | c2 | D02 | d1 | E03 |
| 2-1-d1E03-1-0803 | 809.235 | OH | a1 | B04 | b1 | C31 | c3 | D02 | d1 | E03 |
| 2-1-d1E03-1-0804 | 810.076 | OH | a2 | B17 | b3 | C06 | c2 | D02 | d1 | E03 |
| 2-1-d1E03-1-0805 | 810.164 | OH | a2 | B18 | b3 | C17 | c1 | D01 | d1 | E03 |
| 2-1-d1E03-1-0806 | 810.164 | OH | a2 | B18 | b1 | C14 | c2 | D02 | d1 | E03 |
| 2-1-d1E03-1-0807 | 811.091 | OH | a1 | B04 | b3 | C06 | c2 | D02 | d1 | E03 |
| 2-1-d1E03-1-0808 | 811.140 | OH | a2 | B17 | b3 | C18 | c1 | D01 | d1 | E03 |
| 2-1-d1E03-1-0809 | 811.169 | OH | a1 | B07 | b1 | C13 | c2 | D02 | d1 | E03 |
| 2-1-d1E03-1-0810 | 811.190 | OH | a1 | B10 | b1 | C09 | c1 | D01 | d1 | E03 |
| 2-1-d1E03-1-0811 | 811.271 | OH | a1 | B05 | b2 | C07 | c2 | D02 | d1 | E03 |
| 2-1-d1E03-1-0812 | 812.156 | OH | a1 | B04 | b3 | C18 | c1 | D01 | d1 | E03 |
| 2-1-d1E03-1-0813 | 812.156 | OH | a1 | B08 | b2 | C23 | c3 | D02 | d1 | E03 |
| 2-1-d1E03-1-0814 | 812.231 | OH | a2 | B19 | b1 | C29 | c3 | D02 | d1 | E03 |
| 2-1-d1E03-1-0815 | 813.104 | OH | a1 | B07 | b3 | C15 | c2 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0816 | 813.121 | OH | a1 | B10 | b3 | C06 | c1 | D01 | d1 | E03 |
| 2-1-d1E03-1-0817 | 813.174 | OH | a1 | B07 | b3 | C16 | c1 | D01 | d1 | E03 |
| 2-1-d1E03-1-0818 | 813.250 | OH | a1 | B06 | b2 | C07 | c2 | D02 | d1 | E03 |
| 2-1-d1E03-1-0819 | 814.101 | OH | a1 | B08 | b2 | C06 | c2 | D02 | d1 | E03 |
| 2-1-d1E03-1-0820 | 814.116 | OH | a1 | B10 | b1 | C12 | c2 | D02 | d1 | E03 |
| 2-1-d1E03-1-0821 | 814.211 | OH | a2 | B20 | b1 | C32 | c3 | D02 | d1 | E03 |
| 2-1-d1E03-1-0822 | 814.249 | OH | a2 | B18 | b2 | C07 | c2 | D02 | d1 | E03 |
| 2-1-d1E03-1-0823 | 815.135 | OH | a1 | B07 | b3 | C17 | c1 | D01 | d1 | E03 |
| 2-1-d1E03-1-0824 | 815.135 | OH | a1 | B07 | b1 | C14 | c2 | D02 | d1 | E03 |
| 2-1-d1E03-1-0825 | 815.170 | OH | a2 | B19 | b2 | C05 | c2 | D02 | d1 | E03 |
| 2-1-d1E03-1-0826 | 815.175 | OH | a1 | B05 | b3 | C08 | c2 | D02 | d1 | E03 |
| 2-1-d1E03-1-0827 | 816.154 | OH | a2 | B20 | b2 | C02 | c2 | D02 | d1 | E03 |
| 2-1-d1E03-1-0828 | 816.190 | OH | a2 | B20 | b2 | C21 | c3 | D02 | d1 | E03 |
| 2-1-d1E03-1-0829 | 816.190 | OH | a2 | B19 | b2 | C25 | c3 | D02 | d1 | E03 |
| 2-1-d1E03-1-0830 | 817.149 | OH | a2 | B20 | b2 | C03 | c2 | D02 | d1 | E03 |
| 2-1-d1E03-1-0831 | 817.155 | OH | a1 | B06 | b3 | C08 | c2 | D02 | d1 | E03 |
| 2-1-d1E03-1-0832 | 817.222 | OH | a1 | B09 | b1 | C32 | c3 | D02 | d1 | E03 |
| 2-1-d1E03-1-0833 | 818.149 | OH | a2 | B19 | b2 | C04 | c2 | D02 | d1 | E03 |
| 2-1-d1E03-1-0834 | 818.154 | OH | a2 | B18 | b3 | C08 | c2 | D02 | d1 | E03 |
| 2-1-d1E03-1-0835 | 819.164 | OH | a1 | B09 | b2 | C02 | c2 | D02 | d1 | E03 |
| 2-1-d1E03-1-0836 | 819.201 | OH | a1 | B05 | b1 | C09 | c2 | D02 | d1 | E03 |
| 2-1-d1E03-1-0837 | 819.201 | OH | a1 | B09 | b2 | C21 | c3 | D02 | d1 | E03 |
| 2-1-d1E03-1-0838 | 819.201 | OH | a1 | B10 | b1 | C30 | c3 | D02 | d1 | E03 |
| 2-1-d1E03-1-0839 | 819.221 | OH | a1 | B07 | b2 | C07 | c2 | D02 | d1 | E03 |
| 2-1-d1E03-1-0840 | 819.276 | OH | a1 | B05 | b1 | C31 | c3 | D02 | d1 | E03 |
| 2-1-d1E03-1-0841 | 820.160 | OH | a1 | B09 | b2 | C03 | c2 | D02 | d1 | E03 |
| 2-1-d1E03-1-0842 | 820.165 | OH | a2 | B20 | b2 | C23 | c3 | D02 | d1 | E03 |
| 2-1-d1E03-1-0843 | 820.217 | OH | a1 | B08 | b1 | C29 | c3 | D02 | d1 | E03 |
| 2-1-d1E03-1-0844 | 821.132 | OH | a1 | B05 | b3 | C06 | c2 | D02 | d1 | E03 |
| 2-1-d1E03-1-0845 | 821.180 | OH | a1 | B06 | b1 | C09 | c2 | D02 | d1 | E03 |
| 2-1-d1E03-1-0846 | 821.180 | OH | a1 | B10 | b2 | C19 | c3 | D02 | d1 | E03 |
| 2-1-d1E03-1-0847 | 821.255 | OH | a1 | B06 | b1 | C31 | c3 | D02 | d1 | E03 |
| 2-1-d1E03-1-0848 | 822.110 | OH | a2 | B20 | b2 | C06 | c2 | D02 | d1 | E03 |
| 2-1-d1E03-1-0849 | 822.149 | OH | a2 | B11 | b3 | C16 | c2 | D02 | d1 | E03 |
| 2-1-d1E03-1-0850 | 822.175 | OH | a1 | B10 | b2 | C27 | c3 | D02 | d1 | E03 |
| 2-1-d1E03-1-0851 | 822.179 | OH | a2 | B18 | b1 | C09 | c2 | D02 | d1 | E03 |
| 2-1-d1E03-1-0852 | 822.196 | OH | a1 | B05 | b3 | C18 | c1 | D01 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0853 | 822.255 | OH | a2 | B18 | b1 | C31 | c3 | D02 | d1 | E03 |
| 2-1-d1E03-1-0854 | 823.111 | OH | a1 | B06 | b3 | C06 | c2 | D02 | d1 | E03 |
| 2-1-d1E03-1-0855 | 823.125 | OH | a1 | B07 | b3 | C08 | c2 | D02 | d1 | E03 |
| 2-1-d1E03-1-0856 | 823.144 | OH | a2 | B12 | b3 | C16 | c2 | D02 | d1 | E03 |
| 2-1-d1E03-1-0857 | 823.155 | OH | a1 | B08 | b2 | C05 | c2 | D02 | d1 | E03 |
| 2-1-d1E03-1-0858 | 823.176 | OH | a1 | B09 | b2 | C23 | c3 | D02 | d1 | E03 |
| 2-1-d1E03-1-0859 | 824.110 | OH | a2 | B18 | b3 | C06 | c2 | D02 | d1 | E03 |
| 2-1-d1E03-1-0860 | 824.110 | OH | a2 | B11 | b3 | C17 | c2 | D02 | d1 | E03 |
| 2-1-d1E03-1-0861 | 824.170 | OH | a2 | B19 | b1 | C13 | c2 | D02 | d1 | E03 |
| 2-1-d1E03-1-0862 | 824.176 | OH | a1 | B06 | b3 | C18 | c1 | D01 | d1 | E03 |
| 2-1-d1E03-1-0863 | 824.176 | OH | a1 | B08 | b2 | C25 | c3 | D02 | d1 | E03 |
| 2-1-d1E03-1-0864 | 825.106 | OH | a2 | B12 | b3 | C17 | c2 | D02 | d1 | E03 |
| 2-1-d1E03-1-0865 | 825.121 | OH | a1 | B09 | b2 | C06 | c2 | D02 | d1 | E03 |
| 2-1-d1E03-1-0866 | 825.139 | OH | a1 | B10 | b2 | C01 | c2 | D02 | d1 | E03 |
| 2-1-d1E03-1-0867 | 825.175 | OH | a2 | B18 | b3 | C18 | c1 | D01 | d1 | E03 |
| 2-1-d1E03-1-0868 | 826.105 | OH | a2 | B19 | b3 | C15 | c2 | D02 | d1 | E03 |
| 2-1-d1E03-1-0869 | 826.135 | OH | a1 | B08 | b2 | C04 | c2 | D02 | d1 | E03 |
| 2-1-d1E03-1-0870 | 826.174 | OH | a2 | B19 | b3 | C16 | c1 | D01 | d1 | E03 |
| 2-1-d1E03-1-0871 | 827.151 | OH | a1 | B07 | b1 | C09 | c2 | D02 | d1 | E03 |
| 2-1-d1E03-1-0872 | 827.226 | OH | a1 | B07 | b1 | C31 | c3 | D02 | d1 | E03 |
| 2-1-d1E03-1-0873 | 828.136 | OH | a2 | B19 | b3 | C17 | c1 | D01 | d1 | E03 |
| 2-1-d1E03-1-0874 | 828.136 | OH | a2 | B19 | b1 | C14 | c2 | D02 | d1 | E03 |
| 2-1-d1E03-1-0875 | 828.226 | OH | a2 | B20 | b1 | C29 | c3 | D02 | d1 | E03 |
| 2-1-d1E03-1-0876 | 829.082 | OH | a1 | B07 | b3 | C06 | c2 | D02 | d1 | E03 |
| 2-1-d1E03-1-0877 | 830.146 | OH | a1 | B07 | b3 | C18 | c1 | D01 | d1 | E03 |
| 2-1-d1E03-1-0878 | 831.164 | OH | a2 | B20 | b2 | C05 | c2 | D02 | d1 | E03 |
| 2-1-d1E03-1-0879 | 831.237 | OH | a1 | B09 | b1 | C29 | c3 | D02 | d1 | E03 |
| 2-1-d1E03-1-0880 | 832.156 | OH | a1 | B08 | b1 | C13 | c2 | D02 | d1 | E03 |
| 2-1-d1E03-1-0881 | 832.185 | OH | a2 | B20 | b2 | C25 | c3 | D02 | d1 | E03 |
| 2-1-d1E03-1-0882 | 832.221 | OH | a2 | B19 | b2 | C07 | c2 | D02 | d1 | E03 |
| 2-1-d1E03-1-0883 | 833.216 | OH | a1 | B10 | b1 | C32 | c3 | D02 | d1 | E03 |
| 2-1-d1E03-1-0884 | 834.091 | OH | a1 | B08 | b3 | C15 | c2 | D02 | d1 | E03 |
| 2-1-d1E03-1-0885 | 834.144 | OH | a2 | B20 | b2 | C04 | c2 | D02 | d1 | E03 |
| 2-1-d1E03-1-0886 | 834.160 | OH | a1 | B08 | b3 | C16 | c1 | D01 | d1 | E03 |
| 2-1-d1E03-1-0887 | 834.175 | OH | a1 | B09 | b2 | C05 | c2 | D02 | d1 | E03 |
| 2-1-d1E03-1-0888 | 835.159 | OH | a1 | B10 | b2 | C02 | c2 | D02 | d1 | E03 |
| 2-1-d1E03-1-0889 | 835.196 | OH | a1 | B10 | b2 | C21 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0890 | 835.196 | OH | a1 | B09 | b2 | C25 | c3 | D02 | d1 | E03 |
| 2-1-d1E03-1-0891 | 836.122 | OH | a1 | B08 | b3 | C17 | c1 | D01 | d1 | E03 |
| 2-1-d1E03-1-0892 | 836.122 | OH | a1 | B08 | b1 | C14 | c2 | D02 | d1 | E03 |
| 2-1-d1E03-1-0893 | 836.126 | OH | a2 | B19 | b3 | C08 | c2 | D02 | d1 | E03 |
| 2-1-d1E03-1-0894 | 836.155 | OH | a1 | B10 | b2 | C03 | c2 | D02 | d1 | E03 |
| 2-1-d1E03-1-0895 | 836.164 | OH | a2 | B13 | b3 | C16 | c2 | D02 | d1 | E03 |
| 2-1-d1E03-1-0896 | 837.155 | OH | a1 | B09 | b2 | C04 | c2 | D02 | d1 | E03 |
| 2-1-d1E03-1-0897 | 838.126 | OH | a2 | B13 | b3 | C17 | c2 | D02 | d1 | E03 |
| 2-1-d1E03-1-0898 | 839.121 | OH | a2 | B11 | b3 | C18 | c2 | D02 | d1 | E03 |
| 2-1-d1E03-1-0899 | 839.171 | OH | a1 | B10 | b2 | C23 | c3 | D02 | d1 | E03 |
| 2-1-d1E03-1-0900 | 840.116 | OH | a2 | B12 | b3 | C18 | c2 | D02 | d1 | E03 |
| 2-1-d1E03-1-0901 | 840.139 | OH | a2 | B14 | b3 | C16 | c2 | D02 | d1 | E03 |
| 2-1-d1E03-1-0902 | 840.151 | OH | a2 | B19 | b1 | C09 | c2 | D02 | d1 | E03 |
| 2-1-d1E03-1-0903 | 840.165 | OH | a2 | B20 | b1 | C13 | c2 | D02 | d1 | E03 |
| 2-1-d1E03-1-0904 | 840.207 | OH | a1 | B08 | b2 | C07 | c2 | D02 | d1 | E03 |
| 2-1-d1E03-1-0905 | 840.226 | OH | a2 | B19 | b1 | C31 | c3 | D02 | d1 | E03 |
| 2-1-d1E03-1-0906 | 841.116 | OH | a1 | B10 | b2 | C06 | c2 | D02 | d1 | E03 |
| 2-1-d1E03-1-0907 | 841.155 | OH | a1 | B01 | b3 | C16 | c2 | D02 | d1 | E03 |
| 2-1-d1E03-1-0908 | 842.082 | OH | a2 | B19 | b3 | C06 | c2 | D02 | d1 | E03 |
| 2-1-d1E03-1-0909 | 842.100 | OH | a2 | B20 | b3 | C15 | c2 | D02 | d1 | E03 |
| 2-1-d1E03-1-0910 | 842.101 | OH | a2 | B14 | b3 | C17 | c2 | D02 | d1 | E03 |
| 2-1-d1E03-1-0911 | 842.150 | OH | a1 | B02 | b3 | C16 | c2 | D02 | d1 | E03 |
| 2-1-d1E03-1-0912 | 842.169 | OH | a2 | B20 | b3 | C16 | c1 | D01 | d1 | E03 |
| 2-1-d1E03-1-0913 | 843.116 | OH | a1 | B01 | b3 | C17 | c2 | D02 | d1 | E03 |
| 2-1-d1E03-1-0914 | 843.147 | OH | a2 | B19 | b3 | C18 | c1 | D01 | d1 | E03 |
| 2-1-d1E03-1-0915 | 843.176 | OH | a1 | B09 | b1 | C13 | c2 | D02 | d1 | E03 |
| 2-1-d1E03-1-0916 | 844.111 | OH | a1 | B02 | b3 | C17 | c2 | D02 | d1 | E03 |
| 2-1-d1E03-1-0917 | 844.111 | OH | a1 | B08 | b3 | C08 | c2 | D02 | d1 | E03 |
| 2-1-d1E03-1-0918 | 844.131 | OH | a2 | B20 | b3 | C17 | c1 | D01 | d1 | E03 |
| 2-1-dlE03-1-0919 | 844.131 | OH | a2 | B20 | b1 | C14 | c2 | D02 | d1 | E03 |
| 2-1-d1E03-1-0920 | 845.111 | OH | a1 | B09 | b3 | C15 | c2 | D02 | d1 | E03 |
| 2-1-d1E03-1-0921 | 845.180 | OH | a1 | B09 | b3 | C16 | c1 | D01 | d1 | E03 |
| 2-1-d1E03-1-0922 | 847.142 | OH | a1 | B09 | b3 | C17 | c1 | D01 | d1 | E03 |
| 2-1-d1E03-1-0923 | 847.142 | OH | a1 | B09 | b1 | C14 | c2 | D02 | d1 | E03 |
| 2-1-d1E03-1-0924 | 847.232 | OH | a1 | B10 | b1 | C29 | c3 | D02 | d1 | E03 |
| 2-1-d1E03-1-0925 | 848.137 | OH | a1 | B08 | b1 | C09 | c2 | D02 | d1 | E03 |
| 2-1-d1E03-1-0926 | 848.212 | OH | a1 | B08 | b1 | C31 | c3 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0927 | 848.216 | OH | a2 | B20 | b2 | C07 | c2 | D02 | d1 | E03 |
| 2-1-d1E03-1-0928 | 850.068 | OH | a1 | B08 | b3 | C06 | c2 | D02 | d1 | E03 |
| 2-1-d1E03-1-0929 | 850.170 | OH | a1 | B10 | b2 | C05 | c2 | D02 | d1 | E03 |
| 2-1-d1E03-1-0930 | 850.180 | OH | a2 | B15 | b3 | C16 | c2 | D02 | d1 | E03 |
| 2-1-d1E03-1-0931 | 851.132 | OH | a1 | B08 | b3 | C18 | c1 | D01 | d1 | E03 |
| 2-1-d1E03-1-0932 | 851.191 | OH | a1 | B10 | b2 | C25 | c3 | D02 | d1 | E03 |
| 2-1-d1E03-1-0933 | 851.227 | OH | a1 | B09 | b2 | C07 | c2 | D02 | d1 | E03 |
| 2-1-d1E03-1-0934 | 852.121 | OH | a2 | B20 | b3 | C08 | c2 | D02 | d1 | E03 |
| 2-1-d1E03-1-0935 | 852.142 | OH | a2 | B15 | b3 | C17 | c2 | D02 | d1 | E03 |
| 2-1-d1E03-1-0936 | 852.159 | OH | a2 | B16 | b3 | C16 | c2 | D02 | d1 | E03 |
| 2-1-d1E03-1-0937 | 853.137 | OH | a2 | B13 | b3 | C18 | c2 | D02 | d1 | E03 |
| 2-1-d1E03-1-0938 | 853.150 | OH | a1 | B10 | b2 | C04 | c2 | D02 | d1 | E03 |
| 2-1-d1E03-1-0939 | 854.121 | OH | a2 | B16 | b3 | C17 | c2 | D02 | d1 | E03 |
| 2-1-d1E03-1-0940 | 855.131 | OH | a1 | B09 | b3 | C08 | c2 | D02 | d1 | E03 |
| 2-1-d1E03-1-0941 | 855.170 | OH | a1 | B03 | b3 | C16 | c2 | D02 | d1 | E03 |
| 2-1-d1E03-1-0942 | 856.146 | OH | a2 | B20 | b1 | C09 | c2 | D02 | d1 | E03 |
| 2-1-d1E03-1-0943 | 856.221 | OH | a2 | B20 | b1 | C31 | c3 | D02 | d1 | E03 |
| 2-1-d1E03-1-0944 | 857.112 | OH | a2 | B14 | b3 | C18 | c2 | D02 | d1 | E03 |
| 2-1-d1E03-1-0945 | 857.132 | OH | a1 | B03 | b3 | C17 | c2 | D02 | d1 | E03 |
| 2-1-d1E03-1-0946 | 858.077 | OH | a2 | B20 | b3 | C06 | c2 | D02 | d1 | E03 |
| 2-1-d1E03-1-0947 | 858.127 | OH | a1 | B01 | b3 | C18 | c2 | D02 | d1 | E03 |
| 2-1-d1E03-1-0948 | 858.130 | OH | a2 | B17 | b3 | C16 | c2 | D02 | d1 | E03 |
| 2-1-d1E03-1-0949 | 859.122 | OH | a1 | B02 | b3 | C18 | c2 | D02 | d1 | E03 |
| 2-1-d1E03-1-0950 | 859.142 | OH | a2 | B20 | b3 | C18 | c1 | D01 | d1 | E03 |
| 2-1-d1E03-1-0951 | 859.145 | OH | a1 | B04 | b3 | C16 | c2 | D02 | d1 | E03 |
| 2-1-d1E03-1-0952 | 859.157 | OH | a1 | B09 | b1 | C09 | c2 | D02 | d1 | E03 |
| 2-1-d1E03-1-0953 | 859.171 | OH | a1 | B10 | b1 | C13 | c2 | D02 | d1 | E03 |
| 2-1-d1E03-1-0954 | 859.232 | OH | a1 | B09 | b1 | C31 | c3 | D02 | d1 | E03 |
| 2-1-d1E03-1-0955 | 860.091 | OH | a2 | B17 | b3 | C17 | c2 | D02 | d1 | E03 |
| 2-1-d1E03-1-0956 | 861.088 | OH | a1 | B09 | b3 | C06 | c2 | D02 | d1 | E03 |
| 2-1-d1E03-1-0957 | 861.106 | OH | a1 | B10 | b3 | C15 | c2 | D02 | d1 | E03 |
| 2-1-d1E03-1-0958 | 861.107 | OH | a1 | B04 | b3 | C17 | c2 | D02 | d1 | E03 |
| 2-1-d1E03-1-0959 | 861.175 | OH | a1 | B10 | b3 | C16 | c1 | D01 | d1 | E03 |
| 2-1-d1E03-1-0960 | 862.152 | OH | a1 | B09 | b3 | C18 | c1 | D01 | d1 | E03 |
| 2-1-d1E03-1-0961 | 863.137 | OH | a1 | B10 | b3 | C17 | c1 | D01 | d1 | E03 |
| 2-1-d1E03-1-0962 | 863.137 | OH | a1 | B10 | b1 | C14 | c2 | D02 | d1 | E03 |
| 2-1-d1E03-1-0963 | 867.152 | OH | a2 | B15 | b3 | C18 | c2 | D02 | d1 | E03 |

(continued)

| [Table 8-7-4] Compound that may be contained in mixture 2-1-d1E03-1 | | | | | | | | | | |
|---|---|---|---|---|---|---|---|---|---|---|
| ID | Exact Mass | | a | B | b | C | c | D | d | E |
| 2-1-d1E03-1-0964 | 867.222 | OH | a1 | B10 | b2 | C07 | c2 | D02 | d1 | E03 |
| 2-1-d1E03-1-0965 | 869.132 | OH | a2 | B16 | b3 | C18 | c2 | D02 | d1 | E03 |
| 2-1-d1E03-1-0966 | 869.186 | OH | a1 | B05 | b3 | C16 | c2 | D02 | d1 | E03 |
| 2-1-d1E03-1-0967 | 871.126 | OH | a1 | B10 | b3 | C08 | c2 | D02 | d1 | E03 |
| 2-1-d1E03-1-0968 | 871.147 | OH | a1 | B05 | b3 | C17 | c2 | D02 | d1 | E03 |
| 2-1-d1E03-1-0969 | 871.165 | OH | a1 | B06 | b3 | C16 | c2 | D02 | d1 | E03 |
| 2-1-d1E03-1-0970 | 872.143 | OH | a1 | B03 | b3 | C18 | c2 | D02 | d1 | E03 |
| 2-1-d1E03-1-0971 | 872.164 | OH | a2 | B18 | b3 | C16 | c2 | D02 | d1 | E03 |
| 2-1-d1E03-1-0972 | 873.127 | OH | a1 | B06 | b3 | C17 | c2 | D02 | d1 | E03 |
| 2-1-d1E03-1-0973 | 874.126 | OH | a2 | B18 | b3 | C17 | c2 | D02 | d1 | E03 |
| 2-1-d1E03-1-0974 | 875.102 | OH | a2 | B17 | b3 | C18 | c2 | D02 | d1 | E03 |
| 2-1-d1E03-1-0975 | 875.152 | OH | a1 | B10 | b1 | C09 | c2 | D02 | d1 | E03 |
| 2-1-d1E03-1-0976 | 875.227 | OH | a1 | B10 | b1 | C31 | c3 | D02 | d1 | E03 |
| 2-1-d1E03-1-0977 | 876.118 | OH | a1 | B04 | b3 | C18 | c2 | D02 | d1 | E03 |
| 2-1-d1E03-1-0978 | 877.083 | OH | a1 | B10 | b3 | C06 | c2 | D02 | d1 | E03 |
| 2-1-d1E03-1-0979 | 877.136 | OH | a1 | B07 | b3 | C16 | c2 | D02 | d1 | E03 |
| 2-1-d1E03-1-0980 | 878.147 | OH | a1 | B10 | b3 | C18 | c1 | D01 | d1 | E03 |
| 2-1-d1E03-1-0981 | 879.097 | OH | a1 | B07 | b3 | C17 | c2 | D02 | d1 | E03 |
| 2-1-d1E03-1-0982 | 886.158 | OH | a1 | B05 | b3 | C18 | c2 | D02 | d1 | E03 |
| 2-1-d1E03-1-0983 | 888.138 | OH | a1 | B06 | b3 | C18 | c2 | D02 | d1 | E03 |
| 2-1-d1E03-1-0984 | 889.137 | OH | a2 | B18 | b3 | C18 | c2 | D02 | d1 | E03 |
| 2-1-d1E03-1-0985 | 890.136 | OH | a2 | B19 | b3 | C16 | c2 | D02 | d1 | E03 |
| 2-1-d1E03-1-0986 | 892.098 | OH | a2 | B19 | b3 | C17 | c2 | D02 | d1 | E03 |
| 2-1-d1E03-1-0987 | 894.108 | OH | a1 | B07 | b3 | C18 | c2 | D02 | d1 | E03 |
| 2-1-d1E03-1-0988 | 898.122 | OH | a1 | B08 | b3 | C16 | c2 | D02 | d1 | E03 |
| 2-1-d1E03-1-0989 | 900.083 | OH | a1 | B08 | b3 | C17 | c2 | D02 | d1 | E03 |
| 2-1-d1E03-1-0990 | 906.131 | OH | a2 | B20 | b3 | C16 | c2 | D02 | d1 | E03 |
| 2-1-d1E03-1-0991 | 907.109 | OH | a2 | B19 | b3 | C18 | c2 | D02 | d1 | E03 |
| 2-1-d1E03-1-0992 | 908.093 | OH | a2 | B20 | b3 | C17 | c2 | D02 | d1 | E03 |
| 2-1-d1E03-1-0993 | 909.142 | OH | a1 | B09 | b3 | C16 | c2 | D02 | d1 | E03 |
| 2-1-d1E03-1-0994 | 911.103 | OH | a1 | B09 | b3 | C17 | c2 | D02 | d1 | E03 |
| 2-1-d1E03-1-0995 | 915.094 | OH | a1 | B08 | b3 | C18 | c2 | D02 | d1 | E03 |
| 2-1-d1E03-1-0996 | 923.103 | OH | a2 | B20 | b3 | C18 | c2 | D02 | d1 | E03 |
| 2-1-d1E03-1-0997 | 925.137 | OH | a1 | B10 | b3 | C16 | c2 | D02 | d1 | E03 |
| 2-1-d1E03-1-0998 | 926.114 | OH | a1 | B09 | b3 | C18 | c2 | D02 | d1 | E03 |
| 2-1-d1E03-1-0999 | 927.098 | OH | a1 | B10 | b3 | C17 | c2 | D02 | d1 | E03 |
| 2-1-d1E03-1-1000 | 942.109 | OH | a1 | B10 | b3 | C18 | c2 | D02 | d1 | E03 |

[Table 391]

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0001 | 753.298 | OH | a2 | B11 | b1 | C08 | c1 | D01 | d1 | E04 |
| 2-1-d1E04-1-0002 | 754.294 | OH | a2 | B12 | b1 | C08 | c1 | D01 | d1 | E04 |
| 2-1-d1E04-1-0003 | 754.294 | OH | a2 | B11 | b1 | C11 | c1 | D01 | d1 | E04 |
| 2-1-d1E04-1-0004 | 755.289 | OH | a2 | B11 | b1 | C10 | c1 | D01 | d1 | E04 |
| 2-1-d1E04-1-0005 | 755.289 | OH | a2 | B12 | b1 | C11 | c1 | D01 | d1 | E04 |
| 2-1-d1E04-1-0006 | 756.284 | OH | a2 | B12 | b1 | C10 | c1 | D01 | d1 | E04 |
| 2-1-d1E04-1-0007 | 760.250 | OH | a2 | B11 | b1 | C12 | c1 | D01 | d1 | E04 |
| 2-1-d1E04-1-0008 | 761.245 | OH | a2 | B12 | b1 | C12 | c1 | D01 | d1 | E04 |
| 2-1-d1E04-1-0009 | 767.314 | OH | a2 | B13 | b1 | C08 | c1 | D01 | d1 | E04 |
| 2-1-d1E04-1-0010 | 768.309 | OH | a2 | B13 | b1 | C11 | c1 | D01 | d1 | E04 |
| 2-1-d1E04-1-0011 | 769.305 | OH | a2 | B13 | b1 | C10 | c1 | D01 | d1 | E04 |
| 2-1-d1E04-1-0012 | 771.273 | OH | a2 | B11 | b2 | C01 | c1 | D01 | d1 | E04 |
| 2-1-d1E04-1-0013 | 771.289 | OH | a2 | B14 | b1 | C08 | c1 | D01 | d1 | E04 |
| 2-1-d1E04-1-0014 | 772.268 | OH | a2 | B12 | b2 | C01 | c1 | D01 | d1 | E04 |
| 2-1-d1E04-1-0015 | 772.284 | OH | a2 | B14 | b1 | C11 | c1 | D01 | d1 | E04 |
| 2-1-d1E04-1-0016 | 772.304 | OH | a1 | B01 | b1 | C08 | c1 | D01 | d1 | E04 |
| 2-1-d1E04-1-0017 | 773.280 | OH | a2 | B14 | b1 | C10 | c1 | DOI | d1 | E04 |
| 2-1-d1E04-1-0018 | 773.300 | OH | a1 | B02 | b1 | C08 | c1 | D01 | d1 | E04 |
| 2-1-d1E04-1-0019 | 773.300 | OH | a1 | B01 | b1 | C11 | c1 | D01 | d1 | E04 |
| 2-1-d1E04-1-0020 | 774.266 | OH | a2 | B13 | b1 | C12 | c1 | D01 | d1 | E04 |
| 2-1-d1E04-1-0021 | 774.295 | OH | a1 | B01 | b1 | C10 | c1 | D01 | d1 | E04 |
| 2-1-d1E04-1-0022 | 774.295 | OH | a1 | B02 | b1 | C11 | c1 | D01 | d1 | E04 |
| 2-1-d1E04-1-0023 | 775.290 | OH | a1 | B02 | b1 | C10 | c1 | D01 | d1 | E04 |
| 2-1-d1E04-1-0024 | 778.241 | OH | a2 | B14 | b1 | C12 | c1 | D01 | d1 | E04 |
| 2-1-d1E04-1-0025 | 779.256 | OH | a1 | B01 | b1 | C12 | c1 | D01 | d1 | E04 |
| 2-1-d1E04-1-0026 | 780.251 | OH | a1 | B02 | b1 | C12 | c1 | D01 | d1 | E04 |
| 2-1-dlE04-1-0027 | 781.293 | OH | a2 | B11 | b2 | C02 | c1 | D01 | d1 | E04 |
| 2-1-dlE04-1-0028 | 781.330 | OH | a2 | B15 | b1 | C08 | c1 | D01 | d1 | E04 |
| 2-1-d1E04-1-0029 | 782.289 | OH | a2 | B12 | b2 | C02 | c1 | D01 | d1 | E04 |
| 2-1-d1E04-1-0030 | 782.289 | OH | a2 | B11 | b2 | C03 | c1 | D01 | d1 | E04 |
| 2-1-d1E04-1-0031 | 782.325 | OH | a2 | B15 | b1 | C11 | c1 | D01 | d1 | E04 |
| 2-1-d1E04-1-0032 | 783.284 | OH | a2 | B12 | b2 | C03 | c1 | D01 | d1 | E04 |
| 2-1-d1E04-1-0033 | 783.309 | OH | a2 | B16 | b1 | C08 | c1 | D01 | d1 | E04 |
| 2-1-dlE04-1-0034 | 783.320 | OH | a2 | B15 | b1 | C10 | c1 | D01 | d1 | E04 |
| 2-1-d1E04-1-0035 | 784.304 | OH | a2 | B16 | b1 | C11 | c1 | D01 | d1 | E04 |
| 2-1-d1E04-1-0036 | 785.288 | OH | a2 | B13 | b2 | C01 | c1 | D01 | d1 | E04 |
| 2-1-d1E04-1-0037 | 785.300 | OH | a2 | B16 | b1 | C10 | c1 | D01 | d1 | E04 |
| 2-1-d1E04-1-0038 | 786.320 | OH | a1 | B03 | b1 | C08 | c1 | D01 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0039 | 787.250 | OH | a2 | B11 | b2 | C06 | c1 | D01 | d1 | E04 |
| 2-1-d1E04-1-0040 | 787.315 | OH | a1 | B03 | b1 | C11 | c1 | D01 | d1 | E04 |
| 2-1-d1E04-1-0041 | 788.245 | OH | a2 | B12 | b2 | C06 | c1 | D01 | d1 | E04 |
| 2-1-d1E04-1-0042 | 788.281 | OH | a2 | B15 | b1 | C12 | c1 | D01 | d1 | E04 |
| 2-1-d1E04-1-0043 | 788.310 | OH | a1 | B03 | b1 | C10 | c1 | D01 | d1 | E04 |
| 2-1-d1E04-1-0044 | 789.263 | OH | a2 | B14 | b2 | C01 | c1 | D01 | d1 | E04 |
| 2-1-d1E04-1-0045 | 789.280 | OH | a2 | B17 | b1 | C08 | c1 | D01 | d1 | E04 |
| 2-1-d1E04-1-0046 | 790.261 | OH | a2 | B16 | b1 | C12 | c1 | D01 | d1 | E04 |
| 2-1-d1E04-1-0047 | 790.275 | OH | a2 | B17 | b1 | C11 | c1 | D01 | d1 | E04 |
| 2-1-d1E04-1-0048 | 790.278 | OH | a1 | B01 | b2 | C01 | c1 | D01 | d1 | E04 |
| 2-1-d1E04-1-0049 | 790.295 | OH | a1 | B04 | b1 | C08 | c1 | D01 | d1 | E04 |
| 2-1-d1E04-1-0050 | 791.270 | OH | a2 | B17 | b1 | C10 | c1 | D01 | d1 | E04 |
| 2-1-d1E04-1-0051 | 791.274 | OH | a1 | B02 | b2 | C01 | c1 | D01 | d1 | E04 |
| 2-1-d1E04-1-0052 | 791.290 | OH | a1 | B04 | b1 | C11 | c1 | D01 | d1 | E04 |
| 2-1-d1E04-1-0053 | 792.285 | OH | a1 | B04 | b1 | C10 | c1 | D01 | d1 | E04 |
| 2-1-d1E04-1-0054 | 793.272 | OH | a1 | B03 | b1 | C12 | c1 | D01 | d1 | E04 |
| 2-1-d1E04-1-0055 | 795.309 | OH | a2 | B13 | b2 | C02 | c1 | D01 | d1 | E04 |
| 2-1-d1E04-1-0056 | 796.231 | OH | a2 | B17 | b1 | C12 | c1 | D01 | d1 | E04 |
| 2-1-d1E04-1-0057 | 796.304 | OH | a2 | B13 | b2 | C03 | c1 | D01 | d1 | E04 |
| 2-1-d1E04-1-0058 | 796.304 | OH | a2 | B11 | b2 | C05 | c1 | D01 | d1 | E04 |
| 2-1-d1E04-1-0059 | 797.247 | OH | a1 | B04 | b1 | C12 | c1 | D01 | d1 | E04 |
| 2-1-d1E04-1-0060 | 797.300 | OH | a2 | B12 | b2 | C05 | c1 | D01 | d1 | E04 |
| 2-1-d1E04-1-0061 | 799.284 | OH | a2 | B14 | b2 | C02 | c1 | D01 | d1 | E04 |
| 2-1-d1E04-1-0062 | 799.284 | OH | a2 | B11 | b2 | C04 | c1 | D01 | d1 | E04 |
| 2-1-d1E04-1-0063 | 799.304 | OH | a2 | B15 | b2 | C01 | c1 | D01 | d1 | E04 |
| 2-1-d1E04-1-0064 | 800.279 | OH | a2 | B12 | b2 | C04 | c1 | D01 | d1 | E04 |
| 2-1-d1E04-1-0065 | 800.279 | OH | a2 | B14 | b2 | C03 | c1 | D01 | d1 | E04 |
| 2-1-d1E04-1-0066 | 800.299 | OH | a1 | B01 | b2 | C02 | c1 | D01 | d1 | E04 |
| 2-1-d1E04-1-0067 | 800.336 | OH | a1 | B05 | b1 | C08 | c1 | D01 | d1 | E04 |
| 2-1-d1E04-1-0068 | 801.265 | OH | a2 | B13 | b2 | C06 | c1 | D01 | d1 | E04 |
| 2-1-d1E04-1-0069 | 801.283 | OH | a2 | B16 | b2 | C01 | c1 | D01 | d1 | E04 |
| 2-1-d1E04-1-0070 | 801.294 | OH | a1 | B02 | b2 | C02 | c1 | D01 | d1 | E04 |
| 2-1-d1E04-1-0071 | 801.294 | OH | a1 | B01 | b2 | C03 | c1 | D01 | d1 | E04 |
| 2-1-d1E04-1-0072 | 801.331 | OH | a1 | B05 | b1 | C11 | c1 | D01 | d1 | E04 |
| 2-1-dlE04-1-0073 | 802.290 | OH | a1 | B02 | b2 | C03 | c1 | D01 | d1 | E04 |
| 2-1-dlE04-1-0074 | 802.315 | OH | a1 | B06 | b1 | C08 | c1 | D01 | d1 | E04 |
| 2-1-d1E04-1-0075 | 802.326 | OH | a1 | B05 | b1 | C10 | c1 | D01 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-dlE04-1-0076 | 803.310 | OH | a1 | B06 | b1 | C11 | c1 | D01 | d1 | E04 |
| 2-1-d1E04-1-0077 | 803.314 | OH | a2 | B18 | b1 | C08 | c1 | D01 | d1 | E04 |
| 2-1-d1E04-1-0078 | 804.294 | OH | a1 | B03 | b2 | C01 | c1 | D01 | d1 | E04 |
| 2-1-dlE04-1-0079 | 804.305 | OH | a1 | B06 | b1 | C10 | c1 | D01 | d1 | E04 |
| 2-1-d1E04-1-0080 | 804.309 | OH | a2 | B18 | b1 | C11 | c1 | D01 | d1 | E04 |
| 2-1-d1E04-1-0081 | 805.240 | OH | a2 | B14 | b2 | C06 | c1 | D01 | d1 | E04 |
| 2-1-d1E04-1-0082 | 805.305 | OH | a2 | B18 | b1 | C10 | c1 | D01 | d1 | E04 |
| 2-1-d1E04-1-0083 | 805.305 | OH | a2 | B11 | b1 | C13 | c1 | D01 | d1 | E04 |
| 2-1-d1E04-1-0084 | 806.256 | OH | a1 | B01 | b2 | C06 | c1 | D01 | d1 | E04 |
| 2-1-d1E04-1-0085 | 806.300 | OH | a2 | B12 | b1 | C13 | c1 | D01 | d1 | E04 |
| 2-1-d1E04-1-0086 | 807.240 | OH | a2 | B11 | b3 | C15 | c1 | D01 | d1 | E04 |
| 2-1-d1E04-1-0087 | 807.251 | OH | a1 | B02 | b2 | C06 | c1 | D01 | d1 | E04 |
| 2-1-d1E04-1-0088 | 807.254 | OH | a2 | B17 | b2 | C01 | c1 | D01 | d1 | E04 |
| 2-1-d1E04-1-0089 | 807.287 | OH | a1 | B05 | b1 | C12 | c1 | D01 | d1 | E04 |
| 2-1-d1E04-1-0090 | 808.235 | OH | a2 | B12 | b3 | C15 | c1 | D01 | d1 | E04 |
| 2-1-d1E04-1-0091 | 808.269 | OH | a1 | B04 | b2 | C01 | c1 | D01 | d1 | E04 |
| 2-1-d1E04-1-0092 | 808.285 | OH | a1 | B07 | b1 | C08 | c1 | D01 | d1 | E04 |
| 2-1-d1E04-1-0093 | 809.267 | OH | a1 | B06 | b1 | C12 | c1 | D01 | d1 | E04 |
| 2-1-d1E04-1-0094 | 809.271 | OH | a2 | B11 | b1 | C14 | c1 | D01 | d1 | E04 |
| 2-1-d1E04-1-0095 | 809.281 | OH | a1 | B07 | b1 | C11 | c1 | D01 | d1 | E04 |
| 2-1-d1E04-1-0096 | 809.292 | OH | a2 | B11 | b2 | C33 | c3 | D02 | d1 | E04 |
| 2-1-d1E04-1-0097 | 809.325 | OH | a2 | B15 | b2 | C02 | c1 | D01 | d1 | E04 |
| 2-1-d1E04-1-0098 | 810.266 | OH | a2 | B18 | b1 | C12 | c1 | D01 | d1 | E04 |
| 2-1-d1E04-1-0099 | 810.266 | OH | a2 | B12 | b1 | C14 | c1 | D01 | d1 | E04 |
| 2-1-d1E04-1-0100 | 810.276 | OH | a1 | B07 | b1 | C10 | c1 | D01 | d1 | E04 |
| 2-1-d1E04-1-0101 | 810.287 | OH | a2 | B12 | b2 | C33 | c3 | D02 | d1 | E04 |
| 2-1-d1E04-1-0102 | 810.320 | OH | a2 | B15 | b2 | C03 | c1 | D01 | d1 | E04 |
| 2-1-d1E04-1-0103 | 810.320 | OH | a2 | B13 | b2 | COS | c1 | D01 | d1 | E04 |
| 2-1-d1E04-1-0104 | 811.304 | OH | a2 | B16 | b2 | C02 | c1 | D01 | d1 | E04 |
| 2-1-d1E04-1-0105 | 812.299 | OH | a2 | B16 | b2 | C03 | c1 | D01 | d1 | E04 |
| 2-1-d1E04-1-0106 | 813.300 | OH | a2 | B13 | b2 | C04 | c1 | D01 | d1 | E04 |
| 2-1-d1E04-1-0107 | 813.356 | OH | a2 | B11 | b2 | C07 | c1 | D01 | d1 | E04 |
| 2-1-d1E04-1-0108 | 814.295 | OH | a2 | B14 | b2 | COS | c1 | D01 | d1 | E04 |
| 2-1-d1E04-1-0109 | 814.315 | OH | a1 | B03 | b2 | C02 | c1 | D01 | d1 | E04 |
| 2-1-d1E04-1-0110 | 814.351 | OH | a2 | B12 | b2 | C07 | c1 | D01 | d1 | E04 |
| 2-1-d1E04-1-0111 | 815.237 | OH | a1 | B07 | b1 | C12 | c1 | D01 | d1 | E04 |
| 2-1-d1E04-1-0112 | 815.281 | OH | a2 | B15 | b2 | C06 | c1 | D01 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0113 | 815.310 | OH | a1 | B03 | b2 | C03 | c1 | D01 | d1 | E04 |
| 2-1-d1E04-1-0114 | 815.310 | OH | a1 | B01 | b2 | C05 | c1 | D01 | d1 | E04 |
| 2-1-d1E04-1-0115 | 816.305 | OH | a1 | B02 | b2 | C05 | c1 | D01 | d1 | E04 |
| 2-1-d1E04-1-0116 | 817.260 | OH | a2 | B16 | b2 | C06 | c1 | D01 | d1 | E04 |
| 2-1-d1E04-1-0117 | 817.260 | OH | a2 | B11 | b3 | C08 | c1 | D01 | d1 | E04 |
| 2-1-d1E04-1-0118 | 817.260 | OH | a2 | B11 | b1 | C08 | c2 | D02 | d1 | E04 |
| 2-1-d1E04-1-0119 | 817.275 | OH | a2 | B17 | b2 | C02 | c1 | D01 | d1 | E04 |
| 2-1-d1E04-1-0120 | 817.275 | OH | a2 | B14 | b2 | C04 | c1 | D01 | d1 | E04 |
| 2-1-d1E04-1-0121 | 818.256 | OH | a2 | B12 | b3 | C08 | c1 | D01 | d1 | E04 |
| 2-1-d1E04-1-0122 | 818.256 | OH | a2 | B12 | b1 | C08 | c2 | D02 | d1 | E04 |
| 2-1-d1E04-1-0123 | 818.256 | OH | a2 | B11 | b1 | C11 | c2 | D02 | d1 | E04 |
| 2-1-d1E04-1-0124 | 818.270 | OH | a2 | B17 | b2 | C03 | c1 | D01 | d1 | E04 |
| 2-1-d1E04-1-0125 | 818.290 | OH | a1 | B04 | b2 | C02 | c1 | D01 | d1 | E04 |
| 2-1-d1E04-1-0126 | 818.290 | OH | a1 | B01 | b2 | C04 | c1 | D01 | d1 | E04 |
| 2-1-d1E04-1-0127 | 818.310 | OH | a1 | B05 | b2 | C01 | c1 | D01 | d1 | E04 |
| 2-1-d1E04-1-0128 | 819.251 | OH | a2 | B11 | b1 | C10 | c2 | D02 | d1 | E04 |
| 2-1-d1E04-1-0129 | 819.251 | OH | a2 | B12 | b1 | C11 | c2 | D02 | d1 | E04 |
| 2-1-d1E04-1-0130 | 819.285 | OH | a1 | B02 | b2 | C04 | c1 | D01 | d1 | E04 |
| 2-1-d1E04-1-0131 | 819.285 | OH | a1 | B04 | b2 | C03 | c1 | D01 | d1 | E04 |
| 2-1-d1E04-1-0132 | 819.32 | OH | a2 | B13 | b1 | C13 | c1 | D01 | d1 | E04 |
| 2-1-d1E04-1-0133 | 820.246 | OH | a2 | B12 | b1 | C10 | c2 | D02 | d1 | E04 |
| 2-1-d1E04-1-0134 | 820.271 | OH | a1 | B03 | b2 | C06 | c1 | D01 | d1 | E04 |
| 2-1-d1E04-1-0135 | 820.289 | OH | a1 | B06 | b2 | C01 | c1 | D01 | d1 | E04 |
| 2-1-d1E04-1-0136 | 821.255 | OH | a2 | B13 | b3 | C15 | c1 | D01 | d1 | E04 |
| 2-1-d1E04-1-0137 | 821.286 | OH | a2 | B19 | b1 | C08 | c1 | D01 | d1 | E04 |
| 2-1-d1E04-1-0138 | 821.286 | OH | a2 | B11 | b1 | C09 | c1 | D01 | d1 | E04 |
| 2-1-d1E04-1-0139 | 821.288 | OH | a2 | B18 | b2 | C01 | c1 | D01 | d1 | E04 |
| 2-1-d1E04-1-0140 | 822.281 | OH | a2 | B12 | b1 | C09 | c1 | D01 | d1 | E04 |
| 2-1-d1E04-1-0141 | 822.281 | OH | a2 | B19 | b1 | C11 | c1 | D01 | d1 | E04 |
| 2-1-d1E04-1-0142 | 823.217 | OH | a2 | B11 | b3 | C06 | c1 | D01 | d1 | E04 |
| 2-1-d1E04-1-0143 | 823.231 | OH | a2 | B17 | b2 | C06 | c1 | D01 | d1 | E04 |
| 2-1-d1E04-1-0144 | 823.276 | OH | a2 | B19 | b1 | C10 | c1 | D01 | d1 | E04 |
| 2-1-d1E04-1-0145 | 823.286 | OH | a2 | B13 | b1 | C14 | c1 | D01 | d1 | E04 |
| 2-1-d1E04-1-0146 | 823.295 | OH | a2 | B14 | b1 | C13 | c1 | D01 | d1 | E04 |
| 2-1-d1E04-1-0147 | 823.307 | OH | a2 | B13 | b2 | C33 | c3 | D02 | d1 | E04 |
| 2-1-d1E04-1-0148 | 824.212 | OH | a2 | B12 | b3 | C06 | c1 | D01 | d1 | E04 |
| 2-1-d1E04-1-0149 | 824.212 | OH | a2 | B11 | b1 | C12 | c2 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0150 | 824.246 | OH | a1 | B04 | b2 | C06 | c1 | D01 | d1 | E04 |
| 2-1-d1E04-1-0151 | 824.310 | OH | a1 | B01 | b1 | C13 | c1 | D01 | d1 | E04 |
| 2-1-d1E04-1-0152 | 824.336 | OH | a2 | B15 | b2 | C05 | c1 | D01 | d1 | E04 |
| 2-1-d1E04-1-0153 | 825.207 | OH | a2 | B12 | b1 | C12 | c2 | D02 | d1 | E04 |
| 2-1-d1E04-1-0154 | 825.230 | OH | a2 | B14 | b3 | C15 | c1 | D01 | d1 | E04 |
| 2-1-d1E04-1-0155 | 825.306 | OH | a1 | B02 | b1 | C13 | c1 | D01 | d1 | E04 |
| 2-1-d1E04-1-0156 | 826.245 | OH | a1 | B01 | b3 | C15 | c1 | D01 | d1 | E04 |
| 2-1-d1E04-1-0157 | 826.260 | OH | a1 | B07 | b2 | C01 | c1 | D01 | d1 | E04 |
| 2-1-d1E04-1-0158 | 826.315 | OH | a2 | B16 | b2 | C05 | c1 | D01 | d1 | E04 |
| 2-1-d1E04-1-0159 | 827.241 | OH | a1 | B02 | b3 | C15 | c1 | D01 | d1 | E04 |
| 2-1-d1E04-1-0160 | 827.261 | OH | a2 | B14 | b1 | C14 | c1 | D01 | d1 | E04 |
| 2-1-d1E04-1-0161 | 827.282 | OH | a2 | B14 | b2 | C33 | c3 | D02 | d1 | E04 |
| 2-1-d1E04-1-0162 | 827.315 | OH | a2 | B15 | b2 | C04 | c1 | D01 | d1 | E04 |
| 2-1-d1E04-1-0163 | 827.372 | OH | a2 | B13 | b2 | C07 | c1 | D01 | d1 | E04 |
| 2-1-d1E04-1-0164 | 828.238 | OH | a2 | B19 | b1 | C12 | c1 | D01 | d1 | E04 |
| 2-1-d1E04-1-0165 | 828.276 | OH | a1 | B01 | b1 | C14 | c1 | D01 | d1 | E04 |
| 2-1-d1E04-1-0166 | 828.298 | OH | a1 | B01 | b2 | C33 | c3 | D02 | d1 | E04 |
| 2-1-d1E04-1-0167 | 828.331 | OH | a1 | B05 | b2 | C02 | c1 | D01 | d1 | E04 |
| 2-1-d1E04-1-0168 | 829.272 | OH | a1 | B08 | b1 | C08 | c1 | D01 | d1 | E04 |
| 2-1-d1E04-1-0169 | 829.272 | OH | a1 | B02 | b1 | C14 | c1 | D01 | d1 | E04 |
| 2-1-d1E04-1-0170 | 829.293 | OH | a1 | B02 | b2 | C33 | c3 | D02 | d1 | E04 |
| 2-1-d1E04-1-0171 | 829.295 | OH | a2 | B16 | b2 | C04 | c1 | D01 | d1 | E04 |
| 2-1-d1E04-1-0172 | 829.297 | OH | a2 | B11 | b1 | C30 | c3 | D02 | d1 | E04 |
| 2-1-d1E04-1-0173 | 829.326 | OH | a1 | B05 | b2 | C03 | c1 | D01 | d1 | E04 |
| 2-1-d1E04-1-0174 | 829.326 | OH | a1 | B03 | b2 | C05 | c1 | D01 | d1 | E04 |
| 2-1-d1E04-1-0175 | 830.267 | OH | a1 | B08 | b1 | C11 | c1 | D01 | d1 | E04 |
| 2-1-d1E04-1-0176 | 830.292 | OH | a2 | B12 | b1 | C30 | c3 | D02 | d1 | E04 |
| 2-1-d1E04-1-0177 | 830.310 | OH | a1 | B06 | b2 | C02 | c1 | D01 | d1 | E04 |
| 2-1-d1E04-1-0178 | 831.262 | OH | a1 | B08 | b1 | C10 | c1 | D01 | d1 | E04 |
| 2-1-d1E04-1-0179 | 831.276 | OH | a2 | B13 | b3 | C08 | c1 | D01 | d1 | E04 |
| 2-1-d1E04-1-0180 | 831.276 | OH | a2 | B13 | b1 | C08 | c2 | D02 | d1 | E04 |
| 2-1-d1E04-1-0181 | 831.276 | OH | a2 | B11 | b2 | C19 | c3 | D02 | d1 | E04 |
| 2-1-d1E04-1-0182 | 831.305 | OH | a1 | B06 | b2 | C03 | c1 | D01 | d1 | E04 |
| 2-1-d1E04-1-0183 | 831.309 | OH | a2 | B18 | b2 | C02 | c1 | D01 | d1 | E04 |
| 2-1-d1E04-1-0184 | 831.347 | OH | a2 | B14 | b2 | C07 | c1 | D01 | d1 | E04 |
| 2-1-d1E04-1-0185 | 832.271 | OH | a2 | B13 | b1 | C11 | c2 | D02 | d1 | E04 |
| 2-1-d1E04-1-0186 | 832.271 | OH | a2 | B12 | b2 | C19 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0187 | 832.271 | OH | a2 | B11 | b2 | C27 | c3 | D02 | d1 | E04 |
| 2-1-d1E04-1-0188 | 832.285 | OH | a2 | B17 | b2 | C05 | c1 | D01 | d1 | E04 |
| 2-1-d1E04-1-0189 | 832.304 | OH | a2 | B18 | b2 | C03 | c1 | D01 | d1 | E04 |
| 2-1-d1E04-1-0190 | 832.305 | OH | a1 | B03 | b2 | C04 | c1 | D01 | d1 | E04 |
| 2-1-d1E04-1-0191 | 832.362 | OH | a1 | B01 | b2 | C07 | c1 | D01 | d1 | E04 |
| 2-1-d1E04-1-0192 | 833.267 | OH | a2 | B13 | b1 | C10 | c2 | D02 | d1 | E04 |
| 2-1-d1E04-1-0193 | 833.267 | OH | a2 | B12 | b2 | C27 | c3 | D02 | d1 | E04 |
| 2-1-d1E04-1-0194 | 833.301 | OH | a1 | B04 | b2 | C05 | c1 | D01 | d1 | E04 |
| 2-1-d1E04-1-0195 | 833.336 | OH | a2 | B15 | b1 | C13 | c1 | D01 | d1 | E04 |
| 2-1-d1E04-1-0196 | 833.357 | OH | a1 | B02 | b2 | C07 | c1 | D01 | d1 | E04 |
| 2-1-d1E04-1-0197 | 834.287 | OH | a1 | B05 | b2 | C06 | c1 | D01 | d1 | E04 |
| 2-1-d1E04-1-0198 | 835.235 | OH | a2 | B11 | b2 | C01 | c2 | D02 | d1 | E04 |
| 2-1-d1E04-1-0199 | 835.251 | OH | a2 | B14 | b3 | C08 | c1 | D01 | d1 | E04 |
| 2-1-d1E04-1-0200 | 835.251 | OH | a2 | B14 | b1 | C08 | c2 | D02 | d1 | E04 |
| 2-1-d1E04-1-0201 | 835.265 | OH | a2 | B17 | b2 | C04 | c1 | D01 | d1 | E04 |
| 2-1-d1E04-1-0202 | 835.271 | OH | a2 | B15 | b3 | C15 | c1 | D01 | d1 | E04 |
| 2-1-d1E04-1-0203 | 835.302 | OH | a2 | B13 | b1 | C09 | c1 | D01 | d1 | E04 |
| 2-1-d1E04-1-0204 | 835.315 | OH | a2 | B16 | b1 | C13 | c1 | D01 | d1 | E04 |
| 2-1-d1E04-1-0205 | 836.223 | OH | a1 | B08 | b1 | C12 | c1 | D01 | d1 | E04 |
| 2-1-d1E04-1-0206 | 836.230 | OH | a2 | B12 | b2 | C01 | c2 | D02 | d1 | E04 |
| 2-1-d1E04-1-0207 | 836.246 | OH | a2 | B14 | b1 | C11 | c2 | D02 | d1 | E04 |
| 2-1-d1E04-1-0208 | 836.266 | OH | a1 | B06 | b2 | C06 | c1 | D01 | d1 | E04 |
| 2-1-d1E04-1-0209 | 836.266 | OH | a1 | B01 | b3 | C08 | c1 | D01 | d1 | E04 |
| 2-1-d1E04-1-0210 | 836.266 | OH | a1 | B01 | b1 | C08 | c2 | D02 | d1 | E04 |
| 2-1-d1E04-1-0211 | 836.280 | OH | a1 | B07 | b2 | C02 | c1 | D01 | d1 | E04 |
| 2-1-d1E04-1-0212 | 836.280 | OH | a1 | B04 | b2 | C04 | c1 | D01 | d1 | E04 |
| 2-1-d1E04-1-0213 | 837.232 | OH | a2 | B13 | b3 | C06 | c1 | D01 | d1 | E04 |
| 2-1-d1E04-1-0214 | 837.241 | OH | a2 | B14 | b1 | C10 | c2 | D02 | d1 | E04 |
| 2-1-d1E04-1-0215 | 837.250 | OH | a2 | B16 | b3 | C15 | c1 | D01 | d1 | E04 |
| 2-1-d1E04-1-0216 | 837.261 | OH | a1 | B02 | b3 | C08 | c1 | D01 | d1 | E04 |
| 2-1-d1E04-1-0217 | 837.261 | OH | a1 | B02 | b1 | C08 | c2 | D02 | d1 | E04 |
| 2-1-d1E04-1-0218 | 837.261 | OH | a1 | B01 | b1 | C11 | c2 | D02 | d1 | E04 |
| 2-1-d1E04-1-0219 | 837.265 | OH | a2 | B18 | b2 | C06 | c1 | D01 | d1 | E04 |
| 2-1-d1E04-1-0220 | 837.276 | OH | a1 | B07 | b2 | C03 | c1 | D01 | d1 | E04 |
| 2-1-d1E04-1-0221 | 837.281 | OH | a2 | B20 | b1 | C08 | c1 | D01 | d1 | E04 |
| 2-1-d1E04-1-0222 | 837.302 | OH | a2 | B15 | b1 | C14 | c1 | D01 | d1 | E04 |
| 2-1-d1E04-1-0223 | 837.323 | OH | a2 | B15 | b2 | C33 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0224 | 838.228 | OH | a2 | B13 | b1 | C12 | c2 | D02 | d1 | E04 |
| 2-1-d1E04-1-0225 | 838.257 | OH | a1 | B01 | b1 | C10 | c2 | D02 | d1 | E04 |
| 2-1-d1E04-1-0226 | 838.257 | OH | a1 | B02 | b1 | C11 | c2 | D02 | d1 | E04 |
| 2-1-d1E04-1-0227 | 838.276 | OH | a2 | B20 | b1 | C11 | c1 | D01 | d1 | E04 |
| 2-1-d1E04-1-0228 | 838.326 | OH | a1 | B03 | b1 | C13 | c1 | D01 | d1 | E04 |
| 2-1-d1E04-1-0229 | 839.252 | OH | a1 | B02 | b1 | C10 | c2 | D02 | d1 | E04 |
| 2-1-d1E04-1-0230 | 839.260 | OH | a2 | B19 | b2 | C01 | c1 | D01 | d1 | E04 |
| 2-1-d1E04-1-0231 | 839.271 | OH | a2 | B20 | b1 | C10 | c1 | D01 | d1 | E04 |
| 2-1-d1E04-1-0232 | 839.276 | OH | a2 | B14 | b1 | C09 | c1 | D01 | d1 | E04 |
| 2-1-d1E04-1-0233 | 839.281 | OH | a2 | B16 | b1 | C14 | c1 | D01 | d1 | E04 |
| 2-1-d1E04-1-0234 | 839.302 | OH | a2 | B16 | b2 | C33 | c3 | D02 | d1 | E04 |
| 2-1-d1E04-1-0235 | 840.261 | OH | a1 | B03 | b3 | C15 | c1 | D01 | d1 | E04 |
| 2-1-d1E04-1-0236 | 840.292 | OH | a1 | B09 | b1 | C08 | c1 | D01 | d1 | E04 |
| 2-1-d1E04-1-0237 | 840.292 | OH | a1 | B01 | b1 | C09 | c1 | D01 | d1 | E04 |
| 2-1-d1E04-1-0238 | 841.207 | OH | a2 | B14 | b3 | C06 | c1 | D01 | d1 | E04 |
| 2-1-d1E04-1-0239 | 841.286 | OH | a2 | B17 | b1 | C13 | c1 | D01 | d1 | E04 |
| 2-1-d1E04-1-0240 | 841.287 | OH | a1 | B02 | b1 | C09 | c1 | D01 | d1 | E04 |
| 2-1-d1E04-1-0241 | 841.287 | OH | a1 | B09 | b1 | C11 | c1 | D01 | d1 | E04 |
| 2-1-d1E04-1-0242 | 841.387 | OH | a2 | B15 | b2 | C07 | c1 | D01 | d1 | E04 |
| 2-1-d1E04-1-0243 | 842.203 | OH | a2 | B14 | b1 | C12 | c2 | D02 | d1 | E04 |
| 2-1-d1E04-1-0244 | 842.223 | OH | a1 | B01 | b3 | C06 | c1 | D01 | d1 | E04 |
| 2-1-d1E04-1-0245 | 842.237 | OH | a1 | B07 | b2 | C06 | c1 | D01 | d1 | E04 |
| 2-1-d1E04-1-0246 | 842.282 | OH | a1 | B09 | b1 | C10 | c1 | D01 | d1 | E04 |
| 2-1-d1E04-1-0247 | 842.292 | OH | a1 | B03 | b1 | C14 | c1 | D01 | d1 | E04 |
| 2-1-d1E04-1-0248 | 842.301 | OH | a1 | B04 | b1 | C13 | c1 | D01 | d1 | E04 |
| 2-1-d1E04-1-0249 | 842.313 | OH | a1 | B03 | b2 | C33 | c3 | D02 | d1 | E04 |
| 2-1-d1E04-1-0250 | 843.218 | OH | a1 | B02 | b3 | C06 | c1 | D01 | d1 | E04 |
| 2-1-d1E04-1-0251 | 843.218 | OH | a1 | B01 | b1 | C12 | c2 | D02 | d1 | E04 |
| 2-1-d1E04-1-0252 | 843.221 | OH | a2 | B17 | b3 | C15 | c1 | D01 | d1 | E04 |
| 2-1-d1E04-1-0253 | 843.312 | OH | a2 | B13 | b1 | C30 | c3 | D02 | d1 | E04 |
| 2-1-d1E04-1-0254 | 843.312 | OH | a2 | B11 | b1 | C32 | c3 | D02 | d1 | E04 |
| 2-1-d1E04-1-0255 | 843.341 | OH | a1 | B05 | b2 | C05 | c1 | D01 | d1 | E04 |
| 2-1-d1E04-1-0256 | 843.367 | OH | a2 | B16 | b2 | C07 | c1 | D01 | d1 | E04 |
| 2-1-d1E04-1-0257 | 844.213 | OH | a1 | B02 | b1 | C12 | c2 | D02 | d1 | E04 |
| 2-1-d1E04-1-0258 | 844.232 | OH | a2 | B20 | b1 | C12 | c1 | D01 | d1 | E04 |
| 2-1-d1E04-1-0259 | 844.236 | OH | a1 | B04 | b3 | C15 | c1 | D01 | d1 | E04 |
| 2-1-d1E04-1-0260 | 844.308 | OH | a2 | B12 | b1 | C32 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0261 | 845.252 | OH | a2 | B17 | b1 | C14 | c1 | D01 | d1 | E04 |
| 2-1-d1E04-1-0262 | 845.255 | OH | a2 | B11 | b2 | C02 | c2 | D02 | d1 | E04 |
| 2-1-d1E04-1-0263 | 845.273 | OH | a2 | B17 | b2 | C33 | c3 | D02 | d1 | E04 |
| 2-1-d1E04-1-0264 | 845.292 | OH | a2 | B15 | b3 | C08 | c1 | D01 | d1 | E04 |
| 2-1-d1E04-1-0265 | 845.292 | OH | a2 | B15 | b1 | C08 | c2 | D02 | d1 | E04 |
| 2-1-d1E04-1-0266 | 845.292 | OH | a2 | B13 | b2 | C19 | c3 | D02 | d1 | E04 |
| 2-1-d1E04-1-0267 | 845.292 | OH | a2 | B11 | b2 | C21 | c3 | D02 | d1 | E04 |
| 2-1-d1E04-1-0268 | 845.321 | OH | a1 | B06 | b2 | C05 | c1 | D01 | d1 | E04 |
| 2-1-d1E04-1-0269 | 846.251 | OH | a2 | B12 | b2 | C02 | c2 | D02 | d1 | E04 |
| 2-1-d1E04-1-0270 | 846.251 | OH | a2 | B11 | b2 | C03 | c2 | D02 | d1 | E04 |
| 2-1-d1E04-1-0271 | 846.267 | OH | a1 | B04 | b1 | C14 | c1 | D01 | d1 | E04 |
| 2-1-d1E04-1-0272 | 846.287 | OH | a2 | B15 | b1 | C11 | c2 | D02 | d1 | E04 |
| 2-1-d1E04-1-0273 | 846.287 | OH | a2 | B12 | b2 | C21 | c3 | D02 | d1 | E04 |
| 2-1-d1E04-1-0274 | 846.287 | OH | a2 | B13 | b2 | C27 | c3 | D02 | d1 | E04 |
| 2-1-d1E04-1-0275 | 846.288 | OH | a1 | B04 | b2 | C33 | c3 | D02 | d1 | E04 |
| 2-1-d1E04-1-0276 | 846.320 | OH | a2 | B18 | b2 | C05 | c1 | D01 | d1 | E04 |
| 2-1-d1E04-1-0277 | 846.321 | OH | a1 | B05 | b2 | C04 | c1 | D01 | d1 | E04 |
| 2-1-d1E04-1-0278 | 846.377 | OH | a1 | B03 | b2 | C07 | c1 | D01 | d1 | E04 |
| 2-1-d1E04-1-0279 | 847.243 | OH | a1 | B09 | b1 | C12 | c1 | D01 | d1 | E04 |
| 2-1-d1E04-1-0280 | 847.246 | OH | a1 | B08 | b2 | C01 | c1 | D01 | d1 | E04 |
| 2-1-d1E04-1-0281 | 847.246 | OH | a2 | B12 | b2 | C03 | c2 | D02 | d1 | E04 |
| 2-1-d1E04-1-0282 | 847.271 | OH | a2 | B16 | b3 | C08 | c1 | D01 | d1 | E04 |
| 2-1-d1E04-1-0283 | 847.271 | OH | a2 | B16 | b1 | C08 | c2 | D02 | d1 | E04 |
| 2-1-d1E04-1-0284 | 847.282 | OH | a2 | B15 | b1 | C10 | c2 | D02 | d1 | E04 |
| 2-1-d1E04-1-0285 | 847.287 | OH | a2 | B14 | b1 | C30 | c3 | D02 | d1 | E04 |
| 2-1-d1E04-1-0286 | 848.266 | OH | a2 | B16 | b1 | C11 | c2 | D02 | d1 | E04 |
| 2-1-d1E04-1-0287 | 848.300 | OH | a1 | B06 | b2 | C04 | c1 | D01 | d1 | E04 |
| 2-1-d1E04-1-0288 | 848.303 | OH | a1 | B01 | b1 | C30 | c3 | D02 | d1 | E04 |
| 2-1-d1E04-1-0289 | 849.250 | OH | a2 | B13 | b2 | C01 | c2 | D02 | d1 | E04 |
| 2-1-d1E04-1-0290 | 849.261 | OH | a2 | B16 | b1 | C10 | c2 | D02 | d1 | E04 |
| 2-1-d1E04-1-0291 | 849.267 | OH | a2 | B14 | b2 | C19 | c3 | D02 | d1 | E04 |
| 2-1-d1E04-1-0292 | 849.267 | OH | a2 | B11 | b2 | C23 | c3 | D02 | d1 | E04 |
| 2-1-d1E04-1-0293 | 849.281 | OH | a2 | B19 | b2 | C02 | c1 | D01 | d1 | E04 |
| 2-1-d1E04-1-0294 | 849.298 | OH | a1 | B02 | b1 | C30 | c3 | D02 | d1 | E04 |
| 2-1-d1E04-1-0295 | 849.300 | OH | a2 | B18 | b2 | C04 | c1 | D01 | d1 | E04 |
| 2-1-d1E04-1-0296 | 849.317 | OH | a2 | B15 | b1 | C09 | c1 | D01 | d1 | E04 |
| 2-1-d1E04-1-0297 | 849.337 | OH | a2 | B17 | b2 | C07 | c1 | D01 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0298 | 850.262 | OH | a2 | B12 | b2 | C23 | c3 | D02 | d1 | E04 |
| 2-1-d1E04-1-0299 | 850.262 | OH | a2 | B14 | b2 | C27 | c3 | D02 | d1 | E04 |
| 2-1-d1E04-1-0300 | 850.276 | OH | a2 | B19 | b2 | C03 | c1 | D01 | d1 | E04 |
| 2-1-d1E04-1-0301 | 850.282 | OH | a1 | B03 | b3 | C08 | c1 | D01 | d1 | E04 |
| 2-1-d1E04-1-0302 | 850.282 | OH | a1 | B03 | b1 | C08 | c2 | D02 | d1 | E04 |
| 2-1-d1E04-1-0303 | 850.282 | OH | a1 | B01 | b2 | C19 | c3 | D02 | d1 | E04 |
| 2-1-d1E04-1-0304 | 850.352 | OH | a1 | B04 | b2 | C07 | c1 | D01 | d1 | E04 |
| 2-1-d1E04-1-0305 | 851.212 | OH | a2 | B11 | b2 | C06 | c2 | D02 | d1 | E04 |
| 2-1-d1E04-1-0306 | 851.248 | OH | a2 | B15 | b3 | C06 | c1 | D01 | d1 | E04 |
| 2-1-d1E04-1-0307 | 851.277 | OH | a1 | B03 | b1 | C11 | c2 | D02 | d1 | E04 |
| 2-1-d1E04-1-0308 | 851.277 | OH | a1 | B02 | b2 | C19 | c3 | D02 | d1 | E04 |
| 2-1-d1E04-1-0309 | 851.277 | OH | a1 | B01 | b2 | C27 | c3 | D02 | d1 | E04 |
| 2-1-d1E04-1-0310 | 851.291 | OH | a1 | B07 | b2 | C05 | c1 | D01 | d1 | E04 |
| 2-1-d1E04-1-0311 | 851.296 | OH | a2 | B16 | b1 | C09 | c1 | D01 | d1 | E04 |
| 2-1-d1E04-1-0312 | 852.207 | OH | a2 | B12 | b2 | C06 | c2 | D02 | d1 | E04 |
| 2-1-d1E04-1-0313 | 852.243 | OH | a2 | B15 | b1 | C12 | c2 | D02 | d1 | E04 |
| 2-1-d1E04-1-0314 | 852.272 | OH | a1 | B05 | b1 | C10 | c2 | D02 | d1 | E04 |
| 2-1-d1E04-1-0315 | 852.272 | OH | a1 | B02 | b2 | C27 | c3 | D02 | d1 | E04 |
| 2-1-d1E04-1-0316 | 852.342 | OH | a1 | B05 | b1 | C13 | c1 | D01 | d1 | E04 |
| 2-1-d1E04-1-0317 | 853.225 | OH | a2 | B14 | b2 | C01 | c2 | D02 | d1 | E04 |
| 2-1-d1E04-1-0318 | 853.227 | OH | a2 | B16 | b3 | C06 | c1 | D01 | d1 | E04 |
| 2-1-d1E04-1-0319 | 853.242 | OH | a2 | B17 | b3 | C08 | c1 | D01 | d1 | E04 |
| 2-1-d1E04-1-0320 | 853.242 | OH | a2 | B17 | b1 | C08 | c2 | D02 | d1 | E04 |
| 2-1-d1E04-1-0321 | 854.223 | OH | a2 | B16 | b1 | C12 | c2 | D02 | d1 | E04 |
| 2-1-d1E04-1-0322 | 854.237 | OH | a2 | B17 | b1 | C11 | c2 | D02 | d1 | E04 |
| 2-1-d1E04-1-0323 | 854.240 | OH | a1 | B01 | b2 | C01 | c2 | D02 | d1 | E04 |
| 2-1-d1E04-1-0324 | 854.257 | OH | a1 | B04 | b3 | C08 | c1 | D01 | d1 | E04 |
| 2-1-d1E04-1-0325 | 854.257 | OH | a1 | B04 | b1 | C08 | c2 | D02 | d1 | E04 |
| 2-1-d1E04-1-0326 | 854.271 | OH | a1 | B07 | b2 | C04 | c1 | D01 | d1 | E04 |
| 2-1-d1E04-1-0327 | 854.277 | OH | a1 | B05 | b3 | C15 | c1 | D01 | d1 | E04 |
| 2-1-d1E04-1-0328 | 854.307 | OH | a1 | B03 | b1 | C09 | c1 | D01 | d1 | E04 |
| 2-1-d1E04-1-0329 | 854.321 | OH | a1 | B06 | b1 | C13 | c1 | D01 | d1 | E04 |
| 2-1-d1E04-1-0330 | 855.232 | OH | a2 | B17 | b1 | C10 | c2 | D02 | d1 | E04 |
| 2-1-d1E04-1-0331 | 855.236 | OH | a1 | B02 | b2 | C01 | c2 | D02 | d1 | E04 |
| 2-1-d1E04-1-0332 | 855.237 | OH | a2 | B19 | b2 | C06 | c1 | D01 | d1 | E04 |
| 2-1-d1E04-1-0333 | 855.252 | OH | a1 | B04 | b1 | C11 | c2 | D02 | d1 | E04 |
| 2-1-d1E04-1-0334 | 855.255 | OH | a2 | B20 | b2 | C01 | c1 | D01 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0335 | 855.320 | OH | a2 | B18 | b1 | C13 | c1 | D01 | d1 | E04 |
| 2-1-d1E04-1-0336 | 856.238 | OH | a1 | B03 | b3 | C06 | c1 | D01 | d1 | E04 |
| 2-1-d1E04-1-0337 | 856.247 | OH | a1 | B04 | b1 | C10 | c2 | D02 | d1 | E04 |
| 2-1-d1E04-1-0338 | 856.256 | OH | a1 | B06 | b3 | C15 | c1 | D01 | d1 | E04 |
| 2-1-d1E04-1-0339 | 856.287 | OH | a1 | B10 | b1 | C08 | c1 | D01 | d1 | E04 |
| 2-1-d1E04-1-0340 | 856.308 | OH | a1 | B05 | b1 | C14 | c1 | D01 | d1 | E04 |
| 2-1-d1E04-1-0341 | 856.329 | OH | a1 | B05 | b2 | C33 | c3 | D02 | d1 | E04 |
| 2-1-d1E04-1-0342 | 857.234 | OH | a1 | B03 | b1 | C12 | c2 | D02 | d1 | E04 |
| 2-1-d1E04-1-0343 | 857.255 | OH | a2 | B18 | b3 | C15 | c1 | D01 | d1 | E04 |
| 2-1-d1E04-1-0344 | 857.267 | OH | a1 | B05 | b2 | C02 | c1 | D01 | d1 | E04 |
| 2-1-d1E04-1-0345 | 857.267 | OH | a2 | B17 | b1 | C09 | c1 | D01 | d1 | E04 |
| 2-1-d1E04-1-0346 | 857.282 | OH | a1 | B10 | b1 | C11 | c1 | D01 | d1 | E04 |
| 2-1-d1E04-1-0347 | 857.328 | OH | a2 | B15 | b1 | C30 | c3 | D02 | d1 | E04 |
| 2-1-d1E04-1-0348 | 857.328 | OH | a2 | B11 | b1 | C29 | c3 | D02 | d1 | E04 |
| 2-1-d1E04-1-0349 | 857.328 | OH | a2 | B13 | b1 | C32 | c3 | D02 | d1 | E04 |
| 2-1-d1E04-1-0350 | 858.262 | OH | a1 | B05 | b2 | COS | c1 | D01 | d1 | E04 |
| 2-1-d1E04-1-0351 | 858.266 | OH | a1 | B09 | b2 | C01 | c1 | D01 | d1 | E04 |
| 2-1-d1E04-1-0352 | 858.277 | OH | a1 | B10 | b1 | C10 | c1 | D01 | d1 | E04 |
| 2-1-d1E04-1-0353 | 858.282 | OH | a1 | B04 | b1 | C09 | c1 | D01 | d1 | E04 |
| 2-1-d1E04-1-0354 | 858.287 | OH | a1 | B06 | b1 | C14 | c1 | D01 | d1 | E04 |
| 2-1-d1E04-1-0355 | 858.308 | OH | a1 | B06 | b2 | C33 | c3 | D02 | d1 | E04 |
| 2-1-d1E04-1-0356 | 858.323 | OH | a2 | B12 | b1 | C29 | c3 | D02 | d1 | E04 |
| 2-1-d1E04-1-0357 | 859.198 | OH | a2 | B17 | b3 | C06 | c1 | D01 | d1 | E04 |
| 2-1-d1E04-1-0358 | 859.271 | OH | a2 | B13 | b2 | C02 | c2 | D02 | d1 | E04 |
| 2-1-d1E04-1-0359 | 859.286 | OH | a2 | B18 | b1 | C14 | c1 | D01 | d1 | E04 |
| 2-1-d1E04-1-0360 | 859.307 | OH | a2 | B15 | b2 | C19 | c3 | D02 | d1 | E04 |
| 2-1-d1E04-1-0361 | 859.307 | OH | a2 | B13 | b2 | C21 | c3 | D02 | d1 | E04 |
| 2-1-d1E04-1-0362 | 859.307 | OH | a2 | B16 | b1 | C30 | c3 | D02 | d1 | E04 |
| 2-1-d1E04-1-0363 | 859.307 | OH | a2 | B18 | b2 | C33 | c3 | D02 | d1 | E04 |
| 2-1-d1E04-1-0364 | 860.193 | OH | a2 | B17 | b1 | C12 | c2 | D02 | d1 | E04 |
| 2-1-d1E04-1-0365 | 860.213 | OH | a1 | B04 | b3 | C06 | c1 | D01 | d1 | E04 |
| 2-1-d1E04-1-0366 | 860.266 | OH | a2 | B13 | b2 | C03 | c2 | D02 | d1 | E04 |
| 2-1-d1E04-1-0367 | 860.266 | OH | a2 | B11 | b2 | 005 | c2 | D02 | d1 | E04 |
| 2-1-d1E04-1-0368 | 860.292 | OH | a1 | B07 | b1 | C13 | c1 | D01 | d1 | E04 |
| 2-1-d1E04-1-0369 | 860.303 | OH | a2 | B15 | b2 | C27 | c3 | D02 | d1 | E04 |
| 2-1-d1E04-1-0370 | 860.393 | OH | a1 | B05 | b2 | C07 | c1 | D01 | d1 | E04 |
| 2-1-d1E04-1-0371 | 861.208 | OH | a1 | B04 | b1 | C12 | c2 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0372 | 861.261 | OH | a2 | B12 | b2 | COS | c2 | D02 | d1 | E04 |
| 2-1-d1E04-1-0373 | 861.287 | OH | a2 | B16 | b2 | C19 | c3 | D02 | d1 | E04 |
| 2-1-d1E04-1-0374 | 861.287 | OH | a2 | B11 | b2 | C25 | c3 | D02 | d1 | E04 |
| 2-1-d1E04-1-0375 | 861.303 | OH | a2 | B14 | b1 | C32 | c3 | D02 | d1 | E04 |
| 2-1-d1E04-1-0376 | 862.227 | OH | a1 | B07 | b3 | C15 | c1 | D01 | d1 | E04 |
| 2-1-d1E04-1-0377 | 862.282 | OH | a2 | B12 | b2 | C25 | c3 | D02 | d1 | E04 |
| 2-1-d1E04-1-0378 | 862.282 | OH | a2 | B16 | b2 | C27 | c3 | D02 | d1 | E04 |
| 2-1-d1E04-1-0379 | 862.318 | OH | a1 | B03 | b1 | C30 | c3 | D02 | d1 | E04 |
| 2-1-d1E04-1-0380 | 862.318 | OH | a1 | B01 | b1 | C32 | c3 | D02 | d1 | E04 |
| 2-1-d1E04-1-0381 | 862.372 | OH | a1 | B06 | b2 | C07 | c1 | D01 | d1 | E04 |
| 2-1-d1E04-1-0382 | 863.223 | OH | a1 | B08 | b2 | C06 | c1 | D01 | d1 | E04 |
| 2-1-d1E04-1-0383 | 863.238 | OH | a1 | B10 | b1 | C12 | c1 | D01 | d1 | E04 |
| 2-1-d1E04-1-0384 | 863.246 | OH | a2 | B14 | b2 | C02 | c2 | D02 | d1 | E04 |
| 2-1-d1E04-1-0385 | 863.246 | OH | a2 | B11 | b2 | C04 | c2 | D02 | d1 | E04 |
| 2-1-d1E04-1-0386 | 863.266 | OH | a2 | B15 | b2 | C01 | c2 | D02 | d1 | E04 |
| 2-1-d1E04-1-0387 | 863.282 | OH | a2 | B14 | b2 | C21 | c3 | D02 | d1 | E04 |
| 2-1-d1E04-1-0388 | 863.282 | OH | a2 | B13 | b2 | C23 | c3 | D02 | d1 | E04 |
| 2-1-d1E04-1-0389 | 863.313 | OH | a1 | B02 | b1 | C32 | c3 | D02 | d1 | E04 |
| 2-1-d1E04-1-0390 | 863.372 | OH | a2 | B18 | b2 | C07 | c1 | D01 | d1 | E04 |
| 2-1-d1E04-1-0391 | 864.241 | OH | a2 | B12 | b2 | C04 | c2 | D02 | d1 | E04 |
| 2-1-d1E04-1-0392 | 864.241 | OH | a2 | B14 | b2 | C03 | c2 | D02 | d1 | E04 |
| 2-1-d1E04-1-0393 | 864.258 | OH | a1 | B07 | b1 | C14 | c1 | D01 | d1 | E04 |
| 2-1-d1E04-1-0394 | 864.261 | OH | a1 | B01 | b2 | C02 | c2 | D02 | d1 | E04 |
| 2-1-d1E04-1-0395 | 864.279 | OH | a1 | B07 | b2 | C33 | c3 | D02 | d1 | E04 |
| 2-1-d1E04-1-0396 | 864.292 | OH | a2 | B19 | b2 | C05 | c1 | D01 | d1 | E04 |
| 2-1-d1E04-1-0397 | 864.298 | OH | a1 | B05 | b3 | C08 | c1 | D01 | d1 | E04 |
| 2-1-d1E04-1-0398 | 864.298 | OH | a1 | B05 | b1 | C08 | c2 | D02 | d1 | E04 |
| 2-1-d1E04-1-0399 | 864.298 | OH | a1 | B03 | b2 | C19 | c3 | D02 | d1 | E04 |
| 2-1-d1E04-1-0400 | 864.298 | OH | a1 | B01 | b2 | C21 | c3 | D02 | d1 | E04 |
| 2-1-d1E04-1-0401 | 865.227 | OH | a2 | B13 | b2 | C06 | c2 | D02 | d1 | E04 |
| 2-1-d1E04-1-0402 | 865.245 | OH | a2 | B16 | b2 | C01 | c2 | D02 | d1 | E04 |
| 2-1-d1E04-1-0403 | 865.256 | OH | a1 | B02 | b2 | C02 | c2 | D02 | d1 | E04 |
| 2-1-d1E04-1-0404 | 865.256 | OH | a1 | BO1 | b2 | C03 | c2 | D02 | d1 | E04 |
| 2-1-d1E04-1-0405 | 865.276 | OH | a2 | B20 | b2 | C02 | c1 | D01 | d1 | E04 |
| 2-1-d1E04-1-0406 | 865.278 | OH | a2 | B17 | b1 | C30 | c3 | D02 | d1 | E04 |
| 2-1-d1E04-1-0407 | 865.293 | OH | a1 | B05 | b1 | C11 | c2 | D02 | d1 | E04 |
| 2-1-d1E04-1-0408 | 865.293 | OH | a1 | B02 | b2 | C21 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0409 | 865.293 | OH | a1 | B05 | b2 | C27 | c3 | D02 | d1 | E04 |
| 2-1-d1E04-1-0410 | 866.252 | OH | a1 | B02 | b2 | C03 | c2 | D02 | d1 | E04 |
| 2-1-d1E04-1-0411 | 866.271 | OH | a2 | B20 | b2 | C03 | c1 | D01 | d1 | E04 |
| 2-1-d1E04-1-0412 | 866.277 | OH | a1 | B06 | b3 | C08 | c1 | D01 | d1 | E04 |
| 2-1-d1E04-1-0413 | 866.277 | OH | a1 | B06 | b1 | C08 | c2 | D02 | d1 | E04 |
| 2-1-d1E04-1-0414 | 866.288 | OH | a1 | B05 | b1 | C10 | c2 | D02 | d1 | E04 |
| 2-1-d1E04-1-0415 | 866.293 | OH | a1 | B04 | b1 | C30 | c3 | D02 | d1 | E04 |
| 2-1-d1E04-1-0416 | 867.257 | OH | a2 | B17 | b2 | C19 | c3 | D02 | d1 | E04 |
| 2-1-d1E04-1-0417 | 867.257 | OH | a2 | B14 | b2 | C23 | c3 | D02 | d1 | E04 |
| 2-1-d1E04-1-0418 | 867.271 | OH | a2 | B19 | b2 | C04 | c1 | D01 | d1 | E04 |
| 2-1-d1E04-1-0419 | 867.272 | OH | a1 | B06 | b1 | C11 | c2 | D02 | d1 | E04 |
| 2-1-d1E04-1-0420 | 867.276 | OH | a2 | B18 | b3 | C08 | c1 | D01 | d1 | E04 |
| 2-1-d1E04-1-0421 | 867.276 | OH | a2 | B18 | b1 | C08 | c2 | D02 | d1 | E04 |
| 2-1-d1E04-1-0422 | 868.252 | OH | a2 | B17 | b2 | C27 | c3 | D02 | d1 | E04 |
| 2-1-d1E04-1-0423 | 868.256 | OH | a1 | B03 | b2 | C01 | c2 | D02 | d1 | E04 |
| 2-1-d1E04-1-0424 | 868.267 | OH | a1 | B06 | b1 | C10 | c2 | D02 | d1 | E04 |
| 2-1-d1E04-1-0425 | 868.271 | OH | a2 | B18 | b1 | C11 | c2 | D02 | d1 | E04 |
| 2-1-d1E04-1-0426 | 868.272 | OH | a1 | B04 | b2 | C19 | c3 | D02 | d1 | E04 |
| 2-1-d1E04-1-0427 | 868.272 | OH | a1 | B01 | b2 | C23 | c3 | D02 | d1 | E04 |
| 2-1-d1E04-1-0428 | 868.287 | OH | a1 | B09 | b2 | C02 | c1 | D01 | d1 | E04 |
| 2-1-d1E04-1-0429 | 868.323 | OH | a1 | B05 | b1 | C09 | c1 | D01 | d1 | E04 |
| 2-1-d1E04-1-0430 | 868.343 | OH | a1 | B07 | b2 | C07 | c1 | D01 | d1 | E04 |
| 2-1-d1E04-1-0431 | 869.202 | OH | a2 | B14 | b2 | C06 | c2 | D02 | d1 | E04 |
| 2-1-d1E04-1-0432 | 869.267 | OH | a2 | B18 | b1 | C10 | c2 | D02 | d1 | E04 |
| 2-1-d1E04-1-0433 | 869.267 | OH | a2 | B11 | b1 | C13 | c2 | D02 | d1 | E04 |
| 2-1-d1E04-1-0434 | 869.268 | OH | a1 | B02 | b2 | C23 | c3 | D02 | d1 | E04 |
| 2-1-d1E04-1-0435 | 869.268 | OH | a1 | B04 | b2 | C27 | c3 | D02 | d1 | E04 |
| 2-1-d1E04-1-0436 | 869.282 | OH | a1 | B09 | b2 | C03 | c1 | D01 | d1 | E04 |
| 2-1-d1E04-1-0437 | 870.218 | OH | a1 | B01 | b2 | C06 | c2 | D02 | d1 | E04 |
| 2-1-d1E04-1-0438 | 870.254 | OH | a1 | B05 | b3 | C06 | c1 | D01 | d1 | E04 |
| 2-1-d1E04-1-0439 | 870.262 | OH | a2 | B12 | b1 | C13 | c2 | D02 | d1 | E04 |
| 2-1-d1E04-1-0440 | 870.302 | OH | a1 | B06 | b1 | C09 | c1 | D01 | d1 | E04 |
| 2-1-d1E04-1-0441 | 871.202 | OH | a2 | B11 | b3 | C15 | c2 | D02 | d1 | E04 |
| 2-1-d1E04-1-0442 | 871.213 | OH | a1 | B02 | b2 | C06 | c2 | D02 | d1 | E04 |
| 2-1-d1E04-1-0443 | 871.216 | OH | a2 | B17 | b2 | C01 | c2 | D02 | d1 | E04 |
| 2-1-d1E04-1-0444 | 871.232 | OH | a2 | B20 | b2 | C06 | c1 | D01 | d1 | E04 |
| 2-1-d1E04-1-0445 | 871.249 | OH | a1 | B05 | b1 | C12 | c2 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0446 | 871.271 | OH | a2 | B11 | b3 | C16 | c1 | D01 | d1 | E04 |
| 2-1-d1E04-1-0447 | 871.302 | OH | a2 | B18 | b1 | C09 | c1 | D01 | d1 | E04 |
| 2-1-d1E04-1-0448 | 871.344 | OH | a2 | B13 | b1 | C29 | c3 | D02 | d1 | E04 |
| 2-1-d1E04-1-0449 | 871.344 | OH | a2 | B15 | b1 | C32 | c3 | D02 | d1 | E04 |
| 2-1-d1E04-1-0450 | 872.197 | OH | a2 | B12 | b3 | C15 | c2 | D02 | d1 | E04 |
| 2-1-d1E04-1-0451 | 872.231 | OH | a1 | B04 | b2 | C01 | c2 | D02 | d1 | E04 |
| 2-1-d1E04-1-0452 | 872.233 | OH | a1 | B06 | b3 | C06 | c1 | D01 | d1 | E04 |
| 2-1-d1E04-1-0453 | 872.247 | OH | a1 | B07 | b3 | C08 | c1 | D01 | d1 | E04 |
| 2-1-d1E04-1-0454 | 872.247 | OH | a1 | B07 | b1 | C08 | c2 | D02 | d1 | E04 |
| 2-1-d1E04-1-0455 | 872.266 | OH | a2 | B12 | b3 | C16 | c1 | D01 | d1 | E04 |
| 2-1-d1E04-1-0456 | 872.277 | OH | a1 | B08 | b2 | C05 | c1 | D01 | d1 | E04 |
| 2-1-d1E04-1-0457 | 873.228 | OH | a1 | B06 | b1 | C12 | c2 | D02 | d1 | E04 |
| 2-1-d1E04-1-0458 | 873.232 | OH | a2 | B18 | b3 | C06 | c1 | D01 | d1 | E04 |
| 2-1-d1E04-1-0459 | 873.232 | OH | a2 | B11 | b3 | C17 | c1 | D01 | d1 | E04 |
| 2-1-d1E04-1-0460 | 873.232 | OH | a2 | B11 | b1 | C14 | c2 | D02 | d1 | E04 |
| 2-1-d1E04-1-0461 | 873.243 | OH | a1 | B07 | b1 | C11 | c2 | D02 | d1 | E04 |
| 2-1-d1E04-1-0462 | 873.287 | OH | a2 | B15 | b2 | C02 | c2 | D02 | d1 | E04 |
| 2-1-d1E04-1-0463 | 873.292 | OH | a2 | B19 | b1 | C13 | c1 | D01 | d1 | E04 |
| 2-1-d1E04-1-0464 | 873.323 | OH | a2 | B15 | b2 | C21 | c3 | D02 | d1 | E04 |
| 2-1-d1E04-1-0465 | 873.323 | OH | a2 | B16 | b1 | C32 | c3 | D02 | d1 | E04 |
| 2-1-d1E04-1-0466 | 874.228 | OH | a2 | B12 | b3 | C17 | c1 | D01 | d1 | E04 |
| 2-1-d1E04-1-0467 | 874.228 | OH | a2 | B18 | b1 | C12 | c2 | D02 | d1 | E04 |
| 2-1-d1E04-1-0468 | 874.228 | OH | a2 | B12 | b1 | C14 | c2 | D02 | d1 | E04 |
| 2-1-d1E04-1-0469 | 874.238 | OH | a1 | B07 | b1 | C10 | c2 | D02 | d1 | E04 |
| 2-1-d1E04-1-0470 | 874.243 | OH | a1 | B09 | b2 | C06 | c1 | D01 | d1 | E04 |
| 2-1-d1E04-1-0471 | 874.261 | OH | a1 | B10 | b2 | C01 | c1 | D01 | d1 | E04 |
| 2-1-d1E04-1-0472 | 874.282 | OH | a2 | B15 | b2 | C03 | c2 | D02 | d1 | E04 |
| 2-1-d1E04-1-0473 | 874.282 | OH | a2 | B13 | b2 | C05 | c2 | D02 | d1 | E04 |
| 2-1-d1E04-1-0474 | 875.227 | OH | a2 | B19 | b3 | C15 | c1 | D01 | d1 | E04 |
| 2-1-d1E04-1-0475 | 875.257 | OH | a1 | B08 | b2 | C04 | c1 | D01 | d1 | E04 |
| 2-1-d1E04-1-0476 | 875.266 | OH | a2 | B16 | b2 | C02 | c2 | D02 | d1 | E04 |
| 2-1-d1E04-1-0477 | 875.302 | OH | a2 | B16 | b2 | C21 | c3 | D02 | d1 | E04 |
| 2-1-d1E04-1-0478 | 875.302 | OH | a2 | B13 | b2 | C25 | c3 | D02 | d1 | E04 |
| 2-1-d1E04-1-0479 | 875.319 | OH | a2 | B14 | b1 | C29 | c3 | D02 | d1 | E04 |
| 2-1-d1E04-1-0480 | 876.261 | OH | a2 | B16 | b2 | C03 | c2 | D02 | d1 | E04 |
| 2-1-d1E04-1-0481 | 876.273 | OH | a1 | B07 | b1 | C09 | c1 | D01 | d1 | E04 |
| 2-1-d1E04-1-0482 | 876.334 | OH | a1 | B05 | b1 | C30 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0483 | 876.334 | OH | a1 | B01 | b1 | C29 | c3 | D02 | d1 | E04 |
| 2-1-d1E04-1-0484 | 876.334 | OH | a1 | B03 | b1 | C32 | c3 | D02 | d1 | E04 |
| 2-1-d1E04-1-0485 | 877.258 | OH | a2 | B19 | b1 | C14 | c1 | D01 | d1 | E04 |
| 2-1-d1E04-1-0486 | 877.262 | OH | a2 | B13 | b2 | C04 | c2 | D02 | d1 | E04 |
| 2-1-d1E04-1-0487 | 877.279 | OH | a2 | B19 | b2 | C33 | c3 | D02 | d1 | E04 |
| 2-1-d1E04-1-0488 | 877.298 | OH | a2 | B15 | b2 | C23 | c3 | D02 | d1 | E04 |
| 2-1-d1E04-1-0489 | 877.318 | OH | a2 | B11 | b2 | C07 | c2 | D02 | d1 | E04 |
| 2-1-d1E04-1-0490 | 877.329 | OH | a1 | B02 | b1 | C29 | c3 | D02 | d1 | E04 |
| 2-1-d1E04-1-0491 | 878.204 | OH | a1 | B07 | b3 | C06 | c1 | D01 | d1 | E04 |
| 2-1-d1E04-1-0492 | 878.257 | OH | a2 | B14 | b2 | C05 | c2 | D02 | d1 | E04 |
| 2-1-d1E04-1-0493 | 878.277 | OH | a1 | B03 | b2 | C02 | c2 | D02 | d1 | E04 |
| 2-1-d1E04-1-0494 | 878.313 | OH | a2 | B12 | b2 | C07 | c2 | D02 | d1 | E04 |
| 2-1-d1E04-1-0495 | 878.313 | OH | a1 | B05 | b2 | C19 | c3 | D02 | d1 | E04 |
| 2-1-d1E04-1-0496 | 878.313 | OH | a1 | B03 | b2 | C21 | c3 | D02 | d1 | E04 |
| 2-1-d1E04-1-0497 | 878.313 | OH | a1 | B06 | b1 | C30 | c3 | D02 | d1 | E04 |
| 2-1-d1E04-1-0498 | 879.199 | OH | a1 | B07 | b1 | C12 | c2 | D02 | d1 | E04 |
| 2-1-d1E04-1-0499 | 879.243 | OH | a2 | B15 | b2 | C06 | c2 | D02 | d1 | E04 |
| 2-1-d1E04-1-0500 | 879.272 | OH | a1 | B03 | b2 | C03 | c2 | D02 | d1 | E04 |
| 2-1-d1E04-1-0501 | 879.272 | OH | a1 | B01 | b2 | C05 | c2 | D02 | d1 | E04 |
| 2-1-d1E04-1-0502 | 879.277 | OH | a2 | B16 | b2 | C23 | c3 | D02 | d1 | E04 |
| 2-1-d1E04-1-0503 | 879.277 | OH | a2 | B14 | b2 | C25 | c3 | D02 | d1 | E04 |
| 2-1-d1E04-1-0504 | 879.294 | OH | a2 | B17 | b1 | C32 | c3 | D02 | d1 | E04 |
| 2-1-d1E04-1-0505 | 879.308 | OH | a1 | B05 | b2 | C27 | c3 | D02 | d1 | E04 |
| 2-1-d1E04-1-0506 | 879.312 | OH | a2 | B18 | b1 | C30 | c3 | D02 | d1 | E04 |
| 2-1-d1E04-1-0507 | 880.267 | OH | a1 | B02 | b2 | C05 | c2 | D02 | d1 | E04 |
| 2-1-d1E04-1-0508 | 880.287 | OH | a2 | B20 | b2 | C05 | c1 | D01 | d1 | E04 |
| 2-1-d1E04-1-0509 | 880.292 | OH | a1 | B06 | b2 | C19 | c3 | D02 | d1 | E04 |
| 2-1-d1E04-1-0510 | 880.292 | OH | a1 | B01 | b2 | C25 | c3 | D02 | d1 | E04 |
| 2-1-d1E04-1-0511 | 880.309 | OH | a1 | B04 | b1 | C32 | c3 | D02 | d1 | E04 |
| 2-1-d1E04-1-0512 | 881.222 | OH | a2 | B16 | b2 | C06 | c2 | D02 | d1 | E04 |
| 2-1-d1E04-1-0513 | 881.222 | OH | a2 | B11 | b3 | C08 | c2 | D02 | d1 | E04 |
| 2-1-d1E04-1-0514 | 881.236 | OH | a2 | B17 | b2 | C02 | c2 | D02 | d1 | E04 |
| 2-1-d1E04-1-0515 | 881.236 | OH | a2 | B14 | b2 | C04 | c2 | D02 | d1 | E04 |
| 2-1-d1E04-1-0516 | 881.273 | OH | a2 | B17 | b2 | C21 | c3 | D02 | d1 | E04 |
| 2-1-d1E04-1-0517 | 881.278 | OH | a1 | B08 | b1 | C13 | c1 | D01 | d1 | E04 |
| 2-1-d1E04-1-0518 | 881.288 | OH | a1 | B02 | b2 | C25 | c3 | D02 | d1 | E04 |
| 2-1-d1E04-1-0519 | 881.288 | OH | a1 | B06 | b2 | C27 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0520 | 881.292 | OH | a2 | B18 | b2 | C19 | c3 | D02 | d1 | E04 |
| 2-1-d1E04-1-0521 | 881.343 | OH | a2 | B19 | b2 | C07 | c1 | D01 | d1 | E04 |
| 2-1-d1E04-1-0522 | 882.218 | OH | a2 | B12 | b3 | C08 | c2 | D02 | d1 | E04 |
| 2-1-d1E04-1-0523 | 882.232 | OH | a2 | B17 | b2 | C03 | c2 | D02 | d1 | E04 |
| 2-1-d1E04-1-0524 | 882.252 | OH | a1 | B04 | b2 | C02 | c2 | D02 | d1 | E04 |
| 2-1-d1E04-1-0525 | 882.252 | OH | a1 | B01 | b2 | C04 | c2 | D02 | d1 | E04 |
| 2-1-d1E04-1-0526 | 882.272 | OH | a1 | B05 | b2 | C01 | c2 | D02 | d1 | E04 |
| 2-1-d1E04-1-0527 | 882.287 | OH | a2 | B18 | b2 | C27 | c3 | D02 | d1 | E04 |
| 2-1-d1E04-1-0528 | 882.288 | OH | a1 | B04 | b2 | C21 | c3 | D02 | d1 | E04 |
| 2-1-d1E04-1-0529 | 882.288 | OH | a1 | B03 | b2 | C23 | c3 | D02 | d1 | E04 |
| 2-1-d1E04-1-0530 | 883.213 | OH | a1 | B08 | b3 | C15 | c1 | D01 | d1 | E04 |
| 2-1-d1E04-1-0531 | 883.247 | OH | a1 | B02 | b2 | C04 | c2 | D02 | d1 | E04 |
| 2-1-d1E04-1-0532 | 883.247 | OH | a1 | B04 | b2 | C03 | c2 | D02 | d1 | E04 |
| 2-1-d1E04-1-0533 | 883.266 | OH | a2 | B20 | b2 | C04 | c1 | D01 | d1 | E04 |
| 2-1-d1E04-1-0534 | 883.282 | OH | a2 | B13 | b1 | C13 | c2 | D02 | d1 | E04 |
| 2-1-d1E04-1-0535 | 883.298 | OH | a1 | B09 | b2 | C05 | c1 | D01 | d1 | E04 |
| 2-1-d1E04-1-0536 | 884.233 | OH | a1 | B03 | b2 | C06 | c2 | D02 | d1 | E04 |
| 2-1-d1E04-1-0537 | 884.251 | OH | a1 | B06 | b2 | C01 | c2 | D02 | d1 | E04 |
| 2-1-d1E04-1-0538 | 884.282 | OH | a1 | B10 | b2 | C02 | c1 | D01 | d1 | E04 |
| 2-1-d1E04-1-0539 | 884.284 | OH | a1 | B07 | b1 | C30 | c3 | D02 | d1 | E04 |
| 2-1-d1E04-1-0540 | 885.217 | OH | a2 | B13 | b3 | C15 | c2 | D02 | d1 | E04 |
| 2-1-d1E04-1-0541 | 885.244 | OH | a1 | B08 | b1 | C14 | c1 | D01 | d1 | E04 |
| 2-1-d1E04-1-0542 | 885.248 | OH | a2 | B19 | b3 | C08 | c1 | D01 | d1 | E04 |
| 2-1-d1E04-1-0543 | 885.248 | OH | a2 | B19 | b1 | C08 | c2 | D02 | d1 | E04 |
| 2-1-d1E04-1-0544 | 885.248 | OH | a2 | B11 | b1 | C09 | c2 | D02 | d1 | E04 |
| 2-1-d1E04-1-0545 | 885.248 | OH | a2 | B17 | b2 | C23 | c3 | D02 | d1 | E04 |
| 2-1-d1E04-1-0546 | 885.250 | OH | a2 | B18 | b2 | C01 | c2 | D02 | d1 | E04 |
| 2-1-d1E04-1-0547 | 885.265 | OH | a1 | B08 | b2 | C33 | c3 | D02 | d1 | E04 |
| 2-1-d1E04-1-0548 | 885.277 | OH | a1 | B10 | b2 | C03 | c1 | D01 | d1 | E04 |
| 2-1-d1E04-1-0549 | 885.287 | OH | a2 | B13 | b3 | C16 | c1 | D01 | d1 | E04 |
| 2-1-d1E04-1-0550 | 885.323 | OH | a2 | B11 | b1 | C31 | c3 | D02 | d1 | E04 |
| 2-1-d1E04-1-0551 | 885.359 | OH | a2 | B15 | b1 | C29 | c3 | D02 | d1 | E04 |
| 2-1-d1E04-1-0552 | 886.243 | OH | a2 | B12 | b1 | C09 | c2 | D02 | d1 | E04 |
| 2-1-d1E04-1-0553 | 886.243 | OH | a2 | B19 | b1 | C11 | c2 | D02 | d1 | E04 |
| 2-1-d1E04-1-0554 | 886.263 | OH | a1 | B07 | b2 | C19 | c3 | D02 | d1 | E04 |
| 2-1-d1E04-1-0555 | 886.263 | OH | a1 | B04 | b2 | C23 | c3 | D02 | d1 | E04 |
| 2-1-d1E04-1-0556 | 886.277 | OH | a1 | B09 | b2 | C04 | c1 | D01 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0557 | 886.318 | OH | a2 | B12 | b1 | C31 | c3 | D02 | d1 | E04 |
| 2-1-d1E04-1-0558 | 887.179 | OH | a2 | B11 | b3 | C06 | c2 | D02 | d1 | E04 |
| 2-1-d1E04-1-0559 | 887.193 | OH | a2 | B17 | b2 | C06 | c2 | D02 | d1 | E04 |
| 2-1-d1E04-1-0560 | 887.238 | OH | a2 | B19 | b1 | C10 | c2 | D02 | d1 | E04 |
| 2-1-d1E04-1-0561 | 887.248 | OH | a2 | B13 | b3 | C17 | c1 | D01 | d1 | E04 |
| 2-1-d1E04-1-0562 | 887.248 | OH | a2 | B13 | b1 | C14 | c2 | D02 | d1 | E04 |
| 2-1-d1E04-1-0563 | 887.257 | OH | a2 | B14 | b1 | C13 | c2 | D02 | d1 | E04 |
| 2-1-d1E04-1-0564 | 887.258 | OH | a1 | B07 | b2 | C27 | c3 | D02 | d1 | E04 |
| 2-1-d1E04-1-0565 | 887.339 | OH | a2 | B16 | b1 | C29 | c3 | D02 | d1 | E04 |
| 2-1-d1E04-1-0566 | 888.174 | OH | a2 | B12 | b3 | C06 | c2 | D02 | d1 | E04 |
| 2-1-d1E04-1-0567 | 888.208 | OH | a1 | B04 | b2 | C06 | c2 | D02 | d1 | E04 |
| 2-1-d1E04-1-0568 | 888.243 | OH | a2 | B11 | b3 | C18 | c1 | D01 | d1 | E04 |
| 2-1-d1E04-1-0569 | 888.272 | OH | a1 | B01 | b1 | C13 | c2 | D02 | d1 | E04 |
| 2-1-d1E04-1-0570 | 888.298 | OH | a2 | B15 | b2 | C05 | c2 | D02 | d1 | E04 |
| 2-1-d1E04-1-0571 | 889.192 | OH | a2 | B14 | b3 | C15 | c2 | D02 | d1 | E04 |
| 2-1-d1E04-1-0572 | 889.239 | OH | a2 | B12 | b3 | C18 | c1 | D01 | d1 | E04 |
| 2-1-d1E04-1-0573 | 889.262 | OH | a2 | B14 | b3 | C16 | c1 | D01 | d1 | E04 |
| 2-1-d1E04-1-0574 | 889.268 | OH | a1 | B02 | b1 | C13 | c2 | D02 | d1 | E04 |
| 2-1-d1E04-1-0575 | 889.273 | OH | a2 | B19 | b1 | C09 | c1 | D01 | d1 | E04 |
| 2-1-d1E04-1-0576 | 889.287 | OH | a2 | B20 | b1 | C13 | c1 | D01 | d1 | E04 |
| 2-1-d1E04-1-0577 | 889.318 | OH | a2 | B15 | b2 | C25 | c3 | D02 | d1 | E04 |
| 2-1-d1E04-1-0578 | 889.329 | OH | a1 | B08 | b2 | C07 | c1 | D01 | d1 | E04 |
| 2-1-d1E04-1-0579 | 890.207 | OH | a1 | B01 | b3 | C15 | c2 | D02 | d1 | E04 |
| 2-1-d1E04-1-0580 | 890.222 | OH | a1 | B07 | b2 | C01 | c2 | D02 | d1 | E04 |
| 2-1-d1E04-1-0581 | 890.238 | OH | a1 | B10 | b2 | C06 | c1 | D01 | d1 | E04 |
| 2-1-d1E04-1-0582 | 890.277 | OH | a1 | B01 | b3 | C16 | c1 | D01 | d1 | E04 |
| 2-1-d1E04-1-0583 | 890.277 | OH | a2 | B16 | b2 | C05 | c2 | D02 | d1 | E04 |
| 2-1-d1E04-1-0584 | 890.350 | OH | a1 | B03 | b1 | C29 | c3 | D02 | d1 | E04 |
| 2-1-d1E04-1-0585 | 890.350 | OH | a1 | B05 | b1 | C32 | c3 | D02 | d1 | E04 |
| 2-1-d1E04-1-0586 | 891.203 | OH | a1 | B02 | b3 | C15 | c2 | D02 | d1 | E04 |
| 2-1-d1E04-1-0587 | 891.204 | OH | a2 | B19 | b3 | C06 | c1 | D01 | d1 | E04 |
| 2-1-d1E04-1-0588 | 891.222 | OH | a2 | B20 | b3 | C15 | c1 | D01 | d1 | E04 |
| 2-1-d1E04-1-0589 | 891.223 | OH | a2 | B14 | b3 | C17 | c1 | D01 | d1 | E04 |
| 2-1-d1E04-1-0590 | 891.223 | OH | a2 | B14 | b1 | C14 | c2 | D02 | d1 | E04 |
| 2-1-d1E04-1-0591 | 891.272 | OH | a1 | B02 | b3 | C16 | c1 | D01 | d1 | E04 |
| 2-1-d1E04-1-0592 | 891.277 | OH | a2 | B15 | b2 | C04 | c2 | D02 | d1 | E04 |
| 2-1-d1E04-1-0593 | 891.297 | OH | a2 | B16 | b2 | C25 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0594 | 891.334 | OH | a2 | B13 | b2 | C07 | c2 | D02 | d1 | E04 |
| 2-1-d1E04-1-0595 | 892.199 | OH | a2 | B19 | b1 | C12 | c2 | D02 | d1 | E04 |
| 2-1-d1E04-1-0596 | 892.238 | OH | a1 | B01 | b3 | C17 | c1 | D01 | d1 | E04 |
| 2-1-d1E04-1-0597 | 892.238 | OH | a1 | B01 | b1 | C14 | c2 | D02 | d1 | E04 |
| 2-1-d1E04-1-0598 | 892.292 | OH | a1 | B05 | b2 | C02 | c2 | D02 | d1 | E04 |
| 2-1-d1E04-1-0599 | 892.298 | OH | a1 | B09 | b1 | C13 | c1 | D01 | d1 | E04 |
| 2-1-d1E04-1-0600 | 892.329 | OH | a1 | B05 | b2 | C21 | c3 | D02 | d1 | E04 |
| 2-1-d1E04-1-0601 | 892.329 | OH | a1 | B06 | b1 | C32 | c3 | D02 | d1 | E04 |
| 2-1-d1E04-1-0602 | 893.234 | OH | a1 | B02 | b3 | C17 | c1 | D01 | d1 | E04 |
| 2-1-d1E04-1-0603 | 893.234 | OH | a1 | B08 | b3 | C08 | c1 | D01 | d1 | E04 |
| 2-1-d1E04-1-0604 | 893.234 | OH | a1 | B08 | b1 | C08 | c2 | D02 | d1 | E04 |
| 2-1-d1E04-1-0605 | 893.234 | OH | a1 | B02 | b1 | C14 | c2 | D02 | d1 | E04 |
| 2-1-d1E04-1-0606 | 893.253 | OH | a2 | B20 | b1 | C14 | c1 | D01 | d1 | E04 |
| 2-1-d1E04-1-0607 | 893.256 | OH | a2 | B16 | b2 | C04 | c2 | D02 | d1 | E04 |
| 2-1-d1E04-1-0608 | 893.274 | OH | a2 | B20 | b2 | C33 | c3 | D02 | d1 | E04 |
| 2-1-d1E04-1-0609 | 893.288 | OH | a1 | B05 | b2 | C03 | c2 | D02 | d1 | E04 |
| 2-1-d1E04-1-0610 | 893.288 | OH | a1 | B03 | b2 | C05 | c2 | D02 | d1 | E04 |
| 2-1-d1E04-1-0611 | 893.309 | OH | a2 | B17 | b1 | C29 | c3 | D02 | d1 | E04 |
| 2-1-d1E04-1-0612 | 893.328 | OH | a2 | B18 | b1 | C32 | c3 | D02 | d1 | E04 |
| 2-1-d1E04-1-0613 | 894.229 | OH | a1 | B08 | b1 | C11 | c2 | D02 | d1 | E04 |
| 2-1-d1E04-1-0614 | 894.233 | OH | a1 | B09 | b3 | C15 | c1 | D01 | d1 | E04 |
| 2-1-d1E04-1-0615 | 894.272 | OH | a1 | B06 | b2 | C02 | c2 | D02 | d1 | E04 |
| 2-1-d1E04-1-0616 | 894.308 | OH | a1 | B06 | b2 | C21 | c3 | D02 | d1 | E04 |
| 2-1-d1E04-1-0617 | 894.308 | OH | a1 | B03 | b2 | C25 | c3 | D02 | d1 | E04 |
| 2-1-d1E04-1-0618 | 894.324 | OH | a1 | B04 | b1 | C29 | c3 | D02 | d1 | E04 |
| 2-1-d1E04-1-0619 | 895.224 | OH | a1 | B08 | b1 | C10 | c2 | D02 | d1 | E04 |
| 2-1-d1E04-1-0620 | 895.238 | OH | a2 | B13 | b3 | C08 | c2 | D02 | d1 | E04 |
| 2-1-d1E04-1-0621 | 895.267 | OH | a1 | B06 | b2 | C03 | c2 | D02 | d1 | E04 |
| 2-1-d1E04-1-0622 | 895.271 | OH | a2 | B18 | b2 | C02 | c2 | D02 | d1 | E04 |
| 2-1-d1E04-1-0623 | 895.307 | OH | a2 | B18 | b2 | C21 | c3 | D02 | d1 | E04 |
| 2-1-d1E04-1-0624 | 895.308 | OH | a2 | B14 | b2 | C07 | c2 | D02 | d1 | E04 |
| 2-1-d1E04-1-0625 | 896.247 | OH | a2 | B17 | b2 | C05 | c2 | D02 | d1 | E04 |
| 2-1-d1E04-1-0626 | 896.264 | OH | a1 | B09 | b1 | C14 | c1 | D01 | d1 | E04 |
| 2-1-d1E04-1-0627 | 896.266 | OH | a2 | B18 | b2 | C03 | c2 | D02 | d1 | E04 |
| 2-1-d1E04-1-0628 | 896.267 | OH | a1 | B03 | b2 | C04 | c2 | D02 | d1 | E04 |
| 2-1-d1E04-1-0629 | 896.285 | OH | a1 | B09 | b2 | C33 | c3 | D02 | d1 | E04 |
| 2-1-d1E04-1-0630 | 896.304 | OH | a1 | B05 | b2 | C23 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0631 | 896.324 | OH | a1 | B01 | b2 | C07 | c2 | D02 | d1 | E04 |
| 2-1-d1E04-1-0632 | 897.259 | OH | a1 | B08 | b1 | C09 | c1 | D01 | d1 | E04 |
| 2-1-d1E04-1-0633 | 897.263 | OH | a1 | B04 | b2 | C05 | c2 | D02 | d1 | E04 |
| 2-1-d1E04-1-0634 | 897.268 | OH | a2 | B17 | b2 | C25 | c3 | D02 | d1 | E04 |
| 2-1-d1E04-1-0635 | 897.284 | OH | a2 | B19 | b1 | C30 | c3 | D02 | d1 | E04 |
| 2-1-d1E04-1-0636 | 897.298 | OH | a2 | B15 | b1 | C13 | c2 | D02 | d1 | E04 |
| 2-1-d1E04-1-0637 | 897.319 | OH | a1 | B02 | b2 | C07 | c2 | D02 | d1 | E04 |
| 2-1-d1E04-1-0638 | 897.338 | OH | a2 | B20 | b2 | C07 | c1 | D01 | d1 | E04 |
| 2-1-d1E04-1-0639 | 898.249 | OH | a1 | B05 | b2 | C06 | c2 | D02 | d1 | E04 |
| 2-1-d1E04-1-0640 | 898.283 | OH | a1 | B06 | b2 | C23 | c3 | D02 | d1 | E04 |
| 2-1-d1E04-1-0641 | 898.283 | OH | a1 | B04 | b2 | C25 | c3 | D02 | d1 | E04 |
| 2-1-d1E04-1-0642 | 898.299 | OH | a1 | B07 | b1 | C32 | c3 | D02 | d1 | E04 |
| 2-1-d1E04-1-0643 | 899.190 | OH | a1 | B08 | b3 | C06 | c1 | D01 | d1 | E04 |
| 2-1-d1E04-1-0644 | 899.213 | OH | a2 | B14 | b3 | C08 | c2 | D02 | d1 | E04 |
| 2-1-d1E04-1-0645 | 899.227 | OH | a2 | B17 | b2 | C04 | c2 | D02 | d1 | E04 |
| 2-1-d1E04-1-0646 | 899.233 | OH | a2 | B15 | b3 | C15 | c2 | D02 | d1 | E04 |
| 2-1-d1E04-1-0647 | 899.263 | OH | a2 | B13 | b1 | C09 | c2 | D02 | d1 | E04 |
| 2-1-d1E04-1-0648 | 899.263 | OH | a2 | B19 | b2 | C19 | c3 | D02 | d1 | E04 |
| 2-1-d1E04-1-0649 | 899.277 | OH | a2 | B16 | b1 | C13 | c2 | D02 | d1 | E04 |
| 2-1-d1E04-1-0650 | 899.282 | OH | a2 | B18 | b2 | C23 | c3 | D02 | d1 | E04 |
| 2-1-d1E04-1-0651 | 899.292 | OH | a1 | B10 | b2 | C05 | c1 | D01 | d1 | E04 |
| 2-1-d1E04-1-0652 | 899.302 | OH | a2 | B15 | b3 | C16 | c1 | D01 | d1 | E04 |
| 2-1-d1E04-1-0653 | 899.339 | OH | a2 | B13 | b1 | C31 | c3 | D02 | d1 | E04 |
| 2-1-d1E04-1-0654 | 900.185 | OH | a1 | B08 | b1 | C12 | c2 | D02 | d1 | E04 |
| 2-1-d1E04-1-0655 | 900.228 | OH | a1 | B06 | b2 | C06 | c2 | D02 | d1 | E04 |
| 2-1-d1E04-1-0656 | 900.228 | OH | a1 | B01 | b3 | C08 | c2 | D02 | d1 | E04 |
| 2-1-d1E04-1-0657 | 900.242 | OH | a1 | B07 | b2 | C02 | c2 | D02 | d1 | E04 |
| 2-1-d1E04-1-0658 | 900.242 | OH | a1 | B04 | b2 | C04 | c2 | D02 | d1 | E04 |
| 2-1-d1E04-1-0659 | 900.259 | OH | a2 | B19 | b2 | C27 | c3 | D02 | d1 | E04 |
| 2-1-d1E04-1-0660 | 900.279 | OH | a1 | B07 | b2 | C21 | c3 | D02 | d1 | E04 |
| 2-1-d1E04-1-0661 | 900.349 | OH | a1 | B09 | b2 | C07 | c1 | D01 | d1 | E04 |
| 2-1-d1E04-1-0662 | 901.194 | OH | a2 | B13 | b3 | C06 | c2 | D02 | d1 | E04 |
| 2-1-d1E04-1-0663 | 901.212 | OH | a2 | B16 | b3 | C15 | c2 | D02 | d1 | E04 |
| 2-1-d1E04-1-0664 | 901.223 | OH | a1 | B02 | b3 | C08 | c2 | D02 | d1 | E04 |
| 2-1-d1E04-1-0665 | 901.227 | OH | a2 | B18 | b2 | C06 | c2 | D02 | d1 | E04 |
| 2-1-d1E04-1-0666 | 901.238 | OH | a1 | B07 | b2 | C03 | c2 | D02 | d1 | E04 |
| 2-1-d1E04-1-0667 | 901.243 | OH | a2 | B20 | b3 | C08 | c1 | D01 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0668 | 901.243 | OH | a2 | B20 | b1 | C08 | c2 | D02 | d1 | E04 |
| 2-1-d1E04-1-0669 | 901.264 | OH | a2 | B15 | b3 | C17 | c1 | D01 | d1 | E04 |
| 2-1-d1E04-1-0670 | 901.264 | OH | a2 | B15 | b1 | C14 | c2 | D02 | d1 | E04 |
| 2-1-d1E04-1-0671 | 901.282 | OH | a2 | B16 | b3 | C16 | c1 | D01 | d1 | E04 |
| 2-1-d1E04-1-0672 | 902.238 | OH | a2 | B20 | b1 | C11 | c2 | D02 | d1 | E04 |
| 2-1-d1E04-1-0673 | 902.259 | OH | a2 | B13 | b3 | C18 | c1 | D01 | d1 | E04 |
| 2-1-d1E04-1-0674 | 902.272 | OH | a1 | B10 | b2 | C04 | c1 | D01 | d1 | E04 |
| 2-1-d1E04-1-0675 | 902.288 | OH | a1 | B03 | b1 | C13 | c2 | D02 | d1 | E04 |
| 2-1-d1E04-1-0676 | 903.222 | OH | a2 | B19 | b2 | C01 | c2 | D02 | d1 | E04 |
| 2-1-d1E04-1-0677 | 903.233 | OH | a2 | B20 | b1 | C10 | c2 | D02 | d1 | E04 |
| 2-1-d1E04-1-0678 | 903.238 | OH | a2 | B14 | b1 | C09 | c2 | D02 | d1 | E04 |
| 2-1-d1E04-1-0679 | 903.243 | OH | a2 | B16 | b3 | C17 | c1 | D01 | d1 | E04 |
| 2-1-d1E04-1-0680 | 903.243 | OH | a2 | B16 | b1 | C14 | c2 | D02 | d1 | E04 |
| 2-1-d1E04-1-0681 | 903.314 | OH | a2 | B14 | b1 | C31 | c3 | D02 | d1 | E04 |
| 2-1-d1E04-1-0682 | 904.223 | OH | a1 | B03 | b3 | C15 | c2 | D02 | d1 | E04 |
| 2-1-d1E04-1-0683 | 904.254 | OH | a1 | B09 | b3 | C08 | c1 | D01 | d1 | E04 |
| 2-1-d1E04-1-0684 | 904.254 | OH | a1 | B09 | b1 | C08 | c2 | D02 | d1 | E04 |
| 2-1-d1E04-1-0685 | 904.254 | OH | a1 | B01 | b1 | C09 | c2 | D02 | d1 | E04 |
| 2-1-d1E04-1-0686 | 904.254 | OH | a1 | B07 | b2 | C23 | c3 | D02 | d1 | E04 |
| 2-1-d1E04-1-0687 | 904.292 | OH | a1 | B03 | b3 | C16 | c1 | D01 | d1 | E04 |
| 2-1-d1E04-1-0688 | 904.329 | OH | a1 | B01 | b1 | C31 | c3 | D02 | d1 | E04 |
| 2-1-d1E04-1-0689 | 904.365 | OH | a1 | B05 | b1 | C29 | c3 | D02 | d1 | E04 |
| 2-1-d1E04-1-0690 | 905.169 | OH | a2 | B14 | b3 | C06 | c2 | D02 | d1 | E04 |
| 2-1-d1E04-1-0691 | 905.248 | OH | a2 | B17 | b1 | C13 | c2 | D02 | d1 | E04 |
| 2-1-d1E04-1-0692 | 905.249 | OH | a1 | B02 | b1 | C09 | c2 | D02 | d1 | E04 |
| 2-1-d1E04-1-0693 | 905.249 | OH | a1 | B09 | b1 | C11 | c2 | D02 | d1 | E04 |
| 2-1-d1E04-1-0694 | 905.268 | OH | a2 | B20 | b1 | C09 | c1 | D01 | d1 | E04 |
| 2-1-d1E04-1-0695 | 905.270 | OH | a1 | B08 | b1 | C30 | c3 | D02 | d1 | E04 |
| 2-1-d1E04-1-0696 | 905.324 | OH | a1 | B02 | b1 | C31 | c3 | D02 | d1 | E04 |
| 2-1-d1E04-1-0697 | 905.349 | OH | a2 | B15 | b2 | C07 | c2 | D02 | d1 | E04 |
| 2-1-d1E04-1-0698 | 906.185 | OH | a1 | B01 | b3 | C06 | c2 | D02 | d1 | E04 |
| 2-1-d1E04-1-0699 | 906.199 | OH | a1 | B07 | b2 | C06 | c2 | D02 | d1 | E04 |
| 2-1-d1E04-1-0700 | 906.234 | OH | a2 | B14 | b3 | C18 | c1 | D01 | d1 | E04 |
| 2-1-d1E04-1-0701 | 906.244 | OH | a1 | B09 | b1 | C10 | c2 | D02 | d1 | E04 |
| 2-1-d1E04-1-0702 | 906.254 | OH | a1 | B03 | b3 | C17 | c1 | D01 | d1 | E04 |
| 2-1-d1E04-1-0703 | 906.254 | OH | a1 | B03 | b1 | C14 | c2 | D02 | d1 | E04 |
| 2-1-d1E04-1-0704 | 906.263 | OH | a1 | B04 | b1 | C13 | c2 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0705 | 906.344 | OH | a1 | B06 | b1 | C29 | c3 | D02 | d1 | E04 |
| 2-1-d1E04-1-0706 | 907.180 | OH | a1 | B02 | b3 | C06 | c2 | D02 | d1 | E04 |
| 2-1-d1E04-1-0707 | 907.183 | OH | a2 | B17 | b3 | C15 | c2 | D02 | d1 | E04 |
| 2-1-d1E04-1-0708 | 907.199 | OH | a2 | B20 | b3 | C06 | c1 | D01 | d1 | E04 |
| 2-1-d1E04-1-0709 | 907.249 | OH | a1 | B01 | b3 | C18 | c1 | D01 | d1 | E04 |
| 2-1-d1E04-1-0710 | 907.249 | OH | a1 | B08 | b2 | C19 | c3 | D02 | d1 | E04 |
| 2-1-d1E04-1-0711 | 907.252 | OH | a2 | B17 | b3 | C16 | c1 | D01 | d1 | E04 |
| 2-1-d1E04-1-0712 | 907.303 | OH | a1 | B05 | b2 | C05 | c2 | D02 | d1 | E04 |
| 2-1-d1E04-1-0713 | 907.328 | OH | a2 | B16 | b2 | C07 | c2 | D02 | d1 | E04 |
| 2-1-d1E04-1-0714 | 907.344 | OH | a2 | B18 | b1 | C29 | c3 | D02 | d1 | E04 |
| 2-1-d1E04-1-0715 | 908.194 | OH | a2 | B20 | b1 | C12 | c2 | D02 | d1 | E04 |
| 2-1-d1E04-1-0716 | 908.198 | OH | a1 | B04 | b3 | C15 | c2 | D02 | d1 | E04 |
| 2-1-d1E04-1-0717 | 908.244 | OH | a1 | B02 | b3 | C18 | c1 | D01 | d1 | E04 |
| 2-1-d1E04-1-0718 | 908.244 | OH | a1 | B08 | b2 | C27 | c3 | D02 | d1 | E04 |
| 2-1-d1E04-1-0719 | 908.267 | OH | a1 | B04 | b3 | C16 | c1 | D01 | d1 | E04 |
| 2-1-d1E04-1-0720 | 908.279 | OH | a1 | B09 | b1 | C09 | c1 | D01 | d1 | E04 |
| 2-1-d1E04-1-0721 | 908.293 | OH | a1 | B10 | b1 | C13 | c1 | D01 | d1 | E04 |
| 2-1-d1E04-1-0722 | 908.324 | OH | a1 | B05 | b2 | C25 | c3 | D02 | d1 | E04 |
| 2-1-d1E04-1-0723 | 909.214 | OH | a2 | B17 | b3 | C17 | c1 | D01 | d1 | E04 |
| 2-1-d1E04-1-0724 | 909.214 | OH | a2 | B17 | b1 | C14 | c2 | D02 | d1 | E04 |
| 2-1-d1E04-1-0725 | 909.254 | OH | a2 | B15 | b3 | C08 | c2 | D02 | d1 | E04 |
| 2-1-d1E04-1-0726 | 909.283 | OH | a1 | B06 | b2 | C05 | c2 | D02 | d1 | E04 |
| 2-1-d1E04-1-0727 | 910.210 | OH | a1 | B09 | b3 | C06 | c1 | D01 | d1 | E04 |
| 2-1-d1E04-1-0728 | 910.228 | OH | a1 | B10 | b3 | C15 | c1 | D01 | d1 | E04 |
| 2-1-d1E04-1-0729 | 910.229 | OH | a1 | B04 | b3 | C17 | c1 | D01 | d1 | E04 |
| 2-1-d1E04-1-0730 | 910.229 | OH | a1 | B04 | b1 | C14 | c2 | D02 | d1 | E04 |
| 2-1-d1E04-1-0731 | 910.282 | OH | a2 | B18 | b2 | C05 | c2 | D02 | d1 | E04 |
| 2-1-d1E04-1-0732 | 910.283 | OH | a1 | B05 | b2 | C04 | c2 | D02 | d1 | E04 |
| 2-1-d1E04-1-0733 | 910.303 | OH | a1 | B06 | b2 | C25 | c3 | D02 | d1 | E04 |
| 2-1-d1E04-1-0734 | 910.339 | OH | a1 | B03 | b2 | C07 | c2 | D02 | d1 | E04 |
| 2-1-d1E04-1-0735 | 911.205 | OH | a1 | B09 | b1 | C12 | c2 | D02 | d1 | E04 |
| 2-1-d1E04-1-0736 | 911.208 | OH | a1 | B08 | b2 | C01 | c2 | D02 | d1 | E04 |
| 2-1-d1E04-1-0737 | 911.233 | OH | a2 | B16 | b3 | C08 | c2 | D02 | d1 | E04 |
| 2-1-d1E04-1-0738 | 911.300 | OH | a2 | B19 | b1 | C32 | c3 | D02 | d1 | E04 |
| 2-1-d1E04-1-0739 | 911.302 | OH | a2 | B18 | b2 | C25 | c3 | D02 | d1 | E04 |
| 2-1-d1E04-1-0740 | 912.259 | OH | a1 | B10 | b1 | C14 | c1 | D01 | d1 | E04 |
| 2-1-d1E04-1-0741 | 912.262 | OH | a1 | B06 | b2 | C04 | c2 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0742 | 912.280 | OH | a1 | B10 | b2 | C33 | c3 | D02 | d1 | E04 |
| 2-1-d1E04-1-0743 | 912.315 | OH | a1 | B07 | b1 | C29 | c3 | D02 | d1 | E04 |
| 2-1-d1E04-1-0744 | 913.243 | OH | a2 | B19 | b2 | C02 | c2 | D02 | d1 | E04 |
| 2-1-d1E04-1-0745 | 913.262 | OH | a2 | B18 | b2 | C04 | c2 | D02 | d1 | E04 |
| 2-1-d1E04-1-0746 | 913.279 | OH | a2 | B15 | b1 | C09 | c2 | D02 | d1 | E04 |
| 2-1-d1E04-1-0747 | 913.279 | OH | a2 | B19 | b2 | C21 | c3 | D02 | d1 | E04 |
| 2-1-d1E04-1-0748 | 913.279 | OH | a2 | B20 | b1 | C30 | c3 | D02 | d1 | E04 |
| 2-1-d1E04-1-0749 | 913.299 | OH | a2 | B17 | b2 | C07 | c2 | D02 | d1 | E04 |
| 2-1-d1E04-1-0750 | 913.354 | OH | a2 | B15 | b1 | C31 | c3 | D02 | d1 | E04 |
| 2-1-d1E04-1-0751 | 914.238 | OH | a2 | B19 | b2 | C03 | c2 | D02 | d1 | E04 |
| 2-1-d1E04-1-0752 | 914.244 | OH | a1 | B03 | b3 | C08 | c2 | D02 | d1 | E04 |
| 2-1-d1E04-1-0753 | 914.314 | OH | a1 | B04 | b2 | C07 | c2 | D02 | d1 | E04 |
| 2-1-d1E04-1-0754 | 915.210 | OH | a2 | B15 | b3 | C06 | c2 | D02 | d1 | E04 |
| 2-1-d1E04-1-0755 | 915.253 | OH | a1 | B07 | b2 | C05 | c2 | D02 | d1 | E04 |
| 2-1-d1E04-1-0756 | 915.258 | OH | a2 | B16 | b1 | C09 | c2 | D02 | d1 | E04 |
| 2-1-d1E04-1-0757 | 915.258 | OH | a2 | B20 | b2 | C19 | c3 | D02 | d1 | E04 |
| 2-1-d1E04-1-0758 | 915.334 | OH | a2 | B16 | b1 | C31 | c3 | D02 | d1 | E04 |
| 2-1-d1E04-1-0759 | 916.254 | OH | a2 | B20 | b2 | C27 | c3 | D02 | d1 | E04 |
| 2-1-d1E04-1-0760 | 916.274 | OH | a1 | B07 | b2 | C25 | c3 | D02 | d1 | E04 |
| 2-1-d1E04-1-0761 | 916.275 | OH | a2 | B15 | b3 | C18 | c1 | D01 | d1 | E04 |
| 2-1-d1E04-1-0762 | 916.290 | OH | a1 | B09 | b1 | C30 | c3 | D02 | d1 | E04 |
| 2-1-d1E04-1-0763 | 916.304 | OH | a1 | B05 | b1 | C13 | c2 | D02 | d1 | E04 |
| 2-1-d1E04-1-0764 | 916.344 | OH | a1 | B10 | b2 | C07 | c1 | D01 | d1 | E04 |
| 2-1-d1E04-1-0765 | 917.189 | OH | a2 | B16 | b3 | C06 | c2 | D02 | d1 | E04 |
| 2-1-d1E04-1-0766 | 917.203 | OH | a2 | B17 | b3 | C08 | c2 | D02 | d1 | E04 |
| 2-1-d1E04-1-0767 | 917.254 | OH | a2 | B19 | b2 | C23 | c3 | D02 | d1 | E04 |
| 2-1-d1E04-1-0768 | 918.219 | OH | a1 | B04 | b3 | C08 | c2 | D02 | d1 | E04 |
| 2-1-d1E04-1-0769 | 918.233 | OH | a1 | B07 | b2 | C04 | c2 | D02 | d1 | E04 |
| 2-1-d1E04-1-0770 | 918.239 | OH | a1 | B05 | b3 | C15 | c2 | D02 | d1 | E04 |
| 2-1-d1E04-1-0771 | 918.254 | OH | a2 | B16 | b3 | C18 | c1 | D01 | d1 | E04 |
| 2-1-d1E04-1-0772 | 918.269 | OH | a1 | B03 | b1 | C09 | c2 | D02 | d1 | E04 |
| 2-1-d1E04-1-0773 | 918.269 | OH | a1 | B09 | b2 | C19 | c3 | D02 | d1 | E04 |
| 2-1-d1E04-1-0774 | 918.283 | OH | a1 | B06 | b1 | C13 | c2 | D02 | d1 | E04 |
| 2-1-d1E04-1-0775 | 918.308 | OH | a1 | B05 | b3 | C16 | c1 | D01 | d1 | E04 |
| 2-1-d1E04-1-0776 | 918.344 | OH | a1 | B03 | b1 | C31 | c3 | D02 | d1 | E04 |
| 2-1-d1E04-1-0777 | 919.199 | OH | a2 | B19 | b2 | C06 | c2 | D02 | d1 | E04 |
| 2-1-d1E04-1-0778 | 919.217 | OH | a2 | B20 | b2 | C01 | c2 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0779 | 919.264 | OH | a1 | B09 | b2 | C27 | c3 | D02 | d1 | E04 |
| 2-1-d1E04-1-0780 | 919.282 | OH | a2 | B18 | b1 | C13 | c2 | D02 | d1 | E04 |
| 2-1-d1E04-1-0781 | 919.286 | OH | a1 | B08 | b1 | C32 | c3 | D02 | d1 | E04 |
| 2-1-d1E04-1-0782 | 920.200 | OH | a1 | B03 | b3 | C06 | c2 | D02 | d1 | E04 |
| 2-1-d1E04-1-0783 | 920.218 | OH | a1 | B06 | b3 | C15 | c2 | D02 | d1 | E04 |
| 2-1-d1E04-1-0784 | 920.249 | OH | a1 | B10 | b3 | C08 | c1 | D01 | d1 | E04 |
| 2-1-d1E04-1-0785 | 920.249 | OH | a1 | B10 | b1 | C08 | c2 | D02 | d1 | E04 |
| 2-1-d1E04-1-0786 | 920.270 | OH | a1 | B05 | b3 | C17 | c1 | D01 | d1 | E04 |
| 2-1-d1E04-1-0787 | 920.270 | OH | a1 | B05 | b1 | C14 | c2 | D02 | d1 | E04 |
| 2-1-d1E04-1-0788 | 920.287 | OH | a1 | B06 | b3 | C16 | c1 | D01 | d1 | E04 |
| 2-1-d1E04-1-0789 | 921.217 | OH | a2 | B18 | b3 | C15 | c2 | D02 | d1 | E04 |
| 2-1-d1E04-1-0790 | 921.228 | OH | a1 | B08 | b2 | C02 | c2 | D02 | d1 | E04 |
| 2-1-d1E04-1-0791 | 921.229 | OH | a2 | B17 | b1 | C09 | c2 | D02 | d1 | E04 |
| 2-1-d1E04-1-0792 | 921.244 | OH | a1 | B10 | b1 | C11 | c2 | D02 | d1 | E04 |
| 2-1-d1E04-1-0793 | 921.265 | OH | a1 | B03 | b3 | C18 | c1 | D01 | d1 | E04 |
| 2-1-d1E04-1-0794 | 921.265 | OH | a1 | B08 | b2 | C21 | c3 | D02 | d1 | E04 |
| 2-1-d1E04-1-0795 | 921.287 | OH | a2 | B18 | b3 | C16 | c1 | D01 | d1 | E04 |
| 2-1-d1E04-1-0796 | 921.304 | OH | a2 | B17 | b1 | C31 | c3 | D02 | d1 | E04 |
| 2-1-d1E04-1-0797 | 922.224 | OH | a1 | B08 | b2 | C03 | c2 | D02 | d1 | E04 |
| 2-1-d1E04-1-0798 | 922.228 | OH | a1 | B09 | b2 | C01 | c2 | D02 | d1 | E04 |
| 2-1-d1E04-1-0799 | 922.239 | OH | a1 | B10 | b1 | C10 | c2 | D02 | d1 | E04 |
| 2-1-d1E04-1-0800 | 922.244 | OH | a1 | B04 | b1 | C09 | c2 | D02 | d1 | E04 |
| 2-1-d1E04-1-0801 | 922.249 | OH | a1 | B06 | b3 | C17 | c1 | D01 | d1 | E04 |
| 2-1-d1E04-1-0802 | 922.249 | OH | a1 | B06 | b1 | C14 | c2 | D02 | d1 | E04 |
| 2-1-d1E04-1-0803 | 922.319 | OH | a1 | B04 | b1 | C31 | c3 | D02 | d1 | E04 |
| 2-1-d1E04-1-0804 | 923.160 | OH | a2 | B17 | b3 | C06 | c2 | D02 | d1 | E04 |
| 2-1-d1E04-1-0805 | 923.248 | OH | a2 | B18 | b3 | C17 | c1 | D01 | d1 | E04 |
| 2-1-d1E04-1-0806 | 923.248 | OH | a2 | B18 | b1 | C14 | c2 | D02 | d1 | E04 |
| 2-1-d1E04-1-0807 | 924.175 | OH | a1 | B04 | b3 | C06 | c2 | D02 | d1 | E04 |
| 2-1-d1E04-1-0808 | 924.225 | OH | a2 | B17 | b3 | C18 | c1 | D01 | d1 | E04 |
| 2-1-d1E04-1-0809 | 924.254 | OH | a1 | B07 | b1 | C13 | c2 | D02 | d1 | E04 |
| 2-1-d1E04-1-0810 | 924.274 | OH | a1 | B10 | b1 | C09 | c1 | D01 | d1 | E04 |
| 2-1-d1E04-1-0811 | 924.355 | OH | a1 | B05 | b2 | C07 | c2 | D02 | d1 | E04 |
| 2-1-d1E04-1-0812 | 925.240 | OH | a1 | B04 | b3 | C18 | c1 | D01 | d1 | E04 |
| 2-1-d1E04-1-0813 | 925.240 | OH | a1 | B08 | b2 | C23 | c3 | D02 | d1 | E04 |
| 2-1-d1E04-1-0814 | 925.315 | OH | a2 | B19 | b1 | C29 | c3 | D02 | d1 | E04 |
| 2-1-d1E04-1-0815 | 926.189 | OH | a1 | B07 | b3 | C15 | c2 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0816 | 926.205 | OH | a1 | B10 | b3 | C06 | c1 | D01 | d1 | E04 |
| 2-1-d1E04-1-0817 | 926.258 | OH | a1 | B07 | b3 | C16 | c1 | D01 | d1 | E04 |
| 2-1-d1E04-1-0818 | 926.334 | OH | a1 | B06 | b2 | C07 | c2 | D02 | d1 | E04 |
| 2-1-d1E04-1-0819 | 927.185 | OH | a1 | B08 | b2 | C06 | c2 | D02 | d1 | E04 |
| 2-1-d1E04-1-0820 | 927.200 | OH | a1 | B10 | b1 | C12 | c2 | D02 | d1 | E04 |
| 2-1-d1E04-1-0821 | 927.295 | OH | a2 | B20 | b1 | C32 | c3 | D02 | d1 | E04 |
| 2-1-d1E04-1-0822 | 927.334 | OH | a2 | B18 | b2 | C07 | c2 | D02 | d1 | E04 |
| 2-1-d1E04-1-0823 | 928.219 | OH | a1 | B07 | b3 | C17 | c1 | D01 | d1 | E04 |
| 2-1-d1E04-1-0824 | 928.219 | OH | a1 | B07 | b1 | C14 | c2 | D02 | d1 | E04 |
| 2-1-d1E04-1-0825 | 928.254 | OH | a2 | B19 | b2 | C05 | c2 | D02 | d1 | E04 |
| 2-1-d1E04-1-0826 | 928.259 | OH | a1 | B05 | b3 | C08 | c2 | D02 | d1 | E04 |
| 2-1-d1E04-1-0827 | 929.238 | OH | a2 | B20 | b2 | C02 | c2 | D02 | d1 | E04 |
| 2-1-d1E04-1-0828 | 929.274 | OH | a2 | B20 | b2 | C21 | c3 | D02 | d1 | E04 |
| 2-1-d1E04-1-0829 | 929.274 | OH | a2 | B19 | b2 | C25 | c3 | D02 | d1 | E04 |
| 2-1-d1E04-1-0830 | 930.233 | OH | a2 | B20 | b2 | C03 | c2 | D02 | d1 | E04 |
| 2-1-d1E04-1-0831 | 930.239 | OH | a1 | B06 | b3 | C08 | c2 | D02 | d1 | E04 |
| 2-1-d1E04-1-0832 | 930.306 | OH | a1 | B09 | b1 | C32 | c3 | D02 | d1 | E04 |
| 2-1-d1E04-1-0833 | 931.233 | OH | a2 | B19 | b2 | C04 | c2 | D02 | d1 | E04 |
| 2-1-d1E04-1-0834 | 931.238 | OH | a2 | B18 | b3 | C08 | c2 | D02 | d1 | E04 |
| 2-1-d1E04-1-0835 | 932.249 | OH | a1 | B09 | b2 | C02 | c2 | D02 | d1 | E04 |
| 2-1-d1E04-1-0836 | 932.285 | OH | a1 | B05 | b1 | C09 | c2 | D02 | d1 | E04 |
| 2-1-d1E04-1-0837 | 932.285 | OH | a1 | B09 | b2 | C21 | c3 | D02 | d1 | E04 |
| 2-1-d1E04-1-0838 | 932.285 | OH | a1 | B10 | b1 | C30 | c3 | D02 | d1 | E04 |
| 2-1-d1E04-1-0839 | 932.305 | OH | a1 | B07 | b2 | C07 | c2 | D02 | d1 | E04 |
| 2-1-d1E04-1-0840 | 932.360 | OH | a1 | B05 | b1 | C31 | c3 | D02 | d1 | E04 |
| 2-1-d1E04-1-0841 | 933.244 | OH | a1 | B09 | b2 | C03 | c2 | D02 | d1 | E04 |
| 2-1-d1E04-1-0842 | 933.249 | OH | a2 | B20 | b2 | C23 | c3 | D02 | d1 | E04 |
| 2-1-d1E04-1-0843 | 933.301 | OH | a1 | B08 | b1 | C29 | c3 | D02 | d1 | E04 |
| 2-1-d1E04-1-0844 | 934.216 | OH | a1 | B05 | b3 | C06 | c2 | D02 | d1 | E04 |
| 2-1-d1E04-1-0845 | 934.264 | OH | a1 | B06 | b1 | C09 | c2 | D02 | d1 | E04 |
| 2-1-d1E04-1-0846 | 934.264 | OH | a1 | B10 | b2 | C19 | c3 | D02 | d1 | E04 |
| 2-1-d1E04-1-0847 | 934.339 | OH | a1 | B06 | b1 | C31 | c3 | D02 | d1 | E04 |
| 2-1-d1E04-1-0848 | 935.194 | OH | a2 | B20 | b2 | C06 | c2 | D02 | d1 | E04 |
| 2-1-d1E04-1-0849 | 935.233 | OH | a2 | B11 | b3 | C16 | c2 | D02 | d1 | E04 |
| 2-1-d1E04-1-0850 | 935.259 | OH | a1 | B10 | b2 | C27 | c3 | D02 | d1 | E04 |
| 2-1-d1E04-1-0851 | 935.263 | OH | a2 | B18 | b1 | C09 | c2 | D02 | d1 | E04 |
| 2-1-d1E04-1-0852 | 935.280 | OH | a1 | B05 | b3 | C18 | c1 | D01 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0853 | 935.339 | OH | a2 | B18 | b1 | C31 | c3 | D02 | d1 | E04 |
| 2-1-d1E04-1-0854 | 936.195 | OH | a1 | B06 | b3 | C06 | c2 | D02 | d1 | E04 |
| 2-1-d1E04-1-0855 | 936.209 | OH | a1 | B07 | b3 | C08 | c2 | D02 | d1 | E04 |
| 2-1-d1E04-1-0856 | 936.228 | OH | a2 | B12 | b3 | C16 | c2 | D02 | d1 | E04 |
| 2-1-d1E04-1-0857 | 936.239 | OH | a1 | B08 | b2 | C05 | c2 | D02 | d1 | E04 |
| 2-1-d1E04-1-0858 | 936.260 | OH | a1 | B09 | b2 | C23 | c3 | D02 | d1 | E04 |
| 2-1-d1E04-1-0859 | 937.194 | OH | a2 | B18 | b3 | C06 | c2 | D02 | d1 | E04 |
| 2-1-d1E04-1-0860 | 937.194 | OH | a2 | B11 | b3 | C17 | c2 | D02 | d1 | E04 |
| 2-1-d1E04-1-0861 | 937.254 | OH | a2 | B19 | b1 | C13 | c2 | D02 | d1 | E04 |
| 2-1-d1E04-1-0862 | 937.260 | OH | a1 | B06 | b3 | C18 | c1 | D01 | d1 | E04 |
| 2-1-d1E04-1-0863 | 937.260 | OH | a1 | B08 | b2 | C25 | c3 | D02 | d1 | E04 |
| 2-1-d1E04-1-0864 | 938.190 | OH | a2 | B12 | b3 | C17 | c2 | D02 | d1 | E04 |
| 2-1-d1E04-1-0865 | 938.205 | OH | a1 | B09 | b2 | C06 | c2 | D02 | d1 | E04 |
| 2-1-d1E04-1-0866 | 938.223 | OH | a1 | B10 | b2 | C01 | c2 | D02 | d1 | E04 |
| 2-1-d1E04-1-0867 | 938.259 | OH | a2 | B18 | b3 | C18 | c1 | D01 | d1 | E04 |
| 2-1-d1E04-1-0868 | 939.189 | OH | a2 | B19 | b3 | C15 | c2 | D02 | d1 | E04 |
| 2-1-d1E04-1-0869 | 939.219 | OH | a1 | B08 | b2 | C04 | c2 | D02 | d1 | E04 |
| 2-1-d1E04-1-0870 | 939.258 | OH | a2 | B19 | b3 | C16 | c1 | D01 | d1 | E04 |
| 2-1-d1E04-1-0871 | 940.235 | OH | a1 | B07 | b1 | C09 | c2 | D02 | d1 | E04 |
| 2-1-d1E04-1-0872 | 940.310 | OH | a1 | B07 | b1 | C31 | c3 | D02 | d1 | E04 |
| 2-1-d1E04-1-0873 | 941.220 | OH | a2 | B19 | b3 | C17 | c1 | D01 | d1 | E04 |
| 2-1-d1E04-1-0874 | 941.220 | OH | a2 | B19 | b1 | C14 | c2 | D02 | d1 | E04 |
| 2-1-d1E04-1-0875 | 941.310 | OH | a2 | B20 | b1 | C29 | c3 | D02 | d1 | E04 |
| 2-1-d1E04-1-0876 | 942.166 | OH | a1 | B07 | b3 | C06 | c2 | D02 | d1 | E04 |
| 2-1-d1E04-1-0877 | 943.230 | OH | a1 | B07 | b3 | C18 | c1 | D01 | d1 | E04 |
| 2-1-d1E04-1-0878 | 944.249 | OH | a2 | B20 | b2 | C05 | c2 | D02 | d1 | E04 |
| 2-1-d1E04-1-0879 | 944.321 | OH | a1 | B09 | b1 | C29 | c3 | D02 | d1 | E04 |
| 2-1-d1E04-1-0880 | 945.240 | OH | a1 | B08 | b1 | C13 | c2 | D02 | d1 | E04 |
| 2-1-d1E04-1-0881 | 945.269 | OH | a2 | B20 | b2 | C25 | c3 | D02 | d1 | E04 |
| 2-1-d1E04-1-0882 | 945.305 | OH | a2 | B19 | b2 | C07 | c2 | D02 | d1 | E04 |
| 2-1-d1E04-1-0883 | 946.301 | OH | a1 | B10 | b1 | C32 | c3 | D02 | d1 | E04 |
| 2-1-d1E04-1-0884 | 947.175 | OH | a1 | B08 | b3 | C15 | c2 | D02 | d1 | E04 |
| 2-1-d1E04-1-0885 | 947.228 | OH | a2 | B20 | b2 | C04 | c2 | D02 | d1 | E04 |
| 2-1-d1E04-1-0886 | 947.244 | OH | a1 | B08 | b3 | C16 | c1 | D01 | d1 | E04 |
| 2-1-d1E04-1-0887 | 947.259 | OH | a1 | B09 | b2 | C05 | c2 | D02 | d1 | E04 |
| 2-1-d1E04-1-0888 | 948.243 | OH | a1 | B10 | b2 | C02 | c2 | D02 | d1 | E04 |
| 2-1-d1E04-1-0889 | 948.28 | OH | a1 | B10 | b2 | C21 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0890 | 948.28 | OH | a1 | B09 | b2 | C25 | c3 | D02 | d1 | E04 |
| 2-1-d1E04-1-0891 | 949.206 | OH | a1 | B08 | b3 | C17 | c1 | D01 | d1 | E04 |
| 2-1-d1E04-1-0892 | 949.206 | OH | a1 | B08 | b1 | C14 | c2 | D02 | d1 | E04 |
| 2-1-d1E04-1-0893 | 949.210 | OH | a2 | B19 | b3 | C08 | c2 | D02 | d1 | E04 |
| 2-1-d1E04-1-0894 | 949.239 | OH | a1 | B10 | b2 | C03 | c2 | D02 | d1 | E04 |
| 2-1-d1E04-1-0895 | 949.249 | OH | a2 | B13 | b3 | C16 | c2 | D02 | d1 | E04 |
| 2-1-d1E04-1-0896 | 950.239 | OH | a1 | B09 | b2 | C04 | c2 | D02 | d1 | E04 |
| 2-1-d1E04-1-0897 | 951.210 | OH | a2 | B13 | b3 | C17 | c2 | D02 | d1 | E04 |
| 2-1-d1E04-1-0898 | 952.205 | OH | a2 | B11 | b3 | C18 | c2 | D02 | d1 | E04 |
| 2-1-d1E04-1-0899 | 952.255 | OH | a1 | B10 | b2 | C23 | c3 | D02 | d1 | E04 |
| 2-1-d1E04-1-0900 | 953.201 | OH | a2 | B12 | b3 | C18 | c2 | D02 | d1 | E04 |
| 2-1-d1E04-1-0901 | 953.223 | OH | a2 | B14 | b3 | C16 | c2 | D02 | d1 | E04 |
| 2-1-d1E04-1-0902 | 953.235 | OH | a2 | B19 | b1 | C09 | c2 | D02 | d1 | E04 |
| 2-1-d1E04-1-0903 | 953.249 | OH | a2 | B20 | b1 | C13 | c2 | D02 | d1 | E04 |
| 2-1-d1E04-1-0904 | 953.291 | OH | a1 | B08 | b2 | C07 | c2 | D02 | d1 | E04 |
| 2-1-d1E04-1-0905 | 953.310 | OH | a2 | B19 | b1 | C31 | c3 | D02 | d1 | E04 |
| 2-1-d1E04-1-0906 | 954.200 | OH | a1 | B10 | b2 | C06 | c2 | D02 | d1 | E04 |
| 2-1-d1E04-1-0907 | 954.239 | OH | a1 | B01 | b3 | C16 | c2 | D02 | d1 | E04 |
| 2-1-d1E04-1-0908 | 955.166 | OH | a2 | B19 | b3 | C06 | c2 | D02 | d1 | E04 |
| 2-1-d1E04-1-0909 | 955.184 | OH | a2 | B20 | b3 | C15 | c2 | D02 | d1 | E04 |
| 2-1-d1E04-1-0910 | 955.185 | OH | a2 | B14 | b3 | C17 | c2 | D02 | d1 | E04 |
| 2-1-d1E04-1-0911 | 955.234 | OH | a1 | B02 | b3 | C16 | c2 | D02 | d1 | E04 |
| 2-1-d1E04-1-0912 | 955.253 | OH | a2 | B20 | b3 | C16 | c1 | D01 | d1 | E04 |
| 2-1-d1E04-1-0913 | 956.200 | OH | a1 | B01 | b3 | C17 | c2 | D02 | d1 | E04 |
| 2-1-d1E04-1-0914 | 956.231 | OH | a2 | B19 | b3 | C18 | c1 | D01 | d1 | E04 |
| 2-1-d1E04-1-0915 | 956.260 | OH | a1 | B09 | b1 | C13 | c2 | D02 | d1 | E04 |
| 2-1-d1E04-1-0916 | 957.195 | OH | a1 | B02 | b3 | C17 | c2 | D02 | d1 | E04 |
| 2-1-d1E04-1-0917 | 957.195 | OH | a1 | B08 | b3 | C08 | c2 | D02 | d1 | E04 |
| 2-1-d1E04-1-0918 | 957.215 | OH | a2 | B20 | b3 | C17 | c1 | D01 | d1 | E04 |
| 2-1-d1E04-1-0919 | 957.215 | OH | a2 | B20 | b1 | C14 | c2 | D02 | d1 | E04 |
| 2-1-d1E04-1-0920 | 958.195 | OH | a1 | B09 | b3 | C15 | c2 | D02 | d1 | E04 |
| 2-1-d1E04-1-0921 | 958.264 | OH | a1 | B09 | b3 | C16 | c1 | D01 | d1 | E04 |
| 2-1-d1E04-1-0922 | 960.226 | OH | a1 | B09 | b3 | C17 | c1 | D01 | d1 | E04 |
| 2-1-d1E04-1-0923 | 960.226 | OH | a1 | B09 | b1 | C14 | c2 | D02 | d1 | E04 |
| 2-1-d1E04-1-0924 | 960.316 | OH | a1 | B10 | b1 | C29 | c3 | D02 | d1 | E04 |
| 2-1-d1E04-1-0925 | 961.221 | OH | a1 | B08 | b1 | C09 | c2 | D02 | d1 | E04 |
| 2-1-d1E04-1-0926 | 961.296 | OH | a1 | B08 | b1 | C31 | c3 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0927 | 961.300 | OH | a2 | B20 | b2 | C07 | c2 | D02 | d1 | E04 |
| 2-1-d1E04-1-0928 | 963.152 | OH | a1 | B08 | b3 | C06 | c2 | D02 | d1 | E04 |
| 2-1-d1E04-1-0929 | 963.254 | OH | a1 | B10 | b2 | C05 | c2 | D02 | d1 | E04 |
| 2-1-d1E04-1-0930 | 963.264 | OH | a2 | B15 | b3 | C16 | c2 | D02 | d1 | E04 |
| 2-1-d1E04-1-0931 | 964.216 | OH | a1 | B08 | b3 | C18 | c1 | D01 | d1 | E04 |
| 2-1-d1E04-1-0932 | 964.275 | OH | a1 | B10 | b2 | C25 | c3 | D02 | d1 | E04 |
| 2-1-d1E04-1-0933 | 964.311 | OH | a1 | B09 | b2 | C07 | c2 | D02 | d1 | E04 |
| 2-1-d1E04-1-0934 | 965.205 | OH | a2 | B20 | b3 | C08 | c2 | D02 | d1 | E04 |
| 2-1-d1E04-1-0935 | 965.226 | OH | a2 | B15 | b3 | C17 | c2 | D02 | d1 | E04 |
| 2-1-d1E04-1-0936 | 965.243 | OH | a2 | B16 | b3 | C16 | c2 | D02 | d1 | E04 |
| 2-1-d1E04-1-0937 | 966.221 | OH | a2 | B13 | b3 | C18 | c2 | D02 | d1 | E04 |
| 2-1-d1E04-1-0938 | 966.234 | OH | a1 | B10 | b2 | C04 | c2 | D02 | d1 | E04 |
| 2-1-d1E04-1-0939 | 967.205 | OH | a2 | B16 | b3 | C17 | c2 | D02 | d1 | E04 |
| 2-1-d1E04-1-0940 | 968.215 | OH | a1 | B09 | b3 | C08 | c2 | D02 | d1 | E04 |
| 2-1-d1E04-1-0941 | 968.254 | OH | a1 | B03 | b3 | C16 | c2 | D02 | d1 | E04 |
| 2-1-d1E04-1-0942 | 969.230 | OH | a2 | B20 | b1 | C09 | c2 | D02 | d1 | E04 |
| 2-1-d1E04-1-0943 | 969.305 | OH | a2 | B20 | b1 | C31 | c3 | D02 | d1 | E04 |
| 2-1-d1E04-1-0944 | 970.196 | OH | a2 | B14 | b3 | C18 | c2 | D02 | d1 | E04 |
| 2-1-d1E04-1-0945 | 970.216 | OH | a1 | B03 | b3 | C17 | c2 | D02 | d1 | E04 |
| 2-1-d1E04-1-0946 | 971.161 | OH | a2 | B20 | b3 | C06 | c2 | D02 | d1 | E04 |
| 2-1-d1E04-1-0947 | 971.211 | OH | a1 | B01 | b3 | C18 | c2 | D02 | d1 | E04 |
| 2-1-d1E04-1-0948 | 971.214 | OH | a2 | B17 | b3 | C16 | c2 | D02 | d1 | E04 |
| 2-1-d1E04-1-0949 | 972.206 | OH | a1 | B02 | b3 | C18 | c2 | D02 | d1 | E04 |
| 2-1-d1E04-1-0950 | 972.226 | OH | a2 | B20 | b3 | C18 | c1 | D01 | d1 | E04 |
| 2-1-d1E04-1-0951 | 972.229 | OH | a1 | B04 | b3 | C16 | c2 | D02 | d1 | E04 |
| 2-1-d1E04-1-0952 | 972.241 | OH | a1 | B09 | b1 | C09 | c2 | D02 | d1 | E04 |
| 2-1-d1E04-1-0953 | 972.255 | OH | a1 | B10 | b1 | C13 | c2 | D02 | d1 | E04 |
| 2-1-d1E04-1-0954 | 972.316 | OH | a1 | B09 | b1 | C31 | c3 | D02 | d1 | E04 |
| 2-1-d1E04-1-0955 | 973.176 | OH | a2 | B17 | b3 | C17 | c2 | D02 | d1 | E04 |
| 2-1-d1E04-1-0956 | 974.172 | OH | a1 | B09 | b3 | C06 | c2 | D02 | d1 | E04 |
| 2-1-d1E04-1-0957 | 974.190 | OH | a1 | B10 | b3 | C15 | c2 | D02 | d1 | E04 |
| 2-1-d1E04-1-0958 | 974.191 | OH | a1 | B04 | b3 | C17 | c2 | D02 | d1 | E04 |
| 2-1-d1E04-1-0959 | 974.259 | OH | a1 | B10 | b3 | C16 | c1 | D01 | d1 | E04 |
| 2-1-d1E04-1-0960 | 975.237 | OH | a1 | B09 | b3 | C18 | c1 | D01 | d1 | E04 |
| 2-1-d1E04-1-0961 | 976.221 | OH | a1 | B10 | b3 | C17 | c1 | D01 | d1 | E04 |
| 2-1-d1E04-1-0962 | 976.221 | OH | a1 | B10 | b1 | C14 | c2 | D02 | d1 | E04 |
| 2-1-d1E04-1-0963 | 980.237 | OH | a2 | B15 | b3 | C18 | c2 | D02 | d1 | E04 |

(continued)

| [Table 8-7-5] Compound that may be contained in mixture 2-1-d1E04-1 | | | | | | | | | | |
|---|---|---|---|---|---|---|---|---|---|---|
| ID | Exact Mass | | a | B | b | C | c | D | d | E |
| 2-1-d1E04-1-0964 | 980.306 | OH | a1 | B10 | b2 | C07 | c2 | D02 | d1 | E04 |
| 2-1-d1E04-1-0965 | 982.216 | OH | a2 | B16 | b3 | C18 | c2 | D02 | d1 | E04 |
| 2-1-d1E04-1-0966 | 982.270 | OH | a1 | B05 | b3 | C16 | c2 | D02 | d1 | E04 |
| 2-1-d1E04-1-0967 | 984.210 | OH | a1 | B10 | b3 | C08 | c2 | D02 | d1 | E04 |
| 2-1-d1E04-1-0968 | 984.231 | OH | a1 | B05 | b3 | C17 | c2 | D02 | d1 | E04 |
| 2-1-d1E04-1-0969 | 984.249 | OH | a1 | B06 | b3 | C16 | c2 | D02 | d1 | E04 |
| 2-1-d1E04-1-0970 | 985.227 | OH | a1 | B03 | b3 | C18 | c2 | D02 | d1 | E04 |
| 2-1-d1E04-1-0971 | 985.249 | OH | a2 | B18 | b3 | C16 | c2 | D02 | d1 | E04 |
| 2-1-d1E04-1-0972 | 986.211 | OH | a1 | B06 | b3 | C17 | c2 | D02 | d1 | E04 |
| 2-1-d1E04-1-0973 | 987.210 | OH | a2 | B18 | b3 | C17 | c2 | D02 | d1 | E04 |
| 2-1-d1E04-1-0974 | 988.186 | OH | a2 | B17 | b3 | C18 | c2 | D02 | d1 | E04 |
| 2-1-d1E04-1-0975 | 988.236 | OH | a1 | B10 | b1 | C09 | c2 | D02 | d1 | E04 |
| 2-1-d1E04-1-0976 | 988.311 | OH | a1 | B10 | b1 | C31 | c3 | D02 | d1 | E04 |
| 2-1-d1E04-1-0977 | 989.202 | OH | a1 | B04 | b3 | C18 | c2 | D02 | d1 | E04 |
| 2-1-d1E04-1-0978 | 990.167 | OH | a1 | B10 | b3 | C06 | c2 | D02 | d1 | E04 |
| 2-1-d1E04-1-0979 | 990.220 | OH | a1 | B07 | b3 | C16 | c2 | D02 | d1 | E04 |
| 2-1-d1E04-1-0980 | 991.231 | OH | a1 | B10 | b3 | C18 | c1 | D01 | d1 | E04 |
| 2-1-d1E04-1-0981 | 992.181 | OH | a1 | B07 | b3 | C17 | c2 | D02 | d1 | E04 |
| 2-1-d1E04-1-0982 | 999.242 | OH | a1 | B05 | b3 | C18 | c2 | D02 | d1 | E04 |
| 2-1-d1E04-1-0983 | 1001.22 | OH | a1 | B06 | b3 | C18 | c2 | D02 | d1 | E04 |
| 2-1-d1E04-1-0984 | 1002.22 | OH | a2 | B18 | b3 | C18 | c2 | D02 | d1 | E04 |
| 2-1-d1E04-1-0985 | 1003.22 | OH | a2 | B19 | b3 | C16 | c2 | D02 | d1 | E04 |
| 2-1-d1E04-1-0986 | 1005.18 | OH | a2 | B19 | b3 | C17 | c2 | D02 | d1 | E04 |
| 2-1-d1E04-1-0987 | 1007.19 | OH | a1 | B07 | b3 | C18 | c2 | D02 | d1 | E04 |
| 2-1-d1E04-1-0988 | 1011.21 | OH | a1 | B08 | b3 | C16 | c2 | D02 | d1 | E04 |
| 2-1-d1E04-1-0989 | 1013.17 | OH | a1 | B08 | b3 | C17 | c2 | D02 | d1 | E04 |
| 2-1-d1E04-1-0990 | 1019.22 | OH | a2 | B20 | b3 | C16 | c2 | D02 | d1 | E04 |
| 2-1-d1E04-1-0991 | 1020.19 | OH | a2 | B19 | b3 | C18 | c2 | D02 | d1 | E04 |
| 2-1-d1E04-1-0992 | 1021.18 | OH | a2 | B20 | b3 | C17 | c2 | D02 | d1 | E04 |
| 2-1-d1E04-1-0993 | 1022.23 | OH | a1 | B09 | b3 | C16 | c2 | D02 | d1 | E04 |
| 2-1-d1E04-1-0994 | 1024.19 | OH | a1 | B09 | b3 | C17 | c2 | D02 | d1 | E04 |
| 2-1-d1E04-1-0995 | 1028.18 | OH | a1 | B08 | b3 | C18 | c2 | D02 | d1 | E04 |
| 2-1-d1E04-1-0996 | 1036.19 | OH | a2 | B20 | b3 | C18 | c2 | D02 | d1 | E04 |
| 2-1-d1E04-1-0997 | 1038.22 | OH | a1 | B10 | b3 | C16 | c2 | D02 | d1 | E04 |
| 2-1-d1E04-1-0998 | 1039.20 | OH | a1 | B09 | b3 | C18 | c2 | D02 | d1 | E04 |
| 2-1-d1E04-1-0999 | 1040.18 | OH | a1 | B10 | b3 | C17 | c2 | D02 | d1 | E04 |
| 2-1-d1E04-1-1000 | 1055.19 | OH | a1 | B10 | b3 | C18 | c2 | D02 | d1 | E04 |

Example 8-8: Synthesis of mixture 2-1-m1E01-0 to mixture 2-1-m1E04-0 by methylation step m1

A reaction formula in step m1E01 performed using mixture 2-1-d1E01-0 will be illustrated below.

[Formula 644]

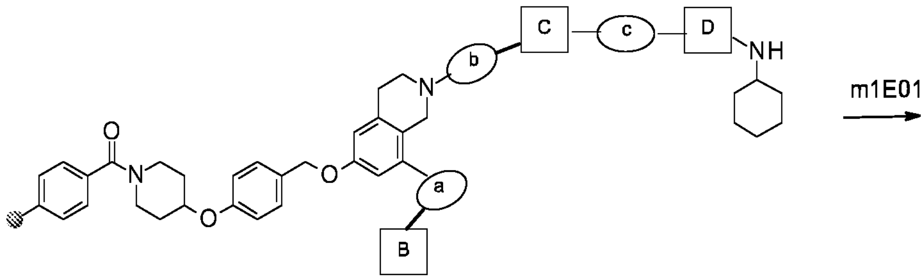


2-1-d1E01-0

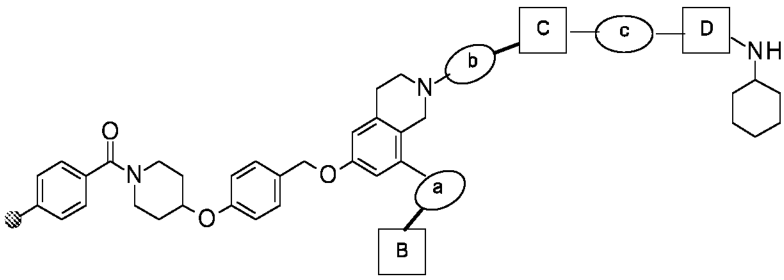

2-1-m1E01-0

In step m1, components having C19, C21, C23, C25 or C27 as core block C among the components contained in the mixtures are methylated to convert these core blocks to C20, C22, C24, C26 and C28, respectively.

Mixture 2-1-m1E01-0 to mixture 2-1-m1E04-0 were synthesized as follows using mixture 2-1-d1E01-0 to mixture 2-1-d1E04-0.

Separate solid phases (230 mg) described in Table 8-8-1, together with 1,4-dioxane (2.8 mL), were added to four 8 mL glass vials, respectively, and shaken at room temperature for 1 hour. DIPEA (121 μL, 690 μmol) and trimethyl phosphate (160 μL, 1.38 mmol) were added thereto, and each mixture was shaken at 80°C for 24 hours.

Solid-phase purification

The suspension of the reaction solution and the resin was transferred to a 6 mL column with a filter, filtered, and washed three times with NMP/water = 1/1 (5 mL), three times with NMP (5 mL), three times with 0.05 M $TBAHSO_4$ + DTBP in NMP (5 mL), three times with 0.05 M NMM in NMP (5 mL), three times with NMP/water = 1/1 (5 mL), three times with NMP (5 mL), three times with MeOH (5 mL), three times with DCM (5 mL), and three times with heptane (5 mL), and the obtained solid phase was dried under reduced pressure to obtain the mixtures described in Table 8-8-1.

[Table 392]

| [Table 8-8-1] Starting material mixture used and obtained mixture in step m1 | |
|---|---|
| Mixture used | Obtained mixture |
| 2-1-d1E01-0 | 2-1-m1E01-0 |
| 2-1-d1E02-0 | 2-1-m1E02-0 |

(continued)

| [Table 8-8-1] Starting material mixture used and obtained mixture in step m1 | |
|---|---|
| Mixture used | Obtained mixture |
| 2-1-d1E03-0 | 2-1-m1E03-0 |
| 2-1-d1E04-0 | 2-1-m1E04-0 |

Mixture 2-1-m1E01-0 to mixture 2-1-d1E04-0 can be treated by cleavage in the same manner as in Example 8-1-1 to obtain mixture 2-1-m1E01-1 to mixture 2-1-d1E04-1. The details of the mixtures are shown in Table 8-8-2 to Table 8-8-5.

In Table 8-8-2 to Table 8-8-5, each compound that may be contained in mixture 2-1- m1E01-1 to mixture 2-1-d1E04-1 is indicated by ID, and core blocks and linkers in combination in the structures of mixture 2-1-m1E01-0 to mixture 2-1-d1E04-0 are indicated by symbols or chemical formulas. The correspondence between the symbols and specific structures are shown in Figure 4. In Table 8-8-2 to Table 8-8-5, "×" is represented by a chemical formula.

[Formula 645]

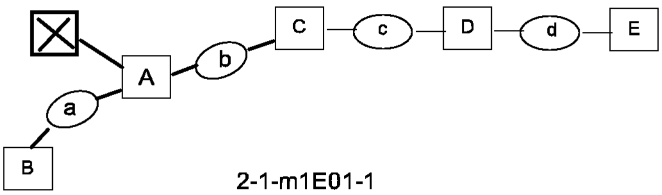


2-1-m1E01-1

For example, when ID is 2-1-m1E01-1-0181, the compound is represented by the following structural formula.

[Formula 646]

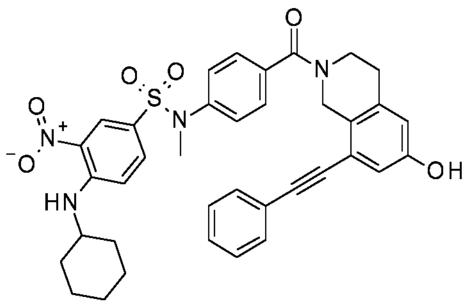

2-1-m1E01-1-0181

[Table 393]

| Notation in [Table 8-8-2] | | | | | | | | | |
|---|---|---|---|---|---|---|---|---|---|
| ID | | a | B | b | C | c | D | d | E |
| 2-1-m1E01-1-0181 | OH | a2 | B11 | b2 | C20 | c3 | D02 | d1 | E01 |

[Table 394]

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0001 | 586.258 | OH | a2 | B11 | b1 | C08 | c1 | D01 | d1 | E01 |
| 2-1-m1E01-1-0002 | 587.253 | OH | a2 | B12 | b1 | C08 | c1 | D01 | d1 | E01 |
| 2-1-m1E01-1-0003 | 587.253 | OH | a2 | B11 | b1 | C11 | c1 | D01 | d1 | E01 |
| 2-1-m1E01-1-0004 | 588.249 | OH | a2 | B11 | b1 | C10 | c1 | D01 | d1 | E01 |
| 2-1-m1E01-1-0005 | 588.249 | OH | a2 | B12 | b1 | C11 | c1 | D01 | d1 | E01 |
| 2-1-m1E01-1-0006 | 589.244 | OH | a2 | B12 | b1 | C10 | c1 | D01 | d1 | E01 |
| 2-1-m1E01-1-0007 | 593.210 | OH | a2 | B11 | b1 | C12 | c1 | D01 | d1 | E01 |
| 2-1-m1E01-1-0008 | 594.205 | OH | a2 | B12 | b1 | C12 | c1 | D01 | d1 | E01 |
| 2-1-m1E01-1-0009 | 600.274 | OH | a2 | B13 | b1 | C08 | c1 | D01 | d1 | E01 |
| 2-1-m1E01-1-0010 | 601.269 | OH | a2 | B13 | b1 | C11 | c1 | D01 | d1 | E01 |
| 2-1-m1E01-1-0011 | 602.264 | OH | a2 | B13 | b1 | C10 | c1 | D01 | d1 | E01 |
| 2-1-m1E01-1-0012 | 604.232 | OH | a2 | B11 | b2 | C01 | c1 | D01 | d1 | E01 |
| 2-1-m1E01-1-0013 | 604.249 | OH | a2 | B14 | b1 | C08 | c1 | D01 | d1 | E01 |
| 2-1-m1E01-1-0014 | 605.227 | OH | a2 | B12 | b2 | C01 | c1 | D01 | d1 | E01 |
| 2-1-m1E01-1-0015 | 605.244 | OH | a2 | B14 | b1 | C11 | c1 | D01 | d1 | E01 |
| 2-1-m1E01-1-0016 | 605.264 | OH | a1 | B01 | b1 | C08 | c1 | D01 | d1 | E01 |
| 2-1-m1E01-1-0017 | 606.239 | OH | a2 | B14 | b1 | C10 | c1 | D01 | d1 | E01 |
| 2-1-m1E01-1-0018 | 606.259 | OH | a1 | B02 | b1 | C08 | c1 | D01 | d1 | E01 |
| 2-1-m1E01-1-0019 | 606.259 | OH | a1 | B01 | b1 | C11 | c1 | D01 | d1 | E01 |
| 2-1-m1E01-1-0020 | 607.225 | OH | a2 | B13 | b1 | C12 | c1 | D01 | d1 | E01 |
| 2-1-m1E01-1-0021 | 607.254 | OH | a1 | B01 | b1 | C10 | c1 | D01 | d1 | E01 |
| 2-1-m1E01-1-0022 | 607.254 | OH | a1 | B02 | b1 | C11 | c1 | D01 | d1 | E01 |
| 2-1-m1E01-1-0023 | 608.250 | OH | a1 | B02 | b1 | C10 | c1 | D01 | d1 | E01 |
| 2-1-m1E01-1-0024 | 611.200 | OH | a2 | B14 | b1 | C12 | c1 | D01 | d1 | E01 |
| 2-1-m1E01-1-0025 | 612.215 | OH | a1 | B01 | b1 | C12 | c1 | D01 | d1 | E01 |
| 2-1-m1E01-1-0026 | 613.211 | OH | a1 | B02 | b1 | C12 | c1 | D01 | d1 | E01 |
| 2-1-m1E01-1-0027 | 614.253 | OH | a2 | B11 | b2 | C02 | c1 | D01 | d1 | E01 |
| 2-1-m1E01-1-0028 | 614.289 | OH | a2 | B15 | b1 | C08 | c1 | D01 | d1 | E01 |
| 2-1-m1E01-1-0029 | 615.248 | OH | a2 | B12 | b2 | C02 | c1 | D01 | d1 | E01 |
| 2-1-m1E01-1-0030 | 615.248 | OH | a2 | B11 | b2 | C03 | c1 | D01 | d1 | E01 |
| 2-1-m1E01-1-0031 | 615.285 | OH | a2 | B15 | b1 | C11 | c1 | D01 | d1 | E01 |
| 2-1-m1E01-1-0032 | 616.243 | OH | a2 | B12 | b2 | C03 | c1 | D01 | d1 | E01 |
| 2-1-m1E01-1-0033 | 616.269 | OH | a2 | B16 | b1 | C08 | c1 | D01 | d1 | E01 |
| 2-1-m1E01-1-0034 | 616.280 | OH | a2 | B15 | b1 | C10 | c1 | D01 | d1 | E01 |
| 2-1-m1E01-1-0035 | 617.264 | OH | a2 | B16 | b1 | C11 | c1 | D01 | d1 | E01 |
| 2-1-m1E01-1-0036 | 618.248 | OH | a2 | B13 | b2 | C01 | c1 | D01 | d1 | E01 |
| 2-1-m1E01-1-0037 | 618.259 | OH | a2 | B16 | b1 | C10 | c1 | D01 | d1 | E01 |
| 2-1-m1E01-1-0038 | 619.279 | OH | a1 | B03 | b1 | C08 | c1 | D01 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0039 | 620.209 | OH | a2 | B11 | b2 | C06 | c1 | D01 | d 1 | E01 |
| 2-1-m1E01-1-0040 | 620.275 | OH | a1 | B03 | b1 | C11 | c1 | D01 | d 1 | E01 |
| 2-1-m1E01-1-0041 | 621.205 | OH | a2 | B12 | b2 | C06 | c1 | D01 | d1 | E01 |
| 2-1-m1E01-1-0042 | 621.241 | OH | a2 | B15 | b1 | C12 | c1 | D01 | d1 | E01 |
| 2-1-m1E01-1-0043 | 621.270 | OH | a1 | B03 | b1 | C10 | c1 | D01 | d1 | E01 |
| 2-1-m1E01-1-0044 | 622.223 | OH | a2 | B14 | b2 | C01 | c1 | D01 | d1 | E01 |
| 2-1-m1E01-1-0045 | 622.239 | OH | a2 | B17 | b1 | C08 | c1 | D01 | d1 | E01 |
| 2-1-m1E01-1-0046 | 623.220 | OH | a2 | B16 | b1 | C12 | c1 | D01 | d1 | E01 |
| 2-1-m1E01-1-0047 | 623.234 | OH | a2 | B17 | b1 | C11 | c1 | D01 | d1 | E01 |
| 2-1-m1E01-1-0048 | 623.238 | OH | a1 | B01 | b2 | C01 | c1 | D01 | d1 | E01 |
| 2-1-m1E01-1-0049 | 623.254 | OH | a1 | B04 | b1 | C08 | c1 | D01 | d 1 | E01 |
| 2-1-m1E01-1-0050 | 624.230 | OH | a2 | B17 | b1 | C10 | c1 | D01 | d1 | E01 |
| 2-1-m1E01-1-0051 | 624.233 | OH | a1 | B02 | b2 | C01 | c1 | D01 | d1 | E01 |
| 2-1-m1E01-1-0052 | 624.250 | OH | a1 | B04 | b1 | C11 | c1 | D01 | d1 | E01 |
| 2-1-m1E01-1-0053 | 625.245 | OH | a1 | B04 | b1 | C10 | c1 | D01 | d1 | E01 |
| 2-1-m1E01-1-0054 | 626.231 | OH | a1 | B03 | b1 | C12 | c1 | D01 | d1 | E01 |
| 2-1-m1E01-1-0055 | 628.269 | OH | a2 | B13 | b2 | C02 | c1 | D01 | d1 | E01 |
| 2-1-m1E01-1-0056 | 629.191 | OH | a2 | B17 | b1 | C12 | c1 | D01 | d1 | E01 |
| 2-1-m1E01-1-0057 | 629.264 | OH | a2 | B13 | b2 | C03 | c1 | D01 | d1 | E01 |
| 2-1-m1E01-1-0058 | 629.264 | OH | a2 | B11 | b2 | C05 | c1 | D01 | d 1 | E01 |
| 2-1-m1E01-1-0059 | 630.206 | OH | a1 | B04 | b1 | C12 | c1 | D01 | d1 | E01 |
| 2-1-m1E01-1-0060 | 630.259 | OH | a2 | B12 | b2 | C05 | c1 | D01 | d1 | E01 |
| 2-1-m1E01-1-0061 | 632.243 | OH | a2 | B14 | b2 | C02 | c1 | D01 | d1 | E01 |
| 2-1-m1E01-1-0062 | 632.243 | OH | a2 | B11 | b2 | C04 | c1 | D01 | d1 | E01 |
| 2-1-m1E01-1-0063 | 632.263 | OH | a2 | B15 | b2 | C01 | c1 | D01 | d1 | E01 |
| 2-1-m1E01-1-0064 | 633.239 | OH | a2 | B12 | b2 | C04 | c1 | D01 | d1 | E01 |
| 2-1-m1E01-1-0065 | 633.239 | OH | a2 | B14 | b2 | C03 | c1 | D01 | d1 | E01 |
| 2-1-m1E01-1-0066 | 633.259 | OH | a1 | B01 | b2 | C02 | c1 | D01 | d1 | E01 |
| 2-1-m1E01-1-0067 | 633.295 | OH | a1 | B05 | b1 | C08 | c1 | D01 | d 1 | E01 |
| 2-1-m1E01-1-0068 | 634.225 | OH | a2 | B13 | b2 | C06 | c1 | D01 | d1 | E01 |
| 2-1-m1E01-1-0069 | 634.243 | OH | a2 | B16 | b2 | C01 | c1 | D01 | d1 | E01 |
| 2-1-m1E01-1-0070 | 634.254 | OH | a1 | B02 | b2 | C02 | c1 | D01 | d1 | E01 |
| 2-1-m1E01-1-0071 | 634.254 | OH | a1 | B01 | b2 | C03 | c1 | D01 | d1 | E01 |
| 2-1-m1E01-1-0072 | 634.290 | OH | a1 | B05 | b1 | C11 | c1 | D01 | d1 | E01 |
| 2-1-m1E01-1-0073 | 635.249 | OH | a1 | B02 | b2 | C03 | c1 | D01 | d1 | E01 |
| 2-1-m1E01-1-0074 | 635.274 | OH | a1 | B06 | b1 | C08 | c1 | D01 | d1 | E01 |
| 2-1-m1E01-1-0075 | 635.286 | OH | a1 | B05 | b1 | C10 | c1 | D01 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0076 | 636.270 | OH | a1 | B06 | b1 | C11 | c1 | D01 | d 1 | E01 |
| 2-1-m1E01-1-0077 | 636.274 | OH | a2 | B18 | b1 | C08 | c1 | D01 | d1 | E01 |
| 2-1-m1E01-1-0078 | 637.254 | OH | a1 | B03 | b2 | C01 | c1 | D01 | d1 | E01 |
| 2-1-m1E01-1-0079 | 637.265 | OH | a1 | B06 | b1 | C10 | c1 | D01 | d1 | E01 |
| 2-1-m1E01-1-0080 | 637.269 | OH | a2 | B18 | b1 | C11 | c1 | D01 | d1 | E01 |
| 2-1-m1E01-1-0081 | 638.200 | OH | a2 | B14 | b2 | C06 | c1 | D01 | d1 | E01 |
| 2-1-m1E01-1-0082 | 638.264 | OH | a2 | B18 | b1 | C10 | c1 | D01 | d1 | E01 |
| 2-1-m1E01-1-0083 | 638.264 | OH | a2 | B11 | b1 | C13 | c1 | D01 | d1 | E01 |
| 2-1-m1E01-1-0084 | 639.215 | OH | a1 | B01 | b2 | C06 | c1 | D01 | d1 | E01 |
| 2-1-m1E01-1-0085 | 639.259 | OH | a2 | B12 | b1 | C13 | c1 | D01 | d 1 | E01 |
| 2-1-m1E01-1-0086 | 640.199 | OH | a2 | B11 | b3 | C15 | c1 | D01 | d 1 | E01 |
| 2-1-m1E01-1-0087 | 640.210 | OH | a1 | B02 | b2 | C06 | c1 | D01 | d 1 | E01 |
| 2-1-m1E01-1-0088 | 640.213 | OH | a2 | B17 | b2 | C01 | c1 | D01 | d1 | E01 |
| 2-1-m1E01-1-0089 | 640.247 | OH | a1 | B05 | b1 | C12 | c1 | D01 | d1 | E01 |
| 2-1-m1E01-1-0090 | 641.194 | OH | a2 | B12 | b3 | C15 | c1 | D01 | d1 | E01 |
| 2-1-m1E01-1-0091 | 641.229 | OH | a1 | B04 | b2 | C01 | c1 | D01 | d1 | E01 |
| 2-1-m1E01-1-0092 | 641.245 | OH | a1 | B07 | b1 | C08 | c1 | D01 | d1 | E01 |
| 2-1-m1E01-1-0093 | 642.226 | OH | a1 | B06 | b1 | C12 | c1 | D01 | d1 | E01 |
| 2-1-m1E01-1-0094 | 642.230 | OH | a2 | B11 | b1 | C14 | c1 | D01 | d1 | E01 |
| 2-1-m1E01-1-0095 | 642.240 | OH | a1 | B07 | b1 | C11 | c1 | D01 | d1 | E01 |
| 2-1-m1E01-1-0096 | 642.251 | OH | a2 | B11 | b2 | C33 | c3 | D02 | d 1 | E01 |
| 2-1-m1E01-1-0097 | 642.284 | OH | a2 | B15 | b2 | C02 | c1 | D01 | d1 | E01 |
| 2-1-m1E01-1-0098 | 643.225 | OH | a2 | B18 | b1 | C12 | c1 | D01 | d1 | E01 |
| 2-1-m1E01-1-0099 | 643.225 | OH | a2 | B12 | b1 | C14 | c1 | D01 | d1 | E01 |
| 2-1-m1E01-1-0100 | 643.235 | OH | a1 | B07 | b1 | C10 | c1 | D01 | d1 | E01 |
| 2-1-m1E01-1-0101 | 643.246 | OH | a2 | B12 | b2 | C33 | c3 | D02 | d1 | E01 |
| 2-1-m1E01-1-0102 | 643.279 | OH | a2 | B15 | b2 | C03 | c1 | D01 | d1 | E01 |
| 2-1-m1E01-1-0103 | 643.279 | OH | a2 | B13 | b2 | C05 | c1 | D01 | d1 | E01 |
| 2-1-m1E01-1-0104 | 644.263 | OH | a2 | B16 | b2 | C02 | c1 | D01 | d1 | E01 |
| 2-1-m1E01-1-0105 | 645.259 | OH | a2 | B16 | b2 | C03 | c1 | D01 | d 1 | E01 |
| 2-1-m1E01-1-0106 | 646.259 | OH | a2 | B13 | b2 | C04 | c1 | D01 | d1 | E01 |
| 2-1-m1E01-1-0107 | 646.316 | OH | a2 | B11 | b2 | C07 | c1 | D01 | d1 | E01 |
| 2-1-m1E01-1-0108 | 647.254 | OH | a2 | B14 | b2 | COS | c1 | D01 | d1 | E01 |
| 2-1-m1E01-1-0109 | 647.274 | OH | a1 | B03 | b2 | C02 | c1 | D01 | d1 | E01 |
| 2-1-m1E01-1-0110 | 647.311 | OH | a2 | B12 | b2 | C07 | c1 | D01 | d1 | E01 |
| 2-1-m1E01-1-0111 | 648.197 | OH | a1 | B07 | b1 | C12 | c1 | D01 | d1 | E01 |
| 2-1-m1E01-1-0112 | 648.241 | OH | a2 | B15 | b2 | C06 | c1 | D01 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0113 | 648.270 | OH | a1 | B03 | b2 | C03 | c1 | D01 | d1 | E01 |
| 2-1-m1E01-1-0114 | 648.270 | OH | a1 | B01 | b2 | C05 | c1 | D01 | d 1 | E01 |
| 2-1-m1E01-1-0115 | 649.265 | OH | a1 | B02 | b2 | C05 | c1 | D01 | d1 | E01 |
| 2-1-m1E01-1-0116 | 650.220 | OH | a2 | B16 | b2 | C06 | c1 | D01 | d1 | E01 |
| 2-1-m1E01-1-0117 | 650.220 | OH | a2 | B11 | b3 | C08 | c1 | D01 | d1 | E01 |
| 2-1-m1E01-1-0118 | 650.220 | OH | a2 | B11 | b1 | C08 | c2 | D02 | d1 | E01 |
| 2-1-m1E01-1-0119 | 650.234 | OH | a2 | B17 | b2 | C02 | c1 | D01 | d1 | E01 |
| 2-1-m1E01-1-0120 | 650.234 | OH | a2 | B14 | b2 | C04 | c1 | D01 | d1 | E01 |
| 2-1-m1E01-1-0121 | 651.215 | OH | a2 | B12 | b3 | C08 | c1 | D01 | d1 | E01 |
| 2-1-m1E01-1-0122 | 651.215 | OH | a2 | B12 | b1 | C08 | c2 | D02 | d1 | E01 |
| 2-1-m1E01-1-0123 | 651.215 | OH | a2 | B11 | b1 | C11 | c2 | D02 | d 1 | E01 |
| 2-1-m1E01-1-0124 | 651.229 | OH | a2 | B17 | b2 | C03 | c1 | D01 | d1 | E01 |
| 2-1-m1E01-1-0125 | 651.249 | OH | a1 | B04 | b2 | C02 | c1 | D01 | d1 | E01 |
| 2-1-m1E01-1-0126 | 651.249 | OH | a1 | B01 | b2 | C04 | c1 | D01 | d1 | E01 |
| 2-1-m1E01-1-0127 | 651.269 | OH | a1 | B05 | b2 | C01 | c1 | D01 | d1 | E01 |
| 2-1-m1E01-1-0128 | 652.210 | OH | a2 | B11 | b1 | C10 | c2 | D02 | d1 | E01 |
| 2-1-m1E01-1-0129 | 652.210 | OH | a2 | B12 | b1 | C11 | c2 | D02 | d1 | E01 |
| 2-1-m1E01-1-0130 | 652.245 | OH | a1 | B02 | b2 | C04 | c1 | D01 | d1 | E01 |
| 2-1-m1E01-1-0131 | 652.245 | OH | a1 | B04 | b2 | C03 | c1 | D01 | d1 | E01 |
| 2-1-m1E01-1-0132 | 652.280 | OH | a2 | B13 | b1 | C13 | c1 | D01 | d 1 | E01 |
| 2-1-m1E01-1-0133 | 653.206 | OH | a2 | B12 | b1 | C10 | c2 | D02 | d 1 | E01 |
| 2-1-m1E01-1-0134 | 653.231 | OH | a1 | B03 | b2 | C06 | c1 | D01 | d 1 | E01 |
| 2-1-m1E01-1-0135 | 653.249 | OH | a1 | B06 | b2 | C01 | c1 | D01 | d1 | E01 |
| 2-1-m1E01-1-0136 | 654.215 | OH | a2 | B13 | b3 | C15 | c1 | D01 | d1 | E01 |
| 2-1-m1E01-1-0137 | 654.245 | OH | a2 | B19 | b1 | C08 | c1 | D01 | d1 | E01 |
| 2-1-m1E01-1-0138 | 654.245 | OH | a2 | B11 | b1 | C09 | c1 | D01 | d1 | E01 |
| 2-1-m1E01-1-0139 | 654.248 | OH | a2 | B18 | b2 | C01 | c1 | D01 | d1 | E01 |
| 2-1-m1E01-1-0140 | 655.241 | OH | a2 | B12 | b1 | C09 | c1 | D01 | d1 | E01 |
| 2-1-m1E01-1-0141 | 655.241 | OH | a2 | B19 | b1 | C11 | c1 | D01 | d1 | E01 |
| 2-1-m1E01-1-0142 | 656.176 | OH | a2 | B11 | b3 | C06 | c1 | D01 | d1 | E01 |
| 2-1-m1E01-1-0143 | 656.190 | OH | a2 | B17 | b2 | C06 | c1 | D01 | d 1 | E01 |
| 2-1-m1E01-1-0144 | 656.236 | OH | a2 | B19 | b1 | C10 | c1 | D01 | d1 | E01 |
| 2-1-m1E01-1-0145 | 656.246 | OH | a2 | B13 | b1 | C14 | c1 | D01 | d1 | E01 |
| 2-1-m1E01-1-0146 | 656.255 | OH | a2 | B14 | b1 | C13 | c1 | D01 | d1 | E01 |
| 2-1-m1E01-1-0147 | 656.267 | OH | a2 | B13 | b2 | C33 | c3 | D02 | d1 | E01 |
| 2-1-m1E01-1-0148 | 657.172 | OH | a2 | B12 | b3 | C06 | c1 | D01 | d1 | E01 |
| 2-1-m1E01-1-0149 | 657.172 | OH | a2 | B11 | b1 | C12 | c2 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0150 | 657.206 | OH | a1 | B04 | b2 | C06 | c1 | D01 | d1 | E01 |
| 2-1-m1E01-1-0151 | 657.270 | OH | a1 | B01 | b1 | C13 | c1 | D01 | d1 | E01 |
| 2-1-m1E01-1-0152 | 657.295 | OH | a2 | B15 | b2 | C05 | c1 | D01 | d 1 | E01 |
| 2-1-m1E01-1-0153 | 658.167 | OH | a2 | B12 | b1 | C12 | c2 | D02 | d1 | E01 |
| 2-1-m1E01-1-0154 | 658.190 | OH | a2 | B14 | b3 | C15 | c1 | D01 | d1 | E01 |
| 2-1-m1E01-1-0155 | 658.265 | OH | a1 | B02 | b1 | C13 | c1 | D01 | d1 | E01 |
| 2-1-m1E01-1-0156 | 659.205 | OH | a1 | B01 | b3 | C15 | c1 | D01 | d1 | E01 |
| 2-1-m1E01-1-0157 | 659.219 | OH | a1 | B07 | b2 | C01 | c1 | D01 | d1 | E01 |
| 2-1-m1E01-1-0158 | 659.274 | OH | a2 | B16 | b2 | C05 | c1 | D01 | d1 | E01 |
| 2-1-m1E01-1-0159 | 660.200 | OH | a1 | B02 | b3 | C15 | c1 | D01 | d1 | E01 |
| 2-1-m1E01-1-0160 | 660.221 | OH | a2 | B14 | b1 | C14 | c1 | D01 | d1 | E01 |
| 2-1-m1E01-1-0161 | 660.242 | OH | a2 | B14 | b2 | C33 | c3 | D02 | d 1 | E01 |
| 2-1-m1E01-1-0162 | 660.275 | OH | a2 | B15 | b2 | C04 | c1 | D01 | d1 | E01 |
| 2-1-m1E01-1-0163 | 660.331 | OH | a2 | B13 | b2 | C07 | c1 | D01 | d1 | E01 |
| 2-1-m1E01-1-0164 | 661.197 | OH | a2 | B19 | b1 | C12 | c1 | D01 | d1 | E01 |
| 2-1-m1E01-1-0165 | 661.236 | OH | a1 | B01 | b1 | C14 | c1 | D01 | d1 | E01 |
| 2-1-m1E01-1-0166 | 661.257 | OH | a1 | B01 | b2 | C33 | c3 | D02 | d1 | E01 |
| 2-1-m1E01-1-0167 | 661.290 | OH | a1 | B05 | b2 | C02 | c1 | D01 | d1 | E01 |
| 2-1-m1E01-1-0168 | 662.231 | OH | a1 | B08 | b1 | C08 | c1 | D01 | d1 | E01 |
| 2-1-m1E01-1-0169 | 662.231 | OH | a1 | B02 | b1 | C14 | c1 | D01 | d1 | E01 |
| 2-1-m1E01-1-0170 | 662.252 | OH | a1 | B02 | b2 | C33 | c3 | D02 | d 1 | E01 |
| 2-1-m1E01-1-0171 | 662.254 | OH | a2 | B16 | b2 | C04 | c1 | D01 | d1 | E01 |
| 2-1-m1E01-1-0172 | 662.256 | OH | a2 | B11 | b1 | C30 | c3 | D02 | d1 | E01 |
| 2-1-m1E01-1-0173 | 662.285 | OH | a1 | B05 | b2 | C03 | c1 | D01 | d1 | E01 |
| 2-1-m1E01-1-0174 | 662.285 | OH | a1 | B03 | b2 | C05 | c1 | D01 | d1 | E01 |
| 2-1-m1E01-1-0175 | 663.226 | OH | a1 | B08 | b1 | C11 | c1 | D01 | d1 | E01 |
| 2-1-m1E01-1-0176 | 663.252 | OH | a2 | B12 | b1 | C30 | c3 | D02 | d1 | E01 |
| 2-1-m1E01-1-0177 | 663.269 | OH | a1 | B06 | b2 | C02 | c1 | D01 | d1 | E01 |
| 2-1-m1E01-1-0178 | 664.222 | OH | a1 | B08 | b1 | C10 | c1 | D01 | d1 | E01 |
| 2-1-m1E01-1-0179 | 664.236 | OH | a2 | B13 | b3 | C08 | c1 | D01 | d 1 | E01 |
| 2-1-m1E01-1-0180 | 664.236 | OH | a2 | B13 | b1 | C08 | c2 | D02 | d 1 | E01 |
| 2-1-m1E01-1-0181 | 664.236 | OH | a2 | B11 | b2 | C20 | c3 | D02 | d 1 | E01 |
| 2-1-m1E01-1-0182 | 664.265 | OH | a1 | B06 | b2 | C03 | c1 | D01 | d1 | E01 |
| 2-1-m1E01-1-0183 | 664.269 | OH | a2 | B18 | b2 | C02 | c1 | D01 | d1 | E01 |
| 2-1-m1E01-1-0184 | 664.306 | OH | a2 | B14 | b2 | C07 | c1 | D01 | d1 | E01 |
| 2-1-m1E01-1-0185 | 665.231 | OH | a2 | B13 | b1 | C11 | c2 | D02 | d1 | E01 |
| 2-1-m1E01-1-0186 | 665.231 | OH | a2 | B12 | b2 | C20 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0187 | 665.231 | OH | a2 | B11 | b2 | C28 | c3 | D02 | d1 | E01 |
| 2-1-m1E01-1-0188 | 665.245 | OH | a2 | B17 | b2 | COS | c1 | D01 | d1 | E01 |
| 2-1-m1E01-1-0189 | 665.264 | OH | a2 | B18 | b2 | C03 | c1 | D01 | d1 | E01 |
| 2-1-m1E01-1-0190 | 665.265 | OH | a1 | B03 | b2 | C04 | c1 | D01 | d 1 | E01 |
| 2-1-m1E01-1-0191 | 665.321 | OH | a1 | B01 | b2 | C07 | c1 | D01 | d1 | E01 |
| 2-1-m1E01-1-0192 | 666.226 | OH | a2 | B13 | b1 | C10 | c2 | D02 | d1 | E01 |
| 2-1-m1E01-1-0193 | 666.226 | OH | a2 | B12 | b2 | C28 | c3 | D02 | d1 | E01 |
| 2-1-m1E01-1-0194 | 666.260 | OH | a1 | B04 | b2 | C05 | c1 | D01 | d1 | E01 |
| 2-1-m1E01-1-0195 | 666.295 | OH | a2 | B15 | b1 | C13 | c1 | D01 | d1 | E01 |
| 2-1-m1E01-1-0196 | 666.317 | OH | a1 | B02 | b2 | C07 | c1 | D01 | d1 | E01 |
| 2-1-m1E01-1-0197 | 667.246 | OH | a1 | B05 | b2 | C06 | c1 | D01 | d1 | E01 |
| 2-1-m1E01-1-0198 | 668.194 | OH | a2 | B11 | b2 | C01 | c2 | D02 | d1 | E01 |
| 2-1-m1E01-1-0199 | 668.210 | OH | a2 | B14 | b3 | C08 | c1 | D01 | d 1 | E01 |
| 2-1-m1E01-1-0200 | 668.210 | OH | a2 | B14 | b1 | C08 | c2 | D02 | d1 | E01 |
| 2-1-m1E01-1-0201 | 668.225 | OH | a2 | B17 | b2 | C04 | c1 | D01 | d1 | E01 |
| 2-1-m1E01-1-0202 | 668.230 | OH | a2 | B15 | b3 | C15 | c1 | D01 | d1 | E01 |
| 2-1-m1E01-1-0203 | 668.261 | OH | a2 | B13 | b1 | C09 | c1 | D01 | d1 | E01 |
| 2-1-m1E01-1-0204 | 668.275 | OH | a2 | B16 | b1 | C13 | c1 | D01 | d1 | E01 |
| 2-1-m1E01-1-0205 | 669.183 | OH | a1 | B08 | b1 | C12 | c1 | D01 | d1 | E01 |
| 2-1-m1E01-1-0206 | 669.189 | OH | a2 | B12 | b2 | C01 | c2 | D02 | d1 | E01 |
| 2-1-m1E01-1-0207 | 669.206 | OH | a2 | B14 | b1 | C11 | c2 | D02 | d1 | E01 |
| 2-1-m1E01-1-0208 | 669.226 | OH | a1 | B06 | b2 | C06 | c1 | D01 | d 1 | E01 |
| 2-1-m1E01-1-0209 | 669.226 | OH | a1 | B01 | b3 | C08 | c1 | D01 | d1 | E01 |
| 2-1-m1E01-1-0210 | 669.226 | OH | a1 | B01 | b1 | C08 | c2 | D02 | d1 | E01 |
| 2-1-m1E01-1-0211 | 669.240 | OH | a1 | B07 | b2 | C02 | c1 | D01 | d1 | E01 |
| 2-1-m1E01-1-0212 | 669.240 | OH | a1 | B04 | b2 | C04 | c1 | D01 | d1 | E01 |
| 2-1-m1E01-1-0213 | 670.192 | OH | a2 | B13 | b3 | C06 | c1 | D01 | d1 | E01 |
| 2-1-m1E01-1-0214 | 670.201 | OH | a2 | B14 | b1 | C10 | c2 | D02 | d1 | E01 |
| 2-1-m1E01-1-0215 | 670.210 | OH | a2 | B16 | b3 | C15 | c1 | D01 | d1 | E01 |
| 2-1-m1E01-1-0216 | 670.221 | OH | a1 | B02 | b3 | C08 | c1 | D01 | d1 | E01 |
| 2-1-m1E01-1-0217 | 670.221 | OH | a1 | B02 | b1 | C08 | c2 | D02 | d 1 | E01 |
| 2-1-m1E01-1-0218 | 670.221 | OH | a1 | B01 | b1 | C11 | c2 | D02 | d1 | E01 |
| 2-1-m1E01-1-0219 | 670.225 | OH | a2 | B18 | b2 | C06 | c1 | D01 | d1 | E01 |
| 2-1-m1E01-1-0220 | 670.235 | OH | a1 | B07 | b2 | C03 | c1 | D01 | d1 | E01 |
| 2-1-m1E01-1-0221 | 670.240 | OH | a2 | B20 | b1 | C08 | c1 | D01 | d1 | E01 |
| 2-1-m1E01-1-0222 | 670.261 | OH | a2 | B15 | b1 | C14 | c1 | D01 | d1 | E01 |
| 2-1-m1E01-1-0223 | 670.283 | OH | a2 | B15 | b2 | C33 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0224 | 671.187 | OH | a2 | B13 | b1 | C12 | c2 | D02 | d1 | E01 |
| 2-1-m1E01-1-0225 | 671.216 | OH | a1 | B01 | b1 | C10 | c2 | D02 | d1 | E01 |
| 2-1-m1E01-1-0226 | 671.216 | OH | a1 | B02 | b1 | C11 | c2 | D02 | d 1 | E01 |
| 2-1-m1E01-1-0227 | 671.236 | OH | a2 | B20 | b1 | C11 | c1 | D01 | d 1 | E01 |
| 2-1-m1E01-1-0228 | 671.286 | OH | a1 | B03 | b1 | C13 | c1 | D01 | d 1 | E01 |
| 2-1-m1E01-1-0229 | 672.211 | OH | a1 | B02 | b1 | C10 | c2 | D02 | d1 | E01 |
| 2-1-m1E01-1-0230 | 672.220 | OH | a2 | B19 | b2 | C01 | c1 | D01 | d1 | E01 |
| 2-1-m1E01-1-0231 | 672.231 | OH | a2 | B20 | b1 | C10 | c1 | D01 | d1 | E01 |
| 2-1-m1E01-1-0232 | 672.236 | OH | a2 | B14 | b1 | C09 | c1 | D01 | d1 | E01 |
| 2-1-m1E01-1-0233 | 672.241 | OH | a2 | B16 | b1 | C14 | c1 | D01 | d1 | E01 |
| 2-1-m1E01-1-0234 | 672.262 | OH | a2 | B16 | b2 | C33 | c3 | D02 | d1 | E01 |
| 2-1-m1E01-1-0235 | 673.221 | OH | a1 | B03 | b3 | C15 | c1 | D01 | d1 | E01 |
| 2-1-m1E01-1-0236 | 673.251 | OH | a1 | B09 | b1 | C08 | c1 | D01 | d1 | E01 |
| 2-1-m1E01-1-0237 | 673.251 | OH | a1 | B01 | b1 | C09 | c1 | D01 | d 1 | E01 |
| 2-1-m1E01-1-0238 | 674.167 | OH | a2 | B14 | b3 | C06 | c1 | D01 | d1 | E01 |
| 2-1-m1E01-1-0239 | 674.245 | OH | a2 | B17 | b1 | C13 | c1 | D01 | d1 | E01 |
| 2-1-m1E01-1-0240 | 674.246 | OH | a1 | B02 | b1 | C09 | c1 | D01 | d1 | E01 |
| 2-1-m1E01-1-0241 | 674.246 | OH | a1 | B09 | b1 | C11 | c1 | D01 | d1 | E01 |
| 2-1-m1E01-1-0242 | 674.347 | OH | a2 | B15 | b2 | C07 | c1 | D01 | d1 | E01 |
| 2-1-m1E01-1-0243 | 675.162 | OH | a2 | B14 | b1 | C12 | c2 | D02 | d1 | E01 |
| 2-1-m1E01-1-0244 | 675.182 | OH | a1 | B01 | b3 | C06 | c1 | D01 | d1 | E01 |
| 2-1-m1E01-1-0245 | 675.196 | OH | a1 | B07 | b2 | C06 | c1 | D01 | d1 | E01 |
| 2-1-m1E01-1-0246 | 675.242 | OH | a1 | B09 | b1 | C10 | c1 | D01 | d 1 | E01 |
| 2-1-m1E01-1-0247 | 675.252 | OH | a1 | B03 | b1 | C14 | c1 | D01 | d1 | E01 |
| 2-1-m1E01-1-0248 | 675.261 | OH | a1 | B04 | b1 | C13 | c1 | D01 | d1 | E01 |
| 2-1-m1E01-1-0249 | 675.273 | OH | a1 | B03 | b2 | C33 | c3 | D02 | d1 | E01 |
| 2-1-m1E01-1-0250 | 676.177 | OH | a1 | B02 | b3 | C06 | c1 | D01 | d1 | E01 |
| 2-1-m1E01-1-0251 | 676.177 | OH | a1 | B01 | b1 | C12 | c2 | D02 | d1 | E01 |
| 2-1-m1E01-1-0252 | 676.180 | OH | a2 | B17 | b3 | C15 | c1 | D01 | d1 | E01 |
| 2-1-m1E01-1-0253 | 676.272 | OH | a2 | B13 | b1 | C30 | c3 | D02 | d1 | E01 |
| 2-1-m1E01-1-0254 | 676.272 | OH | a2 | B11 | b1 | C32 | c3 | D02 | d1 | E01 |
| 2-1-m1E01-1-0255 | 676.301 | OH | a1 | B05 | b2 | C05 | c1 | D01 | d 1 | E01 |
| 2-1-m1E01-1-0256 | 676.326 | OH | a2 | B16 | b2 | C07 | c1 | D01 | d1 | E01 |
| 2-1-m1E01-1-0257 | 677.173 | OH | a1 | B02 | b1 | C12 | c2 | D02 | d1 | E01 |
| 2-1-m1E01-1-0258 | 677.192 | OH | a2 | B20 | b1 | C12 | c1 | D01 | d1 | E01 |
| 2-1-m1E01-1-0259 | 677.196 | OH | a1 | B04 | b3 | C15 | c1 | D01 | d1 | E01 |
| 2-1-m1E01-1-0260 | 677.267 | OH | a2 | B12 | b1 | C32 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0261 | 678.211 | OH | a2 | B17 | b1 | C14 | c1 | D01 | d1 | E01 |
| 2-1-m1E01-1-0262 | 678.215 | OH | a2 | B11 | b2 | C02 | c2 | D02 | d1 | E01 |
| 2-1-m1E01-1-0263 | 678.232 | OH | a2 | B17 | b2 | C33 | c3 | D02 | d1 | E01 |
| 2-1-m1E01-1-0264 | 678.251 | OH | a2 | B15 | b3 | C08 | c1 | D01 | d 1 | E01 |
| 2-1-m1E01-1-0265 | 678.251 | OH | a2 | B15 | b1 | C08 | c2 | D02 | d1 | E01 |
| 2-1-m1E01-1-0266 | 678.251 | OH | a2 | B13 | b2 | C20 | c3 | D02 | d1 | E01 |
| 2-1-m1E01-1-0267 | 678.251 | OH | a2 | B11 | b2 | C22 | c3 | D02 | d1 | E01 |
| 2-1-m1E01-1-0268 | 678.280 | OH | a1 | B06 | b2 | C05 | c1 | D01 | d1 | E01 |
| 2-1-m1E01-1-0269 | 679.210 | OH | a2 | B12 | b2 | C02 | c2 | D02 | d1 | E01 |
| 2-1-m1E01-1-0270 | 679.210 | OH | a2 | B11 | b2 | C03 | c2 | D02 | d1 | E01 |
| 2-1-m1E01-1-0271 | 679.226 | OH | a1 | B04 | b1 | C14 | c1 | D01 | d1 | E01 |
| 2-1-m1E01-1-0272 | 679.246 | OH | a2 | B15 | b1 | C11 | c2 | D02 | d1 | E01 |
| 2-1-m1E01-1-0273 | 679.246 | OH | a2 | B12 | b2 | C22 | c3 | D02 | d 1 | E01 |
| 2-1-m1E01-1-0274 | 679.246 | OH | a2 | B13 | b2 | C28 | c3 | D02 | d 1 | E01 |
| 2-1-m1E01-1-0275 | 679.248 | OH | a1 | B04 | b2 | C33 | c3 | D02 | d 1 | E01 |
| 2-1-m1E01-1-0276 | 679.279 | OH | a2 | B18 | b2 | C05 | c1 | D01 | d1 | E01 |
| 2-1-m1E01-1-0277 | 679.281 | OH | a1 | B05 | b2 | C04 | c1 | D01 | d1 | E01 |
| 2-1-m1E01-1-0278 | 679.337 | OH | a1 | B03 | b2 | C07 | c1 | D01 | d1 | E01 |
| 2-1-m1E01-1-0279 | 680.203 | OH | a1 | B09 | b1 | C12 | c1 | D01 | d1 | E01 |
| 2-1-m1E01-1-0280 | 680.205 | OH | a1 | B08 | b2 | C01 | c1 | D01 | d1 | E01 |
| 2-1-m1E01-1-0281 | 680.205 | OH | a2 | B12 | b2 | C03 | c2 | D02 | d1 | E01 |
| 2-1-m1E01-1-0282 | 680.230 | OH | a2 | B16 | b3 | C08 | c1 | D01 | d1 | E01 |
| 2-1-m1E01-1-0283 | 680.230 | OH | a2 | B16 | b1 | C08 | c2 | D02 | d1 | E01 |
| 2-1-m1E01-1-0284 | 680.242 | OH | a2 | B15 | b1 | C10 | c2 | D02 | d 1 | E01 |
| 2-1-m1E01-1-0285 | 680.247 | OH | a2 | B14 | b1 | C30 | c3 | D02 | d1 | E01 |
| 2-1-m1E01-1-0286 | 681.226 | OH | a2 | B16 | b1 | C11 | c2 | D02 | d1 | E01 |
| 2-1-m1E01-1-0287 | 681.260 | OH | a1 | B06 | b2 | C04 | c1 | D01 | d1 | E01 |
| 2-1-m1E01-1-0288 | 681.262 | OH | a1 | B01 | b1 | C30 | c3 | D02 | d1 | E01 |
| 2-1-m1E01-1-0289 | 682.210 | OH | a2 | B13 | b2 | C01 | c2 | D02 | d1 | E01 |
| 2-1-m1E01-1-0290 | 682.221 | OH | a2 | B16 | b1 | C10 | c2 | D02 | d1 | E01 |
| 2-1-m1E01-1-0291 | 682.226 | OH | a2 | B14 | b2 | C20 | c3 | D02 | d1 | E01 |
| 2-1-m1E01-1-0292 | 682.226 | OH | a2 | B11 | b2 | C24 | c3 | D02 | d1 | E01 |
| 2-1-m1E01-1-0293 | 682.240 | OH | a2 | B19 | b2 | C02 | c1 | D01 | d 1 | E01 |
| 2-1-m1E01-1-0294 | 682.257 | OH | a1 | B02 | b1 | C30 | c3 | D02 | d1 | E01 |
| 2-1-m1E01-1-0295 | 682.259 | OH | a2 | B18 | b2 | C04 | c1 | D01 | d1 | E01 |
| 2-1-m1E01-1-0296 | 682.277 | OH | a2 | B15 | b1 | C09 | c1 | D01 | d1 | E01 |
| 2-1-m1E01-1-0297 | 682.297 | OH | a2 | B17 | b2 | C07 | c1 | D01 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0298 | 683.221 | OH | a2 | B12 | b2 | C24 | c3 | D02 | d1 | E01 |
| 2-1-m1E01-1-0299 | 683.221 | OH | a2 | B14 | b2 | C28 | c3 | D02 | d1 | E01 |
| 2-1-m1E01-1-0300 | 683.236 | OH | a2 | B19 | b2 | C03 | c1 | D01 | d1 | E01 |
| 2-1-m1E01-1-0301 | 683.241 | OH | a1 | B03 | b3 | C08 | c1 | D01 | d1 | E01 |
| 2-1-m1E01-1-0302 | 683.241 | OH | a1 | B03 | b1 | C08 | c2 | D02 | d 1 | E01 |
| 2-1-m1E01-1-0303 | 683.241 | OH | a1 | B01 | b2 | C20 | c3 | D02 | d1 | E01 |
| 2-1-m1E01-1-0304 | 683.312 | OH | a1 | B04 | b2 | C07 | c1 | D01 | d1 | E01 |
| 2-1-m1E01-1-0305 | 684.171 | OH | a2 | B11 | b2 | C06 | c2 | D02 | d1 | E01 |
| 2-1-m1E01-1-0306 | 684.208 | OH | a2 | B15 | b3 | C06 | c1 | D01 | d1 | E01 |
| 2-1-m1E01-1-0307 | 684.237 | OH | a1 | B03 | b1 | C11 | c2 | D02 | d1 | E01 |
| 2-1-m1E01-1-0308 | 684.237 | OH | a1 | B02 | b2 | C20 | c3 | D02 | d1 | E01 |
| 2-1-m1E01-1-0309 | 684.237 | OH | a1 | B01 | b2 | C28 | c3 | D02 | d1 | E01 |
| 2-1-m1E01-1-0310 | 684.251 | OH | a1 | B07 | b2 | C05 | c1 | D01 | d1 | E01 |
| 2-1-m1E01-1-0311 | 684.256 | OH | a2 | B16 | b1 | C09 | c1 | D01 | d 1 | E01 |
| 2-1-m1E01-1-0312 | 685.166 | OH | a2 | B12 | b2 | C06 | c2 | D02 | d1 | E01 |
| 2-1-m1E01-1-0313 | 685.203 | OH | a2 | B15 | b1 | C12 | c2 | D02 | d1 | E01 |
| 2-1-m1E01-1-0314 | 685.232 | OH | a1 | B03 | b1 | C10 | c2 | D02 | d1 | E01 |
| 2-1-m1E01-1-0315 | 685.232 | OH | a1 | B02 | b2 | C28 | c3 | D02 | d1 | E01 |
| 2-1-m1E01-1-0316 | 685.301 | OH | a1 | B05 | b1 | C13 | c1 | D01 | d1 | E01 |
| 2-1-m1E01-1-0317 | 686.185 | OH | a2 | B14 | b2 | C01 | c2 | D02 | d1 | E01 |
| 2-1-m1E01-1-0318 | 686.187 | OH | a2 | B16 | b3 | C06 | c1 | D01 | d1 | E01 |
| 2-1-m1E01-1-0319 | 686.201 | OH | a2 | B17 | b3 | C08 | c1 | D01 | d1 | E01 |
| 2-1-m1E01-1-0320 | 686.201 | OH | a2 | B17 | b1 | C08 | c2 | D02 | d 1 | E01 |
| 2-1-m1E01-1-0321 | 687.182 | OH | a2 | B16 | b1 | C12 | c2 | D02 | d 1 | E01 |
| 2-1-m1E01-1-0322 | 687.196 | OH | a2 | B17 | b1 | C11 | c2 | D02 | d 1 | E01 |
| 2-1-m1E01-1-0323 | 687.200 | OH | a1 | B01 | b2 | C01 | c2 | D02 | d1 | E01 |
| 2-1-m1E01-1-0324 | 687.216 | OH | a1 | B04 | b3 | C08 | c1 | D01 | d1 | E01 |
| 2-1-m1E01-1-0325 | 687.216 | OH | a1 | B04 | b1 | C08 | c2 | D02 | d1 | E01 |
| 2-1-m1E01-1-0326 | 687.230 | OH | a1 | B07 | b2 | C04 | c1 | D01 | d1 | E01 |
| 2-1-m1E01-1-0327 | 687.236 | OH | a1 | B05 | b3 | C15 | c1 | D01 | d1 | E01 |
| 2-1-m1E01-1-0328 | 687.267 | OH | a1 | B03 | b1 | C09 | c1 | D01 | d1 | E01 |
| 2-1-m1E01-1-0329 | 687.281 | OH | a1 | B06 | b1 | C13 | c1 | D01 | d1 | E01 |
| 2-1-m1E01-1-0330 | 688.192 | OH | a2 | B17 | b1 | C10 | c2 | D02 | d1 | E01 |
| 2-1-m1E01-1-0331 | 688.195 | OH | a1 | B02 | b2 | C01 | c2 | D02 | d 1 | E01 |
| 2-1-m1E01-1-0332 | 688.197 | OH | a2 | B19 | b2 | C06 | c1 | D01 | d1 | E01 |
| 2-1-m1E01-1-0333 | 688.212 | OH | a1 | B04 | b1 | C11 | c2 | D02 | d1 | E01 |
| 2-1-m1E01-1-0334 | 688.214 | OH | a2 | B20 | b2 | C01 | c1 | D01 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0335 | 688.280 | OH | a2 | B18 | b1 | C13 | c1 | D01 | d1 | E01 |
| 2-1-m1E01-1-0336 | 689.198 | OH | a1 | B03 | b3 | C06 | c1 | D01 | d1 | E01 |
| 2-1-m1E01-1-0337 | 689.207 | OH | a1 | B04 | b1 | C10 | c2 | D02 | d1 | E01 |
| 2-1-m1E01-1-0338 | 689.216 | OH | a1 | B06 | b3 | C15 | c1 | D01 | d1 | E01 |
| 2-1-m1E01-1-0339 | 689.246 | OH | a1 | B10 | b1 | C08 | c1 | D01 | d1 | E01 |
| 2-1-m1E01-1-0340 | 689.267 | OH | a1 | B05 | b1 | C14 | c1 | D01 | d 1 | E01 |
| 2-1-m1E01-1-0341 | 689.288 | OH | a1 | B05 | b2 | C33 | c3 | D02 | d1 | E01 |
| 2-1-m1E01-1-0342 | 690.193 | OH | a1 | B03 | b1 | C12 | c2 | D02 | d1 | E01 |
| 2-1-m1E01-1-0343 | 690.215 | OH | a2 | B18 | b3 | C15 | c1 | D01 | d1 | E01 |
| 2-1-m1E01-1-0344 | 690.226 | OH | a1 | B08 | b2 | C02 | c1 | D01 | d1 | E01 |
| 2-1-m1E01-1-0345 | 690.227 | OH | a2 | B17 | b1 | C09 | c1 | D01 | d1 | E01 |
| 2-1-m1E01-1-0346 | 690.241 | OH | a1 | B10 | b1 | C11 | c1 | D01 | d1 | E01 |
| 2-1-m1E01-1-0347 | 690.288 | OH | a2 | B15 | b1 | C30 | c3 | D02 | d1 | E01 |
| 2-1-m1E01-1-0348 | 690.288 | OH | a2 | B11 | b1 | C29 | c3 | D02 | d1 | E01 |
| 2-1-m1E01-1-0349 | 690.288 | OH | a2 | B13 | b1 | C32 | c3 | D02 | d 1 | E01 |
| 2-1-m1E01-1-0350 | 691.221 | OH | a1 | B08 | b2 | C03 | c1 | D01 | d1 | E01 |
| 2-1-m1E01-1-0351 | 691.225 | OH | a1 | B09 | b2 | C01 | c1 | D01 | d1 | E01 |
| 2-1-m1E01-1-0352 | 691.237 | OH | a1 | B10 | b1 | C10 | c1 | D01 | d1 | E01 |
| 2-1-m1E01-1-0353 | 691.242 | OH | a1 | B04 | b1 | C09 | c1 | D01 | d1 | E01 |
| 2-1-m1E01-1-0354 | 691.246 | OH | a1 | B06 | b1 | C14 | c1 | D01 | d1 | E01 |
| 2-1-m1E01-1-0355 | 691.268 | OH | a1 | B06 | b2 | C33 | c3 | D02 | d1 | E01 |
| 2-1-m1E01-1-0356 | 691.283 | OH | a2 | B12 | b1 | C29 | c3 | D02 | d1 | E01 |
| 2-1-m1E01-1-0357 | 692.157 | OH | a2 | B17 | b3 | C06 | c1 | D01 | d1 | E01 |
| 2-1-m1E01-1-0358 | 692.230 | OH | a2 | B13 | b2 | C02 | c2 | D02 | d 1 | E01 |
| 2-1-m1E01-1-0359 | 692.246 | OH | a2 | B18 | b1 | C14 | c1 | D01 | d1 | E01 |
| 2-1-m1E01-1-0360 | 692.267 | OH | a2 | B15 | b2 | C20 | c3 | D02 | d1 | E01 |
| 2-1-m1E01-1-0361 | 692.267 | OH | a2 | B13 | b2 | C22 | c3 | D02 | d1 | E01 |
| 2-1-m1E01-1-0362 | 692.267 | OH | a2 | B16 | b1 | C30 | c3 | D02 | d1 | E01 |
| 2-1-m1E01-1-0363 | 692.267 | OH | a2 | B18 | b2 | C33 | c3 | D02 | d1 | E01 |
| 2-1-m1E01-1-0364 | 693.153 | OH | a2 | B17 | b1 | C12 | c2 | D02 | d1 | E01 |
| 2-1-m1E01-1-0365 | 693.173 | OH | a1 | B04 | b3 | C06 | c1 | D01 | d1 | E01 |
| 2-1-m1E01-1-0366 | 693.226 | OH | a2 | B13 | b2 | C03 | c2 | D02 | d1 | E01 |
| 2-1-m1E01-1-0367 | 693.226 | OH | a2 | B11 | b2 | C05 | c2 | D02 | d 1 | E01 |
| 2-1-m1E01-1-0368 | 693.251 | OH | a1 | B07 | b1 | C13 | c1 | D01 | d 1 | E01 |
| 2-1-m1E01-1-0369 | 693.262 | OH | a2 | B15 | b2 | C28 | c3 | D02 | d 1 | E01 |
| 2-1-m1E01-1-0370 | 693.353 | OH | a1 | B05 | b2 | C07 | c1 | D01 | d1 | E01 |
| 2-1-m1E01-1-0371 | 694.168 | OH | a1 | B04 | b1 | C12 | c2 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0372 | 694.221 | OH | a2 | B12 | b2 | C05 | c2 | D02 | d1 | E01 |
| 2-1-m1E01-1-0373 | 694.246 | OH | a2 | B16 | b2 | C20 | c3 | D02 | d1 | E01 |
| 2-1-m1E01-1-0374 | 694.246 | OH | a2 | B11 | b2 | C26 | c3 | D02 | d1 | E01 |
| 2-1-m1E01-1-0375 | 694.263 | OH | a2 | B14 | b1 | C32 | c3 | D02 | d1 | E01 |
| 2-1-m1E01-1-0376 | 695.186 | OH | a1 | B07 | b3 | C15 | c1 | D01 | d1 | E01 |
| 2-1-m1E01-1-0377 | 695.241 | OH | a2 | B12 | b2 | C26 | c3 | D02 | d1 | E01 |
| 2-1-m1E01-1-0378 | 695.241 | OH | a2 | B16 | b2 | C28 | c3 | D02 | d 1 | E01 |
| 2-1-m1E01-1-0379 | 695.278 | OH | a1 | B03 | b1 | C30 | c3 | D02 | d1 | E01 |
| 2-1-m1E01-1-0380 | 695.278 | OH | a1 | B01 | b1 | C32 | c3 | D02 | d1 | E01 |
| 2-1-m1E01-1-0381 | 695.332 | OH | a1 | B06 | b2 | C07 | c1 | D01 | d1 | E01 |
| 2-1-m1E01-1-0382 | 696.182 | OH | a1 | B08 | b2 | C06 | c1 | D01 | d1 | E01 |
| 2-1-m1E01-1-0383 | 696.198 | OH | a1 | B10 | b1 | C12 | c1 | D01 | d1 | E01 |
| 2-1-m1E01-1-0384 | 696.205 | OH | a2 | B14 | b2 | C02 | c2 | D02 | d1 | E01 |
| 2-1-m1E01-1-0385 | 696.205 | OH | a2 | B11 | b2 | C04 | c2 | D02 | d1 | E01 |
| 2-1-m1E01-1-0386 | 696.225 | OH | a2 | B15 | b2 | C01 | c2 | D02 | d1 | E01 |
| 2-1-m1E01-1-0387 | 696.242 | OH | a2 | B14 | b2 | C22 | c3 | D02 | d 1 | E01 |
| 2-1-m1E01-1-0388 | 696.242 | OH | a2 | B13 | b2 | C24 | c3 | D02 | d1 | E01 |
| 2-1-m1E01-1-0389 | 696.273 | OH | a1 | B02 | b1 | C32 | c3 | D02 | d1 | E01 |
| 2-1-m1E01-1-0390 | 696.331 | OH | a2 | B18 | b2 | C07 | c1 | D01 | d1 | E01 |
| 2-1-m1E01-1-0391 | 697.201 | OH | a2 | B12 | b2 | C04 | c2 | D02 | d1 | E01 |
| 2-1-m1E01-1-0392 | 697.201 | OH | a2 | B14 | b2 | C03 | c2 | D02 | d1 | E01 |
| 2-1-m1E01-1-0393 | 697.217 | OH | a1 | B07 | b1 | C14 | c1 | D01 | d1 | E01 |
| 2-1-m1E01-1-0394 | 697.221 | OH | a1 | B01 | b2 | C02 | c2 | D02 | d1 | E01 |
| 2-1-m1E01-1-0395 | 697.238 | OH | a1 | B07 | b2 | C33 | c3 | D02 | d1 | E01 |
| 2-1-m1E01-1-0396 | 697.251 | OH | a2 | B19 | b2 | C05 | c1 | D01 | d 1 | E01 |
| 2-1-m1E01-1-0397 | 697.257 | OH | a1 | B05 | b3 | C08 | c1 | D01 | d1 | E01 |
| 2-1-m1E01-1-0398 | 697.257 | OH | a1 | B05 | b1 | C08 | c2 | D02 | d1 | E01 |
| 2-1-m1E01-1-0399 | 697.257 | OH | a1 | B03 | b2 | C20 | c3 | D02 | d1 | E01 |
| 2-1-m1E01-1-0400 | 697.257 | OH | a1 | B01 | b2 | C22 | c3 | D02 | d1 | E01 |
| 2-1-m1E01-1-0401 | 698.187 | OH | a2 | B13 | b2 | C06 | c2 | D02 | d1 | E01 |
| 2-1-m1E01-1-0402 | 698.205 | OH | a2 | B16 | b2 | C01 | c2 | D02 | d1 | E01 |
| 2-1-m1E01-1-0403 | 698.216 | OH | a1 | B02 | b2 | C02 | c2 | D02 | d1 | E01 |
| 2-1-m1E01-1-0404 | 698.216 | OH | a1 | B01 | b2 | C03 | c2 | D02 | d1 | E01 |
| 2-1-m1E01-1-0405 | 698.235 | OH | a2 | B20 | b2 | C02 | c1 | D01 | d 1 | E01 |
| 2-1-m1E01-1-0406 | 698.237 | OH | a2 | B17 | b1 | C30 | c3 | D02 | d1 | E01 |
| 2-1-m1E01-1-0407 | 698.252 | OH | a1 | B05 | b1 | C11 | c2 | D02 | d1 | E01 |
| 2-1-m1E01-1-0408 | 698.252 | OH | a1 | B02 | b2 | C22 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0409 | 698.252 | OH | a1 | B03 | b2 | C28 | c3 | D02 | d1 | E01 |
| 2-1-m1E01-1-0410 | 699.211 | OH | a1 | B02 | b2 | C03 | c2 | D02 | d1 | E01 |
| 2-1-m1E01-1-0411 | 699.230 | OH | a2 | B20 | b2 | C03 | c1 | D01 | d1 | E01 |
| 2-1-m1E01-1-0412 | 699.236 | OH | a1 | B06 | b3 | C08 | c1 | D01 | d1 | E01 |
| 2-1-m1E01-1-0413 | 699.236 | OH | a1 | B06 | b1 | C08 | c2 | D02 | d1 | E01 |
| 2-1-m1E01-1-0414 | 699.248 | OH | a1 | B05 | b1 | C10 | c2 | D02 | d 1 | E01 |
| 2-1-m1E01-1-0415 | 699.253 | OH | a1 | B04 | b1 | C30 | c3 | D02 | d1 | E01 |
| 2-1-m1E01-1-0416 | 700.217 | OH | a2 | B17 | b2 | C20 | c3 | D02 | d1 | E01 |
| 2-1-mE01-1-0417 | 700.217 | OH | a2 | B14 | b2 | C24 | c3 | D02 | d1 | E01 |
| 2-1-m1E01-1-0418 | 700.231 | OH | a2 | B19 | b2 | C04 | c1 | D01 | d1 | E01 |
| 2-1-m1E01-1-0419 | 700.232 | OH | a1 | B06 | b1 | C11 | c2 | D02 | d1 | E01 |
| 2-1-m1E01-1-0420 | 700.236 | OH | a2 | B18 | b3 | C08 | c1 | D01 | d1 | E01 |
| 2-1-m1E01-1-0421 | 700.236 | OH | a2 | B18 | b1 | C08 | c2 | D02 | d1 | E01 |
| 2-1-m1E01-1-0422 | 701.212 | OH | a2 | B17 | b2 | C28 | c3 | D02 | d1 | E01 |
| 2-1-m1E01-1-0423 | 701.216 | OH | a1 | B03 | b2 | C01 | c2 | D02 | d1 | E01 |
| 2-1-m1E01-1-0424 | 701.227 | OH | a1 | B06 | b1 | C10 | c2 | D02 | d1 | E01 |
| 2-1-m1E01-1-0425 | 701.231 | OH | a2 | B18 | b1 | C11 | c2 | D02 | d1 | E01 |
| 2-1-m1E01-1-0426 | 701.232 | OH | a1 | B04 | b2 | C20 | c3 | D02 | d1 | E01 |
| 2-1-m1E01-1-0427 | 701.232 | OH | a1 | B01 | b2 | C24 | c3 | D02 | d1 | E01 |
| 2-1-m1E01-1-0428 | 701.246 | OH | a1 | B09 | b2 | C02 | c1 | D01 | d1 | E01 |
| 2-1-m1E01-1-0429 | 701.283 | OH | a1 | B05 | b1 | C09 | c1 | D01 | d1 | E01 |
| 2-1-m1E01-1-0430 | 701.302 | OH | a1 | B07 | b2 | C07 | c1 | D01 | d1 | E01 |
| 2-1-m1E01-1-0431 | 702.162 | OH | a2 | B14 | b2 | C06 | c2 | D02 | d1 | E01 |
| 2-1-m1E01-1-0432 | 702.226 | OH | a2 | B18 | b1 | C10 | c2 | D02 | d1 | E01 |
| 2-1-m1E01-1-0433 | 702.226 | OH | a2 | B11 | b1 | C13 | c2 | D02 | d1 | E01 |
| 2-1-m1E01-1-0434 | 702.227 | OH | a1 | B02 | b2 | C24 | c3 | D02 | d1 | E01 |
| 2-1-m1E01-1-0435 | 702.227 | OH | a1 | B04 | b2 | C28 | c3 | D02 | d1 | E01 |
| 2-1-m1E01-1-0436 | 702.241 | OH | a1 | B09 | b2 | C03 | c1 | D01 | d1 | E01 |
| 2-1-m1E01-1-0437 | 703.177 | OH | a1 | B01 | b2 | C06 | c2 | D02 | d1 | E01 |
| 2-1-m1E01-1-0438 | 703.213 | OH | a1 | B05 | b3 | C06 | c1 | D01 | d1 | E01 |
| 2-1-m1E01-1-0439 | 703.221 | OH | a2 | B12 | b1 | C13 | c2 | D02 | d1 | E01 |
| 2-1-m1E01-1-0440 | 703.262 | OH | a1 | B06 | b1 | C09 | c1 | D01 | d1 | E01 |
| 2-1-m1E01-1-0441 | 704.161 | OH | a2 | B11 | b3 | C15 | c2 | D02 | d1 | E01 |
| 2-1-m1E01-1-0442 | 704.172 | OH | a1 | B02 | b2 | C06 | c2 | D02 | d1 | E01 |
| 2-1-m1E01-1-0443 | 704.175 | OH | a2 | B17 | b2 | C01 | c2 | D02 | d1 | E01 |
| 2-1-m1E01-1-0444 | 704.192 | OH | a2 | B20 | b2 | C06 | c1 | D01 | d1 | E01 |
| 2-1-m1E01-1-0445 | 704.209 | OH | a1 | B05 | b1 | C12 | c2 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0446 | 704.230 | OH | a2 | B11 | b3 | C16 | c1 | D01 | d1 | E01 |
| 2-1-m1E01-1-0447 | 704.261 | OH | a2 | B18 | b1 | C09 | c1 | D01 | d1 | E01 |
| 2-1-m1E01-1-0448 | 704.303 | OH | a2 | B13 | b1 | C29 | c3 | D02 | d1 | E01 |
| 2-1-m1E01-1-0449 | 704.303 | OH | a2 | B15 | b1 | C32 | c3 | D02 | d1 | E01 |
| 2-1-m1E01-1-0450 | 705.156 | OH | a2 | B12 | b3 | C15 | c2 | D02 | d1 | E01 |
| 2-1-m1E01-1-0451 | 705.190 | OH | a1 | B04 | b2 | C01 | c2 | D02 | d1 | E01 |
| 2-1-m1E01-1-0452 | 705.193 | OH | a1 | B06 | b3 | C06 | c1 | D01 | d1 | E01 |
| 2-1-m1E01-1-0453 | 705.207 | OH | a1 | B07 | b3 | C08 | c1 | D01 | d1 | E01 |
| 2-1-m1E01-1-0454 | 705.207 | OH | a1 | B07 | b1 | C08 | c2 | D02 | d1 | E01 |
| 2-1-m1E01-1-0455 | 705.226 | OH | a2 | B12 | b3 | C16 | c1 | D01 | d1 | E01 |
| 2-1-m1E01-1-0456 | 705.237 | OH | a1 | B08 | b2 | C05 | c1 | D01 | d1 | E01 |
| 2-1-m1E01-1-0457 | 706.188 | OH | a1 | B06 | b1 | C12 | c2 | D02 | d1 | E01 |
| 2-1-m1E01-1-0458 | 706.192 | OH | a2 | B18 | b3 | C06 | c1 | D01 | d1 | E01 |
| 2-1-m1E01-1-0459 | 706.192 | OH | a2 | B11 | b3 | C17 | c1 | D01 | d1 | E01 |
| 2-1-m1E01-1-0460 | 706.192 | OH | a2 | B11 | b1 | C14 | c2 | D02 | d1 | E01 |
| 2-1-m1E01-1-0461 | 706.202 | OH | a1 | B07 | b1 | C11 | c2 | D02 | d1 | E01 |
| 2-1-m1E01-1-0462 | 706.246 | OH | a2 | B15 | b2 | C02 | c2 | D02 | d1 | E01 |
| 2-1-m1E01-1-0463 | 706.252 | OH | a2 | B19 | b1 | C13 | c1 | D01 | d1 | E01 |
| 2-1-m1E01-1-0464 | 706.283 | OH | a2 | B15 | b2 | C22 | c3 | D02 | d1 | E01 |
| 2-1-m1E01-1-0465 | 706.283 | OH | a2 | B16 | b1 | C32 | c3 | D02 | d1 | E01 |
| 2-1-m1E01-1-0466 | 707.187 | OH | a2 | B12 | b3 | C17 | c1 | D01 | d1 | E01 |
| 2-1-m1E01-1-0467 | 707.187 | OH | a2 | B18 | b1 | C12 | c2 | D02 | d1 | E01 |
| 2-1-m1E01-1-0468 | 707.187 | OH | a2 | B12 | b1 | C14 | c2 | D02 | d1 | E01 |
| 2-1-m1E01-1-0469 | 707.197 | OH | a1 | B07 | b1 | C10 | c2 | D02 | d1 | E01 |
| 2-1-m1E01-1-0470 | 707.203 | OH | a1 | B09 | b2 | C06 | c1 | D01 | d1 | E01 |
| 2-1-m1E01-1-0471 | 707.220 | OH | a1 | B10 | b2 | C01 | c1 | D01 | d1 | E01 |
| 2-1-m1E01-1-0472 | 707.241 | OH | a2 | B15 | b2 | C03 | c2 | D02 | d1 | E01 |
| 2-1-m1E01-1-0473 | 707.241 | OH | a2 | B13 | b2 | C05 | c2 | D02 | d1 | E01 |
| 2-1-m1E01-1-0474 | 708.187 | OH | a2 | B19 | b3 | C15 | c1 | D01 | d1 | E01 |
| 2-1-m1E01-1-0475 | 708.217 | OH | a1 | B08 | b2 | C04 | c1 | D01 | d1 | E01 |
| 2-1-m1E01-1-0476 | 708.225 | OH | a2 | B16 | b2 | C02 | c2 | D02 | d1 | E01 |
| 2-1-m1E01-1-0477 | 708.262 | OH | a2 | B16 | b2 | C22 | c3 | D02 | d1 | E01 |
| 2-1-m1E01-1-0478 | 708.262 | OH | a2 | B13 | b2 | C26 | c3 | D02 | d1 | E01 |
| 2-1-m1E01-1-0479 | 708.278 | OH | a2 | B14 | b1 | C29 | c3 | D02 | d1 | E01 |
| 2-1-m1E01-1-0480 | 709.221 | OH | a2 | B16 | b2 | C03 | c2 | D02 | d1 | E01 |
| 2-1-m1E01-1-0481 | 709.232 | OH | a1 | B07 | b1 | C09 | c1 | D01 | d1 | E01 |
| 2-1-m1E01-1-0482 | 709.293 | OH | a1 | B05 | b1 | C30 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0483 | 709.293 | OH | a1 | B01 | b1 | C29 | c3 | D02 | d1 | E01 |
| 2-1-m1E01-1-0484 | 709.293 | OH | a1 | B03 | b1 | C32 | c3 | D02 | d1 | E01 |
| 2-1-m1E01-1-0485 | 710.217 | OH | a2 | B19 | b1 | C14 | c1 | D01 | d1 | E01 |
| 2-1-m1E01-1-0486 | 710.221 | OH | a2 | B13 | b2 | C04 | c2 | D02 | d1 | E01 |
| 2-1-m1E01-1-0487 | 710.239 | OH | a2 | B19 | b2 | C33 | c3 | D02 | d1 | E01 |
| 2-1-m1E01-1-0488 | 710.257 | OH | a2 | B15 | b2 | C24 | c3 | D02 | d1 | E01 |
| 2-1-m1E01-1-0489 | 710.277 | OH | a2 | B11 | b2 | C07 | c2 | D02 | d1 | E01 |
| 2-1-m1E01-1-0490 | 710.289 | OH | a1 | B02 | b1 | C29 | c3 | D02 | d1 | E01 |
| 2-1-m1E01-1-0491 | 711.163 | OH | a1 | B07 | b3 | C06 | c1 | D01 | d1 | E01 |
| 2-1-m1E01-1-0492 | 711.216 | OH | a2 | B14 | b2 | C05 | c2 | D02 | d1 | E01 |
| 2-1-m1E01-1-0493 | 711.236 | OH | a1 | B03 | b2 | C02 | c2 | D02 | d1 | E01 |
| 2-1-m1E01-1-0494 | 711.273 | OH | a2 | B12 | b2 | C07 | c2 | D02 | d1 | E01 |
| 2-1-m1E01-1-0495 | 711.273 | OH | a1 | B05 | b2 | C20 | c3 | D02 | d1 | E01 |
| 2-1-m1E01-1-0496 | 711.273 | OH | a1 | B03 | b2 | C22 | c3 | D02 | d1 | E01 |
| 2-1-m1E01-1-0497 | 711.273 | OH | a1 | B06 | b1 | C30 | c3 | D02 | d1 | E01 |
| 2-1-m1E01-1-0498 | 712.159 | OH | a1 | B07 | b1 | C12 | c2 | D02 | d1 | E01 |
| 2-1-m1E01-1-0499 | 712.203 | OH | a2 | B15 | b2 | C06 | c2 | D02 | d1 | E01 |
| 2-1-m1E01-1-0500 | 712.232 | OH | a1 | B03 | b2 | C03 | c2 | D02 | d1 | E01 |
| 2-1-m1E01-1-0501 | 712.232 | OH | a1 | B01 | b2 | C05 | c2 | D02 | d1 | E01 |
| 2-1-m1E01-1-0502 | 712.237 | OH | a2 | B16 | b2 | C24 | c3 | D02 | d1 | E01 |
| 2-1-m1E01-1-0503 | 712.237 | OH | a2 | B14 | b2 | C26 | c3 | D02 | d1 | E01 |
| 2-1-m1E01-1-0504 | 712.253 | OH | a2 | B17 | b1 | C32 | c3 | D02 | d1 | E01 |
| 2-1-m1E01-1-0505 | 712.268 | OH | a1 | B05 | b2 | C28 | c3 | D02 | d1 | E01 |
| 2-1-m1E01-1-0506 | 712.272 | OH | a2 | B18 | b1 | C30 | c3 | D02 | d1 | E01 |
| 2-1-m1E01-1-0507 | 713.227 | OH | a1 | B02 | b2 | C05 | c2 | D02 | d1 | E01 |
| 2-1-m1E01-1-0508 | 713.246 | OH | a2 | B20 | b2 | C05 | c1 | D01 | d1 | E01 |
| 2-1-m1E01-1-0509 | 713.252 | OH | a1 | B06 | b2 | C20 | c3 | D02 | d1 | E01 |
| 2-1-m1E01-1-0510 | 713.252 | OH | a1 | B01 | b2 | C26 | c3 | D02 | d1 | E01 |
| 2-1-m1E01-1-0511 | 713.268 | OH | a1 | B04 | b1 | C32 | c3 | D02 | d1 | E01 |
| 2-1-m1E01-1-0512 | 714.182 | OH | a2 | B16 | b2 | C06 | c2 | D02 | d1 | E01 |
| 2-1-m1E01-1-0513 | 714.182 | OH | a2 | B11 | b3 | C08 | c2 | D02 | d1 | E01 |
| 2-1-m1E01-1-0514 | 714.196 | OH | a2 | B17 | b2 | C02 | c2 | D02 | d1 | E01 |
| 2-1-m1E01-1-0515 | 714.196 | OH | a2 | B14 | b2 | C04 | c2 | D02 | d1 | E01 |
| 2-1-m1E01-1-0516 | 714.232 | OH | a2 | B17 | b2 | C22 | c3 | D02 | d1 | E01 |
| 2-1-m1E01-1-0517 | 714.237 | OH | a1 | B08 | b1 | C13 | c1 | D01 | d1 | E01 |
| 2-1-m1E01-1-0518 | 714.247 | OH | a1 | B02 | b2 | C26 | c3 | D02 | d1 | E01 |
| 2-1-m1E01-1-0519 | 714.247 | OH | a1 | B06 | b2 | C28 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0520 | 714.251 | OH | a2 | B18 | b2 | C20 | c3 | D02 | d1 | E01 |
| 2-1-m1E01-1-0521 | 714.303 | OH | a2 | B19 | b2 | C07 | c1 | D01 | d1 | E01 |
| 2-1-m1E01-1-0522 | 715.177 | OH | a2 | B12 | b3 | C08 | c2 | D02 | d1 | E01 |
| 2-1-m1E01-1-0523 | 715.191 | OH | a2 | B17 | b2 | C03 | c2 | D02 | d1 | E01 |
| 2-1-m1E01-1-0524 | 715.211 | OH | a1 | B04 | b2 | C02 | c2 | D02 | d1 | E01 |
| 2-1-m1E01-1-0525 | 715.211 | OH | a1 | B01 | b2 | C04 | c2 | D02 | d1 | E01 |
| 2-1-m1E01-1-0526 | 715.231 | OH | a1 | B05 | b2 | C01 | c2 | D02 | d1 | E01 |
| 2-1-m1E01-1-0527 | 715.246 | OH | a2 | B18 | b2 | C28 | c3 | D02 | d1 | E01 |
| 2-1-m1E01-1-0528 | 715.248 | OH | a1 | B04 | b2 | C22 | c3 | D02 | d1 | E01 |
| 2-1-m1E01-1-0529 | 715.248 | OH | a1 | B03 | b2 | C24 | c3 | D02 | d1 | E01 |
| 2-1-m1E01-1-0530 | 716.172 | OH | a1 | B08 | b3 | C15 | c1 | D01 | d1 | E01 |
| 2-1-m1E01-1-0531 | 716.206 | OH | a1 | B02 | b2 | C04 | c2 | D02 | d1 | E01 |
| 2-1-m1E01-1-0532 | 716.206 | OH | a1 | B04 | b2 | C03 | c2 | D02 | d1 | E01 |
| 2-1-m1E01-1-0533 | 716.226 | OH | a2 | B20 | b2 | C04 | c1 | D01 | d1 | E01 |
| 2-1-m1E01-1-0534 | 716.242 | OH | a2 | B13 | b1 | C13 | c2 | D02 | d1 | E01 |
| 2-1-m1E01-1-0535 | 716.257 | OH | a1 | B09 | b2 | C05 | c1 | D01 | d1 | E01 |
| 2-1-m1E01-1-0536 | 717.193 | OH | a1 | B03 | b2 | C06 | c2 | D02 | d1 | E01 |
| 2-1-m1E01-1-0537 | 717.210 | OH | a1 | B06 | b2 | C01 | c2 | D02 | d1 | E01 |
| 2-1-m1E01-1-0538 | 717.241 | OH | a1 | B10 | b2 | C02 | c1 | D01 | d1 | E01 |
| 2-1-m1E01-1-0539 | 717.243 | OH | a1 | B07 | b1 | C30 | c3 | D02 | d1 | E01 |
| 2-1-m1E01-1-0540 | 718.177 | OH | a2 | B13 | b3 | C15 | c2 | D02 | d1 | E01 |
| 2-1-m1E01-1-0541 | 718.203 | OH | a1 | B08 | b1 | C14 | c1 | D01 | d1 | E01 |
| 2-1-m1E01-1-0542 | 718.207 | OH | a2 | B19 | b3 | C08 | c1 | D01 | d1 | E01 |
| 2-1-m1E01-1-0543 | 718.207 | OH | a2 | B19 | b1 | C08 | c2 | D02 | d1 | E01 |
| 2-1-m1E01-1-0544 | 718.207 | OH | a2 | B11 | b1 | C09 | c2 | D02 | d1 | E01 |
| 2-1-m1E01-1-0545 | 718.207 | OH | a2 | B17 | b2 | C24 | c3 | D02 | d1 | E01 |
| 2-1-m1E01-1-0546 | 718.210 | OH | a2 | B18 | b2 | C01 | c2 | D02 | d1 | E01 |
| 2-1-m1E01-1-0547 | 718.224 | OH | a1 | B08 | b2 | C33 | c3 | D02 | d1 | E01 |
| 2-1-m1E01-1-0548 | 718.236 | OH | a1 | B10 | b2 | C03 | c1 | D01 | d1 | E01 |
| 2-1-m1E01-1-0549 | 718.246 | OH | a2 | B13 | b3 | C16 | c1 | D01 | d1 | E01 |
| 2-1-m1E01-1-0550 | 718.283 | OH | a2 | B11 | b1 | C31 | c3 | D02 | d1 | E01 |
| 2-1-m1E01-1-0551 | 718.319 | OH | a2 | B15 | b1 | C29 | c3 | D02 | d1 | E01 |
| 2-1-m1E01-1-0552 | 719.203 | OH | a2 | B12 | b1 | C09 | c2 | D02 | d1 | E01 |
| 2-1-m1E01-1-0553 | 719.203 | OH | a2 | B19 | b1 | C11 | c2 | D02 | d1 | E01 |
| 2-1-m1E01-1-0554 | 719.223 | OH | a1 | B07 | b2 | C20 | c3 | D02 | d1 | E01 |
| 2-1-m1E01-1-0555 | 719.223 | OH | a1 | B04 | b2 | C24 | c3 | D02 | d1 | E01 |
| 2-1-m1E01-1-0556 | 719.237 | OH | a1 | B09 | b2 | C04 | c1 | D01 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0557 | 719.278 | OH | a2 | B12 | b1 | C31 | c3 | D02 | d1 | E01 |
| 2-1-m1E01-1-0558 | 720.138 | OH | a2 | B11 | b3 | C06 | c2 | D02 | d1 | E01 |
| 2-1-m1E01-1-0559 | 720.152 | OH | a2 | B17 | b2 | C06 | c2 | D02 | d1 | E01 |
| 2-1-m1E01-1-0560 | 720.198 | OH | a2 | B19 | b1 | C10 | c2 | D02 | d1 | E01 |
| 2-1-m1E01-1-0561 | 720.208 | OH | a2 | B13 | b3 | C17 | c1 | D01 | d1 | E01 |
| 2-1-m1E01-1-0562 | 720.208 | OH | a2 | B13 | b1 | C14 | c2 | D02 | d1 | E01 |
| 2-1-m1E01-1-0563 | 720.217 | OH | a2 | B14 | b1 | C13 | c2 | D02 | d1 | E01 |
| 2-1-m1E01-1-0564 | 720.218 | OH | a1 | B07 | b2 | C28 | c3 | D02 | d1 | E01 |
| 2-1-m1E01-1-0565 | 720.298 | OH | a2 | B16 | b1 | C29 | c3 | D02 | d1 | E01 |
| 2-1-m1E01-1-0566 | 721.133 | OH | a2 | B12 | b3 | C06 | c2 | D02 | d1 | E01 |
| 2-1-m1E01-1-0567 | 721.168 | OH | a1 | B04 | b2 | C06 | c2 | D02 | d1 | E01 |
| 2-1-m1E01-1-0568 | 721.203 | OH | a2 | B11 | b3 | C18 | c1 | D01 | d1 | E01 |
| 2-1-m1E01-1-0569 | 721.232 | OH | a1 | B01 | b1 | C13 | c2 | D02 | d1 | E01 |
| 2-1-m1E01-1-0570 | 721.257 | OH | a2 | B15 | b2 | C05 | c2 | D02 | d1 | E01 |
| 2-1-m1E01-1-0571 | 722.152 | OH | a2 | B14 | b3 | C15 | c2 | D02 | d1 | E01 |
| 2-1-m1E01-1-0572 | 722.198 | OH | a2 | B12 | b3 | C18 | c1 | D01 | d1 | E01 |
| 2-1-m1E01-1-0573 | 722.221 | OH | a2 | B14 | b3 | C16 | c1 | D01 | d1 | E01 |
| 2-1-m1E01-1-0574 | 722.227 | OH | a1 | B02 | b1 | C13 | c2 | D02 | d1 | E01 |
| 2-1-m1E01-1-0575 | 722.233 | OH | a2 | B19 | b1 | C09 | c1 | D01 | d1 | E01 |
| 2-1-m1E01-1-0576 | 722.246 | OH | a2 | B20 | b1 | C13 | c1 | D01 | d1 | E01 |
| 2-1-m1E01-1-0577 | 722.277 | OH | a2 | B15 | b2 | C26 | c3 | D02 | d1 | E01 |
| 2-1-m1E01-1-0578 | 722.289 | OH | a1 | B08 | b2 | C07 | c1 | D01 | d1 | E01 |
| 2-1-m1E01-1-0579 | 723.167 | OH | a1 | B01 | b3 | C15 | c2 | D02 | d1 | E01 |
| 2-1-m1E01-1-0580 | 723.181 | OH | a1 | B07 | b2 | C01 | c2 | D02 | d1 | E01 |
| 2-1-m1E01-1-0581 | 723.197 | OH | a1 | B10 | b2 | C06 | c1 | D01 | d1 | E01 |
| 2-1-m1E01-1-0582 | 723.236 | OH | a1 | B01 | b3 | C16 | c1 | D01 | d1 | E01 |
| 2-1-m1E01-1-0583 | 723.236 | OH | a2 | B16 | b2 | C05 | c2 | D02 | d1 | E01 |
| 2-1-m1E01-1-0584 | 723.309 | OH | a1 | B03 | b1 | C29 | c3 | D02 | d1 | E01 |
| 2-1-m1E01-1-0585 | 723.309 | OH | a1 | B05 | b1 | C32 | c3 | D02 | d1 | E01 |
| 2-1-m1E01-1-0586 | 724.162 | OH | a1 | B02 | b3 | C15 | c2 | D02 | d1 | E01 |
| 2-1-m1E01-1-0587 | 724.164 | OH | a2 | B19 | b3 | C06 | c1 | D01 | d1 | E01 |
| 2-1-m1E01-1-0588 | 724.181 | OH | a2 | B20 | b3 | C15 | c1 | D01 | d1 | E01 |
| 2-1-m1E01-1-0589 | 724.183 | OH | a2 | B14 | b3 | C17 | c1 | D01 | d1 | E01 |
| 2-1-m1E01-1-0590 | 724.183 | OH | a2 | B14 | b1 | C14 | c2 | D02 | d1 | E01 |
| 2-1-m1E01-1-0591 | 724.232 | OH | a1 | B02 | b3 | C16 | c1 | D01 | d1 | E01 |
| 2-1-m1E01-1-0592 | 724.237 | OH | a2 | B15 | b2 | C04 | c2 | D02 | d1 | E01 |
| 2-1-m1E01-1-0593 | 724.257 | OH | a2 | B16 | b2 | C26 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0594 | 724.293 | OH | a2 | B13 | b2 | C07 | c2 | D02 | d1 | E01 |
| 2-1-m1E01-1-0595 | 725.159 | OH | a2 | B19 | b1 | C12 | c2 | D02 | d1 | E01 |
| 2-1-m1E01-1-0596 | 725.198 | OH | a1 | B01 | b3 | C17 | c1 | D01 | d1 | E01 |
| 2-1-m1E01-1-0597 | 725.198 | OH | a1 | B01 | b1 | C14 | c2 | D02 | d1 | E01 |
| 2-1-m1E01-1-0598 | 725.252 | OH | a1 | B05 | b2 | C02 | c2 | D02 | d1 | E01 |
| 2-1-m1E01-1-0599 | 725.257 | OH | a1 | B09 | b1 | C13 | c1 | D01 | d1 | E01 |
| 2-1-m1E01-1-0600 | 725.288 | OH | a1 | B05 | b2 | C22 | c3 | D02 | d1 | E01 |
| 2-1-m1E01-1-0601 | 725.288 | OH | a1 | B06 | b1 | C32 | c3 | D02 | d1 | E01 |
| 2-1-m1E01-1-0602 | 726.193 | OH | a1 | B02 | b3 | C17 | c1 | D01 | d1 | E01 |
| 2-1-m1E01-1-0603 | 726.193 | OH | a1 | B08 | b3 | C08 | c1 | D01 | d1 | E01 |
| 2-1-m1E01-1-0604 | 726.193 | OH | a1 | B08 | b1 | C08 | c2 | D02 | d1 | E01 |
| 2-1-m1E01-1-0605 | 726.193 | OH | a1 | B02 | b1 | C14 | c2 | D02 | d1 | E01 |
| 2-1-m1E01-1-0606 | 726.212 | OH | a2 | B20 | b1 | C14 | c1 | D01 | d1 | E01 |
| 2-1-m1E01-1-0607 | 726.216 | OH | a2 | B16 | b2 | C04 | c2 | D02 | d1 | E01 |
| 2-1-m1E01-1-0608 | 726.234 | OH | a2 | B20 | b2 | C33 | c3 | D02 | d1 | E01 |
| 2-1-m1E01-1-0609 | 726.247 | OH | a1 | B05 | b2 | C03 | c2 | D02 | d1 | E01 |
| 2-1-m1E01-1-0610 | 726.247 | OH | a1 | B03 | b2 | C05 | c2 | D02 | d1 | E01 |
| 2-1-m1E01-1-0611 | 726.269 | OH | a2 | B17 | b1 | C29 | c3 | D02 | d1 | E01 |
| 2-1-m1E01-1-0612 | 726.288 | OH | a2 | B18 | b1 | C32 | c3 | D02 | d1 | E01 |
| 2-1-m1E01-1-0613 | 727.188 | OH | a1 | B08 | b1 | C11 | c2 | D02 | d1 | E01 |
| 2-1-mlE01-1-0614 | 727.192 | OH | a1 | B09 | b3 | C15 | c1 | D01 | d1 | E01 |
| 2-1-m1E01-1-0615 | 727.231 | OH | a1 | B06 | b2 | C02 | c2 | D02 | d1 | E01 |
| 2-1-m1E01-1-0616 | 727.268 | OH | a1 | B06 | b2 | C22 | c3 | D02 | d1 | E01 |
| 2-1-m1E01-1-0617 | 727.268 | OH | a1 | B03 | b2 | C26 | c3 | D02 | d1 | E01 |
| 2-1-m1E01-1-0618 | 727.284 | OH | a1 | B04 | b1 | C29 | c3 | D02 | d1 | E01 |
| 2-1-m1E01-1-0619 | 728.184 | OH | a1 | B08 | b1 | C10 | c2 | D02 | d1 | E01 |
| 2-1-m1E01-1-0620 | 728.197 | OH | a2 | B13 | b3 | C08 | c2 | D02 | d1 | E01 |
| 2-1-m1E01-1-0621 | 728.226 | OH | a1 | B06 | b2 | C03 | c2 | D02 | d1 | E01 |
| 2-1-m1E01-1-0622 | 728.230 | OH | a2 | B18 | b2 | C02 | c2 | D02 | d1 | E01 |
| 2-1-m1E01-1-0623 | 728.267 | OH | a2 | B18 | b2 | C22 | c3 | D02 | d1 | E01 |
| 2-1-m1E01-1-0624 | 728.268 | OH | a2 | B14 | b2 | C07 | c2 | D02 | d1 | E01 |
| 2-1-m1E01-1-0625 | 729.207 | OH | a2 | B17 | b2 | C05 | c2 | D02 | d1 | E01 |
| 2-1-m1E01-1-0626 | 729.223 | OH | a1 | B09 | b1 | C14 | c1 | D01 | d1 | E01 |
| 2-1-m1E01-1-0627 | 729.226 | OH | a2 | B18 | b2 | C03 | c2 | D02 | d1 | E01 |
| 2-1-m1E01-1-0628 | 729.227 | OH | a1 | B03 | b2 | C04 | c2 | D02 | d1 | E01 |
| 2-1-m1E01-1-0629 | 729.244 | OH | a1 | B09 | b2 | C33 | c3 | D02 | d1 | E01 |
| 2-1-m1E01-1-0630 | 729.263 | OH | a1 | B05 | b2 | C24 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0631 | 729.283 | OH | a1 | B01 | b2 | C07 | c2 | D02 | d1 | E01 |
| 2-1-m1E01-1-0632 | 730.219 | OH | a1 | B08 | b1 | C09 | c1 | D01 | d1 | E01 |
| 2-1-m1E01-1-0633 | 730.222 | OH | a1 | B04 | b2 | C05 | c2 | D02 | d1 | E01 |
| 2-1-m1E01-1-0634 | 730.227 | OH | a2 | B17 | b2 | C26 | c3 | D02 | d1 | E01 |
| 2-1-m1E01-1-0635 | 730.244 | OH | a2 | B19 | b1 | C30 | c3 | D02 | d1 | E01 |
| 2-1-m1E01-1-0636 | 730.257 | OH | a2 | B15 | b1 | C13 | c2 | D02 | d1 | E01 |
| 2-1-m1E01-1-0637 | 730.278 | OH | a1 | B02 | b2 | C07 | c2 | D02 | d1 | E01 |
| 2-1-m1E01-1-0638 | 730.298 | OH | a2 | B20 | b2 | C07 | c1 | D01 | d1 | E01 |
| 2-1-m1E01-1-0639 | 731.208 | OH | a1 | B05 | b2 | C06 | c2 | D02 | d1 | E01 |
| 2-1-m1E01-1-0640 | 731.243 | OH | a1 | B06 | b2 | C24 | c3 | D02 | d1 | E01 |
| 2-1-m1E01-1-0641 | 731.243 | OH | a1 | B04 | b2 | C26 | c3 | D02 | d1 | E01 |
| 2-1-m1E01-1-0642 | 731.259 | OH | a1 | B07 | b1 | C32 | c3 | D02 | d1 | E01 |
| 2-1-m1E01-1-0643 | 732.149 | OH | a1 | B08 | b3 | C06 | c1 | D01 | d1 | E01 |
| 2-1-m1E01-1-0644 | 732.172 | OH | a2 | B14 | b3 | C08 | c2 | D02 | d1 | E01 |
| 2-1-m1E01-1-0645 | 732.187 | OH | a2 | B17 | b2 | C04 | c2 | D02 | d1 | E01 |
| 2-1-m1E01-1-0646 | 732.192 | OH | a2 | B15 | b3 | C15 | c2 | D02 | d1 | E01 |
| 2-1-m1E01-1-0647 | 732.223 | OH | a2 | B13 | b1 | C09 | c2 | D02 | d1 | E01 |
| 2-1-m1E01-1-0648 | 732.223 | OH | a2 | B19 | b2 | C20 | c3 | D02 | d1 | E01 |
| 2-1-m1E01-1-0649 | 732.237 | OH | a2 | B16 | b1 | C13 | c2 | D02 | d1 | E01 |
| 2-1-m1E01-1-0650 | 732.242 | OH | a2 | B18 | b2 | C24 | c3 | D02 | d1 | E01 |
| 2-1-m1E01-1-0651 | 732.252 | OH | a1 | B10 | b2 | C05 | c1 | D01 | d1 | E01 |
| 2-1-m1E01-1-0652 | 732.262 | OH | a2 | B15 | b3 | C16 | c1 | D01 | d1 | E01 |
| 2-1-m1E01-1-0653 | 732.298 | OH | a2 | B13 | b1 | C31 | c3 | D02 | d1 | E01 |
| 2-1-m1E01-1-0654 | 733.145 | OH | a1 | B08 | b1 | C12 | c2 | D02 | d1 | E01 |
| 2-1-m1E01-1-0655 | 733.188 | OH | a1 | B06 | b2 | C06 | c2 | D02 | d1 | E01 |
| 2-1-m1E01-1-0656 | 733.188 | OH | a1 | B01 | b3 | C08 | c2 | D02 | d1 | E01 |
| 2-1-m1E01-1-0657 | 733.202 | OH | a1 | B07 | b2 | C02 | c2 | D02 | d1 | E01 |
| 2-1-m1E01-1-0658 | 733.202 | OH | a1 | B04 | b2 | C04 | c2 | D02 | d1 | E01 |
| 2-1-m1E01-1-0659 | 733.218 | OH | a2 | B19 | b2 | C28 | c3 | D02 | d1 | E01 |
| 2-1-m1E01-1-0660 | 733.238 | OH | a1 | B07 | b2 | C22 | c3 | D02 | d1 | E01 |
| 2-1-m1E01-1-0661 | 733.309 | OH | a1 | B09 | b2 | C07 | c1 | D01 | d1 | E01 |
| 2-1-m1E01-1-0662 | 734.154 | OH | a2 | B13 | b3 | C06 | c2 | D02 | d1 | E01 |
| 2-1-m1E01-1-0663 | 734.172 | OH | a2 | B16 | b3 | C15 | c2 | D02 | d1 | E01 |
| 2-1-m1E01-1-0664 | 734.183 | OH | a1 | B02 | b3 | C08 | c2 | D02 | d1 | E01 |
| 2-1-m1E01-1-0665 | 734.187 | OH | a2 | B18 | b2 | C06 | c2 | D02 | d1 | E01 |
| 2-1-m1E01-1-0666 | 734.197 | OH | a1 | B07 | b2 | C03 | c2 | D02 | d1 | E01 |
| 2-1-m1E01-1-0667 | 734.202 | OH | a2 | B20 | b3 | C08 | c1 | D01 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0668 | 734.202 | OH | a2 | B20 | b1 | C08 | c2 | D02 | d1 | E01 |
| 2-1-m1E01-1-0669 | 734.223 | OH | a2 | B15 | b3 | C17 | c1 | D01 | d1 | E01 |
| 2-1-m1E01-1-0670 | 734.223 | OH | a2 | B15 | b1 | C14 | c2 | D02 | d1 | E01 |
| 2-1-m1E01-1-0671 | 734.241 | OH | a2 | B16 | b3 | C16 | c1 | D01 | d1 | E01 |
| 2-1-m1E01-1-0672 | 735.197 | OH | a2 | B20 | b1 | C11 | c2 | D02 | d1 | E01 |
| 2-1-m1E01-1-0673 | 735.219 | OH | a2 | B13 | b3 | C18 | c1 | D01 | d1 | E01 |
| 2-1-m1E01-1-0674 | 735.232 | OH | a1 | B10 | b2 | C04 | c1 | D01 | d1 | E01 |
| 2-1-m1E01-1-0675 | 735.248 | OH | a1 | B03 | b1 | C13 | c2 | D02 | d1 | E01 |
| 2-1-m1E01-1-0676 | 736.181 | OH | a2 | B19 | b2 | C01 | c2 | D02 | d1 | E01 |
| 2-1-m1E01-1-0677 | 736.193 | OH | a2 | B20 | b1 | C10 | c2 | D02 | d1 | E01 |
| 2-1-m1E01-1-0678 | 736.198 | OH | a2 | B14 | b1 | C09 | c2 | D02 | d1 | E01 |
| 2-1-m1E01-1-0679 | 736.203 | OH | a2 | B16 | b3 | C17 | c1 | D01 | d1 | E01 |
| 2-1-m1E01-1-0680 | 736.203 | OH | a2 | B16 | b1 | C14 | c2 | D02 | d1 | E01 |
| 2-1-m1E01-1-0681 | 736.273 | OH | a2 | B14 | b1 | C31 | c3 | D02 | d1 | E01 |
| 2-1-m1E01-1-0682 | 737.183 | OH | a1 | B03 | b3 | C15 | c2 | D02 | d1 | E01 |
| 2-1-m1E01-1-0683 | 737.213 | OH | a1 | B09 | b3 | C08 | c1 | D01 | d1 | E01 |
| 2-1-m1E01-1-0684 | 737.213 | OH | a1 | B09 | b1 | C08 | c2 | D02 | d1 | E01 |
| 2-1-m1E01-1-0685 | 737.213 | OH | a1 | B01 | b1 | C09 | c2 | D02 | d1 | E01 |
| 2-1-m1E01-1-0686 | 737.213 | OH | a1 | B07 | b2 | C24 | c3 | D02 | d1 | E01 |
| 2-1-m1E01-1-0687 | 737.252 | OH | a1 | B03 | b3 | C16 | c1 | D01 | d1 | E01 |
| 2-1-m1E01-1-0688 | 737.288 | OH | a1 | B01 | b1 | C31 | c3 | D02 | d1 | E01 |
| 2-1-m1E01-1-0689 | 737.325 | OH | a1 | B05 | b1 | C29 | c3 | D02 | d1 | E01 |
| 2-1-m1E01-1-0690 | 738.129 | OH | a2 | B14 | b3 | C06 | c2 | D02 | d1 | E01 |
| 2-1-m1E01-1-0691 | 738.207 | OH | a2 | B17 | b1 | C13 | c2 | D02 | d1 | E01 |
| 2-1-m1E01-1-0692 | 738.208 | OH | a1 | B02 | b1 | C09 | c2 | D02 | d1 | E01 |
| 2-1-m1E01-1-0693 | 738.208 | OH | a1 | B09 | b1 | C11 | c2 | D02 | d1 | E01 |
| 2-1-m1E01-1-0694 | 738.228 | OH | a2 | B20 | b1 | C09 | c1 | D01 | d1 | E01 |
| 2-1-m1E01-1-0695 | 738.229 | OH | a1 | B08 | b1 | C30 | c3 | D02 | d1 | E01 |
| 2-1-m1E01-1-0696 | 738.284 | OH | a1 | B02 | b1 | C31 | c3 | D02 | d1 | E01 |
| 2-1-m1E01-1-0697 | 738.309 | OH | a2 | B15 | b2 | C07 | c2 | D02 | d1 | E01 |
| 2-1-m1E01-1-0698 | 739.144 | OH | a1 | B01 | b3 | C06 | c2 | D02 | d1 | E01 |
| 2-1-m1E01-1-0699 | 739.158 | OH | a1 | B07 | b2 | C06 | c2 | D02 | d1 | E01 |
| 2-1-m1E01-1-0700 | 739.193 | OH | a2 | B14 | b3 | C18 | c1 | D01 | d1 | E01 |
| 2-1-m1E01-1-0701 | 739.204 | OH | a1 | B09 | b1 | C10 | c2 | D02 | d1 | E01 |
| 2-1-m1E01-1-0702 | 739.213 | OH | a1 | B03 | b3 | C17 | c1 | D01 | d1 | E01 |
| 2-1-m1E01-1-0703 | 739.213 | OH | a1 | B03 | b1 | C14 | c2 | D02 | d1 | E01 |
| 2-1-m1E01-1-0704 | 739.222 | OH | a1 | B04 | b1 | C13 | c2 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0705 | 739.304 | OH | a1 | B06 | b1 | C29 | c3 | D02 | d1 | E01 |
| 2-1-m1E01-1-0706 | 740.139 | OH | a1 | B02 | b3 | C06 | c2 | D02 | d1 | E01 |
| 2-1-m1E01-1-0707 | 740.142 | OH | a2 | B17 | b3 | C15 | c2 | D02 | d1 | E01 |
| 2-1-m1E01-1-0708 | 740.159 | OH | a2 | B20 | b3 | C06 | c1 | D01 | d1 | E01 |
| 2-1-m1E01-1-0709 | 740.209 | OH | a1 | B01 | b3 | C18 | c1 | D01 | d1 | E01 |
| 2-1-m1E01-1-0710 | 740.209 | OH | a1 | B08 | b2 | C20 | c3 | D02 | d1 | E01 |
| 2-1-m1E01-1-0711 | 740.212 | OH | a2 | B17 | b3 | C16 | c1 | D01 | d1 | E01 |
| 2-1-m1E01-1-0712 | 740.263 | OH | a1 | B05 | b2 | C05 | c2 | D02 | d1 | E01 |
| 2-1-m1E01-1-0713 | 740.288 | OH | a2 | B16 | b2 | C07 | c2 | D02 | d1 | E01 |
| 2-1-m1E01-1-0714 | 740.303 | OH | a2 | B18 | b1 | C29 | c3 | D02 | d1 | E01 |
| 2-1-m1E01-1-0715 | 741.154 | OH | a2 | B20 | b1 | C12 | c2 | D02 | d1 | E01 |
| 2-1-m1E01-1-0716 | 741.157 | OH | a1 | B04 | b3 | C15 | c2 | D02 | d1 | E01 |
| 2-1-m1E01-1-0717 | 741.204 | OH | a1 | B02 | b3 | C18 | c1 | D01 | d1 | E01 |
| 2-1-m1E01-1-0718 | 741.204 | OH | a1 | B08 | b2 | C28 | c3 | D02 | d1 | E01 |
| 2-1-m1E01-1-0719 | 741.227 | OH | a1 | B04 | b3 | C16 | c1 | D01 | d1 | E01 |
| 2-1-m1E01-1-0720 | 741.239 | OH | a1 | B09 | b1 | C09 | c1 | D01 | d1 | E01 |
| 2-1-m1E01-1-0721 | 741.252 | OH | a1 | B10 | b1 | C13 | c1 | D01 | d1 | E01 |
| 2-1-m1E01-1-0722 | 741.283 | OH | a1 | B05 | b2 | C26 | c3 | D02 | d1 | E01 |
| 2-1-m1E01-1-0723 | 742.173 | OH | a2 | B17 | b3 | C17 | c1 | D01 | d1 | E01 |
| 2-1-m1E01-1-0724 | 742.173 | OH | a2 | B17 | b1 | C14 | c2 | D02 | d1 | E01 |
| 2-1-m1E01-1-0725 | 742.213 | OH | a2 | B15 | b3 | C08 | c2 | D02 | d1 | E01 |
| 2-1-m1E01-1-0726 | 742.242 | OH | a1 | B06 | b2 | C05 | c2 | D02 | d1 | E01 |
| 2-1-m1E01-1-0727 | 743.170 | OH | a1 | B09 | b3 | C06 | c1 | D01 | d1 | E01 |
| 2-1-m1E01-1-0728 | 743.187 | OH | a1 | B10 | b3 | C15 | c1 | D01 | d1 | E01 |
| 2-1-m1E01-1-0729 | 743.188 | OH | a1 | B04 | b3 | C17 | c1 | D01 | d1 | E01 |
| 2-1-m1E01-1-0730 | 743.188 | OH | a1 | B04 | b1 | C14 | c2 | D02 | d1 | E01 |
| 2-1-m1E01-1-0731 | 743.241 | OH | a2 | B18 | b2 | C05 | c2 | D02 | d1 | E01 |
| 2-1-m1E01-1-0732 | 743.243 | OH | a1 | B05 | b2 | C04 | c2 | D02 | d1 | E01 |
| 2-1-m1E01-1-0733 | 743.262 | OH | a1 | B06 | b2 | C26 | c3 | D02 | d1 | E01 |
| 2-1-m1E01-1-0734 | 743.299 | OH | a1 | B03 | b2 | C07 | c2 | D02 | d1 | E01 |
| 2-1-m1E01-1-0735 | 744.165 | OH | a1 | B09 | b1 | C12 | c2 | D02 | d1 | E01 |
| 2-1-m1E01-1-0736 | 744.167 | OH | a1 | B08 | b2 | C01 | c2 | D02 | d1 | E01 |
| 2-1-m1E01-1-0737 | 744.192 | OH | a2 | B16 | b3 | C08 | c2 | D02 | d1 | E01 |
| 2-1-m1E01-1-0738 | 744.259 | OH | a2 | B19 | b1 | C32 | c3 | D02 | d1 | E01 |
| 2-1-m1E01-1-0739 | 744.262 | OH | a2 | B18 | b2 | C26 | c3 | D02 | d1 | E01 |
| 2-1-m1E01-1-0740 | 745.218 | OH | a1 | B10 | b1 | C14 | c1 | D01 | d1 | E01 |
| 2-1-m1E01-1-0741 | 745.222 | OH | a1 | B06 | b2 | C04 | c2 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0742 | 745.239 | OH | a1 | B10 | b2 | C33 | c3 | D02 | d1 | E01 |
| 2-1-m1E01-1-0743 | 745.275 | OH | a1 | B07 | b1 | C29 | c3 | D02 | d1 | E01 |
| 2-1-m1E01-1-0744 | 746.202 | OH | a2 | B19 | b2 | C02 | c2 | D02 | d1 | E01 |
| 2-1-m1E01-1-0745 | 746.221 | OH | a2 | B18 | b2 | C04 | c2 | D02 | d1 | E01 |
| 2-1-m1E01-1-0746 | 746.239 | OH | a2 | B15 | b1 | C09 | c2 | D02 | d1 | E01 |
| 2-1-m1E01-1-0747 | 746.239 | OH | a2 | B19 | b2 | C22 | c3 | D02 | d1 | E01 |
| 2-1-m1E01-1-0748 | 746.239 | OH | a2 | B20 | b1 | C30 | c3 | D02 | d1 | E01 |
| 2-1-m1E01-1-0749 | 746.259 | OH | a2 | B17 | b2 | C07 | c2 | D02 | d1 | E01 |
| 2-1-m1E01-1-0750 | 746.314 | OH | a2 | B15 | b1 | C31 | c3 | D02 | d1 | E01 |
| 2-1-m1E01-1-0751 | 747.197 | OH | a2 | B19 | b2 | C03 | c2 | D02 | d1 | E01 |
| 2-1-m1E01-1-0752 | 747.203 | OH | a1 | B03 | b3 | C08 | c2 | D02 | d1 | E01 |
| 2-1-m1E01-1-0753 | 747.274 | OH | a1 | B04 | b2 | C07 | c2 | D02 | d1 | E01 |
| 2-1-m1E01-1-0754 | 748.170 | OH | a2 | B15 | b3 | C06 | c2 | D02 | d1 | E01 |
| 2-1-m1E01-1-0755 | 748.213 | OH | a1 | B07 | b2 | C05 | c2 | D02 | d1 | E01 |
| 2-1-m1E01-1-0756 | 748.218 | OH | a2 | B16 | b1 | C09 | c2 | D02 | d1 | E01 |
| 2-1-m1E01-1-0757 | 748.218 | OH | a2 | B20 | b2 | C20 | c3 | D02 | d1 | E01 |
| 2-1-m1E01-1-0758 | 748.293 | OH | a2 | B16 | b1 | C31 | c3 | D02 | d1 | E01 |
| 2-1-m1E01-1-0759 | 749.213 | OH | a2 | B20 | b2 | C28 | c3 | D02 | d1 | E01 |
| 2-1-m1E01-1-0760 | 749.233 | OH | a1 | B07 | b2 | C26 | c3 | D02 | d1 | E01 |
| 2-1-m1E01-1-0761 | 749.234 | OH | a2 | B15 | b3 | C18 | c1 | D01 | d1 | E01 |
| 2-1-m1E01-1-0762 | 749.249 | OH | a1 | B09 | b1 | C30 | c3 | D02 | d1 | E01 |
| 2-1-m1E01-1-0763 | 749.263 | OH | a1 | B05 | b1 | C13 | c2 | D02 | d1 | E01 |
| 2-1-m1E01-1-0764 | 749.304 | OH | a1 | B10 | b2 | C07 | c1 | D01 | d1 | E01 |
| 2-1-m1E01-1-0765 | 750.149 | OH | a2 | B16 | b3 | C06 | c2 | D02 | d1 | E01 |
| 2-1-m1E01-1-0766 | 750.163 | OH | a2 | B17 | b3 | C08 | c2 | D02 | d1 | E01 |
| 2-1-m1E01-1-0767 | 750.214 | OH | a2 | B19 | b2 | C24 | c3 | D02 | d1 | E01 |
| 2-1-m1E01-1-0768 | 751.178 | OH | a1 | B04 | b3 | C08 | c2 | D02 | d1 | E01 |
| 2-1-m1E01-1-0769 | 751.192 | OH | a1 | B07 | b2 | C04 | c2 | D02 | d1 | E01 |
| 2-1-m1E01-1-0770 | 751.198 | OH | a1 | B05 | b3 | C15 | c2 | D02 | d1 | E01 |
| 2-1-m1E01-1-0771 | 751.213 | OH | a2 | B16 | b3 | C18 | c1 | D01 | d1 | E01 |
| 2-1-m1E01-1-0772 | 751.229 | OH | a1 | B03 | b1 | C09 | c2 | D02 | d1 | E01 |
| 2-1-m1E01-1-0773 | 751.229 | OH | a1 | B09 | b2 | C20 | c3 | D02 | d1 | E01 |
| 2-1-m1E01-1-0774 | 751.242 | OH | a1 | B06 | b1 | C13 | c2 | D02 | d1 | E01 |
| 2-1-m1E01-1-0775 | 751.268 | OH | a1 | B05 | b3 | C16 | c1 | D01 | d1 | E01 |
| 2-1-m1E01-1-0776 | 751.304 | OH | a1 | B03 | b1 | C31 | c3 | D02 | d1 | E01 |
| 2-1-m1E01-1-0777 | 752.159 | OH | a2 | B19 | b2 | C06 | c2 | D02 | d1 | E01 |
| 2-1-m1E01-1-0778 | 752.176 | OH | a2 | B20 | b2 | C01 | c2 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0779 | 752.224 | OH | a1 | B09 | b2 | C28 | c3 | D02 | d1 | E01 |
| 2-1-m1E01-1-0780 | 752.242 | OH | a2 | B18 | b1 | C13 | c2 | D02 | d1 | E01 |
| 2-1-m1E01-1-0781 | 752.245 | OH | a1 | B08 | b1 | C32 | c3 | D02 | d1 | E01 |
| 2-1-m1E01-1-0782 | 753.160 | OH | a1 | B03 | b3 | C06 | c2 | D02 | d1 | E01 |
| 2-1-m1E01-1-0783 | 753.177 | OH | a1 | B06 | b3 | C15 | c2 | D02 | d1 | E01 |
| 2-1-m1E01-1-0784 | 753.208 | OH | a1 | B10 | b3 | C08 | c1 | D01 | d1 | E01 |
| 2-1-m1E01-1-0785 | 753.208 | OH | a1 | B10 | b1 | C08 | c2 | D02 | d1 | E01 |
| 2-1-m1E01-1-0786 | 753.229 | OH | a1 | B05 | b3 | C17 | c1 | D01 | d1 | E01 |
| 2-1-m1E01-1-0787 | 753.229 | OH | a1 | B05 | b1 | C14 | c2 | D02 | d1 | E01 |
| 2-1-m1E01-1-0788 | 753.247 | OH | a1 | B06 | b3 | C16 | c1 | D01 | d1 | E01 |
| 2-1-m1E01-1-0789 | 754.177 | OH | a2 | B18 | b3 | C15 | c2 | D02 | d1 | E01 |
| 2-1-m1E01-1-0790 | 754.188 | OH | a1 | B08 | b2 | C02 | c2 | D02 | d1 | E01 |
| 2-1-m1E01-1-0791 | 754.188 | OH | a2 | B17 | b1 | C09 | c2 | D02 | d1 | E01 |
| 2-1-m1E01-1-0792 | 754.203 | OH | a1 | B10 | b1 | C11 | c2 | D02 | d1 | E01 |
| 2-1-m1E01-1-0793 | 754.224 | OH | a1 | B03 | b3 | C18 | c1 | D01 | d1 | E01 |
| 2-1-m1E01-1-0794 | 754.224 | OH | a1 | B08 | b2 | C22 | c3 | D02 | d1 | E01 |
| 2-1-m1E01-1-0795 | 754.246 | OH | a2 | B18 | b3 | C16 | c1 | D01 | d1 | E01 |
| 2-1-m1E01-1-0796 | 754.264 | OH | a2 | B17 | b1 | C31 | c3 | D02 | d1 | E01 |
| 2-1-m1E01-1-0797 | 755.183 | OH | a1 | B08 | b2 | C03 | c2 | D02 | d1 | E01 |
| 2-1-m1E01-1-0798 | 755.187 | OH | a1 | B09 | b2 | C01 | c2 | D02 | d1 | E01 |
| 2-1-m1E01-1-0799 | 755.199 | OH | a1 | B10 | b1 | C10 | c2 | D02 | d1 | E01 |
| 2-1-m1E01-1-0800 | 755.204 | OH | a1 | B04 | b1 | C09 | c2 | D02 | d1 | E01 |
| 2-1-m1E01-1-0801 | 755.208 | OH | a1 | B06 | b3 | C17 | c1 | D01 | d1 | E01 |
| 2-1-m1E01-1-0802 | 755.208 | OH | a1 | B06 | b1 | C14 | c2 | D02 | d1 | E01 |
| 2-1-m1E01-1-0803 | 755.279 | OH | a1 | B04 | b1 | C31 | c3 | D02 | d1 | E01 |
| 2-1-m1E01-1-0804 | 756.119 | OH | a2 | B17 | b3 | C06 | c2 | D02 | d1 | E01 |
| 2-1-m1E01-1-0805 | 756.208 | OH | a2 | B18 | b3 | C17 | c1 | D01 | d1 | E01 |
| 2-1-m1E01-1-0806 | 756.208 | OH | a2 | B18 | b1 | C14 | c2 | D02 | d1 | E01 |
| 2-1-m1E01-1-0807 | 757.135 | OH | a1 | B04 | b3 | C06 | c2 | D02 | d1 | E01 |
| 2-1-m1E01-1-0808 | 757.184 | OH | a2 | B17 | b3 | C18 | c1 | D01 | d1 | E01 |
| 2-1-m1E01-1-0809 | 757.213 | OH | a1 | B07 | b1 | C13 | c2 | D02 | d1 | E01 |
| 2-1-m1E01-1-0810 | 757.234 | OH | a1 | B10 | b1 | C09 | c1 | D01 | d1 | E01 |
| 2-1-m1E01-1-0811 | 757.315 | OH | a1 | B05 | b2 | C07 | c2 | D02 | d1 | E01 |
| 2-1-m1E01-1-0812 | 758.199 | OH | a1 | B04 | b3 | C18 | c1 | D01 | d1 | E01 |
| 2-1-m1E01-1-0813 | 758.199 | OH | a1 | B08 | b2 | C24 | c3 | D02 | d1 | E01 |
| 2-1-m1E01-1-0814 | 758.275 | OH | a2 | B19 | b1 | C29 | c3 | D02 | d1 | E01 |
| 2-1-m1E01-1-0815 | 759.148 | OH | a1 | B07 | b3 | C15 | c2 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0816 | 759.164 | OH | a1 | B10 | b3 | C06 | c1 | D01 | d1 | E01 |
| 2-1-m1E01-1-0817 | 759.217 | OH | a1 | B07 | b3 | C16 | c1 | D01 | d1 | E01 |
| 2-1-m1E01-1-0818 | 759.294 | OH | a1 | B06 | b2 | C07 | c2 | D02 | d1 | E01 |
| 2-1-m1E01-1-0819 | 760.144 | OH | a1 | B08 | b2 | C06 | c2 | D02 | d1 | E01 |
| 2-1-m1E01-1-0820 | 760.160 | OH | a1 | B10 | b1 | C12 | c2 | D02 | d1 | E01 |
| 2-1-m1E01-1-0821 | 760.254 | OH | a2 | B20 | b1 | C32 | c3 | D02 | d1 | E01 |
| 2-1-m1E01-1-0822 | 760.293 | OH | a2 | B18 | b2 | C07 | c2 | D02 | d1 | E01 |
| 2-1-m1E01-1-0823 | 761.179 | OH | a1 | B07 | b3 | C17 | c1 | D01 | d1 | E01 |
| 2-1-m1E01-1-0824 | 761.179 | OH | a1 | B07 | b1 | C14 | c2 | D02 | d1 | E01 |
| 2-1-m1E01-1-0825 | 761.213 | OH | a2 | B19 | b2 | C05 | c2 | D02 | d1 | E01 |
| 2-1-m1E01-1-0826 | 761.219 | OH | a1 | B05 | b3 | C08 | c2 | D02 | d1 | E01 |
| 2-1-m1E01-1-0827 | 762.197 | OH | a2 | B20 | b2 | C02 | c2 | D02 | d1 | E01 |
| 2-1-m1E01-1-0828 | 762.234 | OH | a2 | B20 | b2 | C22 | c3 | D02 | d1 | E01 |
| 2-1-m1E01-1-0829 | 762.234 | OH | a2 | B19 | b2 | C26 | c3 | D02 | d1 | E01 |
| 2-1-m1E01-1-0830 | 763.192 | OH | a2 | B20 | b2 | C03 | c2 | D02 | d1 | E01 |
| 2-1-m1E01-1-0831 | 763.198 | OH | a1 | B06 | b3 | C08 | c2 | D02 | d1 | E01 |
| 2-1-m1E01-1-0832 | 763.265 | OH | a1 | B09 | b1 | C32 | c3 | D02 | d1 | E01 |
| 2-1-m1E01-1-0833 | 764.193 | OH | a2 | B19 | b2 | C04 | c2 | D02 | d1 | E01 |
| 2-1-m1E01-1-0834 | 764.197 | OH | a2 | B18 | b3 | C08 | c2 | D02 | d1 | E01 |
| 2-1-m1E01-1-0835 | 765.208 | OH | a1 | B09 | b2 | C02 | c2 | D02 | d1 | E01 |
| 2-1-m1E01-1-0836 | 765.244 | OH | a1 | B05 | b1 | C09 | c2 | D02 | d1 | E01 |
| 2-1-m1E01-1-0837 | 765.244 | OH | a1 | B09 | b2 | C22 | c3 | D02 | d1 | E01 |
| 2-1-m1E01-1-0838 | 765.244 | OH | a1 | B10 | b1 | C30 | c3 | D02 | d1 | E01 |
| 2-1-m1E01-1-0839 | 765.264 | OH | a1 | B07 | b2 | C07 | c2 | D02 | d1 | E01 |
| 2-1-m1E01-1-0840 | 765.320 | OH | a1 | B05 | b1 | C31 | c3 | D02 | d1 | E01 |
| 2-1-m1E01-1-0841 | 766.203 | OH | a1 | B09 | b2 | C03 | c2 | D02 | d1 | E01 |
| 2-1-m1E01-1-0842 | 766.208 | OH | a2 | B20 | b2 | C24 | c3 | D02 | d1 | E01 |
| 2-1-m1E01-1-0843 | 766.261 | OH | a1 | B08 | b1 | C29 | c3 | D02 | d1 | E01 |
| 2-1-m1E01-1-0844 | 767.175 | OH | a1 | B05 | b3 | C06 | c2 | D02 | d1 | E01 |
| 2-1-m1E01-1-0845 | 767.224 | OH | a1 | B06 | b1 | C09 | c2 | D02 | d1 | E01 |
| 2-1-m1E01-1-0846 | 767.224 | OH | a1 | B10 | b2 | C20 | c3 | D02 | d1 | E01 |
| 2-1-m1E01-1-0847 | 767.299 | OH | a1 | B06 | b1 | C31 | c3 | D02 | d1 | E01 |
| 2-1-m1E01-1-0848 | 768.154 | OH | a2 | B20 | b2 | C06 | c2 | D02 | d1 | E01 |
| 2-1-m1E01-1-0849 | 768.192 | OH | a2 | B11 | b3 | C16 | c2 | D02 | d1 | E01 |
| 2-1-m1E01-1-0850 | 768.219 | OH | a1 | B10 | b2 | C28 | c3 | D02 | d1 | E01 |
| 2-1-m1E01-1-0851 | 768.223 | OH | a2 | B18 | b1 | C09 | c2 | D02 | d1 | E01 |
| 2-1-m1E01-1-0852 | 768.240 | OH | a1 | B05 | b3 | C18 | c1 | D01 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0853 | 768.298 | OH | a2 | B18 | b1 | C31 | c3 | D02 | d1 | E01 |
| 2-1-m1E01-1-0854 | 769.155 | OH | a1 | B06 | b3 | C06 | c2 | D02 | d1 | E01 |
| 2-1-m1E01-1-0855 | 769.169 | OH | a1 | B07 | b3 | C08 | c2 | D02 | d1 | E01 |
| 2-1-m1E01-1-0856 | 769.188 | OH | a2 | B12 | b3 | C16 | c2 | D02 | d1 | E01 |
| 2-1-m1E01-1-0857 | 769.199 | OH | a1 | B08 | b2 | C05 | c2 | D02 | d1 | E01 |
| 2-1-m1E01-1-0858 | 769.219 | OH | a1 | B09 | b2 | C24 | c3 | D02 | d1 | E01 |
| 2-1-m1E01-1-0859 | 770.154 | OH | a2 | B18 | b3 | C06 | c2 | D02 | d1 | E01 |
| 2-1-m1E01-1-0860 | 770.154 | OH | a2 | B11 | b3 | C17 | c2 | D02 | d1 | E01 |
| 2-1-m1E01-1-0861 | 770.213 | OH | a2 | B19 | b1 | C13 | c2 | D02 | d1 | E01 |
| 2-1-m1E01-1-0862 | 770.219 | OH | a1 | B06 | b3 | C18 | c1 | D01 | d1 | E01 |
| 2-1-m1E01-1-0863 | 770.219 | OH | a1 | B08 | b2 | C26 | c3 | D02 | d1 | E01 |
| 2-1-m1E01-1-0864 | 771.149 | OH | a2 | B12 | b3 | C17 | c2 | D02 | d1 | E01 |
| 2-1-m1E01-1-0865 | 771.164 | OH | a1 | B09 | b2 | C06 | c2 | D02 | d1 | E01 |
| 2-1-m1E01-1-0866 | 771.182 | OH | a1 | B10 | b2 | C01 | c2 | D02 | d1 | E01 |
| 2-1-m1E01-1-0867 | 771.219 | OH | a2 | B18 | b3 | C18 | c1 | D01 | d1 | E01 |
| 2-1-m1E01-1-0868 | 772.148 | OH | a2 | B19 | b3 | C15 | c2 | D02 | d1 | E01 |
| 2-1-m1E01-1-0869 | 772.179 | OH | a1 | B08 | b2 | C04 | c2 | D02 | d1 | E01 |
| 2-1-m1E01-1-0870 | 772.218 | OH | a2 | B19 | b3 | C16 | c1 | D01 | d1 | E01 |
| 2-1-m1E01-1-0871 | 773.194 | OH | a1 | B07 | b1 | C09 | c2 | D02 | d1 | E01 |
| 2-1-m1E01-1-0872 | 773.269 | OH | a1 | B07 | b1 | C31 | c3 | D02 | d1 | E01 |
| 2-1-m1E01-1-0873 | 774.179 | OH | a2 | B19 | b3 | C17 | c1 | D01 | d1 | E01 |
| 2-1-m1E01-1-0874 | 774.179 | OH | a2 | B19 | b1 | C14 | c2 | D02 | d1 | E01 |
| 2-1-m1E01-1-0875 | 774.270 | OH | a2 | B20 | b1 | C29 | c3 | D02 | d1 | E01 |
| 2-1-m1E01-1-0876 | 775.125 | OH | a1 | B07 | b3 | C06 | c2 | D02 | d1 | E01 |
| 2-1-m1E01-1-0877 | 776.190 | OH | a1 | B07 | b3 | C18 | c1 | D01 | d1 | E01 |
| 2-1-m1E01-1-0878 | 777.208 | OH | a2 | B20 | b2 | C05 | c2 | D02 | d1 | E01 |
| 2-1-m1E01-1-0879 | 777.281 | OH | a1 | B09 | b1 | C29 | c3 | D02 | d1 | E01 |
| 2-1-m1E01-1-0880 | 778.199 | OH | a1 | B08 | b1 | C13 | c2 | D02 | d1 | E01 |
| 2-1-m1E01-1-0881 | 778.228 | OH | a2 | B20 | b2 | C26 | c3 | D02 | d1 | E01 |
| 2-1-m1E01-1-0882 | 778.265 | OH | a2 | B19 | b2 | C07 | c2 | D02 | d1 | E01 |
| 2-1-m1E01-1-0883 | 779.260 | OH | a1 | B10 | b1 | C32 | c3 | D02 | d1 | E01 |
| 2-1-m1E01-1-0884 | 780.134 | OH | a1 | B08 | b3 | C15 | c2 | D02 | d1 | E01 |
| 2-1-m1E01-1-0885 | 780.188 | OH | a2 | B20 | b2 | C04 | c2 | D02 | d1 | E01 |
| 2-1-m1E01-1-0886 | 780.204 | OH | a1 | B08 | b3 | C16 | c1 | D01 | d1 | E01 |
| 2-1-m1E01-1-0887 | 780.219 | OH | a1 | B09 | b2 | C05 | c2 | D02 | d1 | E01 |
| 2-1-m1E01-1-0888 | 781.203 | OH | a1 | B10 | b2 | C02 | c2 | D02 | d1 | E01 |
| 2-1-m1E01-1-0889 | 781.239 | OH | a1 | B10 | b2 | C22 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0890 | 781.239 | OH | a1 | B09 | b2 | C26 | c3 | D02 | d1 | E01 |
| 2-1-m1E01-1-0891 | 782.165 | OH | a1 | B08 | b3 | C17 | c1 | D01 | d1 | E01 |
| 2-1-m1E01-1-0892 | 782.165 | OH | a1 | B08 | b1 | C14 | c2 | D02 | d1 | E01 |
| 2-1-m1E01-1-0893 | 782.169 | OH | a2 | B19 | b3 | C08 | c2 | D02 | d1 | E01 |
| 2-1-m1E01-1-0894 | 782.198 | OH | a1 | B10 | b2 | C03 | c2 | D02 | d1 | E01 |
| 2-1-m1E01-1-0895 | 782.208 | OH | a2 | B13 | b3 | C16 | c2 | D02 | d1 | E01 |
| 2-1-m1E01-1-0896 | 783.199 | OH | a1 | B09 | b2 | C04 | c2 | D02 | d1 | E01 |
| 2-1-m1E01-1-0897 | 784.170 | OH | a2 | B13 | b3 | C17 | c2 | D02 | d1 | E01 |
| 2-1-m1E01-1-0898 | 785.165 | OH | a2 | B11 | b3 | C18 | c2 | D02 | d1 | E01 |
| 2-1-m1E01-1-0899 | 785.214 | OH | a1 | B10 | b2 | C24 | c3 | D02 | d1 | E01 |
| 2-1-m1E01-1-0900 | 786.160 | OH | a2 | B12 | b3 | C18 | c2 | D02 | d1 | E01 |
| 2-1-m1E01-1-0901 | 786.183 | OH | a2 | B14 | b3 | C16 | c2 | D02 | d1 | E01 |
| 2-1-m1E01-1-0902 | 786.195 | OH | a2 | B19 | b1 | C09 | c2 | D02 | d1 | E01 |
| 2-1-m1E01-1-0903 | 786.208 | OH | a2 | B20 | b1 | C13 | c2 | D02 | d1 | E01 |
| 2-1-m1E01-1-0904 | 786.251 | OH | a1 | B08 | b2 | C07 | c2 | D02 | d1 | E01 |
| 2-1-m1E01-1-0905 | 786.270 | OH | a2 | B19 | b1 | C31 | c3 | D02 | d1 | E01 |
| 2-1-m1E01-1-0906 | 787.159 | OH | a1 | B10 | b2 | C06 | c2 | D02 | d1 | E01 |
| 2-1-m1E01-1-0907 | 787.198 | OH | a1 | B01 | b3 | C16 | c2 | D02 | d1 | E01 |
| 2-1-m1E01-1-0908 | 788.126 | OH | a2 | B19 | b3 | C06 | c2 | D02 | d1 | E01 |
| 2-1-m1E01-1-0909 | 788.143 | OH | a2 | B20 | b3 | C15 | c2 | D02 | d1 | E01 |
| 2-1-m1E01-1-0910 | 788.144 | OH | a2 | B14 | b3 | C17 | c2 | D02 | d1 | E01 |
| 2-1-m1E01-1-0911 | 788.193 | OH | a1 | B02 | b3 | C16 | c2 | D02 | d1 | E01 |
| 2-1-m1E01-1-0912 | 788.213 | OH | a2 | B20 | b3 | C16 | c1 | D01 | d1 | E01 |
| 2-1-m1E01-1-0913 | 789.160 | OH | a1 | B01 | b3 | C17 | c2 | D02 | d1 | E01 |
| 2-1-m1E01-1-0914 | 789.190 | OH | a2 | B19 | b3 | C18 | c1 | D01 | d1 | E01 |
| 2-1-m1E01-1-0915 | 789.219 | OH | a1 | B09 | b1 | C13 | c2 | D02 | d1 | E01 |
| 2-1-m1E01-1-0916 | 790.155 | OH | a1 | B02 | b3 | C17 | c2 | D02 | d1 | E01 |
| 2-1-m1E01-1-0917 | 790.155 | OH | a1 | B08 | b3 | C08 | c2 | D02 | d1 | E01 |
| 2-1-m1E01-1-0918 | 790.174 | OH | a2 | B20 | b3 | C17 | c1 | D01 | d1 | E01 |
| 2-1-m1E01-1-0919 | 790.174 | OH | a2 | B20 | b1 | C14 | c2 | D02 | d1 | E01 |
| 2-1-m1E01-1-0920 | 791.154 | OH | a1 | B09 | b3 | C15 | c2 | D02 | d1 | E01 |
| 2-1-m1E01-1-0921 | 791.224 | OH | a1 | B09 | b3 | C16 | c1 | D01 | d1 | E01 |
| 2-1-m1E01-1-0922 | 793.185 | OH | a1 | B09 | b3 | C17 | c1 | D01 | d1 | E01 |
| 2-1-m1E01-1-0923 | 793.185 | OH | a1 | B09 | b1 | C14 | c2 | D02 | d1 | E01 |
| 2-1-m1E01-1-0924 | 793.276 | OH | a1 | B10 | b1 | C29 | c3 | D02 | d1 | E01 |
| 2-1-m1E01-1-0925 | 794.180 | OH | a1 | B08 | b1 | C09 | c2 | D02 | d1 | E01 |
| 2-1-m1E01-1-0926 | 794.256 | OH | a1 | B08 | b1 | C31 | c3 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0927 | 794.260 | OH | a2 | B20 | b2 | C07 | c2 | D02 | d1 | E01 |
| 2-1-m1E01-1-0928 | 796.111 | OH | a1 | B08 | b3 | C06 | c2 | D02 | d1 | E01 |
| 2-1-m1E01-1-0929 | 796.214 | OH | a1 | B10 | b2 | C05 | c2 | D02 | d1 | E01 |
| 2-1-m1E01-1-0930 | 796.224 | OH | a2 | B15 | b3 | C16 | c2 | D02 | d1 | E01 |
| 2-1-m1E01-1-0931 | 797.176 | OH | a1 | B08 | b3 | C18 | c1 | D01 | d1 | E01 |
| 2-1-m1E01-1-0932 | 797.234 | OH | a1 | B10 | b2 | C26 | c3 | D02 | d1 | E01 |
| 2-1-m1E01-1-0933 | 797.271 | OH | a1 | B09 | b2 | C07 | c2 | D02 | d1 | E01 |
| 2-1-m1E01-1-0934 | 798.164 | OH | a2 | B20 | b3 | C08 | c2 | D02 | d1 | E01 |
| 2-1-m1E01-1-0935 | 798.185 | OH | a2 | B15 | b3 | C17 | c2 | D02 | d1 | E01 |
| 2-1-m1E01-1-0936 | 798.203 | OH | a2 | B16 | b3 | C16 | c2 | D02 | d1 | E01 |
| 2-1-m1E01-1-0937 | 799.180 | OH | a2 | B13 | b3 | C18 | c2 | D02 | d1 | E01 |
| 2-1-m1E01-1-0938 | 799.194 | OH | a1 | B10 | b2 | C04 | c2 | D02 | d1 | E01 |
| 2-1-m1E01-1-0939 | 800.164 | OH | a2 | B16 | b3 | C17 | c2 | D02 | d1 | E01 |
| 2-1-m1E01-1-0940 | 801.175 | OH | a1 | B09 | b3 | C08 | c2 | D02 | d1 | E01 |
| 2-1-m1E01-1-0941 | 801.214 | OH | a1 | B03 | b3 | C16 | c2 | D02 | d1 | E01 |
| 2-1-m1E01-1-0942 | 802.190 | OH | a2 | B20 | b1 | C09 | c2 | D02 | d1 | E01 |
| 2-1-m1E01-1-0943 | 802.265 | OH | a2 | B20 | b1 | C31 | c3 | D02 | d1 | E01 |
| 2-1-m1E01-1-0944 | 803.155 | OH | a2 | B14 | b3 | C18 | c2 | D02 | d1 | E01 |
| 2-1-m1E01-1-0945 | 803.175 | OH | a1 | B03 | b3 | C17 | c2 | D02 | d1 | E01 |
| 2-1-m1E01-1-0946 | 804.121 | OH | a2 | B20 | b3 | C06 | c2 | D02 | d1 | E01 |
| 2-1-m1E01-1-0947 | 804.171 | OH | a1 | B01 | b3 | C18 | c2 | D02 | d1 | E01 |
| 2-1-m1E01-1-0948 | 804.174 | OH | a2 | B17 | b3 | C16 | c2 | D02 | d1 | E01 |
| 2-1-m1E01-1-0949 | 805.166 | OH | a1 | B02 | b3 | C18 | c2 | D02 | d1 | E01 |
| 2-1-m1E01-1-0950 | 805.185 | OH | a2 | B20 | b3 | C18 | c1 | D01 | d1 | E01 |
| 2-1-m1E01-1-0951 | 805.189 | OH | a1 | B04 | b3 | C16 | c2 | D02 | d1 | E01 |
| 2-1-m1E01-1-0952 | 805.200 | OH | a1 | B09 | b1 | C09 | c2 | D02 | d1 | E01 |
| 2-1-m1E01-1-0953 | 805.214 | OH | a1 | B10 | b1 | C13 | c2 | D02 | d1 | E01 |
| 2-1-m1E01-1-0954 | 805.276 | OH | a1 | B09 | b1 | C31 | c3 | D02 | d1 | E01 |
| 2-1-m1E01-1-0955 | 806.135 | OH | a2 | B17 | b3 | C17 | c2 | D02 | d1 | E01 |
| 2-1-m1E01-1-0956 | 807.131 | OH | a1 | B09 | b3 | C06 | c2 | D02 | d1 | E01 |
| 2-1-m1E01-1-0957 | 807.149 | OH | a1 | B10 | b3 | C15 | c2 | D02 | d1 | E01 |
| 2-1-m1E01-1-0958 | 807.150 | OH | a1 | B04 | b3 | C17 | c2 | D02 | d1 | E01 |
| 2-1-m1E01-1-0959 | 807.219 | OH | a1 | B10 | b3 | C16 | c1 | D01 | d1 | E01 |
| 2-1-m1E01-1-0960 | 808.196 | OH | a1 | B09 | b3 | C18 | c1 | D01 | d1 | E01 |
| 2-1-m1E01-1-0961 | 809.180 | OH | a1 | B10 | b3 | C17 | c1 | D01 | d1 | E01 |
| 2-1-m1E01-1-0962 | 809.180 | OH | a1 | B10 | b1 | C14 | c2 | D02 | d1 | E01 |
| 2-1-m1E01-1-0963 | 813.196 | OH | a2 | B15 | b3 | C18 | c2 | D02 | d1 | E01 |

(continued)

| [Table 8-8-2] Compound that may be contained in mixture 2-1-m1E01-1 | | | | | | | | | | |
|---|---|---|---|---|---|---|---|---|---|---|
| ID | Exact Mass | | a | B | b | C | c | D | d | E |
| 2-1-m1E01-1-0964 | 813.266 | OH | a1 | B10 | b2 | C07 | c2 | D02 | d1 | E01 |
| 2-1-m1E01-1-0965 | 815.175 | OH | a2 | B16 | b3 | C18 | c2 | D02 | d1 | E01 |
| 2-1-m1E01-1-0966 | 815.229 | OH | a1 | B05 | b3 | C16 | c2 | D02 | d1 | E01 |
| 2-1-m1E01-1-0967 | 817.170 | OH | a1 | B10 | b3 | C08 | c2 | D02 | d1 | E01 |
| 2-1-m1E01-1-0968 | 817.191 | OH | a1 | B05 | b3 | C17 | c2 | D02 | d1 | E01 |
| 2-1-m1E01-1-0969 | 817.209 | OH | a1 | B06 | b3 | C16 | c2 | D02 | d1 | E01 |
| 2-1-m1E01-1-0970 | 818.186 | OH | a1 | B03 | b3 | C18 | c2 | D02 | d1 | E01 |
| 2-1-m1E01-1-0971 | 818.208 | OH | a2 | B18 | b3 | C16 | c2 | D02 | d1 | E01 |
| 2-1-m1E01-1-0972 | 819.170 | OH | a1 | B06 | b3 | C17 | c2 | D02 | d1 | E01 |
| 2-1-m1E01-1-0973 | 820.170 | OH | a2 | B18 | b3 | C17 | c2 | D02 | d1 | E01 |
| 2-1-m1E01-1-0974 | 821.146 | OH | a2 | B17 | b3 | C18 | c2 | D02 | d1 | E01 |
| 2-1-m1E01-1-0975 | 821.195 | OH | a1 | B10 | b1 | C09 | c2 | D02 | d1 | E01 |
| 2-1-m1E01-1-0976 | 821.271 | OH | a1 | B10 | b1 | C31 | c3 | D02 | d1 | E01 |
| 2-1-m1E01-1-0977 | 822.161 | OH | a1 | B04 | b3 | C18 | c2 | D02 | d1 | E01 |
| 2-1-m1E01-1-0978 | 823.126 | OH | a1 | B10 | b3 | C06 | c2 | D02 | d1 | E01 |
| 2-1-m1E01-1-0979 | 823.179 | OH | a1 | B07 | b3 | C16 | c2 | D02 | d1 | E01 |
| 2-1-m1E01-1-0980 | 824.191 | OH | a1 | B10 | b3 | C18 | c1 | D01 | d1 | E01 |
| 2-1-m1E01-1-0981 | 825.141 | OH | a1 | B07 | b3 | C17 | c2 | D02 | d1 | E01 |
| 2-1-m1E01-1-0982 | 832.202 | OH | a1 | B05 | b3 | C18 | c2 | D02 | d1 | E01 |
| 2-1-m1E01-1-0983 | 834.181 | OH | a1 | B06 | b3 | C18 | c2 | D02 | d1 | E01 |
| 2-1-m1E01-1-0984 | 835.180 | OH | a2 | B18 | b3 | C18 | c2 | D02 | d1 | E01 |
| 2-1-m1E01-1-0985 | 836.180 | OH | a2 | B19 | b3 | C16 | c2 | D02 | d1 | E01 |
| 2-1-m1E01-1-0986 | 838.141 | OH | a2 | B19 | b3 | C17 | c2 | D02 | d1 | E01 |
| 2-1-m1E01-1-0987 | 840.152 | OH | a1 | B07 | b3 | C18 | c2 | D02 | d1 | E01 |
| 2-1-m1E01-1-0988 | 844.166 | OH | a1 | B08 | b3 | C16 | c2 | D02 | d1 | E01 |
| 2-1-m1E01-1-0989 | 846.127 | OH | a1 | B08 | b3 | C17 | c2 | D02 | d1 | E01 |
| 2-1-m1E01-1-0990 | 852.175 | OH | a2 | B20 | b3 | C16 | c2 | D02 | d1 | E01 |
| 2-1-m1E01-1-0991 | 853.152 | OH | a2 | B19 | b3 | C18 | c2 | D02 | d1 | E01 |
| 2-1-m1E01-1-0992 | 854.136 | OH | a2 | B20 | b3 | C17 | c2 | D02 | d1 | E01 |
| 2-1-m1E01-1-0993 | 855.186 | OH | a1 | B09 | b3 | C16 | c2 | D02 | d1 | E01 |
| 2-1-m1E01-1-0994 | 857.147 | OH | a1 | B09 | b3 | C17 | c2 | D02 | d1 | E01 |
| 2-1-m1E01-1-0995 | 861.138 | OH | a1 | B08 | b3 | C18 | c2 | D02 | d1 | E01 |
| 2-1-m1E01-1-0996 | 869.147 | OH | a2 | B20 | b3 | C18 | c2 | D02 | d1 | E01 |
| 2-1-m1E01-1-0997 | 871.180 | OH | a1 | B10 | b3 | C16 | c2 | D02 | d1 | E01 |
| 2-1-m1E01-1-0998 | 872.158 | OH | a1 | B09 | b3 | C18 | c2 | D02 | d1 | E01 |
| 2-1-m1E01-1-0999 | 873.142 | OH | a1 | B10 | b3 | C17 | c2 | D02 | d1 | E01 |
| 2-1-m1E01-1-1000 | 888.153 | OH | a1 | B10 | b3 | C18 | c2 | D02 | d1 | E01 |

[Table 395]

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0001 | 602.253 | OH | a2 | B11 | b1 | C08 | c1 | D01 | d1 | E02 |
| 2-1-m1E02-1-0002 | 603.248 | OH | a2 | B12 | b1 | C08 | c1 | D01 | d1 | E02 |
| 2-1-m1E02-1-0003 | 603.248 | OH | a2 | B11 | b1 | C11 | c1 | D01 | d1 | E02 |
| 2-1-m1E02-1-0004 | 604.243 | OH | a2 | B11 | b1 | C10 | c1 | D01 | d1 | E02 |
| 2-1-m1E02-1-0005 | 604.243 | OH | a2 | B12 | b1 | C11 | c1 | D01 | d1 | E02 |
| 2-1-m1E02-1-0006 | 605.239 | OH | a2 | B12 | b1 | C10 | c1 | D01 | d1 | E02 |
| 2-1-m1E02-1-0007 | 609.205 | OH | a2 | B11 | b1 | C12 | c1 | D01 | d1 | E02 |
| 2-1-m1E02-1-0008 | 610.200 | OH | a2 | B12 | b1 | C12 | c1 | D01 | d1 | E02 |
| 2-1-m1E02-1-0009 | 616.269 | OH | a2 | B13 | b1 | C08 | c1 | D01 | d1 | E02 |
| 2-1-m1E02-1-0010 | 617.264 | OH | a2 | B13 | b1 | C11 | c1 | D01 | d1 | E02 |
| 2-1-m1E02-1-0011 | 618.259 | OH | a2 | B13 | b1 | C10 | c1 | D01 | d1 | E02 |
| 2-1-m1E02-1-0012 | 620.227 | OH | a2 | B11 | b2 | C01 | c1 | D01 | d1 | E02 |
| 2-1-m1E02-1-0013 | 620.243 | OH | a2 | B14 | b1 | C08 | c1 | D01 | d1 | E02 |
| 2-1-m1E02-1-0014 | 621.222 | OH | a2 | B12 | b2 | C01 | c1 | D01 | d1 | E02 |
| 2-1-m1E02-1-0015 | 621.239 | OH | a2 | B14 | b1 | C11 | c1 | D01 | d1 | E02 |
| 2-1-m1E02-1-0016 | 621.259 | OH | a1 | B01 | b1 | C08 | c1 | D01 | d1 | E02 |
| 2-1-m1E02-1-0017 | 622.234 | OH | a2 | B14 | b1 | C10 | c1 | D01 | d1 | E02 |
| 2-1-m1E02-1-0018 | 622.254 | OH | a1 | B02 | b1 | C08 | c1 | D01 | d1 | E02 |
| 2-1-m1E02-1-0019 | 622.254 | OH | a1 | B01 | b1 | C11 | c1 | D01 | d1 | E02 |
| 2-1-m1E02-1-0020 | 623.220 | OH | a2 | B13 | b1 | C12 | c1 | D01 | d1 | E02 |
| 2-1-m1E02-1-0021 | 623.249 | OH | a1 | B01 | b1 | C10 | c1 | D01 | d1 | E02 |
| 2-1-m1E02-1-0022 | 623.249 | OH | a1 | B02 | b1 | C11 | c1 | D01 | d1 | E02 |
| 2-1-m1E02-1-0023 | 624.244 | OH | a1 | B02 | b1 | C10 | c1 | D01 | d1 | E02 |
| 2-1-m1E02-1-0024 | 627.195 | OH | a2 | B14 | b1 | C12 | c1 | D01 | d1 | E02 |
| 2-1-m1E02-1-0025 | 628.210 | OH | a1 | B01 | b1 | C12 | c1 | D01 | d1 | E02 |
| 2-1-m1E02-1-0026 | 629.206 | OH | a1 | B02 | b1 | C12 | c1 | D01 | d1 | E02 |
| 2-1-m1E02-1-0027 | 630.248 | OH | a2 | B11 | b2 | C02 | c1 | D01 | d1 | E02 |
| 2-1-m1E02-1-0028 | 630.284 | OH | a2 | B15 | b1 | C08 | c1 | D01 | d1 | E02 |
| 2-1-m1E02-1-0029 | 631.243 | OH | a2 | B12 | b2 | C02 | c1 | D01 | d1 | E02 |
| 2-1-m1E02-1-0030 | 631.243 | OH | a2 | B11 | b2 | C03 | c1 | D01 | d1 | E02 |
| 2-1-m1E02-1-0031 | 631.279 | OH | a2 | B15 | b1 | C11 | c1 | D01 | d1 | E02 |
| 2-1-m1E02-1-0032 | 632.238 | OH | a2 | B12 | b2 | C03 | c1 | D01 | d1 | E02 |
| 2-1-m1E02-1-0033 | 632.263 | OH | a2 | B16 | b1 | C08 | c1 | D01 | d1 | E02 |
| 2-1-m1E02-1-0034 | 632.275 | OH | a2 | B15 | b1 | C10 | c1 | D01 | d1 | E02 |
| 2-1-m1E02-1-0035 | 633.259 | OH | a2 | B16 | b1 | C11 | c1 | D01 | d1 | E02 |
| 2-1-m1E02-1-0036 | 634.243 | OH | a2 | B13 | b2 | C01 | c1 | D01 | d1 | E02 |
| 2-1-m1E02-1-0037 | 634.254 | OH | a2 | B16 | b1 | C10 | c1 | D01 | d1 | E02 |
| 2-1-m1E02-1-0038 | 635.274 | OH | a1 | B03 | b1 | C08 | c1 | D01 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0039 | 636.204 | OH | a2 | B11 | b2 | C06 | c1 | D01 | d1 | E02 |
| 2-1-m1E02-1-0040 | 636.270 | OH | a1 | B03 | b1 | C11 | c1 | D01 | d1 | E02 |
| 2-1-m1E02-1-0041 | 637.200 | OH | a2 | B12 | b2 | C06 | c1 | D01 | d1 | E02 |
| 2-1-m1E02-1-0042 | 637.236 | OH | a2 | B15 | b1 | C12 | c1 | D01 | d1 | E02 |
| 2-1-m1E02-1-0043 | 637.265 | OH | a1 | B03 | b1 | C10 | c1 | D01 | d1 | E02 |
| 2-1-m1E02-1-0044 | 638.218 | OH | a2 | B14 | b2 | C01 | c1 | D01 | d1 | E02 |
| 2-1-m1E02-1-0045 | 638.234 | OH | a2 | B17 | b1 | C08 | c1 | D01 | d1 | E02 |
| 2-1-m1E02-1-0046 | 639.215 | OH | a2 | B16 | b1 | C12 | c1 | D01 | d1 | E02 |
| 2-1-m1E02-1-0047 | 639.229 | OH | a2 | B17 | b1 | C11 | c1 | D01 | d1 | E02 |
| 2-1-m1E02-1-0048 | 639.233 | OH | a1 | B01 | b2 | C01 | c1 | D01 | d1 | E02 |
| 2-1-m1E02-1-0049 | 639.249 | OH | a1 | B04 | b1 | C08 | c1 | D01 | d1 | E02 |
| 2-1-m1E02-1-0050 | 640.225 | OH | a2 | B17 | b1 | C10 | c1 | D01 | d1 | E02 |
| 2-1-m1E02-1-0051 | 640.228 | OH | a1 | B02 | b2 | C01 | c1 | D01 | d1 | E02 |
| 2-1-m1E02-1-0052 | 640.245 | OH | a1 | B04 | b1 | C11 | c1 | D01 | d1 | E02 |
| 2-1-m1E02-1-0053 | 641.240 | OH | a1 | B04 | b1 | C10 | c1 | D01 | d1 | E02 |
| 2-1-m1E02-1-0054 | 642.226 | OH | a1 | B03 | b1 | C12 | c1 | D01 | d1 | E02 |
| 2-1-m1E02-1-0055 | 644.263 | OH | a2 | B13 | b2 | C02 | c1 | D01 | d1 | E02 |
| 2-1-m1E02-1-0056 | 645.186 | OH | a2 | B17 | b1 | C12 | c1 | D01 | d1 | E02 |
| 2-1-m1E02-1-0057 | 645.259 | OH | a2 | B13 | b2 | C03 | c1 | D01 | d1 | E02 |
| 2-1-m1E02-1-0058 | 645.259 | OH | a2 | B11 | b2 | C05 | c1 | D01 | d1 | E02 |
| 2-1-m1E02-1-0059 | 646.201 | OH | a1 | B04 | b1 | C12 | c1 | D01 | d1 | E02 |
| 2-1-m1E02-1-0060 | 646.254 | OH | a2 | B12 | b2 | C05 | c1 | D01 | d1 | E02 |
| 2-1-m1E02-1-0061 | 648.238 | OH | a2 | B14 | b2 | C02 | c1 | D01 | d1 | E02 |
| 2-1-m1E02-1-0062 | 648.238 | OH | a2 | B11 | b2 | C04 | c1 | D01 | d1 | E02 |
| 2-1-m1E02-1-0063 | 648.258 | OH | a2 | B15 | b2 | C01 | c1 | D01 | d1 | E02 |
| 2-1-m1E02-1-0064 | 649.234 | OH | a2 | B12 | b2 | C04 | c1 | D01 | d1 | E02 |
| 2-1-m1E02-1-0065 | 649.234 | OH | a2 | B14 | b2 | C03 | c1 | D01 | d1 | E02 |
| 2-1-m1E02-1-0066 | 649.254 | OH | a1 | B01 | b2 | C02 | c1 | D01 | d1 | E02 |
| 2-1-m1E02-1-0067 | 649.290 | OH | a1 | B05 | b1 | C08 | c1 | D01 | d1 | E02 |
| 2-1-m1E02-1-0068 | 650.220 | OH | a2 | B13 | b2 | C06 | c1 | D01 | d1 | E02 |
| 2-1-m1E02-1-0069 | 650.238 | OH | a2 | B16 | b2 | C01 | c1 | D01 | d1 | E02 |
| 2-1-m1E02-1-0070 | 650.249 | OH | a1 | B02 | b2 | C02 | c1 | D01 | d1 | E02 |
| 2-1-m1E02-1-0071 | 650.249 | OH | a1 | B01 | b2 | C03 | c1 | D01 | d1 | E02 |
| 2-1-m1E02-1-0072 | 650.285 | OH | a1 | B05 | b1 | C11 | c1 | D01 | d1 | E02 |
| 2-1-m1E02-1-0073 | 651.244 | OH | a1 | B02 | b2 | C03 | c1 | D01 | d1 | E02 |
| 2-1-m1E02-1-0074 | 651.269 | OH | a1 | B06 | b1 | C08 | c1 | D01 | d1 | E02 |
| 2-1-m1E02-1-0075 | 651.281 | OH | a1 | B05 | b1 | C10 | c1 | D01 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0076 | 652.265 | OH | a1 | B06 | b1 | C11 | c1 | D01 | d1 | E02 |
| 2-1-m1E02-1-0077 | 652.269 | OH | a2 | B18 | b1 | C08 | c1 | D01 | d1 | E02 |
| 2-1-m1E02-1-0078 | 653.249 | OH | a1 | B03 | b2 | C01 | c1 | D01 | d1 | E02 |
| 2-1-m1E02-1-0079 | 653.260 | OH | a1 | B06 | b1 | C10 | c1 | D01 | d1 | E02 |
| 2-1-m1E02-1-0080 | 653.264 | OH | a2 | B18 | b1 | C11 | c1 | D01 | d1 | E02 |
| 2-1-m1E02-1-0081 | 654.195 | OH | a2 | B14 | b2 | C06 | c1 | D01 | d1 | E02 |
| 2-1-m1E02-1-0082 | 654.259 | OH | a2 | B18 | b1 | C10 | c1 | D01 | d1 | E02 |
| 2-1-m1E02-1-0083 | 654.259 | OH | a2 | B11 | b1 | C13 | c1 | D01 | d1 | E02 |
| 2-1-m1E02-1-0084 | 655.210 | OH | a1 | B01 | b2 | C06 | c1 | D01 | d1 | E02 |
| 2-1-m1E02-1-0085 | 655.254 | OH | a2 | B12 | b1 | C13 | c1 | D01 | d1 | E02 |
| 2-1-m1E02-1-0086 | 656.194 | OH | a2 | B11 | b3 | C15 | c1 | D01 | d1 | E02 |
| 2-1-m1E02-1-0087 | 656.205 | OH | a1 | B02 | b2 | C06 | c1 | D01 | d1 | E02 |
| 2-1-m1E02-1-0088 | 656.208 | OH | a2 | B17 | b2 | C01 | c1 | D01 | d1 | E02 |
| 2-1-m1E02-1-0089 | 656.242 | OH | a1 | B05 | b1 | C12 | c1 | D01 | d1 | E02 |
| 2-1-m1E02-1-0090 | 657.189 | OH | a2 | B12 | b3 | C15 | c1 | D01 | d1 | E02 |
| 2-1-m1E02-1-0091 | 657.223 | OH | a1 | B04 | b2 | C01 | c1 | D01 | d1 | E02 |
| 2-1-m1E02-1-0092 | 657.240 | OH | a1 | B07 | b1 | C08 | c1 | D01 | d1 | E02 |
| 2-1-m1E02-1-0093 | 658.221 | OH | a1 | B06 | b1 | C12 | c1 | D01 | d1 | E02 |
| 2-1-m1E02-1-0094 | 658.225 | OH | a2 | B11 | b1 | C14 | c1 | D01 | d1 | E02 |
| 2-1-m1E02-1-0095 | 658.235 | OH | a1 | B07 | b1 | C11 | c1 | D01 | d1 | E02 |
| 2-1-m1E02-1-0096 | 658.246 | OH | a2 | B11 | b2 | C33 | c3 | D02 | d1 | E02 |
| 2-1-m1E02-1-0097 | 658.279 | OH | a2 | B15 | b2 | C02 | c1 | D01 | d1 | E02 |
| 2-1-m1E02-1-0098 | 659.220 | OH | a2 | B18 | b1 | C12 | c1 | D01 | d1 | E02 |
| 2-1-m1E02-1-0099 | 659.220 | OH | a2 | B12 | b1 | C14 | c1 | D01 | d1 | E02 |
| 2-1-m1E02-1-0100 | 659.230 | OH | a1 | B07 | b1 | C10 | c1 | D01 | d1 | E02 |
| 2-1-m1E02-1-0101 | 659.241 | OH | a2 | B12 | b2 | C33 | c3 | D02 | d1 | E02 |
| 2-1-m1E02-1-0102 | 659.274 | OH | a2 | B15 | b2 | C03 | c1 | D01 | d1 | E02 |
| 2-1-m1E02-1-0103 | 659.274 | OH | a2 | B13 | b2 | C05 | c1 | D01 | d1 | E02 |
| 2-1-m1E02-1-0104 | 660.258 | OH | a2 | B16 | b2 | C02 | c1 | D01 | d1 | E02 |
| 2-1-m1E02-1-0105 | 661.254 | OH | a2 | B16 | b2 | C03 | c1 | D01 | d1 | E02 |
| 2-1-m1E02-1-0106 | 662.254 | OH | a2 | B13 | b2 | C04 | c1 | D01 | d1 | E02 |
| 2-1-m1E02-1-0107 | 662.310 | OH | a2 | B11 | b2 | C07 | c1 | D01 | d1 | E02 |
| 2-1-m1E02-1-0108 | 663.249 | OH | a2 | B14 | b2 | C05 | c1 | D01 | d1 | E02 |
| 2-1-m1E02-1-0109 | 663.269 | OH | a1 | B03 | b2 | C02 | c1 | D01 | d1 | E02 |
| 2-1-m1E02-1-0110 | 663.306 | OH | a2 | B12 | b2 | C07 | c1 | D01 | d1 | E02 |
| 2-1-m1E02-1-0111 | 664.192 | OH | a1 | B07 | b1 | C12 | c1 | D01 | d1 | E02 |
| 2-1-m1E02-1-0112 | 664.236 | OH | a2 | B15 | b2 | C06 | c1 | D01 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0113 | 664.265 | OH | a1 | B03 | b2 | C03 | c1 | D01 | d1 | E02 |
| 2-1-m1E02-1-0114 | 664.265 | OH | a1 | B01 | b2 | C05 | c1 | D01 | d1 | E02 |
| 2-1-m1E02-1-0115 | 665.260 | OH | a1 | B02 | b2 | C05 | c1 | D01 | d1 | E02 |
| 2-1-m1E02-1-0116 | 666.215 | OH | a2 | B16 | b2 | C06 | c1 | D01 | d1 | E02 |
| 2-1-m1E02-1-0117 | 666.215 | OH | a2 | B11 | b3 | C08 | c1 | D01 | d1 | E02 |
| 2-1-m1E02-1-0118 | 666.215 | OH | a2 | B11 | b1 | C08 | c2 | D02 | d1 | E02 |
| 2-1-m1E02-1-0119 | 666.229 | OH | a2 | B17 | b2 | C02 | c1 | D01 | d1 | E02 |
| 2-1-m1E02-1-0120 | 666.229 | OH | a2 | B14 | b2 | C04 | c1 | D01 | d1 | E02 |
| 2-1-m1E02-1-0121 | 667.210 | OH | a2 | B12 | b3 | C08 | c1 | D01 | d1 | E02 |
| 2-1-m1E02-1-0122 | 667.210 | OH | a2 | B12 | b1 | C08 | c2 | D02 | d1 | E02 |
| 2-1-m1E02-1-0123 | 667.210 | OH | a2 | B11 | b1 | C11 | c2 | D02 | d1 | E02 |
| 2-1-m1E02-1-0124 | 667.224 | OH | a2 | B17 | b2 | C03 | c1 | D01 | d1 | E02 |
| 2-1-m1E02-1-0125 | 667.244 | OH | a1 | B04 | b2 | C02 | c1 | D01 | d1 | E02 |
| 2-1-m1E02-1-0126 | 667.244 | OH | a1 | B01 | b2 | C04 | c1 | D01 | d1 | E02 |
| 2-1-m1E02-1-0127 | 667.264 | OH | a1 | B05 | b2 | C01 | c1 | D01 | d1 | E02 |
| 2-1-m1E02-1-0128 | 668.205 | OH | a2 | B11 | b1 | C10 | c2 | D02 | d1 | E02 |
| 2-1-m1E02-1-0129 | 668.205 | OH | a2 | B12 | b1 | C11 | c2 | D02 | d1 | E02 |
| 2-1-m1E02-1-0130 | 668.239 | OH | a1 | B02 | b2 | C04 | c1 | D01 | d1 | E02 |
| 2-1-m1E02-1-0131 | 668.239 | OH | a1 | B04 | b2 | C03 | c1 | D01 | d1 | E02 |
| 2-1-m1E02-1-0132 | 668.275 | OH | a2 | B13 | b1 | C13 | c1 | D01 | d1 | E02 |
| 2-1-m1E02-1-0133 | 669.201 | OH | a2 | B12 | b1 | C10 | c2 | D02 | d1 | E02 |
| 2-1-m1E02-1-0134 | 669.226 | OH | a1 | B03 | b2 | C06 | c1 | D01 | d1 | E02 |
| 2-1-m1E02-1-0135 | 669.243 | OH | a1 | B06 | b2 | C01 | c1 | D01 | d1 | E02 |
| 2-1-m1E02-1-0136 | 670.210 | OH | a2 | B13 | b3 | C15 | c1 | D01 | d1 | E02 |
| 2-1-m1E02-1-0137 | 670.240 | OH | a2 | B19 | b1 | C08 | c1 | D01 | d1 | E02 |
| 2-1-m1E02-1-0138 | 670.240 | OH | a2 | B11 | b1 | C09 | c1 | D01 | d1 | E02 |
| 2-1-m1E02-1-0139 | 670.243 | OH | a2 | B18 | b2 | C01 | c1 | D01 | d1 | E02 |
| 2-1-m1E02-1-0140 | 671.236 | OH | a2 | B12 | b1 | C09 | c1 | D01 | d1 | E02 |
| 2-1-m1E02-1-0141 | 671.236 | OH | a2 | B19 | b1 | C11 | c1 | D01 | d1 | E02 |
| 2-1-m1E02-1-0142 | 672.171 | OH | a2 | B11 | b3 | C06 | c1 | D01 | d1 | E02 |
| 2-1-m1E02-1-0143 | 672.185 | OH | a2 | B17 | b2 | C06 | c1 | D01 | d1 | E02 |
| 2-1-m1E02-1-0144 | 672.231 | OH | a2 | B19 | b1 | C10 | c1 | D01 | d1 | E02 |
| 2-1-m1E02-1-0145 | 672.241 | OH | a2 | B13 | b1 | C14 | c1 | D01 | d1 | E02 |
| 2-1-m1E02-1-0146 | 672.250 | OH | a2 | B14 | b1 | C13 | c1 | D01 | d1 | E02 |
| 2-1-m1E02-1-0147 | 672.262 | OH | a2 | B13 | b2 | C33 | c3 | D02 | d1 | E02 |
| 2-1-m1E02-1-0148 | 673.166 | OH | a2 | B12 | b3 | C06 | c1 | D01 | d1 | E02 |
| 2-1-m1E02-1-0149 | 673.166 | OH | a2 | B11 | b1 | C12 | c2 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0150 | 673.201 | OH | a1 | B04 | b2 | C06 | c1 | D01 | d1 | E02 |
| 2-1-m1E02-1-0151 | 673.265 | OH | a1 | B01 | b1 | C13 | c1 | D01 | d1 | E02 |
| 2-1-m1E02-1-0152 | 673.290 | OH | a2 | B15 | b2 | COS | c1 | D01 | d1 | E02 |
| 2-1-m1E02-1-0153 | 674.162 | OH | a2 | B12 | b1 | C12 | c2 | D02 | d1 | E02 |
| 2-1-m1E02-1-0154 | 674.185 | OH | a2 | B14 | b3 | C15 | c1 | D01 | d1 | E02 |
| 2-1-m1E02-1-0155 | 674.260 | OH | a1 | B02 | b1 | C13 | c1 | D01 | d1 | E02 |
| 2-1-m1E02-1-0156 | 675.200 | OH | a1 | B01 | b3 | C15 | c1 | D01 | d1 | E02 |
| 2-1-m1E02-1-0157 | 675.214 | OH | a1 | B07 | b2 | C01 | c1 | D01 | d1 | E02 |
| 2-1-m1E02-1-0158 | 675.269 | OH | a2 | B16 | b2 | COS | c1 | D01 | d1 | E02 |
| 2-1-m1E02-1-0159 | 676.195 | OH | a1 | B02 | b3 | C15 | c1 | D01 | d1 | E02 |
| 2-1-m1E02-1-0160 | 676.216 | OH | a2 | B14 | b1 | C14 | c1 | D01 | d1 | E02 |
| 2-1-m1E02-1-0161 | 676.237 | OH | a2 | B14 | b2 | C33 | c3 | D02 | d1 | E02 |
| 2-1-m1E02-1-0162 | 676.270 | OH | a2 | B15 | b2 | C04 | c1 | D01 | d1 | E02 |
| 2-1-m1E02-1-0163 | 676.326 | OH | a2 | B13 | b2 | C07 | c1 | D01 | d1 | E02 |
| 2-1-m1E02-1-0164 | 677.192 | OH | a2 | B19 | b1 | C12 | c1 | D01 | d1 | E02 |
| 2-1-m1E02-1-0165 | 677.231 | OH | a1 | B01 | b1 | C14 | c1 | D01 | d1 | E02 |
| 2-1-m1E02-1-0166 | 677.252 | OH | a1 | B01 | b2 | C33 | c3 | D02 | d1 | E02 |
| 2-1-m1E02-1-0167 | 677.285 | OH | a1 | B05 | b2 | C02 | c1 | D01 | d1 | E02 |
| 2-1-m1E02-1-0168 | 678.226 | OH | a1 | B08 | b1 | C08 | c1 | D01 | d1 | E02 |
| 2-1-m1E02-1-0169 | 678.226 | OH | a1 | B02 | b1 | C14 | c1 | D01 | d1 | E02 |
| 2-1-m1E02-1-0170 | 678.247 | OH | a1 | B02 | b2 | C33 | c3 | D02 | d1 | E02 |
| 2-1-m1E02-1-0171 | 678.249 | OH | a2 | B16 | b2 | C04 | c1 | D01 | d1 | E02 |
| 2-1-m1E02-1-0172 | 678.251 | OH | a2 | B11 | b1 | C30 | c3 | D02 | d1 | E02 |
| 2-1-m1E02-1-0173 | 678.280 | OH | a1 | B05 | b2 | C03 | c1 | D01 | d1 | E02 |
| 2-1-m1E02-1-0174 | 678.280 | OH | a1 | B03 | b2 | COS | c1 | D01 | d1 | E02 |
| 2-1-m1E02-1-0175 | 679.221 | OH | a1 | B08 | b1 | C11 | c1 | D01 | d1 | E02 |
| 2-1-m1E02-1-0176 | 679.246 | OH | a2 | B12 | b1 | C30 | c3 | D02 | d1 | E02 |
| 2-1-m1E02-1-0177 | 679.264 | OH | a1 | B06 | b2 | C02 | c1 | D01 | d1 | E02 |
| 2-1-m1E02-1-0178 | 680.217 | OH | a1 | B08 | b1 | C10 | c1 | D01 | d1 | E02 |
| 2-1-m1E02-1-0179 | 680.230 | OH | a2 | B13 | b3 | C08 | c1 | D01 | d1 | E02 |
| 2-1-m1E02-1-0180 | 680.230 | OH | a2 | B13 | b1 | C08 | c2 | D02 | d1 | E02 |
| 2-1-m1E02-1-0181 | 680.230 | OH | a2 | B11 | b2 | C20 | c3 | D02 | d1 | E02 |
| 2-1-m1E02-1-0182 | 680.259 | OH | a1 | B06 | b2 | C03 | c1 | D01 | d1 | E02 |
| 2-1-m1E02-1-0183 | 680.263 | OH | a2 | B18 | b2 | C02 | c1 | D01 | d1 | E02 |
| 2-1-m1E02-1-0184 | 680.301 | OH | a2 | B14 | b2 | C07 | c1 | D01 | d1 | E02 |
| 2-1-m1E02-1-0185 | 681.226 | OH | a2 | B13 | b1 | C11 | c2 | D02 | d1 | E02 |
| 2-1-m1E02-1-0186 | 681.226 | OH | a2 | B12 | b2 | C20 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0187 | 681.226 | OH | a2 | B11 | b2 | C28 | c3 | D02 | d1 | E02 |
| 2-1-m1E02-1-0188 | 681.240 | OH | a2 | B17 | b2 | COS | c1 | D01 | d1 | E02 |
| 2-1-m1E02-1-0189 | 681.259 | OH | a2 | B18 | b2 | C03 | c1 | D01 | d1 | E02 |
| 2-1-m1E02-1-0190 | 681.260 | OH | a1 | B03 | b2 | C04 | c1 | D01 | d1 | E02 |
| 2-1-m1E02-1-0191 | 681.316 | OH | a1 | B01 | b2 | C07 | c1 | D01 | d1 | E02 |
| 2-1-m1E02-1-0192 | 682.221 | OH | a2 | B13 | b1 | C10 | c2 | D02 | d1 | E02 |
| 2-1-m1E02-1-0193 | 682.221 | OH | a2 | B12 | b2 | C28 | c3 | D02 | d1 | E02 |
| 2-1-m1E02-1-0194 | 682.255 | OH | a1 | B04 | b2 | COS | c1 | D01 | d1 | E02 |
| 2-1-m1E02-1-0195 | 682.290 | OH | a2 | B15 | b1 | C13 | c1 | D01 | d1 | E02 |
| 2-1-m1E02-1-0196 | 682.311 | OH | a1 | B02 | b2 | C07 | c1 | D01 | d1 | E02 |
| 2-1-m1E02-1-0197 | 683.241 | OH | a1 | B05 | b2 | C06 | c1 | D01 | d1 | E02 |
| 2-1-m1E02-1-0198 | 684.189 | OH | a2 | B11 | b2 | C01 | c2 | D02 | d1 | E02 |
| 2-1-m1E02-1-0199 | 684.205 | OH | a2 | B14 | b3 | C08 | c1 | D01 | d1 | E02 |
| 2-1-m1E02-1-0200 | 684.205 | OH | a2 | B14 | b1 | C08 | c2 | D02 | d1 | E02 |
| 2-1-m1E02-1-0201 | 684.220 | OH | a2 | B17 | b2 | C04 | c1 | D01 | d1 | E02 |
| 2-1-m1E02-1-0202 | 684.225 | OH | a2 | B15 | b3 | C15 | c1 | D01 | d1 | E02 |
| 2-1-m1E02-1-0203 | 684.256 | OH | a2 | B13 | b1 | C09 | c1 | D01 | d1 | E02 |
| 2-1-m1E02-1-0204 | 684.270 | OH | a2 | B16 | b1 | C13 | c1 | D01 | d1 | E02 |
| 2-1-m1E02-1-0205 | 685.178 | OH | a1 | B08 | b1 | C12 | c1 | D01 | d1 | E02 |
| 2-1-m1E02-1-0206 | 685.184 | OH | a2 | B12 | b2 | C01 | c2 | D02 | d1 | E02 |
| 2-1-m1E02-1-0207 | 685.201 | OH | a2 | B14 | b1 | C11 | c2 | D02 | d1 | E02 |
| 2-1-m1E02-1-0208 | 685.221 | OH | a1 | B06 | b2 | C06 | c1 | D01 | d1 | E02 |
| 2-1-m1E02-1-0209 | 685.221 | OH | a1 | B01 | b3 | C08 | c1 | D01 | d1 | E02 |
| 2-1-m1E02-1-0210 | 685.221 | OH | a1 | B01 | b1 | C08 | c2 | D02 | d1 | E02 |
| 2-1-m1E02-1-0211 | 685.235 | OH | a1 | B07 | b2 | C02 | c1 | D01 | d1 | E02 |
| 2-1-m1E02-1-0212 | 685.235 | OH | a1 | B04 | b2 | C04 | c1 | D01 | d1 | E02 |
| 2-1-m1E02-1-0213 | 686.187 | OH | a2 | B13 | b3 | C06 | c1 | D01 | d1 | E02 |
| 2-1-m1E02-1-0214 | 686.196 | OH | a2 | B14 | b1 | C10 | c2 | D02 | d1 | E02 |
| 2-1-m1E02-1-0215 | 686.205 | OH | a2 | B16 | b3 | C15 | c1 | D01 | d1 | E02 |
| 2-1-m1E02-1-0216 | 686.216 | OH | a1 | B02 | b3 | C08 | c1 | D01 | d1 | E02 |
| 2-1-m1E02-1-0217 | 686.216 | OH | a1 | B02 | b1 | C08 | c2 | D02 | d1 | E02 |
| 2-1-m1E02-1-0218 | 686.216 | OH | a1 | B01 | b1 | C11 | c2 | D02 | d1 | E02 |
| 2-1-m1E02-1-0219 | 686.220 | OH | a2 | B18 | b2 | C06 | c1 | D01 | d1 | E02 |
| 2-1-m1E02-1-0220 | 686.230 | OH | a1 | B07 | b2 | C03 | c1 | D01 | d1 | E02 |
| 2-1-m1E02-1-0221 | 686.235 | OH | a2 | B20 | b1 | C08 | c1 | D01 | d1 | E02 |
| 2-1-m1E02-1-0222 | 686.256 | OH | a2 | B15 | b1 | C14 | c1 | D01 | d1 | E02 |
| 2-1-m1E02-1-0223 | 686.277 | OH | a2 | B15 | b2 | C33 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0224 | 687.182 | OH | a2 | B13 | b1 | C12 | c2 | D02 | d1 | E02 |
| 2-1-m1E02-1-0225 | 687.211 | OH | a1 | B01 | b1 | C10 | c2 | D02 | d1 | E02 |
| 2-1-m1E02-1-0226 | 687.211 | OH | a1 | B02 | b1 | C11 | c2 | D02 | d1 | E02 |
| 2-1-m1E02-1-0227 | 687.230 | OH | a2 | B20 | b1 | C11 | c1 | D01 | d1 | E02 |
| 2-1-m1E02-1-0228 | 687.281 | OH | a1 | B03 | b1 | C13 | c1 | D01 | d1 | E02 |
| 2-1-m1E02-1-0229 | 688.206 | OH | a1 | B02 | b1 | C10 | c2 | D02 | d1 | E02 |
| 2-1-m1E02-1-0230 | 688.214 | OH | a2 | B19 | b2 | C01 | c1 | D01 | d1 | E02 |
| 2-1-m1E02-1-0231 | 688.226 | OH | a2 | B20 | b1 | C10 | c1 | D01 | d1 | E02 |
| 2-1-mlE02-1-0232 | 688.231 | OH | a2 | B14 | b1 | C09 | c1 | D01 | d1 | E02 |
| 2-1-m1E02-1-0233 | 688.236 | OH | a2 | B16 | b1 | C14 | c1 | D01 | d1 | E02 |
| 2-1-m1E02-1-0234 | 688.257 | OH | a2 | B16 | b2 | C33 | c3 | D02 | d1 | E02 |
| 2-1-m1E02-1-0235 | 689.216 | OH | a1 | B03 | b3 | C15 | c1 | D01 | d1 | E02 |
| 2-1-mlE02-1-0236 | 689.246 | OH | a1 | B09 | b1 | C08 | c1 | D01 | d1 | E02 |
| 2-1-mlE02-1-0237 | 689.246 | OH | a1 | B01 | b1 | C09 | c1 | D01 | d1 | E02 |
| 2-1-m1E02-1-0238 | 690.162 | OH | a2 | B14 | b3 | C06 | c1 | D01 | d1 | E02 |
| 2-1-m1E02-1-0239 | 690.240 | OH | a2 | B17 | b1 | C13 | c1 | D01 | d1 | E02 |
| 2-1-m1E02-1-0240 | 690.241 | OH | a1 | B02 | b1 | C09 | c1 | D01 | d1 | E02 |
| 2-1-m1E02-1-0241 | 690.241 | OH | a1 | B09 | b1 | C11 | c1 | D01 | d1 | E02 |
| 2-1-m1E02-1-0242 | 690.342 | OH | a2 | B15 | b2 | C07 | c1 | D01 | d1 | E02 |
| 2-1-m1E02-1-0243 | 691.157 | OH | a2 | B14 | b1 | C12 | c2 | D02 | d1 | E02 |
| 2-1-mlE02-1-0244 | 691.177 | OH | a1 | B01 | b3 | C06 | c1 | D01 | d1 | E02 |
| 2-1-m1E02-1-0245 | 691.191 | OH | a1 | B07 | b2 | C06 | c1 | D01 | d1 | E02 |
| 2-1-m1E02-1-0246 | 691.237 | OH | a1 | B09 | b1 | C10 | c1 | D01 | d1 | E02 |
| 2-1-m1E02-1-0247 | 691.246 | OH | a1 | B03 | b1 | C14 | c1 | D01 | d1 | E02 |
| 2-1-m1E02-1-0248 | 691.255 | OH | a1 | B04 | b1 | C13 | c1 | D01 | d1 | E02 |
| 2-1-m1E02-1-0249 | 691.268 | OH | a1 | B03 | b2 | C33 | c3 | D02 | d1 | E02 |
| 2-1-m1E02-1-0250 | 692.172 | OH | a1 | B02 | b3 | C06 | c1 | D01 | d1 | E02 |
| 2-1-m1E02-1-0251 | 692.172 | OH | a1 | B01 | b1 | C12 | c2 | D02 | d1 | E02 |
| 2-1-m1E02-1-0252 | 692.175 | OH | a2 | B17 | b3 | C15 | c1 | D01 | d1 | E02 |
| 2-1-m1E02-1-0253 | 692.267 | OH | a2 | B13 | b1 | C30 | c3 | D02 | d1 | E02 |
| 2-1-m1E02-1-0254 | 692.267 | OH | a2 | B11 | b1 | C32 | c3 | D02 | d1 | E02 |
| 2-1-m1E02-1-0255 | 692.296 | OH | a1 | B05 | b2 | COS | c1 | D01 | d1 | E02 |
| 2-1-m1E02-1-0256 | 692.321 | OH | a2 | B16 | b2 | C07 | c1 | D01 | d1 | E02 |
| 2-1-m1E02-1-0257 | 693.168 | OH | a1 | B02 | b1 | C12 | c2 | D02 | d1 | E02 |
| 2-1-m1E02-1-0258 | 693.187 | OH | a2 | B20 | b1 | C12 | c1 | D01 | d1 | E02 |
| 2-1-m1E02-1-0259 | 693.190 | OH | a1 | B04 | b3 | C15 | c1 | D01 | d1 | E02 |
| 2-1-m1E02-1-0260 | 693.262 | OH | a2 | B12 | b1 | C32 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0261 | 694.206 | OH | a2 | B17 | b1 | C14 | c1 | D01 | d1 | E02 |
| 2-1-m1E02-1-0262 | 694.210 | OH | a2 | B11 | b2 | C02 | c2 | D02 | d1 | E02 |
| 2-1-m1E02-1-0263 | 694.227 | OH | a2 | B17 | b2 | C33 | c3 | D02 | d1 | E02 |
| 2-1-m1E02-1-0264 | 694.246 | OH | a2 | B15 | b3 | C08 | c1 | D01 | d1 | E02 |
| 2-1-m1E02-1-0265 | 694.246 | OH | a2 | B15 | b1 | C08 | c2 | D02 | d1 | E02 |
| 2-1-m1E02-1-0266 | 694.246 | OH | a2 | B13 | b2 | C20 | c3 | D02 | d1 | E02 |
| 2-1-m1E02-1-0267 | 694.246 | OH | a2 | B11 | b2 | C22 | c3 | D02 | d1 | E02 |
| 2-1-m1E02-1-0268 | 694.275 | OH | a1 | B06 | b2 | C05 | c1 | D01 | d1 | E02 |
| 2-1-m1E02-1-0269 | 695.205 | OH | a2 | B12 | b2 | C02 | c2 | D02 | d1 | E02 |
| 2-1-m1E02-1-0270 | 695.205 | OH | a2 | B11 | b2 | C03 | c2 | D02 | d1 | E02 |
| 2-1-m1E02-1-0271 | 695.221 | OH | a1 | B04 | b1 | C14 | c1 | D01 | d1 | E02 |
| 2-1-m1E02-1-0272 | 695.241 | OH | a2 | B15 | b1 | C11 | c2 | D02 | d1 | E02 |
| 2-1-m1E02-1-0273 | 695.241 | OH | a2 | B12 | b2 | C22 | c3 | D02 | d1 | E02 |
| 2-1-m1E02-1-0274 | 695.241 | OH | a2 | B13 | b2 | C28 | c3 | D02 | d1 | E02 |
| 2-1-m1E02-1-0275 | 695.243 | OH | a1 | B04 | b2 | C33 | c3 | D02 | d1 | E02 |
| 2-1-m1E02-1-0276 | 695.274 | OH | a2 | B18 | b2 | C05 | c1 | D01 | d1 | E02 |
| 2-1-m1E02-1-0277 | 695.276 | OH | a1 | B05 | b2 | C04 | c1 | D01 | d1 | E02 |
| 2-1-m1E02-1-0278 | 695.332 | OH | a1 | B03 | b2 | C07 | c1 | D01 | d1 | E02 |
| 2-1-m1E02-1-0279 | 696.198 | OH | a1 | B09 | b1 | C12 | c1 | D01 | d1 | E02 |
| 2-1-m1E02-1-0280 | 696.200 | OH | a1 | B08 | b2 | C01 | c1 | D01 | d1 | E02 |
| 2-1-m1E02-1-0281 | 696.200 | OH | a2 | B12 | b2 | C03 | c2 | D02 | d1 | E02 |
| 2-1-m1E02-1-0282 | 696.225 | OH | a2 | B16 | b3 | C08 | c1 | D01 | d1 | E02 |
| 2-1-m1E02-1-0283 | 696.225 | OH | a2 | B16 | b1 | C08 | c2 | D02 | d1 | E02 |
| 2-1-m1E02-1-0284 | 696.237 | OH | a2 | B15 | b1 | C10 | c2 | D02 | d1 | E02 |
| 2-1-m1E02-1-0285 | 696.242 | OH | a2 | B14 | b1 | C30 | c3 | D02 | d1 | E02 |
| 2-1-m1E02-1-0286 | 697.221 | OH | a2 | B16 | b1 | C11 | c2 | D02 | d1 | E02 |
| 2-1-m1E02-1-0287 | 697.255 | OH | a1 | B06 | b2 | C04 | c1 | D01 | d1 | E02 |
| 2-1-m1E02-1-0288 | 697.257 | OH | a1 | B01 | b1 | C30 | c3 | D02 | d1 | E02 |
| 2-1-m1E02-1-0289 | 698.205 | OH | a2 | B13 | b2 | C01 | c2 | D02 | d1 | E02 |
| 2-1-m1E02-1-0290 | 698.216 | OH | a2 | B16 | b1 | C10 | c2 | D02 | d1 | E02 |
| 2-1-m1E02-1-0291 | 698.221 | OH | a2 | B14 | b2 | C20 | c3 | D02 | d1 | E02 |
| 2-1-m1E02-1-0292 | 698.221 | OH | a2 | B11 | b2 | C24 | c3 | D02 | d1 | E02 |
| 2-1-m1E02-1-0293 | 698.235 | OH | a2 | B19 | b2 | C02 | c1 | D01 | d1 | E02 |
| 2-1-m1E02-1-0294 | 698.252 | OH | a1 | B02 | b1 | C30 | c3 | D02 | d1 | E02 |
| 2-1-m1E02-1-0295 | 698.254 | OH | a2 | B18 | b2 | C04 | c1 | D01 | d1 | E02 |
| 2-1-m1E02-1-0296 | 698.272 | OH | a2 | B15 | b1 | C09 | c1 | D01 | d1 | E02 |
| 2-1-m1E02-1-0297 | 698.292 | OH | a2 | B17 | b2 | C07 | c1 | D01 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0298 | 699.216 | OH | a2 | B12 | b2 | C24 | c3 | D02 | d1 | E02 |
| 2-1-m1E02-1-0299 | 699.216 | OH | a2 | B14 | b2 | C28 | c3 | D02 | d1 | E02 |
| 2-1-m1E02-1-0300 | 699.230 | OH | a2 | B19 | b2 | C03 | c1 | D01 | d1 | E02 |
| 2-1-m1E02-1-0301 | 699.236 | OH | a1 | B03 | b3 | C08 | c1 | D01 | d1 | E02 |
| 2-1-m1E02-1-0302 | 699.236 | OH | a1 | B03 | b1 | C08 | c2 | D02 | d1 | E02 |
| 2-1-m1E02-1-0303 | 699.236 | OH | a1 | B01 | b2 | C20 | c3 | D02 | d1 | E02 |
| 2-1-m1E02-1-0304 | 699.307 | OH | a1 | B04 | b2 | C07 | c1 | D01 | d1 | E02 |
| 2-1-m1E02-1-0305 | 700.166 | OH | a2 | B11 | b2 | C06 | c2 | D02 | d1 | E02 |
| 2-1-m1E02-1-0306 | 700.203 | OH | a2 | B15 | b3 | C06 | c1 | D01 | d1 | E02 |
| 2-1-m1E02-1-0307 | 700.232 | OH | a1 | B03 | b1 | C11 | c2 | D02 | d1 | E02 |
| 2-1-m1E02-1-0308 | 700.232 | OH | a1 | B02 | b2 | C20 | c3 | D02 | d1 | E02 |
| 2-1-m1E02-1-0309 | 700.232 | OH | a1 | B01 | b2 | C28 | c3 | D02 | d1 | E02 |
| 2-1-m1**E**02-1-0310 | 700.246 | OH | a1 | B07 | b2 | C05 | c1 | D01 | d1 | E02 |
| 2-1-m1E02-1-0311 | 700.251 | OH | a2 | B16 | b1 | C09 | c1 | D01 | d1 | E02 |
| 2-1-m1E02-1-0312 | 701.161 | OH | a2 | B12 | b2 | C06 | c2 | D02 | d1 | E02 |
| 2-1-m1E02-1-0313 | 701.198 | OH | a2 | B15 | b1 | C12 | c2 | D02 | d1 | E02 |
| 2-1-m1E02-1-0314 | 701.227 | OH | a1 | B03 | b1 | C10 | c2 | D02 | d1 | E02 |
| 2-1-m1E02-1-0315 | 701.227 | OH | a1 | B02 | b2 | C28 | c3 | D02 | d1 | E02 |
| 2-1-m1E02-1-0316 | 701.296 | OH | a1 | B05 | b1 | C13 | c1 | D01 | d1 | E02 |
| 2-1-m1E02-1-0317 | 702.180 | OH | a2 | B14 | b2 | C01 | c2 | D02 | d1 | E02 |
| 2-1-m1E02-1-0318 | 702.182 | OH | a2 | B16 | b3 | C06 | c1 | D01 | d1 | E02 |
| 2-1-m1E02-1-0319 | 702.196 | OH | a2 | B17 | b3 | C08 | c1 | D01 | d1 | E02 |
| 2-1-m1E02-1-0320 | 702.196 | OH | a2 | B17 | b1 | C08 | c2 | D02 | d1 | E02 |
| 2-1-m1E02-1-0321 | 703.177 | OH | a2 | B16 | b1 | C12 | c2 | D02 | d1 | E02 |
| 2-1-m1E02-1-0322 | 703.191 | OH | a2 | B17 | b1 | C11 | c2 | D02 | d1 | E02 |
| 2-1-m1E02-1-0323 | 703.195 | OH | a1 | B01 | b2 | C01 | c2 | D02 | d1 | E02 |
| 2-1-m1E02-1-0324 | 703.211 | OH | a1 | B04 | b3 | C08 | c1 | D01 | d1 | E02 |
| 2-1-m1E02-1-0325 | 703.211 | OH | a1 | B04 | b1 | C08 | c2 | D02 | d1 | E02 |
| 2-1-m1E02-1-0326 | 703.225 | OH | a1 | B07 | b2 | C04 | c1 | D01 | d1 | E02 |
| 2-1-m1E02-1-0327 | 703.231 | OH | a1 | B05 | b3 | C15 | c1 | D01 | d1 | E02 |
| 2-1-m1E02-1-0328 | 703.262 | OH | a1 | B03 | b1 | C09 | c1 | D01 | d1 | E02 |
| 2-1-m1E02-1-0329 | 703.275 | OH | a1 | B06 | b1 | C13 | c1 | D01 | d1 | E02 |
| 2-1-m1E02-1-0330 | 704.186 | OH | a2 | B17 | b1 | C10 | c2 | D02 | d1 | E02 |
| 2-1-m1E02-1-0331 | 704.190 | OH | a1 | B02 | b2 | C01 | c2 | D02 | d1 | E02 |
| 2-1-m1E02-1-0332 | 704.192 | OH | a2 | B19 | b2 | C06 | c1 | D01 | d1 | E02 |
| 2-1-m1E02-1-0333 | 704.206 | OH | a1 | B04 | b1 | C11 | c2 | D02 | d1 | E02 |
| 2-1-m1E02-1-0334 | 704.209 | OH | a2 | B20 | b2 | C01 | c1 | D01 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0335 | 704.275 | OH | a2 | B18 | b1 | C13 | c1 | D01 | d1 | E02 |
| 2-1-m1E02-1-0336 | 705.193 | OH | a1 | B03 | b3 | C06 | c1 | D01 | d1 | E02 |
| 2-1-m1E02-1-0337 | 705.202 | OH | a1 | B04 | b1 | C10 | c2 | D02 | d1 | E02 |
| 2-1-m1E02-1-0338 | 705.210 | OH | a1 | B06 | b3 | C15 | c1 | D01 | d1 | E02 |
| 2-1-m1E02-1-0339 | 705.241 | OH | a1 | B10 | b1 | C08 | c1 | D01 | d1 | E02 |
| 2-1-m1E02-1-0340 | 705.262 | OH | a1 | B05 | b1 | C14 | c1 | D01 | d1 | E02 |
| 2-1-m1E02-1-0341 | 705.283 | OH | a1 | B05 | b2 | C33 | c3 | D02 | d1 | E02 |
| 2-1-m1E02-1-0342 | 706.188 | OH | a1 | B03 | b1 | C12 | c2 | D02 | d1 | E02 |
| 2-1-m1E02-1-0343 | 706.210 | OH | a2 | B18 | b3 | C15 | c1 | D01 | d1 | E02 |
| 2-1-m1E02-1-0344 | 706.221 | OH | a1 | B08 | b2 | C02 | c1 | D01 | d1 | E02 |
| 2-1-m1E02-1-0345 | 706.221 | OH | a2 | B17 | b1 | C09 | c1 | D01 | d1 | E02 |
| 2-1-m1E02-1-0346 | 706.236 | OH | a1 | B10 | b1 | C11 | c1 | D01 | d1 | E02 |
| 2-1-m1E02-1-0347 | 706.283 | OH | a2 | B15 | b1 | C30 | c3 | D02 | d1 | E02 |
| 2-1-m1E02-1-0348 | 706.283 | OH | a2 | B11 | b1 | C29 | c3 | D02 | d1 | E02 |
| 2-1-m1E02-1-0349 | 706.283 | OH | a2 | B13 | b1 | C32 | c3 | D02 | d1 | E02 |
| 2-1-m1E02-1-0350 | 707.216 | OH | a1 | B08 | b2 | C03 | c1 | D01 | d1 | E02 |
| 2-1-m1E02-1-0351 | 707.220 | OH | a1 | B09 | b2 | C01 | c1 | D01 | d1 | E02 |
| 2-1-m1E02-1-0352 | 707.232 | OH | a1 | B10 | b1 | C10 | c1 | D01 | d1 | E02 |
| 2-1-m1E02-1-0353 | 707.237 | OH | a1 | B04 | b1 | C09 | c1 | D01 | d1 | E02 |
| 2-1-m1E02-1-0354 | 707.241 | OH | a1 | B06 | b1 | C14 | c1 | D01 | d1 | E02 |
| 2-1-m1E02-1-0355 | 707.262 | OH | a1 | B06 | b2 | C33 | c3 | D02 | d1 | E02 |
| 2-1-m1E02-1-0356 | 707.278 | OH | a2 | B12 | b1 | C29 | c3 | D02 | d1 | E02 |
| 2-1-m1E02-1-0357 | 708.152 | OH | a2 | B17 | b3 | C06 | c1 | D01 | d1 | E02 |
| 2-1-m1E02-1-0358 | 708.225 | OH | a2 | B13 | b2 | C02 | c2 | D02 | d1 | E02 |
| 2-1-m1E02-1-0359 | 708.241 | OH | a2 | B18 | b1 | C14 | c1 | D01 | d1 | E02 |
| 2-1-m1E02-1-0360 | 708.262 | OH | a2 | B15 | b2 | C20 | c3 | D02 | d1 | E02 |
| 2-1-m1E02-1-0361 | 708.262 | OH | a2 | B13 | b2 | C22 | c3 | D02 | d1 | E02 |
| 2-1-m1E02-1-0362 | 708.262 | OH | a2 | B16 | b1 | C30 | c3 | D02 | d1 | E02 |
| 2-1-m1E02-1-0363 | 708.262 | OH | a2 | B18 | b2 | C33 | c3 | D02 | d1 | E02 |
| 2-1-m1E02-1-0364 | 709.148 | OH | a2 | B17 | b1 | C12 | c2 | D02 | d1 | E02 |
| 2-1-m1E02-1-0365 | 709.168 | OH | a1 | B04 | b3 | C06 | c1 | D01 | d1 | E02 |
| 2-1-m1E02-1-0366 | 709.221 | OH | a2 | B13 | b2 | C03 | c2 | D02 | d1 | E02 |
| 2-1-m1E02-1-0367 | 709.221 | OH | a2 | B11 | b2 | C05 | c2 | D02 | d1 | E02 |
| 2-1-m1E02-1-0368 | 709.246 | OH | a1 | B07 | b1 | C13 | c1 | D01 | d1 | E02 |
| 2-1-m1E02-1-0369 | 709.257 | OH | a2 | B15 | b2 | C28 | c3 | D02 | d1 | E02 |
| 2-1-m1E02-1-0370 | 709.348 | OH | a1 | B05 | b2 | C07 | c1 | D01 | d1 | E02 |
| 2-1-m1E02-1-0371 | 710.163 | OH | a1 | B04 | b1 | C12 | c2 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0372 | 710.216 | OH | a2 | B12 | b2 | C05 | c2 | D02 | d1 | E02 |
| 2-1-m1E02-1-0373 | 710.241 | OH | a2 | B16 | b2 | C20 | c3 | D02 | d1 | E02 |
| 2-1-m1E02-1-0374 | 710.241 | OH | a2 | B11 | b2 | C26 | c3 | D02 | d1 | E02 |
| 2-1-m1E02-1-0375 | 710.257 | OH | a2 | B14 | b1 | C32 | c3 | D02 | d1 | E02 |
| 2-1-m1E02-1-0376 | 711.181 | OH | a1 | B07 | b3 | C15 | c1 | D01 | d1 | E02 |
| 2-1-m1E02-1-0377 | 711.236 | OH | a2 | B12 | b2 | C26 | c3 | D02 | d1 | E02 |
| 2-1-m1E02-1-0378 | 711.236 | OH | a2 | B16 | b2 | C28 | c3 | D02 | d1 | E02 |
| 2-1-m1E02-1-0379 | 711.273 | OH | a1 | B03 | b1 | C30 | c3 | D02 | d1 | E02 |
| 2-1-m1E02-1-0380 | 711.273 | OH | a1 | B01 | b1 | C32 | c3 | D02 | d1 | E02 |
| 2-1-m1E02-1-0381 | 711.327 | OH | a1 | B06 | b2 | C07 | c1 | D01 | d1 | E02 |
| 2-1-m1E02-1-0382 | 712.177 | OH | a1 | B08 | b2 | C06 | c1 | D01 | d1 | E02 |
| 2-1-m1E02-1-0383 | 712.193 | OH | a1 | B10 | b1 | C12 | c1 | D01 | d1 | E02 |
| 2-1-m1E02-1-0384 | 712.200 | OH | a2 | B14 | b2 | C02 | c2 | D02 | d1 | E02 |
| 2-1-m1E02-1-0385 | 712.200 | OH | a2 | B11 | b2 | C04 | c2 | D02 | d1 | E02 |
| 2-1-m1E02-1-0386 | 712.220 | OH | a2 | B15 | b2 | C01 | c2 | D02 | d1 | E02 |
| 2-1-m1E02-1-0387 | 712.237 | OH | a2 | B14 | b2 | C22 | c3 | D02 | d1 | E02 |
| 2-1-m1E02-1-0388 | 712.237 | OH | a2 | B13 | b2 | C24 | c3 | D02 | d1 | E02 |
| 2-1-m1E02-1-0389 | 712.268 | OH | a1 | B02 | b1 | C32 | c3 | D02 | d1 | E02 |
| 2-1-m1E02-1-0390 | 712.326 | OH | a2 | B18 | b2 | C07 | c1 | D01 | d1 | E02 |
| 2-1-m1E02-1-0391 | 713.196 | OH | a2 | B12 | b2 | C04 | c2 | D02 | d1 | E02 |
| 2-1-m1E02-1-0392 | 713.196 | OH | a2 | B14 | b2 | C03 | c2 | D02 | d1 | E02 |
| 2-1-m1E02-1-0393 | 713.212 | OH | a1 | B07 | b1 | C14 | c1 | D01 | d1 | E02 |
| 2-1-m1E02-1-0394 | 713.216 | OH | a1 | B01 | b2 | C02 | c2 | D02 | d1 | E02 |
| 2-1-m1E02-1-0395 | 713.233 | OH | a1 | B07 | b2 | C33 | c3 | D02 | d1 | E02 |
| 2-1-m1E02-1-0396 | 713.246 | OH | a2 | B19 | b2 | C05 | c1 | D01 | d1 | E02 |
| 2-1-m1E02-1-0397 | 713.252 | OH | a1 | B05 | b3 | C08 | c1 | D01 | d1 | E02 |
| 2-1-m1E02-1-0398 | 713.252 | OH | a1 | B05 | b1 | C08 | c2 | D02 | d1 | E02 |
| 2-1-m1E02-1-0399 | 713.252 | OH | a1 | B03 | b2 | C20 | c3 | D02 | d1 | E02 |
| 2-1-m1E02-1-0400 | 713.252 | OH | a1 | B01 | b2 | C22 | c3 | D02 | d1 | E02 |
| 2-1-m1E02-1-0401 | 714.182 | OH | a2 | B13 | b2 | C06 | c2 | D02 | d1 | E02 |
| 2-1-m1E02-1-0402 | 714.200 | OH | a2 | B16 | b2 | C01 | c2 | D02 | d1 | E02 |
| 2-1-m1E02-1-0403 | 714.211 | OH | a1 | B02 | b2 | C02 | c2 | D02 | d1 | E02 |
| 2-1-m1E02-1-0404 | 714.211 | OH | a1 | B01 | b2 | C03 | c2 | D02 | d1 | E02 |
| 2-1-m1E02-1-0405 | 714.230 | OH | a2 | B20 | b2 | C02 | c1 | D01 | d1 | E02 |
| 2-1-m1E02-1-0406 | 714.232 | OH | a2 | B17 | b1 | C30 | c3 | D02 | d1 | E02 |
| 2-1-m1E02-1-0407 | 714.247 | OH | a1 | B05 | b1 | C11 | c2 | D02 | d1 | E02 |
| 2-1-m1E02-1-0408 | 714.247 | OH | a1 | B02 | b2 | C22 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0409 | 714.247 | OH | a1 | B03 | b2 | C28 | c3 | D02 | d1 | E02 |
| 2-1-m1E02-1-0410 | 715.206 | OH | a1 | B02 | b2 | C03 | c2 | D02 | d1 | E02 |
| 2-1-m1E02-1-0411 | 715.225 | OH | a2 | B20 | b2 | C03 | c1 | D01 | d1 | E02 |
| 2-1-m1E02-1-0412 | 715.231 | OH | a1 | B06 | b3 | C08 | c1 | D01 | d1 | E02 |
| 2-1-m1E02-1-0413 | 715.231 | OH | a1 | B06 | b1 | C08 | c2 | D02 | d1 | E02 |
| 2-1-m1E02-1-0414 | 715.242 | OH | a1 | B05 | b1 | C10 | c2 | D02 | d1 | E02 |
| 2-1-m1E02-1-0415 | 715.248 | OH | a1 | B04 | b1 | C30 | c3 | D02 | d1 | E02 |
| 2-1-m1E02-1-0416 | 716.212 | OH | a2 | B17 | b2 | C20 | c3 | D02 | d1 | E02 |
| 2-1-m1E02-1-0417 | 716.212 | OH | a2 | B14 | b2 | C24 | c3 | D02 | d1 | E02 |
| 2-1-m1E02-1-0418 | 716.226 | OH | a2 | B19 | b2 | C04 | c1 | D01 | d1 | E02 |
| 2-1-m1E02-1-0419 | 716.226 | OH | a1 | B06 | b1 | C11 | c2 | D02 | d1 | E02 |
| 2-1-m1E02-1-0420 | 716.230 | OH | a2 | B18 | b3 | C08 | c1 | D01 | d1 | E02 |
| 2-1-m1E02-1-0421 | 716.230 | OH | a2 | B18 | b1 | C08 | c2 | D02 | d1 | E02 |
| 2-1-m1E02-1-0422 | 717.207 | OH | a2 | B17 | b2 | C28 | c3 | D02 | d1 | E02 |
| 2-1-m1E02-1-0423 | 717.210 | OH | a1 | B03 | b2 | C01 | c2 | D02 | d1 | E02 |
| 2-1-m1E02-1-0424 | 717.222 | OH | a1 | B06 | b1 | C10 | c2 | D02 | d1 | E02 |
| 2-1-m1E02-1-0425 | 717.226 | OH | a2 | B18 | b1 | C11 | c2 | D02 | d1 | E02 |
| 2-1-m1E02-1-0426 | 717.227 | OH | a1 | B04 | b2 | C20 | c3 | D02 | d1 | E02 |
| 2-1-m1E02-1-0427 | 717.227 | OH | a1 | B01 | b2 | C24 | c3 | D02 | d1 | E02 |
| 2-1-m1E02-1-0428 | 717.241 | OH | a1 | B09 | b2 | C02 | c1 | D01 | d1 | E02 |
| 2-1-m1E02-1-0429 | 717.277 | OH | a1 | B05 | b1 | C09 | c1 | D01 | d1 | E02 |
| 2-1-m1E02-1-0430 | 717.297 | OH | a1 | B07 | b2 | C07 | c1 | D01 | d1 | E02 |
| 2-1-m1E02-1-0431 | 718.157 | OH | a2 | B14 | b2 | C06 | c2 | D02 | d1 | E02 |
| 2-1-m1E02-1-0432 | 718.221 | OH | a2 | B18 | b1 | C10 | c2 | D02 | d1 | E02 |
| 2-1-m1E02-1-0433 | 718.221 | OH | a2 | B11 | b1 | C13 | c2 | D02 | d1 | E02 |
| 2-1-m1E02-1-0434 | 718.222 | OH | a1 | B02 | b2 | C24 | c3 | D02 | d1 | E02 |
| 2-1-m1E02-1-0435 | 718.222 | OH | a1 | B04 | b2 | C28 | c3 | D02 | d1 | E02 |
| 2-1-m1E02-1-0436 | 718.236 | OH | a1 | B09 | b2 | C03 | c1 | D01 | d1 | E02 |
| 2-1-m1E02-1-0437 | 719.172 | OH | a1 | B01 | b2 | C06 | c2 | D02 | d1 | E02 |
| 2-1-m1E02-1-0438 | 719.208 | OH | a1 | B05 | b3 | C06 | c1 | D01 | d1 | E02 |
| 2-1-m1E02-1-0439 | 719.216 | OH | a2 | B12 | b1 | C13 | c2 | D02 | d1 | E02 |
| 2-1-m1E02-1-0440 | 719.257 | OH | a1 | B06 | b1 | C09 | c1 | D01 | d1 | E02 |
| 2-1-m1E02-1-0441 | 720.156 | OH | a2 | B11 | b3 | C15 | c2 | D02 | d1 | E02 |
| 2-1-m1E02-1-0442 | 720.167 | OH | a1 | B02 | b2 | C06 | c2 | D02 | d1 | E02 |
| 2-1-m1E02-1-0443 | 720.170 | OH | a2 | B17 | b2 | C01 | c2 | D02 | d1 | E02 |
| 2-1-m1E02-1-0444 | 720.187 | OH | a2 | B20 | b2 | C06 | c1 | D01 | d1 | E02 |
| 2-1-m1E02-1-0445 | 720.204 | OH | a1 | B05 | b1 | C12 | c2 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0446 | 720.225 | OH | a2 | B11 | b3 | C16 | c1 | D01 | d1 | E02 |
| 2-1-m1E02-1-0447 | 720.256 | OH | a2 | B18 | b1 | C09 | c1 | D01 | d1 | E02 |
| 2-1-m1E02-1-0448 | 720.298 | OH | a2 | B13 | b1 | C29 | c3 | D02 | d1 | E02 |
| 2-1-m1E02-1-0449 | 720.298 | OH | a2 | B15 | b1 | C32 | c3 | D02 | d1 | E02 |
| 2-1-m1E02-1-0450 | 721.151 | OH | a2 | B12 | b3 | C15 | c2 | D02 | d1 | E02 |
| 2-1-m1E02-1-0451 | 721.185 | OH | a1 | B04 | b2 | C01 | c2 | D02 | d1 | E02 |
| 2-1-m1E02-1-0452 | 721.188 | OH | a1 | B06 | b3 | C06 | c1 | D01 | d1 | E02 |
| 2-1-m1E02-1-0453 | 721.202 | OH | a1 | B07 | b3 | C08 | c1 | D01 | d1 | E02 |
| 2-1-m1E02-1-0454 | 721.202 | OH | a1 | B07 | b1 | C08 | c2 | D02 | d1 | E02 |
| 2-1-m1E02-1-0455 | 721.221 | OH | a2 | B12 | b3 | C16 | c1 | D01 | d1 | E02 |
| 2-1-m1E02-1-0456 | 721.232 | OH | a1 | B08 | b2 | C05 | c1 | D01 | d1 | E02 |
| 2-1-m1E02-1-0457 | 722.183 | OH | a1 | B06 | b1 | C12 | c2 | D02 | d1 | E02 |
| 2-1-m1E02-1-0458 | 722.187 | OH | a2 | B18 | b3 | C06 | c1 | D01 | d1 | E02 |
| 2-1-m1E02-1-0459 | 722.187 | OH | a2 | B11 | b3 | C17 | c1 | D01 | d1 | E02 |
| 2-1-m1E02-1-0460 | 722.187 | OH | a2 | B11 | b1 | C14 | c2 | D02 | d1 | E02 |
| 2-1-m1E02-1-0461 | 722.197 | OH | a1 | B07 | b1 | C11 | c2 | D02 | d1 | E02 |
| 2-1-m1E02-1-0462 | 722.241 | OH | a2 | B15 | b2 | C02 | c2 | D02 | d1 | E02 |
| 2-1-m1E02-1-0463 | 722.246 | OH | a2 | B19 | b1 | C13 | c1 | D01 | d1 | E02 |
| 2-1-m1E02-1-0464 | 722.277 | OH | a2 | B15 | b2 | C22 | c3 | D02 | d1 | E02 |
| 2-1-m1E02-1-0465 | 722.277 | OH | a2 | B16 | b1 | C32 | c3 | D02 | d1 | E02 |
| 2-1-m1E02-1-0466 | 723.182 | OH | a2 | B12 | b3 | C17 | c1 | D01 | d1 | E02 |
| 2-1-m1E02-1-0467 | 723.182 | OH | a2 | B18 | b1 | C12 | c2 | D02 | d1 | E02 |
| 2-1-m1E02-1-0468 | 723.182 | OH | a2 | B12 | b1 | C14 | c2 | D02 | d1 | E02 |
| 2-1-m1E02-1-0469 | 723.192 | OH | a1 | B07 | b1 | C10 | c2 | D02 | d1 | E02 |
| 2-1-m1E02-1-0470 | 723.197 | OH | a1 | B09 | b2 | C06 | c1 | D01 | d1 | E02 |
| 2-1-m1E02-1-0471 | 723.215 | OH | a1 | B10 | b2 | C01 | c1 | D01 | d1 | E02 |
| 2-1-m1E02-1-0472 | 723.236 | OH | a2 | B15 | b2 | C03 | c2 | D02 | d1 | E02 |
| 2-1-m1E02-1-0473 | 723.236 | OH | a2 | B13 | b2 | C05 | c2 | D02 | d1 | E02 |
| 2-1-m1E02-1-0474 | 724.181 | OH | a2 | B19 | b3 | C15 | c1 | D01 | d1 | E02 |
| 2-1-m1E02-1-0475 | 724.212 | OH | a1 | B08 | b2 | C04 | c1 | D01 | d1 | E02 |
| 2-1-m1E02-1-0476 | 724.220 | OH | a2 | B16 | b2 | C02 | c2 | D02 | d1 | E02 |
| 2-1-m1E02-1-0477 | 724.257 | OH | a2 | B16 | b2 | C22 | c3 | D02 | d1 | E02 |
| 2-1-m1E02-1-0478 | 724.257 | OH | a2 | B13 | b2 | C26 | c3 | D02 | d1 | E02 |
| 2-1-m1E02-1-0479 | 724.273 | OH | a2 | B14 | b1 | C29 | c3 | D02 | d1 | E02 |
| 2-1-m1E02-1-0480 | 725.216 | OH | a2 | B16 | b2 | C03 | c2 | D02 | d1 | E02 |
| 2-1-m1E02-1-0481 | 725.227 | OH | a1 | B07 | b1 | C09 | c1 | D01 | d1 | E02 |
| 2-1-m1E02-1-0482 | 725.288 | OH | a1 | B05 | b1 | C30 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0483 | 725.288 | OH | a1 | B01 | b1 | C29 | c3 | D02 | d1 | E02 |
| 2-1-m1E02-1-0484 | 725.288 | OH | a1 | B03 | b1 | C32 | c3 | D02 | d1 | E02 |
| 2-1-m1E02-1-0485 | 726.212 | OH | a2 | B19 | b1 | C14 | c1 | D01 | d1 | E02 |
| 2-1-m1E02-1-0486 | 726.216 | OH | a2 | B13 | b2 | C04 | c2 | D02 | d1 | E02 |
| 2-1-m1E02-1-0487 | 726.234 | OH | a2 | B19 | b2 | C33 | c3 | D02 | d1 | E02 |
| 2-1-m1E02-1-0488 | 726.252 | OH | a2 | B15 | b2 | C24 | c3 | D02 | d1 | E02 |
| 2-1-m1E02-1-0489 | 726.272 | OH | a2 | B11 | b2 | C07 | c2 | D02 | d1 | E02 |
| 2-1-m1E02-1-0490 | 726.284 | OH | a1 | B02 | b1 | C29 | c3 | D02 | d1 | E02 |
| 2-1-m1E02-1-0491 | 727.158 | OH | a1 | B07 | b3 | C06 | c1 | D01 | d1 | E02 |
| 2-1-m1E02-1-0492 | 727.211 | OH | a2 | B14 | b2 | C05 | c2 | D02 | d1 | E02 |
| 2-1-m1E02-1-0493 | 727.231 | OH | a1 | B03 | b2 | C02 | c2 | D02 | d1 | E02 |
| 2-1-m1E02-1-0494 | 727.268 | OH | a2 | B12 | b2 | C07 | c2 | D02 | d1 | E02 |
| 2-1-m1E02-1-0495 | 727.268 | OH | a1 | B05 | b2 | C20 | c3 | D02 | d1 | E02 |
| 2-1-m1E02-1-0496 | 727.268 | OH | a1 | B03 | b2 | C22 | c3 | D02 | d1 | E02 |
| 2-1-m1E02-1-0497 | 727.268 | OH | a1 | B06 | b1 | C30 | c3 | D02 | d1 | E02 |
| 2-1-m1E02-1-0498 | 728.153 | OH | a1 | B07 | b1 | C12 | c2 | D02 | d1 | E02 |
| 2-1-m1E02-1-0499 | 728.197 | OH | a2 | B15 | b2 | C06 | c2 | D02 | d1 | E02 |
| 2-1-m1E02-1-0500 | 728.226 | OH | a1 | B03 | b2 | C03 | c2 | D02 | d1 | E02 |
| 2-1-m1E02-1-0501 | 728.226 | OH | a1 | B01 | b2 | C05 | c2 | D02 | d1 | E02 |
| 2-1-m1E02-1-0502 | 728.232 | OH | a2 | B16 | b2 | C24 | c3 | D02 | d1 | E02 |
| 2-1-m1E02-1-0503 | 728.232 | OH | a2 | B14 | b2 | C26 | c3 | D02 | d1 | E02 |
| 2-1-m1E02-1-0504 | 728.248 | OH | a2 | B17 | b1 | C32 | c3 | D02 | d1 | E02 |
| 2-1-m1E02-1-0505 | 728.263 | OH | a1 | B05 | b2 | C28 | c3 | D02 | d1 | E02 |
| 2-1-m1E02-1-0506 | 728.267 | OH | a2 | B18 | b1 | C30 | c3 | D02 | d1 | E02 |
| 2-1-m1E02-1-0507 | 729.222 | OH | a1 | B02 | b2 | C05 | c2 | D02 | d1 | E02 |
| 2-1-m1E02-1-0508 | 729.241 | OH | a2 | B20 | b2 | C05 | c1 | D01 | d1 | E02 |
| 2-1-m1E02-1-0509 | 729.247 | OH | a1 | B06 | b2 | C20 | c3 | D02 | d1 | E02 |
| 2-1-m1E02-1-0510 | 729.247 | OH | a1 | B01 | b2 | C26 | c3 | D02 | d1 | E02 |
| 2-1-m1E02-1-0511 | 729.263 | OH | a1 | B04 | b1 | C32 | c3 | D02 | d1 | E02 |
| 2-1-m1E02-1-0512 | 730.177 | OH | a2 | B16 | b2 | C06 | c2 | D02 | d1 | E02 |
| 2-1-m1E02-1-0513 | 730.177 | OH | a2 | B11 | b3 | C08 | c2 | D02 | d1 | E02 |
| 2-1-m1E02-1-0514 | 730.191 | OH | a2 | B17 | b2 | C02 | c2 | D02 | d1 | E02 |
| 2-1-m1E02-1-0515 | 730.191 | OH | a2 | B14 | b2 | C04 | c2 | D02 | d1 | E02 |
| 2-1-m1E02-1-0516 | 730.227 | OH | a2 | B17 | b2 | C22 | c3 | D02 | d1 | E02 |
| 2-1-m1E02-1-0517 | 730.232 | OH | a1 | B08 | b1 | C13 | c1 | D01 | d1 | E02 |
| 2-1-m1E02-1-0518 | 730.242 | OH | a1 | B02 | b2 | C26 | c3 | D02 | d1 | E02 |
| 2-1-m1E02-1-0519 | 730.242 | OH | a1 | B06 | b2 | C28 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0520 | 730.246 | OH | a2 | B18 | b2 | C20 | c3 | D02 | d1 | E02 |
| 2-1-m1E02-1-0521 | 730.298 | OH | a2 | B19 | b2 | C07 | c1 | D01 | d1 | E02 |
| 2-1-m1E02-1-0522 | 731.172 | OH | a2 | B12 | b3 | C08 | c2 | D02 | d1 | E02 |
| 2-1-m1E02-1-0523 | 731.186 | OH | a2 | B17 | b2 | C03 | c2 | D02 | d1 | E02 |
| 2-1-m1E02-1-0524 | 731.206 | OH | a1 | B04 | b2 | C02 | c2 | D02 | d1 | E02 |
| 2-1-m1E02-1-0525 | 731.206 | OH | a1 | B01 | b2 | C04 | c2 | D02 | d1 | E02 |
| 2-1-m1E02-1-0526 | 731.226 | OH | a1 | B05 | b2 | C01 | c2 | D02 | d1 | E02 |
| 2-1-m1E02-1-0527 | 731.241 | OH | a2 | B18 | b2 | C28 | c3 | D02 | d1 | E02 |
| 2-1-m1E02-1-0528 | 731.243 | OH | a1 | B04 | b2 | C22 | c3 | D02 | d1 | E02 |
| 2-1-m1E02-1-0529 | 731.243 | OH | a1 | B03 | b2 | C24 | c3 | D02 | d1 | E02 |
| 2-1-m1E02-1-0530 | 732.167 | OH | a1 | B08 | b3 | C15 | c1 | D01 | d1 | E02 |
| 2-1-m1E02-1-0531 | 732.201 | OH | a1 | B02 | b2 | C04 | c2 | D02 | d1 | E02 |
| 2-1-m1E02-1-0532 | 732.201 | OH | a1 | B04 | b2 | C03 | c2 | D02 | d1 | E02 |
| 2-1-m1E02-1-0533 | 732.221 | OH | a2 | B20 | b2 | C04 | c1 | D01 | d1 | E02 |
| 2-1-m1E02-1-0534 | 732.237 | OH | a2 | B13 | b1 | C13 | c2 | D02 | d1 | E02 |
| 2-1-m1E02-1-0535 | 732.252 | OH | a1 | B09 | b2 | C05 | c1 | D01 | d1 | E02 |
| 2-1-m1E02-1-0536 | 733.188 | OH | a1 | B03 | b2 | C06 | c2 | D02 | d1 | E02 |
| 2-1-m1E02-1-0537 | 733.205 | OH | a1 | B06 | b2 | C01 | c2 | D02 | d1 | E02 |
| 2-1-m1E02-1-0538 | 733.236 | OH | a1 | B10 | b2 | C02 | c1 | D01 | d1 | E02 |
| 2-1-m1E02-1-0539 | 733.238 | OH | a1 | B07 | b1 | C30 | c3 | D02 | d1 | E02 |
| 2-1-m1E02-1-0540 | 734.172 | OH | a2 | B13 | b3 | C15 | c2 | D02 | d1 | E02 |
| 2-1-m1E02-1-0541 | 734.198 | OH | a1 | B08 | b1 | C14 | c1 | D01 | d1 | E02 |
| 2-1-m1E02-1-0542 | 734.202 | OH | a2 | B19 | b3 | C08 | c1 | D01 | d1 | E02 |
| 2-1-m1E02-1-0543 | 734.202 | OH | a2 | B19 | b1 | C08 | c2 | D02 | d1 | E02 |
| 2-1-m1E02-1-0544 | 734.202 | OH | a2 | B11 | b1 | C09 | c2 | D02 | d1 | E02 |
| 2-1-m1E02-1-0545 | 734.202 | OH | a2 | B17 | b2 | C24 | c3 | D02 | d1 | E02 |
| 2-1-m1E02-1-0546 | 734.205 | OH | a2 | B18 | b2 | C01 | c2 | D02 | d1 | E02 |
| 2-1-m1E02-1-0547 | 734.219 | OH | a1 | B08 | b2 | C33 | c3 | D02 | d1 | E02 |
| 2-1-m1E02-1-0548 | 734.231 | OH | a1 | B10 | b2 | C03 | c1 | D01 | d1 | E02 |
| 2-1-m1E02-1-0549 | 734.241 | OH | a2 | B13 | b3 | C16 | c1 | D01 | d1 | E02 |
| 2-1-m1E02-1-0550 | 734.277 | OH | a2 | B11 | b1 | C31 | c3 | D02 | d1 | E02 |
| 2-1-m1E02-1-0551 | 734.314 | OH | a2 | B15 | b1 | C29 | c3 | D02 | d1 | E02 |
| 2-1-m1E02-1-0552 | 735.197 | OH | a2 | B12 | b1 | C09 | c2 | D02 | d1 | E02 |
| 2-1-m1E02-1-0553 | 735.197 | OH | a2 | B19 | b1 | C11 | c2 | D02 | d1 | E02 |
| 2-1-m1E02-1-0554 | 735.217 | OH | a1 | B07 | b2 | C20 | c3 | D02 | d1 | E02 |
| 2-1-m1E02-1-0555 | 735.217 | OH | a1 | B04 | b2 | C24 | c3 | D02 | d1 | E02 |
| 2-1-m1E02-1-0556 | 735.232 | OH | a1 | B09 | b2 | C04 | c1 | D01 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0557 | 735.273 | OH | a2 | B12 | b1 | C31 | c3 | D02 | d1 | E02 |
| 2-1-m1E02-1-0558 | 736.133 | OH | a2 | B11 | b3 | C06 | c2 | D02 | d1 | E02 |
| 2-1-m1E02-1-0559 | 736.147 | OH | a2 | B17 | b2 | C06 | c2 | D02 | d1 | E02 |
| 2-1-m1E02-1-0560 | 736.193 | OH | a2 | B19 | b1 | C10 | c2 | D02 | d1 | E02 |
| 2-1-m1E02-1-0561 | 736.203 | OH | a2 | B13 | b3 | C17 | c1 | D01 | d1 | E02 |
| 2-1-m1E02-1-0562 | 736.203 | OH | a2 | B13 | b1 | C14 | c2 | D02 | d1 | E02 |
| 2-1-m1E02-1-0563 | 736.212 | OH | a2 | B14 | b1 | C13 | c2 | D02 | d1 | E02 |
| 2-1-m1E02-1-0564 | 736.213 | OH | a1 | B07 | b2 | C28 | c3 | D02 | d1 | E02 |
| 2-1-m1E02-1-0565 | 736.293 | OH | a2 | B16 | b1 | C29 | c3 | D02 | d1 | E02 |
| 2-1-m1E02-1-0566 | 737.128 | OH | a2 | B12 | b3 | C06 | c2 | D02 | d1 | E02 |
| 2-1-m1E02-1-0567 | 737.163 | OH | a1 | B04 | b2 | C06 | c2 | D02 | d1 | E02 |
| 2-1-m1E02-1-0568 | 737.198 | OH | a2 | B11 | b3 | C18 | c1 | D01 | d1 | E02 |
| 2-1-m1E02-1-0569 | 737.227 | OH | a1 | B01 | b1 | C13 | c2 | D02 | d1 | E02 |
| 2-1-m1E02-1-0570 | 737.252 | OH | a2 | B15 | b2 | C05 | c2 | D02 | d1 | E02 |
| 2-1-m1E02-1-0571 | 738.147 | OH | a2 | B14 | b3 | C15 | c2 | D02 | d1 | E02 |
| 2-1-m1E02-1-0572 | 738.193 | OH | a2 | B12 | b3 | C18 | c1 | D01 | d1 | E02 |
| 2-1-m1E02-1-0573 | 738.216 | OH | a2 | B14 | b3 | C16 | c1 | D01 | d1 | E02 |
| 2-1-m1E02-1-0574 | 738.222 | OH | a1 | B02 | b1 | C13 | c2 | D02 | d1 | E02 |
| 2-1-m1E02-1-0575 | 738.228 | OH | a2 | B19 | b1 | C09 | c1 | D01 | d1 | E02 |
| 2-1-m1E02-1-0576 | 738.241 | OH | a2 | B20 | b1 | C13 | c1 | D01 | d1 | E02 |
| 2-1-m1E02-1-0577 | 738.272 | OH | a2 | B15 | b2 | C26 | c3 | D02 | d1 | E02 |
| 2-1-m1E02-1-0578 | 738.284 | OH | a1 | B08 | b2 | C07 | c1 | D01 | d1 | E02 |
| 2-1-m1E02-1-0579 | 739.162 | OH | a1 | B01 | b3 | C15 | c2 | D02 | d1 | E02 |
| 2-1-m1E02-1-0580 | 739.176 | OH | a1 | B07 | b2 | C01 | c2 | D02 | d1 | E02 |
| 2-1-m1E02-1-0581 | 739.192 | OH | a1 | B10 | b2 | C06 | c1 | D01 | d1 | E02 |
| 2-1-m1E02-1-0582 | 739.231 | OH | a1 | B01 | b3 | C16 | c1 | D01 | d1 | E02 |
| 2-1-m1E02-1-0583 | 739.231 | OH | a2 | B16 | b2 | C05 | c2 | D02 | d1 | E02 |
| 2-1-m1E02-1-0584 | 739.304 | OH | a1 | B03 | b1 | C29 | c3 | D02 | d1 | E02 |
| 2-1-m1E02-1-0585 | 739.304 | OH | a1 | B05 | b1 | C32 | c3 | D02 | d1 | E02 |
| 2-1-m1E02-1-0586 | 740.157 | OH | a1 | B02 | b3 | C15 | c2 | D02 | d1 | E02 |
| 2-1-m1E02-1-0587 | 740.159 | OH | a2 | B19 | b3 | C06 | c1 | D01 | d1 | E02 |
| 2-1-m1E02-1-0588 | 740.176 | OH | a2 | B20 | b3 | C15 | c1 | D01 | d1 | E02 |
| 2-1-m1E02-1-0589 | 740.177 | OH | a2 | B14 | b3 | C17 | c1 | D01 | d1 | E02 |
| 2-1-m1E02-1-0590 | 740.177 | OH | a2 | B14 | b1 | C14 | c2 | D02 | d1 | E02 |
| 2-1-m1E02-1-0591 | 740.226 | OH | a1 | B02 | b3 | C16 | c1 | D01 | d1 | E02 |
| 2-1-m1E02-1-0592 | 740.232 | OH | a2 | B15 | b2 | C04 | c2 | D02 | d1 | E02 |
| 2-1-m1E02-1-0593 | 740.252 | OH | a2 | B16 | b2 | C26 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0594 | 740.288 | OH | a2 | B13 | b2 | C07 | c2 | D02 | d1 | E02 |
| 2-1-m1E02-1-0595 | 741.154 | OH | a2 | B19 | b1 | C12 | c2 | D02 | d1 | E02 |
| 2-1-m1E02-1-0596 | 741.193 | OH | a1 | B01 | b3 | C17 | c1 | D01 | d1 | E02 |
| 2-1-m1E02-1-0597 | 741.193 | OH | a1 | B01 | b1 | C14 | c2 | D02 | d1 | E02 |
| 2-1-m1E02-1-0598 | 741.247 | OH | a1 | B05 | b2 | C02 | c2 | D02 | d1 | E02 |
| 2-1-m1E02-1-0599 | 741.252 | OH | a1 | B09 | b1 | C13 | c1 | D01 | d1 | E02 |
| 2-1-m1E02-1-0600 | 741.283 | OH | a1 | B05 | b2 | C22 | c3 | D02 | d1 | E02 |
| 2-1-m1E02-1-0601 | 741.283 | OH | a1 | B06 | b1 | C32 | c3 | D02 | d1 | E02 |
| 2-1-m1E02-1-0602 | 742.188 | OH | a1 | B02 | b3 | C17 | c1 | D01 | d1 | E02 |
| 2-1-m1E02-1-0603 | 742.188 | OH | a1 | B08 | b3 | C08 | c1 | D01 | d1 | E02 |
| 2-1-m1E02-1-0604 | 742.188 | OH | a1 | B08 | b1 | C08 | c2 | D02 | d1 | E02 |
| 2-1-m1E02-1-0605 | 742.188 | OH | a1 | B02 | b1 | C14 | c2 | D02 | d1 | E02 |
| 2-1-m1E02-1-0606 | 742.207 | OH | a2 | B20 | b1 | C14 | c1 | D01 | d1 | E02 |
| 2-1-m1E02-1-0607 | 742.211 | OH | a2 | B16 | b2 | C04 | c2 | D02 | d1 1 | E02 |
| 2-1-m1E02-1-0608 | 742.228 | OH | a2 | B20 | b2 | C33 | c3 | D02 | d1 | E02 |
| 2-1-m1E02-1-0609 | 742.242 | OH | a1 | B05 | b2 | C03 | c2 | D02 | d1 | E02 |
| 2-1-m1E02-1-0610 | 742.242 | OH | a1 | B03 | b2 | C05 | c2 | D02 | d1 | E02 |
| 2-1-m1E02-1-0611 | 742.264 | OH | a2 | B17 | b1 | C29 | c3 | D02 | d | E02 |
| 2-1-m1E02-1-0612 | 742.283 | OH | a2 | B18 | b1 | C32 | c3 | D02 | d1 | E02 |
| 2-1-m1E02-1-0613 | 743.183 | OH | a1 | B08 | b1 | C11 | c2 | D02 | d1 | E02 |
| 2-1-m1E02-1-0614 | 743.187 | OH | a1 | B09 | b3 | C15 | c1 | D01 | d1 | E02 |
| 2-1-m1E02-1-0615 | 743.226 | OH | a1 | B06 | b2 | C02 | c2 | D02 | d1 | E02 |
| 2-1-m1E02-1-0616 | 743.262 | OH | a1 | B06 | b2 | C22 | c3 | D02 | d1 | E02 |
| 2-1-m1E02-1-0617 | 743.262 | OH | a1 | B03 | b2 | C26 | c3 | D02 | d1 | E02 |
| 2-1-m1E02-1-0618 | 743.279 | OH | a1 | B04 | b1 | C29 | c3 | D02 | d1 | E02 |
| 2-1-m1E02-1-0619 | 744.178 | OH | a1 | B08 | b1 | C10 | c2 | D02 | d1 | E02 |
| 2-1-m1E02-1-0620 | 744.192 | OH | a2 | B13 | b3 | C08 | c2 | D02 | d1 | E02 |
| 2-1-m1E02-1-0621 | 744.221 | OH | a1 | B06 | b2 | C03 | c2 | D02 | d1 | E02 |
| 2-1-m1E02-1-0622 | 744.225 | OH | a2 | B18 | b2 | C02 | c2 | D02 | d1 | E02 |
| 2-1-m1E02-1-0623 | 744.262 | OH | a2 | B18 | b2 | C22 | c3 | D02 | d1 | E02 |
| 2-1-m1E02-1-0624 | 744.263 | OH | a2 | B14 | b2 | C07 | c2 | D02 | d1 | E02 |
| 2-1-m1E02-1-0625 | 745.202 | OH | a2 | B17 | b2 | C05 | c2 | D02 | d1 | E02 |
| 2-1-m1E02-1-0626 | 745.218 | OH | a1 | B09 | b1 | C14 | c1 | D01 | d1 | E02 |
| 2-1-m1E02-1-0627 | 745.221 | OH | a2 | B18 | b2 | C03 | c2 | D02 | d1 | E02 |
| 2-1-m1E02-1-0628 | 745.222 | OH | a1 | B03 | b2 | C04 | c2 | D02 | d1 | E02 |
| 2-1-m1E02-1-0629 | 745.239 | OH | a1 | B09 | b2 | C33 | c3 | D02 | d1 | E02 |
| 2-1-m1E02-1-0630 | 745.258 | OH | a1 | B05 | b2 | C24 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0631 | 745.278 | OH | a1 | B01 | b2 | C07 | c2 | D02 | d1 | E02 |
| 2-1-m1E02-1-0632 | 746.213 | OH | a1 | B08 | b1 | C09 | c1 | D01 | d1 | E02 |
| 2-1-m1E02-1-0633 | 746.217 | OH | a1 | B04 | b2 | C05 | c2 | D02 | d1 | E02 |
| 2-1-m1E02-1-0634 | 746.222 | OH | a2 | B17 | b2 | C26 | c3 | D02 | d1 | E02 |
| 2-1-m1E02-1-0635 | 746.239 | OH | a2 | B19 | b1 | C30 | c3 | D02 | d1 | E02 |
| 2-1-m1E02-1-0636 | 746.252 | OH | a2 | B15 | b1 | C13 | c2 | D02 | d1 | E02 |
| 2-1-m1E02-1-0637 | 746.273 | OH | a1 | B02 | b2 | C07 | c2 | D02 | d1 | E02 |
| 2-1-m1E02-1-0638 | 746.293 | OH | a2 | B20 | b2 | C07 | c1 | D01 | d1 | E02 |
| 2-1-m1E02-1-0639 | 747.203 | OH | a1 | B05 | b2 | C06 | c2 | D02 | d1 | E02 |
| 2-1-m1E02-1-0640 | 747.237 | OH | a1 | B06 | b2 | C24 | c3 | D02 | d1 | E02 |
| 2-1-m1E02-1-0641 | 747.237 | OH | a1 | B04 | b2 | C26 | c3 | D02 | d1 | E02 |
| 2-1-m1E02-1-0642 | 747.254 | OH | a1 | B07 | b1 | C32 | c3 | D02 | d1 | E02 |
| 2-1-m1E02-1-0643 | 748.144 | OH | a1 | B08 | b3 | C06 | c1 | D01 | d1 | E02 |
| 2-1-m1E02-1-0644 | 748.167 | OH | a2 | B14 | b3 | C08 | c2 | D02 | d1 | E02 |
| 2-1-m1E02-1-0645 | 748.181 | OH | a2 | B17 | b2 | C04 | c2 | D02 | d1 | E02 |
| 2-1-m1E02-1-0646 | 748.187 | OH | a2 | B15 | b3 | C15 | c2 | D02 | d1 | E02 |
| 2-1-m1E02-1-0647 | 748.218 | OH | a2 | B13 | b1 | C09 | c2 | D02 | d1 | E02 |
| 2-1-m1E02-1-0648 | 748.218 | OH | a2 | B19 | b2 | C20 | c3 | D02 | d1 | E02 |
| 2-1-m1E02-1-0649 | 748.232 | OH | a2 | B16 | b1 | C13 | c2 | D02 | d1 | E02 |
| 2-1-m1E02-1-0650 | 748.237 | OH | a2 | B18 | b2 | C24 | c3 | D02 | d1 | E02 |
| 2-1-m1E02-1-0651 | 748.247 | OH | a1 | B10 | b2 | C05 | c1 | D01 | d1 | E02 |
| 2-1-m1E02-1-0652 | 748.257 | OH | a2 | B15 | b3 | C16 | c1 | D01 | d1 | E02 |
| 2-1-m1E02-1-0653 | 748.293 | OH | a2 | B13 | b1 | C31 | c3 | D02 | d1 | E02 |
| 2-1-m1E02-1-0654 | 749.140 | OH | a1 | B08 | b1 | C12 | c2 | D02 | d1 | E02 |
| 2-1-m1E02-1-0655 | 749.183 | OH | a1 | B06 | b2 | C06 | c2 | D02 | d1 | E02 |
| 2-1-m1E02-1-0656 | 749.183 | OH | a1 | B01 | b3 | C08 | c2 | D02 | d1 | E02 |
| 2-1-m1E02-1-0657 | 749.197 | OH | a1 | B07 | b2 | C02 | c2 | D02 | d1 | E02 |
| 2-1-m1E02-1-0658 | 749.197 | OH | a1 | B04 | b2 | C04 | c2 | D02 | d1 | E02 |
| 2-1-m1E02-1-0659 | 749.213 | OH | a2 | B19 | b2 | C28 | c3 | D02 | d1 | E02 |
| 2-1-m1E02-1-0660 | 749.233 | OH | a1 | B07 | b2 | C22 | c3 | D02 | d1 | E02 |
| 2-1-m1E02-1-0661 | 749.304 | OH | a1 | B09 | b2 | C07 | c1 | D01 | d1 | E02 |
| 2-1-m1E02-1-0662 | 750.149 | OH | a2 | B13 | b3 | C06 | c2 | D02 | d1 | E02 |
| 2-1-m1E02-1-0663 | 750.167 | OH | a2 | B16 | b3 | C15 | c2 | D02 | d1 | E02 |
| 2-1-m1E02-1-0664 | 750.178 | OH | a1 | B02 | b3 | C08 | c2 | D02 | d1 | E02 |
| 2-1-m1E02-1-0665 | 750.182 | OH | a2 | B18 | b2 | C06 | c2 | D02 | d1 | E02 |
| 2-1-m1E02-1-0666 | 750.192 | OH | a1 | B07 | b2 | C03 | c2 | D02 | d1 | E02 |
| 2-1-m1E02-1-0667 | 750.197 | OH | a2 | B20 | b3 | C08 | c1 | D01 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0668 | 750.197 | OH | a2 | B20 | b1 | C08 | c2 | D02 | d1 | E02 |
| 2-1-m1E02-1-0669 | 750.218 | OH | a2 | B15 | b3 | C17 | c1 | D01 | d1 | E02 |
| 2-1-m1E02-1-0670 | 750.218 | OH | a2 | B15 | b1 | C14 | c2 | D02 | d1 | E02 |
| 2-1-m1E02-1-0671 | 750.236 | OH | a2 | B16 | b3 | C16 | c1 | D01 | d1 | E02 |
| 2-1-m1E02-1-0672 | 751.192 | OH | a2 | B20 | b1 | C11 | c2 | D02 | d1 | E02 |
| 2-1-m1E02-1-0673 | 751.213 | OH | a2 | B13 | b3 | C18 | c1 | D01 | d1 | E02 |
| 2-1-m1E02-1-0674 | 751.227 | OH | a1 | B10 | b2 | C04 | c1 | D01 | d1 | E02 |
| 2-1-m1E02-1-0675 | 751.242 | OH | a1 | B03 | b1 | C13 | c2 | D02 | d1 | E02 |
| 2-1-m1E02-1-0676 | 752.176 | OH | a2 | B19 | b2 | C01 | c2 | D02 | d1 | E02 |
| 2-1-m1E02-1-0677 | 752.188 | OH | a2 | B20 | b1 | C10 | c2 | D02 | d1 | E02 |
| 2-1-m1E02-1-0678 | 752.193 | OH | a2 | B14 | b1 | C09 | c2 | D02 | d1 | E02 |
| 2-1-m1E02-1-0679 | 752.197 | OH | a2 | B16 | b3 | C17 | c1 | D01 | d1 | E02 |
| 2-1-m1E02-1-0680 | 752.197 | OH | a2 | B16 | b1 | C14 | c2 | D02 | d1 | E02 |
| 2-1-m1E02-1-0681 | 752.268 | OH | a2 | B14 | b1 | C31 | c3 | D02 | d1 | E02 |
| 2-1-m1E02-1-0682 | 753.177 | OH | a1 | B03 | b3 | C15 | c2 | D02 | d1 | E02 |
| 2-1-m1E02-1-0683 | 753.208 | OH | a1 | B09 | b3 | C08 | c1 | D01 | d1 | E02 |
| 2-1-m1E02-1-0684 | 753.208 | OH | a1 | B09 | b1 | C08 | c2 | D02 | d1 | E02 |
| 2-1-m1E02-1-0685 | 753.208 | OH | a1 | B01 | b1 | C09 | c2 | D02 | d1 | E02 |
| 2-1-m1E02-1-0686 | 753.208 | OH | a1 | B07 | b2 | C24 | c3 | D02 | d1 | E02 |
| 2-1-m1E02-1-0687 | 753.247 | OH | a1 | B03 | b3 | C16 | c1 | D01 | d1 | E02 |
| 2-1-m1E02-1-0688 | 753.283 | OH | a1 | B01 | b1 | C31 | c3 | D02 | d1 | E02 |
| 2-1-m1E02-1-0689 | 753.320 | OH | a1 | B05 | b1 | C29 | c3 | D02 | d1 | E02 |
| 2-1-m1E02-1-0690 | 754.124 | OH | a2 | B14 | b3 | C06 | c2 | D02 | d1 | E02 |
| 2-1-m1E02-1-0691 | 754.202 | OH | a2 | B17 | b1 | C13 | c2 | D02 | d1 | E02 |
| 2-1-m1E02-1-0692 | 754.203 | OH | a1 | B02 | b1 | C09 | c2 | D02 | d1 | E02 |
| 2-1-m1E02-1-0693 | 754.203 | OH | a1 | B09 | b1 | C11 | c2 | D02 | d1 | E02 |
| 2-1-m1E02-1-0694 | 754.223 | OH | a2 | B20 | b1 | C09 | c1 | D01 | d1 | E02 |
| 2-1-m1E02-1-0695 | 754.224 | OH | a1 | B08 | b1 | C30 | c3 | D02 | d1 | E02 |
| 2-1-m1E02-1-0696 | 754.278 | OH | a1 | B02 | b1 | C31 | c3 | D02 | d1 | E02 |
| 2-1-m1E02-1-0697 | 754.304 | OH | a2 | B15 | b2 | C07 | c2 | D02 | d1 | E02 |
| 2-1-m1E02-1-0698 | 755.139 | OH | a1 | B01 | b3 | C06 | c2 | D02 | d1 | E02 |
| 2-1-m1E02-1-0699 | 755.153 | OH | a1 | B07 | b2 | C06 | c2 | D02 | d1 | E02 |
| 2-1-m1E02-1-0700 | 755.188 | OH | a2 | B14 | b3 | C18 | c1 | D01 | d1 | E02 |
| 2-1-m1E02-1-0701 | 755.199 | OH | a1 | B09 | b1 | C10 | c2 | D02 | d1 | E02 |
| 2-1-m1E02-1-0702 | 755.208 | OH | a1 | B03 | b3 | C17 | c1 | D01 | d1 | E02 |
| 2-1-m1E02-1-0703 | 755.208 | OH | a1 | B03 | b1 | C14 | c2 | D02 | d1 | E02 |
| 2-1-m1E02-1-0704 | 755.217 | OH | a1 | B04 | b1 | C13 | c2 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0705 | 755.299 | OH | a1 | B06 | b1 | C29 | c3 | D02 | d1 | E02 |
| 2-1-m1E02-1-0706 | 756.134 | OH | a1 | B02 | b3 | C06 | c2 | D02 | d1 | E02 |
| 2-1-m1E02-1-0707 | 756.137 | OH | a2 | B17 | b3 | C15 | c2 | D02 | d1 | E02 |
| 2-1-m1E02-1-0708 | 756.154 | OH | a2 | B20 | b3 | C06 | c1 | D01 | d1 | E02 |
| 2-1-m1E02-1-0709 | 756.204 | OH | a1 | B01 | b3 | C18 | c1 | D01 | d1 | E02 |
| 2-1-m1E02-1-0710 | 756.204 | OH | a1 | B08 | b2 | C20 | c3 | D02 | d1 | E02 |
| 2-1-m1E02-1-0711 | 756.207 | OH | a2 | B17 | b3 | C16 | c1 | D01 | d1 | E02 |
| 2-1-m1E02-1-0712 | 756.258 | OH | a1 | B05 | b2 | C05 | c2 | D02 | d1 | E02 |
| 2-1-m1E02-1-0713 | 756.283 | OH | a2 | B16 | b2 | C07 | c2 | D02 | d1 | E02 |
| 2-1-m1E02-1-0714 | 756.298 | OH | a2 | B18 | b1 | C29 | c3 | D02 | d1 | E02 |
| 2-1-m1E02-1-0715 | 757.149 | OH | a2 | B20 | b1 | C12 | c2 | D02 | d1 | E02 |
| 2-1-m1E02-1-0716 | 757.152 | OH | a1 | B04 | b3 | C15 | c2 | D02 | d1 | E02 |
| 2-1-m1E02-1-0717 | 757.199 | OH | a1 | B02 | b3 | C18 | c1 | D01 | d1 | E02 |
| 2-1-m1E02-1-0718 | 757.199 | OH | a1 | B08 | b2 | C28 | c3 | D02 | d1 | E02 |
| 2-1-m1E02-1-0719 | 757.222 | OH | a1 | B04 | b3 | C16 | c1 | D01 | d1 | E02 |
| 2-1-m1E02-1-0720 | 757.234 | OH | a1 | B09 | b1 | C09 | c1 | D01 | d1 | E02 |
| 2-1-m1E02-1-0721 | 757.247 | OH | a1 | B10 | b1 | C13 | c1 | D01 | d1 | E02 |
| 2-1-m1E02-1-0722 | 757.278 | OH | a1 | B05 | b2 | C26 | c3 | D02 | d1 | E02 |
| 2-1-m1E02-1-0723 | 758.168 | OH | a2 | B17 | b3 | C17 | c1 | D01 | d1 | E02 |
| 2-1-m1E02-1-0724 | 758.168 | OH | a2 | B17 | b1 | C14 | c2 | D02 | d1 | E02 |
| 2-1-m1E02-1-0725 | 758.208 | OH | a2 | B15 | b3 | C08 | c2 | D02 | d1 | E02 |
| 2-1-m1E02-1-0726 | 758.237 | OH | a1 | B06 | b2 | C05 | c2 | D02 | d1 | E02 |
| 2-1-m1E02-1-0727 | 759.164 | OH | a1 | B09 | b3 | C06 | c1 | D01 | d1 | E02 |
| 2-1-m1E02-1-0728 | 759.182 | OH | a1 | B10 | b3 | C15 | c1 | D01 | d1 | E02 |
| 2-1-m1E02-1-0729 | 759.183 | OH | a1 | B04 | b3 | C17 | c1 | D01 | d1 | E02 |
| 2-1-m1E02-1-0730 | 759.183 | OH | a1 | B04 | b1 | C14 | c2 | D02 | d1 | E02 |
| 2-1-m1E02-1-0731 | 759.236 | OH | a2 | B18 | b2 | C05 | c2 | D02 | d1 | E02 |
| 2-1-m1E02-1-0732 | 759.237 | OH | a1 | B05 | b2 | C04 | c2 | D02 | d1 | E02 |
| 2-1-m1E02-1-0733 | 759.257 | OH | a1 | B06 | b2 | C26 | c3 | D02 | d1 | E02 |
| 2-1-m1E02-1-0734 | 759.294 | OH | a1 | B03 | b2 | C07 | c2 | D02 | d1 | E02 |
| 2-1-m1E02-1-0735 | 760.160 | OH | a1 | B09 | b1 | C12 | c2 | D02 | d1 | E02 |
| 2-1-m1E02-1-0736 | 760.162 | OH | a1 | B08 | b2 | C01 | c2 | D02 | d1 | E02 |
| 2-1-m1E02-1-0737 | 760.187 | OH | a2 | B16 | b3 | C08 | c2 | D02 | d1 | E02 |
| 2-1-m1E02-1-0738 | 760.254 | OH | a2 | B19 | b1 | C32 | c3 | D02 | d1 | E02 |
| 2-1-m1E02-1-0739 | 760.257 | OH | a2 | B18 | b2 | C26 | c3 | D02 | d1 | E02 |
| 2-1-m1E02-1-0740 | 761.213 | OH | a1 | B10 | b1 | C14 | c1 | D01 | d1 | E02 |
| 2-1-m1E02-1-0741 | 761.217 | OH | a1 | B06 | b2 | C04 | c2 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0742 | 761.234 | OH | a1 | B10 | b2 | C33 | c3 | D02 | d1 | E02 |
| 2-1-m1E02-1-0743 | 761.269 | OH | a1 | B07 | b1 | C29 | c3 | D02 | d1 | E02 |
| 2-1-m1E02-1-0744 | 762.197 | OH | a2 | B19 | b2 | C02 | c2 | D02 | d1 | E02 |
| 2-1-m1E02-1-0745 | 762.216 | OH | a2 | B18 | b2 | C04 | c2 | D02 | d1 | E02 |
| 2-1-m1E02-1-0746 | 762.234 | OH | a2 | B15 | b1 | C09 | c2 | D02 | d1 | E02 |
| 2-1-m1E02-1-0747 | 762.234 | OH | a2 | B19 | b2 | C22 | c3 | D02 | d1 | E02 |
| 2-1-m1E02-1-0748 | 762.234 | OH | a2 | B20 | b1 | C30 | c3 | D02 | d1 | E02 |
| 2-1-m1E02-1-0749 | 762.253 | OH | a2 | B17 | b2 | C07 | c2 | D02 | d1 | E02 |
| 2-1-m1E02-1-0750 | 762.309 | OH | a2 | B15 | b1 | C31 | c3 | D02 | d1 | E02 |
| 2-1-m1E02-1-0751 | 763.192 | OH | a2 | B19 | b2 | C03 | c2 | D02 | d1 | E02 |
| 2-1-m1E02-1-0752 | 763.198 | OH | a1 | B03 | b3 | C08 | c2 | D02 | d1 | E02 |
| 2-1-m1E02-1-0753 | 763.269 | OH | a1 | B04 | b2 | C07 | c2 | D02 | d1 | E02 |
| 2-1-m1E02-1-0754 | 764.164 | OH | a2 | B15 | b3 | C06 | c2 | D02 | d1 | E02 |
| 2-1-m1E02-1-0755 | 764.208 | OH | a1 | B07 | b2 | C05 | c2 | D02 | d1 | E02 |
| 2-1-m1E02-1-0756 | 764.213 | OH | a2 | B16 | b1 | C09 | c2 | D02 | d1 | E02 |
| 2-1-m1E02-1-0757 | 764.213 | OH | a2 | B20 | b2 | C20 | c3 | D02 | d1 | E02 |
| 2-1-m1E02-1-0758 | 764.288 | OH | a2 | B16 | b1 | C31 | c3 | D02 | d1 | E02 |
| 2-1-m1E02-1-0759 | 765.208 | OH | a2 | B20 | b2 | C28 | c3 | D02 | d1 | E02 |
| 2-1-m1E02-1-0760 | 765.228 | OH | a1 | B07 | b2 | C26 | c3 | D02 | d1 | E02 |
| 2-1-m1E02-1-0761 | 765.229 | OH | a2 | B15 | b3 | C18 | c1 | D01 | d1 | E02 |
| 2-1-m1E02-1-0762 | 765.244 | OH | a1 | B09 | b1 | C30 | c3 | D02 | d1 | E02 |
| 2-1-m1E02-1-0763 | 765.258 | OH | a1 | B05 | b1 | C13 | c2 | D02 | d1 | E02 |
| 2-1-m1E02-1-0764 | 765.299 | OH | a1 | B10 | b2 | C07 | c1 | D01 | d1 | E02 |
| 2-1-m1E02-1-0765 | 766.144 | OH | a2 | B16 | b3 | C06 | c2 | D02 | d1 | E02 |
| 2-1-m1E02-1-0766 | 766.158 | OH | a2 | B17 | b3 | C08 | c2 | D02 | d1 | E02 |
| 2-1-m1E02-1-0767 | 766.208 | OH | a2 | B19 | b2 | C24 | c3 | D02 | d1 | E02 |
| 2-1-m1E02-1-0768 | 767.173 | OH | a1 | B04 | b3 | C08 | c2 | D02 | d1 | E02 |
| 2-1-m1E02-1-0769 | 767.187 | OH | a1 | B07 | b2 | C04 | c2 | D02 | d1 | E02 |
| 2-1-m1E02-1-0770 | 767.193 | OH | a1 | B05 | b3 | C15 | c2 | D02 | d1 | E02 |
| 2-1-m1E02-1-0771 | 767.208 | OH | a2 | B16 | b3 | C18 | c1 | D01 | d1 | E02 |
| 2-1-m1E02-1-0772 | 767.224 | OH | a1 | B03 | b1 | C09 | c2 | D02 | d1 | E02 |
| 2-1-m1E02-1-0773 | 767.224 | OH | a1 | B09 | b2 | C20 | c3 | D02 | d1 | E02 |
| 2-1-m1E02-1-0774 | 767.237 | OH | a1 | B06 | b1 | C13 | c2 | D02 | d1 | E02 |
| 2-1-m1E02-1-0775 | 767.262 | OH | a1 | B05 | b3 | C16 | c1 | D01 | d1 | E02 |
| 2-1-m1E02-1-0776 | 767.299 | OH | a1 | B03 | b1 | C31 | c3 | D02 | d1 | E02 |
| 2-1-m1E02-1-0777 | 768.154 | OH | a2 | B19 | b2 | C06 | c2 | D02 | d1 | E02 |
| 2-1-m1E02-1-0778 | 768.171 | OH | a2 | B20 | b2 | C01 | c2 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0779 | 768.219 | OH | a1 | B09 | b2 | C28 | c3 | D02 | d1 | E02 |
| 2-1-m1E02-1-0780 | 768.237 | OH | a2 | B18 | b1 | C13 | c2 | D02 | d1 | E02 |
| 2-1-m1E02-1-0781 | 768.240 | OH | a1 | B08 | b1 | C32 | c3 | D02 | d1 | E02 |
| 2-1-m1E02-1-0782 | 769.155 | OH | a1 | B03 | b3 | C06 | c2 | D02 | d1 | E02 |
| 2-1-m1E02-1-0783 | 769.172 | OH | a1 | B06 | b3 | C15 | c2 | D02 | d1 | E02 |
| 2-1-m1E02-1-0784 | 769.203 | OH | a1 | B10 | b3 | C08 | c1 | D01 | d1 | E02 |
| 2-1-m1E02-1-0785 | 769.203 | OH | a1 | B10 | b1 | C08 | c2 | D02 | d1 | E02 |
| 2-1-m1E02-1-0786 | 769.224 | OH | a1 | B05 | b3 | C17 | c1 | D01 | d1 | E02 |
| 2-1-m1E02-1-0787 | 769.224 | OH | a1 | B05 | b1 | C14 | c2 | D02 | d1 | E02 |
| 2-1-m1E02-1-0788 | 769.242 | OH | a1 | B06 | b3 | C16 | c1 | D01 | d1 | E02 |
| 2-1-m1E02-1-0789 | 770.172 | OH | a2 | B18 | b3 | C15 | c2 | D02 | d1 | E02 |
| 2-1-m1E02-1-0790 | 770.183 | OH | a1 | B08 | b2 | C02 | c2 | D02 | d1 | E02 |
| 2-1-m1E02-1-0791 | 770.183 | OH | a2 | B17 | b1 | C09 | c2 | D02 | d1 | E02 |
| 2-1-m1E02-1-0792 | 770.198 | OH | a1 | B10 | b1 | C11 | c2 | D02 | d1 | E02 |
| 2-1-m1E02-1-0793 | 770.219 | OH | a1 | B03 | b3 | C18 | c1 | D01 | d1 | E02 |
| 2-1-m1E02-1-0794 | 770.219 | OH | a1 | B08 | b2 | C22 | c3 | D02 | d1 | E02 |
| 2-1-m1E02-1-0795 | 770.241 | OH | a2 | B18 | b3 | C16 | c1 | D01 | d1 | E02 |
| 2-1-m1E02-1-0796 | 770.259 | OH | a2 | B17 | b1 | C31 | c3 | D02 | d1 | E02 |
| 2-1-m1E02-1-0797 | 771.178 | OH | a1 | B08 | b2 | C03 | c2 | D02 | d1 | E02 |
| 2-1-m1E02-1-0798 | 771.182 | OH | a1 | B09 | b2 | C01 | c2 | D02 | d1 | E02 |
| 2-1-m1E02-1-0799 | 771.193 | OH | a1 | B10 | b1 | C10 | c2 | D02 | d1 | E02 |
| 2-1-m1E02-1-0800 | 771.199 | OH | a1 | B04 | b1 | C09 | c2 | D02 | d1 | E02 |
| 2-1-m1E02-1-0801 | 771.203 | OH | a1 | B06 | b3 | C17 | c1 | D01 | d1 | E02 |
| 2-1-m1E02-1-0802 | 771.203 | OH | a1 | B06 | b1 | C14 | c2 | D02 | d1 | E02 |
| 2-1-m1E02-1-0803 | 771.274 | OH | a1 | B04 | b1 | C31 | c3 | D02 | d1 | E02 |
| 2-1-m1E02-1-0804 | 772.114 | OH | a2 | B17 | b3 | C06 | c2 | D02 | d1 | E02 |
| 2-1-m1E02-1-0805 | 772.203 | OH | a2 | B18 | b3 | C17 | c1 | D01 | d1 | E02 |
| 2-1-m1E02-1-0806 | 772.203 | OH | a2 | B18 | b1 | C14 | c2 | D02 | d1 | E02 |
| 2-1-m1E02-1-0807 | 773.130 | OH | a1 | B04 | b3 | C06 | c2 | D02 | d1 | E02 |
| 2-1-m1E02-1-0808 | 773.179 | OH | a2 | B17 | b3 | C18 | c1 | D01 | d1 | E02 |
| 2-1-m1E02-1-0809 | 773.208 | OH | a1 | B07 | b1 | C13 | c2 | D02 | d1 | E02 |
| 2-1-m1E02-1-0810 | 773.228 | OH | a1 | B10 | b1 | C09 | c1 | D01 | d1 | E02 |
| 2-1-m1E02-1-0811 | 773.309 | OH | a1 | B05 | b2 | C07 | c2 | D02 | d1 | E02 |
| 2-1-m1E02-1-0812 | 774.194 | OH | a1 | B04 | b3 | C18 | c1 | D01 | d1 | E02 |
| 2-1-m1E02-1-0813 | 774.194 | OH | a1 | B08 | b2 | C24 | c3 | D02 | d1 | E02 |
| 2-1-m1E02-1-0814 | 774.270 | OH | a2 | B19 | b1 | C29 | c3 | D02 | d1 | E02 |
| 2-1-m1E02-1-0815 | 775.143 | OH | a1 | B07 | b3 | C15 | c2 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0816 | 775.159 | OH | a1 | B10 | b3 | C06 | c1 | D01 | d1 | E02 |
| 2-1-m1E02-1-0817 | 775.212 | OH | a1 | B07 | b3 | C16 | c1 | D01 | d1 | E02 |
| 2-1-m1E02-1-0818 | 775.289 | OH | a1 | B06 | b2 | C07 | c2 | D02 | d1 | E02 |
| 2-1-m1E02-1-0819 | 776.139 | OH | a1 | B08 | b2 | C06 | c2 | D02 | d1 | E02 |
| 2-1-m1E02-1-0820 | 776.155 | OH | a1 | B10 | b1 | C12 | c2 | D02 | d1 | E02 |
| 2-1-m1E02-1-0821 | 776.249 | OH | a2 | B20 | b1 | C32 | c3 | D02 | d1 | E02 |
| 2-1-m1E02-1-0822 | 776.288 | OH | a2 | B18 | b2 | C07 | c2 | D02 | d1 | E02 |
| 2-1-m1E02-1-0823 | 777.174 | OH | a1 | B07 | b3 | C17 | c1 | D01 | d1 | E02 |
| 2-1-m1E02-1-0824 | 777.174 | OH | a1 | B07 | b1 | C14 | c2 | D02 | d1 | E02 |
| 2-1-m1E02-1-0825 | 777.208 | OH | a2 | B19 | b2 | C05 | c2 | D02 | d1 | E02 |
| 2-1-m1E02-1-0826 | 777.214 | OH | a1 | B05 | b3 | C08 | c2 | D02 | d1 | E02 |
| 2-1-m1E02-1-0827 | 778.192 | OH | a2 | B20 | b2 | C02 | c2 | D02 | d1 | E02 |
| 2-1-m1E02-1-0828 | 778.228 | OH | a2 | B20 | b2 | C22 | c3 | D02 | d1 | E02 |
| 2-1-m1E02-1-0829 | 778.228 | OH | a2 | B19 | b2 | C26 | c3 | D02 | d1 | E02 |
| 2-1-m1E02-1-0830 | 779.187 | OH | a2 | B20 | b2 | C03 | c2 | D02 | d1 | E02 |
| 2-1-m1E02-1-0831 | 779.193 | OH | a1 | B06 | b3 | C08 | c2 | D02 | d1 | E02 |
| 2-1-m1E02-1-0832 | 779.260 | OH | a1 | B09 | b1 | C32 | c3 | D02 | d1 | E02 |
| 2-1-m1E02-1-0833 | 780.188 | OH | a2 | B19 | b2 | C04 | c2 | D02 | d1 | E02 |
| 2-1-m1E02-1-0834 | 780.192 | OH | a2 | B18 | b3 | C08 | c2 | D02 | d1 | E02 |
| 2-1-m1E02-1-0835 | 781.203 | OH | a1 | B09 | b2 | C02 | c2 | D02 | d1 | E02 |
| 2-1-m1E02-1-0836 | 781.239 | OH | a1 | B05 | b1 | C09 | c2 | D02 | d1 | E02 |
| 2-1-m1E02-1-0837 | 781.239 | OH | a1 | B09 | b2 | C22 | c3 | D02 | d1 | E02 |
| 2-1-m1E02-1-0838 | 781.239 | OH | a1 | B10 | b1 | C30 | c3 | D02 | d1 | E02 |
| 2-1-m1E02-1-0839 | 781.259 | OH | a1 | B07 | b2 | C07 | c2 | D02 | d1 | E02 |
| 2-1-m1E02-1-0840 | 781.315 | OH | a1 | B05 | b1 | C31 | c3 | D02 | d1 | E02 |
| 2-1-m1E02-1-0841 | 782.198 | OH | a1 | B09 | b2 | C03 | c2 | D02 | d1 | E02 |
| 2-1-m1E02-1-0842 | 782.203 | OH | a2 | B20 | b2 | C24 | c3 | D02 | d1 | E02 |
| 2-1-m1E02-1-0843 | 782.256 | OH | a1 | B08 | b1 | C29 | c3 | D02 | d1 | E02 |
| 2-1-m1E02-1-0844 | 783.170 | OH | a1 | B05 | b3 | C06 | c2 | D02 | d1 | E02 |
| 2-1-m1E02-1-0845 | 783.219 | OH | a1 | B06 | b1 | C09 | c2 | D02 | d1 | E02 |
| 2-1-m1E02-1-0846 | 783.219 | OH | a1 | B10 | b2 | C20 | c3 | D02 | d1 | E02 |
| 2-1-m1E02-1-0847 | 783.294 | OH | a1 | B06 | b1 | C31 | c3 | D02 | d1 | E02 |
| 2-1-m1E02-1-0848 | 784.148 | OH | a2 | B20 | b2 | C06 | c2 | D02 | d1 | E02 |
| 2-1-m1E02-1-0849 | 784.187 | OH | a2 | B11 | b3 | C16 | c2 | D02 | d1 | E02 |
| 2-1-m1E02-1-0850 | 784.214 | OH | a1 | B10 | b2 | C28 | c3 | D02 | d1 | E02 |
| 2-1-m1E02-1-0851 | 784.218 | OH | a2 | B18 | b1 | C09 | c2 | D02 | d1 | E02 |
| 2-1-m1E02-1-0852 | 784.235 | OH | a1 | B05 | b3 | C18 | c1 | D01 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0853 | 784.293 | OH | a2 | B18 | b1 | C31 | c3 | D02 | d1 | E02 |
| 2-1-m1E02-1-0854 | 785.150 | OH | a1 | B06 | b3 | C06 | c2 | D02 | d1 | E02 |
| 2-1-m1E02-1-0855 | 785.164 | OH | a1 | B07 | b3 | C08 | c2 | D02 | d1 | E02 |
| 2-1-m1E02-1-0856 | 785.183 | OH | a2 | B12 | b3 | C16 | c2 | D02 | d1 | E02 |
| 2-1-m1E02-1-0857 | 785.194 | OH | a1 | B08 | b2 | C05 | c2 | D02 | d1 | E02 |
| 2-1-m1E02-1-0858 | 785.214 | OH | a1 | B09 | b2 | C24 | c3 | D02 | d1 | E02 |
| 2-1-m1E02-1-0859 | 786.149 | OH | a2 | B18 | b3 | C06 | c2 | D02 | d1 | E02 |
| 2-1-m1E02-1-0860 | 786.149 | OH | a2 | B11 | b3 | C17 | c2 | D02 | d1 | E02 |
| 2-1-m1E02-1-0861 | 786.208 | OH | a2 | B19 | b1 | C13 | c2 | D02 | d1 | E02 |
| 2-1-m1E02-1-0862 | 786.214 | OH | a1 | B06 | b3 | C18 | c1 | D01 | d1 | E02 |
| 2-1-m1E02-1-0863 | 786.214 | OH | a1 | B08 | b2 | C26 | c3 | D02 | d1 | E02 |
| 2-1-m1E02-1-0864 | 787.144 | OH | a2 | B12 | b3 | C17 | c2 | D02 | d1 | E02 |
| 2-1-m1E02-1-0865 | 787.159 | OH | a1 | B09 | b2 | C06 | c2 | D02 | d1 | E02 |
| 2-1-m1E02-1-0866 | 787.177 | OH | a1 | B10 | b2 | C01 | c2 | D02 | d1 | E02 |
| 2-1-m1E02-1-0867 | 787.213 | OH | a2 | B18 | b3 | C18 | c1 | D01 | d1 | E02 |
| 2-1-m1E02-1-0868 | 788.143 | OH | a2 | B19 | b3 | C15 | c2 | D02 | d1 | E02 |
| 2-1-m1E02-1-0869 | 788.173 | OH | a1 | B08 | b2 | C04 | c2 | D02 | d1 | E02 |
| 2-1-m1E02-1-0870 | 788.213 | OH | a2 | B19 | b3 | C16 | c1 | D01 | d1 | E02 |
| 2-1-m1E02-1-0871 | 789.189 | OH | a1 | B07 | b1 | C09 | c2 | D02 | d1 | E02 |
| 2-1-m1E02-1-0872 | 789.264 | OH | a1 | B07 | b1 | C31 | c3 | D02 | d1 | E02 |
| 2-1-m1E02-1-0873 | 790.174 | OH | a2 | B19 | b3 | C17 | c1 | D01 | d1 | E02 |
| 2-1-m1E02-1-0874 | 790.174 | OH | a2 | B19 | b1 | C14 | c2 | D02 | d1 | E02 |
| 2-1-m1E02-1-0875 | 790.265 | OH | a2 | B20 | b1 | C29 | c3 | D02 | d1 | E02 |
| 2-1-m1E02-1-0876 | 791.120 | OH | a1 | B07 | b3 | C06 | c2 | D02 | d1 | E02 |
| 2-1-m1E02-1-0877 | 792.185 | OH | a1 | B07 | b3 | C18 | c1 | D01 | d1 | E02 |
| 2-1-m1E02-1-0878 | 793.203 | OH | a2 | B20 | b2 | C05 | c2 | D02 | d1 | E02 |
| 2-1-m1E02-1-0879 | 793.276 | OH | a1 | B09 | b1 | C29 | c3 | D02 | d1 | E02 |
| 2-1-m1E02-1-0880 | 794.194 | OH | a1 | B08 | b1 | C13 | c2 | D02 | d1 | E02 |
| 2-1-m1E02-1-0881 | 794.223 | OH | a2 | B20 | b2 | C26 | c3 | D02 | d1 | E02 |
| 2-1-m1E02-1-0882 | 794.260 | OH | a2 | B19 | b2 | C07 | c2 | D02 | d1 | E02 |
| 2-1-m1E02-1-0883 | 795.255 | OH | a1 | B10 | b1 | C32 | c3 | D02 | d1 | E02 |
| 2-1-m1E02-1-0884 | 796.129 | OH | a1 | B08 | b3 | C15 | c2 | D02 | d1 | E02 |
| 2-1-m1E02-1-0885 | 796.183 | OH | a2 | B20 | b2 | C04 | c2 | D02 | d1 | E02 |
| 2-1-m1E02-1-0886 | 796.199 | OH | a1 | B08 | b3 | C16 | c1 | D01 | d1 | E02 |
| 2-1-m1E02-1-0887 | 796.214 | OH | a1 | B09 | b2 | C05 | c2 | D02 | d1 | E02 |
| 2-1-m1E02-1-0888 | 797.198 | OH | a1 | B10 | b2 | C02 | c2 | D02 | d1 | E02 |
| 2-1-m1E02-1-0889 | 797.234 | OH | a1 | B10 | b2 | C22 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0890 | 797.234 | OH | a1 | B09 | b2 | C26 | c3 | D02 | d1 | E02 |
| 2-1-m1E02-1-0891 | 798.160 | OH | a1 | B08 | b3 | C17 | c1 | D01 | d1 | E02 |
| 2-1-m1E02-1-0892 | 798.160 | OH | a1 | B08 | b1 | C14 | c2 | D02 | d1 | E02 |
| 2-1-m1E02-1-0893 | 798.164 | OH | a2 | B19 | b3 | C08 | c2 | D02 | d1 | E02 |
| 2-1-m1E02-1-0894 | 798.193 | OH | a1 | B10 | b2 | C03 | c2 | D02 | d1 | E02 |
| 2-1-m1E02-1-0895 | 798.203 | OH | a2 | B13 | b3 | C16 | c2 | D02 | d1 | E02 |
| 2-1-m1E02-1-0896 | 799.194 | OH | a1 | B09 | b2 | C04 | c2 | D02 | d1 | E02 |
| 2-1-m1E02-1-0897 | 800.164 | OH | a2 | B13 | b3 | C17 | c2 | D02 | d1 | E02 |
| 2-1-m1E02-1-0898 | 801.160 | OH | a2 | B11 | b3 | C18 | c2 | D02 | d1 | E02 |
| 2-1-m1E02-1-0899 | 801.209 | OH | a1 | B10 | b2 | C24 | c3 | D02 | d1 | E02 |
| 2-1-m1E02-1-0900 | 802.155 | OH | a2 | B12 | b3 | C18 | c2 | D02 | d1 | E02 |
| 2-1-m1E02-1-0901 | 802.178 | OH | a2 | B14 | b3 | C16 | c2 | D02 | d1 | E02 |
| 2-1-m1E02-1-0902 | 802.190 | OH | a2 | B19 | b1 | C09 | c2 | D02 | d1 | E02 |
| 2-1-m1E02-1-0903 | 802.203 | OH | a2 | B20 | b1 | C13 | c2 | D02 | d1 | E02 |
| 2-1-m1E02-1-0904 | 802.245 | OH | a1 | B08 | b2 | C07 | c2 | D02 | d1 | E02 |
| 2-1-m1E02-1-0905 | 802.265 | OH | a2 | B19 | b1 | C31 | c3 | D02 | d1 | E02 |
| 2-1-m1E02-1-0906 | 803.154 | OH | a1 | B10 | b2 | C06 | c2 | D02 | d1 | E02 |
| 2-1-m1E02-1-0907 | 803.193 | OH | a1 | B01 | b3 | C16 | c2 | D02 | d1 | E02 |
| 2-1-m1E02-1-0908 | 804.121 | OH | a2 | B19 | b3 | C06 | c2 | D02 | d1 | E02 |
| 2-1-m1E02-1-0909 | 804.138 | OH | a2 | B20 | b3 | C15 | c2 | D02 | d1 | E02 |
| 2-1-m1E02-1-0910 | 804.139 | OH | a2 | B14 | b3 | C17 | c2 | D02 | d1 | E02 |
| 2-1-m1E02-1-0911 | 804.188 | OH | a1 | B02 | b3 | C16 | c2 | D02 | d1 | E02 |
| 2-1-m1E02-1-0912 | 804.208 | OH | a2 | B20 | b3 | C16 | c1 | D01 | d1 | E02 |
| 2-1-m1E02-1-0913 | 805.155 | OH | a1 | B01 | b3 | C17 | c2 | D02 | d1 | E02 |
| 2-1-m1E02-1-0914 | 805.185 | OH | a2 | B19 | b3 | C18 | c1 | D01 | d1 | E02 |
| 2-1-m1E02-1-0915 | 805.214 | OH | a1 | B09 | b1 | C13 | c2 | D02 | d1 | E02 |
| 2-1-m1E02-1-0916 | 806.150 | OH | a1 | B02 | b3 | C17 | c2 | D02 | d1 | E02 |
| 2-1-m1E02-1-0917 | 806.150 | OH | a1 | B08 | b3 | C08 | c2 | D02 | d1 | E02 |
| 2-1-m1E02-1-0918 | 806.169 | OH | a2 | B20 | b3 | C17 | c1 | D01 | d1 | E02 |
| 2-1-m1E02-1-0919 | 806.169 | OH | a2 | B20 | b1 | C14 | c2 | D02 | d1 | E02 |
| 2-1-m1E02-1-0920 | 807.149 | OH | a1 | B09 | b3 | C15 | c2 | D02 | d1 | E02 |
| 2-1-m1E02-1-0921 | 807.219 | OH | a1 | B09 | b3 | C16 | c1 | D01 | d1 | E02 |
| 2-1-m1E02-1-0922 | 809.180 | OH | a1 | B09 | b3 | C17 | c1 | D01 | d1 | E02 |
| 2-1-m1E02-1-0923 | 809.180 | OH | a1 | B09 | b1 | C14 | c2 | D02 | d1 | E02 |
| 2-1-m1E02-1-0924 | 809.271 | OH | a1 | B10 | b1 | C29 | c3 | D02 | d1 | E02 |
| 2-1-m1E02-1-0925 | 810.175 | OH | a1 | B08 | b1 | C09 | c2 | D02 | d1 | E02 |
| 2-1-m1E02-1-0926 | 810.251 | OH | a1 | B08 | b1 | C31 | c3 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0927 | 810.255 | OH | a2 | B20 | b2 | C07 | c2 | D02 | d1 | E02 |
| 2-1-m1E02-1-0928 | 812.106 | OH | a1 | B08 | b3 | C06 | c2 | D02 | d1 | E02 |
| 2-1-m1E02-1-0929 | 812.209 | OH | a1 | B10 | b2 | C05 | c2 | D02 | d1 | E02 |
| 2-1-m1E02-1-0930 | 812.219 | OH | a2 | B15 | b3 | C16 | c2 | D02 | d1 | E02 |
| 2-1-m1E02-1-0931 | 813.171 | OH | a1 | B08 | b3 | C18 | c1 | D01 | d1 | E02 |
| 2-1-m1E02-1-0932 | 813.229 | OH | a1 | B10 | b2 | C26 | c3 | D02 | d1 | E02 |
| 2-1-m1E02-1-0933 | 813.266 | OH | a1 | B09 | b2 | C07 | c2 | D02 | d1 | E02 |
| 2-1-m1E02-1-0934 | 814.159 | OH | a2 | B20 | b3 | C08 | c2 | D02 | d1 | E02 |
| 2-1-m1E02-1-0935 | 814.180 | OH | a2 | B15 | b3 | C17 | c2 | D02 | d1 | E02 |
| 2-1-m1E02-1-0936 | 814.198 | OH | a2 | B16 | b3 | C16 | c2 | D02 | d1 | E02 |
| 2-1-m1E02-1-0937 | 815.175 | OH | a2 | B13 | b3 | C18 | c2 | D02 | d1 | E02 |
| 2-1-m1E02-1-0938 | 815.188 | OH | a1 | B10 | b2 | C04 | c2 | D02 | d1 | E02 |
| 2-1-m1E02-1-0939 | 816.159 | OH | a2 | B16 | b3 | C17 | c2 | D02 | d1 | E02 |
| 2-1-m1E02-1-0940 | 817.170 | OH | a1 | B09 | b3 | C08 | c2 | D02 | d1 | E02 |
| 2-1-m1E02-1-0941 | 817.209 | OH | a1 | B03 | b3 | C16 | c2 | D02 | d1 | E02 |
| 2-1-m1E02-1-0942 | 818.185 | OH | a2 | B20 | b1 | C09 | c2 | D02 | d1 | E02 |
| 2-1-m1E02-1-0943 | 818.26 | OH | a2 | B20 | b1 | C31 | c3 | D02 | d1 | E02 |
| 2-1-m1E02-1-0944 | 819.150 | OH | a2 | B14 | b3 | C18 | c2 | D02 | d1 | E02 |
| 2-1-m1E02-1-0945 | 819.170 | OH | a1 | B03 | b3 | C17 | c2 | D02 | d1 | E02 |
| 2-1-m1E02-1-0946 | 820.115 | OH | a2 | B20 | b3 | C06 | c2 | D02 | d1 | E02 |
| 2-1-m1E02-1-0947 | 820.166 | OH | a1 | B01 | b3 | C18 | c2 | D02 | d1 | E02 |
| 2-1-m1E02-1-0948 | 820.168 | OH | a2 | B17 | b3 | C16 | c2 | D02 | d1 | E02 |
| 2-1-m1E02-1-0949 | 821.161 | OH | a1 | B02 | b3 | C18 | c2 | D02 | d1 | E02 |
| 2-1-m1E02-1-0950 | 821.180 | OH | a2 | B20 | b3 | C18 | c1 | D01 | d1 | E02 |
| 2-1-m1E02-1-0951 | 821.184 | OH | a1 | B04 | b3 | C16 | c2 | D02 | d1 | E02 |
| 2-1-m1E02-1-0952 | 821.195 | OH | a1 | B09 | b1 | C09 | c2 | D02 | d1 | E02 |
| 2-1-m1E02-1-0953 | 821.209 | OH | a1 | B10 | b1 | C13 | c2 | D02 | d1 | E02 |
| 2-1-m1E02-1-0954 | 821.271 | OH | a1 | B09 | b1 | C31 | c3 | D02 | d1 | E02 |
| 2-1-m1E02-1-0955 | 822.130 | OH | a2 | B17 | b3 | C17 | c2 | D02 | d1 | E02 |
| 2-1-mIE02-1-0956 | 823.126 | OH | a1 | B09 | b3 | C06 | c2 | D02 | d1 | E02 |
| 2-1-m1E02-1-0957 | 823.144 | OH | a1 | B10 | b3 | C15 | c2 | D02 | d1 | E02 |
| 2-1-m1E02-1-0958 | 823.145 | OH | a1 | B04 | b3 | C17 | c2 | D02 | d1 | E02 |
| 2-1-m1E02-1-0959 | 823.213 | OH | a1 | B10 | b3 | C16 | c1 | D01 | d1 | E02 |
| 2-1-m1E02-1-0960 | 824.191 | OH | a1 | B09 | b3 | C18 | c1 | D01 | d1 | E02 |
| 2-1-m1E02-1-0961 | 825.175 | OH | a1 | B10 | b3 | C17 | c1 | D01 | d1 | E02 |
| 2-1-m1E02-1-0962 | 825.175 | OH | a1 | B10 | b1 | C14 | c2 | D02 | d1 | E02 |
| 2-1-m1E02-1-0963 | 829.191 | OH | a2 | B15 | b3 | C18 | c2 | D02 | d1 | E02 |

(continued)

| [Table 8-8-3] Compound that may be contained in mixture 2-1-m1E02-1 | | | | | | | | | | |
|---|---|---|---|---|---|---|---|---|---|---|
| ID | Exact Mass | | a | B | b | C | c | D | d | E |
| 2-1-m1E02-1-0964 | 829.260 | OH | a1 | B10 | b2 | C07 | c2 | D02 | d1 | E02 |
| 2-1-m1E02-1-0965 | 831.170 | OH | a2 | B16 | b3 | C18 | c2 | D02 | d1 | E02 |
| 2-1-m1E02-1-0966 | 831.224 | OH | a1 | B05 | b3 | C16 | c2 | D02 | d1 | E02 |
| 2-1-m1E02-1-0967 | 833.165 | OH | a1 | B10 | b3 | C08 | c2 | D02 | d1 | E02 |
| 2-1-m1E02-1-0968 | 833.186 | OH | a1 | B05 | b3 | C17 | c2 | D02 | d1 | E02 |
| 2-1-m1E02-1-0969 | 833.204 | OH | a1 | B06 | b3 | C16 | c2 | D02 | d1 | E02 |
| 2-1-m1E02-1-0970 | 834.181 | OH | a1 | B03 | b3 | C18 | c2 | D02 | d1 | E02 |
| 2-1-m1E02-1-0971 | 834.203 | OH | a2 | B18 | b3 | C16 | c2 | D02 | d1 | E02 |
| 2-1-m1E02-1-0972 | 835.165 | OH | a1 | B06 | b3 | C17 | c2 | D02 | d1 | E02 |
| 2-1-m1E02-1-0973 | 836.164 | OH | a2 | B18 | b3 | C17 | c2 | D02 | d1 | E02 |
| 2-1-m1E02-1-0974 | 837.141 | OH | a2 | B17 | b3 | C18 | c2 | D02 | d1 | E02 |
| 2-1-m1E02-1-0975 | 837.190 | OH | a1 | B10 | b1 | C09 | c2 | D02 | d1 | E02 |
| 2-1-m1E02-1-0976 | 837.266 | OH | a1 | B10 | b1 | C31 | c3 | D02 | d1 | E02 |
| 2-1-m1E02-1-0977 | 838.156 | OH | a1 | B04 | b3 | C18 | c2 | D02 | d1 | E02 |
| 2-1-m1E02-1-0978 | 839.121 | OH | a1 | B10 | b3 | C06 | c2 | D02 | d1 | E02 |
| 2-1-m1E02-1-0979 | 839.174 | OH | a1 | B07 | b3 | C16 | c2 | D02 | d1 | E02 |
| 2-1-m1E02-1-0980 | 840.186 | OH | a1 | B10 | b3 | C18 | c1 | D01 | d1 | E02 |
| 2-1-m1E02-1-0981 | 841.136 | OH | a1 | B07 | b3 | C17 | c2 | D02 | d1 | E02 |
| 2-1-m1E02-1-0982 | 848.197 | OH | a1 | B05 | b3 | C18 | c2 | D02 | d1 | E02 |
| 2-1-m1E02-1-0983 | 850.176 | OH | a1 | B06 | b3 | C18 | c2 | D02 | d1 | E02 |
| 2-1-m1E02-1-0984 | 851.175 | OH | a2 | B18 | b3 | C18 | c2 | D02 | d1 | E02 |
| 2-1-m1E02-1-0985 | 852.175 | OH | a2 | B19 | b3 | C16 | c2 | D02 | d1 | E02 |
| 2-1-m1E02-1-0986 | 854.136 | OH | a2 | B19 | b3 | C17 | c2 | D02 | d1 | E02 |
| 2-1-m1E02-1-0987 | 856.147 | OH | a1 | B07 | b3 | C18 | c2 | D02 | d1 | E02 |
| 2-1-m1E02-1-0988 | 860.160 | OH | a1 | B08 | b3 | C16 | c2 | D02 | d1 | E02 |
| 2-1-m1E02-1-0989 | 862.122 | OH | a1 | B08 | b3 | C17 | c2 | D02 | d1 | E02 |
| 2-1-m1E02-1-0990 | 868.170 | OH | a2 | B20 | b3 | C16 | c2 | D02 | d1 | E02 |
| 2-1-m1E02-1-0991 | 869.147 | OH | a2 | B19 | b3 | C18 | c2 | D02 | d1 | E02 |
| 2-1-m1E02-1-0992 | 870.131 | OH | a2 | B20 | b3 | C17 | c2 | D02 | d1 | E02 |
| 2-1-m1E02-1-0993 | 871.180 | OH | a1 | B09 | b3 | C16 | c2 | D02 | d1 | E02 |
| 2-1-m1E02-1-0994 | 873.142 | OH | a1 | B09 | b3 | C17 | c2 | D02 | d1 | E02 |
| 2-1-m1E02-1-0995 | 877.133 | OH | a1 | B08 | b3 | C18 | c2 | D02 | d1 | E02 |
| 2-1-m1E02-1-0996 | 885.142 | OH | a2 | B20 | b3 | C18 | c2 | D02 | d1 | E02 |
| 2-1-m1E02-1-0997 | 887.175 | OH | a1 | B10 | b3 | C16 | c2 | D02 | d1 | E02 |
| 2-1-m1E02-1-0998 | 888.153 | OH | a1 | B09 | b3 | C18 | c2 | D02 | d1 | E02 |
| 2-1-m1E02-1-0999 | 889.137 | OH | a1 | B10 | b3 | C17 | c2 | D02 | d1 | E02 |
| 2-1-m1E02-1-1000 | 904.148 | OH | a1 | B10 | b3 | C18 | c2 | D02 | d1 | E02 |

[Table 396]

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0001 | 640.214 | OH | a2 | B11 | b1 | C08 | c1 | D01 | d1 | E03 |
| 2-1-m1E03-1-0002 | 641.210 | OH | a2 | B12 | b1 | C08 | c1 | D01 | d1 | E03 |
| 2-1-m1E03-1-0003 | 641.210 | OH | a2 | B11 | b1 | C11 | c1 | D01 | d1 | E03 |
| 2-1-m1E03-1-0004 | 642.205 | OH | a2 | B11 | b1 | C10 | c1 | D01 | d1 | E03 |
| 2-1-m1E03-1-0005 | 642.205 | OH | a2 | B12 | b1 | C11 | c1 | D01 | d1 | E03 |
| 2-1-m1E03-1-0006 | 643.200 | OH | a2 | B12 | b1 | C10 | c1 | D01 | d1 | E03 |
| 2-1-m1E03-1-0007 | 647.166 | OH | a2 | B11 | b1 | C12 | c1 | D01 | d1 | E03 |
| 2-1-m1E03-1-0008 | 648.161 | OH | a2 | B12 | b1 | C12 | c1 | D01 | d1 | E03 |
| 2-1-m1E03-1-0009 | 654.230 | OH | a2 | B13 | b1 | C08 | c1 | D01 | d1 | E03 |
| 2-1-m1E03-1-0010 | 655.225 | OH | a2 | B13 | b1 | C11 | c1 | D01 | d1 | E03 |
| 2-1-m1E03-1-0011 | 656.221 | OH | a2 | B13 | b1 | C10 | c1 | D01 | d1 | E03 |
| 2-1-m1E03-1-0012 | 658.189 | OH | a2 | B11 | b2 | C01 | c1 | D01 | d1 | E03 |
| 2-1-m1E03-1-0013 | 658.205 | OH | a2 | B14 | b1 | C08 | c1 | D01 | d1 | E03 |
| 2-1-m1E03-1-0014 | 659.184 | OH | a2 | B12 | b2 | C01 | c1 | D01 | d1 | E03 |
| 2-1-m1E03-1-0015 | 659.200 | OH | a2 | B14 | b1 | C11 | c1 | D01 | d1 | E03 |
| 2-1-m1E03-1-0016 | 659.220 | OH | a1 | B01 | b1 | C08 | c1 | D01 | d1 | E03 |
| 2-1-m1E03-1-0017 | 660.196 | OH | a2 | B14 | b1 | C10 | c1 | D01 | d1 | E03 |
| 2-1-m1E03-1-0018 | 660.215 | OH | a1 | B02 | b1 | C08 | c1 | D01 | d1 | E03 |
| 2-1-m1E03-1-0019 | 660.215 | OH | a1 | B01 | b1 | C11 | c1 | D01 | d1 | E03 |
| 2-1-m1E03-1-0020 | 661.182 | OH | a2 | B13 | b1 | C12 | c1 | D01 | d1 | E03 |
| 2-1-m1E03-1-0021 | 661.211 | OH | a1 | B01 | b1 | C10 | c1 | D01 | d1 | E03 |
| 2-1-m1E03-1-0022 | 661.211 | OH | a1 | B02 | b1 | C11 | c1 | D01 | d1 | E03 |
| 2-1-m1E03-1-0023 | 662.206 | OH | a1 | B02 | b1 | C10 | c1 | D01 | d1 | E03 |
| 2-1-m1E03-1-0024 | 665.157 | OH | a2 | B14 | b1 | C12 | c1 | D01 | d1 | E03 |
| 2-1-m1E03-1-0025 | 666.172 | OH | a1 | B01 | b1 | C12 | c1 | D01 | d1 | E03 |
| 2-1-m1E03-1-0026 | 667.167 | OH | a1 | B02 | b1 | C12 | c1 | D01 | d1 | E03 |
| 2-1-m1E03-1-0027 | 668.209 | OH | a2 | B11 | b2 | C02 | c1 | D01 | d1 | E03 |
| 2-1-m1E03-1-0028 | 668.246 | OH | a2 | B15 | b1 | C08 | c1 | D01 | d1 | E03 |
| 2-1-m1E03-1-0029 | 669.205 | OH | a2 | B12 | b2 | C02 | c1 | D01 | d1 | E03 |
| 2-1-m1E03-1-0030 | 669.205 | OH | a2 | B11 | b2 | C03 | c1 | D01 | d1 | E03 |
| 2-1-m1E03-1-0031 | 669.241 | OH | a2 | B15 | b1 | C11 | c1 | D01 | d1 | E03 |
| 2-1-m1E03-1-0032 | 670.200 | OH | a2 | B12 | b2 | C03 | c1 | D01 | d1 | E03 |
| 2-1-m1E03-1-0033 | 670.225 | OH | a2 | B16 | b1 | C08 | c1 | D01 | d1 | E03 |
| 2-1-m1E03-1-0034 | 670.236 | OH | a2 | B15 | b1 | C10 | c1 | D01 | d1 | E03 |
| 2-1-m1E03-1-0035 | 671.220 | OH | a2 | B16 | b1 | C11 | c1 | D01 | d1 | E03 |
| 2-1-m1E03-1-0036 | 672.204 | OH | a2 | B13 | b2 | C01 | c1 | D01 | d1 | E03 |
| 2-1-m1E03-1-0037 | 672.215 | OH | a2 | B16 | b1 | C10 | c1 | D01 | d1 | E03 |
| 2-1-m1E03-1-0038 | 673.236 | OH | a1 | B03 | b1 | C08 | c1 | D01 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0039 | 674.166 | OH | a2 | B11 | b2 | C06 | c1 | D01 | d1 | E03 |
| 2-1-m1E03-1-0040 | 674.231 | OH | a1 | B03 | b1 | C11 | c1 | D01 | d1 | E03 |
| 2-1-m1E03-1-0041 | 675.161 | OH | a2 | B12 | b2 | C06 | c1 | D01 | d1 | E03 |
| 2-1-m1E03-1-0042 | 675.197 | OH | a2 | B15 | b1 | C12 | c1 | D01 | d1 | E03 |
| 2-1-m1E03-1-0043 | 675.226 | OH | a1 | B03 | b1 | C10 | c1 | D01 | d1 | E03 |
| 2-1-m1E03-1-0044 | 676.179 | OH | a2 | B14 | b2 | C01 | c1 | D01 | d1 | E03 |
| 2-1-m1E03-1-0045 | 676.196 | OH | a2 | B17 | b1 | C08 | c1 | D01 | d1 | E03 |
| 2-1-m1E03-1-0046 | 677.177 | OH | a2 | B16 | b1 | C12 | c1 | D01 | d1 | E03 |
| 2-1-m1E03-1-0047 | 677.191 | OH | a2 | B17 | b1 | C11 | c1 | D01 | d1 | E03 |
| 2-1-m1E03-1-0048 | 677.194 | OH | a1 | B01 | b2 | C01 | c1 | D01 | d1 | E03 |
| 2-1-m1E03-1-0049 | 677.211 | OH | a1 | B04 | b1 | C08 | c1 | D01 | d1 | E03 |
| 2-1-m1E03-1-0050 | 678.186 | OH | a2 | B17 | b1 | C10 | c1 | D01 | d1 | E03 |
| 2-1-m1E03-1-0051 | 678.190 | OH | a1 | B02 | b2 | C01 | c1 | D01 | d1 | E03 |
| 2-1-m1E03-1-0052 | 678.206 | OH | a1 | B04 | b1 | C11 | c1 | D01 | d1 | E03 |
| 2-1-m1E03-1-0053 | 679.201 | OH | a1 | B04 | b1 | C10 | c1 | D01 | d1 | E03 |
| 2-1-m1E03-1-0054 | 680.188 | OH | a1 | B03 | b1 | C12 | c1 | D01 | d1 | E03 |
| 2-1-m1E03-1-0055 | 682.225 | OH | a2 | B13 | b2 | C02 | c1 | D01 | d1 | E03 |
| 2-1-m1E03-1-0056 | 683.147 | OH | a2 | B17 | b1 | C12 | c1 | D01 | d1 | E03 |
| 2-1-m1E03-1-0057 | 683.220 | OH | a2 | B13 | b2 | C03 | c1 | D01 | d1 | E03 |
| 2-1-m1E03-1-0058 | 683.220 | OH | a2 | B11 | b2 | C05 | c1 | D01 | d1 | E03 |
| 2-1-m1E03-1-0059 | 684.162 | OH | a1 | B04 | b1 | C12 | c1 | D01 | d1 | E03 |
| 2-1-m1E03-1-0060 | 684.215 | OH | a2 | B12 | b2 | C05 | c1 | D01 | d1 | E03 |
| 2-1-m1E03-1-0061 | 686.200 | OH | a2 | B14 | b2 | C02 | c1 | D01 | d1 | E03 |
| 2-1-m1E03-1-0062 | 686.200 | OH | a2 | B11 | b2 | C04 | c1 | D01 | d1 | E03 |
| 2-1-m1E03-1-0063 | 686.220 | OH | a2 | B15 | b2 | C01 | c1 | D01 | d1 | E03 |
| 2-1-m1E03-1-0064 | 687.195 | OH | a2 | B12 | b2 | C04 | c1 | D01 | d1 | E03 |
| 2-1-m1E03-1-0065 | 687.195 | OH | a2 | B14 | b2 | C03 | c1 | D01 | d1 | E03 |
| 2-1-m1E03-1-0066 | 687.215 | OH | a1 | B01 | b2 | C02 | c1 | D01 | d1 | E03 |
| 2-1-m1E03-1-0067 | 687.252 | OH | a1 | B05 | b1 | C08 | c1 | D01 | d1 | E03 |
| 2-1-m1E03-1-0068 | 688.181 | OH | a2 | B13 | b2 | C06 | c1 | D01 | d1 | E03 |
| 2-1-m1E03-1-0069 | 688.199 | OH | a2 | B16 | b2 | C01 | c1 | D01 | d1 | E03 |
| 2-1-m1E03-1-0070 | 688.210 | OH | a1 | B02 | b2 | C02 | c1 | D01 | d1 | E03 |
| 2-1-m1E03-1-0071 | 688.210 | OH | a1 | B01 | b2 | C03 | c1 | D01 | d1 | E03 |
| 2-1-m1E03-1-0072 | 688.247 | OH | a1 | B05 | b1 | C11 | c1 | D01 | d1 | E03 |
| 2-1-m1E03-1-0073 | 689.206 | OH | a1 | B02 | b2 | C03 | c1 | D01 | d1 | E03 |
| 2-1-m1E03-1-0074 | 689.231 | OH | a1 | B06 | b1 | C08 | c1 | D01 | d1 | E03 |
| 2-1-m1E03-1-0075 | 689.242 | OH | a1 | B05 | b1 | C10 | c1 | D01 | d1 | E03 |
| 2-1-m1E03-1-0076 | 690.226 | OH | a1 | B06 | b1 | C11 | c1 | D01 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0077 | 690.230 | OH | a2 | B18 | b1 | C08 | c1 | D01 | d1 | E03 |
| 2-1-m1E03-1-0078 | 691.210 | OH | a1 | B03 | b2 | C01 | c1 | D01 | d1 | E03 |
| 2-1-m1E03-1-0079 | 691.221 | OH | a1 | B06 | b1 | C10 | c1 | D01 | d1 | E03 |
| 2-1-m1E03-1-0080 | 691.225 | OH | a2 | B18 | b1 | C11 | c1 | D01 | d1 | E03 |
| 2-1-m1E03-1-0081 | 692.156 | OH | a2 | B14 | b2 | C06 | c1 | D01 | d1 | E03 |
| 2-1-m1E03-1-0082 | 692.221 | OH | a2 | B18 | b1 | C10 | c1 | D01 | d1 | E03 |
| 2-1-m1E03-1-0083 | 692.221 | OH | a2 | B11 | b1 | C13 | c1 | D01 | d1 | E03 |
| 2-1-m1E03-1-0084 | 693.172 | OH | a1 | B01 | b2 | C06 | c1 | D01 | d1 | E03 |
| 2-1-m1E03-1-0085 | 693.216 | OH | a2 | B12 | b1 | C13 | c1 | D01 | d1 | E03 |
| 2-1-m1E03-1-0086 | 694.156 | OH | a2 | B11 | b3 | C15 | c1 | D01 | d1 | E03 |
| 2-1-m1E03-1-0087 | 694.167 | OH | a1 | B02 | b2 | C06 | c1 | D01 | d1 | E03 |
| 2-1-m1E03-1-0088 | 694.170 | OH | a2 | B17 | b2 | C01 | c1 | D01 | d1 | E03 |
| 2-1-m1E03-1-0089 | 694.203 | OH | a1 | B05 | b1 | C12 | c1 | D01 | d1 | E03 |
| 2-1-m1E03-1-0090 | 695.151 | OH | a2 | B12 | b3 | C15 | c1 | D01 | d1 | E03 |
| 2-1-m1E03-1-0091 | 695.185 | OH | a1 | B04 | b2 | C01 | c1 | D01 | d1 | E03 |
| 2-1-m1E03-1-0092 | 695.201 | OH | a1 | B07 | b1 | C08 | c1 | D01 | d1 | E03 |
| 2-1-m1E03-1-0093 | 696.182 | OH | a1 | B06 | b1 | C12 | c1 | D01 | d1 | E03 |
| 2-1-m1E03-1-0094 | 696.186 | OH | a2 | B11 | b1 | C14 | c1 | D01 | d1 | E03 |
| 2-1-m1E03-1-0095 | 696.197 | OH | a1 | B07 | b1 | C11 | c1 | D01 | d1 | E03 |
| 2-1-m1E03-1-0096 | 696.208 | OH | a2 | B11 | b2 | C33 | c3 | D02 | d1 | E03 |
| 2-1-m1E03-1-0097 | 696.241 | OH | a2 | B15 | b2 | C02 | c1 | D01 | d1 | E03 |
| 2-1-m1E03-1-0098 | 697.182 | OH | a2 | B18 | b1 | C12 | c1 | D01 | d1 | E03 |
| 2-1-m1E03-1-0099 | 697.182 | OH | a2 | B12 | b1 | C14 | c1 | D01 | d1 | E03 |
| 2-1-m1E03-1-0100 | 697.192 | OH | a1 | B07 | b1 | C10 | c1 | D01 | d1 | E03 |
| 2-1-m1E03-1-0101 | 697.203 | OH | a2 | B12 | b2 | C33 | c3 | D02 | d1 | E03 |
| 2-1-m1E03-1-0102 | 697.236 | OH | a2 | B15 | b2 | C03 | c1 | D01 | d1 | E03 |
| 2-1-m1E03-1-0103 | 697.236 | OH | a2 | B13 | b2 | C05 | c1 | D01 | d1 | E03 |
| 2-1-m1E03-1-0104 | 698.220 | OH | a2 | B16 | b2 | C02 | c1 | D01 | d1 | E03 |
| 2-1-m1E03-1-0105 | 699.215 | OH | a2 | B16 | b2 | C03 | c1 | D01 | d1 | E03 |
| 2-1-m1E03-1-0106 | 700.216 | OH | a2 | B13 | b2 | C04 | c1 | D01 | d1 | E03 |
| 2-1-m1E03-1-0107 | 700.272 | OH | a2 | B11 | b2 | C07 | c1 | D01 | d1 | E03 |
| 2-1-m1E03-1-0108 | 701.211 | OH | a2 | B14 | b2 | C05 | c1 | D01 | d1 | E03 |
| 2-1-m1E03-1-0109 | 701.231 | OH | a1 | B03 | b2 | C02 | c1 | D01 | d1 | E03 |
| 2-1-m1E03-1-0110 | 701.267 | OH | a2 | B12 | b2 | C07 | c1 | D01 | d1 | E03 |
| 2-1-m1E03-1-0111 | 702.153 | OH | a1 | B07 | b1 | C12 | c1 | D01 | d1 | E03 |
| 2-1-m1E03-1-0112 | 702.197 | OH | a2 | B15 | b2 | C06 | c1 | D01 | d1 | E03 |
| 2-1-m1E03-1-0113 | 702.226 | OH | a1 | B03 | b2 | C03 | c1 | D01 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0114 | 702.226 | OH | a1 | B01 | b2 | C05 | c1 | D01 | d1 | E03 |
| 2-1-m1E03-1-0115 | 703.221 | OH | a1 | B02 | b2 | C05 | c1 | D01 | d1 | E03 |
| 2-1-m1E03-1-0116 | 704.176 | OH | a2 | B16 | b2 | C06 | c1 | D01 | d1 | E03 |
| 2-1-m1E03-1-0117 | 704.176 | OH | a2 | B11 | b3 | C08 | c1 | D01 | d1 | E03 |
| 2-1-m1E03-1-0118 | 704.176 | OH | a2 | B11 | b1 | C08 | c2 | D02 | d1 | E03 |
| 2-1-m1E03-1-0119 | 704.190 | OH | a2 | B17 | b2 | C02 | c1 | D01 | d1 | E03 |
| 2-1-m1E03-1-0120 | 704.190 | OH | a2 | B14 | b2 | C04 | c1 | D01 | d1 | E03 |
| 2-1-m1E03-1-0121 | 705.172 | OH | a2 | B12 | b3 | C08 | c1 | D01 | d1 | E03 |
| 2-1-m1E03-1-0122 | 705.172 | OH | a2 | B12 | b1 | C08 | c2 | D02 | d1 | E03 |
| 2-1-m1E03-1-0123 | 705.172 | OH | a2 | B11 | b1 | C11 | c2 | D02 | d1 | E03 |
| 2-1-m1E03-1-0124 | 705.186 | OH | a2 | B17 | b2 | C03 | c1 | D01 | d1 | E03 |
| 2-1-m1E03-1-0125 | 705.206 | OH | a1 | B04 | b2 | C02 | c1 | D01 | d1 | E03 |
| 2-1-m1E03-1-0126 | 705.206 | OH | a1 | B01 | b2 | C04 | c1 | D01 | d1 | E03 |
| 2-1-m1E03-1-0127 | 705.226 | OH | a1 | B05 | b2 | C01 | c1 | D01 | d1 | E03 |
| 2-1-m1E03-1-0128 | 706.167 | OH | a2 | B11 | b1 | C10 | c2 | D02 | d1 | E03 |
| 2-1-m1E03-1-0129 | 706.167 | OH | a2 | B12 | b1 | C11 | c2 | D02 | d1 | E03 |
| 2-1-m1E03-1-0130 | 706.201 | OH | a1 | B02 | b2 | C04 | c1 | D01 | d1 | E03 |
| 2-1-m1E03-1-0131 | 706.201 | OH | a1 | B04 | b2 | C03 | c1 | D01 | d1 | E03 |
| 2-1-m1E03-1-0132 | 706.236 | OH | a2 | B13 | b1 | C13 | c1 | D01 | d1 | E03 |
| 2-1-m1E03-1-0133 | 707.162 | OH | a2 | B12 | b1 | C10 | c2 | D02 | d1 | E03 |
| 2-1-m1E03-1-0134 | 707.187 | OH | a1 | B03 | b2 | C06 | c1 | D01 | d1 | E03 |
| 2-1-m1E03-1-0135 | 707.205 | OH | a1 | B06 | b2 | C01 | c1 | D01 | d1 | E03 |
| 2-1-m1E03-1-0136 | 708.171 | OH | a2 | B13 | b3 | C15 | c1 | D01 | d1 | E03 |
| 2-1-m1E03-1-0137 | 708.202 | OH | a2 | B19 | b1 | C08 | c1 | D01 | d1 | E03 |
| 2-1-m1E03-1-0138 | 708.202 | OH | a2 | B11 | b1 | C09 | c1 | D01 | d1 | E03 |
| 2-1-m1E03-1-0139 | 708.204 | OH | a2 | B18 | b2 | C01 | c1 | D01 | d1 | E03 |
| 2-1-m1E03-1-0140 | 709.197 | OH | a2 | B12 | b1 | C09 | c1 | D01 | d1 | E03 |
| 2-1-m1E03-1-0141 | 709.197 | OH | a2 | B19 | b1 | C11 | c1 | D01 | d1 | E03 |
| 2-1-m1E03-1-0142 | 710.133 | OH | a2 | B11 | b3 | C06 | c1 | D01 | d1 | E03 |
| 2-1-m1E03-1-0143 | 710.147 | OH | a2 | B17 | b2 | C06 | c1 | D01 | d1 | E03 |
| 2-1-m1E03-1-0144 | 710.192 | OH | a2 | B19 | b1 | C10 | c1 | D01 | d1 | E03 |
| 2-1-m1E03-1-0145 | 710.202 | OH | a2 | B13 | b1 | C14 | c1 | D01 | d1 | E03 |
| 2-1-m1E03-1-0146 | 710.211 | OH | a2 | B14 | b1 | C13 | c1 | D01 | d1 | E03 |
| 2-1-m1E03-1-0147 | 710.223 | OH | a2 | B13 | b2 | C33 | c3 | D02 | d1 | E03 |
| 2-1-m1E03-1-0148 | 711.128 | OH | a2 | B12 | b3 | C06 | c1 | D01 | d1 | E03 |
| 2-1-m1E03-1-0149 | 711.128 | OH | a2 | B11 | b1 | C12 | c2 | D02 | d1 | E03 |
| 2-1-m1E03-1-0150 | 711.162 | OH | a1 | B04 | b2 | C06 | c1 | D01 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0151 | 711.226 | OH | a1 | B01 | b1 | C13 | c1 | D01 | d1 | E03 |
| 2-1-m1E03-1-0152 | 711.252 | OH | a2 | B15 | b2 | C05 | c1 | D01 | d1 | E03 |
| 2-1-m1E03-1-0153 | 712.123 | OH | a2 | B12 | b1 | C12 | c2 | D02 | d1 | E03 |
| 2-1-m1E03-1-0154 | 712.146 | OH | a2 | B14 | b3 | C15 | c1 | D01 | d1 | E03 |
| 2-1-m1E03-1-0155 | 712.222 | OH | a1 | B02 | b1 | C13 | c1 | D01 | d1 | E03 |
| 2-1-m1E03-1-0156 | 713.161 | OH | a1 | B01 | b3 | C15 | c1 | D01 | d1 | E03 |
| 2-1-m1E03-1-0157 | 713.176 | OH | a1 | B07 | b2 | C01 | c1 | D01 | d1 | E03 |
| 2-1-m1E03-1-0158 | 713.231 | OH | a2 | B16 | b2 | C05 | c1 | D01 | d1 | E03 |
| 2-1-m1E03-1-0159 | 714.157 | OH | a1 | B02 | b3 | C15 | c1 | D01 | d1 | E03 |
| 2-1-m1E03-1-0160 | 714.177 | OH | a2 | B14 | b1 | C14 | c1 | D01 | d1 | E03 |
| 2-1-m1E03-1-0161 | 714.198 | OH | a2 | B14 | b2 | C33 | c3 | D02 | d1 | E03 |
| 2-1-m1E03-1-0162 | 714.231 | OH | a2 | B15 | b2 | C04 | c1 | D01 | d1 | E03 |
| 2-1-m1E03-1-0163 | 714.288 | OH | a2 | B13 | b2 | C07 | c1 | D01 | d1 | E03 |
| 2-1-m1E03-1-0164 | 715.153 | OH | a2 | B19 | b1 | C12 | c1 | D01 | d1 | E03 |
| 2-1-m1E03-1-0165 | 715.192 | OH | a1 | B01 | b1 | C14 | c1 | D01 | d1 | E03 |
| 2-1-m1E03-1-0166 | 715.213 | OH | a1 | B01 | b2 | C33 | c3 | D02 | d1 | E03 |
| 2-1-m1E03-1-0167 | 715.246 | OH | a1 | B05 | b2 | C02 | c1 | D01 | d1 | E03 |
| 2-1-m1E03-1-168 | 716.188 | OH | a1 | B08 | b1 | C08 | c1 | D01 | d1 | E03 |
| 2-1-m1E03-1-0169 | 716.188 | OH | a1 | B02 | b1 | C14 | c1 | D01 | d1 | E03 |
| 2-1-m1E03-1-0170 | 716.209 | OH | a1 | B02 | b2 | C33 | c3 | D02 | d1 | E03 |
| 2-1-m1E03-1-0171 | 716.210 | OH | a2 | B16 | b2 | C04 | c1 | D01 | d1 | E03 |
| 2-1-m1E03-1-0172 | 716.213 | OH | a2 | B11 | b1 | C30 | c3 | D02 | d1 | E03 |
| 2-1-m1E03-1-0173 | 716.242 | OH | a1 | B05 | b2 | C03 | c1 | D01 | d1 | E03 |
| 2-1-m1E03-1-0174 | 716.242 | OH | a1 | B03 | b2 | C05 | c1 | D01 | d1 | E03 |
| 2-1-m1E03-1-0175 | 717.183 | OH | a1 | B08 | b1 | C11 | c1 | D01 | d1 | E03 |
| 2-1-m1E03-1-0176 | 717.208 | OH | a2 | B12 | b1 | C30 | c3 | D02 | d1 | E03 |
| 2-1-m1E03-1-0177 | 717.226 | OH | a1 | B06 | b2 | C02 | c1 | D01 | d1 | E03 |
| 2-1-m1E03-1-0178 | 718.178 | OH | a1 | B08 | b1 | C10 | c1 | D01 | d1 | E03 |
| 2-1-m1E03-1-0179 | 718.192 | OH | a2 | B13 | b3 | C08 | c1 | D01 | d1 | E03 |
| 2-1-m1E03-1-0180 | 718.192 | OH | a2 | B13 | b1 | C08 | c2 | D02 | d1 | E03 |
| 2-1-m1E03-1-0181 | 718.192 | OH | a2 | B11 | b2 | C20 | c3 | D02 | d1 | E03 |
| 2-1-m1E03-1-0182 | 718.221 | OH | a1 | B06 | b2 | C03 | c1 | D01 | d1 | E03 |
| 2-1-m1E03-1-0183 | 718.225 | OH | a2 | B18 | b2 | C02 | c1 | D01 | d1 | E03 |
| 2-1-m1E03-1-0184 | 718.263 | OH | a2 | B14 | b2 | C07 | c1 | D01 | d1 | E03 |
| 2-1-m1E03-1-0185 | 719.187 | OH | a2 | B13 | b1 | C11 | c2 | D02 | d1 | E03 |
| 2-1-m1E03-1-0186 | 719.187 | OH | a2 | B12 | b2 | C20 | c3 | D02 | d1 | E03 |
| 2-1-m1E03-1-0187 | 719.187 | OH | a2 | B11 | b2 | C28 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0188 | 719.201 | OH | a2 | B17 | b2 | C05 | c1 | D01 | d1 | E03 |
| 2-1-m1E03-1-0189 | 719.220 | OH | a2 | B18 | b2 | C03 | c1 | D01 | d1 | E03 |
| 2-1-m1E03-1-0190 | 719.221 | OH | a1 | B03 | b2 | C04 | c1 | D01 | d1 | E03 |
| 2-1-m1E03-1-0191 | 719.278 | OH | a1 | B01 | b2 | C07 | c1 | D01 | d1 | E03 |
| 2-1-m1E03-1-0192 | 720.182 | OH | a2 | B13 | b1 | C10 | c2 | D02 | d1 | E03 |
| 2-1-m1E03-1-0193 | 720.182 | OH | a2 | B12 | b2 | C28 | c3 | D02 | d1 | E03 |
| 2-1-m1E03-1-0194 | 720.217 | OH | a1 | B04 | b2 | C05 | c1 | D01 | d1 | E03 |
| 2-1-m1E03-1-0195 | 720.252 | OH | a2 | B15 | b1 | C13 | c1 | D01 | d1 | E03 |
| 2-1-m1E03-1-0196 | 720.273 | OH | a1 | B02 | b2 | C07 | c1 | D01 | d1 | E03 |
| 2-1-m1E03-1-0197 | 721.203 | OH | a1 | B05 | b2 | C06 | c1 | D01 | d1 | E03 |
| 2-1-m1E03-1-0198 | 722.151 | OH | a2 | B11 | b2 | C01 | c2 | D02 | d1 | E03 |
| 2-1-m1E03-1-0199 | 722.167 | OH | a2 | B14 | b3 | C08 | c1 | D01 | d1 | E03 |
| 2-1-m1E03-1-0200 | 722.167 | OH | a2 | B14 | b1 | C08 | c2 | D02 | d1 | E03 |
| 2-1-m1E03-1-0201 | 722.181 | OH | a2 | B17 | b2 | C04 | c1 | D01 | d1 | E03 |
| 2-1-m1E03-1-0202 | 722.187 | OH | a2 | B15 | b3 | C15 | c1 | D01 | d1 | E03 |
| 2-1-m1E03-1-0203 | 722.217 | OH | a2 | B13 | b1 | C09 | c1 | D01 | d1 | E03 |
| 2-1-m1E03-1-0204 | 722.231 | OH | a2 | B16 | b1 | C13 | c1 | D01 | d1 | E03 |
| 2-1-m1E03-1-0205 | 723.139 | OH | a1 | B08 | b1 | C12 | c1 | D01 | d1 | E03 |
| 2-1-m1E03-1-0206 | 723.146 | OH | a2 | B12 | b2 | C01 | c2 | D02 | d1 | E03 |
| 2-1-m1E03-1-0207 | 723.162 | OH | a2 | B14 | b1 | C11 | c2 | D02 | d1 | E03 |
| 2-1-m1E03-1-0208 | 723.182 | OH | a1 | B06 | b2 | C06 | c1 | D01 | d1 | E03 |
| 2-1-m1E03-1-0209 | 723.182 | OH | a1 | B01 | b3 | C08 | c1 | D01 | d1 | E03 |
| 2-1-m1E03-1-0210 | 723.182 | OH | a1 | B01 | b1 | C08 | c2 | D02 | d1 | E03 |
| 2-1-m1E03-1-0211 | 723.196 | OH | a1 | B07 | b2 | C02 | c1 | D01 | d1 | E03 |
| 2-1-m1E03-1-0212 | 723.196 | OH | a1 | B04 | b2 | C04 | c1 | D01 | d1 | E03 |
| 2-1-m1E03-1-0213 | 724.148 | OH | a2 | B13 | b3 | C06 | c1 | D01 | d1 | E03 |
| 2-1-m1E03-1-0214 | 724.157 | OH | a2 | B14 | b1 | C10 | c2 | D02 | d1 | E03 |
| 2-1-m1E03-1-0215 | 724.166 | OH | a2 | B16 | b3 | C15 | c1 | D01 | d1 | E03 |
| 2-1-m1E03-1-0216 | 724.177 | OH | a1 | B02 | b3 | C08 | c1 | D01 | d1 | E03 |
| 2-1-m1E03-1-0217 | 724.177 | OH | a1 | B02 | b1 | C08 | c2 | D02 | d1 | E03 |
| 2-1-m1E03-1-0218 | 724.177 | OH | a1 | B01 | b1 | C11 | c2 | D02 | d1 | E03 |
| 2-1-m1E03-1-0219 | 724.181 | OH | a2 | B18 | b2 | C06 | c1 | D01 | d1 | E03 |
| 2-1-m1E03-1-0220 | 724.192 | OH | a1 | B07 | b2 | C03 | c1 | D01 | d1 | E03 |
| 2-1-m1E03-1-0221 | 724.197 | OH | a2 | B20 | b1 | C08 | c1 | D01 | d1 | E03 |
| 2-1-m1E03-1-0222 | 724.218 | OH | a2 | B15 | b1 | C14 | c1 | D01 | d1 | E03 |
| 2-1-m1E03-1-0223 | 724.239 | OH | a2 | B15 | b2 | C33 | c3 | D02 | d1 | E03 |
| 2-1-m1E03-1-0224 | 725.144 | OH | a2 | B13 | b1 | C12 | c2 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0225 | 725.173 | OH | a1 | B01 | b1 | C10 | c2 | D02 | d1 | E03 |
| 2-1-m1E03-1-0226 | 725.173 | OH | a1 | B02 | b1 | C11 | c2 | D02 | d1 | E03 |
| 2-1-m1E03-1-0227 | 725.192 | OH | a2 | B20 | b1 | C11 | c1 | D01 | d1 | E03 |
| 2-1-m1E03-1-0228 | 725.242 | OH | a1 | B03 | b1 | C13 | c1 | D01 | d1 | E03 |
| 2-1-m1E03-1-0229 | 726.168 | OH | a1 | B02 | b1 | C10 | c2 | D02 | d1 | E03 |
| 2-1-m1E03-1-0230 | 726.176 | OH | a2 | B19 | b2 | C01 | c1 | D01 | d1 | E03 |
| 2-1-m1E03-1-0231 | 726.187 | OH | a2 | B20 | b1 | C10 | c1 | D01 | d1 | E03 |
| 2-1-m1E03-1-0232 | 726.192 | OH | a2 | B14 | b1 | C09 | c1 | D01 | d1 | E03 |
| 2-1-m1E03-1-0233 | 726.197 | OH | a2 | B16 | b1 | C14 | c1 | D01 | d1 | E03 |
| 2-1-m1E03-1-0234 | 726.218 | OH | a2 | B16 | b2 | C33 | c3 | D02 | d1 | E03 |
| 2-1-m1E03-1-0235 | 727.177 | OH | a1 | B03 | b3 | C15 | c1 | D01 | d1 | E03 |
| 2-1-m1E03-1-0236 | 727.208 | OH | a1 | B09 | b1 | C08 | c1 | D01 | d1 | E03 |
| 2-1-m1E03-1-0237 | 727.208 | OH | a1 | B01 | b1 | C09 | c1 | D01 | d1 | E03 |
| 2-1-m1E03-1-0238 | 728.123 | OH | a2 | B14 | b3 | C06 | c1 | D01 | d1 | E03 |
| 2-1-m1E03-1-0239 | 728.202 | OH | a2 | B17 | b1 | C13 | c1 | D01 | d1 | E03 |
| 2-1-m1E03-1-0240 | 728.203 | OH | a1 | B02 | b1 | C09 | c1 | D01 | d1 | E03 |
| 2-1-m1E03-1-0241 | 728.203 | OH | a1 | B09 | b1 | C11 | c1 | D01 | d1 | E03 |
| 2-1-m1E03-1-0242 | 728.303 | OH | a2 | B15 | b2 | C07 | c1 | D01 | d1 | E03 |
| 2-1-m1E03-1-0243 | 729.119 | OH | a2 | B14 | b1 | C12 | c2 | D02 | d1 | E03 |
| 2-1-m1E03-1-0244 | 729.139 | OH | a1 | B01 | b3 | C06 | c1 | D01 | d1 | E03 |
| 2-1-m1E03-1-0245 | 729.153 | OH | a1 | B07 | b2 | C06 | c1 | D01 | d1 | E03 |
| 2-1-m1E03-1-0246 | 729.198 | OH | a1 | B09 | b1 | C10 | c1 | D01 | d1 | E03 |
| 2-1-m1E03-1-0247 | 729.208 | OH | a1 | B03 | b1 | C14 | c1 | D01 | d1 | E03 |
| 2-1-m1E03-1-0248 | 729.217 | OH | a1 | B04 | b1 | C13 | c1 | D01 | d1 | E03 |
| 2-1-m1E03-1-0249 | 729.229 | OH | a1 | B03 | b2 | C33 | c3 | D02 | d1 | E03 |
| 2-1-m1E03-1-0250 | 730.134 | OH | a1 | B02 | b3 | C06 | c1 | D01 | d1 | E03 |
| 2-1-m1E03-1-0251 | 730.134 | OH | a1 | B01 | b1 | C12 | c2 | D02 | d1 | E03 |
| 2-1-m1E03-1-0252 | 730.137 | OH | a2 | B17 | b3 | C15 | c1 | D01 | d1 | E03 |
| 2-1-m1E03-1-0253 | 730.228 | OH | a2 | B13 | b1 | C30 | c3 | D02 | d1 | E03 |
| 2-1-m1E03-1-0254 | 730.228 | OH | a2 | B11 | b1 | C32 | c3 | D02 | d1 | E03 |
| 2-1-m1E03-1-0255 | 730.257 | OH | a1 | B05 | b2 | COS | c1 | D01 | d1 | E03 |
| 2-1-m1E03-1-0256 | 730.283 | OH | a2 | B16 | b2 | C07 | c1 | D01 | d1 | E03 |
| 2-1-m1E03-1-0257 | 731.129 | OH | a1 | B02 | b1 | C12 | c2 | D02 | d1 | E03 |
| 2-1-m1E03-1-0258 | 731.148 | OH | a2 | B20 | b1 | C12 | c1 | D01 | d1 | E03 |
| 2-1-m1E03-1-0259 | 731.152 | OH | a1 | B04 | b3 | C15 | c1 | D01 | d1 | E03 |
| 2-1-m1E03-1-0260 | 731.224 | OH | a2 | B12 | b1 | C32 | c3 | D02 | d1 | E03 |
| 2-1-m1E03-1-0261 | 732.168 | OH | a2 | B17 | b1 | C14 | c1 | D01 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0262 | 732.171 | OH | a2 | B11 | b2 | C02 | c2 | D02 | d1 | E03 |
| 2-1-m1E03-1-0263 | 732.189 | OH | a2 | B17 | b2 | C33 | c3 | D02 | d1 | E03 |
| 2-1-m1E03-1-0264 | 732.208 | OH | a2 | B15 | b3 | C08 | c1 | D01 | d1 | E03 |
| 2-1-m1E03-1-0265 | 732.208 | OH | a2 | B15 | b1 | C08 | c2 | D02 | d1 | E03 |
| 2-1-m1E03-1-0266 | 732.208 | OH | a2 | B13 | b2 | C20 | c3 | D02 | d1 | E03 |
| 2-1-m1E03-1-0267 | 732.208 | OH | a2 | B11 | b2 | C22 | c3 | D02 | d1 | E03 |
| 2-1-m1E03-1-0268 | 732.237 | OH | a1 | B06 | b2 | COS | c1 | D01 | d1 | E03 |
| 2-1-m1E03-1-0269 | 733.166 | OH | a2 | B12 | b2 | C02 | c2 | D02 | d1 | E03 |
| 2-1-m1E03-1-0270 | 733.166 | OH | a2 | B11 | b2 | C03 | c2 | D02 | d1 | E03 |
| 2-1-m1E03-1-0271 | 733.183 | OH | a1 | B04 | b1 | C14 | c1 | D01 | d1 | E03 |
| 2-1-m1E03-1-0272 | 733.203 | OH | a2 | B15 | b1 | C11 | c2 | D02 | d1 | E03 |
| 2-1-m1E03-1-0273 | 733.203 | OH | a2 | B12 | b2 | C22 | c3 | D02 | d1 | E03 |
| 2-1-m1E03-1-0274 | 733.203 | OH | a2 | B13 | b2 | C28 | c3 | D02 | d1 | E03 |
| 2-1-m1E03-1-0275 | 733.204 | OH | a1 | B04 | b2 | C33 | c3 | D02 | d1 | E03 |
| 2-1-m1E03-1-0276 | 733.236 | OH | a2 | B18 | b2 | COS | c1 | D01 | d1 | E03 |
| 2-1-m1E03-1-0277 | 733.237 | OH | a1 | B05 | b2 | C04 | c1 | D01 | d1 | E03 |
| 2-1-m1E03-1-0278 | 733.293 | OH | a1 | B03 | b2 | C07 | c1 | D01 | d1 | E03 |
| 2-1-m1E03-1-0279 | 734.159 | OH | a1 | B09 | b1 | C12 | c1 | D01 | d1 | E03 |
| 2-1-m1E03-1-0280 | 734.162 | OH | a1 | B08 | b2 | C01 | c1 | D01 | d1 | E03 |
| 2-1-m1E03-1-0281 | 734.162 | OH | a2 | B12 | b2 | C03 | c2 | D02 | d1 | E03 |
| 2-1-m1E03-1-0282 | 734.187 | OH | a2 | B16 | b3 | C08 | c1 | D01 | d1 | E03 |
| 2-1-m1E03-1-0283 | 734.187 | OH | a2 | B16 | b1 | C08 | c2 | D02 | d1 | E03 |
| 2-1-m1E03-1-0284 | 734.198 | OH | a2 | B15 | b1 | C10 | c2 | D02 | d1 | E03 |
| 2-1-m1E03-1-0285 | 734.203 | OH | a2 | B14 | b1 | C30 | c3 | D02 | d1 | E03 |
| 2-1-m1E03-1-0286 | 735.182 | OH | a2 | B16 | b1 | C11 | c2 | D02 | d1 | E03 |
| 2-1-m1E03-1-0287 | 735.216 | OH | a1 | B06 | b2 | C04 | c1 | D01 | d1 | E03 |
| 2-1-m1E03-1-0288 | 735.219 | OH | a1 | B01 | b1 | C30 | c3 | D02 | d1 | E03 |
| 2-1-m1E03-1-0289 | 736.166 | OH | a2 | B13 | b2 | C01 | c2 | D02 | d1 | E03 |
| 2-1-m1E03-1-0290 | 736.177 | OH | a2 | B16 | b1 | C10 | c2 | D02 | d1 | E03 |
| 2-1-m1E03-1-0291 | 736.183 | OH | a2 | B14 | b2 | C20 | c3 | D02 | d1 | E03 |
| 2-1-m1E03-1-0292 | 736.183 | OH | a2 | B11 | b2 | C24 | c3 | D02 | d1 | E03 |
| 2-1-m1E03-1-0293 | 736.197 | OH | a2 | B19 | b2 | C02 | c1 | D01 | d1 | E03 |
| 2-1-m1E03-1-0294 | 736.214 | OH | a1 | B02 | b1 | C30 | c3 | D02 | d1 | E03 |
| 2-1-m1E03-1-0295 | 736.216 | OH | a2 | B18 | b2 | C04 | c1 | D01 | d1 | E03 |
| 2-1-m1E03-1-0296 | 736.233 | OH | a2 | B15 | b1 | C09 | c1 | D01 | d1 | E03 |
| 2-1-m1E03-1-0297 | 736.253 | OH | a2 | B17 | b2 | C07 | c1 | D01 | d1 | E03 |
| 2-1-m1E03-1-0298 | 737.178 | OH | a2 | B12 | b2 | C24 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0299 | 737.178 | OH | a2 | B14 | b2 | C28 | c3 | D02 | d1 | E03 |
| 2-1-m1E03-1-0300 | 737.192 | OH | a2 | B19 | b2 | C03 | c1 | D01 | d1 | E03 |
| 2-1-m1E03-1-0301 | 737.198 | OH | a1 | B03 | b3 | C08 | c1 | D01 | d1 | E03 |
| 2-1-m1E03-1-0302 | 737.198 | OH | a1 | B03 | b1 | C08 | c2 | D02 | d1 | E03 |
| 2-1-m1E03-1-0303 | 737.198 | OH | a1 | B01 | b2 | C20 | c3 | D02 | d1 | E03 |
| 2-1-m1E03-1-0304 | 737.268 | OH | a1 | B04 | b2 | C07 | c1 | D01 | d1 | E03 |
| 2-1-m1E03-1-0305 | 738.128 | OH | a2 | B11 | b2 | C06 | c2 | D02 | d1 | E03 |
| 2-1-m1E03-1-0306 | 738.164 | OH | a2 | B15 | b3 | C06 | c1 | D01 | d1 | E03 |
| 2-1-m1E03-1-0307 | 738.193 | OH | a1 | B03 | b1 | C11 | c2 | D02 | d1 | E03 |
| 2-1-m1E03-1-0308 | 738.193 | OH | a1 | B02 | b2 | C20 | c3 | D02 | d1 | E03 |
| 2-1-m1E03-1-0309 | 738.193 | OH | a1 | B01 | b2 | C28 | c3 | D02 | d1 | E03 |
| 2-1-m1E03-1-0310 | 738.207 | OH | a1 | B07 | b2 | C05 | c1 | D01 | d1 | E03 |
| 2-1-m1E03-1-0311 | 738.212 | OH | a2 | B16 | b1 | C09 | c1 | D01 | d1 | E03 |
| 2-1-m1E03-1-0312 | 739.123 | OH | a2 | B12 | b2 | C06 | c2 | D02 | d1 | E03 |
| 2-1-m1E03-1-0313 | 739.159 | OH | a2 | B15 | b1 | C12 | c2 | D02 | d1 | E03 |
| 2-1-m1E03-1-0314 | 739.188 | OH | a1 | B03 | b1 | C10 | c2 | D02 | d1 | E03 |
| 2-1-m1E03-1-0315 | 739.188 | OH | a1 | B02 | b2 | C28 | c3 | D02 | d1 | E03 |
| 2-1-m1E03-1-0316 | 739.258 | OH | a1 | B05 | b1 | C13 | c1 | D01 | d1 | E03 |
| 2-1-m1E03-1-0317 | 740.141 | OH | a2 | B14 | b2 | C01 | c2 | D02 | d1 | E03 |
| 2-1-m1E03-1-0318 | 740.143 | OH | a2 | B16 | b3 | C06 | c1 | D01 | d1 | E03 |
| 2-1-m1E03-1-0319 | 740.157 | OH | a2 | B17 | b3 | C08 | c1 | D01 | d1 | E03 |
| 2-1-m1E03-1-0320 | 740.157 | OH | a2 | B17 | b1 | C08 | c2 | D02 | d1 | E03 |
| 2-1-m1E03-1-0321 | 741.139 | OH | a2 | B16 | b1 | C12 | c2 | D02 | d1 | E03 |
| 2-1-m1E03-1-0322 | 741.153 | OH | a2 | B17 | b1 | C11 | c2 | D02 | d1 | E03 |
| 2-1-m1E03-1-0323 | 741.156 | OH | a1 | B01 | b2 | C01 | c2 | D02 | d1 | E03 |
| 2-1-m1E03-1-0324 | 741.173 | OH | a1 | B04 | b3 | C08 | c1 | D01 | d1 | E03 |
| 2-1-m1E03-1-0325 | 741.173 | OH | a1 | B04 | b1 | C08 | c2 | D02 | d1 | E03 |
| 2-1-m1E03-1-0326 | 741.187 | OH | a1 | B07 | b2 | C04 | c1 | D01 | d1 | E03 |
| 2-1-m1E03-1-0327 | 741.193 | OH | a1 | B05 | b3 | C15 | c1 | D01 | d1 | E03 |
| 2-1-m1E03-1-0328 | 741.223 | OH | a1 | B03 | b1 | C09 | c1 | D01 | d1 | E03 |
| 2-1-m1E03-1-0329 | 741.237 | OH | a1 | B06 | b1 | C13 | c1 | D01 | d1 | E03 |
| 2-1-m1E03-1-0330 | 742.148 | OH | a2 | B17 | b1 | C10 | c2 | D02 | d1 | E03 |
| 2-1-m1E03-1-0331 | 742.152 | OH | a1 | B02 | b2 | C01 | c2 | D02 | d1 | E03 |
| 2-1-m1E03-1-0332 | 742.153 | OH | a2 | B19 | b2 | C06 | c1 | D01 | d1 | E03 |
| 2-1-m1E03-1-0333 | 742.168 | OH | a1 | B04 | b1 | C11 | c2 | D02 | d1 | E03 |
| 2-1-m1E03-1-0334 | 742.171 | OH | a2 | B20 | b2 | C01 | c1 | D01 | d1 | E03 |
| 2-1-m1E03-1-0335 | 742.236 | OH | a2 | B18 | b1 | C13 | c1 | D01 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0336 | 743.154 | OH | a1 | B03 | b3 | C06 | c1 | D01 | d1 | E03 |
| 2-1-m1E03-1-0337 | 743.163 | OH | a1 | B04 | b1 | C10 | c2 | D02 | d1 | E03 |
| 2-1-m1E03-1-0338 | 743.172 | OH | a1 | B06 | b3 | C15 | c1 | D01 | d1 | E03 |
| 2-1-m1E03-1-0339 | 743.203 | OH | a1 | B10 | b1 | C08 | c1 | D01 | d1 | E03 |
| 2-1-m1E03-1-0340 | 743.224 | OH | a1 | B05 | b1 | C14 | c1 | D01 | d1 | E03 |
| 2-1-m1E03-1-0341 | 743.245 | OH | a1 | B05 | b2 | C33 | c3 | D02 | d1 | E03 |
| 2-1-m1E03-1-0342 | 744.149 | OH | a1 | B03 | b1 | C12 | c2 | D02 | d1 | E03 |
| 2-1-m1E03-1-0343 | 744.171 | OH | a2 | B18 | b3 | C15 | c1 | D01 | d1 | E03 |
| 2-1-m1E03-1-0344 | 744.182 | OH | a1 | B08 | b2 | C02 | c1 | D01 | d1 | E03 |
| 2-1-m1E03-1-0345 | 744.183 | OH | a2 | B17 | b1 | C09 | c1 | D01 | d1 | E03 |
| 2-1-m1E03-1-0346 | 744.198 | OH | a1 | B10 | b1 | C11 | c1 | D01 | d1 | E03 |
| 2-1-m1E03-1-0347 | 744.244 | OH | a2 | B15 | b1 | C30 | c3 | D02 | d1 | E03 |
| 2-1-m1E03-1-0348 | 744.244 | OH | a2 | B11 | b1 | C29 | c3 | D02 | d1 | E03 |
| 2-1-m1E03-1-0349 | 744.244 | OH | a2 | B13 | b1 | C32 | c3 | D02 | d1 | E03 |
| 2-1-m1E03-1-0350 | 745.178 | OH | a1 | B08 | b2 | C03 | c1 | D01 | d1 | E03 |
| 2-1-m1E03-1-0351 | 745.182 | OH | a1 | B09 | b2 | C01 | c1 | D01 | d1 | E03 |
| 2-1-m1E03-1-0352 | 745.193 | OH | a1 | B10 | b1 | C10 | c1 | D01 | d1 | E03 |
| 2-1-m1E03-1-0353 | 745.198 | OH | a1 | B04 | b1 | C09 | c1 | D01 | d1 | E03 |
| 2-1-m1E03-1-0354 | 745.203 | OH | a1 | B06 | b1 | C14 | c1 | D01 | d1 | E03 |
| 2-1-m1E03-1-0355 | 745.224 | OH | a1 | B06 | b2 | C33 | c3 | D02 | d1 | E03 |
| 2-1-m1E03-1-0356 | 745.239 | OH | a2 | B12 | b1 | C29 | c3 | D02 | d1 | E03 |
| 2-1-m1E03-1-0357 | 746.114 | OH | a2 | B17 | b3 | C06 | c1 | D01 | d1 | E03 |
| 2-1-m1E03-1-0358 | 746.187 | OH | a2 | B13 | b2 | C02 | c2 | D02 | d1 | E03 |
| 2-1-m1E03-1-0359 | 746.202 | OH | a2 | B18 | b1 | C14 | c1 | D01 | d1 | E03 |
| 2-1-m1E03-1-0360 | 746.223 | OH | a2 | B15 | b2 | C20 | c3 | D02 | d1 | E03 |
| 2-1-m1E03-1-0361 | 746.223 | OH | a2 | B13 | b2 | C22 | c3 | D02 | d1 | E03 |
| 2-1-m1E03-1-0362 | 746.223 | OH | a2 | B16 | b1 | C30 | c3 | D02 | d1 | E03 |
| 2-1-m1E03-1-0363 | 746.223 | OH | a2 | B18 | b2 | C33 | c3 | D02 | d1 | E03 |
| 2-1-m1E03-1-0364 | 747.109 | OH | a2 | B17 | b1 | C12 | c2 | D02 | d1 | E03 |
| 2-1-m1E03-1-0365 | 747.129 | OH | a1 | B04 | b3 | C06 | c1 | D01 | d1 | E03 |
| 2-1-m1E03-1-0366 | 747.182 | OH | a2 | B13 | b2 | C03 | c2 | D02 | d1 | E03 |
| 2-1-m1E03-1-0367 | 747.182 | OH | a2 | B11 | b2 | C05 | c2 | D02 | d1 | E03 |
| 2-1-m1E03-1-0368 | 747.208 | OH | a1 | B07 | b1 | C13 | c1 | D01 | d1 | E03 |
| 2-1-m1E03-1-0369 | 747.219 | OH | a2 | B15 | b2 | C28 | c3 | D02 | d1 | E03 |
| 2-1-m1E03-1-0370 | 747.309 | OH | a1 | B05 | b2 | C07 | c1 | D01 | d1 | E03 |
| 2-1-m1E03-1-0371 | 748.124 | OH | a1 | B04 | b1 | C12 | c2 | D02 | d1 | E03 |
| 2-1-m1E03-1-0372 | 748.177 | OH | a2 | B12 | b2 | C05 | c2 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0373 | 748.203 | OH | a2 | B16 | b2 | C20 | c3 | D02 | d1 | E03 |
| 2-1-m1E03-1-0374 | 748.203 | OH | a2 | B11 | b2 | C26 | c3 | D02 | d1 | E03 |
| 2-1-m1E03-1-0375 | 748.219 | OH | a2 | B14 | b1 | C32 | c3 | D02 | d1 | E03 |
| 2-1-m1E03-1-0376 | 749.143 | OH | a1 | B07 | b3 | C15 | c1 | D01 | d1 | E03 |
| 2-1-m1E03-1-0377 | 749.198 | OH | a2 | B12 | b2 | C26 | c3 | D02 | d1 | E03 |
| 2-1-m1E03-1-0378 | 749.198 | OH | a2 | B16 | b2 | C28 | c3 | D02 | d1 | E03 |
| 2-1-m1E03-1-0379 | 749.234 | OH | a1 | B03 | b1 | C30 | c3 | D02 | d1 | E03 |
| 2-1-m1E03-1-0380 | 749.234 | OH | a1 | B01 | b1 | C32 | c3 | D02 | d1 | E03 |
| 2-1-m1E03-1-0381 | 749.288 | OH | a1 | B06 | b2 | C07 | c1 | D01 | d1 | E03 |
| 2-1-m1E03-1-0382 | 750.139 | OH | a1 | B08 | b2 | C06 | c1 | D01 | d1 | E03 |
| 2-1-m1E03-1-0383 | 750.154 | OH | a1 | B10 | b1 | C12 | c1 | D01 | d1 | E03 |
| 2-1-m1E03-1-0384 | 750.162 | OH | a2 | B14 | b2 | C02 | c2 | D02 | d1 | E03 |
| 2-1-m1E03-1-0385 | 750.162 | OH | a2 | B11 | b2 | C04 | c2 | D02 | d1 | E03 |
| 2-1-m1E03-1-0386 | 750.182 | OH | a2 | B15 | b2 | C01 | c2 | D02 | d1 | E03 |
| 2-1-m1E03-1-0387 | 750.198 | OH | a2 | B14 | b2 | C22 | c3 | D02 | d1 | E03 |
| 2-1-m1E03-1-0388 | 750.198 | OH | a2 | B13 | b2 | C24 | c3 | D02 | d1 | E03 |
| 2-1-m1E03-1-0389 | 750.229 | OH | a1 | B02 | b1 | C32 | c3 | D02 | d1 | E03 |
| 2-1-m1E03-1-0390 | 750.288 | OH | a2 | B18 | b2 | C07 | c1 | D01 | d1 | E03 |
| 2-1-m1E03-1-0391 | 751.157 | OH | a2 | B12 | b2 | C04 | c2 | D02 | d1 | E03 |
| 2-1-m1E03-1-0392 | 751.157 | OH | a2 | B14 | b2 | C03 | c2 | D02 | d1 | E03 |
| 2-1-m1E03-1-0393 | 751.173 | OH | a1 | B07 | b1 | C14 | c1 | D01 | d1 | E03 |
| 2-1-m1E03-1-0394 | 751.177 | OH | a1 | B01 | b2 | C02 | c2 | D02 | d1 | E03 |
| 2-1-m1E03-1-0395 | 751.195 | OH | a1 | B07 | b2 | C33 | c3 | D02 | d1 | E03 |
| 2-1-m1E03-1-0396 | 751.208 | OH | a2 | B19 | b2 | C05 | c1 | D01 | d1 | E03 |
| 2-1-m1E03-1-0397 | 751.213 | OH | a1 | B05 | b3 | C08 | c1 | D01 | d1 | E03 |
| 2-1-m1E03-1-0398 | 751.213 | OH | a1 | B05 | b1 | C08 | c2 | D02 | d1 | E03 |
| 2-1-m1E03-1-0399 | 751.213 | OH | a1 | B03 | b2 | C20 | c3 | D02 | d1 | E03 |
| 2-1-m1E03-1-0400 | 751.213 | OH | a1 | B01 | b2 | C22 | c3 | D02 | d1 | E03 |
| 2-1-m1E03-1-0401 | 752.143 | OH | a2 | B13 | b2 | C06 | c2 | D02 | d1 | E03 |
| 2-1-m1E03-1-0402 | 752.161 | OH | a2 | B16 | b2 | C01 | c2 | D02 | d1 | E03 |
| 2-1-m1E03-1-0403 | 752.172 | OH | a1 | B02 | b2 | C02 | c2 | D02 | d1 | E03 |
| 2-1-m1E03-1-0404 | 752.172 | OH | a1 | B01 | b2 | C03 | c2 | D02 | d1 | E03 |
| 2-1-m1E03-1-0405 | 752.192 | OH | a2 | B20 | b2 | C02 | c1 | D01 | d1 | E03 |
| 2-1-m1E03-1-0406 | 752.194 | OH | a2 | B17 | b1 | C30 | c3 | D02 | d1 | E03 |
| 2-1-m1E03-1-0407 | 752.209 | OH | a1 | B05 | b1 | C11 | c2 | D02 | d1 | E03 |
| 2-1-m1E03-1-0408 | 752.209 | OH | a1 | B02 | b2 | C22 | c3 | D02 | d1 | E03 |
| 2-1-m1E03-1-0409 | 752.209 | OH | a1 | B03 | b2 | C28 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0410 | 753.168 | OH | a1 | B02 | b2 | C03 | c2 | D02 | d1 | E03 |
| 2-1-m1E03-1-0411 | 753.187 | OH | a2 | B20 | b2 | C03 | c1 | D01 | d1 | E03 |
| 2-1-m1E03-1-0412 | 753.193 | OH | a1 | B06 | b3 | C08 | c1 | D01 | d1 | E03 |
| 2-1-m1E03-1-0413 | 753.193 | OH | a1 | B06 | b1 | C08 | c2 | D02 | d1 | E03 |
| 2-1-m1E03-1-0414 | 753.204 | OH | a1 | B05 | b1 | C10 | c2 | D02 | d1 | E03 |
| 2-1-m1E03-1-0415 | 753.209 | OH | a1 | B04 | b1 | C30 | c3 | D02 | d1 | E03 |
| 2-1-m1E03-1-0416 | 754.173 | OH | a2 | B17 | b2 | C20 | c3 | D02 | d1 | E03 |
| 2-1-m1E03-1-0417 | 754.173 | OH | a2 | B14 | b2 | C24 | c3 | D02 | d1 | E03 |
| 2-1-m1E03-1-0418 | 754.187 | OH | a2 | B19 | b2 | C04 | c1 | D01 | d1 | E03 |
| 2-1-m1E03-1-0419 | 754.188 | OH | a1 | B06 | b1 | C11 | c2 | D02 | d1 | E03 |
| 2-1-m1E03-1-0420 | 754.192 | OH | a2 | B18 | b3 | C08 | c1 | D01 | d1 | E03 |
| 2-1-m1E03-1-0421 | 754.192 | OH | a2 | B18 | b1 | C08 | c2 | D02 | d1 | E03 |
| 2-1-m1E03-1-0422 | 755.168 | OH | a2 | B17 | b2 | C28 | c3 | D02 | d1 | E03 |
| 2-1-m1E03-1-0423 | 755.172 | OH | a1 | B03 | b2 | C01 | c2 | D02 | d1 | E03 |
| 2-1-m1E03-1-0424 | 755.183 | OH | a1 | B06 | b1 | C10 | c2 | D02 | d1 | E03 |
| 2-1-m1E03-1-0425 | 755.187 | OH | a2 | B18 | b1 | C11 | c2 | D02 | d1 | E03 |
| 2-1-m1E03-1-0426 | 755.188 | OH | a1 | B04 | b2 | C20 | c3 | D02 | d1 | E03 |
| 2-1-m1E03-1-0427 | 755.188 | OH | a1 | B01 | b2 | C24 | c3 | D02 | d1 | E03 |
| 2-1-m1E03-1-0428 | 755.203 | OH | a1 | B09 | b2 | C02 | c1 | D01 | d1 | E03 |
| 2-1-m1E03-1-0429 | 755.239 | OH | a1 | B05 | b1 | C09 | c1 | D01 | d1 | E03 |
| 2-1-m1E03-1-0430 | 755.259 | OH | a1 | B07 | b2 | C07 | c1 | D01 | d1 | E03 |
| 2-1-m1E03-1-0431 | 756.118 | OH | a2 | B14 | b2 | C06 | c2 | D02 | d1 | E03 |
| 2-1-m1E03-1-0432 | 756.182 | OH | a2 | B18 | b1 | C10 | c2 | D02 | d1 | E03 |
| 2-1-m1E03-1-0433 | 756.182 | OH | a2 | B11 | b1 | C13 | c2 | D02 | d1 | E03 |
| 2-1-m1E03-1-0434 | 756.184 | OH | a1 | B02 | b2 | C24 | c3 | D02 | d1 | E03 |
| 2-1-m1E03-1-0435 | 756.184 | OH | a1 | B04 | b2 | C28 | c3 | D02 | d1 | E03 |
| 2-1-m1E03-1-0436 | 756.198 | OH | a1 | B09 | b2 | C03 | c1 | D01 | d1 | E03 |
| 2-1-m1E03-1-0437 | 757.133 | OH | a1 | B01 | b2 | C06 | c2 | D02 | d1 | E03 |
| 2-1-m1E03-1-0438 | 757.170 | OH | a1 | B05 | b3 | C06 | c1 | D01 | d1 | E03 |
| 2-1-m1E03-1-0439 | 757.178 | OH | a2 | B12 | b1 | C13 | c2 | D02 | d1 | E03 |
| 2-1-m1E03-1-0440 | 757.218 | OH | a1 | B06 | b1 | C09 | c1 | D01 | d1 | E03 |
| 2-1-m1E03-1-0441 | 758.117 | OH | a2 | B11 | b3 | C15 | c2 | D02 | d1 | E03 |
| 2-1-m1E03-1-0442 | 758.129 | OH | a1 | B02 | b2 | C06 | c2 | D02 | d1 | E03 |
| 2-1-m1E03-1-0443 | 758.132 | OH | a2 | B17 | b2 | C01 | c2 | D02 | d1 | E03 |
| 2-1-m1E03-1-0444 | 758.148 | OH | a2 | B20 | b2 | C06 | c1 | D01 | d1 | E03 |
| 2-1-m1E03-1-0445 | 758.165 | OH | a1 | B05 | b1 | C12 | c2 | D02 | d1 | E03 |
| 2-1-m1E03-1-0446 | 758.187 | OH | a2 | B11 | b3 | C16 | c1 | D01 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0447 | 758.217 | OH | a2 | B18 | b1 | C09 | c1 | D01 | d1 | E03 |
| 2-1-m1E03-1-0448 | 758.260 | OH | a2 | B13 | b1 | C29 | c3 | D02 | d1 | E03 |
| 2-1-m1E03-1-0449 | 758.260 | OH | a2 | B15 | b1 | C32 | c3 | D02 | d1 | E03 |
| 2-1-m1E03-1-0450 | 759.113 | OH | a2 | B12 | b3 | C15 | c2 | D02 | d1 | E03 |
| 2-1-m1E03-1-0451 | 759.147 | OH | a1 | B04 | b2 | C01 | c2 | D02 | d1 | E03 |
| 2-1-m1E03-1-0452 | 759.149 | OH | a1 | B06 | b3 | C06 | c1 | D01 | d1 | E03 |
| 2-1-m1E03-1-0453 | 759.163 | OH | a1 | B07 | b3 | C08 | c1 | D01 | d1 | E03 |
| 2-1-m1E03-1-0454 | 759.163 | OH | a1 | B07 | b1 | C08 | c2 | D02 | d1 | E03 |
| 2-1-m1E03-1-0455 | 759.182 | OH | a2 | B12 | b3 | C16 | c1 | D01 | d1 | E03 |
| 2-1-m1E03-1-0456 | 759.193 | OH | a1 | B08 | b2 | C05 | c1 | D01 | d1 | E03 |
| 2-1-m1E03-1-0457 | 760.144 | OH | a1 | B06 | b1 | C12 | c2 | D02 | d1 | E03 |
| 2-1-m1E03-1-0458 | 760.148 | OH | a2 | B18 | b3 | C06 | c1 | D01 | d1 | E03 |
| 2-1-m1E03-1-0459 | 760.148 | OH | a2 | B11 | b3 | C17 | c1 | D01 | d1 | E03 |
| 2-1-m1E03-1-0460 | 760.148 | OH | a2 | B11 | b1 | C14 | c2 | D02 | d1 | E03 |
| 2-1-m1E03-1-0461 | 760.159 | OH | a1 | B07 | b1 | C11 | c2 | D02 | d1 | E03 |
| 2-1-m1E03-1-0462 | 760.203 | OH | a2 | B15 | b2 | C02 | c2 | D02 | d1 | E03 |
| 2-1-m1E03-1-0463 | 760.208 | OH | a2 | B19 | b1 | C13 | c1 | D01 | d1 | E03 |
| 2-1-m1E03-1-0464 | 760.239 | OH | a2 | B15 | b2 | C22 | c3 | D02 | d1 | E03 |
| 2-1-m1E03-1-0465 | 760.239 | OH | a2 | B16 | b1 | C32 | c3 | D02 | d1 | E03 |
| 2-1-m1E03-1-0466 | 761.144 | OH | a2 | B12 | b3 | C17 | c1 | D01 | d1 | E03 |
| 2-1-m1E03-1-0467 | 761.144 | OH | a2 | B18 | b1 | C12 | c2 | D02 | d1 | E03 |
| 2-1-m1E03-1-0468 | 761.144 | OH | a2 | B12 | b1 | C14 | c2 | D02 | d1 | E03 |
| 2-1-m1E03-1-0469 | 761.154 | OH | a1 | B07 | b1 | C10 | c2 | D02 | d1 | E03 |
| 2-1-m1E03-1-0470 | 761.159 | OH | a1 | B09 | b2 | C06 | c1 | D01 | d1 | E03 |
| 2-1-m1E03-1-0471 | 761.177 | OH | a1 | B10 | b2 | C01 | c1 | D01 | d1 | E03 |
| 2-1-m1E03-1-0472 | 761.198 | OH | a2 | B15 | b2 | C03 | c2 | D02 | d1 | E03 |
| 2-1-m1E03-1-0473 | 761.198 | OH | a2 | B13 | b2 | C05 | c2 | D02 | d1 | E03 |
| 2-1-m1E03-1-0474 | 762.143 | OH | a2 | B19 | b3 | C15 | c1 | D01 | d1 | E03 |
| 2-1-m1E03-1-0475 | 762.173 | OH | a1 | B08 | b2 | C04 | c1 | D01 | d1 | E03 |
| 2-1-m1E03-1-0476 | 762.182 | OH | a2 | B16 | b2 | C02 | c2 | D02 | d1 | E03 |
| 2-1-m1E03-1-0477 | 762.218 | OH | a2 | B16 | b2 | C22 | c3 | D02 | d1 | E03 |
| 2-1-m1E03-1-0478 | 762.218 | OH | a2 | B13 | b2 | C26 | c3 | D02 | d1 | E03 |
| 2-1-m1E03-1-0479 | 762.235 | OH | a2 | B14 | b1 | C29 | c3 | D02 | d1 | E03 |
| 2-1-m1E03-1-0480 | 763.177 | OH | a2 | B16 | b2 | C03 | c2 | D02 | d1 | E03 |
| 2-1-m1E03-1-0481 | 763.189 | OH | a1 | B07 | b1 | C09 | c1 | D01 | d1 | E03 |
| 2-1-m1E03-1-0482 | 763.250 | OH | a1 | B05 | b1 | C30 | c3 | D02 | d1 | E03 |
| 2-1-m1E03-1-0483 | 763.250 | OH | a1 | B01 | b1 | C29 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0484 | 763.250 | OH | a1 | B03 | b1 | C32 | c3 | D02 | d1 | E03 |
| 2-1-m1E03-1-0485 | 764.174 | OH | a2 | B19 | b1 | C14 | c1 | D01 | d1 | E03 |
| 2-1-m1E03-1-0486 | 764.177 | OH | a2 | B13 | b2 | C04 | c2 | D02 | d1 | E03 |
| 2-1-m1E03-1-0487 | 764.195 | OH | a2 | B19 | b2 | C33 | c3 | D02 | d1 | E03 |
| 2-1-m1E03-1-0488 | 764.214 | OH | a2 | B15 | b2 | C24 | c3 | D02 | d1 | E03 |
| 2-1-m1E03-1-0489 | 764.234 | OH | a2 | B11 | b2 | C07 | c2 | D02 | d1 | E03 |
| 2-1-m1E03-1-0490 | 764.245 | OH | a1 | B02 | b1 | C29 | c3 | D02 | d1 | E03 |
| 2-1-m1E03-1-0491 | 765.120 | OH | a1 | B07 | b3 | C06 | c1 | D01 | d1 | E03 |
| 2-1-m1E03-1-0492 | 765.173 | OH | a2 | B14 | b2 | C05 | c2 | D02 | d1 | E03 |
| 2-1-m1E03-1-0493 | 765.193 | OH | a1 | B03 | b2 | C02 | c2 | D02 | d1 | E03 |
| 2-1-m1E03-1-0494 | 765.229 | OH | a2 | B12 | b2 | C07 | c2 | D02 | d1 | E03 |
| 2-1-m1E03-1-0495 | 765.229 | OH | a1 | B05 | b2 | C20 | c3 | D02 | d1 | E03 |
| 2-1-m1E03-1-0496 | 765.229 | OH | a1 | B03 | b2 | C22 | c3 | D02 | d1 | E03 |
| 2-1-m1E03-1-0497 | 765.229 | OH | a1 | B06 | b1 | C30 | c3 | D02 | d1 | E03 |
| 2-1-m1E03-1-0498 | 766.115 | OH | a1 | B07 | b1 | C12 | c2 | D02 | d1 | E03 |
| 2-1-m1E03-1-0499 | 766.159 | OH | a2 | B15 | b2 | C06 | c2 | D02 | d1 | E03 |
| 2-1-m1E03-1-0500 | 766.188 | OH | a1 | B03 | b2 | C03 | c2 | D02 | d1 | E03 |
| 2-1-m1E03-1-0501 | 766.188 | OH | a1 | B01 | b2 | C05 | c2 | D02 | d1 | E03 |
| 2-1-m1E03-1-0502 | 766.193 | OH | a2 | B16 | b2 | C24 | c3 | D02 | d1 | E03 |
| 2-1-m1E03-1-0503 | 766.193 | OH | a2 | B14 | b2 | C26 | c3 | D02 | d1 | E03 |
| 2-1-m1E03-1-0504 | 766.210 | OH | a2 | B17 | b1 | C32 | c3 | D02 | d1 | E03 |
| 2-1-m1E03-1-0505 | 766.224 | OH | a1 | B05 | b2 | C28 | c3 | D02 | d1 | E03 |
| 2-1-m1E03-1-0506 | 766.228 | OH | a2 | B18 | b1 | C30 | c3 | D02 | d1 | E03 |
| 2-1-m1E03-1-0507 | 767.183 | OH | a1 | B02 | b2 | C05 | c2 | D02 | d1 | E03 |
| 2-1-m1E03-1-0508 | 767.203 | OH | a2 | B20 | b2 | C05 | c1 | D01 | d1 | E03 |
| 2-1-m1E03-1-0509 | 767.208 | OH | a1 | B06 | b2 | C20 | c3 | D02 | d1 | E03 |
| 2-1-m1E03-1-0510 | 767.208 | OH | a1 | B01 | b2 | C26 | c3 | D02 | d1 | E03 |
| 2-1-m1E03-1-0511 | 767.225 | OH | a1 | B04 | b1 | C32 | c3 | D02 | d1 | E03 |
| 2-1-m1E03-1-0512 | 768.138 | OH | a2 | B16 | b2 | C06 | c2 | D02 | d1 | E03 |
| 2-1-m1E03-1-0513 | 768.138 | OH | a2 | B11 | b3 | C08 | c2 | D02 | d1 | E03 |
| 2-1-m1E03-1-0514 | 768.152 | OH | a2 | B17 | b2 | C02 | c2 | D02 | d1 | E03 |
| 2-1-m1E03-1-0515 | 768.152 | OH | a2 | B14 | b2 | C04 | c2 | D02 | d1 | E03 |
| 2-1-m1E03-1-0516 | 768.189 | OH | a2 | B17 | b2 | C22 | c3 | D02 | d1 | E03 |
| 2-1-m1E03-1-0517 | 768.194 | OH | a1 | B08 | b1 | C13 | c1 | D01 | d1 | E03 |
| 2-1-m1E03-1-0518 | 768.204 | OH | a1 | B02 | b2 | C26 | c3 | D02 | d1 | E03 |
| 2-1-m1E03-1-0519 | 768.204 | OH | a1 | B06 | b2 | C28 | c3 | D02 | d1 | E03 |
| 2-1-m1E03-1-0520 | 768.208 | OH | a2 | B18 | b2 | C20 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0521 | 768.259 | OH | a2 | B19 | b2 | C07 | c1 | D01 | d1 | E03 |
| 2-1-m1E03-1-0522 | 769.133 | OH | a2 | B12 | b3 | C08 | c2 | D02 | d1 | E03 |
| 2-1-m1E03-1-0523 | 769.148 | OH | a2 | B17 | b2 | C03 | c2 | D02 | d1 | E03 |
| 2-1-m1E03-1-0524 | 769.168 | OH | a1 | B04 | b2 | C02 | c2 | D02 | d1 | E03 |
| 2-1-m1E03-1-0525 | 769.168 | OH | a1 | B01 | b2 | C04 | c2 | D02 | d1 | E03 |
| 2-1-m1E03-1-0526 | 769.188 | OH | a1 | B05 | b2 | C01 | c2 | D02 | d1 | E03 |
| 2-1-m1E03-1-0527 | 769.203 | OH | a2 | B18 | b2 | C28 | c3 | D02 | d1 | E03 |
| 2-1-m1E03-1-0528 | 769.204 | OH | a1 | B04 | b2 | C22 | c3 | D02 | d1 | E03 |
| 2-1-m1E03-1-0529 | 769.204 | OH | a1 | B03 | b2 | C24 | c3 | D02 | d1 | E03 |
| 2-1-m1E03-1-0530 | 770.129 | OH | a1 | B08 | b3 | C15 | c1 | D01 | d1 | E03 |
| 2-1-m1E03-1-0531 | 770.163 | OH | a1 | B02 | b2 | C04 | c2 | D02 | d1 | E03 |
| 2-1-m1E03-1-0532 | 770.163 | OH | a1 | B04 | b2 | C03 | c2 | D02 | d1 | E03 |
| 2-1-m1E03-1-0533 | 770.182 | OH | a2 | B20 | b2 | C04 | c1 | D01 | d1 | E03 |
| 2-1-m1E03-1-0534 | 770.198 | OH | a2 | B13 | b1 | C13 | c2 | D02 | d1 | E03 |
| 2-1-m1E03-1-0535 | 770.213 | OH | a1 | B09 | b2 | C05 | c1 | D01 | d1 | E03 |
| 2-1-m1E03-1-0536 | 771.149 | OH | a1 | B03 | b2 | C06 | c2 | D02 | d1 | E03 |
| 2-1-m1E03-1-0537 | 771.167 | OH | a1 | B06 | b2 | C01 | c2 | D02 | d1 | E03 |
| 2-1-m1E03-1-0538 | 771.197 | OH | a1 | B10 | b2 | C02 | c1 | D01 | d1 | E03 |
| 2-1-m1E03-1-0539 | 771.200 | OH | a1 | B07 | b1 | C30 | c3 | D02 | d1 | E03 |
| 2-1-m1E03-1-0540 | 772.133 | OH | a2 | B13 | b3 | C15 | c2 | D02 | d1 | E03 |
| 2-1-m1E03-1-0541 | 772.160 | OH | a1 | B08 | b1 | C14 | c1 | D01 | d1 | E03 |
| 2-1-m1E03-1-0542 | 772.164 | OH | a2 | B19 | b3 | C08 | c1 | D01 | d1 | E03 |
| 2-1-m1E03-1-0543 | 772.164 | OH | a2 | B19 | b1 | C08 | c2 | D02 | d1 | E03 |
| 2-1-m1E03-1-0544 | 772.164 | OH | a2 | B11 | b1 | C09 | c2 | D02 | d1 | E03 |
| 2-1-m1E03-1-0545 | 772.164 | OH | a2 | B17 | b2 | C24 | c3 | D02 | d1 | E03 |
| 2-1-m1E03-1-0546 | 772.166 | OH | a2 | B18 | b2 | C01 | c2 | D02 | d1 | E03 |
| 2-1-m1E03-1-0547 | 772.181 | OH | a1 | B08 | b2 | C33 | c3 | D02 | d1 | E03 |
| 2-1-m1E03-1-0548 | 772.193 | OH | a1 | B10 | b2 | C03 | c1 | D01 | d1 | E03 |
| 2-1-m1E03-1-0549 | 772.203 | OH | a2 | B13 | b3 | C16 | c1 | D01 | d1 | E03 |
| 2-1-m1E03-1-0550 | 772.239 | OH | a2 | B11 | b1 | C31 | c3 | D02 | d1 | E03 |
| 2-1-m1E03-1-0551 | 772.275 | OH | a2 | B15 | b1 | C29 | c3 | D02 | d1 | E03 |
| 2-1-m1E03-1-0552 | 773.159 | OH | a2 | B12 | b1 | C09 | c2 | D02 | d1 | E03 |
| 2-1-m1E03-1-0553 | 773.159 | OH | a2 | B19 | b1 | C11 | c2 | D02 | d1 | E03 |
| 2-1-m1E03-1-0554 | 773.179 | OH | a1 | B07 | b2 | C20 | c3 | D02 | d1 | E03 |
| 2-1-m1E03-1-0555 | 773.179 | OH | a1 | B04 | b2 | C24 | c3 | D02 | d1 | E03 |
| 2-1-m1E03-1-0556 | 773.193 | OH | a1 | B09 | b2 | C04 | c1 | D01 | d1 | E03 |
| 2-1-m1E03-1-0557 | 773.234 | OH | a2 | B12 | b1 | C31 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0558 | 774.095 | OH | a2 | B11 | b3 | C06 | c2 | D02 | d1 | E03 |
| 2-1-m1E03-1-0559 | 774.109 | OH | a2 | B17 | b2 | C06 | c2 | D02 | d1 | E03 |
| 2-1-m1E03-1-0560 | 774.154 | OH | a2 | B19 | b1 | C10 | c2 | D02 | d1 | E03 |
| 2-1-m1E03-1-0561 | 774.164 | OH | a2 | B13 | b3 | C17 | c1 | D01 | d1 | E03 |
| 2-1-m1E03-1-0562 | 774.164 | OH | a2 | B13 | b1 | C14 | c2 | D02 | d1 | E03 |
| 2-1-m1E03-1-0563 | 774.173 | OH | a2 | B14 | b1 | C13 | c2 | D02 | d1 | E03 |
| 2-1-m1E03-1-0564 | 774.174 | OH | a1 | B07 | b2 | C28 | c3 | D02 | d1 | E03 |
| 2-1-m1E03-1-0565 | 774.255 | OH | a2 | B16 | b1 | C29 | c3 | D02 | d1 | E03 |
| 2-1-m1E03-1-0566 | 775.090 | OH | a2 | B12 | b3 | C06 | c2 | D02 | d1 | E03 |
| 2-1-m1E03-1-0567 | 775.124 | OH | a1 | B04 | b2 | C06 | c2 | D02 | d1 | E03 |
| 2-1-m1E03-1-0568 | 775.159 | OH | a2 | B11 | b3 | C18 | c1 | D01 | d1 | E03 |
| 2-1-m1E03-1-0569 | 775.188 | OH | a1 | B01 | b1 | C13 | c2 | D02 | d1 | E03 |
| 2-1-m1E03-1-0570 | 775.213 | OH | a2 | B15 | b2 | C05 | c2 | D02 | d1 | E03 |
| 2-1-m1E03-1-0571 | 776.108 | OH | a2 | B14 | b3 | C15 | c2 | D02 | d1 | E03 |
| 2-1-m1E03-1-0572 | 776.155 | OH | a2 | B12 | b3 | C18 | c1 | D01 | d1 | E03 |
| 2-1-m1E03-1-0573 | 776.177 | OH | a2 | B14 | b3 | C16 | c1 | D01 | d1 | E03 |
| 2-1-m1E03-1-0574 | 776.184 | OH | a1 | B02 | b1 | C13 | c2 | D02 | d1 | E03 |
| 2-1-m1E03-1-0575 | 776.189 | OH | a2 | B19 | b1 | C09 | c1 | D01 | d1 | E03 |
| 2-1-m1E03-1-0576 | 776.203 | OH | a2 | B20 | b1 | C13 | c1 | D01 | d1 | E03 |
| 2-1-m1E03-1-0577 | 776.234 | OH | a2 | B15 | b2 | C26 | c3 | D02 | d1 | E03 |
| 2-1-m1E03-1-0578 | 776.245 | OH | a1 | B08 | b2 | C07 | c1 | D01 | d1 | E03 |
| 2-1-m1E03-1-0579 | 777.123 | OH | a1 | B01 | b3 | C15 | c2 | D02 | d1 | E03 |
| 2-1-m1E03-1-0580 | 777.137 | OH | a1 | B07 | b2 | C01 | c2 | D02 | d1 | E03 |
| 2-1-m1E03-1-0581 | 777.154 | OH | a1 | B10 | b2 | C06 | c1 | D01 | d1 | E03 |
| 2-1-m1E03-1-0582 | 777.193 | OH | a1 | B01 | b3 | C16 | c1 | D01 | d1 | E03 |
| 2-1-m1E03-1-0583 | 777.193 | OH | a2 | B16 | b2 | C05 | c2 | D02 | d1 | E03 |
| 2-1-m1E03-1-0584 | 777.265 | OH | a1 | B03 | b1 | C29 | c3 | D02 | d1 | E03 |
| 2-1-m1E03-1-0585 | 777.265 | OH | a1 | B05 | b1 | C32 | c3 | D02 | d1 | E03 |
| 2-1-m1E03-1-0586 | 778.119 | OH | a1 | B02 | b3 | C15 | c2 | D02 | d1 | E03 |
| 2-1-m1E03-1-0587 | 778.120 | OH | a2 | B19 | b3 | C06 | c1 | D01 | d1 | E03 |
| 2-1-m1E03-1-0588 | 778.138 | OH | a2 | B20 | b3 | C15 | c1 | D01 | d1 | E03 |
| 2-1-m1E03-1-0589 | 778.139 | OH | a2 | B14 | b3 | C17 | c1 | D01 | d1 | E03 |
| 2-1-m1E03-1-0590 | 778.139 | OH | a2 | B14 | b1 | C14 | c2 | D02 | d1 | E03 |
| 2-1-m1E03-1-0591 | 778.188 | OH | a1 | B02 | b3 | C16 | c1 | D01 | d1 | E03 |
| 2-1-m1E03-1-0592 | 778.193 | OH | a2 | B15 | b2 | C04 | c2 | D02 | d1 | E03 |
| 2-1-m1E03-1-0593 | 778.213 | OH | a2 | B16 | b2 | C26 | c3 | D02 | d1 | E03 |
| 2-1-m1E03-1-0594 | 778.249 | OH | a2 | B13 | b2 | C07 | c2 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0595 | 779.115 | OH | a2 | B19 | b1 | C12 | c2 | D02 | d1 | E03 |
| 2-1-m1E03-1-0596 | 779.154 | OH | a1 | B01 | b3 | C17 | c1 | D01 | d1 | E03 |
| 2-1-m1E03-1-0597 | 779.154 | OH | a1 | B01 | b1 | C14 | c2 | D02 | d1 | E03 |
| 2-1-m1E03-1-0598 | 779.208 | OH | a1 | B05 | b2 | C02 | c2 | D02 | d1 | E03 |
| 2-1-m1E03-1-0599 | 779.214 | OH | a1 | B09 | b1 | C13 | c1 | D01 | d1 | E03 |
| 2-1-m1E03-1-0600 | 779.245 | OH | a1 | B05 | b2 | C22 | c3 | D02 | d1 | E03 |
| 2-1-m1E03-1-0601 | 779.245 | OH | a1 | B06 | b1 | C32 | c3 | D02 | d1 | E03 |
| 2-1-m1E03-1-0602 | 780.149 | OH | a1 | B02 | b3 | C17 | c1 | D01 | d1 | E03 |
| 2-1-m1E03-1-0603 | 780.149 | OH | a1 | B08 | b3 | C08 | c1 | D01 | d1 | E03 |
| 2-1-m1E03-1-0604 | 780.149 | OH | a1 | B08 | b1 | C08 | c2 | D02 | d1 | E03 |
| 2-1-m1E03-1-0605 | 780.149 | OH | a1 | B02 | b1 | C14 | c2 | D02 | d1 | E03 |
| 2-1-m1E03-1-0606 | 780.169 | OH | a2 | B20 | b1 | C14 | c1 | D01 | d1 | E03 |
| 2-1-m1E03-1-0607 | 780.172 | OH | a2 | B16 | b2 | C04 | c2 | D02 | d1 | E03 |
| 2-1-m1E03-1-0608 | 780.190 | OH | a2 | B20 | b2 | C33 | c3 | D02 | d1 | E03 |
| 2-1-m1E03-1-0609 | 780.204 | OH | a1 | B05 | b2 | C03 | c2 | D02 | d1 | E03 |
| 2-1-m1E03-1-0610 | 780.204 | OH | a1 | B03 | b2 | C05 | c2 | D02 | d1 | E03 |
| 2-1-m1E03-1-0611 | 780.225 | OH | a2 | B17 | b1 | C29 | c3 | D02 | d1 | E03 |
| 2-1-m1E03-1-0612 | 780.244 | OH | a2 | B18 | b1 | C32 | c3 | D02 | d1 | E03 |
| 2-1-m1E03-1-0613 | 781.145 | OH | a1 | B08 | b1 | C11 | c2 | D02 | d1 | E03 |
| 2-1-m1E03-1-0614 | 781.149 | OH | a1 | B09 | b3 | C15 | c1 | D01 | d1 | E03 |
| 2-1-m1E03-1-0615 | 781.188 | OH | a1 | B06 | b2 | C02 | c2 | D02 | d1 | E03 |
| 2-1-m1E03-1-0616 | 781.224 | OH | a1 | B06 | b2 | C22 | c3 | D02 | d1 | E03 |
| 2-1-m1E03-1-0617 | 781.224 | OH | a1 | B03 | b2 | C26 | c3 | D02 | d1 | E03 |
| 2-1-m1E03-1-0618 | 781.240 | OH | a1 | B04 | b1 | C29 | c3 | D02 | d1 | E03 |
| 2-1-m1E03-1-0619 | 782.140 | OH | a1 | B08 | b1 | C10 | c2 | D02 | d1 | E03 |
| 2-1-m1E03-1-0620 | 782.154 | OH | a2 | B13 | b3 | C08 | c2 | D02 | d1 | E03 |
| 2-1-m1E03-1-0621 | 782.183 | OH | a1 | B06 | b2 | C03 | c2 | D02 | d1 | E03 |
| 2-1-m1E03-1-0622 | 782.187 | OH | a2 | B18 | b2 | C02 | c2 | D02 | d1 | E03 |
| 2-1-m1E03-1-0623 | 782.223 | OH | a2 | B18 | b2 | C22 | c3 | D02 | d1 | E03 |
| 2-1-m1E03-1-0624 | 782.224 | OH | a2 | B14 | b2 | C07 | c2 | D02 | d1 | E03 |
| 2-1-m1E03-1-0625 | 783.163 | OH | a2 | B17 | b2 | C05 | c2 | D02 | d1 | E03 |
| 2-1-m1E03-1-0626 | 783.180 | OH | a1 | B09 | b1 | C14 | c1 | D01 | d1 | E03 |
| 2-1-m1E03-1-0627 | 783.182 | OH | a2 | B18 | b2 | C03 | c2 | D02 | d1 | E03 |
| 2-1-m1E03-1-0628 | 783.183 | OH | a1 | B03 | b2 | C04 | c2 | D02 | d1 | E03 |
| 2-1-m1E03-1-0629 | 783.201 | OH | a1 | B09 | b2 | C33 | c3 | D02 | d1 | E03 |
| 2-1-m1E03-1-0630 | 783.220 | OH | a1 | B05 | b2 | C24 | c3 | D02 | d1 | E03 |
| 2-1-m1E03-1-0631 | 783.240 | OH | a1 | B01 | b2 | C07 | c2 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0632 | 784.175 | OH | a1 | B08 | b1 | C09 | c1 | D01 | d1 | E03 |
| 2-1-m1E03-1-0633 | 784.179 | OH | a1 | B04 | b2 | C05 | c2 | D02 | d1 | E03 |
| 2-1-m1E03-1-0634 | 784.184 | OH | a2 | B17 | b2 | C26 | c3 | D02 | d1 | E03 |
| 2-1-m1E03-1-0635 | 784.200 | OH | a2 | B19 | b1 | C30 | c3 | D02 | d1 | E03 |
| 2-1-m1E03-1-0636 | 784.214 | OH | a2 | B15 | b1 | C13 | c2 | D02 | d1 | E03 |
| 2-1-m1E03-1-0637 | 784.235 | OH | a1 | B02 | b2 | C07 | c2 | D02 | d1 | E03 |
| 2-1-m1E03-1-0638 | 784.254 | OH | a2 | B20 | b2 | C07 | c1 | D01 | d1 | E03 |
| 2-1-m1E03-1-0639 | 785.165 | OH | a1 | B05 | b2 | C06 | c2 | D02 | d1 | E03 |
| 2-1-m1E03-1-0640 | 785.199 | OH | a1 | B06 | b2 | C24 | c3 | D02 | d1 | E03 |
| 2-1-m1E03-1-0641 | 785.199 | OH | a1 | B04 | b2 | C26 | c3 | D02 | d1 | E03 |
| 2-1-m1E03-1-0642 | 785.215 | OH | a1 | B07 | b1 | C32 | c3 | D02 | d1 | E03 |
| 2-1-m1E03-1-0643 | 786.106 | OH | a1 | B08 | b3 | C06 | c1 | D01 | d1 | E03 |
| 2-1-m1E03-1-0644 | 786.129 | OH | a2 | B14 | b3 | C08 | c2 | D02 | d1 | E03 |
| 2-1-m1E03-1-0645 | 786.143 | OH | a2 | B17 | b2 | C04 | c2 | D02 | d1 | E03 |
| 2-1-m1E03-1-0646 | 786.149 | OH | a2 | B15 | b3 | C15 | c2 | D02 | d1 | E03 |
| 2-1-m1E03-1-0647 | 786.179 | OH | a2 | B13 | b1 | C09 | c2 | D02 | d1 | E03 |
| 2-1-m1E03-1-0648 | 786.179 | OH | a2 | B19 | b2 | C20 | c3 | D02 | d1 | E03 |
| 2-1-m1E03-1-0649 | 786.193 | OH | a2 | B16 | b1 | C13 | c2 | D02 | d1 | E03 |
| 2-1-m1E03-1-0650 | 786.198 | OH | a2 | B18 | b2 | C24 | c3 | D02 | d1 | E03 |
| 2-1-m1E03-1-0651 | 786.208 | OH | a1 | B10 | b2 | C05 | c1 | D01 | d1 | E03 |
| 2-1-m1E03-1-0652 | 786.218 | OH | a2 | B15 | b3 | C16 | c1 | D01 | d1 | E03 |
| 2-1-m1E03-1-0653 | 786.255 | OH | a2 | B13 | b1 | C31 | c3 | D02 | d1 | E03 |
| 2-1-m1E03-1-0654 | 787.101 | OH | a1 | B08 | b1 | C12 | c2 | D02 | d1 | E03 |
| 2-1-m1E03-1-0655 | 787.144 | OH | a1 | B06 | b2 | C06 | c2 | D02 | d1 | E03 |
| 2-1-m1E03-1-0656 | 787.144 | OH | a1 | B01 | b3 | C08 | c2 | D02 | d1 | E03 |
| 2-1-m1E03-1-0657 | 787.158 | OH | a1 | B07 | b2 | C02 | c2 | D02 | d1 | E03 |
| 2-1-m1E03-1-0658 | 787.158 | OH | a1 | B04 | b2 | C04 | c2 | D02 | d1 | E03 |
| 2-1-m1E03-1-0659 | 787.175 | OH | a2 | B19 | b2 | C28 | c3 | D02 | d1 | E03 |
| 2-1-m1E03-1-0660 | 787.195 | OH | a1 | B07 | b2 | C22 | c3 | D02 | d1 | E03 |
| 2-1-m1E03-1-0661 | 787.265 | OH | a1 | B09 | b2 | C07 | c1 | D01 | d1 | E03 |
| 2-1-m1E03-1-0662 | 788.110 | OH | a2 | B13 | b3 | C06 | c2 | D02 | d1 | E03 |
| 2-1-m1E03-1-0663 | 788.128 | OH | a2 | B16 | b3 | C15 | c2 | D02 | d1 | E03 |
| 2-1-m1E03-1-0664 | 788.139 | OH | a1 | B02 | b3 | C08 | c2 | D02 | d1 | E03 |
| 2-1-m1E03-1-0665 | 788.143 | OH | a2 | B18 | b2 | C06 | c2 | D02 | d1 | E03 |
| 2-1-m1E03-1-0666 | 788.153 | OH | a1 | B07 | b2 | C03 | c2 | D02 | d1 | E03 |
| 2-1-m1E03-1-0667 | 788.159 | OH | a2 | B20 | b3 | C08 | c1 | D01 | d1 | E03 |
| 2-1-m1E03-1-0668 | 788.159 | OH | a2 | B20 | b1 | C08 | c2 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0669 | 788.180 | OH | a2 | B15 | b3 | C17 | c1 | D01 | d1 | E03 |
| 2-1-m1E03-1-0670 | 788.180 | OH | a2 | B15 | b1 | C14 | c2 | D02 | d1 | E03 |
| 2-1-m1E03-1-0671 | 788.197 | OH | a2 | B16 | b3 | C16 | c1 | D01 | d1 | E03 |
| 2-1-m1E03-1-0672 | 789.154 | OH | a2 | B20 | b1 | C11 | c2 | D02 | d1 | E03 |
| 2-1-m1E03-1-0673 | 789.175 | OH | a2 | B13 | b3 | C18 | c1 | D01 | d1 | E03 |
| 2-1-m1E03-1-0674 | 789.188 | OH | a1 | B10 | b2 | C04 | c1 | D01 | d1 | E03 |
| 2-1-m1E03-1-0675 | 789.204 | OH | a1 | B03 | b1 | C13 | c2 | D02 | d1 | E03 |
| 2-1-m1E03-1-0676 | 790.138 | OH | a2 | B19 | b2 | C01 | c2 | D02 | d1 | E03 |
| 2-1-m1E03-1-0677 | 790.149 | OH | a2 | B20 | b1 | C10 | c2 | D02 | d1 | E03 |
| 2-1-m1E03-1-0678 | 790.154 | OH | a2 | B14 | b1 | C09 | c2 | D02 | d1 | E03 |
| 2-1-m1E03-1-0679 | 790.159 | OH | a2 | B16 | b3 | C17 | c1 | D01 | d1 | E03 |
| 2-1-m1E03-1-0680 | 790.159 | OH | a2 | B16 | b1 | C14 | c2 | D02 | d1 | E03 |
| 2-1-m1E03-1-0681 | 790.230 | OH | a2 | B14 | b1 | C31 | c3 | D02 | d1 | E03 |
| 2-1-m1E03-1-0682 | 791.139 | OH | a1 | B03 | b3 | C15 | c2 | D02 | d1 | E03 |
| 2-1-m1E03-1-0683 | 791.170 | OH | a1 | B09 | b3 | C08 | c1 | D01 | d1 | E03 |
| 2-1-m1E03-1-0684 | 791.170 | OH | a1 | B09 | b1 | C08 | c2 | D02 | d1 | E03 |
| 2-1-m1E03-1-0685 | 791.170 | OH | a1 | B01 | b1 | C09 | c2 | D02 | d1 | E03 |
| 2-1-m1E03-1-0686 | 791.170 | OH | a1 | B07 | b2 | C24 | c3 | D02 | d1 | E03 |
| 2-1-m1E03-1-0687 | 791.208 | OH | a1 | B03 | b3 | C16 | c1 | D01 | d1 | E03 |
| 2-1-m1E03-1-0688 | 791.245 | OH | a1 | B01 | b1 | C31 | c3 | D02 | d1 | E03 |
| 2-1-m1E03-1-0689 | 791.281 | OH | a1 | B05 | b1 | C29 | c3 | D02 | d1 | E03 |
| 2-1-m1E03-1-0690 | 792.085 | OH | a2 | B14 | b3 | C06 | c2 | D02 | d1 | E03 |
| 2-1-m1E03-1-0691 | 792.164 | OH | a2 | B17 | b1 | C13 | c2 | D02 | d1 | E03 |
| 2-1-m1E03-1-0692 | 792.165 | OH | a1 | B02 | b1 | C09 | c2 | D02 | d1 | E03 |
| 2-1-m1E03-1-0693 | 792.165 | OH | a1 | B09 | b1 | C11 | c2 | D02 | d1 | E03 |
| 2-1-m1E03-1-0694 | 792.184 | OH | a2 | B20 | b1 | C09 | c1 | D01 | d1 | E03 |
| 2-1-m1E03-1-0695 | 792.186 | OH | a1 | B08 | b1 | C30 | c3 | D02 | d1 | E03 |
| 2-1-m1E03-1-0696 | 792.240 | OH | a1 | B02 | b1 | C31 | c3 | D02 | d1 | E03 |
| 2-1-m1E03-1-0697 | 792.265 | OH | a2 | B15 | b2 | C07 | c2 | D02 | d1 | E03 |
| 2-1-m1E03-1-0698 | 793.100 | OH | a1 | B01 | b3 | C06 | c2 | D02 | d1 | E03 |
| 2-1-m1E03-1-0699 | 793.115 | OH | a1 | B07 | b2 | C06 | c2 | D02 | d1 | E03 |
| 2-1-m1E03-1-0700 | 793.150 | OH | a2 | B14 | b3 | C18 | c1 | D01 | d1 | E03 |
| 2-1-m1E03-1-0701 | 793.160 | OH | a1 | B09 | b1 | C10 | c2 | D02 | d1 | E03 |
| 2-1-m1E03-1-0702 | 793.170 | OH | a1 | B03 | b3 | C17 | c1 | D01 | d1 | E03 |
| 2-1-m1E03-1-0703 | 793.170 | OH | a1 | B03 | b1 | C14 | c2 | D02 | d1 | E03 |
| 2-1-m1E03-1-0704 | 793.179 | OH | a1 | B04 | b1 | C13 | c2 | D02 | d1 | E03 |
| 2-1-m1E03-1-0705 | 793.260 | OH | a1 | B06 | b1 | C29 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0706 | 794.096 | OH | a1 | B02 | b3 | C06 | c2 | D02 | d1 | E03 |
| 2-1-m1E03-1-0707 | 794.099 | OH | a2 | B17 | b3 | C15 | c2 | D02 | d1 | E03 |
| 2-1-m1E03-1-0708 | 794.115 | OH | a2 | B20 | b3 | C06 | c1 | D01 | d1 | E03 |
| 2-1-m1E03-1-0709 | 794.165 | OH | a1 | B01 | b3 | C18 | c1 | D01 | d1 | E03 |
| 2-1-m1E03-1-0710 | 794.165 | OH | a1 | B08 | b2 | C20 | c3 | D02 | d1 | E03 |
| 2-1-m1E03-1-0711 | 794.168 | OH | a2 | B17 | b3 | C16 | c1 | D01 | d1 | E03 |
| 2-1-m1E03-1-0712 | 794.219 | OH | a1 | B05 | b2 | C05 | c2 | D02 | d1 | E03 |
| 2-1-m1E03-1-0713 | 794.244 | OH | a2 | B16 | b2 | C07 | c2 | D02 | d1 | E03 |
| 2-1-m1E03-1-0714 | 794.260 | OH | a2 | B18 | b1 | C29 | c3 | D02 | d1 | E03 |
| 2-1-m1E03-1-0715 | 795.110 | OH | a2 | B20 | b1 | C12 | c2 | D02 | d1 | E03 |
| 2-1-m1E03-1-0716 | 795.114 | OH | a1 | B04 | b3 | C15 | c2 | D02 | d1 | E03 |
| 2-1-m1E03-1-0717 | 795.160 | OH | a1 | B02 | b3 | C18 | c1 | D01 | d1 | E03 |
| 2-1-m1E03-1-0718 | 795.160 | OH | a1 | B08 | b2 | C28 | c3 | D02 | d1 | E03 |
| 2-1-m1E03-1-0719 | 795.183 | OH | a1 | B04 | b3 | C16 | c1 | D01 | d1 | E03 |
| 2-1-m1E03-1-0720 | 795.195 | OH | a1 | B09 | b1 | C09 | c1 | D01 | d1 | E03 |
| 2-1-m1E03-1-0721 | 795.209 | OH | a1 | B10 | b1 | C13 | c1 | D01 | d1 | E03 |
| 2-1-m1E03-1-0722 | 795.240 | OH | a1 | B05 | b2 | C26 | c3 | D02 | d1 | E03 |
| 2-1-m1E03-1-0723 | 796.130 | OH | a2 | B17 | b3 | C17 | c1 | D01 | d1 | E03 |
| 2-1-m1E03-1-0724 | 796.130 | OH | a2 | B17 | b1 | C14 | c2 | D02 | d1 | E03 |
| 2-1-m1E03-1-0725 | 796.170 | OH | a2 | B15 | b3 | C08 | c2 | D02 | d1 | E03 |
| 2-1-m1E03-1-0726 | 796.199 | OH | a1 | B06 | b2 | C05 | c2 | D02 | d1 | E03 |
| 2-1-m1E03-1-0727 | 797.126 | OH | a1 | B09 | b3 | C06 | c1 | D01 | d1 | E03 |
| 2-1-m1E03-1-0728 | 797.144 | OH | a1 | B10 | b3 | C15 | c1 | D01 | d1 | E03 |
| 2-1-m1E03-1-0729 | 797.145 | OH | a1 | B04 | b3 | C17 | c1 | D01 | d1 | E03 |
| 2-1-m1E03-1-0730 | 797.145 | OH | a1 | B04 | b1 | C14 | c2 | D02 | d1 | E03 |
| 2-1-m1E03-1-0731 | 797.198 | OH | a2 | B18 | b2 | C05 | c2 | D02 | d1 | E03 |
| 2-1-m1E03-1-0732 | 797.199 | OH | a1 | B05 | b2 | C04 | c2 | D02 | d1 | E03 |
| 2-1-m1E03-1-0733 | 797.219 | OH | a1 | B06 | b2 | C26 | c3 | D02 | d1 | E03 |
| 2-1-m1E03-1-0734 | 797.255 | OH | a1 | B03 | b2 | C07 | c2 | D02 | d1 | E03 |
| 2-1-m1E03-1-0735 | 798.121 | OH | a1 | B09 | b1 | C12 | c2 | D02 | d1 | E03 |
| 2-1-m1E03-1-0736 | 798.124 | OH | a1 | B08 | b2 | C01 | c2 | D02 | d1 | E03 |
| 2-1-m1E03-1-0737 | 798.149 | OH | a2 | B16 | b3 | C08 | c2 | D02 | d1 | E03 |
| 2-1-m1E03-1-0738 | 798.216 | OH | a2 | B19 | b1 | C32 | c3 | D02 | d1 | E03 |
| 2-1-m1E03-1-0739 | 798.218 | OH | a2 | B18 | b2 | C26 | c3 | D02 | d1 | E03 |
| 2-1-m1E03-1-0740 | 799.175 | OH | a1 | B10 | b1 | C14 | c1 | D01 | d1 | E03 |
| 2-1-m1E03-1-0741 | 799.178 | OH | a1 | B06 | b2 | C04 | c2 | D02 | d1 | E03 |
| 2-1-m1E03-1-0742 | 799.196 | OH | a1 | B10 | b2 | C33 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0743 | 799.231 | OH | a1 | B07 | b1 | C29 | c3 | D02 | d1 | E03 |
| 2-1-m1E03-1-0744 | 800.159 | OH | a2 | B19 | b2 | C02 | c2 | D02 | d1 | E03 |
| 2-1-m1E03-1-0745 | 800.177 | OH | a2 | B18 | b2 | C04 | c2 | D02 | d1 | E03 |
| 2-1-m1E03-1-0746 | 800.195 | OH | a2 | B15 | b1 | C09 | c2 | D02 | d1 | E03 |
| 2-1-m1E03-1-0747 | 800.195 | OH | a2 | B19 | b2 | C22 | c3 | D02 | d1 | E03 |
| 2-1-m1E03-1-0748 | 800.195 | OH | a2 | B20 | b1 | C30 | c3 | D02 | d1 | E03 |
| 2-1-m1E03-1-0749 | 800.215 | OH | a2 | B17 | b2 | C07 | c2 | D02 | d1 | E03 |
| 2-1-m1E03-1-0750 | 800.270 | OH | a2 | B15 | b1 | C31 | c3 | D02 | d1 | E03 |
| 2-1-m1E03-1-0751 | 801.154 | OH | a2 | B19 | b2 | C03 | c2 | D02 | d1 | E03 |
| 2-1-m1E03-1-0752 | 801.160 | OH | a1 | B03 | b3 | C08 | c2 | D02 | d1 | E03 |
| 2-1-m1E03-1-0753 | 801.230 | OH | a1 | B04 | b2 | C07 | c2 | D02 | d1 | E03 |
| 2-1-m1E03-1-0754 | 802.126 | OH | a2 | B15 | b3 | C06 | c2 | D02 | d1 | E03 |
| 2-1-m1E03-1-0755 | 802.169 | OH | a1 | B07 | b2 | C05 | c2 | D02 | d1 | E03 |
| 2-1-m1E03-1-0756 | 802.174 | OH | a2 | B16 | b1 | C09 | c2 | D02 | d1 | E03 |
| 2-1-m1E03-1-0757 | 802.174 | OH | a2 | B20 | b2 | C20 | c3 | D02 | d1 | E03 |
| 2-1-m1E03-1-0758 | 802.249 | OH | a2 | B16 | b1 | C31 | c3 | D02 | d1 | E03 |
| 2-1-m1E03-1-0759 | 803.170 | OH | a2 | B20 | b2 | C28 | c3 | D02 | d1 | E03 |
| 2-1-m1E03-1-0760 | 803.190 | OH | a1 | B07 | b2 | C26 | c3 | D02 | d1 | E03 |
| 2-1-m1E03-1-0761 | 803.191 | OH | a2 | B15 | b3 | C18 | c1 | D01 | d1 | E03 |
| 2-1-m1E03-1-0762 | 803.206 | OH | a1 | B09 | b1 | C30 | c3 | D02 | d1 | E03 |
| 2-1-m1E03-1-0763 | 803.220 | OH | a1 | B05 | b1 | C13 | c2 | D02 | d1 | E03 |
| 2-1-m1E03-1-0764 | 803.260 | OH | a1 | B10 | b2 | C07 | c1 | D01 | d1 | E03 |
| 2-1-m1E03-1-0765 | 804.105 | OH | a2 | B16 | b3 | C06 | c2 | D02 | d1 | E03 |
| 2-1-m1E03-1-0766 | 804.119 | OH | a2 | B17 | b3 | C08 | c2 | D02 | d1 | E03 |
| 2-1-m1E03-1-0767 | 804.170 | OH | a2 | B19 | b2 | C24 | c3 | D02 | d1 | E03 |
| 2-1-m1E03-1-0768 | 805.135 | OH | a1 | B04 | b3 | C08 | c2 | D02 | d1 | E03 |
| 2-1-m1E03-1-0769 | 805.149 | OH | a1 | B07 | b2 | C04 | c2 | D02 | d1 | E03 |
| 2-1-m1E03-1-0770 | 805.155 | OH | a1 | B05 | b3 | C15 | c2 | D02 | d1 | E03 |
| 2-1-m1E03-1-0771 | 805.170 | OH | a2 | B16 | b3 | C18 | c1 | D01 | d1 | E03 |
| 2-1-m1E03-1-0772 | 805.185 | OH | a1 | B03 | b1 | C09 | c2 | D02 | d1 | E03 |
| 2-1-m1E03-1-0773 | 805.185 | OH | a1 | B09 | b2 | C20 | c3 | D02 | d1 | E03 |
| 2-1-m1E03-1-0774 | 805.199 | OH | a1 | B06 | b1 | C13 | c2 | D02 | d1 | E03 |
| 2-1-m1E03-1-0775 | 805.224 | OH | a1 | B05 | b3 | C16 | c1 | D01 | d1 | E03 |
| 2-1-m1E03-1-0776 | 805.260 | OH | a1 | B03 | b1 | C31 | c3 | D02 | d1 | E03 |
| 2-1-m1E03-1-0777 | 806.115 | OH | a2 | B19 | b2 | C06 | c2 | D02 | d1 | E03 |
| 2-1-m1E03-1-0778 | 806.133 | OH | a2 | B20 | b2 | C01 | c2 | D02 | d1 | E03 |
| 2-1-m1E03-1-0779 | 806.180 | OH | a1 | B09 | b2 | C28 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0780 | 806.198 | OH | a2 | B18 | b1 | C13 | c2 | D02 | d1 | E03 |
| 2-1-m1E03-1-0781 | 806.201 | OH | a1 | B08 | b1 | C32 | c3 | D02 | d1 | E03 |
| 2-1-m1E03-1-0782 | 807.116 | OH | a1 | B03 | b3 | C06 | c2 | D02 | d1 | E03 |
| 2-1-m1E03-1-0783 | 807.134 | OH | a1 | B06 | b3 | C15 | c2 | D02 | d1 | E03 |
| 2-1-m1E03-1-0784 | 807.164 | OH | a1 | B10 | b3 | C08 | c1 | D01 | d1 | E03 |
| 2-1-m1E03-1-0785 | 807.164 | OH | a1 | B10 | b1 | C08 | c2 | D02 | d1 | E03 |
| 2-1-m1E03-1-0786 | 807.186 | OH | a1 | B05 | b3 | C17 | c1 | D01 | d1 | E03 |
| 2-1-m1E03-1-0787 | 807.186 | OH | a1 | B05 | b1 | C14 | c2 | D02 | d1 | E03 |
| 2-1-m1E03-1-0788 | 807.203 | OH | a1 | B06 | b3 | C16 | c1 | D01 | d1 | E03 |
| 2-1-m1E03-1-0789 | 808.133 | OH | a2 | B18 | b3 | C15 | c2 | D02 | d1 | E03 |
| 2-1-m1E03-1-0790 | 808.144 | OH | a1 | B08 | b2 | C02 | c2 | D02 | d1 | E03 |
| 2-1-m1E03-1-0791 | 808.145 | OH | a2 | B17 | b1 | C09 | c2 | D02 | d1 | E03 |
| 2-1-m1E03-1-0792 | 808.160 | OH | a1 | B10 | b1 | C11 | c2 | D02 | d1 | E03 |
| 2-1-m1E03-1-0793 | 808.181 | OH | a1 | B03 | b3 | C18 | c1 | D01 | d1 | E03 |
| 2-1-m1E03-1-0794 | 808.181 | OH | a1 | B08 | b2 | C22 | c3 | D02 | d1 | E03 |
| 2-1-m1E03-1-0795 | 808.203 | OH | a2 | B18 | b3 | C16 | c1 | D01 | d1 | E03 |
| 2-1-m1E03-1-0796 | 808.220 | OH | a2 | B17 | b1 | C31 | c3 | D02 | d1 | E03 |
| 2-1-m1E03-1-0797 | 809.140 | OH | a1 | B08 | b2 | C03 | c2 | D02 | d1 | E03 |
| 2-1-m1E03-1-0798 | 809.144 | OH | a1 | B09 | b2 | C01 | c2 | D02 | d1 | E03 |
| 2-1-m1E03-1-0799 | 809.155 | OH | a1 | B10 | b1 | C10 | c2 | D02 | d1 | E03 |
| 2-1-m1E03-1-0800 | 809.160 | OH | a1 | B04 | b1 | C09 | c2 | D02 | d1 | E03 |
| 2-1-m1E03-1-0801 | 809.165 | OH | a1 | B06 | b3 | C17 | c1 | D01 | d1 | E03 |
| 2-1-m1E03-1-0802 | 809.165 | OH | a1 | B06 | b1 | C14 | c2 | D02 | d1 | E03 |
| 2-1-m1E03-1-0803 | 809.235 | OH | a1 | B04 | b1 | C31 | c3 | D02 | d1 | E03 |
| 2-1-m1E03-1-0804 | 810.076 | OH | a2 | B17 | b3 | C06 | c2 | D02 | d1 | E03 |
| 2-1-m1E03-1-0805 | 810.164 | OH | a2 | B18 | b3 | C17 | c1 | D01 | d1 | E03 |
| 2-1-m1E03-1-0806 | 810.164 | OH | a2 | B18 | b1 | C14 | c2 | D02 | d1 | E03 |
| 2-1-m1E03-1-0807 | 811.091 | OH | a1 | B04 | b3 | C06 | c2 | D02 | d1 | E03 |
| 2-1-m1E03-1-0808 | 811.140 | OH | a2 | B17 | b3 | C18 | c1 | D01 | d1 | E03 |
| 2-1-m1E03-1-0809 | 811.169 | OH | a1 | B07 | b1 | C13 | c2 | D02 | d1 | E03 |
| 2-1-m1E03-1-0810 | 811.190 | OH | a1 | B10 | b1 | C09 | c1 | D01 | d1 | E03 |
| 2-1-m1E03-1-0811 | 811.271 | OH | a1 | B05 | b2 | C07 | c2 | D02 | d1 | E03 |
| 2-1-m1E03-1-0812 | 812.156 | OH | a1 | B04 | b3 | C18 | c1 | D01 | d1 | E03 |
| 2-1-m1E03-1-0813 | 812.156 | OH | a1 | B08 | b2 | C24 | c3 | D02 | d1 | E03 |
| 2-1-m1E03-1-0814 | 812.231 | OH | a2 | B19 | b1 | C29 | c3 | D02 | d1 | E03 |
| 2-1-m1E03-1-0815 | 813.104 | OH | a1 | B07 | b3 | C15 | c2 | D02 | d1 | E03 |
| 2-1-m1E03-1-0816 | 813.121 | OH | a1 | B10 | b3 | C06 | c1 | D01 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0817 | 813.174 | OH | a1 | B07 | b3 | C16 | c1 | D01 | d1 | E03 |
| 2-1-m1E03-1-0818 | 813.250 | OH | a1 | B06 | b2 | C07 | c2 | D02 | d1 | E03 |
| 2-1-m1E03-1-0819 | 814.101 | OH | a1 | B08 | b2 | C06 | c2 | D02 | d1 | E03 |
| 2-1-m1E03-1-0820 | 814.116 | OH | a1 | B10 | b1 | C12 | c2 | D02 | d1 | E03 |
| 2-1-m1E03-1-0821 | 814.211 | OH | a2 | B20 | b1 | C32 | c3 | D02 | d1 | E03 |
| 2-1-m1E03-1-0822 | 814.249 | OH | a2 | B18 | b2 | C07 | c2 | D02 | d1 | E03 |
| 2-1-m1E03-1-0823 | 815.135 | OH | a1 | B07 | b3 | C17 | c1 | D01 | d1 | E03 |
| 2-1-m1E03-1-0824 | 815.135 | OH | a1 | B07 | b1 | C14 | c2 | D02 | d1 | E03 |
| 2-1-m1E03-1-0825 | 815.170 | OH | a2 | B19 | b2 | C05 | c2 | D02 | d1 | E03 |
| 2-1-m1E03-1-0826 | 815.175 | OH | a1 | B05 | b3 | C08 | c2 | D02 | d1 | E03 |
| 2-1-m1E03-1-0827 | 816.154 | OH | a2 | B20 | b2 | C02 | c2 | D02 | d1 | E03 |
| 2-1-m1E03-1-0828 | 816.190 | OH | a2 | B20 | b2 | C22 | c3 | D02 | d1 | E03 |
| 2-1-m1E03-1-0829 | 816.190 | OH | a2 | B19 | b2 | C26 | c3 | D02 | d1 | E03 |
| 2-1-m1E03-1-0830 | 817.149 | OH | a2 | B20 | b2 | C03 | c2 | D02 | d1 | E03 |
| 2-1-m1E03-1-0831 | 817.155 | OH | a1 | B06 | b3 | C08 | c2 | D02 | d1 | E03 |
| 2-1-m1E03-1-0832 | 817.222 | OH | a1 | B09 | b1 | C32 | c3 | D02 | d1 | E03 |
| 2-1-m1E03-1-0833 | 818.149 | OH | a2 | B19 | b2 | C04 | c2 | D02 | d1 | E03 |
| 2-1-m1E03-1-0834 | 818.154 | OH | a2 | B18 | b3 | C08 | c2 | D02 | d1 | E03 |
| 2-1-m1E03-1-0835 | 819.164 | OH | a1 | B09 | b2 | C02 | c2 | D02 | d1 | E03 |
| 2-1-m1E03-1-0836 | 819.201 | OH | a1 | B05 | b1 | C09 | c2 | D02 | d1 | E03 |
| 2-1-m1E03-1-0837 | 819.201 | OH | a1 | B09 | b2 | C22 | c3 | D02 | d1 | E03 |
| 2-1-m1E03-1-0838 | 819.201 | OH | a1 | B10 | b1 | C30 | c3 | D02 | d1 | E03 |
| 2-1-m1E03-1-0839 | 819.221 | OH | a1 | B07 | b2 | C07 | c2 | D02 | d1 | E03 |
| 2-1-m1E03-1-0840 | 819.276 | OH | a1 | B05 | b1 | C31 | c3 | D02 | d1 | E03 |
| 2-1-m1E03-1-0841 | 820.160 | OH | a1 | B09 | b2 | C03 | c2 | D02 | d1 | E03 |
| 2-1-m1E03-1-0842 | 820.165 | OH | a2 | B20 | b2 | C24 | c3 | D02 | d1 | E03 |
| 2-1-m1E03-1-0843 | 820.217 | OH | a1 | B08 | b1 | C29 | c3 | D02 | d1 | E03 |
| 2-1-m1E03-1-0844 | 821.132 | OH | a1 | B05 | b3 | C06 | c2 | D02 | d1 | E03 |
| 2-1-m1E03-1-0845 | 821.180 | OH | a1 | B06 | b1 | C09 | c2 | D02 | d1 | E03 |
| 2-1-m1E03-1-0846 | 821.180 | OH | a1 | B10 | b2 | C20 | c3 | D02 | d1 | E03 |
| 2-1-m1E03-1-0847 | 821.255 | OH | a1 | B06 | b1 | C31 | c3 | D02 | d1 | E03 |
| 2-1-m1E03-1-0848 | 822.11 | OH | a2 | B20 | b2 | C06 | c2 | D02 | d1 | E03 |
| 2-1-m1E03-1-0849 | 822.149 | OH | a2 | B11 | b3 | C16 | c2 | D02 | d1 | E03 |
| 2-1-m1E03-1-0850 | 822.175 | OH | a1 | B10 | b2 | C28 | c3 | D02 | d1 | E03 |
| 2-1-m1E03-1-0851 | 822.179 | OH | a2 | B18 | b1 | C09 | c2 | D02 | d1 | E03 |
| 2-1-m1E03-1-0852 | 822.196 | OH | a1 | B05 | b3 | C18 | c1 | D01 | d1 | E03 |
| 2-1-m1E03-1-0853 | 822.255 | OH | a2 | B18 | b1 | C31 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0854 | 823.111 | OH | a1 | B06 | b3 | C06 | c2 | D02 | d1 | E03 |
| 2-1-m1E03-1-0855 | 823.125 | OH | a1 | B07 | b3 | C08 | c2 | D02 | d1 | E03 |
| 2-1-m1E03-1-0856 | 823.144 | OH | a2 | B12 | b3 | C16 | c2 | D02 | d1 | E03 |
| 2-1-m1E03-1-0857 | 823.155 | OH | a1 | B08 | b2 | C05 | c2 | D02 | d1 | E03 |
| 2-1-m1E03-1-0858 | 823.176 | OH | a1 | B09 | b2 | C24 | c3 | D02 | d1 | E03 |
| 2-1-m1E03-1-0859 | 824.110 | OH | a2 | B18 | b3 | C06 | c2 | D02 | d1 | E03 |
| 2-1-m1E03-1-0860 | 824.110 | OH | a2 | B11 | b3 | C17 | c2 | D02 | d1 | E03 |
| 2-1-m1E03-1-0861 | 824.170 | OH | a2 | B19 | b1 | C13 | c2 | D02 | d1 | E03 |
| 2-1-m1E03-1-0862 | 824.176 | OH | a1 | B06 | b3 | C18 | c1 | D01 | d1 | E03 |
| 2-1-m1E03-1-0863 | 824.176 | OH | a1 | B08 | b2 | C26 | c3 | D02 | d1 | E03 |
| 2-1-m1E03-1-0864 | 825.106 | OH | a2 | B12 | b3 | C17 | c2 | D02 | d1 | E03 |
| 2-1-m1E03-1-0865 | 825.121 | OH | a1 | B09 | b2 | C06 | c2 | D02 | d1 | E03 |
| 2-1-m1E03-1-0866 | 825.139 | OH | a1 | B10 | b2 | C01 | c2 | D02 | d1 | E03 |
| 2-1-m1E03-1-0867 | 825.175 | OH | a2 | B18 | b3 | C18 | c1 | D01 | d1 | E03 |
| 2-1-m1E03-1-0868 | 826.105 | OH | a2 | B19 | b3 | C15 | c2 | D02 | d1 | E03 |
| 2-1-m1E03-1-0869 | 826.135 | OH | a1 | B08 | b2 | C04 | c2 | D02 | d1 | E03 |
| 2-1-m1E03-1-0870 | 826.174 | OH | a2 | B19 | b3 | C16 | c1 | D01 | d1 | E03 |
| 2-1-m1E03-1-0871 | 827.151 | OH | a1 | B07 | b1 | C09 | c2 | D02 | d1 | E03 |
| 2-1-m1E03-1-0872 | 827.226 | OH | a1 | B07 | b1 | C31 | c3 | D02 | d1 | E03 |
| 2-1-m1E03-1-0873 | 828.136 | OH | a2 | B19 | b3 | C17 | c1 | D01 | d1 | E03 |
| 2-1-m1E03-1-0874 | 828.136 | OH | a2 | B19 | b1 | C14 | c2 | D02 | d1 | E03 |
| 2-1-m1E03-1-0875 | 828.226 | OH | a2 | B20 | b1 | C29 | c3 | D02 | d1 | E03 |
| 2-1-m1E03-1-0876 | 829.082 | OH | a1 | B07 | b3 | C06 | c2 | D02 | d1 | E03 |
| 2-1-m1E03-1-0877 | 830.146 | OH | a1 | B07 | b3 | C18 | c1 | D01 | d1 | E03 |
| 2-1-m1E03-1-0878 | 831.164 | OH | a2 | B20 | b2 | C05 | c2 | D02 | d1 | E03 |
| 2-1-m1E03-1-0879 | 831.237 | OH | a1 | B09 | b1 | C29 | c3 | D02 | d1 | E03 |
| 2-1-m1E03-1-0880 | 832.156 | OH | a1 | B08 | b1 | C13 | c2 | D02 | d1 | E03 |
| 2-1-m1E03-1-0881 | 832.185 | OH | a2 | B20 | b2 | C26 | c3 | D02 | d1 | E03 |
| 2-1-m1E03-1-0882 | 832.221 | OH | a2 | B19 | b2 | C07 | c2 | D02 | d1 | E03 |
| 2-1-m1E03-1-0883 | 833.216 | OH | a1 | B10 | b1 | C32 | c3 | D02 | d1 | E03 |
| 2-1-m1E03-1-0884 | 834.091 | OH | a1 | B08 | b3 | C15 | c2 | D02 | d1 | E03 |
| 2-1-m1E03-1-0885 | 834.144 | OH | a2 | B20 | b2 | C04 | c2 | D02 | d1 | E03 |
| 2-1-m1E03-1-0886 | 834.160 | OH | a1 | B08 | b3 | C16 | c1 | D01 | d1 | E03 |
| 2-1-m1E03-1-0887 | 834.175 | OH | a1 | B09 | b2 | C05 | c2 | D02 | d1 | E03 |
| 2-1-m1E03-1-0888 | 835.159 | OH | a1 | B10 | b2 | C02 | c2 | D02 | d1 | E03 |
| 2-1-m1E03-1-0889 | 835.196 | OH | a1 | B10 | b2 | C22 | c3 | D02 | d1 | E03 |
| 2-1-m1E03-1-0890 | 835.196 | OH | a1 | B09 | b2 | C26 | c3 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0891 | 836.122 | OH | a1 | B08 | b3 | C17 | c1 | D01 | d1 | E03 |
| 2-1-m1E03-1-0892 | 836.122 | OH | a1 | B08 | b1 | C14 | c2 | D02 | d1 | E03 |
| 2-1-m1E03-1-0893 | 836.126 | OH | a2 | B19 | b3 | C08 | c2 | D02 | d1 | E03 |
| 2-1-m1E03-1-0894 | 836.155 | OH | a1 | B10 | b2 | C03 | c2 | D02 | d1 | E03 |
| 2-1-m1E03-1-0895 | 836.164 | OH | a2 | B13 | b3 | C16 | c2 | D02 | d1 | E03 |
| 2-1-m1E03-1-0896 | 837.155 | OH | a1 | B09 | b2 | C04 | c2 | D02 | d1 | E03 |
| 2-1-m1E03-1-0897 | 838.126 | OH | a2 | B13 | b3 | C17 | c2 | D02 | d1 | E03 |
| 2-1-m1E03-1-0898 | 839.121 | OH | a2 | B11 | b3 | C18 | c2 | D02 | d1 | E03 |
| 2-1-m1E03-1-0899 | 839.171 | OH | a1 | B10 | b2 | C24 | c3 | D02 | d1 | E03 |
| 2-1-m1E03-1-0900 | 840.116 | OH | a2 | B12 | b3 | C18 | c2 | D02 | d1 | E03 |
| 2-1-m1E03-1-0901 | 840.139 | OH | a2 | B14 | b3 | C16 | c2 | D02 | d1 | E03 |
| 2-1-m1E03-1-0902 | 840.151 | OH | a2 | B19 | b1 | C09 | c2 | D02 | d1 | E03 |
| 2-1-m1E03-1-0903 | 840.165 | OH | a2 | B20 | b1 | C13 | c2 | D02 | d1 | E03 |
| 2-1-m1E03-1-0904 | 840.207 | OH | a1 | B08 | b2 | C07 | c2 | D02 | d1 | E03 |
| 2-1-m1E03-1-0905 | 840.226 | OH | a2 | B19 | b1 | C31 | c3 | D02 | d1 | E03 |
| 2-1-m1E03-1-0906 | 841.116 | OH | a1 | B10 | b2 | C06 | c2 | D02 | d1 | E03 |
| 2-1-m1E03-1-0907 | 841.155 | OH | a1 | B01 | b3 | C16 | c2 | D02 | d1 | E03 |
| 2-1-m1E03-1-0908 | 842.082 | OH | a2 | B19 | b3 | C06 | c2 | D02 | d1 | E03 |
| 2-1-m1E03-1-0909 | 842.100 | OH | a2 | B20 | b3 | C15 | c2 | D02 | d1 | E03 |
| 2-1-m1E03-1-0910 | 842.101 | OH | a2 | B14 | b3 | C17 | c2 | D02 | d1 | E03 |
| 2-1-m1E03-1-0911 | 842.150 | OH | a1 | B02 | b3 | C16 | c2 | D02 | d1 | E03 |
| 2-1-m1E03-1-0912 | 842.169 | OH | a2 | B20 | b3 | C16 | c1 | D01 | d1 | E03 |
| 2-1-m1E03-1-0913 | 843.116 | OH | a1 | B01 | b3 | C17 | c2 | D02 | d1 | E03 |
| 2-1-m1E03-1-0914 | 843.147 | OH | a2 | B19 | b3 | C18 | c1 | D01 | d1 | E03 |
| 2-1-m1E03-1-0915 | 843.176 | OH | a1 | B09 | b1 | C13 | c2 | D02 | d1 | E03 |
| 2-1-m1E03-1-0916 | 844.111 | OH | a1 | B02 | b3 | C17 | c2 | D02 | d1 | E03 |
| 2-1-m1E03-1-0917 | 844.111 | OH | a1 | B08 | b3 | C08 | c2 | D02 | d1 | E03 |
| 2-1-m1E03-1-0918 | 844.131 | OH | a2 | B20 | b3 | C17 | c1 | D01 | d1 | E03 |
| 2-1-m1E03-1-0919 | 844.131 | OH | a2 | B20 | b1 | C14 | c2 | D02 | d1 | E03 |
| 2-1-m1E03-1-0920 | 845.111 | OH | a1 | B09 | b3 | C15 | c2 | D02 | d1 | E03 |
| 2-1-m1E03-1-0921 | 845.180 | OH | a1 | B09 | b3 | C16 | c1 | D01 | d1 | E03 |
| 2-1-m1E03-1-0922 | 847.142 | OH | a1 | B09 | b3 | C17 | c1 | D01 | d1 | E03 |
| 2-1-m1E03-1-0923 | 847.142 | OH | a1 | B09 | b1 | C14 | c2 | D02 | d1 | E03 |
| 2-1-m1E03-1-0924 | 847.232 | OH | a1 | B10 | b1 | C29 | c3 | D02 | d1 | E03 |
| 2-1-m1E03-1-0925 | 848.137 | OH | a1 | B08 | b1 | C09 | c2 | D02 | d1 | E03 |
| 2-1-m1E03-1-0926 | 848.212 | OH | a1 | B08 | b1 | C31 | c3 | D02 | d1 | E03 |
| 2-1-m1E03-1-0927 | 848.216 | OH | a2 | B20 | b2 | C07 | c2 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0928 | 850.068 | OH | a1 | B08 | b3 | C06 | c2 | D02 | d1 | E03 |
| 2-1-m1E03-1-0929 | 850.170 | OH | a1 | B10 | b2 | C05 | c2 | D02 | d1 | E03 |
| 2-1-m1E03-1-0930 | 850.180 | OH | a2 | B15 | b3 | C16 | c2 | D02 | d1 | E03 |
| 2-1-m1E03-1-0931 | 851.132 | OH | a1 | B08 | b3 | C18 | c1 | D01 | d1 | E03 |
| 2-1-m1E03-1-0932 | 851.191 | OH | a1 | B10 | b2 | C26 | c3 | D02 | d1 | E03 |
| 2-1-m1E03-1-0933 | 851.227 | OH | a1 | B09 | b2 | C07 | c2 | D02 | d1 | E03 |
| 2-1-m1E03-1-0934 | 852.121 | OH | a2 | B20 | b3 | C08 | c2 | D02 | d1 | E03 |
| 2-1-m1E03-1-0935 | 852.142 | OH | a2 | B15 | b3 | C17 | c2 | D02 | d1 | E03 |
| 2-1-m1E03-1-0936 | 852.159 | OH | a2 | B16 | b3 | C16 | c2 | D02 | d1 | E03 |
| 2-1-m1E03-1-0937 | 853.137 | OH | a2 | B13 | b3 | C18 | c2 | D02 | d1 | E03 |
| 2-1-m1E03-1-0938 | 853.150 | OH | a1 | B10 | b2 | C04 | c2 | D02 | d1 | E03 |
| 2-1-m1E03-1-0939 | 854.121 | OH | a2 | B16 | b3 | C17 | c2 | D02 | d1 | E03 |
| 2-1-m1E03-1-0940 | 855.131 | OH | a1 | B09 | b3 | C08 | c2 | D02 | d1 | E03 |
| 2-1-m1E03-1-0941 | 855.170 | OH | a1 | B03 | b3 | C16 | c2 | D02 | d1 | E03 |
| 2-1-m1E03-1-0942 | 856.146 | OH | a2 | B20 | b1 | C09 | c2 | D02 | d1 | E03 |
| 2-1-m1E03-1-0943 | 856.221 | OH | a2 | B20 | b1 | C31 | c3 | D02 | d1 | E03 |
| 2-1-m1E03-1-0944 | 857.112 | OH | a2 | B14 | b3 | C18 | c2 | D02 | d1 | E03 |
| 2-1-m1E03-1-0945 | 857.132 | OH | a1 | B03 | b3 | C17 | c2 | D02 | d1 | E03 |
| 2-1-m1E03-1-0946 | 858.077 | OH | a2 | B20 | b3 | C06 | c2 | D02 | d1 | E03 |
| 2-1-m1E03-1-0947 | 858.127 | OH | a1 | B01 | b3 | C18 | c2 | D02 | d1 | E03 |
| 2-1-m1E03-1-0948 | 858.130 | OH | a2 | B17 | b3 | C16 | c2 | D02 | d1 | E03 |
| 2-1-m1E03-1-0949 | 859.122 | OH | a1 | B02 | b3 | C18 | c2 | D02 | d1 | E03 |
| 2-1-m1E03-1-0950 | 859.142 | OH | a2 | B20 | b3 | C18 | c1 | D01 | d1 | E03 |
| 2-1-m1E03-1-0951 | 859.145 | OH | a1 | B04 | b3 | C16 | c2 | D02 | d1 | E03 |
| 2-1-m1E03-1-0952 | 859.157 | OH | a1 | B09 | b1 | C09 | c2 | D02 | d1 | E03 |
| 2-1-m1E03-1-0953 | 859.171 | OH | a1 | B10 | b1 | C13 | c2 | D02 | d1 | E03 |
| 2-1-m1E03-1-0954 | 859.232 | OH | a1 | B09 | b1 | C31 | c3 | D02 | d1 | E03 |
| 2-1-m1E03-1-0955 | 860.091 | OH | a2 | B17 | b3 | C17 | c2 | D02 | d1 | E03 |
| 2-1-m1E03-1-0956 | 861.088 | OH | a1 | B09 | b3 | C06 | c2 | D02 | d1 | E03 |
| 2-1-m1E03-1-0957 | 861.106 | OH | a1 | B10 | b3 | C15 | c2 | D02 | d1 | E03 |
| 2-1-m1E03-1-0958 | 861.107 | OH | a1 | B04 | b3 | C17 | c2 | D02 | d1 | E03 |
| 2-1-m1E03-1-0959 | 861.175 | OH | a1 | B10 | b3 | C16 | c1 | D01 | d1 | E03 |
| 2-1-m1E03-1-0960 | 862.152 | OH | a1 | B09 | b3 | C18 | c1 | D01 | d1 | E03 |
| 2-1-m1E03-1-0961 | 863.137 | OH | a1 | B10 | b3 | C17 | c1 | D01 | d1 | E03 |
| 2-1-m1E03-1-0962 | 863.137 | OH | a1 | B10 | b1 | C14 | c2 | D02 | d1 | E03 |
| 2-1-m1E03-1-0963 | 867.152 | OH | a2 | B15 | b3 | C18 | c2 | D02 | d1 | E03 |
| 2-1-m1E03-1-0964 | 867.222 | OH | a1 | B10 | b2 | C07 | c2 | D02 | d1 | E03 |

(continued)

| [Table 8-8-4] Compound that may be contained in mixture 2-1-m1E03-1 | | | | | | | | | | |
|---|---|---|---|---|---|---|---|---|---|---|
| ID | Exact Mass | | a | B | b | C | c | D | d | E |
| 2-1-m1E03-1-0965 | 869.132 | OH | a2 | B16 | b3 | C18 | c2 | D02 | d1 | E03 |
| 2-1-m1E03-1-0966 | 869.186 | OH | a1 | B05 | b3 | C16 | c2 | D02 | d1 | E03 |
| 2-1-m1E03-1-0967 | 871.126 | OH | a1 | B10 | b3 | C08 | c2 | D02 | d1 | E03 |
| 2-1-m1E03-1-0968 | 871.147 | OH | a1 | B05 | b3 | C17 | c2 | D02 | d1 | E03 |
| 2-1-m1E03-1-0969 | 871.165 | OH | a1 | B06 | b3 | C16 | c2 | D02 | d1 | E03 |
| 2-1-m1E03-1-0970 | 872.143 | OH | a1 | B03 | b3 | C18 | c2 | D02 | d1 | E03 |
| 2-1-m1E03-1-0971 | 872.164 | OH | a2 | B18 | b3 | C16 | c2 | D02 | d1 | E03 |
| 2-1-m1E03-1-0972 | 873.127 | OH | a1 | B06 | b3 | C17 | c2 | D02 | d1 | E03 |
| 2-1-m1E03-1-0973 | 874.126 | OH | a2 | B18 | b3 | C17 | c2 | D02 | d1 | E03 |
| 2-1-m1E03-1-0974 | 875.102 | OH | a2 | B17 | b3 | C18 | c2 | D02 | d1 | E03 |
| 2-1-m1E03-1-0975 | 875.152 | OH | a1 | B10 | b1 | C09 | c2 | D02 | d1 | E03 |
| 2-1-m1E03-1-0976 | 875.227 | OH | a1 | B10 | b1 | C31 | c3 | D02 | d1 | E03 |
| 2-1-m1E03-1-0977 | 876.118 | OH | a1 | B04 | b3 | C18 | c2 | D02 | d1 | E03 |
| 2-1-m1E03-1-0978 | 877.083 | OH | a1 | B10 | b3 | C06 | c2 | D02 | d1 | E03 |
| 2-1-m1E03-1-0979 | 877.136 | OH | a1 | B07 | b3 | C16 | c2 | D02 | d1 | E03 |
| 2-1-m1E03-1-0980 | 878.147 | OH | a1 | B10 | b3 | C18 | c1 | D01 | d1 | E03 |
| 2-1-m1E03-1-0981 | 879.097 | OH | a1 | B07 | b3 | C17 | c2 | D02 | d1 | E03 |
| 2-1-mlE03-1-0982 | 886.158 | OH | a1 | B05 | b3 | C18 | c2 | D02 | d1 | E03 |
| 2-1-m1E03-1-0983 | 888.138 | OH | a1 | B06 | b3 | C18 | c2 | D02 | d1 | E03 |
| 2-1-m1E03-1-0984 | 889.137 | OH | a2 | B18 | b3 | C18 | c2 | D02 | d1 | E03 |
| 2-1-m1E03-1-0985 | 890.136 | OH | a2 | B19 | b3 | C16 | c2 | D02 | d1 | E03 |
| 2-1-m1E03-1-0986 | 892.098 | OH | a2 | B19 | b3 | C17 | c2 | D02 | d1 | E03 |
| 2-1-m1E03-1-0987 | 894.108 | OH | a1 | B07 | b3 | C18 | c2 | D02 | d1 | E03 |
| 2-1-m1E03-1-0988 | 898.122 | OH | a1 | B08 | b3 | C16 | c2 | D02 | d1 | E03 |
| 2-1-m1E03-1-0989 | 900.083 | OH | a1 | B08 | b3 | C17 | c2 | D02 | d1 | E03 |
| 2-1-m1E03-1-0990 | 906.131 | OH | a2 | B20 | b3 | C16 | c2 | D02 | d1 | E03 |
| 2-1-m1E03-1-0991 | 907.109 | OH | a2 | B19 | b3 | C18 | c2 | D02 | d1 | E03 |
| 2-1-m1E03-1-0992 | 908.093 | OH | a2 | B20 | b3 | C17 | c2 | D02 | d1 | E03 |
| 2-1-m1E03-1-0993 | 909.142 | OH | a1 | B09 | b3 | C16 | c2 | D02 | d1 | E03 |
| 2-1-m1E03-1-0994 | 911.103 | OH | a1 | B09 | b3 | C17 | c2 | D02 | d1 | E03 |
| 2-1-m1E03-1-0995 | 915.094 | OH | a1 | B08 | b3 | C18 | c2 | D02 | d1 | E03 |
| 2-1-m1E03-1-0996 | 923.103 | OH | a2 | B20 | b3 | C18 | c2 | D02 | d1 | E03 |
| 2-1-m1E03-1-0997 | 925.137 | OH | a1 | B10 | b3 | C16 | c2 | D02 | d1 | E03 |
| 2-1-m1E03-1-0998 | 926.114 | OH | a1 | B09 | b3 | C18 | c2 | D02 | d1 | E03 |
| 2-1-m1E03-1-0999 | 927.098 | OH | a1 | B10 | b3 | C17 | c2 | D02 | d1 | E03 |
| 2-1-m1E03-1-1000 | 942.109 | OH | a1 | B10 | b3 | C18 | c2 | D02 | d1 | E03 |

[Table 397]

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0001 | 753.298 | OH | a2 | B11 | b1 | C08 | c1 | D01 | d1 | E04 |
| 2-1-m1E04-1-0002 | 754.294 | OH | a2 | B12 | b1 | C08 | c1 | D01 | d1 | E04 |
| 2-1-m1E04-1-0003 | 754.294 | OH | a2 | B11 | b1 | C11 | c1 | D01 | d1 | E04 |
| 2-1-m1E04-1-0004 | 755.289 | OH | a2 | B11 | b1 | C10 | c1 | D01 | d1 | E04 |
| 2-1-m1E04-1-0005 | 755.289 | OH | a2 | B12 | b1 | C11 | c1 | D01 | d1 | E04 |
| 2-1-m1E04-1-0006 | 756.284 | OH | a2 | B12 | b1 | C10 | c1 | D01 | d1 | E04 |
| 2-1-m1E04-1-0007 | 760.250 | OH | a2 | B11 | b1 | C12 | c1 | D01 | d1 | E04 |
| 2-1-m1E04-1-0008 | 761.245 | OH | a2 | B12 | b1 | C12 | c1 | D01 | d1 | E04 |
| 2-1-m1E04-1-0009 | 767.314 | OH | a2 | B13 | b1 | C08 | c1 | D01 | d1 | E04 |
| 2-1-m1E04-1-0010 | 768.309 | OH | a2 | B13 | b1 | C11 | c1 | D01 | d1 | E04 |
| 2-1-m1E04-1-0011 | 769.305 | OH | a2 | B13 | b1 | C10 | c1 | D01 | d1 | E04 |
| 2-1-m1E04-1-0012 | 771.273 | OH | a2 | B11 | b2 | C01 | c1 | D01 | d1 | E04 |
| 2-1-m1E04-1-0013 | 771.289 | OH | a2 | B14 | b1 | C08 | c1 | D01 | d1 | E04 |
| 2-1-m1E04-1-0014 | 772.268 | OH | a2 | B12 | b2 | C01 | c1 | D01 | d1 | E04 |
| 2-1-m1E04-1-0015 | 772.284 | OH | a2 | B14 | b1 | C11 | c1 | D01 | d1 | E04 |
| 2-1-m1E04-1-0016 | 772.304 | OH | a1 | B01 | b1 | C08 | c1 | D01 | d1 | E04 |
| 2-1-m1E04-1-0017 | 773.280 | OH | a2 | B14 | b1 | C10 | c1 | D01 | d1 | E04 |
| 2-1-m1E04-1-0018 | 773.300 | OH | a1 | B02 | b1 | C08 | c1 | D01 | d1 | E04 |
| 2-1-m1E04-1-0019 | 773.300 | OH | a1 | B01 | b1 | C11 | c1 | D01 | d1 | E04 |
| 2-1-m1E04-1-0020 | 774.266 | OH | a2 | B13 | b1 | C12 | c1 | D01 | d1 | E04 |
| 2-1-m1E04-1-0021 | 774.295 | OH | a1 | B01 | b1 | C10 | c1 | D01 | d1 | E04 |
| 2-1-m1E04-1-0022 | 774.295 | OH | a1 | B02 | b1 | C11 | c1 | D01 | d1 | E04 |
| 2-1-m1E04-1-0023 | 775.290 | OH | a1 | B02 | b1 | C10 | c1 | D01 | d1 | E04 |
| 2-1-m1E04-1-0024 | 778.241 | OH | a2 | B14 | b1 | C12 | c1 | D01 | d1 | E04 |
| 2-1-m1E04-1-0025 | 779.256 | OH | a1 | B01 | b1 | C12 | c1 | D01 | d1 | E04 |
| 2-1-m1E04-1-0026 | 780.251 | OH | a1 | B02 | b1 | C12 | c1 | D01 | d1 | E04 |
| 2-1-m1E04-1-0027 | 781.293 | OH | a2 | B11 | b2 | C02 | c1 | D01 | d1 | E04 |
| 2-1-m1E04-1-0028 | 781.330 | OH | a2 | B15 | b1 | C08 | c1 | D01 | d1 | E04 |
| 2-1-m1E04-1-0029 | 782.289 | OH | a2 | B12 | b2 | C02 | c1 | D01 | d1 | E04 |
| 2-1-m1E04-1-0030 | 782.289 | OH | a2 | B11 | b2 | C03 | c1 | D01 | d1 | E04 |
| 2-1-m1E04-1-0031 | 782.325 | OH | a2 | B15 | b1 | C11 | c1 | D01 | d1 | E04 |
| 2-1-m1E04-1-0032 | 783.284 | OH | a2 | B12 | b2 | C03 | c1 | D01 | d1 | E04 |
| 2-1-m1E04-1-0033 | 783.309 | OH | a2 | B16 | b1 | C08 | c1 | D01 | d1 | E04 |
| 2-1-m1E04-1-0034 | 783.320 | OH | a2 | B15 | b1 | C10 | c1 | D01 | d1 | E04 |
| 2-1-m1E04-1-0035 | 784.304 | OH | a2 | B16 | b1 | C11 | c1 | D01 | d1 | E04 |
| 2-1-m1E04-1-0036 | 785.288 | OH | a2 | B13 | b2 | C01 | c1 | D01 | d1 | E04 |
| 2-1-m1E04-1-0037 | 785.300 | OH | a2 | B16 | b1 | C10 | c1 | D01 | d1 | E04 |
| 2-1-m1E04-1-0038 | 786.32 | OH | a1 | B03 | b1 | C08 | c1 | D01 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0039 | 787.250 | OH | a2 | B11 | b2 | C06 | c1 | D01 | d1 | E04 |
| 2-1-m1E04-1-0040 | 787.315 | OH | a1 | B03 | b1 | C11 | c1 | D01 | d1 | E04 |
| 2-1-m1E04-1-0041 | 788.245 | OH | a2 | B12 | b2 | C06 | c1 | D01 | d1 | E04 |
| 2-1-m1E04-1-0042 | 788.281 | OH | a2 | B15 | b1 | C12 | c1 | D01 | d1 | E04 |
| 2-1-m1E04-1-0043 | 788.310 | OH | a1 | B03 | b1 | C10 | c1 | D01 | d1 | E04 |
| 2-1-m1E04-1-0044 | 789.263 | OH | a2 | B14 | b2 | C01 | c1 | D01 | d1 | E04 |
| 2-1-m1E04-1-0045 | 789.280 | OH | a2 | B17 | b1 | C08 | c1 | D01 | d1 | E04 |
| 2-1-m1E04-1-0046 | 790.261 | OH | a2 | B16 | b1 | C12 | c1 | D01 | d1 | E04 |
| 2-1-m1E04-1-0047 | 790.275 | OH | a2 | B17 | b1 | C11 | c1 | D01 | d1 | E04 |
| 2-1-m1E04-1-0048 | 790.278 | OH | a1 | B01 | b2 | C01 | c1 | D01 | d1 | E04 |
| 2-1-m1E04-1-0049 | 790.295 | OH | a1 | B04 | b1 | C08 | c1 | D01 | d1 | E04 |
| 2-1-m1E04-1-0050 | 791.270 | OH | a2 | B17 | b1 | C10 | c1 | D01 | d1 | E04 |
| 2-1-m1E04-1-0051 | 791.274 | OH | a1 | B02 | b2 | C01 | c1 | D01 | d1 | E04 |
| 2-1-m1E04-1-0052 | 791.290 | OH | a1 | B04 | b1 | C11 | c1 | D01 | d1 | E04 |
| 2-1-m1E04-1-0053 | 792.285 | OH | a1 | B04 | b1 | C10 | c1 | D01 | d1 | E04 |
| 2-1-m1E04-1-0054 | 793.272 | OH | a1 | B03 | b1 | C12 | c1 | D01 | d1 | E04 |
| 2-1-m1E04-1-0055 | 795.309 | OH | a2 | B13 | b2 | C02 | c1 | D01 | d1 | E04 |
| 2-1-m1E04-1-0056 | 796.231 | OH | a2 | B17 | b1 | C12 | c1 | D01 | d1 | E04 |
| 2-1-m1E04-1-0057 | 796.304 | OH | a2 | B13 | b2 | C03 | c1 | D01 | d1 | E04 |
| 2-1-m1E04-1-0058 | 796.304 | OH | a2 | B11 | b2 | C05 | c1 | D01 | d1 | E04 |
| 2-1-m1E04-1-0059 | 797.247 | OH | a1 | B04 | b1 | C12 | c1 | D01 | d1 | E04 |
| 2-1-m1E04-1-0060 | 797.300 | OH | a2 | B12 | b2 | COS | c1 | D01 | d1 | E04 |
| 2-1-m1E04-1-0061 | 799.284 | OH | a2 | B14 | b2 | C02 | c1 | D01 | d1 | E04 |
| 2-1-m1E04-1-0062 | 799.284 | OH | a2 | B11 | b2 | C04 | c1 | D01 | d1 | E04 |
| 2-1-m1E04-1-0063 | 799.304 | OH | a2 | B15 | b2 | C01 | c1 | D01 | d1 | E04 |
| 2-1-m1E04-1-0064 | 800.279 | OH | a2 | B12 | b2 | C04 | c1 | D01 | d1 | E04 |
| 2-1-m1E04-1-0065 | 800.279 | OH | a2 | B14 | b2 | C03 | c1 | D01 | d1 | E04 |
| 2-1-m1E04-1-0066 | 800.299 | OH | a1 | B01 | b2 | C02 | c1 | D01 | d1 | E04 |
| 2-1-m1E04-1-0067 | 800.336 | OH | a1 | B05 | b1 | C08 | c1 | D01 | d1 | E04 |
| 2-1-m1E04-1-0068 | 801.265 | OH | a2 | B13 | b2 | C06 | c1 | D01 | d1 | E04 |
| 2-1-m1E04-1-0069 | 801.283 | OH | a2 | B16 | b2 | C01 | c1 | D01 | d1 | E04 |
| 2-1-m1E04-1-0070 | 801.294 | OH | a1 | B02 | b2 | C02 | c1 | D01 | d1 | E04 |
| 2-1-m1E04-1-0071 | 801.294 | OH | a1 | B01 | b2 | C03 | c1 | D01 | d1 | E04 |
| 2-1-m1E04-1-0072 | 801.331 | OH | a1 | B05 | b1 | C11 | c1 | D01 | d1 | E04 |
| 2-1-m1E04-1-0073 | 802.290 | OH | a1 | B02 | b2 | C03 | c1 | D01 | d1 | E04 |
| 2-1-m1E04-1-0074 | 802.315 | OH | a1 | B06 | b1 | C08 | c1 | D01 | d1 | E04 |
| 2-1-m1E04-1-0075 | 802.326 | OH | a1 | B05 | b1 | C10 | c1 | D01 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0076 | 803.310 | OH | a1 | B06 | b1 | C11 | c1 | D01 | d1 | E04 |
| 2-1-m1E04-1-0077 | 803.314 | OH | a2 | B18 | b1 | C08 | c1 | D01 | d1 | E04 |
| 2-1-m1E04-1-0078 | 804.294 | OH | a1 | B03 | b2 | C01 | c1 | D01 | d1 | E04 |
| 2-1-m1E04-1-0079 | 804.305 | OH | a1 | B06 | b1 | C10 | c1 | D01 | d1 | E04 |
| 2-1-m1E04-1-0080 | 804.309 | OH | a2 | B18 | b1 | C11 | c1 | D01 | d1 | E04 |
| 2-1-m1E04-1-0081 | 805.240 | OH | a2 | B14 | b2 | C06 | c1 | DOI | d1 | E04 |
| 2-1-m1E04-1-0082 | 805.305 | OH | a2 | B18 | b1 | C10 | c1 | D01 | d1 | E04 |
| 2-1-m1E04-1-0083 | 805.305 | OH | a2 | B11 | b1 | C13 | c1 | D01 | d1 | E04 |
| 2-1-m1E04-1-0084 | 806.256 | OH | a1 | B01 | b2 | C06 | c1 | D01 | d1 | E04 |
| 2-1-m1E04-1-0085 | 806.300 | OH | a2 | B12 | b1 | C13 | c1 | D01 | d1 | E04 |
| 2-1-m1E04-1-0086 | 807.240 | OH | a2 | B11 | b3 | C15 | c1 | D01 | d1 | E04 |
| 2-1-m1E04-1-0087 | 807.251 | OH | a1 | B02 | b2 | C06 | c1 | D01 | d1 | E04 |
| 2-1-m1E04-1-0088 | 807.254 | OH | a2 | B17 | b2 | C01 | c1 | D01 | d1 | E04 |
| 2-1-m1E04-1-0089 | 807.287 | OH | a1 | B05 | b1 | C12 | c1 | D01 | d1 | E04 |
| 2-1-m1E04-1-0090 | 808.235 | OH | a2 | B12 | b3 | C15 | c1 | D01 | d1 | E04 |
| 2-1-m1E04-1-0091 | 808.269 | OH | a1 | B04 | b2 | C01 | c1 | D01 | d1 | E04 |
| 2-1-m1E04-1-0092 | 808.285 | OH | a1 | B07 | b1 | C08 | c1 | D01 | d1 | E04 |
| 2-1-m1E04-1-0093 | 809.267 | OH | a1 | B06 | b1 | C12 | c1 | DOI | d1 | E04 |
| 2-1-m1E04-1-0094 | 809.271 | OH | a2 | B11 | b1 | C14 | c1 | D01 | d1 | E04 |
| 2-1-m1E04-1-0095 | 809.281 | OH | a1 | B07 | b1 | C11 | c1 | D01 | d1 | E04 |
| 2-1-m1E04-1-0096 | 809.292 | OH | a2 | B11 | b2 | C33 | c3 | D02 | d1 | E04 |
| 2-1-m1E04-1-0097 | 809.325 | OH | a2 | B15 | b2 | C02 | c1 | D01 | d1 | E04 |
| 2-1-m1E04-1-0098 | 810.266 | OH | a2 | B18 | b1 | C12 | c1 | D01 | d1 | E04 |
| 2-1-m1E04-1-0099 | 810.266 | OH | a2 | B12 | b1 | C14 | c1 | D01 | d1 | E04 |
| 2-1-m1E04-1-0100 | 810.276 | OH | a1 | B07 | b1 | C10 | c1 | D01 | d1 | E04 |
| 2-1-m1E04-1-0101 | 810.287 | OH | a2 | B12 | b2 | C33 | c3 | D02 | d1 | E04 |
| 2-1-m1E04-1-0102 | 810.320 | OH | a2 | B15 | b2 | C03 | c1 | D01 | d1 | E04 |
| 2-1-mlE04-1-0103 | 810.320 | OH | a2 | B13 | b2 | C05 | c1 | D01 | d1 | E04 |
| 2-1-m1E04-1-0104 | 811.304 | OH | a2 | B16 | b2 | C02 | c1 | D01 | d1 | E04 |
| 2-1-m1E04-1-0105 | 812.299 | OH | a2 | B16 | b2 | C03 | c1 | D01 | d1 | E04 |
| 2-1-m1E04-1-0106 | 813.300 | OH | a2 | B13 | b2 | C04 | c1 | D01 | d1 | E04 |
| 2-1-m1E04-1-0107 | 813.356 | OH | a2 | B11 | b2 | C07 | c1 | D01 | d1 | E04 |
| 2-1-mlE04-1-0108 | 814.295 | OH | a2 | B14 | b2 | C05 | c1 | D01 | d1 | E04 |
| 2-1-m1E04-1-0109 | 814.315 | OH | a1 | B03 | b2 | C02 | c1 | D01 | d1 | E04 |
| 2-1-m1E04-1-0110 | 814.351 | OH | a2 | B12 | b2 | C07 | c1 | D01 | d1 | E04 |
| 2-1-m1E04-1-0111 | 815.237 | OH | a1 | B07 | b1 | C12 | c1 | D01 | d1 | E04 |
| 2-1-m1E04-1-0112 | 815.281 | OH | a2 | B15 | b2 | C06 | c1 | D01 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0113 | 815.310 | OH | a1 | B03 | b2 | C03 | c1 | D01 | d1 | E04 |
| 2-1-m1E04-1-0114 | 815.310 | OH | a1 | B01 | b2 | COS | c1 | D01 | d1 | E04 |
| 2-1-m1E04-1-0115 | 816.305 | OH | a1 | B02 | b2 | C05 | c1 | D01 | d1 | E04 |
| 2-1-m1E04-1-0116 | 817.260 | OH | a2 | B16 | b2 | C06 | c1 | D01 | d1 | E04 |
| 2-1-m1E04-1-0117 | 817.260 | OH | a2 | B11 | b3 | C08 | c1 | D01 | d1 | E04 |
| 2-1-m1E04-1-0118 | 817.260 | OH | a2 | B11 | b1 | C08 | c2 | D02 | d1 | E04 |
| 2-1-m1E04-1-0119 | 817.275 | OH | a2 | B17 | b2 | C02 | c1 | DOI | d1 | E04 |
| 2-1-m1E04-1-0120 | 817.275 | OH | a2 | B14 | b2 | C04 | c1 | D01 | d1 | E04 |
| 2-1-m1E04-1-0121 | 818.256 | OH | a2 | B12 | b3 | C08 | c1 | D01 | d1 | E04 |
| 2-1-m1E04-1-0122 | 818.256 | OH | a2 | B12 | b1 | C08 | c2 | D02 | d1 | E04 |
| 2-1-m1E04-1-0123 | 818.256 | OH | a2 | B11 | b1 | C11 | c2 | D02 | d1 | E04 |
| 2-1-m1E04-1-0124 | 818.270 | OH | a2 | B17 | b2 | C03 | c1 | D01 | d1 | E04 |
| 2-1-m1E04-1-0125 | 818.290 | OH | a1 | B04 | b2 | C02 | c1 | D01 | d1 | E04 |
| 2-1-m1E04-1-0126 | 818.290 | OH | a1 | B01 | b2 | C04 | c1 | D01 | d1 | E04 |
| 2-1-m1E04-1-0127 | 818.310 | OH | a1 | B05 | b2 | C01 | c1 | D01 | d1 | E04 |
| 2-1-m1E04-1-0128 | 819.251 | OH | a2 | B11 | b1 | C10 | c2 | D02 | d1 | E04 |
| 2-1-m1E04-1-0129 | 819.251 | OH | a2 | B12 | b1 | C11 | c2 | D02 | d1 | E04 |
| 2-1-m1E04-1-0130 | 819.285 | OH | a1 | B02 | b2 | C04 | c1 | D01 | d1 | E04 |
| 2-1-m1E04-1-0131 | 819.285 | OH | a1 | B04 | b2 | C03 | c1 | D01 | d1 | E04 |
| 2-1-m1E04-1-0132 | 819.320 | OH | a2 | B13 | b1 | C13 | c1 | D01 | d1 | E04 |
| 2-1-mlE04-1-0133 | 820.246 | OH | a2 | B12 | b1 | C10 | c2 | D02 | d1 | E04 |
| 2-1-m1E04-1-0134 | 820.271 | OH | a1 | B03 | b2 | C06 | c1 | D01 | d1 | E04 |
| 2-1-m1E04-1-0135 | 820.289 | OH | a1 | B06 | b2 | C01 | c1 | D01 | d1 | E04 |
| 2-1-mlE04-1-0136 | 821.255 | OH | a2 | B13 | b3 | C15 | c1 | D01 | d1 | E04 |
| 2-1-m1E04-1-0137 | 821.286 | OH | a2 | B19 | b1 | C08 | c1 | D01 | d1 | E04 |
| 2-1-mlE04-1-0138 | 821.286 | OH | a2 | B11 | b1 | C09 | c1 | D01 | d1 | E04 |
| 2-1-m1E04-1-0139 | 821.288 | OH | a2 | B18 | b2 | C01 | c1 | D01 | d1 | E04 |
| 2-1-m1E04-1-0140 | 822.281 | OH | a2 | B12 | b1 | C09 | c1 | D01 | d1 | E04 |
| 2-1-m1E04-1-0141 | 822.281 | OH | a2 | B19 | b1 | C11 | c1 | D01 | d1 | E04 |
| 2-1-m1E04-1-0142 | 823.217 | OH | a2 | B11 | b3 | C06 | c1 | D01 | d1 | E04 |
| 2-1-m1E04-1-0143 | 823.231 | OH | a2 | B17 | b2 | C06 | c1 | D01 | d1 | E04 |
| 2-1-m1E04-1-0144 | 823.276 | OH | a2 | B19 | b1 | C10 | c1 | D01 | d1 | E04 |
| 2-1-m1E04-1-0145 | 823.286 | OH | a2 | B13 | b1 | C14 | c1 | D01 | d1 | E04 |
| 2-1-m1E04-1-0146 | 823.295 | OH | a2 | B14 | b1 | C13 | c1 | D01 | d1 | E04 |
| 2-1-m1E04-1-0147 | 823.307 | OH | a2 | B13 | b2 | C33 | c3 | D02 | d1 | E04 |
| 2-1-m1E04-1-0148 | 824.212 | OH | a2 | B12 | b3 | C06 | c1 | D01 | d1 | E04 |
| 2-1-m1E04-1-0149 | 824.212 | OH | a2 | B11 | b1 | C12 | c2 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0150 | 824.246 | OH | a1 | B04 | b2 | C06 | c1 | D01 | d1 | E04 |
| 2-1-m1E04-1-0151 | 824.310 | OH | a1 | B01 | b1 | C13 | c1 | D01 | d1 | E04 |
| 2-1-m1E04-1-0152 | 824.336 | OH | a2 | B15 | b2 | C05 | c1 | D01 | d1 | E04 |
| 2-1-m1E04-1-0153 | 825.207 | OH | a2 | B12 | b1 | C12 | c2 | D02 | d1 | E04 |
| 2-1-m1E04-1-0154 | 825.230 | OH | a2 | B14 | b3 | C15 | c1 | D01 | d1 | E04 |
| 2-1-m1E04-1-0155 | 825.306 | OH | a1 | B02 | b1 | C13 | c1 | D01 | d1 | E04 |
| 2-1-m1E04-1-0156 | 826.245 | OH | a1 | B01 | b3 | C15 | c1 | D01 | d1 | E04 |
| 2-1-m1E04-1-0157 | 826.260 | OH | a1 | B07 | b2 | C01 | c1 | D01 | d1 | E04 |
| 2-1-m1E04-1-0158 | 826.315 | OH | a2 | B16 | b2 | COS | c1 | D01 | d1 | E04 |
| 2-1-m1E04-1-0159 | 827.241 | OH | a1 | B02 | b3 | C15 | c1 | D01 | d1 | E04 |
| 2-1-m1E04-1-0160 | 827.261 | OH | a2 | B14 | b1 | C14 | c1 | D01 | d1 | E04 |
| 2-1-m1E04-1-0161 | 827.282 | OH | a2 | B14 | b2 | C33 | c3 | D02 | d1 | E04 |
| 2-1-m1E04-1-0162 | 827.315 | OH | a2 | B15 | b2 | C04 | c1 | D01 | d1 | E04 |
| 2-1-m1E04-1-0163 | 827.372 | OH | a2 | B13 | b2 | C07 | c1 | D01 | d1 | E04 |
| 2-1-m1E04-1-0164 | 828.238 | OH | a2 | B19 | b1 | C12 | c1 | D01 | d1 | E04 |
| 2-1-m1E04-1-0165 | 828.276 | OH | a1 | B01 | b1 | C14 | c1 | D01 | d1 | E04 |
| 2-1-m1E04-1-0166 | 828.298 | OH | a1 | B01 | b2 | C33 | c3 | D02 | d1 | E04 |
| 2-1-m1E04-1-0167 | 828.331 | OH | a1 | B05 | b2 | C02 | c1 | D01 | d1 | E04 |
| 2-1-m1E04-1-0168 | 829.272 | OH | a1 | B08 | b1 | C08 | c1 | D01 | d1 | E04 |
| 2-1-m1E04-1-0169 | 829.272 | OH | a1 | B02 | b1 | C14 | c1 | D01 | d1 | E04 |
| 2-1-m1E04-1-0170 | 829.293 | OH | a1 | B02 | b2 | C33 | c3 | D02 | d1 | E04 |
| 2-1-m1E04-1-0171 | 829.295 | OH | a2 | B16 | b2 | C04 | c1 | D01 | d1 | E04 |
| 2-1-m1E04-1-0172 | 829.297 | OH | a2 | B11 | b1 | C30 | c3 | D02 | d1 | E04 |
| 2-1-m1E04-1-0173 | 829.326 | OH | a1 | B05 | b2 | C03 | c1 | D01 | d1 | E04 |
| 2-1-m1E04-1-0174 | 829.326 | OH | a1 | B03 | b2 | COS | c1 | D01 | d1 | E04 |
| 2-1-m1E04-1-0175 | 830.267 | OH | a1 | B08 | b1 | C11 | c1 | D01 | d1 | E04 |
| 2-1-m1E04-1-0176 | 830.292 | OH | a2 | B12 | b1 | C30 | c3 | D02 | d1 | E04 |
| 2-1-m1E04-1-0177 | 830.310 | OH | a1 | B06 | b2 | C02 | c1 | D01 | d1 | E04 |
| 2-1-m1E04-1-0178 | 831.262 | OH | a1 | B08 | b1 | C10 | c1 | D01 | d1 | E04 |
| 2-1-m1E04-1-0179 | 831.276 | OH | a2 | B13 | b3 | C08 | c1 | D01 | d1 | E04 |
| 2-1-m1E04-1-0180 | 831.276 | OH | a2 | B13 | b1 | C08 | c2 | D02 | d1 | E04 |
| 2-1-m1E04-1-0181 | 831.276 | OH | a2 | B11 | b2 | C20 | c3 | D02 | d1 | E04 |
| 2-1-m1E04-1-0182 | 831.305 | OH | a1 | B06 | b2 | C03 | c1 | D01 | d1 | E04 |
| 2-1-m1E04-1-0183 | 831.309 | OH | a2 | B18 | b2 | C02 | c1 | D01 | d1 | E04 |
| 2-1-m1E04-1-0184 | 831.347 | OH | a2 | B14 | b2 | C07 | c1 | D01 | d1 | E04 |
| 2-1-m1E04-1-0185 | 832.271 | OH | a2 | B13 | b1 | C11 | c2 | D02 | d1 | E04 |
| 2-1-m1E04-1-0186 | 832.271 | OH | a2 | B12 | b2 | C20 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0187 | 832.271 | OH | a2 | B11 | b2 | C28 | c3 | D02 | d1 | E04 |
| 2-1-m1E04-1-0188 | 832.285 | OH | a2 | B17 | b2 | C05 | c1 | D01 | d1 | E04 |
| 2-1-m1E04-1-0189 | 832.304 | OH | a2 | B18 | b2 | C03 | c1 | D01 | d1 | E04 |
| 2-1-m1E04-1-0190 | 832.305 | OH | a1 | B03 | b2 | C04 | c1 | D01 | d1 | E04 |
| 2-1-m1E04-1-0191 | 832.362 | OH | a1 | B01 | b2 | C07 | c1 | D01 | d1 | E04 |
| 2-1-m1E04-1-0192 | 833.267 | OH | a2 | B13 | b1 | C10 | c2 | D02 | d1 | E04 |
| 2-1-m1E04-1-0193 | 833.267 | OH | a2 | B12 | b2 | C28 | c3 | D02 | d1 | E04 |
| 2-1-m1E04-1-0194 | 833.301 | OH | a1 | B04 | b2 | C05 | c1 | D01 | d1 | E04 |
| 2-1-m1E04-1-0195 | 833.336 | OH | a2 | B15 | b1 | C13 | c1 | D01 | d1 | E04 |
| 2-1-m1E04-1-0196 | 833.357 | OH | a1 | B02 | b2 | C07 | c1 | D01 | d1 | E04 |
| 2-1-m1E04-1-0197 | 834.287 | OH | a1 | B05 | b2 | C06 | c1 | D01 | d1 | E04 |
| 2-1-m1E04-1-0198 | 835.235 | OH | a2 | B11 | b2 | C01 | c2 | D02 | d1 | E04 |
| 2-1-m1E04-1-0199 | 835.251 | OH | a2 | B14 | b3 | C08 | c1 | D01 | d1 | E04 |
| 2-1-m1E04-1-0200 | 835.251 | OH | a2 | B14 | b1 | C08 | c2 | D02 | d1 | E04 |
| 2-1-m1E04-1-0201 | 835.265 | OH | a2 | B17 | b2 | C04 | c1 | D01 | d1 | E04 |
| 2-1-m1E04-1-0202 | 835.271 | OH | a2 | B15 | b3 | C15 | c1 | D01 | d1 | E04 |
| 2-1-m1E04-1-0203 | 835.302 | OH | a2 | B13 | b1 | C09 | c1 | D01 | d1 | E04 |
| 2-1-m1E04-1-0204 | 835.315 | OH | a2 | B16 | b1 | C13 | c1 | D01 | d1 | E04 |
| 2-1-m1E04-1-0205 | 836.223 | OH | a1 | B08 | b1 | C12 | c1 | D01 | d1 | E04 |
| 2-1-m1E04-1-0206 | 836.230 | OH | a2 | B12 | b2 | C01 | c2 | D02 | d1 | E04 |
| 2-1-m1E04-1-0207 | 836.246 | OH | a2 | B14 | b1 | C11 | c2 | D02 | d1 | E04 |
| 2-1-m1E04-1-0208 | 836.266 | OH | a1 | B06 | b2 | C06 | c1 | D01 | d1 | E04 |
| 2-1-m1E04-1-0209 | 836.266 | OH | a1 | B01 | b3 | C08 | c1 | D01 | d1 | E04 |
| 2-1-m1E04-1-0210 | 836.266 | OH | a1 | B01 | b1 | C08 | c2 | D02 | d1 | E04 |
| 2-1-m1E04-1-0211 | 836.280 | OH | a1 | B07 | b2 | C02 | c1 | D01 | d1 | E04 |
| 2-1-m1E04-1-0212 | 836.280 | OH | a1 | B04 | b2 | C04 | c1 | D01 | d1 | E04 |
| 2-1-m1E04-1-0213 | 837.232 | OH | a2 | B13 | b3 | C06 | c1 | D01 | d1 | E04 |
| 2-1-m1E04-1-0214 | 837.241 | OH | a2 | B14 | b1 | C10 | c2 | D02 | d1 | E04 |
| 2-1-m1E04-1-0215 | 837.250 | OH | a2 | B16 | b3 | C15 | c1 | D01 | d1 | E04 |
| 2-1-m1E04-1-0216 | 837.261 | OH | a1 | B02 | b3 | C08 | c1 | D01 | d1 | E04 |
| 2-1-m1E04-1-0217 | 837.261 | OH | a1 | B02 | b1 | C08 | c2 | D02 | d1 | E04 |
| 2-1-m1E04-1-0218 | 837.261 | OH | a1 | B01 | b1 | C11 | c2 | D02 | d1 | E04 |
| 2-1-m1E04-1-0219 | 837.265 | OH | a2 | B18 | b2 | C06 | c1 | D01 | d1 | E04 |
| 2-1-m1E04-1-0220 | 837.276 | OH | a1 | B07 | b2 | C03 | c1 | D01 | d1 | E04 |
| 2-1-m1E04-1-0221 | 837.281 | OH | a2 | B20 | b1 | C08 | c1 | D01 | d1 | E04 |
| 2-1-m1E04-1-0222 | 837.302 | OH | a2 | B15 | b1 | C14 | c1 | D01 | d1 | E04 |
| 2-1-m1E04-1-0223 | 837.323 | OH | a2 | B15 | b2 | C33 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0224 | 838.228 | OH | a2 | B13 | 1 b1 | C12 | c2 | D02 | d1 | E04 |
| 2-1-m1E04-1-0225 | 838.257 | OH | a1 | B01 | b1 | C10 | c2 | D02 | d1 | E04 |
| 2-1-m1E04-1-0226 | 838.257 | OH | a1 | B02 | b1 | C11 | c2 | D02 | d1 | E04 |
| 2-1-m1E04-1-0227 | 838.276 | OH | a2 | B20 | b1 | C11 | c1 | D01 | d1 | E04 |
| 2-1-m1E04-1-0228 | 838.326 | OH | a1 | B03 | b1 | C13 | c1 | D01 | d1 | E04 |
| 2-1-m1E04-1-0229 | 839.252 | OH | a1 | B02 | b1 | C10 | c2 | D02 | d1 | E04 |
| 2-1-m1E04-1-0230 | 839.260 | OH | a2 | B19 | b2 | C01 | c1 | D01 | d1 | E04 |
| 2-1-m1E04-1-0231 | 839.271 | OH | a2 | B20 | b1 | C10 | c1 | D01 | d1 | E04 |
| 2-1-m1E04-1-0232 | 839.276 | OH | a2 | B14 | b1 | C09 | c1 | D01 | d1 | E04 |
| 2-1-m1E04-1-0233 | 839.281 | OH | a2 | B16 | b1 | C14 | c1 | D01 | d1 | E04 |
| 2-1-m1E04-1-0234 | 839.302 | OH | a2 | B16 | b2 | C33 | c3 | D02 | d1 | E04 |
| 2-1-m1E04-1-0235 | 840.261 | OH | a1 | B03 | b3 | C15 | c1 | D01 | d1 | E04 |
| 2-1-m1E04-1-0236 | 840.292 | OH | a1 | B09 | b1 | C08 | c1 | D01 | d1 | E04 |
| 2-1-m1E04-1-0237 | 840.292 | OH | a1 | B01 | b1 | C09 | c1 | D01 | d1 | E04 |
| 2-1-m1E04-1-0238 | 841.207 | OH | a2 | B14 | b3 | C06 | c1 | D01 | d1 | E04 |
| 2-1-m1E04-1-0239 | 841.286 | OH | a2 | B17 | b1 | C13 | c1 | D01 | d1 | E04 |
| 2-1-m1E04-1-0240 | 841.287 | OH | a1 | B02 | b1 | C09 | c1 | D01 | d1 | E04 |
| 2-1-m1E04-1-0241 | 841.287 | OH | a1 | B09 | b1 | C11 | c1 | D01 | d1 | E04 |
| 2-1-m1E04-1-0242 | 841.387 | OH | a2 | B15 | b2 | C07 | c1 | D01 | d1 | E04 |
| 2-1-m1E04-1-0243 | 842.203 | OH | a2 | B14 | b1 | C12 | c2 | D02 | d1 | E04 |
| 2-1-m1E04-1-0244 | 842.223 | OH | a1 | B01 | b3 | C06 | c1 | D01 | d1 | E04 |
| 2-1-m1E04-1-0245 | 842.237 | OH | a1 | B07 | b2 | C06 | c1 | D01 | d1 | E04 |
| 2-1-m1E04-1-0246 | 842.282 | OH | a1 | B09 | b1 | C10 | c1 | D01 | d1 | E04 |
| 2-1-m1E04-1-0247 | 842.292 | OH | a1 | B03 | b1 | C14 | c1 | D01 | d1 | E04 |
| 2-1-m1E04-1-0248 | 842.301 | OH | a1 | B04 | b1 | C13 | c1 | D01 | d1 | E04 |
| 2-1-m1E04-1-0249 | 842.313 | OH | a1 | B03 | b2 | C33 | c3 | D02 | d1 | E04 |
| 2-1-m1E04-1-0250 | 843.218 | OH | a1 | B02 | b3 | C06 | c1 | D01 | d1 | E04 |
| 2-1-m1E04-1-0251 | 843.218 | OH | a1 | B01 | b1 | C12 | c2 | D02 | d1 | E04 |
| 2-1-m1E04-1-0252 | 843.221 | OH | a2 | B17 | b3 | C15 | c1 | D01 | d1 | E04 |
| 2-1-m1E04-1-0253 | 843.312 | OH | a2 | B13 | b1 | C30 | c3 | D02 | d1 | E04 |
| 2-1-m1E04-1-0254 | 843.312 | OH | a2 | B11 | b1 | C32 | c3 | D02 | d1 | E04 |
| 2-1-m1E04-1-0255 | 843.341 | OH | a1 | B05 | b2 | C05 | c1 | D01 | d1 | E04 |
| 2-1-m1E04-1-0256 | 843.367 | OH | a2 | B16 | b2 | C07 | c1 | D01 | d1 | E04 |
| 2-1-m1E04-1-0257 | 844.213 | OH | a1 | B02 | b1 | C12 | c2 | D02 | d1 | E04 |
| 2-1-m1E04-1-0258 | 844.232 | OH | a2 | B20 | b1 | C12 | c1 | D01 | d1 | E04 |
| 2-1-m1E04-1-0259 | 844.236 | OH | a1 | B04 | b3 | C15 | c1 | D01 | d1 | E04 |
| 2-1-m1E04-1-0260 | 844.308 | OH | a2 | B12 | b1 | C32 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0261 | 845.252 | OH | a2 | B17 | b1 | C14 | c1 | D01 | d1 | E04 |
| 2-1-m1E04-1-0262 | 845.255 | OH | a2 | B11 | b2 | C02 | c2 | D02 | d1 | E04 |
| 2-1-m1E04-1-0263 | 845.273 | OH | a2 | B17 | b2 | C33 | c3 | D02 | d1 | E04 |
| 2-1-m1E04-1-0264 | 845.292 | OH | a2 | B15 | b3 | C08 | c1 | D01 | d1 | E04 |
| 2-1-m1E04-1-0265 | 845.292 | OH | a2 | B15 | b1 | C08 | c2 | D02 | d1 | E04 |
| 2-1-m1E04-1-0266 | 845.292 | OH | a2 | B13 | b2 | C20 | c3 | D02 | d1 | E04 |
| 2-1-m1E04-1-0267 | 845.292 | OH | a2 | B11 | b2 | C22 | c3 | D02 | d1 | E04 |
| 2-1-m1E04-1-0268 | 845.321 | OH | a1 | B06 | b2 | C05 | c1 | D01 | d1 | E04 |
| 2-1-m1E04-1-0269 | 846.251 | OH | a2 | B12 | b2 | C02 | c2 | D02 | d1 | E04 |
| 2-1-m1E04-1-0270 | 846.251 | OH | a2 | B11 | b2 | C03 | c2 | D02 | d1 | E04 |
| 2-1-m1E04-1-0271 | 846.267 | OH | a1 | B04 | b1 | C14 | c1 | D01 | d1 | E04 |
| 2-1-m1E04-1-0272 | 846.287 | OH | a2 | B15 | b1 | C11 | c2 | D02 | d1 | E04 |
| 2-1-m1E04-1-0273 | 846.287 | OH | a2 | B12 | b2 | C22 | c3 | D02 | d1 | E04 |
| 2-1-m1E04-1-0274 | 846.287 | OH | a2 | B13 | b2 | C28 | c3 | D02 | d1 | E04 |
| 2-1-m1E04-1-0275 | 846.288 | OH | a1 | B04 | b2 | C33 | c3 | D02 | d1 | E04 |
| 2-1-m1E04-1-0276 | 846.320 | OH | a2 | B18 | b2 | C05 | c1 | D01 | d1 | E04 |
| 2-1-m1E04-1-0277 | 846.321 | OH | a1 | B05 | b2 | C04 | c1 | D01 | d1 | E04 |
| 2-1-m1E04-1-0278 | 846.377 | OH | a1 | B03 | b2 | C07 | c1 | D01 | d1 | E04 |
| 2-1-m1E04-1-0279 | 847.243 | OH | a1 | B09 | b1 | C12 | c1 | D01 | d1 | E04 |
| 2-1-m1E04-1-0280 | 847.246 | OH | a1 | B08 | b2 | C01 | c1 | D01 | d1 | E04 |
| 2-1-m1E04-1-0281 | 847.246 | OH | a2 | B12 | b2 | C03 | c2 | D02 | d1 | E04 |
| 2-1-m1E04-1-0282 | 847.271 | OH | a2 | B16 | b3 | C08 | c1 | D01 | d1 | E04 |
| 2-1-m1E04-1-0283 | 847.271 | OH | a2 | B16 | b1 | C08 | c2 | D02 | d1 | E04 |
| 2-1-m1E04-1-0284 | 847.282 | OH | a2 | B15 | b1 | C10 | c2 | D02 | d1 | E04 |
| 2-1-m1E04-1-0285 | 847.287 | OH | a2 | B14 | b1 | C30 | c3 | D02 | d1 | E04 |
| 2-1-m1E04-1-0286 | 848.266 | OH | a2 | B16 | b1 | C11 | c2 | D02 | d1 | E04 |
| 2-1-m1E04-1-0287 | 848.300 | OH | a1 | B06 | b2 | C04 | c1 | D01 | d1 | E04 |
| 2-1-m1E04-1-0288 | 848.303 | OH | a1 | B01 | b1 | C30 | c3 | D02 | d1 | E04 |
| 2-1-m1E04-1-0289 | 849.250 | OH | a2 | B13 | b2 | C01 | c2 | D02 | d1 | E04 |
| 2-1-m1E04-1-0290 | 849.261 | OH | a2 | B16 | b1 | C10 | c2 | D02 | d1 | E04 |
| 2-1-m1E04-1-0291 | 849.267 | OH | a2 | B14 | b2 | C20 | c3 | D02 | d1 | E04 |
| 2-1-m1E04-1-0292 | 849.267 | OH | a2 | B11 | b2 | C24 | c3 | D02 | d1 | E04 |
| 2-1-m1E04-1-0293 | 849.281 | OH | a2 | B19 | b2 | C02 | c1 | D01 | d1 | E04 |
| 2-1-m1E04-1-0294 | 849.298 | OH | a1 | B02 | b1 | C30 | c3 | D02 | d1 | E04 |
| 2-1-m1E04-1-0295 | 849.300 | OH | a2 | B18 | b2 | C04 | c1 | D01 | d1 | E04 |
| 2-1-m1E04-1-0296 | 849.317 | OH | a2 | B15 | b1 | C09 | c1 | D01 | d1 | E04 |
| 2-1-m1E04-1-0297 | 849.337 | OH | a2 | B17 | b2 | C07 | c1 | D01 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0298 | 850.262 | OH | a2 | B12 | b2 | C24 | c3 | D02 | d1 | E04 |
| 2-1-m1E04-1-0299 | 850.262 | OH | a2 | B14 | b2 | C28 | c3 | D02 | d1 | E04 |
| 2-1-m1E04-1-0300 | 850.276 | OH | a2 | B19 | b2 | C03 | c1 | D01 | d1 | E04 |
| 2-1-m1E04-1-0301 | 850.282 | OH | a1 | B03 | b3 | C08 | c1 | D01 | d1 | E04 |
| 2-1-m1E04-1-0302 | 850.282 | OH | a1 | B03 | b1 | C08 | c2 | D02 | d1 | E04 |
| 2-1-m1E04-1-0303 | 850.282 | OH | a1 | B01 | b2 | C20 | c3 | D02 | d1 | E04 |
| 2-1-m1E04-1-0304 | 850.352 | OH | a1 | B04 | b2 | C07 | c1 | D01 | d1 | E04 |
| 2-1-m1E04-1-0305 | 851.212 | OH | a2 | B11 | b2 | C06 | c2 | D02 | d1 | E04 |
| 2-1-m1E04-1-0306 | 851.248 | OH | a2 | B15 | b3 | C06 | c1 | D01 | d1 | E04 |
| 2-1-m1E04-1-0307 | 851.277 | OH | a1 | B03 | b1 | C11 | c2 | D02 | d1 | E04 |
| 2-1-m1E04-1-0308 | 851.277 | OH | a1 | B02 | b2 | C20 | c3 | D02 | d1 | E04 |
| 2-1-m1E04-1-0309 | 851.277 | OH | a1 | B01 | b2 | C28 | c3 | D02 | d1 | E04 |
| 2-1-m1E04-1-0310 | 851.291 | OH | a1 | B07 | b2 | C05 | c1 | D01 | d1 | E04 |
| 2-1-mE041-1-0311 | 851.296 | OH | a2 | B16 | b1 | C09 | c1 | D01 | d1 | E04 |
| 2-1-m1E04-1-0312 | 852.207 | OH | a2 | B12 | b2 | C06 | c2 | D02 | d1 | E04 |
| 2-1-m1E04-1-0313 | 852.243 | OH | a2 | B15 | b1 | C12 | c2 | D02 | d1 | E04 |
| 2-1-m1E04-1-0314 | 852.272 | OH | a1 | B03 | b1 | C10 | c2 | D02 | d1 | E04 |
| 2-1-m1E04-1-0315 | 852.272 | OH | a1 | B02 | b2 | C28 | c3 | D02 | d1 | E04 |
| 2-1-m1E04-1-0316 | 852.342 | OH | a1 | B05 | b1 | C13 | c1 | D01 | d1 | E04 |
| 2-1-m1E04-1-0317 | 853.225 | OH | a2 | B14 | b2 | C01 | c2 | D02 | d1 | E04 |
| 2-1-m1E04-1-0318 | 853.227 | OH | a2 | B16 | b3 | C06 | c1 | D01 | d1 | E04 |
| 2-1-m1E04-1-0319 | 853.242 | OH | a2 | B17 | b3 | C08 | c1 | D01 | d1 | E04 |
| 2-1-m1E04-1-0320 | 853.242 | OH | a2 | B17 | b1 | C08 | c2 | D02 | d1 | E04 |
| 2-1-m1E04-1-0321 | 854.223 | OH | a2 | B16 | b1 | C12 | c2 | D02 | d1 | E04 |
| 2-1-m1E04-1-0322 | 854.237 | OH | a2 | B17 | b1 | C11 | c2 | D02 | d1 | E04 |
| 2-1-m1E04-1-0323 | 854.240 | OH | a1 | B01 | b2 | C01 | c2 | D02 | d1 | E04 |
| 2-1-m1E04-1-0324 | 854.257 | OH | a1 | B04 | b3 | C08 | c1 | D01 | d1 | E04 |
| 2-1-m1E04-1-0325 | 854.257 | OH | a1 | B04 | b1 | C08 | c2 | D02 | d1 | E04 |
| 2-1-m1E04-1-0326 | 854.271 | OH | a1 | B07 | b2 | C04 | c1 | D01 | d1 | E04 |
| 2-1-m1E04-1-0327 | 854.277 | OH | a1 | B05 | b3 | C15 | c1 | D01 | d1 | E04 |
| 2-1-m1E04-1-0328 | 854.307 | OH | a1 | B03 | b1 | C09 | c1 | D01 | d1 | E04 |
| 2-1-m1E04-1-0329 | 854.321 | OH | a1 | B06 | b1 | C13 | c1 | D01 | d1 | E04 |
| 2-1-m1E04-1-0330 | 855.232 | OH | a2 | B17 | b1 | C10 | c2 | D02 | d1 | E04 |
| 2-1-m1E04-1-0331 | 855.236 | OH | a1 | B02 | b2 | C01 | c2 | D02 | d1 | E04 |
| 2-1-m1E04-1-0332 | 855.237 | OH | a2 | B19 | b2 | C06 | c1 | D01 | d1 | E04 |
| 2-1-m1E04-1-0333 | 855.252 | OH | a1 | B04 | b1 | C11 | c2 | D02 | d1 | E04 |
| 2-1-m1E04-1-0334 | 855.255 | OH | a2 | B20 | b2 | C01 | c1 | D01 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0335 | 855.320 | OH | a2 | B18 | b1 | C13 | c1 | D01 | d1 | E04 |
| 2-1-m1E04-1-0336 | 856.238 | OH | a1 | B03 | b3 | C06 | c1 | D01 | d1 | E04 |
| 2-1-m1E04-1-0337 | 856.247 | OH | a1 | B04 | b1 | C10 | c2 | D02 | d1 | E04 |
| 2-1-m1E04-1-0338 | 856.256 | OH | a1 | B06 | b3 | C15 | c1 | D01 | d1 | E04 |
| 2-1-m1E04-1-0339 | 856.287 | OH | a1 | B10 | b1 | C08 | c1 | D01 | d1 | E04 |
| 2-1-m1E04-1-0340 | 856.308 | OH | a1 | B05 | b1 | C14 | c1 | D01 | d1 | E04 |
| 2-1-m1E04-1-0341 | 856.329 | OH | a1 | B05 | b2 | C33 | c3 | D02 | d1 | E04 |
| 2-1-m1E04-1-0342 | 857.234 | OH | a1 | B03 | b1 | C12 | c2 | D02 | d1 | E04 |
| 2-1-m1E04-1-0343 | 857.255 | OH | a2 | B18 | b3 | C15 | c1 | D01 | d1 | E04 |
| 2-1-m1E04-1-0344 | 857.267 | OH | a1 | B08 | b2 | C02 | c1 | D01 | d1 | E04 |
| 2-1-m1E04-1-0345 | 857.267 | OH | a2 | B17 | b1 | C09 | c1 | D01 | d1 | E04 |
| 2-1-m1E04-1-0346 | 857.282 | OH | a1 | B10 | b1 | C11 | c1 | D01 | d1 | E04 |
| 2-1-m1E04-1-0347 | 857.328 | OH | a2 | B15 | b1 | C30 | c3 | D02 | d1 | E04 |
| 2-1-m1E04-1-0348 | 857.328 | OH | a2 | B11 | b1 | C29 | c3 | D02 | d1 | E04 |
| 2-1-m1E04-1-0349 | 857.328 | OH | a2 | B13 | b1 | C32 | c3 | D02 | d1 | E04 |
| 2-1-mlE04-1-0350 | 858.262 | OH | a1 | B08 | b2 | C03 | c1 | D01 | d1 | E04 |
| 2-1-mlE04-1-0351 | 858.266 | OH | a1 | B09 | b2 | C01 | c1 | D01 | d1 | E04 |
| 2-1-m1E04-1-0352 | 858.277 | OH | a1 | B10 | b1 | C10 | c1 | D01 | d1 | E04 |
| 2-1-m1E04-1-0353 | 858.282 | OH | a1 | B04 | b1 | C09 | c1 | D01 | d1 | E04 |
| 2-1-m1E04-1-0354 | 858.287 | OH | a1 | B06 | b1 | C14 | c1 | D01 | d1 | E04 |
| 2-1-m1E04-1-0355 | 858.308 | OH | a1 | B06 | b2 | C33 | c3 | D02 | d1 | E04 |
| 2-1-m1E04-1-0356 | 858.323 | OH | a2 | B12 | b1 | C29 | c3 | D02 | d1 | E04 |
| 2-1-m1E04-1-0357 | 859.198 | OH | a2 | B17 | b3 | C06 | c1 | D01 | d1 | E04 |
| 2-1-m1E04-1-0358 | 859.271 | OH | a2 | B13 | b2 | C02 | c2 | D02 | d1 | E04 |
| 2-1-m1E04-1-0359 | 859.286 | OH | a2 | B18 | b1 | C14 | c1 | D01 | d1 | E04 |
| 2-1-m1E04-1-0360 | 859.307 | OH | a2 | B15 | b2 | C20 | c3 | D02 | d1 | E04 |
| 2-1-m1E04-1-0361 | 859.307 | OH | a2 | B13 | b2 | C22 | c3 | D02 | d1 | E04 |
| 2-1-m 1 E04-1-0362 | 859.307 | OH | a2 | B16 | b1 | C30 | c3 | D02 | d1 | E04 |
| 2-1-m1E04-1-0363 | 859.307 | OH | a2 | B18 | b2 | C33 | c3 | D02 | d1 | E04 |
| 2-1-m1E04-1-0364 | 860.193 | OH | a2 | B17 | b1 | C12 | c2 | D02 | d1 | E04 |
| 2-1-m1E04-1-0365 | 860.213 | OH | a1 | B04 | b3 | C06 | c1 | D01 | d1 | E04 |
| 2-1-m1E04-1-0366 | 860.266 | OH | a2 | B13 | b2 | C03 | c2 | D02 | d1 | E04 |
| 2-1-m1E04-1-0367 | 860.266 | OH | a2 | B11 | b2 | C05 | c2 | D02 | d1 | E04 |
| 2-1-m1E04-1-0368 | 860.292 | OH | a1 | B07 | b1 | C13 | c1 | D01 | d1 | E04 |
| 2-1-m1E04-1-0369 | 860.303 | OH | a2 | B15 | b2 | C28 | c3 | D02 | d1 | E04 |
| 2-1-m1E04-1-0370 | 860.393 | OH | a1 | B05 | b2 | C07 | c1 | D01 | d1 | E04 |
| 2-1-m1E04-1-0371 | 861.208 | OH | a1 | B04 | b1 | C12 | c2 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0372 | 861.261 | OH | a2 | B12 | b2 | C05 | c2 | D02 | d1 | E04 |
| 2-1-m1E04-1-0373 | 861.287 | OH | a2 | B16 | b2 | C20 | c3 | D02 | d1 | E04 |
| 2-1-m1E04-1-0374 | 861.287 | OH | a2 | B11 | b2 | C26 | c3 | D02 | d1 | E04 |
| 2-1-m1E04-1-0375 | 861.303 | OH | a2 | B14 | b1 | C32 | c3 | D02 | d1 | E04 |
| 2-1-m1E04-1-0376 | 862.227 | OH | a1 | B07 | b3 | C15 | c1 | D01 | d1 | E04 |
| 2-1-m1E04-1-0377 | 862.282 | OH | a2 | B12 | b2 | C26 | c3 | D02 | d1 | E04 |
| 2-1-m1E04-1-0378 | 862.282 | OH | a2 | B16 | b2 | C28 | c3 | D02 | d1 | E04 |
| 2-1-m1E04-1-0379 | 862.318 | OH | a1 | B03 | b1 | C30 | c3 | D02 | d1 | E04 |
| 2-1-m1E04-1-0380 | 862.318 | OH | a1 | B01 | b1 | C32 | c3 | D02 | d1 | E04 |
| 2-1-m1E04-1-0381 | 862.372 | OH | a1 | B06 | b2 | C07 | c1 | D01 | d1 | E04 |
| 2-1-m1E04-1-0382 | 863.223 | OH | a1 | B08 | b2 | C06 | c1 | D01 | d1 | E04 |
| 2-1-m1E04-1-0383 | 863.238 | OH | a1 | B10 | b1 | C12 | c1 | D01 | d1 | E04 |
| 2-1-m1E04-1-0384 | 863.246 | OH | a2 | B14 | b2 | C02 | c2 | D02 | d1 | E04 |
| 2-1-m1E04-1-0385 | 863.246 | OH | a2 | B11 | b2 | C04 | c2 | D02 | d1 | E04 |
| 2-1-m1E04-1-0386 | 863.266 | OH | a2 | B15 | b2 | C01 | c2 | D02 | d1 | E04 |
| 2-1-m1E04-1-0387 | 863.282 | OH | a2 | B14 | b2 | C22 | c3 | D02 | d1 | E04 |
| 2-1-m1E04-1-0388 | 863.282 | OH | a2 | B13 | b2 | C24 | c3 | D02 | d1 | E04 |
| 2-1-m1E04-1-0389 | 863.313 | OH | a1 | B02 | b1 | C32 | c3 | D02 | d1 | E04 |
| 2-1-m1E04-1-0390 | 863.372 | OH | a2 | B18 | b2 | C07 | c1 | D01 | d1 | E04 |
| 2-1-m1E04-1-0391 | 864.241 | OH | a2 | B12 | b2 | C04 | c2 | D02 | d1 | E04 |
| 2-1-m1E04-1-0392 | 864.241 | OH | a2 | B14 | b2 | C03 | c2 | D02 | d1 | E04 |
| 2-1-m1E04-1-0393 | 864.258 | OH | a1 | B07 | b1 | C14 | c1 | D01 | d1 | E04 |
| 2-1-m1E04-1-0394 | 864.261 | OH | a1 | B01 | b2 | C02 | c2 | D02 | d1 | E04 |
| 2-1-m1E04-1-0395 | 864.279 | OH | a1 | B07 | b2 | C33 | c3 | D02 | d1 | E04 |
| 2-1-m1E04-1-0396 | 864.292 | OH | a2 | B19 | b2 | C05 | c1 | D01 | d1 | E04 |
| 2-1-m1E04-1-0397 | 864.298 | OH | a1 | B05 | b3 | C08 | c1 | D01 | d1 | E04 |
| 2-1-m1E04-1-0398 | 864.298 | OH | a1 | B05 | b1 | C08 | c2 | D02 | d1 | E04 |
| 2-1-m1E04-1-0399 | 864.298 | OH | a1 | B03 | b2 | C20 | c3 | D02 | d1 | E04 |
| 2-1-m1E04-1-0400 | 864.298 | OH | a1 | B01 | b2 | C22 | c3 | D02 | d1 | E04 |
| 2-1-m1E04-1-0401 | 865.227 | OH | a2 | B13 | b2 | C06 | c2 | D02 | d1 | E04 |
| 2-1-m1E04-1-0402 | 865.245 | OH | a2 | B16 | b2 | C01 | c2 | D02 | d1 | E04 |
| 2-1-m1E04-1-0403 | 865.256 | OH | a1 | B02 | b2 | C02 | c2 | D02 | d1 | E04 |
| 2-1-m1E04-1-0404 | 865.256 | OH | a1 | B01 | b2 | C03 | c2 | D02 | d1 | E04 |
| 2-1-m1E04-1-0405 | 865.276 | OH | a2 | B20 | b2 | C02 | c1 | D01 | d1 | E04 |
| 2-1-m1E04-1-0406 | 865.278 | OH | a2 | B17 | b1 | C30 | c3 | D02 | d1 | E04 |
| 2-1-m1E04-1-0407 | 865.293 | OH | a1 | B05 | b1 | C11 | c2 | D02 | d1 | E04 |
| 2-1-m1E04-1-0408 | 865.293 | OH | a1 | B02 | b2 | C22 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0409 | 865.293 | OH | a1 | B03 | b2 | C28 | c3 | D02 | d1 | E04 |
| 2-1-m1E04-1-0410 | 866.252 | OH | a1 | B02 | b2 | C03 | c2 | D02 | d1 | E04 |
| 2-1-m1E04-1-0411 | 866.271 | OH | a2 | B20 | b2 | C03 | c1 | D01 | d1 | E04 |
| 2-1-m1E04-1-0412 | 866.277 | OH | a1 | B06 | b3 | C08 | c1 | D01 | d1 | E04 |
| 2-1-m1E04-1-0413 | 866.277 | OH | a1 | B06 | b1 | C08 | c2 | D02 | d1 | E04 |
| 2-1-m1E04-1-0414 | 866.288 | OH | a1 | B05 | b1 | C10 | c2 | D02 | d1 | E04 |
| 2-1-m1E04-1-0415 | 866.293 | OH | a1 | B04 | b1 | C30 | c3 | D02 | d1 | E04 |
| 2-1-m1E04-1-0416 | 867.257 | OH | a2 | B17 | b2 | C20 | c3 | D02 | d1 | E04 |
| 2-1-m1E04-1-0417 | 867.257 | OH | a2 | B14 | b2 | C24 | c3 | D02 | d1 | E04 |
| 2-1-m1E04-1-0418 | 867.271 | OH | a2 | B19 | b2 | C04 | c1 | D01 | d1 | E04 |
| 2-1-m1E04-1-0419 | 867.272 | OH | a1 | B06 | b1 | C11 | c2 | D02 | d1 | E04 |
| 2-1-m1E04-1-0420 | 867.276 | OH | a2 | B18 | b3 | C08 | c1 | D01 | d1 | E04 |
| 2-1-m1E04-1-0421 | 867.276 | OH | a2 | B18 | b1 | C08 | c2 | D02 | d1 | E04 |
| 2-1-m1E04-1-0422 | 868.252 | OH | a2 | B17 | b2 | C28 | c3 | D02 | d1 | E04 |
| 2-1-m1E04-1-0423 | 868.256 | OH | a1 | B03 | b2 | C01 | c2 | D02 | d1 | E04 |
| 2-1-m1E04-1-0424 | 868.267 | OH | a1 | B06 | b1 | C10 | c2 | D02 | d1 | E04 |
| 2-1-m1E04-1-0425 | 868.271 | OH | a2 | B18 | b1 | C11 | c2 | D02 | d1 | E04 |
| 2-1-m1E04-1-0426 | 868.272 | OH | a1 | B04 | b2 | C20 | c3 | D02 | d1 | E04 |
| 2-1-m1E04-1-0427 | 868.272 | OH | a1 | B01 | b2 | C24 | c3 | D02 | d1 | E04 |
| 2-1-m1E04-1-0428 | 868.287 | OH | a1 | B09 | b2 | C02 | c1 | D01 | d1 | E04 |
| 2-1-m1E04-1-0429 | 868.323 | OH | a1 | B05 | b1 | C09 | c1 | D01 | d1 | E04 |
| 2-1-m1E04-1-0430 | 868.343 | OH | a1 | B07 | b2 | C07 | c1 | D01 | d1 | E04 |
| 2-1-m1E04-1-0431 | 869.202 | OH | a2 | B14 | b2 | C06 | c2 | D02 | d1 | E04 |
| 2-1-m1E04-1-0432 | 869.267 | OH | a2 | B18 | b1 | C10 | c2 | D02 | d1 | E04 |
| 2-1-m1E04-1-0433 | 869.267 | OH | a2 | B11 | b1 | C13 | c2 | D02 | d1 | E04 |
| 2-1-m1E04-1-0434 | 869.268 | OH | a1 | B02 | b2 | C24 | c3 | D02 | d1 | E04 |
| 2-1-m1E04-1-0435 | 869.268 | OH | a1 | B04 | b2 | C28 | c3 | D02 | d1 | E04 |
| 2-1-m1E04-1-0436 | 869.282 | OH | a1 | B09 | b2 | C03 | c1 | D01 | d1 | E04 |
| 2-1-m1E04-1-0437 | 870.218 | OH | a1 | B01 | b2 | C06 | c2 | D02 | d1 | E04 |
| 2-1-m1E04-1-0438 | 870.254 | OH | a1 | B05 | b3 | C06 | c1 | D01 | d1 | E04 |
| 2-1-m1E04-1-0439 | 870.262 | OH | a2 | B12 | b1 | C13 | c2 | D02 | d1 | E04 |
| 2-1-m1E04-1-0440 | 870.302 | OH | a1 | B06 | b1 | C09 | c1 | D01 | d1 | E04 |
| 2-1-m1E04-1-0441 | 871.202 | OH | a2 | B11 | b3 | C15 | c2 | D02 | d1 | E04 |
| 2-1-m1E04-1-0442 | 871.213 | OH | a1 | B02 | b2 | C06 | c2 | D02 | d1 | E04 |
| 2-1-m1E04-1-0443 | 871.216 | OH | a2 | B17 | b2 | C01 | c2 | D02 | d1 | E04 |
| 2-1-m1E04-1-0444 | 871.232 | OH | a2 | B20 | b2 | C06 | c1 | D01 | d1 | E04 |
| 2-1-m1E04-1-0445 | 871.249 | OH | a1 | B05 | b1 | C12 | c2 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0446 | 871.271 | OH | a2 | B11 | b3 | C16 | c1 | D01 | d1 | E04 |
| 2-1-m1E04-1-0447 | 871.302 | OH | a2 | B18 | b1 | C09 | c1 | D01 | d1 | E04 |
| 2-1-m1E04-1-0448 | 871.344 | OH | a2 | B13 | b1 | C29 | c3 | D02 | d1 | E04 |
| 2-1-m1E04-1-0449 | 871.344 | OH | a2 | B15 | b1 | C32 | c3 | D02 | d1 | E04 |
| 2-1-m1E04-1-0450 | 872.197 | OH | a2 | B12 | b3 | C15 | c2 | D02 | d1 | E04 |
| 2-1-m1E04-1-0451 | 872.231 | OH | a1 | B04 | b2 | C01 | c2 | D02 | d1 | E04 |
| 2-1-m1E04-1-0452 | 872.233 | OH | a1 | B06 | b3 | C06 | c1 | D01 | d1 | E04 |
| 2-1-m1E04-1-0453 | 872.247 | OH | a1 | B07 | b3 | C08 | c1 | D01 | d1 | E04 |
| 2-1-m1E04-1-0454 | 872.247 | OH | a1 | B07 | b1 | C08 | c2 | D02 | d1 | E04 |
| 2-1-m1E04-1-0455 | 872.266 | OH | a2 | B12 | b3 | C16 | c1 | D01 | d1 | E04 |
| 2-1-m1E04-1-0456 | 872.277 | OH | a1 | B08 | b2 | C05 | c1 | D01 | d1 | E04 |
| 2-1-m1E04-1-0457 | 873.228 | OH | a1 | B06 | b1 | C12 | c2 | D02 | d1 | E04 |
| 2-1-m1E04-1-0458 | 873.232 | OH | a2 | B18 | b3 | C06 | c1 | D01 | d1 | E04 |
| 2-1-m1E04-1-0459 | 873.232 | OH | a2 | B11 | b3 | C17 | c1 | D01 | d1 | E04 |
| 2-1-m1E04-1-0460 | 873.232 | OH | a2 | B11 | b1 | C14 | c2 | D02 | d1 | E04 |
| 2-1-m1E04-1-0461 | 873.243 | OH | a1 | B07 | b1 | C11 | c2 | D02 | d1 | E04 |
| 2-1-m1E04-1-0462 | 873.287 | OH | a2 | B15 | b2 | C02 | c2 | D02 | d1 | E04 |
| 2-1-m1E04-1-0463 | 873.292 | OH | a2 | B19 | b1 | C13 | c1 | D01 | d1 | E04 |
| 2-1-m1E04-1-0464 | 873.323 | OH | a2 | B15 | b2 | C22 | c3 | D02 | d1 | E04 |
| 2-1-m1E04-1-0465 | 873.323 | OH | a2 | B16 | b1 | C32 | c3 | D02 | d1 | E04 |
| 2-1-m1E04-1-0466 | 874.228 | OH | a2 | B12 | b3 | C17 | c1 | D01 | d1 | E04 |
| 2-1-m1E04-1-0467 | 874.228 | OH | a2 | B18 | b1 | C12 | c2 | D02 | d1 | E04 |
| 2-1-m1E04-1-0468 | 874.228 | OH | a2 | B12 | b1 | C14 | c2 | D02 | d1 | E04 |
| 2-1-m1E04-1-0469 | 874.238 | OH | a1 | B07 | b1 | C10 | c2 | D02 | d1 | E04 |
| 2-1-m1E04-1-0470 | 874.243 | OH | a1 | B09 | b2 | C06 | c1 | D01 | d1 | E04 |
| 2-1-m1E04-1-0471 | 874.261 | OH | a1 | B10 | b2 | C01 | c1 | D01 | d1 | E04 |
| 2-1-m1E04-1-0472 | 874.282 | OH | a2 | B15 | b2 | C03 | c2 | D02 | d1 | E04 |
| 2-1-m1E04-1-0473 | 874.282 | OH | a2 | B13 | b2 | C05 | c2 | D02 | d1 | E04 |
| 2-1-m1E04-1-0474 | 875.227 | OH | a2 | B19 | b3 | C15 | c1 | D01 | d1 | E04 |
| 2-1-m1E04-1-0475 | 875.257 | OH | a1 | B08 | b2 | C04 | c1 | D01 | d1 | E04 |
| 2-1-m1E04-1-0476 | 875.266 | OH | a2 | B16 | b2 | C02 | c2 | D02 | d1 | E04 |
| 2-1-m1E04-1-0477 | 875.302 | OH | a2 | B16 | b2 | C22 | c3 | D02 | d1 | E04 |
| 2-1-m1E04-1-0478 | 875.302 | OH | a2 | B13 | b2 | C26 | c3 | D02 | d1 | E04 |
| 2-1-m1E04-1-0479 | 875.319 | OH | a2 | B14 | b1 | C29 | c3 | D02 | d1 | E04 |
| 2-1-m1E04-1-0480 | 876.261 | OH | a2 | B16 | b2 | C03 | c2 | D02 | d1 | E04 |
| 2-1-m1E04-1-0481 | 876.273 | OH | a1 | B07 | b1 | C09 | c1 | D01 | d1 | E04 |
| 2-1-m1E04-1-0482 | 876.334 | OH | a1 | B05 | b1 | C30 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0483 | 876.334 | OH | a1 | B01 | b1 | C29 | c3 | D02 | d1 | E04 |
| 2-1-m1E04-1-0484 | 876.334 | OH | a1 | B03 | b1 | C32 | c3 | D02 | d1 | E04 |
| 2-1-m1E04-1-0485 | 877.258 | OH | a2 | B19 | b1 | C14 | c1 | D01 | d1 | E04 |
| 2-1-m1E04-1-0486 | 877.262 | OH | a2 | B13 | b2 | C04 | c2 | D02 | d1 | E04 |
| 2-1-m1E04-1-0487 | 877.279 | OH | a2 | B19 | b2 | C33 | c3 | D02 | d1 | E04 |
| 2-1-m1E04-1-0488 | 877.298 | OH | a2 | B15 | b2 | C24 | c3 | D02 | d1 | E04 |
| 2-1-m1E04-1-0489 | 877.318 | OH | a2 | B11 | b2 | C07 | c2 | D02 | d1 | E04 |
| 2-1-m1E04-1-0490 | 877.329 | OH | a1 | B02 | b1 | C29 | c3 | D02 | d1 | E04 |
| 2-1-m1E04-1-0491 | 878.204 | OH | a1 | B07 | b3 | C06 | c1 | D01 | d1 | E04 |
| 2-1-m1E04-1-0492 | 878.257 | OH | a2 | B14 | b2 | C05 | c2 | D02 | d1 | E04 |
| 2-1-m1E04-1-0493 | 878.277 | OH | a1 | B03 | b2 | C02 | c2 | D02 | d1 | E04 |
| 2-1-m1E04-1-0494 | 878.313 | OH | a2 | B12 | b2 | C07 | c2 | D02 | d1 | E04 |
| 2-1-m1E04-1-0495 | 878.313 | OH | a1 | B05 | b2 | C20 | c3 | D02 | d1 | E04 |
| 2-1-m1E04-1-0496 | 878.313 | OH | a1 | B03 | b2 | C22 | c3 | D02 | d1 | E04 |
| 2-1-m1E04-1-0497 | 878.313 | OH | a1 | B06 | b1 | C30 | c3 | D02 | d1 | E04 |
| 2-1-m1E04-1-0498 | 879.199 | OH | a1 | B07 | b1 | C12 | c2 | D02 | d1 | E04 |
| 2-1-m1E04-1-0499 | 879.243 | OH | a2 | B15 | b2 | C06 | c2 | D02 | d1 | E04 |
| 2-1-m1E04-1-0500 | 879.272 | OH | a1 | B03 | b2 | C03 | c2 | D02 | d1 | E04 |
| 2-1-m1E04-1-0501 | 879.272 | OH | a1 | B01 | b2 | C05 | c2 | D02 | d1 | E04 |
| 2-1-m1E04-1-0502 | 879.277 | OH | a2 | B16 | b2 | C24 | c3 | D02 | d1 | E04 |
| 2-1-m1E04-1-0503 | 879.277 | OH | a2 | B14 | b2 | C26 | c3 | D02 | d1 | E04 |
| 2-1-m1E04-1-0504 | 879.294 | OH | a2 | B17 | b1 | C32 | c3 | D02 | d1 | E04 |
| 2-1-m1E04-1-0505 | 879.308 | OH | a1 | B05 | b2 | C28 | c3 | D02 | d1 | E04 |
| 2-1-m1E04-1-0506 | 879.312 | OH | a2 | B18 | b1 | C30 | c3 | D02 | d1 | E04 |
| 2-1-m1E04-1-0507 | 880.267 | OH | a1 | B02 | b2 | C05 | c2 | D02 | d1 | E04 |
| 2-1-m1E04-1-0508 | 880.287 | OH | a2 | B20 | b2 | C05 | c1 | D01 | d1 | E04 |
| 2-1-m1E04-1-0509 | 880.292 | OH | a1 | B06 | b2 | C20 | c3 | D02 | d1 | E04 |
| 2-1-m1E04-1-0510 | 880.292 | OH | a1 | B01 | b2 | C26 | c3 | D02 | d1 | E04 |
| 2-1-m1E04-1-0511 | 880.309 | OH | a1 | B04 | b1 | C32 | c3 | D02 | d1 | E04 |
| 2-1-m1E04-1-0512 | 881.222 | OH | a2 | B16 | b2 | C06 | c2 | D02 | d1 | E04 |
| 2-1-m1E04-1-0513 | 881.222 | OH | a2 | B11 | b3 | C08 | c2 | D02 | d1 | E04 |
| 2-1-m1E04-1-0514 | 881.236 | OH | a2 | B17 | b2 | C02 | c2 | D02 | d1 | E04 |
| 2-1-m1E04-1-0515 | 881.236 | OH | a2 | B14 | b2 | C04 | c2 | D02 | d1 | E04 |
| 2-1-m1E04-1-0516 | 881.273 | OH | a2 | B17 | b2 | C22 | c3 | D02 | d1 | E04 |
| 2-1-m1E04-1-0517 | 881.278 | OH | a1 | B08 | b1 | C13 | c1 | D01 | d1 | E04 |
| 2-1-m1E04-1-0518 | 881.288 | OH | a1 | B02 | b2 | C26 | c3 | D02 | d1 | E04 |
| 2-1-m1E04-1-0519 | 881.288 | OH | a1 | B06 | b2 | C28 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0520 | 881.292 | OH | a2 | B18 | b2 | C20 | c3 | D02 | d1 | E04 |
| 2-1-m1E04-1-0521 | 881.343 | OH | a2 | B19 | b2 | C07 | c1 | D01 | d1 | E04 |
| 2-1-m1E04-1-0522 | 882.218 | OH | a2 | B12 | b3 | C08 | c2 | D02 | d1 | E04 |
| 2-1-m1E04-1-0523 | 882.232 | OH | a2 | B17 | b2 | C03 | c2 | D02 | d1 | E04 |
| 2-1-m1E04-1-0524 | 882.252 | OH | a1 | B04 | b2 | C02 | c2 | D02 | d1 | E04 |
| 2-1-m 1 E04-1-0525 | 882.252 | OH | a1 | B01 | b2 | C04 | c2 | D02 | d1 | E04 |
| 2-1-m1E04-1-0526 | 882.272 | OH | a1 | B05 | b2 | C01 | c2 | D02 | d1 | E04 |
| 2-1-m 1 E04-1-0527 | 882.287 | OH | a2 | B18 | b2 | C28 | c3 | D02 | d1 | E04 |
| 2-1-m1E04-1-0528 | 882.288 | OH | a1 | B04 | b2 | C22 | c3 | D02 | d1 | E04 |
| 2-1-m 1 E04-1-0529 | 882.288 | OH | a1 | B03 | b2 | C24 | c3 | D02 | d1 | E04 |
| 2-1-m1E04-1-0530 | 883.213 | OH | a1 | B08 | b3 | C15 | c1 | D01 | d1 | E04 |
| 2-1-m1E04-1-0531 | 883.247 | OH | a1 | B02 | b2 | C04 | c2 | D02 | d1 | E04 |
| 2-1-m1E04-1-0532 | 883.247 | OH | a1 | B04 | b2 | C03 | c2 | D02 | d1 | E04 |
| 2-1-m1E04-1-0533 | 883.266 | OH | a2 | B20 | b2 | C04 | c1 | D01 | d1 | E04 |
| 2-1-m1E04-1-0534 | 883.282 | OH | a2 | B13 | b1 | C13 | c2 | D02 | d1 | E04 |
| 2-1-m1E04-1-0535 | 883.298 | OH | a1 | B09 | b2 | C05 | c1 | D01 | d1 | E04 |
| 2-1-m1E04-1-0536 | 884.233 | OH | a1 | B03 | b2 | C06 | c2 | D02 | d1 | E04 |
| 2-1-m1E04-1-0537 | 884.251 | OH | a1 | B06 | b2 | C01 | c2 | D02 | d1 | E04 |
| 2-1-m1E04-1-0538 | 884.282 | OH | a1 | B10 | b2 | C02 | c1 | D01 | d1 | E04 |
| 2-1-m1E04-1-0539 | 884.284 | OH | a1 | B07 | b1 | C30 | c3 | D02 | d1 | E04 |
| 2-1-m1E04-1-0540 | 885.217 | OH | a2 | B13 | b3 | C15 | c2 | D02 | d1 | E04 |
| 2-1-m1E04-1-0541 | 885.244 | OH | a1 | B08 | b1 | C14 | c1 | D01 | d1 | E04 |
| 2-1-m1E04-1-0542 | 885.248 | OH | a2 | B19 | b3 | C08 | c1 | D01 | d1 | E04 |
| 2-1-m1E04-1-0543 | 885.248 | OH | a2 | B19 | b1 | C08 | c2 | D02 | d1 | E04 |
| 2-1-m1E04-1-0544 | 885.248 | OH | a2 | B11 | b1 | C09 | c2 | D02 | d1 | E04 |
| 2-1-m1E04-1-0545 | 885.248 | OH | a2 | B17 | b2 | C24 | c3 | D02 | d1 | E04 |
| 2-1-m1E04-1-0546 | 885.250 | OH | a2 | B18 | b2 | C01 | c2 | D02 | d1 | E04 |
| 2-1-m1E04-1-0547 | 885.265 | OH | a1 | B08 | b2 | C33 | c3 | D02 | d1 | E04 |
| 2-1-m1E04-1-0548 | 885.277 | OH | a1 | B10 | b2 | C03 | c1 | D01 | d1 | E04 |
| 2-1-m1E04-1-0549 | 885.287 | OH | a2 | B13 | b3 | C16 | c1 | D01 | d1 | E04 |
| 2-1-m1E04-1-0550 | 885.323 | OH | a2 | B11 | b1 | C31 | c3 | D02 | d1 | E04 |
| 2-1-m1E04-1-0551 | 885.359 | OH | a2 | B15 | b1 | C29 | c3 | D02 | d1 | E04 |
| 2-1-m1E04-1-0552 | 886.243 | OH | a2 | B12 | b1 | C09 | c2 | D02 | d1 | E04 |
| 2-1-m1E04-1-0553 | 886.243 | OH | a2 | B19 | b1 | C11 | c2 | D02 | d1 | E04 |
| 2-1-m1E04-1-0554 | 886.263 | OH | a1 | B07 | b2 | C20 | c3 | D02 | d1 | E04 |
| 2-1-m1E04-1-0555 | 886.263 | OH | a1 | B04 | b2 | C24 | c3 | D02 | d1 | E04 |
| 2-1-m1E04-1-0556 | 886.277 | OH | a1 | B09 | b2 | C04 | c1 | D01 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0557 | 886.318 | OH | a2 | B12 | b1 | C31 | c3 | D02 | d1 | E04 |
| 2-1-m1E04-1-0558 | 887.179 | OH | a2 | B11 | b3 | C06 | c2 | D02 | d1 | E04 |
| 2-1-m1E04-1-0559 | 887.193 | OH | a2 | B17 | b2 | C06 | c2 | D02 | d1 | E04 |
| 2-1-m1E04-1-0560 | 887.238 | OH | a2 | B19 | b1 | C10 | c2 | D02 | d1 | E04 |
| 2-1-m1E04-1-0561 | 887.248 | OH | a2 | B13 | b3 | C17 | c1 | D01 | d1 | E04 |
| 2-1-m1E04-1-0562 | 887.248 | OH | a2 | B13 | b1 | C14 | c2 | D02 | d1 | E04 |
| 2-1-m1E04-1-0563 | 887.257 | OH | a2 | B14 | b1 | C13 | c2 | D02 | d1 | E04 |
| 2-1-m1E04-1-0564 | 887.258 | OH | a1 | B07 | b2 | C28 | c3 | D02 | d1 | E04 |
| 2-1-m1E04-1-0565 | 887.339 | OH | a2 | B16 | b1 | C29 | c3 | D02 | d1 | E04 |
| 2-1-m1E04-1-0566 | 888.174 | OH | a2 | B12 | b3 | C06 | c2 | D02 | d1 | E04 |
| 2-1-m1E04-1-0567 | 888.208 | OH | a1 | B04 | b2 | C06 | c2 | D02 | d1 | E04 |
| 2-1-m1E04-1-0568 | 888.243 | OH | a2 | B11 | b3 | C18 | c1 | D01 | d1 | E04 |
| 2-1-m1E04-1-0569 | 888.272 | OH | a1 | B01 | b1 | C13 | c2 | D02 | d1 | E04 |
| 2-1-m1E04-1-0570 | 888.298 | OH | a2 | B15 | b2 | C05 | c2 | D02 | d1 | E04 |
| 2-1-m1E04-1-0571 | 889.192 | OH | a2 | B14 | b3 | C15 | c2 | D02 | d1 | E04 |
| 2-1-m1E04-1-0572 | 889.239 | OH | a2 | B12 | b3 | C18 | c1 | D01 | d1 | E04 |
| 2-1-m1E04-1-0573 | 889.262 | OH | a2 | B14 | b3 | C16 | c1 | D01 | d1 | E04 |
| 2-1-m1E04-1-0574 | 889.268 | OH | a1 | B02 | b1 | C13 | c2 | D02 | d1 | E04 |
| 2-1-m1E04-1-0575 | 889.273 | OH | a2 | B19 | b1 | C09 | c1 | D01 | d1 | E04 |
| 2-1-m1E04-1-0576 | 889.287 | OH | a2 | B20 | b1 | C13 | c1 | D01 | d1 | E04 |
| 2-1-m1E04-1-0577 | 889.318 | OH | a2 | B15 | b2 | C26 | c3 | D02 | d1 | E04 |
| 2-1-m1E04-1-0578 | 889.329 | OH | a1 | B08 | b2 | C07 | c1 | D01 | d1 | E04 |
| 2-1-m1E04-1-0579 | 890.207 | OH | a1 | B01 | b3 | C15 | c2 | D02 | d1 | E04 |
| 2-1-m1E04-1-0580 | 890.222 | OH | a1 | B07 | b2 | C01 | c2 | D02 | d1 | E04 |
| 2-1-m1E04-1-0581 | 890.238 | OH | a1 | B10 | b2 | C06 | c1 | D01 | d1 | E04 |
| 2-1-m1E04-1-0582 | 890.277 | OH | a1 | B01 | b3 | C16 | c1 | D01 | d1 | E04 |
| 2-1-m1E04-1-0583 | 890.277 | OH | a2 | B16 | b2 | C05 | c2 | D02 | d1 | E04 |
| 2-1-m1E04-1-0584 | 890.350 | OH | a1 | B03 | b1 | C29 | c3 | D02 | d1 | E04 |
| 2-1-m1E04-1-0585 | 890.350 | OH | a1 | B05 | b1 | C32 | c3 | D02 | d1 | E04 |
| 2-1-m1E04-1-0586 | 891.203 | OH | a1 | B02 | b3 | C15 | c2 | D02 | d1 | E04 |
| 2-1-m1E04-1-0587 | 891.204 | OH | a2 | B19 | b3 | C06 | c1 | D01 | d1 | E04 |
| 2-1-m1E04-1-0588 | 891.222 | OH | a2 | B20 | b3 | C15 | c1 | D01 | d1 | E04 |
| 2-1-m1E04-1-0589 | 891.223 | OH | a2 | B14 | b3 | C17 | c1 | D01 | d1 | E04 |
| 2-1-m1E04-1-0590 | 891.223 | OH | a2 | B14 | b1 | C14 | c2 | D02 | d1 | E04 |
| 2-1-m1E04-1-0591 | 891.272 | OH | a1 | B02 | b3 | C16 | c1 | D01 | d1 | E04 |
| 2-1-m1E04-1-0592 | 891.277 | OH | a2 | B15 | b2 | C04 | c2 | D02 | d1 | E04 |
| 2-1-m1E04-1-0593 | 891.297 | OH | a2 | B16 | b2 | C26 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0594 | 891.334 | OH | a2 | B13 | b2 | C07 | c2 | D02 | d1 | E04 |
| 2-1-m1E04-1-0595 | 892.199 | OH | a2 | B19 | b1 | C12 | c2 | D02 | d1 | E04 |
| 2-1-m1E04-1-0596 | 892.238 | OH | a1 | B01 | b3 | C17 | c1 | D01 | d1 | E04 |
| 2-1-m1E04-1-0597 | 892.238 | OH | a1 | B01 | b1 | C14 | c2 | D02 | d1 | E04 |
| 2-1-m1E04-1-0598 | 892.292 | OH | a1 | B05 | b2 | C02 | c2 | D02 | d1 | E04 |
| 2-1-m1E04-1-0599 | 892.298 | OH | a1 | B09 | b1 | C13 | c1 | D01 | d1 | E04 |
| 2-1-m1E04-1-0600 | 892.329 | OH | a1 | B05 | b2 | C22 | c3 | D02 | d1 | E04 |
| 2-1-m1E04-1-0601 | 892.329 | OH | a1 | B06 | b1 | C32 | c3 | D02 | d1 | E04 |
| 2-1-m1E04-1-0602 | 893.234 | OH | a1 | B02 | b3 | C17 | c1 | D01 | d1 | E04 |
| 2-1-m1E04-1-0603 | 893.234 | OH | a1 | B08 | b3 | C08 | c1 | D01 | d1 | E04 |
| 2-1-m1E04-1-0604 | 893.234 | OH | a1 | B08 | b1 | C08 | c2 | D02 | d1 | E04 |
| 2-1-m1E04-1-0605 | 893.234 | OH | a1 | B02 | b1 | C14 | c2 | D02 | d1 | E04 |
| 2-1-m1E04-1-0606 | 893.253 | OH | a2 | B20 | b1 | C14 | c1 | D01 | d1 | E04 |
| 2-1-m1E04-1-0607 | 893.256 | OH | a2 | B16 | b2 | C04 | c2 | D02 | d1 | E04 |
| 2-1-m1E04-1-0608 | 893.274 | OH | a2 | B20 | b2 | C33 | c3 | D02 | d1 | E04 |
| 2-1-m1E04-1-0609 | 893.288 | OH | a1 | B05 | b2 | C03 | c2 | D02 | d1 | E04 |
| 2-1-m1E04-1-0610 | 893.288 | OH | a1 | B03 | b2 | C05 | c2 | D02 | d1 | E04 |
| 2-1-m1E04-1-0611 | 893.309 | OH | a2 | B17 | b1 | C29 | c3 | D02 | d1 | E04 |
| 2-1-m1E04-1-0612 | 893.328 | OH | a2 | B18 | b1 | C32 | c3 | D02 | d1 | E04 |
| 2-1-m1E04-1-0613 | 894.229 | OH | a1 | B08 | b1 | C11 | c2 | D02 | d1 | E04 |
| 2-1-m1E04-1-0614 | 894.233 | OH | a1 | B09 | b3 | C15 | c1 | D01 | d1 | E04 |
| 2-1-m1E04-1-0615 | 894.272 | OH | a1 | B06 | b2 | C02 | c2 | D02 | d1 | E04 |
| 2-1-m1E04-1-0616 | 894.308 | OH | a1 | B06 | b2 | C22 | c3 | D02 | d1 | E04 |
| 2-1-m1E04-1-0617 | 894.308 | OH | a1 | B03 | b2 | C26 | c3 | D02 | d1 | E04 |
| 2-1-m1E04-1-0618 | 894.324 | OH | a1 | B04 | b1 | C29 | c3 | D02 | d1 | E04 |
| 2-1-m1E04-1-0619 | 895.224 | OH | a1 | B08 | b1 | C10 | c2 | D02 | d1 | E04 |
| 2-1-m1E04-1-0620 | 895.238 | OH | a2 | B13 | b3 | C08 | c2 | D02 | d1 | E04 |
| 2-1-m1E04-1-0621 | 895.267 | OH | a1 | B06 | b2 | C03 | c2 | D02 | d1 | E04 |
| 2-1-m1E04-1-0622 | 895.271 | OH | a2 | B18 | b2 | C02 | c2 | D02 | d1 | E04 |
| 2-1-m1E04-1-0623 | 895.307 | OH | a2 | B18 | b2 | C22 | c3 | D02 | d1 | E04 |
| 2-1-m1E04-1-0624 | 895.308 | OH | a2 | B14 | b2 | C07 | c2 | D02 | d1 | E04 |
| 2-1-m1E04-1-0625 | 896.247 | OH | a2 | B17 | b2 | C05 | c2 | D02 | d1 | E04 |
| 2-1-m1E04-1-0626 | 896.264 | OH | a1 | B09 | b1 | C14 | c1 | D01 | d1 | E04 |
| 2-1-m1E04-1-0627 | 896.266 | OH | a2 | B18 | b2 | C03 | c2 | D02 | d1 | E04 |
| 2-1-m1E04-1-0628 | 896.267 | OH | a1 | B03 | b2 | C04 | c2 | D02 | d1 | E04 |
| 2-1-m1E04-1-0629 | 896.285 | OH | a1 | B09 | b2 | C33 | c3 | D02 | d1 | E04 |
| 2-1-m1E04-1-0630 | 896.304 | OH | a1 | B05 | b2 | C24 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0631 | 896.324 | OH | a1 | B01 | b2 | C07 | c2 | D02 | d1 | E04 |
| 2-1-m1E04-1-0632 | 897.259 | OH | a1 | B08 | b1 | C09 | c1 | D01 | d1 | E04 |
| 2-1-m1E04-1-0633 | 897.263 | OH | a1 | B04 | b2 | C05 | c2 | D02 | d1 | E04 |
| 2-1-m1E04-1-0634 | 897.268 | OH | a2 | B17 | b2 | C26 | c3 | D02 | d1 | E04 |
| 2-1-m1E04-1-0635 | 897.284 | OH | a2 | B19 | b1 | C30 | c3 | D02 | d1 | E04 |
| 2-1-m1E04-1-0636 | 897.298 | OH | a2 | B15 | b1 | C13 | c2 | D02 | d1 | E04 |
| 2-1-m1E04-1-0637 | 897.319 | OH | a1 | B02 | b2 | C07 | c2 | D02 | d1 | E04 |
| 2-1-m1E04-1-0638 | 897.338 | OH | a2 | B20 | b2 | C07 | c1 | D01 | d1 | E04 |
| 2-1-m1E04-1-0639 | 898.249 | OH | a1 | B05 | b2 | C06 | c2 | D02 | d1 | E04 |
| 2-1-m1E04-1-0640 | 898.283 | OH | a1 | B06 | b2 | C24 | c3 | D02 | d1 | E04 |
| 2-1-m1E04-1-0641 | 898.283 | OH | a1 | B04 | b2 | C26 | c3 | D02 | d1 | E04 |
| 2-1-m1E04-1-0642 | 898.299 | OH | a1 | B07 | b1 | C32 | c3 | D02 | d1 | E04 |
| 2-1-m1E04-1-0643 | 899.190 | OH | a1 | B08 | b3 | C06 | c1 | D01 | d1 | E04 |
| 2-1-m1E04-1-0644 | 899.213 | OH | a2 | B14 | b3 | C08 | c2 | D02 | d1 | E04 |
| 2-1-m1E04-1-0645 | 899.227 | OH | a2 | B17 | b2 | C04 | c2 | D02 | d1 | E04 |
| 2-1-m1E04-1-0646 | 899.233 | OH | a2 | B15 | b3 | C15 | c2 | D02 | d1 | E04 |
| 2-1-m1E04-1-0647 | 899.263 | OH | a2 | B13 | b1 | C09 | c2 | D02 | d1 | E04 |
| 2-1-m1E04-1-0648 | 899.263 | OH | a2 | B19 | b2 | C20 | c3 | D02 | d1 | E04 |
| 2-1-m1E04-1-0649 | 899.277 | OH | a2 | B16 | b1 | C13 | c2 | D02 | d1 | E04 |
| 2-1-m1E04-1-0650 | 899.282 | OH | a2 | B18 | b2 | C24 | c3 | D02 | d1 | E04 |
| 2-1-m1E04-1-0651 | 899.292 | OH | a1 | B10 | b2 | C05 | c1 | D01 | d1 | E04 |
| 2-1-m1E04-1-0652 | 899.302 | OH | a2 | B15 | b3 | C16 | c1 | D01 | d1 | E04 |
| 2-1-m1E04-1-0653 | 899.339 | OH | a2 | B13 | b1 | C31 | c3 | D02 | d1 | E04 |
| 2-1-m1E04-1-0654 | 900.185 | OH | a1 | B08 | b1 | C12 | c2 | D02 | d1 | E04 |
| 2-1-m1E04-1-0655 | 900.228 | OH | a1 | B06 | b2 | C06 | c2 | D02 | d1 | E04 |
| 2-1-m1E04-1-0656 | 900.228 | OH | a1 | B01 | b3 | C08 | c2 | D02 | d1 | E04 |
| 2-1-m1E04-1-0657 | 900.242 | OH | a1 | B07 | b2 | C02 | c2 | D02 | d1 | E04 |
| 2-1-m1E04-1-0658 | 900.242 | OH | a1 | B04 | b2 | C04 | c2 | D02 | d1 | E04 |
| 2-1-m1E04-1-0659 | 900.259 | OH | a2 | B19 | b2 | C28 | c3 | D02 | d1 | E04 |
| 2-1-m1E04-1-0660 | 900.279 | OH | a1 | B07 | b2 | C22 | c3 | D02 | d1 | E04 |
| 2-1-m1E04-1-0661 | 900.349 | OH | a1 | B09 | b2 | C07 | c1 | D01 | d1 | E04 |
| 2-1-m1E04-1-0662 | 901.194 | OH | a2 | B13 | b3 | C06 | c2 | D02 | d1 | E04 |
| 2-1-m1E04-1-0663 | 901.212 | OH | a2 | B16 | b3 | C15 | c2 | D02 | d1 | E04 |
| 2-1-m1E04-1-0664 | 901.223 | OH | a1 | B02 | b3 | C08 | c2 | D02 | d1 | E04 |
| 2-1-m1E04-1-0665 | 901.227 | OH | a2 | B18 | b2 | C06 | c2 | D02 | d1 | E04 |
| 2-1-m1E04-1-0666 | 901.238 | OH | a1 | B07 | b2 | C03 | c2 | D02 | d1 | E04 |
| 2-1-m1E04-1-0667 | 901.243 | OH | a2 | B20 | b3 | C08 | c1 | D01 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0668 | 901.243 | OH | a2 | B20 | b1 | C08 | c2 | D02 | d1 | E04 |
| 2-1-m1E04-1-0669 | 901.264 | OH | a2 | B15 | b3 | C17 | c1 | D01 | d1 | E04 |
| 2-1-m1E04-1-0670 | 901.264 | OH | a2 | B15 | b1 | C14 | c2 | D02 | d1 | E04 |
| 2-1-m1E04-1-0671 | 901.282 | OH | a2 | B16 | b3 | C16 | c1 | D01 | d1 | E04 |
| 2-1-m1E04-1-0672 | 902.238 | OH | a2 | B20 | b1 | C11 | c2 | D02 | d1 | E04 |
| 2-1-m1E04-1-0673 | 902.259 | OH | a2 | B13 | b3 | C18 | c1 | D01 | d1 | E04 |
| 2-1-m1E04-1-0674 | 902.272 | OH | a1 | B10 | b2 | C04 | c1 | D01 | d1 | E04 |
| 2-1-m1E04-1-0675 | 902.288 | OH | a1 | B03 | b1 | C13 | c2 | D02 | d1 | E04 |
| 2-1-m1E04-1-0676 | 903.222 | OH | a2 | B19 | b2 | C01 | c2 | D02 | d1 | E04 |
| 2-1-m1E04-1-0677 | 903.233 | OH | a2 | B20 | b1 | C10 | c2 | D02 | d1 | E04 |
| 2-1-m1E04-1-0678 | 903.238 | OH | a2 | B14 | b1 | C09 | c2 | D02 | d1 | E04 |
| 2-1-m1E04-1-0679 | 903.243 | OH | a2 | B16 | b3 | C17 | c1 | D01 | d1 | E04 |
| 2-1-m1E04-1-0680 | 903.243 | OH | a2 | B16 | b1 | C14 | c2 | D02 | d1 | E04 |
| 2-1-m1E04-1-0681 | 903.314 | OH | a2 | B14 | b1 | C31 | c3 | D02 | d1 | E04 |
| 2-1-m1E04-1-0682 | 904.223 | OH | a1 | B03 | b3 | C15 | c2 | D02 | d1 | E04 |
| 2-1-m1E04-1-0683 | 904.254 | OH | a1 | B09 | b3 | C08 | c1 | D01 | d1 | E04 |
| 2-1-m1E04-1-0684 | 904.254 | OH | a1 | B09 | b1 | C08 | c2 | D02 | d1 | E04 |
| 2-1-m1E04-1-0685 | 904.254 | OH | a1 | B01 | b1 | C09 | c2 | D02 | d1 | E04 |
| 2-1-m1E04-1-0686 | 904.254 | OH | a1 | B07 | b2 | C24 | c3 | D02 | d1 | E04 |
| 2-1-m1E04-1-0687 | 904.292 | OH | a1 | B03 | b3 | C16 | c1 | D01 | d1 | E04 |
| 2-1-m1E04-1-0688 | 904.329 | OH | a1 | B01 | b1 | C31 | c3 | D02 | d1 | E04 |
| 2-1-m1E04-1-0689 | 904.365 | OH | a1 | B05 | b1 | C29 | c3 | D02 | d1 | E04 |
| 2-1-m1E04-1-0690 | 905.169 | OH | a2 | B14 | b3 | C06 | c2 | D02 | d1 | E04 |
| 2-1-m1E04-1-0691 | 905.248 | OH | a2 | B17 | b1 | C13 | c2 | D02 | d1 | E04 |
| 2-1-m1E04-1-0692 | 905.249 | OH | a1 | B02 | b1 | C09 | c2 | D02 | d1 | E04 |
| 2-1-m1E04-1-0693 | 905.249 | OH | a1 | B09 | b1 | C11 | c2 | D02 | d1 | E04 |
| 2-1-m1E04-1-0694 | 905.268 | OH | a2 | B20 | b1 | C09 | c1 | D01 | d1 | E04 |
| 2-1-m1E04-1-0695 | 905.270 | OH | a1 | B08 | b1 | C30 | c3 | D02 | d1 | E04 |
| 2-1-m1E04-1-0696 | 905.324 | OH | a1 | B02 | b1 | C31 | c3 | D02 | d1 | E04 |
| 2-1-m1E04-1-0697 | 905.349 | OH | a2 | B15 | b2 | C07 | c2 | D02 | d1 | E04 |
| 2-1-m1E04-1-0698 | 906.185 | OH | a1 | B01 | b3 | C06 | c2 | D02 | d1 | E04 |
| 2-1-m1E04-1-0699 | 906.199 | OH | a1 | B07 | b2 | C06 | c2 | D02 | d1 | E04 |
| 2-1-m1E04-1-0700 | 906.234 | OH | a2 | B14 | b3 | C18 | c1 | D01 | d1 | E04 |
| 2-1-m1E04-1-0701 | 906.244 | OH | a1 | B09 | b1 | C10 | c2 | D02 | d1 | E04 |
| 2-1-m1E04-1-0702 | 906.254 | OH | a1 | B03 | b3 | C17 | c1 | D01 | d1 | E04 |
| 2-1-m1E04-1-0703 | 906.254 | OH | a1 | B03 | b1 | C14 | c2 | D02 | d1 | E04 |
| 2-1-m1E04-1-0704 | 906.263 | OH | a1 | B04 | b1 | C13 | c2 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0705 | 906.344 | OH | a1 | B06 | b1 | C29 | c3 | D02 | d1 | E04 |
| 2-1-m1E04-1-0706 | 907.180 | OH | a1 | B02 | b3 | C06 | c2 | D02 | d1 | E04 |
| 2-1-m1E04-1-0707 | 907.183 | OH | a2 | B17 | b3 | C15 | c2 | D02 | d1 | E04 |
| 2-1-m1E04-1-0708 | 907.199 | OH | a2 | B20 | b3 | C06 | c1 | D01 | d1 | E04 |
| 2-1-m1E04-1-0709 | 907.249 | OH | a1 | B01 | b3 | C18 | c1 | D01 | d1 | E04 |
| 2-1-m1E04-1-0710 | 907.249 | OH | a1 | B08 | b2 | C20 | c3 | D02 | d1 | E04 |
| 2-1-m1E04-1-0711 | 907.252 | OH | a2 | B17 | b3 | C16 | c1 | D01 | d1 | E04 |
| 2-1-m1E04-1-0712 | 907.303 | OH | a1 | B05 | b2 | C05 | c2 | D02 | d1 | E04 |
| 2-1-m1E04-1-0713 | 907.328 | OH | a2 | B16 | b2 | C07 | c2 | D02 | d1 | E04 |
| 2-1-m1E04-1-0714 | 907.344 | OH | a2 | B18 | b1 | C29 | c3 | D02 | d1 | E04 |
| 2-1-m1E04-1-0715 | 908.194 | OH | a2 | B20 | b1 | C12 | c2 | D02 | d1 | E04 |
| 2-1-m1E04-1-0716 | 908.198 | OH | a1 | B04 | b3 | C15 | c2 | D02 | d1 | E04 |
| 2-1-m1E04-1-0717 | 908.244 | OH | a1 | B02 | b3 | C18 | c1 | D01 | d1 | E04 |
| 2-1-m1E04-1-0718 | 908.244 | OH | a1 | B08 | b2 | C28 | c3 | D02 | d1 | E04 |
| 2-1-m1E04-1-0719 | 908.267 | OH | a1 | B04 | b3 | C16 | c1 | D01 | d1 | E04 |
| 2-1-m1E04-1-0720 | 908.279 | OH | a1 | B09 | b1 | C09 | c1 | D01 | d1 | E04 |
| 2-1-m1E04-1-0721 | 908.293 | OH | a1 | B10 | b1 | C13 | c1 | D01 | d1 | E04 |
| 2-1-m1E04-1-0722 | 908.324 | OH | a1 | B05 | b2 | C26 | c3 | D02 | d1 | E04 |
| 2-1-m1E04-1-0723 | 909.214 | OH | a2 | B17 | b3 | C17 | c1 | D01 | d1 | E04 |
| 2-1-m1E04-1-0724 | 909.214 | OH | a2 | B17 | b1 | C14 | c2 | D02 | d1 | E04 |
| 2-1-m1E04-1-0725 | 909.254 | OH | a2 | B15 | b3 | C08 | c2 | D02 | d1 | E04 |
| 2-1-m1E04-1-0726 | 909.283 | OH | a1 | B06 | b2 | C05 | c2 | D02 | d1 | E04 |
| 2-1-m1E04-1-0727 | 910.210 | OH | a1 | B09 | b3 | C06 | c1 | D01 | d1 | E04 |
| 2-1-m1E04-1-0728 | 910.228 | OH | a1 | B10 | b3 | C15 | c1 | D01 | d1 | E04 |
| 2-1-m1E04-1-0729 | 910.229 | OH | a1 | B04 | b3 | C17 | c1 | D01 | d1 | E04 |
| 2-1-m1E04-1-0730 | 910.229 | OH | a1 | B04 | b1 | C14 | c2 | D02 | d1 | E04 |
| 2-1-m1E04-1-0731 | 910.282 | OH | a2 | B18 | b2 | C05 | c2 | D02 | d1 | E04 |
| 2-1-m1E04-1-0732 | 910.283 | OH | a1 | B05 | b2 | C04 | c2 | D02 | d1 | E04 |
| 2-1-m1E04-1-0733 | 910.303 | OH | a1 | B06 | b2 | C26 | c3 | D02 | d1 | E04 |
| 2-1-m1E04-1-0734 | 910.339 | OH | a1 | B03 | b2 | C07 | c2 | D02 | d1 | E04 |
| 2-1-m1E04-1-0735 | 911.205 | OH | a1 | B09 | b1 | C12 | c2 | D02 | d1 | E04 |
| 2-1-m1E04-1-0736 | 911.208 | OH | a1 | B08 | b2 | C01 | c2 | D02 | d1 | E04 |
| 2-1-m1E04-1-0737 | 911.233 | OH | a2 | B16 | b3 | C08 | c2 | D02 | d1 | E04 |
| 2-1-m 1 E04-1-073 8 | 911.300 | OH | a2 | B19 | b1 | C32 | c3 | D02 | d1 | E04 |
| 2-1-m1E04-1-0739 | 911.302 | OH | a2 | B18 | b2 | C26 | c3 | D02 | d1 | E04 |
| 2-1-m1E04-1-0740 | 912.259 | OH | a1 | B10 | b1 | C14 | c1 | D01 | d1 | E04 |
| 2-1-m1E04-1-0741 | 912.262 | OH | a1 | B06 | b2 | C04 | c2 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0742 | 912.280 | OH | a1 | B10 | b2 | C33 | c3 | D02 | d1 | E04 |
| 2-1-m1E04-1-0743 | 912.315 | OH | a1 | B07 | b1 | C29 | c3 | D02 | d1 | E04 |
| 2-1-m1E04-1-0744 | 913.243 | OH | a2 | B19 | b2 | C02 | c2 | D02 | d1 | E04 |
| 2-1-m1E04-1-0745 | 913.262 | OH | a2 | B18 | b2 | C04 | c2 | D02 | d1 | E04 |
| 2-1-m1E04-1-0746 | 913.279 | OH | a2 | B15 | b1 | C09 | c2 | D02 | d1 | E04 |
| 2-1-m1E04-1-0747 | 913.279 | OH | a2 | B19 | b2 | C22 | c3 | D02 | d1 | E04 |
| 2-1-m1E04-1-0748 | 913.279 | OH | a2 | B20 | b1 | C30 | c3 | D02 | d1 | E04 |
| 2-1-m1E04-1-0749 | 913.299 | OH | a2 | B17 | b2 | C07 | c2 | D02 | d1 | E04 |
| 2-1-m1E04-1-0750 | 913.354 | OH | a2 | B15 | b1 | C31 | c3 | D02 | d1 | E04 |
| 2-1-m1E04-1-0751 | 914.238 | OH | a2 | B19 | b2 | C03 | c2 | D02 | d1 | E04 |
| 2-1-m1E04-1-0752 | 914.244 | OH | a1 | B03 | b3 | C08 | c2 | D02 | d1 | E04 |
| 2-1-m1E04-1-0753 | 914.314 | OH | a1 | B04 | b2 | C07 | c2 | D02 | d1 | E04 |
| 2-1-m1E04-1-0754 | 915.210 | OH | a2 | B15 | b3 | C06 | c2 | D02 | d1 | E04 |
| 2-1-m1E04-1-0755 | 915.253 | OH | a1 | B07 | b2 | C05 | c2 | D02 | d1 | E04 |
| 2-1-m1E04-1-0756 | 915.258 | OH | a2 | B16 | b1 | C09 | c2 | D02 | d1 | E04 |
| 2-1-m1E04-1-0757 | 915.258 | OH | a2 | B20 | b2 | C20 | c3 | D02 | d1 | E04 |
| 2-1-m1E04-1-0758 | 915.334 | OH | a2 | B16 | b1 | C31 | c3 | D02 | d1 | E04 |
| 2-1-m1E04-1-0759 | 916.254 | OH | a2 | B20 | b2 | C28 | c3 | D02 | d1 | E04 |
| 2-1-m1E04-1-0760 | 916.274 | OH | a1 | B07 | b2 | C26 | c3 | D02 | d1 | E04 |
| 2-1-m1E04-1-0761 | 916.275 | OH | a2 | B15 | b3 | C18 | c1 | D01 | d1 | E04 |
| 2-1-m1E04-1-0762 | 916.290 | OH | a1 | B09 | b1 | C30 | c3 | D02 | d1 | E04 |
| 2-1-m1E04-1-0763 | 916.304 | OH | a1 | B05 | b1 | C13 | c2 | D02 | d1 | E04 |
| 2-1-m1E04-1-0764 | 916.344 | OH | a1 | B10 | b2 | C07 | c1 | D01 | d1 | E04 |
| 2-1-m1E04-1-0765 | 917.189 | OH | a2 | B16 | b3 | C06 | c2 | D02 | d1 | E04 |
| 2-1-m1E04-1-0766 | 917.203 | OH | a2 | B17 | b3 | C08 | c2 | D02 | d1 | E04 |
| 2-1-m1E04-1-0767 | 917.254 | OH | a2 | B19 | b2 | C24 | c3 | D02 | d1 | E04 |
| 2-1-m1E04-1-0768 | 918.219 | OH | a1 | B04 | b3 | C08 | c2 11 | D02 | d1 | E04 |
| 2-1-m1E04-1-0769 | 918.233 | OH | a1 | B07 | b2 | C04 | c2 | D02 | d1 | E04 |
| 2-1-m1E04-1-0770 | 918.239 | OH | a1 | B05 | b3 | C15 | c2 | D02 | d1 | E04 |
| 2-1-m1E04-1-0771 | 918.254 | OH | a2 | B16 | b3 | C18 | c1 | D01 | d1 | E04 |
| 2-1-m1E04-1-0772 | 918.269 | OH | a1 | B03 | b1 | C09 | c2 | D02 | d1 | E04 |
| 2-1-m1E04-1-0773 | 918.269 | OH | a1 | B09 | b2 | C20 | c3 | D02 | d1 | E04 |
| 2-1-m1E04-1-0774 | 918.283 | OH | a1 | B06 | b1 | C13 | c2 | D02 | d1 | E04 |
| 2-1-m1E04-1-0775 | 918.308 | OH | a1 | B05 | b3 | C16 | c1 | D01 | d1 | E04 |
| 2-1-m1E04-1-0776 | 918.344 | OH | a1 | B03 | b1 | C31 | c3 | D02 | d1 | E04 |
| 2-1-m1E04-1-0777 | 919.199 | OH | a2 | B19 | b2 | C06 | c2 | D02 | d1 | E04 |
| 2-1-m1E04-1-0778 | 919.217 | OH | a2 | B20 | b2 | C01 | c2 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0779 | 919.264 | OH | a1 | B09 | b2 | C28 | c3 | D02 | d1 | E04 |
| 2-1-m1E04-1-0780 | 919.282 | OH | a2 | B18 | b1 | C13 | c2 | D02 | d1 | E04 |
| 2-1-m1E04-1-0781 | 919.286 | OH | a1 | B08 | b1 | C32 | c3 | D02 | d1 | E04 |
| 2-1-m1E04-1-0782 | 920.200 | OH | a1 | B03 | b3 | C06 | c2 | D02 | d1 | E04 |
| 2-1-m1E04-1-0783 | 920.218 | OH | a1 | B06 | b3 | C15 | c2 | D02 | d1 | E04 |
| 2-1-m1E04-1-0784 | 920.249 | OH | a1 | B10 | b3 | C08 | c1 | D01 | d1 | E04 |
| 2-1-m1E04-1-0785 | 920.249 | OH | a1 | B10 | b1 | C08 | c2 | D02 | d1 | E04 |
| 2-1-m1E04-1-0786 | 920.270 | OH | a1 | B05 | b3 | C17 | c1 | D01 | d1 | E04 |
| 2-1-m1E04-1-0787 | 920.270 | OH | a1 | B05 | b1 | C14 | c2 | D02 | d1 | E04 |
| 2-1-m1E04-1-0788 | 920.287 | OH | a1 | B06 | b3 | C16 | c1 | D01 | d1 | E04 |
| 2-1-m1E04-1-0789 | 921.217 | OH | a2 | B18 | b3 | C15 | c2 | D02 | d1 | E04 |
| 2-1-m1E04-1-0790 | 921.228 | OH | a1 | B08 | b2 | C02 | c2 | D02 | d1 | E04 |
| 2-1-m1E04-1-0791 | 921.229 | OH | a2 | B17 | b1 | C09 | c2 | D02 | d1 | E04 |
| 2-1-m1E04-1-0792 | 921.244 | OH | a1 | B10 | b1 | C11 | c2 | D02 | d1 | E04 |
| 2-1-m1E04-1-0793 | 921.265 | OH | a1 | B03 | b3 | C18 | c1 | D01 | d1 | E04 |
| 2-1-m1E04-1-0794 | 921.265 | OH | a1 | B08 | b2 | C22 | c3 | D02 | d1 | E04 |
| 2-1-m1E04-1-0795 | 921.287 | OH | a2 | B18 | b3 | C16 | c1 | D01 | d1 | E04 |
| 2-1-m1E04-1-0796 | 921.304 | OH | a2 | B17 | b1 | C31 | c3 | D02 | d1 | E04 |
| 2-1-m1E04-1-0797 | 922.224 | OH | a1 | B08 | b2 | C03 | c2 | D02 | d1 | E04 |
| 2-1-m1E04-1-0798 | 922.228 | OH | a1 | B09 | b2 | C01 | c2 | D02 | d1 | E04 |
| 2-1-m1E04-1-0799 | 922.239 | OH | a1 | B10 | b1 | C10 | c2 | D02 | d1 | E04 |
| 2-1-m1E04-1-0800 | 922.244 | OH | a1 | B04 | b1 | C09 | c2 | D02 | d1 | E04 |
| 2-1-m1E04-1-0801 | 922.249 | OH | a1 | B06 | b3 | C17 | c1 | D01 | d1 | E04 |
| 2-1-m1E04-1-0802 | 922.249 | OH | a1 | B06 | b1 | C14 | c2 | D02 | d1 | E04 |
| 2-1-m1E04-1-0803 | 922.319 | OH | a1 | B04 | b1 | C31 | c3 | D02 | d1 | E04 |
| 2-1-m1E04-1-0804 | 923.160 | OH | a2 | B17 | b3 | C06 | c2 | D02 | d1 | E04 |
| 2-1-m1E04-1-0805 | 923.248 | OH | a2 | B18 | b3 | C17 | c1 | D01 | d1 | E04 |
| 2-1-m1E04-1-0806 | 923.248 | OH | a2 | B18 | b1 | C14 | c2 | D02 | d1 | E04 |
| 2-1-m1E04-1-0807 | 924.175 | OH | a1 | B04 | b3 | C06 | c2 | D02 | d1 | E04 |
| 2-1-m1E04-1-0808 | 924.225 | OH | a2 | B17 | b3 | C18 | c1 | D01 | d1 | E04 |
| 2-1-m1E04-1-0809 | 924.254 | OH | a1 | B07 | b1 | C13 | c2 | D02 | d1 | E04 |
| 2-1-m1E04-1-0810 | 924.274 | OH | a1 | B10 | b1 | C09 | c1 | D01 | d1 | E04 |
| 2-1-m1E04-1-0811 | 924.355 | OH | a1 | B05 | b2 | C07 | c2 | D02 | d1 | E04 |
| 2-1-m1E04-1-0812 | 925.240 | OH | a1 | B04 | b3 | C18 | c1 | D01 | d1 | E04 |
| 2-1-m1E04-1-0813 | 925.240 | OH | a1 | B08 | b2 | C24 | c3 | D02 | d1 | E04 |
| 2-1-m1E04-1-0814 | 925.315 | OH | a2 | B19 | b1 | C29 | c3 | D02 | d1 | E04 |
| 2-1-m1E04-1-0815 | 926.189 | OH | a1 | B07 | b3 | C15 | c2 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0816 | 926.205 | OH | a1 | B10 | b3 | C06 | c1 | D01 | d1 | E04 |
| 2-1-m1E04-1-0817 | 926.258 | OH | a1 | B07 | b3 | C16 | c1 | D01 | d1 | E04 |
| 2-1-m1E04-1-0818 | 926.334 | OH | a1 | B06 | b2 | C07 | c2 | D02 | d1 | E04 |
| 2-1-m1E04-1-0819 | 927.185 | OH | a1 | B08 | b2 | C06 | c2 | D02 | d1 | E04 |
| 2-1-m1E04-1-0820 | 927.200 | OH | a1 | B10 | b1 | C12 | c2 | D02 | d1 | E04 |
| 2-1-m1E04-1-0821 | 927.295 | OH | a2 | B20 | b1 | C32 | c3 | D02 | d1 | E04 |
| 2-1-m1E04-1-0822 | 927.334 | OH | a2 | B18 | b2 | C07 | c2 | D02 | d1 | E04 |
| 2-1-m1E04-1-0823 | 928.219 | OH | a1 | B07 | b3 | C17 | c1 | D01 | d1 | E04 |
| 2-1-m1E04-1-0824 | 928.219 | OH | a1 | B07 | b1 | C14 | c2 | D02 | d1 | E04 |
| 2-1-m1E04-1-0825 | 928.254 | OH | a2 | B19 | b2 | C05 | c2 | D02 | d1 | E04 |
| 2-1-m1E04-1-0826 | 928.259 | OH | a1 | B05 | b3 | C08 | c2 | D02 | d1 | E04 |
| 2-1-m1E04-1-0827 | 929.238 | OH | a2 | B20 | b2 | C02 | c2 | D02 | d1 | E04 |
| 2-1-m1E04-1-0828 | 929.274 | OH | a2 | B20 | b2 | C22 | c3 | D02 | d1 | E04 |
| 2-1-m1E04-1-0829 | 929.274 | OH | a2 | B19 | b2 | C26 | c3 | D02 | d1 | E04 |
| 2-1-m1E04-1-0830 | 930.233 | OH | a2 | B20 | b2 | C03 | c2 | D02 | d1 | E04 |
| 2-1-m1E04-1-0831 | 930.239 | OH | a1 | B06 | b3 | C08 | c2 | D02 | d1 | E04 |
| 2-1-m1E04-1-0832 | 930.306 | OH | a1 | B09 | b1 | C32 | c3 | D02 | d1 | E04 |
| 2-1-m1E04-1-0833 | 931.233 | OH | a2 | B19 | b2 | C04 | c2 | D02 | d1 | E04 |
| 2-1-m1E04-1-0834 | 931.238 | OH | a2 | B18 | b3 | C08 | c2 | D02 | d1 | E04 |
| 2-1-m1E04-1-0835 | 932.249 | OH | a1 | B09 | b2 | C02 | c2 | D02 | d1 | E04 |
| 2-1-m1E04-1-0836 | 932.285 | OH | a1 | B05 | b1 | C09 | c2 | D02 | d1 | E04 |
| 2-1-m1E04-1-0837 | 932.285 | OH | a1 | B09 | b2 | C22 | c3 | D02 | d1 | E04 |
| 2-1-m1E04-1-0838 | 932.285 | OH | a1 | B10 | b1 | C30 | c3 | D02 | d1 | E04 |
| 2-1-m1E04-1-0839 | 932.305 | OH | a1 | B07 | b2 | C07 | c2 | D02 | d1 | E04 |
| 2-1-m1E04-1-0840 | 932.360 | OH | a1 | B05 | b1 | C31 | c3 | D02 | d1 | E04 |
| 2-1-m1E04-1-0841 | 933.244 | OH | a1 | B09 | b2 | C03 | c2 | D02 | d1 | E04 |
| 2-1-m1E04-1-0842 | 933.249 | OH | a2 | B20 | b2 | C24 | c3 | D02 | d1 | E04 |
| 2-1-m1E04-1-0843 | 933.301 | OH | a1 | B08 | b1 | C29 | c3 | D02 | d1 | E04 |
| 2-1-m1E04-1-0844 | 934.216 | OH | a1 | B05 | b3 | C06 | c2 | D02 | d1 | E04 |
| 2-1-m1E04-1-0845 | 934.264 | OH | a1 | B06 | b1 | C09 | c2 | D02 | d1 | E04 |
| 2-1-m1E04-1-0846 | 934.264 | OH | a1 | B10 | b2 | C20 | c3 | D02 | d1 | E04 |
| 2-1-m1E04-1-0847 | 934.339 | OH | a1 | B06 | b1 | C31 | c3 | D02 | d1 | E04 |
| 2-1-m1E04-1-0848 | 935.194 | OH | a2 | B20 | b2 | C06 | c2 | D02 | d1 | E04 |
| 2-1-m1E04-1-0849 | 935.233 | OH | a2 | B11 | b3 | C16 | c2 | D02 | d1 | E04 |
| 2-1-m1E04-1-0850 | 935.259 | OH | a1 | B10 | b2 | C28 | c3 | D02 | d1 | E04 |
| 2-1-m1E04-1-0851 | 935.263 | OH | a2 | B18 | b1 | C09 | c2 | D02 | d1 | E04 |
| 2-1-m1E04-1-0852 | 935.280 | OH | a1 | B05 | b3 | C18 | c1 | D01 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0853 | 935.339 | OH | a2 | B18 | b1 | C31 | c3 | D02 | d1 | E04 |
| 2-1-m1E04-1-0854 | 936.195 | OH | a1 | B06 | b3 | C06 | c2 | D02 | d1 | E04 |
| 2-1-m1E04-1-0855 | 936.209 | OH | a1 | B07 | b3 | C08 | c2 | D02 | d1 | E04 |
| 2-1-m1E04-1-0856 | 936.228 | OH | a2 | B12 | b3 | C16 | c2 | D02 | d1 | E04 |
| 2-1-m1E04-1-0857 | 936.239 | OH | a1 | B08 | b2 | C05 | c2 | D02 | d1 | E04 |
| 2-1-m1E04-1-0858 | 936.260 | OH | a1 | B09 | b2 | C24 | c3 | D02 | d1 | E04 |
| 2-1-m1E04-1-0859 | 937.194 | OH | a2 | B18 | b3 | C06 | c2 | D02 | d1 | E04 |
| 2-1-m1E04-1-0860 | 937.194 | OH | a2 | B11 | b3 | C17 | c2 | D02 | d1 | E04 |
| 2-1-m1E04-1-0861 | 937.254 | OH | a2 | B19 | b1 | C13 | c2 | D02 | d1 | E04 |
| 2-1-m1E04-1-0862 | 937.260 | OH | a1 | B06 | b3 | C18 | c1 | D01 | d1 | E04 |
| 2-1-m1E04-1-0863 | 937.260 | OH | a1 | B08 | b2 | C26 | c3 | D02 | d1 | E04 |
| 2-1-m1E04-1-0864 | 938.190 | OH | a2 | B12 | b3 | C17 | c2 | D02 | d1 | E04 |
| 2-1-m1E04-1-0865 | 938.205 | OH | a1 | B09 | b2 | C06 | c2 | D02 | d1 | E04 |
| 2-1-m1E04-1-0866 | 938.223 | OH | a1 | B10 | b2 | C01 | c2 | D02 | d1 | E04 |
| 2-1-m1E04-1-0867 | 938.259 | OH | a2 | B18 | b3 | C18 | c1 | D01 | d1 | E04 |
| 2-1-m1E04-1-0868 | 939.189 | OH | a2 | B19 | b3 | C15 | c2 | D02 | d1 | E04 |
| 2-1-m1E04-1-0869 | 939.219 | OH | a1 | B08 | b2 | C04 | c2 | D02 | d1 | E04 |
| 2-1-m1E04-1-0870 | 939.258 | OH | a2 | B19 | b3 | C16 | c1 | D01 | d1 | E04 |
| 2-1-m1E04-1-0871 | 940.235 | OH | a1 | B07 | b1 | C09 | c2 | D02 | d1 | E04 |
| 2-1-m1E04-1-0872 | 940.310 | OH | a1 | B07 | b1 | C31 | c3 | D02 | d1 | E04 |
| 2-1-m1E04-1-0873 | 941.220 | OH | a2 | B19 | b3 | C17 | c1 | D01 | d1 | E04 |
| 2-1-m1E04-1-0874 | 941.220 | OH | a2 | B19 | b1 | C14 | c2 | D02 | d1 | E04 |
| 2-1-m1E04-1-0875 | 941.310 | OH | a2 | B20 | b1 | C29 | c3 | D02 | d1 | E04 |
| 2-1-m1E04-1-0876 | 942.166 | OH | a1 | B07 | b3 | C06 | c2 | D02 | d1 | E04 |
| 2-1-m1E04-1-0877 | 943.230 | OH | a1 | B07 | b3 | C18 | c1 | D01 | d1 | E04 |
| 2-1-m1E04-1-0878 | 944.249 | OH | a2 | B20 | b2 | C05 | c2 | D02 | d1 | E04 |
| 2-1-m1E04-1-0879 | 944.321 | OH | a1 | B09 | b1 | C29 | c3 | D02 | d1 | E04 |
| 2-1-m1E04-1-0880 | 945.240 | OH | a1 | B08 | b1 | C13 | c2 | D02 | d1 | E04 |
| 2-1-m1E04-1-0881 | 945.269 | OH | a2 | B20 | b2 | C26 | c3 | D02 | d1 | E04 |
| 2-1-m1E04-1-0882 | 945.305 | OH | a2 | B19 | b2 | C07 | c2 | D02 | d1 | E04 |
| 2-1-m1E04-1-0883 | 946.301 | OH | a1 | B10 | b1 | C32 | c3 | D02 | d1 | E04 |
| 2-1-m1E04-1-0884 | 947.175 | OH | a1 | B08 | b3 | C15 | c2 | D02 | d1 | E04 |
| 2-1-m1E04-1-0885 | 947.228 | OH | a2 | B20 | b2 | C04 | c2 | D02 | d1 | E04 |
| 2-1-m1E04-1-0886 | 947.244 | OH | a1 | B08 | b3 | C16 | c1 | D01 | d1 | E04 |
| 2-1-m1E04-1-0887 | 947.259 | OH | a1 | B09 | b2 | C05 | c2 | D02 | d1 | E04 |
| 2-1-m1E04-1-0888 | 948.243 | OH | a1 | B10 | b2 | C02 | c2 | D02 | d1 | E04 |
| 2-1-m1E04-1-0889 | 948.280 | OH | a1 | B10 | b2 | C22 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0890 | 948.280 | OH | a1 | B09 | b2 | C26 | c3 | D02 | d1 | E04 |
| 2-1-m1E04-1-0891 | 949.206 | OH | a1 | B08 | b3 | C17 | c1 | D01 | d1 | E04 |
| 2-1-m1E04-1-0892 | 949.206 | OH | a1 | B08 | b1 | C14 | c2 | D02 | d1 | E04 |
| 2-1-m1E04-1-0893 | 949.210 | OH | a2 | B19 | b3 | C08 | c2 | D02 | d1 | E04 |
| 2-1-m1E04-1-0894 | 949.239 | OH | a1 | B10 | b2 | C03 | c2 | D02 | d1 | E04 |
| 2-1-m1E04-1-0895 | 949.249 | OH | a2 | B13 | b3 | C16 | c2 | D02 | d1 | E04 |
| 2-1-m1E04-1-0896 | 950.239 | OH | a1 | B09 | b2 | C04 | c2 | D02 | d1 | E04 |
| 2-1-m1E04-1-0897 | 951.210 | OH | a2 | B13 | b3 | C17 | c2 | D02 | d1 | E04 |
| 2-1-m1E04-1-0898 | 952.205 | OH | a2 | B11 | b3 | C18 | c2 | D02 | d1 | E04 |
| 2-1-m1E04-1-0899 | 952.255 | OH | a1 | B10 | b2 | C24 | c3 | D02 | d1 | E04 |
| 2-1-m1E04-1-0900 | 953.201 | OH | a2 | B12 | b3 | C18 | c2 | D02 | d1 | E04 |
| 2-1-m1E04-1-0901 | 953.223 | OH | a2 | B14 | b3 | C16 | c2 | D02 | d1 | E04 |
| 2-1-m1E04-1-0902 | 953.235 | OH | a2 | B19 | b1 | C09 | c2 | D02 | d1 | E04 |
| 2-1-m1E04-1-0903 | 953.249 | OH | a2 | B20 | b1 | C13 | c2 | D02 | d1 | E04 |
| 2-1-m1E04-1-0904 | 953.291 | OH | a1 | B08 | b2 | C07 | c2 | D02 | d1 | E04 |
| 2-1-m1E04-1-0905 | 953.310 | OH | a2 | B19 | b1 | C31 | c3 | D02 | d1 | E04 |
| 2-1-m1E04-1-0906 | 954.200 | OH | a1 | B10 | b2 | C06 | c2 | D02 | d1 | E04 |
| 2-1-m1E04-1-0907 | 954.239 | OH | a1 | B01 | b3 | C16 | c2 | D02 | d1 | E04 |
| 2-1-m1E04-1-0908 | 955.166 | OH | a2 | B19 | b3 | C06 | c2 | D02 | d1 | E04 |
| 2-1-m1E04-1-0909 | 955.184 | OH | a2 | B20 | 1 b3 | C15 | c2 | D02 | d1 | E04 |
| 2-1-m1E04-1-0910 | 955.185 | OH | a2 | B14 | b3 | C17 | c2 | D02 | d1 | E04 |
| 2-1-m1E04-1-0911 | 955.234 | OH | a1 | B02 | b3 | C16 | c2 | D02 | d1 | E04 |
| 2-1-m1E04-1-0912 | 955.253 | OH | a2 | B20 | b3 | C16 | c1 | D01 | d1 | E04 |
| 2-1-m1E04-1-0913 | 956.200 | OH | a1 | B01 | b3 | C17 | c2 | D02 | d1 | E04 |
| 2-1-m1E04-1-0914 | 956.231 | OH | a2 | B19 | b3 | C18 | c1 | D01 | d1 | E04 |
| 2-1-m1E04-1-0915 | 956.260 | OH | a1 | B09 | b1 | C13 | c2 | D02 | d1 | E04 |
| 2-1-m1E04-1-0916 | 957.195 | OH | a1 | B02 | b3 | C17 | c2 | D02 | d1 | E04 |
| 2-1-m1E04-1-0917 | 957.195 | OH | a1 | B08 | b3 | C08 | c2 | D02 | d1 | E04 |
| 2-1-m1E04-1-0918 | 957.215 | OH | a2 | B20 | b3 | C17 | c1 | D01 | d1 | E04 |
| 2-1-m1E04-1-0919 | 957.215 | OH | a2 | B20 | b1 | C14 | c2 | D02 | d1 | E04 |
| 2-1-m1E04-1-0920 | 958.195 | OH | a1 | B09 | b3 | C15 | c2 | D02 | d1 | E04 |
| 2-1-m1E04-1-0921 | 958.264 | OH | a1 | B09 | b3 | C16 | c1 | D01 | d1 | E04 |
| 2-1-m1E04-1-0922 | 960.226 | OH | a1 | B09 | b3 | C17 | c1 | D01 | d1 | E04 |
| 2-1-m1E04-1-0923 | 960.226 | OH | a1 | B09 | b1 | C14 | c2 | D02 | d1 | E04 |
| 2-1-m1E04-1-0924 | 960.316 | OH | a1 | B10 | b1 | C29 | c3 | D02 | d1 | E04 |
| 2-1-m1E04-1-0925 | 961.221 | OH | a1 | B08 | b1 | C09 | c2 | D02 | d1 | E04 |
| 2-1-m1E04-1-0926 | 961.296 | OH | a1 | B08 | b1 | C31 | c3 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0927 | 961.300 | OH | a2 | B20 | b2 | C07 | c2 | D02 | d1 | E04 |
| 2-1-m1E04-1-0928 | 963.152 | OH | a1 | B08 | b3 | C06 | c2 | D02 | d1 | E04 |
| 2-1-m1E04-1-0929 | 963.254 | OH | a1 | B10 | b2 | C05 | c2 | D02 | d1 | E04 |
| 2-1-m1E04-1-0930 | 963.264 | OH | a2 | B15 | b3 | C16 | c2 | D02 | d1 | E04 |
| 2-1-m1E04-1-0931 | 964.216 | OH | a1 | B08 | b3 | C18 | c1 | D01 | d1 | E04 |
| 2-1-m1E04-1-0932 | 964.275 | OH | a1 | B10 | b2 | C26 | c3 | D02 | d1 | E04 |
| 2-1-m1E04-1-0933 | 964.311 | OH | a1 | B09 | b2 | C07 | c2 | D02 | d1 | E04 |
| 2-1-m1E04-1-0934 | 965.205 | OH | a2 | B20 | b3 | C08 | c2 | D02 | d1 | E04 |
| 2-1-m1E04-1-0935 | 965.226 | OH | a2 | B15 | b3 | C17 | c2 | D02 | d1 | E04 |
| 2-1-m1E04-1-0936 | 965.243 | OH | a2 | B16 | b3 | C16 | c2 | D02 | d1 | E04 |
| 2-1-m1E04-1-0937 | 966.221 | OH | a2 | B13 | b3 | C18 | c2 | D02 | d1 | E04 |
| 2-1-m1E04-1-0938 | 966.234 | OH | a1 | B10 | b2 | C04 | c2 | D02 | d1 | E04 |
| 2-1-m1E04-1-0939 | 967.205 | OH | a2 | B16 | b3 | C17 | c2 | D02 | d1 | E04 |
| 2-1-m1E04-1-0940 | 968.215 | OH | a1 | B09 | b3 | C08 | c2 | D02 | d1 | E04 |
| 2-1-m1E04-1-0941 | 968.254 | OH | a1 | B03 | b3 | C16 | c2 | D02 | d1 | E04 |
| 2-1-m1E04-1-0942 | 969.230 | OH | a2 | B20 | b1 | C09 | c2 | D02 | d1 | E04 |
| 2-1-m1E04-1-0943 | 969.305 | OH | a2 | B20 | b1 | C31 | c3 | D02 | d1 | E04 |
| 2-1-m1E04-1-0944 | 970.196 | OH | a2 | B14 | b3 | C18 | c2 | D02 | d1 | E04 |
| 2-1-m1E04-1-0945 | 970.216 | OH | a1 | B03 | b3 | C17 | c2 | D02 | d1 | E04 |
| 2-1-m1E04-1-0946 | 971.161 | OH | a2 | B20 | b3 | C06 | c2 | D02 | d1 | E04 |
| 2-1-m1E04-1-0947 | 971.211 | OH | a1 | B01 | b3 | C18 | c2 | D02 | d1 | E04 |
| 2-1-m1E04-1-0948 | 971.214 | OH | a2 | B17 | b3 | C16 | c2 | D02 | d1 | E04 |
| 2-1-m1E04-1-0949 | 972.206 | OH | a1 | B02 | b3 | C18 | c2 | D02 | d1 | E04 |
| 2-1-m1E04-1-0950 | 972.226 | OH | a2 | B20 | b3 | C18 | c1 | D01 | d1 | E04 |
| 2-1-m1E04-1-0951 | 972.229 | OH | a1 | B04 | b3 | C16 | c2 | D02 | d1 | E04 |
| 2-1-m1E04-1-0952 | 972.241 | OH | a1 | B09 | b1 | C09 | c2 | D02 | d1 | E04 |
| 2-1-m1E04-1-0953 | 972.255 | OH | a1 | B10 | b1 | C13 | c2 | D02 | d1 | E04 |
| 2-1-m1E04-1-0954 | 972.316 | OH | a1 | B09 | b1 | C31 | c3 | D02 | d1 | E04 |
| 2-1-m1E04-1-0955 | 973.176 | OH | a2 | B17 | b3 | C17 | c2 | D02 | d1 | E04 |
| 2-1-m1E04-1-0956 | 974.172 | OH | a1 | B09 | b3 | C06 | c2 | D02 | d1 | E04 |
| 2-1-m1E04-1-0957 | 974.190 | OH | a1 | B10 | b3 | C15 | c2 | D02 | d1 | E04 |
| 2-1-m1E04-1-0958 | 974.191 | OH | a1 | B04 | b3 | C17 | c2 | D02 | d1 | E04 |
| 2-1-m1E04-1-0959 | 974.259 | OH | a1 | B10 | b3 | C16 | c1 | D01 | d1 | E04 |
| 2-1-m1E04-1-0960 | 975.237 | OH | a1 | B09 | b3 | C18 | c1 | D01 | d1 | E04 |
| 2-1-m1E04-1-0961 | 976.221 | OH | a1 | B10 | b3 | C17 | c1 | D01 | d1 | E04 |
| 2-1-m1E04-1-0962 | 976.221 | OH | a1 | B10 | b1 | C14 | c2 | D02 | d1 | E04 |
| 2-1-m1E04-1-0963 | 980.237 | OH | a2 | B15 | b3 | C18 | c2 | D02 | d1 | E04 |

(continued)

| [Table 8-8-5] Compound that may be contained in mixture 2-1-m1E04-1 | | | | | | | | | | |
|---|---|---|---|---|---|---|---|---|---|---|
| ID | Exact Mass | | a | B | b | C | c | D | d | E |
| 2-1-m1E04-1-0964 | 980.306 | OH | a1 | B10 | b2 | C07 | c2 | D02 | d1 | E04 |
| 2-1-m1E04-1-0965 | 982.216 | OH | a2 | B16 | b3 | C18 | c2 | D02 | d1 | E04 |
| 2-1-m1E04-1-0966 | 982.270 | OH | a1 | B05 | b3 | C16 | c2 | D02 | d1 | E04 |
| 2-1-m1E04-1-0967 | 984.210 | OH | a1 | B10 | b3 | C08 | c2 | D02 | d1 | E04 |
| 2-1-m1E04-1-0968 | 984.231 | OH | a1 | B05 | b3 | C17 | c2 | D02 | d1 | E04 |
| 2-1-m1E04-1-0969 | 984.249 | OH | a1 | B06 | b3 | C16 | c2 | D02 | d1 | E04 |
| 2-1-m1E04-1-0970 | 985.227 | OH | a1 | B03 | b3 | C18 | c2 | D02 | d1 | E04 |
| 2-1-m1E04-1-0971 | 985.249 | OH | a2 | B18 | b3 | C16 | c2 | D02 | d1 | E04 |
| 2-1-m1E04-1-0972 | 986.211 | OH | a1 | B06 | b3 | C17 | c2 | D02 | d1 | E04 |
| 2-1-m1E04-1-0973 | 987.210 | OH | a2 | B18 | b3 | C17 | c2 | D02 | d1 | E04 |
| 2-1-m1E04-1-0974 | 988.186 | OH | a2 | B17 | b3 | C18 | c2 | D02 | d1 | E04 |
| 2-1-m1E04-1-0975 | 988.236 | OH | a1 | B10 | b1 | C09 | c2 | D02 | d1 | E04 |
| 2-1-m1E04-1-0976 | 988.311 | OH | a1 | B10 | b1 | C31 | c3 | D02 | d1 | E04 |
| 2-1-m1E04-1-0977 | 989.202 | OH | a1 | B04 | b3 | C18 | c2 | D02 | d1 | E04 |
| 2-1-m1E04-1-0978 | 990.167 | OH | a1 | B10 | b3 | C06 | c2 | D02 | d1 | E04 |
| 2-1-m1E04-1-0979 | 990.220 | OH | a1 | B07 | b3 | C16 | c2 | D02 | d1 | E04 |
| 2-1-m1E04-1-0980 | 991.231 | OH | a1 | B10 | b3 | C18 | c1 | D01 | d1 | E04 |
| 2-1-m1E04-1-0981 | 992.181 | OH | a1 | B07 | b3 | C17 | c2 | D02 | d1 | E04 |
| 2-1-m1E04-1-0982 | 999.242 | OH | a1 | B05 | b3 | C18 | c2 | D02 | d1 | E04 |
| 2-1-m1E04-1-0983 | 1001.220 | OH | a1 | B06 | b3 | C18 | c2 | D02 | d1 | E04 |
| 2-1-m1E04-1-0984 | 1002.220 | OH | a2 | B18 | b3 | C18 | c2 | D02 | d1 | E04 |
| 2-1-m1E04-1-0985 | 1003.220 | OH | a2 | B19 | b3 | C16 | c2 | D02 | d1 | E04 |
| 2-1-m1E04-1-0986 | 1005.180 | OH | a2 | B19 | b3 | C17 | c2 | D02 | d1 | E04 |
| 2-1-m1E04-1-0987 | 1007.190 | OH | a1 | B07 | b3 | C18 | c2 | D02 | d1 | E04 |
| 2-1-m1E04-1-0988 | 1011.210 | OH | a1 | B08 | b3 | C16 | c2 | D02 | d1 | E04 |
| 2-1-m1E04-1-0989 | 1013.170 | OH | a1 | B08 | b3 | C17 | c2 | D02 | d1 | E04 |
| 2-1-m1E04-1-0990 | 1019.220 | OH | a2 | B20 | b3 | C16 | c2 | D02 | d1 | E04 |
| 2-1-m1E04-1-0991 | 1020.190 | OH | a2 | B19 | b3 | C18 | c2 | D02 | d1 | E04 |
| 2-1-m1E04-1-0992 | 1021.180 | OH | a2 | B20 | b3 | C17 | c2 | D02 | d1 | E04 |
| 2-1-m1E04-1-0993 | 1022.230 | OH | a1 | B09 | b3 | C16 | c2 | D02 | d1 | E04 |
| 2-1-m1E04-1-0994 | 1024.190 | OH | a1 | B09 | b3 | C17 | c2 | D02 | d1 | E04 |
| 2-1-m1E04-1-0995 | 1028.180 | OH | a1 | B08 | b3 | C18 | c2 | D02 | d1 | E04 |
| 2-1-m1E04-1-0996 | 1036.190 | OH | a2 | B20 | b3 | C18 | c2 | D02 | d1 | E04 |
| 2-1-m1E04-1-0997 | 1038.220 | OH | a1 | B10 | b3 | C16 | c2 | D02 | d1 | E04 |
| 2-1-m1E04-1-0998 | 1039.200 | OH | a1 | B09 | b3 | C18 | c2 | D02 | d1 | E04 |
| 2-1-m1E04-1-0999 | 1040.180 | OH | a1 | B10 | b3 | C17 | c2 | D02 | d1 | E04 |
| 2-1-m1E04-1-1000 | 1055.190 | OH | a1 | B10 | b3 | C18 | c2 | D02 | d1 | E04 |

Example 8-9: Synthesis of mixture 2-1-m1E01-1 to mixture 2-1-m1E04-1 by cleavage steps E01-1 to E04-1

A reaction formula in step E01-1 performed using mixture 2-1-m1E01-0 will be illustrated below.

[Formula 647]

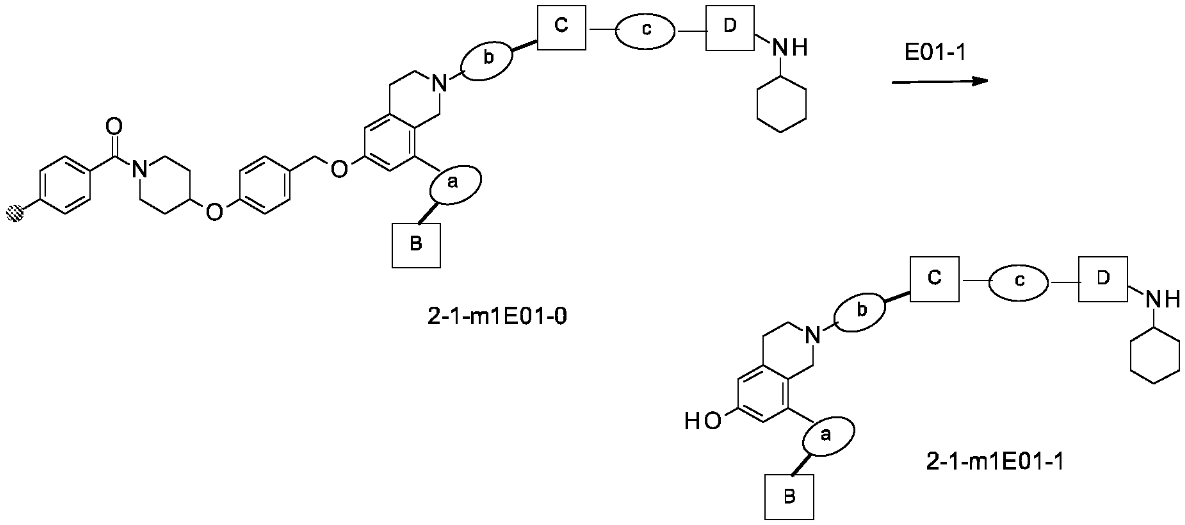


Mixture 2-1-m1E01-1 to mixture 2-1-m1E04-1 were synthesized as follows using mixture 2-1-mlE01-0 to mixture 2-1-m1E04-0.

Separate mixtures (115 mg) described in Table 8-9-1 were added to four 4 mL glass vials, respectively. 0.08 M pentamethylbenzene in DCM (2.3 mL) was added thereto, and each mixture was shaken at room temperature for 1 hour. TFA (0.23 mL) was added thereto, and the mixture was shaken at room temperature for 1 hour. The suspension of the reaction solution and the resin was transferred to a 6 mL column with a filter using DMI (1.1 mL) twice and filtered. The resultant was washed three times with DMI (1.1 mL), and the solvent in combined filtrates was distilled off under reduced pressure. Solid-phase supported morpholine (Sigma-Aldrich Co., LLC, catalog No: 493813) (175 mg) and DMI (0.92 mL) were added thereto, and the mixture was shaken at room temperature for 15 minutes. The solution and the suspension of the solid phase were transferred to a 6 mL column with a filter using DMI (1.1 mL) twice and filtered. The resultant was washed three times with DMI (1.1 mL) and twice with MeCN (0.5 mL), and the solvent in combined filtrates was distilled off by Genevac to obtain the mixtures described in Table 8-9-1. Each obtained residue was dissolved in DMSO having the amounts described in Table 8-9-1. 10 μL of the solution was dispensed for AS-MS assay. The solvent in the remaining solution was distilled off under reduced pressure, and the residue was used as a starting material in the subsequent steps E01- 2 to E04-2. The details of mixture 2-1-m1E01-1 to mixture 2-1-m 1 E04-1 are shown in Table 8-8-2 to Table 8-8-5.

[Table 398]

[Table 8-9-1] Starting material used and obtained mixture in steps E01-1 to E04-1

| Mixture used | Obtained mixture | Amount of DMSO used |
|---|---|---|
| 2-1-m1E01-0 | 2-1-m1E01-1 | 0.160 mL |
| 2-1-m1E02-0 | 2-1-m1E02-1 | 0.104 mL |
| 2-1-m1E03-0 | 2-1-m1E03-1 | 0.176 mL |
| 2-1-m1E04-0 | 2-1-m1E04-1 | 0.112 mL |

ID of compounds that may be contained in the mixtures synthesized in steps E01-1 to E04-1 is shown in Table 8-8-2 to Table 8-8-5.

In Table 8-8-2 to Table 8-8-5, each compound that may be contained in mixture 2-1- m1E01-1 to mixture 2-1-d1E04-1 is indicated by ID, and core blocks and linkers in combination in the structures of mixture 2-1-m1E01-0 to mixture 2-1-d1E04-0 are indicated by symbols or chemical formulas. The correspondence between the symbols and specific structures are shown in Figure 4.

Example 8-10: Synthesis of mixture 2-1-m1E01-2 to mixture 2-1-m1E04-2 by ethylation steps E01-2 to E04-2

A reaction formula in step E01-2 performed using mixture 2-1-m1E01-1 will be illustrated below.

[Formula 648]

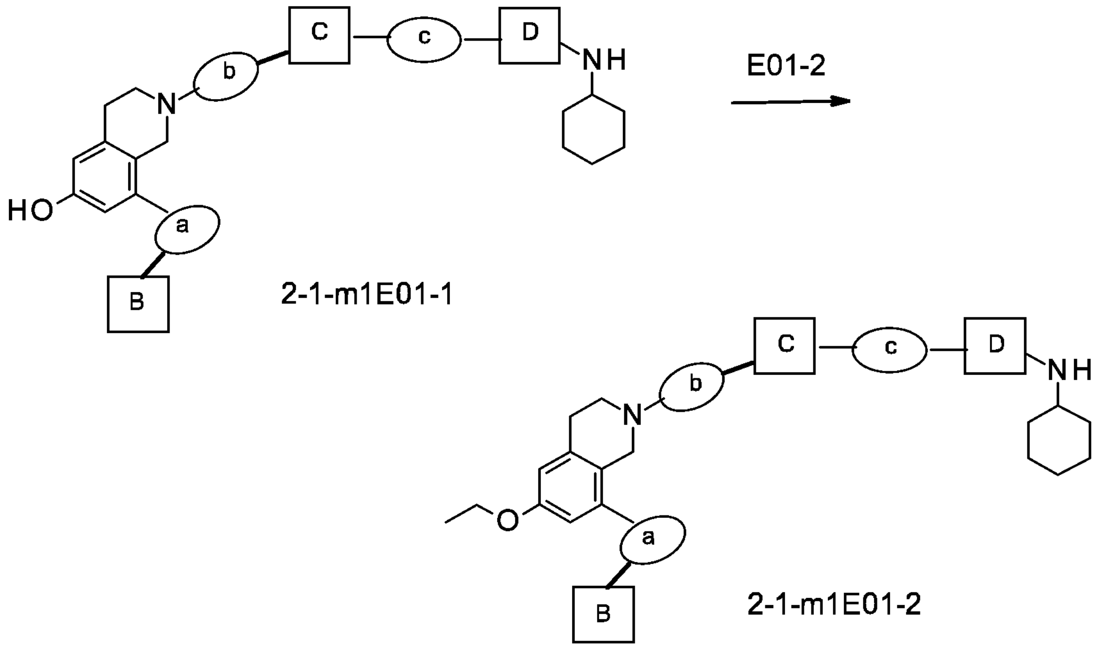


2-1-m1E01-1

2-1-m1E01-2

Mixture 2-1-m1E01-2 to mixture 2-1-m1E04-2 were synthesized as follows using mixture 2-1-m1E01-1 to mixture 2-1-m1E04-1.

DMF, EtOH, BTPP, and $Et_3PO_4$ were added in the amounts described in Table 8-10- 1 to the mixtures described in Table 8-10-1 contained in 0.5 to 2 mL microwave containers, and each mixture was shaken at 70°C for 27 hours. The reaction solution was transferred to a 6 mL column with a filter packed with ISOLUTE(R) CBA (0.7 mmol/g) in the amounts described in Table 8-10-1, using MeCN (0.2 mL) twice. 10 minutes later, the resultant was washed five times with MeCN (0.5 mL), and the solvent in combined filtrates was distilled off under reduced pressure to obtain the mixtures described in Table 8-10-1. The details of the mixtures are shown in Table 8-10-2 to Table 8-10-5.

[Table 399]

| [Table 8-10-1] Starting material used and obtained mixture in steps E01-2 to E04-2 | | | | | | |
|---|---|---|---|---|---|---|
| Mixture used | DMF | EtOH | Amount of BTPP used | Amount of $Et_3PO_4$ used | Amount of ISOLUTE(R) CBA used | Obtained mixture |
| 2-1-m1E01-1 | 154 μL | 38.6 μL | 41.3 μL (0.135 mmol) | 34.4 μL (0.203 mmol) | 189mg | 2-1-m1E01-2 |
| 2-1-m1E02-1 | 89 μL | 22.3 μL | 23.9 μL (0.078 mmol) | 19.9 μL (0.117 mmol) | 111mg | 2-1-m1E02-2 |

(continued)

| [Table 8-10-1] Starting material used and obtained mixture in steps E01-2 to E04-2 | | | | | | |
|---|---|---|---|---|---|---|
| Mixture used | DMF | EtOH | Amount of BTPP used | Amount of $Et_3PO_4$ used | Amount of ISOLUTE(R) CBA used | Obtained mixture |
| 2-1-m1E03-1 | 137 μL | 34.3 μL | 36.7 μL (0.120 mmol) | 30.6 μL (0.180 mmol) | 168mg | 2-1-m1E03-2 |
| 2-1-m1E04-1 | 96.0 μL | 24.0 μL | 25.7 μL (0.084 mmol) | 21.4 μL (0.126 mmol) | 118mg | 2-1-m1E04-2 |

A notation method in Table 8-10-2 to Table 8-10-5 will be described. In Table 8- 10-2 to Table 8-10-5, each compound that may be contained in mixture 2-1-m1E01-2 to mixture 2-1-m1E04-2 is indicated by ID, and core blocks and linkers in combination in the structures of mixture 2-1-m1E01-2 to mixture 2-1-m1E04-2 are indicated by symbols or chemical formulas. The correspondence between the symbols and specific structures are shown in Figure 4. In Table 8-10-2 to Table 8-10-5, "×" is represented by a chemical formula.

[Formula 649]

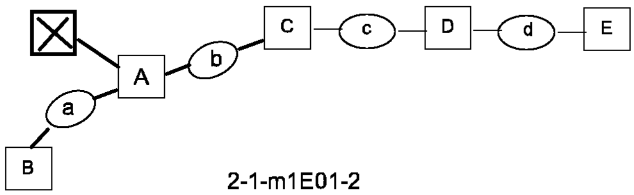


2-1-m1E01-2

For example, when ID is 2-1-m1E01-2-0001, the compound is represented by the following structural formula.

[Formula 650]

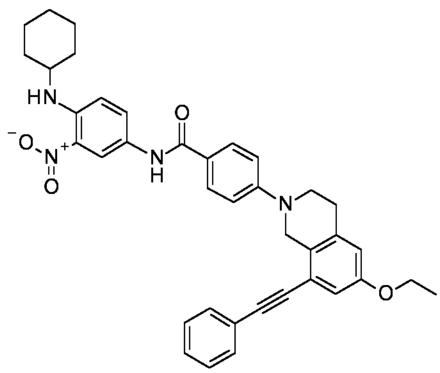

2-1-m1E01-2-0001

[Table 400]

| Notation in [Table 8-10-2] | | | | | | | | | |
|---|---|---|---|---|---|---|---|---|---|
| ID | | a | B | b | C | c | D | d | E |
| 2-1-m1E01-2-0001 | OEt | a2 | B11 | b1 | C08 | c1 | D01 | d1 | E01 |

[Table 401]

| [Table 8-10-1] Starting material used and obtained mixture in steps E01-2 to E04-2 | | | | | | |
|---|---|---|---|---|---|---|
| Mixture used | DMF | EtOH | Amount of BTPP used | Amount of $Et_3PO_4$ used | Amount of ISOLUTE(R) CBA used | Obtained mixture |
| 2-1-m1E01-1 | 154 μL | 38.6 μL | 41.3 μL (0.135 mmol) | 34.4 μL (0.203 mmol) | 189mg | 2-1-m1E01-2 |
| 2-1-m1E02-1 | 89 μL | 22.3 μL | 23.9 μL (0.078 mmol) | 19.9 μL (0.117 mmol) | 111mg | 2-1-m1E02-2 |
| 2-1-m1E03-1 | 137 μL | 34.3 μL | 36.7 μL (0.120 mmol) | 30.6 μL (0.180 mmol) | 168mg | 2-1-m1E03-2 |
| 2-1-m1E04-1 | 96.0 μL | 24.0 μL | 25.7 μL (0.084 mmol) | 21.4 μL (0.126 mmol) | 118mg | 2-1-m1E04-2 |

[Table 402]

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0001 | 614.289 | OEt | a2 | B11 | b1 | C08 | c1 | D01 | d1 | E01 |
| 2-1-m1E01-2-0002 | 615.285 | OEt | a2 | B12 | b1 | C08 | c1 | D01 | d1 | E01 |
| 2-1-m1E01-2-0003 | 615.285 | OEt | a2 | B11 | b1 | C11 | c1 | D01 | d1 | E01 |
| 2-1-m1E01-2-0004 | 616.280 | OEt | a2 | B11 | b1 | C10 | c1 | D01 | d1 | E01 |
| 2-1-m1E01-2-0005 | 616.280 | OEt | a2 | B12 | b1 | C11 | c1 | D01 | d1 | E01 |
| 2-1-m1E01-2-0006 | 617.275 | OEt | a2 | B12 | b1 | C10 | c1 | D01 | d1 | E01 |
| 2-1-m1E01-2-0007 | 621.241 | OEt | a2 | B11 | b1 | C12 | c1 | D01 | d1 | E01 |
| 2-1-m1E01-2-0008 | 622.236 | OEt | a2 | B12 | b1 | C12 | c1 | D01 | d1 | E01 |
| 2-1-m1E01-2-0009 | 628.305 | OEt | a2 | B13 | b1 | C08 | c1 | D01 | d1 | E01 |
| 2-1-m1E01-2-0010 | 629.300 | OEt | a2 | B13 | b1 | C11 | c1 | D01 | d1 | E01 |
| 2-1-m1E01-2-0011 | 630.295 | OEt | a2 | B13 | b1 | C10 | c1 | D01 | d1 | E01 |
| 2-1-m1E01-2-0012 | 632.263 | OEt | a2 | B11 | b2 | C01 | c1 | D01 | d1 | E01 |
| 2-1-m1E01-2-0013 | 632.280 | OEt | a2 | B14 | b1 | C08 | c1 | D01 | d1 | E01 |
| 2-1-m1E01-2-0014 | 633.259 | OEt | a2 | B12 | b2 | C01 | c1 | D01 | d1 | E01 |
| 2-1-m1E01-2-0015 | 633.275 | OEt | a2 | B14 | b1 | C11 | c1 | D01 | d1 | E01 |
| 2-1-m1E01-2-0016 | 633.295 | OEt | a1 | B01 | b1 | C08 | c1 | D01 | d1 | E01 |
| 2-1-m1E01-2-0017 | 634.270 | OEt | a2 | B14 | b1 | C10 | c1 | D01 | d1 | E01 |
| 2-1-m1E01-2-0018 | 634.290 | OEt | a1 | B02 | b1 | C08 | c1 | D01 | d1 | E01 |
| 2-1-m1E01-2-0019 | 634.290 | OEt | a1 | B01 | b1 | C11 | c1 | D01 | d1 | E01 |
| 2-1-m1E01-2-0020 | 635.257 | OEt | a2 | B13 | b1 | C12 | c1 | D01 | d1 | E01 |
| 2-1-m1E01-2-0021 | 635.286 | OEt | a1 | B01 | b1 | C10 | c1 | D01 | d1 | E01 |
| 2-1-m1E01-2-0022 | 635.286 | OEt | a1 | B02 | b1 | C11 | c1 | D01 | d1 | E01 |
| 2-1-m1E01-2-0023 | 636.281 | OEt | a1 | B02 | b1 | C10 | c1 | D01 | d1 | E01 |
| 2-1-m1E01-2-0024 | 639.232 | OEt | a2 | B14 | b1 | C12 | c1 | D01 | d1 | E01 |
| 2-1-m1E01-2-0025 | 640.247 | OEt | a1 | B01 | b1 | C12 | c1 | D01 | d1 | E01 |
| 2-1-m1E01-2-0026 | 641.242 | OEt | a1 | B02 | b1 | C12 | c1 | D01 | d1 | E01 |
| 2-1-m1E01-2-0027 | 642.284 | OEt | a2 | B11 | b2 | C02 | c1 | D01 | d1 | E01 |
| 2-1-m1E01-2-0028 | 642.321 | OEt | a2 | B15 | b1 | C08 | c1 | D01 | d1 | E01 |
| 2-1-m1E01-2-0029 | 643.279 | OEt | a2 | B12 | b2 | C02 | c1 | D01 | d1 | E01 |
| 2-1-m1E01-2-0030 | 643.279 | OEt | a2 | B11 | b2 | C03 | c1 | D01 | d1 | E01 |
| 2-1-m1E01-2-0031 | 643.316 | OEt | a2 | B15 | b1 | C11 | c1 | D01 | d1 | E01 |
| 2-1-m1E01-2-0032 | 644.275 | OEt | a2 | B12 | b2 | C03 | c1 | D01 | d1 | E01 |
| 2-1-m1E01-2-0033 | 644.300 | OEt | a2 | B16 | b1 | C08 | c1 | D01 | d1 | E01 |
| 2-1-m1E01-2-0034 | 644.311 | OEt | a2 | B15 | b1 | C10 | c1 | D01 | d1 | E01 |
| 2-1-m1E01-2-0035 | 645.295 | OEt | a2 | B16 | b1 | C11 | c1 | D01 | d1 | E01 |
| 2-1-m1E01-2-0036 | 646.279 | OEt | a2 | B13 | b2 | C01 | c1 | D01 | d1 | E01 |
| 2-1-m1E01-2-0037 | 646.290 | OEt | a2 | B16 | b1 | C10 | c1 | D01 | d1 | E01 |
| 2-1-m1E01-2-0038 | 647.311 | OEt | a1 | B03 | b1 | C08 | c1 | D01 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0039 | 648.241 | OEt | a2 | B11 | b2 | C06 | c1 | D01 | d1 | E01 |
| 2-1-m1E01-2-0040 | 648.306 | OEt | a1 | B03 | b1 | C11 | c1 | D01 | d1 | E01 |
| 2-1-m1E01-2-0041 | 649.236 | OEt | a2 | B12 | b2 | C06 | c1 | D01 | d1 | E01 |
| 2-1-m1E01-2-0042 | 649.272 | OEt | a2 | B15 | b1 | C12 | c1 | D01 | d1 | E01 |
| 2-1-m1E01-2-0043 | 649.301 | OEt | a1 | B03 | b1 | C10 | c1 | D01 | d1 | E01 |
| 2-1-m1E01-2-0044 | 650.254 | OEt | a2 | B14 | b2 | C01 | c1 | D01 | d1 | E01 |
| 2-1-m1E01-2-0045 | 650.270 | OEt | a2 | B17 | b1 | C08 | c1 | D01 | d1 | E01 |
| 2-1-m1E01-2-0046 | 651.252 | OEt | a2 | B16 | b1 | C12 | c1 | D01 | d1 | E01 |
| 2-1-m1E01-2-0047 | 651.266 | OEt | a2 | B17 | b1 | C11 | c1 | D01 | d1 | E01 |
| 2-1-m1E01-2-0048 | 651.269 | OEt | a1 | B01 | b2 | C01 | c1 | D01 | d1 | E01 |
| 2-1-m1E01-2-0049 | 651.286 | OEt | a1 | B04 | b1 | C08 | c1 | D01 | d1 | E01 |
| 2-1-m1E01-2-0050 | 652.261 | OEt | a2 | B17 | b1 | C10 | c1 | D01 | d1 | E01 |
| 2-1-m1E01-2-0051 | 652.265 | OEt | a1 | B02 | b2 | C01 | c1 | D01 | d1 | E01 |
| 2-1-m1E01-2-0052 | 652.281 | OEt | a1 | B04 | b1 | C11 | c1 | D01 | d1 | E01 |
| 2-1-m1E01-2-0053 | 653.276 | OEt | a1 | B04 | b1 | C10 | c1 | D01 | d1 | E01 |
| 2-1-m1E01-2-0054 | 654.262 | OEt | a1 | B03 | b1 | C12 | c1 | D01 | d1 | E01 |
| 2-1-m1E01-2-0055 | 656.300 | OEt | a2 | B13 | b2 | C02 | c1 | D01 | d1 | E01 |
| 2-1-m1E01-2-0056 | 657.222 | OEt | a2 | B17 | b1 | C12 | c1 | D01 | d1 | E01 |
| 2-1-m1E01-2-0057 | 657.295 | OEt | a2 | B13 | b2 | C03 | c1 | D01 | d1 | E01 |
| 2-1-m1E01-2-0058 | 657.295 | OEt | a2 | B11 | b2 | C05 | c1 | D01 | d1 | E01 |
| 2-1-m1E01-2-0059 | 658.237 | OEt | a1 | B04 | b1 | C12 | c1 | D01 | d1 | E01 |
| 2-1-m1E01-2-0060 | 658.290 | OEt | a2 | B12 | b2 | C05 | c1 | D01 | d1 | E01 |
| 2-1-m1E01-2-0061 | 660.275 | OEt | a2 | B14 | b2 | C02 | c1 | D01 | d1 | E01 |
| 2-1-m1E01-2-0062 | 660.275 | OEt | a2 | B11 | b2 | C04 | c1 | D01 | d1 | E01 |
| 2-1-m1E01-2-0063 | 660.295 | OEt | a2 | B15 | b2 | C01 | c1 | D01 | d1 | E01 |
| 2-1-m1E01-2-0064 | 661.270 | OEt | a2 | B12 | b2 | C04 | c1 | D01 | d1 | E01 |
| 2-1-m1E01-2-0065 | 661.270 | OEt | a2 | B14 | b2 | C03 | c1 | D01 | d1 | E01 |
| 2-1-m1E01-2-0066 | 661.290 | OEt | a1 | B01 | b2 | C02 | c1 | D01 | d1 | E01 |
| 2-1-m1E01-2-0067 | 661.326 | OEt | a1 | B05 | b1 | C08 | c1 | D01 | d1 | E01 |
| 2-1-m1E01-2-0068 | 662.256 | OEt | a2 | B13 | b2 | C06 | c1 | D01 | d1 | E01 |
| 2-1-m1E01-2-0069 | 662.274 | OEt | a2 | B16 | b2 | C01 | c1 | D01 | d1 | E01 |
| 2-1-m1E01-2-0070 | 662.285 | OEt | a1 | B02 | b2 | C02 | c1 | D01 | d1 | E01 |
| 2-1-m1E01-2-0071 | 662.285 | OEt | a1 | B01 | b2 | C03 | c1 | D01 | d1 | E01 |
| 2-1-m1E01-2-0072 | 662.322 | OEt | a1 | B05 | b1 | C11 | c1 | D01 | d1 | E01 |
| 2-1-m1E01-2-0073 | 663.281 | OEt | a1 | B02 | b2 | C03 | c1 | D01 | d1 | E01 |
| 2-1-m1E01-2-0074 | 663.306 | OEt | a1 | B06 | b1 | C08 | c1 | D01 | d1 | E01 |
| 2-1-m1E01-2-0075 | 663.317 | OEt | a1 | B05 | b1 | C10 | c1 | D01 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0076 | 664.301 | OEt | a1 | B06 | b1 | C11 | c1 | D01 | d1 | E01 |
| 2-1-m1E01-2-0077 | 664.305 | OEt | a2 | B18 | b1 | C08 | c1 | D01 | d1 | E01 |
| 2-1-m1E01-2-0078 | 665.285 | OEt | a1 | B03 | b2 | C01 | c1 | D01 | d1 | E01 |
| 2-1-m1E01-2-0079 | 665.296 | OEt | a1 | B06 | b1 | C10 | c1 | D01 | d1 | E01 |
| 2-1-m1E01-2-0080 | 665.300 | OEt | a2 | B18 | b1 | C11 | c1 | D01 | d1 | E01 |
| 2-1-m1E01-2-0081 | 666.231 | OEt | a2 | B14 | b2 | C06 | c1 | D01 | d1 | E01 |
| 2-1-m1E01-2-0082 | 666.295 | OEt | a2 | B18 | b1 | C10 | c1 | D01 | d1 | E01 |
| 2-1-m1E01-2-0083 | 666.295 | OEt | a2 | B11 | b1 | C13 | c1 | D01 | d1 | E01 |
| 2-1-m1E01-2-0084 | 667.246 | OEt | a1 | B01 | b2 | C06 | c1 | D01 | d1 | E01 |
| 2-1-m1E01-2-0085 | 667.291 | OEt | a2 | B12 | b1 | C13 | c1 | D01 | d1 | E01 |
| 2-1-m1E01-2-0086 | 668.230 | OEt | a2 | B11 | b3 | C15 | c1 | D01 | d1 | E01 |
| 2-1-m1E01-2-0087 | 668.242 | OEt | a1 | B02 | b2 | C06 | c1 | D01 | d1 | E01 |
| 2-1-m1E01-2-0088 | 668.245 | OEt | a2 | B17 | b2 | C01 | c1 | D01 | d1 | E01 |
| 2-1-m1E01-2-0089 | 668.278 | OEt | a1 | B05 | b1 | C12 | c1 | D01 | d1 | E01 |
| 2-1-m1E01-2-0090 | 669.226 | OEt | a2 | B12 | b3 | C15 | c1 | D01 | d1 | E01 |
| 2-1-m1E01-2-0091 | 669.260 | OEt | a1 | B04 | b2 | C01 | c1 | D01 | d1 | E01 |
| 2-1-m1E01-2-0092 | 669.276 | OEt | a1 | B07 | b1 | C08 | c1 | D01 | d1 | E01 |
| 2-1-m1E01-2-0093 | 670.257 | OEt | a1 | B06 | b1 | C12 | c1 | D01 | d1 | E01 |
| 2-1-m1E01-2-0094 | 670.261 | OEt | a2 | B11 | b1 | C14 | c1 | D01 | d1 | E01 |
| 2-1-m1E01-2-0095 | 670.272 | OEt | a1 | B07 | b1 | C11 | c1 | D01 | d1 | E01 |
| 2-1-m1E01-2-0096 | 670.283 | OEt | a2 | B11 | b2 | C33 | c3 | D02 | d1 | E01 |
| 2-1-m1E01-2-0097 | 670.316 | OEt | a2 | B15 | b2 | C02 | c1 | D01 | d1 | E01 |
| 2-1-m1E01-2-0098 | 671.257 | OEt | a2 | B18 | b1 | C12 | c1 | D01 | d1 | E01 |
| 2-1-m1E01-2-0099 | 671.257 | OEt | a2 | B12 | b1 | C14 | c1 | D01 | d1 | E01 |
| 2-1-m1E01-2-0100 | 671.267 | OEt | a1 | B07 | b1 | C10 | c1 | D01 | d1 | E01 |
| 2-1-m1E01-2-0101 | 671.278 | OEt | a2 | B12 | b2 | C33 | c3 | D02 | d1 | E01 |
| 2-1-m1E01-2-0102 | 671.311 | OEt | a2 | B15 | b2 | C03 | c1 | D01 | d1 | E01 |
| 2-1-m1E01-2-0103 | 671.311 | OEt | a2 | B13 | b2 | C05 | c1 | D01 | d1 | E01 |
| 2-1-m1E01-2-0104 | 672.295 | OEt | a2 | B16 | b2 | C02 | c1 | D01 | d1 | E01 |
| 2-1-m1E01-2-0105 | 673.290 | OEt | a2 | B16 | b2 | C03 | c1 | D01 | d1 | E01 |
| 2-1-m1E01-2-0106 | 674.290 | OEt | a2 | B13 | b2 | C04 | c1 | D01 | d1 | E01 |
| 2-1-m1E01-2-0107 | 674.347 | OEt | a2 | B11 | b2 | C07 | c1 | D01 | d1 | E01 |
| 2-1-m1E01-2-0108 | 675.286 | OEt | a2 | B14 | b2 | C05 | c1 | D01 | d1 | E01 |
| 2-1-m1E01-2-0109 | 675.306 | OEt | a1 | B03 | b2 | C02 | c1 | D01 | d1 | E01 |
| 2-1-m1E01-2-0110 | 675.342 | OEt | a2 | B12 | b2 | C07 | c1 | D01 | d1 | E01 |
| 2-1-m1E01-2-0111 | 676.228 | OEt | a1 | B07 | b1 | C12 | c1 | D01 | d1 | E01 |
| 2-1-m1E01-2-0112 | 676.272 | OEt | a2 | B15 | b2 | C06 | c1 | D01 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0113 | 676.301 | OEt | a1 | B03 | b2 | C03 | c1 | D01 | d1 | E01 |
| 2-1-m1E01-2-0114 | 676.301 | OEt | a1 | B01 | b2 | C05 | c1 | D01 | d1 | E01 |
| 2-1-m1E01-2-0115 | 677.296 | OEt | a1 | B02 | b2 | C05 | c1 | D01 | d1 | E01 |
| 2-1-m1E01-2-0116 | 678.251 | OEt | a2 | B16 | b2 | C06 | c1 | D01 | d1 | E01 |
| 2-1-m1E01-2-0117 | 678.251 | OEt | a2 | B11 | b3 | C08 | c1 | D01 | d1 | E01 |
| 2-1-m1E01-2-0118 | 678.251 | OEt | a2 | B11 | b1 | C08 | c2 | D02 | d1 | E01 |
| 2-1-m1E01-2-0119 | 678.265 | OEt | a2 | B17 | b2 | C02 | c1 | D01 | d1 | E01 |
| 2-1-m1E01-2-0120 | 678.265 | OEt | a2 | B14 | b2 | C04 | c1 | D01 | d1 | E01 |
| 2-1-m1E01-2-0121 | 679.246 | OEt | a2 | B12 | b3 | C08 | c1 | D01 | d1 | E01 |
| 2-1-m1E01-2-0122 | 679.246 | OEt | a2 | B12 | b1 | C08 | c2 | D02 | d1 | E01 |
| 2-1-m1E01-2-0123 | 679.246 | OEt | a2 | B11 | b1 | C11 | c2 | D02 | d1 | E01 |
| 2-1-m1E01-2-0124 | 679.261 | OEt | a2 | B17 | b2 | C03 | c1 | D01 | d1 | E01 |
| 2-1-m1E01-2-0125 | 679.281 | OEt | a1 | B04 | b2 | C02 | c1 | D01 | d1 | E01 |
| 2-1-m1E01-2-0126 | 679.281 | OEt | a1 | B01 | b2 | C04 | c1 | D01 | d1 | E01 |
| 2-1-m1E01-2-0127 | 679.301 | OEt | a1 | B05 | b2 | C01 | c1 | D01 | d1 | E01 |
| 2-1-m1E01-2-0128 | 680.242 | OEt | a2 | B11 | b1 | C10 | c2 | D02 | d1 | E01 |
| 2-1-m1E01-2-0129 | 680.242 | OEt | a2 | B12 | b1 | C11 | c2 | D02 | d1 | E01 |
| 2-1-m1E01-2-0130 | 680.276 | OEt | a1 | B02 | b2 | C04 | c1 | D01 | d1 | E01 |
| 2-1-m1E01-2-0131 | 680.276 | OEt | a1 | B04 | b2 | C03 | c1 | D01 | d1 | E01 |
| 2-1-m1E01-2-0132 | 680.311 | OEt | a2 | B13 | b1 | C13 | c1 | D01 | d1 | E01 |
| 2-1-m1E01-2-0133 | 681.237 | OEt | a2 | B12 | b1 | C10 | c2 | D02 | d1 | E01 |
| 2-1-m1E01-2-0134 | 681.262 | OEt | a1 | B03 | b2 | C06 | c1 | D01 | d1 | E01 |
| 2-1-m1E01-2-0135 | 681.280 | OEt | a1 | B06 | b2 | C01 | c1 | D01 | d1 | E01 |
| 2-1-m1E01-2-0136 | 682.246 | OEt | a2 | B13 | b3 | C15 | c1 | D01 | d1 | E01 |
| 2-1-mlE01-2-0137 | 682.277 | OEt | a2 | B19 | b1 | C08 | c1 | D01 | d1 | E01 |
| 2-1-m1E01-2-0138 | 682.277 | OEt | a2 | B11 | b1 | C09 | c1 | D01 | d1 | E01 |
| 2-1-mlE01-2-0139 | 682.279 | OEt | a2 | B18 | b2 | C01 | c1 | D01 | d1 | E01 |
| 2-1-m1E01-2-0140 | 683.272 | OEt | a2 | B12 | b1 | C09 | c1 | D01 | d1 | E01 |
| 2-1-mlE01-2-0141 | 683.272 | OEt | a2 | B19 | b1 | C11 | c1 | D01 | d1 | E01 |
| 2-1-m1E01-2-0142 | 684.208 | OEt | a2 | B11 | b3 | C06 | c1 | D01 | d1 | E01 |
| 2-1-mlE01-2-0143 | 684.222 | OEt | a2 | B17 | b2 | C06 | c1 | D01 | d1 | E01 |
| 2-1-m1E01-2-0144 | 684.267 | OEt | a2 | B19 | b1 | C10 | c1 | D01 | d1 | E01 |
| 2-1-m1E01-2-0145 | 684.277 | OEt | a2 | B13 | b1 | C14 | c1 | D01 | d1 | E01 |
| 2-1-m1E01-2-0146 | 684.286 | OEt | a2 | B14 | b1 | C13 | c1 | D01 | d1 | E01 |
| 2-1-mlE01-2-0147 | 684.298 | OEt | a2 | B13 | b2 | C33 | c3 | D02 | d1 | E01 |
| 2-1-m1E01-2-0148 | 685.203 | OEt | a2 | B12 | b3 | C06 | c1 | D01 | d1 | E01 |
| 2-1-mlE01-2-0149 | 685.203 | OEt | a2 | B11 | b1 | C12 | c2 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0150 | 685.237 | OEt | a1 | B04 | b2 | C06 | c1 | D01 | d1 | E01 |
| 2-1-m1E01-2-0151 | 685.301 | OEt | a1 | B01 | b1 | C13 | c1 | D01 | d1 | E01 |
| 2-1-mlE01-2-0152 | 685.326 | OEt | a2 | B15 | b2 | C05 | c1 | D01 | d1 | E01 |
| 2-1-m1E01-2-0153 | 686.198 | OEt | a2 | B12 | b1 | C12 | c2 | D02 | d1 | E01 |
| 2-1-m1E01-2-0154 | 686.221 | OEt | a2 | B14 | b3 | C15 | c1 | D01 | d1 | E01 |
| 2-1-m1E01-2-0155 | 686.297 | OEt | a1 | B02 | b1 | C13 | c1 | D01 | d1 | E01 |
| 2-1-m1E01-2-0156 | 687.236 | OEt | a1 | B01 | b3 | C15 | c1 | D01 | d1 | E01 |
| 2-1-mlE01-2-0157 | 687.250 | OEt | a1 | B07 | b2 | C01 | c1 | D01 | d1 | E01 |
| 2-1-m1E01-2-0158 | 687.306 | OEt | a2 | B16 | b2 | C05 | c1 | D01 | d1 | E01 |
| 2-1-mlE01-2-0159 | 688.232 | OEt | a1 | B02 | b3 | C15 | c1 | D01 | d1 | E01 |
| 2-1-m1E01-2-0160 | 688.252 | OEt | a2 | B14 | b1 | C14 | c1 | D01 | d1 | E01 |
| 2-1-mlE01-2-0161 | 688.273 | OEt | a2 | B14 | b2 | C33 | c3 | D02 | d1 | E01 |
| 2-1-m1E01-2-0162 | 688.306 | OEt | a2 | B15 | b2 | C04 | c1 | D01 | d1 | E01 |
| 2-1-m1E01-2-0163 | 688.362 | OEt | a2 | B13 | b2 | C07 | c1 | D01 | d1 | E01 |
| 2-1-mlE01-2-0164 | 689.228 | OEt | a2 | B19 | b1 | C12 | c1 | D01 | d1 | E01 |
| 2-1-m1E01-2-0165 | 689.267 | OEt | a1 | B01 | b1 | C14 | c1 | D01 | d1 | E01 |
| 2-1-mlE01-2-0166 | 689.288 | OEt | a1 | B01 | b2 | C33 | c3 | D02 | d1 | E01 |
| 2-1-m1E01-2-0167 | 689.321 | OEt | a1 | B05 | b2 | C02 | c1 | D01 | d1 | E01 |
| 2-1-mlE01-2-0168 | 690.262 | OEt | a1 | B05 | b1 | C08 | c1 | D01 | d1 | E01 |
| 2-1-mlE01-2-0169 | 690.262 | OEt | a1 | B02 | b1 | C14 | c1 | D01 | d1 | E01 |
| 2-1-m1E01-2-0170 | 690.284 | OEt | a1 | B02 | b2 | C33 | c3 | D02 | d1 | E01 |
| 2-1-m1E01-2-0171 | 690.285 | OEt | a2 | B16 | b2 | C04 | c1 | D01 | d1 | E01 |
| 2-1-m1E01-2-0172 | 690.288 | OEt | a2 | B11 | b1 | C30 | c3 | D02 | d1 | E01 |
| 2-1-m1E01-2-0173 | 690.317 | OEt | a1 | B05 | b2 | C03 | c1 | D01 | d1 | E01 |
| 2-1-mlE01-2-0174 | 690.317 | OEt | a1 | B03 | b2 | C05 | c1 | D01 | d1 | E01 |
| 2-1-m1E01-2-0175 | 691.258 | OEt | a1 | B08 | b1 | C11 | c1 | D01 | d1 | E01 |
| 2-1-mlE01-2-0176 | 691.283 | OEt | a2 | B12 | b1 | C30 | c3 | D02 | d1 | E01 |
| 2-1-m1E01-2-0177 | 691.301 | OEt | a1 | B06 | b2 | C02 | c1 | D01 | d1 | E01 |
| 2-1-mlE01-2-0178 | 692.253 | OEt | a1 | B08 | b1 | C10 | c1 | D01 | d1 | E01 |
| 2-1-mlE01-2-0179 | 692.267 | OEt | a2 | B13 | b3 | C08 | c1 | D01 | d1 | E01 |
| 2-1-mlE01-2-0180 | 692.267 | OEt | a2 | B13 | b1 | C08 | c2 | D02 | d1 | E01 |
| 2-1-mlE01-2-0181 | 692.267 | OEt | a2 | B11 | b2 | C20 | c3 | D02 | d1 | E01 |
| 2-1-m1E01-2-0182 | 692.296 | OEt | a1 | B06 | b2 | C03 | c1 | D01 | d1 | E01 |
| 2-1-m1E01-2-0183 | 692.300 | OEt | a2 | B18 | b2 | C02 | c1 | D01 | d1 | E01 |
| 2-1-mlE01-2-0184 | 692.337 | OEt | a2 | B14 | b2 | C07 | c1 | D01 | d1 | E01 |
| 2-1-m1E01-2-0185 | 693.262 | OEt | a2 | B13 | b1 | C11 | c2 | D02 | d1 | E01 |
| 2-1-mlE01-2-0186 | 693.262 | OEt | a2 | B12 | b2 | C20 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0187 | 693.262 | OEt | a2 | B11 | b2 | C28 | c3 | D02 | d1 | E01 |
| 2-1-mlE01-2-0188 | 693.276 | OEt | a2 | B17 | b2 | C05 | c1 | D01 | d1 | E01 |
| 2-1-m1E01-2-0189 | 693.295 | OEt | a2 | B18 | b2 | C03 | c1 | D01 | d1 | E01 |
| 2-1-m1E01-2-0190 | 693.296 | OEt | a1 | B03 | b2 | C04 | c1 | D01 | d1 | E01 |
| 2-1-mlE01-2-0191 | 693.353 | OEt | a1 | B01 | b2 | C07 | c1 | D01 | d1 | E01 |
| 2-1-m1E01-2-0192 | 694.257 | OEt | a2 | B13 | b1 | C10 | c2 | D02 | d1 | E01 |
| 2-1-mlE01-2-0193 | 694.257 | OEt | a2 | B12 | b2 | C28 | c3 | D02 | d1 | E01 |
| 2-1-mlE01-2-0194 | 694.292 | OEt | a1 | B04 | b2 | COS | c1 | D01 | d1 | E01 |
| 2-1-m1E01-2-0195 | 694.327 | OEt | a2 | B15 | b1 | C13 | c1 | D01 | d1 | E01 |
| 2-1-mlE01-2-0196 | 694.348 | OEt | a1 | B02 | b2 | C07 | c1 | D01 | d1 | E01 |
| 2-1-mlE01-2-0197 | 695.278 | OEt | a1 | B05 | b2 | C06 | c1 | D01 | d1 | E01 |
| 2-1-m1E01-2-0198 | 696.225 | OEt | a2 | B11 | b2 | C01 | c2 | D02 | d1 | E01 |
| 2-1-m1E01-2-0199 | 696.242 | OEt | a2 | B14 | b3 | C08 | c1 | D01 | d1 | E01 |
| 2-1-mlE01-2-0200 | 696.242 | OEt | a2 | B14 | b1 | C08 | c2 | D02 | d1 | E01 |
| 2-1-m1E01-2-0201 | 696.256 | OEt | a2 | B17 | b2 | C04 | c1 | D01 | d1 | E01 |
| 2-1-m1E01-2-0202 | 696.262 | OEt | a2 | B15 | b3 | C15 | c1 | D01 | d1 | E01 |
| 2-1-m1E01-2-0203 | 696.292 | OEt | a2 | B13 | b1 | C09 | c1 | D01 | d1 | E01 |
| 2-1-m1E01-2-0204 | 696.306 | OEt | a2 | B16 | b1 | C13 | c1 | D01 | d1 | E01 |
| 2-1-mlE01-2-0205 | 697.214 | OEt | a1 | B08 | b1 | C12 | c1 | D01 | d1 | E01 |
| 2-1-m1E01-2-0206 | 697.221 | OEt | a2 | B12 | b2 | C01 | c2 | D02 | d1 | E01 |
| 2-1-m1E01-2-0207 | 697.237 | OEt | a2 | B14 | b1 | C11 | c2 | D02 | d1 | E01 |
| 2-1-m1E01-2-0208 | 697.257 | OEt | a1 | B06 | b2 | C06 | c1 | D01 | d1 | E01 |
| 2-1-m1E01-2-0209 | 697.257 | OEt | a1 | B01 | b3 | C08 | c1 | D01 | d1 | E01 |
| 2-1-mlE01-2-0210 | 697.257 | OEt | a1 | B01 | b1 | C08 | c2 | D02 | d1 | E01 |
| 2-1-m1E01-2-0211 | 697.271 | OEt | a1 | B07 | b2 | C02 | c1 | D01 | d1 | E01 |
| 2-1-mlE01-2-0212 | 697.271 | OEt | a1 | B04 | b2 | C04 | c1 | D01 | d1 | E01 |
| 2-1-mlE01-2-0213 | 698.223 | OEt | a2 | B13 | b3 | C06 | c1 | D01 | d1 | E01 |
| 2-1-m1E01-2-0214 | 698.232 | OEt | a2 | B14 | b1 | C10 | c2 | D02 | d1 | E01 |
| 2-1-mlE01-2-0215 | 698.241 | OEt | a2 | B16 | b3 | C15 | c1 | D01 | d1 | E01 |
| 2-1-mlE01-2-0216 | 698.252 | OEt | a1 | B02 | b3 | C08 | c1 | D01 | d1 | E01 |
| 2-1-m1E01-2-0217 | 698.252 | OEt | a1 | B02 | b1 | C08 | c2 | D02 | d1 | E01 |
| 2-1-mlE01-2-0218 | 698.252 | OEt | a1 | B01 | b1 | C11 | c2 | D02 | d1 | E01 |
| 2-1-m1E01-2-0219 | 698.256 | OEt | a2 | B18 | b2 | C06 | c1 | D01 | d1 | E01 |
| 2-1-mlE01-2-0220 | 698.266 | OEt | a1 | B07 | b2 | C03 | c1 | D01 | d1 | E01 |
| 2-1-m1E01-2-0221 | 698.272 | OEt | a2 | B20 | b1 | C08 | c1 | D01 | d1 | E01 |
| 2-1-m1E01-2-0222 | 698.293 | OEt | a2 | B15 | b1 | C14 | c1 | D01 | d1 | E01 |
| 2-1-mlE01-2-0223 | 698.314 | OEt | a2 | B15 | b2 | C33 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0224 | 699.219 | OEt | a2 | B13 | b1 | C12 | c2 | D02 | d1 | E01 |
| 2-1-mlE01-2-0225 | 699.248 | OEt | a1 | B01 | b1 | C10 | c2 | D02 | d1 | E01 |
| 2-1-mlE01-2-0226 | 699.248 | OEt | a1 | B02 | b1 | C11 | c2 | D02 | d1 | E01 |
| 2-1-mlE01-2-0227 | 699.267 | OEt | a2 | B20 | b1 | C11 | c1 | D01 | d1 | E01 |
| 2-1-mlE01-2-0228 | 699.317 | OEt | a1 | B03 | b1 | C13 | c1 | D01 | d1 | E01 |
| 2-1-mlE01-2-0229 | 700.243 | OEt | a1 | B02 | b1 | C10 | c2 | D02 | d1 | E01 |
| 2-1-mlE01-2-0230 | 700.251 | OEt | a2 | B19 | b2 | C01 | c1 | D01 | d1 | E01 |
| 2-1-m1E01-2-0231 | 700.262 | OEt | a2 | B20 | b1 | C10 | c1 | D01 | d1 | E01 |
| 2-1-m1E01-2-0232 | 700.267 | OEt | a2 | B14 | b1 | C09 | c1 | D01 | d1 | E01 |
| 2-1-mlE01-2-0233 | 700.272 | OEt | a2 | B16 | b1 | C14 | c1 | D01 | d1 | E01 |
| 2-1-m1E01-2-0234 | 700.293 | OEt | a2 | B16 | b2 | C33 | c3 | D02 | d1 | E01 |
| 2-1-mlE01-2-0235 | 701.252 | OEt | a1 | B03 | b3 | C15 | c1 | D01 | d1 | E01 |
| 2-1-m1E01-2-0236 | 701.283 | OEt | a1 | B09 | b1 | C08 | c1 | D01 | d1 | E01 |
| 2-1-m1E01-2-0237 | 701.283 | OEt | a1 | B01 | b1 | C09 | c1 | D01 | d1 | E01 |
| 2-1-mlE01-2-0238 | 702.198 | OEt | a2 | B14 | b3 | C06 | c1 | D01 | d1 | E01 |
| 2-1-mlE01-2-0239 | 702.277 | OEt | a2 | B17 | b1 | C13 | c1 | D01 | d1 | E01 |
| 2-1-m1E01-2-0240 | 702.278 | OEt | a1 | B02 | b1 | C09 | c1 | D01 | d1 | E01 |
| 2-1-m1E01-2-0241 | 702.278 | OEt | a1 | B09 | b1 | C11 | c1 | D01 | d1 | E01 |
| 2-1-m1E01-2-0242 | 702.378 | OEt | a2 | B15 | b2 | C07 | c1 | D01 | d1 | E01 |
| 2-1-m1E01-2-0243 | 703.193 | OEt | a2 | B14 | b1 | C12 | c2 | D02 | d1 | E01 |
| 2-1-m1E01-2-0244 | 703.213 | OEt | a1 | B01 | b3 | C06 | c1 | D01 | d1 | E01 |
| 2-1-mlE01-2-0245 | 703.228 | OEt | a1 | B07 | b2 | C06 | c1 | D01 | d1 | E01 |
| 2-1-m1E01-2-0246 | 703.273 | OEt | a1 | B09 | b1 | C10 | c1 | D01 | d1 | E01 |
| 2-1-m1E01-2-0247 | 703.283 | OEt | a1 | B03 | b1 | C14 | c1 | D01 | d1 | E01 |
| 2-1-m1E01-2-0248 | 703.292 | OEt | a1 | B04 | b1 | C13 | c1 | D01 | d1 | E01 |
| 2-1-mlE01-2-0249 | 703.304 | OEt | a1 | B03 | b2 | C33 | c3 | D02 | d1 | E01 |
| 2-1-m1E01-2-0250 | 704.209 | OEt | a1 | B02 | b3 | C06 | c1 | D01 | d1 | E01 |
| 2-1-m1E01-2-0251 | 704.209 | OEt | a1 | B01 | b1 | C12 | c2 | D02 | d1 | E01 |
| 2-1-m1E01-2-0252 | 704.212 | OEt | a2 | B17 | b3 | C15 | c1 | D01 | d1 | E01 |
| 2-1-m1E01-2-0253 | 704.303 | OEt | a2 | B13 | b1 | C30 | c3 | D02 | d1 | E01 |
| 2-1-m1E01-2-0254 | 704.303 | OEt | a2 | B11 | b1 | C32 | c3 | D02 | d1 | E01 |
| 2-1-m1E01-2-0255 | 704.332 | OEt | a1 | B05 | b2 | COS | c1 | D01 | d1 | E01 |
| 2-1-m1E01-2-0256 | 704.357 | OEt | a2 | B16 | b2 | C07 | c1 | D01 | d1 | E01 |
| 2-1-m1E01-2-0257 | 705.204 | OEt | a1 | B02 | b1 | C12 | c2 | D02 | d1 | E01 |
| 2-1-mlE01-2-0258 | 705.223 | OEt | a2 | B20 | b1 | C12 | c1 | D01 | d1 | E01 |
| 2-1-m1E01-2-0259 | 705.227 | OEt | a1 | B04 | b3 | C15 | c1 | D01 | d1 | E01 |
| 2-1-mlE01-2-0260 | 705.298 | OEt | a2 | B12 | b1 | C32 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0261 | 706.243 | OEt | a2 | B17 | b1 | C14 | c1 | D01 | d1 | E01 |
| 2-1-m1E01-2-0262 | 706.246 | OEt | a2 | B11 | b2 | C02 | c2 | D02 | d1 | E01 |
| 2-1-m1E01-2-0263 | 706.264 | OEt | a2 | B17 | b2 | C33 | c3 | D02 | d1 | E01 |
| 2-1-mlE01-2-0264 | 706.283 | OEt | a2 | B15 | b3 | C08 | c1 | D01 | d1 | E01 |
| 2-1-m1E01-2-0265 | 706.283 | OEt | a2 | B15 | b1 | C08 | c2 | D02 | d1 | E01 |
| 2-1-mlE01-2-0266 | 706.283 | OEt | a2 | B13 | b2 | C20 | c3 | D02 | d1 | E01 |
| 2-1-mlE01-2-0267 | 706.283 | OEt | a2 | B11 | b2 | C22 | c3 | D02 | d1 | E01 |
| 2-1-mlE01-2-0268 | 706.311 | OEt | a1 | B06 | b2 | C05 | c1 | D01 | d1 | E01 |
| 2-1-mlE01-2-0269 | 707.241 | OEt | a2 | B12 | b2 | C02 | c2 | D02 | d1 | E01 |
| 2-1-mlE01-2-0270 | 707.241 | OEt | a2 | B11 | b2 | C03 | c2 | D02 | d1 | E01 |
| 2-1-m1E01-2-0271 | 707.258 | OEt | a1 | B04 | b1 | C14 | c1 | D01 | d1 | E01 |
| 2-1-m1E01-2-0272 | 707.278 | OEt | a2 | B15 | b1 | C11 | c2 | D02 | d1 | E01 |
| 2-1-m1E01-2-0273 | 707.278 | OEt | a2 | B12 | b2 | C22 | c3 | D02 | d1 | E01 |
| 2-1-mlE01-2-0274 | 707.278 | OEt | a2 | B13 | b2 | C28 | c3 | D02 | d1 | E01 |
| 2-1-m1E01-2-0275 | 707.279 | OEt | a1 | B04 | b2 | C33 | c3 | D02 | d1 | E01 |
| 2-1-m1E01-2-0276 | 707.311 | OEt | a2 | B18 | b2 | COS | c1 | D01 | d1 | E01 |
| 2-1-m1E01-2-0277 | 707.312 | OEt | a1 | B05 | b2 | C04 | c1 | D01 | d1 | E01 |
| 2-1-m1E01-2-0278 | 707.368 | OEt | a1 | B03 | b2 | C07 | c1 | D01 | d1 | E01 |
| 2-1-m1E01-2-0279 | 708.234 | OEt | a1 | B09 | b1 | C12 | c1 | D01 | d1 | E01 |
| 2-1-m1E01-2-0280 | 708.237 | OEt | a1 | B08 | b2 | C01 | c1 | D01 | d1 | E01 |
| 2-1-mlE01-2-0281 | 708.237 | OEt | a2 | B12 | b2 | C03 | c2 | D02 | d1 | E01 |
| 2-1-mlE01-2-0282 | 708.262 | OEt | a2 | B16 | b3 | C08 | c1 | D01 | d1 | E01 |
| 2-1-m1E01-2-0283 | 708.262 | OEt | a2 | B16 | b1 | C08 | c2 | D02 | d1 | E01 |
| 2-1-m1E01-2-0284 | 708.273 | OEt | a2 | B15 | b1 | C10 | c2 | D02 | d1 | E01 |
| 2-1-m1E01-2-0285 | 708.278 | OEt | a2 | B14 | b1 | C30 | c3 | D02 | d1 | E01 |
| 2-1-m1E01-2-0286 | 709.257 | OEt | a2 | B16 | b1 | C11 | c2 | D02 | d1 | E01 |
| 2-1-m1E01-2-0287 | 709.291 | OEt | a1 | B06 | b2 | C04 | c1 | D01 | d1 | E01 |
| 2-1-m1E01-2-0288 | 709.293 | OEt | a1 | B01 | b1 | C30 | c3 | D02 | d1 | E01 |
| 2-1-m1E01-2-0289 | 710.241 | OEt | a2 | B13 | b2 | C01 | c2 | D02 | d1 | E01 |
| 2-1-mlE01-2-0290 | 710.252 | OEt | a2 | B16 | b1 | C10 | c2 | D02 | d1 | E01 |
| 2-1-m1E01-2-0291 | 710.257 | OEt | a2 | B14 | b2 | C20 | c3 | D02 | d1 | E01 |
| 2-1-mlE01-2-0292 | 710.257 | OEt | a2 | B11 | b2 | C24 | c3 | D02 | d1 | E01 |
| 2-1-m1E01-2-0293 | 710.272 | OEt | a2 | B19 | b2 | C02 | c1 | D01 | d1 | E01 |
| 2-1-mlE01-2-0294 | 710.289 | OEt | a1 | B02 | b1 | C30 | c3 | D02 | d1 | E01 |
| 2-1-m1E01-2-0295 | 710.290 | OEt | a2 | B18 | b2 | C04 | c1 | D01 | d1 | E01 |
| 2-1-m1E01-2-0296 | 710.308 | OEt | a2 | B15 | b1 | C09 | c1 | D01 | d1 | E01 |
| 2-1-mlE01-2-0297 | 710.328 | OEt | a2 | B17 | b2 | C07 | c1 | D01 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0298 | 711.253 | OEt | a2 | B12 | b2 | C24 | c3 | D02 | d1 | E01 |
| 2-1-m1E01-2-0299 | 711.253 | OEt | a2 | B14 | b2 | C28 | c3 | D02 | d1 | E01 |
| 2-1-m1E01-2-0300 | 711.267 | OEt | a2 | B19 | b2 | C03 | c1 | D01 | d1 | E01 |
| 2-1-mlE01-2-0301 | 711.273 | OEt | a1 | B03 | b3 | C08 | c1 | D01 | d1 | E01 |
| 2-1-m1E01-2-0302 | 711.273 | OEt | a1 | B03 | b1 | C08 | c2 | D02 | d1 | E01 |
| 2-1-mlE01-2-0303 | 711.273 | OEt | a1 | B01 | b2 | C20 | c3 | D02 | d1 | E01 |
| 2-1-mlE01-2-0304 | 711.343 | OEt | a1 | B04 | b2 | C07 | c1 | D01 | d1 | E01 |
| 2-1-m1E01-2-0305 | 712.203 | OEt | a2 | B11 | b2 | C06 | c2 | D02 | d1 | E01 |
| 2-1-mlE01-2-0306 | 712.239 | OEt | a2 | B15 | b3 | C06 | c1 | D01 | d1 | E01 |
| 2-1-m1E01-2-0307 | 712.268 | OEt | a1 | B03 | b1 | C11 | c2 | D02 | d1 | E01 |
| 2-1-mlE01-2-0308 | 712.268 | OEt | a1 | B02 | b2 | C20 | c3 | D02 | d1 | E01 |
| 2-1-m1E01-2-0309 | 712.268 | OEt | a1 | B01 | b2 | C28 | c3 | D02 | d1 | E01 |
| 2-1-m1E01-2-0310 | 712.282 | OEt | a1 | B07 | b2 | COS | c1 | D01 | d1 | E01 |
| 2-1-mlE01-2-0311 | 712.287 | OEt | a2 | B16 | b1 | C09 | c1 | D01 | d1 | E01 |
| 2-1-m1E01-2-0312 | 713.198 | OEt | a2 | B12 | b2 | C06 | c2 | D02 | d1 | E01 |
| 2-1-mlE01-2-0313 | 713.234 | OEt | a2 | B15 | b1 | C12 | c2 | D02 | d1 | E01 |
| 2-1-mlE01-2-0314 | 713.263 | OEt | a1 | B03 | b1 | C10 | c2 | D02 | d1 | E01 |
| 2-1-mlE01-2-0315 | 713.263 | OEt | a1 | B02 | b2 | C28 | c3 | D02 | d1 | E01 |
| 2-1-m1E01-2-0316 | 713.333 | OEt | a1 | B05 | b1 | C13 | c1 | D01 | d1 | E01 |
| 2-1-m1E01-2-0317 | 714.216 | OEt | a2 | B14 | b2 | C01 | c2 | D02 | d1 | E01 |
| 2-1-m1E01-2-0318 | 714.218 | OEt | a2 | B16 | b3 | C06 | c1 | D01 | d1 | E01 |
| 2-1-m1E01-2-0319 | 714.232 | OEt | a2 | B17 | b3 | C08 | c1 | D01 | d1 | E01 |
| 2-1-m1E01-2-0320 | 714.232 | OEt | a2 | B17 | b1 | C08 | c2 | D02 | d1 | E01 |
| 2-1-mlE01-2-0321 | 715.213 | OEt | a2 | B16 | b1 | C12 | c2 | D02 | d1 | E01 |
| 2-1-m1E01-2-0322 | 715.228 | OEt | a2 | B17 | b1 | C11 | c2 | D02 | d1 | E01 |
| 2-1-m1E01-2-0323 | 715.231 | OEt | a1 | B01 | b2 | C01 | c2 | D02 | d1 | E01 |
| 2-1-mlE01-2-0324 | 715.248 | OEt | a1 | B04 | b3 | C08 | c1 | D01 | d1 | E01 |
| 2-1-m1E01-2-0325 | 715.248 | OEt | a1 | B04 | b1 | C08 | c2 | D02 | d1 | E01 |
| 2-1-m1E01-2-0326 | 715.262 | OEt | a1 | B07 | b2 | C04 | c1 | D01 | d1 | E01 |
| 2-1-mlE01-2-0327 | 715.268 | OEt | a1 | B05 | b3 | C15 | c1 | D01 | d1 | E01 |
| 2-1-m1E01-2-0328 | 715.298 | OEt | a1 | B03 | b1 | C09 | c1 | D01 | d1 | E01 |
| 2-1-m1E01-2-0329 | 715.312 | OEt | a1 | B06 | b1 | C13 | c1 | D01 | d1 | E01 |
| 2-1-m1E01-2-0330 | 716.223 | OEt | a2 | B17 | b1 | C10 | c2 | D02 | d1 | E01 |
| 2-1-mlE01-2-0331 | 716.226 | OEt | a1 | B02 | b2 | C01 | c2 | D02 | d1 | E01 |
| 2-1-mlE01-2-0332 | 716.228 | OEt | a2 | B19 | b2 | C06 | c1 | D01 | d1 | E01 |
| 2-1-mlE01-2-0333 | 716.243 | OEt | a1 | B04 | b1 | C11 | c2 | D02 | d1 | E01 |
| 2-1-mlE01-2-0334 | 716.246 | OEt | a2 | B20 | b2 | C01 | c1 | D01 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0335 | 716.311 | OEt | a2 | B18 | b1 | C13 | c1 | D01 | d1 | E01 |
| 2-1-mlE01-2-0336 | 717.229 | OEt | a1 | B03 | b3 | C06 | c1 | D01 | d1 | E01 |
| 2-1-mlE01-2-0337 | 717.238 | OEt | a1 | B04 | b1 | C10 | c2 | D02 | d1 | E01 |
| 2-1-mlE01-2-0338 | 717.247 | OEt | a1 | B06 | b3 | C15 | c1 | D01 | d1 | E01 |
| 2-1-mlE01-2-0339 | 717.277 | OEt | a1 | B10 | b1 | C08 | c1 | D01 | d1 | E01 |
| 2-1-m1E01-2-0340 | 717.298 | OEt | a1 | B05 | b1 | C14 | c1 | D01 | d1 | E01 |
| 2-1-mlE01-2-0341 | 717.320 | OEt | a1 | B05 | b2 | C33 | c3 | D02 | d1 | E01 |
| 2-1-mlE01-2-0342 | 718.224 | OEt | a1 | B03 | b1 | C12 | c2 | D02 | d1 | E01 |
| 2-1-mlE01-2-0343 | 718.246 | OEt | a2 | B18 | b3 | C15 | c1 | D01 | d1 | E01 |
| 2-1-mlE01-2-0344 | 718.257 | OEt | a1 | B08 | b2 | C02 | c1 | D01 | d1 | E01 |
| 2-1-m1E01-2-0345 | 718.258 | OEt | a2 | B17 | b1 | C09 | c1 | D01 | d1 | E01 |
| 2-1-mlE01-2-0346 | 718.273 | OEt | a1 | B10 | b1 | C11 | c1 | D01 | d1 | E01 |
| 2-1-m1E01-2-0347 | 718.319 | OEt | a2 | B15 | b1 | C30 | c3 | D02 | d1 | E01 |
| 2-1-mlE01-2-0348 | 718.319 | OEt | a2 | B11 | b1 | C29 | c3 | D02 | d1 | E01 |
| 2-1-m1E01-2-0349 | 718.319 | OEt | a2 | B13 | b1 | C32 | c3 | D02 | d1 | E01 |
| 2-1-mlE01-2-0350 | 719.253 | OEt | a1 | B08 | b2 | C03 | c1 | D01 | d1 | E01 |
| 2-1-m1E01-2-0351 | 719.257 | OEt | a1 | B09 | b2 | C01 | c1 | D01 | d1 | E01 |
| 2-1-m1E01-2-0352 | 719.268 | OEt | a1 | B10 | b1 | C10 | c1 | D01 | d1 | E01 |
| 2-1-mlE01-2-0353 | 719.273 | OEt | a1 | B04 | b1 | C09 | c1 | D01 | d1 | E01 |
| 2-1-m1E01-2-0354 | 719.278 | OEt | a1 | B06 | b1 | C14 | c1 | D01 | d1 | E01 |
| 2-1-mlE01-2-0355 | 719.299 | OEt | a1 | B06 | b2 | C33 | c3 | D02 | d1 | E01 |
| 2-1-m1E01-2-0356 | 719.314 | OEt | a2 | B12 | b1 | C29 | c3 | D02 | d1 | E01 |
| 2-1-m1E01-2-0357 | 720.189 | OEt | a2 | B17 | b3 | C06 | c1 | D01 | d1 | E01 |
| 2-1-mlE01-2-0358 | 720.262 | OEt | a2 | B13 | b2 | C02 | c2 | D02 | d1 | E01 |
| 2-1-m1E01-2-0359 | 720.277 | OEt | a2 | B18 | b1 | C14 | c1 | D01 | d1 | E01 |
| 2-1-mlE01-2-0360 | 720.298 | OEt | a2 | B15 | b2 | C20 | c3 | D02 | d1 | E01 |
| 2-1-m1E01-2-0361 | 720.298 | OEt | a2 | B13 | b2 | C22 | c3 | D02 | d1 | E01 |
| 2-1-mlE01-2-0362 | 720.298 | OEt | a2 | B16 | b1 | C30 | c3 | D02 | d1 | E01 |
| 2-1-m1E01-2-0363 | 720.298 | OEt | a2 | B18 | b2 | C33 | c3 | D02 | d1 | E01 |
| 2-1-mlE01-2-0364 | 721.184 | OEt | a2 | B17 | b1 | C12 | c2 | D02 | d1 | E01 |
| 2-1-m1E01-2-0365 | 721.204 | OEt | a1 | B04 | b3 | C06 | c1 | D01 | d1 | E01 |
| 2-1-m 1 E01-2-0366 | 721.257 | OEt | a2 | B13 | b2 | C03 | c2 | D02 | d1 | E01 |
| 2-1-mlE01-2-0367 | 721.257 | OEt | a2 | B11 | b2 | C05 | c2 | D02 | d1 | E01 |
| 2-1-mlE01-2-0368 | 721.282 | OEt | a1 | B07 | b1 | C13 | c1 | D01 | d1 | E01 |
| 2-1-mlE01-2-0369 | 721.293 | OEt | a2 | B15 | b2 | C28 | c3 | D02 | d1 | E01 |
| 2-1-m1E01-2-0370 | 721.384 | OEt | a1 | B05 | b2 | C07 | c1 | D01 | d1 | E01 |
| 2-1-mlE01-2-0371 | 722.199 | OEt | a1 | B04 | b1 | C12 | c2 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0372 | 722.252 | OEt | a2 | B12 | b2 | C05 | c2 | D02 | d1 | E01 |
| 2-1-m1E01-2-0373 | 722.277 | OEt | a2 | B16 | b2 | C20 | c3 | D02 | d1 | E01 |
| 2-1-mlE01-2-0374 | 722.277 | OEt | a2 | B11 | b2 | C26 | c3 | D02 | d1 | E01 |
| 2-1-m1E01-2-0375 | 722.294 | OEt | a2 | B14 | b1 | C32 | c3 | D02 | d1 | E01 |
| 2-1-mlE01-2-0376 | 723.217 | OEt | a1 | B07 | b3 | C15 | c1 | D01 | d1 | E01 |
| 2-1-m1E01-2-0377 | 723.273 | OEt | a2 | B12 | b2 | C26 | c3 | D02 | d1 | E01 |
| 2-1-m1E01-2-0378 | 723.273 | OEt | a2 | B16 | b2 | C28 | c3 | D02 | d1 | E01 |
| 2-1-mlE01-2-0379 | 723.309 | OEt | a1 | B03 | b1 | C30 | c3 | D02 | d1 | E01 |
| 2-1-mlE01-2-0380 | 723.309 | OEt | a1 | B01 | b1 | C32 | c3 | D02 | d1 | E01 |
| 2-1-mlE01-2-0381 | 723.363 | OEt | a1 | B06 | b2 | C07 | c1 | D01 | d1 | E01 |
| 2-1-m1E01-2-0382 | 724.214 | OEt | a1 | B08 | b2 | C06 | c1 | D01 | d1 | E01 |
| 2-1-mlE01-2-0383 | 724.229 | OEt | a1 | B10 | b1 | C12 | c1 | D01 | d1 | E01 |
| 2-1-m1E01-2-0384 | 724.237 | OEt | a2 | B14 | b2 | C02 | c2 | D02 | d1 | E01 |
| 2-1-mlE01-2-0385 | 724.237 | OEt | a2 | B11 | b2 | C04 | c2 | D02 | d1 | E01 |
| 2-1-mlE01-2-0386 | 724.257 | OEt | a2 | B15 | b2 | C01 | c2 | D02 | d1 | E01 |
| 2-1-m1E01-2-0387 | 724.273 | OEt | a2 | B14 | b2 | C22 | c3 | D02 | d1 | E01 |
| 2-1-mlE01-2-0388 | 724.273 | OEt | a2 | B13 | b2 | C24 | c3 | D02 | d1 | E01 |
| 2-1-mlE01-2-0389 | 724.304 | OEt | a1 | B02 | b1 | C32 | c3 | D02 | d1 | E01 |
| 2-1-mlE01-2-0390 | 724.362 | OEt | a2 | B18 | b2 | C07 | c1 | D01 | d1 | E01 |
| 2-1-mlE01-2-0391 | 725.232 | OEt | a2 | B12 | b2 | C04 | c2 | D02 | d1 | E01 |
| 2-1-m1E01-2-0392 | 725.232 | OEt | a2 | B14 | b2 | C03 | c2 | D02 | d1 | E01 |
| 2-1-mlE01-2-0393 | 725.248 | OEt | a1 | B07 | b1 | C14 | c1 | D01 | d1 | E01 |
| 2-1-m1E01-2-0394 | 725.252 | OEt | a1 | B01 | b2 | C02 | c2 | D02 | d1 | E01 |
| 2-1-mlE01-2-0395 | 725.269 | OEt | a1 | B07 | b2 | C33 | c3 | D02 | d1 | E01 |
| 2-1-m1E01-2-0396 | 725.283 | OEt | a2 | B19 | b2 | C05 | c1 | D01 | d1 | E01 |
| 2-1-mlE01-2-0397 | 725.288 | OEt | a1 | B05 | b3 | C08 | c1 | D01 | d1 | E01 |
| 2-1-mlE01-2-0398 | 725.288 | OEt | a1 | B05 | b1 | C08 | c2 | D02 | d1 | E01 |
| 2-1-m1E01-2-0399 | 725.288 | OEt | a1 | B03 | b2 | C20 | c3 | D02 | d1 | E01 |
| 2-1-mlE01-2-0400 | 725.288 | OEt | a1 | B01 | b2 | C22 | c3 | D02 | d1 | E01 |
| 2-1-m1E01-2-0401 | 726.218 | OEt | a2 | B13 | b2 | C06 | c2 | D02 | d1 | E01 |
| 2-1-m1E01-2-0402 | 726.236 | OEt | a2 | B16 | b2 | C01 | c2 | D02 | d1 | E01 |
| 2-1-m1E01-2-0403 | 726.247 | OEt | a1 | B02 | b2 | C02 | c2 | D02 | d1 | E01 |
| 2-1-m1E01-2-0404 | 726.247 | OEt | a1 | B01 | b2 | C03 | c2 | D02 | d1 | E01 |
| 2-1-mlE01-2-0405 | 726.267 | OEt | a2 | B20 | b2 | C02 | c1 | D01 | d1 | E01 |
| 2-1-m1E01-2-0406 | 726.269 | OEt | a2 | B17 | b1 | C30 | c3 | D02 | d1 | E01 |
| 2-1-m1E01-2-0407 | 726.284 | OEt | a1 | B05 | b1 | C11 | c2 | D02 | d1 | E01 |
| 2-1-m1E01-2-0408 | 726.284 | OEt | a1 | B02 | b2 | C22 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0409 | 726.284 | OEt | a1 | B03 | b2 | C28 | c3 | D02 | d1 | E01 |
| 2-1-m1E01-2-0410 | 727.242 | OEt | a1 | B02 | b2 | C03 | c2 | D02 | d1 | E01 |
| 2-1-mlE01-2-0411 | 727.262 | OEt | a2 | B20 | b2 | C03 | c1 | D01 | d1 | E01 |
| 2-1-mlE01-2-0412 | 727.268 | OEt | a1 | B06 | b3 | C08 | c1 | D01 | d1 | E01 |
| 2-1-mlE01-2-0413 | 727.268 | OEt | a1 | B06 | b1 | C08 | c2 | D02 | d1 | E01 |
| 2-1-mlE01-2-0414 | 727.279 | OEt | a1 | B05 | b1 | C10 | c2 | D02 | d1 | E01 |
| 2-1-mlE01-2-0415 | 727.284 | OEt | a1 | B04 | b1 | C30 | c3 | D02 | d1 | E01 |
| 2-1-mlE01-2-0416 | 728.248 | OEt | a2 | B17 | b2 | C20 | c3 | D02 | d1 | E01 |
| 2-1-m1E01-2-0417 | 728.248 | OEt | a2 | B14 | b2 | C24 | c3 | D02 | d1 | E01 |
| 2-1-mlE01-2-0418 | 728.262 | OEt | a2 | B19 | b2 | C04 | c1 | D01 | d1 | E01 |
| 2-1-m1E01-2-0419 | 728.263 | OEt | a1 | B06 | b1 | C11 | c2 | D02 | d1 | E01 |
| 2-1-m1E01-2-0420 | 728.267 | OEt | a2 | B18 | b3 | C08 | c1 | D01 | d1 | E01 |
| 2-1-m1E01-2-0421 | 728.267 | OEt | a2 | B18 | b1 | C08 | c2 | D02 | d1 | E01 |
| 2-1-m1E01-2-0422 | 729.243 | OEt | a2 | B17 | b2 | C28 | c3 | D02 | d1 | E01 |
| 2-1-m1E01-2-0423 | 729.247 | OEt | a1 | B03 | b2 | C01 | c2 | D02 | d1 | E01 |
| 2-1-m1E01-2-0424 | 729.258 | OEt | a1 | B06 | b1 | C10 | c2 | D02 | d1 | E01 |
| 2-1-m1E01-2-0425 | 729.262 | OEt | a2 | B18 | b1 | C11 | c2 | D02 | d1 | E01 |
| 2-1-m1E01-2-0426 | 729.263 | OEt | a1 | B04 | b2 | C20 | c3 | D02 | d1 | E01 |
| 2-1-m1E01-2-0427 | 729.263 | OEt | a1 | B01 | b2 | C24 | c3 | D02 | d1 | E01 |
| 2-1-mlE01-2-0428 | 729.277 | OEt | a1 | B09 | b2 | C02 | c1 | D01 | d1 | E01 |
| 2-1-m1E01-2-0429 | 729.314 | OEt | a1 | B05 | b1 | C09 | c1 | D01 | d1 | E01 |
| 2-1-mlE01-2-0430 | 729.334 | OEt | a1 | B07 | b2 | C07 | c1 | D01 | d1 | E01 |
| 2-1-m1E01-2-0431 | 730.193 | OEt | a2 | B14 | b2 | C06 | c2 | D02 | d1 | E01 |
| 2-1-mlE01-2-0432 | 730.257 | OEt | a2 | B18 | b1 | C10 | c2 | D02 | d1 | E01 |
| 2-1-mlE01-2-0433 | 730.257 | OEt | a2 | B11 | b1 | C13 | c2 | D02 | d1 | E01 |
| 2-1-mlE01-2-0434 | 730.258 | OEt | a1 | B02 | b2 | C24 | c3 | D02 | d1 | E01 |
| 2-1-mlE01-2-0435 | 730.258 | OEt | a1 | B04 | b2 | C28 | c3 | D02 | d1 | E01 |
| 2-1-mlE01-2-0436 | 730.273 | OEt | a1 | B09 | b2 | C03 | c1 | D01 | d1 | E01 |
| 2-1-mlE01-2-0437 | 731.208 | OEt | a1 | B01 | b2 | C06 | c2 | D02 | d1 | E01 |
| 2-1-mlE01-2-0438 | 731.245 | OEt | a1 | B05 | b3 | C06 | c1 | D01 | d1 | E01 |
| 2-1-mlE01-2-0439 | 731.253 | OEt | a2 | B12 | b1 | C13 | c2 | D02 | d1 | E01 |
| 2-1-mlE01-2-0440 | 731.293 | OEt | a1 | B06 | b1 | C09 | c1 | D01 | d1 | E01 |
| 2-1-m1E01-2-0441 | 732.192 | OEt | a2 | B11 | b3 | C15 | c2 | D02 | d1 | E01 |
| 2-1-mlE01-2-0442 | 732.204 | OEt | a1 | B02 | b2 | C06 | c2 | D02 | d1 | E01 |
| 2-1-mlE01-2-0443 | 732.207 | OEt | a2 | B17 | b2 | C01 | c2 | D02 | d1 | E01 |
| 2-1-mlE01-2-0444 | 732.223 | OEt | a2 | B20 | b2 | C06 | c1 | D01 | d1 | E01 |
| 2-1-mlE01-2-0445 | 732.240 | OEt | a1 | B05 | b1 | C12 | c2 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0446 | 732.262 | OEt | a2 | B11 | b3 | C16 | c1 | D01 | d1 | E01 |
| 2-1-mlE01-2-0447 | 732.292 | OEt | a2 | B18 | b1 | C09 | c1 | D01 | d1 | E01 |
| 2-1-m1E01-2-0448 | 732.335 | OEt | a2 | B13 | b1 | C29 | c3 | D02 | d1 | E01 |
| 2-1-mlE01-2-0449 | 732.335 | OEt | a2 | B15 | b1 | C32 | c3 | D02 | d1 | E01 |
| 2-1-mlE01-2-0450 | 733.188 | OEt | a2 | B12 | b3 | C15 | c2 | D02 | d1 | E01 |
| 2-1-m1E01-2-0451 | 733.222 | OEt | a1 | B04 | b2 | C01 | c2 | D02 | d1 | E01 |
| 2-1-mlE01-2-0452 | 733.224 | OEt | a1 | B06 | b3 | C06 | c1 | D01 | d1 | E01 |
| 2-1-mlE01-2-0453 | 733.238 | OEt | a1 | B07 | b3 | C08 | c1 | D01 | d1 | E01 |
| 2-1-mlE01-2-0454 | 733.238 | OEt | a1 | B07 | b1 | C08 | c2 | D02 | d1 | E01 |
| 2-1-mlE01-2-0455 | 733.257 | OEt | a2 | B12 | b3 | C16 | c1 | D01 | d1 | E01 |
| 2-1-mlE01-2-0456 | 733.268 | OEt | a1 | B08 | b2 | C05 | c1 | D01 | d1 | E01 |
| 2-1-mlE01-2-0457 | 734.219 | OEt | a1 | B06 | b1 | C12 | c2 | D02 | d1 | E01 |
| 2-1-mlE01-2-0458 | 734.223 | OEt | a2 | B18 | b3 | C06 | c1 | D01 | d1 | E01 |
| 2-1-mlE01-2-0459 | 734.223 | OEt | a2 | B11 | b3 | C17 | c1 | D01 | d1 | E01 |
| 2-1-mlE01-2-0460 | 734.223 | OEt | a2 | B11 | b1 | C14 | c2 | D02 | d1 | E01 |
| 2-1-m1E01-2-0461 | 734.233 | OEt | a1 | B07 | b1 | C11 | c2 | D02 | d1 | E01 |
| 2-1-mlE01-2-0462 | 734.277 | OEt | a2 | B15 | b2 | C02 | c2 | D02 | d1 | E01 |
| 2-1-mlE01-2-0463 | 734.283 | OEt | a2 | B19 | b1 | C13 | c1 | D01 | d1 | E01 |
| 2-1-mlE01-2-0464 | 734.314 | OEt | a2 | B15 | b2 | C22 | c3 | D02 | d1 | E01 |
| 2-1-mlE01-2-0465 | 734.314 | OEt | a2 | B16 | b1 | C32 | c3 | D02 | d1 | E01 |
| 2-1-mlE01-2-0466 | 735.219 | OEt | a2 | B12 | b3 | C17 | c1 | D01 | d1 | E01 |
| 2-1-mlE01-2-0467 | 735.219 | OEt | a2 | B18 | b1 | C12 | c2 | D02 | d1 | E01 |
| 2-1-mlE01-2-0468 | 735.219 | OEt | a2 | B12 | b1 | C14 | c2 | D02 | d1 | E01 |
| 2-1-mlE01-2-0469 | 735.229 | OEt | a1 | B07 | b1 | C10 | c2 | D02 | d1 | E01 |
| 2-1-mlE01-2-0470 | 735.234 | OEt | a1 | B09 | b2 | C06 | c1 | D01 | d1 | E01 |
| 2-1-mlE01-2-0471 | 735.252 | OEt | a1 | B10 | b2 | C01 | c1 | D01 | d1 | E01 |
| 2-1-mlE01-2-0472 | 735.273 | OEt | a2 | B15 | b2 | C03 | c2 | D02 | d1 | E01 |
| 2-1-m1E01-2-0473 | 735.273 | OEt | a2 | B13 | b2 | C05 | c2 | D02 | d1 | E01 |
| 2-1-mlE01-2-0474 | 736.218 | OEt | a2 | B19 | b3 | C15 | c1 | D01 | d1 | E01 |
| 2-1-mlE01-2-0475 | 736.248 | OEt | a1 | B08 | b2 | C04 | c1 | D01 | d1 | E01 |
| 2-1-mlE01-2-0476 | 736.257 | OEt | a2 | B16 | b2 | C02 | c2 | D02 | d1 | E01 |
| 2-1-mlE01-2-0477 | 736.293 | OEt | a2 | B16 | b2 | C22 | c3 | D02 | d1 | E01 |
| 2-1-mlE01-2-0478 | 736.293 | OEt | a2 | B13 | b2 | C26 | c3 | D02 | d1 | E01 |
| 2-1-mlE01-2-0479 | 736.309 | OEt | a2 | B14 | b1 | C29 | c3 | D02 | d1 | E01 |
| 2-1-mlE01-2-0480 | 737.252 | OEt | a2 | B16 | b2 | C03 | c2 | D02 | d1 | E01 |
| 2-1-mlE01-2-0481 | 737.264 | OEt | a1 | B07 | b1 | C09 | c1 | D01 | d1 | E01 |
| 2-1-mlE01-2-0482 | 737.325 | OEt | a1 | B05 | b1 | C30 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0483 | 737.325 | OEt | a1 | B01 | b1 | C29 | c3 | D02 | d1 | E01 |
| 2-1-m1E01-2-0484 | 737.325 | OEt | a1 | B03 | b1 | C32 | c3 | D02 | d1 | E01 |
| 2-1-mlE01-2-0485 | 738.249 | OEt | a2 | B19 | b1 | C14 | c1 | D01 | d1 | E01 |
| 2-1-mlE01-2-0486 | 738.252 | OEt | a2 | B13 | b2 | C04 | c2 | D02 | d1 | E01 |
| 2-1-mlE01-2-0487 | 738.270 | OEt | a2 | B19 | b2 | C33 | c3 | D02 | d1 | E01 |
| 2-1-mlE01-2-0488 | 738.289 | OEt | a2 | B15 | b2 | C24 | c3 | D02 | d1 | E01 |
| 2-1-mlE01-2-0489 | 738.309 | OEt | a2 | B11 | b2 | C07 | c2 | D02 | d1 | E01 |
| 2-1-m1E01-2-0490 | 738.320 | OEt | a1 | B02 | b1 | C29 | c3 | D02 | d1 | E01 |
| 2-1-m1E01-2-0491 | 739.195 | OEt | a1 | B07 | b3 | C06 | c1 | D01 | d1 | E01 |
| 2-1-mlE01-2-0492 | 739.248 | OEt | a2 | B14 | b2 | C05 | c2 | D02 | d1 | E01 |
| 2-1-mlE01-2-0493 | 739.268 | OEt | a1 | B03 | b2 | C02 | c2 | D02 | d1 | E01 |
| 2-1-mlE01-2-0494 | 739.304 | OEt | a2 | B12 | b2 | C07 | c2 | D02 | d1 | E01 |
| 2-1-m1E01-2-0495 | 739.304 | OEt | a1 | B05 | b2 | C20 | c3 | D02 | d1 | E01 |
| 2-1-mlE01-2-0496 | 739.304 | OEt | a1 | B03 | b2 | C22 | c3 | D02 | d1 | E01 |
| 2-1-mlE01-2-0497 | 739.304 | OEt | a1 | B06 | b1 | C30 | c3 | D02 | d1 | E01 |
| 2-1-mlE01-2-0498 | 740.190 | OEt | a1 | B07 | b1 | C12 | c2 | D02 | d1 | E01 |
| 2-1-mlE01-2-0499 | 740.234 | OEt | a2 | B15 | b2 | C06 | c2 | D02 | d1 | E01 |
| 2-1-m1E01-2-0500 | 740.263 | OEt | a1 | B03 | b2 | C03 | c2 | D02 | d1 | E01 |
| 2-1-mlE01-2-0501 | 740.263 | OEt | a1 | B01 | b2 | C05 | c2 | D02 | d1 | E01 |
| 2-1-m1E01-2-0502 | 740.268 | OEt | a2 | B16 | b2 | C24 | c3 | D02 | d1 | E01 |
| 2-1-mlE01-2-0503 | 740.268 | OEt | a2 | B14 | b2 | C26 | c3 | D02 | d1 | E01 |
| 2-1-mlE01-2-0504 | 740.284 | OEt | a2 | B17 | b1 | C32 | c3 | D02 | d1 | E01 |
| 2-1-mlE01-2-0505 | 740.299 | OEt | a1 | B05 | b2 | C28 | c3 | D02 | d1 | E01 |
| 2-1-m1E01-2-0506 | 740.303 | OEt | a2 | B18 | b1 | C30 | c3 | D02 | d1 | E01 |
| 2-1-mlE01-2-0507 | 741.258 | OEt | a1 | B02 | b2 | COS | c2 | D02 | d1 | E01 |
| 2-1-mlE01-2-0508 | 741.277 | OEt | a2 | B20 | b2 | C05 | c1 | D01 | d1 | E01 |
| 2-1-m1E01-2-0509 | 741.283 | OEt | a1 | B06 | b2 | C20 | c3 | D02 | d1 | E01 |
| 2-1-mlE01-2-0510 | 741.283 | OEt | a1 | B01 | b2 | C26 | c3 | D02 | d1 | E01 |
| 2-1-mlE01-2-0511 | 741.300 | OEt | a1 | B04 | b1 | C32 | c3 | D02 | d1 | E01 |
| 2-1-mlE01-2-0512 | 742.213 | OEt | a2 | B16 | b2 | C06 | c2 | D02 | d1 | E01 |
| 2-1-mlE01-2-0513 | 742.213 | OEt | a2 | B11 | b3 | C08 | c2 | D02 | d1 | E01 |
| 2-1-m1E01-2-0514 | 742.227 | OEt | a2 | B17 | b2 | C02 | c2 | D02 | d1 | E01 |
| 2-1-mlE01-2-0515 | 742.227 | OEt | a2 | B14 | b2 | C04 | c2 | D02 | d1 | E01 |
| 2-1-m1E01-2-0516 | 742.264 | OEt | a2 | B17 | b2 | C22 | c3 | D02 | d1 | E01 |
| 2-1-mlE01-2-0517 | 742.269 | OEt | a1 | B08 | b1 | C13 | c1 | D01 | d1 | E01 |
| 2-1-m1E01-2-0518 | 742.278 | OEt | a1 | B02 | b2 | C26 | c3 | D02 | d1 | E01 |
| 2-1-m1E01-2-0519 | 742.278 | OEt | a1 | B06 | b2 | C28 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mIE01-2-0520 | 742.283 | OEt | a2 | B18 | b2 | C20 | c3 | D02 | d1 | E01 |
| 2-1-mIE01-2-0521 | 742.334 | OEt | a2 | B19 | b2 | C07 | c1 | D01 | d1 | E01 |
| 2-1-mIE01-2-0522 | 743.208 | OEt | a2 | B12 | b3 | C08 | c2 | D02 | d1 | E01 |
| 2-1-mIE01-2-0523 | 743.223 | OEt | a2 | B17 | b2 | C03 | c2 | D02 | d1 | E01 |
| 2-1-mIE01-2-0524 | 743.243 | OEt | a1 | B04 | b2 | C02 | c2 | D02 | d1 | E01 |
| 2-1-m1E01-2-0525 | 743.243 | OEt | a1 | B01 | b2 | C04 | c2 | D02 | d1 | E01 |
| 2-1-m1E01-2-0526 | 743.262 | OEt | a1 | B05 | b2 | C01 | c2 | D02 | d1 | E01 |
| 2-1-mIE01-2-0527 | 743.278 | OEt | a2 | B18 | b2 | C28 | c3 | D02 | d1 | E01 |
| 2-1-mIE01-2-0528 | 743.279 | OEt | a1 | B04 | b2 | C22 | c3 | D02 | d1 | E01 |
| 2-1-m1E01-2-0529 | 743.279 | OEt | a1 | B03 | b2 | C24 | c3 | D02 | d1 | E01 |
| 2-1-mIE01-2-0530 | 744.204 | OEt | a1 | B08 | b3 | C15 | c1 | D01 | d1 | E01 |
| 2-1-mIE01-2-0531 | 744.238 | OEt | a1 | B02 | b2 | C04 | c2 | D02 | d1 | E01 |
| 2-1-mIE01-2-0532 | 744.238 | OEt | a1 | B04 | b2 | C03 | c2 | D02 | d1 | E01 |
| 2-1-mIE01-2-0533 | 744.257 | OEt | a2 | B20 | b2 | C04 | c1 | D01 | d1 | E01 |
| 2-1-mIE01-2-0534 | 744.273 | OEt | a2 | B13 | b1 | C13 | c2 | D02 | d1 | E01 |
| 2-1-mIE01-2-0535 | 744.288 | OEt | a1 | B09 | b2 | COS | c1 | D01 | d1 | E01 |
| 2-1-mIE01-2-0536 | 745.224 | OEt | a1 | B03 | b2 | C06 | c2 | D02 | d1 | E01 |
| 2-1-mIE01-2-0537 | 745.242 | OEt | a1 | B06 | b2 | C01 | c2 | D02 | d1 | E01 |
| 2-1-mIE01-2-0538 | 745.272 | OEt | a1 | B10 | b2 | C02 | c1 | D01 | d1 | E01 |
| 2-1-mIE01-2-0539 | 745.275 | OEt | a1 | B07 | b1 | C30 | c3 | D02 | d1 | E01 |
| 2-1-mIE01-2-0540 | 746.208 | OEt | a2 | B13 | b3 | C15 | c2 | D02 | d1 | E01 |
| 2-1-mIE01-2-0541 | 746.235 | OEt | a1 | B08 | b1 | C14 | c1 | D01 | d1 | E01 |
| 2-1-mIE01-2-0542 | 746.239 | OEt | a2 | B19 | b3 | C08 | c1 | D01 | d1 | E01 |
| 2-1-mIE01-2-0543 | 746.239 | OEt | a2 | B19 | b1 | C08 | c2 | D02 | d1 | E01 |
| 2-1-mIE01-2-0544 | 746.239 | OEt | a2 | B11 | b1 | C09 | c2 | D02 | d1 | E01 |
| 2-1-mIE01-2-0545 | 746.239 | OEt | a2 | B17 | b2 | C24 | c3 | D02 | d1 | E01 |
| 2-1-mIE01-2-0546 | 746.241 | OEt | a2 | B18 | b2 | C01 | c2 | D02 | d1 | E01 |
| 2-1-mIE01-2-0547 | 746.256 | OEt | a1 | B08 | b2 | C33 | c3 | D02 | d1 | E01 |
| 2-1-mIE01-2-0548 | 746.268 | OEt | a1 | B10 | b2 | C03 | c1 | D01 | d1 | E01 |
| 2-1-mIE01-2-0549 | 746.277 | OEt | a2 | B13 | b3 | C16 | c1 | D01 | d1 | E01 |
| 2-1-mIE01-2-0550 | 746.314 | OEt | a2 | B11 | b1 | C31 | c3 | D02 | d1 | E01 |
| 2-1-mIE01-2-0551 | 746.350 | OEt | a2 | B15 | b1 | C29 | c3 | D02 | d1 | E01 |
| 2-1-mIE01-2-0552 | 747.234 | OEt | a2 | B12 | b1 | C09 | c2 | D02 | d1 | E01 |
| 2-1-mIE01-2-0553 | 747.234 | OEt | a2 | B19 | b1 | C11 | c2 | D02 | d1 | E01 |
| 2-1-mIE01-2-0554 | 747.254 | OEt | a1 | B07 | b2 | C20 | c3 | D02 | d1 | E01 |
| 2-1-mIE01-2-0555 | 747.254 | OEt | a1 | B04 | b2 | C24 | c3 | D02 | d1 | E01 |
| 2-1-mIE01-2-0556 | 747.268 | OEt | a1 | B09 | b2 | C04 | c1 | D01 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0557 | 747.309 | OEt | a2 | B12 | b1 | C31 | c3 | D02 | d1 | E01 |
| 2-1-mlE01-2-0558 | 748.170 | OEt | a2 | B11 | b3 | C06 | c2 | D02 | d1 | E01 |
| 2-1-mlE01-2-0559 | 748.184 | OEt | a2 | B17 | b2 | C06 | c2 | D02 | d1 | E01 |
| 2-1-mlE01-2-0560 | 748.229 | OEt | a2 | B19 | b1 | C10 | c2 | D02 | d1 | E01 |
| 2-1-mlE01-2-0561 | 748.239 | OEt | a2 | B13 | b3 | C17 | c1 | D01 | d1 | E01 |
| 2-1-mlE01-2-0562 | 748.239 | OEt | a2 | B13 | b1 | C14 | c2 | D02 | d1 | E01 |
| 2-1-mlE01-2-0563 | 748.248 | OEt | a2 | B14 | b1 | C13 | c2 | D02 | d1 | E01 |
| 2-1-mlE01-2-0564 | 748.249 | OEt | a1 | B07 | b2 | C28 | c3 | D02 | d1 | E01 |
| 2-1-mlE01-2-0565 | 748.329 | OEt | a2 | B16 | b1 | C29 | c3 | D02 | d1 | E01 |
| 2-1-mlE01-2-0566 | 749.165 | OEt | a2 | B12 | b3 | C06 | c2 | D02 | d1 | E01 |
| 2-1-mlE01-2-0567 | 749.199 | OEt | a1 | B04 | b2 | C06 | c2 | D02 | d1 | E01 |
| 2-1-mlE01-2-0568 | 749.234 | OEt | a2 | B11 | b3 | C18 | c1 | D01 | d1 | E01 |
| 2-1-mlE01-2-0569 | 749.263 | OEt | a1 | B01 | b1 | C13 | c2 | D02 | d1 | E01 |
| 2-1-mlE01-2-0570 | 749.288 | OEt | a2 | B15 | b2 | C05 | c2 | D02 | d1 | E01 |
| 2-1-mlE01-2-0571 | 750.183 | OEt | a2 | B14 | b3 | C15 | c2 | D02 | d1 | E01 |
| 2-1-m1E01-2-0572 | 750.229 | OEt | a2 | B12 | b3 | C18 | c1 | D01 | d1 | E01 |
| 2-1-m1E01-2-0573 | 750.252 | OEt | a2 | B14 | b3 | C16 | c1 | D01 | d1 | E01 |
| 2-1-mlE01-2-0574 | 750.258 | OEt | a1 | B02 | b1 | C13 | c2 | D02 | d1 | E01 |
| 2-1-mlE01-2-0575 | 750.264 | OEt | a2 | B19 | b1 | C09 | c1 | D01 | d1 | E01 |
| 2-1-mlE01-2-0576 | 750.278 | OEt | a2 | B20 | b1 | C13 | c1 | D01 | d1 | E01 |
| 2-1-mlE01-2-0577 | 750.309 | OEt | a2 | B15 | b2 | C26 | c3 | D02 | d1 | E01 |
| 2-1-mlE01-2-0578 | 750.320 | OEt | a1 | B08 | b2 | C07 | c1 | D01 | d1 | E01 |
| 2-1-mlE01-2-0579 | 751.198 | OEt | a1 | B01 | b3 | C15 | c2 | D02 | d1 | E01 |
| 2-1-mlE01-2-0580 | 751.212 | OEt | a1 | B07 | b2 | C01 | c2 | D02 | d1 | E01 |
| 2-1-mlE01-2-0581 | 751.229 | OEt | a1 | B10 | b2 | C06 | c1 | D01 | d1 | E01 |
| 2-1-mlE01-2-0582 | 751.268 | OEt | a1 | B01 | b3 | C16 | c1 | D01 | d1 | E01 |
| 2-1-mlE01-2-0583 | 751.268 | OEt | a2 | B16 | b2 | C05 | c2 | D02 | d1 | E01 |
| 2-1-mlE01-2-0584 | 751.340 | OEt | a1 | B03 | b1 | C29 | c3 | D02 | d1 | E01 |
| 2-1-mlE01-2-0585 | 751.340 | OEt | a1 | B05 | b1 | C32 | c3 | D02 | d1 | E01 |
| 2-1-mlE01-2-0586 | 752.193 | OEt | a1 | B02 | b3 | C15 | c2 | D02 | d1 | E01 |
| 2-1-mlE01-2-0587 | 752.195 | OEt | a2 | B19 | b3 | C06 | c1 | D01 | d1 | E01 |
| 2-1-mlE01-2-0588 | 752.213 | OEt | a2 | B20 | b3 | C15 | c1 | D01 | d1 | E01 |
| 2-1-mlE01-2-0589 | 752.214 | OEt | a2 | B14 | b3 | C17 | c1 | D01 | d1 | E01 |
| 2-1-mlE01-2-0590 | 752.214 | OEt | a2 | B14 | b1 | C14 | c2 | D02 | d1 | E01 |
| 2-1-mlE01-2-0591 | 752.263 | OEt | a1 | B02 | b3 | C16 | c1 | D01 | d1 | E01 |
| 2-1-mlE01-2-0592 | 752.268 | OEt | a2 | B15 | b2 | C04 | c2 | D02 | d1 | E01 |
| 2-1-mlE01-2-0593 | 752.288 | OEt | a2 | B16 | b2 | C26 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0594 | 752.324 | OEt | a2 | B13 | b2 | C07 | c2 | D02 | d1 | E01 |
| 2-1-mlE01-2-0595 | 753.190 | OEt | a2 | B19 | b1 | C12 | c2 | D02 | d1 | E01 |
| 2-1-mlE01-2-0596 | 753.229 | OEt | a1 | B01 | b3 | C17 | c1 | D01 | d1 | E01 |
| 2-1-mlE01-2-0597 | 753.229 | OEt | a1 | B01 | b1 | C14 | c2 | D02 | d1 | E01 |
| 2-1-mlE01-2-0598 | 753.283 | OEt | a1 | B05 | b2 | C02 | c2 | D02 | d1 | E01 |
| 2-1-mlE01-2-0599 | 753.289 | OEt | a1 | B09 | b1 | C13 | c1 | D01 | d1 | E01 |
| 2-1-m1E01-2-0600 | 753.320 | OEt | a1 | B05 | b2 | C22 | c3 | D02 | d1 | E01 |
| 2-1-m1E01-2-0601 | 753.320 | OEt | a1 | B06 | b1 | C32 | c3 | D02 | d1 | E01 |
| 2-1-m1E01-2-0602 | 754.224 | OEt | a1 | B02 | b3 | C17 | c1 | D01 | d1 | E01 |
| 2-1-m1E01-2-0603 | 754.224 | OEt | a1 | B08 | b3 | C08 | c1 | D01 | d1 | E01 |
| 2-1-m1E01-2-0604 | 754.224 | OEt | a1 | B08 | b1 | C08 | c2 | D02 | d1 | E01 |
| 2-1-m1E01-2-0605 | 754.224 | OEt | a1 | B02 | b1 | C14 | c2 | D02 | d1 | E01 |
| 2-1-m1E01-2-0606 | 754.244 | OEt | a2 | B20 | b1 | C14 | c1 | D01 | d1 | E01 |
| 2-1-m1E01-2-0607 | 754.247 | OEt | a2 | B16 | b2 | C04 | c2 | D02 | d1 | E01 |
| 2-1-m1E01-2-0608 | 754.265 | OEt | a2 | B20 | b2 | C33 | c3 | D02 | d1 | E01 |
| 2-1-m1E01-2-0609 | 754.278 | OEt | a1 | B05 | b2 | C03 | c2 | D02 | d1 | E01 |
| 2-1-mlE01-2-0610 | 754.278 | OEt | a1 | B03 | b2 | C05 | c2 | D02 | d1 | E01 |
| 2-1-mlE01-2-0611 | 754.300 | OEt | a2 | B17 | b1 | C29 | c3 | D02 | d1 | E01 |
| 2-1-mlE01-2-0612 | 754.319 | OEt | a2 | B18 | b1 | C32 | c3 | D02 | d1 | E01 |
| 2-1-mlE01-2-0613 | 755.220 | OEt | a1 | B08 | b1 | C11 | c2 | D02 | d1 | E01 |
| 2-1-mlE01-2-0614 | 755.224 | OEt | a1 | B09 | b3 | C15 | c1 | D01 | d1 | E01 |
| 2-1-mlE01-2-0615 | 755.262 | OEt | a1 | B06 | b2 | C02 | c2 | D02 | d1 | E01 |
| 2-1-mlE01-2-0616 | 755.299 | OEt | a1 | B06 | b2 | C22 | c3 | D02 | d1 | E01 |
| 2-1-mlE01-2-0617 | 755.299 | OEt | a1 | B03 | b2 | C26 | c3 | D02 | d1 | E01 |
| 2-1-mlE01-2-0618 | 755.315 | OEt | a1 | B04 | b1 | C29 | c3 | D02 | d1 | E01 |
| 2-1-mlE01-2-0619 | 756.215 | OEt | a1 | B08 | b1 | C10 | c2 | D02 | d1 | E01 |
| 2-1-mlE01-2-0620 | 756.229 | OEt | a2 | B13 | b3 | C08 | c2 | D02 | d1 | E01 |
| 2-1-m1E01-2-0621 | 756.258 | OEt | a1 | B06 | b2 | C03 | c2 | D02 | d1 | E01 |
| 2-1-mlE01-2-0622 | 756.262 | OEt | a2 | B18 | b2 | C02 | c2 | D02 | d1 | E01 |
| 2-1-m1E01-2-0623 | 756.298 | OEt | a2 | B18 | b2 | C22 | c3 | D02 | d1 | E01 |
| 2-1-m1E01-2-0624 | 756.299 | OEt | a2 | B14 | b2 | C07 | c2 | D02 | d1 | E01 |
| 2-1-m1E01-2-0625 | 757.238 | OEt | a2 | B17 | b2 | C05 | c2 | D02 | d1 | E01 |
| 2-1-m1E01-2-0626 | 757.255 | OEt | a1 | B09 | b1 | C14 | c1 | D01 | d1 | E01 |
| 2-1-m1E01-2-0627 | 757.257 | OEt | a2 | B18 | b2 | C03 | c2 | D02 | d1 | E01 |
| 2-1-mlE01-2-0628 | 757.258 | OEt | a1 | B03 | b2 | C04 | c2 | D02 | d1 | E01 |
| 2-1-m1E01-2-0629 | 757.276 | OEt | a1 | B09 | b2 | C33 | c3 | D02 | d1 | E01 |
| 2-1-m1E01-2-0630 | 757.295 | OEt | a1 | B05 | b2 | C24 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0631 | 757.315 | OEt | a1 | B01 | b2 | C07 | c2 | D02 | d1 | E01 |
| 2-1-mlE01-2-0632 | 758.250 | OEt | a1 | B08 | b1 | C09 | c1 | D01 | d1 | E01 |
| 2-1-m1E01-2-0633 | 758.253 | OEt | a1 | B04 | b2 | C05 | c2 | D02 | d1 | E01 |
| 2-1-mlE01-2-0634 | 758.259 | OEt | a2 | B17 | b2 | C26 | c3 | D02 | d1 | E01 |
| 2-1-m1E01-2-0635 | 758.275 | OEt | a2 | B19 | b1 | C30 | c3 | D02 | d1 | E01 |
| 2-1-mlE01-2-0636 | 758.289 | OEt | a2 | B15 | b1 | C13 | c2 | D02 | d1 | E01 |
| 2-1-mlE01-2-0637 | 758.310 | OEt | a1 | B02 | b2 | C07 | c2 | D02 | d1 | E01 |
| 2-1-mlE01-2-0638 | 758.329 | OEt | a2 | B20 | b2 | C07 | c1 | D01 | d1 | E01 |
| 2-1-mlE01-2-0639 | 759.240 | OEt | a1 | B05 | b2 | C06 | c2 | D02 | d1 | E01 |
| 2-1-m1E01-2-0640 | 759.274 | OEt | a1 | B06 | b2 | C24 | c3 | D02 | d1 | E01 |
| 2-1-m1E01-2-0641 | 759.274 | OEt | a1 | B04 | b2 | C26 | c3 | D02 | d1 | E01 |
| 2-1-m1E01-2-0642 | 759.290 | OEt | a1 | B07 | b1 | C32 | c3 | D02 | d1 | E01 |
| 2-1-m1E01-2-0643 | 760.181 | OEt | a1 | B08 | b3 | C06 | c1 | D01 | d1 | E01 |
| 2-1-mlE01-2-0644 | 760.204 | OEt | a2 | B14 | b3 | C08 | c2 | D02 | d1 | E01 |
| 2-1-mlE01-2-0645 | 760.218 | OEt | a2 | B17 | b2 | C04 | c2 | D02 | d1 | E01 |
| 2-1-mlE01-2-0646 | 760.224 | OEt | a2 | B15 | b3 | C15 | c2 | D02 | d1 | E01 |
| 2-1-mlE01-2-0647 | 760.254 | OEt | a2 | B13 | b1 | C09 | c2 | D02 | d1 | E01 |
| 2-1-mlE01-2-0648 | 760.254 | OEt | a2 | B19 | b2 | C20 | c3 | D02 | d1 | E01 |
| 2-1-mlE01-2-0649 | 760.268 | OEt | a2 | B16 | b1 | C13 | c2 | D02 | d1 | E01 |
| 2-1-mlE01-2-0650 | 760.273 | OEt | a2 | B18 | b2 | C24 | c3 | D02 | d1 | E01 |
| 2-1-m1E01-2-0651 | 760.283 | OEt | a1 | B10 | b2 | C05 | c1 | D01 | d1 | E01 |
| 2-1-mlE01-2-0652 | 760.293 | OEt | a2 | B15 | b3 | C16 | c1 | D01 | d1 | E01 |
| 2-1-mlE01-2-0653 | 760.329 | OEt | a2 | B13 | b1 | C31 | c3 | D02 | d1 | E01 |
| 2-1-mlE01-2-0654 | 761.176 | OEt | a1 | B08 | b1 | C12 | c2 | D02 | d1 | E01 |
| 2-1-mlE01-2-0655 | 761.219 | OEt | a1 | B06 | b2 | C06 | c2 | D02 | d1 | E01 |
| 2-1-mlE01-2-0656 | 761.219 | OEt | a1 | B01 | b3 | C08 | c2 | D02 | d1 | E01 |
| 2-1-mlE01-2-0657 | 761.233 | OEt | a1 | B07 | b2 | C02 | c2 | D02 | d1 | E01 |
| 2-1-mlE01-2-0658 | 761.233 | OEt | a1 | B04 | b2 | C04 | c2 | D02 | d1 | E01 |
| 2-1-mlE01-2-0659 | 761.249 | OEt | a2 | B19 | b2 | C28 | c3 | D02 | d1 | E01 |
| 2-1-mlE01-2-0660 | 761.269 | OEt | a1 | B07 | b2 | C22 | c3 | D02 | d1 | E01 |
| 2-1-m1E01-2-0661 | 761.340 | OEt | a1 | B09 | b2 | C07 | c1 | D01 | d1 | E01 |
| 2-1-mlE01-2-0662 | 762.185 | OEt | a2 | B13 | b3 | C06 | c2 | D02 | d1 | E01 |
| 2-1-mlE01-2-0663 | 762.203 | OEt | a2 | B16 | b3 | C15 | c2 | D02 | d1 | E01 |
| 2-1-mlE01-2-0664 | 762.214 | OEt | a1 | B02 | b3 | C08 | c2 | D02 | d1 | E01 |
| 2-1-mlE01-2-0665 | 762.218 | OEt | a2 | B18 | b2 | C06 | c2 | D02 | d1 | E01 |
| 2-1-m1E01-2-0666 | 762.228 | OEt | a1 | B07 | b2 | C03 | c2 | D02 | d1 | E01 |
| 2-1-m1E01-2-0667 | 762.234 | OEt | a2 | B20 | b3 | C08 | c1 | D01 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0668 | 762.234 | OEt | a2 | B20 | b1 | C08 | c2 | D02 | d1 | E01 |
| 2-1-m1E01-2-0669 | 762.255 | OEt | a2 | B15 | b3 | C17 | c1 | D01 | d1 | E01 |
| 2-1-mlE01-2-0670 | 762.255 | OEt | a2 | B15 | b1 | C14 | c2 | D02 | d1 | E01 |
| 2-1-m1E01-2-0671 | 762.272 | OEt | a2 | B16 | b3 | C16 | c1 | D01 | d1 | E01 |
| 2-1-mlE01-2-0672 | 763.229 | OEt | a2 | B20 | b1 | C11 | c2 | D02 | d1 | E01 |
| 2-1-mlE01-2-0673 | 763.250 | OEt | a2 | B13 | b3 | C18 | c1 | D01 | d1 | E01 |
| 2-1-mlE01-2-0674 | 763.263 | OEt | a1 | B10 | b2 | C04 | c1 | D01 | d1 | E01 |
| 2-1-mlE01-2-0675 | 763.279 | OEt | a1 | B03 | b1 | C13 | c2 | D02 | d1 | E01 |
| 2-1-mlE01-2-0676 | 764.213 | OEt | a2 | B19 | b2 | C01 | c2 | D02 | d1 | E01 |
| 2-1-mlE01-2-0677 | 764.224 | OEt | a2 | B20 | b1 | C10 | c2 | D02 | d1 | E01 |
| 2-1-mlE01-2-0678 | 764.229 | OEt | a2 | B14 | b1 | C09 | c2 | D02 | d1 | E01 |
| 2-1-mlE01-2-0679 | 764.234 | OEt | a2 | B16 | b3 | C17 | c1 | D01 | d1 | E01 |
| 2-1-mlE01-2-0680 | 764.234 | OEt | a2 | B16 | b1 | C14 | c2 | D02 | d1 | E01 |
| 2-1-mlE01-2-0681 | 764.304 | OEt | a2 | B14 | b1 | C31 | c3 | D02 | d1 | E01 |
| 2-1-mlE01-2-0682 | 765.214 | OEt | a1 | B03 | b3 | C15 | c2 | D02 | d1 | E01 |
| 2-1-mlE01-2-0683 | 765.244 | OEt | a1 | B09 | b3 | C08 | c1 | D01 | d1 | E01 |
| 2-1-mlE01-2-0684 | 765.244 | OEt | a1 | B09 | b1 | C08 | c2 | D02 | d1 | E01 |
| 2-1-mlE01-2-0685 | 765.244 | OEt | a1 | B01 | b1 | C09 | c2 | D02 | d1 | E01 |
| 2-1-mlE01-2-0686 | 765.244 | OEt | a1 | B07 | b2 | C24 | c3 | D02 | d1 | E01 |
| 2-1-mlE01-2-0687 | 765.283 | OEt | a1 | B03 | b3 | C16 | c1 | D01 | d1 | E01 |
| 2-1-mlE01-2-0688 | 765.320 | OEt | a1 | B01 | b1 | C31 | c3 | D02 | d1 | E01 |
| 2-1-mlE01-2-0689 | 765.356 | OEt | a1 | B05 | b1 | C29 | c3 | D02 | d1 | E01 |
| 2-1-mlE01-2-0690 | 766.160 | OEt | a2 | B14 | b3 | C06 | c2 | D02 | d1 | E01 |
| 2-1-m1E01-2-0691 | 766.239 | OEt | a2 | B17 | b1 | C13 | c2 | D02 | d1 | E01 |
| 2-1-mlE01-2-0692 | 766.240 | OEt | a1 | B02 | b1 | C09 | c2 | D02 | d1 | E01 |
| 2-1-mlE01-2-0693 | 766.240 | OEt | a1 | B09 | b1 | C11 | c2 | D02 | d1 | E01 |
| 2-1-mlE01-2-0694 | 766.259 | OEt | a2 | B20 | b1 | C09 | c1 | D01 | d1 | E01 |
| 2-1-mlE01-2-0695 | 766.261 | OEt | a1 | B08 | b1 | C30 | c3 | D02 | d1 | E01 |
| 2-1-mlE01-2-0696 | 766.315 | OEt | a1 | B02 | b1 | C31 | c3 | D02 | d1 | E01 |
| 2-1-mlE01-2-0697 | 766.340 | OEt | a2 | B15 | b2 | C07 | c2 | D02 | d1 | E01 |
| 2-1-mlE01-2-0698 | 767.175 | OEt | a1 | B01 | b3 | C06 | c2 | D02 | d1 | E01 |
| 2-1-mlE01-2-0699 | 767.190 | OEt | a1 | B07 | b2 | C06 | c2 | D02 | d1 | E01 |
| 2-1-mlE01-2-0700 | 767.225 | OEt | a2 | B14 | b3 | C18 | c1 | D01 | d1 | E01 |
| 2-1-m1E01-2-0701 | 767.235 | OEt | a1 | B09 | b1 | C10 | c2 | D02 | d1 | E01 |
| 2-1-m1E01-2-0702 | 767.245 | OEt | a1 | B03 | b3 | C17 | c1 | D01 | d1 | E01 |
| 2-1-m1E01-2-0703 | 767.245 | OEt | a1 | B03 | b1 | C14 | c2 | D02 | d1 | E01 |
| 2-1-m1E01-2-0704 | 767.254 | OEt | a1 | B04 | b1 | C13 | c2 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0705 | 767.335 | OEt | a1 | B06 | b1 | C29 | c3 | D02 | d1 | E01 |
| 2-1-m1E01-2-0706 | 768.171 | OEt | a1 | B02 | b3 | C06 | c2 | D02 | d1 | E01 |
| 2-1-m1E01-2-0707 | 768.174 | OEt | a2 | B17 | b3 | C15 | c2 | D02 | d1 | E01 |
| 2-1-m1E01-2-0708 | 768.190 | OEt | a2 | B20 | b3 | C06 | c1 | D01 | d1 | E01 |
| 2-1-mlE01-2-0709 | 768.240 | OEt | a1 | B01 | b3 | C18 | c1 | D01 | d1 | E01 |
| 2-1-mlE01-2-0710 | 768.240 | OEt | a1 | B08 | b2 | C20 | c3 | D02 | d1 | E01 |
| 2-1-mlE01-2-0711 | 768.243 | OEt | a2 | B17 | b3 | C16 | c1 | D01 | d1 | E01 |
| 2-1-mlE01-2-0712 | 768.294 | OEt | a1 | B05 | b2 | C05 | c2 | D02 | d1 | E01 |
| 2-1-mlE01-2-0713 | 768.319 | OEt | a2 | B16 | b2 | C07 | c2 | D02 | d1 | E01 |
| 2-1-mlE01-2-0714 | 768.335 | OEt | a2 | B18 | b1 | C29 | c3 | D02 | d1 | E01 |
| 2-1-mlE01-2-0715 | 769.185 | OEt | a2 | B20 | b1 | C12 | c2 | D02 | d1 | E01 |
| 2-1-mlE01-2-0716 | 769.189 | OEt | a1 | B04 | b3 | C15 | c2 | D02 | d1 | E01 |
| 2-1-mlE01-2-0717 | 769.235 | OEt | a1 | B02 | b3 | C18 | c1 | D01 | d1 | E01 |
| 2-1-mlE01-2-0718 | 769.235 | OEt | a1 | B08 | b2 | C28 | c3 | D02 | d1 | E01 |
| 2-1-mlE01-2-0719 | 769.258 | OEt | a1 | B04 | b3 | C16 | c1 | D01 | d1 | E01 |
| 2-1-mlE01-2-0720 | 769.270 | OEt | a1 | B09 | b1 | C09 | c1 | D01 | d1 | E01 |
| 2-1-m1E01-2-0721 | 769.284 | OEt | a1 | B10 | b1 | C13 | c1 | D01 | d1 | E01 |
| 2-1-mlE01-2-0722 | 769.315 | OEt | a1 | B05 | b2 | C26 | c3 | D02 | d1 | E01 |
| 2-1-mlE01-2-0723 | 770.204 | OEt | a2 | B17 | b3 | C17 | c1 | D01 | d1 | E01 |
| 2-1-mlE01-2-0724 | 770.204 | OEt | a2 | B17 | b1 | C14 | c2 | D02 | d1 | E01 |
| 2-1-mlE01-2-0725 | 770.244 | OEt | a2 | B15 | b3 | C08 | c2 | D02 | d1 | E01 |
| 2-1-mlE01-2-0726 | 770.273 | OEt | a1 | B06 | b2 | C05 | c2 | D02 | d1 | E01 |
| 2-1-mlE01-2-0727 | 771.201 | OEt | a1 | B09 | b3 | C06 | c1 | D01 | d1 | E01 |
| 2-1-mlE01-2-0728 | 771.219 | OEt | a1 | B10 | b3 | C15 | c1 | D01 | d1 | E01 |
| 2-1-mlE01-2-0729 | 771.220 | OEt | a1 | B04 | b3 | C17 | c1 | D01 | d1 | E01 |
| 2-1-mlE01-2-0730 | 771.220 | OEt | a1 | B04 | b1 | C14 | c2 | D02 | d1 | E01 |
| 2-1-m1E01-2-0731 | 771.273 | OEt | a2 | B18 | b2 | C05 | c2 | D02 | d1 | E01 |
| 2-1-mlE01-2-0732 | 771.274 | OEt | a1 | B05 | b2 | C04 | c2 | D02 | d1 | E01 |
| 2-1-mlE01-2-0733 | 771.294 | OEt | a1 | B06 | b2 | C26 | c3 | D02 | d1 | E01 |
| 2-1-mlE01-2-0734 | 771.330 | OEt | a1 | B03 | b2 | C07 | c2 | D02 | d1 | E01 |
| 2-1-mlE01-2-0735 | 772.196 | OEt | a1 | B09 | b1 | C12 | c2 | D02 | d1 | E01 |
| 2-1-mlE01-2-0736 | 772.199 | OEt | a1 | B08 | b2 | C01 | c2 | D02 | d1 | E01 |
| 2-1-mlE01-2-0737 | 772.224 | OEt | a2 | B16 | b3 | C08 | c2 | D02 | d1 | E01 |
| 2-1-mlE01-2-0738 | 772.291 | OEt | a2 | B19 | b1 | C32 | c3 | D02 | d1 | E01 |
| 2-1-mlE01-2-0739 | 772.293 | OEt | a2 | B18 | b2 | C26 | c3 | D02 | d1 | E01 |
| 2-1-mlE01-2-0740 | 773.249 | OEt | a1 | B10 | b1 | C14 | c1 | D01 | d1 | E01 |
| 2-1-mlE01-2-0741 | 773.253 | OEt | a1 | B06 | b2 | C04 | c2 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0742 | 773.271 | OEt | a1 | B10 | b2 | C33 | c3 | D02 | d1 | E01 |
| 2-1-mlE01-2-0743 | 773.306 | OEt | a1 | B07 | b1 | C29 | c3 | D02 | d1 | E01 |
| 2-1-mlE01-2-0744 | 774.234 | OEt | a2 | B19 | b2 | C02 | c2 | D02 | d1 | E01 |
| 2-1-mlE01-2-0745 | 774.252 | OEt | a2 | B18 | b2 | C04 | c2 | D02 | d1 | E01 |
| 2-1-mlE01-2-0746 | 774.270 | OEt | a2 | B15 | b1 | C09 | c2 | D02 | d1 | E01 |
| 2-1-mlE01-2-0747 | 774.270 | OEt | a2 | B19 | b2 | C22 | c3 | D02 | d1 | E01 |
| 2-1-mlE01-2-0748 | 774.270 | OEt | a2 | B20 | b1 | C30 | c3 | D02 | d1 | E01 |
| 2-1-mlE01-2-0749 | 774.290 | OEt | a2 | B17 | b2 | C07 | c2 | D02 | d1 | E01 |
| 2-1-mlE01-2-0750 | 774.345 | OEt | a2 | B15 | b1 | C31 | c3 | D02 | d1 | E01 |
| 2-1-mlE01-2-0751 | 775.229 | OEt | a2 | B19 | b2 | C03 | c2 | D02 | d1 | E01 |
| 2-1-mlE01-2-0752 | 775.235 | OEt | a1 | B03 | b3 | C08 | c2 | D02 | d1 | E01 |
| 2-1-mlE01-2-0753 | 775.305 | OEt | a1 | B04 | b2 | C07 | c2 | D02 | d1 | E01 |
| 2-1-mlE01-2-0754 | 776.201 | OEt | a2 | B15 | b3 | C06 | c2 | D02 | d1 | E01 |
| 2-1-mlE01-2-0755 | 776.244 | OEt | a1 | B07 | b2 | C05 | c2 | D02 | d1 | E01 |
| 2-1-mlE01-2-0756 | 776.249 | OEt | a2 | B16 | b1 | C09 | c2 | D02 | d1 | E01 |
| 2-1-mlE01-2-0757 | 776.249 | OEt | a2 | B20 | b2 | C20 | c3 | D02 | d1 | E01 |
| 2-1-mlE01-2-0758 | 776.324 | OEt | a2 | B16 | b1 | C31 | c3 | D02 | d1 | E01 |
| 2-1-mlE01-2-0759 | 777.244 | OEt | a2 | B20 | b2 | C28 | c3 | D02 | d1 | E01 |
| 2-1-mlE01-2-0760 | 777.264 | OEt | a1 | B07 | b2 | C26 | c3 | D02 | d1 | E01 |
| 2-1-mlE01-2-0761 | 777.265 | OEt | a2 | B15 | b3 | C18 | c1 | D01 | d1 | E01 |
| 2-1-mlE01-2-0762 | 777.281 | OEt | a1 | B09 | b1 | C30 | c3 | D02 | d1 | E01 |
| 2-1-mlE01-2-0763 | 777.294 | OEt | a1 | B05 | b1 | C13 | c2 | D02 | d1 | E01 |
| 2-1-mlE01-2-0764 | 777.335 | OEt | a1 | B10 | b2 | C07 | c1 | D01 | d1 | E01 |
| 2-1-mlE01-2-0765 | 778.180 | OEt | a2 | B16 | b3 | C06 | c2 | D02 | d1 | E01 |
| 2-1-mlE01-2-0766 | 778.194 | OEt | a2 | B17 | b3 | C08 | c2 | D02 | d1 | E01 |
| 2-1-mlE01-2-0767 | 778.245 | OEt | a2 | B19 | b2 | C24 | c3 | D02 | d1 | E01 |
| 2-1-mlE01-2-0768 | 779.209 | OEt | a1 | B04 | b3 | C08 | c2 | D02 | d1 | E01 |
| 2-1-mlE01-2-0769 | 779.224 | OEt | a1 | B07 | b2 | C04 | c2 | D02 | d1 | E01 |
| 2-1-mlE01-2-0770 | 779.229 | OEt | a1 | B05 | b3 | C15 | c2 | D02 | d1 | E01 |
| 2-1-mlE01-2-0771 | 779.245 | OEt | a2 | B16 | b3 | C18 | c1 | D01 | d1 | E01 |
| 2-1-mlE01-2-0772 | 779.260 | OEt | a1 | B03 | b1 | C09 | c2 | D02 | d1 | E01 |
| 2-1-mlE01-2-0773 | 779.260 | OEt | a1 | B09 | b2 | C20 | c3 | D02 | d1 | E01 |
| 2-1-mlE01-2-0774 | 779.274 | OEt | a1 | B06 | b1 | C13 | c2 | D02 | d1 | E01 |
| 2-1-mlE01-2-0775 | 779.299 | OEt | a1 | B05 | b3 | C16 | c1 | D01 | d1 | E01 |
| 2-1-mlE01-2-0776 | 779.335 | OEt | a1 | B03 | b1 | C31 | c3 | D02 | d1 | E01 |
| 2-1-mlE01-2-0777 | 780.190 | OEt | a2 | B19 | b2 | C06 | c2 | D02 | d1 | E01 |
| 2-1-mlE01-2-0778 | 780.208 | OEt | a2 | B20 | b2 | C01 | c2 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0779 | 780.255 | OEt | a1 | B09 | b2 | C28 | c3 | D02 | d1 | E01 |
| 2-1-mlE01-2-0780 | 780.273 | OEt | a2 | B18 | b1 | C13 | c2 | D02 | d1 | E01 |
| 2-1-mlE01-2-0781 | 780.276 | OEt | a1 | B08 | b1 | C32 | c3 | D02 | d1 | E01 |
| 2-1-m1E01-2-0782 | 781.191 | OEt | a1 | B03 | b3 | C06 | c2 | D02 | d1 | E01 |
| 2-1-mlE01-2-0783 | 781.209 | OEt | a1 | B06 | b3 | C15 | c2 | D02 | d1 | E01 |
| 2-1-m1E01-2-0784 | 781.239 | OEt | a1 | B10 | b3 | C08 | c1 | D01 | d1 | E01 |
| 2-1-mlE01-2-0785 | 781.239 | OEt | a1 | B10 | b1 | C08 | c2 | D02 | d1 | E01 |
| 2-1-mlE01-2-0786 | 781.260 | OEt | a1 | B05 | b3 | C17 | c1 | D01 | d1 | E01 |
| 2-1-mlE01-2-0787 | 781.260 | OEt | a1 | B05 | b1 | C14 | c2 | D02 | d1 | E01 |
| 2-1-mlE01-2-0788 | 781.278 | OEt | a1 | B06 | b3 | C16 | c1 | D01 | d1 | E01 |
| 2-1-mlE01-2-0789 | 782.208 | OEt | a2 | B18 | b3 | C15 | c2 | D02 | d1 | E01 |
| 2-1-mlE01-2-0790 | 782.219 | OEt | a1 | B08 | b2 | C02 | c2 | D02 | d1 | E01 |
| 2-1-mlE01-2-0791 | 782.220 | OEt | a2 | B17 | b1 | C09 | c2 | D02 | d1 | E01 |
| 2-1-mlE01-2-0792 | 782.235 | OEt | a1 | B10 | b1 | C11 | c2 | D02 | d1 | E01 |
| 2-1-mlE01-2-0793 | 782.256 | OEt | a1 | B03 | b3 | C18 | c1 | D01 | d1 | E01 |
| 2-1-mlE01-2-0794 | 782.256 | OEt | a1 | B08 | b2 | C22 | c3 | D02 | d1 | E01 |
| 2-1-mlE01-2-0795 | 782.277 | OEt | a2 | B18 | b3 | C16 | c1 | D01 | d1 | E01 |
| 2-1-mlE01-2-0796 | 782.295 | OEt | a2 | B17 | b1 | C31 | c3 | D02 | d1 | E01 |
| 2-1-mlE01-2-0797 | 783.215 | OEt | a1 | B08 | b2 | C03 | c2 | D02 | d1 | E01 |
| 2-1-mlE01-2-0798 | 783.219 | OEt | a1 | B09 | b2 | C01 | c2 | D02 | d1 | E01 |
| 2-1-mlE01-2-0799 | 783.230 | OEt | a1 | B10 | b1 | C10 | c2 | D02 | d1 | E01 |
| 2-1-mlE01-2-0800 | 783.235 | OEt | a1 | B04 | b1 | C09 | c2 | D02 | d1 | E01 |
| 2-1-m1E01-2-0801 | 783.240 | OEt | a1 | B06 | b3 | C17 | c1 | D01 | d1 | E01 |
| 2-1-m1E01-2-0802 | 783.240 | OEt | a1 | B06 | b1 | C14 | c2 | D02 | d1 | E01 |
| 2-1-mlE01-2-0803 | 783.310 | OEt | a1 | B04 | b1 | C31 | c3 | D02 | d1 | E01 |
| 2-1-m1E01-2-0804 | 784.151 | OEt | a2 | B17 | b3 | C06 | c2 | D02 | d1 | E01 |
| 2-1-m1E01-2-0805 | 784.239 | OEt | a2 | B18 | b3 | C17 | c1 | D01 | d1 | E01 |
| 2-1-mlE01-2-0806 | 784.239 | OEt | a2 | B18 | b1 | C14 | c2 | D02 | d1 | E01 |
| 2-1-m1E01-2-0807 | 785.166 | OEt | a1 | B04 | b3 | C06 | c2 | D02 | d1 | E01 |
| 2-1-mlE01-2-0808 | 785.215 | OEt | a2 | B17 | b3 | C18 | c1 | D01 | d1 | E01 |
| 2-1-m1E01-2-0809 | 785.244 | OEt | a1 | B07 | b1 | C13 | c2 | D02 | d1 | E01 |
| 2-1-mlE01-2-0810 | 785.265 | OEt | a1 | B10 | b1 | C09 | c1 | D01 | d1 | E01 |
| 2-1-m1E01-2-0811 | 785.346 | OEt | a1 | B05 | b2 | C07 | c2 | D02 | d1 | E01 |
| 2-1-mlE01-2-0812 | 786.231 | OEt | a1 | B04 | b3 | C18 | c1 | D01 | d1 | E01 |
| 2-1-mlE01-2-0813 | 786.231 | OEt | a1 | B08 | b2 | C24 | c3 | D02 | d1 | E01 |
| 2-1-mlE01-2-0814 | 786.306 | OEt | a2 | B19 | b1 | C29 | c3 | D02 | d1 | E01 |
| 2-1-mlE01-2-0815 | 787.179 | OEt | a1 | B07 | b3 | C15 | c2 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-mlE01-2-0816 | 787.196 | OEt | a1 | B10 | b3 | C06 | c1 | D01 | d1 | E01 |
| 2-1-mlE01-2-0817 | 787.249 | OEt | a1 | B07 | b3 | C16 | c1 | D01 | d1 | E01 |
| 2-1-mlE01-2-0818 | 787.325 | OEt | a1 | B06 | b2 | C07 | c2 | D02 | d1 | E01 |
| 2-1-m1E01-2-0819 | 788.176 | OEt | a1 | B08 | b2 | C06 | c2 | D02 | d1 | E01 |
| 2-1-mlE01-2-0820 | 788.191 | OEt | a1 | B10 | b1 | C12 | c2 | D02 | d1 | E01 |
| 2-1-mlE01-2-0821 | 788.286 | OEt | a2 | B20 | b1 | C32 | c3 | D02 | d1 | E01 |
| 2-1-mlE01-2-0822 | 788.324 | OEt | a2 | B18 | b2 | C07 | c2 | D02 | d1 | E01 |
| 2-1-mlE01-2-0823 | 789.210 | OEt | a1 | B07 | b3 | C17 | c1 | D01 | d1 | E01 |
| 2-1-m1E01-2-0824 | 789.210 | OEt | a1 | B07 | b1 | C14 | c2 | D02 | d1 | E01 |
| 2-1-mlE01-2-0825 | 789.244 | OEt | a2 | B19 | b2 | C05 | c2 | D02 | d1 | E01 |
| 2-1-mlE01-2-0826 | 789.250 | OEt | a1 | B05 | b3 | C08 | c2 | D02 | d1 | E01 |
| 2-1-mlE01-2-0827 | 790.228 | OEt | a2 | B20 | b2 | C02 | c2 | D02 | d1 | E01 |
| 2-1-mlE01-2-0828 | 790.265 | OEt | a2 | B20 | b2 | C22 | c3 | D02 | d1 | E01 |
| 2-1-mlE01-2-0829 | 790.265 | OEt | a2 | B19 | b2 | C26 | c3 | D02 | d1 | E01 |
| 2-1-m1E01-2-0830 | 791.224 | OEt | a2 | B20 | b2 | C03 | c2 | D02 | d1 | E01 |
| 2-1-mlE01-2-0831 | 791.229 | OEt | a1 | B06 | b3 | C08 | c2 | D02 | d1 | E01 |
| 2-1-mlE01-2-0832 | 791.296 | OEt | a1 | B09 | b1 | C32 | c3 | D02 | d1 | E01 |
| 2-1-mlE01-2-0833 | 792.224 | OEt | a2 | B19 | b2 | C04 | c2 | D02 | d1 | E01 |
| 2-1-m1E01-2-0834 | 792.229 | OEt | a2 | B18 | b3 | C08 | c2 | D02 | d1 | E01 |
| 2-1-mlE01-2-0835 | 793.239 | OEt | a1 | B09 | b2 | C02 | c2 | D02 | d1 | E01 |
| 2-1-mlE01-2-0836 | 793.276 | OEt | a1 | B05 | b1 | C09 | c2 | D02 | d1 | E01 |
| 2-1-mlE01-2-0837 | 793.276 | OEt | a1 | B09 | b2 | C22 | c3 | D02 | d1 | E01 |
| 2-1-mlE01-2-0838 | 793.276 | OEt | a1 | B10 | b1 | C30 | c3 | D02 | d1 | E01 |
| 2-1-m1E01-2-0839 | 793.296 | OEt | a1 | B07 | b2 | C07 | c2 | D02 | d1 | E01 |
| 2-1-m1E01-2-0840 | 793.351 | OEt | a1 | B05 | b1 | C31 | c3 | D02 | d1 | E01 |
| 2-1-mlE01-2-0841 | 794.235 | OEt | a1 | B09 | b2 | C03 | c2 | D02 | d1 | E01 |
| 2-1-mlE01-2-0842 | 794.240 | OEt | a2 | B20 | b2 | C24 | c3 | D02 | d1 | E01 |
| 2-1-m1E01-2-0843 | 794.292 | OEt | a1 | B08 | b1 | C29 | c3 | D02 | d1 | E01 |
| 2-1-mlE01-2-0844 | 795.207 | OEt | a1 | B05 | b3 | C06 | c2 | D02 | d1 | E01 |
| 2-1-mlE01-2-0845 | 795.255 | OEt | a1 | B06 | b1 | C09 | c2 | D02 | d1 | E01 |
| 2-1-mlE01-2-0846 | 795.255 | OEt | a1 | B10 | b2 | C20 | c3 | D02 | d1 | E01 |
| 2-1-mlE01-2-0847 | 795.330 | OEt | a1 | B06 | b1 | C31 | c3 | D02 | d1 | E01 |
| 2-1-mlE01-2-0848 | 796.185 | OEt | a2 | B20 | b2 | C06 | c2 | D02 | d1 | E01 |
| 2-1-m1E01-2-0849 | 796.224 | OEt | a2 | B11 | b3 | C16 | c2 | D02 | d1 | E01 |
| 2-1-m1E01-2-0850 | 796.250 | OEt | a1 | B10 | b2 | C28 | c3 | D02 | d1 | E01 |
| 2-1-m1E01-2-0851 | 796.254 | OEt | a2 | B18 | b1 | C09 | c2 | D02 | d1 | E01 |
| 2-1-mlE01-2-0852 | 796.271 | OEt | a1 | B05 | b3 | C18 | c1 | D01 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0853 | 796.329 | OEt | a2 | B18 | b1 | C31 | c3 | D02 | d1 | E01 |
| 2-1-m1E01-2-0854 | 797.186 | OEt | a1 | B06 | b3 | C06 | c2 | D02 | d1 | E01 |
| 2-1-mlE01-2-0855 | 797.200 | OEt | a1 | B07 | b3 | C08 | c2 | D02 | d1 | E01 |
| 2-1-m1E01-2-0856 | 797.219 | OEt | a2 | B12 | b3 | C16 | c2 | D02 | d1 | E01 |
| 2-1-m1E01-2-0857 | 797.230 | OEt | a1 | B08 | b2 | C05 | c2 | D02 | d1 | E01 |
| 2-1-m1E01-2-0858 | 797.251 | OEt | a1 | B09 | b2 | C24 | c3 | D02 | d1 | E01 |
| 2-1-m1E01-2-0859 | 798.185 | OEt | a2 | B18 | b3 | C06 | c2 | D02 | d1 | E01 |
| 2-1-mlE01-2-0860 | 798.185 | OEt | a2 | B11 | b3 | C17 | c2 | D02 | d1 | E01 |
| 2-1-m1E01-2-0861 | 798.245 | OEt | a2 | B19 | b1 | C13 | c2 | D02 | d1 | E01 |
| 2-1-mlE01-2-0862 | 798.251 | OEt | a1 | B06 | b3 | C18 | c1 | D01 | d1 | E01 |
| 2-1-mlE01-2-0863 | 798.251 | OEt | a1 | B08 | b2 | C26 | c3 | D02 | d1 | E01 |
| 2-1-mlE01-2-0864 | 799.180 | OEt | a2 | B12 | b3 | C17 | c2 | D02 | d1 | E01 |
| 2-1-mlE01-2-0865 | 799.196 | OEt | a1 | B09 | b2 | C06 | c2 | D02 | d1 | E01 |
| 2-1-mlE01-2-0866 | 799.213 | OEt | a1 | B10 | b2 | C01 | c2 | D02 | d1 | E01 |
| 2-1-mlE01-2-0867 | 799.250 | OEt | a2 | B18 | b3 | C18 | c1 | D01 | d1 | E01 |
| 2-1-m1E01-2-0868 | 800.180 | OEt | a2 | B19 | b3 | C15 | c2 | D02 | d1 | E01 |
| 2-1-m1E01-2-0869 | 800.210 | OEt | a1 | B08 | b2 | C04 | c2 | D02 | d1 | E01 |
| 2-1-mlE01-2-0870 | 800.249 | OEt | a2 | B19 | b3 | C16 | c1 | D01 | d1 | E01 |
| 2-1-m1E01-2-0871 | 801.226 | OEt | a1 | B07 | b1 | C09 | c2 | D02 | d1 | E01 |
| 2-1-mlE01-2-0872 | 801.301 | OEt | a1 | B07 | b1 | C31 | c3 | D02 | d1 | E01 |
| 2-1-mlE01-2-0873 | 802.211 | OEt | a2 | B19 | b3 | C17 | c1 | D01 | d1 | E01 |
| 2-1-mlE01-2-0874 | 802.211 | OEt | a2 | B19 | b1 | C14 | c2 | D02 | d1 | E01 |
| 2-1-mlE01-2-0875 | 802.301 | OEt | a2 | B20 | b1 | C29 | c3 | D02 | d1 | E01 |
| 2-1-mlE01-2-0876 | 803.156 | OEt | a1 | B07 | b3 | C06 | c2 | D02 | d1 | E01 |
| 2-1-mlE01-2-0877 | 804.221 | OEt | a1 | B07 | b3 | C18 | c1 | D01 | d1 | E01 |
| 2-1-m1E01-2-0878 | 805.239 | OEt | a2 | B20 | b2 | C05 | c2 | D02 | d1 | E01 |
| 2-1-mlE01-2-0879 | 805.312 | OEt | a1 | B09 | b1 | C29 | c3 | D02 | d1 | E01 |
| 2-1-mlE01-2-0880 | 806.230 | OEt | a1 | B08 | b1 | C13 | c2 | D02 | d1 | E01 |
| 2-1-m1E01-2-0881 | 806.260 | OEt | a2 | B20 | b2 | C26 | c3 | D02 | d1 | E01 |
| 2-1-mlE01-2-0882 | 806.296 | OEt | a2 | B19 | b2 | C07 | c2 | D02 | d1 | E01 |
| 2-1-mlE01-2-0883 | 807.291 | OEt | a1 | B10 | b1 | C32 | c3 | D02 | d1 | E01 |
| 2-1-m1E01-2-0884 | 808.166 | OEt | a1 | B08 | b3 | C15 | c2 | D02 | d1 | E01 |
| 2-1-mlE01-2-0885 | 808.219 | OEt | a2 | B20 | b2 | C04 | c2 | D02 | d1 | E01 |
| 2-1-m1E01-2-0886 | 808.235 | OEt | a1 | B08 | b3 | C16 | c1 | D01 | d1 | E01 |
| 2-1-m1E01-2-0887 | 808.250 | OEt | a1 | B09 | b2 | C05 | c2 | D02 | d1 | E01 |
| 2-1-m1E01-2-0888 | 809.234 | OEt | a1 | B10 | b2 | C02 | c2 | D02 | d1 | E01 |
| 2-1-m1E01-2-0889 | 809.271 | OEt | a1 | B10 | b2 | C22 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0890 | 809.271 | OEt | a1 | B09 | b2 | C26 | c3 | D02 | d1 | E01 |
| 2-1-m1E01-2-0891 | 810.196 | OEt | a1 | B08 | b3 | C17 | c1 | D01 | d1 | E01 |
| 2-1-m1E01-2-0892 | 810.196 | OEt | a1 | B08 | b1 | C14 | c2 | D02 | d1 | E01 |
| 2-1-m1E01-2-0893 | 810.200 | OEt | a2 | B19 | b3 | C08 | c2 | D02 | d1 | E01 |
| 2-1-m1E01-2-0894 | 810.229 | OEt | a1 | B10 | b2 | C03 | c2 | D02 | d1 | E01 |
| 2-1-m1E01-2-0895 | 810.239 | OEt | a2 | B13 | b3 | C16 | c2 | D02 | d1 | E01 |
| 2-1-mlE01-2-0896 | 811.230 | OEt | a1 | B09 | b2 | C04 | c2 | D02 | d1 | E01 |
| 2-1-m1E01-2-0897 | 812.201 | OEt | a2 | B13 | b3 | C17 | c2 | D02 | d1 | E01 |
| 2-1-mlE01-2-0898 | 813.196 | OEt | a2 | B11 | b3 | C18 | c2 | D02 | d1 | E01 |
| 2-1-mlE01-2-0899 | 813.246 | OEt | a1 | B10 | b2 | C24 | c3 | D02 | d1 | E01 |
| 2-1-m1E01-2-0900 | 814.191 | OEt | a2 | B12 | b3 | C18 | c2 | D02 | d1 | E01 |
| 2-1-m1E01-2-0901 | 814.214 | OEt | a2 | B14 | b3 | C16 | c2 | D02 | d1 | E01 |
| 2-1-m1E01-2-0902 | 814.226 | OEt | a2 | B19 | b1 | C09 | c2 | D02 | d1 | E01 |
| 2-1-m1E01-2-0903 | 814.240 | OEt | a2 | B20 | b1 | C13 | c2 | D02 | d1 | E01 |
| 2-1-m1E01-2-0904 | 814.282 | OEt | a1 | B08 | b2 | C07 | c2 | D02 | d1 | E01 |
| 2-1-m1E01-2-0905 | 814.301 | OEt | a2 | B19 | b1 | C31 | c3 | D02 | d1 | E01 |
| 2-1-m1E01-2-0906 | 815.191 | OEt | a1 | B10 | b2 | C06 | c2 | D02 | d1 | E01 |
| 2-1-m1E01-2-0907 | 815.229 | OEt | a1 | B01 | b3 | C16 | c2 | D02 | d1 | E01 |
| 2-1-m1E01-2-0908 | 816.157 | OEt | a2 | B19 | b3 | C06 | c2 | D02 | d1 | E01 |
| 2-1-m1E01-2-0909 | 816.175 | OEt | a2 | B20 | b3 | C15 | c2 | D02 | d1 | E01 |
| 2-1-m1E01-2-0910 | 816.176 | OEt | a2 | B14 | b3 | C17 | c2 | D02 | d1 | E01 |
| 2-1-mlE01-2-0911 | 816.225 | OEt | a1 | B02 | b3 | C16 | c2 | D02 | d1 | E01 |
| 2-1-mlE01-2-0912 | 816.244 | OEt | a2 | B20 | b3 | C16 | c1 | D01 | d1 | E01 |
| 2-1-mlE01-2-0913 | 817.191 | OEt | a1 | B01 | b3 | C17 | c2 | D02 | d1 | E01 |
| 2-1-mlE01-2-0914 | 817.222 | OEt | a2 | B19 | b3 | C18 | c1 | D01 | d1 | E01 |
| 2-1-mlE01-2-0915 | 817.251 | OEt | a1 | B09 | b1 | C13 | c2 | D02 | d1 | E01 |
| 2-1-mlE01-2-0916 | 818.186 | OEt | a1 | B02 | b3 | C17 | c2 | D02 | d1 | E01 |
| 2-1-mlE01-2-0917 | 818.186 | OEt | a1 | B08 | b3 | C08 | c2 | D02 | d1 | E01 |
| 2-1-mlE01-2-0918 | 818.206 | OEt | a2 | B20 | b3 | C17 | c1 | D01 | d1 | E01 |
| 2-1-mlE01-2-0919 | 818.206 | OEt | a2 | B20 | b1 | C14 | c2 | D02 | d1 | E01 |
| 2-1-mlE01-2-0920 | 819.186 | OEt | a1 | B09 | b3 | C15 | c2 | D02 | d1 | E01 |
| 2-1-m1E01-2-0921 | 819.255 | OEt | a1 | B09 | b3 | C16 | c1 | D01 | d1 | E01 |
| 2-1-m1E01-2-0922 | 821.216 | OEt | a1 | B09 | b3 | C17 | c1 | D01 | d1 | E01 |
| 2-1-mlE01-2-0923 | 821.216 | OEt | a1 | B09 | b1 | C14 | c2 | D02 | d1 | E01 |
| 2-1-m1E01-2-0924 | 821.307 | OEt | a1 | B10 | b1 | C29 | c3 | D02 | d1 | E01 |
| 2-1-mlE01-2-0925 | 822.212 | OEt | a1 | B08 | b1 | C09 | c2 | D02 | d1 | E01 |
| 2-1-mlE01-2-0926 | 822.287 | OEt | a1 | B08 | b1 | C31 | c3 | D02 | d1 | E01 |

(continued)

| [Table 8-10-2] Compound that may be contained in mixture 2-1-m1E01-2 | | | | | | | | | | |
|---|---|---|---|---|---|---|---|---|---|---|
| ID | Exact Mass | | a | B | b | C | c | D | d | E |
| 2-1-m1E01-2-0927 | 822.291 | OEt | a2 | B20 | b2 | C07 | c2 | D02 | d1 | E01 |
| 2-1-mlE01-2-0928 | 824.143 | OEt | a1 | B08 | b3 | C06 | c2 | D02 | d1 | E01 |
| 2-1-m1E01-2-0929 | 824.245 | OEt | a1 | B10 | b2 | C05 | c2 | D02 | d1 | E01 |
| 2-1-m 1 E01-2-0930 | 824.255 | OEt | a2 | B15 | b3 | C16 | c2 | D02 | d1 | E01 |
| 2-1-m1E01-2-0931 | 825.207 | OEt | a1 | B08 | b3 | C18 | c1 | D01 | d1 | E01 |
| 2-1-mlE01-2-0932 | 825.266 | OEt | a1 | B10 | b2 | C26 | c3 | D02 | d1 | E01 |
| 2-1-mlE01-2-0933 | 825.302 | OEt | a1 | B09 | b2 | C07 | c2 | D02 | d1 | E01 |
| 2-1-mlE01-2-0934 | 826.195 | OEt | a2 | B20 | b3 | C08 | c2 | D02 | d1 | E01 |
| 2-1-mlE01-2-0935 | 826.216 | OEt | a2 | B15 | b3 | C17 | c2 | D02 | d1 | E01 |
| 2-1-mlE01-2-0936 | 826.234 | OEt | a2 | B16 | b3 | C16 | c2 | D02 | d1 | E01 |
| 2-1-mlE01-2-0937 | 827.212 | OEt | a2 | B13 | b3 | C18 | c2 | D02 | d1 | E01 |
| 2-1-mlE01-2-0938 | 827.225 | OEt | a1 | B10 | b2 | C04 | c2 | D02 | d1 | E01 |
| 2-1-mlE01-2-0939 | 828.196 | OEt | a2 | B16 | b3 | C17 | c2 | D02 | d1 | E01 |
| 2-1-m1E01-2-0940 | 829.206 | OEt | a1 | B09 | b3 | C08 | c2 | D02 | d1 | E01 |
| 2-1-m1E01-2-0941 | 829.245 | OEt | a1 | B03 | b3 | C16 | c2 | D02 | d1 | E01 |
| 2-1-mlE01-2-0942 | 830.221 | OEt | a2 | B20 | b1 | C09 | c2 | D02 | d1 | E01 |
| 2-1-mlE01-2-0943 | 830.296 | OEt | a2 | B20 | b1 | C31 | c3 | D02 | d1 | E01 |
| 2-1-m1E01-2-0944 | 831.187 | OEt | a2 | B14 | b3 | C18 | c2 | D02 | d1 | E01 |
| 2-1-mlE01-2-0945 | 831.207 | OEt | a1 | B03 | b3 | C17 | c2 | D02 | d1 | E01 |
| 2-1-mlE01-2-0946 | 832.152 | OEt | a2 | B20 | b3 | C06 | c2 | D02 | d1 | E01 |
| 2-1-mlE01-2-0947 | 832.202 | OEt | a1 | B01 | b3 | C18 | c2 | D02 | d1 | E01 |
| 2-1-mlE01-2-0948 | 832.205 | OEt | a2 | B17 | b3 | C16 | c2 | D02 | d1 | E01 |
| 2-1-m1E01-2-0949 | 833.197 | OEt | a1 | B02 | b3 | C18 | c2 | D02 | d1 | E01 |
| 2-1-m1E01-2-0950 | 833.216 | OEt | a2 | B20 | b3 | C18 | c1 | D01 | d1 | E01 |
| 2-1-m1E01-2-0951 | 833.220 | OEt | a1 | B04 | b3 | C16 | c2 | D02 | d1 | E01 |
| 2-1-mlE01-2-0952 | 833.232 | OEt | a1 | B09 | b1 | C09 | c2 | D02 | d1 | E01 |
| 2-1-m1E01-2-0953 | 833.245 | OEt | a1 | B10 | b1 | C13 | c2 | D02 | d1 | E01 |
| 2-1-mlE01-2-0954 | 833.307 | OEt | a1 | B09 | b1 | C31 | c3 | D02 | d1 | E01 |
| 2-1-mlE01-2-0955 | 834.166 | OEt | a2 | B17 | b3 | C17 | c2 | D02 | d1 | E01 |
| 2-1-m1E01-2-0956 | 835.163 | OEt | a1 | B09 | b3 | C06 | c2 | D02 | d1 | E01 |
| 2-1-mlE01-2-0957 | 835.180 | OEt | a1 | B10 | b3 | C15 | c2 | D02 | d1 | E01 |
| 2-1-mlE01-2-0958 | 835.182 | OEt | a1 | B04 | b3 | C17 | c2 | D02 | d1 | E01 |
| 2-1-mlE01-2-0959 | 835.250 | OEt | a1 | B10 | b3 | C16 | c1 | D01 | d1 | E01 |
| 2-1-m1E01-2-0960 | 836.227 | OEt | a1 | B09 | b3 | C18 | c1 | D01 | d1 | E01 |
| 2-1-m1E01-2-0961 | 837.211 | OEt | a1 | B10 | b3 | C17 | c1 | D01 | d1 | E01 |
| 2-1-mlE01-2-0962 | 837.211 | OEt | a1 | B10 | b1 | C14 | c2 | D02 | d1 | E01 |
| 2-1-mlE01-2-0963 | 841.227 | OEt | a2 | B15 | b3 | C18 | c2 | D02 | d1 | E01 |

(continued)

[Table 8-10-2] Compound that may be contained in mixture 2-1-m1E01-2

| ID | Exact Mass | | a | B | b | C | c | D | d | E |
|---|---|---|---|---|---|---|---|---|---|---|
| 2-1-mlE01-2-0964 | 841.297 | OEt | a1 | B10 | b2 | C07 | c2 | D02 | d1 | E01 |
| 2-1-mlE01-2-0965 | 843.207 | OEt | a2 | B16 | b3 | C18 | c2 | D02 | d1 | E01 |
| 2-1-mlE01-2-0966 | 843.261 | OEt | a1 | B05 | b3 | C16 | c2 | D02 | d1 | E01 |
| 2-1-m1E01-2-0967 | 845.201 | OEt | a1 | B10 | b3 | C08 | c2 | D02 | d1 | E01 |
| 2-1-mlE01-2-0968 | 845.222 | OEt | a1 | B05 | b3 | C17 | c2 | D02 | d1 | E01 |
| 2-1-mlE01-2-0969 | 845.240 | OEt | a1 | B06 | b3 | C16 | c2 | D02 | d1 | E01 |
| 2-1-mlE01-2-0970 | 846.218 | OEt | a1 | B03 | b3 | C18 | c2 | D02 | d1 | E01 |
| 2-1-m1E01-2-0971 | 846.239 | OEt | a2 | B18 | b3 | C16 | c2 | D02 | d1 | E01 |
| 2-1-m1E01-2-0972 | 847.202 | OEt | a1 | B06 | b3 | C17 | c2 | D02 | d1 | E01 |
| 2-1-m1E01-2-0973 | 848.201 | OEt | a2 | B18 | b3 | C17 | c2 | D02 | d1 | E01 |
| 2-1-m1E01-2-0974 | 849.177 | OEt | a2 | B17 | b3 | C18 | c2 | D02 | d1 | E01 |
| 2-1-m1E01-2-0975 | 849.227 | OEt | a1 | B10 | b1 | C09 | c2 | D02 | d1 | E01 |
| 2-1-m1E01-2-0976 | 849.302 | OEt | a1 | B10 | b1 | C31 | c3 | D02 | d1 | E01 |
| 2-1-m1E01-2-0977 | 850.192 | OEt | a1 | B04 | b3 | C18 | c2 | D02 | d1 | E01 |
| 2-1-m1E01-2-0978 | 851.158 | OEt | a1 | B10 | b3 | C06 | c2 | D02 | d1 | E01 |
| 2-1-m1E01-2-0979 | 851.211 | OEt | a1 | B07 | b3 | C16 | c2 | D02 | d1 | E01 |
| 2-1-m1E01-2-0980 | 852.222 | OEt | a1 | B10 | b3 | C18 | c1 | D01 | d1 | E01 |
| 2-1-m1E01-2-0981 | 853.172 | OEt | a1 | B07 | b3 | C17 | c2 | D02 | d1 | E01 |
| 2-1-m1E01-2-0982 | 860.233 | OEt | a1 | B05 | b3 | C18 | c2 | D02 | d1 | E01 |
| 2-1-m1E01-2-0983 | 862.212 | OEt | a1 | B06 | b3 | C18 | c2 | D02 | d1 | E01 |
| 2-1-m1E01-2-0984 | 863.212 | OEt | a2 | B18 | b3 | C18 | c2 | D02 | d1 | E01 |
| 2-1-m1E01-2-0985 | 864.211 | OEt | a2 | B19 | b3 | C16 | c2 | D02 | d1 | E01 |
| 2-1-m1E01-2-0986 | 866.173 | OEt | a2 | B19 | b3 | C17 | c2 | D02 | d1 | E01 |
| 2-1-m1E01-2-0987 | 868.183 | OEt | a1 | B07 | b3 | C18 | c2 | D02 | d1 | E01 |
| 2-1-m1E01-2-0988 | 872.197 | OEt | a1 | B08 | b3 | C16 | c2 | D02 | d1 | E01 |
| 2-1-m1E01-2-0989 | 874.158 | OEt | a1 | B08 | b3 | C17 | c2 | D02 | d1 | E01 |
| 2-1-m1E01-2-0990 | 880.206 | OEt | a2 | B20 | b3 | C16 | c2 | D02 | d1 | E01 |
| 2-1-mlE01-2-0991 | 881.183 | OEt | a2 | B19 | b3 | C18 | c2 | D02 | d1 | E01 |
| 2-1-m1E01-2-0992 | 882.167 | OEt | a2 | B20 | b3 | C17 | c2 | D02 | d1 | E01 |
| 2-1-m1E01-2-0993 | 883.217 | OEt | a1 | B09 | b3 | C16 | c2 | D02 | d1 | E01 |
| 2-1-m1E01-2-0994 | 885.178 | OEt | a1 | B09 | b3 | C17 | c2 | D02 | d1 | E01 |
| 2-1-m1E01-2-0995 | 889.169 | OEt | a1 | B08 | b3 | C18 | c2 | D02 | d1 | E01 |
| 2-1-mlE01-2-0996 | 897.178 | OEt | a2 | B20 | b3 | C18 | c2 | D02 | d1 | E01 |
| 2-1-m1E01-2-0997 | 899.212 | OEt | a1 | B10 | b3 | C16 | c2 | D02 | d1 | E01 |
| 2-1-m1E01-2-0998 | 900.189 | OEt | a1 | B09 | b3 | C18 | c2 | D02 | d1 | E01 |
| 2-1-m1E01-2-0999 | 901.173 | OEt | a1 | B10 | b3 | C17 | c2 | D02 | d1 | E01 |
| 2-1-m1E01-2-1000 | 916.184 | OEt | a1 | B10 | b3 | C18 | c2 | D02 | d1 | E01 |

[Table 403]

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0001 | 630.284 | OEt | a2 | B11 | b1 | C08 | c1 | D01 | d1 | E02 |
| 2-1-m1E02-2-0002 | 631.279 | OEt | a2 | B12 | b1 | C08 | c1 | D01 | d1 | E02 |
| 2-1-mlE02-2-0003 | 631.279 | OEt | a2 | B11 | b1 | C11 | c1 | D01 | d1 | E02 |
| 2-1-m1E02-2-0004 | 632.275 | OEt | a2 | B11 | b1 | C10 | c1 | D01 | d1 | E02 |
| 2-1-mlE02-2-0005 | 632.275 | OEt | a2 | B12 | b1 | C11 | c1 | D01 | d1 | E02 |
| 2-1-mlE02-2-0006 | 633.270 | OEt | a2 | B12 | b1 | C10 | c1 | D01 | d1 | E02 |
| 2-1-m1E02-2-0007 | 637.236 | OEt | a2 | B11 | b1 | C12 | c1 | D01 | d1 | E02 |
| 2-1-mlE02-2-0008 | 638.231 | OEt | a2 | B12 | b1 | C12 | c1 | D01 | d1 | E02 |
| 2-1-m1E02-2-0009 | 644.300 | OEt | a2 | B13 | b1 | C08 | c1 | D01 | d1 | E02 |
| 2-1-mlE02-2-0010 | 645.295 | OEt | a2 | B13 | b1 | C11 | c1 | D01 | d1 | E02 |
| 2-1-m1E02-2-0011 | 646.290 | OEt | a2 | B13 | b1 | C10 | c1 | D01 | d1 | E02 |
| 2-1-mlE02-2-0012 | 648.258 | OEt | a2 | B11 | b2 | C01 | c1 | D01 | d1 | E02 |
| 2-1-mlE02-2-0013 | 648.275 | OEt | a2 | B14 | b1 | C08 | c1 | D01 | d1 | E02 |
| 2-1-m1E02-2-0014 | 649.254 | OEt | a2 | B12 | b2 | C01 | c1 | D01 | d1 | E02 |
| 2-1-mlE02-2-0015 | 649.270 | OEt | a2 | B14 | b1 | C11 | c1 | D01 | d1 | E02 |
| 2-1-m1E02-2-0016 | 649.290 | OEt | a1 | B01 | b1 | C08 | c1 | D01 | d1 | E02 |
| 2-1-mlE02-2-0017 | 650.265 | OEt | a2 | B14 | b1 | C10 | c1 | D01 | d1 | E02 |
| 2-1-mlE02-2-0018 | 650.285 | OEt | a1 | B02 | b1 | C08 | c1 | D01 | d1 | E02 |
| 2-1-m1E02-2-0019 | 650.285 | OEt | a1 | B01 | b1 | C11 | c1 | D01 | d1 | E02 |
| 2-1-mlE02-2-0020 | 651.252 | OEt | a2 | B13 | b1 | C12 | c1 | D01 | d1 | E02 |
| 2-1-m1E02-2-0021 | 651.281 | OEt | a1 | B01 | b1 | C10 | c1 | D01 | d1 | E02 |
| 2-1-mlE02-2-0022 | 651.281 | OEt | a1 | B02 | b1 | C11 | c1 | D01 | d1 | E02 |
| 2-1-m1E02-2-0023 | 652.276 | OEt | a1 | B02 | b1 | C10 | c1 | D01 | d1 | E02 |
| 2-1-m1E02-2-0024 | 655.226 | OEt | a2 | B14 | b1 | C12 | c1 | D01 | d1 | E02 |
| 2-1-mlE02-2-0025 | 656.242 | OEt | a1 | B01 | b1 | C12 | c1 | D01 | d1 | E02 |
| 2-1-m1E02-2-0026 | 657.237 | OEt | a1 | B02 | b1 | C12 | c1 | D01 | d1 | E02 |
| 2-1-mlE02-2-0027 | 658.279 | OEt | a2 | B11 | b2 | C02 | c1 | D01 | d1 | E02 |
| 2-1-m1E02-2-0028 | 658.316 | OEt | a2 | B15 | b1 | C08 | c1 | D01 | d1 | E02 |
| 2-1-mlE02-2-0029 | 659.274 | OEt | a2 | B12 | b2 | C02 | c1 | D01 | d1 | E02 |
| 2-1-mlE02-2-0030 | 659.274 | OEt | a2 | B11 | b2 | C03 | c1 | D01 | d1 | E02 |
| 2-1-m1E02-2-0031 | 659.311 | OEt | a2 | B15 | b1 | C11 | c1 | D01 | d1 | E02 |
| 2-1-mlE02-2-0032 | 660.270 | OEt | a2 | B12 | b2 | C03 | c1 | D01 | d1 | E02 |
| 2-1-m1E02-2-0033 | 660.295 | OEt | a2 | B16 | b1 | C08 | c1 | D01 | d1 | E02 |
| 2-1-mlE02-2-0034 | 660.306 | OEt | a2 | B15 | b1 | C10 | c1 | D01 | d1 | E02 |
| 2-1-m1E02-2-0035 | 661.290 | OEt | a2 | B16 | b1 | C11 | c1 | D01 | d1 | E02 |
| 2-1-mlE02-2-0036 | 662.274 | OEt | a2 | B13 | b2 | C01 | c1 | D01 | d1 | E02 |
| 2-1-mlE02-2-0037 | 662.285 | OEt | a2 | B16 | b1 | C10 | c1 | D01 | d1 | E02 |
| 2-1-m1E02-2-0038 | 663.306 | OEt | a1 | B03 | b1 | C08 | c1 | D01 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0039 | 664.236 | OEt | a2 | B11 | b2 | C06 | c1 | D01 | d1 | E02 |
| 2-1-m1E02-2-0040 | 664.301 | OEt | a1 | B03 | b1 | C11 | c1 | D01 | d1 | E02 |
| 2-1-mlE02-2-0041 | 665.231 | OEt | a2 | B12 | b2 | C06 | c1 | D01 | d1 | E02 |
| 2-1-mlE02-2-0042 | 665.267 | OEt | a2 | B15 | b1 | C12 | c1 | D01 | d1 | E02 |
| 2-1-m1E02-2-0043 | 665.296 | OEt | a1 | B03 | b1 | C10 | c1 | D01 | d1 | E02 |
| 2-1-mlE02-2-0044 | 666.249 | OEt | a2 | B14 | b2 | C01 | c1 | D01 | d1 | E02 |
| 2-1-m1E02-2-0045 | 666.265 | OEt | a2 | B17 | b1 | C08 | c1 | D01 | d1 | E02 |
| 2-1-mlE02-2-0046 | 667.246 | OEt | a2 | B16 | b1 | C12 | c1 | D01 | d1 | E02 |
| 2-1-m1E02-2-0047 | 667.261 | OEt | a2 | B17 | b1 | C11 | c1 | D01 | d1 | E02 |
| 2-1-mlE02-2-0048 | 667.264 | OEt | a1 | B01 | b2 | C01 | c1 | D01 | d1 | E02 |
| 2-1-m1E02-2-0049 | 667.281 | OEt | a1 | B04 | b1 | C08 | c1 | D01 | d1 | E02 |
| 2-1-m1E02-2-0050 | 668.256 | OEt | a2 | B17 | b1 | C10 | c1 | D01 | d1 | E02 |
| 2-1-mlE02-2-0051 | 668.259 | OEt | a1 | B02 | b2 | C01 | c1 | D01 | d1 | E02 |
| 2-1-m1E02-2-0052 | 668.276 | OEt | a1 | B04 | b1 | C11 | c1 | D01 | d1 | E02 |
| 2-1-m 1 E02-2-0053 | 669.271 | OEt | a1 | B04 | b1 | C10 | c1 | D01 | d1 | E02 |
| 2-1-m1E02-2-0054 | 670.257 | OEt | a1 | B03 | b1 | C12 | c1 | D01 | d1 | E02 |
| 2-1-mlE02-2-0055 | 672.295 | OEt | a2 | B13 | b2 | C02 | c1 | D01 | d1 | E02 |
| 2-1-mlE02-2-0056 | 673.217 | OEt | a2 | B17 | b1 | C12 | c1 | D01 | d1 | E02 |
| 2-1-m1E02-2-0057 | 673.290 | OEt | a2 | B13 | b2 | C03 | c1 | D01 | d1 | E02 |
| 2-1-m1E02-2-0058 | 673.290 | OEt | a2 | B11 | b2 | COS | c1 | D01 | d1 | E02 |
| 2-1-m 1 E02-2-0059 | 674.232 | OEt | a1 | B04 | b1 | C12 | c1 | D01 | d1 | E02 |
| 2-1-m1E02-2-0060 | 674.285 | OEt | a2 | B12 | b2 | COS | c1 | D01 | d1 | E02 |
| 2-1-m1E02-2-0061 | 676.270 | OEt | a2 | B14 | b2 | C02 | c1 | D01 | d1 | E02 |
| 2-1-mlE02-2-0062 | 676.270 | OEt | a2 | B11 | b2 | C04 | c1 | D01 | d1 | E02 |
| 2-1-m1E02-2-0063 | 676.290 | OEt | a2 | B15 | b2 | C01 | c1 | D01 | d1 | E02 |
| 2-1-mlE02-2-0064 | 677.265 | OEt | a2 | B12 | b2 | C04 | c1 | D01 | d1 | E02 |
| 2-1-mlE02-2-0065 | 677.265 | OEt | a2 | B14 | b2 | C03 | c1 | D01 | d1 | E02 |
| 2-1-m1E02-2-0066 | 677.285 | OEt | a1 | B01 | b2 | C02 | c1 | D01 | d1 | E02 |
| 2-1-mlE02-2-0067 | 677.321 | OEt | a1 | B05 | b1 | C08 | c1 | D01 | d1 | E02 |
| 2-1-m1E02-2-0068 | 678.251 | OEt | a2 | B13 | b2 | C06 | c1 | D01 | d1 | E02 |
| 2-1-mlE02-2-0069 | 678.269 | OEt | a2 | B16 | b2 | C01 | c1 | D01 | d1 | E02 |
| 2-1-m1E02-2-0070 | 678.280 | OEt | a1 | B02 | b2 | C02 | c1 | D01 | d1 | E02 |
| 2-1-m1E02-2-0071 | 678.280 | OEt | a1 | B01 | b2 | C03 | c1 | D01 | d1 | E02 |
| 2-1-mlE02-2-0072 | 678.317 | OEt | a1 | B05 | b1 | C11 | c1 | D01 | d1 | E02 |
| 2-1-m1E02-2-0073 | 679.275 | OEt | a1 | B02 | b2 | C03 | c1 | D01 | d1 | E02 |
| 2-1-mlE02-2-0074 | 679.301 | OEt | a1 | B06 | b1 | C08 | c1 | D01 | d1 | E02 |
| 2-1-m1E02-2-0075 | 679.312 | OEt | a1 | B05 | b1 | C10 | c1 | D01 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0076 | 680.296 | OEt | a1 | B06 | b1 | C11 | c1 | D01 | d1 | E02 |
| 2-1-mlE02-2-0077 | 680.300 | OEt | a2 | B18 | b1 | C08 | c1 | D01 | d1 | E02 |
| 2-1-m1E02-2-0078 | 681.280 | OEt | a1 | B03 | b2 | C01 | c1 | D01 | d1 | E02 |
| 2-1-mlE02-2-0079 | 681.291 | OEt | a1 | B06 | b1 | C10 | c1 | D01 | d1 | E02 |
| 2-1-m1E02-2-0080 | 681.295 | OEt | a2 | B18 | b1 | C11 | c1 | D01 | d1 | E02 |
| 2-1-mlE02-2-0081 | 682.226 | OEt | a2 | B14 | b2 | C06 | c1 | D01 | d1 | E02 |
| 2-1-m1E02-2-0082 | 682.290 | OEt | a2 | B18 | b1 | C10 | c1 | D01 | d1 | E02 |
| 2-1-mlE02-2-0083 | 682.290 | OEt | a2 | B11 | b1 | C13 | c1 | D01 | d1 | E02 |
| 2-1-mlE02-2-0084 | 683.241 | OEt | a1 | B01 | b2 | C06 | c1 | D01 | d1 | E02 |
| 2-1-m1E02-2-0085 | 683.286 | OEt | a2 | B12 | b1 | C13 | c1 | D01 | d1 | E02 |
| 2-1-mlE02-2-0086 | 684.225 | OEt | a2 | B11 | b3 | C15 | c1 | D01 | d1 | E02 |
| 2-1-m1E02-2-0087 | 684.237 | OEt | a1 | B02 | b2 | C06 | c1 | D01 | d1 | E02 |
| 2-1-mlE02-2-0088 | 684.240 | OEt | a2 | B17 | b2 | C01 | c1 | D01 | d1 | E02 |
| 2-1-m1E02-2-0089 | 684.273 | OEt | a1 | B05 | b1 | C12 | c1 | D01 | d1 | E02 |
| 2-1-m1E02-2-0090 | 685.221 | OEt | a2 | B12 | b3 | C15 | c1 | D01 | d1 | E02 |
| 2-1-mlE02-2-0091 | 685.255 | OEt | a1 | B04 | b2 | C01 | c1 | D01 | d1 | E02 |
| 2-1-m1E02-2-0092 | 685.271 | OEt | a1 | B07 | b1 | C08 | c1 | D01 | d1 | E02 |
| 2-1-mlE02-2-0093 | 686.252 | OEt | a1 | B06 | b1 | C12 | c1 | D01 | d1 | E02 |
| 2-1-m1E02-2-0094 | 686.256 | OEt | a2 | B11 | b1 | C14 | c1 | D01 | d1 | E02 |
| 2-1-mlE02-2-0095 | 686.266 | OEt | a1 | B07 | b1 | C11 | c1 | D01 | d1 | E02 |
| 2-1-mlE02-2-0096 | 686.277 | OEt | a2 | B11 | b2 | C33 | c3 | D02 | d1 | E02 |
| 2-1-m1E02-2-0097 | 686.310 | OEt | a2 | B15 | b2 | C02 | c1 | D01 | d1 | E02 |
| 2-1-mlE02-2-0098 | 687.252 | OEt | a2 | B18 | b1 | C12 | c1 | D01 | d1 | E02 |
| 2-1-m1E02-2-0099 | 687.252 | OEt | a2 | B12 | b1 | C14 | c1 | D01 | d1 | E02 |
| 2-1-mlE02-2-0100 | 687.262 | OEt | a1 | B07 | b1 | C10 | c1 | D01 | d1 | E02 |
| 2-1-m1E02-2-0101 | 687.273 | OEt | a2 | B12 | b2 | C33 | c3 | D02 | d1 | E02 |
| 2-1-m1E02-2-0102 | 687.306 | OEt | a2 | B15 | b2 | C03 | c1 | D01 | d1 | E02 |
| 2-1-mlE02-2-0103 | 687.306 | OEt | a2 | B13 | b2 | C05 | c1 | D01 | d1 | E02 |
| 2-1-m1E02-2-0104 | 688.290 | OEt | a2 | B16 | b2 | C02 | c1 | D01 | d1 | E02 |
| 2-1-mlE02-2-0105 | 689.285 | OEt | a2 | B16 | b2 | C03 | c1 | D01 | d1 | E02 |
| 2-1-m1E02-2-0106 | 690.285 | OEt | a2 | B13 | b2 | C04 | c1 | D01 | d1 | E02 |
| 2-1-m1E02-2-0107 | 690.342 | OEt | a2 | B11 | b2 | C07 | c1 | D01 | d1 | E02 |
| 2-1-mlE02-2-0108 | 691.281 | OEt | a2 | B14 | b2 | COS | c1 | D01 | d1 | E02 |
| 2-1-m1E02-2-0109 | 691.301 | OEt | a1 | B03 | b2 | C02 | c1 | D01 | d1 | E02 |
| 2-1-mlE02-2-0110 | 691.337 | OEt | a2 | B12 | b2 | C07 | c1 | D01 | d1 | E02 |
| 2-1-mlE02-2-0111 | 692.223 | OEt | a1 | B07 | b1 | C12 | c1 | D01 | d1 | E02 |
| 2-1-mlE02-2-0112 | 692.267 | OEt | a2 | B15 | b2 | C06 | c1 | D01 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0113 | 692.296 | OEt | a1 | B03 | b2 | C03 | c1 | D01 | d1 | E02 |
| 2-1-mlE02-2-0114 | 692.296 | OEt | a1 | B01 | b2 | C05 | c1 | D01 | d1 | E02 |
| 2-1-mlE02-2-0115 | 693.291 | OEt | a1 | B02 | b2 | COS | c1 | D01 | d1 | E02 |
| 2-1-mlE02-2-0116 | 694.246 | OEt | a2 | B16 | b2 | C06 | c1 | D01 | d1 | E02 |
| 2-1-mlE02-2-0117 | 694.246 | OEt | a2 | B11 | b3 | C08 | c1 | D01 | d1 | E02 |
| 2-1-m1E02-2-0118 | 694.246 | OEt | a2 | B11 | b1 | C08 | c2 | D02 | d1 | E02 |
| 2-1-m1E02-2-0119 | 694.260 | OEt | a2 | B17 | b2 | C02 | c1 | D01 | d1 | E02 |
| 2-1-m1E02-2-0120 | 694.260 | OEt | a2 | B14 | b2 | C04 | c1 | D01 | d1 | E02 |
| 2-1-mlE02-2-0121 | 695.241 | OEt | a2 | B12 | b3 | C08 | c1 | D01 | d1 | E02 |
| 2-1-mlE02-2-0122 | 695.241 | OEt | a2 | B12 | b1 | C08 | c2 | D02 | d1 | E02 |
| 2-1-m1E02-2-0123 | 695.241 | OEt | a2 | B11 | b1 | C11 | c2 | D02 | d1 | E02 |
| 2-1-mlE02-2-0124 | 695.256 | OEt | a2 | B17 | b2 | C03 | c1 | D01 | d1 | E02 |
| 2-1-m1E02-2-0125 | 695.276 | OEt | a1 | B04 | b2 | C02 | c1 | D01 | d1 | E02 |
| 2-1-m1E02-2-0126 | 695.276 | OEt | a1 | B01 | b2 | C04 | c1 | D01 | d1 | E02 |
| 2-1-m1E02-2-0127 | 695.296 | OEt | a1 | B05 | b2 | C01 | c1 | D01 | d1 | E02 |
| 2-1-mlE02-2-0128 | 696.237 | OEt | a2 | B11 | b1 | C10 | c2 | D02 | d1 | E02 |
| 2-1-m1E02-2-0129 | 696.237 | OEt | a2 | B12 | b1 | C11 | c2 | D02 | d1 | E02 |
| 2-1-mlE02-2-0130 | 696.271 | OEt | a1 | B02 | b2 | C04 | c1 | D01 | d1 | E02 |
| 2-1-mlE02-2-0131 | 696.271 | OEt | a1 | B04 | b2 | C03 | c1 | D01 | d1 | E02 |
| 2-1-mlE02-2-0132 | 696.306 | OEt | a2 | B13 | b1 | C13 | c1 | D01 | d1 | E02 |
| 2-1-mlE02-2-0133 | 697.232 | OEt | a2 | B12 | b1 | C10 | c2 | D02 | d1 | E02 |
| 2-1-m 1 E02-2-0134 | 697.257 | OEt | a1 | B03 | b2 | C06 | c1 | D01 | d1 | E02 |
| 2-1-mlE02-2-0135 | 697.275 | OEt | a1 | B06 | b2 | C01 | c1 | D01 | d1 | E02 |
| 2-1-mlE02-2-0136 | 698.241 | OEt | a2 | B13 | b3 | C15 | c1 | D01 | d1 | E02 |
| 2-1-mlE02-2-0137 | 698.272 | OEt | a2 | B19 | b1 | C08 | c1 | D01 | d1 | E02 |
| 2-1-mlE02-2-0138 | 698.272 | OEt | a2 | B11 | b1 | C09 | c1 | D01 | d1 | E02 |
| 2-1-mlE02-2-0139 | 698.274 | OEt | a2 | B18 | b2 | C01 | c1 | D01 | d1 | E02 |
| 2-1-m1E02-2-0140 | 699.267 | OEt | a2 | B12 | b1 | C09 | c1 | D01 | d1 | E02 |
| 2-1-mlE02-2-0141 | 699.267 | OEt | a2 | B19 | b1 | C11 | c1 | D01 | d1 | E02 |
| 2-1-mlE02-2-0142 | 700.203 | OEt | a2 | B11 | b3 | C06 | c1 | D01 | d1 | E02 |
| 2-1-mlE02-2-0143 | 700.217 | OEt | a2 | B17 | b2 | C06 | c1 | D01 | d1 | E02 |
| 2-1-m1E02-2-0144 | 700.262 | OEt | a2 | B19 | b1 | C10 | c1 | D01 | d1 | E02 |
| 2-1-mlE02-2-0145 | 700.272 | OEt | a2 | B13 | b1 | C14 | c1 | D01 | d1 | E02 |
| 2-1-mlE02-2-0146 | 700.281 | OEt | a2 | B14 | b1 | C13 | c1 | D01 | d1 | E02 |
| 2-1-mlE02-2-0147 | 700.293 | OEt | a2 | B13 | b2 | C33 | c3 | D02 | d1 | E02 |
| 2-1-mlE02-2-0148 | 701.198 | OEt | a2 | B12 | b3 | C06 | c1 | D01 | d1 | E02 |
| 2-1-mlE02-2-0149 | 701.198 | OEt | a2 | B11 | b1 | C12 | c2 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0150 | 701.232 | OEt | a1 | B04 | b2 | C06 | c1 | D01 | d1 | E02 |
| 2-1-m1E02-2-0151 | 701.296 | OEt | a1 | B01 | b1 | C13 | c1 | D01 | d1 | E02 |
| 2-1-mlE02-2-0152 | 701.321 | OEt | a2 | B15 | b2 | C05 | c1 | D01 | d1 | E02 |
| 2-1-mlE02-2-0153 | 702.193 | OEt | a2 | B12 | b1 | C12 | c2 | D02 | d1 | E02 |
| 2-1-mlE02-2-0154 | 702.216 | OEt | a2 | B14 | b3 | C15 | c1 | D01 | d1 | E02 |
| 2-1-mlE02-2-0155 | 702.291 | OEt | a1 | B02 | b1 | C13 | c1 | D01 | d1 | E02 |
| 2-1-mlE02-2-0156 | 703.231 | OEt | a1 | B01 | b3 | C15 | c1 | D01 | d1 | E02 |
| 2-1-mlE02-2-0157 | 703.245 | OEt | a1 | B07 | b2 | C01 | c1 | D01 | d1 | E02 |
| 2-1-mlE02-2-0158 | 703.301 | OEt | a2 | B16 | b2 | C05 | c1 | D01 | d1 | E02 |
| 2-1-mlE02-2-0159 | 704.226 | OEt | a1 | B02 | b3 | C15 | c1 | D01 | d1 | E02 |
| 2-1-m1E02-2-0160 | 704.247 | OEt | a2 | B14 | b1 | C14 | c1 | D01 | d1 | E02 |
| 2-1-mlE02-2-0161 | 704.268 | OEt | a2 | B14 | b2 | C33 | c3 | D02 | d1 | E02 |
| 2-1-m1E02-2-0162 | 704.301 | OEt | a2 | B15 | b2 | C04 | c1 | D01 | d1 | E02 |
| 2-1-m1E02-2-0163 | 704.357 | OEt | a2 | B13 | b2 | C07 | c1 | D01 | d1 | E02 |
| 2-1-m1E02-2-0164 | 705.223 | OEt | a2 | B19 | b1 | C12 | c1 | D01 | d1 | E02 |
| 2-1-m1E02-2-0165 | 705.262 | OEt | a1 | B01 | b1 | C14 | c1 | D01 | d1 | E02 |
| 2-1-m1E02-2-0166 | 705.283 | OEt | a1 | B01 | b2 | C33 | c3 | D02 | d1 | E02 |
| 2-1-m1E02-2-0167 | 705.316 | OEt | a1 | B05 | b2 | C02 | c1 | D01 | d1 | E02 |
| 2-1-mlE02-2-0168 | 706.257 | OEt | a1 | B08 | b1 | C08 | c1 | D01 | d1 | E02 |
| 2-1-m1E02-2-0169 | 706.257 | OEt | a1 | B02 | b1 | C14 | c1 | D01 | d1 | E02 |
| 2-1-mlE02-2-0170 | 706.278 | OEt | a1 | B02 | b2 | C33 | c3 | D02 | d1 | E02 |
| 2-1-mlE02-2-0171 | 706.280 | OEt | a2 | B16 | b2 | C04 | c1 | D01 | d1 | E02 |
| 2-1-mlE02-2-0172 | 706.283 | OEt | a2 | B11 | b1 | C30 | c3 | D02 | d1 | E02 |
| 2-1-mlE02-2-0173 | 706.311 | OEt | a1 | B05 | b2 | C03 | c1 | D01 | d1 | E02 |
| 2-1-mlE02-2-0174 | 706.311 | OEt | a1 | B03 | b2 | COS | c1 | D01 | d1 | E02 |
| 2-1-mlE02-2-0175 | 707.253 | OEt | a1 | B08 | b1 | C11 | c1 | D01 | d1 | E02 |
| 2-1-mlE02-2-0176 | 707.278 | OEt | a2 | B12 | b1 | C30 | c3 | D02 | d1 | E02 |
| 2-1-mlE02-2-0177 | 707.296 | OEt | a1 | B06 | b2 | C02 | c1 | D01 | d1 | E02 |
| 2-1-mlE02-2-0178 | 708.248 | OEt | a1 | B08 | b1 | C10 | c1 | D01 | d1 | E02 |
| 2-1-mlE02-2-0179 | 708.262 | OEt | a2 | B13 | b3 | C08 | c1 | D01 | d1 | E02 |
| 2-1-mlE02-2-0180 | 708.262 | OEt | a2 | B13 | b1 | C08 | c2 | D02 | d1 | E02 |
| 2-1-mlE02-2-0181 | 708.262 | OEt | a2 | B11 | b2 | C20 | c3 | D02 | d1 | E02 |
| 2-1-m1E02-2-0182 | 708.291 | OEt | a1 | B06 | b2 | C03 | c1 | D01 | d1 | E02 |
| 2-1-mlE02-2-0183 | 708.295 | OEt | a2 | B18 | b2 | C02 | c1 | D01 | d1 | E02 |
| 2-1-m1E02-2-0184 | 708.332 | OEt | a2 | B14 | b2 | C07 | c1 | D01 | d1 | E02 |
| 2-1-mlE02-2-0185 | 709.257 | OEt | a2 | B13 | b1 | C11 | c2 | D02 | d1 | E02 |
| 2-1-mlE02-2-0186 | 709.257 | OEt | a2 | B12 | b2 | C20 | c3 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0187 | 709.257 | OEt | a2 | B11 | b2 | C28 | c3 | D02 | d1 | E02 |
| 2-1-m1E02-2-0188 | 709.271 | OEt | a2 | B17 | b2 | COS | c1 | D01 | d1 | E02 |
| 2-1-mlE02-2-0189 | 709.290 | OEt | a2 | B18 | b2 | C03 | c1 | D01 | d1 | E02 |
| 2-1-m1E02-2-0190 | 709.291 | OEt | a1 | B03 | b2 | C04 | c1 | D01 | d1 | E02 |
| 2-1-mlE02-2-0191 | 709.348 | OEt | a1 | B01 | b2 | C07 | c1 | D01 | d1 | E02 |
| 2-1-mlE02-2-0192 | 710.252 | OEt | a2 | B13 | b1 | C10 | c2 | D02 | d1 | E02 |
| 2-1-mlE02-2-0193 | 710.252 | OEt | a2 | B12 | b2 | C28 | c3 | D02 | d1 | E02 |
| 2-1-mlE02-2-0194 | 710.286 | OEt | a1 | B04 | b2 | C05 | c1 | D01 | d1 | E02 |
| 2-1-mlE02-2-0195 | 710.322 | OEt | a2 | B15 | b1 | C13 | c1 | D01 | d1 | E02 |
| 2-1-mlE02-2-0196 | 710.343 | OEt | a1 | B02 | b2 | C07 | c1 | D01 | d1 | E02 |
| 2-1-mlE02-2-0197 | 711.273 | OEt | a1 | B05 | b2 | C06 | c1 | D01 | d1 | E02 |
| 2-1-mlE02-2-0198 | 712.220 | OEt | a2 | B11 | b2 | C01 | c2 | D02 | d1 | E02 |
| 2-1-mlE02-2-0199 | 712.237 | OEt | a2 | B14 | b3 | C08 | c1 | D01 | d1 | E02 |
| 2-1-m1E02-2-0200 | 712.237 | OEt | a2 | B14 | b1 | C08 | c2 | D02 | d1 | E02 |
| 2-1-m1E02-2-0201 | 712.251 | OEt | a2 | B17 | b2 | C04 | c1 | D01 | d1 | E02 |
| 2-1-m1E02-2-0202 | 712.257 | OEt | a2 | B15 | b3 | C15 | c1 | D01 | d1 | E02 |
| 2-1-mlE02-2-0203 | 712.287 | OEt | a2 | B13 | b1 | C09 | c1 | D01 | d1 | E02 |
| 2-1-m1E02-2-0204 | 712.301 | OEt | a2 | B16 | b1 | C13 | c1 | D01 | d1 | E02 |
| 2-1-mlE02-2-0205 | 713.209 | OEt | a1 | B08 | b1 | C12 | c1 | D01 | d1 | E02 |
| 2-1-mlE02-2-0206 | 713.216 | OEt | a2 | B12 | b2 | C01 | c2 | D02 | d1 | E02 |
| 2-1-mlE02-2-0207 | 713.232 | OEt | a2 | B14 | b1 | C11 | c2 | D02 | d1 | E02 |
| 2-1-mlE02-2-0208 | 713.252 | OEt | a1 | B06 | b2 | C06 | c1 | D01 | d1 | E02 |
| 2-1-mlE02-2-0209 | 713.252 | OEt | a1 | B01 | b3 | C08 | c1 | D01 | d1 | E02 |
| 2-1-mlE02-2-0210 | 713.252 | OEt | a1 | B01 | b1 | C08 | c2 | D02 | d1 | E02 |
| 2-1-mlE02-2-0211 | 713.266 | OEt | a1 | B07 | b2 | C02 | c1 | D01 | d1 | E02 |
| 2-1-mlE02-2-0212 | 713.266 | OEt | a1 | B04 | b2 | C04 | c1 | D01 | d1 | E02 |
| 2-1-mlE02-2-0213 | 714.218 | OEt | a2 | B13 | b3 | C06 | c1 | D01 | d1 | E02 |
| 2-1-m1E02-2-0214 | 714.227 | OEt | a2 | B14 | b1 | C10 | c2 | D02 | d1 | E02 |
| 2-1-mlE02-2-0215 | 714.236 | OEt | a2 | B16 | b3 | C15 | c1 | D01 | d1 | E02 |
| 2-1-mlE02-2-0216 | 714.247 | OEt | a1 | B02 | b3 | C08 | c1 | D01 | d1 | E02 |
| 2-1-mlE02-2-0217 | 714.247 | OEt | a1 | B02 | b1 | C08 | c2 | D02 | d1 | E02 |
| 2-1-m1E02-2-0218 | 714.247 | OEt | a1 | B01 | b1 | C11 | c2 | D02 | d1 | E02 |
| 2-1-mlE02-2-0219 | 714.251 | OEt | a2 | B18 | b2 | C06 | c1 | D01 | d1 | E02 |
| 2-1-m1E02-2-0220 | 714.261 | OEt | a1 | B07 | b2 | C03 | c1 | D01 | d1 | E02 |
| 2-1-mlE02-2-0221 | 714.267 | OEt | a2 | B20 | b1 | C08 | c1 | D01 | d1 | E02 |
| 2-1-m1E02-2-0222 | 714.288 | OEt | a2 | B15 | b1 | C14 | c1 | D01 | d1 | E02 |
| 2-1-mlE02-2-0223 | 714.309 | OEt | a2 | B15 | b2 | C33 | c3 | D02 | d1 | E02 |

(continued)

| [Table 8-10-3] Compound that may be contained in mixture 2-1-mIE02-2 | | | | | | | | | | |
|---|---|---|---|---|---|---|---|---|---|---|
| ID | Exact Mass | | a | B | b | C | c | D | d | E |
| 2-1-mIE02-2-0224 | 715.213 | OEt | a2 | B13 | b1 | C12 | c2 | D02 | d1 | E02 |
| 2-1-mIE02-2-0225 | 715.242 | OEt | a1 | B01 | b1 | C10 | c2 | D02 | d1 | E02 |
| 2-1-mIE02-2-0226 | 715.242 | OEt | a1 | B02 | b1 | C11 | c2 | D02 | d1 | E02 |
| 2-1-mIE02-2-0227 | 715.262 | OEt | a2 | B20 | b1 | C11 | c1 | D01 | d1 | E02 |
| 2-1-mIE02-2-0228 | 715.312 | OEt | a1 | B03 | b1 | C13 | c1 | D01 | d1 | E02 |
| 2-1-mIE02-2-0229 | 716.238 | OEt | a1 | B02 | b1 | C10 | c2 | D02 | d1 | E02 |
| 2-1-m1E02-2-0230 | 716.246 | OEt | a2 | B19 | b2 | C01 | c1 | D01 | d1 | E02 |
| 2-1-m1E02-2-0231 | 716.257 | OEt | a2 | B20 | b1 | C10 | c1 | D01 | d1 | E02 |
| 2-1-mIE02-2-0232 | 716.262 | OEt | a2 | B14 | b1 | C09 | c1 | D01 | d1 | E02 |
| 2-1-mIE02-2-0233 | 716.267 | OEt | a2 | B16 | b1 | C14 | c1 | D01 | d1 | E02 |
| 2-1-m1E02-2-0234 | 716.288 | OEt | a2 | B16 | b2 | C33 | c3 | D02 | d1 | E02 |
| 2-1-mIE02-2-0235 | 717.247 | OEt | a1 | B03 | b3 | C15 | c1 | D01 | d1 | E02 |
| 2-1-mIE02-2-0236 | 717.277 | OEt | a1 | B09 | b1 | C08 | c1 | D01 | d1 | E02 |
| 2-1-mIE02-2-0237 | 717.277 | OEt | a1 | B01 | b1 | C09 | c1 | D01 | d1 | E02 |
| 2-1-mIE02-2-0238 | 718.193 | OEt | a2 | B14 | b3 | C06 | c1 | D01 | d1 | E02 |
| 2-1-mIE02-2-0239 | 718.272 | OEt | a2 | B17 | b1 | C13 | c1 | D01 | d1 | E02 |
| 2-1-m1E02-2-0240 | 718.273 | OEt | a1 | B02 | b1 | C09 | c1 | D01 | d1 | E02 |
| 2-1-m1E02-2-0241 | 718.273 | OEt | a1 | B09 | b1 | C11 | c1 | D01 | d1 | E02 |
| 2-1-mIE02-2-0242 | 718.373 | OEt | a2 | B15 | b2 | C07 | c1 | D01 | d1 | E02 |
| 2-1-mIE02-2-0243 | 719.188 | OEt | a2 | B14 | b1 | C12 | c2 | D02 | d1 | E02 |
| 2-1-mIE02-2-0244 | 719.208 | OEt | a1 | B01 | b3 | C06 | c1 | D01 | d1 | E02 |
| 2-1-mIE02-2-0245 | 719.223 | OEt | a1 | B07 | b2 | C06 | c1 | D01 | d1 | E02 |
| 2-1-mIE02-2-0246 | 719.268 | OEt | a1 | B09 | b1 | C10 | c1 | D01 | d1 | E02 |
| 2-1-mIE02-2-0247 | 719.278 | OEt | a1 | B03 | b1 | C14 | c1 | D01 | d1 | E02 |
| 2-1-mIE02-2-0248 | 719.287 | OEt | a1 | B04 | b1 | C13 | c1 | D01 | d1 | E02 |
| 2-1-mIE02-2-0249 | 719.299 | OEt | a1 | B03 | b2 | C33 | c3 | D02 | d1 | E02 |
| 2-1-mIE02-2-0250 | 720.204 | OEt | a1 | B02 | b3 | C06 | c1 | D01 | d1 | E02 |
| 2-1-mIE02-2-0251 | 720.204 | OEt | a1 | B01 | b1 | C12 | c2 | D02 | d1 | E02 |
| 2-1-mIE02-2-0252 | 720.207 | OEt | a2 | B17 | b3 | C15 | c1 | D01 | d1 | E02 |
| 2-1-mIE02-2-0253 | 720.298 | OEt | a2 | B13 | b1 | C30 | c3 | D02 | d1 | E02 |
| 2-1-mIE02-2-0254 | 720.298 | OEt | a2 | B11 | b1 | C32 | c3 | D02 | d1 | E02 |
| 2-1-mIE02-2-0255 | 720.327 | OEt | a1 | B05 | b2 | C05 | c1 | D01 | d1 | E02 |
| 2-1-mIE02-2-0256 | 720.352 | OEt | a2 | B16 | b2 | C07 | c1 | D01 | d1 | E02 |
| 2-1-mIE02-2-0257 | 721.199 | OEt | a1 | B02 | b1 | C12 | c2 | D02 | d1 | E02 |
| 2-1-mIE02-2-0258 | 721.218 | OEt | a2 | B20 | b1 | C12 | c1 | D01 | d1 | E02 |
| 2-1-mIE02-2-0259 | 721.222 | OEt | a1 | B04 | b3 | C15 | c1 | D01 | d1 | E02 |
| 2-1-mIE02-2-0260 | 721.293 | OEt | a2 | B12 | b1 | C32 | c3 | D02 | d1 | E02 |

(continued)

| [Table 8-10-3] Compound that may be contained in mixture 2-1-mIE02-2 | | | | | | | | | | |
|---|---|---|---|---|---|---|---|---|---|---|
| ID | Exact Mass | | a | B | b | C | c | D | d | E |
| 2-1-mIE02-2-0261 | 722.237 | OEt | a2 | B17 | b1 | C14 | c1 | D01 | d1 | E02 |
| 2-1-mIE02-2-0262 | 722.241 | OEt | a2 | B11 | b2 | C02 | c2 | D02 | d1 | E02 |
| 2-1-mIE02-2-0263 | 722.259 | OEt | a2 | B17 | b2 | C33 | c3 | D02 | d1 | E02 |
| 2-1-mIE02-2-0264 | 722.277 | OEt | a2 | B15 | b3 | C08 | c1 | D01 | d1 | E02 |
| 2-1-mIE02-2-0265 | 722.277 | OEt | a2 | B15 | b1 | C08 | c2 | D02 | d1 | E02 |
| 2-1-mIE02-2-0266 | 722.277 | OEt | a2 | B13 | b2 | C20 | c3 | D02 | d1 | E02 |
| 2-1-mIE02-2-0267 | 722.277 | OEt | a2 | B11 | b2 | C22 | c3 | D02 | d1 | E02 |
| 2-1-mIE02-2-0268 | 722.306 | OEt | a1 | B06 | b2 | C05 | c1 | D01 | d1 | E02 |
| 2-1-mIE02-2-0269 | 723.236 | OEt | a2 | B12 | b2 | C02 | c2 | D02 | d1 | E02 |
| 2-1-mIE02-2-0270 | 723.236 | OEt | a2 | B11 | b2 | C03 | c2 | D02 | d1 | E02 |
| 2-1-mIE02-2-0271 | 723.253 | OEt | a1 | B04 | b1 | C14 | c1 | D01 | d1 | E02 |
| 2-1-mIE02-2-0272 | 723.273 | OEt | a2 | B15 | b1 | C11 | c2 | D02 | d1 | E02 |
| 2-1-mIE02-2-0273 | 723.273 | OEt | a2 | B12 | b2 | C22 | c3 | D02 | d1 | E02 |
| 2-1-mIE02-2-0274 | 723.273 | OEt | a2 | B13 | b2 | C28 | c3 | D02 | d1 | E02 |
| 2-1-mIE02-2-0275 | 723.274 | OEt | a1 | B04 | b2 | C33 | c3 | D02 | d1 | E02 |
| 2-1-mIE02-2-0276 | 723.306 | OEt | a2 | B18 | b2 | C05 | c1 | D01 | d1 | E02 |
| 2-1-mIE02-2-0277 | 723.307 | OEt | a1 | B05 | b2 | C04 | c1 | D01 | d1 | E02 |
| 2-1-mIE02-2-0278 | 723.363 | OEt | a1 | B03 | b2 | C07 | c1 | D01 | d1 | E02 |
| 2-1-mIE02-2-0279 | 724.229 | OEt | a1 | B09 | b1 | C12 | c1 | D01 | d1 | E02 |
| 2-1-mIE02-2-0280 | 724.232 | OEt | a1 | B08 | b2 | C01 | c1 | D01 | d1 | E02 |
| 2-1-mIE02-2-0281 | 724.232 | OEt | a2 | B12 | b2 | C03 | c2 | D02 | d1 | E02 |
| 2-1-mIE02-2-0282 | 724.257 | OEt | a2 | B16 | b3 | C08 | c1 | D01 | d1 | E02 |
| 2-1-mIE02-2-0283 | 724.257 | OEt | a2 | B16 | b1 | C08 | c2 | D02 | d1 | E02 |
| 2-1-mIE02-2-0284 | 724.268 | OEt | a2 | B15 | b1 | C10 | c2 | D02 | d1 | E02 |
| 2-1-mIE02-2-0285 | 724.273 | OEt | a2 | B14 | b1 | C30 | c3 | D02 | d1 | E02 |
| 2-1-mIE02-2-0286 | 725.252 | OEt | a2 | B16 | b1 | C11 | c2 | D02 | d1 | E02 |
| 2-1-mIE02-2-0287 | 725.286 | OEt | a1 | B06 | b2 | C04 | c1 | D01 | d1 | E02 |
| 2-1-mIE02-2-0288 | 725.288 | OEt | a1 | B01 | b1 | C30 | c3 | D02 | d1 | E02 |
| 2-1-mIE02-2-0289 | 726.236 | OEt | a2 | B13 | b2 | C01 | c2 | D02 | d1 | E02 |
| 2-1-mIE02-2-0290 | 726.247 | OEt | a2 | B16 | b1 | C10 | c2 | D02 | d1 | E02 |
| 2-1-mIE02-2-0291 | 726.252 | OEt | a2 | B14 | b2 | C20 | c3 | D02 | d1 | E02 |
| 2-1-mIE02-2-0292 | 726.252 | OEt | a2 | B11 | b2 | C24 | c3 | D02 | d1 | E02 |
| 2-1-mIE02-2-0293 | 726.267 | OEt | a2 | B19 | b2 | C02 | c1 | D01 | d1 | E02 |
| 2-1-mIE02-2-0294 | 726.284 | OEt | a1 | B02 | b1 | C30 | c3 | D02 | d1 | E02 |
| 2-1-mIE02-2-0295 | 726.285 | OEt | a2 | B18 | b2 | C04 | c1 | D01 | d1 | E02 |
| 2-1-mIE02-2-0296 | 726.303 | OEt | a2 | B15 | b1 | C09 | c1 | D01 | d1 | E02 |
| 2-1-mIE02-2-0297 | 726.323 | OEt | a2 | B17 | b2 | C07 | c1 | D01 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0298 | 727.248 | OEt | a2 | B12 | b2 | C24 | c3 | D02 | d1 | E02 |
| 2-1-mlE02-2-0299 | 727.248 | OEt | a2 | B14 | b2 | C28 | c3 | D02 | d1 | E02 |
| 2-1-mlE02-2-0300 | 727.262 | OEt | a2 | B19 | b2 | C03 | c1 | D01 | d1 | E02 |
| 2-1-m1E02-2-0301 | 727.268 | OEt | a1 | B03 | b3 | C08 | c1 | D01 | d1 | E02 |
| 2-1-m1E02-2-0302 | 727.268 | OEt | a1 | B03 | b1 | C08 | c2 | D02 | d1 | E02 |
| 2-1-m1E02-2-0303 | 727.268 | OEt | a1 | B01 | b2 | C20 | c3 | D02 | d1 | E02 |
| 2-1-m1E02-2-0304 | 727.338 | OEt | a1 | B04 | b2 | C07 | c1 | D01 | d1 | E02 |
| 2-1-m1E02-2-0305 | 728.197 | OEt | a2 | B11 | b2 | C06 | c2 | D02 | d1 | E02 |
| 2-1-m1E02-2-0306 | 728.234 | OEt | a2 | B15 | b3 | C06 | c1 | D01 | d1 | E02 |
| 2-1-mlE02-2-0307 | 728.263 | OEt | a1 | B03 | b1 | C11 | c2 | D02 | d1 | E02 |
| 2-1-m1E02-2-0308 | 728.263 | OEt | a1 | B02 | b2 | C20 | c3 | D02 | d1 | E02 |
| 2-1-mlE02-2-0309 | 728.263 | OEt | a1 | B01 | b2 | C28 | c3 | D02 | d1 | E02 |
| 2-1-mlE02-2-0310 | 728.277 | OEt | a1 | B07 | b2 | COS | c1 | D01 | d1 | E02 |
| 2-1-mlE02-2-0311 | 728.282 | OEt | a2 | B16 | b1 | C09 | c1 | D01 | d1 | E02 |
| 2-1-mlE02-2-0312 | 729.193 | OEt | a2 | B12 | b2 | C06 | c2 | D02 | d1 | E02 |
| 2-1-mlE02-2-0313 | 729.229 | OEt | a2 | B15 | b1 | C12 | c2 | D02 | d1 | E02 |
| 2-1-m1E02-2-0314 | 729.258 | OEt | a1 | B03 | b1 | C10 | c2 | D02 | d1 | E02 |
| 2-1-mlE02-2-0315 | 729.258 | OEt | a1 | B02 | b2 | C28 | c3 | D02 | d1 | E02 |
| 2-1-mlE02-2-0316 | 729.327 | OEt | a1 | B05 | b1 | C13 | c1 | D01 | d1 | E02 |
| 2-1-mlE02-2-0317 | 730.211 | OEt | a2 | B14 | b2 | C01 | c2 | D02 | d1 | E02 |
| 2-1-mlE02-2-0318 | 730.213 | OEt | a2 | B16 | b3 | C06 | c1 | D01 | d1 | E02 |
| 2-1-mlE02-2-0319 | 730.227 | OEt | a2 | B17 | b3 | C08 | c1 | D01 | d1 | E02 |
| 2-1-mlE02-2-0320 | 730.227 | OEt | a2 | B17 | b1 | C08 | c2 | D02 | d1 | E02 |
| 2-1-mlE02-2-0321 | 731.208 | OEt | a2 | B16 | b1 | C12 | c2 | D02 | d1 | E02 |
| 2-1-mlE02-2-0322 | 731.223 | OEt | a2 | B17 | b1 | C11 | c2 | D02 | d1 | E02 |
| 2-1-mlE02-2-0323 | 731.226 | OEt | a1 | B01 | b2 | C01 | c2 | D02 | d1 | E02 |
| 2-1-mlE02-2-0324 | 731.243 | OEt | a1 | B04 | b3 | C08 | c1 | D01 | d1 | E02 |
| 2-1-mlE02-2-0325 | 731.243 | OEt | a1 | B04 | b1 | C08 | c2 | D02 | d1 | E02 |
| 2-1-mlE02-2-0326 | 731.257 | OEt | a1 | B07 | b2 | C04 | c1 | D01 | d1 | E02 |
| 2-1-mlE02-2-0327 | 731.262 | OEt | a1 | B05 | b3 | C15 | c1 | D01 | d1 | E02 |
| 2-1-mlE02-2-0328 | 731.293 | OEt | a1 | B03 | b1 | C09 | c1 | D01 | d1 | E02 |
| 2-1-mlE02-2-0329 | 731.307 | OEt | a1 | B06 | b1 | C13 | c1 | D01 | d1 | E02 |
| 2-1-mlE02-2-0330 | 732.218 | OEt | a2 | B17 | b1 | C10 | c2 | D02 | d1 | E02 |
| 2-1-mlE02-2-0331 | 732.221 | OEt | a1 | B02 | b2 | C01 | c2 | D02 | d1 | E02 |
| 2-1-mlE02-2-0332 | 732.223 | OEt | a2 | B19 | b2 | C06 | c1 | D01 | d1 | E02 |
| 2-1-mlE02-2-0333 | 732.238 | OEt | a1 | B04 | b1 | C1 | c2 | D02 | d1 | E02 |
| 2-1-mlE02-2-0334 | 732.241 | OEt | a2 | B20 | b2 | C01 | c1 | D01 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0335 | 732.306 | OEt | a2 | B18 | b1 | C13 | c1 | D01 | d1 | E02 |
| 2-1-mlE02-2-0336 | 733.224 | OEt | a1 | B03 | b3 | C06 | c1 | D01 | d1 | E02 |
| 2-1-mlE02-2-0337 | 733.233 | OEt | a1 | B04 | b1 | C10 | c2 | D02 | d1 | E02 |
| 2-1-mlE02-2-0338 | 733.242 | OEt | a1 | B06 | b3 | C15 | c1 | D01 | d1 | E02 |
| 2-1-mlE02-2-0339 | 733.272 | OEt | a1 | B10 | b1 | C08 | c1 | D01 | d1 | E02 |
| 2-1-mlE02-2-0340 | 733.293 | OEt | a1 | B05 | b1 | C14 | c1 | D01 | d1 | E02 |
| 2-1-mlE02-2-0341 | 733.315 | OEt | a1 | B05 | b2 | C33 | c3 | D02 | d1 | E02 |
| 2-1-mlE02-2-0342 | 734.219 | OEt | a1 | B03 | b1 | C12 | c2 | D02 | d1 | E02 |
| 2-1-mlE02-2-0343 | 734.241 | OEt | a2 | B18 | b3 | C15 | c1 | D01 | d1 | E02 |
| 2-1-mlE02-2-0344 | 734.252 | OEt | a1 | B08 | b2 | C02 | c1 | D01 | d1 | E02 |
| 2-1-mlE02-2-0345 | 734.253 | OEt | a2 | B17 | b1 | C09 | c1 | D01 | d1 | E02 |
| 2-1-mlE02-2-0346 | 734.268 | OEt | a1 | B10 | b1 | C11 | c1 | D01 | d1 | E02 |
| 2-1-mlE02-2-0347 | 734.314 | OEt | a2 | B15 | b1 | C30 | c3 | D02 | d1 | E02 |
| 2-1-mlE02-2-0348 | 734.314 | OEt | a2 | B11 | b1 | C29 | c3 | D02 | d1 | E02 |
| 2-1-mlE02-2-0349 | 734.314 | OEt | a2 | B13 | b1 | C32 | c3 | D02 | d1 | E02 |
| 2-1-mlE02-2-0350 | 735.248 | OEt | a1 | B08 | b2 | C03 | c1 | D01 | d1 | E02 |
| 2-1-mlE02-2-0351 | 735.252 | OEt | a1 | B09 | b2 | C01 | c1 | D01 | d1 | E02 |
| 2-1-mlE02-2-0352 | 735.263 | OEt | a1 | B10 | b1 | C10 | c1 | D01 | d1 | E02 |
| 2-1-mlE02-2-0353 | 735.268 | OEt | a1 | B04 | b1 | C09 | c1 | D01 | d1 | E02 |
| 2-1-mlE02-2-0354 | 735.273 | OEt | a1 | B06 | b1 | C14 | c1 | D01 | d1 | E02 |
| 2-1-mlE02-2-0355 | 735.294 | OEt | a1 | B06 | b2 | C33 | c3 | D02 | d1 | E02 |
| 2-1-mlE02-2-0356 | 735.309 | OEt | a2 | B12 | b1 | C29 | c3 | D02 | d1 | E02 |
| 2-1-mlE02-2-0357 | 736.184 | OEt | a2 | B17 | b3 | C06 | c1 | D01 | d1 | E02 |
| 2-1-mlE02-2-0358 | 736.257 | OEt | a2 | B13 | b2 | C02 | c2 | D02 | d1 | E02 |
| 2-1-mlE02-2-0359 | 736.272 | OEt | a2 | B18 | b1 | C14 | c1 | D01 | d1 | E02 |
| 2-1-mlE02-2-0360 | 736.293 | OEt | a2 | B15 | b2 | C20 | c3 | D02 | d1 | E02 |
| 2-1-mlE02-2-0361 | 736.293 | OEt | a2 | B13 | b2 | C22 | c3 | D02 | d1 | E02 |
| 2-1-mlE02-2-0362 | 736.293 | OEt | a2 | B16 | b1 | C30 | c3 | D02 | d1 | E02 |
| 2-1-mlE02-2-0363 | 736.293 | OEt | a2 | B18 | b2 | C33 | c3 | D02 | d1 | E02 |
| 2-1-mlE02-2-0364 | 737.179 | OEt | a2 | B17 | b1 | C12 | c2 | D02 | d1 | E02 |
| 2-1-mlE02-2-0365 | 737.199 | OEt | a1 | B04 | b3 | C06 | c1 | D01 | d1 | E02 |
| 2-1-mlE02-2-0366 | 737.252 | OEt | a2 | B13 | b2 | C03 | c2 | D02 | d1 | E02 |
| 2-1-mlE02-2-0367 | 737.252 | OEt | a2 | B11 | b2 | C05 | c2 | D02 | d1 | E02 |
| 2-1-mlE02-2-0368 | 737.277 | OEt | a1 | B07 | b1 | C13 | c1 | D01 | d1 | E02 |
| 2-1-m 1 E02-2-0369 | 737.288 | OEt | a2 | B15 | b2 | C28 | c3 | D02 | d1 | E02 |
| 2-1-m1E02-2-0370 | 737.379 | OEt | a1 | B05 | b2 | C07 | c1 | D01 | d1 | E02 |
| 2-1-m1E02-2-0371 | 738.194 | OEt | a1 | B04 | b1 | C12 | c2 | D02 | d1 | E02 |

(continued)

| [Table 8-10-3] Compound that may be contained in mixture 2-1-mIE02-2 | | | | | | | | | | |
|---|---|---|---|---|---|---|---|---|---|---|
| ID | Exact Mass | | a | B | b | C | c | D | d | E |
| 2-1-m1E02-2-0372 | 738.247 | OEt | a2 | B12 | b2 | COS | c2 | D02 | d1 | E02 |
| 2-1-m1E02-2-0373 | 738.272 | OEt | a2 | B16 | b2 | C20 | c3 | D02 | d1 | E02 |
| 2-1-m1E02-2-0374 | 738.272 | OEt | a2 | B11 | b2 | C26 | c3 | D02 | d1 | E02 |
| 2-1-mIE02-2-0375 | 738.289 | OEt | a2 | B14 | b1 | C32 | c3 | D02 | d1 | E02 |
| 2-1-mIE02-2-0376 | 739.212 | OEt | a1 | B07 | b3 | C15 | c1 | D01 | d1 | E02 |
| 2-1-m1E02-2-0377 | 739.268 | OEt | a2 | B12 | b2 | C26 | c3 | D02 | d1 | E02 |
| 2-1-m1E02-2-0378 | 739.268 | OEt | a2 | B16 | b2 | C28 | c3 | D02 | d1 | E02 |
| 2-1-m1E02-2-0379 | 739.304 | OEt | a1 | B03 | b1 | C30 | c3 | D02 | d1 | E02 |
| 2-1-mIE02-2-0380 | 739.304 | OEt | a1 | B01 | b1 | C32 | c3 | D02 | d1 | E02 |
| 2-1-mIE02-2-0381 | 739.358 | OEt | a1 | B06 | b2 | C07 | c1 | D01 | d1 | E02 |
| 2-1-mIE02-2-0382 | 740.209 | OEt | a1 | B08 | b2 | C06 | c1 | D01 | d1 | E02 |
| 2-1-mIE02-2-0383 | 740.224 | OEt | a1 | B10 | b1 | C12 | c1 | D01 | d1 | E02 |
| 2-1-mIE02-2-0384 | 740.232 | OEt | a2 | B14 | b2 | C02 | c2 | D02 | d1 | E02 |
| 2-1-mIE02-2-0385 | 740.232 | OEt | a2 | B11 | b2 | C04 | c2 | D02 | d1 | E02 |
| 2-1-mIE02-2-0386 | 740.252 | OEt | a2 | B15 | b2 | C01 | c2 | D02 | d1 | E02 |
| 2-1-mIE02-2-0387 | 740.268 | OEt | a2 | B14 | b2 | C22 | c3 | D02 | d1 | E02 |
| 2-1-mIE02-2-0388 | 740.268 | OEt | a2 | B13 | b2 | C24 | c3 | D02 | d1 | E02 |
| 2-1-mIE02-2-0389 | 740.299 | OEt | a1 | B02 | b1 | C32 | c3 | D02 | d1 | E02 |
| 2-1-mIE02-2-0390 | 740.357 | OEt | a2 | B18 | b2 | C07 | c1 | D01 | d1 | E02 |
| 2-1-mIE02-2-0391 | 741.227 | OEt | a2 | B12 | b2 | C04 | c2 | D02 | d1 | E02 |
| 2-1-mIE02-2-0392 | 741.227 | OEt | a2 | B14 | b2 | C03 | c2 | D02 | d1 | E02 |
| 2-1-mIE02-2-0393 | 741.243 | OEt | a1 | B07 | b1 | C14 | c1 | D01 | d1 | E02 |
| 2-1-mIE02-2-0394 | 741.247 | OEt | a1 | B01 | b2 | C02 | c2 | D02 | d1 | E02 |
| 2-1-mIE02-2-0395 | 741.264 | OEt | a1 | B07 | b2 | C33 | c3 | D02 | d1 | E02 |
| 2-1-mIE02-2-0396 | 741.277 | OEt | a2 | B19 | b2 | C05 | c1 | D01 | d1 | E02 |
| 2-1-mIE02-2-0397 | 741.283 | OEt | a1 | B05 | b3 | C08 | c1 | D01 | d1 | E02 |
| 2-1-mIE02-2-0398 | 741.283 | OEt | a1 | B05 | b1 | C08 | c2 | D02 | d1 | E02 |
| 2-1-mIE02-2-0399 | 741.283 | OEt | a1 | B03 | b2 | C20 | c3 | D02 | d1 | E02 |
| 2-1-mIE02-2-0400 | 741.283 | OEt | a1 | B01 | b2 | C22 | c3 | D02 | d1 | E02 |
| 2-1-mIE02-2-0401 | 742.213 | OEt | a2 | B13 | b2 | C06 | c2 | D02 | d1 | E02 |
| 2-1-mIE02-2-0402 | 742.231 | OEt | a2 | B16 | b2 | C01 | c2 | D02 | d1 | E02 |
| 2-1-mIE02-2-0403 | 742.242 | OEt | a1 | B02 | b2 | C02 | c2 | D02 | d1 | E02 |
| 2-1-mIE02-2-0404 | 742.242 | OEt | a1 | B01 | b2 | C03 | c2 | D02 | d1 | E02 |
| 2-1-mIE02-2-0405 | 742.261 | OEt | a2 | B20 | b2 | C02 | c1 | D01 | d1 | E02 |
| 2-1-mIE02-2-0406 | 742.264 | OEt | a2 | B17 | b1 | C30 | c3 | D02 | d1 | E02 |
| 2-1-mIE02-2-0407 | 742.278 | OEt | a1 | B05 | b1 | C11 | c2 | D02 | d1 | E02 |
| 2-1-mIE02-2-0408 | 742.278 | OEt | a1 | B02 | b2 | C22 | c3 | D02 | d1 | E02 |

(continued)

| [Table 8-10-3] Compound that may be contained in mixture 2-1-mIE02-2 | | | | | | | | | | |
|---|---|---|---|---|---|---|---|---|---|---|
| ID | Exact Mass | | a | B | b | C | c | D | d | E |
| 2-1-mIE02-2-0409 | 742.278 | OEt | a1 | B03 | b2 | C28 | c3 | D02 | d1 | E02 |
| 2-1-mIE02-2-0410 | 743.237 | OEt | a1 | B02 | b2 | C03 | c2 | D02 | d1 | E02 |
| 2-1-mIE02-2-0411 | 743.257 | OEt | a2 | B20 | b2 | C03 | c1 | D01 | d1 | E02 |
| 2-1-mIE02-2-0412 | 743.262 | OEt | a1 | B06 | b3 | C08 | c1 | D01 | d1 | E02 |
| 2-1-mIE02-2-0413 | 743.262 | OEt | a1 | B06 | b1 | C08 | c2 | D02 | d1 | E02 |
| 2-1-mIE02-2-0414 | 743.274 | OEt | a1 | B05 | b1 | C10 | c2 | D02 | d1 | E02 |
| 2-1-mIE02-2-0415 | 743.279 | OEt | a1 | B04 | b1 | C30 | c3 | D02 | d1 | E02 |
| 2-1-mIE02-2-0416 | 744.243 | OEt | a2 | B17 | b2 | C20 | c3 | D02 | d1 | E02 |
| 2-1-mIE02-2-0417 | 744.243 | OEt | a2 | B14 | b2 | C24 | c3 | D02 | d1 | E02 |
| 2-1-mIE02-2-0418 | 744.257 | OEt | a2 | B19 | b2 | C04 | c1 | D01 | d1 | E02 |
| 2-1-mIE02-2-0419 | 744.258 | OEt | a1 | B06 | b1 | C11 | c2 | D02 | d1 | E02 |
| 2-1-mIE02-2-0420 | 744.262 | OEt | a2 | B18 | b3 | C08 | c1 | D01 | d1 | E02 |
| 2-1-mIE02-2-0421 | 744.262 | OEt | a2 | B18 | b1 | C08 | c2 | D02 | d1 | E02 |
| 2-1-mIE02-2-0422 | 745.238 | OEt | a2 | B17 | b2 | C28 | c3 | D02 | d1 | E02 |
| 2-1-mIE02-2-0423 | 745.242 | OEt | a1 | B03 | b2 | C01 | c2 | D02 | d1 | E02 |
| 2-1-mIE02-2-0424 | 745.253 | OEt | a1 | B06 | b1 | C10 | c2 | D02 | d1 | E02 |
| 2-1-mIE02-2-0425 | 745.257 | OEt | a2 | B18 | b1 | C11 | c2 | D02 | d1 | E02 |
| 2-1-mIE02-2-0426 | 745.258 | OEt | a1 | B04 | b2 | C20 | c3 | D02 | d1 | E02 |
| 2-1-mIE02-2-0427 | 745.258 | OEt | a1 | B01 | b2 | C24 | c3 | D02 | d1 | E02 |
| 2-1-mIE02-2-0428 | 745.272 | OEt | a1 | B09 | b2 | C02 | c1 | D01 | d1 | E02 |
| 2-1-mIE02-2-0429 | 745.309 | OEt | a1 | B05 | b1 | C09 | c1 | D01 | d1 | E02 |
| 2-1-mIE02-2-0430 | 745.329 | OEt | a1 | B07 | b2 | C07 | c1 | D01 | d1 | E02 |
| 2-1-mIE02-2-0431 | 746.188 | OEt | a2 | B14 | b2 | C06 | c2 | D02 | d1 | E02 |
| 2-1-mIE02-2-0432 | 746.252 | OEt | a2 | B18 | b1 | C10 | c2 | D02 | d1 | E02 |
| 2-1-mIE02-2-0433 | 746.252 | OEt | a2 | B11 | b1 | C13 | c2 | D02 | d1 | E02 |
| 2-1-mIE02-2-0434 | 746.253 | OEt | a1 | B02 | b2 | C24 | c3 | D02 | d1 | E02 |
| 2-1-mIE02-2-0435 | 746.253 | OEt | a1 | B04 | b2 | C28 | c3 | D02 | d1 | E02 |
| 2-1-mIE02-2-0436 | 746.268 | OEt | a1 | B09 | b2 | C03 | c1 | DOI | d1 | E02 |
| 2-1-mIE02-2-0437 | 747.203 | OEt | a1 | B01 | b2 | C06 | c2 | D02 | d1 | E02 |
| 2-1-mIE02-2-0438 | 747.240 | OEt | a1 | B05 | b3 | C06 | c1 | D01 | d1 | E02 |
| 2-1-mIE02-2-0439 | 747.248 | OEt | a2 | B12 | b1 | C13 | c2 | D02 | d1 | E02 |
| 2-1-mIE02-2-0440 | 747.288 | OEt | a1 | B06 | b1 | C09 | c1 | D01 | d1 | E02 |
| 2-1-mIE02-2-0441 | 748.187 | OEt | a2 | B11 | b3 | C15 | c2 | D02 | d1 | E02 |
| 2-1-mIE02-2-0442 | 748.199 | OEt | a1 | B02 | b2 | C06 | c2 | D02 | d1 | E02 |
| 2-1-mIE02-2-0443 | 748.201 | OEt | a2 | B17 | b2 | C01 | c2 | D02 | d1 | E02 |
| 2-1-mIE02-2-0444 | 748.218 | OEt | a2 | B20 | b2 | C06 | c1 | D01 | d1 | E02 |
| 2-1-mIE02-2-0445 | 748.235 | OEt | a1 | B05 | b1 | C12 | c2 | D02 | d1 | E02 |

(continued)

| [Table 8-10-3] Compound that may be contained in mixture 2-1-mIE02-2 | | | | | | | | | | |
|---|---|---|---|---|---|---|---|---|---|---|
| ID | Exact Mass | | a | B | b | C | c | D | d | E |
| 2-1-mIE02-2-0446 | 748.257 | OEt | a2 | B11 | b3 | C16 | c1 | D01 | d1 | E02 |
| 2-1-mIE02-2-0447 | 748.287 | OEt | a2 | B18 | b1 | C09 | c1 | D01 | d1 | E02 |
| 2-1-mIE02-2-0448 | 748.329 | OEt | a2 | B13 | b1 | C29 | c3 | D02 | d1 | E02 |
| 2-1-mIE02-2-0449 | 748.329 | OEt | a2 | B15 | b1 | C32 | c3 | D02 | d1 | E02 |
| 2-1-mIE02-2-0450 | 749.183 | OEt | a2 | B12 | b3 | C15 | c2 | D02 | d1 | E02 |
| 2-1-mIE02-2-0451 | 749.217 | OEt | a1 | B04 | b2 | C01 | c2 | D02 | d1 | E02 |
| 2-1-mIE02-2-0452 | 749.219 | OEt | a1 | B06 | b3 | C06 | c1 | D01 | d1 | E02 |
| 2-1-mIE02-2-0453 | 749.233 | OEt | a1 | B07 | b3 | C08 | c1 | D01 | d1 | E02 |
| 2-1-mIE02-2-0454 | 749.233 | OEt | a1 | B07 | b1 | C08 | c2 | D02 | d1 | E02 |
| 2-1-mIE02-2-0455 | 749.252 | OEt | a2 | B12 | b3 | C16 | c1 | D01 | d1 | E02 |
| 2-1-mIE02-2-0456 | 749.263 | OEt | a1 | B08 | b2 | C05 | c1 | D01 | d1 | E02 |
| 2-1-mIE02-2-0457 | 750.214 | OEt | a1 | B06 | b1 | C12 | c2 | D02 | d1 | E02 |
| 2-1-mIE02-2-0458 | 750.218 | OEt | a2 | B18 | b3 | C06 | c1 | D01 | d1 | E02 |
| 2-1-mIE02-2-0459 | 750.218 | OEt | a2 | B11 | b3 | C17 | c1 | D01 | d1 | E02 |
| 2-1-mIE02-2-0460 | 750.218 | OEt | a2 | B11 | b1 | C14 | c2 | D02 | d1 | E02 |
| 2-1-mIE02-2-0461 | 750.228 | OEt | a1 | B07 | b1 | C11 | c2 | D02 | d1 | E02 |
| 2-1-mIE02-2-0462 | 750.272 | OEt | a2 | B15 | b2 | C02 | c2 | D02 | d1 | E02 |
| 2-1-mIE02-2-0463 | 750.278 | OEt | a2 | B19 | b1 | C13 | c1 | D01 | d1 | E02 |
| 2-1-mIE02-2-0464 | 750.309 | OEt | a2 | B15 | b2 | C22 | c3 | D02 | d1 | E02 |
| 2-1-mIE02-2-0465 | 750.309 | OEt | a2 | B16 | b1 | C32 | c3 | D02 | d1 | E02 |
| 2-1-mIE02-2-0466 | 751.213 | OEt | a2 | B12 | b3 | C17 | c1 | D01 | d1 | E02 |
| 2-1-mIE02-2-0467 | 751.213 | OEt | a2 | B18 | b1 | C12 | c2 | D02 | d1 | E02 |
| 2-1-mIE02-2-0468 | 751.213 | OEt | a2 | B12 | b1 | C14 | c2 | D02 | d1 | E02 |
| 2-1-mIE02-2-0469 | 751.224 | OEt | a1 | B07 | b1 | C10 | c2 | D02 | d1 | E02 |
| 2-1-mIE02-2-0470 | 751.229 | OEt | a1 | B09 | b2 | C06 | c1 | D01 | d1 | E02 |
| 2-1-mIE02-2-0471 | 751.247 | OEt | a1 | B10 | b2 | C01 | c1 | D01 | d1 | E02 |
| 2-1-mIE02-2-0472 | 751.268 | OEt | a2 | B15 | b2 | C03 | c2 | D02 | d1 | E02 |
| 2-1-mIE02-2-0473 | 751.268 | OEt | a2 | B13 | b2 | C05 | c2 | D02 | d1 | E02 |
| 2-1-mIE02-2-0474 | 752.213 | OEt | a2 | B19 | b3 | C15 | c1 | D01 | d1 | E02 |
| 2-1-mIE02-2-0475 | 752.243 | OEt | a1 | B08 | b2 | C04 | c1 | D01 | d1 | E02 |
| 2-1-mIE02-2-0476 | 752.252 | OEt | a2 | B16 | b2 | C02 | c2 | D02 | d1 | E02 |
| 2-1-mIE02-2-0477 | 752.288 | OEt | a2 | B16 | b2 | C22 | c3 | D02 | d1 | E02 |
| 2-1-mIE02-2-0478 | 752.288 | OEt | 1 a2 | B13 | b2 | C26 | c3 | D02 | d1 | E02 |
| 2-1-mIE02-2-0479 | 752.304 | OEt | a2 | B14 | b1 | C29 | c3 | D02 | d1 | E02 |
| 2-1-mIE02-2-0480 | 753.247 | OEt | a2 | B16 | b2 | C03 | c2 | D02 | d1 | E02 |
| 2-1-mIE02-2-0481 | 753.259 | OEt | a1 | B07 | b1 | C09 | c1 | D01 | d1 | E02 |
| 2-1-mIE02-2-0482 | 753.320 | OEt | a1 | B05 | b1 | C30 | c3 | D02 | d1 | E02 |

(continued)

| [Table 8-10-3] Compound that may be contained in mixture 2-1-mIE02-2 | | | | | | | | | | |
|---|---|---|---|---|---|---|---|---|---|---|
| ID | Exact Mass | | a | B | b | C | c | D | d | E |
| 2-1-mIE02-2-0483 | 753.320 | OEt | a1 | B01 | b1 | C29 | c3 | D02 | d1 | E02 |
| 2-1-mIE02-2-0484 | 753.320 | OEt | a1 | B03 | b1 | C32 | c3 | D02 | d1 | E02 |
| 2-1-mIE02-2-0485 | 754.244 | OEt | a2 | B19 | b1 | C14 | c1 | D01 | d1 | E02 |
| 2-1-mIE02-2-0486 | 754.247 | OEt | a2 | B13 | b2 | C04 | c2 | D02 | d1 | E02 |
| 2-1-mIE02-2-0487 | 754.265 | OEt | a2 | B19 | b2 | C33 | c3 | D02 | d1 | E02 |
| 2-1-mIE02-2-0488 | 754.284 | OEt | a2 | B15 | b2 | C24 | c3 | D02 | d1 | E02 |
| 2-1-mIE02-2-0489 | 754.304 | OEt | a2 | B11 | b2 | C07 | c2 | D02 | d1 | E02 |
| 2-1-mIE02-2-0490 | 754.315 | OEt | a1 | B02 | b1 | C29 | c3 | D02 | d1 | E02 |
| 2-1-mIE02-2-0491 | 755.190 | OEt | a1 | B07 | b3 | C06 | c1 | D01 | d1 | E02 |
| 2-1-mIE02-2-0492 | 755.243 | OEt | a2 | B14 | b2 | C05 | c2 | D02 | d1 | E02 |
| 2-1-mIE02-2-0493 | 755.262 | OEt | a1 | B03 | b2 | C02 | c2 | D02 | d1 | E02 |
| 2-1-mIE02-2-0494 | 755.299 | OEt | a2 | B12 | b2 | C07 | c2 | D02 | d1 | E02 |
| 2-1-mIE02-2-0495 | 755.299 | OEt | a1 | B05 | b2 | C20 | c3 | D02 | d1 | E02 |
| 2-1-mIE02-2-0496 | 755.299 | OEt | a1 | B03 | b2 | C22 | c3 | D02 | d1 | E02 |
| 2-1-mIE02-2-0497 | 755.299 | OEt | a1 | B06 | b1 | C30 | c3 | D02 | d1 | E02 |
| 2-1-mIE02-2-0498 | 756.185 | OEt | a1 | B07 | b1 | C12 | c2 | D02 | d1 | E02 |
| 2-1-mIE02-2-0499 | 756.229 | OEt | a2 | B15 | b2 | C06 | c2 | D02 | d1 | E02 |
| 2-1-mIE02-2-0500 | 756.258 | OEt | a1 | B03 | b2 | C03 | c2 | D02 | d1 | E02 |
| 2-1-m1E02-2-0501 | 756.258 | OEt | a1 | B01 | b2 | C05 | c2 | D02 | d1 | E02 |
| 2-1-mIE02-2-0502 | 756.263 | OEt | a2 | B16 | b2 | C24 | c3 | D02 | d1 | E02 |
| 2-1-mIE02-2-0503 | 756.263 | OEt | a2 | B14 | b2 | C26 | c3 | D02 | d1 | E02 |
| 2-1-mIE02-2-0504 | 756.279 | OEt | a2 | B17 | b1 | C32 | c3 | D02 | d1 | E02 |
| 2-1-mIE02-2-0505 | 756.294 | OEt | a1 | B05 | b2 | C28 | c3 | D02 | d1 | E02 |
| 2-1-mIE02-2-0506 | 756.298 | OEt | a2 | B18 | b1 | C30 | c3 | D02 | d1 | E02 |
| 2-1-mIE02-2-0507 | 757.253 | OEt | a1 | B02 | b2 | C05 | c2 | D02 | d1 | E02 |
| 2-1-mIE02-2-0508 | 757.272 | OEt | a2 | B20 | b2 | C05 | c1 | D01 | d1 | E02 |
| 2-1-mIE02-2-0509 | 757.278 | OEt | a1 | B06 | b2 | C20 | c3 | D02 | d1 | E02 |
| 2-1-mIE02-2-0510 | 757.278 | OEt | a1 | B01 | b2 | C26 | c3 | D02 | d1 | E02 |
| 2-1-mIE02-2-0511 | 757.295 | OEt | a1 | B04 | b1 | C32 | c3 | D02 | d1 | E02 |
| 2-1-mIE02-2-0512 | 758.208 | OEt | a2 | B16 | b2 | C06 | c2 | D02 | d1 | E02 |
| 2-1-mIE02-2-0513 | 758.208 | OEt | a2 | B11 | b3 | C08 | c2 | D02 | d1 | E02 |
| 2-1-mIE02-2-0514 | 758.222 | OEt | a2 | B17 | b2 | C02 | c2 | D02 | d1 | E02 |
| 2-1-mIE02-2-0515 | 758.222 | OEt | a2 | B14 | b2 | C04 | c2 | D02 | d1 | E02 |
| 2-1-mIE02-2-0516 | 758.259 | OEt | a2 | B17 | b2 | C22 | c3 | D02 | d1 | E02 |
| 2-1-mIE02-2-0517 | 758.264 | OEt | a1 | B08 | b1 | C13 | c1 | D01 | d1 | E02 |
| 2-1-mIE02-2-0518 | 758.273 | OEt | a1 | B02 | b2 | C26 | c3 | D02 | d1 | E02 |
| 2-1-mIE02-2-0519 | 758.273 | OEt | a1 | B06 | b2 | C28 | c3 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0520 | 758.277 | OEt | a2 | B18 | b2 | C20 | c3 | D02 | d1 | E02 |
| 2-1-mlE02-2-0521 | 758.329 | OEt | a2 | B19 | b2 | C07 | c1 | D01 | d1 | E02 |
| 2-1-mlE02-2-0522 | 759.203 | OEt | a2 | B12 | b3 | C08 | c2 | D02 | d1 | E02 |
| 2-1-mlE02-2-0523 | 759.217 | OEt | a2 | B17 | b2 | C03 | c2 | D02 | d1 | E02 |
| 2-1-mlE02-2-0524 | 759.237 | OEt | a1 | B04 | b2 | C02 | c2 | D02 | d1 | E02 |
| 2-1-mlE02-2-0525 | 759.237 | OEt | a1 | B01 | b2 | C04 | c2 | D02 | d1 | E02 |
| 2-1-mlE02-2-0526 | 759.257 | OEt | a1 | B05 | b2 | C01 | c2 | D02 | d1 | E02 |
| 2-1-mlE02-2-0527 | 759.273 | OEt | a2 | B18 | b2 | C28 | c3 | D02 | d1 | E02 |
| 2-1-mlE02-2-0528 | 759.274 | OEt | a1 | B04 | b2 | C22 | c3 | D02 | d1 | E02 |
| 2-1-mlE02-2-0529 | 759.274 | OEt | a1 | B03 | b2 | C24 | c3 | D02 | d1 | E02 |
| 2-1-mlE02-2-0530 | 760.199 | OEt | a1 | B08 | b3 | C15 | c1 | D01 | d1 | E02 |
| 2-1-mlE02-2-0531 | 760.233 | OEt | a1 | B02 | b2 | C04 | c2 | D02 | d1 | E02 |
| 2-1-m1E02-2-0532 | 760.233 | OEt | a1 | B04 | b2 | C03 | c2 | D02 | d1 | E02 |
| 2-1-m1E02-2-0533 | 760.252 | OEt | a2 | B20 | b2 | C04 | c1 | D01 | d1 | E02 |
| 2-1-m1E02-2-0534 | 760.268 | OEt | a2 | B13 | b1 | C13 | c2 | D02 | d1 | E02 |
| 2-1-m1E02-2-0535 | 760.283 | OEt | a1 | B09 | b2 | C05 | c1 | D01 | d1 | E02 |
| 2-1-mlE02-2-0536 | 761.219 | OEt | a1 | B03 | b2 | C06 | c2 | D02 | d1 | E02 |
| 2-1-m1E02-2-0537 | 761.237 | OEt | a1 | B06 | b2 | C01 | c2 | D02 | d1 | E02 |
| 2-1-mlE02-2-0538 | 761.267 | OEt | a1 | B10 | b2 | C02 | c1 | D01 | d1 | E02 |
| 2-1-m1E02-2-0539 | 761.269 | OEt | a1 | B07 | b1 | C30 | c3 | D02 | d1 | E02 |
| 2-1-mlE02-2-0540 | 762.203 | OEt | a2 | B13 | b3 | C15 | c2 | D02 | d1 | E02 |
| 2-1-m1E02-2-0541 | 762.229 | OEt | a1 | B08 | b1 | C14 | c1 | D01 | d1 | E02 |
| 2-1-m1E02-2-0542 | 762.234 | OEt | a2 | B19 | b3 | C08 | c1 | D01 | d1 | E02 |
| 2-1-m1E02-2-0543 | 762.234 | OEt | a2 | B19 | b1 | C08 | c2 | D02 | d1 | E02 |
| 2-1-m1E02-2-0544 | 762.234 | OEt | a2 | B11 | b1 | C09 | c2 | D02 | d1 | E02 |
| 2-1-m1E02-2-0545 | 762.234 | OEt | a2 | B17 | b2 | C24 | c3 | D02 | d1 | E02 |
| 2-1-m1E02-2-0546 | 762.236 | OEt | a2 | B18 | b2 | C01 | c2 | D02 | d1 | E02 |
| 2-1-m1E02-2-0547 | 762.251 | OEt | a1 | B08 | b2 | C33 | c3 | D02 | d1 | E02 |
| 2-1-m1E02-2-0548 | 762.262 | OEt | a1 | B10 | b2 | C03 | c1 | D01 | d1 | E02 |
| 2-1-m1E02-2-0549 | 762.272 | OEt | a2 | B13 | b3 | C16 | c1 | D01 | d1 | E02 |
| 2-1-m1E02-2-0550 | 762.309 | OEt | a2 | B11 | b1 | C31 | c3 | D02 | d1 | E02 |
| 2-1-mlE02-2-0551 | 762.345 | OEt | a2 | B15 | b1 | C29 | c3 | D02 | d1 | E02 |
| 2-1-mlE02-2-0552 | 763.229 | OEt | a2 | B12 | b1 | C09 | c2 | D02 | d1 | E02 |
| 2-1-mlE02-2-0553 | 763.229 | OEt | a2 | B19 | b1 | C11 | c2 | D02 | d1 | E02 |
| 2-1-mlE02-2-0554 | 763.249 | OEt | a1 | B07 | b2 | C20 | c3 | D02 | d1 | E02 |
| 2-1-mlE02-2-0555 | 763.249 | OEt | a1 | B04 | b2 | C24 | c3 | D02 | d1 | E02 |
| 2-1-mlE02-2-0556 | 763.263 | OEt | a1 | B09 | b2 | C04 | c1 | D01 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0557 | 763.304 | OEt | a2 | B12 | b1 | C31 | c3 | D02 | d1 | E02 |
| 2-1-mlE02-2-0558 | 764.164 | OEt | a2 | B11 | b3 | C06 | c2 | D02 | d1 | E02 |
| 2-1-mlE02-2-0559 | 764.179 | OEt | a2 | B17 | b2 | C06 | c2 | D02 | d1 | E02 |
| 2-1-mlE02-2-0560 | 764.224 | OEt | a2 | B19 | b1 | C10 | c2 | D02 | d1 | E02 |
| 2-1-mlE02-2-0561 | 764.234 | OEt | a2 | B13 | b3 | C17 | c1 | D01 | d1 | E02 |
| 2-1-mlE02-2-0562 | 764.234 | OEt | a2 | B13 | b1 | C14 | c2 | D02 | d1 | E02 |
| 2-1-mlE02-2-0563 | 764.243 | OEt | a2 | B14 | b1 | C13 | c2 | D02 | d1 | E02 |
| 2-1-mlE02-2-0564 | 764.244 | OEt | a1 | B07 | b2 | C28 | c3 | D02 | d1 | E02 |
| 2-1-mlE02-2-0565 | 764.324 | OEt | a2 | B16 | b1 | C29 | c3 | D02 | d1 | E02 |
| 2-1-m1E02-2-0566 | 765.160 | OEt | a2 | B12 | b3 | C06 | c2 | D02 | d1 | E02 |
| 2-1-m1E02-2-0567 | 765.194 | OEt | a1 | B04 | b2 | C06 | c2 | D02 | d1 | E02 |
| 2-1-mlE02-2-0568 | 765.229 | OEt | a2 | B11 | b3 | C18 | c1 | D01 | d1 | E02 |
| 2-1-mlE02-2-0569 | 765.258 | OEt | a1 | B01 | b1 | C13 | c2 | D02 | d1 | E02 |
| 2-1-mlE02-2-0570 | 765.283 | OEt | a2 | B15 | b2 | C05 | c2 | D02 | d1 | E02 |
| 2-1-m1E02-2-0571 | 766.178 | OEt | a2 | B14 | b3 | C15 | c2 | D02 | d1 | E02 |
| 2-1-mlE02-2-0572 | 766.224 | OEt | a2 | B12 | b3 | C18 | c1 | D01 | d1 | E02 |
| 2-1-m1E02-2-0573 | 766.247 | OEt | a2 | B14 | b3 | C16 | c1 | D01 | d1 | E02 |
| 2-1-mlE02-2-0574 | 766.253 | OEt | a1 | B02 | b1 | C13 | c2 | D02 | d1 | E02 |
| 2-1-m1E02-2-0575 | 766.259 | OEt | a2 | B19 | b1 | C09 | c1 | D01 | d1 | E02 |
| 2-1-m1E02-2-0576 | 766.273 | OEt | a2 | B20 | b1 | C13 | c1 | D01 | d1 | E02 |
| 2-1-m1E02-2-0577 | 766.304 | OEt | a2 | B15 | b2 | C26 | c3 | D02 | d1 | E02 |
| 2-1-mlE02-2-0578 | 766.315 | OEt | a1 | B08 | b2 | C07 | c1 | D01 | d1 | E02 |
| 2-1-mlE02-2-0579 | 767.193 | OEt | a1 | B01 | b3 | C15 | c2 | D02 | d1 | E02 |
| 2-1-mlE02-2-0580 | 767.207 | OEt | a1 | B07 | b2 | C01 | c2 | D02 | d1 | E02 |
| 2-1-mlE02-2-0581 | 767.224 | OEt | a1 | B10 | b2 | C06 | c1 | D01 | d1 | E02 |
| 2-1-mlE02-2-0582 | 767.262 | OEt | a1 | B01 | b3 | C16 | c1 | D01 | d1 | E02 |
| 2-1-mlE02-2-0583 | 767.262 | OEt | a2 | B16 | b2 | C05 | c2 | D02 | d1 | E02 |
| 2-1-mlE02-2-0584 | 767.335 | OEt | a1 | B03 | b1 | C29 | c3 | D02 | d1 | E02 |
| 2-1-mlE02-2-0585 | 767.335 | OEt | a1 | B05 | b1 | C32 | c3 | D02 | d1 | E02 |
| 2-1-mlE02-2-0586 | 768.188 | OEt | a1 | B02 | b3 | C15 | c2 | D02 | d1 | E02 |
| 2-1-mlE02-2-0587 | 768.190 | OEt | a2 | B19 | b3 | C06 | c1 | D01 | d1 | E02 |
| 2-1-m1E02-2-0588 | 768.208 | OEt | a2 | B20 | b3 | C15 | c1 | D01 | d1 | E02 |
| 2-1-m1E02-2-0589 | 768.209 | OEt | a2 | B14 | b3 | C17 | c1 | D01 | d1 | E02 |
| 2-1-m1E02-2-0590 | 768.209 | OEt | a2 | B14 | b1 | C14 | c2 | D02 | d1 | E02 |
| 2-1-m1E02-2-0591 | 768.258 | OEt | a1 | B02 | b3 | C16 | c1 | D01 | d1 | E02 |
| 2-1-m1E02-2-0592 | 768.263 | OEt | a2 | B15 | b2 | C04 | c2 | D02 | d1 | E02 |
| 2-1-m1E02-2-0593 | 768.283 | OEt | a2 | B16 | b2 | C26 | c3 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0594 | 768.319 | OEt | a2 | B13 | b2 | C07 | c2 | D02 | d1 | E02 |
| 2-1-m1E02-2-0595 | 769.185 | OEt | a2 | B19 | b1 | C12 | c2 | D02 | d1 | E02 |
| 2-1-m1E02-2-0596 | 769.224 | OEt | a1 | B01 | b3 | C17 | c1 | D01 | d1 | E02 |
| 2-1-m1E02-2-0597 | 769.224 | OEt | a1 | B01 | b1 | C14 | c2 | D02 | d1 | E02 |
| 2-1-m1E02-2-0598 | 769.278 | OEt | a1 | B05 | b2 | C02 | c2 | D02 | d1 | E02 |
| 2-1-m1E02-2-0599 | 769.284 | OEt | a1 | B09 | b1 | C13 | c1 | D01 | d1 | E02 |
| 2-1-m1E02-2-0600 | 769.315 | OEt | a1 | B05 | b2 | C22 | c3 | D02 | d1 | E02 |
| 2-1-m1E02-2-0601 | 769.315 | OEt | a1 | B06 | b1 | C32 | c3 | D02 | d1 | E02 |
| 2-1-mlE02-2-0602 | 770.219 | OEt | a1 | B02 | b3 | C17 | c1 | D01 | d1 | E02 |
| 2-1-m1E02-2-0603 | 770.219 | OEt | a1 | B08 | b3 | C08 | c1 | D01 | d1 | E02 |
| 2-1-mlE02-2-0604 | 770.219 | OEt | a1 | B08 | b1 | C08 | c2 | D02 | d1 | E02 |
| 2-1-m1E02-2-0605 | 770.219 | OEt | a1 | B02 | b1 | C14 | c2 | D02 | d1 | E02 |
| 2-1-mlE02-2-0606 | 770.239 | OEt | a2 | B20 | b1 | C14 | c1 | D01 | d1 | E02 |
| 2-1-mlE02-2-0607 | 770.242 | OEt | a2 | B16 | b2 | C04 | c2 | D02 | d1 | E02 |
| 2-1-m1E02-2-0608 | 770.260 | OEt | a2 | B20 | b2 | C33 | c3 | D02 | d1 | E02 |
| 2-1-mlE02-2-0609 | 770.273 | OEt | a1 | B05 | b2 | C03 | c2 | D02 | d1 | E02 |
| 2-1-mlE02-2-0610 | 770.273 | OEt | a1 | B03 | b2 | C05 | c2 | D02 | d1 | E02 |
| 2-1-mlE02-2-0611 | 770.295 | OEt | a2 | B17 | b1 | C29 | c3 | D02 | d1 | E02 |
| 2-1-m1E02-2-0612 | 770.314 | OEt | a2 | B18 | b1 | C32 | c3 | D02 | d1 | E02 |
| 2-1-mlE02-2-0613 | 771.215 | OEt | a1 | B08 | b1 | C11 | c2 | D02 | d1 | E02 |
| 2-1-mlE02-2-0614 | 771.219 | OEt | a1 | B09 | b3 | C15 | c1 | D01 | d1 | E02 |
| 2-1-m1E02-2-0615 | 771.257 | OEt | a1 | B06 | b2 | C02 | c2 | D02 | d1 | E02 |
| 2-1-mlE02-2-0616 | 771.294 | OEt | a1 | B06 | b2 | C22 | c3 | D02 | d1 | E02 |
| 2-1-m1E02-2-0617 | 771.294 | OEt | a1 | B03 | b2 | C26 | c3 | D02 | d1 | E02 |
| 2-1-mlE02-2-0618 | 771.310 | OEt | a1 | B04 | b1 | C29 | c3 | D02 | d1 | E02 |
| 2-1-m1E02-2-0619 | 772.210 | OEt | a1 | B08 | b1 | C10 | c2 | D02 | d1 | E02 |
| 2-1-m1E02-2-0620 | 772.224 | OEt | a2 | B13 | b3 | C08 | c2 | D02 | d1 | E02 |
| 2-1-mlE02-2-0621 | 772.253 | OEt | a1 | B06 | b2 | C03 | c2 | D02 | d1 | E02 |
| 2-1-m1E02-2-0622 | 772.257 | OEt | a2 | B18 | b2 | C02 | c2 | D02 | d1 | E02 |
| 2-1-mlE02-2-0623 | 772.293 | OEt | a2 | B18 | b2 | C22 | c3 | D02 | d1 | E02 |
| 2-1-m1E02-2-0624 | 772.294 | OEt | a2 | B14 | b2 | C07 | c2 | D02 | d1 | E02 |
| 2-1-mlE02-2-0625 | 773.233 | OEt | a2 | B17 | b2 | C05 | c2 | D02 | d1 | E02 |
| 2-1-m1E02-2-0626 | 773.249 | OEt | a1 | B09 | b1 | C14 | c1 | D01 | d1 | E02 |
| 2-1-m1E02-2-0627 | 773.252 | OEt | a2 | B18 | b2 | C03 | c2 | D02 | d1 | E02 |
| 2-1-m1E02-2-0628 | 773.253 | OEt | a1 | B03 | b2 | C04 | c2 | D02 | d1 | E02 |
| 2-1-m1E02-2-0629 | 773.271 | OEt | a1 | B09 | b2 | C33 | c3 | D02 | d1 | E02 |
| 2-1-m1E02-2-0630 | 773.289 | OEt | a1 | B05 | b2 | C24 | c3 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0631 | 773.309 | OEt | a1 | B01 | b2 | C07 | c2 | D02 | d1 | E02 |
| 2-1-m1E02-2-0632 | 774.245 | OEt | a1 | B08 | b1 | C09 | c1 | D01 | d1 | E02 |
| 2-1-m1E02-2-0633 | 774.248 | OEt | a1 | B04 | b2 | C05 | c2 | D02 | d1 | E02 |
| 2-1-m1E02-2-0634 | 774.253 | OEt | a2 | B17 | b2 | C26 | c3 | D02 | d1 | E02 |
| 2-1-m1E02-2-0635 | 774.270 | OEt | a2 | B19 | b1 | C30 | c3 | D02 | d1 | E02 |
| 2-1-m1E02-2-0636 | 774.284 | OEt | a2 | B15 | b1 | C13 | c2 | D02 | d1 | E02 |
| 2-1-m1E02-2-0637 | 774.305 | OEt | a1 | B02 | b2 | C07 | c2 | D02 | d1 | E02 |
| 2-1-m1E02-2-0638 | 774.324 | OEt | a2 | B20 | b2 | C07 | c1 | D01 | d1 | E02 |
| 2-1-m1E02-2-0639 | 775.235 | OEt | a1 | B05 | b2 | C06 | c2 | D02 | d1 | E02 |
| 2-1-m1E02-2-0640 | 775.269 | OEt | a1 | B06 | b2 | C24 | c3 | D02 | d1 | E02 |
| 2-1-m1E02-2-0641 | 775.269 | OEt | a1 | B04 | b2 | C26 | c3 | D02 | d1 | E02 |
| 2-1-m1E02-2-0642 | 775.285 | OEt | a1 | B07 | b1 | C32 | c3 | D02 | d1 | E02 |
| 2-1-m1E02-2-0643 | 776.176 | OEt | a1 | B08 | b3 | C06 | c1 | D01 | d1 | E02 |
| 2-1-m1E02-2-0644 | 776.199 | OEt | a2 | B14 | b3 | C08 | c2 | D02 | d1 | E02 |
| 2-1-m1E02-2-0645 | 776.213 | OEt | a2 | B17 | b2 | C04 | c2 | D02 | d1 | E02 |
| 2-1-m1E02-2-0646 | 776.219 | OEt | a2 | B15 | b3 | C15 | c2 | D02 | d1 | E02 |
| 2-1-m1E02-2-0647 | 776.249 | OEt | a2 | B13 | b1 | C09 | c2 | D02 | d1 | E02 |
| 2-1-m1E02-2-0648 | 776.249 | OEt | a2 | B19 | b2 | C20 | c3 | D02 | d1 | E02 |
| 2-1-m1E02-2-0649 | 776.263 | OEt | a2 | B16 | b1 | C13 | c2 | D02 | d1 | E02 |
| 2-1-m1E02-2-0650 | 776.268 | OEt | a2 | B18 | b2 | C24 | c3 | D02 | d1 | E02 |
| 2-1-m1E02-2-0651 | 776.278 | OEt | a1 | B10 | b2 | C05 | c1 | D01 | d1 | E02 |
| 2-1-m1E02-2-0652 | 776.288 | OEt | a2 | B15 | b3 | C16 | c1 | D01 | d1 | E02 |
| 2-1-m1E02-2-0653 | 776.324 | OEt | a2 | B13 | b1 | C31 | c3 | D02 | d1 | E02 |
| 2-1-m1E02-2-0654 | 777.171 | OEt | a1 | B08 | b1 | C12 | c2 | D02 | d1 | E02 |
| 2-1-m1E02-2-0655 | 777.214 | OEt | a1 | B06 | b2 | C06 | c2 | D02 | d1 | E02 |
| 2-1-m1E02-2-0656 | 777.214 | OEt | a1 | B01 | b3 | C08 | c2 | D02 | d1 | E02 |
| 2-1-m1E02-2-0657 | 777.228 | OEt | a1 | B07 | 11 b2 | C02 | c2 | D02 | d1 | E02 |
| 2-1-m1E02-2-0658 | 777.228 | OEt | a1 | B04 | b2 | C04 | c2 | D02 | d1 | E02 |
| 2-1-m1E02-2-0659 | 777.244 | OEt | a2 | B19 | b2 | C28 | c3 | D02 | d1 | E02 |
| 2-1-m1E02-2-0660 | 777.264 | OEt | a1 | B07 | b2 | C22 | c3 | D02 | d1 | E02 |
| 2-1-m1E02-2-0661 | 777.335 | OEt | a1 | B09 | b2 | C07 | c1 | D01 | d1 | E02 |
| 2-1-m1E02-2-0662 | 778.180 | OEt | a2 | B13 | b3 | C06 | c2 | D02 | d1 | E02 |
| 2-1-m1E02-2-0663 | 778.198 | OEt | a2 | B16 | b3 | C15 | c2 | D02 | d1 | E02 |
| 2-1-m1E02-2-0664 | 778.209 | OEt | a1 | B02 | b3 | C08 | c2 | D02 | d1 | E02 |
| 2-1-m1E02-2-0665 | 778.213 | OEt | a2 | B18 | b2 | C06 | c2 | D02 | d1 | E02 |
| 2-1-m1E02-2-0666 | 778.223 | OEt | a1 | B07 | b2 | C03 | c2 | D02 | d1 | E02 |
| 2-1-m1E02-2-0667 | 778.228 | OEt | a2 | B20 | b3 | C08 | c1 | D01 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0668 | 778.228 | OEt | a2 | B20 | b1 | C08 | c2 | D02 | d1 | E02 |
| 2-1-m1E02-2-0669 | 778.249 | OEt | a2 | B15 | b3 | C17 | c1 | D01 | d1 | E02 |
| 2-1-m1E02-2-0670 | 778.249 | OEt | a2 | B15 | b1 | C14 | c2 | D02 | d1 | E02 |
| 2-1-m1E02-2-0671 | 778.267 | OEt | a2 | B16 | b3 | C16 | c1 | D01 | d1 | E02 |
| 2-1-m1E02-2-0672 | 779.224 | OEt | a2 | B20 | b1 | C11 | c2 | D02 | d1 | E02 |
| 2-1-m1E02-2-0673 | 779.245 | OEt | a2 | B13 | b3 | C18 | c1 | D01 | d1 | E02 |
| 2-1-m1E02-2-0674 | 779.258 | OEt | a1 | B10 | b2 | C04 | c1 | D01 | d1 | E02 |
| 2-1-m1E02-2-0675 | 779.274 | OEt | a1 | B03 | b1 | C13 | c2 | D02 | d1 | E02 |
| 2-1-m1E02-2-0676 | 780.208 | OEt | a2 | B19 | b2 | C01 | c2 | D02 | d1 | E02 |
| 2-1-m1E02-2-0677 | 780.219 | OEt | a2 | B20 | b1 | C10 | c2 | D02 | d1 | E02 |
| 2-1-m1E02-2-0678 | 780.224 | OEt | a2 | B14 | b1 | C09 | c2 | D02 | d1 | E02 |
| 2-1-m1E02-2-0679 | 780.229 | OEt | a2 | B16 | b3 | C17 | c1 | D01 | d1 | E02 |
| 2-1-m1E02-2-0680 | 780.229 | OEt | a2 | B16 | b1 | C14 | c2 | D02 | d1 | E02 |
| 2-1-m1E02-2-0681 | 780.299 | OEt | a2 | B14 | b1 | C31 | c3 | D02 | d1 | E02 |
| 2-1-m1E02-2-0682 | 781.209 | OEt | a1 | B03 | b3 | C15 | c2 | D02 | d1 | E02 |
| 2-1-m1E02-2-0683 | 781.239 | OEt | a1 | B09 | b3 | C08 | c1 | D01 | d1 | E02 |
| 2-1-m1E02-2-0684 | 781.239 | OEt | a1 | B09 | b1 | C08 | c2 | D02 | d1 | E02 |
| 2-1-m1E02-2-0685 | 781.239 | OEt | a1 | B01 | b1 | C09 | c2 | D02 | d1 | E02 |
| 2-1-m1E02-2-0686 | 781.239 | OEt | a1 | B07 | b2 | C24 | c3 | D02 | d1 | E02 |
| 2-1-m1E02-2-0687 | 781.278 | OEt | a1 | B03 | b3 | C16 | c1 | D01 | d1 | E02 |
| 2-1-m1E02-2-0688 | 781.315 | OEt | a1 | B01 | b1 | C31 | c3 | D02 | d1 | E02 |
| 2-1-m1E02-2-0689 | 781.351 | OEt | a1 | B05 | b1 | C29 | c3 | D02 | d1 | E02 |
| 2-1-m1E02-2-0690 | 782.155 | OEt | a2 | B14 | b3 | C06 | c2 | D02 | d1 | E02 |
| 2-1-m1E02-2-0691 | 782.233 | OEt | a2 | B17 | b1 | C13 | c2 | D02 | d1 | E02 |
| 2-1-m1E02-2-0692 | 782.235 | OEt | a1 | B02 | b1 | C09 | c2 | D02 | d1 | E02 |
| 2-1-m1E02-2-0693 | 782.235 | OEt | a1 | B09 | b1 | C11 | c2 | D02 | d1 | E02 |
| 2-1-m1E02-2-0694 | 782.254 | OEt | a2 | B20 | b1 | C09 | c1 | D01 | d1 | E02 |
| 2-1-m1E02-2-0695 | 782.256 | OEt | a1 | B08 | b1 | C30 | c3 | D02 | d1 | E02 |
| 2-1-mlE02-2-0696 | 782.310 | OEt | a1 | B02 | b1 | C31 | c3 | D02 | d1 | E02 |
| 2-1-m1E02-2-0697 | 782.335 | OEt | a2 | B15 | b2 | C07 | c2 | D02 | d1 | E02 |
| 2-1-mlE02-2-0698 | 783.170 | OEt | a1 | B01 | b3 | C06 | c2 | D02 | d1 | E02 |
| 2-1-m1E02-2-0699 | 783.184 | OEt | a1 | B07 | b2 | C06 | c2 | D02 | d1 | E02 |
| 2-1-mlE02-2-0700 | 783.220 | OEt | a2 | B14 | b3 | C18 | c1 | D01 | d1 | E02 |
| 2-1-mlE02-2-0701 | 783.230 | OEt | a1 | B09 | b1 | C10 | c2 | D02 | d1 | E02 |
| 2-1-m1E02-2-0702 | 783.240 | OEt | a1 | B03 | b3 | C17 | c1 | D01 | d1 | E02 |
| 2-1-mlE02-2-0703 | 783.240 | OEt | a1 | B03 | b1 | C14 | c2 | D02 | d1 | E02 |
| 2-1-m1E02-2-0704 | 783.249 | OEt | a1 | B04 | b1 | C13 | c2 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-mlE02-2-0705 | 783.330 | OEt | a1 | B06 | b1 | C29 | c3 | D02 | d1 | E02 |
| 2-1-m1E02-2-0706 | 784.166 | OEt | a1 | B02 | b3 | C06 | c2 | D02 | d1 | E02 |
| 2-1-mlE02-2-0707 | 784.168 | OEt | a2 | B17 | b3 | C15 | c2 | D02 | d1 | E02 |
| 2-1-mlE02-2-0708 | 784.185 | OEt | a2 | B20 | b3 | C06 | c1 | D01 | d1 | E02 |
| 2-1-m1E02-2-0709 | 784.235 | OEt | a1 | B01 | b3 | C18 | c1 | D01 | d1 | E02 |
| 2-1-mlE02-2-0710 | 784.235 | OEt | a1 | B08 | b2 | C20 | c3 | D02 | d1 | E02 |
| 2-1-mlE02-2-0711 | 784.238 | OEt | a2 | B17 | b3 | C16 | c1 | D01 | d1 | E02 |
| 2-1-m1E02-2-0712 | 784.289 | OEt | a1 | B05 | b2 | C05 | c2 | D02 | d1 | E02 |
| 2-1-m1E02-2-0713 | 784.314 | OEt | a2 | B16 | b2 | C07 | c2 | D02 | d1 | E02 |
| 2-1-m1E02-2-0714 | 784.329 | OEt | a2 | B18 | b1 | C29 | c3 | D02 | d1 | E02 |
| 2-1-mlE02-2-0715 | 785.180 | OEt | a2 | B20 | b1 | C12 | c2 | D02 | d1 | E02 |
| 2-1-m1E02-2-0716 | 785.184 | OEt | a1 | B04 | b3 | C15 | c2 | D02 | d1 | E02 |
| 2-1-m1E02-2-0717 | 785.230 | OEt | a1 | B02 | b3 | C18 | c1 | D01 | d1 | E02 |
| 2-1-m1E02-2-0718 | 785.230 | OEt | a1 | B08 | b2 | C28 | c3 | D02 | d1 | E02 |
| 2-1-mlE02-2-0719 | 785.253 | OEt | a1 | B04 | b3 | C16 | c1 | D01 | d1 | E02 |
| 2-1-m1E02-2-0720 | 785.265 | OEt | a1 | B09 | b1 | C09 | c1 | D01 | d1 | E02 |
| 2-1-m1E02-2-0721 | 785.278 | OEt | a1 | B10 | b1 | C13 | c1 | D01 | d1 | E02 |
| 2-1-m1E02-2-0722 | 785.309 | OEt | a1 | B05 | b2 | C26 | c3 | D02 | d1 | E02 |
| 2-1-m1E02-2-0723 | 786.199 | OEt | a2 | B17 | b3 | C17 | c1 | D01 | d1 | E02 |
| 2-1-mlE02-2-0724 | 786.199 | OEt | a2 | B17 | b1 | C14 | c2 | D02 | d1 | E02 |
| 2-1-m1E02-2-0725 | 786.239 | OEt | a2 | B15 | b3 | C08 | c2 | D02 | d1 | E02 |
| 2-1-mlE02-2-0726 | 786.268 | OEt | a1 | B06 | b2 | C05 | c2 | D02 | d1 | E02 |
| 2-1-m1E02-2-0727 | 787.196 | OEt | a1 | B09 | b3 | C06 | c1 | D01 | d1 | E02 |
| 2-1-mlE02-2-0728 | 787.213 | OEt | a1 | B10 | b3 | C15 | c1 | D01 | d1 | E02 |
| 2-1-m1E02-2-0729 | 787.215 | OEt | a1 | B04 | b3 | C17 | c1 | D01 | d1 | E02 |
| 2-1-m1E02-2-0730 | 787.215 | OEt | a1 | B04 | b1 | C14 | c2 | D02 | d1 | E02 |
| 2-1-mlE02-2-0731 | 787.268 | OEt | a2 | B18 | b2 | COS | c2 | D02 | d1 | E02 |
| 2-1-m1E02-2-0732 | 787.269 | OEt | a1 | B05 | b2 | C04 | c2 | D02 | d1 | E02 |
| 2-1-m1E02-2-0733 | 787.289 | OEt | a1 | B06 | b2 | C26 | c3 | D02 | d1 | E02 |
| 2-1-m1E02-2-0734 | 787.325 | OEt | a1 | B03 | b2 | C07 | c2 | D02 | d1 | E02 |
| 2-1-m1E02-2-0735 | 788.191 | OEt | a1 | B09 | b1 | C12 | c2 | D02 | d1 | E02 |
| 2-1-m1E02-2-0736 | 788.193 | OEt | a1 | B08 | b2 | C01 | c2 | D02 | d1 | E02 |
| 2-1-m1E02-2-0737 | 788.219 | OEt | a2 | B16 | b3 | C08 | c2 | D02 | d1 | E02 |
| 2-1-mlE02-2-0738 | 788.286 | OEt | a2 | B19 | b1 | C32 | c3 | D02 | d1 | E02 |
| 2-1-m1E02-2-0739 | 788.288 | OEt | a2 | B18 | b2 | C26 | c3 | D02 | d1 | E02 |
| 2-1-mlE02-2-0740 | 789.244 | OEt | a1 | B10 | b1 | C14 | c1 | D01 | d1 | E02 |
| 2-1-m1E02-2-0741 | 789.248 | OEt | a1 | B06 | b2 | C04 | c2 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0742 | 789.266 | OEt | a1 | B10 | b2 | C33 | c3 | D02 | d1 | E02 |
| 2-1-mlE02-2-0743 | 789.301 | OEt | a1 | B07 | b1 | C29 | c3 | D02 | d1 | E02 |
| 2-1-m1E02-2-0744 | 790.228 | OEt | a2 | B19 | b2 | C02 | c2 | D02 | d1 | E02 |
| 2-1-mlE02-2-0745 | 790.247 | OEt | a2 | B18 | b2 | C04 | c2 | D02 | d1 | E02 |
| 2-1-m1E02-2-0746 | 790.265 | OEt | a2 | B15 | b1 | C09 | c2 | D02 | d1 | E02 |
| 2-1-mlE02-2-0747 | 790.265 | OEt | a2 | B19 | b2 | C22 | c3 | D02 | d1 | E02 |
| 2-1-mlE02-2-0748 | 790.265 | OEt | a2 | B20 | b1 | C30 | c3 | D02 | d1 | E02 |
| 2-1-m1E02-2-0749 | 790.285 | OEt | a2 | B17 | b2 | C07 | c2 | D02 | d1 | E02 |
| 2-1-mlE02-2-0750 | 790.340 | OEt | a2 | B15 | b1 | C31 | c3 | D02 | d1 | E02 |
| 2-1-m1E02-2-0751 | 791.224 | OEt | a2 | B19 | b2 | C03 | c2 | D02 | d1 | E02 |
| 2-1-mlE02-2-0752 | 791.229 | OEt | a1 | B03 | b3 | C08 | c2 | D02 | d1 | E02 |
| 2-1-m1E02-2-0753 | 791.300 | OEt | a1 | B04 | b2 | C07 | c2 | D02 | d1 | E02 |
| 2-1-mlE02-2-0754 | 792.196 | OEt | a2 | B15 | b3 | C06 | c2 | D02 | d1 | E02 |
| 2-1-mlE02-2-0755 | 792.239 | OEt | a1 | B07 | b2 | C05 | c2 | D02 | d1 | E02 |
| 2-1-m1E02-2-0756 | 792.244 | OEt | a2 | B16 | b1 | C09 | c2 | D02 | d1 | E02 |
| 2-1-mlE02-2-0757 | 792.244 | OEt | a2 | B20 | b2 | C20 | c3 | D02 | d1 | E02 |
| 2-1-m1E02-2-0758 | 792.319 | OEt | a2 | B16 | b1 | C31 | c3 | D02 | d1 | E02 |
| 2-1-mlE02-2-0759 | 793.239 | OEt | a2 | B20 | b2 | C28 | c3 | D02 | d1 | E02 |
| 2-1-mlE02-2-0760 | 793.259 | OEt | a1 | B07 | b2 | C26 | c3 | D02 | d1 | E02 |
| 2-1-mlE02-2-0761 | 793.260 | OEt | a2 | B15 | b3 | C18 | c1 | D01 | d1 | E02 |
| 2-1-mlE02-2-0762 | 793.276 | OEt | a1 | B09 | b1 | C30 | c3 | D02 | d1 | E02 |
| 2-1-mlE02-2-0763 | 793.289 | OEt | a1 | B05 | b1 | C13 | c2 | D02 | d1 | E02 |
| 2-1-m1E02-2-0764 | 793.330 | OEt | a1 | B10 | b2 | C07 | c1 | D01 | d1 | E02 |
| 2-1-m1E02-2-0765 | 794.175 | OEt | a2 | B16 | b3 | C06 | c2 | D02 | d1 | E02 |
| 2-1-m1E02-2-0766 | 794.189 | OEt | a2 | B17 | b3 | C08 | c2 | D02 | d1 | E02 |
| 2-1-m1E02-2-0767 | 794.240 | OEt | a2 | B19 | b2 | C24 | c3 | D02 | d1 | E02 |
| 2-1-m1E02-2-0768 | 795.204 | OEt | a1 | B04 | b3 | C08 | c2 | D02 | d1 | E02 |
| 2-1-m1E02-2-0769 | 795.219 | OEt | a1 | B07 | b2 | C04 | c2 | D02 | d1 | E02 |
| 2-1-m1E02-2-0770 | 795.224 | OEt | a1 | B05 | b3 | C15 | c2 | D02 | d1 | E02 |
| 2-1-mlE02-2-0771 | 795.240 | OEt | a2 | B16 | b3 | C18 | c1 | D01 | d1 | E02 |
| 2-1-m1E02-2-0772 | 795.255 | OEt | a1 | B03 | b1 | C09 | c2 | D02 | d1 | E02 |
| 2-1-mlE02-2-0773 | 795.255 | OEt | a1 | B09 | b2 | C20 | c3 | D02 | d1 | E02 |
| 2-1-m1E02-2-0774 | 795.269 | OEt | a1 | B06 | b1 | C13 | c2 | D02 | d1 | E02 |
| 2-1-mlE02-2-0775 | 795.294 | OEt | a1 | B05 | b3 | C16 | c1 | D01 | d1 | E02 |
| 2-1-m1E02-2-0776 | 795.330 | OEt | a1 | B03 | b1 | C31 | c3 | D02 | d1 | E02 |
| 2-1-m1E02-2-0777 | 796.185 | OEt | a2 | B19 | b2 | C06 | c2 | D02 | d1 | E02 |
| 2-1-m1E02-2-0778 | 796.203 | OEt | a2 | B20 | b2 | C01 | c2 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0779 | 796.250 | OEt | a1 | B09 | b2 | C28 | c3 | D02 | d1 | E02 |
| 2-1-m1E02-2-0780 | 796.268 | OEt | a2 | B18 | b1 | C13 | c2 | D02 | d1 | E02 |
| 2-1-m1E02-2-0781 | 796.271 | OEt | a1 | B08 | b1 | C32 | c3 | D02 | d1 | E02 |
| 2-1-m1E02-2-0782 | 797.186 | OEt | a1 | B03 | b3 | C06 | c2 | D02 | d1 | E02 |
| 2-1-m1E02-2-0783 | 797.204 | OEt | a1 | B06 | b3 | C15 | c2 | D02 | d1 | E02 |
| 2-1-m1E02-2-0784 | 797.234 | OEt | a1 | B10 | b3 | C08 | c1 | D01 | d1 | E02 |
| 2-1-m1E02-2-0785 | 797.234 | OEt | a1 | B10 | b1 | C08 | c2 | D02 | d1 | E02 |
| 2-1-m1E02-2-0786 | 797.255 | OEt | a1 | B05 | b3 | C17 | c1 | D01 | d1 | E02 |
| 2-1-m1E02-2-0787 | 797.255 | OEt | a1 | B05 | b1 | C14 | c2 | D02 | d1 | E02 |
| 2-1-m1E02-2-0788 | 797.273 | OEt | a1 | B06 | b3 | C16 | c1 | D01 | d1 | E02 |
| 2-1-m1E02-2-0789 | 798.203 | OEt | a2 | B18 | b3 | C15 | c2 | D02 | d1 | E02 |
| 2-1-mlE02-2-0790 | 798.214 | OEt | a1 | B08 | b2 | C02 | c2 | D02 | d1 | E02 |
| 2-1-m1E02-2-0791 | 798.215 | OEt | a2 | B17 | b1 | C09 | c2 | D02 | d1 | E02 |
| 2-1-mlE02-2-0792 | 798.229 | OEt | a1 | B10 | b1 | C11 | c2 | D02 | d1 | E02 |
| 2-1-m1E02-2-0793 | 798.251 | OEt | a1 | B03 | b3 | C18 | c1 | D01 | d1 | E02 |
| 2-1-mlE02-2-0794 | 798.251 | OEt | a1 | B08 | b2 | C22 | c3 | D02 | d1 | E02 |
| 2-1-mlE02-2-0795 | 798.272 | OEt | a2 | B18 | b3 | C16 | c1 | D01 | d1 | E02 |
| 2-1-m1E02-2-0796 | 798.290 | OEt | a2 | B17 | b1 | C31 | c3 | D02 | d1 | E02 |
| 2-1-mlE02-2-0797 | 799.209 | OEt | a1 | B08 | b2 | C03 | c2 | D02 | d1 | E02 |
| 2-1-m1E02-2-0798 | 799.213 | OEt | a1 | B09 | b2 | C01 | c2 | D02 | d1 | E02 |
| 2-1-mlE02-2-0799 | 799.225 | OEt | a1 | B10 | b1 | C10 | c2 | D02 | d1 | E02 |
| 2-1-m1E02-2-0800 | 799.230 | OEt | a1 | B04 | b1 | C09 | c2 | D02 | d1 | E02 |
| 2-1-mlE02-2-0801 | 799.235 | OEt | a1 | B06 | b3 | C17 | c1 | D01 | d1 | E02 |
| 2-1-mlE02-2-0802 | 799.235 | OEt | a1 | B06 | b1 | C14 | c2 | D02 | d1 | E02 |
| 2-1-m1E02-2-0803 | 799.305 | OEt | a1 | B04 | b1 | C31 | c3 | D02 | d1 | E02 |
| 2-1-mlE02-2-0804 | 800.146 | OEt | a2 | B17 | b3 | C06 | c2 | D02 | d1 | E02 |
| 2-1-m1E02-2-0805 | 800.234 | OEt | a2 | B18 | b3 | C17 | c1 | D01 | d1 | E02 |
| 2-1-mlE02-2-0806 | 800.234 | OEt | a2 | B18 | b1 | C14 | c2 | D02 | d1 | E02 |
| 2-1-m1E02-2-0807 | 801.161 | OEt | a1 | B04 | b3 | C06 | c2 | D02 | d1 | E02 |
| 2-1-m1E02-2-0808 | 801.210 | OEt | a2 | B17 | b3 | C18 | c1 | D01 | d1 | E02 |
| 2-1-mlE02-2-0809 | 801.239 | OEt | a1 | B07 | b1 | C13 | c2 | D02 | d1 | E02 |
| 2-1-mlE02-2-0810 | 801.260 | OEt | a1 | B10 | b1 | C09 | c1 | D01 | d1 | E02 |
| 2-1-mlE02-2-0811 | 801.341 | OEt | a1 | B05 | b2 | C07 | c2 | D02 | d1 | E02 |
| 2-1-mlE02-2-0812 | 802.225 | OEt | a1 | B04 | b3 | C18 | c1 | D01 | d1 | E02 |
| 2-1-mlE02-2-0813 | 802.225 | OEt | a1 | B08 | b2 | C24 | c3 | D02 | d1 | E02 |
| 2-1-m1E02-2-0814 | 802.301 | OEt | a2 | B19 | b1 | C29 | c3 | D02 | d1 | E02 |
| 2-1-m1E02-2-0815 | 803.174 | OEt | a1 | B07 | b3 | C15 | c2 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0816 | 803.191 | OEt | a1 | B10 | b3 | C06 | c1 | D01 | d1 | E02 |
| 2-1-m1E02-2-0817 | 803.244 | OEt | a1 | B07 | b3 | C16 | c1 | D01 | d1 | E02 |
| 2-1-m1E02-2-0818 | 803.320 | OEt | a1 | B06 | b2 | C07 | c2 | D02 | d1 | E02 |
| 2-1-m1E02-2-0819 | 804.171 | OEt | a1 | B08 | b2 | C06 | c2 | D02 | d1 | E02 |
| 2-1-m1E02-2-0820 | 804.186 | OEt | a1 | B10 | b1 | C12 | c2 | D02 | d1 | E02 |
| 2-1-m1E02-2-0821 | 804.280 | OEt | a2 | B20 | b1 | C32 | c3 | D02 | d1 | E02 |
| 2-1-m1E02-2-0822 | 804.319 | OEt | a2 | B18 | b2 | C07 | c2 | D02 | d1 | E02 |
| 2-1-m1E02-2-0823 | 805.205 | OEt | a1 | B07 | b3 | C17 | c1 | D01 | d1 | E02 |
| 2-1-m1E02-2-0824 | 805.205 | OEt | a1 | B07 | b1 | C14 | c2 | D02 | d1 | E02 |
| 2-1-m1E02-2-0825 | 805.239 | OEt | a2 | B19 | b2 | C08 | c2 | D02 | d1 | E02 |
| 2-1-m1E02-2-0826 | 805.245 | OEt | a1 | B05 | b3 | C08 | c2 | D02 | d1 | E02 |
| 2-1-m1E02-2-0827 | 806.223 | OEt | a2 | B20 | b2 | C02 | c2 | D02 | d1 | E02 |
| 2-1-m1E02-2-0828 | 806.260 | OEt | a2 | B20 | b2 | C22 | c3 | D02 | d1 | E02 |
| 2-1-m1E02-2-0829 | 806.260 | OEt | a2 | B19 | b2 | C26 | c3 | D02 | d1 | E02 |
| 2-1-m1E02-2-0830 | 807.219 | OEt | a2 | B20 | b2 | C03 | c2 | D02 | d1 | E02 |
| 2-1-m1E02-2-0831 | 807.224 | OEt | a1 | B06 | b3 | C08 | c2 | D02 | d1 | E02 |
| 2-1-m1E02-2-0832 | 807.291 | OEt | a1 | B09 | b1 | C32 | c3 | D02 | d1 | E02 |
| 2-1-m1E02-2-0833 | 808.219 | OEt | a2 | B19 | b2 | C04 | c2 | D02 | d1 | E02 |
| 2-1-m1E02-2-0834 | 808.224 | OEt | a2 | B18 | b3 | C08 | c2 | D02 | d1 | E02 |
| 2-1-m1E02-2-0835 | 809.234 | OEt | a1 | B09 | b2 | C02 | c2 | D02 | d1 | E02 |
| 2-1-m1E02-2-0836 | 809.271 | OEt | a1 | B05 | b1 | C09 | c2 | D02 | d1 | E02 |
| 2-1-m1E02-2-0837 | 809.271 | OEt | a1 | B09 | b2 | C22 | c3 | D02 | d1 | E02 |
| 2-1-m1E02-2-0838 | 809.271 | OEt | a1 | B10 | b1 | C30 | c3 | D02 | d1 | E02 |
| 2-1-m1E02-2-0839 | 809.291 | OEt | a1 | B07 | b2 | C07 | c2 | D02 | d1 | E02 |
| 2-1-m1E02-2-0840 | 809.346 | OEt | a1 | B05 | b1 | C31 | c3 | D02 | d1 | E02 |
| 2-1-m1E02-2-0841 | 810.229 | OEt | a1 | B09 | b2 | C03 | c2 | D02 | d1 | E02 |
| 2-1-m1E02-2-0842 | 810.235 | OEt | a2 | B20 | b2 | C24 | c3 | D02 | d1 | E02 |
| 2-1-m1E02-2-0843 | 810.287 | OEt | a1 | B08 | b1 | C29 | c3 | D02 | d1 | E02 |
| 2-1-m1E02-2-0844 | 811.202 | OEt | a1 | B05 | b3 | C06 | c2 | D02 | d1 | E02 |
| 2-1-m1E02-2-0845 | 811.250 | OEt | a1 | B06 | b1 | C09 | c2 | D02 | d1 | E02 |
| 2-1-m1E02-2-0846 | 811.250 | OEt | a1 | B10 | b2 | C20 | c3 | D02 | d1 | E02 |
| 2-1-m1E02-2-0847 | 811.325 | OEt | a1 | B06 | b1 | C31 | c3 | D02 | d1 | E02 |
| 2-1-m1E02-2-0848 | 812.180 | OEt | a2 | B20 | b2 | C06 | c2 | D02 | d1 | E02 |
| 2-1-m1E02-2-0849 | 812.219 | OEt | a2 | B11 | b3 | C16 | c2 | D02 | d1 | E02 |
| 2-1-m1E02-2-0850 | 812.245 | OEt | a1 | B10 | b2 | C28 | c3 | D02 | d1 | E02 |
| 2-1-m1E02-2-0851 | 812.249 | OEt | a2 | B18 | b1 | C09 | c2 | D02 | d1 | E02 |
| 2-1-m1E02-2-0852 | 812.266 | OEt | a1 | B05 | b3 | C18 | c1 | D01 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0853 | 812.324 | OEt | a2 | B18 | b1 | C31 | c3 | D02 | d1 | E02 |
| 2-1-m1E02-2-0854 | 813.181 | OEt | a1 | B06 | b3 | C06 | c2 | D02 | d1 | E02 |
| 2-1-m1E02-2-0855 | 813.195 | OEt | a1 | B07 | b3 | C08 | c2 | D02 | d1 | E02 |
| 2-1-m1E02-2-0856 | 813.214 | OEt | a2 | B12 | b3 | C16 | c2 | D02 | d1 | E02 |
| 2-1-m1E02-2-0857 | 813.225 | OEt | a1 | B08 | b2 | C05 | c2 | D02 | d1 | E02 |
| 2-1-m1E02-2-0858 | 813.246 | OEt | a1 | B09 | b2 | C24 | c3 | D02 | d1 | E02 |
| 2-1-m1E02-2-0859 | 814.180 | OEt | a2 | B18 | b3 | C06 | c2 | D02 | d1 | E02 |
| 2-1-m1E02-2-0860 | 814.180 | OEt | a2 | B11 | b3 | C17 | c2 | D02 | d1 | E02 |
| 2-1-m1E02-2-0861 | 814.240 | OEt | a2 | B19 | b1 | C13 | c2 | D02 | d1 | E02 |
| 2-1-m1E02-2-0862 | 814.245 | OEt | a1 | B06 | b3 | C18 | c1 | D01 | d1 | E02 |
| 2-1-m1E02-2-0863 | 814.245 | OEt | a1 | B08 | b2 | C26 | c3 | D02 | d1 | E02 |
| 2-1-m1E02-2-0864 | 815.175 | OEt | a2 | B12 | b3 | C17 | c2 | D02 | d1 | E02 |
| 2-1-m1E02-2-0865 | 815.191 | OEt | a1 | B09 | b2 | C06 | c2 | D02 | d1 | E02 |
| 2-1-m1E02-2-0866 | 815.208 | OEt | a1 | B10 | b2 | C01 | c2 | D02 | d1 | E02 |
| 2-1-m1E02-2-0867 | 815.245 | OEt | a2 | B18 | b3 | C18 | c1 | D01 | d1 | E02 |
| 2-1-m1E02-2-0868 | 816.175 | OEt | a2 | B19 | b3 | C15 | c2 | D02 | d1 | E02 |
| 2-1-m1E02-2-0869 | 816.205 | OEt | a1 | B08 | b2 | C04 | c2 | D02 | d1 | E02 |
| 2-1-m1E02-2-0870 | 816.244 | OEt | a2 | B19 | b3 | C16 | c1 | D01 | d1 | E02 |
| 2-1-m1E02-2-0871 | 817.220 | OEt | a1 | B07 | b1 | C09 | c2 | D02 | d1 | E02 |
| 2-1-m1E02-2-0872 | 817.296 | OEt | a1 | B07 | b1 | C31 | c3 | D02 | d1 | E02 |
| 2-1-m1E02-2-0873 | 818.206 | OEt | a2 | B19 | b3 | C17 | c1 | D01 | d1 | E02 |
| 2-1-m1E02-2-0874 | 818.206 | OEt | a2 | B19 | b1 | C14 | c2 | D02 | d1 | E02 |
| 2-1-m1E02-2-0875 | 818.296 | OEt | a2 | B20 | b1 | C29 | c3 | D02 | d1 | E02 |
| 2-1-m1E02-2-0876 | 819.151 | OEt | a1 | B07 | b3 | C06 | c2 | D02 | d1 | E02 |
| 2-1-m1E02-2-0877 | 820.216 | OEt | a1 | B07 | b3 | C18 | c1 | D01 | d1 | E02 |
| 2-1-m1E02-2-0878 | 821.234 | OEt | a2 | B20 | b2 | C05 | c2 | D02 | d1 | E02 |
| 2-1-m1E02-2-0879 | 821.307 | OEt | a1 | B09 | b1 | C29 | c3 | D02 | d1 | E02 |
| 2-1-m1E02-2-0880 | 822.225 | OEt | a1 | B08 | b1 | C13 | c2 | D02 | d1 | E02 |
| 2-1-m1E02-2-0881 | 822.255 | OEt | a2 | B20 | b2 | C26 | c3 | D02 | d1 | E02 |
| 2-1-m1E02-2-0882 | 822.291 | OEt | a2 | B19 | b2 | C07 | c2 | D02 | d1 | E02 |
| 2-1-m1E02-2-0883 | 823.286 | OEt | a1 | B10 | b1 | C32 | c3 | D02 | d1 | E02 |
| 2-1-m1E02-2-0884 | 824.160 | OEt | a1 | B08 | b3 | C15 | c2 | D02 | d1 | E02 |
| 2-1-m1E02-2-0885 | 824.214 | OEt | a2 | B20 | b2 | C04 | c2 | D02 | d1 | E02 |
| 2-1-m 1 E02-2-0886 | 824.230 | OEt | a1 | B08 | b3 | C16 | c1 | D01 | d1 | E02 |
| 2-1-m1E02-2-0887 | 824.245 | OEt | a1 | B09 | b2 | C05 | c2 | D02 | d1 | E02 |
| 2-1-m1E02-2-0888 | 825.229 | OEt | a1 | B10 | b2 | C02 | c2 | D02 | d1 | E02 |
| 2-1-m1E02-2-0889 | 825.266 | OEt | a1 | B10 | b2 | C22 | c3 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0890 | 825.266 | OEt | a1 | B09 | b2 | C26 | c3 | D02 | d1 | E02 |
| 2-1-m1E02-2-0891 | 826.191 | OEt | a1 | B08 | b3 | C17 | c1 | D01 | d1 | E02 |
| 2-1-m1E02-2-0892 | 826.191 | OEt | a1 | B08 | b1 | C14 | c2 | D02 | d1 | E02 |
| 2-1-m1E02-2-0893 | 826.195 | OEt | a2 | B19 | b3 | C08 | c2 | D02 | d1 | E02 |
| 2-1-m1E02-2-0894 | 826.224 | OEt | a1 | B10 | b2 | C03 | c2 | D02 | d1 | E02 |
| 2-1-m1E02-2-0895 | 826.234 | OEt | a2 | B13 | b3 | C16 | c2 | D02 | d1 | E02 |
| 2-1-m1E02-2-0896 | 827.225 | OEt | a1 | B09 | b2 | C04 | c2 | D02 | d1 | E02 |
| 2-1-m1E02-2-0897 | 828.196 | OEt | a2 | B13 | b3 | C17 | c2 | D02 | d1 | E02 |
| 2-1-m1E02-2-0898 | 829.191 | OEt | a2 | B11 | b3 | C18 | c2 | D02 | d1 | E02 |
| 2-1-m1E02-2-0899 | 829.240 | OEt | a1 | B10 | b2 | C24 | c3 | D02 | d1 | E02 |
| 2-1-m1E02-2-0900 | 830.186 | OEt | a2 | B12 | b3 | C18 | c2 | D02 | d1 | E02 |
| 2-1-m1E02-2-0901 | 830.209 | OEt | a2 | B14 | b3 | C16 | c2 | D02 | d1 | E02 |
| 2-1-m1E02-2-0902 | 830.221 | OEt | a2 | B19 | b1 | C09 | c2 | D02 | d1 | E02 |
| 2-1-m1E02-2-0903 | 830.235 | OEt | a2 | B20 | b1 | C13 | c2 | D02 | d1 | E02 |
| 2-1-m1E02-2-0904 | 830.277 | OEt | a1 | B08 | b2 | C07 | c2 | D02 | d1 | E02 |
| 2-1-m1E02-2-0905 | 830.296 | OEt | a2 | B19 | b1 | C31 | c3 | D02 | d1 | E02 |
| 2-1-m1E02-2-0906 | 831.186 | OEt | a1 | B10 | b2 | C06 | c2 | D02 | d1 | E02 |
| 2-1-m1E02-2-0907 | 831.224 | OEt | a1 | B01 | b3 | C16 | c2 | D02 | d1 | E02 |
| 2-1-m1E02-2-0908 | 832.152 | OEt | a2 | B19 | b3 | C06 | c2 | D02 | d1 | E02 |
| 2-1-m1E02-2-0909 | 832.170 | OEt | a2 | B20 | b3 | C15 | c2 | D02 | d1 | E02 |
| 2-1-m1E02-2-0910 | 832.171 | OEt | a2 | B14 | b3 | C17 | c2 | D02 | d1 | E02 |
| 2-1-m1E02-2-0911 | 832.220 | OEt | a1 | B02 | b3 | C16 | c2 | D02 | d1 | E02 |
| 2-1-m1E02-2-0912 | 832.239 | OEt | a2 | B20 | b3 | C16 | c1 | D01 | d1 | E02 |
| 2-1-m1E02-2-0913 | 833.186 | OEt | a1 | B01 | b3 | C17 | c2 | D02 | d1 | E02 |
| 2-1-m1E02-2-0914 | 833.216 | OEt | a2 | B19 | b3 | C18 | c1 | D01 | d1 | E02 |
| 2-1-m1E02-2-0915 | 833.245 | OEt | a1 | B09 | b1 | C13 | c2 | D02 | d1 | E02 |
| 2-1-m1E02-2-0916 | 834.181 | OEt | a1 | B02 | b3 | C17 | c2 | D02 | d1 | E02 |
| 2-1-m1E02-2-0917 | 834.181 | OEt | a1 | B08 | b3 | C08 | c2 | D02 | d1 | E02 |
| 2-1-m1E02-2-0918 | 834.200 | OEt | a2 | B20 | b3 | C17 | c1 | D01 | d1 | E02 |
| 2-1-m1E02-2-0919 | 834.200 | OEt | a2 | B20 | b1 | C14 | c2 | D02 | d1 | E02 |
| 2-1-m1E02-2-0920 | 835.180 | OEt | a1 | B09 | b3 | C15 | c2 | D02 | d1 | E02 |
| 2-1-m1E02-2-0921 | 835.250 | OEt | a1 | B09 | b3 | C16 | c1 | D01 | d1 | E02 |
| 2-1-m1E02-2-0922 | 837.211 | OEt | a1 | B09 | b3 | C17 | c1 | D01 | d1 | E02 |
| 2-1-m1E02-2-0923 | 837.211 | OEt | a1 | B09 | b1 | C14 | c2 | D02 | d1 | E02 |
| 2-1-m1E02-2-0924 | 837.302 | OEt | a1 | B10 | b1 | C29 | c3 | D02 | d1 | E02 |
| 2-1-m1E02-2-0925 | 838.207 | OEt | a1 | B08 | b1 | C09 | c2 | D02 | d1 | E02 |
| 2-1-m1E02-2-0926 | 838.282 | OEt | a1 | B08 | b1 | C31 | c3 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0927 | 838.286 | OEt | a2 | B20 | b2 | C07 | c2 | D02 | d1 | E02 |
| 2-1-m1E02-2-0928 | 840.138 | OEt | a1 | B08 | b3 | C06 | c2 | D02 | d1 | E02 |
| 2-1-m1E02-2-0929 | 840.240 | OEt | a1 | B10 | b2 | C05 | c2 | D02 | d1 | E02 |
| 2-1-m1E02-2-0930 | 840.250 | OEt | a2 | B15 | b3 | C16 | c2 | D02 | d1 | E02 |
| 2-1-m1E02-2-0931 | 841.202 | OEt | a1 | B08 | b3 | C18 | c1 | D01 | d1 | E02 |
| 2-1-m1E02-2-0932 | 841.260 | OEt | a1 | B10 | b2 | C26 | c3 | D02 | d1 | E02 |
| 2-1-m1E02-2-0933 | 841.297 | OEt | a1 | B09 | b2 | C07 | c2 | D02 | d1 | E02 |
| 2-1-m1E02-2-0934 | 842.190 | OEt | a2 | B20 | b3 | C08 | c2 | D02 | d1 | E02 |
| 2-1-m1E02-2-0935 | 842.211 | OEt | a2 | B15 | b3 | C17 | c2 | D02 | d1 | E02 |
| 2-1-m1E02-2-0936 | 842.229 | OEt | a2 | B16 | b3 | C16 | c2 | D02 | d1 | E02 |
| 2-1-m1E02-2-0937 | 843.207 | OEt | a2 | B13 | b3 | C18 | c2 | D02 | d1 | E02 |
| 2-1-m1E02-2-0938 | 843.220 | OEt | a1 | B10 | b2 | C04 | c2 | D02 | d1 | E02 |
| 2-1-m1E02-2-0939 | 844.191 | OEt | a2 | B16 | b3 | C17 | c2 | D02 | d1 | E02 |
| 2-1-m1E02-2-0940 | 845.201 | OEt | a1 | B09 | b3 | C08 | c2 | D02 | d1 | E02 |
| 2-1-m1E02-2-0941 | 845.240 | OEt | a1 | B03 | b3 | C16 | c2 | D02 | d1 | E02 |
| 2-1-m1E02-2-0942 | 846.216 | OEt | a2 | B20 | b1 | C09 | c2 | D02 | d1 | E02 |
| 2-1-m1E02-2-0943 | 846.291 | OEt | a2 | B20 | b1 | C31 | c3 | D02 | d1 | E02 |
| 2-1-m1E02-2-0944 | 847.182 | OEt | a2 | B14 | b3 | C18 | c2 | D02 | d1 | E02 |
| 2-1-m1E02-2-0945 | 847.202 | OEt | a1 | B03 | b3 | C17 | c2 | D02 | d1 | E02 |
| 2-1-m1E02-2-0946 | 848.147 | OEt | a2 | B20 | b3 | C06 | c2 | D02 | d1 | E02 |
| 2-1-m1E02-2-0947 | 848.197 | OEt | a1 | B01 | b3 | C18 | c2 | D02 | d1 | E02 |
| 2-1-m1E02-2-0948 | 848.200 | OEt | a2 | B17 | b3 | C16 | c2 | D02 | d1 | E02 |
| 2-1-m1E02-2-0949 | 849.192 | OEt | a1 | B02 | b3 | C18 | c2 | D02 | d1 | E02 |
| 2-1-m1E02-2-0950 | 849.211 | OEt | a2 | B20 | b3 | C18 | c1 | D01 | d1 | E02 |
| 2-1-m1E02-2-0951 | 849.215 | OEt | a1 | B04 | b3 | C16 | c2 | D02 | d1 | E02 |
| 2-1-m1E02-2-0952 | 849.227 | OEt | a1 | B09 | b1 | C09 | c2 | D02 | d1 | E02 |
| 2-1-m1E02-2-0953 | 849.240 | OEt | a1 | B10 | b1 | C13 | c2 | D02 | d1 | E02 |
| 2-1-m1E02-2-0954 | 849.302 | OEt | a1 | B09 | b1 | C31 | c3 | D02 | d1 | E02 |
| 2-1-m1E02-2-0955 | 850.161 | OEt | a2 | B17 | b3 | C17 | c2 | D02 | d1 | E02 |
| 2-1-m1E02-2-0956 | 851.158 | OEt | a1 | B09 | b3 | C06 | c2 | D02 | d1 | E02 |
| 2-1-m1E02-2-0957 | 851.175 | OEt | a1 | B10 | b3 | C15 | c2 | D02 | d1 | E02 |
| 2-1-m1E02-2-0958 | 851.176 | OEt | a1 | B04 | b3 | C17 | c2 | D02 | d1 | E02 |
| 2-1-m1E02-2-0959 | 851.245 | OEt | a1 | B10 | b3 | C16 | c1 | D01 | d1 | E02 |
| 2-1-m1E02-2-0960 | 852.222 | OEt | a1 | B09 | b3 | C18 | c1 | D01 | d1 | E02 |
| 2-1-m1E02-2-0961 | 853.206 | OEt | a1 | B10 | b3 | C17 | c1 | D01 | d1 | E02 |
| 2-1-m1E02-2-0962 | 853.206 | OEt | a1 | B10 | b1 | C14 | c2 | D02 | d1 | E02 |
| 2-1-m1E02-2-0963 | 857.222 | OEt | a2 | B15 | b3 | C18 | c2 | D02 | d1 | E02 |

(continued)

| [Table 8-10-3] Compound that may be contained in mixture 2-1-mlE02-2 | | | | | | | | | | |
|---|---|---|---|---|---|---|---|---|---|---|
| ID | Exact Mass | | a | B | b | C | c | D | d | E |
| 2-1-m1E02-2-0964 | 857.292 | OEt | a1 | B10 | b2 | C07 | c2 | D02 | d1 | E02 |
| 2-1-m1E02-2-0965 | 859.202 | OEt | a2 | B16 | b3 | C18 | c2 | D02 | d1 | E02 |
| 2-1-m1E02-2-0966 | 859.256 | OEt | a1 | B05 | b3 | C16 | c2 | D02 | d1 | E02 |
| 2-1-m1E02-2-0967 | 861.196 | OEt | a1 | B10 | b3 | C08 | c2 | D02 | d1 | E02 |
| 2-1-m1E02-2-0968 | 861.217 | OEt | a1 | B05 | b3 | C17 | c2 | D02 | d1 | E02 |
| 2-1-m1E02-2-0969 | 861.235 | OEt | a1 | B06 | b3 | C16 | c2 | D02 | d1 | E02 |
| 2-1-m1E02-2-0970 | 862.212 | OEt | a1 | B03 | b3 | C18 | c2 | D02 | d1 | E02 |
| 2-1-m1E02-2-0971 | 862.234 | OEt | a2 | B18 | b3 | C16 | c2 | D02 | d1 | E02 |
| 2-1-m1E02-2-0972 | 863.196 | OEt | a1 | B06 | b3 | C17 | c2 | D02 | d1 | E02 |
| 2-1-m1E02-2-0973 | 864.196 | OEt | a2 | B18 | b3 | C17 | c2 | D02 | d1 | E02 |
| 2-1-m1E02-2-0974 | 865.172 | OEt | a2 | B17 | b3 | C18 | c2 | D02 | d1 | E02 |
| 2-1-m1E02-2-0975 | 865.222 | OEt | a1 | B10 | b1 | C09 | c2 | D02 | d1 | E02 |
| 2-1-m1E02-2-0976 | 865.297 | OEt | a1 | B10 | b1 | C31 | c3 | D02 | d1 | E02 |
| 2-1-m1E02-2-0977 | 866.187 | OEt | a1 | B04 | b3 | C18 | c2 | D02 | d1 | E02 |
| 2-1-m1E02-2-0978 | 867.153 | OEt | a1 | B10 | b3 | C06 | c2 | D02 | d1 | E02 |
| 2-1-m1E02-2-0979 | 867.206 | OEt | a1 | B07 | b3 | C16 | c2 | D02 | d1 | E02 |
| 2-1-m1E02-2-0980 | 868.217 | OEt | a1 | B10 | b3 | C18 | c1 | D01 | d1 | E02 |
| 2-1-m1E02-2-0981 | 869.167 | OEt | a1 | B07 | b3 | C17 | c2 | D02 | d1 | E02 |
| 2-1-m1E02-2-0982 | 876.228 | OEt | a1 | B05 | b3 | C18 | c2 | D02 | d1 | E02 |
| 2-1-m1E02-2-0983 | 878.207 | OEt | a1 | B06 | b3 | C18 | c2 | D02 | d1 | E02 |
| 2-1-m1E02-2-0984 | 879.207 | OEt | a2 | B18 | b3 | C18 | c2 | D02 | d1 | E02 |
| 2-1-m1E02-2-0985 | 880.206 | OEt | a2 | B19 | b3 | C16 | c2 | D02 | d1 | E02 |
| 2-1-m1E02-2-0986 | 882.167 | OEt | a2 | B19 | b3 | C17 | c2 | D02 | d1 | E02 |
| 2-1-m1E02-2-0987 | 884.178 | OEt | a1 | B07 | b3 | C18 | c2 | D02 | d1 | E02 |
| 2-1-m1E02-2-0988 | 888.192 | OEt | a1 | B08 | b3 | C16 | c2 | D02 | d1 | E02 |
| 2-1-m1E02-2-0989 | 890.153 | OEt | a1 | B08 | b3 | C17 | c2 | D02 | d1 | E02 |
| 2-1-m1E02-2-0990 | 896.201 | OEt | a2 | B20 | b3 | C16 | c2 | D02 | d1 | E02 |
| 2-1-m1E02-2-0991 | 897.178 | OEt | a2 | B19 | b3 | C18 | c2 | D02 | d1 | E02 |
| 2-1-m1E02-2-0992 | 898.162 | OEt | a2 | B20 | b3 | C17 | c2 | D02 | d1 | E02 |
| 2-1-m1E02-2-0993 | 899.212 | OEt | a1 | B09 | b3 | C16 | c2 | D02 | d1 | E02 |
| 2-1-m1E02-2-0994 | 901.173 | OEt | a1 | B09 | b3 | C17 | c2 | D02 | d1 | E02 |
| 2-1-m1E02-2-0995 | 905.164 | OEt | a1 | B08 | b3 | C18 | c2 | D02 | d1 | E02 |
| 2-1-m1E02-2-0996 | 913.173 | OEt | a2 | B20 | b3 | C18 | c2 | D02 | d1 | E02 |
| 2-1-m1E02-2-0997 | 915.207 | OEt | a1 | B10 | b3 | C16 | c2 | D02 | d1 | E02 |
| 2-1-m1E02-2-0998 | 916.184 | OEt | a1 | B09 | b3 | C18 | c2 | D02 | d1 | E02 |
| 2-1-m1E02-2-0999 | 917.168 | OEt | a1 | B10 | b3 | C17 | c2 | D02 | d1 | E02 |
| 2-1-m1E02-2-1000 | 932.179 | OEt | a1 | B10 | b3 | C18 | c2 | D02 | d1 | E02 |

[Table 404]

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0001 | 668.246 | OEt | a2 | B11 | b1 | C08 | c1 | D01 | d1 | E03 |
| 2-1-m1E03-2-0002 | 669.241 | OEt | a2 | B12 | b1 | C08 | c1 | D01 | d1 | E03 |
| 2-1-m1E03-2-0003 | 669.241 | OEt | a2 | B11 | b1 | C11 | c1 | D01 | d1 | E03 |
| 2-1-m1E03-2-0004 | 670.236 | OEt | a2 | B11 | b1 | C10 | c1 | D01 | d1 | E03 |
| 2-1-m1E03-2-0005 | 670.236 | OEt | a2 | B12 | b1 | C11 | c1 | D01 | d1 | E03 |
| 2-1-m1E03-2-0006 | 671.231 | OEt | a2 | B12 | b1 | C10 | c1 | D01 | d1 | E03 |
| 2-1-m1E03-2-0007 | 675.197 | OEt | a2 | B11 | b1 | C12 | c1 | D01 | d1 | E03 |
| 2-1-m1E03-2-0008 | 676.193 | OEt | a2 | B12 | b1 | C12 | c1 | D01 | d1 | E03 |
| 2-1-m1E03-2-0009 | 682.261 | OEt | a2 | B13 | b1 | C08 | c1 | D01 | d1 | E03 |
| 2-1-m1E03-2-0010 | 683.257 | OEt | a2 | B13 | b1 | C11 | c1 | D01 | d1 | E03 |
| 2-1-m1E03-2-0011 | 684.252 | OEt | a2 | B13 | b1 | C10 | c1 | D01 | d1 | E03 |
| 2-1-m1E03-2-0012 | 686.220 | OEt | a2 | B11 | b2 | C01 | c1 | D01 | d1 | E03 |
| 2-1-m1E03-2-0013 | 686.236 | OEt | a2 | B14 | b1 | C08 | c1 | D01 | d1 | E03 |
| 2-1-m1E03-2-0014 | 687.215 | OEt | a2 | B12 | b2 | C01 | c1 | D01 | d1 | E03 |
| 2-1-m1E03-2-0015 | 687.232 | OEt | a2 | B14 | b1 | C11 | c1 | D01 | d1 | E03 |
| 2-1-m1E03-2-0016 | 687.252 | OEt | a1 | B01 | b1 | C08 | c1 | D01 | d1 | E03 |
| 2-1-m1E03-2-0017 | 688.227 | OEt | a2 | B14 | b1 | C10 | c1 | D01 | d1 | E03 |
| 2-1-m1E03-2-0018 | 688.247 | OEt | a1 | B02 | b1 | C08 | c1 | D01 | d1 | E03 |
| 2-1-m1E03-2-0019 | 688.247 | OEt | a1 | B01 | b1 | C11 | c1 | D01 | d1 | E03 |
| 2-1-m1E03-2-0020 | 689.213 | OEt | a2 | B13 | b1 | C12 | c1 | D01 | d1 | E03 |
| 2-1-m1E03-2-0021 | 689.242 | OEt | a1 | B01 | b1 | C10 | c1 | D01 | d1 | E03 |
| 2-1-m1E03-2-0022 | 689.242 | OEt | a1 | B02 | b1 | C11 | c1 | D01 | d1 | E03 |
| 2-1-m 1 E03-2-0023 | 690.237 | OEt | a1 | B02 | b1 | C10 | c1 | D01 | d1 | E03 |
| 2-1-m1E03-2-0024 | 693.188 | OEt | a2 | B14 | b1 | C12 | c1 | D01 | d1 | E03 |
| 2-1-m1E03-2-0025 | 694.203 | OEt | a1 | B01 | b1 | C12 | c1 | D01 | d1 | E03 |
| 2-1-m1E03-2-0026 | 695.198 | OEt | a1 | B02 | b1 | C12 | c1 | D01 | d1 | E03 |
| 2-1-m1E03-2-0027 | 696.241 | OEt | a2 | B11 | b2 | C02 | c1 | D01 | d1 | E03 |
| 2-1-m1E03-2-0028 | 696.277 | OEt | a2 | B15 | b1 | C08 | c1 | D01 | d1 | E03 |
| 2-1-m1E03-2-0029 | 697.236 | OEt | a2 | B12 | b2 | C02 | c1 | D01 | d1 | E03 |
| 2-1-m1E03-2-0030 | 697.236 | OEt | a2 | B11 | b2 | C03 | c1 | D01 | d1 | E03 |
| 2-1-m1E03-2-0031 | 697.272 | OEt | a2 | B15 | b1 | C11 | c1 | D01 | d1 | E03 |
| 2-1-m1E03-2-0032 | 698.231 | OEt | a2 | B12 | b2 | C03 | c1 | D01 | d1 | E03 |
| 2-1-m1E03-2-0033 | 698.256 | OEt | a2 | B16 | b1 | C08 | c1 | D01 | d1 | E03 |
| 2-1-m1E03-2-0034 | 698.268 | OEt | a2 | B15 | b1 | C10 | c1 | D01 | d1 | E03 |
| 2-1-m1E03-2-0035 | 699.252 | OEt | a2 | B16 | b1 | C11 | c1 | D01 | d1 | E03 |
| 2-1-m1E03-2-0036 | 700.236 | OEt | a2 | B13 | b2 | C01 | c1 | D01 | d1 | E03 |
| 2-1-m1E03-2-0037 | 700.247 | OEt | a2 | B16 | b1 | C10 | c1 | D01 | d1 | E03 |
| 2-1-m1E03-2-0038 | 701.267 | OEt | a1 | B03 | b1 | C08 | c1 | D01 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0039 | 702.197 | OEt | a2 | B11 | b2 | C06 | c1 | D01 | d1 | E03 |
| 2-1-m1E03-2-0040 | 702.262 | OEt | a1 | B03 | b1 | C11 | c1 | D01 | d1 | E03 |
| 2-1-m1E03-2-0041 | 703.192 | OEt | a2 | B12 | b2 | C06 | c1 | D01 | d1 | E03 |
| 2-1-m1E03-2-0042 | 703.229 | OEt | a2 | B15 | b1 | C12 | c1 | D01 | d1 | E03 |
| 2-1-m1E03-2-0043 | 703.258 | OEt | a1 | B03 | b1 | C10 | c1 | D01 | d1 | E03 |
| 2-1-m1E03-2-0044 | 704.210 | OEt | a2 | B14 | b2 | C01 | c1 | D01 | d1 | E03 |
| 2-1-m1E03-2-0045 | 704.227 | OEt | a2 | B17 | b1 | C08 | c1 | D01 | d1 | E03 |
| 2-1-m1E03-2-0046 | 705.208 | OEt | a2 | B16 | b1 | C12 | c1 | D01 | d1 | E03 |
| 2-1-m1E03-2-0047 | 705.222 | OEt | a2 | B17 | b1 | C11 | c1 | D01 | d1 | E03 |
| 2-1-m1E03-2-0048 | 705.226 | OEt | a1 | B01 | b2 | C01 | c1 | D01 | d1 | E03 |
| 2-1-m1E03-2-0049 | 705.242 | OEt | a1 | B04 | b1 | C08 | c1 | D01 | d1 | E03 |
| 2-1-m1E03-2-0050 | 706.217 | OEt | a2 | B17 | b1 | C10 | c1 | D01 | d1 | E03 |
| 2-1-m1E03-2-0051 | 706.221 | OEt | a1 | B02 | b2 | C01 | c1 | D01 | d1 | E03 |
| 2-1-m1E03-2-0052 | 706.237 | OEt | a1 | B04 | b1 | C11 | c1 | D01 | d1 | E03 |
| 2-1-m1E03-2-0053 | 707.233 | OEt | a1 | B04 | b1 | C10 | c1 | D01 | d1 | E03 |
| 2-1-m1E03-2-0054 | 708.219 | OEt | a1 | B03 | b1 | C12 | c1 | D01 | d1 | E03 |
| 2-1-m1E03-2-0055 | 710.256 | OEt | a2 | B13 | b2 | C02 | c1 | D01 | d1 | E03 |
| 2-1-m1E03-2-0056 | 711.179 | OEt | a2 | B17 | b1 | C12 | c1 | D01 | d1 | E03 |
| 2-1-m1E03-2-0057 | 711.252 | OEt | a2 | B13 | b2 | C03 | c1 | D01 | d1 | E03 |
| 2-1-m1E03-2-0058 | 711.252 | OEt | a2 | B11 | b2 | C05 | c1 | D01 | d1 | E03 |
| 2-1-m1E03-2-0059 | 712.194 | OEt | a1 | B04 | b1 | C12 | c1 | D01 | d1 | E03 |
| 2-1-m1E03-2-0060 | 712.247 | OEt | a2 | B12 | b2 | C05 | c1 | D01 | d1 | E03 |
| 2-1-m1E03-2-0061 | 714.231 | OEt | a2 | B14 | b2 | C02 | c1 | D01 | d1 | E03 |
| 2-1-m1E03-2-0062 | 714.231 | OEt | a2 | B11 | b2 | C04 | c1 | D01 | d1 | E03 |
| 2-1-m1E03-2-0063 | 714.251 | OEt | a2 | B15 | b2 | C01 | c1 | D01 | d1 | E03 |
| 2-1-m1E03-2-0064 | 715.226 | OEt | a2 | B12 | b2 | C04 | c1 | D01 | d1 | E03 |
| 2-1-m1E03-2-0065 | 715.226 | OEt | a2 | B14 | b2 | C03 | c1 | D01 | d1 | E03 |
| 2-1-m1E03-2-0066 | 715.246 | OEt | a1 | B01 | b2 | C02 | c1 | D01 | d1 | E03 |
| 2-1-m1E03-2-0067 | 715.283 | OEt | a1 | B05 | b1 | C08 | c1 | D01 | d1 | E03 |
| 2-1-m1E03-2-0068 | 716.213 | OEt | a2 | B13 | b2 | C06 | c1 | D01 | d1 | E03 |
| 2-1-m1E03-2-0069 | 716.230 | OEt | a2 | B16 | b2 | C01 | c1 | D01 | d1 | E03 |
| 2-1-m1E03-2-0070 | 716.242 | OEt | a1 | B02 | b2 | C02 | c1 | D01 | d1 | E03 |
| 2-1-m1E03-2-0071 | 716.242 | OEt | a1 | B01 | b2 | C03 | c1 | D01 | d1 | E03 |
| 2-1-m1E03-2-0072 | 716.278 | OEt | a1 | B05 | b1 | C11 | c1 | D01 | d1 | E03 |
| 2-1-m1E03-2-0073 | 717.237 | OEt | a1 | B02 | b2 | C03 | c1 | D01 | d1 | E03 |
| 2-1-m1E03-2-0074 | 717.262 | OEt | a1 | B06 | b1 | C08 | c1 | D01 | d1 | E03 |
| 2-1-m1E03-2-0075 | 717.273 | OEt | a1 | B05 | b1 | C10 | c1 | D01 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0076 | 718.257 | OEt | a1 | B06 | b1 | C11 | c1 | D01 | d1 | E03 |
| 2-1-m1E03-2-0077 | 718.261 | OEt | a2 | B18 | b1 | C08 | c1 | D01 | d1 | E03 |
| 2-1-m1E03-2-0078 | 719.241 | OEt | a1 | B03 | b2 | C01 | c1 | D01 | d1 | E03 |
| 2-1-m1E03-2-0079 | 719.253 | OEt | a1 | B06 | b1 | C10 | c1 | D01 | d1 | E03 |
| 2-1-m1E03-2-0080 | 719.257 | OEt | a2 | B18 | b1 | C11 | c1 | D01 | d1 | E03 |
| 2-1-m1E03-2-0081 | 720.188 | OEt | a2 | B14 | b2 | C06 | c1 | D01 | d1 | E03 |
| 2-1-m1E03-2-0082 | 720.252 | OEt | a2 | B18 | b1 | C10 | c1 | D01 | d1 | E03 |
| 2-1-m1E03-2-0083 | 720.252 | OEt | a2 | B11 | b1 | C13 | c1 | D01 | d1 | E03 |
| 2-1-m1E03-2-0084 | 721.203 | OEt | a1 | B01 | b2 | C06 | c1 | D01 | d1 | E03 |
| 2-1-m1E03-2-0085 | 721.247 | OEt | a2 | B12 | b1 | C13 | c1 | D01 | d1 | E03 |
| 2-1-m1E03-2-0086 | 722.187 | OEt | a2 | B11 | b3 | C15 | c1 | D01 | d1 | E03 |
| 2-1-m1E03-2-0087 | 722.198 | OEt | a1 | B02 | b2 | C06 | c1 | D01 | d1 | E03 |
| 2-1-m1E03-2-0088 | 722.201 | OEt | a2 | B17 | b2 | C01 | c1 | D01 | d1 | E03 |
| 2-1-m1E03-2-0089 | 722.235 | OEt | a1 | B05 | b1 | C12 | c1 | D01 | d1 | E03 |
| 2-1-mlE03-2-0090 | 723.182 | OEt | a2 | B12 | b3 | C15 | c1 | D01 | d1 | E03 |
| 2-1-mlE03-2-0091 | 723.216 | OEt | a1 | B04 | b2 | C01 | c1 | D01 | d1 | E03 |
| 2-1-mlE03-2-0092 | 723.233 | OEt | a1 | B07 | b1 | C08 | c1 | D01 | d1 | E03 |
| 2-1-mlE03-2-0093 | 724.214 | OEt | a1 | B06 | b1 | C12 | c1 | D01 | d1 | E03 |
| 2-1-mlE03-2-0094 | 724.218 | OEt | a2 | B11 | b1 | C14 | c1 | D01 | d1 | E03 |
| 2-1-mlE03-2-0095 | 724.228 | OEt | a1 | B07 | b1 | C11 | c1 | D01 | d1 | E03 |
| 2-1-mlE03-2-0096 | 724.239 | OEt | a2 | B11 | b2 | C33 | c3 | D02 | d1 | E03 |
| 2-1-mlE03-2-0097 | 724.272 | OEt | a2 | B15 | b2 | C02 | c1 | D01 | d1 | E03 |
| 2-1-m1E03-2-0098 | 725.213 | OEt | a2 | B18 | b1 | C12 | c1 | D01 | d1 | E03 |
| 2-1-mlE03-2-0099 | 725.213 | OEt | a2 | B12 | b1 | C14 | c1 | D01 | d1 | E03 |
| 2-1-mlE03-2-0100 | 725.223 | OEt | a1 | B07 | b1 | C10 | c1 | D01 | d1 | E03 |
| 2-1-m1E03-2-0101 | 725.234 | OEt | a2 | B12 | b2 | C33 | c3 | D02 | d1 | E03 |
| 2-1-mlE03-2-0102 | 725.267 | OEt | a2 | B15 | b2 | C03 | c1 | D01 | d1 | E03 |
| 2-1-m1E03-2-0103 | 725.267 | OEt | a2 | B13 | b2 | COS | c1 | D01 | d1 | E03 |
| 2-1-mlE03-2-0104 | 726.251 | OEt | a2 | B16 | b2 | C02 | c1 | D01 | d1 | E03 |
| 2-1-mlE03-2-0105 | 727.246 | OEt | a2 | B16 | b2 | C03 | c1 | D01 | d1 | E03 |
| 2-1-m1E03-2-0106 | 728.247 | OEt | a2 | B13 | b2 | C04 | c1 | D01 | d1 | E03 |
| 2-1-mlE03-2-0107 | 728.303 | OEt | a2 | B11 | b2 | C07 | c1 | D01 | d1 | E03 |
| 2-1-mlE03-2-0108 | 729.242 | OEt | a2 | B14 | b2 | C05 | c1 | D01 | d1 | E03 |
| 2-1-m1E03-2-0109 | 729.262 | OEt | a1 | B03 | b2 | C02 | c1 | D01 | d1 | E03 |
| 2-1-m1E03-2-0110 | 729.298 | OEt | a2 | B12 | b2 | C07 | c1 | D01 | d1 | E03 |
| 2-1-m1E03-2-0111 | 730.184 | OEt | a1 | B07 | b1 | C12 | c1 | D01 | d1 | E03 |
| 2-1-mlE03-2-0112 | 730.228 | OEt | a2 | B15 | b2 | C06 | c1 | D01 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0113 | 730.257 | OEt | a1 | B03 | b2 | C03 | c1 | D01 | d1 | E03 |
| 2-1-m1E03-2-0114 | 730.257 | OEt | a1 | B01 | b2 | C05 | c1 | D01 | d1 | E03 |
| 2-1-m1E03-2-0115 | 731.253 | OEt | a1 | B02 | b2 | COS | c1 | D01 | d1 | E03 |
| 2-1-mlE03-2-0116 | 732.208 | OEt | a2 | B16 | b2 | C06 | c1 | D01 | d1 | E03 |
| 2-1-m1E03-2-0117 | 732.208 | OEt | a2 | B11 | b3 | C08 | c1 | D01 | d1 | E03 |
| 2-1-m1E03-2-0118 | 732.208 | OEt | a2 | B11 | b1 | C08 | c2 | D02 | d1 | E03 |
| 2-1-m1E03-2-0119 | 732.222 | OEt | a2 | B17 | b2 | C02 | c1 | D01 | d1 | E03 |
| 2-1-m1E03-2-0120 | 732.222 | OEt | a2 | B14 | b2 | C04 | c1 | D01 | d1 | E03 |
| 2-1-m1E03-2-0121 | 733.203 | OEt | a2 | B12 | b3 | C08 | c1 | D01 | d1 | E03 |
| 2-1-m1E03-2-0122 | 733.203 | OEt | a2 | B12 | b1 | C08 | c2 | D02 | d1 | E03 |
| 2-1-mlE03-2-0123 | 733.203 | OEt | a2 | B11 | b1 | C11 | c2 | D02 | d1 | E03 |
| 2-1-m1E03-2-0124 | 733.217 | OEt | a2 | B17 | b2 | C03 | c1 | D01 | d1 | E03 |
| 2-1-m1E03-2-0125 | 733.237 | OEt | a1 | B04 | b2 | C02 | c1 | D01 | d1 | E03 |
| 2-1-m1E03-2-0126 | 733.237 | OEt | a1 | B01 | b2 | C04 | c1 | D01 | d1 | E03 |
| 2-1-m1E03-2-0127 | 733.257 | OEt | a1 | B05 | b2 | C01 | c1 | D01 | d1 | E03 |
| 2-1-mlE03-2-0128 | 734.198 | OEt | a2 | B11 | b1 | C10 | c2 | D02 | d1 | E03 |
| 2-1-m1E03-2-0129 | 734.198 | OEt | a2 | B12 | b1 | C11 | c2 | D02 | d1 | E03 |
| 2-1-m1E03-2-0130 | 734.232 | OEt | a1 | B02 | b2 | C04 | c1 | D01 | d1 | E03 |
| 2-1-m 1 E03-2-0131 | 734.232 | OEt | a1 | B04 | b2 | C03 | c1 | D01 | d1 | E03 |
| 2-1-m 1 E03-2-0132 | 734.268 | OEt | a2 | B13 | b1 | C13 | c1 | D01 | d1 | E03 |
| 2-1-m1E03-2-0133 | 735.193 | OEt | a2 | B12 | b1 | C10 | c2 | D02 | d1 | E03 |
| 2-1-m 1 E03-2-0134 | 735.219 | OEt | a1 | B03 | b2 | C06 | c1 | D01 | d1 | E03 |
| 2-1-m1E03-2-0135 | 735.236 | OEt | a1 | B06 | b2 | C01 | c1 | D01 | d1 | E03 |
| 2-1-m1E03-2-0136 | 736.203 | OEt | a2 | B13 | b3 | C15 | c1 | D01 | d1 | E03 |
| 2-1-m1E03-2-0137 | 736.233 | OEt | a2 | B19 | b1 | C08 | c1 | D01 | d1 | E03 |
| 2-1-mlE03-2-0138 | 736.233 | OEt | a2 | B11 | b1 | C09 | c1 | D01 | d1 | E03 |
| 2-1-mlE03-2-0139 | 736.236 | OEt | a2 | B18 | b2 | C01 | c1 | D01 | d1 | E03 |
| 2-1-mlE03-2-0140 | 737.228 | OEt | a2 | B12 | b1 | C09 | c1 | D01 | d1 | E03 |
| 2-1-m1E03-2-0141 | 737.228 | OEt | a2 | B19 | b1 | C11 | c1 | D01 | d1 | E03 |
| 2-1-mlE03-2-0142 | 738.164 | OEt | a2 | B11 | b3 | C06 | c1 | D01 | d1 | E03 |
| 2-1-mlE03-2-0143 | 738.178 | OEt | a2 | B17 | b2 | C06 | c1 | D01 | d1 | E03 |
| 2-1-mlE03-2-0144 | 738.224 | OEt | a2 | B19 | b1 | C10 | c1 | D01 | d1 | E03 |
| 2-1-m1E03-2-0145 | 738.233 | OEt | a2 | B13 | b1 | C14 | c1 | D01 | d1 | E03 |
| 2-1-m1E03-2-0146 | 738.242 | OEt | a2 | B14 | b1 | C13 | c1 | D01 | d1 | E03 |
| 2-1-mlE03-2-0147 | 738.255 | OEt | a2 | B13 | b2 | C33 | c3 | D02 | d1 | E03 |
| 2-1-m1E03-2-0148 | 739.159 | OEt | a2 | B12 | b3 | C06 | c1 | D01 | d1 | E03 |
| 2-1-m1E03-2-0149 | 739.159 | OEt | a2 | B11 | b1 | C12 | c2 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0150 | 739.193 | OEt | a1 | B04 | b2 | C06 | c1 | D01 | d1 | E03 |
| 2-1-mlE03-2-0151 | 739.258 | OEt | a1 | B01 | b1 | C13 | c1 | D01 | d1 | E03 |
| 2-1-mlE03-2-0152 | 739.283 | OEt | a2 | B15 | b2 | C05 | c1 | D01 | d1 | E03 |
| 2-1-m1E03-2-0153 | 740.155 | OEt | a2 | B12 | b1 | C12 | c2 | D02 | d1 | E03 |
| 2-1-m 1 E03-2-0154 | 740.177 | OEt | a2 | B14 | b3 | C15 | c1 | D01 | d1 | E03 |
| 2-1-m 1 E03-2-0155 | 740.253 | OEt | a1 | B02 | b1 | C13 | c1 | D01 | d1 | E03 |
| 2-1-m 1 E03-2-0156 | 741.193 | OEt | a1 | B01 | b3 | C15 | c1 | D01 | d1 | E03 |
| 2-1-m 1 E03-2-0157 | 741.207 | OEt | a1 | B07 | b2 | C01 | c1 | D01 | d1 | E03 |
| 2-1-m1E03-2-0158 | 741.262 | OEt | a2 | B16 | b2 | C05 | c1 | D01 | d1 | E03 |
| 2-1-mlE03-2-0159 | 742.188 | OEt | a1 | B02 | b3 | C15 | c1 | D01 | d1 | E03 |
| 2-1-m 1 E03-2-0160 | 742.208 | OEt | a2 | B14 | b1 | C14 | c1 | D01 | d1 | E03 |
| 2-1-m 1 E03-2-0161 | 742.230 | OEt | a2 | B14 | b2 | C33 | c3 | D02 | d1 | E03 |
| 2-1-m 1 E03-2-0162 | 742.263 | OEt | a2 | B15 | b2 | C04 | c1 | D01 | d1 | E03 |
| 2-1-m 1 E03-2-0163 | 742.319 | OEt | a2 | B13 | b2 | C07 | c1 | D01 | d1 | E03 |
| 2-1-m 1 E03-2-0164 | 743.185 | OEt | a2 | B19 | b1 | C12 | c1 | D01 | d1 | E03 |
| 2-1-m 1 E03-2-0165 | 743.224 | OEt | a1 | B01 | b1 | C14 | c1 | D01 | d1 | E03 |
| 2-1-m 1 E03-2-0166 | 743.245 | OEt | a1 | B01 | b2 | C33 | c3 | D02 | d1 | E03 |
| 2-1-m 1 E03-2-0167 | 743.278 | OEt | a1 | B05 | b2 | C02 | c1 | D01 | d1 | E03 |
| 2-1-m 1 E03-2-0168 | 744.219 | OEt | a1 | B08 | b1 | C08 | c1 | D01 | d1 | E03 |
| 2-1-m 1 E03-2-0169 | 744.219 | OEt | a1 | B02 | b1 | C14 | c1 | D01 | d1 | E03 |
| 2-1-mlE03-2-0170 | 744.240 | OEt | a1 | B02 | b2 | C33 | c3 | D02 | d1 | E03 |
| 2-1-m 1 E03-2-0171 | 744.242 | OEt | a2 | B16 | b2 | C04 | c1 | D01 | d1 | E03 |
| 2-1-m 1 E03-2-0172 | 744.244 | OEt | a2 | B11 | b1 | C30 | c3 | D02 | d1 | E03 |
| 2-1-m 1 E03-2-0173 | 744.273 | OEt | a1 | B05 | b2 | C03 | c1 | D01 | d1 | E03 |
| 2-1-m 1 E03-2-0174 | 744.273 | OEt | a1 | B03 | b2 | C05 | c1 | D01 | d1 | E03 |
| 2-1-m 1 E03-2-0175 | 745.214 | OEt | a1 | B08 | b1 | C11 | c1 | D01 | d1 | E03 |
| 2-1-m 1 E03-2-0176 | 745.239 | OEt | a2 | B12 | b1 | C30 | c3 | D02 | d1 | E03 |
| 2-1-m 1 E03-2-0177 | 745.257 | OEt | a1 | B06 | b2 | C02 | c1 | D01 | d1 | E03 |
| 2-1-m 1 E03-2-0178 | 746.209 | OEt | a1 | B08 | b1 | C10 | c1 | D01 | d1 | E03 |
| 2-1-m 1 E03-2-0179 | 746.223 | OEt | a2 | B13 | b3 | C08 | c1 | D01 | d1 | E03 |
| 2-1-m 1 E03-2-0180 | 746.223 | OEt | a2 | B13 | b1 | C08 | c2 | D02 | d1 | E03 |
| 2-1-m 1 E03-2-0181 | 746.223 | OEt | a2 | B11 | b2 | C20 | c3 | D02 | d1 | E03 |
| 2-1-m 1 E03-2-0182 | 746.252 | OEt | a1 | B06 | b2 | C03 | c1 | D01 | d1 | E03 |
| 2-1-m 1 E03-2-0183 | 746.256 | OEt | a2 | B18 | b2 | C02 | c1 | D01 | d1 | E03 |
| 2-1-m1E03-2-0184 | 746.294 | OEt | a2 | B14 | b2 | C07 | c1 | D01 | d1 | E03 |
| 2-1-mlE03-2-0185 | 747.219 | OEt | a2 | B13 | b1 | C11 | c2 | D02 | d1 | E03 |
| 2-1-mlE03-2-0186 | 747.219 | OEt | a2 | B12 | b2 | C20 | c3 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-mlE03-2-0187 | 747.219 | OEt | a2 | B11 | b2 | C28 | c3 | D02 | d1 | E03 |
| 2-1-m1E03-2-0188 | 747.233 | OEt | a2 | B17 | b2 | C05 | c1 | D01 | d1 | E03 |
| 2-1-mlE03-2-0189 | 747.252 | OEt | a2 | B18 | b2 | C03 | c1 | D01 | d1 | E03 |
| 2-1-m1E03-2-0190 | 747.253 | OEt | a1 | B03 | b2 | C04 | c1 | D01 | d1 | E03 |
| 2-1-mlE03-2-0191 | 747.309 | OEt | a1 | B01 | b2 | C07 | c1 | D01 | d1 | E03 |
| 2-1-m1E03-2-0192 | 748.214 | OEt | a2 | B13 | b1 | C10 | c2 | D02 | d1 | E03 |
| 2-1-m1E03-2-0193 | 748.214 | OEt | a2 | B12 | b2 | C28 | c3 | D02 | d1 | E03 |
| 2-1-m 1 E03-2-0194 | 748.248 | OEt | a1 | B04 | b2 | C05 | c1 | D01 | d1 | E03 |
| 2-1-m 1 E03-2-0195 | 748.283 | OEt | a2 | B15 | b1 | C13 | c1 | D01 | d1 | E03 |
| 2-1-m 1 E03-2-0196 | 748.304 | OEt | a1 | B02 | b2 | C07 | c1 | D01 | d1 | E03 |
| 2-1-m 1 E03-2-0197 | 749.234 | OEt | a1 | B05 | b2 | C06 | c1 | D01 | d1 | E03 |
| 2-1-m 1 E03-2-0198 | 750.182 | OEt | a2 | B11 | b2 | C01 | c2 | D02 | d1 | E03 |
| 2-1-m 1 E03-2-0199 | 750.198 | OEt | a2 | B14 | b3 | C08 | c1 | D01 | d1 | E03 |
| 2-1-m1E03-2-0200 | 750.198 | OEt | a2 | B14 | b1 | C08 | c2 | D02 | d1 | E03 |
| 2-1-m 1 E03-2-0201 | 750.212 | OEt | a2 | B17 | b2 | C04 | c1 | D01 | d1 | E03 |
| 2-1-m1E03-2-0202 | 750.218 | OEt | a2 | B15 | b3 | C15 | c1 | D01 | d1 | E03 |
| 2-1-m 1 E03-2-0203 | 750.249 | OEt | a2 | B13 | b1 | C09 | c1 | D01 | d1 | E03 |
| 2-1-m1E03-2-0204 | 750.262 | OEt | a2 | B16 | b1 | C13 | c1 | D01 | d1 | E03 |
| 2-1-mlE03-2-0205 | 751.171 | OEt | a1 | B08 | b1 | C12 | c1 | D01 | d1 | E03 |
| 2-1-mlE03-2-0206 | 751.177 | OEt | a2 | B12 | b2 | C01 | c2 | D02 | d1 | E03 |
| 2-1-m1E03-2-0207 | 751.193 | OEt | a2 | B14 | b1 | C11 | c2 | D02 | d1 | E03 |
| 2-1-mlE03-2-0208 | 751.213 | OEt | a1 | B06 | b2 | C06 | c1 | D01 | d1 | E03 |
| 2-1-m 1 E03-2-0209 | 751.213 | OEt | a1 | B01 | b3 | C08 | c1 | D01 | d1 | E03 |
| 2-1-m 1 E03-2-0210 | 751.213 | OEt | a1 | B01 | b1 | C08 | c2 | D02 | d1 | E03 |
| 2-1-m1E03-2-0211 | 751.228 | OEt | a1 | B07 | b2 | C02 | c1 | D01 | d1 | E03 |
| 2-1-m 1 E03-2-0212 | 751.228 | OEt | a1 | B04 | b2 | C04 | c1 | D01 | d1 | E03 |
| 2-1-m 1 E03-2-0213 | 752.180 | OEt | a2 | B13 | b3 | C06 | c1 | D01 | d1 | E03 |
| 2-1-m 1 E03-2-0214 | 752.189 | OEt | a2 | B14 | b1 | C10 | c2 | D02 | d1 | E03 |
| 2-1-m1E03-2-0215 | 752.197 | OEt | a2 | B16 | b3 | C15 | c1 | D01 | d1 | E03 |
| 2-1-m 1 E03-2-0216 | 752.209 | OEt | a1 | B02 | b3 | C08 | c1 | D01 | d1 | E03 |
| 2-1-m 1 E03-2-0217 | 752.209 | OEt | a1 | B02 | b1 | C08 | c2 | D02 | d1 | E03 |
| 2-1-m 1 E03-2-0218 | 752.209 | OEt | a1 | B01 | b1 | C11 | c2 | D02 | d1 | E03 |
| 2-1-m 1 E03-2-0219 | 752.213 | OEt | a2 | B18 | b2 | C06 | c1 | D01 | d1 | E03 |
| 2-1-m 1 E03-2-0220 | 752.223 | OEt | a1 | B07 | b2 | C03 | c1 | D01 | d1 | E03 |
| 2-1-m1E03-2-0221 | 752.228 | OEt | a2 | B20 | b1 | C08 | c1 | D01 | d1 | E03 |
| 2-1-mlE03-2-0222 | 752.249 | OEt | a2 | B15 | b1 | C14 | c1 | D01 | d1 | E03 |
| 2-1-m1E03-2-0223 | 752.270 | OEt | a2 | B15 | b2 | C33 | c3 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-mlE03-2-0224 | 753.175 | OEt | a2 | B13 | b1 | C12 | c2 | D02 | d1 | E03 |
| 2-1-mlE03-2-0225 | 753.204 | OEt | a1 | B01 | b1 | C10 | c2 | D02 | d1 | E03 |
| 2-1-m1E03-2-0226 | 753.204 | OEt | a1 | B02 | b1 | C11 | c2 | D02 | d1 | E03 |
| 2-1-m 1 E03-2-0227 | 753.223 | OEt | a2 | B20 | b1 | C11 | c1 | D01 | d1 | E03 |
| 2-1-m 1 E03-2-0228 | 753.273 | OEt | a1 | B03 | b1 | C13 | c1 | D01 | d1 | E03 |
| 2-1-m 1 E03-2-0229 | 754.199 | OEt | a1 | B02 | b1 | C10 | c2 | D02 | d1 | E03 |
| 2-1-m1E03-2-0230 | 754.207 | OEt | a2 | B19 | b2 | C01 | c1 | D01 | d1 | E03 |
| 2-1-m1E03-2-0231 | 754.219 | OEt | a2 | B20 | b1 | C10 | c1 | D01 | d1 | E03 |
| 2-1-m1E03-2-0232 | 754.224 | OEt | a2 | B14 | b1 | C09 | c1 | D01 | d1 | E03 |
| 2-1-mlE03-2-0233 | 754.228 | OEt | a2 | B16 | b1 | C14 | c1 | D01 | d1 | E03 |
| 2-1-mlE03-2-0234 | 754.249 | OEt | a2 | B16 | b2 | C33 | c3 | D02 | d1 | E03 |
| 2-1-mlE03-2-0235 | 755.208 | OEt | a1 | B03 | b3 | C15 | c1 | D01 | d1 | E03 |
| 2-1-mlE03-2-0236 | 755.239 | OEt | a1 | B09 | b1 | C08 | c1 | D01 | d1 | E03 |
| 2-1-mlE03-2-0237 | 755.239 | OEt | a1 | B01 | b1 | C09 | c1 | D01 | d1 | E03 |
| 2-1-mlE03-2-0238 | 756.155 | OEt | a2 | B14 | b3 | C06 | c1 | D01 | d1 | E03 |
| 2-1-mlE03-2-0239 | 756.233 | OEt | a2 | B17 | b1 | C13 | c1 | D01 | d1 | E03 |
| 2-1-m1E03-2-0240 | 756.234 | OEt | a1 | B02 | b1 | C09 | c1 | D01 | d1 | E03 |
| 2-1-m1E03-2-0241 | 756.234 | OEt | a1 | B09 | b1 | C11 | c1 | D01 | d1 | E03 |
| 2-1-m1E03-2-0242 | 756.335 | OEt | a2 | B15 | b2 | C07 | c1 | D01 | d1 | E03 |
| 2-1-mlE03-2-0243 | 757.150 | OEt | a2 | B14 | b1 | C12 | c2 | D02 | d1 | E03 |
| 2-1-m1E03-2-0244 | 757.170 | OEt | a1 | B01 | b3 | C06 | c1 | D01 | d1 | E03 |
| 2-1-mlE03-2-0245 | 757.184 | OEt | a1 | B07 | b2 | C06 | c1 | D01 | d1 | E03 |
| 2-1-mlE03-2-0246 | 757.229 | OEt | a1 | B09 | b1 | C10 | c1 | D01 | d1 | E03 |
| 2-1-mlE03-2-0247 | 757.239 | OEt | a1 | B03 | b1 | C14 | c1 | D01 | d1 | E03 |
| 2-1-mlE03-2-0248 | 757.248 | OEt | a1 | B04 | b1 | C13 | c1 | D01 | d1 | E03 |
| 2-1-mlE03-2-0249 | 757.260 | OEt | a1 | B03 | b2 | C33 | c3 | D02 | d1 | E03 |
| 2-1-m1E03-2-0250 | 758.165 | OEt | a1 | B02 | b3 | C06 | c1 | D01 | d1 | E03 |
| 2-1-mlE03-2-0251 | 758.165 | OEt | a1 | B01 | b1 | C12 | c2 | D02 | d1 | E03 |
| 2-1-mlE03-2-0252 | 758.168 | OEt | a2 | B17 | b3 | C15 | c1 | D01 | d1 | E03 |
| 2-1-m1E03-2-0253 | 758.260 | OEt | a2 | B13 | b1 | C30 | c3 | D02 | d1 | E03 |
| 2-1-mlE03-2-0254 | 758.260 | OEt | a2 | B11 | b1 | C32 | c3 | D02 | d1 | E03 |
| 2-1-mlE03-2-0255 | 758.289 | OEt | a1 | B05 | b2 | C05 | c1 | D01 | d1 | E03 |
| 2-1-mlE03-2-0256 | 758.314 | OEt | a2 | B16 | b2 | C07 | c1 | D01 | d1 | E03 |
| 2-1-mlE03-2-0257 | 759.160 | OEt | a1 | B02 | b1 | C12 | c2 | D02 | d1 | E03 |
| 2-1-mlE03-2-0258 | 759.180 | OEt | a2 | B20 | b1 | C12 | c1 | D01 | d1 | E03 |
| 2-1-mlE03-2-0259 | 759.183 | OEt | a1 | B04 | b3 | C15 | c1 | D01 | d1 | E03 |
| 2-1-mlE03-2-0260 | 759.255 | OEt | a2 | B12 | b1 | C32 | c3 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-mlE03-2-0261 | 760.199 | OEt | a2 | B17 | b1 | C14 | c1 | D01 | d1 | E03 |
| 2-1-mlE03-2-0262 | 760.203 | OEt | a2 | B11 | b2 | C02 | c2 | D02 | d1 | E03 |
| 2-1-mlE03-2-0263 | 760.220 | OEt | a2 | B17 | b2 | C33 | c3 | D02 | d1 | E03 |
| 2-1-mlE03-2-0264 | 760.239 | OEt | a2 | B15 | b3 | C08 | c1 | D01 | d1 | E03 |
| 2-1-mlE03-2-0265 | 760.239 | OEt | a2 | B15 | b1 | C08 | c2 | D02 | d1 | E03 |
| 2-1-mlE03-2-0266 | 760.239 | OEt | a2 | B13 | b2 | C20 | c3 | D02 | d1 | E03 |
| 2-1-mlE03-2-0267 | 760.239 | OEt | a2 | B11 | b2 | C22 | c3 | D02 | d1 | E03 |
| 2-1-mlE03-2-0268 | 760.268 | OEt | a1 | B06 | b2 | C05 | c1 | D01 | d1 | E03 |
| 2-1-mlE03-2-0269 | 761.198 | OEt | a2 | B12 | b2 | C02 | c2 | D02 | d1 | E03 |
| 2-1-mlE03-2-0270 | 761.198 | OEt | a2 | B11 | b2 | C03 | c2 | D02 | d1 | E03 |
| 2-1-mlE03-2-0271 | 761.214 | OEt | a1 | B04 | b1 | C14 | c1 | D01 | d1 | E03 |
| 2-1-mlE03-2-0272 | 761.234 | OEt | a2 | B15 | b1 | C11 | c2 | D02 | d1 | E03 |
| 2-1-mlE03-2-0273 | 761.234 | OEt | a2 | B12 | b2 | C22 | c3 | D02 | d1 | E03 |
| 2-1-mlE03-2-0274 | 761.234 | OEt | a2 | B13 | b2 | C28 | c3 | D02 | d1 | E03 |
| 2-1-mlE03-2-0275 | 761.235 | OEt | a1 | B04 | b2 | C33 | c3 | D02 | d1 | E03 |
| 2-1-mlE03-2-0276 | 761.267 | OEt | a2 | B18 | b2 | C05 | c1 | D01 | d1 | E03 |
| 2-1-mlE03-2-0277 | 761.268 | OEt | a1 | B05 | b2 | C04 | c1 | D01 | d1 | E03 |
| 2-1-m1E03-2-0278 | 761.325 | OEt | a1 | B03 | b2 | C07 | c1 | D01 | d1 | E03 |
| 2-1-m1E03-2-0279 | 762.191 | OEt | a1 | B09 | b1 | C12 | c1 | D01 | d1 | E03 |
| 2-1-mlE03-2-0280 | 762.193 | OEt | a1 | B08 | b2 | C01 | c1 | D01 | d1 | E03 |
| 2-1-m1E03-2-0281 | 762.193 | OEt | a2 | B12 | b2 | C03 | c2 | D02 | d1 | E03 |
| 2-1-mlE03-2-0282 | 762.218 | OEt | a2 | B16 | b3 | C08 | c1 | D01 | d1 | E03 |
| 2-1-mlE03-2-0283 | 762.218 | OEt | a2 | B16 | b1 | C08 | c2 | D02 | d1 | E03 |
| 2-1-mlE03-2-0284 | 762.229 | OEt | a2 | B15 | b1 | C10 | c2 | D02 | d1 | E03 |
| 2-1-mlE03-2-0285 | 762.235 | OEt | a2 | B14 | b1 | C30 | c3 | D02 | d1 | E03 |
| 2-1-mlE03-2-0286 | 763.213 | OEt | a2 | B16 | b1 | C11 | c2 | D02 | d1 | E03 |
| 2-1-m1E03-2-0287 | 763.248 | OEt | a1 | B06 | b2 | C04 | c1 | D01 | d1 | E03 |
| 2-1-mlE03-2-0288 | 763.250 | OEt | a1 | B01 | b1 | C30 | c3 | D02 | d1 | E03 |
| 2-1-m1E03-2-0289 | 764.197 | OEt | a2 | B13 | b2 | C01 | c2 | D02 | d1 | E03 |
| 2-1-mlE03-2-0290 | 764.209 | OEt | a2 | B16 | b1 | C10 | c2 | D02 | d1 | E03 |
| 2-1-m1E03-2-0291 | 764.214 | OEt | a2 | B14 | b2 | C20 | c3 | D02 | d1 | E03 |
| 2-1-mlE03-2-0292 | 764.214 | OEt | a2 | B11 | b2 | C24 | c3 | D02 | d1 | E03 |
| 2-1-mlE03-2-0293 | 764.228 | OEt | a2 | B19 | b2 | C02 | c1 | D01 | d1 | E03 |
| 2-1-m1E03-2-0294 | 764.245 | OEt | a1 | B02 | b1 | C30 | c3 | D02 | d1 | E03 |
| 2-1-m1E03-2-0295 | 764.247 | OEt | a2 | B18 | b2 | C04 | c1 | D01 | d1 | E03 |
| 2-1-m1E03-2-0296 | 764.264 | OEt | a2 | B15 | b1 | C09 | c1 | D01 | d1 | E03 |
| 2-1-mlE03-2-0297 | 764.284 | OEt | a2 | B17 | b2 | C07 | c1 | D01 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0298 | 765.209 | OEt | a2 | B12 | b2 | C24 | c3 | D02 | d1 | E03 |
| 2-1-m1E03-2-0299 | 765.209 | OEt | a2 | B14 | b2 | C28 | c3 | D02 | d1 | E03 |
| 2-1-m1E03-2-0300 | 765.223 | OEt | a2 | B19 | b2 | C03 | c1 | D01 | d1 | E03 |
| 2-1-m1E03-2-0301 | 765.229 | OEt | a1 | B03 | b3 | C08 | c1 | D01 | d1 | E03 |
| 2-1-mlE03-2-0302 | 765.229 | OEt | a1 | B03 | b1 | C08 | c2 | D02 | d1 | E03 |
| 2-1-m1E03-2-0303 | 765.229 | OEt | a1 | B01 | b2 | C20 | c3 | D02 | d1 | E03 |
| 2-1-mlE03-2-0304 | 765.300 | OEt | a1 | B04 | b2 | C07 | c1 | D01 | d1 | E03 |
| 2-1-m1E03-2-0305 | 766.159 | OEt | a2 | B11 | b2 | C06 | c2 | D02 | d1 | E03 |
| 2-1-m1E03-2-0306 | 766.195 | OEt | a2 | B15 | b3 | C06 | c1 | D01 | d1 | E03 |
| 2-1-mlE03-2-0307 | 766.224 | OEt | a1 | B03 | b1 | C11 | c2 | D02 | d1 | E03 |
| 2-1-m1E03-2-0308 | 766.224 | OEt | a1 | B02 | b2 | C20 | c3 | D02 | d1 | E03 |
| 2-1-mlE03-2-0309 | 766.224 | OEt | a1 | B01 | b2 | C28 | c3 | D02 | d1 | E03 |
| 2-1-m1E03-2-0310 | 766.239 | OEt | a1 | B07 | b2 | C05 | c1 | D01 | d1 | E03 |
| 2-1-mlE03-2-0311 | 766.244 | OEt | a2 | B16 | b1 | C09 | c1 | D01 | d1 | E03 |
| 2-1-m1E03-2-0312 | 767.154 | OEt | a2 | B12 | b2 | C06 | c2 | D02 | d1 | E03 |
| 2-1-m1E03-2-0313 | 767.191 | OEt | a2 | B15 | b1 | C12 | c2 | D02 | d1 | E03 |
| 2-1-m1E03-2-0314 | 767.220 | OEt | a1 | B03 | b1 | C10 | c2 | D02 | d1 | E03 |
| 2-1-m1E03-2-0315 | 767.220 | OEt | a1 | B02 | b2 | C28 | c3 | D02 | d1 | E03 |
| 2-1-m1E03-2-0316 | 767.289 | OEt | a1 | B05 | b1 | C13 | c1 | D01 | d1 | E03 |
| 2-1-m1E03-2-0317 | 768.172 | OEt | a2 | B14 | b2 | C01 | c2 | D02 | d1 | E03 |
| 2-1-m1E03-2-0318 | 768.175 | OEt | a2 | B16 | b3 | C06 | c1 | D01 | d1 | E03 |
| 2-1-m1E03-2-0319 | 768.189 | OEt | a2 | B17 | b3 | C08 | c1 | D01 | d1 | E03 |
| 2-1-mlE03-2-0320 | 768.189 | OEt | a2 | B17 | b1 | C08 | c2 | D02 | d1 | E03 |
| 2-1-mlE03-2-0321 | 769.170 | OEt | a2 | B16 | b1 | C12 | c2 | D02 | d1 | E03 |
| 2-1-mlE03-2-0322 | 769.184 | OEt | a2 | B17 | b1 | C11 | c2 | D02 | d1 | E03 |
| 2-1-m1E03-2-0323 | 769.188 | OEt | a1 | B01 | b2 | C01 | c2 | D02 | d1 | E03 |
| 2-1-mlE03-2-0324 | 769.204 | OEt | a1 | B04 | b3 | C08 | c1 | D01 | d1 | E03 |
| 2-1-mlE03-2-0325 | 769.204 | OEt | a1 | B04 | b1 | C08 | c2 | D02 | d1 | E03 |
| 2-1-mlE03-2-0326 | 769.218 | OEt | a1 | B07 | b2 | C04 | c1 | D01 | d1 | E03 |
| 2-1-m1E03-2-0327 | 769.224 | OEt | a1 | B05 | b3 | C15 | c1 | D01 | d1 | E03 |
| 2-1-m1E03-2-0328 | 769.255 | OEt | a1 | B03 | b1 | C09 | c1 | D01 | d1 | E03 |
| 2-1-mlE03-2-0329 | 769.268 | OEt | a1 | B06 | b1 | C13 | c1 | D01 | d1 | E03 |
| 2-1-m1E03-2-0330 | 770.179 | OEt | a2 | B17 | b1 | C10 | c2 | D02 | d1 | E03 |
| 2-1-mlE03-2-0331 | 770.183 | OEt | a1 | B02 | b2 | C01 | c2 | D02 | d1 | E03 |
| 2-1-m1E03-2-0332 | 770.184 | OEt | a2 | B19 | b2 | C06 | c1 | D01 | d1 | E03 |
| 2-1-mlE03-2-0333 | 770.199 | OEt | a1 | B04 | b1 | C11 | c2 | D02 | d1 | E03 |
| 2-1-m1E03-2-0334 | 770.202 | OEt | a2 | B20 | b2 | C01 | c1 | D01 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0335 | 770.268 | OEt | a2 | B18 | b1 | C13 | c1 | D01 | d1 | E03 |
| 2-1-m1E03-2-0336 | 771.186 | OEt | a1 | B03 | b3 | C06 | c1 | D01 | d1 | E03 |
| 2-1-m1E03-2-0337 | 771.195 | OEt | a1 | B04 | b1 | C10 | c2 | D02 | d1 | E03 |
| 2-1-m1E03-2-0338 | 771.203 | OEt | a1 | B06 | b3 | C15 | c1 | D01 | d1 | E03 |
| 2-1-mlE03-2-0339 | 771.234 | OEt | a1 | B10 | b1 | C08 | c1 | D01 | d1 | E03 |
| 2-1-m1E03-2-0340 | 771.255 | OEt | a1 | B05 | b1 | C14 | c1 | D01 | d1 | E03 |
| 2-1-m1E03-2-0341 | 771.276 | OEt | a1 | B05 | b2 | C33 | c3 | D02 | d1 | E03 |
| 2-1-mlE03-2-0342 | 772.181 | OEt | a1 | B03 | b1 | C12 | c2 | D02 | d1 | E03 |
| 2-1-m1E03-2-0343 | 772.203 | OEt | a2 | B18 | b3 | C15 | c1 | D01 | d1 | E03 |
| 2-1-mlE03-2-0344 | 772.214 | OEt | a1 | B08 | b2 | C02 | c1 | D01 | d1 | E03 |
| 2-1-m1E03-2-0345 | 772.214 | OEt | a2 | B17 | b1 | C09 | c1 | D01 | d1 | E03 |
| 2-1-m1E03-2-0346 | 772.229 | OEt | a1 | B10 | b1 | C11 | c1 | D01 | d1 | E03 |
| 2-1-m1E03-2-0347 | 772.275 | OEt | a2 | B15 | b1 | C30 | c3 | D02 | d1 | E03 |
| 2-1-mlE03-2-0348 | 772.275 | OEt | a2 | B11 | b1 | C29 | c3 | D02 | d1 | E03 |
| 2-1-m1E03-2-0349 | 772.275 | OEt | a2 | B13 | b1 | C32 | c3 | D02 | d1 | E03 |
| 2-1-mlE03-2-0350 | 773.209 | OEt | a1 | B08 | b2 | C03 | c1 | D01 | d1 | E03 |
| 2-1-m1E03-2-0351 | 773.213 | OEt | a1 | B09 | b2 | C01 | c1 | D01 | d1 | E03 |
| 2-1-mlE03-2-0352 | 773.224 | OEt | a1 | B10 | b1 | C10 | c1 | D01 | d1 | E03 |
| 2-1-mlE03-2-0353 | 773.230 | OEt | a1 | B04 | b1 | C09 | c1 | D01 | d1 | E03 |
| 2-1-m1E03-2-0354 | 773.234 | OEt | a1 | B06 | b1 | C14 | c1 | D01 | d1 | E03 |
| 2-1-mlE03-2-0355 | 773.255 | OEt | a1 | B06 | b2 | C33 | c3 | D02 | d1 | E03 |
| 2-1-m1E03-2-0356 | 773.271 | OEt | a2 | B12 | b1 | C29 | c3 | D02 | d1 | E03 |
| 2-1-mlE03-2-0357 | 774.145 | OEt | a2 | B17 | b3 | C06 | c1 | D01 | d1 | E03 |
| 2-1-m1E03-2-0358 | 774.218 | OEt | a2 | B13 | b2 | C02 | c2 | D02 | d1 | E03 |
| 2-1-mlE03-2-0359 | 774.233 | OEt | a2 | B18 | b1 | C14 | c1 | D01 | d1 | E03 |
| 2-1-mlE03-2-0360 | 774.255 | OEt | a2 | B15 | b2 | C20 | c3 | D02 | d1 | E03 |
| 2-1-m1E03-2-0361 | 774.255 | OEt | a2 | B13 | b2 | C22 | c3 | D02 | d1 | E03 |
| 2-1-mlE03-2-0362 | 774.255 | OEt | a2 | B16 | b1 | C30 | c3 | D02 | d1 | E03 |
| 2-1-m1E03-2-0363 | 774.255 | OEt | a2 | B18 | b2 | C33 | c3 | D02 | d1 | E03 |
| 2-1-mlE03-2-0364 | 775.140 | OEt | a2 | B17 | b1 | C12 | c2 | D02 | d1 | E03 |
| 2-1-m1E03-2-0365 | 775.160 | OEt | a1 | B04 | b3 | C06 | c1 | D01 | d1 | E03 |
| 2-1-m1E03-2-0366 | 775.213 | OEt | a2 | B13 | b2 | C03 | c2 | D02 | d1 | E03 |
| 2-1-mlE03-2-0367 | 775.213 | OEt | a2 | B11 | b2 | C05 | c2 | D02 | d1 | E03 |
| 2-1-m1E03-2-0368 | 775.239 | OEt | a1 | B07 | b1 | C13 | c1 | D01 | d1 | E03 |
| 2-1-mlE03-2-0369 | 775.250 | OEt | a2 | B15 | b2 | C28 | c3 | D02 | d1 | E03 |
| 2-1-m1E03-2-0370 | 775.340 | OEt | a1 | B05 | b2 | C07 | c1 | D01 | d1 | E03 |
| 2-1-m1E03-2-0371 | 776.156 | OEt | a1 | B04 | b1 | C12 | c2 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0372 | 776.209 | OEt | a2 | B12 | b2 | C05 | c2 | D02 | d1 | E03 |
| 2-1-m1E03-2-0373 | 776.234 | OEt | a2 | B16 | b2 | C20 | c3 | D02 | d1 | E03 |
| 2-1-m1E03-2-0374 | 776.234 | OEt | a2 | B11 | b2 | C26 | c3 | D02 | d1 | E03 |
| 2-1-m1E03-2-0375 | 776.250 | OEt | a2 | B14 | b1 | C32 | c3 | D02 | d1 | E03 |
| 2-1-m1E03-2-0376 | 777.174 | OEt | a1 | B07 | b3 | C15 | c1 | D01 | d1 | E03 |
| 2-1-m1E03-2-0377 | 777.229 | OEt | a2 | B12 | b2 | C26 | c3 | D02 | d1 | E03 |
| 2-1-m1E03-2-0378 | 777.229 | OEt | a2 | B16 | b2 | C28 | c3 | D02 | d1 | E03 |
| 2-1-m1E03-2-0379 | 777.265 | OEt | a1 | B03 | b1 | C30 | c3 | D02 | d1 | E03 |
| 2-1-m1E03-2-0380 | 777.265 | OEt | a1 | B01 | b1 | C32 | c3 | D02 | d1 | E03 |
| 2-1-m1E03-2-0381 | 777.320 | OEt | a1 | B06 | b2 | C07 | c1 | D01 | d1 | E03 |
| 2-1-m1E03-2-0382 | 778.170 | OEt | a1 | B08 | b2 | C06 | c1 | D01 | d1 | E03 |
| 2-1-m1E03-2-0383 | 778.186 | OEt | a1 | B10 | b1 | C12 | c1 | D01 | d1 | E03 |
| 2-1-m1E03-2-0384 | 778.193 | OEt | a2 | B14 | b2 | C02 | c2 | D02 | d1 | E03 |
| 2-1-m1E03-2-0385 | 778.193 | OEt | a2 | B11 | b2 | C04 | c2 | D02 | d1 | E03 |
| 2-1-m1E03-2-0386 | 778.213 | OEt | a2 | B15 | b2 | C01 | c2 | D02 | d1 | E03 |
| 2-1-m1E03-2-0387 | 778.230 | OEt | a2 | B14 | b2 | C22 | c3 | D02 | d1 | E03 |
| 2-1-m1E03-2-0388 | 778.230 | OEt | a2 | B13 | b2 | C24 | c3 | D02 | d1 | E03 |
| 2-1-m1E03-2-0389 | 778.261 | OEt | a1 | B02 | b1 | C32 | c3 | D02 | d1 | E03 |
| 2-1-mlE03-2-0390 | 778.319 | OEt | a2 | B18 | b2 | C07 | c1 | D01 | d1 | E03 |
| 2-1-m1E03-2-0391 | 779.188 | OEt | a2 | B12 | b2 | C04 | c2 | D02 | d1 | E03 |
| 2-1-m1E03-2-0392 | 779.188 | OEt | a2 | B14 | b2 | C03 | c2 | D02 | d1 | E03 |
| 2-1-mlE03-2-0393 | 779.205 | OEt | a1 | B07 | b1 | C14 | c1 | D01 | d1 | E03 |
| 2-1-mlE03-2-0394 | 779.208 | OEt | a1 | B01 | b2 | C02 | c2 | D02 | d1 | E03 |
| 2-1-mlE03-2-0395 | 779.226 | OEt | a1 | B07 | b2 | C33 | c3 | D02 | d1 | E03 |
| 2-1-m1E03-2-0396 | 779.239 | OEt | a2 | B19 | b2 | C05 | c1 | D01 | d1 | E03 |
| 2-1-mlE03-2-0397 | 779.245 | OEt | a1 | B05 | b3 | C08 | c1 | D01 | d1 | E03 |
| 2-1-m1E03-2-0398 | 779.245 | OEt | a1 | B05 | b1 | C08 | c2 | D02 | d1 | E03 |
| 2-1-m1E03-2-0399 | 779.245 | OEt | a1 | B03 | b2 | C20 | c3 | D02 | d1 | E03 |
| 2-1-m1E03-2-0400 | 779.245 | OEt | a1 | B01 | b2 | C22 | c3 | D02 | d1 | E03 |
| 2-1-m1E03-2-0401 | 780.175 | OEt | a2 | B13 | b2 | C06 | c2 | D02 | d1 | E03 |
| 2-1-m1E03-2-0402 | 780.192 | OEt | a2 | B16 | b2 | C01 | c2 | D02 | d1 | E03 |
| 2-1-m1E03-2-0403 | 780.204 | OEt | a1 | B02 | b2 | C02 | c2 | D02 | d1 | E03 |
| 2-1-m1E03-2-0404 | 780.204 | OEt | a1 | B01 | b2 | C03 | c2 | D02 | d1 | E03 |
| 2-1-m1E03-2-0405 | 780.223 | OEt | a2 | B20 | b2 | C02 | c1 | D01 | d1 | E03 |
| 2-1-mlE03-2-0406 | 780.225 | OEt | a2 | B17 | b1 | C30 | c3 | D02 | d1 | E03 |
| 2-1-mlE03-2-0407 | 780.240 | OEt | a1 | B05 | b1 | C11 | c2 | D02 | d1 | E03 |
| 2-1-m1E03-2-0408 | 780.240 | OEt | a1 | B02 | b2 | C22 | c3 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0409 | 780.240 | OEt | a1 | B03 | b2 | C28 | c3 | D02 | d1 | E03 |
| 2-1-m1E03-2-0410 | 781.199 | OEt | a1 | B02 | b2 | C03 | c2 | D02 | d1 | E03 |
| 2-1-mlE03-2-0411 | 781.218 | OEt | a2 | B20 | b2 | C03 | c1 | D01 | d1 | E03 |
| 2-1-m1E03-2-0412 | 781.224 | OEt | a1 | B06 | b3 | C08 | c1 | D01 | d1 | E03 |
| 2-1-m1E03-2-0413 | 781.224 | OEt | a1 | B06 | b1 | C08 | c2 | D02 | d1 | E03 |
| 2-1-m1E03-2-0414 | 781.235 | OEt | a1 | B05 | b1 | C10 | c2 | D02 | d1 | E03 |
| 2-1-m1E03-2-0415 | 781.240 | OEt | a1 | B04 | b1 | C30 | c3 | D02 | d1 | E03 |
| 2-1-mlE03-2-0416 | 782.204 | OEt | a2 | B17 | b2 | C20 | c3 | D02 | d1 | E03 |
| 2-1-m1E03-2-0417 | 782.204 | OEt | a2 | B14 | b2 | C24 | c3 | D02 | d1 | E03 |
| 2-1-m1E03-2-0418 | 782.219 | OEt | a2 | B19 | b2 | C04 | c1 | D01 | d1 | E03 |
| 2-1-m1E03-2-0419 | 782.219 | OEt | a1 | B06 | b1 | C11 | c2 | D02 | d1 | E03 |
| 2-1-m1E03-2-0420 | 782.223 | OEt | a2 | B18 | b3 | C08 | c1 | D01 | d1 | E03 |
| 2-1-m1E03-2-0421 | 782.223 | OEt | a2 | B18 | b1 | C08 | c2 | D02 | d1 | E03 |
| 2-1-m1E03-2-0422 | 783.200 | OEt | a2 | B17 | b2 | C28 | c3 | D02 | d1 | E03 |
| 2-1-m1E03-2-0423 | 783.203 | OEt | a1 | B03 | b2 | C01 | c2 | D02 | d1 | E03 |
| 2-1-m1E03-2-0424 | 783.215 | OEt | a1 | B06 | b1 | C10 | c2 | D02 | d1 | E03 |
| 2-1-m1E03-2-0425 | 783.219 | OEt | a2 | B18 | b1 | C11 | c2 | D02 | d1 | E03 |
| 2-1-m1E03-2-0426 | 783.220 | OEt | a1 | B04 | b2 | C20 | c3 | D02 | d1 | E03 |
| 2-1-m1E03-2-0427 | 783.220 | OEt | a1 | B01 | b2 | C24 | c3 | D02 | d1 | E03 |
| 2-1-m1E03-2-0428 | 783.234 | OEt | a1 | B09 | b2 | C02 | c1 | D01 | d1 | E03 |
| 2-1-m1E03-2-0429 | 783.270 | OEt | a1 | B05 | b1 | C09 | c1 | D01 | d1 | E03 |
| 2-1-m1E03-2-0430 | 783.290 | OEt | a1 | B07 | b2 | C07 | c1 | D01 | d1 | E03 |
| 2-1-m1E03-2-0431 | 784.150 | OEt | a2 | B14 | b2 | C06 | c2 | D02 | d1 | E03 |
| 2-1-m1E03-2-0432 | 784.214 | OEt | a2 | B18 | b1 | C10 | c2 | D02 | d1 | E03 |
| 2-1-m1E03-2-0433 | 784.214 | OEt | a2 | B11 | b1 | C13 | c2 | D02 | d1 | E03 |
| 2-1-m1E03-2-0434 | 784.215 | OEt | a1 | B02 | b2 | C24 | c3 | D02 | d1 | E03 |
| 2-1-m1E03-2-0435 | 784.215 | OEt | a1 | B04 | b2 | C28 | c3 | D02 | d1 | E03 |
| 2-1-m1E03-2-0436 | 784.229 | OEt | a1 | B09 | b2 | C03 | c1 | D01 | d1 | E03 |
| 2-1-mlE03-2-0437 | 785.165 | OEt | a1 | B01 | b2 | C06 | c2 | D02 | d1 | E03 |
| 2-1-m1E03-2-0438 | 785.201 | OEt | a1 | B05 | b3 | C06 | c1 | D01 | d1 | E03 |
| 2-1-m1E03-2-0439 | 785.209 | OEt | a2 | B12 | b1 | C13 | c2 | D02 | d1 | E03 |
| 2-1-m1E03-2-0440 | 785.249 | OEt | a1 | B06 | b1 | C09 | c1 | D01 | d1 | E03 |
| 2-1-m1E03-2-0441 | 786.149 | OEt | a2 | B11 | b3 | C15 | c2 | D02 | d1 | E03 |
| 2-1-mlE03-2-0442 | 786.160 | OEt | a1 | B02 | b2 | C06 | c2 | D02 | d1 | E03 |
| 2-1-m1E03-2-0443 | 786.163 | OEt | a2 | B17 | b2 | C01 | c2 | D02 | d1 | E03 |
| 2-1-m1E03-2-0444 | 786.179 | OEt | a2 | B20 | b2 | C06 | c1 | D01 | d1 | E03 |
| 2-1-m1E03-2-0445 | 786.196 | OEt | a1 | B05 | b1 | C12 | c2 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-mlE03-2-0446 | 786.218 | OEt | a2 | B11 | b3 | C16 | c1 | D01 | d1 | E03 |
| 2-1-mlE03-2-0447 | 786.249 | OEt | a2 | B18 | b1 | C09 | c1 | D01 | d1 | E03 |
| 2-1-m1E03-2-0448 | 786.291 | OEt | a2 | B13 | b1 | C29 | c3 | D02 | d1 | E03 |
| 2-1-mlE03-2-0449 | 786.291 | OEt | a2 | B15 | b1 | C32 | c3 | D02 | d1 | E03 |
| 2-1-m1E03-2-0450 | 787.144 | OEt | a2 | B12 | b3 | C15 | c2 | D02 | d1 | E03 |
| 2-1-m1E03-2-0451 | 787.178 | OEt | a1 | B04 | b2 | C01 | c2 | D02 | d1 | E03 |
| 2-1-m1E03-2-0452 | 787.180 | OEt | a1 | B06 | b3 | C06 | c1 | D01 | d1 | E03 |
| 2-1-m1E03-2-0453 | 787.195 | OEt | a1 | B07 | b3 | C08 | c1 | D01 | d1 | E03 |
| 2-1-m1E03-2-0454 | 787.195 | OEt | a1 | B07 | b1 | C08 | c2 | D02 | d1 | E03 |
| 2-1-m1E03-2-0455 | 787.213 | OEt | a2 | B12 | b3 | C16 | c1 | D01 | d1 | E03 |
| 2-1-m1E03-2-0456 | 787.225 | OEt | a1 | B08 | b2 | C05 | c1 | D01 | d1 | E03 |
| 2-1-m1E03-2-0457 | 788.176 | OEt | a1 | B06 | b1 | C12 | c2 | D02 | d1 | E03 |
| 2-1-mlE03-2-0458 | 788.180 | OEt | a2 | B18 | b3 | C06 | c1 | D01 | d1 | E03 |
| 2-1-m1E03-2-0459 | 788.180 | OEt | a2 | B11 | b3 | C17 | c1 | D01 | d1 | E03 |
| 2-1-m1E03-2-0460 | 788.180 | OEt | a2 | B11 | b1 | C14 | c2 | D02 | d1 | E03 |
| 2-1-mlE03-2-0461 | 788.190 | OEt | a1 | B07 | b1 | C11 | c2 | D02 | d1 | E03 |
| 2-1-m1E03-2-0462 | 788.234 | OEt | a2 | B15 | b2 | C02 | c2 | D02 | d1 | E03 |
| 2-1-mlE03-2-0463 | 788.239 | OEt | a2 | B19 | b1 | C13 | c1 | D01 | d1 | E03 |
| 2-1-m1E03-2-0464 | 788.270 | OEt | a2 | B15 | b2 | C22 | c3 | D02 | d1 | E03 |
| 2-1-mlE03-2-0465 | 788.270 | OEt | a2 | B16 | b1 | C32 | c3 | D02 | d1 | E03 |
| 2-1-m1E03-2-0466 | 789.175 | OEt | a2 | B12 | b3 | C17 | c1 | D01 | d1 | E03 |
| 2-1-m1E03-2-0467 | 789.175 | OEt | a2 | B18 | b1 | C12 | c2 | D02 | d1 | E03 |
| 2-1-m1E03-2-0468 | 789.175 | OEt | a2 | B12 | b1 | C14 | c2 | D02 | d1 | E03 |
| 2-1-m1E03-2-0469 | 789.185 | OEt | a1 | B07 | b1 | C10 | c2 | D02 | d1 | E03 |
| 2-1-m1E03-2-0470 | 789.190 | OEt | a1 | B09 | b2 | C06 | c1 | D01 | d1 | E03 |
| 2-1-m1E03-2-0471 | 789.208 | OEt | a1 | B10 | b2 | C01 | c1 | D01 | d1 | E03 |
| 2-1-m1E03-2-0472 | 789.229 | OEt | a2 | B15 | b2 | C03 | c2 | D02 | d1 | E03 |
| 2-1-m1E03-2-0473 | 789.229 | OEt | a2 | B13 | b2 | C05 | c2 | D02 | d1 | E03 |
| 2-1-m1E03-2-0474 | 790.174 | OEt | a2 | B19 | b3 | C15 | c1 | D01 | d1 | E03 |
| 2-1-m1E03-2-0475 | 790.204 | OEt | a1 | B08 | b2 | C04 | c1 | D01 | d1 | E03 |
| 2-1-m1E03-2-0476 | 790.213 | OEt | a2 | B16 | b2 | C02 | c2 | D02 | d1 | E03 |
| 2-1-m1E03-2-0477 | 790.249 | OEt | a2 | B16 | b2 | C22 | c3 | D02 | d1 | E03 |
| 2-1-m1E03-2-0478 | 790.249 | OEt | a2 | B13 | b2 | C26 | c3 | D02 | d1 | E03 |
| 2-1-m1E03-2-0479 | 790.266 | OEt | a2 | B14 | b1 | C29 | c3 | D02 | d1 | E03 |
| 2-1-m1E03-2-0480 | 791.208 | OEt | a2 | B16 | b2 | C03 | c2 | D02 | d1 | E03 |
| 2-1-m1E03-2-0481 | 791.220 | OEt | a1 | B07 | b1 | C09 | c1 | D01 | d1 | E03 |
| 2-1-m1E03-2-0482 | 791.281 | OEt | a1 | B05 | b1 | C30 | c3 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0483 | 791.281 | OEt | a1 | B01 | b1 | C29 | c3 | D02 | d1 | E03 |
| 2-1-mlE03-2-0484 | 791.281 | OEt | a1 | B03 | b1 | C32 | c3 | D02 | d1 | E03 |
| 2-1-m1E03-2-0485 | 792.205 | OEt | a2 | B19 | b1 | C14 | c1 | D01 | d1 | E03 |
| 2-1-mlE03-2-0486 | 792.209 | OEt | a2 | B13 | b2 | C04 | c2 | D02 | d1 | E03 |
| 2-1-m1E03-2-0487 | 792.226 | OEt | a2 | B19 | b2 | C33 | c3 | D02 | d1 | E03 |
| 2-1-m1E03-2-0488 | 792.245 | OEt | a2 | B15 | b2 | C24 | c3 | D02 | d1 | E03 |
| 2-1-m1E03-2-0489 | 792.265 | OEt | a2 | B11 | b2 | C07 | c2 | D02 | d1 | E03 |
| 2-1-m1E03-2-0490 | 792.276 | OEt | a1 | B02 | b1 | C29 | c3 | D02 | d1 | E03 |
| 2-1-m1E03-2-0491 | 793.151 | OEt | a1 | B07 | b3 | C06 | c1 | D01 | d1 | E03 |
| 2-1-m1E03-2-0492 | 793.204 | OEt | a2 | B14 | b2 | C05 | c2 | D02 | d1 | E03 |
| 2-1-m1E03-2-0493 | 793.224 | OEt | a1 | B03 | b2 | C02 | c2 | D02 | d1 | E03 |
| 2-1-m1E03-2-0494 | 793.260 | OEt | a2 | B12 | b2 | C07 | c2 | D02 | d1 | E03 |
| 2-1-m1E03-2-0495 | 793.260 | OEt | a1 | B05 | b2 | C20 | c3 | D02 | d1 | E03 |
| 2-1-m1E03-2-0496 | 793.260 | OEt | a1 | B03 | b2 | C22 | c3 | D02 | d1 | E03 |
| 2-1-m1E03-2-0497 | 793.260 | OEt | a1 | B06 | b1 | C30 | c3 | D02 | d1 | E03 |
| 2-1-m1E03-2-0498 | 794.146 | OEt | a1 | B07 | b1 | C12 | c2 | D02 | d1 | E03 |
| 2-1-m1E03-2-0499 | 794.190 | OEt | a2 | B15 | b2 | C06 | c2 | D02 | d1 | E03 |
| 2-1-m1E03-2-0500 | 794.219 | OEt | a1 | B03 | b2 | C03 | c2 | D02 | d1 | E03 |
| 2-1-m1E03-2-0501 | 794.219 | OEt | a1 | B01 | b2 | C05 | c2 | D02 | d1 | E03 |
| 2-1-m1E03-2-0502 | 794.224 | OEt | a2 | B16 | b2 | C24 | c3 | D02 | d1 | E03 |
| 2-1-m1E03-2-0503 | 794.224 | OEt | a2 | B14 | b2 | C26 | c3 | D02 | d1 | E03 |
| 2-1-m1E03-2-0504 | 794.241 | OEt | a2 | B17 | b1 | C32 | c3 | D02 | d1 | E03 |
| 2-1-m1E03-2-0505 | 794.256 | OEt | a1 | B05 | b2 | C28 | c3 | D02 | d1 | E03 |
| 2-1-m1E03-2-0506 | 794.260 | OEt | a2 | B18 | b1 | C30 | c3 | D02 | d1 | E03 |
| 2-1-m1E03-2-0507 | 795.215 | OEt | a1 | B02 | b2 | C05 | c2 | D02 | d1 | E03 |
| 2-1-m1E03-2-0508 | 795.234 | OEt | a2 | B20 | b2 | C05 | c1 | D01 | d1 | E03 |
| 2-1-m1E03-2-0509 | 795.240 | OEt | a1 | B06 | b2 | C20 | c3 | D02 | d1 | E03 |
| 2-1-m1E03-2-0510 | 795.240 | OEt | a1 | B01 | b2 | C26 | c3 | D02 | d1 | E03 |
| 2-1-m1E03-2-0511 | 795.256 | OEt | a1 | B04 | b1 | C32 | c3 | D02 | d1 | E03 |
| 2-1-mlE03-2-0512 | 796.170 | OEt | a2 | B16 | b2 | C06 | c2 | D02 | d1 | E03 |
| 2-1-m1E03-2-0513 | 796.170 | OEt | a2 | B11 | b3 | C08 | c2 | D02 | d1 | E03 |
| 2-1-m1E03-2-0514 | 796.184 | OEt | a2 | B17 | b2 | C02 | c2 | D02 | d1 | E03 |
| 2-1-m1E03-2-0515 | 796.184 | OEt | a2 | B14 | b2 | C04 | c2 | D02 | d1 | E03 |
| 2-1-m1E03-2-0516 | 796.220 | OEt | a2 | B17 | b2 | C22 | c3 | D02 | d1 | E03 |
| 2-1-m1E03-2-0517 | 796.225 | OEt | a1 | B08 | b1 | C13 | c1 | D01 | d1 | E03 |
| 2-1-m1E03-2-0518 | 796.235 | OEt | a1 | B02 | b2 | C26 | c3 | D02 | d1 | E03 |
| 2-1-m1E03-2-0519 | 796.235 | OEt | a1 | B06 | b2 | C28 | c3 | D02 | d1 | E03 |

(continued)

[Table 8-10-4] Compound that may be contained in mixture 2-1-m1E03-2

| ID | Exact Mass | | a | B | b | C | c | D | d | E |
|---|---|---|---|---|---|---|---|---|---|---|
| 2-1-m1E03-2-0520 | 796.239 | OEt | a2 | B18 | b2 | C20 | c3 | D02 | d1 | E03 |
| 2-1-m1E03-2-0521 | 796.291 | OEt | a2 | B19 | b2 | C07 | c1 | D01 | d1 | E03 |
| 2-1-m1E03-2-0522 | 797.165 | OEt | a2 | B12 | b3 | C08 | c2 | D02 | d1 | E03 |
| 2-1-m1E03-2-0523 | 797.179 | OEt | a2 | B17 | b2 | C03 | c2 | D02 | d1 | E03 |
| 2-1-m1E03-2-0524 | 797.199 | OEt | a1 | B04 | b2 | C02 | c2 | D02 | d1 | E03 |
| 2-1-m1E03-2-0525 | 797.199 | OEt | a1 | B01 | b2 | C04 | c2 | D02 | d1 | E03 |
| 2-1-m1E03-2-0526 | 797.219 | OEt | a1 | B05 | b2 | C01 | c2 | D02 | d1 | E03 |
| 2-1-m1E03-2-0527 | 797.234 | OEt | a2 | B18 | b2 | C28 | c3 | D02 | d1 | E03 |
| 2-1-m1E03-2-0528 | 797.235 | OEt | a1 | B04 | b2 | C22 | c3 | D02 | d1 | E03 |
| 2-1-m1E03-2-0529 | 797.235 | OEt | a1 | B03 | b2 | C24 | c3 | D02 | d1 | E03 |
| 2-1-m1E03-2-0530 | 798.160 | OEt | a1 | B08 | b3 | C15 | c1 | D01 | d1 | E03 |
| 2-1-m1E03-2-0531 | 798.194 | OEt | a1 | B02 | b2 | C04 | c2 | D02 | d1 | E03 |
| 2-1-m1E03-2-0532 | 798.194 | OEt | a1 | B04 | b2 | C03 | c2 | D02 | d1 | E03 |
| 2-1-m1E03-2-0533 | 798.214 | OEt | a2 | B20 | b2 | C04 | c1 | D01 | d1 | E03 |
| 2-1-m1E03-2-0534 | 798.229 | OEt | a2 | B13 | b1 | C13 | c2 | D02 | d1 | E03 |
| 2-1-m1E03-2-0535 | 798.245 | OEt | a1 | B09 | b2 | C05 | c1 | D01 | d1 | E03 |
| 2-1-m1E03-2-0536 | 799.180 | OEt | a1 | B03 | b2 | C06 | c2 | D02 | d1 | E03 |
| 2-1-m1E03-2-0537 | 799.198 | OEt | a1 | B06 | b2 | C01 | c2 | D02 | d1 | E03 |
| 2-1-m1E03-2-0538 | 799.229 | OEt | a1 | B10 | b2 | C02 | c1 | D01 | d1 | E03 |
| 2-1-m1E03-2-0539 | 799.231 | OEt | a1 | B07 | b1 | C30 | c3 | D02 | d1 | E03 |
| 2-1-m1E03-2-0540 | 800.164 | OEt | a2 | B13 | b3 | C15 | c2 | D02 | d1 | E03 |
| 2-1-m1E03-2-0541 | 800.191 | OEt | a1 | B08 | b1 | C14 | c1 | D01 | d1 | E03 |
| 2-1-m1E03-2-0542 | 800.195 | OEt | a2 | B19 | b3 | C08 | c1 | D01 | d1 | E03 |
| 2-1-m1E03-2-0543 | 800.195 | OEt | a2 | B19 | b1 | C08 | c2 | D02 | d1 | E03 |
| 2-1-m1E03-2-0544 | 800.195 | OEt | a2 | B11 | b1 | C09 | c2 | D02 | d1 | E03 |
| 2-1-m1E03-2-0545 | 800.195 | OEt | a2 | B17 | b2 | C24 | c3 | D02 | d1 | E03 |
| 2-1-m1E03-2-0546 | 800.197 | OEt | a2 | B18 | b2 | C01 | c2 | D02 | d1 | E03 |
| 2-1-m1E03-2-0547 | 800.212 | OEt | a1 | B08 | b2 | C33 | c3 | D02 | d1 | E03 |
| 2-1-m1E03-2-0548 | 800.224 | OEt | a1 | B10 | b2 | C03 | c1 | D01 | d1 | E03 |
| 2-1-m1E03-2-0549 | 800.234 | OEt | a2 | B13 | b3 | C16 | c1 | D01 | d1 | E03 |
| 2-1-m1E03-2-0550 | 800.270 | OEt | a2 | B11 | b1 | C31 | c3 | D02 | d1 | E03 |
| 2-1-m1E03-2-0551 | 800.307 | OEt | a2 | B15 | b1 | C29 | c3 | D02 | d1 | E03 |
| 2-1-mIE03-2-0552 | 801.190 | OEt | a2 | B12 | b1 | C09 | c2 | D02 | d1 | E03 |
| 2-1-m1E03-2-0553 | 801.190 | OEt | a2 | B19 | b1 | C11 | c2 | D02 | d1 | E03 |
| 2-1-m1E03-2-0554 | 801.210 | OEt | a1 | B07 | b2 | C20 | c3 | D02 | d1 | E03 |
| 2-1-mIE03-2-0555 | 801.210 | OEt | a1 | B04 | b2 | C24 | c3 | D02 | d1 | E03 |
| 2-1-m1E03-2-0556 | 801.224 | OEt | a1 | B09 | b2 | C04 | c1 | D01 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0557 | 801.265 | OEt | a2 | B12 | b1 | C31 | c3 | D02 | d1 | E03 |
| 2-1-m1E03-2-0558 | 802.126 | OEt | a2 | B11 | b3 | C06 | c2 | D02 | d1 | E03 |
| 2-1-m1E03-2-0559 | 802.140 | OEt | a2 | B17 | b2 | C06 | c2 | D02 | d1 | E03 |
| 2-1-m1E03-2-0560 | 802.186 | OEt | a2 | B19 | b1 | C10 | c2 | D02 | d1 | E03 |
| 2-1-m1E03-2-0561 | 802.195 | OEt | a2 | B13 | b3 | C17 | c1 | D01 | d1 | E03 |
| 2-1-m1E03-2-0562 | 802.195 | OEt | a2 | B13 | b1 | C14 | c2 | D02 | d1 | E03 |
| 2-1-m1E03-2-0563 | 802.204 | OEt | a2 | B14 | b1 | C13 | c2 | D02 | d1 | E03 |
| 2-1-m1E03-2-0564 | 802.205 | OEt | a1 | B07 | b2 | C28 | c3 | D02 | d1 | E03 |
| 2-1-m1E03-2-0565 | 802.286 | OEt | a2 | B16 | b1 | C29 | c3 | D02 | d1 | E03 |
| 2-1-m1E03-2-0566 | 803.121 | OEt | a2 | B12 | b3 | C06 | c2 | D02 | d1 | E03 |
| 2-1-m1E03-2-0567 | 803.155 | OEt | a1 | B04 | b2 | C06 | c2 | D02 | d1 | E03 |
| 2-1-m1E03-2-0568 | 803.191 | OEt | a2 | B11 | b3 | C18 | c1 | D01 | d1 | E03 |
| 2-1-m1E03-2-0569 | 803.220 | OEt | a1 | B01 | b1 | C13 | c2 | D02 | d1 | E03 |
| 2-1-m1E03-2-0570 | 803.245 | OEt | a2 | B15 | b2 | C05 | c2 | D02 | d1 | E03 |
| 2-1-m1E03-2-0571 | 804.139 | OEt | a2 | B14 | b3 | C15 | c2 | D02 | d1 | E03 |
| 2-1-m1E03-2-0572 | 804.186 | OEt | a2 | B12 | b3 | C18 | c1 | D01 | d1 | E03 |
| 2-1-m1E03-2-0573 | 804.209 | OEt | a2 | B14 | b3 | C16 | c1 | D01 | d1 | E03 |
| 2-1-m1E03-2-0574 | 804.215 | OEt | a1 | B02 | b1 | C13 | c2 | D02 | d1 | E03 |
| 2-1-mIE03-2-0575 | 804.220 | OEt | a2 | B19 | b1 | C09 | c1 | D01 | d1 | E03 |
| 2-1-m1E03-2-0576 | 804.234 | OEt | a2 | B20 | b1 | C13 | c1 | D01 | d1 | E03 |
| 2-1-mIE03-2-0577 | 804.265 | OEt | a2 | B15 | b2 | C26 | c3 | D02 | d1 | E03 |
| 2-1-m1E03-2-0578 | 804.276 | OEt | a1 | B08 | b2 | C07 | c1 | D01 | d1 | E03 |
| 2-1-m1E03-2-0579 | 805.155 | OEt | a1 | B01 | b3 | C15 | c2 | D02 | d1 | E03 |
| 2-1-m1E03-2-0580 | 805.169 | OEt | a1 | B07 | b2 | C01 | c2 | D02 | d1 | E03 |
| 2-1-m1E03-2-0581 | 805.185 | OEt | a1 | B10 | b2 | C06 | c1 | D01 | d1 | E03 |
| 2-1-m1E03-2-0582 | 805.224 | OEt | a1 | B01 | b3 | C16 | c1 | D01 | d1 | E03 |
| 2-1-m1E03-2-0583 | 805.224 | OEt | a2 | B16 | b2 | C05 | c2 | D02 | d1 | E03 |
| 2-1-m1E03-2-0584 | 805.297 | OEt | a1 | B03 | b1 | C29 | c3 | D02 | d1 | E03 |
| 2-1-m1E03-2-0585 | 805.297 | OEt | a1 | B05 | b1 | C32 | c3 | D02 | d1 | E03 |
| 2-1-m1E03-2-0586 | 806.150 | OEt | a1 | B02 | b3 | C15 | c2 | D02 | d1 | E03 |
| 2-1-m1E03-2-0587 | 806.151 | OEt | a2 | B19 | b3 | C06 | c1 | D01 | d1 | E03 |
| 2-1-m1E03-2-0588 | 806.169 | OEt | a2 | B20 | b3 | C15 | c1 | D01 | d1 | E03 |
| 2-1-m1E03-2-0589 | 806.170 | OEt | a2 | B14 | b3 | C17 | c1 | D01 | d1 | E03 |
| 2-1-m1E03-2-0590 | 806.170 | OEt | a2 | B14 | b1 | C14 | c2 | D02 | d1 | E03 |
| 2-1-m1E03-2-0591 | 806.219 | OEt | a1 | B02 | b3 | C16 | c1 | D01 | d1 | E03 |
| 2-1-m1E03-2-0592 | 806.224 | OEt | a2 | B15 | b2 | C04 | c2 | D02 | d1 | E03 |
| 2-1-m1E03-2-0593 | 806.244 | OEt | a2 | B16 | b2 | C26 | c3 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0594 | 806.281 | OEt | a2 | B13 | b2 | C07 | c2 | D02 | d1 | E03 |
| 2-1-m1E03-2-0595 | 807.147 | OEt | a2 | B19 | b1 | C12 | c2 | D02 | d1 | E03 |
| 2-1-mIE03-2-0596 | 807.186 | OEt | a1 | B01 | b3 | C17 | c1 | D01 | d1 | E03 |
| 2-1-m1E03-2-0597 | 807.186 | OEt | a1 | B01 | b1 | C14 | c2 | D02 | d1 | E03 |
| 2-1-m1E03-2-0598 | 807.240 | OEt | a1 | B05 | b2 | C02 | c2 | D02 | d1 | E03 |
| 2-1-m1E03-2-0599 | 807.245 | OEt | a1 | B09 | b1 | C13 | c1 | D01 | d1 | E03 |
| 2-1-m1E03-2-0600 | 807.276 | OEt | a1 | B05 | b2 | C22 | c3 | D02 | d1 | E03 |
| 2-1-m1E03-2-0601 | 807.276 | OEt | a1 | B06 | b1 | C32 | c3 | D02 | d1 | E03 |
| 2-1-m1E03-2-0602 | 808.181 | OEt | a1 | B02 | b3 | C17 | c1 | D01 | d1 | E03 |
| 2-1-m1E03-2-0603 | 808.181 | OEt | a1 | B08 | b3 | C08 | c1 | D01 | d1 | E03 |
| 2-1-m1E03-2-0604 | 808.181 | OEt | a1 | B08 | b1 | C08 | c2 | D02 | d1 | E03 |
| 2-1-m1E03-2-0605 | 808.181 | OEt | a1 | B02 | b1 | C14 | c2 | D02 | d1 | E03 |
| 2-1-m1E03-2-0606 | 808.200 | OEt | a2 | B20 | b1 | C14 | c1 | D01 | d1 | E03 |
| 2-1-m1E03-2-0607 | 808.204 | OEt | a2 | B16 | b2 | C04 | c2 | D02 | d1 | E03 |
| 2-1-m1E03-2-0608 | 808.221 | OEt | a2 | B20 | b2 | C33 | c3 | D02 | d1 | E03 |
| 2-1-m1E03-2-0609 | 808.235 | OEt | a1 | B05 | b2 | C03 | c2 | D02 | d1 | E03 |
| 2-1-m1E03-2-0610 | 808.235 | OEt | a1 | B03 | b2 | C05 | c2 | D02 | d1 | E03 |
| 2-1-m1E03-2-0611 | 808.256 | OEt | a2 | B17 | b1 | C29 | c3 | D02 | d1 | E03 |
| 2-1-m1E03-2-0612 | 808.275 | OEt | a2 | B18 | b1 | C32 | c3 | D02 | d1 | E03 |
| 2-1-m1E03-2-0613 | 809.176 | OEt | a1 | B08 | b1 | C11 | c2 | D02 | d1 | E03 |
| 2-1-m1E03-2-0614 | 809.180 | OEt | a1 | B09 | b3 | C15 | c1 | D01 | d1 | E03 |
| 2-1-m1E03-2-0615 | 809.219 | OEt | a1 | B06 | b2 | C02 | c2 | D02 | d1 | E03 |
| 2-1-m1E03-2-0616 | 809.255 | OEt | a1 | B06 | b2 | C22 | c3 | D02 | d1 | E03 |
| 2-1-m1E03-2-0617 | 809.255 | OEt | a1 | B03 | b2 | C26 | c3 | D02 | d1 | E03 |
| 2-1-m1E03-2-0618 | 809.272 | OEt | a1 | B04 | b1 | C29 | c3 | D02 | d1 | E03 |
| 2-1-m1E03-2-0619 | 810.171 | OEt | a1 | B08 | b1 | C10 | c2 | D02 | d1 | E03 |
| 2-1-m1E03-2-0620 | 810.185 | OEt | a2 | B13 | b3 | C08 | c2 | D02 | d1 | E03 |
| 2-1-m1E03-2-0621 | 810.214 | OEt | a1 | B06 | b2 | C03 | c2 | D02 | d1 | E03 |
| 2-1-m1E03-2-0622 | 810.218 | OEt | a2 | B18 | b2 | C02 | c2 | D02 | d1 | E03 |
| 2-1-m1E03-2-0623 | 810.255 | OEt | a2 | B18 | b2 | C22 | c3 | D02 | d1 | E03 |
| 2-1-m1E03-2-0624 | 810.256 | OEt | a2 | B14 | b2 | C07 | c2 | D02 | d1 | E03 |
| 2-1-m1E03-2-0625 | 811.195 | OEt | a2 | B17 | b2 | C05 | c2 | D02 | d1 | E03 |
| 2-1-m1E03-2-0626 | 811.211 | OEt | a1 | B09 | b1 | C14 | c1 | D01 | d1 | E03 |
| 2-1-m1E03-2-0627 | 811.213 | OEt | a2 | B18 | b2 | C03 | c2 | D02 | d1 | E03 |
| 2-1-m1E03-2-0628 | 811.215 | OEt | a1 | B03 | b2 | C04 | c2 | D02 | d1 | E03 |
| 2-1-m1E03-2-0629 | 811.232 | OEt | a1 | B09 | b2 | C33 | c3 | D02 | d1 | E03 |
| 2-1-m1E03-2-0630 | 811.251 | OEt | a1 | B05 | b2 | C24 | c3 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0631 | 811.271 | OEt | a1 | B01 | b2 | C07 | c2 | D02 | d1 | E03 |
| 2-1-m1E03-2-0632 | 812.206 | OEt | a1 | B08 | b1 | C09 | c1 | D01 | d1 | E03 |
| 2-1-m1E03-2-0633 | 812.210 | OEt | a1 | B04 | b2 | C05 | c2 | D02 | d1 | E03 |
| 2-1-m1E03-2-0634 | 812.215 | OEt | a2 | B17 | b2 | C26 | c3 | D02 | d1 | E03 |
| 2-1-m1E03-2-0635 | 812.231 | OEt | a2 | B19 | b1 | C30 | c3 | D02 | d1 | E03 |
| 2-1-m1E03-2-0636 | 812.245 | OEt | a2 | B15 | b1 | C13 | c2 | D02 | d1 | E03 |
| 2-1-m1E03-2-0637 | 812.266 | OEt | a1 | B02 | b2 | C07 | c2 | D02 | d1 | E03 |
| 2-1-m1E03-2-0638 | 812.286 | OEt | a2 | B20 | b2 | C07 | c1 | D01 | d1 | E03 |
| 2-1-m1E03-2-0639 | 813.196 | OEt | a1 | B05 | b2 | C06 | c2 | D02 | d1 | E03 |
| 2-1-m1E03-2-0640 | 813.230 | OEt | a1 | B06 | b2 | C24 | c3 | D02 | d1 | E03 |
| 2-1-m1E03-2-0641 | 813.230 | OEt | a1 | B04 | b2 | C26 | c3 | D02 | d1 | E03 |
| 2-1-m1E03-2-0642 | 813.247 | OEt | a1 | B07 | b1 | C32 | c3 | D02 | d1 | E03 |
| 2-1-m1E03-2-0643 | 814.137 | OEt | a1 | B08 | b3 | C06 | c1 | D01 | d1 | E03 |
| 2-1-m1E03-2-0644 | 814.160 | OEt | a2 | B14 | b3 | C08 | c2 | D02 | d1 | E03 |
| 2-1-m1E03-2-0645 | 814.174 | OEt | a2 | B17 | b2 | C04 | c2 | D02 | d1 | E03 |
| 2-1-m1E03-2-0646 | 814.180 | OEt | a2 | B15 | b3 | C15 | c2 | D02 | d1 | E03 |
| 2-1-m1E03-2-0647 | 814.211 | OEt | a2 | B13 | b1 | C09 | c2 | D02 | d1 | E03 |
| 2-1-m1E03-2-0648 | 814.211 | OEt | a2 | B19 | b2 | C20 | c3 | D02 | d1 | E03 |
| 2-1-mlE03-2-0649 | 814.224 | OEt | a2 | B16 | b1 | C13 | c2 | D02 | d1 | E03 |
| 2-1-m1E03-2-0650 | 814.230 | OEt | a2 | B18 | b2 | C24 | c3 | D02 | d1 | E03 |
| 2-1-m1E03-2-0651 | 814.240 | OEt | a1 | B10 | b2 | C05 | c1 | D01 | d1 | E03 |
| 2-1-m1E03-2-0652 | 814.249 | OEt | a2 | B15 | b3 | C16 | c1 | D01 | d1 | E03 |
| 2-1-m1E03-2-0653 | 814.286 | OEt | a2 | B13 | b1 | C31 | c3 | D02 | d1 | E03 |
| 2-1-m1E03-2-0654 | 815.132 | OEt | a1 | B08 | b1 | C12 | c2 | D02 | d1 | E03 |
| 2-1-m1E03-2-0655 | 815.175 | OEt | a1 | B06 | b2 | C06 | c2 | D02 | d1 | E03 |
| 2-1-m1E03-2-0656 | 815.175 | OEt | a1 | B01 | b3 | C08 | c2 | D02 | d1 | E03 |
| 2-1-m1E03-2-0657 | 815.190 | OEt | a1 | B07 | b2 | C02 | c2 | D02 | d1 | E03 |
| 2-1-m1E03-2-0658 | 815.190 | OEt | a1 | B04 | b2 | C04 | c2 | D02 | d1 | E03 |
| 2-1-m1E03-2-0659 | 815.206 | OEt | a2 | B19 | b2 | C28 | c3 | D02 | d1 | E03 |
| 2-1-m1E03-2-0660 | 815.226 | OEt | a1 | B07 | b2 | C22 | c3 | D02 | d1 | E03 |
| 2-1-m1E03-2-0661 | 815.296 | OEt | a1 | B09 | b2 | C07 | c1 | D01 | d1 | E03 |
| 2-1-m1E03-2-0662 | 816.142 | OEt | a2 | B13 | b3 | C06 | c2 | D02 | d1 | E03 |
| 2-1-m1E03-2-0663 | 816.159 | OEt | a2 | B16 | b3 | C15 | c2 | D02 | d1 | E03 |
| 2-1-m1E03-2-0664 | 816.171 | OEt | a1 | B02 | b3 | C08 | c2 | D02 | d1 | E03 |
| 2-1-m1E03-2-0665 | 816.175 | OEt | a2 | B18 | b2 | C06 | c2 | D02 | d1 | E03 |
| 2-1-m1E03-2-0666 | 816.185 | OEt | a1 | B07 | b2 | C03 | c2 | D02 | d1 | E03 |
| 2-1-m1E03-2-0667 | 816.190 | OEt | a2 | B20 | b3 | C08 | c1 | D01 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0668 | 816.190 | OEt | a2 | B20 | b1 | C08 | c2 | D02 | d1 | E03 |
| 2-1-m1E03-2-0669 | 816.211 | OEt | a2 | B15 | b3 | C17 | c1 | D01 | d1 | E03 |
| 2-1-m1E03-2-0670 | 816.211 | OEt | a2 | B15 | b1 | C14 | c2 | D02 | d1 | E03 |
| 2-1-m1E03-2-0671 | 816.229 | OEt | a2 | B16 | b3 | C16 | c1 | D01 | d1 | E03 |
| 2-1-m1E03-2-0672 | 817.185 | OEt | a2 | B20 | b1 | C11 | c2 | D02 | d1 | E03 |
| 2-1-m1E03-2-0673 | 817.206 | OEt | a2 | B13 | b3 | C18 | c1 | D01 | d1 | E03 |
| 2-1-m1E03-2-0674 | 817.219 | OEt | a1 | B10 | b2 | C04 | c1 | D01 | d1 | E03 |
| 2-1-m1E03-2-0675 | 817.235 | OEt | a1 | B03 | b1 | C13 | c2 | D02 | d1 | E03 |
| 2-1-m1E03-2-0676 | 818.169 | OEt | a2 | B19 | b2 | C01 | c2 | D02 | d1 | E03 |
| 2-1-m1E03-2-0677 | 818.180 | OEt | a2 | B20 | b1 | C10 | c2 | D02 | d1 | E03 |
| 2-1-m1E03-2-0678 | 818.186 | OEt | a2 | B14 | b1 | C09 | c2 | D02 | d1 | E03 |
| 2-1-m1E03-2-0679 | 818.190 | OEt | a2 | B16 | b3 | C17 | c1 | D01 | d1 | E03 |
| 2-1-m1E03-2-0680 | 818.190 | OEt | a2 | B16 | b1 | C14 | c2 | D02 | d1 | E03 |
| 2-1-m 1 E03-2-0681 | 818.261 | OEt | a2 | B14 | b1 | C31 | c3 | D02 | d1 | E03 |
| 2-1-m1E03-2-0682 | 819.170 | OEt | a1 | B03 | b3 | C15 | c2 | D02 | d1 | E03 |
| 2-1-m1E03-2-0683 | 819.201 | OEt | a1 | B09 | b3 | C08 | c1 | D01 | d1 | E03 |
| 2-1-m1E03-2-0684 | 819.201 | OEt | a1 | B09 | b1 | C08 | c2 | D02 | d1 | E03 |
| 2-1-m1E03-2-0685 | 819.201 | OEt | a1 | B01 | b1 | C09 | c2 | D02 | d1 | E03 |
| 2-1-m1E03-2-0686 | 819.201 | OEt | a1 | B07 | b2 | C24 | c3 | D02 | d1 | E03 |
| 2-1-m1E03-2-0687 | 819.240 | OEt | a1 | B03 | b3 | C16 | c1 | D01 | d1 | E03 |
| 2-1-mlE03-2-0688 | 819.276 | OEt | a1 | B01 | b1 | C31 | c3 | D02 | d1 | E03 |
| 2-1-mlE03-2-0689 | 819.312 | OEt | a1 | B05 | b1 | C29 | c3 | D02 | d1 | E03 |
| 2-1-m1E03-2-0690 | 820.117 | OEt | a2 | B14 | b3 | C06 | c2 | D02 | d1 | E03 |
| 2-1-m1E03-2-0691 | 820.195 | OEt | a2 | B17 | b1 | C13 | c2 | D02 | d1 | E03 |
| 2-1-m1E03-2-0692 | 820.196 | OEt | a1 | B02 | b1 | C09 | c2 | D02 | d1 | E03 |
| 2-1-m1E03-2-0693 | 820.196 | OEt | a1 | B09 | b1 | C11 | c2 | D02 | d1 | E03 |
| 2-1-m1E03-2-0694 | 820.215 | OEt | a2 | B20 | b1 | C09 | c1 | D01 | d1 | E03 |
| 2-1-m1E03-2-0695 | 820.217 | OEt | a1 | B08 | b1 | C30 | c3 | D02 | d1 | E03 |
| 2-1-m1E03-2-0696 | 820.271 | OEt | a1 | B02 | b1 | C31 | c3 | D02 | d1 | E03 |
| 2-1-m1E03-2-0697 | 820.296 | OEt | a2 | B15 | b2 | C07 | c2 | D02 | d1 | E03 |
| 2-1-m1E03-2-0698 | 821.132 | OEt | a1 | B01 | b3 | C06 | c2 | D02 | d1 | E03 |
| 2-1-m1E03-2-0699 | 821.146 | OEt | a1 | B07 | b2 | C06 | c2 | D02 | d1 | E03 |
| 2-1-m1E03-2-0700 | 821.181 | OEt | a2 | B14 | b3 | C18 | c1 | D01 | d1 | E03 |
| 2-1-m1E03-2-0701 | 821.191 | OEt | a1 | B09 | b1 | C10 | c2 | D02 | d1 | E03 |
| 2-1-m1E03-2-0702 | 821.201 | OEt | a1 | B03 | b3 | C17 | c1 | D01 | d1 | E03 |
| 2-1-m1E03-2-0703 | 821.201 | OEt | a1 | B03 | b1 | C14 | c2 | D02 | d1 | E03 |
| 2-1-m1E03-2-0704 | 821.210 | OEt | a1 | B04 | b1 | C13 | c2 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0705 | 821.292 | OEt | a1 | B06 | b1 | C29 | c3 | D02 | d1 | E03 |
| 2-1-m1E03-2-0706 | 822.127 | OEt | a1 | B02 | b3 | C06 | c2 | D02 | d1 | E03 |
| 2-1-m1E03-2-0707 | 822.130 | OEt | a2 | B17 | b3 | C15 | c2 | D02 | d1 | E03 |
| 2-1-m1E03-2-0708 | 822.146 | OEt | a2 | B20 | b3 | C06 | c1 | D01 | d1 | E03 |
| 2-1-m1E03-2-0709 | 822.196 | OEt | a1 | B01 | b3 | C18 | c1 | D01 | d1 | E03 |
| 2-1-m1E03-2-0710 | 822.196 | OEt | a1 | B08 | b2 | C20 | c3 | D02 | d1 | E03 |
| 2-1-m1E03-2-0711 | 822.199 | OEt | a2 | B17 | b3 | C16 | c1 | D01 | d1 | E03 |
| 2-1-m1E03-2-0712 | 822.251 | OEt | a1 | B05 | b2 | C05 | c2 | D02 | d1 | E03 |
| 2-1-m1E03-2-0713 | 822.276 | OEt | a2 | B16 | b2 | C07 | c2 | D02 | d1 | E03 |
| 2-1-m1E03-2-0714 | 822.291 | OEt | a2 | B18 | b1 | C29 | c3 | D02 | d1 | E03 |
| 2-1-m1E03-2-0715 | 823.142 | OEt | a2 | B20 | b1 | C12 | c2 | D02 | d1 | E03 |
| 2-1-m1E03-2-0716 | 823.145 | OEt | a1 | B04 | b3 | C15 | c2 | D02 | d1 | E03 |
| 2-1-m1E03-2-0717 | 823.192 | OEt | a1 | B02 | b3 | C18 | c1 | D01 | d1 | E03 |
| 2-1-m1E03-2-0718 | 823.192 | OEt | a1 | B08 | b2 | C28 | c3 | D02 | d1 | E03 |
| 2-1-m1E03-2-0719 | 823.215 | OEt | a1 | B04 | b3 | C16 | c1 | D01 | d1 | E03 |
| 2-1-m1E03-2-0720 | 823.226 | OEt | a1 | B09 | b1 | C09 | c1 | D01 | d1 | E03 |
| 2-1-m1E03-2-0721 | 823.240 | OEt | a1 | B10 | b1 | C13 | c1 | D01 | d1 | E03 |
| 2-1-m1E03-2-0722 | 823.271 | OEt | a1 | B05 | b2 | C26 | c3 | D02 | d1 | E03 |
| 2-1-m1E03-2-0723 | 824.161 | OEt | a2 | B17 | b3 | C17 | c1 | D01 | d1 | E03 |
| 2-1-m1E03-2-0724 | 824.161 | OEt | a2 | B17 | b1 | C14 | c2 | D02 | d1 | E03 |
| 2-1-m1E03-2-0725 | 824.201 | OEt | a2 | B15 | b3 | C08 | c2 | D02 | d1 | E03 |
| 2-1-m 1 E03-2-0726 | 824.230 | OEt | a1 | B06 | b2 | C05 | c2 | D02 | d1 | E03 |
| 2-1-m1E03-2-0727 | 825.157 | OEt | a1 | B09 | b3 | C06 | c1 | D01 | d1 | E03 |
| 2-1-m1E03-2-0728 | 825.175 | OEt | a1 | B10 | b3 | C15 | c1 | D01 | d1 | E03 |
| 2-1-m1E03-2-0729 | 825.176 | OEt | a1 | B04 | b3 | C17 | c1 | D01 | d1 | E03 |
| 2-1-m1E03-2-0730 | 825.176 | OEt | a1 | B04 | b1 | C14 | c2 | D02 | d1 | E03 |
| 2-1-m1E03-2-0731 | 825.229 | OEt | a2 | B18 | b2 | C05 | c2 | D02 | d1 | E03 |
| 2-1-m1E03-2-0732 | 825.230 | OEt | a1 | B05 | b2 | C04 | c2 | D02 | d1 | E03 |
| 2-1-m1E03-2-0733 | 825.250 | OEt | a1 | B06 | b2 | C26 | c3 | D02 | d1 | E03 |
| 2-1-m1E03-2-0734 | 825.287 | OEt | a1 | B03 | b2 | C07 | c2 | D02 | d1 | E03 |
| 2-1-m1E03-2-0735 | 826.152 | OEt | a1 | B09 | b1 | C12 | c2 | D02 | d1 | E03 |
| 2-1-m1E03-2-0736 | 826.155 | OEt | a1 | B08 | b2 | C01 | c2 | D02 | d1 | E03 |
| 2-1-m1E03-2-0737 | 826.180 | OEt | a2 | B16 | b3 | C08 | c2 | D02 | d1 | E03 |
| 2-1-m1E03-2-0738 | 826.247 | OEt | a2 | B19 | b1 | C32 | c3 | D02 | d1 | E03 |
| 2-1-m1E03-2-0739 | 826.249 | OEt | a2 | B18 | b2 | C26 | c3 | D02 | d1 | E03 |
| 2-1-m1E03-2-0740 | 827.206 | OEt | a1 | B10 | b1 | C14 | c1 | D01 | d1 | E03 |
| 2-1-m1E03-2-0741 | 827.209 | OEt | a1 | B06 | b2 | C04 | c2 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m 1 E03-2-0742 | 827.227 | OEt | a1 | B10 | b2 | C33 | c3 | D02 | d1 | E03 |
| 2-1-mlE03-2-0743 | 827.262 | OEt | a1 | B07 | b1 | C29 | c3 | D02 | d1 | E03 |
| 2-1-m 1 E03-2-0744 | 828.190 | OEt | a2 | B19 | b2 | C02 | c2 | D02 | d1 | E03 |
| 2-1-m1E03-2-0745 | 828.209 | OEt | a2 | B18 | b2 | C04 | c2 | D02 | d1 | E03 |
| 2-1-m 1 E03-2-0746 | 828.226 | OEt | a2 | B15 | b1 | C09 | c2 | D02 | d1 | E03 |
| 2-1-mlE03-2-0747 | 828.226 | OEt | a2 | B19 | b2 | C22 | c3 | D02 | d1 | E03 |
| 2-1-m1E03-2-0748 | 828.226 | OEt | a2 | B20 | b1 | C30 | c3 | D02 | d1 | E03 |
| 2-1-m1E03-2-0749 | 828.246 | OEt | a2 | B17 | b2 | C07 | c2 | D02 | d1 | E03 |
| 2-1-m1E03-2-0750 | 828.302 | OEt | a2 | B15 | b1 | C31 | c3 | D02 | d1 | E03 |
| 2-1-m1E03-2-0751 | 829.185 | OEt | a2 | B19 | b2 | C03 | c2 | D02 | d1 | E03 |
| 2-1-m1E03-2-0752 | 829.191 | OEt | a1 | B03 | b3 | C08 | c2 | D02 | d1 | E03 |
| 2-1-m1E03-2-0753 | 829.262 | OEt | a1 | B04 | b2 | C07 | c2 | D02 | d1 | E03 |
| 2-1-m1E03-2-0754 | 830.157 | OEt | a2 | B15 | b3 | C06 | c2 | D02 | d1 | E03 |
| 2-1-m1E03-2-0755 | 830.200 | OEt | a1 | B07 | b2 | C05 | c2 | D02 | d1 | E03 |
| 2-1-m1E03-2-0756 | 830.206 | OEt | a2 | B16 | b1 | C09 | c2 | D02 | d1 | E03 |
| 2-1-m1E03-2-0757 | 830.206 | OEt | a2 | B20 | b2 | C20 | c3 | D02 | d1 | E03 |
| 2-1-m1E03-2-0758 | 830.281 | OEt | a2 | B16 | b1 | C31 | c3 | D02 | d1 | E03 |
| 2-1-m1E03-2-0759 | 831.201 | OEt | a2 | B20 | b2 | C28 | c3 | D02 | d1 | E03 |
| 2-1-m1E03-2-0760 | 831.221 | OEt | a1 | B07 | b2 | C26 | c3 | D02 | d1 | E03 |
| 2-1-m 1 E03-2-0761 | 831.222 | OEt | a2 | B15 | b3 | C18 | c1 | D01 | d1 | E03 |
| 2-1-m 1 E03-2-0762 | 831.237 | OEt | a1 | B09 | b1 | C30 | c3 | D02 | d1 | E03 |
| 2-1-m1E03-2-0763 | 831.251 | OEt | a1 | B05 | b1 | C13 | c2 | D02 | d1 | E03 |
| 2-1-m 1 E03-2-0764 | 831.291 | OEt | a1 | B10 | b2 | C07 | c1 | D01 | d1 | E03 |
| 2-1-m1E03-2-0765 | 832.137 | OEt | a2 | B16 | b3 | C06 | c2 | D02 | d1 | E03 |
| 2-1-m1E03-2-0766 | 832.151 | OEt | a2 | B17 | b3 | C08 | c2 | D02 | d1 | E03 |
| 2-1-m1E03-2-0767 | 832.201 | OEt | a2 | B19 | b2 | C24 | c3 | D02 | d1 | E03 |
| 2-1-m1E03-2-0768 | 833.166 | OEt | a1 | B04 | b3 | C08 | c2 | D02 | d1 | E03 |
| 2-1-m1E03-2-0769 | 833.180 | OEt | a1 | B07 | b2 | C04 | c2 | D02 | d1 | E03 |
| 2-1-m1E03-2-0770 | 833.186 | OEt | a1 | B05 | b3 | C15 | c2 | D02 | d1 | E03 |
| 2-1-m1E03-2-0771 | 833.201 | OEt | a2 | B16 | b3 | C18 | c1 | D01 | d1 | E03 |
| 2-1-m1E03-2-0772 | 833.216 | OEt | a1 | B03 | b1 | C09 | c2 | D02 | d1 | E03 |
| 2-1-m1E03-2-0773 | 833.216 | OEt | a1 | B09 | b2 | C20 | c3 | D02 | d1 | E03 |
| 2-1-m1E03-2-0774 | 833.230 | OEt | a1 | B06 | b1 | C13 | c2 | D02 | d1 | E03 |
| 2-1-m1E03-2-0775 | 833.255 | OEt | a1 | B05 | b3 | C16 | c1 | D01 | d1 | E03 |
| 2-1-m1E03-2-0776 | 833.292 | OEt | a1 | B03 | b1 | C31 | c3 | D02 | d1 | E03 |
| 2-1-m1E03-2-0777 | 834.146 | OEt | a2 | B19 | b2 | C06 | c2 | D02 | d1 | E03 |
| 2-1-m1E03-2-0778 | 834.164 | OEt | a2 | B20 | b2 | C01 | c2 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0779 | 834.212 | OEt | a1 | B09 | b2 | C28 | c3 | D02 | d1 | E03 |
| 2-1-m1E03-2-0780 | 834.229 | OEt | a2 | B18 | b1 | C13 | c2 | D02 | d1 | E03 |
| 2-1-m1E03-2-0781 | 834.233 | OEt | a1 | B08 | b1 | C32 | c3 | D02 | d1 | E03 |
| 2-1-mlE03-2-0782 | 835.147 | OEt | a1 | B03 | b3 | C06 | c2 | D02 | d1 | E03 |
| 2-1-m1E03-2-0783 | 835.165 | OEt | a1 | B06 | b3 | C15 | c2 | D02 | d1 | E03 |
| 2-1-m1E03-2-0784 | 835.196 | OEt | a1 | B10 | b3 | C08 | c1 | D01 | d1 | E03 |
| 2-1-m1E03-2-0785 | 835.196 | OEt | a1 | B10 | b1 | C08 | c2 | D02 | d1 | E03 |
| 2-1-m1E03-2-0786 | 835.217 | OEt | a1 | B05 | b3 | C17 | c1 | D01 | d1 | E03 |
| 2-1-m1E03-2-0787 | 835.217 | OEt | a1 | B05 | b1 | C14 | c2 | D02 | d1 | E03 |
| 2-1-m1E03-2-0788 | 835.235 | OEt | a1 | B06 | b3 | C16 | c1 | D01 | d1 | E03 |
| 2-1-m1E03-2-0789 | 836.164 | OEt | a2 | B18 | b3 | C15 | c2 | D02 | d1 | E03 |
| 2-1-m1E03-2-0790 | 836.176 | OEt | a1 | B08 | b2 | C02 | c2 | D02 | d1 | E03 |
| 2-1-m 1 E03-2-0791 | 836.176 | OEt | a2 | B17 | b1 | C09 | c2 | D02 | d1 | E03 |
| 2-1-m 1 E03-2-0792 | 836.191 | OEt | a1 | B10 | b1 | C11 | c2 | D02 | d1 | E03 |
| 2-1-m1E03-2-0793 | 836.212 | OEt | a1 | B03 | b3 | C18 | c1 | D01 | d1 | E03 |
| 2-1-m 1 E03-2-0794 | 836.212 | OEt | a1 | B08 | b2 | C22 | c3 | D02 | d1 | E03 |
| 2-1-m1E03-2-0795 | 836.234 | OEt | a2 | B18 | b3 | C16 | c1 | D01 | d1 | E03 |
| 2-1-m1E03-2-0796 | 836.251 | OEt | a2 | B17 | b1 | C31 | c3 | D02 | d1 | E03 |
| 2-1-m1E03-2-0797 | 837.171 | OEt | a1 | B08 | b2 | C03 | c2 | D02 | d1 | E03 |
| 2-1-m1E03-2-0798 | 837.175 | OEt | a1 | B09 | b2 | C01 | c2 | D02 | d1 | E03 |
| 2-1-m1E03-2-0799 | 837.186 | OEt | a1 | B10 | b1 | C10 | c2 | D02 | d1 | E03 |
| 2-1-m1E03-2-0800 | 837.191 | OEt | a1 | B04 | b1 | C09 | c2 | D02 | d1 | E03 |
| 2-1-m1E03-2-0801 | 837.196 | OEt | a1 | B06 | b3 | C17 | c1 | D01 | d1 | E03 |
| 2-1-m1E03-2-0802 | 837.196 | OEt | a1 | B06 | b1 | C14 | c2 | D02 | d1 | E03 |
| 2-1-m1E03-2-0803 | 837.267 | OEt | a1 | B04 | b1 | C31 | c3 | D02 | d1 | E03 |
| 2-1-m1E03-2-0804 | 838.107 | OEt | a2 | B17 | b3 | C06 | c2 | D02 | d1 | E03 |
| 2-1-m1E03-2-0805 | 838.195 | OEt | a2 | B18 | b3 | C17 | c1 | D01 | d1 | E03 |
| 2-1-m1E03-2-0806 | 838.195 | OEt | a2 | B18 | b1 | C14 | c2 | D02 | d1 | E03 |
| 2-1-m1E03-2-0807 | 839.122 | OEt | a1 | B04 | b3 | C06 | c2 | D02 | d1 | E03 |
| 2-1-m1E03-2-0808 | 839.172 | OEt | a2 | B17 | b3 | C18 | c1 | D01 | d1 | E03 |
| 2-1-m1E03-2-0809 | 839.201 | OEt | a1 | B07 | b1 | C13 | c2 | D02 | d1 | E03 |
| 2-1-m1E03-2-0810 | 839.221 | OEt | a1 | B10 | b1 | C09 | c1 | D01 | d1 | E03 |
| 2-1-m1E03-2-0811 | 839.302 | OEt | a1 | B05 | b2 | C07 | c2 | D02 | d1 | E03 |
| 2-1-m1E03-2-0812 | 840.187 | OEt | a1 | B04 | b3 | C18 | c1 | D01 | d1 | E03 |
| 2-1-m1E03-2-0813 | 840.187 | OEt | a1 | B08 | b2 | C24 | c3 | D02 | d1 | E03 |
| 2-1-m1E03-2-0814 | 840.263 | OEt | a2 | B19 | b1 | C29 | c3 | D02 | d1 | E03 |
| 2-1-m1E03-2-0815 | 841.136 | OEt | a1 | B07 | b3 | C15 | c2 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0816 | 841.152 | OEt | a1 | B10 | b3 | C06 | c1 | D01 | d1 | E03 |
| 2-1-m1E03-2-0817 | 841.205 | OEt | a1 | B07 | b3 | C16 | c1 | D01 | d1 | E03 |
| 2-1-m1E03-2-0818 | 841.282 | OEt | a1 | B06 | b2 | C07 | c2 | D02 | d1 | E03 |
| 2-1-m1E03-2-0819 | 842.132 | OEt | a1 | B08 | b2 | C06 | c2 | D02 | d1 | E03 |
| 2-1-m1E03-2-0820 | 842.147 | OEt | a1 | B10 | b1 | C12 | c2 | D02 | d1 | E03 |
| 2-1-m1E03-2-0821 | 842.242 | OEt | a2 | B20 | b1 | C32 | c3 | D02 | d1 | E03 |
| 2-1-m1E03-2-0822 | 842.281 | OEt | a2 | B18 | b2 | C07 | c2 | D02 | d1 | E03 |
| 2-1-m1E03-2-0823 | 843.167 | OEt | a1 | B07 | b3 | C17 | c1 | D01 | d1 | E03 |
| 2-1-m1E03-2-0824 | 843.167 | OEt | a1 | B07 | b1 | C14 | c2 | D02 | d1 | E03 |
| 2-1-m1E03-2-0825 | 843.201 | OEt | a2 | B19 | b2 | C05 | c2 | D02 | d1 | E03 |
| 2-1-m1E03-2-0826 | 843.207 | OEt | a1 | B05 | b3 | C08 | c2 | D02 | d1 | E03 |
| 2-1-m1E03-2-0827 | 844.185 | OEt | a2 | B20 | b2 | C02 | c2 | D02 | d1 | E03 |
| 2-1-m1E03-2-0828 | 844.221 | OEt | a2 | B20 | b2 | C22 | c3 | D02 | d1 | E03 |
| 2-1-m1E03-2-0829 | 844.221 | OEt | a2 | B19 | b2 | C26 | c3 | D02 | d1 | E03 |
| 2-1-m1E03-2-0830 | 845.180 | OEt | a2 | B20 | b2 | C03 | c2 | D02 | d1 | E03 |
| 2-1-m1E03-2-0831 | 845.186 | OEt | a1 | B06 | b3 | C08 | c2 | D02 | d1 | E03 |
| 2-1-m1E03-2-0832 | 845.253 | OEt | a1 | B09 | b1 | C32 | c3 | D02 | d1 | E03 |
| 2-1-m1E03-2-0833 | 846.181 | OEt | a2 | B19 | b2 | C04 | c2 | D02 | d1 | E03 |
| 2-1-mlE03-2-0834 | 846.185 | OEt | a2 | B18 | b3 | C08 | c2 | D02 | d1 | E03 |
| 2-1-m1E03-2-0835 | 847.196 | OEt | a1 | B09 | b2 | C02 | c2 | D02 | d1 | E03 |
| 2-1-m1E03-2-0836 | 847.232 | OEt | a1 | B05 | b1 | C09 | c2 | D02 | d1 | E03 |
| 2-1-mlE03-2-0837 | 847.232 | OEt | a1 | B09 | b2 | C22 | c3 | D02 | d1 | E03 |
| 2-1-m1E03-2-0838 | 847.232 | OEt | a1 | B10 | b1 | C30 | c3 | D02 | d1 | E03 |
| 2-1-m1E03-2-0839 | 847.252 | OEt | a1 | B07 | b2 | C07 | c2 | D02 | d1 | E03 |
| 2-1-m1E03-2-0840 | 847.307 | OEt | a1 | B05 | b1 | C31 | c3 | D02 | d1 | E03 |
| 2-1-m1E03-2-0841 | 848.191 | OEt | a1 | B09 | b2 | C03 | c2 | D02 | d1 | E03 |
| 2-1-m1E03-2-0842 | 848.196 | OEt | a2 | B20 | b2 | C24 | c3 | D02 | d1 | E03 |
| 2-1-m1E03-2-0843 | 848.248 | OEt | a1 | B08 | b1 | C29 | c3 | D02 | d1 | E03 |
| 2-1-m1E03-2-0844 | 849.163 | OEt | a1 | B05 | b3 | C06 | c2 | D02 | d1 | E03 |
| 2-1-m1E03-2-0845 | 849.211 | OEt | a1 | B06 | b1 | C09 | c2 | D02 | d1 | E03 |
| 2-1-m1E03-2-0846 | 849.211 | OEt | a1 | B10 | b2 | C20 | c3 | D02 | d1 | E03 |
| 2-1-m1E03-2-0847 | 849.287 | OEt | a1 | B06 | b1 | C31 | c3 | D02 | d1 | E03 |
| 2-1-m1E03-2-0848 | 850.141 | OEt | a2 | B20 | b2 | C06 | c2 | D02 | d1 | E03 |
| 2-1-m1E03-2-0849 | 850.180 | OEt | a2 | B11 | b3 | C16 | c2 | D02 | d1 | E03 |
| 2-1-m1E03-2-0850 | 850.207 | OEt | a1 | B10 | b2 | C28 | c3 | D02 | d1 | E03 |
| 2-1-m1E03-2-0851 | 850.211 | OEt | a2 | B18 | b1 | C09 | c2 | D02 | d1 | E03 |
| 2-1-m1E03-2-0852 | 850.228 | OEt | a1 | B05 | b3 | C18 | c1 | D01 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0853 | 850.286 | OEt | a2 | B18 | b1 | C31 | c3 | D02 | d1 | E03 |
| 2-1-m1E03-2-0854 | 851.142 | OEt | a1 | B06 | b3 | C06 | c2 | D02 | d1 | E03 |
| 2-1-m1E03-2-0855 | 851.156 | OEt | a1 | B07 | b3 | C08 | c2 | D02 | d1 | E03 |
| 2-1-m1E03-2-0856 | 851.175 | OEt | a2 | B12 | b3 | C16 | c2 | D02 | d1 | E03 |
| 2-1-m1E03-2-0857 | 851.187 | OEt | a1 | B08 | b2 | C05 | c2 | D02 | d1 | E03 |
| 2-1-m1E03-2-0858 | 851.207 | OEt | a1 | B09 | b2 | C24 | c3 | D02 | d1 | E03 |
| 2-1-m1E03-2-0859 | 852.142 | OEt | a2 | B18 | b3 | C06 | c2 | D02 | d1 | E03 |
| 2-1-m1E03-2-0860 | 852.142 | OEt | a2 | B11 | b3 | C17 | c2 | D02 | d1 | E03 |
| 2-1-m1E03-2-0861 | 852.201 | OEt | a2 | B19 | b1 | C13 | c2 | D02 | d1 | E03 |
| 2-1-mlE03-2-0862 | 852.207 | OEt | a1 | B06 | b3 | C18 | c1 | D01 | d1 | E03 |
| 2-1-m1E03-2-0863 | 852.207 | OEt | a1 | B08 | b2 | C26 | c3 | D02 | d1 | E03 |
| 2-1-m1E03-2-0864 | 853.137 | OEt | a2 | B12 | b3 | C17 | c2 | D02 | d1 | E03 |
| 2-1-m1E03-2-0865 | 853.152 | OEt | a1 | B09 | b2 | C06 | c2 | D02 | d1 | E03 |
| 2-1-m1E03-2-0866 | 853.170 | OEt | a1 | B10 | b2 | C01 | c2 | D02 | d1 | E03 |
| 2-1-m1E03-2-0867 | 853.206 | OEt | a2 | B18 | b3 | C18 | c1 | D01 | d1 | E03 |
| 2-1-m1E03-2-0868 | 854.136 | OEt | a2 | B19 | b3 | C15 | c2 | D02 | d1 | E03 |
| 2-1-m1E03-2-0869 | 854.166 | OEt | a1 | B08 | b2 | C04 | c2 | D02 | d1 | E03 |
| 2-1-m1E03-2-0870 | 854.206 | OEt | a2 | B19 | b3 | C16 | c1 | D01 | d1 | E03 |
| 2-1-m1E03-2-0871 | 855.182 | OEt | a1 | B07 | b1 | C09 | c2 | D02 | d1 | E03 |
| 2-1-m1E03-2-0872 | 855.257 | OEt | a1 | B07 | b1 | C31 | c3 | D02 | d1 | E03 |
| 2-1-m1E03-2-0873 | 856.167 | OEt | a2 | B19 | b3 | C17 | c1 | D01 | d1 | E03 |
| 2-1-m1E03-2-0874 | 856.167 | OEt | a2 | B19 | b1 | C14 | c2 | D02 | d1 | E03 |
| 2-1-m1E03-2-0875 | 856.258 | OEt | a2 | B20 | b1 | C29 | c3 | D02 | d1 | E03 |
| 2-1-m1E03-2-0876 | 857.113 | OEt | a1 | B07 | b3 | C06 | c2 | D02 | d1 | E03 |
| 2-1-m1E03-2-0877 | 858.178 | OEt | a1 | B07 | b3 | C18 | c1 | D01 | d1 | E03 |
| 2-1-m1E03-2-0878 | 859.196 | OEt | a2 | B20 | b2 | C05 | c2 | D02 | d1 | E03 |
| 2-1-m1E03-2-0879 | 859.269 | OEt | a1 | B09 | b1 | C29 | c3 | D02 | d1 | E03 |
| 2-1-m1E03-2-0880 | 860.187 | OEt | a1 | B08 | b1 | C13 | c2 | D02 | d1 | E03 |
| 2-1-m1E03-2-0881 | 860.216 | OEt | a2 | B20 | b2 | C26 | c3 | D02 | d1 | E03 |
| 2-1-m1E03-2-0882 | 860.253 | OEt | a2 | B19 | b2 | C07 | c2 | D02 | d1 | E03 |
| 2-1-m1E03-2-0883 | 861.248 | OEt | a1 | B10 | b1 | C32 | c3 | D02 | d1 | E03 |
| 2-1-m1E03-2-0884 | 862.122 | OEt | a1 | B08 | b3 | C15 | c2 | D02 | d1 | E03 |
| 2-1-m1E03-2-0885 | 862.175 | OEt | a2 | B20 | b2 | C04 | c2 | D02 | d1 | E03 |
| 2-1-m1E03-2-0886 | 862.191 | OEt | a1 | B08 | b3 | C16 | c1 | D01 | d1 | E03 |
| 2-1-m1E03-2-0887 | 862.207 | OEt | a1 | B09 | b2 | C05 | c2 | D02 | d1 | E03 |
| 2-1-m1E03-2-0888 | 863.191 | OEt | a1 | B10 | b2 | C02 | c2 | D02 | d1 | E03 |
| 2-1-m1E03-2-0889 | 863.227 | OEt | a1 | B10 | b2 | C22 | c3 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0890 | 863.227 | OEt | a1 | B09 | b2 | C26 | c3 | D02 | d1 | E03 |
| 2-1-m1E03-2-0891 | 864.153 | OEt | a1 | B08 | b3 | C17 | c1 | D01 | d1 | E03 |
| 2-1-m1E03-2-0892 | 864.153 | OEt | a1 | B08 | b1 | C14 | c2 | D02 | d1 | E03 |
| 2-1-m1E03-2-0893 | 864.157 | OEt | a2 | B19 | b3 | C08 | c2 | D02 | d1 | E03 |
| 2-1-m1E03-2-0894 | 864.186 | OEt | a1 | B10 | b2 | C03 | c2 | D02 | d1 | E03 |
| 2-1-m1E03-2-0895 | 864.196 | OEt | a2 | B13 | b3 | C16 | c2 | D02 | d1 | E03 |
| 2-1-m1E03-2-0896 | 865.186 | OEt | a1 | B09 | b2 | C04 | c2 | D02 | d1 | E03 |
| 2-1-m1E03-2-0897 | 866.157 | OEt | a2 | B13 | b3 | C17 | c2 | D02 | d1 | E03 |
| 2-1-m1E03-2-0898 | 867.152 | OEt | a2 | B11 | b3 | C18 | c2 | D02 | d1 | E03 |
| 2-1-m1E03-2-0899 | 867.202 | OEt | a1 | B10 | b2 | C24 | c3 | D02 | d1 | E03 |
| 2-1-m1E03-2-0900 | 868.148 | OEt | a2 | B12 | b3 | C18 | c2 | D02 | d1 | E03 |
| 2-1-m1E03-2-0901 | 868.171 | OEt | a2 | B14 | b3 | C16 | c2 | D02 | d1 | E03 |
| 2-1-m1E03-2-0902 | 868.182 | OEt | a2 | B19 | b1 | C09 | c2 | D02 | d1 | E03 |
| 2-1-m1E03-2-0903 | 868.196 | OEt | a2 | B20 | b1 | C13 | c2 | D02 | d1 | E03 |
| 2-1-m1E03-2-0904 | 868.238 | OEt | a1 | B08 | b2 | C07 | c2 | D02 | d1 | E03 |
| 2-1-m1E03-2-0905 | 868.258 | OEt | a2 | B19 | b1 | C31 | c3 | D02 | d1 | E03 |
| 2-1-m1E03-2-0906 | 869.147 | OEt | a1 | B10 | b2 | C06 | c2 | D02 | d1 | E03 |
| 2-1-m1E03-2-0907 | 869.186 | OEt | a1 | B01 | b3 | C16 | c2 | D02 | d1 | E03 |
| 2-1-m1E03-2-0908 | 870.113 | OEt | a2 | B19 | b3 | C06 | c2 | D02 | d1 | E03 |
| 2-1-m1E03-2-0909 | 870.131 | OEt | a2 | B20 | b3 | C15 | c2 | D02 | d1 | E03 |
| 2-1-m1E03-2-0910 | 870.132 | OEt | a2 | B14 | b3 | C17 | c2 | D02 | d1 | E03 |
| 2-1-m1E03-2-0911 | 870.181 | OEt | a1 | B02 | b3 | C16 | c2 | D02 | d1 | E03 |
| 2-1-m1E03-2-0912 | 870.200 | OEt | a2 | B20 | b3 | C16 | c1 | D01 | d1 | E03 |
| 2-1-m1E03-2-0913 | 871.147 | OEt | a1 | B01 | b3 | C17 | c2 | D02 | d1 | E03 |
| 2-1-m1E03-2-0914 | 871.178 | OEt | a2 | B19 | b3 | C18 | c1 | D01 | d1 | E03 |
| 2-1-m1E03-2-0915 | 871.207 | OEt | a1 | B09 | b1 | C13 | c2 | D02 | d1 | E03 |
| 2-1-m1E03-2-0916 | 872.143 | OEt | a1 | B02 | b3 | C17 | c2 | D02 | d1 | E03 |
| 2-1-m1E03-2-0917 | 872.143 | OEt | a1 | B08 | b3 | C08 | c2 | D02 | d1 | E03 |
| 2-1-m1E03-2-0918 | 872.162 | OEt | a2 | B20 | b3 | C17 | c1 | D01 | d1 | E03 |
| 2-1-m1E03-2-0919 | 872.162 | OEt | a2 | B20 | b1 | C14 | c2 | D02 | d1 | E03 |
| 2-1-m1E03-2-0920 | 873.142 | OEt | a1 | B09 | b3 | C15 | c2 | D02 | d1 | E03 |
| 2-1-m1E03-2-0921 | 873.211 | OEt | a1 | B09 | b3 | C16 | c1 | D01 | d1 | E03 |
| 2-1-m1E03-2-0922 | 875.173 | OEt | a1 | B09 | b3 | C17 | c1 | D01 | d1 | E03 |
| 2-1-mlE03-2-0923 | 875.173 | OEt | a1 | B09 | b1 | C14 | c2 | D02 | d1 | E03 |
| 2-1-mlE03-2-0924 | 875.263 | OEt | a1 | B10 | b1 | C29 | c3 | D02 | d1 | E03 |
| 2-1-m1E03-2-0925 | 876.168 | OEt | a1 | B08 | b1 | C09 | c2 | D02 | d1 | E03 |
| 2-1-m1E03-2-0926 | 876.243 | OEt | a1 | B08 | b1 | C31 | c3 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0927 | 876.247 | OEt | a2 | B20 | b2 | C07 | c2 | D02 | d1 | E03 |
| 2-1-m1E03-2-0928 | 878.099 | OEt | a1 | B08 | b3 | C06 | c2 | D02 | d1 | E03 |
| 2-1-m1E03-2-0929 | 878.202 | OEt | a1 | B10 | b2 | C05 | c2 | D02 | d1 | E03 |
| 2-1-m1E03-2-0930 | 878.211 | OEt | a2 | B15 | b3 | C16 | c2 | D02 | d1 | E03 |
| 2-1-m1E03-2-0931 | 879.164 | OEt | a1 | B08 | b3 | C18 | c1 | D01 | d1 | E03 |
| 2-1-m1E03-2-0932 | 879.222 | OEt | a1 | B10 | b2 | C26 | c3 | D02 | d1 | E03 |
| 2-1-m1E03-2-0933 | 879.258 | OEt | a1 | B09 | b2 | C07 | c2 | D02 | d1 | E03 |
| 2-1-m1E03-2-0934 | 880.152 | OEt | a2 | B20 | b3 | C08 | c2 | D02 | d1 | E03 |
| 2-1-mlE03-2-0935 | 880.173 | OEt | a2 | B15 | b3 | C17 | c2 | D02 | d1 | E03 |
| 2-1-m1E03-2-0936 | 880.191 | OEt | a2 | B16 | b3 | C16 | c2 | D02 | d1 | E03 |
| 2-1-m1E03-2-0937 | 881.168 | OEt | a2 | B13 | b3 | C18 | c2 | D02 | d1 | E03 |
| 2-1-m1E03-2-0938 | 881.181 | OEt | a1 | B10 | b2 | C04 | c2 | D02 | d1 | E03 |
| 2-1-m1E03-2-0939 | 882.152 | OEt | a2 | B16 | b3 | C17 | c2 | D02 | d1 | E03 |
| 2-1-m1E03-2-0940 | 883.163 | OEt | a1 | B09 | b3 | C08 | c2 | D02 | d1 | E03 |
| 2-1-m1E03-2-0941 | 883.202 | OEt | a1 | B03 | b3 | C16 | c2 | D02 | d1 | E03 |
| 2-1-m1E03-2-0942 | 884.177 | OEt | a2 | B20 | b1 | C09 | c2 | D02 | d1 | E03 |
| 2-1-m1E03-2-0943 | 884.253 | OEt | a2 | B20 | b1 | C31 | c3 | D02 | d1 | E03 |
| 2-1-m1E03-2-0944 | 885.143 | OEt | a2 | B14 | b3 | C18 | c2 | D02 | d1 | E03 |
| 2-1-m1E03-2-0945 | 885.163 | OEt | a1 | B03 | b3 | C17 | c2 | D02 | d1 | E03 |
| 2-1-m1E03-2-0946 | 886.108 | OEt | a2 | B20 | b3 | C06 | c2 | D02 | d1 | E03 |
| 2-1-m1E03-2-0947 | 886.158 | OEt | a1 | B01 | b3 | C18 | c2 | D02 | d1 | E03 |
| 2-1-m1E03-2-0948 | 886.161 | OEt | a2 | B17 | b3 | C16 | c2 | D02 | d1 | E03 |
| 2-1-m1E03-2-0949 | 887.154 | OEt | a1 | B02 | b3 | C18 | c2 | D02 | d1 | E03 |
| 2-1-m1E03-2-0950 | 887.173 | OEt | a2 | B20 | b3 | C18 | c1 | D01 | d1 | E03 |
| 2-1-m1E03-2-0951 | 887.176 | OEt | a1 | B04 | b3 | C16 | c2 | D02 | d1 | E03 |
| 2-1-m1E03-2-0952 | 887.188 | OEt | a1 | B09 | b1 | C09 | c2 | D02 | d1 | E03 |
| 2-1-m1E03-2-0953 | 887.202 | OEt | a1 | B10 | b1 | C13 | c2 | D02 | d1 | E03 |
| 2-1-m1E03-2-0954 | 887.263 | OEt | a1 | B09 | b1 | C31 | c3 | D02 | d1 | E03 |
| 2-1-m1E03-2-0955 | 888.123 | OEt | a2 | B17 | b3 | C17 | c2 | D02 | d1 | E03 |
| 2-1-m1E03-2-0956 | 889.119 | OEt | a1 | B09 | b3 | C06 | c2 | D02 | d1 | E03 |
| 2-1-m1E03-2-0957 | 889.137 | OEt | a1 | B10 | b3 | C15 | c2 | D02 | d1 | E03 |
| 2-1-m1E03-2-0958 | 889.138 | OEt | a1 | B04 | b3 | C17 | c2 | D02 | d1 | E03 |
| 2-1-m1E03-2-0959 | 889.206 | OEt | a1 | B10 | b3 | C16 | c1 | D01 | d1 | E03 |
| 2-1-m1E03-2-0960 | 890.184 | OEt | a1 | B09 | b3 | C18 | c1 | D01 | d1 | E03 |
| 2-1-m1E03-2-0961 | 891.168 | OEt | a1 | B10 | b3 | C17 | c1 | D01 | d1 | E03 |
| 2-1-m1E03-2-0962 | 891.168 | OEt | a1 | B10 | b1 | C14 | c2 | D02 | d1 | E03 |
| 2-1-m1E03-2-0963 | 895.184 | OEt | a2 | B15 | b3 | C18 | c2 | D02 | d1 | E03 |

(continued)

| [Table 8-10-4] Compound that may be contained in mixture 2-1-m1E03-2 | | | | | | | | | | |
|---|---|---|---|---|---|---|---|---|---|---|
| ID | Exact Mass | | a | B | b | C | c | D | d | E |
| 2-1-m1E03-2-0964 | 895.253 | OEt | a1 | B10 | b2 | C07 | c2 | D02 | d1 | E03 |
| 2-1-m1E03-2-0965 | 897.163 | OEt | a2 | B16 | b3 | C18 | c2 | D02 | d1 | E03 |
| 2-1-m1E03-2-0966 | 897.217 | OEt | a1 | B05 | b3 | C16 | c2 | D02 | d1 | E03 |
| 2-1-m1E03-2-0967 | 899.158 | OEt | a1 | B10 | b3 | C08 | c2 | D02 | d1 | E03 |
| 2-1-m1E03-2-0968 | 899.179 | OEt | a1 | B05 | b3 | C17 | c2 | D02 | d1 | E03 |
| 2-1-m1E03-2-0969 | 899.196 | OEt | a1 | B06 | b3 | C16 | c2 | D02 | d1 | E03 |
| 2-1-m1E03-2-0970 | 900.174 | OEt | a1 | B03 | b3 | C18 | c2 | D02 | d1 | E03 |
| 2-1-m1E03-2-0971 | 900.196 | OEt | a2 | B18 | b3 | C16 | c2 | D02 | d1 | E03 |
| 2-1-m1E03-2-0972 | 901.158 | OEt | a1 | B06 | b3 | C17 | c2 | D02 | d1 | E03 |
| 2-1-m1E03-2-0973 | 902.157 | OEt | a2 | B18 | b3 | C17 | c2 | D02 | d1 | E03 |
| 2-1-mIE03-2-0974 | 903.134 | OEt | a2 | B17 | b3 | C18 | c2 | D02 | d1 | E03 |
| 2-1-m1E03-2-0975 | 903.183 | OEt | a1 | B10 | b1 | C09 | c2 | D02 | d1 | E03 |
| 2-1-m1E03-2-0976 | 903.258 | OEt | a1 | B10 | b1 | C31 | c3 | D02 | d1 | E03 |
| 2-1-m1E03-2-0977 | 904.149 | OEt | a1 | B04 | b3 | C18 | c2 | D02 | d1 | E03 |
| 2-1-m1E03-2-0978 | 905.114 | OEt | a1 | B10 | b3 | C06 | c2 | D02 | d1 | E03 |
| 2-1-m1E03-2-0979 | 905.167 | OEt | a1 | B07 | b3 | C16 | c2 | D02 | d1 | E03 |
| 2-1-m1E03-2-0980 | 906.179 | OEt | a1 | B10 | b3 | C18 | c1 | D01 | d1 | E03 |
| 2-1-m1E03-2-0981 | 907.129 | OEt | a1 | B07 | b3 | C17 | c2 | D02 | d1 | E03 |
| 2-1-m1E03-2-0982 | 914.190 | OEt | a1 | B05 | b3 | C18 | c2 | D02 | d1 | E03 |
| 2-1-m1E03-2-0983 | 916.169 | OEt | a1 | B06 | b3 | C18 | c2 | D02 | d1 | E03 |
| 2-1-m1E03-2-0984 | 917.168 | OEt | a2 | B18 | b3 | C18 | c2 | D02 | d1 | E03 |
| 2-1-m1E03-2-0985 | 918.167 | OEt | a2 | B19 | b3 | C16 | c2 | D02 | d1 | E03 |
| 2-1-m1E03-2-0986 | 920.129 | OEt | a2 | B19 | b3 | C17 | c2 | D02 | d1 | E03 |
| 2-1-m1E03-2-0987 | 922.139 | OEt | a1 | B07 | b3 | C18 | c2 | D02 | d1 | E03 |
| 2-1-m1E03-2-0988 | 926.153 | OEt | a1 | B08 | b3 | C16 | c2 | D02 | d1 | E03 |
| 2-1-m1E03-2-0989 | 928.115 | OEt | a1 | B08 | b3 | C17 | c2 | D02 | d1 | E03 |
| 2-1-m1E03-2-0990 | 934.162 | OEt | a2 | B20 | b3 | C16 | c2 | D02 | d1 | E03 |
| 2-1-m1E03-2-0991 | 935.140 | OEt | a2 | B19 | b3 | C18 | c2 | D02 | d1 | E03 |
| 2-1-m1E03-2-0992 | 936.124 | OEt | a2 | B20 | b3 | C17 | c2 | D02 | d1 | E03 |
| 2-1-m1E03-2-0993 | 937.173 | OEt | a1 | B09 | b3 | C16 | c2 | D02 | d1 | E03 |
| 2-1-m1E03-2-0994 | 939.135 | OEt | a1 | B09 | b3 | C17 | c2 | D02 | d1 | E03 |
| 2-1-m1E03-2-0995 | 943.126 | OEt | a1 | B08 | b3 | C18 | c2 | D02 | d1 | E03 |
| 2-1-m1E03-2-0996 | 951.135 | OEt | a2 | B20 | b3 | C18 | c2 | D02 | d1 | E03 |
| 2-1-m1E03-2-0997 | 953.168 | OEt | a1 | B10 | b3 | C16 | c2 | D02 | d1 | E03 |
| 2-1-m1E03-2-0998 | 954.146 | OEt | a1 | B09 | b3 | C18 | c2 | D02 | d1 | E03 |
| 2-1-m1E03-2-0999 | 955.130 | OEt | a1 | B10 | b3 | C17 | c2 | D02 | d1 | E03 |
| 2-1-m1E03-2-1000 | 970.141 | OEt | a1 | B10 | b3 | C18 | c2 | D02 | d1 | E03 |

[Table 405]

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0001 | 781.330 | OEt | a2 | B11 | b1 | C08 | c1 | D01 | d1 | E04 |
| 2-1-m1E04-2-0002 | 782.325 | OEt | a2 | B12 | b1 | C08 | c1 | D01 | d1 | E04 |
| 2-1-m1E04-2-0003 | 782.325 | OEt | a2 | B11 | b1 | C11 | c1 | D01 | d1 | E04 |
| 2-1-m1E04-2-0004 | 783.320 | OEt | a2 | B11 | b1 | C10 | c1 | D01 | d1 | E04 |
| 2-1-m1E04-2-0005 | 783.320 | OEt | a2 | B12 | b1 | C11 | c1 | D01 | d1 | E04 |
| 2-1-m1E04-2-0006 | 784.316 | OEt | a2 | B12 | b1 | C10 | c1 | D01 | d1 | E04 |
| 2-1-m1E04-2-0007 | 788.281 | OEt | a2 | B11 | b1 | C12 | c1 | D01 | d1 | E04 |
| 2-1-m1E04-2-0008 | 789.277 | OEt | a2 | B12 | b1 | C12 | c1 | D01 | d1 | E04 |
| 2-1-m1E04-2-0009 | 795.345 | OEt | a2 | B13 | b1 | C08 | c1 | D01 | d1 | E04 |
| 2-1-m1E04-2-0010 | 796.341 | OEt | a2 | B13 | b1 | C11 | c1 | D01 | d1 | E04 |
| 2-1-m1E04-2-0011 | 797.336 | OEt | a2 | B13 | b1 | C10 | c1 | D01 | d1 | E04 |
| 2-1-m1E04-2-0012 | 799.304 | OEt | a2 | B11 | b2 | C01 | c1 | D01 | d1 | E04 |
| 2-1-m1E04-2-0013 | 799.320 | OEt | a2 | B14 | b1 | C08 | c1 | D01 | d1 | E04 |
| 2-1-m1E04-2-0014 | 800.299 | OEt | a2 | B12 | b2 | C01 | c1 | D01 | d1 | E04 |
| 2-1-m1E04-2-0015 | 800.316 | OEt | a2 | B14 | b1 | C11 | c1 | D01 | d1 | E04 |
| 2-1-m1E04-2-0016 | 800.336 | OEt | a1 | B01 | b1 | C08 | c1 | D01 | d1 | E04 |
| 2-1-m1E04-2-0017 | 801.311 | OEt | a2 | B14 | b1 | C10 | c1 | D01 | d1 | E04 |
| 2-1-m1E04-2-0018 | 801.331 | OEt | a1 | B02 | b1 | C08 | c1 | D01 | d1 | E04 |
| 2-1-m1E04-2-0019 | 801.331 | OEt | a1 | B01 | b1 | C11 | c1 | D01 | d1 | E04 |
| 2-1-m1E04-2-0020 | 802.297 | OEt | a2 | B13 | b1 | C12 | c1 | D01 | d1 | E04 |
| 2-1-m1E04-2-0021 | 802.326 | OEt | a1 | B01 | b1 | C10 | c1 | D01 | d1 | E04 |
| 2-1-m1E04-2-0022 | 802.326 | OEt | a1 | B02 | b1 | C11 | c1 | D01 | d1 | E04 |
| 2-1-m1E04-2-0023 | 803.321 | OEt | a1 | B02 | b1 | C10 | c1 | D01 | d1 | E04 |
| 2-1-m1E04-2-0024 | 806.272 | OEt | a2 | B14 | b1 | C12 | c1 | D01 | d1 | E04 |
| 2-1-m1E04-2-0025 | 807.287 | OEt | a1 | B01 | b1 | C12 | c1 | D01 | d1 | E04 |
| 2-1-m1E04-2-0026 | 808.283 | OEt | a1 | B02 | b1 | C12 | c1 | D01 | d1 | E04 |
| 2-1-m1E04-2-0027 | 809.325 | OEt | a2 | B11 | b2 | C02 | c1 | D01 | d1 | E04 |
| 2-1-m1E04-2-0028 | 809.361 | OEt | a2 | B15 | b1 | C08 | c1 | D01 | d1 | E04 |
| 2-1-m1E04-2-0029 | 810.320 | OEt | a2 | B12 | b2 | C02 | c1 | D01 | d1 | E04 |
| 2-1-m1E04-2-0030 | 810.320 | OEt | a2 | B11 | b2 | C03 | c1 | D01 | d1 | E04 |
| 2-1-m1E04-2-0031 | 810.356 | OEt | a2 | B15 | b1 | C11 | c1 | D01 | d1 | E04 |
| 2-1-m1E04-2-0032 | 811.315 | OEt | a2 | B12 | b2 | C03 | c1 | D01 | d1 | E04 |
| 2-1-m1E04-2-0033 | 811.340 | OEt | a2 | B16 | b1 | C08 | c1 | D01 | d1 | E04 |
| 2-1-m1E04-2-0034 | 811.352 | OEt | a2 | B15 | b1 | C10 | c1 | D01 | d1 | E04 |
| 2-1-m1E04-2-0035 | 812.336 | OEt | a2 | B16 | b1 | C11 | c1 | D01 | d1 | E04 |
| 2-1-m1E04-2-0036 | 813.320 | OEt | a2 | B13 | b2 | C01 | c1 | D01 | d1 | E04 |
| 2-1-m1E04-2-0037 | 813.331 | OEt | a2 | B16 | b1 | C10 | c1 | D01 | d1 | E04 |
| 2-1-m1E04-2-0038 | 814.351 | OEt | a1 | B03 | b1 | C08 | c1 | D01 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0039 | 815.281 | OEt | a2 | B11 | b2 | C06 | c1 | D01 | d1 | E04 |
| 2-1-m1E04-2-0040 | 815.347 | OEt | a1 | B03 | b1 | C11 | c1 | D01 | d1 | E04 |
| 2-1-m1E04-2-0041 | 816.276 | OEt | a2 | B12 | b2 | C06 | c1 | D01 | d1 | E04 |
| 2-1-mlE04-2-0042 | 816.313 | OEt | a2 | B15 | b1 | C12 | c1 | D01 | d1 | E04 |
| 2-1-m1E04-2-0043 | 816.342 | OEt | a1 | B03 | b1 | C10 | c1 | D01 | d1 | E04 |
| 2-1-m1E04-2-0044 | 817.295 | OEt | a2 | B14 | b2 | C01 | c1 | D01 | d1 | E04 |
| 2-1-m1E04-2-0045 | 817.311 | OEt | a2 | B17 | b1 | C08 | c1 | D01 | d1 | E04 |
| 2-1-m1E04-2-0046 | 818.292 | OEt | a2 | B16 | b1 | C12 | c1 | D01 | d1 | E04 |
| 2-1-m1E04-2-0047 | 818.306 | OEt | a2 | B17 | b1 | C11 | c1 | D01 | d1 | E04 |
| 2-1-m1E04-2-0048 | 818.310 | OEt | a1 | B01 | b2 | C01 | c1 | D01 | d1 | E04 |
| 2-1-m1E04-2-0049 | 818.326 | OEt | a1 | B04 | b1 | C08 | c1 | D01 | d1 | E04 |
| 2-1-m1E04-2-0050 | 819.301 | OEt | a2 | B17 | b1 | C10 | c1 | D01 | d1 | E04 |
| 2-1-m1E04-2-0051 | 819.305 | OEt | a1 | B02 | b2 | C01 | c1 | D01 | d1 | E04 |
| 2-1-m1E04-2-0052 | 819.321 | OEt | a1 | B04 | b1 | C11 | c1 | D01 | d1 | E04 |
| 2-1-m1E04-2-0053 | 820.317 | OEt | a1 | B04 | b1 | C10 | c1 | D01 | d1 | E04 |
| 2-1-m1E04-2-0054 | 821.303 | OEt | a1 | B03 | b1 | C12 | c1 | D01 | d1 | E04 |
| 2-1-m1E04-2-0055 | 823.340 | OEt | a2 | B13 | b2 | C02 | c1 | D01 | d1 | E04 |
| 2-1-m1E04-2-0056 | 824.263 | OEt | a2 | B17 | b1 | C12 | c1 | D01 | d1 | E04 |
| 2-1-m1E04-2-0057 | 824.336 | OEt | a2 | B13 | b2 | C03 | c1 | D01 | d1 | E04 |
| 2-1-m1E04-2-0058 | 824.336 | OEt | a2 | B11 | b2 | C05 | c1 | D01 | d1 | E04 |
| 2-1-m1E04-2-0059 | 825.278 | OEt | a1 | B04 | b1 | C12 | c1 | D01 | d1 | E04 |
| 2-1-m1E04-2-0060 | 825.331 | OEt | a2 | B12 | b2 | C05 | c1 | D01 | d1 | E04 |
| 2-1-m1E04-2-0061 | 827.315 | OEt | a2 | B14 | b2 | C02 | c1 | D01 | d1 | E04 |
| 2-1-m1E04-2-0062 | 827.315 | OEt | a2 | B11 | b2 | C04 | c1 | D01 | d1 | E04 |
| 2-1-m1E04-2-0063 | 827.335 | OEt | a2 | B15 | b2 | C01 | c1 | D01 | d1 | E04 |
| 2-1-m1E04-2-0064 | 828.311 | OEt | a2 | B12 | b2 | C04 | c1 | D01 | d1 | E04 |
| 2-1-m1E04-2-0065 | 828.311 | OEt | a2 | B14 | b2 | C03 | c1 | D01 | d1 | E04 |
| 2-1-m1E04-2-0066 | 828.331 | OEt | a1 | B01 | b2 | C02 | c1 | D01 | d1 | E04 |
| 2-1-m1E04-2-0067 | 828.367 | OEt | a1 | B05 | b1 | C08 | c1 | D01 | d1 | E04 |
| 2-1-m1E04-2-0068 | 829.297 | OEt | a2 | B13 | b2 | C06 | c1 | D01 | d1 | E04 |
| 2-1-m1E04-2-0069 | 829.315 | OEt | a2 | B16 | b2 | C01 | c1 | D01 | d1 | E04 |
| 2-1-m1E04-2-0070 | 829.326 | OEt | a1 | B02 | b2 | C02 | c1 | D01 | d1 | E04 |
| 2-1-m1E04-2-0071 | 829.326 | OEt | a1 | B01 | b2 | C03 | c1 | D01 | d1 | E04 |
| 2-1-m1E04-2-0072 | 829.362 | OEt | a1 | B05 | b1 | C11 | c1 | D01 | d1 | E04 |
| 2-1-mlE04-2-0073 | 830.321 | OEt | a1 | B02 | b2 | C03 | c1 | D01 | d1 | E04 |
| 2-1-m1E04-2-0074 | 830.346 | OEt | a1 | B06 | b1 | C08 | c1 | D01 | d1 | E04 |
| 2-1-mlE04-2-0075 | 830.357 | OEt | a1 | B05 | b1 | C10 | c1 | D01 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0076 | 831.341 | OEt | a1 | B06 | b1 | C11 | c1 | D01 | d1 | E04 |
| 2-1-m1E04-2-0077 | 831.345 | OEt | a2 | B18 | b1 | C08 | c1 | D01 | d1 | E04 |
| 2-1-m1E04-2-0078 | 832.325 | OEt | a1 | B03 | b2 | C01 | c1 | D01 | d1 | E04 |
| 2-1-m1E04-2-0079 | 832.337 | OEt | a1 | B06 | b1 | C10 | c1 | D01 | d1 | E04 |
| 2-1-mlE04-2-0080 | 832.341 | OEt | a2 | B18 | b1 | C11 | c1 | D01 | d1 | E04 |
| 2-1-m1E04-2-0081 | 833.272 | OEt | a2 | B14 | b2 | C06 | c1 | D01 | d1 | E04 |
| 2-1-mlE04-2-0082 | 833.336 | OEt | a2 | B18 | b1 | C10 | c1 | D01 | d1 | E04 |
| 2-1-m1E04-2-0083 | 833.336 | OEt | a2 | B11 | b1 | C13 | c1 | D01 | d1 | E04 |
| 2-1-mlE04-2-0084 | 834.287 | OEt | a1 | B01 | b2 | C06 | c1 | D01 | d1 | E04 |
| 2-1-mlE04-2-0085 | 834.331 | OEt | a2 | B12 | b1 | C13 | c1 | D01 | d1 | E04 |
| 2-1-m1E04-2-0086 | 835.271 | OEt | a2 | B11 | b3 | C15 | c1 | D01 | d1 | E04 |
| 2-1-m1E04-2-0087 | 835.282 | OEt | a1 | B02 | b2 | C06 | c1 | D01 | d1 | E04 |
| 2-1-mlE04-2-0088 | 835.285 | OEt | a2 | B17 | b2 | C01 | c1 | D01 | d1 | E04 |
| 2-1-mlE04-2-0089 | 835.319 | OEt | a1 | B05 | b1 | C12 | c1 | D01 | d1 | E04 |
| 2-1-mlE04-2-0090 | 836.266 | OEt | a2 | B12 | b3 | C15 | c1 | D01 | d1 | E04 |
| 2-1-mlE04-2-0091 | 836.300 | OEt | a1 | B04 | b2 | C01 | c1 | D01 | d1 | E04 |
| 2-1-m1E04-2-0092 | 836.317 | OEt | a1 | B07 | b1 | C08 | c1 | D01 | d1 | E04 |
| 2-1-m1E04-2-0093 | 837.298 | OEt | a1 | B06 | b1 | C12 | c1 | D01 | d1 | E04 |
| 2-1-mlE04-2-0094 | 837.302 | OEt | a2 | B11 | b1 | C14 | c1 | D01 | d1 | E04 |
| 2-1-m1E04-2-0095 | 837.312 | OEt | a1 | B07 | b1 | C11 | c1 | D01 | d1 | E04 |
| 2-1-mlE04-2-0096 | 837.323 | OEt | a2 | B11 | b2 | C33 | c3 | D02 | d1 | E04 |
| 2-1-mlE04-2-0097 | 837.356 | OEt | a2 | B15 | b2 | C02 | c1 | D01 | d1 | E04 |
| 2-1-mlE04-2-0098 | 838.297 | OEt | a2 | B18 | b1 | C12 | c1 | D01 | d1 | E04 |
| 2-1-mlE04-2-0099 | 838.297 | OEt | a2 | B12 | b1 | C14 | c1 | D01 | d1 | E04 |
| 2-1-m1E04-2-0100 | 838.307 | OEt | a1 | B07 | b1 | C10 | c1 | D01 | d1 | E04 |
| 2-1-m1E04-2-0101 | 838.318 | OEt | a2 | B12 | b2 | C33 | c3 | D02 | d1 | E04 |
| 2-1-m1E04-2-0102 | 838.351 | OEt | a2 | B15 | b2 | C03 | c1 | D01 | d1 | E04 |
| 2-1-m1E04-2-0103 | 838.351 | OEt | a2 | B13 | b2 | C05 | c1 | D01 | d1 | E04 |
| 2-1-m1E04-2-0104 | 839.335 | OEt | a2 | B16 | b2 | C02 | c1 | D01 | d1 | E04 |
| 2-1-m1E04-2-0105 | 840.331 | OEt | a2 | B16 | b2 | C03 | c1 | D01 | d1 | E04 |
| 2-1-m1E04-2-0106 | 841.331 | OEt | a2 | B13 | b2 | C04 | c1 | D01 | d1 | E04 |
| 2-1-m1E04-2-0107 | 841.387 | OEt | a2 | B11 | b2 | C07 | c1 | D01 | d1 | E04 |
| 2-1-mlE04-2-0108 | 842.326 | OEt | a2 | B14 | b2 | C05 | c1 | D01 | d1 | E04 |
| 2-1-m1E04-2-0109 | 842.346 | OEt | a1 | B03 | b2 | C02 | c1 | D01 | d1 | E04 |
| 2-1-mlE04-2-0110 | 842.383 | OEt | a2 | B12 | b2 | C07 | c1 | D01 | d1 | E04 |
| 2-1-m1E04-2-0111 | 843.268 | OEt | a1 | B07 | b1 | C12 | c1 | D01 | d1 | E04 |
| 2-1-mlE04-2-0112 | 843.312 | OEt | a2 | B15 | b2 | C06 | c1 | D01 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-mlE04-2-0113 | 843.341 | OEt | a1 | B03 | b2 | C03 | c1 | D01 | d1 | E04 |
| 2-1-m1E04-2-0114 | 843.341 | OEt | a1 | B01 | b2 | C05 | c1 | D01 | d1 | E04 |
| 2-1-mlE04-2-0115 | 844.337 | OEt | a1 | B02 | b2 | C05 | c1 | D01 | d1 | E04 |
| 2-1-mlE04-2-0116 | 845.292 | OEt | a2 | B16 | b2 | C06 | c1 | D01 | d1 | E04 |
| 2-1-mlE04-2-0117 | 845.292 | OEt | a2 | B11 | b3 | C08 | c1 | D01 | d1 | E04 |
| 2-1-mlE04-2-0118 | 845.292 | OEt | a2 | B11 | b1 | C08 | c2 | D02 | d1 | E04 |
| 2-1-mlE04-2-0119 | 845.306 | OEt | a2 | B17 | b2 | C02 | c1 | D01 | d1 | E04 |
| 2-1-m1E04-2-0120 | 845.306 | OEt | a2 | B14 | b2 | C04 | c1 | D01 | d1 | E04 |
| 2-1-mlE04-2-0121 | 846.287 | OEt | a2 | B12 | b3 | C08 | c1 | D01 | d1 | E04 |
| 2-1-mlE04-2-0122 | 846.287 | OEt | a2 | B12 | b1 | C08 | c2 | D02 | d1 | E04 |
| 2-1-mlE04-2-0123 | 846.287 | OEt | a2 | B11 | b1 | C11 | c2 | D02 | d1 | E04 |
| 2-1-mlE04-2-0124 | 846.301 | OEt | a2 | B17 | b2 | C03 | c1 | D01 | d1 | E04 |
| 2-1-mlE04-2-0125 | 846.321 | OEt | a1 | B04 | b2 | C02 | c1 | D01 | d1 | E04 |
| 2-1-mlE04-2-0126 | 846.321 | OEt | a1 | B01 | b2 | C04 | c1 | D01 | d1 | E04 |
| 2-1-mlE04-2-0127 | 846.341 | OEt | a1 | B05 | b2 | C01 | c1 | D01 | d1 | E04 |
| 2-1-m1E04-2-0128 | 847.282 | OEt | a2 | B11 | b1 | C10 | c2 | D02 | d1 | E04 |
| 2-1-mlE04-2-0129 | 847.282 | OEt | a2 | B12 | b1 | C11 | c2 | D02 | d1 | E04 |
| 2-1-mlE04-2-0130 | 847.316 | OEt | a1 | B02 | b2 | C04 | c1 | D01 | d1 | E04 |
| 2-1-mlE04-2-0131 | 847.316 | OEt | a1 | B04 | b2 | C03 | c1 | D01 | d1 | E04 |
| 2-1-m1E04-2-0132 | 847.352 | OEt | a2 | B13 | b1 | C13 | c1 | D01 | d1 | E04 |
| 2-1-mlE04-2-0133 | 848.277 | OEt | a2 | B12 | b1 | C10 | c2 | D02 | d1 | E04 |
| 2-1-mlE04-2-0134 | 848.303 | OEt | a1 | B03 | b2 | C06 | c1 | D01 | d1 | E04 |
| 2-1-m1E04-2-0135 | 848.320 | OEt | a1 | B06 | b2 | C01 | c1 | D01 | d1 | E04 |
| 2-1-mlE04-2-0136 | 849.287 | OEt | a2 | B13 | b3 | C15 | c1 | D01 | d1 | E04 |
| 2-1-mlE04-2-0137 | 849.317 | OEt | a2 | B19 | b1 | C08 | c1 | D01 | d1 | E04 |
| 2-1-mlE04-2-0138 | 849.317 | OEt | a2 | B11 | b1 | C09 | c1 | D01 | d1 | E04 |
| 2-1-m1E04-2-0139 | 849.320 | OEt | a2 | B18 | b2 | C01 | c1 | D01 | d1 | E04 |
| 2-1-m1E04-2-0140 | 850.312 | OEt | a2 | B12 | b1 | C09 | c1 | D01 | d1 | E04 |
| 2-1-mlE04-2-0141 | 850.312 | OEt | a2 | B19 | b1 | C11 | c1 | D01 | d1 | E04 |
| 2-1-m1E04-2-0142 | 851.248 | OEt | a2 | B11 | b3 | C06 | c1 | D01 | d1 | E04 |
| 2-1-m1E04-2-0143 | 851.262 | OEt | a2 | B17 | b2 | C06 | c1 | D01 | d1 | E04 |
| 2-1-m1E04-2-0144 | 851.308 | OEt | a2 | B19 | b1 | C10 | c1 | D01 | d1 | E04 |
| 2-1-m1E04-2-0145 | 851.318 | OEt | a2 | B13 | b1 | C14 | c1 | D01 | d1 | E04 |
| 2-1-m1E04-2-0146 | 851.327 | OEt | a2 | B14 | b1 | C13 | c1 | D01 | d1 | E04 |
| 2-1-m1E04-2-0147 | 851.339 | OEt | a2 | B13 | b2 | C33 | c3 | D02 | d1 | E04 |
| 2-1-mlE04-2-0148 | 852.243 | OEt | a2 | B12 | b3 | C06 | c1 | D01 | d1 | E04 |
| 2-1-m1E04-2-0149 | 852.243 | OEt | a2 | B11 | b1 | C12 | c2 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0150 | 852.278 | OEt | a1 | B04 | b2 | C06 | c1 | D01 | d1 | E04 |
| 2-1-m1E04-2-0151 | 852.342 | OEt | a1 | B01 | b1 | C13 | c1 | D01 | d1 | E04 |
| 2-1-m1E04-2-0152 | 852.367 | OEt | a2 | B15 | b2 | C05 | c1 | D01 | d1 | E04 |
| 2-1-m1E04-2-0153 | 853.239 | OEt | a2 | B12 | b1 | C12 | c2 | D02 | d1 | E04 |
| 2-1-mlE04-2-0154 | 853.262 | OEt | a2 | B14 | b3 | C15 | c1 | D01 | d1 | E04 |
| 2-1-m1E04-2-0155 | 853.337 | OEt | a1 | B02 | b1 | C13 | c1 | D01 | d1 | E04 |
| 2-1-m1E04-2-0156 | 854.277 | OEt | a1 | B01 | b3 | C15 | c1 | D01 | d1 | E04 |
| 2-1-m1E04-2-0157 | 854.291 | OEt | a1 | B07 | b2 | C01 | c1 | D01 | d1 | E04 |
| 2-1-m1E04-2-0158 | 854.346 | OEt | a2 | B16 | b2 | C05 | c1 | D01 | d1 | E04 |
| 2-1-mlE04-2-0159 | 855.272 | OEt | a1 | B02 | b3 | C15 | c1 | D01 | d1 | E04 |
| 2-1-m1E04-2-0160 | 855.292 | OEt | a2 | B14 | b1 | C14 | c1 | D01 | d1 | E04 |
| 2-1-mlE04-2-0161 | 855.314 | OEt | a2 | B14 | b2 | C33 | c3 | D02 | d1 | E04 |
| 2-1-m1E04-2-0162 | 855.347 | OEt | a2 | B15 | b2 | C04 | c1 | D01 | d1 | E04 |
| 2-1-m1E04-2-0163 | 855.403 | OEt | a2 | B13 | b2 | C07 | c1 | D01 | d1 | E04 |
| 2-1-mlE04-2-0164 | 856.269 | OEt | a2 | B19 | b1 | C12 | c1 | D01 | d1 | E04 |
| 2-1-m1E04-2-0165 | 856.308 | OEt | a1 | B01 | b1 | C14 | c1 | D01 | d1 | E04 |
| 2-1-m1E04-2-0166 | 856.329 | OEt | a1 | B01 | b2 | C33 | c3 | D02 | d1 | E04 |
| 2-1-mlE04-2-0167 | 856.362 | OEt | a1 | B05 | b2 | C02 | c1 | D01 | d1 | E04 |
| 2-1-mlE04-2-0168 | 857.303 | OEt | a1 | B08 | b1 | C08 | c1 | D01 | d1 | E04 |
| 2-1-m1E04-2-0169 | 857.303 | OEt | a1 | B02 | b1 | C14 | c1 | D01 | d1 | E04 |
| 2-1-mlE04-2-0170 | 857.324 | OEt | a1 | B02 | b2 | C33 | c3 | D02 | d1 | E04 |
| 2-1-mlE04-2-0171 | 857.326 | OEt | a2 | B16 | b2 | C04 | c1 | D01 | d1 | E04 |
| 2-1-mlE04-2-0172 | 857.328 | OEt | a2 | B11 | b1 | C30 | c3 | D02 | d1 | E04 |
| 2-1-mlE04-2-0173 | 857.357 | OEt | a1 | B05 | b2 | C03 | c1 | D01 | d1 | E04 |
| 2-1-mlE04-2-0174 | 857.357 | OEt | a1 | B03 | b2 | COS | c1 | D01 | d1 | E04 |
| 2-1-m1E04-2-0175 | 858.298 | OEt | a1 | B08 | b1 | C11 | c1 | D01 | d1 | E04 |
| 2-1-mlE04-2-0176 | 858.323 | OEt | a2 | B12 | b1 | C30 | c3 | D02 | d1 | E04 |
| 2-1-m1E04-2-0177 | 858.341 | OEt | a1 | B06 | b2 | C02 | c1 | D01 | d1 | E04 |
| 2-1-mlE04-2-0178 | 859.293 | OEt | a1 | B08 | b1 | C10 | c1 | D01 | d1 | E04 |
| 2-1-m1E04-2-0179 | 859.307 | OEt | a2 | B13 | b3 | C08 | c1 | D01 | d1 | E04 |
| 2-1-mlE04-2-0180 | 859.307 | OEt | a2 | B13 | b1 | C08 | c2 | D02 | d1 | E04 |
| 2-1-mlE04-2-0181 | 859.307 | OEt | a2 | B11 | b2 | C20 | c3 | D02 | d1 | E04 |
| 2-1-m1E04-2-0182 | 859.336 | OEt | a1 | B06 | b2 | C03 | c1 | D01 | d1 | E04 |
| 2-1-mlE04-2-0183 | 859.340 | OEt | a2 | B18 | b2 | C02 | c1 | D01 | d1 | E04 |
| 2-1-m1E04-2-0184 | 859.378 | OEt | a2 | B14 | b2 | C07 | c1 | D01 | d1 | E04 |
| 2-1-mlE04-2-0185 | 860.303 | OEt | a2 | B13 | b1 | C11 | c2 | D02 | d1 | E04 |
| 2-1-mlE04-2-0186 | 860.303 | OEt | a2 | B12 | b2 | C20 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-mlE04-2-0187 | 860.303 | OEt | a2 | B11 | b2 | C28 | c3 | D02 | d1 | E04 |
| 2-1-m1E04-2-0188 | 860.317 | OEt | a2 | B17 | b2 | C05 | c1 | D01 | d1 | E04 |
| 2-1-m1E04-2-0189 | 860.336 | OEt | a2 | B18 | b2 | C03 | c1 | D01 | d1 | E04 |
| 2-1-m1E04-2-0190 | 860.337 | OEt | a1 | B03 | b2 | C04 | c1 | D01 | d1 | E04 |
| 2-1-mlE04-2-0191 | 860.393 | OEt | a1 | B01 | b2 | C07 | c1 | D01 | d1 | E04 |
| 2-1-m1E04-2-0192 | 861.298 | OEt | a2 | B13 | b1 | C10 | c2 | D02 | d1 | E04 |
| 2-1-m1E04-2-0193 | 861.298 | OEt | a2 | B12 | b2 | C28 | c3 | D02 | d1 | E04 |
| 2-1-m1E04-2-0194 | 861.332 | OEt | a1 | B04 | b2 | C05 | c1 | D01 | d1 | E04 |
| 2-1-m1E04-2-0195 | 861.367 | OEt | a2 | B15 | b1 | C13 | c1 | D01 | d1 | E04 |
| 2-1-m1E04-2-0196 | 861.388 | OEt | a1 | B02 | b2 | C07 | c1 | D01 | d1 | E04 |
| 2-1-mlE04-2-0197 | 862.318 | OEt | a1 | B05 | b2 | C06 | c1 | D01 | d1 | E04 |
| 2-1-m1E04-2-0198 | 863.266 | OEt | a2 | B11 | b2 | C01 | c2 | D02 | d1 | E04 |
| 2-1-mlE04-2-0199 | 863.282 | OEt | a2 | B14 | b3 | C08 | c1 | D01 | d1 | E04 |
| 2-1-m1E04-2-0200 | 863.282 | OEt | a2 | B14 | b1 | C08 | c2 | D02 | d1 | E04 |
| 2-1-m1E04-2-0201 | 863.296 | OEt | a2 | B17 | b2 | C04 | c1 | D01 | d1 | E04 |
| 2-1-m1E04-2-0202 | 863.302 | OEt | a2 | B15 | b3 | C15 | c1 | D01 | d1 | E04 |
| 2-1-m1E04-2-0203 | 863.333 | OEt | a2 | B13 | b1 | C09 | c1 | D01 | d1 | E04 |
| 2-1-m1E04-2-0204 | 863.347 | OEt | a2 | B16 | b1 | C13 | c1 | D01 | d1 | E04 |
| 2-1-m1E04-2-0205 | 864.255 | OEt | a1 | B08 | b1 | C12 | c1 | D01 | d1 | E04 |
| 2-1-m1E04-2-0206 | 864.261 | OEt | a2 | B12 | b2 | C01 | c2 | D02 | d1 | E04 |
| 2-1-m1E04-2-0207 | 864.278 | OEt | a2 | B14 | b1 | C11 | c2 | D02 | d1 | E04 |
| 2-1-m1E04-2-0208 | 864.298 | OEt | a1 | B06 | b2 | C06 | c1 | D01 | d1 | E04 |
| 2-1-m1E04-2-0209 | 864.298 | OEt | a1 | B01 | b3 | C08 | c1 | D01 | d1 | E04 |
| 2-1-m1E04-2-0210 | 864.298 | OEt | a1 | B01 | b1 | C08 | c2 | D02 | d1 | E04 |
| 2-1-mlE04-2-0211 | 864.312 | OEt | a1 | B07 | b2 | C02 | c1 | D01 | d1 | E04 |
| 2-1-m1E04-2-0212 | 864.312 | OEt | a1 | B04 | b2 | C04 | c1 | D01 | d1 | E04 |
| 2-1-m1E04-2-0213 | 865.264 | OEt | a2 | B13 | b3 | C06 | c1 | D01 | d1 | E04 |
| 2-1-m1E04-2-0214 | 865.273 | OEt | a2 | B14 | b1 | C10 | c2 | D02 | d1 | E04 |
| 2-1-mlE04-2-0215 | 865.282 | OEt | a2 | B16 | b3 | C15 | c1 | D01 | d1 | E04 |
| 2-1-mlE04-2-0216 | 865.293 | OEt | a1 | B02 | b3 | C08 | c1 | D01 | d1 | E04 |
| 2-1-mlE04-2-0217 | 865.293 | OEt | a1 | B02 | b1 | C08 | c2 | D02 | d1 | E04 |
| 2-1-mlE04-2-0218 | 865.293 | OEt | a1 | B01 | b1 | C11 | c2 | D02 | d1 | E04 |
| 2-1-m1E04-2-0219 | 865.297 | OEt | a2 | B18 | b2 | C06 | c1 | D01 | d1 | E04 |
| 2-1-mlE04-2-0220 | 865.307 | OEt | a1 | B07 | b2 | C03 | c1 | D01 | d1 | E04 |
| 2-1-mlE04-2-0221 | 865.312 | OEt | a2 | B20 | b1 | C08 | c1 | D01 | d1 | E04 |
| 2-1-m1E04-2-0222 | 865.333 | OEt | a2 | B15 | b1 | C14 | c1 | D01 | d1 | E04 |
| 2-1-m1E04-2-0223 | 865.354 | OEt | a2 | B15 | b2 | C33 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0224 | 866.259 | OEt | a2 | B13 | b1 | C12 | c2 | D02 | d1 | E04 |
| 2-1-m1E04-2-0225 | 866.288 | OEt | a1 | B01 | b1 | C10 | c2 | D02 | d1 | E04 |
| 2-1-m1E04-2-0226 | 866.288 | OEt | a1 | B02 | b1 | C11 | c2 | D02 | d1 | E04 |
| 2-1-m1E04-2-0227 | 866.307 | OEt | a2 | B20 | b1 | C11 | c1 | D01 | d1 | E04 |
| 2-1-m1E04-2-0228 | 866.357 | OEt | a1 | B03 | b1 | C13 | c1 | D01 | d1 | E04 |
| 2-1-m1E04-2-0229 | 867.283 | OEt | a1 | B02 | b1 | C10 | c2 | D02 | d1 | E04 |
| 2-1-m1E04-2-0230 | 867.291 | OEt | a2 | B19 | b2 | C01 | c1 | D01 | d1 | E04 |
| 2-1-m1E04-2-0231 | 867.303 | OEt | a2 | B20 | b1 | C10 | c1 | D01 | d1 | E04 |
| 2-1-m1E04-2-0232 | 867.308 | OEt | a2 | B14 | b1 | C09 | c1 | D01 | d1 | E04 |
| 2-1-m1E04-2-0233 | 867.312 | OEt | a2 | B16 | b1 | C14 | c1 | D01 | d1 | E04 |
| 2-1-m1E04-2-0234 | 867.334 | OEt | a2 | B16 | b2 | C33 | c3 | D02 | d1 | E04 |
| 2-1-m1E04-2-0235 | 868.292 | OEt | a1 | B03 | b3 | C15 | c1 | D01 | d1 | E04 |
| 2-1-m1E04-2-0236 | 868.323 | OEt | a1 | B09 | b1 | C08 | c1 | D01 | d1 | E04 |
| 2-1-m1E04-2-0237 | 868.323 | OEt | a1 | B01 | b1 | C09 | c1 | D01 | d1 | E04 |
| 2-1-m1E04-2-0238 | 869.239 | OEt | a2 | B14 | b3 | C06 | c1 | D01 | d1 | E04 |
| 2-1-m1E04-2-0239 | 869.317 | OEt | a2 | B17 | b1 | C13 | c1 | D01 | d1 | E04 |
| 2-1-m1E04-2-0240 | 869.318 | OEt | a1 | B02 | b1 | C09 | c1 | D01 | d1 | E04 |
| 2-1-m1E04-2-0241 | 869.318 | OEt | a1 | B09 | b1 | C11 | c1 | D01 | d1 | E04 |
| 2-1-m1E04-2-0242 | 869.419 | OEt | a2 | B15 | b2 | C07 | c1 | D01 | d1 | E04 |
| 2-1-m1E04-2-0243 | 870.234 | OEt | a2 | B14 | b1 | C12 | c2 | D02 | d1 | E04 |
| 2-1-m1E04-2-0244 | 870.254 | OEt | a1 | B01 | b3 | C06 | c1 | D01 | d1 | E04 |
| 2-1-m1E04-2-0245 | 870.268 | OEt | a1 | B07 | b2 | C06 | c1 | D01 | d1 | E04 |
| 2-1-m1E04-2-0246 | 870.313 | OEt | a1 | B09 | b1 | C10 | c1 | D01 | d1 | E04 |
| 2-1-m1E04-2-0247 | 870.323 | OEt | a1 | B03 | b1 | C14 | c1 | D01 | d1 | E04 |
| 2-1-mlE04-2-0248 | 870.332 | OEt | a1 | B04 | b1 | C13 | c1 | D01 | d1 | E04 |
| 2-1-mlE04-2-0249 | 870.344 | OEt | a1 | B03 | b2 | C33 | c3 | D02 | d1 | E04 |
| 2-1-m1E04-2-0250 | 871.249 | OEt | a1 | B02 | b3 | C06 | c1 | D01 | d1 | E04 |
| 2-1-m1E04-2-0251 | 871.249 | OEt | a1 | B01 | b1 | C12 | c2 | D02 | d1 | E04 |
| 2-1-m1E04-2-0252 | 871.252 | OEt | a2 | B17 | b3 | C15 | c1 | D01 | d1 | E04 |
| 2-1-m1E04-2-0253 | 871.344 | OEt | a2 | B13 | b1 | C30 | c3 | D02 | d1 | E04 |
| 2-1-m1E04-2-0254 | 871.344 | OEt | a2 | B11 | b1 | C32 | c3 | D02 | d1 | E04 |
| 2-1-m1E04-2-0255 | 871.373 | OEt | a1 | B05 | b2 | C05 | c1 | D01 | d1 | E04 |
| 2-1-m1E04-2-0256 | 871.398 | OEt | a2 | B16 | b2 | C07 | c1 | D01 | d1 | E04 |
| 2-1-m1E04-2-0257 | 872.244 | OEt | a1 | B02 | b1 | C12 | c2 | D02 | d1 | E04 |
| 2-1-m1E04-2-0258 | 872.264 | OEt | a2 | B20 | b1 | C12 | c1 | D01 | d1 | E04 |
| 2-1-mlE04-2-0259 | 872.267 | OEt | a1 | B04 | b3 | C15 | c1 | D01 | d1 | E04 |
| 2-1-m1E04-2-0260 | 872.339 | OEt | a2 | B12 | b1 | C32 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0261 | 873.283 | OEt | a2 | B17 | b1 | C14 | c1 | D01 | d1 | E04 |
| 2-1-mlE04-2-0262 | 873.287 | OEt | a2 | B11 | b2 | C02 | c2 | D02 | d1 | E04 |
| 2-1-m1E04-2-0263 | 873.304 | OEt | a2 | B17 | b2 | C33 | c3 | D02 | d1 | E04 |
| 2-1-mlE04-2-0264 | 873.323 | OEt | a2 | B15 | b3 | C08 | c1 | D01 | d1 | E04 |
| 2-1-m1E04-2-0265 | 873.323 | OEt | a2 | B15 | b1 | C08 | c2 | D02 | d1 | E04 |
| 2-1-mlE04-2-0266 | 873.323 | OEt | a2 | B13 | b2 | C20 | c3 | D02 | d1 | E04 |
| 2-1-mlE04-2-0267 | 873.323 | OEt | a2 | B11 | b2 | C22 | c3 | D02 | d1 | E04 |
| 2-1-mlE04-2-0268 | 873.352 | OEt | a1 | B06 | b2 | C05 | c1 | D01 | d1 | E04 |
| 2-1-mlE04-2-0269 | 874.282 | OEt | a2 | B12 | b2 | C02 | c2 | D02 | d1 | E04 |
| 2-1-m1E04-2-0270 | 874.282 | OEt | a2 | B11 | b2 | C03 | c2 | D02 | d1 | E04 |
| 2-1-m1E04-2-0271 | 874.298 | OEt | a1 | B04 | b1 | C14 | c1 | D01 | d1 | E04 |
| 2-1-mlE04-2-0272 | 874.318 | OEt | a2 | B15 | b1 | C11 | c2 | D02 | d1 | E04 |
| 2-1-m1E04-2-0273 | 874.318 | OEt | a2 | B12 | b2 | C22 | c3 | D02 | d1 | E04 |
| 2-1-mlE04-2-0274 | 874.318 | OEt | a2 | B13 | b2 | C28 | c3 | D02 | d1 | E04 |
| 2-1-m1E04-2-0275 | 874.319 | OEt | a1 | B04 | b2 | C33 | c3 | D02 | d1 | E04 |
| 2-1-mlE04-2-0276 | 874.351 | OEt | a2 | B18 | b2 | C05 | c1 | D01 | d1 | E04 |
| 2-1-mlE04-2-0277 | 874.352 | OEt | a1 | B05 | b2 | C04 | c1 | D01 | d1 | E04 |
| 2-1-mlE04-2-0278 | 874.409 | OEt | a1 | B03 | b2 | C07 | c1 | D01 | d1 | E04 |
| 2-1-mlE04-2-0279 | 875.275 | OEt | a1 | B09 | b1 | C12 | c1 | D01 | d1 | E04 |
| 2-1-mlE04-2-0280 | 875.277 | OEt | a1 | B08 | b2 | C01 | c1 | D01 | d1 | E04 |
| 2-1-m1E04-2-0281 | 875.277 | OEt | a2 | B12 | b2 | C03 | c2 | D02 | d1 | E04 |
| 2-1-mlE04-2-0282 | 875.302 | OEt | a2 | B16 | b3 | C08 | c1 | D01 | d1 | E04 |
| 2-1-mlE04-2-0283 | 875.302 | OEt | a2 | B16 | b1 | C08 | c2 | D02 | d1 | E04 |
| 2-1-mlE04-2-0284 | 875.313 | OEt | a2 | B15 | b1 | C10 | c2 | D02 | d1 | E04 |
| 2-1-mlE04-2-0285 | 875.319 | OEt | a2 | B14 | b1 | C30 | c3 | D02 | d1 | E04 |
| 2-1-mlE04-2-0286 | 876.298 | OEt | a2 | B16 | b1 | C11 | c2 | D02 | d1 | E04 |
| 2-1-mlE04-2-0287 | 876.332 | OEt | a1 | B06 | b2 | C04 | c1 | D01 | d1 | E04 |
| 2-1-mlE04-2-0288 | 876.334 | OEt | a1 | B01 | b1 | C30 | c3 | D02 | d1 | E04 |
| 2-1-mlE04-2-0289 | 877.282 | OEt | a2 | B13 | b2 | C01 | c2 | D02 | d1 | E04 |
| 2-1-m1E04-2-0290 | 877.293 | OEt | a2 | B16 | b1 | C10 | c2 | D02 | d1 | E04 |
| 2-1-mlE04-2-0291 | 877.298 | OEt | a2 | B14 | b2 | C20 | c3 | D02 | d1 | E04 |
| 2-1-mlE04-2-0292 | 877.298 | OEt | a2 | B11 | b2 | C24 | c3 | D02 | d1 | E04 |
| 2-1-mlE04-2-0293 | 877.312 | OEt | a2 | B19 | b2 | C02 | c1 | D01 | d1 | E04 |
| 2-1-mlE04-2-0294 | 877.329 | OEt | a1 | B02 | b1 | C30 | c3 | D02 | d1 | E04 |
| 2-1-mlE04-2-0295 | 877.331 | OEt | a2 | B18 | b2 | C04 | c1 | D01 | d1 | E04 |
| 2-1-mlE04-2-0296 | 877.348 | OEt | a2 | B15 | b1 | C09 | c1 | D01 | d1 | E04 |
| 2-1-mlE04-2-0297 | 877.368 | OEt | a2 | B17 | b2 | C07 | c1 | D01 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-mlE04-2-0298 | 878.293 | OEt | a2 | B12 | b2 | C24 | c3 | D02 | d1 | E04 |
| 2-1-mlE04-2-0299 | 878.293 | OEt | a2 | B14 | b2 | C28 | c3 | D02 | d1 | E04 |
| 2-1-m1E04-2-0300 | 878.307 | OEt | a2 | B19 | b2 | C03 | c1 | D01 | d1 | E04 |
| 2-1-m1E04-2-0301 | 878.313 | OEt | a1 | B03 | b3 | C08 | c1 | D01 | d1 | E04 |
| 2-1-m1E04-2-0302 | 878.313 | OEt | a1 | B03 | b1 | C08 | c2 | D02 | d1 | E04 |
| 2-1-m1E04-2-0303 | 878.313 | OEt | a1 | B01 | b2 | C20 | c3 | D02 | d1 | E04 |
| 2-1-m1E04-2-0304 | 878.384 | OEt | a1 | B04 | b2 | C07 | c1 | D01 | d1 | E04 |
| 2-1-mlE04-2-0305 | 879.243 | OEt | a2 | B11 | b2 | C06 | c2 | D02 | d1 | E04 |
| 2-1-mlE04-2-0306 | 879.279 | OEt | a2 | B15 | b3 | C06 | c1 | D01 | d1 | E04 |
| 2-1-m1E04-2-0307 | 879.308 | OEt | a1 | B03 | b1 | C11 | c2 | D02 | d1 | E04 |
| 2-1-m1E04-2-0308 | 879.308 | OEt | a1 | B02 | b2 | C20 | c3 | D02 | d1 | E04 |
| 2-1-m1E04-2-0309 | 879.308 | OEt | a1 | B01 | b2 | C28 | c3 | D02 | d1 | E04 |
| 2-1-m1E04-2-0310 | 879.323 | OEt | a1 | B07 | b2 | C05 | c1 | D01 | d1 | E04 |
| 2-1-m1E04-2-0311 | 879.328 | OEt | a2 | B16 | b1 | C09 | c1 | D01 | d1 | E04 |
| 2-1-m1E04-2-0312 | 880.238 | OEt | a2 | B12 | b2 | C06 | c2 | D02 | d1 | E04 |
| 2-1-m1E04-2-0313 | 880.275 | OEt | a2 | B15 | b1 | C12 | c2 | D02 | d1 | E04 |
| 2-1-mlE04-2-0314 | 880.304 | OEt | a1 | B03 | b1 | C10 | c2 | D02 | d1 | E04 |
| 2-1-m1E04-2-0315 | 880.304 | OEt | a1 | B02 | b2 | C28 | c3 | D02 | d1 | E04 |
| 2-1-mlE04-2-0316 | 880.373 | OEt | a1 | B05 | b1 | C13 | c1 | D01 | d1 | E04 |
| 2-1-mlE04-2-0317 | 881.256 | OEt | a2 | B14 | b2 | C01 | c2 | D02 | d1 | E04 |
| 2-1-mlE04-2-0318 | 881.259 | OEt | a2 | B16 | b3 | C06 | c1 | D01 | d1 | E04 |
| 2-1-mlE04-2-0319 | 881.273 | OEt | a2 | B17 | b3 | C08 | c1 | D01 | d1 | E04 |
| 2-1-m1E04-2-0320 | 881.273 | OEt | a2 | B17 | b1 | C08 | c2 | D02 | d1 | E04 |
| 2-1-m1E04-2-0321 | 882.254 | OEt | a2 | B16 | b1 | C12 | c2 | D02 | d1 | E04 |
| 2-1-m1E04-2-0322 | 882.268 | OEt | a2 | B17 | b1 | C11 | c2 | D02 | d1 | E04 |
| 2-1-m1E04-2-0323 | 882.272 | OEt | a1 | B01 | b2 | C01 | c2 | D02 | d1 | E04 |
| 2-1-m1E04-2-0324 | 882.288 | OEt | a1 | B04 | b3 | C08 | c1 | D01 | d1 | E04 |
| 2-1-m1E04-2-0325 | 882.288 | OEt | a1 | B04 | b1 | C08 | c2 | D02 | d1 | E04 |
| 2-1-m1E04-2-0326 | 882.302 | OEt | a1 | B07 | b2 | C04 | c1 | D01 | d1 | E04 |
| 2-1-m1E04-2-0327 | 882.308 | OEt | a1 | B05 | b3 | C15 | c1 | D01 | d1 | E04 |
| 2-1-m1E04-2-0328 | 882.339 | OEt | a1 | B03 | b1 | C09 | c1 | D01 | d1 | E04 |
| 2-1-m1E04-2-0329 | 882.352 | OEt | a1 | B06 | b1 | C13 | c1 | D01 | d1 | E04 |
| 2-1-m1E04-2-0330 | 883.263 | OEt | a2 | B17 | b1 | C10 | c2 | D02 | d1 | E04 |
| 2-1-m1E04-2-0331 | 883.267 | OEt | a1 | B02 | b2 | C01 | c2 | D02 | d1 | E04 |
| 2-1-m1E04-2-0332 | 883.269 | OEt | a2 | B19 | b2 | C06 | c1 | D01 | d1 | E04 |
| 2-1-m1E04-2-0333 | 883.283 | OEt | a1 | B04 | b1 | C11 | c2 | D02 | d1 | E04 |
| 2-1-m1E04-2-0334 | 883.286 | OEt | a2 | B20 | b2 | C01 | c1 | D01 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0335 | 883.352 | OEt | a2 | B18 | b1 | C13 | c1 | D01 | d1 | E04 |
| 2-1-m1E04-2-0336 | 884.270 | OEt | a1 | B03 | b3 | C06 | c1 | D01 | d1 | E04 |
| 2-1-m1E04-2-0337 | 884.279 | OEt | a1 | B04 | b1 | C10 | c2 | D02 | d1 | E04 |
| 2-1-m1E04-2-0338 | 884.287 | OEt | a1 | B06 | b3 | C15 | c1 | D01 | d1 | E04 |
| 2-1-m1E04-2-0339 | 884.318 | OEt | a1 | B10 | b1 | C08 | c1 | D01 | d1 | E04 |
| 2-1-m1E04-2-0340 | 884.339 | OEt | a1 | B05 | b1 | C14 | c1 | D01 | d1 | E04 |
| 2-1-m1E04-2-0341 | 884.360 | OEt | a1 | B05 | b2 | C33 | c3 | D02 | d1 | E04 |
| 2-1-m1E04-2-0342 | 885.265 | OEt | a1 | B03 | b1 | C12 | c2 | D02 | d1 | E04 |
| 2-1-m1E04-2-0343 | 885.287 | OEt | a2 | B18 | b3 | C15 | c1 | D01 | d1 | E04 |
| 2-1-mlE04-2-0344 | 885.298 | OEt | a1 | B08 | b2 | C02 | c1 | D01 | d1 | E04 |
| 2-1-m1E04-2-0345 | 885.298 | OEt | a2 | B17 | b1 | C09 | c1 | D01 | d1 | E04 |
| 2-1-m1E04-2-0346 | 885.313 | OEt | a1 | B10 | b1 | C11 | c1 | D01 | d1 | E04 |
| 2-1-m1E04-2-0347 | 885.359 | OEt | a2 | B15 | b1 | C30 | c3 | D02 | d1 | E04 |
| 2-1-m1E04-2-0348 | 885.359 | OEt | a2 | B11 | b1 | C29 | c3 | D02 | d1 | E04 |
| 2-1-m1E04-2-0349 | 885.359 | OEt | a2 | B13 | b1 | C32 | c3 | D02 | d1 | E04 |
| 2-1-m1E04-2-0350 | 886.293 | OEt | a1 | B08 | b2 | C03 | c1 | D01 | d1 | E04 |
| 2-1-m1E04-2-0351 | 886.297 | OEt | a1 | B09 | b2 | C01 | c1 | D01 | d1 | E04 |
| 2-1-mlE04-2-0352 | 886.308 | OEt | a1 | B10 | b1 | C10 | c1 | D01 | d1 | E04 |
| 2-1-mlE04-2-0353 | 886.314 | OEt | a1 | B04 | b1 | C09 | c1 | D01 | d1 | E04 |
| 2-1-mlE04-2-0354 | 886.318 | OEt | a1 | B06 | b1 | C14 | c1 | D01 | d1 | E04 |
| 2-1-m 1 E04-2-0355 | 886.339 | OEt | a1 | B06 | b2 | C33 | c3 | D02 | d1 | E04 |
| 2-1-m1E04-2-0356 | 886.355 | OEt | a2 | B12 | b1 | C29 | c3 | D02 | d1 | E04 |
| 2-1-m1E04-2-0357 | 887.229 | OEt | a2 | B17 | b3 | C06 | c1 | D01 | d1 | E04 |
| 2-1-m1E04-2-0358 | 887.302 | OEt | a2 | B13 | b2 | C02 | c2 | D02 | d1 | E04 |
| 2-1-m1E04-2-0359 | 887.318 | OEt | a2 | B18 | b1 | C14 | c1 | D01 | d1 | E04 |
| 2-1-m1E04-2-0360 | 887.339 | OEt | a2 | B15 | b2 | C20 | c3 | D02 | d1 | E04 |
| 2-1-mlE04-2-0361 | 887.339 | OEt | a2 | B13 | b2 | C22 | c3 | D02 | d1 | E04 |
| 2-1-mlE04-2-0362 | 887.339 | OEt | a2 | B16 | b1 | C30 | c3 | D02 | d1 | E04 |
| 2-1-m1E04-2-0363 | 887.339 | OEt | a2 | B18 | b2 | C33 | c3 | D02 | d1 | E04 |
| 2-1-m1E04-2-0364 | 888.225 | OEt | a2 | B17 | b1 | C12 | c2 | D02 | d1 | E04 |
| 2-1-m1E04-2-0365 | 888.245 | OEt | a1 | B04 | b3 | C06 | c1 | D01 | d1 | E04 |
| 2-1-m1E04-2-0366 | 888.298 | OEt | a2 | B13 | b2 | C03 | c2 | D02 | d1 | E04 |
| 2-1-m1E04-2-0367 | 888.298 | OEt | a2 | B11 | b2 | C05 | c2 | D02 | d1 | E04 |
| 2-1-m1E04-2-0368 | 888.323 | OEt | a1 | B07 | b1 | C13 | c1 | D01 | d1 | E04 |
| 2-1-m1E04-2-0369 | 888.334 | OEt | a2 | B15 | b2 | C28 | c3 | D02 | d1 | E04 |
| 2-1-m1E04-2-0370 | 888.424 | OEt | a1 | B05 | b2 | C07 | c1 | D01 | d1 | E04 |
| 2-1-m1E04-2-0371 | 889.240 | OEt | a1 | B04 | b1 | C12 | c2 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0372 | 889.293 | OEt | a2 | B12 | b2 | C05 | c2 | D02 | d1 | E04 |
| 2-1-m1E04-2-0373 | 889.318 | OEt | a2 | B16 | b2 | C20 | c3 | D02 | d1 | E04 |
| 2-1-m1E04-2-0374 | 889.318 | OEt | a2 | B11 | b2 | C26 | c3 | D02 | d1 | E04 |
| 2-1-m1E04-2-0375 | 889.334 | OEt | a2 | B14 | b1 | C32 | c3 | D02 | d1 | E04 |
| 2-1-m1E04-2-0376 | 890.258 | OEt | a1 | B07 | b3 | C15 | c1 | D01 | d1 | E04 |
| 2-1-m1E04-2-0377 | 890.313 | OEt | a2 | B12 | b2 | C26 | c3 | D02 | d1 | E04 |
| 2-1-m1E04-2-0378 | 890.313 | OEt | a2 | B16 | b2 | C28 | c3 | D02 | d1 | E04 |
| 2-1-m1E04-2-0379 | 890.350 | OEt | a1 | B03 | b1 | C30 | c3 | D02 | d1 | E04 |
| 2-1-m1E04-2-0380 | 890.350 | OEt | a1 | B01 | b1 | C32 | c3 | D02 | d1 | E04 |
| 2-1-m1E04-2-0381 | 890.404 | OEt | a1 | B06 | b2 | C07 | c1 | D01 | d1 | E04 |
| 2-1-m1E04-2-0382 | 891.254 | OEt | a1 | B08 | b2 | C06 | c1 | D01 | d1 | E04 |
| 2-1-m1E04-2-0383 | 891.270 | OEt | a1 | B10 | b1 | C12 | c1 | D01 | d1 | E04 |
| 2-1-m1E04-2-0384 | 891.277 | OEt | a2 | B14 | b2 | C02 | c2 | D02 | d1 | E04 |
| 2-1-m1E04-2-0385 | 891.277 | OEt | a2 | B11 | b2 | C04 | c2 | D02 | d1 | E04 |
| 2-1-m1E04-2-0386 | 891.297 | OEt | a2 | B15 | b2 | C01 | c2 | D02 | d1 | E04 |
| 2-1-m1E04-2-0387 | 891.314 | OEt | a2 | B14 | b2 | C22 | c3 | D02 | d1 | E04 |
| 2-1-m1E04-2-0388 | 891.314 | OEt | a2 | B13 | b2 | C24 | c3 | D02 | d1 | E04 |
| 2-1-m1E04-2-0389 | 891.345 | OEt | a1 | B02 | b1 | C32 | c3 | D02 | d1 | E04 |
| 2-1-m1E04-2-0390 | 891.403 | OEt | a2 | B18 | b2 | C07 | c1 | D01 | d1 | E04 |
| 2-1-m1E04-2-0391 | 892.272 | OEt | a2 | B12 | b2 | C04 | c2 | D02 | d1 | E04 |
| 2-1-m1E04-2-0392 | 892.272 | OEt | a2 | B14 | b2 | C03 | c2 | D02 | d1 | E04 |
| 2-1-m1E04-2-0393 | 892.289 | OEt | a1 | B07 | b1 | C14 | c1 | D01 | d1 | E04 |
| 2-1-m1E04-2-0394 | 892.292 | OEt | a1 | B01 | b2 | C02 | c2 | D02 | d1 | E04 |
| 2-1-m1E04-2-0395 | 892.310 | OEt | a1 | B07 | b2 | C33 | c3 | D02 | d1 | E04 |
| 2-1-m1E04-2-0396 | 892.323 | OEt | a2 | B19 | b2 | C05 | c1 | D01 | d1 | E04 |
| 2-1-m1E04-2-0397 | 892.329 | OEt | a1 | B05 | b3 | C08 | c1 | D01 | d1 | E04 |
| 2-1-m1E04-2-0398 | 892.329 | OEt | a1 | B05 | b1 | C08 | c2 | D02 | d1 | E04 |
| 2-1-m1E04-2-0399 | 892.329 | OEt | a1 | B03 | b2 | C20 | c3 | D02 | d1 | E04 |
| 2-1-m1E04-2-0400 | 892.329 | OEt | a1 | B01 | b2 | C22 | c3 | D02 | d1 | E04 |
| 2-1-m1E04-2-0401 | 893.259 | OEt | a2 | B13 | b2 | C06 | c2 | D02 | d1 | E04 |
| 2-1-m1E04-2-0402 | 893.276 | OEt | a2 | B16 | b2 | C01 | c2 | D02 | d1 | E04 |
| 2-1-m1E04-2-0403 | 893.288 | OEt | a1 | B02 | b2 | C02 | c2 | D02 | d1 | E04 |
| 2-1-m1E04-2-0404 | 893.288 | OEt | a1 | B01 | b2 | C03 | c2 | D02 | d1 | E04 |
| 2-1-m1E04-2-0405 | 893.307 | OEt | a2 | B20 | b2 | C02 | c1 | D01 | d1 | E04 |
| 2-1-m1E04-2-0406 | 893.309 | OEt | a2 | B17 | b1 | C30 | c3 | D02 | d1 | E04 |
| 2-1-m1E04-2-0407 | 893.324 | OEt | a1 | B05 | b1 | C11 | c2 | D02 | d1 | E04 |
| 2-1-m1E04-2-0408 | 893.324 | OEt | a1 | B02 | b2 | C22 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0409 | 893.324 | OEt | a1 | B03 | b2 | C28 | c3 | D02 | d1 | E04 |
| 2-1-m1E04-2-0410 | 894.283 | OEt | a1 | B02 | b2 | C03 | c2 | D02 | d1 | E04 |
| 2-1-m1E04-2-0411 | 894.302 | OEt | a2 | B20 | b2 | C03 | c1 | D01 | d1 | E04 |
| 2-1-m1E04-2-0412 | 894.308 | OEt | a1 | B06 | b3 | C08 | c1 | D01 | d1 | E04 |
| 2-1-m1E04-2-0413 | 894.308 | OEt | a1 | B06 | b1 | C08 | c2 | D02 | d1 | E04 |
| 2-1-m1E04-2-0414 | 894.319 | OEt | a1 | B05 | b1 | C10 | c2 | D02 | d1 | E04 |
| 2-1-m1E04-2-0415 | 894.324 | OEt | a1 | B04 | b1 | C30 | c3 | D02 | d1 | E04 |
| 2-1-m1E04-2-0416 | 895.288 | OEt | a2 | B17 | b2 | C20 | c3 | D02 | d1 | E04 |
| 2-1-m1E04-2-0417 | 895.288 | OEt | a2 | B14 | b2 | C24 | c3 | D02 | d1 | E04 |
| 2-1-mlE04-2-0418 | 895.303 | OEt | a2 | B19 | b2 | C04 | c1 | D01 | d1 | E04 |
| 2-1-m1E04-2-0419 | 895.303 | OEt | a1 | B06 | b1 | C11 | c2 | D02 | d1 | E04 |
| 2-1-m1E04-2-0420 | 895.307 | OEt | a2 | B18 | b3 | C08 | c1 | D01 | d1 | E04 |
| 2-1-m1E04-2-0421 | 895.307 | OEt | a2 | B18 | b1 | C08 | c2 | D02 | d1 | E04 |
| 2-1-m1E04-2-0422 | 896.284 | OEt | a2 | B17 | b2 | C28 | c3 | D02 | d1 | E04 |
| 2-1-m1E04-2-0423 | 896.287 | OEt | a1 | B03 | b2 | C01 | c2 | D02 | d1 | E04 |
| 2-1-m1E04-2-0424 | 896.299 | OEt | a1 | B06 | b1 | C10 | c2 | D02 | d1 | E04 |
| 2-1-m1E04-2-0425 | 896.303 | OEt | a2 | B18 | b1 | C11 | c2 | D02 | d1 | E04 |
| 2-1-m1E04-2-0426 | 896.304 | OEt | a1 | B04 | b2 | C20 | c3 | D02 | d1 | E04 |
| 2-1-mlE04-2-0427 | 896.304 | OEt | a1 | B01 | b2 | C24 | c3 | D02 | d1 | E04 |
| 2-1-m1E04-2-0428 | 896.318 | OEt | a1 | B09 | b2 | C02 | c1 | D01 | d1 | E04 |
| 2-1-m1E04-2-0429 | 896.354 | OEt | a1 | B05 | b1 | C09 | c1 | D01 | d1 | E04 |
| 2-1-m1E04-2-0430 | 896.374 | OEt | a1 | B07 | b2 | C07 | c1 | D01 | d1 | E04 |
| 2-1-m1E04-2-0431 | 897.234 | OEt | a2 | B14 | b2 | C06 | c2 | D02 | d1 | E04 |
| 2-1-m1E04-2-0432 | 897.298 | OEt | a2 | B18 | b1 | C10 | c2 | D02 | d1 | E04 |
| 2-1-m1E04-2-0433 | 897.298 | OEt | a2 | B11 | b1 | C13 | c2 | D02 | d1 | E04 |
| 2-1-m1E04-2-0434 | 897.299 | OEt | a1 | B02 | b2 | C24 | c3 | D02 | d1 | E04 |
| 2-1-m1E04-2-0435 | 897.299 | OEt | a1 | B04 | b2 | C28 | c3 | D02 | d1 | E04 |
| 2-1-mlE04-2-0436 | 897.313 | OEt | a1 | B09 | b2 | C03 | c1 | D01 | d1 | E04 |
| 2-1-m1E04-2-0437 | 898.249 | OEt | a1 | B01 | b2 | C06 | c2 | D02 | d1 | E04 |
| 2-1-m1E04-2-0438 | 898.285 | OEt | a1 | B05 | b3 | C06 | c1 | D01 | d1 | E04 |
| 2-1-m1E04-2-0439 | 898.293 | OEt | a2 | B12 | b1 | C13 | c2 | D02 | d1 | E04 |
| 2-1-m1E04-2-0440 | 898.334 | OEt | a1 | B06 | b1 | C09 | c1 | D01 | d1 | E04 |
| 2-1-m1E04-2-0441 | 899.233 | OEt | a2 | B11 | b3 | C15 | c2 | D02 | d1 | E04 |
| 2-1-m1E04-2-0442 | 899.244 | OEt | a1 | B02 | b2 | C06 | c2 | D02 | d1 | E04 |
| 2-1-m1E04-2-0443 | 899.247 | OEt | a2 | B17 | b2 | C01 | c2 | D02 | d1 | E04 |
| 2-1-m1E04-2-0444 | 899.263 | OEt | a2 | B20 | b2 | C06 | c1 | D01 | d1 | E04 |
| 2-1-m1E04-2-0445 | 899.280 | OEt | a1 | B05 | b1 | C12 | c2 | D02 | d1 | E04 |

(continued)

[Table 8-10-5] Compound that may be contained in mixture 2-1-m1E04-2

| ID | Exact Mass | × | a | B | b | C | c | D | d | E |
|---|---|---|---|---|---|---|---|---|---|---|
| 2-1-m1E04-2-0446 | 899.302 | OEt | a2 | B11 | b3 | C16 | c1 | D01 | d1 | E04 |
| 2-1-m1E04-2-0447 | 899.333 | OEt | a2 | B18 | b1 | C09 | c1 | D01 | d1 | E04 |
| 2-1-mlE04-2-0448 | 899.375 | OEt | a2 | B13 | b1 | C29 | c3 | D02 | d1 | E04 |
| 2-1-m1E04-2-0449 | 899.375 | OEt | a2 | B15 | b1 | C32 | c3 | D02 | d1 | E04 |
| 2-1-m1E04-2-0450 | 900.228 | OEt | a2 | B12 | b3 | C15 | c2 | D02 | d1 | E04 |
| 2-1-m1E04-2-0451 | 900.262 | OEt | a1 | B04 | b2 | C01 | c2 | D02 | d1 | E04 |
| 2-1-m1E04-2-0452 | 900.264 | OEt | a1 | B06 | b3 | C06 | c1 | D01 | d1 | E04 |
| 2-1-m1E04-2-0453 | 900.279 | OEt | a1 | B07 | b3 | C08 | c1 | D01 | d1 | E04 |
| 2-1-m1E04-2-0454 | 900.279 | OEt | a1 | B07 | b1 | C08 | c2 | D02 | d1 | E04 |
| 2-1-m1E04-2-0455 | 900.298 | OEt | a2 | B12 | b3 | C16 | c1 | D01 | d1 | E04 |
| 2-1-m1E04-2-0456 | 900.309 | OEt | a1 | B08 | b2 | C05 | c1 | D01 | d1 | E04 |
| 2-1-m1E04-2-0457 | 901.260 | OEt | a1 | B06 | b1 | C12 | c2 | D02 | d1 | E04 |
| 2-1-m1E04-2-0458 | 901.264 | OEt | a2 | B18 | b3 | C06 | c1 | D01 | d1 | E04 |
| 2-1-m1E04-2-0459 | 901.264 | OEt | a2 | B11 | b3 | C17 | c1 | D01 | d1 | E04 |
| 2-1-m1E04-2-0460 | 901.264 | OEt | a2 | B11 | b1 | C14 | c2 | D02 | d1 | E04 |
| 2-1-m1E04-2-0461 | 901.274 | OEt | a1 | B07 | b1 | C11 | c2 | D02 | d1 | E04 |
| 2-1-m1E04-2-0462 | 901.318 | OEt | a2 | B15 | b2 | C02 | c2 | D02 | d1 | E04 |
| 2-1-m1E04-2-0463 | 901.323 | OEt | a2 | B19 | b1 | C13 | c1 | D01 | d1 | E04 |
| 2-1-m1E04-2-0464 | 901.354 | OEt | a2 | B15 | b2 | C22 | c3 | D02 | d1 | E04 |
| 2-1-m1E04-2-0465 | 901.354 | OEt | a2 | B16 | b1 | C32 | c3 | D02 | d1 | E04 |
| 2-1-m1E04-2-0466 | 902.259 | OEt | a2 | B12 | b3 | C17 | c1 | D01 | d1 | E04 |
| 2-1-m1E04-2-0467 | 902.259 | OEt | a2 | B18 | b1 | C12 | c2 | D02 | d1 | E04 |
| 2-1-m1E04-2-0468 | 902.259 | OEt | a2 | B12 | b1 | C14 | c2 | D02 | d1 | E04 |
| 2-1-m1E04-2-0469 | 902.269 | OEt | a1 | B07 | b1 | C10 | c2 | D02 | d1 | E04 |
| 2-1-m1E04-2-0470 | 902.274 | OEt | a1 | B09 | b2 | C06 | c1 | D01 | d1 | E04 |
| 2-1-m1E04-2-0471 | 902.292 | OEt | a1 | B10 | b2 | C01 | c1 | D01 | d1 | E04 |
| 2-1-m1E04-2-0472 | 902.313 | OEt | a2 | B15 | b2 | C03 | c2 | D02 | d1 | E04 |
| 2-1-m1E04-2-0473 | 902.313 | OEt | a2 | B13 | b2 | C05 | c2 | D02 | d1 | E04 |
| 2-1-m1E04-2-0474 | 903.258 | OEt | a2 | B19 | b3 | C15 | c1 | D01 | d1 | E04 |
| 2-1-m1E04-2-0475 | 903.288 | OEt | a1 | B08 | b2 | C04 | c1 | D01 | d1 | E04 |
| 2-1-m1E04-2-0476 | 903.297 | OEt | a2 | B16 | b2 | C02 | c2 | D02 | d1 | E04 |
| 2-1-m1E04-2-0477 | 903.334 | OEt | a2 | B16 | b2 | C22 | c3 | D02 | d1 | E04 |
| 2-1-m1E04-2-0478 | 903.334 | OEt | a2 | B13 | b2 | C26 | c3 | D02 | d1 | E04 |
| 2-1-m1E04-2-0479 | 903.350 | OEt | a2 | B14 | b1 | C29 | c3 | D02 | d1 | E04 |
| 2-1-m1E04-2-0480 | 904.292 | OEt | a2 | B16 | b2 | C03 | c2 | D02 | d1 | E04 |
| 2-1-m1E04-2-0481 | 904.304 | OEt | a1 | B07 | b1 | C09 | c1 | D01 | d1 | E04 |
| 2-1-m1E04-2-0482 | 904.365 | OEt | a1 | B05 | b1 | C30 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0483 | 904.365 | OEt | a1 | B01 | b1 | C29 | c3 | D02 | d1 | E04 |
| 2-1-m1E04-2-0484 | 904.365 | OEt | a1 | B03 | b1 | C32 | c3 | D02 | d1 | E04 |
| 2-1-m1E04-2-0485 | 905.289 | OEt | a2 | B19 | b1 | C14 | c1 | D01 | d1 | E04 |
| 2-1-mlE04-2-0486 | 905.293 | OEt | a2 | B13 | b2 | C04 | c2 | D02 | d1 | E04 |
| 2-1-m1E04-2-0487 | 905.310 | OEt | a2 | B19 | b2 | C33 | c3 | D02 | d1 | E04 |
| 2-1-m1E04-2-0488 | 905.329 | OEt | a2 | B15 | b2 | C24 | c3 | D02 | d1 | E04 |
| 2-1-mlE04-2-0489 | 905.349 | OEt | a2 | B11 | b2 | C07 | c2 | D02 | d1 | E04 |
| 2-1-m1E04-2-0490 | 905.360 | OEt | a1 | B02 | b1 | C29 | c3 | D02 | d1 | E04 |
| 2-1-m1E04-2-0491 | 906.235 | OEt | a1 | B07 | b3 | C06 | c1 | D01 | d1 | E04 |
| 2-1-m1E04-2-0492 | 906.288 | OEt | a2 | B14 | b2 | C05 | c2 | D02 | d1 | E04 |
| 2-1-m1E04-2-0493 | 906.308 | OEt | a1 | B03 | b2 | C02 | c2 | D02 | d1 | E04 |
| 2-1-m1E04-2-0494 | 906.344 | OEt | a2 | B12 | b2 | C07 | c2 | D02 | d1 | E04 |
| 2-1-m1E04-2-0495 | 906.344 | OEt | a1 | B05 | b2 | C20 | c3 | D02 | d1 | E04 |
| 2-1-m 1 E04-2-0496 | 906.344 | OEt | a1 | B03 | b2 | C22 | c3 | D02 | d1 | E04 |
| 2-1-m1E04-2-0497 | 906.344 | OEt | a1 | B06 | b1 | C30 | c3 | D02 | d1 | E04 |
| 2-1-m1E04-2-0498 | 907.230 | OEt | a1 | B07 | b1 | C12 | c2 | D02 | d1 | E04 |
| 2-1-m1E04-2-0499 | 907.274 | OEt | a2 | B15 | b2 | C06 | c2 | D02 | d1 | E04 |
| 2-1-m1E04-2-0500 | 907.303 | OEt | a1 | B03 | b2 | C03 | c2 | D02 | d1 | E04 |
| 2-1-m1E04-2-0501 | 907.303 | OEt | a1 | B01 | b2 | C05 | c2 | D02 | d1 | E04 |
| 2-1-m1E04-2-0502 | 907.308 | OEt | a2 | B16 | b2 | C24 | c3 | D02 | d1 | E04 |
| 2-1-m1E04-2-0503 | 907.308 | OEt | a2 | B14 | b2 | C26 | c3 | D02 | d1 | E04 |
| 2-1-m1E04-2-0504 | 907.325 | OEt | a2 | B17 | b1 | C32 | c3 | D02 | d1 | E04 |
| 2-1-m1E04-2-0505 | 907.340 | OEt | a1 | B05 | b2 | C28 | c3 | D02 | d1 | E04 |
| 2-1-m1E04-2-0506 | 907.344 | OEt | a2 | B18 | b1 | C30 | c3 | D02 | d1 | E04 |
| 2-1-m1E04-2-0507 | 908.299 | OEt | a1 | B02 | b2 | C05 | c2 | D02 | d1 | E04 |
| 2-1-m1E04-2-0508 | 908.318 | OEt | a2 | B20 | b2 | C05 | c1 | D01 | d1 | E04 |
| 2-1-m1E04-2-0509 | 908.324 | OEt | a1 | B06 | b2 | C20 | c3 | D02 | d1 | E04 |
| 2-1-m1E04-2-0510 | 908.324 | OEt | a1 | B01 | b2 | C26 | c3 | D02 | d1 | E04 |
| 2-1-m1E04-2-0511 | 908.340 | OEt | a1 | B04 | b1 | C32 | c3 | D02 | d1 | E04 |
| 2-1-m1E04-2-0512 | 909.254 | OEt | a2 | B16 | b2 | C06 | c2 | D02 | d1 | E04 |
| 2-1-m1E04-2-0513 | 909.254 | OEt | a2 | B11 | b3 | C08 | c2 | D02 | d1 | E04 |
| 2-1-m1E04-2-0514 | 909.268 | OEt | a2 | B17 | b2 | C02 | c2 | D02 | d1 | E04 |
| 2-1-m1E04-2-0515 | 909.268 | OEt | a2 | B14 | b2 | C04 | c2 | D02 | d1 | E04 |
| 2-1-m1E04-2-0516 | 909.304 | OEt | a2 | B17 | b2 | C22 | c3 | D02 | d1 | E04 |
| 2-1-m1E04-2-0517 | 909.309 | OEt | a1 | B08 | b1 | C13 | c1 | D01 | d1 | E04 |
| 2-1-m1E04-2-0518 | 909.319 | OEt | a1 | B02 | b2 | C26 | c3 | D02 | d1 | E04 |
| 2-1-m1E04-2-0519 | 909.319 | OEt | a1 | B06 | b2 | C28 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m 1 E04-2-0520 | 909.323 | OEt | a2 | B18 | b2 | C20 | c3 | D02 | d1 | E04 |
| 2-1-m1E04-2-0521 | 909.375 | OEt | a2 | B19 | b2 | C07 | c1 | D01 | d1 | E04 |
| 2-1-m1E04-2-0522 | 910.249 | OEt | a2 | B12 | b3 | C08 | c2 | D02 | d1 | E04 |
| 2-1-m1E04-2-0523 | 910.263 | OEt | a2 | B17 | b2 | C03 | c2 | D02 | d1 | E04 |
| 2-1-m1E04-2-0524 | 910.283 | OEt | a1 | B04 | b2 | C02 | c2 | D02 | d1 | E04 |
| 2-1-m1E04-2-0525 | 910.283 | OEt | a1 | B01 | b2 | C04 | c2 | D02 | d1 | E04 |
| 2-1-m1E04-2-0526 | 910.303 | OEt | a1 | B05 | b2 | C01 | c2 | D02 | d1 | E04 |
| 2-1-m1E04-2-0527 | 910.318 | OEt | a2 | B18 | b2 | C28 | c3 | D02 | d1 | E04 |
| 2-1-m1E04-2-0528 | 910.319 | OEt | a1 | B04 | b2 | C22 | c3 | D02 | d1 | E04 |
| 2-1-m1E04-2-0529 | 910.319 | OEt | a1 | B03 | b2 | C24 | c3 | D02 | d1 | E04 |
| 2-1-m1E04-2-0530 | 911.244 | OEt | a1 | B08 | b3 | C15 | c1 | D01 | d1 | E04 |
| 2-1-m1E04-2-0531 | 911.278 | OEt | a1 | B02 | b2 | C04 | c2 | D02 | d1 | E04 |
| 2-1-m1E04-2-0532 | 911.278 | OEt | a1 | B04 | b2 | C03 | c2 | D02 | d1 | E04 |
| 2-1-m1E04-2-0533 | 911.298 | OEt | a2 | B20 | b2 | C04 | c1 | D01 | d1 | E04 |
| 2-1-m1E04-2-0534 | 911.313 | OEt | a2 | B13 | b1 | C13 | c2 | D02 | d1 | E04 |
| 2-1-m1E04-2-0535 | 911.329 | OEt | a1 | B09 | b2 | C05 | c1 | D01 | d1 | E04 |
| 2-1-m1E04-2-0536 | 912.264 | OEt | a1 | B03 | b2 | C06 | c2 | D02 | d1 | E04 |
| 2-1-m1E04-2-0537 | 912.282 | OEt | a1 | B06 | b2 | C01 | c2 | D02 | d1 | E04 |
| 2-1-m1E04-2-0538 | 912.313 | OEt | a1 | B10 | b2 | C02 | c1 | D01 | d1 | E04 |
| 2-1-m1E04-2-0539 | 912.315 | OEt | a1 | B07 | b1 | C30 | c3 | D02 | d1 | E04 |
| 2-1-m1E04-2-0540 | 913.249 | OEt | a2 | B13 | b3 | C15 | c2 | D02 | d1 | E04 |
| 2-1-m1E04-2-0541 | 913.275 | OEt | a1 | B08 | b1 | C14 | c1 | D01 | d1 | E04 |
| 2-1-m1E04-2-0542 | 913.279 | OEt | a2 | B19 | b3 | C08 | c1 | D01 | d1 | E04 |
| 2-1-m1E04-2-0543 | 913.279 | OEt | a2 | B19 | b1 | C08 | c2 | D02 | d1 | E04 |
| 2-1-m1E04-2-0544 | 913.279 | OEt | a2 | B11 | b1 | C09 | c2 | D02 | d1 | E04 |
| 2-1-m1E04-2-0545 | 913.279 | OEt | a2 | B17 | b2 | C24 | c3 | D02 | d1 | E04 |
| 2-1-m1E04-2-0546 | 913.282 | OEt | a2 | B18 | b2 | C01 | c2 | D02 | d1 | E04 |
| 2-1-m1E04-2-0547 | 913.296 | OEt | a1 | B08 | b2 | C33 | c3 | D02 | d1 | E04 |
| 2-1-m1E04-2-0548 | 913.308 | OEt | a1 | B10 | b2 | C03 | c1 | D01 | d1 | E04 |
| 2-1-m1E04-2-0549 | 913.318 | OEt | a2 | B13 | b3 | C16 | c1 | D01 | d1 | E04 |
| 2-1-m1E04-2-0550 | 913.354 | OEt | a2 | B11 | b1 | C31 | c3 | D02 | d1 | E04 |
| 2-1-m1E04-2-0551 | 913.391 | OEt | a2 | B15 | b1 | C29 | c3 | D02 | d1 | E04 |
| 2-1-m1E04-2-0552 | 914.274 | OEt | a2 | B12 | b1 | C09 | c2 | D02 | d1 | E04 |
| 2-1-m1E04-2-0553 | 914.274 | OEt | a2 | B19 | b1 | C11 | c2 | D02 | d1 | E04 |
| 2-1-m1E04-2-0554 | 914.294 | OEt | a1 | B07 | b2 | C20 | c3 | D02 | d1 | E04 |
| 2-1-m1E04-2-0555 | 914.294 | OEt | a1 | B04 | b2 | C24 | c3 | D02 | d1 | E04 |
| 2-1-m1E04-2-0556 | 914.308 | OEt | a1 | B09 | b2 | C04 | c1 | D01 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0557 | 914.350 | OEt | a2 | B12 | b1 | C31 | c3 | D02 | d1 | E04 |
| 2-1-m1E04-2-0558 | 915.210 | OEt | a2 | B11 | b3 | C06 | c2 | D02 | d1 | E04 |
| 2-1-m1E04-2-0559 | 915.224 | OEt | a2 | B17 | b2 | C06 | c2 | D02 | d1 | E04 |
| 2-1-m1E04-2-0560 | 915.270 | OEt | a2 | B19 | b1 | C10 | c2 | D02 | d1 | E04 |
| 2-1-m1E04-2-0561 | 915.279 | OEt | a2 | B13 | b3 | C17 | c1 | D01 | d1 | E04 |
| 2-1-m1E04-2-0562 | 915.279 | OEt | a2 | B13 | b1 | C14 | c2 | D02 | d1 | E04 |
| 2-1-m1E04-2-0563 | 915.288 | OEt | a2 | B14 | b1 | C13 | c2 | D02 | d1 | E04 |
| 2-1-m1E04-2-0564 | 915.290 | OEt | a1 | B07 | b2 | C28 | c3 | D02 | d1 | E04 |
| 2-1-m1E04-2-0565 | 915.370 | OEt | a2 | B16 | b1 | C29 | c3 | D02 | d1 | E04 |
| 2-1-m1E04-2-0566 | 916.205 | OEt | a2 | B12 | b3 | C06 | c2 | D02 | d1 | E04 |
| 2-1-m1E04-2-0567 | 916.239 | OEt | a1 | B04 | b2 | C06 | c2 | D02 | d1 | E04 |
| 2-1-m1E04-2-0568 | 916.275 | OEt | a2 | B11 | b3 | C18 | c1 | D01 | d1 | E04 |
| 2-1-m1E04-2-0569 | 916.304 | OEt | a1 | B01 | b1 | C13 | c2 | D02 | d1 | E04 |
| 2-1-m1E04-2-0570 | 916.329 | OEt | a2 | B15 | b2 | C05 | c2 | D02 | d1 | E04 |
| 2-1-m1E04-2-0571 | 917.223 | OEt | a2 | B14 | b3 | C15 | c2 | D02 | d1 | E04 |
| 2-1-m1E04-2-0572 | 917.270 | OEt | a2 | B12 | b3 | C18 | c1 | D01 | d1 | E04 |
| 2-1-m1E04-2-0573 | 917.293 | OEt | a2 | B14 | b3 | C16 | c1 | D01 | d1 | E04 |
| 2-1-m1E04-2-0574 | 917.299 | OEt | a1 | B02 | b1 | C13 | c2 | D02 | d1 | E04 |
| 2-1-m1E04-2-0575 | 917.305 | OEt | a2 | B19 | b1 | C09 | c1 | D01 | d1 | E04 |
| 2-1-m 1 E04-2-0576 | 917.318 | OEt | a2 | B20 | b1 | C13 | c1 | D01 | d1 | E04 |
| 2-1-m1E04-2-0577 | 917.349 | OEt | a2 | B15 | b2 | C26 | c3 | D02 | d1 | E04 |
| 2-1-m 1 E04-2-0578 | 917.360 | OEt | a1 | B08 | b2 | C07 | c1 | D01 | d1 | E04 |
| 2-1-m 1 E04-2-0579 | 918.239 | OEt | a1 | B01 | b3 | C15 | c2 | D02 | d1 | E04 |
| 2-1-m1E04-2-0580 | 918.253 | OEt | a1 | B07 | b2 | C01 | c2 | D02 | d1 | E04 |
| 2-1-m1E04-2-0581 | 918.269 | OEt | a1 | B10 | b2 | C06 | c1 | D01 | d1 | E04 |
| 2-1-m1E04-2-0582 | 918.308 | OEt | a1 | B01 | b3 | C16 | c1 | D01 | d1 | E04 |
| 2-1-m1E04-2-0583 | 918.308 | OEt | a2 | B16 | b2 | C05 | c2 | D02 | d1 | E04 |
| 2-1-m1E04-2-0584 | 918.381 | OEt | a1 | B03 | b1 | C29 | c3 | D02 | d1 | E04 |
| 2-1-m1E04-2-0585 | 918.381 | OEt | a1 | B05 | b1 | C32 | c3 | D02 | d1 | E04 |
| 2-1-m1E04-2-0586 | 919.234 | OEt | a1 | B02 | b3 | C15 | c2 | D02 | d1 | E04 |
| 2-1-m1E04-2-0587 | 919.235 | OEt | a2 | B19 | b3 | C06 | c1 | D01 | d1 | E04 |
| 2-1-m1E04-2-0588 | 919.253 | OEt | a2 | B20 | b3 | C15 | c1 | D01 | d1 | E04 |
| 2-1-m1E04-2-0589 | 919.254 | OEt | a2 | B14 | b3 | C17 | c1 | D01 | d1 | E04 |
| 2-1-m1E04-2-0590 | 919.254 | OEt | a2 | B14 | b1 | C14 | c2 | D02 | d1 | E04 |
| 2-1-m1E04-2-0591 | 919.303 | OEt | a1 | B02 | b3 | C16 | c1 | D01 | d1 | E04 |
| 2-1-m1E04-2-0592 | 919.308 | OEt | a2 | B15 | b2 | C04 | c2 | D02 | d1 | E04 |
| 2-1-m1E04-2-0593 | 919.328 | OEt | a2 | B16 | b2 | C26 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0594 | 919.365 | OEt | a2 | B13 | b2 | C07 | c2 | D02 | d1 | E04 |
| 2-1-m1E04-2-0595 | 920.231 | OEt | a2 | B19 | b1 | C12 | c2 | D02 | d1 | E04 |
| 2-1-m1E04-2-0596 | 920.270 | OEt | a1 | B01 | b3 | C17 | c1 | D01 | d1 | E04 |
| 2-1-mlE04-2-0597 | 920.270 | OEt | a1 | B01 | b1 | C14 | c2 | D02 | d1 | E04 |
| 2-1-m1E04-2-0598 | 920.324 | OEt | a1 | B05 | b2 | C02 | c2 | D02 | d1 | E04 |
| 2-1-m1E04-2-0599 | 920.329 | OEt | a1 | B09 | b1 | C13 | c1 | D01 | d1 | E04 |
| 2-1-m1E04-2-0600 | 920.360 | OEt | a1 | B05 | b2 | C22 | c3 | D02 | d1 | E04 |
| 2-1-m1E04-2-0601 | 920.360 | OEt | a1 | B06 | b1 | C32 | c3 | D02 | d1 | E04 |
| 2-1-m1E04-2-0602 | 921.265 | OEt | a1 | B02 | b3 | C17 | c1 | D01 | d1 | E04 |
| 2-1-m1E04-2-0603 | 921.265 | OEt | a1 | B08 | b3 | C08 | c1 | D01 | d1 | E04 |
| 2-1-m1E04-2-0604 | 921.265 | OEt | a1 | B08 | b1 | C08 | c2 | D02 | d1 | E04 |
| 2-1-m1E04-2-0605 | 921.265 | OEt | a1 | B02 | b1 | C14 | c2 | D02 | d1 | E04 |
| 2-1-m1E04-2-0606 | 921.284 | OEt | a2 | B20 | b1 | C14 | c1 | D01 | d1 | E04 |
| 2-1-m1E04-2-0607 | 921.288 | OEt | a2 | B16 | b2 | C04 | c2 | D02 | d1 | E04 |
| 2-1-m1E04-2-0608 | 921.305 | OEt | a2 | B20 | b2 | C33 | c3 | D02 | d1 | E04 |
| 2-1-m1E04-2-0609 | 921.319 | OEt | a1 | B05 | b2 | C03 | c2 | D02 | d1 | E04 |
| 2-1-m1E04-2-0610 | 921.319 | OEt | a1 | B03 | b2 | C05 | c2 | D02 | d1 | E04 |
| 2-1-m1E04-2-0611 | 921.341 | OEt | a2 | B17 | b1 | C29 | c3 | D02 | d1 | E04 |
| 2-1-m1E04-2-0612 | 921.359 | OEt | a2 | B18 | b1 | C32 | c3 | D02 | d1 | E04 |
| 2-1-m1E04-2-0613 | 922.260 | OEt | a1 | B08 | b1 | C11 | c2 | D02 | d1 | E04 |
| 2-1-m1E04-2-0614 | 922.264 | OEt | a1 | B09 | b3 | C15 | c1 | D01 | d1 | E04 |
| 2-1-m1E04-2-0615 | 922.303 | OEt | a1 | B06 | b2 | C02 | c2 | D02 | d1 | E04 |
| 2-1-m1E04-2-0616 | 922.339 | OEt | a1 | B06 | b2 | C22 | c3 | D02 | d1 | E04 |
| 2-1-m1E04-2-0617 | 922.339 | OEt | a1 | B03 | b2 | C26 | c3 | D02 | d1 | E04 |
| 2-1-m1E04-2-0618 | 922.356 | OEt | a1 | B04 | b1 | C29 | c3 | D02 | d1 | E04 |
| 2-1-m1E04-2-0619 | 923.255 | OEt | a1 | B08 | b1 | C10 | c2 | D02 | d1 | E04 |
| 2-1-m1E04-2-0620 | 923.269 | OEt | a2 | B13 | b3 | C08 | c2 | D02 | d1 | E04 |
| 2-1-m1E04-2-0621 | 923.298 | OEt | a1 | B06 | b2 | C03 | c2 | D02 | d1 | E04 |
| 2-1-m1E04-2-0622 | 923.302 | OEt | a2 | B18 | b2 | C02 | c2 | D02 | d1 | E04 |
| 2-1-m1E04-2-0623 | 923.339 | OEt | a2 | B18 | b2 | C22 | c3 | D02 | d1 | E04 |
| 2-1-m1E04-2-0624 | 923.340 | OEt | a2 | B14 | b2 | C07 | c2 | D02 | d1 | E04 |
| 2-1-m1E04-2-0625 | 924.279 | OEt | a2 | B17 | b2 | C05 | c2 | D02 | d1 | E04 |
| 2-1-m1E04-2-0626 | 924.295 | OEt | a1 | B09 | b1 | C14 | c1 | D01 | d1 | E04 |
| 2-1-m1E04-2-0627 | 924.298 | OEt | a2 | B18 | b2 | C03 | c2 | D02 | d1 | E04 |
| 2-1-m1E04-2-0628 | 924.299 | OEt | a1 | B03 | b2 | C04 | c2 | D02 | d1 | E04 |
| 2-1-m1E04-2-0629 | 924.316 | OEt | a1 | B09 | b2 | C33 | c3 | D02 | d1 | E04 |
| 2-1-m1E04-2-0630 | 924.335 | OEt | a1 | B05 | b2 | C24 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0631 | 924.355 | OEt | a1 | B01 | b2 | C07 | c2 | D02 | d1 | E04 |
| 2-1-m1E04-2-0632 | 925.290 | OEt | a1 | B08 | b1 | C09 | c1 | D01 | d1 | E04 |
| 2-1-m1E04-2-0633 | 925.294 | OEt | a1 | B04 | b2 | C05 | c2 | D02 | d1 | E04 |
| 2-1-m1E04-2-0634 | 925.299 | OEt | a2 | B17 | b2 | C26 | c3 | D02 | d1 | E04 |
| 2-1-m1E04-2-0635 | 925.315 | OEt | a2 | B19 | b1 | C30 | c3 | D02 | d1 | E04 |
| 2-1-m1E04-2-0636 | 925.329 | OEt | a2 | B15 | b1 | C13 | c2 | D02 | d1 | E04 |
| 2-1-mlE04-2-0637 | 925.350 | OEt | a1 | B02 | b2 | C07 | c2 | D02 | d1 | E04 |
| 2-1-m1E04-2-0638 | 925.370 | OEt | a2 | B20 | b2 | C07 | c1 | D01 | d1 | E04 |
| 2-1-m1E04-2-0639 | 926.280 | OEt | a1 | B05 | b2 | C06 | c2 | D02 | d1 | E04 |
| 2-1-m1E04-2-0640 | 926.314 | OEt | a1 | B06 | b2 | C24 | c3 | D02 | d1 | E04 |
| 2-1-m1E04-2-0641 | 926.314 | OEt | a1 | B04 | b2 | C26 | c3 | D02 | d1 | E04 |
| 2-1-mlE04-2-0642 | 926.331 | OEt | a1 | B07 | b1 | C32 | c3 | D02 | d1 | E04 |
| 2-1-m1E04-2-0643 | 927.221 | OEt | a1 | B08 | b3 | C06 | c1 | D01 | d1 | E04 |
| 2-1-mlE04-2-0644 | 927.244 | OEt | a2 | B14 | b3 | C08 | c2 | D02 | d1 | E04 |
| 2-1-m1E04-2-0645 | 927.258 | OEt | a2 | B17 | b2 | C04 | c2 | D02 | d1 | E04 |
| 2-1-m1E04-2-0646 | 927.264 | OEt | a2 | B15 | b3 | C15 | c2 | D02 | d1 | E04 |
| 2-1-m1E04-2-0647 | 927.295 | OEt | a2 | B13 | b1 | C09 | c2 | D02 | d1 | E04 |
| 2-1-m1E04-2-0648 | 927.295 | OEt | a2 | B19 | b2 | C20 | c3 | D02 | d1 | E04 |
| 2-1-m1E04-2-0649 | 927.308 | OEt | a2 | B16 | b1 | C13 | c2 | D02 | d1 | E04 |
| 2-1-m1E04-2-0650 | 927.314 | OEt | a2 | B18 | b2 | C24 | c3 | D02 | d1 | E04 |
| 2-1-m1E04-2-0651 | 927.324 | OEt | a1 | B10 | b2 | C05 | c1 | D01 | d1 | E04 |
| 2-1-m1E04-2-0652 | 927.334 | OEt | a2 | B15 | b3 | C16 | c1 | D01 | d1 | E04 |
| 2-1-m1E04-2-0653 | 927.370 | OEt | a2 | B13 | b1 | C31 | c3 | D02 | d1 | E04 |
| 2-1-m1E04-2-0654 | 928.216 | OEt | a1 | B08 | b1 | C12 | c2 | D02 | d1 | E04 |
| 2-1-m1E04-2-0655 | 928.259 | OEt | a1 | B06 | b2 | C06 | c2 | D02 | d1 | E04 |
| 2-1-m1E04-2-0656 | 928.259 | OEt | a1 | B01 | b3 | C08 | c2 | D02 | d1 | E04 |
| 2-1-m1E04-2-0657 | 928.274 | OEt | a1 | B07 | b2 | C02 | c2 | D02 | d1 | E04 |
| 2-1-m1E04-2-0658 | 928.274 | OEt | a1 | B04 | b2 | C04 | c2 | D02 | d1 | E04 |
| 2-1-mlE04-2-0659 | 928.290 | OEt | a2 | B19 | b2 | C28 | c3 | D02 | d1 | E04 |
| 2-1-m1E04-2-0660 | 928.310 | OEt | a1 | B07 | b2 | C22 | c3 | D02 | d1 | E04 |
| 2-1-mlE04-2-0661 | 928.381 | OEt | a1 | B09 | b2 | C07 | c1 | D01 | d1 | E04 |
| 2-1-m1E04-2-0662 | 929.226 | OEt | a2 | B13 | b3 | C06 | c2 | D02 | d1 | E04 |
| 2-1-m1E04-2-0663 | 929.243 | OEt | a2 | B16 | b3 | C15 | c2 | D02 | d1 | E04 |
| 2-1-mlE04-2-0664 | 929.255 | OEt | a1 | B02 | b3 | C08 | c2 | D02 | d1 | E04 |
| 2-1-m1E04-2-0665 | 929.259 | OEt | a2 | B18 | b2 | C06 | c2 | D02 | d1 | E04 |
| 2-1-mlE04-2-0666 | 929.269 | OEt | a1 | B07 | b2 | C03 | c2 | D02 | d1 | E04 |
| 2-1-m1E04-2-0667 | 929.274 | OEt | a2 | B20 | b3 | C08 | c1 | D01 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0668 | 929.274 | OEt | a2 | B20 | b1 | C08 | c2 | D02 | d1 | E04 |
| 2-1-m1E04-2-0669 | 929.295 | OEt | a2 | B15 | b3 | C17 | c1 | D01 | d1 | E04 |
| 2-1-m1E04-2-0670 | 929.295 | OEt | a2 | B15 | b1 | C14 | c2 | D02 | d1 | E04 |
| 2-1-m1E04-2-0671 | 929.313 | OEt | a2 | B16 | b3 | C16 | c1 | D01 | d1 | E04 |
| 2-1-m1E04-2-0672 | 930.269 | OEt | a2 | B20 | b1 | C11 | c2 | D02 | d1 | E04 |
| 2-1-m1E04-2-0673 | 930.290 | OEt | a2 | B13 | b3 | C18 | c1 | D01 | d1 | E04 |
| 2-1-m1E04-2-0674 | 930.303 | OEt | a1 | B10 | b2 | C04 | c1 | D01 | d1 | E04 |
| 2-1-mlE04-2-0675 | 930.319 | OEt | a1 | B03 | b1 | C13 | c2 | D02 | d1 | E04 |
| 2-1-m1E04-2-0676 | 931.253 | OEt | a2 | B19 | b2 | C01 | c2 | D02 | d1 | E04 |
| 2-1-m1E04-2-0677 | 931.264 | OEt | a2 | B20 | b1 | C10 | c2 | D02 | d1 | E04 |
| 2-1-m1E04-2-0678 | 931.270 | OEt | a2 | B14 | b1 | C09 | c2 | D02 | d1 | E04 |
| 2-1-m1E04-2-0679 | 931.274 | OEt | a2 | B16 | b3 | C17 | c1 | D01 | d1 | E04 |
| 2-1-m1E04-2-0680 | 931.274 | OEt | a2 | B16 | b1 | C14 | c2 | D02 | d1 | E04 |
| 2-1-m1E04-2-0681 | 931.345 | OEt | a2 | B14 | b1 | C31 | c3 | D02 | d1 | E04 |
| 2-1-m1E04-2-0682 | 932.254 | OEt | a1 | B03 | b3 | C15 | c2 | D02 | d1 | E04 |
| 2-1-m1E04-2-0683 | 932.285 | OEt | a1 | B09 | b3 | C08 | c1 | D01 | d1 | E04 |
| 2-1-mlE04-2-0684 | 932.285 | OEt | a1 | B09 | b1 | C08 | c2 | D02 | d1 | E04 |
| 2-1-m1E04-2-0685 | 932.285 | OEt | a1 | B01 | b1 | C09 | c2 | D02 | d1 | E04 |
| 2-1-m1E04-2-0686 | 932.285 | OEt | a1 | B07 | b2 | C24 | c3 | D02 | d1 | E04 |
| 2-1-mlE04-2-0687 | 932.324 | OEt | a1 | B03 | b3 | C16 | c1 | D01 | d1 | E04 |
| 2-1-m1E04-2-0688 | 932.360 | OEt | a1 | B01 | b1 | C31 | c3 | D02 | d1 | E04 |
| 2-1-mlE04-2-0689 | 932.396 | OEt | a1 | B05 | b1 | C29 | c3 | D02 | d1 | E04 |
| 2-1-m1E04-2-0690 | 933.201 | OEt | a2 | B14 | b3 | C06 | c2 | D02 | d1 | E04 |
| 2-1-mlE04-2-0691 | 933.279 | OEt | a2 | B17 | b1 | C13 | c2 | D02 | d1 | E04 |
| 2-1-m1E04-2-0692 | 933.280 | OEt | a1 | B02 | b1 | C09 | c2 | D02 | d1 | E04 |
| 2-1-m1E04-2-0693 | 933.280 | OEt | a1 | B09 | b1 | C11 | c2 | D02 | d1 | E04 |
| 2-1-m1E04-2-0694 | 933.299 | OEt | a2 | B20 | b1 | C09 | c1 | D01 | d1 | E04 |
| 2-1-m1E04-2-0695 | 933.301 | OEt | a1 | B08 | b1 | C30 | c3 | D02 | d1 | E04 |
| 2-1-m1E04-2-0696 | 933.355 | OEt | a1 | B02 | b1 | C31 | c3 | D02 | d1 | E04 |
| 2-1-m1E04-2-0697 | 933.381 | OEt | a2 | B15 | b2 | C07 | c2 | D02 | d1 | E04 |
| 2-1-m1E04-2-0698 | 934.216 | OEt | a1 | B01 | b3 | C06 | c2 | D02 | d1 | E04 |
| 2-1-m1E04-2-0699 | 934.230 | OEt | a1 | B07 | b2 | C06 | c2 | D02 | d1 | E04 |
| 2-1-m1E04-2-0700 | 934.265 | OEt | a2 | B14 | b3 | C18 | c1 | D01 | d1 | E04 |
| 2-1-m1E04-2-0701 | 934.275 | OEt | a1 | B09 | b1 | C10 | c2 | D02 | d1 | E04 |
| 2-1-m1E04-2-0702 | 934.285 | OEt | a1 | B03 | b3 | C17 | c1 | D01 | d1 | E04 |
| 2-1-m1E04-2-0703 | 934.285 | OEt | a1 | B03 | b1 | C14 | c2 | D02 | d1 | E04 |
| 2-1-m1E04-2-0704 | 934.294 | OEt | a1 | B04 | b1 | C13 | c2 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0705 | 934.376 | OEt | a1 | B06 | b1 | C29 | c3 | D02 | d1 | E04 |
| 2-1-m1E04-2-0706 | 935.211 | OEt | a1 | B02 | b3 | C06 | c2 | D02 | d1 | E04 |
| 2-1-m1E04-2-0707 | 935.214 | OEt | a2 | B17 | b3 | C15 | c2 | D02 | d1 | E04 |
| 2-1-m1E04-2-0708 | 935.230 | OEt | a2 | B20 | b3 | C06 | c1 | D01 | d1 | E04 |
| 2-1-m1E04-2-0709 | 935.280 | OEt | a1 | B01 | b3 | C18 | c1 | D01 | d1 | E04 |
| 2-1-mlE04-2-0710 | 935.280 | OEt | a1 | B08 | b2 | C20 | c3 | D02 | d1 | E04 |
| 2-1-m1E04-2-0711 | 935.283 | OEt | a2 | B17 | b3 | C16 | c1 | D01 | d1 | E04 |
| 2-1-m1E04-2-0712 | 935.335 | OEt | a1 | B05 | b2 | C05 | c2 | D02 | d1 | E04 |
| 2-1-mlE04-2-0713 | 935.360 | OEt | a2 | B16 | b2 | C07 | c2 | D02 | d1 | E04 |
| 2-1-m1E04-2-0714 | 935.375 | OEt | a2 | B18 | b1 | C29 | c3 | D02 | d1 | E04 |
| 2-1-m1E04-2-0715 | 936.226 | OEt | a2 | B20 | b1 | C12 | c2 | D02 | d1 | E04 |
| 2-1-m1E04-2-0716 | 936.229 | OEt | a1 | B04 | b3 | C15 | c2 | D02 | d1 | E04 |
| 2-1-m1E04-2-0717 | 936.276 | OEt | a1 | B02 | b3 | C18 | c1 | D01 | d1 | E04 |
| 2-1-m1E04-2-0718 | 936.276 | OEt | a1 | B08 | b2 | C28 | c3 | D02 | d1 | E04 |
| 2-1-m1E04-2-0719 | 936.299 | OEt | a1 | B04 | b3 | C16 | c1 | D01 | d1 | E04 |
| 2-1-mlE04-2-0720 | 936.310 | OEt | a1 | B09 | b1 | C09 | c1 | D01 | d1 | E04 |
| 2-1-m1E04-2-0721 | 936.324 | OEt | a1 | B10 | b1 | C13 | c1 | D01 | d1 | E04 |
| 2-1-mlE04-2-0722 | 936.355 | OEt | a1 | B05 | b2 | C26 | c3 | D02 | d1 | E04 |
| 2-1-m1E04-2-0723 | 937.245 | OEt | a2 | B17 | b3 | C17 | c1 | D01 | d1 | E04 |
| 2-1-m1E04-2-0724 | 937.245 | OEt | a2 | B17 | b1 | C14 | c2 | D02 | d1 | E04 |
| 2-1-mlE04-2-0725 | 937.285 | OEt | a2 | B15 | b3 | C08 | c2 | D02 | d1 | E04 |
| 2-1-m1E04-2-0726 | 937.314 | OEt | a1 | B06 | b2 | C05 | c2 | D02 | d1 | E04 |
| 2-1-mlE04-2-0727 | 938.241 | OEt | a1 | B09 | b3 | C06 | c1 | D01 | d1 | E04 |
| 2-1-m 1 E04-2-0728 | 938.259 | OEt | a1 | B10 | b3 | C15 | c1 | D01 | d1 | E04 |
| 2-1-m1E04-2-0729 | 938.260 | OEt | a1 | B04 | b3 | C17 | c1 | D01 | d1 | E04 |
| 2-1-m1E04-2-0730 | 938.260 | OEt | a1 | B04 | b1 | C14 | c2 | D02 | d1 | E04 |
| 2-1-m1E04-2-0731 | 938.313 | OEt | a2 | B18 | b2 | COS | c2 | D02 | d1 | E04 |
| 2-1-m1E04-2-0732 | 938.314 | OEt | a1 | B05 | b2 | C04 | c2 | D02 | d1 | E04 |
| 2-1-m1E04-2-0733 | 938.334 | OEt | a1 | B06 | b2 | C26 | c3 | D02 | d1 | E04 |
| 2-1-mlE04-2-0734 | 938.371 | OEt | a1 | B03 | b2 | C07 | c2 | D02 | d1 | E04 |
| 2-1-m1E04-2-0735 | 939.237 | OEt | a1 | B09 | b1 | C12 | c2 | D02 | d1 | E04 |
| 2-1-mlE04-2-0736 | 939.239 | OEt | a1 | B08 | b2 | C01 | c2 | D02 | d1 | E04 |
| 2-1-m1E04-2-0737 | 939.264 | OEt | a2 | B16 | b3 | C08 | c2 | D02 | d1 | E04 |
| 2-1-mlE04-2-0738 | 939.331 | OEt | a2 | B19 | b1 | C32 | c3 | D02 | d1 | E04 |
| 2-1-m1E04-2-0739 | 939.334 | OEt | a2 | B18 | b2 | C26 | c3 | D02 | d1 | E04 |
| 2-1-m1E04-2-0740 | 940.290 | OEt | a1 | B10 | b1 | C14 | c1 | D01 | d1 | E04 |
| 2-1-m1E04-2-0741 | 940.294 | OEt | a1 | B06 | b2 | C04 | c2 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0742 | 940.311 | OEt | a1 | B10 | b2 | C33 | c3 | D02 | d1 | E04 |
| 2-1-m1E04-2-0743 | 940.346 | OEt | a1 | B07 | b1 | C29 | c3 | D02 | d1 | E04 |
| 2-1-m1E04-2-0744 | 941.274 | OEt | a2 | B19 | b2 | C02 | c2 | D02 | d1 | E04 |
| 2-1-m1E04-2-0745 | 941.293 | OEt | a2 | B18 | b2 | C04 | c2 | D02 | d1 | E04 |
| 2-1-m1E04-2-0746 | 941.310 | OEt | a2 | B15 | b1 | C09 | c2 | D02 | d1 | E04 |
| 2-1-m1E04-2-0747 | 941.310 | OEt | a2 | B19 | b2 | C22 | c3 | D02 | d1 | E04 |
| 2-1-m1E04-2-0748 | 941.310 | OEt | a2 | B20 | b1 | C30 | c3 | D02 | d1 | E04 |
| 2-1-m1E04-2-0749 | 941.330 | OEt | a2 | B17 | b2 | C07 | c2 | D02 | d1 | E04 |
| 2-1-m1E04-2-0750 | 941.386 | OEt | a2 | B15 | b1 | C31 | c3 | D02 | d1 | E04 |
| 2-1-mlE04-2-0751 | 942.269 | OEt | a2 | B19 | b2 | C03 | c2 | D02 | d1 | E04 |
| 2-1-mlE04-2-0752 | 942.275 | OEt | a1 | B03 | b3 | C08 | c2 | D02 | d1 | E04 |
| 2-1-m1E04-2-0753 | 942.346 | OEt | a1 | B04 | b2 | C07 | c2 | D02 | d1 | E04 |
| 2-1-mlE04-2-0754 | 943.241 | OEt | a2 | B15 | b3 | C06 | c2 | D02 | d1 | E04 |
| 2-1-m1E04-2-0755 | 943.284 | OEt | a1 | B07 | b2 | C05 | c2 | D02 | d1 | E04 |
| 2-1-mlE04-2-0756 | 943.290 | OEt | a2 | B16 | b1 | C09 | c2 | D02 | d1 | E04 |
| 2-1-m1E04-2-0757 | 943.290 | OEt | a2 | B20 | b2 | C20 | c3 | D02 | d1 | E04 |
| 2-1-m1E04-2-0758 | 943.365 | OEt | a2 | B16 | b1 | C31 | c3 | D02 | d1 | E04 |
| 2-1-m1E04-2-0759 | 944.285 | OEt | a2 | B20 | b2 | C28 | c3 | D02 | d1 | E04 |
| 2-1-m1E04-2-0760 | 944.305 | OEt | a1 | B07 | b2 | C26 | c3 | D02 | d1 | E04 |
| 2-1-m1E04-2-0761 | 944.306 | OEt | a2 | B15 | b3 | C18 | c1 | D01 | d1 | E04 |
| 2-1-m1E04-2-0762 | 944.321 | OEt | a1 | B09 | b1 | C30 | c3 | D02 | d1 | E04 |
| 2-1-m1E04-2-0763 | 944.335 | OEt | a1 | B05 | b1 | C13 | c2 | D02 | d1 | E04 |
| 2-1-m1E04-2-0764 | 944.375 | OEt | a1 | B10 | b2 | C07 | c1 | D01 | d1 | E04 |
| 2-1-m1E04-2-0765 | 945.221 | OEt | a2 | B16 | b3 | C06 | c2 | D02 | d1 | E04 |
| 2-1-m1E04-2-0766 | 945.235 | OEt | a2 | B17 | b3 | C08 | c2 | D02 | d1 | E04 |
| 2-1-m1E04-2-0767 | 945.285 | OEt | a2 | B19 | b2 | C24 | c3 | D02 | d1 | E04 |
| 2-1-m1E04-2-0768 | 946.250 | OEt | a1 | B04 | b3 | C08 | c2 | D02 | d1 | E04 |
| 2-1-m1E04-2-0769 | 946.264 | OEt | a1 | B07 | b2 | C04 | c2 | D02 | d1 | E04 |
| 2-1-m1E04-2-0770 | 946.270 | OEt | a1 | B05 | b3 | C15 | c2 | D02 | d1 | E04 |
| 2-1-m1E04-2-0771 | 946.285 | OEt | a2 | B16 | b3 | C18 | c1 | D01 | d1 | E04 |
| 2-1-m1E04-2-0772 | 946.301 | OEt | a1 | B03 | b1 | C09 | c2 | D02 | d1 | E04 |
| 2-1-m1E04-2-0773 | 946.301 | OEt | a1 | B09 | b2 | C20 | c3 | D02 | d1 | E04 |
| 2-1-m1E04-2-0774 | 946.314 | OEt | a1 | B06 | b1 | C13 | c2 | D02 | d1 | E04 |
| 2-1-m1E04-2-0775 | 946.339 | OEt | a1 | B05 | b3 | C16 | c1 | D01 | d1 | E04 |
| 2-1-mlE04-2-0776 | 946.376 | OEt | a1 | B03 | b1 | C31 | c3 | D02 | d1 | E04 |
| 2-1-m1E04-2-0777 | 947.230 | OEt | a2 | B19 | b2 | C06 | c2 | D02 | d1 | E04 |
| 2-1-m1E04-2-0778 | 947.248 | OEt | a2 | B20 | b2 | C01 | c2 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0779 | 947.296 | OEt | a1 | B09 | b2 | C28 | c3 | D02 | d1 | E04 |
| 2-1-m1E04-2-0780 | 947.313 | OEt | a2 | B18 | b1 | C13 | c2 | D02 | d1 | E04 |
| 2-1-m1E04-2-0781 | 947.317 | OEt | a1 | B08 | b1 | C32 | c3 | D02 | d1 | E04 |
| 2-1-m1E04-2-0782 | 948.231 | OEt | a1 | B03 | b3 | C06 | c2 | D02 | d1 | E04 |
| 2-1-m1E04-2-0783 | 948.249 | OEt | a1 | B06 | b3 | C15 | c2 | D02 | d1 | E04 |
| 2-1-m1E04-2-0784 | 948.280 | OEt | a1 | B10 | b3 | C08 | c1 | D01 | d1 | E04 |
| 2-1-m1E04-2-0785 | 948.280 | OEt | a1 | B10 | b1 | C08 | c2 | D02 | d1 | E04 |
| 2-1-m1E04-2-0786 | 948.301 | OEt | a1 | B05 | b3 | C17 | c1 | D01 | d1 | E04 |
| 2-1-m1E04-2-0787 | 948.301 | OEt | a1 | B05 | b1 | C14 | c2 | D02 | d1 | E04 |
| 2-1-m1E04-2-0788 | 948.319 | OEt | a1 | B06 | b3 | C16 | c1 | D01 | d1 | E04 |
| 2-1-m1E04-2-0789 | 949.249 | OEt | a2 | B18 | b3 | C15 | c2 | D02 | d1 | E04 |
| 2-1-m1E04-2-0790 | 949.260 | OEt | a1 | B08 | b2 | C02 | c2 | D02 | d1 | E04 |
| 2-1-m1E04-2-0791 | 949.260 | OEt | a2 | B17 | b1 | C09 | c2 | D02 | d1 | E04 |
| 2-1-m1E04-2-0792 | 949.275 | OEt | a1 | B10 | b1 | C11 | c2 | D02 | d1 | E04 |
| 2-1-m1E04-2-0793 | 949.296 | OEt | a1 | B03 | b3 | C18 | c1 | D01 | d1 | E04 |
| 2-1-m1E04-2-0794 | 949.296 | OEt | a1 | B08 | b2 | C22 | c3 | D02 | d1 | E04 |
| 2-1-m1E04-2-0795 | 949.318 | OEt | a2 | B18 | b3 | C16 | c1 | D01 | d1 | E04 |
| 2-1-m1E04-2-0796 | 949.335 | OEt | a2 | B17 | b1 | C31 | c3 | D02 | d1 | E04 |
| 2-1-m1E04-2-0797 | 950.255 | OEt | a1 | B08 | b2 | C03 | c2 | D02 | d1 | E04 |
| 2-1-m1E04-2-0798 | 950.259 | OEt | a1 | B09 | b2 | C01 | c2 | D02 | d1 | E04 |
| 2-1-m1E04-2-0799 | 950.270 | OEt | a1 | B10 | b1 | C10 | c2 | D02 | d1 | E04 |
| 2-1-m1E04-2-0800 | 950.275 | OEt | a1 | B04 | b1 | C09 | c2 | D02 | d1 | E04 |
| 2-1-m1E04-2-0801 | 950.280 | OEt | a1 | B06 | b3 | C17 | c1 | D01 | d1 | E04 |
| 2-1-m1E04-2-0802 | 950.280 | OEt | a1 | B06 | b1 | C14 | c2 | D02 | d1 | E04 |
| 2-1-m1E04-2-0803 | 950.351 | OEt | a1 | B04 | b1 | C31 | c3 | D02 | d1 | E04 |
| 2-1-m1E04-2-0804 | 951.191 | OEt | a2 | B17 | b3 | C06 | c2 | D02 | d1 | E04 |
| 2-1-m1E04-2-0805 | 951.279 | OEt | a2 | B18 | b3 | C17 | c1 | D01 | d1 | E04 |
| 2-1-m1E04-2-0806 | 951.279 | OEt | a2 | B18 | b1 | C14 | c2 | D02 | d1 | E04 |
| 2-1-m1E04-2-0807 | 952.206 | OEt | a1 | B04 | b3 | C06 | c2 | D02 | d1 | E04 |
| 2-1-m1E04-2-0808 | 952.256 | OEt | a2 | B17 | b3 | C18 | c1 | D01 | d1 | E04 |
| 2-1-m1E04-2-0809 | 952.285 | OEt | a1 | B07 | b1 | C13 | c2 | D02 | d1 | E04 |
| 2-1-m1E04-2-0810 | 952.305 | OEt | a1 | B10 | b1 | C09 | c1 | D01 | d1 | E04 |
| 2-1-m1E04-2-0811 | 952.386 | OEt | a1 | B05 | b2 | C07 | c2 | D02 | d1 | E04 |
| 2-1-m1E04-2-0812 | 953.271 | OEt | a1 | B04 | b3 | C18 | c1 | D01 | d1 | E04 |
| 2-1-m1E04-2-0813 | 953.271 | OEt | a1 | B08 | b2 | C24 | c3 | D02 | d1 | E04 |
| 2-1-m1E04-2-0814 | 953.347 | OEt | a2 | B19 | b1 | C29 | c3 | D02 | d1 | E04 |
| 2-1-m1E04-2-0815 | 954.220 | OEt | a1 | B07 | b3 | C15 | c2 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0816 | 954.236 | OEt | a1 | B10 | b3 | C06 | c1 | D01 | d1 | E04 |
| 2-1-m1E04-2-0817 | 954.289 | OEt | a1 | B07 | b3 | C16 | c1 | D01 | d1 | E04 |
| 2-1-m1E04-2-0818 | 954.366 | OEt | a1 | B06 | b2 | C07 | c2 | D02 | d1 | E04 |
| 2-1-m1E04-2-0819 | 955.216 | OEt | a1 | B08 | b2 | C06 | c2 | D02 | d1 | E04 |
| 2-1-m1E04-2-0820 | 955.231 | OEt | a1 | B10 | b1 | C12 | c2 | D02 | d1 | E04 |
| 2-1-m1E04-2-0821 | 955.326 | OEt | a2 | B20 | b1 | C32 | c3 | D02 | d1 | E04 |
| 2-1-m1E04-2-0822 | 955.365 | OEt | a2 | B18 | b2 | C07 | c2 | D02 | d1 | E04 |
| 2-1-m1E04-2-0823 | 956.251 | OEt | a1 | B07 | b3 | C17 | c1 | D01 | d1 | E04 |
| 2-1-m1E04-2-0824 | 956.251 | OEt | a1 | B07 | b1 | C14 | c2 | D02 | d1 | E04 |
| 2-1-m1E04-2-0825 | 956.285 | OEt | a2 | B19 | b2 | C05 | c2 | D02 | d1 | E04 |
| 2-1-m1E04-2-0826 | 956.291 | OEt | a1 | B05 | b3 | C08 | c2 | D02 | d1 | E04 |
| 2-1-m1E04-2-0827 | 957.269 | OEt | a2 | B20 | b2 | C02 | c2 | D02 | d1 | E04 |
| 2-1-m1E04-2-0828 | 957.305 | OEt | a2 | B20 | b2 | C22 | c3 | D02 | d1 | E04 |
| 2-1-m1E04-2-0829 | 957.305 | OEt | a2 | B19 | b2 | C26 | c3 | D02 | d1 | E04 |
| 2-1-m1E04-2-0830 | 958.264 | OEt | a2 | B20 | b2 | C03 | c2 | D02 | d1 | E04 |
| 2-1-m1E04-2-0831 | 958.270 | OEt | a1 | B06 | b3 | C08 | c2 | D02 | d1 | E04 |
| 2-1-m1E04-2-0832 | 958.337 | OEt | a1 | B09 | b1 | C32 | c3 | D02 | d1 | E04 |
| 2-1-m1E04-2-0833 | 959.265 | OEt | a2 | B19 | b2 | C04 | c2 | D02 | d1 | E04 |
| 2-1-m1E04-2-0834 | 959.269 | OEt | a2 | B18 | b3 | C08 | c2 | D02 | d1 | E04 |
| 2-1-m1E04-2-0835 | 960.280 | OEt | a1 | B09 | b2 | C02 | c2 | D02 | d1 | E04 |
| 2-1-m1E04-2-0836 | 960.316 | OEt | a1 | B05 | b1 | C09 | c2 | D02 | d1 | E04 |
| 2-1-m1E04-2-0837 | 960.316 | OEt | a1 | B09 | b2 | C22 | c3 | D02 | d1 | E04 |
| 2-1-m1E04-2-0838 | 960.316 | OEt | a1 | B10 | b1 | C30 | c3 | D02 | d1 | E04 |
| 2-1-m1E04-2-0839 | 960.336 | OEt | a1 | B07 | b2 | C07 | c2 | D02 | d1 | E04 |
| 2-1-m1E04-2-0840 | 960.391 | OEt | a1 | B05 | b1 | C31 | c3 | D02 | d1 | E04 |
| 2-1-m1E04-2-0841 | 961.275 | OEt | a1 | B09 | b2 | C03 | c2 | D02 | d1 | E04 |
| 2-1-m1E04-2-0842 | 961.280 | OEt | a2 | B20 | b2 | C24 | c3 | D02 | d1 | E04 |
| 2-1-m1E04-2-0843 | 961.333 | OEt | a1 | B08 | b1 | C29 | c3 | D02 | d1 | E04 |
| 2-1-m1E04-2-0844 | 962.247 | OEt | a1 | B05 | b3 | C06 | c2 | D02 | d1 | E04 |
| 2-1-m1E04-2-0845 | 962.295 | OEt | a1 | B06 | b1 | C09 | c2 | D02 | d1 | E04 |
| 2-1-m1E04-2-0846 | 962.295 | OEt | a1 | B10 | b2 | C20 | c3 | D02 | d1 | E04 |
| 2-1-m1E04-2-0847 | 962.371 | OEt | a1 | B06 | b1 | C31 | c3 | D02 | d1 | E04 |
| 2-1-m1E04-2-0848 | 963.225 | OEt | a2 | B20 | b2 | C06 | c2 | D02 | d1 | E04 |
| 2-1-m1E04-2-0849 | 963.264 | OEt | a2 | B11 | b3 | C16 | c2 | D02 | d1 | E04 |
| 2-1-m1E04-2-0850 | 963.291 | OEt | a1 | B10 | b2 | C28 | c3 | D02 | d1 | E04 |
| 2-1-m1E04-2-0851 | 963.295 | OEt | a2 | B18 | b1 | C09 | c2 | D02 | d1 | E04 |
| 2-1-m1E04-2-0852 | 963.312 | OEt | a1 | B05 | b3 | C18 | c1 | D01 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0853 | 963.370 | OEt | a2 | B18 | b1 | C31 | c3 | D02 | d1 | E04 |
| 2-1-m1E04-2-0854 | 964.226 | OEt | a1 | B06 | b3 | C06 | c2 | D02 | d1 | E04 |
| 2-1-m1E04-2-0855 | 964.241 | OEt | a1 | B07 | b3 | C08 | c2 | D02 | d1 | E04 |
| 2-1-m1E04-2-0856 | 964.259 | OEt | a2 | B12 | b3 | C16 | c2 | D02 | d1 | E04 |
| 2-1-m1E04-2-0857 | 964.271 | OEt | a1 | B08 | b2 | C05 | c2 | D02 | d1 | E04 |
| 2-1-m1E04-2-0858 | 964.291 | OEt | a1 | B09 | b2 | C24 | c3 | D02 | d1 | E04 |
| 2-1-m1E04-2-0859 | 965.226 | OEt | a2 | B18 | b3 | C06 | c2 | D02 | d1 | E04 |
| 2-1-m1E04-2-0860 | 965.226 | OEt | a2 | B11 | b3 | C17 | c2 | D02 | d1 | E04 |
| 2-1-m1E04-2-0861 | 965.285 | OEt | a2 | B19 | b1 | C13 | c2 | D02 | d1 | E04 |
| 2-1-m1E04-2-0862 | 965.291 | OEt | a1 | B06 | b3 | C18 | c1 | D01 | d1 | E04 |
| 2-1-m1E04-2-0863 | 965.291 | OEt | a1 | B08 | b2 | C26 | c3 | D02 | d1 | E04 |
| 2-1-m1E04-2-0864 | 966.221 | OEt | a2 | B12 | b3 | C17 | c2 | D02 | d1 | E04 |
| 2-1-m1E04-2-0865 | 966.236 | OEt | a1 | B09 | b2 | C06 | c2 | D02 | d1 | E04 |
| 2-1-m1E04-2-0866 | 966.254 | OEt | a1 | B10 | b2 | C01 | c2 | D02 | d1 | E04 |
| 2-1-m1E04-2-0867 | 966.290 | OEt | a2 | B18 | b3 | C18 | c1 | D01 | d1 | E04 |
| 2-1-m1E04-2-0868 | 967.220 | OEt | a2 | B19 | b3 | C15 | c2 | D02 | d1 | E04 |
| 2-1-m1E04-2-0869 | 967.250 | OEt | a1 | B08 | b2 | C04 | c2 | D02 | d1 | E04 |
| 2-1-m1E04-2-0870 | 967.290 | OEt | a2 | B19 | b3 | C16 | c1 | D01 | d1 | E04 |
| 2-1-m1E04-2-0871 | 968.266 | OEt | a1 | B07 | b1 | C09 | c2 | D02 | d1 | E04 |
| 2-1-m 1 E04-2-0872 | 968.341 | OEt | a1 | B07 | b1 | C31 | c3 | D02 | d1 | E04 |
| 2-1-m1E04-2-0873 | 969.251 | OEt | a2 | B19 | b3 | C17 | c1 | D01 | d1 | E04 |
| 2-1-m1E04-2-0874 | 969.251 | OEt | a2 | B19 | b1 | C14 | c2 | D02 | d1 | E04 |
| 2-1-m1E04-2-0875 | 969.342 | OEt | a2 | B20 | b1 | C29 | c3 | D02 | d1 | E04 |
| 2-1-m1E04-2-0876 | 970.197 | OEt | a1 | B07 | b3 | C06 | c2 | D02 | d1 | E04 |
| 2-1-m1E04-2-0877 | 971.262 | OEt | a1 | B07 | b3 | C18 | c1 | D01 | d1 | E04 |
| 2-1-m1E04-2-0878 | 972.280 | OEt | a2 | B20 | b2 | C05 | c2 | D02 | d1 | E04 |
| 2-1-m1E04-2-0879 | 972.353 | OEt | a1 | B09 | b1 | C29 | c3 | D02 | d1 | E04 |
| 2-1-m1E04-2-0880 | 973.271 | OEt | a1 | B08 | b1 | C13 | c2 | D02 | d1 | E04 |
| 2-1-m1E04-2-0881 | 973.300 | OEt | a2 | B20 | b2 | C26 | c3 | D02 | d1 | E04 |
| 2-1-m1E04-2-0882 | 973.337 | OEt | a2 | B19 | b2 | C07 | c2 | D02 | d1 | E04 |
| 2-1-m1E04-2-0883 | 974.332 | OEt | a1 | B10 | b1 | C32 | c3 | D02 | d1 | E04 |
| 2-1-m1E04-2-0884 | 975.206 | OEt | a1 | B08 | b3 | C15 | c2 | D02 | d1 | E04 |
| 2-1-m1E04-2-0885 | 975.259 | OEt | a2 | B20 | b2 | C04 | c2 | D02 | d1 | E04 |
| 2-1-m1E04-2-0886 | 975.275 | OEt | a1 | B08 | b3 | C16 | c1 | D01 | d1 | E04 |
| 2-1-m1E04-2-0887 | 975.291 | OEt | a1 | B09 | b2 | C05 | c2 | D02 | d1 | E04 |
| 2-1-m1E04-2-0888 | 976.275 | OEt | a1 | B10 | b2 | C02 | c2 | D02 | d1 | E04 |
| 2-1-m1E04-2-0889 | 976.311 | OEt | a1 | B10 | b2 | C22 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0890 | 976.311 | OEt | a1 | B09 | b2 | C26 | c3 | D02 | d1 | E04 |
| 2-1-m1E04-2-0891 | 977.237 | OEt | a1 | B08 | b3 | C17 | c1 | D01 | d1 | E04 |
| 2-1-m1E04-2-0892 | 977.237 | OEt | a1 | B08 | b1 | C14 | c2 | D02 | d1 | E04 |
| 2-1-m1E04-2-0893 | 977.241 | OEt | a2 | B19 | b3 | C08 | c2 | D02 | d1 | E04 |
| 2-1-m1E04-2-0894 | 977.270 | OEt | a1 | B10 | b2 | C03 | c2 | D02 | d1 | E04 |
| 2-1-m1E04-2-0895 | 977.280 | OEt | a2 | B13 | b3 | C16 | c2 | D02 | d1 | E04 |
| 2-1-m1E04-2-0896 | 978.270 | OEt | a1 | B09 | b2 | C04 | c2 | D02 | d1 | E04 |
| 2-1-m1E04-2-0897 | 979.241 | OEt | a2 | B13 | b3 | C17 | c2 | D02 | d1 | E04 |
| 2-1-m1E04-2-0898 | 980.237 | OEt | a2 | B11 | b3 | C18 | c2 | D02 | d1 | E04 |
| 2-1-m1E04-2-0899 | 980.286 | OEt | a1 | B10 | b2 | C24 | c3 | D02 | d1 | E04 |
| 2-1-m1E04-2-0900 | 981.232 | OEt | a2 | B12 | b3 | C18 | c2 | D02 | d1 | E04 |
| 2-1-m1E04-2-0901 | 981.255 | OEt | a2 | B14 | b3 | C16 | c2 | D02 | d1 | E04 |
| 2-1-m1E04-2-0902 | 981.266 | OEt | a2 | B19 | b1 | C09 | c2 | D02 | d1 | E04 |
| 2-1-m1E04-2-0903 | 981.280 | OEt | a2 | B20 | b1 | C13 | c2 | D02 | d1 | E04 |
| 2-1-m1E04-2-0904 | 981.322 | OEt | a1 | B08 | b2 | C07 | c2 | D02 | d1 | E04 |
| 2-1-m1E04-2-0905 | 981.342 | OEt | a2 | B19 | b1 | C31 | c3 | D02 | d1 | E04 |
| 2-1-m1E04-2-0906 | 982.231 | OEt | a1 | B10 | b2 | C06 | c2 | D02 | d1 | E04 |
| 2-1-m1E04-2-0907 | 982.270 | OEt | a1 | B01 | b3 | C16 | c2 | D02 | d1 | E04 |
| 2-1-m1E04-2-0908 | 983.197 | OEt | a2 | B19 | b3 | C06 | c2 | D02 | d1 | E04 |
| 2-1-m1E04-2-0909 | 983.215 | OEt | a2 | B20 | b3 | C15 | c2 | D02 | d1 | E04 |
| 2-1-m1E04-2-0910 | 983.216 | OEt | a2 | B14 | b3 | C17 | c2 | D02 | d1 | E04 |
| 2-1-m1E04-2-0911 | 983.265 | OEt | a1 | B02 | b3 | C16 | c2 | D02 | d1 | E04 |
| 2-1-m1E04-2-0912 | 983.285 | OEt | a2 | B20 | b3 | C16 | c1 | D01 | d1 | E04 |
| 2-1-m1E04-2-0913 | 984.231 | OEt | a1 | B01 | b3 | C17 | c2 | D02 | d1 | E04 |
| 2-1-m1E04-2-0914 | 984.262 | OEt | a2 | B19 | b3 | C18 | c1 | D01 | d1 | E04 |
| 2-1-m1E04-2-0915 | 984.291 | OEt | a1 | B09 | b1 | C13 | c2 | D02 | d1 | E04 |
| 2-1-m1E04-2-0916 | 985.227 | OEt | a1 | B02 | b3 | C17 | c2 | D02 | d1 | E04 |
| 2-1-m1E04-2-0917 | 985.227 | OEt | a1 | B08 | b3 | C08 | c2 | D02 | d1 | E04 |
| 2-1-m1E04-2-0918 | 985.246 | OEt | a2 | B20 | b3 | C17 | c1 | D01 | d1 | E04 |
| 2-1-m1E04-2-0919 | 985.246 | OEt | a2 | B20 | b1 | C14 | c2 | D02 | d1 | E04 |
| 2-1-m1E04-2-0920 | 986.226 | OEt | a1 | B09 | b3 | C15 | c2 | D02 | d1 | E04 |
| 2-1-m1E04-2-0921 | 986.295 | OEt | a1 | B09 | b3 | C16 | c1 | D01 | d1 | E04 |
| 2-1-m1E04-2-0922 | 988.257 | OEt | a1 | B09 | b3 | C17 | c1 | D01 | d1 | E04 |
| 2-1-m1E04-2-0923 | 988.257 | OEt | a1 | B09 | b1 | C14 | c2 | D02 | d1 | E04 |
| 2-1-m1E04-2-0924 | 988.347 | OEt | a1 | B10 | b1 | C29 | c3 | D02 | d1 | E04 |
| 2-1-m1E04-2-0925 | 989.252 | OEt | a1 | B08 | b1 | C09 | c2 | D02 | d1 | E04 |
| 2-1-m1E04-2-0926 | 989.327 | OEt | a1 | B08 | b1 | C31 | c3 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0927 | 989.332 | OEt | a2 | B20 | b2 | C07 | c2 | D02 | d1 | E04 |
| 2-1-m1E04-2-0928 | 991.183 | OEt | a1 | B08 | b3 | C06 | c2 | D02 | d1 | E04 |
| 2-1-m1E04-2-0929 | 991.286 | OEt | a1 | B10 | b2 | C05 | c2 | D02 | d1 | E04 |
| 2-1-m1E04-2-0930 | 991.295 | OEt | a2 | B15 | b3 | C16 | c2 | D02 | d1 | E04 |
| 2-1-m1E04-2-0931 | 992.248 | OEt | a1 | B08 | b3 | C18 | c1 | D01 | d1 | E04 |
| 2-1-m1E04-2-0932 | 992.306 | OEt | a1 | B10 | b2 | C26 | c3 | D02 | d1 | E04 |
| 2-1-m1E04-2-0933 | 992.342 | OEt | a1 | B09 | b2 | C07 | c2 | D02 | d1 | E04 |
| 2-1-m1E04-2-0934 | 993.236 | OEt | a2 | B20 | b3 | C08 | c2 | D02 | d1 | E04 |
| 2-1-m1E04-2-0935 | 993.257 | OEt | a2 | B15 | b3 | C17 | c2 | D02 | d1 | E04 |
| 2-1-m1E04-2-0936 | 993.275 | OEt | a2 | B16 | b3 | C16 | c2 | D02 | d1 | E04 |
| 2-1-m1E04-2-0937 | 994.252 | OEt | a2 | B13 | b3 | C18 | c2 | D02 | d1 | E04 |
| 2-1-m1E04-2-0938 | 994.265 | OEt | a1 | B10 | b2 | C04 | c2 | D02 | d1 | E04 |
| 2-1-m1E04-2-0939 | 995.236 | OEt | a2 | B16 | b3 | C17 | c2 | D02 | d1 | E04 |
| 2-1-m1E04-2-0940 | 996.247 | OEt | a1 | B09 | b3 | C08 | c2 | D02 | d1 | E04 |
| 2-1-m1E04-2-0941 | 996.286 | OEt | a1 | B03 | b3 | C16 | c2 | D02 | d1 | E04 |
| 2-1-m1E04-2-0942 | 997.261 | OEt | a2 | B20 | b1 | C09 | c2 | D02 | d1 | E04 |
| 2-1-m1E04-2-0943 | 997.337 | OEt | a2 | B20 | b1 | C31 | c3 | D02 | d1 | E04 |
| 2-1-m1E04-2-0944 | 998.227 | OEt | a2 | B14 | b3 | C18 | c2 | D02 | d1 | E04 |
| 2-1-m1E04-2-0945 | 998.247 | OEt | a1 | B03 | b3 | C17 | c2 | D02 | d1 | E04 |
| 2-1-m1E04-2-0946 | 999.192 | OEt | a2 | B20 | b3 | C06 | c2 | D02 | d1 | E04 |
| 2-1-m1E04-2-0947 | 999.242 | OEt | a1 | B01 | b3 | C18 | c2 | D02 | d1 | E04 |
| 2-1-m1E04-2-0948 | 999.245 | OEt | a2 | B17 | b3 | C16 | c2 | D02 | d1 | E04 |
| 2-1-m1E04-2-0949 | 1000.240 | OEt | a1 | B02 | b3 | C18 | c2 | D02 | d1 | E04 |
| 2-1-m1E04-2-0950 | 1000.260 | OEt | a2 | B20 | b3 | C18 | c1 | D01 | d1 | E04 |
| 2-1-m1E04-2-0951 | 1000.260 | OEt | a1 | B04 | b3 | C16 | c2 | D02 | d1 | E04 |
| 2-1-m1E04-2-0952 | 1000.270 | OEt | a1 | B09 | b1 | C09 | c2 | D02 | d1 | E04 |
| 2-1-m1E04-2-0953 | 1000.290 | OEt | a1 | B10 | b1 | C13 | c2 | D02 | d1 | E04 |
| 2-1-m1E04-2-0954 | 1000.350 | OEt | a1 | B09 | b1 | C31 | c3 | D02 | d1 | E04 |
| 2-1-m1E04-2-0955 | 1001.210 | OEt | a2 | B17 | b3 | C17 | c2 | D02 | d1 | E04 |
| 2-1-m1E04-2-0956 | 1002.200 | OEt | a1 | B09 | b3 | C06 | c2 | D02 | d1 | E04 |
| 2-1-m1E04-2-0957 | 1002.220 | OEt | a1 | B10 | b3 | C15 | c2 | D02 | d1 | E04 |
| 2-1-m1E04-2-0958 | 1002.220 | OEt | a1 | B04 | b3 | C17 | c2 | D02 | d1 | E04 |
| 2-1-m1E04-2-0959 | 1002.290 | OEt | a1 | B10 | b3 | C16 | c1 | D01 | d1 | E04 |
| 2-1-m1E04-2-0960 | 1003.270 | OEt | a1 | B09 | b3 | C18 | c1 | D01 | d1 | E04 |
| 2-1-m1E04-2-0961 | 1004.250 | OEt | a1 | B10 | b3 | C17 | c1 | D01 | d1 | E04 |
| 2-1-m1E04-2-0962 | 1004.250 | OEt | a1 | B10 | b1 | C14 | c2 | D02 | d1 | E04 |
| 2-1-m1E04-2-0963 | 1008.270 | OEt | a2 | B15 | b3 | C18 | c2 | D02 | d1 | E04 |

(continued)

| [Table 8-10-5] Compound that may be contained in mixture 2-1-m1E04-2 | | | | | | | | | | |
|---|---|---|---|---|---|---|---|---|---|---|
| ID | Exact Mass | × | a | B | b | C | c | D | d | E |
| 2-1-m1E04-2-0964 | 1008.340 | OEt | a1 | B10 | b2 | C07 | c2 | D02 | d1 | E04 |
| 2-1-m1E04-2-0965 | 1010.250 | OEt | a2 | B16 | b3 | C18 | c2 | D02 | d1 | E04 |
| 2-1-m1E04-2-0966 | 1010.300 | OEt | a1 | B05 | b3 | C16 | c2 | D02 | d1 | E04 |
| 2-1-m1E04-2-0967 | 1012.240 | OEt | a1 | B10 | b3 | C08 | c2 | D02 | d1 | E04 |
| 2-1-m1E04-2-0968 | 1012.260 | OEt | a1 | B05 | b3 | C17 | c2 | D02 | d1 | E04 |
| 2-1-m1E04-2-0969 | 1012.280 | OEt | a1 | B06 | b3 | C16 | c2 | D02 | d1 | E04 |
| 2-1-m1E04-2-0970 | 1013.260 | OEt | a1 | B03 | b3 | C18 | c2 | D02 | d1 | E04 |
| 2-1-m1E04-2-0971 | 1013.280 | OEt | a2 | B18 | b3 | C16 | c2 | D02 | d1 | E04 |
| 2-1-m1E04-2-0972 | 1014.240 | OEt | a1 | B06 | b3 | C17 | c2 | D02 | d1 | E04 |
| 2-1-m1E04-2-0973 | 1015.240 | OEt | a2 | B18 | b3 | C17 | c2 | D02 | d1 | E04 |
| 2-1-m1E04-2-0974 | 1016.220 | OEt | a2 | B17 | b3 | C18 | c2 | D02 | d1 | E04 |
| 2-1-m1E04-2-0975 | 1016.270 | OEt | a1 | B10 | b1 | C09 | c2 | D02 | d1 | E04 |
| 2-1-m1E04-2-0976 | 1016.340 | OEt | a1 | B10 | b1 | C31 | c3 | D02 | d1 | E04 |
| 2-1-m1E04-2-0977 | 1017.230 | OEt | a1 | B04 | b3 | C18 | c2 | D02 | d1 | E04 |
| 2-1-m1E04-2-0978 | 1018.200 | OEt | a1 | B10 | b3 | C06 | c2 | D02 | d1 | E04 |
| 2-1-m1E04-2-0979 | 1018.250 | OEt | a1 | B07 | b3 | C16 | c2 | D02 | d1 | E04 |
| 2-1-m1E04-2-0980 | 1019.260 | OEt | a1 | B10 | b3 | C18 | c1 | D01 | d1 | E04 |
| 2-1-m1E04-2-0981 | 1020.210 | OEt | a1 | B07 | b3 | C17 | c2 | D02 | d1 | E04 |
| 2-1-m1E04-2-0982 | 1027.270 | OEt | a1 | B05 | b3 | C18 | c2 | D02 | d1 | E04 |
| 2-1-m1E04-2-0983 | 1029.250 | OEt | a1 | B06 | b3 | C18 | c2 | D02 | d1 | E04 |
| 2-1-m1E04-2-0984 | 1030.250 | OEt | a2 | B18 | b3 | C18 | c2 | D02 | d1 | E04 |
| 2-1-m1E04-2-0985 | 1031.250 | OEt | a2 | B19 | b3 | C16 | c2 | D02 | d1 | E04 |
| 2-1-m1E04-2-0986 | 1033.210 | OEt | a2 | B19 | b3 | C17 | c2 | D02 | d1 | E04 |
| 2-1-m1E04-2-0987 | 1035.220 | OEt | a1 | B07 | b3 | C18 | c2 | D02 | d1 | E04 |
| 2-1-m1E04-2-0988 | 1039.240 | OEt | a1 | B08 | b3 | C16 | c2 | D02 | d1 | E04 |
| 2-1-m1E04-2-0989 | 1041.200 | OEt | a1 | B08 | b3 | C17 | c2 | D02 | d1 | E04 |
| 2-1-m1E04-2-0990 | 1047.250 | OEt | a2 | B20 | b3 | C16 | c2 | D02 | d1 | E04 |
| 2-1-m1E04-2-0991 | 1048.220 | OEt | a2 | B19 | b3 | C18 | c2 | D02 | d1 | E04 |
| 2-1-m1E04-2-0992 | 1049.210 | OEt | a2 | B20 | b3 | C17 | c2 | D02 | d1 | E04 |
| 2-1-m1E04-2-0993 | 1050.260 | OEt | a1 | B09 | b3 | C16 | c2 | D02 | d1 | E04 |
| 2-1-m1E04-2-0994 | 1052.220 | OEt | a1 | B09 | b3 | C17 | c2 | D02 | d1 | E04 |
| 2-1-m1E04-2-0995 | 1056.210 | OEt | a1 | B08 | b3 | C18 | c2 | D02 | d1 | E04 |
| 2-1-m1E04-2-0996 | 1064.220 | OEt | a2 | B20 | b3 | C18 | c2 | D02 | d1 | E04 |
| 2-1-m1E04-2-0997 | 1066.250 | OEt | a1 | B10 | b3 | C16 | c2 | D02 | d1 | E04 |
| 2-1-m1E04-2-0998 | 1067.230 | OEt | a1 | B09 | b3 | C18 | c2 | D02 | d1 | E04 |
| 2-1-m1E04-2-0999 | 1068.210 | OEt | a1 | B10 | b3 | C17 | c2 | D02 | d1 | E04 |
| 2-1-m1E04-2-1000 | 1083.220 | OEt | a1 | B10 | b3 | C18 | c2 | D02 | d1 | E04 |

Example 8-11: Analysis of mixture

The mixtures synthesized in Examples 8-9 and 8-10 were subjected to retention time measurement and mass spectrometry under the following analysis conditions and analyzed using Compound Discoverer 3.2 (Thermo Fisher Scientific Inc.).

[Table 406]

| Reverse-phase LC/MS conditions | |
|---|---|
| Vanquish UHPLC (The rmo Fisher Scientific) | |
| Oven (°C) | 35 |
| Column | Ascentis Express C18 2.1mmI.D.x150mm, 2.7μm |
| Auto Sampler (°C) | 20 |
| | |
| Mobile phase | A) 0.1%FA $H_2O$<br>B) 0.1 %FA MeCN |
| Gradient | A/B = 95/5 → 0/100 (18min) → 0/100 (20min) |
| Flow rate | 0.5 mL/min |
| Orbitrap Fusion Lumos Tribrid mass spectrometer (Thermo Fisher Scientific) | |
| Polarity | Positive / Negative mode |
| Data Type | profile |

The results of analyzing each mixture are shown in Table 8-11-1 to Table 8-11-8. Each table shows m/z and retention times of observed MS peaks and the number of a compound that can be assigned to each MS peak.

Although m/z of the MS peaks was confirmed down to four decimal places, 0 at the decimal level may be omitted in the description in Table 8-11-1 to Table 8-11-8. For example, "587.0000" is also referred to as "587", "587.1000" is also referred to as "587.1", "587.1200" is also referred to as "587.12", and "587.1230" is also referred to as "587.123". Although the retention times were confirmed down to three decimal places, 0 at the decimal level may be omitted in the description in Table 8-11-1 to Table 8-11-8. For example, "15.000" is also referred to as "15", "15.100" is also referred to as "15.1", and "15.120" is also referred to as "15.12".

[Table 407]

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 587.2662 | 14.953 | 0001 |
| M+H | 588.2615 | 14.092 | 0002,0003 |
| M+H | 588.2612 | 13.04 | 0002,0003 |
| M+H | 589.2574 | 15.734 | 0004,0005 |
| M+H | 589.2564 | 11.882 | 0004,0005 |
| M+H | 590.2523 | 13.71 | 0006 |
| M+H | 594.2189 | 15.544 | 0007 |
| M+H | 595.2134 | 13.635 | 0008 |
| M+H | 601.2824 | 15.484 | 0009 |
| M+H | 602.2775 | 14.643 | 0010 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 603.2729 | 16.218 | 0011 |
| M+H | 605.2402 | 13.428 | 0012 |
| M+H | 605.2566 | 15.006 | 0013 |
| M+H | 606.2361 | 11.426 | 0014 |
| M+H | 606.2526 | 14.205 | 0015 |
| M+H | 606.2723 | 13.012 | 0016 |
| M+H | 607.2474 | 15.689 | 0017 |
| M+H | 607.2674 | 11.526 | 0018,0019 |
| M+H | 607.2668 | 9.75 | 0018,0019 |
| M+H | 608.2340 | 16.108 | 0020 |
| M+H | 608.2625 | 8.464 | 0021,0022 |
| M+H | 608.2627 | 13.785 | 0021,0022 |
| M+H | 609.2577 | 10.592 | 0023 |
| M+H | 612.2091 | 15.591 | 0024 |
| M+H | 613.2237 | 13.277 | 0025 |
| M+H | 614.2188 | 10.031 | 0026 |
| M+H | 615.2612 | 9.774 | 0027 |
| M+H | 615.2971 | 16.06 | 0028 |
| M+H | 616.2568 | 11.374 | 0029,0030 |
| M+H | 616.2568 | 13.321 | 0029,0030 |
| M+H | 616.2932 | 15.304 | 0031 |
| M+H | 617.2502 | 4.746 | 0032 |
| M+H | 617.2744 | 12.366 | 0033 |
| M+H | 617.2885 | 16.593 | 0034 |
| M+H | 618.2721 | 9.301 | 0035 |
| M+H | 619.2563 | 13.929 | 0036 |
| M+H | 619.2672 | 15.57 | 0037 |
| M+H | 620.2879 | 13.437 | 0038 |
| M+H | 621.2138 | 6.67 | 0039 |
| M+H | 621.2832 | 12.01 | 0040 |
| M+H | 622.2130 | 11.933 | 0041 |
| M+H | 622.2496 | 16.515 | 0042 |
| M+H | 622.2780 | 7.202 | 0043 |
| M+H | 623.2308 | 13.484 | 0044 |
| M+H | 623.2473 | 15.311 | 0045 |
| M+H | 624.2292 | 15.451 | 0046 |
| M+H | 624.2429 | 14.614 | 0047,0048 |
| M+H | 624.2463 | 11.886 | 0047,0048 |

(continued)

| [Table 8-11-1] Compound that may be contained in mixture 2-1-m1E01-1 | | | |
|---|---|---|---|
| Ion species | m/z [M+H]+ or [M-H]- | Retention time of MS peak (min) | No. of assigned compound described with 2-1-m1E01-1-omitted |
| M+H | 624.2625 | 13.106 | 0049 |
| M+H | 625.2382 | 15.927 | 0050,0051 |
| M+H | 625.2416 | 8.741 | 0050,0051 |
| M+H | 625.2578 | 11.003 | 0052 |
| M+H | 626.2532 | 13.849 | 0053 |
| M+H | 627.2397 | 13.696 | 0054 |
| M+H | 629.2764 | 13.734 | 0055 |
| M+H | 630.1993 | 15.764 | 0056 |
| M+H | 630.2720 | 13.143 | 0057,0058 |
| M+H | 630.2720 | 13.864 | 0057,0058 |
| M+H | 631.2150 | 13.343 | 0059 |
| M+H | 631.2677 | 11.317 | 0060 |
| M+H | 633.2521 | 13.563 | 0061,0062 |
| M+H | 633.2520 | 13.374 | 0061,0062 |
| M+H | 633.2716 | 14.467 | 0063 |
| M+H | 634.2480 | 11.96 | 0064,0065 |
| M+H | 634.2475 | 13.432 | 0064,0065 |
| M+H | 634.2671 | 9.008 | 0066 |
| M+H | 634.3038 | 13.977 | 0067 |
| M+H | 635.2328 | 14.147 | 0068 |
| M+H | 635.2511 | 12.175 | 0069 |
| M+H | 635.2623 | 8.541 | 0070,0071 |
| M+H | 635.2633 | 11.801 | 0070,0071 |
| M+H | 635.2987 | 12.644 | 0072 |
| M+H | 636.2580 | 8.677 | 0073 |
| M-H | 634.2656 | 12.232 | 0074 |
| M+H | 636.2944 | 14.767 | 0075 |
| M+H | 637.2784 | 7.649 | 0076,0077 |
| M+H | 637.2820 | 15.977 | 0077 |
| M+H | 638.2614 | 12.305 | 0078 |
| M+H | 638.2727 | 6.265 | 0079,0080 |
| M+H | 638.2732 | 13.679 | 0079,0080 |
| M+H | 639.2080 | 13.772 | 0081 |
| M+H | 639.2730 | 16.457 | 0082,0083 |
| M+H | 639.2729 | 13.952 | 0082,0083 |
| M+H | 640.2233 | 11.835 | 0084 |
| M+H | 640.2645 | 2.785 | 0085 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M-H | 639.1899 | 13.962 | 0086 |
| M+H | 641.2196 | 8.866 | 0087,0088 |
| M+H | 641.2230 | 10.333 | 0088 |
| M+H | 641.2558 | 14.228 | 0089 |
| M+H | 642.2030 | 12.216 | 0090 |
| M+H | 642.2364 | 11.957 | 0091 |
| M+H | 642.2537 | 13.775 | 0092 |
| M+H | 643.2356 | 13.231 | 0093,0094 |
| M+H | 643.2377 | 7.732 | 0093,0094 |
| M+H | 643.2488 | 12.529 | 0095 |
| M-H | 641.2424 | 13.177 | 0096 |
| M+H | 643.2925 | 14.254 | 0097 |
| M+H | 644.2337 | 16.628 | 0098,0099 |
| M+H | 644.2342 | 14.19 | 0098,0099 |
| M+H | 644.2441 | 14.617 | 0100 |
| M+H | 644.2550 | 11.413 | 0101 |
| M+H | 644.2882 | 13.631 | 0102,0103 |
| M+H | 644.2882 | 14.417 | 0102,0103 |
| M+H | 645.2724 | 13.218 | 0104 |
| M+H | 646.2675 | 13.269 | 0105 |
| M+H | 647.2673 | 14.041 | 0106 |
| M+H | 647.3240 | 13.84 | 0107 |
| M-H | 646.2461 | 13.262 | 0108 |
| M-H | 646.2661 | 11.991 | 0109 |
| M+H | 648.3194 | 11.86 | 0110 |
| M+H | 649.2051 | 13.977 | 0111 |
| M+H | 649.2485 | 14.632 | 0112 |
| M+H | 649.2781 | 11.247 | 0113,0114 |
| M+H | 649.2785 | 12.209 | 0113,0114 |
| M+H | 650.2735 | 8.304 | 0115 |
| M+H | 651.2287 | 14.213 | 0116,0117,0118 |
| M+H | 651.2281 | 12.474 | 0116,0117,0118 |
| M+H | 651.2290 | 14.466 | 0116,0117,0118 |
| M+H | 651.2417 | 13.656 | 0119,0120 |
| M+H | 651.2420 | 13.6 | 0119,0120 |
| M+H | 652.2236 | 10.728 | 0121,0122,0123 |
| M+H | 652.2250 | 13.632 | 0121,0122,0123 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 652.2240 | 12.204 | 0121,0122,0123 |
| M+H | 652.2366 | 9.267 | 0124 |
| M-H | 650.2407 | 11.679 | 0125,0126 |
| M+H | 652.2581 | 11.875 | 0125,0126 |
| M+H | 652.2776 | 12.844 | 0127 |
| M+H | 653.2194 | 10.452 | 0128,0129 |
| M+H | 653.2183 | 11.541 | 0128,0129 |
| M+H | 653.2529 | 8.874 | 0130,0131 |
| M+H | 653.2528 | 11.908 | 0130,0131 |
| M+H | 653.2884 | 14.564 | 0132 |
| M+H | 654.2150 | 13.065 | 0133 |
| M+H | 654.2382 | 12.453 | 0134 |
| M-H | 652.2401 | 11.828 | 0135 |
| M-H | 653.2049 | 14.456 | 0136 |
| M+H | 655.2551 | 9.641 | 0137,0138,0139 |
| M+H | 655.2555 | 12.568 | 0137,0138,0139 |
| M+H | 655.2563 | 14.291 | 0137,0138,0139 |
| M+H | 656.2508 | 15.108 | 0140,0141 |
| M+H | 656.2506 | 15.04 | 0140,0141 |
| M+H | 657.1808 | 7.692 | 0142 |
| M+H | 657.1986 | 13.964 | 0143 |
| M+H | 657.2451 | 16.235 | 0144 |
| M+H | 657.2526 | 13.711 | 0145 |
| M+H | 657.2632 | 14.054 | 0146 |
| M+H | 657.2753 | 13.771 | 0147 |
| M+H | 658.1797 | 12.749 | 0148,0149 |
| M+H | 658.1802 | 14.836 | 0148,0149 |
| M+H | 658.2134 | 9.525 | 0150 |
| M+H | 658.2785 | 11.692 | 0151 |
| M+H | 658.3053 | 14.115 | 0152 |
| M+H | 659.1758 | 12.929 | 0153 |
| M+H | 659.1987 | 14.047 | 0154 |
| M+H | 659.2732 | 8.667 | 0155 |
| M+H | 660.2138 | 12.676 | 0156 |
| M+H | 660.2281 | 7.964 | 0157 |
| M-H | 658.2659 | 13.085 | 0158 |
| M+H | 661.2087 | 9.524 | 0159 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 661.2293 | 15.88 | 0160 |
| M+H | 661.2493 | 9.866 | 0161 |
| M+H | 661.2832 | 14.638 | 0162 |
| M+H | 661.3396 | 14.526 | 0163 |
| M+H | 662.2064 | 16.226 | 0164 |
| M+H | 662.2440 | 8.667 | 0165 |
| M+H | 662.2653 | 11.634 | 0166 |
| M+H | 662.2982 | 12.531 | 0167 |
| M+H | 663.2395 | 13.714 | 0168,0169 |
| M+H | 663.2393 | 13.842 | 0168,0169 |
| M+H | 663.2605 | 8.749 | 0170,0171,0172 |
| M+H | 663.2629 | 13.539 | 0170,0171,0172 |
| M+H | 663.2630 | 8.323 | 0170,0171,0172 |
| M+H | 663.2942 | 11.598 | 0173,0174 |
| M+H | 663.2936 | 12.759 | 0173,0174 |
| M+H | 664.2344 | 12.765 | 0175 |
| M+H | 664.2599 | 8.539 | 0176 |
| M+H | 664.2780 | 11.327 | 0177 |
| M+H | 665.2301 | 14.402 | 0178 |
| M+H | 665.2435 | 14.723 | 0179,0180,0181 |
| M+H | 665.2431 | 12.835 | 0179,0180,0181 |
| M+H | 665.2431 | 12.981 | 0179,0180,0181 |
| M+H | 665.2730 | 11.792 | 0182,0183 |
| M+H | 665.2771 | 14.247 | 0183 |
| M+H | 665.3146 | 13.899 | 0184 |
| M+H | 666.2395 | 11.053 | 0185,0186,0187 |
| M+H | 666.2399 | 14.201 | 0185,0186,0187 |
| M+H | 666.2398 | 14.162 | 0185,0186,0187 |
| M+H | 666.2531 | 13.405 | 0188 |
| M+H | 666.2732 | 14.275 | 0189,0190 |
| M-H | 664.2563 | 12.135 | 0189,0190 |
| M+H | 666.3293 | 12.046 | 0191 |
| M+H | 667.2345 | 7.801 | 0192,0193 |
| M+H | 667.2353 | 11.236 | 0192,0193 |
| M+H | 667.2688 | 11.325 | 0194 |
| M+H | 667.3040 | 15.292 | 0195 |
| M+H | 667.3248 | 8.963 | 0196 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 668.2538 | 12.71 | 0197 |
| M+H | 669.2032 | 13.151 | 0198 |
| M+H | 669.2193 | 14.255 | 0199,0200 |
| M+H | 669.2194 | 12.611 | 0199,0200 |
| M+H | 669.2336 | 10.95 | 0201,0202 |
| M+H | 669.2385 | 9.753 | 0202 |
| M+H | 669.2694 | 15.608 | 0203 |
| M+H | 669.2833 | 14.071 | 0204 |
| M+H | 670.1916 | 14.054 | 0205 |
| M+H | 670.1985 | 11.099 | 0206 |
| M+H | 670.2143 | 10.627 | 0207 |
| M+H | 670.2324 | 12.486 | 0208,0209,0210 |
| M-H | 668.2149 | 9.674 | 0208,0209,0210 |
| M-H | 668.2171 | 13.091 | 0208,0209,0210 |
| M+H | 670.2469 | 11.847 | 0211,0212 |
| M+H | 670.2482 | 12.269 | 0211,0212 |
| M+H | 671.2013 | 14.949 | 0213 |
| M+H | 671.2090 | 15.098 | 0214 |
| M+H | 671.2180 | 13.829 | 0215 |
| M+H | 671.2297 | 9.703 | 0216,0217,0218,0219 |
| M+H | 671.2292 | 10.755 | 0216,0217,0218,0219 |
| M+H | 671.2297 | 11.534 | 0216,0217,0218,0219 |
| M+H | 671.2295 | 8.144 | 0216,0217,0218,0219 |
| M+H | 671.2441 | 6.789 | 0220,0221 |
| M+H | 671.2492 | 15.858 | 0221 |
| M+H | 671.2711 | 16.753 | 0222 |
| M+H | 671.2931 | 15.16 | 0223 |
| M+H | 672.1963 | 15.351 | 0224 |
| M+H | 672.2244 | 8.249 | 0225,0226 |
| M+H | 672.2247 | 7.801 | 0225,0226 |
| M+H | 672.2438 | 9.946 | 0227 |
| M+H | 672.2939 | 12.152 | 0228 |
| M+H | 673.2202 | 9.721 | 0229 |
| M+H | 673.2288 | 14.22 | 0230 |
| M+H | 673.2380 | 7.975 | 0231 |
| M+H | 673.2466 | 10.231 | 0232,0233 |
| M+H | 673.2466 | 9.863 | 0232,0233 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 673.2701 | 13.212 | 0234 |
| M+H | 674.2292 | 13.101 | 0235 |
| M+H | 674.2595 | 14.101 | 0236,0237 |
| M+H | 674.2596 | 14.308 | 0236,0237 |
| M+H | 675.1748 | 14.556 | 0238 |
| M+H | 675.2551 | 10.474 | 0239,0240,0241 |
| M+H | 675.2550 | 12.988 | 0239,0240,0241 |
| M+H | 675.2550 | 13.261 | 0239,0240,0241 |
| M+H | 675.3554 | 14.923 | 0242 |
| M+H | 676.1716 | 14.895 | 0243 |
| M-H | 674.1739 | 13.025 | 0244 |
| M+H | 676.2043 | 12.513 | 0245 |
| M+H | 676.2499 | 14.854 | 0246 |
| M+H | 676.2598 | 8.741 | 0247 |
| M+H | 676.2685 | 11.852 | 0248 |
| M+H | 676.2805 | 12.101 | 0249 |
| M+H | 677.1861 | 12.892 | 0250,0251,0252 |
| M-H | 675.1700 | 14.264 | 0250,0251,0252 |
| M+H | 677.1854 | 9.924 | 0250,0251,0252 |
| M+H | 677.2808 | 15.328 | 0253,0254 |
| M+H | 677.2761 | 7.415 | 0253,0254 |
| M+H | 677.3091 | 12.11 | 0255 |
| M+H | 677.3345 | 13.832 | 0256 |
| M+H | 678.1812 | 9.669 | 0257 |
| M+H | 678.2003 | 16.356 | 0258,0259 |
| M+H | 678.2038 | 12.769 | 0258,0259 |
| M+H | 678.2753 | 8.99 | 0260 |
| M+H | 679.2212 | 12.764 | 0261,0262 |
| M+H | 679.2229 | 9.759 | 0261,0262 |
| M+H | 679.2394 | 10.099 | 0263 |
| M+H | 679.2593 | 15.291 | 0264,0265,0266,0267 |
| M+H | 679.2603 | 13.298 | 0264,0265,0266,0267 |
| M+H | 679.2603 | 13.51 | 0264,0265,0266,0267 |
| M+H | 679.2603 | 13.361 | 0264,0265,0266,0267 |
| M+H | 679.2890 | 11.288 | 0268 |
| M+H | 680.2189 | 11.007 | 0269,0270 |
| M+H | 680.2189 | 12.772 | 0269,0270 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M-H | 678.2191 | 13.353 | 0271 |
| M+H | 680.2552 | 14.747 | 0272,0273,0274,0275 |
| M+H | 680.2552 | 14.707 | 0272,0273,0274,0275 |
| M+H | 680.2550 | 9.19 | 0272,0273,0274,0275 |
| M+H | 680.2553 | 11.782 | 0272,0273,0274,0275 |
| M+H | 680.2891 | 12.638 | 0276,0277 |
| M+H | 680.2878 | 14.258 | 0276,0277 |
| M+H | 680.3451 | 12.463 | 0278 |
| M+H | 681.2115 | 14.338 | 0279,0280,0281 |
| M+H | 681.2160 | 11.017 | 0280,0281 |
| M+H | 681.2143 | 12.233 | 0279,0280,0281 |
| M+H | 681.2395 | 14.192 | 0282,0283 |
| M+H | 681.2385 | 11.957 | 0282,0283 |
| M+H | 681.2497 | 16.001 | 0284,0285 |
| M+H | 681.2511 | 15.944 | 0284,0285 |
| M+H | 682.2342 | 10.1 | 0286 |
| M+H | 682.2657 | 7.407 | 0287,0288 |
| M+H | 682.2720 | 11.504 | 0287,0288 |
| M+H | 683.2174 | 13.712 | 0289 |
| M+H | 683.2293 | 14.933 | 0290,0291,0292 |
| M+H | 683.2346 | 12.982 | 0291,0292 |
| M+H | 683.2347 | 13.134 | 0291,0292 |
| M+H | 683.2485 | 10.451 | 0293 |
| M+H | 683.2677 | 14.492 | 0294,0295 |
| M+H | 683.2677 | 14.457 | 0294,0295 |
| M+H | 683.2846 | 16.053 | 0296 |
| M+H | 683.3054 | 14.211 | 0297 |
| M+H | 684.2308 | 13.271 | 0298,0299 |
| M+H | 684.2301 | 11.62 | 0298,0299 |
| M+H | 684.2445 | 14.248 | 0300,0301,0302,0303 |
| M+H | 684.2507 | 11.496 | 0301,0302,0303 |
| M+H | 684.2504 | 11.319 | 0301,0302,0303 |
| M+H | 684.2490 | 13.193 | 0301,0302,0303 |
| M+H | 684.3199 | 12.146 | 0304 |
| M+H | 685.1804 | 13.319 | 0305 |
| M+H | 685.2166 | 15.392 | 0306 |
| M+H | 685.2449 | 11.165 | 0307,0308,0309 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 685.2451 | 7.032 | 0307,0308,0309 |
| M+H | 685.2450 | 8.038 | 0307,0308,0309 |
| M+H | 685.2569 | 11.914 | 0310 |
| M+H | 685.2644 | 15.052 | 0311 |
| M+H | 686.1753 | 11.492 | 0312 |
| M+H | 686.2115 | 15.838 | 0313 |
| M+H | 686.2403 | 8.405 | 0314,0315 |
| M+H | 686.2397 | 6.999 | 0314,0315 |
| M+H | 686.3088 | 12.745 | 0316 |
| M+H | 687.1922 | 13.326 | 0317,0318 |
| M+H | 687.1933 | 9.382 | 0317,0318 |
| M+H | 687.2088 | 14.537 | 0319,0320 |
| M+H | 687.2097 | 12.598 | 0319,0320 |
| M+H | 688.1912 | 14.745 | 0321 |
| M+H | 688.2044 | 14.065 | 0322,0323 |
| M+H | 688.2052 | 12.658 | 0322,0323 |
| M+H | 688.2254 | 11.356 | 0324,0325 |
| M+H | 688.2252 | 11.211 | 0324,0325 |
| M+H | 688.2389 | 10.03 | 0326,0327 |
| M+H | 688.2445 | 13.605 | 0327 |
| M+H | 688.2754 | 13.856 | 0328 |
| M+H | 688.2895 | 11.747 | 0329 |
| M+H | 689.2003 | 15.3 | 0330,0331,0332 |
| M+H | 689.2035 | 8.353 | 0330,0331,0332 |
| M+H | 689.2040 | 8.23 | 0331,0332 |
| M+H | 689.2198 | 10.881 | 0333,0334 |
| M+H | 689.2200 | 11.682 | 0333,0334 |
| M+H | 689.2881 | 15.208 | 0335 |
| M+H | 690.2063 | 13.413 | 0336 |
| M+H | 690.2137 | 6.977 | 0337 |
| M+H | 690.2229 | 12.686 | 0338 |
| M+H | 690.2551 | 14.182 | 0339 |
| M+H | 690.2760 | 9.257 | 0340 |
| M-H | 688.2790 | 12.612 | 0341 |
| M+H | 691.2016 | 13.309 | 0342 |
| M+H | 691.2235 | 14.904 | 0343 |
| M+H | 691.2354 | 15.343 | 0344,0345 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 691.2345 | 12.095 | 0344,0345 |
| M+H | 691.2497 | 13.04 | 0346 |
| M+H | 691.2961 | 15.767 | 0347,0348,0349 |
| M+H | 691.2961 | 15.823 | 0347,0348,0349 |
| M+H | 691.2973 | 16.18 | 0347,0348,0349 |
| M+H | 692.2308 | 12.932 | 0350,0351 |
| M+H | 692.2299 | 12.206 | 0350,0351 |
| M+H | 692.2450 | 14.942 | 0352,0353 |
| M+H | 692.2509 | 13.531 | 0353,0354 |
| M+H | 692.2510 | 13.892 | 0353,0354 |
| M+H | 692.2762 | 10.405 | 0355 |
| M+H | 692.2910 | 8.673 | 0356 |
| M+H | 693.1656 | 14.754 | 0357 |
| M+H | 693.2394 | 17.299 | 0358 |
| M+H | 693.2557 | 10.003 | 0359 |
| M+H | 693.2755 | 13.905 | 0360,0361,0362,0363 |
| M+H | 693.2751 | 14.205 | 0360,0361,0362,0363 |
| M+H | 693.2751 | 15.199 | 0360,0361,0362,0363 |
| M+H | 693.2756 | 14.55 | 0360,0361,0362,0363 |
| M+H | 694.1615 | 15.056 | 0364 |
| M+H | 694.1808 | 13.122 | 0365 |
| M+H | 694.2339 | 12.71 | 0366,0367 |
| M-H | 692.2166 | 11.49 | 0366,0367 |
| M+H | 694.2608 | 12.581 | 0368 |
| M+H | 694.2705 | 9.516 | 0369 |
| M+H | 694.3607 | 12.996 | 0370 |
| M+H | 695.1765 | 12.972 | 0371 |
| M+H | 695.2288 | 11.324 | 0372 |
| M+H | 695.2550 | 12.466 | 0373,0374 |
| M+H | 695.2546 | 12.87 | 0373,0374 |
| M+H | 695.2685 | 15.362 | 0375 |
| M+H | 696.1951 | 7.023 | 0376 |
| M+H | 696.2503 | 9.309 | 0377,0378 |
| M+H | 696.2502 | 14.658 | 0377,0378 |
| M+H | 696.2872 | 13.542 | 0379,0380 |
| M+H | 696.2831 | 8.611 | 0379,0380 |
| M+H | 696.3392 | 12.061 | 0381 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 697.1911 | 12.412 | 0382 |
| M+H | 697.2060 | 14.432 | 0383 |
| M-H | 695.1977 | 12.924 | 0384,0385 |
| M+H | 697.2146 | 12.875 | 0384,0385 |
| M+H | 697.2305 | 10.166 | 0386 |
| M+H | 697.2519 | 6.966 | 0387,0388 |
| M+H | 697.2513 | 13.142 | 0387,0388 |
| M+H | 697.2812 | 10.162 | 0389 |
| M+H | 697.3398 | 14.879 | 0390 |
| M+H | 698.2095 | 11.829 | 0391,0392 |
| M+H | 698.2095 | 12.119 | 0391,0392 |
| M-H | 696.2121 | 9.54 | 0393,0394 |
| M+H | 698.2297 | 8.992 | 0394 |
| M+H | 698.2459 | 8.595 | 0395 |
| M+H | 698.2600 | 13.976 | 0396,0397,0398,0399,0400 |
| M+H | 698.2650 | 11.719 | 0397,0398,0399,0400 |
| M+H | 698.2650 | 13.701 | 0397,0398,0399,0400 |
| M+H | 698.2660 | 13.387 | 0397,0398,0399,0400 |
| M+H | 698.2651 | 12.017 | 0397,0398,0399,0400 |
| M+H | 699.1964 | 13.941 | 0401 |
| M+H | 699.2141 | 13.186 | 0402 |
| M+H | 699.2246 | 8.407 | 0403,0404 |
| M+H | 699.2225 | 13.519 | 0403,0404 |
| M+H | 699.2444 | 11.544 | 0405,0406 |
| M+H | 699.2459 | 15.637 | 0405,0406 |
| M+H | 699.2611 | 12.559 | 0407,0408,0409 |
| M+H | 699.2616 | 11.701 | 0407,0408,0409 |
| M+H | 699.2608 | 11.909 | 0407,0408,0409 |
| M+H | 700.2195 | 8.642 | 0410 |
| M+H | 700.2394 | 14.418 | 0411,0412,0413 |
| M+H | 700.2441 | 10.998 | 0412,0413 |
| M+H | 700.2421 | 11.165 | 0411,0412,0413 |
| M+H | 700.2581 | 14.088 | 0414,0415 |
| M+H | 700.2603 | 9.679 | 0415 |
| M-H | 699.2074 | 13.208 | 0416,0417 |
| M+H | 701.2257 | 15.484 | 0416,0417 |
| M+H | 701.2398 | 11.577 | 0418,0419,0420,0421 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 701.2402 | 7.966 | 0418,0419,0420,0421 |
| M+H | 701.2437 | 15.187 | 0419,0420,0421 |
| M+H | 701.2396 | 14.364 | 0418,0419,0420,0421 |
| M+H | 702.2206 | 13.528 | 0422,0423 |
| M+H | 702.2199 | 13.443 | 0422,0423 |
| M+H | 702.2341 | 6.084 | 0424,0425 |
| M+H | 702.2409 | 13.771 | 0425,0426,0427 |
| M+H | 702.2398 | 14.739 | 0425,0426,0427 |
| M+H | 702.2405 | 11.798 | 0425,0426,0427 |
| M+H | 702.2544 | 12.675 | 0428 |
| M+H | 702.2907 | 14.502 | 0429 |
| M+H | 702.3106 | 12.79 | 0430 |
| M+H | 703.1714 | 13.487 | 0431 |
| M+H | 703.2359 | 7.104 | 0432,0433,0434,0435 |
| M+H | 703.2352 | 8.96 | 0432,0433,0434,0435 |
| M+H | 703.2357 | 8.312 | 0432,0433,0434,0435 |
| M-H | 701.2202 | 10.397 | 0432,0433,0434,0435 |
| M+H | 703.2488 | 12.894 | 0436 |
| M+H | 704.1856 | 11.64 | 0437 |
| M+H | 704.2220 | 13.908 | 0438 |
| M+H | 704.2291 | 10.927 | 0439 |
| M+H | 704.2694 | 13.451 | 0440 |
| M-H | 703.1525 | 13.81 | 0441 |
| M+H | 705.1810 | 8.42 | 0442,0443 |
| M+H | 705.1846 | 10.314 | 0443 |
| M+H | 705.2000 | 14.69 | 0444 |
| M+H | 705.2178 | 13.824 | 0445 |
| M+H | 705.2396 | 15.466 | 0446 |
| M+H | 705.2694 | 16.03 | 0447 |
| M+H | 705.3131 | 16.275 | 0448,0449 |
| M+H | 705.3125 | 16.221 | 0448,0449 |
| M+H | 706.1649 | 11.919 | 0450 |
| M-H | 704.1821 | 11.932 | 0451,0452 |
| M+H | 706.1998 | 11.853 | 0451,0452 |
| M+H | 706.2153 | 11.467 | 0453,0454 |
| M+H | 706.2157 | 11.756 | 0453,0454 |
| M+H | 706.2344 | 13.737 | 0455 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 706.2448 | 11.8 | 0456 |
| M+H | 707.1940 | 10.906 | 0457 |
| M+H | 707.1998 | 15.776 | 0457,0458,0459,0460 |
| M+H | 707.2007 | 15.438 | 0458,0459,0460 |
| M-H | 705.1815 | 15.43 | 0457,0458,0459,0460 |
| M+H | 707.2109 | 12.377 | 0461 |
| M+H | 707.2527 | 13.818 | 0462 |
| M+H | 707.2602 | 15.056 | 0463 |
| M+H | 707.2913 | 15.233 | 0464,0465 |
| M+H | 707.2913 | 15.279 | 0464,0465 |
| M+H | 708.1964 | 15.775 | 0466,0467,0468 |
| M+H | 708.1962 | 13.286 | 0466,0467,0468 |
| M+H | 708.1964 | 15.862 | 0466,0467,0468 |
| M+H | 708.2069 | 8.945 | 0469,0470 |
| M-H | 706.1936 | 12.911 | 0469,0470 |
| M+H | 708.2283 | 13.051 | 0471 |
| M+H | 708.2488 | 14.082 | 0472,0473 |
| M+H | 708.2502 | 13.407 | 0472,0473 |
| M+H | 709.1923 | 13.182 | 0474 |
| M+H | 709.2252 | 12.321 | 0475 |
| M+H | 709.2305 | 12.822 | 0476 |
| M+H | 709.2701 | 13.037 | 0477,0478 |
| M+H | 709.2691 | 13.181 | 0477,0478 |
| M+H | 709.2846 | 8.464 | 0479 |
| M+H | 710.2292 | 11.002 | 0480 |
| M-H | 708.2228 | 14.075 | 0481 |
| M+H | 710.3021 | 14.089 | 0482,0483,0484 |
| M+H | 710.3023 | 8.651 | 0482,0483,0484 |
| M+H | 710.3023 | 13.614 | 0482,0483,0484 |
| M+H | 711.2266 | 16.52 | 0485,0486 |
| M+H | 711.2294 | 13.472 | 0485,0486 |
| M+H | 711.2464 | 10.198 | 0487 |
| M+H | 711.2642 | 10.774 | 0488 |
| M+H | 711.2857 | 13.178 | 0489 |
| M+H | 711.2967 | 6.32 | 0490 |
| M+H | 712.1714 | 13.631 | 0491 |
| M+H | 712.2270 | 9.373 | 0492 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M-H | 710.2274 | 11.931 | 0493 |
| M+H | 712.2809 | 12.238 | 0494,0495,0496,0497 |
| M-H | 710.2633 | 11.454 | 0494,0495,0496,0497 |
| M+H | 712.2812 | 12.541 | 0494,0495,0496,0497 |
| M-H | 710.2635 | 12.133 | 0494,0495,0496,0497 |
| M+H | 713.1679 | 13.531 | 0498 |
| M+H | 713.2114 | 14.483 | 0499 |
| M+H | 713.2397 | 7.251 | 0500,0501,0502,0503 |
| M+H | 713.2392 | 12.053 | 0500,0501,0502,0503 |
| M+H | 713.2405 | 8.904 | 0500,0501,0502,0503 |
| M+H | 713.2426 | 13.211 | 0500,0501,0502,0503 |
| M+H | 713.2616 | 15.688 | 0504 |
| M+H | 713.2764 | 13.753 | 0505,0506 |
| M+H | 713.2774 | 12.42 | 0505,0506 |
| M+H | 714.2349 | 8.25 | 0507 |
| M+H | 714.2553 | 14.198 | 0508,0509,0510 |
| M+H | 714.2603 | 11.409 | 0509,0510 |
| M+H | 714.2584 | 11.278 | 0509,0510 |
| M+H | 714.2763 | 9.998 | 0511 |
| M-H | 713.1725 | 13.564 | 0512,0513 |
| M+H | 715.1917 | 13.949 | 0512,0513 |
| M+H | 715.2045 | 13.029 | 0514,0515 |
| M+H | 715.2040 | 13.188 | 0514,0515 |
| M+H | 715.2374 | 8.082 | 0516 |
| M-H | 713.2264 | 11.111 | 0516,0517 |
| M+H | 715.2557 | 6.872 | 0518,0519,0520 |
| M+H | 715.2559 | 12.817 | 0518,0519,0520 |
| M+H | 715.2599 | 13.854 | 0520 |
| M+H | 715.3116 | 14.679 | 0521 |
| M+H | 716.1859 | 12.222 | 0522 |
| M+H | 716.2006 | 9.609 | 0523 |
| M+H | 716.2197 | 11.567 | 0524,0525 |
| M-H | 714.2019 | 11.617 | 0524,0525 |
| M-H | 714.2224 | 12.797 | 0526 |
| M+H | 716.2556 | 15.805 | 0527,0528,0529 |
| M+H | 716.2567 | 14.136 | 0527,0528,0529 |
| M+H | 716.2559 | 12.292 | 0527,0528,0529 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 717.1809 | 13.08 | 0530 |
| M-H | 715.1973 | 8.697 | 0531,0532 |
| M+H | 717.2161 | 6.76 | 0531,0532 |
| M+H | 717.2342 | 14.525 | 0533 |
| M+H | 717.2504 | 15.281 | 0534 |
| M+H | 717.2650 | 12.319 | 0535 |
| M+H | 718.2005 | 12.127 | 0536 |
| M-H | 716.1998 | 11.848 | 0537 |
| M+H | 718.2503 | 13.033 | 0538,0539 |
| M+H | 718.2488 | 12.806 | 0538,0539 |
| M-H | 717.1665 | 14.28 | 0540 |
| M+H | 719.2109 | 14.601 | 0541,0542,0543,0544,0545 |
| M+H | 719.2171 | 13.438 | 0542,0543,0544,0545,0546 |
| M+H | 719.2153 | 14.882 | 0541,0542,0543,0544,0545,0546 |
| M+H | 719.2156 | 15.521 | 0542,0543,0544,0545,0546 |
| M+H | 719.2156 | 15.091 | 0542,0543,0544,0545,0546 |
| M+H | 719.2154 | 14.996 | 0541,0542,0543,0544,0545,0546 |
| M+H | 719.2326 | 12.254 | 0547 |
| M+H | 719.2436 | 13.023 | 0548 |
| M+H | 719.2555 | 9.767 | 0549 |
| M+H | 719.2912 | 10.406 | 0550 |
| M+H | 719.3290 | 16.683 | 0551 |
| M+H | 720.2113 | 13.192 | 0552,0553 |
| M+H | 720.2111 | 12.835 | 0552,0553 |
| M+H | 720.2307 | 11.959 | 0554,0555 |
| M+H | 720.2281 | 11.996 | 0554,0555 |
| M+H | 720.2449 | 13.266 | 0556 |
| M+H | 720.2868 | 13.691 | 0557 |
| M-H | 719.1275 | 15.604 | 0558 |
| M-H | 719.1429 | 13.898 | 0559 |
| M+H | 721.2076 | 15.736 | 0560 |
| M+H | 721.2166 | 15.526 | 0561,0562 |
| M+H | 721.2145 | 12.962 | 0561,0562 |
| M+H | 721.2261 | 11.375 | 0563,0564 |
| M+H | 721.2241 | 13.436 | 0563,0564 |
| M+H | 721.3068 | 15.738 | 0565 |
| M+H | 722.1421 | 12.869 | 0566 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 722.1765 | 11.81 | 0567 |
| M+H | 722.2118 | 14.886 | 0568 |
| M+H | 722.2385 | 10.987 | 0569 |
| M+H | 722.2665 | 13.798 | 0570 |
| M-H | 721.1450 | 10.268 | 0571 |
| M+H | 723.2063 | 13.326 | 0572 |
| M-H | 721.2106 | 15.417 | 0573 |
| M+H | 723.2352 | 7.895 | 0574,0575 |
| M+H | 723.2415 | 15.775 | 0575 |
| M+H | 723.2551 | 15.044 | 0576 |
| M+H | 723.2815 | 3.596 | 0577 |
| M+H | 723.2972 | 12.68 | 0578 |
| M+H | 724.1774 | 12.586 | 0579 |
| M+H | 724.1910 | 9.421 | 0580 |
| M+H | 724.2049 | 11.951 | 0581 |
| M+H | 724.2451 | 13.94 | 0582,0583 |
| M+H | 724.2444 | 12.749 | 0582,0583 |
| M+H | 724.3173 | 14.155 | 0584,0585 |
| M+H | 724.3144 | 9.747 | 0584,0585 |
| M+H | 725.1710 | 9.249 | 0586,0587 |
| M+H | 725.1728 | 15.237 | 0586,0587 |
| M+H | 725.1909 | 15.102 | 0588,0589,0590 |
| M+H | 725.1910 | 14.897 | 0588,0589,0590 |
| M+H | 725.1887 | 8.026 | 0588,0589,0590 |
| M+H | 725.2396 | 10.99 | 0591,0592 |
| M+H | 725.2471 | 14.292 | 0592 |
| M+H | 725.2618 | 10.643 | 0593 |
| M+H | 725.3017 | 13.732 | 0594 |
| M+H | 726.1685 | 15.517 | 0595 |
| M+H | 726.2062 | 13.688 | 0596,0597 |
| M+H | 726.2063 | 9.197 | 0596,0597 |
| M-H | 724.2424 | 12.463 | 0598 |
| M+H | 726.2656 | 13.003 | 0599 |
| M+H | 726.2968 | 7.844 | 0600,0601 |
| M+H | 726.2974 | 13.636 | 0600,0601 |
| M+H | 727.2018 | 11.683 | 0602,0603,0604,0605 |
| M+H | 727.2012 | 13.105 | 0602,0603,0604,0605 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 727.2011 | 10.14 | 0602,0603,0604,0605 |
| M+H | 727.2014 | 13.822 | 0602,0603,0604,0605 |
| M+H | 727.2218 | 16.57 | 0606,0607 |
| M+H | 727.2218 | 16.62 | 0606,0607 |
| M+H | 727.2407 | 9.563 | 0608 |
| M+H | 727.2561 | 11.513 | 0609,0610 |
| M+H | 727.2555 | 12.562 | 0609,0610 |
| M+H | 727.2775 | 16.129 | 0611 |
| M+H | 727.2971 | 16.226 | 0612 |
| M+H | 728.1969 | 11.36 | 0613,0614 |
| M+H | 728.1970 | 12.486 | 0613,0614 |
| M-H | 726.2258 | 11.535 | 0615 |
| M+H | 728.2756 | 11.95 | 0616,0617 |
| M+H | 728.2761 | 12.178 | 0616,0617 |
| M+H | 728.2921 | 9.052 | 0618 |
| M+H | 729.1927 | 13.705 | 0619 |
| M-H | 727.1877 | 14.559 | 0620 |
| M+H | 729.2351 | 11.198 | 0621,0622 |
| M+H | 729.2370 | 10.616 | 0621,0622 |
| M+H | 729.2711 | 7.324 | 0623,0624 |
| M+H | 729.2762 | 13.283 | 0623,0624 |
| M+H | 730.2153 | 13.051 | 0625 |
| M+H | 730.2317 | 14.894 | 0626,0627,0628 |
| M+H | 730.2356 | 11.881 | 0627,0628 |
| M+H | 730.2371 | 11.079 | 0627,0628 |
| M+H | 730.2517 | 9.745 | 0629 |
| M+H | 730.2721 | 13.036 | 0630 |
| M+H | 730.2922 | 11.625 | 0631 |
| M+H | 731.2269 | 14.214 | 0632,0633 |
| M+H | 731.2269 | 14.037 | 0632,0633 |
| M+H | 731.2310 | 9.116 | 0633,0634 |
| M+H | 731.2518 | 16.005 | 0635 |
| M+H | 731.2658 | 14.412 | 0636 |
| M+H | 731.2869 | 8.842 | 0637 |
| M+H | 731.3065 | 14.815 | 0638 |
| M+H | 732.2175 | 12.676 | 0639 |
| M+H | 732.2479 | 12.37 | 0640,0641 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 732.2467 | 16.779 | 0640,0641 |
| M+H | 732.2694 | 14.481 | 0642 |
| M+H | 733.1589 | 13.532 | 0643 |
| M+H | 733.1832 | 13.203 | 0644 |
| M+H | 733.1957 | 10.712 | 0645,0646 |
| M-H | 731.1819 | 14.711 | 0645,0646 |
| M+H | 733.2316 | 14.969 | 0647,0648 |
| M+H | 733.2317 | 15.377 | 0647,0648 |
| M+H | 733.2412 | 14.058 | 0649 |
| M+H | 733.2526 | 12.76 | 0650 |
| M+H | 733.2602 | 12.402 | 0651 |
| M+H | 733.2709 | 16.295 | 0652 |
| M+H | 733.3068 | 15.778 | 0653 |
| M+H | 734.1541 | 13.463 | 0654 |
| M-H | 732.1787 | 12.693 | 0655,0656 |
| M-H | 732.1787 | 13.157 | 0655,0656 |
| M-H | 732.1927 | 11.572 | 0657,0658 |
| M+H | 734.2096 | 10.827 | 0657,0658 |
| M+H | 734.2278 | 13.771 | 0659 |
| M-H | 732.2325 | 13.605 | 0660 |
| M+H | 734.3169 | 13.137 | 0661 |
| M+H | 735.1625 | 15.778 | 0662 |
| M+H | 735.1810 | 13.338 | 0663 |
| M-H | 733.1737 | 9.435 | 0664,0665 |
| M+H | 735.1916 | 9.396 | 0664,0665 |
| M-H | 733.1918 | 15.036 | 0666,0667,0668 |
| M+H | 735.2116 | 15.126 | 0667,0668 |
| M+H | 735.2109 | 13.567 | 0667,0668 |
| M+H | 735.2324 | 15.952 | 0669,0670 |
| M+H | 735.2280 | 6.754 | 0669,0670 |
| M+H | 735.2498 | 15.351 | 0671 |
| M+H | 736.2062 | 14.688 | 0672 |
| M+H | 736.2276 | 15.374 | 0673 |
| M-H | 734.2235 | 12.884 | 0674 |
| M+H | 736.2574 | 11.458 | 0675 |
| M+H | 737.1901 | 14.099 | 0676 |
| M+H | 737.2017 | 15.829 | 0677,0678 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 737.2017 | 15.88 | 0677,0678 |
| M+H | 737.2091 | 14.991 | 0678,0679,0680 |
| M+H | 737.2066 | 14.547 | 0678,0679,0680 |
| M+H | 737.2817 | 15.34 | 0681 |
| M+H | 738.1910 | 13.008 | 0682 |
| M+H | 738.2238 | 11.417 | 0683,0684,0685,0686 |
| M+H | 738.2238 | 12.19 | 0683,0684,0685,0686 |
| M+H | 738.2190 | 13.469 | 0683,0684,0685,0686 |
| M-H | 736.2039 | 13.78 | 0683,0684,0685,0686 |
| M+H | 738.2612 | 13.271 | 0687 |
| M+H | 738.2971 | 13.526 | 0688 |
| M+H | 738.3329 | 9.914 | 0689 |
| M-H | 737.1179 | 15.946 | 0690 |
| M+H | 739.2149 | 11.09 | 0691,0692,0693 |
| M+H | 739.2168 | 11.857 | 0691,0692,0693 |
| M+H | 739.2164 | 10.106 | 0691,0692,0693 |
| M+H | 739.2364 | 15.896 | 0694,0695 |
| M+H | 739.2372 | 10.139 | 0694,0695 |
| M+H | 739.2915 | 10.72 | 0696 |
| M+H | 739.3175 | 14.275 | 0697 |
| M-H | 738.1341 | 14.138 | 0698 |
| M-H | 738.1488 | 12.867 | 0699 |
| M-H | 738.1827 | 14.894 | 0700 |
| M+H | 740.2142 | 14.169 | 0701 |
| M+H | 740.2190 | 10.46 | 0702,0703 |
| M+H | 740.2203 | 13.56 | 0702,0703 |
| M+H | 740.2315 | 11.164 | 0704 |
| M+H | 740.3116 | 9.203 | 0705 |
| M+H | 741.1480 | 9.437 | 0706,0707 |
| M-H | 739.1370 | 13.993 | 0707 |
| M+H | 741.1677 | 15.365 | 0708 |
| M+H | 741.2177 | 8.76 | 0709,0710,0711 |
| M+H | 741.2169 | 8.401 | 0709,0710,0711 |
| M+H | 741.2207 | 15.676 | 0709,0710,0711 |
| M+H | 741.2721 | 12.02 | 0712 |
| M-H | 739.2786 | 13.223 | 0713 |
| M+H | 741.3114 | 11.752 | 0714 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 742.1629 | 15.659 | 0715,0716 |
| M-H | 740.1486 | 12.907 | 0715,0716 |
| M+H | 742.2117 | 10.58 | 0717,0718 |
| M+H | 742.2117 | 10.658 | 0717,0718 |
| M+H | 742.2320 | 11.086 | 0719 |
| M+H | 742.2467 | 14.377 | 0720 |
| M+H | 742.2607 | 13.077 | 0721 |
| M+H | 742.2894 | 12.722 | 0722 |
| M+H | 743.1807 | 15.344 | 0723,0724 |
| M+H | 743.1810 | 15.472 | 0723,0724 |
| M+H | 743.2184 | 11.969 | 0725 |
| M+H | 743.2524 | 10.627 | 0726 |
| M+H | 744.1782 | 13.991 | 0727 |
| M+H | 744.1965 | 9.823 | 0728,0729,0730 |
| M-H | 742.1799 | 13.733 | 0728,0729,0730 |
| M+H | 744.1964 | 13.785 | 0728,0729,0730 |
| M+H | 744.2514 | 12.358 | 0731,0732 |
| M-H | 742.2317 | 13.845 | 0731,0732 |
| M+H | 744.2675 | 10.184 | 0733 |
| M+H | 744.3074 | 12.062 | 0734 |
| M-H | 743.1592 | 12.381 | 0735,0736 |
| M+H | 745.1765 | 12.293 | 0735,0736 |
| M-H | 743.1824 | 14.082 | 0737 |
| M+H | 745.2645 | 9.072 | 0738,0739 |
| M+H | 745.2674 | 16.066 | 0738,0739 |
| M+H | 746.2267 | 14.961 | 0740,0741 |
| M+H | 746.2259 | 9.928 | 0740,0741 |
| M+H | 746.2469 | 12.904 | 0742 |
| M+H | 746.2832 | 11.74 | 0743 |
| M+H | 747.2107 | 13.699 | 0744 |
| M+H | 747.2280 | 13.931 | 0745 |
| M+H | 747.2474 | 15.836 | 0746,0747,0748 |
| M+H | 747.2473 | 15.415 | 0746,0747,0748 |
| M+H | 747.2474 | 10.115 | 0746,0747,0748 |
| M+H | 747.2671 | 13.586 | 0749 |
| M+H | 747.3229 | 16.185 | 0750 |
| M+H | 748.2012 | 7.046 | 0751 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M-H | 746.1942 | 13.101 | 0752 |
| M+H | 748.2826 | 11.751 | 0753 |
| M+H | 749.1780 | 16.398 | 0754 |
| M+H | 749.2211 | 11.746 | 0755,0756,0757 |
| M+H | 749.2262 | 12.899 | 0756,0757 |
| M+H | 749.2260 | 16.003 | 0756,0757 |
| M+H | 749.3017 | 15.244 | 0758 |
| M+H | 750.2221 | 14.202 | 0759 |
| M+H | 750.2434 | 15.838 | 0760,0761 |
| M-H | 748.2229 | 15.813 | 0760,0761 |
| M+H | 750.2563 | 10.776 | 0762 |
| M+H | 750.2717 | 12.056 | 0763 |
| M+H | 750.3118 | 13.245 | 0764 |
| M+H | 751.1564 | 9.499 | 0765 |
| M-H | 749.1531 | 14.436 | 0766 |
| M+H | 751.2183 | 15.342 | 0767 |
| M-H | 750.1685 | 12.797 | 0768 |
| M+H | 752.2008 | 12.016 | 0769,0770 |
| M+H | 752.2067 | 13.541 | 0770 |
| M+H | 752.2222 | 14.861 | 0771 |
| M+H | 752.2334 | 10.152 | 0772,0773 |
| M+H | 752.2366 | 13.276 | 0772,0773 |
| M-H | 750.2340 | 10.391 | 0774 |
| M+H | 752.2757 | 14.768 | 0775 |
| M+H | 752.3116 | 13.906 | 0776 |
| M+H | 753.1680 | 14.414 | 0777 |
| M+H | 753.1833 | 14.329 | 0778 |
| M+H | 753.2318 | 10.873 | 0779 |
| M+H | 753.2502 | 14.465 | 0780,0781 |
| M+H | 753.2530 | 13.958 | 0780,0781 |
| M-H | 752.1492 | 14.673 | 0782 |
| M+H | 754.1873 | 7.485 | 0783 |
| M+H | 754.2164 | 12.286 | 0784,0785 |
| M+H | 754.2185 | 13.894 | 0784,0785 |
| M-H | 752.2178 | 14.488 | 0786,0787 |
| M+H | 754.2396 | 14.656 | 0786,0787 |
| M-H | 752.2371 | 13.924 | 0788 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 755.1865 | 10 | 0789 |
| M+H | 755.1972 | 11.935 | 0790,0791 |
| M+H | 755.1971 | 14.737 | 0790,0791 |
| M+H | 755.2115 | 12.888 | 0792 |
| M-H | 753.2123 | 13.842 | 0793,0794 |
| M+H | 755.2286 | 12.298 | 0793,0794 |
| M+H | 755.2548 | 16.22 | 0795 |
| M+H | 755.2725 | 15.591 | 0796 |
| M+H | 756.1921 | 12.303 | 0797,0798 |
| M-H | 754.1790 | 12.959 | 0797,0798 |
| M+H | 756.2070 | 14.264 | 0799,0800 |
| M-H | 754.1939 | 12.427 | 0799,0800 |
| M+H | 756.2144 | 13.129 | 0800,0801,0802 |
| M+H | 756.2167 | 13.359 | 0801,0802 |
| M+H | 756.2881 | 13.596 | 0803 |
| M-H | 755.1080 | 16.113 | 0804 |
| M+H | 757.2116 | 11.481 | 0805,0806 |
| M+H | 757.2160 | 15.941 | 0805,0806 |
| M-H | 756.1242 | 14.405 | 0807 |
| M+H | 758.1928 | 15.144 | 0808 |
| M+H | 758.2209 | 10.046 | 0809 |
| M+H | 758.2422 | 14.577 | 0810 |
| M+H | 758.3229 | 12.57 | 0811 |
| M+H | 759.2083 | 13.878 | 0812,0813 |
| M+H | 759.2076 | 13.532 | 0812,0813 |
| M+H | 759.2835 | 16.429 | 0814 |
| M-H | 758.1384 | 13.519 | 0815 |
| M+H | 760.1718 | 14.084 | 0816 |
| M+H | 760.2225 | 6.768 | 0817 |
| M+H | 760.3018 | 11.669 | 0818 |
| M+H | 761.1528 | 12.436 | 0819 |
| M+H | 761.1683 | 13.995 | 0820 |
| M+H | 761.2578 | 10.091 | 0821 |
| M+H | 761.3008 | 14.241 | 0822 |
| M-H | 760.1697 | 14.22 | 0823,0824 |
| M+H | 762.1878 | 11.494 | 0823,0824 |
| M+H | 762.2216 | 13.664 | 0825,0826 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M-H | 760.2083 | 13.57 | 0825,0826 |
| M+H | 763.2026 | 7.124 | 0827 |
| M+H | 763.2418 | 15.293 | 0828,0829 |
| M+H | 763.2418 | 15.235 | 0828,0829 |
| M-H | 762.1851 | 13.855 | 0830,0831 |
| M+H | 764.2047 | 13.875 | 0831 |
| M+H | 764.2713 | 10.391 | 0832 |
| M-H | 763.1835 | 13.865 | 0833 |
| M+H | 765.2014 | 10.915 | 0833,0834 |
| M+H | 766.2157 | 12.544 | 0835 |
| M+H | 766.2530 | 10.875 | 0836,0837,0838 |
| M+H | 766.2528 | 13.744 | 0836,0837,0838 |
| M+H | 766.2529 | 14.334 | 0836,0837,0838 |
| M+H | 766.2724 | 12.29 | 0839 |
| M+H | 766.3277 | 14.349 | 0840 |
| M+H | 767.2119 | 12.707 | 0841,0842 |
| M+H | 767.2157 | 11.737 | 0842 |
| M+H | 767.2676 | 9.654 | 0843 |
| M-H | 766.1641 | 15.296 | 0844 |
| M+H | 768.2315 | 12.478 | 0845,0846 |
| M+H | 768.2316 | 12.102 | 0845,0846 |
| M+H | 768.3066 | 15.458 | 0847 |
| M+H | 769.1618 | 14.558 | 0848 |
| M-H | 767.1821 | 14.605 | 0849 |
| M+H | 769.2279 | 13.979 | 0850,0851 |
| M+H | 769.2320 | 15.362 | 0851 |
| M+H | 769.2483 | 8.621 | 0852 |
| M+H | 769.3067 | 16.194 | 0853 |
| M+H | 770.1628 | 13.729 | 0854 |
| M-H | 768.1589 | 13.31 | 0855 |
| M+H | 770.1967 | 12.854 | 0856 |
| M+H | 770.2069 | 11.741 | 0857 |
| M+H | 770.2265 | 13.808 | 0858 |
| M+H | 771.1627 | 15.274 | 0859,0860 |
| M+H | 771.1628 | 16.326 | 0859,0860 |
| M+H | 771.2225 | 14.209 | 0861,0862,0863 |
| M+H | 771.2266 | 11.908 | 0862,0863 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 771.2272 | 12.249 | 0862,0863 |
| M+H | 772.1576 | 13.884 | 0864 |
| M-H | 770.1563 | 13.12 | 0865 |
| M+H | 772.1878 | 10.332 | 0866 |
| M+H | 772.2273 | 15.764 | 0867 |
| M-H | 771.1376 | 14.709 | 0868 |
| M+H | 773.1864 | 12.095 | 0869 |
| M+H | 773.2267 | 16.025 | 0870 |
| M+H | 774.2016 | 13.481 | 0871 |
| M+H | 774.2773 | 14.104 | 0872 |
| M+H | 775.1883 | 15.726 | 0873,0874 |
| M+H | 775.1844 | 14.193 | 0873,0874 |
| M+H | 775.2791 | 16.499 | 0875 |
| M-H | 774.1141 | 15.107 | 0876 |
| M-H | 775.1798 | 13.996 | 0877 |
| M+H | 778.2167 | 13.768 | 0878 |
| M+H | 778.2900 | 10.232 | 0879 |
| M+H | 779.2077 | 11.878 | 0880 |
| M+H | 779.2327 | 11.072 | 0881 |
| M-H | 777.2542 | 14.094 | 0882 |
| M+H | 780.2707 | 12.074 | 0883 |
| M+H | 781.1425 | 13.042 | 0884 |
| M+H | 781.1962 | 13.959 | 0885 |
| M+H | 781.2131 | 7.123 | 0886 |
| M+H | 781.2275 | 12.222 | 0887 |
| M-H | 780.1947 | 12.671 | 0888 |
| M+H | 782.2445 | 9.911 | 0889,0890 |
| M-H | 780.2281 | 12.88 | 0889,0890 |
| M+H | 783.1730 | 14.15 | 0891,0892,0893 |
| M+H | 783.1735 | 14.254 | 0891,0892,0893 |
| M+H | 783.1735 | 14.337 | 0891,0892,0893 |
| M+H | 783.2060 | 12.829 | 0894 |
| M+H | 783.2183 | 9.811 | 0895 |
| M+H | 784.2077 | 12.537 | 0896 |
| M+H | 785.1787 | 15.818 | 0897 |
| M+H | 786.1727 | 17.096 | 0898 |
| M+H | 786.2214 | 11.579 | 0899 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 787.1687 | 15.519 | 0900 |
| M-H | 785.1721 | 14.652 | 0901 |
| M+H | 787.2044 | 15.178 | 0902 |
| M+H | 787.2161 | 14.362 | 0903 |
| M-H | 785.2407 | 12.267 | 0904 |
| M+H | 787.2785 | 15.915 | 0905 |
| M+H | 788.1682 | 12.974 | 0906 |
| M-H | 786.1877 | 13.16 | 0907 |
| M-H | 787.1140 | 16.725 | 0908 |
| M+H | 789.1542 | 15.386 | 0909,0910 |
| M-H | 787.1328 | 14.846 | 0909,0910 |
| M+H | 789.2013 | 10.131 | 0911 |
| M+H | 789.2218 | 16.145 | 0912 |
| M-H | 788.1507 | 13.97 | 0913 |
| M+H | 790.1990 | 15.551 | 0914 |
| M+H | 790.2288 | 12.346 | 0915 |
| M+H | 791.1633 | 13.133 | 0916,0917 |
| M-H | 789.1460 | 13.268 | 0916,0917 |
| M+H | 791.1834 | 15.837 | 0918,0919 |
| M+H | 791.1780 | 10.397 | 0918,0919 |
| M+H | 792.1639 | 13.746 | 0920 |
| M+H | 792.2326 | 14.787 | 0921 |
| M+H | 794.1929 | 14.547 | 0922,0923 |
| M+H | 794.1936 | 14.689 | 0922,0923 |
| M+H | 794.2845 | 10.343 | 0924 |
| M+H | 795.1894 | 13.436 | 0925 |
| M+H | 795.2648 | 14.322 | 0926,0927 |
| M+H | 795.2645 | 14.167 | 0926,0927 |
| M+H | 797.1196 | 14.304 | 0928 |
| M+H | 797.2225 | 12.317 | 0929 |
| M+H | 797.2319 | 15.474 | 0930 |
| M+H | 798.1840 | 14.115 | 0931 |
| M+H | 798.2393 | 9.68 | 0932 |
| M+H | 798.2781 | 12.736 | 0933 |
| M+H | 799.1724 | 14.925 | 0934 |
| M+H | 799.1955 | 16.336 | 0935 |
| M-H | 797.1926 | 14.586 | 0936 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 800.1893 | 17.615 | 0937 |
| M+H | 800.2008 | 12.642 | 0938 |
| M+H | 801.1749 | 14.843 | 0939 |
| M-H | 800.1656 | 13.766 | 0940 |
| M-H | 800.2037 | 13.518 | 0941 |
| M+H | 803.1983 | 15.299 | 0942 |
| M+H | 803.2727 | 16.016 | 0943 |
| M+H | 804.1631 | 17.248 | 0944 |
| M-H | 802.1648 | 14.389 | 0945 |
| M+H | 805.1297 | 16.422 | 0946 |
| M+H | 805.1791 | 8.854 | 0947,0948 |
| M+H | 805.1793 | 13.472 | 0947,0948 |
| M+H | 806.1748 | 10.086 | 0949 |
| M+H | 806.1943 | 15.686 | 0950,0951 |
| M-H | 804.1792 | 13.26 | 0950,0951 |
| M+H | 806.2092 | 13.818 | 0952 |
| M+H | 806.2227 | 12.43 | 0953 |
| M+H | 806.2836 | 14.466 | 0954 |
| M+H | 807.1441 | 15.512 | 0955 |
| M-H | 806.1206 | 15.652 | 0956 |
| M-H | 806.1393 | 13.974 | 0957,0958 |
| M-H | 806.1392 | 14.049 | 0957,0958 |
| M+H | 808.2271 | 14.905 | 0959 |
| M+H | 809.2041 | 14.363 | 0960 |
| M+H | 810.1879 | 14.627 | 0961,0962 |
| M+H | 810.1885 | 14.727 | 0961,0962 |
| M+H | 814.2056 | 17.986 | 0963 |
| M-H | 812.2568 | 12.784 | 0964 |
| M+H | 816.1838 | 17.078 | 0965 |
| M+H | 816.2385 | 13.907 | 0966 |
| M-H | 816.1595 | 13.866 | 0967 |
| M+H | 818.1995 | 14.599 | 0968 |
| M-H | 816.1993 | 13.165 | 0969 |
| M+H | 819.1949 | 15.476 | 0970 |
| M+H | 819.2164 | 15.437 | 0971 |
| M-H | 818.1602 | 13.736 | 0972 |
| M+H | 821.1785 | 16.241 | 0973 |

(continued)

| [Table 8-11-1] Compound that may be contained in mixture 2-1-m1E01-1 | | | |
|---|---|---|---|
| Ion species | m/z $[M+H]^+$ or $[M-H]^-$ | Retention time of MS peak (min) | No. of assigned compound described with 2-1-m1E01-1-omitted |
| M+H | 822.1526 | 17.479 | 0974 |
| M+H | 822.2044 | 13.906 | 0975 |
| M+H | 822.2789 | 14.533 | 0976 |
| M+H | 823.1701 | 15.041 | 0977 |
| M-H | 822.1155 | 15.649 | 0978 |
| M-H | 822.1692 | 13.683 | 0979 |
| M+H | 825.1994 | 14.469 | 0980 |
| M-H | 824.1304 | 14.597 | 0981 |
| M+H | 833.2113 | 16.121 | 0982 |
| M+H | 835.1899 | 14.823 | 0983 |
| M+H | 836.1897 | 18.002 | 0984 |
| M+H | 837.1851 | 10.284 | 0985 |
| M+H | 839.1498 | 16.138 | 0986 |
| M+H | 841.1607 | 15.748 | 0987 |
| M+H | 845.1741 | 13.592 | 0988 |
| M+H | 847.1361 | 14.287 | 0989 |
| M+H | 853.1839 | 15.394 | 0990 |
| M+H | 854.1609 | 17.914 | 0991 |
| M+H | 855.1457 | 16.219 | 0992 |
| M-H | 854.1740 | 14.111 | 0993 |
| M+H | 858.1564 | 14.76 | 0994 |
| M+H | 862.1470 | 15.677 | 0995 |
| M-H | 868.1401 | 13.882 | 0996 |
| M-H | 870.1695 | 14.181 | 0997 |
| M+H | 873.1666 | 16.35 | 0998 |
| M-H | 872.1302 | 15.182 | 0999 |
| M+H | 889.1616 | 16.452 | 1000 |

[Table 408]

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 603.2613 | 12.635 | 0001 |
| M+H | 604.2568 | 11.532 | 0002,0003 |
| M+H | 604.2564 | 10.472 | 0002,0003 |
| M+H | 605.2529 | 13.185 | 0004,0005 |
| M+H | 605.2517 | 9.151 | 0004,0005 |
| M+H | 606.2471 | 10.948 | 0006 |
| M+H | 610.2136 | 13.1 | 0007 |
| M+H | 611.2086 | 10.928 | 0008 |
| M+H | 617.2753 | 12.358 | 0009 |
| M+H | 618.273 | 12.186 | 0010 |
| M+H | 619.2679 | 13.717 | 0011 |
| M-H | 619.218 | 10.945 | 0012 |
| M+H | 621.2519 | 12.744 | 0013 |
| M+H | 622.2317 | 8.863 | 0014 |
| M+H | 622.2478 | 11.691 | 0015 |
| M+H | 622.2669 | 10.526 | 0016 |
| M+H | 623.2432 | 13.222 | 0017 |
| M+H | 623.2624 | 8.85 | 0018,0019 |
| M+H | 623.2619 | 7.347 | 0018,0019 |
| M+H | 624.2295 | 13.693 | 0020 |
| M+H | 624.2579 | 6.093 | 0021,0022 |
| M+H | 624.2579 | 11.114 | 0021,0022 |
| M+H | 625.2529 | 7.974 | 0023 |
| M+H | 628.2042 | 13.183 | 0024 |
| M+H | 629.2189 | 10.72 | 0025 |
| M+H | 630.2142 | 7.573 | 0026 |
| M+H | 631.2565 | 7.965 | 0027 |
| M+H | 631.2938 | 13.823 | 0028 |
| M+H | 632.2523 | 8.824 | 0029,0030 |
| M+H | 632.2523 | 10.056 | 0029,0030 |
| M+H | 632.2885 | 12.85 | 0031 |
| M+H | 633.2471 | 8.735 | 0032 |
| M+H | 633.2701 | 12.451 | 0033 |
| M+H | 633.2843 | 14.322 | 0034 |
| M+H | 634.2677 | 11.577 | 0035 |
| M+H | 635.2512 | 11.453 | 0036 |
| M+H | 635.2635 | 13.087 | 0037 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 636.2832 | 10.987 | 0038 |
| M-H | 635.1962 | 11.164 | 0039 |
| M+H | 637.2786 | 9.365 | 0040 |
| M+H | 638.2087 | 9.333 | 0041 |
| M+H | 638.2455 | 14.276 | 0042 |
| M+H | 638.2738 | 9.999 | 0043 |
| M+H | 639.2262 | 11.041 | 0044 |
| M+H | 639.2426 | 13.1 | 0045 |
| M-H | 638.2059 | 13.031 | 0046 |
| M+H | 640.2381 | 12.152 | 0047,0048 |
| M+H | 640.241 | 9.325 | 0047,0048 |
| M+H | 640.2578 | 10.679 | 0049 |
| M+H | 641.234 | 13.546 | 0050,0051 |
| M+H | 641.2367 | 6.459 | 0050,0051 |
| M+H | 641.2534 | 9.083 | 0052 |
| M+H | 642.2491 | 11.204 | 0053 |
| M+H | 643.2352 | 11.15 | 0054 |
| M+H | 645.2724 | 11.29 | 0055 |
| M+H | 646.1952 | 13.482 | 0056 |
| M+H | 646.2676 | 10.623 | 0057,0058 |
| M+H | 646.269 | 11.332 | 0057,0058 |
| M+H | 647.2099 | 10.846 | 0059 |
| M+H | 647.263 | 8.661 | 0060 |
| M+H | 649.2473 | 10.946 | 0061,0062 |
| M+H | 649.2471 | 11.069 | 0061,0062 |
| M+H | 649.2673 | 12.019 | 0063 |
| M+H | 650.2429 | 9.292 | 0064,0065 |
| M-H | 648.2283 | 10.938 | 0064,0065 |
| M+H | 650.262 | 9.114 | 0066 |
| M+H | 650.2987 | 11.572 | 0067 |
| M+H | 651.2288 | 11.658 | 0068 |
| M+H | 651.2456 | 10.234 | 0069 |
| M+H | 651.2579 | 6.248 | 0070,0071 |
| M+H | 651.2566 | 9.258 | 0070,0071 |
| M+H | 651.2941 | 10.028 | 0072 |
| M+H | 652.2534 | 6.402 | 0073 |
| M-H | 650.2617 | 10.566 | 0074 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 652.2893 | 12.108 | 0075 |
| M+H | 653.2734 | 8.963 | 0076,0077 |
| M+H | 653.2783 | 13.834 | 0077 |
| M+H | 654.257 | 9.738 | 0078 |
| M+H | 654.2688 | 11.059 | 0079,0080 |
| M+H | 654.2728 | 12.889 | 0080 |
| M+H | 655.2034 | 11.329 | 0081 |
| M+H | 655.2684 | 14.148 | 0082,0083 |
| M+H | 655.268 | 11.509 | 0082,0083 |
| M+H | 656.2187 | 9.276 | 0084 |
| M+H | 656.2636 | 9.12 | 0085 |
| M+H | 657.203 | 11.753 | 0086 |
| M+H | 657.2144 | 6.514 | 0087,0088 |
| M+H | 657.2161 | 11.313 | 0087,0088 |
| M+H | 657.2508 | 11.721 | 0089 |
| M+H | 658.1982 | 9.794 | 0090 |
| M+H | 658.2319 | 9.44 | 0091 |
| M+H | 658.2489 | 11.397 | 0092 |
| M+H | 659.2301 | 10.723 | 0093,0094 |
| M+H | 659.2333 | 13.737 | 0094 |
| M+H | 659.2436 | 9.939 | 0095 |
| M+H | 659.2543 | 10.836 | 0096 |
| M+H | 659.2887 | 11.825 | 0097 |
| M+H | 660.2298 | 14.202 | 0098,0099 |
| M+H | 660.2299 | 14.131 | 0098,0099 |
| M+H | 660.2398 | 11.931 | 0100 |
| M+H | 660.2509 | 8.942 | 0101 |
| M+H | 660.2839 | 11.1 | 0102,0103 |
| M+H | 660.283 | 11.89 | 0102,0103 |
| M+H | 661.2641 | 9.088 | 0104 |
| M-H | 660.2454 | 10.758 | 0105 |
| M+H | 663.2629 | 11.536 | 0106 |
| M+H | 663.3195 | 11.373 | 0107 |
| M+H | 664.259 | 10.782 | 0108 |
| M+H | 664.2783 | 9.534 | 0109 |
| M+H | 664.3147 | 9.287 | 0110 |
| M+H | 665.2007 | 11.537 | 0111 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 665.2444 | 12.16 | 0112 |
| M+H | 665.2735 | 8.631 | 0113,0114 |
| M+H | 665.2737 | 9.653 | 0113,0114 |
| M+H | 666.2681 | 5.966 | 0115 |
| M+H | 667.2235 | 10.401 | 0116,0117,0118 |
| M+H | 667.2235 | 11.959 | 0116,0117,0118 |
| M+H | 667.2246 | 12.278 | 0116,0117,0118 |
| M+H | 667.2334 | 11.199 | 0119,0120 |
| M+H | 667.2378 | 11.243 | 0119,0120 |
| M+H | 668.2196 | 10.016 | 0121,0122,0123 |
| M+H | 668.2183 | 8.558 | 0121,0122,0123 |
| M+H | 668.2192 | 10.085 | 0121,0122,0123 |
| M+H | 668.2325 | 11.151 | 0124 |
| M+H | 668.2525 | 9.248 | 0125,0126 |
| M-H | 666.2359 | 9.177 | 0125,0126 |
| M+H | 668.2734 | 10.293 | 0127 |
| M+H | 669.2143 | 8.817 | 0128,0129 |
| M+H | 669.2148 | 12.754 | 0128,0129 |
| M+H | 669.2485 | 6.467 | 0130,0131 |
| M+H | 669.2479 | 9.403 | 0130,0131 |
| M+H | 669.2836 | 12.152 | 0132 |
| M+H | 670.2103 | 10.448 | 0133 |
| M+H | 670.2346 | 6.683 | 0134 |
| M-H | 668.2342 | 9.34 | 0135 |
| M-H | 669.2014 | 12.266 | 0136 |
| M+H | 671.2489 | 13.621 | 0137,0138,0139 |
| M+H | 671.2488 | 12.954 | 0137,0138,0139 |
| M+H | 671.2521 | 11.835 | 0137,0138,0139 |
| M+H | 672.2446 | 12.757 | 0140,0141 |
| M+H | 672.2443 | 10.972 | 0140,0141 |
| M-H | 671.1627 | 12.201 | 0142 |
| M+H | 673.1942 | 11.568 | 0143 |
| M+H | 673.2401 | 14.071 | 0144 |
| M+H | 673.2493 | 14.311 | 0145 |
| M+H | 673.2591 | 11.659 | 0146 |
| M+H | 673.2704 | 11.477 | 0147 |
| M+H | 674.1758 | 10.29 | 0148,0149 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 674.1758 | 12.538 | 0148,0149 |
| M+H | 674.2092 | 9.39 | 0150 |
| M+H | 674.2736 | 9.215 | 0151 |
| M+H | 674.3003 | 11.636 | 0152 |
| M-H | 673.1525 | 10.341 | 0153 |
| M+H | 675.1935 | 11.875 | 0154 |
| M+H | 675.2684 | 6.506 | 0155 |
| M-H | 674.1907 | 10.324 | 0156 |
| M+H | 676.2225 | 9.982 | 0157 |
| M-H | 674.2607 | 10.618 | 0158 |
| M-H | 675.1865 | 7.198 | 0159 |
| M+H | 677.2254 | 13.823 | 0160 |
| M+H | 677.2455 | 10.996 | 0161 |
| M+H | 677.2789 | 12.05 | 0162 |
| M+H | 677.3345 | 11.937 | 0163 |
| M+H | 678.2016 | 14.022 | 0164 |
| M+H | 678.2395 | 6.94 | 0165 |
| M+H | 678.2601 | 9.194 | 0166 |
| M+H | 678.2937 | 10.091 | 0167 |
| M+H | 679.2341 | 11.325 | 0168,0169 |
| M-H | 677.2176 | 8.374 | 0168,0169 |
| M+H | 679.2568 | 11.091 | 0170,0171,0172 |
| M+H | 679.254 | 6.702 | 0170,0171,0172 |
| M+H | 679.2568 | 10.195 | 0170,0171,0172 |
| M+H | 679.289 | 8.976 | 0173,0174 |
| M+H | 679.2891 | 10.222 | 0173,0174 |
| M+H | 680.2297 | 10.143 | 0175 |
| M+H | 680.2525 | 9.406 | 0176 |
| M+H | 680.2727 | 9.147 | 0177 |
| M+H | 681.2272 | 11.819 | 0178 |
| M+H | 681.2397 | 10.965 | 0179,0180,0181 |
| M+H | 681.2394 | 12.511 | 0179,0180,0181 |
| M-H | 679.2219 | 10.924 | 0179,0180,0181 |
| M+H | 681.2677 | 9.266 | 0182,0183 |
| M+H | 681.2721 | 11.844 | 0183 |
| M+H | 681.3101 | 11.49 | 0184 |
| M+H | 682.2349 | 8.786 | 0185,0186,0187 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 682.2344 | 10.624 | 0185,0186,0187 |
| M+H | 682.2345 | 9.335 | 0185,0186,0187 |
| M+H | 682.2488 | 11.123 | 0188 |
| M+H | 682.2684 | 9.523 | 0189,0190 |
| M+H | 682.2672 | 11.763 | 0189,0190 |
| M-H | 680.3075 | 9.469 | 0191 |
| M+H | 683.2297 | 8.885 | 0192,0193 |
| M+H | 683.2302 | 13.317 | 0192,0193 |
| M+H | 683.2636 | 8.766 | 0194 |
| M+H | 683.2994 | 12.787 | 0195 |
| M+H | 683.3203 | 6.632 | 0196 |
| M+H | 684.2498 | 10.178 | 0197 |
| M+H | 685.1984 | 10.872 | 0198 |
| M+H | 685.2142 | 10.591 | 0199,0200 |
| M+H | 685.2138 | 12.091 | 0199,0200 |
| M+H | 685.2249 | 6.336 | 0201 |
| M-H | 683.2175 | 8.098 | 0202 |
| M+H | 685.2658 | 13.503 | 0203 |
| M+H | 685.2785 | 11.658 | 0204 |
| M-H | 684.1691 | 11.532 | 0205 |
| M+H | 686.194 | 8.578 | 0206 |
| M+H | 686.2095 | 10.294 | 0207 |
| M-H | 684.2114 | 8.926 | 0208,0209,0210 |
| M+H | 686.2257 | 11.89 | 0208,0209,0210 |
| M+H | 686.2272 | 10.407 | 0208,0209,0210 |
| M+H | 686.2427 | 9.292 | 0211,0212 |
| M+H | 686.2434 | 9.836 | 0211,0212 |
| M+H | 687.1955 | 12.745 | 0213 |
| M+H | 687.2054 | 12.873 | 0214 |
| M+H | 687.2136 | 11.782 | 0215 |
| M+H | 687.2214 | 8.554 | 0216,0217,0218 |
| M+H | 687.2247 | 6.093 | 0216,0217,0218,0219 |
| M+H | 687.2263 | 8.641 | 0216,0217,0218,0219 |
| M+H | 687.2263 | 8.599 | 0216,0217,0218,0219 |
| M+H | 687.2387 | 9.922 | 0220,0221 |
| M+H | 687.2437 | 13.769 | 0221 |
| M+H | 687.2651 | 14.849 | 0222 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 687.2863 | 12.082 | 0223 |
| M-H | 686.1731 | 13.1 | 0224 |
| M+H | 688.2194 | 5.682 | 0225,0226 |
| M+H | 688.2194 | 5.764 | 0225,0226 |
| M+H | 688.2395 | 12.898 | 0227 |
| M+H | 688.2898 | 9.697 | 0228 |
| M+H | 689.2158 | 10.77 | 0229 |
| M+H | 689.2236 | 11.892 | 0230 |
| M+H | 689.2349 | 6.832 | 0231,0232 |
| M+H | 689.2348 | 14.232 | 0231,0232 |
| M+H | 689.2407 | 13.041 | 0232,0233 |
| M-H | 687.2481 | 10.906 | 0234 |
| M+H | 690.2238 | 10.752 | 0235 |
| M+H | 690.2547 | 11.089 | 0236,0237 |
| M+H | 690.2547 | 11.821 | 0236,0237 |
| M+H | 691.1708 | 12.351 | 0238 |
| M-H | 689.2333 | 8.041 | 0239,0240,0241 |
| M+H | 691.2508 | 10.484 | 0239,0240,0241 |
| M+H | 691.2493 | 12.051 | 0239,0240,0241 |
| M+H | 691.351 | 12.511 | 0242 |
| M+H | 692.1671 | 12.656 | 0243 |
| M+H | 692.1855 | 10.668 | 0244 |
| M+H | 692.1998 | 10.045 | 0245 |
| M+H | 692.2461 | 12.366 | 0246 |
| M+H | 692.2558 | 7.041 | 0247 |
| M+H | 692.2645 | 9.404 | 0248 |
| M+H | 692.2765 | 9.695 | 0249 |
| M+H | 693.1813 | 7.57 | 0250,0251,0252 |
| M+H | 693.1827 | 10.466 | 0250,0251,0252 |
| M+H | 693.1842 | 12.141 | 0250,0251,0252 |
| M+H | 693.2751 | 13.221 | 0253,0254 |
| M-H | 691.2578 | 13.59 | 0253,0254 |
| M+H | 693.3049 | 9.497 | 0255 |
| M+H | 693.3301 | 11.4 | 0256 |
| M+H | 694.1763 | 7.222 | 0257 |
| M+H | 694.196 | 14.188 | 0258,0259 |
| M+H | 694.1992 | 10.464 | 0259 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 694.2711 | 6.122 | 0260 |
| M-H | 693.1973 | 10.458 | 0261,0262 |
| M+H | 695.2183 | 9.73 | 0261,0262 |
| M+H | 695.2347 | 10.915 | 0263 |
| M+H | 695.2549 | 11.516 | 0264,0265,0266,0267 |
| M-H | 693.2371 | 11.277 | 0264,0265,0266,0267 |
| M-H | 693.2369 | 13.018 | 0264,0265,0266,0267 |
| M+H | 695.2553 | 13.601 | 0264,0265,0266,0267 |
| M-H | 693.2667 | 8.711 | 0268 |
| M+H | 696.2141 | 8.597 | 0269,0270 |
| M+H | 696.2134 | 10.403 | 0269,0270 |
| M+H | 696.2261 | 8.473 | 0271 |
| M+H | 696.2508 | 12.435 | 0272,0273,0274,0275 |
| M+H | 696.2508 | 11.224 | 0272,0273,0274,0275 |
| M+H | 696.2509 | 9.704 | 0272,0273,0274,0275 |
| M+H | 696.2508 | 9.38 | 0272,0273,0274,0275 |
| M-H | 694.267 | 10.041 | 0276,0277 |
| M-H | 694.2656 | 11.623 | 0276,0277 |
| M+H | 696.341 | 9.893 | 0278 |
| M-H | 695.1919 | 9.718 | 0279,0280,0281 |
| M+H | 697.2064 | 11.977 | 0279,0280,0281 |
| M+H | 697.2086 | 4.05 | 0279,0280,0281 |
| M+H | 697.2345 | 9.921 | 0282,0283 |
| M-H | 695.2151 | 10.462 | 0282,0283 |
| M+H | 697.245 | 9 | 0284,0285 |
| M+H | 697.2477 | 13.836 | 0284,0285 |
| M+H | 698.2291 | 8.013 | 0286 |
| M+H | 698.2649 | 9.256 | 0287,0288 |
| M+H | 698.2645 | 9.41 | 0287,0288 |
| M+H | 699.212 | 9.73 | 0289 |
| M+H | 699.2245 | 7.297 | 0290,0291,0292 |
| M-H | 697.2121 | 10.913 | 0290,0291,0292 |
| M+H | 699.2302 | 10.988 | 0291,0292 |
| M+H | 699.2442 | 8.5 | 0293 |
| M+H | 699.2628 | 12.025 | 0294,0295 |
| M-H | 697.2439 | 7.59 | 0294,0295 |
| M+H | 699.2808 | 14.034 | 0296 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 699.3007 | 11.848 | 0297 |
| M+H | 700.225 | 10.337 | 0298,0299 |
| M+H | 700.2251 | 9.448 | 0298,0299 |
| M+H | 700.2392 | 9.918 | 0300,0301,0302,0303 |
| M+H | 700.2449 | 9.146 | 0301,0302,0303 |
| M+H | 700.2425 | 11.803 | 0300,0301,0302,0303 |
| M+H | 700.2452 | 10.846 | 0301,0302,0303 |
| M-H | 698.2984 | 9.625 | 0304 |
| M+H | 701.1754 | 11.035 | 0305 |
| M-H | 699.1932 | 13.275 | 0306 |
| M+H | 701.2404 | 9.305 | 0307,0308,0309 |
| M-H | 699.2226 | 6.23 | 0307,0308,0309 |
| M+H | 701.2401 | 6.757 | 0307,0308,0309 |
| M+H | 701.2509 | 9.446 | 0310 |
| M+H | 701.2594 | 12.91 | 0311 |
| M+H | 702.1711 | 8.937 | 0312 |
| M+H | 702.2049 | 9.369 | 0313 |
| M+H | 702.2361 | 6.302 | 0314,0315 |
| M+H | 702.2362 | 11.183 | 0314,0315 |
| M+H | 702.3055 | 10.32 | 0316 |
| M-H | 701.1696 | 10.994 | 0317,0318 |
| M+H | 703.1884 | 11.085 | 0317,0318 |
| M+H | 703.2047 | 10.635 | 0319,0320 |
| M+H | 703.2053 | 10.821 | 0319,0320 |
| M+H | 704.1864 | 12.485 | 0321 |
| M+H | 704.2001 | 11.733 | 0322,0323 |
| M-H | 702.1888 | 9.266 | 0323 |
| M+H | 704.2197 | 9.095 | 0324,0325 |
| M+H | 704.2207 | 9.238 | 0324,0325 |
| M+H | 704.2341 | 8.178 | 0326,0327 |
| M+H | 704.2369 | 9.695 | 0326,0327 |
| M+H | 704.2705 | 11.511 | 0328 |
| M+H | 704.2829 | 9.318 | 0329 |
| M+H | 705.1957 | 13.096 | 0330,0331,0332 |
| M+H | 705.1989 | 5.984 | 0331,0332 |
| M+H | 705.2005 | 12.156 | 0331,0332 |
| M+H | 705.2156 | 9.123 | 0333,0334 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 705.2151 | 8.728 | 0333,0334 |
| M+H | 705.2835 | 12.876 | 0335 |
| M-H | 704.1846 | 11.072 | 0336 |
| M+H | 706.2105 | 10.878 | 0337 |
| M+H | 706.2204 | 10.37 | 0338 |
| M+H | 706.2494 | 11.9 | 0339 |
| M+H | 706.2712 | 7.486 | 0340 |
| M+H | 706.2917 | 10.266 | 0341 |
| M+H | 707.1972 | 6.513 | 0342 |
| M+H | 707.2186 | 12.778 | 0343 |
| M+H | 707.2302 | 13.291 | 0344,0345 |
| M-H | 705.2124 | 9.679 | 0344,0345 |
| M+H | 707.2456 | 10.536 | 0346 |
| M-H | 705.2745 | 13.786 | 0347,0348,0349 |
| M-H | 705.2747 | 8.458 | 0347,0348,0349 |
| M-H | 705.2733 | 14.167 | 0347,0348,0349 |
| M+H | 708.2236 | 8.87 | 0350,0351 |
| M+H | 708.2284 | 9.676 | 0350,0351 |
| M-H | 706.2222 | 12.446 | 0352 |
| M+H | 708.2416 | 12.46 | 0352,0353 |
| M+H | 708.2519 | 2.977 | 0354 |
| M+H | 708.2716 | 9.292 | 0355 |
| M+H | 708.2864 | 7.101 | 0356 |
| M+H | 709.1609 | 12.609 | 0357 |
| M+H | 709.2336 | 11.024 | 0358 |
| M+H | 709.2491 | 14.876 | 0359 |
| M+H | 709.2706 | 11.866 | 0360,0361,0362,0363 |
| M+H | 709.2705 | 11.945 | 0360,0361,0362,0363 |
| M+H | 709.2663 | 7.178 | 0360,0361,0362,0363 |
| M+H | 709.2673 | 7.204 | 0360,0361,0362,0363 |
| M+H | 710.1565 | 12.838 | 0364 |
| M+H | 710.1765 | 10.812 | 0365 |
| M+H | 710.2301 | 10.367 | 0366,0367 |
| M+H | 710.2297 | 10.883 | 0366,0367 |
| M+H | 710.2503 | 7.617 | 0368 |
| M+H | 710.2633 | 11.608 | 0369 |
| M+H | 710.3568 | 10.432 | 0370 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 711.172 | 10.611 | 0371 |
| M+H | 711.225 | 8.794 | 0372 |
| M+H | 711.2503 | 10.435 | 0373,0374 |
| M-H | 709.2319 | 10.388 | 0373,0374 |
| M+H | 711.2639 | 13.371 | 0375 |
| M-H | 710.1734 | 11.024 | 0376 |
| M+H | 712.2449 | 8.99 | 0377,0378 |
| M-H | 710.2269 | 7.646 | 0377,0378 |
| M+H | 712.2815 | 6.132 | 0379,0380 |
| M+H | 712.2817 | 11.125 | 0379,0380 |
| M-H | 710.3182 | 9.518 | 0381 |
| M+H | 713.1856 | 9.933 | 0382 |
| M+H | 713.202 | 12.064 | 0383 |
| M+H | 713.2087 | 10.662 | 0384,0385 |
| M-H | 711.1921 | 10.626 | 0384,0385 |
| M+H | 713.2251 | 8.542 | 0386 |
| M+H | 713.2463 | 11.566 | 0387,0388 |
| M+H | 713.2406 | 12.272 | 0387,0388 |
| M+H | 713.2774 | 9.827 | 0389 |
| M+H | 713.3352 | 12.472 | 0390 |
| M+H | 714.2046 | 10.605 | 0391,0392 |
| M-H | 712.1868 | 9.352 | 0391,0392 |
| M+H | 714.2188 | 9.783 | 0393,0394 |
| M-H | 712.2079 | 9.063 | 0393,0394 |
| M+H | 714.2424 | 9.967 | 0395 |
| M-H | 712.2373 | 11.596 | 0396 |
| M+H | 714.2607 | 9.584 | 0397,0398,0399,0400 |
| M+H | 714.2606 | 11.391 | 0397,0398,0399,0400 |
| M+H | 714.2594 | 9.929 | 0397,0398,0399,0400 |
| M-H | 712.2441 | 11.158 | 0397,0398,0399,0400 |
| M+H | 715.1915 | 11.715 | 0401 |
| M-H | 713.1907 | 10.897 | 0402 |
| M+H | 715.2194 | 9.207 | 0403,0404 |
| M+H | 715.2188 | 11.76 | 0403,0404 |
| M+H | 715.2389 | 11.989 | 0405,0406 |
| M+H | 715.2388 | 7.949 | 0405,0406 |
| M+H | 715.2554 | 10.065 | 0407,0408,0409 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 715.2549 | 9.695 | 0407,0408,0409 |
| M+H | 715.2567 | 7.042 | 0407,0408,0409 |
| M-H | 714.1977 | 6.344 | 0410 |
| M+H | 716.2299 | 8.984 | 0411 |
| M+H | 716.2387 | 8.897 | 0412,0413 |
| M-H | 714.222 | 9.674 | 0411,0412,0413 |
| M+H | 716.2519 | 11.742 | 0414,0415 |
| M+H | 716.2561 | 7.794 | 0415 |
| M+H | 717.2208 | 11.163 | 0416,0417 |
| M+H | 717.2161 | 5.336 | 0416,0417 |
| M+H | 717.2356 | 5.998 | 0418,0419,0420,0421 |
| M+H | 717.2352 | 9.009 | 0418,0419,0420,0421 |
| M+H | 717.2397 | 11.423 | 0420,0421 |
| M+H | 717.2394 | 13.124 | 0420,0421 |
| M+H | 718.2156 | 11.432 | 0422,0423 |
| M-H | 716.2022 | 9.778 | 0422,0423 |
| M+H | 718.2322 | 11.152 | 0424,0425,0426,0427 |
| M+H | 718.2362 | 9.271 | 0425,0426,0427 |
| M+H | 718.2347 | 11.221 | 0425,0426,0427 |
| M-H | 716.2191 | 9.297 | 0425,0426,0427 |
| M+H | 718.2498 | 10.35 | 0428 |
| M+H | 718.2864 | 12.036 | 0429 |
| M+H | 718.3065 | 10.312 | 0430 |
| M+H | 719.1665 | 11.254 | 0431 |
| M+H | 719.2312 | 6.905 | 0432,0433,0434,0435 |
| M-H | 717.2124 | 10.694 | 0432,0433,0434,0435 |
| M+H | 719.231 | 6.541 | 0432,0433,0434,0435 |
| M-H | 717.2115 | 13.819 | 0432,0433,0434,0435 |
| M+H | 719.2453 | 10.477 | 0436 |
| M+H | 720.1813 | 9.244 | 0437 |
| M+H | 720.2173 | 11.617 | 0438 |
| M+H | 720.2248 | 8.365 | 0439 |
| M-H | 718.2482 | 11.097 | 0440 |
| M-H | 719.147 | 11.613 | 0441 |
| M+H | 721.1763 | 6.058 | 0442,0443 |
| M+H | 721.1793 | 11.365 | 0442,0443 |
| M+H | 721.196 | 12.314 | 0444 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 721.2131 | 11.494 | 0445 |
| M+H | 721.2346 | 10.576 | 0446 |
| M+H | 721.2646 | 14.026 | 0447 |
| M+H | 721.3065 | 14.311 | 0448,0449 |
| M+H | 721.3073 | 6.24 | 0448,0449 |
| M+H | 722.1603 | 9.592 | 0450 |
| M-H | 720.1769 | 9.448 | 0451,0452 |
| M+H | 722.1949 | 9.492 | 0451,0452 |
| M+H | 722.2097 | 9.403 | 0453,0454 |
| M+H | 722.2096 | 9.68 | 0453,0454 |
| M+H | 722.2259 | 7.423 | 0455 |
| M+H | 722.2407 | 9.275 | 0456 |
| M-H | 721.1774 | 13.287 | 0457,0458,0459,0460 |
| M+H | 723.1951 | 12.857 | 0457,0458,0459,0460 |
| M+H | 723.1929 | 10.514 | 0457,0458,0459,0460 |
| M+H | 723.1948 | 12.952 | 0457,0458,0459,0460 |
| M+H | 723.2064 | 9.907 | 0461 |
| M+H | 723.2494 | 11.611 | 0462,0463 |
| M+H | 723.2555 | 12.636 | 0463 |
| M+H | 723.283 | 3.597 | 0464,0465 |
| M+H | 723.2864 | 13.199 | 0464,0465 |
| M+H | 724.1925 | 11.405 | 0466,0467,0468 |
| M-H | 722.173 | 13.632 | 0466,0467,0468 |
| M+H | 724.1911 | 10.952 | 0466,0467,0468 |
| M+H | 724.2023 | 7.549 | 0469,0470 |
| M+H | 724.2056 | 10.509 | 0470 |
| M+H | 724.2237 | 10.623 | 0471 |
| M+H | 724.2452 | 11.468 | 0472,0473 |
| M-H | 722.2269 | 10.943 | 0472,0473 |
| M+H | 725.1903 | 12.683 | 0474 |
| M+H | 725.2208 | 9.788 | 0475 |
| M-H | 723.2127 | 10.55 | 0476 |
| M+H | 725.2644 | 11.001 | 0477,0478 |
| M+H | 725.2647 | 11.039 | 0477,0478 |
| M+H | 725.2769 | 6.332 | 0479 |
| M-H | 724.2056 | 9.778 | 0480 |
| M+H | 726.2363 | 11.823 | 0481 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M-H | 724.2813 | 11.721 | 0482,0483,0484 |
| M+H | 726.2984 | 8.761 | 0482,0483,0484 |
| M-H | 724.2806 | 6.647 | 0482,0483,0484 |
| M+H | 727.2223 | 14.621 | 0485,0486 |
| M+H | 727.2252 | 11.275 | 0486 |
| M+H | 727.2425 | 12.049 | 0487 |
| M-H | 725.2434 | 9.057 | 0488 |
| M+H | 727.2809 | 10.889 | 0489 |
| M+H | 727.2919 | 4.643 | 0490 |
| M+H | 728.1665 | 11.378 | 0491 |
| M+H | 728.22 | 10.513 | 0492 |
| M-H | 726.2226 | 9.513 | 0493 |
| M+H | 728.2764 | 9.047 | 0494,0495,0496,0497 |
| M+H | 728.2769 | 10.13 | 0494,0495,0496,0497 |
| M+H | 728.2762 | 10.68 | 0494,0495,0496,0497 |
| M+H | 728.2757 | 6.806 | 0494,0495,0496,0497 |
| M+H | 729.1627 | 11.226 | 0498 |
| M+H | 729.2069 | 12.334 | 0499 |
| M+H | 729.2347 | 8.664 | 0500,0501,0502,0503 |
| M-H | 727.2166 | 5.405 | 0500,0501 |
| M+H | 729.2354 | 9.622 | 0500,0501,0502,0503 |
| M+H | 729.237 | 10.175 | 0500,0501,0502,0503 |
| M-H | 727.2387 | 13.644 | 0504 |
| M+H | 729.2727 | 10.245 | 0505,0506 |
| M+H | 729.2721 | 7.276 | 0505,0506 |
| M+H | 730.2306 | 5.9 | 0507 |
| M+H | 730.2501 | 11.792 | 0508,0509,0510 |
| M+H | 730.2553 | 9.348 | 0509,0510 |
| M-H | 728.2377 | 9.288 | 0509,0510 |
| M+H | 730.272 | 8.119 | 0511 |
| M-H | 729.1686 | 11.078 | 0512,0513 |
| M+H | 731.1863 | 11.895 | 0512,0513 |
| M+H | 731.1946 | 11.309 | 0514,0515 |
| M+H | 731.1979 | 11.076 | 0514,0515 |
| M-H | 729.2166 | 11.407 | 0516 |
| M+H | 731.2412 | 10.173 | 0517 |
| M+H | 731.2468 | 7.014 | 0518,0519 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 731.2501 | 5.287 | 0518,0519,0520 |
| M+H | 731.2548 | 9.62 | 0520 |
| M+H | 731.3073 | 12.389 | 0521 |
| M-H | 730.1632 | 9.826 | 0522 |
| M+H | 732.196 | 10.909 | 0523 |
| M+H | 732.2149 | 11.249 | 0524,0525 |
| M+H | 732.2141 | 9.071 | 0524,0525 |
| M+H | 732.2356 | 10.388 | 0526 |
| M+H | 732.2513 | 10.252 | 0527,0528,0529 |
| M+H | 732.2515 | 10.456 | 0527,0528,0529 |
| M+H | 732.2514 | 13.65 | 0527,0528,0529 |
| M+H | 733.1767 | 10.823 | 0530 |
| M+H | 733.2093 | 9.39 | 0531,0532 |
| M-H | 731.1927 | 6.331 | 0531,0532 |
| M+H | 733.2295 | 12.155 | 0533 |
| M+H | 733.2456 | 11.393 | 0534 |
| M+H | 733.2617 | 9.842 | 0535 |
| M+H | 734.1966 | 9.796 | 0536 |
| M+H | 734.2139 | 6.338 | 0537 |
| M-H | 732.227 | 10.451 | 0538,0539 |
| M+H | 734.2471 | 7.896 | 0538,0539 |
| M-H | 733.1633 | 12.172 | 0540 |
| M+H | 735.2067 | 7.264 | 0541,0542,0543,0544,0545 |
| M+H | 735.2116 | 11.498 | 0542,0543,0544,0545,0546 |
| M+H | 735.2111 | 12.916 | 0542,0543,0544,0545,0546 |
| M-H | 733.196 | 11.908 | 0542,0543,0544,0545,0546 |
| M-H | 733.193 | 12.38 | 0541,0542,0543,0544,0545,0546 |
| M+H | 735.2144 | 11.965 | 0542,0543,0544,0545,0546 |
| M+H | 735.2275 | 9.948 | 0547 |
| M+H | 735.2404 | 10.574 | 0548 |
| M+H | 735.2506 | 13.651 | 0549 |
| M+H | 735.2878 | 13.289 | 0550 |
| M+H | 735.3243 | 14.101 | 0551 |
| M+H | 736.2065 | 10.561 | 0552,0553 |
| M+H | 736.2068 | 12.337 | 0552,0553 |
| M+H | 736.2277 | 7.266 | 0554,0555 |
| M-H | 734.2089 | 9.853 | 0554,0555 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 736.2403 | 10.363 | 0556 |
| M+H | 736.2812 | 11.432 | 0557 |
| M-H | 735.1241 | 12.829 | 0558 |
| M+H | 737.1572 | 11.549 | 0559 |
| M+H | 737.2023 | 13.694 | 0560 |
| M+H | 737.2121 | 13.371 | 0561,0562 |
| M+H | 737.211 | 13.511 | 0561,0562 |
| M+H | 737.2214 | 9.696 | 0563,0564 |
| M+H | 737.221 | 10.915 | 0563,0564 |
| M+H | 737.3026 | 9.772 | 0565 |
| M+H | 738.1376 | 10.276 | 0566 |
| M+H | 738.1722 | 9.466 | 0567 |
| M+H | 738.208 | 12.66 | 0568 |
| M-H | 736.2191 | 8.496 | 0569 |
| M+H | 738.2611 | 11.523 | 0570 |
| M-H | 737.1382 | 11.776 | 0571 |
| M+H | 739.2022 | 10.711 | 0572 |
| M+H | 739.2251 | 13.207 | 0573,0574 |
| M+H | 739.2308 | 5.661 | 0574,0575 |
| M+H | 739.2371 | 13.799 | 0575 |
| M+H | 739.2508 | 12.798 | 0576 |
| M+H | 739.2823 | 8.698 | 0577 |
| M-H | 737.2747 | 10.173 | 0578 |
| M-H | 738.1533 | 10.334 | 0579 |
| M+H | 740.1852 | 10.009 | 0580 |
| M+H | 740.2007 | 9.915 | 0581 |
| M+H | 740.2409 | 10.479 | 0582,0583 |
| M-H | 738.2246 | 9.152 | 0582,0583 |
| M+H | 740.313 | 7.762 | 0584,0585 |
| M+H | 740.3133 | 7.237 | 0584,0585 |
| M+H | 741.1678 | 13.165 | 0586,0587 |
| M+H | 741.166 | 6.886 | 0586,0587 |
| M-H | 739.1708 | 10.082 | 0588,0589,0590 |
| M+H | 741.1863 | 12.953 | 0588,0589,0590 |
| M+H | 741.1863 | 12.854 | 0588,0589,0590 |
| M+H | 741.2354 | 8.496 | 0591,0592 |
| M+H | 741.2405 | 11.8 | 0592 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 741.2575 | 8.999 | 0593 |
| M+H | 741.2968 | 11.489 | 0594 |
| M+H | 742.1637 | 13.454 | 0595 |
| M+H | 742.2019 | 11.334 | 0596,0597 |
| M+H | 742.2017 | 7.36 | 0596,0597 |
| M+H | 742.2548 | 10.089 | 0598,0599 |
| M+H | 742.2609 | 10.669 | 0599 |
| M-H | 740.2721 | 10.039 | 0600,0601 |
| M+H | 742.2924 | 10.898 | 0600,0601 |
| M+H | 743.197 | 9.611 | 0602,0603,0604,0605 |
| M+H | 743.1969 | 11.188 | 0602,0603,0604,0605 |
| M-H | 741.1797 | 10.875 | 0602,0603,0604,0605 |
| M+H | 743.1953 | 7.85 | 0602,0603,0604,0605 |
| M+H | 743.2161 | 8.98 | 0606,0607 |
| M+H | 743.2198 | 10.785 | 0607 |
| M+H | 743.2377 | 12.178 | 0608 |
| M+H | 743.2515 | 9.173 | 0609,0610 |
| M+H | 743.2505 | 9.327 | 0609,0610 |
| M+H | 743.2727 | 6.919 | 0611 |
| M+H | 743.2905 | 7.468 | 0612 |
| M+H | 744.1915 | 9.228 | 0613,0614 |
| M+H | 744.1925 | 10.006 | 0613,0614 |
| M+H | 744.2297 | 11.754 | 0615 |
| M+H | 744.2713 | 9.919 | 0616,0617 |
| M-H | 742.2534 | 9.888 | 0616,0617 |
| M+H | 744.2877 | 8.679 | 0618 |
| M-H | 743.1713 | 11.324 | 0619 |
| M+H | 745.2014 | 12.46 | 0620 |
| M+H | 745.2274 | 8.835 | 0621 |
| M+H | 745.2347 | 11.564 | 0622 |
| M+H | 745.2711 | 11.021 | 0623,0624 |
| M+H | 745.2727 | 12.766 | 0623,0624 |
| M+H | 746.2114 | 10.811 | 0625 |
| M+H | 746.2292 | 11.31 | 0626,0627,0628 |
| M+H | 746.2301 | 9.564 | 0626,0627,0628 |
| M+H | 746.2286 | 12.719 | 0626,0627,0628 |
| M-H | 744.2303 | 10.573 | 0629 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 746.2685 | 7.054 | 0630 |
| M+H | 746.2865 | 9.274 | 0631 |
| M+H | 747.2226 | 11.751 | 0632,0633 |
| M-H | 745.2085 | 8.815 | 0632,0633 |
| M+H | 747.2263 | 6.921 | 0633,0634 |
| M-H | 745.2324 | 10.781 | 0635 |
| M+H | 747.2614 | 12.053 | 0636 |
| M+H | 747.2823 | 6.569 | 0637 |
| M+H | 747.3022 | 12.532 | 0638 |
| M-H | 746.1939 | 10.348 | 0639 |
| M+H | 748.2473 | 11.398 | 0640,0641 |
| M+H | 748.245 | 8.987 | 0640,0641 |
| M+H | 748.2581 | 14.191 | 0642 |
| M+H | 749.1538 | 11.251 | 0643 |
| M+H | 749.1773 | 10.713 | 0644 |
| M+H | 749.1875 | 12.344 | 0645 |
| M+H | 749.1913 | 8.942 | 0645,0646 |
| M+H | 749.2268 | 11.814 | 0647,0648 |
| M+H | 749.2265 | 12.92 | 0647,0648 |
| M+H | 749.2412 | 10.888 | 0649,0650 |
| M+H | 749.2451 | 10.422 | 0650 |
| M+H | 749.2554 | 9.926 | 0651 |
| M+H | 749.2656 | 14.188 | 0652 |
| M+H | 749.3033 | 13.826 | 0653 |
| M+H | 750.1485 | 11.14 | 0654 |
| M+H | 750.1918 | 10.38 | 0655,0656 |
| M-H | 748.1724 | 10.787 | 0655,0656 |
| M-H | 748.1874 | 9.789 | 0657,0658 |
| M+H | 750.2053 | 9.227 | 0657,0658 |
| M-H | 748.2056 | 11.58 | 0659 |
| M-H | 748.2278 | 8.982 | 0660 |
| M+H | 750.3124 | 10.734 | 0661 |
| M+H | 751.158 | 13.773 | 0662 |
| M+H | 751.1763 | 11.724 | 0663 |
| M-H | 749.1687 | 7.016 | 0664,0665 |
| M+H | 751.1881 | 8.571 | 0664,0665 |
| M-H | 749.1822 | 9.86 | 0666 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 751.2072 | 13.071 | 0667,0668 |
| M+H | 751.2067 | 11.719 | 0667,0668 |
| M+H | 751.228 | 13.913 | 0669,0670 |
| M+H | 751.2236 | 7.407 | 0669,0670 |
| M+H | 751.2448 | 13.117 | 0671 |
| M+H | 752.2014 | 12.488 | 0672 |
| M+H | 752.2234 | 13.199 | 0673 |
| M+H | 752.235 | 10.422 | 0674 |
| M+H | 752.2519 | 8.997 | 0675 |
| M+H | 753.1863 | 12.042 | 0676 |
| M+H | 753.1976 | 13.818 | 0677,0678 |
| M-H | 751.1836 | 12.487 | 0677,0678 |
| M+H | 753.2041 | 11.592 | 0678,0679,0680 |
| M+H | 753.2041 | 11.561 | 0678,0679,0680 |
| M+H | 753.278 | 13.371 | 0681 |
| M-H | 752.1683 | 10.846 | 0682 |
| M+H | 754.2175 | 9.756 | 0683,0684,0685,0686 |
| M-H | 752.1988 | 10.197 | 0683,0684,0685,0686 |
| M+H | 754.2132 | 11.405 | 0683,0684,0685,0686 |
| M-H | 752.1991 | 10.705 | 0683,0684,0685,0686 |
| M-H | 752.2378 | 11.903 | 0687 |
| M+H | 754.2925 | 8.021 | 0688 |
| M+H | 754.3285 | 8.349 | 0689 |
| M-H | 753.1135 | 13.099 | 0690 |
| M+H | 755.2117 | 7.804 | 0691,0692,0693 |
| M-H | 753.194 | 10.45 | 0691,0692,0693 |
| M-H | 753.1928 | 11.269 | 0691,0692,0693 |
| M-H | 753.2129 | 13.93 | 0694,0695 |
| M+H | 755.2326 | 13.946 | 0694,0695 |
| M-H | 753.2705 | 8.512 | 0696 |
| M+H | 755.313 | 12.045 | 0697 |
| M+H | 756.1484 | 11.327 | 0698 |
| M+H | 756.1621 | 10.139 | 0699 |
| M+H | 756.1981 | 12.744 | 0700 |
| M-H | 754.189 | 8.05 | 0701 |
| M-H | 754.2001 | 11.706 | 0702,0703 |
| M+H | 756.2181 | 8.997 | 0702,0703 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 756.2248 | 11.391 | 0704 |
| M+H | 756.3077 | 7.937 | 0705 |
| M+H | 757.1438 | 7.04 | 0706,0707 |
| M-H | 755.1278 | 12.047 | 0706,0707 |
| M+H | 757.1622 | 13.323 | 0708 |
| M+H | 757.2129 | 9.756 | 0709,0710,0711 |
| M+H | 757.212 | 11.091 | 0709,0710,0711 |
| M+H | 757.2155 | 13.509 | 0709,0710,0711 |
| M+H | 757.267 | 9.711 | 0712 |
| M+H | 757.2915 | 10.962 | 0713 |
| M+H | 757.306 | 14.678 | 0714 |
| M+H | 758.158 | 13.615 | 0715,0716 |
| M-H | 756.1435 | 10.531 | 0715,0716 |
| M+H | 758.2076 | 5.116 | 0717,0718 |
| M+H | 758.2083 | 7.866 | 0717,0718 |
| M+H | 758.2303 | 11.614 | 0719 |
| M+H | 758.2423 | 12.222 | 0720 |
| M+H | 758.2512 | 8.752 | 0721 |
| M+H | 758.2872 | 10.638 | 0722 |
| M+H | 759.1779 | 13.243 | 0723,0724 |
| M-H | 757.1582 | 13.229 | 0723,0724 |
| M+H | 759.2188 | 10.647 | 0725 |
| M+H | 759.2467 | 9.071 | 0726 |
| M+H | 760.173 | 11.813 | 0727 |
| M+H | 760.192 | 11.431 | 0728,0729,0730 |
| M+H | 760.1922 | 11.598 | 0728,0729,0730 |
| M+H | 760.1877 | 8.782 | 0728,0729,0730 |
| M+H | 760.2452 | 10.092 | 0731,0732 |
| M+H | 760.2448 | 11.555 | 0731,0732 |
| M+H | 760.2622 | 8.492 | 0733 |
| M+H | 760.3017 | 9.741 | 0734 |
| M+H | 761.1698 | 11.702 | 0735,0736 |
| M+H | 761.1718 | 10.004 | 0735,0736 |
| M-H | 759.1777 | 11.872 | 0737 |
| M+H | 761.2621 | 6.768 | 0738,0739 |
| M+H | 761.2643 | 7.348 | 0738,0739 |
| M-H | 760.207 | 9.194 | 0740,0741 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 762.2274 | 9.734 | 0741 |
| M+H | 762.2427 | 10.659 | 0742 |
| M+H | 762.2796 | 8.247 | 0743 |
| M+H | 763.2064 | 10.232 | 0744 |
| M+H | 763.225 | 11.979 | 0745 |
| M+H | 763.2443 | 13.429 | 0746,0747,0748 |
| M+H | 763.2426 | 13.942 | 0746,0747,0748 |
| M-H | 761.2241 | 13.413 | 0746,0747,0748 |
| M+H | 763.2629 | 11.341 | 0749 |
| M-H | 761.2997 | 14.332 | 0750 |
| M+H | 764.2017 | 11.513 | 0751,0752 |
| M-H | 762.1885 | 10.777 | 0751,0752 |
| M+H | 764.278 | 9.443 | 0753 |
| M+H | 765.1749 | 14.5 | 0754 |
| M+H | 765.2163 | 9.45 | 0755,0756,0757 |
| M-H | 763.2037 | 11.97 | 0755,0756,0757 |
| M+H | 765.2207 | 12.374 | 0756,0757 |
| M+H | 765.2965 | 13.265 | 0758 |
| M+H | 766.2185 | 10.849 | 0759 |
| M+H | 766.239 | 13.739 | 0760,0761 |
| M-H | 764.2194 | 13.729 | 0760,0761 |
| M-H | 764.2355 | 12.053 | 0762 |
| M+H | 766.2681 | 9.659 | 0763 |
| M-H | 764.2901 | 10.807 | 0764 |
| M+H | 767.1535 | 13.134 | 0765 |
| M+H | 767.1673 | 12.342 | 0766 |
| M+H | 767.2163 | 13.53 | 0767 |
| M-H | 766.1638 | 10.517 | 0768 |
| M+H | 768.1965 | 10.63 | 0769,0770 |
| M+H | 768.1992 | 9.672 | 0769,0770 |
| M-H | 766.1989 | 12.653 | 0771 |
| M+H | 768.2318 | 11.116 | 0772,0773 |
| M-H | 766.2146 | 10.368 | 0772,0773 |
| M+H | 768.2468 | 7.991 | 0774 |
| M+H | 768.2718 | 12.433 | 0775 |
| M+H | 768.3079 | 11.775 | 0776 |
| M+H | 769.1592 | 8.282 | 0777 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M-H | 767.162 | 12.153 | 0778 |
| M+H | 769.2279 | 8.654 | 0779 |
| M+H | 769.2455 | 12.134 | 0780,0781 |
| M+H | 769.2493 | 7.11 | 0781 |
| M-H | 768.1467 | 11.679 | 0782 |
| M+H | 770.1825 | 10.447 | 0783 |
| M-H | 768.194 | 10.293 | 0784,0785 |
| M-H | 768.1917 | 11.683 | 0784,0785 |
| M+H | 770.2318 | 12.215 | 0786,0787 |
| M+H | 770.2346 | 11.373 | 0786,0787 |
| M+H | 770.2507 | 11.531 | 0788 |
| M+H | 771.1826 | 12.761 | 0789 |
| M+H | 771.1919 | 4.769 | 0790,0791 |
| M+H | 771.1906 | 12.727 | 0790,0791 |
| M+H | 771.2058 | 9.971 | 0792 |
| M+H | 771.2279 | 11.507 | 0793,0794 |
| M-H | 769.2084 | 11.45 | 0793,0794 |
| M+H | 771.2504 | 14.152 | 0795 |
| M+H | 771.2686 | 13.659 | 0796 |
| M-H | 770.1727 | 10.643 | 0797,0798 |
| M+H | 772.1915 | 10.691 | 0798 |
| M-H | 770.1843 | 12.018 | 0799 |
| M-H | 770.1898 | 10.474 | 0799,0800 |
| M+H | 772.2108 | 10.481 | 0800,0801,0802 |
| M-H | 770.1951 | 11.352 | 0800,0801,0802 |
| M+H | 772.283 | 11.499 | 0803 |
| M-H | 771.1047 | 13.441 | 0804 |
| M+H | 773.2088 | 9.437 | 0805,0806 |
| M+H | 773.2109 | 14.06 | 0805,0806 |
| M+H | 774.1386 | 11.611 | 0807 |
| M+H | 774.189 | 13.031 | 0808 |
| M+H | 774.2116 | 9.431 | 0809 |
| M+H | 774.2376 | 12.302 | 0810 |
| M+H | 774.318 | 10.271 | 0811 |
| M+H | 775.2027 | 11.208 | 0812,0813 |
| M+H | 775.2039 | 10.258 | 0812,0813 |
| M+H | 775.2803 | 9.846 | 0814 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M-H | 774.1337 | 11.153 | 0815 |
| M+H | 776.1673 | 11.917 | 0816 |
| M+H | 776.2176 | 4.957 | 0817 |
| M+H | 776.2968 | 9.35 | 0818 |
| M+H | 777.1492 | 10.209 | 0819 |
| M-H | 775.1456 | 11.772 | 0820 |
| M+H | 777.2584 | 14.446 | 0821 |
| M+H | 777.2971 | 12.02 | 0822 |
| M+H | 778.1823 | 11.983 | 0823,0824 |
| M+H | 778.1828 | 9.856 | 0823,0824 |
| M+H | 778.2177 | 11.481 | 0825,0826 |
| M-H | 776.2038 | 10.339 | 0825,0826 |
| M+H | 779.2005 | 11.718 | 0827 |
| M+H | 779.2377 | 13.379 | 0828,0829 |
| M+H | 779.2385 | 12.833 | 0828,0829 |
| M+H | 780.1961 | 11.693 | 0830,0831 |
| M-H | 778.1836 | 10.168 | 0830,0831 |
| M+H | 780.2653 | 7.641 | 0832 |
| M+H | 781.193 | 9.21 | 0833 |
| M+H | 781.2018 | 13.007 | 0834 |
| M+H | 782.2113 | 9.957 | 0835 |
| M+H | 782.249 | 12.029 | 0836,0837,0838 |
| M+H | 782.2482 | 11.624 | 0836,0837,0838 |
| M+H | 782.2446 | 7.237 | 0836,0837,0838 |
| M+H | 782.2683 | 10.009 | 0839 |
| M+H | 782.3239 | 12.314 | 0840 |
| M-H | 781.1906 | 10.437 | 0841,0842 |
| M+H | 783.2105 | 9.458 | 0842 |
| M+H | 783.2638 | 8.153 | 0843 |
| M-H | 782.1614 | 12.407 | 0844 |
| M+H | 784.2259 | 10.467 | 0845,0846 |
| M+H | 784.2283 | 10.148 | 0845,0846 |
| M+H | 784.3027 | 6.576 | 0847 |
| M+H | 785.1578 | 12.505 | 0848 |
| M-H | 783.1795 | 9.176 | 0849 |
| M+H | 785.2228 | 10.551 | 0850,0851 |
| M-H | 783.2078 | 13.414 | 0850,0851 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 785.244 | 12.029 | 0852 |
| M-H | 783.2837 | 14.329 | 0853 |
| M+H | 786.1591 | 11.311 | 0854 |
| M-H | 784.155 | 11.106 | 0855 |
| M+H | 786.1917 | 10.544 | 0856 |
| M+H | 786.2027 | 9.463 | 0857 |
| M+H | 786.2196 | 6.707 | 0858 |
| M+H | 787.158 | 14.415 | 0859,0860 |
| M+H | 787.1591 | 13.295 | 0859,0860 |
| M+H | 787.2171 | 11.968 | 0861,0862,0863 |
| M+H | 787.2236 | 11.122 | 0862,0863 |
| M+H | 787.2241 | 9.84 | 0862,0863 |
| M+H | 788.1543 | 9.394 | 0864 |
| M+H | 788.1692 | 10.842 | 0865 |
| M+H | 788.1835 | 8.575 | 0866 |
| M+H | 788.2236 | 13.701 | 0867 |
| M-H | 787.1338 | 12.626 | 0868 |
| M+H | 789.1824 | 9.882 | 0869 |
| M+H | 789.2233 | 13.982 | 0870 |
| M+H | 790.1984 | 11.385 | 0871 |
| M+H | 790.2679 | 7.5 | 0872 |
| M+H | 791.1842 | 13.715 | 0873,0874 |
| M-H | 789.164 | 13.702 | 0873,0874 |
| M+H | 791.273 | 14.626 | 0875 |
| M-H | 790.1107 | 12.173 | 0876 |
| M+H | 793.1937 | 11.797 | 0877 |
| M+H | 794.2118 | 11.63 | 0878 |
| M+H | 794.285 | 8.426 | 0879 |
| M+H | 795.2026 | 9.472 | 0880 |
| M+H | 795.2287 | 9.543 | 0881 |
| M+H | 795.2681 | 11.969 | 0882 |
| M+H | 796.2586 | 9.841 | 0883 |
| M+H | 797.1386 | 10.984 | 0884 |
| M+H | 797.1917 | 11.872 | 0885 |
| M-H | 795.1892 | 12.057 | 0886 |
| M-H | 795.2053 | 10.004 | 0887 |
| M-H | 796.1884 | 10.449 | 0888 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M-H | 796.223 | 10.412 | 0889,0890 |
| M+H | 798.2415 | 10.788 | 0889,0890 |
| M+H | 799.1705 | 11.855 | 0891,0892,0893 |
| M-H | 797.1507 | 11.842 | 0891,0892 |
| M+H | 799.1738 | 12.965 | 0893 |
| M+H | 799.2014 | 10.573 | 0894 |
| M+H | 799.2131 | 12.97 | 0895 |
| M+H | 800.2017 | 10.386 | 0896 |
| M+H | 801.1735 | 13.923 | 0897 |
| M+H | 802.1696 | 14.837 | 0898 |
| M+H | 802.219 | 11.268 | 0899 |
| M+H | 803.1645 | 11.489 | 0900 |
| M+H | 803.1872 | 12.58 | 0901 |
| M+H | 803.1987 | 10.307 | 0902 |
| M+H | 803.2126 | 12.137 | 0903 |
| M-H | 801.2362 | 10.01 | 0904 |
| M+H | 803.2741 | 14.128 | 0905 |
| M+H | 804.1639 | 10.896 | 0906 |
| M+H | 804.2025 | 10.888 | 0907 |
| M+H | 805.1307 | 14.497 | 0908 |
| M+H | 805.1448 | 10.195 | 0909,0910 |
| M+H | 805.1497 | 13.475 | 0909,0910 |
| M+H | 805.1972 | 7.785 | 0911 |
| M+H | 805.2169 | 14.087 | 0912 |
| M-H | 804.1447 | 11.345 | 0913 |
| M+H | 806.1956 | 13.519 | 0914 |
| M+H | 806.2235 | 10.064 | 0915 |
| M+H | 807.1599 | 7.894 | 0916,0917 |
| M+H | 807.1595 | 11.054 | 0916,0917 |
| M+H | 807.179 | 13.84 | 0918,0919 |
| M+H | 807.176 | 8.837 | 0918,0919 |
| M-H | 806.1398 | 11.663 | 0920 |
| M+H | 808.2286 | 12.599 | 0921 |
| M+H | 810.1883 | 12.378 | 0922,0923 |
| M-H | 808.172 | 12.131 | 0922,0923 |
| M+H | 810.2813 | 7.935 | 0924 |
| M+H | 811.1838 | 11.325 | 0925 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M-H | 809.2425 | 12.069 | 0926,0927 |
| M+H | 811.2642 | 12.119 | 0927 |
| M+H | 813.1154 | 12.149 | 0928 |
| M+H | 813.2188 | 10.125 | 0929 |
| M+H | 813.2264 | 13.457 | 0930 |
| M+H | 814.181 | 11.65 | 0931 |
| M+H | 814.2342 | 7.779 | 0932 |
| M+H | 814.2735 | 10.544 | 0933 |
| M+H | 815.1641 | 11.56 | 0934 |
| M+H | 815.1902 | 14.559 | 0935 |
| M+H | 815.2076 | 12.473 | 0936 |
| M+H | 816.1856 | 15.741 | 0937 |
| M-H | 814.1791 | 10.466 | 0938 |
| M+H | 817.1693 | 13.301 | 0939 |
| M+H | 818.1785 | 9.319 | 0940 |
| M+H | 818.2178 | 11.268 | 0941 |
| M+H | 819.1929 | 13.369 | 0942 |
| M-H | 817.2517 | 14.238 | 0943 |
| M+H | 820.16 | 15.074 | 0944 |
| M+H | 820.1803 | 11.95 | 0945 |
| M+H | 821.126 | 14.679 | 0946 |
| M+H | 821.1757 | 12.362 | 0947,0948 |
| M+H | 821.1757 | 12.854 | 0947,0948 |
| M+H | 822.17 | 7.608 | 0949 |
| M+H | 822.1901 | 13.632 | 0950,0951 |
| M+H | 822.1901 | 13.671 | 0950,0951 |
| M+H | 822.2042 | 11.806 | 0952 |
| M+H | 822.2185 | 10.153 | 0953 |
| M+H | 822.2795 | 12.483 | 0954 |
| M+H | 823.1397 | 13.763 | 0955 |
| M-H | 822.1164 | 12.8 | 0956 |
| M-H | 822.1348 | 11.78 | 0957,0958 |
| M-H | 822.1348 | 11.54 | 0957,0958 |
| M+H | 824.2224 | 12.692 | 0959 |
| M+H | 825.1997 | 12.185 | 0960 |
| M+H | 826.1832 | 12.46 | 0961,0962 |
| M+H | 826.1842 | 10.657 | 0961,0962 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 830.2008 | 16.475 | 0963 |
| M-H | 828.2513 | 10.645 | 0964 |
| M+H | 832.1805 | 14.912 | 0965 |
| M+H | 832.2336 | 11.795 | 0966 |
| M-H | 832.1553 | 11.712 | 0967 |
| M+H | 834.1957 | 12.572 | 0968 |
| M+H | 834.2143 | 10.928 | 0969 |
| M+H | 835.1912 | 12.991 | 0970 |
| M+H | 835.2125 | 13.492 | 0971 |
| M-H | 834.1555 | 11.403 | 0972 |
| M+H | 837.1741 | 14.375 | 0973 |
| M+H | 838.1503 | 15.547 | 0974 |
| M-H | 836.181 | 11.882 | 0975 |
| M+H | 838.2742 | 12.547 | 0976 |
| M+H | 839.1658 | 12.637 | 0977 |
| M-H | 838.1121 | 12.961 | 0978 |
| M+H | 840.1838 | 11.575 | 0979 |
| M+H | 841.1947 | 12.286 | 0980 |
| M-H | 840.126 | 12.118 | 0981 |
| M+H | 849.2071 | 13.815 | 0982 |
| M+H | 851.1858 | 12.424 | 0983 |
| M+H | 852.1842 | 16.666 | 0984 |
| M+H | 853.1845 | 13.393 | 0985 |
| M+H | 855.1461 | 14.46 | 0986 |
| M+H | 857.1562 | 13.481 | 0987 |
| M+H | 861.1694 | 11.48 | 0988 |
| M+H | 863.1317 | 12.219 | 0989 |
| M+H | 869.1797 | 13.549 | 0990 |
| M-H | 868.1365 | 16.023 | 0991 |
| M+H | 871.1412 | 14.651 | 0992 |
| M-H | 870.1709 | 11.98 | 0993 |
| M-H | 872.1314 | 12.72 | 0994 |
| M+H | 878.143 | 13.321 | 0995 |
| M+H | 886.1509 | 16.699 | 0996 |
| M-H | 886.1663 | 12.088 | 0997 |
| M+H | 889.1624 | 14.234 | 0998 |
| M+H | 890.1471 | 12.944 | 0999 |

(continued)

| [Table 8-11-2] Compound that may be contained in mixture 2-1-m1E02-1 | | | |
|---|---|---|---|
| Ion species | m/z $[M+H]^+$ or $[M-H]^-$ | Retention time of MS peak (min) | No. of assigned compound described with 2-1-m1E02-1-omitted |
| M+H | 905.1571 | 14.384 | 1000 |

[Table 409]

| [Table 8-11-3] Compound that may be contained in mixture 2-1-m1E03-1 | | | |
|---|---|---|---|
| Ion species | m/z [M+H]+ or [M-H]- | Retention time of MS peak (min) | No. of assigned compound described with 2-1-m1E03-1-omitted |
| M+H | 641.2226 | 14.283 | 0001 |
| M+H | 642.219 | 13.468 | 0002,0003 |
| M-H | 640.2012 | 12.473 | 0002,0003 |
| M+H | 643.2136 | 14.854 | 0004,0005 |
| M+H | 643.214 | 4.957 | 0004,0005 |
| M-H | 642.1911 | 13.045 | 0006 |
| M+H | 648.1757 | 14.745 | 0007 |
| M+H | 649.1708 | 13.062 | 0008 |
| M+H | 655.2389 | 14.805 | 0009 |
| M+H | 656.2343 | 14.035 | 0010 |
| M+H | 657.2299 | 15.35 | 0011 |
| M+H | 659.1969 | 12.835 | 0012 |
| M+H | 659.2091 | 12.285 | 0013 |
| M+H | 660.1931 | 11.093 | 0014 |
| M+H | 660.2093 | 13.567 | 0015 |
| M+H | 660.2284 | 12.45 | 0016 |
| M+H | 661.2048 | 14.885 | 0017 |
| M+H | 661.2248 | 11.064 | 0018,0019 |
| M-H | 659.2076 | 9.425 | 0018,0019 |
| M+H | 662.1909 | 15.288 | 0020 |
| M+H | 662.2193 | 8.247 | 0021,0022 |
| M+H | 662.2203 | 13.046 | 0021,0022 |
| M-H | 661.1978 | 10.146 | 0023 |
| M+H | 666.1659 | 14.793 | 0024 |
| M+H | 667.1814 | 12.651 | 0025 |
| M+H | 668.1763 | 9.68 | 0026 |
| M+H | 669.2182 | 7.975 | 0027 |
| M+H | 669.2544 | 15.294 | 0028 |
| M+H | 670.2144 | 10.904 | 0029,0030 |
| M+H | 670.2131 | 10.961 | 0029,0030 |
| M+H | 670.2509 | 14.607 | 0031 |
| M-H | 669.1915 | 10.899 | 0032 |
| M+H | 671.2333 | 14.27 | 0033 |
| M+H | 671.2458 | 15.858 | 0034 |
| M+H | 672.2296 | 13.469 | 0035 |
| M+H | 673.213 | 13.294 | 0036 |
| M+H | 673.2244 | 14.743 | 0037 |
| M+H | 674.2441 | 12.85 | 0038 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 675.1751 | 13.009 | 0039 |
| M+H | 675.2405 | 11.508 | 0040 |
| M+H | 676.17 | 11.427 | 0041 |
| M+H | 676.2065 | 15.716 | 0042 |
| M+H | 676.2363 | 13.451 | 0043 |
| M+H | 677.1882 | 12.899 | 0044 |
| M+H | 677.2048 | 14.644 | 0045 |
| M+H | 678.1862 | 14.649 | 0046 |
| M+H | 678.1996 | 13.961 | 0047,0048 |
| M-H | 676.186 | 11.385 | 0047,0048 |
| M+H | 678.2148 | 10.302 | 0049 |
| M+H | 679.1951 | 15.128 | 0050,0051 |
| M+H | 679.1989 | 8.527 | 0051 |
| M+H | 679.215 | 11.234 | 0052 |
| M+H | 680.2106 | 13.075 | 0053 |
| M+H | 681.1977 | 13.17 | 0054 |
| M+H | 683.2344 | 13.162 | 0055 |
| M+H | 684.157 | 15.003 | 0056 |
| M+H | 684.2295 | 12.543 | 0057,0058 |
| M+H | 684.2275 | 10.85 | 0057,0058 |
| M+H | 685.172 | 12.722 | 0059 |
| M+H | 685.2232 | 10.874 | 0060 |
| M+H | 687.2039 | 8.299 | 0061,0062 |
| M+H | 687.2091 | 12.913 | 0061,0062 |
| M+H | 687.2288 | 13.797 | 0063 |
| M+H | 688.2048 | 11.408 | 0064,0065 |
| M+H | 688.2041 | 6.018 | 0064,0065 |
| M+H | 688.2243 | 11.121 | 0066 |
| M+H | 688.2601 | 13.353 | 0067 |
| M+H | 689.1873 | 10.443 | 0068 |
| M+H | 689.2086 | 10.821 | 0069 |
| M+H | 689.219 | 8.34 | 0070,0071 |
| M+H | 689.2194 | 12.195 | 0070,0071 |
| M+H | 689.256 | 12.09 | 0072 |
| M+H | 690.215 | 8.465 | 0073 |
| M-H | 688.2232 | 10.324 | 0074 |
| M+H | 690.2512 | 13.932 | 0075 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 691.236 | 6.056 | 0076,0077 |
| M+H | 691.2399 | 15.3 | 0077 |
| M+H | 692.2191 | 11.767 | 0078 |
| M+H | 692.2296 | 13.004 | 0079,0080 |
| M+H | 692.2343 | 14.604 | 0080 |
| M+H | 693.1656 | 13.131 | 0081 |
| M+H | 693.23 | 15.694 | 0082,0083 |
| M+H | 693.2301 | 15.637 | 0082,0083 |
| M+H | 694.1806 | 11.335 | 0084 |
| M-H | 692.2106 | 6.133 | 0085 |
| M+H | 695.1643 | 13.405 | 0086 |
| M+H | 695.1761 | 8.604 | 0087,0088 |
| M+H | 695.1799 | 9.948 | 0088 |
| M+H | 695.2098 | 8.528 | 0089 |
| M+H | 696.1598 | 11.757 | 0090 |
| M+H | 696.1938 | 11.464 | 0091 |
| M+H | 696.2053 | 10.373 | 0092 |
| M+H | 697.1918 | 10.411 | 0093,0094 |
| M+H | 697.1971 | 6.158 | 0094 |
| M+H | 697.2056 | 11.994 | 0095 |
| M+H | 697.2174 | 12.584 | 0096 |
| M+H | 697.25 | 13.6 | 0097 |
| M+H | 698.191 | 15.686 | 0098,0099 |
| M-H | 696.1726 | 15.63 | 0098,0099 |
| M+H | 698.2015 | 13.728 | 0100 |
| M+H | 698.2118 | 10.914 | 0101 |
| M+H | 698.2455 | 12.99 | 0102,0103 |
| M+H | 698.2451 | 13.691 | 0102,0103 |
| M+H | 699.2299 | 9 | 0104 |
| M+H | 700.2241 | 10 | 0105 |
| M+H | 701.2246 | 13.35 | 0106 |
| M+H | 701.2812 | 13.234 | 0107 |
| M+H | 702.2206 | 12.665 | 0108 |
| M+H | 702.2404 | 11.533 | 0109 |
| M+H | 702.2751 | 11.533 | 0110 |
| M+H | 703.1633 | 13.321 | 0111 |
| M+H | 703.2067 | 13.912 | 0112 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 703.2353 | 10.781 | 0113,0114 |
| M+H | 703.2341 | 12.089 | 0113,0114 |
| M+H | 704.2308 | 8.086 | 0115 |
| M+H | 705.1808 | 14.266 | 0116,0117,0118 |
| M-H | 703.1683 | 10.952 | 0116,0117,0118 |
| M+H | 705.1849 | 13.605 | 0116,0117,0118 |
| M+H | 705.1964 | 13.015 | 0119,0120 |
| M+H | 705.1967 | 13.141 | 0119,0120 |
| M-H | 704.163 | 11.767 | 0121,0122,0123 |
| M-H | 704.1629 | 10.373 | 0121,0122,0123 |
| M-H | 704.1638 | 12.937 | 0121,0122,0123 |
| M+H | 706.1935 | 7.507 | 0124 |
| M+H | 706.2096 | 6.108 | 0125,0126 |
| M-H | 704.1982 | 11.244 | 0125,0126 |
| M+H | 706.2347 | 12.259 | 0127 |
| M+H | 707.1764 | 10.126 | 0128,0129 |
| M+H | 707.1754 | 10.964 | 0128,0129 |
| M+H | 707.2104 | 8.594 | 0130,0131 |
| M+H | 707.2101 | 11.424 | 0130,0131 |
| M+H | 707.2449 | 13.91 | 0132 |
| M-H | 706.1537 | 12.326 | 0133 |
| M+H | 708.1927 | 8.904 | 0134 |
| M-H | 706.1971 | 9.171 | 0135 |
| M+H | 709.1757 | 10.041 | 0136 |
| M+H | 709.2107 | 15.066 | 0137,0138,0139 |
| M-H | 707.1922 | 14.472 | 0137,0138,0139 |
| M+H | 709.2116 | 14.438 | 0137,0138,0139 |
| M-H | 708.1885 | 8.534 | 0140,0141 |
| M-H | 708.1881 | 12.811 | 0140,0141 |
| M+H | 711.14 | 9.633 | 0142 |
| M+H | 711.1561 | 13.332 | 0143 |
| M+H | 711.2007 | 15.545 | 0144 |
| M+H | 711.207 | 10.977 | 0145 |
| M+H | 711.2204 | 13.437 | 0146 |
| M+H | 711.2323 | 13.13 | 0147 |
| M+H | 712.1376 | 12.205 | 0148,0149 |
| M+H | 712.1372 | 14.059 | 0148,0149 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M-H | 710.1536 | 11.403 | 0150 |
| M+H | 712.235 | 11.237 | 0151 |
| M+H | 712.2615 | 13.477 | 0152 |
| M+H | 713.1314 | 6.26 | 0153 |
| M+H | 713.1564 | 13.488 | 0154 |
| M+H | 713.2283 | 10.252 | 0155 |
| M+H | 714.1701 | 12.185 | 0156 |
| M+H | 714.186 | 13.257 | 0157 |
| M+H | 714.2402 | 10.457 | 0158 |
| M+H | 715.1657 | 9.259 | 0159 |
| M+H | 715.1817 | 12.837 | 0160 |
| M+H | 715.2074 | 12.719 | 0161 |
| M+H | 715.241 | 13.809 | 0162 |
| M+H | 715.2963 | 13.714 | 0163 |
| M+H | 716.1631 | 15.434 | 0164 |
| M+H | 716.2019 | 8.284 | 0165 |
| M+H | 716.2216 | 10.24 | 0166 |
| M+H | 716.2555 | 12.027 | 0167 |
| M-H | 715.1785 | 13.109 | 0168,0169 |
| M+H | 717.1952 | 7.035 | 0168,0169 |
| M+H | 717.2181 | 8.34 | 0170,0171,0172 |
| M+H | 717.2194 | 12.91 | 0170,0171,0172 |
| M+H | 717.2203 | 10.873 | 0170,0171,0172 |
| M+H | 717.251 | 11.096 | 0173,0174 |
| M+H | 717.2511 | 12.179 | 0173,0174 |
| M+H | 718.1919 | 12.202 | 0175 |
| M+H | 718.2151 | 10.158 | 0176 |
| M+H | 718.2342 | 9.035 | 0177 |
| M+H | 719.1881 | 13.633 | 0178 |
| M+H | 719.2011 | 12.511 | 0179,0180,0181 |
| M+H | 719.2011 | 12.366 | 0179,0180,0181 |
| M+H | 719.2019 | 14.083 | 0179,0180,0181 |
| M+H | 719.2298 | 8.12 | 0182,0183 |
| M+H | 719.234 | 13.581 | 0183 |
| M+H | 719.2718 | 13.3 | 0184 |
| M+H | 720.197 | 10.657 | 0185,0186,0187 |
| M+H | 720.1973 | 13.484 | 0185,0186,0187 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 720.1967 | 12.262 | 0185,0186,0187 |
| M+H | 720.2103 | 12.833 | 0188 |
| M+H | 720.2248 | 5.687 | 0189,0190 |
| M+H | 720.2307 | 11.587 | 0189,0190 |
| M+H | 720.2877 | 11.54 | 0191 |
| M+H | 721.192 | 14.754 | 0192,0193 |
| M+H | 721.1926 | 10.886 | 0192,0193 |
| M+H | 721.2262 | 8.777 | 0194 |
| M+H | 721.2619 | 14.463 | 0195 |
| M-H | 719.2643 | 8.726 | 0196 |
| M+H | 722.2094 | 8.609 | 0197 |
| M-H | 721.1413 | 12.629 | 0198 |
| M+H | 723.1709 | 7.893 | 0199,0200 |
| M+H | 723.1762 | 12.171 | 0199,0200 |
| M+H | 723.1907 | 8.404 | 0201,0202 |
| M+H | 723.1906 | 10.293 | 0201,0202 |
| M+H | 723.2271 | 14.949 | 0203 |
| M+H | 723.2402 | 13.449 | 0204 |
| M+H | 724.1487 | 13.354 | 0205,0206 |
| M+H | 724.156 | 10.711 | 0206 |
| M-H | 722.1536 | 10.421 | 0207 |
| M+H | 724.1868 | 12.559 | 0208,0209,0210 |
| M+H | 724.1905 | 10.733 | 0208,0209,0210 |
| M-H | 722.1738 | 9.072 | 0208,0209,0210 |
| M+H | 724.2055 | 11.763 | 0211,0212 |
| M+H | 724.204 | 11.336 | 0211,0212 |
| M+H | 725.1534 | 10.093 | 0213 |
| M+H | 725.1628 | 14.313 | 0214 |
| M+H | 725.1754 | 13.052 | 0215 |
| M+H | 725.1833 | 10.109 | 0216,0217,0218 |
| M+H | 725.1862 | 12.363 | 0216,0217,0218,0219 |
| M-H | 723.1689 | 9.385 | 0216,0217,0218,0219 |
| M+H | 725.1863 | 10.985 | 0216,0217,0218,0219 |
| M+H | 725.1994 | 11.882 | 0220,0221 |
| M+H | 725.205 | 12.23 | 0221 |
| M+H | 725.2266 | 9.489 | 0222 |
| M+H | 725.2482 | 13.657 | 0223 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M-H | 724.1349 | 14.553 | 0224 |
| M+H | 726.1812 | 7.584 | 0225,0226 |
| M-H | 724.1653 | 7.896 | 0225,0226 |
| M+H | 726.2011 | 14.542 | 0227 |
| M+H | 726.2514 | 11.662 | 0228 |
| M+H | 727.1723 | 11.96 | 0229 |
| M+H | 727.1849 | 13.626 | 0230 |
| M+H | 727.1967 | 15.63 | 0231,0232 |
| M+H | 727.1966 | 15.672 | 0231,0232 |
| M+H | 727.2045 | 12.033 | 0232,0233 |
| M+H | 727.2272 | 12.608 | 0234 |
| M+H | 728.1872 | 9.426 | 0235 |
| M+H | 728.2171 | 13.512 | 0236,0237 |
| M+H | 728.2168 | 12.864 | 0236,0237 |
| M+H | 729.1323 | 13.915 | 0238 |
| M+H | 729.2118 | 12.427 | 0239,0240,0241 |
| M+H | 729.2107 | 13.778 | 0239,0240,0241 |
| M-H | 727.1951 | 10.056 | 0239,0240,0241 |
| M+H | 729.3125 | 14.247 | 0242 |
| M+H | 730.1291 | 14.132 | 0243 |
| M-H | 728.1305 | 12.465 | 0244 |
| M+H | 730.1621 | 11.974 | 0245 |
| M+H | 730.2075 | 14.027 | 0246 |
| M+H | 730.2145 | 12.804 | 0247 |
| M+H | 730.226 | 11.39 | 0248 |
| M+H | 730.2345 | 10.03 | 0249 |
| M+H | 731.1437 | 9.583 | 0250,0251,0252 |
| M+H | 731.1422 | 14.173 | 0250,0251,0252 |
| M+H | 731.1454 | 13.712 | 0250,0251,0252 |
| M+H | 731.2368 | 11.694 | 0253,0254 |
| M+H | 731.2355 | 11.323 | 0253,0254 |
| M+H | 731.2672 | 11.562 | 0255 |
| M+H | 731.2921 | 13.233 | 0256 |
| M-H | 730.1211 | 9.174 | 0257 |
| M+H | 732.1575 | 15.584 | 0258,0259 |
| M+H | 732.1598 | 11.468 | 0258,0259 |
| M+H | 732.2326 | 8.147 | 0260 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 733.1784 | 12.249 | 0261,0262 |
| M+H | 733.1812 | 10.609 | 0262 |
| M+H | 733.198 | 12.964 | 0263 |
| M+H | 733.2164 | 14.526 | 0264,0265,0266,0267 |
| M+H | 733.2131 | 6.065 | 0264,0265,0266,0267 |
| M+H | 733.2169 | 12.863 | 0264,0265,0266,0267 |
| M-H | 731.1975 | 10.427 | 0264,0265,0266,0267 |
| M+H | 733.2457 | 8.461 | 0268 |
| M+H | 734.1759 | 10.578 | 0269,0270 |
| M+H | 734.1759 | 10.959 | 0269,0270 |
| M+H | 734.1909 | 9.534 | 0271 |
| M+H | 734.2129 | 14.019 | 0272,0273,0274,0275 |
| M+H | 734.2129 | 11.271 | 0272,0273,0274,0275 |
| M+H | 734.2115 | 9.319 | 0272,0273,0274,0275 |
| M-H | 732.1943 | 12.797 | 0272,0273,0274,0275 |
| M+H | 734.2457 | 13.444 | 0276,0277 |
| M+H | 734.2462 | 12.034 | 0276,0277 |
| M+H | 734.3031 | 11.915 | 0278 |
| M+H | 735.1687 | 13.663 | 0279,0280,0281 |
| M+H | 735.1716 | 11.722 | 0279,0280,0281 |
| M+H | 735.1719 | 14.748 | 0280,0281 |
| M+H | 735.1961 | 12.071 | 0282,0283 |
| M+H | 735.1964 | 13.559 | 0282,0283 |
| M+H | 735.2071 | 15.231 | 0284,0285 |
| M+H | 735.2071 | 7.244 | 0284,0285 |
| M+H | 736.1915 | 8.452 | 0286 |
| M+H | 736.2256 | 8.937 | 0287,0288 |
| M+H | 736.2248 | 11.151 | 0287,0288 |
| M+H | 737.1708 | 13.936 | 0289 |
| M+H | 737.1867 | 8.533 | 0290,0291,0292 |
| M+H | 737.1922 | 12.506 | 0291,0292 |
| M+H | 737.1911 | 12.638 | 0291,0292 |
| M+H | 737.2042 | 10.817 | 0293 |
| M+H | 737.218 | 7.087 | 0294,0295 |
| M+H | 737.2208 | 8.262 | 0294,0295 |
| M+H | 737.2421 | 15.39 | 0296 |
| M+H | 737.2616 | 13.577 | 0297 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 738.1846 | 8.788 | 0298,0299 |
| M+H | 738.1869 | 9.091 | 0298,0299 |
| M+H | 738.2019 | 13.537 | 0300,0301,0302,0303 |
| M+H | 738.2069 | 9.596 | 0301,0302,0303 |
| M+H | 738.2048 | 9.694 | 0301,0302,0303 |
| M+H | 738.2056 | 9.66 | 0301,0302,0303 |
| M+H | 738.2778 | 11.646 | 0304 |
| M+H | 739.1376 | 12.931 | 0305 |
| M+H | 739.1719 | 10.221 | 0306 |
| M+H | 739.2029 | 8.088 | 0307,0308,0309 |
| M+H | 739.204 | 11.385 | 0307,0308,0309 |
| M+H | 739.2021 | 7.807 | 0307,0308,0309 |
| M-H | 737.1983 | 11.43 | 0310 |
| M+H | 739.2214 | 12.763 | 0311 |
| M+H | 740.1328 | 11.109 | 0312 |
| M+H | 740.1657 | 9.951 | 0313 |
| M+H | 740.1972 | 6.705 | 0314,0315 |
| M+H | 740.1967 | 7.435 | 0314,0315 |
| M+H | 740.2669 | 12.211 | 0316 |
| M-H | 739.1325 | 12.764 | 0317,0318 |
| M+H | 741.1501 | 12.826 | 0317,0318 |
| M+H | 741.167 | 12.397 | 0319,0320 |
| M+H | 741.1671 | 12.302 | 0319,0320 |
| M+H | 742.1482 | 13.982 | 0321 |
| M+H | 742.1626 | 13.372 | 0322,0323 |
| M+H | 742.167 | 11.324 | 0323 |
| M-H | 740.1643 | 11.049 | 0324,0325 |
| M-H | 740.1643 | 10.845 | 0324,0325 |
| M+H | 742.1962 | 12.101 | 0326,0327 |
| M+H | 742.1967 | 12.568 | 0326,0327 |
| M+H | 742.2323 | 13.231 | 0328 |
| M+H | 742.2464 | 9.158 | 0329 |
| M+H | 743.158 | 14.548 | 0330,0331,0332 |
| M+H | 743.1613 | 7.981 | 0331,0332 |
| M+H | 743.1631 | 13.851 | 0331,0332 |
| M+H | 743.177 | 8.133 | 0333,0334 |
| M+H | 743.1772 | 11.128 | 0333,0334 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 743.2459 | 14.521 | 0335 |
| M+H | 744.1623 | 12.833 | 0336 |
| M-H | 742.1549 | 12.565 | 0337 |
| M+H | 744.1773 | 13.507 | 0338 |
| M+H | 744.2119 | 10.436 | 0339 |
| M+H | 744.2324 | 8.885 | 0340 |
| M+H | 744.2535 | 12.055 | 0341 |
| M+H | 745.1593 | 12.621 | 0342 |
| M+H | 745.1804 | 14.289 | 0343 |
| M+H | 745.1921 | 11.642 | 0344,0345 |
| M+H | 745.1921 | 11.82 | 0344,0345 |
| M+H | 745.2074 | 12.471 | 0346 |
| M+H | 745.2547 | 15.477 | 0347,0348,0349 |
| M+H | 745.2525 | 15.124 | 0347,0348,0349 |
| M+H | 745.2535 | 10.693 | 0347,0348,0349 |
| M+H | 746.1816 | 11.896 | 0350 |
| M-H | 744.1735 | 12.353 | 0350,0351 |
| M+H | 746.2028 | 14.122 | 0352,0353 |
| M+H | 746.2026 | 11.452 | 0352,0353 |
| M+H | 746.2105 | 12.407 | 0353,0354 |
| M+H | 746.233 | 9.214 | 0355 |
| M+H | 746.2482 | 8.858 | 0356 |
| M+H | 747.1237 | 14.128 | 0357 |
| M+H | 747.1966 | 12.748 | 0358 |
| M-H | 745.1942 | 16.114 | 0359 |
| M+H | 747.2329 | 13.377 | 0360,0361,0362,0363 |
| M+H | 747.2327 | 13.53 | 0360,0361,0362,0363 |
| M+H | 747.233 | 13.594 | 0360,0361,0362,0363 |
| M+H | 747.2329 | 13.996 | 0360,0361,0362,0363 |
| M+H | 748.1182 | 14.268 | 0364 |
| M+H | 748.1379 | 12.568 | 0365 |
| M+H | 748.191 | 7.918 | 0366,0367 |
| M+H | 748.1911 | 12.662 | 0366,0367 |
| M+H | 748.2175 | 12.078 | 0368 |
| M+H | 748.2265 | 8.681 | 0369 |
| M+H | 748.3186 | 12.398 | 0370 |
| M+H | 749.1335 | 12.317 | 0371 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 749.1857 | 10.897 | 0372 |
| M+H | 749.2116 | 11.101 | 0373,0374 |
| M+H | 749.2114 | 12.409 | 0373,0374 |
| M+H | 749.2227 | 7.37 | 0375 |
| M+H | 750.1507 | 12.744 | 0376 |
| M+H | 750.2081 | 8.941 | 0377,0378 |
| M+H | 750.2074 | 11.806 | 0377,0378 |
| M+H | 750.2404 | 9.446 | 0379,0380 |
| M+H | 750.2379 | 11.618 | 0379,0380 |
| M+H | 750.2971 | 9.303 | 0381 |
| M+H | 751.1483 | 11.881 | 0382 |
| M-H | 749.1477 | 8.238 | 0383 |
| M+H | 751.1704 | 12.401 | 0384,0385 |
| M+H | 751.1659 | 9.21 | 0383,0384,0385 |
| M+H | 751.1886 | 9.718 | 0386 |
| M+H | 751.2067 | 13.184 | 0387,0388 |
| M+H | 751.2061 | 13.667 | 0387,0388 |
| M+H | 751.2383 | 11.748 | 0389 |
| M+H | 751.2965 | 14.198 | 0390 |
| M+H | 752.167 | 11.308 | 0391,0392 |
| M+H | 752.1672 | 11.391 | 0391,0392 |
| M+H | 752.1815 | 8.546 | 0393,0394 |
| M+H | 752.1858 | 7.917 | 0394 |
| M+H | 752.2012 | 7.615 | 0395 |
| M+H | 752.217 | 13.381 | 0396,0397,0398,0399,0400 |
| M+H | 752.2228 | 11.31 | 0397,0398,0399,0400 |
| M+H | 752.2217 | 13.112 | 0397,0398,0399,0400 |
| M+H | 752.2216 | 8.684 | 0397,0398,0399,0400 |
| M-H | 750.2052 | 11.598 | 0397,0398,0399,0400 |
| M+H | 753.1527 | 13.522 | 0401 |
| M+H | 753.1706 | 12.73 | 0402 |
| M+H | 753.1818 | 7.053 | 0403,0404 |
| M-H | 751.1625 | 8.094 | 0403,0404 |
| M+H | 753.201 | 8.117 | 0405,0406 |
| M+H | 753.201 | 8.167 | 0405,0406 |
| M-H | 751.2027 | 11.297 | 0407,0408,0409 |
| M-H | 751.2001 | 11.94 | 0407,0408,0409 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 753.2176 | 12.032 | 0407,0408,0409 |
| M+H | 754.1761 | 8.292 | 0410 |
| M+H | 754.1965 | 13.686 | 0411,0412,0413 |
| M+H | 754.1999 | 10.732 | 0412,0413 |
| M+H | 754.1969 | 11.221 | 0411,0412,0413 |
| M+H | 754.2143 | 7.026 | 0414,0415 |
| M+H | 754.2179 | 13.268 | 0415 |
| M+H | 755.1804 | 11.493 | 0416,0417 |
| M+H | 755.1817 | 12.743 | 0416,0417 |
| M+H | 755.197 | 7.83 | 0418,0419,0420,0421 |
| M+H | 755.1966 | 8.675 | 0418,0419,0420,0421 |
| M+H | 755.1973 | 11.066 | 0418,0419,0420,0421 |
| M+H | 755.1977 | 6.009 | 0418,0419,0420,0421 |
| M+H | 756.1782 | 9.996 | 0422,0423 |
| M+H | 756.1816 | 11.731 | 0423 |
| M+H | 756.1935 | 12.734 | 0424,0425,0426,0427 |
| M+H | 756.1912 | 7.551 | 0424,0425,0426,0427 |
| M+H | 756.1974 | 11.442 | 0425,0426,0427 |
| M+H | 756.1964 | 14.014 | 0425,0426,0427 |
| M+H | 756.2121 | 12.185 | 0428 |
| M+H | 756.2475 | 13.693 | 0429 |
| M-H | 754.2503 | 12.236 | 0430 |
| M+H | 757.1286 | 13.085 | 0431 |
| M+H | 757.1921 | 15.135 | 0432,0433,0434,0435 |
| M+H | 757.1914 | 12.581 | 0432,0433,0434,0435 |
| M+H | 757.1921 | 15.179 | 0432,0433,0434,0435 |
| M+H | 757.1924 | 6.933 | 0432,0433,0434,0435 |
| M-H | 755.1883 | 12.335 | 0436 |
| M+H | 758.1429 | 11.366 | 0437 |
| M+H | 758.1763 | 8.985 | 0438 |
| M-H | 756.1731 | 12.463 | 0439 |
| M-H | 756.2097 | 10.837 | 0440 |
| M-H | 757.1082 | 13.222 | 0441 |
| M-H | 757.1195 | 8.148 | 0442,0443 |
| M+H | 759.1423 | 10.009 | 0443 |
| M+H | 759.1551 | 5.689 | 0444 |
| M+H | 759.1719 | 8.605 | 0445 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 759.1946 | 8.043 | 0446 |
| M+H | 759.2261 | 15.375 | 0447 |
| M+H | 759.2651 | 9.419 | 0448,0449 |
| M+H | 759.2675 | 15.578 | 0448,0449 |
| M+H | 760.1231 | 11.541 | 0450 |
| M+H | 760.1544 | 9.598 | 0451,0452 |
| M-H | 758.1395 | 11.388 | 0451,0452 |
| M+H | 760.1718 | 11.167 | 0453,0454 |
| M+H | 760.1735 | 8.503 | 0453,0454 |
| M+H | 760.1921 | 13.075 | 0455 |
| M+H | 760.2026 | 11.308 | 0456 |
| M+H | 761.1541 | 6.271 | 0457,0458,0459,0460 |
| M+H | 761.1577 | 14.752 | 0458,0459,0460 |
| M+H | 761.1565 | 14.375 | 0457,0458,0459,0460 |
| M+H | 761.1547 | 12.235 | 0457,0458,0459,0460 |
| M+H | 761.1682 | 8.078 | 0461 |
| M+H | 761.2094 | 13.544 | 0462 |
| M+H | 761.2126 | 8.032 | 0462,0463 |
| M+H | 761.2492 | 14.288 | 0464,0465 |
| M+H | 761.247 | 14.58 | 0464,0465 |
| M+H | 762.1534 | 13.225 | 0466,0467,0468 |
| M+H | 762.153 | 14.996 | 0466,0467,0468 |
| M+H | 762.1535 | 13.341 | 0466,0467,0468 |
| M+H | 762.1641 | 5.901 | 0469,0470 |
| M+H | 762.167 | 12.349 | 0470 |
| M+H | 762.1859 | 12.474 | 0471 |
| M+H | 762.2065 | 12.746 | 0472,0473 |
| M+H | 762.2015 | 10.563 | 0472,0473 |
| M+H | 763.1473 | 9.38 | 0474 |
| M+H | 763.1827 | 11.765 | 0475 |
| M-H | 761.1738 | 12.295 | 0476 |
| M+H | 763.227 | 11.447 | 0477,0478 |
| M+H | 763.2269 | 12.601 | 0477,0478 |
| M+H | 763.2405 | 7.829 | 0479 |
| M+H | 764.1869 | 10.457 | 0480 |
| M+H | 764.1957 | 8.014 | 0481 |
| M-H | 762.2418 | 12.171 | 0482,0483,0484 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M-H | 762.2426 | 13.356 | 0482,0483,0484 |
| M+H | 764.2595 | 8.982 | 0482,0483,0484 |
| M+H | 765.1803 | 8.354 | 0485,0486 |
| M+H | 765.1865 | 12.929 | 0486 |
| M+H | 765.2061 | 10.728 | 0487 |
| M+H | 765.2191 | 6.742 | 0488 |
| M-H | 763.2247 | 12.59 | 0489 |
| M+H | 765.2542 | 7.26 | 0490 |
| M+H | 766.1285 | 13.052 | 0491 |
| M+H | 766.1822 | 7.487 | 0492 |
| M+H | 766.2007 | 11.427 | 0493 |
| M-H | 764.2204 | 11.806 | 0494,0495,0496,0497 |
| M-H | 764.2204 | 10.95 | 0494,0495,0496,0497 |
| M+H | 766.2382 | 11.065 | 0494,0495,0496,0497 |
| M-H | 764.2181 | 11.571 | 0494,0495,0496,0497 |
| M+H | 767.1247 | 8.659 | 0498 |
| M+H | 767.1655 | 10.203 | 0499 |
| M+H | 767.1941 | 11.694 | 0500,0501 |
| M-H | 765.1792 | 10.7 | 0500,0501 |
| M+H | 767.1971 | 6.989 | 0500,0501,0502,0503 |
| M+H | 767.1985 | 12.121 | 0500,0501,0502,0503 |
| M+H | 767.2136 | 7.627 | 0504 |
| M+H | 767.2326 | 9.284 | 0505,0506 |
| M+H | 767.2344 | 12.008 | 0505,0506 |
| M+H | 768.1919 | 8.012 | 0507 |
| M+H | 768.2119 | 13.547 | 0508,0509,0510 |
| M+H | 768.2123 | 10.985 | 0508,0509,0510 |
| M+H | 768.218 | 11.121 | 0509,0510 |
| M+H | 768.2286 | 4.894 | 0511 |
| M+H | 769.1477 | 13 | 0512,0513 |
| M-H | 767.1292 | 12.915 | 0512,0513 |
| M+H | 769.1624 | 12.661 | 0514,0515 |
| M+H | 769.1594 | 7.228 | 0514,0515 |
| M+H | 769.1964 | 11.373 | 0516,0517 |
| M+H | 769.202 | 12.082 | 0517 |
| M-H | 767.1931 | 9.507 | 0518,0519 |
| M+H | 769.2085 | 6.796 | 0518,0519 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 769.2175 | 13.34 | 0520 |
| M+H | 769.2682 | 14.054 | 0521 |
| M-H | 768.1249 | 11.742 | 0522 |
| M-H | 768.1405 | 12.624 | 0523 |
| M+H | 770.1719 | 6.043 | 0524,0525 |
| M-H | 768.1605 | 11.143 | 0524,0525 |
| M+H | 770.1921 | 10.843 | 0526 |
| M+H | 770.213 | 11.174 | 0527,0528,0529 |
| M+H | 770.213 | 11.933 | 0527,0528,0529 |
| M+H | 770.213 | 15.096 | 0527,0528,0529 |
| M+H | 771.1383 | 12.571 | 0530 |
| M+H | 771.1722 | 8.385 | 0531,0532 |
| M-H | 769.154 | 11.27 | 0531,0532 |
| M+H | 771.192 | 13.837 | 0533 |
| M+H | 771.2077 | 14.744 | 0534 |
| M+H | 771.2223 | 11.794 | 0535 |
| M+H | 772.1528 | 9.096 | 0536 |
| M+H | 772.1752 | 12.771 | 0537 |
| M+H | 772.206 | 9.855 | 0538,0539 |
| M+H | 772.2059 | 12.257 | 0538,0539 |
| M+H | 773.141 | 8.215 | 0540 |
| M-H | 771.1544 | 12.944 | 0541,0542,0543,0544,0545,0546 |
| M+H | 773.1729 | 14.319 | 0542,0543,0544,0545,0546 |
| M+H | 773.1708 | 9.36 | 0541,0542,0543,0544,0545,0546 |
| M+H | 773.1728 | 14.368 | 0542,0543,0544,0545,0546 |
| M+H | 773.171 | 5.143 | 0541,0542,0543,0544,0545,0546 |
| M+H | 773.1755 | 13.659 | 0542,0543,0544,0545,0546 |
| M-H | 771.1722 | 11.685 | 0547 |
| M+H | 773.2019 | 12.447 | 0548 |
| M+H | 773.2122 | 15.096 | 0549 |
| M+H | 773.2478 | 8.82 | 0550 |
| M+H | 773.2807 | 7.005 | 0551 |
| M+H | 774.1691 | 13.87 | 0552,0553 |
| M+H | 774.1679 | 8.721 | 0552,0553 |
| M+H | 774.1886 | 9.01 | 0554,0555 |
| M+H | 774.1899 | 5.834 | 0554,0555 |
| M+H | 774.2035 | 4.754 | 0556 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 774.2431 | 13.065 | 0557 |
| M+H | 775.1041 | 15.061 | 0558 |
| M+H | 775.1195 | 13.331 | 0559 |
| M+H | 775.1634 | 14.972 | 0560 |
| M+H | 775.1737 | 14.824 | 0561,0562 |
| M+H | 775.1726 | 12.256 | 0561,0562 |
| M+H | 775.1842 | 10.854 | 0563,0564 |
| M-H | 773.1668 | 10.156 | 0563,0564 |
| M-H | 773.2499 | 9.04 | 0565 |
| M+H | 776.0991 | 12.742 | 0566 |
| M+H | 776.1344 | 11.536 | 0567 |
| M+H | 776.1682 | 9.214 | 0568 |
| M+H | 776.1971 | 8.771 | 0569 |
| M+H | 776.2231 | 13.245 | 0570 |
| M-H | 775.0993 | 13.321 | 0571 |
| M+H | 777.1619 | 12.605 | 0572 |
| M+H | 777.1868 | 14.691 | 0573,0574 |
| M+H | 777.1924 | 7.653 | 0574,0575 |
| M+H | 777.1994 | 15.141 | 0575 |
| M+H | 777.2071 | 11.142 | 0576 |
| M+H | 777.2413 | 8.059 | 0577 |
| M-H | 775.236 | 12.124 | 0578 |
| M-H | 776.1143 | 12.169 | 0579 |
| M+H | 778.1478 | 10.676 | 0580 |
| M+H | 778.1626 | 12.41 | 0581 |
| M+H | 778.2007 | 10.494 | 0582,0583 |
| M-H | 776.1837 | 13.207 | 0582,0583 |
| M-H | 776.2574 | 9.528 | 0584,0585 |
| M-H | 776.2567 | 13.13 | 0584,0585 |
| M+H | 779.1277 | 8.987 | 0586,0587 |
| M+H | 779.1304 | 14.588 | 0586,0587 |
| M+H | 779.1429 | 7.205 | 0588,0589,0590 |
| M+H | 779.1488 | 14.439 | 0588,0589,0590 |
| M+H | 779.1488 | 14.308 | 0588,0589,0590 |
| M+H | 779.1976 | 10.496 | 0591,0592 |
| M+H | 779.2039 | 10.541 | 0592 |
| M+H | 779.2224 | 10.826 | 0593 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 779.2597 | 13.113 | 0594 |
| M+H | 780.1244 | 14.765 | 0595 |
| M+H | 780.1634 | 13.055 | 0596,0597 |
| M+H | 780.1603 | 12.98 | 0596,0597 |
| M-H | 778.2004 | 11.913 | 0598 |
| M+H | 780.2176 | 7.266 | 0598,0599 |
| M-H | 778.234 | 12.026 | 0600,0601 |
| M+H | 780.2547 | 7.508 | 0600,0601 |
| M+H | 781.1596 | 10.501 | 0602,0603,0604,0605 |
| M+H | 781.159 | 9.711 | 0602,0603,0604,0605 |
| M-H | 779.1391 | 10.341 | 0602,0603,0604,0605 |
| M-H | 779.1405 | 12.763 | 0602,0603,0604,0605 |
| M-H | 779.1642 | 10.787 | 0606,0607 |
| M+H | 781.1824 | 10.857 | 0607 |
| M+H | 781.1983 | 8.33 | 0608 |
| M-H | 779.1953 | 11.154 | 0609,0610 |
| M+H | 781.2125 | 12.006 | 0609,0610 |
| M-H | 779.2197 | 10.422 | 0611 |
| M+H | 781.2545 | 7.515 | 0612 |
| M+H | 782.1533 | 10.991 | 0613,0614 |
| M+H | 782.1545 | 11.88 | 0613,0614 |
| M-H | 780.1793 | 9.129 | 0615 |
| M+H | 782.2336 | 11.615 | 0616,0617 |
| M+H | 782.2328 | 8.621 | 0616,0617 |
| M+H | 782.2457 | 6.697 | 0618 |
| M+H | 783.1447 | 10.302 | 0619 |
| M-H | 781.1438 | 13.941 | 0620 |
| M+H | 783.1934 | 10.15 | 0621,0622 |
| M+H | 783.1969 | 13.279 | 0622 |
| M-H | 781.2188 | 9.761 | 0623,0624 |
| M-H | 781.2151 | 12.681 | 0623,0624 |
| M+H | 784.1723 | 12.577 | 0625 |
| M+H | 784.1864 | 5.652 | 0626,0627,0628 |
| M+H | 784.1911 | 13.064 | 0626,0627,0628 |
| M+H | 784.1912 | 11.417 | 0626,0627,0628 |
| M+H | 784.2076 | 11.153 | 0629 |
| M+H | 784.2233 | 4.789 | 0630 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 784.2476 | 11.141 | 0631 |
| M+H | 785.1784 | 14.507 | 0632 |
| M+H | 785.184 | 13.415 | 0632,0633 |
| M+H | 785.1877 | 10.852 | 0633,0634 |
| M+H | 785.2102 | 11.424 | 0635 |
| M+H | 785.2233 | 13.798 | 0636 |
| M-H | 783.2263 | 8.488 | 0637 |
| M+H | 785.2634 | 14.181 | 0638 |
| M-H | 784.1536 | 8.858 | 0639 |
| M+H | 786.2057 | 12.304 | 0640,0641 |
| M+H | 786.2044 | 9.993 | 0640,0641 |
| M-H | 784.204 | 9.315 | 0642 |
| M+H | 787.1153 | 12.951 | 0643 |
| M+H | 787.1393 | 12.593 | 0644 |
| M+H | 787.1509 | 12.732 | 0645 |
| M+H | 787.1522 | 8.15 | 0645,0646 |
| M+H | 787.1868 | 11.462 | 0647,0648 |
| M+H | 787.1888 | 14.693 | 0647,0648 |
| M+H | 787.2011 | 12.744 | 0649,0650 |
| M+H | 787.2077 | 9.773 | 0650 |
| M+H | 787.2185 | 11.864 | 0651 |
| M+H | 787.2275 | 15.538 | 0652 |
| M+H | 787.2642 | 9.172 | 0653 |
| M+H | 788.11 | 12.337 | 0654 |
| M-H | 786.1351 | 12.154 | 0655,0656 |
| M+H | 788.1531 | 9.406 | 0655,0656 |
| M-H | 786.1498 | 11.608 | 0657,0658 |
| M+H | 788.1683 | 11.631 | 0657,0658 |
| M+H | 788.1841 | 13.542 | 0659 |
| M+H | 788.2011 | 9.957 | 0660 |
| M+H | 788.2745 | 12.588 | 0661 |
| M+H | 789.1202 | 15.689 | 0662 |
| M-H | 787.1189 | 13.26 | 0663 |
| M+H | 789.1479 | 9.076 | 0664,0665 |
| M+H | 789.153 | 14.077 | 0665 |
| M-H | 787.1487 | 14.365 | 0666,0667,0668 |
| M+H | 789.1679 | 13.117 | 0667,0668 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 789.1677 | 11.124 | 0667,0668 |
| M+H | 789.1851 | 9.428 | 0669,0670 |
| M+H | 789.1867 | 11.22 | 0669,0670 |
| M+H | 789.2072 | 14.583 | 0671 |
| M+H | 790.1633 | 12.923 | 0672 |
| M+H | 790.1832 | 14.641 | 0673 |
| M+H | 790.196 | 9.772 | 0674 |
| M+H | 790.2093 | 9.247 | 0675 |
| M+H | 791.1478 | 13.653 | 0676 |
| M+H | 791.1598 | 15.14 | 0677,0678 |
| M+H | 791.1652 | 10.057 | 0678,0679,0680 |
| M+H | 791.168 | 14.376 | 0679,0680 |
| M+H | 791.1675 | 14.276 | 0679,0680 |
| M+H | 791.2381 | 14.674 | 0681 |
| M-H | 790.1295 | 12.594 | 0682 |
| M+H | 792.1787 | 12.406 | 0683,0684,0685,0686 |
| M+H | 792.1779 | 11.787 | 0683,0684,0685,0686 |
| M+H | 792.175 | 12.867 | 0683,0684,0685,0686 |
| M+H | 792.1801 | 8.987 | 0683,0684,0685,0686 |
| M+H | 792.2177 | 13.595 | 0687 |
| M+H | 792.2544 | 8.071 | 0688 |
| M+H | 792.2891 | 10.065 | 0689 |
| M+H | 793.0952 | 15.178 | 0690 |
| M+H | 793.1746 | 12.217 | 0691,0692,0693 |
| M+H | 793.1747 | 11.646 | 0691,0692,0693 |
| M+H | 793.1747 | 11.595 | 0691,0692,0693 |
| M+H | 793.1929 | 15.238 | 0694,0695 |
| M+H | 793.1923 | 13.09 | 0694,0695 |
| M+H | 793.2494 | 9.601 | 0696 |
| M+H | 793.2743 | 13.632 | 0697 |
| M-H | 792.0916 | 13.454 | 0698 |
| M-H | 792.1097 | 9.968 | 0699 |
| M+H | 794.1597 | 14.277 | 0700 |
| M+H | 794.1669 | 12.11 | 0701 |
| M+H | 794.1796 | 13.389 | 0702,0703 |
| M+H | 794.1777 | 13.42 | 0702,0703 |
| M-H | 792.1744 | 10.754 | 0704 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 794.2698 | 9.843 | 0705 |
| M-H | 793.0866 | 9.222 | 0706,0707 |
| M+H | 795.1055 | 9.3 | 0706,0707 |
| M+H | 795.124 | 14.716 | 0708 |
| M+H | 795.1749 | 11.461 | 0709,0710,0711 |
| M+H | 795.1743 | 8.478 | 0709,0710,0711 |
| M+H | 795.1775 | 14.948 | 0711 |
| M+H | 795.2284 | 11.598 | 0712 |
| M+H | 795.2533 | 12.641 | 0713 |
| M+H | 795.2639 | 7.049 | 0714 |
| M+H | 796.1191 | 14.884 | 0715,0716 |
| M+H | 796.1195 | 14.927 | 0715,0716 |
| M+H | 796.1671 | 10.111 | 0717,0718 |
| M+H | 796.1681 | 10.054 | 0717,0718 |
| M+H | 796.1925 | 13.315 | 0719 |
| M+H | 796.2037 | 13.768 | 0720 |
| M+H | 796.2123 | 9.696 | 0721 |
| M-H | 794.2284 | 9.441 | 0722 |
| M-H | 795.1187 | 14.66 | 0723,0724 |
| M+H | 797.1369 | 11.492 | 0723,0724 |
| M+H | 797.1731 | 10.361 | 0725 |
| M+H | 797.2079 | 10.665 | 0726 |
| M+H | 798.1361 | 13.373 | 0727 |
| M+H | 798.1482 | 9.46 | 0728,0729,0730 |
| M+H | 798.1532 | 13.112 | 0728,0729,0730 |
| M-H | 796.1356 | 13.171 | 0728,0729,0730 |
| M+H | 798.2076 | 11.876 | 0731,0732 |
| M+H | 798.2077 | 13.256 | 0731,0732 |
| M+H | 798.2243 | 8.168 | 0733 |
| M+H | 798.2651 | 11.543 | 0734 |
| M-H | 797.1146 | 11.825 | 0735,0736 |
| M+H | 799.1332 | 11.876 | 0735,0736 |
| M+H | 799.1535 | 8.021 | 0737 |
| M+H | 799.2237 | 8.698 | 0738,0739 |
| M+H | 799.2278 | 10.558 | 0738,0739 |
| M+H | 800.1843 | 14.217 | 0740,0741 |
| M+H | 800.187 | 9.157 | 0741 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 800.2047 | 12.32 | 0742 |
| M+H | 800.2357 | 9.212 | 0743 |
| M+H | 801.1684 | 13.198 | 0744 |
| M+H | 801.1864 | 13.424 | 0745 |
| M+H | 801.204 | 15.147 | 0746,0747,0748 |
| M+H | 801.2042 | 14.756 | 0746,0747,0748 |
| M+H | 801.2036 | 15.229 | 0746,0747,0748 |
| M+H | 801.2242 | 12.969 | 0749 |
| M+H | 801.2804 | 15.525 | 0750 |
| M-H | 800.1447 | 13.106 | 0751 |
| M-H | 800.1503 | 12.536 | 0751,0752 |
| M+H | 802.2393 | 11.261 | 0753 |
| M+H | 803.1332 | 11.665 | 0754 |
| M-H | 801.1642 | 12.601 | 0755,0756,0757 |
| M+H | 803.1852 | 13.807 | 0756,0757 |
| M+H | 803.1852 | 13.96 | 0756,0757 |
| M+H | 803.2596 | 14.592 | 0758 |
| M+H | 804.1771 | 9.858 | 0759 |
| M+H | 804.1929 | 7.605 | 0760,0761 |
| M+H | 804.1998 | 15.147 | 0760,0761 |
| M+H | 804.2095 | 6.925 | 0762 |
| M+H | 804.2294 | 7.688 | 0763 |
| M+H | 804.2684 | 10.129 | 0764 |
| M+H | 805.1151 | 15.082 | 0765 |
| M-H | 803.1098 | 13.842 | 0766 |
| M+H | 805.1785 | 9.543 | 0767 |
| M-H | 804.1253 | 12.268 | 0768 |
| M+H | 806.1579 | 11.06 | 0769,0770 |
| M+H | 806.1583 | 12.049 | 0769,0770 |
| M+H | 806.1771 | 9.351 | 0771 |
| M+H | 806.1913 | 9.839 | 0772,0773 |
| M+H | 806.1941 | 11.974 | 0772,0773 |
| M-H | 804.1901 | 8.566 | 0774 |
| M+H | 806.233 | 14.039 | 0775 |
| M+H | 806.2698 | 13.272 | 0776 |
| M+H | 807.1244 | 14.014 | 0777 |
| M+H | 807.1428 | 13.797 | 0778 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 807.1906 | 12.107 | 0779 |
| M+H | 807.2081 | 13.848 | 0780,0781 |
| M+H | 807.2048 | 4.896 | 0780,0781 |
| M-H | 806.1065 | 13.907 | 0782 |
| M+H | 808.1434 | 10.495 | 0783 |
| M+H | 808.1716 | 11.885 | 0784,0785 |
| M+H | 808.1737 | 11.015 | 0784,0785 |
| M+H | 808.1966 | 13.821 | 0786,0787 |
| M-H | 806.1766 | 13.812 | 0786,0787 |
| M+H | 808.211 | 11.147 | 0788 |
| M+H | 809.1428 | 14.245 | 0789 |
| M-H | 807.1361 | 11.484 | 0790,0791 |
| M+H | 809.1545 | 14.096 | 0790,0791 |
| M+H | 809.1694 | 12.287 | 0792 |
| M+H | 809.1879 | 13.223 | 0793,0794 |
| M-H | 807.1711 | 10.884 | 0793,0794 |
| M+H | 809.2113 | 15.461 | 0795 |
| M+H | 809.2289 | 14.903 | 0796 |
| M-H | 808.1347 | 12.356 | 0797,0798 |
| M+H | 810.1513 | 10.354 | 0797,0798 |
| M-H | 808.1488 | 12.427 | 0799,0800 |
| M+H | 810.1698 | 11.614 | 0800,0801,0802 |
| M+H | 810.174 | 11.165 | 0801,0802 |
| M+H | 810.174 | 11.031 | 0801,0802 |
| M+H | 810.2443 | 13 | 0803 |
| M+H | 811.0845 | 15.539 | 0804 |
| M+H | 811.1707 | 7.595 | 0805,0806 |
| M+H | 811.1729 | 15.231 | 0805,0806 |
| M-H | 810.0811 | 13.677 | 0807 |
| M+H | 812.1511 | 11.534 | 0808 |
| M+H | 812.1731 | 11.412 | 0809 |
| M-H | 810.1809 | 11.55 | 0810 |
| M+H | 812.2801 | 12.012 | 0811 |
| M+H | 813.1655 | 12.94 | 0812,0813 |
| M-H | 811.1461 | 12.899 | 0812,0813 |
| M+H | 813.2396 | 7.74 | 0814 |
| M-H | 812.0944 | 12.863 | 0815 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 814.1295 | 13.459 | 0816 |
| M+H | 814.184 | 13.799 | 0817 |
| M+H | 814.2593 | 9.304 | 0818 |
| M+H | 815.1104 | 12.115 | 0819 |
| M-H | 813.1104 | 9.565 | 0820 |
| M+H | 815.2199 | 15.656 | 0821 |
| M+H | 815.2586 | 13.591 | 0822 |
| M+H | 816.145 | 13.579 | 0823,0824 |
| M-H | 814.1255 | 13.536 | 0823,0824 |
| M+H | 816.1792 | 13.131 | 0825,0826 |
| M+H | 816.1808 | 8.819 | 0825,0826 |
| M+H | 817.1622 | 13.293 | 0827 |
| M+H | 817.1994 | 14.748 | 0828,0829 |
| M+H | 817.1992 | 12.863 | 0828,0829 |
| M+H | 818.1597 | 11.096 | 0830,0831 |
| M+H | 818.1604 | 13.289 | 0830,0831 |
| M-H | 816.216 | 9.017 | 0832 |
| M-H | 817.1404 | 13.258 | 0833 |
| M+H | 819.1638 | 14.446 | 0834 |
| M+H | 820.1738 | 12.081 | 0835 |
| M+H | 820.2051 | 7.572 | 0836,0837,0838 |
| M+H | 820.208 | 13.126 | 0836,0837,0838 |
| M+H | 820.2095 | 13.148 | 0836,0837,0838 |
| M+H | 820.2305 | 11.759 | 0839 |
| M+H | 820.285 | 13.713 | 0840 |
| M+H | 821.1702 | 12.169 | 0841,0842 |
| M+H | 821.174 | 10.968 | 0842 |
| M+H | 821.2257 | 9.818 | 0843 |
| M+H | 822.1415 | 14.748 | 0844 |
| M+H | 822.1879 | 12.062 | 0845,0846 |
| M+H | 822.1899 | 10.044 | 0845,0846 |
| M+H | 822.2644 | 11.209 | 0847 |
| M+H | 823.1202 | 14.163 | 0848 |
| M+H | 823.1542 | 11.558 | 0849 |
| M+H | 823.1851 | 12.457 | 0850,0851 |
| M-H | 821.1693 | 14.738 | 0850,0851 |
| M+H | 823.2053 | 10.695 | 0852 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 823.2636 | 15.497 | 0853 |
| M-H | 822.1028 | 11.303 | 0854 |
| M-H | 822.1157 | 12.776 | 0855 |
| M+H | 824.1543 | 12.34 | 0856 |
| M+H | 824.1653 | 11.346 | 0857 |
| M+H | 824.1862 | 13.259 | 0858 |
| M+H | 825.1202 | 16.11 | 0859,0860 |
| M+H | 825.1202 | 16.256 | 0859,0860 |
| M+H | 825.1787 | 11.873 | 0861,0862,0863 |
| M+H | 825.1845 | 12.868 | 0862,0863 |
| M+H | 825.1845 | 10.85 | 0862,0863 |
| M+H | 826.1154 | 13.294 | 0864 |
| M+H | 826.1309 | 12.638 | 0865 |
| M-H | 824.1315 | 10.254 | 0866 |
| M+H | 826.185 | 15.108 | 0867 |
| M-H | 825.0953 | 14.08 | 0868 |
| M+H | 827.1445 | 11.641 | 0869 |
| M+H | 827.1837 | 15.307 | 0870 |
| M+H | 828.1607 | 12.906 | 0871 |
| M-H | 826.2161 | 13.491 | 0872 |
| M+H | 829.1455 | 15.057 | 0873,0874 |
| M+H | 829.147 | 10.116 | 0873,0874 |
| M-H | 827.2192 | 15.794 | 0875 |
| M-H | 828.0722 | 14.276 | 0876 |
| M+H | 831.156 | 13.42 | 0877 |
| M+H | 832.1738 | 13.717 | 0878 |
| M+H | 832.2411 | 9.487 | 0879 |
| M+H | 833.1609 | 10.281 | 0880 |
| M+H | 833.1944 | 11.825 | 0881 |
| M+H | 833.2257 | 11.568 | 0882 |
| M+H | 834.2208 | 11.48 | 0883 |
| M+H | 835.101 | 12.699 | 0884 |
| M+H | 835.1546 | 11.289 | 0885 |
| M+H | 835.1698 | 13.717 | 0886 |
| M+H | 835.1788 | 11.234 | 0887 |
| M-H | 834.1511 | 12.167 | 0888 |
| M+H | 836.2048 | 11.512 | 0889,0890 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 836.2031 | 13.18 | 0889,0890 |
| M-H | 835.1118 | 13.491 | 0891,0892 |
| M-H | 835.115 | 14.28 | 0891,0892,0893 |
| M+H | 837.1356 | 14.393 | 0893 |
| M-H | 835.1481 | 9.088 | 0894 |
| M+H | 837.168 | 9.703 | 0895 |
| M+H | 838.165 | 10.345 | 0896 |
| M+H | 839.1362 | 15.586 | 0897 |
| M+H | 840.1294 | 17.199 | 0898 |
| M+H | 840.1799 | 12.808 | 0899 |
| M+H | 841.1257 | 14.269 | 0900 |
| M+H | 841.1486 | 14.037 | 0901 |
| M+H | 841.1603 | 11.957 | 0902 |
| M+H | 841.1741 | 9.538 | 0903 |
| M-H | 839.1971 | 11.717 | 0904 |
| M+H | 841.2352 | 15.295 | 0905 |
| M+H | 842.1256 | 12.742 | 0906 |
| M+H | 842.1644 | 12.583 | 0907 |
| M-H | 841.0722 | 15.994 | 0908 |
| M+H | 843.111 | 15.191 | 0909,0910 |
| M+H | 843.1097 | 12.395 | 0909,0910 |
| M+H | 843.1592 | 9.874 | 0911 |
| M+H | 843.1788 | 15.433 | 0912 |
| M+H | 844.1253 | 13.366 | 0913 |
| M+H | 844.1566 | 14.901 | 0914 |
| M-H | 842.1715 | 7.867 | 0915 |
| M+H | 845.1204 | 10.004 | 0916,0917 |
| M-H | 843.1015 | 12.688 | 0916,0917 |
| M+H | 845.1412 | 15.125 | 0918,0919 |
| M+H | 845.1389 | 12.985 | 0918,0919 |
| M+H | 846.1215 | 13.424 | 0920 |
| M+H | 846.189 | 14.089 | 0921 |
| M+H | 848.1513 | 13.895 | 0922,0923 |
| M-H | 846.1317 | 13.758 | 0922,0923 |
| M+H | 848.2375 | 6.972 | 0924 |
| M+H | 849.1459 | 12.858 | 0925 |
| M-H | 847.2019 | 13.517 | 0926 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 849.2257 | 13.524 | 0927 |
| M+H | 851.0777 | 14.279 | 0928 |
| M+H | 851.1796 | 11.886 | 0929 |
| M+H | 851.189 | 14.865 | 0930 |
| M+H | 852.1427 | 13.328 | 0931 |
| M+H | 852.1959 | 7.251 | 0932 |
| M+H | 852.2353 | 12.187 | 0933 |
| M+H | 853.1298 | 14.526 | 0934 |
| M+H | 853.1523 | 16.121 | 0935 |
| M+H | 853.1629 | 12.727 | 0936 |
| M+H | 854.1458 | 17.656 | 0937 |
| M+H | 854.1592 | 12.159 | 0938 |
| M+H | 855.1311 | 14.967 | 0939 |
| M-H | 854.1208 | 13.193 | 0940 |
| M-H | 854.1605 | 12.886 | 0941 |
| M+H | 857.1537 | 9.852 | 0942 |
| M+H | 857.2299 | 13.517 | 0943 |
| M+H | 858.1209 | 17.177 | 0944 |
| M+H | 858.1417 | 13.736 | 0945 |
| M+H | 859.0867 | 14.127 | 0946 |
| M+H | 859.1363 | 8.624 | 0947,0948 |
| M+H | 859.1372 | 14.851 | 0947,0948 |
| M+H | 860.1321 | 9.998 | 0949 |
| M+H | 860.1516 | 15.026 | 0950,0951 |
| M+H | 860.1502 | 12.819 | 0950,0951 |
| M+H | 860.166 | 13.242 | 0952 |
| M+H | 860.1791 | 9.618 | 0953 |
| M+H | 860.2411 | 13.837 | 0954 |
| M+H | 861.1014 | 15.389 | 0955 |
| M-H | 860.0774 | 14.843 | 0956 |
| M-H | 860.0957 | 13.43 | 0957,0958 |
| M-H | 860.0957 | 13.37 | 0957,0958 |
| M+H | 862.1836 | 14.179 | 0959 |
| M+H | 863.1623 | 13.735 | 0960 |
| M+H | 864.1468 | 13.975 | 0961,0962 |
| M+H | 864.1457 | 11.692 | 0961,0962 |
| M+H | 868.1616 | 17.992 | 0963 |

(continued)

| [Table 8-11-3] Compound that may be contained in mixture 2-1-m1E03-1 | | | |
|---|---|---|---|
| Ion species | m/z $[M+H]^+$ or $[M-H]^-$ | Retention time of MS peak (min) | No. of assigned compound described with 2-1-m1E03-1-omitted |
| M+H | 868.2309 | 12.281 | 0964 |
| M+H | 870.1415 | 15.007 | 0965 |
| M+H | 870.1962 | 13.377 | 0966 |
| M-H | 870.116 | 13.256 | 0967 |
| M+H | 872.1575 | 14.343 | 0968 |
| M-H | 870.1559 | 10.793 | 0969 |
| M+H | 873.1527 | 15.519 | 0970 |
| M+H | 873.1744 | 14.864 | 0971 |
| M+H | 874.1376 | 11.546 | 0972 |
| M+H | 875.1355 | 16.016 | 0973 |
| M+H | 876.1122 | 17.611 | 0974 |
| M+H | 876.1613 | 13.279 | 0975 |
| M-H | 874.2163 | 13.872 | 0976 |
| M+H | 877.1283 | 15.54 | 0977 |
| M-H | 876.073 | 14.971 | 0978 |
| M-H | 876.1254 | 13.096 | 0979 |
| M+H | 879.1572 | 13.813 | 0980 |
| M-H | 878.0869 | 13.902 | 0981 |
| M+H | 887.1686 | 16.221 | 0982 |
| M+H | 889.1466 | 14.703 | 0983 |
| M+H | 890.1438 | 18.051 | 0984 |
| M-H | 889.1263 | 14.586 | 0985 |
| M+H | 893.1085 | 15.969 | 0986 |
| M+H | 895.1151 | 15.874 | 0987 |
| M+H | 899.1317 | 13.073 | 0988 |
| M+H | 901.0928 | 14.047 | 0989 |
| M+H | 907.1411 | 14.861 | 0990 |
| M-H | 906.0962 | 17.806 | 0991 |
| M+H | 909.1021 | 16.104 | 0992 |
| M+H | 910.1503 | 13.472 | 0993 |
| M+H | 912.1119 | 14.425 | 0994 |
| M+H | 916.1044 | 15.79 | 0995 |
| M+H | 924.1138 | 17.873 | 0996 |
| M+H | 926.1463 | 13.581 | 0997 |
| M+H | 927.124 | 16.46 | 0998 |
| M+H | 928.1085 | 14.595 | 0999 |
| M+H | 943.1189 | 16.436 | 1000 |

[Table 410]

| [Table 8-11-4] Compound that may be contained in mixture 2-1-m1E04-1 | | | |
|---|---|---|---|
| Ion species | m/z [M+H]$^+$ or [M-H]$^-$ | Retention time of MS peak (min) | No. of assigned compound described with 2-1-m1E04-1-omitted |
| M+H | 754.3053 | 9.098 | 0001 |
| M+H | 755.2998 | 8.507 | 0002,0003 |
| M+H | 755.3 | 7.82 | 0002,0003 |
| M+H | 756.2956 | 9.102 | 0004,0005 |
| M+H | 756.2958 | 7.155 | 0004,0005 |
| M+H | 757.2905 | 7.934 | 0006 |
| M+H | 761.2564 | 9.175 | 0007 |
| M+H | 762.2515 | 7.976 | 0008 |
| M+H | 768.3204 | 9.47 | 0009 |
| M+H | 769.316 | 8.902 | 0010 |
| M+H | 770.3116 | 9.427 | 0011 |
| M+H | 772.279 | 8.04 | 0012 |
| M+H | 772.2954 | 9.195 | 0013 |
| M+H | 773.2741 | 6.886 | 0014 |
| M+H | 773.2909 | 8.626 | 0015 |
| M+H | 773.3108 | 7.792 | 0016 |
| M+H | 774.2859 | 9.096 | 0017 |
| M+H | 774.306 | 6.947 | 0018,0019 |
| M+H | 774.3061 | 6.522 | 0018,0019 |
| M+H | 775.2727 | 9.532 | 0020 |
| M+H | 775.3011 | 5.38 | 0021,0022 |
| M+H | 775.3013 | 7.882 | 0021,0022 |
| M+H | 776.2959 | 6.525 | 0023 |
| M+H | 779.2475 | 9.225 | 0024 |
| M+H | 780.2629 | 7.799 | 0025 |
| M+H | 781.2573 | 6.271 | 0026 |
| M+H | 782.2996 | 7.932 | 0027 |
| M+H | 782.3364 | 9.837 | 0028 |
| M+H | 783.2956 | 7.805 | 0029,0030 |
| M+H | 783.2947 | 6.814 | 0029,0030 |
| M+H | 783.3316 | 9.303 | 0031 |
| M+H | 784.2907 | 6.694 | 0032 |
| M+H | 784.3155 | 8.559 | 0033 |
| M+H | 784.327 | 9.8 | 0034 |
| M+H | 785.3103 | 7.378 | 0035 |
| M+H | 786.2952 | 8.358 | 0036 |
| M+H | 786.306 | 9.066 | 0037 |

(continued)

| [Table 8-11-4] Compound that may be contained in mixture 2-1-m1E04-1 | | | |
|---|---|---|---|
| Ion species | m/z $[M+H]^+$ or $[M-H]^-$ | Retention time of MS peak (min) | No. of assigned compound described with 2-1-m1E04-1-omitted |
| M+H | 787.3262 | 8.096 | 0038 |
| M-H | 786.2391 | 8.154 | 0039 |
| M+H | 788.3214 | 7.279 | 0040 |
| M+H | 789.2522 | 7.114 | 0041 |
| M+H | 789.288 | 9.886 | 0042 |
| M+H | 789.3176 | 7.481 | 0043 |
| M+H | 790.2702 | 8.106 | 0044 |
| M+H | 790.2867 | 9.424 | 0045 |
| M+H | 791.2675 | 9.175 | 0046 |
| M+H | 791.2812 | 8.894 | 0047,0048 |
| M+H | 791.2852 | 6.999 | 0047,0048 |
| M+H | 791.3011 | 7.911 | 0049 |
| M+H | 792.2798 | 5.53 | 0050,0051 |
| M+H | 792.2802 | 6.619 | 0050,0051 |
| M+H | 792.2962 | 7.098 | 0052 |
| M+H | 793.2914 | 7.912 | 0053 |
| M+H | 794.2776 | 8.062 | 0054 |
| M+H | 796.3156 | 8.246 | 0055 |
| M+H | 797.2381 | 9.44 | 0056 |
| M+H | 797.311 | 8.139 | 0057,0058 |
| M+H | 797.3116 | 7.813 | 0057,0058 |
| M+H | 798.253 | 7.881 | 0059 |
| M+H | 798.3058 | 6.699 | 0060 |
| M+H | 800.29 | 8.043 | 0061,0062 |
| M+H | 800.2901 | 8.074 | 0061,0062 |
| M+H | 800.311 | 8.716 | 0063 |
| M+H | 801.2861 | 7.048 | 0064,0065 |
| M-H | 799.269 | 7.924 | 0064,0065 |
| M+H | 801.3056 | 6.886 | 0066 |
| M+H | 801.3418 | 8.476 | 0067 |
| M-H | 800.2547 | 8.392 | 0068 |
| M+H | 802.2902 | 7.467 | 0069 |
| M+H | 802.3016 | 5.342 | 0070,0071 |
| M+H | 802.3045 | 7.248 | 0070,0071 |
| M+H | 802.3373 | 7.696 | 0072 |
| M+H | 803.2961 | 5.436 | 0073 |
| M+H | 803.3196 | 7.215 | 0074 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 803.3331 | 7.766 | 0075 |
| M+H | 804.3164 | 6.226 | 0076,0077 |
| M+H | 804.3204 | 9.874 | 0076,0077 |
| M+H | 805.3006 | 7.257 | 0078 |
| M+H | 805.3161 | 9.343 | 0079,0080 |
| M+H | 805.3162 | 9.274 | 0079,0080 |
| M+H | 806.2473 | 8.239 | 0081 |
| M+H | 806.3113 | 9.676 | 0082,0083 |
| M+H | 806.3111 | 8.547 | 0082,0083 |
| M+H | 807.2622 | 6.94 | 0084 |
| M+H | 807.3079 | 6.213 | 0085 |
| M+H | 808.247 | 8.588 | 0086 |
| M+H | 808.2567 | 5.517 | 0087,0088 |
| M+H | 808.2603 | 8.29 | 0087,0088 |
| M+H | 808.2937 | 8.381 | 0089 |
| M+H | 809.2413 | 7.389 | 0090 |
| M+H | 809.2762 | 7.065 | 0091 |
| M+H | 809.2924 | 8.38 | 0092 |
| M+H | 810.277 | 9.831 | 0093,0094 |
| M+H | 810.2784 | 7.064 | 0093,0094 |
| M+H | 810.287 | 7.626 | 0095 |
| M+H | 810.2979 | 7.919 | 0096 |
| M+H | 810.3315 | 8.583 | 0097 |
| M+H | 811.2718 | 8.543 | 0098,0099 |
| M-H | 809.2554 | 9.852 | 0098,0099 |
| M+H | 811.2821 | 8.292 | 0100 |
| M+H | 811.293 | 6.68 | 0101 |
| M+H | 811.3272 | 8.485 | 0102,0103 |
| M+H | 811.3267 | 8.104 | 0102,0103 |
| M+H | 812.3101 | 7.978 | 0104 |
| M+H | 813.3047 | 6.973 | 0105 |
| M+H | 814.3065 | 8.352 | 0106 |
| M-H | 812.3459 | 8.31 | 0107 |
| M-H | 813.2841 | 7.944 | 0108 |
| M+H | 815.3216 | 7.16 | 0109 |
| M+H | 815.3579 | 7.123 | 0110 |
| M+H | 816.2433 | 8.3 | 0111 |

(continued)

| [Table 8-11-4] Compound that may be contained in mixture 2-1-m1E04-1 | | | |
|---|---|---|---|
| Ion species | m/z [M+H]$^+$ or [M-H]$^-$ | Retention time of MS peak (min) | No. of assigned compound described with 2-1-m1E04-1-omitted |
| M+H | 816.2844 | 7.249 | 0112 |
| M+H | 816.3161 | 6.516 | 0113,0114 |
| M+H | 816.3164 | 4.809 | 0113,0114 |
| M+H | 817.3119 | 5.119 | 0115 |
| M+H | 818.267 | 8.709 | 0116,0117,0118 |
| M+H | 818.2672 | 7.749 | 0116,0117,0118 |
| M-H | 816.2549 | 7.223 | 0116,0117,0118 |
| M+H | 818.2813 | 8.158 | 0119,0120 |
| M-H | 816.2641 | 8.259 | 0119,0120 |
| M+H | 819.2624 | 8.072 | 0121,0122,0123 |
| M+H | 819.2619 | 7.498 | 0121,0122,0123 |
| M+H | 819.2617 | 6.556 | 0121,0122,0123 |
| M+H | 819.275 | 8.07 | 0124 |
| M+H | 819.2961 | 6.843 | 0125,0126 |
| M-H | 817.2796 | 6.988 | 0125,0126 |
| M+H | 819.3164 | 7.609 | 0127 |
| M+H | 820.2577 | 6.626 | 0128,0129 |
| M+H | 820.2574 | 9.052 | 0128,0129 |
| M+H | 820.2959 | 6.969 | 0130,0131 |
| M+H | 820.2917 | 6.966 | 0130,0131 |
| M+H | 820.325 | 8.939 | 0132 |
| M+H | 821.2521 | 7.618 | 0133 |
| M+H | 821.2784 | 7.179 | 0134 |
| M+H | 821.2955 | 6.301 | 0135 |
| M+H | 822.2591 | 6.396 | 0136 |
| M+H | 822.292 | 9.797 | 0137,0138,0139 |
| M+H | 822.2961 | 8.651 | 0137,0138,0139 |
| M+H | 822.2926 | 9.303 | 0137,0138,0139 |
| M+H | 823.2875 | 8.109 | 0140,0141 |
| M+H | 823.291 | 7.462 | 0140,0141 |
| M+H | 824.2239 | 8.847 | 0142 |
| M+H | 824.2379 | 8.412 | 0143 |
| M+H | 824.2827 | 9.696 | 0144 |
| M+H | 824.293 | 10.164 | 0145 |
| M+H | 824.3041 | 6.327 | 0146 |
| M+H | 824.3112 | 10.436 | 0147 |
| M+H | 825.2187 | 7.673 | 0148,0149 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 825.2186 | 8.95 | 0148,0149 |
| M+H | 825.2527 | 7.014 | 0150 |
| M+H | 825.3167 | 7.183 | 0151 |
| M+H | 825.3426 | 8.438 | 0152 |
| M+H | 826.2136 | 7.579 | 0153 |
| M+H | 826.2371 | 8.686 | 0154 |
| M+H | 826.3126 | 6.967 | 0155 |
| M+H | 827.252 | 7.64 | 0156 |
| M-H | 825.2485 | 7.456 | 0157 |
| M+H | 827.322 | 7.84 | 0158 |
| M+H | 828.2474 | 5.962 | 0159 |
| M+H | 828.2693 | 7.439 | 0160 |
| M+H | 828.2883 | 8.004 | 0161 |
| M+H | 828.3228 | 8.668 | 0162 |
| M+H | 829.2445 | 9.774 | 0164 |
| M+H | 829.2836 | 8.418 | 0165 |
| M+H | 829.3044 | 6.8 | 0166 |
| M+H | 829.3367 | 7.513 | 0167 |
| M+H | 830.2783 | 8.353 | 0168,0169 |
| M+H | 830.2809 | 10.848 | 0168,0169 |
| M+H | 830.2991 | 6.233 | 0170,0171,0172 |
| M+H | 830.2995 | 5.176 | 0170,0171,0172 |
| M+H | 830.3013 | 8.071 | 0170,0171,0172 |
| M-H | 828.3149 | 6.773 | 0173,0174 |
| M+H | 830.3347 | 7.121 | 0173,0174 |
| M+H | 831.2732 | 7.748 | 0175 |
| M+H | 831.2966 | 6.998 | 0176 |
| M+H | 831.3157 | 6.212 | 0177 |
| M+H | 832.269 | 8.306 | 0178 |
| M+H | 832.2823 | 9.077 | 0179,0180,0181 |
| M+H | 832.2833 | 7.962 | 0179,0180,0181 |
| M+H | 832.2833 | 8.073 | 0179,0180,0181 |
| M+H | 832.3099 | 4.909 | 0182 |
| M+H | 832.316 | 8.607 | 0182,0183 |
| M+H | 832.356 | 3.922 | 0184 |
| M+H | 833.2773 | 8.476 | 0185,0186,0187 |
| M+H | 833.2772 | 6.749 | 0185,0186,0187 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 833.2771 | 7.217 | 0185,0186,0187 |
| M+H | 833.2917 | 8.07 | 0188 |
| M+H | 833.3124 | 7.067 | 0189,0190 |
| M+H | 833.3119 | 8.473 | 0189,0190 |
| M+H | 833.3686 | 7.103 | 0191 |
| M+H | 834.2772 | 7.298 | 0192,0193 |
| M+H | 834.2763 | 7.48 | 0192,0193 |
| M+H | 834.307 | 6.611 | 0194 |
| M+H | 834.342 | 9.323 | 0195 |
| M+H | 834.3629 | 5.626 | 0196 |
| M+H | 835.2939 | 7.528 | 0197 |
| M+H | 836.2413 | 8.029 | 0198 |
| M+H | 836.2574 | 8.814 | 0199,0200 |
| M+H | 836.2574 | 7.915 | 0199,0200 |
| M+H | 836.2719 | 7.284 | 0201 |
| M-H | 834.2595 | 17.369 | 0201,0202 |
| M+H | 836.3076 | 9.695 | 0203 |
| M+H | 836.3219 | 8.669 | 0204 |
| M+H | 837.2293 | 8.3 | 0205 |
| M+H | 837.2363 | 6.507 | 0206 |
| M+H | 837.2526 | 8.194 | 0207 |
| M+H | 837.2724 | 7.698 | 0208,0209,0210 |
| M+H | 837.2745 | 7.476 | 0208,0209,0210 |
| M+H | 837.2741 | 8.294 | 0208,0209,0210 |
| M+H | 837.2866 | 6.915 | 0211,0212 |
| M+H | 837.287 | 7.341 | 0211,0212 |
| M+H | 838.2405 | 9.156 | 0213 |
| M+H | 838.2477 | 9.156 | 0214 |
| M+H | 838.2554 | 7.792 | 0215 |
| M+H | 838.2682 | 6.57 | 0216,0217,0218,0219 |
| M+H | 838.267 | 7.037 | 0216,0217,0218 |
| M+H | 838.2678 | 5.736 | 0216,0217,0218,0219 |
| M+H | 838.2762 | 7.995 | 0219 |
| M+H | 838.2857 | 8.853 | 0220,0221 |
| M+H | 838.2878 | 9.896 | 0220,0221 |
| M+H | 838.3082 | 10.528 | 0222 |
| M-H | 836.3123 | 8.681 | 0223 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 839.2345 | 9.319 | 0224 |
| M+H | 839.2629 | 4.759 | 0225,0226 |
| M+H | 839.263 | 4.898 | 0225,0226 |
| M+H | 839.283 | 9.402 | 0227 |
| M+H | 839.3321 | 7.496 | 0228 |
| M+H | 840.2582 | 5.91 | 0229 |
| M+H | 840.2705 | 6.605 | 0230 |
| M+H | 840.278 | 9.849 | 0231 |
| M+H | 840.2877 | 10.619 | 0232,0233 |
| M+H | 840.2873 | 6.783 | 0232,0233 |
| M+H | 840.3057 | 9.635 | 0234 |
| M+H | 841.2677 | 7.92 | 0235 |
| M+H | 841.298 | 8.693 | 0236,0237 |
| M+H | 841.2985 | 8.129 | 0236,0237 |
| M+H | 842.2141 | 8.963 | 0238 |
| M+H | 842.2922 | 8.897 | 0239,0240,0241 |
| M+H | 842.2928 | 6.45 | 0239,0240,0241 |
| M+H | 842.2933 | 8 | 0239,0240,0241 |
| M+H | 842.3942 | 8.965 | 0242 |
| M+H | 843.2092 | 9.052 | 0243 |
| M+H | 843.2297 | 7.856 | 0244 |
| M+H | 843.2431 | 7.442 | 0245 |
| M+H | 843.289 | 8.611 | 0246 |
| M+H | 843.2982 | 5.749 | 0247 |
| M+H | 843.3068 | 7.318 | 0248 |
| M-H | 841.3024 | 7.142 | 0249 |
| M+H | 844.2236 | 6.185 | 0250,0251,0252 |
| M+H | 844.2247 | 7.188 | 0250,0251,0252 |
| M+H | 844.2268 | 8.874 | 0250,0251,0252 |
| M+H | 844.3194 | 9.427 | 0253,0254 |
| M+H | 844.3188 | 9.637 | 0253,0254 |
| M+H | 844.3481 | 7.083 | 0255 |
| M+H | 844.3715 | 8.322 | 0256 |
| M+H | 845.2198 | 5.813 | 0257 |
| M+H | 845.2387 | 9.911 | 0258,0259 |
| M+H | 845.2421 | 7.744 | 0258,0259 |
| M+H | 845.3132 | 6.684 | 0260 |

(continued)

| [Table 8-11-4] Compound that may be contained in mixture 2-1-m1E04-1 | | | |
|---|---|---|---|
| Ion species | m/z $[M+H]^+$ or $[M-H]^-$ | Retention time of MS peak (min) | No. of assigned compound described with 2-1-m1E04-1-omitted |
| M+H | 846.2626 | 7.807 | 0261,0262 |
| M+H | 846.2584 | 10.105 | 0261,0262 |
| M+H | 846.2763 | 8.18 | 0263 |
| M+H | 846.2983 | 9.446 | 0264,0265,0266,0267 |
| M+H | 846.2985 | 8.507 | 0264,0265,0266,0267 |
| M+H | 846.295 | 4.33 | 0264,0265,0266,0267 |
| M-H | 844.2813 | 8.349 | 0264,0265,0266,0267 |
| M+H | 846.3267 | 5.89 | 0268 |
| M+H | 847.2567 | 6.551 | 0269,0270 |
| M+H | 847.2561 | 6.923 | 0269,0270 |
| M+H | 847.2769 | 6.409 | 0271 |
| M+H | 847.2933 | 8.883 | 0272,0273,0274,0275 |
| M+H | 847.2911 | 7.064 | 0272,0273,0274,0275 |
| M+H | 847.2946 | 0.656 | 0272,0273,0274,0275 |
| M+H | 847.2948 | 6.91 | 0272,0273,0274,0275 |
| M+H | 847.3271 | 7.404 | 0276,0277 |
| M+H | 847.3264 | 8.452 | 0276,0277 |
| M-H | 845.3665 | 7.382 | 0278 |
| M+H | 848.252 | 7.367 | 0279,0280,0281 |
| M+H | 848.2524 | 6.606 | 0279,0280,0281 |
| M+H | 848.2498 | 8.603 | 0279,0280,0281 |
| M+H | 848.277 | 7.6 | 0282,0283 |
| M+H | 848.2766 | 8.378 | 0282,0283 |
| M+H | 848.2891 | 9.811 | 0284 |
| M+H | 848.2935 | 9.343 | 0284,0285 |
| M+H | 849.2723 | 6.405 | 0286 |
| M+H | 849.3067 | 6.051 | 0287,0288 |
| M+H | 849.3062 | 6.185 | 0287,0288 |
| M+H | 850.2577 | 8.434 | 0289 |
| M+H | 850.268 | 5.912 | 0290 |
| M+H | 850.2733 | 8.314 | 0290,0291,0292 |
| M+H | 850.2719 | 11.491 | 0290,0291,0292 |
| M+H | 850.2878 | 8.607 | 0293 |
| M+H | 850.302 | 5.751 | 0294,0295 |
| M-H | 848.2891 | 8.682 | 0294,0295 |
| M+H | 850.324 | 10.049 | 0296 |
| M-H | 848.3256 | 8.631 | 0297 |

(continued)

| [Table 8-11-4] Compound that may be contained in mixture 2-1-m1E04-1 | | | |
|---|---|---|---|
| Ion species | m/z [M+H]$^+$ or [M-H]$^-$ | Retention time of MS peak (min) | No. of assigned compound described with 2-1-m1E04-1-omitted |
| M+H | 851.2728 | 7.132 | 0298,0299 |
| M+H | 851.269 | 9.168 | 0298,0299 |
| M+H | 851.2828 | 8.498 | 0300 |
| M+H | 851.2883 | 7.99 | 0301,0302,0303 |
| M+H | 851.2888 | 7.04 | 0301,0302,0303 |
| M+H | 851.2889 | 7.786 | 0301,0302,0303 |
| M+H | 851.3585 | 7.215 | 0304 |
| M+H | 852.2182 | 8.091 | 0305 |
| M+H | 852.2543 | 8.058 | 0306 |
| M+H | 852.2837 | 6.921 | 0307,0308,0309 |
| M+H | 852.2846 | 5.225 | 0307,0308,0309 |
| M+H | 852.2846 | 5.359 | 0307,0308,0309 |
| M+H | 852.2956 | 7.006 | 0310 |
| M+H | 852.3042 | 9.343 | 0311 |
| M+H | 853.2134 | 6.706 | 0312 |
| M+H | 853.251 | 9.704 | 0313 |
| M+H | 853.279 | 8.06 | 0314,0315 |
| M+H | 853.2814 | 5.876 | 0314,0315 |
| M+H | 853.3482 | 7.886 | 0316 |
| M+H | 854.2314 | 8.162 | 0317,0318 |
| M+H | 854.238 | 8.903 | 0318 |
| M+H | 854.248 | 9.016 | 0319,0320 |
| M+H | 854.248 | 8.138 | 0319,0320 |
| M+H | 855.2295 | 8.944 | 0321 |
| M+H | 855.2432 | 8.458 | 0322,0323 |
| M+H | 855.2468 | 6.926 | 0322,0323 |
| M+H | 855.2633 | 7.605 | 0324,0325 |
| M+H | 855.2627 | 6.973 | 0324,0325 |
| M-H | 853.2604 | 7.34 | 0326 |
| M+H | 855.283 | 8.285 | 0326,0327 |
| M+H | 855.3136 | 8.41 | 0328 |
| M+H | 855.3269 | 6.542 | 0329 |
| M+H | 856.2421 | 4.933 | 0330,0331,0332 |
| M+H | 856.24 | 9.34 | 0330,0331,0332 |
| M+H | 856.2436 | 8.78 | 0330,0331,0332 |
| M+H | 856.2579 | 6.733 | 0333,0334 |
| M+H | 856.2658 | 7.773 | 0334 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 856.326 | 9.392 | 0335 |
| M+H | 857.2457 | 8.135 | 0336 |
| M+H | 857.2531 | 7.907 | 0337 |
| M+H | 857.2654 | 6.975 | 0338 |
| M+H | 857.2924 | 8.706 | 0339 |
| M+H | 857.3139 | 6.121 | 0340 |
| M+H | 857.3352 | 7.504 | 0341 |
| M+H | 858.2395 | 5.525 | 0342 |
| M+H | 858.2624 | 9.266 | 0343 |
| M+H | 858.273 | 7.32 | 0344,0345 |
| M+H | 858.2737 | 9.608 | 0344,0345 |
| M+H | 858.2877 | 6.196 | 0346 |
| M+H | 858.3343 | 9.795 | 0347,0348,0349 |
| M+H | 858.3345 | 10.016 | 0347,0348,0349 |
| M+H | 858.3345 | 9.76 | 0347,0348,0349 |
| M-H | 857.251 | 7.228 | 0350 |
| M+H | 859.2689 | 7.211 | 0350,0351 |
| M+H | 859.2835 | 8.706 | 0352 |
| M+H | 859.2887 | 8.231 | 0352,0353 |
| M+H | 859.2938 | 6.302 | 0353,0354 |
| M+H | 859.3139 | 6.744 | 0355 |
| M+H | 859.3301 | 8.333 | 0356 |
| M-H | 858.1873 | 9.153 | 0357 |
| M+H | 860.2778 | 10.903 | 0358 |
| M+H | 860.2939 | 6.802 | 0359 |
| M+H | 860.3134 | 8.566 | 0360,0361,0362,0363 |
| M+H | 860.3132 | 9.276 | 0360,0361,0362,0363 |
| M+H | 860.3119 | 8.628 | 0360,0361,0362,0363 |
| M+H | 860.317 | 8.705 | 0360,0361,0362,0363 |
| M+H | 861.1996 | 9.187 | 0364 |
| M+H | 861.2198 | 7.973 | 0365 |
| M+H | 861.2729 | 7.718 | 0366,0367 |
| M+H | 861.2726 | 10.365 | 0366,0367 |
| M+H | 861.2978 | 7.791 | 0368 |
| M+H | 861.3087 | 6.305 | 0369 |
| M+H | 861.3993 | 7.699 | 0370 |
| M+H | 862.2145 | 7.743 | 0371 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 862.2669 | 6.664 | 0372 |
| M+H | 862.292 | 7.95 | 0373,0374 |
| M-H | 860.2767 | 8.755 | 0373,0374 |
| M+H | 862.309 | 9.524 | 0375 |
| M-H | 861.2153 | 8.143 | 0376 |
| M+H | 863.2909 | 7.021 | 0377,0378 |
| M+H | 863.2861 | 8.727 | 0377,0378 |
| M+H | 863.3247 | 8.091 | 0379,0380 |
| M+H | 863.3222 | 6.432 | 0379,0380 |
| M+H | 863.3782 | 6.51 | 0381 |
| M+H | 864.2293 | 7.442 | 0382 |
| M+H | 864.245 | 8.673 | 0383 |
| M+H | 864.2522 | 7.946 | 0384,0385 |
| M+H | 864.2518 | 7.15 | 0384,0385 |
| M+H | 864.2694 | 6.878 | 0386 |
| M+H | 864.2929 | 6.131 | 0387,0388 |
| M+H | 864.2882 | 8.638 | 0387,0388 |
| M-H | 862.301 | 7.427 | 0389 |
| M+H | 864.3752 | 11.528 | 0390 |
| M+H | 865.2484 | 6.985 | 0391,0392 |
| M+H | 865.2502 | 5.752 | 0391,0392 |
| M+H | 865.2639 | 8.995 | 0393,0394 |
| M+H | 865.2668 | 6.816 | 0393,0394 |
| M+H | 865.2857 | 7.284 | 0395 |
| M+H | 865.2985 | 8.473 | 0396 |
| M+H | 865.304 | 8.355 | 0397,0398,0399,0400 |
| M+H | 865.3034 | 7.222 | 0396,0397,0398,0399,0400 |
| M+H | 865.3044 | 8.171 | 0397,0398,0399,0400 |
| M+H | 865.3032 | 9.2 | 0396,0397,0398,0399,0400 |
| M+H | 866.2333 | 8.524 | 0401 |
| M+H | 866.2506 | 8.107 | 0402 |
| M+H | 866.2629 | 5.043 | 0403,0404 |
| M+H | 866.2675 | 7.117 | 0403,0404 |
| M+H | 866.2828 | 8.727 | 0405,0406 |
| M+H | 866.284 | 9.628 | 0405,0406 |
| M+H | 866.2978 | 7.359 | 0407,0408,0409 |
| M+H | 866.2969 | 7.282 | 0407,0408,0409 |

(continued)

| [Table 8-11-4] Compound that may be contained in mixture 2-1-m1E04-1 | | | |
|---|---|---|---|
| Ion species | m/z [M+H]$^+$ or [M-H]$^-$ | Retention time of MS peak (min) | No. of assigned compound described with 2-1-m1E04-1-omitted |
| M+H | 866.2989 | 4.643 | 0407,0408,0409 |
| M+H | 867.2584 | 5.216 | 0410 |
| M+H | 867.2783 | 7.404 | 0411 |
| M+H | 867.283 | 7.165 | 0411,0412,0413 |
| M+H | 867.284 | 6.267 | 0412,0413 |
| M+H | 867.2928 | 7.296 | 0414 |
| M+H | 867.2995 | 7.916 | 0414,0415 |
| M+H | 868.2633 | 8.267 | 0416,0417 |
| M+H | 868.2611 | 9.024 | 0416,0417 |
| M+H | 868.2785 | 5.977 | 0418,0419,0420,0421 |
| M-H | 866.2603 | 8.702 | 0418,0419 |
| M+H | 868.2822 | 9.472 | 0418,0419,0420,0421 |
| M+H | 868.2827 | 8.494 | 0418,0419,0420,0421 |
| M+H | 869.2592 | 8.492 | 0422,0423 |
| M+H | 869.2621 | 7.306 | 0422,0423 |
| M+H | 869.2737 | 7.284 | 0424,0425 |
| M+H | 869.2786 | 7.302 | 0424,0425,0426,0427 |
| M+H | 869.2774 | 8.923 | 0424,0425,0426,0427 |
| M+H | 869.2782 | 8.179 | 0424,0425,0426,0427 |
| M+H | 869.2926 | 7.714 | 0428 |
| M+H | 869.3299 | 8.767 | 0429 |
| M+H | 869.3493 | 7.644 | 0430 |
| M-H | 868.1911 | 8.242 | 0431 |
| M+H | 870.2724 | 7.955 | 0432,0433,0434,0435 |
| M+H | 870.2734 | 4.702 | 0432,0433,0434,0435 |
| M+H | 870.2726 | 9.797 | 0432,0433,0434,0435 |
| M+H | 870.2791 | 6.441 | 0434,0435 |
| M-H | 868.2722 | 7.664 | 0436 |
| M+H | 871.2237 | 6.912 | 0437 |
| M+H | 871.2594 | 6.971 | 0438 |
| M+H | 871.2673 | 6.559 | 0439 |
| M+H | 871.3091 | 7.791 | 0440 |
| M+H | 872.2087 | 8.591 | 0441 |
| M+H | 872.2189 | 5.006 | 0442,0443 |
| M-H | 870.2042 | 8.392 | 0442,0443 |
| M+H | 872.2389 | 8.294 | 0444 |
| M+H | 872.258 | 8.148 | 0445 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 872.2775 | 9.352 | 0446 |
| M+H | 872.3076 | 10.063 | 0447 |
| M+H | 872.3508 | 10.185 | 0448,0449 |
| M+H | 872.3507 | 9.973 | 0448,0449 |
| M+H | 873.2026 | 7.157 | 0450 |
| M+H | 873.2382 | 7.062 | 0451,0452 |
| M+H | 873.2395 | 7.317 | 0451,0452 |
| M+H | 873.2529 | 7.102 | 0453,0454 |
| M+H | 873.2532 | 8.222 | 0453,0454 |
| M+H | 873.273 | 8.178 | 0455 |
| M+H | 873.2835 | 7.017 | 0456 |
| M+H | 874.2387 | 9.38 | 0457,0458,0459,0460 |
| M+H | 874.2401 | 9.554 | 0457,0458,0459,0460 |
| M-H | 872.2227 | 9.265 | 0457,0458,0459,0460 |
| M-H | 872.2241 | 8.309 | 0457,0458,0459,0460 |
| M+H | 874.2492 | 7.254 | 0461 |
| M+H | 874.2952 | 8.578 | 0462,0463 |
| M+H | 874.2981 | 9.303 | 0462,0463 |
| M+H | 874.3293 | 9.442 | 0464,0465 |
| M+H | 874.3309 | 6.755 | 0464,0465 |
| M+H | 875.2344 | 8.29 | 0466,0467,0468 |
| M+H | 875.2334 | 9.706 | 0466,0467,0468 |
| M+H | 875.2369 | 8.268 | 0466,0467,0468 |
| M+H | 875.2492 | 7.769 | 0469,0470 |
| M+H | 875.2533 | 7.249 | 0470 |
| M+H | 875.2672 | 7.945 | 0471 |
| M+H | 875.2883 | 8.517 | 0472,0473 |
| M+H | 875.2874 | 8.121 | 0472,0473 |
| M+H | 876.2343 | 9.255 | 0474 |
| M+H | 876.263 | 9.873 | 0475 |
| M+H | 876.2712 | 7.487 | 0476 |
| M+H | 876.3243 | 9.71 | 0479 |
| M+H | 877.2666 | 7.307 | 0480 |
| M+H | 877.2794 | 8.692 | 0481 |
| M+H | 877.3404 | 8.483 | 0482,0483,0484 |
| M+H | 877.3401 | 8.26 | 0482,0483,0484 |
| M+H | 877.34 | 7.994 | 0482,0483,0484 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 878.2648 | 10.472 | 0485,0486 |
| M+H | 878.2679 | 8.297 | 0485,0486 |
| M+H | 878.2833 | 6.961 | 0487 |
| M+H | 878.3017 | 5.519 | 0488 |
| M-H | 876.3067 | 8.071 | 0489 |
| M+H | 879.2102 | 8.359 | 0491 |
| M+H | 879.2628 | 4.914 | 0492 |
| M+H | 879.2829 | 7.137 | 0493 |
| M+H | 879.3192 | 7.604 | 0494,0495,0496,0497 |
| M+H | 879.3196 | 6.878 | 0494,0495,0496,0497 |
| M+H | 879.3204 | 7.301 | 0494,0495,0496,0497 |
| M+H | 879.3204 | 7.325 | 0494,0495,0496,0497 |
| M+H | 880.2053 | 8.166 | 0498 |
| M+H | 880.2521 | 8.994 | 0499 |
| M+H | 880.2788 | 7.18 | 0500,0501 |
| M+H | 880.2785 | 4.571 | 0500,0501 |
| M+H | 880.2831 | 11.047 | 0500,0501,0502,0503 |
| M+H | 880.281 | 7.996 | 0500,0501,0502,0503 |
| M+H | 880.2998 | 9.793 | 0504 |
| M+H | 880.3192 | 10.046 | 0505,0506 |
| M+H | 880.3166 | 7.747 | 0505,0506 |
| M+H | 881.2733 | 4.634 | 0507 |
| M+H | 881.2932 | 8.585 | 0508 |
| M+H | 881.2984 | 7.062 | 0508,0509,0510 |
| M+H | 881.2961 | 7.681 | 0508,0509,0510 |
| M+H | 881.318 | 7.748 | 0511 |
| M+H | 882.2306 | 8.142 | 0512,0513 |
| M+H | 882.2289 | 8.702 | 0512,0513 |
| M+H | 882.2424 | 7.999 | 0514,0515 |
| M+H | 882.2427 | 8.142 | 0514,0515 |
| M+H | 882.2841 | 7.811 | 0516,0517 |
| M+H | 882.2835 | 7.776 | 0516,0517 |
| M+H | 882.2921 | 5.046 | 0518,0519 |
| M+H | 882.2937 | 4.454 | 0518,0519 |
| M+H | 882.2957 | 7.605 | 0518,0519,0520 |
| M+H | 882.3544 | 6.268 | 0521 |
| M+H | 883.2241 | 7.309 | 0522 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M-H | 881.2221 | 18.656 | 0523 |
| M+H | 883.2582 | 6.945 | 0524,0525 |
| M+H | 883.2578 | 6.813 | 0524,0525 |
| M+H | 883.2783 | 7.696 | 0526 |
| M-H | 881.2752 | 6.927 | 0527,0528,0529 |
| M+H | 883.2922 | 7.637 | 0527,0528,0529 |
| M+H | 883.2912 | 7.77 | 0527,0528,0529 |
| M+H | 884.2189 | 8.032 | 0530 |
| M+H | 884.253 | 7.015 | 0531,0532 |
| M+H | 884.2527 | 5.174 | 0531,0532 |
| M+H | 884.2732 | 8.794 | 0533 |
| M-H | 882.278 | 7.289 | 0534 |
| M+H | 884.3046 | 7.33 | 0535 |
| M+H | 885.2385 | 7.282 | 0536 |
| M+H | 885.2571 | 6.373 | 0537 |
| M-H | 883.272 | 8.402 | 0538,0539 |
| M+H | 885.2894 | 6.937 | 0538,0539 |
| M+H | 886.2203 | 8.98 | 0540 |
| M+H | 886.254 | 8.554 | 0541,0542,0543,0544,0545,0546 |
| M+H | 886.2562 | 8.845 | 0542,0543,0544,0545,0546 |
| M+H | 886.2549 | 9.056 | 0541,0542,0543,0544,0545,0546 |
| M+H | 886.2527 | 7.007 | 0541,0542,0543,0544,0545,0546 |
| M+H | 886.2553 | 8.738 | 0541,0542,0543,0544,0545,0546 |
| M+H | 886.2542 | 9.411 | 0541,0542,0543,0544,0545,0546 |
| M+H | 886.271 | 7.246 | 0547 |
| M-H | 884.2695 | 7.795 | 0548 |
| M+H | 886.2932 | 11.283 | 0549 |
| M+H | 886.3297 | 9.54 | 0550 |
| M+H | 886.3658 | 10.36 | 0551 |
| M+H | 887.2494 | 8.899 | 0552,0553 |
| M+H | 887.2495 | 7.907 | 0552,0553 |
| M+H | 887.269 | 7.391 | 0554,0555 |
| M+H | 887.269 | 7.514 | 0554,0555 |
| M+H | 887.2832 | 7.635 | 0556 |
| M+H | 887.3247 | 8.376 | 0557 |
| M+H | 888.1849 | 9.084 | 0558 |
| M-H | 886.1815 | 8.486 | 0559 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 888.2453 | 9.748 | 0560 |
| M+H | 888.2537 | 9.749 | 0561,0562 |
| M+H | 888.2529 | 14.411 | 0561,0562 |
| M+H | 888.2632 | 7.656 | 0563,0564 |
| M+H | 888.2638 | 8.099 | 0563,0564 |
| M+H | 888.3455 | 9.613 | 0565 |
| M+H | 889.18 | 7.443 | 0566 |
| M+H | 889.2139 | 7.055 | 0567 |
| M+H | 889.2491 | 9.116 | 0568 |
| M+H | 889.2787 | 6.536 | 0569 |
| M+H | 889.3033 | 8.498 | 0570 |
| M+H | 890.1982 | 8.728 | 0571 |
| M+H | 890.2481 | 6.33 | 0572 |
| M+H | 890.2686 | 9.444 | 0573 |
| M+H | 890.2741 | 4.884 | 0574 |
| M+H | 890.2803 | 9.975 | 0575 |
| M+H | 890.2934 | 9.403 | 0576 |
| M+H | 890.3212 | 9.004 | 0577 |
| M+H | 890.3354 | 7.605 | 0578 |
| M-H | 889.1967 | 7.682 | 0579 |
| M+H | 891.2276 | 7.524 | 0580 |
| M+H | 891.2449 | 7.809 | 0581 |
| M+H | 891.2834 | 8.345 | 0582,0583 |
| M+H | 891.2883 | 7.769 | 0582,0583 |
| M+H | 891.3563 | 8.288 | 0584,0585 |
| M+H | 891.356 | 8.643 | 0584,0585 |
| M+H | 892.211 | 9.536 | 0586,0587 |
| M+H | 892.2087 | 5.593 | 0586,0587 |
| M+H | 892.23 | 9.48 | 0588,0589,0590 |
| M+H | 892.2281 | 9.336 | 0588,0589,0590 |
| M+H | 892.2282 | 9.373 | 0588,0589,0590 |
| M+H | 892.2782 | 6.732 | 0591 |
| M+H | 892.2842 | 8.677 | 0591,0592 |
| M+H | 892.3004 | 7.288 | 0593 |
| M+H | 892.3404 | 8.436 | 0594 |
| M+H | 893.2057 | 9.643 | 0595 |
| M+H | 893.2447 | 8.28 | 0596,0597 |

(continued)

| [Table 8-11-4] Compound that may be contained in mixture 2-1-m1E04-1 | | | |
|---|---|---|---|
| Ion species | m/z $[M+H]^+$ or $[M-H]^-$ | Retention time of MS peak (min) | No. of assigned compound described with 2-1-m1E04-1-omitted |
| M+H | 893.2444 | 8.025 | 0596,0597 |
| M+H | 893.2981 | 7.529 | 0598 |
| M+H | 893.303 | 7.295 | 0598,0599 |
| M+H | 893.335 | 7.938 | 0600,0601 |
| M+H | 893.3345 | 7.714 | 0600,0601 |
| M+H | 894.2398 | 7.245 | 0602,0603,0604,0605 |
| M+H | 894.2402 | 6.781 | 0602,0603,0604,0605 |
| M-H | 892.2215 | 8.164 | 0602,0603,0604,0605 |
| M+H | 894.2395 | 6.226 | 0602,0603,0604,0605 |
| M-H | 892.2432 | 8.05 | 0606,0607 |
| M+H | 894.2595 | 10.559 | 0606,0607 |
| M+H | 894.2784 | 6.398 | 0608 |
| M+H | 894.2941 | 6.938 | 0609,0610 |
| M+H | 894.2977 | 4.827 | 0609,0610 |
| M+H | 894.3163 | 9.959 | 0611 |
| M+H | 894.3343 | 10.197 | 0612 |
| M+H | 895.2345 | 7.34 | 0613 |
| M+H | 895.2388 | 8.457 | 0613,0614 |
| M+H | 895.2776 | 6.286 | 0615 |
| M+H | 895.3139 | 7.447 | 0616,0617 |
| M-H | 893.2969 | 7.471 | 0616,0617 |
| M+H | 895.3301 | 8.125 | 0618 |
| M+H | 896.2306 | 8.191 | 0619 |
| M-H | 894.2319 | 18.982 | 0620 |
| M+H | 896.2752 | 8.881 | 0621,0622 |
| M+H | 896.2745 | 5.737 | 0621,0622 |
| M-H | 894.2979 | 8.127 | 0623,0624 |
| M+H | 896.3168 | 7.447 | 0623,0624 |
| M+H | 897.2535 | 8.037 | 0625 |
| M+H | 897.27 | 9.28 | 0626,0627,0628 |
| M+H | 897.273 | 6.808 | 0626,0627,0628 |
| M+H | 897.2737 | 7.157 | 0626,0627,0628 |
| M+H | 897.2911 | 7.684 | 0629 |
| M+H | 897.3126 | 8.117 | 0630 |
| M+H | 897.3309 | 6.971 | 0631 |
| M+H | 898.2654 | 8.612 | 0632,0633 |
| M+H | 898.2685 | 4.64 | 0632,0633 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 898.279 | 7.625 | 0634 |
| M+H | 898.2903 | 10.008 | 0635 |
| M+H | 898.3044 | 8.798 | 0636 |
| M+H | 898.3254 | 5.407 | 0637 |
| M+H | 898.3446 | 9.066 | 0638 |
| M+H | 899.2553 | 7.727 | 0639 |
| M+H | 899.2943 | 7.689 | 0640,0641 |
| M+H | 899.2864 | 7.299 | 0640,0641 |
| M+H | 899.3058 | 8.581 | 0642 |
| M+H | 900.1964 | 8.286 | 0643 |
| M+H | 900.2186 | 8.845 | 0644 |
| M+H | 900.233 | 7.214 | 0645 |
| M+H | 900.2445 | 7.326 | 0646 |
| M+H | 900.2693 | 8.731 | 0647,0648 |
| M+H | 900.2702 | 9.401 | 0647,0648 |
| M+H | 900.2886 | 7.501 | 0649,0650 |
| M+H | 900.2928 | 10.005 | 0650 |
| M+H | 900.2988 | 7.391 | 0651 |
| M+H | 900.3101 | 10.033 | 0652 |
| M+H | 900.3451 | 9.881 | 0653 |
| M+H | 901.1913 | 8.107 | 0654 |
| M+H | 901.2344 | 7.665 | 0655,0656 |
| M+H | 901.2339 | 6.367 | 0655,0656 |
| M+H | 901.248 | 6.913 | 0657,0658 |
| M+H | 901.2493 | 7.345 | 0657,0658 |
| M+H | 901.2645 | 9.776 | 0659 |
| M-H | 899.2678 | 7.474 | 0660 |
| M+H | 901.3547 | 7.929 | 0661 |
| M-H | 900.1874 | 19.364 | 0662 |
| M+H | 902.2222 | 4.515 | 0663 |
| M+H | 902.2297 | 5.667 | 0664,0665 |
| M+H | 902.2382 | 7.661 | 0665 |
| M+H | 902.2459 | 8.76 | 0666,0667,0668 |
| M+H | 902.2488 | 9.524 | 0666,0667,0668 |
| M+H | 902.2491 | 9.503 | 0666,0667,0668 |
| M+H | 902.2701 | 10.07 | 0669,0670 |
| M+H | 902.2687 | 8.085 | 0669,0670 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M-H | 900.2739 | 8.299 | 0671 |
| M+H | 903.2442 | 9.002 | 0672 |
| M+H | 903.2634 | 7.214 | 0673 |
| M+H | 903.2833 | 10.572 | 0674 |
| M+H | 903.294 | 6.884 | 0675 |
| M+H | 904.228 | 8.875 | 0676 |
| M+H | 904.2395 | 9.854 | 0677 |
| M+H | 904.245 | 9.199 | 0677,0678 |
| M+H | 904.2492 | 8.109 | 0678,0679,0680 |
| M+H | 904.2474 | 8.519 | 0678,0679,0680 |
| M+H | 904.3216 | 9.617 | 0681 |
| M+H | 905.2286 | 8.008 | 0682 |
| M+H | 905.2629 | 8.443 | 0683,0684,0685,0686 |
| M+H | 905.2616 | 7.661 | 0683,0684,0685,0686 |
| M+H | 905.2602 | 7.898 | 0683,0684,0685,0686 |
| M+H | 905.2599 | 8.542 | 0683,0684,0685,0686 |
| M+H | 905.299 | 8.603 | 0687 |
| M+H | 905.3349 | 8.308 | 0688 |
| M+H | 905.3722 | 8.673 | 0689 |
| M+H | 906.1757 | 9.25 | 0690 |
| M+H | 906.254 | 8.344 | 0691,0692,0693 |
| M+H | 906.2549 | 7.48 | 0691,0692,0693 |
| M+H | 906.2545 | 6.246 | 0691,0692,0693 |
| M+H | 906.2737 | 10.071 | 0694,0695 |
| M+H | 906.274 | 9.207 | 0694,0695 |
| M+H | 906.3309 | 6.758 | 0696 |
| M+H | 906.3552 | 8.799 | 0697 |
| M+H | 907.1909 | 8.021 | 0698 |
| M-H | 905.1878 | 7.556 | 0699 |
| M+H | 907.2389 | 7.952 | 0700 |
| M+H | 907.2507 | 8.616 | 0701 |
| M+H | 907.2654 | 8.928 | 0702,0703,0704 |
| M+H | 907.2653 | 7.731 | 0702,0703,0704 |
| M+H | 907.2704 | 8.416 | 0704 |
| M+H | 907.3491 | 6.453 | 0705 |
| M+H | 908.19 | 8.936 | 0706,0707 |
| M+H | 908.1859 | 5.738 | 0706,0707 |

(continued)

| [Table 8-11-4] Compound that may be contained in mixture 2-1-m1E04-1 | | | |
|---|---|---|---|
| Ion species | m/z [M+H]$^+$ or [M-H]$^-$ | Retention time of MS peak (min) | No. of assigned compound described with 2-1-m1E04-1-omitted |
| M+H | 908.2053 | 9.636 | 0708 |
| M+H | 908.2591 | 9.65 | 0709,0710,0711 |
| M+H | 908.2564 | 7.366 | 0709,0710,0711 |
| M+H | 908.255 | 8.12 | 0709,0710,0711 |
| M+H | 908.3096 | 7.32 | 0712 |
| M-H | 906.3212 | 8.322 | 0713 |
| M+H | 908.3503 | 10.387 | 0714 |
| M+H | 909.2042 | 7.847 | 0715,0716 |
| M+H | 909.2014 | 9.749 | 0715,0716 |
| M+H | 909.2501 | 6.472 | 0717,0718 |
| M+H | 909.2501 | 6.527 | 0717,0718 |
| M-H | 907.2556 | 8.461 | 0719 |
| M+H | 909.2855 | 8.953 | 0720 |
| M+H | 909.2981 | 7.538 | 0721 |
| M-H | 907.3141 | 8.108 | 0722 |
| M+H | 910.2204 | 9.503 | 0723,0724 |
| M+H | 910.2197 | 9.694 | 0723,0724 |
| M+H | 910.2623 | 8.52 | 0725 |
| M+H | 910.289 | 6.155 | 0726 |
| M+H | 911.2163 | 8.685 | 0727 |
| M+H | 911.2354 | 8.358 | 0728,0729,0730 |
| M+H | 911.2339 | 8.534 | 0728,0729,0730 |
| M+H | 911.2364 | 8.153 | 0728,0729,0730 |
| M-H | 909.2719 | 7.552 | 0731,0732 |
| M+H | 911.2909 | 7.634 | 0731,0732 |
| M-H | 909.2935 | 8.36 | 0733 |
| M+H | 911.346 | 7.285 | 0734 |
| M+H | 912.2149 | 7.487 | 0735,0736 |
| M+H | 912.2113 | 8.543 | 0735,0736 |
| M-H | 910.2271 | 18.749 | 0737 |
| M+H | 912.307 | 10.157 | 0738,0739 |
| M+H | 912.3118 | 6.698 | 0738,0739 |
| M+H | 913.2673 | 8.414 | 0740,0741 |
| M+H | 913.2652 | 9.317 | 0740,0741 |
| M+H | 913.2861 | 7.773 | 0742 |
| M+H | 913.3252 | 6.144 | 0743 |
| M+H | 914.2491 | 8.613 | 0744 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 914.2716 | 6.298 | 0745 |
| M+H | 914.2854 | 10.092 | 0746,0747,0748 |
| M+H | 914.2859 | 9.74 | 0746,0747,0748 |
| M+H | 914.2855 | 10.036 | 0746,0747,0748 |
| M-H | 912.2877 | 8.358 | 0749 |
| M+H | 914.3618 | 10.229 | 0750 |
| M-H | 913.2304 | 7.345 | 0751,0752 |
| M+H | 915.2499 | 7.995 | 0751,0752 |
| M+H | 915.3204 | 7.075 | 0753 |
| M+H | 916.2183 | 9.969 | 0754 |
| M+H | 916.2597 | 7.118 | 0755 |
| M+H | 916.2651 | 8.843 | 0755,0756,0757 |
| M+H | 916.2653 | 8.414 | 0755,0756,0757 |
| M+H | 916.3413 | 9.549 | 0758 |
| M+H | 917.2604 | 8.999 | 0759 |
| M+H | 917.2764 | 5.904 | 0760 |
| M-H | 915.2634 | 9.821 | 0760,0761 |
| M+H | 917.2959 | 8.736 | 0762 |
| M+H | 917.3085 | 6.263 | 0763 |
| M+H | 917.3503 | 7.976 | 0764 |
| M+H | 918.1969 | 9.165 | 0765 |
| M+H | 918.2108 | 9.003 | 0766 |
| M+H | 918.2615 | 9.059 | 0767 |
| M-H | 917.2069 | 7.823 | 0768 |
| M+H | 919.2411 | 7.952 | 0769,0770 |
| M+H | 919.2442 | 8.435 | 0769,0770 |
| M+H | 919.2605 | 6.764 | 0771 |
| M+H | 919.2763 | 8.178 | 0772,0773 |
| M+H | 919.2724 | 6.898 | 0772,0773 |
| M+H | 919.2886 | 6.078 | 0774 |
| M+H | 919.3145 | 8.949 | 0775 |
| M+H | 919.3501 | 8.567 | 0776 |
| M-H | 918.1952 | 8.98 | 0777 |
| M+H | 920.2203 | 7.243 | 0778 |
| M+H | 920.2678 | 7.946 | 0779 |
| M+H | 920.288 | 8.854 | 0780,0781 |
| M+H | 920.2946 | 7.394 | 0781 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 921.2071 | 8.365 | 0782 |
| M+H | 921.2235 | 7.185 | 0783 |
| M+H | 921.2555 | 8.61 | 0784,0785 |
| M+H | 921.2547 | 8.446 | 0784,0785 |
| M+H | 921.2764 | 8.851 | 0786,0787 |
| M+H | 921.2759 | 8.69 | 0786,0787 |
| M-H | 919.2752 | 7.654 | 0788 |
| M+H | 922.2232 | 9.378 | 0789 |
| M-H | 920.2172 | 7.318 | 0790,0791 |
| M+H | 922.2345 | 9.317 | 0790,0791 |
| M+H | 922.2486 | 7.564 | 0792 |
| M+H | 922.2706 | 8.391 | 0793,0794 |
| M+H | 922.2696 | 9.307 | 0793,0794 |
| M+H | 922.2954 | 10.04 | 0795 |
| M+H | 922.3102 | 9.829 | 0796 |
| M+H | 923.2287 | 9.392 | 0797 |
| M+H | 923.2344 | 7.303 | 0797,0798 |
| M+H | 923.2453 | 8.723 | 0799 |
| M+H | 923.2499 | 7.878 | 0799,0800 |
| M+H | 923.2545 | 8.224 | 0800,0801,0802 |
| M+H | 923.2529 | 7.583 | 0800,0801,0802 |
| M-H | 921.309 | 8.426 | 0803 |
| M+H | 924.1662 | 9.525 | 0804 |
| M+H | 924.2549 | 10.141 | 0805,0806 |
| M+H | 924.2553 | 8.8 | 0805,0806 |
| M+H | 925.1816 | 8.198 | 0807 |
| M-H | 923.2136 | 9.407 | 0808 |
| M+H | 925.2638 | 7.591 | 0809 |
| M+H | 925.2799 | 9.001 | 0810 |
| M+H | 925.3614 | 7.648 | 0811 |
| M-H | 924.2292 | 8.232 | 0812,0813 |
| M+H | 926.2426 | 7.311 | 0812,0813 |
| M+H | 926.3215 | 10.358 | 0814 |
| M+H | 927.1935 | 8.3 | 0815 |
| M-H | 925.1937 | 8.763 | 0816 |
| M+H | 927.2692 | 5.906 | 0817 |
| M+H | 927.3407 | 6.501 | 0818 |

(continued)

| [Table 8-11-4] Compound that may be contained in mixture 2-1-m1E04-1 | | | |
|---|---|---|---|
| Ion species | m/z [M+H]$^+$ or [M-H]$^-$ | Retention time of MS peak (min) | No. of assigned compound described with 2-1-m1E04-1-omitted |
| M+H | 928.1909 | 7.591 | 0819 |
| M+H | 928.2066 | 8.58 | 0820 |
| M+H | 928.3006 | 10.257 | 0821 |
| M+H | 928.3364 | 4.136 | 0822 |
| M-H | 927.2097 | 7.643 | 0823,0824 |
| M+H | 929.2605 | 8.546 | 0825 |
| M+H | 929.266 | 9.226 | 0826 |
| M+H | 930.2443 | 8.054 | 0827 |
| M+H | 930.2799 | 9.38 | 0828,0829 |
| M+H | 930.2825 | 9.687 | 0828,0829 |
| M-H | 929.2271 | 7.321 | 0830,0831 |
| M+H | 931.249 | 7.526 | 0831 |
| M+H | 931.3153 | 6.342 | 0832 |
| M+H | 932.2393 | 8.662 | 0833 |
| M+H | 932.2441 | 9.506 | 0833,0834 |
| M-H | 931.2382 | 7.769 | 0835 |
| M+H | 933.2907 | 8.73 | 0836,0837,0838 |
| M+H | 933.2909 | 8.783 | 0836,0837,0838 |
| M+H | 933.2937 | 10.314 | 0836,0837,0838 |
| M+H | 933.3104 | 7.472 | 0839 |
| M+H | 933.3663 | 8.905 | 0840 |
| M+H | 934.2501 | 9.055 | 0841 |
| M+H | 934.2564 | 7.948 | 0842 |
| M+H | 934.3073 | 8.615 | 0843 |
| M+H | 935.2219 | 8.85 | 0844 |
| M+H | 935.2691 | 7.646 | 0845,0846 |
| M+H | 935.2706 | 7.856 | 0845,0846 |
| M+H | 935.3453 | 7.901 | 0847 |
| M+H | 936.1995 | 9.135 | 0848 |
| M+H | 936.2439 | 8.264 | 0849 |
| M+H | 936.2697 | 9.76 | 0850,0851 |
| M+H | 936.2697 | 9.796 | 0850,0851 |
| M+H | 936.2862 | 8.729 | 0852 |
| M+H | 936.345 | 10.245 | 0853 |
| M+H | 937.2012 | 7.467 | 0854 |
| M+H | 937.215 | 8.254 | 0855 |
| M+H | 937.2348 | 7.77 | 0856 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 937.2452 | 7.167 | 0857 |
| M+H | 937.2706 | 7.96 | 0858 |
| M+H | 938.2001 | 9.44 | 0859,0860 |
| M-H | 936.1844 | 9.956 | 0859,0860 |
| M+H | 938.262 | 8.798 | 0861,0862,0863 |
| M+H | 938.266 | 7.438 | 0861,0862,0863 |
| M+H | 938.2694 | 8.609 | 0862,0863 |
| M+H | 939.1958 | 8.085 | 0864 |
| M+H | 939.2095 | 7.847 | 0865 |
| M+H | 939.2303 | 7.304 | 0866 |
| M+H | 939.2678 | 7.47 | 0867 |
| M+H | 940.196 | 9.412 | 0868 |
| M+H | 940.225 | 7.404 | 0869 |
| M+H | 940.2655 | 10.002 | 0870 |
| M-H | 939.2227 | 8.417 | 0871 |
| M+H | 941.3163 | 8.798 | 0872 |
| M+H | 942.2263 | 7.4 | 0873,0874 |
| M+H | 942.227 | 9.863 | 0873,0874 |
| M+H | 942.3169 | 10.433 | 0875 |
| M+H | 943.1707 | 8.697 | 0876 |
| M+H | 944.2365 | 8.616 | 0877 |
| M+H | 945.2552 | 8.63 | 0878 |
| M+H | 945.3296 | 8.962 | 0879 |
| M+H | 946.2455 | 7.215 | 0880 |
| M+H | 946.2722 | 8.97 | 0881 |
| M+H | 947.3077 | 8.938 | 0883 |
| M+H | 948.1805 | 8.155 | 0884 |
| M+H | 948.2341 | 8.784 | 0885 |
| M+H | 948.2496 | 8.735 | 0886 |
| M+H | 948.2655 | 7.555 | 0887 |
| M+H | 949.2493 | 7.84 | 0888 |
| M+H | 949.2838 | 8.048 | 0889,0890 |
| M+H | 949.2856 | 7.946 | 0889,0890 |
| M+H | 950.212 | 8.63 | 0891,0892,0893 |
| M+H | 950.2154 | 9.537 | 0891,0892,0893 |
| M+H | 950.2158 | 9.501 | 0891,0892,0893 |
| M+H | 950.2493 | 7.847 | 0894 |

(continued)

| [Table 8-11-4] Compound that may be contained in mixture 2-1-m1E04-1 | | | |
|---|---|---|---|
| Ion species | m/z [M+H]+ or [M-H]- | Retention time of MS peak (min) | No. of assigned compound described with 2-1-m1E04-1-omitted |
| M+H | 950.2551 | 9.414 | 0895 |
| M+H | 951.2475 | 7.765 | 0896 |
| M-H | 950.2035 | 19.889 | 0897 |
| M+H | 953.2114 | 9.742 | 0898 |
| M+H | 953.2606 | 8.361 | 0899 |
| M+H | 954.2068 | 8.07 | 0900 |
| M+H | 954.2293 | 9.168 | 0901 |
| M+H | 954.2414 | 9.696 | 0902 |
| M+H | 954.2552 | 8.938 | 0903 |
| M+H | 954.2966 | 7.473 | 0904 |
| M+H | 954.3168 | 10.184 | 0905 |
| M-H | 953.1891 | 8.301 | 0906 |
| M+H | 955.2488 | 7.076 | 0907 |
| M+H | 956.1735 | 10.042 | 0908 |
| M+H | 956.1899 | 9.54 | 0909,0910 |
| M+H | 956.1915 | 8.731 | 0909,0910 |
| M+H | 956.2399 | 6.155 | 0911 |
| M+H | 956.2592 | 10.1 | 0912 |
| M+H | 957.2084 | 8.391 | 0913 |
| M+H | 957.2371 | 9.744 | 0914 |
| M+H | 957.2661 | 7.609 | 0915 |
| M+H | 958.2017 | 8.189 | 0916,0917 |
| M+H | 958.2018 | 6.251 | 0916,0917 |
| M+H | 958.221 | 10.185 | 0918,0919 |
| M+H | 958.2207 | 9.954 | 0918,0919 |
| M-H | 957.1834 | 8.741 | 0920 |
| M+H | 959.2695 | 9.133 | 0921 |
| M+H | 961.2318 | 9.013 | 0922,0923 |
| M+H | 961.2315 | 8.932 | 0922,0923 |
| M-H | 959.3051 | 8.961 | 0924 |
| M+H | 962.2265 | 8.373 | 0925 |
| M+H | 962.306 | 8.915 | 0926,0927 |
| M+H | 962.3027 | 8.795 | 0926,0927 |
| M+H | 964.1577 | 8.562 | 0928 |
| M+H | 964.2601 | 7.633 | 0929 |
| M+H | 964.2758 | 10.12 | 0930 |
| M+H | 965.2224 | 8.517 | 0931 |

(continued)

| [Table 8-11-4] Compound that may be contained in mixture 2-1-m1E04-1 | | | |
|---|---|---|---|
| Ion species | m/z $[M+H]^+$ or $[M-H]^-$ | Retention time of MS peak (min) | No. of assigned compound described with 2-1-m1E04-1-omitted |
| M+H | 965.2787 | 8.138 | 0932 |
| M-H | 963.2996 | 7.844 | 0933 |
| M+H | 966.211 | 9.653 | 0934 |
| M+H | 966.2325 | 10.308 | 0935 |
| M+H | 966.247 | 9.039 | 0936 |
| M+H | 967.2272 | 10.201 | 0937 |
| M+H | 967.2403 | 7.839 | 0938 |
| M-H | 966.1993 | 19.693 | 0939 |
| M+H | 969.2218 | 8.628 | 0940 |
| M+H | 969.2607 | 8.282 | 0941 |
| M+H | 970.236 | 9.795 | 0942 |
| M+H | 970.3117 | 10.278 | 0943 |
| M+H | 971.2021 | 9.9 | 0944 |
| M-H | 969.2041 | 8.637 | 0945 |
| M+H | 972.167 | 10.174 | 0946 |
| M+H | 972.2168 | 5.793 | 0947,0948 |
| M+H | 972.2197 | 9.375 | 0947,0948 |
| M+H | 973.2124 | 6.169 | 0949 |
| M+H | 973.2312 | 9.842 | 0950 |
| M+H | 973.2352 | 8.117 | 0950,0951 |
| M+H | 973.2465 | 8.723 | 0952 |
| M+H | 973.2617 | 7.674 | 0953 |
| M+H | 973.3217 | 9.106 | 0954 |
| M+H | 974.1811 | 9.844 | 0955 |
| M+H | 975.1775 | 9.158 | 0956 |
| M+H | 975.1962 | 8.801 | 0957,0958 |
| M+H | 975.1963 | 8.45 | 0957,0958 |
| M+H | 975.265 | 9.193 | 0959 |
| M+H | 976.2423 | 8.903 | 0960 |
| M+H | 977.2266 | 9.072 | 0961,0962 |
| M+H | 977.2259 | 8.339 | 0961,0962 |
| M+H | 981.2427 | 10.683 | 0963 |
| M+H | 981.3112 | 7.926 | 0964 |
| M+H | 983.2218 | 9.814 | 0965 |
| M+H | 983.2761 | 8.659 | 0966 |
| M+H | 985.2152 | 8.738 | 0967 |
| M+H | 985.2375 | 9.062 | 0968 |

(continued)

| [Table 8-11-4] Compound that may be contained in mixture 2-1-m1E04-1 | | | |
|---|---|---|---|
| Ion species | m/z $[M+H]^+$ or $[M-H]^-$ | Retention time of MS peak (min) | No. of assigned compound described with 2-1-m1E04-1-omitted |
| M-H | 983.237 | 7.622 | 0969 |
| M-H | 984.2153 | 8.838 | 0970 |
| M+H | 986.2545 | 9.798 | 0971 |
| M+H | 987.2168 | 7.792 | 0972 |
| M+H | 988.2164 | 10.316 | 0973 |
| M+H | 989.1932 | 10.142 | 0974 |
| M+H | 989.2415 | 8.779 | 0975 |
| M+H | 989.3171 | 9.154 | 0976 |
| M+H | 990.2069 | 8.636 | 0977 |
| M+H | 991.1731 | 9.245 | 0978 |
| M+H | 991.2256 | 8.514 | 0979 |
| M-H | 990.2199 | 8.983 | 0980 |
| M+H | 993.1867 | 8.891 | 0981 |
| M-H | 998.2305 | 9.305 | 0982 |
| M+H | 1002.2273 | 7.905 | 0983 |
| M+H | 1003.2262 | 10.693 | 0984 |
| M+H | 1004.2261 | 9.8 | 0985 |
| M+H | 1006.1872 | 10.358 | 0986 |
| M+H | 1008.1969 | 9.144 | 0987 |
| M+H | 1012.2122 | 8.46 | 0988 |
| M+H | 1014.1728 | 8.842 | 0989 |
| M+H | 1020.2202 | 9.908 | 0990 |
| M+H | 1021.1984 | 10.697 | 0991 |
| M+H | 1022.1826 | 10.485 | 0992 |
| M+H | 1023.231 | 8.892 | 0993 |
| M+H | 1025.1935 | 9.332 | 0994 |
| M+H | 1029.1842 | 9.044 | 0995 |
| M+H | 1037.1936 | 10.817 | 0996 |
| M+H | 1039.2262 | 8.966 | 0997 |
| M+H | 1040.2045 | 9.603 | 0998 |
| M+H | 1041.1884 | 9.409 | 0999 |
| M+H | 1056.1998 | 9.69 | 1000 |

[Table 411]

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 615.296 | 17.252 | 0001 |
| M+H | 616.2912 | 15.766 | 0002,0003 |
| M+H | 616.2921 | 16.748 | 0002,0003 |
| M+H | 617.2864 | 14.961 | 0004,0005 |
| M+H | 617.2868 | 18.132 | 0004,0005 |
| M+H | 618.2826 | 16.819 | 0006 |
| M+H | 622.2482 | 17.91 | 0007 |
| M+H | 623.2421 | 16.535 | 0008 |
| M+H | 629.3113 | 17.654 | 0009 |
| M+H | 630.3073 | 12.138 | 0010 |
| M+H | 631.3014 | 10.986 | 0011 |
| M+H | 633.2696 | 16.026 | 0012 |
| M+H | 633.2874 | 17.173 | 0013 |
| M+H | 634.2657 | 14.144 | 0014 |
| M+H | 634.2827 | 16.799 | 0015 |
| M+H | 634.3017 | 14.716 | 0016 |
| M+H | 635.2768 | 10.625 | 0017 |
| M+H | 635.2972 | 13.588 | 0018,0019 |
| M+H | 635.2966 | 11.635 | 0018,0019 |
| M+H | 636.2633 | 18.39 | 0020 |
| M+H | 636.2922 | 10.398 | 0021,0022 |
| M+H | 636.2939 | 15.822 | 0021,0022 |
| M+H | 637.2877 | 13.021 | 0023 |
| M+H | 640.2389 | 17.848 | 0024 |
| M+H | 641.2543 | 15.133 | 0025 |
| M+H | 642.2482 | 12.183 | 0026 |
| M+H | 643.2916 | 15.79 | 0027 |
| M+H | 643.3284 | 17.979 | 0028 |
| M+H | 644.2869 | 14.099 | 0029,0030 |
| M+H | 644.2863 | 10.186 | 0029,0030 |
| M+H | 644.3225 | 12.769 | 0031 |
| M+H | 645.2822 | 14.271 | 0032 |
| M+H | 645.3079 | 17.164 | 0033 |
| M+H | 645.3181 | 18.758 | 0034 |
| M+H | 646.3018 | 16.677 | 0035 |
| M+H | 647.2863 | 16.484 | 0036 |
| M+H | 647.298 | 17.961 | 0037 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 648.3182 | 15.069 | 0038 |
| M+H | 649.247 | 16.224 | 0039 |
| M+H | 649.3133 | 14.027 | 0040 |
| M+H | 650.2432 | 14.67 | 0041 |
| M+H | 650.2805 | 17.141 | 0042 |
| M+H | 650.3084 | 14.535 | 0043 |
| M+H | 651.2604 | 15.993 | 0044 |
| M+H | 651.2754 | 13.334 | 0045 |
| M+H | 652.2584 | 17.776 | 0046 |
| M+H | 652.2725 | 17.059 | 0047,0048 |
| M-H | 650.2586 | 13.796 | 0047,0048 |
| M+H | 652.2942 | 11.17 | 0049 |
| M+H | 653.2672 | 18.198 | 0050,0051 |
| M+H | 653.2714 | 10.6 | 0050,0051 |
| M+H | 653.287 | 12.907 | 0052 |
| M+H | 654.2846 | 15.82 | 0053 |
| M+H | 655.2689 | 15.546 | 0054 |
| M+H | 657.3065 | 16.333 | 0055 |
| M+H | 658.2298 | 18.013 | 0056 |
| M+H | 658.3021 | 15.741 | 0057,0058 |
| M+H | 658.3018 | 10.667 | 0057,0058 |
| M+H | 659.2446 | 15.226 | 0059 |
| M+H | 659.2972 | 14.033 | 0060 |
| M+H | 661.2812 | 15.924 | 0061,0062 |
| M-H | 659.2634 | 15.849 | 0061,0062 |
| M+H | 661.3017 | 16.974 | 0063 |
| M+H | 662.2762 | 10.328 | 0064,0065 |
| M+H | 662.277 | 16.194 | 0064,0065 |
| M+H | 662.2968 | 13.437 | 0066 |
| M+H | 662.3338 | 15.595 | 0067 |
| M+H | 663.2626 | 16.704 | 0068 |
| M+H | 663.2819 | 15.859 | 0069 |
| M+H | 663.2916 | 10.306 | 0070,0071 |
| M+H | 663.2923 | 13.796 | 0070,0071 |
| M+H | 663.3294 | 14.603 | 0072 |
| M+H | 664.2873 | 10.637 | 0073 |
| M+H | 664.312 | 14.447 | 0074 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 664.3239 | 12.308 | 0075 |
| M+H | 665.308 | 13.566 | 0076,0077 |
| M+H | 665.311 | 17.998 | 0076,0077 |
| M+H | 666.2917 | 14.217 | 0078 |
| M+H | 666.302 | 15.686 | 0079 |
| M+H | 666.3076 | 17.731 | 0079,0080 |
| M+H | 667.2388 | 14.082 | 0081 |
| M+H | 667.3013 | 16.569 | 0082,0083 |
| M+H | 667.3013 | 16.594 | 0082,0083 |
| M+H | 668.253 | 13.582 | 0084 |
| M+H | 668.297 | 14.635 | 0085 |
| M+H | 669.2384 | 13.926 | 0086 |
| M+H | 669.2485 | 14.546 | 0087,0088 |
| M+H | 669.2487 | 10.568 | 0087,0088 |
| M+H | 669.2851 | 15.997 | 0089 |
| M+H | 670.2323 | 14.607 | 0090 |
| M+H | 670.2656 | 13.856 | 0091 |
| M+H | 670.2842 | 15.407 | 0092 |
| M+H | 671.2639 | 15.095 | 0093,0094 |
| M+H | 671.2684 | 17.988 | 0093,0094 |
| M+H | 671.2786 | 14.526 | 0095 |
| M+H | 671.291 | 15.79 | 0096 |
| M+H | 671.3243 | 16.814 | 0097 |
| M+H | 672.2627 | 12.732 | 0098,0099 |
| M+H | 672.2628 | 16.856 | 0098,0099 |
| M+H | 672.2729 | 16.489 | 0100 |
| M+H | 672.2849 | 14.072 | 0101 |
| M+H | 672.3176 | 11.31 | 0102,0103 |
| M+H | 672.3175 | 13.583 | 0102,0103 |
| M+H | 673.3029 | 15.751 | 0104 |
| M+H | 674.2962 | 15.999 | 0105 |
| M+H | 675.2965 | 14.262 | 0106 |
| M+H | 675.3538 | 16.332 | 0107 |
| M+H | 676.294 | 15.826 | 0108 |
| M-H | 674.2946 | 13.847 | 0109 |
| M+H | 676.3491 | 14.582 | 0110 |
| M+H | 677.2358 | 15.817 | 0111 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 677.2772 | 16.134 | 0112 |
| M+H | 677.3075 | 14.248 | 0113,0114 |
| M+H | 677.3075 | 13.062 | 0113,0114 |
| M+H | 678.3022 | 9.947 | 0115 |
| M+H | 679.2574 | 15.081 | 0116,0117,0118 |
| M+H | 679.2573 | 15.165 | 0116,0117,0118 |
| M+H | 679.2596 | 16.594 | 0116,0117,0118 |
| M+H | 679.2701 | 16.073 | 0119,0120 |
| M+H | 679.2731 | 2.379 | 0119,0120 |
| M+H | 680.253 | 14.95 | 0121,0122,0123 |
| M+H | 680.253 | 15.078 | 0121,0122,0123 |
| M+H | 680.253 | 13.453 | 0121,0122,0123 |
| M+H | 680.2675 | 16.368 | 0124 |
| M+H | 680.2869 | 13.492 | 0125,0126 |
| M-H | 678.2699 | 13.449 | 0125,0126 |
| M+H | 680.3065 | 14.758 | 0127 |
| M+H | 681.2487 | 14.493 | 0128,0129 |
| M+H | 681.2481 | 13.177 | 0128,0129 |
| M+H | 681.2833 | 13.846 | 0130,0131 |
| M+H | 681.2822 | 13.901 | 0130,0131 |
| M+H | 681.3201 | 14.149 | 0132 |
| M+H | 682.2435 | 15.823 | 0133 |
| M-H | 680.251 | 13.985 | 0134 |
| M+H | 682.2901 | 11.29 | 0135 |
| M+H | 683.2554 | 14.695 | 0136 |
| M+H | 683.2838 | 17.684 | 0137,0138,0139 |
| M+H | 683.2836 | 17.16 | 0137,0138,0139 |
| M+H | 683.2847 | 16.868 | 0137,0138,0139 |
| M+H | 684.2792 | 15.811 | 0140,0141 |
| M+H | 684.2795 | 15.767 | 0140,0141 |
| M+H | 685.2129 | 16.678 | 0142 |
| M+H | 685.2271 | 16.444 | 0143 |
| M+H | 685.2738 | 8.939 | 0144 |
| M+H | 685.2839 | 15.221 | 0145 |
| M+H | 685.2918 | 16.595 | 0146 |
| M+H | 685.3038 | 9.451 | 0147 |
| M+H | 686.2109 | 17.173 | 0148,0149 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 686.2109 | 17.107 | 0148,0149 |
| M+H | 686.2434 | 13.604 | 0150 |
| M-H | 684.2905 | 13.503 | 0151 |
| M+H | 686.3343 | 16.646 | 0152 |
| M+H | 687.2043 | 15.619 | 0153 |
| M+H | 687.2293 | 16.187 | 0154 |
| M+H | 687.3029 | 10.301 | 0155 |
| M+H | 688.2431 | 14.248 | 0156 |
| M+H | 688.2569 | 14.352 | 0157 |
| M+H | 688.312 | 15.403 | 0158 |
| M+H | 689.2379 | 11.167 | 0159 |
| M+H | 689.2601 | 14.356 | 0160 |
| M+H | 689.2813 | 15.715 | 0161 |
| M+H | 689.3134 | 17.27 | 0162 |
| M+H | 689.3712 | 16.897 | 0163 |
| M+H | 690.2344 | 18.258 | 0164 |
| M+H | 690.2734 | 15.547 | 0165 |
| M-H | 688.2785 | 13.425 | 0166 |
| M+H | 690.3284 | 14.34 | 0167 |
| M+H | 691.2687 | 12.811 | 0168,0169 |
| M+H | 691.2681 | 15.483 | 0168,0169 |
| M+H | 691.2904 | 10.453 | 0170,0171,0172 |
| M-H | 689.2794 | 13.125 | 0170,0171,0172 |
| M+H | 691.2948 | 17.722 | 0170,0171,0172 |
| M+H | 691.3237 | 13.408 | 0173,0174 |
| M+H | 691.3234 | 12.201 | 0173,0174 |
| M+H | 692.2646 | 14.927 | 0175 |
| M+H | 692.2908 | 16.486 | 0176 |
| M+H | 692.3085 | 13.956 | 0177 |
| M+H | 693.26 | 16.515 | 0178 |
| M+H | 693.2743 | 17.054 | 0179,0180,0181 |
| M+H | 693.2733 | 15.497 | 0179,0180,0181 |
| M+H | 693.2743 | 17.102 | 0179,0180,0181 |
| M+H | 693.304 | 13.798 | 0182,0183 |
| M+H | 693.3059 | 16.228 | 0182,0183 |
| M+H | 693.3435 | 16.356 | 0184 |
| M+H | 694.2689 | 13.8 | 0185,0186,0187 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 694.27 | 16.75 | 0185,0186,0187 |
| M+H | 694.2706 | 15.463 | 0185,0186,0187 |
| M+H | 694.2823 | 15.936 | 0188 |
| M+H | 694.3036 | 13.571 | 0189,0190 |
| M+H | 694.3031 | 17.047 | 0189,0190 |
| M+H | 694.3589 | 13.823 | 0191 |
| M+H | 695.266 | 13.996 | 0192,0193 |
| M+H | 695.263 | 17.693 | 0192,0193 |
| M+H | 695.2985 | 13.067 | 0194 |
| M+H | 695.3333 | 17.611 | 0195 |
| M-H | 693.3377 | 10.705 | 0196 |
| M+H | 696.2845 | 14.428 | 0197 |
| M+H | 697.2343 | 13.973 | 0198 |
| M+H | 697.2486 | 15.229 | 0199,0200 |
| M+H | 697.2476 | 16.563 | 0199,0200 |
| M+H | 697.2627 | 17.796 | 0201 |
| M+H | 697.2706 | 15.025 | 0202 |
| M+H | 697.2985 | 17.528 | 0203 |
| M+H | 697.3126 | 16.663 | 0204 |
| M+H | 698.2205 | 16.015 | 0205 |
| M+H | 698.2275 | 13.881 | 0206 |
| M+H | 698.2435 | 13.373 | 0207 |
| M+H | 698.2638 | 12.925 | 0208,0209,0210 |
| M+H | 698.2634 | 14.362 | 0208,0209,0210 |
| M+H | 698.2653 | 14.127 | 0208,0209,0210 |
| M+H | 698.2781 | 14.027 | 0211,0212 |
| M+H | 698.2781 | 14.069 | 0211,0212 |
| M-H | 697.2124 | 17.068 | 0213 |
| M+H | 699.2424 | 14.531 | 0214 |
| M-H | 697.2318 | 16.58 | 0215 |
| M+H | 699.2587 | 12.608 | 0216,0217,0218,0219 |
| M+H | 699.2588 | 11.244 | 0216,0217,0218,0219 |
| M+H | 699.2587 | 9.851 | 0216,0217,0218,0219 |
| M+H | 699.2669 | 13.021 | 0219 |
| M+H | 699.2733 | 14.418 | 0220 |
| M+H | 699.2783 | 17.769 | 0220,0221 |
| M+H | 699.3013 | 10.955 | 0222 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 699.3213 | 16.74 | 0223 |
| M+H | 700.2244 | 17.595 | 0224 |
| M+H | 700.2543 | 10.169 | 0225,0226 |
| M+H | 700.2543 | 9.464 | 0225,0226 |
| M+H | 700.2734 | 13.05 | 0227 |
| M+H | 700.3243 | 13.946 | 0228 |
| M+H | 701.2531 | 10.868 | 0229 |
| M-H | 699.239 | 16.637 | 0229,0230 |
| M+H | 701.2687 | 12.129 | 0231 |
| M+H | 701.2749 | 17.155 | 0232,0233 |
| M+H | 701.2785 | 17.912 | 0232,0233 |
| M+H | 701.2996 | 15.737 | 0234 |
| M+H | 702.2612 | 14.657 | 0235 |
| M+H | 702.2888 | 14.967 | 0236,0237 |
| M+H | 702.2897 | 15.613 | 0236,0237 |
| M-H | 701.1874 | 16.714 | 0238 |
| M+H | 703.2843 | 12.256 | 0239,0240,0241 |
| M+H | 703.2849 | 14.85 | 0239,0240,0241 |
| M+H | 703.2844 | 14.883 | 0239,0240,0241 |
| M+H | 703.386 | 17.262 | 0242 |
| M+H | 704.1995 | 17.107 | 0243 |
| M+H | 704.2211 | 14.523 | 0244 |
| M+H | 704.2346 | 14.264 | 0245 |
| M+H | 704.2786 | 16.543 | 0246 |
| M+H | 704.2886 | 15.991 | 0247 |
| M+H | 704.2981 | 13.701 | 0248 |
| M+H | 704.3115 | 13.846 | 0249 |
| M-H | 703.1988 | 11.622 | 0250,0251,0252 |
| M+H | 705.2171 | 14.625 | 0250,0251,0252 |
| M+H | 705.2155 | 11.715 | 0250,0251,0252 |
| M+H | 705.3087 | 16.694 | 0253,0254 |
| M+H | 705.3087 | 16.784 | 0253,0254 |
| M+H | 705.3397 | 13.554 | 0255 |
| M+H | 705.3663 | 16.33 | 0256 |
| M+H | 706.2104 | 10.956 | 0257 |
| M+H | 706.2299 | 18.39 | 0258,0259 |
| M+H | 706.2354 | 13.995 | 0258,0259 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 706.3045 | 16.547 | 0260 |
| M+H | 707.2516 | 15.395 | 0261,0262 |
| M+H | 707.2516 | 15.294 | 0261,0262 |
| M+H | 707.2741 | 17.594 | 0263 |
| M+H | 707.2911 | 17.449 | 0264,0265,0266,0267 |
| M+H | 707.2881 | 16.055 | 0264,0265,0266,0267 |
| M+H | 707.2897 | 17.413 | 0264,0265,0266,0267 |
| M+H | 707.2879 | 16.206 | 0264,0265,0266,0267 |
| M+H | 707.3182 | 11.515 | 0268 |
| M+H | 708.248 | 13.674 | 0269,0270 |
| M-H | 706.2298 | 13.629 | 0269,0270 |
| M+H | 708.2653 | 18.019 | 0271 |
| M+H | 708.2852 | 13.494 | 0272,0273,0274,0275 |
| M+H | 708.2852 | 13.537 | 0272,0273,0274,0275 |
| M+H | 708.2859 | 17.166 | 0272,0273,0274,0275 |
| M+H | 708.2873 | 17.218 | 0272,0273,0274,0275 |
| M+H | 708.3194 | 13.928 | 0276,0277 |
| M+H | 708.3178 | 16.569 | 0276,0277 |
| M+H | 708.3748 | 14.255 | 0278 |
| M+H | 709.2434 | 13.528 | 0279,0280,0281 |
| M+H | 709.2432 | 13.569 | 0279,0280,0281 |
| M-H | 707.2259 | 14.241 | 0279,0280,0281 |
| M+H | 709.2676 | 15.627 | 0282,0283 |
| M+H | 709.2687 | 14.726 | 0282,0283 |
| M+H | 709.2805 | 17.954 | 0284,0285 |
| M+H | 709.2828 | 13.904 | 0284,0285 |
| M+H | 710.2636 | 12.816 | 0286 |
| M+H | 710.3007 | 15.085 | 0287,0288 |
| M+H | 710.3022 | 14.517 | 0287,0288 |
| M+H | 711.2483 | 16.326 | 0289 |
| M-H | 709.2454 | 15.742 | 0290,0291,0292 |
| M+H | 711.2646 | 15.742 | 0291,0292 |
| M-H | 709.2508 | 12.898 | 0291,0292 |
| M+H | 711.2766 | 16.534 | 0293 |
| M+H | 711.2934 | 12.273 | 0294,0295 |
| M+H | 711.299 | 18.595 | 0294,0295 |
| M+H | 711.3161 | 17.849 | 0296 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 711.3362 | 16.64 | 0297 |
| M+H | 712.2599 | 14.412 | 0298,0299 |
| M+H | 712.2599 | 14.325 | 0298,0299 |
| M+H | 712.2738 | 11.572 | 0300 |
| M+H | 712.2792 | 13.192 | 0301,0302,0303 |
| M+H | 712.2792 | 13.352 | 0301,0302,0303 |
| M+H | 712.2792 | 14.747 | 0301,0302,0303 |
| M+H | 712.3493 | 13.884 | 0304 |
| M+H | 713.2098 | 16.385 | 0305 |
| M+H | 713.2474 | 17.45 | 0306 |
| M+H | 713.2754 | 13.032 | 0307,0308,0309 |
| M+H | 713.2745 | 10.103 | 0307,0308,0309 |
| M+H | 713.2754 | 13.996 | 0307,0308,0309 |
| M+H | 713.2878 | 13.784 | 0310 |
| M+H | 713.2943 | 17.098 | 0310,0311 |
| M+H | 714.2041 | 14.526 | 0312 |
| M+H | 714.2429 | 17.907 | 0313 |
| M+H | 714.2722 | 15.223 | 0314,0315 |
| M-H | 712.2583 | 13.969 | 0314,0315 |
| M+H | 714.341 | 14.507 | 0316 |
| M+H | 715.2215 | 15.885 | 0317,0318 |
| M-H | 713.2116 | 12.964 | 0317,0318 |
| M+H | 715.2381 | 15.275 | 0319,0320 |
| M+H | 715.2381 | 15.497 | 0319,0320 |
| M+H | 716.2211 | 17.039 | 0321 |
| M+H | 716.2343 | 15.35 | 0322,0323 |
| M-H | 714.2205 | 13.541 | 0322,0323 |
| M+H | 716.2538 | 12.974 | 0324,0325 |
| M+H | 716.2537 | 13.237 | 0324,0325 |
| M+H | 716.2684 | 8.983 | 0326 |
| M+H | 716.274 | 15.13 | 0326,0327 |
| M-H | 714.2864 | 15.382 | 0328 |
| M+H | 716.3175 | 13.502 | 0329 |
| M+H | 717.23 | 17.43 | 0330,0331 |
| M+H | 717.233 | 10.061 | 0330,0331,0332 |
| M+H | 717.2378 | 16.858 | 0331,0332 |
| M+H | 717.249 | 13.557 | 0333,0334 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 717.25 | 10.113 | 0333,0334 |
| M+H | 717.3182 | 17.724 | 0335 |
| M+H | 718.2356 | 14.959 | 0336 |
| M+H | 718.248 | 12.04 | 0337 |
| M+H | 718.2521 | 13.501 | 0338 |
| M+H | 718.2837 | 15.7 | 0339 |
| M+H | 718.3066 | 16.343 | 0340 |
| M+H | 718.3261 | 14.398 | 0341 |
| M+H | 719.2317 | 8.899 | 0342 |
| M+H | 719.2517 | 10.127 | 0343 |
| M+H | 719.266 | 14.04 | 0344,0345 |
| M-H | 717.2462 | 14.063 | 0344,0345 |
| M+H | 719.2804 | 14.865 | 0346 |
| M+H | 719.3294 | 13.604 | 0347,0348,0349 |
| M+H | 719.3256 | 18.075 | 0347,0348,0349 |
| M+H | 719.3256 | 18.127 | 0347,0348,0349 |
| M+H | 720.2588 | 14.346 | 0350 |
| M+H | 720.263 | 14.736 | 0350,0351 |
| M+H | 720.2733 | 16.649 | 0352 |
| M+H | 720.2793 | 14.956 | 0352,0353 |
| M+H | 720.284 | 10.845 | 0353,0354 |
| M+H | 720.305 | 13.477 | 0355 |
| M+H | 720.3205 | 11.236 | 0356 |
| M-H | 719.177 | 16.865 | 0357 |
| M-H | 719.2514 | 15.916 | 0358 |
| M+H | 721.2808 | 18.693 | 0359 |
| M+H | 721.3045 | 17.653 | 0360,0361,0362,0363 |
| M+H | 721.3036 | 16.492 | 0360,0361,0362,0363 |
| M+H | 721.3039 | 16.569 | 0360,0361,0362,0363 |
| M+H | 721.3048 | 16.695 | 0360,0361,0362,0363 |
| M+H | 722.1923 | 17.233 | 0364 |
| M+H | 722.2112 | 14.633 | 0365 |
| M+H | 722.2631 | 15.443 | 0366,0367 |
| M+H | 722.2627 | 15.371 | 0366,0367 |
| M+H | 722.2902 | 14.358 | 0368 |
| M+H | 722.3 | 13.524 | 0369 |
| M+H | 722.39 | 14.747 | 0370 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 723.2097 | 13.742 | 0371 |
| M+H | 723.2605 | 15.347 | 0372 |
| M+H | 723.2853 | 15.26 | 0373,0374 |
| M-H | 721.2659 | 15.276 | 0373,0374 |
| M+H | 723.3009 | 17.688 | 0375 |
| M+H | 724.2233 | 14.834 | 0376 |
| M+H | 724.2829 | 13.863 | 0377,0378 |
| M+H | 724.2797 | 13.903 | 0377,0378 |
| M+H | 724.3132 | 14.323 | 0379,0380 |
| M+H | 724.3141 | 14.27 | 0379,0380 |
| M+H | 724.3702 | 13.802 | 0381 |
| M+H | 725.2208 | 14.278 | 0382 |
| M+H | 725.2352 | 16.106 | 0383 |
| M+H | 725.242 | 15.453 | 0384,0385 |
| M+H | 725.2419 | 15.349 | 0384,0385 |
| M+H | 725.2646 | 14.003 | 0386 |
| M+H | 725.2802 | 16.128 | 0387,0388 |
| M-H | 723.2628 | 16.223 | 0387,0388 |
| M+H | 725.3143 | 18.853 | 0389 |
| M+H | 725.3686 | 17.33 | 0390 |
| M+H | 726.2389 | 14.366 | 0391,0392 |
| M+H | 726.238 | 14.411 | 0391,0392 |
| M+H | 726.2588 | 13.165 | 0393,0394 |
| M-H | 724.2419 | 13.209 | 0393,0394 |
| M+H | 726.2758 | 14.652 | 0395 |
| M+H | 726.2877 | 16.453 | 0396 |
| M+H | 726.295 | 13.565 | 0397,0398,0399,0400 |
| M+H | 726.2953 | 15.212 | 0397,0398,0399,0400 |
| M+H | 726.2948 | 13.834 | 0397,0398,0399,0400 |
| M+H | 726.2945 | 13.88 | 0396,0397,0398,0399,0400 |
| M+H | 727.2255 | 16.932 | 0401 |
| M+H | 727.2434 | 15.737 | 0402 |
| M+H | 727.254 | 10.096 | 0403,0404 |
| M-H | 725.2375 | 13.42 | 0403,0404 |
| M+H | 727.2734 | 16.672 | 0405,0406 |
| M+H | 727.2761 | 17.909 | 0405,0406 |
| M+H | 727.2904 | 14.418 | 0407,0408,0409 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 727.2901 | 13.125 | 0407,0408,0409 |
| M+H | 727.2913 | 14.451 | 0407,0408,0409 |
| M+H | 728.2488 | 10.359 | 0410 |
| M+H | 728.2685 | 11.665 | 0411 |
| M+H | 728.2741 | 12.9 | 0412,0413 |
| M+H | 728.2732 | 12.954 | 0411,0412,0413 |
| M+H | 728.2853 | 12.188 | 0414 |
| M+H | 728.2935 | 13.526 | 0415 |
| M+H | 729.2557 | 15.829 | 0416,0417 |
| M+H | 729.2581 | 11.289 | 0416,0417 |
| M+H | 729.27 | 9.768 | 0418,0419,0420,0421 |
| M+H | 729.269 | 13.441 | 0418,0419 |
| M-H | 727.2549 | 16.17 | 0418,0419,0420,0421 |
| M-H | 727.2549 | 16.105 | 0418,0419,0420,0421 |
| M-H | 728.2362 | 13.935 | 0422,0423 |
| M-H | 728.2366 | 13.971 | 0422,0423 |
| M-H | 728.2524 | 13.644 | 0424,0425,0426,0427 |
| M+H | 730.2704 | 13.613 | 0424,0425,0426,0427 |
| M+H | 730.27 | 13.708 | 0424,0425,0426,0427 |
| M+H | 730.2703 | 13.269 | 0424,0425,0426,0427 |
| M-H | 728.2659 | 14.445 | 0428 |
| M+H | 730.3214 | 15.185 | 0429 |
| M+H | 730.3416 | 14.51 | 0430 |
| M+H | 731.2004 | 16.421 | 0431 |
| M+H | 731.2647 | 16.133 | 0432,0433,0434,0435 |
| M+H | 731.2645 | 10.859 | 0432,0433,0434,0435 |
| M+H | 731.2671 | 13.38 | 0432,0433,0434,0435 |
| M+H | 731.2631 | 6.32 | 0432,0433,0434,0435 |
| M+H | 731.2786 | 14.803 | 0436 |
| M+H | 732.2147 | 13.681 | 0437 |
| M+H | 732.2504 | 15.396 | 0438 |
| M+H | 732.2591 | 13.978 | 0439 |
| M+H | 732.2985 | 14.968 | 0440 |
| M+H | 733.1993 | 11.693 | 0441 |
| M-H | 731.1942 | 16.055 | 0442,0443 |
| M+H | 733.2103 | 10.189 | 0442,0443 |
| M+H | 733.2305 | 17.026 | 0444 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 733.2472 | 13.325 | 0445 |
| M+H | 733.2678 | 14.196 | 0446 |
| M+H | 733.3007 | 9.881 | 0447 |
| M+H | 733.3402 | 18.362 | 0448,0449 |
| M+H | 733.3415 | 18.427 | 0448,0449 |
| M+H | 734.1941 | 14.441 | 0450 |
| M+H | 734.2293 | 13.61 | 0451,0452 |
| M-H | 732.2123 | 13.65 | 0451,0452 |
| M-H | 732.229 | 13.33 | 0453,0454 |
| M+H | 734.2454 | 13.271 | 0453,0454 |
| M+H | 734.2632 | 16.112 | 0455 |
| M+H | 734.2748 | 13.716 | 0456 |
| M-H | 733.2118 | 17.524 | 0457,0458,0459,0460 |
| M-H | 733.2185 | 11.388 | 0458,0459,0460 |
| M+H | 735.2296 | 17.475 | 0457,0458,0459,0460 |
| M+H | 735.2313 | 17.142 | 0457,0458,0459,0460 |
| M+H | 735.2408 | 12.623 | 0461 |
| M+H | 735.2849 | 16.408 | 0462 |
| M+H | 735.2908 | 17.223 | 0463 |
| M+H | 735.3204 | 17.604 | 0464,0465 |
| M+H | 735.3204 | 17.678 | 0464,0465 |
| M+H | 736.2244 | 16.201 | 0466,0467,0468 |
| M+H | 736.2243 | 16.143 | 0466,0467,0468 |
| M+H | 736.2257 | 16.347 | 0466,0467,0468 |
| M+H | 736.2346 | 15.355 | 0469 |
| M+H | 736.2397 | 14.557 | 0469,0470 |
| M+H | 736.2576 | 14.797 | 0471 |
| M+H | 736.2782 | 13.662 | 0472,0473 |
| M+H | 736.2786 | 15.997 | 0472,0473 |
| M-H | 735.2092 | 16.392 | 0474 |
| M+H | 737.2553 | 11.128 | 0475 |
| M+H | 737.2644 | 15.365 | 0476 |
| M+H | 737.3003 | 15.795 | 0477,0478 |
| M+H | 737.3003 | 15.733 | 0477,0478 |
| M+H | 737.317 | 17.997 | 0479 |
| M+H | 738.2605 | 15.261 | 0480 |
| M+H | 738.2694 | 15.55 | 0481 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 738.3312 | 10.684 | 0482,0483,0484 |
| M+H | 738.3316 | 15.542 | 0482,0483,0484 |
| M+H | 738.3316 | 15.493 | 0482,0483,0484 |
| M+H | 739.2541 | 18.354 | 0485 |
| M-H | 737.2414 | 16.003 | 0485,0486 |
| M+H | 739.2766 | 16.481 | 0487 |
| M+H | 739.2964 | 16.793 | 0488 |
| M-H | 737.2974 | 15.726 | 0489 |
| M+H | 739.3244 | 12.897 | 0490 |
| M+H | 740.2006 | 15.136 | 0491 |
| M+H | 740.255 | 15.52 | 0492 |
| M-H | 738.2565 | 13.67 | 0493 |
| M+H | 740.3115 | 14.031 | 0494,0495,0496,0497 |
| M+H | 740.3114 | 14.119 | 0494,0495,0496,0497 |
| M+H | 740.3104 | 14.905 | 0494,0495,0496,0497 |
| M+H | 740.3121 | 13.952 | 0494,0495,0496,0497 |
| M+H | 741.1997 | 15.198 | 0498 |
| M+H | 741.2407 | 17.352 | 0499 |
| M+H | 741.2687 | 12.877 | 0500,0501 |
| M+H | 741.2691 | 13.81 | 0500,0501 |
| M+H | 741.2752 | 10.341 | 0502,0503 |
| M-H | 739.2593 | 13.762 | 0500,0501,0502,0503 |
| M+H | 741.2914 | 17.909 | 0504 |
| M+H | 741.3062 | 8.21 | 0505,0506 |
| M-H | 739.296 | 14.325 | 0506 |
| M+H | 742.2646 | 9.904 | 0507 |
| M+H | 742.2827 | 16.586 | 0508 |
| M+H | 742.2901 | 13.341 | 0509,0510 |
| M+H | 742.2897 | 13.387 | 0509,0510 |
| M+H | 742.3076 | 15.245 | 0511 |
| M-H | 741.2016 | 16.465 | 0512,0513 |
| M-H | 741.2018 | 16.27 | 0512,0513 |
| M+H | 743.2332 | 15.48 | 0514,0515 |
| M+H | 743.2333 | 15.726 | 0514,0515 |
| M+H | 743.2734 | 13.744 | 0516,0517 |
| M+H | 743.2791 | 11.274 | 0517 |
| M-H | 741.2699 | 16.506 | 0518,0519,0520 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 743.2833 | 2.364 | 0518,0519 |
| M-H | 741.2756 | 13.351 | 0520 |
| M+H | 743.3398 | 16.915 | 0521 |
| M+H | 744.2153 | 14.886 | 0522 |
| M-H | 742.2119 | 15.598 | 0523 |
| M+H | 744.2487 | 13.119 | 0524,0525 |
| M+H | 744.2485 | 13.314 | 0524,0525 |
| M-H | 742.2514 | 14.502 | 0526 |
| M+H | 744.2858 | 14.177 | 0527,0528,0529 |
| M+H | 744.2864 | 14.365 | 0527,0528,0529 |
| M+H | 744.2839 | 12.853 | 0527,0528,0529 |
| M+H | 745.2106 | 14.68 | 0530 |
| M+H | 745.2439 | 10.327 | 0531,0532 |
| M+H | 745.2437 | 13.486 | 0531,0532 |
| M+H | 745.2651 | 12.033 | 0533 |
| M+H | 745.2791 | 11.648 | 0534 |
| M+H | 745.2951 | 14.045 | 0535 |
| M+H | 746.2319 | 14.179 | 0536 |
| M+H | 746.2509 | 15.034 | 0537 |
| M+H | 746.2808 | 14.467 | 0538,0539 |
| M+H | 746.2808 | 14.509 | 0538,0539 |
| M-H | 745.1961 | 16.589 | 0540 |
| M+H | 747.2445 | 17.065 | 0541,0542,0543,0544,0545,0546 |
| M-H | 745.2282 | 16.785 | 0541,0542,0543,0544,0545,0546 |
| M-H | 745.2266 | 16.912 | 0541,0542,0543,0544,0545 |
| M+H | 747.2438 | 15.942 | 0541,0542,0543,0544,0545,0546 |
| M+H | 747.2444 | 17.158 | 0541,0542,0543,0544,0545,0546 |
| M+H | 747.2452 | 15.897 | 0541,0542,0543,0544,0545,0546 |
| M+H | 747.2633 | 14.097 | 0547 |
| M+H | 747.2749 | 14.908 | 0548 |
| M+H | 747.2811 | 11.48 | 0549 |
| M+H | 747.3246 | 14.547 | 0550 |
| M+H | 747.356 | 16.702 | 0551 |
| M+H | 748.2406 | 15.266 | 0552,0553 |
| M+H | 748.2398 | 15.301 | 0552,0553 |
| M+H | 748.2598 | 13.87 | 0554,0555 |
| M+H | 748.2602 | 13.814 | 0554,0555 |

(continued)

| [Table 8-11-5] Compound that may be contained in mixture 2-1-m1E01-2 | | | |
|---|---|---|---|
| Ion species | m/z [M+H]+ or [M-H]- | Retention time of MS peak (min) | No. of assigned compound described with 2-1-m1E01-2-omitted |
| M-H | 746.2574 | 12.369 | 0556 |
| M+H | 748.3175 | 16.355 | 0557 |
| M+H | 749.1755 | 12.361 | 0558 |
| M+H | 749.1883 | 16.526 | 0559 |
| M-H | 747.2182 | 17.744 | 0560 |
| M+H | 749.246 | 17.843 | 0561,0562 |
| M+H | 749.2443 | 15.249 | 0561,0562 |
| M+H | 749.2548 | 16.03 | 0563,0564 |
| M+H | 749.2548 | 15.977 | 0563,0564 |
| M+H | 749.3365 | 17.956 | 0565 |
| M+H | 750.1691 | 16.944 | 0566 |
| M+H | 750.2044 | 13.814 | 0567 |
| M+H | 750.2422 | 14.751 | 0568 |
| M+H | 750.2674 | 12.696 | 0569 |
| M+H | 750.2961 | 16.475 | 0570 |
| M+H | 751.1926 | 11.832 | 0571 |
| M+H | 751.2359 | 15.481 | 0572 |
| M+H | 751.259 | 17.404 | 0573 |
| M+H | 751.265 | 9.544 | 0574 |
| M+H | 751.27 | 17.535 | 0574,0575 |
| M+H | 751.2838 | 17.33 | 0576 |
| M+H | 751.3167 | 16.568 | 0577 |
| M+H | 751.3265 | 14.552 | 0578 |
| M-H | 750.1895 | 14.195 | 0579 |
| M+H | 752.2208 | 12.527 | 0580 |
| M-H | 750.2169 | 14.616 | 0581 |
| M+H | 752.273 | 15.399 | 0582,0583 |
| M-H | 750.256 | 15.344 | 0582,0583 |
| M+H | 752.344 | 14.742 | 0584,0585 |
| M+H | 752.3467 | 11.172 | 0584,0585 |
| M-H | 751.182 | 17.214 | 0586,0587 |
| M+H | 753.1993 | 10.92 | 0586,0587 |
| M+H | 753.2206 | 17.478 | 0588,0589,0590 |
| M+H | 753.2206 | 17.415 | 0588,0589,0590 |
| M+H | 753.2199 | 17.057 | 0588,0589,0590 |
| M-H | 751.2515 | 12.71 | 0591 |
| M-H | 751.2567 | 16.381 | 0591,0592 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 753.2945 | 13.316 | 0593 |
| M+H | 753.3309 | 14.545 | 0594 |
| M+H | 754.1977 | 17.577 | 0595 |
| M+H | 754.2349 | 15.132 | 0596,0597 |
| M+H | 754.2351 | 14.969 | 0596,0597 |
| M+H | 754.2913 | 14.171 | 0598,0599 |
| M+H | 754.2957 | 14.672 | 0599 |
| M+H | 754.3287 | 13.455 | 0600,0601 |
| M+H | 754.3266 | 15.22 | 0600,0601 |
| M+H | 755.2314 | 12.654 | 0602,0603,0604,0605 |
| M+H | 755.2309 | 13.598 | 0602,0603,0604,0605 |
| M+H | 755.2314 | 12.127 | 0602,0603,0604,0605 |
| M+H | 755.2322 | 14.896 | 0602,0603,0604,0605 |
| M+H | 755.2522 | 15.45 | 0606,0607 |
| M+H | 755.2523 | 15.388 | 0606,0607 |
| M+H | 755.2684 | 13.027 | 0608 |
| M+H | 755.2851 | 13.343 | 0609,0610 |
| M+H | 755.285 | 12.008 | 0609,0610 |
| M+H | 755.3048 | 17.306 | 0611 |
| M+H | 755.3248 | 18.391 | 0612 |
| M+H | 756.2259 | 14.466 | 0613 |
| M+H | 756.23 | 12.207 | 0613,0614 |
| M-H | 754.2531 | 13 | 0615 |
| M+H | 756.3056 | 13.904 | 0616,0617 |
| M+H | 756.3059 | 13.963 | 0616,0617 |
| M+H | 756.3234 | 15.556 | 0618 |
| M+H | 757.2211 | 15.505 | 0619 |
| M+H | 757.2339 | 12.13 | 0620 |
| M+H | 757.2615 | 10.939 | 0621 |
| M+H | 757.2698 | 12.987 | 0621,0622 |
| M+H | 757.3087 | 13.847 | 0623,0624 |
| M+H | 757.3043 | 15.717 | 0623,0624 |
| M-H | 756.2261 | 15.709 | 0625 |
| M+H | 758.2645 | 12.944 | 0626,0627,0628 |
| M+H | 758.2652 | 10.394 | 0626,0627,0628 |
| M+H | 758.264 | 13.566 | 0626,0627,0628 |
| M+H | 758.2837 | 14.505 | 0629 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 758.3015 | 13.195 | 0630 |
| M+H | 758.3207 | 13.326 | 0631 |
| M+H | 759.2565 | 15.596 | 0632,0633 |
| M+H | 759.2595 | 12.967 | 0632,0633 |
| M-H | 757.2495 | 13.649 | 0633,0634 |
| M+H | 759.2817 | 18.104 | 0635 |
| M+H | 759.2959 | 17.264 | 0636 |
| M+H | 759.3163 | 10.478 | 0637 |
| M+H | 759.3368 | 17.068 | 0638 |
| M-H | 758.2283 | 14.724 | 0639 |
| M-H | 758.2686 | 15.66 | 0640,0641 |
| M+H | 760.2794 | 13.733 | 0640,0641 |
| M+H | 760.2946 | 11.583 | 0642 |
| M-H | 759.1688 | 15.118 | 0643 |
| M+H | 761.2116 | 16.536 | 0644 |
| M+H | 761.2231 | 15.691 | 0645 |
| M+H | 761.2346 | 13.503 | 0646 |
| M+H | 761.2627 | 16.265 | 0647,0648 |
| M+H | 761.2596 | 17.152 | 0647,0648 |
| M+H | 761.2775 | 15.145 | 0649,0650 |
| M+H | 761.2779 | 15.097 | 0649,0650 |
| M+H | 761.2911 | 14.124 | 0651 |
| M+H | 761.2991 | 14.574 | 0652 |
| M+H | 761.3366 | 17.91 | 0653 |
| M+H | 762.1838 | 15.26 | 0654 |
| M-H | 760.2085 | 14.222 | 0655,0656 |
| M-H | 760.2089 | 14.282 | 0655,0656 |
| M+H | 762.2387 | 13.196 | 0657,0658 |
| M-H | 760.2214 | 13.868 | 0657,0658 |
| M+H | 762.2565 | 11.893 | 0659 |
| M+H | 762.2741 | 14.576 | 0660 |
| M+H | 762.3461 | 14.8 | 0661 |
| M+H | 763.191 | 12.939 | 0662 |
| M+H | 763.2206 | 11.101 | 0664,0665 |
| M-H | 761.206 | 17.305 | 0664,0665 |
| M-H | 761.2189 | 17.017 | 0666 |
| M+H | 763.2412 | 17.01 | 0667,0668 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 763.2412 | 17.222 | 0667,0668 |
| M+H | 763.2614 | 18.133 | 0669,0670 |
| M+H | 763.2614 | 18.206 | 0669,0670 |
| M+H | 763.2764 | 14.295 | 0671 |
| M+H | 764.2351 | 15.924 | 0672 |
| M+H | 764.2547 | 13.508 | 0673 |
| M+H | 764.2689 | 12.75 | 0674 |
| M+H | 764.2852 | 13.411 | 0675 |
| M-H | 763.2016 | 16.54 | 0676 |
| M+H | 765.2309 | 17.771 | 0677 |
| M-H | 763.2175 | 15.955 | 0677,0678 |
| M+H | 765.2406 | 17.415 | 0678,0679,0680 |
| M+H | 765.2406 | 17.374 | 0678,0679,0680 |
| M+H | 765.3115 | 17.54 | 0681 |
| M-H | 764.2027 | 14.588 | 0682 |
| M+H | 766.2512 | 14.485 | 0683,0684,0685,0686 |
| M+H | 766.2521 | 13.933 | 0683,0684,0685,0686 |
| M+H | 766.2509 | 15.065 | 0683,0684,0685,0686 |
| M+H | 766.2521 | 13.975 | 0683,0684,0685,0686 |
| M+H | 766.2901 | 15.717 | 0687 |
| M+H | 766.327 | 15.191 | 0688 |
| M+H | 766.3651 | 16.296 | 0689 |
| M+H | 767.1699 | 10.958 | 0690 |
| M+H | 767.2462 | 11.813 | 0691,0692,0693 |
| M+H | 767.247 | 14.58 | 0691,0692,0693 |
| M+H | 767.247 | 14.525 | 0691,0692,0693 |
| M+H | 767.2653 | 17.611 | 0694,0695 |
| M+H | 767.2654 | 17.658 | 0694,0695 |
| M+H | 767.3212 | 12.662 | 0696 |
| M+H | 767.3482 | 16.695 | 0697 |
| M+H | 768.1952 | 14.453 | 0699 |
| M+H | 768.2297 | 16.87 | 0700 |
| M+H | 768.2403 | 15.687 | 0701 |
| M+H | 768.2506 | 15.456 | 0702,0703 |
| M+H | 768.2507 | 15.502 | 0702,0703 |
| M+H | 768.2604 | 13.058 | 0704 |
| M+H | 768.3438 | 14.048 | 0705 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 769.1768 | 11.48 | 0706,0707 |
| M-H | 767.1614 | 16.345 | 0706,0707 |
| M-H | 767.1782 | 17.332 | 0708 |
| M-H | 767.2282 | 13.836 | 0709,0710 |
| M-H | 767.2281 | 14.912 | 0709,0710 |
| M+H | 769.2504 | 17.583 | 0709,0710,0711 |
| M+H | 769.3008 | 13.788 | 0712 |
| M-H | 767.3089 | 15.709 | 0713 |
| M+H | 769.3387 | 12.569 | 0714 |
| M-H | 768.1769 | 14.243 | 0715,0716 |
| M-H | 768.1773 | 14.326 | 0715,0716 |
| M-H | 768.2254 | 12.101 | 0717,0718 |
| M+H | 770.2417 | 14.216 | 0717,0718 |
| M+H | 770.2661 | 15.396 | 0719 |
| M+H | 770.2783 | 15.736 | 0720 |
| M+H | 770.2895 | 14.73 | 0721 |
| M+H | 770.3214 | 14.299 | 0722 |
| M+H | 771.2083 | 13.96 | 0723,0724 |
| M+H | 771.2094 | 17.284 | 0723,0724 |
| M+H | 771.2552 | 12.939 | 0725 |
| M+H | 771.2798 | 12.958 | 0726 |
| M+H | 772.2091 | 15.375 | 0727 |
| M+H | 772.2256 | 15.149 | 0728,0729,0730 |
| M-H | 770.2087 | 13.731 | 0728,0729,0730 |
| M-H | 770.2087 | 13.705 | 0728,0729,0730 |
| M+H | 772.2819 | 14.057 | 0731,0732 |
| M+H | 772.2819 | 13.999 | 0731,0732 |
| M-H | 770.2819 | 8.168 | 0733 |
| M+H | 772.3361 | 13.756 | 0734 |
| M+H | 773.2043 | 14.199 | 0735,0736 |
| M+H | 773.2025 | 15.481 | 0735,0736 |
| M-H | 771.2174 | 11.904 | 0737 |
| M+H | 773.2972 | 18.076 | 0738,0739 |
| M+H | 773.2972 | 18.141 | 0738,0739 |
| M+H | 774.2564 | 16.415 | 0740,0741 |
| M+H | 774.2583 | 13.204 | 0740,0741 |
| M+H | 774.2785 | 14.498 | 0742 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 774.31 | 15.191 | 0743 |
| M+H | 775.2399 | 16.119 | 0744 |
| M-H | 773.2478 | 14.325 | 0745 |
| M+H | 775.2767 | 17.47 | 0746,0747,0748 |
| M+H | 775.2767 | 17.409 | 0746,0747,0748 |
| M+H | 775.2764 | 18.202 | 0746,0747,0748 |
| M+H | 775.2934 | 15.586 | 0749 |
| M+H | 775.351 | 18.265 | 0750 |
| M-H | 774.2175 | 15.972 | 0751 |
| M-H | 774.2239 | 14.689 | 0751,0752 |
| M+H | 776.3112 | 13.414 | 0753 |
| M-H | 775.1889 | 18.527 | 0754 |
| M+H | 777.2501 | 13.61 | 0755 |
| M+H | 777.2556 | 15.467 | 0755,0756,0757 |
| M+H | 777.2538 | 16.578 | 0755,0756,0757 |
| M+H | 777.3303 | 17.492 | 0758 |
| M+H | 778.25 | 12.212 | 0759 |
| M+H | 778.2739 | 17.612 | 0760,0761 |
| M+H | 778.2703 | 13.103 | 0760,0761 |
| M+H | 778.2873 | 18.127 | 0762 |
| M+H | 778.3006 | 11.472 | 0763 |
| M+H | 778.3417 | 14.89 | 0764 |
| M+H | 779.2013 | 16.736 | 0766 |
| M-H | 777.2326 | 13.021 | 0767 |
| M-H | 778.1974 | 14.373 | 0768 |
| M+H | 780.2309 | 13.765 | 0769 |
| M-H | 778.218 | 15.077 | 0769,0770 |
| M+H | 780.2514 | 14.04 | 0771 |
| M+H | 780.2659 | 14.745 | 0772,0773 |
| M+H | 780.2659 | 14.799 | 0772,0773 |
| M+H | 780.2802 | 12.967 | 0774 |
| M+H | 780.3071 | 16.064 | 0775 |
| M+H | 780.3408 | 15.546 | 0776 |
| M+H | 781.1981 | 17.137 | 0777 |
| M-H | 779.1955 | 16.691 | 0778 |
| M+H | 781.2656 | 18.58 | 0779 |
| M+H | 781.2822 | 15.961 | 0780,0781 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 781.281 | 11.326 | 0780,0781 |
| M-H | 780.1791 | 16.531 | 0782 |
| M-H | 780.2036 | 10.063 | 0783 |
| M+H | 782.2463 | 15.274 | 0784,0785 |
| M+H | 782.2473 | 13.985 | 0784,0785 |
| M+H | 782.2663 | 15.732 | 0786,0787 |
| M+H | 782.2659 | 15.795 | 0786,0787 |
| M+H | 782.286 | 15.362 | 0788 |
| M-H | 781.2031 | 16.851 | 0789 |
| M-H | 781.2102 | 13.738 | 0790,0791 |
| M-H | 781.2102 | 13.768 | 0790,0791 |
| M+H | 783.2406 | 14.626 | 0792 |
| M+H | 783.26 | 15.092 | 0793,0794 |
| M+H | 783.2614 | 14.013 | 0793,0794 |
| M+H | 783.2843 | 18.073 | 0795 |
| M+H | 783.3019 | 17.731 | 0796 |
| M-H | 782.2073 | 14.579 | 0797,0798 |
| M-H | 782.2073 | 14.627 | 0797,0798 |
| M+H | 784.2409 | 14.182 | 0799,0800 |
| M+H | 784.243 | 14.45 | 0800,0801,0802 |
| M-H | 782.2337 | 14.03 | 0801,0802 |
| M+H | 784.2451 | 14.267 | 0800,0801,0802 |
| M+H | 784.3165 | 15.245 | 0803 |
| M+H | 785.2443 | 18.194 | 0805,0806 |
| M-H | 783.2271 | 17.875 | 0805,0806 |
| M-H | 784.1536 | 16.231 | 0807 |
| M+H | 786.2233 | 17.042 | 0808 |
| M+H | 786.251 | 13.861 | 0809 |
| M+H | 786.2724 | 15.793 | 0810 |
| M+H | 786.352 | 14.243 | 0811 |
| M+H | 787.2365 | 14.932 | 0812,0813 |
| M+H | 787.237 | 14.886 | 0812,0813 |
| M+H | 787.3109 | 18.399 | 0814 |
| M-H | 786.1678 | 14.909 | 0815 |
| M+H | 788.2024 | 15.486 | 0816 |
| M+H | 788.2553 | 15.882 | 0817 |
| M+H | 788.3305 | 13.358 | 0818 |

(continued)

| [Table 8-11-5] Compound that may be contained in mixture 2-1-m1E01-2 | | | |
|---|---|---|---|
| Ion species | m/z [M+H]+ or [M-H]- | Retention time of MS peak (min) | No. of assigned compound described with 2-1-m1E01-2-omitted |
| M+H | 789.1837 | 14.553 | 0819 |
| M+H | 789.2016 | 15.55 | 0820 |
| M+H | 789.292 | 18.131 | 0821 |
| M-H | 787.3133 | 16.707 | 0822 |
| M+H | 790.2173 | 15.628 | 0823,0824 |
| M+H | 790.2173 | 15.671 | 0823,0824 |
| M+H | 790.2496 | 16.231 | 0825 |
| M-H | 788.2395 | 15.083 | 0825,0826 |
| M+H | 791.238 | 16.305 | 0827 |
| M+H | 791.273 | 17.048 | 0828,0829 |
| M+H | 791.2728 | 18.064 | 0828,0829 |
| M+H | 792.2286 | 16.119 | 0830 |
| M-H | 790.2187 | 14.277 | 0830,0831 |
| M+H | 792.3008 | 15.006 | 0832 |
| M+H | 793.2303 | 14.468 | 0833 |
| M-H | 791.2199 | 9.963 | 0833,0834 |
| M-H | 792.2284 | 14.251 | 0835 |
| M+H | 794.2824 | 13.752 | 0836,0837,0838 |
| M+H | 794.2821 | 15.198 | 0836,0837,0838 |
| M+H | 794.2827 | 15.285 | 0836,0837,0838 |
| M+H | 794.3037 | 14.019 | 0839 |
| M+H | 794.3568 | 15.981 | 0840 |
| M-H | 793.2241 | 14.376 | 0841 |
| M+H | 795.2468 | 14.253 | 0841,0842 |
| M+H | 795.296 | 11.823 | 0843 |
| M+H | 796.2145 | 17.26 | 0844 |
| M+H | 796.2617 | 14.213 | 0845,0846 |
| M+H | 796.2617 | 14.263 | 0845,0846 |
| M+H | 796.335 | 15.18 | 0847 |
| M+H | 797.1931 | 17.272 | 0848 |
| M+H | 797.2291 | 16.795 | 0849 |
| M-H | 795.2412 | 17.509 | 0850,0851 |
| M+H | 797.261 | 17.424 | 0850,0851 |
| M+H | 797.2773 | 15.362 | 0852 |
| M+H | 797.3355 | 18.245 | 0853 |
| M-H | 796.1755 | 17.162 | 0854 |
| M-H | 796.1881 | 14.866 | 0855 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 798.2257 | 15.286 | 0856 |
| M+H | 798.2371 | 13.648 | 0857 |
| M+H | 798.2577 | 14.357 | 0858 |
| M+H | 799.1943 | 17.989 | 0859,0860 |
| M-H | 797.1779 | 15.98 | 0859,0860 |
| M+H | 799.2523 | 16.847 | 0861 |
| M+H | 799.2571 | 14.399 | 0862,0863 |
| M+H | 799.2567 | 14.848 | 0861,0862,0863 |
| M-H | 798.1692 | 16.682 | 0864 |
| M-H | 798.1855 | 14.926 | 0865 |
| M-H | 798.2013 | 14.704 | 0866 |
| M+H | 800.2564 | 17.674 | 0867 |
| M-H | 799.1727 | 17.791 | 0868 |
| M+H | 801.216 | 13.854 | 0869 |
| M+H | 801.2539 | 15.248 | 0870 |
| M+H | 802.2328 | 13.526 | 0871 |
| M-H | 800.2903 | 14.629 | 0872 |
| M+H | 803.218 | 17.842 | 0873,0874 |
| M+H | 803.218 | 17.908 | 0873,0874 |
| M+H | 803.3068 | 18.391 | 0875 |
| M+H | 804.1641 | 14.591 | 0876 |
| M-H | 803.2088 | 15.449 | 0877 |
| M+H | 806.2439 | 16.263 | 0878 |
| M+H | 806.3193 | 16.106 | 0879 |
| M+H | 807.2373 | 13.949 | 0880 |
| M+H | 807.268 | 16.532 | 0881 |
| M+H | 807.3032 | 16.388 | 0882 |
| M+H | 808.2968 | 16.042 | 0883 |
| M+H | 809.172 | 14.741 | 0884 |
| M+H | 809.2256 | 13.202 | 0885 |
| M+H | 809.2408 | 15.897 | 0886 |
| M+H | 809.2566 | 13.999 | 0887 |
| M+H | 810.2416 | 14.334 | 0888 |
| M+H | 810.2765 | 14.918 | 0889,0890 |
| M+H | 810.2757 | 13.791 | 0889,0890 |
| M+H | 811.2028 | 15.672 | 0891,0892,0893 |
| M-H | 809.1879 | 17.069 | 0891,0892,0893 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 811.2355 | 14.43 | 0894 |
| M+H | 811.2441 | 14.264 | 0895 |
| M+H | 812.2381 | 14.174 | 0896 |
| M-H | 812.1905 | 18.086 | 0898 |
| M-H | 812.2369 | 15.522 | 0899 |
| M-H | 813.1842 | 11.388 | 0900 |
| M+H | 815.222 | 16.789 | 0901 |
| M+H | 815.232 | 14.825 | 0902 |
| M+H | 815.2444 | 17.03 | 0903 |
| M+H | 815.2898 | 14.033 | 0904 |
| M+H | 815.3085 | 17.91 | 0905 |
| M-H | 814.18 | 14.884 | 0906 |
| M+H | 816.2344 | 14.574 | 0907 |
| M-H | 815.1449 | 18.556 | 0908 |
| M-H | 815.1631 | 17.928 | 0909,0910 |
| M+H | 817.1818 | 12.938 | 0909,0910 |
| M+H | 817.2316 | 11.741 | 0911 |
| M+H | 817.2514 | 17.826 | 0912 |
| M+H | 818.1967 | 15.553 | 0913 |
| M-H | 816.2095 | 17.287 | 0914 |
| M+H | 818.2566 | 14.217 | 0915 |
| M+H | 819.1926 | 12.219 | 0916,0917 |
| M+H | 819.1932 | 14.866 | 0916,0917 |
| M+H | 819.2128 | 17.907 | 0918,0919 |
| M-H | 817.1941 | 17.633 | 0918,0919 |
| M+H | 820.1922 | 15.292 | 0920 |
| M+H | 820.26 | 16.113 | 0921 |
| M+H | 822.2237 | 15.853 | 0922,0923 |
| M+H | 822.2237 | 15.902 | 0922,0923 |
| M+H | 822.3129 | 12.1 | 0924 |
| M+H | 823.2185 | 15.045 | 0925 |
| M+H | 823.2935 | 15.902 | 0926,0927 |
| M-H | 821.28 | 16.468 | 0926,0927 |
| M-H | 823.1303 | 16.851 | 0928 |
| M+H | 825.252 | 14.072 | 0929 |
| M+H | 825.26 | 12.075 | 0930 |
| M+H | 826.2134 | 15.425 | 0931 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 826.2767 | 10.428 | 0932 |
| M+H | 826.3091 | 14.327 | 0933 |
| M+H | 827.2024 | 17.103 | 0934 |
| M-H | 825.2058 | 12.497 | 0935 |
| M+H | 827.2441 | 10.795 | 0936 |
| M-H | 826.2068 | 18.212 | 0937 |
| M+H | 828.23 | 14.242 | 0938 |
| M+H | 829.2041 | 17.907 | 0939 |
| M-H | 828.1945 | 15.205 | 0940 |
| M+H | 830.2517 | 14.976 | 0941 |
| M-H | 829.2101 | 17.264 | 0942 |
| M+H | 831.3038 | 17.956 | 0943 |
| M-H | 830.1793 | 14.422 | 0944 |
| M-H | 830.1943 | 15.956 | 0945 |
| M-H | 831.1913 | 16.968 | 0947 |
| M+H | 833.2083 | 9.79 | 0947,0948 |
| M+H | 834.2039 | 12.195 | 0949 |
| M+H | 834.2274 | 14.605 | 0950,0951 |
| M-H | 832.2097 | 14.668 | 0950,0951 |
| M-H | 832.2251 | 12.09 | 0952 |
| M+H | 834.2519 | 14.275 | 0953 |
| M+H | 834.3144 | 15.984 | 0954 |
| M-H | 833.1565 | 12.228 | 0955 |
| M-H | 834.153 | 14.879 | 0956 |
| M-H | 834.168 | 15.316 | 0957 |
| M+H | 836.1885 | 15.733 | 0957,0958 |
| M+H | 836.2561 | 16.138 | 0959 |
| M+H | 837.2324 | 15.633 | 0960 |
| M+H | 838.2156 | 15.817 | 0961,0962 |
| M+H | 838.2158 | 12.87 | 0961,0962 |
| M-H | 840.2155 | 13.268 | 0963 |
| M+H | 842.3032 | 14.393 | 0964 |
| M-H | 842.1947 | 14.445 | 0965 |
| M-H | 842.2489 | 15.381 | 0966 |
| M-H | 844.1926 | 13.276 | 0967 |
| M+H | 846.2262 | 16.539 | 0968 |
| M+H | 846.2488 | 14.604 | 0969 |

(continued)

| [Table 8-11-5] Compound that may be contained in mixture 2-1-m1E01-2 | | | |
|---|---|---|---|
| Ion species | m/z $[M+H]^+$ or $[M-H]^-$ | Retention time of MS peak (min) | No. of assigned compound described with 2-1-m1E01-2-omitted |
| M+H | 847.224 | 10.015 | 0970 |
| M-H | 845.2291 | 15.305 | 0971 |
| M-H | 846.1922 | 18.55 | 0972 |
| M-H | 847.1959 | 14.83 | 0973 |
| M-H | 848.1668 | 14.979 | 0974 |
| M+H | 850.2319 | 15.302 | 0975 |
| M+H | 850.3056 | 16.045 | 0976 |
| M+H | 852.1639 | 17.447 | 0978 |
| M+H | 852.218 | 15.176 | 0979 |
| M+H | 853.2274 | 15.737 | 0980 |
| M+H | 854.1781 | 16.302 | 0981 |
| M-H | 859.225 | 10.481 | 0982 |
| M+H | 863.2161 | 15.436 | 0983 |
| M-H | 862.2001 | 15.938 | 0984 |
| M+H | 865.2189 | 17.273 | 0985 |
| M-H | 865.1611 | 19.71 | 0986 |
| M+H | 869.1892 | 18.089 | 0987 |
| M+H | 873.2025 | 15.158 | 0988 |
| M-H | 873.1458 | 16.398 | 0989 |
| M+H | 881.2129 | 17.314 | 0990 |
| M-H | 880.1714 | 15.221 | 0991 |
| M-H | 882.2044 | 15.477 | 0993 |
| M-H | 884.1676 | 15.524 | 0994 |
| M+H | 890.1761 | 18.189 | 0995 |
| M-H | 898.1998 | 15.541 | 0997 |
| M+H | 901.1952 | 18.349 | 0998 |
| M-H | 900.1606 | 16.582 | 0999 |
| M-H | 915.1751 | 14.882 | 1000 |

[Table 412]

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 631.2905 | 15.543 | 0001 |
| M+H | 632.2858 | 13.454 | 0002,0003 |
| M+H | 632.2865 | 14.679 | 0002,0003 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 633.2817 | 12.318 | 0004,0005 |
| M+H | 633.2816 | 16.257 | 0004,0005 |
| M+H | 634.2765 | 14.227 | 0006 |
| M+H | 638.2435 | 16.123 | 0007 |
| M+H | 639.2387 | 14.064 | 0008 |
| M+H | 645.3074 | 16.128 | 0009 |
| M+H | 646.3022 | 15.348 | 0010 |
| M+H | 647.2974 | 16.867 | 0011 |
| M+H | 649.2647 | 13.799 | 0012 |
| M+H | 649.2825 | 15.509 | 0013 |
| M+H | 650.2604 | 11.543 | 0014 |
| M+H | 650.2778 | 14.759 | 0015 |
| M+H | 650.297 | 12.48 | 0016 |
| M+H | 651.2722 | 16.306 | 0017 |
| M+H | 651.2923 | 11.049 | 0018,0019 |
| M+H | 651.2921 | 9.139 | 0018,0019 |
| M+H | 652.259 | 16.748 | 0020 |
| M+H | 652.2872 | 7.862 | 0021,0022 |
| M+H | 652.2876 | 13.296 | 0021,0022 |
| M+H | 653.2827 | 10.054 | 0023 |
| M+H | 656.234 | 16.131 | 0024 |
| M+H | 657.2492 | 12.745 | 0025 |
| M-H | 656.2279 | 9.494 | 0026 |
| M+H | 659.2856 | 13.61 | 0027 |
| M+H | 659.3231 | 16.584 | 0028 |
| M+H | 660.2816 | 11.493 | 0029,0030 |
| M+H | 660.2839 | 13.614 | 0029,0030 |
| M+H | 660.3184 | 16.018 | 0031 |
| M+H | 661.2777 | 11.398 | 0032 |
| M+H | 661.302 | 15.46 | 0033 |
| M+H | 661.3134 | 17.251 | 0034 |
| M+H | 662.297 | 14.678 | 0035 |
| M+H | 663.281 | 14.322 | 0036 |
| M+H | 663.2924 | 16.219 | 0037 |
| M+H | 664.3128 | 12.943 | 0038 |
| M+H | 665.2422 | 14.047 | 0039 |
| M+H | 665.3076 | 11.568 | 0040 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 666.2375 | 12.11 | 0041 |
| M+H | 666.2755 | 17.22 | 0042 |
| M+H | 666.3029 | 13.676 | 0043 |
| M+H | 667.2557 | 13.8 | 0044 |
| M+H | 667.2725 | 15.88 | 0045 |
| M+H | 668.254 | 16.04 | 0046 |
| M+H | 668.268 | 15.207 | 0047,0048 |
| M+H | 668.2715 | 11.238 | 0047,0048 |
| M+H | 668.2878 | 12.591 | 0049 |
| M+H | 669.2664 | 8.101 | 0050,0051 |
| M+H | 669.2669 | 8.132 | 0050,0051 |
| M-H | 667.2642 | 11.235 | 0052 |
| M+H | 670.2779 | 13.349 | 0053 |
| M+H | 671.2646 | 13.235 | 0054 |
| M+H | 673.3012 | 14.189 | 0055 |
| M+H | 674.2236 | 16.364 | 0056 |
| M+H | 674.2968 | 13.411 | 0057,0058 |
| M+H | 674.2964 | 13.461 | 0057,0058 |
| M+H | 675.2391 | 12.875 | 0059 |
| M+H | 675.2925 | 11.374 | 0060 |
| M+H | 677.2767 | 13.733 | 0061,0062 |
| M+H | 677.2773 | 11.901 | 0061,0062 |
| M+H | 677.2964 | 14.953 | 0063 |
| M+H | 678.2752 | 13.726 | 0064,0065 |
| M+H | 678.2752 | 13.669 | 0064,0065 |
| M+H | 678.2921 | 10.978 | 0066 |
| M+H | 678.3288 | 13.472 | 0067 |
| M+H | 679.258 | 14.599 | 0068 |
| M+H | 679.2766 | 14.272 | 0069 |
| M+H | 679.287 | 7.869 | 0070,0071 |
| M+H | 679.287 | 11.216 | 0070,0071 |
| M+H | 679.3235 | 12.228 | 0072 |
| M+H | 680.2817 | 8.049 | 0073 |
| M+H | 680.3057 | 9.175 | 0074 |
| M+H | 680.3182 | 14.249 | 0075 |
| M+H | 681.3026 | 11.119 | 0076,0077 |
| M+H | 681.3069 | 16.664 | 0076,0077 |
| M+H | 682.2864 | 11.722 | 0078 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 682.2981 | 13.19 | 0079,0080 |
| M+H | 682.3052 | 9.305 | 0080 |
| M+H | 683.233 | 14.118 | 0081 |
| M+H | 683.2966 | 14.47 | 0082,0083 |
| M+H | 683.2968 | 17.19 | 0082,0083 |
| M+H | 684.2482 | 11.188 | 0084 |
| M+H | 684.292 | 12.07 | 0085 |
| M+H | 685.2332 | 11.632 | 0086 |
| M+H | 685.2434 | 8.124 | 0087,0088 |
| M+H | 685.2458 | 14.113 | 0087,0088 |
| M+H | 685.2794 | 13.79 | 0089 |
| M+H | 686.2272 | 12.265 | 0090 |
| M+H | 686.2614 | 11.37 | 0091 |
| M+H | 686.2778 | 13.339 | 0092 |
| M+H | 687.2628 | 16.534 | 0093,0094 |
| M+H | 687.2628 | 16.582 | 0093,0094 |
| M+H | 687.2731 | 12.164 | 0095 |
| M+H | 687.2867 | 2.371 | 0096 |
| M+H | 687.3174 | 14.742 | 0097 |
| M+H | 688.2586 | 17.157 | 0098,0099 |
| M+H | 688.2595 | 14.767 | 0098,0099 |
| M+H | 688.2682 | 14.122 | 0100 |
| M+H | 688.2797 | 11.5 | 0101 |
| M+H | 688.3134 | 14.012 | 0102,0103 |
| M+H | 688.3126 | 14.844 | 0102,0103 |
| M+H | 689.2971 | 13.56 | 0104 |
| M+H | 690.2936 | 13.566 | 0105 |
| M+H | 691.2941 | 13.807 | 0106 |
| M+H | 691.3481 | 14.22 | 0107 |
| M+H | 692.2894 | 7.178 | 0108 |
| M+H | 692.307 | 11.413 | 0109 |
| M+H | 692.3441 | 12.037 | 0110 |
| M+H | 693.2294 | 13.554 | 0111 |
| M+H | 693.2733 | 15.144 | 0112 |
| M+H | 693.3027 | 11.637 | 0113,0114 |
| M+H | 693.302 | 10.55 | 0113,0114 |
| M+H | 694.2992 | 10.275 | 0115 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 695.252 | 13.057 | 0116,0117,0118 |
| M+H | 695.2565 | 13.989 | 0116,0117,0118 |
| M+H | 695.252 | 13.102 | 0116,0117,0118 |
| M+H | 695.2642 | 13.979 | 0119,0120 |
| M+H | 695.2661 | 14.034 | 0119,0120 |
| M+H | 696.2481 | 12.763 | 0121,0122,0123 |
| M+H | 696.2485 | 11.11 | 0121,0122,0123 |
| M+H | 696.2477 | 14.192 | 0121,0122,0123 |
| M+H | 696.2642 | 13.929 | 0124 |
| M+H | 696.2819 | 11.09 | 0125,0126 |
| M+H | 696.2829 | 9.319 | 0125,0126 |
| M+H | 696.3024 | 12.254 | 0127 |
| M+H | 697.2435 | 11.864 | 0128,0129 |
| M+H | 697.2437 | 10.787 | 0128,0129 |
| M+H | 697.2777 | 11.332 | 0130,0131 |
| M+H | 697.2777 | 8.17 | 0130,0131 |
| M+H | 697.3124 | 15.191 | 0132 |
| M+H | 698.2391 | 13.416 | 0133 |
| M+H | 698.2634 | 11.58 | 0134 |
| M+H | 698.2849 | 8.87 | 0135 |
| M+H | 699.2478 | 15.575 | 0136 |
| M+H | 699.2778 | 15.645 | 0137,0138,0139 |
| M+H | 699.2802 | 14.817 | 0137,0138,0139 |
| M+H | 699.2787 | 16.261 | 0137,0138,0139 |
| M+H | 700.2733 | 13.737 | 0140,0141 |
| M+H | 700.273 | 15.727 | 0140,0141 |
| M+H | 701.2087 | 14.774 | 0142 |
| M+H | 701.223 | 14.401 | 0143 |
| M+H | 701.2692 | 16.882 | 0144 |
| M+H | 701.2789 | 17.051 | 0145 |
| M+H | 701.2882 | 14.545 | 0146 |
| M+H | 701.3006 | 11.422 | 0147 |
| M+H | 702.2044 | 12.88 | 0148,0149 |
| M+H | 702.2044 | 15.367 | 0148,0149 |
| M+H | 702.2385 | 11.277 | 0150 |
| M+H | 702.3031 | 11.096 | 0151 |
| M+H | 702.3302 | 14.531 | 0152 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M-H | 701.1821 | 13.245 | 0153 |
| M+H | 703.2252 | 12.665 | 0154 |
| M+H | 703.2977 | 7.985 | 0155 |
| M-H | 702.2235 | 14.523 | 0156 |
| M+H | 704.2505 | 11.96 | 0157 |
| M+H | 704.3076 | 12.738 | 0158 |
| M+H | 705.2335 | 8.711 | 0159 |
| M+H | 705.2534 | 16.538 | 0160 |
| M+H | 705.2753 | 13.651 | 0161 |
| M+H | 705.3098 | 13.959 | 0162 |
| M+H | 705.365 | 14.862 | 0163 |
| M+H | 706.2304 | 16.775 | 0164 |
| M+H | 706.2688 | 13.478 | 0165 |
| M+H | 706.2898 | 11.011 | 0166 |
| M+H | 706.3233 | 11.968 | 0167 |
| M+H | 707.2641 | 13.345 | 0168,0169 |
| M+H | 707.2669 | 10.291 | 0168,0169 |
| M+H | 707.2852 | 7.982 | 0170,0171,0172 |
| M+H | 707.2848 | 10.642 | 0170,0171,0172 |
| M+H | 707.2879 | 11.981 | 0170,0171,0172 |
| M+H | 707.3178 | 12.204 | 0173,0174 |
| M+H | 707.3182 | 9.587 | 0173,0174 |
| M-H | 706.2426 | 12.499 | 0175 |
| M+H | 708.2856 | 8.966 | 0176 |
| M+H | 708.3022 | 11.504 | 0177 |
| M-H | 707.2372 | 14.177 | 0178 |
| M+H | 709.2683 | 15.35 | 0179,0180,0181 |
| M-H | 707.2517 | 13.414 | 0179,0180,0181 |
| M+H | 709.2677 | 15.074 | 0179,0180,0181 |
| M+H | 709.2989 | 14.43 | 0182,0183 |
| M+H | 709.2989 | 14.374 | 0182,0183 |
| M+H | 709.3387 | 14.239 | 0184 |
| M+H | 710.2636 | 11.438 | 0185,0186,0187 |
| M+H | 710.2641 | 14.798 | 0185,0186,0187 |
| M+H | 710.2639 | 14.849 | 0185,0186,0187 |
| M+H | 710.2778 | 13.746 | 0188 |
| M+H | 710.2939 | 13.778 | 0189,0190 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 710.2994 | 14.767 | 0189,0190 |
| M+H | 710.3541 | 11.356 | 0191 |
| M+H | 711.2603 | 16.036 | 0192,0193 |
| M+H | 711.2603 | 16.11 | 0192,0193 |
| M+H | 711.2929 | 10.656 | 0194 |
| M+H | 711.3291 | 15.814 | 0195 |
| M+H | 711.3491 | 8.237 | 0196 |
| M+H | 712.2793 | 12.131 | 0197 |
| M+H | 713.2271 | 13.641 | 0198 |
| M+H | 713.2435 | 13.201 | 0199,0200 |
| M+H | 713.2421 | 12.757 | 0199,0200 |
| M+H | 713.2584 | 16.404 | 0201 |
| M+H | 713.2644 | 12.857 | 0202 |
| M+H | 713.295 | 16.132 | 0203 |
| M+H | 713.3082 | 14.587 | 0204 |
| M+H | 714.2162 | 13.724 | 0205 |
| M+H | 714.2225 | 11.255 | 0206 |
| M+H | 714.2385 | 11.041 | 0207 |
| M+H | 714.2582 | 12.03 | 0208,0209,0210 |
| M+H | 714.2586 | 10.78 | 0208,0209,0210 |
| M+H | 714.2576 | 12.099 | 0208,0209,0210 |
| M+H | 714.2723 | 11.75 | 0211,0212 |
| M-H | 712.2559 | 9.985 | 0211,0212 |
| M+H | 715.2244 | 15.365 | 0213 |
| M+H | 715.2331 | 15.573 | 0214 |
| M+H | 715.2451 | 8.758 | 0215 |
| M+H | 715.2534 | 11.008 | 0216,0217,0218 |
| M+H | 715.2539 | 10.402 | 0216,0217,0218,0219 |
| M+H | 715.2541 | 8.832 | 0216,0217,0218,0219 |
| M+H | 715.2581 | 15.224 | 0216,0217,0218,0219 |
| M+H | 715.2677 | 11.903 | 0220 |
| M+H | 715.2728 | 16.365 | 0220,0221 |
| M+H | 715.2958 | 12.919 | 0222 |
| M+H | 715.3158 | 14.835 | 0223 |
| M+H | 716.2224 | 12.328 | 0224 |
| M+H | 716.2491 | 7.502 | 0225,0226 |
| M-H | 714.2334 | 7.305 | 0225,0226 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 716.2684 | 15.811 | 0227 |
| M+H | 716.3184 | 11.595 | 0228 |
| M+H | 717.245 | 9.098 | 0229 |
| M+H | 717.2522 | 14.649 | 0230 |
| M+H | 717.2637 | 17.054 | 0231 |
| M+H | 717.268 | 15.642 | 0231,0232 |
| M+H | 717.2731 | 16.538 | 0232,0233 |
| M+H | 717.2965 | 13.503 | 0234 |
| M+H | 718.2532 | 12.398 | 0235 |
| M+H | 718.2834 | 13.679 | 0236,0237 |
| M+H | 718.2843 | 12.921 | 0236,0237 |
| M+H | 719.1995 | 14.87 | 0238 |
| M+H | 719.2777 | 14.94 | 0239,0240,0241 |
| M+H | 719.2796 | 9.828 | 0239,0240,0241 |
| M+H | 719.28 | 12.611 | 0239,0240,0241 |
| M+H | 719.3788 | 15.446 | 0242 |
| M+H | 720.195 | 15.419 | 0243 |
| M+H | 720.2129 | 12.559 | 0244 |
| M+H | 720.229 | 12.004 | 0245 |
| M+H | 720.2745 | 14.395 | 0246 |
| M+H | 720.2842 | 14.03 | 0247 |
| M+H | 720.2939 | 11.261 | 0248 |
| M+H | 720.3057 | 11.531 | 0249 |
| M+H | 721.2104 | 9.108 | 0250,0251,0252 |
| M+H | 721.2108 | 12.39 | 0250,0251,0252 |
| M+H | 721.2147 | 12.366 | 0250,0251,0252 |
| M+H | 721.3036 | 14.585 | 0253,0254 |
| M+H | 721.3048 | 13.818 | 0253,0254 |
| M+H | 721.334 | 11.46 | 0255 |
| M+H | 721.3591 | 14.173 | 0256 |
| M+H | 722.2052 | 9.044 | 0257 |
| M+H | 722.2249 | 16.923 | 0258,0259 |
| M+H | 722.2279 | 11.557 | 0258,0259 |
| M+H | 722.2995 | 14.328 | 0260 |
| M+H | 723.2474 | 13.149 | 0261,0262 |
| M+H | 723.2478 | 13.101 | 0261,0262 |
| M+H | 723.264 | 12.809 | 0263 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 723.2841 | 14.292 | 0264,0265,0266,0267 |
| M+H | 723.2855 | 15.824 | 0264,0265,0266,0267 |
| M+H | 723.284 | 14.094 | 0264,0265,0266,0267 |
| M+H | 723.2842 | 14.221 | 0264,0265,0266,0267 |
| M+H | 723.3128 | 8.891 | 0268 |
| M+H | 724.2431 | 11.14 | 0269,0270 |
| M+H | 724.2428 | 12.974 | 0269,0270 |
| M+H | 724.2592 | 13.409 | 0271 |
| M+H | 724.2783 | 15.368 | 0272,0273,0274,0275 |
| M+H | 724.2784 | 15.436 | 0272,0273,0274,0275 |
| M+H | 724.2819 | 11.184 | 0272,0273,0274,0275 |
| M-H | 722.2637 | 11.238 | 0272,0273,0274,0275 |
| M+H | 724.3131 | 14.508 | 0276,0277 |
| M+H | 724.3133 | 11.435 | 0276,0277 |
| M+H | 724.3699 | 11.816 | 0278 |
| M+H | 725.2351 | 13.952 | 0279,0280,0281 |
| M+H | 725.2382 | 11.75 | 0279,0280,0281 |
| M+H | 725.2357 | 13.921 | 0279,0280,0281 |
| M+H | 725.2627 | 13.612 | 0282,0283 |
| M+H | 725.2617 | 13.116 | 0282,0283 |
| M+H | 725.2745 | 7.247 | 0284 |
| M+H | 725.279 | 13.344 | 0284,0285 |
| M+H | 726.2592 | 10.462 | 0286 |
| M+H | 726.2944 | 12.096 | 0287,0288 |
| M+H | 726.2952 | 11.842 | 0287,0288 |
| M+H | 727.2431 | 14.304 | 0289 |
| M+H | 727.2538 | 15.355 | 0290 |
| M-H | 725.2451 | 10.353 | 0291,0292 |
| M+H | 727.2581 | 8.881 | 0290,0291,0292 |
| M+H | 727.2731 | 14.547 | 0293 |
| M+H | 727.2943 | 14.923 | 0294,0295 |
| M+H | 727.2902 | 14.023 | 0294,0295 |
| M+H | 727.3107 | 16.631 | 0296 |
| M+H | 727.3295 | 14.661 | 0297 |
| M+H | 728.257 | 8.248 | 0298,0299 |
| M+H | 728.2558 | 12.04 | 0298,0299 |
| M-H | 726.2509 | 11.888 | 0300 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 728.2745 | 11.022 | 0301,0302,0303 |
| M+H | 728.2742 | 12.536 | 0301,0302,0303 |
| M+H | 728.2745 | 11.237 | 0301,0302,0303 |
| M+H | 728.3449 | 11.491 | 0304 |
| M+H | 729.204 | 14.102 | 0305 |
| M-H | 727.2224 | 15.825 | 0306 |
| M+H | 729.2701 | 11.523 | 0307,0308,0309 |
| M+H | 729.2696 | 10.864 | 0307,0308,0309 |
| M+H | 729.2697 | 7.861 | 0307,0308,0309 |
| M+H | 729.2837 | 11.409 | 0310 |
| M+H | 729.2881 | 15.539 | 0310,0311 |
| M+H | 730.2 | 11.858 | 0312 |
| M+H | 730.2359 | 10.536 | 0313 |
| M+H | 730.2651 | 13.044 | 0314,0315 |
| M+H | 730.2645 | 13.085 | 0314,0315 |
| M+H | 730.335 | 12.213 | 0316 |
| M-H | 729.2 | 13.675 | 0317,0318 |
| M+H | 731.2176 | 13.733 | 0317,0318 |
| M+H | 731.2336 | 13.204 | 0319,0320 |
| M+H | 731.2343 | 13.524 | 0319,0320 |
| M+H | 732.2148 | 15.257 | 0321 |
| M+H | 732.2292 | 13.296 | 0322,0323 |
| M-H | 730.2165 | 11.191 | 0322,0323 |
| M+H | 732.249 | 10.917 | 0324,0325 |
| M+H | 732.249 | 11.09 | 0324,0325 |
| M+H | 732.2648 | 12.399 | 0326 |
| M+H | 732.2701 | 12.944 | 0327 |
| M+H | 732.2999 | 13.34 | 0328 |
| M+H | 732.3137 | 11.157 | 0329 |
| M-H | 731.2114 | 14.983 | 0330,0331,0332 |
| M+H | 733.2296 | 14.943 | 0330,0331,0332 |
| M+H | 733.2278 | 7.586 | 0330,0331,0332 |
| M+H | 733.2432 | 11.183 | 0333,0334 |
| M+H | 733.2444 | 7.908 | 0333,0334 |
| M+H | 733.3129 | 15.917 | 0335 |
| M+H | 734.2309 | 12.75 | 0336 |
| M+H | 734.2404 | 11.38 | 0337 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M-H | 732.2313 | 11.989 | 0338 |
| M+H | 734.2788 | 13.701 | 0339 |
| M+H | 734.3003 | 14.667 | 0340 |
| M+H | 734.3208 | 12.107 | 0341 |
| M+H | 735.2253 | 12.858 | 0342 |
| M+H | 735.2504 | 12.401 | 0343 |
| M+H | 735.2586 | 11.657 | 0344,0345 |
| M+H | 735.2591 | 9.693 | 0344,0345 |
| M+H | 735.2747 | 12.616 | 0346 |
| M+H | 735.3195 | 13.892 | 0347,0348,0349 |
| M+H | 735.3242 | 12.105 | 0347,0348,0349 |
| M+H | 735.3188 | 13.139 | 0347,0348,0349 |
| M+H | 736.2547 | 11.722 | 0350,0351 |
| M-H | 734.2409 | 12.414 | 0350,0351 |
| M+H | 736.2691 | 14.465 | 0352 |
| M+H | 736.2745 | 12.992 | 0352,0353 |
| M+H | 736.2803 | 12.082 | 0354 |
| M+H | 736.3028 | 11.091 | 0355 |
| M+H | 736.3154 | 9.531 | 0356 |
| M-H | 735.1731 | 15.096 | 0357 |
| M+H | 737.2632 | 13.73 | 0358 |
| M+H | 737.2772 | 17.576 | 0359 |
| M+H | 737.2988 | 14.676 | 0360,0361,0362,0363 |
| M+H | 737.3001 | 14.629 | 0360,0361,0362,0363 |
| M+H | 737.3001 | 14.743 | 0360,0361,0362,0363 |
| M+H | 737.2988 | 14.882 | 0360,0361,0362,0363 |
| M+H | 738.1847 | 15.536 | 0364 |
| M+H | 738.2054 | 12.44 | 0365 |
| M+H | 738.258 | 13.091 | 0366,0367 |
| M+H | 738.2588 | 13.491 | 0366,0367 |
| M+H | 738.2854 | 12.874 | 0368 |
| M+H | 738.2939 | 10.511 | 0369 |
| M+H | 738.3855 | 12.361 | 0370 |
| M+H | 739.1987 | 12.75 | 0371 |
| M+H | 739.254 | 11.448 | 0372 |
| M+H | 739.2765 | 13.061 | 0373,0374 |
| M+H | 739.2771 | 13.081 | 0373,0374 |

(continued)

| [Table 8-11-6] Compound that may be contained in mixture 2-1-m1E02-2 | | | |
|---|---|---|---|
| Ion species | m/z [M+H]+ or [M-H]- | Retention time of MS peak (min) | No. of assigned compound described with 2-1-m1E02-2-omitted |
| M+H | 739.2945 | 14.84 | 0375 |
| M+H | 740.2201 | 10.621 | 0376 |
| M-H | 738.2623 | 12.617 | 0377,0378 |
| M-H | 738.2601 | 9.158 | 0377,0378 |
| M+H | 740.308 | 11.808 | 0379,0380 |
| M+H | 740.3105 | 12.385 | 0379,0380 |
| M+H | 740.3657 | 11.405 | 0381 |
| M+H | 741.2164 | 11.927 | 0382 |
| M+H | 741.2309 | 14 | 0383 |
| M+H | 741.2382 | 13.271 | 0384,0385 |
| M+H | 741.2417 | 12.829 | 0384,0385 |
| M+H | 741.2557 | 12.604 | 0386 |
| M+H | 741.2739 | 14.228 | 0387,0388 |
| M+H | 741.2746 | 14.171 | 0387,0388 |
| M+H | 741.3101 | 15.188 | 0389 |
| M+H | 741.363 | 15.376 | 0390 |
| M+H | 742.2312 | 11.937 | 0391,0392 |
| M+H | 742.2334 | 11.967 | 0391,0392 |
| M+H | 742.2494 | 13.792 | 0393,0394 |
| M-H | 740.2374 | 10.878 | 0393,0394 |
| M+H | 742.2707 | 10.323 | 0395 |
| M+H | 742.2838 | 14.367 | 0396 |
| M+H | 742.2898 | 11.442 | 0397,0398,0399,0400 |
| M+H | 742.2901 | 13.005 | 0397,0398,0399,0400 |
| M+H | 742.2901 | 11.793 | 0397,0398,0399,0400 |
| M-H | 740.2733 | 11.488 | 0396,0397,0398,0399,0400 |
| M+H | 743.2197 | 14.846 | 0401 |
| M+H | 743.2376 | 13.656 | 0402 |
| M+H | 743.249 | 7.714 | 0403,0404 |
| M+H | 743.2487 | 11.057 | 0403,0404 |
| M+H | 743.2717 | 16.473 | 0405,0406 |
| M+H | 743.268 | 14.73 | 0405,0406 |
| M+H | 743.286 | 12.141 | 0407,0408,0409 |
| M+H | 743.2861 | 11.416 | 0407,0408,0409 |
| M-H | 741.268 | 12.191 | 0407,0408,0409 |
| M+H | 744.2439 | 7.985 | 0410 |
| M+H | 744.2664 | 11.859 | 0411,0412,0413 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 744.2691 | 10.732 | 0412,0413 |
| M-H | 742.2523 | 11.556 | 0411,0412,0413 |
| M+H | 744.2806 | 9.534 | 0414 |
| M+H | 744.2889 | 12.913 | 0415 |
| M+H | 745.25 | 13.867 | 0416,0417 |
| M+H | 745.2516 | 13.235 | 0416,0417 |
| M+H | 745.264 | 11.086 | 0418,0419 |
| M+H | 745.2645 | 7.586 | 0418,0419,0420,0421 |
| M+H | 745.2676 | 15.926 | 0418,0419,0420,0421 |
| M+H | 745.2686 | 15.867 | 0418,0419,0420,0421 |
| M-H | 744.2312 | 11.635 | 0422,0423 |
| M-H | 744.2316 | 11.696 | 0422,0423 |
| M+H | 746.2637 | 15.409 | 0424,0425,0426,0427 |
| M+H | 746.2634 | 13.968 | 0424,0425,0426,0427 |
| M+H | 746.2647 | 11.547 | 0424,0425,0426,0427 |
| M+H | 746.2646 | 11.148 | 0424,0425,0426,0427 |
| M+H | 746.2783 | 10.507 | 0428 |
| M+H | 746.3159 | 13.845 | 0429 |
| M+H | 746.3354 | 12.243 | 0430 |
| M-H | 745.178 | 14.28 | 0431 |
| M-H | 745.2404 | 16.54 | 0432,0433,0434,0435 |
| M+H | 747.262 | 11.237 | 0432,0433,0434,0435 |
| M+H | 747.2584 | 13.785 | 0432,0433,0434,0435 |
| M+H | 747.2622 | 9.62 | 0432,0433,0434,0435 |
| M+H | 747.2738 | 12.392 | 0436 |
| M+H | 748.2098 | 11.271 | 0437 |
| M+H | 748.247 | 13.273 | 0438 |
| M+H | 748.254 | 11.235 | 0439 |
| M+H | 748.2931 | 12.886 | 0440 |
| M-H | 747.1776 | 14.146 | 0441 |
| M+H | 749.2058 | 7.687 | 0442,0443 |
| M-H | 747.1911 | 14.038 | 0442,0443 |
| M+H | 749.2245 | 13.519 | 0444 |
| M+H | 749.2418 | 10.931 | 0445 |
| M+H | 749.2612 | 13.861 | 0446 |
| M+H | 749.2977 | 12.915 | 0447 |
| M+H | 749.3399 | 11.593 | 0448,0449 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 749.3361 | 17.073 | 0448,0449 |
| M+H | 750.189 | 12.103 | 0450 |
| M+H | 750.2251 | 12.355 | 0451,0452 |
| M-H | 748.2069 | 11.351 | 0451,0452 |
| M+H | 750.2398 | 11.213 | 0453,0454 |
| M+H | 750.2399 | 12.811 | 0453,0454 |
| M+H | 750.2594 | 13.736 | 0455 |
| M+H | 750.2706 | 11.272 | 0456 |
| M+H | 751.224 | 15.918 | 0457,0458,0459,0460 |
| M+H | 751.2244 | 15.357 | 0457,0458,0459,0460 |
| M-H | 749.2062 | 15.399 | 0457,0458,0459,0460 |
| M+H | 751.2234 | 15.863 | 0457,0458,0459,0460 |
| M+H | 751.2352 | 11.997 | 0461 |
| M+H | 751.2785 | 14.375 | 0462 |
| M+H | 751.2832 | 15.421 | 0462,0463 |
| M+H | 751.3155 | 16.127 | 0464,0465 |
| M+H | 751.3155 | 16.075 | 0464,0465 |
| M+H | 752.22 | 14.047 | 0466,0467,0468 |
| M+H | 752.2198 | 14.004 | 0466,0467,0468 |
| M+H | 752.2187 | 16.375 | 0466,0467,0468 |
| M+H | 752.2303 | 13.287 | 0469 |
| M+H | 752.2344 | 12.392 | 0469,0470 |
| M+H | 752.252 | 12.512 | 0471 |
| M+H | 752.2724 | 13.773 | 0472,0473 |
| M+H | 752.2735 | 13.807 | 0472,0473 |
| M+H | 753.2225 | 16.432 | 0474 |
| M+H | 753.2492 | 10.888 | 0475 |
| M+H | 753.2588 | 13.145 | 0476 |
| M+H | 753.2925 | 13.713 | 0477,0478 |
| M+H | 753.2953 | 13.661 | 0477,0478 |
| M+H | 753.3114 | 11.162 | 0479 |
| M+H | 754.2522 | 12.997 | 0480 |
| M+H | 754.2642 | 7.17 | 0481 |
| M+H | 754.3264 | 9.076 | 0482,0483,0484 |
| M+H | 754.3263 | 13.89 | 0482,0483,0484 |
| M+H | 754.3265 | 13.849 | 0482,0483,0484 |
| M+H | 755.2532 | 13.975 | 0485,0486 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 755.2531 | 13.926 | 0485,0486 |
| M+H | 755.2715 | 12.75 | 0487 |
| M+H | 755.288 | 12.99 | 0488 |
| M+H | 755.312 | 13.549 | 0489 |
| M+H | 755.3212 | 6.333 | 0490 |
| M+H | 756.1958 | 13.014 | 0491 |
| M+H | 756.2489 | 13.22 | 0492 |
| M+H | 756.269 | 11.342 | 0493 |
| M+H | 756.3057 | 11.591 | 0494,0495,0496,0497 |
| M+H | 756.3049 | 12.023 | 0494,0495,0496,0497 |
| M+H | 756.3062 | 12.505 | 0494,0495,0496,0497 |
| M+H | 756.3048 | 11.953 | 0494,0495,0496,0497 |
| M+H | 757.1905 | 13.114 | 0498 |
| M+H | 757.2347 | 15.485 | 0499 |
| M+H | 757.2647 | 10.52 | 0500,0501 |
| M+H | 757.2644 | 11.466 | 0500,0501 |
| M+H | 757.2669 | 15.409 | 0500,0501,0502,0503 |
| M+H | 757.2669 | 15.458 | 0500,0501,0502,0503 |
| M+H | 757.2831 | 9.846 | 0504 |
| M+H | 757.3028 | 11.738 | 0505,0506 |
| M+H | 757.3077 | 10.852 | 0506 |
| M+H | 758.2595 | 7.475 | 0507 |
| M+H | 758.2792 | 14.592 | 0508 |
| M+H | 758.2849 | 11.207 | 0509,0510 |
| M-H | 756.2675 | 12.108 | 0508,0509,0510 |
| M-H | 756.2825 | 12.501 | 0511 |
| M+H | 759.2147 | 14.122 | 0512,0513 |
| M-H | 757.1978 | 14.519 | 0512,0513 |
| M+H | 759.2317 | 13.333 | 0514,0515 |
| M+H | 759.2291 | 13.611 | 0514,0515 |
| M+H | 759.2646 | 14.907 | 0516 |
| M+H | 759.2704 | 12.174 | 0516,0517 |
| M-H | 757.2622 | 11.095 | 0518,0519 |
| M+H | 759.2842 | 13.939 | 0518,0519,0520 |
| M+H | 759.2837 | 14.664 | 0518,0519,0520 |
| M+H | 759.3352 | 15.066 | 0521 |
| M+H | 760.2095 | 12.493 | 0522 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 760.2238 | 13.454 | 0523 |
| M-H | 758.2278 | 11.021 | 0524,0525 |
| M+H | 760.2437 | 10.863 | 0524,0525 |
| M+H | 760.2638 | 12.267 | 0526 |
| M+H | 760.2806 | 12.103 | 0527,0528,0529 |
| M+H | 760.2798 | 11.755 | 0527,0528,0529 |
| M+H | 760.2812 | 10.616 | 0527,0528,0529 |
| M+H | 761.24 | 7.914 | 0531,0532 |
| M-H | 759.2231 | 11.195 | 0531,0532 |
| M+H | 761.2594 | 9.438 | 0533 |
| M+H | 761.2754 | 9.983 | 0534 |
| M+H | 761.2904 | 11.771 | 0535 |
| M-H | 760.2083 | 11.828 | 0536 |
| M-H | 760.225 | 11.274 | 0537 |
| M+H | 762.2725 | 10.456 | 0538,0539 |
| M+H | 762.2776 | 10.149 | 0538,0539 |
| M+H | 763.2139 | 12.123 | 0540 |
| M+H | 763.2395 | 15.281 | 0541,0542,0543,0544,0545,0546 |
| M+H | 763.2411 | 14.125 | 0541,0542,0543,0544,0545,0546 |
| M+H | 763.2397 | 15.066 | 0541,0542,0543,0544,0545,0546 |
| M+H | 763.239 | 15.583 | 0541,0542,0543,0544,0545,0546 |
| M-H | 761.2243 | 14.843 | 0541,0542,0543,0544,0545,0546 |
| M+H | 763.2405 | 15.019 | 0541,0542,0543,0544,0545,0546 |
| M+H | 763.257 | 11.806 | 0547 |
| M+H | 763.268 | 12.512 | 0548 |
| M+H | 763.2764 | 14.315 | 0549 |
| M+H | 763.3154 | 16.045 | 0550 |
| M+H | 763.3554 | 12.001 | 0551 |
| M+H | 764.2353 | 13.165 | 0552,0553 |
| M+H | 764.2354 | 13.198 | 0552,0553 |
| M-H | 762.2412 | 11.779 | 0554,0555 |
| M-H | 762.2418 | 11.812 | 0554,0555 |
| M+H | 764.3103 | 14.283 | 0557 |
| M-H | 763.1525 | 16.639 | 0558 |
| M+H | 765.1841 | 14.544 | 0559 |
| M+H | 765.2311 | 16.311 | 0560 |
| M+H | 765.2405 | 13.856 | 0561,0562 |

(continued)

| [Table 8-11-6] Compound that may be contained in mixture 2-1-m1E02-2 | | | |
|---|---|---|---|
| Ion species | m/z [M+H]+ or [M-H]- | Retention time of MS peak (min) | No. of assigned compound described with 2-1-m1E02-2-omitted |
| M+H | 765.2416 | 16.494 | 0561,0562 |
| M+H | 765.2491 | 13.967 | 0563,0564 |
| M+H | 765.2516 | 10.895 | 0563,0564 |
| M+H | 765.3303 | 12.672 | 0565 |
| M+H | 766.167 | 13.736 | 0566 |
| M+H | 766.1992 | 11.469 | 0567 |
| M+H | 766.235 | 15.033 | 0568 |
| M-H | 764.2476 | 10.464 | 0569 |
| M+H | 766.2904 | 14.375 | 0570 |
| M-H | 765.1666 | 14.223 | 0571 |
| M+H | 767.2303 | 13.11 | 0572 |
| M-H | 765.2364 | 15.655 | 0573 |
| M+H | 767.2598 | 7.162 | 0574 |
| M+H | 767.2668 | 16.245 | 0575 |
| M+H | 767.2789 | 15.586 | 0576 |
| M+H | 767.3115 | 14.591 | 0577 |
| M+H | 767.3212 | 12.168 | 0578 |
| M-H | 766.1832 | 11.987 | 0579 |
| M-H | 766.1967 | 12.038 | 0580 |
| M+H | 768.23 | 12.427 | 0581 |
| M+H | 768.2692 | 13.131 | 0582,0583 |
| M+H | 768.2693 | 10.46 | 0582,0583 |
| M+H | 768.3397 | 12.258 | 0584,0585 |
| M+H | 768.3392 | 12.287 | 0584,0585 |
| M+H | 769.1946 | 15.586 | 0586,0587 |
| M+H | 769.1942 | 8.414 | 0586,0587 |
| M+H | 769.2149 | 15.418 | 0588,0589,0590 |
| M+H | 769.215 | 15.917 | 0588,0589,0590 |
| M+H | 769.2144 | 15.372 | 0588,0589,0590 |
| M+H | 769.2645 | 10.04 | 0591 |
| M+H | 769.2686 | 14.497 | 0591,0592 |
| M+H | 769.2911 | 10.236 | 0593 |
| M+H | 769.3281 | 10.656 | 0594 |
| M+H | 770.1905 | 16.023 | 0595 |
| M+H | 770.2306 | 12.924 | 0596,0597 |
| M+H | 770.2308 | 12.963 | 0596,0597 |
| M+H | 770.2841 | 11.898 | 0598 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M-H | 768.2723 | 12.524 | 0598,0599 |
| M+H | 770.3208 | 12.854 | 0600,0601 |
| M+H | 770.3211 | 13.137 | 0600,0601 |
| M+H | 771.2262 | 10.106 | 0602,0603,0604,0605 |
| M+H | 771.226 | 11.507 | 0602,0603,0604,0605 |
| M+H | 771.2262 | 12.78 | 0602,0603,0604,0605 |
| M+H | 771.2259 | 12.717 | 0602,0603,0604,0605 |
| M+H | 771.2453 | 17.254 | 0606,0607 |
| M+H | 771.2482 | 13.349 | 0606,0607 |
| M+H | 771.2643 | 11.372 | 0608 |
| M+H | 771.2803 | 11.042 | 0609,0610 |
| M+H | 771.2803 | 9.518 | 0609,0610 |
| M+H | 771.2992 | 15.259 | 0611 |
| M+H | 771.321 | 17.148 | 0612 |
| M+H | 772.2216 | 12.149 | 0613,0614 |
| M+H | 772.2267 | 13.036 | 0613,0614 |
| M-H | 770.2478 | 10.955 | 0615 |
| M+H | 772.2997 | 11.78 | 0616,0617 |
| M+H | 772.2975 | 10.84 | 0616,0617 |
| M+H | 772.3171 | 9.282 | 0618 |
| M+H | 773.2163 | 13.346 | 0619 |
| M-H | 771.2125 | 15.135 | 0620 |
| M+H | 773.2611 | 14.953 | 0621,0622 |
| M+H | 773.2633 | 14.327 | 0621,0622 |
| M-H | 771.2831 | 13.648 | 0623,0624 |
| M+H | 773.3005 | 13.611 | 0623,0624 |
| M-H | 772.2233 | 13.514 | 0625 |
| M+H | 774.2582 | 13.934 | 0626,0627,0628 |
| M+H | 774.2562 | 12.403 | 0626,0627,0628 |
| M+H | 774.2592 | 11.376 | 0626,0627,0628 |
| M+H | 774.2771 | 10.362 | 0629 |
| M+H | 774.2997 | 8.595 | 0630 |
| M+H | 774.3162 | 11.041 | 0631 |
| M+H | 775.2513 | 13.628 | 0632,0633 |
| M+H | 775.2545 | 10.66 | 0632,0633 |
| M+H | 775.2636 | 8.573 | 0634 |
| M+H | 775.2787 | 13.461 | 0635 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 775.2934 | 11.125 | 0636 |
| M+H | 775.3107 | 8.11 | 0637 |
| M+H | 775.3312 | 15.254 | 0638 |
| M+H | 776.2408 | 12.469 | 0639 |
| M+H | 776.277 | 1.766 | 0640,0641 |
| M+H | 776.2723 | 10.523 | 0640,0641 |
| M+H | 776.2894 | 9.537 | 0642 |
| M+H | 777.1825 | 12.944 | 0643 |
| M-H | 775.1877 | 14.649 | 0644 |
| M+H | 777.2195 | 13.62 | 0645 |
| M-H | 775.2072 | 15.397 | 0645,0646 |
| M+H | 777.2545 | 15.581 | 0647,0648 |
| M+H | 777.2545 | 14.479 | 0647,0648 |
| M+H | 777.2673 | 9.869 | 0649 |
| M+H | 777.2739 | 12.873 | 0649,0650 |
| M+H | 777.2843 | 11.821 | 0651 |
| M+H | 777.2941 | 12.398 | 0652 |
| M+H | 777.3326 | 13.959 | 0653 |
| M+H | 778.1768 | 13.107 | 0654 |
| M-H | 776.2036 | 12.089 | 0655,0656 |
| M+H | 778.2203 | 11.391 | 0655,0656 |
| M+H | 778.2341 | 10.983 | 0657,0658 |
| M-H | 776.2182 | 11.651 | 0657,0658 |
| M+H | 778.2511 | 10.569 | 0659 |
| M+H | 778.2702 | 12.257 | 0660 |
| M+H | 778.3413 | 12.569 | 0661 |
| M-H | 777.1868 | 14.176 | 0663 |
| M+H | 779.2153 | 8.61 | 0664 |
| M-H | 777.2018 | 15.508 | 0664,0665 |
| M-H | 777.2137 | 11.748 | 0666 |
| M+H | 779.2336 | 14.288 | 0666,0667,0668 |
| M+H | 779.2336 | 15.681 | 0666,0667,0668 |
| M+H | 779.2558 | 16.947 | 0669,0670 |
| M+H | 779.2559 | 16.88 | 0669,0670 |
| M+H | 779.277 | 12.661 | 0671 |
| M+H | 780.2304 | 14.063 | 0672 |
| M+H | 780.2513 | 11.134 | 0673 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 780.2627 | 10.283 | 0674 |
| M+H | 780.2804 | 11.004 | 0675 |
| M+H | 781.2131 | 14.709 | 0676 |
| M+H | 781.2261 | 16.339 | 0677 |
| M+H | 781.2305 | 14.14 | 0677,0678 |
| M+H | 781.2338 | 15.055 | 0678,0679,0680 |
| M+H | 781.2334 | 15.014 | 0678,0679,0680 |
| M+H | 781.3066 | 16.04 | 0681 |
| M-H | 780.1974 | 12.41 | 0682 |
| M+H | 782.2455 | 13.178 | 0683,0684,0685,0686 |
| M+H | 782.2458 | 12.011 | 0683,0684,0685,0686 |
| M+H | 782.2463 | 8.341 | 0683,0684,0685,0686 |
| M+H | 782.2478 | 8.433 | 0683,0684,0685,0686 |
| M+H | 782.2853 | 13.526 | 0687 |
| M+H | 782.3215 | 10.925 | 0688 |
| M+H | 782.358 | 9.342 | 0689 |
| M+H | 783.1618 | 16.793 | 0690 |
| M+H | 783.2421 | 9.534 | 0691,0692,0693 |
| M+H | 783.2418 | 11.692 | 0691,0692,0693 |
| M+H | 783.2426 | 12.437 | 0691,0692,0693 |
| M+H | 783.2606 | 16.373 | 0694,0695 |
| M+H | 783.2607 | 16.323 | 0694,0695 |
| M+H | 783.3164 | 10.295 | 0696 |
| M+H | 783.3442 | 14.754 | 0697 |
| M-H | 782.1598 | 13.434 | 0698 |
| M-H | 782.174 | 12.177 | 0699 |
| M+H | 784.2265 | 13.033 | 0700 |
| M+H | 784.2365 | 13.711 | 0701 |
| M+H | 784.246 | 13.348 | 0702,0703 |
| M-H | 782.2301 | 12.458 | 0702,0703 |
| M+H | 784.2552 | 10.659 | 0704 |
| M+H | 784.334 | 10.145 | 0705 |
| M+H | 785.172 | 8.689 | 0706,0707 |
| M-H | 783.1569 | 14.521 | 0706,0707 |
| M+H | 785.19 | 15.688 | 0708 |
| M+H | 785.2412 | 12.657 | 0709,0710,0711 |
| M-H | 783.2246 | 11.704 | 0709,0710,0711 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 785.2434 | 12.827 | 0709,0710,0711 |
| M+H | 785.2952 | 11.582 | 0712 |
| M-H | 783.3031 | 13.603 | 0713 |
| M+H | 785.3402 | 13.179 | 0714 |
| M+H | 786.1871 | 16.219 | 0715,0716 |
| M+H | 786.1871 | 16.121 | 0715,0716 |
| M+H | 786.2372 | 9.628 | 0717,0718 |
| M+H | 786.2361 | 13.01 | 0717,0718 |
| M+H | 786.2597 | 13.175 | 0719 |
| M+H | 786.2711 | 13.947 | 0720 |
| M+H | 786.2855 | 12.561 | 0721 |
| M+H | 786.3163 | 12.248 | 0722 |
| M+H | 787.2031 | 15.628 | 0723,0724 |
| M+H | 787.2046 | 16.254 | 0723,0724 |
| M-H | 785.2286 | 15.722 | 0725 |
| M+H | 787.2738 | 10.654 | 0726 |
| M+H | 788.2023 | 13.375 | 0727 |
| M-H | 786.2587 | 11.908 | 0731,0732 |
| M+H | 788.2739 | 14.443 | 0731,0732 |
| M+H | 788.293 | 10.821 | 0733 |
| M+H | 788.3314 | 11.524 | 0734 |
| M+H | 789.2008 | 11.928 | 0735,0736 |
| M+H | 789.1973 | 13.494 | 0735,0736 |
| M+H | 789.2282 | 14.506 | 0737 |
| M+H | 789.2924 | 16.794 | 0738,0739 |
| M+H | 789.2915 | 15.089 | 0738,0739 |
| M+H | 790.2507 | 14.547 | 0740,0741 |
| M-H | 788.2379 | 10.978 | 0740,0741 |
| M-H | 788.2543 | 12.398 | 0742 |
| M+H | 790.3056 | 12.404 | 0743 |
| M+H | 791.2348 | 14.219 | 0744 |
| M+H | 791.2533 | 14.47 | 0745 |
| M+H | 791.2692 | 16.071 | 0746,0747,0748 |
| M+H | 791.2703 | 15.993 | 0746,0747,0748 |
| M+H | 791.2747 | 12.402 | 0746,0747,0748 |
| M+H | 791.2884 | 13.429 | 0749 |
| M+H | 791.3466 | 16.948 | 0750 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M-H | 790.2124 | 14.013 | 0751 |
| M-H | 790.2198 | 12.554 | 0751,0752 |
| M+H | 792.3063 | 11.171 | 0753 |
| M+H | 793.1992 | 13.983 | 0754 |
| M+H | 793.2461 | 11.375 | 0755 |
| M+H | 793.2504 | 14.958 | 0755,0756,0757 |
| M+H | 793.2515 | 13.495 | 0756,0757 |
| M+H | 793.325 | 12.65 | 0758 |
| M+H | 794.2463 | 14.041 | 0759 |
| M+H | 794.2668 | 12.526 | 0760,0761 |
| M+H | 794.2673 | 11.566 | 0760,0761 |
| M+H | 794.2863 | 11.206 | 0762 |
| M+H | 794.2958 | 11.654 | 0763 |
| M+H | 794.3359 | 12.656 | 0764 |
| M-H | 793.1684 | 12.451 | 0765 |
| M-H | 793.1777 | 14.911 | 0766 |
| M-H | 793.2283 | 10.921 | 0767 |
| M-H | 794.1939 | 12.206 | 0768 |
| M+H | 796.2247 | 11.634 | 0769 |
| M-H | 794.2136 | 12.994 | 0769,0770 |
| M+H | 796.2455 | 11.77 | 0771 |
| M+H | 796.2639 | 12.599 | 0772,0773 |
| M+H | 796.2615 | 12.875 | 0772,0773 |
| M+H | 796.2747 | 10.561 | 0774 |
| M+H | 796.3 | 14.037 | 0775 |
| M+H | 796.3359 | 11.098 | 0776 |
| M-H | 795.1742 | 15.283 | 0777 |
| M+H | 797.2079 | 14.864 | 0778 |
| M+H | 797.2575 | 8.122 | 0779 |
| M+H | 797.2743 | 15.405 | 0780,0781 |
| M+H | 797.2751 | 15.361 | 0780,0781 |
| M-H | 796.1748 | 13.922 | 0782 |
| M-H | 796.1933 | 12.02 | 0783 |
| M+H | 798.2406 | 13.24 | 0784,0785 |
| M+H | 798.2411 | 12.087 | 0784,0785 |
| M+H | 798.2623 | 13.857 | 0786,0787 |
| M-H | 796.2453 | 12.221 | 0786,0787 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 798.2796 | 13.132 | 0788 |
| M-H | 797.1943 | 15.354 | 0789 |
| M+H | 799.2205 | 11.509 | 0790,0791 |
| M+H | 799.2208 | 15.295 | 0790,0791 |
| M+H | 799.2361 | 12.491 | 0792 |
| M+H | 799.257 | 13.039 | 0793,0794 |
| M+H | 799.2571 | 13.09 | 0793,0794 |
| M+H | 799.2806 | 13.985 | 0795 |
| M+H | 799.298 | 16.315 | 0796 |
| M-H | 798.2036 | 12.498 | 0797,0798 |
| M-H | 798.2036 | 12.528 | 0797,0798 |
| M+H | 800.2304 | 13.797 | 0799 |
| M+H | 800.236 | 12.264 | 0799,0800 |
| M+H | 800.2419 | 12.708 | 0800,0801,0802 |
| M-H | 798.2228 | 12.75 | 0800,0801,0802 |
| M+H | 800.3121 | 13.285 | 0803 |
| M-H | 799.1334 | 17.081 | 0804 |
| M+H | 801.2377 | 16.992 | 0805,0806 |
| M+H | 801.2385 | 16.888 | 0805,0806 |
| M-H | 800.1496 | 13.632 | 0807 |
| M+H | 802.217 | 15.336 | 0808 |
| M+H | 802.2455 | 11.498 | 0809 |
| M+H | 802.2661 | 14.012 | 0810 |
| M+H | 802.3468 | 12.053 | 0811 |
| M+H | 803.2318 | 12.707 | 0812,0813 |
| M-H | 801.2146 | 12.748 | 0812,0813 |
| M+H | 803.3094 | 15.016 | 0814 |
| M-H | 802.1633 | 12.806 | 0815 |
| M+H | 804.1965 | 13.457 | 0816 |
| M+H | 804.2497 | 13.693 | 0817 |
| M+H | 804.3267 | 11.102 | 0818 |
| M+H | 805.1771 | 12.272 | 0819 |
| M+H | 805.1943 | 11.549 | 0820 |
| M+H | 805.2871 | 16.877 | 0821 |
| M-H | 803.3077 | 14.762 | 0822 |
| M+H | 806.2108 | 13.616 | 0823,0824 |
| M+H | 806.2095 | 14.953 | 0823,0824 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 806.2449 | 14.183 | 0825 |
| M+H | 806.2513 | 12.13 | 0825,0826 |
| M+H | 807.2289 | 14.3 | 0827 |
| M+H | 807.2657 | 15.435 | 0828,0829 |
| M+H | 807.2662 | 15.379 | 0828,0829 |
| M+H | 808.2258 | 14.183 | 0830 |
| M-H | 806.2141 | 12.146 | 0830,0831 |
| M+H | 808.3013 | 12.784 | 0832 |
| M-H | 807.2124 | 15.722 | 0833,0834 |
| M-H | 807.2122 | 15.633 | 0833,0834 |
| M-H | 808.2228 | 12.131 | 0835 |
| M+H | 810.279 | 13.263 | 0836,0837,0838 |
| M+H | 810.2815 | 11.262 | 0836,0837,0838 |
| M+H | 810.2777 | 13.342 | 0836,0837,0838 |
| M+H | 810.2968 | 11.819 | 0839 |
| M+H | 810.3567 | 13.175 | 0840 |
| M-H | 809.2195 | 12.219 | 0841 |
| M-H | 809.2279 | 11.385 | 0842 |
| M+H | 811.2919 | 10.378 | 0843 |
| M-H | 810.1899 | 14.671 | 0844 |
| M+H | 812.2568 | 11.924 | 0845,0846 |
| M+H | 812.2566 | 12.289 | 0845,0846 |
| M+H | 812.3289 | 10.59 | 0847 |
| M-H | 811.1685 | 15.408 | 0848 |
| M+H | 813.2235 | 14.943 | 0849 |
| M+H | 813.2538 | 16.053 | 0850,0851 |
| M+H | 813.2538 | 10.978 | 0850,0851 |
| M+H | 813.273 | 13.567 | 0852 |
| M+H | 813.3287 | 16.965 | 0853 |
| M-H | 812.1698 | 13.439 | 0854 |
| M-H | 812.1854 | 12.814 | 0855 |
| M+H | 814.2203 | 12.942 | 0856 |
| M+H | 814.2318 | 11.373 | 0857 |
| M+H | 814.2504 | 12.617 | 0858 |
| M+H | 815.1872 | 16.387 | 0859,0860 |
| M+H | 815.1872 | 16.315 | 0859,0860 |
| M+H | 815.2463 | 14.996 | 0861 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 815.2517 | 12.689 | 0861,0862,0863 |
| M+H | 815.2524 | 12.196 | 0862,0863 |
| M+H | 816.1813 | 14.265 | 0864 |
| M-H | 814.1807 | 12.809 | 0865 |
| M-H | 814.1983 | 12.592 | 0866 |
| M+H | 816.252 | 16.081 | 0867 |
| M-H | 815.163 | 15.132 | 0868 |
| M+H | 817.2113 | 11.702 | 0869 |
| M+H | 817.2493 | 13.203 | 0870 |
| M-H | 816.2085 | 13.144 | 0871 |
| M+H | 818.303 | 12.439 | 0872 |
| M+H | 819.2131 | 16.582 | 0873,0874 |
| M+H | 819.2093 | 16.04 | 0873,0874 |
| M-H | 817.2838 | 12.018 | 0875 |
| M-H | 818.1394 | 14.476 | 0876 |
| M+H | 821.2227 | 13.337 | 0877 |
| M+H | 822.24 | 14.315 | 0878 |
| M-H | 820.2959 | 8.182 | 0879 |
| M+H | 823.2322 | 11.529 | 0880 |
| M+H | 823.2578 | 18.76 | 0881 |
| M+H | 823.2968 | 14.518 | 0882 |
| M+H | 824.2938 | 11.95 | 0883 |
| M+H | 825.1667 | 12.64 | 0884 |
| M+H | 825.2201 | 14.435 | 0885 |
| M+H | 825.2367 | 13.723 | 0886 |
| M+H | 825.252 | 11.864 | 0887 |
| M-H | 824.2191 | 12.214 | 0888 |
| M+H | 826.2717 | 13.284 | 0889,0890 |
| M-H | 824.2545 | 13.202 | 0889,0890 |
| M+H | 827.1972 | 13.544 | 0891,0892 |
| M+H | 827.1973 | 13.731 | 0891,0892 |
| M-H | 825.1841 | 15.484 | 0891,0892,0893 |
| M+H | 827.2306 | 12.33 | 0894 |
| M-H | 825.2243 | 15.505 | 0895 |
| M+H | 828.2314 | 12.149 | 0896 |
| M-H | 827.1833 | 17.012 | 0897 |
| M-H | 828.1811 | 13.798 | 0898 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M-H | 828.2343 | 13.372 | 0899 |
| M+H | 831.1924 | 15.174 | 0900 |
| M+H | 831.2167 | 14.936 | 0901 |
| M+H | 831.2272 | 12.696 | 0902 |
| M+H | 831.241 | 15.148 | 0903 |
| M+H | 831.2832 | 11.839 | 0904 |
| M+H | 831.3038 | 16.65 | 0905 |
| M+H | 832.1912 | 12.865 | 0906 |
| M+H | 832.2298 | 12.49 | 0907 |
| M-H | 831.1445 | 11.651 | 0908 |
| M-H | 831.1575 | 15.209 | 0909,0910 |
| M-H | 831.1586 | 16.464 | 0909,0910 |
| M+H | 833.2272 | 9.272 | 0911 |
| M-H | 831.2278 | 16.412 | 0912 |
| M-H | 832.1757 | 13.269 | 0913 |
| M+H | 834.2223 | 15.731 | 0914 |
| M+H | 834.2523 | 11.997 | 0915 |
| M+H | 835.1874 | 9.523 | 0916,0917 |
| M+H | 835.1872 | 12.766 | 0916,0917 |
| M+H | 835.2079 | 16.646 | 0918,0919 |
| M+H | 835.2082 | 16.118 | 0918,0919 |
| M-H | 834.1693 | 13.241 | 0920 |
| M+H | 836.2585 | 11.402 | 0921 |
| M+H | 838.2167 | 13.941 | 0922,0923 |
| M-H | 836.1993 | 14.029 | 0922,0923 |
| M+H | 838.3088 | 10.616 | 0924 |
| M+H | 839.2145 | 13.118 | 0925 |
| M-H | 837.2751 | 14.669 | 0926,0927 |
| M+H | 839.293 | 14.591 | 0926,0927 |
| M+H | 841.1436 | 14.327 | 0928 |
| M+H | 841.2469 | 11.908 | 0929 |
| M-H | 839.2374 | 15.962 | 0930 |
| M+H | 842.2072 | 13.247 | 0931 |
| M+H | 842.2647 | 14.241 | 0932 |
| M+H | 842.3024 | 12.269 | 0933 |
| M-H | 841.1782 | 15.619 | 0934 |
| M+H | 843.2164 | 11.873 | 0935 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M-H | 841.218 | 14.916 | 0936 |
| M+H | 844.2124 | 12.066 | 0937 |
| M+H | 844.2254 | 12.175 | 0938 |
| M+H | 845.1963 | 16.409 | 0939 |
| M-H | 844.1905 | 13.309 | 0940 |
| M+H | 846.2451 | 12.907 | 0941 |
| M+H | 847.2209 | 15.818 | 0942 |
| M+H | 847.2985 | 16.694 | 0943 |
| M-H | 846.1737 | 10.922 | 0944 |
| M-H | 846.1916 | 13.805 | 0945 |
| M-H | 847.1846 | 14.456 | 0947 |
| M+H | 849.2037 | 14.597 | 0947,0948 |
| M+H | 850.1986 | 9.107 | 0949 |
| M+H | 850.2191 | 15.814 | 0950,0951 |
| M-H | 848.2037 | 12.595 | 0950,0951 |
| M+H | 850.2326 | 13.465 | 0952 |
| M+H | 850.2471 | 12.063 | 0953 |
| M+H | 850.307 | 12.318 | 0954 |
| M+H | 851.1679 | 16.729 | 0955 |
| M-H | 850.1452 | 15.062 | 0956 |
| M-H | 850.1634 | 13.455 | 0957,0958 |
| M+H | 852.1836 | 13.5 | 0957,0958 |
| M+H | 852.2503 | 14.185 | 0959 |
| M+H | 853.2274 | 13.657 | 0960 |
| M-H | 852.1939 | 14.02 | 0961,0962 |
| M-H | 852.1939 | 13.981 | 0961,0962 |
| M+H | 858.2288 | 12.622 | 0963 |
| M+H | 858.2978 | 12.346 | 0964 |
| M-H | 858.1925 | 12.189 | 0965 |
| M+H | 860.2614 | 13.419 | 0966 |
| M-H | 860.1848 | 13.366 | 0967 |
| M-H | 860.2061 | 14.376 | 0968 |
| M-H | 860.2233 | 12.51 | 0969 |
| M+H | 863.2182 | 14.187 | 0970 |
| M-H | 861.2216 | 16.002 | 0971 |
| M-H | 863.1912 | 12.406 | 0973 |
| M-H | 864.1652 | 11.219 | 0974 |

(continued)

| [Table 8-11-6] Compound that may be contained in mixture 2-1-m1E02-2 | | | |
|---|---|---|---|
| Ion species | m/z $[M+H]^+$ or $[M-H]^-$ | Retention time of MS peak (min) | No. of assigned compound described with 2-1-m1E02-2-omitted |
| M+H | 866.2294 | 13.512 | 0975 |
| M+H | 866.3051 | 14.278 | 0976 |
| M-H | 865.1755 | 14.754 | 0977 |
| M-H | 866.1401 | 15.077 | 0978 |
| M-H | 866.1952 | 13.166 | 0979 |
| M+H | 869.2237 | 13.718 | 0980 |
| M-H | 868.1564 | 14.107 | 0981 |
| M+H | 877.2362 | 16.212 | 0982 |
| M+H | 879.2115 | 13.295 | 0983 |
| M-H | 878.1959 | 13.741 | 0984 |
| M+H | 881.2128 | 15.73 | 0985 |
| M+H | 883.1745 | 17.292 | 0986 |
| M-H | 883.1655 | 15.627 | 0987 |
| M+H | 889.1973 | 13.103 | 0988 |
| M+H | 891.1598 | 14.17 | 0989 |
| M+H | 897.2084 | 15.814 | 0990 |
| M-H | 896.1685 | 13.188 | 0991 |
| M+H | 899.1694 | 17.396 | 0992 |
| M-H | 898.2008 | 13.552 | 0993 |
| M-H | 900.1618 | 14.586 | 0994 |
| M+H | 906.1704 | 15.723 | 0995 |
| M-H | 912.1649 | 13.277 | 0996 |
| M-H | 914.1961 | 13.647 | 0997 |
| M-H | 915.1755 | 13.054 | 0998 |
| M-H | 916.157 | 14.698 | 0999 |
| M+H | 933.1854 | 16.587 | 1000 |

[Table 413]

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 669.2529 | 16.657 | 0001 |
| M+H | 670.248 | 15.137 | 0002,0003 |
| M+H | 670.2487 | 16.14 | 0002,0003 |
| M+H | 671.2428 | 14.334 | 0004,0005 |
| M+H | 671.2426 | 17.392 | 0004,0005 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 672.2376 | 15.989 | 0006 |
| M+H | 676.2036 | 17.198 | 0007 |
| M+H | 677.2 | 15.777 | 0008 |
| M+H | 683.2683 | 17.088 | 0009 |
| M+H | 684.2637 | 16.694 | 0010 |
| M+H | 685.2586 | 17.79 | 0011 |
| M+H | 687.228 | 15.345 | 0012 |
| M+H | 687.2417 | 16.672 | 0013 |
| M+H | 688.2228 | 13.554 | 0014 |
| M+H | 688.2379 | 16.141 | 0015 |
| M+H | 688.2584 | 14.122 | 0016 |
| M+H | 689.2338 | 17.267 | 0017 |
| M+H | 689.2541 | 13.033 | 0018,0019 |
| M+H | 689.254 | 13.064 | 0018,0019 |
| M+H | 690.2194 | 17.635 | 0020 |
| M+H | 690.2487 | 10.109 | 0021,0022 |
| M+H | 690.2483 | 15.015 | 0021,0022 |
| M+H | 691.2443 | 12.335 | 0023 |
| M+H | 694.1943 | 17.167 | 0024 |
| M+H | 695.2104 | 14.48 | 0025 |
| M+H | 696.2052 | 11.667 | 0026 |
| M+H | 697.2462 | 15.189 | 0027 |
| M+H | 697.2828 | 17.513 | 0028 |
| M+H | 698.2431 | 13.496 | 0029,0030 |
| M+H | 698.243 | 13.551 | 0029,0030 |
| M+H | 698.2784 | 17.137 | 0031 |
| M+H | 699.2408 | 13.507 | 0032 |
| M+H | 699.2623 | 16.619 | 0033 |
| M+H | 699.2735 | 18.136 | 0034 |
| M+H | 700.2588 | 14.564 | 0035 |
| M+H | 701.2428 | 15.87 | 0036 |
| M+H | 701.2534 | 17.331 | 0037 |
| M+H | 702.2739 | 14.542 | 0038 |
| M+H | 703.2025 | 15.555 | 0039 |
| M+H | 703.2693 | 13.503 | 0040 |
| M+H | 704.1998 | 14.01 | 0041 |
| M+H | 704.2354 | 17.968 | 0042 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 704.265 | 15.309 | 0043 |
| M+H | 705.2169 | 15.381 | 0044 |
| M+H | 705.2332 | 16.889 | 0045 |
| M+H | 706.2142 | 17.149 | 0046 |
| M+H | 706.2315 | 13.191 | 0047,0048 |
| M+H | 706.2297 | 16.517 | 0047,0048 |
| M+H | 706.2489 | 14.174 | 0049 |
| M+H | 707.2224 | 17.542 | 0050 |
| M+H | 707.2279 | 10.254 | 0050,0051 |
| M+H | 707.2423 | 13.181 | 0052 |
| M+H | 708.2405 | 14.938 | 0053 |
| M+H | 709.2268 | 14.865 | 0054 |
| M+H | 711.2623 | 15.706 | 0055 |
| M+H | 712.1854 | 17.34 | 0056 |
| M+H | 712.2581 | 15.064 | 0057,0058 |
| M+H | 712.2579 | 15.108 | 0057,0058 |
| M+H | 713.2007 | 14.517 | 0059 |
| M+H | 713.2537 | 13.431 | 0060 |
| M-H | 713.2196 | 15.268 | 0061,0062 |
| M-H | 713.2209 | 15.204 | 0061,0062 |
| M+H | 715.2595 | 16.275 | 0063 |
| M+H | 716.2338 | 15.376 | 0064,0065 |
| M+H | 716.2338 | 15.312 | 0064,0065 |
| M+H | 716.2537 | 12.88 | 0066 |
| M+H | 716.2897 | 14.969 | 0067 |
| M+H | 717.2182 | 16.046 | 0068 |
| M+H | 717.2358 | 11.725 | 0069 |
| M+H | 717.2487 | 9.986 | 0070,0071 |
| M+H | 717.2481 | 13.16 | 0070,0071 |
| M+H | 717.2857 | 14.048 | 0072 |
| M+H | 718.2445 | 10.219 | 0073 |
| M+H | 718.2691 | 12.257 | 0074 |
| M+H | 718.2804 | 11.848 | 0075 |
| M+H | 719.2639 | 10.892 | 0076,0077 |
| M+H | 719.2672 | 17.537 | 0076,0077 |
| M+H | 720.2482 | 13.571 | 0078 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 720.2634 | 17.169 | 0079,0080 |
| M+H | 720.2668 | 12.049 | 0080 |
| M+H | 721.1969 | 15.626 | 0081 |
| M+H | 721.2576 | 18.125 | 0082,0083 |
| M+H | 721.2583 | 15.944 | 0082,0083 |
| M+H | 722.2097 | 13.039 | 0084 |
| M+H | 722.2541 | 14.027 | 0085 |
| M+H | 723.194 | 13.414 | 0086 |
| M+H | 723.2049 | 10.227 | 0087,0088 |
| M+H | 723.2105 | 15.596 | 0088 |
| M+H | 723.2413 | 15.286 | 0089 |
| M+H | 724.1886 | 14.025 | 0090 |
| M+H | 724.2225 | 13.225 | 0091 |
| M+H | 724.2413 | 14.785 | 0092 |
| M+H | 725.2242 | 17.449 | 0093,0094 |
| M-H | 723.2084 | 13.349 | 0093,0094 |
| M+H | 725.2354 | 13.942 | 0095 |
| M+H | 725.2448 | 11.744 | 0096 |
| M+H | 725.2798 | 16.152 | 0097 |
| M+H | 726.2192 | 16.212 | 0098,0099 |
| M+H | 726.2208 | 16.134 | 0098,0099 |
| M+H | 726.2322 | 15.608 | 0100 |
| M+H | 726.2419 | 13.402 | 0101 |
| M+H | 726.2726 | 16.289 | 0102,0103 |
| M+H | 726.2756 | 12.869 | 0102,0103 |
| M+H | 727.2593 | 15.118 | 0104 |
| M+H | 728.2536 | 15.18 | 0105 |
| M+H | 729.2511 | 7.816 | 0106 |
| M+H | 729.3098 | 15.745 | 0107 |
| M+H | 730.2505 | 11.862 | 0108 |
| M+H | 730.2691 | 13.256 | 0109 |
| M+H | 730.3066 | 13.973 | 0110 |
| M+H | 731.1906 | 15.115 | 0111 |
| M+H | 731.2337 | 15.461 | 0112 |
| M+H | 731.2642 | 13.547 | 0113,0114 |
| M+H | 731.2643 | 12.498 | 0113,0114 |
| M+H | 732.2597 | 9.659 | 0115 |
| M+H | 733.2145 | 14.565 | 0116,0117,0118 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 733.2136 | 15.942 | 0116,0117,0118 |
| M+H | 733.213 | 15.994 | 0116,0117,0118 |
| M+H | 733.2276 | 15.503 | 0119,0120 |
| M+H | 733.2318 | 15.847 | 0119,0120 |
| M+H | 734.2103 | 12.88 | 0121,0122,0123 |
| M+H | 734.2097 | 14.361 | 0121,0122,0123 |
| M+H | 734.2102 | 14.311 | 0121,0122,0123 |
| M+H | 734.2271 | 8.935 | 0124 |
| M+H | 734.2432 | 12.935 | 0125,0126 |
| M-H | 732.2267 | 13.162 | 0125,0126 |
| M+H | 734.2631 | 14.054 | 0127 |
| M+H | 735.2048 | 13.777 | 0128,0129 |
| M+H | 735.2052 | 12.648 | 0128,0129 |
| M+H | 735.2385 | 13.224 | 0130,0131 |
| M+H | 735.2379 | 10.273 | 0130,0131 |
| M+H | 735.2764 | 13.57 | 0132 |
| M+H | 736.2 | 15.071 | 0133 |
| M+H | 736.2251 | 13.396 | 0134 |
| M+H | 736.2429 | 11.037 | 0135 |
| M+H | 737.2076 | 13.991 | 0136 |
| M+H | 737.2392 | 17.193 | 0137,0138,0139 |
| M+H | 737.241 | 16.642 | 0137,0138,0139 |
| M+H | 737.2394 | 17.128 | 0137,0138,0139 |
| M+H | 738.2363 | 15.189 | 0140,0141 |
| M+H | 738.235 | 16.812 | 0140,0141 |
| M+H | 739.1726 | 11.423 | 0142 |
| M+H | 739.1844 | 15.781 | 0143 |
| M+H | 739.2314 | 17.784 | 0144 |
| M+H | 739.2393 | 17.858 | 0145 |
| M+H | 739.2499 | 15.938 | 0146 |
| M+H | 739.2612 | 13.458 | 0147 |
| M+H | 740.1669 | 14.56 | 0148,0149 |
| M+H | 740.1665 | 1.282 | 0148,0149 |
| M+H | 740.2 | 13.092 | 0150 |
| M+H | 740.2647 | 12.964 | 0151 |
| M+H | 740.2904 | 15.995 | 0152 |
| M-H | 739.1435 | 14.897 | 0153 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M-H | 739.1654 | 12.745 | 0154 |
| M+H | 741.2595 | 10 | 0155 |
| M+H | 742.2009 | 13.616 | 0156 |
| M+H | 742.2128 | 13.735 | 0157 |
| M+H | 742.2686 | 14.601 | 0158 |
| M+H | 743.1947 | 10.777 | 0159 |
| M+H | 743.2155 | 17.392 | 0160 |
| M-H | 741.2238 | 8.754 | 0161 |
| M+H | 743.2687 | 12.967 | 0162 |
| M+H | 743.3238 | 8.974 | 0163 |
| M+H | 744.1909 | 17.591 | 0164 |
| M+H | 744.2321 | 14.976 | 0165 |
| M+H | 744.2515 | 12.838 | 0166 |
| M+H | 744.2844 | 13.743 | 0167 |
| M-H | 743.2078 | 14.89 | 0168,0169 |
| M+H | 745.2255 | 12.274 | 0168,0169 |
| M+H | 745.247 | 9.952 | 0170,0171,0172 |
| M+H | 745.2471 | 12.628 | 0170,0171,0172 |
| M+H | 745.2514 | 12.794 | 0170,0171,0172 |
| M+H | 745.2808 | 12.858 | 0173,0174 |
| M+H | 745.28 | 11.706 | 0173,0174 |
| M+H | 746.2213 | 14.297 | 0175 |
| M+H | 746.2481 | 12.832 | 0176 |
| M+H | 746.2631 | 10.8 | 0177 |
| M+H | 747.2169 | 15.702 | 0178 |
| M+H | 747.2282 | 16.467 | 0179,0180,0181 |
| M-H | 745.2114 | 14.951 | 0179,0180,0181 |
| M-H | 745.2118 | 15.082 | 0179,0180,0181 |
| M+H | 747.2576 | 10.921 | 0182 |
| M+H | 747.2643 | 13.042 | 0182,0183 |
| M+H | 747.3014 | 15.749 | 0184 |
| M+H | 748.2229 | 13.231 | 0185,0186,0187 |
| M+H | 748.2238 | 13.764 | 0185,0186,0187 |
| M+H | 748.2266 | 16.116 | 0185,0186,0187 |
| M+H | 748.2402 | 15.291 | 0188 |
| M+H | 748.2559 | 13.179 | 0189,0190 |
| M+H | 748.259 | 16.243 | 0189,0190 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 748.3149 | 13.254 | 0191 |
| M+H | 749.2187 | 17.031 | 0192,0193 |
| M+H | 749.2219 | 12.09 | 0192,0193 |
| M+H | 749.2549 | 12.579 | 0194 |
| M+H | 749.2893 | 17.002 | 0195 |
| M+H | 749.311 | 10.36 | 0196 |
| M+H | 750.2413 | 13.83 | 0197 |
| M+H | 751.1907 | 13.391 | 0198 |
| M+H | 751.2044 | 15.993 | 0199,0200 |
| M+H | 751.2047 | 14.603 | 0199,0200 |
| M+H | 751.2199 | 17.288 | 0201 |
| M+H | 751.226 | 14.408 | 0202 |
| M+H | 751.2531 | 17.015 | 0203 |
| M+H | 751.2699 | 16.027 | 0204 |
| M+H | 752.1772 | 15.288 | 0205 |
| M+H | 752.1839 | 13.261 | 0206 |
| M+H | 752.1994 | 12.941 | 0207 |
| M+H | 752.2199 | 12.461 | 0208,0209,0210 |
| M+H | 752.2206 | 13.558 | 0208,0209,0210 |
| M-H | 750.2026 | 13.768 | 0208,0209,0210 |
| M-H | 750.2155 | 13.51 | 0211,0212 |
| M+H | 752.2314 | 13.464 | 0211,0212 |
| M+H | 753.1866 | 13.344 | 0213 |
| M-H | 751.177 | 16.661 | 0214 |
| M+H | 753.2053 | 15.509 | 0215 |
| M+H | 753.2155 | 12.854 | 0216,0217,0218,0219 |
| M+H | 753.2156 | 10.945 | 0216,0217,0218,0219 |
| M+H | 753.2157 | 9.499 | 0216,0217,0218,0219 |
| M+H | 753.2217 | 12.382 | 0219 |
| M-H | 751.2161 | 14.077 | 0220,0221 |
| M+H | 753.2341 | 17.282 | 0220,0221 |
| M+H | 753.256 | 18.163 | 0222 |
| M+H | 753.2745 | 14.631 | 0223 |
| M-H | 752.1629 | 16.92 | 0224 |
| M+H | 754.211 | 9.72 | 0225,0226 |
| M+H | 754.2101 | 9.161 | 0225,0226 |
| M+H | 754.2292 | 16.89 | 0227 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 754.2801 | 13.4 | 0228 |
| M+H | 755.2065 | 11.043 | 0229 |
| M+H | 755.2141 | 15.983 | 0230 |
| M+H | 755.2288 | 16.649 | 0231,0232 |
| M+H | 755.2291 | 16.595 | 0231,0232 |
| M+H | 755.2346 | 17.402 | 0232,0233 |
| M+H | 755.2543 | 8.808 | 0234 |
| M+H | 756.2161 | 14.036 | 0235 |
| M+H | 756.246 | 14.401 | 0236,0237 |
| M+H | 756.2448 | 15.064 | 0236,0237 |
| M+H | 757.1639 | 15.011 | 0238 |
| M+H | 757.2408 | 14.293 | 0239,0240,0241 |
| M+H | 757.2409 | 11.787 | 0239,0240,0241 |
| M+H | 757.2407 | 14.247 | 0239,0240,0241 |
| M+H | 757.3441 | 12.71 | 0242 |
| M+H | 758.1573 | 16.488 | 0243 |
| M+H | 758.1761 | 13.96 | 0244 |
| M+H | 758.1904 | 13.7 | 0245 |
| M+H | 758.2359 | 15.72 | 0246 |
| M+H | 758.246 | 11.268 | 0247 |
| M+H | 758.2549 | 13.073 | 0248 |
| M+H | 758.2662 | 13.269 | 0249 |
| M+H | 759.172 | 11.319 | 0250,0251,0252 |
| M-H | 757.1544 | 11.435 | 0250,0251 |
| M+H | 759.1719 | 11.139 | 0250,0251,0252 |
| M+H | 759.2644 | 15.997 | 0253,0254 |
| M+H | 759.2658 | 17.134 | 0253,0254 |
| M+H | 759.295 | 13.313 | 0255 |
| M+H | 759.3241 | 18.806 | 0256 |
| M-H | 758.1505 | 11.007 | 0257 |
| M+H | 760.186 | 17.666 | 0258,0259 |
| M-H | 758.1729 | 13.779 | 0258,0259 |
| M+H | 760.2606 | 15.819 | 0260 |
| M-H | 759.1903 | 14.738 | 0261,0262 |
| M+H | 761.2092 | 14.599 | 0261,0262 |
| M+H | 761.2237 | 12.603 | 0263 |
| M+H | 761.2462 | 16.826 | 0264,0265,0266,0267 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 761.2462 | 16.88 | 0264,0265,0266,0267 |
| M+H | 761.2491 | 15.507 | 0264,0265,0266,0267 |
| M+H | 761.2455 | 15.579 | 0264,0265,0266,0267 |
| M+H | 761.2747 | 10.984 | 0268 |
| M+H | 762.2054 | 12.964 | 0269,0270 |
| M-H | 760.1864 | 13.032 | 0269,0270 |
| M+H | 762.2202 | 15.008 | 0271 |
| M+H | 762.2407 | 14.138 | 0272,0273,0274,0275 |
| M+H | 762.2423 | 16.566 | 0272,0273,0274,0275 |
| M+H | 762.2407 | 14.093 | 0272,0273,0274,0275 |
| M+H | 762.2413 | 12.855 | 0272,0273,0274,0275 |
| M+H | 762.2752 | 13.262 | 0276,0277 |
| M+H | 762.2741 | 13.312 | 0276,0277 |
| M+H | 762.3307 | 13.651 | 0278 |
| M+H | 763.1996 | 13.598 | 0279,0280,0281 |
| M+H | 763.2015 | 12.967 | 0279,0280,0281 |
| M+H | 763.2004 | 12.931 | 0279,0280,0281 |
| M+H | 763.2238 | 15.939 | 0282,0283 |
| M+H | 763.224 | 14.201 | 0282,0283 |
| M+H | 763.235 | 17.392 | 0284 |
| M+H | 763.2399 | 15.541 | 0284,0285 |
| M+H | 764.2199 | 12.423 | 0286 |
| M+H | 764.256 | 13.648 | 0287,0288 |
| M+H | 764.258 | 14.407 | 0287,0288 |
| M+H | 765.2058 | 15.763 | 0289 |
| M-H | 763.2024 | 15.032 | 0290,0291,0292 |
| M-H | 763.2024 | 14.983 | 0290,0291,0292 |
| M+H | 765.2205 | 15.179 | 0290,0291,0292 |
| M+H | 765.233 | 15.941 | 0293 |
| M-H | 763.2371 | 12.152 | 0294,0295 |
| M-H | 763.2371 | 12.127 | 0294,0295 |
| M+H | 765.2723 | 17.39 | 0296 |
| M+H | 765.289 | 16.008 | 0297 |
| M+H | 766.2191 | 10.741 | 0298,0299 |
| M+H | 766.2188 | 10.513 | 0298,0299 |
| M-H | 764.2174 | 14.148 | 0300,0301,0302,0303 |
| M+H | 766.2357 | 12.71 | 0301,0302,0303 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 766.2358 | 13.939 | 0301,0302,0303 |
| M-H | 764.2189 | 12.873 | 0300,0301,0302,0303 |
| M+H | 766.3054 | 13.323 | 0304 |
| M+H | 767.1681 | 15.777 | 0305 |
| M+H | 767.2002 | 14.227 | 0306 |
| M+H | 767.2305 | 13.262 | 0307,0308,0309 |
| M+H | 767.231 | 9.749 | 0307,0308,0309 |
| M+H | 767.2311 | 12.567 | 0307,0308,0309 |
| M+H | 767.2443 | 9.315 | 0310 |
| M+H | 767.2519 | 16.484 | 0311 |
| M+H | 768.161 | 13.922 | 0312 |
| M+H | 768.1944 | 10.702 | 0313 |
| M+H | 768.2275 | 14.494 | 0314,0315 |
| M+H | 768.2275 | 14.559 | 0314,0315 |
| M+H | 768.2963 | 13.932 | 0316 |
| M-H | 767.1605 | 15.294 | 0317 |
| M+H | 769.1801 | 15.3 | 0317,0318 |
| M+H | 769.1946 | 14.92 | 0319,0320 |
| M+H | 769.1968 | 16.239 | 0319,0320 |
| M+H | 770.1761 | 12.883 | 0321 |
| M+H | 770.1892 | 15.887 | 0322 |
| M+H | 770.1953 | 13.021 | 0322,0323 |
| M+H | 770.21 | 13.815 | 0324,0325 |
| M+H | 770.2103 | 12.546 | 0324,0325 |
| M-H | 768.2068 | 10.261 | 0326 |
| M+H | 770.2311 | 14.406 | 0327 |
| M+H | 770.2612 | 14.765 | 0328 |
| M+H | 770.2756 | 10.988 | 0329 |
| M-H | 769.1681 | 16.824 | 0330 |
| M-H | 769.1713 | 16.278 | 0330,0331,0332 |
| M-H | 769.1727 | 9.673 | 0330,0331,0332 |
| M+H | 771.2056 | 9.801 | 0333,0334 |
| M+H | 771.2059 | 12.958 | 0333,0334 |
| M+H | 771.2735 | 17.057 | 0335 |
| M-H | 770.1756 | 14.322 | 0336 |
| M+H | 772.199 | 14.195 | 0337 |
| M+H | 772.2091 | 11.771 | 0338 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 772.2398 | 15.106 | 0339 |
| M+H | 772.2623 | 11.403 | 0340 |
| M+H | 772.2821 | 13.76 | 0341 |
| M+H | 773.1909 | 14.349 | 0342 |
| M-H | 771.1951 | 11.778 | 0343 |
| M+H | 773.2207 | 16.825 | 0344,0345 |
| M+H | 773.2208 | 13.471 | 0344,0345 |
| M+H | 773.2359 | 14.312 | 0346 |
| M+H | 773.2788 | 11.558 | 0347,0348,0349 |
| M+H | 773.2823 | 17.794 | 0347,0348,0349 |
| M+H | 773.2808 | 17.469 | 0347,0348,0349 |
| M+H | 774.2151 | 13.633 | 0350 |
| M-H | 772.2011 | 14.085 | 0350,0351 |
| M+H | 774.232 | 15.78 | 0352,0353 |
| M+H | 774.2388 | 14.408 | 0353,0354 |
| M+H | 774.2388 | 14.459 | 0353,0354 |
| M+H | 774.2768 | 11.346 | 0356 |
| M-H | 773.1338 | 16.281 | 0357 |
| M+H | 775.2227 | 14.046 | 0358 |
| M+H | 775.2391 | 18.217 | 0359 |
| M+H | 775.2634 | 12.855 | 0360,0361,0362,0363 |
| M+H | 775.2586 | 13.703 | 0360,0361,0362,0363 |
| M+H | 775.2592 | 15.943 | 0360,0361,0362,0363 |
| M+H | 775.2604 | 15.876 | 0360,0361,0362,0363 |
| M+H | 776.1458 | 16.602 | 0364 |
| M+H | 776.1661 | 14.017 | 0365 |
| M+H | 776.2209 | 14.781 | 0366,0367 |
| M+H | 776.2209 | 14.71 | 0366,0367 |
| M+H | 776.2455 | 13.803 | 0368 |
| M+H | 776.2551 | 14.526 | 0369 |
| M+H | 776.3461 | 14.123 | 0370 |
| M+H | 777.1661 | 13.947 | 0371 |
| M+H | 777.2146 | 13.421 | 0372 |
| M+H | 777.2418 | 15.302 | 0373,0374 |
| M+H | 777.2418 | 15.338 | 0373,0374 |
| M+H | 777.2569 | 12.229 | 0375 |
| M+H | 778.1815 | 11.794 | 0376 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M-H | 776.2171 | 11.647 | 0377,0378 |
| M+H | 778.2357 | 11.887 | 0377,0378 |
| M+H | 778.2691 | 13.603 | 0379,0380 |
| M+H | 778.2721 | 14.12 | 0379,0380 |
| M+H | 778.3267 | 11.154 | 0381 |
| M+H | 779.1766 | 13.688 | 0382 |
| M+H | 779.195 | 10.56 | 0383 |
| M+H | 779.1989 | 14.827 | 0384,0385 |
| M+H | 779.1992 | 14.762 | 0384,0385 |
| M+H | 779.2203 | 16.186 | 0386 |
| M+H | 779.2349 | 14.471 | 0387,0388 |
| M+H | 779.238 | 15.648 | 0387,0388 |
| M-H | 777.255 | 10.801 | 0389 |
| M+H | 780.1945 | 13.745 | 0391,0392 |
| M+H | 780.1935 | 14.66 | 0391,0392 |
| M-H | 778.1986 | 12.676 | 0393,0394 |
| M+H | 780.2156 | 13.453 | 0393,0394 |
| M+H | 780.2308 | 14.129 | 0395 |
| M-H | 778.2325 | 14.573 | 0396,0397,0398,0399,0400 |
| M+H | 780.251 | 13.076 | 0396,0397,0398,0399,0400 |
| M+H | 780.2509 | 13.349 | 0396,0397,0398,0399,0400 |
| M+H | 780.2521 | 14.571 | 0397,0398,0399,0400 |
| M+H | 780.2535 | 13.843 | 0397,0398,0399,0400 |
| M-H | 779.1639 | 16.307 | 0401 |
| M+H | 781.1983 | 13.665 | 0402 |
| M+H | 781.21 | 9.69 | 0403,0404 |
| M-H | 779.194 | 12.819 | 0403,0404 |
| M-H | 779.2109 | 16.006 | 0405 |
| M+H | 781.229 | 11.526 | 0405,0406 |
| M+H | 781.2465 | 13.796 | 0407,0408,0409 |
| M+H | 781.2468 | 14.074 | 0407,0408,0409 |
| M+H | 781.2468 | 13.892 | 0407,0408,0409 |
| M+H | 782.2051 | 9.925 | 0410 |
| M+H | 782.2244 | 16.125 | 0411 |
| M+H | 782.2304 | 12.457 | 0411,0412,0413 |
| M-H | 780.2142 | 12.494 | 0411,0412,0413 |
| M+H | 782.2416 | 11.612 | 0414 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 782.2496 | 13.736 | 0415 |
| M+H | 783.21 | 15.266 | 0416,0417 |
| M-H | 781.1951 | 12.836 | 0416,0417 |
| M+H | 783.2258 | 9.477 | 0418,0419,0420,0421 |
| M+H | 783.2259 | 10.96 | 0418,0419,0420,0421 |
| M+H | 783.2272 | 16.85 | 0418,0419,0420,0421 |
| M+H | 783.2313 | 12.809 | 0419,0420,0421 |
| M-H | 782.1923 | 13.453 | 0422,0423 |
| M-H | 782.1926 | 13.375 | 0422,0423 |
| M-H | 782.2084 | 13.211 | 0424,0425,0426,0427 |
| M+H | 784.2264 | 13.183 | 0424,0425,0426,0427 |
| M+H | 784.2257 | 12.783 | 0424,0425,0426,0427 |
| M+H | 784.2262 | 15.366 | 0424,0425,0426,0427 |
| M-H | 782.2222 | 13.86 | 0428 |
| M+H | 784.2771 | 15.2 | 0429 |
| M+H | 784.2955 | 13.97 | 0430 |
| M+H | 785.1547 | 15.903 | 0431 |
| M+H | 785.221 | 17.396 | 0432,0433,0434,0435 |
| M+H | 785.2243 | 11.51 | 0432,0433,0434,0435 |
| M+H | 785.2208 | 7.838 | 0432,0433,0434,0435 |
| M+H | 785.2202 | 15.419 | 0432,0433,0434,0435 |
| M-H | 783.2175 | 14.093 | 0436 |
| M+H | 786.1713 | 13.207 | 0437 |
| M+H | 786.2071 | 14.751 | 0438 |
| M+H | 786.2156 | 13.338 | 0439 |
| M+H | 786.2564 | 12.682 | 0440 |
| M+H | 787.166 | 9.826 | 0442,0443 |
| M-H | 785.1523 | 15.532 | 0442,0443 |
| M+H | 787.1849 | 15.019 | 0444 |
| M+H | 787.2009 | 10.638 | 0445 |
| M+H | 787.2592 | 15.023 | 0447 |
| M+H | 787.3016 | 13.497 | 0448,0449 |
| M+H | 787.2978 | 17.802 | 0448,0449 |
| M+H | 788.1502 | 13.879 | 0450 |
| M+H | 788.1869 | 12.091 | 0451,0452 |
| M-H | 786.1685 | 13.121 | 0451,0452 |
| M+H | 788.2011 | 12.861 | 0453,0454 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 788.2005 | 14.196 | 0453,0454 |
| M+H | 788.217 | 17.036 | 0455 |
| M+H | 788.2318 | 13.14 | 0456 |
| M+H | 789.1824 | 12.25 | 0457,0458,0459,0460 |
| M+H | 789.1847 | 16.94 | 0457,0458,0459,0460 |
| M-H | 787.1682 | 16.892 | 0457,0458,0459,0460 |
| M-H | 787.1751 | 10.948 | 0458,0459,0460 |
| M+H | 789.1961 | 13.617 | 0461 |
| M+H | 789.239 | 15.803 | 0462 |
| M+H | 789.2447 | 16.628 | 0462,0463 |
| M+H | 789.2765 | 17.045 | 0464,0465 |
| M+H | 789.2765 | 17.008 | 0464,0465 |
| M+H | 790.1836 | 15.477 | 0466,0467,0468 |
| M+H | 790.1844 | 15.615 | 0466,0467,0468 |
| M+H | 790.1844 | 15.547 | 0466,0467,0468 |
| M+H | 790.1914 | 14.679 | 0469 |
| M+H | 790.1961 | 14.019 | 0469,0470 |
| M+H | 790.2138 | 14.128 | 0471 |
| M+H | 790.2393 | 13.058 | 0472,0473 |
| M+H | 790.2354 | 9.956 | 0472,0473 |
| M-H | 789.1654 | 15.48 | 0474 |
| M+H | 791.2156 | 11.055 | 0475,0476 |
| M-H | 789.208 | 9.607 | 0476 |
| M-H | 789.2372 | 17.591 | 0477,0478 |
| M+H | 791.255 | 15.444 | 0477,0478 |
| M+H | 791.272 | 17.394 | 0479 |
| M+H | 792.2136 | 12.625 | 0480 |
| M+H | 792.2259 | 14.991 | 0481 |
| M+H | 792.287 | 10.848 | 0482,0483,0484 |
| M+H | 792.2894 | 14.93 | 0482,0483,0484 |
| M+H | 792.2879 | 15.254 | 0482,0483,0484 |
| M+H | 793.2101 | 10.91 | 0485 |
| M+H | 793.2179 | 14.927 | 0486 |
| M+H | 793.2324 | 13.812 | 0487 |
| M+H | 793.2498 | 13.95 | 0488 |
| M-H | 791.2542 | 15.056 | 0489 |
| M+H | 793.2831 | 8 | 0490 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 794.1562 | 14.521 | 0491 |
| M+H | 794.2101 | 14.884 | 0492 |
| M-H | 792.2138 | 13.069 | 0493 |
| M+H | 794.267 | 13.4 | 0494,0495,0496,0497 |
| M+H | 794.2667 | 13.589 | 0494,0495,0496,0497 |
| M+H | 794.2648 | 11.049 | 0494,0495,0496,0497 |
| M+H | 794.2653 | 14.318 | 0494,0495,0496,0497 |
| M-H | 793.1373 | 14.468 | 0498 |
| M+H | 795.1948 | 16.846 | 0499 |
| M+H | 795.2251 | 12.423 | 0500,0501 |
| M-H | 793.2096 | 13.206 | 0500,0501 |
| M+H | 795.2281 | 16.191 | 0500,0501,0502,0503 |
| M+H | 795.2281 | 16.147 | 0500,0501,0502,0503 |
| M+H | 795.2469 | 17.381 | 0504 |
| M+H | 795.2625 | 11.344 | 0505,0506 |
| M+H | 795.2673 | 13.764 | 0505,0506 |
| M+H | 796.2207 | 9.573 | 0507 |
| M+H | 796.2393 | 15.937 | 0508 |
| M+H | 796.246 | 12.906 | 0508,0509,0510 |
| M+H | 796.2441 | 11.574 | 0508,0509,0510 |
| M+H | 796.2595 | 14.191 | 0511 |
| M-H | 795.1577 | 15.878 | 0512,0513 |
| M-H | 795.1577 | 15.761 | 0512,0513 |
| M+H | 797.1893 | 14.884 | 0514,0515 |
| M+H | 797.1891 | 15.068 | 0514,0515 |
| M+H | 797.227 | 13.992 | 0516 |
| M+H | 797.2317 | 13.93 | 0516,0517 |
| M-H | 795.2286 | 12.947 | 0518,0519,0520 |
| M+H | 797.2423 | 15.984 | 0518,0519,0520 |
| M-H | 795.2316 | 12.911 | 0520 |
| M-H | 795.2785 | 16.369 | 0521 |
| M+H | 798.1711 | 14.205 | 0522 |
| M+H | 798.1866 | 14.93 | 0523 |
| M-H | 796.189 | 12.764 | 0524,0525 |
| M+H | 798.2053 | 12.639 | 0524,0525 |
| M-H | 796.2082 | 13.946 | 0526 |
| M+H | 798.2382 | 13.168 | 0527,0528,0529 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M-H | 796.2234 | 13.688 | 0527,0528,0529 |
| M-H | 796.2234 | 13.65 | 0527,0528,0529 |
| M+H | 799.1663 | 14.111 | 0530 |
| M+H | 799.2005 | 9.931 | 0531,0532 |
| M+H | 799.2006 | 12.902 | 0531,0532 |
| M+H | 799.2206 | 11.585 | 0533 |
| M+H | 799.2382 | 10.821 | 0534 |
| M+H | 799.2529 | 13.51 | 0535 |
| M+H | 800.1861 | 13.687 | 0536 |
| M-H | 798.1889 | 11.268 | 0537 |
| M+H | 800.2358 | 13.916 | 0538,0539 |
| M-H | 798.2252 | 13.694 | 0538,0539 |
| M+H | 801.2003 | 16.187 | 0541,0542,0543,0544,0545,0546 |
| M+H | 801.2023 | 15.374 | 0541,0542,0543,0544,0545,0546 |
| M+H | 801.2023 | 15.324 | 0541,0542,0543,0544,0545,0546 |
| M-H | 799.1827 | 15.394 | 0541,0542,0543,0544,0545 |
| M+H | 801.2032 | 16.557 | 0541,0542,0543,0544,0545,0546 |
| M+H | 801.2032 | 16.51 | 0541,0542,0543,0544,0545,0546 |
| M+H | 801.2189 | 13.468 | 0547 |
| M+H | 801.2307 | 14.19 | 0548 |
| M+H | 801.2745 | 13.188 | 0550 |
| M+H | 801.3109 | 18.072 | 0551 |
| M+H | 802.1974 | 14.66 | 0552,0553 |
| M+H | 802.1974 | 14.706 | 0552,0553 |
| M+H | 802.2164 | 13.345 | 0554,0555 |
| M+H | 802.2144 | 11.973 | 0554,0555 |
| M+H | 802.2331 | 11.699 | 0556 |
| M+H | 802.2731 | 12.231 | 0557 |
| M-H | 801.1285 | 15.95 | 0559 |
| M+H | 803.1901 | 17.15 | 0560 |
| M+H | 803.2 | 17.284 | 0561,0562 |
| M+H | 803.1994 | 17.36 | 0561,0562 |
| M+H | 803.2129 | 8.284 | 0563,0564 |
| M+H | 803.2128 | 10.472 | 0563,0564 |
| M+H | 803.2909 | 12.757 | 0565 |
| M+H | 804.1292 | 16.268 | 0566 |
| M+H | 804.1614 | 13.353 | 0567 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 804.1953 | 10.865 | 0568 |
| M+H | 804.2263 | 12.412 | 0569 |
| M+H | 804.2519 | 15.83 | 0570 |
| M-H | 803.1337 | 12.438 | 0571 |
| M+H | 805.1915 | 14.783 | 0572 |
| M-H | 803.1998 | 12.858 | 0573 |
| M+H | 805.2212 | 9.22 | 0574 |
| M+H | 805.2273 | 17.044 | 0575 |
| M+H | 805.2419 | 16.744 | 0576 |
| M+H | 805.2724 | 12.272 | 0577 |
| M+H | 805.2834 | 13.943 | 0578 |
| M-H | 804.1452 | 13.667 | 0579 |
| M+H | 806.176 | 13.199 | 0580 |
| M+H | 806.191 | 14.05 | 0581 |
| M+H | 806.2332 | 14.643 | 0582,0583 |
| M+H | 806.2332 | 14.793 | 0582,0583 |
| M+H | 806.303 | 11.305 | 0584,0585 |
| M+H | 806.3004 | 14.042 | 0584,0585 |
| M+H | 807.1556 | 10.504 | 0586,0587 |
| M-H | 805.1396 | 16.602 | 0586,0587 |
| M+H | 807.1758 | 16.881 | 0588,0589,0590 |
| M+H | 807.175 | 16.926 | 0588,0589,0590 |
| M-H | 805.159 | 16.822 | 0588,0589,0590 |
| M+H | 807.2261 | 12.046 | 0591 |
| M-H | 805.2139 | 15.74 | 0591,0592 |
| M+H | 807.2551 | 18.45 | 0593 |
| M+H | 807.2853 | 12.925 | 0594 |
| M+H | 808.1536 | 16.935 | 0595 |
| M+H | 808.1924 | 14.489 | 0596,0597 |
| M+H | 808.1931 | 14.333 | 0596,0597 |
| M+H | 808.2456 | 13.569 | 0598 |
| M+H | 808.2509 | 14.087 | 0598,0599 |
| M+H | 808.2839 | 14.558 | 0600,0601 |
| M+H | 808.2824 | 14.365 | 0600,0601 |
| M+H | 809.1873 | 12.089 | 0602,0603,0604,0605 |
| M+H | 809.1872 | 13.095 | 0602,0603,0604,0605 |
| M+H | 809.1867 | 14.285 | 0602,0603,0604,0605 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 809.1873 | 11.521 | 0602,0603,0604,0605 |
| M+H | 809.2054 | 17.883 | 0606 |
| M-H | 807.1913 | 14.866 | 0606,0607 |
| M+H | 809.2316 | 13.185 | 0608 |
| M+H | 809.2417 | 12.839 | 0609,0610 |
| M+H | 809.2407 | 11.506 | 0609,0610 |
| M+H | 809.2599 | 16.569 | 0611 |
| M+H | 809.2812 | 17.85 | 0612 |
| M+H | 810.1822 | 13.804 | 0613 |
| M+H | 810.1903 | 14.349 | 0614 |
| M-H | 808.2092 | 12.523 | 0615 |
| M+H | 810.2613 | 13.39 | 0616,0617 |
| M+H | 810.262 | 11.063 | 0616,0617 |
| M+H | 810.2783 | 10.991 | 0618 |
| M+H | 811.1901 | 14.198 | 0620 |
| M+H | 811.2204 | 10.155 | 0621 |
| M+H | 811.2249 | 15.707 | 0621,0622 |
| M+H | 811.2581 | 10.48 | 0623,0624 |
| M+H | 811.2614 | 15.093 | 0623,0624 |
| M+H | 812.2157 | 7.168 | 0626,0627 |
| M+H | 812.2213 | 12.544 | 0626,0627,0628 |
| M-H | 810.2031 | 13.057 | 0626,0627,0628 |
| M+H | 812.2378 | 13.792 | 0629 |
| M-H | 810.2444 | 13.456 | 0630 |
| M+H | 812.2772 | 12.764 | 0631 |
| M+H | 813.2166 | 12.503 | 0632,0633 |
| M+H | 813.2159 | 7.992 | 0632,0633 |
| M+H | 813.223 | 15.719 | 0634 |
| M+H | 813.2369 | 17.537 | 0635 |
| M+H | 813.2506 | 16.657 | 0636 |
| M+H | 813.2726 | 10.047 | 0637 |
| M+H | 813.292 | 16.343 | 0638 |
| M-H | 812.1856 | 13.314 | 0639 |
| M+H | 814.2369 | 11.67 | 0640,0641 |
| M+H | 814.2373 | 11.783 | 0640,0641 |
| M+H | 814.2552 | 16.562 | 0642 |
| M+H | 815.1441 | 14.467 | 0643 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 815.1687 | 11.934 | 0644 |
| M-H | 813.163 | 15.099 | 0645 |
| M+H | 815.2151 | 16.548 | 0647,0648 |
| M+H | 815.2151 | 16.606 | 0647,0648 |
| M+H | 815.2288 | 12.571 | 0649 |
| M+H | 815.2472 | 13.568 | 0651 |
| M+H | 815.2546 | 13.191 | 0652 |
| M+H | 815.2924 | 17.383 | 0653 |
| M-H | 814.1222 | 14.534 | 0654 |
| M+H | 816.1812 | 13.741 | 0655,0656 |
| M-H | 814.1641 | 14.028 | 0655,0656 |
| M+H | 816.1956 | 12.711 | 0657,0658 |
| M+H | 816.197 | 13.316 | 0657,0658 |
| M+H | 816.2151 | 10.556 | 0659 |
| M+H | 816.2314 | 13.978 | 0660 |
| M+H | 816.3019 | 14.239 | 0661 |
| M-H | 815.1294 | 18.224 | 0662 |
| M+H | 817.1767 | 10.655 | 0664 |
| M+H | 817.1798 | 12.95 | 0664,0665 |
| M+H | 817.1906 | 13.409 | 0666 |
| M+H | 817.1958 | 15.499 | 0666,0667,0668 |
| M+H | 817.1949 | 16.607 | 0666,0667,0668 |
| M+H | 817.2163 | 17.679 | 0669,0670 |
| M+H | 817.2156 | 17.608 | 0669,0670 |
| M+H | 817.2357 | 12.816 | 0671 |
| M+H | 818.1928 | 16.384 | 0672 |
| M-H | 816.1957 | 13.139 | 0673 |
| M+H | 818.23 | 14.05 | 0674 |
| M+H | 818.2426 | 12.879 | 0675 |
| M+H | 819.1768 | 16.027 | 0676 |
| M-H | 817.1738 | 15.444 | 0677,0678 |
| M+H | 819.1915 | 16.093 | 0677,0678 |
| M+H | 819.1975 | 14.53 | 0678,0679,0680 |
| M+H | 819.194 | 15.431 | 0678,0679,0680 |
| M+H | 819.2679 | 16.961 | 0681 |
| M-H | 818.1593 | 14.084 | 0682 |
| M+H | 820.2085 | 14.466 | 0683,0684,0685,0686 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 820.2085 | 14.6 | 0683,0684,0685,0686 |
| M+H | 820.2068 | 13.487 | 0683,0684,0685,0686 |
| M+H | 820.2072 | 13.893 | 0683,0684,0685,0686 |
| M-H | 818.2284 | 14.433 | 0687 |
| M+H | 820.2808 | 12.069 | 0688 |
| M+H | 820.3189 | 11.893 | 0689 |
| M+H | 821.1275 | 13.218 | 0690 |
| M+H | 821.203 | 13.971 | 0691,0692,0693 |
| M+H | 821.2051 | 11.556 | 0691,0692,0693 |
| M+H | 821.2014 | 14.248 | 0691,0692,0693 |
| M+H | 821.2238 | 15.654 | 0694,0695 |
| M+H | 821.2232 | 17.188 | 0694,0695 |
| M+H | 821.278 | 11.506 | 0696 |
| M-H | 819.2852 | 13.874 | 0697 |
| M-H | 820.1198 | 15.437 | 0698 |
| M-H | 820.1344 | 13.818 | 0699 |
| M+H | 822.1879 | 12.233 | 0700 |
| M+H | 822.1974 | 15.022 | 0701 |
| M+H | 822.2088 | 14.804 | 0702,0703 |
| M-H | 820.189 | 14.838 | 0702,0703 |
| M+H | 822.2166 | 12.543 | 0704 |
| M+H | 822.2995 | 14.635 | 0705 |
| M-H | 821.1174 | 15.782 | 0706,0707 |
| M+H | 823.1335 | 11.062 | 0706,0707 |
| M+H | 823.1513 | 8.066 | 0708 |
| M-H | 821.1847 | 14.263 | 0709,0710 |
| M+H | 823.2033 | 15.167 | 0709,0710,0711 |
| M+H | 823.2038 | 13.31 | 0709,0710,0711 |
| M+H | 823.2576 | 13.336 | 0712 |
| M+H | 823.2961 | 18.135 | 0714 |
| M+H | 824.1523 | 13.832 | 0715,0716 |
| M-H | 822.1341 | 13.761 | 0715,0716 |
| M+H | 824.1988 | 11.56 | 0717,0718 |
| M+H | 824.1971 | 14.61 | 0717,0718 |
| M+H | 824.2207 | 14.655 | 0719 |
| M+H | 824.2316 | 15.181 | 0720 |
| M+H | 824.2464 | 14.153 | 0721 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 824.2773 | 13.821 | 0722 |
| M+H | 825.1648 | 17.14 | 0723,0724 |
| M+H | 825.1662 | 17.096 | 0723,0724 |
| M+H | 825.2362 | 10.658 | 0726 |
| M+H | 826.1622 | 14.79 | 0727 |
| M+H | 826.1788 | 10.804 | 0728,0729,0730 |
| M+H | 826.1828 | 14.481 | 0728,0729,0730 |
| M+H | 826.1815 | 14.523 | 0728,0729,0730 |
| M+H | 826.2373 | 13.49 | 0731,0732 |
| M-H | 824.2202 | 13.521 | 0731,0732 |
| M+H | 826.2556 | 11.438 | 0733 |
| M+H | 826.2927 | 13.179 | 0734 |
| M+H | 827.1607 | 13.648 | 0735,0736 |
| M+H | 827.1611 | 13.747 | 0735,0736 |
| M+H | 827.1852 | 14.602 | 0737 |
| M+H | 827.2538 | 13.843 | 0738,0739 |
| M+H | 827.2529 | 17.553 | 0738,0739 |
| M+H | 828.212 | 15.837 | 0740,0741 |
| M+H | 828.2157 | 10.957 | 0740,0741 |
| M+H | 828.2331 | 13.907 | 0742 |
| M+H | 828.2657 | 14.469 | 0743 |
| M-H | 827.1785 | 15.557 | 0744 |
| M-H | 827.1966 | 15.848 | 0745 |
| M-H | 827.2146 | 16.961 | 0746,0747,0748 |
| M+H | 829.2308 | 17.051 | 0746,0747,0748 |
| M+H | 829.2335 | 16.921 | 0746,0747,0748 |
| M+H | 829.2506 | 14.878 | 0749 |
| M+H | 829.3082 | 17.705 | 0750 |
| M-H | 828.179 | 14.124 | 0751,0752 |
| M+H | 830.199 | 14.153 | 0752 |
| M+H | 830.2681 | 12.851 | 0753 |
| M+H | 831.2064 | 13.129 | 0755 |
| M+H | 831.2115 | 14.994 | 0755,0756,0757 |
| M+H | 831.2122 | 14.914 | 0755,0756,0757 |
| M+H | 831.2844 | 16.571 | 0758 |
| M+H | 832.2049 | 12.204 | 0759 |
| M+H | 832.2268 | 18.555 | 0760,0761 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 832.2268 | 18.502 | 0760,0761 |
| M-H | 830.2279 | 11.255 | 0762 |
| M+H | 832.2576 | 13.421 | 0763 |
| M+H | 832.2974 | 14.287 | 0764 |
| M-H | 831.139 | 16.167 | 0766 |
| M+H | 833.2061 | 11.171 | 0767 |
| M-H | 832.1554 | 13.841 | 0768 |
| M+H | 834.1859 | 13.268 | 0769 |
| M-H | 832.1759 | 14.517 | 0769,0770 |
| M+H | 834.2062 | 16.218 | 0771 |
| M+H | 834.2231 | 14.174 | 0772,0773 |
| M+H | 834.2228 | 14.264 | 0772,0773 |
| M+H | 834.2369 | 10.542 | 0774 |
| M-H | 832.2443 | 15.413 | 0775 |
| M+H | 834.2987 | 14.283 | 0776 |
| M+H | 835.1545 | 16.601 | 0777 |
| M-H | 833.1522 | 16.129 | 0778 |
| M+H | 835.2203 | 10.745 | 0779 |
| M+H | 835.2358 | 16.711 | 0780,0781 |
| M+H | 835.2385 | 17.019 | 0780,0781 |
| M-H | 834.1367 | 15.904 | 0782 |
| M+H | 836.1716 | 14.58 | 0783 |
| M+H | 836.2008 | 14.691 | 0784,0785 |
| M+H | 836.2021 | 13.515 | 0784,0785 |
| M+H | 836.2244 | 15.17 | 0786,0787 |
| M+H | 836.223 | 15.236 | 0786,0787 |
| M+H | 836.2414 | 12.714 | 0788 |
| M-H | 835.1574 | 13.906 | 0789 |
| M+H | 837.1808 | 13.349 | 0790,0791 |
| M+H | 837.1829 | 13.215 | 0790,0791 |
| M+H | 837.1971 | 14.011 | 0792 |
| M+H | 837.2178 | 14.463 | 0793,0794 |
| M+H | 837.2175 | 14.505 | 0793,0794 |
| M+H | 837.2587 | 17.167 | 0796 |
| M-H | 836.1642 | 14.033 | 0797,0798 |
| M-H | 836.164 | 14.087 | 0797,0798 |
| M-H | 836.1793 | 13.756 | 0799,0800 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M-H | 836.1793 | 13.926 | 0799,0800 |
| M+H | 838.1993 | 13.74 | 0800,0801,0802 |
| M+H | 838.1993 | 13.68 | 0800,0801,0802 |
| M+H | 838.2718 | 14.631 | 0803 |
| M+H | 839.2003 | 17.707 | 0805,0806 |
| M-H | 837.1835 | 17.641 | 0805,0806 |
| M+H | 840.1293 | 15.812 | 0807 |
| M+H | 840.1755 | 16.458 | 0808 |
| M+H | 840.2081 | 13.372 | 0809 |
| M-H | 838.2148 | 11.66 | 0810 |
| M+H | 840.3069 | 13.65 | 0811 |
| M+H | 841.1922 | 14.281 | 0812,0813 |
| M+H | 841.1934 | 14.234 | 0812,0813 |
| M+H | 841.2684 | 17.851 | 0814 |
| M-H | 840.1237 | 14.38 | 0815 |
| M+H | 842.157 | 14.87 | 0816 |
| M-H | 840.1927 | 15.181 | 0817 |
| M+H | 842.2882 | 11.046 | 0818 |
| M+H | 843.1386 | 14.044 | 0819 |
| M+H | 843.1564 | 11.52 | 0820 |
| M+H | 843.2474 | 17.645 | 0821 |
| M+H | 843.2894 | 16.053 | 0822 |
| M-H | 842.1542 | 14.997 | 0823,0824 |
| M+H | 844.1728 | 14.963 | 0823,0824 |
| M+H | 844.2092 | 15.644 | 0825,0826 |
| M-H | 842.195 | 14.617 | 0825,0826 |
| M-H | 843.1729 | 15.689 | 0827 |
| M-H | 844.1679 | 15.525 | 0830 |
| M-H | 844.1775 | 19.696 | 0831 |
| M+H | 846.2617 | 10.718 | 0832 |
| M-H | 845.1713 | 16.825 | 0833 |
| M+H | 847.1884 | 15.572 | 0833,0834 |
| M-H | 846.1842 | 13.654 | 0835 |
| M+H | 848.2378 | 14.701 | 0836,0837,0838 |
| M+H | 848.2384 | 14.657 | 0836,0837,0838 |
| M+H | 848.2394 | 14.361 | 0836,0837,0838 |
| M+H | 848.2577 | 13.424 | 0839 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 848.3139 | 15.354 | 0840 |
| M-H | 847.1803 | 13.779 | 0841 |
| M-H | 847.1838 | 14.773 | 0841,0842 |
| M+H | 849.2555 | 16.313 | 0843 |
| M-H | 848.1507 | 11.847 | 0844 |
| M+H | 850.2165 | 13.734 | 0845,0846 |
| M+H | 850.218 | 13.454 | 0845,0846 |
| M+H | 850.2915 | 12.841 | 0847 |
| M-H | 849.1297 | 16.764 | 0848 |
| M-H | 849.1731 | 13.134 | 0849 |
| M-H | 849.1974 | 16.96 | 0850,0851 |
| M+H | 851.2178 | 16.935 | 0850,0851 |
| M+H | 851.2339 | 14.751 | 0852 |
| M+H | 851.2899 | 17.419 | 0853 |
| M-H | 850.1306 | 13.595 | 0854 |
| M-H | 850.1442 | 14.322 | 0855 |
| M+H | 852.1831 | 14.626 | 0856 |
| M+H | 852.1915 | 13.139 | 0857 |
| M+H | 853.1494 | 13.09 | 0859,0860 |
| M-H | 851.1295 | 17.376 | 0859,0860 |
| M+H | 853.2065 | 16.256 | 0861 |
| M+H | 853.2135 | 12.538 | 0862,0863 |
| M+H | 853.2128 | 13.869 | 0861,0862,0863 |
| M-H | 852.1251 | 15.974 | 0864 |
| M-H | 852.1418 | 14.44 | 0865 |
| M-H | 852.1587 | 14.15 | 0866 |
| M-H | 852.194 | 17.062 | 0867 |
| M+H | 855.1451 | 16.135 | 0868 |
| M+H | 855.1724 | 13.328 | 0869 |
| M+H | 855.2089 | 14.662 | 0870 |
| M+H | 856.1884 | 14.459 | 0871 |
| M+H | 856.2671 | 13.439 | 0872 |
| M+H | 857.1722 | 17.384 | 0873,0874 |
| M+H | 857.1737 | 17.326 | 0873,0874 |
| M+H | 857.263 | 17.883 | 0875 |
| M-H | 856.1019 | 16.756 | 0876 |
| M+H | 859.1826 | 14.783 | 0877 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 860.2004 | 13.754 | 0878 |
| M+H | 861.1931 | 13.371 | 0880 |
| M-H | 859.2408 | 15.793 | 0882 |
| M+H | 862.2533 | 13.528 | 0883 |
| M+H | 863.1268 | 14.183 | 0884 |
| M-H | 861.164 | 15.693 | 0885 |
| M+H | 863.197 | 15.168 | 0886 |
| M+H | 863.2128 | 13.512 | 0887 |
| M-H | 862.1785 | 13.775 | 0888 |
| M+H | 864.2341 | 12.85 | 0889,0890 |
| M+H | 864.2319 | 13.361 | 0889,0890 |
| M+H | 865.1579 | 14.992 | 0891,0892 |
| M-H | 863.1436 | 16.602 | 0891,0892,0893 |
| M+H | 865.1628 | 16.544 | 0891,0892,0893 |
| M-H | 863.1751 | 13.855 | 0894 |
| M+H | 866.1912 | 13.654 | 0896 |
| M+H | 867.1602 | 15.043 | 0897 |
| M-H | 866.1451 | 12.458 | 0898 |
| M-H | 866.1929 | 14.833 | 0899 |
| M+H | 869.152 | 17.951 | 0900 |
| M-H | 867.158 | 16.187 | 0901 |
| M-H | 867.17 | 16.615 | 0902 |
| M+H | 869.2013 | 16.456 | 0903 |
| M+H | 869.2448 | 13.439 | 0904 |
| M+H | 869.2645 | 16.229 | 0905 |
| M-H | 868.1346 | 14.736 | 0906 |
| M-H | 868.175 | 13.997 | 0907 |
| M-H | 869.1185 | 16.381 | 0909 |
| M-H | 869.1204 | 17.585 | 0909,0910 |
| M+H | 871.1877 | 11.196 | 0911 |
| M+H | 871.2074 | 17.248 | 0912 |
| M+H | 872.1534 | 15.04 | 0913 |
| M-H | 870.1671 | 16.737 | 0914 |
| M+H | 872.214 | 13.681 | 0915 |
| M+H | 873.1478 | 11.692 | 0916,0917 |
| M+H | 873.148 | 14.323 | 0916,0917 |
| M+H | 873.1673 | 17.393 | 0918,0919 |

(continued)

| [Table 8-11-7] Compound that may be contained in mixture 2-1-m1E03-2 | | | |
|---|---|---|---|
| Ion species | m/z $[M+H]^+$ or $[M-H]^-$ | Retention time of MS peak (min) | No. of assigned compound described with 2-1-m1E03-2-omitted |
| M+H | 873.1662 | 17.471 | 0918,0919 |
| M+H | 874.1479 | 14.754 | 0920 |
| M+H | 874.2155 | 15.421 | 0921 |
| M+H | 876.1783 | 15.195 | 0922,0923 |
| M+H | 876.1786 | 15.248 | 0922,0923 |
| M+H | 877.175 | 14.47 | 0925 |
| M+H | 877.2504 | 15.259 | 0926,0927 |
| M-H | 875.2355 | 15.899 | 0926,0927 |
| M+H | 879.1066 | 16.27 | 0928 |
| M+H | 879.208 | 13.572 | 0929 |
| M+H | 879.2228 | 17.892 | 0930 |
| M+H | 880.1684 | 14.738 | 0931 |
| M+H | 880.2249 | 14.511 | 0932 |
| M+H | 880.264 | 13.763 | 0933 |
| M+H | 881.1564 | 13.449 | 0934 |
| M-H | 879.1795 | 14.207 | 0936 |
| M-H | 880.1639 | 12.887 | 0937 |
| M-H | 880.1704 | 13.726 | 0938 |
| M-H | 881.1404 | 17.528 | 0939 |
| M-H | 882.1523 | 14.149 | 0940 |
| M+H | 884.2103 | 14.353 | 0941 |
| M+H | 885.1854 | 16.718 | 0942 |
| M+H | 885.2577 | 14.322 | 0943 |
| M-H | 884.1333 | 13.829 | 0944 |
| M-H | 884.1506 | 15.441 | 0945 |
| M+H | 887.165 | 15.152 | 0947,0948 |
| M+H | 887.1645 | 13.56 | 0947,0948 |
| M+H | 888.1599 | 11.719 | 0949 |
| M+H | 888.1776 | 16.818 | 0950 |
| M-H | 886.1653 | 14.09 | 0950,0951 |
| M+H | 888.208 | 13.725 | 0953 |
| M+H | 888.2686 | 15.399 | 0954 |
| M-H | 888.1094 | 17.188 | 0956 |
| M+H | 890.1458 | 15.182 | 0957,0958 |
| M+H | 890.1458 | 15.107 | 0957,0958 |
| M-H | 888.1936 | 15.453 | 0959 |
| M+H | 891.1887 | 15.005 | 0960 |

(continued)

| [Table 8-11-7] Compound that may be contained in mixture 2-1-m1E03-2 | | | |
|---|---|---|---|
| Ion species | m/z [M+H]+ or [M-H]- | Retention time of MS peak (min) | No. of assigned compound described with 2-1-m1E03-2-omitted |
| M+H | 892.1732 | 15.274 | 0961,0962 |
| M+H | 892.1742 | 13.218 | 0961,0962 |
| M-H | 894.1714 | 14.593 | 0963 |
| M+H | 896.2598 | 13.785 | 0964 |
| M-H | 896.1514 | 13.472 | 0965 |
| M-H | 896.2044 | 14.822 | 0966 |
| M+H | 900.1644 | 14.81 | 0967 |
| M-H | 898.1692 | 14.577 | 0968 |
| M+H | 900.2018 | 12.446 | 0969 |
| M+H | 901.1813 | 15.583 | 0970 |
| M+H | 902.1651 | 13.442 | 0972 |
| M-H | 901.1527 | 14.095 | 0973 |
| M-H | 902.1251 | 14.733 | 0974 |
| M+H | 904.1886 | 14.734 | 0975 |
| M+H | 904.2617 | 15.435 | 0976 |
| M+H | 905.1535 | 17.166 | 0977 |
| M-H | 904.1047 | 12.095 | 0978 |
| M-H | 904.1546 | 14.576 | 0979 |
| M+H | 907.1835 | 15.107 | 0980 |
| M-H | 906.1158 | 15.713 | 0981 |
| M-H | 913.1813 | 10.048 | 0982 |
| M+H | 917.1765 | 13.77 | 0983 |
| M+H | 918.1759 | 14.742 | 0984 |
| M-H | 917.156 | 13.91 | 0985 |
| M+H | 923.1447 | 17.796 | 0987 |
| M+H | 927.1594 | 14.558 | 0988 |
| M-H | 927.1021 | 15.777 | 0989 |
| M+H | 935.1712 | 16.812 | 0990 |
| M-H | 934.1281 | 14.617 | 0991 |
| M-H | 936.1613 | 14.889 | 0993 |
| M-H | 938.1218 | 16.116 | 0994 |
| M+H | 944.1311 | 17.803 | 0995 |
| M+H | 954.1753 | 14.935 | 0997 |
| M+H | 955.1511 | 18.05 | 0998 |
| M+H | 956.1365 | 16.223 | 0999 |
| M+H | 971.1464 | 18.078 | 1000 |

[Table 414]

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 782.3361 | 10.873 | 0001 |
| M+H | 783.3316 | 10.324 | 0002,0003 |
| M+H | 783.3321 | 9.496 | 0002,0003 |
| M+H | 784.3268 | 8.88 | 0004,0005 |
| M+H | 784.3274 | 10.918 | 0004,0005 |
| M+H | 785.3226 | 9.598 | 0006 |
| M+H | 789.2881 | 10.973 | 0007 |
| M+H | 790.2833 | 9.644 | 0008 |
| M+H | 796.3527 | 11.289 | 0009 |
| M+H | 797.3469 | 10.756 | 0010 |
| M+H | 798.3427 | 11.245 | 0011 |
| M+H | 800.3107 | 9.667 | 0012 |
| M+H | 800.3267 | 10.941 | 0013 |
| M+H | 801.3061 | 8.39 | 0014 |
| M+H | 801.3227 | 10.416 | 0015 |
| M+H | 801.3422 | 9.007 | 0016 |
| M+H | 802.317 | 10.88 | 0017 |
| M+H | 802.3372 | 8.248 | 0018,0019 |
| M+H | 802.337 | 7.186 | 0018,0019 |
| M+H | 803.3027 | 11.379 | 0020 |
| M+H | 803.3326 | 6.534 | 0021,0022 |
| M-H | 801.3169 | 9.055 | 0021,0022 |
| M+H | 804.3279 | 7.651 | 0023 |
| M+H | 807.2783 | 10.974 | 0024 |
| M+H | 808.294 | 9.009 | 0025 |
| M+H | 809.2891 | 7.405 | 0026 |
| M+H | 810.3325 | 9.552 | 0027 |
| M+H | 810.368 | 11.709 | 0028 |
| M+H | 811.3268 | 8.341 | 0029,0030 |
| M+H | 811.3273 | 9.349 | 0029,0030 |
| M+H | 811.3627 | 11.182 | 0031 |
| M+H | 812.3224 | 8.129 | 0032 |
| M+H | 812.3466 | 10.882 | 0033 |
| M+H | 812.359 | 11.693 | 0034 |
| M+H | 813.3425 | 10.323 | 0035 |
| M+H | 814.3262 | 10.03 | 0036 |

(continued)

| [Table 8-11-8] Compound that may be contained in mixture 2-1-m1E04-2 | | | |
|---|---|---|---|
| Ion species | m/z $[M+H]^+$ or $[M-H]^-$ | Retention time of MS peak (min) | No. of assigned compound described with 2-1-m1E04-2-omitted |
| M+H | 814.3378 | 10.219 | 0037 |
| M+H | 815.3587 | 9.31 | 0038 |
| M+H | 816.2885 | 9.825 | 0039 |
| M+H | 816.3537 | 8.576 | 0040 |
| M+H | 817.283 | 8.694 | 0041 |
| M+H | 817.3198 | 11.76 | 0042 |
| M-H | 815.3328 | 9.262 | 0043 |
| M+H | 818.3018 | 9.713 | 0044 |
| M+H | 818.318 | 11.184 | 0045 |
| M+H | 819.2981 | 10.954 | 0046 |
| M+H | 819.3118 | 10.704 | 0047 |
| M+H | 819.3167 | 8.138 | 0047,0048 |
| M+H | 819.3326 | 9.088 | 0049 |
| M+H | 820.3117 | 6.587 | 0050,0051 |
| M+H | 820.3083 | 11.137 | 0050,0051 |
| M+H | 820.3282 | 8.382 | 0052 |
| M+H | 821.3235 | 9.08 | 0053 |
| M+H | 822.3098 | 9.282 | 0054 |
| M+H | 824.3473 | 9.898 | 0055 |
| M+H | 825.2694 | 11.22 | 0056 |
| M+H | 825.3426 | 9.734 | 0057,0058 |
| M+H | 825.3425 | 7.652 | 0057,0058 |
| M+H | 826.2845 | 9.082 | 0059 |
| M+H | 826.337 | 8.253 | 0060 |
| M+H | 828.3223 | 9.642 | 0061,0062 |
| M-H | 826.3087 | 8.652 | 0061,0062 |
| M+H | 828.3426 | 10.419 | 0063 |
| M+H | 829.3173 | 9.445 | 0064,0065 |
| M+H | 829.3144 | 7.887 | 0064,0065 |
| M+H | 829.3373 | 7.982 | 0066 |
| M+H | 829.3736 | 9.639 | 0067 |
| M+H | 830.3034 | 10.154 | 0068 |
| M+H | 830.3258 | 9.816 | 0069 |
| M+H | 830.3325 | 6.411 | 0070,0071 |
| M+H | 830.3355 | 7.983 | 0070,0071 |
| M+H | 830.3688 | 8.981 | 0072 |
| M+H | 831.3278 | 6.474 | 0073 |
| M+H | 831.3528 | 8.449 | 0074 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 831.3641 | 8.874 | 0075 |
| M+H | 832.3479 | 7.583 | 0076,0077 |
| M+H | 832.3516 | 11.726 | 0076,0077 |
| M+H | 833.3328 | 8.4 | 0078 |
| M+H | 833.3472 | 11.226 | 0079,0080 |
| M+H | 833.3472 | 11.139 | 0079,0080 |
| M+H | 834.2782 | 9.912 | 0081 |
| M+H | 834.3426 | 11.602 | 0082,0083 |
| M+H | 834.342 | 10.224 | 0082,0083 |
| M+H | 835.294 | 8.139 | 0084 |
| M+H | 835.3381 | 8.809 | 0085 |
| M+H | 836.2794 | 8.407 | 0086 |
| M+H | 836.2886 | 6.589 | 0087,0088 |
| M+H | 836.2909 | 9.909 | 0087,0088 |
| M+H | 836.3253 | 9.614 | 0089 |
| M+H | 837.2735 | 8.837 | 0090 |
| M+H | 837.3069 | 8.196 | 0091 |
| M+H | 837.3265 | 9.537 | 0092 |
| M+H | 838.3091 | 11.688 | 0093,0094 |
| M+H | 838.3091 | 11.608 | 0093,0094 |
| M+H | 838.3186 | 8.942 | 0095 |
| M+H | 838.3301 | 9.51 | 0096 |
| M+H | 838.363 | 10.267 | 0097 |
| M+H | 839.3032 | 11.731 | 0098,0099 |
| M+H | 839.3041 | 10.301 | 0098,0099 |
| M+H | 839.314 | 9.488 | 0100 |
| M+H | 839.3218 | 8.221 | 0101 |
| M+H | 839.3576 | 10.089 | 0102,0103 |
| M+H | 839.3579 | 9.782 | 0102,0103 |
| M+H | 840.3429 | 9.549 | 0104 |
| M+H | 841.3379 | 9.341 | 0105 |
| M+H | 842.336 | 9.764 | 0106 |
| M+H | 842.3986 | 11.67 | 0107 |
| M+H | 843.3333 | 9.544 | 0108 |
| M+H | 843.3526 | 8.251 | 0109 |
| M+H | 843.3894 | 8.667 | 0110 |
| M+H | 844.2754 | 9.503 | 0111 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 844.3168 | 9.761 | 0112 |
| M+H | 844.348 | 7.723 | 0113,0114 |
| M+H | 844.3504 | 8.254 | 0113,0114 |
| M+H | 845.3432 | 6.199 | 0115 |
| M+H | 846.298 | 10.479 | 0116,0117,0118 |
| M+H | 846.2988 | 9.244 | 0116,0117,0118 |
| M-H | 844.2811 | 9.344 | 0116,0117,0118 |
| M+H | 846.3119 | 9.849 | 0119,0120 |
| M+H | 846.3171 | 9 | 0119,0120 |
| M+H | 847.2932 | 9.927 | 0121,0122,0123 |
| M+H | 847.2935 | 8.984 | 0121,0122,0123 |
| M+H | 847.2934 | 8.093 | 0121,0122,0123 |
| M+H | 847.3086 | 9.59 | 0124 |
| M+H | 847.3275 | 8.065 | 0125,0126 |
| M+H | 847.325 | 9.554 | 0125,0126 |
| M+H | 847.3477 | 8.749 | 0127 |
| M+H | 848.2885 | 8.427 | 0128,0129 |
| M+H | 848.2884 | 7.928 | 0128,0129 |
| M+H | 848.3269 | 8.065 | 0130,0131 |
| M+H | 848.3269 | 8.034 | 0130,0131 |
| M+H | 848.358 | 10.665 | 0132 |
| M+H | 849.2847 | 9.329 | 0133 |
| M+H | 849.3088 | 8.391 | 0134 |
| M+H | 849.3303 | 6.715 | 0135 |
| M+H | 850.2936 | 8.854 | 0136 |
| M+H | 850.3263 | 10.361 | 0137,0138,0139 |
| M+H | 850.3242 | 11.091 | 0137,0138,0139 |
| M+H | 850.3234 | 11.561 | 0137,0138,0139 |
| M+H | 851.3193 | 9.761 | 0140,0141 |
| M+H | 851.3195 | 8.75 | 0140,0141 |
| M+H | 852.2554 | 10.422 | 0142 |
| M+H | 852.2672 | 10.085 | 0143 |
| M+H | 852.3144 | 11.508 | 0144 |
| M+H | 852.3243 | 12.098 | 0145 |
| M+H | 852.3332 | 10.319 | 0146 |
| M+H | 852.3425 | 13.041 | 0147 |
| M+H | 853.2501 | 10.725 | 0148,0149 |

(continued)

| [Table 8-11-8] Compound that may be contained in mixture 2-1-m1E04-2 | | | |
|---|---|---|---|
| Ion species | m/z [M+H]$^+$ or [M-H]$^-$ | Retention time of MS peak (min) | No. of assigned compound described with 2-1-m1E04-2-omitted |
| M+H | 853.2485 | 9.154 | 0148,0149 |
| M+H | 853.284 | 8.209 | 0150 |
| M+H | 853.3481 | 8.303 | 0151 |
| M+H | 853.3736 | 9.372 | 0152 |
| M+H | 854.2447 | 9.259 | 0153 |
| M+H | 854.2691 | 10.178 | 0154 |
| M+H | 854.3443 | 6.605 | 0155 |
| M+H | 855.2856 | 10.209 | 0156 |
| M+H | 855.299 | 10.685 | 0157 |
| M+H | 855.3532 | 8.944 | 0158 |
| M+H | 856.2788 | 6.888 | 0159 |
| M+H | 856.298 | 11.736 | 0160 |
| M+H | 856.3205 | 9.579 | 0161 |
| M+H | 856.3503 | 10.534 | 0162 |
| M+H | 857.2754 | 11.564 | 0164 |
| M+H | 857.3135 | 9.66 | 0165 |
| M-H | 855.3181 | 7.958 | 0166 |
| M+H | 857.3683 | 8.604 | 0167 |
| M+H | 858.3113 | 9.557 | 0168,0169 |
| M-H | 856.2912 | 7.9 | 0168,0169 |
| M+H | 858.3306 | 6.232 | 0170,0171,0172 |
| M-H | 856.319 | 7.639 | 0170,0171,0172 |
| M+H | 858.3344 | 11.238 | 0170,0171,0172 |
| M+H | 858.3661 | 8.608 | 0173,0174 |
| M+H | 858.3628 | 7.187 | 0173,0174 |
| M+H | 859.3042 | 9.05 | 0175 |
| M+H | 859.33 | 9.815 | 0176 |
| M+H | 859.3472 | 7.852 | 0177 |
| M+H | 860.3013 | 9.547 | 0178 |
| M+H | 860.3133 | 10.892 | 0179,0180,0181 |
| M+H | 860.3142 | 9.711 | 0179,0180,0181 |
| M+H | 860.3133 | 10.814 | 0179,0180,0181 |
| M+H | 860.3434 | 6.91 | 0182,0183 |
| M+H | 860.3434 | 6.734 | 0182,0183 |
| M+H | 860.3854 | 10.003 | 0184 |
| M+H | 861.3091 | 10.363 | 0185,0186,0187 |
| M+H | 861.3092 | 8.306 | 0185,0186,0187 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M-H | 859.2933 | 8.45 | 0185,0186,0187 |
| M+H | 861.323 | 9.703 | 0188 |
| M+H | 861.3387 | 5.071 | 0189 |
| M+H | 861.3438 | 7.934 | 0189,0190 |
| M-H | 859.3836 | 8.254 | 0191 |
| M+H | 862.3063 | 9.963 | 0192,0193 |
| M+H | 862.3041 | 8.421 | 0192,0193 |
| M+H | 862.3379 | 7.809 | 0194 |
| M+H | 862.3735 | 11.086 | 0195 |
| M+H | 862.3946 | 6.667 | 0196 |
| M+H | 863.3258 | 8.742 | 0197 |
| M+H | 864.2735 | 8.444 | 0198 |
| M+H | 864.2887 | 10.558 | 0199,0200 |
| M+H | 864.2896 | 9.49 | 0199,0200 |
| M-H | 862.2883 | 18.894 | 0201 |
| M+H | 864.3086 | 9.089 | 0201,0202 |
| M+H | 864.339 | 11.487 | 0203 |
| M+H | 864.3543 | 9.722 | 0204 |
| M+H | 865.2613 | 9.527 | 0205 |
| M+H | 865.2679 | 8.208 | 0206 |
| M+H | 865.2843 | 10.016 | 0207 |
| M+H | 865.305 | 8.724 | 0208,0209,0210 |
| M+H | 865.3034 | 7.896 | 0208,0209,0210 |
| M+H | 865.3027 | 7.568 | 0208,0209,0210 |
| M+H | 865.3181 | 8.503 | 0211,0212 |
| M+H | 865.3217 | 7.102 | 0211,0212 |
| M-H | 864.2531 | 18.83 | 0213 |
| M+H | 866.2797 | 10.914 | 0214 |
| M+H | 866.2872 | 9.935 | 0215 |
| M+H | 866.2993 | 7.909 | 0216,0217,0218,0219 |
| M+H | 866.2989 | 6.145 | 0216,0217,0218 |
| M+H | 866.2992 | 6.947 | 0216,0217,0218,0219 |
| M+H | 866.3014 | 10.56 | 0216,0217,0218,0219 |
| M+H | 866.3179 | 11.667 | 0220,0221 |
| M+H | 866.3195 | 10.661 | 0221 |
| M+H | 866.3399 | 12.488 | 0222 |
| M+H | 866.3615 | 10.372 | 0223 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 867.2664 | 11.148 | 0224 |
| M+H | 867.2931 | 5.968 | 0225,0226 |
| M+H | 867.2943 | 8.955 | 0225,0226 |
| M+H | 867.3136 | 11.189 | 0227 |
| M+H | 867.3641 | 8.62 | 0228 |
| M+H | 868.2868 | 9.632 | 0229 |
| M+H | 868.298 | 10.311 | 0230 |
| M+H | 868.3107 | 11.628 | 0231,0232 |
| M-H | 866.2975 | 11.166 | 0231,0232 |
| M+H | 868.318 | 12.573 | 0232,0233 |
| M+H | 868.3398 | 12.416 | 0234 |
| M+H | 869.2991 | 8.904 | 0235 |
| M+H | 869.3299 | 9.891 | 0236,0237 |
| M+H | 869.33 | 9.322 | 0236,0237 |
| M+H | 870.2463 | 10.473 | 0238 |
| M+H | 870.3247 | 7.587 | 0239,0240,0241 |
| M+H | 870.3248 | 9.269 | 0239,0240,0241 |
| M+H | 870.3231 | 10.571 | 0239,0240,0241 |
| M+H | 871.2406 | 10.809 | 0243 |
| M+H | 871.2604 | 8.867 | 0244 |
| M+H | 871.2746 | 8.677 | 0245 |
| M+H | 871.3199 | 9.752 | 0246 |
| M+H | 871.3299 | 9.94 | 0247 |
| M+H | 871.339 | 8.425 | 0248 |
| M+H | 871.3508 | 8.268 | 0249 |
| M+H | 872.2554 | 7.116 | 0250,0251,0252 |
| M+H | 872.2594 | 10.34 | 0250,0251,0252 |
| M+H | 872.2593 | 10.372 | 0250,0251,0252 |
| M+H | 872.3476 | 10.053 | 0253,0254 |
| M+H | 872.3498 | 11.367 | 0253,0254 |
| M+H | 872.379 | 8.302 | 0255 |
| M-H | 871.2366 | 6.982 | 0257 |
| M+H | 873.2709 | 11.673 | 0258,0259 |
| M+H | 873.2704 | 11.712 | 0258,0259 |
| M+H | 873.3496 | 9.997 | 0260 |
| M+H | 874.2891 | 11.997 | 0261,0262 |
| M+H | 874.2923 | 9.496 | 0261,0262 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 874.3118 | 9.808 | 0263 |
| M+H | 874.3292 | 11.294 | 0264,0265,0266,0267 |
| M+H | 874.3289 | 10.15 | 0264,0265,0266,0267 |
| M+H | 874.3302 | 9.967 | 0264,0265,0266,0267 |
| M+H | 874.3584 | 7.153 | 0268 |
| M+H | 875.2891 | 8.127 | 0269,0270 |
| M+H | 875.2888 | 9.346 | 0269,0270 |
| M-H | 873.2931 | 18.794 | 0271 |
| M+H | 875.3257 | 8.052 | 0272,0273,0274,0275 |
| M+H | 875.3263 | 7.992 | 0272,0273,0274,0275 |
| M+H | 875.3246 | 8.875 | 0272,0273,0274,0275 |
| M+H | 875.3249 | 10.778 | 0272,0273,0274,0275 |
| M+H | 875.357 | 10.148 | 0276,0277 |
| M+H | 875.3581 | 10.182 | 0276,0277 |
| M+H | 875.4147 | 8.524 | 0278 |
| M+H | 876.2837 | 8.522 | 0279,0280,0281 |
| M+H | 876.2814 | 9.803 | 0279,0280,0281 |
| M+H | 876.2874 | 12.403 | 0280,0281 |
| M+H | 876.3092 | 10.497 | 0282,0283 |
| M+H | 876.3094 | 9.106 | 0282,0283 |
| M+H | 876.3198 | 11.641 | 0284 |
| M+H | 876.3285 | 8.805 | 0285 |
| M+H | 877.3037 | 7.911 | 0286 |
| M+H | 877.3409 | 9.142 | 0287,0288 |
| M+H | 877.3399 | 9.577 | 0287,0288 |
| M+H | 878.2884 | 10.267 | 0289 |
| M-H | 876.2868 | 9.709 | 0290,0291,0292 |
| M+H | 878.3073 | 9.496 | 0291,0292 |
| M+H | 878.3073 | 9.538 | 0291,0292 |
| M+H | 878.3183 | 10.232 | 0293 |
| M+H | 878.3348 | 7.386 | 0294,0295 |
| M+H | 878.3422 | 9.15 | 0295 |
| M+H | 878.3563 | 11.875 | 0296 |
| M+H | 878.3758 | 10.229 | 0297 |
| M-H | 877.2876 | 9.879 | 0298,0299 |
| M+H | 879.3134 | 10.052 | 0300 |
| M+H | 879.32 | 9.014 | 0301,0302,0303 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M-H | 877.3018 | 8.268 | 0300,0301,0302,0303 |
| M+H | 879.3195 | 7.843 | 0300,0301,0302,0303 |
| M+H | 879.39 | 8.35 | 0304 |
| M+H | 880.249 | 10.036 | 0305 |
| M+H | 880.2839 | 9.898 | 0306 |
| M+H | 880.315 | 8.252 | 0307,0308,0309 |
| M+H | 880.3151 | 6.31 | 0307,0308,0309 |
| M+H | 880.3151 | 7.716 | 0307,0308,0309 |
| M+H | 880.3284 | 8.294 | 0310 |
| M+H | 880.3344 | 11.061 | 0310,0311 |
| M+H | 881.2445 | 8.524 | 0312 |
| M+H | 881.2813 | 11.551 | 0313 |
| M+H | 881.3105 | 9.259 | 0314,0315 |
| M+H | 881.3108 | 6.448 | 0314,0315 |
| M+H | 881.3804 | 8.338 | 0316 |
| M+H | 882.2623 | 9.919 | 0317,0318 |
| M+H | 882.279 | 10.787 | 0319,0320 |
| M+H | 882.2799 | 9.655 | 0319,0320 |
| M+H | 883.2603 | 10.69 | 0321 |
| M+H | 883.2746 | 10.305 | 0322,0323 |
| M+H | 883.2778 | 8.216 | 0322,0323 |
| M+H | 883.2943 | 8.806 | 0324,0325 |
| M+H | 883.2942 | 8.01 | 0324,0325 |
| M+H | 883.3138 | 7.633 | 0326,0327 |
| M+H | 883.318 | 8.349 | 0327 |
| M+H | 883.3458 | 9.614 | 0328 |
| M+H | 883.3578 | 11.097 | 0329 |
| M+H | 884.2736 | 6.072 | 0330,0331,0332 |
| M+H | 884.2705 | 11.12 | 0330,0331 |
| M+H | 884.2764 | 10.495 | 0331,0332 |
| M+H | 884.2901 | 8.037 | 0333,0334 |
| M+H | 884.2895 | 6.363 | 0333,0334 |
| M+H | 884.3574 | 11.076 | 0335 |
| M-H | 883.2589 | 9.106 | 0336 |
| M+H | 885.2848 | 9.054 | 0337 |
| M+H | 885.2911 | 7.591 | 0338 |
| M+H | 885.3237 | 9.92 | 0339 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 885.3467 | 10.261 | 0340 |
| M+H | 885.3668 | 8.653 | 0341 |
| M+H | 886.2717 | 9.162 | 0342 |
| M+H | 886.2925 | 12.61 | 0343 |
| M+H | 886.304 | 8.461 | 0344,0345 |
| M+H | 886.305 | 11.335 | 0344,0345 |
| M+H | 886.3191 | 9.287 | 0346 |
| M+H | 886.3654 | 11.717 | 0347,0348,0349 |
| M+H | 886.3654 | 11.798 | 0347,0348,0349 |
| M+H | 886.3675 | 12.094 | 0347,0348,0349 |
| M+H | 887.2996 | 8.347 | 0350,0351 |
| M+H | 887.304 | 8.91 | 0350,0351 |
| M+H | 887.3154 | 9.849 | 0352 |
| M+H | 887.3206 | 9.412 | 0352,0353,0354 |
| M+H | 887.3243 | 9.014 | 0353,0354 |
| M+H | 887.3449 | 7.85 | 0355 |
| M+H | 887.3618 | 7.633 | 0356 |
| M+H | 888.2352 | 10.709 | 0357 |
| M+H | 888.308 | 12.886 | 0358 |
| M+H | 888.3235 | 12.545 | 0359 |
| M+H | 888.3462 | 10.378 | 0360,0361,0362,0363 |
| M+H | 888.3502 | 9.116 | 0360,0361,0362,0363 |
| M+H | 889.231 | 10.931 | 0364 |
| M+H | 889.2505 | 8.966 | 0365 |
| M+H | 889.3042 | 9.444 | 0366,0367 |
| M+H | 889.3037 | 8.733 | 0366,0367 |
| M+H | 889.3296 | 8.932 | 0368 |
| M+H | 889.3411 | 8.59 | 0369 |
| M+H | 889.4304 | 8.865 | 0370 |
| M+H | 890.2993 | 9.212 | 0372 |
| M+H | 890.3246 | 9.526 | 0373,0374 |
| M+H | 890.3246 | 9.617 | 0373,0374 |
| M+H | 890.3379 | 10.223 | 0375 |
| M+H | 891.2641 | 9.126 | 0376 |
| M+H | 891.3198 | 9.949 | 0377,0378 |
| M+H | 891.3198 | 9.991 | 0377,0378 |
| M+H | 891.3547 | 8.398 | 0379,0380 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 891.3558 | 9.412 | 0379,0380 |
| M+H | 891.4122 | 13.353 | 0381 |
| M+H | 892.2608 | 8.644 | 0382 |
| M+H | 892.276 | 9.862 | 0383 |
| M+H | 892.2843 | 9.596 | 0384,0385 |
| M-H | 890.2686 | 7.409 | 0384,0385 |
| M+H | 892.3035 | 10.718 | 0386 |
| M+H | 892.3213 | 10.29 | 0387,0388 |
| M+H | 892.3164 | 10.822 | 0387,0388 |
| M-H | 890.3393 | 9.312 | 0389 |
| M+H | 892.4076 | 13.368 | 0390 |
| M+H | 893.2781 | 8.567 | 0391,0392 |
| M+H | 893.2794 | 9.482 | 0391,0392 |
| M+H | 893.2995 | 7.996 | 0393,0394 |
| M+H | 893.2997 | 8.055 | 0393,0394 |
| M+H | 893.316 | 8.466 | 0395 |
| M+H | 893.3345 | 8.373 | 0396,0397,0398,0399,0400 |
| M+H | 893.335 | 9.383 | 0396,0397,0398,0399,0400 |
| M+H | 893.3352 | 8.591 | 0396,0397,0398,0399,0400 |
| M+H | 893.335 | 8.81 | 0396,0397,0398,0399,0400 |
| M+H | 893.3356 | 8.733 | 0397,0398,0399,0400 |
| M+H | 894.2656 | 10.523 | 0401 |
| M+H | 894.2828 | 8.566 | 0402 |
| M+H | 894.2941 | 6.163 | 0403,0404 |
| M+H | 894.2994 | 9.282 | 0403,0404 |
| M+H | 894.3154 | 11.635 | 0405,0406 |
| M+H | 894.3139 | 10.339 | 0405,0406 |
| M+H | 894.3306 | 8.674 | 0407,0408,0409 |
| M+H | 894.3305 | 7.47 | 0407,0408,0409 |
| M+H | 894.3307 | 7.824 | 0407,0408,0409 |
| M-H | 893.2724 | 6.352 | 0410 |
| M+H | 895.3086 | 10.161 | 0411 |
| M+H | 895.3154 | 7.845 | 0412,0413 |
| M+H | 895.3188 | 11.614 | 0412,0413 |
| M+H | 895.3264 | 9.63 | 0414 |
| M+H | 895.3316 | 9.251 | 0415 |
| M+H | 896.295 | 9.904 | 0416,0417 |

(continued)

| [Table 8-11-8] Compound that may be contained in mixture 2-1-m1E04-2 | | | |
|---|---|---|---|
| Ion species | m/z [M+H]$^+$ or [M-H]$^-$ | Retention time of MS peak (min) | No. of assigned compound described with 2-1-m1E04-2-omitted |
| M+H | 896.2991 | 9.284 | 0416,0417 |
| M+H | 896.3137 | 11.325 | 0418,0419,0420,0421 |
| M+H | 896.3097 | 6.115 | 0418,0419,0420,0421 |
| M+H | 896.3103 | 7.408 | 0418,0419,0420,0421 |
| M+H | 896.3149 | 10.151 | 0418,0419,0420,0421 |
| M+H | 897.2931 | 8.567 | 0422,0423 |
| M+H | 897.294 | 8.542 | 0422,0423 |
| M+H | 897.3091 | 10.832 | 0424,0425,0426,0427 |
| M+H | 897.3108 | 8.403 | 0424,0425,0426,0427 |
| M+H | 897.3113 | 8.158 | 0425,0426,0427 |
| M+H | 897.3089 | 9.995 | 0424,0425,0426,0427 |
| M-H | 895.306 | 8.828 | 0428 |
| M+H | 897.3605 | 9.941 | 0429 |
| M+H | 897.38 | 8.818 | 0430 |
| M+H | 898.2398 | 10.144 | 0431 |
| M+H | 898.3045 | 9.842 | 0432,0433,0434,0435 |
| M+H | 898.3044 | 11.64 | 0432,0433,0434,0435 |
| M+H | 898.3065 | 8.242 | 0432,0433,0434,0435 |
| M+H | 898.3061 | 6.816 | 0432,0433,0434,0435 |
| M+H | 898.3237 | 8.796 | 0436 |
| M+H | 899.2559 | 8.316 | 0437 |
| M+H | 899.2916 | 9.512 | 0438 |
| M+H | 899.2989 | 8.28 | 0439 |
| M+H | 899.3451 | 7.87 | 0440 |
| M+H | 900.2399 | 10.271 | 0441 |
| M-H | 898.2336 | 10.144 | 0442 |
| M+H | 900.2503 | 6.185 | 0442,0443 |
| M+H | 900.2706 | 9.819 | 0444 |
| M+H | 900.2854 | 7.143 | 0445 |
| M+H | 900.3094 | 13.019 | 0446 |
| M+H | 900.3403 | 11.894 | 0447 |
| M+H | 900.3825 | 12.235 | 0448,0449 |
| M+H | 900.3812 | 12.147 | 0448,0449 |
| M+H | 901.2342 | 8.799 | 0450 |
| M+H | 901.2684 | 8.344 | 0451,0452 |
| M+H | 901.2696 | 8.273 | 0451,0452 |
| M+H | 901.2856 | 9.264 | 0453,0454 |

(continued)

| [Table 8-11-8] Compound that may be contained in mixture 2-1-m1E04-2 | | | |
|---|---|---|---|
| Ion species | m/z [M+H]$^+$ or [M-H]$^-$ | Retention time of MS peak (min) | No. of assigned compound described with 2-1-m1E04-2-omitted |
| M+H | 901.2855 | 8.207 | 0453,0454 |
| M+H | 901.3031 | 9.546 | 0455 |
| M+H | 901.3152 | 8.216 | 0456 |
| M+H | 902.2701 | 8.835 | 0457,0458,0459,0460 |
| M+H | 902.2706 | 11.388 | 0457,0458,0459,0460 |
| M+H | 902.2713 | 11.223 | 0457,0458,0459,0460 |
| M+H | 902.271 | 10.808 | 0457,0458,0459,0460 |
| M+H | 902.281 | 8.602 | 0461 |
| M+H | 902.3247 | 10.312 | 0462 |
| M+H | 902.3296 | 10.976 | 0462,0463 |
| M+H | 903.2652 | 11.533 | 0466,0467,0468 |
| M+H | 903.2658 | 9.934 | 0466,0467,0468 |
| M+H | 903.2651 | 11.456 | 0466,0467,0468 |
| M+H | 903.2758 | 9.423 | 0469 |
| M-H | 901.2661 | 8.568 | 0470 |
| M+H | 903.2979 | 8.987 | 0471 |
| M+H | 903.3189 | 9.906 | 0472,0473 |
| M+H | 903.3187 | 10.143 | 0472,0473 |
| M+H | 904.2649 | 10.726 | 0474 |
| M+H | 904.2967 | 10.876 | 0475 |
| M+H | 904.3393 | 9.937 | 0477,0478 |
| M-H | 902.3227 | 9.991 | 0477,0478 |
| M+H | 904.3573 | 11.769 | 0479 |
| M+H | 905.2999 | 9.367 | 0480 |
| M+H | 905.3152 | 7.527 | 0481 |
| M+H | 905.3726 | 9.581 | 0482,0483,0484 |
| M-H | 903.3544 | 9.837 | 0482,0483,0484 |
| M+H | 905.3701 | 9.442 | 0482,0483,0484 |
| M+H | 906.2958 | 12.342 | 0485,0486 |
| M+H | 906.3015 | 9.485 | 0485,0486 |
| M+H | 906.3175 | 10.292 | 0487 |
| M+H | 906.3345 | 9.249 | 0488 |
| M+H | 906.3561 | 9.664 | 0489 |
| M+H | 906.3658 | 8.22 | 0490 |
| M+H | 907.2409 | 9.381 | 0491 |
| M+H | 907.2962 | 8.118 | 0492 |
| M+H | 907.3142 | 8.313 | 0493 |

(continued)

| [Table 8-11-8] Compound that may be contained in mixture 2-1-m1E04-2 | | | |
|---|---|---|---|
| Ion species | m/z $[M+H]^+$ or $[M-H]^-$ | Retention time of MS peak (min) | No. of assigned compound described with 2-1-m1E04-2-omitted |
| M+H | 907.3508 | 8.408 | 0494,0495,0496,0497 |
| M+H | 907.3503 | 8.734 | 0494,0495,0496,0497 |
| M+H | 907.3491 | 7.561 | 0494,0495,0496,0497 |
| M+H | 907.3507 | 9.01 | 0494,0495,0496,0497 |
| M+H | 908.2375 | 9.377 | 0498 |
| M+H | 908.2805 | 10.992 | 0499 |
| M+H | 908.3103 | 7.82 | 0500,0501 |
| M+H | 908.3116 | 10.546 | 0500,0501,0502,0503 |
| M+H | 908.3116 | 10.496 | 0500,0501,0502,0503 |
| M+H | 908.3149 | 13.096 | 0500,0501,0502,0503 |
| M+H | 908.3316 | 11.734 | 0504 |
| M+H | 908.3457 | 7.135 | 0505 |
| M+H | 908.3494 | 12.143 | 0505,0506 |
| M+H | 909.3049 | 6.026 | 0507 |
| M+H | 909.3245 | 10.249 | 0508 |
| M+H | 909.3312 | 7.688 | 0509,0510 |
| M+H | 909.3295 | 7.88 | 0508,0509,0510 |
| M+H | 909.3443 | 8.829 | 0511 |
| M+H | 910.2594 | 10.468 | 0512,0513 |
| M-H | 908.2435 | 10.127 | 0512,0513 |
| M+H | 910.2736 | 9.786 | 0514,0515 |
| M+H | 910.2735 | 9.672 | 0514,0515 |
| M+H | 910.3123 | 10.445 | 0516,0517 |
| M+H | 910.3166 | 8.989 | 0517 |
| M+H | 910.3253 | 10.407 | 0518,0519,0520 |
| M+H | 910.3301 | 10.403 | 0518,0519,0520 |
| M-H | 908.3157 | 8.162 | 0520 |
| M+H | 910.3811 | 10.618 | 0521 |
| M+H | 911.2557 | 9.006 | 0522 |
| M+H | 911.2719 | 9.661 | 0523 |
| M+H | 911.2892 | 8.02 | 0524,0525 |
| M+H | 911.2892 | 8.114 | 0524,0525 |
| M+H | 911.3092 | 8.978 | 0526 |
| M+H | 911.3285 | 6.873 | 0527,0528,0529 |
| M+H | 911.326 | 8.785 | 0527,0528,0529 |
| M+H | 911.3248 | 8.858 | 0527,0528,0529 |
| M-H | 910.2338 | 9.067 | 0530 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 912.2847 | 6.265 | 0531,0532 |
| M+H | 912.2848 | 8.162 | 0531,0532 |
| M+H | 912.3082 | 10.581 | 0533 |
| M+H | 912.3216 | 8.209 | 0534 |
| M+H | 912.3356 | 8.544 | 0535 |
| M-H | 911.2539 | 8.7 | 0536 |
| M+H | 913.2905 | 7.879 | 0537 |
| M+H | 913.3231 | 10.321 | 0538,0539 |
| M+H | 913.3197 | 8.844 | 0538,0539 |
| M+H | 914.2853 | 11.184 | 0541,0542,0543,0544,0545,0546 |
| M+H | 914.2855 | 10.138 | 0541,0542,0543,0544,0545,0546 |
| M+H | 914.2862 | 10.819 | 0541,0542,0543,0544,0545,0546 |
| M+H | 914.2865 | 10.708 | 0541,0542,0543,0544,0545,0546 |
| M+H | 914.2865 | 10.933 | 0541,0542,0543,0544,0545,0546 |
| M+H | 914.2865 | 10.886 | 0541,0542,0543,0544,0545,0546 |
| M+H | 914.303 | 8.388 | 0547 |
| M+H | 914.3173 | 8.872 | 0548 |
| M+H | 914.3245 | 13.423 | 0549 |
| M+H | 914.3612 | 11.353 | 0550 |
| M+H | 914.3968 | 12.556 | 0551 |
| M+H | 915.2809 | 10.723 | 0552,0553 |
| M+H | 915.2814 | 9.528 | 0552,0553 |
| M+H | 915.3002 | 8.611 | 0554,0555 |
| M+H | 915.3004 | 8.569 | 0554,0555 |
| M+H | 915.3127 | 10.578 | 0556 |
| M+H | 915.3551 | 10.044 | 0557 |
| M+H | 916.2172 | 11.167 | 0558 |
| M-H | 914.2128 | 10.444 | 0559 |
| M+H | 916.2764 | 11.533 | 0560 |
| M+H | 916.2862 | 11.823 | 0561,0562 |
| M+H | 916.2849 | 10.718 | 0561,0562 |
| M+H | 916.2918 | 9.968 | 0563 |
| M+H | 916.2975 | 7.485 | 0563,0564 |
| M+H | 917.2119 | 9.417 | 0566 |
| M+H | 917.2456 | 8.46 | 0567 |
| M+H | 917.2811 | 10.559 | 0568 |
| M+H | 917.3088 | 7.804 | 0569 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 917.3353 | 10.296 | 0570 |
| M-H | 916.2192 | 7.566 | 0571 |
| M+H | 918.2757 | 9.271 | 0572 |
| M-H | 916.2874 | 5.953 | 0573,0574 |
| M+H | 918.3026 | 8.7 | 0573,0574 |
| M+H | 918.3115 | 11.704 | 0575 |
| M+H | 918.3251 | 11.087 | 0576 |
| M+H | 918.3552 | 12.593 | 0577 |
| M+H | 918.367 | 8.764 | 0578 |
| M+H | 919.2449 | 8.781 | 0579 |
| M+H | 919.2588 | 8.842 | 0580 |
| M+H | 919.2761 | 9.001 | 0581 |
| M+H | 919.3158 | 9.319 | 0582,0583 |
| M+H | 919.3122 | 10.434 | 0582,0583 |
| M+H | 919.3878 | 9.696 | 0584,0585 |
| M+H | 919.3877 | 9.732 | 0584,0585 |
| M+H | 920.243 | 11.099 | 0586,0587 |
| M+H | 920.2403 | 6.629 | 0586,0587 |
| M+H | 920.2609 | 11.467 | 0588,0589,0590 |
| M+H | 920.2606 | 10.882 | 0588,0589,0590 |
| M-H | 918.2492 | 19.585 | 0588,0589,0590 |
| M+H | 920.3097 | 7.602 | 0591 |
| M+H | 920.3144 | 10.435 | 0591,0592 |
| M+H | 920.3326 | 6.746 | 0593 |
| M+H | 920.3685 | 11.01 | 0594 |
| M+H | 921.2362 | 11.396 | 0595 |
| M+H | 921.2768 | 9.355 | 0596,0597 |
| M+H | 921.2787 | 8.605 | 0596,0597 |
| M+H | 921.3298 | 8.694 | 0598 |
| M+H | 921.3352 | 9.245 | 0598,0599 |
| M+H | 921.3655 | 9.209 | 0600,0601 |
| M+H | 921.369 | 9.65 | 0600,0601 |
| M+H | 922.2716 | 8.396 | 0602,0603,0604,0605 |
| M+H | 922.2714 | 7.451 | 0602,0603,0604,0605 |
| M+H | 922.2711 | 7.711 | 0602,0603,0604,0605 |
| M-H | 920.2539 | 9.172 | 0602,0603,0604,0605 |
| M+H | 922.294 | 9.543 | 0606,0607 |

(continued)

| [Table 8-11-8] Compound that may be contained in mixture 2-1-m1E04-2 | | | |
|---|---|---|---|
| Ion species | m/z $[M+H]^+$ or $[M-H]^-$ | Retention time of MS peak (min) | No. of assigned compound described with 2-1-m1E04-2-omitted |
| M+H | 922.2905 | 12.438 | 0606,0607 |
| M+H | 922.3132 | 10.428 | 0608 |
| M+H | 922.3257 | 8.177 | 0609,0610 |
| M+H | 922.3258 | 7.122 | 0609,0610 |
| M+H | 922.3448 | 10.59 | 0611 |
| M+H | 922.3656 | 12.232 | 0612 |
| M+H | 923.2712 | 9.411 | 0613,0614 |
| M+H | 923.2669 | 8.18 | 0613,0614 |
| M+H | 923.3089 | 7.532 | 0615 |
| M+H | 923.346 | 8.545 | 0616,0617 |
| M+H | 923.3461 | 7.628 | 0616,0617 |
| M+H | 923.3628 | 9.549 | 0618 |
| M+H | 924.2625 | 9.394 | 0619 |
| M+H | 924.274 | 9.155 | 0620 |
| M-H | 922.2918 | 10.309 | 0621,0622 |
| M+H | 924.3091 | 10.341 | 0621,0622 |
| M+H | 924.3489 | 8.459 | 0623,0624 |
| M-H | 922.3282 | 9.755 | 0623,0624 |
| M+H | 925.2853 | 9.801 | 0625 |
| M+H | 925.3018 | 10.058 | 0626,0627,0628 |
| M+H | 925.3023 | 10.497 | 0626,0627,0628 |
| M+H | 925.3047 | 8.367 | 0626,0627,0628 |
| M+H | 925.3224 | 8.834 | 0629 |
| M+H | 925.3414 | 9.047 | 0630 |
| M+H | 925.361 | 8.083 | 0631 |
| M+H | 926.2997 | 7.928 | 0632,0633 |
| M+H | 926.305 | 13.287 | 0633,0634 |
| M+H | 926.3102 | 7.811 | 0634 |
| M+H | 926.3223 | 11.987 | 0635 |
| M+H | 926.3359 | 10.799 | 0636 |
| M+H | 926.3566 | 6.445 | 0637 |
| M+H | 926.3757 | 10.707 | 0638 |
| M+H | 927.2867 | 9.14 | 0639 |
| M-H | 925.3021 | 8.073 | 0640,0641 |
| M+H | 927.3206 | 8.026 | 0640,0641 |
| M+H | 927.3412 | 6.814 | 0642 |
| M+H | 928.2281 | 9.303 | 0643 |

(continued)

| [Table 8-11-8] Compound that may be contained in mixture 2-1-m1E04-2 | | | |
|---|---|---|---|
| Ion species | m/z $[M+H]^+$ or $[M-H]^-$ | Retention time of MS peak (min) | No. of assigned compound described with 2-1-m1E04-2-omitted |
| M+H | 928.251 | 10.594 | 0644 |
| M-H | 926.2478 | 9.899 | 0645 |
| M+H | 928.3019 | 11.225 | 0647,0648 |
| M-H | 926.2835 | 10.359 | 0647,0648 |
| M+H | 928.3193 | 8.991 | 0649,0650 |
| M+H | 928.3243 | 8.032 | 0650 |
| M+H | 928.3301 | 8.577 | 0651 |
| M+H | 928.3758 | 11.764 | 0653 |
| M-H | 927.2122 | 9.258 | 0654 |
| M+H | 929.2662 | 8.863 | 0655,0656 |
| M+H | 929.2657 | 7.888 | 0655,0656 |
| M+H | 929.2796 | 8.112 | 0657,0658 |
| M-H | 927.2631 | 8.589 | 0657,0658 |
| M-H | 927.281 | 18.426 | 0659 |
| M+H | 929.3144 | 8.846 | 0660 |
| M+H | 929.3872 | 9.071 | 0661 |
| M+H | 930.2286 | 9.255 | 0662 |
| M+H | 930.2612 | 6.737 | 0664,0665 |
| M+H | 930.2672 | 10.977 | 0665 |
| M+H | 930.2798 | 11.286 | 0666,0667,0668 |
| M+H | 930.2807 | 11.257 | 0666,0667,0668 |
| M+H | 930.2811 | 10.434 | 0667,0668 |
| M+H | 930.3013 | 12.245 | 0669,0670 |
| M+H | 930.3044 | 10.338 | 0669,0670 |
| M+H | 930.3211 | 9.041 | 0671 |
| M+H | 931.2756 | 10.826 | 0672 |
| M+H | 931.2945 | 10.933 | 0673 |
| M+H | 931.3146 | 12.52 | 0674 |
| M+H | 931.3254 | 8.156 | 0675 |
| M+H | 932.2591 | 10.654 | 0676 |
| M+H | 932.2761 | 10.342 | 0677,0678 |
| M+H | 932.276 | 10.881 | 0677,0678 |
| M+H | 932.2805 | 11.095 | 0678,0679,0680 |
| M+H | 932.2781 | 10.682 | 0678,0679,0680 |
| M+H | 932.3501 | 11.409 | 0681 |
| M+H | 933.2609 | 9.113 | 0682 |
| M+H | 933.2926 | 9.541 | 0683,0684,0685,0686 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 933.2916 | 9.054 | 0683,0684,0685,0686 |
| M+H | 933.2915 | 8.804 | 0683,0684,0685,0686 |
| M+H | 933.2905 | 8.992 | 0683,0684,0685,0686 |
| M+H | 933.3304 | 9.594 | 0687 |
| M+H | 933.3667 | 9.474 | 0688 |
| M+H | 933.4031 | 8.019 | 0689 |
| M+H | 934.207 | 11.317 | 0690 |
| M+H | 934.2865 | 8.934 | 0691,0692,0693 |
| M+H | 934.2873 | 7.33 | 0691,0692,0693 |
| M+H | 934.2861 | 8.959 | 0691,0692,0693 |
| M+H | 934.3079 | 9.728 | 0694,0695 |
| M+H | 934.3057 | 10.887 | 0694,0695 |
| M+H | 934.3619 | 7.849 | 0696 |
| M+H | 934.388 | 10.467 | 0697 |
| M+H | 935.2213 | 9.395 | 0698 |
| M+H | 935.2369 | 9.005 | 0699 |
| M+H | 935.2717 | 9.287 | 0700 |
| M+H | 935.2822 | 9.757 | 0701 |
| M+H | 935.2921 | 9.658 | 0702,0703 |
| M+H | 935.2903 | 8.606 | 0702,0703 |
| M+H | 935.3004 | 7.955 | 0704 |
| M-H | 933.3662 | 8.725 | 0705 |
| M+H | 936.217 | 6.861 | 0706,0707 |
| M+H | 936.2203 | 10.607 | 0706,0707 |
| M+H | 936.2377 | 11.199 | 0708 |
| M+H | 936.2878 | 8.687 | 0709,0710,0711 |
| M+H | 936.2902 | 11.19 | 0709,0710,0711 |
| M+H | 936.2865 | 8.529 | 0709,0710,0711 |
| M+H | 936.3406 | 8.56 | 0712 |
| M+H | 936.3677 | 9.712 | 0713 |
| M+H | 936.3819 | 12.553 | 0714 |
| M+H | 937.2355 | 8.935 | 0715,0716 |
| M+H | 937.232 | 11.512 | 0715,0716 |
| M+H | 937.2836 | 13.004 | 0717,0718 |
| M+H | 937.2875 | 11.611 | 0717,0718 |
| M+H | 937.3056 | 9.406 | 0719 |
| M+H | 937.3155 | 10.127 | 0720 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 937.3305 | 9.28 | 0721 |
| M+H | 937.3617 | 8.804 | 0722 |
| M+H | 938.2505 | 11.723 | 0723,0724 |
| M+H | 938.2516 | 11.081 | 0723,0724 |
| M+H | 938.2894 | 10.284 | 0725 |
| M+H | 938.3213 | 7.467 | 0726 |
| M+H | 939.2475 | 9.678 | 0727 |
| M+H | 939.2668 | 9.475 | 0728,0729,0730 |
| M-H | 937.2496 | 9.368 | 0728,0729,0730 |
| M+H | 939.2698 | 8.366 | 0728,0729,0730 |
| M+H | 939.3195 | 10.34 | 0731,0732 |
| M+H | 939.3208 | 8.747 | 0731,0732 |
| M+H | 939.339 | 7.818 | 0733 |
| M+H | 939.3774 | 8.402 | 0734 |
| M+H | 940.2451 | 8.766 | 0735,0736 |
| M+H | 940.2446 | 12.148 | 0735,0736 |
| M+H | 940.2671 | 12.945 | 0737 |
| M+H | 940.3379 | 12.099 | 0738,0739 |
| M-H | 938.3284 | 8.977 | 0739 |
| M+H | 941.2972 | 10.53 | 0740,0741 |
| M+H | 941.3 | 7.608 | 0740,0741 |
| M+H | 941.317 | 8.901 | 0742 |
| M+H | 941.3502 | 9.077 | 0743 |
| M+H | 942.2808 | 10.259 | 0744 |
| M+H | 942.3007 | 10.46 | 0745 |
| M+H | 942.3173 | 12.079 | 0746,0747,0748 |
| M+H | 942.3178 | 11.557 | 0746,0747,0748 |
| M+H | 942.3167 | 11.602 | 0746,0747,0748 |
| M+H | 942.3334 | 9.352 | 0749 |
| M+H | 942.3935 | 12.182 | 0750 |
| M+H | 943.2749 | 10.138 | 0751 |
| M+H | 943.2831 | 9.292 | 0752 |
| M+H | 943.3522 | 8.179 | 0753 |
| M+H | 944.246 | 9.696 | 0754 |
| M+H | 944.2954 | 9.935 | 0755,0756,0757 |
| M+H | 944.2976 | 10.46 | 0756,0757 |
| M+H | 944.2961 | 10.805 | 0755,0756,0757 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 944.3717 | 11.349 | 0758 |
| M+H | 945.2895 | 10.57 | 0759 |
| M+H | 945.3128 | 11.316 | 0760,0761 |
| M+H | 945.3127 | 11.245 | 0760,0761 |
| M+H | 945.3414 | 8.312 | 0763 |
| M+H | 945.3818 | 9.111 | 0764 |
| M-H | 944.2088 | 18.732 | 0765 |
| M+H | 946.2414 | 10.813 | 0766 |
| M+H | 946.2964 | 11.613 | 0767 |
| M-H | 945.2402 | 9.006 | 0768 |
| M+H | 947.2708 | 8.581 | 0769 |
| M+H | 947.2763 | 9.521 | 0769,0770 |
| M+H | 947.2971 | 9.981 | 0771 |
| M+H | 947.3066 | 9.331 | 0772,0773 |
| M-H | 945.2909 | 9.099 | 0772,0773 |
| M+H | 947.3207 | 7.419 | 0774 |
| M+H | 947.3454 | 9.94 | 0775 |
| M+H | 947.3832 | 9.754 | 0776 |
| M+H | 948.2358 | 10.972 | 0777 |
| M+H | 948.2533 | 10.791 | 0778 |
| M+H | 948.3052 | 8.811 | 0779 |
| M+H | 948.3194 | 10.88 | 0780,0781 |
| M+H | 948.3234 | 9.876 | 0780,0781 |
| M+H | 949.2379 | 9.729 | 0782 |
| M+H | 949.2562 | 8.478 | 0783 |
| M+H | 949.2868 | 9.612 | 0784,0785 |
| M+H | 949.2857 | 8.861 | 0784,0785 |
| M+H | 949.3086 | 9.866 | 0786,0787 |
| M+H | 949.3075 | 10.043 | 0786,0787 |
| M+H | 949.325 | 8.736 | 0788 |
| M+H | 950.2572 | 11.084 | 0789 |
| M+H | 950.2661 | 8.49 | 0790,0791 |
| M+H | 950.2664 | 11.081 | 0790,0791 |
| M+H | 950.2807 | 8.995 | 0792 |
| M+H | 950.3026 | 9.303 | 0793,0794 |
| M+H | 950.3003 | 10.992 | 0793,0794 |
| M+H | 950.3209 | 11.977 | 0795 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 950.3407 | 11.65 | 0796 |
| M+H | 951.262 | 8.436 | 0797,0798 |
| M+H | 951.2651 | 9.234 | 0797,0798 |
| M+H | 951.2762 | 9.828 | 0799 |
| M+H | 951.2822 | 9.146 | 0799,0800 |
| M+H | 951.2833 | 9.114 | 0800,0801,0802 |
| M-H | 949.2711 | 8.933 | 0800,0801,0802 |
| M+H | 951.358 | 9.558 | 0803 |
| M+H | 952.1969 | 11.583 | 0804 |
| M+H | 952.2863 | 12.282 | 0805,0806 |
| M+H | 952.2862 | 8.644 | 0805,0806 |
| M+H | 953.213 | 9.557 | 0807 |
| M+H | 953.2613 | 10.847 | 0808 |
| M+H | 953.29 | 8.502 | 0809 |
| M+H | 953.3112 | 10.151 | 0810 |
| M+H | 953.3921 | 8.763 | 0811 |
| M-H | 952.2595 | 9.163 | 0812,0813 |
| M+H | 954.278 | 9.079 | 0812,0813 |
| M+H | 954.3538 | 12.406 | 0814 |
| M+H | 955.2245 | 9.402 | 0815 |
| M+H | 955.2437 | 9.725 | 0816 |
| M+H | 955.2958 | 9.818 | 0817 |
| M+H | 955.3718 | 7.646 | 0818 |
| M+H | 956.2217 | 8.99 | 0819 |
| M+H | 956.2379 | 9.744 | 0820 |
| M+H | 956.3321 | 12.152 | 0821 |
| M+H | 956.3716 | 10.467 | 0822 |
| M+H | 957.2546 | 9.75 | 0823,0824 |
| M+H | 957.2542 | 10.465 | 0823,0824 |
| M+H | 957.2913 | 10.245 | 0825 |
| M+H | 957.2974 | 9.608 | 0826 |
| M+H | 958.2752 | 10.366 | 0827 |
| M+H | 958.3136 | 10.853 | 0828,0829 |
| M+H | 958.3131 | 10.937 | 0828,0829 |
| M+H | 959.271 | 10.262 | 0830 |
| M+H | 959.2754 | 8.441 | 0830,0831 |
| M+H | 959.3439 | 10.19 | 0832 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M-H | 958.2583 | 11.33 | 0833,0834 |
| M+H | 960.2718 | 9.512 | 0833,0834 |
| M+H | 961.2851 | 8.942 | 0835 |
| M+H | 961.3226 | 9.686 | 0836,0837,0838 |
| M+H | 961.322 | 9.392 | 0836,0837,0838 |
| M+H | 961.3208 | 9.455 | 0836,0837,0838 |
| M+H | 961.3417 | 8.621 | 0839 |
| M-H | 959.3812 | 10.106 | 0840 |
| M+H | 962.2861 | 8.944 | 0841,0842 |
| M+H | 962.285 | 8.925 | 0841,0842 |
| M+H | 962.337 | 11.562 | 0843 |
| M+H | 963.2523 | 10.233 | 0844 |
| M+H | 963.3026 | 8.736 | 0845,0846 |
| M+H | 963.301 | 8.288 | 0845,0846 |
| M+H | 964.2322 | 11.117 | 0848 |
| M+H | 964.2706 | 10.728 | 0849 |
| M-H | 962.2829 | 0.342 | 0850,0851 |
| M+H | 964.3006 | 11.63 | 0850,0851 |
| M+H | 964.3182 | 9.452 | 0852 |
| M+H | 964.3767 | 12.188 | 0853 |
| M+H | 965.2312 | 8.897 | 0854 |
| M-H | 963.2297 | 9.459 | 0855 |
| M+H | 965.2656 | 9.33 | 0856 |
| M+H | 965.2781 | 8.407 | 0857 |
| M-H | 963.2832 | 0.695 | 0858 |
| M+H | 966.2308 | 11.439 | 0859,0860 |
| M+H | 966.2311 | 12.131 | 0859,0860 |
| M+H | 966.2915 | 10.737 | 0861 |
| M+H | 966.2969 | 9.552 | 0861,0862,0863 |
| M+H | 966.2942 | 8.524 | 0861,0862,0863 |
| M+H | 967.2274 | 9.888 | 0864 |
| M+H | 967.2444 | 9.39 | 0865 |
| M+H | 967.2605 | 8.613 | 0866 |
| M+H | 967.2966 | 11.306 | 0867 |
| M-H | 966.2091 | 11.068 | 0868 |
| M+H | 968.2568 | 8.609 | 0869 |
| M+H | 968.2945 | 9.479 | 0870 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M-H | 967.2623 | 7.708 | 0871 |
| M+H | 969.3482 | 8.966 | 0872 |
| M+H | 970.2574 | 12.126 | 0873,0874 |
| M+H | 970.2567 | 11.44 | 0873,0874 |
| M+H | 970.3476 | 12.505 | 0875 |
| M+H | 971.2009 | 10.071 | 0876 |
| M+H | 972.267 | 9.544 | 0877 |
| M+H | 973.2859 | 10.384 | 0878 |
| M+H | 974.2769 | 8.491 | 0880 |
| M+H | 974.3434 | 10.417 | 0882 |
| M+H | 975.3373 | 10.22 | 0883 |
| M+H | 976.2117 | 9.287 | 0884 |
| M+H | 976.2648 | 10.471 | 0885 |
| M+H | 976.2818 | 9.722 | 0886 |
| M+H | 976.2968 | 8.797 | 0887 |
| M+H | 977.2807 | 8.993 | 0888 |
| M+H | 977.318 | 9.505 | 0889,0890 |
| M+H | 977.3181 | 9.605 | 0889,0890 |
| M+H | 978.2428 | 9.65 | 0891,0892 |
| M+H | 978.2426 | 9.936 | 0891,0892 |
| M+H | 978.2469 | 11.3 | 0891,0892,0893 |
| M+H | 978.2793 | 8.983 | 0894 |
| M-H | 976.2763 | 19.13 | 0895 |
| M+H | 979.2812 | 8.322 | 0896 |
| M-H | 978.2315 | 11.264 | 0897 |
| M+H | 981.2423 | 11.685 | 0898 |
| M-H | 979.2776 | 18.437 | 0899 |
| M-H | 980.2208 | 9.802 | 0900 |
| M+H | 982.2625 | 10.812 | 0901 |
| M+H | 982.2724 | 11.45 | 0902 |
| M+H | 982.2862 | 10.711 | 0903 |
| M+H | 982.3285 | 8.593 | 0904 |
| M+H | 982.3482 | 12.01 | 0905 |
| M+H | 983.2383 | 9.496 | 0906 |
| M+H | 983.2763 | 9.097 | 0907 |
| M+H | 984.2042 | 12.134 | 0908 |
| M+H | 984.2213 | 11.221 | 0909,0910 |

(continued)

| [Table 8-11-8] Compound that may be contained in mixture 2-1-m1E04-2 | | | |
|---|---|---|---|
| Ion species | m/z [M+H]$^+$ or [M-H]$^-$ | Retention time of MS peak (min) | No. of assigned compound described with 2-1-m1E04-2-omitted |
| M+H | 984.2221 | 11.545 | 0909,0910 |
| M+H | 984.2713 | 7.133 | 0911 |
| M+H | 984.2889 | 8.884 | 0912 |
| M+H | 985.2382 | 9.576 | 0913 |
| M+H | 985.2683 | 11.182 | 0914 |
| M-H | 983.279 | 8.893 | 0915 |
| M+H | 986.2325 | 9.366 | 0916,0917 |
| M+H | 986.2329 | 7.31 | 0916,0917 |
| M+H | 986.2522 | 12.224 | 0918,0919 |
| M+H | 986.2516 | 11.497 | 0918,0919 |
| M+H | 987.2326 | 9.799 | 0920 |
| M+H | 987.3018 | 10.085 | 0921 |
| M+H | 989.2634 | 10.015 | 0922,0923 |
| M+H | 989.264 | 10.257 | 0922,0923 |
| M+H | 989.3546 | 10.382 | 0924 |
| M+H | 990.2586 | 9.568 | 0925 |
| M+H | 990.3336 | 10 | 0926 |
| M+H | 990.338 | 10.525 | 0926,0927 |
| M+H | 992.1888 | 9.93 | 0928 |
| M+H | 992.2917 | 8.802 | 0929 |
| M-H | 990.283 | 19.754 | 0930 |
| M+H | 993.2532 | 9.458 | 0931 |
| M-H | 991.2941 | 8.658 | 0932 |
| M+H | 993.348 | 8.943 | 0933 |
| M+H | 994.2413 | 11.382 | 0934 |
| M-H | 992.2521 | 9.833 | 0935 |
| M+H | 994.2862 | 8.708 | 0936 |
| M+H | 995.2575 | 12.141 | 0937 |
| M+H | 995.2723 | 9.031 | 0938 |
| M+H | 996.2428 | 11.497 | 0939 |
| M-H | 995.2355 | 9.84 | 0940 |
| M+H | 997.2917 | 9.407 | 0941 |
| M+H | 998.2679 | 11.557 | 0942 |
| M+H | 998.3434 | 12.1 | 0943 |
| M+H | 999.2334 | 11.821 | 0944 |
| M+H | 999.2524 | 9.918 | 0945 |
| M+H | 1000.1978 | 12.283 | 0946 |

(continued)

| [Table 8-11-8] Compound that may be contained in mixture 2-1-m1E04-2 | | | |
|---|---|---|---|
| Ion species | m/z [M+H]+ or [M-H]- | Retention time of MS peak (min) | No. of assigned compound described with 2-1-m1E04-2-omitted |
| M+H | 1000.2517 | 11.052 | 0947,0948 |
| M+H | 1000.2487 | 9.735 | 0947,0948 |
| M+H | 1001.2436 | 7.131 | 0949 |
| M+H | 1001.2625 | 11.252 | 0950 |
| M+H | 1001.2663 | 9.223 | 0950,0951 |
| M+H | 1001.2755 | 9.827 | 0952 |
| M+H | 1001.2929 | 8.932 | 0953 |
| M+H | 1001.3533 | 10.283 | 0954 |
| M+H | 1002.2142 | 11.805 | 0955 |
| M+H | 1003.2095 | 10.498 | 0956 |
| M+H | 1003.2264 | 9.852 | 0957,0958 |
| M+H | 1003.2291 | 9.71 | 0957,0958 |
| M+H | 1003.2967 | 10.142 | 0959 |
| M+H | 1004.2753 | 10.289 | 0960 |
| M+H | 1005.2568 | 10.059 | 0961,0962 |
| M+H | 1005.2598 | 10.288 | 0961,0962 |
| M+H | 1009.2734 | 12.767 | 0963 |
| M+H | 1009.3438 | 9.01 | 0964 |
| M+H | 1011.2526 | 11.752 | 0965 |
| M+H | 1011.3076 | 9.793 | 0966 |
| M+H | 1013.248 | 9.899 | 0967 |
| M+H | 1013.2687 | 10.358 | 0968 |
| M+H | 1013.2855 | 8.681 | 0969 |
| M+H | 1014.2644 | 10.286 | 0970 |
| M+H | 1014.2883 | 11.562 | 0971 |
| M+H | 1015.2499 | 9.896 | 0972 |
| M+H | 1016.2475 | 12.428 | 0973 |
| M+H | 1017.2242 | 12.093 | 0974 |
| M+H | 1017.2739 | 9.888 | 0975 |
| M-H | 1015.3307 | 10.328 | 0976 |
| M-H | 1016.2214 | 9.744 | 0977 |
| M+H | 1019.2036 | 10.566 | 0978 |
| M+H | 1019.2574 | 9.58 | 0979 |
| M+H | 1020.2686 | 9.887 | 0980 |
| M+H | 1021.219 | 10.194 | 0981 |
| M+H | 1028.2793 | 10.598 | 0982 |
| M+H | 1030.2598 | 9.591 | 0983 |

(continued)

| [Table 8-11-8] Compound that may be contained in mixture 2-1-m1E04-2 | | | |
|---|---|---|---|
| Ion species | m/z $[M+H]^+$ or $[M-H]^-$ | Retention time of MS peak (min) | No. of assigned compound described with 2-1-m1E04-2-omitted |
| M+H | 1031.2583 | 12.786 | 0984 |
| M+H | 1032.2589 | 11.452 | 0985 |
| M+H | 1034.2185 | 12.315 | 0986 |
| M+H | 1036.2291 | 10.436 | 0987 |
| M+H | 1040.2434 | 9.564 | 0988 |
| M+H | 1042.2043 | 10.129 | 0989 |
| M+H | 1048.2523 | 11.58 | 0990 |
| M+H | 1049.231 | 12.655 | 0991 |
| M+H | 1050.2153 | 12.414 | 0992 |
| M+H | 1051.2633 | 9.996 | 0993 |
| M+H | 1053.2247 | 10.596 | 0994 |
| M+H | 1057.2165 | 10.319 | 0995 |
| M+H | 1065.2253 | 12.753 | 0996 |
| M+H | 1067.2568 | 10.056 | 0997 |
| M+H | 1068.2359 | 10.848 | 0998 |
| M+H | 1069.2186 | 10.655 | 0999 |
| M+H | 1084.2301 | 10.929 | 1000 |

The ratio of observed m/z $(M+H)^+$ or $(M-H)^-$ derived from the compounds designed as library components in each mixture is shown in Table 8-11-9.

[Table 415]

| [Table 8-11-9] | | | | | | | | |
|---|---|---|---|---|---|---|---|---|
| Mixture No. | 2-1- mlE01-1 | 2-1- m1E02-1 | 2-1- m1E03-1 | 2-1- m1E04-1 | 2-1- m1E01-2 | 2-1- m1E02-2 | 2-1- m1E03-2 | 2-1- m1E04-2 |
| The number of corresponding m/z observed | 1000 | 1000 | 1000 | 994 | 990 | 992 | 966 | 979 |
| Ratio of corresponding m/z observed to whole library components (%) | 100 | 100 | 100 | 99.4 | 99.0 | 99.2 | 96.6 | 97.9 |
| The number of $M+H^+$ observed | 905 | 861 | 877 | 929 | 893 | 920 | 858 | 916 |
| Ratio of $M+H^+$ observed (%) | 90.5 | 86.1 | 87.7 | 92.9 | 89.3 | 92.0 | 85.8 | 91.6 |
| The number of $M-H^-$ observed | 580 | 708 | 641 | 738 | 584 | 753 | 617 | 694 |
| Ratio of $M-H^-$ observed (%) | 58.0 | 70.8 | 64.1 | 73.8 | 58.4 | 75.3 | 61.7 | 69.4 |
| The number of $M+H^+$ and $M-H^-$ observed together | 485 | 569 | 518 | 667 | 487 | 681 | 509 | 631 |
| The number of $M+H^+$ observed alone | 420 | 292 | 359 | 262 | 406 | 239 | 349 | 285 |
| The number of $M-H^-$ observed alone | 95 | 139 | 123 | 71 | 97 | 72 | 108 | 63 |

Thus, in Example 8, a plurality of mixture libraries of 1,000 compounds having diversity imparted not only to core blocks but to linkers that linked them were successfully synthesized by appropriately selecting and applying highly robust methods as to reaction conditions for use in diverse bond formations and washing methods. These libraries were synthesized with a very high success rate as described above. This shows as an actual example that in the synthesis of a library in which the "diversity of building blocks" and the "diversity imparted to linkers that link the building blocks" are multiplied, a compound group containing 1,000 desired compounds is practically feasible with high quality. This Example also illustrates an actual example of bond formation reaction using a mixture of 1,000 compounds as substrates, and indicates that library synthesis can be performed with a high probability of success and high quality even for such highly diverse substrates. In short, bond formation is achieved even for a mixture of 1,000 compounds with quality maintained, indicating high value of the present invention. This Example is a mere illustration of library synthesis. In the present invention, the building blocks or the linkers that link them are not limited to those shown as an actual example, and library synthesis can be carried out by selecting arbitrary building blocks and arbitrary linkers.

Example 9; Practice example of compound library synthesis for AS-MS - 3

Figure 6 shows the "diversity of core blocks" and "diversity of linkers" involved in the library. Figure 7 shows a library synthesis route by split & mix.

In Example 9, mixture numbers may be used to indicate synthesized mixtures. For example, "2-2" in "2-2-B00-0" represents the type of a library, and "B00" represents the type of a mixture. "-0" represents a state supported on the solid phase, and the term "-1" represents a state cleaved from the solid phase. Also, "-2" represents a state where functional group conversion was performed after cleavage from the solid phase. ID may be used to indicate each compound contained in a mixture. For example, "0001" in "2-2-a1B01-0- 0001" represents an arbitrary compound among compounds contained in the mixture "2-2- a1B01-0".

Example 9-1: Formation of linker a1 by step h01

Example 9-1-1: Preparation of 2-2-B00-0 for use in step a1

[Formula 651]

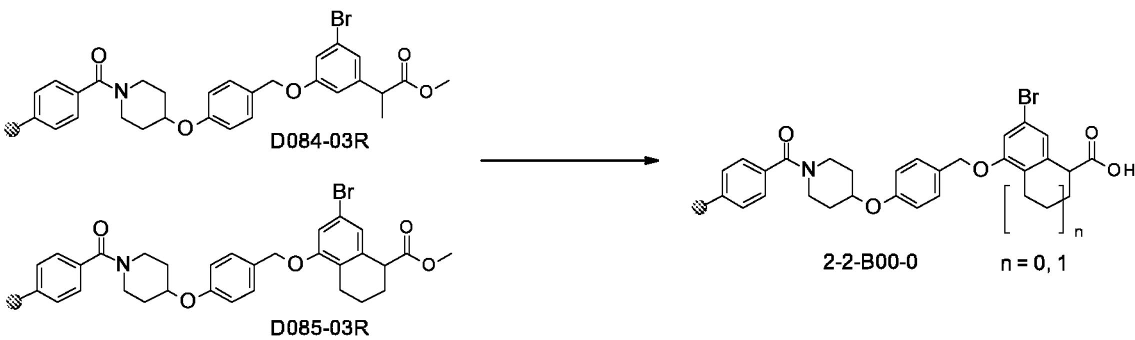

D084-03R

D085-03R

2-2-B00-0

n = 0, 1

Compound D084-03R (3.2 g, 0.200 mmol/g), compound D085-03R (3.2 g, 0.200 mmol/g), NMP (90 mL), and tAmylOH (25.6 mL) were added to a 200 mL column with a filter. The column was capped so as not to leak the reaction solution, and shaken at room temperature for 5 minutes. A 1 M aqueous TBAOH solution (2.56 mL, 2.56 mmol) was added thereto. The column was capped so as not to leak the reaction solution, and shaken at room temperature for 1 hour.

Solid-phase purification

The suspension of the reaction solution and the resin was filtered through the filter of the column and washed three times with NMP/water = 1/1 (128 mL), three times with NMP (128 mL), three times with 0.05 M $TBAHSO_4$ + DTBP in NMP (128 mL), three times with NMP (128 mL), three times with MeOH (128 mL), three times with DCM (128 mL), and three times with heptane (128 mL), and the obtained solid phase was dried under reduced pressure to obtain mixture 2-2-B00-0 (6.81 g, 0.201 mmol/g).

Mixture 2-2-B00-0 can be treated by cleavage in the same manner as in Example 8- 1-1 to obtain mixture 2-2-B00-1. The details of the mixture are shown in Table 9-1-0.

A notation method in each table will be described. For example, in Table 9-1-0, each compound that may be contained in 2-2-B00-1 is indicated by ID, and core blocks and linkers in combination in the structure of mixture 2-2-B00-1 are indicated by symbols or chemical formulas. The correspondence between the symbols and specific structures are shown in Figure 6. In Table 9-1-0, "×", "a" and "b" are represented by chemical formulas.

[Formula 652]

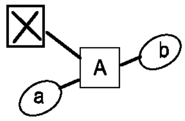

2-2-B00-1

For example, when ID is 2-2-B00-1-0001, the compound is represented by the following structural formula.

[Formula 653]

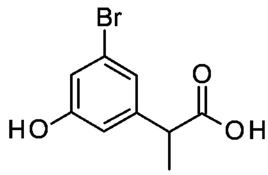

2-2-B00-1-0001

[Table 416]

| Exemplary notation in [Table 9-1-0] | | | | | |
|---|---|---|---|---|---|
| ID | Exact Mass | | A | a | b |
| 2-2-B00-1-0001 | 243.974 | OH | A01 | COOH | Br |

[Table 417]

| Table 9-1-0 Compound that may be contained in mixture 2-2-B00-1 | | | | | |
|---|---|---|---|---|---|
| ID | Exact Mass | | A | a | b |
| 2-2-B00-1-0001 | 243.974 | OH | A01 | COOH | Br |
| 2-2-B00-1-0002 | 269.989 | OH | A02 | COOH | Br |

Example 9-1-2: Synthesis of 2-2-a1B01-0 to 2-2-a1B05-0 by step a1

[Formula 654]

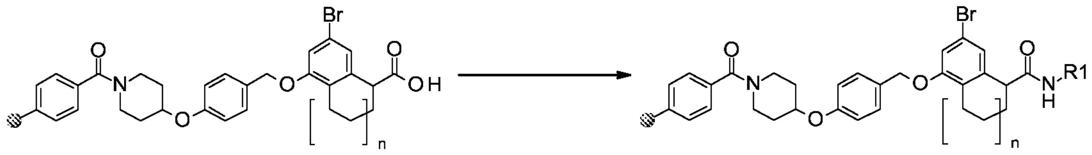

2-2-B00-0 n = 0, 1 2-2-a1B01-0 ~ 2-2-a1B05-0 n = 0, 1

Mixture 2-2-B00-0 (1.28 g, 0.201 mmol/g) and DCM (15.4 mL) were added to five 20 mL columns with a filter. Separate amines (1.80 mmol) described in Table 9-1-1 were added to the columns, respectively, which were then capped so as not to leak the reaction solution, and shaken at room temperature for 1 hour. 1 M PipClU (0.770 mmol) + NMI (0.770 mmol) in MeCN (0.013 mL) were added to the columns, which were then capped so as not to leak the reaction solution, and shaken at room temperature for 2 hours.

Solid-phase purification

The suspension of the reaction solution and the solid phase was filtered through the filter of the column and washed three times with NMP/water = 1/1 (25 mL), three times with NMP (25 mL), three times with MeOH (25 mL), three times with DCM (25 mL), and once with heptane (25 mL), and the obtained solid phase was dried under reduced pressure to obtain the mixtures described in Table 9-1-1.

[Table 418]

| [Table 9-1-1] | | | | |
|---|---|---|---|---|
| Compound No. of amine | Compound name of amine | Molar number used | Amount used | No. obtained mixture |
| BB2-2-01 | Aniline | 1.80 mmol | 0.164 mL | 2-2-a1B01-0 |
| BB2-2-02 | 4-Methylaniline | 1.80 mmol | 0.193 g | 2-2-a1B02-0 |
| BB2-2-03 | 4-Fluoroaniline | 1.80 mmol | 0.170 mL | 2-2-a1B03-0 |
| BB2-2-04 | 1,3-Benzothiazol-2-amine | 1.80 mmol | 0.270 g | 2-2-a1B04-0 |
| BB2-2-05 | 4-(Trifluoromethyl)aniline | 1.80 mmol | 0.226 mL | 2-2-a1B05-0 |

Mixture 2-2-a1B01-0 to 2-2-a1B05-0 can be treated by cleavage in the same manner as in Example 8-1-1 to obtain mixture 2-2-a1B01-1 to 2-2-a1B05-1. The details of the mixtures are shown in Table 9-1-2 to Table 9-1-6.

A notation method in each table will be described. For example, in Table 9-1-2, each compound that may be contained in 2-2-a1B01-1 is indicated by ID, and core blocks and linkers in combination in the structure of mixture 2-2-a1B01-1 are indicated by symbols or chemical formulas. The correspondence between the symbols and specific structures are shown in Figure 6.

In Table 9-1-2 to Table 9-1-6, "×" and "b" are represented by chemical formulas.

[Formula 655]

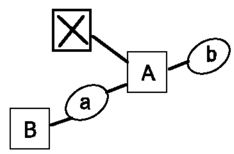


2−2−a1B01−1

For example, when ID is 2-2-a1B01-1-0001, the compound is represented by the following structural formula.

[Formula 656]

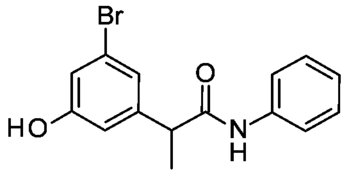

2-2-a1B01-1-0001

Exemplary notation corresponding to 2-2-a1B01-1-0001

[Table 419]

| Exemplary notation in [Table 9-1-2] | | | | | | |
|---|---|---|---|---|---|---|
| ID | Exact Mass | | A | a | B | b |
| 2-2-a1B01-1-0001 | 319.021 | OH | A01 | a1 | B01 | Br |

[Table 420]

| [Table 9-1-2] Compound that may be contained in mixture 2-2-a1B01-1 | | | | | | |
|---|---|---|---|---|---|---|
| ID | Exact Mass | | A | a | B | b |
| 2-2-a1B01-1-0001 | 319.021 | OH | A01 | a1 | B01 | Br |
| 2-2-a1B01-1-0002 | 345.036 | OH | A02 | a1 | B01 | Br |

[Table 421]

| [Table 9-1-3] Compound that may be contained in mixture 2-2-a1B02-1 | | | | | | |
|---|---|---|---|---|---|---|
| ID | Exact Mass | | A | a | B | b |
| 2-2-a1B02-1-0001 | 333.036 | OH | A01 | a1 | B02 | Br |
| 2-2-a1B02-1-0002 | 359.052 | OH | A02 | a1 | B02 | Br |

[Table 422]

| [Table 9-1-4] Compound that may be contained in mixture 2-2-a1B03-1 | | | | | | |
|---|---|---|---|---|---|---|
| ID | Exact Mass | | A | a | B | b |
| 2-2-a1B03-1-0001 | 337.011 | OH | A01 | a1 | B03 | Br |
| 2-2-a1B03-1-0002 | 363.027 | OH | A02 | a1 | B03 | Br |

[Table 423]

| [Table 9-1-5] Compound that may be contained in mixture 2-2-a1B04-1 | | | | | | |
|---|---|---|---|---|---|---|
| ID | Exact Mass | | A | a | B | b |
| 2-2-a1B04-1-0001 | 375.988 | OH | A01 | a1 | B04 | Br |
| 2-2-a1B04-1-0002 | 402.004 | OH | A02 | a1 | B04 | Br |

[Table 424]

| [Table 9-1-6] Compound that may be contained in mixture 2-2-a1B05-1 | | | | | | |
|---|---|---|---|---|---|---|
| ID | Exact Mass | | A | a | B | b |
| 2-2-a1B05-1-0001 | 387.008 | OH | A01 | a1 | B05 | Br |
| 2-2-a1B05-1-0002 | 413.024 | OH | A02 | a1 | B05 | Br |

Example 9-2: Formation of linkers b1 to b4 by step b

Example 9-2-0: Preparation of 2-2-C00-0 for use in step b

[Formula 657]

2-2-a1B01-0

2-2-a1B02-0

2-2-a1B03-0

2-2-a1B04-0

2-2-a1B05-0

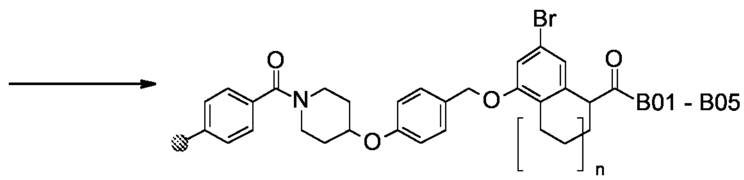

2-2-C00-0

n = 0, 1

5 types of mixtures described in Table 9-2-0 and DCM (70 mL) were added to a 120 mL column with a filter. 1 minute later, the suspension of the solvent and the solid phase was filtered through the filter of the column and washed once with heptane (70 mL), and the obtained solid phase was dried under reduced pressure to obtain mixture 2-2-C00-0 (3.67 g, 0.196 mmol/g).

[Table 425]

| [Table 9-2-0] | |
|---|---|
| Mixture | Amount used |
| 2-2-a1B01-0 | 0.700 g |
| 2-2-a1B02-0 | 0.700 g |
| 2-2-a1B03-0 | 0.700 g |
| 2-2-a1B04-0 | 0.700 g |
| 2-2-a1B05-0 | 0.700 g |

Example 9-2-1: Synthesis of mixtures 2-2-b1C01-0 to 2-2-b1C05-0 by step b1

[Formula 658]

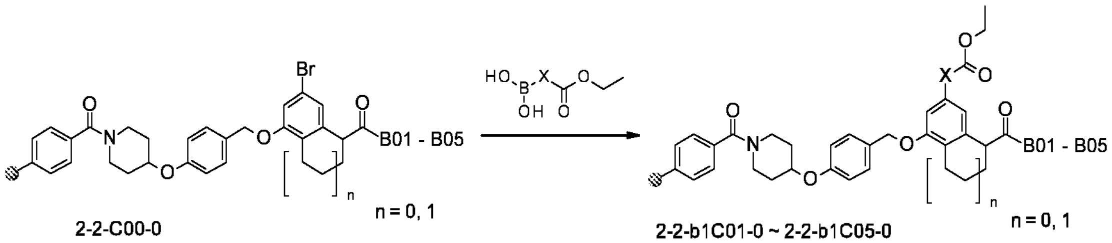


2-2-C00-0 2-2-b1C01-0 ~ 2-2-b1C05-0

Mixture 2-2-C00-0 (128 mg, 0.196 mmol/g), THF (2.56 mL), and water (6.79 μL, 0.377 mmol) were added to five 4 mL glass vials and shaken at room temperature for 1 hour. Separate boronic acids (0.251 mmol) described in Table 9-2-1 were added to the glass vials, respectively. meCgPPh Pd G4 (17.0 mg, 0.025 mmol) and BTMG (75.0 μL, 0.377 mmol) were added thereto, and each mixture was shaken at 60°C for 12 hours.

Solid-phase purification

The suspension of the reaction solution and the solid phase was transferred to a 6 mL column with a filter, filtered, and washed three times with NMP/water = 1/1 (2.6 mL), three times with NMP (2.6 mL), three times with 0.2 M NAC in NMP/water = 5/1 (2.6 mL), three times with 0.05 M $TBAHSO_4$ + DTBP in NMP (2.6 mL), three times with 0.05 M NMM in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain the mixtures described in Table 9-2-1.

[Table 426]

[Table 9-2-1]

| Compound No. of boronic acid | Boronic acid used | Molar number used | Amount used | No. of obtained mixture |
|---|---|---|---|---|
| BB2-2-06 | (5-Ethoxycarbonylfuran-2-yl)boronic acid | 0.251 mmol | 46.2 mg | 2-2-b1C01-0 |
| BB2-2-07 | (3-Ethoxycarbonylphenyl)boronic acid | 0.251 mmol | 48.8 mg | 2-2-b1C02-0 |
| BB2-2-08 | (4-Ethoxycarbonylphenyl)boronic acid | 0.251 mmol | 48.8 mg | 2-2-b1C03-0 |
| BB2-2-09 | (5-Ethoxycarbonylpyridin-3-yl)boronic acid | 0.251 mmol | 49.0 mg | 2-2-b1C04-0 |
| BB2-2-10 | (5-Ethoxycarbonylthiophen-2-yl)boronic acid | 0.251 mmol | 50.3 mg | 2-2-b1C05-0 |

Mixtures 2-2-b1C01-0 to 2-2-b1C05-0 can be treated by cleavage in the same manner as in step a1 to obtain mixtures 2-2-b1C01-1 to 2-2-b1C05-1. The details of the mixtures are shown in Table 9-2-2 to Table 9-2-6.

A notation method in each table will be described. For example, in Table 9-2-2, each compound that may be contained in 2-2-b1C01-1 is indicated by ID, and core blocks and linkers in combination in the structure of mixture 2-2-b1C01-1 are indicated by symbols or chemical formulas. The correspondence between the symbols and specific structures are shown in Figure 6.

In Table 9-2-2 to Table 9-2-6, "×" and "c" are represented by chemical formulas.

[Formula 659]

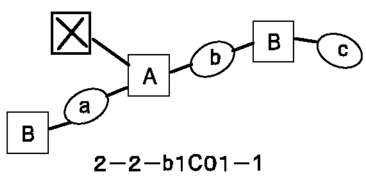


2−2−b1C01−1

For example, when ID is 2-2-b1C01-1-0001, the compound is represented by the following structural formula.

[Formula 660]

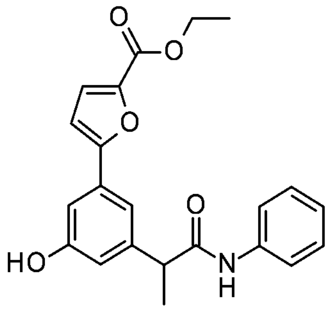

2-2-b1C01-1-0001

Exemplary notation of 2-2-b1C01-1-0001

[Table 427]

| ID | Exact Mass | | A | a | B | b | C | c |
|---|---|---|---|---|---|---|---|---|
| 2-2-b1C01-1-0001 | 379.142 | OH | A01 | a1 | B01 | b1 | C01 | COOEt |

[Table 428]

| [Table 9-2-2] Compound that may be contained in mixture 2-2-b1C01-1 | | | | | | | | |
|---|---|---|---|---|---|---|---|---|
| ID | Exact Mass | | A | a | B | b | C | c |
| 2-2-b1C01-1-0001 | 379.142 | OH | A01 | a1 | B01 | b1 | C01 | COOEt |
| 2-2-b1C01-1-0002 | 393.158 | OH | A01 | a1 | B02 | b1 | C01 | COOEt |
| 2-2-b1C01-1-0003 | 397.133 | OH | A01 | a1 | B03 | b1 | C01 | COOEt |
| 2-2-b1C01-1-0004 | 405.158 | OH | A02 | a1 | B01 | b1 | C01 | COOEt |
| 2-2-b1C01-1-0005 | 419.173 | OH | A02 | a1 | B02 | b1 | C01 | COOEt |
| 2-2-b1C01-1-0006 | 423.148 | OH | A02 | a1 | B03 | b1 | C01 | COOEt |
| 2-2-b1C01-1-0007 | 436.109 | OH | A01 | a1 | B04 | b1 | C01 | COOEt |
| 2-2-b1C01-1-0008 | 447.129 | OH | A01 | a1 | B05 | b1 | C01 | COOEt |
| 2-2-b1C01-1-0009 | 462.125 | OH | A02 | a1 | B04 | b1 | C01 | COOEt |
| 2-2-b1C01-1-0010 | 473.145 | OH | A02 | a1 | B05 | b1 | C01 | COOEt |

[Table 429]

| [Table 9-2-3] Compound that may be contained in mixture 2-2-b1C02-1 | | | | | | | | |
|---|---|---|---|---|---|---|---|---|
| ID | Exact Mass | | A | a | B | b | C | c |
| 2-2-b1C02-1-0001 | 389.163 | OH | A01 | a1 | B01 | b1 | C02 | COOEt |
| 2-2-b1C02-1-0002 | 403.178 | OH | A01 | a1 | B02 | b1 | C02 | COOEt |

(continued)

| [Table 9-2-3] Compound that may be contained in mixture 2-2-b1C02-1 | | | | | | | | |
|---|---|---|---|---|---|---|---|---|
| ID | Exact Mass | | A | a | B | b | C | c |
| 2-2-b1C02-1-0003 | 407.153 | OH | A01 | a1 | B03 | b1 | C02 | COOEt |
| 2-2-b1C02-1-0004 | 415.178 | OH | A02 | a1 | B01 | b1 | C02 | COOEt |
| 2-2-b1C02-1-0005 | 429.194 | OH | A02 | a1 | B02 | b1 | C02 | COOEt |
| 2-2-b1C02-1-0006 | 433.169 | OH | A02 | a1 | B03 | b1 | C02 | COOEt |
| 2-2-b1C02-1-0007 | 446.130 | OH | A01 | a1 | B04 | b1 | C02 | COOEt |
| 2-2-b1C02-1-0008 | 457.150 | OH | A01 | a1 | B05 | b1 | C02 | COOEt |
| 2-2-b1C02-1-0009 | 472.146 | OH | A02 | a1 | B04 | b1 | C02 | COOEt |
| 2-2-b1C02-1-0010 | 483.166 | OH | A02 | a1 | B05 | b1 | C02 | COOEt |

[Table 430]

| [Table 9-2-4] Compound that may be contained in mixture 2-2-b1C03-1 | | | | | | | | |
|---|---|---|---|---|---|---|---|---|
| ID | Exact Mass | | A | a | B | b | C | c |
| 2-2-b1C03-1-0001 | 389.163 | OH | A01 | a1 | B01 | b1 | C03 | COOEt |
| 2-2-b1C03-1-0002 | 403.178 | OH | A01 | a1 | B02 | b1 | C03 | COOEt |
| 2-2-b1C03-1-0003 | 407.153 | OH | A01 | a1 | B03 | b1 | C03 | COOEt |
| 2-2-b1C03-1-0004 | 415.178 | OH | A02 | a1 | B01 | b1 | C03 | COOEt |
| 2-2-b1C03-1-0005 | 429.194 | OH | A02 | a1 | B02 | b1 | C03 | COOEt |
| 2-2-b1C03-1-0006 | 433.169 | OH | A02 | a1 | B03 | b1 | C03 | COOEt |
| 2-2-b1C03-1-0007 | 446.130 | OH | A01 | a1 | B04 | b1 | C03 | COOEt |
| 2-2-b1C03-1-0008 | 457.150 | OH | A01 | a1 | B05 | b1 | C03 | COOEt |
| 2-2-b1C03-1-0009 | 472.146 | OH | A02 | a1 | B04 | b1 | C03 | COOEt |
| 2-2-b1C03-1-0010 | 483.166 | OH | A02 | a1 | B05 | b1 | C03 | COOEt |

[Table 431]

| [Table 9-2-5] Compound that may be contained in mixture 2-2-b1C04-1 | | | | | | | | |
|---|---|---|---|---|---|---|---|---|
| ID | Exact Mass | | A | a | B | b | C | c |
| 2-2-b1C04-1-0001 | 390.158 | OH | A01 | a1 | B01 | b1 | C04 | COOEt |
| 2-2-b1C04-1-0002 | 404.174 | OH | A01 | a1 | B02 | b1 | C04 | COOEt |
| 2-2-b1C04-1-0003 | 408.149 | OH | A01 | a1 | B03 | b1 | C04 | COOEt |
| 2-2-b1C04-1-0004 | 416.174 | OH | A02 | a1 | B01 | b1 | C04 | COOEt |
| 2-2-b1C04-1-0005 | 430.189 | OH | A02 | a1 | B02 | b1 | C04 | COOEt |
| 2-2-b1C04-1-0006 | 434.164 | OH | A02 | a1 | B03 | b1 | C04 | COOEt |
| 2-2-b1C04-1-0007 | 447.125 | OH | A01 | a1 | B04 | b1 | C04 | COOEt |
| 2-2-b1C04-1-0008 | 458.145 | OH | A01 | a1 | B05 | b1 | C04 | COOEt |
| 2-2-b1C04-1-0009 | 473.141 | OH | A02 | a1 | B04 | b1 | C04 | COOEt |
| 2-2-b1C04-1-0010 | 484.161 | OH | A02 | a1 | B05 | b1 | C04 | COOEt |

[Table 432]

| [Table 9-2-6] Compound that may be contained in mixture 2-2-b1C05-1 | | | | | | | | |
|---|---|---|---|---|---|---|---|---|
| ID | Exact Mass | | A | a | B | b | C | c |
| 2-2-b1C05-1-0001 | 395.119 | OH | A01 | a1 | B01 | b1 | C05 | COOEt |
| 2-2-b1C05-1-0002 | 409.135 | OH | A01 | a1 | B02 | b1 | C05 | COOEt |
| 2-2-b1C05-1-0003 | 413.110 | OH | A01 | a1 | B03 | b1 | C05 | COOEt |
| 2-2-b1C05-1-0004 | 421.135 | OH | A02 | a1 | B01 | b1 | C05 | COOEt |
| 2-2-b1C05-1-0005 | 435.150 | OH | A02 | a1 | B02 | b1 | C05 | COOEt |
| 2-2-b1C05-1-0006 | 439.125 | OH | A02 | a1 | B03 | b1 | C05 | COOEt |
| 2-2-b1C05-1-0007 | 452.086 | OH | A01 | a1 | B04 | b1 | C05 | COOEt |
| 2-2-b1C05-1-0008 | 463.107 | OH | A01 | a1 | B05 | b1 | C05 | COOEt |
| 2-2-b1C05-1-0009 | 478.102 | OH | A02 | a1 | B04 | b1 | C05 | COOEt |
| 2-2-b1C05-1-0010 | 489.122 | OH | A02 | a1 | B05 | b1 | C05 | COOEt |

Example 9-2-2: Synthesis of mixtures 2-2-b2C02-0 to 2-2-b2C04-0, 2-2-b2C06-0 and 2-2-b2C07-0 by step b2

[Formula 661]

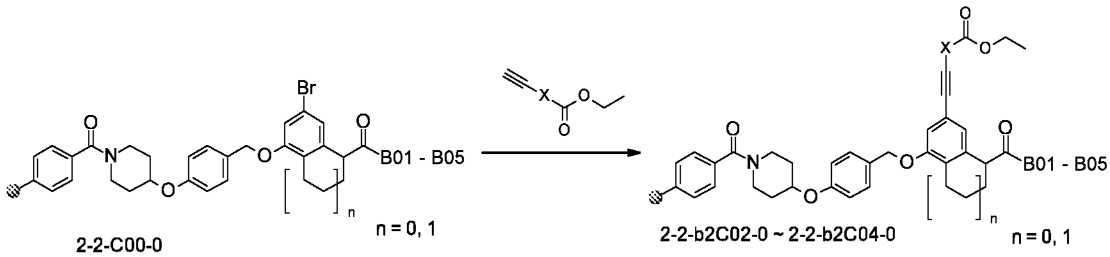

2-2-C00-0

2-2-b2C02-0 ~ 2-2-b2C04-0

2-2-b2C06-0, 2-2-b2C07-0

2-2-C00-0 (128 mg, 0.196 mmol/g) and NMP (2.56 mL) were added to five 4 mL glass vials and shaken at room temperature for 1 hour. Separate alkynes (0.251 mmol) described in Table 9-2-7 were added to the glass vials, respectively. XPhos Pd G4 (21.6 mg, 0.025 mmol) and diisopropylamine (53.7 μL, 0.377 mmol) were added thereto, and each mixture was shaken at 60°C for 12 hours.

Solid-phase purification

The suspension of the reaction solution and the solid phase was transferred to a 6 mL column with a filter, filtered, and washed three times with NMP/water = 1/1 (2.6 mL), three times with NMP (2.6 mL), three times with 0.2 M NAC in NMP/water = 5/1 (2.6 mL), three times with 0.05 M $TBAHSO_4$ + DTBP in NMP (2.6 mL), three times with 0.05 M NMM in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain the mixtures described in Table 9-2-7.

[Table 433]

[Table 9-2-7]

| Compound No. of alkyne | Alkyne used | Molar number used | Amount used | No. of obtained mixture |
|---|---|---|---|---|
| BB2-2-11 | Ethyl 3-ethynylbenzoate | 0.251 mmol | 43.8 mg | 2-2-b2C02-0 |
| BB2-2-12 | Ethyl 4-ethynylbenzoate | 0.251 mmol | 43.8 mg | 2-2-b2C03-0 |
| BB2-2-13 | Ethyl 5-ethynylpyridine-3-carboxylate | 0.251 mmol | 44.0 mg | 2-2-b2C04-0 |
| BB2-2-14 | Ethyl 6-ethynylpyridine-2-carboxylate | 0.251 mmol | 44.0 mg | 2-2-b2C06-0 |
| BB2-2-15 | Ethyl 4-ethynylpyridine-2-carboxylate | 0.251 mmol | 44.0 mg | 2-2-b2C07-0 |

Mixtures 2-2-b2C02-0 to 2-2-b2C04-0, 2-2-b2C06-0 and 2-2-b2C07-0 can be treated by cleavage in the same manner as in step a1 to obtain mixtures 2-2-b2C02-1 to 2-2- b2C04-1, 2-2-b2C06-1 and 2-2-b2C07-1. The details of the mixtures are shown in Table 9-2- 8 to Table 9-2-12.

A notation method in each table will be described. For example, in Table 9-2-8, each compound that may be contained in 2-2-b2C02-1 is indicated by ID, and core blocks and linkers in combination in the structure of mixture 2-2-b2C02-1 are indicated by symbols or chemical formulas. The correspondence between the symbols and specific structures are shown in Figure 6.

In Table 9-2-8 to Table 9-2-12, "×" and "c" are represented by chemical formulas.

[Formula 662]

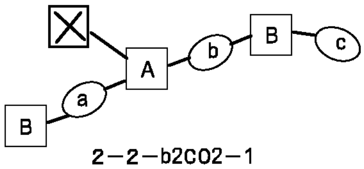


2−2−b2C02−1

[Table 434]

| [Table 9-2-8] Compound that may be contained in mixture 2-2-b2C02-1 | | | | | | | | |
|---|---|---|---|---|---|---|---|---|
| ID | Exact Mass | | A | a | B | b | C | c |
| 2-2-b2C02-1-0001 | 413.163 | OH | A01 | a1 | B01 | b2 | C02 | COOEt |
| 2-2-b2C02-1-0002 | 427.178 | OH | A01 | a1 | B02 | b2 | C02 | COOEt |
| 2-2-b2C02-1-0003 | 431.153 | OH | A01 | a1 | B03 | b2 | C02 | COOEt |
| 2-2-b2C02-1-0004 | 439.178 | OH | A02 | a1 | B01 | b2 | C02 | COOEt |
| 2-2-b2C02-1-0005 | 453.194 | OH | A02 | a1 | B02 | b2 | C02 | COOEt |
| 2-2-b2C02-1-0006 | 457.169 | OH | A02 | a1 | B03 | b2 | C02 | COOEt |
| 2-2-b2C02-1-0007 | 470.130 | OH | A01 | a1 | B04 | b2 | C02 | COOEt |
| 2-2-b2C02-1-0008 | 481.150 | OH | A01 | a1 | B05 | b2 | C02 | COOEt |
| 2-2-b2C02-1-0009 | 496.146 | OH | A02 | a1 | B04 | b2 | C02 | COOEt |
| 2-2-b2C02-1-0010 | 507.166 | OH | A02 | a1 | B05 | b2 | C02 | COOEt |

[Table 435]

| [Table 9-2-9] Compound that may be contained in mixture 2-2-b2C03-1 | | | | | | | | |
|---|---|---|---|---|---|---|---|---|
| ID | Exact Mass | | A | a | B | b | C | c |
| 2-2-b2C03-1-0001 | 413.163 | OH | A01 | a1 | B01 | b2 | C03 | COOEt |
| 2-2-b2C03-1-0002 | 427.178 | OH | A01 | a1 | B02 | b2 | C03 | COOEt |
| 2-2-b2C03-1-0003 | 431.153 | OH | A01 | a1 | B03 | b2 | C03 | COOEt |
| 2-2-b2C03-1-0004 | 439.178 | OH | A02 | a1 | B01 | b2 | C03 | COOEt |
| 2-2-b2C03-1-0005 | 453.194 | OH | A02 | a1 | B02 | b2 | C03 | COOEt |
| 2-2-b2C03-1-0006 | 457.169 | OH | A02 | a1 | B03 | b2 | C03 | COOEt |
| 2-2-b2C03-1-0007 | 470.130 | OH | A01 | a1 | B04 | b2 | C03 | COOEt |
| 2-2-b2C03-1-0008 | 481.150 | OH | A01 | a1 | B05 | b2 | C03 | COOEt |
| 2-2-b2C03-1-0009 | 496.146 | OH | A02 | a1 | B04 | b2 | C03 | COOEt |
| 2-2-b2C03-1-0010 | 507.166 | OH | A02 | a1 | B05 | b2 | C03 | COOEt |

[Table 436]

| [Table 9-2-10] Compound that may be contained in mixture 2-2-b2C04-1 | | | | | | | | |
|---|---|---|---|---|---|---|---|---|
| ID | Exact Mass | | A | a | B | b | C | c |
| 2-2-b2C04-1-0001 | 414.158 | OH | A01 | a1 | B01 | b2 | C04 | COOEt |
| 2-2-b2C04-1-0002 | 428.174 | OH | A01 | a1 | B02 | b2 | C04 | COOEt |
| 2-2-b2C04-1-0003 | 432.149 | OH | A01 | a1 | B03 | b2 | C04 | COOEt |
| 2-2-b2C04-1-0004 | 440.174 | OH | A02 | a1 | B01 | b2 | C04 | COOEt |
| 2-2-b2C04-1-0005 | 454.189 | OH | A02 | a1 | B02 | b2 | C04 | COOEt |
| 2-2-b2C04-1-0006 | 458.164 | OH | A02 | a1 | B03 | b2 | C04 | COOEt |
| 2-2-b2C04-1-0007 | 471.125 | OH | A01 | a1 | B04 | b2 | C04 | COOEt |
| 2-2-b2C04-1-0008 | 482.145 | OH | A01 | a1 | B05 | b2 | C04 | COOEt |
| 2-2-b2C04-1-0009 | 497.141 | OH | A02 | a1 | B04 | b2 | C04 | COOEt |
| 2-2-b2C04-1-0010 | 508.161 | OH | A02 | a1 | B05 | b2 | C04 | COOEt |

[Table 437]

| [Table 9-2-11] Compound that may be contained in mixture 2-2-b2C06-1 | | | | | | | | |
|---|---|---|---|---|---|---|---|---|
| ID | Exact Mass | | A | a | B | b | C | c |
| 2-2-b2C06-1-0001 | 414.158 | OH | A01 | a1 | B01 | b2 | C06 | COOEt |
| 2-2-b2C06-1-0002 | 428.174 | OH | A01 | a1 | B02 | b2 | C06 | COOEt |
| 2-2-b2C06-1-0003 | 432.149 | OH | A01 | a1 | B03 | b2 | C06 | COOEt |
| 2-2-b2C06-1-0004 | 440.174 | OH | A02 | a1 | B01 | b2 | C06 | COOEt |
| 2-2-b2C06-1-0005 | 454.189 | OH | A02 | a1 | B02 | b2 | C06 | COOEt |
| 2-2-b2C06-1-0006 | 458.164 | OH | A02 | a1 | B03 | b2 | C06 | COOEt |

(continued)

| [Table 9-2-11] Compound that may be contained in mixture 2-2-b2C06-1 | | | | | | | | |
|---|---|---|---|---|---|---|---|---|
| ID | Exact Mass | | A | a | B | b | C | c |
| 2-2-b2C06-1-0007 | 471.125 | OH | A01 | a1 | B04 | b2 | C06 | COOEt |
| 2-2-b2C06-1-0008 | 482.145 | OH | A01 | a1 | B05 | b2 | C06 | COOEt |
| 2-2-b2C06-1-0009 | 497.141 | OH | A02 | a1 | B04 | b2 | C06 | COOEt |
| 2-2-b2C06-1-0010 | 508.161 | OH | A02 | a1 | B05 | b2 | C06 | COOEt |

[Table 438]

| [Table 9-2-12] Compound that may be contained in mixture 2-2-b2C07-1 | | | | | | | | |
|---|---|---|---|---|---|---|---|---|
| ID | Exact Mass | | A | a | B | b | C | c |
| 2-2-b2C07-1-0001 | 414.158 | OH | A01 | a1 | B01 | b2 | C07 | COOEt |
| 2-2-b2C07-1-0002 | 428.174 | OH | A01 | a1 | B02 | b2 | C07 | COOEt |
| 2-2-b2C07-1-0003 | 432.149 | OH | A01 | a1 | B03 | b2 | C07 | COOEt |
| 2-2-b2C07-1-0004 | 440.174 | OH | A02 | a1 | B01 | b2 | C07 | COOEt |
| 2-2-b2C07-1-0005 | 454.189 | OH | A02 | a1 | B02 | b2 | C07 | COOEt |
| 2-2-b2C07-1-0006 | 458.164 | OH | A02 | a1 | B03 | b2 | C07 | COOEt |
| 2-2-b2C07-1-0007 | 471.125 | OH | A01 | a1 | B04 | b2 | C07 | COOEt |
| 2-2-b2C07-1-0008 | 482.145 | OH | A01 | a1 | B05 | b2 | C07 | COOEt |
| 2-2-b2C07-1-0009 | 497.141 | OH | A02 | a1 | B04 | b2 | C07 | COOEt |
| 2-2-b2C07-1-0010 | 508.161 | OH | A02 | a1 | B05 | b2 | C07 | COOEt |

Example 9-2-3: Synthesis of mixture 2-2-b3C11-0 by step b3

[Formula 663]

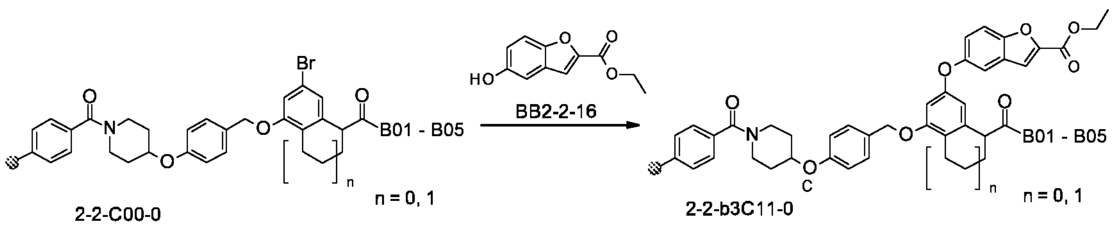


2-2-C00-0 2-2-b3C11-0

2-2-C00-0 (128 mg, 0.196 mmol/g) and toluene (2.56 mL) were added to a 4 mL glass vial and shaken at room temperature for 1 hour. Ethyl 5-hydroxybenzofuran-2- carboxylate (BB2-2-16) (51.8 mg, 0.251 mmol) was added thereto. AdBippyPhos (33.3 mg, 0.050 mmol), $Pd_2(dba)_3CHCl_3$ (13.0 mg, 0.013 mmol), and $K_3PO_4$ (80.0 mg, 0.377 mmol) were added thereto, and the mixture was shaken at 100°C for 6 hours.

Solid-phase purification

The suspension of the reaction solution and the solid phase was transferred to a 6 mL column with a filter, filtered, and washed three times with NMP/water = 1/1 (2.6 mL), three times with NMP (2.6 mL), three times with 0.2 M NAC in NMP/water = 5/1 (2.6 mL), three times with 0.05 M $TBAHSO_4$ + DTBP in NMP (2.6 mL), three times with 0.05

M NMM in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain mixture 2-2-b3C11-0.

Mixture 2-2-b3C11-0 can be treated by cleavage in the same manner as in step a1 to obtain mixture 2-2-b3C11-1. The details of the mixture are shown in Table 9-2-13.

A notation method in each table will be described. For example, in Table 9-2-13, each compound that may be contained in 2-2-b3C11-1 is indicated by ID, and core blocks and linkers in combination in the structure of mixture 2-2-b3C11-1 are indicated by symbols or chemical formulas. The correspondence between the symbols and specific structures are shown in Figure 6.

In Table 9-2-13, "×" and "c" are represented by chemical formulas.

[Formula 664]

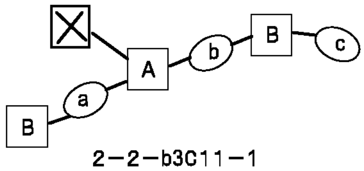


2−2−b3C11−1

[Table 439]

| [Table 9-2-13] Compound that may be contained in mixture 2-2-b3C11-1 | | | | | | | | |
|---|---|---|---|---|---|---|---|---|
| ID | Exact Mass | | A | a | B | b | C | c |
| 2-2-b3C11-1-0001 | 445.153 | OH | A01 | a1 | B01 | b3 | C11 | COOEt |
| 2-2-b3C11-1-0002 | 459.168 | OH | A01 | a1 | B02 | b3 | C11 | COOEt |
| 2-2-b3C11-1-0003 | 463.143 | OH | A01 | a1 | B03 | b3 | C11 | COOEt |
| 2-2-b3C11-1-0004 | 471.168 | OH | A02 | a1 | B01 | b3 | C11 | COOEt |
| 2-2-b3C11-1-0005 | 485.184 | OH | A02 | a1 | B02 | b3 | C11 | COOEt |
| 2-2-b3C11-1-0006 | 489.159 | OH | A02 | a1 | B03 | b3 | C11 | COOEt |
| 2-2-b3C11-1-0007 | 502.120 | OH | A01 | a1 | B04 | b3 | C11 | COOEt |
| 2-2-b3C11-1-0008 | 513.140 | OH | A01 | a1 | B05 | b3 | C11 | COOEt |
| 2-2-b3C11-1-0009 | 528.136 | OH | A02 | a1 | B04 | b3 | C11 | COOEt |
| 2-2-b3C11-1-0010 | 539.156 | OH | A02 | a1 | B05 | b3 | C11 | COOEt |

Example 9-2-4: Synthesis of mixture 2-2-b3C00-0 for use in steps b3C08 to b3C10

[Formula 665]

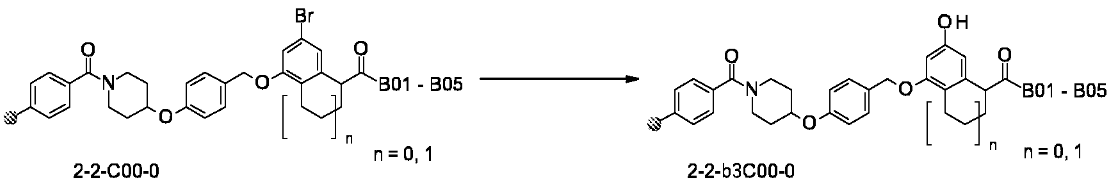

2-2-C00-0 2-2-b3C00-0

Mixture 2-2-C00-0 (550 mg, 0.196 mmol/g), DEAc (11.0 mL), and water (0.275 mL, 15.3 mmol) were added to a 15 mL glass vial and shaken at room temperature for 1 hour. AdBrettPhos Pd G6 Br (21.0 mg, 0.022 mmol) and BTMG (0.275 mL, 1.38 mmol) were added thereto, and the mixture was shaken at 60°C for 15 hours.

Solid-phase purification

The suspension of the reaction solution and the solid phase was transferred to a column with a filter, filtered,

and washed three times with NMP (11 mL), three times with 0.2 M NAC in NMP/water = 5/1 (11 mL), three times with 0.05 M $TBAHSO_4$ + DTBP in NMP (11 mL), three times with 0.05 M NMM in NMP (11 mL), three times with NMP/water = 1/1 (11 mL), three times with NMP (11 mL), three times with MeOH (11 mL), three times with DCM (11 mL), and once with heptane (11 mL), and the obtained solid phase was dried under reduced pressure to obtain mixture 2-2-b3C00-0.

Example 9-2-5: Synthesis of mixtures 2-2-b3C08-0 to 2-2-b3C10-0 by steps b3C08 to b3C10

[Formula 666]

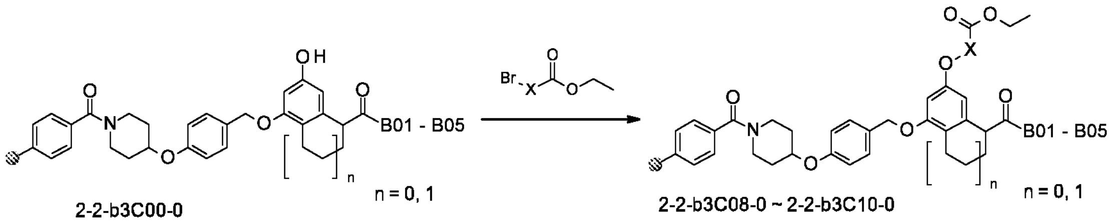

2-2-b3C00-0

2-2-b3C08-0 ~ 2-2-b3C10-0

Mixture 2-2-C00-0 (128 mg, 0.199 mmol/g) and DME (2.56 mL) were added to three 4 mL glass vials and shaken at room temperature for 1 hour. Separate aryl bromides (0.254 mmol) described in Table 9-2-14 were added to the glass vials, respectively. GPhos (27.3 mg, 0.051 mmol), $Pd_2(dba)_3CHCl_3$ (13.2 mg, 0.013 mmol), KOTf(71.8 mg, 0.382 mmol), and BTMG (76.0 μL, 0.382 mmol) were added thereto, and each mixture was shaken at 80°C for 12 hours.

Solid-phase purification

The suspension of the reaction solution and the solid phase was transferred to a 6 mL column with a filter, filtered, and washed three times with NMP/water = 1/1 (2.6 mL), three times with NMP (2.6 mL), three times with 0.2 M NAC in NMP/water = 5/1 (2.6 mL), three times with 0.05 M $TBAHSO_4$ + DTBP in NMP (2.6 mL), three times with 0.05 M NMM in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain the mixtures described in Table 9-2-14.

[Table 440]

[Table 9-2-14]

| Compound No. of aryl bromide | Aryl bromide used | Molar number used | Amount used | No. of obtained mixture |
|---|---|---|---|---|
| BB-2-2-17 | Ethyl 2-bromo-1,3-oxazole-4-carboxylate | 0.254 mmol | 56.0 mg | 2-2-b3C08-0 |
| BB-2-2-18 | Ethyl 2-bromopyrimidine-4-carboxylate | 0.254 mmol | 58.8 mg | 2-2-b3C09-0 |
| BB-2-2-19 | Ethyl 2-bromo-1,3-thiazole-4-carboxylate | 0.254 mmol | 60.1 mg | 2-2-b3C10-0 |

Mixtures 2-2-b3C08-0 to 2-2-b3C10-0 can be treated by cleavage in the same manner as in step a1 to obtain mixtures 2-2-b3C08-1 to 2-2-b3C10-1. The details of the mixtures are shown in Table 9-2-15 to Table 9-2-17.

A notation method in each table will be described. For example, in Table 9-2-15, each compound that may be contained in 2-2-b3C08-1 is indicated by ID, and core blocks and linkers in combination in the structure of mixture 2-2-b3C08-1 are indicated by symbols or chemical formulas. The correspondence between the symbols and specific structures are shown in Figure 6.

In Table 9-2-15 to Table 9-2-17, "×" and "c" are represented by chemical formulas.

[Formula 667]

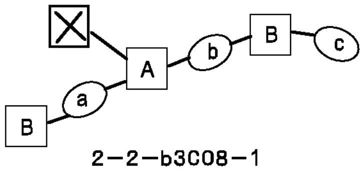

2-2-b3C08-1

[Table 441]

| [Table 9-2-15] Compound that may be contained in mixture 2-2-b3C08-1 | | | | | | | | |
|---|---|---|---|---|---|---|---|---|
| ID | Exact Mass | | A | a | B | b | C | c |
| 2-2-b3C08-1-0001 | 396.132 | OH | A01 | a1 | B01 | b3 | C08 | COOEt |
| 2-2-b3C08-1-0002 | 410.148 | OH | A01 | a1 | B02 | b3 | C08 | COOEt |
| 2-2-b3C08-1-0003 | 414.123 | OH | A01 | a1 | B03 | b3 | C08 | COOEt |
| 2-2-b3C08-1-0004 | 422.148 | OH | A02 | a1 | B01 | b3 | C08 | COOEt |
| 2-2-b3C08-1-0005 | 436.163 | OH | A02 | a1 | B02 | b3 | C08 | COOEt |
| 2-2-b3C08-1-0006 | 440.138 | OH | A02 | a1 | B03 | b3 | C08 | COOEt |
| 2-2-b3C08-1-0007 | 453.099 | OH | A01 | a1 | B04 | b3 | C08 | COOEt |
| 2-2-b3C08-1-0008 | 464.1280 | OH | A01 | a1 | B05 | b3 | C08 | COOEt |
| 2-2-b3C08-1-0009 | 479.115 | OH | A02 | a1 | B04 | b3 | C08 | COOEt |
| 2-2-b3C08-1-0010 | 490.135 | OH | A02 | a1 | B05 | b3 | C08 | COOEt |

[Table 442]

| [Table 9-2-16] Compound that may be contained in mixture 2-2-b3C09-1 | | | | | | | | |
|---|---|---|---|---|---|---|---|---|
| ID | Exact Mass | | A | a | B | b | C | c |
| 2-2-b3C09-1-0001 | 407.148 | OH | A01 | a1 | B01 | b3 | C09 | COOEt |
| 2-2-b3C09-1-0002 | 421.164 | OH | A01 | a1 | B02 | b3 | C09 | COOEt |
| 2-2-b3C09-1-0003 | 425.139 | OH | A01 | a1 | B03 | b3 | C09 | COOEt |
| 2-2-b3C09-1-0004 | 433.164 | OH | A02 | a1 | B01 | b3 | C09 | COOEt |
| 2-2-b3C09-1-0005 | 447.179 | OH | A02 | a1 | B02 | b3 | C09 | COOEt |
| 2-2-b3C09-1-0006 | 451.154 | OH | A02 | a1 | B03 | b3 | C09 | COOEt |
| 2-2-b3C09-1-0007 | 464.115 | OH | A01 | a1 | B04 | b3 | C09 | COOEt |
| 2-2-b3C09-1-0008 | 475.136 | OH | A01 | a1 | B05 | b3 | C09 | COOEt |
| 2-2-b3C09-1-0009 | 490.131 | OH | A02 | a1 | B04 | b3 | C09 | COOEt |
| 2-2-b3C09-1-0010 | 501.151 | OH | A02 | a1 | B05 | b3 | C09 | COOEt |

[Table 443]

| [Table 9-2-17] Compound that may be contained in mixture 2-2-b3C10-1 | | | | | | | | |
|---|---|---|---|---|---|---|---|---|
| ID | Exact Mass | | A | a | B | b | C | c |
| 2-2-b3C10-1-0001 | 412.109 | OH | A01 | a1 | B01 | b3 | C10 | COOEt |
| 2-2-b3C10-1-0002 | 426.125 | OH | A01 | a1 | B02 | b3 | C10 | COOEt |
| 2-2-b3C10-1-0003 | 430.100 | OH | A01 | a1 | B03 | b3 | C10 | COOEt |
| 2-2-b3C10-1-0004 | 438.125 | OH | A02 | a1 | B01 | b3 | C10 | COOEt |
| 2-2-b3C10-1-0005 | 452.141 | OH | A02 | a1 | B02 | b3 | C10 | COOEt |
| 2-2-b3C10-1-0006 | 456.116 | OH | A02 | a1 | B03 | b3 | C10 | COOEt |
| 2-2-b3C10-1-0007 | 469.077 | OH | A01 | a1 | B04 | b3 | C10 | COOEt |
| 2-2-b3C10-1-0008 | 480.097 | OH | A01 | a1 | B05 | b3 | C10 | COOEt |
| 2-2-b3C10-1-0009 | 495.092 | OH | A02 | a1 | B04 | b3 | C10 | COOEt |
| 2-2-b3C10-1-0010 | 506.112 | OH | A02 | a1 | B05 | b3 | C10 | COOEt |

Example 9-2-6: Synthesis of mixtures 2-2-b4C01-0, 2-2-b4C02-0, 2-2-b4C05-0, 2- 2-b4C12-0 and 2-2-b4C13-0 by step b4

[Formula 668]

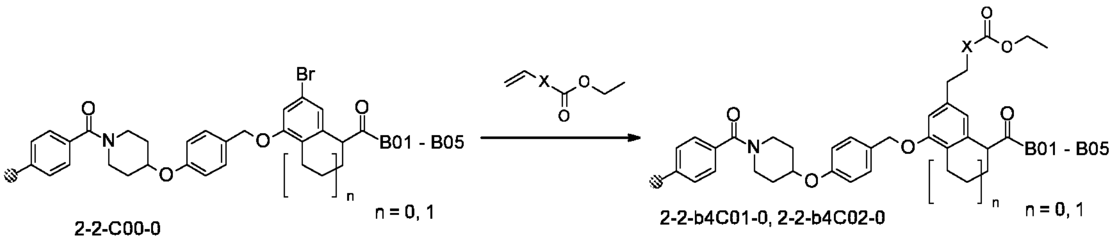

2-2-C00-0

2-2-b4C01-0, 2-2-b4C02-0
2-2-b4C05-0, 2-2-b4C12-0, 2-2-b4C13-0

2-2-C00-0 (128 mg, 0.196 mmol/g), THF (2.56 mL), and water (6.79 μL, 0.377 mmol) were added to five 4 mL glass vials and shaken at room temperature for 1 hour. Separate solutions of alkene compounds (0.251 mmol) described in Table 2-3-18 and 0.5 M 9-BBN in THF (0.503 mL, 0.251 mmol) left standing for 1 hour were added to the glass vials, respectively. (dtbpf)$PdCl_2$ (16.4 mg, 0.025 mmol) and BTMG (75.0 μL, 0.377 mmol) were added thereto, and each mixture was shaken at 60°C for 12 hours.

Solid-phase purification

The suspension of the reaction solution and the solid phase was transferred to a 6 mL column with a filter, filtered, and washed three times with NMP/water = 1/1 (2.6 mL), three times with NMP (2.6 mL), three times with 0.2 M NAC in NMP/water = 5/1 (2.6 mL), three times with 0.05 M $TBAHSO_4$ + DTBP in NMP (2.6 mL), three times with 0.05 M NMM in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain the mixtures described in Table 2-3-18.

[Table 444]

[Table 2-3-18]

| No. of alkene compound | Alkene compound used | Molar number used | Amount used | No. of obtained mixture |
|---|---|---|---|---|
| BB2-2-20 | Ethyl 5-prop-2-enylfuran-2-carboxylate | 0.251 mmol | 45.3 mg | 2-2-b4C01-0 |
| BB2-2-21 | Ethyl 3-prop-2-enylbenzoate | 0.251 mmol | 47.8 mg | 2-2-b4C02-0 |
| BB2-2-22 | Ethyl 5-prop-2-enylthiophene-2-carboxylate | 0.251 mmol | 49.3 mg | 2-2-b4C05-0 |
| BB2-2-23 | Ethyl 2-methylpent-4-enoate | 0.251 mmol | 35.7 mg | 2-2-b4C12-0 |
| BB2-2-24 | Ethyl 2-prop-2-enoxyacetate | 0.251 mmol | 36.2 mg | 2-2-b4C13-0 |

Mixtures 2-2-b4C01-0, 2-2-b4C02-0, 2-2-b4C05-0, 2-2-b4C12-0, 2-2-b4C13-0 can be treated by cleavage in the same manner as in step a1 to obtain mixtures 2-2-b4C01-1, 2-2- b4C02-1, 2-2-b4C05-1, 2-2-b4C12-1, 2-2-b4C13-1. The details of the mixtures are shown in Table 9-2-19 to Table 9-2-23.

A notation method in each table will be described. For example, in Table 9-2-19, each compound that may be contained in 2-2-b4C01-1 is indicated by ID, and core blocks and linkers in combination in the structure of mixture 2-2-b4C01-1 are indicated by symbols or chemical formulas. The correspondence between the symbols and specific structures are shown in Figure 6.

In Table 9-2-19 to Table 9-2-23, "×" and "c" are represented by chemical formulas.

[Formula 669]

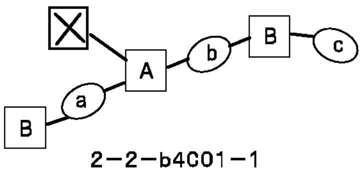


2-2-b4C01-1

[Table 445]

| [Table 9-2-19] Compound that may be contained in mixture 2-2-b4C01-1 | | | | | | | | |
|---|---|---|---|---|---|---|---|---|
| ID | Exact Mass | | A | a | B | b | C | c |
| 2-2-b4C01-1-0001 | 421.189 | OH | A01 | a1 | B01 | b4 | C01 | COOEt |
| 2-2-b4C01-1-0002 | 435.205 | OH | A01 | a1 | B02 | b4 | C01 | COOEt |
| 2-2-b4C01-1-0003 | 439.180 | OH | A01 | a1 | B03 | b4 | C01 | COOEt |
| 2-2-b4C01-1-0004 | 447.205 | OH | A02 | a1 | B01 | b4 | C01 | COOEt |
| 2-2-b4C01-1-0005 | 461.220 | OH | A02 | a1 | B02 | b4 | C01 | COOEt |
| 2-2-b4C01-1-0006 | 465.195 | OH | A02 | a1 | B03 | b4 | C01 | COOEt |
| 2-2-b4C01-1-0007 | 478.156 | OH | A01 | a1 | B04 | b4 | C01 | COOEt |
| 2-2-b4C01-1-0008 | 489.176 | OH | A01 | a1 | B05 | b4 | C01 | COOEt |
| 2-2-b4C01-1-0009 | 504.172 | OH | A02 | a1 | B04 | b4 | C01 | COOEt |
| 2-2-b4C01-1-0010 | 515.192 | OH | A02 | a1 | B05 | b4 | C01 | COOEt |

[Table 446]

| [Table 9-2-20] Compound that may be contained in mixture 2-2-b4C02-1 | | | | | | | | |
|---|---|---|---|---|---|---|---|---|
| ID | Exact Mass | | A | a | B | b | C | c |
| 2-2-b4C02-1-0001 | 431.210 | OH | A01 | a1 | B01 | b4 | C02 | COOEt |
| 2-2-b4C02-1-0002 | 445.225 | OH | A01 | a1 | B02 | b4 | C02 | COOEt |
| 2-2-b4C02-1-0003 | 449.200 | OH | A01 | a1 | B03 | b4 | C02 | COOEt |
| 2-2-b4C02-1-0004 | 457.225 | OH | A02 | a1 | B01 | b4 | C02 | COOEt |
| 2-2-b4C02-1-0005 | 471.241 | OH | A02 | a1 | B02 | b4 | C02 | COOEt |
| 2-2-b4C02-1-0006 | 475.216 | OH | A02 | a1 | B03 | b4 | C02 | COOEt |
| 2-2-b4C02-1-0007 | 488.177 | OH | A01 | a1 | B04 | b4 | C02 | COOEt |
| 2-2-b4C02-1-0008 | 499.197 | OH | A01 | a1 | B05 | b4 | C02 | COOEt |
| 2-2-b4C02-1-0009 | 514.193 | OH | A02 | a1 | B04 | b4 | C02 | COOEt |
| 2-2-b4C02-1-0010 | 525.213 | OH | A02 | a1 | B05 | b4 | C02 | COOEt |

[Table 447]

| [Table 9-2-21] Compound that may be contained in mixture 2-2-b4C05-1 | | | | | | | | |
|---|---|---|---|---|---|---|---|---|
| ID | Exact Mass | | A | a | B | b | C | c |
| 2-2-b4C05-1-0001 | 437.166 | OH | A01 | a1 | B01 | b4 | C05 | COOEt |
| 2-2-b4C05-1-0002 | 451.182 | OH | A01 | a1 | B02 | b4 | C05 | COOEt |
| 2-2-b4C05-1-0003 | 455.157 | OH | A01 | a1 | B03 | b4 | C05 | COOEt |
| 2-2-b4C05-1-0004 | 463.182 | OH | A02 | a1 | B01 | b4 | C05 | COOEt |
| 2-2-b4C05-1-0005 | 477.197 | OH | A02 | a1 | B02 | b4 | C05 | COOEt |
| 2-2-b4C05-1-0006 | 481.172 | OH | A02 | a1 | B03 | b4 | C05 | COOEt |
| 2-2-b4C05-1-0007 | 494.133 | OH | A01 | a1 | B04 | b4 | C05 | COOEt |
| 2-2-b4C05-1-0008 | 505.153 | OH | A01 | a1 | B05 | b4 | C05 | COOEt |
| 2-2-b4C05-1-0009 | 520.149 | OH | A02 | a1 | B04 | b4 | C05 | COOEt |
| 2-2-b4C05-1-0010 | 531.169 | OH | A02 | a1 | B05 | b4 | C05 | COOEt |

[Table 448]

| [Table 9-2-22] Compound that may be contained in mixture 2-2-b4C12-1 | | | | | | | | |
|---|---|---|---|---|---|---|---|---|
| ID | Exact Mass | | A | a | B | b | C | c |
| 2-2-b4C12-1-0001 | 383.210 | OH | A01 | a1 | B01 | b4 | C12 | COOEt |
| 2-2-b4C12-1-0002 | 397.225 | OH | A01 | a1 | B02 | b4 | C12 | COOEt |
| 2-2-b4C12-1-0003 | 401.200 | OH | A01 | a1 | B03 | b4 | C12 | COOEt |
| 2-2-b4C12-1-0004 | 409.225 | OH | A02 | a1 | B01 | b4 | C12 | COOEt |
| 2-2-b4C12-1-0005 | 423.241 | OH | A02 | a1 | B02 | b4 | C12 | COOEt |
| 2-2-b4C12-1-0006 | 427.216 | OH | A02 | a1 | B03 | b4 | C12 | COOEt |
| 2-2-b4C12-1-0007 | 440.177 | OH | A01 | a1 | B04 | b4 | C12 | COOEt |

(continued)

| [Table 9-2-22] Compound that may be contained in mixture 2-2-b4C12-1 | | | | | | | | |
|---|---|---|---|---|---|---|---|---|
| ID | Exact Mass | | A | a | B | b | C | c |
| 2-2-b4C12-1-0008 | 451.197 | OH | A01 | a1 | B05 | b4 | C12 | COOEt |
| 2-2-b4C12-1-0009 | 466.193 | OH | A02 | a1 | B04 | b4 | C12 | COOEt |
| 2-2-b4C12-1-0010 | 477.213 | OH | A02 | a1 | B05 | b4 | C12 | COOEt |

[Table 449]

| [Table 9-2-23] Compound that may be contained in mixture 2-2-b4C13-1 | | | | | | | | |
|---|---|---|---|---|---|---|---|---|
| ID | Exact Mass | | A | a | B | b | C | c |
| 2-2-b4C13-1-0001 | 385.189 | OH | A01 | a1 | B01 | b4 | C13 | COOEt |
| 2-2-b4C13-1-0002 | 399.205 | OH | A01 | a1 | B02 | b4 | C13 | COOEt |
| 2-2-b4C13-1-0003 | 403.180 | OH | A01 | a1 | B03 | b4 | C13 | COOEt |
| 2-2-b4C13-1-0004 | 411.205 | OH | A02 | a1 | B01 | b4 | C13 | COOEt |
| 2-2-b4C13-1-0005 | 425.220 | OH | A02 | a1 | B02 | b4 | C13 | COOEt |
| 2-2-b4C13-1-0006 | 429.195 | OH | A02 | a1 | B03 | b4 | C13 | COOEt |
| 2-2-b4C13-1-0007 | 442.156 | OH | A01 | a1 | B04 | b4 | C13 | COOEt |
| 2-2-b4C13-1-0008 | 453.176 | OH | A01 | a1 | B05 | b4 | C13 | COOEt |
| 2-2-b4C13-1-0009 | 468.172 | OH | A02 | a1 | B04 | b4 | C13 | COOEt |
| 2-2-b4C13-1-0010 | 479.192 | OH | A02 | a1 | B05 | b4 | C13 | COOEt |

Example 9-2-7: Synthesis of mixture 2-2-b1C15-0 by step b1C15

[Formula 670]

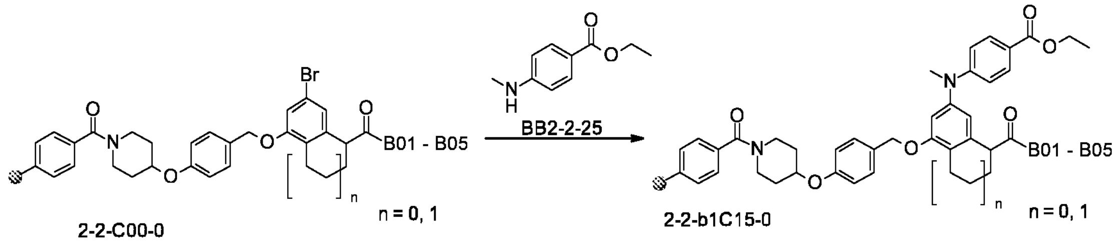


2-2-C00-0

2-2-b1C15-0

2-2-C00-0 (128 mg, 0.196 mmol/g) and THF (2.56 mL) were added to a 4 mL glass vial and shaken at room temperature for 1 hour. Ethyl 4-(methylamino)benzoate (BB2-2-25) (45.0 mg, 0.251 mmol) was added thereto. (tBu)Ph-CPhos Pd G4 (19.8 mg, 0.025 mmol) and 2 M $P_2$tBu in THF (0.189 mL, 0.377 mmol) were added thereto, and the mixture was shaken at 60°C for 6 hours.

Solid-phase purification

The suspension of the reaction solution and the solid phase was transferred to a 6 mL column with a filter, filtered, and washed three times with NMP/water = 1/1 (2.6 mL), three times with NMP (2.6 mL), three times with 0.2 M NAC in NMP/water = 5/1 (2.6 mL), three times with 0.05 M $TBAHSO_4$ + DTBP in NMP (2.6 mL), three times with 0.05 M NMM in NMP (2.6 mL), three times with NMP/water = 1/1 (2.6 mL), three times with NMP (2.6 mL), three times with

MeOH (2.8 mL), three times with DCM (2.8 mL), and three times with heptane (2.8 mL), and the obtained solid phase was dried under reduced pressure to obtain mixture 2-2-b1C15-0.

Mixture 2-2-b1C15-0 can be treated by cleavage in the same manner as in step a1 to obtain mixture 2-2-b1C15-1. The details of the mixture are shown in Table 9-2-24.

A notation method in each table will be described. For example, in Table 9-2-24, each compound that may be contained in 2-2-b1C15-1 is indicated by ID, and core blocks and linkers in combination in the structure of mixture 2-2-b1C15-1 are indicated by symbols or chemical formulas. The correspondence between the symbols and specific structures are shown in Figure 6.

In Table 9-2-24, "×" and "c" are represented by chemical formulas.

[Formula 671]

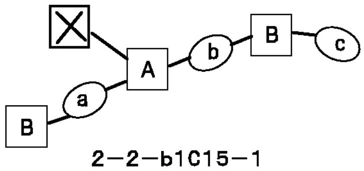


2−2−b1C15−1

[Table 450]

| [Table 9-2-24] Compound that may be contained in mixture 2-2-b1C15-1 | | | | | | | | |
|---|---|---|---|---|---|---|---|---|
| ID | Exact Mass | | A | a | B | b | C | c |
| 2-2-b1C15-1-0001 | 418.189 | OH | A01 | a1 | B01 | b1 | C15 | COOEt |
| 2-2-b1C15-1-0002 | 432.205 | OH | A01 | a1 | B02 | b1 | C15 | COOEt |
| 2-2-b1C15-1-0003 | 436.180 | OH | A01 | a1 | B03 | b1 | C15 | COOEt |
| 2-2-b1C15-1-0004 | 444.205 | OH | A02 | a1 | B01 | b1 | C15 | COOEt |
| 2-2-b1C15-1-0005 | 458.221 | OH | A02 | a1 | B02 | b1 | C15 | COOEt |
| 2-2-b1C15-1-0006 | 462.195 | OH | A02 | a1 | B03 | b1 | C15 | COOEt |
| 2-2-b1C15-1-0007 | 475.157 | OH | A01 | a1 | B04 | b1 | C15 | COOEt |
| 2-2-b1C15-1-0008 | 486.177 | OH | A01 | a1 | B05 | b1 | C15 | COOEt |
| 2-2-b1C15-1-0009 | 501.172 | OH | A02 | a1 | B04 | b1 | C15 | COOEt |
| 2-2-b1C15-1-0010 | 512.192 | OH | A02 | a1 | B05 | b1 | C15 | COOEt |

Example 9-2-8: Synthesis of mixtures 2-2-b1C14-0, 2-2-b1C16-0 to 2-2-b1C18-0 by step b1

[Formula 672]

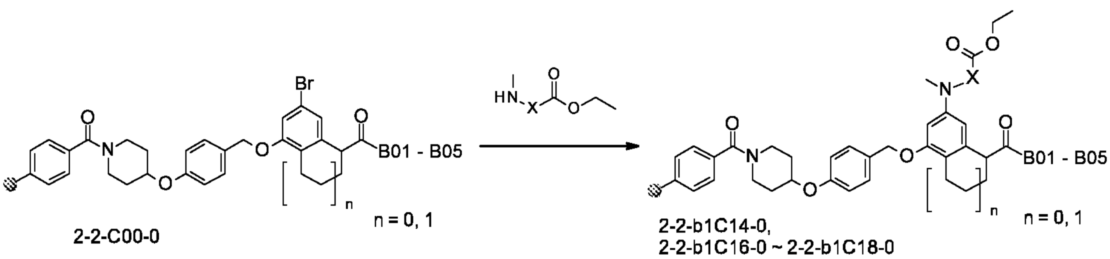

2-2-C00-0

2-2-b1C14-0,
2-2-b1C16-0 ~ 2-2-b1C18-0

2-2-C00-0 (128 mg, 0.196 mmol/g) and THF (2.56 mL) were added to four 4 mL glass vials and shaken at room temperature for 1 hour. Separate amines (0.251 mmol) described in Table 9-2-25 were added to the glass vials, respectively. AdBippyPhos (33.3 mg, 0.050 mmol), $Pd_2(dba)_3CHCl_3$ (13.0 mg, 0.013 mmol), KOTf (23.7 mg, 0.126 mmol), and BTMG (75.0 μL, 0.377 mmol) were added thereto, and the mixture was shaken at 60°C for 6 hours.

Solid-phase purification

The suspension of the reaction solution and the solid phase was transferred to a 6 mL column with a filter, filtered, and washed three times with NMP/water = 1/1 (2.6 mL), three times with NMP (2.6 mL), three times with 0.2 M NAC in NMP/water = 5/1 (2.6 mL), three times with 0.05 M $TBAHSO_4$ + DTBP in NMP (2.6 mL), three times with 0.05 M NMM in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain the mixtures described in Table 9-2-25.

[Table 451]

[Table 9-2-25]

| Compound No. of amine | Amine used | Molar number used | Amount used | No. of obtained mixture |
|---|---|---|---|---|
| BB2-2-26 | Ethyl 2-(methylamino)-1,3-oxazole-4-carboxylate | 0.251 mmol | 42.8 mg | 2-2-b1C14-0 |
| BB2-2-27 | Ethyl 6-(methylamino)pyridine-2-carboxylate | 0.251 mmol | 45.3 mg | 2-2-b1C16-0 |
| BB2-2-28 | Ethyl 6-(methylamino)pyridazine-3-carboxylate | 0.251 mmol | 45.5 mg | 2-2-b1C17-0 |
| BB2-2-29 | Ethyl 2-(methylamino)-1,3-thiazole-4-carboxylate | 0.251 mmol | 46.8 mg | 2-2-b1C18-0 |

Mixtures 2-2-b1C14-0, 2-2-b1C16-0, 2-2-b1C17-0, 2-2-b1C18-0 can be treated by cleavage in the same manner as in step a1 to obtain mixtures 2-2-b1C14-1, 2-2-b1C16-1, 2-2- b1C17-1, 2-2-b1C18-1. The details of the mixtures are shown in Table 9-2-26 to Table 9-2- 29.

A notation method in each table will be described. For example, in Table 9-2-26, each compound that may be contained in 2-2-b1C14-1 is indicated by ID, and core blocks and linkers in combination in the structure of mixture 2-2-b1C14-1 are indicated by symbols or chemical formulas. The correspondence between the symbols and specific structures are shown in Figure 6.

In Table 9-2-26 to Table 9-2-29, "×" and "c" are represented by chemical formulas.

[Formula 673]

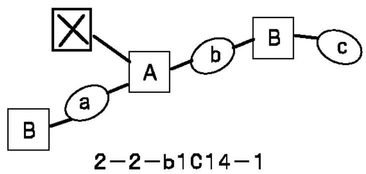


2-2-b1C14-1

[Table 452]

| [Table 9-2-26] Compound that may be contained in mixture 2-2-b1C14-1 | | | | | | | | |
|---|---|---|---|---|---|---|---|---|
| ID | Exact Mass | | A | a | B | b | C | c |
| 2-2-b1C14-1-0001 | 409.164 | OH | A01 | a1 | B01 | b1 | C14 | COOEt |
| 2-2-b1C14-1-0002 | 423.179 | OH | A01 | a1 | B02 | b1 | C14 | COOEt |
| 2-2-b1C14-1-0003 | 427.154 | OH | A01 | a1 | B03 | b1 | C14 | COOEt |
| 2-2-b1C14-1-0004 | 435.179 | OH | A02 | a1 | B01 | b1 | C14 | COOEt |
| 2-2-b1C14-1-0005 | 449.195 | OH | A02 | a1 | B02 | b1 | C14 | COOEt |

(continued)

| [Table 9-2-26] Compound that may be contained in mixture 2-2-b1C14-1 | | | | | | | | |
|---|---|---|---|---|---|---|---|---|
| ID | Exact Mass | | A | a | B | b | C | c |
| 2-2-b1C14-1-0006 | 453.170 | OH | A02 | a1 | B03 | b1 | C14 | COOEt |
| 2-2-b1C14-1-0007 | 466.131 | OH | A01 | a1 | B04 | b1 | C14 | COOEt |
| 2-2-b1C14-1-0008 | 477.151 | OH | A01 | a1 | B05 | b1 | C14 | COOEt |
| 2-2-b1C14-1-0009 | 492.147 | OH | A02 | a1 | B04 | b1 | C14 | COOEt |
| 2-2-b1C14-1-0010 | 503.167 | OH | A02 | a1 | B05 | b1 | C14 | COOEt |

[Table 453]

| [Table 9-2-27] Compound that may be contained in mixture 2-2-b1C16-1 | | | | | | | | |
|---|---|---|---|---|---|---|---|---|
| ID | Exact Mass | | A | a | B | b | C | c |
| 2-2-b1C16-1-0001 | 419.185 | OH | A01 | a1 | B01 | b1 | C16 | COOEt |
| 2-2-b1C16-1-0002 | 433.200 | OH | A01 | a1 | B02 | b1 | C16 | COOEt |
| 2-2-b1C16-1-0003 | 437.175 | OH | A01 | a1 | B03 | b1 | C16 | COOEt |
| 2-2-b1C16-1-0004 | 445.200 | OH | A02 | a1 | B01 | b1 | C16 | COOEt |
| 2-2-b1C16-1-0005 | 459.216 | OH | A02 | a1 | B02 | b1 | C16 | COOEt |
| 2-2-b1C16-1-0006 | 463.191 | OH | A02 | a1 | B03 | b1 | C16 | COOEt |
| 2-2-b1C16-1-0007 | 476.152 | OH | A01 | a1 | B04 | b1 | C16 | COOEt |
| 2-2-b1C16-1-0008 | 487.172 | OH | A01 | a1 | B05 | b1 | C16 | COOEt |
| 2-2-b1C16-1-0009 | 502.167 | OH | A02 | a1 | B04 | b1 | C16 | COOEt |
| 2-2-b1C16-1-0010 | 513.188 | OH | A02 | a1 | B05 | b1 | C16 | COOEt |

[Table 454]

| [Table 9-2-28] Compound that may be contained in mixture 2-2-b1C17-1 | | | | | | | | |
|---|---|---|---|---|---|---|---|---|
| ID | Exact Mass | | A | a | B | b | C | c |
| 2-2-b1C17-1-0001 | 420.18 | OH | A01 | a1 | B01 | b1 | C17 | COOEt |
| 2-2-b1C17-1-0002 | 434.195 | OH | A01 | a1 | B02 | b1 | C17 | COOEt |
| 2-2-b1C17-1-0003 | 438.170 | OH | A01 | a1 | B03 | b1 | C17 | COOEt |
| 2-2-b1C17-1-0004 | 446.195 | OH | A02 | a1 | B01 | b1 | C17 | COOEt |
| 2-2-b1C17-1-0005 | 460.211 | OH | A02 | a1 | B02 | b1 | C17 | COOEt |
| 2-2-b1C17-1-0006 | 464.186 | OH | A02 | a1 | B03 | b1 | C17 | COOEt |
| 2-2-b1C17-1-0007 | 477.147 | OH | A01 | a1 | B04 | b1 | C17 | COOEt |
| 2-2-b1C17-1-0008 | 488.167 | OH | A01 | a1 | B05 | b1 | C17 | COOEt |
| 2-2-b1C17-1-0009 | 503.163 | OH | A02 | a1 | B04 | b1 | C17 | COOEt |
| 2-2-b1C17-1-0010 | 514.183 | OH | A02 | a1 | B05 | b1 | C17 | COOEt |

[Table 455]

| Table 9-2-29] Compound that may be contained in mixture 2-2-b1C18-1 | | | | | | | | |
|---|---|---|---|---|---|---|---|---|
| ID | Exact Mass | | A | a | B | b | C | c |
| 2-2-b1C18-1-0001 | 425.141 | OH | A01 | a1 | B01 | b1 | C18 | COOEt |
| 2-2-b1C18-1-0002 | 439.157 | OH | A01 | a1 | B02 | b1 | C18 | COOEt |
| 2-2-b1C18-1-0003 | 443.132 | OH | A01 | a1 | B03 | b1 | C18 | COOEt |
| 2-2-b1C18-1-0004 | 451.157 | OH | A02 | a1 | B01 | b1 | C18 | COOEt |
| 2-2-b1C18-1-0005 | 465.172 | OH | A02 | a1 | B02 | b1 | C18 | COOEt |
| 2-2-b1C18-1-0006 | 469.147 | OH | A02 | a1 | B03 | b1 | C18 | COOEt |
| 2-2-b1C18-1-0007 | 482.108 | OH | A01 | a1 | B04 | b1 | C18 | COOEt |
| 2-2-b1C18-1-0008 | 493.128 | OH | A01 | a1 | B05 | b1 | C18 | COOEt |
| 2-2-b1C18-1-0009 | 508.124 | OH | A02 | a1 | B04 | b1 | C18 | COOEt |
| 2-2-b1C18-1-0010 | 519.144 | OH | A02 | a1 | B05 | b1 | C18 | COOEt |

Example 9-2-9: Synthesis of mixture 2-2-b1C19-0 by step b1C19

[Formula 674]

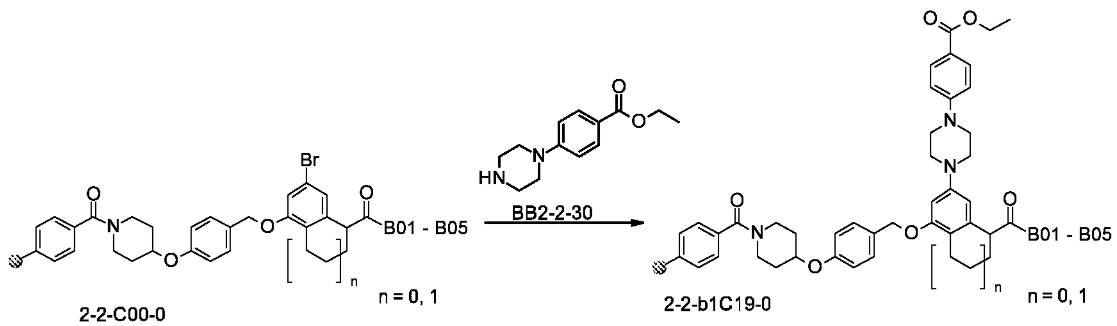


2-2-C00-0 (128 mg, 0.196 mmol/g) and DMPr (2.56 mL) were added to a 4 mL glass vial and shaken at room temperature for 1 hour. Ethyl 4-(piperazin-1-yl)benzoate (BB2-2-30) (58.9 mg, 0.251 mmol) was added thereto. (tBu)Ph-CPhos Pd G4 (19. mg, 0.025 mmol) and 2 M PztBu in THF (0.189 mL, 0.377 mmol) were added thereto, and the mixture was shaken at 25°C for 1 hour.

Solid-phase purification

The suspension of the reaction solution and the solid phase was transferred to a 6 mL column with a filter and washed three times with NMP/water = 1/1 (2.6 mL), three times with NMP (2.6 mL), three times with 0.2 M NAC in NMP/water = 5/1 (2.6 mL), three times with 0.05 M $TBAHSO_4$ + DTBP in NMP (2.6 mL), three times with 0.05 M NMM in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain mixture 2-2-b1C19-0.

Mixture 2-2-b1C19-0 can be treated by cleavage in the same manner as in step a1 to obtain mixture 2-2-b1C19-1. The details of the mixture are shown in Table 9-2-30.

A notation method in each table will be described. For example, in Table 9-2-30, each compound that may be contained in 2-2-b1C19-1 is indicated by ID, and core blocks and linkers in combination in the structure of mixture 2-2-b1C19-1 are indicated by symbols or chemical formulas. The correspondence between the symbols and specific structures are shown in Figure 6.

In Table 9-2-30, "×" and "c" are represented by chemical formulas.

[Formula 675]

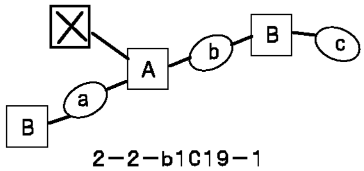


2−2−b1C19−1

[Table 456]

| [Table 9-2-30] Compound that may be contained in mixture 2-2-b1C19-1 | | | | | | | | |
|---|---|---|---|---|---|---|---|---|
| ID | Exact Mass | | A | a | B | b | C | c |
| 2-2-b1C19-1-0001 | 473.231 | OH | A01 | a1 | B01 | b1 | C19 | COOEt |
| 2-2-b1C19-1-0002 | 487.247 | OH | A01 | a1 | B02 | b1 | C19 | COOEt |
| 2-2-b1C19-1-0003 | 491.222 | OH | A01 | a1 | B03 | b1 | C19 | COOEt |
| 2-2-b1C19-1-0004 | 499.247 | OH | A02 | a1 | B01 | b1 | C19 | COOEt |
| 2-2-b1C19-1-0005 | 513.263 | OH | A02 | a1 | B02 | b1 | C19 | COOEt |
| 2-2-b1C19-1-0006 | 517.238 | OH | A02 | a1 | B03 | b1 | C19 | COOEt |
| 2-2-b1C19-1-0007 | 530.199 | OH | A01 | a1 | B04 | b1 | C19 | COOEt |
| 2-2-b1C19-1-0008 | 541.219 | OH | A01 | a1 | B05 | b1 | C19 | COOEt |
| 2-2-b1C19-1-0009 | 556.214 | OH | A02 | a1 | B04 | b1 | C19 | COOEt |
| 2-2-b1C19-1-0010 | 567.234 | OH | A02 | a1 | B05 | b1 | C19 | COOEt |

Example 9-3: Hydrolysis step for linker c formation

Example 9-3-1: Synthesis of mixtures described in Table 9-3-1 by hydrolysis step h

[Formula 676]

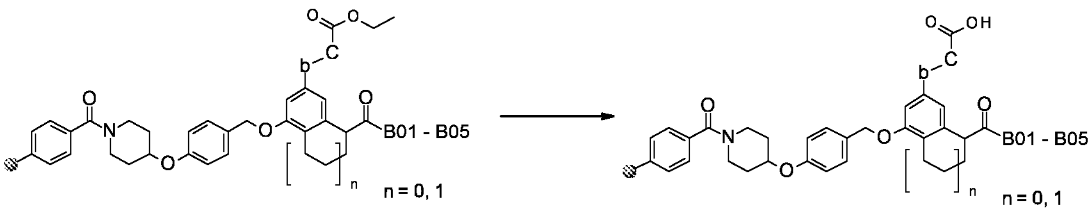

The mixtures described in Table 9-3-1, which were obtained in Example 2, were added to fourteen 6 mL columns with a filter, respectively. NMP (1.79 mL), 1 M TBAOH (0.074 mL, 0.074 mmol), and tAmylOH (0.512 mL) were added thereto. The columns were capped so as not to leak the reaction solution, and shaken at room temperature for 3 hours.

Solid-phase purification

The suspension of the reaction solution and the solid phase was filtered through the filter of the column and washed three times with NMP/water = 1/1 (2.6 mL), three times with 0.05 M $TBAHSO_4$ + DTBP in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain the mixtures described in Table 9-3-1.

[Table 457]

[Table 9-3-1]

| No. of mixture used | No. of obtained mixture |
|---|---|
| 2-2-b1C04-0 | 2-2-h05C04-0 |
| 2-2-b1C01-0 | 2-2-h02C01-0 |
| 2-2-b2C02-0 | 2-2-h07C02-0 |
| 2-2-b4C12-0 | 2-2-h16C12-0 |
| 2-2-b4C13-0 | 2-2-h17C13-0 |
| 2-2-b4C02-0 | 2-2-h19C02-0 |
| 2-2-b4C01-0 | 2-2-h18C01-0 |
| 2-2-b4C05-0 | 2-2-h20C05-0 |
| 2-2-b3C11-0 | 2-2-h15C11-0 |
| 2-2-b3C08-0 | 2-2-h12C08-0 |
| 2-2-b3C09-0 | 2-2-h13C09-0 |
| 2-2-b3C10-0 | 2-2-h14C10-0 |
| 2-2-b1C17-0 | 2-2-h24C17-0 |
| 2-2-b1C14-0 | 2-2-h21C14-0 |

The obtained mixtures in Table 9-3-1 can be treated by cleavage in the same manner as in step a1 to respectively obtain mixtures in a state cleaved from the solid phase. The details of the mixtures are shown in Table 9-3-2 to Table 9-3-15.

A notation method in each table will be described. For example, in Table 9-3-2, each compound that may be contained in 2-2-h05C04-1 is indicated by ID, and core blocks and linkers in combination in the structure of mixture 2-2-h05C04-1 are indicated by symbols or chemical formulas. The correspondence between the symbols and specific structures are shown in Figure 6.

In Table 9-3-2 to Table 9-3-15, "×" and "c" are represented by chemical formulas.

[Formula 677]

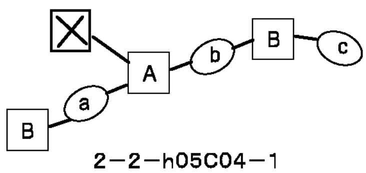


2-2-h05C04-1

For example, when ID is 2-2-h05C04-1-0001, the compound is represented by the following structural formula.

[Formula 678]

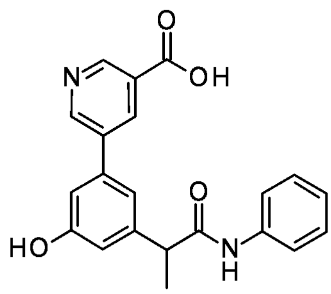

2-2-h05C04-1-0001

[Table 458]

| Exemplary notation in [Table 9-3-2] | | | | | | | | |
|---|---|---|---|---|---|---|---|---|
| ID | Exact Mass | | A | a | B | b | C | c |
| 2-2-h05C04-1-0001 | 362.127 | OH | A01 | a1 | B01 | b1 | C04 | COOH |

[Table 459]

| [Table 9-3-2] Compound that may be contained in mixture 2-2-h05C04-1 | | | | | | | | |
|---|---|---|---|---|---|---|---|---|
| ID | Exact Mass | | A | a | B | b | C | c |
| 2-2-h05C04-1-000 1 | 362.127 | OH | A01 | a1 | B01 | b1 | C04 | COOH |
| 2-2-h05C04-1-0002 | 376.142 | OH | A01 | a1 | B02 | b1 | C04 | COOH |
| 2-2-h05C04-1-0003 | 380.117 | OH | A01 | a1 | B03 | b1 | C04 | COOH |
| 2-2-h05C04-1-0004 | 388.142 | OH | A02 | a1 | B01 | b1 | C04 | COOH |
| 2-2-h05C04-1-0005 | 402.158 | OH | A02 | a1 | B02 | b1 | C04 | COOH |
| 2-2-h05C04-1-0006 | 406.133 | OH | A02 | a1 | B03 | b1 | C04 | COOH |
| 2-2-h05C04-1-0007 | 419.094 | OH | A01 | a1 | B04 | b1 | C04 | COOH |
| 2-2-h05C04-1-0008 | 430.114 | OH | A01 | a1 | B05 | b1 | C04 | COOH |
| 2-2-h05C04-1-0009 | 445.110 | OH | A02 | a1 | B04 | b1 | C04 | COOH |
| 2-2-h05C04-1-0010 | 456.130 | OH | A02 | a1 | B05 | b1 | C04 | COOH |

[Table 460]

| [Table 9-3-3] Compound that may be contained in mixture 2-2-h02C01-1 | | | | | | | | |
|---|---|---|---|---|---|---|---|---|
| ID | Exact Mass | | A | a | B | b | C | c |
| 2-2-h02C01-1-0001 | 351.111 | OH | A01 | a1 | B01 | b1 | C01 | COOH |
| 2-2-h02C01-1-0002 | 365.126 | OH | A01 | a1 | B02 | b1 | C01 | COOH |
| 2-2-h02C01-1-0003 | 369.101 | OH | A01 | a1 | B03 | b1 | C01 | COOH |
| 2-2-h02C01-1-0004 | 377.126 | OH | A02 | a1 | B01 | b1 | C01 | COOH |
| 2-2-h02C01-1-0005 | 391.142 | OH | A02 | a1 | B02 | b1 | C01 | COOH |
| 2-2-h02C01-1-0006 | 395.117 | OH | A02 | a1 | B03 | b1 | C01 | COOH |
| 2-2-h02C01-1-0007 | 408.078 | OH | A01 | a1 | B04 | b1 | C01 | COOH |
| 2-2-h02C01-1-0008 | 419.098 | OH | A01 | a1 | B05 | b1 | C01 | COOH |
| 2-2-h02C01-1-0009 | 434.094 | OH | A02 | a1 | B04 | b1 | C01 | COOH |
| 2-2-h02C01-1-0010 | 445.114 | OH | A02 | a1 | B05 | b1 | C01 | COOH |

[Table 461]

| [Table 9-3-4] Compound that may be contained in mixture 2-2-h07C02-1 | | | | | | | | |
|---|---|---|---|---|---|---|---|---|
| ID | Exact Mass | | A | a | B | b | C | c |
| 2-2-h07C02-1-0001 | 385.131 | OH | A01 | a1 | B01 | b2 | C02 | COOH |
| 2-2-h07C02-1-0002 | 399.147 | OH | A01 | a1 | B02 | b2 | C02 | COOH |
| 2-2-h07C02-1-0003 | 403.122 | OH | A01 | a1 | B03 | b2 | C02 | COOH |
| 2-2-h07C02-1-0004 | 411.147 | OH | A02 | a1 | B01 | b2 | C02 | COOH |
| 2-2-h07C02-1-0005 | 425.163 | OH | A02 | a1 | B02 | b2 | C02 | COOH |
| 2-2-h07C02-1-0006 | 429.138 | OH | A02 | a1 | B03 | b2 | C02 | COOH |
| 2-2-h07C02-1-0007 | 442.099 | OH | A01 | a1 | B04 | b2 | C02 | COOH |
| 2-2-h07C02-1-0008 | 453.119 | OH | A01 | a1 | B05 | b2 | C02 | COOH |
| 2-2-h07C02-1-0009 | 468.114 | OH | A02 | a1 | B04 | b2 | C02 | COOH |
| 2-2-h07C02-1-0010 | 479.134 | OH | A02 | a1 | B05 | b2 | C02 | COOH |

[Table 462]

| [Table 9-3-5] Compound that may be contained in mixture 2-2-h16C12-1 | | | | | | | | |
|---|---|---|---|---|---|---|---|---|
| ID | Exact Mass | | A | a | B | b | C | c |
| 2-2-h16C12-1-0001 | 355.178 | OH | A01 | a1 | B01 | b4 | C12 | COOH |
| 2-2-h16C12-1-0002 | 369.194 | OH | A01 | a1 | B02 | b4 | C12 | COOH |
| 2-2-h16C12-1-0003 | 373.169 | OH | A01 | a1 | B03 | b4 | C12 | COOH |
| 2-2-h16C12-1-0004 | 381.194 | OH | A02 | a1 | B01 | b4 | C12 | COOH |
| 2-2-h16C12-1-0005 | 395.2100 | OH | A02 | a1 | B02 | b4 | C12 | COOH |
| 2-2-h16C12-1-0006 | 399.185 | OH | A02 | a1 | B03 | b4 | C12 | COOH |
| 2-2-h16C12-1-0007 | 412.146 | OH | A01 | a1 | B04 | b4 | C12 | COOH |
| 2-2-h16C12-1-0008 | 423.166 | OH | A01 | a1 | B05 | b4 | C12 | COOH |
| 2-2-h16C12-1-0009 | 438.161 | OH | A02 | a1 | B04 | b4 | C12 | COOH |
| 2-2-h16C12-1-0010 | 449.181 | OH | A02 | a1 | B05 | b4 | C12 | COOH |

[Table 463]

| [Table 9-3-6] Compound that may be contained in mixture 2-2-h17C13-1 | | | | | | | | |
|---|---|---|---|---|---|---|---|---|
| ID | Exact Mass | | A | a | B | b | C | c |
| 2-2-h17C13-1-0001 | 357.158 | OH | A01 | a1 | B01 | b4 | C13 | COOH |
| 2-2-h17C13-1-0002 | 371.173 | OH | A01 | a1 | B02 | b4 | C13 | COOH |
| 2-2-h17C13-1-0003 | 375.148 | OH | A01 | a1 | B03 | b4 | C13 | COOH |
| 2-2-h17C13-1-0004 | 383.173 | OH | A02 | a1 | B01 | b4 | C13 | COOH |
| 2-2-h17C13-1-0005 | 397.189 | OH | A02 | a1 | B02 | b4 | C13 | COOH |
| 2-2-h17C13-1-0006 | 401.164 | OH | A02 | a1 | B03 | b4 | C13 | COOH |

(continued)

| [Table 9-3-6] Compound that may be contained in mixture 2-2-h17C13-1 | | | | | | | | |
|---|---|---|---|---|---|---|---|---|
| ID | Exact Mass | | A | a | B | b | C | c |
| 2-2-h17C13-1-0007 | 414.125 | OH | A01 | a1 | B04 | b4 | C13 | COOH |
| 2-2-h17C13-1-0008 | 425.145 | OH | A01 | a1 | B05 | b4 | C13 | COOH |
| 2-2-h17C13-1-0009 | 440.141 | OH | A02 | a1 | B04 | b4 | C13 | COOH |
| 2-2-h17C13-1-0010 | 451.161 | OH | A02 | a1 | B05 | b4 | C13 | COOH |

[Table 464]

| [Table 9-3-7] Compound that may be contained in mixture 2-2-h19C02-1 | | | | | | | | |
|---|---|---|---|---|---|---|---|---|
| ID | Exact Mass | | A | a | B | b | C | c |
| 2-2-h19C02-1-0001 | 403.178 | OH | A01 | a1 | B01 | b4 | C02 | COOH |
| 2-2-h19C02-1-0002 | 417.194 | OH | A01 | a1 | B02 | b4 | C02 | COOH |
| 2-2-h19C02-1-0003 | 421.169 | OH | A01 | a1 | B03 | b4 | C02 | COOH |
| 2-2-h19C02-1-0004 | 429.194 | OH | A02 | a1 | B01 | b4 | C02 | COOH |
| 2-2-h19C02-1-0005 | 443.210 | OH | A02 | a1 | B02 | b4 | C02 | COOH |
| 2-2-h19C02-1-0006 | 447.185 | OH | A02 | a1 | B03 | b4 | C02 | COOH |
| 2-2-h19C02-1-0007 | 460.146 | OH | A01 | a1 | B04 | b4 | C02 | COOH |
| 2-2-h19C02-1-0008 | 471.166 | OH | A01 | a1 | B05 | b4 | C02 | COOH |
| 2-2-h19C02-1-0009 | 486.161 | OH | A02 | a1 | B04 | b4 | C02 | COOH |
| 2-2-h19C02-1-0010 | 497.181 | OH | A02 | a1 | B05 | b4 | C02 | COOH |

[Table 465]

| [Table 9-3-8] Compound that may be contained in mixture 2-2-hl8C01-l | | | | | | | | |
|---|---|---|---|---|---|---|---|---|
| ID | Exact Mass | | A | a | B | b | C | c |
| 2-2-h18C01-1-0001 | 393.158 | OH | A01 | a1 | B01 | b4 | C01 | COOH |
| 2-2-h18C01-1-0002 | 407.173 | OH | A01 | a1 | B02 | b4 | C01 | COOH |
| 2-2-h18C01-1-0003 | 411.148 | OH | A01 | a1 | B03 | b4 | C01 | COOH |
| 2-2-h18C01-1-0004 | 419.173 | OH | A02 | a1 | B01 | b4 | C01 | COOH |
| 2-2-h18C01-1-0005 | 433.189 | OH | A02 | a1 | B02 | b4 | C01 | COOH |
| 2-2-h18C01-1-0006 | 437.164 | OH | A02 | a1 | B03 | b4 | C01 | COOH |
| 2-2-h18C01-1-0007 | 450.125 | OH | A01 | a1 | B04 | b4 | C01 | COOH |
| 2-2-h18C01-1-0008 | 461.145 | OH | A01 | a1 | B05 | b4 | C01 | COOH |
| 2-2-h18C01-1-0009 | 476.141 | OH | A02 | a1 | B04 | b4 | C01 | COOH |
| 2-2-h18C01-1-0010 | 487.161 | OH | A02 | a1 | B05 | b4 | C01 | COOH |

[Table 466]

| [Table 9-3-9] Compound that may be contained in mixture 2-2-h20C05-1 | | | | | | | | |
|---|---|---|---|---|---|---|---|---|
| ID | Exact Mass | | A | a | B | b | C | c |
| 2-2-h20C05-1-0001 | 409.135 | OH | A01 | a1 | B01 | b4 | C05 | COOH |
| 2-2-h20C05-1-0002 | 423.150 | OH | A01 | a1 | B02 | b4 | C05 | COOH |
| 2-2-h20C05-1-0003 | 427.125 | OH | A01 | a1 | B03 | b4 | C05 | COOH |
| 2-2-h20C05-1-0004 | 435.150 | OH | A02 | a1 | B01 | b4 | C05 | COOH |
| 2-2-h20C05-1-0005 | 449.166 | OH | A02 | a1 | B02 | b4 | C05 | COOH |
| 2-2-h20C05-1-0006 | 453.141 | OH | A02 | a1 | B03 | b4 | C05 | COOH |
| 2-2-h20C05-1-0007 | 466.102 | OH | A01 | a1 | B04 | b4 | C05 | COOH |
| 2-2-h20C05-1-0008 | 477.122 | OH | A01 | a1 | B05 | b4 | C05 | COOH |
| 2-2-h20C05-1-0009 | 492.118 | OH | A02 | a1 | B04 | b4 | C05 | COOH |
| 2-2-h20C05-1-0010 | 503.138 | OH | A02 | a1 | B05 | b4 | C05 | COOH |

[Table 467]

| [Table 9-3-10] Compound that may be contained in mixture 2-2-hl5Cl 1-1 | | | | | | | | |
|---|---|---|---|---|---|---|---|---|
| ID | Exact Mass | | A | a | B | b | C | c |
| 2-2-h15C11-1-0001 | 417.121 | OH | A01 | a1 | B01 | b3 | C11 | COOH |
| 2-2-h15C11-1-0002 | 431.137 | OH | A01 | a1 | B02 | b3 | C11 | COOH |
| 2-2-h15C11-1-0003 | 435.112 | OH | A01 | a1 | B03 | b3 | C11 | COOH |
| 2-2-h15C11-1-0004 | 443.137 | OH | A02 | a1 | B01 | b3 | C11 | COOH |
| 2-2-h15C11-1-0005 | 457.153 | OH | A02 | a1 | B02 | b3 | C11 | COOH |
| 2-2-h15C11-1-0006 | 461.127 | OH | A02 | a1 | B03 | b3 | C11 | COOH |
| 2-2-h15C11-1-0007 | 474.089 | OH | A01 | a1 | B04 | b3 | C11 | COOH |
| 2-2-h15C11-1-0008 | 485.109 | OH | A01 | a1 | B05 | b3 | C11 | COOH |
| 2-2-h15C11-1-0009 | 500.104 | OH | A02 | a1 | B04 | b3 | C11 | COOH |
| 2-2-hl5Cl 1-1-0010 | 511.124 | OH | A02 | a1 | B05 | b3 | C11 | COOH |

[Table 468]

| [Table 9-3-11] Compound that may be contained in mixture 2-2-h12C08-1 | | | | | | | | |
|---|---|---|---|---|---|---|---|---|
| ID | Exact Mass | | A | a | B | b | C | c |
| 2-2-h12C08-1-0001 | 417.121 | OH | A01 | a1 | B01 | b3 | C08 | COOH |
| 2-2-h12C08-1-0002 | 431.137 | OH | A01 | a1 | B02 | b3 | C08 | COOH |
| 2-2-h12C08-1-0003 | 435.112 | OH | A01 | a1 | B03 | b3 | C08 | COOH |
| 2-2-h12C08-1-0004 | 443.137 | OH | A02 | a1 | B01 | b3 | C08 | COOH |
| 2-2-h12C08-1-0005 | 457.153 | OH | A02 | a1 | B02 | b3 | C08 | COOH |
| 2-2-h12C08-1-0006 | 461.127 | OH | A02 | a1 | B03 | b3 | C08 | COOH |

(continued)

| [Table 9-3-11] Compound that may be contained in mixture 2-2-h12C08-1 | | | | | | | | |
|---|---|---|---|---|---|---|---|---|
| ID | Exact Mass | | A | a | B | b | C | c |
| 2-2-h12C08-1-0007 | 474.089 | OH | A01 | a1 | B04 | b3 | C08 | COOH |
| 2-2-h12C08-1-0008 | 485.109 | OH | A01 | a1 | B05 | b3 | C08 | COOH |
| 2-2-h12C08-1-0009 | 500.104 | OH | A02 | a1 | B04 | b3 | C08 | COOH |
| 2-2-h12C08-1-0010 | 511.124 | OH | A02 | a1 | B05 | b3 | C08 | COOH |

[Table 469]

| [Table 9-3-12] Compound that may be contained in mixture 2-2-h13C09-1 | | | | | | | | |
|---|---|---|---|---|---|---|---|---|
| ID | Exact Mass | | A | a | B | b | C | c |
| 2-2-h13C09-1-0001 | 379.117 | OH | A01 | a1 | B01 | b3 | C09 | COOH |
| 2-2-h13C09-1-0002 | 393.132 | OH | A01 | a1 | B02 | b3 | C09 | COOH |
| 2-2-h13C09-1-0003 | 397.107 | OH | A01 | a1 | B03 | b3 | C09 | COOH |
| 2-2-h13C09-1-0004 | 405.132 | OH | A02 | a1 | B01 | b3 | C09 | COOH |
| 2-2-h13C09-1-0005 | 419.148 | OH | A02 | a1 | B02 | b3 | C09 | COOH |
| 2-2-h13C09-1-0006 | 423.123 | OH | A02 | a1 | B03 | b3 | C09 | COOH |
| 2-2-h13C09-1-0007 | 436.084 | OH | A01 | a1 | B04 | b3 | C09 | COOH |
| 2-2-h13C09-1-0008 | 447.104 | OH | A01 | a1 | B05 | b3 | C09 | COOH |
| 2-2-h13C09-1-0009 | 462.100 | OH | A02 | a1 | B04 | b3 | C09 | COOH |
| 2-2-h13C09-1-0010 | 473.120 | OH | A02 | a1 | B05 | b3 | C09 | COOH |

[Table 470]

| [Table 9-3-13] Compound that may be contained in mixture 2-2-h14C10-1 | | | | | | | | |
|---|---|---|---|---|---|---|---|---|
| ID | Exact Mass | | A | a | B | b | C | c |
| 2-2-h14C10-1-0001 | 384.078 | OH | A01 | a1 | B01 | b3 | C10 | COOH |
| 2-2-h14C10-1-0002 | 398.094 | OH | A01 | a1 | B02 | b3 | C10 | COOH |
| 2-2-h14C10-1-0003 | 402.069 | OH | A01 | a1 | B03 | b3 | C10 | COOH |
| 2-2-h14C10-1-0004 | 410.094 | OH | A02 | a1 | B01 | b3 | C10 | COOH |
| 2-2-h14C10-1-0005 | 424.109 | OH | A02 | a1 | B02 | b3 | C10 | COOH |
| 2-2-h14C10-1-0006 | 428.084 | OH | A02 | a1 | B03 | b3 | C10 | COOH |
| 2-2-h14C10-1-0007 | 441.045 | OH | A01 | a1 | B04 | b3 | C10 | COOH |
| 2-2-h14C10-1-0008 | 452.065 | OH | A01 | a1 | B05 | b3 | C10 | COOH |
| 2-2-h14C10-1-0009 | 467.061 | OH | A02 | a1 | B04 | b3 | C10 | COOH |
| 2-2-h14C10-1-0010 | 478.081 | OH | A02 | a1 | B05 | b3 | C10 | COOH |

[Table 471]

| [Table 9-3-14] Compound that may be contained in mixture 2-2-h24C17-1 | | | | | | | | |
|---|---|---|---|---|---|---|---|---|
| ID | Exact Mass | | A | a | B | b | C | c |
| 2-2-h24C17-1-0001 | 392.148 | OH | A01 | a1 | B01 | b1 | C17 | COOH |
| 2-2-h24C17-1-0002 | 406.164 | OH | A01 | a1 | B02 | b1 | C17 | COOH |
| 2-2-h24C17-1-0003 | 410.139 | OH | A01 | a1 | B03 | b1 | C17 | COOH |
| 2-2-h24C17-1-0004 | 418.164 | OH | A02 | a1 | B01 | b1 | C17 | COOH |
| 2-2-h24C17-1-0005 | 432.180 | OH | A02 | a1 | B02 | b1 | C17 | COOH |
| 2-2-h24C17-1-0006 | 436.155 | OH | A02 | a1 | B03 | b1 | C17 | COOH |
| 2-2-h24C17-1-0007 | 449.116 | OH | A01 | a1 | B04 | b1 | C17 | COOH |
| 2-2-h24C17-1-0008 | 460.136 | OH | A01 | a1 | B05 | b1 | C17 | COOH |
| 2-2-h24C17-1-0009 | 475.131 | OH | A02 | a1 | B04 | b1 | C17 | COOH |
| 2-2-h24C17-1-0010 | 486.151 | OH | A02 | a1 | B05 | b1 | C17 | COOH |

[Table 472]

| [Table 9-3-15] Compound that may be contained in mixture 2-2-h21C14-1 | | | | | | | | |
|---|---|---|---|---|---|---|---|---|
| ID | Exact Mass | | A | a | B | b | C | c |
| 2-2-h21C14-1-0001 | 381.132 | OH | A01 | a1 | B01 | b1 | C14 | COOH |
| 2-2-h21C14-1-0002 | 395.148 | OH | A01 | a1 | B02 | b1 | C14 | COOH |
| 2-2-h21C14-1-0003 | 399.123 | OH | A01 | a1 | B03 | b1 | C14 | COOH |
| 2-2-h21C14-1-0004 | 407.148 | OH | A02 | a1 | B01 | b1 | C14 | COOH |
| 2-2-h21C14-1-0005 | 421.164 | OH | A02 | a1 | B02 | b1 | C14 | COOH |
| 2-2-h21C14-1-0006 | 425.139 | OH | A02 | a1 | B03 | b1 | C14 | COOH |
| 2-2-h21C14-1-0007 | 438.100 | OH | A01 | a1 | B04 | b1 | C14 | COOH |
| 2-2-h21C14-1-0008 | 449.120 | OH | A01 | a1 | B05 | b1 | C14 | COOH |
| 2-2-h21C14-1-0009 | 464.115 | OH | A02 | a1 | B04 | b1 | C14 | COOH |
| 2-2-h21C14-1-0010 | 475.136 | OH | A02 | a1 | B05 | b1 | C14 | COOH |

Example 9-3-2: Synthesis of mixtures described in Table 9-3-16 by hydrolysis step h

NMP (1.79 mL), tAmylOH (0.512 mL), and a 1 M aqueous TBAOH solution (0.074 mL, 0.074 mmol) were added to nine 6 mL columns with a filter containing the mixtures described in Table 9-3-16, which were obtained by the preceding process. The columns were capped so as not to leak the reaction solution, and shaken at room temperature for 3 hours.
The suspension of the reaction solution and the solid phase was filtered through the filter of the column and washed three times with NMP/water = 1/1 (2.6 mL), three times with 0.05 M $TBAHSO_4$ + DTBP in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure.
NMP (1.79 mL), tAmylOH (0.512 mL), and 1 M aqueous TBAOH solution (0.124 mL, 0.124 mmol) were added thereto. The columns were capped so as not to leak the reaction solution, and shaken at room temperature for 17 hours.

Solid-phase purification

The suspension of the reaction solution and the solid phase was filtered through the filter of the column and

washed three times with NMP/water = 1/1 (2.6 mL), three times with 0.05 M $TBAHSO_4$ + DTBP in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain the mixtures described in Table 9-3-16.

[Table 473]

| [Table 9-3-16] | |
|---|---|
| No. of mixture used | No. of obtained mixture |
| 2-2-b1C02-0 | 2-2-h03C02-0 |
| 2-2-b1C03-0 | 2-2-h04C03-0 |
| 2-2-b1C05-0 | 2-2-h06C05-0 |
| 2-2-b2C03-0 | 2-2-h08C03-0 |
| 2-2-b2C04-0 | 2-2-h09C04-0 |
| 2-2-b2C07-0 | 2-2-h11C07-0 |
| 2-2-b2C06-0 | 2-2-h10C06-0 |
| 2-2-b1C16-0 | 2-2-h23C16-0 |
| 2-2-b1C18-0 | 2-2-h25C18-0 |

The obtained mixtures in Table 9-3-16 can be treated by cleavage in the same manner as in step a1 to respectively obtain mixtures in a state cleaved from the solid phase. The details of the mixtures are shown in Table 9-3-17 to Table 9-3-25.

A notation method in each table will be described. For example, in Table 9-3-17, each compound that may be contained in 2-2-h03C02-1 is indicated by ID, and core blocks and linkers in combination in the structure of mixture 2-2-h03C02-1 are indicated by symbols or chemical formulas. The correspondence between the symbols and specific structures are shown in Figure 6.

In Table 9-3-17 to Table 9-3-25, "×" and "c" are represented by chemical formulas.

[Formula 679]

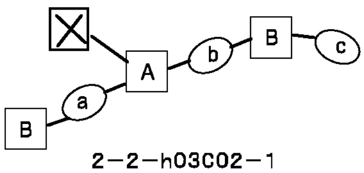


2-2-h03C02-1

[Table 474]

| [Table 9-3-17] Compound that may be contained in mixture 2-2-h03C02-1 | | | | | | | | |
|---|---|---|---|---|---|---|---|---|
| ID | Exact Mass | | A | a | B | b | C | c |
| 2-2-h03C02-1-0001 | 361.131 | OH | A01 | a1 | B01 | b1 | C02 | COOH |
| 2-2-h03C02-1-0002 | 375.147 | OH | A01 | a1 | B02 | b1 | C02 | COOH |
| 2-2-h03C02-1-0003 | 379.122 | OH | A01 | a1 | B03 | b1 | C02 | COOH |
| 2-2-h03C02-1-0004 | 387.147 | OH | A02 | a1 | B01 | b1 | C02 | COOH |
| 2-2-h03C02-1-0005 | 401.163 | OH | A02 | a1 | B02 | b1 | C02 | COOH |
| 2-2-h03C02-1-0006 | 405.138 | OH | A02 | a1 | B03 | b1 | C02 | COOH |
| 2-2-h03C02-1-0007 | 418.099 | OH | A01 | a1 | B04 | b1 | C02 | COOH |
| 2-2-h03C02-1-0008 | 429.119 | OH | A01 | a1 | B05 | b1 | C02 | COOH |
| 2-2-h03C02-1-0009 | 444.114 | OH | A02 | a1 | B04 | b1 | C02 | COOH |
| 2-2-h03C02-1-0010 | 455.134 | OH | A02 | a1 | B05 | b1 | C02 | COOH |

[Table 475]

| [Table 9-3-18] Compound that may be contained in mixture 2-2-h04C03-1 | | | | | | | | |
|---|---|---|---|---|---|---|---|---|
| ID | Exact Mass | | A | a | B | b | C | c |
| 2-2-h04C03-1-0001 | 361.131 | OH | A01 | a1 | B01 | b1 | C03 | COOH |
| 2-2-h04C03-1-0002 | 375.147 | OH | A01 | a1 | B02 | b1 | C03 | COOH |
| 2-2-h04C03-1-0003 | 379.122 | OH | A01 | a1 | B03 | b1 | C03 | COOH |
| 2-2-h04C03-1-0004 | 387.147 | OH | A02 | a1 | B01 | b1 | C03 | COOH |
| 2-2-h04C03-1-0005 | 401.163 | OH | A02 | a1 | B02 | b1 | C03 | COOH |
| 2-2-h04C03-1-0006 | 405.138 | OH | A02 | a1 | B03 | b1 | C03 | COOH |
| 2-2-h04C03-1-0007 | 418.099 | OH | A01 | a1 | B04 | b1 | C03 | COOH |
| 2-2-h04C03-1-0008 | 429.119 | OH | A01 | a1 | B05 | b1 | C03 | COOH |
| 2-2-h04C03-1-0009 | 444.114 | OH | A02 | a1 | B04 | b1 | C03 | COOH |
| 2-2-h04C03-1-0010 | 455.134 | OH | A02 | a1 | B05 | b1 | C03 | COOH |

[Table 476]

| [Table 9-3-19] Compound that may be contained in mixture 2-2-h06C05-1 | | | | | | | | |
|---|---|---|---|---|---|---|---|---|
| ID | Exact Mass | | A | a | B | b | C | c |
| 2-2-h06C05-1-0001 | 367.088 | OH | A01 | a1 | B01 | b1 | C05 | COOH |
| 2-2-h06C05-1-0002 | 381.103 | OH | A01 | a1 | B02 | b1 | C05 | COOH |
| 2-2-h06C05-1-0003 | 385.078 | OH | A01 | a1 | B03 | b1 | C05 | COOH |
| 2-2-h06C05-1-0004 | 393.103 | OH | A02 | a1 | B01 | b1 | C05 | COOH |
| 2-2-h06C05-1-0005 | 407.119 | OH | A02 | a1 | B02 | b1 | C05 | COOH |
| 2-2-h06C05-1-0006 | 411.094 | OH | A02 | a1 | B03 | b1 | C05 | COOH |
| 2-2-h06C05-1-0007 | 424.055 | OH | A01 | a1 | B04 | b1 | C05 | COOH |
| 2-2-h06C05-1-0008 | 435.075 | OH | A01 | a1 | B05 | b1 | C05 | COOH |
| 2-2-h06C05-1-0009 | 450.071 | OH | A02 | a1 | B04 | b1 | C05 | COOH |
| 2-2-h06C05-1-0010 | 461.091 | OH | A02 | a1 | B05 | b1 | C05 | COOH |

[Table 477]

| [Table 9-3-20] Compound that may be contained in mixture 2-2-h08C03-1 | | | | | | | | |
|---|---|---|---|---|---|---|---|---|
| ID | Exact Mass | | A | a | B | b | C | c |
| 2-2-h08C03-1-0001 | 385.131 | OH | A01 | a1 | B01 | b2 | C03 | COOH |
| 2-2-h08C03-1-0002 | 399.147 | OH | A01 | a1 | B02 | b2 | C03 | COOH |
| 2-2-h08C03-1-0003 | 403.122 | OH | A01 | a1 | B03 | b2 | C03 | COOH |
| 2-2-h08C03-1-0004 | 411.147 | OH | A02 | a1 | B01 | b2 | C03 | COOH |
| 2-2-h08C03-1-0005 | 425.163 | OH | A02 | a1 | B02 | b2 | C03 | COOH |

(continued)

| [Table 9-3-20] Compound that may be contained in mixture 2-2-h08C03-1 | | | | | | | | |
|---|---|---|---|---|---|---|---|---|
| ID | Exact Mass | | A | a | B | b | C | c |
| 2-2-h08C03-1-0006 | 429.138 | OH | A02 | a1 | B03 | b2 | C03 | COOH |
| 2-2-h08C03-1-0007 | 442.099 | OH | A01 | a1 | B04 | b2 | C03 | COOH |
| 2-2-h08C03-1-0008 | 453.119 | OH | A01 | a1 | B05 | b2 | C03 | COOH |
| 2-2-h08C03-1-0009 | 468.114 | OH | A02 | a1 | B04 | b2 | C03 | COOH |
| 2-2-h08C03-1-0010 | 479.134 | OH | A02 | a1 | B05 | b2 | C03 | COOH |

[Table 478]

| [Table 9-3-21] Compound that may be contained in mixture 2-2-h09C04-1 | | | | | | | | |
|---|---|---|---|---|---|---|---|---|
| ID | Exact Mass | | A | a | B | b | C | c |
| 2-2-h09C04-1-0001 | 386.127 | OH | A01 | a1 | B01 | b2 | C04 | COOH |
| 2-2-h09C04-1-0002 | 400.142 | OH | A01 | a1 | B02 | b2 | C04 | COOH |
| 2-2-h09C04-1-0003 | 404.117 | OH | A01 | a1 | B03 | b2 | C04 | COOH |
| 2-2-h09C04-1-0004 | 412.142 | OH | A02 | a1 | B01 | b2 | C04 | COOH |
| 2-2-h09C04-1-0005 | 426.158 | OH | A02 | a1 | B02 | b2 | C04 | COOH |
| 2-2-h09C04-1-0006 | 430.133 | OH | A02 | a1 | B03 | b2 | C04 | COOH |
| 2-2-h09C04-1-0007 | 443.094 | OH | A01 | a1 | B04 | b2 | C04 | COOH |
| 2-2-h09C04-1-0008 | 454.114 | OH | A01 | a1 | B05 | b2 | C04 | COOH |
| 2-2-h09C04-1-0009 | 469.110 | OH | A02 | a1 | B04 | b2 | C04 | COOH |
| 2-2-h09C04-1-0010 | 480.130 | OH | A02 | a1 | B05 | b2 | C04 | COOH |

[Table 479]

| [Table 9-3-22] Compound that may be contained in mixture 2-2-h 11 C07-1 | | | | | | | | |
|---|---|---|---|---|---|---|---|---|
| ID | Exact Mass | | A | a | B | b | C | c |
| 2-2-h11C07-1-0001 | 386.127 | OH | A01 | a1 | B01 | b2 | C07 | COOH |
| 2-2-h11C07-1-0002 | 400.142 | OH | A01 | a1 | B02 | b2 | C07 | COOH |
| 2-2-h11C07-1-0003 | 404.117 | OH | A01 | a1 | B03 | b2 | C07 | COOH |
| 2-2-h11C07-1-0004 | 412.142 | OH | A02 | a1 | B01 | b2 | C07 | COOH |
| 2-2-h11C07-1-0005 | 426.158 | OH | A02 | a1 | B02 | b2 | C07 | COOH |
| 2-2-h11C07-1-0006 | 430.133 | OH | A02 | a1 | B03 | b2 | C07 | COOH |
| 2-2-h11C07-1-0007 | 443.094 | OH | A01 | a1 | B04 | b2 | C07 | COOH |
| 2-2-h11C07-1-0008 | 454.114 | OH | A01 | a1 | B05 | b2 | C07 | COOH |
| 2-2-h11C07-1-0009 | 469.110 | OH | A02 | a1 | B04 | b2 | C07 | COOH |
| 2-2-h11C07-1-0010 | 480.130 | OH | A02 | a1 | B05 | b2 | C07 | COOH |

[Table 480]

| [Table 9-3-23] Compound that may be contained in mixture 2-2-h10C06-1 | | | | | | | | |
|---|---|---|---|---|---|---|---|---|
| ID | Exact Mass | | A | a | B | b | C | c |
| 2-2-h10C06-1-0001 | 386.127 | OH | A01 | a1 | B01 | b2 | C06 | COOH |
| 2-2-h10C06-1-0002 | 400.142 | OH | A01 | a1 | B02 | b2 | C06 | COOH |
| 2-2-h10C06-1-0003 | 404.117 | OH | A01 | a1 | B03 | b2 | C06 | COOH |
| 2-2-h10C06-1-0004 | 412.142 | OH | A02 | a1 | B01 | b2 | C06 | COOH |
| 2-2-h10C06-1-0005 | 426.158 | OH | A02 | a1 | B02 | b2 | C06 | COOH |
| 2-2-h10C06-1-0006 | 430.133 | OH | A02 | a1 | B03 | b2 | C06 | COOH |
| 2-2-h10C06-1-0007 | 443.094 | OH | A01 | a1 | B04 | b2 | C06 | COOH |
| 2-2-h10C06-1-0008 | 454.114 | OH | A01 | a1 | B05 | b2 | C06 | COOH |
| 2-2-h10C06-1-0009 | 469.110 | OH | A02 | a1 | B04 | b2 | C06 | COOH |
| 2-2-h10C06-1-0010 | 480.130 | OH | A02 | a1 | B05 | b2 | C06 | COOH |

[Table 481]

| [Table 9-3-24] Compound that may be contained in mixture 2-2-h23C16-1 | | | | | | | | |
|---|---|---|---|---|---|---|---|---|
| ID | Exact Mass | | A | a | B | b | C | c |
| 2-2-h23C16-1-0001 | 391.153 | OH | A01 | a1 | B01 | b1 | C16 | COOH |
| 2-2-h23C16-1-0002 | 405.169 | OH | A01 | a1 | B02 | b1 | C16 | COOH |
| 2-2-h23C16-1-0003 | 409.144 | OH | A01 | a1 | B03 | b1 | C16 | COOH |
| 2-2-h23C16-1-0004 | 417.169 | OH | A02 | a1 | B01 | b1 | C16 | COOH |
| 2-2-h23C16-1-0005 | 431.185 | OH | A02 | a1 | B02 | b1 | C16 | COOH |
| 2-2-h23C16-1-0006 | 435.159 | OH | A02 | a1 | B03 | b1 | C16 | COOH |
| 2-2-h23C16-1-0007 | 448.121 | OH | A01 | a1 | B04 | b1 | C16 | COOH |
| 2-2-h23C16-1-0008 | 459.141 | OH | A01 | a1 | B05 | b1 | C16 | COOH |
| 2-2-h23C16-1-0009 | 474.136 | OH | A02 | a1 | B04 | b1 | C16 | COOH |
| 2-2-h23C16-1-0010 | 485.156 | OH | A02 | a1 | B05 | b1 | C16 | COOH |

[Table 482]

| [Table 9-3-25] Compound that may be contained in mixture 2-2-h25C 18-1 | | | | | | | | |
|---|---|---|---|---|---|---|---|---|
| ID | Exact Mass | | A | a | B | b | C | c |
| 2-2-h25C18-1-0001 | 397.110 | OH | A01 | a1 | B01 | b1 | C18 | COOH |
| 2-2-h25C18-1-0002 | 411.125 | OH | A01 | a1 | B02 | b1 | C18 | COOH |
| 2-2-h25C18-1-0003 | 415.100 | OH | A01 | a1 | B03 | b1 | C18 | COOH |
| 2-2-h25C18-1-0004 | 423.125 | OH | A02 | a1 | B01 | b1 | C18 | COOH |
| 2-2-h25C18-1-0005 | 437.141 | OH | A02 | a1 | B02 | b1 | C18 | COOH |
| 2-2-h25C18-1-0006 | 441.116 | OH | A02 | a1 | B03 | b1 | C18 | COOH |
| 2-2-h25C18-1-0007 | 454.077 | OH | A01 | a1 | B04 | b1 | C18 | COOH |
| 2-2-h25C18-1-0008 | 465.097 | OH | A01 | a1 | B05 | b1 | C18 | COOH |

(continued)

| [Table 9-3-25] Compound that may be contained in mixture 2-2-h25C 18-1 | | | | | | | | |
|---|---|---|---|---|---|---|---|---|
| ID | Exact Mass | | A | a | B | b | C | c |
| 2-2-h25C18-1-0009 | 480.093 | OH | A02 | a1 | B04 | b1 | C18 | COOH |
| 2-2-h25C18-1-0010 | 491.113 | OH | A02 | a1 | B05 | b1 | C18 | COOH |

Example 9-3-3: Synthesis of 2-2-h22C15-1 by hydrolysis step h

NMP (1.79 mL), tAmylOH (0.512 mL), and a 1 M aqueous TBAOH solution (0.074 mL, 0.074 mmol) were added to a 6 mL column with a filter containing mixture 2-2- b1C15-0 obtained in Example 9-2-8. The column was capped so as not to leak the reaction solution, and shaken at room temperature for 3 hours.

The suspension of the reaction solution and the solid phase was filtered through the filter of the column and washed three times with NMP/water = 1/1 (2.6 mL), three times with 0.05 M $TBAHSO_4$ + DTBP in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure.

NMP (1.79 mL), tAmylOH (0.512 mL), and a 1 M aqueous TBAOH solution (0.124 mL, 0.124 mmol) were added thereto. The column was capped so as not to leak the reaction solution, and shaken at 60°C for 17 hours.

Solid-phase purification

The suspension of the reaction solution and the solid phase was filtered through the filter of the column and washed three times with NMP/water = 1/1 (2.6 mL), three times with 0.05 M $TBAHSO_4$ + DTBP in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain mixture 2-2-h22C15-0.

Mixture 2-2-h22C15-0 can be treated by cleavage in the same manner as in step a1 to obtain mixture 2-2-h22C15-1. The details of the mixture are shown in Table 9-3-26.

A notation method in each table will be described. For example, in Table 9-3-26, each compound that may be contained in 2-2-h22C15-1 is indicated by ID, and core blocks and linkers in combination in the structure of mixture 2-2-h22C15-1 are indicated by symbols or chemical formulas. The correspondence between the symbols and specific structures are shown in Figure 6.

In Table 9-3-26, "×" and "c" are represented by chemical formulas.

[Formula 680]

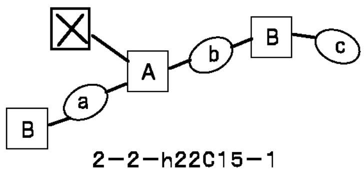


2-2-h22C15-1

[Table 483]

| [Table 9-3-26] Compound that may be contained in mixture 2-2-h22C15-1 | | | | | | | | |
|---|---|---|---|---|---|---|---|---|
| ID | Exact Mass | | A | a | B | b | C | c |
| 2-2-h22C15-1-0001 | 390.158 | OH | A01 | a1 | B01 | b1 | C15 | COOH |
| 2-2-h22C15-1-0002 | 404.174 | OH | A01 | a1 | B02 | b1 | C15 | COOH |
| 2-2-h22C15-1-0003 | 408.149 | OH | A01 | a1 | B03 | b1 | C15 | COOH |
| 2-2-h22C15-1-0004 | 416.174 | OH | A02 | a1 | B01 | b1 | C15 | COOH |
| 2-2-h22C15-1-0005 | 430.189 | OH | A02 | a1 | B02 | b1 | C15 | COOH |

(continued)

| [Table 9-3-26] Compound that may be contained in mixture 2-2-h22C15-1 | | | | | | | | |
|---|---|---|---|---|---|---|---|---|
| ID | Exact Mass | | A | a | B | b | C | c |
| 2-2-h22C15-1-0006 | 434.164 | OH | A02 | a1 | B03 | b1 | C15 | COOH |
| 2-2-h22C15-1-0007 | 447.125 | OH | A01 | a1 | B04 | b1 | C15 | COOH |
| 2-2-h22C15-1-0008 | 458.145 | OH | A01 | a1 | B05 | b1 | C15 | COOH |
| 2-2-h22C15-1-0009 | 473.141 | OH | A02 | a1 | B04 | b1 | C15 | COOH |
| 2-2-h22C15-1-0010 | 484.161 | OH | A02 | a1 | B05 | b1 | C15 | COOH |

Example 9-3-4: Synthesis of 2-2-h26C19-0 by hydrolysis step h

NMP (1.79 mL), tAmylOH (0.512 mL), and a 1 M aqueous TBAOH solution (0.124 mL, 0.124 mmol) were added to a 6 mL column with a filter containing mixture 2-2- b1C19-0 obtained in Example 9-2-10. The column was capped so as not to leak the reaction solution, and shaken at 60°C for 14 hours.

Solid-phase purification

The suspension of the reaction solution and the solid phase was filtered through the filter of the column and washed three times with NMP/water = 1/1 (2.6 mL), three times with 0.05 M $TBAHSO_4$ + DTBP in NMP (2.6 mL), three times with NMP/water = 1/1 (2.6 mL), three times with NMP (2.6 mL), three times with MeOH (2.8 mL), three times with DCM (2.8 mL), and three times with heptane (2.8 mL), and the obtained solid phase was dried under reduced pressure to obtain mixture 2-2-h26C19-0.

Mixture 2-2-h26C19-0 can be treated by cleavage in the same manner as in step a1 to obtain mixture 2-2-h26C19-1. The details of the mixture are shown in Table 9-3-27.

A notation method in each table will be described. For example, in Table 9-3-27, each compound that may be contained in 2-2-h26C19-1 is indicated by ID, and core blocks and linkers in combination in the structure of mixture 2-2-h26C19-1 are indicated by symbols or chemical formulas. The correspondence between the symbols and specific structures are shown in Figure 6.

In Table 9-3-27, "×" and "c" are represented by chemical formulas.

[Formula 681]

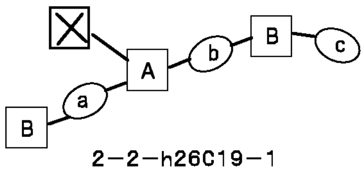

2−2−h26C19−1

[Table 484]

| [Table 9-3-27] Compound that may be contained in mixture 2-2-h26C19-1 | | | | | | | | |
|---|---|---|---|---|---|---|---|---|
| ID | Exact Mass | | A | a | B | b | C | c |
| 2-2-h26C19-1-0001 | 445.200 | OH | A01 | a1 | B01 | b1 | C19 | COOH |
| 2-2-h26C19-1-0002 | 459.216 | OH | A01 | a1 | B02 | b1 | C19 | COOH |
| 2-2-h26C19-1-0003 | 463.191 | OH | A01 | a1 | B03 | b1 | C19 | COOH |
| 2-2-h26C19-1-0004 | 471.216 | OH | A02 | a1 | B01 | b1 | C19 | COOH |
| 2-2-h26C19-1-0005 | 485.231 | OH | A02 | a1 | B02 | b1 | C19 | COOH |
| 2-2-h26C19-1-0006 | 489.206 | OH | A02 | a1 | B03 | b1 | C19 | COOH |
| 2-2-h26C19-1-0007 | 502.167 | OH | A01 | a1 | B04 | b1 | C19 | COOH |
| 2-2-h26C19-1-0008 | 513.188 | OH | A01 | a1 | B05 | b1 | C19 | COOH |

(continued)

| [Table 9-3-27] Compound that may be contained in mixture 2-2-h26C19-1 | | | | | | | | |
|---|---|---|---|---|---|---|---|---|
| ID | Exact Mass | | A | a | B | b | C | c |
| 2-2-h26C19-1-0009 | 528.183 | OH | A02 | a1 | B04 | b1 | C19 | COOH |
| 2-2-h26C19-1-0010 | 539.203 | OH | A02 | a1 | B05 | b1 | C19 | COOH |

Example 9-4: Formation of linkers c1 and c2 by step c

Example 9-4-1: Formation of amide linker by step c1D01

Amidation

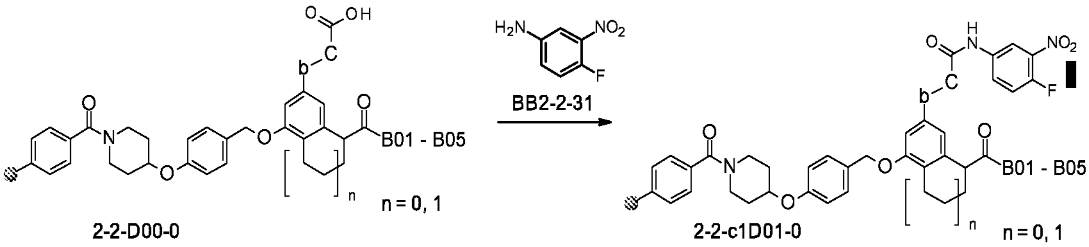


2-2-D00-0 2-2-c1D01-0

25 types of mixtures described in Table 9-4-1 were added to a 20 mL glass vial to prepare 2-2-D00-0. Then, DCM (12.0 mL) was added thereto, and the mixture was shaken at room temperature for 1 hour. 4-Fluoro-3-nitroaniline (BB2-2-31) (0.242 g, 1.55 mmol) was added thereto. A mixed solution of PipClU (0.224 g, 0.621 mmol), NMI (0.049 mL, 0.621 mmol), and MeCN (0.50 mL) was added thereto, and the mixture was shaken at room temperature for 17 hours.

Solid-phase purification

The suspension of the reaction solution and the solid phase was transferred to a column with a filter, filtered, and washed three times with NMP/water = 1/1 (16 mL), three times with 0.05 M $TBAHSO_4$ + DTBP in NMP (16 mL), three times with 0.05 M NMM in NMP (16 mL), three times with NMP/water = 1/1 (16 mL), three times with NMP (16 mL), three times with MeOH (16 mL), three times with DCM (16 mL), and three times with heptane (16 mL), and the obtained solid phase was dried under reduced pressure to obtain mixture 2-2-c1D01-0.

[Table 485]

| [Table 9-4-1] | |
|---|---|
| No. of mixture used | Amount used |
| 2-2-h03C02-0 | 32 mg |
| 2-2-h05C04-0 | 32 mg |
| 2-2-h04C03-0 | 32 mg |
| 2-2-h06C05-0 | 32 mg |
| 2-2-h02C01-0 | 32 mg |
| 2-2-h08C03-0 | 32 mg |
| 2-2-h07C02-0 | 32 mg |
| 2-2-h09C04-0 | 32 mg |
| 2-2-h11C07-0 | 32 mg |
| 2-2-h10C06-0 | 32 mg |

(continued)

| [Table 9-4-1] | |
|---|---|
| No. of mixture used | Amount used |
| 2-2-h16C12-0 | 32 mg |
| 2-2-h17C13-0 | 32 mg |
| 2-2-h19C02-0 | 32 mg |
| 2-2-h18C01-0 | 32 mg |
| 2-2-h20C05-0 | 32 mg |
| 2-2-h15C11-0 | 32 mg |
| 2-2-h12C08-0 | 32 mg |
| 2-2-h13C09-0 | 32 mg |
| 2-2-h14C10-0 | 32 mg |
| 2-2-h22C15-0 | 32 mg |
| 2-2-h23C16-0 | 32 mg |
| 2-2-h24C17-0 | 32 mg |
| 2-2-h21C14-0 | 32 mg |
| 2-2-h25C18-0 | 32 mg |
| 2-2-h26C 19-0 | 32 mg |

The obtained mixtures in Table 9-4-1 can be treated by cleavage in the same manner as in step a1 to respectively obtain mixtures in a state cleaved from the solid phase. The details of the mixtures are shown in Table 9-4-2.

A notation method in each table will be described. For example, in Table 9-4-2, each compound that may be contained in 2-2-c1D01-1 is indicated by ID, and core blocks and linkers in combination in the structure of mixture 2-2-c1D01-1 are indicated by symbols or chemical formulas. The correspondence between the symbols and specific structures are shown in Figure 6.

In Table 9-4-2, "×" and "d" are represented by chemical formulas.

[Formula 682]

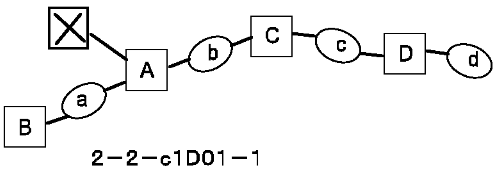


2-2-c1D01-1

For example, when ID is 2-2-c1D01-1-0001, the compound is represented by the following structural formula.

[Formula 683]

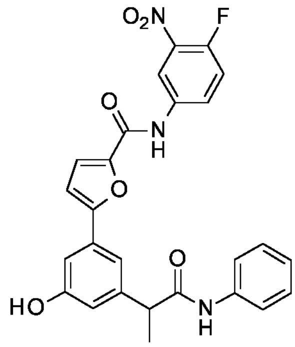

2-2-c1D01-1-0001

[Table 486]

| Exemplary notation in [Table 9-4-2] | | | | | | | | | | |
|---|---|---|---|---|---|---|---|---|---|---|
| ID | Exact Mass | | A | a | B | b | C | c | D | d |
| 2-2-c1D01-1-0001 | 489.134 | OH | A01 | a1 | B01 | b1 | C01 | c1 | D01 | F |

[Table 486-1]

| [Table 9-4-2] Compound that may be contained in mixture 2-2-c1D01-1 | | | | | | | | | | |
|---|---|---|---|---|---|---|---|---|---|---|
| ID | Exact Mass | | A | a | B | b | C | c | D | d |
| 2-2-c1D01-1-0001 | 489.134 | OH | A01 | a1 | B01 | b1 | C01 | c1 | D01 | F |
| 2-2-c1D01-1-0002 | 493.201 | OH | A01 | a1 | B01 | b4 | C12 | c1 | D01 | F |
| 2-2-c1D01-1-0003 | 495.181 | OH | A01 | a1 | B01 | b4 | C13 | c1 | D01 | F |
| 2-2-c1D01-1-0004 | 499.154 | OH | A01 | a1 | B01 | b1 | C02 | c1 | D01 | F |
| 2-2-c1D01-1-0005 | 499.154 | OH | A01 | a1 | B01 | b1 | C03 | c1 | D01 | F |
| 2-2-c1D01-1-0006 | 500.150 | OH | A01 | a1 | B01 | b1 | C04 | c1 | D01 | F |
| 2-2-c1D01-1-0007 | 503.149 | OH | A01 | a1 | B02 | b1 | C01 | c1 | D01 | F |
| 2-2-c1D01-1-0008 | 505.111 | OH | A01 | a1 | B01 | b1 | C05 | c1 | D01 | F |
| 2-2-c1D01-1-0009 | 506.124 | OH | A01 | a1 | B01 | b3 | C08 | c1 | D01 | F |
| 2-2-c1D01-1-0010 | 507.124 | OH | A01 | a1 | B03 | b1 | C01 | c1 | D01 | F |
| 2-2-c1D01-1-0011 | 507.217 | OH | A01 | a1 | B02 | b4 | C12 | c1 | D01 | F |
| 2-2-c1D01-1-0012 | 509.196 | OH | A01 | a1 | B02 | b4 | C13 | c1 | D01 | F |
| 2-2-c1D01-1-0013 | 511.192 | OH | A01 | a1 | B03 | b4 | C12 | c1 | D01 | F |
| 2-2-c1D01-1-0014 | 513.170 | OH | A01 | a1 | B02 | b1 | C02 | c1 | D01 | F |
| 2-2-c1D01-1-0015 | 513.170 | OH | A01 | a1 | B02 | b1 | C03 | c1 | D01 | F |
| 2-2-c1D01-1-0016 | 513.171 | OH | A01 | a1 | B03 | b4 | C13 | c1 | D01 | F |
| 2-2-c1D01-1-0017 | 514.165 | OH | A01 | a1 | B02 | b1 | C04 | c1 | D01 | F |
| 2-2-c1D01-1-0018 | 515.149 | OH | A02 | a1 | B01 | b1 | C01 | c1 | D01 | F |
| 2-2-c1D01-1-0019 | 517.140 | OH | A01 | a1 | B01 | b3 | C09 | c1 | D01 | F |

(continued)

| [Table 9-4-2] Compound that may be contained in mixture 2-2-c1D01-1 | | | | | | | | | | |
|---|---|---|---|---|---|---|---|---|---|---|
| ID | Exact Mass | | A | a | B | b | C | c | D | d |
| 2-2-c1D01-1-0020 | 517.145 | OH | A01 | a1 | B03 | b1 | C02 | c1 | D01 | F |
| 2-2-c1D01-1-0021 | 517.145 | OH | A01 | a1 | B03 | b1 | C03 | c1 | D01 | F |
| 2-2-c1D01-1-0022 | 518.140 | OH | A01 | a1 | B03 | b1 | C04 | c1 | D01 | F |
| 2-2-c1D01-1-0023 | 519.126 | OH | A01 | a1 | B02 | b1 | C05 | c1 | D01 | F |
| 2-2-c1D01-1-0024 | 519.155 | OH | A01 | a1 | B01 | b1 | C14 | c1 | D01 | F |
| 2-2-c1D01-1-0025 | 519.217 | OH | A02 | a1 | B01 | b4 | C12 | c1 | D01 | F |
| 2-2-c1D01-1-0026 | 520.139 | OH | A01 | a1 | B02 | b3 | C08 | c1 | D01 | F |
| 2-2-c1D01-1-0027 | 521.196 | OH | A02 | a1 | B01 | b4 | C13 | c1 | D01 | F |
| 2-2-c1D01-1-0028 | 522.101 | OH | A01 | a1 | B01 | b3 | C10 | c1 | D01 | F |
| 2-2-c1D01-1-0029 | 523.101 | OH | A01 | a1 | B03 | b1 | C05 | c1 | D01 | F |
| 2-2-c1D01-1-0030 | 523.154 | OH | A01 | a1 | B01 | b2 | C02 | c1 | D01 | F |
| 2-2-c1D01-1-0031 | 523.154 | OH | A01 | a1 | B01 | b2 | C03 | c1 | D01 | F |
| 2-2-c1D01-1-0032 | 524.114 | OH | A01 | a1 | B03 | b3 | C08 | c1 | D01 | F |
| 2-2-c1D01-1-0033 | 524.150 | OH | A01 | a1 | B01 | b2 | C04 | c1 | D01 | F |
| 2-2-c1D01-1-0034 | 524.150 | OH | A01 | a1 | B01 | b2 | C06 | c1 | D01 | F |
| 2-2-c1D01-1-0035 | 524.150 | OH | A01 | a1 | B01 | b2 | C07 | c1 | D01 | F |
| 2-2-c1D01-1-0036 | 525.170 | OH | A02 | a1 | B01 | b1 | C02 | c1 | D01 | F |
| 2-2-c1D01-1-0037 | 525.170 | OH | A02 | a1 | B01 | b1 | C03 | c1 | D01 | F |
| 2-2-c1D01-1-0038 | 526.165 | OH | A02 | a1 | B01 | b1 | C04 | c1 | D01 | F |
| 2-2-c1D01-1-0039 | 528.181 | OH | A01 | a1 | B01 | b1 | C15 | c1 | D01 | F |
| 2-2-c1D01-1-0040 | 529.165 | OH | A02 | a1 | B02 | b1 | C01 | c1 | D01 | F |
| 2-2-c1D01-1-0041 | 529.176 | OH | A01 | a1 | B01 | b1 | C16 | c1 | D01 | F |
| 2-2-c1D01-1-0042 | 530.171 | OH | A01 | a1 | B01 | b1 | C17 | c1 | D01 | F |
| 2-2-c1D01-1-0043 | 531.126 | OH | A02 | a1 | B01 | b1 | C05 | c1 | D01 | F |
| 2-2-c1D01-1-0044 | 531.155 | OH | A01 | a1 | B02 | b3 | C09 | c1 | D01 | F |
| 2-2-c1D01-1-0045 | 531.181 | OH | A01 | a1 | B01 | b4 | C01 | c1 | D01 | F |
| 2-2-c1D01-1-0046 | 532.139 | OH | A02 | a1 | B01 | b3 | C08 | c1 | D01 | F |
| 2-2-c1D01-1-0047 | 533.140 | OH | A02 | a1 | B03 | b1 | C01 | c1 | D01 | F |
| 2-2-c1D01-1-0048 | 533.171 | OH | A01 | a1 | B02 | b1 | C14 | c1 | D01 | F |
| 2-2-c1D01-1-0049 | 533.233 | OH | A02 | a1 | B02 | b4 | C12 | c1 | D01 | F |
| 2-2-c1D01-1-0050 | 535.130 | OH | A01 | a1 | B03 | b3 | C09 | c1 | D01 | F |
| 2-2-c1D01-1-0051 | 535.133 | OH | A01 | a1 | B01 | b1 | C18 | c1 | D01 | F |
| 2-2-c1D01-1-0052 | 535.212 | OH | A02 | a1 | B02 | b4 | C13 | c1 | D01 | F |
| 2-2-c1D01-1-0053 | 536.117 | OH | A01 | a1 | B02 | b3 | C10 | c1 | D01 | F |
| 2-2-c1D01-1-0054 | 537.146 | OH | A01 | a1 | B03 | b1 | C14 | c1 | D01 | F |
| 2-2-c1D01-1-0055 | 537.170 | OH | A01 | a1 | B02 | b2 | C02 | c1 | D01 | F |
| 2-2-c1D01-1-0056 | 537.170 | OH | A01 | a1 | B02 | b2 | C03 | c1 | D01 | F |

(continued)

| [Table 9-4-2] Compound that may be contained in mixture 2-2-c1D01-1 | | | | | | | | | | |
|---|---|---|---|---|---|---|---|---|---|---|
| ID | Exact Mass | | A | a | B | b | C | c | D | d |
| 2-2-c1D01-1-0057 | 537.208 | OH | A02 | a1 | B03 | b4 | C12 | c1 | D01 | F |
| 2-2-c1D01-1-0058 | 538.165 | OH | A01 | a1 | B02 | b2 | C04 | c1 | D01 | F |
| 2-2-c1D01-1-0059 | 538.165 | OH | A01 | a1 | B02 | b2 | C06 | c1 | D01 | F |
| 2-2-c1D01-1-0060 | 538.165 | OH | A01 | a1 | B02 | b2 | C07 | c1 | D01 | F |
| 2-2-c1D01-1-0061 | 539.186 | OH | A02 | a1 | B02 | b1 | C02 | c1 | D01 | F |
| 2-2-c1D01-1-0062 | 539.186 | OH | A02 | a1 | B02 | b1 | C03 | c1 | D01 | F |
| 2-2-c1D01-1-0063 | 539.187 | OH | A02 | a1 | B03 | b4 | C13 | c1 | D01 | F |
| 2-2-c1D01-1-0064 | 540.092 | OH | A01 | a1 | B03 | b3 | C10 | c1 | D01 | F |
| 2-2-c1D01-1-0065 | 540.181 | OH | A02 | a1 | B02 | b1 | C04 | c1 | D01 | F |
| 2-2-c1D01-1-0066 | 541.145 | OH | A01 | a1 | B03 | b2 | C02 | c1 | D01 | F |
| 2-2-c1D01-1-0067 | 541.145 | OH | A01 | a1 | B03 | b2 | C03 | c1 | D01 | F |
| 2-2-c1D01-1-0068 | 541.201 | OH | A01 | a1 | B01 | b4 | C02 | c1 | D01 | F |
| 2-2-c1D01-1-0069 | 542.140 | OH | A01 | a1 | B03 | b2 | C04 | c1 | D01 | F |
| 2-2-c1D01-1-0070 | 542.140 | OH | A01 | a1 | B03 | b2 | C06 | c1 | D01 | F |
| 2-2-c1D01-1-0071 | 542.140 | OH | A01 | a1 | B03 | b2 | C07 | c1 | D01 | F |
| 2-2-c1D01-1-0072 | 542.197 | OH | A01 | a1 | B02 | b1 | C15 | c1 | D01 | F |
| 2-2-c1D01-1-0073 | 543.155 | OH | A02 | a1 | B01 | b3 | C09 | c1 | D01 | F |
| 2-2-c1D01-1-0074 | 543.161 | OH | A02 | a1 | B03 | b1 | C02 | c1 | D01 | F |
| 2-2-c1D01-1-0075 | 543.161 | OH | A02 | a1 | B03 | b1 | C03 | c1 | D01 | F |
| 2-2-c1D01-1-0076 | 543.192 | OH | A01 | a1 | B02 | b1 | C16 | c1 | D01 | F |
| 2-2-c1D01-1-0077 | 544.156 | OH | A02 | a1 | B03 | b1 | C04 | c1 | D01 | F |
| 2-2-c1D01-1-0078 | 544.187 | OH | A01 | a1 | B02 | b1 | C17 | c1 | D01 | F |
| 2-2-c1D01-1-0079 | 545.142 | OH | A02 | a1 | B02 | b1 | C05 | c1 | D01 | F |
| 2-2-c1D01-1-0080 | 545.171 | OH | A02 | a1 | B01 | b1 | C14 | c1 | D01 | F |
| 2-2-c1D01-1-0081 | 545.196 | OH | A01 | a1 | B02 | b4 | C01 | c1 | D01 | F |
| 2-2-c1D01-1-0082 | 546.101 | OH | A01 | a1 | B04 | b1 | C01 | c1 | D01 | F |
| 2-2-c1D01-1-0083 | 546.155 | OH | A02 | a1 | B02 | b3 | C08 | c1 | D01 | F |
| 2-2-c1D01-1-0084 | 546.171 | OH | A01 | a1 | B03 | b1 | C15 | c1 | D01 | F |
| 2-2-c1D01-1-0085 | 547.158 | OH | A01 | a1 | B01 | b4 | C05 | c1 | D01 | F |
| 2-2-c1D01-1-0086 | 547.167 | OH | A01 | a1 | B03 | b1 | C16 | c1 | D01 | F |
| 2-2-c1D01-1-0087 | 548.117 | OH | A02 | a1 | B01 | b3 | C10 | c1 | D01 | F |
| 2-2-c1D01-1-0088 | 548.162 | OH | A01 | a1 | B03 | b1 | C17 | c1 | D01 | F |
| 2-2-c1D01-1-0089 | 549.117 | OH | A02 | a1 | B03 | b1 | C05 | c1 | D01 | F |
| 2-2-c1D01-1-0090 | 549.148 | OH | A01 | a1 | B02 | b1 | C18 | c1 | D01 | F |
| 2-2-c1D01-1-0091 | 549.170 | OH | A02 | a1 | B01 | b2 | C02 | c1 | D01 | F |
| 2-2-c1D01-1-0092 | 549.170 | OH | A02 | a1 | B01 | b2 | C03 | c1 | D01 | F |
| 2-2-c1D01-1-0093 | 549.171 | OH | A01 | a1 | B03 | b4 | C01 | c1 | D01 | F |
| 2-2-c1D01-1-0094 | 550.130 | OH | A02 | a1 | B03 | b3 | C08 | c1 | D01 | F |

(continued)

| [Table 9-4-2] Compound that may be contained in mixture 2-2-c1D01-1 | | | | | | | | | | |
|---|---|---|---|---|---|---|---|---|---|---|
| ID | Exact Mass | | A | a | B | b | C | c | D | d |
| 2-2-c1D01-1-0095 | 550.165 | OH | A02 | a1 | B01 | b2 | C04 | c1 | D01 | F |
| 2-2-c1D01-1-0096 | 550.165 | OH | A02 | a1 | B01 | b2 | C06 | c1 | D01 | F |
| 2-2-c1D01-1-0097 | 550.165 | OH | A02 | a1 | B01 | b2 | C07 | c1 | D01 | F |
| 2-2-c1D01-1-0098 | 550.169 | OH | A01 | a1 | B04 | b4 | C12 | c1 | D01 | F |
| 2-2-c1D01-1-0099 | 552.148 | OH | A01 | a1 | B04 | b4 | C13 | c1 | D01 | F |
| 2-2-c1D01-1-0100 | 553.123 | OH | A01 | a1 | B03 | b1 | C18 | c1 | D01 | F |
| 2-2-c1D01-1-0101 | 554.197 | OH | A02 | a1 | B01 | b1 | C15 | c1 | D01 | F |
| 2-2-c1D01-1-0102 | 555.144 | OH | A01 | a1 | B01 | b3 | C11 | c1 | D01 | F |
| 2-2-c1D01-1-0103 | 555.192 | OH | A02 | a1 | B01 | b1 | C16 | c1 | D01 | F |
| 2-2-c1D01-1-0104 | 555.217 | OH | A01 | a1 | B02 | b4 | C02 | c1 | D01 | F |
| 2-2-c1D01-1-0105 | 556.122 | OH | A01 | a1 | B04 | b1 | C02 | c1 | D01 | F |
| 2-2-c1D01-1-0106 | 556.122 | OH | A01 | a1 | B04 | b1 | C03 | c1 | D01 | F |
| 2-2-c1D01-1-0107 | 556.187 | OH | A02 | a1 | B01 | b1 | C17 | c1 | D01 | F |
| 2-2-c1D01-1-0108 | 557.117 | OH | A01 | a1 | B04 | b1 | C04 | c1 | D01 | F |
| 2-2-c1D01-1-0109 | 557.121 | OH | A01 | a1 | B05 | b1 | C01 | c1 | D01 | F |
| 2-2-c1D01-1-0110 | 557.171 | OH | A02 | a1 | B02 | b3 | C09 | c1 | D01 | F |
| 2-2-c1D01-1-0111 | 557.196 | OH | A02 | a1 | B01 | b4 | C01 | c1 | D01 | F |
| 2-2-c1D01-1-0112 | 559.187 | OH | A02 | a1 | B02 | b1 | C14 | c1 | D01 | F |
| 2-2-c1D01-1-0113 | 559.192 | OH | A01 | a1 | B03 | b4 | C02 | c1 | D01 | F |
| 2-2-c1D01-1-0114 | 561.146 | OH | A02 | a1 | B03 | b3 | C09 | c1 | D01 | F |
| 2-2-c1D01-1-0115 | 561.148 | OH | A02 | a1 | B01 | b1 | C18 | c1 | D01 | F |
| 2-2-c1D01-1-0116 | 561.173 | OH | A01 | a1 | B02 | b4 | C05 | c1 | D01 | F |
| 2-2-c1D01-1-0117 | 561.189 | OH | A01 | a1 | B05 | b4 | C12 | c1 | D01 | F |
| 2-2-c1D01-1-0118 | 562.078 | OH | A01 | a1 | B04 | b1 | C05 | c1 | D01 | F |
| 2-2-c1D01-1-0119 | 562.132 | OH | A02 | a1 | B02 | b3 | C10 | c1 | D01 | F |
| 2-2-c1D01-1-0120 | 563.091 | OH | A01 | a1 | B04 | b3 | C08 | c1 | D01 | F |
| 2-2-c1D01-1-0121 | 563.162 | OH | A02 | a1 | B03 | b1 | C14 | c1 | D01 | F |
| 2-2-c1D01-1-0122 | 563.168 | OH | A01 | a1 | B05 | b4 | C13 | c1 | D01 | F |
| 2-2-c1D01-1-0123 | 563.186 | OH | A02 | a1 | B02 | b2 | C02 | c1 | D01 | F |
| 2-2-c1D01-1-0124 | 563.186 | OH | A02 | a1 | B02 | b2 | C03 | c1 | D01 | F |
| 2-2-c1D01-1-0125 | 564.181 | OH | A02 | a1 | B02 | b2 | C04 | c1 | D01 | F |
| 2-2-c1D01-1-0126 | 564.181 | OH | A02 | a1 | B02 | b2 | C06 | c1 | D01 | F |
| 2-2-c1D01-1-0127 | 564.181 | OH | A02 | a1 | B02 | b2 | C07 | c1 | D01 | F |
| 2-2-c1D01-1-0128 | 565.148 | OH | A01 | a1 | B03 | b4 | C05 | c1 | D01 | F |
| 2-2-c1D01-1-0129 | 566.107 | OH | A02 | a1 | B03 | b3 | C10 | c1 | D01 | F |
| 2-2-c1D01-1-0130 | 567.142 | OH | A01 | a1 | B05 | b1 | C02 | c1 | D01 | F |
| 2-2-c1D01-1-0131 | 567.142 | OH | A01 | a1 | B05 | b1 | C03 | c1 | D01 | F |

(continued)

| [Table 9-4-2] Compound that may be contained in mixture 2-2-c1D01-1 | | | | | | | | | | |
|---|---|---|---|---|---|---|---|---|---|---|
| ID | Exact Mass | | A | a | B | b | C | c | D | d |
| 2-2-c1D01-1-0132 | 567.161 | OH | A02 | a1 | B03 | b2 | C02 | c1 | D01 | F |
| 2-2-c1D01-1-0133 | 567.161 | OH | A02 | a1 | B03 | b2 | C03 | c1 | D01 | F |
| 2-2-c1D01-1-0134 | 567.217 | OH | A02 | a1 | B01 | b4 | C02 | c1 | D01 | F |
| 2-2-c1D01-1-0135 | 568.137 | OH | A01 | a1 | B05 | b1 | C04 | c1 | D01 | F |
| 2-2-c1D01-1-0136 | 568.156 | OH | A02 | a1 | B03 | b2 | C04 | c1 | D01 | F |
| 2-2-c1D01-1-0137 | 568.156 | OH | A02 | a1 | B03 | b2 | C06 | c1 | D01 | F |
| 2-2-c1D01-1-0138 | 568.156 | OH | A02 | a1 | B03 | b2 | C07 | c1 | D01 | F |
| 2-2-c1D01-1-0139 | 568.212 | OH | A02 | a1 | B02 | b1 | C15 | c1 | D01 | F |
| 2-2-c1D01-1-0140 | 569.160 | OH | A01 | a1 | B02 | b3 | C11 | c1 | D01 | F |
| 2-2-c1D01-1-0141 | 569.207 | OH | A02 | a1 | B02 | b1 | C16 | c1 | D01 | F |
| 2-2-c1D01-1-0142 | 570.203 | OH | A02 | a1 | B02 | b1 | C17 | c1 | D01 | F |
| 2-2-c1D01-1-0143 | 571.212 | OH | A02 | a1 | B02 | b4 | C01 | c1 | D01 | F |
| 2-2-c1D01-1-0144 | 572.117 | OH | A02 | a1 | B04 | b1 | C01 | c1 | D01 | F |
| 2-2-c1D01-1-0145 | 572.187 | OH | A02 | a1 | B03 | b1 | C15 | c1 | D01 | F |
| 2-2-c1D01-1-0146 | 573.098 | OH | A01 | a1 | B05 | b1 | C05 | c1 | D01 | F |
| 2-2-c1D01-1-0147 | 573.135 | OH | A01 | a1 | B03 | b3 | C11 | c1 | D01 | F |
| 2-2-c1D01-1-0148 | 573.173 | OH | A02 | a1 | B01 | b4 | C05 | c1 | D01 | F |
| 2-2-c1D01-1-0149 | 573.182 | OH | A02 | a1 | B03 | b1 | C16 | c1 | D01 | F |
| 2-2-c1D01-1-0150 | 574.107 | OH | A01 | a1 | B04 | b3 | C09 | c1 | D01 | F |
| 2-2-c1D01-1-0151 | 574.111 | OH | A01 | a1 | B05 | b3 | C08 | c1 | D01 | F |
| 2-2-c1D01-1-0152 | 574.178 | OH | A02 | a1 | B03 | b1 | C17 | c1 | D01 | F |
| 2-2-c1D01-1-0153 | 575.164 | OH | A02 | a1 | B02 | b1 | C18 | c1 | D01 | F |
| 2-2-c1D01-1-0154 | 575.187 | OH | A02 | a1 | B03 | b4 | C01 | c1 | D01 | F |
| 2-2-c1D01-1-0155 | 576.123 | OH | A01 | a1 | B04 | b1 | C14 | c1 | D01 | F |
| 2-2-c1D01-1-0156 | 576.184 | OH | A02 | a1 | B04 | b4 | C12 | c1 | D01 | F |
| 2-2-c1D01-1-0157 | 578.164 | OH | A02 | a1 | B04 | b4 | C13 | c1 | D01 | F |
| 2-2-c1D01-1-0158 | 579.068 | OH | A01 | a1 | B04 | b3 | C10 | c1 | D01 | F |
| 2-2-c1D01-1-0159 | 579.139 | OH | A02 | a1 | B03 | b1 | C18 | c1 | D01 | F |
| 2-2-c1D01-1-0160 | 580.122 | OH | A01 | a1 | B04 | b2 | C02 | c1 | D01 | F |
| 2-2-c1D01-1-0161 | 580.122 | OH | A01 | a1 | B04 | b2 | C03 | c1 | D01 | F |
| 2-2-c1D01-1-0162 | 581.117 | OH | A01 | a1 | B04 | b2 | C04 | c1 | D01 | F |
| 2-2-c1D01-1-0163 | 581.117 | OH | A01 | a1 | B04 | b2 | C06 | c1 | D01 | F |
| 2-2-c1D01-1-0164 | 581.117 | OH | A01 | a1 | B04 | b2 | C07 | c1 | D01 | F |
| 2-2-c1D01-1-0165 | 581.160 | OH | A02 | a1 | B01 | b3 | C11 | c1 | D01 | F |
| 2-2-c1D01-1-0166 | 581.233 | OH | A02 | a1 | B02 | b4 | C02 | c1 | D01 | F |
| 2-2-c1D01-1-0167 | 582.137 | OH | A02 | a1 | B04 | b1 | C02 | c1 | D01 | F |
| 2-2-c1D01-1-0168 | 582.137 | OH | A02 | a1 | B04 | b1 | C03 | c1 | D01 | F |

(continued)

| [Table 9-4-2] Compound that may be contained in mixture 2-2-c1D01-1 | | | | | | | | | | |
|---|---|---|---|---|---|---|---|---|---|---|
| ID | Exact Mass | | A | a | B | b | C | c | D | d |
| 2-2-c1D01-1-0169 | 583.133 | OH | A02 | a1 | B04 | b1 | C04 | c1 | D01 | F |
| 2-2-c1D01-1-0170 | 583.137 | OH | A02 | a1 | B05 | b1 | C01 | c1 | D01 | F |
| 2-2-c1D01-1-0171 | 583.223 | OH | A01 | a1 | B01 | b1 | C19 | c1 | D01 | F |
| 2-2-c1D01-1-0172 | 585.127 | OH | A01 | a1 | B05 | b3 | C09 | c1 | D01 | F |
| 2-2-c1D01-1-0173 | 585.148 | OH | A01 | a1 | B04 | b1 | C15 | c1 | D01 | F |
| 2-2-c1D01-1-0174 | 585.208 | OH | A02 | a1 | B03 | b4 | C02 | c1 | D01 | F |
| 2-2-c1D01-1-0175 | 586.143 | OH | A01 | a1 | B04 | b1 | C16 | c1 | D01 | F |
| 2-2-c1D01-1-0176 | 587.139 | OH | A01 | a1 | B04 | b1 | C17 | c1 | D01 | F |
| 2-2-c1D01-1-0177 | 587.143 | OH | A01 | a1 | B05 | b1 | C14 | c1 | D01 | F |
| 2-2-c1D01-1-0178 | 587.189 | OH | A02 | a1 | B02 | b4 | C05 | c1 | D01 | F |
| 2-2-c1D01-1-0179 | 587.204 | OH | A02 | a1 | B05 | b4 | C12 | c1 | D01 | F |
| 2-2-c1D01-1-0180 | 588.094 | OH | A02 | a1 | B04 | b1 | C05 | c1 | D01 | F |
| 2-2-c1D01-1-0181 | 588.148 | OH | A01 | a1 | B04 | b4 | C01 | c1 | D01 | F |
| 2-2-c1D01-1-0182 | 589.107 | OH | A02 | a1 | B04 | b3 | C08 | c1 | D01 | F |
| 2-2-c1D01-1-0183 | 589.184 | OH | A02 | a1 | B05 | b4 | C13 | c1 | D01 | F |
| 2-2-c1D01-1-0184 | 590.088 | OH | A01 | a1 | B05 | b3 | C10 | c1 | D01 | F |
| 2-2-c1D01-1-0185 | 591.142 | OH | A01 | a1 | B05 | b2 | C02 | c1 | D01 | F |
| 2-2-c1D01-1-0186 | 591.142 | OH | A01 | a1 | B05 | b2 | C03 | c1 | D01 | F |
| 2-2-c1D01-1-0187 | 591.164 | OH | A02 | a1 | B03 | b4 | C05 | c1 | D01 | F |
| 2-2-c1D01-1-0188 | 592.100 | OH | A01 | a1 | B04 | b1 | C18 | c1 | D01 | F |
| 2-2-c1D01-1-0189 | 592.137 | OH | A01 | a1 | B05 | b2 | C04 | c1 | D01 | F |
| 2-2-c1D01-1-0190 | 592.137 | OH | A01 | a1 | B05 | b2 | C06 | c1 | D01 | F |
| 2-2-c1D01-1-0191 | 592.137 | OH | A01 | a1 | B05 | b2 | C07 | c1 | D01 | F |
| 2-2-c1D01-1-0192 | 593.157 | OH | A02 | a1 | B05 | b1 | C02 | c1 | D01 | F |
| 2-2-c1D01-1-0193 | 593.157 | OH | A02 | a1 | B05 | b1 | C03 | c1 | D01 | F |
| 2-2-c1D01-1-0194 | 594.153 | OH | A02 | a1 | B05 | b1 | C04 | c1 | D01 | F |
| 2-2-c1D01-1-0195 | 595.175 | OH | A02 | a1 | B02 | b3 | C11 | c1 | D01 | F |
| 2-2-c1D01-1-0196 | 596.168 | OH | A01 | a1 | B05 | b1 | C15 | c1 | D01 | F |
| 2-2-c1D01-1-0197 | 597.164 | OH | A01 | a1 | B05 | b1 | C16 | c1 | D01 | F |
| 2-2-c1D01-1-0198 | 597.239 | OH | A01 | a1 | B02 | b1 | C19 | c1 | D01 | F |
| 2-2-c1D01-1-0199 | 598.159 | OH | A01 | a1 | B05 | b1 | C17 | c1 | D01 | F |
| 2-2-c1D01-1-0200 | 598.169 | OH | A01 | a1 | B04 | b4 | C02 | c1 | D01 | F |
| 2-2-clD01-1-0201 | 599.114 | OH | A02 | a1 | B05 | b1 | C05 | c1 | D01 | F |
| 2-2-c1D01-1-0202 | 599.150 | OH | A02 | a1 | B03 | b3 | C11 | c1 | D01 | F |
| 2-2-c1D01-1-0203 | 599.168 | OH | A01 | a1 | B05 | b4 | C01 | c1 | D01 | F |
| 2-2-c1D01-1-0204 | 600.123 | OH | A02 | a1 | B04 | b3 | C09 | c1 | D01 | F |
| 2-2-c1D01-1-0205 | 600.127 | OH | A02 | a1 | B05 | b3 | C08 | c1 | D01 | F |

(continued)

| [Table 9-4-2] Compound that may be contained in mixture 2-2-c1D01-1 | | | | | | | | | | |
|---|---|---|---|---|---|---|---|---|---|---|
| ID | Exact Mass | | A | a | B | b | C | c | D | d |
| 2-2-c1D01-1-0206 | 601.214 | OH | A01 | a1 | B03 | b1 | C19 | c1 | D01 | F |
| 2-2-c1D01-1-0207 | 602.138 | OH | A02 | a1 | B04 | b1 | C14 | c1 | D01 | F |
| 2-2-clD01-1-0208 | 603.120 | OH | A01 | a1 | B05 | b1 | C18 | c1 | D01 | F |
| 2-2-clD01-1-0209 | 604.125 | OH | A01 | a1 | B04 | b4 | C05 | c1 | D01 | F |
| 2-2-c1D01-1-0210 | 605.084 | OH | A02 | a1 | B04 | b3 | C10 | c1 | D01 | F |
| 2-2-c1D01-1-0211 | 606.137 | OH | A02 | a1 | B04 | b2 | C02 | c1 | D01 | F |
| 2-2-c1D01-1-0212 | 606.137 | OH | A02 | a1 | B04 | b2 | C03 | c1 | D01 | F |
| 2-2-c1D01-1-0213 | 607.133 | OH | A02 | a1 | B04 | b2 | C04 | c1 | D01 | F |
| 2-2-c1D01-1-0214 | 607.133 | OH | A02 | a1 | B04 | b2 | C06 | c1 | D01 | F |
| 2-2-c1D01-1-0215 | 607.133 | OH | A02 | a1 | B04 | b2 | C07 | c1 | D01 | F |
| 2-2-c1D01-1-0216 | 609.189 | OH | A01 | a1 | B05 | b4 | C02 | c1 | D01 | F |
| 2-2-c1D01-1-0217 | 609.239 | OH | A02 | a1 | B01 | b1 | C19 | c1 | D01 | F |
| 2-2-c1D01-1-0218 | 611.143 | OH | A02 | a1 | B05 | b3 | C09 | c1 | D01 | F |
| 2-2-c1D01-1-0219 | 611.164 | OH | A02 | a1 | B04 | b1 | C15 | c1 | D01 | F |
| 2-2-c1D01-1-0220 | 612.111 | OH | A01 | a1 | B04 | b3 | C11 | c1 | D01 | F |
| 2-2-c1D01-1-0221 | 612.159 | OH | A02 | a1 | B04 | b1 | C16 | c1 | D01 | F |
| 2-2-c1D01-1-0222 | 613.154 | OH | A02 | a1 | B04 | b1 | C17 | c1 | D01 | F |
| 2-2-c1D01-1-0223 | 613.158 | OH | A02 | a1 | B05 | b1 | C14 | c1 | D01 | F |
| 2-2-c1D01-1-0224 | 614.164 | OH | A02 | a1 | B04 | b4 | C01 | c1 | D01 | F |
| 2-2-c1D01-1-0225 | 615.145 | OH | A01 | a1 | B05 | b4 | C05 | c1 | D01 | F |
| 2-2-c1D01-1-0226 | 616.104 | OH | A02 | a1 | B05 | b3 | C10 | c1 | D01 | F |
| 2-2-c1D01-1-0227 | 617.157 | OH | A02 | a1 | B05 | b2 | C02 | c1 | D01 | F |
| 2-2-c1D01-1-0228 | 617.157 | OH | A02 | a1 | B05 | b2 | C03 | c1 | D01 | F |
| 2-2-c1D01-1-0229 | 618.116 | OH | A02 | a1 | B04 | b1 | C18 | c1 | D01 | F |
| 2-2-c1D01-1-0230 | 618.153 | OH | A02 | a1 | B05 | b2 | C04 | c1 | D01 | F |
| 2-2-c1D01-1-0231 | 618.153 | OH | A02 | a1 | B05 | b2 | C06 | c1 | D01 | F |
| 2-2-c1D01-1-0232 | 618.153 | OH | A02 | a1 | B05 | b2 | C07 | c1 | D01 | F |
| 2-2-c1D01-1-0233 | 622.184 | OH | A02 | a1 | B05 | b1 | C15 | c1 | D01 | F |
| 2-2-c1D01-1-0234 | 623.132 | OH | A01 | a1 | B05 | b3 | C11 | c1 | D01 | F |
| 2-2-c1D01-1-0235 | 623.179 | OH | A02 | a1 | B05 | b1 | C16 | c1 | D01 | F |
| 2-2-c1D01-1-0236 | 623.254 | OH | A02 | a1 | B02 | b1 | C19 | c1 | D01 | F |
| 2-2-c1D01-1-0237 | 624.174 | OH | A02 | a1 | B05 | b1 | C17 | c1 | D01 | F |
| 2-2-c1D01-1-0238 | 624.184 | OH | A02 | a1 | B04 | b4 | C02 | c1 | D01 | F |
| 2-2-c1D01-1-0239 | 625.184 | OH | A02 | a1 | B05 | b4 | C01 | c1 | D01 | F |
| 2-2-c1D01-1-0240 | 627.229 | OH | A02 | a1 | B03 | b1 | C19 | c1 | D01 | F |
| 2-2-c1D01-1-0241 | 629.136 | OH | A02 | a1 | B05 | b1 | C18 | c1 | D01 | F |
| 2-2-c1D01-1-0242 | 630.141 | OH | A02 | a1 | B04 | b4 | C05 | c1 | D01 | F |

(continued)

| [Table 9-4-2] Compound that may be contained in mixture 2-2-c1D01-1 | | | | | | | | | | |
|---|---|---|---|---|---|---|---|---|---|---|
| ID | Exact Mass | | A | a | B | b | C | c | D | d |
| 2-2-c1D01-1-0243 | 635.204 | OH | A02 | a1 | B05 | b4 | C02 | c1 | D01 | F |
| 2-2-c1D01-1-0244 | 638.127 | OH | A02 | a1 | B04 | b3 | C11 | c1 | D01 | F |
| 2-2-c1D01-1-0245 | 640.190 | OH | A01 | a1 | B04 | b1 | C19 | c1 | D01 | F |
| 2-2-c1D01-1-0246 | 641.161 | OH | A02 | a1 | B05 | b4 | C05 | c1 | D01 | F |
| 2-2-c1D01-1-0247 | 649.147 | OH | A02 | a1 | B05 | b3 | C11 | c1 | D01 | F |
| 2-2-c1D01-1-0248 | 651.210 | OH | A01 | a1 | B05 | b1 | C19 | c1 | D01 | F |
| 2-2-c1D01-1-0249 | 666.206 | OH | A02 | a1 | B04 | b1 | C19 | c1 | D01 | F |
| 2-2-c1D01-1-0250 | 677.226 | OH | A02 | a1 | B05 | b1 | C19 | c1 | D01 | F |

Example 9-4-2: Formation of acylsulfonamide linker by step c2D02

[Formula 684]

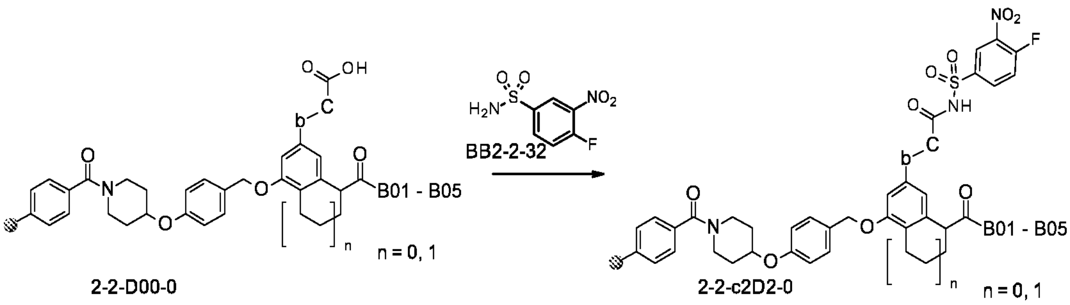


2-2-D00-0 2-2-c2D2-0

25 types of mixtures described in Table 9-4-3 were added to a 20 mL glass vial to prepare 2-2-D00-0. Then, NMP (12.0 mL) was added thereto, and the mixture was shaken at room temperature for 1 hour. 2,6-Lutidine (0.108 mL, 0.931 mmol) and HATU (0.354 g, 0.931 mmol) were added thereto, and the mixture was shaken at 45°C for 17 hours. 4- Fluoro-3-nitrobenzenesulfonamide (BB2-2-32) (0.239 g, 1.09 mmol) and PltBu (0.592 mL, 2.33 mmol) were added thereto, and the mixture was shaken at room temperature for 18 hours.

Solid-phase purification

The suspension of the reaction solution and the solid phase was transferred to a column with a filter, filtered, and washed three times with NMP/water = 1/1 (16 mL), three times with 0.05 M $TBAHSO_4$ + DTBP in NMP (16 mL), three times with 0.05 M NMM in NMP (16 mL), three times with NMP/water = 1/1 (16 mL), three times with NMP (16 mL), three times with MeOH (16 mL), three times with DCM (16 mL), and three times with heptane (16 mL), and the obtained solid phase was dried under reduced pressure to obtain mixture 2-2-c2D02-0.

[Table 487]

| [Table 9-4-3] | |
|---|---|
| No. of mixture used | Amount used |
| 2-2-h03C02-0 | 32 mg |
| 2-2-h05C04-0 | 32 mg |

(continued)

| [Table 9-4-3] | |
|---|---|
| No. of mixture used | Amount used |
| 2-2-h04C03-0 | 32 mg |
| 2-2-h06C05-0 | 32 mg |
| 2-2-h02C01-0 | 32 mg |
| 2-2-h08C03-0 | 32 mg |
| 2-2-h07C02-0 | 32 mg |
| 2-2-h09C04-0 | 32 mg |
| 2-2-h11C07-0 | 32 mg |
| 2-2-h10C06-0 | 32 mg |
| 2-2-h16C12-0 | 32 mg |
| 2-2-h17C13-0 | 32 mg |
| 2-2-h19C02-0 | 32 mg |
| 2-2-h18C01-0 | 32 mg |
| 2-2-h20C05-0 | 32 mg |
| 2-2-h15C11-0 | 32 mg |
| 2-2-h12C08-0 | 32 mg |
| 2-2-h13C09-0 | 32 mg |
| 2-2-h14C10-0 | 32 mg |
| 2-2-h22C15-0 | 64 mg |
| 2-2-h23C16-0 | 32 mg |
| 2-2-h24C17-0 | 32 mg |
| 2-2-h21C14-0 | 32 mg |
| 2-2-h25C18-0 | 32 mg |
| 2-2-h26C19-0 | 32 mg |

The obtained mixtures in Table 9-4-3 can be treated by cleavage in the same manner as in step a1 to respectively obtain mixtures in a state cleaved from the solid phase. The details of the mixtures are shown in Table 9-4-4.

A notation method in each table will be described. For example, in Table 9-4-4, each compound that may be contained in 2-2-c2D02-1 is indicated by ID, and core blocks and linkers in combination in the structure of mixture 2-2-c2D02-1 are indicated by symbols or chemical formulas. The correspondence between the symbols and specific structures are shown in Figure 6.

In Table 9-4-4, "×" and "d" are represented by chemical formulas.

[Formula 685]

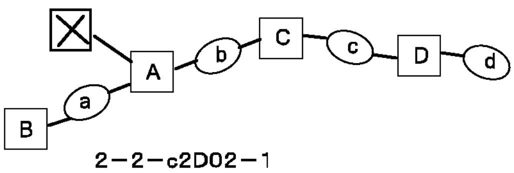


2−2−c2D02−1

For example, when ID is 2-2-c2D02-1-0001, the compound is represented by the following structural formula.

[Formula 686]

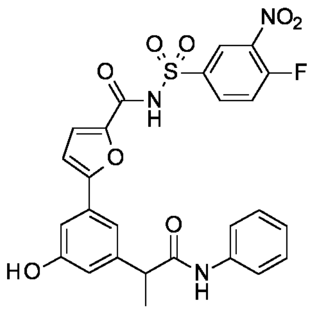

2-2-c2D02-1-0001

[Table 488]

| Exemplary notation in [Table 9-4-4] | | | | | | | | | | |
|---|---|---|---|---|---|---|---|---|---|---|
| ID | Exact Mass | | A | a | B | b | C | c | D | d |
| 2-2-c2D01-1-0001 | 553.096 | OH | A01 | a1 | B01 | b1 | C01 | c2 | D02 | F |

[Table 489]

| [Table 9-4-4] Compound that may be contained in mixture 2-2-c2D02-1 | | | | | | | | | | |
|---|---|---|---|---|---|---|---|---|---|---|
| ID | Exact Mass | | A | a | B | b | C | c | D | d |
| 2-2-c2D02-1-0001 | 553.096 | OH | A01 | a1 | B01 | b1 | C01 | c2 | D02 | F |
| 2-2-c2D02-1-0002 | 557.163 | OH | A01 | a1 | B01 | b4 | C12 | c2 | D02 | F |
| 2-2-c2D02-1-0003 | 559.142 | OH | A01 | a1 | B01 | b4 | C13 | c2 | D02 | F |
| 2-2-c2D02-1-0004 | 563.116 | OH | A01 | a1 | B01 | b1 | C02 | c2 | D02 | F |
| 2-2-c2D02-1-0005 | 563.116 | OH | A01 | a1 | B01 | b1 | C03 | c2 | D02 | F |
| 2-2-c2D02-1-0006 | 564.111 | OH | A01 | a1 | B01 | b1 | C04 | c2 | D02 | F |
| 2-2-c2D02-1-0007 | 567.111 | OH | A01 | a1 | B02 | b1 | C01 | c2 | D02 | F |
| 2-2-c2D02-1-0008 | 569.073 | OH | A01 | a1 | B01 | b1 | C05 | c2 | D02 | F |
| 2-2-c2D02-1-0009 | 570.086 | OH | A01 | a1 | B01 | b3 | C08 | c2 | D02 | F |
| 2-2-c2D02-1-0010 | 571.086 | OH | A01 | a1 | B03 | b1 | C01 | c2 | D02 | F |
| 2-2-c2D02-1-0011 | 571.179 | OH | A01 | a1 | B02 | b4 | C12 | c2 | D02 | F |
| 2-2-c2D02-1-0012 | 573.158 | OH | A01 | a1 | B02 | b4 | C13 | c2 | D02 | F |
| 2-2-c2D02-1-0013 | 575.154 | OH | A01 | a1 | B03 | b4 | C12 | c2 | D02 | F |
| 2-2-c2D02-1-0014 | 577.132 | OH | A01 | a1 | B02 | b1 | C02 | c2 | D02 | F |
| 2-2-c2D02-1-0015 | 577.132 | OH | A01 | a1 | B02 | b1 | C03 | c2 | D02 | F |
| 2-2-c2D02-1-0016 | 577.133 | OH | A01 | a1 | B03 | b4 | C13 | c2 | D02 | F |
| 2-2-c2D02-1-0017 | 578.127 | OH | A01 | a1 | B02 | b1 | C04 | c2 | D02 | F |
| 2-2-c2D02-1-0018 | 579.111 | OH | A02 | a1 | B01 | b1 | C01 | c2 | D02 | F |
| 2-2-c2D02-1-0019 | 581.102 | OH | A01 | a1 | B01 | b3 | C09 | c2 | D02 | F |
| 2-2-c2D02-1-0020 | 581.107 | OH | A01 | a1 | B03 | b1 | C02 | c2 | D02 | F |

(continued)

| [Table 9-4-4] Compound that may be contained in mixture 2-2-c2D02-1 | | | | | | | | | | |
|---|---|---|---|---|---|---|---|---|---|---|
| ID | Exact Mass | | A | a | B | b | C | c | D | d |
| 2-2-c2D02-1-0021 | 581.107 | OH | A01 | a1 | B03 | b1 | C03 | c2 | D02 | F |
| 2-2-c2D02-1-0022 | 582.102 | OH | A01 | a1 | B03 | b1 | C04 | c2 | D02 | F |
| 2-2-c2D02-1-0023 | 583.088 | OH | A01 | a1 | B02 | b1 | C05 | c2 | D02 | F |
| 2-2-c2D02-1-0024 | 583.117 | OH | A01 | a1 | B01 | b1 | C14 | c2 | D02 | F |
| 2-2-c2D02-1-0025 | 583.179 | OH | A02 | a1 | B01 | b4 | C12 | c2 | D02 | F |
| 2-2-c2D02-1-0026 | 584.101 | OH | A01 | a1 | B02 | b3 | C08 | c2 | D02 | F |
| 2-2-c2D02-1-0027 | 585.158 | OH | A02 | a1 | B01 | b4 | C13 | c2 | D02 | F |
| 2-2-c2D02-1-0028 | 586.063 | OH | A01 | a1 | B01 | b3 | C10 | c2 | D02 | F |
| 2-2-c2D02-1-0029 | 587.063 | OH | A01 | a1 | B03 | b1 | C05 | c2 | D02 | F |
| 2-2-c2D02-1-0030 | 587.116 | OH | A01 | a1 | B01 | b2 | C02 | c2 | D02 | F |
| 2-2-c2D02-1-0031 | 587.116 | OH | A01 | a1 | B01 | b2 | C03 | c2 | D02 | F |
| 2-2-c2D02-1-0032 | 588.076 | OH | A01 | a1 | B03 | b3 | C08 | c2 | D02 | F |
| 2-2-c2D02-1-0033 | 588.111 | OH | A01 | a1 | B01 | b2 | C04 | c2 | D02 | F |
| 2-2-c2D02-1-0034 | 588.111 | OH | A01 | a1 | B01 | b2 | C06 | c2 | D02 | F |
| 2-2-c2D02-1-0035 | 588.111 | OH | A01 | a1 | B01 | b2 | C07 | c2 | D02 | F |
| 2-2-c2D02-1-0036 | 589.132 | OH | A02 | a1 | B01 | b1 | C02 | c2 | D02 | F |
| 2-2-c2D02-1-0037 | 589.132 | OH | A02 | a1 | B01 | b1 | C03 | c2 | D02 | F |
| 2-2-c2D02-1-0038 | 590.127 | OH | A02 | a1 | B01 | b1 | C04 | c2 | D02 | F |
| 2-2-c2D02-1-0039 | 592.143 | OH | A01 | a1 | B01 | b1 | C15 | c2 | D02 | F |
| 2-2-c2D02-1-0040 | 593.127 | OH | A02 | a1 | B02 | b1 | C01 | c2 | D02 | F |
| 2-2-c2D02-1-0041 | 593.138 | OH | A01 | a1 | B01 | b1 | C16 | c2 | D02 | F |
| 2-2-c2D02-1-0042 | 594.133 | OH | A01 | a1 | B01 | b1 | C17 | c2 | D02 | F |
| 2-2-c2D02-1-0043 | 595.088 | OH | A02 | a1 | B01 | b1 | C05 | c2 | D02 | F |
| 2-2-c2D02-1-0044 | 595.117 | OH | A01 | a1 | B02 | b3 | C09 | c2 | D02 | F |
| 2-2-c2D02-1-0045 | 595.142 | OH | A01 | a1 | B01 | b4 | C01 | c2 | D02 | F |
| 2-2-c2D02-1-0046 | 596.101 | OH | A02 | a1 | B01 | b3 | C08 | c2 | D02 | F |
| 2-2-c2D02-1-0047 | 597.102 | OH | A02 | a1 | B03 | b1 | C01 | c2 | D02 | F |
| 2-2-c2D02-1-0048 | 597.133 | OH | A01 | a1 | B02 | b1 | C14 | c2 | D02 | F |
| 2-2-c2D02-1-0049 | 597.194 | OH | A02 | a1 | B02 | b4 | C12 | c2 | D02 | F |
| 2-2-c2D02-1-0050 | 599.092 | OH | A01 | a1 | B03 | b3 | C09 | c2 | D02 | F |
| 2-2-c2D02-1-0051 | 599.094 | OH | A01 | a1 | B01 | b1 | C18 | c2 | D02 | F |
| 2-2-c2D02-1-0052 | 599.174 | OH | A02 | a1 | B02 | b4 | C13 | c2 | D02 | F |
| 2-2-c2D02-1-0053 | 600.078 | OH | A01 | a1 | B02 | b3 | C10 | c2 | D02 | F |
| 2-2-c2D02-1-0054 | 601.108 | OH | A01 | a1 | B03 | b1 | C14 | c2 | D02 | F |
| 2-2-c2D02-1-0055 | 601.132 | OH | A01 | a1 | B02 | b2 | C02 | c2 | D02 | F |
| 2-2-c2D02-1-0056 | 601.132 | OH | A01 | a1 | B02 | b2 | C03 | c2 | D02 | F |
| 2-2-c2D02-1-0057 | 601.169 | OH | A02 | a1 | B03 | b4 | C12 | c2 | D02 | F |

(continued)

| [Table 9-4-4] Compound that may be contained in mixture 2-2-c2D02-1 | | | | | | | | | | |
|---|---|---|---|---|---|---|---|---|---|---|
| ID | Exact Mass | | A | a | B | b | C | c | D | d |
| 2-2-c2D02-1-0058 | 602.127 | OH | A01 | a1 | B02 | b2 | C04 | c2 | D02 | F |
| 2-2-c2D02-1-0059 | 602.127 | OH | A01 | a1 | B02 | b2 | C06 | c2 | D02 | F |
| 2-2-c2D02-1-0060 | 602.127 | OH | A01 | a1 | B02 | b2 | C07 | c2 | D02 | F |
| 2-2-c2D02-1-0061 | 603.148 | OH | A02 | a1 | B02 | b1 | C02 | c2 | D02 | F |
| 2-2-c2D02-1-0062 | 603.148 | OH | A02 | a1 | B02 | b1 | C03 | c2 | D02 | F |
| 2-2-c2D02-1-0063 | 603.149 | OH | A02 | a1 | B03 | b4 | C13 | c2 | D02 | F |
| 2-2-c2D02-1-0064 | 604.053 | OH | A01 | a1 | B03 | b3 | C10 | c2 | D02 | F |
| 2-2-c2D02-1-0065 | 604.143 | OH | A02 | a1 | B02 | b1 | C04 | c2 | D02 | F |
| 2-2-c2D02-1-0066 | 605.107 | OH | A01 | a1 | B03 | b2 | C02 | c2 | D02 | F |
| 2-2-c2D02-1-0067 | 605.107 | OH | A01 | a1 | B03 | b2 | C03 | c2 | D02 | F |
| 2-2-c2D02-1-0068 | 605.163 | OH | A01 | a1 | B01 | b4 | C02 | c2 | D02 | F |
| 2-2-c2D02-1-0069 | 606.102 | OH | A01 | a1 | B03 | b2 | C04 | c2 | D02 | F |
| 2-2-c2D02-1-0070 | 606.102 | OH | A01 | a1 | B03 | b2 | C06 | c2 | D02 | F |
| 2-2-c2D02-1-0071 | 606.102 | OH | A01 | a1 | B03 | b2 | C07 | c2 | D02 | F |
| 2-2-c2D02-1-0072 | 606.158 | OH | A01 | a1 | B02 | b1 | C15 | c2 | D02 | F |
| 2-2-c2D02-1-0073 | 607.117 | OH | A02 | a1 | B01 | b3 | C09 | c2 | D02 | F |
| 2-2-c2D02-1-0074 | 607.122 | OH | A02 | a1 | B03 | b1 | C02 | c2 | D02 | F |
| 2-2-c2D02-1-0075 | 607.122 | OH | A02 | a1 | B03 | b1 | C03 | c2 | D02 | F |
| 2-2-c2D02-1-0076 | 607.154 | OH | A01 | a1 | B02 | b1 | C16 | c2 | D02 | F |
| 2-2-c2D02-1-0077 | 608.118 | OH | A02 | a1 | B03 | b1 | C04 | c2 | D02 | F |
| 2-2-c2D02-1-0078 | 608.149 | OH | A01 | a1 | B02 | b1 | C17 | c2 | D02 | F |
| 2-2-c2D02-1-0079 | 609.104 | OH | A02 | a1 | B02 | b1 | C05 | c2 | D02 | F |
| 2-2-c2D02-1-0080 | 609.133 | OH | A02 | a1 | B01 | b1 | C14 | c2 | D02 | F |
| 2-2-c2D02-1-0081 | 609.158 | OH | A01 | a1 | B02 | b4 | C01 | c2 | D02 | F |
| 2-2-c2D02-1-0082 | 610.063 | OH | A01 | a1 | B04 | b1 | C01 | c2 | D02 | F |
| 2-2-c2D02-1-0083 | 610.117 | OH | A02 | a1 | B02 | b3 | C08 | c2 | D02 | F |
| 2-2-c2D02-1-0084 | 610.133 | OH | A01 | a1 | B03 | b1 | C15 | c2 | D02 | F |
| 2-2-c2D02-1-0085 | 611.120 | OH | A01 | a1 | B01 | b4 | C05 | c2 | D02 | F |
| 2-2-c2D02-1-0086 | 611.129 | OH | A01 | a1 | B03 | b1 | C16 | c2 | D02 | F |
| 2-2-c2D02-1-0087 | 612.078 | OH | A02 | a1 | B01 | b3 | C10 | c2 | D02 | F |
| 2-2-c2D02-1-0088 | 612.124 | OH | A01 | a1 | B03 | b1 | C17 | c2 | D02 | F |
| 2-2-c2D02-1-0089 | 613.079 | OH | A02 | a1 | B03 | b1 | C05 | c2 | D02 | F |
| 2-2-c2D02-1-0090 | 613.110 | OH | A01 | a1 | B02 | b1 | C18 | c2 | D02 | F |
| 2-2-c2D02-1-0091 | 613.132 | OH | A02 | a1 | B01 | b2 | C02 | c2 | D02 | F |
| 2-2-c2D02-1-0092 | 613.132 | OH | A02 | a1 | B01 | b2 | C03 | c2 | D02 | F |
| 2-2-c2D02-1-0093 | 613.133 | OH | A01 | a1 | B03 | b4 | C01 | c2 | D02 | F |
| 2-2-c2D02-1-0094 | 614.092 | OH | A02 | a1 | B03 | b3 | C08 | c2 | D02 | F |
| 2-2-c2D02-1-0095 | 614.127 | OH | A02 | a1 | B01 | b2 | C04 | c2 | D02 | F |

(continued)

| [Table 9-4-4] Compound that may be contained in mixture 2-2-c2D02-1 | | | | | | | | | | |
|---|---|---|---|---|---|---|---|---|---|---|
| ID | Exact Mass | | A | a | B | b | C | c | D | d |
| 2-2-c2D02-1-0096 | 614.127 | OH | A02 | a1 | B01 | b2 | C06 | c2 | D02 | F |
| 2-2-c2D02-1-0097 | 614.127 | OH | A02 | a1 | B01 | b2 | C07 | c2 | D02 | F |
| 2-2-c2D02-1-0098 | 614.131 | OH | A01 | a1 | B04 | b4 | C12 | c2 | D02 | F |
| 2-2-c2D02-1-0099 | 616.110 | OH | A01 | a1 | B04 | b4 | C13 | c2 | D02 | F |
| 2-2-c2D02-1-0100 | 617.085 | OH | A01 | a1 | B03 | b1 | C18 | c2 | D02 | F |
| 2-2-c2D02-1-0101 | 618.158 | OH | A02 | a1 | B01 | b1 | C15 | c2 | D02 | F |
| 2-2-c2D02-1-0102 | 619.106 | OH | A01 | a1 | B01 | b3 | C11 | c2 | D02 | F |
| 2-2-c2D02-1-0103 | 619.154 | OH | A02 | a1 | B01 | b1 | C16 | c2 | D02 | F |
| 2-2-c2D02-1-0104 | 619.179 | OH | A01 | a1 | B02 | b4 | C02 | c2 | D02 | F |
| 2-2-c2D02-1-0105 | 620.084 | OH | A01 | a1 | B04 | b1 | C02 | c2 | D02 | F |
| 2-2-c2D02-1-0106 | 620.084 | OH | A01 | a1 | B04 | b1 | C03 | c2 | D02 | F |
| 2-2-c2D02-1-0107 | 620.149 | OH | A02 | a1 | B01 | b1 | C17 | c2 | D02 | F |
| 2-2-c2D02-1-0108 | 621.079 | OH | A01 | a1 | B04 | b1 | C04 | c2 | D02 | F |
| 2-2-c2D02-1-0109 | 621.083 | OH | A01 | a1 | B05 | b1 | C01 | c2 | D02 | F |
| 2-2-c2D02-1-0110 | 621.133 | OH | A02 | a1 | B02 | b3 | C09 | c2 | D02 | F |
| 2-2-c2D02-1-0111 | 621.158 | OH | A02 | a1 | B01 | b4 | C01 | c2 | D02 | F |
| 2-2-c2D02-1-0112 | 623.149 | OH | A02 | a1 | B02 | b1 | C14 | c2 | D02 | F |
| 2-2-c2D02-1-0113 | 623.154 | OH | A01 | a1 | B03 | b4 | C02 | c2 | D02 | F |
| 2-2-c2D02-1-0114 | 625.108 | OH | A02 | a1 | B03 | b3 | C09 | c2 | D02 | F |
| 2-2-c2D02-1-0115 | 625.110 | OH | A02 | a1 | B01 | b1 | C18 | c2 | D02 | F |
| 2-2-c2D02-1-0116 | 625.135 | OH | A01 | a1 | B02 | b4 | C05 | c2 | D02 | F |
| 2-2-c2D02-1-0117 | 625.151 | OH | A01 | a1 | B05 | b4 | C12 | c2 | D02 | F |
| 2-2-c2D02-1-0118 | 626.040 | OH | A01 | a1 | B04 | b1 | C05 | c2 | D02 | F |
| 2-2-c2D02-1-0119 | 626.094 | OH | A02 | a1 | B02 | b3 | C10 | c2 | D02 | F |
| 2-2-c2D02-1-0120 | 627.053 | OH | A01 | a1 | B04 | b3 | C08 | c2 | D02 | F |
| 2-2-c2D02-1-0121 | 627.124 | OH | A02 | a1 | B03 | b1 | C14 | c2 | D02 | F |
| 2-2-c2D02-1-0122 | 627.130 | OH | A01 | a1 | B05 | b4 | C13 | c2 | D02 | F |
| 2-2-c2D02-1-0123 | 627.148 | OH | A02 | a1 | B02 | b2 | C02 | c2 | D02 | F |
| 2-2-c2D02-1-0124 | 627.148 | OH | A02 | a1 | B02 | b2 | C03 | c2 | D02 | F |
| 2-2-c2D02-1-0125 | 628.143 | OH | A02 | a1 | B02 | b2 | C04 | c2 | D02 | F |
| 2-2-c2D02-1-0126 | 628.143 | OH | A02 | a1 | B02 | b2 | C06 | c2 | D02 | F |
| 2-2-c2D02-1-0127 | 628.143 | OH | A02 | a1 | B02 | b2 | C07 | c2 | D02 | F |
| 2-2-c2D02-1-0128 | 629.110 | OH | A01 | a1 | B03 | b4 | C05 | c2 | D02 | F |
| 2-2-c2D02-1-0129 | 630.069 | OH | A02 | a1 | B03 | b3 | C10 | c2 | D02 | F |
| 2-2-c2D02-1-0130 | 631.104 | OH | A01 | a1 | B05 | b1 | C02 | c2 | D02 | F |
| 2-2-c2D02-1-0131 | 631.104 | OH | A01 | a1 | B05 | b1 | C03 | c2 | D02 | F |
| 2-2-c2D02-1-0132 | 631.122 | OH | A02 | a1 | B03 | b2 | C02 | c2 | D02 | F |

(continued)

| [Table 9-4-4] Compound that may be contained in mixture 2-2-c2D02-1 | | | | | | | | | | |
|---|---|---|---|---|---|---|---|---|---|---|
| ID | Exact Mass | | A | a | B | b | C | c | D | d |
| 2-2-c2D02-1-0133 | 631.122 | OH | A02 | a1 | B03 | b2 | C03 | c2 | D02 | F |
| 2-2-c2D02-1-0134 | 631.179 | OH | A02 | a1 | B01 | b4 | C02 | c2 | D02 | F |
| 2-2-c2D02-1-0135 | 632.099 | OH | A01 | a1 | B05 | b1 | C04 | c2 | D02 | F |
| 2-2-c2D02-1-0136 | 632.118 | OH | A02 | a1 | B03 | b2 | C04 | c2 | D02 | F |
| 2-2-c2D02-1-0137 | 632.118 | OH | A02 | a1 | B03 | b2 | C06 | c2 | D02 | F |
| 2-2-c2D02-1-0138 | 632.118 | OH | A02 | a1 | B03 | b2 | C07 | c2 | D02 | F |
| 2-2-c2D02-1-0139 | 632.174 | OH | A02 | a1 | B02 | b1 | C15 | c2 | D02 | F |
| 2-2-c2D02-1-0140 | 633.122 | OH | A01 | a1 | B02 | b3 | C11 | c2 | D02 | F |
| 2-2-c2D02-1-0141 | 633.169 | OH | A02 | a1 | B02 | b1 | C16 | c2 | D02 | F |
| 2-2-c2D02-1-0142 | 634.165 | OH | A02 | a1 | B02 | b1 | C17 | c2 | D02 | F |
| 2-2-c2D02-1-0143 | 635.174 | OH | A02 | a1 | B02 | b4 | C01 | c2 | D02 | F |
| 2-2-c2D02-1-0144 | 636.078 | OH | A02 | a1 | B04 | b1 | C01 | c2 | D02 | F |
| 2-2-c2D02-1-0145 | 636.149 | OH | A02 | a1 | B03 | b1 | C15 | c2 | D02 | F |
| 2-2-c2D02-1-0146 | 637.060 | OH | A01 | a1 | B05 | b1 | C05 | c2 | D02 | F |
| 2-2-c2D02-1-0147 | 637.097 | OH | A01 | a1 | B03 | b3 | C11 | c2 | D02 | F |
| 2-2-c2D02-1-0148 | 637.135 | OH | A02 | a1 | B01 | b4 | C05 | c2 | D02 | F |
| 2-2-c2D02-1-0149 | 637.144 | OH | A02 | a1 | B03 | b1 | C16 | c2 | D02 | F |
| 2-2-c2D02-1-0150 | 638.069 | OH | A01 | a1 | B04 | b3 | C09 | c2 | D02 | F |
| 2-2-c2D02-1-0151 | 638.073 | OH | A01 | a1 | B05 | b3 | C08 | c2 | D02 | F |
| 2-2-c2D02-1-0152 | 638.140 | OH | A02 | a1 | B03 | b1 | C17 | c2 | D02 | F |
| 2-2-c2D02-1-0153 | 639.126 | OH | A02 | a1 | B02 | b1 | C18 | c2 | D02 | F |
| 2-2-c2D02-1-0154 | 639.149 | OH | A02 | a1 | B03 | b4 | C01 | c2 | D02 | F |
| 2-2-c2D02-1-0155 | 640.085 | OH | A01 | a1 | B04 | b1 | C14 | c2 | D02 | F |
| 2-2-c2D02-1-0156 | 640.146 | OH | A02 | a1 | B04 | b4 | C12 | c2 | D02 | F |
| 2-2-c2D02-1-0157 | 642.125 | OH | A02 | a1 | B04 | b4 | C13 | c2 | D02 | F |
| 2-2-c2D02-1-0158 | 643.030 | OH | A01 | a1 | B04 | b3 | C10 | c2 | D02 | F |
| 2-2-c2D02-1-0159 | 643.101 | OH | A02 | a1 | B03 | b1 | C18 | c2 | D02 | F |
| 2-2-c2D02-1-0160 | 644.084 | OH | A01 | a1 | B04 | b2 | C02 | c2 | D02 | F |
| 2-2-c2D02-1-0161 | 644.084 | OH | A01 | a1 | B04 | b2 | C03 | c2 | D02 | F |
| 2-2-c2D02-1-0162 | 645.079 | OH | A01 | a1 | B04 | b2 | C04 | c2 | D02 | F |
| 2-2-c2D02-1-0163 | 645.079 | OH | A01 | a1 | B04 | b2 | C06 | c2 | D02 | F |
| 2-2-c2D02-1-0164 | 645.079 | OH | A01 | a1 | B04 | b2 | C07 | c2 | D02 | F |
| 2-2-c2D02-1-0165 | 645.122 | OH | A02 | a1 | B01 | b3 | C11 | c2 | D02 | F |
| 2-2-c2D02-1-0166 | 645.194 | OH | A02 | a1 | B02 | b4 | C02 | c2 | D02 | F |
| 2-2-c2D02-1-0167 | 646.099 | OH | A02 | a1 | B04 | b1 | C02 | c2 | D02 | F |
| 2-2-c2D02-1-0168 | 646.099 | OH | A02 | a1 | B04 | b1 | C03 | c2 | D02 | F |
| 2-2-c2D02-1-0169 | 647.094 | OH | A02 | a1 | B04 | b1 | C04 | c2 | D02 | F |

(continued)

| [Table 9-4-4] Compound that may be contained in mixture 2-2-c2D02-1 | | | | | | | | | | |
|---|---|---|---|---|---|---|---|---|---|---|
| ID | Exact Mass | | A | a | B | b | C | c | D | d |
| 2-2-c2D02-1-0170 | 647.099 | OH | A02 | a1 | B05 | b1 | C01 | c2 | D02 | F |
| 2-2-c2D02-1-0171 | 647.185 | OH | A01 | a1 | B01 | b1 | C19 | c2 | D02 | F |
| 2-2-c2D02-1-0172 | 649.089 | OH | A01 | a1 | B05 | b3 | C09 | c2 | D02 | F |
| 2-2-c2D02-1-0173 | 649.110 | OH | A01 | a1 | B04 | b1 | C15 | c2 | D02 | F |
| 2-2-c2D02-1-0174 | 649.169 | OH | A02 | a1 | B03 | b4 | C02 | c2 | D02 | F |
| 2-2-c2D02-1-0175 | 650.105 | OH | A01 | a1 | B04 | b1 | C16 | c2 | D02 | F |
| 2-2-c2D02-1-0176 | 651.101 | OH | A01 | a1 | B04 | b1 | C17 | c2 | D02 | F |
| 2-2-c2D02-1-0177 | 651.105 | OH | A01 | a1 | B05 | b1 | C14 | c2 | D02 | F |
| 2-2-c2D02-1-0178 | 651.151 | OH | A02 | a1 | B02 | b4 | C05 | c2 | D02 | F |
| 2-2-c2D02-1-0179 | 651.166 | OH | A02 | a1 | B05 | b4 | C12 | c2 | D02 | F |
| 2-2-c2D02-1-0180 | 652.056 | OH | A02 | a1 | B04 | b1 | C05 | c2 | D02 | F |
| 2-2-c2D02-1-0181 | 652.110 | OH | A01 | a1 | B04 | b4 | C01 | c2 | D02 | F |
| 2-2-c2D02-1-0182 | 653.069 | OH | A02 | a1 | B04 | b3 | C08 | c2 | D02 | F |
| 2-2-c2D02-1-0183 | 653.145 | OH | A02 | a1 | B05 | b4 | C13 | c2 | D02 | F |
| 2-2-c2D02-1-0184 | 654.050 | OH | A01 | a1 | B05 | b3 | C10 | c2 | D02 | F |
| 2-2-c2D02-1-0185 | 655.104 | OH | A01 | a1 | B05 | b2 | C02 | c2 | D02 | F |
| 2-2-c2D02-1-0186 | 655.104 | OH | A01 | a1 | B05 | b2 | C03 | c2 | D02 | F |
| 2-2-c2D02-1-0187 | 655.126 | OH | A02 | a1 | B03 | b4 | C05 | c2 | D02 | F |
| 2-2-c2D02-1-0188 | 656.062 | OH | A01 | a1 | B04 | b1 | C18 | c2 | D02 | F |
| 2-2-c2D02-1-0189 | 656.099 | OH | A01 | a1 | B05 | b2 | C04 | c2 | D02 | F |
| 2-2-c2D02-1-0190 | 656.099 | OH | A01 | a1 | B05 | b2 | C06 | c2 | D02 | F |
| 2-2-c2D02-1-0191 | 656.099 | OH | A01 | a1 | B05 | b2 | C07 | c2 | D02 | F |
| 2-2-c2D02-1-0192 | 657.119 | OH | A02 | a1 | B05 | b1 | C02 | c2 | D02 | F |
| 2-2-c2D02-1-0193 | 657.119 | OH | A02 | a1 | B05 | b1 | C03 | c2 | D02 | F |
| 2-2-c2D02-1-0194 | 658.115 | OH | A02 | a1 | B05 | b1 | C04 | c2 | D02 | F |
| 2-2-c2D02-1-0195 | 659.137 | OH | A02 | a1 | B02 | b3 | C11 | c2 | D02 | F |
| 2-2-c2D02-1-0196 | 660.130 | OH | A01 | a1 | B05 | b1 | C15 | c2 | D02 | F |
| 2-2-c2D02-1-0197 | 661.125 | OH | A01 | a1 | B05 | b1 | C16 | c2 | D02 | F |
| 2-2-c2D02-1-0198 | 661.201 | OH | A01 | a1 | B02 | b1 | C19 | c2 | D02 | F |
| 2-2-c2D02-1-0199 | 662.121 | OH | A01 | a1 | B05 | b1 | C17 | c2 | D02 | F |
| 2-2-c2D02-1-0200 | 662.131 | OH | A01 | a1 | B04 | b4 | C02 | c2 | D02 | F |
| 2-2-c2D02-1-0201 | 663.076 | OH | A02 | a1 | B05 | b1 | C05 | c2 | D02 | F |
| 2-2-c2D02-1-0202 | 663.112 | OH | A02 | a1 | B03 | b3 | C11 | c2 | D02 | F |
| 2-2-c2D02-1-0203 | 663.130 | OH | A01 | a1 | B05 | b4 | C01 | c2 | D02 | F |
| 2-2-c2D02-1-0204 | 664.085 | OH | A02 | a1 | B04 | b3 | C09 | c2 | D02 | F |
| 2-2-c2D02-1-0205 | 664.089 | OH | A02 | a1 | B05 | b3 | C08 | c2 | D02 | F |
| 2-2-c2D02-1-0206 | 665.176 | OH | A01 | a1 | B03 | b1 | C19 | c2 | D02 | F |

(continued)

| [Table 9-4-4] Compound that may be contained in mixture 2-2-c2D02-1 | | | | | | | | | | |
|---|---|---|---|---|---|---|---|---|---|---|
| ID | Exact Mass | | A | a | B | b | C | c | D | d |
| 2-2-c2D02-1-0207 | 666.100 | OH | A02 | a1 | B04 | b1 | C14 | c2 | D02 | F |
| 2-2-c2D02-1-0208 | 667.082 | OH | A01 | a1 | B05 | b1 | C18 | c2 | D02 | F |
| 2-2-c2D02-1-0209 | 668.087 | OH | A01 | a1 | B04 | b4 | C05 | c2 | D02 | F |
| 2-2-c2D02-1-0210 | 669.046 | OH | A02 | a1 | B04 | b3 | C10 | c2 | D02 | F |
| 2-2-c2D02-1-0211 | 670.099 | OH | A02 | a1 | B04 | b2 | C02 | c2 | D02 | F |
| 2-2-c2D02-1-0212 | 670.099 | OH | A02 | a1 | B04 | b2 | C03 | c2 | D02 | F |
| 2-2-c2D02-1-0213 | 671.094 | OH | A02 | a1 | B04 | b2 | C04 | c2 | D02 | F |
| 2-2-c2D02-1-0214 | 671.094 | OH | A02 | a1 | B04 | b2 | C06 | c2 | D02 | F |
| 2-2-c2D02-1-0215 | 671.094 | OH | A02 | a1 | B04 | b2 | C07 | c2 | D02 | F |
| 2-2-c2D02-1-0216 | 673.151 | OH | A01 | a1 | B05 | b4 | C02 | c2 | D02 | F |
| 2-2-c2D02-1-0217 | 673.201 | OH | A02 | a1 | B01 | b1 | C19 | c2 | D02 | F |
| 2-2-c2D02-1-0218 | 675.105 | OH | A02 | a1 | B05 | b3 | C09 | c2 | D02 | F |
| 2-2-c2D02-1-0219 | 675.126 | OH | A02 | a1 | B04 | b1 | C15 | c2 | D02 | F |
| 2-2-c2D02-1-0220 | 676.073 | OH | A01 | a1 | B04 | b3 | C11 | c2 | D02 | F |
| 2-2-c2D02-1-0221 | 676.121 | OH | A02 | a1 | B04 | b1 | C16 | c2 | D02 | F |
| 2-2-c2D02-1-0222 | 677.116 | OH | A02 | a1 | B04 | b1 | C17 | c2 | D02 | F |
| 2-2-c2D02-1-0223 | 677.120 | OH | A02 | a1 | B05 | b1 | C14 | c2 | D02 | F |
| 2-2-c2D02-1-0224 | 678.125 | OH | A02 | a1 | B04 | b4 | C01 | c2 | D02 | F |
| 2-2-c2D02-1-0225 | 679.107 | OH | A01 | a1 | B05 | b4 | C05 | c2 | D02 | F |
| 2-2-c2D02-1-0226 | 680.066 | OH | A02 | a1 | B05 | b3 | C10 | c2 | D02 | F |
| 2-2-c2D02-1-0227 | 681.119 | OH | A02 | a1 | B05 | b2 | C02 | c2 | D02 | F |
| 2-2-c2D02-1-0228 | 681.119 | OH | A02 | a1 | B05 | b2 | C03 | c2 | D02 | F |
| 2-2-c2D02-1-0229 | 682.077 | OH | A02 | a1 | B04 | b1 | C18 | c2 | D02 | F |
| 2-2-c2D02-1-0230 | 682.115 | OH | A02 | a1 | B05 | b2 | C04 | c2 | D02 | F |
| 2-2-c2D02-1-0231 | 682.115 | OH | A02 | a1 | B05 | b2 | C06 | c2 | D02 | F |
| 2-2-c2D02-1-0232 | 682.115 | OH | A02 | a1 | B05 | b2 | C07 | c2 | D02 | F |
| 2-2-c2D02-1-0233 | 686.146 | OH | A02 | a1 | B05 | b1 | C15 | c2 | D02 | F |
| 2-2-c2D02-1-0234 | 687.093 | OH | A01 | a1 | B05 | b3 | C11 | c2 | D02 | F |
| 2-2-c2D02-1-0235 | 687.141 | OH | A02 | a1 | B05 | b1 | C16 | c2 | D02 | F |
| 2-2-c2D02-1-0236 | 687.216 | OH | A02 | a1 | B02 | b1 | C19 | c2 | D02 | F |
| 2-2-c2D02-1-0237 | 688.136 | OH | A02 | a1 | B05 | b1 | C17 | c2 | D02 | F |
| 2-2-c2D02-1-0238 | 688.146 | OH | A02 | a1 | B04 | b4 | C02 | c2 | D02 | F |
| 2-2-c2D02-1-0239 | 689.145 | OH | A02 | a1 | B05 | b4 | C01 | c2 | D02 | F |
| 2-2-c2D02-1-0240 | 691.191 | OH | A02 | a1 | B03 | b1 | C19 | c2 | D02 | F |
| 2-2-c2D02-1-0241 | 693.098 | OH | A02 | a1 | B05 | b1 | C18 | c2 | D02 | F |
| 2-2-c2D02-1-0242 | 694.103 | OH | A02 | a1 | B04 | b4 | C05 | c2 | D02 | F |
| 2-2-c2D02-1-0243 | 699.166 | OH | A02 | a1 | B05 | b4 | C02 | c2 | D02 | F |

(continued)

| [Table 9-4-4] Compound that may be contained in mixture 2-2-c2D02-1 | | | | | | | | | | |
|---|---|---|---|---|---|---|---|---|---|---|
| ID | Exact Mass | | A | a | B | b | C | c | D | d |
| 2-2-c2D02-1-0244 | 702.089 | OH | A02 | a1 | B04 | b3 | C11 | c2 | D02 | F |
| 2-2-c2D02-1-0245 | 704.152 | OH | A01 | a1 | B04 | b1 | C19 | c2 | D02 | F |
| 2-2-c2D02-1-0246 | 705.123 | OH | A02 | a1 | B05 | b4 | C05 | c2 | D02 | F |
| 2-2-c2D02-1-0247 | 713.109 | OH | A02 | a1 | B05 | b3 | C11 | c2 | D02 | F |
| 2-2-c2D02-1-0248 | 715.172 | OH | A01 | a1 | B05 | b1 | C19 | c2 | D02 | F |
| 2-2-c2D02-1-0249 | 730.168 | OH | A02 | a1 | B04 | b1 | C19 | c2 | D02 | F |
| 2-2-c2D02-1-0250 | 741.188 | OH | A02 | a1 | B05 | b1 | C19 | c2 | D02 | F |

Example 9-5: Formation of linker dl by step dl

Example 9-5-1: Synthesis of 2-2-d1E01-0 to 2-2-d1E04-0 by steps d1E01 to dlE04

[Formula 687]

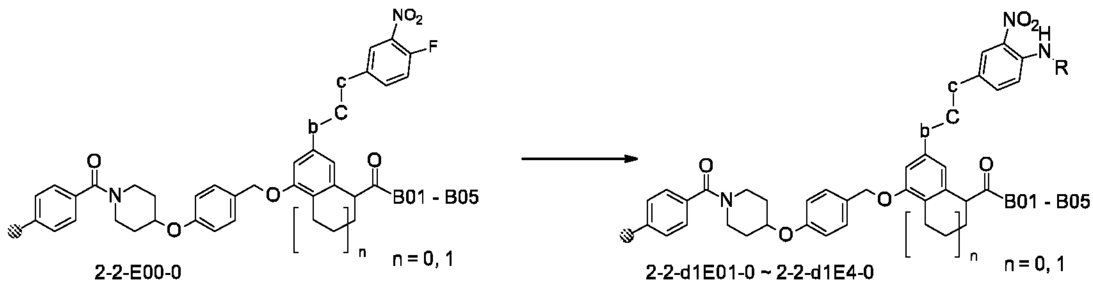

2-2-c1D01-0 (100 mg, 0.187 mmol/g) and 2-2-c2D02-0 (100 mg, 0.189 mmol/g) were mixed (this mixture is referred to as 2-2-E00-0) in four 4 mL glass vials. NMP (2.0 mL) was added thereto, and mixtures were shaken at room temperature for 1 hour. Separate amines (0.375 mmol) described in Table 9-5-1 were added to the glass vials, respectively. Proton sponge (121 mg, 0.563 mmol) was added thereto, and each mixture was shaken at 80°C for 18 hours.

Solid-phase purification

The suspension of the reaction solution and the solid phase was transferred to a column with a filter, filtered, and washed three times with NMP/water = 1/1 (4 mL), three times with NMP (4 mL), three times with 0.05 M $TBAHSO_4$ + DTBP in NMP (4 mL), three times with 0.05 M NMM in NMP (4 mL), three times with NMP/water = 1/1 (4 mL), three times with NMP (4 mL), three times with MeOH (4.5 mL), three times with DCM (4.5 mL), and three times with heptane (4.5 mL), and the obtained solid phase was dried under reduced pressure to obtain the mixtures described in Table 9-5-1.

[Table 490]

[Table 9-5-1]

| Compound No. of amine | Amine used | Molar number used | Amount used | No. of obtained mixture |
|---|---|---|---|---|
| BB2-2-33 | Cyclohexanamine | 0.375 mmol | 37.2 mg | 2-2-d1E01-0 |
| BB2-2-34 | Oxan-4-ylmethanamine | 0.375 mmol | 43.2 mg | 2-2-d1E02-0 |
| BB2-2-35 | 2-Phenylsulfanylethanamine | 0.375 mmol | 57.5 mg | 2-2-d1E03-0 |
| BB2-2-36 | (2R)-4-Morpholin-4-yl-1-phenylsulfanylbutan-2-amine | 0.375 mmol | 100 mg | 2-2-d1E04-0 |

The obtained mixtures in Table 9-5-1 can be treated by cleavage in the same manner as in step a1 to respectively obtain mixtures in a state cleaved from the solid phase. The details of the mixtures are shown in Table 9-5-2 to Table 9-5-5.

A notation method in each table will be described. For example, in Table 9-5-2, each compound that may be contained in 2-2-d1E01-1 is indicated by ID, and core blocks and linkers in combination in the structure of mixture 2-2-d1E01-1 are indicated by symbols or chemical formulas. The correspondence between the symbols and specific structures are shown in Figure 6.

In Table 9-5-2 to Table 9-5-5, "×" is represented by chemical formulas.

[Formula 688]

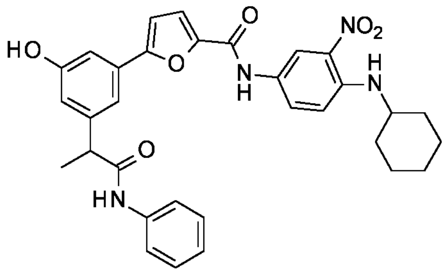

2-2-d1E01-1-0001

[Table 491]

| Exemplary notation in [Table 9-5-2] | | | | | | | | | | |
|---|---|---|---|---|---|---|---|---|---|---|
| ID | | A | a | B | b | C | c | D | d | E |
| 2-2-d1E01-1-0001 | | OH A01 | a1 | B01 | b1 | C01 | | c1 D01 d1 | | E01 |

[Table 492]

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0001 | 568.232 | OH | A01 | a1 | B01 | b1 | C01 | c1 | D01 | d1 | E01 |
| 2-2-d1E01-1-0002 | 572.300 | OH | A01 | a1 | B01 | b4 | C12 | c1 | D01 | d1 | E01 |
| 2-2-d1E01-1-0003 | 574.279 | OH | A01 | a1 | B01 | b4 | C13 | c1 | D01 | d1 | E01 |
| 2-2-d1E01-1-0004 | 578.253 | OH | A01 | a1 | B01 | b1 | C02 | c1 | D01 | d1 | E01 |
| 2-2-d1E01-1-0005 | 578.253 | OH | A01 | a1 | B01 | b1 | C03 | c1 | D01 | d1 | E01 |
| 2-2-d1E01-1-0006 | 579.248 | OH | A01 | a1 | B01 | b1 | C04 | c1 | D01 | d1 | E01 |
| 2-2-d1E01-1-0007 | 582.248 | OH | A01 | a1 | B02 | b1 | C01 | c1 | D01 | d1 | E01 |
| 2-2-d1E01-1-0008 | 584.209 | OH | A01 | a1 | B01 | b1 | C05 | c1 | D01 | d1 | E01 |
| 2-2-d1E01-1-0009 | 585.222 | OH | A01 | a1 | B01 | b3 | C08 | c1 | D01 | d1 | E01 |
| 2-2-d1E01-1-00 1 0 | 586.223 | OH | A01 | a1 | B03 | b1 | C01 | c1 | D01 | d1 | E01 |
| 2-2-d1E01-1-0011 | 586.316 | OH | A01 | a1 | B02 | b4 | C12 | c1 | D01 | d1 | E01 |
| 2-2-d1E01-1-0012 | 588.295 | OH | A01 | a1 | B02 | b4 | C13 | c1 | D01 | d1 | E01 |
| 2-2-d1E01-1-0013 | 590.290 | OH | A01 | a1 | B03 | b4 | C12 | c1 | D01 | d1 | E01 |
| 2-2-d1E01-1-0014 | 592.269 | OH | A01 | a1 | B02 | b1 | C02 | c1 | D01 | d1 | E01 |
| 2-2-d1E01-1-0015 | 592.269 | OH | A01 | a1 | B02 | b1 | C03 | c1 | D01 | d1 | E01 |
| 2-2-d1E01-1-0016 | 592.270 | OH | A01 | a1 | B03 | b4 | C13 | c1 | D01 | d1 | E01 |
| 2-2-d1E01-1-0017 | 593.264 | OH | A01 | a1 | B02 | b1 | C04 | c1 | D01 | d1 | E01 |
| 2-2-d1E01-1-0018 | 594.248 | OH | A02 | a1 | B01 | b1 | C01 | c1 | D01 | d1 | E01 |
| 2-2-d1E01-1-0019 | 596.238 | OH | A01 | a1 | B01 | b3 | C09 | c1 | D01 | d1 | E01 |
| 2-2-d1E01-1-0020 | 596.243 | OH | A01 | a1 | B03 | b1 | C02 | c1 | D01 | d1 | E01 |
| 2-2-d1E01-1-0021 | 596.243 | OH | A01 | a1 | B03 | b1 | C03 | c1 | D01 | d1 | E01 |
| 2-2-d1E01-1-0022 | 597.239 | OH | A01 | a1 | B03 | b1 | C04 | c1 | D01 | d1 | E01 |
| 2-2-d1E01-1-0023 | 598.225 | OH | A01 | a1 | B02 | b1 | C05 | c1 | D01 | d1 | E01 |
| 2-2-d1E01-1-0024 | 598.254 | OH | A01 | a1 | B01 | b1 | C14 | c1 | D01 | d1 | E01 |
| 2-2-d1E01-1-0025 | 598.316 | OH | A02 | a1 | B01 | b4 | C12 | c1 | D01 | d1 | E01 |
| 2-2-d1E01-1-0026 | 599.238 | OH | A01 | a1 | B02 | b3 | C08 | c1 | D01 | d1 | E01 |
| 2-2-d1E01-1-0027 | 600.295 | OH | A02 | a1 | B01 | b4 | C13 | c1 | D01 | d1 | E01 |
| 2-2-d1E01-1-0028 | 601.200 | OH | A01 | a1 | B01 | b3 | C10 | c1 | D01 | d1 | E01 |
| 2-2-d1E01-1-0029 | 602.200 | OH | A01 | a1 | B03 | b1 | C05 | c1 | D01 | d1 | E01 |
| 2-2-d1E01-1-0030 | 602.253 | OH | A01 | a1 | B01 | b2 | C02 | c1 | D01 | d1 | E01 |
| 2-2-d1E01-1-0031 | 602.253 | OH | A01 | a1 | B01 | b2 | C03 | c1 | D01 | d1 | E01 |
| 2-2-d1E01-1-0032 | 603.213 | OH | A01 | a1 | B03 | b3 | C08 | c1 | D01 | d1 | E01 |
| 2-2-d1E01-1-0033 | 603.248 | OH | A01 | a1 | B01 | b2 | C04 | c1 | D01 | d1 | E01 |
| 2-2-d1E01-1-0034 | 603.248 | OH | A01 | a1 | B01 | b2 | C06 | c1 | D01 | d1 | E01 |
| 2-2-d1E01-1-0035 | 603.248 | OH | A01 | a1 | B01 | b2 | C07 | c1 | D01 | d1 | E01 |
| 2-2-d1E01-1-0036 | 604.269 | OH | A02 | a1 | B01 | b1 | C02 | c1 | D01 | d1 | E01 |
| 2-2-d1E01-1-0037 | 604.269 | OH | A02 | a1 | B01 | b1 | C03 | c1 | D01 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0038 | 605.264 | OH | A02 | a1 | B01 | b1 | C04 | c1 | D01 | d1 | E01 |
| 2-2-d1E01-1-0039 | 607.279 | OH | A01 | a1 | B01 | b1 | C15 | c1 | D01 | d1 | E01 |
| 2-2-d1E01-1-0040 | 608.263 | OH | A02 | a1 | B02 | b1 | C01 | c1 | D01 | d1 | E01 |
| 2-2-d1E01-1-0041 | 608.275 | OH | A01 | a1 | B01 | b1 | C16 | c1 | D01 | d1 | E01 |
| 2-2-d1E01-1-0042 | 609.270 | OH | A01 | a1 | B01 | b1 | C17 | c1 | D01 | d1 | E01 |
| 2-2-d1E01-1-0043 | 610.225 | OH | A02 | a1 | B01 | b1 | C05 | c1 | D01 | d1 | E01 |
| 2-2-d1E01-1-0044 | 610.254 | OH | A01 | a1 | B02 | b3 | C09 | c1 | D01 | d1 | E01 |
| 2-2-d1E01-1-0045 | 610.279 | OH | A01 | a1 | B01 | b4 | C01 | c1 | D01 | d1 | E01 |
| 2-2-d1E01-1-0046 | 611.238 | OH | A02 | a1 | B01 | b3 | C08 | c1 | D01 | d1 | E01 |
| 2-2-d1E01-1-0047 | 612.238 | OH | A02 | a1 | B03 | b1 | C01 | c1 | D01 | d1 | E01 |
| 2-2-d1E01-1-0048 | 612.270 | OH | A01 | a1 | B02 | b1 | C14 | c1 | D01 | d1 | E01 |
| 2-2-d1E01-1-0049 | 612.331 | OH | A02 | a1 | B02 | b4 | C12 | c1 | D01 | d1 | E01 |
| 2-2-d1E01-1-0050 | 614.229 | OH | A01 | a1 | B03 | b3 | C09 | c1 | D01 | d1 | E01 |
| 2-2-d1E01-1-0051 | 614.231 | OH | A01 | a1 | B01 | b1 | C18 | c1 | D01 | d1 | E01 |
| 2-2-d1E01-1-0052 | 614.310 | OH | A02 | a1 | B02 | b4 | C13 | c1 | D01 | d1 | E01 |
| 2-2-d1E01-1-0053 | 615.215 | OH | A01 | a1 | B02 | b3 | C10 | c1 | D01 | d1 | E01 |
| 2-2-d1E01-1-0054 | 616.245 | OH | A01 | a1 | B03 | b1 | C14 | c1 | D01 | d1 | E01 |
| 2-2-d1E01-1-0055 | 616.269 | OH | A01 | a1 | B02 | b2 | C02 | c1 | D01 | d1 | E01 |
| 2-2-d1E01-1-0056 | 616.269 | OH | A01 | a1 | B02 | b2 | C03 | c1 | D01 | d1 | E01 |
| 2-2-d1E01-1-0057 | 616.306 | OH | A02 | a1 | B03 | b4 | C12 | c1 | D01 | d1 | E01 |
| 2-2-d1E01-1-0058 | 617.264 | OH | A01 | a1 | B02 | b2 | C04 | c1 | D01 | d1 | E01 |
| 2-2-d1E01-1-0059 | 617.264 | OH | A01 | a1 | B02 | b2 | C06 | c1 | D01 | d1 | E01 |
| 2-2-d1E01-1-0060 | 617.264 | OH | A01 | a1 | B02 | b2 | C07 | c1 | D01 | d1 | E01 |
| 2-2-d1E01-1-0061 | 618.284 | OH | A02 | a1 | B02 | b1 | C02 | c1 | D01 | d1 | E01 |
| 2-2-d1E01-1-0062 | 618.284 | OH | A02 | a1 | B02 | b1 | C03 | c1 | D01 | d1 | E01 |
| 2-2-d1E01-1-0063 | 618.285 | OH | A02 | a1 | B03 | b4 | C13 | c1 | D01 | d1 | E01 |
| 2-2-d1E01-1-0064 | 619.190 | OH | A01 | a1 | B03 | b3 | C10 | c1 | DOI | d1 | E01 |
| 2-2-d1E01-1-0065 | 619.279 | OH | A02 | a1 | B02 | b1 | C04 | c1 | D01 | d1 | E01 |
| 2-2-d1E01-1-0066 | 620.243 | OH | A01 | a1 | B03 | b2 | C02 | c1 | DOI | d1 | E01 |
| 2-2-d1E01-1-0067 | 620.243 | OH | A01 | a1 | B03 | b2 | C03 | c1 | DOI | d1 | E01 |
| 2-2-d1E01-1-0068 | 620.300 | OH | A01 | a1 | B01 | b4 | C02 | c1 | D01 | d1 | E01 |
| 2-2-d1E01-1-0069 | 621.239 | OH | A01 | a1 | B03 | b2 | C04 | c1 | DOI | d1 | E01 |
| 2-2-d1E01-1-0070 | 621.239 | OH | A01 | a1 | B03 | b2 | C06 | c1 | D01 | d1 | E01 |
| 2-2-d1E01-1-0071 | 621.239 | OH | A01 | a1 | B03 | b2 | C07 | c1 | DOI | d1 | E01 |
| 2-2-d1E01-1-0072 | 621.295 | OH | A01 | a1 | B02 | b1 | C15 | c1 | D01 | d1 | E01 |
| 2-2-d1E01-1-0073 | 622.254 | OH | A02 | a1 | B01 | b3 | C09 | c1 | D01 | d1 | E01 |
| 2-2-d1E01-1-0074 | 622.259 | OH | A02 | a1 | B03 | b1 | C02 | c1 | DOI | d1 | E01 |
| 2-2-d1E01-1-0075 | 622.259 | OH | A02 | a1 | B03 | b1 | C03 | c1 | D01 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0076 | 622.290 | OH | A01 | a1 | B02 | b1 | C16 | c1 | D01 | d1 | E01 |
| 2-2-d1E01-1-0077 | 623.254 | OH | A02 | a1 | B03 | b1 | C04 | c1 | D01 | d1 | E01 |
| 2-2-d1E01-1-0078 | 623.286 | OH | A01 | a1 | B02 | b1 | C17 | c1 | D01 | d1 | E01 |
| 2-2-d1E01-1-0079 | 624.241 | OH | A02 | a1 | B02 | b1 | COS | c1 | D01 | d1 | E01 |
| 2-2-d1E01-1-0080 | 624.270 | OH | A02 | a1 | B01 | b1 | C14 | c1 | D01 | d1 | E01 |
| 2-2-d1E01-1-0081 | 624.295 | OH | A01 | a1 | B02 | b4 | C01 | c1 | D01 | d1 | E01 |
| 2-2-d1E01-1-0082 | 625.200 | OH | AOI | a1 | B04 | b1 | C01 | c1 | D01 | d1 | E01 |
| 2-2-d1E01-1-0083 | 625.254 | OH | A02 | a1 | B02 | b3 | C08 | c1 | D01 | d1 | E01 |
| 2-2-d1E01-1-0084 | 625.270 | OH | AOI | a1 | B03 | b1 | C15 | c1 | D01 | d1 | E01 |
| 2-2-d1E01-1-0085 | 626.256 | OH | A01 | a1 | B01 | b4 | C05 | c1 | D01 | d1 | E01 |
| 2-2-d1E01-1-0086 | 626.265 | OH | A01 | a1 | B03 | b1 | C16 | c1 | D01 | d1 | E01 |
| 2-2-d1E01-1-0087 | 627.215 | OH | A02 | a1 | B01 | b3 | C10 | c1 | D01 | d1 | E01 |
| 2-2-d1E01-1-0088 | 627.261 | OH | A01 | a1 | B03 | b1 | C17 | c1 | D01 | d1 | E01 |
| 2-2-d1E01-1-0089 | 628.216 | OH | A02 | a1 | B03 | b1 | C05 | c1 | D01 | d1 | E01 |
| 2-2-d1E01-1-0090 | 628.247 | OH | A01 | a1 | B02 | b1 | C18 | c1 | D01 | d1 | E01 |
| 2-2-d1E01-1-0091 | 628.269 | OH | A02 | a1 | B01 | b2 | C02 | c1 | D01 | d1 | E01 |
| 2-2-d1E01-1-0092 | 628.269 | OH | A02 | a1 | B01 | b2 | C03 | c1 | D01 | d1 | E01 |
| 2-2-d1E01-1-0093 | 628.270 | OH | A01 | a1 | B03 | b4 | C01 | c1 | D01 | d1 | E01 |
| 2-2-d1E01-1-0094 | 629.229 | OH | A02 | a1 | B03 | b3 | C08 | c1 | D01 | d1 | E01 |
| 2-2-d1E01-1-0095 | 629.264 | OH | A02 | a1 | B01 | b2 | C04 | c1 | D01 | d1 | E01 |
| 2-2-d1E01-1-0096 | 629.264 | OH | A02 | a1 | B01 | b2 | C06 | c1 | D01 | d1 | E01 |
| 2-2-d1E01-1-0097 | 629.264 | OH | A02 | a1 | B01 | b2 | C07 | c1 | D01 | d1 | E01 |
| 2-2-dlE01-1-0098 | 629.267 | OH | A01 | a1 | B04 | b4 | C12 | c1 | D01 | d1 | E01 |
| 2-2-dlE01-1-0099 | 631.246 | OH | A01 | a1 | B04 | b4 | C13 | c1 | D01 | d1 | E01 |
| 2-2-d1E01-1-0100 | 632.194 | OH | A01 | a1 | B01 | b1 | C01 | c2 | D02 | d1 | E01 |
| 2-2-d1E01-1-0101 | 632.222 | OH | AOI | a1 | B03 | b1 | C18 | c1 | D01 | d1 | E01 |
| 2-2-d1E01-1-0102 | 633.295 | OH | A02 | a1 | B01 | b1 | C15 | c1 | D01 | d1 | E01 |
| 2-2-d1E01-1-0103 | 634.243 | OH | AOI | a1 | B01 | b3 | C 11 | c1 | D01 | d1 | E01 |
| 2-2-d1E01-1-0104 | 634.290 | OH | A02 | a1 | B01 | b1 | C16 | c1 | D01 | d1 | E01 |
| 2-2-d1E01-1-0105 | 634.316 | OH | A01 | a1 | B02 | b4 | C02 | c1 | D01 | d1 | E01 |
| 2-2-d1E01-1-0106 | 635.220 | OH | AOI | a1 | B04 | b1 | C02 | c1 | D01 | d1 | E01 |
| 2-2-d1E01-1-0107 | 635.220 | OH | A01 | a1 | B04 | b1 | C03 | c1 | D01 | d1 | E01 |
| 2-2-d1E01-1-0108 | 635.286 | OH | A02 | a1 | B01 | b1 | C17 | c1 | D01 | d1 | E01 |
| 2-2-d1E01-1-0109 | 636.215 | OH | A01 | a1 | B04 | b1 | C04 | c1 | D01 | d1 | E01 |
| 2-2-d1E01-1-0110 | 636.220 | OH | A01 | a1 | B05 | b1 | C01 | c1 | D01 | d1 | E01 |
| 2-2-d1E01-1-0111 | 636.262 | OH | A01 | a1 | B01 | b4 | C12 | c2 | D02 | d1 | E01 |
| 2-2-d1E01-1-0112 | 636.270 | OH | A02 | a1 | B02 | b3 | C09 | c1 | D01 | d1 | E01 |
| 2-2-d1E01-1-0113 | 636.295 | OH | A02 | a1 | B01 | b4 | C01 | c1 | DOI | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0114 | 638.241 | OH | A01 | a1 | B01 | b4 | C13 | c2 | D02 | d1 | E01 |
| 2-2-d1E01-1-0115 | 638.285 | OH | A02 | a1 | B02 | b1 | C14 | c1 | D01 | d1 | E01 |
| 2-2-d1E01-1-0116 | 638.290 | OH | A01 | a1 | B03 | b4 | C02 | c1 | DOI | d1 | E01 |
| 2-2-d1E01-1-0117 | 640.245 | OH | A02 | a1 | B03 | b3 | C09 | c1 | D01 | d1 | E01 |
| 2-2-d1E01-1-0118 | 640.247 | OH | A02 | a1 | B01 | b1 | C18 | c1 | DOI | d1 | E01 |
| 2-2-d1E01-1-0119 | 640.272 | OH | A01 | a1 | B02 | b4 | C05 | c1 | D01 | d1 | E01 |
| 2-2-d1E01-1-0120 | 640.287 | OH | A01 | a1 | B05 | b4 | C12 | c1 | D01 | d1 | E01 |
| 2-2-d1E01-1-0121 | 641.177 | OH | A01 | a1 | B04 | b1 | C05 | c1 | D01 | d1 | E01 |
| 2-2-d1E01-1-0122 | 641.231 | OH | A02 | a1 | B02 | b3 | C10 | c1 | D01 | d1 | E01 |
| 2-2-d1E01-1-0123 | 642.190 | OH | A01 | a1 | B04 | b3 | C08 | c1 | D01 | d1 | E01 |
| 2-2-d1E01-1-0124 | 642.215 | OH | A01 | a1 | B01 | b1 | C02 | c2 | D02 | d1 | E01 |
| 2-2-dlE01-1-0125 | 642.215 | OH | A01 | a1 | B01 | b1 | C03 | c2 | D02 | d1 | E01 |
| 2-2-dlE01-1-0126 | 642.260 | OH | A02 | a1 | B03 | b1 | C14 | c1 | D01 | d1 | E01 |
| 2-2-d1E01-1-0127 | 642.267 | OH | A01 | a1 | B05 | b4 | C13 | c1 | D01 | d1 | E01 |
| 2-2-d1E01-1-0128 | 642.284 | OH | A02 | a1 | B02 | b2 | C02 | c1 | D01 | d1 | E01 |
| 2-2-d1E01-1-0129 | 642.284 | OH | A02 | a1 | B02 | b2 | C03 | c1 | D01 | d1 | E01 |
| 2-2-d1E01-1-0130 | 643.210 | OH | A01 | a1 | B01 | b1 | C04 | c2 | D02 | d1 | E01 |
| 2-2-d1E01-1-0131 | 643.279 | OH | A02 | a1 | B02 | b2 | C04 | c1 | D01 | d1 | E01 |
| 2-2-d1E01-1-0132 | 643.279 | OH | A02 | a1 | B02 | b2 | C06 | c1 | D01 | d1 | E01 |
| 2-2-d1E01-1-0133 | 643.279 | OH | A02 | a1 | B02 | b2 | C07 | c1 1 | D01 | d1 | E01 |
| 2-2-d1E01-1-0134 | 644.247 | OH | AOI | a1 | B03 | b4 | C05 | c1 | D01 | d1 | E01 |
| 2-2-d1E01-1-0135 | 645.206 | OH | A02 | a1 | B03 | b3 | C10 | c1 | D01 | d1 | E01 |
| 2-2-d1E01-1-0136 | 646.210 | OH | A01 | a1 | B02 | b1 | C01 | c2 | D02 | d1 | E01 |
| 2-2-d1E01-1-0137 | 646.240 | OH | A01 | a1 | B05 | b1 | C02 | c1 | D01 | d1 | E01 |
| 2-2-d1E01-1-0138 | 646.240 | OH | AOI | a1 | B05 | b1 | C03 | c1 | D01 | d1 | E01 |
| 2-2-d1E01-1-0139 | 646.259 | OH | A02 | a1 | B03 | b2 | C02 | c1 | D01 | d1 | E01 |
| 2-2-d1E01-1-0140 | 646.259 | OH | A02 | a1 | B03 | b2 | C03 | c1 | D01 | d1 | E01 |
| 2-2-d1E01-1-0141 | 646.316 | OH | A02 | a1 | B01 | b4 | C02 | c1 | D01 | d1 | E01 |
| 2-2-d1E01-1-0142 | 647.236 | OH | A01 | a1 | B05 | b1 | C04 | c1 | D01 | d1 | E01 |
| 2-2-d1E01-1-0143 | 647.254 | OH | A02 | a1 | B03 | b2 | C04 | c1 | D01 | d1 | E01 |
| 2-2-dlE01-1-0144 | 647.254 | OH | A02 | a1 | B03 | b2 | C06 | c1 | D01 | d1 | E01 |
| 2-2-d1E01-1-0145 | 647.254 | OH | A02 | a1 | B03 | b2 | C07 | c1 | D01 | d1 | E01 |
| 2-2-d1E01-1-0146 | 647.311 | OH | A02 | a1 | B02 | b | 1 C15 | c1 | D01 | d1 | E01 |
| 2-2-d1E01-1-0147 | 648.171 | OH | A01 | a1 | B01 | b1 | COS | c2 | D02 | d1 | E01 |
| 2-2-d1E01-1-0148 | 648.258 | OH | AOI | a1 | B02 | b3 | C 11 | c1 | D01 | d1 | E01 |
| 2-2-dlE01-1-0149 | 648.306 | OH | A02 | a1 | B02 | b1 | C16 | c1 | D01 | d1 | E01 |
| 2-2-d1E01-1-0150 | 649.184 | OH | AOI | a1 | B01 | b3 | C08 | c2 | D02 | d1 | E01 |
| 2-2-d1E01-1-0151 | 649.301 | OH | A02 | a1 | B02 | b1 | C17 | c1 | D01 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0152 | 650.185 | OH | A01 | a1 | B03 | b1 | C01 | c2 | D02 | d1 | E01 |
| 2-2-d1E01-1-0153 | 650.277 | OH | AOI | a1 | B02 | b4 | C12 | c2 | D02 | d1 | E01 |
| 2-2-d1E01-1-0154 | 650.310 | OH | A02 | a1 | B02 | b4 | C01 | c1 | D01 | d1 | E01 |
| 2-2-d1E01-1-0155 | 651.215 | OH | A02 | a1 | B04 | b1 | C01 | c1 | D01 | d1 | E01 |
| 2-2-d1E01-1-0156 | 651.286 | OH | A02 | a1 | B03 | b1 | C15 | c1 | D01 | d1 | E01 |
| 2-2-d1E01-1-0157 | 652.197 | OH | A01 | a1 | B05 | b1 | C05 | c1 | D01 | d1 | E01 |
| 2-2-d1E01-1-0158 | 652.233 | OH | A01 | a1 | B03 | b3 | C 11 | c1 | DOI | d1 | E01 |
| 2-2-d1E01-1-0159 | 652.257 | OH | A01 | a1 | B02 | b4 | C13 | c2 | D02 | d1 | E01 |
| 2-2-dlE01-1-0 160 | 652.272 | OH | A02 | a1 | B01 | b4 | COS | c1 | DOI | d1 | E01 |
| 2-2-d1E01-1-0161 | 652.281 | OH | A02 | a1 | B03 | b1 | C16 | c1 | DOI | d1 | E01 |
| 2-2-d1E01-1-0162 | 653.206 | OH | A01 | a1 | B04 | b3 | C09 | c1 | D01 | d1 | E01 |
| 2-2-dlE01-1-0 163 | 653.210 | OH | A01 | a1 | B05 | b3 | C08 | c1 | DOI | d1 | E01 |
| 2-2-d1E01-1-0164 | 653.276 | OH | A02 | a1 | B03 | b | 1 C17 | c1 | D01 | d1 | E01 |
| 2-2-d1E01-1-0165 | 654.252 | OH | A01 | a1 | B03 | b4 | C12 | c2 | D02 | d1 | E01 |
| 2-2-d1E01-1-0166 | 654.262 | OH | A02 | a1 | B02 | b1 | C18 | c1 | D01 | d1 | E01 |
| 2-2-d1E01-1-0167 | 654.285 | OH | A02 | a1 | B03 | b4 | C01 | c1 | D01 | d1 | E01 |
| 2-2-d1E01-1-0168 | 655.221 | OH | A01 | a1 | B04 | b1 | C14 | c1 | DOI | d1 | E01 |
| 2-2-d1E01-1-0169 | 655.283 | OH | A02 | a1 | B04 | b4 | C12 | c1 | D01 | d1 | E01 |
| 2-2-d1E01-1-0170 | 656.230 | OH | A01 | a1 | B02 | b1 | C02 | c2 | D02 | d1 | E01 |
| 2-2-d1E01-1-0171 | 656.230 | OH | A01 | a1 | B02 | b1 | C03 | c2 | D02 | d1 | E01 |
| 2-2-d1E01-1-0172 | 656.232 | OH | A01 | a1 | B03 | b4 | C13 | c2 | D02 | d1 | E01 |
| 2-2-d1E01-1-0173 | 657.226 | OH | A01 | a1 | B02 | b1 | C04 | c2 | D02 | d1 | E01 |
| 2-2-d1E01-1-0174 | 657.262 | OH | A02 | a1 | B04 | b4 | C13 | c1 | D01 | d1 | E01 |
| 2-2-d1E01-1-0175 | 658.167 | OH | A01 | a1 | B04 | b3 | C10 | c1 | DOI | d1 | E01 |
| 2-2-d1E01-1-0176 | 658.210 | OH | A02 | a1 | B01 | b1 | C01 | c2 | D02 | d1 | E01 |
| 2-2-d1E01-1-0177 | 658.237 | OH | A02 | a1 | B03 | b1 | C18 | c1 | D01 | d1 | E01 |
| 2-2-d1E01-1-0178 | 659.220 | OH | AOI | a1 | B04 | b2 | C02 | c1 | D01 | d1 | E01 |
| 2-2-d1E01-1-0179 | 659.220 | OH | A01 | a1 | B04 | b2 | C03 | c1 | D01 | d1 | E01 |
| 2-2-d1E01-1-0180 | 660.200 | OH | A01 | a1 | B01 | b3 | C09 | c2 | D02 | d1 | E01 |
| 2-2-d1E01-1-0181 | 660.205 | OH | AOI | a1 | B03 | b1 | C02 | c2 | D02 | d1 | E01 |
| 2-2-d1E01-1-0182 | 660.205 | OH | A01 | a1 | B03 | b1 | C03 | c2 | D02 | d1 | E01 |
| 2-2-d1E01-1-0183 | 660.215 | OH | A01 | a1 | B04 | b2 | C04 | c1 | D01 | d1 | E01 |
| 2-2-d1E01-1-0184 | 660.215 | OH | A01 | a1 | B04 | b2 | C06 | c1 | D01 | d1 | E01 |
| 2-2-d1E01-1-0185 | 660.215 | OH | AOI | a1 | B04 | b2 | C07 | c1 | D01 | d1 | E01 |
| 2-2-d1E01-1-0186 | 660.258 | OH | A02 | a1 | B01 | b3 | C 11 | c1 | D01 | d1 | E01 |
| 2-2-d1E01-1-0187 | 660.331 | OH | A02 | a1 | B02 | b4 | C02 | c1 | D01 | d1 | E01 |
| 2-2-d1E01-1-0188 | 661.201 | OH | AOI | a1 | B03 | b1 | C04 | c2 | D02 | d1 | E01 |
| 2-2-d1E01-1-0189 | 661.236 | OH | A02 | a1 | B04 | b1 | C02 | c1 | D01 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0190 | 661.236 | OH | A02 | a1 | B04 | b1 | C03 | c1 | D01 | d1 | E01 |
| 2-2-d1E01-1-0191 | 662.187 | OH | A01 | a1 | B02 | b1 | C05 | c2 | D02 | d1 | E01 |
| 2-2-d1E01-1-0192 | 662.216 | OH | A01 | a1 | B01 | b1 | C14 | c2 | D02 | d1 | E01 |
| 2-2-d1E01-1-0193 | 662.231 | OH | A02 | a1 | B04 | b1 | C04 | c1 | D01 | d1 | E01 |
| 2-2-d1E01-1-0194 | 662.235 | OH | A02 | a1 | B05 | b1 | C01 | c1 | DOI | d1 | E01 |
| 2-2-d1E01-1-0195 | 662.277 | OH | A02 | a1 | B01 | b4 | C12 | c2 | D02 | d1 | E01 |
| 2-2-d1E01-1-0196 | 662.322 | OH | A01 | a1 | B01 | b1 | C19 | c1 | DOI | d1 | E01 |
| 2-2-d1E01-1-0197 | 663.200 | OH | AOI | a1 | B02 | b3 | C08 | c2 | D02 | d1 | E01 |
| 2-2-d1E01-1-0198 | 664.226 | OH | A01 | a1 | B05 | b3 | C09 | c1 | DOI | d1 | E01 |
| 2-2-d1E01-1-0199 | 664.247 | OH | A01 | a1 | B04 | b1 | C15 | c1 | DOI | d1 | E01 |
| 2-2-d1E01-1-0200 | 664.257 | OH | A02 | a1 | B01 | b4 | C13 | c2 | D02 | d1 | E01 |
| 2-2-dlE01-1-0201 | 664.306 | OH | A02 | a1 | B03 | b4 | C02 | c1 | DOI | d1 | E01 |
| 2-2-d1E01-1-0202 | 665.161 | OH | A01 | a1 | B01 | b3 | C10 | c2 | D02 | d1 | E01 |
| 2-2-d1E01-1-0203 | 665.242 | OH | A01 | a1 | B04 | b1 | C16 | c1 | D01 | d1 | E01 |
| 2-2-d1E01-1-0204 | 666.162 | OH | A01 | a1 | B03 | b1 | C05 | c2 | D02 | d1 | E01 |
| 2-2-d1E01-1-0205 | 666.215 | OH | A01 | a1 | B01 | b2 | C02 | c2 | D02 | d1 | E01 |
| 2-2-d1E01-1-0206 | 666.215 | OH | A01 | a1 | B01 | b2 | C03 | c2 | D02 | d1 | E01 |
| 2-2-d1E01-1-0207 | 666.237 | OH | A01 | a1 | B04 | b1 | C17 | c1 | D01 | d1 | E01 |
| 2-2-d1E01-1-0208 | 666.241 | OH | A01 | a1 | B05 | b1 | C14 | c1 | D01 | d1 | E01 |
| 2-2-d1E01-1-0209 | 666.288 | OH | A02 | a1 | B02 | b4 | C05 | c1 | D01 | d1 | E01 |
| 2-2-d1E01-1-0210 | 666.303 | OH | A02 | a1 | B05 | b4 | C12 | c1 | D01 | d1 | E01 |
| 2-2-d1E01-1-0211 | 667.175 | OH | A01 | a1 | B03 | b3 | C08 | c2 | D02 | d1 | E01 |
| 2-2-d1E01-1-0212 | 667.192 | OH | A02 | a1 | B04 | b1 | C05 | c1 | D01 | d1 | E01 |
| 2-2-d1E01-1-0213 | 667.210 | OH | A01 | a1 | B01 | b2 | C04 | c2 | D02 | d1 | E01 |
| 2-2-d1E01-1-0214 | 667.210 | OH | A01 | a1 | B01 | b2 | C06 | c2 | D02 | d1 | E01 |
| 2-2-d1E01-1-0215 | 667.210 | OH | A01 | a1 | B01 | b2 | C07 | c2 | D02 | d1 | E01 |
| 2-2-d1E01-1-0216 | 667.246 | OH | A01 | a1 | B04 | b4 | C01 | c1 | D01 | d1 | E01 |
| 2-2-d1E01-1-0217 | 668.205 | OH | A02 | a1 | B04 | b3 | C08 | c1 | D01 | d1 | E01 |
| 2-2-d1E01-1-0218 | 668.230 | OH | A02 | a1 | B01 | b1 | C02 | c2 | D02 | d1 | E01 |
| 2-2-d1E01-1-0219 | 668.230 | OH | A02 | a1 | B01 | b1 | C03 | c2 | D02 | d1 | E01 |
| 2-2-d1E01-1-0220 | 668.282 | OH | A02 | a1 | B05 | b4 | C13 | c1 | D01 | d1 | E01 |
| 2-2-d1E01-1-0221 | 669.187 | OH | A01 | a1 | B05 | b3 | C10 | c1 | D01 | d1 | E01 |
| 2-2-d1E01-1-0222 | 669.226 | OH | A02 | a1 | B01 | b1 | C04 | c2 | D02 | d1 | E01 |
| 2-2-d1E01-1-0223 | 670.240 | OH | A01 | a1 | B05 | b2 | C02 | c1 | D01 | d1 | E01 |
| 2-2-d1E01-1-0224 | 670.240 | OH | A01 | a1 | B05 | b2 | C03 | c1 | D01 | d1 | E01 |
| 2-2-d1E01-1-0225 | 670.263 | OH | A02 | a1 | B03 | b4 | C05 | c1 | D01 | d1 | E01 |
| 2-2-d1E01-1-0226 | 671.198 | OH | A01 | a1 | B04 | b1 | C18 | c1 | D01 | d1 | E01 |
| 2-2-d1E01-1-0227 | 671.236 | OH | A01 | a1 | B05 | b2 | C04 | c1 | D01 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0228 | 671.236 | OH | A01 | a1 | B05 | b2 | C06 | c1 | D01 | d1 | E01 |
| 2-2-d1E01-1-0229 | 671.236 | OH | A01 | a1 | B05 | b2 | C07 | c1 | D01 | d1 | E01 |
| 2-2-d1E01-1-0230 | 671.241 | OH | A01 | a1 | B01 | b1 | C15 | c2 | D02 | d1 | E01 |
| 2-2-d1E01-1-0231 | 672.225 | OH | A02 | a1 | B02 | b1 | C01 | c2 | D02 | d1 | E01 |
| 2-2-d1E01-1-0232 | 672.237 | OH | A01 | a1 | B01 | b1 | C16 | c2 | D02 | d1 | E01 |
| 2-2-d1E01-1-0233 | 672.256 | OH | A02 | a1 | B05 | b1 | C02 | c1 | D01 | d1 | E01 |
| 2-2-d1E01-1-0234 | 672.256 | OH | A02 | a1 | B05 | b1 | C03 | c1 | D01 | d1 | E01 |
| 2-2-d1E01-1-0235 | 673.232 | OH | A01 | a1 | B01 | b1 | C17 | c2 | D02 | d1 | E01 |
| 2-2-d1E01-1-0236 | 673.251 | OH | A02 | a1 | B05 | b1 | C04 | c1 | D01 | d1 | E01 |
| 2-2-d1E01-1-0237 | 674.187 | OH | A02 | a1 | B01 | b1 | C05 | c2 | D02 | d1 | E01 |
| 2-2-d1E01-1-0238 | 674.216 | OH | A01 | a1 | B02 | b3 | C09 | c2 | D02 | d1 | E01 |
| 2-2-d1E01-1-0239 | 674.241 | OH | A01 | a1 | B01 | b4 | C01 | c2 | D02 | d1 | E01 |
| 2-2-d1E01-1-0240 | 674.274 | OH | A02 | a1 | B02 | b3 | C11 | c1 | D01 | d1 | E01 |
| 2-2-d1E01-1-0241 | 675.200 | OH | A02 | a1 | B01 | b3 | C08 | c2 | D02 | d1 | E01 |
| 2-2-d1E01-1-0242 | 675.267 | OH | A01 | a1 | B05 | b1 | C15 | c1 | D01 | d1 | E01 |
| 2-2-d1E01-1-0243 | 676.200 | OH | A02 | a1 | B03 | b1 | C01 | c2 | D02 | d1 | E01 |
| 2-2-d1E01-1-0244 | 676.232 | OH | A01 | a1 | B02 | b1 | C14 | c2 | D02 | d1 | E01 |
| 2-2-d1E01-1-0245 | 676.262 | OH | A01 | a1 | B05 | b1 | C16 | c1 | D01 | d1 | E01 |
| 2-2-d1E01-1-0246 | 676.293 | OH | A02 | a1 | B02 | b4 | C12 | c2 | D02 | d1 | E01 |
| 2-2-d1E01-1-0247 | 676.337 | OH | A01 | a1 | B02 | b1 | C19 | c1 | D01 | d1 | E01 |
| 2-2-d1E01-1-0248 | 677.257 | OH | A01 | a1 | B05 | b1 | C17 | c1 | D01 | d1 | E01 |
| 2-2-d1E01-1-0249 | 677.267 | OH | A01 | a1 | B04 | b4 | C02 | c1 | D01 | d1 | E01 |
| 2-2-d1E01-1-0250 | 678.191 | OH | A01 | a1 | B03 | b3 | C09 | c2 | D02 | d1 | E01 |
| 2-2-d1E01-1-0251 | 678.193 | OH | A01 | a1 | B01 | b1 | C18 | c2 | D02 | d1 | E01 |
| 2-2-d1E01-1-0252 | 678.212 | OH | A02 | a1 | B05 | b1 | C05 | c1 | D01 | d1 | E01 |
| 2-2-d1E01-1-0253 | 678.249 | OH | A02 | a1 | B03 | b3 | C11 | c1 | D01 | d1 | E01 |
| 2-2-d1E01-1-0254 | 678.267 | OH | A01 | a1 | B05 | b4 | C01 | c1 | D01 | d1 | E01 |
| 2-2-d1E01-1-0255 | 678.272 | OH | A02 | a1 | B02 | b4 | C13 | c2 | D02 | d1 | E01 |
| 2-2-d1E01-1-0256 | 679.177 | OH | A01 | a1 | B02 | b3 | C10 | c2 | D02 | d1 | E01 |
| 2-2-d1E01-1-0257 | 679.221 | OH | A02 | a1 | B04 | b3 | C09 | c1 | D01 | d1 | E01 |
| 2-2-d1E01-1-0258 | 679.225 | OH | A02 | a1 | B05 | b3 | C08 | c1 | D01 | d1 | E01 |
| 2-2-d1E01-1-0259 | 680.206 | OH | A01 | a1 | B03 | b1 | C14 | c2 | D02 | d1 | E01 |
| 2-2-d1E01-1-0260 | 680.230 | OH | A01 | a1 | B02 | b2 | C02 | c2 | D02 | d1 | E01 |
| 2-2-d1E01-1-0261 | 680.230 | OH | A01 | a1 | B02 | b2 | C03 | c2 | D02 | d1 | E01 |
| 2-2-d1E01-1-0262 | 680.268 | OH | A02 | a1 | B03 | b4 | C12 | c2 | D02 | d1 | E01 |
| 2-2-d1E01-1-0263 | 680.312 | OH | A01 | a1 | B03 | b1 | C19 | c1 | D01 | d1 | E01 |
| 2-2-d1E01-1-0264 | 681.226 | OH | A01 | a1 | B02 | b2 | C04 | c2 | D02 | d1 | E01 |
| 2-2-d1E01-1-0265 | 681.226 | OH | A01 | a1 | B02 | b2 | C06 | c2 | D02 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0266 | 681.226 | OH | A01 | a1 | B02 | b2 | C07 | c2 | D02 | d1 | E01 |
| 2-2-d1E01-1-0267 | 681.237 | OH | A02 | a1 | B04 | b1 | C14 | c1 | D01 | d1 | E01 |
| 2-2-d1E01-1-0268 | 682.219 | OH | A01 | a1 | B05 | b1 | C18 | c1 | D01 | d1 | E01 |
| 2-2-d1E01-1-0269 | 682.246 | OH | A02 | a1 | B02 | b1 | C02 | c2 | D02 | d1 | E01 |
| 2-2-d1E01-1-0270 | 682.246 | OH | A02 | a1 | B02 | b1 | C03 | c2 | D02 | d1 | E01 |
| 2-2-d1E01-1-0271 | 682.247 | OH | A02 | a1 | B03 | b4 | C13 | c2 | D02 | d1 | E01 |
| 2-2-d1E01-1-0272 | 683.152 | OH | A01 | a1 | B03 | b3 | C10 | c2 | D02 | d1 | E01 |
| 2-2-d1E01-1-0273 | 683.224 | OH | A01 | a1 | B04 | b4 | C05 | c1 | D01 | d1 | E01 |
| 2-2-d1E01-1-0274 | 683.241 | OH | A02 | a1 | B02 | b1 | C04 | c2 | D02 | d1 | E01 |
| 2-2-d1E01-1-0275 | 684.182 | OH | A02 | a1 | B04 | b3 | C10 | c1 | D01 | d1 | E01 |
| 2-2-d1E01-1-0276 | 684.205 | OH | A01 | a1 | B03 | b2 | C02 | c2 | D02 | d1 | E01 |
| 2-2-d1E01-1-0277 | 684.205 | OH | A01 | a1 | B03 | b2 | C03 | c2 | D02 | d1 | E01 |
| 2-2-d1E01-1-0278 | 684.262 | OH | A01 | a1 | B01 | b4 | C02 | c2 | D02 | d1 | E01 |
| 2-2-d1E01-1-0279 | 685.201 | OH | A01 | a1 | B03 | b2 | C04 | c2 | D02 | d1 | E01 |
| 2-2-d1E01-1-0280 | 685.201 | OH | A01 | a1 | B03 | b2 | C06 | c2 | D02 | d1 | E01 |
| 2-2-d1E01-1-0281 | 685.201 | OH | A01 | a1 | B03 | b2 | C07 | c2 | D02 | d1 | E01 |
| 2-2-d1E01-1-0282 | 685.236 | OH | A02 | a1 | B04 | b2 | C02 | c1 | D01 | d1 | E01 |
| 2-2-d1E01-1-0283 | 685.236 | OH | A02 | a1 | B04 | b2 | C03 | c1 | D01 | d1 | E01 |
| 2-2-d1E01-1-0284 | 685.257 | OH | A01 | a1 | B02 | b1 | C15 | c2 | D02 | d1 | E01 |
| 2-2-d1E01-1-0285 | 686.216 | OH | A02 | a1 | B01 | b3 | C09 | c2 | D02 | d1 | E01 |
| 2-2-d1E01-1-0286 | 686.221 | OH | A02 | a1 | B03 | b1 | C02 | c2 | D02 | d1 | E01 |
| 2-2-d1E01-1-0287 | 686.221 | OH | A02 | a1 | B03 | b1 | C03 | c2 | D02 | d1 | E01 |
| 2-2-d1E01-1-0288 | 686.231 | OH | A02 | a1 | B04 | b2 | C04 | c1 | D01 | d1 | E01 |
| 2-2-d1E01-1-0289 | 686.231 | OH | A02 | a1 | B04 | b2 | C06 | c1 | D01 | d1 | E01 |
| 2-2-d1E01-1-0290 | 686.231 | OH | A02 | a1 | B04 | b2 | C07 | c1 | D01 | d1 | E01 |
| 2-2-d1E01-1-0291 | 686.252 | OH | A01 | a1 | B02 | b1 | C16 | c2 | D02 | d1 | E01 |
| 2-2-d1E01-1-0292 | 687.216 | OH | A02 | a1 | B03 | b1 | C04 | c2 | D02 | d1 | E01 |
| 2-2-d1E01-1-0293 | 687.248 | OH | A01 | a1 | B02 | b1 | C17 | c2 | D02 | d1 | E01 |
| 2-2-d1E01-1-0294 | 688.203 | OH | A02 | a1 | B02 | b1 | C05 | c2 | D02 | d1 | E01 |
| 2-2-d1E01-1-0295 | 688.232 | OH | A02 | a1 | B01 | b1 | C14 | c2 | D02 | d1 | E01 |
| 2-2-d1E01-1-0296 | 688.257 | OH | A01 | a1 | B02 | b4 | C01 | c2 | D02 | d1 | E01 |
| 2-2-d1E01-1-0297 | 688.287 | OH | A01 | a1 | B05 | b4 | C02 | c1 | D01 | d1 | E01 |
| 2-2-d1E01-1-0298 | 688.337 | OH | A02 | a1 | B01 | b1 | C19 | c1 | D01 | d1 | E01 |
| 2-2-d1E01-1-0299 | 689.161 | OH | A01 | a1 | B04 | b1 | C01 | c2 | D02 | d1 | E01 |
| 2-2-dlE01-1-0300 | 689.216 | OH | A02 | a1 | B02 | b3 | C08 | c2 | D02 | d1 | E01 |
| 2-2-d1E01-1-0301 | 689.232 | OH | A01 | a1 | B03 | b1 | C15 | c2 | D02 | d1 | E01 |
| 2-2-d1E01-1-0302 | 690.218 | OH | A01 | a1 | B01 | b4 | COS | c2 | D02 | d1 | E01 |
| 2-2-d1E01-1-0303 | 690.227 | OH | A01 | a1 | B03 | b1 | C16 | c2 | D02 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0304 | 690.241 | OH | A02 | a1 | B05 | b3 | C09 | c1 | D01 | d1 | E01 |
| 2-2-d1E01-1-0305 | 690.262 | OH | A02 | a1 | B04 | b1 | C15 | c1 | D01 | d1 | E01 |
| 2-2-d1E01-1-0306 | 691.177 | OH | A02 | a1 | B01 | b3 | C10 | c2 | D02 | d1 | E01 |
| 2-2-d1E01-1-0307 | 691.210 | OH | A01 | a1 | B04 | b3 | C11 | c1 | D01 | d1 | E01 |
| 2-2-d1E01-1-0308 | 691.222 | OH | A01 | a1 | B03 | b1 | C17 | c2 | D02 | d1 | E01 |
| 2-2-d1E01-1-0309 | 691.258 | OH | A02 | a1 | B04 | b1 | C16 | c1 | D01 | d1 | E01 |
| 2-2-d1E01-1-0310 | 692.177 | OH | A02 | a1 | B03 | b1 | C05 | c2 | D02 | d1 | E01 |
| 2-2-d1E01-1-0311 | 692.209 | OH | A01 | a1 | B02 | b1 | C18 | c2 | D02 | d1 | E01 |
| 2-2-d1E01-1-0312 | 692.230 | OH | A02 | a1 | B01 | b2 | C02 | c2 | D02 | d1 | E01 |
| 2-2-d1E01-1-0313 | 692.230 | OH | A02 | a1 | B01 | b2 | C03 | c2 | D02 | d1 | E01 |
| 2-2-d1E01-1-0314 | 692.232 | OH | A01 | a1 | B03 | b4 | C01 | c2 | D02 | d1 | E01 |
| 2-2-d1E01-1-0315 | 692.253 | OH | A02 | a1 | B04 | b1 | C17 | c1 | D01 | d1 | E01 |
| 2-2-d1E01-1-0316 | 692.257 | OH | A02 | a1 | B05 | b1 | C14 | c1 | D01 | d1 | E01 |
| 2-2-d1E01-1-0317 | 693.190 | OH | A02 | a1 | B03 | b3 | C08 | c2 | D02 | d1 | E01 |
| 2-2-d1E01-1-0318 | 693.226 | OH | A02 | a1 | B01 | b2 | C04 | c2 | D02 | d1 | E01 |
| 2-2-d1E01-1-0319 | 693.226 | OH | A02 | a1 | B01 | b2 | C06 | c2 | D02 | d1 | E01 |
| 2-2-d1E01-1-0320 | 693.226 | OH | A02 | a1 | B01 | b2 | C07 | c2 | D02 | d1 | E01 |
| 2-2-d1E01-1-0321 | 693.229 | OH | A01 | a1 | B04 | b4 | C12 | c2 | D02 | d1 | E01 |
| 2-2-d1E01-1-0322 | 693.262 | OH | A02 | a1 | B04 | b4 | C01 | c1 | D01 | d1 | E01 |
| 2-2-d1E01-1-0323 | 694.244 | OH | A01 | a1 | B05 | b4 | C05 | c1 | D01 | d1 | E01 |
| 2-2-d1E01-1-0324 | 695.203 | OH | A02 | a1 | B05 | b3 | C10 | c1 | D01 | d1 | E01 |
| 2-2-d1E01-1-0325 | 695.208 | OH | A01 | a1 | B04 | b4 | C13 | c2 | D02 | d1 | E01 |
| 2-2-d1E01-1-0326 | 696.184 | OH | A01 | a1 | B03 | b1 | C18 | c2 | D02 | d1 | E01 |
| 2-2-d1E01-1-0327 | 696.256 | OH | A02 | a1 | B05 | b2 | C02 | c1 | D01 | d1 | E01 |
| 2-2-d1E01-1-0328 | 696.256 | OH | A02 | a1 | B05 | b2 | C03 | c1 | D01 | d1 | E01 |
| 2-2-d1E01-1-0329 | 697.214 | OH | A02 | a1 | B04 | b1 | C18 | c1 | D01 | d1 | E01 |
| 2-2-d1E01-1-0330 | 697.251 | OH | A02 | a1 | B05 | b2 | C04 | c1 | D01 | d1 | E01 |
| 2-2-d1E01-1-0331 | 697.251 | OH | A02 | a1 | B05 | b2 | C06 | c1 | D01 | d1 | E01 |
| 2-2-d1E01-1-0332 | 697.251 | OH | A02 | a1 | B05 | b2 | C07 | c1 | D01 | d1 | E01 |
| 2-2-d1E01-1-0333 | 697.257 | OH | A02 | a1 | B01 | b1 | C15 | c2 | D02 | d1 | E01 |
| 2-2-d1E01-1-0334 | 698.205 | OH | A01 | a1 | B01 | b3 | C11 | c2 | D02 | d1 | E01 |
| 2-2-d1E01-1-0335 | 698.252 | OH | A02 | a1 | B01 | b1 | C16 | c2 | D02 | d1 | E01 |
| 2-2-d1E01-1-0336 | 698.277 | OH | A01 | a1 | B02 | b4 | C02 | c2 | D02 | d1 | E01 |
| 2-2-d1E01-1-0337 | 699.182 | OH | A01 | a1 | B04 | b1 | C02 | c2 | D02 | d1 | E01 |
| 2-2-d1E01-1-0338 | 699.182 | OH | A01 | a1 | B04 | b1 | C03 | c2 | D02 | d1 | E01 |
| 2-2-d1E01-1-0339 | 699.248 | OH | A02 | a1 | B01 | b1 | C17 | c2 | D02 | d1 | E01 |
| 2-2-d1E01-1-0340 | 700.177 | OH | A01 | a1 | B04 | b1 | C04 | c2 | D02 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0341 | 700.181 | OH | A01 | a1 | B05 | b1 | C01 | c2 | D02 | d1 | E01 |
| 2-2-d1E01-1-0342 | 700.232 | OH | A02 | a1 | B02 | b3 | C09 | c2 | D02 | d1 | E01 |
| 2-2-d1E01-1-0343 | 700.257 | OH | A02 | a1 | B01 | b4 | C01 | c2 | D02 | d1 | E01 |
| 2-2-d1E01-1-0344 | 701.283 | OH | A02 | a1 | B05 | b1 | C15 | c1 | D01 | d1 | E01 |
| 2-2-d1E01-1-0345 | 702.230 | OH | A01 | a1 | B05 | b3 | C11 | c1 | D01 | d1 | E01 |
| 2-2-d1E01-1-0346 | 702.247 | OH | A02 | a1 | B02 | b1 | C14 | c2 | D02 | d1 | E01 |
| 2-2-d1E01-1-0347 | 702.252 | OH | A01 | a1 | B03 | b4 | C02 | c2 | D02 | d1 | E01 |
| 2-2-d1E01-1-0348 | 702.278 | OH | A02 | a1 | B05 | b1 | C16 | c1 | D01 | d1 | E01 |
| 2-2-d1E01-1-0349 | 702.353 | OH | A02 | a1 | B02 | b1 | C19 | c1 | D01 | d1 | E01 |
| 2-2-d1E01-1-0350 | 703.273 | OH | A02 | a1 | B05 | b1 | C17 | c1 | D01 | d1 | E01 |
| 2-2-d1E01-1-0351 | 703.283 | OH | A02 | a1 | B04 | b4 | C02 | c1 | D01 | d1 | E01 |
| 2-2-d1E01-1-0352 | 704.206 | OH | A02 | a1 | B03 | b3 | C09 | c2 | D02 | d1 | E01 |
| 2-2-d1E01-1-0353 | 704.209 | OH | A02 | a1 | B01 | b1 | C18 | c2 | D02 | d1 | E01 |
| 2-2-d1E01-1-0354 | 704.234 | OH | A01 | a1 | B02 | b4 | C05 | c2 | D02 | d1 | E01 |
| 2-2-d1E01-1-0355 | 704.249 | OH | A01 | a1 | B05 | b4 | C12 | c2 | D02 | d1 | E01 |
| 2-2-d1E01-1-0356 | 704.282 | OH | A02 | a1 | B05 | b4 | C01 | c1 | D01 | d1 | E01 |
| 2-2-d1E01-1-0357 | 705.139 | OH | A01 | a1 | B04 | b1 | C05 | c2 | D02 | d1 | E01 |
| 2-2-d1E01-1-0358 | 705.193 | OH | A02 | a1 | B02 | b3 | C10 | c2 | D02 | d1 | E01 |
| 2-2-d1E01-1-0359 | 706.152 | OH | A01 | a1 | B04 | b3 | C08 | c2 | D02 | d1 | E01 |
| 2-2-d1E01-1-0360 | 706.222 | OH | A02 | a1 | B03 | b1 | C14 | c2 | D02 | d1 | E01 |
| 2-2-d1E01-1-0361 | 706.228 | OH | A01 | a1 | B05 | b4 | C13 | c2 | D02 | d1 | E01 |
| 2-2-d1E01-1-0362 | 706.246 | OH | A02 | a1 | B02 | b2 | C02 | c2 | D02 | d1 | E01 |
| 2-2-d1E01-1-0363 | 706.246 | OH | A02 | a1 | B02 | b2 | C03 | c2 | D02 | d1 | E01 |
| 2-2-d1E01-1-0364 | 706.328 | OH | A02 | a1 | B03 | b1 | C19 | c1 | D01 | d1 | E01 |
| 2-2-d1E01-1-0365 | 707.241 | OH | A02 | a1 | B02 | b2 | C04 | c2 | D02 | d1 | E01 |
| 2-2-d1E01-1-0366 | 707.241 | OH | A02 | a1 | B02 | b2 | C06 | c2 | D02 | d1 | E01 |
| 2-2-d1E01-1-0367 | 707.241 | OH | A02 | a1 | B02 | b2 | C07 | c2 | D02 | d1 | E01 |
| 2-2-d1E01-1-0368 | 708.209 | OH | A01 | a1 | B03 | b4 | C05 | c2 | D02 | d1 | E01 |
| 2-2-d1E01-1-0369 | 708.234 | OH | A02 | a1 | B05 | b1 | C18 | c1 | D01 | d1 | E01 |
| 2-2-d1E01-1-0370 | 709.168 | OH | A02 | a1 | B03 | b3 | C10 | c2 | D02 | d1 | E01 |
| 2-2-d1E01-1-0371 | 709.239 | OH | A02 | a1 | B04 | b4 | C05 | c1 | D01 | d1 | E01 |
| 2-2-d1E01-1-0372 | 710.202 | OH | A01 | a1 | B05 | b1 | C02 | c2 | D02 | d1 | E01 |
| 2-2-d1E01-1-0373 | 710.202 | OH | A01 | a1 | B05 | b1 | C03 | c2 | D02 | d1 | E01 |
| 2-2-d1E01-1-0374 | 710.221 | OH | A02 | a1 | B03 | b2 | C02 | c2 | D02 | d1 | E01 |
| 2-2-d1E01-1-0375 | 710.221 | OH | A02 | a1 | B03 | b2 | C03 | c2 | D02 | d1 | E01 |
| 2-2-d1E01-1-0376 | 710.277 | OH | A02 | a1 | B01 | b4 | C02 | c2 | D02 | d1 | E01 |
| 2-2-d1E01-1-0377 | 711.197 | OH | A01 | a1 | B05 | b1 | C04 | c2 | D02 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0378 | 711.216 | OH | A02 | a1 | B03 | b2 | C04 | c2 | D02 | d1 | E01 |
| 2-2-d1E01-1-0379 | 711.216 | OH | A02 | a1 | B03 | b2 | C06 | c2 | D02 | d1 | E01 |
| 2-2-d1E01-1-0380 | 711.216 | OH | A02 | a1 | B03 | b2 | C07 | c2 | D02 | d1 | E01 |
| 2-2-d1E01-1-0381 | 711.273 | OH | A02 | a1 | B02 | b1 | C15 | c2 | D02 | d1 | E01 |
| 2-2-d1E01-1-0382 | 712.220 | OH | A01 | a1 | B02 | b3 | C11 | c2 | D02 | d1 | E01 |
| 2-2-d1E01-1-0383 | 712.268 | OH | A02 | a1 | B02 | b1 | C16 | c2 | D02 | d1 | E01 |
| 2-2-d1E01-1-0384 | 713.263 | OH | A02 | a1 | B02 | b1 | C17 | c2 | D02 | d1 | E01 |
| 2-2-d1E01-1-0385 | 714.272 | OH | A02 | a1 | B02 | b4 | C01 | c2 | D02 | d1 | E01 |
| 2-2-d1E01-1-0386 | 714.303 | OH | A02 | a1 | B05 | b4 | C02 | c1 | D01 | d1 | E01 |
| 2-2-d1E01-1-0387 | 715.177 | OH | A02 | a1 | B04 | b1 | C01 | c2 | D02 | d1 | E01 |
| 2-2-d1E01-1-0388 | 715.248 | OH | A02 | a1 | B03 | b1 | C15 | c2 | D02 | d1 | E01 |
| 2-2-d1E01-1-0389 | 716.159 | OH | A01 | a1 | B05 | b1 | C05 | c2 | D02 | d1 | E01 |
| 2-2-d1E01-1-0390 | 716.195 | OH | A01 | a1 | B03 | b3 | C11 | c2 | D02 | d1 | E01 |
| 2-2-d1E01-1-0391 | 716.234 | OH | A02 | a1 | B01 | b4 | C05 | c2 | D02 | d1 | E01 |
| 2-2-d1E01-1-0392 | 716.243 | OH | A02 | a1 | B03 | b1 | C16 | c2 | D02 | d1 | E01 |
| 2-2-d1E01-1-0393 | 717.168 | OH | A01 | a1 | B04 | b3 | C09 | c2 | D02 | d1 | E01 |
| 2-2-d1E01-1-0394 | 717.172 | OH | A01 | a1 | B05 | b3 | C08 | c2 | D02 | d1 | E01 |
| 2-2-d1E01-1-0395 | 717.226 | OH | A02 | a1 | B04 | b3 | C11 | c1 | D01 | d1 | E01 |
| 2-2-d1E01-1-0396 | 717.238 | OH | A02 | a1 | B03 | b1 | C17 | c2 | D02 | d1 | E01 |
| 2-2-d1E01-1-0397 | 718.224 | OH | A02 | a1 | B02 | b1 | C18 | c2 | D02 | d1 | E01 |
| 2-2-d1E01-1-0398 | 718.247 | OH | A02 | a1 | B03 | b4 | C01 | c2 | D02 | d1 | E01 |
| 2-2-d1E01-1-0399 | 719.183 | OH | A01 | a1 | B04 | b1 | C14 | c2 | D02 | d1 | E01 |
| 2-2-d1E01-1-0400 | 719.245 | OH | A02 | a1 | B04 | b4 | C12 | c2 | D02 | d1 | E01 |
| 2-2-d1E01-1-0401 | 719.289 | OH | A01 | a1 | B04 | b1 | C19 | c1 | D01 | d1 | E01 |
| 2-2-d1E01-1-0402 | 720.259 | OH | A02 | a1 | B05 | b4 | C05 | c1 | D01 | d1 | E01 |
| 2-2-d1E01-1-0403 | 721.224 | OH | A02 | a1 | B04 | b4 | C13 | c2 | D02 | d1 | E01 |
| 2-2-d1E01-1-0404 | 722.129 | OH | A01 | a1 | B04 | b3 | C10 | c2 | D02 | d1 | E01 |
| 2-2-d1E01-1-0405 | 722.199 | OH | A02 | a1 | B03 | b1 | C18 | c2 | D02 | d1 | E01 |
| 2-2-d1E01-1-0406 | 723.182 | OH | A01 | a1 | B04 | b2 | C02 | c2 | D02 | d1 | E01 |
| 2-2-dlE01-1-0407 | 723.182 | OH | A01 | a1 | B04 | b2 | C03 | c2 | D02 | d1 | E01 |
| 2-2-d1E01-1-0408 | 724.177 | OH | A01 | a1 | B04 | b2 | C04 | c2 | D02 | d1 | E01 |
| 2-2-d1E01-1-0409 | 724.177 | OH | A01 | a1 | B04 | b2 | C06 | c2 | D02 | d1 | E01 |
| 2-2-d1E01-1-0410 | 724.177 | OH | A01 | a1 | B04 | b2 | C07 | c2 | D02 | d1 | E01 |
| 2-2-d1E01-1-0411 | 724.220 | OH | A02 | a1 | B01 | b3 | C11 | c2 | D02 | d1 | E01 |
| 2-2-d1E01-1-0412 | 724.293 | OH | A02 | a1 | B02 | b4 | C02 | c2 | D02 | d1 | E01 |
| 2-2-d1E01-1-0413 | 725.198 | OH | A02 | a1 | B04 | b1 | C02 | c2 | D02 | d1 | E01 |
| 2-2-d1E01-1-0414 | 725.198 | OH | A02 | a1 | B04 | b1 | C03 | c2 | D02 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0415 | 726.193 | OH | A02 | a1 | B04 | b1 | C04 | c2 | D02 | d1 | E01 |
| 2-2-d1E01-1-0416 | 726.197 | OH | A02 | a1 | B05 | b1 | C01 | c2 | D02 | d1 | E01 |
| 2-2-d1E01-1-0417 | 726.284 | OH | A01 | a1 | B01 | b1 | C19 | c2 | D02 | d1 | E01 |
| 2-2-d1E01-1-0418 | 728.188 | OH | A01 | a1 | B05 | b3 | C09 | c2 | D02 | d1 | E01 |
| 2-2-d1E01-1-0419 | 728.209 | OH | A01 | a1 | B04 | b1 | C15 | c2 | D02 | d1 | E01 |
| 2-2-d1E01-1-0420 | 728.246 | OH | A02 | a1 | B05 | b3 | C11 | c1 | D01 | d1 | E01 |
| 2-2-d1E01-1-0421 | 728.268 | OH | A02 | a1 | B03 | b4 | C02 | c2 | D02 | d1 | E01 |
| 2-2-d1E01-1-0422 | 729.204 | OH | A01 | a1 | B04 | b1 | C16 | c2 | D02 | d1 | E01 |
| 2-2-d1E01-1-0423 | 730.199 | OH | A01 | a1 | B04 | b1 | C17 | c2 | D02 | d1 | E01 |
| 2-2-d1E01-1-0424 | 730.203 | OH | A01 | a1 | B05 | b1 | C14 | c2 | D02 | d1 | E01 |
| 2-2-d1E01-1-0425 | 730.249 | OH | A02 | a1 | B02 | b4 | C05 | c2 | D02 | d1 | E01 |
| 2-2-d1E01-1-0426 | 730.265 | OH | A02 | a1 | B05 | b4 | C12 | c2 | D02 | d1 | E01 |
| 2-2-d1E01-1-0427 | 730.309 | OH | A01 | a1 | B05 | b1 | C19 | c1 | D01 | d1 | E01 |
| 2-2-d1E01-1-0428 | 731.154 | OH | A02 | a1 | B04 | b1 | C05 | c2 | D02 | d1 | E01 |
| 2-2-d1E01-1-0429 | 731.208 | OH | A01 | a1 | B04 | b4 | C01 | c2 | D02 | d1 | E01 |
| 2-2-d1E01-1-0430 | 732.167 | OH | A02 | a1 | B04 | b3 | C08 | c2 | D02 | d1 | E01 |
| 2-2-d1E01-1-0431 | 732.244 | OH | A02 | a1 | B05 | b4 | C13 | c2 | D02 | d1 | E01 |
| 2-2-d1E01-1-0432 | 733.149 | OH | A01 | a1 | B05 | b3 | C10 | c2 | D02 | d1 | E01 |
| 2-2-d1E01-1-0433 | 734.202 | OH | A01 | a1 | B05 | b2 | C02 | c2 | D02 | d1 | E01 |
| 2-2-d1E01-1-0434 | 734.202 | OH | A01 | a1 | B05 | b2 | C03 | c2 | D02 | d1 | E01 |
| 2-2-d1E01-1-0435 | 734.224 | OH | A02 | a1 | B03 | b4 | C05 | c2 | D02 | d1 | E01 |
| 2-2-d1E01-1-0436 | 735.160 | OH | A01 | a1 | B04 | b1 | C18 | c2 | D02 | d1 | E01 |
| 2-2-d1E01-1-0437 | 735.197 | OH | A01 | a1 | B05 | b2 | C04 | c2 | D02 | d1 | E01 |
| 2-2-d1E01-1-0438 | 735.197 | OH | A01 | a1 | B05 | b2 | C06 | c2 | D02 | d1 | E01 |
| 2-2-d1E01-1-0439 | 735.197 | OH | A01 | a1 | B05 | b2 | C07 | c2 | D02 | d1 | E01 |
| 2-2-d1E01-1-0440 | 736.218 | OH | A02 | a1 | B05 | b1 | C02 | c2 | D02 | d1 | E01 |
| 2-2-d1E01-1-0441 | 736.218 | OH | A02 | a1 | B05 | b1 | C03 | c2 | D02 | d1 | E01 |
| 2-2-d1E01-1-0442 | 737.213 | OH | A02 | a1 | B05 | b1 | C04 | c2 | D02 | d1 | E01 |
| 2-2-d1E01-1-0443 | 738.236 | OH | A02 | a1 | B02 | b3 | C11 | c2 | D02 | d1 | E01 |
| 2-2-d1E01-1-0444 | 739.229 | OH | A01 | a1 | B05 | b1 | C15 | c2 | D02 | d1 | E01 |
| 2-2-d1E01-1-0445 | 740.224 | OH | A01 | a1 | B05 | b1 | C16 | c2 | D02 | d1 | E01 |
| 2-2-d1E01-1-0446 | 740.299 | OH | A01 | a1 | B02 | b1 | C19 | c2 | D02 | d1 | E01 |
| 2-2-d1E01-1-0447 | 741.219 | OH | A01 | a1 | B05 | b1 | C17 | c2 | D02 | d1 | E01 |
| 2-2-d1E01-1-0448 | 741.229 | OH | A01 | a1 | B04 | b4 | C02 | c2 | D02 | d1 | E01 |
| 2-2-d1E01-1-0449 | 742.174 | OH | A02 | a1 | B05 | b1 | C05 | c2 | D02 | d1 | E01 |
| 2-2-d1E01-1-0450 | 742.211 | OH | A02 | a1 | B03 | b3 | C11 | c2 | D02 | d1 | E01 |
| 2-2-d1E01-1-0451 | 742.228 | OH | A01 | a1 | B05 | b4 | C01 | c2 | D02 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0452 | 743.183 | OH | A02 | a1 | B04 | b3 | C09 | c2 | D02 | d1 | E01 |
| 2-2-d1E01-1-0453 | 743.187 | OH | A02 | a1 | B05 | b3 | C08 | c2 | D02 | d1 | E01 |
| 2-2-d1E01-1-0454 | 744.274 | OH | A01 | a1 | B03 | b1 | C19 | c2 | D02 | d1 | E01 |
| 2-2-d1E01-1-0455 | 745.199 | OH | A02 | a1 | B04 | b1 | C14 | c2 | D02 | d1 | E01 |
| 2-2-d1E01-1-0456 | 745.305 | OH | A02 | a1 | B04 | b1 | C19 | c1 | D01 | d1 | E01 |
| 2-2-d1E01-1-0457 | 746.180 | OH | A01 | a1 | B05 | b1 | C18 | c2 | D02 | d1 | E01 |
| 2-2-d1E01-1-0458 | 747.186 | OH | A01 | a1 | B04 | b4 | C05 | c2 | D02 | d1 | E01 |
| 2-2-d1E01-1-0459 | 748.144 | OH | A02 | a1 | B04 | b3 | C10 | c2 | D02 | d1 | E01 |
| 2-2-d1E01-1-0460 | 749.198 | OH | A02 | a1 | B04 | b2 | C02 | c2 | D02 | d1 | E01 |
| 2-2-d1E01-1-0461 | 749.198 | OH | A02 | a1 | B04 | b2 | C03 | c2 | D02 | d1 | E01 |
| 2-2-d1E01-1-0462 | 750.193 | OH | A02 | a1 | B04 | b2 | C04 | c2 | D02 | d1 | E01 |
| 2-2-d1E01-1-0463 | 750.193 | OH | A02 | a1 | B04 | b2 | C06 | c2 | D02 | d1 | E01 |
| 2-2-d1E01-1-0464 | 750.193 | OH | A02 | a1 | B04 | b2 | C07 | c2 | D02 | d1 | E01 |
| 2-2-d1E01-1-0465 | 752.249 | OH | A01 | a1 | B05 | b4 | C02 | c2 | D02 | d1 | E01 |
| 2-2-d1E01-1-0466 | 752.299 | OH | A02 | a1 | B01 | b1 | C19 | c2 | D02 | d1 | E01 |
| 2-2-d1E01-1-0467 | 754.203 | OH | A02 | a1 | B05 | b3 | C09 | c2 | D02 | d1 | E01 |
| 2-2-d1E01-1-0468 | 754.224 | OH | A02 | a1 | B04 | b1 | C15 | c2 | D02 | d1 | E01 |
| 2-2-d1E01-1-0469 | 755.172 | OH | A01 | a1 | B04 | b3 | C11 | c2 | D02 | d1 | E01 |
| 2-2-d1E01-1-0470 | 755.220 | OH | A02 | a1 | B04 | b1 | C16 | c2 | D02 | d1 | E01 |
| 2-2-d1E01-1-0471 | 756.215 | OH | A02 | a1 | B04 | b1 | C17 | c2 | D02 | d1 | E01 |
| 2-2-d1E01-1-0472 | 756.219 | OH | A02 | a1 | B05 | b1 | C14 | c2 | D02 | d1 | E01 |
| 2-2-d1E01-1-0473 | 756.325 | OH | A02 | a1 | B05 | b1 | C19 | c1 | D01 | d1 | E01 |
| 2-2-d1E01-1-0474 | 757.224 | OH | A02 | a1 | B04 | b4 | C01 | c2 | D02 | d1 | E01 |
| 2-2-d1E01-1-0475 | 758.206 | OH | A01 | a1 | B05 | b4 | C05 | c2 | D02 | d1 | E01 |
| 2-2-d1E01-1-0476 | 759.164 | OH | A02 | a1 | B05 | b3 | C10 | c2 | D02 | d1 | E01 |
| 2-2-d1E01-1-0477 | 760.218 | OH | A02 | a1 | B05 | b2 | C02 | c2 | D02 | d1 | E01 |
| 2-2-d1E01-1-0478 | 760.218 | OH | A02 | a1 | B05 | b2 | C03 | c2 | D02 | d1 | E01 |
| 2-2-d1E01-1-0479 | 761.176 | OH | A02 | a1 | B04 | b1 | C18 | c2 | D02 | d1 | E01 |
| 2-2-d1E01-1-0480 | 761.213 | OH | A02 | a1 | B05 | b2 | C04 | c2 | D02 | d1 | E01 |
| 2-2-d1E01-1-0481 | 761.213 | OH | A02 | a1 | B05 | b2 | C06 | c2 | D02 | d1 | E01 |
| 2-2-d1E01-1-0482 | 761.213 | OH | A02 | a1 | B05 | b2 | C07 | c2 | D02 | d1 | E01 |
| 2-2-d1E01-1-0483 | 765.244 | OH | A02 | a1 | B05 | b1 | C15 | c2 | D02 | d1 | E01 |
| 2-2-d1E01-1-0484 | 766.192 | OH | A01 | a1 | B05 | b3 | C11 | c2 | D02 | d1 | E01 |
| 2-2-d1E01-1-0485 | 766.240 | OH | A02 | a1 | B05 | b1 | C16 | c2 | D02 | d1 | E01 |
| 2-2-d1E01-1-0486 | 766.315 | OH | A02 | a1 | B02 | b1 | C19 | c2 | D02 | d1 | E01 |
| 2-2-d1E01-1-0487 | 767.235 | OH | A02 | a1 | B05 | b1 | C17 | c2 | D02 | d1 | E01 |
| 2-2-dlE01-1-0488 | 767.245 | OH | A02 | a1 | B04 | b4 | C02 | c2 | D02 | d1 | E01 |

(continued)

| [Table 9-5-2] Compound that may be contained in mixture 2-2-d1E01-1 | | | | | | | | | | | |
|---|---|---|---|---|---|---|---|---|---|---|---|
| ID | Exact Mass | | A | a | B | b | C | c | D | d | E |
| 2-2-d1E01-1-0489 | 768.244 | OH | A02 | a1 | B05 | b4 | C01 | c2 | D02 | d1 | E01 |
| 2-2-d1E01-1-0490 | 770.290 | OH | A02 | a1 | B03 | b1 | C19 | c2 | D02 | d1 | E01 |
| 2-2-d1E01-1-0491 | 772.196 | OH | A02 | a1 | B05 | b1 | C18 | c2 | D02 | d1 | E01 |
| 2-2-d1E01-1-0492 | 773.201 | OH | A02 | a1 | B04 | b4 | COS | c2 | D02 | d1 | E01 |
| 2-2-d1E01-1-0493 | 778.265 | OH | A02 | a1 | B05 | b4 | C02 | c2 | D02 | d1 | E01 |
| 2-2-dlE01-1-0494 | 781.188 | OH | A02 | a1 | B04 | b3 | C11 | c2 | D02 | d1 | E01 |
| 2-2-d1E01-1-0495 | 783.251 | OH | A01 | a1 | B04 | b1 | C19 | c2 | D02 | d1 | E01 |
| 2-2-d1E01-1-0496 | 784.221 | OH | A02 | a1 | B05 | b4 | C05 | c2 | D02 | d1 | E01 |
| 2-2-dlE01-1-0497 | 792.208 | OH | A02 | a1 | B05 | b3 | C11 | c2 | D02 | d1 | E01 |
| 2-2-dlE01-1-0498 | 794.271 | OH | A01 | a1 | B05 | b1 | C19 | c2 | D02 | d1 | E01 |
| 2-2-dlE01-1-0499 | 809.267 | OH | A02 | a1 | B04 | b1 | C19 | c2 | D02 | d1 | E01 |
| 2-2-d1E01-1-0500 | 820.287 | OH | A02 | a1 | B05 | b1 | C19 | c2 | D02 | d1 | E01 |

[Table 493]

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0001 | 584.227 | OH | A01 | a1 | B01 | b1 | C01 | c1 | DOI | d1 | E02 |
| 2-2-d1E02-1-0002 | 588.295 | OH | A01 | a1 | B01 | b4 | C12 | c1 | D01 | d1 | E02 |
| 2-2-d1E02-1-0003 | 590.274 | OH | A01 | a1 | B01 | b4 | C13 | c1 | D01 | d1 | E02 |
| 2-2-d1E02-1-0004 | 594.248 | OH | A01 | a1 | B01 | b1 | C02 | c1 | D01 | d1 | E02 |
| 2-2-d1E02-1-0005 | 594.248 | OH | A01 | a1 | B01 | b1 | C03 | c1 | D01 | d1 | E02 |
| 2-2-d1E02-1-0006 | 595.243 | OH | A01 | a1 | B01 | b1 | C04 | c1 | D01 | d1 | E02 |
| 2-2-d1E02-1-0007 | 598.243 | OH | A01 | a1 | B02 | b1 | C01 | c1 | D01 | d1 | E02 |
| 2-2-d1E02-1-0008 | 600.204 | OH | A01 | a1 | B01 | b1 | COS | c1 | D01 | d1 | E02 |
| 2-2-d1E02-1-0009 | 601.217 | OH | A01 | a1 | B01 | b3 | C08 | c1 | D01 | d1 | E02 |
| 2-2-d1E02-1-0010 | 602.218 | OH | A01 | a1 | B03 | b1 | C01 | c1 | D01 | d1 | E02 |
| 2-2-d1E02-1-0011 | 602.310 | OH | A01 | a1 | B02 | b4 | C12 | c1 | D01 | d1 | E02 |
| 2-2-d1E02-1-0012 | 604.290 | OH | A01 | a1 | B02 | b4 | C13 | c1 | D01 | d1 | E02 |
| 2-2-d1E02-1-0013 | 606.285 | OH | A01 | a1 | B03 | b4 | C12 | c1 | D01 | d1 | E02 |
| 2-2-d1E02-1-0014 | 608.263 | OH | A01 | a1 | B02 | b1 | C02 | c1 | D01 | d1 | E02 |
| 2-2-d1E02-1-0015 | 608.263 | OH | A01 | a1 | B02 | b1 | C03 | c1 | D01 | d1 | E02 |
| 2-2-d1E02-1-0016 | 608.265 | OH | A01 | a1 | B03 | b4 | C13 | c1 | D01 | d1 | E02 |
| 2-2-d1E02-1-0017 | 609.259 | OH | A01 | a1 | B02 | b1 | C04 | c1 | D01 | d1 | E02 |
| 2-2-d1E02-1-0018 | 610.243 | OH | A02 | a1 | B01 | b1 | C01 | c1 | D01 | d1 | E02 |
| 2-2-d1E02-1-0019 | 612.233 | OH | A01 | a1 | B01 | b3 | C09 | c1 | D01 | d1 | E02 |
| 2-2-d1E02-1-0020 | 612.238 | OH | A01 | a1 | B03 | b1 | C02 | c1 | D01 | d1 | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0021 | 612.238 | OH | A01 | a1 | B03 | b1 | C03 | c1 | D01 | d1 | E02 |
| 2-2-d1E02-1-0022 | 613.234 | OH | A01 | a1 | B03 | b1 | C04 | c1 | D01 | d1 | E02 |
| 2-2-d1E02-1-0023 | 614.220 | OH | A01 | a1 | B02 | b1 | C05 | c1 | D01 | d1 | E02 |
| 2-2-d1E02-1-0024 | 614.249 | OH | A01 | a1 | B01 | b1 | C14 | c1 | D01 | d1 | E02 |
| 2-2-d1E02-1-0025 | 614.310 | OH | A02 | a1 | B01 | b4 | C12 | c1 | D01 | d1 | E02 |
| 2-2-d1E02-1-0026 | 615.233 | OH | A01 | a1 | B02 | b3 | C08 | c1 | D01 | d1 | E02 |
| 2-2-d1E02-1-0027 | 616.290 | OH | A02 | a1 | B01 | b4 | C13 | c1 | D01 | d1 | E02 |
| 2-2-d1E02-1-0028 | 617.194 | OH | A01 | a1 | B01 | b3 | C10 | c1 | D01 | d1 | E02 |
| 2-2-d1E02-1-0029 | 618.195 | OH | A01 | a1 | B03 | b1 | C05 | c1 | D01 | d1 | E02 |
| 2-2-dlE02-1-0030 | 618.248 | OH | A01 | a1 | B01 | b2 | C02 | c1 | D01 | d1 | E02 |
| 2-2-d1E02-1-0031 | 618.248 | OH | A01 | a1 | B01 | b2 | C03 | c1 | D01 | d1 | E02 |
| 2-2-d1E02-1-0032 | 619.208 | OH | A01 | a1 | B03 | b3 | C08 | c1 | D01 | d1 | E02 |
| 2-2-d1E02-1-0033 | 619.243 | OH | A01 | a1 | B01 | b2 | C04 | c1 | D01 | d1 | E02 |
| 2-2-d1E02-1-0034 | 619.243 | OH | A01 | a1 | B01 | b2 | C06 | c1 | D01 | d1 | E02 |
| 2-2-dlE02-1-0035 | 619.243 | OH | A01 | a1 | B01 | b2 | C07 | c1 | D01 | d1 | E02 |
| 2-2-d1E02-1-0036 | 620.263 | OH | A02 | a1 | B01 | b1 | C02 | c1 | D01 | d1 | E02 |
| 2-2-dlE02-1-0037 | 620.263 | OH | A02 | a1 | B01 | b1 | C03 | c1 | D01 | d1 | E02 |
| 2-2-dlE02-1-0038 | 621.259 | OH | A02 | a1 | B01 | b1 | C04 | c1 | D01 | d1 | E02 |
| 2-2-d1E02-1-0039 | 623.274 | OH | A01 | a1 | B01 | b1 | C15 | c1 | D01 | d1 | E02 |
| 2-2-dlE02-1-0040 | 624.258 | OH | A02 | a1 | B02 | b1 | C01 | c1 | D01 | d1 | E02 |
| 2-2-dlE02-1-0041 | 624.270 | OH | A01 | a1 | B01 | b1 | C16 | c1 | D01 | d1 | E02 |
| 2-2-d1E02-1-0042 | 625.265 | OH | A01 | a1 | B01 | b1 | C17 | c1 | D01 | d1 | E02 |
| 2-2-d1E02-1-0043 | 626.220 | OH | A02 | a1 | B01 | b1 | COS | c1 | D01 | d1 | E02 |
| 2-2-d1E02-1-0044 | 626.249 | OH | A01 | a1 | B02 | b3 | C09 | c1 | D01 | d1 | E02 |
| 2-2-d1E02-1-0045 | 626.274 | OH | A01 | a1 | B01 | b4 | C01 | c1 | D01 | d1 | E02 |
| 2-2-d1E02-1-0046 | 627.233 | OH | A02 | a1 | B01 | b3 | C08 | c1 | D01 | d1 | E02 |
| 2-2-d1E02-1-0047 | 628.233 | OH | A02 | a1 | B03 | b1 | C01 | c1 | D01 | d1 | E02 |
| 2-2-dlE02-1-0048 | 628.265 | OH | A01 | a1 | B02 | b1 | C14 | c1 | D01 | d1 | E02 |
| 2-2-dlE02-1-0049 | 628.326 | OH | A02 | a1 | B02 | b4 | C12 | c1 | D01 | d1 | E02 |
| 2-2-d1E02-1-0050 | 630.224 | OH | A01 | a1 | B03 | b3 | C09 | c1 | D01 | d1 | E02 |
| 2-2-d1E02-1-0051 | 630.226 | OH | A01 | a1 | B01 | b1 | C18 | c1 | D01 | d1 | E02 |
| 2-2-d1E02-1-0052 | 630.305 | OH | A02 | a1 | B02 | b4 | C13 | c1 | D01 | d1 | E02 |
| 2-2-d1E02-1-0053 | 631.210 | OH | A01 | a1 | B02 | b3 | C10 | c1 | D01 | d1 | E02 |
| 2-2-dlE02-1-0054 | 632.239 | OH | A01 | a1 | B03 | b1 | C14 | c1 | D01 | d1 | E02 |
| 2-2-dlE02-1-0055 | 632.263 | OH | A01 | a1 | B02 | b2 | C02 | c1 | D01 | d1 | E02 |
| 2-2-d1E02-1-0056 | 632.263 | OH | A01 | a1 | B02 | b2 | C03 | c1 | D01 | d1 | E02 |
| 2-2-d1E02-1-0057 | 632.301 | OH | A02 | a1 | B03 | b4 | C12 | c1 | D01 | d1 | E02 |
| 2-2-dlE02-1-0058 | 633.259 | OH | A01 | a1 | B02 | b2 | C04 | c1 | D01 | d1 | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-dlE02-1-0059 | 633.259 | OH | A01 | a1 | B02 | b2 | C06 | c1 | D01 | d1 | E02 |
| 2-2-dlE02-1-0060 | 633.259 | OH | A01 | a1 | B02 | b2 | C07 | c1 | D01 | d1 | E02 |
| 2-2-d1E02-1-0061 | 634.279 | OH | A02 | a1 | B02 | b1 | C02 | c1 | D01 | d1 | E02 |
| 2-2-d1E02-1-0062 | 634.279 | OH | A02 | a1 | B02 | b1 | C03 | c1 | D01 | d1 | E02 |
| 2-2-d1E02-1-0063 | 634.280 | OH | A02 | a1 | B03 | b4 | C13 | c1 | D01 | d1 | E02 |
| 2-2-dlE02-1-0064 | 635.185 | OH | A01 | a1 | B03 | b3 | C10 | c1 | D01 | d1 | E02 |
| 2-2-dlE02-1-0065 | 635.274 | OH | A02 | a1 | B02 | b1 | C04 | c1 | D01 | d1 | E02 |
| 2-2-d1E02-1-0066 | 636.238 | OH | A01 | a1 | B03 | b2 | C02 | c1 | D01 | d1 | E02 |
| 2-2-d1E02-1-0067 | 636.238 | OH | A01 | a1 | B03 | b2 | C03 | c1 | D01 | d1 | E02 |
| 2-2-d1E02-1-0068 | 636.295 | OH | A01 | a1 | B01 | b4 | C02 | c1 | D01 | d1 | E02 |
| 2-2-dlE02-1-0069 | 637.234 | OH | A01 | a1 | B03 | 11 b2 | C04 | c1 | D01 | d1 | E02 |
| 2-2-dlE02-1-0070 | 637.234 | OH | A01 | a1 | B03 | b2 | C06 | c1 | D01 | d1 | E02 |
| 2-2-d1E02-1-0071 | 637.234 | OH | A01 | a1 | B03 | b2 | C07 | c1 | D01 | d1 | E02 |
| 2-2-d1E02-1-0072 | 637.290 | OH | A01 | a1 | B02 | b1 | C15 | c1 | D01 | d1 | E02 |
| 2-2-dlE02-1-0073 | 638.249 | OH | A02 | a1 | B01 | b3 | C09 | c1 | D01 | d1 | E02 |
| 2-2-d1E02-1-0074 | 638.254 | OH | A02 | a1 | B03 | b1 | C02 | c1 | D01 | d1 | E02 |
| 2-2-d1E02-1-0075 | 638.254 | OH | A02 | a1 | B03 | b1 | C03 | c1 | D01 | d1 | E02 |
| 2-2-dlE02-1-0076 | 638.285 | OH | A01 | a1 | B02 | b1 | C16 | c1 | D01 | d1 | E02 |
| 2-2-d1E02-1-0077 | 639.249 | OH | A02 | a1 | B03 | b1 | C04 | c1 | D01 | d1 | E02 |
| 2-2-d1E02-1-0078 | 639.281 | OH | A01 | a1 | B02 | b1 | C17 | c1 | D01 | d1 | E02 |
| 2-2-d1E02-1-0079 | 640.236 | OH | A02 | a1 | B02 | b1 | COS | c1 | D01 | d1 | E02 |
| 2-2-dlE02-1-0080 | 640.265 | OH | A02 | a1 | B01 | b1 | C14 | c1 | D01 | d1 | E02 |
| 2-2-d1E02-1-0081 | 640.290 | OH | A01 | a1 | B02 | b4 | C01 | c1 | D01 | d1 | E02 |
| 2-2-dlE02-1-0082 | 641.194 | OH | A01 | a1 | B04 | b1 | C01 | c1 | D01 | d1 | E02 |
| 2-2-d1E02-1-0083 | 641.249 | OH | A02 | a1 | B02 | b3 | C08 | c1 | D01 | d1 | E02 |
| 2-2-dlE02-1-0084 | 641.265 | OH | A01 | a1 | B03 | b1 | C15 | c1 | D01 | d1 | E02 |
| 2-2-dlE02-1-0085 | 642.251 | OH | A01 | a1 | B01 | b4 | C05 | c1 | D01 | d1 | E02 |
| 2-2-dlE02-1-0086 | 642.260 | OH | A01 | a1 | B03 | b1 | C16 | c1 | D01 | d1 | E02 |
| 2-2-d1E02-1-0087 | 643.210 | OH | A02 | a1 | B01 | b3 | C10 | c1 | D01 | d1 | E02 |
| 2-2-dlE02-1-0088 | 643.255 | OH | A01 | a1 | B03 | b1 | C17 | c1 | D01 | d1 | E02 |
| 2-2-dlE02-1-0089 | 644.210 | OH | A02 | a1 | B03 | b1 | C05 | c1 | D01 | d1 | E02 |
| 2-2-dlE02-1-0090 | 644.242 | OH | A01 | a1 | B02 | b1 | C18 | c1 | D01 | d1 | E02 |
| 2-2-d1E02-1-0091 | 644.263 | OH | A02 | a1 | B01 | b2 | C02 | c1 | D01 | d1 | E02 |
| 2-2-d1E02-1-0092 | 644.263 | OH | A02 | a1 | B01 | b2 | C03 | c1 | D01 | d1 | E02 |
| 2-2-d1E02-1-0093 | 644.265 | OH | A01 | a1 | B03 | b4 | C01 | c1 | D01 | d1 | E02 |
| 2-2-d1E02-1-0094 | 645.223 | OH | A02 | a1 | B03 | b3 | C08 | c1 | D01 | d1 | E02 |
| 2-2-dlE02-1-0095 | 645.259 | OH | A02 | a1 | B01 | b2 | C04 | c1 | D01 | d1 | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-dlE02-1-0096 | 645.259 | OH | A02 | a1 | B01 | b2 | C06 | c1 | D01 | d1 | E02 |
| 2-2-d1E02-1-0097 | 645.259 | OH | A02 | a1 | B01 | b2 | C07 | c1 | D01 | d1 | E02 |
| 2-2-d1E02-1-0098 | 645.262 | OH | A01 | a1 | B04 | b4 | C12 | c1 | D01 | d1 | E02 |
| 2-2-d1E02-1-0099 | 647.241 | OH | A01 | a1 | B04 | b4 | C13 | c1 | D01 | d1 | E02 |
| 2-2-dlE02-1-0100 | 648.189 | OH | A01 | a1 | B01 | b1 | C01 | c2 | D02 | d1 | E02 |
| 2-2-d1E02-1-0101 | 648.217 | OH | A01 | a1 | B03 | b1 | C18 | c1 | D01 | d1 | E02 |
| 2-2-d1E02-1-0102 | 649.290 | OH | A02 | a1 | B01 | b1 | C15 | c1 | D01 | d1 | E02 |
| 2-2-d1E02-1-0103 | 650.238 | OH | A01 | a1 | B01 | b3 | C11 | c1 | D01 | d1 | E02 |
| 2-2-dlE02-1-0104 | 650.285 | OH | A02 | a1 | B01 | b1 | C16 | c1 | D01 | d1 | E02 |
| 2-2-d1E02-1-0105 | 650.310 | OH | A01 | a1 | B02 | b4 | C02 | c1 | D01 | d1 | E02 |
| 2-2-d1E02-1-0106 | 651.215 | OH | A01 | a1 | B04 | b1 | C02 | c1 | D01 | d1 | E02 |
| 2-2-d1E02-1-0107 | 651.215 | OH | A01 | a1 | B04 | b1 | C03 | c1 | D01 | d1 | E02 |
| 2-2-d1E02-1-0108 | 651.281 | OH | A02 | a1 | B01 | b1 | C17 | c1 | D01 | d1 | E02 |
| 2-2-d1E02-1-0109 | 652.210 | OH | A01 | a1 | B04 | b1 | C04 | c1 | D01 | d1 | E02 |
| 2-2-d1E02-1-0110 | 652.214 | OH | A01 | a1 | B05 | b1 | C01 | c1 | D01 | d1 | E02 |
| 2-2-d1E02-1-0111 | 652.257 | OH | A01 | a1 | B01 | b4 | C12 | c2 | D02 | d1 | E02 |
| 2-2-d1E02-1-0112 | 652.265 | OH | A02 | a1 | B02 | b3 | C09 | c1 | D01 | d1 | E02 |
| 2-2-d1E02-1-0113 | 652.290 | OH | A02 | a1 | B01 | b4 | C01 | c1 | D01 | d1 | E02 |
| 2-2-d1E02-1-0114 | 654.236 | OH | A01 | a1 | B01 | b4 | C13 | c2 | D02 | d1 | E02 |
| 2-2-d1E02-1-0115 | 654.280 | OH | A02 | a1 | B02 | b1 | C14 | c1 | D01 | d1 | E02 |
| 2-2-d1E02-1-0116 | 654.285 | OH | A01 | a1 | B03 | b4 | C02 | c1 | D01 | d1 | E02 |
| 2-2-d1E02-1-0117 | 656.239 | OH | A02 | a1 | B03 | b3 | C09 | c1 | D01 | d1 | E02 |
| 2-2-d1E02-1-0118 | 656.242 | OH | A02 | a1 | B01 | b1 | C18 | c1 | D01 | d1 | E02 |
| 2-2-d1E02-1-0119 | 656.267 | OH | A01 | a1 | B02 | b4 | COS | c1 | D01 | d1 | E02 |
| 2-2-d1E02-1-0120 | 656.282 | OH | A01 | a1 | B05 | b4 | C12 | c1 | D01 | d1 | E02 |
| 2-2-d1E02-1-0121 | 657.172 | OH | A01 | a1 | B04 | b1 | COS | c1 | D01 | d1 | E02 |
| 2-2-d1E02-1-0122 | 657.226 | OH | A02 | a1 | B02 | b3 | C10 | c1 | D01 | d1 | E02 |
| 2-2-d1E02-1-0123 | 658.185 | OH | A01 | a1 | B04 | b3 | C08 | c1 | D01 | d1 | E02 |
| 2-2-d1E02-1-0124 | 658.210 | OH | A01 | a1 | B01 | b1 | C02 | c2 | D02 | d1 | E02 |
| 2-2-d1E02-1-0125 | 658.210 | OH | A01 | a1 | B01 | b1 | C03 | c2 | D02 | d1 | E02 |
| 2-2-d1E02-1-0126 | 658.255 | OH | A02 | a1 | B03 | b1 | C14 | c1 | D01 | d1 | E02 |
| 2-2-d1E02-1-0127 | 658.261 | OH | A01 | a1 | B05 | b4 | C13 | c1 | D01 | d1 | E02 |
| 2-2-d1E02-1-0128 | 658.279 | OH | A02 | a1 | B02 | b2 | C02 | c1 | D01 | d1 | E02 |
| 2-2-d1E02-1-0129 | 658.279 | OH | A02 | a1 | B02 | b2 | C03 | c1 | D01 | d1 | E02 |
| 2-2-d1E02-1-0130 | 659.205 | OH | A01 | a1 | B01 | b1 | C04 | c2 | D02 | d1 | E02 |
| 2-2-d1E02-1-0131 | 659.274 | OH | A02 | a1 | B02 | b2 | C04 | c1 | D01 | d1 | E02 |
| 2-2-d1E02-1-0132 | 659.274 | OH | A02 | a1 | B02 | b2 | C06 | c1 | D01 | d1 | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0133 | 659.274 | OH | A02 | a1 | B02 | b2 | C07 | c1 | D01 | d1 | E02 |
| 2-2-d1E02-1-0134 | 660.242 | OH | A01 | a1 | B03 | b4 | C05 | c1 | D01 | d1 | E02 |
| 2-2-d1E02-1-0135 | 661.201 | OH | A02 | a1 | B03 | b3 | C10 | c1 | D01 | d1 | E02 |
| 2-2-d1E02-1-0136 | 662.205 | OH | A01 | a1 | B02 | b1 | C01 | c2 | D02 | d1 | E02 |
| 2-2-d1E02-1-0137 | 662.235 | OH | A01 | a1 | B05 | b1 | C02 | c1 | D01 | d1 | E02 |
| 2-2-d1E02-1-0138 | 662.235 | OH | A01 | a1 | B05 | b1 | C03 | c1 | D01 | d1 | E02 |
| 2-2-d1E02-1-0139 | 662.254 | OH | A02 | a1 | B03 | b2 | C02 | c1 | D01 | d1 | E02 |
| 2-2-d1E02-1-0140 | 662.254 | OH | A02 | a1 | B03 | b2 | C03 | c1 | D01 | d1 | E02 |
| 2-2-d1E02-1-0141 | 662.310 | OH | A02 | a1 | B01 | b4 | C02 | c1 | D01 | d1 | E02 |
| 2-2-d1E02-1-0142 | 663.230 | OH | A01 | a1 | B05 | b1 | C04 | c1 | D01 | d1 | E02 |
| 2-2-d1E02-1-0143 | 663.249 | OH | A02 | a1 | B03 | b2 | C04 | c1 | D01 | d1 | E02 |
| 2-2-d1E02-1-0144 | 663.249 | OH | A02 | a1 | B03 | b2 | C06 | c1 | D01 | d1 | E02 |
| 2-2-d1E02-1-0145 | 663.249 | OH | A02 | a1 | B03 | b2 | C07 | c1 | D01 | d1 | E02 |
| 2-2-d1E02-1-0146 | 663.306 | OH | A02 | a1 | B02 | b1 | C15 | c1 | D01 | d1 | E02 |
| 2-2-d1E02-1-0147 | 664.166 | OH | A01 | a1 | B01 | b1 | C05 | c2 | D02 | d1 | E02 |
| 2-2-d1E02-1-0148 | 664.253 | OH | A01 | a1 | B02 | b3 | C11 | c1 | D01 | d1 | E02 |
| 2-2-d1E02-1-0149 | 664.301 | OH | A02 | a1 | B02 | b1 | C16 | c1 | D01 | d1 | E02 |
| 2-2-d1E02-1-0150 | 665.179 | OH | A01 | a1 | B01 | b3 | C08 | c2 | D02 | d1 | E02 |
| 2-2-d1E02-1-0151 | 665.296 | OH | A02 | a1 | B02 | b1 | C17 | c1 | D01 | d1 | E02 |
| 2-2-d1E02-1-0152 | 666.180 | OH | A01 | a1 | B03 | b1 | C01 | c2 | D02 | d1 | E02 |
| 2-2-d1E02-1-0153 | 666.272 | OH | A01 | a1 | B02 | b4 | C12 | c2 | D02 | d1 | E02 |
| 2-2-d1E02-1-0154 | 666.305 | OH | A02 | a1 | B02 | b4 | C01 | c1 | D01 | d1 | E02 |
| 2-2-d1E02-1-0155 | 667.210 | OH | A02 | a1 | B04 | b1 | C01 | c1 | D01 | d1 | E02 |
| 2-2-d1E02-1-0156 | 667.281 | OH | A02 | a1 | B03 | b1 | C15 | c1 | D01 | d1 | E02 |
| 2-2-d1E02-1-0157 | 668.192 | OH | A01 | a1 | B05 | b1 | C05 | c1 | D01 | d1 | E02 |
| 2-2-d1E02-1-0158 | 668.228 | OH | A01 | a1 | B03 | b3 | C11 | c1 | D01 | d1 | E02 |
| 2-2-d1E02-1-0159 | 668.252 | OH | A01 | a1 | B02 | b4 | C13 | c2 | D02 | d1 | E02 |
| 2-2-d1E02-1-0160 | 668.267 | OH | A02 | a1 | B01 | b4 | C05 | c1 | D01 | d1 | E02 |
| 2-2-d1E02-1-0161 | 668.276 | OH | A02 | a1 | B03 | b1 | C16 | c1 | D01 | d1 | E02 |
| 2-2-d1E02-1-0162 | 669.201 | OH | A01 | a1 | B04 | b3 | C09 | c1 | D01 | d1 | E02 |
| 2-2-d1E02-1-0163 | 669.205 | OH | A01 | a1 | B05 | b3 | C08 | c1 | D01 | d1 | E02 |
| 2-2-d1E02-1-0164 | 669.271 | OH | A02 | a1 | B03 | b1 | C17 | c1 | D01 | d1 | E02 |
| 2-2-d1E02-1-0165 | 670.247 | OH | A01 | a1 | B03 | b4 | C12 | c2 | D02 | d1 | E02 |
| 2-2-d1E02-1-0166 | 670.257 | OH | A02 | a1 | B02 | b1 | C18 | c1 | D01 | d1 | E02 |
| 2-2-d1E02-1-0167 | 670.280 | OH | A02 | a1 | B03 | b4 | C01 | c1 | D01 | d1 | E02 |
| 2-2-d1E02-1-0168 | 671.216 | OH | A01 | a1 | B04 | b1 | C14 | c1 | D01 | d1 | E02 |
| 2-2-d1E02-1-0169 | 671.278 | OH | A02 | a1 | B04 | b4 | C12 | c1 | D01 | d1 | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0170 | 672.225 | OH | A01 | a1 | B02 | b1 | C02 | c2 | D02 | d1 | E02 |
| 2-2-d1E02-1-0171 | 672.225 | OH | A01 | a1 | B02 | b1 | C03 | c2 | D02 | d1 | E02 |
| 2-2-d1E02-1-0172 | 672.227 | OH | A01 | a1 | B03 | b4 | C13 | c2 | D02 | d1 | E02 |
| 2-2-d1E02-1-0173 | 673.221 | OH | A01 | a1 | B02 | b1 | C04 | c2 | D02 | d1 | E02 |
| 2-2-d1E02-1-0174 | 673.257 | OH | A02 | a1 | B04 | b4 | C13 | c1 | D01 | d1 | E02 |
| 2-2-d1E02-1-0175 | 674.162 | OH | A01 | a1 | B04 | b3 | C10 | c1 | D01 | d1 | E02 |
| 2-2-d1E02-1-0176 | 674.205 | OH | A02 | a1 | B01 | b1 | C01 | c2 | D02 | d1 | E02 |
| 2-2-d1E02-1-0177 | 674.232 | OH | A02 | a1 | B03 | b1 | C18 | c1 | D01 | d1 | E02 |
| 2-2-d1E02-1-0178 | 675.215 | OH | A01 | a1 | B04 | b2 | C02 | c1 | D01 | d1 | E02 |
| 2-2-d1E02-1-0179 | 675.215 | OH | A01 | a1 | B04 | b2 | C03 | c1 | D01 | d1 | E02 |
| 2-2-d1E02-1-0180 | 676.195 | OH | A01 | a1 | B01 | b3 | C09 | c2 | D02 | d1 | E02 |
| 2-2-d1E02-1-0181 | 676.200 | OH | A01 | a1 | B03 | b1 | C02 | c2 | D02 | d1 | E02 |
| 2-2-d1E02-1-0182 | 676.200 | OH | A01 | a1 | B03 | b1 | C03 | c2 | D02 | d1 | E02 |
| 2-2-d1E02-1-0183 | 676.210 | OH | A01 | a1 | B04 | b2 | C04 | c1 | D01 | d1 | E02 |
| 2-2-d1E02-1-0184 | 676.210 | OH | A01 | a1 | B04 | b2 | C06 | c1 | D01 | d1 | E02 |
| 2-2-d1E02-1-0185 | 676.210 | OH | A01 | a1 | B04 | b2 | C07 | c1 | D01 | d1 | E02 |
| 2-2-d1E02-1-0186 | 676.253 | OH | A02 | a1 | B01 | b3 | C11 | c1 | D01 | d1 | E02 |
| 2-2-d1E02-1-0187 | 676.326 | OH | A02 | a1 | B02 | b4 | C02 | c1 | D01 | d1 | E02 |
| 2-2-d1E02-1-0188 | 677.196 | OH | A01 | a1 | B03 | b1 | C04 | c2 | D02 | d1 | E02 |
| 2-2-d1E02-1-0189 | 677.231 | OH | A02 | a1 | B04 | b1 | C02 | c1 | D01 | d1 | E02 |
| 2-2-d1E02-1-0190 | 677.231 | OH | A02 | a1 | B04 | b1 | C03 | c1 | D01 | d1 | E02 |
| 2-2-d1E02-1-0191 | 678.182 | OH | A01 | a1 | B02 | b1 | C05 | c2 | D02 | d1 | E02 |
| 2-2-d1E02-1-0192 | 678.211 | OH | A01 | a1 | B01 | b1 | C14 | c2 | D02 | d1 | E02 |
| 2-2-d1E02-1-0193 | 678.226 | OH | A02 | a1 | B04 | b1 | C04 | c1 | D01 | d1 | E02 |
| 2-2-d1E02-1-0194 | 678.230 | OH | A02 | a1 | B05 | b1 | C01 | c1 | D01 | d1 | E02 |
| 2-2-d1E02-1-0195 | 678.272 | OH | A02 | a1 | B01 | b4 | C12 | c2 | D02 | d1 | E02 |
| 2-2-d1E02-1-0196 | 678.317 | OH | A01 | a1 | B01 | b1 | C19 | c1 | D01 | d1 | E02 |
| 2-2-d1E02-1-0197 | 679.195 | OH | A01 | a1 | B02 | b3 | C08 | c2 | D02 | d1 | E02 |
| 2-2-d1E02-1-0198 | 680.221 | OH | A01 | a1 | B05 | b3 | C09 | c1 | D01 | d1 | E02 |
| 2-2-d1E02-1-0199 | 680.242 | OH | A01 | a1 | B04 | b1 | C15 | c1 | D01 | d1 | E02 |
| 2-2-d1E02-1-0200 | 680.252 | OH | A02 | a1 | B01 | b4 | C13 | c2 | D02 | d1 | E02 |
| 2-2-d1E02-1-0201 | 680.301 | OH | A02 | a1 | B03 | b4 | C02 | c1 | D01 | d1 | E02 |
| 2-2-d1E02-1-0202 | 681.156 | OH | A01 | a1 | B01 | b3 | C10 | c2 | D02 | d1 | E02 |
| 2-2-d1E02-1-0203 | 681.237 | OH | A01 | a1 | B04 | b1 | C16 | c1 | D01 | d1 | E02 |
| 2-2-d1E02-1-0204 | 682.157 | OH | A01 | a1 | B03 | b1 | C05 | c2 | D02 | d1 | E02 |
| 2-2-d1E02-1-0205 | 682.210 | OH | A01 | a1 | B01 | b2 | C02 | c2 | D02 | d1 | E02 |
| 2-2-d1E02-1-0206 | 682.210 | OH | A01 | a1 | B01 | b2 | C03 | c2 | D02 | d1 | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0207 | 682.232 | OH | A01 | a1 | B04 | b1 | C17 | c1 | D01 | d1 | E02 |
| 2-2-d1E02-1-0208 | 682.236 | OH | A01 | a1 | B05 | b1 | C14 | c1 | D01 | d1 | E02 |
| 2-2-d1E02-1-0209 | 682.283 | OH | A02 | a1 | B02 | b4 | C05 | c1 | D01 | d1 | E02 |
| 2-2-d1E02-1-0210 | 682.298 | OH | A02 | a1 | B05 | b4 | C12 | c1 | D01 | d1 | E02 |
| 2-2-d1E02-1-0211 | 683.170 | OH | A01 | a1 | B03 | b3 | C08 | c2 | D02 | d1 | E02 |
| 2-2-d1E02-1-0212 | 683.187 | OH | A02 | a1 | B04 | b1 | C05 | c1 | D01 | d1 | E02 |
| 2-2-d1E02-1-0213 | 683.205 | OH | A01 | a1 | B01 | b2 | C04 | c2 | D02 | d1 | E02 |
| 2-2-d1E02-1-0214 | 683.205 | OH | A01 | a1 | B01 | b2 | C06 | c2 | D02 | d1 | E02 |
| 2-2-d1E02-1-0215 | 683.205 | OH | A01 | a1 | B01 | b2 | C07 | c2 | D02 | d1 | E02 |
| 2-2-d1E02-1-0216 | 683.241 | OH | A01 | a1 | B04 | b4 | C01 | c1 | D01 | dl | E02 |
| 2-2-d1E02-1-0217 | 684.200 | OH | A02 | a1 | B04 | b3 | C08 | c1 | D01 | dl | E02 |
| 2-2-d1E02-1-0218 | 684.225 | OH | A02 | a1 | B01 | b1 | C02 | c2 | D02 | dl | E02 |
| 2-2-d1E02-1-0219 | 684.225 | OH | A02 | a1 | B01 | b1 | C03 | c2 | D02 | dl | E02 |
| 2-2-d1E02-1-0220 | 684.277 | OH | A02 | a1 | B05 | b4 | C13 | c1 | D01 | dl | E02 |
| 2-2-d1E02-1-0221 | 685.182 | OH | A01 | a1 | B05 | b3 | C10 | c1 | D01 | dl | E02 |
| 2-2-d1E02-1-0222 | 685.221 | OH | A02 | a1 | B01 | b1 | C04 | c2 | D02 | dl | E02 |
| 2-2-d1E02-1-0223 | 686.235 | OH | A01 | a1 | B05 | b2 | C02 | c1 | D01 | dl | E02 |
| 2-2-d1E02-1-0224 | 686.235 | OH | A01 | a1 | B05 | b2 | C03 | c1 | D01 | dl | E02 |
| 2-2-d1E02-1-0225 | 686.257 | OH | A02 | a1 | B03 | b4 | C05 | c1 | D01 | dl | E02 |
| 2-2-d1E02-1-0226 | 687.193 | OH | A01 | a1 | B04 | b1 | C18 | c1 | D01 | dl | E02 |
| 2-2-d1E02-1-0227 | 687.230 | OH | A01 | a1 | B05 | b2 | C04 | c1 | D01 | dl | E02 |
| 2-2-d1E02-1-0228 | 687.230 | OH | A01 | a1 | B05 | b2 | C06 | c1 | D01 | dl | E02 |
| 2-2-d1E02-1-0229 | 687.230 | OH | A01 | a1 | B05 | b2 | C07 | c1 | D01 | dl | E02 |
| 2-2-d1E02-1-0230 | 687.236 | OH | A01 | a1 | B01 | b1 | C15 | c2 | D02 | dl | E02 |
| 2-2-d1E02-1-0231 | 688.220 | OH | A02 | a1 | B02 | b1 | C01 | c2 | D02 | dl | E02 |
| 2-2-d1E02-1-0232 | 688.232 | OH | A01 | a1 | B01 | b1 | C16 | c2 | D02 | dl | E02 |
| 2-2-d1E02-1-0233 | 688.251 | OH | A02 | a1 | B05 | b1 | C02 | c1 | D01 | dl | E02 |
| 2-2-d1E02-1-0234 | 688.251 | OH | A02 | a1 | B05 | b1 | C03 | c1 | D01 | dl | E02 |
| 2-2-d1E02-1-0235 | 689.227 | OH | A01 | a1 | B01 | b1 | C17 | c2 | D02 | dl | E02 |
| 2-2-d1E02-1-0236 | 689.246 | OH | A02 | a1 | B05 | b1 | C04 | c1 | D01 | dl | E02 |
| 2-2-d1E02-1-0237 | 690.182 | OH | A02 | a1 | B01 | b1 | C05 | c2 | D02 | dl | E02 |
| 2-2-d1E02-1-0238 | 690.211 | OH | A01 | a1 | B02 | b3 | C09 | c2 | D02 | dl | E02 |
| 2-2-d1E02-1-0239 | 690.236 | OH | A01 | a1 | B01 | b4 | C01 | c2 | D02 | dl | E02 |
| 2-2-d1E02-1-0240 | 690.269 | OH | A02 | a1 | B02 | b3 | C11 | c1 | D01 | dl | E02 |
| 2-2-d1E02-1-0241 | 691.195 | OH | A02 | a1 | B01 | b3 | C08 | c2 | D02 | dl | E02 |
| 2-2-d1E02-1-0242 | 691.262 | OH | A01 | a1 | B05 | b1 | C15 | c1 | D01 | dl | E02 |
| 2-2-d1E02-1-0243 | 692.195 | OH | A02 | a1 | B03 | b1 | C01 | c2 | D02 | dl | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0244 | 692.226 | OH | A01 | a1 | B02 | b1 | C14 | c2 | D02 | dl | E02 |
| 2-2-d1E02-1-0245 | 692.257 | OH | A01 | a1 | B05 | b1 | C16 | c1 | D01 | dl | E02 |
| 2-2-d1E02-1-0246 | 692.288 | OH | A02 | a1 | B02 | b4 | C12 | c2 | D02 | dl | E02 |
| 2-2-d1E02-1-0247 | 692.332 | OH | A01 | a1 | B02 | b1 | C19 | c1 | D01 | dl | E02 |
| 2-2-d1E02-1-0248 | 693.252 | OH | A01 | a1 | B05 | b1 | C17 | c1 | D01 | dl | E02 |
| 2-2-d1E02-1-0249 | 693.262 | OH | A01 | a1 | B04 | b4 | C02 | c1 | D01 | dl | E02 |
| 2-2-d1E02-1-0250 | 694.186 | OH | A01 | a1 | B03 | b3 | C09 | c2 | D02 | dl | E02 |
| 2-2-d1E02-1-0251 | 694.188 | OH | A01 | a1 | B01 | b1 | C18 | c2 | D02 | dl | E02 |
| 2-2-d1E02-1-0252 | 694.207 | OH | A02 | a1 | B05 | b1 | C05 | c1 | D01 | dl | E02 |
| 2-2-d1E02-1-0253 | 694.244 | OH | A02 | a1 | B03 | b3 | C11 | c1 | D01 | dl | E02 |
| 2-2-d1E02-1-0254 | 694.261 | OH | A01 | a1 | B05 | b4 | C01 | c1 | D01 | dl | E02 |
| 2-2-d1E02-1-0255 | 694.267 | OH | A02 | a1 | B02 | b4 | C13 | c2 | D02 | dl | E02 |
| 2-2-d1E02-1-0256 | 695.172 | OH | A01 | a1 | B02 | b3 | C10 | c2 | D02 | dl | E02 |
| 2-2-d1E02-1-0257 | 695.216 | OH | A02 | a1 | B04 | b3 | C09 | c1 | D01 | dl | E02 |
| 2-2-d1E02-1-0258 | 695.220 | OH | A02 | a1 | B05 | b3 | C08 | c1 | D01 | dl | E02 |
| 2-2-d1E02-1-0259 | 696.201 | OH | A01 | a1 | B03 | b1 | C14 | c2 | D02 | dl | E02 |
| 2-2-d1E02-1-0260 | 696.225 | OH | A01 | a1 | B02 | b2 | C02 | c2 | D02 | dl | E02 |
| 2-2-d1E02-1-0261 | 696.225 | OH | A01 | a1 | B02 | b2 | C03 | c2 | D02 | dl | E02 |
| 2-2-d1E02-1-0262 | 696.263 | OH | A02 | a1 | B03 | b4 | C12 | c2 | D02 | dl | E02 |
| 2-2-d1E02-1-0263 | 696.307 | OH | A01 | a1 | B03 | b1 | C19 | c1 | D01 | dl | E02 |
| 2-2-d1E02-1-0264 | 697.221 | OH | A01 | a1 | B02 | b2 | C04 | c2 | D02 | dl | E02 |
| 2-2-d1E02-1-0265 | 697.221 | OH | A01 | a1 | B02 | b2 | C06 | c2 | D02 | dl | E02 |
| 2-2-d1E02-1-0266 | 697.221 | OH | A01 | a1 | B02 | b2 | C07 | c2 | D02 | dl | E02 |
| 2-2-d1E02-1-0267 | 697.232 | OH | A02 | a1 | B04 | b1 | C14 | c1 | D01 | dl | E02 |
| 2-2-d1E02-1-0268 | 698.213 | OH | A01 | a1 | B05 | b1 | C18 | c1 | D01 | dl | E02 |
| 2-2-d1E02-1-0269 | 698.241 | OH | A02 | a1 | B02 | b1 | C02 | c2 | D02 | dl | E02 |
| 2-2-d1E02-1-0270 | 698.241 | OH | A02 | a1 | B02 | b1 | C03 | c2 | D02 | dl | E02 |
| 2-2-d1E02-1-0271 | 698.242 | OH | A02 | a1 | B03 | b4 | C13 | c2 | D02 | dl | E02 |
| 2-2-d1E02-1-0272 | 699.147 | OH | A01 | a1 | B03 | b3 | C10 | c2 | D02 | dl | E02 |
| 2-2-d1E02-1-0273 | 699.219 | OH | A01 | a1 | B04 | b4 | C05 | c1 | D01 | dl | E02 |
| 2-2-d1E02-1-0274 | 699.236 | OH | A02 | a1 | B02 | b1 | C04 | c2 | D02 | dl | E02 |
| 2-2-d1E02-1-0275 | 700.177 | OH | A02 | a1 | B04 | b3 | C10 | c1 | D01 | dl | E02 |
| 2-2-d1E02-1-0276 | 700.200 | OH | A01 | a1 | B03 | b2 | C02 | c2 | D02 | dl | E02 |
| 2-2-d1E02-1-0277 | 700.200 | OH | A01 | a1 | B03 | b2 | C03 | c2 | D02 | dl | E02 |
| 2-2-d1E02-1-0278 | 700.257 | OH | A01 | a1 | B01 | b4 | C02 | c2 | D02 | dl | E02 |
| 2-2-d1E02-1-0279 | 701.196 | OH | A01 | a1 | B03 | b2 | C04 | c2 | D02 | dl | E02 |
| 2-2-d1E02-1-0280 | 701.196 | OH | A01 | a1 | B03 | b2 | C06 | c2 | D02 | dl | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0281 | 701.196 | OH | A01 | a1 | B03 | b2 | C07 | c2 | D02 | dl | E02 |
| 2-2-d1E02-1-0282 | 701.231 | OH | A02 | a1 | B04 | b2 | C02 | c1 | D01 | dl | E02 |
| 2-2-d1E02-1-0283 | 701.231 | OH | A02 | a1 | B04 | b2 | C03 | c1 | D01 | dl | E02 |
| 2-2-d1E02-1-0284 | 701.252 | OH | A01 | a1 | B02 | b1 | C15 | c2 | D02 | dl | E02 |
| 2-2-d1E02-1-0285 | 702.211 | OH | A02 | a1 | B01 | b3 | C09 | c2 | D02 | dl | E02 |
| 2-2-d1E02-1-0286 | 702.216 | OH | A02 | a1 | B03 | b1 | C02 | c2 | D02 | dl | E02 |
| 2-2-d1E02-1-0287 | 702.216 | OH | A02 | a1 | B03 | b1 | C03 | c2 | D02 | dl | E02 |
| 2-2-d1E02-1-0288 | 702.226 | OH | A02 | a1 | B04 | b2 | C04 | c1 | D01 | dl | E02 |
| 2-2-d1E02-1-0289 | 702.226 | OH | A02 | a1 | B04 | b2 | C06 | c1 | D01 | dl | E02 |
| 2-2-d1E02-1-0290 | 702.226 | OH | A02 | a1 | B04 | b2 | C07 | c1 | D01 | dl | E02 |
| 2-2-d1E02-1-0291 | 702.247 | OH | A01 | a1 | B02 | b1 | C16 | c2 | D02 | dl | E02 |
| 2-2-d1E02-1-0292 | 703.211 | OH | A02 | a1 | B03 | b1 | C04 | c2 | D02 | dl | E02 |
| 2-2-d1E02-1-0293 | 703.242 | OH | A01 | a1 | B02 | b1 | C17 | c2 | D02 | dl | E02 |
| 2-2-d1E02-1-0294 | 704.197 | OH | A02 | a1 | B02 | b1 | C05 | c2 | D02 | dl | E02 |
| 2-2-d1E02-1-0295 | 704.226 | OH | A02 | a1 | B01 | b1 | C14 | c2 | D02 | dl | E02 |
| 2-2-d1E02-1-0296 | 704.252 | OH | A01 | a1 | B02 | b4 | C01 | c2 | D02 | dl | E02 |
| 2-2-d1E02-1-0297 | 704.282 | OH | A01 | a1 | B05 | b4 | C02 | c1 | D01 | dl | E02 |
| 2-2-d1E02-1-0298 | 704.332 | OH | A02 | a1 | B01 | b1 | C19 | c1 | D01 | dl | E02 |
| 2-2-d1E02-1-0299 | 705.156 | OH | A01 | a1 | B04 | b1 | C01 | c2 | D02 | dl | E02 |
| 2-2-d1E02-1-0300 | 705.210 | OH | A02 | a1 | B02 | b3 | C08 | c2 | D02 | dl | E02 |
| 2-2-d1E02-1-0301 | 705.227 | OH | A01 | a1 | B03 | b1 | C15 | c2 | D02 | dl | E02 |
| 2-2-d1E02-1-0302 | 706.213 | OH | A01 | a1 | B01 | b4 | C05 | c2 | D02 | dl | E02 |
| 2-2-d1E02-1-0303 | 706.222 | OH | A01 | a1 | B03 | b1 | C16 | c2 | D02 | dl | E02 |
| 2-2-d1E02-1-0304 | 706.236 | OH | A02 | a1 | B05 | b3 | C09 | c1 | D01 | dl | E02 |
| 2-2-d1E02-1-0305 | 706.257 | OH | A02 | a1 | B04 | b1 | C15 | c1 | D01 | dl | E02 |
| 2-2-d1E02-1-0306 | 707.172 | OH | A02 | a1 | B01 | b3 | C10 | c2 | D02 | dl | E02 |
| 2-2-d1E02-1-0307 | 707.205 | OH | A01 | a1 | B04 | b3 | C11 | c1 | D01 | dl | E02 |
| 2-2-d1E02-1-0308 | 707.217 | OH | A01 | a1 | B03 | b1 | C17 | c2 | D02 | dl | E02 |
| 2-2-d1E02-1-0309 | 707.253 | OH | A02 | a1 | B04 | b1 | C16 | c1 | D01 | dl | E02 |
| 2-2-d1E02-1-0310 | 708.172 | OH | A02 | a1 | B03 | b1 | C05 | c2 | D02 | dl | E02 |
| 2-2-d1E02-1-0311 | 708.204 | OH | A01 | a1 | B02 | b1 | C18 | c2 | D02 | dl | E02 |
| 2-2-d1E02-1-0312 | 708.225 | OH | A02 | a1 | B01 | b2 | C02 | c2 | D02 | dl | E02 |
| 2-2-d1E02-1-0313 | 708.225 | OH | A02 | a1 | B01 | b2 | C03 | c2 | D02 | dl | E02 |
| 2-2-d1E02-1-0314 | 708.227 | OH | A01 | a1 | B03 | b4 | C01 | c2 | D02 | dl | E02 |
| 2-2-d1E02-1-0315 | 708.248 | OH | A02 | a1 | B04 | b1 | C17 | c1 | D01 | dl | E02 |
| 2-2-d1E02-1-0316 | 708.252 | OH | A02 | a1 | B05 | b1 | C14 | c1 | D01 | dl | E02 |
| 2-2-d1E02-1-0317 | 709.185 | OH | A02 | a1 | B03 | b3 | C08 | c2 | D02 | dl | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0318 | 709.221 | OH | A02 | a1 | B01 | b2 | C04 | c2 | D02 | dl | E02 |
| 2-2-d1E02-1-0319 | 709.221 | OH | A02 | a1 | B01 | b2 | C06 | c2 | D02 | dl | E02 |
| 2-2-d1E02-1-0320 | 709.221 | OH | A02 | a1 | B01 | b2 | C07 | c2 | D02 | dl | E02 |
| 2-2-d1E02-1-0321 | 709.224 | OH | A01 | a1 | B04 | b4 | C12 | c2 | D02 | dl | E02 |
| 2-2-d1E02-1-0322 | 709.257 | OH | A02 | a1 | B04 | b4 | C01 | c1 | D01 | dl | E02 |
| 2-2-d1E02-1-0323 | 710.239 | OH | A01 | a1 | B05 | b4 | C05 | c1 | D01 | dl | E02 |
| 2-2-d1E02-1-0324 | 711.197 | OH | A02 | a1 | B05 | b3 | C10 | c1 | D01 | dl | E02 |
| 2-2-d1E02-1-0325 | 711.203 | OH | A01 | a1 | B04 | b4 | C13 | c2 | D02 | dl | E02 |
| 2-2-d1E02-1-0326 | 712.179 | OH | A01 | a1 | B03 | b1 | C18 | c2 | D02 | dl | E02 |
| 2-2-d1E02-1-0327 | 712.251 | OH | A02 | a1 | B05 | b2 | C02 | c1 | D01 | dl | E02 |
| 2-2-d1E02-1-0328 | 712.251 | OH | A02 | a1 | B05 | b2 | C03 | c1 | D01 | dl | E02 |
| 2-2-d1E02-1-0329 | 713.209 | OH | A02 | a1 | B04 | b1 | C18 | c1 | D01 | dl | E02 |
| 2-2-d1E02-1-0330 | 713.246 | OH | A02 | a1 | B05 | b2 | C04 | c1 | D01 | dl | E02 |
| 2-2-d1E02-1-0331 | 713.246 | OH | A02 | a1 | B05 | b2 | C06 | c1 | D01 | dl | E02 |
| 2-2-d1E02-1-0332 | 713.246 | OH | A02 | a1 | B05 | b2 | C07 | c1 | D01 | dl | E02 |
| 2-2-d1E02-1-0333 | 713.252 | OH | A02 | a1 | B01 | b1 | C15 | c2 | D02 | dl | E02 |
| 2-2-d1E02-1-0334 | 714.200 | OH | A01 | a1 | B01 | b3 | C11 | c2 | D02 | dl | E02 |
| 2-2-d1E02-1-0335 | 714.247 | OH | A02 | a1 | B01 | b1 | C16 | c2 | D02 | dl | E02 |
| 2-2-d1E02-1-0336 | 714.272 | OH | A01 | a1 | B02 | b4 | C02 | c2 | D02 | dl | E02 |
| 2-2-d1E02-1-0337 | 715.177 | OH | A01 | a1 | B04 | b1 | C02 | c2 | D02 | dl | E02 |
| 2-2-d1E02-1-0338 | 715.177 | OH | A01 | a1 | B04 | b1 | C03 | c2 | D02 | dl | E02 |
| 2-2-d1E02-1-0339 | 715.242 | OH | A02 | a1 | B01 | b1 | C17 | c2 | D02 | dl | E02 |
| 2-2-d1E02-1-0340 | 716.172 | OH | A01 | a1 | B04 | b1 | C04 | c2 | D02 | dl | E02 |
| 2-2-d1E02-1-0341 | 716.176 | OH | A01 | a1 | B05 | b1 | C01 | c2 | D02 | dl | E02 |
| 2-2-d1E02-1-0342 | 716.226 | OH | A02 | a1 | B02 | b3 | C09 | c2 | D02 | dl | E02 |
| 2-2-d1E02-1-0343 | 716.252 | OH | A02 | a1 | B01 | b4 | C01 | c2 | D02 | dl | E02 |
| 2-2-d1E02-1-0344 | 717.277 | OH | A02 | a1 | B05 | b1 | C15 | c1 | D01 | dl | E02 |
| 2-2-d1E02-1-0345 | 718.225 | OH | A01 | a1 | B05 | b3 | C11 | c1 | D01 | dl | E02 |
| 2-2-d1E02-1-0346 | 718.242 | OH | A02 | a1 | B02 | b1 | C14 | c2 | D02 | dl | E02 |
| 2-2-d1E02-1-0347 | 718.247 | OH | A01 | a1 | B03 | b4 | C02 | c2 | D02 | dl | E02 |
| 2-2-d1E02-1-0348 | 718.273 | OH | A02 | a1 | B05 | b1 | C16 | c1 | D01 | dl | E02 |
| 2-2-d1E02-1-0349 | 718.348 | OH | A02 | a1 | B02 | b1 | C19 | c1 | D01 | dl | E02 |
| 2-2-d1E02-1-0350 | 719.268 | OH | A02 | a1 | B05 | b1 | C17 | c1 | D01 | dl | E02 |
| 2-2-d1E02-1-0351 | 719.278 | OH | A02 | a1 | B04 | b4 | C02 | c1 | D01 | dl | E02 |
| 2-2-d1E02-1-0352 | 720.201 | OH | A02 | a1 | B03 | b3 | C09 | c2 | D02 | dl | E02 |
| 2-2-d1E02-1-0353 | 720.204 | OH | A02 | a1 | B01 | b1 | C18 | c2 | D02 | dl | E02 |
| 2-2-d1E02-1-0354 | 720.229 | OH | A01 | a1 | B02 | b4 | C05 | c2 | D02 | dl | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0355 | 720.244 | OH | A01 | a1 | B05 | b4 | C12 | c2 | D02 | dl | E02 |
| 2-2-d1E02-1-0356 | 720.277 | OH | A02 | a1 | B05 | b4 | C01 | c1 | D01 | dl | E02 |
| 2-2-d1E02-1-0357 | 721.133 | OH | A01 | a1 | B04 | b1 | C05 | c2 | D02 | d1 | E02 |
| 2-2-d1E02-1-0358 | 721.188 | OH | A02 | a1 | B02 | b3 | C10 | c2 | D02 | d1 | E02 |
| 2-2-d1E02-1-0359 | 722.146 | OH | A01 | a1 | B04 | b3 | C08 | c2 | D02 | d1 | E02 |
| 2-2-d1E02-1-0360 | 722.217 | OH | A02 | a1 | B03 | b1 | C14 | c2 | D02 | d1 | E02 |
| 2-2-d1E02-1-0361 | 722.223 | OH | A01 | a1 | B05 | b4 | C13 | c2 | D02 | d1 | E02 |
| 2-2-d1E02-1-0362 | 722.241 | OH | A02 | a1 | B02 | b2 | C02 | c2 | D02 | d1 | E02 |
| 2-2-d1E02-1-0363 | 722.241 | OH | A02 | a1 | B02 | b2 | C03 | c2 | D02 | d1 | E02 |
| 2-2-d1E02-1-0364 | 722.323 | OH | A02 | a1 | B03 | b1 | C19 | c1 | D01 | d1 | E02 |
| 2-2-d1E02-1-0365 | 723.236 | OH | A02 | a1 | B02 | b2 | C04 | c2 | D02 | d1 | E02 |
| 2-2-d1E02-1-0366 | 723.236 | OH | A02 | a1 | B02 | b2 | C06 | c2 | D02 | d1 | E02 |
| 2-2-d1E02-1-0367 | 723.236 | OH | A02 | a1 | B02 | b2 | C07 | c2 | D02 | d1 | E02 |
| 2-2-d1E02-1-0368 | 724.204 | OH | A01 | a1 | B03 | b4 | C05 | c2 | D02 | d1 | E02 |
| 2-2-d1E02-1-0369 | 724.229 | OH | A02 | a1 | B05 | b1 | C18 | c1 | D01 | d1 | E02 |
| 2-2-d1E02-1-0370 | 725.163 | OH | A02 | a1 | B03 | b3 | C10 | c2 | D02 | d1 | E02 |
| 2-2-d1E02-1-0371 | 725.234 | OH | A02 | a1 | B04 | b4 | C05 | c1 | D01 | d1 | E02 |
| 2-2-d1E02-1-0372 | 726.197 | OH | A01 | a1 | B05 | b1 | C02 | c2 | D02 | d1 | E02 |
| 2-2-d1E02-1-0373 | 726.197 | OH | A01 | a1 | B05 | b1 | C03 | c2 | D02 | d1 | E02 |
| 2-2-d1E02-1-0374 | 726.216 | OH | A02 | a1 | B03 | b2 | C02 | c2 | D02 | d1 | E02 |
| 2-2-d1E02-1-0375 | 726.216 | OH | A02 | a1 | B03 | b2 | C03 | c2 | D02 | d1 | E02 |
| 2-2-d1E02-1-0376 | 726.272 | OH | A02 | a1 | B01 | b4 | C02 | c2 | D02 | d1 | E02 |
| 2-2-d1E02-1-0377 | 727.192 | OH | A01 | a1 | B05 | b1 | C04 | c2 | D02 | d1 | E02 |
| 2-2-d1E02-1-0378 | 727.211 | OH | A02 | a1 | B03 | b2 | C04 | c2 | D02 | d1 | E02 |
| 2-2-d1E02-1-0379 | 727.211 | OH | A02 | a1 | B03 | b2 | C06 | c2 | D02 | d1 | E02 |
| 2-2-d1E02-1-0380 | 727.211 | OH | A02 | a1 | B03 | b2 | C07 | c2 | D02 | d1 | E02 |
| 2-2-d1E02-1-0381 | 727.268 | OH | A02 | a1 | B02 | b1 | C15 | c2 | D02 | d1 | E02 |
| 2-2-d1E02-1-0382 | 728.215 | OH | A01 | a1 | B02 | b3 | C11 | c2 | D02 | d1 | E02 |
| 2-2-d1E02-1-0383 | 728.263 | OH | A02 | a1 | B02 | b1 | C16 | c2 | D02 | d1 | E02 |
| 2-2-d1E02-1-0384 | 729.258 | OH | A02 | a1 | B02 | b1 | C17 | c2 | D02 | d1 | E02 |
| 2-2-d1E02-1-0385 | 730.267 | OH | A02 | a1 | B02 | b4 | C01 | c2 | D02 | d1 | E02 |
| 2-2-d1E02-1-0386 | 730.298 | OH | A02 | a1 | B05 | b4 | C02 | c1 | D01 | d1 | E02 |
| 2-2-d1E02-1-0387 | 731.172 | OH | A02 | a1 | B04 | b1 | C01 | c2 | D02 | d1 | E02 |
| 2-2-d1E02-1-0388 | 731.243 | OH | A02 | a1 | B03 | b1 | C15 | c2 | D02 | d1 | E02 |
| 2-2-d1E02-1-0389 | 732.154 | OH | A01 | a1 | B05 | b1 | C05 | c2 | D02 | d1 | E02 |
| 2-2-d1E02-1-0390 | 732.190 | OH | A01 | a1 | B03 | b3 | C11 | c2 | D02 | d1 | E02 |
| 2-2-d1E02-1-0391 | 732.229 | OH | A02 | a1 | B01 | b4 | C05 | c2 | D02 | d1 | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0392 | 732.238 | OH | A02 | a1 | B03 | b1 | C16 | c2 | D02 | d1 | E02 |
| 2-2-d1E02-1-0393 | 733.162 | OH | A01 | a1 | B04 | b3 | C09 | c2 | D02 | d1 | E02 |
| 2-2-d1E02-1-0394 | 733.167 | OH | A01 | a1 | B05 | b3 | C08 | c2 | D02 | d1 | E02 |
| 2-2-d1E02-1-0395 | 733.221 | OH | A02 | a1 | B04 | b3 | C11 | c1 | D01 | d1 | E02 |
| 2-2-d1E02-1-0396 | 733.233 | OH | A02 | a1 | B03 | b1 | C17 | c2 | D02 | d1 | E02 |
| 2-2-d1E02-1-0397 | 734.219 | OH | A02 | a1 | B02 | b1 | C18 | c2 | D02 | d1 | E02 |
| 2-2-d1E02-1-0398 | 734.242 | OH | A02 | a1 | B03 | b4 | C01 | c2 | D02 | d1 | E02 |
| 2-2-d1E02-1-0399 | 735.178 | OH | A01 | a1 | B04 | b1 | C14 | c2 | D02 | d1 | E02 |
| 2-2-d1E02-1-0400 | 735.240 | OH | A02 | a1 | B04 | b4 | C12 | c2 | D02 | d1 | E02 |
| 2-2-d1E02-1-0401 | 735.284 | OH | A01 | a1 | B04 | b1 | C19 | c1 | D01 | d1 | E02 |
| 2-2-d1E02-1-0402 | 736.254 | OH | A02 | a1 | B05 | b4 | C05 | c1 | D01 | d1 | E02 |
| 2-2-d1E02-1-0403 | 737.219 | OH | A02 | a1 | B04 | b4 | C13 | c2 | D02 | d1 | E02 |
| 2-2-d1E02-1-0404 | 738.124 | OH | A01 | a1 | B04 | b3 | C10 | c2 | D02 | dl | E02 |
| 2-2-d1E02-1-0405 | 738.194 | OH | A02 | a1 | B03 | b1 | C18 | c2 | D02 | dl | E02 |
| 2-2-d1E02-1-0406 | 739.177 | OH | A01 | a1 | B04 | b2 | C02 | c2 | D02 | dl | E02 |
| 2-2-d1E02-1-0407 | 739.177 | OH | A01 | a1 | B04 | b2 | C03 | c2 | D02 | d1 | E02 |
| 2-2-d1E02-1-0408 | 740.172 | OH | A01 | a1 | B04 | b2 | C04 | c2 | D02 | d1 | E02 |
| 2-2-d1E02-1-0409 | 740.172 | OH | A01 | a1 | B04 | b2 | C06 | c2 | D02 | dl | E02 |
| 2-2-d1E02-1-0410 | 740.172 | OH | A01 | a1 | B04 | b2 | C07 | c2 | D02 | d1 | E02 |
| 2-2-d1E02-1-0411 | 740.215 | OH | A02 | a1 | B01 | b3 | C11 | c2 | D02 | dl | E02 |
| 2-2-d1E02-1-0412 | 740.288 | OH | A02 | a1 | B02 | b4 | C02 | c2 | D02 | d1 | E02 |
| 2-2-d1E02-1-0413 | 741.193 | OH | A02 | a1 | B04 | b1 | C02 | c2 | D02 | dl | E02 |
| 2-2-d1E02-1-0414 | 741.193 | OH | A02 | a1 | B04 | b1 | C03 | c2 | D02 | dl | E02 |
| 2-2-d1E02-1-0415 | 742.188 | OH | A02 | a1 | B04 | b1 | C04 | c2 | D02 | d1 | E02 |
| 2-2-d1E02-1-0416 | 742.192 | OH | A02 | a1 | B05 | b1 | C01 | c2 | D02 | dl | E02 |
| 2-2-d1E02-1-0417 | 742.278 | OH | A01 | a1 | B01 | b1 | C19 | c2 | D02 | d1 | E02 |
| 2-2-d1E02-1-0418 | 744.183 | OH | A01 | a1 | B05 | b3 | C09 | c2 | D02 | dl | E02 |
| 2-2-d1E02-1-0419 | 744.204 | OH | A01 | a1 | B04 | b1 | C15 | c2 | D02 | d1 | E02 |
| 2-2-d1E02-1-0420 | 744.241 | OH | A02 | a1 | B05 | b3 | C11 | c1 | D01 | d1 | E02 |
| 2-2-d1E02-1-0421 | 744.263 | OH | A02 | a1 | B03 | b4 | C02 | c2 | D02 | dl | E02 |
| 2-2-d1E02-1-0422 | 745.199 | OH | A01 | a1 | B04 | b1 | C16 | c2 | D02 | dl | E02 |
| 2-2-d1E02-1-0423 | 746.194 | OH | A01 | a1 | B04 | b1 | C17 | c2 | D02 | dl | E02 |
| 2-2-d1E02-1-0424 | 746.198 | OH | A01 | a1 | B05 | b1 | C14 | c2 | D02 | d1 | E02 |
| 2-2-d1E02-1-0425 | 746.244 | OH | A02 | a1 | B02 | b4 | COS | c2 | D02 | d1 | E02 |
| 2-2-d1E02-1-0426 | 746.260 | OH | A02 | a1 | B05 | b4 | C12 | c2 | D02 | d1 | E02 |
| 2-2-d1E02-1-0427 | 746.304 | OH | A01 | a1 | B05 | b1 | C19 | c1 | D01 | d1 | E02 |
| 2-2-d1E02-1-0428 | 747.149 | OH | A02 | a1 | B04 | b1 | COS | c2 | D02 | d1 | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0429 | 747.203 | OH | A01 | a1 | B04 | b4 | C01 | c2 | D02 | d1 | E02 |
| 2-2-d1E02-1-0430 | 748.162 | OH | A02 | a1 | B04 | b3 | C08 | c2 | D02 | d1 | E02 |
| 2-2-d1E02-1-0431 | 748.239 | OH | A02 | a1 | B05 | b4 | C13 | c2 | D02 | dl | E02 |
| 2-2-d1E02-1-0432 | 749.144 | OH | A01 | a1 | B05 | b3 | C10 | c2 | D02 | dl | E02 |
| 2-2-d1E02-1-0433 | 750.197 | OH | A01 | a1 | B05 | b2 | C02 | c2 | D02 | d1 | E02 |
| 2-2-d1E02-1-0434 | 750.197 | OH | A01 | a1 | B05 | b2 | C03 | c2 | D02 | d1 | E02 |
| 2-2-d1E02-1-0435 | 750.219 | OH | A02 | a1 | B03 | b4 | COS | c2 | D02 | d1 | E02 |
| 2-2-d1E02-1-0436 | 751.155 | OH | A01 | a1 | B04 | b1 | C18 | c2 | D02 | d1 | E02 |
| 2-2-d1E02-1-0437 | 751.192 | OH | A01 | a1 | B05 | b2 | C04 | c2 | D02 | d1 | E02 |
| 2-2-d1E02-1-0438 | 751.192 | OH | A01 | a1 | B05 | b2 | C06 | c2 | D02 | d1 | E02 |
| 2-2-d1E02-1-0439 | 751.192 | OH | A01 | a1 | B05 | b2 | C07 | c2 | D02 | d1 | E02 |
| 2-2-d1E02-1-0440 | 752.213 | OH | A02 | a1 | B05 | b1 | C02 | c2 | D02 | dl | E02 |
| 2-2-d1E02-1-0441 | 752.213 | OH | A02 | a1 | B05 | b1 | C03 | c2 | D02 | dl | E02 |
| 2-2-d1E02-1-0442 | 753.208 | OH | A02 | a1 | B05 | b1 | C04 | c2 | D02 | d1 | E02 |
| 2-2-d1E02-1-0443 | 754.231 | OH | A02 | a1 | B02 | b3 | C11 | c2 | D02 | d1 | E02 |
| 2-2-d1E02-1-0444 | 755.224 | OH | A01 | a1 | B05 | b1 | C15 | c2 | D02 | d1 | E02 |
| 2-2-d1E02-1-0445 | 756.219 | OH | A01 | a1 | B05 | b1 | C16 | c2 | D02 | d1 | E02 |
| 2-2-d1E02-1-0446 | 756.294 | OH | A01 | a1 | B02 | b1 | C19 | c2 | D02 | d1 | E02 |
| 2-2-d1E02-1-0447 | 757.214 | OH | A01 | a1 | B05 | b1 | C17 | c2 | D02 | d1 | E02 |
| 2-2-d1E02-1-0448 | 757.224 | OH | A01 | a1 | B04 | b4 | C02 | c2 | D02 | d1 | E02 |
| 2-2-d1E02-1-0449 | 758.169 | OH | A02 | a1 | B05 | b1 | C05 | c2 | D02 | dl | E02 |
| 2-2-d1E02-1-0450 | 758.206 | OH | A02 | a1 | B03 | b3 | C11 | c2 | D02 | dl | E02 |
| 2-2-d1E02-1-0451 | 758.223 | OH | A01 | a1 | B05 | b4 | C01 | c2 | D02 | dl | E02 |
| 2-2-d1E02-1-0452 | 759.178 | OH | A02 | a1 | B04 | b3 | C09 | c2 | D02 | dl | E02 |
| 2-2-d1E02-1-0453 | 759.182 | OH | A02 | a1 | B05 | b3 | C08 | c2 | D02 | dl | E02 |
| 2-2-d1E02-1-0454 | 760.269 | OH | A01 | a1 | B03 | b1 | C19 | c2 | D02 | d1 | E02 |
| 2-2-d1E02-1-0455 | 761.194 | OH | A02 | a1 | B04 | b1 | C14 | c2 | D02 | d1 | E02 |
| 2-2-d1E02-1-0456 | 761.300 | OH | A02 | a1 | B04 | b1 | C19 | c1 | D01 | dl | E02 |
| 2-2-d1E02-1-0457 | 762.175 | OH | A01 | a1 | B05 | b1 | C18 | c2 | D02 | d1 | E02 |
| 2-2-d1E02-1-0458 | 763.180 | OH | A01 | a1 | B04 | b4 | C05 | c2 | D02 | dl | E02 |
| 2-2-d1E02-1-0459 | 764.139 | OH | A02 | a1 | B04 | b3 | C10 | c2 | D02 | d1 | E02 |
| 2-2-d1E02-1-0460 | 765.193 | OH | A02 | a1 | B04 | b2 | C02 | c2 | D02 | dl | E02 |
| 2-2-d1E02-1-0461 | 765.193 | OH | A02 | a1 | B04 | b2 | C03 | c2 | D02 | dl | E02 |
| 2-2-d1E02-1-0462 | 766.188 | OH | A02 | a1 | B04 | b2 | C04 | c2 | D02 | d1 | E02 |
| 2-2-d1E02-1-0463 | 766.188 | OH | A02 | a1 | B04 | b2 | C06 | c2 | D02 | dl | E02 |
| 2-2-d1E02-1-0464 | 766.188 | OH | A02 | a1 | B04 | b2 | C07 | c2 | D02 | d1 | E02 |
| 2-2-d1E02-1-0465 | 768.244 | OH | A01 | a1 | B05 | b4 | C02 | c2 | D02 | dl | E02 |

(continued)

| [Table 9-5-3] Compound that may be contained in mixture 2-2-d1E02-1 | | | | | | | | | | | |
|---|---|---|---|---|---|---|---|---|---|---|---|
| ID | Exact Mass | | A | a | B | b | C | c | D | d | E |
| 2-2-d1E02-1-0466 | 768.294 | OH | A02 | a1 | B01 | b1 | C19 | c2 | D02 | d1 | E02 |
| 2-2-d1E02-1-0467 | 770.198 | OH | A02 | a1 | B05 | b3 | C09 | c2 | D02 | d1 | E02 |
| 2-2-d1E02-1-0468 | 770.219 | OH | A02 | a1 | B04 | b1 | C15 | c2 | D02 | dl | E02 |
| 2-2-d1E02-1-0469 | 771.167 | OH | A01 | a1 | B04 | b3 | C11 | c2 | D02 | dl | E02 |
| 2-2-d1E02-1-0470 | 771.215 | OH | A02 | a1 | B04 | b1 | C16 | c2 | D02 | dl | E02 |
| 2-2-d1E02-1-0471 | 772.210 | OH | A02 | a1 | B04 | b1 | C17 | c2 | D02 | d1 | E02 |
| 2-2-d1E02-1-0472 | 772.214 | OH | A02 | a1 | B05 | b1 | C14 | c2 | D02 | dl | E02 |
| 2-2-d1E02-1-0473 | 772.320 | OH | A02 | a1 | B05 | b1 | C19 | c1 | D01 | d1 | E02 |
| 2-2-d1E02-1-0474 | 773.219 | OH | A02 | a1 | B04 | b4 | C01 | c2 | D02 | d1 | E02 |
| 2-2-d1E02-1-0475 | 774.200 | OH | A01 | a1 | B05 | b4 | C05 | c2 | D02 | dl | E02 |
| 2-2-d1E02-1-0476 | 775.159 | OH | A02 | a1 | B05 | b3 | C10 | c2 | D02 | d1 | E02 |
| 2-2-d1E02-1-0477 | 776.213 | OH | A02 | a1 | B05 | b2 | C02 | c2 | D02 | dl | E02 |
| 2-2-d1E02-1-0478 | 776.213 | OH | A02 | a1 | B05 | b2 | C03 | c2 | D02 | dl | E02 |
| 2-2-d1E02-1-0479 | 777.171 | OH | A02 | a1 | B04 | b1 | C18 | c2 | D02 | dl | E02 |
| 2-2-d1E02-1-0480 | 777.208 | OH | A02 | a1 | B05 | b2 | C04 | c2 | D02 | dl | E02 |
| 2-2-d1E02-1-0481 | 777.208 | OH | A02 | a1 | B05 | b2 | C06 | c2 | D02 | d1 | E02 |
| 2-2-d1E02-1-0482 | 777.208 | OH | A02 | a1 | B05 | b2 | C07 | c2 | D02 | dl | E02 |
| 2-2-d1E02-1-0483 | 781.239 | OH | A02 | a1 | B05 | b1 | C15 | c2 | D02 | d1 | E02 |
| 2-2-d1E02-1-0484 | 782.187 | OH | A01 | a1 | B05 | b3 | C11 | c2 | D02 | d1 | E02 |
| 2-2-d1E02-1-0485 | 782.235 | OH | A02 | a1 | B05 | b1 | C16 | c2 | D02 | d1 | E02 |
| 2-2-d1E02-1-0486 | 782.310 | OH | A02 | a1 | B02 | b1 | C19 | c2 | D02 | d1 | E02 |
| 2-2-d1E02-1-0487 | 783.230 | OH | A02 | a1 | B05 | b1 | C17 | c2 | D02 | dl | E02 |
| 2-2-d1E02-1-0488 | 783.240 | OH | A02 | a1 | B04 | b4 | C02 | c2 | D02 | dl | E02 |
| 2-2-d1E02-1-0489 | 784.239 | OH | A02 | a1 | B05 | b4 | C01 | c2 | D02 | d1 | E02 |
| 2-2-d1E02-1-0490 | 786.285 | OH | A02 | a1 | B03 | b1 | C19 | c2 | D02 | d1 | E02 |
| 2-2-d1E02-1-0491 | 788.191 | OH | A02 | a1 | B05 | b1 | C18 | c2 | D02 | d1 | E02 |
| 2-2-d1E02-1-0492 | 789.196 | OH | A02 | a1 | B04 | b4 | COS | c2 | D02 | d1 | E02 |
| 2-2-d1E02-1-0493 | 794.260 | OH | A02 | a1 | B05 | b4 | C02 | c2 | D02 | d1 | E02 |
| 2-2-d1E02-1-0494 | 797.183 | OH | A02 | a1 | B04 | b3 | C11 | c2 | D02 | d1 | E02 |
| 2-2-d1E02-1-0495 | 799.246 | OH | A01 | a1 | B04 | b1 | C19 | c2 | D02 | d1 | E02 |
| 2-2-d1E02-1-0496 | 800.216 | OH | A02 | a1 | B05 | b4 | COS | c2 | D02 | dl | E02 |
| 2-2-d1E02-1-0497 | 808.203 | OH | A02 | a1 | B05 | b3 | C11 | c2 | D02 | dl | E02 |
| 2-2-d1E02-1-0498 | 810.266 | OH | A01 | a1 | B05 | b1 | C19 | c2 | D02 | d1 | E02 |
| 2-2-d1E02-1-0499 | 825.261 | OH | A02 | a1 | B04 | b1 | C19 | c2 | D02 | d1 | E02 |
| 2-2-d1E02-1-0500 | 836.282 | OH | A02 | a1 | B05 | b1 | C19 | c2 | D02 | d1 | E02 |

[Table 494]

[Table 9-5-4] Compound that may be contained in mixture 2-2-d1E03-1

| ID | Exact Mass | | A | a | B | b | C | c | D | d | E |
|---|---|---|---|---|---|---|---|---|---|---|---|
| 2-2-d1E03-1-0001 | 622.189 | OH | A01 | a1 | B01 | b1 | C01 | c1 | D01 | d1 | E03 |
| 2-2-d1E03-1-0002 | 626.256 | OH | A01 | a1 | B01 | b4 | C12 | c1 | D01 | d1 | E03 |
| 2-2-d1E03-1-0003 | 628.236 | OH | A01 | a1 | B01 | b4 | C13 | c1 | D01 | d1 | E03 |
| 2-2-d1E03-1-0004 | 632.209 | OH | A01 | a1 | B01 | b1 | C02 | c1 | D01 | d1 | E03 |
| 2-2-d1E03-1-0005 | 632.209 | OH | A01 | a1 | B01 | b1 | C03 | c1 | D01 | d1 | E03 |
| 2-2-d1E03-1-0006 | 633.205 | OH | A01 | a1 | B01 | b1 | C04 | c1 | D01 | d1 | E03 |
| 2-2-d1E03-1-0007 | 636.204 | OH | A01 | a1 | B02 | b1 | C01 | c1 | D01 | d1 | E03 |
| 2-2-d1E03-1-0008 | 638.166 | OH | A01 | a1 | B01 | b1 | COS | c1 | D01 | d1 | E03 |
| 2-2-d1E03-1-0009 | 639.179 | OH | A01 | a1 | B01 | b3 | C08 | c1 | D01 | d1 | E03 |
| 2-2-d1E03-1-0010 | 640.179 | OH | A01 | a1 | B03 | b1 | C01 | c1 | D01 | d1 | E03 |
| 2-2-d1E03-1-0011 | 640.272 | OH | A01 | a1 | B02 | b4 | C12 | c1 | D01 | d1 | E03 |
| 2-2-d1E03-1-0012 | 642.251 | OH | A01 | a1 | B02 | b4 | C13 | c1 | D01 | d1 | E03 |
| 2-2-d1E03-1-0013 | 644.247 | OH | A01 | a1 | B03 | b4 | C12 | c1 | D01 | d1 | E03 |
| 2-2-d1E03-1-0014 | 646.225 | OH | A01 | a1 | B02 | b1 | C02 | c1 | D01 | d1 | E03 |
| 2-2-d1E03-1-0015 | 646.225 | OH | A01 | a1 | B02 | b1 | C03 | c1 | D01 | d1 | E03 |
| 2-2-d1E03-1-0016 | 646.226 | OH | A01 | a1 | B03 | b4 | C13 | c1 | D01 | d1 | E03 |
| 2-2-d1E03-1-0017 | 647.220 | OH | A01 | a1 | B02 | b1 | C04 | c1 | D01 | d1 | E03 |
| 2-2-d1E03-1-0018 | 648.204 | OH | A02 | a1 | B01 | b1 | C01 | c1 | D01 | d1 | E03 |
| 2-2-d1E03-1-0019 | 650.195 | OH | A01 | a1 | B01 | b3 | C09 | c1 | D01 | d1 | E03 |
| 2-2-d1E03-1-0020 | 650.200 | OH | A01 | a1 | B03 | b1 | C02 | c1 | D01 | d1 | E03 |
| 2-2-d1E03-1-0021 | 650.200 | OH | A01 | a1 | B03 | b1 | C03 | c1 | D01 | d1 | E03 |
| 2-2-d1E03-1-0022 | 651.195 | OH | A01 | a1 | B03 | b1 | C04 | c1 | D01 | d1 | E03 |
| 2-2-d1E03-1-0023 | 652.181 | OH | A01 | a1 | B02 | b1 | C05 | c1 | D01 | d1 | E03 |
| 2-2-d1E03-1-0024 | 652.210 | OH | A01 | a1 | B01 | b1 | C14 | c1 | D01 | d1 | E03 |
| 2-2-d1E03-1-0025 | 652.272 | OH | A02 | a1 | B01 | b4 | C12 | c1 | D01 | d1 | E03 |
| 2-2-d1E03-1-0026 | 653.194 | OH | A01 | a1 | B02 | b3 | C08 | c1 | D01 | d1 | E03 |
| 2-2-d1E03-1-0027 | 654.251 | OH | A02 | a1 | B01 | b4 | C13 | c1 | D01 | d1 | E03 |
| 2-2-d1E03-1-0028 | 655.156 | OH | A01 | a1 | B01 | b3 | C10 | c1 | D01 | d1 | E03 |
| 2-2-d1E03-1-0029 | 656.156 | OH | A01 | a1 | B03 | b1 | C05 | c1 | D01 | d1 | E03 |
| 2-2-d1E03-1-0030 | 656.209 | OH | A01 | a1 | B01 | b2 | C02 | c1 | D01 | d1 | E03 |
| 2-2-d1E03-1-0031 | 656.209 | OH | A01 | a1 | B01 | b2 | C03 | c1 | D01 | d1 | E03 |
| 2-2-d1E03-1-0032 | 657.169 | OH | A01 | a1 | B03 | b3 | C08 | c1 | D01 | d1 | E03 |
| 2-2-d1E03-1-0033 | 657.205 | OH | A01 | a1 | B01 | b2 | C04 | c1 | D01 | d1 | E03 |
| 2-2-d1E03-1-0034 | 657.205 | OH | A01 | a1 | B01 | b2 | C06 | c1 | D01 | d1 | E03 |
| 2-2-d1E03-1-0035 | 657.205 | OH | A01 | a1 | B01 | b2 | C07 | c1 | D01 | d1 | E03 |
| 2-2-d1E03-1-0036 | 658.225 | OH | A02 | a1 | B01 | b1 | C02 | c1 | D01 | d1 | E03 |
| 2-2-d1E03-1-0037 | 658.225 | OH | A02 | a1 | B01 | b1 | C03 | c1 | D01 | d1 | E03 |
| 2-2-d1E03-1-0038 | 659.220 | OH | A02 | a1 | B01 | b1 | C04 | c1 | D01 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0039 | 661.236 | OH | A01 | a1 | B01 | b1 | C15 | c1 | D01 | d1 | E03 |
| 2-2-d1E03-1-0040 | 662.220 | OH | A02 | a1 | B02 | b1 | C01 | c1 | D01 | d1 | E03 |
| 2-2-d1E03-1-0041 | 662.231 | OH | A01 | a1 | B01 | b1 | C16 | c1 | D01 | d1 | E03 |
| 2-2-d1E03-1-0042 | 663.226 | OH | A01 | a1 | B01 | b1 | C17 | c1 | D01 | d1 | E03 |
| 2-2-d1E03-1-0043 | 664.181 | OH | A02 | a1 | B01 | b1 | C05 | c1 | D01 | d1 | E03 |
| 2-2-d1E03-1-0044 | 664.210 | OH | A01 | a1 | B02 | b3 | C09 | c1 | D01 | d1 | E03 |
| 2-2-d1E03-1-0045 | 664.236 | OH | A01 | a1 | B01 | b4 | C01 | c1 | D01 | d1 | E03 |
| 2-2-d1E03-1-0046 | 665.194 | OH | A02 | a1 | B01 | b3 | C08 | c1 | D01 | d1 | E03 |
| 2-2-d1E03-1-0047 | 666.195 | OH | A02 | a1 | B03 | b1 | C01 | c1 | D01 | d1 | E03 |
| 2-2-d1E03-1-0048 | 666.226 | OH | A01 | a1 | B02 | b1 | C14 | c1 | D01 | d1 | E03 |
| 2-2-d1E03-1-0049 | 666.288 | OH | A02 | a1 | B02 | b4 | C12 | c1 | D01 | d1 | E03 |
| 2-2-d1E03-1-0050 | 668.185 | OH | A01 | a1 | B03 | b3 | C09 | c1 | D01 | d1 | E03 |
| 2-2-d1E03-1-0051 | 668.188 | OH | A01 | a1 | B01 | b1 | C18 | c1 | D01 | d1 | E03 |
| 2-2-d1E03-1-0052 | 668.267 | OH | A02 | a1 | B02 | b4 | C13 | c1 | D01 | d1 | E03 |
| 2-2-d1E03-1-0053 | 669.172 | OH | A01 | a1 | B02 | b3 | C10 | c1 | D01 | d1 | E03 |
| 2-2-d1E03-1-0054 | 670.201 | OH | A01 | a1 | B03 | b1 | C14 | c1 | D01 | d1 | E03 |
| 2-2-d1E03-1-0055 | 670.225 | OH | A01 | a1 | B02 | b2 | C02 | c1 | D01 | d1 | E03 |
| 2-2-d1E03-1-0056 | 670.225 | OH | A01 | a1 | B02 | b2 | C03 | c1 | D01 | d1 | E03 |
| 2-2-d1E03-1-0057 | 670.263 | OH | A02 | a1 | B03 | b4 | C12 | c1 | D01 | d1 | E03 |
| 2-2-d1E03-1-0058 | 671.220 | OH | A01 | a1 | B02 | b2 | C04 | c1 | D01 | d1 | E03 |
| 2-2-d1E03-1-0059 | 671.220 | OH | A01 | a1 | B02 | b2 | C06 | c1 | D01 | d1 | E03 |
| 2-2-d1E03-1-0060 | 671.220 | OH | A01 | a1 | B02 | b2 | C07 | c1 | D01 | d1 | E03 |
| 2-2-d1E03-1-0061 | 672.241 | OH | A02 | a1 | B02 | b1 | C02 | c1 | D01 | d1 | E03 |
| 2-2-d1E03-1-0062 | 672.241 | OH | A02 | a1 | B02 | b1 | C03 | c1 | D01 | d1 | E03 |
| 2-2-d1E03-1-0063 | 672.242 | OH | A02 | a1 | B03 | b4 | C13 | c1 | D01 | d1 | E03 |
| 2-2-d1E03-1-0064 | 673.147 | OH | A01 | a1 | B03 | b3 | C10 | c1 | D01 | d1 | E03 |
| 2-2-d1E03-1-0065 | 673.236 | OH | A02 | a1 | B02 | b1 | C04 | c1 | D01 | d1 | E03 |
| 2-2-d1E03-1-0066 | 674.200 | OH | A01 | a1 | B03 | b2 | C02 | c1 | D01 | d1 | E03 |
| 2-2-d1E03-1-0067 | 674.200 | OH | A01 | a1 | B03 | b2 | C03 | c1 | D01 | d1 | E03 |
| 2-2-d1E03-1-0068 | 674.256 | OH | A01 | a1 | B01 | b4 | C02 | c1 | D01 | d1 | E03 |
| 2-2-d1E03-1-0069 | 675.195 | OH | A01 | a1 | B03 | b2 | C04 | c1 | D01 | d1 | E03 |
| 2-2-d1E03-1-0070 | 675.195 | OH | A01 | a1 | B03 | b2 | C06 | c1 | D01 | d1 | E03 |
| 2-2-d1E03-1-0071 | 675.195 | OH | A01 | a1 | B03 | b2 | C07 | c1 | D01 | d1 | E03 |
| 2-2-d1E03-1-0072 | 675.252 | OH | A01 | a1 | B02 | b1 | C15 | c1 | D01 | d1 | E03 |
| 2-2-d1E03-1-0073 | 676.210 | OH | A02 | a1 | B01 | b3 | C09 | c1 | D01 | d1 | E03 |
| 2-2-d1E03-1-0074 | 676.216 | OH | A02 | a1 | B03 | b1 | C02 | c1 | D01 | d1 | E03 |
| 2-2-d1E03-1-0075 | 676.216 | OH | A02 | a1 | B03 | b1 | C03 | c1 | D01 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0076 | 676.247 | OH | A01 | a1 | B02 | b1 | C16 | c1 | D01 | d1 | E03 |
| 2-2-d1E03-1-0077 | 677.211 | OH | A02 | a1 | B03 | b1 | C04 | c1 | D01 | d1 | E03 |
| 2-2-d1E03-1-0078 | 677.242 | OH | A01 | a1 | B02 | b1 | C17 | c1 | D01 | d1 | E03 |
| 2-2-d1E03-1-0079 | 678.197 | OH | A02 | a1 | B02 | b1 | C05 | c1 | D01 | d1 | E03 |
| 2-2-d1E03-1-0080 | 678.226 | OH | A02 | a1 | B01 | b1 | C14 | c1 | D01 | d1 | E03 |
| 2-2-d1E03-1-0081 | 678.251 | OH | A01 | a1 | B02 | b4 | C01 | c1 | D01 | d1 | E03 |
| 2-2-d1E03-1-0082 | 679.156 | OH | A01 | a1 | B04 | b1 | C01 | c1 | D01 | d1 | E03 |
| 2-2-d1E03-1-0083 | 679.210 | OH | A02 | a1 | B02 | b3 | C08 | c1 | D01 | d1 | E03 |
| 2-2-d1E03-1-0084 | 679.226 | OH | A01 | a1 | B03 | b1 | C15 | c1 | D01 | d1 | E03 |
| 2-2-d1E03-1-0085 | 680.213 | OH | A01 | a1 | B01 | b4 | C05 | c1 | D01 | d1 | E03 |
| 2-2-d1E03-1-0086 | 680.222 | OH | A01 | a1 | B03 | b1 | C16 | c1 | D01 | d1 | E03 |
| 2-2-d1E03-1-0087 | 681.172 | OH | A02 | a1 | B01 | b3 | C10 | c1 | D01 | d1 | E03 |
| 2-2-d1E03-1-0088 | 681.217 | OH | A01 | a1 | B03 | b1 | C17 | c1 | D01 | d1 | E03 |
| 2-2-d1E03-1-0089 | 682.172 | OH | A02 | a1 | B03 | b1 | C05 | c1 | D01 | d1 | E03 |
| 2-2-d1E03-1-0090 | 682.203 | OH | A01 | a1 | B02 | b1 | C18 | c1 | D01 | d1 | E03 |
| 2-2-d1E03-1-0091 | 682.225 | OH | A02 | a1 | B01 | b2 | C02 | c1 | D01 | d1 | E03 |
| 2-2-d1E03-1-0092 | 682.225 | OH | A02 | a1 | B01 | b2 | C03 | c1 | D01 | d1 | E03 |
| 2-2-d1E03-1-0093 | 682.226 | OH | A01 | a1 | B03 | b4 | C01 | c1 | D01 | d1 | E03 |
| 2-2-d1E03-1-0094 | 683.185 | OH | A02 | a1 | B03 | b3 | C08 | c1 | D01 | d1 | E03 |
| 2-2-d1E03-1-0095 | 683.220 | OH | A02 | a1 | B01 | b2 | C04 | c1 | D01 | d1 | E03 |
| 2-2-d1E03-1-0096 | 683.220 | OH | A02 | a1 | B01 | b2 | C06 | c1 | D01 | d1 | E03 |
| 2-2-d1E03-1-0097 | 683.220 | OH | A02 | a1 | B01 | b2 | C07 | c1 | D01 | d1 | E03 |
| 2-2-d1E03-1-0098 | 683.224 | OH | A01 | a1 | B04 | b4 | C12 | c1 | D01 | d1 | E03 |
| 2-2-d1E03-1-0099 | 685.203 | OH | A01 | a1 | B04 | b4 | C13 | c1 | D01 | d1 | E03 |
| 2-2-d1E03-1-0100 | 686.151 | OH | A01 | a1 | B01 | b1 | C01 | c2 | D02 | d1 | E03 |
| 2-2-d1E03-1-0101 | 686.178 | OH | A01 | a1 | B03 | b1 | C18 | c1 | D01 | d1 | E03 |
| 2-2-d1E03-1-0102 | 687.252 | OH | A02 | a1 | B01 | b1 | C15 | c1 | D01 | d1 | E03 |
| 2-2-d1E03-1-0103 | 688.199 | OH | A01 | a1 | B01 | b3 | C11 | c1 | D01 | d1 | E03 |
| 2-2-d1E03-1-0104 | 688.247 | OH | A02 | a1 | B01 | b1 | C16 | c1 | D01 | d1 | E03 |
| 2-2-d1E03-1-0105 | 688.272 | OH | A01 | a1 | B02 | b4 | C02 | c1 | D01 | d1 | E03 |
| 2-2-d1E03-1-0106 | 689.177 | OH | A01 | a1 | B04 | b1 | C02 | c1 | D01 | d1 | E03 |
| 2-2-d1E03-1-0107 | 689.177 | OH | A01 | a1 | B04 | b1 | C03 | c1 | D01 | d1 | E03 |
| 2-2-d1E03-1-0108 | 689.242 | OH | A02 | a1 | B01 | b1 | C17 | c1 | D01 | d1 | E03 |
| 2-2-d1E03-1-0109 | 690.172 | OH | A01 | a1 | B04 | b1 | C04 | c1 | D01 | d1 | E03 |
| 2-2-d1E03-1-0110 | 690.176 | OH | A01 | a1 | B05 | b1 | C01 | c1 | D01 | d1 | E03 |
| 2-2-d1E03-1-0111 | 690.218 | OH | A01 | a1 | B01 | b4 | C12 | c2 | D02 | d1 | E03 |
| 2-2-d1E03-1-0112 | 690.226 | OH | A02 | a1 | B02 | b3 | C09 | c1 | D01 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0113 | 690.251 | OH | A02 | a1 | B01 | b4 | C01 | c1 | D01 | d1 | E03 |
| 2-2-d1E03-1-0114 | 692.197 | OH | A01 | a1 | B01 | b4 | C13 | c2 | D02 | d1 | E03 |
| 2-2-d1E03-1-0115 | 692.242 | OH | A02 | a1 | B02 | b1 | C14 | c1 | D01 | d1 | E03 |
| 2-2-d1E03-1-0116 | 692.247 | OH | A01 | a1 | B03 | b4 | C02 | c1 | D01 | d1 | E03 |
| 2-2-d1E03-1-0117 | 694.201 | OH | A02 | a1 | B03 | b3 | C09 | c1 | D01 | d1 | E03 |
| 2-2-d1E03-1-0118 | 694.203 | OH | A02 | a1 | B01 | b1 | C18 | c1 | D01 | d1 | E03 |
| 2-2-d1E03-1-0119 | 694.228 | OH | A01 | a1 | B02 | b4 | C05 | c1 | D01 | d1 | E03 |
| 2-2-d1E03-1-0120 | 694.244 | OH | A01 | a1 | B05 | b4 | C12 | c1 | D01 | d1 | E03 |
| 2-2-d1E03-1-0121 | 695.133 | OH | A01 | a1 | B04 | b1 | C05 | c1 | D01 | d1 | E03 |
| 2-2-d1E03-1-0122 | 695.187 | OH | A02 | a1 | B02 | b3 | C10 | c1 | D01 | d1 | E03 |
| 2-2-d1E03-1-0123 | 696.146 | OH | A01 | a1 | B04 | b3 | C08 | c1 | D01 | d1 | E03 |
| 2-2-d1E03-1-0124 | 696.171 | OH | A01 | a1 | B01 | b1 | C02 | c2 | D02 | d1 | E03 |
| 2-2-d1E03-1-0125 | 696.171 | OH | A01 | a1 | B01 | b1 | C03 | c2 | D02 | d1 | E03 |
| 2-2-d1E03-1-0126 | 696.217 | OH | A02 | a1 | B03 | b1 | C14 | c1 | D01 | d1 | E03 |
| 2-2-d1E03-1-0127 | 696.223 | OH | A01 | a1 | B05 | b4 | C13 | c1 | D01 | d1 | E03 |
| 2-2-d1E03-1-0128 | 696.241 | OH | A02 | a1 | B02 | b2 | C02 | c1 | D01 | d1 | E03 |
| 2-2-d1E03-1-0129 | 696.241 | OH | A02 | a1 | B02 | b2 | C03 | c1 | D01 | d1 | E03 |
| 2-2-d1E03-1-0130 | 697.166 | OH | A01 | a1 | B01 | b1 | C04 | c2 | D02 | d1 | E03 |
| 2-2-d1E03-1-0131 | 697.236 | OH | A02 | a1 | B02 | b2 | C04 | c1 | D01 | d1 | E03 |
| 2-2-d1E03-1-0132 | 697.236 | OH | A02 | a1 | B02 | b2 | C06 | c1 | D01 | d1 | E03 |
| 2-2-d1E03-1-0133 | 697.236 | OH | A02 | a1 | B02 | b2 | C07 | c1 | D01 | d1 | E03 |
| 2-2-d1E03-1-0134 | 698.203 | OH | A01 | a1 | B03 | b4 | C05 | c1 | D01 | d1 | E03 |
| 2-2-d1E03-1-0135 | 699.162 | OH | A02 | a1 | B03 | b3 | C10 | c1 | D01 | d1 | E03 |
| 2-2-d1E03-1-0136 | 700.166 | OH | A01 | a1 | B02 | b1 | C01 | c2 | D02 | d1 | E03 |
| 2-2-d1E03-1-0137 | 700.197 | OH | A01 | a1 | B05 | b1 | C02 | c1 | D01 | d1 | E03 |
| 2-2-d1E03-1-0138 | 700.197 | OH | A01 | a1 | B05 | b1 | C03 | c1 | D01 | d1 | E03 |
| 2-2-d1E03-1-0139 | 700.216 | OH | A02 | a1 | B03 | b2 | C02 | c1 | D01 | d1 | E03 |
| 2-2-d1E03-1-0140 | 700.216 | OH | A02 | a1 | B03 | b2 | C03 | c1 | D01 | d1 | E03 |
| 2-2-d1E03-1-0141 | 700.272 | OH | A02 | a1 | B01 | b4 | C02 | c1 | D01 | d1 | E03 |
| 2-2-d1E03-1-0142 | 701.192 | OH | A01 | a1 | B05 | b1 | C04 | c1 | D01 | d1 | E03 |
| 2-2-d1E03-1-0143 | 701.211 | OH | A02 | a1 | B03 | b2 | C04 | c1 | D01 | d1 | E03 |
| 2-2-d1E03-1-0144 | 701.211 | OH | A02 | a1 | B03 | b2 | C06 | c1 | D01 | d1 | E03 |
| 2-2-d1E03-1-0145 | 701.211 | OH | A02 | a1 | B03 | b2 | C07 | c1 | D01 | d1 | E03 |
| 2-2-d1E03-1-0146 | 701.267 | OH | A02 | a1 | B02 | b1 | C15 | c1 | D01 | d1 | E03 |
| 2-2-d1E03-1-0147 | 702.128 | OH | A01 | a1 | B01 | b1 | C05 | c2 | D02 | d1 | E03 |
| 2-2-d1E03-1-0148 | 702.215 | OH | A01 | a1 | B02 | b3 | C11 | c1 | D01 | d1 | E03 |
| 2-2-d1E03-1-0149 | 702.262 | OH | A02 | a1 | B02 | b1 | C16 | c1 | D01 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0150 | 703.141 | OH | A01 | a1 | B01 | b3 | C08 | c2 | D02 | d1 | E03 |
| 2-2-d1E03-1-0151 | 703.258 | OH | A02 | a1 | B02 | b1 | C17 | c1 | D01 | d1 | E03 |
| 2-2-d1E03-1-0152 | 704.141 | OH | A01 | a1 | B03 | b1 | C01 | c2 | D02 | d1 | E03 |
| 2-2-d1E03-1-0153 | 704.234 | OH | A01 | a1 | B02 | b4 | C12 | c2 | D02 | d1 | E03 |
| 2-2-d1E03-1-0154 | 704.267 | OH | A02 | a1 | B02 | b4 | C01 | c1 | D01 | d1 | E03 |
| 2-2-d1E03-1-0155 | 705.172 | OH | A02 | a1 | B04 | b1 | C01 | c1 | D01 | d1 | E03 |
| 2-2-d1E03-1-0156 | 705.242 | OH | A02 | a1 | B03 | b1 | C15 | c1 | D01 | d1 | E03 |
| 2-2-d1E03-1-0157 | 706.153 | OH | A01 | a1 | B05 | b1 | C05 | c1 | D01 | d1 | E03 |
| 2-2-d1E03-1-0158 | 706.190 | OH | A01 | a1 | B03 | b3 | C11 | c1 | D01 | d1 | E03 |
| 2-2-d1E03-1-0159 | 706.213 | OH | A01 | a1 | B02 | b4 | C13 | c2 | D02 | d1 | E03 |
| 2-2-d1E03-1-0160 | 706.228 | OH | A02 | a1 | B01 | b4 | C05 | c1 | D01 | d1 | E03 |
| 2-2-d1E03-1-0161 | 706.237 | OH | A02 | a1 | B03 | b1 | C16 | c1 | D01 | d1 | E03 |
| 2-2-d1E03-1-0162 | 707.162 | OH | A01 | a1 | B04 | b3 | C09 | c1 | D01 | d1 | E03 |
| 2-2-d1E03-1-0163 | 707.166 | OH | A01 | a1 | B05 | b3 | C08 | c1 | D01 | d1 | E03 |
| 2-2-d1E03-1-0164 | 707.233 | OH | A02 | a1 | B03 | b1 | C17 | c1 | D01 | d1 | E03 |
| 2-2-d1E03-1-0165 | 708.209 | OH | A01 | a1 | B03 | b4 | C12 | c2 | D02 | d1 | E03 |
| 2-2-d1E03-1-0166 | 708.219 | OH | A02 | a1 | B02 | b1 | C18 | c1 | D01 | d1 | E03 |
| 2-2-d1E03-1-0167 | 708.242 | OH | A02 | a1 | B03 | b4 | C01 | c1 | D01 | d1 | E03 |
| 2-2-d1E03-1-0168 | 709.178 | OH | A01 | a1 | B04 | b1 | C14 | c1 | D01 | d1 | E03 |
| 2-2-d1E03-1-0169 | 709.239 | OH | A02 | a1 | B04 | b4 | C12 | c1 | D01 | d1 | E03 |
| 2-2-d1E03-1-0170 | 710.187 | OH | A01 | a1 | B02 | b1 | C02 | c2 | D02 | d1 | E03 |
| 2-2-d1E03-1-0171 | 710.187 | OH | A01 | a1 | B02 | b1 | C03 | c2 | D02 | d1 | E03 |
| 2-2-d1E03-1-0172 | 710.188 | OH | A01 | a1 | B03 | b4 | C13 | c2 | D02 | d1 | E03 |
| 2-2-d1E03-1-0173 | 711.182 | OH | A01 | a1 | B02 | b1 | C04 | c2 | D02 | d1 | E03 |
| 2-2-d1E03-1-0174 | 711.219 | OH | A02 | a1 | B04 | b4 | C13 | c1 | D01 | d1 | E03 |
| 2-2-d1E03-1-0175 | 712.123 | OH | A01 | a1 | B04 | b3 | C10 | c1 | D01 | d1 | E03 |
| 2-2-d1E03-1-0176 | 712.166 | OH | A02 | a1 | B01 | b1 | C01 | c2 | D02 | d1 | E03 |
| 2-2-d1E03-1-0177 | 712.194 | OH | A02 | a1 | B03 | b1 | C18 | c1 | D01 | d1 | E03 |
| 2-2-d1E03-1-0178 | 713.177 | OH | A01 | a1 | B04 | b2 | C02 | c1 | D01 | d1 | E03 |
| 2-2-d1E03-1-0179 | 713.177 | OH | A01 | a1 | B04 | b2 | C03 | c1 | D01 | d1 | E03 |
| 2-2-d1E03-1-0180 | 714.157 | OH | A01 | a1 | B01 | b3 | C09 | c2 | D02 | d1 | E03 |
| 2-2-d1E03-1-0181 | 714.162 | OH | A01 | a1 | B03 | b1 | C02 | c2 | D02 | d1 | E03 |
| 2-2-d1E03-1-0182 | 714.162 | OH | A01 | a1 | B03 | b1 | C03 | c2 | D02 | d1 | E03 |
| 2-2-d1E03-1-0183 | 714.172 | OH | A01 | a1 | B04 | b2 | C04 | c1 | D01 | d1 | E03 |
| 2-2-d1E03-1-0184 | 714.172 | OH | A01 | a1 | B04 | b2 | C06 | c1 | D01 | d1 | E03 |
| 2-2-d1E03-1-0185 | 714.172 | OH | A01 | a1 | B04 | b2 | C07 | c1 | D01 | d1 | E03 |
| 2-2-d1E03-1-0186 | 714.215 | OH | A02 | a1 | B01 | b3 | C11 | c1 | D01 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0187 | 714.288 | OH | A02 | a1 | B02 | b4 | C02 | c1 | D01 | d1 | E03 |
| 2-2-d1E03-1-0188 | 715.157 | OH | A01 | a1 | B03 | b1 | C04 | c2 | D02 | d1 | E03 |
| 2-2-d1E03-1-0189 | 715.192 | OH | A02 | a1 | B04 | b1 | C02 | c1 | D01 | d1 | E03 |
| 2-2-d1E03-1-0190 | 715.192 | OH | A02 | a1 | B04 | b1 | C03 | c1 | D01 | d1 | E03 |
| 2-2-d1E03-1-0191 | 716.143 | OH | A01 | a1 | B02 | b1 | C05 | c2 | D02 | d1 | E03 |
| 2-2-d1E03-1-0192 | 716.172 | OH | A01 | a1 | B01 | b1 | C14 | c2 | D02 | d1 | E03 |
| 2-2-d1E03-1-0193 | 716.188 | OH | A02 | a1 | B04 | b1 | C04 | c1 | D01 | d1 | E03 |
| 2-2-d1E03-1-0194 | 716.192 | OH | A02 | a1 | B05 | b1 | C01 | c1 | D01 | d1 | E03 |
| 2-2-d1E03-1-0195 | 716.234 | OH | A02 | a1 | B01 | b4 | C12 | c2 | D02 | d1 | E03 |
| 2-2-d1E03-1-0196 | 716.278 | OH | A01 | a1 | B01 | b1 | C19 | c1 | D01 | d1 | E03 |
| 2-2-d1E03-1-0197 | 717.156 | OH | A01 | a1 | B02 | b3 | C08 | c2 | D02 | d1 | E03 |
| 2-2-d1E03-1-0198 | 718.182 | OH | A01 | a1 | B05 | b3 | C09 | c1 | D01 | d1 | E03 |
| 2-2-d1E03-1-0199 | 718.203 | OH | A01 | a1 | B04 | b1 | C15 | c1 | D01 | d1 | E03 |
| 2-2-d1E03-1-0200 | 718.213 | OH | A02 | a1 | B01 | b4 | C13 | c2 | D02 | d1 | E03 |
| 2-2-d1E03-1-0201 | 718.263 | OH | A02 | a1 | B03 | b4 | C02 | c1 | D01 | d1 | E03 |
| 2-2-d1E03-1-0202 | 719.118 | OH | A01 | a1 | B01 | b3 | C10 | c2 | D02 | d1 | E03 |
| 2-2-d1E03-1-0203 | 719.198 | OH | A01 | a1 | B04 | b1 | C16 | c1 | D01 | d1 | E03 |
| 2-2-d1E03-1-0204 | 720.118 | OH | A01 | a1 | B03 | b1 | C05 | c2 | D02 | d1 | E03 |
| 2-2-d1E03-1-0205 | 720.171 | OH | A01 | a1 | B01 | b2 | C02 | c2 | D02 | d1 | E03 |
| 2-2-d1E03-1-0206 | 720.171 | OH | A01 | a1 | B01 | b2 | C03 | c2 | D02 | d1 | E03 |
| 2-2-d1E03-1-0207 | 720.194 | OH | A01 | a1 | B04 | b1 | C17 | c1 | D01 | d1 | E03 |
| 2-2-d1E03-1-0208 | 720.198 | OH | A01 | a1 | B05 | b1 | C14 | c1 | D01 | d1 | E03 |
| 2-2-d1E03-1-0209 | 720.244 | OH | A02 | a1 | B02 | b4 | C05 | c1 | D01 | d1 | E03 |
| 2-2-d1E03-1-0210 | 720.259 | OH | A02 | a1 | B05 | b4 | C12 | c1 | D01 | d1 | E03 |
| 2-2-d1E03-1-0211 | 721.131 | OH | A01 | a1 | B03 | b3 | C08 | c2 | D02 | d1 | E03 |
| 2-2-d1E03-1-0212 | 721.149 | OH | A02 | a1 | B04 | b1 | C05 | c1 | D01 | d1 | E03 |
| 2-2-d1E03-1-0213 | 721.166 | OH | A01 | a1 | B01 | b2 | C04 | c2 | D02 | d1 | E03 |
| 2-2-d1E03-1-0214 | 721.166 | OH | A01 | a1 | B01 | b2 | C06 | c2 | D02 | d1 | E03 |
| 2-2-d1E03-1-0215 | 721.166 | OH | A01 | a1 | B01 | b2 | C07 | c2 | D02 | d1 | E03 |
| 2-2-d1E03-1-0216 | 721.203 | OH | A01 | a1 | B04 | b4 | C01 | c1 | D01 | d1 | E03 |
| 2-2-d1E03-1-0217 | 722.162 | OH | A02 | a1 | B04 | b3 | C08 | c1 | D01 | d1 | E03 |
| 2-2-d1E03-1-0218 | 722.187 | OH | A02 | a1 | B01 | b1 | C02 | c2 | D02 | d1 | E03 |
| 2-2-d1E03-1-0219 | 722.187 | OH | A02 | a1 | B01 | b1 | C03 | c2 | D02 | d1 | E03 |
| 2-2-d1E03-1-0220 | 722.239 | OH | A02 | a1 | B05 | b4 | C13 | c1 | D01 | d1 | E03 |
| 2-2-d1E03-1-0221 | 723.143 | OH | A01 | a1 | B05 | b3 | C10 | c1 | D01 | d1 | E03 |
| 2-2-d1E03-1-0222 | 723.182 | OH | A02 | a1 | B01 | b1 | C04 | c2 | D02 | d1 | E03 |
| 2-2-d1E03-1-0223 | 724.197 | OH | A01 | a1 | B05 | b2 | C02 | c1 | D01 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0224 | 724.197 | OH | A01 | a1 | B05 | b2 | C03 | c1 | D01 | d1 | E03 |
| 2-2-d1E03-1-0225 | 724.219 | OH | A02 | a1 | B03 | b4 | C05 | c1 | D01 | d1 | E03 |
| 2-2-d1E03-1-0226 | 725.155 | OH | A01 | a1 | B04 | b1 | C18 | c1 | D01 | d1 | E03 |
| 2-2-d1E03-1-0227 | 725.192 | OH | A01 | a1 | B05 | b2 | C04 | c1 | D01 | d1 | E03 |
| 2-2-d1E03-1-0228 | 725.192 | OH | A01 | a1 | B05 | b2 | C06 | c1 | D01 | d1 | E03 |
| 2-2-d1E03-1-0229 | 725.192 | OH | A01 | a1 | B05 | b2 | C07 | c1 | D01 | d1 | E03 |
| 2-2-d1E03-1-0230 | 725.198 | OH | A01 | a1 | B01 | b1 | C15 | c2 | D02 | d1 | E03 |
| 2-2-d1E03-1-0231 | 726.182 | OH | A02 | a1 | B02 | b1 | C01 | c2 | D02 | d1 | E03 |
| 2-2-d1E03-1-0232 | 726.193 | OH | A01 | a1 | B01 | b1 | C16 | c2 | D02 | d1 | E03 |
| 2-2-d1E03-1-0233 | 726.212 | OH | A02 | a1 | B05 | b1 | C02 | c1 | D01 | d1 | E03 |
| 2-2-d1E03-1-0234 | 726.212 | OH | A02 | a1 | B05 | b1 | C03 | c1 | D01 | d1 | E03 |
| 2-2-d1E03-1-0235 | 727.188 | OH | A01 | a1 | B01 | b1 | C17 | c2 | D02 | d1 | E03 |
| 2-2-d1E03-1-0236 | 727.208 | OH | A02 | a1 | B05 | b1 | C04 | c1 | D01 | d1 | E03 |
| 2-2-d1E03-1-0237 | 728.143 | OH | A02 | a1 | B01 | b1 | C05 | c2 | D02 | d1 | E03 |
| 2-2-d1E03-1-0238 | 728.172 | OH | A01 | a1 | B02 | b3 | C09 | c2 | D02 | d1 | E03 |
| 2-2-d1E03-1-0239 | 728.197 | OH | A01 | a1 | B01 | b4 | C01 | c2 | D02 | d1 | E03 |
| 2-2-d1E03-1-0240 | 728.230 | OH | A02 | a1 | B02 | b3 | C11 | c1 | D01 | d1 | E03 |
| 2-2-d1E03-1-0241 | 729.156 | OH | A02 | a1 | B01 | b3 | C08 | c2 | D02 | d1 | E03 |
| 2-2-d1E03-1-0242 | 729.223 | OH | A01 | a1 | B05 | b1 | C15 | c1 | D01 | d1 | E03 |
| 2-2-d1E03-1-0243 | 730.157 | OH | A02 | a1 | B03 | b1 | C01 | c2 | D02 | d1 | E03 |
| 2-2-d1E03-1-0244 | 730.188 | OH | A01 | a1 | B02 | b1 | C14 | c2 | D02 | d1 | E03 |
| 2-2-d1E03-1-0245 | 730.219 | OH | A01 | a1 | B05 | b1 | C16 | c1 | D01 | d1 | E03 |
| 2-2-d1E03-1-0246 | 730.249 | OH | A02 | a1 | B02 | b4 | C12 | c2 | D02 | d1 | E03 |
| 2-2-d1E03-1-0247 | 730.294 | OH | A01 | a1 | B02 | b1 | C19 | c1 | D01 | d1 | E03 |
| 2-2-d1E03-1-0248 | 731.214 | OH | A01 | a1 | B05 | b1 | C17 | c1 | D01 | d1 | E03 |
| 2-2-d1E03-1-0249 | 731.224 | OH | A01 | a1 | B04 | b4 | C02 | c1 | D01 | d1 | E03 |
| 2-2-d1E03-1-0250 | 732.147 | OH | A01 | a1 | B03 | b3 | C09 | c2 | D02 | d1 | E03 |
| 2-2-d1E03-1-0251 | 732.149 | OH | A01 | a1 | B01 | b1 | C18 | c2 | D02 | d1 | E03 |
| 2-2-d1E03-1-0252 | 732.169 | OH | A02 | a1 | B05 | b1 | C05 | c1 | D01 | d1 | E03 |
| 2-2-d1E03-1-0253 | 732.205 | OH | A02 | a1 | B03 | b3 | C11 | c1 | D01 | d1 | E03 |
| 2-2-d1E03-1-0254 | 732.223 | OH | A01 | a1 | B05 | b4 | C01 | c1 | D01 | d1 | E03 |
| 2-2-d1E03-1-0255 | 732.229 | OH | A02 | a1 | B02 | b4 | C13 | c2 | D02 | d1 | E03 |
| 2-2-d1E03-1-0256 | 733.133 | OH | A01 | a1 | B02 | b3 | C10 | c2 | D02 | d1 | E03 |
| 2-2-d1E03-1-0257 | 733.178 | OH | A02 | a1 | B04 | b3 | C09 | c1 | D01 | d1 | E03 |
| 2-2-d1E03-1-0258 | 733.182 | OH | A02 | a1 | B05 | b3 | C08 | c1 | D01 | d1 | E03 |
| 2-2-d1E03-1-0259 | 734.163 | OH | A01 | a1 | B03 | b1 | C14 | c2 | D02 | d1 | E03 |
| 2-2-d1E03-1-0260 | 734.187 | OH | A01 | a1 | B02 | b2 | C02 | c2 | D02 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0261 | 734.187 | OH | A01 | a1 | B02 | b2 | C03 | c2 | D02 | d1 | E03 |
| 2-2-d1E03-1-0262 | 734.224 | OH | A02 | a1 | B03 | b4 | C12 | c2 | D02 | d1 | E03 |
| 2-2-d1E03-1-0263 | 734.269 | OH | A01 | a1 | B03 | b1 | C19 | c1 | D01 | d1 | E03 |
| 2-2-d1E03-1-0264 | 735.182 | OH | A01 | a1 | B02 | b2 | C04 | c2 | D02 | d1 | E03 |
| 2-2-d1E03-1-0265 | 735.182 | OH | A01 | a1 | B02 | b2 | C06 | c2 | D02 | d1 | E03 |
| 2-2-d1E03-1-0266 | 735.182 | OH | A01 | a1 | B02 | b2 | C07 | c2 | D02 | d1 | E03 |
| 2-2-d1E03-1-0267 | 735.193 | OH | A02 | a1 | B04 | b1 | C14 | c1 | D01 | d1 | E03 |
| 2-2-d1E03-1-0268 | 736.175 | OH | A01 | a1 | B05 | b1 | C18 | c1 | D01 | d1 | E03 |
| 2-2-d1E03-1-0269 | 736.203 | OH | A02 | a1 | B02 | b1 | C02 | c2 | D02 | d1 | E03 |
| 2-2-d1E03-1-0270 | 736.203 | OH | A02 | a1 | B02 | b1 | C03 | c2 | D02 | d1 | E03 |
| 2-2-d1E03-1-0271 | 736.204 | OH | A02 | a1 | B03 | b4 | C13 | c2 | D02 | d1 | E03 |
| 2-2-d1E03-1-0272 | 737.108 | OH | A01 | a1 | B03 | b3 | C10 | c2 | D02 | d1 | E03 |
| 2-2-d1E03-1-0273 | 737.180 | OH | A01 | a1 | B04 | b4 | C05 | c1 | D01 | d1 | E03 |
| 2-2-d1E03-1-0274 | 737.198 | OH | A02 | a1 | B02 | b1 | C04 | c2 | D02 | d1 | E03 |
| 2-2-d1E03-1-0275 | 738.139 | OH | A02 | a1 | B04 | b3 | C10 | c1 | D01 | d1 | E03 |
| 2-2-d1E03-1-0276 | 738.162 | OH | A01 | a1 | B03 | b2 | C02 | c2 | D02 | d1 | E03 |
| 2-2-d1E03-1-0277 | 738.162 | OH | A01 | a1 | B03 | b2 | C03 | c2 | D02 | d1 | E03 |
| 2-2-d1E03-1-0278 | 738.218 | OH | A01 | a1 | B01 | b4 | C02 | c2 | D02 | d1 | E03 |
| 2-2-d1E03-1-0279 | 739.157 | OH | A01 | a1 | B03 | b2 | C04 | c2 | D02 | d1 | E03 |
| 2-2-d1E03-1-0280 | 739.157 | OH | A01 | a1 | B03 | b2 | C06 | c2 | D02 | d1 | E03 |
| 2-2-d1E03-1-0281 | 739.157 | OH | A01 | a1 | B03 | b2 | C07 | c2 | D02 | d1 | E03 |
| 2-2-d1E03-1-0282 | 739.192 | OH | A02 | a1 | B04 | b2 | C02 | c1 | D01 | d1 | E03 |
| 2-2-d1E03-1-0283 | 739.192 | OH | A02 | a1 | B04 | b2 | C03 | c1 | D01 | d1 | E03 |
| 2-2-d1E03-1-0284 | 739.213 | OH | A01 | a1 | B02 | b1 | C15 | c2 | D02 | d1 | E03 |
| 2-2-d1E03-1-0285 | 740.172 | OH | A02 | a1 | B01 | b3 | C09 | c2 | D02 | d1 | E03 |
| 2-2-d1E03-1-0286 | 740.177 | OH | A02 | a1 | B03 | b1 | C02 | c2 | D02 | d1 | E03 |
| 2-2-d1E03-1-0287 | 740.177 | OH | A02 | a1 | B03 | b1 | C03 | c2 | D02 | d1 | E03 |
| 2-2-d1E03-1-0288 | 740.188 | OH | A02 | a1 | B04 | b2 | C04 | c1 | D01 | d1 | E03 |
| 2-2-d1E03-1-0289 | 740.188 | OH | A02 | a1 | B04 | b2 | C06 | c1 | D01 | d1 | E03 |
| 2-2-d1E03-1-0290 | 740.188 | OH | A02 | a1 | B04 | b2 | C07 | c1 | D01 | d1 | E03 |
| 2-2-d1E03-1-0291 | 740.209 | OH | A01 | a1 | B02 | b1 | C16 | c2 | D02 | d1 | E03 |
| 2-2-d1E03-1-0292 | 741.173 | OH | A02 | a1 | B03 | b1 | C04 | c2 | D02 | d1 | E03 |
| 2-2-d1E03-1-0293 | 741.204 | OH | A01 | a1 | B02 | b1 | C17 | c2 | D02 | d1 | E03 |
| 2-2-d1E03-1-0294 | 742.159 | OH | A02 | a1 | B02 | b1 | C05 | c2 | D02 | d1 | E03 |
| 2-2-d1E03-1-0295 | 742.188 | OH | A02 | a1 | B01 | b1 | C14 | c2 | D02 | d1 | E03 |
| 2-2-d1E03-1-0296 | 742.213 | OH | A01 | a1 | B02 | b4 | C01 | c2 | D02 | d1 | E03 |
| 2-2-d1E03-1-0297 | 742.244 | OH | A01 | a1 | B05 | b4 | C02 | c1 | D01 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0298 | 742.294 | OH | A02 | a1 | B01 | b1 | C19 | c1 | D01 | d1 | E03 |
| 2-2-d1E03-1-0299 | 743.118 | OH | A01 | a1 | B04 | b1 | C01 | c2 | D02 | d1 | E03 |
| 2-2-d1E03-1-0300 | 743.172 | OH | A02 | a1 | B02 | b3 | C08 | c2 | D02 | d1 | E03 |
| 2-2-d1E03-1-0301 | 743.188 | OH | A01 | a1 | B03 | b1 | C15 | c2 | D02 | d1 | E03 |
| 2-2-d1E03-1-0302 | 744.175 | OH | A01 | a1 | B01 | b4 | C05 | c2 | D02 | d1 | E03 |
| 2-2-d1E03-1-0303 | 744.184 | OH | A01 | a1 | B03 | b1 | C16 | c2 | D02 | d1 | E03 |
| 2-2-d1E03-1-0304 | 744.198 | OH | A02 | a1 | B05 | b3 | C09 | c1 | D01 | d1 | E03 |
| 2-2-d1E03-1-0305 | 744.219 | OH | A02 | a1 | B04 | b1 | C15 | c1 | D01 | d1 | E03 |
| 2-2-d1E03-1-0306 | 745.133 | OH | A02 | a1 | B01 | b3 | C10 | c2 | D02 | d1 | E03 |
| 2-2-d1E03-1-0307 | 745.166 | OH | A01 | a1 | B04 | b3 | C11 | c1 | D01 | d1 | E03 |
| 2-2-d1E03-1-0308 | 745.179 | OH | A01 | a1 | B03 | b1 | C17 | c2 | D02 | d1 | E03 |
| 2-2-d1E03-1-0309 | 745.214 | OH | A02 | a1 | B04 | b1 | C16 | c1 | D01 | d1 | E03 |
| 2-2-d1E03-1-0310 | 746.134 | OH | A02 | a1 | B03 | b1 | C05 | c2 | D02 | d1 | E03 |
| 2-2-d1E03-1-0311 | 746.165 | OH | A01 | a1 | B02 | b1 | C18 | c2 | D02 | d1 | E03 |
| 2-2-d1E03-1-0312 | 746.187 | OH | A02 | a1 | B01 | b2 | C02 | c2 | D02 | d1 | E03 |
| 2-2-d1E03-1-0313 | 746.187 | OH | A02 | a1 | B01 | b2 | C03 | c2 | D02 | d1 | E03 |
| 2-2-d1E03-1-0314 | 746.188 | OH | A01 | a1 | B03 | b4 | C01 | c2 | D02 | d1 | E03 |
| 2-2-d1E03-1-0315 | 746.209 | OH | A02 | a1 | B04 | b1 | C17 | c1 | D01 | d1 | E03 |
| 2-2-d1E03-1-0316 | 746.213 | OH | A02 | a1 | B05 | b1 | C14 | c1 | D01 | d1 | E03 |
| 2-2-d1E03-1-0317 | 747.147 | OH | A02 | a1 | B03 | b3 | C08 | c2 | D02 | d1 | E03 |
| 2-2-d1E03-1-0318 | 747.182 | OH | A02 | a1 | B01 | b2 | C04 | c2 | D02 | d1 | E03 |
| 2-2-d1E03-1-0319 | 747.182 | OH | A02 | a1 | B01 | b2 | C06 | c2 | D02 | d1 | E03 |
| 2-2-d1E03-1-0320 | 747.182 | OH | A02 | a1 | B01 | b2 | C07 | c2 | D02 | d1 | E03 |
| 2-2-d1E03-1-0321 | 747.186 | OH | A01 | a1 | B04 | b4 | C12 | c2 | D02 | d1 | E03 |
| 2-2-d1E03-1-0322 | 747.219 | OH | A02 | a1 | B04 | b4 | C01 | c1 | D01 | d1 | E03 |
| 2-2-d1E03-1-0323 | 748.200 | OH | A01 | a1 | B05 | b4 | C05 | c1 | D01 | d1 | E03 |
| 2-2-d1E03-1-0324 | 749.159 | OH | A02 | a1 | B05 | b3 | C10 | c1 | D01 | d1 | E03 |
| 2-2-d1E03-1-0325 | 749.165 | OH | A01 | a1 | B04 | b4 | C13 | c2 | D02 | d1 | E03 |
| 2-2-d1E03-1-0326 | 750.140 | OH | A01 | a1 | B03 | b1 | C18 | c2 | D02 | d1 | E03 |
| 2-2-d1E03-1-0327 | 750.212 | OH | A02 | a1 | B05 | b2 | C02 | c1 | D01 | d1 | E03 |
| 2-2-d1E03-1-0328 | 750.212 | OH | A02 | a1 | B05 | b2 | C03 | c1 | D01 | d1 | E03 |
| 2-2-d1E03-1-0329 | 751.171 | OH | A02 | a1 | B04 | b1 | C18 | c1 | D01 | d1 | E03 |
| 2-2-d1E03-1-0330 | 751.208 | OH | A02 | a1 | B05 | b2 | C04 | c1 | D01 | d1 | E03 |
| 2-2-d1E03-1-0331 | 751.208 | OH | A02 | a1 | B05 | b2 | C06 | c1 | D01 | d1 | E03 |
| 2-2-d1E03-1-0332 | 751.208 | OH | A02 | a1 | B05 | b2 | C07 | c1 | D01 | d1 | E03 |
| 2-2-d1E03-1-0333 | 751.213 | OH | A02 | a1 | B01 | b1 | C15 | c2 | D02 | d1 | E03 |
| 2-2-d1E03-1-0334 | 752.161 | OH | A01 | a1 | B01 | b3 | C11 | c2 | D02 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0335 | 752.209 | OH | A02 | a1 | B01 | b1 | C16 | c2 | D02 | d1 | E03 |
| 2-2-d1E03-1-0336 | 752.234 | OH | A01 | a1 | B02 | b4 | C02 | c2 | D02 | d1 | E03 |
| 2-2-d1E03-1-0337 | 753.139 | OH | A01 | a1 | B04 | b1 | C02 | c2 | D02 | d1 | E03 |
| 2-2-d1E03-1-0338 | 753.139 | OH | A01 | a1 | B04 | b1 | C03 | c2 | D02 | d1 | E03 |
| 2-2-d1E03-1-0339 | 753.204 | OH | A02 | a1 | B01 | b1 | C17 | c2 | D02 | d1 | E03 |
| 2-2-d1E03-1-0340 | 754.134 | OH | A01 | a1 | B04 | b1 | C04 | c2 | D02 | d1 | E03 |
| 2-2-d1E03-1-0341 | 754.138 | OH | A01 | a1 | B05 | b1 | C01 | c2 | D02 | d1 | E03 |
| 2-2-d1E03-1-0342 | 754.188 | OH | A02 | a1 | B02 | b3 | C09 | c2 | D02 | d1 | E03 |
| 2-2-d1E03-1-0343 | 754.213 | OH | A02 | a1 | B01 | b4 | C01 | c2 | D02 | d1 | E03 |
| 2-2-d1E03-1-0344 | 755.239 | OH | A02 | a1 | B05 | b1 | C15 | c1 | D01 | d1 | E03 |
| 2-2-d1E03-1-0345 | 756.187 | OH | A01 | a1 | B05 | b3 | C11 | c1 | D01 | d1 | E03 |
| 2-2-d1E03-1-0346 | 756.204 | OH | A02 | a1 | B02 | b1 | C14 | c2 | D02 | d1 | E03 |
| 2-2-d1E03-1-0347 | 756.209 | OH | A01 | a1 | B03 | b4 | C02 | c2 | D02 | d1 | E03 |
| 2-2-d1E03-1-0348 | 756.234 | OH | A02 | a1 | B05 | b1 | C16 | c1 | D01 | d1 | E03 |
| 2-2-d1E03-1-0349 | 756.309 | OH | A02 | a1 | B02 | b1 | C19 | c1 | D01 | d1 | E03 |
| 2-2-d1E03-1-0350 | 757.229 | OH | A02 | a1 | B05 | b1 | C17 | c1 | D01 | d1 | E03 |
| 2-2-d1E03-1-0351 | 757.239 | OH | A02 | a1 | B04 | b4 | C02 | c1 | D01 | d1 | E03 |
| 2-2-d1E03-1-0352 | 758.163 | OH | A02 | a1 | B03 | b3 | C09 | c2 | D02 | d1 | E03 |
| 2-2-d1E03-1-0353 | 758.165 | OH | A02 | a1 | B01 | b1 | C18 | c2 | D02 | d1 | E03 |
| 2-2-d1E03-1-0354 | 758.190 | OH | A01 | a1 | B02 | b4 | C05 | c2 | D02 | d1 | E03 |
| 2-2-d1E03-1-0355 | 758.206 | OH | A01 | a1 | B05 | b4 | C12 | c2 | D02 | d1 | E03 |
| 2-2-d1E03-1-0356 | 758.239 | OH | A02 | a1 | B05 | b4 | C01 | c1 | D01 | d1 | E03 |
| 2-2-d1E03-1-0357 | 759.095 | OH | A01 | a1 | B04 | b1 | C05 | c2 | D02 | d1 | E03 |
| 2-2-d1E03-1-0358 | 759.149 | OH | A02 | a1 | B02 | b3 | C10 | c2 | D02 | d1 | E03 |
| 2-2-d1E03-1-0359 | 760.108 | OH | A01 | a1 | B04 | b3 | C08 | c2 | D02 | d1 | E03 |
| 2-2-d1E03-1-0360 | 760.179 | OH | A02 | a1 | B03 | b1 | C14 | c2 | D02 | d1 | E03 |
| 2-2-d1E03-1-0361 | 760.185 | OH | A01 | a1 | B05 | b4 | C13 | c2 | D02 | d1 | E03 |
| 2-2-d1E03-1-0362 | 760.203 | OH | A02 | a1 | B02 | b2 | C02 | c2 | D02 | d1 | E03 |
| 2-2-d1E03-1-0363 | 760.203 | OH | A02 | a1 | B02 | b2 | C03 | c2 | D02 | d1 | E03 |
| 2-2-d1E03-1-0364 | 760.284 | OH | A02 | a1 | B03 | b1 | C19 | c1 | D01 | d1 | E03 |
| 2-2-d1E03-1-0365 | 761.198 | OH | A02 | a1 | B02 | b2 | C04 | c2 | D02 | d1 | E03 |
| 2-2-d1E03-1-0366 | 761.198 | OH | A02 | a1 | B02 | b2 | C06 | c2 | D02 | d1 | E03 |
| 2-2-d1E03-1-0367 | 761.198 | OH | A02 | a1 | B02 | b2 | C07 | c2 | D02 | d1 | E03 |
| 2-2-d1E03-1-0368 | 762.165 | OH | A01 | a1 | B03 | b4 | C05 | c2 | D02 | d1 | E03 |
| 2-2-d1E03-1-0369 | 762.191 | OH | A02 | a1 | B05 | b1 | C18 | c1 | D01 | d1 | E03 |
| 2-2-d1E03-1-0370 | 763.124 | OH | A02 | a1 | B03 | b3 | C10 | c2 | D02 | d1 | E03 |
| 2-2-d1E03-1-0371 | 763.196 | OH | A02 | a1 | B04 | b4 | C05 | c1 | D01 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0372 | 764.159 | OH | A01 | a1 | B05 | b1 | C02 | c2 | D02 | d1 | E03 |
| 2-2-d1E03-1-0373 | 764.159 | OH | A01 | a1 | B05 | b1 | C03 | c2 | D02 | d1 | E03 |
| 2-2-d1E03-1-0374 | 764.177 | OH | A02 | a1 | B03 | b2 | C02 | c2 | D02 | d1 | E03 |
| 2-2-d1E03-1-0375 | 764.177 | OH | A02 | a1 | B03 | b2 | C03 | c2 | D02 | d1 | E03 |
| 2-2-d1E03-1-0376 | 764.234 | OH | A02 | a1 | B01 | b4 | C02 | c2 | D02 | d1 | E03 |
| 2-2-d1E03-1-0377 | 765.154 | OH | A01 | a1 | B05 | b1 | C04 | c2 | D02 | d1 | E03 |
| 2-2-d1E03-1-0378 | 765.173 | OH | A02 | a1 | B03 | b2 | C04 | c2 | D02 | d1 | E03 |
| 2-2-d1E03-1-0379 | 765.173 | OH | A02 | a1 | B03 | b2 | C06 | c2 | D02 | d1 | E03 |
| 2-2-d1E03-1-0380 | 765.173 | OH | A02 | a1 | B03 | b2 | C07 | c2 | D02 | d1 | E03 |
| 2-2-d1E03-1-0381 | 765.229 | OH | A02 | a1 | B02 | b1 | C15 | c2 | D02 | d1 | E03 |
| 2-2-d1E03-1-0382 | 766.177 | OH | A01 | a1 | B02 | b3 | C11 | c2 | D02 | d1 | E03 |
| 2-2-d1E03-1-0383 | 766.224 | OH | A02 | a1 | B02 | b1 | C16 | c2 | D02 | d1 | E03 |
| 2-2-d1E03-1-0384 | 767.220 | OH | A02 | a1 | B02 | b1 | C17 | c2 | D02 | d1 | E03 |
| 2-2-d1E03-1-0385 | 768.229 | OH | A02 | a1 | B02 | b4 | C01 | c2 | D02 | d1 | E03 |
| 2-2-d1E03-1-0386 | 768.259 | OH | A02 | a1 | B05 | b4 | C02 | c1 | D01 | d1 | E03 |
| 2-2-d1E03-1-0387 | 769.133 | OH | A02 | a1 | B04 | b1 | C01 | c2 | D02 | d1 | E03 |
| 2-2-d1E03-1-0388 | 769.204 | OH | A02 | a1 | B03 | b1 | C15 | c2 | D02 | d1 | E03 |
| 2-2-d1E03-1-0389 | 770.115 | OH | A01 | a1 | B05 | b1 | C05 | c2 | D02 | d1 | E03 |
| 2-2-d1E03-1-0390 | 770.152 | OH | A01 | a1 | B03 | b3 | C11 | c2 | D02 | d1 | E03 |
| 2-2-d1E03-1-0391 | 770.190 | OH | A02 | a1 | B01 | b4 | C05 | c2 | D02 | d1 | E03 |
| 2-2-d1E03-1-0392 | 770.199 | OH | A02 | a1 | B03 | b1 | C16 | c2 | D02 | d1 | E03 |
| 2-2-d1E03-1-0393 | 771.124 | OH | A01 | a1 | B04 | b3 | C09 | c2 | D02 | d1 | E03 |
| 2-2-d1E03-1-0394 | 771.128 | OH | A01 | a1 | B05 | b3 | C08 | c2 | D02 | d1 | E03 |
| 2-2-d1E03-1-0395 | 771.182 | OH | A02 | a1 | B04 | b3 | C11 | c1 | D01 | d1 | E03 |
| 2-2-d1E03-1-0396 | 771.195 | OH | A02 | a1 | B03 | b1 | C17 | c2 | D02 | d1 | E03 |
| 2-2-d1E03-1-0397 | 772.181 | OH | A02 | a1 | B02 | b1 | C18 | c2 | D02 | d1 | E03 |
| 2-2-d1E03-1-0398 | 772.204 | OH | A02 | a1 | B03 | b4 | C01 | c2 | D02 | d1 | E03 |
| 2-2-d1E03-1-0399 | 773.140 | OH | A01 | a1 | B04 | b1 | C14 | c2 | D02 | d1 | E03 |
| 2-2-d1E03-1-0400 | 773.201 | OH | A02 | a1 | B04 | b4 | C12 | c2 | D02 | d1 | E03 |
| 2-2-d1E03-1-0401 | 773.245 | OH | A01 | a1 | B04 | b1 | C19 | c1 | D01 | d1 | E03 |
| 2-2-d1E03-1-0402 | 774.216 | OH | A02 | a1 | B05 | b4 | C05 | c1 | D01 | d1 | E03 |
| 2-2-d1E03-1-0403 | 775.180 | OH | A02 | a1 | B04 | b4 | C13 | c2 | D02 | d1 | E03 |
| 2-2-d1E03-1-0404 | 776.085 | OH | A01 | a1 | B04 | b3 | C10 | c2 | D02 | d1 | E03 |
| 2-2-d1E03-1-0405 | 776.156 | OH | A02 | a1 | B03 | b1 | C18 | c2 | D02 | d1 | E03 |
| 2-2-d1E03-1-0406 | 777.139 | OH | A01 | a1 | B04 | b2 | C02 | c2 | D02 | d1 | E03 |
| 2-2-d1E03-1-0407 | 777.139 | OH | A01 | a1 | B04 | b2 | C03 | c2 | D02 | d1 | E03 |
| 2-2-d1E03-1-0408 | 778.134 | OH | A01 | a1 | B04 | b2 | C04 | c2 | D02 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0409 | 778.134 | OH | A01 | a1 | B04 | b2 | C06 | c2 | D02 | d1 | E03 |
| 2-2-d1E03-1-0410 | 778.134 | OH | A01 | a1 | B04 | b2 | C07 | c2 | D02 | d1 | E03 |
| 2-2-d1E03-1-0411 | 778.177 | OH | A02 | a1 | B01 | b3 | C11 | c2 | D02 | d1 | E03 |
| 2-2-d1E03-1-0412 | 778.249 | OH | A02 | a1 | B02 | b4 | C02 | c2 | D02 | d1 | E03 |
| 2-2-d1E03-1-0413 | 779.154 | OH | A02 | a1 | B04 | b1 | C02 | c2 | D02 | d1 | E03 |
| 2-2-d1E03-1-0414 | 779.154 | OH | A02 | a1 | B04 | b1 | C03 | c2 | D02 | d1 | E03 |
| 2-2-d1E03-1-0415 | 780.149 | OH | A02 | a1 | B04 | b1 | C04 | c2 | D02 | d1 | E03 |
| 2-2-d1E03-1-0416 | 780.154 | OH | A02 | a1 | B05 | b1 | C01 | c2 | D02 | d1 | E03 |
| 2-2-d1E03-1-0417 | 780.240 | OH | A01 | a1 | B01 | b1 | C19 | c2 | D02 | d1 | E03 |
| 2-2-d1E03-1-0418 | 782.144 | OH | A01 | a1 | B05 | b3 | C09 | c2 | D02 | d1 | E03 |
| 2-2-d1E03-1-0419 | 782.165 | OH | A01 | a1 | B04 | b1 | C15 | c2 | D02 | d1 | E03 |
| 2-2-d1E03-1-0420 | 782.202 | OH | A02 | a1 | B05 | b3 | C11 | c1 | D01 | d1 | E03 |
| 2-2-d1E03-1-0421 | 782.224 | OH | A02 | a1 | B03 | b4 | C02 | c2 | D02 | d1 | E03 |
| 2-2-d1E03-1-0422 | 783.160 | OH | A01 | a1 | B04 | b1 | C16 | c2 | D02 | d1 | E03 |
| 2-2-d1E03-1-0423 | 784.156 | OH | A01 | a1 | B04 | b1 | C17 | c2 | D02 | d1 | E03 |
| 2-2-d1E03-1-0424 | 784.160 | OH | A01 | a1 | B05 | b1 | C14 | c2 | D02 | d1 | E03 |
| 2-2-d1E03-1-0425 | 784.206 | OH | A02 | a1 | B02 | b4 | C05 | c2 | D02 | d1 | E03 |
| 2-2-d1E03-1-0426 | 784.221 | OH | A02 | a1 | B05 | b4 | C12 | c2 | D02 | d1 | E03 |
| 2-2-d1E03-1-0427 | 784.265 | OH | A01 | a1 | B05 | b1 | C19 | c1 | D01 | d1 | E03 |
| 2-2-d1E03-1-0428 | 785.111 | OH | A02 | a1 | B04 | b1 | C05 | c2 | D02 | d1 | E03 |
| 2-2-d1E03-1-0429 | 785.165 | OH | A01 | a1 | B04 | b4 | C01 | c2 | D02 | d1 | E03 |
| 2-2-d1E03-1-0430 | 786.124 | OH | A02 | a1 | B04 | b3 | C08 | c2 | D02 | d1 | E03 |
| 2-2-d1E03-1-0431 | 786.200 | OH | A02 | a1 | B05 | b4 | C13 | c2 | D02 | d1 | E03 |
| 2-2-d1E03-1-0432 | 787.105 | OH | A01 | a1 | B05 | b3 | C10 | c2 | D02 | d1 | E03 |
| 2-2-d1E03-1-0433 | 788.159 | OH | A01 | a1 | B05 | b2 | C02 | c2 | D02 | d1 | E03 |
| 2-2-d1E03-1-0434 | 788.159 | OH | A01 | a1 | B05 | b2 | C03 | c2 | D02 | d1 | E03 |
| 2-2-d1E03-1-0435 | 788.181 | OH | A02 | a1 | B03 | b4 | C05 | c2 | D02 | d1 | E03 |
| 2-2-d1E03-1-0436 | 789.117 | OH | A01 | a1 | B04 | b1 | C18 | c2 | D02 | d1 | E03 |
| 2-2-d1E03-1-0437 | 789.154 | OH | A01 | a1 | B05 | b2 | C04 | c2 | D02 | d1 | E03 |
| 2-2-d1E03-1-0438 | 789.154 | OH | A01 | a1 | B05 | b2 | C06 | c2 | D02 | d1 | E03 |
| 2-2-d1E03-1-0439 | 789.154 | OH | A01 | a1 | B05 | b2 | C07 | c2 | D02 | d1 | E03 |
| 2-2-d1E03-1-0440 | 790.174 | OH | A02 | a1 | B05 | b1 | C02 | c2 | D02 | d1 | E03 |
| 2-2-d1E03-1-0441 | 790.174 | OH | A02 | a1 | B05 | b1 | C03 | c2 | D02 | d1 | E03 |
| 2-2-d1E03-1-0442 | 791.170 | OH | A02 | a1 | B05 | b1 | C04 | c2 | D02 | d1 | E03 |
| 2-2-d1E03-1-0443 | 792.192 | OH | A02 | a1 | B02 | b3 | C11 | c2 | D02 | d1 | E03 |
| 2-2-d1E03-1-0444 | 793.185 | OH | A01 | a1 | B05 | b1 | C15 | c2 | D02 | d1 | E03 |
| 2-2-d1E03-1-0445 | 794.180 | OH | A01 | a1 | B05 | b1 | C16 | c2 | D02 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0446 | 794.256 | OH | A01 | a1 | B02 | b1 | C19 | c2 | D02 | d1 | E03 |
| 2-2-d1E03-1-0447 | 795.176 | OH | A01 | a1 | B05 | b1 | C17 | c2 | D02 | d1 | E03 |
| 2-2-d1E03-1-0448 | 795.186 | OH | A01 | a1 | B04 | b4 | C02 | c2 | D02 | d1 | E03 |
| 2-2-d1E03-1-0449 | 796.131 | OH | A02 | a1 | B05 | b1 | C05 | c2 | D02 | d1 | E03 |
| 2-2-d1E03-1-0450 | 796.167 | OH | A02 | a1 | B03 | b3 | C11 | c2 | D02 | d1 | E03 |
| 2-2-d1E03-1-0451 | 796.185 | OH | A01 | a1 | B05 | b4 | C01 | c2 | D02 | d1 | E03 |
| 2-2-d1E03-1-0452 | 797.140 | OH | A02 | a1 | B04 | b3 | C09 | c2 | D02 | d1 | E03 |
| 2-2-d1E03-1-0453 | 797.144 | OH | A02 | a1 | B05 | b3 | C08 | c2 | D02 | d1 | E03 |
| 2-2-d1E03-1-0454 | 798.231 | OH | A01 | a1 | B03 | b1 | C19 | c2 | D02 | d1 | E03 |
| 2-2-d1E03-1-0455 | 799.155 | OH | A02 | a1 | B04 | b1 | C14 | c2 | D02 | d1 | E03 |
| 2-2-d1E03-1-0456 | 799.261 | OH | A02 | a1 | B04 | b1 | C19 | c1 | D01 | d1 | E03 |
| 2-2-d1E03-1-0457 | 800.137 | OH | A01 | a1 | B05 | b1 | C18 | c2 | D02 | d1 | E03 |
| 2-2-d1E03-1-0458 | 801.142 | OH | A01 | a1 | B04 | b4 | C05 | c2 | D02 | d1 | E03 |
| 2-2-d1E03-1-0459 | 802.101 | OH | A02 | a1 | B04 | b3 | C10 | c2 | D02 | d1 | E03 |
| 2-2-d1E03-1-0460 | 803.154 | OH | A02 | a1 | B04 | b2 | C02 | c2 | D02 | d1 | E03 |
| 2-2-d1E03-1-0461 | 803.154 | OH | A02 | a1 | B04 | b2 | C03 | c2 | D02 | d1 | E03 |
| 2-2-d1E03-1-0462 | 804.149 | OH | A02 | a1 | B04 | b2 | C04 | c2 | D02 | d1 | E03 |
| 2-2-d1E03-1-0463 | 804.149 | OH | A02 | a1 | B04 | b2 | C06 | c2 | D02 | d1 | E03 |
| 2-2-d1E03-1-0464 | 804.149 | OH | A02 | a1 | B04 | b2 | C07 | c2 | D02 | d1 | E03 |
| 2-2-dlE03-1-0465 | 806.206 | OH | A01 | a1 | B05 | b4 | C02 | c2 | D02 | d1 | E03 |
| 2-2-d1E03-1-0466 | 806.256 | OH | A02 | a1 | B01 | b1 | C19 | c2 | D02 | d1 | E03 |
| 2-2-d1E03-1-0467 | 808.160 | OH | A02 | a1 | B05 | b3 | C09 | c2 | D02 | d1 | E03 |
| 2-2-dlE03-1-0468 | 808.181 | OH | A02 | a1 | B04 | b1 | C15 | c2 | D02 | d1 | E03 |
| 2-2-d1E03-1-0469 | 809.128 | OH | A01 | a1 | B04 | b3 | C11 | c2 | D02 | d1 | E03 |
| 2-2-dlE03-1-0470 | 809.176 | OH | A02 | a1 | B04 | b1 | C16 | c2 | D02 | d1 | E03 |
| 2-2-d1E03-1-0471 | 810.171 | OH | A02 | a1 | B04 | b1 | C17 | c2 | D02 | d1 | E03 |
| 2-2-d1E03-1-0472 | 810.175 | OH | A02 | a1 | B05 | b1 | C14 | c2 | D02 | d1 | E03 |
| 2-2-dlE03-1-0473 | 810.281 | OH | A02 | a1 | B05 | b1 | C19 | c1 | D01 | d1 | E03 |
| 2-2-dlE03-1-0474 | 811.180 | OH | A02 | a1 | B04 | b4 | C01 | c2 | D02 | d1 | E03 |
| 2-2-d1E03-1-0475 | 812.162 | OH | A01 | a1 | B05 | b4 | C05 | c2 | D02 | d1 | E03 |
| 2-2-dlE03-1-0476 | 813.121 | OH | A02 | a1 | B05 | b3 | C10 | c2 | D02 | d1 | E03 |
| 2-2-dlE03-1-0477 | 814.174 | OH | A02 | a1 | B05 | b2 | C02 | c2 | D02 | d1 | E03 |
| 2-2-d1E03-1-0478 | 814.174 | OH | A02 | a1 | B05 | b2 | C03 | c2 | D02 | d1 | E03 |
| 2-2-d1E03-1-0479 | 815.132 | OH | A02 | a1 | B04 | b1 | C18 | c2 | D02 | d1 | E03 |
| 2-2-d1E03-1-0480 | 815.170 | OH | A02 | a1 | B05 | b2 | C04 | c2 | D02 | d1 | E03 |
| 2-2-d1E03-1-0481 | 815.170 | OH | A02 | a1 | B05 | b2 | C06 | c2 | D02 | d1 | E03 |
| 2-2-d1E03-1-0482 | 815.170 | OH | A02 | a1 | B05 | b2 | C07 | c2 | D02 | d1 | E03 |

(continued)

| [Table 9-5-4] Compound that may be contained in mixture 2-2-d1E03-1 | | | | | | | | | | | |
|---|---|---|---|---|---|---|---|---|---|---|---|
| ID | Exact Mass | | A | a | B | b | C | c | D | d | E |
| 2-2-d1E03-1-0483 | 819.201 | OH | A02 | a1 | B05 | b1 | C15 | c2 | D02 | d1 | E03 |
| 2-2-d1E03-1-0484 | 820.148 | OH | A01 | a1 | B05 | b3 | C11 | c2 | D02 | d1 | E03 |
| 2-2-d1E03-1-0485 | 820.196 | OH | A02 | a1 | B05 | b1 | C16 | c2 | D02 | d1 | E03 |
| 2-2-d1E03-1-0486 | 820.271 | OH | A02 | a1 | B02 | b1 | C19 | c2 | D02 | d1 | E03 |
| 2-2-d1E03-1-0487 | 821.191 | OH | A02 | a1 | B05 | b1 | C17 | c2 | D02 | d1 | E03 |
| 2-2-d1E03-1-0488 | 821.201 | OH | A02 | a1 | B04 | b4 | C02 | c2 | D02 | d1 | E03 |
| 2-2-d1E03-1-0489 | 822.200 | OH | A02 | a1 | B05 | b4 | C01 | c2 | D02 | d1 | E03 |
| 2-2-d1E03-1-0490 | 824.246 | OH | A02 | a1 | B03 | b1 | C19 | c2 | D02 | d1 | E03 |
| 2-2-d1E03-1-0491 | 826.152 | OH | A02 | a1 | B05 | b1 | C18 | c2 | D02 | d1 | E03 |
| 2-2-d1E03-1-0492 | 827.158 | OH | A02 | a1 | B04 | b4 | C05 | c2 | D02 | d1 | E03 |
| 2-2-d1E03-1-0493 | 832.221 | OH | A02 | a1 | B05 | b4 | C02 | c2 | D02 | d1 | E03 |
| 2-2-d1E03-1-0494 | 835.144 | OH | A02 | a1 | B04 | b3 | C11 | c2 | D02 | d1 | E03 |
| 2-2-d1E03-1-0495 | 837.207 | OH | A01 | a1 | B04 | b1 | C19 | c2 | D02 | d1 | E03 |
| 2-2-d1E03-1-0496 | 838.178 | OH | A02 | a1 | B05 | b4 | C05 | c2 | D02 | d1 | E03 |
| 2-2-d1E03-1-0497 | 846.164 | OH | A02 | a1 | B05 | b3 | C11 | c2 | D02 | d1 | E03 |
| 2-2-d1E03-1-0498 | 848.227 | OH | A01 | a1 | B05 | b1 | C19 | c2 | D02 | d1 | E03 |
| 2-2-d1E03-1-0499 | 863.223 | OH | A02 | a1 | B04 | b1 | C19 | c2 | D02 | d1 | E03 |
| 2-2-d1E03-1-0500 | 874.243 | OH | A02 | a1 | B05 | b1 | C19 | c2 | D02 | d1 | E03 |

[Table 495]

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0001 | 735.273 | OH | A01 | a1 | B01 | b1 | C01 | c1 | D01 | d1 | E04 |
| 2-2-d1E04-1-0002 | 739.340 | OH | A01 | a1 | B01 | b4 | C12 | c1 | D01 | d1 | E04 |
| 2-2-d1E04-1-0003 | 741.320 | OH | A01 | a1 | B01 | b4 | C13 | c1 | D01 | d1 | E04 |
| 2-2-d1E04-1-0004 | 745.293 | OH | A01 | a1 | B01 | b1 | C02 | c1 | D01 | d1 | E04 |
| 2-2-d1E04-1-0005 | 745.293 | OH | A01 | a1 | B01 | b1 | C03 | c1 | D01 | d1 | E04 |
| 2-2-d1E04-1-0006 | 746.289 | OH | A01 | a1 | B01 | b1 | C04 | c1 | D01 | d1 | E04 |
| 2-2-d1E04-1-0007 | 749.288 | OH | A01 | a1 | B02 | b1 | C01 | c1 | D01 | d1 | E04 |
| 2-2-d1E04-1-0008 | 751.250 | OH | A01 | a1 | B01 | b1 | C05 | c1 | D01 | d1 | E04 |
| 2-2-d1E04-1-0009 | 752.263 | OH | A01 | a1 | B01 | b3 | C08 | c1 | D01 | d1 | E04 |
| 2-2-d1E04-1-0010 | 753.263 | OH | A01 | a1 | B03 | b1 | C01 | c1 | D01 | d1 | E04 |
| 2-2-d1E04-1-0011 | 753.356 | OH | A01 | a1 | B02 | b4 | C12 | c1 | D01 | d1 | E04 |
| 2-2-d1E04-1-0012 | 755.335 | OH | A01 | a1 | B02 | b4 | C13 | c1 | D01 | d1 | E04 |
| 2-2-d1E04-1-0013 | 757.331 | OH | A01 | a1 | B03 | b4 | C12 | c1 | D01 | d1 | E04 |
| 2-2-d1E04-1-0014 | 759.309 | OH | A01 | a1 | B02 | b1 | C02 | c1 | D01 | d1 | E04 |
| 2-2-d1E04-1-0015 | 759.309 | OH | A01 | a1 | B02 | b1 | C03 | c1 | D01 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0016 | 759.310 | OH | A01 | a1 | B03 | b4 | C13 | c1 | D01 | d1 | E04 |
| 2-2-d1E04-1-0017 | 760.304 | OH | A01 | a1 | B02 | b1 | C04 | c1 | D01 | d1 | E04 |
| 2-2-d1E04-1-0018 | 761.288 | OH | A02 | a1 | B01 | b1 | C01 | c1 | D01 | d1 | E04 |
| 2-2-d1E04-1-0019 | 763.279 | OH | A01 | a1 | B01 | b3 | C09 | c1 | D01 | d1 | E04 |
| 2-2-d1E04-1-0020 | 763.284 | OH | A01 | a1 | B03 | b1 | C02 | c1 | D01 | d1 | E04 |
| 2-2-d1E04-1-0021 | 763.284 | OH | A01 | a1 | B03 | b1 | C03 | c1 | D01 | d1 | E04 |
| 2-2-d1E04-1-0022 | 764.279 | OH | A01 | a1 | B03 | b1 | C04 | c1 | D01 | d1 | E04 |
| 2-2-dlE04-1-0023 | 765.265 | OH | A01 | a1 | B02 | b1 | C05 | c1 | D01 | d1 | E04 |
| 2-2-d1E04-1-0024 | 765.294 | OH | A01 | a1 | B01 | b1 | C14 | c1 | D01 | d1 | E04 |
| 2-2-d1E04-1-0025 | 765.356 | OH | A02 | a1 | B01 | b4 | C12 | c1 | D01 | d1 | E04 |
| 2-2-d1E04-1-0026 | 766.278 | OH | A01 | a1 | B02 | b3 | C08 | c1 | D01 | d1 | E04 |
| 2-2-d1E04-1-0027 | 767.335 | OH | A02 | a1 | B01 | b4 | C13 | c1 | D01 | d1 | E04 |
| 2-2-d1E04-1-0028 | 768.240 | OH | A01 | a1 | B01 | b3 | C10 | c1 | D01 | d1 | E04 |
| 2-2-d1E04-1-0029 | 769.240 | OH | A01 | a1 | B03 | b1 | C05 | c1 | D01 | d1 | E04 |
| 2-2-d1E04-1-0030 | 769.293 | OH | A01 | a1 | B01 | b2 | C02 | c1 | D01 | d1 | E04 |
| 2-2-d1E04-1-0031 | 769.293 | OH | A01 | a1 | B01 | b2 | C03 | c1 | D01 | d1 | E04 |
| 2-2-d1E04-1-0032 | 770.253 | OH | A01 | a1 | B03 | b3 | C08 | c1 | D01 | d1 | E04 |
| 2-2-d1E04-1-0033 | 770.289 | OH | A01 | a1 | B01 | b2 | C04 | c1 | D01 | d1 | E04 |
| 2-2-d1E04-1-0034 | 770.289 | OH | A01 | a1 | B01 | b2 | C06 | c1 | D01 | d1 | E04 |
| 2-2-d1E04-1-0035 | 770.289 | OH | A01 | a1 | B01 | b2 | C07 | c1 | D01 | d1 | E04 |
| 2-2-d1E04-1-0036 | 771.309 | OH | A02 | a1 | B01 | b1 | C02 | c1 | D01 | d1 | E04 |
| 2-2-d1E04-1-0037 | 771.309 | OH | A02 | a1 | B01 | b1 | C03 | c1 | D01 | d1 | E04 |
| 2-2-d1E04-1-0038 | 772.304 | OH | A02 | a1 | B01 | b1 | C04 | c1 | D01 | d1 | E04 |
| 2-2-d1E04-1-0039 | 774.320 | OH | A01 | a1 | B01 | b1 | C15 | c1 | D01 | d1 | E04 |
| 2-2-d1E04-1-0040 | 775.304 | OH | A02 | a1 | B02 | b1 | C01 | c1 | D01 | d1 | E04 |
| 2-2-d1E04-1-0041 | 775.315 | OH | A01 | a1 | B01 | b1 | C16 | c1 | D01 | d1 | E04 |
| 2-2-d1E04-1-0042 | 776.310 | OH | A01 | a1 | B01 | b1 | C17 | c1 | D01 | d1 | E04 |
| 2-2-d1E04-1-0043 | 777.265 | OH | A02 | a1 | B01 | b1 | C05 | c1 | D01 | d1 | E04 |
| 2-2-d1E04-1-0044 | 777.294 | OH | A01 | a1 | B02 | b3 | C09 | c1 | D01 | d1 | E04 |
| 2-2-d1E04-1-0045 | 777.320 | OH | A01 | a1 | B01 | b4 | C01 | c1 | D01 | d1 | E04 |
| 2-2-d1E04-1-0046 | 778.278 | OH | A02 | a1 | B01 | b3 | C08 | c1 | D01 | d1 | E04 |
| 2-2-d1E04-1-0047 | 779.279 | OH | A02 | a1 | B03 | b1 | C01 | c1 | D01 | d1 | E04 |
| 2-2-dlE04-1-0048 | 779.310 | OH | A01 | a1 | B02 | b1 | C14 | c1 | D01 | d1 | E04 |
| 2-2-d1E04-1-0049 | 779.372 | OH | A02 | a1 | B02 | b4 | C12 | c1 | D01 | d1 | E04 |
| 2-2-d1E04-1-0050 | 781.269 | OH | A01 | a1 | B03 | b3 | C09 | c1 | D01 | d1 | E04 |
| 2-2-d1E04-1-0051 | 781.272 | OH | A01 | a1 | B01 | b1 | C18 | c1 | D01 | d1 | E04 |
| 2-2-d1E04-1-0052 | 781.351 | OH | A02 | a1 | B02 | b4 | C13 | c1 | D01 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0053 | 782.256 | OH | A01 | a1 | B02 | b3 | C10 | c1 | D01 | d1 | E04 |
| 2-2-d1E04-1-0054 | 783.285 | OH | A01 | a1 | B03 | b1 | C14 | c1 | D01 | d1 | E04 |
| 2-2-d1E04-1-0055 | 783.309 | OH | A01 | a1 | B02 | b2 | C02 | c1 | D01 | d1 | E04 |
| 2-2-d1E04-1-0056 | 783.309 | OH | A01 | a1 | B02 | b2 | C03 | c1 | D01 | d1 | E04 |
| 2-2-d1E04-1-0057 | 783.347 | OH | A02 | a1 | B03 | b4 | C12 | c1 | D01 | d1 | E04 |
| 2-2-d1E04-1-0058 | 784.304 | OH | A01 | a1 | B02 | b2 | C04 | c1 | D01 | d1 | E04 |
| 2-2-d1E04-1-0059 | 784.304 | OH | A01 | a1 | B02 | b2 | C06 | c1 | D01 | d1 | E04 |
| 2-2-d1E04-1-0060 | 784.304 | OH | A01 | a1 | B02 | b2 | C07 | c1 | D01 | d1 | E04 |
| 2-2-d1E04-1-0061 | 785.325 | OH | A02 | a1 | B02 | b1 | C02 | c1 | D01 | d1 | E04 |
| 2-2-dlE04-1-0062 | 785.325 | OH | A02 | a1 | B02 | b1 | C03 | c1 | D01 | d1 | E04 |
| 2-2-dlE04-1-0063 | 785.326 | OH | A02 | a1 | B03 | b4 | C13 | c1 | D01 | d1 | E04 |
| 2-2-d1E04-1-0064 | 786.231 | OH | A01 | a1 | B03 | b3 | C10 | c1 | D01 | d1 | E04 |
| 2-2-d1E04-1-0065 | 786.320 | OH | A02 | a1 | B02 | b1 | C04 | c1 | D01 | d1 | E04 |
| 2-2-d1E04-1-0066 | 787.284 | OH | A01 | a1 | B03 | b2 | C02 | c1 | D01 | d1 | E04 |
| 2-2-d1E04-1-0067 | 787.284 | OH | A01 | a1 | B03 | b2 | C03 | c1 | D01 | d1 | E04 |
| 2-2-d1E04-1-0068 | 787.340 | OH | A01 | a1 | B01 | b4 | C02 | c1 | D01 | d1 | E04 |
| 2-2-d1E04-1-0069 | 788.279 | OH | A01 | a1 | B03 | b2 | C04 | c1 | D01 | d1 | E04 |
| 2-2-d1E04-1-0070 | 788.279 | OH | A01 | a1 | B03 | b2 | C06 | c1 | D01 | d1 | E04 |
| 2-2-d1E04-1-0071 | 788.279 | OH | A01 | a1 | B03 | b2 | C07 | c1 | D01 | d1 | E04 |
| 2-2-d1E04-1-0072 | 788.336 | OH | A01 | a1 | B02 | b1 | C15 | c1 | D01 | d1 | E04 |
| 2-2-d1E04-1-0073 | 789.294 | OH | A02 | a1 | B01 | b3 | C09 | c1 | D01 | d1 | E04 |
| 2-2-d1E04-1-0074 | 789.300 | OH | A02 | a1 | B03 | b1 | C02 | c1 | D01 | d1 | E04 |
| 2-2-d1E04-1-0075 | 789.300 | OH | A02 | a1 | B03 | b1 | C03 | c1 | D01 | d1 | E04 |
| 2-2-d1E04-1-0076 | 789.331 | OH | A01 | a1 | B02 | b1 | C16 | c1 | D01 | d1 | E04 |
| 2-2-d1E04-1-0077 | 790.295 | OH | A02 | a1 | B03 | b1 | C04 | c1 | D01 | d1 | E04 |
| 2-2-d1E04-1-0078 | 790.326 | OH | A01 | a1 | B02 | b1 | C17 | c1 | D01 | d1 | E04 |
| 2-2-d1E04-1-0079 | 791.281 | OH | A02 | a1 | B02 | b1 | C05 | c1 | D01 | d1 | E04 |
| 2-2-dlE04-1-0080 | 791.310 | OH | A02 | a1 | B01 | b1 | C14 | c1 | D01 | d1 | E04 |
| 2-2-d1E04-1-0081 | 791.335 | OH | A01 | a1 | B02 | b4 | C01 | c1 | D01 | d1 | E04 |
| 2-2-d1E04-1-0082 | 792.240 | OH | A01 | a1 | B04 | b1 | C01 | c1 | D01 | d1 | E04 |
| 2-2-d1E04-1-0083 | 792.294 | OH | A02 | a1 | B02 | b3 | C08 | c1 | D01 | d1 | E04 |
| 2-2-d1E04-1-0084 | 792.311 | OH | A01 | a1 | B03 | b1 | C15 | c1 | D01 | d1 | E04 |
| 2-2-d1E04-1-0085 | 793.297 | OH | A01 | a1 | B01 | b4 | C05 | c1 | D01 | d1 | E04 |
| 2-2-d1E04-1-0086 | 793.306 | OH | A01 | a1 | B03 | b1 | C16 | c1 | D01 | d1 | E04 |
| 2-2-d1E04-1-0087 | 794.256 | OH | A02 | a1 | B01 | b3 | C10 | c1 | D01 | d1 | E04 |
| 2-2-d1E04-1-0088 | 794.301 | OH | A01 | a1 | B03 | b1 | C17 | c1 | D01 | d1 | E04 |
| 2-2-dlE04-1-0089 | 795.256 | OH | A02 | a1 | B03 | b1 | C05 | c1 | D01 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0090 | 795.287 | OH | A01 | a1 | B02 | b1 | C18 | c1 | D01 | d1 | E04 |
| 2-2-d1E04-1-0091 | 795.309 | OH | A02 | a1 | B01 | b2 | C02 | c1 | D01 | d1 | E04 |
| 2-2-d1E04-1-0092 | 795.309 | OH | A02 | a1 | B01 | b2 | C03 | c1 | D01 | d1 | E04 |
| 2-2-d1E04-1-0093 | 795.310 | OH | A01 | a1 | B03 | b4 | C01 | c1 | D01 | d1 | E04 |
| 2-2-d1E04-1-0094 | 796.269 | OH | A02 | a1 | B03 | b3 | C08 | c1 | D01 | d1 | E04 |
| 2-2-d1E04-1-0095 | 796.304 | OH | A02 | a1 | B01 | b2 | C04 | c1 | D01 | d1 | E04 |
| 2-2-dlE04-1-0096 | 796.304 | OH | A02 | a1 | B01 | b2 | C06 | c1 | D01 | d1 | E04 |
| 2-2-d1E04-1-0097 | 796.304 | OH | A02 | a1 | B01 | b2 | C07 | c1 | D01 | d1 | E04 |
| 2-2-dlE04-1-0098 | 796.308 | OH | A01 | a1 | B04 | b4 | C12 | c1 | D01 | d1 | E04 |
| 2-2-dlE04-1-0099 | 798.287 | OH | A01 | a1 | B04 | b4 | C13 | c1 | D01 | d1 | E04 |
| 2-2-d1E04-1-0100 | 799.235 | OH | A01 | a1 | B01 | b1 | C01 | c2 | D02 | d1 | E04 |
| 2-2-d1E04-1-0101 | 799.262 | OH | A01 | a1 | B03 | b1 | C18 | c1 | D01 | d1 | E04 |
| 2-2-d1E04-1-0102 | 800.336 | OH | A02 | a1 | B01 | b1 | C15 | c1 | D01 | d1 | E04 |
| 2-2-d1E04-1-0103 | 801.283 | OH | A01 | a1 | B01 | b3 | C11 | c1 | D01 | d1 | E04 |
| 2-2-dlE04-1-0104 | 801.331 | OH | A02 | a1 | B01 | b1 | C16 | c1 | D01 | d1 | E04 |
| 2-2-d1E04-1-0105 | 801.356 | OH | A01 | a1 | B02 | b4 | C02 | c1 | D01 | d1 | E04 |
| 2-2-d1E04-1-0106 | 802.261 | OH | A01 | a1 | B04 | b1 | C02 | c1 | D01 | d1 | E04 |
| 2-2-d1E04-1-0107 | 802.261 | OH | A01 | a1 | B04 | b1 | C03 | c1 | D01 | d1 | E04 |
| 2-2-d1E04-1-0108 | 802.326 | OH | A02 | a1 | B01 | b1 | C17 | c1 | D01 | d1 | E04 |
| 2-2-d1E04-1-0109 | 803.256 | OH | A01 | a1 | B04 | b1 | C04 | c1 | D01 | d1 | E04 |
| 2-2-d1E04-1-0110 | 803.260 | OH | A01 | a1 | B05 | b1 | C01 | c1 | D01 | d1 | E04 |
| 2-2-d1E04-1-0111 | 803.302 | OH | A01 | a1 | B01 | b4 | C12 | c2 | D02 | d1 | E04 |
| 2-2-d1E04-1-0112 | 803.310 | OH | A02 | a1 | B02 | b3 | C09 | c1 | D01 | d1 | E04 |
| 2-2-d1E04-1-0113 | 803.335 | OH | A02 | a1 | B01 | b4 | C01 | c1 | D01 | d1 | E04 |
| 2-2-d1E04-1-0114 | 805.282 | OH | A01 | a1 | B01 | b4 | C13 | c2 | D02 | d1 | E04 |
| 2-2-d1E04-1-0115 | 805.326 | OH | A02 | a1 | B02 | b1 | C14 | c1 | D01 | d1 | E04 |
| 2-2-d1E04-1-0116 | 805.331 | OH | A01 | a1 | B03 | b4 | C02 | c1 | D01 | d1 | E04 |
| 2-2-d1E04-1-0117 | 807.285 | OH | A02 | a1 | B03 | b3 | C09 | c1 | D01 | d1 | E04 |
| 2-2-d1E04-1-0118 | 807.287 | OH | A02 | a1 | B01 | b1 | C18 | c1 | D01 | d1 | E04 |
| 2-2-d1E04-1-0119 | 807.312 | OH | A01 | a1 | B02 | b4 | C05 | c1 | D01 | d1 | E04 |
| 2-2-d1E04-1-0120 | 807.328 | OH | A01 | a1 | B05 | b4 | C12 | c1 | D01 | d1 | E04 |
| 2-2-d1E04-1-0121 | 808.217 | OH | A01 | a1 | B04 | b1 | C05 | c1 | D01 | d1 | E04 |
| 2-2-d1E04-1-0122 | 808.271 | OH | A02 | a1 | B02 | b3 | C10 | c1 | D01 | d1 | E04 |
| 2-2-d1E04-1-0123 | 809.230 | OH | A01 | a1 | B04 | b3 | C08 | c1 | D01 | d1 | E04 |
| 2-2-d1E04-1-0124 | 809.255 | OH | A01 | a1 | B01 | b1 | C02 | c2 | D02 | d1 | E04 |
| 2-2-d1E04-1-0125 | 809.255 | OH | A01 | a1 | B01 | b1 | C03 | c2 | D02 | d1 | E04 |
| 2-2-d1E04-1-0126 | 809.301 | OH | A02 | a1 | B03 | b1 | C14 | c1 | D01 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0127 | 809.307 | OH | A01 | a1 | B05 | b4 | C13 | c1 | D01 | d1 | E04 |
| 2-2-d1E04-1-0128 | 809.325 | OH | A02 | a1 | B02 | b2 | C02 | c1 | D01 | d1 | E04 |
| 2-2-d1E04-1-0129 | 809.325 | OH | A02 | a1 | B02 | b2 | C03 | c1 | D01 | d1 | E04 |
| 2-2-d1E04-1-0130 | 810.251 | OH | A01 | a1 | B01 | b1 | C04 | c2 | D02 | d1 | E04 |
| 2-2-d1E04-1-0131 | 810.320 | OH | A02 | a1 | B02 | b2 | C04 | c1 | D01 | d1 | E04 |
| 2-2-d1E04-1-0132 | 810.320 | OH | A02 | a1 | B02 | b2 | C06 | c1 | D01 | d1 | E04 |
| 2-2-d1E04-1-0133 | 810.320 | OH | A02 | a1 | B02 | b2 | C07 | c1 | D01 | d1 | E04 |
| 2-2-d1E04-1-0134 | 811.287 | OH | A01 | a1 | B03 | b4 | C05 | c1 | D01 | d1 | E04 |
| 2-2-d1E04-1-0135 | 812.246 | OH | A02 | a1 | B03 | b3 | C10 | c1 | D01 | d1 | E04 |
| 2-2-d1E04-1-0136 | 813.250 | OH | A01 | a1 | B02 | b1 | C01 | c2 | D02 | d1 | E04 |
| 2-2-d1E04-1-0137 | 813.281 | OH | A01 | a1 | B05 | b1 | C02 | c1 | D01 | d1 | E04 |
| 2-2-d1E04-1-0138 | 813.281 | OH | A01 | a1 | B05 | b1 | C03 | c1 | D01 | d1 | E04 |
| 2-2-d1E04-1-0139 | 813.300 | OH | A02 | a1 | B03 | b2 | C02 | c1 | D01 | d1 | E04 |
| 2-2-d1E04-1-0140 | 813.300 | OH | A02 | a1 | B03 | b2 | C03 | c1 | D01 | d1 | E04 |
| 2-2-d1E04-1-0141 | 813.356 | OH | A02 | a1 | B01 | b4 | C02 | c1 | D01 | d1 | E04 |
| 2-2-d1E04-1-0142 | 814.276 | OH | A01 | a1 | B05 | b1 | C04 | c1 | D01 | d1 | E04 |
| 2-2-d1E04-1-0143 | 814.295 | OH | A02 | a1 | B03 | b2 | C04 | c1 | D01 | d1 | E04 |
| 2-2-d1E04-1-0144 | 814.295 | OH | A02 | a1 | B03 | b2 | C06 | c1 | D01 | d1 | E04 |
| 2-2-d1E04-1-0145 | 814.295 | OH | A02 | a1 | B03 | b2 | C07 | c1 | D01 | d1 | E04 |
| 2-2-d1E04-1-0146 | 814.351 | OH | A02 | a1 | B02 | b1 | C15 | c1 | D01 | d1 | E04 |
| 2-2-d1E04-1-0147 | 815.212 | OH | A01 | a1 | B01 | b1 | C05 | c2 | D02 | d1 | E04 |
| 2-2-d1E04-1-0148 | 815.299 | OH | A01 | a1 | B02 | b3 | C11 | c1 | D01 | d1 | E04 |
| 2-2-d1E04-1-0149 | 815.347 | OH | A02 | a1 | B02 | b1 | C16 | c1 | D01 | d1 | E04 |
| 2-2-d1E04-1-0150 | 816.225 | OH | A01 | a1 | B01 | b3 | C08 | c2 | D02 | d1 | E04 |
| 2-2-d1E04-1-0151 | 816.342 | OH | A02 | a1 | B02 | b1 | C17 | c1 | D01 | d1 | E04 |
| 2-2-d1E04-1-0152 | 817.225 | OH | A01 | a1 | B03 | b1 | C01 | c2 | D02 | d1 | E04 |
| 2-2-d1E04-1-0153 | 817.318 | OH | A01 | a1 | B02 | b4 | C12 | c2 | D02 | d1 | E04 |
| 2-2-d1E04-1-0154 | 817.351 | OH | A02 | a1 | B02 | b4 | C01 | c1 | D01 | d1 | E04 |
| 2-2-d1E04-1-0155 | 818.256 | OH | A02 | a1 | B04 | b1 | C01 | c1 | D01 | d1 | E04 |
| 2-2-d1E04-1-0156 | 818.326 | OH | A02 | a1 | B03 | b1 | C15 | c1 | D01 | d1 | E04 |
| 2-2-d1E04-1-0157 | 819.237 | OH | A01 | a1 | B05 | b1 | C05 | c1 | D01 | d1 | E04 |
| 2-2-d1E04-1-0158 | 819.274 | OH | A01 | a1 | B03 | b3 | C11 | c1 | D01 | d1 | E04 |
| 2-2-d1E04-1-0159 | 819.297 | OH | A01 | a1 | B02 | b4 | C13 | c2 | D02 | d1 | E04 |
| 2-2-d1E04-1-0160 | 819.312 | OH | A02 | a1 | B01 | b4 | C05 | c1 | D01 | d1 | E04 |
| 2-2-d1E04-1-0161 | 819.321 | OH | A02 | a1 | B03 | b1 | C16 | c1 | D01 | d1 | E04 |
| 2-2-d1E04-1-0162 | 820.246 | OH | A01 | a1 | B04 | b3 | C09 | c1 | D01 | d1 | E04 |
| 2-2-d1E04-1-0163 | 820.250 | OH | A01 | a1 | B05 | b3 | C08 | c1 | D01 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0164 | 820.317 | OH | A02 | a1 | B03 | b1 | C17 | c1 | D01 | d1 | E04 |
| 2-2-d1E04-1-0165 | 821.293 | OH | A01 | a1 | B03 | b4 | C12 | c2 | D02 | d1 | E04 |
| 2-2-d1E04-1-0166 | 821.303 | OH | A02 | a1 | B02 | b1 | C18 | c1 | D01 | d1 | E04 |
| 2-2-d1E04-1-0167 | 821.326 | OH | A02 | a1 | B03 | b4 | C01 | c1 | D01 | d1 | E04 |
| 2-2-d1E04-1-0168 | 822.262 | OH | A01 | a1 | B04 | b1 | C14 | c1 | D01 | d1 | E04 |
| 2-2-d1E04-1-0169 | 822.323 | OH | A02 | a1 | B04 | b4 | C12 | c1 | D01 | d1 | E04 |
| 2-2-d1E04-1-0170 | 823.271 | OH | A01 | a1 | B02 | b1 | C02 | c2 | D02 | d1 | E04 |
| 2-2-d1E04-1-0171 | 823.271 | OH | A01 | a1 | B02 | b1 | C03 | c2 | D02 | d1 | E04 |
| 2-2-d1E04-1-0172 | 823.272 | OH | A01 | a1 | B03 | b4 | C13 | c2 | D02 | d1 | E04 |
| 2-2-d1E04-1-0173 | 824.266 | OH | A01 | a1 | B02 | b1 | C04 | c2 | D02 | d1 | E04 |
| 2-2-d1E04-1-0174 | 824.303 | OH | A02 | a1 | B04 | b4 | C13 | c1 | D01 | d1 | E04 |
| 2-2-d1E04-1-0175 | 825.207 | OH | A01 | a1 | B04 | b3 | C10 | c1 | D01 | d1 | E04 |
| 2-2-d1E04-1-0176 | 825.250 | OH | A02 | a1 | B01 | b1 | C01 | c2 | D02 | d1 | E04 |
| 2-2-d1E04-1-0177 | 825.278 | OH | A02 | a1 | B03 | b1 | C18 | c1 | D01 | d1 | E04 |
| 2-2-d1E04-1-0178 | 826.261 | OH | A01 | a1 | B04 | b2 | C02 | c1 | D01 | d1 | E04 |
| 2-2-d1E04-1-0179 | 826.261 | OH | A01 | a1 | B04 | b2 | C03 | c1 | D01 | d1 | E04 |
| 2-2-d1E04-1-0180 | 827.241 | OH | A01 | a1 | B01 | b3 | C09 | c2 | D02 | d1 | E04 |
| 2-2-d1E04-1-0181 | 827.246 | OH | A01 | a1 | B03 | b1 | C02 | c2 | D02 | d1 | E04 |
| 2-2-d1E04-1-0182 | 827.246 | OH | A01 | a1 | B03 | b1 | C03 | c2 | D02 | d1 | E04 |
| 2-2-d1E04-1-0183 | 827.256 | OH | A01 | a1 | B04 | b2 | C04 | c1 | D01 | d1 | E04 |
| 2-2-d1E04-1-0184 | 827.256 | OH | A01 | a1 | B04 | b2 | C06 | c1 | D01 | d1 | E04 |
| 2-2-d1E04-1-0185 | 827.256 | OH | A01 | a1 | B04 | b2 | C07 | c1 | D01 | d1 | E04 |
| 2-2-d1E04-1-0186 | 827.299 | OH | A02 | a1 | B01 | b3 | C11 | c1 | D01 | d1 | E04 |
| 2-2-d1E04-1-0187 | 827.372 | OH | A02 | a1 | B02 | b4 1 | C02 | c1 | D01 | d1 | E04 |
| 2-2-d1E04-1-0188 | 828.241 | OH | A01 | a1 | B03 | b1 | C04 | c2 | D02 | d1 | E04 |
| 2-2-d1E04-1-0189 | 828.276 | OH | A02 | a1 | B04 | b1 | C02 | c1 | D01 | d1 | E04 |
| 2-2-d1E04-1-0190 | 828.276 | OH | A02 | a1 | B04 | b1 | C03 | c1 | D01 | d1 | E04 |
| 2-2-d1E04-1-0191 | 829.227 | OH | A01 | a1 | B02 | b | COS | c2 | D02 | d1 | E04 |
| 2-2-dlE04-1-0192 | 829.256 | OH | A01 | a1 | B01 | b1 | C14 | c2 | D02 | d1 | E04 |
| 2-2-dlE04-1-0193 | 829.272 | OH | A02 | a1 | B04 | b1 | C04 | c1 | D01 | d1 | E04 |
| 2-2-d1E04-1-0194 | 829.276 | OH | A02 | a1 | B05 | b1 | C01 | c1 | D01 | d1 | E04 |
| 2-2-d1E04-1-0195 | 829.318 | OH | A02 | a1 | B01 | b4 | C12 | c2 | D02 | d1 | E04 |
| 2-2-d1E04-1-0196 | 829.362 | OH | A01 | a1 | B01 | b1 | C19 | c1 | D01 | d1 | E04 |
| 2-2-d1E04-1-0197 | 830.240 | OH | A01 | a1 | B02 | b3 | C08 | c2 | D02 | d1 | E04 |
| 2-2-d1E04-1-0198 | 831.266 | OH | A01 | a1 | B05 | b3 | C09 | c1 | D01 | d1 | E04 |
| 2-2-d1E04-1-0199 | 831.287 | OH | A01 | a1 | B04 | b1 | C15 | c1 | D01 | d1 | E04 |
| 2-2-dlE04-1-0200 | 831.297 | OH | A02 | a1 | B01 | b4 | C13 | c2 | D02 | d1 | E04 |
| 2-2-d1E04-1-0201 | 831.347 | OH | A02 | a1 | B03 | b4 | C02 | c1 | D01 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0202 | 832.202 | OH | A01 | a1 | B01 | b3 | C10 | c2 | D02 | d1 | E04 |
| 2-2-dlE04-1-0203 | 832.283 | OH | A01 | a1 | B04 | b1 | C16 | c1 | D01 | d1 | E04 |
| 2-2-dlE04-1-0204 | 833.202 | OH | A01 | a1 | B03 | b1 | C05 | c2 | D02 | d1 | E04 |
| 2-2-dlE04-1-0205 | 833.255 | OH | A01 | a1 | B01 | b2 | C02 | c2 | D02 | d1 | E04 |
| 2-2-d1E04-1-0206 | 833.255 | OH | A01 | a1 | B01 | b2 | C03 | c2 | D02 | d1 | E04 |
| 2-2-dlE04-1-0207 | 833.278 | OH | A01 | a1 | B04 | b1 | C17 | c1 | D01 | d1 | E04 |
| 2-2-d1E04-1-0208 | 833.282 | OH | A01 | a1 | B05 | b1 | C14 | c1 | D01 | d1 | E04 |
| 2-2-d1E04-1-0209 | 833.328 | OH | A02 | a1 | B02 | b4 | C05 | c1 | D01 | d1 | E04 |
| 2-2-d1E04-1-0210 | 833.343 | OH | A02 | a1 | B05 | b4 | C12 | c1 | D01 | d1 | E04 |
| 2-2-d1E04-1-0211 | 834.215 | OH | A01 | a1 | B03 | b3 | C08 | c2 | D02 | d1 | E04 |
| 2-2-dlE04-1-0212 | 834.233 | OH | A02 | a1 | B04 | b1 | C05 | c1 | D01 | d1 | E04 |
| 2-2-d1E04-1-0213 | 834.251 | OH | A01 | a1 | B01 | b2 | C04 | c2 | D02 | d1 | E04 |
| 2-2-d1E04-1-0214 | 834.251 | OH | A01 | a1 | B01 | b2 | C06 | c2 | D02 | d1 | E04 |
| 2-2-d1E04-1-0215 | 834.251 | OH | A01 | a1 | B01 | b2 | C07 | c2 | D02 | d1 | E04 |
| 2-2-d1E04-1-0216 | 834.287 | OH | A01 | a1 | B04 | b4 | C01 | c1 | D01 | d1 | E04 |
| 2-2-d1E04-1-0217 | 835.246 | OH | A02 | a1 | B04 | b3 | C08 | c1 | D01 | d1 | E04 |
| 2-2-d1E04-1-0218 | 835.271 | OH | A02 | a1 | B01 | b1 | C02 | c2 | D02 | d1 | E04 |
| 2-2-d1E04-1-0219 | 835.271 | OH | A02 | a1 | B01 | b1 | C03 | c2 | D02 | d1 | E04 |
| 2-2-d1E04-1-0220 | 835.323 | OH | A02 | a1 | B05 | b4 | C13 | c1 | D01 | d1 | E04 |
| 2-2-d1E04-1-0221 | 836.227 | OH | A01 | a1 | B05 | b3 | C10 | c1 | D01 | d1 | E04 |
| 2-2-dlE04-1-0222 | 836.266 | OH | A02 | a1 | B01 | b1 | C04 | c2 | D02 | d1 | E04 |
| 2-2-d1E04-1-0223 | 837.281 | OH | A01 | a1 | B05 | b2 | C02 | c1 | D01 | d1 | E04 |
| 2-2-dlE04-1-0224 | 837.281 | OH | A01 | a1 | B05 | b2 | C03 | c1 | D01 | d1 | E04 |
| 2-2-d1E04-1-0225 | 837.303 | OH | A02 | a1 | B03 | b4 | C05 | c1 | D01 | d1 | E04 |
| 2-2-dlE04-1-0226 | 838.239 | OH | A01 | a1 | B04 | b1 | C18 | c1 | D01 | d1 | E04 |
| 2-2-d1E04-1-0227 | 838.276 | OH | A01 | a1 | B05 | b2 | C04 | c1 | D01 | d1 | E04 |
| 2-2-dlE04-1-0228 | 838.276 | OH | A01 | a1 | B05 | b2 | C06 | c1 | D01 | d1 | E04 |
| 2-2-dlE04-1-0229 | 838.276 | OH | A01 | a1 | B05 | b2 | C07 | c1 | D01 | d1 | E04 |
| 2-2-d1E04-1-0230 | 838.282 | OH | A01 | a1 | B01 | b1 | C15 | c2 | D02 | d1 | E04 |
| 2-2-d1E04-1-0231 | 839.266 | OH | A02 | a1 | B02 | b1 | C01 | c2 | D02 | d1 | E04 |
| 2-2-d1E04-1-0232 | 839.277 | OH | A01 | a1 | B01 | b1 | C16 | c2 | D02 | d1 | E04 |
| 2-2-dlE04-1-0233 | 839.296 | OH | A02 | a1 | B05 | b1 | C02 | c1 | D01 | d1 | E04 |
| 2-2-d1E04-1-0234 | 839.296 | OH | A02 | a1 | B05 | b1 | C03 | c1 | D01 | d1 | E04 |
| 2-2-d1E04-1-0235 | 840.272 | OH | A01 | a1 | B01 | b1 | C17 | c2 | D02 | d1 | E04 |
| 2-2-d1E04-1-0236 | 840.292 | OH | A02 | a1 | B05 | b1 | C04 | c1 | D01 | d1 | E04 |
| 2-2-d1E04-1-0237 | 841.227 | OH | A02 | a1 | B01 | b1 | C05 | c2 | D02 | d1 | E04 |
| 2-2-d1E04-1-0238 | 841.256 | OH | A01 | a1 | B02 | b3 | C09 | c2 | D02 | d1 | E04 |
| 2-2-dlE04-1-0239 | 841.282 | OH | A01 | a1 | B01 | b4 | C01 | c2 | D02 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-dlE04-1-0240 | 841.315 | OH | A02 | a1 | B02 | b3 | C11 | c1 | D01 | d1 | E04 |
| 2-2-d1E04-1-0241 | 842.240 | OH | A02 | a1 | B01 | b3 | C08 | c2 | D02 | d1 | E04 |
| 2-2-dlE04-1-0242 | 842.307 | OH | A01 | a1 | B05 | b1 | C15 | c1 | D01 | d1 | E04 |
| 2-2-d1E04-1-0243 | 843.241 | OH | A02 | a1 | B03 | b1 | C01 | c2 | D02 | d1 | E04 |
| 2-2-dlE04-1-0244 | 843.272 | OH | A01 | a1 | B02 | b1 | C14 | c2 | D02 | d1 | E04 |
| 2-2-dlE04-1-0245 | 843.303 | OH | A01 | a1 | B05 | b1 | C16 | c1 | D01 | d1 | E04 |
| 2-2-d1E04-1-0246 | 843.334 | OH | A02 | a1 | B02 | b4 | C12 | c2 | D02 | d1 | E04 |
| 2-2-dlE04-1-0247 | 843.378 | OH | A01 | a1 | B02 | b1 | C19 | c1 | D01 | d1 | E04 |
| 2-2-d1E04-1-0248 | 844.298 | OH | A01 | a1 | B05 | b1 | C17 | c1 | D01 | d1 | E04 |
| 2-2-dlE04-1-0249 | 844.308 | OH | A01 | a1 | B04 | b4 | C02 | c1 | D01 | d1 | E04 |
| 2-2-dlE04-1-0250 | 845.231 | OH | A01 | a1 | B03 | b3 | C09 | c2 | D02 | d1 | E04 |
| 2-2-d1E04-1-0251 | 845.234 | OH | A01 | a1 | B01 | b1 | C18 | c2 | D02 | d1 | E04 |
| 2-2-dlE04-1-0252 | 845.253 | OH | A02 | a1 | B05 | b1 | C05 | c1 | D01 | d1 | E04 |
| 2-2-d1E04-1-0253 | 845.289 | OH | A02 | a1 | B03 | b3 | C11 | c1 | D01 | d1 | E04 |
| 2-2-dlE04-1-0254 | 845.307 | OH | A01 | a1 | B05 | b4 | C01 | c1 | D01 | d1 | E04 |
| 2-2-d1E04-1-0255 | 845.313 | OH | A02 | a1 | B02 | b4 | C13 | c2 | D02 | d1 | E04 |
| 2-2-d1E04-1-0256 | 846.218 | OH | A01 | a1 | B02 | b3 | C10 | c2 | D02 | d1 | E04 |
| 2-2-dlE04-1-0257 | 846.262 | OH | A02 | a1 | B04 | b3 | C09 | c1 | D01 | d1 | E04 |
| 2-2-d1E04-1-0258 | 846.266 | OH | A02 | a1 | B05 | b3 | C08 | c1 | D01 | d1 | E04 |
| 2-2-dlE04-1-0259 | 847.247 | OH | A01 | a1 | B03 | b1 | C14 | c2 | D02 | d1 | E04 |
| 2-2-dlE04-1-0260 | 847.271 | OH | A01 | a1 | B02 | b2 | C02 | c2 | D02 | d1 | E04 |
| 2-2-d1E04-1-0261 | 847.271 | OH | A01 | a1 | B02 | b2 | C03 | c2 | D02 | d1 | E04 |
| 2-2-d1E04-1-0262 | 847.308 | OH | A02 | a1 | B03 | b4 | C12 | c2 | D02 | d1 | E04 |
| 2-2-d1E04-1-0263 | 847.353 | OH | A01 | a1 | B03 | b1 | C19 | c1 | D01 | d1 | E04 |
| 2-2-dlE04-1-0264 | 848.266 | OH | A01 | a1 | B02 | b2 | C04 | c2 | D02 | d1 | E04 |
| 2-2-d1E04-1-0265 | 848.266 | OH | A01 | a1 | B02 | b2 | C06 | c2 | D02 | d1 | E04 |
| 2-2-dlE04-1-0266 | 848.266 | OH | A01 | a1 | B02 | b2 | C07 | c2 | D02 | d1 | E04 |
| 2-2-d1E04-1-0267 | 848.277 | OH | A02 | a1 | B04 | b1 | C14 | c1 | D01 | d1 | E04 |
| 2-2-dlE04-1-0268 | 849.259 | OH | A01 | a1 | B05 | b1 | C18 | c1 | D01 | d1 | E04 |
| 2-2-dlE04-1-0269 | 849.287 | OH | A02 | a1 | B02 | b1 | C02 | c2 | D02 | d1 | E04 |
| 2-2-d1E04-1-0270 | 849.287 | OH | A02 | a1 | B02 | b1 | C03 | c2 | D02 | d1 | E04 |
| 2-2-d1E04-1-0271 | 849.288 | OH | A02 | a1 | B03 | b4 | C13 | c2 | D02 | d1 | E04 |
| 2-2-d1E04-1-0272 | 850.192 | OH | A01 | a1 | B03 | b3 | C10 | c2 | D02 | d1 | E04 |
| 2-2-dlE04-1-0273 | 850.264 | OH | A01 | a1 | B04 | b4 | C05 | c1 | D01 | d1 | E04 |
| 2-2-d1E04-1-0274 | 850.282 | OH | A02 | a1 | B02 | b1 | C04 | c2 | D02 | d1 | E04 |
| 2-2-dlE04-1-0275 | 851.223 | OH | A02 | a1 | B04 | b3 | C10 | c1 | D01 | d1 | E04 |
| 2-2-d1E04-1-0276 | 851.246 | OH | A01 | a1 | B03 | b2 | C02 | c2 | D02 | d1 | E04 |
| 2-2-d1E04-1-0277 | 851.246 | OH | A01 | a1 | B03 | b2 | C03 | c2 | D02 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-dlE04-1-0278 | 851.302 | OH | A01 | a1 | B01 | b4 | C02 | c2 | D02 | d1 | E04 |
| 2-2-d1E04-1-0279 | 852.241 | OH | A01 | a1 | B03 | b2 | C04 | c2 | D02 | d1 | E04 |
| 2-2-dlE04-1-0280 | 852.241 | OH | A01 | a1 | B03 | b2 | C06 | c2 | D02 | d1 | E04 |
| 2-2-d1E04-1-0281 | 852.241 | OH | A01 | a1 | B03 | b2 | C07 | c2 | D02 | d1 | E04 |
| 2-2-d1E04-1-0282 | 852.276 | OH | A02 | a1 | B04 | b2 | C02 | c1 | D01 | d1 | E04 |
| 2-2-dlE04-1-0283 | 852.276 | OH | A02 | a1 | B04 | b2 | C03 | c1 | D01 | d1 | E04 |
| 2-2-d1E04-1-0284 | 852.298 | OH | A01 | a1 | B02 | b1 | C15 | c2 | D02 | d1 | E04 |
| 2-2-dlE04-1-0285 | 853.256 | OH | A02 | a1 | B01 | b3 | C09 | c2 | D02 | d1 | E04 |
| 2-2-d1E04-1-0286 | 853.262 | OH | A02 | a1 | B03 | b1 | C02 | c2 | D02 | d1 | E04 |
| 2-2-dlE04-1-0287 | 853.262 | OH | A02 | a1 | B03 | b1 | C03 | c2 | D02 | d1 | E04 |
| 2-2-d1E04-1-0288 | 853.272 | OH | A02 | a1 | B04 | b2 | C04 | c1 | D01 | d1 | E04 |
| 2-2-dlE04-1-0289 | 853.272 | OH | A02 | a1 | B04 | b2 | C06 | c1 | D01 | d1 | E04 |
| 2-2-d1E04-1-0290 | 853.272 | OH | A02 | a1 | B04 | b2 | C07 | c1 | D01 | d1 | E04 |
| 2-2-d1E04-1-0291 | 853.293 | OH | A01 | a1 | B02 | b1 | C16 | c2 | D02 | d1 | E04 |
| 2-2-dlE04-1-0292 | 854.257 | OH | A02 | a1 | B03 | b1 | C04 | c2 | D02 | d1 | E04 |
| 2-2-dlE04-1-0293 | 854.288 | OH | A01 | a1 | B02 | b1 | C17 | c2 | D02 | d1 | E04 |
| 2-2-dlE04-1-0294 | 855.243 | OH | A02 | a1 | B02 | b1 | C05 | c2 | D02 | d1 | E04 |
| 2-2-d1E04-1-0295 | 855.272 | OH | A02 | a1 | B01 | b1 | C14 | c2 | D02 | d1 | E04 |
| 2-2-dlE04-1-0296 | 855.297 | OH | A01 | a1 | B02 | b4 | C01 | c2 | D02 | d1 | E04 |
| 2-2-dlE04-1-0297 | 855.328 | OH | A01 | a1 | B05 | b4 | C02 | c1 | D01 | d1 | E04 |
| 2-2-d1E04-1-0298 | 855.378 | OH | A02 | a1 | B01 | b1 | C19 | c1 | D01 | d1 | E04 |
| 2-2-dlE04-1-0299 | 856.202 | OH | A01 | a1 | B04 | b1 | C01 | c2 | D02 | d1 | E04 |
| 2-2-d1E04-1-0300 | 856.256 | OH | A02 | a1 | B02 | b3 | C08 | c2 | D02 | d1 | E04 |
| 2-2-d1E04-1-0301 | 856.272 | OH | A01 | a1 | B03 | b1 | C15 | c2 | D02 | d1 | E04 |
| 2-2-d1E04-1-0302 | 857.259 | OH | A01 | a1 | B01 | b4 | C05 | c2 | D02 | d1 | E04 |
| 2-2-d1E04-1-0303 | 857.268 | OH | A01 | a1 | B03 | b1 | C16 | c2 | D02 | d1 | E04 |
| 2-2-dlE04-1-0304 | 857.282 | OH | A02 | a1 | B05 | b3 | C09 | c1 | D01 | d1 | E04 |
| 2-2-d1E04-1-0305 | 857.303 | OH | A02 | a1 | B04 | b1 | C15 | c1 | D01 | d1 | E04 |
| 2-2-dlE04-1-0306 | 858.218 | OH | A02 | a1 | B01 | b3 | C10 | c2 | D02 | d1 | E04 |
| 2-2-d1E04-1-0307 | 858.251 | OH | A01 | a1 | B04 | b3 | C11 | c1 | D01 | d1 | E04 |
| 2-2-d1E04-1-0308 | 858.263 | OH | A01 | a1 | B03 | b1 | C17 | c2 | D02 | d1 | E04 |
| 2-2-d1E04-1-0309 | 858.298 | OH | A02 | a1 | B04 | b1 | C16 | c1 | D01 | d1 | E04 |
| 2-2-d1E04-1-0310 | 859.218 | OH | A02 | a1 | B03 | b1 | C05 | c2 | D02 | d1 | E04 |
| 2-2-d1E04-1-0311 | 859.249 | OH | A01 | a1 | B02 | b1 | C18 | c2 | D02 | d1 | E04 |
| 2-2-d1E04-1-0312 | 859.271 | OH | A02 | a1 | B01 | b2 | C02 | c2 | D02 | d1 | E04 |
| 2-2-d1E04-1-0313 | 859.271 | OH | A02 | a1 | B01 | b2 | C03 | c2 | D02 | d1 | E04 |
| 2-2-d1E04-1-0314 | 859.272 | OH | A01 | a1 | B03 | b4 | C01 | c2 | D02 | d1 | E04 |
| 2-2-d1E04-1-0315 | 859.293 | OH | A02 | a1 | B04 | b1 | C17 | c1 | D01 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0316 | 859.298 | OH | A02 | a1 | B05 | b1 | C14 | c1 | D01 | d1 | E04 |
| 2-2-d1E04-1-0317 | 860.231 | OH | A02 | a1 | B03 | b3 | C08 | c2 | D02 | d1 | E04 |
| 2-2-d1E04-1-0318 | 860.266 | OH | A02 | a1 | B01 | b2 | C04 | c2 | D02 | d1 | E04 |
| 2-2-d1E04-1-0319 | 860.266 | OH | A02 | a1 | B01 | b2 | C06 | c2 | D02 | d1 | E04 |
| 2-2-d1E04-1-0320 | 860.266 | OH | A02 | a1 | B01 | b2 | C07 | c2 | D02 | d1 | E04 |
| 2-2-d1E04-1-0321 | 860.270 | OH | A01 | a1 | B04 | b4 | C12 | c2 | D02 | d1 | E04 |
| 2-2-d1E04-1-0322 | 860.303 | OH | A02 | a1 | B04 | b4 | C01 | c1 | D01 | d1 | E04 |
| 2-2-d1E04-1-0323 | 861.284 | OH | A01 | a1 | B05 | b4 | C05 | c1 | D01 | d1 | E04 |
| 2-2-d1E04-1-0324 | 862.243 | OH | A02 | a1 | B05 | b3 | C10 | c1 | D01 | d1 | E04 |
| 2-2-d1E04-1-0325 | 862.249 | OH | A01 | a1 | B04 | b4 | C13 | c2 | D02 | d1 | E04 |
| 2-2-d1E04-1-0326 | 863.224 | OH | A01 | a1 | B03 | b1 | C18 | c2 | D02 | d1 | E04 |
| 2-2-d1E04-1-0327 | 863.296 | OH | A02 | a1 | B05 | b2 | C02 | c1 | D01 | d1 | E04 |
| 2-2-d1E04-1-0328 | 863.296 | OH | A02 | a1 | B05 | b2 | C03 | c1 | D01 | d1 | E04 |
| 2-2-d1E04-1-0329 | 864.255 | OH | A02 | a1 | B04 | b1 | C18 | c1 | D01 | d1 | E04 |
| 2-2-d1E04-1-0330 | 864.292 | OH | A02 | a1 | B05 | b2 | C04 | c1 | D01 | d1 | E04 |
| 2-2-d1E04-1-0331 | 864.292 | OH | A02 | a1 | B05 | b2 | C06 | c1 | D01 | d1 | E04 |
| 2-2-d1E04-1-0332 | 864.292 | OH | A02 | a1 | B05 | b2 | C07 | c1 | D01 | d1 | E04 |
| 2-2-d1E04-1-0333 | 864.298 | OH | A02 | a1 | B01 | b1 | C15 | c2 | D02 | d1 | E04 |
| 2-2-dlE04-1-0334 | 865.245 | OH | A01 | a1 | B01 | b3 | C11 | c2 | D02 | d1 | E04 |
| 2-2-d1E04-1-0335 | 865.293 | OH | A02 | a1 | B01 | b1 | C16 | c2 | D02 | d1 | E04 |
| 2-2-dlE04-1-0336 | 865.318 | OH | A01 | a1 | B02 | b4 | C02 | c2 | D02 | d1 | E04 |
| 2-2-d1E04-1-0337 | 866.223 | OH | A01 | a1 | B04 | b1 | C02 | c2 | D02 | d1 | E04 |
| 2-2-d1E04-1-0338 | 866.223 | OH | A01 | a1 | B04 | b1 | C03 | c2 | D02 | d1 | E04 |
| 2-2-dlE04-1-0339 | 866.288 | OH | A02 | a1 | B01 | b1 | C17 | c2 | D02 | d1 | E04 |
| 2-2-d1E04-1-0340 | 867.218 | OH | A01 | a1 | B04 | b1 | C04 | c2 | D02 | d1 | E04 |
| 2-2-d1E04-1-0341 | 867.222 | OH | A01 | a1 | B05 | b1 | C01 | c2 | D02 | d1 | E04 |
| 2-2-d1E04-1-0342 | 867.272 | OH | A02 | a1 | B02 | b3 | C09 | c2 | D02 | d1 | E04 |
| 2-2-dlE04-1-0343 | 867.297 | OH | A02 | a1 | B01 | b4 | C01 | c2 | D02 | d1 | E04 |
| 2-2-dlE04-1-0344 | 868.323 | OH | A02 | a1 | B05 | b1 | C15 | c1 | D01 | d1 | E04 |
| 2-2-d1E04-1-0345 | 869.271 | OH | A01 | a1 | B05 | b3 | C11 | c1 | D01 | d1 | E04 |
| 2-2-dlE04-1-0346 | 869.288 | OH | A02 | a1 | B02 | b1 | C14 | c2 | D02 | d1 | E04 |
| 2-2-d1E04-1-0347 | 869.293 | OH | A01 | a1 | B03 | b4 | C02 | c2 | D02 | d1 | E04 |
| 2-2-dlE04-1-0348 | 869.318 | OH | A02 | a1 | B05 | b1 | C16 | c1 | D01 | d1 | E04 |
| 2-2-d1E04-1-0349 | 869.393 | OH | A02 | a1 | B02 | b1 | C19 | c1 | D01 | d1 | E04 |
| 2-2-d1E04-1-0350 | 870.313 | OH | A02 | a1 | B05 | b1 | C17 | c1 | D01 | d1 | E04 |
| 2-2-d1E04-1-0351 | 870.323 | OH | A02 | a1 | B04 | b4 | C02 | c1 | D01 | d1 | E04 |
| 2-2-d1E04-1-0352 | 871.247 | OH | A02 | a1 | B03 | b3 | C09 | c2 | D02 | d1 | E04 |
| 2-2-dlE04-1-0353 | 871.249 | OH | A02 | a1 | B01 | b1 | C18 | c2 | D02 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0354 | 871.274 | OH | A01 | a1 | B02 | b4 | C05 | c2 | D02 | d1 | E04 |
| 2-2-d1E04-1-0355 | 871.290 | OH | A01 | a1 | B05 | b4 | C12 | c2 | D02 | d1 | E04 |
| 2-2-d1E04-1-0356 | 871.323 | OH | A02 | a1 | B05 | b4 | C01 | c1 | D01 | d1 | E04 |
| 2-2-d1E04-1-0357 | 872.179 | OH | A01 | a1 | B04 | b1 | C05 | c2 | D02 | d1 | E04 |
| 2-2-d1E04-1-0358 | 872.233 | OH | A02 | a1 | B02 | b3 | C10 | c2 | D02 | d1 | E04 |
| 2-2-d1E04-1-0359 | 873.192 | OH | A01 | a1 | B04 | b3 | C08 | c2 | D02 | d1 | E04 |
| 2-2-d1E04-1-0360 | 873.263 | OH | A02 | a1 | B03 | b1 | C14 | c2 | D02 | d1 | E04 |
| 2-2-d1E04-1-0361 | 873.269 | OH | A01 | a1 | B05 | b4 | C13 | c2 | D02 | d1 | E04 |
| 2-2-d1E04-1-0362 | 873.287 | OH | A02 | a1 | B02 | b2 | C02 | c2 | D02 | d1 | E04 |
| 2-2-dlE04-1-0363 | 873.287 | OH | A02 | a1 | B02 | b2 | C03 | c2 | D02 | d1 | E04 |
| 2-2-d1E04-1-0364 | 873.368 | OH | A02 | a1 | B03 | b1 | C19 | c1 | D01 | d1 | E04 |
| 2-2-d1E04-1-0365 | 874.282 | OH | A02 | a1 | B02 | b2 | C04 | c2 | D02 | d1 | E04 |
| 2-2-d1E04-1-0366 | 874.282 | OH | A02 | a1 | B02 | b2 | C06 | c2 | D02 | d1 | E04 |
| 2-2-d1E04-1-0367 | 874.282 | OH | A02 | a1 | B02 | b2 | C07 | c2 | D02 | d1 | E04 |
| 2-2-d1E04-1-0368 | 875.249 | OH | A01 | a1 | B03 | b4 | C05 | c2 | D02 | d1 | E04 |
| 2-2-d1E04-1-0369 | 875.275 | OH | A02 | a1 | B05 | b1 | C18 | c1 | D01 | d1 | E04 |
| 2-2-d1E04-1-0370 | 876.208 | OH | A02 | a1 | B03 | b3 | C10 | c2 | D02 | d1 | E04 |
| 2-2-d1E04-1-0371 | 876.280 | OH | A02 | a1 | B04 | b4 | C05 | c1 | D01 | d1 | E04 |
| 2-2-d1E04-1-0372 | 877.243 | OH | A01 | a1 | B05 | b1 | C02 | c2 | D02 | d1 | E04 |
| 2-2-d1E04-1-0373 | 877.243 | OH | A01 | a1 | B05 | b1 | C03 | c2 | D02 | d1 | E04 |
| 2-2-d1E04-1-0374 | 877.262 | OH | A02 | a1 | B03 | b2 | C02 | c2 | D02 | d1 | E04 |
| 2-2-d1E04-1-0375 | 877.262 | OH | A02 | a1 | B03 | b2 | C03 | c2 | D02 | d1 | E04 |
| 2-2-d1E04-1-0376 | 877.318 | OH | A02 | a1 | B01 | b4 | C02 | c2 | D02 | d1 | E04 |
| 2-2-d1E04-1-0377 | 878.238 | OH | A01 | a1 | B05 | b1 | C04 | c2 | D02 | d1 | E04 |
| 2-2-d1E04-1-0378 | 878.257 | OH | A02 | a1 | B03 | b2 | C04 | c2 | D02 | d1 | E04 |
| 2-2-d1E04-1-0379 | 878.257 | OH | A02 | a1 | B03 | b2 | C06 | c2 | D02 | d1 | E04 |
| 2-2-d1E04-1-0380 | 878.257 | OH | A02 | a1 | B03 | b2 | C07 | c2 | D02 | d1 | E04 |
| 2-2-d1E04-1-0381 | 878.313 | OH | A02 | a1 | B02 | b1 | C15 | c2 | D02 | d1 | E04 |
| 2-2-d1E04-1-0382 | 879.261 | OH | A01 | a1 | B02 | b3 | C11 | c2 | D02 | d1 | E04 |
| 2-2-d1E04-1-0383 | 879.308 | OH | A02 | a1 | B02 | b1 | C16 | c2 | D02 | d1 | E04 |
| 2-2-d1E04-1-0384 | 880.304 | OH | A02 | a1 | B02 | b1 | C17 | c2 | D02 | d1 | E04 |
| 2-2-d1E04-1-0385 | 881.313 | OH | A02 | a1 | B02 | b4 | C01 | c2 | D02 | d1 | E04 |
| 2-2-d1E04-1-0386 | 881.343 | OH | A02 | a1 | B05 | b4 | C02 | c1 | D01 | d1 | E04 |
| 2-2-d1E04-1-0387 | 882.218 | OH | A02 | a1 | B04 | b1 | C01 | c2 | D02 | d1 | E04 |
| 2-2-d1E04-1-0388 | 882.288 | OH | A02 | a1 | B03 | b1 | C15 | c2 | D02 | d1 | E04 |
| 2-2-d1E04-1-0389 | 883.199 | OH | A01 | a1 | B05 | b1 | C05 | c2 | D02 | d1 | E04 |
| 2-2-d1E04-1-0390 | 883.236 | OH | A01 | a1 | B03 | b3 | C11 | c2 | D02 | d1 | E04 |
| 2-2-d1E04-1-0391 | 883.274 | OH | A02 | a1 | B01 | b4 | C05 | c2 | D02 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0392 | 883.283 | OH | A02 | a1 | B03 | b1 | C16 | c2 | D02 | d1 | E04 |
| 2-2-d1E04-1-0393 | 884.208 | OH | A01 | a1 | B04 | b3 | C09 | c2 | D02 | d1 | E04 |
| 2-2-d1E04-1-0394 | 884.212 | OH | A01 | a1 | B05 | b3 | C08 | c2 | D02 | d1 | E04 |
| 2-2-d1E04-1-0395 | 884.266 | OH | A02 | a1 | B04 | b3 | C11 | c1 | D01 | d1 | E04 |
| 2-2-d1E04-1-0396 | 884.279 | OH | A02 | a1 | B03 | b1 | C17 | c2 | D02 | d1 | E04 |
| 2-2-d1E04-1-0397 | 885.265 | OH | A02 | a1 | B02 | b1 | C18 | c2 | D02 | d1 | E04 |
| 2-2-d1E04-1-0398 | 885.288 | OH | A02 | a1 | B03 | b4 | C01 | c2 | D02 | d1 | E04 |
| 2-2-d1E04-1-0399 | 886.224 | OH | A01 | a1 | B04 | b1 | C14 | c2 | D02 | d1 | E04 |
| 2-2-d1E04-1-0400 | 886.285 | OH | A02 | a1 | B04 | b4 | C12 | c2 | D02 | d1 | E04 |
| 2-2-d1E04-1-0401 | 886.329 | OH | A01 | a1 | B04 | b1 | C19 | c1 | D01 | d1 | E04 |
| 2-2-d1E04-1-0402 | 887.300 | OH | A02 | a1 | B05 | b4 | C05 | c1 | D01 | d1 | E04 |
| 2-2-d1E04-1-0403 | 888.264 | OH | A02 | a1 | B04 | b4 | C13 | c2 | D02 | d1 | E04 |
| 2-2-d1E04-1-0404 | 889.169 | OH | A01 | a1 | B04 | b3 | C10 | c2 | D02 | d1 | E04 |
| 2-2-d1E04-1-0405 | 889.240 | OH | A02 | a1 | B03 | b1 | C18 | c2 | D02 | d1 | E04 |
| 2-2-d1E04-1-0406 | 890.223 | OH | A01 | a1 | B04 | b2 | C02 | c2 | D02 | d1 | E04 |
| 2-2-d1E04-1-0407 | 890.223 | OH | A01 | a1 | B04 | b2 | C03 | c2 | D02 | d1 | E04 |
| 2-2-d1E04-1-0408 | 891.218 | OH | A01 | a1 | B04 | b2 | C04 | c2 | D02 | d1 | E04 |
| 2-2-d1E04-1-0409 | 891.218 | OH | A01 | a1 | B04 | b2 | C06 | c2 | D02 | d1 | E04 |
| 2-2-d1E04-1-0410 | 891.218 | OH | A01 | a1 | B04 | b2 | C07 | c2 | D02 | d1 | E04 |
| 2-2-d1E04-1-0411 | 891.261 | OH | A02 | a1 | B01 | b3 | C11 | c2 | D02 | d1 | E04 |
| 2-2-d1E04-1-0412 | 891.334 | OH | A02 | a1 | B02 | b4 | C02 | c2 | D02 | d1 | E04 |
| 2-2-d1E04-1-0413 | 892.238 | OH | A02 | a1 | B04 | b1 | C02 | c2 | D02 | d1 | E04 |
| 2-2-d1E04-1-0414 | 892.238 | OH | A02 | a1 | B04 | b1 | C03 | c2 | D02 | d1 | E04 |
| 2-2-d1E04-1-0415 | 893.234 | OH | A02 | a1 | B04 | b1 | C04 | c2 | D02 | d1 | E04 |
| 2-2-d1E04-1-0416 | 893.238 | OH | A02 | a1 | B05 | b1 | C01 | c2 | D02 | d1 | E04 |
| 2-2-d1E04-1-0417 | 893.324 | OH | A01 | a1 | B01 | b1 | C19 | c2 | D02 | d1 | E04 |
| 2-2-d1E04-1-0418 | 895.228 | OH | A01 | a1 | B05 | b3 | C09 | c2 | D02 | d1 | E04 |
| 2-2-d1E04-1-0419 | 895.249 | OH | A01 | a1 | B04 | b1 | C15 | c2 | D02 | d1 | E04 |
| 2-2-d1E04-1-0420 | 895.286 | OH | A02 | a1 | B05 | b3 | C11 | c1 | D01 | d1 | E04 |
| 2-2-d1E04-1-0421 | 895.308 | OH | A02 | a1 | B03 | b4 | C02 | c2 | D02 | d1 | E04 |
| 2-2-d1E04-1-0422 | 896.244 | OH | A01 | a1 | B04 | b1 | C16 | c2 | D02 | d1 | E04 |
| 2-2-d1E04-1-0423 | 897.240 | OH | A01 | a1 | B04 | b1 | C17 | c2 | D02 | d1 | E04 |
| 2-2-d1E04-1-0424 | 897.244 | OH | A01 | a1 | B05 | b1 | C14 | c2 | D02 | d1 | E04 |
| 2-2-d1E04-1-0425 | 897.290 | OH | A02 | a1 | B02 | b4 | C05 | c2 | D02 | d1 | E04 |
| 2-2-d1E04-1-0426 | 897.305 | OH | A02 | a1 | B05 | b4 | C12 | c2 | D02 | d1 | E04 |
| 2-2-d1E04-1-0427 | 897.350 | OH | A01 | a1 | B05 | b1 | C19 | c1 | D01 | d1 | E04 |
| 2-2-d1E04-1-0428 | 898.195 | OH | A02 | a1 | B04 | b1 | C05 | c2 | D02 | d1 | E04 |
| 2-2-d1E04-1-0429 | 898.249 | OH | A01 | a1 | B04 | b4 | C01 | c2 | D02 | d1 | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0430 | 899.208 | OH | A02 | a1 | B04 | b3 | C08 | c2 | D02 | d1 | E04 |
| 2-2-d1E04-1-0431 | 899.285 | OH | A02 | a1 | B05 | b4 | C13 | c2 | D02 | d1 | E04 |
| 2-2-d1E04-1-0432 | 900.189 | OH | A01 | a1 | B05 | b3 | C10 | c2 | D02 | d1 | E04 |
| 2-2-d1E04-1-0433 | 901.243 | OH | A01 | a1 | B05 | b2 | C02 | c2 | D02 | d1 | E04 |
| 2-2-d1E04-1-0434 | 901.243 | OH | A01 | a1 | B05 | b2 | C03 | c2 | D02 | d1 | E04 |
| 2-2-d1E04-1-0435 | 901.265 | OH | A02 | a1 | B03 | b4 | C05 | c2 | D02 | d1 | E04 |
| 2-2-d1E04-1-0436 | 902.201 | OH | A01 | a1 | B04 | b1 | C18 | c2 | D02 | d1 | E04 |
| 2-2-d1E04-1-0437 | 902.238 | OH | A01 | a1 | B05 | b2 | C04 | c2 | D02 | d1 | E04 |
| 2-2-d1E04-1-0438 | 902.238 | OH | A01 | a1 | B05 | b2 | C06 | c2 | D02 | d1 | E04 |
| 2-2-d1E04-1-0439 | 902.238 | OH | A01 | a1 | B05 | b2 | C07 | c2 | D02 | d1 | E04 |
| 2-2-d1E04-1-0440 | 903.258 | OH | A02 | a1 | B05 | b1 | C02 | c2 | D02 | d1 | E04 |
| 2-2-d1E04-1-0441 | 903.258 | OH | A02 | a1 | B05 | b1 | C03 | c2 | D02 | d1 | E04 |
| 2-2-d1E04-1-0442 | 904.254 | OH | A02 | a1 | B05 | b1 | C04 | c2 | D02 | d1 | E04 |
| 2-2-d1E04-1-0443 | 905.276 | OH | A02 | a1 | B02 | b3 | C11 | c2 | D02 | d1 | E04 |
| 2-2-d1E04-1-0444 | 906.269 | OH | A01 | a1 | B05 | b1 | C15 | c2 | D02 | d1 | E04 |
| 2-2-d1E04-1-0445 | 907.264 | OH | A01 | a1 | B05 | b1 | C16 | c2 | D02 | d1 | E04 |
| 2-2-d1E04-1-0446 | 907.340 | OH | A01 | a1 | B02 | b1 | C19 | c2 | D02 | d1 | E04 |
| 2-2-d1E04-1-0447 | 908.260 | OH | A01 | a1 | B05 | b1 | C17 | c2 | D02 | d1 | E04 |
| 2-2-d1E04-1-0448 | 908.270 | OH | A01 | a1 | B04 | b4 | C02 | c2 | D02 | d1 | E04 |
| 2-2-d1E04-1-0449 | 909.215 | OH | A02 | a1 | B05 | b1 | C05 | c2 | D02 | dl | E04 |
| 2-2-d1E04-1-0450 | 909.251 | OH | A02 | a1 | B03 | b3 | C11 | c2 | D02 | dl | E04 |
| 2-2-d1E04-1-0451 | 909.269 | OH | A01 | a1 | B05 | b4 | C01 | c2 | D02 | dl | E04 |
| 2-2-d1E04-1-0452 | 910.224 | OH | A02 | a1 | B04 | b3 | C09 | c2 | D02 | dl | E04 |
| 2-2-d1E04-1-0453 | 910.228 | OH | A02 | a1 | B05 | b3 | C08 | c2 | D02 | dl | E04 |
| 2-2-d1E04-1-0454 | 911.315 | OH | A01 | a1 | B03 | b1 | C19 | c2 | D02 | dl | E04 |
| 2-2-d1E04-1-0455 | 912.239 | OH | A02 | a1 | B04 | b1 | C14 | c2 | D02 | dl | E04 |
| 2-2-d1E04-1-0456 | 912.345 | OH | A02 | a1 | B04 | b1 | C19 | c1 | D01 | dl | E04 |
| 2-2-d1E04-1-0457 | 913.221 | OH | A01 | a1 | B05 | b1 | C18 | c2 | D02 | dl | E04 |
| 2-2-d1E04-1-0458 | 914.226 | OH | A01 | a1 | B04 | b4 | C05 | c2 | D02 | dl | E04 |
| 2-2-d1E04-1-0459 | 915.185 | OH | A02 | a1 | B04 | b3 | C10 | c2 | D02 | dl | E04 |
| 2-2-d1E04-1-0460 | 916.238 | OH | A02 | a1 | B04 | b2 | C02 | c2 | D02 | dl | E04 |
| 2-2-d1E04-1-0461 | 916.238 | OH | A02 | a1 | B04 | b2 | C03 | c2 | D02 | dl | E04 |
| 2-2-d1E04-1-0462 | 917.234 | OH | A02 | a1 | B04 | b2 | C04 | c2 | D02 | dl | E04 |
| 2-2-d1E04-1-0463 | 917.234 | OH | A02 | a1 | B04 | b2 | C06 | c2 | D02 | dl | E04 |
| 2-2-dlE04-1-0464 | 917.234 | OH | A02 | a1 | B04 | b2 | C07 | c2 | D02 | dl | E04 |
| 2-2-d1E04-1-0465 | 919.290 | OH | A01 | a1 | B05 | b4 | C02 | c2 | D02 | dl | E04 |
| 2-2-dlE04-1-0466 | 919.340 | OH | A02 | a1 | B01 | b1 | C19 | c2 | D02 | dl | E04 |

(continued)

| [Table 9-5-5] Compound that may be contained in mixture 2-2-d1E04-1 | | | | | | | | | | | |
|---|---|---|---|---|---|---|---|---|---|---|---|
| ID | Exact Mass | | A | a | B | b | C | c | D | d | E |
| 2-2-d1E04-1-0467 | 921.244 | OH | A02 | a1 | B05 | b3 | C09 | c2 | D02 | dl | E04 |
| 2-2-d1E04-1-0468 | 921.265 | OH | A02 | a1 | B04 | b1 | C15 | c2 | D02 | dl | E04 |
| 2-2-d1E04-1-0469 | 922.212 | OH | A01 | a1 | B04 | b3 | C11 | c2 | D02 | dl | E04 |
| 2-2-d1E04-1-0470 | 922.260 | OH | A02 | a1 | B04 | b1 | C16 | c2 | D02 | dl | E04 |
| 2-2-d1E04-1-0471 | 923.255 | OH | A02 | a1 | B04 | b1 | C17 | c2 | D02 | dl | E04 |
| 2-2-d1E04-1-0472 | 923.259 | OH | A02 | a1 | B05 | b1 | C14 | c2 | D02 | dl | E04 |
| 2-2-dlE04-1-0473 | 923.365 | OH | A02 | a1 | B05 | b1 | C19 | c1 | D01 | dl | E04 |
| 2-2-d1E04-1-0474 | 924.264 | OH | A02 | a1 | B04 | b4 | C01 | c2 | D02 | d1 | E04 |
| 2-2-d1E04-1-0475 | 925.246 | OH | A01 | a1 | B05 | b4 | C05 | c2 | D02 | d1 | E04 |
| 2-2-d1E04-1-0476 | 926.205 | OH | A02 | a1 | B05 | b3 | C10 | c2 | D02 | d1 | E04 |
| 2-2-d1E04-1-0477 | 927.258 | OH | A02 | a1 | B05 | b2 | C02 | c2 | D02 | d1 | E04 |
| 2-2-d1E04-1-0478 | 927.258 | OH | A02 | a1 | B05 | b2 | C03 | c2 | D02 | d1 | E04 |
| 2-2-d1E04-1-0479 | 928.216 | OH | A02 | a1 | B04 | b1 | C18 | c2 | D02 | d1 | E04 |
| 2-2-d1E04-1-0480 | 928.254 | OH | A02 | a1 | B05 | b2 | C04 | c2 | D02 | d1 | E04 |
| 2-2-d1E04-1-0481 | 928.254 | OH | A02 | a1 | B05 | b2 | C06 | c2 | D02 | d1 | E04 |
| 2-2-d1E04-1-0482 | 928.254 | OH | A02 | a1 | B05 | b2 | C07 | c2 | D02 | d1 | E04 |
| 2-2-d1E04-1-0483 | 932.285 | OH | A02 | a1 | B05 | b1 | C15 | c2 | D02 | d1 | E04 |
| 2-2-d1E04-1-0484 | 933.233 | OH | A01 | a1 | B05 | b3 | C11 | c2 | D02 | d1 | E04 |
| 2-2-d1E04-1-0485 | 933.280 | OH | A02 | a1 | B05 | b1 | C16 | c2 | D02 | d1 | E04 |
| 2-2-d1E04-1-0486 | 933.355 | OH | A02 | a1 | B02 | b1 | C19 | c2 | D02 | d1 | E04 |
| 2-2-d1E04-1-0487 | 934.275 | OH | A02 | a1 | B05 | b1 | C17 | c2 | D02 | d1 | E04 |
| 2-2-d1E04-1-0488 | 934.285 | OH | A02 | a1 | B04 | b4 | C02 | c2 | D02 | d1 | E04 |
| 2-2-d1E04-1-0489 | 935.285 | OH | A02 | a1 | B05 | b4 | C01 | c2 | D02 | d1 | E04 |
| 2-2-d1E04-1-0490 | 937.330 | OH | A02 | a1 | B03 | b1 | C19 | c2 | D02 | d1 | E04 |
| 2-2-d1E04-1-0491 | 939.237 | OH | A02 | a1 | B05 | b1 | C18 | c2 | D02 | d1 | E04 |
| 2-2-d1E04-1-0492 | 940.242 | OH | A02 | a1 | B04 | b4 | C05 | c2 | D02 | d1 | E04 |
| 2-2-d1E04-1-0493 | 945.305 | OH | A02 | a1 | B05 | b4 | C02 | c2 | D02 | d1 | E04 |
| 2-2-d1E04-1-0494 | 948.228 | OH | A02 | a1 | B04 | b3 | C11 | c2 | D02 | d1 | E04 |
| 2-2-d1E04-1-0495 | 950.291 | OH | A01 | a1 | B04 | b1 | C19 | c2 | D02 | d1 | E04 |
| 2-2-d1E04-1-0496 | 951.262 | OH | A02 | a1 | B05 | b4 | C05 | c2 | D02 | d1 | E04 |
| 2-2-d1E04-1-0497 | 959.248 | OH | A02 | a1 | B05 | b3 | C11 | c2 | D02 | d1 | E04 |
| 2-2-d1E04-1-0498 | 961.311 | OH | A01 | a1 | B05 | b1 | C19 | c2 | D02 | d1 | E04 |
| 2-2-d1E04-1-0499 | 976.307 | OH | A02 | a1 | B04 | b1 | C19 | c2 | D02 | d1 | E04 |
| 2-2-d1E04-1-0500 | 987.327 | OH | A02 | a1 | B05 | b1 | C19 | c2 | D02 | d1 | E04 |

Example 9-6: Synthesis of mixtures described in Table 9-6-1 by steps E01-1 to E04- 1

[Formula 689]

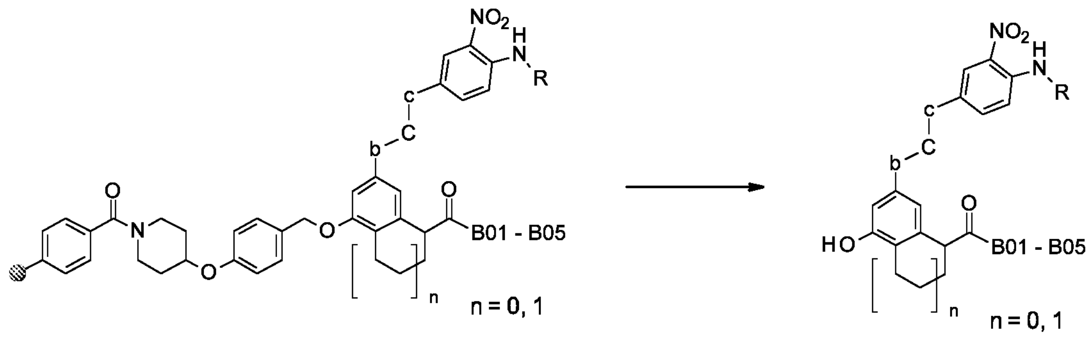

Separate mixtures (50 mg) described in Table 9-6-1 were added to four 2 mL glass vials, respectively. 0.08 M pentamethylbenzene in DCM (1.0 mL) was added thereto, and each mixture was shaken at room temperature for 30 minutes. TFA (0.10 mL) was added thereto, and the mixture was shaken at room temperature for 30 minutes. The suspension of the reaction solution and the solid phase was transferred to a 3 mL column with a filter using DMI (0.5 mL) twice and filtered. The resultant was washed twice with DMI (0.5 mL), and combined filtrates were collected into a 15 mm*75 mm test tube. The solvent in the solution was distilled off by Genevac. Solid-phase supported morpholine (Sigma-Aldrich Co., LLC, catalog No: 493813) (80 mg) and DMI (0.4 mL) were added thereto, and the mixture was shaken at 1500 rpm for 15 minutes. The solution and the suspension of the solid phase were transferred to a 3 mL column with a filter using DMI (0.4 mL) twice and filtered. The resultant was washed twice with DMI (0.2 mL) and twice with MeCN (0.2 mL), and combined filtrates were collected into a 0.5 to 2 mL microwave container. The solvent in the solution was distilled off under reduced pressure (Genevac reduced-pressure concentrator was used). DMSO was added in the amounts described in Table 9-6-1 to the microwave container to dissolve the residue. 10 μL of the solution was dispensed for LC-MS analysis described in Example 9-8 and for AS-MS assay in Example 11 to obtain the mixtures described in Table 9-6-1. The solvent in the remaining solution was distilled off under reduced pressure (Genevac reduced-pressure concentrator was used), and the residue was used in Example 9-7. The details of compounds that may be contained in each mixture are shown in Table 9-6-1.

[Table 496]

| [Table 9-6-1] | | | |
|---|---|---|---|
| Mixture used | Amount of DMSO used | Obtained mixture | Table No. showing compound that may be contained |
| 2-2-d1E01-0 | 0.185 mL | 2-2-d1E01-1 | [Table 9-5-2] |
| 2-2-d1E02-0 | 0.184 mL | 2-2-d1E02-1 | [Table 9-5-3] |
| 2-2-d1E03-0 | 0.183 mL | 2-2-d1E03-1 | [Table 9-5-4] |
| 2-2-d1E04-0 | 0.179 mL | 2-2-d1E04-1 | [Table 9-5-5] |

Example 9-7: Synthesis of mixtures described in Table 9-7-1 by ethylation steps E01-2 to E04-2

[Formula 690]

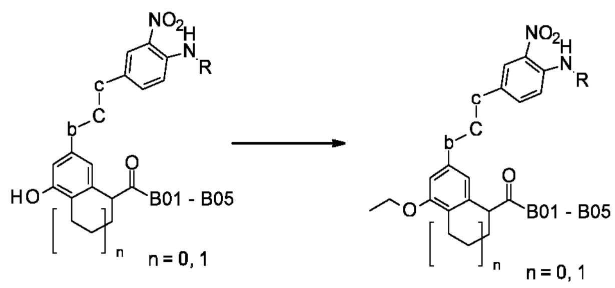

DMF, EtOH, BTPP, and $Et_3PO_4$ were added in the amounts described in Table 9-7-1 to the mixtures described in Table 9-7-1 contained in four 0.5 to 2 mL microwave containers obtained in Example 9-6, and each mixture was shaken at 70°C for 27 hours. The reaction solution was transferred to ISOLUTE(R) CBA (200 mg, 0.7 mmol/g) using MeCN (0.2 mL) twice. 10 minutes later, the resultant was washed three times with MeCN (0.5 mL), and combined filtrates were collected into a 15 mm*75 mm test tube. The solvent in the solution was distilled off under reduced pressure (Genevac reduced-pressure concentrator was used) to obtain the mixtures described in Table 9-7-1. DMSO was added to each of four types of mixtures obtained to prepare four types of 10 mM solutions in DMSO, which were then subjected to LC-MS analysis described in Example 9-8 and an experiment to evaluate binding to Bcl-2 by AS-MS in Example 11.
[Table 497]

| [Table 9-7-1] | | | | | |
|---|---|---|---|---|---|
| Mixture used | DMF | EtOH | BTPP | Et3P04 | Obtained mixture |
| 2-2-d1E01-1-01 | 100 μL | 25.0 μL | 26.8 μL 0.088 mmol | 22.3 μL 0.131 mmol | 2-2-d1E01-2 |
| 2-2-d1E02-1-01 | 100 μL | 24.9 μL | 26.7 μL 0.087 mmol | 22.2 μL 0.131 mmol | 2-2-d1E02-2 |
| 2-2-d1E03-1-01 | 99.0 μL | 24.7 μL | 26.5 μL 0.087 mmol | 22.1 μL 0.130 mmol | 2-2-d1E03-2 |
| 2-2-d1E04-1-01 | 97.0 μL | 24.2 μL | 25.9 μL 0.085 mmol | 21.6 μL 0.127 mmol | 2-2-d1E04-2 |

The details of the obtained mixtures in Table 9-7-1 are shown in Table 9-7-2 to Table 9-7-5. A notation method in each table will be described. For example, in Table 9-7-2, each compound that may be contained in 2-2-d1E01-2 is indicated by ID, and core blocks and linkers in combination in the structure of mixture 2-2-d1E01-2 are indicated by symbols or chemical formulas. The correspondence between the symbols and specific structures are shown in Figure 6.
In Table 9-7-2 to Table 9-7-5, "×" is represented by a chemical formula.

[Formula 691]

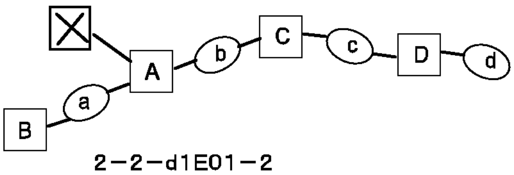


2-2-d1E01-2

For example, when ID is 2-2-d1E01-2-0001, the compound is represented by the following structural formula.

[Formula 692]

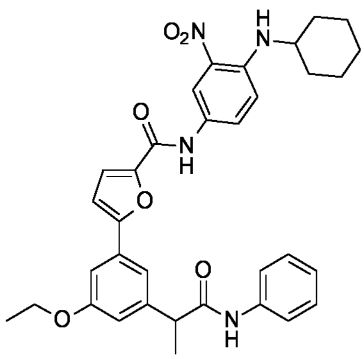

2-2-d1E01-2-0001

[Table 498]

| Exemplary notation in [Table 9-7-2] | | | | | | | | | | | |
|---|---|---|---|---|---|---|---|---|---|---|---|
| ID | Exact Mass | | A | a | B | b | C | c | D | d | E |
| 2-2-d1E01-2-0001 | 596.263 | OEt | A01 | a1 | B01 | b1 | C01 | c1 | D01 | d1 | E01 |

[Table 499]

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0001 | 596.263 | OEt | A01 | a1 | B01 | b1 | C01 | c1 | D01 | d1 | E01 |
| 2-2-d1E01-2-0002 | 600.331 | OEt | A01 | a1 | B01 | b4 | C12 | c1 | D01 | d1 | E01 |
| 2-2-d1E01-2-0003 | 602.310 | OEt | A01 | a1 | B01 | b4 | C13 | c1 | D01 | d1 | E01 |
| 2-2-d1E01-2-0004 | 606.284 | OEt | A01 | a1 | B01 | b1 | C02 | c1 | D01 | d1 | E01 |
| 2-2-d1E01-2-0005 | 606.284 | OEt | A01 | a1 | B01 | b1 | C03 | c1 | D01 | d1 | E01 |
| 2-2-d1E01-2-0006 | 607.279 | OEt | A01 | a1 | B01 | b1 | C04 | c1 | D01 | d1 | E01 |
| 2-2-d1E01-2-0007 | 610.279 | OEt | A01 | a1 | B02 | b1 | C01 | c1 | D01 | d1 | E01 |
| 2-2-d1E01-2-0008 | 612.241 | OEt | A01 | a1 | B01 | b1 | C05 | c1 | D01 | d1 | E01 |
| 2-2-d1E01-2-0009 | 613.254 | OEt | A01 | a1 | B01 | b3 | C08 | c1 | D01 | d1 | E01 |
| 2-2-d1E01-2-0010 | 614.254 | OEt | A01 | a1 | B03 | b1 | C01 | c1 | D01 | d1 | E01 |
| 2-2-d1E01-2-0011 | 614.347 | OEt | A01 | a1 | B02 | b4 | C12 | c1 | D01 | d1 | E01 |
| 2-2-d1E01-2-0012 | 616.326 | OEt | A01 | a1 | B02 | b4 | C13 | c1 | D01 | d1 | E01 |
| 2-2-d1E01-2-0013 | 618.322 | OEt | A01 | a1 | B03 | b4 | C12 | c1 | D01 | d1 | E01 |
| 2-2-d1E01-2-0014 | 620.300 | OEt | A01 | a1 | B02 | b1 | C02 | c1 | D01 | d1 | E01 |
| 2-2-d1E01-2-0015 | 620.300 | OEt | A01 | a1 | B02 | b1 | C03 | c1 | D01 | d1 | E01 |
| 2-2-d1E01-2-0016 | 620.301 | OEt | A01 | a1 | B03 | b4 | C13 | c1 | D01 | d1 | E01 |
| 2-2-d1E01-2-0017 | 621.295 | OEt | A01 | a1 | B02 | b1 | C04 | c1 | D01 | d1 | E01 |
| 2-2-d1E01-2-0018 | 622.279 | OEt | A02 | a1 | B01 | b1 | C01 | c1 | D01 | d1 | E01 |
| 2-2-d1E01-2-0019 | 624.270 | OEt | A01 | a1 | B01 | b3 | C09 | c1 | D01 | d1 | E01 |
| 2-2-d1E01-2-0020 | 624.275 | OEt | A01 | a1 | B03 | b1 | C02 | c1 | D01 | d1 | E01 |
| 2-2-d1E01-2-0021 | 624.275 | OEt | A01 | a1 | B03 | b1 | C03 | c1 | D01 | d1 | E01 |
| 2-2-d1E01-2-0022 | 625.270 | OEt | A01 | a1 | B03 | b1 | C04 | c1 | D01 | d1 | E01 |
| 2-2-d1E01-2-0023 | 626.256 | OEt | A01 | a1 | B02 | b1 | C05 | c1 | D01 | d1 | E01 |
| 2-2-d1E01-2-0024 | 626.285 | OEt | A01 | a1 | B01 | b1 | C14 | c1 | D01 | d1 | E01 |
| 2-2-d1E01-2-0025 | 626.347 | OEt | A02 | a1 | B01 | b4 | C12 | c1 | D01 | d1 | E01 |
| 2-2-d1E01-2-0026 | 627.269 | OEt | A01 | a1 | B02 | b3 | C08 | c1 | D01 | d1 | E01 |
| 2-2-d1E01-2-0027 | 628.326 | OEt | A02 | a1 | B01 | b4 | C13 | c1 | D01 | d1 | E01 |
| 2-2-d1E01-2-0028 | 629.231 | OEt | A01 | a1 | B01 | b3 | C10 | c1 | D01 | d1 | E01 |
| 2-2-d1E01-2-0029 | 630.231 | OEt | A01 | a1 | B03 | b1 | C05 | c1 | D01 | d1 | E01 |
| 2-2-d1E01-2-0030 | 630.284 | OEt | A01 | a1 | B01 | b2 | C02 | c1 | D01 | d1 | E01 |
| 2-2-d1E01-2-0031 | 630.284 | OEt | A01 | a1 | B01 | b2 | C03 | c1 | D01 | d1 | E01 |
| 2-2-d1E01-2-0032 | 631.244 | OEt | A01 | a1 | B03 | b3 | C08 | c1 | D01 | d1 | E01 |
| 2-2-d1E01-2-0033 | 631.279 | OEt | A01 | a1 | B01 | b2 | C04 | c1 | D01 | d1 | E01 |
| 2-2-d1E01-2-0034 | 631.279 | OEt | A01 | a1 | B01 | b2 | C06 | c1 | D01 | d1 | E01 |
| 2-2-d1E01-2-0035 | 631.279 | OEt | A01 | a1 | B01 | b2 | C07 | c1 | D01 | d1 | E01 |
| 2-2-d1E01-2-0036 | 632.300 | OEt | A02 | a1 | B01 | b1 | C02 | c1 | D01 | d1 | E01 |
| 2-2-d1E01-2-0037 | 632.300 | OEt | A02 | a1 | B01 | b1 | C03 | c1 | D01 | d1 | E01 |
| 2-2-d1E01-2-0038 | 633.295 | OEt | A02 | a1 | B01 | b1 | C04 | c1 | D01 | d1 | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0039 | 635.311 | OEt | A01 | a1 | B01 | b1 | C15 | c1 | D01 | d1 | E01 |
| 2-2-d1E01-2-0040 | 636.295 | OEt | A02 | a1 | B02 | b1 | C01 | c1 | D01 | d1 | E01 |
| 2-2-d1E01-2-0041 | 636.306 | OEt | A01 | a1 | B01 | b1 | C16 | c1 | D01 | d1 | E01 |
| 2-2-d1E01-2-0042 | 637.301 | OEt | A01 | a1 | B01 | b1 | C17 | c1 | D01 | d1 | E01 |
| 2-2-d1E01-2-0043 | 638.256 | OEt | A02 | a1 | B01 | b1 | C05 | c1 | D01 | d1 | E01 |
| 2-2-d1E01-2-0044 | 638.285 | OEt | A01 | a1 | B02 | b3 | C09 | c1 | D01 | d1 | E01 |
| 2-2-d1E01-2-0045 | 638.310 | OEt | A01 | a1 | B01 | b4 | C01 | c1 | D01 | d1 | E01 |
| 2-2-d1E01-2-0046 | 639.269 | OEt | A02 | a1 | B01 | b3 | C08 | c1 | D01 | d1 | E01 |
| 2-2-d1E01-2-0047 | 640.270 | OEt | A02 | a1 | B03 | b1 | C01 | c1 | D01 | d1 | E01 |
| 2-2-d1E01-2-0048 | 640.301 | OEt | A01 | a1 | B02 | b1 | C14 | c1 | D01 | d1 | E01 |
| 2-2-d1E01-2-0049 | 640.362 | OEt | A02 | a1 | B02 | b4 | C12 | c1 | D01 | d1 | E01 |
| 2-2-d1E01-2-0050 | 642.260 | OEt | A01 | a1 | B03 | b3 | C09 | c1 | D01 | d1 | E01 |
| 2-2-d1E01-2-0051 | 642.262 | OEt | A01 | a1 | B01 | b1 | C18 | c1 | D01 | d1 | E01 |
| 2-2-d1E01-2-0052 | 642.342 | OEt | A02 | a1 | B02 | b4 | C13 | c1 | D01 | d1 | E01 |
| 2-2-d1E01-2-0053 | 643.246 | OEt | A01 | a1 | B02 | b3 | C10 | c1 | D01 | d1 | E01 |
| 2-2-d1E01-2-0054 | 644.276 | OEt | A01 | a1 | B03 | b1 | C14 | c1 | D01 | d1 | E01 |
| 2-2-d1E01-2-0055 | 644.300 | OEt | A01 | a1 | B02 | b2 | C02 | c1 | D01 | d1 | E01 |
| 2-2-d1E01-2-0056 | 644.300 | OEt | A01 | a1 | B02 | b2 | C03 | c1 | D01 | d1 | E01 |
| 2-2-d1E01-2-0057 | 644.337 | OEt | A02 | a1 | B03 | b4 | C12 | c1 | D01 | d1 | E01 |
| 2-2-d1E01-2-0058 | 645.295 | OEt | A01 | a1 | B02 | b2 | C04 | c1 | D01 | d1 | E01 |
| 2-2-d1E01-2-0059 | 645.295 | OEt | A01 | a1 | B02 | b2 | C06 | c1 | D01 | d1 | E01 |
| 2-2-d1E01-2-0060 | 645.295 | OEt | A01 | a1 | B02 | b2 | C07 | c1 | D01 | d1 | E01 |
| 2-2-d1E01-2-0061 | 646.316 | OEt | A02 | a1 | B02 | b1 | C02 | c1 | D01 | d1 | E01 |
| 2-2-d1E01-2-0062 | 646.316 | OEt | A02 | a1 | B02 | b1 | C03 | c1 | D01 | d1 | E01 |
| 2-2-d1E01-2-0063 | 646.317 | OEt | A02 | a1 | B03 | b4 | C13 | c1 | D01 | d1 | E01 |
| 2-2-d1E01-2-0064 | 647.221 | OEt | A01 | a1 | B03 | b3 | C10 | c1 | D01 | d1 | E01 |
| 2-2-d1E01-2-0065 | 647.311 | OEt | A02 | a1 | B02 | b1 | C04 | c1 | D01 | d1 | E01 |
| 2-2-d1E01-2-0066 | 648.275 | OEt | A01 | a1 | B03 | b2 | C02 | c1 | D01 | d1 | E01 |
| 2-2-d1E01-2-0067 | 648.275 | OEt | A01 | a1 | B03 | b2 | C03 | c1 | D01 | d1 | E01 |
| 2-2-d1E01-2-0068 | 648.331 | OEt | A01 | a1 | B01 | b4 | C02 | c1 | D01 | d1 | E01 |
| 2-2-d1E01-2-0069 | 649.270 | OEt | A01 | a1 | B03 | b2 | C04 | c1 | D01 | d1 | E01 |
| 2-2-d1E01-2-0070 | 649.270 | OEt | A01 | a1 | B03 | b2 | C06 | c1 | D01 | d1 | E01 |
| 2-2-d1E01-2-0071 | 649.270 | OEt | A01 | a1 | B03 | b2 | C07 | c1 | D01 | d1 | E01 |
| 2-2-d1E01-2-0072 | 649.326 | OEt | A01 | a1 | B02 | b1 | C15 | c1 | D01 | d1 | E01 |
| 2-2-d1E01-2-0073 | 650.285 | OEt | A02 | a1 | B01 | b3 | C09 | c1 | D01 | d1 | E01 |
| 2-2-d1E01-2-0074 | 650.290 | OEt | A02 | a1 | B03 | b1 | C02 | c1 | D01 | d1 | E01 |
| 2-2-d1E01-2-0075 | 650.290 | OEt | A02 | a1 | B03 | b1 | C03 | c1 | D01 | d1 | E01 |
| 2-2-d1E01-2-0076 | 650.322 | OEt | A01 | a1 | B02 | b1 | C16 | c1 | D01 | d1 | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0077 | 651.286 | OEt | A02 | a1 | B03 | b1 | C04 | c1 | D01 | d1 | E01 |
| 2-2-d1E01-2-0078 | 651.317 | OEt | A01 | a1 | B02 | b1 | C17 | c1 | D01 | d1 | E01 |
| 2-2-d1E01-2-0079 | 652.272 | OEt | A02 | a1 | B02 | b1 | COS | c1 | D01 | d1 | E01 |
| 2-2-d1E01-2-0080 | 652.301 | OEt | A02 | a1 | B01 | bl | C14 | c1 | D01 | d1 | E01 |
| 2-2-d1E01-2-0081 | 652.326 | OEt | A01 | a1 | B02 | b4 | C01 | c1 | D01 | d1 | E01 |
| 2-2-d1E01-2-0082 | 653.231 | OEt | A01 | a1 | B04 | b1 | C01 | c1 | D01 | d1 | E01 |
| 2-2-d1E01-2-0083 | 653.285 | OEt | A02 | a1 | B02 | b3 | C08 | c1 | D01 | d1 | E01 |
| 2-2-d1E01-2-0084 | 653.301 | OEt | A01 | a1 | B03 | b1 | C15 | c1 | D01 | d1 | E01 |
| 2-2-d1E01-2-0085 | 654.288 | OEt | A01 | a1 | B01 | b4 | COS | c1 | D01 | d1 | E01 |
| 2-2-d1E01-2-0086 | 654.297 | OEt | A01 | a1 | B03 | b1 | C16 | c1 | D01 | d1 | E01 |
| 2-2-d1E01-2-0087 | 655.246 | OEt | A02 | a1 | B01 | b3 | C10 | c1 | D01 | d1 | E01 |
| 2-2-d1E01-2-0088 | 655.292 | OEt | A01 | a1 | B03 | b1 | C17 | c1 | D01 | d1 | E01 |
| 2-2-d1E01-2-0089 | 656.247 | OEt | A02 | a1 | B03 | b1 | COS | c1 | D01 | d1 | E01 |
| 2-2-d1E01-2-0090 | 656.278 | OEt | A01 | a1 | B02 | b1 | C18 | c1 | D01 | d1 | E01 |
| 2-2-d1E01-2-0091 | 656.300 | OEt | A02 | a1 | B01 | b2 | C02 | c1 | D01 | d1 | E01 |
| 2-2-d1E01-2-0092 | 656.300 | OEt | A02 | a1 | B01 | b2 | C03 | c1 | D01 | d1 | E01 |
| 2-2-d1E01-2-0093 | 656.301 | OEt | A01 | a1 | B03 | b4 | C01 | c1 | D01 | d1 | E01 |
| 2-2-d1E01-2-0094 | 657.260 | OEt | A02 | a1 | B03 | b3 | C08 | c1 | D01 | d1 | E01 |
| 2-2-d1E01-2-0095 | 657.295 | OEt | A02 | a1 | B01 | b2 | C04 | c1 | D01 | d1 | E01 |
| 2-2-d1E01-2-0096 | 657.295 | OEt | A02 | a1 | B01 | b2 | C06 | c1 | D01 | d1 | E01 |
| 2-2-d1E01-2-0097 | 657.295 | OEt | A02 | a1 | B01 | b2 | C07 | c1 | D01 | d1 | E01 |
| 2-2-d1E01-2-0098 | 657.298 | OEt | A01 | a1 | B04 | b4 | C12 | c1 | D01 | d1 | E01 |
| 2-2-d1E01-2-0099 | 659.278 | OEt | A01 | a1 | B04 | b4 | C13 | c1 | D01 | d1 | E01 |
| 2-2-d1E01-2-0100 | 660.225 | OEt | A01 | a1 | B01 | b1 | C01 | c2 | D02 | d1 | E01 |
| 2-2-d1E01-2-0101 | 660.253 | OEt | A01 | a1 | B03 | b1 | C18 | c1 | D01 | d1 | E01 |
| 2-2-d1E01-2-0102 | 661.326 | OEt | A02 | a1 | B01 | b1 | C15 | c1 | D01 | d1 | E01 |
| 2-2-d1E01-2-0103 | 662.274 | OEt | A01 | a1 | B01 | b3 | C11 | c1 | D01 | d1 | E01 |
| 2-2-d1E01-2-0104 | 662.322 | OEt | A02 | a1 | B01 | b1 | C16 | c1 | D01 | d1 | E01 |
| 2-2-d1E01-2-0105 | 662.347 | OEt | A01 | a1 | B02 | b4 | C02 | c1 | D01 | d1 | E01 |
| 2-2-d1E01-2-0106 | 663.252 | OEt | A01 | a1 | B04 | b1 | C02 | c1 | D01 | d1 | E01 |
| 2-2-d1E01-2-0107 | 663.252 | OEt | A01 | a1 | B04 | b1 | C03 | c1 | D01 | d1 | E01 |
| 2-2-d1E01-2-0108 | 663.317 | OEt | A02 | a1 | B01 | b1 | C17 | c1 | D01 | d1 | E01 |
| 2-2-d1E01-2-0109 | 664.247 | OEt | A01 | a1 | B04 | b1 | C04 | c1 | D01 | d1 | E01 |
| 2-2-d1E01-2-0110 | 664.251 | OEt | A01 | a1 | B05 | b1 | C01 | c1 | D01 | d1 | E01 |
| 2-2-d1E01-2-0111 | 664.293 | OEt | A01 | a1 | B01 | b4 | C12 | c2 | D02 | d1 | E01 |
| 2-2-d1E01-2-0112 | 664.301 | OEt | A02 | a1 | B02 | b3 | C09 | c1 | D01 | d1 | E01 |
| 2-2-d1E01-2-0113 | 664.326 | OEt | A02 | a1 | B01 | b4 | C01 | c1 | D01 | d1 | E01 |
| 2-2-d1E01-2-0114 | 666.272 | OEt | A01 | a1 | B01 | b4 | C13 | c2 | D02 | d1 | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0115 | 666.317 | OEt | A02 | a1 | B02 | b1 | C14 | c1 | D01 | d1 | E01 |
| 2-2-d1E01-2-0116 | 666.322 | OEt | A01 | a1 | B03 | b4 | C02 | c1 | D01 | d1 | E01 |
| 2-2-d1E01-2-0117 | 668.276 | OEt | A02 | a1 | B03 | b3 | C09 | c1 | D01 | d1 | E01 |
| 2-2-d1E01-2-0118 | 668.278 | OEt | A02 | a1 | B01 | b1 | C18 | c1 | D01 | d1 | E01 |
| 2-2-d1E01-2-0119 | 668.303 | OEt | A01 | a1 | B02 | b4 | C05 | c1 | D01 | d1 | E01 |
| 2-2-d1E01-2-0120 | 668.319 | OEt | A01 | a1 | B05 | b4 | C12 | c1 | D01 | d1 | E01 |
| 2-2-d1E01-2-0121 | 669.208 | OEt | A01 | a1 | B04 | b1 | C05 | c1 | D01 | d1 | E01 |
| 2-2-d1E01-2-0122 | 669.262 | OEt | A02 | a1 | B02 | b3 | C10 | c1 | D01 | d1 | E01 |
| 2-2-d1E01-2-0123 | 670.221 | OEt | A01 | a1 | B04 | b3 | C08 | c1 | D01 | d1 | E01 |
| 2-2-d1E01-2-0124 | 670.246 | OEt | A01 | a1 | B01 | b1 | C02 | c2 | D02 | d1 | E01 |
| 2-2-d1E01-2-0125 | 670.246 | OEt | A01 | a1 | B01 | b1 | C03 | c2 | D02 | d1 | E01 |
| 2-2-d1E01-2-0126 | 670.292 | OEt | A02 | a1 | B03 | b1 | C14 | c1 | D01 | d1 | E01 |
| 2-2-d1E01-2-0127 | 670.298 | OEt | A01 | a1 | B05 | b4 | C13 | c1 | D01 | d1 | E01 |
| 2-2-d1E01-2-0128 | 670.316 | OEt | A02 | a1 | B02 | b2 | C02 | c1 | D01 | d1 | E01 |
| 2-2-d1E01-2-0129 | 670.316 | OEt | A02 | a1 | B02 | b2 | C03 | c1 | D01 | d1 | E01 |
| 2-2-d1E01-2-0130 | 671.241 | OEt | A01 | a1 | B01 | b1 | C04 | c2 | D02 | d1 | E01 |
| 2-2-d1E01-2-0131 | 671.311 | OEt | A02 | a1 | B02 | b2 | C04 | c1 | D01 | d1 | E01 |
| 2-2-d1E01-2-0132 | 671.311 | OEt | A02 | a1 | B02 | b2 | C06 | c1 | D01 | d1 | E01 |
| 2-2-d1E01-2-0133 | 671.311 | OEt | A02 | a1 | B02 | b2 | C07 | c1 | D01 | d1 | E01 |
| 2-2-d1E01-2-0134 | 672.278 | OEt | A01 | a1 | B03 | b4 | C05 | c1 | D01 | d1 | E01 |
| 2-2-d1E01-2-0135 | 673.237 | OEt | A02 | a1 | B03 | b3 | C10 | c1 | D01 | d1 | E01 |
| 2-2-d1E01-2-0136 | 674.241 | OEt | A01 | a1 | B02 | b1 | C01 | c2 | D02 | d1 | E01 |
| 2-2-d1E01-2-0137 | 674.272 | OEt | A01 | a1 | B05 | b1 | C02 | c1 | D01 | d1 | E01 |
| 2-2-d1E01-2-0138 | 674.272 | OEt | A01 | a1 | B05 | b1 | C03 | c1 | D01 | d1 | E01 |
| 2-2-d1E01-2-0139 | 674.290 | OEt | A02 | a1 | B03 | b2 | C02 | c1 | D01 | d1 | E01 |
| 2-2-d1E01-2-0140 | 674.290 | OEt | A02 | a1 | B03 | b2 | C03 | c1 | D01 | d1 | E01 |
| 2-2-d1E01-2-0141 | 674.347 | OEt | A02 | a1 | B01 | b4 | C02 | c1 | D01 | d1 | E01 |
| 2-2-d1E01-2-0142 | 675.267 | OEt | A01 | a1 | B05 | b1 | C04 | c1 | D01 | d1 | E01 |
| 2-2-d1E01-2-0143 | 675.286 | OEt | A02 | a1 | B03 | b2 | C04 | c1 | D01 | d1 | E01 |
| 2-2-d1E01-2-0144 | 675.286 | OEt | A02 | a1 | B03 | b2 | C06 | c1 | D01 | d1 | E01 |
| 2-2-d1E01-2-0145 | 675.286 | OEt | A02 | a1 | B03 | b2 | C07 | c1 | D01 | d1 | E01 |
| 2-2-d1E01-2-0146 | 675.342 | OEt | A02 | a1 | B02 | b1 | C15 | c1 | D01 | d1 | E01 |
| 2-2-d1E01-2-0147 | 676.203 | OEt | A01 | a1 | B01 | b1 | C05 | c2 | D02 | d1 | E01 |
| 2-2-d1E01-2-0148 | 676.290 | OEt | A01 | a1 | B02 | b3 | C11 | c1 | D01 | d1 | E01 |
| 2-2-d1E01-2-0149 | 676.337 | OEt | A02 | a1 | B02 | b1 | C16 | c1 | D01 | d1 | E01 |
| 2-2-d1E01-2-0150 | 677.216 | OEt | A01 | a1 | B01 | b3 | C08 | c2 | D02 | d1 | E01 |
| 2-2-d1E01-2-0151 | 677.333 | OEt | A02 | a1 | B02 | b1 | C17 | c1 | D01 | d1 | E01 |
| 2-2-d1E01-2-0152 | 678.216 | OEt | A01 | a1 | B03 | b1 | C01 | c2 | D02 | d1 | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0153 | 678.309 | OEt | A01 | a1 | B02 | b4 | C12 | c2 | D02 | d1 | E01 |
| 2-2-d1E01-2-0154 | 678.342 | OEt | A02 | a1 | B02 | b4 | C01 | c1 | D01 | d1 | E01 |
| 2-2-d1E01-2-0155 | 679.246 | OEt | A02 | a1 | B04 | b1 | C01 | c1 | D01 | d1 | E01 |
| 2-2-d1E01-2-0156 | 679.317 | OEt | A02 | a1 | B03 | b1 | C15 | c1 | D01 | d1 | E01 |
| 2-2-d1E01-2-0157 | 680.228 | OEt | A01 | a1 | B05 | b1 | C05 | c1 | D01 | d1 | E01 |
| 2-2-d1E01-2-0158 | 680.265 | OEt | A01 | a1 | B03 | b3 | C11 | c1 | D01 | d1 | E01 |
| 2-2-d1E01-2-0159 | 680.288 | OEt | A01 | a1 | B02 | b4 | C13 | c2 | D02 | d1 | E01 |
| 2-2-d1E01-2-0160 | 680.303 | OEt | A02 | a1 | B01 | b4 | C05 | c1 | D01 | d1 | E01 |
| 2-2-d1E01-2-0161 | 680.312 | OEt | A02 | a1 | B03 | b1 | C16 | c1 | D01 | d1 | E01 |
| 2-2-d1E01-2-0162 | 681.237 | OEt | A01 | a1 | B04 | b3 | C09 | c1 | D01 | d1 | E01 |
| 2-2-d1E01-2-0163 | 681.241 | OEt | A01 | a1 | B05 | b3 | C08 | c1 | D01 | d1 | E01 |
| 2-2-d1E01-2-0164 | 681.307 | OEt | A02 | a1 | B03 | b1 | C17 | c1 | D01 | d1 | E01 |
| 2-2-d1E01-2-0165 | 682.284 | OEt | A01 | a1 | B03 | b4 | C12 | c2 | D02 | d1 | E01 |
| 2-2-d1E01-2-0166 | 682.294 | OEt | A02 | a1 | B02 | b1 | C18 | c1 | D01 | d1 | E01 |
| 2-2-d1E01-2-0167 | 682.317 | OEt | A02 | a1 | B03 | b4 | C01 | c1 | D01 | d1 | E01 |
| 2-2-d1E01-2-0168 | 683.253 | OEt | A01 | a1 | B04 | b1 | C14 | c1 | D01 | d1 | E01 |
| 2-2-d1E01-2-0169 | 683.314 | OEt | A02 | a1 | B04 | b4 | C12 | c1 | D01 | d1 | E01 |
| 2-2-d1E01-2-0170 | 684.262 | OEt | A01 | a1 | B02 | b1 | C02 | c2 | D02 | d1 | E01 |
| 2-2-d1E01-2-0171 | 684.262 | OEt | A01 | a1 | B02 | b1 | C03 | c2 | D02 | d1 | E01 |
| 2-2-d1E01-2-0172 | 684.263 | OEt | A01 | a1 | B03 | b4 | C13 | c2 | D02 | d1 | E01 |
| 2-2-d1E01-2-0173 | 685.257 | OEt | A01 | a1 | B02 | b1 | C04 | c2 | D02 | d1 | E01 |
| 2-2-d1E01-2-0174 | 685.293 | OEt | A02 | a1 | B04 | b4 | C13 | c1 | D01 | d1 | E01 |
| 2-2-d1E01-2-0175 | 686.198 | OEt | A01 | a1 | B04 | b3 | C10 | c1 | D01 | d1 | E01 |
| 2-2-d1E01-2-0176 | 686.241 | OEt | A02 | a1 | B01 | b1 | C01 | c2 | D02 | d1 | E01 |
| 2-2-d1E01-2-0177 | 686.269 | OEt | A02 | a1 | B03 | b1 | C18 | c1 | D01 | d1 | E01 |
| 2-2-d1E01-2-0178 | 687.252 | OEt | A01 | a1 | B04 | b2 | C02 | c1 | D01 | d1 | E01 |
| 2-2-d1E01-2-0179 | 687.252 | OEt | A01 | a1 | B04 | b2 | C03 | c1 | D01 | d1 | E01 |
| 2-2-d1E01-2-0180 | 688.232 | OEt | A01 | a1 | B01 | b3 | C09 | c2 | D02 | d1 | E01 |
| 2-2-d1E01-2-0181 | 688.237 | OEt | A01 | a1 | B03 | b1 | C02 | c2 | D02 | d1 | E01 |
| 2-2-d1E01-2-0182 | 688.237 | OEt | A01 | a1 | B03 | b1 | C03 | c2 | D02 | d1 | E01 |
| 2-2-d1E01-2-0183 | 688.247 | OEt | A01 | a1 | B04 | b2 | C04 | c1 | D01 | d1 | E01 |
| 2-2-d1E01-2-0184 | 688.247 | OEt | A01 | a1 | B04 | b2 | C06 | c1 | D01 | d1 | E01 |
| 2-2-d1E01-2-0185 | 688.247 | OEt | A01 | a1 | B04 | b2 | C07 | c1 | D01 | d1 | E01 |
| 2-2-d1E01-2-0186 | 688.290 | OEt | A02 | a1 | B01 | b3 | C11 | c1 | D01 | d1 | E01 |
| 2-2-d1E01-2-0187 | 688.362 | OEt | A02 | a1 | B02 | b4 | C02 | c1 | D01 | d1 | E01 |
| 2-2-d1E01-2-0188 | 689.232 | OEt | A01 | a1 | B03 | b1 | C04 | c2 | D02 | d1 | E01 |
| 2-2-d1E01-2-0189 | 689.267 | OEt | A02 | a1 | B04 | b1 | C02 | c1 | D01 | d1 | E01 |
| 2-2-d1E01-2-0190 | 689.267 | OEt | A02 | a1 | B04 | b1 | C03 | c1 | D01 | d1 | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0191 | 690.218 | OEt | A01 | a1 | B02 | b1 | C05 | c2 | D02 | d1 | E01 |
| 2-2-d1E01-2-0192 | 690.247 | OEt | A01 | a1 | B01 | b1 | C14 | c2 | D02 | d1 | E01 |
| 2-2-d1E01-2-0193 | 690.262 | OEt | A02 | a1 | B04 | b1 | C04 | c1 | D01 | d1 | E01 |
| 2-2-d1E01-2-0194 | 690.267 | OEt | A02 | a1 | B05 | b1 | C01 | c1 | D01 | d1 | E01 |
| 2-2-d1E01-2-0195 | 690.309 | OEt | A02 | a1 | B01 | b4 | C12 | c2 | D02 | d1 | E01 |
| 2-2-d1E01-2-0196 | 690.353 | OEt | A01 | a1 | B01 | b1 | C19 | c1 | D01 | d1 | E01 |
| 2-2-d1E01-2-0197 | 691.231 | OEt | A01 | a1 | B02 | b3 | C08 | c2 | D02 | d1 | E01 |
| 2-2-d1E01-2-0198 | 692.257 | OEt | A01 | a1 | B05 | b3 | C09 | c1 | D01 | d1 | E01 |
| 2-2-d1E01-2-0199 | 692.278 | OEt | A01 | a1 | B04 | b1 | C15 | c1 | D01 | dl | E01 |
| 2-2-d1E01-2-0200 | 692.288 | OEt | A02 | a1 | B01 | b4 | C13 | c2 | D02 | dl | E01 |
| 2-2-d1E01-2-0201 | 692.337 | OEt | A02 | a1 | B03 | b4 | C02 | c1 | D01 | dl | E01 |
| 2-2-d1E01-2-0202 | 693.193 | OEt | A01 | a1 | B01 | b3 | C10 | c2 | D02 | dl | E01 |
| 2-2-d1E01-2-0203 | 693.273 | OEt | A01 | a1 | B04 | b1 | C16 | c1 | D01 | dl | E01 |
| 2-2-d1E01-2-0204 | 694.193 | OEt | A01 | a1 | B03 | b1 | C05 | c2 | D02 | dl | E01 |
| 2-2-d1E01-2-0205 | 694.246 | OEt | A01 | a1 | B01 | b2 | C02 | c2 | D02 | dl | E01 |
| 2-2-d1E01-2-0206 | 694.246 | OEt | A01 | a1 | B01 | b2 | C03 | c2 | D02 | dl | E01 |
| 2-2-d1E01-2-0207 | 694.269 | OEt | A01 | a1 | B04 | b1 | C17 | c1 | D01 | dl | E01 |
| 2-2-d1E01-2-0208 | 694.273 | OEt | A01 | a1 | B05 | b1 | C14 | c1 | D01 | dl | E01 |
| 2-2-d1E01-2-0209 | 694.319 | OEt | A02 | a1 | B02 | b4 | C05 | c1 | D01 | dl | E01 |
| 2-2-d1E01-2-0210 | 694.334 | OEt | A02 | a1 | B05 | b4 | C12 | c1 | D01 | dl | E01 |
| 2-2-d1E01-2-0211 | 695.206 | OEt | A01 | a1 | B03 | b3 | C08 | c2 | D02 | dl | E01 |
| 2-2-d1E01-2-0212 | 695.224 | OEt | A02 | a1 | B04 | b1 | C05 | c1 | D01 | dl | E01 |
| 2-2-d1E01-2-0213 | 695.241 | OEt | A01 | a1 | B01 | b2 | C04 | c2 | D02 | dl | E01 |
| 2-2-d1E01-2-0214 | 695.241 | OEt | A01 | a1 | B01 | b2 | C06 | c2 | D02 | dl | E01 |
| 2-2-d1E01-2-0215 | 695.241 | OEt | A01 | a1 | B01 | b2 | C07 | c2 | D02 | dl | E01 |
| 2-2-d1E01-2-0216 | 695.278 | OEt | A01 | a1 | B04 | b4 | C01 | c1 | D01 | dl | E01 |
| 2-2-d1E01-2-0217 | 696.237 | OEt | A02 | a1 | B04 | b3 | C08 | c1 | D01 | dl | E01 |
| 2-2-d1E01-2-0218 | 696.262 | OEt | A02 | a1 | B01 | b1 | C02 | c2 | D02 | dl | E01 |
| 2-2-d1E01-2-0219 | 696.262 | OEt | A02 | a1 | B01 | b1 | C03 | c2 | D02 | dl | E01 |
| 2-2-d1E01-2-0220 | 696.313 | OEt | A02 | a1 | B05 | b4 | C13 | c1 | D01 | dl | E01 |
| 2-2-d1E01-2-0221 | 697.218 | OEt | A01 | a1 | B05 | b3 | C10 | c1 | D01 | dl | E01 |
| 2-2-d1E01-2-0222 | 697.257 | OEt | A02 | a1 | B01 | b1 | C04 | c2 | D02 | dl | E01 |
| 2-2-d1E01-2-0223 | 698.272 | OEt | A01 | a1 | B05 | b2 | C02 | c1 | D01 | dl | E01 |
| 2-2-d1E01-2-0224 | 698.272 | OEt | A01 | a1 | B05 | b2 | C03 | c1 | D01 | dl | E01 |
| 2-2-d1E01-2-0225 | 698.294 | OEt | A02 | a1 | B03 | b4 | COS | c1 | D01 | dl | E01 |
| 2-2-d1E01-2-0226 | 699.230 | OEt | A01 | a1 | B04 | b1 | C18 | c1 | D01 | dl | E01 |
| 2-2-d1E01-2-0227 | 699.267 | OEt | A01 | a1 | B05 | b2 | C04 | c1 | D01 | dl | E01 |
| 2-2-d1E01-2-0228 | 699.267 | OEt | A01 | a1 | B05 | b2 | C06 | c1 | D01 | dl | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0229 | 699.267 | OEt | A01 | a1 | B05 | b2 | C07 | c1 | D01 | dl | E01 |
| 2-2-d1E01-2-0230 | 699.273 | OEt | A01 | a1 | B01 | b1 | C15 | c2 | D02 | dl | E01 |
| 2-2-d1E01-2-0231 | 700.257 | OEt | A02 | a1 | B02 | b1 | C01 | c2 | D02 | dl | E01 |
| 2-2-d1E01-2-0232 | 700.268 | OEt | A01 | a1 | B01 | b1 | C16 | c2 | D02 | dl | E01 |
| 2-2-d1E01-2-0233 | 700.287 | OEt | A02 | a1 | B05 | b1 | C02 | c1 | D01 | dl | E01 |
| 2-2-d1E01-2-0234 | 700.287 | OEt | A02 | a1 | B05 | b1 | C03 | c1 | D01 | dl | E01 |
| 2-2-d1E01-2-0235 | 701.263 | OEt | A01 | a1 | B01 | b1 | C17 | c2 | D02 | dl | E01 |
| 2-2-d1E01-2-0236 | 701.283 | OEt | A02 | a1 | B05 | b1 | C04 | c1 | D01 | dl | E01 |
| 2-2-d1E01-2-0237 | 702.218 | OEt | A02 | a1 | B01 | b1 | C05 | c2 | D02 | dl | E01 |
| 2-2-d1E01-2-0238 | 702.247 | OEt | A01 | a1 | B02 | b3 | C09 | c2 | D02 | dl | E01 |
| 2-2-d1E01-2-0239 | 702.272 | OEt | A01 | a1 | B01 | b4 | C01 | c2 | D02 | dl | E01 |
| 2-2-d1E01-2-0240 | 702.305 | OEt | A02 | a1 | B02 | b3 | C11 | c1 | D01 | dl | E01 |
| 2-2-d1E01-2-0241 | 703.231 | OEt | A02 | a1 | B01 | b3 | C08 | c2 | D02 | dl | E01 |
| 2-2-d1E01-2-0242 | 703.298 | OEt | A01 | a1 | B05 | b1 | C15 | c1 | D01 | dl | E01 |
| 2-2-d1E01-2-0243 | 704.232 | OEt | A02 | a1 | B03 | b1 | C01 | c2 | D02 | dl | E01 |
| 2-2-d1E01-2-0244 | 704.263 | OEt | A01 | a1 | B02 | b1 | C14 | c2 | D02 | dl | E01 |
| 2-2-d1E01-2-0245 | 704.293 | OEt | A01 | a1 | B05 | b1 | C16 | c1 | D01 | dl | E01 |
| 2-2-d1E01-2-0246 | 704.324 | OEt | A02 | a1 | B02 | b4 | C12 | c2 | D02 | dl | E01 |
| 2-2-d1E01-2-0247 | 704.369 | OEt | A01 | a1 | B02 | b1 | C19 | c1 | D01 | dl | E01 |
| 2-2-d1E01-2-0248 | 705.289 | OEt | A01 | a1 | B05 | b1 | C17 | c1 | D01 | dl | E01 |
| 2-2-d1E01-2-0249 | 705.298 | OEt | A01 | a1 | B04 | b4 | C02 | c1 | D01 | dl | E01 |
| 2-2-d1E01-2-0250 | 706.222 | OEt | A01 | a1 | B03 | b3 | C09 | c2 | D02 | dl | E01 |
| 2-2-d1E01-2-0251 | 706.224 | OEt | A01 | a1 | B01 | b1 | C18 | c2 | D02 | dl | E01 |
| 2-2-d1E01-2-0252 | 706.244 | OEt | A02 | a1 | B05 | b1 | C05 | c1 | D01 | dl | E01 |
| 2-2-d1E01-2-0253 | 706.280 | OEt | A02 | a1 | B03 | b3 | C11 | c1 | D01 | dl | E01 |
| 2-2-d1E01-2-0254 | 706.298 | OEt | A01 | a1 | B05 | b4 | C01 | c1 | D01 | dl | E01 |
| 2-2-d1E01-2-0255 | 706.304 | OEt | A02 | a1 | B02 | b4 | C13 | c2 | D02 | dl | E01 |
| 2-2-d1E01-2-0256 | 707.208 | OEt | A01 | a1 | B02 | b3 | C10 | c2 | D02 | dl | E01 |
| 2-2-d1E01-2-0257 | 707.253 | OEt | A02 | a1 | B04 | b3 | C09 | c1 | D01 | dl | E01 |
| 2-2-d1E01-2-0258 | 707.257 | OEt | A02 | a1 | B05 | b3 | C08 | c1 | D01 | dl | E01 |
| 2-2-d1E01-2-0259 | 708.238 | OEt | A01 | a1 | B03 | b1 | C14 | c2 | D02 | dl | E01 |
| 2-2-d1E01-2-0260 | 708.262 | OEt | A01 | a1 | B02 | b2 | C02 | c2 | D02 | dl | E01 |
| 2-2-d1E01-2-0261 | 708.262 | OEt | A01 | a1 | B02 | b2 | C03 | c2 | D02 | dl | E01 |
| 2-2-d1E01-2-0262 | 708.299 | OEt | A02 | a1 | B03 | b4 | C12 | c2 | D02 | dl | E01 |
| 2-2-d1E01-2-0263 | 708.344 | OEt | A01 | a1 | B03 | b1 | C19 | c1 | D01 | dl | E01 |
| 2-2-d1E01-2-0264 | 709.257 | OEt | A01 | a1 | B02 | b2 | C04 | c2 | D02 | dl | E01 |
| 2-2-d1E01-2-0265 | 709.257 | OEt | A01 | a1 | B02 | b2 | C06 | c2 | D02 | dl | E01 |
| 2-2-d1E01-2-0266 | 709.257 | OEt | A01 | a1 | B02 | b2 | C07 | c2 | D02 | dl | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0267 | 709.268 | OEt | A02 | a1 | B04 | b1 | C14 | c1 | D01 | dl | E01 |
| 2-2-d1E01-2-0268 | 710.250 | OEt | A01 | a1 | B05 | b1 | C18 | c1 | D01 | dl | E01 |
| 2-2-d1E01-2-0269 | 710.277 | OEt | A02 | a1 | B02 | b1 | C02 | c2 | D02 | dl | E01 |
| 2-2-d1E01-2-0270 | 710.277 | OEt | A02 | a1 | B02 | b1 | C03 | c2 | D02 | dl | E01 |
| 2-2-d1E01-2-0271 | 710.279 | OEt | A02 | a1 | B03 | b4 | C13 | c2 | D02 | dl | E01 |
| 2-2-d1E01-2-0272 | 711.183 | OEt | A01 | a1 | B03 | b3 | C10 | c2 | D02 | dl | E01 |
| 2-2-d1E01-2-0273 | 711.255 | OEt | A01 | a1 | B04 | b4 | C05 | c1 | D01 | dl | E01 |
| 2-2-d1E01-2-0274 | 711.273 | OEt | A02 | a1 | B02 | b1 | C04 | c2 | D02 | dl | E01 |
| 2-2-d1E01-2-0275 | 712.214 | OEt | A02 | a1 | B04 | b3 | C10 | c1 | D01 | dl | E01 |
| 2-2-d1E01-2-0276 | 712.237 | OEt | A01 | a1 | B03 | b2 | C02 | c2 | D02 | dl | E01 |
| 2-2-d1E01-2-0277 | 712.237 | OEt | A01 | a1 | B03 | b2 | C03 | c2 | D02 | dl | E01 |
| 2-2-d1E01-2-0278 | 712.293 | OEt | A01 | a1 | B01 | b4 | C02 | c2 | D02 | dl | E01 |
| 2-2-d1E01-2-0279 | 713.232 | OEt | A01 | a1 | B03 | b2 | C04 | c2 | D02 | dl | E01 |
| 2-2-d1E01-2-0280 | 713.232 | OEt | A01 | a1 | B03 | b2 | C06 | c2 | D02 | dl | E01 |
| 2-2-d1E01-2-0281 | 713.232 | OEt | A01 | a1 | B03 | b2 | C07 | c2 | D02 | dl | E01 |
| 2-2-d1E01-2-0282 | 713.267 | OEt | A02 | a1 | B04 | b2 | C02 | c1 | D01 | dl | E01 |
| 2-2-d1E01-2-0283 | 713.267 | OEt | A02 | a1 | B04 | b2 | C03 | c1 | D01 | dl | E01 |
| 2-2-d1E01-2-0284 | 713.288 | OEt | A01 | a1 | B02 | b1 | C15 | c2 | D02 | dl | E01 |
| 2-2-d1E01-2-0285 | 714.247 | OEt | A02 | a1 | B01 | b3 | C09 | c2 | D02 | dl | E01 |
| 2-2-d1E01-2-0286 | 714.252 | OEt | A02 | a1 | B03 | b1 | C02 | c2 | D02 | dl | E01 |
| 2-2-d1E01-2-0287 | 714.252 | OEt | A02 | a1 | B03 | b1 | C03 | c2 | D02 | dl | E01 |
| 2-2-d1E01-2-0288 | 714.262 | OEt | A02 | a1 | B04 | b2 | C04 | c1 | D01 | dl | E01 |
| 2-2-d1E01-2-0289 | 714.262 | OEt | A02 | a1 | B04 | b2 | C06 | c1 | D01 | dl | E01 |
| 2-2-d1E01-2-0290 | 714.262 | OEt | A02 | a1 | B04 | b2 | C07 | c1 | D01 | dl | E01 |
| 2-2-d1E01-2-0291 | 714.284 | OEt | A01 | a1 | B02 | b1 | C16 | c2 | D02 | dl | E01 |
| 2-2-d1E01-2-0292 | 715.248 | OEt | A02 | a1 | B03 | b1 | C04 | c2 | D02 | dl | E01 |
| 2-2-d1E01-2-0293 | 715.279 | OEt | A01 | a1 | B02 | b1 | C17 | c2 | D02 | dl | E01 |
| 2-2-d1E01-2-0294 | 716.234 | OEt | A02 | a1 | B02 | b1 | COS | c2 | D02 | dl | E01 |
| 2-2-d1E01-2-0295 | 716.263 | OEt | A02 | a1 | B01 | b1 | C14 | c2 | D02 | dl | E01 |
| 2-2-d1E01-2-0296 | 716.288 | OEt | A01 | a1 | B02 | b4 | C01 | c2 | D02 | dl | E01 |
| 2-2-d1E01-2-0297 | 716.319 | OEt | A01 | a1 | B05 | b4 | C02 | c1 | D01 | dl | E01 |
| 2-2-d1E01-2-0298 | 716.369 | OEt | A02 | a1 | B01 | b1 | C19 | c1 | D01 | dl | E01 |
| 2-2-d1E01-2-0299 | 717.193 | OEt | A01 | a1 | B04 | b1 | C01 | c2 | D02 | dl | E01 |
| 2-2-d1E01-2-0300 | 717.247 | OEt | A02 | a1 | B02 | b3 | C08 | c2 | D02 | dl | E01 |
| 2-2-d1E01-2-0301 | 717.263 | OEt | A01 | a1 | B03 | b1 | C15 | c2 | D02 | dl | E01 |
| 2-2-d1E01-2-0302 | 718.249 | OEt | A01 | a1 | B01 | b4 | C05 | c2 | D02 | dl | E01 |
| 2-2-d1E01-2-0303 | 718.258 | OEt | A01 | a1 | B03 | b1 | C16 | c2 | D02 | dl | E01 |
| 2-2-d1E01-2-0304 | 718.273 | OEt | A02 | a1 | B05 | b3 | C09 | c1 | D01 | dl | E01 |

(continued)

[Table 9-7-2] Compound that may be contained in mixture 2-2-d1E01-2

| ID | Exact Mass | | A | a | B | b | C | c | D | d | E |
|---|---|---|---|---|---|---|---|---|---|---|---|
| 2-2-d1E01-2-0305 | 718.294 | OEt | A02 | a1 | B04 | b1 | C15 | c1 | D01 | dl | E01 |
| 2-2-d1E01-2-0306 | 719.208 | OEt | A02 | a1 | B01 | b3 | C10 | c2 | D02 | dl | E01 |
| 2-2-d1E01-2-0307 | 719.241 | OEt | A01 | a1 | B04 | b3 | C11 | c1 | D01 | dl | E01 |
| 2-2-d1E01-2-0308 | 719.254 | OEt | A01 | a1 | B03 | b1 | C17 | c2 | D02 | dl | E01 |
| 2-2-d1E01-2-0309 | 719.289 | OEt | A02 | a1 | B04 | b1 | C16 | c1 | D01 | dl | E01 |
| 2-2-d1E01-2-0310 | 720.209 | OEt | A02 | a1 | B03 | b1 | COS | c2 | D02 | dl | E01 |
| 2-2-d1E01-2-0311 | 720.240 | OEt | A01 | a1 | B02 | b1 | C18 | c2 | D02 | dl | E01 |
| 2-2-d1E01-2-0312 | 720.262 | OEt | A02 | a1 | B01 | b2 | C02 | c2 | D02 | dl | E01 |
| 2-2-d1E01-2-0313 | 720.262 | OEt | A02 | a1 | B01 | b2 | C03 | c2 | D02 | dl | E01 |
| 2-2-d1E01-2-0314 | 720.263 | OEt | A01 | a1 | B03 | b4 | C01 | c2 | D02 | dl | E01 |
| 2-2-d1E01-2-0315 | 720.284 | OEt | A02 | a1 | B04 | b1 | C17 | c1 | D01 | dl | E01 |
| 2-2-d1E01-2-0316 | 720.288 | OEt | A02 | a1 | B05 | b1 | C14 | c1 | D01 | dl | E01 |
| 2-2-d1E01-2-0317 | 721.222 | OEt | A02 | a1 | B03 | b3 | C08 | c2 | D02 | dl | E01 |
| 2-2-d1E01-2-0318 | 721.257 | OEt | A02 | a1 | B01 | b2 | C04 | c2 | D02 | dl | E01 |
| 2-2-d1E01-2-0319 | 721.257 | OEt | A02 | a1 | B01 | b2 | C06 | c2 | D02 | dl | E01 |
| 2-2-d1E01-2-0320 | 721.257 | OEt | A02 | a1 | B01 | b2 | C07 | c2 | D02 | dl | E01 |
| 2-2-d1E01-2-0321 | 721.260 | OEt | A01 | a1 | B04 | b4 | C12 | c2 | D02 | dl | E01 |
| 2-2-d1E01-2-0322 | 721.293 | OEt | A02 | a1 | B04 | b4 | C01 | c1 | D01 | dl | E01 |
| 2-2-d1E01-2-0323 | 722.275 | OEt | A01 | a1 | B05 | b4 | C05 | c1 | D01 | dl | E01 |
| 2-2-d1E01-2-0324 | 723.234 | OEt | A02 | a1 | B05 | b3 | C10 | c1 | D01 | dl | E01 |
| 2-2-d1E01-2-0325 | 723.240 | OEt | A01 | a1 | B04 | b4 | C13 | c2 | D02 | dl | E01 |
| 2-2-d1E01-2-0326 | 724.215 | OEt | A01 | a1 | B03 | b1 | C18 | c2 | D02 | dl | E01 |
| 2-2-d1E01-2-0327 | 724.287 | OEt | A02 | a1 | B05 | b2 | C02 | c1 | D01 | dl | E01 |
| 2-2-d1E01-2-0328 | 724.287 | OEt | A02 | a1 | B05 | b2 | C03 | c1 | D01 | dl | E01 |
| 2-2-d1E01-2-0329 | 725.245 | OEt | A02 | a1 | B04 | b1 | C18 | c1 | D01 | dl | E01 |
| 2-2-d1E01-2-0330 | 725.283 | OEt | A02 | a1 | B05 | b2 | C04 | c1 | D01 | dl | E01 |
| 2-2-d1E01-2-0331 | 725.283 | OEt | A02 | a1 | B05 | b2 | C06 | c1 | D01 | dl | E01 |
| 2-2-d1E01-2-0332 | 725.283 | OEt | A02 | a1 | B05 | b2 | C07 | c1 | D01 | dl | E01 |
| 2-2-d1E01-2-0333 | 725.288 | OEt | A02 | a1 | B01 | b1 | C15 | c2 | D02 | dl | E01 |
| 2-2-d1E01-2-0334 | 726.236 | OEt | A01 | a1 | B01 | b3 | C11 | c2 | D02 | dl | E01 |
| 2-2-d1E01-2-0335 | 726.284 | OEt | A02 | a1 | B01 | b1 | C16 | c2 | D02 | dl | E01 |
| 2-2-d1E01-2-0336 | 726.309 | OEt | A01 | a1 | B02 | b4 | C02 | c2 | D02 | dl | E01 |
| 2-2-d1E01-2-0337 | 727.213 | OEt | A01 | a1 | B04 | b1 | C02 | c2 | D02 | dl | E01 |
| 2-2-d1E01-2-0338 | 727.213 | OEt | A01 | a1 | B04 | b1 | C03 | c2 | D02 | dl | E01 |
| 2-2-d1E01-2-0339 | 727.279 | OEt | A02 | a1 | B01 | b1 | C17 | c2 | D02 | dl | E01 |
| 2-2-d1E01-2-0340 | 728.209 | OEt | A01 | a1 | B04 | b1 | C04 | c2 | D02 | dl | E01 |
| 2-2-d1E01-2-0341 | 728.213 | OEt | A01 | a1 | B05 | b1 | C01 | c2 | D02 | dl | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0342 | 728.263 | OEt | A02 | a1 | B02 | b3 | C09 | c2 | D02 | dl | E01 |
| 2-2-d1E01-2-0343 | 728.288 | OEt | A02 | a1 | B01 | b4 | C01 | c2 | D02 | dl | E01 |
| 2-2-d1E01-2-0344 | 729.314 | OEt | A02 | a1 | B05 | b1 | C15 | c1 | D01 | dl | E01 |
| 2-2-d1E01-2-0345 | 730.261 | OEt | A01 | a1 | B05 | b3 | C11 | c1 | D01 | dl | E01 |
| 2-2-d1E01-2-0346 | 730.278 | OEt | A02 | a1 | B02 | b1 | C14 | c2 | D02 | dl | E01 |
| 2-2-d1E01-2-0347 | 730.284 | OEt | A01 | a1 | B03 | b4 | C02 | c2 | D02 | dl | E01 |
| 2-2-d1E01-2-0348 | 730.309 | OEt | A02 | a1 | B05 | b1 | C16 | c1 | D01 | dl | E01 |
| 2-2-d1E01-2-0349 | 730.384 | OEt | A02 | a1 | B02 | b1 | C19 | c1 | D01 | dl | E01 |
| 2-2-d1E01-2-0350 | 731.304 | OEt | A02 | a1 | B05 | b1 | C17 | c1 | D01 | dl | E01 |
| 2-2-d1E01-2-0351 | 731.314 | OEt | A02 | a1 | B04 | b4 | C02 | c1 | D01 | dl | E01 |
| 2-2-d1E01-2-0352 | 732.238 | OEt | A02 | a1 | B03 | b3 | C09 | c2 | D02 | dl | E01 |
| 2-2-d1E01-2-0353 | 732.240 | OEt | A02 | a1 | B01 | b1 | C18 | c2 | D02 | dl | E01 |
| 2-2-d1E01-2-0354 | 732.265 | OEt | A01 | a1 | B02 | b4 | C05 | c2 | D02 | dl | E01 |
| 2-2-d1E01-2-0355 | 732.280 | OEt | A01 | a1 | B05 | b4 | C12 | c2 | D02 | dl | E01 |
| 2-2-d1E01-2-0356 | 732.313 | OEt | A02 | a1 | B05 | b4 | C01 | c1 | D01 | dl | E01 |
| 2-2-d1E01-2-0357 | 733.170 | OEt | A01 | a1 | B04 | b1 | COS | c2 | D02 | dl | E01 |
| 2-2-d1E01-2-0358 | 733.224 | OEt | A02 | a1 | B02 | b3 | C10 | c2 | D02 | dl | E01 |
| 2-2-d1E01-2-0359 | 734.183 | OEt | A01 | a1 | B04 | b3 | C08 | c2 | D02 | dl | E01 |
| 2-2-d1E01-2-0360 | 734.253 | OEt | A02 | a1 | B03 | b1 | C14 | c2 | D02 | dl | E01 |
| 2-2-d1E01-2-0361 | 734.260 | OEt | A01 | a1 | B05 | b4 | C13 | c2 | D02 | dl | E01 |
| 2-2-d1E01-2-0362 | 734.277 | OEt | A02 | a1 | B02 | b2 | C02 | c2 | D02 | dl | E01 |
| 2-2-d1E01-2-0363 | 734.277 | OEt | A02 | a1 | B02 | b2 | C03 | c2 | D02 | dl | E01 |
| 2-2-d1E01-2-0364 | 734.359 | OEt | A02 | a1 | B03 | b1 | C19 | c1 | D01 | dl | E01 |
| 2-2-d1E01-2-0365 | 735.273 | OEt | A02 | a1 | B02 | b2 | C04 | c2 | D02 | dl | E01 |
| 2-2-d1E01-2-0366 | 735.273 | OEt | A02 | a1 | B02 | b2 | C06 | c2 | D02 | dl | E01 |
| 2-2-d1E01-2-0367 | 735.273 | OEt | A02 | a1 | B02 | b2 | C07 | c2 | D02 | dl | E01 |
| 2-2-d1E01-2-0368 | 736.240 | OEt | A01 | a1 | B03 | b4 | COS | c2 | D02 | dl | E01 |
| 2-2-d1E01-2-0369 | 736.265 | OEt | A02 | a1 | B05 | b1 | C18 | c1 | D01 | dl | E01 |
| 2-2-d1E01-2-0370 | 737.199 | OEt | A02 | a1 | B03 | b3 | C10 | c2 | D02 | dl | E01 |
| 2-2-d1E01-2-0371 | 737.271 | OEt | A02 | a1 | B04 | b4 | COS | c1 | D01 | dl | E01 |
| 2-2-d1E01-2-0372 | 738.234 | OEt | A01 | a1 | B05 | b1 | C02 | c2 | D02 | dl | E01 |
| 2-2-d1E01-2-0373 | 738.234 | OEt | A01 | a1 | B05 | b1 | C03 | c2 | D02 | dl | E01 |
| 2-2-d1E01-2-0374 | 738.252 | OEt | A02 | a1 | B03 | b2 | C02 | c2 | D02 | dl | E01 |
| 2-2-d1E01-2-0375 | 738.252 | OEt | A02 | a1 | B03 | b2 | C03 | c2 | D02 | dl | E01 |
| 2-2-d1E01-2-0376 | 738.309 | OEt | A02 | a1 | B01 | b4 | C02 | c2 | D02 | dl | E01 |
| 2-2-d1E01-2-0377 | 739.229 | OEt | A01 | a1 | B05 | b1 | C04 | c2 | D02 | dl | E01 |
| 2-2-d1E01-2-0378 | 739.248 | OEt | A02 | a1 | B03 | b2 | C04 | c2 | D02 | dl | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0379 | 739.248 | OEt | A02 | a1 | B03 | b2 | C06 | c2 | D02 | dl | E01 |
| 2-2-d1E01-2-0380 | 739.248 | OEt | A02 | a1 | B03 | b2 | C07 | c2 | D02 | dl | E01 |
| 2-2-d1E01-2-0381 | 739.304 | OEt | A02 | a1 | B02 | b1 | C15 | c2 | D02 | dl | E01 |
| 2-2-d1E01-2-0382 | 740.252 | OEt | A01 | a1 | B02 | b3 | C11 | c2 | D02 | dl | E01 |
| 2-2-d1E01-2-0383 | 740.299 | OEt | A02 | a1 | B02 | b1 | C16 | c2 | D02 | dl | E01 |
| 2-2-d1E01-2-0384 | 741.294 | OEt | A02 | a1 | B02 | b1 | C17 | c2 | D02 | dl | E01 |
| 2-2-d1E01-2-0385 | 742.304 | OEt | A02 | a1 | B02 | b4 | C01 | c2 | D02 | dl | E01 |
| 2-2-d1E01-2-0386 | 742.334 | OEt | A02 | a1 | B05 | b4 | C02 | c1 | D01 | dl | E01 |
| 2-2-d1E01-2-0387 | 743.208 | OEt | A02 | a1 | B04 | b1 | C01 | c2 | D02 | dl | E01 |
| 2-2-d1E01-2-0388 | 743.279 | OEt | A02 | a1 | B03 | b1 | C15 | c2 | D02 | dl | E01 |
| 2-2-d1E01-2-0389 | 744.190 | OEt | A01 | a1 | B05 | b1 | C05 | c2 | D02 | dl | E01 |
| 2-2-d1E01-2-0390 | 744.227 | OEt | A01 | a1 | B03 | b3 | C11 | c2 | D02 | dl | E01 |
| 2-2-d1E01-2-0391 | 744.265 | OEt | A02 | a1 | B01 | b4 | C05 | c2 | D02 | dl | E01 |
| 2-2-d1E01-2-0392 | 744.274 | OEt | A02 | a1 | B03 | b1 | C16 | c2 | D02 | dl | E01 |
| 2-2-d1E01-2-0393 | 745.199 | OEt | A01 | a1 | B04 | b3 | C09 | c2 | D02 | dl | E01 |
| 2-2-d1E01-2-0394 | 745.203 | OEt | A01 | a1 | B05 | b3 | C08 | c2 | D02 | dl | E01 |
| 2-2-d1E01-2-0395 | 745.257 | OEt | A02 | a1 | B04 | b3 | C11 | c1 | D01 | dl | E01 |
| 2-2-d1E01-2-0396 | 745.269 | OEt | A02 | a1 | B03 | b1 | C17 | c2 | D02 | dl | E01 |
| 2-2-d1E01-2-0397 | 746.256 | OEt | A02 | a1 | B02 | b1 | C18 | c2 | D02 | dl | E01 |
| 2-2-d1E01-2-0398 | 746.279 | OEt | A02 | a1 | B03 | b4 | C01 | c2 | D02 | dl | E01 |
| 2-2-d1E01-2-0399 | 747.215 | OEt | A01 | a1 | B04 | b1 | C14 | c2 | D02 | dl | E01 |
| 2-2-d1E01-2-0400 | 747.276 | OEt | A02 | a1 | B04 | b4 | C12 | c2 | D02 | dl | E01 |
| 2-2-d1E01-2-0401 | 747.320 | OEt | A01 | a1 | B04 | b1 | C19 | c1 | D01 | dl | E01 |
| 2-2-d1E01-2-0402 | 748.291 | OEt | A02 | a1 | B05 | b4 | C05 | c1 | D01 | dl | E01 |
| 2-2-d1E01-2-0403 | 749.255 | OEt | A02 | a1 | B04 | b4 | C13 | c2 | D02 | dl | E01 |
| 2-2-d1E01-2-0404 | 750.160 | OEt | A01 | a1 | B04 | b3 | C10 | c2 | D02 | dl | E01 |
| 2-2-d1E01-2-0405 | 750.231 | OEt | A02 | a1 | B03 | b1 | C18 | c2 | D02 | dl | E01 |
| 2-2-d1E01-2-0406 | 751.213 | OEt | A01 | a1 | B04 | b2 | C02 | c2 | D02 | dl | E01 |
| 2-2-d1E01-2-0407 | 751.213 | OEt | A01 | a1 | B04 | b2 | C03 | c2 | D02 | dl | E01 |
| 2-2-d1E01-2-0408 | 752.209 | OEt | A01 | a1 | B04 | b2 | C04 | c2 | D02 | dl | E01 |
| 2-2-d1E01-2-0409 | 752.209 | OEt | A01 | a1 | B04 | b2 | C06 | c2 | D02 | dl | E01 |
| 2-2-d1E01-2-0410 | 752.209 | OEt | A01 | a1 | B04 | b2 | C07 | c2 | D02 | dl | E01 |
| 2-2-d1E01-2-0411 | 752.252 | OEt | A02 | a1 | B01 | b3 | C11 | c2 | D02 | dl | E01 |
| 2-2-d1E01-2-0412 | 752.324 | OEt | A02 | a1 | B02 | b4 | C02 | c2 | D02 | dl | E01 |
| 2-2-d1E01-2-0413 | 753.229 | OEt | A02 | a1 | B04 | b1 | C02 | c2 | D02 | dl | E01 |
| 2-2-d1E01-2-0414 | 753.229 | OEt | A02 | a1 | B04 | b1 | C03 | c2 | D02 | dl | E01 |
| 2-2-d1E01-2-0415 | 754.224 | OEt | A02 | a1 | B04 | b1 | C04 | c2 | D02 | dl | E01 |

(continued)

[Table 9-7-2] Compound that may be contained in mixture 2-2-d1E01-2

| ID | Exact Mass | | A | a | B | b | C | c | D | d | E |
|---|---|---|---|---|---|---|---|---|---|---|---|
| 2-2-d1E01-2-0416 | 754.228 | OEt | A02 | a1 | B05 | b1 | C01 | c2 | D02 | dl | E01 |
| 2-2-d1E01-2-0417 | 754.315 | OEt | A01 | a1 | B01 | b1 | C19 | c2 | D02 | dl | E01 |
| 2-2-d1E01-2-0418 | 756.219 | OEt | A01 | a1 | B05 | b3 | C09 | c2 | D02 | dl | E01 |
| 2-2-d1E01-2-0419 | 756.240 | OEt | A01 | a1 | B04 | b1 | C15 | c2 | D02 | dl | E01 |
| 2-2-d1E01-2-0420 | 756.277 | OEt | A02 | a1 | B05 | b3 | C11 | c1 | D01 | dl | E01 |
| 2-2-d1E01-2-0421 | 756.299 | OEt | A02 | a1 | B03 | b4 | C02 | c2 | D02 | dl | E01 |
| 2-2-d1E01-2-0422 | 757.235 | OEt | A01 | a1 | B04 | b1 | C16 | c2 | D02 | dl | E01 |
| 2-2-d1E01-2-0423 | 758.230 | OEt | A01 | a1 | B04 | b1 | C17 | c2 | D02 | dl | E01 |
| 2-2-d1E01-2-0424 | 758.235 | OEt | A01 | a1 | B05 | b1 | C14 | c2 | D02 | dl | E01 |
| 2-2-d1E01-2-0425 | 758.281 | OEt | A02 | a1 | B02 | b4 | COS | c2 | D02 | dl | E01 |
| 2-2-d1E01-2-0426 | 758.296 | OEt | A02 | a1 | B05 | b4 | C12 | c2 | D02 | dl | E01 |
| 2-2-d1E01-2-0427 | 758.340 | OEt | A01 | a1 | B05 | b1 | C19 | c1 | D01 | dl | E01 |
| 2-2-d1E01-2-0428 | 759.186 | OEt | A02 | a1 | B04 | b1 | C05 | c2 | D02 | dl | E01 |
| 2-2-d1E01-2-0429 | 759.240 | OEt | A01 | a1 | B04 | b4 | C01 | c2 | D02 | dl | E01 |
| 2-2-d1E01-2-0430 | 760.199 | OEt | A02 | a1 | B04 | b3 | C08 | c2 | D02 | dl | E01 |
| 2-2-d1E01-2-0431 | 760.275 | OEt | A02 | a1 | B05 | b4 | C13 | c2 | D02 | dl | E01 |
| 2-2-d1E01-2-0432 | 761.180 | OEt | A01 | a1 | B05 | b3 | C10 | c2 | D02 | dl | E01 |
| 2-2-d1E01-2-0433 | 762.234 | OEt | A01 | a1 | B05 | b2 | C02 | c2 | D02 | dl | E01 |
| 2-2-d1E01-2-0434 | 762.234 | OEt | A01 | a1 | B05 | b2 | C03 | c2 | D02 | dl | E01 |
| 2-2-d1E01-2-0435 | 762.256 | OEt | A02 | a1 | B03 | b4 | COS | c2 | D02 | dl | E01 |
| 2-2-d1E01-2-0436 | 763.192 | OEt | A01 | a1 | B04 | b1 | C18 | c2 | D02 | dl | E01 |
| 2-2-d1E01-2-0437 | 763.229 | OEt | A01 | a1 | B05 | b2 | C04 | c2 | D02 | dl | E01 |
| 2-2-d1E01-2-0438 | 763.229 | OEt | A01 | a1 | B05 | b2 | C06 | c2 | D02 | dl | E01 |
| 2-2-d1E01-2-0439 | 763.229 | OEt | A01 | a1 | B05 | b2 | C07 | c2 | D02 | dl | E01 |
| 2-2-d1E01-2-0440 | 764.249 | OEt | A02 | a1 | B05 | b1 | C02 | c2 | D02 | dl | E01 |
| 2-2-d1E01-2-0441 | 764.249 | OEt | A02 | a1 | B05 | b1 | C03 | c2 | D02 | dl | E01 |
| 2-2-d1E01-2-0442 | 765.244 | OEt | A02 | a1 | B05 | b1 | C04 | c2 | D02 | dl | E01 |
| 2-2-d1E01-2-0443 | 766.267 | OEt | A02 | a1 | B02 | b3 | C11 | c2 | D02 | dl | E01 |
| 2-2-d1E01-2-0444 | 767.260 | OEt | A01 | a1 | B05 | b1 | C15 | c2 | D02 | dl | E01 |
| 2-2-d1E01-2-0445 | 768.255 | OEt | A01 | a1 | B05 | b1 | C16 | c2 | D02 | dl | E01 |
| 2-2-d1E01-2-0446 | 768.331 | OEt | A01 | a1 | B02 | b1 | C19 | c2 | D02 | dl | E01 |
| 2-2-d1E01-2-0447 | 769.251 | OEt | A01 | a1 | B05 | b1 | C17 | c2 | D02 | dl | E01 |
| 2-2-d1E01-2-0448 | 769.260 | OEt | A01 | a1 | B04 | b4 | C02 | c2 | D02 | dl | E01 |
| 2-2-d1E01-2-0449 | 770.206 | OEt | A02 | a1 | B05 | b1 | C05 | c2 | D02 | dl | E01 |
| 2-2-d1E01-2-0450 | 770.242 | OEt | A02 | a1 | B03 | b3 | C11 | c2 | D02 | dl | E01 |
| 2-2-d1E01-2-04SI | 770.260 | OEt | A01 | a1 | B05 | b4 | C01 | c2 | D02 | dl | E01 |
| 2-2-d1E01-2-0452 | 771.215 | OEt | A02 | a1 | B04 | b3 | C09 | c2 | D02 | dl | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0453 | 771.219 | OEt | A02 | a1 | B05 | b3 | C08 | c2 | D02 | dl | E01 |
| 2-2-d1E01-2-0454 | 772.305 | OEt | A01 | a1 | B03 | b1 | C19 | c2 | D02 | dl | E01 |
| 2-2-d1E01-2-0455 | 773.230 | OEt | A02 | a1 | B04 | b1 | C14 | c2 | D02 | dl | E01 |
| 2-2-d1E01-2-0456 | 773.336 | OEt | A02 | a1 | B04 | b1 | C19 | c1 | D01 | dl | E01 |
| 2-2-d1E01-2-0457 | 774.212 | OEt | A01 | a1 | B05 | b1 | C18 | c2 | D02 | dl | E01 |
| 2-2-d1E01-2-0458 | 775.217 | OEt | A01 | a1 | B04 | b4 | COS | c2 | D02 | dl | E01 |
| 2-2-d1E01-2-0459 | 776.176 | OEt | A02 | a1 | B04 | b3 | C10 | c2 | D02 | dl | E01 |
| 2-2-d1E01-2-0460 | 777.229 | OEt | A02 | a1 | B04 | b2 | C02 | c2 | D02 | dl | E01 |
| 2-2-d1E01-2-0461 | 777.229 | OEt | A02 | a1 | B04 | b2 | C03 | c2 | D02 | dl | E01 |
| 2-2-d1E01-2-0462 | 778.224 | OEt | A02 | a1 | B04 | b2 | C04 | c2 | D02 | dl | E01 |
| 2-2-d1E01-2-0463 | 778.224 | OEt | A02 | a1 | B04 | b2 | C06 | c2 | D02 | dl | E01 |
| 2-2-d1E01-2-0464 | 778.224 | OEt | A02 | a1 | B04 | b2 | C07 | c2 | D02 | dl | E01 |
| 2-2-d1E01-2-0465 | 780.280 | OEt | A01 | a1 | B05 | b4 | C02 | c2 | D02 | dl | E01 |
| 2-2-d1E01-2-0466 | 780.331 | OEt | A02 | a1 | B01 | b1 | C19 | c2 | D02 | dl | E01 |
| 2-2-d1E01-2-0467 | 782.235 | OEt | A02 | a1 | B05 | b3 | C09 | c2 | D02 | dl | E01 |
| 2-2-d1E01-2-0468 | 782.256 | OEt | A02 | a1 | B04 | b1 | C15 | c2 | D02 | dl | E01 |
| 2-2-d1E01-2-0469 | 783.203 | OEt | A01 | a1 | B04 | b3 | C11 | c2 | D02 | dl | E01 |
| 2-2-d1E01-2-0470 | 783.251 | OEt | A02 | a1 | B04 | b1 | C16 | c2 | D02 | dl | E01 |
| 2-2-d1E01-2-0471 | 784.246 | OEt | A02 | a1 | B04 | b1 | C17 | c2 | D02 | dl | E01 |
| 2-2-d1E01-2-0472 | 784.250 | OEt | A02 | a1 | B05 | b1 | C14 | c2 | D02 | dl | E01 |
| 2-2-d1E01-2-0473 | 784.356 | OEt | A02 | a1 | B05 | b1 | C19 | c1 | D01 | dl | E01 |
| 2-2-d1E01-2-0474 | 785.255 | OEt | A02 | a1 | B04 | b4 | C01 | c2 | D02 | dl | E01 |
| 2-2-d1E01-2-0475 | 786.237 | OEt | A01 | a1 | B05 | b4 | C05 | c2 | D02 | dl | E01 |
| 2-2-d1E01-2-0476 | 787.196 | OEt | A02 | a1 | B05 | b3 | C10 | c2 | D02 | dl | E01 |
| 2-2-d1E01-2-0477 | 788.249 | OEt | A02 | a1 | B05 | b2 | C02 | c2 | D02 | dl | E01 |
| 2-2-d1E01-2-0478 | 788.249 | OEt | A02 | a1 | B05 | b2 | C03 | c2 | D02 | dl | E01 |
| 2-2-d1E01-2-0479 | 789.207 | OEt | A02 | a1 | B04 | b1 | C18 | c2 | D02 | dl | E01 |
| 2-2-21E01-2-0480 | 789.244 | OEt | A02 | a1 | B05 | b2 | C04 | c2 | D02 | dl | E01 |
| 2-2-d1E01-2-0481 | 789.244 | OEt | A02 | a1 | B05 | b2 | C06 | c2 | D02 | dl | E01 |
| 2-2-d1E01-2-0482 | 789.244 | OEt | A02 | a1 | B05 | b2 | C07 | c2 | D02 | dl | E01 |
| 2-2-d1E01-2-0483 | 793.276 | OEt | A02 | a1 | B05 | b1 | C15 | c2 | D02 | dl | E01 |
| 2-2-d1E01-2-0484 | 794.223 | OEt | A01 | a1 | B05 | b3 | C11 | c2 | D02 | dl | E01 |
| 2-2-d1E01-2-0485 | 794.271 | OEt | A02 | a1 | B05 | b1 | C16 | c2 | D02 | dl | E01 |
| 2-2-d1E01-2-0486 | 794.346 | OEt | A02 | a1 | B02 | b1 | C19 | c2 | D02 | dl | E01 |
| 2-2-d1E01-2-0487 | 795.266 | OEt | A02 | a1 | B05 | b1 | C17 | c2 | D02 | dl | E01 |
| 2-2-d1E01-2-0488 | 795.276 | OEt | A02 | a1 | B04 | b4 | C02 | c2 | D02 | dl | E01 |
| 2-2-d1E01-2-0489 | 796.275 | OEt | A02 | a1 | B05 | b4 | C01 | c2 | D02 | dl | E01 |

(continued)

| [Table 9-7-2] Compound that may be contained in mixture 2-2-d1E01-2 | | | | | | | | | | | |
|---|---|---|---|---|---|---|---|---|---|---|---|
| ID | Exact Mass | | A | a | B | b | C | c | D | d | E |
| 2-2-d1E01-2-0490 | 798.321 | OEt | A02 | a1 | B03 | b1 | C19 | c2 | D02 | dl | E01 |
| 2-2-d1E01-2-0491 | 800.227 | OEt | A02 | a1 | B05 | b1 | C18 | c2 | D02 | dl | E01 |
| 2-2-d1E01-2-0492 | 801.232 | OEt | A02 | a1 | B04 | b4 | C05 | c2 | D02 | dl | E01 |
| 2-2-d1E01-2-0493 | 806.296 | OEt | A02 | a1 | B05 | b4 | C02 | c2 | D02 | dl | E01 |
| 2-2-d1E01-2-0494 | 809.219 | OEt | A02 | a1 | B04 | b3 | C11 | c2 | D02 | dl | E01 |
| 2-2-d1E01-2-0495 | 811.282 | OEt | A01 | a1 | B04 | b1 | C19 | c2 | D02 | dl | E01 |
| 2-2-d1E01-2-0496 | 812.253 | OEt | A02 | a1 | B05 | b4 | C05 | c2 | D02 | dl | E01 |
| 2-2-d1E01-2-0497 | 820.239 | OEt | A02 | a1 | B05 | b3 | C11 | c2 | D02 | dl | E01 |
| 2-2-d1E01-2-0498 | 822.302 | OEt | A01 | a1 | B05 | b1 | C19 | c2 | D02 | dl | E01 |
| 2-2-d1E01-2-0499 | 837.298 | OEt | A02 | a1 | B04 | b1 | C19 | c2 | D02 | dl | E01 |
| 2-2-d1E01-2-0500 | 848.318 | OEt | A02 | a1 | B05 | b1 | C19 | c2 | D02 | dl | E01 |

[Table 500]

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0001 | 612.258 | OEt | A01 | a1 | B01 | b1 | C01 | c1 | D01 | dl | E02 |
| 2-2-d1E02-2-0002 | 616.326 | OEt | A01 | a1 | B01 | b4 | C12 | c1 | D01 | dl | E02 |
| 2-2-d1E02-2-0003 | 618.305 | OEt | A01 | a1 | B01 | b4 | C13 | c1 | D01 | dl | E02 |
| 2-2-d1E02-2-0004 | 622.279 | OEt | A01 | a1 | B01 | b1 | C02 | c1 | D01 | dl | E02 |
| 2-2-d1E02-2-0005 | 622.279 | OEt | A01 | a1 | B01 | b1 | C03 | c1 | D01 | dl | E02 |
| 2-2-d1E02-2-0006 | 623.274 | OEt | A01 | a1 | B01 | b1 | C04 | c1 | D01 | dl | E02 |
| 2-2-d1E02-2-0007 | 626.274 | OEt | A01 | a1 | B02 | b1 | C01 | c1 | D01 | dl | E02 |
| 2-2-d1E02-2-0008 | 628.236 | OEt | A01 | a1 | B01 | b1 | COS | c1 | D01 | dl | E02 |
| 2-2-d1E02-2-0009 | 629.249 | OEt | A01 | a1 | B01 | b3 | C08 | c1 | D01 | dl | E02 |
| 2-2-d1E02-2-0010 | 630.249 | OEt | A01 | a1 | B03 | b1 | C01 | c1 | D01 | dl | E02 |
| 2-2-d1E02-2-0011 | 630.342 | OEt | A01 | a1 | B02 | b4 | C12 | c1 | D01 | dl | E02 |
| 2-2-d1E02-2-0012 | 632.321 | OEt | A01 | a1 | B02 | b4 | C13 | c1 | D01 | dl | E02 |
| 2-2-d1E02-2-0013 | 634.317 | OEt | A01 | a1 | B03 | b4 | C12 | c1 | D01 | dl | E02 |
| 2-2-d1E02-2-0014 | 636.295 | OEt | A01 | a1 | B02 | b1 | C02 | c1 | D01 | dl | E02 |
| 2-2-d1E02-2-0015 | 636.295 | OEt | A01 | a1 | B02 | b1 | C03 | c1 | D01 | dl | E02 |
| 2-2-d1E02-2-0016 | 636.296 | OEt | A01 | a1 | B03 | b4 | C13 | c1 | D01 | dl | E02 |
| 2-2-d1E02-2-0017 | 637.290 | OEt | A01 | a1 | B02 | b1 | C04 | c1 | D01 | dl | E02 |
| 2-2-d1E02-2-0018 | 638.274 | OEt | A02 | a1 | B01 | b1 | C01 | c1 | D01 | dl | E02 |
| 2-2-d1E02-2-0019 | 640.265 | OEt | A01 | a1 | B01 | b3 | C09 | c1 | D01 | dl | E02 |
| 2-2-d1E02-2-0020 | 640.270 | OEt | A01 | a1 | B03 | b1 | C02 | c1 | D01 | dl | E02 |
| 2-2-d1E02-2-0021 | 640.270 | OEt | A01 | a1 | B03 | b1 | C03 | c1 | D01 | dl | E02 |

(continued)

| [Table 9-7-3] Compound that may be contained in mixture 2-2-dIE02-2 | | | | | | | | | | | |
|---|---|---|---|---|---|---|---|---|---|---|---|
| ID | Exact Mass | | A | a | B | b | C | c | D | d | E |
| 2-2-d1E02-2-0022 | 641.265 | OEt | A01 | a1 | B03 | b1 | C04 | c1 | D01 | dl | E02 |
| 2-2-d1E02-2-0023 | 642.251 | OEt | A01 | a1 | B02 | b1 | C05 | c1 | D01 | dl | E02 |
| 2-2-d1E02-2-0024 | 642.280 | OEt | A01 | a1 | B01 | b1 | C14 | c1 | D01 | dl | E02 |
| 2-2-d1E02-2-0025 | 642.342 | OEt | A02 | a1 | B01 | b4 | C12 | c1 | D01 | dl | E02 |
| 2-2-d1E02-2-0026 | 643.264 | OEt | A01 | a1 | B02 | b3 | C08 | c1 | D01 | dl | E02 |
| 2-2-d1E02-2-0027 | 644.321 | OEt | A02 | a1 | B01 | b4 | C13 | c1 | D01 | dl | E02 |
| 2-2-d1E02-2-0028 | 645.226 | OEt | A01 | a1 | B01 | b3 | C10 | c1 | D01 | dl | E02 |
| 2-2-d1E02-2-0029 | 646.226 | OEt | A01 | a1 | B03 | b1 | COS | c1 | D01 | dl | E02 |
| 2-2-d1E02-2-0030 | 646.279 | OEt | A01 | a1 | B01 | b2 | C02 | c1 | D01 | dl | E02 |
| 2-2-d1E02-2-0031 | 646.279 | OEt | A01 | a1 | B01 | b2 | C03 | c1 | D01 | dl | E02 |
| 2-2-d1E02-2-0032 | 647.239 | OEt | A01 | a1 | B03 | b3 | C08 | c1 | D01 | dl | E02 |
| 2-2-d1E02-2-0033 | 647.274 | OEt | A01 | a1 | B01 | b2 | C04 | c1 | D01 | dl | E02 |
| 2-2-d1E02-2-0034 | 647.274 | OEt | A01 | a1 | B01 | b2 | C06 | c1 | D01 | dl | E02 |
| 2-2-d1E02-2-0035 | 647.274 | OEt | A01 | a1 | B01 | b2 | C07 | c1 | D01 | dl | E02 |
| 2-2-d1E02-2-0036 | 648.295 | OEt | A02 | a1 | B01 | b1 | C02 | c1 | D01 | dl | E02 |
| 2-2-d1E02-2-0037 | 648.295 | OEt | A02 | a1 | B01 | b1 | C03 | c1 | D01 | dl | E02 |
| 2-2-d1E02-2-0038 | 649.290 | OEt | A02 | a1 | B01 | b1 | C04 | c1 | D01 | dl | E02 |
| 2-2-d1E02-2-0039 | 651.306 | OEt | A01 | a1 | B01 | b1 | C15 | c1 | D01 | dl | E02 |
| 2-2-d1E02-2-0040 | 652.290 | OEt | A02 | a1 | B02 | b1 | C01 | c1 | D01 | dl | E02 |
| 2-2-d1E02-2-0041 | 652.301 | OEt | A01 | a1 | B01 | b1 | C16 | c1 | D01 | dl | E02 |
| 2-2-d1E02-2-0042 | 653.296 | OEt | A01 | a1 | B01 | b1 | C17 | c1 | D01 | dl | E02 |
| 2-2-d1E02-2-0043 | 654.251 | OEt | A02 | a1 | B01 | b1 | C05 | c1 | D01 | dl | E02 |
| 2-2-d1E02-2-0044 | 654.280 | OEt | A01 | a1 | B02 | b3 | C09 | c1 | D01 | dl | E02 |
| 2-2-d1E02-2-0045 | 654.305 | OEt | A01 | a1 | B01 | b4 | C01 | c1 | D01 | dl | E02 |
| 2-2-d1E02-2-0046 | 655.264 | OEt | A02 | a1 | B01 | b3 | C08 | c1 | D01 | dl | E02 |
| 2-2-d1E02-2-0047 | 656.265 | OEt | A02 | a1 | B03 | b1 | C01 | c1 | D01 | dl | E02 |
| 2-2-d1E02-2-0048 | 656.296 | OEt | A01 | a1 | B02 | b1 | C14 | c1 | D01 | dl | E02 |
| 2-2-d1E02-2-0049 | 656.357 | OEt | A02 | a1 | B02 | b4 | C12 | c1 | D01 | dl | E02 |
| 2-2-d1E02-2-0050 | 658.255 | OEt | A01 | a1 | B03 | b3 | C09 | c1 | D01 | dl | E02 |
| 2-2-d1E02-2-0051 | 658.257 | OEt | A01 | a1 | B01 | b1 | C18 | c1 | D01 | dl | E02 |
| 2-2-d1E02-2-0052 | 658.337 | OEt | A02 | a1 | B02 | b4 | C13 | c1 | D01 | dl | E02 |
| 2-2-d1E02-2-0053 | 659.241 | OEt | A01 | a1 | B02 | b3 | C10 | c1 | D01 | dl | E02 |
| 2-2-d1E02-2-0054 | 660.271 | OEt | A01 | a1 | B03 | b1 | C14 | c1 | D01 | dl | E02 |
| 2-2-d1E02-2-0055 | 660.295 | OEt | A01 | a1 | B02 | b2 | C02 | c1 | D01 | dl | E02 |
| 2-2-d1E02-2-0056 | 660.295 | OEt | A01 | a1 | B02 | b2 | C03 | c1 | D01 | dl | E02 |
| 2-2-d1E02-2-0057 | 660.332 | OEt | A02 | a1 | B03 | b4 | C12 | c1 | D01 | dl | E02 |
| 2-2-d1E02-2-0058 | 661.290 | OEt | A01 | a1 | B02 | b2 | C04 | c1 | D01 | dl | E02 |
| 2-2-d1E02-2-0059 | 661.290 | OEt | A01 | a1 | B02 | b2 | C06 | c1 | D01 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0060 | 661.290 | OEt | A01 | a1 | B02 | b2 | C07 | c1 | D01 | dl | E02 |
| 2-2-d1E02-2-0061 | 662.310 | OEt | A02 | a1 | B02 | b1 | C02 | c1 | D01 | dl | E02 |
| 2-2-d1E02-2-0062 | 662.310 | OEt | A02 | a1 | B02 | b1 | C03 | c1 | D01 | dl | E02 |
| 2-2-d1E02-2-0063 | 662.312 | OEt | A02 | a1 | B03 | b4 | C13 | c1 | D01 | dl | E02 |
| 2-2-d1E02-2-0064 | 663.216 | OEt | A01 | a1 | B03 | b3 | C10 | c1 | D01 | dl | E02 |
| 2-2-d1E02-2-0065 | 663.306 | OEt | A02 | a1 | B02 | b1 | C04 | c1 | D01 | dl | E02 |
| 2-2-d1E02-2-0066 | 664.270 | OEt | A01 | a1 | B03 | b2 | C02 | c1 | D01 | dl | E02 |
| 2-2-d1E02-2-0067 | 664.270 | OEt | A01 | a1 | B03 | b2 | C03 | c1 | D01 | dl | E02 |
| 2-2-d1E02-2-0068 | 664.326 | OEt | A01 | a1 | B01 | b4 | C02 | c1 | D01 | dl | E02 |
| 2-2-d1E02-2-0069 | 665.265 | OEt | A01 | a1 | B03 | b2 | C04 | c1 | D01 | dl | E02 |
| 2-2-d1E02-2-0070 | 665.265 | OEt | A01 | a1 | B03 | b2 | C06 | c1 | D01 | dl | E02 |
| 2-2-d1E02-2-0071 | 665.265 | OEt | A01 | a1 | B03 | b2 | C07 | c1 | D01 | dl | E02 |
| 2-2-d1E02-2-0072 | 665.321 | OEt | A01 | a1 | B02 | b1 | C15 | c1 | D01 | dl | E02 |
| 2-2-d1E02-2-0073 | 666.280 | OEt | A02 | a1 | B01 | b3 | C09 | c1 | D01 | dl | E02 |
| 2-2-d1E02-2-0074 | 666.285 | OEt | A02 | a1 | B03 | b1 | C02 | c1 | D01 | dl | E02 |
| 2-2-d1E02-2-0075 | 666.285 | OEt | A02 | a1 | B03 | b1 | C03 | c1 | D01 | dl | E02 |
| 2-2-d1E02-2-0076 | 666.317 | OEt | A01 | a1 | B02 | b1 | C16 | c1 | D01 | dl | E02 |
| 2-2-d1E02-2-0077 | 667.281 | OEt | A02 | a1 | B03 | b1 | C04 | c1 | D01 | dl | E02 |
| 2-2-d1E02-2-0078 | 667.312 | OEt | A01 | a1 | B02 | b1 | C17 | c1 | D01 | dl | E02 |
| 2-2-d1E02-2-0079 | 668.267 | OEt | A02 | a1 | B02 | b1 | C05 | c1 | D01 | dl | E02 |
| 2-2-d1E02-2-0080 | 668.296 | OEt | A02 | a1 | B01 | b1 | C14 | c1 | D01 | dl | E02 |
| 2-2-d1E02-2-0081 | 668.321 | OEt | A01 | a1 | B02 | b4 | C01 | c1 | D01 | dl | E02 |
| 2-2-d1E02-2-0082 | 669.226 | OEt | A01 | a1 | B04 | b1 | C01 | c1 | D01 | dl | E02 |
| 2-2-d1E02-2-0083 | 669.280 | OEt | A02 | a1 | B02 | b3 | C08 | c1 | D01 | dl | E02 |
| 2-2-d1E02-2-0084 | 669.296 | OEt | A01 | a1 | B03 | b1 | C15 | c1 | D01 | dl | E02 |
| 2-2-d1E02-2-0085 | 670.283 | OEt | A01 | a1 | B01 | b4 | C05 | c1 | D01 | dl | E02 |
| 2-2-d1E02-2-0086 | 670.292 | OEt | A01 | a1 | B03 | b1 | C16 | c1 | D01 | dl | E02 |
| 2-2-d1E02-2-0087 | 671.241 | OEt | A02 | a1 | B01 | b3 | C10 | c1 | D01 | dl | E02 |
| 2-2-d1E02-2-0088 | 671.287 | OEt | A01 | a1 | B03 | b1 | C17 | c1 | D01 | dl | E02 |
| 2-2-d1E02-2-0089 | 672.242 | OEt | A02 | a1 | B03 | b1 | C05 | c1 | D01 | dl | E02 |
| 2-2-d1E02-2-0090 | 672.273 | OEt | A01 | a1 | B02 | b1 | C18 | c1 | D01 | dl | E02 |
| 2-2-d1E02-2-0091 | 672.295 | OEt | A02 | a1 | B01 | b2 | C02 | c1 | D01 | dl | E02 |
| 2-2-d1E02-2-0092 | 672.295 | OEt | A02 | a1 | B01 | b2 | C03 | c1 | D01 | dl | E02 |
| 2-2-d1E02-2-0093 | 672.296 | OEt | A01 | a1 | B03 | b4 | C01 | c1 | D01 | dl | E02 |
| 2-2-d1E02-2-0094 | 673.255 | OEt | A02 | a1 | B03 | b3 | C08 | c1 | D01 | dl | E02 |
| 2-2-d1E02-2-0095 | 673.290 | OEt | A02 | a1 | B01 | b2 | C04 | c1 | D01 | dl | E02 |
| 2-2-d1E02-2-0096 | 673.290 | OEt | A02 | a1 | B01 | b2 | C06 | c1 | D01 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0097 | 673.290 | OEt | A02 | a1 | B01 | b2 | C07 | c1 | D01 | dl | E02 |
| 2-2-d1E02-2-0098 | 673.293 | OEt | A01 | a1 | B04 | b4 | C12 | c1 | D01 | dl | E02 |
| 2-2-d1E02-2-0099 | 675.273 | OEt | A01 | a1 | B04 | b4 | C13 | c1 | D01 | dl | E02 |
| 2-2-d1E02-2-0100 | 676.220 | OEt | A01 | a1 | B01 | b1 | C01 | c2 | D02 | dl | E02 |
| 2-2-d1E02-2-0101 | 676.248 | OEt | A01 | a1 | B03 | b1 | C18 | c1 | D01 | dl | E02 |
| 2-2-d1E02-2-0102 | 677.321 | OEt | A02 | a1 | B01 | b1 | C15 | c1 | D01 | dl | E02 |
| 2-2-d1E02-2-0103 | 678.269 | OEt | A01 | a1 | B01 | b3 | C11 | c1 | D01 | dl | E02 |
| 2-2-d1E02-2-0104 | 678.317 | OEt | A02 | a1 | B01 | b1 | C16 | c1 | D01 | dl | E02 |
| 2-2-d1E02-2-0105 | 678.342 | OEt | A01 | a1 | B02 | b4 | C02 | c1 | D01 | dl | E02 |
| 2-2-d1E02-2-0106 | 679.246 | OEt | A01 | a1 | B04 | b1 | C02 | c1 | D01 | dl | E02 |
| 2-2-d1E02-2-0107 | 679.246 | OEt | A01 | a1 | B04 | b1 | C03 | c1 | D01 | dl | E02 |
| 2-2-d1E02-2-0108 | 679.312 | OEt | A02 | a1 | B01 | b1 | C17 | c1 | D01 | dl | E02 |
| 2-2-d1E02-2-0109 | 680.242 | OEt | A01 | a1 | B04 | b1 | C04 | c1 | D01 | dl | E02 |
| 2-2-d1E02-2-0110 | 680.246 | OEt | A01 | a1 | B05 | b1 | C01 | c1 | D01 | dl | E02 |
| 2-2-d1E02-2-0111 | 680.288 | OEt | A01 | a1 | B01 | b4 | C12 | c2 | D02 | dl | E02 |
| 2-2-d1E02-2-0112 | 680.296 | OEt | A02 | a1 | B02 | b3 | C09 | c1 | D01 | dl | E02 |
| 2-2-d1E02-2-0113 | 680.321 | OEt | A02 | a1 | B01 | b4 | C01 | c1 | D01 | dl | E02 |
| 2-2-d1E02-2-0114 | 682.267 | OEt | A01 | a1 | B01 | b4 | C13 | c2 | D02 | dl | E02 |
| 2-2-d1E02-2-0115 | 682.311 | OEt | A02 | a1 | B02 | b1 | C14 | c1 | D01 | dl | E02 |
| 2-2-d1E02-2-0116 | 682.317 | OEt | A01 | a1 | B03 | b4 | C02 | c1 | D01 | dl | E02 |
| 2-2-d1E02-2-0117 | 684.271 | OEt | A02 | a1 | B03 | b3 | C09 | c1 | D01 | dl | E02 |
| 2-2-d1E02-2-0118 | 684.273 | OEt | A02 | a1 | B01 | b1 | C18 | c1 | D01 | dl | E02 |
| 2-2-d1E02-2-0119 | 684.298 | OEt | A01 | a1 | B02 | b4 | C05 | c1 | D01 | dl | E02 |
| 2-2-d1E02-2-0120 | 684.313 | OEt | A01 | a1 | B05 | b4 | C12 | c1 | D01 | dl | E02 |
| 2-2-d1E02-2-0121 | 685.203 | OEt | A01 | a1 | B04 | b1 | C05 | c1 | D01 | dl | E02 |
| 2-2-d1E02-2-0122 | 685.257 | OEt | A02 | a1 | B02 | b3 | C10 | c1 | D01 | dl | E02 |
| 2-2-d1E02-2-0123 | 686.216 | OEt | A01 | a1 | B04 | b3 | C08 | c1 | D01 | dl | E02 |
| 2-2-d1E02-2-0124 | 686.241 | OEt | A01 | a1 | B01 | b1 | C02 | c2 | D02 | dl | E02 |
| 2-2-d1E02-2-0125 | 686.241 | OEt | A01 | a1 | B01 | b1 | C03 | c2 | D02 | dl | E02 |
| 2-2-d1E02-2-0126 | 686.286 | OEt | A02 | a1 | B03 | b1 | C14 | c1 | D01 | dl | E02 |
| 2-2-d1E02-2-0127 | 686.293 | OEt | A01 | a1 | B05 | b4 | C13 | c1 | D01 | dl | E02 |
| 2-2-d1E02-2-0128 | 686.310 | OEt | A02 | a1 | B02 | b2 | C02 | c1 | D01 | dl | E02 |
| 2-2-d1E02-2-0129 | 686.310 | OEt | A02 | a1 | B02 | b2 | C03 | c1 | D01 | dl | E02 |
| 2-2-d1E02-2-0130 | 687.236 | OEt | A01 | a1 | B01 | b1 | C04 | c2 | D02 | dl | E02 |
| 2-2-d1E02-2-0131 | 687.306 | OEt | A02 | a1 | B02 | b2 | C04 | c1 | D01 | dl | E02 |
| 2-2-d1E02-2-0132 | 687.306 | OEt | A02 | a1 | B02 | b2 | C06 | c1 | D01 | dl | E02 |
| 2-2-d1E02-2-0133 | 687.306 | OEt | A02 | a1 | B02 | b2 | C07 | c1 | D01 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0134 | 688.273 | OEt | A01 | a1 | B03 | b4 | COS | c1 | D01 | dl | E02 |
| 2-2-d1E02-2-0135 | 689.232 | OEt | A02 | a1 | B03 | b3 | C10 | c1 | D01 | dl | E02 |
| 2-2-d1E02-2-0136 | 690.236 | OEt | A01 | a1 | B02 | b1 | C01 | c2 | D02 | dl | E02 |
| 2-2-d1E02-2-0137 | 690.267 | OEt | A01 | a1 | B05 | b1 | C02 | c1 | D01 | dl | E02 |
| 2-2-d1E02-2-0138 | 690.267 | OEt | A01 | a1 | B05 | b1 | C03 | c1 | D01 | dl | E02 |
| 2-2-d1E02-2-0139 | 690.285 | OEt | A02 | a1 | B03 | b2 | C02 | c1 | D01 | dl | E02 |
| 2-2-d1E02-2-0140 | 690.285 | OEt | A02 | a1 | B03 | b2 | C03 | c1 | D01 | dl | E02 |
| 2-2-d1E02-2-0141 | 690.342 | OEt | A02 | a1 | B01 | b4 | C02 | c1 | D01 | dl | E02 |
| 2-2-d1E02-2-0142 | 691.262 | OEt | A01 | a1 | B05 | b1 | C04 | c1 | D01 | dl | E02 |
| 2-2-d1E02-2-0143 | 691.281 | OEt | A02 | a1 | B03 | b2 | C04 | c1 | D01 | dl | E02 |
| 2-2-d1E02-2-0144 | 691.281 | OEt | A02 | a1 | B03 | b2 | C06 | c1 | D01 | dl | E02 |
| 2-2-d1E02-2-0145 | 691.281 | OEt | A02 | a1 | B03 | b2 | C07 | c1 | D01 | dl | E02 |
| 2-2-d1E02-2-0146 | 691.337 | OEt | A02 | a1 | B02 | b1 | C15 | c1 | D01 | dl | E02 |
| 2-2-d1E02-2-0147 | 692.197 | OEt | A01 | a1 | B01 | b1 | C05 | c2 | D02 | dl | E02 |
| 2-2-d1E02-2-0148 | 692.285 | OEt | A01 | a1 | B02 | b3 | C11 | c1 | D01 | dl | E02 |
| 2-2-d1E02-2-0149 | 692.332 | OEt | A02 | a1 | B02 | b1 | C16 | c1 | D01 | dl | E02 |
| 2-2-d1E02-2-0150 | 693.210 | OEt | A01 | a1 | B01 | b3 | C08 | c2 | D02 | dl | E02 |
| 2-2-d1E02-2-0151 | 693.327 | OEt | A02 | a1 | B02 | b1 | C17 | c1 | D01 | dl | E02 |
| 2-2-d1E02-2-0152 | 694.211 | OEt | A01 | a1 | B03 | b1 | C01 | c2 | D02 | dl | E02 |
| 2-2-d1E02-2-0153 | 694.304 | OEt | A01 | a1 | B02 | b4 | C12 | c2 | D02 | dl | E02 |
| 2-2-d1E02-2-0154 | 694.337 | OEt | A02 | a1 | B02 | b4 | C01 | c1 | D01 | dl | E02 |
| 2-2-d1E02-2-0155 | 695.241 | OEt | A02 | a1 | B04 | b1 | C01 | c1 | D01 | dl | E02 |
| 2-2-d1E02-2-0156 | 695.312 | OEt | A02 | a1 | B03 | b1 | C15 | c1 | D01 | dl | E02 |
| 2-2-d1E02-2-0157 | 696.223 | OEt | A01 | a1 | B05 | b1 | C05 | c1 | D01 | dl | E02 |
| 2-2-d1E02-2-0158 | 696.260 | OEt | A01 | a1 | B03 | b3 | C11 | c1 | D01 | dl | E02 |
| 2-2-d1E02-2-0159 | 696.283 | OEt | A01 | a1 | B02 | b4 | C13 | c2 | D02 | dl | E02 |
| 2-2-d1E02-2-0160 | 696.298 | OEt | A02 | a1 | B01 | b4 | C05 | c1 | D01 | dl | E02 |
| 2-2-d1E02-2-0161 | 696.307 | OEt | A02 | a1 | B03 | b1 | C16 | c1 | D01 | dl | E02 |
| 2-2-d1E02-2-0162 | 697.232 | OEt | A01 | a1 | B04 | b3 | C09 | c1 | D01 | dl | E02 |
| 2-2-d1E02-2-0163 | 697.236 | OEt | A01 | a1 | B05 | b3 | C08 | c1 | D01 | dl | E02 |
| 2-2-d1E02-2-0164 | 697.302 | OEt | A02 | a1 | B03 | b1 | C17 | c1 | D01 | dl | E02 |
| 2-2-d1E02-2-0165 | 698.279 | OEt | A01 | a1 | B03 | b4 | C12 | c2 | D02 | dl | E02 |
| 2-2-d1E02-2-0166 | 698.289 | OEt | A02 | a1 | B02 | b1 | C18 | c1 | D01 | dl | E02 |
| 2-2-d1E02-2-0167 | 698.312 | OEt | A02 | a1 | B03 | b4 | C01 | c1 | D01 | dl | E02 |
| 2-2-d1E02-2-0168 | 699.248 | OEt | A01 | a1 | B04 | b1 | C14 | c1 | D01 | dl | E02 |
| 2-2-d1E02-2-0169 | 699.309 | OEt | A02 | a1 | B04 | b4 | C12 | c1 | D01 | dl | E02 |
| 2-2-d1E02-2-0170 | 700.257 | OEt | A01 | a1 | B02 | b1 | C02 | c2 | D02 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0171 | 700.257 | OEt | A01 | a1 | B02 | b1 | C03 | c2 | D02 | dl | E02 |
| 2-2-d1E02-2-0172 | 700.258 | OEt | A01 | a1 | B03 | b4 | C13 | c2 | D02 | dl | E02 |
| 2-2-d1E02-2-0173 | 701.252 | OEt | A01 | a1 | B02 | b1 | C04 | c2 | D02 | dl | E02 |
| 2-2-d1E02-2-0174 | 701.288 | OEt | A02 | a1 | B04 | b4 | C13 | c1 | D01 | dl | E02 |
| 2-2-d1E02-2-0175 | 702.193 | OEt | A01 | a1 | B04 | b3 | C10 | c1 | D01 | dl | E02 |
| 2-2-d1E02-2-0176 | 702.236 | OEt | A02 | a1 | B01 | b1 | C01 | c2 | D02 | dl | E02 |
| 2-2-d1E02-2-0177 | 702.264 | OEt | A02 | a1 | B03 | b1 | C18 | c1 | D01 | dl | E02 |
| 2-2-d1E02-2-0178 | 703.246 | OEt | A01 | a1 | B04 | b2 | C02 | c1 | D01 | dl | E02 |
| 2-2-d1E02-2-0179 | 703.246 | OEt | A01 | a1 | B04 | b2 | C03 | c1 | D01 | dl | E02 |
| 2-2-d1E02-2-0180 | 704.226 | OEt | A01 | a1 | B01 | b3 | C09 | c2 | D02 | dl | E02 |
| 2-2-d1E02-2-0181 | 704.232 | OEt | A01 | a1 | B03 | b1 | C02 | c2 | D02 | dl | E02 |
| 2-2-d1E02-2-0182 | 704.232 | OEt | A01 | a1 | B03 | b1 | C03 | c2 | D02 | dl | E02 |
| 2-2-d1E02-2-0183 | 704.242 | OEt | A01 | a1 | B04 | b2 | C04 | c1 | D01 | dl | E02 |
| 2-2-d1E02-2-0184 | 704.242 | OEt | A01 | a1 | B04 | b2 | C06 | c1 | D01 | dl | E02 |
| 2-2-d1E02-2-0185 | 704.242 | OEt | A01 | a1 | B04 | b2 | C07 | c1 | D01 | dl | E02 |
| 2-2-d1E02-2-0186 | 704.285 | OEt | A02 | a1 | B01 | b3 | C11 | c1 | D01 | dl | E02 |
| 2-2-d1E02-2-0187 | 704.357 | OEt | A02 | a1 | B02 | b4 | C02 | c1 | D01 | dl | E02 |
| 2-2-d1E02-2-0188 | 705.227 | OEt | A01 | a1 | B03 | b1 | C04 | c2 | D02 | dl | E02 |
| 2-2-d1E02-2-0189 | 705.262 | OEt | A02 | a1 | B04 | b1 | C02 | c1 | D01 | dl | E02 |
| 2-2-d1E02-2-0190 | 705.262 | OEt | A02 | a1 | B04 | b1 | C03 | c1 | D01 | dl | E02 |
| 2-2-d1E02-2-0191 | 706.213 | OEt | A01 | a1 | B02 | b1 | C05 | c2 | D02 | dl | E02 |
| 2-2-d1E02-2-0192 | 706.242 | OEt | A01 | a1 | B01 | b1 | C14 | c2 | D02 | dl | E02 |
| 2-2-d1E02-2-0193 | 706.257 | OEt | A02 | a1 | B04 | b1 | C04 | c1 | D01 | dl | E02 |
| 2-2-d1E02-2-0194 | 706.261 | OEt | A02 | a1 | B05 | b1 | C01 | c1 | D01 | dl | E02 |
| 2-2-d1E02-2-0195 | 706.304 | OEt | A02 | a1 | B01 | b4 | C12 | c2 | D02 | dl | E02 |
| 2-2-d1E02-2-0196 | 706.348 | OEt | A01 | a1 | B01 | b1 | C19 | c1 | D01 | dl | E02 |
| 2-2-d1E02-2-0197 | 707.226 | OEt | A01 | a1 | B02 | b3 | C08 | c2 | D02 | dl | E02 |
| 2-2-d1E02-2-0198 | 708.252 | OEt | A01 | a1 | B05 | b3 | C09 | c1 | D01 | dl | E02 |
| 2-2-d1E02-2-0199 | 708.273 | OEt | A01 | a1 | B04 | b1 | C15 | c1 | D01 | dl | E02 |
| 2-2-d1E02-2-0200 | 708.283 | OEt | A02 | a1 | B01 | b4 | C13 | c2 | D02 | dl | E02 |
| 2-2-d1E02-2-0201 | 708.332 | OEt | A02 | a1 | B03 | b4 | C02 | c1 | D01 | dl | E02 |
| 2-2-d1E02-2-0202 | 709.188 | OEt | A01 | a1 | B01 | b3 | C10 | c2 | D02 | dl | E02 |
| 2-2-d1E02-2-0203 | 709.268 | OEt | A01 | a1 | B04 | b1 | C16 | c1 | D01 | dl | E02 |
| 2-2-d1E02-2-0204 | 710.188 | OEt | A01 | a1 | B03 | b1 | C05 | c2 | D02 | dl | E02 |
| 2-2-d1E02-2-0205 | 710.241 | OEt | A01 | a1 | B01 | b2 | C02 | c2 | D02 | dl | E02 |
| 2-2-d1E02-2-0206 | 710.241 | OEt | A01 | a1 | B01 | b2 | C03 | c2 | D02 | dl | E02 |
| 2-2-d1E02-2-0207 | 710.264 | OEt | A01 | a1 | B04 | b1 | C17 | c1 | D01 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0208 | 710.268 | OEt | A01 | a1 | B05 | b1 | C14 | c1 | D01 | dl | E02 |
| 2-2-d1E02-2-0209 | 710.314 | OEt | A02 | a1 | B02 | b4 | C05 | c1 | D01 | dl | E02 |
| 2-2-d1E02-2-0210 | 710.329 | OEt | A02 | a1 | B05 | b4 | C12 | c1 | D01 | dl | E02 |
| 2-2-d1E02-2-0211 | 711.201 | OEt | A01 | a1 | B03 | b3 | C08 | c2 | D02 | dl | E02 |
| 2-2-d1E02-2-0212 | 711.219 | OEt | A02 | a1 | B04 | b1 | C05 | c1 | D01 | dl | E02 |
| 2-2-d1E02-2-0213 | 711.236 | OEt | A01 | a1 | B01 | b2 | C04 | c2 | D02 | dl | E02 |
| 2-2-d1E02-2-0214 | 711.236 | OEt | A01 | a1 | B01 | b2 | C06 | c2 | D02 | dl | E02 |
| 2-2-d1E02-2-0215 | 711.236 | OEt | A01 | a1 | B01 | b2 | C07 | c2 | D02 | dl | E02 |
| 2-2-d1E02-2-0216 | 711.273 | OEt | A01 | a1 | B04 | b4 | C01 | c1 | D01 | dl | E02 |
| 2-2-d1E02-2-0217 | 712.232 | OEt | A02 | a1 | B04 | b3 | C08 | c1 | D01 | dl | E02 |
| 2-2-d1E02-2-0218 | 712.257 | OEt | A02 | a1 | B01 | b1 | C02 | c2 | D02 | dl | E02 |
| 2-2-d1E02-2-0219 | 712.257 | OEt | A02 | a1 | B01 | b1 | C03 | c2 | D02 | dl | E02 |
| 2-2-d1E02-2-0220 | 712.308 | OEt | A02 | a1 | B05 | b4 | C13 | c1 | D01 | dl | E02 |
| 2-2-d1E02-2-0221 | 713.213 | OEt | A01 | a1 | B05 | b3 | C10 | c1 | D01 | dl | E02 |
| 2-2-d1E02-2-0222 | 713.252 | OEt | A02 | a1 | B01 | b1 | C04 | c2 | D02 | dl | E02 |
| 2-2-d1E02-2-0223 | 714.267 | OEt | A01 | a1 | B05 | b2 | C02 | c1 | D01 | dl | E02 |
| 2-2-d1E02-2-0224 | 714.267 | OEt | A01 | a1 | B05 | b2 | C03 | c1 | D01 | dl | E02 |
| 2-2-d1E02-2-0225 | 714.289 | OEt | A02 | a1 | B03 | b4 | C05 | c1 | D01 | dl | E02 |
| 2-2-d1E02-2-0226 | 715.225 | OEt | A01 | a1 | B04 | b1 | C18 | c1 | D01 | dl | E02 |
| 2-2-d1E02-2-0227 | 715.262 | OEt | A01 | a1 | B05 | b2 | C04 | c1 | D01 | dl | E02 |
| 2-2-d1E02-2-0228 | 715.262 | OEt | A01 | a1 | B05 | b2 | C06 | c1 | D01 | dl | E02 |
| 2-2-d1E02-2-0229 | 715.262 | OEt | A01 | a1 | B05 | b2 | C07 | c1 | D01 | dl | E02 |
| 2-2-d1E02-2-0230 | 715.268 | OEt | A01 | a1 | B01 | b1 | C15 | c2 | D02 | dl | E02 |
| 2-2-d1E02-2-0231 | 716.252 | OEt | A02 | a1 | B02 | b1 | C01 | c2 | D02 | dl | E02 |
| 2-2-d1E02-2-0232 | 716.263 | OEt | A01 | a1 | B01 | b1 | C16 | c2 | D02 | dl | E02 |
| 2-2-d1E02-2-0233 | 716.282 | OEt | A02 | a1 | B05 | b1 | C02 | c1 | D01 | dl | E02 |
| 2-2-d1E02-2-0234 | 716.282 | OEt | A02 | a1 | B05 | b1 | C03 | c1 | D01 | dl | E02 |
| 2-2-d1E02-2-0235 | 717.258 | OEt | A01 | a1 | B01 | b1 | C17 | c2 | D02 | dl | E02 |
| 2-2-d1E02-2-0236 | 717.277 | OEt | A02 | a1 | B05 | b1 | C04 | c1 | D01 | dl | E02 |
| 2-2-d1E02-2-0237 | 718.213 | OEt | A02 | a1 | B01 | b1 | C05 | c2 | D02 | dl | E02 |
| 2-2-d1E02-2-0238 | 718.242 | OEt | A01 | a1 | B02 | b3 | C09 | c2 | D02 | dl | E02 |
| 2-2-d1E02-2-0239 | 718.267 | OEt | A01 | a1 | B01 | b4 | C01 | c2 | D02 | dl | E02 |
| 2-2-d1E02-2-0240 | 718.300 | OEt | A02 | a1 | B02 | b3 | C11 | c1 | D01 | dl | E02 |
| 2-2-d1E02-2-0241 | 719.226 | OEt | A02 | a1 | B01 | b3 | C08 | c2 | D02 | dl | E02 |
| 2-2-d1E02-2-0242 | 719.293 | OEt | A01 | a1 | B05 | b1 | C15 | c1 | D01 | dl | E02 |
| 2-2-d1E02-2-0243 | 720.227 | OEt | A02 | a1 | B03 | b1 | C01 | c2 | D02 | dl | E02 |
| 2-2-d1E02-2-0244 | 720.258 | OEt | A01 | a1 | B02 | b1 | C14 | c2 | D02 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0245 | 720.288 | OEt | A01 | a1 | B05 | b1 | C16 | c1 | D01 | dl | E02 |
| 2-2-d1E02-2-0246 | 720.319 | OEt | A02 | a1 | B02 | b4 | C12 | c2 | D02 | dl | E02 |
| 2-2-d1E02-2-0247 | 720.364 | OEt | A01 | a1 | B02 | b1 | C19 | c1 | D01 | dl | E02 |
| 2-2-d1E02-2-0248 | 721.284 | OEt | A01 | a1 | B05 | b1 | C17 | c1 | D01 | dl | E02 |
| 2-2-d1E02-2-0249 | 721.293 | OEt | A01 | a1 | B04 | b4 | C02 | c1 | D01 | dl | E02 |
| 2-2-d1E02-2-0250 | 722.217 | OEt | A01 | a1 | B03 | b3 | C09 | c2 | D02 | dl | E02 |
| 2-2-d1E02-2-0251 | 722.219 | OEt | A01 | a1 | B01 | b1 | C18 | c2 | D02 | dl | E02 |
| 2-2-d1E02-2-0252 | 722.239 | OEt | A02 | a1 | B05 | b1 | C05 | c1 | D01 | dl | E02 |
| 2-2-d1E02-2-0253 | 722.275 | OEt | A02 | a1 | B03 | b3 | C11 | c1 | D01 | dl | E02 |
| 2-2-d1E02-2-0254 | 722.293 | OEt | A01 | a1 | B05 | b4 | C01 | c1 | D01 | dl | E02 |
| 2-2-d1E02-2-0255 | 722.299 | OEt | A02 | a1 | B02 | b4 | C13 | c2 | D02 | dl | E02 |
| 2-2-d1E02-2-0256 | 723.203 | OEt | A01 | a1 | B02 | b3 | C10 | c2 | D02 | dl | E02 |
| 2-2-d1E02-2-0257 | 723.248 | OEt | A02 | a1 | B04 | b3 | C09 | c1 | D01 | dl | E02 |
| 2-2-d1E02-2-0258 | 723.252 | OEt | A02 | a1 | B05 | b3 | C08 | c1 | D01 | dl | E02 |
| 2-2-d1E02-2-0259 | 724.233 | OEt | A01 | a1 | B03 | b1 | C14 | c2 | D02 | dl | E02 |
| 2-2-d1E02-2-0260 | 724.257 | OEt | A01 | a1 | B02 | b2 | C02 | c2 | D02 | dl | E02 |
| 2-2-d1E02-2-0261 | 724.257 | OEt | A01 | a1 | B02 | b2 | C03 | c2 | D02 | dl | E02 |
| 2-2-d1E02-2-0262 | 724.294 | OEt | A02 | a1 | B03 | b4 | C12 | c2 | D02 | dl | E02 |
| 2-2-d1E02-2-0263 | 724.338 | OEt | A01 | a1 | B03 | b1 | C19 | c1 | D01 | dl | E02 |
| 2-2-d1E02-2-0264 | 725.252 | OEt | A01 | a1 | B02 | b2 | C04 | c2 | D02 | dl | E02 |
| 2-2-d1E02-2-0265 | 725.252 | OEt | A01 | a1 | B02 | b2 | C06 | c2 | D02 | dl | E02 |
| 2-2-d1E02-2-0266 | 725.252 | OEt | A01 | a1 | B02 | b2 | C07 | c2 | D02 | dl | E02 |
| 2-2-d1E02-2-0267 | 725.263 | OEt | A02 | a1 | B04 | b1 | C14 | c1 | D01 | dl | E02 |
| 2-2-d1E02-2-0268 | 726.245 | OEt | A01 | a1 | B05 | b1 | C18 | c1 | D01 | dl | E02 |
| 2-2-d1E02-2-0269 | 726.272 | OEt | A02 | a1 | B02 | b1 | C02 | c2 | D02 | dl | E02 |
| 2-2-d1E02-2-0270 | 726.272 | OEt | A02 | a1 | B02 | b1 | C03 | c2 | D02 | dl | E02 |
| 2-2-d1E02-2-0271 | 726.273 | OEt | A02 | a1 | B03 | b4 | C13 | c2 | D02 | dl | E02 |
| 2-2-d1E02-2-0272 | 727.178 | OEt | A01 | a1 | B03 | b3 | C10 | c2 | D02 | dl | E02 |
| 2-2-d1E02-2-0273 | 727.250 | OEt | A01 | a1 | B04 | b4 | C05 | c1 | D01 | dl | E02 |
| 2-2-d1E02-2-0274 | 727.268 | OEt | A02 | a1 | B02 | b1 | C04 | c2 | D02 | dl | E02 |
| 2-2-d1E02-2-0275 | 728.209 | OEt | A02 | a1 | B04 | b3 | C10 | c1 | D01 | dl | E02 |
| 2-2-d1E02-2-0276 | 728.232 | OEt | A01 | a1 | B03 | b2 | C02 | c2 | D02 | dl | E02 |
| 2-2-d1E02-2-0277 | 728.232 | OEt | A01 | a1 | B03 | b2 | C03 | c2 | D02 | dl | E02 |
| 2-2-d1E02-2-0278 | 728.288 | OEt | A01 | a1 | B01 | b4 | C02 | c2 | D02 | dl | E02 |
| 2-2-d1E02-2-0279 | 729.227 | OEt | A01 | a1 | B03 | b2 | C04 | c2 | D02 | dl | E02 |
| 2-2-d1E02-2-0280 | 729.227 | OEt | A01 | a1 | B03 | b2 | C06 | c2 | D02 | dl | E02 |
| 2-2-d1E02-2-0281 | 729.227 | OEt | A01 | a1 | B03 | b2 | C07 | c2 | D02 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0282 | 729.262 | OEt | A02 | a1 | B04 | b2 | C02 | c1 | D01 | dl | E02 |
| 2-2-d1E02-2-0283 | 729.262 | OEt | A02 | a1 | B04 | b2 | C03 | c1 | D01 | dl | E02 |
| 2-2-d1E02-2-0284 | 729.283 | OEt | A01 | a1 | B02 | b1 | C15 | c2 | D02 | dl | E02 |
| 2-2-d1E02-2-0285 | 730.242 | OEt | A02 | a1 | B01 | b3 | C09 | c2 | D02 | dl | E02 |
| 2-2-d1E02-2-0286 | 730.247 | OEt | A02 | a1 | B03 | b1 | C02 | c2 | D02 | dl | E02 |
| 2-2-d1E02-2-0287 | 730.247 | OEt | A02 | a1 | B03 | b1 | C03 | c2 | D02 | dl | E02 |
| 2-2-d1E02-2-0288 | 730.257 | OEt | A02 | a1 | B04 | b2 | C04 | c1 | D01 | dl | E02 |
| 2-2-d1E02-2-0289 | 730.257 | OEt | A02 | a1 | B04 | b2 | C06 | c1 | D01 | dl | E02 |
| 2-2-d1E02-2-0290 | 730.257 | OEt | A02 | a1 | B04 | b2 | C07 | c1 | D01 | dl | E02 |
| 2-2-d1E02-2-0291 | 730.278 | OEt | A01 | a1 | B02 | b1 | C16 | c2 | D02 | dl | E02 |
| 2-2-d1E02-2-0292 | 731.243 | OEt | A02 | a1 | B03 | b1 | C04 | c2 | D02 | dl | E02 |
| 2-2-d1E02-2-0293 | 731.274 | OEt | A01 | a1 | B02 | b1 | C17 | c2 | D02 | dl | E02 |
| 2-2-d1E02-2-0294 | 732.229 | OEt | A02 | a1 | B02 | b1 | C05 | c2 | D02 | dl | E02 |
| 2-2-d1E02-2-0295 | 732.258 | OEt | A02 | a1 | B01 | b1 | C14 | c2 | D02 | dl | E02 |
| 2-2-d1E02-2-0296 | 732.283 | OEt | A01 | a1 | B02 | b4 | C01 | c2 | D02 | dl | E02 |
| 2-2-d1E02-2-0297 | 732.313 | OEt | A01 | a1 | B05 | b4 | C02 | c1 | D01 | dl | E02 |
| 2-2-d1E02-2-0298 | 732.364 | OEt | A02 | a1 | B01 | b1 | C19 | c1 | D01 | dl | E02 |
| 2-2-d1E02-2-0299 | 733.188 | OEt | A01 | a1 | B04 | b1 | C01 | c2 | D02 | dl | E02 |
| 2-2-d1E02-2-0300 | 733.242 | OEt | A02 | a1 | B02 | b3 | C08 | c2 | D02 | dl | E02 |
| 2-2-d1E02-2-0301 | 733.258 | OEt | A01 | a1 | B03 | b1 | C15 | c2 | D02 | dl | E02 |
| 2-2-d1E02-2-0302 | 734.244 | OEt | A01 | a1 | B01 | b4 | C05 | c2 | D02 | dl | E02 |
| 2-2-d1E02-2-0303 | 734.253 | OEt | A01 | a1 | B03 | b1 | C16 | c2 | D02 | dl | E02 |
| 2-2-d1E02-2-0304 | 734.268 | OEt | A02 | a1 | B05 | b3 | C09 | c1 | D01 | dl | E02 |
| 2-2-d1E02-2-0305 | 734.289 | OEt | A02 | a1 | B04 | b1 | C15 | c1 | D01 | dl | E02 |
| 2-2-d1E02-2-0306 | 735.203 | OEt | A02 | a1 | B01 | b3 | C10 | c2 | D02 | dl | E02 |
| 2-2-d1E02-2-0307 | 735.236 | OEt | A01 | a1 | B04 | b3 | C11 | c1 | D01 | dl | E02 |
| 2-2-d1E02-2-0308 | 735.249 | OEt | A01 | a1 | B03 | b1 | C17 | c2 | D02 | dl | E02 |
| 2-2-d1E02-2-0309 | 735.284 | OEt | A02 | a1 | B04 | b1 | C16 | c1 | D01 | dl | E02 |
| 2-2-d1E02-2-0310 | 736.204 | OEt | A02 | a1 | B03 | b1 | C05 | c2 | D02 | dl | E02 |
| 2-2-d1E02-2-0311 | 736.235 | OEt | A01 | a1 | B02 | b1 | C18 | c2 | D02 | dl | E02 |
| 2-2-d1E02-2-0312 | 736.257 | OEt | A02 | a1 | B01 | b2 | C02 | c2 | D02 | dl | E02 |
| 2-2-d1E02-2-0313 | 736.257 | OEt | A02 | a1 | B01 | b2 | C03 | c2 | D02 | dl | E02 |
| 2-2-d1E02-2-0314 | 736.258 | OEt | A01 | a1 | B03 | b4 | C01 | c2 | D02 | dl | E02 |
| 2-2-d1E02-2-0315 | 736.279 | OEt | A02 | a1 | B04 | b1 | C17 | c1 | D01 | dl | E02 |
| 2-2-d1E02-2-0316 | 736.283 | OEt | A02 | a1 | B05 | b1 | C14 | c1 | D01 | dl | E02 |
| 2-2-d1E02-2-0317 | 737.217 | OEt | A02 | a1 | B03 | b3 | C08 | c2 | D02 | dl | E02 |
| 2-2-d1E02-2-0318 | 737.252 | OEt | A02 | a1 | B01 | b2 | C04 | c2 | D02 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0319 | 737.252 | OEt | A02 | a1 | B01 | b2 | C06 | c2 | D02 | dl | E02 |
| 2-2-d1E02-2-0320 | 737.252 | OEt | A02 | a1 | B01 | b2 | C07 | c2 | D02 | dl | E02 |
| 2-2-d1E02-2-0321 | 737.255 | OEt | A01 | a1 | B04 | b4 | C12 | c2 | D02 | dl | E02 |
| 2-2-d1E02-2-0322 | 737.288 | OEt | A02 | a1 | B04 | b4 | C01 | c1 | D01 | dl | E02 |
| 2-2-d1E02-2-0323 | 738.270 | OEt | A01 | a1 | B05 | b4 | C05 | c1 | D01 | dl | E02 |
| 2-2-d1E02-2-0324 | 739.229 | OEt | A02 | a1 | B05 | b3 | C10 | c1 | D01 | dl | E02 |
| 2-2-d1E02-2-0325 | 739.235 | OEt | A01 | a1 | B04 | b4 | C13 | c2 | D02 | dl | E02 |
| 2-2-d1E02-2-0326 | 740.210 | OEt | A01 | a1 | B03 | b1 | C18 | c2 | D02 | dl | E02 |
| 2-2-d1E02-2-0327 | 740.282 | OEt | A02 | a1 | B05 | b2 | C02 | c1 | D01 | dl | E02 |
| 2-2-d1E02-2-0328 | 740.282 | OEt | A02 | a1 | B05 | b2 | C03 | c1 | D01 | dl | E02 |
| 2-2-d1E02-2-0329 | 741.240 | OEt | A02 | a1 | B04 | b1 | C18 | c1 | D01 | dl | E02 |
| 2-2-d1E02-2-0330 | 741.277 | OEt | A02 | a1 | B05 | b2 | C04 | c1 | D01 | dl | E02 |
| 2-2-d1E02-2-0331 | 741.277 | OEt | A02 | a1 | B05 | b2 | C06 | c1 | D01 | dl | E02 |
| 2-2-d1E02-2-0332 | 741.277 | OEt | A02 | a1 | B05 | b2 | C07 | c1 | D01 | dl | E02 |
| 2-2-d1E02-2-0333 | 741.283 | OEt | A02 | a1 | B01 | b1 | C15 | c2 | D02 | dl | E02 |
| 2-2-d1E02-2-0334 | 742.231 | OEt | A01 | a1 | B01 | b3 | C11 | c2 | D02 | dl | E02 |
| 2-2-d1E02-2-0335 | 742.278 | OEt | A02 | a1 | B01 | b1 | C16 | c2 | D02 | dl | E02 |
| 2-2-d1E02-2-0336 | 742.304 | OEt | A01 | a1 | B02 | b4 | C02 | c2 | D02 | dl | E02 |
| 2-2-d1E02-2-0337 | 743.208 | OEt | A01 | a1 | B04 | b1 | C02 | c2 | D02 | dl | E02 |
| 2-2-d1E02-2-0338 | 743.208 | OEt | A01 | a1 | B04 | b1 | C03 | c2 | D02 | dl | E02 |
| 2-2-d1E02-2-0339 | 743.274 | OEt | A02 | a1 | B01 | b1 | C17 | c2 | D02 | dl | E02 |
| 2-2-d1E02-2-0340 | 744.204 | OEt | A01 | a1 | B04 | b1 | C04 | c2 | D02 | dl | E02 |
| 2-2-d1E02-2-0341 | 744.208 | OEt | A01 | a1 | B05 | b1 | C01 | c2 | D02 | dl | E02 |
| 2-2-d1E02-2-0342 | 744.258 | OEt | A02 | a1 | B02 | b3 | C09 | c2 | D02 | dl | E02 |
| 2-2-d1E02-2-0343 | 744.283 | OEt | A02 | a1 | B01 | b4 | C01 | c2 | D02 | dl | E02 |
| 2-2-d1E02-2-0344 | 745.309 | OEt | A02 | a1 | B05 | b1 | C15 | c1 | D01 | dl | E02 |
| 2-2-d1E02-2-0345 | 746.256 | OEt | A01 | a1 | B05 | b3 | C11 | c1 | D01 | dl | E02 |
| 2-2-d1E02-2-0346 | 746.273 | OEt | A02 | a1 | B02 | b1 | C14 | c2 | D02 | dl | E02 |
| 2-2-d1E02-2-0347 | 746.279 | OEt | A01 | a1 | B03 | b4 | C02 | c2 | D02 | dl | E02 |
| 2-2-d1E02-2-0348 | 746.304 | OEt | A02 | a1 | B05 | b1 | C16 | c1 | D01 | dl | E02 |
| 2-2-d1E02-2-0349 | 746.379 | OEt | A02 | a1 | B02 | b1 | C19 | c1 | D01 | dl | E02 |
| 2-2-d1E02-2-0350 | 747.299 | OEt | A02 | a1 | B05 | b1 | C17 | c1 | D01 | dl | E02 |
| 2-2-d1E02-2-0351 | 747.309 | OEt | A02 | a1 | B04 | b4 | C02 | c1 | D01 | dl | E02 |
| 2-2-d1E02-2-0352 | 748.233 | OEt | A02 | a1 | B03 | b3 | C09 | c2 | D02 | dl | E02 |
| 2-2-d1E02-2-0353 | 748.235 | OEt | A02 | a1 | B01 | b1 | C18 | c2 | D02 | dl | E02 |
| 2-2-d1E02-2-0354 | 748.260 | OEt | A01 | a1 | B02 | b4 | C05 | c2 | D02 | dl | E02 |
| 2-2-d1E02-2-0355 | 748.275 | OEt | A01 | a1 | B05 | b4 | C12 | c2 | D02 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0356 | 748.308 | OEt | A02 | a1 | B05 | b4 | C01 | c1 | D01 | dl | E02 |
| 2-2-d1E02-2-0357 | 749.165 | OEt | A01 | a1 | B04 | b1 | C05 | c2 | D02 | dl | E02 |
| 2-2-d1E02-2-0358 | 749.219 | OEt | A02 | a1 | B02 | b3 | C10 | c2 | D02 | dl | E02 |
| 2-2-d1E02-2-0359 | 750.178 | OEt | A01 | a1 | B04 | b3 | C08 | c2 | D02 | dl | E02 |
| 2-2-d1E02-2-0360 | 750.248 | OEt | A02 | a1 | B03 | b1 | C14 | c2 | D02 | dl | E02 |
| 2-2-d1E02-2-0361 | 750.255 | OEt | A01 | a1 | B05 | b4 | C13 | c2 | D02 | dl | E02 |
| 2-2-d1E02-2-0362 | 750.272 | OEt | A02 | a1 | B02 | b2 | C02 | c2 | D02 | dl | E02 |
| 2-2-d1E02-2-0363 | 750.272 | OEt | A02 | a1 | B02 | b2 | C03 | c2 | D02 | dl | E02 |
| 2-2-d1E02-2-0364 | 750.354 | OEt | A02 | a1 | B03 | b1 | C19 | c1 | D01 | dl | E02 |
| 2-2-d1E02-2-0365 | 751.268 | OEt | A02 | a1 | B02 | b2 | C04 | c2 | D02 | dl | E02 |
| 2-2-d1E02-2-0366 | 751.268 | OEt | A02 | a1 | B02 | b2 | C06 | c2 | D02 | dl | E02 |
| 2-2-d1E02-2-0367 | 751.268 | OEt | A02 | a1 | B02 | b2 | C07 | c2 | D02 | dl | E02 |
| 2-2-d1E02-2-0368 | 752.235 | OEt | A01 | a1 | B03 | b4 | C05 | c2 | D02 | dl | E02 |
| 2-2-d1E02-2-0369 | 752.260 | OEt | A02 | a1 | B05 | b1 | C18 | c1 | D01 | dl | E02 |
| 2-2-d1E02-2-0370 | 753.194 | OEt | A02 | a1 | B03 | b3 | C10 | c2 | D02 | dl | E02 |
| 2-2-d1E02-2-0371 | 753.265 | OEt | A02 | a1 | B04 | b4 | C05 | c1 | D01 | dl | E02 |
| 2-2-d1E02-2-0372 | 754.228 | OEt | A01 | a1 | B05 | b1 | C02 | c2 | D02 | dl | E02 |
| 2-2-d1E02-2-0373 | 754.228 | OEt | A01 | a1 | B05 | b1 | C03 | c2 | D02 | dl | E02 |
| 2-2-d1E02-2-0374 | 754.247 | OEt | A02 | a1 | B03 | b2 | C02 | c2 | D02 | dl | E02 |
| 2-2-d1E02-2-0375 | 754.247 | OEt | A02 | a1 | B03 | b2 | C03 | c2 | D02 | dl | E02 |
| 2-2-d1E02-2-0376 | 754.304 | OEt | A02 | a1 | B01 | b4 | C02 | c2 | D02 | dl | E02 |
| 2-2-d1E02-2-0377 | 755.224 | OEt | A01 | a1 | B05 | b1 | C04 | c2 | D02 | dl | E02 |
| 2-2-d1E02-2-0378 | 755.243 | OEt | A02 | a1 | B03 | b2 | C04 | c2 | D02 | dl | E02 |
| 2-2-d1E02-2-0379 | 755.243 | OEt | A02 | a1 | B03 | b2 | C06 | c2 | D02 | dl | E02 |
| 2-2-d1E02-2-0380 | 755.243 | OEt | A02 | a1 | B03 | b2 | C07 | c2 | D02 | dl | E02 |
| 2-2-d1E02-2-0381 | 755.299 | OEt | A02 | a1 | B02 | b1 | C15 | c2 | D02 | dl | E02 |
| 2-2-d1E02-2-0382 | 756.247 | OEt | A01 | a1 | B02 | b3 | C11 | c2 | D02 | dl | E02 |
| 2-2-d1E02-2-0383 | 756.294 | OEt | A02 | a1 | B02 | b1 | C16 | c2 | D02 | dl | E02 |
| 2-2-d1E02-2-0384 | 757.289 | OEt | A02 | a1 | B02 | b1 | C17 | c2 | D02 | dl | E02 |
| 2-2-d1E02-2-0385 | 758.299 | OEt | A02 | a1 | B02 | b4 | C01 | c2 | D02 | dl | E02 |
| 2-2-d1E02-2-0386 | 758.329 | OEt | A02 | a1 | B05 | b4 | C02 | c1 | D01 | dl | E02 |
| 2-2-d1E02-2-0387 | 759.203 | OEt | A02 | a1 | B04 | b1 | C01 | c2 | D02 | dl | E02 |
| 2-2-d1E02-2-0388 | 759.274 | OEt | A02 | a1 | B03 | b1 | C15 | c2 | D02 | dl | E02 |
| 2-2-d1E02-2-0389 | 760.185 | OEt | A01 | a1 | B05 | b1 | C05 | c2 | D02 | dl | E02 |
| 2-2-d1E02-2-0390 | 760.221 | OEt | A01 | a1 | B03 | b3 | C11 | c2 | D02 | dl | E02 |
| 2-2-d1E02-2-0391 | 760.260 | OEt | A02 | a1 | B01 | b4 | C05 | c2 | D02 | dl | E02 |
| 2-2-d1E02-2-0392 | 760.269 | OEt | A02 | a1 | B03 | b1 | C16 | c2 | D02 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0393 | 761.194 | OEt | A01 | a1 | B04 | b3 | C09 | c2 | D02 | dl | E02 |
| 2-2-d1E02-2-0394 | 761.198 | OEt | A01 | a1 | B05 | b3 | C08 | c2 | D02 | dl | E02 |
| 2-2-d1E02-2-0395 | 761.252 | OEt | A02 | a1 | B04 | b3 | C11 | c1 | D01 | dl | E02 |
| 2-2-d1E02-2-0396 | 761.264 | OEt | A02 | a1 | B03 | b1 | C17 | c2 | D02 | dl | E02 |
| 2-2-d1E02-2-0397 | 762.251 | OEt | A02 | a1 | B02 | b1 | C18 | c2 | D02 | dl | E02 |
| 2-2-d1E02-2-0398 | 762.273 | OEt | A02 | a1 | B03 | b4 | C01 | c2 | D02 | dl | E02 |
| 2-2-d1E02-2-0399 | 763.209 | OEt | A01 | a1 | B04 | b1 | C14 | c2 | D02 | dl | E02 |
| 2-2-d1E02-2-0400 | 763.271 | OEt | A02 | a1 | B04 | b4 | C12 | c2 | D02 | dl | E02 |
| 2-2-d1E02-2-0401 | 763.315 | OEt | A01 | a1 | B04 | b1 | C19 | c1 | D01 | dl | E02 |
| 2-2-d1E02-2-0402 | 764.286 | OEt | A02 | a1 | B05 | b4 | C05 | c1 | D01 | dl | E02 |
| 2-2-d1E02-2-0403 | 765.250 | OEt | A02 | a1 | B04 | b4 | C13 | c2 | D02 | d1 | E02 |
| 2-2-d1E02-2-0404 | 766.155 | OEt | A01 | a1 | B04 | b3 | C10 | c2 | D02 | dl | E02 |
| 2-2-d1E02-2-0405 | 766.225 | OEt | A02 | a1 | B03 | b1 | C18 | c2 | D02 | dl | E02 |
| 2-2-d1E02-2-0406 | 767.208 | OEt | A01 | a1 | B04 | b2 | C02 | c2 | D02 | dl | E02 |
| 2-2-d1E02-2-0407 | 767.208 | OEt | A01 | a1 | B04 | b2 | C03 | c2 | D02 | dl | E02 |
| 2-2-d1E02-2-0408 | 768.204 | OEt | A01 | a1 | B04 | b2 | C04 | c2 | D02 | dl | E02 |
| 2-2-d1E02-2-0409 | 768.204 | OEt | A01 | a1 | B04 | b2 | C06 | c2 | D02 | dl | E02 |
| 2-2-d1E02-2-0410 | 768.204 | OEt | A01 | a1 | B04 | b2 | C07 | c2 | D02 | dl | E02 |
| 2-2-d1E02-2-0411 | 768.247 | OEt | A02 | a1 | B01 | b3 | C11 | c2 | D02 | dl | E02 |
| 2-2-d1E02-2-0412 | 768.319 | OEt | A02 | a1 | B02 | b4 | C02 | c2 | D02 | dl | E02 |
| 2-2-d1E02-2-0413 | 769.224 | OEt | A02 | a1 | B04 | b1 | C02 | c2 | D02 | dl | E02 |
| 2-2-d1E02-2-0414 | 769.224 | OEt | A02 | a1 | B04 | b1 | C03 | c2 | D02 | dl | E02 |
| 2-2-d1E02-2-0415 | 770.219 | OEt | A02 | a1 | B04 | b1 | C04 | c2 | D02 | dl | E02 |
| 2-2-d1E02-2-0416 | 770.223 | OEt | A02 | a1 | B05 | b1 | C01 | c2 | D02 | dl | E02 |
| 2-2-d1E02-2-0417 | 770.310 | OEt | A01 | a1 | B01 | b1 | C19 | c2 | D02 | dl | E02 |
| 2-2-d1E02-2-0418 | 772.214 | OEt | A01 | a1 | B05 | b3 | C09 | c2 | D02 | dl | E02 |
| 2-2-d1E02-2-0419 | 772.235 | OEt | A01 | a1 | B04 | b1 | C15 | c2 | D02 | dl | E02 |
| 2-2-d1E02-2-0420 | 772.272 | OEt | A02 | a1 | B05 | b3 | C11 | c1 | D01 | dl | E02 |
| 2-2-d1E02-2-0421 | 772.294 | OEt | A02 | a1 | B03 | b4 | C02 | c2 | D02 | dl | E02 |
| 2-2-d1E02-2-0422 | 773.230 | OEt | A01 | a1 | B04 | b1 | C16 | c2 | D02 | dl | E02 |
| 2-2-d1E02-2-0423 | 774.225 | OEt | A01 | a1 | B04 | b1 | C17 | c2 | D02 | dl | E02 |
| 2-2-d1E02-2-0424 | 774.229 | OEt | A01 | a1 | B05 | b1 | C14 | c2 | D02 | dl | E02 |
| 2-2-d1E02-2-0425 | 774.276 | OEt | A02 | a1 | B02 | b4 | C05 | c2 | D02 | dl | E02 |
| 2-2-d1E02-2-0426 | 774.291 | OEt | A02 | a1 | B05 | b4 | C12 | c2 | D02 | dl | E02 |
| 2-2-d1E02-2-0427 | 774.335 | OEt | A01 | a1 | B05 | b1 | C19 | c1 | D01 | dl | E02 |
| 2-2-d1E02-2-0428 | 775.180 | OEt | A02 | a1 | B04 | b1 | C05 | c2 | D02 | dl | E02 |
| 2-2-d1E02-2-0429 | 775.235 | OEt | A01 | a1 | B04 | b4 | C01 | c2 | D02 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0430 | 776.193 | OEt | A02 | a1 | B04 | b3 | C08 | c2 | D02 | dl | E02 |
| 2-2-d1E02-2-0431 | 776.270 | OEt | A02 | a1 | B05 | b4 | C13 | c2 | D02 | dl | E02 |
| 2-2-d1E02-2-0432 | 777.175 | OEt | A01 | a1 | B05 | b3 | C10 | c2 | D02 | dl | E02 |
| 2-2-d1E02-2-0433 | 778.228 | OEt | A01 | a1 | B05 | b2 | C02 | c2 | D02 | dl | E02 |
| 2-2-d1E02-2-0434 | 778.228 | OEt | A01 | a1 | B05 | b2 | C03 | c2 | D02 | dl | E02 |
| 2-2-d1E02-2-0435 | 778.251 | OEt | A02 | a1 | B03 | b4 | C05 | c2 | D02 | dl | E02 |
| 2-2-d1E02-2-0436 | 779.187 | OEt | A01 | a1 | B04 | b1 | C18 | c2 | D02 | dl | E02 |
| 2-2-d1E02-2-0437 | 779.224 | OEt | A01 | a1 | B05 | b2 | C04 | c2 | D02 | dl | E02 |
| 2-2-d1E02-2-0438 | 779.224 | OEt | A01 | a1 | B05 | b2 | C06 | c2 | D02 | dl | E02 |
| 2-2-d1E02-2-0439 | 779.224 | OEt | A01 | a1 | B05 | b2 | C07 | c2 | D02 | dl | E02 |
| 2-2-d1E02-2-0440 | 780.244 | OEt | A02 | a1 | B05 | b1 | C02 | c2 | D02 | dl | E02 |
| 2-2-d1E02-2-0441 | 780.244 | OEt | A02 | a1 | B05 | b1 | C03 | c2 | D02 | dl | E02 |
| 2-2-d1E02-2-0442 | 781.239 | OEt | A02 | a1 | B05 | b1 | C04 | c2 | D02 | dl | E02 |
| 2-2-d1E02-2-0443 | 782.262 | OEt | A02 | a1 | B02 | b3 | C11 | c2 | D02 | dl | E02 |
| 2-2-d1E02-2-0444 | 783.255 | OEt | A01 | a1 | B05 | b1 | C15 | c2 | D02 | dl | E02 |
| 2-2-d1E02-2-0445 | 784.250 | OEt | A01 | a1 | B05 | b1 | C16 | c2 | D02 | dl | E02 |
| 2-2-d1E02-2-0446 | 784.325 | OEt | A01 | a1 | B02 | b1 | C19 | c2 | D02 | dl | E02 |
| 2-2-d1E02-2-0447 | 785.245 | OEt | A01 | a1 | B05 | b1 | C17 | c2 | D02 | dl | E02 |
| 2-2-d1E02-2-0448 | 785.255 | OEt | A01 | a1 | B04 | b4 | C02 | c2 | D02 | dl | E02 |
| 2-2-d1E02-2-0449 | 786.200 | OEt | A02 | a1 | B05 | b1 | C05 | c2 | D02 | dl | E02 |
| 2-2-d1E02-2-0450 | 786.237 | OEt | A02 | a1 | B03 | b3 | C11 | c2 | D02 | dl | E02 |
| 2-2-d1E02-2-0451 | 786.255 | OEt | A01 | a1 | B05 | b4 | C01 | c2 | D02 | dl | E02 |
| 2-2-d1E02-2-0452 | 787.209 | OEt | A02 | a1 | B04 | b3 | C09 | c2 | D02 | dl | E02 |
| 2-2-d1E02-2-0453 | 787.213 | OEt | A02 | a1 | B05 | b3 | C08 | c2 | D02 | dl | E02 |
| 2-2-d1E02-2-0454 | 788.300 | OEt | A01 | a1 | B03 | b1 | C19 | c2 | D02 | dl | E02 |
| 2-2-d1E02-2-0455 | 789.225 | OEt | A02 | a1 | B04 | b1 | C14 | c2 | D02 | dl | E02 |
| 2-2-d1E02-2-0456 | 789.331 | OEt | A02 | a1 | B04 | b1 | C19 | c1 | D01 | dl | E02 |
| 2-2-d1E02-2-0457 | 790.207 | OEt | A01 | a1 | B05 | b1 | C18 | c2 | D02 | dl | E02 |
| 2-2-d1E02-2-0458 | 791.212 | OEt | A01 | a1 | B04 | b4 | C05 | c2 | D02 | dl | E02 |
| 2-2-d1E02-2-0459 | 792.171 | OEt | A02 | a1 | B04 | b3 | C10 | c2 | D02 | dl | E02 |
| 2-2-d1E02-2-0460 | 793.224 | OEt | A02 | a1 | B04 | b2 | C02 | c2 | D02 | dl | E02 |
| 2-2-d1E02-2-0461 | 793.224 | OEt | A02 | a1 | B04 | b2 | C03 | c2 | D02 | dl | E02 |
| 2-2-d1E02-2-0462 | 794.219 | OEt | A02 | a1 | B04 | b2 | C04 | c2 | D02 | dl | E02 |
| 2-2-d1E02-2-0463 | 794.219 | OEt | A02 | a1 | B04 | b2 | C06 | c2 | D02 | dl | E02 |
| 2-2-d1E02-2-0464 | 794.219 | OEt | A02 | a1 | B04 | b2 | C07 | c2 | D02 | dl | E02 |
| 2-2-d1E02-2-0465 | 796.275 | OEt | A01 | a1 | B05 | b4 | C02 | c2 | D02 | dl | E02 |
| 2-2-d1E02-2-0466 | 796.325 | OEt | A02 | a1 | B01 | b1 | C19 | c2 | D02 | dl | E02 |

(continued)

| [Table 9-7-3] Compound that may be contained in mixture 2-2-dlE02-2 | | | | | | | | | | | |
|---|---|---|---|---|---|---|---|---|---|---|---|
| ID | Exact Mass | | A | a | B | b | C | c | D | d | E |
| 2-2-d1E02-2-0467 | 798.229 | OEt | A02 | a1 | B05 | b3 | C09 | c2 | D02 | dl | E02 |
| 2-2-d1E02-2-0468 | 798.251 | OEt | A02 | a1 | B04 | b1 | C15 | c2 | D02 | dl | E02 |
| 2-2-d1E02-2-0469 | 799.198 | OEt | A01 | a1 | B04 | b3 | C11 | c2 | D02 | dl | E02 |
| 2-2-d1E02-2-0470 | 799.246 | OEt | A02 | a1 | B04 | b1 | C16 | c2 | D02 | dl | E02 |
| 2-2-d1E02-2-0471 | 800.241 | OEt | A02 | a1 | B04 | b1 | C17 | c2 | D02 | dl | E02 |
| 2-2-d1E02-2-0472 | 800.245 | OEt | A02 | a1 | B05 | b1 | C14 | c2 | D02 | dl | E02 |
| 2-2-d1E02-2-0473 | 800.351 | OEt | A02 | a1 | B05 | b1 | C19 | c1 | D01 | dl | E02 |
| 2-2-d1E02-2-0474 | 801.250 | OEt | A02 | a1 | B04 | b4 | C01 | c2 | D02 | dl | E02 |
| 2-2-d1E02-2-0475 | 802.232 | OEt | A01 | a1 | B05 | b4 | C05 | c2 | D02 | dl | E02 |
| 2-2-d1E02-2-0476 | 803.191 | OEt | A02 | a1 | B05 | b3 | C10 | c2 | D02 | dl | E02 |
| 2-2-d1E02-2-0477 | 804.244 | OEt | A02 | a1 | B05 | b2 | C02 | c2 | D02 | dl | E02 |
| 2-2-d1E02-2-0478 | 804.244 | OEt | A02 | a1 | B05 | b2 | C03 | c2 | D02 | dl | E02 |
| 2-2-d1E02-2-0479 | 805.202 | OEt | A02 | a1 | B04 | b1 | C18 | c2 | D02 | dl | E02 |
| 2-2-d1E02-2-0480 | 805.239 | OEt | A02 | a1 | B05 | b2 | C04 | c2 | D02 | dl | E02 |
| 2-2-d1E02-2-0481 | 805.239 | OEt | A02 | a1 | B05 | b2 | C06 | c2 | D02 | dl | E02 |
| 2-2-d1E02-2-0482 | 805.239 | OEt | A02 | a1 | B05 | b2 | C07 | c2 | D02 | dl | E02 |
| 2-2-d1E02-2-0483 | 809.271 | OEt | A02 | a1 | B05 | b1 | C15 | c2 | D02 | dl | E02 |
| 2-2-d1E02-2-0484 | 810.218 | OEt | A01 | a1 | B05 | b3 | C11 | c2 | D02 | dl | E02 |
| 2-2-d1E02-2-0485 | 810.266 | OEt | A02 | a1 | B05 | b1 | C16 | c2 | D02 | dl | E02 |
| 2-2-d1E02-2-0486 | 810.341 | OEt | A02 | a1 | B02 | b1 | C19 | c2 | D02 | dl | E02 |
| 2-2-d1E02-2-0487 | 811.261 | OEt | A02 | a1 | B05 | b1 | C17 | c2 | D02 | dl | E02 |
| 2-2-d1E02-2-0488 | 811.271 | OEt | A02 | a1 | B04 | b4 | C02 | c2 | D02 | dl | E02 |
| 2-2-d1E02-2-0489 | 812.270 | OEt | A02 | a1 | B05 | b4 | C01 | c2 | D02 | dl | E02 |
| 2-2-d1E02-2-0490 | 814.316 | OEt | A02 | a1 | B03 | b1 | C19 | c2 | D02 | dl | E02 |
| 2-2-d1E02-2-0491 | 816.222 | OEt | A02 | a1 | B05 | b1 | C18 | c2 | D02 | dl | E02 |
| 2-2-d1E02-2-0492 | 817.227 | OEt | A02 | a1 | B04 | b4 | C05 | c2 | D02 | dl | E02 |
| 2-2-d1E02-2-0493 | 822.291 | OEt | A02 | a1 | B05 | b4 | C02 | c2 | D02 | dl | E02 |
| 2-2-d1E02-2-0494 | 825.214 | OEt | A02 | a1 | B04 | b3 | C11 | c2 | D02 | dl | E02 |
| 2-2-d1E02-2-0495 | 827.277 | OEt | A01 | a1 | B04 | b1 | C19 | c2 | D02 | dl | E02 |
| 2-2-d1E02-2-0496 | 828.247 | OEt | A02 | a1 | B05 | b4 | COS | c2 | D02 | dl | E02 |
| 2-2-d1E02-2-0497 | 836.234 | OEt | A02 | a1 | B05 | b3 | C11 | c2 | D02 | dl | E02 |
| 2-2-d1E02-2-0498 | 838.297 | OEt | A01 | a1 | B05 | b1 | C19 | c2 | D02 | dl | E02 |
| 2-2-d1E02-2-0499 | 853.293 | OEt | A02 | a1 | B04 | b1 | C19 | c2 | D02 | dl | E02 |
| 2-2-d1E02-2-0500 | 864.313 | OEt | A02 | a1 | B05 | b1 | C19 | c2 | D02 | dl | E02 |

[Table 501]

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0001 | 650.220 | OEt | A01 | a1 | B01 | b1 | C01 | c1 | D01 | dl | E04 |
| 2-2-d1E03-2-0002 | 654.288 | OEt | A01 | a1 | B01 | b4 | C12 | c1 | D01 | dl | E04 |
| 2-2-d1E03-2-0003 | 656.267 | OEt | A01 | a1 | B01 | b4 | C13 | c1 | D01 | dl | E04 |
| 2-2-d1E03-2-0004 | 660.241 | OEt | A01 | a1 | B01 | b1 | C02 | c1 | D01 | dl | E04 |
| 2-2-d1E03-2-0005 | 660.241 | OEt | A01 | a1 | B01 | b1 | C03 | c1 | D01 | dl | E04 |
| 2-2-d1E03-2-0006 | 661.236 | OEt | A01 | a1 | B01 | b1 | C04 | c1 | D01 | dl | E04 |
| 2-2-d1E03-2-0007 | 664.236 | OEt | A01 | a1 | B02 | b1 | C01 | c1 | D01 | dl | E04 |
| 2-2-d1E03-2-0008 | 666.197 | OEt | A01 | a1 | B01 | b1 | C05 | c1 | D01 | dl | E04 |
| 2-2-d1E03-2-0009 | 667.210 | OEt | A01 | a1 | B01 | b3 | C08 | c1 | D01 | dl | E04 |
| 2-2-d1E03-2-0010 | 668.210 | OEt | A01 | a1 | B03 | b1 | C01 | c1 | D01 | dl | E04 |
| 2-2-d1E03-2-0011 | 668.303 | OEt | A01 | a1 | B02 | b4 | C12 | c1 | D01 | dl | E04 |
| 2-2-d1E03-2-0012 | 670.283 | OEt | A01 | a1 | B02 | b4 | C13 | c1 | D01 | dl | E04 |
| 2-2-d1E03-2-0013 | 672.278 | OEt | A01 | a1 | B03 | b4 | C12 | c1 | D01 | dl | E04 |
| 2-2-d1E03-2-0014 | 674.256 | OEt | A01 | a1 | B02 | b1 | C02 | c1 | D01 | dl | E04 |
| 2-2-d1E03-2-0015 | 674.256 | OEt | A01 | a1 | B02 | b1 | C03 | c1 | D01 | dl | E04 |
| 2-2-d1E03-2-0016 | 674.257 | OEt | A01 | a1 | B03 | b4 | C13 | c1 | D01 | dl | E04 |
| 2-2-d1E03-2-0017 | 675.252 | OEt | A01 | a1 | B02 | b1 | C04 | c1 | D01 | dl | E04 |
| 2-2-d1E03-2-0018 | 676.236 | OEt | A02 | a1 | B01 | b1 | C01 | c1 | D01 | dl | E04 |
| 2-2-d1E03-2-0019 | 678.226 | OEt | A01 | a1 | B01 | b3 | C09 | c1 | D01 | dl | E04 |
| 2-2-d1E03-2-0020 | 678.231 | OEt | A01 | a1 | B03 | b1 | C02 | c1 | D01 | dl | E04 |
| 2-2-d1E03-2-0021 | 678.231 | OEt | A01 | a1 | B03 | b1 | C03 | c1 | D01 | dl | E04 |
| 2-2-d1E03-2-0022 | 679.226 | OEt | A01 | a1 | B03 | b1 | C04 | c1 | D01 | dl | E04 |
| 2-2-d1E03-2-0023 | 680.213 | OEt | A01 | a1 | B02 | b1 | C05 | c1 | D01 | dl | E04 |
| 2-2-d1E03-2-0024 | 680.242 | OEt | A01 | a1 | B01 | b1 | C14 | c1 | D01 | dl | E04 |
| 2-2-d1E03-2-0025 | 680.303 | OEt | A02 | a1 | B01 | b4 | C12 | c1 | D01 | dl | E04 |
| 2-2-d1E03-2-0026 | 681.226 | OEt | A01 | a1 | B02 | b3 | C08 | c1 | D01 | dl | E04 |
| 2-2-d1E03-2-0027 | 682.283 | OEt | A02 | a1 | B01 | b4 | C13 | c1 | D01 | dl | E04 |
| 2-2-d1E03-2-0028 | 683.187 | OEt | A01 | a1 | B01 | b3 | C10 | c1 | D01 | dl | E04 |
| 2-2-d1E03-2-0029 | 684.188 | OEt | A01 | a1 | B03 | b1 | C05 | c1 | D01 | dl | E04 |
| 2-2-d1E03-2-0030 | 684.241 | OEt | A01 | a1 | B01 | b2 | C02 | c1 | D01 | dl | E04 |
| 2-2-d1E03-2-0031 | 684.241 | OEt | A01 | a1 | B01 | b2 | C03 | c1 | D01 | dl | E04 |
| 2-2-d1E03-2-0032 | 685.201 | OEt | A01 | a1 | B03 | b3 | C08 | c1 | D01 | dl | E04 |
| 2-2-d1E03-2-0033 | 685.236 | OEt | A01 | a1 | B01 | b2 | C04 | c1 | D01 | dl | E04 |
| 2-2-d1E03-2-0034 | 685.236 | OEt | A01 | a1 | B01 | b2 | C06 | c1 | D01 | dl | E04 |
| 2-2-d1E03-2-0035 | 685.236 | OEt | A01 | a1 | B01 | b2 | C07 | c1 | D01 | dl | E04 |
| 2-2-d1E03-2-0036 | 686.256 | OEt | A02 | a1 | B01 | b1 | C02 | c1 | D01 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0037 | 686.256 | OEt | A02 | a1 | B01 | b1 | C03 | c1 | D01 | dl | E04 |
| 2-2-d1E03-2-0038 | 687.252 | OEt | A02 | a1 | B01 | b1 | C04 | c1 | D01 | dl | E04 |
| 2-2-d1E03-2-0039 | 689.267 | OEt | A01 | a1 | B01 | b1 | C15 | c1 | D01 | dl | E04 |
| 2-2-d1E03-2-0040 | 690.251 | OEt | A02 | a1 | B02 | b1 | C01 | c1 | D01 | dl | E04 |
| 2-2-d1E03-2-0041 | 690.262 | OEt | A01 | a1 | B01 | b1 | C16 | c1 | D01 | dl | E04 |
| 2-2-d1E03-2-0042 | 691.258 | OEt | A01 | a1 | B01 | b1 | C17 | c1 | D01 | dl | E04 |
| 2-2-d1E03-2-0043 | 692.213 | OEt | A02 | a1 | B01 | b1 | C05 | c1 | D01 | dl | E04 |
| 2-2-d1E03-2-0044 | 692.242 | OEt | A01 | a1 | B02 | b3 | C09 | c1 | D01 | dl | E04 |
| 2-2-d1E03-2-0045 | 692.267 | OEt | A01 | a1 | B01 | b4 | C01 | c1 | D01 | dl | E04 |
| 2-2-d1E03-2-0046 | 693.226 | OEt | A02 | a1 | B01 | b3 | C08 | c1 | D01 | dl | E04 |
| 2-2-d1E03-2-0047 | 694.226 | OEt | A02 | a1 | B03 | b1 | C01 | c1 | D01 | dl | E04 |
| 2-2-d1E03-2-0048 | 694.257 | OEt | A01 | a1 | B02 | b1 | C14 | c1 | D01 | dl | E04 |
| 2-2-d1E03-2-0049 | 694.319 | OEt | A02 | a1 | B02 | b4 | C12 | c1 | D01 | dl | E04 |
| 2-2-d1E03-2-0050 | 696.217 | OEt | A01 | a1 | B03 | b3 | C09 | c1 | D01 | dl | E04 |
| 2-2-d1E03-2-0051 | 696.219 | OEt | A01 | a1 | B01 | b1 | C18 | c1 | D01 | dl | E04 |
| 2-2-d1E03-2-0052 | 696.298 | OEt | A02 | a1 | B02 | b4 | C13 | c1 | D01 | dl | E04 |
| 2-2-d1E03-2-0053 | 697.203 | OEt | A01 | a1 | B02 | b3 | C10 | c1 | D01 | dl | E04 |
| 2-2-d1E03-2-0054 | 698.232 | OEt | A01 | a1 | B03 | b1 | C14 | c1 | D01 | dl | E04 |
| 2-2-d1E03-2-0055 | 698.256 | OEt | A01 | a1 | B02 | b2 | C02 | c1 | D01 | dl | E04 |
| 2-2-d1E03-2-0056 | 698.256 | OEt | A01 | a1 | B02 | b2 | C03 | c1 | D01 | dl | E04 |
| 2-2-d1E03-2-0057 | 698.294 | OEt | A02 | a1 | B03 | b4 | C12 | c1 | D01 | dl | E04 |
| 2-2-d1E03-2-0058 | 699.252 | OEt | A01 | a1 | B02 | b2 | C04 | c1 | D01 | dl | E04 |
| 2-2-d1E03-2-0059 | 699.252 | OEt | A01 | a1 | B02 | b2 | C06 | c1 | D01 | dl | E04 |
| 2-2-d1E03-2-0060 | 699.252 | OEt | A01 | a1 | B02 | b2 | C07 | c1 | D01 | dl | E04 |
| 2-2-d1E03-2-0061 | 700.272 | OEt | A02 | a1 | B02 | b1 | C02 | c1 | D01 | dl | E04 |
| 2-2-d1E03-2-0062 | 700.272 | OEt | A02 | a1 | B02 | b1 | C03 | c1 | D01 | dl | E04 |
| 2-2-d1E03-2-0063 | 700.273 | OEt | A02 | a1 | B03 | b4 | C13 | c1 | D01 | dl | E04 |
| 2-2-d1E03-2-0064 | 701.178 | OEt | A01 | a1 | B03 | b3 | C10 | c1 | D01 | dl | E04 |
| 2-2-d1E03-2-0065 | 701.267 | OEt | A02 | a1 | B02 | b1 | C04 | c1 | D01 | dl | E04 |
| 2-2-d1E03-2-0066 | 702.231 | OEt | A01 | a1 | B03 | b2 | C02 | c1 | D01 | dl | E04 |
| 2-2-d1E03-2-0067 | 702.231 | OEt | A01 | a1 | B03 | b2 | C03 | c1 | D01 | dl | E04 |
| 2-2-d1E03-2-0068 | 702.288 | OEt | A01 | a1 | B01 | b4 | C02 | c1 | D01 | dl | E04 |
| 2-2-d1E03-2-0069 | 703.226 | OEt | A01 | a1 | B03 | b2 | C04 | c1 | D01 | dl | E04 |
| 2-2-d1E03-2-0070 | 703.226 | OEt | A01 | a1 | B03 | b2 | C06 | c1 | D01 | dl | E04 |
| 2-2-d1E03-2-0071 | 703.226 | OEt | A01 | a1 | B03 | b2 | C07 | c1 | D01 | dl | E04 |
| 2-2-d1E03-2-0072 | 703.283 | OEt | A01 | a1 | B02 | b1 | C15 | c1 | D01 | dl | E04 |
| 2-2-d1E03-2-0073 | 704.242 | OEt | A02 | a1 | B01 | b3 | C09 | c1 | D01 | dl | E04 |
| 2-2-d1E03-2-0074 | 704.247 | OEt | A02 | a1 | B03 | b1 | C02 | c1 | D01 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0075 | 704.247 | OEt | A02 | a1 | B03 | b1 | C03 | c1 | D01 | dl | E04 |
| 2-2-d1E03-2-0076 | 704.278 | OEt | A01 | a1 | B02 | b1 | C16 | c1 | D01 | dl | E04 |
| 2-2-d1E03-2-0077 | 705.242 | OEt | A02 | a1 | B03 | b1 | C04 | c1 | D01 | dl | E04 |
| 2-2-d1E03-2-0078 | 705.273 | OEt | A01 | a1 | B02 | b1 | C17 | c1 | D01 | dl | E04 |
| 2-2-d1E03-2-0079 | 706.228 | OEt | A02 | a1 | B02 | b1 | C05 | c1 | D01 | dl | E04 |
| 2-2-d1E03-2-0080 | 706.257 | OEt | A02 | a1 | B01 | b1 | C14 | c1 | D01 | dl | E04 |
| 2-2-d1E03-2-0081 | 706.283 | OEt | A01 | a1 | B02 | b4 | C01 | c1 | D01 | dl | E04 |
| 2-2-d1E03-2-0082 | 707.187 | OEt | A01 | a1 | B04 | b1 | C01 | c1 | D01 | dl | E04 |
| 2-2-d1E03-2-0083 | 707.241 | OEt | A02 | a1 | B02 | b3 | C08 | c1 | D01 | dl | E04 |
| 2-2-d1E03-2-0084 | 707.258 | OEt | A01 | a1 | B03 | b1 | C15 | c1 | D01 | dl | E04 |
| 2-2-d1E03-2-0085 | 708.244 | OEt | A01 | a1 | B01 | b4 | COS | c1 | D01 | dl | E04 |
| 2-2-d1E03-2-0086 | 708.253 | OEt | A01 | a1 | B03 | b1 | C16 | c1 | D01 | dl | E04 |
| 2-2-d1E03-2-0087 | 709.203 | OEt | A02 | a1 | B01 | b3 | C10 | c1 | D01 | dl | E04 |
| 2-2-d1E03-2-0088 | 709.248 | OEt | A01 | a1 | B03 | b1 | C17 | c1 | D01 | dl | E04 |
| 2-2-d1E03-2-0089 | 710.203 | OEt | A02 | a1 | B03 | b1 | C05 | c1 | D01 | dl | E04 |
| 2-2-d1E03-2-0090 | 710.235 | OEt | A01 | a1 | B02 | b1 | C18 | c1 | D01 | dl | E04 |
| 2-2-d1E03-2-0091 | 710.256 | OEt | A02 | a1 | B01 | b2 | C02 | c1 | D01 | dl | E04 |
| 2-2-d1E03-2-0092 | 710.256 | OEt | A02 | a1 | B01 | b2 | C03 | c1 | D01 | dl | E04 |
| 2-2-d1E03-2-0093 | 710.257 | OEt | A01 | a1 | B03 | b4 | C01 | c1 | D01 | dl | E04 |
| 2-2-d1E03-2-0094 | 711.216 | OEt | A02 | a1 | B03 | b3 | C08 | c1 | D01 | dl | E04 |
| 2-2-d1E03-2-0095 | 711.252 | OEt | A02 | a1 | B01 | b2 | C04 | c1 | D01 | dl | E04 |
| 2-2-d1E03-2-0096 | 711.252 | OEt | A02 | a1 | B01 | b2 | C06 | c1 | D01 | dl | E04 |
| 2-2-d1E03-2-0097 | 711.252 | OEt | A02 | a1 | B01 | b2 | C07 | c1 | D01 | dl | E04 |
| 2-2-d1E03-2-0098 | 711.255 | OEt | A01 | a1 | B04 | b4 | C12 | c1 | D01 | dl | E04 |
| 2-2-d1E03-2-0099 | 713.234 | OEt | A01 | a1 | B04 | b4 | C13 | c1 | D01 | dl | E04 |
| 2-2-d1E03-2-0100 | 714.182 | OEt | A01 | a1 | B01 | b1 | C01 | c2 | D02 | dl | E04 |
| 2-2-d1E03-2-0101 | 714.209 | OEt | A01 | a1 | B03 | b1 | C18 | c1 | D01 | dl | E04 |
| 2-2-d1E03-2-0102 | 715.283 | OEt | A02 | a1 | B01 | b1 | C15 | c1 | D01 | dl | E04 |
| 2-2-d1E03-2-0103 | 716.230 | OEt | A01 | a1 | B01 | b3 | C11 | c1 | D01 | dl | E04 |
| 2-2-d1E03-2-0104 | 716.278 | OEt | A02 | a1 | B01 | b1 | C16 | c1 | D01 | dl | E04 |
| 2-2-d1E03-2-0105 | 716.303 | OEt | A01 | a1 | B02 | b4 | C02 | c1 | D01 | dl | E04 |
| 2-2-d1E03-2-0106 | 717.208 | OEt | A01 | a1 | B04 | b1 | C02 | c1 | D01 | dl | E04 |
| 2-2-d1E03-2-0107 | 717.208 | OEt | A01 | a1 | B04 | b1 | C03 | c1 | D01 | dl | E04 |
| 2-2-d1E03-2-0108 | 717.273 | OEt | A02 | a1 | B01 | b1 | C17 | c1 | D01 | dl | E04 |
| 2-2-d1E03-2-0109 | 718.203 | OEt | A01 | a1 | B04 | b1 | C04 | c1 | D01 | dl | E04 |
| 2-2-d1E03-2-0110 | 718.207 | OEt | A01 | a1 | B05 | b1 | C01 | c1 | D01 | dl | E04 |
| 2-2-d1E03-2-0111 | 718.249 | OEt | A01 | a1 | B01 | b4 | C12 | c2 | D02 | dl | E04 |
| 2-2-d1E03-2-0112 | 718.257 | OEt | A02 | a1 | B02 | b3 | C09 | c1 | D01 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0113 | 718.283 | OEt | A02 | a1 | B01 | b4 | C01 | c1 | D01 | dl | E04 |
| 2-2-d1E03-2-0114 | 720.229 | OEt | A01 | a1 | B01 | b4 | C13 | c2 | D02 | dl | E04 |
| 2-2-d1E03-2-0115 | 720.273 | OEt | A02 | a1 | B02 | b1 | C14 | c1 | D01 | dl | E04 |
| 2-2-d1E03-2-0116 | 720.278 | OEt | A01 | a1 | B03 | b4 | C02 | c1 | D01 | dl | E04 |
| 2-2-d1E03-2-0117 | 722.232 | OEt | A02 | a1 | B03 | b3 | C09 | c1 | D01 | dl | E04 |
| 2-2-d1E03-2-0118 | 722.235 | OEt | A02 | a1 | B01 | b1 | C18 | c1 | D01 | dl | E04 |
| 2-2-d1E03-2-0119 | 722.260 | OEt | A01 | a1 | B02 | b4 | C05 | c1 | D01 | dl | E04 |
| 2-2-d1E03-2-0120 | 722.275 | OEt | A01 | a1 | B05 | b4 | C12 | c1 | D01 | dl | E04 |
| 2-2-d1E03-2-0121 | 723.164 | OEt | A01 | a1 | B04 | b1 | C05 | c1 | D01 | dl | E04 |
| 2-2-d1E03-2-0122 | 723.219 | OEt | A02 | a1 | B02 | b3 | C10 | c1 | D01 | dl | E04 |
| 2-2-d1E03-2-0123 | 724.177 | OEt | A01 | a1 | B04 | b3 | C08 | c1 | D01 | dl | E04 |
| 2-2-d1E03-2-0124 | 724.203 | OEt | A01 | a1 | B01 | b1 | C02 | c2 | D02 | dl | E04 |
| 2-2-d1E03-2-0125 | 724.203 | OEt | A01 | a1 | B01 | b1 | C03 | c2 | D02 | dl | E04 |
| 2-2-d1E03-2-0126 | 724.248 | OEt | A02 | a1 | B03 | b1 | C14 | c1 | D01 | dl | E04 |
| 2-2-d1E03-2-0127 | 724.254 | OEt | A01 | a1 | B05 | b4 | C13 | c1 | D01 | dl | E04 |
| 2-2-d1E03-2-0128 | 724.272 | OEt | A02 | a1 | B02 | b2 | C02 | c1 | D01 | dl | E04 |
| 2-2-d1E03-2-0129 | 724.272 | OEt | A02 | a1 | B02 | b2 | C03 | c1 | D01 | dl | E04 |
| 2-2-d1E03-2-0130 | 725.198 | OEt | A01 | a1 | B01 | b1 | C04 | c2 | D02 | dl | E04 |
| 2-2-d1E03-2-0131 | 725.267 | OEt | A02 | a1 | B02 | b2 | C04 | c1 | D01 | dl | E04 |
| 2-2-d1E03-2-0132 | 725.267 | OEt | A02 | a1 | B02 | b2 | C06 | c1 | D01 | dl | E04 |
| 2-2-d1E03-2-0133 | 725.267 | OEt | A02 | a1 | B02 | b2 | C07 | c1 | D01 | dl | E04 |
| 2-2-d1E03-2-0134 | 726.235 | OEt | A01 | a1 | B03 | b4 | C05 | c1 | D01 | dl | E04 |
| 2-2-d1E03-2-0135 | 727.193 | OEt | A02 | a1 | B03 | b3 | C10 | c1 | D01 | dl | E04 |
| 2-2-d1E03-2-0136 | 728.197 | OEt | A01 | a1 | B02 | b1 | C01 | c2 | D02 | dl | E04 |
| 2-2-d1E03-2-0137 | 728.228 | OEt | A01 | a1 | B05 | b1 | C02 | c1 | D01 | dl | E04 |
| 2-2-d1E03-2-0138 | 728.228 | OEt | A01 | a1 | B05 | b1 | C03 | c1 | D01 | dl | E04 |
| 2-2-d1E03-2-0139 | 728.247 | OEt | A02 | a1 | B03 | b2 | C02 | c1 | D01 | dl | E04 |
| 2-2-d1E03-2-0140 | 728.247 | OEt | A02 | a1 | B03 | b2 | C03 | c1 | D01 | dl | E04 |
| 2-2-d1E03-2-0141 | 728.303 | OEt | A02 | a1 | B01 | b4 | C02 | c1 | D01 | dl | E04 |
| 2-2-d1E03-2-0142 | 729.223 | OEt | A01 | a1 | B05 | b1 | C04 | c1 | D01 | dl | E04 |
| 2-2-d1E03-2-0143 | 729.242 | OEt | A02 | a1 | B03 | b2 | C04 | c1 | D01 | dl | E04 |
| 2-2-d1E03-2-0144 | 729.242 | OEt | A02 | a1 | B03 | b2 | C06 | c1 | D01 | dl | E04 |
| 2-2-d1E03-2-0145 | 729.242 | OEt | A02 | a1 | B03 | b2 | C07 | c1 | D01 | dl | E04 |
| 2-2-d1E03-2-0146 | 729.298 | OEt | A02 | a1 | B02 | b1 | C15 | c1 | D01 | dl | E04 |
| 2-2-d1E03-2-0147 | 730.159 | OEt | A01 | a1 | B01 | b1 | C05 | c2 | D02 | dl | E04 |
| 2-2-d1E03-2-0148 | 730.246 | OEt | A01 | a1 | B02 | b3 | C11 | c1 | D01 | dl | E04 |
| 2-2-d1E03-2-0149 | 730.294 | OEt | A02 | a1 | B02 | b1 | C16 | c1 | D01 | dl | E04 |
| 2-2-d1E03-2-0150 | 731.172 | OEt | A01 | a1 | B01 | b3 | C08 | c2 | D02 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0151 | 731.289 | OEt | A02 | a1 | B02 | b1 | C17 | c1 | D01 | dl | E04 |
| 2-2-d1E03-2-0152 | 732.172 | OEt | A01 | a1 | B03 | b1 | C01 | c2 | D02 | dl | E04 |
| 2-2-d1E03-2-0153 | 732.265 | OEt | A01 | a1 | B02 | b4 | C12 | c2 | D02 | dl | E04 |
| 2-2-d1E03-2-0154 | 732.298 | OEt | A02 | a1 | B02 | b4 | C01 | c1 | D01 | dl | E04 |
| 2-2-d1E03-2-0155 | 733.203 | OEt | A02 | a1 | B04 | b1 | C01 | c1 | D01 | dl | E04 |
| 2-2-d1E03-2-0156 | 733.273 | OEt | A02 | a1 | B03 | b1 | C15 | c1 | D01 | dl | E04 |
| 2-2-d1E03-2-0157 | 734.184 | OEt | A01 | a1 | B05 | b1 | C05 | c1 | D01 | dl | E04 |
| 2-2-d1E03-2-0158 | 734.221 | OEt | A01 | a1 | B03 | b3 | C11 | c1 | D01 | dl | E04 |
| 2-2-d1E03-2-0159 | 734.244 | OEt | A01 | a1 | B02 | b4 | C13 | c2 | D02 | dl | E04 |
| 2-2-d1E03-2-0160 | 734.260 | OEt | A02 | a1 | B01 | b4 | C05 | c1 | D01 | dl | E04 |
| 2-2-d1E03-2-0161 | 734.269 | OEt | A02 | a1 | B03 | b1 | C16 | c1 | D01 | dl | E04 |
| 2-2-d1E03-2-0162 | 735.193 | OEt | A01 | a1 | B04 | b3 | C09 | c1 | D01 | dl | E04 |
| 2-2-d1E03-2-0163 | 735.197 | OEt | A01 | a1 | B05 | b3 | C08 | c1 | D01 | dl | E04 |
| 2-2-d1E03-2-0164 | 735.264 | OEt | A02 | a1 | B03 | b1 | C17 | c1 | D01 | dl | E04 |
| 2-2-d1E03-2-0165 | 736.240 | OEt | A01 | a1 | B03 | b4 | C12 | c2 | D02 | dl | E04 |
| 2-2-d1E03-2-0166 | 736.250 | OEt | A02 | a1 | B02 | b1 | C18 | c1 | D01 | dl | E04 |
| 2-2-d1E03-2-0167 | 736.273 | OEt | A02 | a1 | B03 | b4 | C01 | c1 | D01 | dl | E04 |
| 2-2-d1E03-2-0168 | 737.209 | OEt | A01 | a1 | B04 | b1 | C14 | c1 | D01 | dl | E04 |
| 2-2-d1E03-2-0169 | 737.271 | OEt | A02 | a1 | B04 | b4 | C12 | c1 | D01 | dl | E04 |
| 2-2-d1E03-2-0170 | 738.218 | OEt | A01 | a1 | B02 | b1 | C02 | c2 | D02 | dl | E04 |
| 2-2-d1E03-2-0171 | 738.218 | OEt | A01 | a1 | B02 | b1 | C03 | c2 | D02 | dl | E04 |
| 2-2-d1E03-2-0172 | 738.219 | OEt | A01 | a1 | B03 | b4 | C13 | c2 | D02 | dl | E04 |
| 2-2-d1E03-2-0173 | 739.213 | OEt | A01 | a1 | B02 | b1 | C04 | c2 | D02 | dl | E04 |
| 2-2-d1E03-2-0174 | 739.250 | OEt | A02 | a1 | B04 | b4 | C13 | c1 | D01 | dl | E04 |
| 2-2-d1E03-2-0175 | 740.155 | OEt | A01 | a1 | B04 | b3 | C10 | c1 | D01 | dl | E04 |
| 2-2-d1E03-2-0176 | 740.197 | OEt | A02 | a1 | B01 | b1 | C01 | c2 | D02 | dl | E04 |
| 2-2-d1E03-2-0177 | 740.225 | OEt | A02 | a1 | B03 | b1 | C18 | c1 | D01 | dl | E04 |
| 2-2-d1E03-2-0178 | 741.208 | OEt | A01 | a1 | B04 | b2 | C02 | c1 | D01 | dl | E04 |
| 2-2-d1E03-2-0179 | 741.208 | OEt | A01 | a1 | B04 | b2 | C03 | c1 | D01 | dl | E04 |
| 2-2-d1E03-2-0180 | 742.188 | OEt | A01 | a1 | B01 | b3 | C09 | c2 | D02 | dl | E04 |
| 2-2-d1E03-2-0181 | 742.193 | OEt | A01 | a1 | B03 | b1 | C02 | c2 | D02 | dl | E04 |
| 2-2-d1E03-2-0182 | 742.193 | OEt | A01 | a1 | B03 | b1 | C03 | c2 | D02 | dl | E04 |
| 2-2-d1E03-2-0183 | 742.203 | OEt | A01 | a1 | B04 | b2 | C04 | c1 | D01 | dl | E04 |
| 2-2-d1E03-2-0184 | 742.203 | OEt | A01 | a1 | B04 | b2 | C06 | c1 | D01 | dl | E04 |
| 2-2-d1E03-2-0185 | 742.203 | OEt | A01 | a1 | B04 | b2 | C07 | c1 | D01 | dl | E04 |
| 2-2-d1E03-2-0186 | 742.246 | OEt | A02 | a1 | B01 | b3 | C11 | c1 | D01 | dl | E04 |
| 2-2-d1E03-2-0187 | 742.319 | OEt | A02 | a1 | B02 | b4 | C02 | c1 | D01 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0188 | 743.188 | OEt | A01 | a1 | B03 | b1 | C04 | c2 | D02 | dl | E04 |
| 2-2-d1E03-2-0189 | 743.224 | OEt | A02 | a1 | B04 | b1 | C02 | c1 | D01 | dl | E04 |
| 2-2-d1E03-2-0190 | 743.224 | OEt | A02 | a1 | B04 | b1 | C03 | c1 | D01 | dl | E04 |
| 2-2-d1E03-2-0191 | 744.175 | OEt | A01 | a1 | B02 | b1 | C05 | c2 | D02 | dl | E04 |
| 2-2-d1E03-2-0192 | 744.204 | OEt | A01 | a1 | B01 | b1 | C14 | c2 | D02 | dl | E04 |
| 2-2-d1E03-2-0193 | 744.219 | OEt | A02 | a1 | B04 | b1 | C04 | c1 | D01 | dl | E04 |
| 2-2-d1E03-2-0194 | 744.223 | OEt | A02 | a1 | B05 | b1 | C01 | c1 | D01 | dl | E04 |
| 2-2-d1E03-2-0195 | 744.265 | OEt | A02 | a1 | B01 | b4 | C12 | c2 | D02 | dl | E04 |
| 2-2-d1E03-2-0196 | 744.309 | OEt | A01 | a1 | B01 | b1 | C19 | c1 | D01 | dl | E04 |
| 2-2-d1E03-2-0197 | 745.188 | OEt | A01 | al | B02 | b3 | C08 | c2 | D02 | dl | E04 |
| 2-2-d1E03-2-0198 | 746.213 | OEt | A01 | al | B05 | b3 | C09 | c1 | D01 | dl | E04 |
| 2-2-d1E03-2-0199 | 746.235 | OEt | A01 | al | B04 | b1 | C15 | c1 | D01 | dl | E04 |
| 2-2-d1E03-2-0200 | 746.244 | OEt | A02 | al | B01 | b4 | C13 | c2 | D02 | dl | E04 |
| 2-2-d1E03-2-0201 | 746.294 | OEt | A02 | al | B03 | b4 | C02 | c1 | D01 | dl | E04 |
| 2-2-d1E03-2-0202 | 747.149 | OEt | A01 | al | B01 | b3 | C10 | c2 | D02 | dl | E04 |
| 2-2-d1E03-2-0203 | 747.230 | OEt | A01 | al | B04 | b1 | C16 | c1 | D01 | dl | E04 |
| 2-2-d1E03-2-0204 | 748.150 | OEt | A01 | al | B03 | b1 | C05 | c2 | D02 | dl | E04 |
| 2-2-d1E03-2-0205 | 748.203 | OEt | A01 | al | B01 | b2 | C02 | c2 | D02 | dl | E04 |
| 2-2-d1E03-2-0206 | 748.203 | OEt | A01 | al | B01 | b2 | C03 | c2 | D02 | dl | E04 |
| 2-2-d1E03-2-0207 | 748.225 | OEt | A01 | al | B04 | b1 | C17 | c1 | D01 | dl | E04 |
| 2-2-d1E03-2-0208 | 748.229 | OEt | A01 | al | B05 | b1 | C14 | c1 | D01 | dl | E04 |
| 2-2-d1E03-2-0209 | 748.275 | OEt | A02 | al | B02 | b4 | C05 | c1 | D01 | dl | E04 |
| 2-2-d1E03-2-0210 | 748.291 | OEt | A02 | al | B05 | b4 | C12 | c1 | D01 | dl | E04 |
| 2-2-d1E03-2-0211 | 749.163 | OEt | A01 | al | B03 | b3 | C08 | c2 | D02 | dl | E04 |
| 2-2-d1E03-2-0212 | 749.180 | OEt | A02 | al | B04 | b1 | C05 | c1 | D01 | dl | E04 |
| 2-2-d1E03-2-0213 | 749.198 | OEt | A01 | al | B01 | b2 | C04 | c2 | D02 | dl | E04 |
| 2-2-d1E03-2-0214 | 749.198 | OEt | A01 | al | B01 | b2 | C06 | c2 | D02 | dl | E04 |
| 2-2-d1E03-2-0215 | 749.198 | OEt | A01 | al | B01 | b2 | C07 | c2 | D02 | dl | E04 |
| 2-2-d1E03-2-0216 | 749.234 | OEt | A01 | al | B04 | b4 | C01 | c1 | D01 | dl | E04 |
| 2-2-d1E03-2-0217 | 750.193 | OEt | A02 | al | B04 | b3 | C08 | c1 | D01 | dl | E04 |
| 2-2-d1E03-2-0218 | 750.218 | OEt | A02 | al | B01 | b1 | C02 | c2 | D02 | dl | E04 |
| 2-2-d1E03-2-0219 | 750.218 | OEt | A02 | al | B01 | b1 | C03 | c2 | D02 | dl | E04 |
| 2-2-d1E03-2-0220 | 750.270 | OEt | A02 | al | B05 | b4 | C13 | c1 | D01 | dl | E04 |
| 2-2-d1E03-2-0221 | 751.175 | OEt | A01 | al | B05 | b3 | C10 | c1 | D01 | dl | E04 |
| 2-2-d1E03-2-0222 | 751.213 | OEt | A02 | al | B01 | b1 | C04 | c2 | D02 | dl | E04 |
| 2-2-d1E03-2-0223 | 752.228 | OEt | A01 | al | B05 | b2 | C02 | c1 | D01 | dl | E04 |
| 2-2-d1E03-2-0224 | 752.228 | OEt | A01 | al | B05 | b2 | C03 | c1 | D01 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0225 | 752.250 | OEt | A02 | al | B03 | b4 | C05 | c1 | D01 | dl | E04 |
| 2-2-d1E03-2-0226 | 753.186 | OEt | A01 | al | B04 | b1 | C18 | c1 | D01 | dl | E04 |
| 2-2-d1E03-2-0227 | 753.223 | OEt | A01 | al | B05 | b2 | C04 | c1 | D01 | dl | E04 |
| 2-2-d1E03-2-0228 | 753.223 | OEt | A01 | al | B05 | b2 | C06 | c1 | D01 | dl | E04 |
| 2-2-d1E03-2-0229 | 753.223 | OEt | A01 | al | B05 | b2 | C07 | c1 | D01 | dl | E04 |
| 2-2-d1E03-2-0230 | 753.229 | OEt | A01 | al | B01 | b1 | C15 | c2 | D02 | dl | E04 |
| 2-2-d1E03-2-0231 | 754.213 | OEt | A02 | al | B02 | b1 | C01 | c2 | D02 | dl | E04 |
| 2-2-d1E03-2-0232 | 754.224 | OEt | A01 | al | B01 | b1 | C16 | c2 | D02 | dl | E04 |
| 2-2-d1E03-2-0233 | 754.244 | OEt | A02 | al | B05 | b1 | C02 | c1 | D01 | dl | E04 |
| 2-2-d1E03-2-0234 | 754.244 | OEt | A02 | al | B05 | b1 | C03 | c1 | D01 | dl | E04 |
| 2-2-d1E03-2-0235 | 755.220 | OEt | A01 | al | B01 | b1 | C17 | c2 | D02 | dl | E04 |
| 2-2-d1E03-2-0236 | 755.239 | OEt | A02 | al | B05 | b1 | C04 | c1 | D01 | dl | E04 |
| 2-2-d1E03-2-0237 | 756.175 | OEt | A02 | al | B01 | b1 | C05 | c2 | D02 | dl | E04 |
| 2-2-d1E03-2-0238 | 756.204 | OEt | A01 | al | B02 | b3 | C09 | c2 | D02 | dl | E04 |
| 2-2-d1E03-2-0239 | 756.229 | OEt | A01 | al | B01 | b4 | C01 | c2 | D02 | dl | E04 |
| 2-2-d1E03-2-0240 | 756.262 | OEt | A02 | al | B02 | b3 | C11 | c1 | D01 | dl | E04 |
| 2-2-d1E03-2-0241 | 757.188 | OEt | A02 | al | B01 | b3 | C08 | c2 | D02 | dl | E04 |
| 2-2-d1E03-2-0242 | 757.255 | OEt | A01 | al | B05 | b1 | C15 | c1 | D01 | dl | E04 |
| 2-2-d1E03-2-0243 | 758.188 | OEt | A02 | al | B03 | b1 | C01 | c2 | D02 | dl | E04 |
| 2-2-d1E03-2-0244 | 758.219 | OEt | A01 | al | B02 | b1 | C14 | c2 | D02 | dl | E04 |
| 2-2-d1E03-2-0245 | 758.250 | OEt | A01 | al | B05 | b1 | C16 | c1 | D01 | dl | E04 |
| 2-2-d1E03-2-0246 | 758.281 | OEt | A02 | al | B02 | b4 | C12 | c2 | D02 | dl | E04 |
| 2-2-d1E03-2-0247 | 758.325 | OEt | A01 | al | B02 | b1 | C19 | c1 | D01 | dl | E04 |
| 2-2-d1E03-2-0248 | 759.245 | OEt | A01 | al | B05 | b1 | C17 | c1 | D01 | dl | E04 |
| 2-2-d1E03-2-0249 | 759.255 | OEt | A01 | al | B04 | b4 | C02 | c1 | D01 | dl | E04 |
| 2-2-d1E03-2-0250 | 760.179 | OEt | A01 | al | B03 | b3 | C09 | c2 | D02 | dl | E04 |
| 2-2-d1E03-2-0251 | 760.181 | OEt | A01 | al | B01 | b1 | C18 | c2 | D02 | dl | E04 |
| 2-2-d1E03-2-0252 | 760.200 | OEt | A02 | al | B05 | b1 | C05 | c1 | D01 | dl | E04 |
| 2-2-d1E03-2-0253 | 760.237 | OEt | A02 | al | B03 | b3 | C11 | c1 | D01 | dl | E04 |
| 2-2-d1E03-2-0254 | 760.254 | OEt | A01 | al | B05 | b4 | C01 | c1 | D01 | dl | E04 |
| 2-2-d1E03-2-0255 | 760.260 | OEt | A02 | al | B02 | b4 | C13 | c2 | D02 | dl | E04 |
| 2-2-d1E03-2-0256 | 761.165 | OEt | A01 | al | B02 | b3 | C10 | c2 | D02 | dl | E04 |
| 2-2-d1E03-2-0257 | 761.209 | OEt | A02 | al | B04 | b3 | C09 | c1 | D01 | dl | E04 |
| 2-2-d1E03-2-0258 | 761.213 | OEt | A02 | al | B05 | b3 | C08 | c1 | D01 | dl | E04 |
| 2-2-d1E03-2-0259 | 762.194 | OEt | A01 | al | B03 | b1 | C14 | c2 | D02 | dl | E04 |
| 2-2-d1E03-2-0260 | 762.218 | OEt | A01 | al | B02 | b2 | C02 | c2 | D02 | dl | E04 |
| 2-2-d1E03-2-0261 | 762.218 | OEt | A01 | al | B02 | b2 | C03 | c2 | D02 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0262 | 762.256 | OEt | A02 | al | B03 | b4 | C12 | c2 | D02 | dl | E04 |
| 2-2-d1E03-2-0263 | 762.300 | OEt | A01 | al | B03 | b1 | C19 | c1 | D01 | dl | E04 |
| 2-2-d1E03-2-0264 | 763.213 | OEt | A01 | al | B02 | b2 | C04 | c2 | D02 | dl | E04 |
| 2-2-d1E03-2-0265 | 763.213 | OEt | A01 | al | B02 | b2 | C06 | c2 | D02 | dl | E04 |
| 2-2-d1E03-2-0266 | 763.213 | OEt | A01 | al | B02 | b2 | C07 | c2 | D02 | dl | E04 |
| 2-2-d1E03-2-0267 | 763.225 | OEt | A02 | al | B04 | b1 | C14 | c1 | D01 | dl | E04 |
| 2-2-d1E03-2-0268 | 764.206 | OEt | A01 | al | B05 | b1 | C18 | c1 | D01 | dl | E04 |
| 2-2-d1E03-2-0269 | 764.234 | OEt | A02 | al | B02 | b1 | C02 | c2 | D02 | dl | E04 |
| 2-2-d1E03-2-0270 | 764.234 | OEt | A02 | al | B02 | b1 | C03 | c2 | D02 | dl | E04 |
| 2-2-d1E03-2-0271 | 764.235 | OEt | A02 | al | B03 | b4 | C13 | c2 | D02 | dl | E04 |
| 2-2-d1E03-2-0272 | 765.140 | OEt | A01 | al | B03 | b3 | C10 | c2 | D02 | dl | E04 |
| 2-2-d1E03-2-0273 | 765.211 | OEt | A01 | al | B04 | b4 | C05 | c1 | D01 | dl | E04 |
| 2-2-d1E03-2-0274 | 765.229 | OEt | A02 | al | B02 | b1 | C04 | c2 | D02 | d1 | E04 |
| 2-2-d1E03-2-0275 | 766.170 | OEt | A02 | al | B04 | b3 | C10 | c1 | D01 | dl | E04 |
| 2-2-d1E03-2-0276 | 766.193 | OEt | A01 | al | B03 | b2 | C02 | c2 | D02 | d1 | E04 |
| 2-2-d1E03-2-0277 | 766.193 | OEt | A01 | al | B03 | b2 | C03 | c2 | D02 | dl | E04 |
| 2-2-d1E03-2-0278 | 766.249 | OEt | A01 | al | B01 | b4 | C02 | c2 | D02 | d1 | E04 |
| 2-2-d1E03-2-0279 | 767.188 | OEt | A01 | al | B03 | b2 | C04 | c2 | D02 | dl | E04 |
| 2-2-d1E03-2-0280 | 767.188 | OEt | A01 | al | B03 | b2 | C06 | c2 | D02 | dl | E04 |
| 2-2-d1E03-2-0281 | 767.188 | OEt | A01 | al | B03 | b2 | C07 | c2 | D02 | dl | E04 |
| 2-2-d1E03-2-0282 | 767.224 | OEt | A02 | al | B04 | b2 | C02 | c1 | D01 | dl | E04 |
| 2-2-d1E03-2-0283 | 767.224 | OEt | A02 | al | B04 | b2 | C03 | c1 | D01 | dl | E04 |
| 2-2-d1E03-2-0284 | 767.245 | OEt | A01 | al | B02 | b1 | C15 | c2 | D02 | dl | E04 |
| 2-2-d1E03-2-0285 | 768.204 | OEt | A02 | al | B01 | b3 | C09 | c2 | D02 | dl | E04 |
| 2-2-d1E03-2-0286 | 768.209 | OEt | A02 | al | B03 | b1 | C02 | c2 | D02 | dl | E04 |
| 2-2-d1E03-2-0287 | 768.209 | OEt | A02 | al | B03 | b1 | C03 | c2 | D02 | dl | E04 |
| 2-2-d1E03-2-0288 | 768.219 | OEt | A02 | al | B04 | b2 | C04 | c1 | D01 | dl | E04 |
| 2-2-d1E03-2-0289 | 768.219 | OEt | A02 | al | B04 | b2 | C06 | c1 | D01 | dl | E04 |
| 2-2-d1E03-2-0290 | 768.219 | OEt | A02 | al | B04 | b2 | C07 | c1 | D01 | dl | E04 |
| 2-2-d1E03-2-0291 | 768.240 | OEt | A01 | al | B02 | b1 | C16 | c2 | D02 | dl | E04 |
| 2-2-d1E03-2-0292 | 769.204 | OEt | A02 | al | B03 | b1 | C04 | c2 | D02 | dl | E04 |
| 2-2-d1E03-2-0293 | 769.235 | OEt | A01 | al | B02 | b1 | C17 | c2 | D02 | dl | E04 |
| 2-2-d1E03-2-0294 | 770.190 | OEt | A02 | al | B02 | b1 | C05 | c2 | D02 | dl | E04 |
| 2-2-d1E03-2-0295 | 770.219 | OEt | A02 | al | B01 | b1 | C14 | c2 | D02 | dl | E04 |
| 2-2-d1E03-2-0296 | 770.244 | OEt | A01 | al | B02 | b4 | C01 | c2 | D02 | dl | E04 |
| 2-2-d1E03-2-0297 | 770.275 | OEt | A01 | al | B05 | b4 | C02 | c1 | D01 | dl | E04 |
| 2-2-d1E03-2-0298 | 770.325 | OEt | A02 | al | B01 | b1 | C19 | c1 | D01 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0299 | 771.149 | OEt | A01 | al | B04 | b1 | C01 | c2 | D02 | dl | E04 |
| 2-2-d1E03-2-0300 | 771.203 | OEt | A02 | al | B02 | b3 | C08 | c2 | D02 | dl | E04 |
| 2-2-d1E03-2-0301 | 771.220 | OEt | A01 | al | B03 | b1 | C15 | c2 | D02 | dl | E04 |
| 2-2-d1E03-2-0302 | 772.206 | OEt | A01 | al | B01 | b4 | C05 | c2 | D02 | dl | E04 |
| 2-2-d1E03-2-0303 | 772.215 | OEt | A01 | al | B03 | b1 | C16 | c2 | D02 | dl | E04 |
| 2-2-d1E03-2-0304 | 772.229 | OEt | A02 | al | B05 | b3 | C09 | c1 | D01 | dl | E04 |
| 2-2-d1E03-2-0305 | 772.250 | OEt | A02 | al | B04 | b1 | C15 | c1 | D01 | dl | E04 |
| 2-2-d1E03-2-0306 | 773.165 | OEt | A02 | al | B01 | b3 | C10 | c2 | D02 | dl | E04 |
| 2-2-d1E03-2-0307 | 773.198 | OEt | A01 | al | B04 | b3 | C11 | c1 | D01 | dl | E04 |
| 2-2-d1E03-2-0308 | 773.210 | OEt | A01 | al | B03 | b1 | C17 | c2 | D02 | dl | E04 |
| 2-2-d1E03-2-0309 | 773.245 | OEt | A02 | al | B04 | b1 | C16 | c1 | D01 | dl | E04 |
| 2-2-d1E03-2-0310 | 774.165 | OEt | A02 | al | B03 | b1 | C05 | c2 | D02 | dl | E04 |
| 2-2-d1E03-2-0311 | 774.196 | OEt | A01 | al | B02 | b1 | C18 | c2 | D02 | dl | E04 |
| 2-2-d1E03-2-0312 | 774.218 | OEt | A02 | al | B01 | b2 | C02 | c2 | D02 | dl | E04 |
| 2-2-d1E03-2-0313 | 774.218 | OEt | A02 | al | B01 | b2 | C03 | c2 | D02 | dl | E04 |
| 2-2-d1E03-2-0314 | 774.219 | OEt | A01 | al | B03 | b4 | C01 | c2 | D02 | dl | E04 |
| 2-2-d1E03-2-0315 | 774.241 | OEt | A02 | al | B04 | b1 | C17 | c1 | D01 | dl | E04 |
| 2-2-d1E03-2-0316 | 774.245 | OEt | A02 | al | B05 | b1 | C14 | c1 | D01 | dl | E04 |
| 2-2-d1E03-2-0317 | 775.178 | OEt | A02 | al | B03 | b3 | C08 | c2 | D02 | dl | E04 |
| 2-2-d1E03-2-0318 | 775.213 | OEt | A02 | al | B01 | b2 | C04 | c2 | D02 | dl | E04 |
| 2-2-d1E03-2-0319 | 775.213 | OEt | A02 | al | B01 | b2 | C06 | c2 | D02 | dl | E04 |
| 2-2-d1E03-2-0320 | 775.213 | OEt | A02 | al | B01 | b2 | C07 | c2 | D02 | dl | E04 |
| 2-2-d1E03-2-0321 | 775.217 | OEt | A01 | al | B04 | b4 | C12 | c2 | D02 | dl | E04 |
| 2-2-d1E03-2-0322 | 775.250 | OEt | A02 | al | B04 | b4 | C01 | c1 | D01 | dl | E04 |
| 2-2-d1E03-2-0323 | 776.231 | OEt | A01 | al | B05 | b4 | C05 | c1 | D01 | dl | E04 |
| 2-2-d1E03-2-0324 | 777.190 | OEt | A02 | al | B05 | b3 | C10 | c1 | D01 | dl | E04 |
| 2-2-d1E03-2-0325 | 777.196 | OEt | A01 | al | B04 | b4 | C13 | c2 | D02 | dl | E04 |
| 2-2-d1E03-2-0326 | 778.171 | OEt | A01 | al | B03 | b1 | C18 | c2 | D02 | dl | E04 |
| 2-2-d1E03-2-0327 | 778.244 | OEt | A02 | al | B05 | b2 | C02 | c1 | D01 | dl | E04 |
| 2-2-d1E03-2-0328 | 778.244 | OEt | A02 | al | B05 | b2 | C03 | c1 | D01 | dl | E04 |
| 2-2-d1E03-2-0329 | 779.202 | OEt | A02 | al | B04 | b1 | C18 | c1 | D01 | dl | E04 |
| 2-2-d1E03-2-0330 | 779.239 | OEt | A02 | al | B05 | b2 | C04 | c1 | D01 | dl | E04 |
| 2-2-d1E03-2-0331 | 779.239 | OEt | A02 | al | B05 | b2 | C06 | c1 | D01 | dl | E04 |
| 2-2-d1E03-2-0332 | 779.239 | OEt | A02 | al | B05 | b2 | C07 | c1 | D01 | dl | E04 |
| 2-2-d1E03-2-0333 | 779.245 | OEt | A02 | al | B01 | b1 | C15 | c2 | D02 | dl | E04 |
| 2-2-d1E03-2-0334 | 780.192 | OEt | A01 | al | B01 | b3 | C11 | c2 | D02 | dl | E04 |
| 2-2-d1E03-2-0335 | 780.240 | OEt | A02 | al | B01 | b1 | C16 | c2 | D02 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0336 | 780.265 | OEt | A01 | al | B02 | b4 | C02 | c2 | D02 | dl | E04 |
| 2-2-d1E03-2-0337 | 781.170 | OEt | A01 | al | B04 | b1 | C02 | c2 | D02 | dl | E04 |
| 2-2-d1E03-2-0338 | 781.170 | OEt | A01 | al | B04 | b1 | C03 | c2 | D02 | dl | E04 |
| 2-2-d1E03-2-0339 | 781.235 | OEt | A02 | al | B01 | b1 | C17 | c2 | D02 | dl | E04 |
| 2-2-d1E03-2-0340 | 782.165 | OEt | A01 | al | B04 | b1 | C04 | c2 | D02 | dl | E04 |
| 2-2-d1E03-2-0341 | 782.169 | OEt | A01 | al | B05 | b1 | C01 | c2 | D02 | dl | E04 |
| 2-2-d1E03-2-0342 | 782.219 | OEt | A02 | al | B02 | b3 | C09 | c2 | D02 | dl | E04 |
| 2-2-d1E03-2-0343 | 782.244 | OEt | A02 | al | B01 | b4 | C01 | c2 | D02 | dl | E04 |
| 2-2-d1E03-2-0344 | 783.270 | OEt | A02 | al | B05 | b1 | C15 | c1 | D01 | dl | E04 |
| 2-2-d1E03-2-0345 | 784.218 | OEt | A01 | al | B05 | b3 | C11 | c1 | D01 | dl | E04 |
| 2-2-d1E03-2-0346 | 784.235 | OEt | A02 | al | B02 | b1 | C14 | c2 | D02 | dl | E04 |
| 2-2-d1E03-2-0347 | 784.240 | OEt | A01 | al | B03 | b4 | C02 | c2 | D02 | dl | E04 |
| 2-2-d1E03-2-0348 | 784.265 | OEt | A02 | al | B05 | b1 | C16 | c1 | D01 | dl | E04 |
| 2-2-d1E03-2-0349 | 784.341 | OEt | A02 | al | B02 | b1 | C19 | c1 | D01 | dl | E04 |
| 2-2-d1E03-2-0350 | 785.261 | OEt | A02 | al | B05 | b1 | C17 | c1 | D01 | dl | E04 |
| 2-2-d1E03-2-0351 | 785.271 | OEt | A02 | al | B04 | b4 | C02 | c1 | D01 | dl | E04 |
| 2-2-d1E03-2-0352 | 786.194 | OEt | A02 | al | B03 | b3 | C09 | c2 | D02 | dl | E04 |
| 2-2-d1E03-2-0353 | 786.196 | OEt | A02 | al | B01 | b1 | C18 | c2 | D02 | dl | E04 |
| 2-2-d1E03-2-0354 | 786.222 | OEt | A01 | al | B02 | b4 | C05 | c2 | D02 | dl | E04 |
| 2-2-d1E03-2-0355 | 786.237 | OEt | A01 | al | B05 | b4 | C12 | c2 | D02 | dl | E04 |
| 2-2-d1E03-2-0356 | 786.270 | OEt | A02 | al | B05 | b4 | C01 | c1 | D01 | dl | E04 |
| 2-2-d1E03-2-0357 | 787.126 | OEt | A01 | al | B04 | b1 | C05 | c2 | D02 | dl | E04 |
| 2-2-d1E03-2-0358 | 787.180 | OEt | A02 | al | B02 | b3 | C10 | c2 | D02 | dl | E04 |
| 2-2-d1E03-2-0359 | 788.139 | OEt | A01 | al | B04 | b3 | C08 | c2 | D02 | dl | E04 |
| 2-2-d1E03-2-0360 | 788.210 | OEt | A02 | al | B03 | b1 | C14 | c2 | D02 | dl | E04 |
| 2-2-d1E03-2-0361 | 788.216 | OEt | A01 | al | B05 | b4 | C13 | c2 | D02 | dl | E04 |
| 2-2-d1E03-2-0362 | 788.234 | OEt | A02 | al | B02 | b2 | C02 | c2 | D02 | dl | E04 |
| 2-2-d1E03-2-0363 | 788.234 | OEt | A02 | al | B02 | b2 | C03 | c2 | D02 | dl | E04 |
| 2-2-d1E03-2-0364 | 788.316 | OEt | A02 | al | B03 | b1 | C19 | c1 | D01 | dl | E04 |
| 2-2-d1E03-2-0365 | 789.229 | OEt | A02 | al | B02 | b2 | C04 | c2 | D02 | dl | E04 |
| 2-2-d1E03-2-0366 | 789.229 | OEt | A02 | al | B02 | b2 | C06 | c2 | D02 | dl | E04 |
| 2-2-d1E03-2-0367 | 789.229 | OEt | A02 | al | B02 | b2 | C07 | c2 | D02 | dl | E04 |
| 2-2-d1E03-2-0368 | 790.196 | OEt | A01 | al | B03 | b4 | C05 | c2 | D02 | dl | E04 |
| 2-2-d1E03-2-0369 | 790.222 | OEt | A02 | al | B05 | b1 | C18 | c1 | D01 | dl | E04 |
| 2-2-d1E03-2-0370 | 791.155 | OEt | A02 | al | B03 | b3 | C10 | c2 | D02 | dl | E04 |
| 2-2-d1E03-2-0371 | 791.227 | OEt | A02 | al | B04 | b4 | C05 | c1 | D01 | dl | E04 |
| 2-2-d1E03-2-0372 | 792.190 | OEt | A01 | al | B05 | b1 | C02 | c2 | D02 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0373 | 792.190 | OEt | A01 | al | B05 | b1 | C03 | c2 | D02 | dl | E04 |
| 2-2-d1E03-2-0374 | 792.209 | OEt | A02 | al | B03 | b2 | C02 | c2 | D02 | dl | E04 |
| 2-2-d1E03-2-0375 | 792.209 | OEt | A02 | al | B03 | b2 | C03 | c2 | D02 | dl | E04 |
| 2-2-d1E03-2-0376 | 792.265 | OEt | A02 | al | B01 | b4 | C02 | c2 | D02 | dl | E04 |
| 2-2-d1E03-2-0377 | 793.185 | OEt | A01 | al | B05 | b1 | C04 | c2 | D02 | dl | E04 |
| 2-2-d1E03-2-0378 | 793.204 | OEt | A02 | al | B03 | b2 | C04 | c2 | D02 | dl | E04 |
| 2-2-d1E03-2-0379 | 793.204 | OEt | A02 | al | B03 | b2 | C06 | c2 | D02 | dl | E04 |
| 2-2-d1E03-2-0380 | 793.204 | OEt | A02 | al | B03 | b2 | C07 | c2 | D02 | dl | E04 |
| 2-2-d1E03-2-0381 | 793.260 | OEt | A02 | al | B02 | b1 | C15 | c2 | D02 | dl | E04 |
| 2-2-d1E03-2-0382 | 794.208 | OEt | A01 | al | B02 | b3 | C11 | c2 | D02 | dl | E04 |
| 2-2-d1E03-2-0383 | 794.256 | OEt | A02 | al | B02 | b1 | C16 | c2 | D02 | dl | E04 |
| 2-2-d1E03-2-0384 | 795.251 | OEt | A02 | al | B02 | b1 | C17 | c2 | D02 | dl | E04 |
| 2-2-d1E03-2-0385 | 796.260 | OEt | A02 | al | B02 | b4 | C01 | c2 | D02 | dl | E04 |
| 2-2-d1E03-2-0386 | 796.291 | OEt | A02 | al | B05 | b4 | C02 | c1 | D01 | dl | E04 |
| 2-2-d1E03-2-0387 | 797.165 | OEt | A02 | al | B04 | b1 | C01 | c2 | D02 | dl | E04 |
| 2-2-d1E03-2-0388 | 797.235 | OEt | A02 | al | B03 | b1 | C15 | c2 | D02 | dl | E04 |
| 2-2-d1E03-2-0389 | 798.146 | OEt | A01 | al | B05 | b1 | C05 | c2 | D02 | dl | E04 |
| 2-2-d1E03-2-0390 | 798.183 | OEt | A01 | al | B03 | b3 | C11 | c2 | D02 | dl | E04 |
| 2-2-d1E03-2-0391 | 798.222 | OEt | A02 | al | B01 | b4 | C05 | c2 | D02 | dl | E04 |
| 2-2-d1E03-2-0392 | 798.231 | OEt | A02 | al | B03 | b1 | C16 | c2 | D02 | dl | E04 |
| 2-2-d1E03-2-0393 | 799.155 | OEt | A01 | al | B04 | b3 | C09 | c2 | D02 | dl | E04 |
| 2-2-d1E03-2-0394 | 799.159 | OEt | A01 | al | B05 | b3 | C08 | c2 | D02 | dl | E04 |
| 2-2-d1E03-2-0395 | 799.213 | OEt | A02 | al | B04 | b3 | C11 | c1 | D01 | dl | E04 |
| 2-2-d1E03-2-0396 | 799.226 | OEt | A02 | al | B03 | b1 | C17 | c2 | D02 | dl | E04 |
| 2-2-d1E03-2-0397 | 800.212 | OEt | A02 | al | B02 | b1 | C18 | c2 | D02 | dl | E04 |
| 2-2-d1E03-2-0398 | 800.235 | OEt | A02 | al | B03 | b4 | C01 | c2 | D02 | dl | E04 |
| 2-2-d1E03-2-0399 | 801.171 | OEt | A01 | al | B04 | b1 | C14 | c2 | D02 | dl | E04 |
| 2-2-d1E03-2-0400 | 801.232 | OEt | A02 | al | B04 | b4 | C12 | c2 | D02 | dl | E04 |
| 2-2-d1E03-2-0401 | 801.277 | OEt | A01 | al | B04 | b1 | C19 | c1 | D01 | dl | E04 |
| 2-2-d1E03-2-0402 | 802.247 | OEt | A02 | al | B05 | b4 | C05 | c1 | D01 | dl | E04 |
| 2-2-d1E03-2-0403 | 803.212 | OEt | A02 | al | B04 | b4 | C13 | c2 | D02 | dl | E04 |
| 2-2-d1E03-2-0404 | 804.116 | OEt | A01 | al | B04 | b3 | C10 | c2 | D02 | dl | E04 |
| 2-2-d1E03-2-0405 | 804.187 | OEt | A02 | al | B03 | b1 | C18 | c2 | D02 | dl | E04 |
| 2-2-d1E03-2-0406 | 805.170 | OEt | A01 | al | B04 | b2 | C02 | c2 | D02 | dl | E04 |
| 2-2-d1E03-2-0407 | 805.170 | OEt | A01 | al | B04 | b2 | C03 | c2 | D02 | dl | E04 |
| 2-2-d1E03-2-0408 | 806.165 | OEt | A01 | al | B04 | b2 | C04 | c2 | D02 | dl | E04 |
| 2-2-d1E03-2-0409 | 806.165 | OEt | A01 | al | B04 | b2 | C06 | c2 | D02 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0410 | 806.165 | OEt | A01 | al | B04 | b2 | C07 | c2 | D02 | dl | E04 |
| 2-2-d1E03-2-0411 | 806.208 | OEt | A02 | al | B01 | b3 | C11 | c2 | D02 | dl | E04 |
| 2-2-d1E03-2-0412 | 806.281 | OEt | A02 | al | B02 | b4 | C02 | c2 | D02 | dl | E04 |
| 2-2-d1E03-2-0413 | 807.186 | OEt | A02 | al | B04 | b1 | C02 | c2 | D02 | dl | E04 |
| 2-2-d1E03-2-0414 | 807.186 | OEt | A02 | al | B04 | b1 | C03 | c2 | D02 | dl | E04 |
| 2-2-d1E03-2-0415 | 808.181 | OEt | A02 | al | B04 | b1 | C04 | c2 | D02 | dl | E04 |
| 2-2-d1E03-2-0416 | 808.185 | OEt | A02 | al | B05 | b1 | C01 | c2 | D02 | dl | E04 |
| 2-2-d1E03-2-0417 | 808.271 | OEt | A01 | al | B01 | b1 | C19 | c2 | D02 | dl | E04 |
| 2-2-d1E03-2-0418 | 810.175 | OEt | A01 | al | B05 | b3 | C09 | c2 | D02 | dl | E04 |
| 2-2-d1E03-2-0419 | 810.196 | OEt | A01 | al | B04 | b1 | C15 | c2 | D02 | dl | E04 |
| 2-2-d1E03-2-0420 | 810.234 | OEt | A02 | al | B05 | b3 | C11 | c1 | D01 | dl | E04 |
| 2-2-d1E03-2-0421 | 810.256 | OEt | A02 | al | B03 | b4 | C02 | c2 | D02 | dl | E04 |
| 2-2-d1E03-2-0422 | 811.192 | OEt | A01 | al | B04 | b1 | C16 | c2 | D02 | dl | E04 |
| 2-2-d1E03-2-0423 | 812.187 | OEt | A01 | al | B04 | b1 | C17 | c2 | D02 | dl | E04 |
| 2-2-d1E03-2-0424 | 812.191 | OEt | A01 | al | B05 | b1 | C14 | c2 | D02 | dl | E04 |
| 2-2-d1E03-2-0425 | 812.237 | OEt | A02 | al | B02 | b4 | C05 | c2 | D02 | dl | E04 |
| 2-2-d1E03-2-0426 | 812.253 | OEt | A02 | al | B05 | b4 | C12 | c2 | D02 | dl | E04 |
| 2-2-d1E03-2-0427 | 812.297 | OEt | A01 | al | B05 | b1 | C19 | c1 | D01 | dl | E04 |
| 2-2-d1E03-2-0428 | 813.142 | OEt | A02 | al | B04 | b1 | C05 | c2 | D02 | dl | E04 |
| 2-2-d1E03-2-0429 | 813.196 | OEt | A01 | al | B04 | b4 | C01 | c2 | D02 | dl | E04 |
| 2-2-d1E03-2-0430 | 814.155 | OEt | A02 | al | B04 | b3 | C08 | c2 | D02 | dl | E04 |
| 2-2-d1E03-2-0431 | 814.232 | OEt | A02 | al | B05 | b4 | C13 | c2 | D02 | dl | E04 |
| 2-2-d1E03-2-0432 | 815.137 | OEt | A01 | al | B05 | b3 | C10 | c2 | D02 | dl | E04 |
| 2-2-d1E03-2-0433 | 816.190 | OEt | A01 | al | B05 | b2 | C02 | c2 | D02 | dl | E04 |
| 2-2-d1E03-2-0434 | 816.190 | OEt | A01 | al | B05 | b2 | C03 | c2 | D02 | dl | E04 |
| 2-2-d1E03-2-0435 | 816.212 | OEt | A02 | al | B03 | b4 | C05 | c2 | D02 | dl | E04 |
| 2-2-d1E03-2-0436 | 817.148 | OEt | A01 | al | B04 | b1 | C18 | c2 | D02 | dl | E04 |
| 2-2-d1E03-2-0437 | 817.185 | OEt | A01 | al | B05 | b2 | C04 | c2 | D02 | dl | E04 |
| 2-2-d1E03-2-0438 | 817.185 | OEt | A01 | al | B05 | b2 | C06 | c2 | D02 | dl | E04 |
| 2-2-d1E03-2-0439 | 817.185 | OEt | A01 | al | B05 | b2 | C07 | c2 | D02 | dl | E04 |
| 2-2-d1E03-2-0440 | 818.206 | OEt | A02 | al | B05 | b1 | C02 | c2 | D02 | dl | E04 |
| 2-2-d1E03-2-0441 | 818.206 | OEt | A02 | al | B05 | b1 | C03 | c2 | D02 | dl | E04 |
| 2-2-d1E03-2-0442 | 819.201 | OEt | A02 | al | B05 | b1 | C04 | c2 | D02 | dl | E04 |
| 2-2-d1E03-2-0443 | 820.224 | OEt | A02 | al | B02 | b3 | C11 | c2 | D02 | dl | E04 |
| 2-2-d1E03-2-0444 | 821.216 | OEt | A01 | al | B05 | b1 | C15 | c2 | D02 | dl | E04 |
| 2-2-d1E03-2-0445 | 822.212 | OEt | A01 | al | B05 | b1 | C16 | c2 | D02 | dl | E04 |
| 2-2-d1E03-2-0446 | 822.287 | OEt | A01 | al | B02 | b1 | C19 | c2 | D02 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0447 | 823.207 | OEt | A01 | al | B05 | b1 | C17 | c2 | D02 | dl | E04 |
| 2-2-d1E03-2-0448 | 823.217 | OEt | A01 | al | B04 | b4 | C02 | c2 | D02 | dl | E04 |
| 2-2-d1E03-2-0449 | 824.162 | OEt | A02 | al | B05 | b1 | C05 | c2 | D02 | dl | E04 |
| 2-2-d1E03-2-0450 | 824.199 | OEt | A02 | al | B03 | b3 | C11 | c2 | D02 | dl | E04 |
| 2-2-d1E03-2-0451 | 824.216 | OEt | A01 | al | B05 | b4 | C01 | c2 | D02 | dl | E04 |
| 2-2-d1E03-2-0452 | 825.171 | OEt | A02 | al | B04 | b3 | C09 | c2 | D02 | dl | E04 |
| 2-2-d1E03-2-0453 | 825.175 | OEt | A02 | al | B05 | b3 | C08 | c2 | D02 | dl | E04 |
| 2-2-d1E03-2-0454 | 826.262 | OEt | A01 | al | B03 | b1 | C19 | c2 | D02 | dl | E04 |
| 2-2-d1E03-2-0455 | 827.187 | OEt | A02 | al | B04 | b1 | C14 | c2 | D02 | dl | E04 |
| 2-2-d1E03-2-0456 | 827.292 | OEt | A02 | al | B04 | b1 | C19 | c1 | D01 | dl | E04 |
| 2-2-d1E03-2-0457 | 828.168 | OEt | A01 | al | B05 | b1 | C18 | c2 | D02 | dl | E04 |
| 2-2-d1E03-2-0458 | 829.173 | OEt | A01 | al | B04 | b4 | C05 | c2 | D02 | dl | E04 |
| 2-2-d1E03-2-0459 | 830.132 | OEt | A02 | al | B04 | b3 | C10 | c2 | D02 | dl | E04 |
| 2-2-d1E03-2-0460 | 831.186 | OEt | A02 | al | B04 | b2 | C02 | c2 | D02 | dl | E04 |
| 2-2-d1E03-2-0461 | 831.186 | OEt | A02 | al | B04 | b2 | C03 | c2 | D02 | dl | E04 |
| 2-2-d1E03-2-0462 | 832.181 | OEt | A02 | al | B04 | b2 | C04 | c2 | D02 | dl | E04 |
| 2-2-d1E03-2-0463 | 832.181 | OEt | A02 | al | B04 | b2 | C06 | c2 | D02 | dl | E04 |
| 2-2-d1E03-2-0464 | 832.181 | OEt | A02 | al | B04 | b2 | C07 | c2 | D02 | dl | E04 |
| 2-2-d1E03-2-0465 | 834.237 | OEt | A01 | al | B05 | b4 | C02 | c2 | D02 | dl | E04 |
| 2-2-d1E03-2-0466 | 834.287 | OEt | A02 | al | B01 | b1 | C19 | c2 | D02 | dl | E04 |
| 2-2-d1E03-2-0467 | 836.191 | OEt | A02 | al | B05 | b3 | C09 | c2 | D02 | dl | E04 |
| 2-2-d1E03-2-0468 | 836.212 | OEt | A02 | al | B04 | b1 | C15 | c2 | D02 | dl | E04 |
| 2-2-d1E03-2-0469 | 837.160 | OEt | A01 | al | B04 | b3 | C11 | c2 | D02 | dl | E04 |
| 2-2-d1E03-2-0470 | 837.207 | OEt | A02 | al | B04 | b1 | C16 | c2 | D02 | dl | E04 |
| 2-2-d1E03-2-0471 | 838.203 | OEt | A02 | al | B04 | b1 | C17 | c2 | D02 | dl | E04 |
| 2-2-d1E03-2-0472 | 838.207 | OEt | A02 | al | B05 | b1 | C14 | c2 | D02 | dl | E04 |
| 2-2-d1E03-2-0473 | 838.312 | OEt | A02 | al | B05 | b1 | C19 | c1 | D01 | dl | E04 |
| 2-2-d1E03-2-0474 | 839.212 | OEt | A02 | al | B04 | b4 | C01 | c2 | D02 | dl | E04 |
| 2-2-d1E03-2-0475 | 840.193 | OEt | A01 | al | B05 | b4 | C05 | c2 | D02 | dl | E04 |
| 2-2-d1E03-2-0476 | 841.152 | OEt | A02 | al | B05 | b3 | C10 | c2 | D02 | dl | E04 |
| 2-2-d1E03-2-0477 | 842.206 | OEt | A02 | al | B05 | b2 | C02 | c2 | D02 | dl | E04 |
| 2-2-d1E03-2-0478 | 842.206 | OEt | A02 | al | B05 | b2 | C03 | c2 | D02 | dl | E04 |
| 2-2-d1E03-2-0479 | 843.164 | OEt | A02 | al | B04 | b1 | C18 | c2 | D02 | dl | E04 |
| 2-2-d1E03-2-0480 | 843.201 | OEt | A02 | al | B05 | b2 | C04 | c2 | D02 | dl | E04 |
| 2-2-d1E03-2-0481 | 843.201 | OEt | A02 | al | B05 | b2 | C06 | c2 | D02 | dl | E04 |
| 2-2-d1E03-2-0482 | 843.201 | OEt | A02 | al | B05 | b2 | C07 | c2 | D02 | dl | E04 |
| 2-2-d1E03-2-0483 | 847.232 | OEt | A02 | al | B05 | b1 | C15 | c2 | D02 | dl | E04 |

(continued)

| [Table 9-7-4] Compound that may be contained in mixture 2-2-dlE03-2 | | | | | | | | | | | |
|---|---|---|---|---|---|---|---|---|---|---|---|
| ID | Exact Mass | | A | a | B | b | C | c | D | d | E |
| 2-2-d1E03-2-0484 | 848.180 | OEt | A01 | al | B05 | b3 | C11 | c2 | D02 | dl | E04 |
| 2-2-d1E03-2-0485 | 848.227 | OEt | A02 | al | B05 | b1 | C16 | c2 | D02 | dl | E04 |
| 2-2-d1E03-2-0486 | 848.303 | OEt | A02 | al | B02 | b1 | C19 | c2 | D02 | dl | E04 |
| 2-2-d1E03-2-0487 | 849.223 | OEt | A02 | al | B05 | b1 | C17 | c2 | D02 | dl | E04 |
| 2-2-d1E03-2-0488 | 849.232 | OEt | A02 | al | B04 | b4 | C02 | c2 | D02 | dl | E04 |
| 2-2-d1E03-2-0489 | 850.232 | OEt | A02 | al | B05 | b4 | C01 | c2 | D02 | dl | E04 |
| 2-2-d1E03-2-0490 | 852.278 | OEt | A02 | al | B03 | b1 | C19 | c2 | D02 | dl | E04 |
| 2-2-d1E03-2-0491 | 854.184 | OEt | A02 | al | B05 | b1 | C18 | c2 | D02 | dl | E04 |
| 2-2-d1E03-2-0492 | 855.189 | OEt | A02 | al | B04 | b4 | C05 | c2 | D02 | dl | E04 |
| 2-2-d1E03-2-0493 | 860.253 | OEt | A02 | al | B05 | b4 | C02 | c2 | D02 | dl | E04 |
| 2-2-d1E03-2-0494 | 863.175 | OEt | A02 | al | B04 | b3 | C11 | c2 | D02 | dl | E04 |
| 2-2-d1E03-2-0495 | 865.239 | OEt | A01 | al | B04 | b1 | C19 | c2 | D02 | dl | E04 |
| 2-2-d1E03-2-0496 | 866.209 | OEt | A02 | al | B05 | b4 | COS | c2 | D02 | dl | E04 |
| 2-2-d1E03-2-0497 | 874.195 | OEt | A02 | al | B05 | b3 | C11 | c2 | D02 | dl | E04 |
| 2-2-d1E03-2-0498 | 876.259 | OEt | A01 | al | B05 | b1 | C19 | c2 | D02 | dl | E04 |
| 2-2-d1E03-2-0499 | 891.254 | OEt | A02 | al | B04 | b1 | C19 | c2 | D02 | dl | E04 |
| 2-2-d1E03-2-0500 | 902.274 | OEt | A02 | al | B05 | b1 | C19 | c2 | D02 | dl | E04 |

[Table 502]

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0001 | 763.304 | OEt | A01 | al | B01 | b1 | C01 | c1 | D01 | dl | E04 |
| 2-2-d1E04-2-0002 | 767.372 | OEt | A01 | al | B01 | b4 | C12 | c1 | D01 | dl | E04 |
| 2-2-d1E04-2-0003 | 769.351 | OEt | A01 | al | B01 | b4 | C13 | c1 | D01 | dl | E04 |
| 2-2-d1E04-2-0004 | 773.325 | OEt | A01 | al | B01 | b1 | C02 | c1 | D01 | dl | E04 |
| 2-2-d1E04-2-0005 | 773.325 | OEt | A01 | al | B01 | b1 | C03 | c1 | D01 | dl | E04 |
| 2-2-d1E04-2-0006 | 774.320 | OEt | A01 | al | B01 | b1 | C04 | c1 | D01 | dl | E04 |
| 2-2-d1E04-2-0007 | 777.320 | OEt | A01 | al | B02 | b1 | C01 | c1 | D01 | dl | E04 |
| 2-2-d1E04-2-0008 | 779.281 | OEt | A01 | al | B01 | b1 | C05 | c1 | D01 | dl | E04 |
| 2-2-d1E04-2-0009 | 780.294 | OEt | A01 | al | B01 | b3 | C08 | c1 | D01 | dl | E04 |
| 2-2-dlE04-2-0010 | 781.295 | OEt | A01 | al | B03 | b1 | C01 | c1 | D01 | dl | E04 |
| 2-2-d1E04-2-0011 | 781.387 | OEt | A01 | al | B02 | b4 | C12 | c1 | D01 | dl | E04 |
| 2-2-dlE04-2-0012 | 783.367 | OEt | A01 | al | B02 | b4 | C13 | c1 | D01 | dl | E04 |
| 2-2-d1E04-2-0013 | 785.362 | OEt | A01 | al | B03 | b4 | C12 | c1 | D01 | dl | E04 |
| 2-2-d1E04-2-0014 | 787.340 | OEt | A01 | al | B02 | b1 | C02 | c1 | D01 | dl | E04 |
| 2-2-d1E04-2-0015 | 787.340 | OEt | A01 | al | B02 | b1 | C03 | c1 | D01 | dl | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0016 | 787.341 | OEt | A01 | al | B03 | b4 | C13 | c1 | D01 | dl | E04 |
| 2-2-d1E04-2-0017 | 788.336 | OEt | A01 | al | B02 | b1 | C04 | c1 | D01 | dl | E04 |
| 2-2-dlE04-2-0018 | 789.320 | OEt | A02 | al | B01 | b1 | C01 | c1 | D01 | dl | E04 |
| 2-2-dlE04-2-0019 | 791.310 | OEt | A01 | al | B01 | b3 | C09 | c1 | D01 | dl | E04 |
| 2-2-d1E04-2-0020 | 791.315 | OEt | A01 | al | B03 | b1 | C02 | c1 | D01 | dl | E04 |
| 2-2-d1E04-2-0021 | 791.315 | OEt | A01 | al | B03 | b1 | C03 | c1 | D01 | dl | E04 |
| 2-2-d1E04-2-0022 | 792.311 | OEt | A01 | al | B03 | b1 | C04 | c1 | D01 | dl | E04 |
| 2-2-d1E04-2-0023 | 793.297 | OEt | A01 | al | B02 | b1 | C05 | c1 | D01 | dl | E04 |
| 2-2-dlE04-2-0024 | 793.326 | OEt | A01 | al | B01 | b1 | C14 | c1 | D01 | dl | E04 |
| 2-2-d1E04-2-0025 | 793.387 | OEt | A02 | al | B01 | b4 | C12 | c1 | D01 | dl | E04 |
| 2-2-d1E04-2-0026 | 794.310 | OEt | A01 | al | B02 | b3 | C08 | c1 | D01 | dl | E04 |
| 2-2-dlE04-2-0027 | 795.367 | OEt | A02 | al | B01 | b4 | C13 | c1 | D01 | dl | E04 |
| 2-2-dlE04-2-0028 | 796.271 | OEt | A01 | al | B01 | b3 | C10 | c1 | D01 | dl | E04 |
| 2-2-d1E04-2-0029 | 797.272 | OEt | A01 | al | B03 | b1 | C05 | c1 | D01 | dl | E04 |
| 2-2-d1E04-2-0030 | 797.325 | OEt | A01 | al | B01 | b2 | C02 | c1 | D01 | dl | E04 |
| 2-2-d1E04-2-0031 | 797.325 | OEt | A01 | al | B01 | b2 | C03 | c1 | D01 | dl | E04 |
| 2-2-d1E04-2-0032 | 798.285 | OEt | A01 | al | B03 | b3 | C08 | c1 | D01 | dl | E04 |
| 2-2-d1E04-2-0033 | 798.320 | OEt | A01 | al | B01 | b2 | C04 | c1 | D01 | dl | E04 |
| 2-2-d1E04-2-0034 | 798.320 | OEt | A01 | al | B01 | b2 | C06 | c1 | D01 | dl | E04 |
| 2-2-d1E04-2-0035 | 798.320 | OEt | A01 | al | B01 | b2 | C07 | c1 | D01 | dl | E04 |
| 2-2-dlE04-2-0036 | 799.340 | OEt | A02 | al | B01 | b1 | C02 | c1 | D01 | dl | E04 |
| 2-2-d1E04-2-0037 | 799.340 | OEt | A02 | al | B01 | b1 | C03 | c1 | D01 | dl | E04 |
| 2-2-d1E04-2-0038 | 800.336 | OEt | A02 | al | B01 | b1 | C04 | c1 | D01 | dl | E04 |
| 2-2-d1E04-2-0039 | 802.351 | OEt | A01 | al | B01 | b1 | C15 | c1 | D01 | dl | E04 |
| 2-2-d1E04-2-0040 | 803.335 | OEt | A02 | al | B02 | b1 | C01 | c1 | D01 | dl | E04 |
| 2-2-d1E04-2-0041 | 803.347 | OEt | A01 | al | B01 | b1 | C16 | c1 | D01 | dl | E04 |
| 2-2-dlE04-2-0042 | 804.342 | OEt | A01 | al | B01 | b1 | C17 | c1 | D01 | dl | E04 |
| 2-2-dlE04-2-0043 | 805.297 | OEt | A02 | al | B01 | b1 | C05 | c1 | D01 | dl | E04 |
| 2-2-dlE04-2-0044 | 805.326 | OEt | A01 | al | B02 | b3 | C09 | c1 | D01 | dl | E04 |
| 2-2-dlE04-2-0045 | 805.351 | OEt | A01 | al | B01 | b4 | C01 | c1 | D01 | dl | E04 |
| 2-2-d1E04-2-0046 | 806.310 | OEt | A02 | al | B01 | b3 | C08 | c1 | D01 | dl | E04 |
| 2-2-d1E04-2-0047 | 807.310 | OEt | A02 | al | B03 | b1 | C01 | c1 | D01 | d1 | E04 |
| 2-2-d1E04-2-0048 | 807.341 | OEt | A01 | al | B02 | b1 | C14 | c1 | D01 | dl | E04 |
| 2-2-d1E04-2-0049 | 807.403 | OEt | A02 | al | B02 | b4 | C12 | c1 | D01 | dl | E04 |
| 2-2-d1E04-2-0050 | 809.301 | OEt | A01 | al | B03 | b3 | C09 | c1 | D01 | dl | E04 |
| 2-2-d1E04-2-0051 | 809.303 | OEt | A01 | al | B01 | b1 | C18 | c1 | D01 | dl | E04 |
| 2-2-d1E04-2-0052 | 809.382 | OEt | A02 | al | B02 | b4 | C13 | c1 | D01 | dl | E04 |
| 2-2-d1E04-2-0053 | 810.287 | OEt | A01 | al | B02 | b3 | C10 | c1 | D01 | d1 | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0054 | 811.316 | OEt | A01 | al | B03 | b1 | C14 | c1 | D01 | d1 | E04 |
| 2-2-d1E04-2-0055 | 811.340 | OEt | A01 | al | B02 | b2 | C02 | c1 | D01 | dl | E04 |
| 2-2-d1E04-2-0056 | 811.340 | OEt | A01 | al | B02 | b2 | C03 | c1 | D01 | d1 | E04 |
| 2-2-d1E04-2-0057 | 811.378 | OEt | A02 | al | B03 | b4 | C12 | c1 | D01 | d1 | E04 |
| 2-2-d1E04-2-0058 | 812.336 | OEt | A01 | al | B02 | b2 | C04 | c1 | D01 | d1 | E04 |
| 2-2-d1E04-2-0059 | 812.336 | OEt | A01 | al | B02 | b2 | C06 | c1 | D01 | d1 | E04 |
| 2-2-d1E04-2-0060 | 812.336 | OEt | A01 | al | B02 | b2 | C07 | c1 | D01 | d1 | E04 |
| 2-2-d1E04-2-0061 | 813.356 | OEt | A02 | al | B02 | b1 | C02 | c1 | D01 | d1 | E04 |
| 2-2-d1E04-2-0062 | 813.356 | OEt | A02 | al | B02 | b1 | C03 | c1 | D01 | d1 | E04 |
| 2-2-d1E04-2-0063 | 813.357 | OEt | A02 | al | B03 | b4 | C13 | c1 | D01 | d1 | E04 |
| 2-2-d1E04-2-0064 | 814.262 | OEt | A01 | al | B03 | b3 | C10 | c1 | D01 | dl | E04 |
| 2-2-d1E04-2-0065 | 814.351 | OEt | A02 | al | B02 | b1 | C04 | c1 | D01 | d1 | E04 |
| 2-2-d1E04-2-0066 | 815.315 | OEt | A01 | al | B03 | b2 | C02 | c1 | D01 | d1 | E04 |
| 2-2-d1E04-2-0067 | 815.315 | OEt | A01 | al | B03 | b2 | C03 | c1 | D01 | d1 | E04 |
| 2-2-d1E04-2-0068 | 815.372 | OEt | A01 | al | B01 | b4 | C02 | c1 | D01 | d1 | E04 |
| 2-2-d1E04-2-0069 | 816.311 | OEt | A01 | al | B03 | b2 | C04 | c1 | D01 | d1 | E04 |
| 2-2-d1E04-2-0070 | 816.311 | OEt | A01 | al | B03 | b2 | C06 | c1 | D01 | d1 | E04 |
| 2-2-d1E04-2-0071 | 816.311 | OEt | A01 | al | B03 | b2 | C07 | c1 | D01 | d1 | E04 |
| 2-2-d1E04-2-0072 | 816.367 | OEt | A01 | al | B02 | b1 | C15 | c1 | D01 | d1 | E04 |
| 2-2-d1E04-2-0073 | 817.326 | OEt | A02 | al | B01 | b3 | C09 | c1 | D01 | dl | E04 |
| 2-2-d1E04-2-0074 | 817.331 | OEt | A02 | al | B03 | b1 | C02 | c1 | D01 | d1 | E04 |
| 2-2-d1E04-2-0075 | 817.331 | OEt | A02 | al | B03 | b1 | C03 | c1 | D01 | d1 | E04 |
| 2-2-d1E04-2-0076 | 817.362 | OEt | A01 | al | B02 | b1 | C16 | c1 | D01 | d1 | E04 |
| 2-2-d1E04-2-0077 | 818.326 | OEt | A02 | al | B03 | b1 | C04 | c1 | D01 | d1 | E04 |
| 2-2-d1E04-2-0078 | 818.357 | OEt | A01 | al | B02 | b1 | C17 | c1 | D01 | d1 | E04 |
| 2-2-d1E04-2-0079 | 819.312 | OEt | A02 | al | B02 | b1 | C05 | c1 | D01 | d1 | E04 |
| 2-2-d1E04-2-0080 | 819.341 | OEt | A02 | al | B01 | b1 | C14 | c1 | D01 | d1 | E04 |
| 2-2-d1E04-2-0081 | 819.367 | OEt | A01 | al | B02 | b4 | C01 | c1 | D01 | d1 | E04 |
| 2-2-d1E04-2-0082 | 820.271 | OEt | A01 | al | B04 | b1 | C01 | c1 | D01 | dl | E04 |
| 2-2-d1E04-2-0083 | 820.325 | OEt | A02 | al | B02 | b3 | C08 | c1 | D01 | d1 | E04 |
| 2-2-d1E04-2-0084 | 820.342 | OEt | A01 | al | B03 | b1 | C15 | c1 | D01 | d1 | E04 |
| 2-2-d1E04-2-0085 | 821.328 | OEt | A01 | al | B01 | b4 | C05 | c1 | D01 | d1 | E04 |
| 2-2-d1E04-2-0086 | 821.337 | OEt | A01 | al | B03 | b1 | C16 | c1 | D01 | d1 | E04 |
| 2-2-d1E04-2-0087 | 822.287 | OEt | A02 | al | B01 | b3 | C10 | c1 | D01 | d1 | E04 |
| 2-2-d1E04-2-0088 | 822.332 | OEt | A01 | al | B03 | b1 | C17 | c1 | D01 | d1 | E04 |
| 2-2-d1E04-2-0089 | 823.287 | OEt | A02 | al | B03 | b1 | COS | c1 | D01 | d1 | E04 |
| 2-2-d1E04-2-0090 | 823.319 | OEt | A01 | al | B02 | b1 | C18 | c1 | D01 | d1 | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0091 | 823.340 | OEt | A02 | al | B01 | b2 | C02 | c1 | D01 | dl | E04 |
| 2-2-d1E04-2-0092 | 823.340 | OEt | A02 | al | B01 | b2 | C03 | c1 | D01 | dl | E04 |
| 2-2-d1E04-2-0093 | 823.341 | OEt | A01 | al | B03 | b4 | C01 | c1 | D01 | dl | E04 |
| 2-2-d1E04-2-0094 | 824.300 | OEt | A02 | al | B03 | b3 | C08 | c1 | D01 | d1 | E04 |
| 2-2-d1E04-2-0095 | 824.336 | OEt | A02 | al | B01 | b2 | C04 | c1 | D01 | dl | E04 |
| 2-2-d1E04-2-0096 | 824.336 | OEt | A02 | al | B01 | b2 | C06 | c1 | D01 | dl | E04 |
| 2-2-d1E04-2-0097 | 824.336 | OEt | A02 | al | B01 | b2 | C07 | c1 | D01 | dl | E04 |
| 2-2-d1E04-2-0098 | 824.339 | OEt | A01 | al | B04 | b4 | C12 | c1 | D01 | dl | E04 |
| 2-2-d1E04-2-0099 | 826.318 | OEt | A01 | al | B04 | b4 | C13 | c1 | D01 | dl | E04 |
| 2-2-dlE04-2-0100 | 827.266 | OEt | A01 | al | B01 | b1 | C01 | c2 | D02 | d1 | E04 |
| 2-2-d1E04-2-0101 | 827.294 | OEt | A01 | al | B03 | b1 | C18 | c1 | D01 | d1 | E04 |
| 2-2-d1E04-2-0102 | 828.367 | OEt | A02 | al | B01 | b1 | C15 | c1 | D01 | dl | E04 |
| 2-2-d1E04-2-0103 | 829.315 | OEt | A01 | al | B01 | b3 | C11 | c1 | D01 | d1 | E04 |
| 2-2-d1E04-2-0104 | 829.362 | OEt | A02 | al | B01 | b1 | C16 | c1 | D01 | d1 | E04 |
| 2-2-d1E04-2-0105 | 829.387 | OEt | A01 | al | B02 | b4 | C02 | c1 | D01 | d1 | E04 |
| 2-2-d1E04-2-0106 | 830.292 | OEt | A01 | al | B04 | b1 | C02 | c1 | D01 | d1 | E04 |
| 2-2-d1E04-2-0107 | 830.292 | OEt | A01 | al | B04 | b1 | C03 | c1 | D01 | d1 | E04 |
| 2-2-d1E04-2-0108 | 830.357 | OEt | A02 | al | B01 | b1 | C17 | c1 | D01 | d1 | E04 |
| 2-2-d1E04-2-0109 | 831.287 | OEt | A01 | al | B04 | b1 | C04 | c1 | D01 | d1 | E04 |
| 2-2-d1E04-2-0110 | 831.291 | OEt | A01 | al | B05 | b1 | C01 | c1 | D01 | d1 | E04 |
| 2-2-d1E04-2-0111 | 831.334 | OEt | A01 | al | B01 | b4 | C12 | c2 | D02 | dl | E04 |
| 2-2-d1E04-2-0112 | 831.341 | OEt | A02 | al | B02 | b3 | C09 | c1 | D01 | d1 | E04 |
| 2-2-d1E04-2-0113 | 831.367 | OEt | A02 | al | B01 | b4 | C01 | c1 | D01 | d1 | E04 |
| 2-2-d1E04-2-0114 | 833.313 | OEt | A01 | al | B01 | b4 | C13 | c2 | D02 | d1 | E04 |
| 2-2-d1E04-2-0115 | 833.357 | OEt | A02 | al | B02 | b1 | C14 | c1 | D01 | d1 | E04 |
| 2-2-d1E04-2-0116 | 833.362 | OEt | A01 | al | B03 | b4 | C02 | c1 | D01 | d1 | E04 |
| 2-2-d1E04-2-0117 | 835.316 | OEt | A02 | al | B03 | b3 | C09 | c1 | D01 | d1 | E04 |
| 2-2-d1E04-2-0118 | 835.319 | OEt | A02 | al | B01 | b1 | C18 | c1 | D01 | d1 | E04 |
| 2-2-d1E04-2-0119 | 835.344 | OEt | A01 | al | B02 | b4 | C05 | c1 | D01 | d1 | E04 |
| 2-2-d1E04-2-0120 | 835.359 | OEt | A01 | al | B05 | b4 | C12 | c1 | D01 | dl | E04 |
| 2-2-d1E04-2-0121 | 836.248 | OEt | A01 | al | B04 | b1 | C05 | c1 | D01 | d1 | E04 |
| 2-2-dlE04-2-0122 | 836.303 | OEt | A02 | al | B02 | b3 | C10 | c1 | D01 | d1 | E04 |
| 2-2-d1E04-2-0123 | 837.261 | OEt | A01 | al | B04 | b3 | C08 | c1 | D01 | d1 | E04 |
| 2-2-dlE04-2-0124 | 837.287 | OEt | A01 | al | B01 | b1 | C02 | c2 | D02 | d1 | E04 |
| 2-2-dlE04-2-0125 | 837.287 | OEt | A01 | al | B01 | b1 | C03 | c2 | D02 | d1 | E04 |
| 2-2-d1E04-2-0126 | 837.332 | OEt | A02 | al | B03 | b1 | C14 | c1 | D01 | d1 | E04 |
| 2-2-d1E04-2-0127 | 837.338 | OEt | A01 | al | B05 | b4 | C13 | c1 | D01 | d1 | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0128 | 837.356 | OEt | A02 | al | B02 | b2 | C02 | c1 | D01 | d1 | E04 |
| 2-2-d1E04-2-0129 | 837.356 | OEt | A02 | al | B02 | b2 | C03 | c1 | D01 | dl | E04 |
| 2-2-dlE04-2-0130 | 838.282 | OEt | A01 | al | B01 | b1 | C04 | c2 | D02 | d1 | E04 |
| 2-2-d1E04-2-0131 | 838.351 | OEt | A02 | al | B02 | b2 | C04 | c1 | D01 | d1 | E04 |
| 2-2-d1E04-2-0132 | 838.351 | OEt | A02 | al | B02 | b2 | C06 | c1 | D01 | d1 | E04 |
| 2-2-d1E04-2-0133 | 838.351 | OEt | A02 | al | B02 | b2 | C07 | c1 | D01 | d1 | E04 |
| 2-2-d1E04-2-0134 | 839.319 | OEt | A01 | al | B03 | b4 | C05 | c1 | D01 | d1 | E04 |
| 2-2-d1E04-2-0135 | 840.278 | OEt | A02 | al | B03 | b3 | C10 | c1 | D01 | d1 | E04 |
| 2-2-d1E04-2-0136 | 841.282 | OEt | A01 | al | B02 | b1 | C01 | c2 | D02 | d1 | E04 |
| 2-2-d1E04-2-0137 | 841.312 | OEt | A01 | al | B05 | b1 | C02 | c1 | D01 | d1 | E04 |
| 2-2-d1E04-2-0138 | 841.312 | OEt | A01 | al | B05 | b1 | C03 | c1 | D01 | dl | E04 |
| 2-2-d1E04-2-0139 | 841.331 | OEt | A02 | al | B03 | b2 | C02 | c1 | D01 | dl | E04 |
| 2-2-d1E04-2-0140 | 841.331 | OEt | A02 | al | B03 | b2 | C03 | c1 | D01 | dl | E04 |
| 2-2-d1E04-2-0141 | 841.387 | OEt | A02 | al | B01 | b4 | C02 | c1 | D01 | d1 | E04 |
| 2-2-d1E04-2-0142 | 842.307 | OEt | A01 | al | B05 | b1 | C04 | c1 | D01 | dl | E04 |
| 2-2-d1E04-2-0143 | 842.326 | OEt | A02 | al | B03 | b2 | C04 | c1 | D01 | dl | E04 |
| 2-2-d1E04-2-0144 | 842.326 | OEt | A02 | al | B03 | b2 | C06 | c1 | D01 | dl | E04 |
| 2-2-d1E04-2-0145 | 842.326 | OEt | A02 | al | B03 | b2 | C07 | c1 | D01 | d1 | E04 |
| 2-2-d1E04-2-0146 | 842.383 | OEt | A02 | al | B02 | b1 | C15 | c1 | D01 | d1 | E04 |
| 2-2-d1E04-2-0147 | 843.243 | OEt | A01 | al | B01 | b1 | C05 | c2 | D02 | d1 | E04 |
| 2-2-d1E04-2-0148 | 843.330 | OEt | A01 | al | B02 | b3 | C11 | c1 | D01 | d1 | E04 |
| 2-2-d1E04-2-0149 | 843.378 | OEt | A02 | al | B02 | b1 | C16 | c1 | D01 | d1 | E04 |
| 2-2-d1E04-2-0150 | 844.256 | OEt | A01 | al | B01 | b3 | C08 | c2 | D02 | d1 | E04 |
| 2-2-d1E04-2-0151 | 844.373 | OEt | A02 | al | B02 | b1 | C17 | c1 | D01 | d1 | E04 |
| 2-2-d1E04-2-0152 | 845.256 | OEt | A01 | al | B03 | b1 | C01 | c2 | D02 | d1 | E04 |
| 2-2-d1E04-2-0153 | 845.349 | OEt | A01 | al | B02 | b4 | C12 | c2 | D02 | d1 | E04 |
| 2-2-d1E04-2-0154 | 845.382 | OEt | A02 | al | B02 | b4 | C01 | c1 | D01 | d1 | E04 |
| 2-2-d1E04-2-0155 | 846.287 | OEt | A02 | al | B04 | b1 | C01 | c1 | D01 | d1 | E04 |
| 2-2-dlE04-2-0156 | 846.357 | OEt | A02 | al | B03 | b1 | C15 | c1 | D01 | d1 | E04 |
| 2-2-d1E04-2-0157 | 847.269 | OEt | A01 | al | B05 | b1 | COS | c1 | D01 | d1 | E04 |
| 2-2-d1E04-2-0158 | 847.305 | OEt | A01 | al | B03 | b3 | C11 | c1 | D01 | d1 | E04 |
| 2-2-d1E04-2-0159 | 847.328 | OEt | A01 | al | B02 | b4 | C13 | c2 | D02 | d1 | E04 |
| 2-2-d1E04-2-0160 | 847.344 | OEt | A02 | al | B01 | b4 | C05 | c1 | D01 | d1 | E04 |
| 2-2-d1E04-2-0161 | 847.353 | OEt | A02 | al | B03 | b1 | C16 | c1 | D01 | d1 | E04 |
| 2-2-d1E04-2-0162 | 848.277 | OEt | A01 | al | B04 | b3 | C09 | c1 | D01 | d1 | E04 |
| 2-2-d1E04-2-0163 | 848.282 | OEt | A01 | al | B05 | b3 | C08 | c1 | D01 | d1 | E04 |
| 2-2-dlE04-2-0164 | 848.348 | OEt | A02 | al | B03 | b1 | C17 | c1 | D01 | d1 | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0165 | 849.324 | OEt | A01 | al | B03 | b4 | C12 | c2 | D02 | d1 | E04 |
| 2-2-dlE04-2-0166 | 849.334 | OEt | A02 | al | B02 | b1 | C18 | c1 | D01 | d1 | E04 |
| 2-2-d1E04-2-0167 | 849.357 | OEt | A02 | al | B03 | b4 | C01 | c1 | D01 | dl | E04 |
| 2-2-d1E04-2-0168 | 850.293 | OEt | A01 | al | B04 | b1 | C14 | c1 | D01 | d1 | E04 |
| 2-2-d1E04-2-0169 | 850.355 | OEt | A02 | al | B04 | b4 | C12 | c1 | D01 | d1 | E04 |
| 2-2-d1E04-2-0170 | 851.302 | OEt | A01 | al | B02 | b1 | C02 | c2 | D02 | d1 | E04 |
| 2-2-d1E04-2-0171 | 851.302 | OEt | A01 | al | B02 | b1 | C03 | c2 | D02 | d1 | E04 |
| 2-2-d1E04-2-0172 | 851.303 | OEt | A01 | al | B03 | b4 | C13 | c2 | D02 | d1 | E04 |
| 2-2-d1E04-2-0173 | 852.298 | OEt | A01 | al | B02 | b1 | C04 | c2 | D02 | d1 | E04 |
| 2-2-d1E04-2-0174 | 852.334 | OEt | A02 | al | B04 | b4 | C13 | c1 | D01 | d1 | E04 |
| 2-2-d1E04-2-0175 | 853.239 | OEt | A01 | al | B04 | b3 | C10 | c1 | D01 | d1 | E04 |
| 2-2-d1E04-2-0176 | 853.282 | OEt | A02 | al | B01 | b1 | C01 | c2 | D02 | dl | E04 |
| 2-2-d1E04-2-0177 | 853.309 | OEt | A02 | al | B03 | b1 | C18 | c1 | D01 | d1 | E04 |
| 2-2-d1E04-2-0178 | 854.292 | OEt | A01 | al | B04 | b2 | C02 | c1 | D01 | d1 | E04 |
| 2-2-d1E04-2-0179 | 854.292 | OEt | A01 | al | B04 | b2 | C03 | c1 | D01 | d1 | E04 |
| 2-2-d1E04-2-0180 | 855.272 | OEt | A01 | al | B01 | b3 | C09 | c2 | D02 | d1 | E04 |
| 2-2-d1E04-2-0181 | 855.277 | OEt | A01 | al | B03 | b1 | C02 | c2 | D02 | d1 | E04 |
| 2-2-dlE04-2-0182 | 855.277 | OEt | A01 | al | B03 | b1 | C03 | c2 | D02 | d1 | E04 |
| 2-2-d1E04-2-0183 | 855.287 | OEt | A01 | al | B04 | b2 | C04 | c1 | D01 | d1 | E04 |
| 2-2-dlE04-2-0184 | 855.287 | OEt | A01 | al | B04 | b2 | C06 | c1 | D01 | d1 | E04 |
| 2-2-d1E04-2-0185 | 855.287 | OEt | A01 | al | B04 | b2 | C07 | c1 | D01 | dl | E04 |
| 2-2-d1E04-2-0186 | 855.330 | OEt | A02 | al | B01 | b3 | C11 | c1 | D01 | dl | E04 |
| 2-2-dlE04-2-0187 | 855.403 | OEt | A02 | al | B02 | b4 | C02 | c1 | D01 | dl | E04 |
| 2-2-dlE04-2-0188 | 856.272 | OEt | A01 | al | B03 | b1 | C04 | c2 | D02 | dl | E04 |
| 2-2-dlE04-2-0189 | 856.308 | OEt | A02 | al | B04 | b1 | C02 | c1 | D01 | dl | E04 |
| 2-2-dlE04-2-0190 | 856.308 | OEt | A02 | al | B04 | b1 | C03 | c1 | D01 | dl | E04 |
| 2-2-dlE04-2-0191 | 857.259 | OEt | A01 | al | B02 | b1 | C05 | c2 | D02 | dl | E04 |
| 2-2-dlE04-2-0192 | 857.288 | OEt | A01 | al | B01 | b1 | C14 | c2 | D02 | dl | E04 |
| 2-2-d1E04-2-0193 | 857.303 | OEt | A02 | al | B04 | b1 | C04 | c1 | D01 | dl | E04 |
| 2-2-dlE04-2-0194 | 857.307 | OEt | A02 | al | B05 | b1 | C01 | c1 | D01 | dl | E04 |
| 2-2-d1E04-2-0195 | 857.349 | OEt | A02 | al | B01 | b4 | C12 | c2 | D02 | dl | E04 |
| 2-2-dlE04-2-0196 | 857.393 | OEt | A01 | al | B01 | b1 | C19 | c1 | D01 | dl | E04 |
| 2-2-dlE04-2-0197 | 858.272 | OEt | A01 | al | B02 | b3 | C08 | c2 | D02 | dl | E04 |
| 2-2-d1E04-2-0198 | 859.298 | OEt | A01 | al | B05 | b3 | C09 | c1 | D01 | dl | E04 |
| 2-2-dlE04-2-0199 | 859.319 | OEt | A01 | al | B04 | b1 | C15 | c1 | D01 | dl | E04 |
| 2-2-d1E04-2-0200 | 859.328 | OEt | A02 | al | B01 | b4 | C13 | c2 | D02 | dl | E04 |
| 2-2-d1E04-2-0201 | 859.378 | OEt | A02 | al | B03 | b4 | C02 | c1 | D01 | dl | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0202 | 860.233 | OEt | A01 | al | B01 | b3 | C10 | c2 | D02 | dl | E04 |
| 2-2-d1E04-2-0203 | 860.314 | OEt | A01 | al | B04 | b1 | C16 | c1 | D01 | dl | E04 |
| 2-2-d1E04-2-0204 | 861.234 | OEt | A01 | al | B03 | b1 | COS | c2 | D02 | dl | E04 |
| 2-2-d1E04-2-0205 | 861.287 | OEt | A01 | al | B01 | b2 | C02 | c2 | D02 | dl | E04 |
| 2-2-d1E04-2-0206 | 861.287 | OEt | A01 | al | B01 | b2 | C03 | c2 | D02 | dl | E04 |
| 2-2-d1E04-2-0207 | 861.309 | OEt | A01 | al | B04 | b1 | C17 | c1 | D01 | dl | E04 |
| 2-2-dlE04-2-0208 | 861.313 | OEt | A01 | al | B05 | b1 | C14 | c1 | D01 | dl | E04 |
| 2-2-d1E04-2-0209 | 861.359 | OEt | A02 | al | B02 | b4 | C05 | c1 | D01 | dl | E04 |
| 2-2-d1E04-2-0210 | 861.375 | OEt | A02 | al | B05 | b4 | C12 | c1 | D01 | dl | E04 |
| 2-2-d1E04-2-0211 | 862.247 | OEt | A01 | al | B03 | b3 | C08 | c2 | D02 | dl | E04 |
| 2-2-dlE04-2-0212 | 862.264 | OEt | A02 | al | B04 | b1 | C05 | c1 | D01 | dl | E04 |
| 2-2-d1E04-2-0213 | 862.282 | OEt | A01 | al | B01 | b2 | C04 | c2 | D02 | dl | E04 |
| 2-2-dlE04-2-0214 | 862.282 | OEt | A01 | al | B01 | b2 | C06 | c2 | D02 | dl | E04 |
| 2-2-d1E04-2-0215 | 862.282 | OEt | A01 | al | B01 | b2 | C07 | c2 | D02 | dl | E04 |
| 2-2-dlE04-2-0216 | 862.318 | OEt | A01 | al | B04 | b4 | C01 | c1 | D01 | dl | E04 |
| 2-2-d1E04-2-0217 | 863.277 | OEt | A02 | al | B04 | b3 | C08 | c1 | D01 | dl | E04 |
| 2-2-dlE04-2-0218 | 863.302 | OEt | A02 | al | B01 | b1 | C02 | c2 | D02 | dl | E04 |
| 2-2-dlE04-2-0219 | 863.302 | OEt | A02 | al | B01 | b1 | C03 | c2 | D02 | dl | E04 |
| 2-2-d1E04-2-0220 | 863.354 | OEt | A02 | al | B05 | b4 | C13 | c1 | D01 | dl | E04 |
| 2-2-dlE04-2-0221 | 864.259 | OEt | A01 | al | B05 | b3 | C10 | c1 | D01 | dl | E04 |
| 2-2-dlE04-2-0222 | 864.298 | OEt | A02 | al | B01 | b1 | C04 | c2 | D02 | dl | E04 |
| 2-2-dlE04-2-0223 | 865.312 | OEt | A01 | al | B05 | b2 | C02 | c1 | D01 | dl | E04 |
| 2-2-dlE04-2-0224 | 865.312 | OEt | A01 | al | B05 | b2 | C03 | c1 | D01 | dl | E04 |
| 2-2-d1E04-2-0225 | 865.334 | OEt | A02 | al | B03 | b4 | COS | c1 | D01 | dl | E04 |
| 2-2-d1E04-2-0226 | 866.270 | OEt | A01 | al | B04 | b1 | C18 | c1 | D01 | dl | E04 |
| 2-2-dlE04-2-0227 | 866.307 | OEt | A01 | al | B05 | b2 | C04 | c1 | D01 | dl | E04 |
| 2-2-dlE04-2-0228 | 866.307 | OEt | A01 | al | B05 | b2 | C06 | c1 | D01 | dl | E04 |
| 2-2-d1E04-2-0229 | 866.307 | OEt | A01 | al | B05 | b2 | C07 | c1 | D01 | dl | E04 |
| 2-2-d1E04-2-0230 | 866.313 | OEt | A01 | al | B01 | b1 | C15 | c2 | D02 | dl | E04 |
| 2-2-dlE04-2-0231 | 867.297 | OEt | A02 | al | B02 | b1 | C01 | c2 | D02 | dl | E04 |
| 2-2-d1E04-2-0232 | 867.308 | OEt | A01 | al | B01 | b1 | C16 | c2 | D02 | dl | E04 |
| 2-2-dlE04-2-0233 | 867.328 | OEt | A02 | al | B05 | b1 | C02 | c1 | D01 | dl | E04 |
| 2-2-dlE04-2-0234 | 867.328 | OEt | A02 | al | B05 | b1 | C03 | c1 | D01 | dl | E04 |
| 2-2-d1E04-2-0235 | 868.304 | OEt | A01 | al | B01 | b1 | C17 | c2 | D02 | dl | E04 |
| 2-2-d1E04-2-0236 | 868.323 | OEt | A02 | al | B05 | b1 | C04 | c1 | D01 | dl | E04 |
| 2-2-dlE04-2-0237 | 869.259 | OEt | A02 | al | B01 | b1 | C05 | c2 | D02 | dl | E04 |
| 2-2-dlE04-2-0238 | 869.288 | OEt | A01 | al | B02 | b3 | C09 | c2 | D02 | dl | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0239 | 869.313 | OEt | A01 | al | B01 | b4 | C01 | c2 | D02 | dl | E04 |
| 2-2-d1E04-2-0240 | 869.346 | OEt | A02 | al | B02 | b3 | C11 | c1 | D01 | dl | E04 |
| 2-2-d1E04-2-0241 | 870.272 | OEt | A02 | al | B01 | b3 | C08 | c2 | D02 | dl | E04 |
| 2-2-dlE04-2-0242 | 870.339 | OEt | A01 | al | B05 | b1 | C15 | c1 | D01 | dl | E04 |
| 2-2-d1E04-2-0243 | 871.272 | OEt | A02 | al | B03 | b1 | C01 | c2 | D02 | dl | E04 |
| 2-2-dlE04-2-0244 | 871.303 | OEt | A01 | al | B02 | b1 | C14 | c2 | D02 | dl | E04 |
| 2-2-d1E04-2-0245 | 871.334 | OEt | A01 | al | B05 | b1 | C16 | c1 | D01 | dl | E04 |
| 2-2-d1E04-2-0246 | 871.365 | OEt | A02 | al | B02 | b4 | C12 | c2 | D02 | dl | E04 |
| 2-2-d1E04-2-0247 | 871.409 | OEt | A01 | al | B02 | b1 | C19 | c1 | D01 | dl | E04 |
| 2-2-d1E04-2-0248 | 872.329 | OEt | A01 | al | B05 | b1 | C17 | c1 | D01 | dl | E04 |
| 2-2-d1E04-2-0249 | 872.339 | OEt | A01 | al | B04 | b4 | C02 | c1 | D01 | dl | E04 |
| 2-2-d1E04-2-0250 | 873.263 | OEt | A01 | al | B03 | b3 | C09 | c2 | D02 | dl | E04 |
| 2-2-d1E04-2-0251 | 873.265 | OEt | A01 | al | B01 | b1 | C18 | c2 | D02 | dl | E04 |
| 2-2-dlE04-2-0252 | 873.284 | OEt | A02 | al | B05 | b1 | C05 | c1 | D01 | dl | E04 |
| 2-2-dlE04-2-0253 | 873.321 | OEt | A02 | al | B03 | b3 | C11 | c1 | D01 | dl | E04 |
| 2-2-d1E04-2-0254 | 873.338 | OEt | A01 | al | B05 | b4 | C01 | c1 | D01 | dl | E04 |
| 2-2-d1E04-2-0255 | 873.344 | OEt | A02 | al | B02 | b4 | C13 | c2 | D02 | dl | E04 |
| 2-2-dl E04-2-0256 | 874.249 | OEt | A01 | al | B02 | b3 | C10 | c2 | D02 | dl | E04 |
| 2-2-d1E04-2-0257 | 874.293 | OEt | A02 | al | B04 | b3 | C09 | c1 | D01 | dl | E04 |
| 2-2-d1E04-2-0258 | 874.297 | OEt | A02 | al | B05 | b3 | C08 | c1 | D01 | dl | E04 |
| 2-2-d1E04-2-0259 | 875.278 | OEt | A01 | al | B03 | b1 | C14 | c2 | D02 | dl | E04 |
| 2-2-dlE04-2-0260 | 875.302 | OEt | A01 | al | B02 | b2 | C02 | c2 | D02 | dl | E04 |
| 2-2-d1E04-2-0261 | 875.302 | OEt | A01 | al | B02 | b2 | C03 | c2 | D02 | dl | E04 |
| 2-2-dlE04-2-0262 | 875.340 | OEt | A02 | al | B03 | b4 | C12 | c2 | D02 | dl | E04 |
| 2-2-dlE04-2-0263 | 875.384 | OEt | A01 | al | B03 | b1 | C19 | c1 | D01 | dl | E04 |
| 2-2-dlE04-2-0264 | 876.298 | OEt | A01 | al | B02 | b2 | C04 | c2 | D02 | dl | E04 |
| 2-2-d1E04-2-0265 | 876.298 | OEt | A01 | al | B02 | b2 | C06 | c2 | D02 | dl | E04 |
| 2-2-d1E04-2-0266 | 876.298 | OEt | A01 | al | B02 | b2 | C07 | c2 | D02 | dl | E04 |
| 2-2-d1E04-2-0267 | 876.309 | OEt | A02 | al | B04 | b1 | C14 | c1 | D01 | dl | E04 |
| 2-2-dlE04-2-0268 | 877.290 | OEt | A01 | al | B05 | b1 | C18 | c1 | D01 | dl | E04 |
| 2-2-dlE04-2-0269 | 877.318 | OEt | A02 | al | B02 | b1 | C02 | c2 | D02 | dl | E04 |
| 2-2-d1E04-2-0270 | 877.318 | OEt | A02 | al | B02 | b1 | C03 | c2 | D02 | dl | E04 |
| 2-2-d1E04-2-0271 | 877.319 | OEt | A02 | al | B03 | b4 | C13 | c2 | D02 | dl | E04 |
| 2-2-dlE04-2-0272 | 878.224 | OEt | A01 | al | B03 | b3 | C10 | c2 | D02 | dl | E04 |
| 2-2-d1E04-2-0273 | 878.295 | OEt | A01 | al | B04 | b4 | C05 | c1 | D01 | dl | E04 |
| 2-2-d1E04-2-0274 | 878.313 | OEt | A02 | al | B02 | b1 | C04 | c2 | D02 | dl | E04 |
| 2-2-d1E04-2-0275 | 879.254 | OEt | A02 | al | B04 | b3 | C10 | c1 | D01 | dl | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-dlE04-2-0276 | 879.277 | OEt | A01 | al | B03 | b2 | C02 | c2 | D02 | dl | E04 |
| 2-2-dlE04-2-0277 | 879.277 | OEt | A01 | al | B03 | b2 | C03 | c2 | D02 | dl | E04 |
| 2-2-dlE04-2-0278 | 879.334 | OEt | A01 | al | B01 | b4 | C02 | c2 | D02 | dl | E04 |
| 2-2-d1E04-2-0279 | 880.272 | OEt | A01 | al | B03 | b2 | C04 | c2 | D02 | dl | E04 |
| 2-2-dlE04-2-0280 | 880.272 | OEt | A01 | al | B03 | b2 | C06 | c2 | D02 | dl | E04 |
| 2-2-dlE04-2-0281 | 880.272 | OEt | A01 | al | B03 | b2 | C07 | c2 | D02 | dl | E04 |
| 2-2-dlE04-2-0282 | 880.308 | OEt | A02 | al | B04 | b2 | C02 | c1 | D01 | dl | E04 |
| 2-2-dlE04-2-0283 | 880.308 | OEt | A02 | al | B04 | b2 | C03 | c1 | D01 | dl | E04 |
| 2-2-dlE04-2-0284 | 880.329 | OEt | A01 | al | B02 | b1 | C15 | c2 | D02 | dl | E04 |
| 2-2-dlE04-2-0285 | 881.288 | OEt | A02 | al | B01 | b3 | C09 | c2 | D02 | dl | E04 |
| 2-2-d1E04-2-0286 | 881.293 | OEt | A02 | al | B03 | b1 | C02 | c2 | D02 | dl | E04 |
| 2-2-d1E04-2-0287 | 881.293 | OEt | A02 | al | B03 | b1 | C03 | c2 | D02 | dl | E04 |
| 2-2-d1E04-2-0288 | 881.303 | OEt | A02 | al | B04 | b2 | C04 | c1 | D01 | dl | E04 |
| 2-2-d1E04-2-0289 | 881.303 | OEt | A02 | al | B04 | b2 | C06 | c1 | D01 | dl | E04 |
| 2-2-d1E04-2-0290 | 881.303 | OEt | A02 | al | B04 | b2 | C07 | c1 | D01 | dl | E04 |
| 2-2-dlE04-2-0291 | 881.324 | OEt | A01 | al | B02 | b1 | C16 | c2 | D02 | dl | E04 |
| 2-2-dlE04-2-0292 | 882.288 | OEt | A02 | al | B03 | b1 | C04 | c2 | D02 | dl | E04 |
| 2-2-dlE04-2-0293 | 882.319 | OEt | A01 | al | B02 | b1 | C17 | c2 | D02 | dl | E04 |
| 2-2-dlE04-2-0294 | 883.274 | OEt | A02 | al | B02 | b1 | C05 | c2 | D02 | dl | E04 |
| 2-2-d1E04-2-0295 | 883.303 | OEt | A02 | al | B01 | b1 | C14 | c2 | D02 | dl | E04 |
| 2-2-d1E04-2-0296 | 883.328 | OEt | A01 | al | B02 | b4 | C01 | c2 | D02 | dl | E04 |
| 2-2-d1E04-2-0297 | 883.359 | OEt | A01 | al | B05 | b4 | C02 | c1 | D01 | dl | E04 |
| 2-2-dlE04-2-0298 | 883.409 | OEt | A02 | al | B01 | b1 | C19 | c1 | D01 | dl | E04 |
| 2-2-d1E04-2-0299 | 884.233 | OEt | A01 | al | B04 | b1 | C01 | c2 | D02 | dl | E04 |
| 2-2-dlE04-2-0300 | 884.287 | OEt | A02 | al | B02 | b3 | C08 | c2 | D02 | dl | E04 |
| 2-2-d1E04-2-0301 | 884.304 | OEt | A01 | al | B03 | b1 | C15 | c2 | D02 | dl | E04 |
| 2-2-d1E04-2-0302 | 885.290 | OEt | A01 | al | B01 | b4 | C05 | c2 | D02 | dl | E04 |
| 2-2-d1E04-2-0303 | 885.299 | OEt | A01 | al | B03 | b1 | C16 | c2 | D02 | dl | E04 |
| 2-2-d1E04-2-0304 | 885.313 | OEt | A02 | al | B05 | b3 | C09 | c1 | D01 | dl | E04 |
| 2-2-d1E04-2-0305 | 885.334 | OEt | A02 | al | B04 | b1 | C15 | c1 | D01 | dl | E04 |
| 2-2-d1E04-2-0306 | 886.249 | OEt | A02 | al | B01 | b3 | C10 | c2 | D02 | dl | E04 |
| 2-2-d1E04-2-0307 | 886.282 | OEt | A01 | al | B04 | b3 | C11 | c1 | D01 | dl | E04 |
| 2-2-d1E04-2-0308 | 886.294 | OEt | A01 | al | B03 | b1 | C17 | c2 | D02 | dl | E04 |
| 2-2-dlE04-2-0309 | 886.329 | OEt | A02 | al | B04 | b1 | C16 | c1 | D01 | dl | E04 |
| 2-2-dlE04-2-0310 | 887.249 | OEt | A02 | al | B03 | b1 | C05 | c2 | D02 | dl | E04 |
| 2-2-d1E04-2-0311 | 887.280 | OEt | A01 | al | B02 | b1 | C18 | c2 | D02 | dl | E04 |
| 2-2-d1E04-2-0312 | 887.302 | OEt | A02 | al | B01 | b2 | C02 | c2 | D02 | dl | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0313 | 887.302 | OEt | A02 | al | B01 | b2 | C03 | c2 | D02 | dl | E04 |
| 2-2-d1E04-2-0314 | 887.303 | OEt | A01 | al | B03 | b4 | C01 | c2 | D02 | dl | E04 |
| 2-2-d1E04-2-0315 | 887.325 | OEt | A02 | al | B04 | b1 | C17 | c1 | D01 | dl | E04 |
| 2-2-d1E04-2-0316 | 887.329 | OEt | A02 | al | B05 | b1 | C14 | c1 | D01 | dl | E04 |
| 2-2-d1E04-2-0317 | 888.262 | OEt | A02 | al | B03 | b3 | C08 | c2 | D02 | dl | E04 |
| 2-2-dlE04-2-0318 | 888.298 | OEt | A02 | al | B01 | b2 | C04 | c2 | D02 | dl | E04 |
| 2-2-dlE04-2-0319 | 888.298 | OEt | A02 | al | B01 | b2 | C06 | c2 | D02 | dl | E04 |
| 2-2-dlE04-2-0320 | 888.298 | OEt | A02 | al | B01 | b2 | C07 | c2 | D02 | dl | E04 |
| 2-2-d1E04-2-0321 | 888.301 | OEt | A01 | al | B04 | b4 | C12 | c2 | D02 | dl | E04 |
| 2-2-dlE04-2-0322 | 888.334 | OEt | A02 | al | B04 | b4 | C01 | c1 | D01 | dl | E04 |
| 2-2-dlE04-2-0323 | 889.315 | OEt | A01 | al | B05 | b4 | C05 | c1 | D01 | dl | E04 |
| 2-2-dlE04-2-0324 | 890.274 | OEt | A02 | al | B05 | b3 | C10 | c1 | D01 | dl | E04 |
| 2-2-d1E04-2-0325 | 890.280 | OEt | A01 | al | B04 | b4 | C13 | c2 | D02 | dl | E04 |
| 2-2-d1E04-2-0326 | 891.255 | OEt | A01 | al | B03 | b1 | C18 | c2 | D02 | dl | E04 |
| 2-2-d1E04-2-0327 | 891.328 | OEt | A02 | al | B05 | b2 | C02 | c1 | D01 | dl | E04 |
| 2-2-d1E04-2-0328 | 891.328 | OEt | A02 | al | B05 | b2 | C03 | c1 | D01 | d1 | E04 |
| 2-2-d1E04-2-0329 | 892.286 | OEt | A02 | al | B04 | b1 | C18 | c1 | D01 | d1 | E04 |
| 2-2-d1E04-2-0330 | 892.323 | OEt | A02 | al | B05 | b2 | C04 | c1 | D01 | d1 | E04 |
| 2-2-d1E04-2-0331 | 892.323 | OEt | A02 | al | B05 | b2 | C06 | c1 | D01 | d1 | E04 |
| 2-2-d1E04-2-0332 | 892.323 | OEt | A02 | a1 | B05 | b2 | C07 | c1 | D01 | d1 | E04 |
| 2-2-d1E04-2-0333 | 892.329 | OEt | A02 | al | B01 | b1 | C15 | c2 | D02 | dl | E04 |
| 2-2-d1E04-2-0334 | 893.276 | OEt | A01 | a1 | B01 | b3 | C11 | c2 | D02 | d1 | E04 |
| 2-2-d1E04-2-0335 | 893.324 | OEt | A02 | al | B01 | b1 | C16 | c2 | D02 | d1 | E04 |
| 2-2-d1E04-2-0336 | 893.349 | OEt | A01 | a1 | B02 | b4 | C02 | c2 | D02 | d1 | E04 |
| 2-2-d1E04-2-0337 | 894.254 | OEt | A01 | al | B04 | b1 | C02 | c2 | D02 | dl | E04 |
| 2-2-d1E04-2-0338 | 894.254 | OEt | A01 | al | B04 | b1 | C03 | c2 | D02 | dl | E04 |
| 2-2-d1E04-2-0339 | 894.319 | OEt | A02 | al | B01 | b1 | C17 | c2 | D02 | dl | E04 |
| 2-2-d1E04-2-0340 | 895.249 | OEt | A01 | al | B04 | b1 | C04 | c2 | D02 | dl | E04 |
| 2-2-d1E04-2-0341 | 895.253 | OEt | A01 | al | B05 | b1 | C01 | c2 | D02 | dl | E04 |
| 2-2-d1E04-2-0342 | 895.303 | OEt | A02 | al | B02 | b3 | C09 | c2 | D02 | dl | E04 |
| 2-2-d1E04-2-0343 | 895.328 | OEt | A02 | al | B01 | b4 | C01 | c2 | D02 | dl | E04 |
| 2-2-d1E04-2-0344 | 896.354 | OEt | A02 | al | B05 | b1 | C15 | c1 | D01 | dl | E04 |
| 2-2-d1E04-2-0345 | 897.302 | OEt | A01 | al | B05 | b3 | C11 | c1 | D01 | dl | E04 |
| 2-2-d1E04-2-0346 | 897.319 | OEt | A02 | al | B02 | b1 | C14 | c2 | D02 | dl | E04 |
| 2-2-d1E04-2-0347 | 897.324 | OEt | A01 | al | B03 | b4 | C02 | c2 | D02 | dl | E04 |
| 2-2-d1E04-2-0348 | 897.350 | OEt | A02 | al | B05 | b1 | C16 | c1 | D01 | dl | E04 |
| 2-2-d1E04-2-0349 | 897.425 | OEt | A02 | al | B02 | b1 | C19 | c1 | D01 | dl | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0350 | 898.345 | OEt | A02 | al | B05 | b1 | C17 | c1 | D01 | dl | E04 |
| 2-2-d1E04-2-0351 | 898.355 | OEt | A02 | al | B04 | b4 | C02 | c1 | D01 | dl | E04 |
| 2-2-d1E04-2-0352 | 899.278 | OEt | A02 | al | B03 | b3 | C09 | c2 | D02 | dl | E04 |
| 2-2-d1E04-2-0353 | 899.280 | OEt | A02 | al | B01 | b1 | C18 | c2 | D02 | dl | E04 |
| 2-2-d1E04-2-0354 | 899.306 | OEt | A01 | al | B02 | b4 | C05 | c2 | D02 | dl | E04 |
| 2-2-d1E04-2-0355 | 899.321 | OEt | A01 | al | B05 | b4 | C12 | c2 | D02 | dl | E04 |
| 2-2-d1E04-2-0356 | 899.354 | OEt | A02 | al | B05 | b4 | C01 | c1 | D01 | dl | E04 |
| 2-2-d1E04-2-0357 | 900.210 | OEt | A01 | al | B04 | b1 | C05 | c2 | D02 | dl | E04 |
| 2-2-d1E04-2-0358 | 900.264 | OEt | A02 | al | B02 | b3 | C10 | c2 | D02 | dl | E04 |
| 2-2-d1E04-2-0359 | 901.223 | OEt | A01 | al | B04 | b3 | C08 | c2 | D02 | dl | E04 |
| 2-2-d1E04-2-0360 | 901.294 | OEt | A02 | al | B03 | b1 | C14 | c2 | D02 | dl | E04 |
| 2-2-d1E04-2-0361 | 901.300 | OEt | A01 | al | B05 | b4 | C13 | c2 | D02 | dl | E04 |
| 2-2-d1E04-2-0362 | 901.318 | OEt | A02 | al | B02 | b2 | C02 | c2 | D02 | dl | E04 |
| 2-2-d1E04-2-0363 | 901.318 | OEt | A02 | al | B02 | b2 | C03 | c2 | D02 | dl | E04 |
| 2-2-d1E04-2-0364 | 901.400 | OEt | A02 | al | B03 | b1 | C19 | c1 | D01 | dl | E04 |
| 2-2-d1E04-2-0365 | 902.313 | OEt | A02 | al | B02 | b2 | C04 | c2 | D02 | dl | E04 |
| 2-2-d1E04-2-0366 | 902.313 | OEt | A02 | al | B02 | b2 | C06 | c2 | D02 | dl | E04 |
| 2-2-d1E04-2-0367 | 902.313 | OEt | A02 | al | B02 | b2 | C07 | c2 | D02 | dl | E04 |
| 2-2-d1E04-2-0368 | 903.281 | OEt | A01 | al | B03 | b4 | C05 | c2 | D02 | dl | E04 |
| 2-2-d1E04-2-0369 | 903.306 | OEt | A02 | al | B05 | b1 | C18 | c1 | D01 | dl | E04 |
| 2-2-d1E04-2-0370 | 904.239 | OEt | A02 | al | B03 | b3 | C10 | c2 | D02 | dl | E04 |
| 2-2-d1E04-2-0371 | 904.311 | OEt | A02 | al | B04 | b4 | C05 | c1 | D01 | dl | E04 |
| 2-2-d1E04-2-0372 | 905.274 | OEt | A01 | al | B05 | b1 | C02 | c2 | D02 | dl | E04 |
| 2-2-d1E04-2-0373 | 905.274 | OEt | A01 | al | B05 | b1 | C03 | c2 | D02 | dl | E04 |
| 2-2-d1E04-2-0374 | 905.293 | OEt | A02 | al | B03 | b2 | C02 | c2 | D02 | dl | E04 |
| 2-2-d1E04-2-0375 | 905.293 | OEt | A02 | al | B03 | b2 | C03 | c2 | D02 | dl | E04 |
| 2-2-d1E04-2-0376 | 905.349 | OEt | A02 | al | B01 | b4 | C02 | c2 | D02 | d1 | E04 |
| 2-2-d1E04-2-0377 | 906.269 | OEt | A01 | al | B05 | b1 | C04 | c2 | D02 | dl | E04 |
| 2-2-d1E04-2-0378 | 906.288 | OEt | A02 | al | B03 | b2 | C04 | c2 | D02 | d1 | E04 |
| 2-2-d1E04-2-0379 | 906.288 | OEt | A02 | al | B03 | b2 | C06 | c2 | D02 | dl | E04 |
| 2-2-d1E04-2-0380 | 906.288 | OEt | A02 | al | B03 | b2 | C07 | c2 | D02 | dl | E04 |
| 2-2-d1E04-2-0381 | 906.344 | OEt | A02 | al | B02 | b1 | C15 | c2 | D02 | dl | E04 |
| 2-2-d1E04-2-0382 | 907.292 | OEt | A01 | al | B02 | b3 | C11 | c2 | D02 | dl | E04 |
| 2-2-d1E04-2-0383 | 907.340 | OEt | A02 | al | B02 | b1 | C16 | c2 | D02 | dl | E04 |
| 2-2-d1E04-2-0384 | 908.335 | OEt | A02 | al | B02 | b1 | C17 | c2 | D02 | dl | E04 |
| 2-2-d1E04-2-0385 | 909.344 | OEt | A02 | al | B02 | b4 | C01 | c2 | D02 | dl | E04 |
| 2-2-d1E04-2-0386 | 909.375 | OEt | A02 | al | B05 | b4 | C02 | c1 | D01 | dl | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0387 | 910.249 | OEt | A02 | al | B04 | b1 | C01 | c2 | D02 | dl | E04 |
| 2-2-d1E04-2-0388 | 910.319 | OEt | A02 | al | B03 | b1 | C15 | c2 | D02 | dl | E04 |
| 2-2-d1E04-2-0389 | 911.230 | OEt | A01 | al | B05 | b1 | C05 | c2 | D02 | dl | E04 |
| 2-2-d1E04-2-0390 | 911.267 | OEt | A01 | al | B03 | b3 | C11 | c2 | D02 | dl | E04 |
| 2-2-d1E04-2-0391 | 911.306 | OEt | A02 | al | B01 | b4 | C05 | c2 | D02 | dl | E04 |
| 2-2-d1E04-2-0392 | 911.315 | OEt | A02 | al | B03 | b1 | C16 | c2 | D02 | dl | E04 |
| 2-2-d1E04-2-0393 | 912.239 | OEt | A01 | al | B04 | b3 | C09 | c2 | D02 | dl | E04 |
| 2-2-d1E04-2-0394 | 912.243 | OEt | A01 | al | B05 | b3 | C08 | c2 | D02 | dl | E04 |
| 2-2-d1E04-2-0395 | 912.298 | OEt | A02 | al | B04 | b3 | C11 | c1 | D01 | dl | E04 |
| 2-2-d1E04-2-0396 | 912.310 | OEt | A02 | al | B03 | b1 | C17 | c2 | D02 | dl | E04 |
| 2-2-d1E04-2-0397 | 913.296 | OEt | A02 | al | B02 | b1 | C18 | c2 | D02 | dl | E04 |
| 2-2-d1E04-2-0398 | 913.319 | OEt | A02 | al | B03 | b4 | C01 | c2 | D02 | dl | E04 |
| 2-2-d1E04-2-0399 | 914.255 | OEt | A01 | al | B04 | b1 | C14 | c2 | D02 | dl | E04 |
| 2-2-d1E04-2-0400 | 914.317 | OEt | A02 | al | B04 | b4 | C12 | c2 | D02 | dl | E04 |
| 2-2-d1E04-2-0401 | 914.361 | OEt | A01 | al | B04 | b1 | C19 | c1 | D01 | dl | E04 |
| 2-2-d1E04-2-0402 | 915.331 | OEt | A02 | al | B05 | b4 | C05 | c1 | D01 | dl | E04 |
| 2-2-d1E04-2-0403 | 916.296 | OEt | A02 | al | B04 | b4 | C13 | c2 | D02 | dl | E04 |
| 2-2-d1E04-2-0404 | 917.201 | OEt | A01 | al | B04 | b3 | C10 | c2 | D02 | dl | E04 |
| 2-2-d1E04-2-0405 | 917.271 | OEt | A02 | al | B03 | b1 | C18 | c2 | D02 | dl | E04 |
| 2-2-d1E04-2-0406 | 918.254 | OEt | A01 | al | B04 | b2 | C02 | c2 | D02 | dl | E04 |
| 2-2-d1E04-2-0407 | 918.254 | OEt | A01 | al | B04 | b2 | C03 | c2 | D02 | dl | E04 |
| 2-2-d1E04-2-0408 | 919.249 | OEt | A01 | al | B04 | b2 | C04 | c2 | D02 | dl | E04 |
| 2-2-d1E04-2-0409 | 919.249 | OEt | A01 | al | B04 | b2 | C06 | c2 | D02 | dl | E04 |
| 2-2-d1E04-2-0410 | 919.249 | OEt | A01 | al | B04 | b2 | C07 | c2 | D02 | dl | E04 |
| 2-2-d1E04-2-0411 | 919.292 | OEt | A02 | al | B01 | b3 | C11 | c2 | D02 | dl | E04 |
| 2-2-d1E04-2-0412 | 919.365 | OEt | A02 | al | B02 | b4 | C02 | c2 | D02 | dl | E04 |
| 2-2-d1E04-2-0413 | 920.270 | OEt | A02 | al | B04 | b1 | C02 | c2 | D02 | dl | E04 |
| 2-2-d1E04-2-0414 | 920.270 | OEt | A02 | al | B04 | b1 | C03 | c2 | D02 | dl | E04 |
| 2-2-d1E04-2-0415 | 921.265 | OEt | A02 | al | B04 | b1 | C04 | c2 | D02 | dl | E04 |
| 2-2-d1E04-2-0416 | 921.269 | OEt | A02 | al | B05 | b1 | C01 | c2 | D02 | dl | E04 |
| 2-2-d1E04-2-0417 | 921.355 | OEt | A01 | al | B01 | b1 | C19 | c2 | D02 | dl | E04 |
| 2-2-d1E04-2-0418 | 923.259 | OEt | A01 | al | B05 | b3 | C09 | c2 | D02 | dl | E04 |
| 2-2-d1E04-2-0419 | 923.280 | OEt | A01 | al | B04 | b1 | C15 | c2 | D02 | dl | E04 |
| 2-2-d1E04-2-0420 | 923.318 | OEt | A02 | al | B05 | b3 | C11 | c1 | D01 | dl | E04 |
| 2-2-d1E04-2-0421 | 923.340 | OEt | A02 | al | B03 | b4 | C02 | c2 | D02 | dl | E04 |
| 2-2-d1E04-2-0422 | 924.276 | OEt | A01 | al | B04 | b1 | C16 | c2 | D02 | dl | E04 |
| 2-2-d1E04-2-0423 | 925.271 | OEt | A01 | al | B04 | b1 | C17 | c2 | D02 | d1 | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0424 | 925.275 | OEt | A01 | al | B05 | b1 | C14 | c2 | D02 | dl | E04 |
| 2-2-d1E04-2-0425 | 925.321 | OEt | A02 | al | B02 | b4 | C05 | c2 | D02 | d1 | E04 |
| 2-2-d1E04-2-0426 | 925.337 | OEt | A02 | al | B05 | b4 | C12 | c2 | D02 | d1 | E04 |
| 2-2-d1E04-2-0427 | 925.381 | OEt | A01 | al | B05 | b1 | C19 | c1 | D01 | dl | E04 |
| 2-2-d1E04-2-0428 | 926.226 | OEt | A02 | al | B04 | b1 | C05 | c2 | D02 | dl | E04 |
| 2-2-d1E04-2-0429 | 926.280 | OEt | A01 | al | B04 | b4 | C01 | c2 | D02 | dl | E04 |
| 2-2-d1E04-2-0430 | 927.239 | OEt | A02 | al | B04 | b3 | C08 | c2 | D02 | dl | E04 |
| 2-2-d1E04-2-0431 | 927.316 | OEt | A02 | al | B05 | b4 | C13 | c2 | D02 | dl | E04 |
| 2-2-d1E04-2-0432 | 928.221 | OEt | A01 | al | B05 | b3 | C10 | c2 | D02 | dl | E04 |
| 2-2-d1E04-2-0433 | 929.274 | OEt | A01 | al | B05 | b2 | C02 | c2 | D02 | dl | E04 |
| 2-2-d1E04-2-0434 | 929.274 | OEt | A01 | al | B05 | b2 | C03 | c2 | D02 | dl | E04 |
| 2-2-d1E04-2-0435 | 929.296 | OEt | A02 | al | B03 | b4 | C05 | c2 | D02 | dl | E04 |
| 2-2-d1E04-2-0436 | 930.232 | OEt | A01 | al | B04 | b1 | C18 | c2 | D02 | dl | E04 |
| 2-2-d1E04-2-0437 | 930.269 | OEt | A01 | al | B05 | b2 | C04 | c2 | D02 | dl | E04 |
| 2-2-d1E04-2-0438 | 930.269 | OEt | A01 | al | B05 | b2 | C06 | c2 | D02 | dl | E04 |
| 2-2-d1E04-2-0439 | 930.269 | OEt | A01 | al | B05 | b2 | C07 | c2 | D02 | dl | E04 |
| 2-2-d1E04-2-0440 | 931.290 | OEt | A02 | al | B05 | b1 | C02 | c2 | D02 | dl | E04 |
| 2-2-d1E04-2-0441 | 931.290 | OEt | A02 | al | B05 | b1 | C03 | c2 | D02 | dl | E04 |
| 2-2-d1E04-2-0442 | 932.285 | OEt | A02 | al | B05 | b1 | C04 | c2 | D02 | dl | E04 |
| 2-2-d1E04-2-0443 | 933.308 | OEt | A02 | al | B02 | b3 | C11 | c2 | D02 | dl | E04 |
| 2-2-d1E04-2-0444 | 934.301 | OEt | A01 | al | B05 | b1 | C15 | c2 | D02 | dl | E04 |
| 2-2-d1E04-2-0445 | 935.296 | OEt | A01 | al | B05 | b1 | C16 | c2 | D02 | dl | E04 |
| 2-2-d1E04-2-0446 | 935.371 | OEt | A01 | al | B02 | b1 | C19 | c2 | D02 | dl | E04 |
| 2-2-dlE04-2-0447 | 936.291 | OEt | A01 | al | B05 | b1 | C17 | c2 | D02 | dl | E04 |
| 2-2-d1E04-2-0448 | 936.301 | OEt | A01 | al | B04 | b4 | C02 | c2 | D02 | dl | E04 |
| 2-2-d1E04-2-0449 | 937.246 | OEt | A02 | al | B05 | b1 | C05 | c2 | D02 | dl | E04 |
| 2-2-d1E04-2-0450 | 937.283 | OEt | A02 | al | B03 | b3 | C11 | c2 | D02 | dl | E04 |
| 2-2-d1E04-2-0451 | 937.300 | OEt | A01 | al | B05 | b4 | C01 | c2 | D02 | dl | E04 |
| 2-2-d1E04-2-0452 | 938.255 | OEt | A02 | al | B04 | b3 | C09 | c2 | D02 | dl | E04 |
| 2-2-d1E04-2-0453 | 938.259 | OEt | A02 | al | B05 | b3 | C08 | c2 | D02 | dl | E04 |
| 2-2-d1E04-2-0454 | 939.346 | OEt | A01 | al | B03 | b1 | C19 | c2 | D02 | dl | E04 |
| 2-2-d1E04-2-0455 | 940.271 | OEt | A02 | al | B04 | b1 | C14 | c2 | D02 | dl | E04 |
| 2-2-d1E04-2-0456 | 940.376 | OEt | A02 | al | B04 | b1 | C19 | c1 | D01 | dl | E04 |
| 2-2-d1E04-2-0457 | 941.252 | OEt | A01 | al | B05 | b1 | C18 | c2 | D02 | dl | E04 |
| 2-2-d1E04-2-0458 | 942.257 | OEt | A01 | al | B04 | b4 | C05 | c2 | D02 | dl | E04 |
| 2-2-d1E04-2-0459 | 943.216 | OEt | A02 | al | B04 | b3 | C10 | c2 | D02 | dl | E04 |
| 2-2-d1E04-2-0460 | 944.270 | OEt | A02 | al | B04 | b2 | C02 | c2 | D02 | dl | E04 |
| 2-2-d1E04-2-0461 | 944.270 | OEt | A02 | al | B04 | b2 | C03 | c2 | D02 | dl | E04 |

(continued)

| [Table 9-7-5] Compound that may be contained in mixture 2-2-d1E04-2 | | | | | | | | | | | |
|---|---|---|---|---|---|---|---|---|---|---|---|
| ID | Exact Mass | | A | a | B | b | C | c | D | d | E |
| 2-2-d1E04-2-0462 | 945.265 | OEt | A02 | al | B04 | b2 | C04 | c2 | D02 | dl | E04 |
| 2-2-d1E04-2-0463 | 945.265 | OEt | A02 | al | B04 | b2 | C06 | c2 | D02 | dl | E04 |
| 2-2-d1E04-2-0464 | 945.265 | OEt | A02 | al | B04 | b2 | C07 | c2 | D02 | dl | E04 |
| 2-2-d1E04-2-0465 | 947.321 | OEt | A01 | al | B05 | b4 | C02 | c2 | D02 | dl | E04 |
| 2-2-d1E04-2-0466 | 947.371 | OEt | A02 | al | B01 | b1 | C19 | c2 | D02 | dl | E04 |
| 2-2-d1E04-2-0467 | 949.275 | OEt | A02 | al | B05 | b3 | C09 | c2 | D02 | dl | E04 |
| 2-2-d1E04-2-0468 | 949.296 | OEt | A02 | al | B04 | b1 | C15 | c2 | D02 | dl | E04 |
| 2-2-dlE04-2-0469 | 950.244 | OEt | A01 | al | B04 | b3 | C11 | c2 | D02 | dl | E04 |
| 2-2-dlE04-2-0470 | 950.291 | OEt | A02 | al | B04 | b1 | C16 | c2 | D02 | dl | E04 |
| 2-2-d1E04-2-0471 | 951.287 | OEt | A02 | al | B04 | b1 | C17 | c2 | D02 | dl | E04 |
| 2-2-dlE04-2-0472 | 951.291 | OEt | A02 | al | B05 | b1 | C14 | c2 | D02 | dl | E04 |
| 2-2-dlE04-2-0473 | 951.396 | OEt | A02 | al | B05 | b1 | C19 | c1 | D01 | dl | E04 |
| 2-2-d1E04-2-0474 | 952.296 | OEt | A02 | al | B04 | b4 | C01 | c2 | D02 | dl | E04 |
| 2-2-d1E04-2-0475 | 953.277 | OEt | A01 | al | B05 | b4 | C05 | c2 | D02 | dl | E04 |
| 2-2-dlE04-2-0476 | 954.236 | OEt | A02 | al | B05 | b3 | C10 | c2 | D02 | dl | E04 |
| 2-2-d1E04-2-0477 | 955.290 | OEt | A02 | al | B05 | b2 | C02 | c2 | D02 | dl | E04 |
| 2-2-dlE04-2-0478 | 955.290 | OEt | A02 | al | B05 | b2 | C03 | c2 | D02 | dl | E04 |
| 2-2-dlE04-2-0479 | 956.248 | OEt | A02 | al | B04 | b1 | C18 | c2 | D02 | dl | E04 |
| 2-2-d1E04-2-0480 | 956.285 | OEt | A02 | al | B05 | b2 | C04 | c2 | D02 | dl | E04 |
| 2-2-dlE04-2-0481 | 956.285 | OEt | A02 | al | B05 | b2 | C06 | c2 | D02 | dl | E04 |
| 2-2-dlE04-2-0482 | 956.285 | OEt | A02 | al | B05 | b2 | C07 | c2 | D02 | dl | E04 |
| 2-2-d1E04-2-0483 | 960.316 | OEt | A02 | al | B05 | b1 | C15 | c2 | D02 | dl | E04 |
| 2-2-dlE04-2-0484 | 961.264 | OEt | A01 | al | B05 | b3 | C11 | c2 | D02 | dl | E04 |
| 2-2-d1E04-2-0485 | 961.311 | OEt | A02 | al | B05 | b1 | C16 | c2 | D02 | dl | E04 |
| 2-2-d1E04-2-0486 | 961.387 | OEt | A02 | al | B02 | b1 | C19 | c2 | D02 | dl | E04 |
| 2-2-d1E04-2-0487 | 962.307 | OEt | A02 | al | B05 | b1 | C17 | c2 | D02 | dl | E04 |
| 2-2-dlE04-2-0488 | 962.317 | OEt | A02 | al | B04 | b4 | C02 | c2 | D02 | dl | E04 |
| 2-2-d1E04-2-0489 | 963.316 | OEt | A02 | al | B05 | b4 | C01 | c2 | D02 | dl | E04 |
| 2-2-dlE04-2-0490 | 965.362 | OEt | A02 | al | B03 | b1 | C19 | c2 | D02 | dl | E04 |
| 2-2-d1E04-2-0491 | 967.268 | OEt | A02 | al | B05 | b1 | C18 | c2 | D02 | dl | E04 |
| 2-2-dlE04-2-0492 | 968.273 | OEt | A02 | al | B04 | b4 | C05 | c2 | D02 | dl | E04 |
| 2-2-d1E04-2-0493 | 973.337 | OEt | A02 | al | B05 | b4 | C02 | c2 | D02 | dl | E04 |
| 2-2-dlE04-2-0494 | 976.259 | OEt | A02 | al | B04 | b3 | C11 | c2 | D02 | dl | E04 |
| 2-2-d1E04-2-0495 | 978.323 | OEt | A01 | al | B04 | b1 | C19 | c2 | D02 | dl | E04 |
| 2-2-d1E04-2-0496 | 979.293 | OEt | A02 | al | B05 | b4 | C05 | c2 | D02 | dl | E04 |
| 2-2-dlE04-2-0497 | 987.279 | OEt | A02 | al | B05 | b3 | C11 | c2 | D02 | dl | E04 |
| 2-2-dlE04-2-0498 | 989.343 | OEt | A01 | al | B05 | b1 | C19 | c2 | D02 | dl | E04 |
| 2-2-dlE04-2-0499 | 1004.340 | OEt | A02 | al | B04 | b1 | C19 | c2 | D02 | dl | E04 |

(continued)

| [Table 9-7-5] Compound that may be contained in mixture 2-2-d1E04-2 | | | | | | | | | | | | |
|---|---|---|---|---|---|---|---|---|---|---|---|---|
| ID | Exact Mass | | A | a | B | b | C | c | D | d | E |
| 2-2-dlE04-2-0500 | 1015.360 | OEt | A02 | al | B05 | b1 | C19 | c2 | D02 | dl | E04 |

Example 9-8: Analysis of mixture

The mixtures synthesized in Examples 9-6 and 9-7 were subjected to retention time measurement and mass spectrometry under the following analysis conditions and analyzed using Compound Discoverer 3.2 (Thermo Fisher Scientific Inc.).

[Table 503]

| Reverse-phase LC/MS conditions | |
|---|---|
| Vanquish UHPLC (Thermo Fisher Scientific) | |
| Oven (°C) | 35 |
| Column | Ascentis Express C18<br>2.1mmI.D.×150mm, 2.7μm |
| Auto Sampler (°C) | 20 |
| | |
| Mobile phase | A) 0.1%FA H2O<br>B) 0.1%FAMeCN |
| Gradient | A/B = 95/5 -> 0/100 (18min) -> 0/100 (20min) |
| Flow rate | 0.5 mL/min |
| Orbitrap Fusion Lumos Tribrid mass spectrometer (Thermo Fisher Scientific) | |
| Polarity | Positive / Negative mode |
| Data Type | profile |

The results of analyzing each mixture are shown in Table 9-8-1 to Table 9-8-8. Each table shows m/z and retention times of observed MS peaks and the number of a compound that can be assigned to each MS peak.

Although the retention times were confirmed down to three decimal places, 0 at the decimal level may be omitted in the description in Table 9-8-1 to Table 9-8-8. For example, "15.000" is also referred to as "15", "15.100" is also referred to as "15.1", and "15.120" is also referred to as "15.12".

[Table 504]

| [Table 9-8-1] Compound that may be contained in mixture 2-2-d1E01-1 | | | |
|---|---|---|---|
| Ion species | m/z<br>[M+H]+ or [M-H]- | Retention time of MS peak (min) | No. of assigned compound<br>(described with 2-2-d1E01-1- omitted) |
| M+H | 569.2389 | 12.807 | 0001 |
| M+H | 573.3063 | 12.662 | 0002 |
| M-H | 573.2697 | 12.323 | 0003 |
| M+H | 579.2597 | 13.22 | 0004,0005 |
| M+H | 579.2597 | 13.111 | 0004,0005 |
| M-H | 578.2389 | 12.008 | 0006 |
| M+H | 583.2546 | 14.995 | 0007 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 585.2164 | 13.133 | 0008 |
| M+H | 586.2295 | 12.594 | 0009 |
| M+H | 587.2292 | 12.896 | 0010 |
| M+H | 587.3219 | 13.083 | 0011 |
| M+H | 589.3017 | 12.745 | 0012 |
| M-H | 589.2813 | 12.812 | 0013 |
| M+H | 593.2765 | 12.427 | 0014,0015,0016 |
| M+H | 593.2755 | 13.511 | 0014,0015,0016 |
| M-H | 591.2591 | 13.358 | 0014,0015,0016 |
| M+H | 594.2734 | 12.422 | 0017 |
| M+H | 595.2543 | 13.05 | 0018 |
| M+H | 597.2454 | 12.566 | 0019 |
| M+H | 597.2501 | 13.2 | 0019,0020,0021 |
| M+H | 597.2502 | 13.272 | 0019,0020,0021 |
| M-H | 596.2294 | 12.134 | 0022 |
| M+H | 599.2315 | 13.535 | 0023 |
| M+H | 599.2609 | 12.642 | 0024 |
| M+H | 599.3216 | 13.072 | 0025 |
| M+H | 600.2446 | 13.001 | 0026 |
| M+H | 601.3012 | 12.731 | 0027 |
| M+H | 602.2062 | 13.044 | 0028 |
| M+H | 603.2068 | 13.232 | 0029 |
| M+H | 603.2596 | 13.778 | 0030,0031 |
| M-H | 601.2431 | 13.67 | 0030,0031 |
| M+H | 604.2196 | 12.677 | 0032 |
| M+H | 604.2550 | 12.862 | 0033,0034,0035 |
| M+H | 604.2549 | 13.956 | 0033,0034,0035 |
| M+H | 604.2549 | 14.295 | 0033,0034,0035 |
| M+H | 605.2763 | 13.33 | 0036,0037 |
| M+H | 605.2747 | 13.229 | 0036,0037 |
| M+H | 606.2704 | 12.241 | 0038 |
| M+H | 608.2858 | 13.059 | 0039 |
| M+H | 609.2705 | 13.454 | 0040 |
| M+H | 609.2821 | 13.634 | 0041 |
| M+H | 610.2766 | 12.735 | 0042 |
| M+H | 611.2301 | 14.63 | 0043 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 611.2604 | 13.006 | 0044 |
| M+H | 611.2855 | 13.279 | 0045 |
| M+H | 612.2452 | 12.902 | 0046 |
| M+H | 613.2453 | 13.082 | 0047 |
| M+H | 613.2763 | 13.056 | 0048 |
| M+H | 613.3377 | 13.512 | 0049 |
| M+H | 615.2351 | 12.66 | 0050,0051 |
| M+H | 615.2390 | 13.193 | 0050,0051 |
| M+H | 615.3165 | 13.136 | 0052 |
| M+H | 616.2222 | 13.408 | 0053 |
| M+H | 617.2511 | 12.74 | 0054 |
| M+H | 617.2750 | 14.155 | 0055,0056 |
| M+H | 617.2750 | 14.056 | 0055,0056 |
| M+H | 617.3118 | 11.17 | 0057 |
| M+H | 618.2703 | 13.28 | 0058,0059,0060 |
| M-H | 616.2540 | 14.364 | 0058,0059,0060 |
| M+H | 618.2705 | 14.675 | 0058,0059,0060 |
| M+H | 619.2922 | 12.799 | 0061,0062,0063 |
| M+H | 619.2924 | 12.854 | 0061,0062,0063 |
| M+H | 619.2908 | 13.76 | 0061,0062,0063 |
| M+H | 620.1971 | 13.083 | 0064 |
| M+H | 620.2859 | 12.723 | 0065 |
| M+H | 621.2503 | 13.759 | 0066,0067 |
| M+H | 621.2503 | 13.847 | 0066,0067 |
| M+H | 621.3066 | 13.667 | 0068 |
| M+H | 622.2456 | 12.964 | 0069,0070,0071 |
| M+H | 622.2454 | 14.403 | 0069,0070,0071 |
| M+H | 622.2453 | 14.028 | 0069,0070,0071 |
| M+H | 622.3008 | 13.419 | 0072 |
| M+H | 623.2598 | 13.049 | 0073 |
| M+H | 623.2661 | 13.391 | 0073,0074,0075 |
| M+H | 623.2656 | 13.249 | 0073,0074,0075 |
| M+H | 623.2970 | 14.045 | 0076 |
| M-H | 622.2445 | 12.35 | 0077 |
| M+H | 624.2924 | 13.151 | 0078 |
| M-H | 623.2343 | 11.393 | 0079 |
| M+H | 625.2756 | 12.876 | 0080 |
| M-H | 623.2840 | 13.679 | 0081 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M-H | 624.1891 | 13.317 | 0082 |
| M-H | 624.2445 | 12.009 | 0083 |
| M+H | 626.2756 | 11.537 | 0084 |
| M+H | 627.2665 | 13.663 | 0085 |
| M+H | 627.2723 | 13.719 | 0086 |
| M+H | 628.2235 | 11.147 | 0087 |
| M+H | 628.2678 | 14.14 | 0088 |
| M+H | 629.2223 | 13.4 | 0089 |
| M+H | 629.2540 | 13.594 | 0090 |
| M+H | 629.2770 | 13.356 | 0091,0092,0093 |
| M+H | 629.2752 | 13.939 | 0091,0092,0093 |
| M+H | 629.2750 | 13.827 | 0091,0092,0093 |
| M+H | 630.2362 | 12.887 | 0094 |
| M+H | 630.2712 | 13.117 | 0095,0096,0097,0098 |
| M+H | 630.2705 | 14.474 | 0095,0096,0097,0098 |
| M+H | 630.2708 | 14.428 | 0095,0096,0097,0098 |
| M+H | 630.2735 | 13.223 | 0095,0096,0097,0098 |
| M-H | 630.2362 | 12.898 | 0099 |
| M+H | 633.2008 | 12.5 | 0100 |
| M+H | 633.2282 | 13.236 | 0101 |
| M+H | 634.3016 | 8.625 | 0102 |
| M+H | 635.2495 | 13.699 | 0103 |
| M+H | 635.2972 | 13.946 | 0104 |
| M+H | 635.3227 | 14.208 | 0105 |
| M+H | 636.2277 | 13.487 | 0106,0107 |
| M+H | 636.2278 | 13.634 | 0106,0107 |
| M+H | 636.2921 | 12.919 | 0108 |
| M+H | 637.2220 | 12.563 | 0109,0110 |
| M+H | 637.2257 | 13.829 | 0109,0110 |
| M+H | 637.2682 | 12.257 | 0111 |
| M+H | 637.2759 | 13.496 | 0112 |
| M+H | 637.3028 | 13.715 | 0113 |
| M+H | 639.2481 | 12.058 | 0114 |
| M+H | 639.2915 | 13.33 | 0115 |
| M+H | 639.2973 | 13.883 | 0115,0116 |
| M+H | 641.2505 | 13.09 | 0117,0118 |
| M+H | 641.2525 | 14.583 | 0117,0118 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 641.2786 | 13.981 | 0119 |
| M+H | 641.2941 | 13.765 | 0120 |
| M+H | 642.1835 | 13.629 | 0121 |
| M+H | 642.2376 | 13.679 | 0122 |
| M-H | 641.1796 | 13.102 | 0123 |
| M+H | 643.2190 | 12.921 | 0124,0125 |
| M-H | 641.2046 | 12.524 | 0124,0125 |
| M+H | 643.2671 | 12.933 | 0126 |
| M+H | 643.2738 | 13.404 | 0127 |
| M+H | 643.2908 | 14.383 | 0128,0129 |
| M+H | 643.2908 | 14.253 | 0128,0129 |
| M+H | 644.2167 | 11.628 | 0130 |
| M+H | 644.2863 | 14.559 | 0131,0132,0133 |
| M+H | 644.2864 | 14.962 | 0131,0132,0133 |
| M-H | 642.2690 | 14.859 | 0131,0132,0133 |
| M+H | 645.2536 | 13.732 | 0134 |
| M+H | 646.2123 | 13.291 | 0135 |
| M+H | 647.2155 | 14.679 | 0136 |
| M+H | 647.2470 | 14.125 | 0137,0138 |
| M+H | 647.2484 | 13.982 | 0137,0138 |
| M+H | 647.2659 | 10.425 | 0139,0140 |
| M-H | 645.2486 | 13.901 | 0139,0140 |
| M-H | 645.3043 | 14.117 | 0141 |
| M-H | 646.2254 | 13.171 | 0142 |
| M+H | 648.2617 | 14.501 | 0143,0144,0145 |
| M+H | 648.2602 | 14.164 | 0143,0144,0145 |
| M+H | 648.2616 | 14.193 | 0143,0144,0145 |
| M+H | 648.3170 | 13.398 | 0146 |
| M+H | 649.1776 | 12.652 | 0147 |
| M+H | 649.2664 | 13.19 | 0148 |
| M+H | 649.3138 | 14.403 | 0149 |
| M-H | 648.1750 | 12.412 | 0150 |
| M-H | 648.2912 | 13.415 | 0151 |
| M-H | 649.1745 | 12.613 | 0152 |
| M+H | 651.2835 | 12.65 | 0153 |
| M+H | 651.3167 | 13.686 | 0154 |
| M+H | 652.2223 | 13.525 | 0155 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 652.2933 | 13.634 | 0156 |
| M+H | 653.2031 | 14.152 | 0157 |
| M+H | 653.2404 | 13.679 | 0158 |
| M+H | 653.2634 | 12.367 | 0159 |
| M+H | 653.2774 | 13.169 | 0160 |
| M+H | 653.2907 | 12.435 | 0161 |
| M+H | 654.2130 | 12.99 | 0162,0163 |
| M+H | 654.2163 | 13.666 | 0162,0163 |
| M+H | 654.2824 | 12.999 | 0164 |
| M-H | 653.2424 | 12.326 | 0165 |
| M-H | 653.2518 | 12.808 | 0166 |
| M-H | 653.2738 | 13.679 | 0167 |
| M+H | 656.2272 | 13.179 | 0168 |
| M+H | 656.2900 | 13.763 | 0169 |
| M-H | 655.2220 | 12.184 | 0170,0171,0172 |
| M+H | 657.2356 | 13.124 | 0170,0171,0172 |
| M+H | 657.2384 | 12.138 | 0170,0171,0172 |
| M+H | 658.2332 | 12.126 | 0173 |
| M-H | 656.2516 | 13.286 | 0174 |
| M-H | 657.1558 | 13.531 | 0175 |
| M+H | 659.2161 | 12.766 | 0176 |
| M+H | 659.2442 | 13.424 | 0177 |
| M+H | 660.2271 | 14.28 | 0178,0179 |
| M+H | 660.2270 | 14.169 | 0178,0179 |
| M-H | 659.1907 | 12.422 | 0180 |
| M+H | 661.2123 | 12.839 | 0180,0181,0182 |
| M+H | 661.2120 | 12.619 | 0180,0181,0182 |
| M+H | 661.2223 | 14.471 | 0183,0184,0185 |
| M+H | 661.2222 | 14.832 | 0183,0184,0185 |
| M+H | 661.2223 | 13.399 | 0183,0184,0185 |
| M+H | 661.2651 | 13.74 | 0186 |
| M+H | 661.3385 | 14.512 | 0187 |
| M+H | 662.2075 | 11.799 | 0188 |
| M+H | 662.2432 | 13.872 | 0189,0190 |
| M+H | 662.2431 | 13.698 | 0189,0190 |
| M+H | 663.1931 | 13.056 | 0191 |
| M+H | 663.2224 | 12.257 | 0192 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 663.2378 | 12.853 | 0193,0194 |
| M+H | 663.2416 | 14.051 | 0193,0194 |
| M-H | 661.2681 | 12.78 | 0195 |
| M+H | 663.3280 | 12.897 | 0196 |
| M-H | 662.1916 | 10.825 | 0197 |
| M+H | 665.2324 | 13.662 | 0198 |
| M+H | 665.2570 | 13.985 | 0199 |
| M+H | 665.2632 | 12.482 | 0200 |
| M+H | 665.3124 | 14.153 | 0201 |
| M+H | 666.1661 | 11.045 | 0202 |
| M+H | 666.2485 | 14.157 | 0203 |
| M+H | 667.1682 | 12.761 | 0204 |
| M+H | 667.2208 | 13.162 | 0205,0206 |
| M+H | 667.2210 | 13.225 | 0205,0206 |
| M+H | 667.2439 | 13.398 | 0207,0208 |
| M+H | 667.2481 | 13.793 | 0207,0208 |
| M+H | 667.2935 | 14.387 | 0209 |
| M+H | 667.3102 | 14.237 | 0210 |
| M-H | 666.1652 | 12.521 | 0211 |
| M+H | 668.1998 | 13.842 | 0212 |
| M+H | 668.2161 | 12.962 | 0213,0214,0215 |
| M-H | 666.1998 | 13.417 | 0213,0214,0215 |
| M+H | 668.2171 | 12.917 | 0213,0214,0215 |
| M+H | 668.2532 | 13.821 | 0216 |
| M+H | 669.2098 | 13.713 | 0217 |
| M+H | 669.2373 | 13.009 | 0218,0219 |
| M+H | 669.2365 | 12.81 | 0218,0219 |
| M+H | 669.2884 | 13.842 | 0220 |
| M+H | 670.1936 | 14.086 | 0221 |
| M+H | 670.2325 | 11.909 | 0222 |
| M+H | 671.2470 | 14.691 | 0223,0224 |
| M+H | 671.2471 | 14.617 | 0223,0224 |
| M+H | 671.2691 | 14.023 | 0225 |
| M+H | 672.2052 | 13.707 | 0226 |
| M+H | 672.2424 | 13.901 | 0227,0228,0229 |
| M+H | 672.2422 | 14.912 | 0227,0228,0229 |
| M+H | 672.2424 | 15.237 | 0227,0228,0229 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 672.2474 | 12.549 | 0227,0228,0229,0230 |
| M-H | 671.2173 | 11.717 | 0231 |
| M+H | 673.2419 | 13.231 | 0232 |
| M+H | 673.2633 | 14.341 | 0233,0234 |
| M+H | 673.2632 | 14.232 | 0233,0234 |
| M+H | 674.2388 | 12.068 | 0235 |
| M+H | 674.2576 | 13.42 | 0236 |
| M+H | 675.1918 | 12.345 | 0237 |
| M-H | 673.2056 | 12.84 | 0238 |
| M+H | 675.2474 | 12.667 | 0239 |
| M+H | 675.2836 | 12.948 | 0240 |
| M+H | 676.2071 | 12.742 | 0241 |
| M-H | 674.2597 | 13.12 | 0242 |
| M-H | 675.1900 | 12.807 | 0243 |
| M-H | 675.2207 | 12.671 | 0244 |
| M+H | 677.2705 | 14.612 | 0245 |
| M+H | 677.2994 | 13.165 | 0246 |
| M+H | 677.3435 | 13.283 | 0247 |
| M+H | 678.2642 | 13.886 | 0248 |
| M+H | 678.2740 | 14.333 | 0249 |
| M+H | 679.1996 | 12.791 | 0250,0251 |
| M+H | 679.1997 | 12.743 | 0250,0251 |
| M+H | 679.2195 | 14.357 | 0252 |
| M+H | 679.2575 | 12.412 | 0253 |
| M-H | 677.2615 | 12.961 | 0254,0255 |
| M-H | 677.2626 | 12.447 | 0254,0255 |
| M+H | 680.1835 | 13.062 | 0256 |
| M+H | 680.2290 | 13.486 | 0257,0258 |
| M+H | 680.2321 | 13.938 | 0257,0258 |
| M+H | 681.2131 | 12.377 | 0259 |
| M+H | 681.2365 | 13.599 | 0260,0261 |
| M+H | 681.2359 | 13.648 | 0260,0261 |
| M+H | 681.2768 | 13.306 | 0262 |
| M-H | 679.3018 | 13 | 0263 |
| M+H | 682.2323 | 13.748 | 0264,0265,0266 |
| M-H | 680.2147 | 13.796 | 0264,0265,0266 |
| M+H | 682.2329 | 11.955 | 0264,0265,0266 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 682.2418 | 13.419 | 0267 |
| M+H | 683.2255 | 14.196 | 0268 |
| M+H | 683.2566 | 10.467 | 0269,0270,0271 |
| M+H | 683.2566 | 10.441 | 0269,0270,0271 |
| M+H | 683.2541 | 12.605 | 0269,0270,0271 |
| M+H | 684.1577 | 12.729 | 0272 |
| M+H | 684.2307 | 14.401 | 0273 |
| M+H | 684.2478 | 12.447 | 0274 |
| M-H | 683.1715 | 13.706 | 0275 |
| M+H | 685.2119 | 13.349 | 0276,0277 |
| M+H | 685.2119 | 13.256 | 0276,0277 |
| M-H | 683.2507 | 13.169 | 0278 |
| M+H | 686.2070 | 13.47 | 0279,0280,0281 |
| M+H | 686.2068 | 13.086 | 0279,0280,0281 |
| M-H | 684.1893 | 13.515 | 0279,0280,0281 |
| M+H | 686.2430 | 14.437 | 0282,0283 |
| M+H | 686.2423 | 14.301 | 0282,0283 |
| M+H | 686.2635 | 12.943 | 0284 |
| M+H | 687.2234 | 12.818 | 0285,0286,0287 |
| M+H | 687.2277 | 13.034 | 0285,0286,0287 |
| M+H | 687.2274 | 12.863 | 0285,0286,0287 |
| M+H | 687.2382 | 14.625 | 0288,0289,0290 |
| M+H | 687.2382 | 14.932 | 0288,0289,0290 |
| M+H | 687.2378 | 13.631 | 0288,0289,0290 |
| M+H | 687.2593 | 13.668 | 0291 |
| M+H | 688.2234 | 12.062 | 0292 |
| M+H | 688.2575 | 8.242 | 0293 |
| M+H | 689.2094 | 10.752 | 0294 |
| M+H | 689.2382 | 12.771 | 0295 |
| M+H | 689.2634 | 13.132 | 0296 |
| M+H | 689.2945 | 14.701 | 0297 |
| M+H | 689.3437 | 12.908 | 0298 |
| M+H | 690.1676 | 12.982 | 0299 |
| M+H | 690.2223 | 13.187 | 0300 |
| M-H | 688.2218 | 12.663 | 0301 |
| M+H | 691.2239 | 12.988 | 0302 |
| M+H | 691.2336 | 13.35 | 0303 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 691.2483 | 14.084 | 0304 |
| M+H | 691.2687 | 13.819 | 0305 |
| M+H | 692.1815 | 12.901 | 0306 |
| M+H | 692.2166 | 14.268 | 0307 |
| M-H | 690.2134 | 12.224 | 0308 |
| M+H | 692.2641 | 14.487 | 0309 |
| M+H | 693.1831 | 13.048 | 0310 |
| M+H | 693.2155 | 13.186 | 0311 |
| M-H | 691.2205 | 12.788 | 0312,0313,0314 |
| M-H | 691.2212 | 12.832 | 0312,0313,0314 |
| M+H | 693.2378 | 12.872 | 0312,0313,0314 |
| M+H | 693.2602 | 13.564 | 0315,0316 |
| M+H | 693.2640 | 13.942 | 0315,0316 |
| M+H | 694.1979 | 12.827 | 0317 |
| M+H | 694.2355 | 12.732 | 0318,0319,0320,0321 |
| M+H | 694.2342 | 13.044 | 0318,0319,0320,0321 |
| M-H | 692.2166 | 13.645 | 0318,0319,0320,0321 |
| M+H | 694.2355 | 12.769 | 0318,0319,0320,0321 |
| M+H | 694.2685 | 14.154 | 0322 |
| M+H | 695.2503 | 14.577 | 0323 |
| M-H | 694.1912 | 14.272 | 0324 |
| M+H | 696.2149 | 12.583 | 0325 |
| M+H | 697.1932 | 12.266 | 0326 |
| M+H | 697.2624 | 14.789 | 0327,0328 |
| M+H | 697.2623 | 14.878 | 0327,0328 |
| M+H | 698.2207 | 13.995 | 0329 |
| M+H | 698.2586 | 15.063 | 0330,0331,0332 |
| M+H | 698.2587 | 15.342 | 0330,0331,0332 |
| M+H | 698.2587 | 15.376 | 0330,0331,0332 |
| M+H | 698.2643 | 13.304 | 0333 |
| M+H | 699.2105 | 13.157 | 0334 |
| M+H | 699.2602 | 15.45 | 0335 |
| M+H | 699.2834 | 13.668 | 0336 |
| M+H | 700.1890 | 13.235 | 0337,0338 |
| M+H | 700.1891 | 13.015 | 0337,0338 |
| M+H | 700.2517 | 13.144 | 0339 |
| M-H | 699.1675 | 12.258 | 0340 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M-H | 699.1701 | 13.575 | 0340,0341 |
| M-H | 699.2214 | 13.273 | 0342 |
| M+H | 701.2625 | 13.176 | 0343 |
| M+H | 702.2888 | 14.294 | 0344 |
| M-H | 701.2192 | 12.206 | 0345 |
| M+H | 703.2538 | 13.155 | 0346 |
| M+H | 703.2587 | 13.321 | 0346,0347 |
| M+H | 703.2843 | 14.969 | 0348 |
| M+H | 703.3594 | 13.345 | 0349 |
| M+H | 704.2795 | 14.082 | 0350 |
| M+H | 704.2886 | 14.673 | 0351 |
| M+H | 705.2109 | 13.053 | 0352 |
| M-H | 703.2003 | 10.481 | 0352,0353 |
| M+H | 705.2407 | 13.398 | 0354 |
| M-H | 703.2379 | 13.328 | 0355 |
| M-H | 703.2725 | 14.59 | 0356 |
| M+H | 706.1453 | 13.148 | 0357 |
| M+H | 706.1997 | 13.362 | 0358 |
| M+H | 707.1579 | 12.952 | 0359 |
| M+H | 707.2288 | 12.699 | 0360 |
| M-H | 705.2179 | 13.15 | 0360,0361 |
| M+H | 707.2528 | 13.796 | 0362,0363 |
| M+H | 707.2521 | 13.927 | 0362,0363 |
| M+H | 707.3342 | 12.997 | 0364 |
| M+H | 708.2478 | 14.053 | 0365,0366,0367 |
| M+H | 708.2490 | 13.819 | 0365,0366,0367 |
| M-H | 706.2330 | 13.74 | 0365,0366,0367 |
| M+H | 709.2151 | 13.087 | 0368 |
| M+H | 709.2405 | 14.411 | 0369 |
| M+H | 710.1733 | 13.034 | 0370 |
| M+H | 710.2463 | 14.491 | 0371 |
| M+H | 711.2082 | 13.76 | 0372,0373 |
| M+H | 711.2086 | 13.716 | 0372,0373 |
| M+H | 711.2274 | 13.581 | 0374,0375 |
| M+H | 711.2273 | 13.469 | 0374,0375 |
| M+H | 711.2828 | 13.585 | 0376 |
| M+H | 712.2037 | 13.005 | 0377 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 712.2249 | 13.45 | 0378,0379,0380 |
| M+H | 712.2249 | 13.407 | 0378,0379,0380 |
| M+H | 712.2249 | 13.351 | 0378,0379,0380 |
| M-H | 710.2636 | 10.962 | 0381 |
| M+H | 713.2261 | 13.691 | 0382 |
| M+H | 713.2787 | 8.5 | 0383 |
| M+H | 714.2679 | 12.754 | 0384 |
| M+H | 715.2797 | 13.545 | 0385 |
| M+H | 715.3103 | 15.147 | 0386 |
| M+H | 716.1835 | 13.279 | 0387 |
| M+H | 716.2548 | 13.986 | 0388 |
| M+H | 717.1650 | 13.732 | 0389 |
| M-H | 715.1850 | 14.212 | 0390 |
| M+H | 717.2403 | 13.39 | 0391 |
| M+H | 717.2499 | 13.564 | 0392 |
| M+H | 718.1743 | 12.958 | 0393,0394 |
| M+H | 718.1795 | 13.347 | 0393,0394 |
| M+H | 718.2331 | 14.261 | 0395 |
| M-H | 716.2303 | 13.923 | 0396 |
| M-H | 717.2185 | 12.706 | 0397 |
| M+H | 719.2527 | 13.259 | 0398 |
| M+H | 720.1894 | 12.789 | 0399 |
| M+H | 720.2515 | 13.412 | 0400 |
| M+H | 720.2958 | 13.495 | 0401 |
| M+H | 721.2660 | 14.927 | 0402 |
| M+H | 722.2301 | 13.067 | 0403 |
| M+H | 723.1356 | 13.18 | 0404 |
| M+H | 723.2053 | 13.149 | 0405 |
| M+H | 724.1890 | 13.741 | 0406,0407 |
| M+H | 724.1890 | 13.63 | 0406,0407 |
| M+H | 725.1842 | 13.515 | 0408,0409,0410 |
| M+H | 725.1845 | 13.869 | 0408,0409,0410 |
| M+H | 725.1847 | 13.978 | 0408,0409,0410 |
| M-H | 723.2095 | 13.311 | 0411 |
| M+H | 725.2996 | 14.037 | 0412 |
| M+H | 726.2044 | 13.345 | 0413,0414 |
| M+H | 726.2038 | 13.525 | 0413,0414 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 727.1992 | 12.574 | 0415 |
| M+H | 727.2021 | 13.853 | 0415,0416 |
| M+H | 727.2901 | 12.454 | 0417 |
| M-H | 727.1795 | 13.011 | 0418 |
| M+H | 729.2146 | 13.082 | 0419 |
| M+H | 729.2516 | 14.676 | 0420 |
| M+H | 729.2740 | 13.69 | 0421 |
| M+H | 730.2107 | 13.794 | 0422 |
| M+H | 731.2054 | 12.817 | 0423 |
| M+H | 731.2134 | 13.848 | 0424 |
| M+H | 731.2555 | 11.309 | 0425 |
| M-H | 729.2538 | 13.868 | 0426 |
| M+H | 731.3156 | 13.932 | 0427 |
| M+H | 732.1612 | 13.467 | 0428 |
| M+H | 732.2152 | 13.292 | 0429 |
| M+H | 733.1731 | 13.209 | 0430 |
| M-H | 731.2336 | 13.618 | 0431 |
| M+H | 735.2085 | 14.222 | 0433,0434 |
| M-H | 733.1923 | 14.115 | 0433,0434 |
| M-H | 733.2146 | 13.482 | 0435 |
| M+H | 736.1667 | 13.304 | 0436 |
| M+H | 736.2058 | 14.336 | 0437,0438,0439 |
| M+H | 736.2066 | 9.571 | 0437,0438,0439 |
| M+H | 736.2069 | 14.137 | 0437,0438,0439 |
| M+H | 737.2243 | 14.044 | 0440,0441 |
| M+H | 737.2240 | 13.84 | 0440,0441 |
| M-H | 736.2026 | 13.312 | 0442 |
| M-H | 737.2255 | 13.788 | 0443 |
| M+H | 740.2327 | 13.524 | 0444 |
| M+H | 741.2306 | 14.247 | 0445 |
| M+H | 741.3047 | 12.82 | 0446 |
| M+H | 742.2254 | 13.177 | 0447 |
| M+H | 742.2360 | 13.794 | 0448 |
| M+H | 743.1813 | 14.047 | 0449 |
| M+H | 743.2165 | 13.396 | 0450 |
| M+H | 743.2385 | 13.979 | 0451 |
| M+H | 744.1894 | 13.303 | 0452 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M-H | 742.1765 | 13.861 | 0452,0453 |
| M+H | 745.2805 | 12.562 | 0454 |
| M+H | 746.2058 | 13.119 | 0455 |
| M+H | 746.3111 | 13.584 | 0456 |
| M+H | 747.1866 | 13.797 | 0457 |
| M+H | 748.1928 | 13.563 | 0458 |
| M+H | 749.1504 | 13.389 | 0459 |
| M+H | 750.2024 | 13.851 | 0460,0461 |
| M+H | 750.2041 | 13.977 | 0460,0461 |
| M+H | 751.1999 | 13.917 | 0462,0463,0464 |
| M+H | 751.1997 | 14.099 | 0462,0463,0464 |
| M+H | 751.1992 | 13.972 | 0462,0463,0464 |
| M-H | 751.2386 | 14.211 | 0465 |
| M+H | 753.3071 | 15.473 | 0466 |
| M+H | 755.2070 | 13.874 | 0467 |
| M+H | 755.2303 | 13.389 | 0468 |
| M+H | 756.1791 | 13.689 | 0469 |
| M+H | 756.2257 | 14.039 | 0470 |
| M+H | 757.2258 | 13.678 | 0471,0472 |
| M-H | 755.2112 | 13.77 | 0471,0472 |
| M+H | 757.3310 | 14.053 | 0473 |
| M+H | 758.2310 | 13.711 | 0474 |
| M-H | 757.1965 | 13.456 | 0475 |
| M+H | 760.1706 | 13.973 | 0476 |
| M+H | 761.2238 | 14.472 | 0477,0478 |
| M+H | 761.2237 | 14.349 | 0477,0478 |
| M+H | 762.1827 | 13.566 | 0479 |
| M+H | 762.2191 | 14.568 | 0480,0481,0482 |
| M+H | 762.2221 | 11.105 | 0480,0481,0482 |
| M+H | 762.2216 | 10.042 | 0480,0481,0482 |
| M+H | 766.2501 | 13.884 | 0483 |
| M+H | 767.1978 | 14.197 | 0484 |
| M+H | 767.2459 | 14.498 | 0485 |
| M-H | 765.3033 | 12.964 | 0486 |
| M+H | 768.2422 | 13.447 | 0487 |
| M+H | 768.2511 | 14.169 | 0488 |
| M-H | 767.2327 | 14.187 | 0489 |

(continued)

| [Table 9-8-1] Compound that may be contained in mixture 2-2-d1E01-1 | | | |
|---|---|---|---|
| Ion species | m/z [M+H]+ or [M-H]- | Retention time of MS peak (min) | No. of assigned compound (described with 2-2-d1E01-1- omitted) |
| M+H | 771.2961 | 12.651 | 0490 |
| M+H | 773.2024 | 14.07 | 0491 |
| M+H | 774.2084 | 13.94 | 0492 |
| M-H | 777.2529 | 14.607 | 0493 |
| M+H | 782.1941 | 13.915 | 0494 |
| M+H | 784.2573 | 12.999 | 0495 |
| M-H | 783.2105 | 14.372 | 0496 |
| M-H | 791.1957 | 14.267 | 0497 |
| M+H | 795.2775 | 13.456 | 0498 |
| M+H | 810.2732 | 13.184 | 0499 |
| M+H | 821.2937 | 13.649 | 0500 |

[Table 505]

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 585.2339 | 10.323 | 0001 |
| M+H | 589.3015 | 10.282 | 0002 |
| M+H | 591.2812 | 9.856 | 0003 |
| M+H | 595.2546 | 10.684 | 0004,0005 |
| M+H | 595.2546 | 10.516 | 0004,0005 |
| M+H | 596.2499 | 9.482 | 0006 |
| M+H | 599.2495 | 10.776 | 0007 |
| M+H | 601.2110 | 10.682 | 0008 |
| M+H | 602.2239 | 10.171 | 0009 |
| M+H | 603.2241 | 10.474 | 0010 |
| M+H | 603.3174 | 10.742 | 0011 |
| M+H | 605.2964 | 10.32 | 0012 |
| M+H | 607.2922 | 10.437 | 0013 |
| M-H | 607.2556 | 10.02 | 0014,0015,0016 |
| M+H | 609.2701 | 10.96 | 0014,0015,0016 |
| M+H | 609.2700 | 11.119 | 0014,0015,0016 |
| M+H | 610.2658 | 9.954 | 0017 |
| M+H | 611.2495 | 10.589 | 0018 |
| M+H | 613.2401 | 10.005 | 0019 |
| M+H | 613.2448 | 10.829 | 0019,0020,0021 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 613.2449 | 10.665 | 0019,0020,0021 |
| M+H | 614.2411 | 9.66 | 0022 |
| M+H | 615.2263 | 11.127 | 0023 |
| M+H | 615.2561 | 10.147 | 0024 |
| M+H | 615.3171 | 10.789 | 0025 |
| M+H | 616.2394 | 10.592 | 0026 |
| M-H | 615.2804 | 10.266 | 0027 |
| M+H | 618.2010 | 10.571 | 0028 |
| M+H | 619.2008 | 10.834 | 0029 |
| M+H | 619.2547 | 11.473 | 0030,0031 |
| M+H | 619.2547 | 11.343 | 0030,0031 |
| M+H | 620.2143 | 10.299 | 0032 |
| M+H | 620.2498 | 11.557 | 0033,0034,0035 |
| M+H | 620.2498 | 11.822 | 0033,0034,0035 |
| M+H | 620.2499 | 10.456 | 0033,0034,0035 |
| M+H | 621.2704 | 10.95 | 0036,0037 |
| M+H | 621.2697 | 10.835 | 0036,0037 |
| M+H | 622.2661 | 9.767 | 0038 |
| M+H | 624.2810 | 10.639 | 0039 |
| M+H | 625.2656 | 11.053 | 0040 |
| M+H | 625.2769 | 11.172 | 0041 |
| M+H | 626.2718 | 10.151 | 0042 |
| M+H | 627.2253 | 12.522 | 0043 |
| M+H | 627.2556 | 10.435 | 0044 |
| M+H | 627.2806 | 10.924 | 0045 |
| M+H | 628.2390 | 10.458 | 0046 |
| M+H | 629.2402 | 10.67 | 0047 |
| M+H | 629.2712 | 10.574 | 0048 |
| M+H | 629.3327 | 11.289 | 0049 |
| M-H | 629.2167 | 10.678 | 0050,0051 |
| M+H | 631.2304 | 10.128 | 0050,0051 |
| M+H | 631.3122 | 10.736 | 0052 |
| M+H | 632.2170 | 11.003 | 0053 |
| M+H | 633.2460 | 10.28 | 0054 |
| M+H | 633.2702 | 11.888 | 0055,0056 |
| M+H | 633.2702 | 11.764 | 0055,0056 |
| M+H | 633.3081 | 10.905 | 0057 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 634.2661 | 12.257 | 0058,0059,0060 |
| M+H | 634.2660 | 11.992 | 0058,0059,0060 |
| M+H | 634.2657 | 10.901 | 0058,0059,0060 |
| M+H | 635.2874 | 10.389 | 0061,0062,0063 |
| M+H | 635.2855 | 11.398 | 0061,0062,0063 |
| M+H | 635.2854 | 11.307 | 0061,0062,0063 |
| M+H | 636.1914 | 10.7 | 0064 |
| M+H | 636.2817 | 10.25 | 0065 |
| M+H | 637.2453 | 11.599 | 0066,0067 |
| M+H | 637.2454 | 11.472 | 0066,0067 |
| M+H | 637.3014 | 11.598 | 0068 |
| M+H | 638.2402 | 11.682 | 0069,0070,0071 |
| M+H | 638.2404 | 10.606 | 0069,0070,0071 |
| M+H | 638.2401 | 11.947 | 0069,0070,0071 |
| M+H | 638.2967 | 11.071 | 0072 |
| M-H | 637.2395 | 10.432 | 0073 |
| M+H | 639.2612 | 11.059 | 0073,0074,0075 |
| M+H | 639.2611 | 10.959 | 0073,0074,0075 |
| M-H | 637.2755 | 11.608 | 0076 |
| M+H | 640.2564 | 9.919 | 0077 |
| M+H | 640.2875 | 10.63 | 0078 |
| M+H | 641.2419 | 10.526 | 0079 |
| M+H | 641.2710 | 10.407 | 0080 |
| M+H | 641.2967 | 11.338 | 0081 |
| M+H | 642.2015 | 10.903 | 0082 |
| M-H | 640.2391 | 9.485 | 0083 |
| M+H | 642.2716 | 10.761 | 0084 |
| M+H | 643.2584 | 11.339 | 0085 |
| M+H | 643.2667 | 11.292 | 0086 |
| M+H | 644.2177 | 8.802 | 0087 |
| M+H | 644.2622 | 10.325 | 0088 |
| M-H | 643.2018 | 11.115 | 0089 |
| M+H | 645.2485 | 11.108 | 0090 |
| M+H | 645.2716 | 11.05 | 0091,0092,0093 |
| M+H | 645.2700 | 11.702 | 0091,0092,0093 |
| M+H | 645.2700 | 11.585 | 0091,0092,0093 |
| M+H | 646.2302 | 10.538 | 0094 |
| M+H | 646.2652 | 12.036 | 0095,0096,0097,0098 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 646.2652 | 11.766 | 0095,0096,0097,0098 |
| M+H | 646.2652 | 12.145 | 0095,0096,0097,0098 |
| M+H | 646.2685 | 10.904 | 0095,0096,0097,0098 |
| M+H | 648.2482 | 10.486 | 0099 |
| M+H | 649.1956 | 10.152 | 0100 |
| M+H | 649.2240 | 10.808 | 0101 |
| M+H | 650.2974 | 7.641 | 0102 |
| M+H | 651.2452 | 11.233 | 0103 |
| M+H | 651.2914 | 11.504 | 0104 |
| M+H | 651.3179 | 11.971 | 0105 |
| M+H | 652.2218 | 11.282 | 0106,0107 |
| M+H | 652.2218 | 11.128 | 0106,0107 |
| M+H | 652.2873 | 10.358 | 0108 |
| M+H | 653.2171 | 10.108 | 0109,0110 |
| M+H | 653.2207 | 11.565 | 0109,0110 |
| M-H | 651.2478 | 10.055 | 0111 |
| M-H | 651.2545 | 10.903 | 0112 |
| M+H | 653.2962 | 11.26 | 0113 |
| M+H | 655.2436 | 9.771 | 0114 |
| M+H | 655.2873 | 10.84 | 0115 |
| M+H | 655.2916 | 11.677 | 0115,0116 |
| M+H | 657.2479 | 12.403 | 0117,0118 |
| M+H | 657.2458 | 10.521 | 0117,0118 |
| M+H | 657.2743 | 11.74 | 0119 |
| M+H | 657.2886 | 11.583 | 0120 |
| M+H | 658.1786 | 11.259 | 0121 |
| M+H | 658.2326 | 11.227 | 0122 |
| M+H | 659.1917 | 10.739 | 0123 |
| M+H | 659.2171 | 10.246 | 0124,0125 |
| M+H | 659.2171 | 10.433 | 0124,0125 |
| M+H | 659.2621 | 10.504 | 0126 |
| M+H | 659.2684 | 11.187 | 0127 |
| M+H | 659.2859 | 12.148 | 0128,0129 |
| M-H | 657.2694 | 12.032 | 0128,0129 |
| M+H | 660.2119 | 8.966 | 0130 |
| M+H | 660.2812 | 12.494 | 0131,0132,0133 |
| M+H | 660.2811 | 12.217 | 0131,0132,0133 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 660.2803 | 11.214 | 0131,0132,0133 |
| M+H | 661.2486 | 11.463 | 0134 |
| M+H | 662.2079 | 10.879 | 0135 |
| M+H | 663.2114 | 10.621 | 0136 |
| M+H | 663.2418 | 11.913 | 0137,0138 |
| M+H | 663.2426 | 11.77 | 0137,0138 |
| M+H | 663.2606 | 11.692 | 0139,0140 |
| M+H | 663.2607 | 11.799 | 0139,0140 |
| M+H | 663.3175 | 11.844 | 0141 |
| M+H | 664.2380 | 10.823 | 0142 |
| M+H | 664.2561 | 12.13 | 0143,0144,0145 |
| M+H | 664.2564 | 10.873 | 0143,0144,0145 |
| M+H | 664.2555 | 11.862 | 0143,0144,0145 |
| M+H | 664.3114 | 6.061 | 0146 |
| M+H | 665.1731 | 10.342 | 0147 |
| M-H | 663.2482 | 9.568 | 0148 |
| M+H | 665.3108 | 7.102 | 0149 |
| M-H | 664.1699 | 10.094 | 0150 |
| M+H | 666.3040 | 10.86 | 0151 |
| M+H | 667.1858 | 10.327 | 0152 |
| M-H | 665.2638 | 10.513 | 0153 |
| M-H | 665.2956 | 11.701 | 0154 |
| M+H | 668.2172 | 11.188 | 0155 |
| M-H | 666.2705 | 11.01 | 0156 |
| M+H | 669.1982 | 11.92 | 0157 |
| M+H | 669.2327 | 8.508 | 0158 |
| M+H | 669.2584 | 10.227 | 0159 |
| M+H | 669.2770 | 11.655 | 0160 |
| M+H | 669.2818 | 11.619 | 0161 |
| M+H | 670.2072 | 10.424 | 0162,0163 |
| M+H | 670.2117 | 11.416 | 0162,0163 |
| M+H | 670.2787 | 10.495 | 0164 |
| M+H | 671.2525 | 7.849 | 0165 |
| M+H | 671.2645 | 6.951 | 0166 |
| M+H | 671.2872 | 11.357 | 0167 |
| M+H | 672.2235 | 10.73 | 0168 |
| M+H | 672.2849 | 11.326 | 0169 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 673.2339 | 9.946 | 0170,0171,0172 |
| M+H | 673.2322 | 10.876 | 0170,0171,0172 |
| M+H | 673.2315 | 10.667 | 0170,0171,0172 |
| M+H | 674.2276 | 9.521 | 0173 |
| M+H | 674.2643 | 10.868 | 0174 |
| M+H | 675.1684 | 11.11 | 0175 |
| M-H | 673.1947 | 9.929 | 0176 |
| M+H | 675.2396 | 11.017 | 0177 |
| M+H | 676.2220 | 11.89 | 0178,0179 |
| M-H | 674.2053 | 12.047 | 0178,0179 |
| M-H | 675.1863 | 10.014 | 0180 |
| M+H | 677.2065 | 10.593 | 0180,0181,0182 |
| M-H | 675.1907 | 10.41 | 0180,0181,0182 |
| M+H | 677.2176 | 11.05 | 0183,0184,0185 |
| M-H | 675.2002 | 12.145 | 0183,0184,0185 |
| M-H | 675.2002 | 12.407 | 0183,0184,0185 |
| M+H | 677.2631 | 10.074 | 0186 |
| M+H | 677.3327 | 12.275 | 0187 |
| M+H | 678.2019 | 9.211 | 0188 |
| M+H | 678.2370 | 11.405 | 0189,0190 |
| M+H | 678.2369 | 11.556 | 0189,0190 |
| M+H | 679.1879 | 10.791 | 0191 |
| M+H | 679.2175 | 9.93 | 0192 |
| M+H | 679.2323 | 10.433 | 0193 |
| M+H | 679.2369 | 11.808 | 0193,0194 |
| M+H | 679.2788 | 10.539 | 0195 |
| M-H | 677.3068 | 10.484 | 0196 |
| M+H | 680.2011 | 8.543 | 0197 |
| M-H | 679.2101 | 11.267 | 0198 |
| M+H | 681.2490 | 11.244 | 0199 |
| M+H | 681.2575 | 10.242 | 0200 |
| M+H | 681.3074 | 11.939 | 0201 |
| M-H | 680.1466 | 10.343 | 0202 |
| M-H | 680.2299 | 10.88 | 0203 |
| M+H | 683.1630 | 10.516 | 0204 |
| M+H | 683.2164 | 10.093 | 0205,0206 |
| M+H | 683.2163 | 10.97 | 0205,0206 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 683.2389 | 10.874 | 0207,0208 |
| M-H | 681.2260 | 11.406 | 0207,0208 |
| M-H | 681.2735 | 10.254 | 0209 |
| M+H | 683.3047 | 12.006 | 0210 |
| M-H | 682.1601 | 10.265 | 0211 |
| M+H | 684.1940 | 11.592 | 0212 |
| M+H | 684.2109 | 10.469 | 0213,0214,0215 |
| M+H | 684.2116 | 11.144 | 0213,0214,0215 |
| M+H | 684.2116 | 11.279 | 0213,0214,0215 |
| M+H | 684.2481 | 11.405 | 0216 |
| M+H | 685.2063 | 10.973 | 0217 |
| M+H | 685.2312 | 10.743 | 0218,0219 |
| M+H | 685.2313 | 10.604 | 0218,0219 |
| M+H | 685.2835 | 11.599 | 0220 |
| M+H | 686.1879 | 11.813 | 0221 |
| M+H | 686.2272 | 9.304 | 0222 |
| M+H | 687.2429 | 12.613 | 0223,0224 |
| M-H | 685.2295 | 10.56 | 0223,0224 |
| M+H | 687.2642 | 11.775 | 0225 |
| M+H | 688.2009 | 11.232 | 0226 |
| M+H | 688.2377 | 12.727 | 0227,0228,0229 |
| M+H | 688.2378 | 12.988 | 0227,0228,0229 |
| M+H | 688.2370 | 11.695 | 0227,0228,0229 |
| M+H | 688.2425 | 10.312 | 0227,0228,0229,0230 |
| M+H | 689.2274 | 10.985 | 0231 |
| M-H | 687.2213 | 10.868 | 0232 |
| M+H | 689.2574 | 12.178 | 0233,0234 |
| M+H | 689.2579 | 12.1 | 0233,0234 |
| M+H | 690.2348 | 10.399 | 0235 |
| M+H | 690.2525 | 11.116 | 0236 |
| M+H | 691.1859 | 10.068 | 0237 |
| M-H | 689.2016 | 10.492 | 0238 |
| M+H | 691.2428 | 10.554 | 0239 |
| M+H | 691.2787 | 11.073 | 0240 |
| M-H | 690.1847 | 8.201 | 0241 |
| M-H | 690.2518 | 11.868 | 0242 |
| M+H | 693.2017 | 10.639 | 0243 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 693.2336 | 10.384 | 0244 |
| M+H | 693.2634 | 12.371 | 0245 |
| M+H | 693.2950 | 11.009 | 0246 |
| M-H | 691.3222 | 10.915 | 0247 |
| M+H | 694.2594 | 11.514 | 0248 |
| M+H | 694.2690 | 12.135 | 0249 |
| M-H | 693.1746 | 10.202 | 0250 |
| M+H | 695.1939 | 10.473 | 0250,0251 |
| M+H | 695.2144 | 12.24 | 0252 |
| M-H | 693.2344 | 11.432 | 0253 |
| M-H | 693.2507 | 12.101 | 0254 |
| M-H | 693.2569 | 10.725 | 0254,0255 |
| M+H | 696.1781 | 10.783 | 0256 |
| M+H | 696.2230 | 10.893 | 0257,0258 |
| M+H | 696.2270 | 11.652 | 0257,0258 |
| M+H | 697.2079 | 10.104 | 0259 |
| M+H | 697.2325 | 11.339 | 0260,0261 |
| M+H | 697.2326 | 11.554 | 0260,0261 |
| M+H | 697.2710 | 10.714 | 0262 |
| M+H | 697.3134 | 10.63 | 0263 |
| M+H | 698.2296 | 10.99 | 0264,0265,0266 |
| M+H | 698.2271 | 11.569 | 0264,0265,0266 |
| M+H | 698.2274 | 9.173 | 0264,0265,0266 |
| M+H | 698.2363 | 9.849 | 0267 |
| M+H | 699.2199 | 11.944 | 0268 |
| M+H | 699.2458 | 1.58 | 0269,0270,0271 |
| M+H | 699.2511 | 8.781 | 0269,0270,0271 |
| M+H | 699.2486 | 10.394 | 0269,0270,0271 |
| M-H | 698.1374 | 10.5 | 0272 |
| M+H | 700.2250 | 12.123 | 0273 |
| M+H | 700.2424 | 9.882 | 0274 |
| M+H | 701.1841 | 11.292 | 0275 |
| M+H | 701.2069 | 11.219 | 0276,0277 |
| M+H | 701.2066 | 11.11 | 0276,0277 |
| M+H | 701.2638 | 11.137 | 0278 |
| M+H | 702.2027 | 10.671 | 0279,0280,0281 |
| M-H | 700.1856 | 11.278 | 0279,0280,0281 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 702.2023 | 11.249 | 0279,0280,0281 |
| M+H | 702.2379 | 12.239 | 0282,0283 |
| M-H | 700.2210 | 12.126 | 0282,0283 |
| M+H | 702.2582 | 10.738 | 0284 |
| M-H | 701.2059 | 10.936 | 0285,0286,0287 |
| M+H | 703.2224 | 10.849 | 0285,0286,0287 |
| M+H | 703.2225 | 10.748 | 0285,0286,0287 |
| M+H | 703.2337 | 12.582 | 0288,0289,0290 |
| M-H | 701.2155 | 12.329 | 0288,0289,0290 |
| M+H | 703.2337 | 12.431 | 0288,0289,0290 |
| M+H | 703.2543 | 11.305 | 0291 |
| M+H | 704.2183 | 9.529 | 0292 |
| M+H | 704.2489 | 10.155 | 0293 |
| M+H | 705.2048 | 9.054 | 0294 |
| M+H | 705.2330 | 10.299 | 0295 |
| M+H | 705.2583 | 10.977 | 0296 |
| M+H | 705.2892 | 12.657 | 0297 |
| M-H | 703.3224 | 10.457 | 0298 |
| M+H | 706.1633 | 10.717 | 0299 |
| M-H | 704.2042 | 6.7 | 0300 |
| M-H | 704.2192 | 10.662 | 0301 |
| M-H | 705.2047 | 10.382 | 0302 |
| M+H | 707.2286 | 11.009 | 0303 |
| M+H | 707.2422 | 11.68 | 0304 |
| M+H | 707.2635 | 11.52 | 0305 |
| M+H | 708.1790 | 10.652 | 0306 |
| M+H | 708.2153 | 10.381 | 0307 |
| M+H | 708.2240 | 9.874 | 0308 |
| M+H | 708.2588 | 12.102 | 0309 |
| M-H | 707.1630 | 9.025 | 0310 |
| M+H | 709.2099 | 10.911 | 0311 |
| M-H | 707.2167 | 10.71 | 0312,0313,0314 |
| M-H | 707.2149 | 11.377 | 0312,0313,0314 |
| M+H | 709.2335 | 10.664 | 0312,0313,0314 |
| M+H | 709.2546 | 11.106 | 0315,0316 |
| M+H | 709.2593 | 11.644 | 0315,0316 |
| M-H | 708.1760 | 10.639 | 0317 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M-H | 708.2117 | 11.437 | 0318,0319,0320,0321 |
| M+H | 710.2275 | 11.539 | 0318,0319,0320,0321 |
| M+H | 710.2311 | 11.206 | 0318,0319,0320,0321 |
| M+H | 710.2315 | 10.633 | 0318,0319,0320,0321 |
| M+H | 710.2640 | 11.863 | 0322 |
| M+H | 711.2458 | 12.472 | 0323 |
| M+H | 712.2051 | 12.001 | 0324 |
| M-H | 710.1941 | 10.899 | 0325 |
| M+H | 713.1851 | 10.627 | 0326 |
| M+H | 713.2577 | 12.833 | 0327,0328 |
| M+H | 713.2577 | 12.737 | 0327,0328 |
| M+H | 714.2163 | 11.482 | 0329 |
| M+H | 714.2531 | 13.194 | 0330,0331,0332 |
| M+H | 714.2535 | 11.981 | 0330,0331,0332 |
| M-H | 712.2355 | 12.907 | 0330,0331,0332 |
| M+H | 714.2586 | 10.962 | 0333 |
| M-H | 713.1894 | 10.899 | 0334 |
| M+H | 715.2565 | 12.909 | 0335 |
| M+H | 715.2783 | 11.537 | 0336 |
| M+H | 716.1839 | 11.008 | 0337,0338 |
| M+H | 716.1841 | 10.821 | 0337,0338 |
| M+H | 716.2495 | 9.99 | 0339 |
| M+H | 717.1791 | 9.729 | 0340,0341 |
| M+H | 717.1830 | 11.435 | 0340,0341 |
| M+H | 717.2370 | 6.108 | 0342 |
| M+H | 717.2586 | 10.948 | 0343 |
| M+H | 718.2837 | 12.145 | 0344 |
| M+H | 719.2314 | 9.78 | 0345 |
| M+H | 719.2487 | 10.766 | 0346 |
| M+H | 719.2535 | 11.278 | 0346,0347 |
| M+H | 719.2793 | 12.762 | 0348 |
| M+H | 719.3541 | 10.927 | 0349 |
| M+H | 720.2745 | 11.736 | 0350 |
| M+H | 720.2842 | 12.438 | 0351 |
| M+H | 721.2094 | 10.778 | 0352,0353 |
| M+H | 721.2137 | 13.127 | 0353 |
| M+H | 721.2369 | 11.25 | 0354 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M-H | 719.2345 | 11.322 | 0355 |
| M+H | 721.2820 | 2.379 | 0356 |
| M+H | 722.1396 | 10.922 | 0357 |
| M+H | 722.1940 | 11.109 | 0358 |
| M+H | 723.1534 | 10.726 | 0359 |
| M+H | 723.2239 | 10.458 | 0360 |
| M-H | 721.2141 | 11.08 | 0361 |
| M+H | 723.2473 | 11.816 | 0362,0363 |
| M+H | 723.2474 | 11.718 | 0362,0363 |
| M+H | 723.3294 | 10.597 | 0364 |
| M+H | 724.2427 | 11.459 | 0365,0366,0367 |
| M+H | 724.2430 | 11.872 | 0365,0366,0367 |
| M+H | 724.2411 | 11.111 | 0365,0366,0367 |
| M-H | 723.1946 | 10.99 | 0368 |
| M+H | 725.2356 | 12.156 | 0369 |
| M+H | 726.1690 | 10.804 | 0370 |
| M+H | 726.2409 | 12.266 | 0371 |
| M+H | 727.2034 | 11.667 | 0372,0373 |
| M-H | 725.1877 | 11.477 | 0372,0373 |
| M+H | 727.2225 | 11.405 | 0374,0375 |
| M+H | 727.2231 | 11.504 | 0374,0375 |
| M+H | 727.2783 | 11.463 | 0376 |
| M+H | 728.1988 | 10.599 | 0377 |
| M+H | 728.2177 | 11.105 | 0378,0379,0380 |
| M+H | 728.2171 | 11.531 | 0378,0379,0380 |
| M+H | 728.2171 | 11.22 | 0378,0379,0380 |
| M-H | 726.2586 | 9.274 | 0381 |
| M+H | 729.2214 | 11.325 | 0382 |
| M+H | 729.2717 | 6.83 | 0383 |
| M+H | 730.2640 | 10.477 | 0384 |
| M+H | 731.2740 | 11.381 | 0385 |
| M+H | 731.3045 | 12.998 | 0386 |
| M+H | 732.1785 | 11.088 | 0387 |
| M+H | 732.2474 | 11.747 | 0388 |
| M+H | 733.1596 | 11.639 | 0389 |
| M+H | 733.1946 | 11.049 | 0390 |
| M-H | 731.2188 | 11.245 | 0391 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 733.2449 | 11.263 | 0392 |
| M+H | 734.1694 | 10.642 | 0393,0394 |
| M-H | 732.1579 | 10.553 | 0393,0394 |
| M+H | 734.2269 | 11.945 | 0395 |
| M-H | 732.2253 | 12.727 | 0396 |
| M-H | 733.2145 | 10.062 | 0397 |
| M+H | 735.2482 | 11.07 | 0398 |
| M+H | 736.1849 | 10.552 | 0399 |
| M+H | 736.2468 | 11.242 | 0400 |
| M+H | 736.2900 | 11.18 | 0401 |
| M+H | 737.2608 | 12.822 | 0402 |
| M+H | 738.2260 | 10.886 | 0403 |
| M+H | 739.1306 | 10.935 | 0404 |
| M+H | 739.1998 | 10.927 | 0405 |
| M+H | 740.1834 | 11.623 | 0406,0407 |
| M+H | 740.1833 | 11.503 | 0406,0407 |
| M+H | 741.1789 | 11.13 | 0408,0409,0410 |
| M+H | 741.1793 | 11.675 | 0408,0409,0410 |
| M+H | 741.1791 | 11.253 | 0408,0409,0410 |
| M+H | 741.2215 | 11.08 | 0411 |
| M+H | 741.2945 | 11.884 | 0412 |
| M+H | 742.1994 | 11.144 | 0413,0414 |
| M+H | 742.1994 | 11.353 | 0413,0414 |
| M+H | 743.1949 | 10.096 | 0415,0416 |
| M+H | 743.1981 | 11.801 | 0415,0416 |
| M+H | 743.2854 | 10.217 | 0417 |
| M-H | 743.1725 | 11.386 | 0418 |
| M+H | 745.2101 | 10.921 | 0419 |
| M+H | 745.2472 | 12.49 | 0420 |
| M+H | 745.2689 | 11.581 | 0421 |
| M+H | 746.2049 | 11.487 | 0422 |
| M+H | 747.2017 | 10.352 | 0423,0424 |
| M+H | 747.2084 | 11.485 | 0424 |
| M+H | 747.2512 | 9.799 | 0425 |
| M-H | 745.2501 | 11.846 | 0426 |
| M+H | 747.3111 | 11.743 | 0427 |
| M+H | 748.1557 | 11.322 | 0428 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 748.2111 | 11.319 | 0429 |
| M-H | 747.1524 | 10.673 | 0430 |
| M-H | 747.2287 | 11.519 | 0431 |
| M+H | 750.1505 | 11.6 | 0432 |
| M-H | 749.1876 | 12.279 | 0433,0434 |
| M+H | 751.2023 | 10.376 | 0433,0434 |
| M-H | 749.2096 | 11.33 | 0435 |
| M-H | 750.1452 | 11.054 | 0436 |
| M+H | 752.1993 | 11.983 | 0437,0438,0439 |
| M+H | 752.1993 | 12.293 | 0437,0438,0439 |
| M-H | 750.1829 | 11.964 | 0437,0438,0439 |
| M+H | 753.2188 | 11.862 | 0440,0441 |
| M+H | 753.2182 | 12.012 | 0440,0441 |
| M+H | 754.2148 | 10.966 | 0442 |
| M+H | 755.2363 | 11.504 | 0443 |
| M+H | 756.2305 | 11.511 | 0444 |
| M+H | 757.2249 | 12.107 | 0445 |
| M+H | 757.3002 | 10.631 | 0446 |
| M-H | 756.2040 | 11.014 | 0447 |
| M+H | 758.2305 | 11.699 | 0448 |
| M+H | 759.1760 | 12.026 | 0449 |
| M-H | 757.1946 | 10.329 | 0450 |
| M+H | 759.2332 | 11.811 | 0451 |
| M+H | 760.1844 | 10.993 | 0452 |
| M-H | 758.1716 | 11.797 | 0452,0453 |
| M+H | 761.2756 | 10.373 | 0454 |
| M+H | 762.1998 | 10.919 | 0455 |
| M+H | 762.3064 | 11.245 | 0456 |
| M+H | 763.1811 | 11.7 | 0457 |
| M+H | 764.1872 | 11.434 | 0458 |
| M+H | 765.1461 | 11.191 | 0459 |
| M+H | 766.1991 | 11.912 | 0460,0461 |
| M+H | 766.1989 | 11.812 | 0460,0461 |
| M+H | 767.1936 | 11.598 | 0462,0463,0464 |
| M+H | 767.1948 | 12.742 | 0462,0463,0464 |
| M+H | 767.1937 | 11.704 | 0462,0463,0464 |
| M-H | 767.2342 | 12.258 | 0465 |

(continued)

| [Table 9-8-2] Compound that may be contained in mixture 2-2-d1E02-1 | | | |
|---|---|---|---|
| Ion species | m/z [M+H]+ or [M-H]- | Retention time of MS peak (min) | No. of assigned compound (described with 2-2-d1E02-1- omitted) |
| M+H | 769.3004 | 10.281 | 0466 |
| M-H | 769.1877 | 11.172 | 0467 |
| M+H | 771.2262 | 11.244 | 0468 |
| M+H | 772.1740 | 11.508 | 0469 |
| M+H | 772.2216 | 11.759 | 0470 |
| M+H | 773.2133 | 9.776 | 0471 |
| M+H | 773.2198 | 11.576 | 0471,0472 |
| M+H | 773.3260 | 11.831 | 0473 |
| M+H | 774.2251 | 11.544 | 0474 |
| M-H | 773.1913 | 11.997 | 0475 |
| M-H | 774.1488 | 11.92 | 0476 |
| M+H | 777.2185 | 12.553 | 0477,0478 |
| M+H | 777.2185 | 12.437 | 0477,0478 |
| M+H | 778.1781 | 11.372 | 0479 |
| M+H | 778.2155 | 12.433 | 0480,0481,0482 |
| M+H | 778.2172 | 12.549 | 0480,0481,0482 |
| M+H | 778.2152 | 12.588 | 0480,0481,0482 |
| M+H | 782.2454 | 11.849 | 0483 |
| M+H | 783.1932 | 12.165 | 0484 |
| M+H | 783.2413 | 12.366 | 0485 |
| M+H | 783.3162 | 10.739 | 0486 |
| M+H | 784.2368 | 11.33 | 0487 |
| M+H | 784.2461 | 12.055 | 0488 |
| M-H | 783.2290 | 12.175 | 0489 |
| M+H | 787.2915 | 10.442 | 0490 |
| M+H | 789.1967 | 12.038 | 0491 |
| M+H | 790.2027 | 11.828 | 0492 |
| M-H | 793.2500 | 12.62 | 0493 |
| M+H | 798.1895 | 11.74 | 0494 |
| M+H | 800.2530 | 10.866 | 0495 |
| M-H | 799.2048 | 12.4 | 0496 |
| M-H | 807.1924 | 12.364 | 0497 |
| M+H | 811.2722 | 11.433 | 0498 |
| M-H | 824.2514 | 11.036 | 0499 |

[Table 506]

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 623.1956 | 12.267 | 0001 |
| M+H | 627.2634 | 12.204 | 0002 |
| M+H | 629.2423 | 11.863 | 0003 |
| M+H | 633.2162 | 12.656 | 0004,0005 |
| M+H | 633.2162 | 12.562 | 0004,0005 |
| M+H | 634.2115 | 11.523 | 0006 |
| M+H | 637.2092 | 12.017 | 0007 |
| M+H | 639.1729 | 12.564 | 0008 |
| M+H | 640.1851 | 12.1 | 0009 |
| M+H | 641.1857 | 12.372 | 0010 |
| M+H | 641.2795 | 12.583 | 0011 |
| M+H | 643.2583 | 12.259 | 0012 |
| M+H | 645.2537 | 12.314 | 0013 |
| M+H | 647.2321 | 12.944 | 0014,0015,0016 |
| M-H | 645.2166 | 11.975 | 0014,0015,0016 |
| M+H | 647.2330 | 11.93 | 0014,0015,0016 |
| M+H | 648.2293 | 12.025 | 0017 |
| M+H | 649.2111 | 12.519 | 0018 |
| M+H | 651.2014 | 12.168 | 0019 |
| M+H | 651.2066 | 12.657 | 0019,0020,0021 |
| M+H | 651.2087 | 8.117 | 0020,0021 |
| M+H | 652.2021 | 11.648 | 0022 |
| M+H | 653.1881 | 12.967 | 0023 |
| M+H | 653.2169 | 12.122 | 0024 |
| M+H | 653.2788 | 12.578 | 0025 |
| M+H | 654.2009 | 12.469 | 0026 |
| M+H | 655.2580 | 12.246 | 0027 |
| M+H | 656.1625 | 12.491 | 0028 |
| M+H | 657.1629 | 12.67 | 0029 |
| M+H | 657.2161 | 13.195 | 0030,0031 |
| M+H | 657.2159 | 13.091 | 0030,0031 |
| M+H | 658.1762 | 12.168 | 0032 |
| M+H | 658.2112 | 13.575 | 0033,0034,0035 |
| M+H | 658.2117 | 12.322 | 0033,0034,0035 |
| M+H | 658.2114 | 13.311 | 0033,0034,0035 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 659.2299 | 8.241 | 0036,0037 |
| M+H | 659.2319 | 12.802 | 0036,0037 |
| M+H | 660.2270 | 11.785 | 0038 |
| M+H | 662.2424 | 12.518 | 0039 |
| M+H | 663.2268 | 12.906 | 0040 |
| M+H | 663.2384 | 13.041 | 0041 |
| M+H | 664.2338 | 12.172 | 0042 |
| M+H | 665.1866 | 14.076 | 0043 |
| M-H | 663.2005 | 12.422 | 0044 |
| M+H | 665.2425 | 12.741 | 0045 |
| M+H | 666.2004 | 12.389 | 0046 |
| M-H | 665.1843 | 12.598 | 0047 |
| M+H | 667.2335 | 13.56 | 0048 |
| M+H | 667.2949 | 13.004 | 0049 |
| M+H | 669.1920 | 12.121 | 0050,0051 |
| M+H | 669.1958 | 12.581 | 0050,0051 |
| M+H | 669.2735 | 12.659 | 0052 |
| M+H | 670.1787 | 12.867 | 0053 |
| M+H | 671.2076 | 12.205 | 0054 |
| M+H | 671.2324 | 12.702 | 0055,0056 |
| M+H | 671.2318 | 13.53 | 0055,0056 |
| M+H | 671.2711 | 12.767 | 0057 |
| M+H | 672.2274 | 13.686 | 0058,0059,0060 |
| M+H | 672.2273 | 12.709 | 0058,0059,0060 |
| M+H | 672.2274 | 13.949 | 0058,0059,0060 |
| M-H | 671.2333 | 12.323 | 0061,0062,0063 |
| M+H | 673.2488 | 12.381 | 0061,0062,0063 |
| M+H | 673.2476 | 13.198 | 0061,0062,0063 |
| M+H | 674.1535 | 12.531 | 0064 |
| M+H | 674.2424 | 12.213 | 0065 |
| M+H | 675.2063 | 13.271 | 0066,0067 |
| M+H | 675.2061 | 13.17 | 0066,0067 |
| M+H | 675.2632 | 13.275 | 0068 |
| M+H | 676.2022 | 13.666 | 0069,0070,0071 |
| M+H | 676.2021 | 13.384 | 0069,0070,0071 |
| M-H | 674.1845 | 12.429 | 0069,0070,0071 |
| M+H | 676.2585 | 12.887 | 0072 |
| M-H | 675.2046 | 12.866 | 0073,0074,0075 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M-H | 675.2095 | 9.613 | 0074,0075 |
| M-H | 675.2084 | 11.291 | 0074,0075 |
| M+H | 677.2537 | 13.381 | 0076 |
| M+H | 678.2175 | 11.882 | 0077 |
| M+H | 678.2498 | 12.6 | 0078 |
| M+H | 679.2058 | 8.664 | 0079 |
| M+H | 679.2319 | 12.483 | 0080 |
| M+H | 679.2583 | 13.102 | 0081 |
| M+H | 680.1628 | 12.747 | 0082 |
| M+H | 680.2167 | 12.773 | 0083 |
| M+H | 680.2330 | 12.726 | 0084 |
| M-H | 679.2057 | 13.095 | 0085 |
| M+H | 681.2259 | 13.101 | 0086 |
| M+H | 682.1800 | 10.826 | 0087 |
| M+H | 682.2239 | 12.283 | 0088 |
| M+H | 683.1784 | 12.887 | 0089 |
| M+H | 683.2099 | 12.961 | 0090 |
| M+H | 683.2314 | 13.393 | 0091,0092,0093 |
| M+H | 683.2312 | 13.289 | 0091,0092,0093 |
| M+H | 683.2329 | 12.811 | 0091,0092,0093 |
| M+H | 684.1924 | 12.436 | 0094 |
| M+H | 684.2272 | 13.755 | 0095,0096,0097,0098 |
| M+H | 684.2282 | 7.434 | 0095,0096,0097,0098 |
| M-H | 682.2094 | 13.509 | 0095,0096,0097 |
| M-H | 682.2128 | 12.727 | 0095,0096,0097,0098 |
| M+H | 686.2102 | 12.396 | 0099 |
| M+H | 687.1571 | 11.938 | 0100 |
| M+H | 687.1849 | 12.661 | 0101 |
| M+H | 688.2574 | 8.25 | 0102 |
| M+H | 689.2089 | 9.898 | 0103 |
| M+H | 689.2533 | 13.321 | 0104 |
| M+H | 689.2786 | 13.633 | 0105 |
| M+H | 690.1836 | 13.064 | 0106,0107 |
| M+H | 690.1837 | 12.921 | 0106,0107 |
| M+H | 690.2488 | 12.368 | 0108 |
| M-H | 689.1655 | 13.271 | 0109,0110 |
| M+H | 691.1826 | 13.675 | 0109,0110 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M-H | 689.2088 | 11.848 | 0111 |
| M-H | 689.2185 | 12.903 | 0112 |
| M+H | 691.2563 | 13.245 | 0113 |
| M+H | 693.2035 | 11.614 | 0114 |
| M+H | 693.2490 | 12.867 | 0115 |
| M+H | 693.2532 | 13.341 | 0115,0116 |
| M+H | 695.2058 | 12.805 | 0117,0118 |
| M+H | 695.2074 | 12.458 | 0117,0118 |
| M+H | 695.2352 | 13.437 | 0119 |
| M+H | 695.2518 | 10.715 | 0120 |
| M+H | 696.1403 | 13.043 | 0121 |
| M-H | 694.1777 | 10.363 | 0122 |
| M+H | 697.1531 | 9.555 | 0123 |
| M+H | 697.1778 | 12.342 | 0124,0125 |
| M+H | 697.1774 | 12.156 | 0124,0125 |
| M+H | 697.2237 | 12.414 | 0126 |
| M+H | 697.2293 | 12.93 | 0127 |
| M+H | 697.2451 | 1.581 | 0128,0129 |
| M-H | 695.2298 | 13.789 | 0128,0129 |
| M+H | 698.1734 | 11.184 | 0130 |
| M+H | 698.2430 | 14.149 | 0131,0132,0133 |
| M+H | 698.2428 | 13.902 | 0131,0132,0133 |
| M+H | 698.2426 | 12.994 | 0131,0132,0133 |
| M+H | 699.2094 | 13.174 | 0134 |
| M-H | 698.1510 | 12.74 | 0135 |
| M+H | 701.1730 | 12.442 | 0136 |
| M+H | 701.2036 | 13.42 | 0137,0138 |
| M-H | 699.1864 | 13.618 | 0137,0138 |
| M+H | 701.2222 | 13.358 | 0139,0140 |
| M-H | 699.2044 | 13.458 | 0139,0140 |
| M+H | 701.2787 | 13.55 | 0141 |
| M+H | 702.1989 | 12.62 | 0142 |
| M+H | 702.2187 | 15.294 | 0143,0144,0145 |
| M+H | 702.2189 | 8.655 | 0143,0144,0145 |
| M+H | 702.2160 | 15.466 | 0143,0144,0145 |
| M+H | 702.2718 | 7.734 | 0146 |
| M+H | 703.1339 | 12.062 | 0147 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 703.2207 | 13.548 | 0148 |
| M+H | 703.2728 | 8.559 | 0149 |
| M-H | 702.1302 | 11.921 | 0150 |
| M+H | 704.2651 | 12.801 | 0151 |
| M+H | 705.1451 | 12.162 | 0152 |
| M-H | 703.2241 | 12.247 | 0153 |
| M+H | 705.2733 | 13.441 | 0154 |
| M+H | 706.1787 | 12.995 | 0155 |
| M+H | 706.2463 | 12.45 | 0156 |
| M+H | 707.1596 | 13.566 | 0157 |
| M+H | 707.1957 | 13.083 | 0158 |
| M+H | 707.2201 | 11.995 | 0159 |
| M+H | 707.2380 | 13.418 | 0160 |
| M+H | 707.2419 | 13.387 | 0161 |
| M+H | 708.1687 | 12.402 | 0162,0163 |
| M+H | 708.1729 | 13.115 | 0162,0163 |
| M+H | 708.2365 | 12.849 | 0164 |
| M-H | 707.1987 | 8.865 | 0165 |
| M+H | 709.2251 | 8.414 | 0166 |
| M+H | 709.2476 | 13.117 | 0167 |
| M+H | 710.1854 | 13.671 | 0168 |
| M+H | 710.2472 | 13.124 | 0169 |
| M+H | 711.1936 | 12.537 | 0170,0171,0172 |
| M+H | 711.1923 | 13.563 | 0170,0171,0172 |
| M-H | 709.1781 | 11.714 | 0170,0171,0172 |
| M+H | 712.1886 | 11.655 | 0173 |
| M+H | 712.2252 | 12.762 | 0174 |
| M+H | 713.1304 | 12.944 | 0175 |
| M+H | 713.1728 | 12.265 | 0176 |
| M+H | 713.2016 | 13.494 | 0177 |
| M+H | 714.1837 | 13.562 | 0178,0179 |
| M+H | 714.1838 | 13.825 | 0178,0179 |
| M+H | 715.1626 | 11.93 | 0180 |
| M+H | 715.1678 | 12.085 | 0180,0181,0182 |
| M+H | 715.1702 | 11.333 | 0181,0182 |
| M+H | 715.1790 | 12.821 | 0183,0184,0185 |
| M+H | 715.1802 | 13.776 | 0183,0184,0185 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 715.1802 | 13.807 | 0183,0184,0185 |
| M+H | 715.2241 | 12.046 | 0186 |
| M+H | 715.2937 | 13.926 | 0187 |
| M+H | 716.1641 | 11.365 | 0188 |
| M+H | 716.1995 | 13.312 | 0189,0190 |
| M+H | 716.1997 | 13.144 | 0189,0190 |
| M+H | 717.1496 | 12.492 | 0191 |
| M+H | 717.1786 | 11.75 | 0192 |
| M-H | 715.1812 | 13.496 | 0193,0194 |
| M+H | 717.1959 | 13.544 | 0193,0194 |
| M-H | 715.2241 | 12.311 | 0195 |
| M+H | 717.2850 | 12.377 | 0196 |
| M-H | 716.1468 | 12.305 | 0197 |
| M-H | 717.1720 | 13.03 | 0198 |
| M+H | 719.2093 | 13.026 | 0199 |
| M-H | 717.2025 | 12.05 | 0200 |
| M+H | 719.2695 | 13.626 | 0201 |
| M-H | 718.1083 | 12.111 | 0202 |
| M+H | 720.2057 | 13.488 | 0203 |
| M+H | 721.1245 | 12.223 | 0204 |
| M+H | 721.1779 | 12.676 | 0205,0206 |
| M+H | 721.1778 | 12.586 | 0205,0206 |
| M+H | 721.2006 | 12.782 | 0207,0208 |
| M+H | 721.2027 | 11.486 | 0207,0208 |
| M+H | 721.2490 | 8.169 | 0209 |
| M+H | 721.2643 | 13.74 | 0210 |
| M-H | 720.1212 | 12.042 | 0211 |
| M+H | 722.1561 | 13.305 | 0212 |
| M+H | 722.1731 | 12.494 | 0213,0214,0215 |
| M+H | 722.1720 | 12.756 | 0213,0214,0215 |
| M+H | 722.1729 | 10.631 | 0213,0214,0215 |
| M+H | 722.2104 | 13.143 | 0216 |
| M-H | 721.1533 | 13.077 | 0217 |
| M+H | 723.1960 | 9.578 | 0218,0219 |
| M+H | 723.1954 | 6.866 | 0218,0219 |
| M+H | 723.2446 | 13.323 | 0220 |
| M+H | 724.1508 | 13.658 | 0221 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 724.1891 | 11.495 | 0222 |
| M+H | 725.2031 | 14.113 | 0223,0224 |
| M+H | 725.2028 | 14.021 | 0223,0224 |
| M+H | 725.2254 | 13.453 | 0225 |
| M-H | 724.1445 | 13.077 | 0226 |
| M+H | 726.1988 | 14.246 | 0227,0228,0229 |
| M+H | 726.1992 | 14.504 | 0227,0228,0229 |
| M+H | 726.1994 | 13.334 | 0227,0228,0229 |
| M+H | 726.2045 | 12.011 | 0230 |
| M+H | 727.1892 | 12.68 | 0231 |
| M+H | 727.1988 | 12.604 | 0232 |
| M+H | 727.2187 | 13.775 | 0233,0234 |
| M+H | 727.2184 | 13.691 | 0233,0234 |
| M+H | 728.1953 | 11.591 | 0235 |
| M+H | 728.2147 | 12.889 | 0236 |
| M-H | 727.1339 | 10.653 | 0237 |
| M-H | 727.1638 | 12.316 | 0238 |
| M-H | 727.1876 | 12.228 | 0239 |
| M+H | 729.2392 | 12.771 | 0240 |
| M+H | 730.1636 | 9.583 | 0241 |
| M+H | 730.2307 | 13.788 | 0242 |
| M+H | 731.1639 | 12.345 | 0243 |
| M+H | 731.1950 | 12.138 | 0244 |
| M+H | 731.2252 | 13.993 | 0245 |
| M+H | 731.2565 | 12.818 | 0246 |
| M+H | 731.3002 | 12.741 | 0247 |
| M+H | 732.2207 | 13.278 | 0248 |
| M+H | 732.2303 | 13.768 | 0249 |
| M+H | 733.1564 | 12.18 | 0250,0251 |
| M-H | 731.1389 | 12.065 | 0250,0251 |
| M-H | 731.1576 | 13.816 | 0252 |
| M+H | 733.2116 | 13.17 | 0253 |
| M-H | 731.2150 | 13.693 | 0254 |
| M+H | 733.2356 | 12.422 | 0255 |
| M+H | 734.1396 | 12.495 | 0256 |
| M+H | 734.1846 | 12.785 | 0257,0258 |
| M+H | 734.1886 | 13.358 | 0257,0258 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M-H | 733.1525 | 11.876 | 0259 |
| M+H | 735.1940 | 13.612 | 0260,0261 |
| M+H | 735.1940 | 13.082 | 0260,0261 |
| M+H | 735.2296 | 11.029 | 0262 |
| M+H | 735.2749 | 12.479 | 0263 |
| M+H | 736.1901 | 13.13 | 0264,0265,0266 |
| M+H | 736.1902 | 13.25 | 0264,0265,0266 |
| M+H | 736.1919 | 13.485 | 0264,0265,0266 |
| M+H | 736.2015 | 10.763 | 0267 |
| M+H | 737.1823 | 13.61 | 0268 |
| M+H | 737.2100 | 12.108 | 0269,0270,0271 |
| M+H | 737.2129 | 10.619 | 0269,0270,0271 |
| M-H | 735.1937 | 12.147 | 0269,0270,0271 |
| M-H | 736.0983 | 12.224 | 0272 |
| M+H | 738.1868 | 13.665 | 0273 |
| M+H | 738.2048 | 11.996 | 0274 |
| M+H | 739.1466 | 13.109 | 0275 |
| M+H | 739.1677 | 12.776 | 0276,0277 |
| M-H | 737.1511 | 12.641 | 0276,0277 |
| M+H | 739.2242 | 12.718 | 0278 |
| M+H | 740.1644 | 12.658 | 0279,0280,0281 |
| M+H | 740.1639 | 12.853 | 0279,0280,0281 |
| M-H | 738.1464 | 12.574 | 0279,0280,0281 |
| M+H | 740.1982 | 13.844 | 0282,0283 |
| M+H | 740.1971 | 13.727 | 0282,0283 |
| M+H | 740.2198 | 7.727 | 0284 |
| M-H | 739.1629 | 12.315 | 0285 |
| M+H | 741.1878 | 10.745 | 0286,0287 |
| M+H | 741.1840 | 12.535 | 0285,0286,0287 |
| M+H | 741.1942 | 13.97 | 0288,0289,0290 |
| M+H | 741.1942 | 14.077 | 0288,0289,0290 |
| M-H | 739.1770 | 14.214 | 0288,0289,0290 |
| M+H | 741.2154 | 12.998 | 0291 |
| M+H | 742.1793 | 11.651 | 0292 |
| M+H | 742.2113 | 11.923 | 0293 |
| M+H | 743.1661 | 10.824 | 0294 |
| M+H | 743.1946 | 12.095 | 0295 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 743.2197 | 12.581 | 0296 |
| M+H | 743.2501 | 14.155 | 0297 |
| M+H | 743.3009 | 12.4 | 0298 |
| M+H | 744.1245 | 12.405 | 0299 |
| M+H | 744.1801 | 10.113 | 0300 |
| M+H | 744.1954 | 11.391 | 0301 |
| M-H | 743.1639 | 12.454 | 0302 |
| M+H | 745.1899 | 12.672 | 0303 |
| M+H | 745.2043 | 13.428 | 0304 |
| M+H | 745.2262 | 13.277 | 0305 |
| M-H | 744.1227 | 7.219 | 0306 |
| M+H | 746.1739 | 13.52 | 0307 |
| M+H | 746.1853 | 11.662 | 0308 |
| M+H | 746.2209 | 13.827 | 0309 |
| M+H | 747.1417 | 9.139 | 0310 |
| M+H | 747.1716 | 12.601 | 0311 |
| M-H | 745.1792 | 12.332 | 0312,0313,0314 |
| M+H | 747.1924 | 12.867 | 0312,0313,0314 |
| M-H | 745.1769 | 12.929 | 0312,0313,0314 |
| M-H | 745.1987 | 14.298 | 0315 |
| M+H | 747.2209 | 13.372 | 0315,0316 |
| M+H | 748.1522 | 12.532 | 0317 |
| M+H | 748.1897 | 7.531 | 0318,0319,0320,0321 |
| M+H | 748.1884 | 13.024 | 0318,0319,0320,0321 |
| M+H | 748.1902 | 12.862 | 0318,0319,0320,0321 |
| M+H | 748.1923 | 12.346 | 0318,0319,0320,0321 |
| M+H | 748.2253 | 13.572 | 0322 |
| M+H | 749.2066 | 13.999 | 0323 |
| M-H | 748.1542 | 12.123 | 0324,0325 |
| M+H | 750.1738 | 13.277 | 0325 |
| M+H | 751.1460 | 12.305 | 0326 |
| M+H | 751.2198 | 14.293 | 0327,0328 |
| M+H | 751.2230 | 12.607 | 0327,0328 |
| M-H | 750.1591 | 13.28 | 0329 |
| M-H | 750.1956 | 14.651 | 0330,0331,0332 |
| M+H | 752.2153 | 14.618 | 0330,0331,0332 |
| M+H | 752.2160 | 7.71 | 0330,0331,0332,0333 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 752.2184 | 8.152 | 0330,0331,0332,0333 |
| M-H | 751.1498 | 12.643 | 0334 |
| M+H | 753.2184 | 14.614 | 0335 |
| M+H | 753.2399 | 13.071 | 0336 |
| M+H | 754.1450 | 12.636 | 0337,0338 |
| M-H | 752.1269 | 12.452 | 0337,0338 |
| M+H | 754.2105 | 11.83 | 0339 |
| M+H | 755.1401 | 11.791 | 0340 |
| M-H | 753.1267 | 12.976 | 0340,0341 |
| M+H | 755.1982 | 11.691 | 0342 |
| M-H | 753.2036 | 10.913 | 0343 |
| M+H | 756.2460 | 13.756 | 0344 |
| M-H | 755.1747 | 11.812 | 0345 |
| M-H | 755.1988 | 13.337 | 0346,0347 |
| M-H | 755.1993 | 12.812 | 0346,0347 |
| M+H | 757.2412 | 14.3 | 0348 |
| M+H | 757.3165 | 12.798 | 0349 |
| M+H | 758.2358 | 13.476 | 0350 |
| M+H | 758.2461 | 14.059 | 0351 |
| M+H | 759.1724 | 12.527 | 0352,0353 |
| M+H | 759.1724 | 12.49 | 0352,0353 |
| M+H | 759.1982 | 12.799 | 0354 |
| M-H | 757.1944 | 12.88 | 0355 |
| M+H | 759.2459 | 8.177 | 0356 |
| M+H | 760.1029 | 12.593 | 0357 |
| M+H | 760.1567 | 12.793 | 0358 |
| M+H | 761.1151 | 12.441 | 0359 |
| M+H | 761.1855 | 12.173 | 0360 |
| M+H | 761.1920 | 12.92 | 0361 |
| M+H | 761.2091 | 13.346 | 0362,0363 |
| M+H | 761.2087 | 13.235 | 0362,0363 |
| M+H | 761.2908 | 12.487 | 0364 |
| M-H | 760.1872 | 13.909 | 0365,0366,0367 |
| M+H | 762.2055 | 14.042 | 0365,0366,0367 |
| M-H | 760.1874 | 13.424 | 0365,0366,0367 |
| M-H | 761.1547 | 12.548 | 0368 |
| M+H | 763.1965 | 13.784 | 0369 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 764.1295 | 12.493 | 0370 |
| M+H | 764.2030 | 13.906 | 0371 |
| M+H | 765.1647 | 13.167 | 0372,0373 |
| M-H | 763.1516 | 11.978 | 0372,0373 |
| M+H | 765.1825 | 13.022 | 0374,0375 |
| M-H | 763.1694 | 12.818 | 0374,0375 |
| M+H | 765.2417 | 12.994 | 0376 |
| M+H | 766.1608 | 12.524 | 0377 |
| M+H | 766.1805 | 13.049 | 0378,0379,0380 |
| M+H | 766.1805 | 13.088 | 0378,0379,0380 |
| M+H | 766.1816 | 10.816 | 0378,0379,0380 |
| M+H | 766.2366 | 9.205 | 0381 |
| M+H | 767.1855 | 13.46 | 0382 |
| M+H | 767.2303 | 6.977 | 0383 |
| M+H | 768.2263 | 12.243 | 0384 |
| M-H | 767.2171 | 12.993 | 0385 |
| M+H | 769.2670 | 14.459 | 0386 |
| M+H | 770.1401 | 12.73 | 0387 |
| M+H | 770.2097 | 11.047 | 0388 |
| M-H | 769.1052 | 13.172 | 0389 |
| M+H | 771.1597 | 8.574 | 0390 |
| M-H | 769.1810 | 12.819 | 0391 |
| M+H | 771.2061 | 12.946 | 0392 |
| M-H | 770.1148 | 12.45 | 0393,0394 |
| M+H | 772.1381 | 13.344 | 0394 |
| M+H | 772.1885 | 13.63 | 0395 |
| M-H | 770.1848 | 11.929 | 0396 |
| M+H | 773.1860 | 11.525 | 0397 |
| M+H | 773.2088 | 12.696 | 0398 |
| M+H | 774.1467 | 12.256 | 0399 |
| M+H | 774.2079 | 12.829 | 0400 |
| M+H | 774.2515 | 12.952 | 0401 |
| M+H | 775.2217 | 14.325 | 0402 |
| M-H | 774.1703 | 12.613 | 0403 |
| M+H | 777.0919 | 12.614 | 0404 |
| M+H | 777.1600 | 12.55 | 0405 |
| M+H | 778.1453 | 13.035 | 0406,0407 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 778.1454 | 13.142 | 0406,0407 |
| M+H | 779.1413 | 13.203 | 0408,0409,0410 |
| M+H | 779.1404 | 13.041 | 0408,0409,0410 |
| M+H | 779.1415 | 13.307 | 0408,0409,0410 |
| M+H | 779.1839 | 12.852 | 0411 |
| M+H | 779.2556 | 13.42 | 0412 |
| M+H | 780.1606 | 12.768 | 0413,0414 |
| M+H | 780.1607 | 12.949 | 0413,0414 |
| M+H | 781.1557 | 12.131 | 0415 |
| M+H | 781.1632 | 12.069 | 0416 |
| M+H | 781.2463 | 11.902 | 0417 |
| M-H | 781.1353 | 12.413 | 0418 |
| M+H | 783.1723 | 12.507 | 0419 |
| M+H | 783.2081 | 14.035 | 0420 |
| M-H | 781.2134 | 13.089 | 0421 |
| M+H | 784.1674 | 13.105 | 0422 |
| M+H | 785.1619 | 12.068 | 0423 |
| M+H | 785.1665 | 13.624 | 0423,0424 |
| M+H | 785.2122 | 11.418 | 0425 |
| M-H | 783.2101 | 13.376 | 0426 |
| M+H | 785.2716 | 13.524 | 0427 |
| M+H | 786.1172 | 12.927 | 0428 |
| M+H | 786.1686 | 13.712 | 0429 |
| M+H | 787.1305 | 12.745 | 0430 |
| M-H | 785.1897 | 13.092 | 0431 |
| M-H | 786.0970 | 12.949 | 0432 |
| M-H | 787.1478 | 13.65 | 0433,0434 |
| M-H | 787.1478 | 13.519 | 0433,0434 |
| M+H | 789.1867 | 12.879 | 0435 |
| M+H | 790.1235 | 12.678 | 0436 |
| M-H | 788.1430 | 13.936 | 0437,0438,0439 |
| M-H | 788.1428 | 14.805 | 0437,0438,0439 |
| M-H | 788.1428 | 14.025 | 0437,0438,0439 |
| M-H | 789.1634 | 13.47 | 0440,0441 |
| M+H | 791.1799 | 13.308 | 0440,0441 |
| M+H | 792.1764 | 12.86 | 0442 |
| M+H | 793.1990 | 13.207 | 0443 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M+H | 794.1918 | 12.995 | 0444 |
| M+H | 795.1869 | 13.595 | 0445 |
| M+H | 795.2618 | 12.255 | 0446 |
| M+H | 796.1820 | 12.608 | 0447 |
| M+H | 796.1925 | 13.207 | 0448 |
| M+H | 797.1370 | 13.505 | 0449 |
| M+H | 797.1742 | 12.943 | 0450 |
| M+H | 797.1919 | 13.12 | 0451 |
| M+H | 798.1465 | 12.797 | 0452,0453 |
| M+H | 798.1494 | 13.305 | 0452,0453 |
| M+H | 799.2379 | 12.012 | 0454 |
| M+H | 800.1628 | 12.578 | 0455 |
| M+H | 800.2677 | 13.049 | 0456 |
| M+H | 801.1429 | 13.198 | 0457 |
| M+H | 802.1487 | 12.967 | 0458 |
| M+H | 803.1078 | 12.845 | 0459 |
| M+H | 804.1612 | 13.401 | 0460,0461 |
| M+H | 804.1594 | 13.3 | 0460,0461 |
| M+H | 805.1561 | 13.46 | 0462,0463,0464 |
| M-H | 803.1396 | 13.413 | 0462,0463,0464 |
| M+H | 805.1562 | 13.57 | 0462,0463,0464 |
| M-H | 805.1953 | 13.645 | 0465 |
| M+H | 807.2626 | 12.006 | 0466 |
| M-H | 807.1547 | 12.336 | 0467 |
| M+H | 809.1872 | 12.825 | 0468 |
| M+H | 810.1354 | 13.157 | 0469 |
| M+H | 810.1826 | 13.387 | 0470 |
| M+H | 811.1782 | 12.386 | 0471,0472 |
| M+H | 811.1813 | 13.15 | 0471,0472 |
| M+H | 811.2874 | 13.502 | 0473 |
| M+H | 812.1870 | 13.926 | 0474 |
| M-H | 811.1510 | 13.419 | 0475 |
| M-H | 812.1113 | 13.419 | 0476 |
| M-H | 813.1621 | 13.912 | 0477,0478 |
| M+H | 815.1815 | 13.814 | 0477,0478 |
| M+H | 816.1388 | 12.974 | 0479 |
| M+H | 816.1775 | 10.798 | 0480,0481,0482 |

(continued)

| [Table 9-8-3] Compound that may be contained in mixture 2-2-dlE03-l | | | |
|---|---|---|---|
| Ion species | m/z [M+H]+ or [M-H]- | Retention time of MS peak (min) | No. of assigned compound (described with 2-2-d1E03-1- omitted) |
| M-H | 814.1592 | 14.106 | 0480,0481,0482 |
| M+H | 816.1773 | 10.462 | 0480,0481,0482 |
| M+H | 820.2076 | 13.3 | 0483 |
| M-H | 819.1384 | 13.69 | 0484 |
| M+H | 821.2020 | 13.832 | 0485 |
| M+H | 821.2775 | 12.402 | 0486 |
| M+H | 822.1980 | 12.904 | 0487 |
| M+H | 822.2082 | 13.565 | 0488 |
| M-H | 821.1895 | 13.625 | 0489 |
| M+H | 825.2523 | 12.115 | 0490 |
| M+H | 827.1592 | 13.472 | 0491 |
| M+H | 828.1657 | 13.347 | 0492 |
| M-H | 831.2097 | 14.013 | 0493 |
| M+H | 836.1506 | 13.39 | 0494 |
| M+H | 838.2141 | 12.441 | 0495 |
| M-H | 837.1664 | 13.816 | 0496 |
| M-H | 845.1518 | 14.01 | 0497 |
| M+H | 849.2337 | 12.896 | 0498 |
| M+H | 864.2295 | 12.63 | 0499 |
| M+H | 875.2540 | 14.509 | 0500 |

[Table 507]

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 736.2802 | 7.6 | 0001 |
| M+H | 740.3473 | 7.514 | 0002 |
| M+H | 742.3270 | 7.277 | 0003 |
| M+H | 746.3006 | 7.742 | 0004,0005 |
| M+H | 746.3006 | 7.816 | 0004,0005 |
| M+H | 747.2957 | 7.113 | 0006 |
| M+H | 750.2954 | 7.893 | 0007 |
| M-H | 750.2397 | 7.835 | 0008 |
| M+H | 753.2712 | 6.844 | 0009 |
| M+H | 754.2700 | 7.707 | 0010 |
| M+H | 754.3627 | 7.84 | 0011 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 756.3422 | 7.589 | 0012 |
| M+H | 758.3381 | 7.664 | 0013 |
| M+H | 760.3166 | 8.106 | 0014,0015,0016 |
| M+H | 760.3173 | 7.407 | 0014,0015,0016 |
| M+H | 760.3165 | 8.046 | 0014,0015,0016 |
| M+H | 761.3117 | 7.416 | 0017 |
| M+H | 762.2957 | 7.734 | 0018 |
| M+H | 764.2858 | 7.316 | 0019 |
| M+H | 764.2909 | 7.928 | 0019,0020,0021 |
| M+H | 764.2909 | 7.863 | 0019,0020,0021 |
| M+H | 765.2864 | 7.236 | 0022 |
| M+H | 766.2721 | 8.12 | 0023 |
| M+H | 766.3019 | 7.486 | 0024 |
| M+H | 766.3627 | 7.875 | 0025 |
| M+H | 767.2853 | 7.774 | 0026 |
| M+H | 768.3418 | 7.532 | 0027 |
| M+H | 769.2470 | 7.726 | 0028 |
| M+H | 770.2473 | 7.945 | 0029 |
| M+H | 770.3005 | 8.372 | 0030,0031 |
| M+H | 770.3007 | 8.324 | 0030,0031 |
| M+H | 771.2600 | 7.586 | 0032 |
| M+H | 771.2958 | 8.482 | 0033,0034,0035 |
| M+H | 771.2954 | 8.603 | 0033,0034,0035 |
| M+H | 771.2957 | 6.937 | 0033,0034,0035 |
| M+H | 772.3158 | 7.971 | 0036,0037 |
| M+H | 772.3135 | 6.25 | 0036,0037 |
| M+H | 773.3115 | 7.308 | 0038 |
| M+H | 775.3271 | 8.024 | 0039 |
| M+H | 776.3121 | 7.075 | 0040 |
| M+H | 776.3217 | 8.073 | 0041 |
| M+H | 777.3174 | 7.499 | 0042 |
| M+H | 778.2706 | 9.092 | 0043 |
| M+H | 778.3018 | 7.614 | 0044 |
| M+H | 778.3266 | 7.999 | 0045 |
| M+H | 779.2850 | 7.689 | 0046 |
| M+H | 780.2860 | 7.85 | 0047 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 780.3170 | 7.755 | 0048 |
| M+H | 780.3778 | 8.203 | 0049 |
| M+H | 782.2792 | 7.791 | 0050,0051 |
| M+H | 782.2760 | 7.399 | 0050,0051 |
| M+H | 782.3578 | 7.834 | 0052 |
| M+H | 783.2622 | 8.002 | 0053 |
| M+H | 784.2924 | 7.987 | 0054 |
| M+H | 784.3155 | 8.639 | 0055,0056 |
| M+H | 784.3169 | 8.939 | 0055,0056 |
| M+H | 784.3529 | 7.951 | 0057 |
| M+H | 785.3111 | 8.029 | 0058,0059,0060 |
| M+H | 785.3113 | 8.767 | 0058,0059,0060 |
| M+H | 785.3121 | 8.612 | 0058,0059,0060 |
| M+H | 786.3332 | 7.621 | 0061,0062,0063 |
| M+H | 786.3322 | 8.265 | 0061,0062,0063 |
| M+H | 786.3333 | 7.531 | 0061,0062,0063 |
| M+H | 787.2374 | 7.813 | 0064 |
| M+H | 787.3274 | 7.608 | 0065 |
| M+H | 788.2911 | 8.492 | 0066,0067 |
| M+H | 788.2910 | 8.435 | 0066,0067 |
| M+H | 788.3474 | 8.415 | 0068 |
| M+H | 789.2861 | 7.852 | 0069,0070,0071 |
| M+H | 789.2858 | 8.588 | 0069,0070,0071 |
| M+H | 789.2850 | 8.764 | 0069,0070,0071 |
| M+H | 789.3431 | 8.129 | 0072 |
| M+H | 790.3011 | 7.29 | 0073 |
| M+H | 790.3057 | 8.076 | 0073,0074,0075 |
| M+H | 790.3043 | 7.504 | 0073,0074,0075 |
| M+H | 790.3381 | 8.368 | 0076 |
| M+H | 791.3023 | 7.277 | 0077 |
| M+H | 791.3327 | 7.804 | 0078 |
| M+H | 792.2900 | 8.344 | 0079 |
| M+H | 792.3165 | 7.686 | 0080 |
| M+H | 792.3423 | 8.264 | 0081 |
| M+H | 793.2469 | 7.998 | 0082 |
| M+H | 793.3036 | 7.735 | 0083 |
| M+H | 793.3148 | 4.088 | 0084 |
| M+H | 794.3039 | 8.305 | 0085 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 794.3121 | 8.168 | 0086 |
| M+H | 795.2634 | 7.907 | 0087 |
| M+H | 795.3078 | 7.623 | 0088 |
| M+H | 796.2634 | 8.152 | 0089 |
| M+H | 796.2937 | 8.073 | 0090 |
| M+H | 796.3160 | 8.5 | 0091,0092,0093 |
| M+H | 796.3167 | 8.098 | 0091,0092,0093 |
| M+H | 796.3166 | 9.029 | 0091,0092,0093 |
| M+H | 797.2760 | 7.736 | 0094 |
| M+H | 797.3141 | 7.925 | 0095,0096,0097,0098 |
| M+H | 797.3108 | 8.628 | 0095,0096,0097,0098 |
| M+H | 797.3132 | 8.016 | 0095,0096,0097,0098 |
| M+H | 797.3141 | 7.969 | 0095,0096,0097,0098 |
| M+H | 799.2930 | 7.67 | 0099 |
| M+H | 800.2411 | 7.425 | 0100 |
| M+H | 800.2691 | 7.886 | 0101 |
| M+H | 801.3455 | 4.674 | 0102 |
| M+H | 802.2928 | 7.211 | 0103 |
| M+H | 802.3373 | 7.418 | 0104 |
| M+H | 802.3624 | 8.683 | 0105 |
| M+H | 803.2683 | 8.243 | 0106,0107 |
| M+H | 803.2684 | 8.191 | 0106,0107 |
| M+H | 803.3327 | 7.646 | 0108 |
| M+H | 804.2632 | 7.541 | 0109,0110 |
| M+H | 804.2684 | 8.462 | 0110 |
| M+H | 804.3086 | 7.427 | 0111 |
| M+H | 804.3167 | 7.807 | 0112 |
| M+H | 804.3419 | 8.192 | 0113 |
| M+H | 806.2879 | 8.12 | 0114 |
| M+H | 806.3326 | 7.993 | 0115 |
| M+H | 806.3382 | 8.508 | 0116 |
| M+H | 808.2929 | 7.606 | 0117,0118 |
| M+H | 808.2972 | 7.633 | 0117,0118 |
| M+H | 808.3198 | 8.595 | 0119 |
| M+H | 808.3350 | 8.425 | 0120 |
| M+H | 809.2241 | 8.251 | 0121 |
| M+H | 809.2769 | 8.113 | 0122 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 810.2367 | 7.916 | 0123 |
| M+H | 810.2626 | 7.514 | 0124,0125 |
| M+H | 810.2626 | 7.607 | 0124,0125 |
| M+H | 810.3076 | 7.734 | 0126 |
| M+H | 810.3142 | 8.205 | 0127 |
| M+H | 810.3316 | 8.798 | 0128,0129 |
| M+H | 810.3315 | 8.74 | 0128,0129 |
| M-H | 809.2400 | 6.732 | 0130 |
| M+H | 811.3277 | 8.749 | 0131,0132,0133 |
| M+H | 811.3263 | 8.929 | 0131,0132,0133 |
| M+H | 811.3274 | 8.216 | 0131,0132,0133 |
| M+H | 812.2948 | 8.395 | 0134 |
| M+H | 813.2527 | 7.903 | 0135 |
| M+H | 814.2574 | 8.529 | 0136 |
| M-H | 812.2705 | 8.658 | 0137,0138 |
| M+H | 814.2896 | 9.192 | 0137,0138 |
| M+H | 814.3064 | 6.132 | 0139,0140 |
| M+H | 814.3055 | 9.13 | 0139,0140 |
| M+H | 815.2863 | 8.007 | 0142 |
| M+H | 815.3013 | 8.72 | 0143,0144,0145 |
| M-H | 813.2852 | 8.493 | 0143,0144,0145 |
| M+H | 815.3016 | 8.645 | 0143,0144,0145 |
| M-H | 813.3408 | 7.403 | 0146 |
| M+H | 816.2186 | 7.568 | 0147 |
| M+H | 816.3056 | 8.483 | 0148 |
| M+H | 816.3564 | 5.973 | 0149 |
| M+H | 817.2311 | 7.46 | 0150 |
| M+H | 817.3483 | 7.962 | 0151 |
| M+H | 818.2307 | 7.56 | 0152 |
| M-H | 816.3078 | 7.743 | 0153 |
| M+H | 818.3583 | 8.465 | 0154 |
| M+H | 819.2622 | 8.16 | 0155 |
| M+H | 819.3342 | 8.678 | 0156 |
| M+H | 820.2439 | 8.71 | 0157 |
| M+H | 820.2810 | 8.313 | 0158 |
| M+H | 820.3042 | 7.523 | 0159 |
| M+H | 820.3167 | 9.44 | 0160 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 820.3285 | 8.362 | 0161 |
| M+H | 821.2572 | 8.344 | 0162,0163 |
| M+H | 821.2535 | 7.649 | 0162,0163 |
| M+H | 821.3240 | 7.755 | 0164 |
| M-H | 820.2830 | 7.406 | 0165 |
| M-H | 820.2919 | 5.866 | 0166 |
| M+H | 822.3330 | 8.272 | 0167 |
| M+H | 823.2680 | 7.906 | 0168 |
| M+H | 823.3324 | 7.299 | 0169 |
| M+H | 824.2776 | 7.922 | 0170,0171,0172 |
| M+H | 824.2796 | 7.343 | 0170,0171,0172 |
| M+H | 824.2776 | 7.826 | 0170,0171,0172 |
| M+H | 825.2731 | 7.103 | 0173 |
| M+H | 825.3088 | 7.962 | 0174 |
| M+H | 826.2141 | 8.129 | 0175 |
| M+H | 826.2576 | 7.153 | 0176 |
| M+H | 826.2839 | 8.021 | 0177 |
| M+H | 827.2679 | 8.766 | 0178,0179 |
| M-H | 825.2510 | 8.809 | 0178,0179 |
| M-H | 826.2305 | 7.308 | 0180 |
| M+H | 828.2529 | 7.727 | 0180,0181,0182 |
| M-H | 826.2353 | 7.657 | 0180,0181,0182 |
| M+H | 828.2623 | 8.16 | 0183,0184,0185 |
| M+H | 828.2623 | 8.902 | 0183,0184,0185 |
| M+H | 828.2623 | 8.198 | 0183,0184,0185 |
| M+H | 828.3057 | 8.271 | 0186 |
| M+H | 828.3793 | 8.856 | 0187 |
| M+H | 829.2476 | 6.898 | 0188 |
| M+H | 829.2843 | 8.402 | 0189,0190 |
| M-H | 827.2678 | 7.406 | 0189,0190 |
| M+H | 830.2345 | 7.891 | 0191 |
| M+H | 830.2632 | 7.302 | 0192 |
| M+H | 830.2780 | 7.763 | 0193,0194 |
| M+H | 830.2830 | 8.616 | 0193,0194 |
| M+H | 830.3246 | 7.685 | 0195 |
| M+H | 830.3689 | 7.811 | 0196 |
| M+H | 831.2499 | 8.493 | 0197 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 832.2701 | 12.527 | 0198 |
| M-H | 830.2816 | 8.428 | 0199 |
| M+H | 832.3039 | 7.495 | 0200 |
| M+H | 832.3538 | 8.657 | 0201 |
| M+H | 833.2082 | 7.573 | 0202 |
| M+H | 833.2895 | 8.473 | 0203 |
| M+H | 834.2088 | 7.708 | 0204 |
| M+H | 834.2628 | 8.141 | 0205,0206 |
| M+H | 834.2620 | 9.083 | 0205,0206 |
| M+H | 834.2842 | 8.002 | 0207,0208 |
| M+H | 834.2885 | 8.336 | 0207,0208 |
| M+H | 834.3382 | 8.745 | 0209 |
| M+H | 834.3504 | 8.785 | 0210 |
| M+H | 835.2210 | 7.591 | 0211 |
| M+H | 835.2397 | 8.493 | 0212 |
| M+H | 835.2563 | 7.754 | 0213,0214,0215 |
| M+H | 835.2579 | 7.947 | 0213,0214,0215 |
| M+H | 835.2579 | 7.924 | 0213,0214,0215 |
| M+H | 835.2938 | 8.419 | 0216 |
| M-H | 834.2357 | 8.022 | 0217 |
| M+H | 836.2773 | 7.818 | 0218,0219 |
| M+H | 836.2799 | 7.099 | 0218,0219 |
| M+H | 836.3297 | 8.452 | 0220 |
| M+H | 837.2341 | 8.578 | 0221 |
| M+H | 837.2728 | 7.011 | 0222 |
| M+H | 838.2884 | 9.184 | 0223,0224 |
| M+H | 838.2885 | 9.111 | 0223,0224 |
| M+H | 838.3097 | 8.561 | 0225 |
| M+H | 839.2455 | 8.198 | 0226 |
| M+H | 839.2827 | 8.605 | 0227,0228,0229 |
| M+H | 839.2834 | 9.329 | 0227,0228,0229 |
| M-H | 837.2706 | 7.567 | 0227,0228,0229,0230 |
| M+H | 839.2911 | 7.615 | 0230 |
| M+H | 840.2728 | 8.019 | 0231 |
| M+H | 840.2838 | 7.731 | 0232 |
| M+H | 840.3029 | 8.842 | 0233,0234 |
| M+H | 840.3028 | 8.892 | 0233,0234 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 841.2792 | 7.117 | 0235 |
| M+H | 841.2986 | 8.211 | 0236 |
| M+H | 842.2315 | 9.245 | 0237 |
| M+H | 842.2634 | 7.616 | 0238 |
| M-H | 840.2703 | 7.737 | 0239 |
| M+H | 842.3213 | 8.497 | 0240 |
| M+H | 843.2466 | 7.711 | 0241 |
| M+H | 843.3141 | 8.706 | 0242 |
| M+H | 844.2475 | 7.796 | 0243 |
| M+H | 844.2807 | 9.434 | 0244 |
| M+H | 844.3094 | 8.938 | 0245 |
| M+H | 844.3401 | 8.017 | 0246 |
| M+H | 844.3847 | 8.102 | 0247 |
| M+H | 845.3048 | 8.428 | 0248 |
| M+H | 845.3149 | 8.831 | 0249 |
| M+H | 846.2362 | 7.477 | 0250,0251 |
| M+H | 846.2404 | 7.618 | 0250,0251 |
| M+H | 846.2598 | 8.952 | 0252 |
| M+H | 846.2988 | 9.129 | 0253 |
| M+H | 846.3144 | 8.802 | 0254 |
| M+H | 846.3192 | 7.822 | 0254,0255 |
| M-H | 845.2070 | 7.892 | 0256 |
| M+H | 847.2685 | 7.796 | 0257,0258 |
| M+H | 847.2742 | 8.425 | 0258 |
| M+H | 848.2536 | 7.431 | 0259 |
| M+H | 848.2790 | 8.903 | 0260,0261 |
| M+H | 848.2779 | 8.402 | 0260,0261 |
| M+H | 848.3184 | 7.816 | 0262 |
| M+H | 848.3593 | 7.928 | 0263 |
| M+H | 849.2753 | 8.119 | 0264,0265,0266 |
| M+H | 849.2751 | 8.246 | 0264,0265,0266 |
| M+H | 849.2764 | 8.06 | 0264,0265,0266 |
| M+H | 849.2837 | 8.059 | 0267 |
| M+H | 850.2659 | 8.651 | 0268 |
| M+H | 850.2897 | 5.588 | 0269,0270 |
| M+H | 850.2932 | 8.539 | 0269,0270,0271 |
| M+H | 850.2947 | 7.609 | 0269,0270,0271 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 851.1989 | 7.699 | 0272 |
| M+H | 851.2709 | 8.735 | 0273 |
| M+H | 851.2886 | 7.388 | 0274 |
| M+H | 852.2288 | 8.199 | 0275 |
| M+H | 852.2525 | 8.224 | 0276,0277 |
| M+H | 852.2526 | 8.27 | 0276,0277 |
| M+H | 852.3090 | 8.144 | 0278 |
| M+H | 853.2484 | 7.923 | 0279,0280,0281 |
| M+H | 853.2482 | 7.878 | 0279,0280,0281 |
| M+H | 853.2523 | 9.817 | 0279,0280,0281 |
| M+H | 853.2828 | 8.903 | 0282,0283 |
| M+H | 853.2833 | 9.459 | 0282,0283 |
| M-H | 851.2880 | 7.794 | 0284 |
| M-H | 852.2471 | 7.957 | 0285 |
| M+H | 854.2675 | 7.876 | 0285,0286,0287 |
| M-H | 852.2491 | 7.902 | 0285,0286,0287 |
| M+H | 854.2783 | 8.339 | 0288,0289,0290 |
| M+H | 854.2786 | 9.035 | 0288,0289,0290 |
| M+H | 854.2795 | 8.799 | 0288,0289,0290 |
| M-H | 852.2828 | 8.101 | 0291 |
| M+H | 855.2633 | 7.17 | 0292 |
| M+H | 855.2935 | 7.429 | 0293 |
| M+H | 856.2532 | 9.21 | 0294 |
| M+H | 856.2801 | 8.852 | 0295 |
| M+H | 856.3031 | 7.796 | 0296 |
| M+H | 856.3343 | 9.212 | 0297 |
| M+H | 856.3842 | 7.811 | 0298 |
| M+H | 857.2097 | 7.857 | 0299 |
| M+H | 857.2623 | 8.056 | 0300 |
| M+H | 857.2794 | 8.571 | 0301 |
| M+H | 858.2656 | 7.949 | 0302 |
| M+H | 858.2775 | 6.858 | 0303 |
| M-H | 856.2717 | 8.382 | 0304 |
| M+H | 858.3090 | 8.413 | 0305 |
| M-H | 857.2067 | 7.777 | 0306 |
| M+H | 859.2607 | 7.723 | 0307 |
| M+H | 859.2700 | 7.245 | 0308 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 859.3045 | 8.653 | 0309 |
| M+H | 860.2235 | 7.97 | 0310 |
| M+H | 860.2539 | 6.309 | 0311 |
| M+H | 860.2785 | 7.854 | 0312,0313,0314 |
| M+H | 860.2776 | 8.325 | 0312,0313,0314 |
| M+H | 860.2777 | 8.257 | 0312,0313,0314 |
| M+H | 860.3040 | 8.483 | 0315,0316 |
| M+H | 860.3038 | 8.531 | 0315,0316 |
| M+H | 861.2375 | 7.835 | 0317 |
| M+H | 861.2728 | 8.079 | 0318,0319,0320,0321 |
| M+H | 861.2734 | 8.325 | 0318,0319,0320,0321 |
| M+H | 861.2717 | 10.265 | 0318,0319,0320 |
| M+H | 861.2785 | 8.266 | 0318,0319,0320,0321 |
| M+H | 861.3090 | 8.607 | 0322 |
| M+H | 862.2920 | 9.108 | 0323 |
| M+H | 863.2487 | 8.69 | 0324 |
| M+H | 863.2554 | 7.655 | 0325 |
| M+H | 864.2304 | 7.732 | 0326 |
| M+H | 864.3035 | 9.343 | 0327,0328 |
| M+H | 864.3036 | 9.24 | 0327,0328 |
| M+H | 865.2616 | 8.343 | 0329 |
| M+H | 865.2991 | 9.272 | 0330,0331,0332 |
| M+H | 865.2985 | 8.794 | 0330,0331,0332 |
| M+H | 865.2990 | 9.474 | 0330,0331,0332 |
| M+H | 865.3063 | 8.24 | 0333 |
| M+H | 866.2527 | 8.045 | 0334 |
| M+H | 866.3012 | 9.474 | 0335 |
| M+H | 866.3239 | 8.423 | 0336 |
| M+H | 867.2292 | 8.029 | 0337,0338 |
| M+H | 867.2292 | 7.968 | 0337,0338 |
| M+H | 867.2954 | 7.301 | 0339 |
| M+H | 868.2290 | 8.392 | 0340,0341 |
| M+H | 868.2251 | 7.237 | 0340,0341 |
| M+H | 868.2750 | 8.123 | 0342 |
| M+H | 868.3040 | 7.972 | 0343 |
| M+H | 869.3306 | 8.833 | 0344 |
| M+H | 870.2762 | 7.374 | 0345 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 870.2944 | 7.859 | 0346 |
| M+H | 870.2997 | 8.252 | 0346,0347 |
| M+H | 870.3242 | 9.151 | 0348 |
| M+H | 870.3996 | 8.123 | 0349 |
| M+H | 871.3201 | 8.572 | 0350 |
| M+H | 871.3300 | 8.987 | 0351 |
| M+H | 872.2536 | 7.315 | 0352,0353 |
| M+H | 872.2536 | 7.3 | 0352,0353 |
| M+H | 872.2820 | 8.226 | 0354 |
| M+H | 872.2955 | 8.337 | 0355 |
| M+H | 872.3291 | 9.044 | 0356 |
| M+H | 873.1851 | 8.027 | 0357 |
| M-H | 871.2224 | 7.957 | 0358 |
| M+H | 874.1982 | 7.943 | 0359 |
| M+H | 874.2694 | 7.667 | 0360 |
| M+H | 874.2760 | 9.198 | 0361 |
| M+H | 874.2927 | 8.638 | 0362,0363 |
| M+H | 874.2927 | 8.448 | 0362,0363 |
| M+H | 874.3745 | 7.925 | 0364 |
| M+H | 875.2891 | 8.444 | 0365,0366,0367 |
| M+H | 875.2890 | 8.403 | 0365,0366,0367 |
| M+H | 875.2892 | 8.555 | 0365,0366,0367 |
| M+H | 876.2536 | 8.529 | 0368 |
| M+H | 876.2815 | 8.806 | 0369 |
| M+H | 877.2154 | 7.881 | 0370 |
| M+H | 877.2861 | 8.894 | 0371 |
| M-H | 876.2324 | 8.533 | 0372,0373 |
| M-H | 876.2324 | 8.468 | 0372,0373 |
| M+H | 878.2655 | 8.426 | 0374,0375 |
| M+H | 878.2677 | 9.355 | 0374,0375 |
| M+H | 878.3238 | 8.283 | 0376 |
| M+H | 879.2446 | 7.878 | 0377 |
| M+H | 879.2634 | 8.226 | 0378,0379,0380 |
| M+H | 879.2634 | 8.165 | 0378,0379,0380 |
| M+H | 879.2662 | 13.535 | 0378,0379,0380 |
| M+H | 879.3165 | 8.778 | 0381 |
| M+H | 880.2676 | 8.359 | 0382 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 880.3115 | 8.333 | 0383 |
| M+H | 881.3105 | 7.618 | 0384 |
| M+H | 882.3198 | 8.106 | 0385 |
| M+H | 882.3505 | 9.4 | 0386 |
| M+H | 883.2242 | 8.101 | 0387 |
| M+H | 883.2948 | 8.709 | 0388 |
| M+H | 884.2058 | 8.567 | 0389 |
| M+H | 884.2417 | 8.182 | 0390 |
| M+H | 884.2778 | 9.35 | 0391 |
| M-H | 882.2755 | 9.192 | 0392 |
| M+H | 885.2144 | 7.812 | 0393 |
| M+H | 885.2187 | 8.448 | 0393,0394 |
| M+H | 885.2725 | 8.646 | 0395 |
| M+H | 885.2851 | 7.413 | 0396 |
| M+H | 886.2698 | 6.589 | 0397 |
| M+H | 886.2942 | 8.077 | 0398 |
| M+H | 887.2300 | 7.767 | 0399 |
| M+H | 887.2910 | 8.165 | 0400 |
| M+H | 887.3362 | 8.307 | 0401 |
| M+H | 888.3065 | 9.295 | 0402 |
| M+H | 889.2715 | 8.966 | 0403 |
| M+H | 890.1756 | 8.021 | 0404 |
| M+H | 890.2503 | 8.205 | 0405 |
| M+H | 891.2292 | 8.536 | 0406,0407 |
| M+H | 891.2293 | 8.566 | 0406,0407 |
| M+H | 892.2274 | 8.25 | 0408,0409,0410 |
| M+H | 892.2274 | 8.368 | 0408,0409,0410 |
| M+H | 892.2252 | 8.848 | 0408,0409,0410 |
| M+H | 892.2666 | 8.16 | 0411 |
| M+H | 892.3396 | 8.637 | 0412 |
| M+H | 893.2444 | 8.179 | 0413,0414 |
| M+H | 893.2447 | 8.211 | 0413,0414 |
| M+H | 894.2433 | 8.661 | 0415,0416 |
| M+H | 894.2434 | 8.609 | 0415,0416 |
| M+H | 894.3308 | 7.568 | 0417 |
| M+H | 896.2351 | 8.372 | 0418 |
| M+H | 896.2554 | 8.007 | 0419 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 896.2966 | 8.458 | 0420 |
| M+H | 896.3151 | 8.431 | 0421 |
| M+H | 898.2504 | 8.255 | 0423,0424 |
| M+H | 898.2466 | 7.602 | 0423,0424 |
| M+H | 898.2984 | 7.201 | 0425 |
| M+H | 898.3111 | 8.642 | 0426 |
| M+H | 898.3567 | 8.712 | 0427 |
| M+H | 899.2016 | 8.302 | 0428 |
| M+H | 899.2582 | 9.049 | 0429 |
| M+H | 900.2141 | 8.128 | 0430 |
| M-H | 898.2738 | 8.47 | 0431 |
| M+H | 901.1960 | 8.504 | 0432 |
| M+H | 902.2492 | 9.004 | 0433,0434 |
| M+H | 902.2492 | 9.051 | 0433,0434 |
| M-H | 900.2551 | 8.29 | 0435 |
| M+H | 903.2079 | 8.049 | 0436 |
| M+H | 903.2450 | 8.86 | 0437,0438,0439 |
| M+H | 903.2448 | 8.794 | 0437,0438,0439 |
| M+H | 903.2449 | 8.765 | 0437,0438,0439 |
| M+H | 904.2652 | 8.706 | 0440,0441 |
| M+H | 904.2651 | 8.742 | 0440,0441 |
| M+H | 905.2596 | 8.162 | 0442 |
| M+H | 906.2844 | 8.464 | 0443 |
| M+H | 907.2767 | 8.447 | 0444 |
| M+H | 908.2708 | 8.715 | 0445 |
| M+H | 908.3465 | 7.868 | 0446 |
| M+H | 909.2660 | 8.067 | 0447 |
| M+H | 909.2764 | 8.564 | 0448 |
| M+H | 910.2210 | 8.846 | 0449 |
| M+H | 910.2579 | 8.27 | 0450 |
| M+H | 910.2746 | 8.639 | 0451 |
| M+H | 911.2297 | 8.064 | 0452 |
| M+H | 911.2338 | 8.704 | 0452,0453 |
| M+H | 912.3210 | 7.693 | 0454 |
| M+H | 913.2454 | 7.981 | 0455 |
| M+H | 913.3517 | 8.356 | 0456 |
| M+H | 914.2274 | 8.573 | 0457 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 915.2328 | 8.389 | 0458 |
| M+H | 916.1910 | 8.154 | 0459 |
| M+H | 917.2452 | 8.734 | 0460,0461 |
| M+H | 917.2452 | 8.663 | 0460,0461 |
| M+H | 918.2394 | 8.685 | 0462,0463,0464 |
| M+H | 918.2444 | 9.368 | 0462,0463,0464 |
| M+H | 918.2422 | 8.646 | 0462,0463,0464 |
| M+H | 920.2952 | 8.995 | 0465 |
| M+H | 920.3466 | 7.621 | 0466 |
| M+H | 922.2496 | 8.573 | 0467 |
| M+H | 922.2711 | 8.194 | 0468 |
| M+H | 923.2190 | 8.528 | 0469 |
| M+H | 923.2660 | 8.408 | 0470 |
| M+H | 924.2581 | 6.837 | 0471 |
| M+H | 924.2662 | 8.502 | 0471,0472 |
| M+H | 924.3725 | 8.77 | 0473 |
| M+H | 925.2716 | 8.408 | 0474 |
| M+H | 926.2524 | 8.835 | 0475 |
| M+H | 927.2115 | 8.705 | 0476 |
| M+H | 928.2648 | 9.239 | 0477,0478 |
| M+H | 928.2648 | 9.175 | 0477,0478 |
| M+H | 929.2232 | 8.219 | 0479 |
| M+H | 929.2610 | 9.169 | 0480,0481,0482 |
| M+H | 929.2612 | 8.995 | 0480,0481,0482 |
| M+H | 929.2613 | 6.766 | 0480,0481,0482 |
| M+H | 933.2916 | 8.65 | 0483 |
| M+H | 934.2384 | 9.044 | 0484 |
| M+H | 934.2871 | 8.903 | 0485 |
| M+H | 934.3616 | 7.94 | 0486 |
| M+H | 935.2820 | 8.251 | 0487 |
| M+H | 935.2917 | 8.753 | 0488 |
| M-H | 934.2735 | 8.914 | 0489 |
| M+H | 938.3367 | 7.738 | 0490 |
| M+H | 940.2425 | 8.719 | 0491 |
| M+H | 941.2480 | 8.609 | 0492 |
| M+H | 946.3116 | 9.218 | 0493 |
| M+H | 949.2336 | 8.678 | 0494 |

(continued)

| [Table 9-8-4] Compound that may be contained in mixture 2-2-d1E04-1 | | | |
|---|---|---|---|
| Ion species | m/z [M+H]+ or [M-H]- | Retention time of MS peak (min) | No. of assigned compound (described with 2-2-d1E04-1- omitted) |
| M+H | 951.2977 | 8.065 | 0495 |
| M+H | 952.2690 | 9.089 | 0496 |
| M+H | 960.2542 | 9.17 | 0497 |
| M+H | 962.3183 | 8.489 | 0498 |
| M+H | 977.3124 | 8.164 | 0499 |
| M+H | 988.3333 | 8.6 | 0500 |

[Table 508]

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M+H | 597.2700 | 14.696 | 0001 |
| M+H | 601.3383 | 14.482 | 0002 |
| M+H | 603.3171 | 14.204 | 0003 |
| M+H | 607.2905 | 15.122 | 0004,0005 |
| M+H | 607.2905 | 14.919 | 0004,0005 |
| M+H | 608.2866 | 13.889 | 0006 |
| M+H | 611.2861 | 15.147 | 0007 |
| M+H | 613.2482 | 15.043 | 0008 |
| M+H | 614.2617 | 12.974 | 0009 |
| M+H | 615.2623 | 6.987 | 0010 |
| M+H | 615.3532 | 14.889 | 0011 |
| M+H | 617.3328 | 14.608 | 0012 |
| M+H | 619.3288 | 14.504 | 0013 |
| M+H | 621.3088 | 14.296 | 0014,0015,0016 |
| M+H | 621.3067 | 12.096 | 0014,0015,0016 |
| M+H | 621.3088 | 14.243 | 0014,0015,0016 |
| M+H | 622.3028 | 14.428 | 0017 |
| M+H | 623.2859 | 15.272 | 0018 |
| M-H | 623.2639 | 14.887 | 0019,0020,0021 |
| M+H | 625.2816 | 15.016 | 0019,0020,0021 |
| M+H | 625.2814 | 14.96 | 0019,0020,0021 |
| M+H | 626.2767 | 13.968 | 0022 |
| M+H | 627.2627 | 15.423 | 0023 |
| M+H | 627.2915 | 14.537 | 0024 |
| M+H | 627.3539 | 15.297 | 0025 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M+H | 628.2769 | 14.293 | 0026 |
| M+H | 629.3326 | 14.949 | 0027 |
| M+H | 630.2375 | 15.081 | 0028 |
| M+H | 631.2380 | 15.134 | 0029 |
| M+H | 631.2908 | 15.614 | 0030,0031 |
| M+H | 631.2908 | 15.539 | 0030,0031 |
| M+H | 632.2531 | 12.889 | 0032 |
| M+H | 632.2860 | 14.807 | 0033,0034,0035 |
| M+H | 632.2867 | 15.907 | 0033,0034,0035 |
| M+H | 632.2869 | 16.259 | 0033,0034,0035 |
| M+H | 633.3050 | 15.475 | 0036,0037 |
| M+H | 633.3047 | 15.351 | 0036,0037 |
| M+H | 634.3025 | 14.455 | 0038 |
| M+H | 636.3179 | 13.74 | 0039 |
| M+H | 637.3019 | 14.694 | 0040 |
| M+H | 637.3126 | 15.729 | 0041 |
| M+H | 638.3083 | 14.688 | 0042 |
| M+H | 639.2632 | 9.25 | 0043 |
| M-H | 637.2761 | 14.198 | 0044 |
| M+H | 639.3184 | 15.051 | 0045 |
| M+H | 640.2791 | 8.682 | 0046 |
| M+H | 641.2769 | 15.285 | 0047 |
| M-H | 639.2912 | 14.958 | 0048 |
| M+H | 641.3694 | 15.778 | 0049 |
| M+H | 643.2702 | 15.072 | 0050,0051 |
| M+H | 643.2702 | 15.134 | 0050,0051 |
| M+H | 643.3494 | 15.327 | 0052 |
| M+H | 644.2537 | 15.412 | 0053 |
| M+H | 645.2824 | 14.576 | 0054 |
| M+H | 645.3068 | 12.028 | 0055,0056 |
| M+H | 645.3064 | 15.933 | 0055,0056 |
| M+H | 645.3437 | 13.261 | 0057 |
| M+H | 646.3015 | 16.241 | 0058,0059,0060 |
| M+H | 646.3016 | 16.181 | 0058,0059,0060 |
| M+H | 646.3018 | 10.947 | 0058,0059,0060 |
| M+H | 647.3231 | 15.003 | 0061,0062,0063 |

(continued)

| [Table 9-8-5] Compound that may be contained in mixture 2-2-dlE01-2 | | | |
|---|---|---|---|
| Ion species | m/z<br>[M+H]+ or [M-H]- | Retention time of MS peak (min) | No. of assigned compound (described with 2-2-d1E01-2- omitted) |
| M+H | 647.3228 | 14.9 | 0061,0062,0063 |
| M+H | 647.3224 | 12.93 | 0061,0062,0063 |
| M+H | 648.2294 | 15.058 | 0064 |
| M+H | 648.3180 | 12.665 | 0065 |
| M+H | 649.2832 | 15.579 | 0066,0067 |
| M+H | 649.2819 | 11.785 | 0066,0067 |
| M+H | 649.3388 | 15.645 | 0068 |
| M+H | 650.2763 | 14.86 | 0069,0070,0071 |
| M+H | 650.2765 | 15.94 | 0069,0070,0071 |
| M+H | 650.2766 | 16.25 | 0069,0070,0071 |
| M+H | 650.3334 | 14.163 | 0072 |
| M-H | 649.2795 | 15.455 | 0073,0074,0075 |
| M+H | 651.2961 | 15.404 | 0073,0074,0075 |
| M+H | 651.2962 | 15.363 | 0073,0074,0075 |
| M+H | 651.3290 | 16.039 | 0076 |
| M+H | 652.2933 | 14.579 | 0077 |
| M+H | 652.3244 | 15.07 | 0078 |
| M+H | 653.2792 | 10.757 | 0079 |
| M+H | 653.3093 | 15.101 | 0080 |
| M+H | 653.3326 | 15.48 | 0081 |
| M+H | 654.2384 | 15.324 | 0082 |
| M-H | 652.2744 | 13.889 | 0083 |
| M+H | 654.3060 | 14.751 | 0084 |
| M+H | 655.2942 | 15.238 | 0085 |
| M+H | 655.3032 | 15.71 | 0086 |
| M+H | 656.2560 | 12.823 | 0087 |
| M+H | 656.2988 | 14.704 | 0088 |
| M+H | 657.2539 | 15.572 | 0089 |
| M+H | 657.2854 | 15.486 | 0090 |
| M+H | 657.3065 | 16.078 | 0091,0092,0093 |
| M+H | 657.3064 | 15.895 | 0091,0092,0093 |
| M+H | 657.3062 | 15.939 | 0091,0092,0093 |
| M+H | 658.2699 | 13.281 | 0094 |
| M+H | 658.3030 | 15.335 | 0095,0096,0097,0098 |
| M+H | 658.3051 | 14.256 | 0095,0096,0097,0098 |
| M+H | 658.3016 | 16.313 | 0095,0096,0097,0098 |
| M+H | 658.3066 | 15.104 | 0095,0096,0097,0098 |
| M+H | 660.2847 | 14.853 | 0099 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M-H | 659.2151 | 14.191 | 0100 |
| M+H | 661.2592 | 15.175 | 0101 |
| M+H | 662.3324 | 16.234 | 0102 |
| M+H | 663.2809 | 15.337 | 0103 |
| M+H | 663.3287 | 12.907 | 0104 |
| M+H | 663.3537 | 15.934 | 0105 |
| M+H | 664.2590 | 15.542 | 0106,0107 |
| M+H | 664.2584 | 15.402 | 0106,0107 |
| M+H | 664.3226 | 15.155 | 0108 |
| M+H | 665.2532 | 14.524 | 0109,0110 |
| M+H | 665.2612 | 15.651 | 0110 |
| M+H | 665.2999 | 14.028 | 0111 |
| M+H | 665.3079 | 11.568 | 0112 |
| M+H | 665.3321 | 15.756 | 0113 |
| M+H | 667.2789 | 13.746 | 0114 |
| M+H | 667.3238 | 15.46 | 0115 |
| M+H | 667.3290 | 15.686 | 0116 |
| M+H | 669.2821 | 11.183 | 0117,0118 |
| M+H | 669.2885 | 13.655 | 0118 |
| M+H | 669.3091 | 15.739 | 0119 |
| M-H | 667.3071 | 15.463 | 0120 |
| M+H | 670.2146 | 15.63 | 0121 |
| M+H | 670.2711 | 12.694 | 0122 |
| M+H | 671.2284 | 12.538 | 0123 |
| M+H | 671.2531 | 14.488 | 0124,0125 |
| M+H | 671.2522 | 14.279 | 0124,0125 |
| M+H | 671.3001 | 15.109 | 0126 |
| M+H | 671.3035 | 15.238 | 0126,0127 |
| M+H | 671.3218 | 16.387 | 0128,0129 |
| M+H | 671.3216 | 16.247 | 0128,0129 |
| M-H | 670.2322 | 13.571 | 0130 |
| M+H | 672.3181 | 15.682 | 0131,0132,0133 |
| M+H | 672.3179 | 16.914 | 0131,0132,0133 |
| M+H | 672.3175 | 16.691 | 0131,0132,0133 |
| M+H | 673.2842 | 15.482 | 0134 |
| M+H | 674.2440 | 15.552 | 0135 |
| M+H | 675.2477 | 14.586 | 0136 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M+H | 675.2789 | 15.772 | 0137,0138 |
| M+H | 675.2789 | 15.811 | 0137,0138 |
| M+H | 675.2966 | 16.078 | 0139,0140 |
| M+H | 675.2966 | 15.904 | 0139,0140 |
| M+H | 675.3543 | 16.185 | 0141 |
| M+H | 676.2744 | 15.052 | 0142 |
| M+H | 676.2923 | 15.347 | 0143,0144,0145 |
| M+H | 676.2920 | 16.367 | 0143,0144,0145 |
| M+H | 676.2920 | 16.644 | 0143,0144,0145 |
| M+H | 676.3493 | 13.106 | 0146 |
| M+H | 677.2086 | 14.513 | 0147 |
| M+H | 677.2965 | 15.678 | 0148 |
| M+H | 677.3478 | 9.909 | 0149 |
| M+H | 678.2221 | 13.975 | 0150 |
| M+H | 678.3387 | 15.361 | 0151 |
| M+H | 679.2236 | 14.31 | 0152 |
| M+H | 679.3150 | 14.404 | 0153 |
| M+H | 679.3488 | 16.081 | 0154 |
| M+H | 680.2535 | 15.948 | 0155 |
| M+H | 680.3219 | 8.435 | 0156 |
| M+H | 681.2343 | 15.911 | 0157 |
| M+H | 681.2700 | 15.406 | 0158 |
| M+H | 681.2944 | 14.111 | 0159 |
| M+H | 681.3110 | 16.047 | 0160 |
| M+H | 681.3208 | 16.272 | 0161 |
| M+H | 682.2436 | 14.958 | 0162,0163 |
| M+H | 682.2471 | 11.698 | 0162,0163 |
| M+H | 682.3142 | 15.112 | 0164 |
| M-H | 681.2722 | 11.935 | 0165 |
| M+H | 683.2999 | 9.765 | 0166 |
| M+H | 683.3224 | 15.803 | 0167 |
| M+H | 684.2598 | 15.116 | 0168 |
| M-H | 682.3025 | 15.958 | 0169 |
| M+H | 685.2700 | 13.853 | 0170,0171,0172 |
| M+H | 685.2689 | 14.638 | 0170,0171,0172 |
| M+H | 685.2699 | 13.808 | 0170,0171,0172 |
| M+H | 686.2637 | 14.054 | 0173 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M+H | 686.2999 | 14.704 | 0174 |
| M-H | 685.1864 | 15.538 | 0175 |
| M-H | 685.2309 | 13.716 | 0176 |
| M+H | 687.2749 | 15.625 | 0177 |
| M+H | 688.2579 | 16.053 | 0178,0179 |
| M+H | 688.2575 | 16.154 | 0178,0179 |
| M+H | 689.2409 | 13.919 | 0180,0181,0182 |
| M+H | 689.2429 | 14.344 | 0180,0181,0182 |
| M+H | 689.2432 | 14.503 | 0180,0181,0182 |
| M+H | 689.2534 | 15.387 | 0183,0184,0185 |
| M+H | 689.2544 | 16.398 | 0183,0184,0185 |
| M+H | 689.2546 | 16.431 | 0183,0184,0185 |
| M+H | 689.2943 | 13.377 | 0186 |
| M+H | 689.3677 | 16.559 | 0187 |
| M+H | 690.2391 | 13.728 | 0188 |
| M+H | 690.2752 | 15.893 | 0189,0190 |
| M+H | 690.2755 | 15.972 | 0189,0190 |
| M+H | 691.2248 | 14.868 | 0191 |
| M+H | 691.2539 | 13.847 | 0192 |
| M+H | 691.2686 | 15.128 | 0193 |
| M-H | 689.2561 | 16.054 | 0193,0194 |
| M+H | 691.3149 | 14.892 | 0195 |
| M+H | 691.3597 | 14.749 | 0196 |
| M+H | 692.2393 | 14.42 | 0197 |
| M+H | 693.2641 | 14.062 | 0198 |
| M+H | 693.2839 | 15.78 | 0199 |
| M+H | 693.2937 | 14.578 | 0200 |
| M+H | 693.3420 | 15.743 | 0201 |
| M+H | 694.1984 | 14.328 | 0202 |
| M+H | 694.2799 | 16.231 | 0203 |
| M+H | 695.1989 | 14.625 | 0204 |
| M+H | 695.2519 | 14.981 | 0205,0206 |
| M+H | 695.2518 | 14.926 | 0205,0206 |
| M+H | 695.2759 | 15.337 | 0207,0208 |
| M+H | 695.2786 | 15.481 | 0207,0208 |
| M-H | 693.3088 | 14.896 | 0209 |
| M+H | 695.3424 | 16.141 | 0210 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M+H | 696.2156 | 12.557 | 0211 |
| M+H | 696.2310 | 16.112 | 0212 |
| M+H | 696.2493 | 15.089 | 0213,0214,0215 |
| M+H | 696.2474 | 15.166 | 0213,0214,0215 |
| M-H | 694.2321 | 15.038 | 0213,0214,0215 |
| M+H | 696.2854 | 14.913 | 0216 |
| M+H | 697.2457 | 15.654 | 0217 |
| M+H | 697.2725 | 5.181 | 0218,0219 |
| M-H | 695.2539 | 12.461 | 0218,0219 |
| M+H | 697.3196 | 15.865 | 0220 |
| M+H | 698.2246 | 15.891 | 0221 |
| M+H | 698.2637 | 12.413 | 0222 |
| M+H | 699.2769 | 16.374 | 0223,0224 |
| M+H | 699.2758 | 13.03 | 0223,0224 |
| M+H | 699.3023 | 16.038 | 0225 |
| M+H | 700.2372 | 15.658 | 0226 |
| M+H | 700.2730 | 15.73 | 0227,0228,0229 |
| M+H | 700.2727 | 16.669 | 0227,0228,0229 |
| M+H | 700.2724 | 16.62 | 0227,0228,0229 |
| M+H | 700.2789 | 14.266 | 0227,0228,0229,0230 |
| M+H | 701.2637 | 15.166 | 0231 |
| M+H | 701.2745 | 15.051 | 0232 |
| M+H | 701.2929 | 16.231 | 0233,0234 |
| M+H | 701.2928 | 16.28 | 0233,0234 |
| M+H | 702.2704 | 13.795 | 0235 |
| M+H | 702.2886 | 15.433 | 0236 |
| M+H | 703.2235 | 14.063 | 0237 |
| M-H | 701.2362 | 14.398 | 0238 |
| M+H | 703.2793 | 14.448 | 0239 |
| M+H | 703.3118 | 16.165 | 0240 |
| M+H | 704.2366 | 14.606 | 0241 |
| M+H | 704.3046 | 14.926 | 0242 |
| M+H | 705.2379 | 14.804 | 0243 |
| M+H | 705.2697 | 14.218 | 0244 |
| M+H | 705.2996 | 16.506 | 0245 |
| M-H | 703.3129 | 15.277 | 0246 |
| M+H | 705.3754 | 15.121 | 0247 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M+H | 706.2959 | 15.598 | 0248 |
| M+H | 706.3063 | 16.173 | 0249 |
| M+H | 707.2322 | 14.452 | 0250,0251 |
| M+H | 707.2319 | 14.508 | 0250,0251 |
| M+H | 707.2520 | 13.875 | 0252 |
| M+H | 707.2881 | 15.809 | 0253 |
| M+H | 707.3038 | 15.953 | 0254 |
| M-H | 705.2944 | 14.965 | 0254,0255 |
| M+H | 708.2143 | 14.707 | 0256 |
| M+H | 708.2591 | 15.778 | 0257,0258 |
| M+H | 708.2606 | 15.857 | 0257,0258 |
| M+H | 709.2441 | 13.937 | 0259 |
| M+H | 709.2694 | 15.348 | 0260,0261 |
| M+H | 709.2691 | 15.246 | 0260,0261 |
| M+H | 709.3085 | 14.748 | 0262 |
| M+H | 709.3497 | 14.787 | 0263 |
| M+H | 710.2637 | 15.481 | 0264,0265,0266 |
| M+H | 710.2665 | 15.431 | 0264,0265,0266 |
| M+H | 710.2647 | 13.7 | 0264,0265,0266 |
| M+H | 710.2752 | 15.677 | 0267 |
| M+H | 711.2562 | 15.986 | 0268 |
| M+H | 711.2840 | 14.603 | 0269,0270,0271 |
| M+H | 711.2844 | 14.518 | 0269,0270,0271 |
| M-H | 709.2669 | 14.548 | 0269,0270,0271 |
| M-H | 710.1717 | 14.399 | 0272 |
| M+H | 712.2617 | 15.261 | 0273 |
| M+H | 712.2798 | 14.674 | 0274 |
| M+H | 713.2196 | 16.047 | 0275 |
| M+H | 713.2433 | 15.014 | 0276,0277 |
| M+H | 713.2435 | 15.153 | 0276,0277 |
| M+H | 713.2988 | 15.066 | 0278 |
| M+H | 714.2385 | 15.203 | 0279,0280,0281 |
| M-H | 712.2210 | 15.134 | 0279,0280,0281 |
| M+H | 714.2409 | 12.704 | 0279,0280,0281 |
| M+H | 714.2731 | 16.447 | 0282,0283 |
| M+H | 714.2727 | 16.603 | 0282,0283 |
| M+H | 714.2978 | 13.061 | 0284 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M-H | 713.2410 | 14.812 | 0285,0286,0287 |
| M+H | 715.2626 | 13.342 | 0286,0287 |
| M+H | 715.2589 | 14.89 | 0285,0286,0287 |
| M+H | 715.2688 | 15.932 | 0288,0289,0290 |
| M+H | 715.2708 | 16.887 | 0288,0289,0290 |
| M+H | 715.2700 | 17.146 | 0288,0289,0290 |
| M+H | 715.2897 | 15.426 | 0291 |
| M+H | 716.2548 | 14.298 | 0292 |
| M+H | 716.2858 | 14.182 | 0293 |
| M+H | 717.2704 | 14.547 | 0295 |
| M+H | 717.2943 | 14.853 | 0296 |
| M+H | 717.3243 | 16.392 | 0297 |
| M+H | 717.3742 | 15.211 | 0298 |
| M+H | 718.2005 | 14.775 | 0299 |
| M+H | 718.2547 | 14.934 | 0300 |
| M+H | 718.2731 | 14.594 | 0301 |
| M+H | 719.2564 | 14.813 | 0302 |
| M+H | 719.2662 | 15.123 | 0303 |
| M+H | 719.2816 | 12 | 0304 |
| M+H | 719.3001 | 15.94 | 0305 |
| M+H | 720.2122 | 14.882 | 0306 |
| M+H | 720.2518 | 13.301 | 0307 |
| M+H | 720.2608 | 13.843 | 0308 |
| M+H | 720.2963 | 16.834 | 0309 |
| M+H | 721.2140 | 15.169 | 0310 |
| M+H | 721.2463 | 14.882 | 0311 |
| M-H | 719.2510 | 14.566 | 0312,0313,0314 |
| M+H | 721.2697 | 15.587 | 0312,0313,0314 |
| M+H | 721.2697 | 15.532 | 0312,0313,0314 |
| M+H | 721.2913 | 15.838 | 0315,0316 |
| M+H | 721.2929 | 15.893 | 0315,0316 |
| M-H | 720.2103 | 14.686 | 0317 |
| M+H | 722.2631 | 15.756 | 0318,0319,0320,0321 |
| M+H | 722.2669 | 13.721 | 0318,0319,0320,0321 |
| M-H | 720.2467 | 15.631 | 0318,0319,0320 |
| M-H | 720.2508 | 13.815 | 0318,0319,0320,0321 |
| M+H | 722.2992 | 16.317 | 0322 |

(continued)

| [Table 9-8-5] Compound that may be contained in mixture 2-2-dlE01-2 | | | |
|---|---|---|---|
| Ion species | m/z<br>[M+H]+ or [M-H]- | Retention time of MS peak (min) | No. of assigned compound (described with 2-2-d1E01-2- omitted) |
| M+H | 723.2815 | 16.199 | 0323 |
| M+H | 724.2387 | 16.305 | 0324 |
| M+H | 724.2471 | 14.392 | 0325 |
| M+H | 725.2211 | 14.578 | 0326 |
| M+H | 725.2955 | 16.695 | 0327,0328 |
| M+H | 725.2958 | 16.581 | 0327,0328 |
| M+H | 726.2520 | 16.158 | 0329 |
| M+H | 726.2903 | 17.016 | 0330,0331,0332 |
| M+H | 726.2903 | 16.972 | 0330,0331,0332 |
| M+H | 726.2903 | 16.934 | 0330,0331,0332 |
| M+H | 726.2940 | 12.059 | 0330,0331,0332,0333 |
| M-H | 725.2258 | 14.954 | 0334 |
| M+H | 727.2903 | 12.454 | 0335 |
| M+H | 727.3138 | 11.98 | 0336 |
| M+H | 728.2205 | 14.848 | 0337,0338 |
| M+H | 728.2207 | 15.005 | 0337,0338 |
| M+H | 728.2862 | 12.117 | 0339 |
| M+H | 729.2151 | 14.346 | 0340,0341 |
| M+H | 729.2192 | 15.189 | 0340,0341 |
| M+H | 729.2687 | 12.745 | 0342 |
| M-H | 727.2771 | 15.127 | 0343 |
| M+H | 730.3212 | 15.568 | 0344 |
| M+H | 731.2691 | 16.181 | 0345 |
| M+H | 731.2845 | 14.894 | 0346 |
| M+H | 731.2898 | 15.085 | 0346,0347 |
| M+H | 731.3160 | 16.894 | 0348 |
| M+H | 731.3908 | 15.617 | 0349 |
| M+H | 732.3112 | 16.048 | 0350 |
| M+H | 732.3211 | 16.756 | 0351 |
| M+H | 733.2442 | 12.441 | 0352,0353 |
| M-H | 731.2352 | 13.634 | 0353 |
| M+H | 733.2703 | 15.165 | 0354 |
| M-H | 731.2705 | 14.969 | 0355 |
| M+H | 733.3203 | 16.456 | 0356 |
| M+H | 734.1764 | 15.15 | 0357 |
| M+H | 734.2307 | 6.846 | 0358 |
| M+H | 735.1901 | 14.625 | 0359 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M-H | 733.2482 | 14.775 | 0360,0361 |
| M-H | 733.2482 | 14.709 | 0360,0361 |
| M+H | 735.2835 | 15.936 | 0362,0363 |
| M+H | 735.2845 | 15.878 | 0362,0363 |
| M+H | 735.3650 | 15.263 | 0364 |
| M+H | 736.2789 | 16.108 | 0365,0366,0367 |
| M+H | 736.2790 | 16.173 | 0365,0366,0367 |
| M+H | 736.2785 | 15.98 | 0365,0366,0367 |
| M+H | 737.2462 | 14.908 | 0368 |
| M+H | 737.2719 | 16.369 | 0369 |
| M-H | 736.1887 | 14.918 | 0370 |
| M+H | 738.2766 | 16.576 | 0371 |
| M+H | 739.2393 | 15.398 | 0372,0373 |
| M+H | 739.2407 | 15.336 | 0372,0373 |
| M+H | 739.2604 | 15.597 | 0374,0375 |
| M+H | 739.2604 | 15.511 | 0374,0375 |
| M-H | 737.2974 | 15.716 | 0376 |
| M+H | 740.2356 | 14.904 | 0377 |
| M+H | 740.2531 | 15.819 | 0378,0379,0380 |
| M+H | 740.2537 | 15.781 | 0378,0379,0380 |
| M+H | 740.2537 | 15.671 | 0378,0379,0380 |
| M+H | 740.3100 | 12.806 | 0381 |
| M+H | 741.2568 | 15.288 | 0382 |
| M+H | 741.3096 | 9.676 | 0383 |
| M+H | 742.3002 | 14.779 | 0384 |
| M+H | 743.3116 | 15.527 | 0385 |
| M-H | 741.3232 | 16.856 | 0386 |
| M+H | 744.2160 | 15.309 | 0387 |
| M+H | 744.2853 | 12.163 | 0388 |
| M+H | 745.1982 | 15.543 | 0389 |
| M+H | 745.2339 | 15.023 | 0390 |
| M+H | 745.2703 | 14.506 | 0391 |
| M+H | 745.2809 | 15.569 | 0392 |
| M+H | 746.2049 | 14.573 | 0393 |
| M-H | 744.1920 | 15.052 | 0393,0394 |
| M+H | 746.2646 | 16.389 | 0395 |
| M+H | 746.2754 | 12.248 | 0396 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M+H | 747.2625 | 9.439 | 0397 |
| M+H | 747.2836 | 15.21 | 0398 |
| M+H | 748.2211 | 14.478 | 0399 |
| M+H | 748.2840 | 15.497 | 0400 |
| M+H | 748.3258 | 15.401 | 0401 |
| M+H | 749.2987 | 16.751 | 0402 |
| M+H | 750.2619 | 15.16 | 0403 |
| M+H | 751.1658 | 14.867 | 0404 |
| M+H | 751.2370 | 15.002 | 0405 |
| M+H | 752.2206 | 15.544 | 0406,0407 |
| M+H | 752.2206 | 15.636 | 0406,0407 |
| M+H | 753.2157 | 15.755 | 0408,0409,0410 |
| M+H | 753.2147 | 15.684 | 0408,0409,0410 |
| M+H | 753.2151 | 15.986 | 0408,0409,0410 |
| M-H | 751.2414 | 13.907 | 0411 |
| M+H | 753.3296 | 16.027 | 0412 |
| M+H | 754.2363 | 15.412 | 0413,0414 |
| M+H | 754.2326 | 14.937 | 0413,0414 |
| M+H | 755.2312 | 14.911 | 0415,0416 |
| M+H | 755.2321 | 15.191 | 0415,0416 |
| M+H | 755.3214 | 14.186 | 0417 |
| M-H | 755.2071 | 14.99 | 0418 |
| M+H | 757.2458 | 14.884 | 0419 |
| M+H | 757.2832 | 16.458 | 0420 |
| M-H | 755.2882 | 15.716 | 0421 |
| M+H | 758.2430 | 15.587 | 0422 |
| M+H | 759.2373 | 14.515 | 0423,0424 |
| M+H | 759.2415 | 12.819 | 0423,0424 |
| M+H | 759.2869 | 15.865 | 0425 |
| M+H | 759.3062 | 14.828 | 0426 |
| M+H | 759.3477 | 15.654 | 0427 |
| M+H | 760.1927 | 15.691 | 0428 |
| M+H | 760.2460 | 14.238 | 0429 |
| M+H | 761.2066 | 15.112 | 0430 |
| M-H | 759.2638 | 15.44 | 0431 |
| M-H | 760.1697 | 15.288 | 0432 |
| M+H | 763.2394 | 15.922 | 0433,0434 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M+H | 763.2386 | 15.828 | 0433,0434 |
| M+H | 763.2633 | 15.55 | 0435 |
| M+H | 764.1980 | 15.051 | 0436 |
| M+H | 764.2380 | 16.243 | 0437,0438,0439 |
| M+H | 764.2354 | 16.176 | 0437,0438,0439 |
| M-H | 762.2194 | 15.943 | 0437,0438,0439 |
| M+H | 765.2536 | 15.816 | 0440,0441 |
| M+H | 765.2537 | 15.682 | 0440,0441 |
| M+H | 766.2520 | 15.404 | 0442 |
| M+H | 767.2719 | 15.78 | 0443 |
| M+H | 768.2659 | 15.162 | 0444 |
| M+H | 769.2614 | 15.866 | 0445 |
| M+H | 769.3378 | 14.525 | 0446 |
| M+H | 770.2595 | 14.886 | 0447 |
| M+H | 770.2678 | 15.601 | 0448 |
| M+H | 771.2112 | 15.992 | 0449 |
| M+H | 771.2525 | 15.556 | 0450 |
| M+H | 771.2674 | 15.347 | 0451 |
| M+H | 772.2210 | 15.19 | 0452,0453 |
| M+H | 772.2260 | 15.349 | 0452,0453 |
| M+H | 773.3114 | 14.271 | 0454 |
| M+H | 774.2370 | 15.074 | 0455 |
| M+H | 774.3417 | 15.892 | 0456 |
| M+H | 775.2194 | 15.392 | 0457 |
| M+H | 776.2236 | 14.575 | 0458 |
| M+H | 777.1824 | 15.363 | 0459 |
| M+H | 778.2366 | 16.114 | 0460,0461 |
| M+H | 778.2366 | 15.987 | 0460,0461 |
| M+H | 779.2302 | 16.395 | 0462,0463,0464 |
| M+H | 779.2316 | 16.251 | 0462,0463,0464 |
| M+H | 779.2294 | 16.318 | 0462,0463,0464 |
| M-H | 779.2695 | 15.859 | 0465 |
| M+H | 781.3363 | 17.162 | 0466 |
| M-H | 781.2268 | 11.623 | 0467 |
| M+H | 783.2632 | 15.427 | 0468 |
| M+H | 784.2102 | 15.552 | 0469 |
| M+H | 784.2603 | 16.192 | 0470 |

(continued)

| [Table 9-8-5] Compound that may be contained in mixture 2-2-dlE01-2 | | | |
|---|---|---|---|
| Ion species | m/z [M+H]+ or [M-H]- | Retention time of MS peak (min) | No. of assigned compound (described with 2-2-d1E01-2- omitted) |
| M+H | 785.2568 | 15.351 | 0471,0472 |
| M+H | 785.2561 | 15.321 | 0471,0472 |
| M+H | 785.3619 | 16.075 | 0473 |
| M+H | 786.2610 | 15.756 | 0474 |
| M-H | 785.2274 | 15.237 | 0475 |
| M-H | 786.1838 | 15.73 | 0476 |
| M-H | 787.2369 | 16.313 | 0477,0478 |
| M-H | 787.2369 | 16.178 | 0477,0478 |
| M+H | 790.2144 | 15.617 | 0479 |
| M+H | 790.2533 | 12.709 | 0480,0481,0482 |
| M+H | 790.2533 | 12.658 | 0480,0481,0482 |
| M+H | 790.2500 | 16.711 | 0480,0481,0482 |
| M+H | 794.2830 | 13.227 | 0483 |
| M+H | 795.2302 | 15.869 | 0484 |
| M+H | 795.2762 | 16.377 | 0485 |
| M+H | 795.3538 | 15.107 | 0486 |
| M+H | 796.2764 | 15.337 | 0487 |
| M+H | 796.2826 | 16.237 | 0488 |
| M+H | 797.2821 | 15.929 | 0489 |
| M+H | 799.3282 | 14.758 | 0490 |
| M-H | 799.2175 | 11.809 | 0491 |
| M+H | 802.2412 | 16.026 | 0492 |
| M-H | 805.2846 | 16.379 | 0493 |
| M+H | 810.2230 | 12.284 | 0494 |
| M+H | 812.2878 | 14.822 | 0495 |
| M-H | 811.2443 | 15.464 | 0496 |
| M+H | 821.2457 | 16.239 | 0497 |
| M+H | 823.3086 | 15.09 | 0498 |
| M+H | 838.3043 | 15.376 | 0499 |
| M+H | 849.3260 | 15.594 | 0500 |

[Table 509]

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 613.2655 | 12.376 | 0001 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2-omitted) |
| M+H | 617.3329 | 12.206 | 0002 |
| M+H | 619.3123 | 11.796 | 0003 |
| M+H | 623.2859 | 12.689 | 0004,0005 |
| M+H | 623.2858 | 12.548 | 0004,0005 |
| M+H | 624.2813 | 11.404 | 0006 |
| M+H | 627.2811 | 12.825 | 0007 |
| M+H | 629.2423 | 12.788 | 0008 |
| M+H | 630.2571 | 10.243 | 0009 |
| M+H | 631.2558 | 12.481 | 0010 |
| M+H | 631.3482 | 12.644 | 0011 |
| M+H | 633.3274 | 12.282 | 0012 |
| M+H | 635.3234 | 12.341 | 0013 |
| M+H | 637.3017 | 12.976 | 0014,0015,0016 |
| M+H | 637.3017 | 13.121 | 0014,0015,0016 |
| M+H | 637.3025 | 11.957 | 0014,0015,0016 |
| M+H | 638.2974 | 11.868 | 0017 |
| M+H | 639.2810 | 13.033 | 0018 |
| M+H | 641.2710 | 11.969 | 0019 |
| M+H | 641.2768 | 12.8 | 0019,0020,0021 |
| M+H | 641.2770 | 10.384 | 0020,0021 |
| M+H | 642.2723 | 11.544 | 0022 |
| M-H | 641.2409 | 13.222 | 0023 |
| M+H | 643.2873 | 12.137 | 0024 |
| M+H | 643.3484 | 10.267 | 0025 |
| M+H | 644.2709 | 10.067 | 0026 |
| M+H | 645.3274 | 12.606 | 0027 |
| M+H | 646.2323 | 12.668 | 0028 |
| M+H | 647.2329 | 12.896 | 0029 |
| M+H | 647.2864 | 13.525 | 0030,0031 |
| M+H | 647.2863 | 13.41 | 0030,0031 |
| M+H | 648.2476 | 10.487 | 0032 |
| M+H | 648.2816 | 13.666 | 0033,0034,0035 |
| M+H | 648.2821 | 12.484 | 0033,0034,0035 |
| M+H | 648.2814 | 13.625 | 0033,0034,0035 |
| M+H | 649.3021 | 10.935 | 0036,0037 |
| M+H | 649.3008 | 9.686 | 0036,0037 |
| M+H | 650.2971 | 12.045 | 0038 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 652.3128 | 11.138 | 0039 |
| M+H | 653.2975 | 13.479 | 0040 |
| M+H | 653.3081 | 13.364 | 0041 |
| M+H | 654.3035 | 12.179 | 0042 |
| M+H | 655.2579 | 9.891 | 0043 |
| M-H | 653.2698 | 12.351 | 0044 |
| M+H | 655.3123 | 12.87 | 0045 |
| M+H | 656.2701 | 9.447 | 0046 |
| M+H | 657.2711 | 13.099 | 0047 |
| M+H | 657.3025 | 12.562 | 0048 |
| M+H | 657.3661 | 13.596 | 0049 |
| M+H | 659.2637 | 12.825 | 0050,0051 |
| M+H | 659.2650 | 12.758 | 0050,0051 |
| M+H | 659.3432 | 13.086 | 0052 |
| M+H | 660.2478 | 13.09 | 0053 |
| M+H | 661.2779 | 12.244 | 0054 |
| M+H | 661.3017 | 13.906 | 0055,0056 |
| M+H | 661.3017 | 13.791 | 0055,0056 |
| M+H | 661.3400 | 13.19 | 0057 |
| M+H | 662.2967 | 12.927 | 0058,0059,0060 |
| M+H | 662.2976 | 14.053 | 0058,0059,0060 |
| M+H | 662.2968 | 14.436 | 0058,0059,0060 |
| M+H | 663.3190 | 12.679 | 0061,0062,0063 |
| M+H | 663.3186 | 12.729 | 0061,0062,0063 |
| M+H | 663.3176 | 10.171 | 0061,0062,0063 |
| M+H | 664.2232 | 12.771 | 0064 |
| M+H | 664.3129 | 12.521 | 0065 |
| M+H | 665.2764 | 13.611 | 0066,0067 |
| M+H | 665.2766 | 13.461 | 0066,0067 |
| M+H | 665.3331 | 13.551 | 0068 |
| M+H | 666.2731 | 12.602 | 0069,0070,0071 |
| M+H | 666.2724 | 13.761 | 0069,0070,0071 |
| M+H | 666.2722 | 14.11 | 0069,0070,0071 |
| M+H | 666.3299 | 11.64 | 0072 |
| M-H | 665.2749 | 13.23 | 0073,0074,0075 |
| M+H | 667.2916 | 13.415 | 0073,0074,0075 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 667.2928 | 11.063 | 0074,0075 |
| M+H | 667.3232 | 13.77 | 0076 |
| M+H | 668.2875 | 12.17 | 0077 |
| M+H | 668.3188 | 12.673 | 0078 |
| M+H | 669.2739 | 10.374 | 0079 |
| M+H | 669.3033 | 12.754 | 0080 |
| M+H | 669.3275 | 13.305 | 0081 |
| M+H | 670.2329 | 13.035 | 0082 |
| M-H | 668.2703 | 11.405 | 0083 |
| M+H | 670.3044 | 11.305 | 0084 |
| M+H | 671.2892 | 13.308 | 0085 |
| M+H | 671.2988 | 13.46 | 0086 |
| M+H | 672.2503 | 10.346 | 0087 |
| M+H | 672.2941 | 12.336 | 0088 |
| M+H | 673.2490 | 13.525 | 0089 |
| M+H | 673.2806 | 13.176 | 0090 |
| M+H | 673.3029 | 12.99 | 0091,0092,0093 |
| M+H | 673.3015 | 14.069 | 0091,0092,0093 |
| M+H | 673.3029 | 12.948 | 0091,0092,0093 |
| M+H | 674.2640 | 10.869 | 0094 |
| M+H | 674.3006 | 11.986 | 0095,0096,0097,0098 |
| M+H | 674.2964 | 14.207 | 0095,0096,0097,0098 |
| M+H | 674.2969 | 14.55 | 0095,0096,0097,0098 |
| M+H | 674.3002 | 12.889 | 0095,0096,0097,0098 |
| M+H | 676.2797 | 12.521 | 0099 |
| M+H | 677.2269 | 11.966 | 0100 |
| M+H | 677.2549 | 12.858 | 0101 |
| M+H | 678.3285 | 11.903 | 0102 |
| M-H | 677.2632 | 11.581 | 0103 |
| M+H | 679.3233 | 10.632 | 0104 |
| M+H | 679.3483 | 13.934 | 0105 |
| M+H | 680.2539 | 13.358 | 0106,0107 |
| M+H | 680.2539 | 13.242 | 0106,0107 |
| M+H | 680.3182 | 12.766 | 0108 |
| M+H | 681.2486 | 12.126 | 0109,0110 |
| M+H | 681.2526 | 13.557 | 0109,0110 |
| M+H | 681.2952 | 11.835 | 0111 |
| M+H | 681.3052 | 10.668 | 0112 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 681.3277 | 13.429 | 0113 |
| M+H | 683.2751 | 11.571 | 0114 |
| M+H | 683.3187 | 13.162 | 0115 |
| M+H | 683.3231 | 13.659 | 0115,0116 |
| M+H | 685.2804 | 13.341 | 0117,0118 |
| M+H | 685.2787 | 9.919 | 0117,0118 |
| M+H | 685.3056 | 13.669 | 0119 |
| M-H | 683.3027 | 13.433 | 0120 |
| M+H | 686.2100 | 13.462 | 0121 |
| M+H | 686.2634 | 13.657 | 0122 |
| M+H | 687.2228 | 12.736 | 0123 |
| M+H | 687.2481 | 12.071 | 0124,0125 |
| M+H | 687.2480 | 12.264 | 0124,0125 |
| M+H | 687.2936 | 12.802 | 0126 |
| M+H | 687.2991 | 13.072 | 0127 |
| M+H | 687.3174 | 14.469 | 0128,0129 |
| M+H | 687.3175 | 14.31 | 0128,0129 |
| M+H | 688.2428 | 10.954 | 0130 |
| M+H | 688.3129 | 14.602 | 0131,0132,0133 |
| M+H | 688.3129 | 13.55 | 0131,0132,0133 |
| M+H | 688.3131 | 14.96 | 0131,0132,0133 |
| M+H | 689.2796 | 13.412 | 0134 |
| M+H | 690.2379 | 13.559 | 0135 |
| M+H | 691.2429 | 12.421 | 0136 |
| M+H | 691.2734 | 13.849 | 0137,0138 |
| M+H | 691.2725 | 13.744 | 0137,0138 |
| M+H | 691.2921 | 14.021 | 0139,0140 |
| M+H | 691.2922 | 14.089 | 0139,0140 |
| M+H | 691.3482 | 14.253 | 0141 |
| M+H | 692.2690 | 12.707 | 0142 |
| M+H | 692.2879 | 13.186 | 0143,0144,0145 |
| M+H | 692.2875 | 14.583 | 0143,0144,0145 |
| M+H | 692.2865 | 14.264 | 0143,0144,0145 |
| M+H | 692.3437 | 12.414 | 0146 |
| M+H | 693.2040 | 12.282 | 0147 |
| M+H | 693.2924 | 13.599 | 0148 |
| M+H | 693.3390 | 11.065 | 0149 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 694.2173 | 11.816 | 0150 |
| M+H | 694.3349 | 13.216 | 0151 |
| M+H | 695.2182 | 12.066 | 0152 |
| M+H | 695.3096 | 12.255 | 0153 |
| M+H | 695.3411 | 11.404 | 0154 |
| M+H | 696.2487 | 13.699 | 0155 |
| M+H | 696.3177 | 10.79 | 0156 |
| M+H | 697.2290 | 13.979 | 0157 |
| M+H | 697.2665 | 13.275 | 0158 |
| M+H | 697.2893 | 11.988 | 0159 |
| M+H | 697.3046 | 10.525 | 0160 |
| M+H | 697.3113 | 14.075 | 0161 |
| M+H | 698.2390 | 12.466 | 0162,0163 |
| M+H | 698.2447 | 7.779 | 0163 |
| M+H | 698.3093 | 12.844 | 0164 |
| M+H | 699.2839 | 9.617 | 0165 |
| M+H | 699.2942 | 10.399 | 0166 |
| M+H | 699.3176 | 13.667 | 0167 |
| M+H | 700.2542 | 12.776 | 0168 |
| M+H | 700.3162 | 13.741 | 0169 |
| M-H | 699.2485 | 11.716 | 0170,0171,0172 |
| M+H | 701.2640 | 12.506 | 0170,0171,0172 |
| M+H | 701.2648 | 11.685 | 0170,0171,0172 |
| M+H | 702.2597 | 11.494 | 0173 |
| M+H | 702.2950 | 13.325 | 0174 |
| M+H | 703.2004 | 13.244 | 0175 |
| M+H | 703.2425 | 12.617 | 0176 |
| M+H | 703.2705 | 13.372 | 0177 |
| M+H | 704.2530 | 14.025 | 0178,0179 |
| M-H | 702.2352 | 14.147 | 0178,0179 |
| M-H | 703.2163 | 11.731 | 0180 |
| M+H | 705.2378 | 12.207 | 0180,0181,0182 |
| M+H | 705.2377 | 12.39 | 0180,0181,0182 |
| M+H | 705.2485 | 13.148 | 0183,0184,0185 |
| M+H | 705.2482 | 14.298 | 0183,0184,0185 |
| M-H | 703.2304 | 14.645 | 0183,0184,0185 |
| M+H | 705.2911 | 13.745 | 0186 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 705.3645 | 14.655 | 0187 |
| M+H | 706.2341 | 11.165 | 0188 |
| M+H | 706.2689 | 13.909 | 0189,0190 |
| M+H | 706.2672 | 14.017 | 0189,0190 |
| M+H | 707.2201 | 12.727 | 0191 |
| M+H | 707.2496 | 11.655 | 0192 |
| M+H | 707.2648 | 12.806 | 0193,0194 |
| M+H | 707.2709 | 16.823 | 0194 |
| M+H | 707.3102 | 11.168 | 0195 |
| M+H | 707.3543 | 12.559 | 0196 |
| M+H | 708.2328 | 9.698 | 0197 |
| M+H | 709.2559 | 12.072 | 0198 |
| M+H | 709.2797 | 13.329 | 0199 |
| M+H | 709.2891 | 12.414 | 0200 |
| M+H | 709.3384 | 14.268 | 0201 |
| M-H | 708.1771 | 12.136 | 0202 |
| M+H | 710.2747 | 10.697 | 0203 |
| M+H | 711.1948 | 12.428 | 0204 |
| M+H | 711.2479 | 12.859 | 0205,0206 |
| M+H | 711.2473 | 12.894 | 0205,0206 |
| M+H | 711.2712 | 12.953 | 0207,0208 |
| M-H | 709.2625 | 12.106 | 0208 |
| M+H | 711.3207 | 14.463 | 0209 |
| M+H | 711.3358 | 14.185 | 0210 |
| M-H | 710.1913 | 11.963 | 0211 |
| M+H | 712.2249 | 14.12 | 0212 |
| M+H | 712.2425 | 12.626 | 0213,0214,0215 |
| M+H | 712.2429 | 12.653 | 0213,0214,0215 |
| M+H | 712.2430 | 13.022 | 0213,0214,0215 |
| M+H | 712.2803 | 13.53 | 0216 |
| M-H | 711.2202 | 13.365 | 0217 |
| M+H | 713.2638 | 11.706 | 0218,0219 |
| M+H | 713.2633 | 12.805 | 0218,0219 |
| M+H | 713.3156 | 13.812 | 0220 |
| M+H | 714.2197 | 13.821 | 0221 |
| M-H | 712.2419 | 11.629 | 0222 |
| M+H | 715.2731 | 14.547 | 0223,0224 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 715.2737 | 14.484 | 0223,0224 |
| M+H | 715.2952 | 14.075 | 0225 |
| M-H | 714.2134 | 13.364 | 0226 |
| M+H | 716.2684 | 14.712 | 0227,0228,0229 |
| M+H | 716.2679 | 13.665 | 0227,0228,0229 |
| M+H | 716.2685 | 15.067 | 0227,0228,0229 |
| M+H | 716.2756 | 12.102 | 0230 |
| M+H | 717.2585 | 13.065 | 0231 |
| M+H | 717.2699 | 12.859 | 0232 |
| M+H | 717.2890 | 14.384 | 0233,0234 |
| M+H | 717.2896 | 12.262 | 0233,0234 |
| M+H | 718.2658 | 12.041 | 0235 |
| M+H | 718.2838 | 13.288 | 0236 |
| M+H | 719.2179 | 11.887 | 0237 |
| M-H | 717.2321 | 12.175 | 0238 |
| M+H | 719.2735 | 12.336 | 0239 |
| M-H | 717.2933 | 12.861 | 0240 |
| M-H | 718.2155 | 12.471 | 0241 |
| M+H | 720.3000 | 13.767 | 0242 |
| M+H | 721.2331 | 12.722 | 0243 |
| M+H | 721.2650 | 12.086 | 0244 |
| M+H | 721.2952 | 14.472 | 0245 |
| M+H | 721.3230 | 13.563 | 0246 |
| M+H | 721.3712 | 12.97 | 0247 |
| M+H | 722.2907 | 13.462 | 0248 |
| M+H | 722.3001 | 14.175 | 0249 |
| M-H | 721.2074 | 11.882 | 0250,0251 |
| M+H | 723.2266 | 12.301 | 0250,0251 |
| M+H | 723.2452 | 14.517 | 0252 |
| M+H | 723.2821 | 13.762 | 0253 |
| M+H | 723.2991 | 13.939 | 0254 |
| M+H | 723.3057 | 12.831 | 0255 |
| M+H | 724.2096 | 12.559 | 0256 |
| M+H | 724.2539 | 13.306 | 0257,0258 |
| M-H | 722.2427 | 13.285 | 0257,0258 |
| M-H | 723.2226 | 11.799 | 0259 |
| M+H | 725.2618 | 13.29 | 0260,0261 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 725.2618 | 13.4 | 0260,0261 |
| M-H | 723.2829 | 12.784 | 0262 |
| M+H | 725.3451 | 12.666 | 0263 |
| M-H | 724.2413 | 13.153 | 0264,0265,0266 |
| M-H | 724.2446 | 13.434 | 0264,0265,0266 |
| M+H | 726.2589 | 11.339 | 0264,0265,0266 |
| M+H | 726.2671 | 13.435 | 0267 |
| M+H | 727.2515 | 13.904 | 0268 |
| M+H | 727.2795 | 12.505 | 0269,0270,0271 |
| M+H | 727.2790 | 12.186 | 0269,0270,0271 |
| M+H | 727.2820 | 11.189 | 0269,0270,0271 |
| M+H | 728.1843 | 12.258 | 0272 |
| M+H | 728.2576 | 13.98 | 0273 |
| M+H | 728.2746 | 12.179 | 0274 |
| M+H | 729.2159 | 13.818 | 0275 |
| M+H | 729.2381 | 13.101 | 0276,0277 |
| M+H | 729.2381 | 13.002 | 0276,0277 |
| M-H | 727.2785 | 13.023 | 0278 |
| M+H | 730.2338 | 12.832 | 0279,0280,0281 |
| M+H | 730.2351 | 13.125 | 0279,0280,0281 |
| M+H | 730.2354 | 13.08 | 0279,0280,0281 |
| M+H | 730.2682 | 14.706 | 0282,0283 |
| M+H | 730.2684 | 16.22 | 0282,0283 |
| M+H | 730.2899 | 12.514 | 0284 |
| M-H | 729.2336 | 11.811 | 0285 |
| M+H | 731.2520 | 12.955 | 0285,0286,0287 |
| M+H | 731.2520 | 12.889 | 0285,0286,0287 |
| M+H | 731.2636 | 13.798 | 0288,0289,0290 |
| M+H | 731.2642 | 15.192 | 0288,0289,0290 |
| M+H | 731.2635 | 13.879 | 0288,0289,0290 |
| M+H | 731.2852 | 13.29 | 0291 |
| M+H | 732.2478 | 11.149 | 0292 |
| M+H | 732.2805 | 11.983 | 0293 |
| M+H | 733.2337 | 12.837 | 0294 |
| M+H | 733.2646 | 12.349 | 0295 |
| M+H | 733.2893 | 12.782 | 0296 |
| M+H | 733.3194 | 14.556 | 0297 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 733.3707 | 13.026 | 0298 |
| M+H | 734.1957 | 12.595 | 0299 |
| M+H | 734.2501 | 12.902 | 0300 |
| M+H | 734.2641 | 12.21 | 0301 |
| M+H | 735.2512 | 12.693 | 0302 |
| M+H | 735.2613 | 12.949 | 0303 |
| M+H | 735.2772 | 9.409 | 0304 |
| M+H | 735.2952 | 13.92 | 0305 |
| M+H | 736.2098 | 12.749 | 0306 |
| M+H | 736.2469 | 11.243 | 0307 |
| M+H | 736.2550 | 11.621 | 0308 |
| M+H | 736.2909 | 14.746 | 0309 |
| M+H | 737.2100 | 13.088 | 0310 |
| M+H | 737.2428 | 12.734 | 0311 |
| M+H | 737.2634 | 13.511 | 0312,0313,0314 |
| M+H | 737.2655 | 11.014 | 0312,0313,0314 |
| M-H | 735.2487 | 12.486 | 0312,0313,0314 |
| M+H | 737.2862 | 13.567 | 0315,0316 |
| M+H | 737.2940 | 11.376 | 0316 |
| M+H | 738.2259 | 10.885 | 0317 |
| M+H | 738.2629 | 11.683 | 0318,0319,0320,0321 |
| M+H | 738.2590 | 13.437 | 0318,0319,0320,0321 |
| M+H | 738.2592 | 13.621 | 0318,0319,0320,0321 |
| M+H | 738.2642 | 12.491 | 0318,0319,0320,0321 |
| M+H | 738.2941 | 14.274 | 0322 |
| M+H | 739.2766 | 14.36 | 0323 |
| M-H | 738.2238 | 12.258 | 0324,0325 |
| M+H | 740.2414 | 11.39 | 0325 |
| M+H | 741.2170 | 12.424 | 0326 |
| M+H | 741.2886 | 15.025 | 0327,0328 |
| M+H | 741.2885 | 14.948 | 0327,0328 |
| M+H | 742.2475 | 13.999 | 0329 |
| M+H | 742.2845 | 14.199 | 0330,0331,0332 |
| M+H | 742.2839 | 15.498 | 0330,0331,0332 |
| M+H | 742.2840 | 15.179 | 0330,0331,0332 |
| M+H | 742.2925 | 12.846 | 0333 |
| M+H | 743.2377 | 12.837 | 0334 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 743.2839 | 10.221 | 0335 |
| M+H | 743.3094 | 13.423 | 0336 |
| M+H | 744.2150 | 12.913 | 0337,0338 |
| M+H | 744.2150 | 12.744 | 0337,0338 |
| M+H | 744.2809 | 12.12 | 0339 |
| M+H | 745.2106 | 11.816 | 0340,0341 |
| M+H | 745.2141 | 13.182 | 0340,0341 |
| M-H | 743.2475 | 12.899 | 0342 |
| M+H | 745.2890 | 13.113 | 0343 |
| M-H | 744.3016 | 13.65 | 0344 |
| M+H | 747.2616 | 14.247 | 0345 |
| M+H | 747.2803 | 12.782 | 0346 |
| M-H | 745.2687 | 13.027 | 0346,0347 |
| M+H | 747.3114 | 15.065 | 0348 |
| M+H | 747.3861 | 13.455 | 0349 |
| M+H | 748.3053 | 13.952 | 0350 |
| M+H | 748.3155 | 14.866 | 0351 |
| M+H | 749.2430 | 12.935 | 0352,0353 |
| M+H | 749.2422 | 10.806 | 0352,0353 |
| M+H | 749.2665 | 13.104 | 0354 |
| M+H | 749.2827 | 12.173 | 0355 |
| M+H | 749.3155 | 14.598 | 0356 |
| M+H | 750.1725 | 12.979 | 0357 |
| M+H | 751.1841 | 12.498 | 0359 |
| M+H | 751.2553 | 12.453 | 0360 |
| M+H | 751.2599 | 12.786 | 0360,0361 |
| M+H | 751.2787 | 13.904 | 0362,0363 |
| M+H | 751.2783 | 13.996 | 0362,0363 |
| M+H | 751.3611 | 13.139 | 0364 |
| M+H | 752.2752 | 13.939 | 0365,0366,0367 |
| M+H | 752.2748 | 14.028 | 0365,0366,0367 |
| M-H | 750.2583 | 13.885 | 0365,0366,0367 |
| M-H | 751.2256 | 12.809 | 0368 |
| M+H | 753.2668 | 14.401 | 0369 |
| M+H | 754.1999 | 12.865 | 0370 |
| M+H | 754.2719 | 14.692 | 0371 |
| M+H | 755.2348 | 13.272 | 0372,0373 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 755.2349 | 13.416 | 0372,0373 |
| M+H | 755.2530 | 13.595 | 0374,0375 |
| M+H | 755.2535 | 13.676 | 0374,0375 |
| M+H | 755.3094 | 13.71 | 0376 |
| M+H | 756.2297 | 12.559 | 0377 |
| M+H | 756.2505 | 13.687 | 0378,0379,0380 |
| M+H | 756.2510 | 13.657 | 0378,0379,0380 |
| M+H | 756.2493 | 13.5 | 0378,0379,0380 |
| M+H | 756.3059 | 11.388 | 0381 |
| M+H | 757.2512 | 13.25 | 0382 |
| M+H | 757.3045 | 8.016 | 0383 |
| M+H | 758.2993 | 14.455 | 0384 |
| M+H | 759.3059 | 13.527 | 0385 |
| M+H | 759.3353 | 15.194 | 0386 |
| M+H | 760.2104 | 13.265 | 0387 |
| M-H | 758.2658 | 14.744 | 0388 |
| M+H | 761.1913 | 13.57 | 0389 |
| M-H | 759.2103 | 12.952 | 0390 |
| M+H | 761.2652 | 12.396 | 0391 |
| M+H | 761.2735 | 11.615 | 0392 |
| M+H | 762.2009 | 12.4 | 0393,0394 |
| M-H | 760.1876 | 13.079 | 0393,0394 |
| M+H | 762.2623 | 13.132 | 0395 |
| M-H | 760.2564 | 13.679 | 0396 |
| M+H | 763.2568 | 7.815 | 0397 |
| M-H | 761.2639 | 10.166 | 0398 |
| M+H | 764.2158 | 12.339 | 0399 |
| M+H | 764.2813 | 13.64 | 0400 |
| M+H | 764.3219 | 13.308 | 0401 |
| M+H | 765.2911 | 14.963 | 0402 |
| M+H | 766.2568 | 12.186 | 0403 |
| M+H | 767.1616 | 12.74 | 0404 |
| M+H | 767.2366 | 4.766 | 0405 |
| M+H | 768.2152 | 13.501 | 0406,0407 |
| M+H | 768.2151 | 13.601 | 0406,0407 |
| M+H | 769.2103 | 13.429 | 0408,0409,0410 |
| M-H | 767.1947 | 13.604 | 0408,0409,0410 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M-H | 767.1919 | 13.375 | 0408,0409,0410 |
| M-H | 767.2347 | 13.373 | 0411 |
| M+H | 769.3261 | 14.093 | 0412 |
| M+H | 770.2289 | 13.565 | 0413,0414 |
| M+H | 770.2278 | 13.45 | 0413,0414 |
| M+H | 771.2253 | 12.473 | 0415 |
| M+H | 771.2299 | 13.765 | 0415,0416 |
| M+H | 771.3164 | 12.108 | 0417 |
| M-H | 771.2038 | 12.994 | 0418 |
| M+H | 773.2415 | 13.023 | 0419 |
| M+H | 773.2782 | 14.639 | 0420 |
| M+H | 773.3000 | 13.776 | 0421 |
| M+H | 774.2366 | 13.51 | 0422 |
| M+H | 775.2361 | 12.859 | 0423,0424 |
| M-H | 773.2188 | 12.9 | 0423,0424 |
| M+H | 775.2828 | 13.877 | 0425 |
| M-H | 773.2797 | 13.824 | 0426 |
| M+H | 775.3423 | 13.722 | 0427 |
| M+H | 776.1864 | 13.662 | 0428 |
| M+H | 776.2410 | 13.03 | 0429 |
| M+H | 777.1998 | 13.077 | 0430 |
| M-H | 775.2594 | 13.524 | 0431 |
| M-H | 776.1638 | 13.265 | 0432 |
| M+H | 779.2352 | 14.085 | 0433,0434 |
| M-H | 777.2169 | 14.002 | 0433,0434 |
| M+H | 779.2565 | 13.543 | 0435 |
| M-H | 778.1752 | 12.934 | 0436 |
| M+H | 780.2311 | 14.079 | 0437,0438,0439 |
| M+H | 780.2313 | 14.165 | 0437,0438,0439 |
| M+H | 780.2309 | 14.117 | 0437,0438,0439 |
| M+H | 781.2503 | 13.888 | 0440,0441 |
| M+H | 781.2506 | 13.984 | 0440,0441 |
| M+H | 782.2462 | 13.165 | 0442 |
| M-H | 781.2500 | 13.769 | 0443 |
| M+H | 784.2611 | 13.246 | 0444 |
| M+H | 785.2570 | 13.994 | 0445 |
| M+H | 785.3317 | 12.49 | 0446 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 786.2534 | 12.749 | 0447 |
| M+H | 786.2601 | 12.707 | 0448 |
| M+H | 787.2072 | 14.165 | 0449 |
| M+H | 787.2473 | 10.972 | 0450 |
| M-H | 785.2439 | 13.508 | 0451 |
| M-H | 786.1997 | 13.065 | 0452 |
| M-H | 786.2017 | 13.644 | 0452,0453 |
| M+H | 789.3070 | 12.197 | 0454 |
| M+H | 790.2307 | 13.592 | 0455 |
| M+H | 790.3373 | 13.854 | 0456 |
| M+H | 791.2134 | 13.46 | 0457 |
| M+H | 792.2184 | 13.365 | 0458 |
| M+H | 793.1768 | 13.33 | 0459 |
| M+H | 794.2307 | 14.129 | 0460,0461 |
| M+H | 794.2302 | 14.208 | 0460,0461 |
| M+H | 795.2269 | 14.214 | 0462,0463,0464 |
| M+H | 795.2247 | 14.171 | 0462,0463,0464 |
| M+H | 795.2272 | 14.357 | 0462,0463,0464 |
| M-H | 795.2652 | 14.072 | 0465 |
| M+H | 797.3325 | 11.06 | 0466 |
| M-H | 797.2218 | 9.209 | 0467 |
| M+H | 799.2562 | 13.427 | 0468 |
| M+H | 800.2044 | 13.512 | 0469 |
| M+H | 800.2522 | 14.189 | 0470 |
| M+H | 801.2482 | 12.855 | 0471,0472 |
| M+H | 801.2529 | 13.515 | 0471,0472 |
| M+H | 801.3564 | 12.34 | 0473 |
| M+H | 802.2563 | 13.75 | 0474 |
| M-H | 801.2205 | 13.788 | 0475 |
| M+H | 804.1971 | 13.845 | 0476 |
| M+H | 805.2496 | 14.534 | 0477,0478 |
| M+H | 805.2507 | 14.618 | 0477,0478 |
| M+H | 806.2082 | 13.6 | 0479 |
| M+H | 806.2463 | 14.826 | 0480,0481,0482 |
| M+H | 806.2463 | 14.615 | 0480,0481,0482 |
| M+H | 806.2465 | 14.772 | 0480,0481,0482 |
| M+H | 810.2740 | 11.622 | 0483 |

(continued)

| [Table 9-8-6] Compound that may be contained in mixture 2-2-d1E02-2 | | | |
|---|---|---|---|
| Ion species | m/z [M+H]+ or [M-H]- | Retention time of MS peak (min) | No. of assigned compound (described with 2-2-d1E02-2- omitted) |
| M+H | 811.2235 | 14.048 | 0484 |
| M+H | 811.2716 | 14.51 | 0485 |
| M+H | 811.3479 | 13.076 | 0486 |
| M+H | 812.2698 | 13.283 | 0487 |
| M+H | 812.2774 | 14.343 | 0488 |
| M-H | 811.2596 | 14.171 | 0489 |
| M+H | 815.3229 | 12.747 | 0490 |
| M+H | 817.2287 | 14.045 | 0491 |
| M+H | 818.2339 | 14.127 | 0492 |
| M-H | 821.2792 | 14.674 | 0493 |
| M+H | 826.2202 | 14.082 | 0494 |
| M+H | 828.2843 | 12.817 | 0495 |
| M+H | 829.2528 | 14.462 | 0496 |
| M-H | 835.2219 | 13.676 | 0497 |
| M+H | 839.3036 | 13.242 | 0498 |
| M+H | 854.2985 | 13.417 | 0499 |
| M+H | 865.3185 | 13.788 | 0500 |

[Table 510]

| [Table 9-8-7] Compound that may be contained in mixture 2-2-dlE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 651.2269 | 14.068 | 0001 |
| M+H | 655.2942 | 13.917 | 0002 |
| M+H | 657.2742 | 13.643 | 0003 |
| M+H | 661.2484 | 14.304 | 0004,0005 |
| M+H | 661.2480 | 14.343 | 0004,0005 |
| M+H | 662.2430 | 13.278 | 0006 |
| M+H | 665.2403 | 14.099 | 0007 |
| M+H | 667.2037 | 14.433 | 0008 |
| M+H | 668.2195 | 12.405 | 0009 |
| M+H | 669.2174 | 14.124 | 0010 |
| M+H | 669.3107 | 14.295 | 0011 |
| M+H | 671.2901 | 14 | 0012 |
| M+H | 673.2862 | 13.886 | 0013 |
| M-H | 673.2463 | 13.729 | 0014,0015,0016 |

(continued)

| [Table 9-8-7] Compound that may be contained in mixture 2-2-dlE03-2 | | | |
|---|---|---|---|
| Ion species | m/z [M+H]+ or [M-H]- | Retention time of MS peak (min) | No. of assigned compound (described with 2-2-d1E03-2-omitted) |
| M+H | 675.2631 | 14.565 | 0014,0015,0016 |
| M+H | 675.2634 | 14.707 | 0014,0015,0016 |
| M+H | 676.2608 | 13.679 | 0017 |
| M+H | 677.2438 | 13.369 | 0018 |
| M-H | 677.2193 | 14.401 | 0019,0020,0021 |
| M+H | 679.2393 | 14.604 | 0020,0021 |
| M+H | 679.2391 | 11.397 | 0020,0021 |
| M+H | 680.2351 | 13.361 | 0022 |
| M+H | 681.2193 | 14.752 | 0023 |
| M+H | 681.2484 | 13.875 | 0024 |
| M+H | 681.3101 | 14.689 | 0025 |
| M+H | 682.2347 | 13.695 | 0026 |
| M+H | 683.2895 | 14.339 | 0027 |
| M+H | 684.1941 | 14.368 | 0028 |
| M+H | 685.1943 | 14.496 | 0029 |
| M+H | 685.2474 | 14.993 | 0030,0031 |
| M+H | 685.2473 | 14.907 | 0030,0031 |
| M+H | 686.2106 | 12.389 | 0032 |
| M+H | 686.2426 | 14.165 | 0033,0034,0035 |
| M+H | 686.2430 | 15.431 | 0033,0034,0035 |
| M+H | 686.2436 | 15.489 | 0033,0034,0035 |
| M+H | 687.2632 | 14.744 | 0036,0037 |
| M+H | 687.2633 | 9.711 | 0036,0037 |
| M+H | 688.2592 | 13.865 | 0038 |
| M+H | 690.2732 | 14.284 | 0039 |
| M+H | 691.2609 | 15.053 | 0040 |
| M+H | 691.2689 | 14.975 | 0041 |
| M+H | 692.2652 | 13.964 | 0042 |
| M+H | 693.2233 | 14.351 | 0043 |
| M-H | 691.2323 | 13.611 | 0044 |
| M+H | 693.2738 | 14.479 | 0045 |
| M+H | 694.2358 | 10.886 | 0046 |
| M+H | 695.2340 | 14.404 | 0047 |
| M+H | 695.2648 | 14.278 | 0048 |
| M+H | 695.3262 | 15.155 | 0049 |
| M+H | 697.2261 | 14.442 | 0050,0051 |
| M-H | 695.2080 | 14.4 | 0050,0051 |

(continued)

| [Table 9-8-7] Compound that may be contained in mixture 2-2-dIE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 697.3049 | 14.699 | 0052 |
| M+H | 698.2101 | 14.727 | 0053 |
| M+H | 699.2395 | 13.955 | 0054 |
| M+H | 699.2669 | 11.682 | 0055,0056 |
| M-H | 697.2449 | 15.235 | 0055,0056 |
| M-H | 697.2828 | 14.679 | 0057 |
| M+H | 700.2584 | 14.535 | 0058,0059,0060 |
| M+H | 700.2588 | 15.526 | 0058,0059,0060 |
| M+H | 700.2587 | 15.459 | 0058,0059,0060 |
| M+H | 701.2786 | 12.658 | 0061,0062,0063 |
| M+H | 701.2765 | 15.126 | 0061,0062,0063 |
| M+H | 701.2807 | 14.38 | 0061,0062,0063 |
| M+H | 702.1847 | 14.394 | 0064 |
| M+H | 702.2746 | 14.268 | 0065 |
| M+H | 703.2384 | 14.986 | 0066,0067 |
| M+H | 703.2382 | 14.943 | 0066,0067 |
| M+H | 703.2945 | 15.037 | 0068 |
| M+H | 704.2335 | 15.183 | 0069,0070,0071 |
| M+H | 704.2333 | 15.226 | 0069,0070,0071 |
| M+H | 704.2334 | 15.486 | 0069,0070,0071 |
| M+H | 704.2915 | 13.418 | 0072 |
| M-H | 703.2354 | 14.833 | 0073,0074,0075 |
| M+H | 705.2538 | 12.304 | 0073,0074,0075 |
| M-H | 703.2354 | 14.924 | 0073,0074,0075 |
| M+H | 705.2853 | 15.326 | 0076 |
| M+H | 706.2486 | 13.915 | 0077 |
| M+H | 706.2812 | 14.399 | 0078 |
| M+H | 707.2650 | 14.489 | 0080 |
| M+H | 707.2895 | 14.831 | 0081 |
| M+H | 708.1946 | 14.657 | 0082 |
| M-H | 706.2307 | 13.28 | 0083 |
| M+H | 708.2676 | 14.083 | 0084 |
| M+H | 709.2517 | 14.816 | 0085 |
| M+H | 709.2622 | 14.988 | 0086 |
| M+H | 710.2125 | 12.322 | 0087 |
| M+H | 710.2555 | 14.067 | 0088 |

(continued)

| [Table 9-8-7] Compound that may be contained in mixture 2-2-dIE03-2 | | | |
|---|---|---|---|
| Ion species | m/z [M+H]+ or [M-H]- | Retention time of MS peak (min) | No. of assigned compound (described with 2-2-d1E03-2-omitted) |
| M+H | 711.2127 | 16.218 | 0089 |
| M+H | 711.2426 | 14.766 | 0090 |
| M+H | 711.2629 | 15.459 | 0091,0092,0093 |
| M+H | 711.2631 | 15.037 | 0091,0092,0093 |
| M-H | 709.2459 | 14.549 | 0091,0092,0093 |
| M+H | 712.2256 | 12.76 | 0094 |
| M+H | 712.2621 | 13.715 | 0095,0096,0097,0098 |
| M+H | 712.2565 | 14.717 | 0095,0096,0097 |
| M+H | 712.2589 | 15.891 | 0095,0096,0097,0098 |
| M+H | 712.2625 | 14.515 | 0095,0096,0097,0098 |
| M+H | 714.2417 | 14.234 | 0099 |
| M+H | 715.1882 | 13.573 | 0100 |
| M+H | 715.2163 | 14.496 | 0101 |
| M+H | 716.2900 | 13.738 | 0102 |
| M-H | 715.2235 | 13.184 | 0103 |
| M+H | 717.2847 | 12.341 | 0104 |
| M+H | 717.3113 | 15.386 | 0105 |
| M+H | 718.2152 | 14.894 | 0106,0107 |
| M-H | 716.1966 | 14.793 | 0106,0107 |
| M+H | 718.2794 | 14.495 | 0108 |
| M+H | 719.2094 | 13.89 | 0109 |
| M-H | 717.1958 | 14.975 | 0109,0110 |
| M-H | 717.2399 | 13.488 | 0111 |
| M+H | 719.2653 | 14.116 | 0112 |
| M+H | 719.2870 | 10.669 | 0113 |
| M-H | 719.2183 | 13.233 | 0114 |
| M+H | 721.2805 | 14.764 | 0115,0116 |
| M+H | 721.2842 | 15.091 | 0115,0116 |
| M-H | 721.2288 | 14.672 | 0117,0118 |
| M+H | 723.2666 | 15.134 | 0119 |
| M+H | 723.2826 | 14.866 | 0120 |
| M+H | 724.1727 | 14.987 | 0121 |
| M+H | 724.2261 | 15.234 | 0122 |
| M+H | 725.1880 | 12.15 | 0123 |
| M+H | 725.2085 | 13.799 | 0124,0125 |
| M+H | 725.2086 | 13.64 | 0124,0125 |
| M+H | 725.2550 | 14.435 | 0126 |
| M+H | 725.2609 | 14.624 | 0127 |

(continued)

| [Table 9-8-7] Compound that may be contained in mixture 2-2-dIE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 725.2795 | 15.67 | 0128,0129 |
| M+H | 725.2795 | 15.747 | 0128,0129 |
| M+H | 726.2046 | 12.993 | 0130 |
| M+H | 726.2743 | 15.935 | 0131,0132,0133 |
| M+H | 726.2734 | 16.194 | 0131,0132,0133 |
| M+H | 726.2752 | 15.974 | 0131,0132,0133 |
| M+H | 727.2415 | 14.867 | 0134 |
| M+H | 728.2020 | 14.893 | 0135 |
| M+H | 729.2041 | 13.956 | 0136 |
| M+H | 729.2349 | 15.187 | 0137,0138 |
| M+H | 729.2346 | 15.241 | 0137,0138 |
| M+H | 729.2553 | 15.471 | 0139,0140 |
| M+H | 729.2533 | 11.102 | 0139,0140 |
| M+H | 729.3113 | 15.593 | 0141 |
| M+H | 730.2308 | 14.309 | 0142 |
| M+H | 730.2495 | 14.725 | 0143,0144,0145 |
| M+H | 730.2489 | 15.633 | 0143,0144,0145 |
| M+H | 730.2487 | 15.905 | 0143,0144,0145 |
| M+H | 730.3048 | 13.049 | 0146 |
| M+H | 731.1655 | 13.89 | 0147 |
| M+H | 731.2524 | 15.017 | 0148 |
| M+H | 731.3027 | 12.741 | 0149 |
| M+H | 732.1810 | 12.13 | 0150 |
| M+H | 732.2964 | 14.809 | 0151 |
| M+H | 733.1792 | 13.656 | 0152 |
| M+H | 733.2724 | 13.802 | 0153 |
| M+H | 733.3064 | 15.487 | 0154 |
| M+H | 734.2095 | 15.204 | 0155 |
| M+H | 734.2822 | 10.755 | 0156 |
| M+H | 735.1906 | 15.319 | 0157 |
| M+H | 735.2278 | 14.784 | 0158 |
| M+H | 735.2504 | 13.556 | 0159 |
| M-H | 733.2479 | 15.447 | 0160 |
| M+H | 735.2780 | 13.756 | 0161 |
| M+H | 736.2006 | 14.26 | 0162,0163 |
| M+H | 736.2012 | 12.863 | 0162,0163 |
| M+H | 736.2710 | 14.531 | 0164 |

(continued)

| [Table 9-8-7] Compound that may be contained in mixture 2-2-dIE03-2 | | | |
|---|---|---|---|
| Ion species | m/z [M+H]+ or [M-H]- | Retention time of MS peak (min) | No. of assigned compound (described with 2-2-d1E03-2-omitted) |
| M+H | 737.2459 | 11.96 | 0165 |
| M+H | 737.2539 | 9.574 | 0166 |
| M+H | 737.2793 | 15.126 | 0167 |
| M+H | 738.2159 | 14.441 | 0168 |
| M+H | 738.2778 | 15.258 | 0169 |
| M-H | 737.2103 | 13.295 | 0170,0171,0172 |
| M+H | 739.2258 | 13.989 | 0170,0171,0172 |
| M-H | 737.2085 | 14.182 | 0170,0171,0172 |
| M+H | 740.2201 | 13.457 | 0173 |
| M+H | 740.2571 | 14.947 | 0174 |
| M+H | 741.1619 | 14.849 | 0175 |
| M-H | 739.1875 | 13.146 | 0176 |
| M+H | 741.2313 | 14.968 | 0177 |
| M+H | 742.2149 | 15.494 | 0178,0179 |
| M+H | 742.2149 | 15.384 | 0178,0179 |
| M-H | 741.1782 | 13.428 | 0180 |
| M+H | 743.1990 | 13.887 | 0180,0181,0182 |
| M+H | 743.1990 | 13.727 | 0180,0181,0182 |
| M+H | 743.2106 | 14.72 | 0183,0184,0185 |
| M+H | 743.2114 | 15.694 | 0183,0184,0185 |
| M+H | 743.2114 | 15.987 | 0183,0184,0185 |
| M+H | 743.2570 | 13.639 | 0186 |
| M+H | 743.3236 | 11.585 | 0187 |
| M-H | 742.1780 | 13.146 | 0188 |
| M+H | 744.2306 | 15.319 | 0189,0190 |
| M+H | 744.2274 | 9.065 | 0189,0190 |
| M+H | 745.1824 | 14.28 | 0191 |
| M+H | 745.2109 | 13.28 | 0192 |
| M+H | 745.2253 | 14.497 | 0193,0194 |
| M-H | 743.2131 | 15.452 | 0193,0194 |
| M+H | 745.2728 | 14.278 | 0195 |
| M+H | 745.3158 | 14.195 | 0196 |
| M+H | 746.1950 | 13.814 | 0197 |
| M-H | 745.2021 | 14.672 | 0198 |
| M+H | 747.2420 | 14.814 | 0199 |
| M+H | 747.2502 | 13.969 | 0200 |
| M-H | 745.2827 | 15.554 | 0201 |

(continued)

| [Table 9-8-7] Compound that may be contained in mixture 2-2-dlE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 748.1553 | 13.711 | 0202 |
| M+H | 748.2362 | 15.493 | 0203 |
| M+H | 749.1550 | 13.995 | 0204 |
| M-H | 747.1914 | 14.417 | 0205,0206 |
| M+H | 749.2108 | 13.667 | 0205,0206 |
| M+H | 749.2314 | 15.079 | 0207,0208 |
| M-H | 747.2175 | 14.829 | 0207,0208 |
| M-H | 747.2643 | 14.269 | 0209 |
| M+H | 749.2980 | 15.531 | 0210 |
| M+H | 750.1714 | 12.119 | 0211 |
| M+H | 750.1887 | 15.514 | 0212 |
| M+H | 750.2054 | 14.53 | 0213,0214,0215 |
| M-H | 748.1879 | 14.443 | 0213,0214,0215 |
| M-H | 748.1867 | 14.5 | 0213,0214,0215 |
| M+H | 750.2413 | 15.032 | 0216 |
| M+H | 751.2020 | 14.94 | 0217 |
| M-H | 749.2086 | 13.446 | 0218,0219 |
| M-H | 749.2095 | 12.025 | 0218,0219 |
| M+H | 751.2766 | 15.198 | 0220 |
| M+H | 752.1810 | 15.227 | 0221 |
| M+H | 752.2204 | 12.702 | 0222 |
| M+H | 753.2360 | 15.745 | 0223,0224 |
| M+H | 753.2360 | 15.797 | 0223,0224 |
| M+H | 753.2565 | 15.484 | 0225 |
| M+H | 754.1934 | 14.949 | 0226 |
| M+H | 754.2291 | 15.086 | 0227,0228,0229 |
| M+H | 754.2301 | 15.937 | 0227,0228,0229 |
| M+H | 754.2306 | 15.98 | 0227,0228,0229 |
| M+H | 754.2332 | 13.63 | 0227,0228,0229,0230 |
| M+H | 755.2209 | 13.429 | 0231 |
| M+H | 755.2303 | 14.289 | 0232 |
| M+H | 755.2508 | 15.661 | 0233,0234 |
| M+H | 755.2508 | 15.584 | 0233,0234 |
| M+H | 756.2264 | 13.158 | 0235 |
| M+H | 756.2464 | 14.819 | 0236 |
| M+H | 757.1804 | 13.481 | 0237 |
| M-H | 755.1977 | 12.81 | 0238 |

(continued)

| [Table 9-8-7] Compound that may be contained in mixture 2-2-dlE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 757.2355 | 13.842 | 0239 |
| M+H | 757.2667 | 15.474 | 0240 |
| M-H | 756.1770 | 14.039 | 0241 |
| M+H | 758.2603 | 14.121 | 0242 |
| M+H | 759.1951 | 14.199 | 0243 |
| M+H | 759.2265 | 13.671 | 0244 |
| M+H | 759.2574 | 15.811 | 0245 |
| M+H | 759.2863 | 14.65 | 0246 |
| M+H | 759.3317 | 14.533 | 0247 |
| M+H | 760.2528 | 14.99 | 0248 |
| M+H | 760.2618 | 15.537 | 0249 |
| M+H | 761.1877 | 13.823 | 0250,0251 |
| M+H | 761.1878 | 14.09 | 0250,0251 |
| M+H | 761.2040 | 12.795 | 0252 |
| M+H | 761.2474 | 11.056 | 0253 |
| M+H | 761.2618 | 15.328 | 0254 |
| M+H | 761.2663 | 14.357 | 0254,0255 |
| M+H | 762.1702 | 14.086 | 0256 |
| M+H | 762.2159 | 14.966 | 0257,0258 |
| M+H | 762.2203 | 13.494 | 0257,0258 |
| M+H | 763.2013 | 13.39 | 0259 |
| M+H | 763.2254 | 14.682 | 0260,0261 |
| M+H | 763.2254 | 14.632 | 0260,0261 |
| M-H | 761.2449 | 14.406 | 0262 |
| M+H | 763.3077 | 14.255 | 0263 |
| M-H | 762.2026 | 14.94 | 0264,0265,0266 |
| M-H | 762.2027 | 14.811 | 0264,0265,0266 |
| M-H | 762.2026 | 14.882 | 0264,0265,0266 |
| M+H | 764.2314 | 15.029 | 0267 |
| M+H | 765.2132 | 15.32 | 0268 |
| M-H | 763.2226 | 14.02 | 0269,0270,0271 |
| M-H | 763.2226 | 14.056 | 0269,0270,0271 |
| M+H | 765.2421 | 13.987 | 0269,0270,0271 |
| M-H | 764.1295 | 13.792 | 0272 |
| M+H | 766.2195 | 15.383 | 0273 |
| M+H | 766.2369 | 13.084 | 0274 |
| M+H | 767.1766 | 15.357 | 0275 |

(continued)

| [Table 9-8-7] Compound that may be contained in mixture 2-2-dlE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 767.1997 | 14.397 | 0276,0277 |
| M-H | 765.1820 | 14.488 | 0276,0277 |
| M+H | 767.2561 | 14.403 | 0278 |
| M+H | 768.1973 | 14.649 | 0279,0280,0281 |
| M-H | 766.1801 | 14.517 | 0279,0280,0281 |
| M+H | 768.1970 | 14.583 | 0279,0280,0281 |
| M+H | 768.2313 | 15.978 | 0282,0283 |
| M+H | 768.2313 | 15.86 | 0282,0283 |
| M+H | 768.2495 | 13.988 | 0284 |
| M-H | 767.1927 | 14.219 | 0285 |
| M+H | 769.2156 | 14.218 | 0285,0286,0287 |
| M+H | 769.2156 | 14.304 | 0285,0286,0287 |
| M+H | 769.2264 | 16.414 | 0288,0289,0290 |
| M+H | 769.2253 | 15.28 | 0288,0289,0290 |
| M+H | 769.2264 | 16.157 | 0288,0289,0290 |
| M+H | 769.2474 | 14.67 | 0291 |
| M+H | 770.2101 | 13.728 | 0292 |
| M+H | 770.2420 | 13.537 | 0293 |
| M+H | 771.1965 | 14.511 | 0294 |
| M+H | 771.2255 | 13.932 | 0295 |
| M+H | 771.2520 | 14.201 | 0296 |
| M+H | 771.2825 | 15.759 | 0297 |
| M+H | 771.3322 | 14.658 | 0298 |
| M+H | 772.1561 | 14.114 | 0299 |
| M+H | 772.2092 | 14.7 | 0300 |
| M-H | 770.2077 | 13.707 | 0301 |
| M+H | 773.2117 | 14.158 | 0302 |
| M+H | 773.2225 | 14.406 | 0303 |
| M-H | 771.2216 | 11.538 | 0304 |
| M+H | 773.2577 | 15.369 | 0305 |
| M+H | 774.1691 | 14.265 | 0306 |
| M+H | 774.2083 | 12.944 | 0307 |
| M+H | 774.2168 | 13.233 | 0308 |
| M+H | 774.2524 | 16.102 | 0309 |
| M+H | 775.1700 | 14.566 | 0310 |
| M+H | 775.2031 | 14.213 | 0311 |
| M+H | 775.2250 | 14.866 | 0312,0313,0314 |

(continued)

| [Table 9-8-7] Compound that may be contained in mixture 2-2-dlE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 775.2219 | 11.587 | 0312,0313,0314 |
| M+H | 775.2250 | 14.949 | 0312,0313,0314 |
| M+H | 775.2485 | 15.256 | 0315,0316 |
| M+H | 775.2485 | 15.536 | 0315,0316 |
| M+H | 776.1870 | 12.587 | 0317 |
| M+H | 776.2244 | 13.333 | 0318,0319,0320,0321 |
| M+H | 776.2228 | 14.956 | 0318,0319,0320,0321 |
| M+H | 776.2192 | 15.087 | 0318,0319,0320,0321 |
| M+H | 776.2258 | 14.012 | 0321 |
| M+H | 776.2572 | 15.666 | 0322 |
| M+H | 777.2393 | 15.597 | 0323 |
| M-H | 776.1843 | 13.823 | 0324,0325 |
| M+H | 778.2034 | 13.049 | 0325 |
| M+H | 779.1776 | 13.917 | 0326 |
| M-H | 777.2324 | 16.054 | 0327,0328 |
| M-H | 777.2315 | 16.163 | 0327,0328 |
| M+H | 780.2076 | 15.482 | 0329 |
| M+H | 780.2451 | 16.331 | 0330,0331,0332 |
| M+H | 780.2449 | 16.573 | 0330,0331,0332 |
| M+H | 780.2459 | 16.533 | 0330,0331,0332 |
| M+H | 780.2532 | 16.066 | 0333 |
| M+H | 781.2001 | 14.371 | 0334 |
| M+H | 781.2459 | 11.879 | 0335 |
| M+H | 781.2704 | 14.767 | 0336 |
| M+H | 782.1762 | 14.217 | 0337,0338 |
| M+H | 782.1761 | 14.362 | 0337,0338 |
| M+H | 782.2389 | 14.054 | 0339 |
| M+H | 783.1716 | 13.741 | 0340,0341 |
| M+H | 783.1748 | 14.561 | 0340,0341 |
| M+H | 783.2247 | 14.435 | 0342 |
| M-H | 781.2368 | 14.5 | 0343 |
| M-H | 782.2604 | 15.08 | 0344 |
| M+H | 785.2228 | 15.563 | 0345 |
| M+H | 785.2426 | 14.294 | 0346,0347 |
| M-H | 783.2284 | 14.416 | 0346,0347 |
| M+H | 785.2733 | 16.256 | 0348 |
| M+H | 785.3482 | 15.026 | 0349 |

(continued)

| [Table 9-8-7] Compound that may be contained in mixture 2-2-dIE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 786.2691 | 15.393 | 0350 |
| M+H | 786.2773 | 16.139 | 0351 |
| M+H | 787.2015 | 14.191 | 0352,0353 |
| M-H | 785.1896 | 13.117 | 0352,0353 |
| M+H | 787.2293 | 14.499 | 0354 |
| M-H | 785.2246 | 14.298 | 0355 |
| M-H | 785.2578 | 15.886 | 0356 |
| M+H | 788.1329 | 14.498 | 0357 |
| M-H | 786.1700 | 14.625 | 0358 |
| M+H | 789.1458 | 14.07 | 0359 |
| M-H | 787.2053 | 14.181 | 0360,0361 |
| M+H | 789.2210 | 14.239 | 0360,0361 |
| M+H | 789.2407 | 15.275 | 0362,0363 |
| M+H | 789.2407 | 15.194 | 0362,0363 |
| M+H | 789.3226 | 14.659 | 0364 |
| M+H | 790.2381 | 15.991 | 0365,0366,0367 |
| M+H | 790.2395 | 15.646 | 0365,0366,0367 |
| M+H | 790.2395 | 15.6 | 0365,0366,0367 |
| M+H | 791.2040 | 14.229 | 0368 |
| M+H | 791.2285 | 15.717 | 0369 |
| M+H | 792.1639 | 14.334 | 0370 |
| M-H | 790.2147 | 15.997 | 0371 |
| M+H | 793.1969 | 14.606 | 0372,0373 |
| M+H | 793.1959 | 14.739 | 0372,0373 |
| M+H | 793.2156 | 14.973 | 0374,0375 |
| M+H | 793.2156 | 14.946 | 0374,0375 |
| M+H | 793.2688 | 15.035 | 0376 |
| M+H | 794.1923 | 14.311 | 0377 |
| M+H | 794.2109 | 15.254 | 0378,0379,0380 |
| M+H | 794.2130 | 14.991 | 0378,0379,0380 |
| M+H | 794.2109 | 15.313 | 0378,0379,0380 |
| M+H | 794.2685 | 13.065 | 0381 |
| M+H | 795.2131 | 14.764 | 0382 |
| M+H | 795.2666 | 9.368 | 0383 |
| M+H | 796.2614 | 16.105 | 0384 |
| M+H | 797.2686 | 14.898 | 0385 |
| M+H | 797.2958 | 16.263 | 0386 |

(continued)

| [Table 9-8-7] Compound that may be contained in mixture 2-2-dlE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 798.1749 | 13.879 | 0387 |
| M-H | 796.2257 | 15.734 | 0388 |
| M-H | 797.1359 | 14.905 | 0389 |
| M+H | 799.1903 | 14.45 | 0390 |
| M+H | 799.2261 | 13.91 | 0391 |
| M+H | 799.2382 | 14.95 | 0392 |
| M+H | 800.1625 | 14.017 | 0393,0394 |
| M-H | 798.1477 | 14.492 | 0393,0394 |
| M+H | 800.2209 | 15.744 | 0395 |
| M+H | 800.2326 | 13.819 | 0396 |
| M+H | 801.2186 | 9.123 | 0397 |
| M+H | 801.2413 | 14.548 | 0398 |
| M-H | 800.1608 | 13.883 | 0399 |
| M+H | 802.2402 | 14.819 | 0400 |
| M+H | 802.2840 | 14.81 | 0401 |
| M+H | 803.2580 | 15.275 | 0402 |
| M+H | 804.2200 | 14.591 | 0403 |
| M+H | 805.1245 | 14.227 | 0404 |
| M+H | 805.1945 | 14.44 | 0405 |
| M+H | 806.1773 | 14.944 | 0406,0407 |
| M+H | 806.1773 | 14.812 | 0406,0407 |
| M+H | 807.1718 | 15.172 | 0408,0409,0410 |
| M+H | 807.1718 | 15.121 | 0408,0409,0410 |
| M-H | 805.1599 | 15.288 | 0408,0409,0410 |
| M+H | 807.2124 | 13.641 | 0411 |
| M+H | 807.2896 | 15.378 | 0412 |
| M+H | 808.1936 | 14.316 | 0413,0414 |
| M+H | 808.1899 | 14.178 | 0413,0414 |
| M+H | 809.1876 | 14.311 | 0415,0416 |
| M+H | 809.1948 | 14.805 | 0416 |
| M+H | 809.2776 | 13.594 | 0417 |
| M-H | 809.1633 | 14.391 | 0418 |
| M+H | 811.2026 | 14.812 | 0419 |
| M+H | 811.2412 | 15.842 | 0420 |
| M+H | 811.2645 | 15.088 | 0421 |
| M+H | 812.1985 | 14.898 | 0422 |
| M-H | 811.1792 | 14.297 | 0423,0424 |

(continued)

| [Table 9-8-7] Compound that may be contained in mixture 2-2-dIE03-2 | | | |
|---|---|---|---|
| Ion species | m/z [M+H]+ or [M-H]- | Retention time of MS peak (min) | No. of assigned compound (described with 2-2-d1E03-2- omitted) |
| M-H | 811.1792 | 14.251 | 0423,0424 |
| M+H | 813.2461 | 15.21 | 0425 |
| M-H | 811.2413 | 15.142 | 0426 |
| M+H | 813.3028 | 15.086 | 0427 |
| M+H | 814.1488 | 15.078 | 0428 |
| M+H | 814.2029 | 13.614 | 0429 |
| M+H | 815.1618 | 14.559 | 0430 |
| M+H | 815.2371 | 14.826 | 0431 |
| M+H | 816.1447 | 14.638 | 0432 |
| M-H | 815.1793 | 15.244 | 0433,0434 |
| M-H | 815.1778 | 15.31 | 0433,0434 |
| M+H | 817.2194 | 14.809 | 0435 |
| M+H | 818.1547 | 14.374 | 0436 |
| M+H | 818.1942 | 15.292 | 0437,0438,0439 |
| M+H | 818.1909 | 15.73 | 0437,0438,0439 |
| M+H | 818.1927 | 15.668 | 0437,0438,0439 |
| M+H | 819.2116 | 15.127 | 0440,0441 |
| M+H | 819.2114 | 15.191 | 0440,0441 |
| M+H | 820.2074 | 14.82 | 0442 |
| M+H | 821.2291 | 15.256 | 0443 |
| M+H | 822.2211 | 14.564 | 0444 |
| M+H | 823.2186 | 15.186 | 0445 |
| M+H | 823.2941 | 13.915 | 0446 |
| M+H | 824.2150 | 14.183 | 0447 |
| M+H | 824.2245 | 14.959 | 0448 |
| M+H | 825.1702 | 15.398 | 0449 |
| M-H | 823.2046 | 14.752 | 0451 |
| M+H | 826.1780 | 14.726 | 0452,0453 |
| M+H | 826.1811 | 15.23 | 0452,0453 |
| M+H | 827.2688 | 13.672 | 0454 |
| M+H | 828.1930 | 14.494 | 0455 |
| M+H | 828.2994 | 15.307 | 0456 |
| M-H | 827.1560 | 14.769 | 0457 |
| M+H | 830.1805 | 14.728 | 0458 |
| M+H | 831.1376 | 14.756 | 0459 |
| M+H | 832.1937 | 15.476 | 0460,0461 |
| M-H | 830.1737 | 15.473 | 0460,0461 |

(continued)

| [Table 9-8-7] Compound that may be contained in mixture 2-2-dIE03-2 | | | |
|---|---|---|---|
| Ion species | m/z [M+H]+ or [M-H]- | Retention time of MS peak (min) | No. of assigned compound (described with 2-2-d1E03-2- omitted) |
| M+H | 833.1875 | 15.874 | 0462,0463,0464 |
| M+H | 833.1886 | 16.048 | 0462,0463,0464 |
| M+H | 833.1889 | 16.179 | 0462,0463,0464 |
| M+H | 835.2440 | 15.267 | 0465 |
| M+H | 835.2945 | 14.089 | 0466 |
| M-H | 835.1829 | 11.108 | 0467 |
| M+H | 837.2193 | 14.787 | 0468 |
| M+H | 838.1672 | 14.963 | 0469 |
| M+H | 838.2146 | 15.499 | 0470 |
| M+H | 839.2092 | 14.376 | 0471,0472 |
| M-H | 837.2003 | 14.861 | 0472 |
| M+H | 839.3192 | 15.479 | 0473 |
| M+H | 840.2166 | 14.363 | 0474 |
| M-H | 839.1832 | 14.631 | 0475 |
| M-H | 840.1410 | 15.126 | 0476 |
| M+H | 843.2113 | 15.75 | 0477,0478 |
| M+H | 843.2125 | 15.697 | 0477,0478 |
| M+H | 844.1714 | 14.967 | 0479 |
| M+H | 844.2086 | 16.277 | 0480,0481,0482 |
| M+H | 844.2086 | 16.191 | 0480,0481,0482 |
| M-H | 842.1927 | 12.124 | 0480,0481,0482 |
| M+H | 848.2378 | 15.074 | 0483 |
| M+H | 849.1874 | 15.38 | 0484 |
| M+H | 849.2343 | 15.735 | 0485 |
| M+H | 849.3088 | 14.489 | 0486 |
| M+H | 850.2305 | 14.699 | 0487 |
| M+H | 850.2391 | 15.594 | 0488 |
| M+H | 851.2388 | 11.398 | 0489 |
| M+H | 853.2834 | 14.188 | 0490 |
| M+H | 855.1907 | 15.233 | 0491 |
| M+H | 856.1962 | 15.412 | 0492 |
| M+H | 861.2609 | 15.75 | 0493 |
| M+H | 864.1827 | 15.521 | 0494 |
| M+H | 866.2456 | 14.207 | 0495 |
| M-H | 865.2015 | 14.874 | 0496 |
| M+H | 875.2042 | 15.71 | 0497 |
| M+H | 877.2662 | 14.48 | 0498 |

(continued)

| [Table 9-8-7] Compound that may be contained in mixture 2-2-dlE03-2 | | | |
|---|---|---|---|
| Ion species | m/z [M+H]+ or [M-H]- | Retention time of MS peak (min) | No. of assigned compound (described with 2-2-d1E03-2-omitted) |
| M+H | 892.2604 | 14.77 | 0499 |
| M+H | 903.2817 | 14.992 | 0500 |

[Table 511]

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2-omitted) |
| M+H | 764.3103 | 8.81 | 0001 |
| M+H | 768.3779 | 8.664 | 0002 |
| M+H | 770.3577 | 8.451 | 0003 |
| M+H | 774.3309 | 8.988 | 0004,0005 |
| M+H | 774.3309 | 9.026 | 0004,0005 |
| M+H | 775.3263 | 8.248 | 0006 |
| M+H | 778.3261 | 9.095 | 0007 |
| M-H | 778.2712 | 9.073 | 0008 |
| M+H | 781.3024 | 7.498 | 0009 |
| M+H | 782.3010 | 8.909 | 0010 |
| M+H | 782.3938 | 8.937 | 0011 |
| M+H | 784.3728 | 8.749 | 0012 |
| M+H | 786.3711 | 9.318 | 0013 |
| M+H | 788.3475 | 8.553 | 0014,0015,0016 |
| M+H | 788.3473 | 8.424 | 0014,0015,0016 |
| M+H | 788.3466 | 9.278 | 0014,0015,0016 |
| M+H | 789.3418 | 8.541 | 0017 |
| M+H | 790.3264 | 9.211 | 0018 |
| M+H | 792.3164 | 8.363 | 0019 |
| M+H | 792.3219 | 9.095 | 0019,0020,0021 |
| M+H | 792.3220 | 9.128 | 0019,0020,0021 |
| M+H | 793.3162 | 8.91 | 0022 |
| M+H | 794.3028 | 9.381 | 0023 |
| M+H | 794.3326 | 8.647 | 0024 |
| M+H | 794.3943 | 9.179 | 0025 |
| M+H | 795.3162 | 8.883 | 0026 |
| M-H | 794.3565 | 8.936 | 0027 |
| M+H | 797.2775 | 8.928 | 0028 |
| M+H | 798.2785 | 9.2 | 0029 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 798.3317 | 9.588 | 0030,0031 |
| M+H | 798.3302 | 9.621 | 0030,0031 |
| M+H | 799.2930 | 7.751 | 0032 |
| M+H | 799.3262 | 8.944 | 0033,0034,0035 |
| M+H | 799.3259 | 9.78 | 0033,0034,0035 |
| M+H | 799.3261 | 8.868 | 0033,0034,0035 |
| M+H | 800.3464 | 9.416 | 0036,0037 |
| M+H | 801.3421 | 8.692 | 0038 |
| M+H | 803.3572 | 7.782 | 0039 |
| M+H | 804.3410 | 9.486 | 0040 |
| M+H | 804.3527 | 9.297 | 0041 |
| M+H | 805.3480 | 8.692 | 0042 |
| M+H | 806.3004 | 10.398 | 0043 |
| M+H | 806.3358 | 8.478 | 0044 |
| M+H | 806.3576 | 9.16 | 0045 |
| M+H | 807.3196 | 7.82 | 0046 |
| M+H | 808.3162 | 9.275 | 0047 |
| M+H | 808.3473 | 8.915 | 0048 |
| M+H | 808.4089 | 9.46 | 0049 |
| M+H | 810.3099 | 9.045 | 0050,0051 |
| M+H | 810.3096 | 8.994 | 0050,0051 |
| M+H | 810.3884 | 8.616 | 0052 |
| M+H | 811.2938 | 9.195 | 0053 |
| M+H | 812.3229 | 8.735 | 0054 |
| M+H | 812.3463 | 9.853 | 0055,0056 |
| M+H | 812.3462 | 9.891 | 0055,0056 |
| M+H | 812.3824 | 9.273 | 0057 |
| M+H | 813.3423 | 9.227 | 0058,0059,0060 |
| M+H | 813.3421 | 10.054 | 0058,0059,0060 |
| M+H | 813.3422 | 9.241 | 0058,0059,0060 |
| M+H | 814.3639 | 8.989 | 0061,0062,0063 |
| M+H | 814.3634 | 9.712 | 0061,0062,0063 |
| M-H | 812.3472 | 9.109 | 0061,0062,0063 |
| M-H | 813.2518 | 8.999 | 0064 |
| M+H | 815.3570 | 8.933 | 0065 |
| M+H | 816.3206 | 9.707 | 0066,0067 |
| M+H | 816.3209 | 10.574 | 0066,0067 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 816.3781 | 9.6 | 0068 |
| M+H | 817.3166 | 9.045 | 0069,0070,0071 |
| M+H | 817.3166 | 9.872 | 0069,0070,0071 |
| M+H | 817.3163 | 9.845 | 0069,0070,0071 |
| M+H | 817.3743 | 9.311 | 0072 |
| M+H | 818.3319 | 10.054 | 0073 |
| M+H | 818.3367 | 9.505 | 0073,0074,0075 |
| M+H | 818.3684 | 9.584 | 0076 |
| M+H | 819.3329 | 8.797 | 0077 |
| M+H | 819.3637 | 8.984 | 0078 |
| M+H | 820.3161 | 10.021 | 0079 |
| M+H | 820.3465 | 8.909 | 0080 |
| M+H | 820.3730 | 9.424 | 0081 |
| M+H | 821.2771 | 9.238 | 0082 |
| M-H | 819.3150 | 8.259 | 0083 |
| M+H | 821.3478 | 7.917 | 0084 |
| M+H | 822.3346 | 9.507 | 0085 |
| M+H | 822.3410 | 9.383 | 0086 |
| M+H | 823.2923 | 9.275 | 0087 |
| M-H | 821.3223 | 8.809 | 0088 |
| M+H | 824.2940 | 9.622 | 0089 |
| M+H | 824.3248 | 9.279 | 0090 |
| M+H | 824.3462 | 9.976 | 0091,0092,0093 |
| M+H | 824.3460 | 9.248 | 0091,0092,0093 |
| M+H | 824.3476 | 9.219 | 0091,0092,0093 |
| M+H | 825.3088 | 7.973 | 0094 |
| M+H | 825.3415 | 9.333 | 0095,0096,0097,0098 |
| M+H | 825.3418 | 10.139 | 0095,0096,0097,0098 |
| M+H | 825.3422 | 8.796 | 0095,0096,0097,0098 |
| M+H | 825.3425 | 9.894 | 0095,0096,0097,0098 |
| M+H | 827.3244 | 8.913 | 0099 |
| M+H | 828.2721 | 8.585 | 0100 |
| M+H | 828.2998 | 9.089 | 0101 |
| M+H | 829.3721 | 8.21 | 0102 |
| M+H | 830.3236 | 8.342 | 0103 |
| M+H | 830.3681 | 7.828 | 0104 |
| M+H | 830.3930 | 9.869 | 0105 |
| M+H | 831.2979 | 9.463 | 0106,0107 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 831.2975 | 8.887 | 0106,0107 |
| M+H | 831.3666 | 9.672 | 0108 |
| M+H | 832.2973 | 9.649 | 0109,0110 |
| M+H | 832.2976 | 10.399 | 0109,0110 |
| M+H | 832.3395 | 8.528 | 0111 |
| M-H | 830.3315 | 8.924 | 0112 |
| M+H | 832.3700 | 9.216 | 0113 |
| M-H | 832.3055 | 8.255 | 0114 |
| M+H | 834.3621 | 9.202 | 0115 |
| M+H | 834.3684 | 9.711 | 0115,0116 |
| M-H | 834.3068 | 9.2 | 0117,0118 |
| M+H | 836.3217 | 14.576 | 0117,0118 |
| M+H | 836.3500 | 9.741 | 0119 |
| M+H | 836.3653 | 9.522 | 0120 |
| M+H | 837.2548 | 9.561 | 0121 |
| M+H | 837.3127 | 7.542 | 0122 |
| M+H | 838.2704 | 7.901 | 0123 |
| M+H | 838.2928 | 8.775 | 0124,0125 |
| M+H | 838.2927 | 9.814 | 0124,0125 |
| M+H | 838.3386 | 9.061 | 0126 |
| M+H | 838.3452 | 9.332 | 0127 |
| M+H | 838.3623 | 10.238 | 0128,0129 |
| M+H | 838.3624 | 10.153 | 0128,0129 |
| M+H | 839.2880 | 7.948 | 0130 |
| M+H | 839.3573 | 9.603 | 0131,0132,0133 |
| M+H | 839.3584 | 10.147 | 0131,0132,0133 |
| M+H | 839.3581 | 10.414 | 0131,0132,0133 |
| M+H | 840.3244 | 9.588 | 0134 |
| M+H | 841.2849 | 9.324 | 0135 |
| M+H | 842.2879 | 8.883 | 0136 |
| M+H | 842.3185 | 9.861 | 0137,0138 |
| M+H | 842.3181 | 10.628 | 0137,0138 |
| M+H | 842.3371 | 10.043 | 0139,0140 |
| M+H | 842.3370 | 9.987 | 0139,0140 |
| M+H | 842.3905 | 10 | 0141 |
| M+H | 843.3135 | 9.138 | 0142 |
| M+H | 843.3324 | 9.417 | 0143,0144,0145 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 843.3323 | 10.215 | 0143,0144,0145 |
| M+H | 843.3327 | 9.952 | 0143,0144,0145 |
| M+H | 843.3879 | 8.575 | 0146 |
| M-H | 842.2325 | 8.856 | 0147 |
| M+H | 844.3363 | 9.672 | 0148 |
| M+H | 844.3846 | 8.112 | 0149 |
| M-H | 843.2451 | 8.569 | 0150 |
| M+H | 845.3785 | 9.309 | 0151 |
| M+H | 846.2621 | 8.699 | 0152 |
| M+H | 846.3556 | 8.82 | 0153 |
| M+H | 846.3877 | 9.866 | 0154 |
| M+H | 847.2933 | 9.625 | 0155 |
| M+H | 847.3663 | 9.127 | 0156 |
| M+H | 848.2740 | 9.975 | 0157 |
| M+H | 848.3099 | 9.506 | 0158 |
| M+H | 848.3351 | 8.636 | 0159 |
| M+H | 848.3498 | 9.957 | 0160 |
| M+H | 848.3577 | 7.929 | 0161 |
| M+H | 849.2843 | 8.368 | 0162,0163 |
| M+H | 849.3536 | 9.126 | 0164 |
| M-H | 848.3146 | 9.942 | 0165 |
| M-H | 848.3289 | 8.898 | 0166 |
| M+H | 850.3632 | 9.676 | 0167 |
| M+H | 851.2993 | 9.07 | 0168 |
| M-H | 849.3442 | 9.605 | 0169 |
| M+H | 852.3093 | 9.042 | 0170,0171,0172 |
| M+H | 852.3099 | 8.456 | 0170,0171,0172 |
| M+H | 852.3087 | 9.076 | 0170,0171,0172 |
| M+H | 853.3035 | 8.306 | 0173 |
| M+H | 853.3413 | 10.166 | 0174 |
| M+H | 854.2442 | 9.305 | 0175 |
| M+H | 854.2860 | 8.856 | 0176 |
| M+H | 854.3147 | 9.43 | 0177 |
| M+H | 855.2981 | 10.042 | 0178,0179 |
| M+H | 855.2974 | 10.815 | 0178,0179 |
| M-H | 854.2665 | 8.85 | 0180,0181,0182 |
| M-H | 854.2665 | 8.884 | 0180,0181,0182 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 856.2818 | 8.209 | 0180,0181,0182 |
| M+H | 856.2927 | 9.381 | 0183,0184,0185 |
| M+H | 856.2931 | 10.215 | 0183,0184,0185 |
| M+H | 856.2928 | 9.996 | 0183,0184,0185 |
| M+H | 856.3363 | 9.738 | 0186 |
| M+H | 856.4104 | 10.299 | 0187 |
| M+H | 857.2787 | 8.115 | 0188 |
| M+H | 857.3128 | 9.926 | 0189,0190 |
| M+H | 857.3127 | 9.851 | 0189,0190 |
| M-H | 856.2478 | 9.17 | 0191 |
| M+H | 858.2931 | 8.406 | 0192 |
| M+H | 858.3084 | 9.135 | 0193 |
| M+H | 858.3118 | 10.008 | 0193,0194 |
| M+H | 858.3557 | 8.99 | 0195 |
| M+H | 858.3990 | 9.09 | 0196 |
| M+H | 859.2766 | 7.867 | 0197 |
| M-H | 858.2872 | 9.219 | 0198 |
| M+H | 860.3287 | 9.276 | 0199 |
| M+H | 860.3338 | 8.856 | 0200 |
| M+H | 860.3842 | 10.099 | 0201 |
| M+H | 861.2388 | 8.693 | 0202 |
| M+H | 861.3189 | 9.708 | 0203 |
| M+H | 862.2394 | 8.987 | 0204 |
| M+H | 862.2939 | 9.391 | 0205,0206 |
| M+H | 862.2933 | 10.586 | 0205,0206 |
| M+H | 862.3158 | 9.199 | 0207,0208 |
| M+H | 862.3215 | 8.646 | 0208 |
| M+H | 862.3659 | 10.21 | 0209 |
| M+H | 862.3835 | 9.311 | 0210 |
| M+H | 863.2511 | 8.691 | 0211 |
| M+H | 863.2700 | 10.025 | 0212 |
| M+H | 863.2877 | 9.136 | 0213,0214,0215 |
| M+H | 863.2877 | 9.311 | 0213,0214,0215 |
| M+H | 863.2877 | 9.193 | 0213,0214,0215 |
| M+H | 863.3229 | 9.035 | 0216 |
| M+H | 864.2842 | 9.41 | 0217 |
| M+H | 864.3052 | 9.196 | 0218,0219 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 864.3052 | 9.215 | 0218,0219 |
| M+H | 864.3599 | 9.773 | 0220 |
| M+H | 865.2646 | 9.733 | 0221 |
| M+H | 865.3025 | 8.405 | 0222 |
| M+H | 866.3176 | 10.394 | 0223,0224 |
| M-H | 864.3017 | 10.441 | 0223,0224 |
| M-H | 864.3235 | 10.035 | 0225 |
| M+H | 867.2758 | 9.41 | 0226 |
| M+H | 867.3138 | 9.801 | 0227,0228,0229 |
| M+H | 867.3156 | 10.623 | 0227,0228,0229,0230 |
| M+H | 867.3138 | 9.738 | 0227,0228,0229 |
| M+H | 867.3195 | 8.718 | 0227,0228,0229,0230 |
| M+H | 868.3056 | 8.701 | 0231 |
| M+H | 868.3151 | 9.038 | 0232 |
| M+H | 868.3344 | 10.206 | 0233,0234 |
| M+H | 868.3343 | 10.274 | 0233,0234 |
| M+H | 869.3094 | 8.248 | 0235 |
| M+H | 869.3285 | 9.51 | 0236 |
| M+H | 870.2626 | 10.391 | 0237 |
| M+H | 870.2944 | 8.745 | 0238 |
| M+H | 870.3180 | 8.879 | 0239 |
| M+H | 870.3514 | 9.95 | 0240 |
| M+H | 871.2780 | 8.962 | 0241 |
| M+H | 871.3445 | 8.738 | 0242 |
| M+H | 872.2779 | 9.122 | 0243 |
| M+H | 872.3075 | 9.691 | 0244 |
| M+H | 872.3402 | 10.136 | 0245 |
| M+H | 872.3713 | 1.476 | 0246 |
| M+H | 872.4146 | 9.366 | 0247 |
| M+H | 873.3348 | 9.592 | 0248 |
| M+H | 873.3464 | 9.481 | 0249 |
| M+H | 874.2712 | 8.788 | 0250,0251 |
| M+H | 874.2681 | 8.541 | 0250,0251 |
| M+H | 874.2901 | 10.366 | 0252 |
| M+H | 874.3306 | 10.339 | 0253 |
| M-H | 872.3338 | 9.162 | 0254,0255 |
| M+H | 874.3498 | 8.542 | 0254,0255 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 875.2546 | 8.99 | 0256 |
| M+H | 875.2985 | 9.141 | 0257 |
| M+H | 875.3060 | 9.486 | 0258 |
| M+H | 876.2834 | 8.527 | 0259 |
| M+H | 876.3077 | 10.994 | 0260,0261 |
| M+H | 876.3076 | 9.688 | 0260,0261 |
| M+H | 876.3479 | 9.097 | 0262 |
| M+H | 876.3904 | 9.191 | 0263 |
| M+H | 877.3037 | 9.599 | 0264,0265,0266 |
| M+H | 877.3034 | 9.5 | 0264,0265,0266 |
| M+H | 877.3071 | 9.466 | 0264,0265,0266 |
| M+H | 877.3129 | 9.591 | 0267 |
| M+H | 878.2960 | 9.797 | 0268 |
| M+H | 878.3253 | 8.991 | 0269,0270,0271 |
| M-H | 876.3122 | 8.339 | 0269,0270,0271 |
| M-H | 876.3122 | 8.311 | 0269,0270,0271 |
| M+H | 879.2298 | 8.811 | 0272 |
| M+H | 879.2990 | 9.948 | 0273 |
| M+H | 879.3196 | 8.752 | 0274 |
| M+H | 880.2592 | 9.612 | 0275 |
| M+H | 880.2828 | 9.504 | 0276,0277 |
| M+H | 880.2825 | 10.716 | 0276,0277 |
| M-H | 878.3221 | 9.308 | 0278 |
| M+H | 881.2787 | 9.289 | 0279,0280,0281 |
| M+H | 881.2785 | 9.42 | 0279,0280,0281 |
| M+H | 881.2786 | 9.248 | 0279,0280,0281 |
| M+H | 881.3145 | 10.431 | 0282,0283 |
| M+H | 881.3147 | 10.271 | 0282,0283 |
| M+H | 881.3346 | 8.02 | 0284 |
| M+H | 882.2942 | 8.883 | 0285 |
| M+H | 882.3091 | 10.601 | 0288,0289,0290 |
| M+H | 882.3132 | 10.035 | 0288,0289,0290 |
| M+H | 882.3095 | 11.567 | 0288,0289,0290 |
| M+H | 882.3293 | 9.34 | 0291 |
| M+H | 883.2936 | 8.554 | 0292 |
| M+H | 883.3284 | 9.448 | 0293 |
| M+H | 884.2776 | 9.767 | 0294 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 884.3094 | 8.841 | 0295 |
| M+H | 884.3349 | 9.172 | 0296 |
| M+H | 884.3646 | 10.401 | 0297 |
| M+H | 884.4161 | 9.384 | 0298 |
| M+H | 885.2391 | 9.043 | 0299 |
| M+H | 885.2955 | 8.365 | 0300 |
| M+H | 885.3113 | 15.861 | 0301 |
| M+H | 886.2970 | 9.148 | 0302 |
| M+H | 886.3030 | 9.136 | 0303 |
| M+H | 886.3200 | 9.287 | 0304 |
| M+H | 886.3391 | 9.883 | 0305 |
| M+H | 887.2539 | 9.086 | 0306 |
| M+H | 887.2928 | 8.804 | 0307 |
| M+H | 887.2989 | 8.365 | 0308 |
| M+H | 887.3352 | 10.167 | 0309 |
| M+H | 888.2548 | 9.45 | 0310 |
| M+H | 888.2861 | 9.064 | 0311 |
| M+H | 888.3092 | 8.993 | 0312,0313,0314 |
| M+H | 888.3088 | 9.795 | 0312,0313,0314 |
| M+H | 888.3068 | 11.104 | 0312,0313,0314 |
| M+H | 888.3304 | 9.559 | 0315 |
| M+H | 888.3343 | 9.766 | 0315,0316 |
| M+H | 889.2699 | 7.933 | 0317 |
| M+H | 889.3037 | 9.669 | 0318,0319,0320,0321 |
| M+H | 889.3069 | 8.976 | 0318,0319,0320,0321 |
| M+H | 889.3038 | 9.713 | 0318,0319,0320,0321 |
| M+H | 889.3038 | 9.577 | 0318,0319,0320,0321 |
| M+H | 889.3382 | 8.634 | 0322 |
| M+H | 890.3225 | 10.304 | 0323 |
| M+H | 891.2796 | 9.995 | 0324 |
| M+H | 891.2864 | 8.804 | 0324,0325 |
| M+H | 892.2612 | 8.89 | 0326 |
| M+H | 892.3336 | 10.727 | 0327,0328 |
| M+H | 892.3337 | 10.614 | 0327,0328 |
| M+H | 893.2913 | 9.808 | 0329 |
| M+H | 893.3292 | 10.656 | 0330,0331,0332 |
| M+H | 893.3294 | 10.143 | 0330,0331,0332 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 893.3287 | 10.937 | 0330,0331,0332 |
| M+H | 893.3319 | 11.682 | 0330,0331,0332,0333 |
| M+H | 894.2827 | 9.353 | 0334 |
| M+H | 894.3289 | 7.583 | 0335 |
| M+H | 894.3551 | 9.592 | 0336 |
| M+H | 895.2603 | 9.222 | 0337,0338 |
| M+H | 895.2596 | 8.564 | 0337,0338 |
| M+H | 895.3271 | 8.636 | 0339 |
| M+H | 896.2592 | 9.519 | 0340,0341 |
| M+H | 896.2593 | 10.725 | 0340,0341 |
| M+H | 896.3093 | 8.644 | 0342 |
| M+H | 896.3348 | 9.394 | 0343 |
| M+H | 897.3658 | 10.317 | 0344 |
| M+H | 898.3079 | 10.145 | 0345 |
| M+H | 898.3246 | 9.134 | 0346 |
| M+H | 898.3303 | 9.42 | 0346,0347 |
| M+H | 898.3550 | 10.531 | 0348 |
| M+H | 898.4307 | 9.66 | 0349 |
| M+H | 899.3505 | 9.888 | 0350 |
| M+H | 899.3605 | 10.459 | 0351 |
| M+H | 900.2842 | 8.45 | 0352,0353 |
| M+H | 900.2922 | 9.924 | 0353 |
| M+H | 900.3129 | 10.671 | 0354 |
| M+H | 900.3286 | 8.776 | 0355 |
| M+H | 900.3600 | 9.99 | 0356 |
| M+H | 901.2158 | 9.374 | 0357 |
| M+H | 901.2683 | 9.361 | 0358 |
| M+H | 902.2289 | 9.076 | 0359 |
| M+H | 902.2996 | 8.943 | 0360 |
| M-H | 900.2905 | 9.266 | 0361 |
| M+H | 902.3236 | 10.068 | 0362,0363 |
| M+H | 902.3236 | 10.136 | 0362,0363 |
| M+H | 902.4050 | 9.466 | 0364 |
| M+H | 903.3190 | 10.004 | 0365,0366,0367 |
| M+H | 903.3191 | 9.844 | 0365,0366,0367 |
| M+H | 903.3224 | 9.413 | 0365,0366,0367 |
| M+H | 904.2923 | 9.242 | 0368 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 904.3117 | 10.144 | 0369 |
| M+H | 905.2459 | 9.188 | 0370 |
| M+H | 905.3167 | 10.401 | 0371 |
| M+H | 906.2801 | 9.665 | 0372,0373 |
| M+H | 906.2802 | 9.588 | 0372,0373 |
| M+H | 906.2985 | 9.878 | 0374,0375 |
| M+H | 906.2986 | 9.927 | 0374,0375 |
| M+H | 906.3555 | 9.747 | 0376 |
| M+H | 907.2755 | 9.104 | 0377 |
| M+H | 907.2945 | 9.774 | 0378,0379,0380 |
| M+H | 907.2947 | 9.807 | 0378,0379,0380 |
| M+H | 907.2946 | 9.741 | 0378,0379,0380 |
| M+H | 907.3502 | 8.51 | 0381 |
| M+H | 908.2968 | 10.708 | 0382 |
| M+H | 908.3489 | 6.562 | 0383 |
| M+H | 909.3453 | 10.418 | 0384 |
| M+H | 910.3502 | 9.219 | 0385 |
| M+H | 910.3801 | 10.771 | 0386 |
| M+H | 911.2544 | 9.468 | 0387 |
| M+H | 911.3276 | 16.941 | 0388 |
| M+H | 912.2364 | 9.858 | 0389 |
| M+H | 912.2706 | 9.476 | 0390 |
| M+H | 912.3100 | 9.645 | 0391 |
| M+H | 912.3204 | 7.709 | 0392 |
| M+H | 913.2454 | 8.907 | 0393 |
| M+H | 913.2489 | 9.544 | 0393,0394 |
| M+H | 913.3038 | 10.164 | 0395 |
| M+H | 913.3156 | 8.738 | 0396 |
| M-H | 912.2908 | 9.329 | 0397 |
| M+H | 914.3239 | 9.453 | 0398 |
| M+H | 915.2599 | 8.894 | 0399 |
| M+H | 915.3227 | 8.927 | 0400 |
| M+H | 915.3669 | 10.966 | 0401 |
| M+H | 916.3365 | 10.693 | 0402 |
| M+H | 917.3028 | 9.314 | 0403 |
| M+H | 918.2063 | 9.141 | 0404 |
| M+H | 918.2771 | 9.275 | 0405 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 919.2613 | 9.251 | 0406,0407 |
| M+H | 919.2605 | 9.741 | 0406,0407 |
| M+H | 920.2555 | 9.732 | 0408,0409,0410 |
| M+H | 920.2555 | 9.688 | 0408,0409,0410 |
| M+H | 920.2606 | 11.133 | 0408,0409,0410 |
| M+H | 920.2991 | 9.741 | 0411 |
| M-H | 918.3521 | 10.119 | 0412 |
| M+H | 921.2731 | 9.633 | 0413,0414 |
| M+H | 921.2731 | 9.67 | 0413,0414 |
| M+H | 922.2711 | 8.966 | 0415 |
| M+H | 922.2741 | 9.924 | 0415,0416 |
| M+H | 922.3620 | 8.811 | 0417 |
| M+H | 924.2651 | 9.424 | 0418 |
| M+H | 924.2876 | 9.192 | 0419 |
| M+H | 924.3236 | 10.433 | 0420 |
| M-H | 922.3288 | 9.837 | 0421 |
| M+H | 925.2815 | 9.51 | 0422 |
| M+H | 926.2774 | 8.749 | 0423,0424 |
| M+H | 926.2805 | 9.34 | 0423,0424 |
| M-H | 924.3110 | 8.911 | 0425 |
| M-H | 924.3273 | 9.118 | 0426 |
| M+H | 926.3863 | 9.958 | 0427 |
| M+H | 927.2310 | 9.83 | 0428 |
| M+H | 927.2856 | 8.806 | 0429 |
| M+H | 928.2442 | 9.415 | 0430 |
| M+H | 928.3217 | 9.74 | 0431 |
| M-H | 927.2095 | 9.616 | 0432 |
| M+H | 930.2814 | 10.294 | 0433,0434 |
| M-H | 928.2633 | 10.258 | 0433,0434 |
| M+H | 930.3055 | 9.737 | 0435 |
| M+H | 931.2383 | 9.222 | 0436 |
| M+H | 931.2760 | 10.183 | 0437,0438,0439 |
| M+H | 931.2762 | 10.259 | 0437,0438,0439 |
| M+H | 931.2759 | 10.232 | 0437,0438,0439 |
| M+H | 932.2951 | 10.049 | 0440,0441 |
| M+H | 932.2952 | 10.097 | 0440,0441 |
| M+H | 933.2903 | 9.51 | 0442 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 934.3140 | 8.455 | 0443 |
| M+H | 935.3064 | 9.587 | 0444 |
| M+H | 936.3005 | 9.946 | 0445 |
| M+H | 936.3771 | 9.094 | 0446 |
| M+H | 937.2998 | 9.178 | 0447 |
| M+H | 937.3063 | 9.746 | 0448 |
| M+H | 938.2507 | 10.279 | 0449 |
| M+H | 938.2886 | 9.833 | 0450 |
| M+H | 938.3057 | 9.738 | 0451 |
| M+H | 939.2643 | 9.871 | 0452,0453 |
| M+H | 939.2623 | 9.366 | 0452,0453 |
| M+H | 940.3518 | 8.923 | 0454 |
| M+H | 941.2762 | 9.309 | 0455 |
| M+H | 941.3817 | 9.936 | 0456 |
| M+H | 942.2574 | 9.662 | 0457 |
| M+H | 943.2635 | 9.005 | 0458 |
| M+H | 944.2217 | 9.497 | 0459 |
| M+H | 945.2751 | 10.213 | 0460,0461 |
| M+H | 945.2751 | 10.292 | 0460,0461 |
| M+H | 946.2728 | 10.138 | 0462,0463,0464 |
| M+H | 946.2728 | 10.023 | 0462,0463,0464 |
| M+H | 946.2728 | 10.077 | 0462,0463,0464 |
| M+H | 948.3270 | 10.15 | 0465 |
| M+H | 948.3767 | 9.159 | 0466 |
| M+H | 950.2828 | 6.867 | 0467 |
| M+H | 950.3018 | 9.973 | 0468 |
| M+H | 951.2494 | 9.86 | 0469 |
| M+H | 951.2963 | 8.075 | 0470 |
| M+H | 952.2965 | 9.716 | 0471,0472 |
| M+H | 952.3020 | 9.727 | 0472 |
| M+H | 952.4020 | 10.253 | 0473 |
| M+H | 953.3011 | 9.826 | 0474 |
| M+H | 954.2826 | 10.009 | 0475 |
| M+H | 955.2411 | 9.938 | 0476 |
| M+H | 956.2952 | 10.644 | 0477,0478 |
| M+H | 956.2952 | 10.725 | 0477,0478 |
| M+H | 957.2532 | 9.645 | 0479 |

(continued)

| [Table 9-8-8] Compound that may be contained in mixture 2-2-d1E04-2 | | | |
|---|---|---|---|
| Ion species | m/z [M+H]+ or [M-H]- | Retention time of MS peak (min) | No. of assigned compound (described with 2-2-d1E04-2- omitted) |
| M+H | 957.2905 | 10.504 | 0480,0481,0482 |
| M+H | 957.2913 | 10.744 | 0480,0481,0482 |
| M+H | 957.2928 | 7.774 | 0480,0481,0482 |
| M+H | 961.3208 | 9.954 | 0483 |
| M+H | 962.2700 | 10.338 | 0484 |
| M+H | 962.3166 | 10.28 | 0485 |
| M+H | 962.3918 | 9.456 | 0486 |
| M+H | 963.3133 | 9.517 | 0487 |
| M+H | 963.3221 | 10.202 | 0488 |
| M+H | 964.3225 | 10.208 | 0489 |
| M+H | 966.3658 | 9.253 | 0490 |
| M+H | 968.2733 | 10.042 | 0491 |
| M+H | 969.2784 | 10.112 | 0492 |
| M+H | 974.3422 | 10.527 | 0493 |
| M+H | 977.2654 | 10.262 | 0494 |
| M+H | 979.3286 | 9.331 | 0495 |
| M+H | 980.2981 | 10.493 | 0496 |
| M+H | 988.2844 | 10.65 | 0497 |
| M+H | 990.3476 | 9.707 | 0498 |
| M+H | 1005.3438 | 9.711 | 0499 |
| M+H | 1016.3642 | 10.044 | 0500 |

The ratio of observed m/z $(M+H)^+$ or $(M-H)^-$ derived from the compounds designed as library components in the analysis of each mixture is shown in Table 9-9.

[Table 512]

| [Table 9-9] | | | | | | | | |
|---|---|---|---|---|---|---|---|---|
| Mixture No. (2-2-) | d1E01-1 | d1E02-1 | d1E03-1 | d1E04-1 | d1E01-2 | d1E02-2 | d1E03-2 | d1E04-2 |
| The number of corresponding m/z observed | 499 | 500 | 500 | 498 | 499 | 499 | 497 | 495 |
| Ratio of corresponding m/z observed to whole library components (%) | 99.8 | 100.0 | 100.0 | 99.6 | 99.8 | 99.8 | 99.4 | 99.0 |
| The number of $M+H^+$ observed | 440 | 444 | 438 | 472 | 463 | 457 | 439 | 467 |
| Ratio of $M+H^+$ observed (%) | 88.0 | 88.8 | 87.6 | 94.4 | 92.6 | 91.4 | 87.8 | 93.4 |

(continued)

[Table 9-9]

| Mixture No. (2-2-) | d1E01-1 | d1E02-1 | d1E03-1 | d1E04-1 | d1E01-2 | d1E02-2 | d1E03-2 | d1E04-2 |
|---|---|---|---|---|---|---|---|---|
| The number of M- $H^-$ observed | 409 | 404 | 379 | 377 | 308 | 411 | 304 | 375 |
| Ratio of M-$H^-$ observed (%) | 81.8 | 80.8 | 75.8 | 75.4 | 61.6 | 82.2 | 60.8 | 75.0 |
| The number of M+$H^+$ and M-$H^-$ observed together | 350 | 348 | 316 | 351 | 272 | 369 | 246 | 347 |
| The number of M+$H^+$ observed alone | 90 | 96 | 122 | 121 | 191 | 88 | 193 | 120 |
| The number of M- $H^-$ observed alone | 59 | 56 | 63 | 26 | 36 | 42 | 58 | 28 |

Thus, Example 9 illustrates an actual example in which a plurality of mixture libraries of 500 compounds having diversity imparted not only to core blocks but to linkers that linked them were synthesized by appropriately selecting and applying highly robust methods as to reaction conditions for use in diverse bond formations and washing methods. These libraries were synthesized with a very high success rate as described above. This Example is a mere illustration of library synthesis. In the present invention, the building blocks or the linkers that link them are not limited to those shown as an actual example, and library synthesis can be carried out by using arbitrary building blocks and arbitrary linkers.

Example 10: Practice example of preparing mixture library having bond diversity by each independently synthesizing mixtures having bonds between core blocks, all of which are constituted by different types of bonds, followed by mixing of synthesized mixtures: practice example of synthesis of plurality of libraries having such different types of bonds

The libraries synthesized in Examples 4, 8, and 9 showed an example of synthesizing a library also having diversity in linking moieties (linkers) that linked building blocks, wherein the "diversity of core blocks" that formed pharmacophores and the "diversity of linkers" were multiplied.

Example 10 showed a synthesis example of a mixture library having bond diversity by each independently synthesizing mixtures still having the diversity of core blocks and also having linking moieties (linkers) that link building blocks, all of which are constituted by different types of bonds, followed by the mixing of the synthesized mixtures. This library can be divided to a library in which the first linking moiety (linker) is constituted by an amide bond, the second linker is constituted by an ether bond, and the third linker is constituted by a C-N bond through sulfonamide coupling (referred to as case 1); and a library in which the first linking moiety (linker) is constituted by an amide bond, the second linker is constituted by a C-N bond through amide coupling, and the third linker is constituted by an ester bond (referred to as case 2). These libraries are finally mixed to prepare one library. The bonds used in library synthesis are not limited to the bonds given here, and a library can be synthesized by selecting arbitrary bonds.

Figures 8 and 9 show the "diversity of core blocks" and the "diversity of linkers" contained in the library.

Figures 10 and 11 show a library synthesis route by split & mix.

The building blocks used in Example 10 are shown in Table BB4-1 and Table BB4- 2.7

[Table 513]

[Table BB4-1] Building block used in Case 1

| Table [BB4-1] | | |
|---|---|---|
| Compound No. | | Compound name |
| BB4-1-01 | 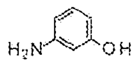 | 3-Aminophenol |
| BB4-1-02 | 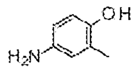 | 4-Amino-2-methylphenol |
| BB4-1-03 | 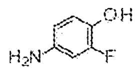 | 4-Amino-2-fluorophenol |
| BB4-1-04 | 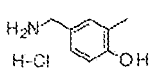 | 4-(Aminomethyl)-2-methylphenol hydrochloride |
| BB4-1-05 | 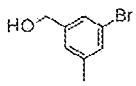 | (3-Bromo-5-methylphenyl)methanol |
| BB4-1-06 | 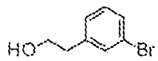 | 2-(3-Bromophenyl)ethanol |
| BB4-1-07 | 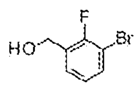 | (3-Bromo-2-fluorophenyl)methanol |
| BB4-1-08 | 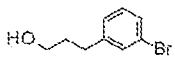 | 3-(3-Bromophenyl)propan-1-ol |
| BB4-1-09 | 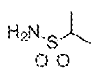 | Propane-2-sulfonamide |
| BB4-1-10 | 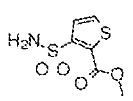 | Methyl 3-sulfamoylthiophene-2-carboxylate |
| BB4-1-11 | 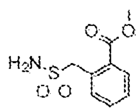 | Methyl 2-(sulfamoylmethyl)benzoate |
| BB4-1-12 | 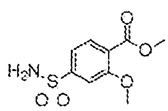 | Methyl 2-methoxy-4-sulfamoylbenzoate |

[Table 514]

[Table BB4-2] Building block used in Case 2

| Table [BB4-2] | | |
|---|---|---|
| Compound No. | | Compound name |
| BB4-2-01 | 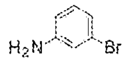 | 3-Bromoaniline |
| BB4-2-02 | 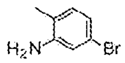 | 5-Bromo-2-methylaniline |
| BB4-2-03 | 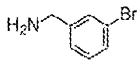 | (3-Bromophenyl)methanamine |
| BB4-2-04 | 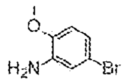 | 5-Bromo-2-methoxyaniline |
| BB4-2-05 | 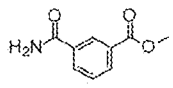 | Methyl 3-carbamoylbenzoate |
| BB4-2-06 | 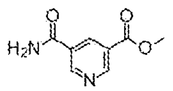 | Methyl 5-carbamoylpyridine-3-carboxylate |
| BB4-2-07 | 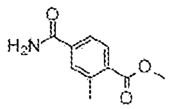 | Methyl 4-carbamoyl-2-methylbenzoate |
| BB4-2-08 | 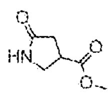 | Methyl 5-oxopyrrolidine-3-carboxylate |
| BB4-2-09 | 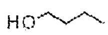 | Butan-1-ol |
| BB4-2-10 | 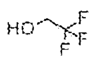 | 2,2,2-Trifluoroethanol |
| BB4-2-11 | 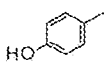 | 4-Methylphenol |
| BB4-2-12 | 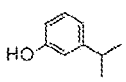 | 3-Propan-2-ylphenol |

Library case 1 shows a synthesis example of a library in which the first linking moiety (linker) (a1) is constituted by an amide bond, the second linker (b1) is constituted by an ether bond, and the third linker (c1) is constituted by a C-N bond through sulfonamide coupling.

In Example 10, mixture numbers may be used to indicate synthesized mixtures. For example, "4-1" in "4-1-a1B01-0" represents the type of a library, and "a1B01" represents the type of a mixture. "-0" represents a state supported on the solid phase, and the term "-1" represents a state cleaved from the solid phase. Also, "-2" represents a state where functional group conversion was performed after cleavage from the solid phase. ID may be used to indicate each compound contained in a mixture. For example, "0001" in "4-1-a1B01-0- 0001" represents an arbitrary compound among compounds contained in the mixture "4-1- a1B01-0".

Example 10-1: Formation of linker a1 by step a1 in case 1

A reaction formula in step a1B01 performed using BB4-1-01 will be illustrated below.

[Formula 693]

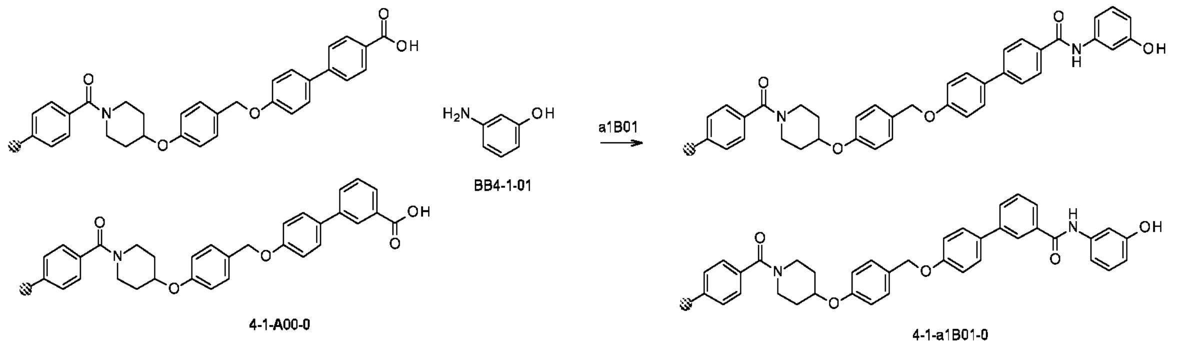


BB4-1-01

4-1-A00-0

4-1-a1B01-0

Mixture 4-1-a1B01-0 was synthesized as follows.

Under a nitrogen atmosphere, compound C005-03R (160 mg, 0.201 mmol/g), compound C003-03R (160 mg, 0.201 mmol/g), and NMP (5.0 mL) were added to an 8 mL glass vial and shaken at room temperature for 1 hour to prepare mixture 4-1-A00-0. 3- Aminophenol (BB-4-1-01) (20 mg, 0.187 mmol), HOAt (25 mg, 0.187 mmol), and DIC (28 μL, 0.183 mmol) were added thereto, and the mixture was shaken at 60°C for 6 hours.

Solid-phase purification

The suspension of the reaction solution and the solid phase was wholly transferred onto the filter of a column and washed three times with NMP (6 mL), three times with NMP- water (1:1, 6 mL), three times with NMP (6 mL), three times with methanol (6 mL), three times with DCM (6 mL), and three times with heptane (6 mL), and the obtained solid phase was dried overnight under reduced pressure to obtain mixture 4-1-a1B01-0 (amount supported: 0.197 mmol/g, 0.322 g).

Mixture 4-1-a1B01-0 can be treated by cleavage in the same manner as in Example 8-1-1 to obtain mixture 4-1-a1B01-1. The details of the mixture are shown in Table 10-1-3.

Steps a1B02 and a1B03 were performed in the same manner as in the synthesis of mixture 4-1-a1B01-0 using BB4-1-02 and -03 instead of BB4-1-01 to obtain mixture 4-1- a1B02-0 (amount supported: 0.197 mmol/g, 0.286 g) and mixture 4-1-a1B03-0 (amount supported: 0.197 mmol/g, 0.351 g).

Next, a reaction formula in step a1B04 performed using BB4-1-04 will be illustrated below.

[Formula 694]

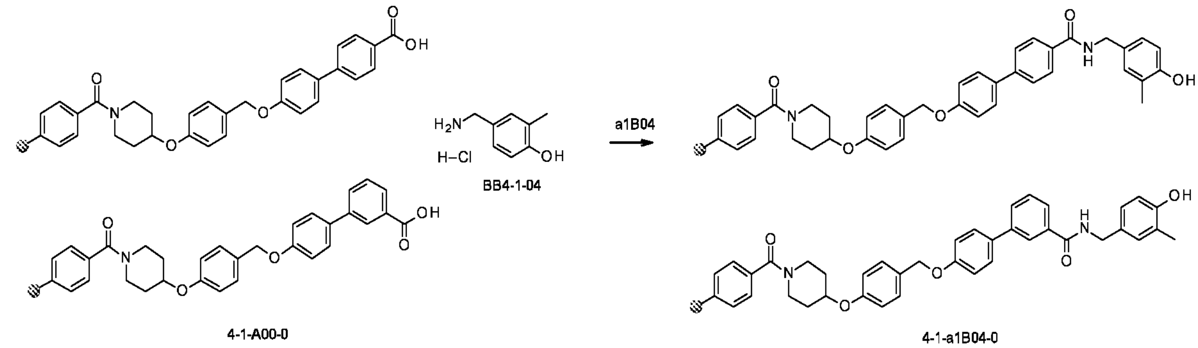


BB4-1-04

4-1-A00-0

4-1-a1B04-0

Mixture 4-1-a1B04-0 was synthesized as follows.

Under a nitrogen atmosphere, compound C005-03R (160 mg, 0.201 mmol/g), compound C003-03R (160 mg, 0.201 mmol/g), and NMP (4.5 mL) were added to an 8 mL glass vial and shaken at room temperature for 1 hour to prepare mixture 4-1-A00-0. 4- (Aminomethyl)-2-methylphenol hydrochloride (BB-4-1-04) (52 mg, 0.299 mmol), DIPEA (53 μL, 0.302 mmol), HOAt (41 mg, 0.302 mmol), and DIC (47 μL, 0.302 mmol) were added thereto, and the mixture was shaken at room temperature for 14 hours.

Solid-phase purification

The suspension of the reaction solution and the solid phase was wholly transferred onto the filter of a column and washed three times with NMP (6 mL), three times with NMP- water (1:1, 6 mL), three times with NMP (6 mL), three times with methanol (6 mL), three times with DCM (6 mL), and three times with heptane (6 mL), and the obtained solid phase was dried overnight under reduced pressure to obtain mixture 4-1-a1B04-0 (amount supported: 0.196 mmol/g, 0.331 g).

The reagents used in step a1 and the obtained mixtures are shown in Table 10-1-1.

[Table 515]

| [Table 10-1-1] Reagent used and obtained compound in step a1 | | | | | | | |
|---|---|---|---|---|---|---|---|
| Starting material used | Amount used | Amine reagent used | Amount used | Molar number | Obtained mixture | Yield | Amount supported |
| C005-03R /C003-03R | 160 mg /160 mg | BB4-1-01 | 20 mg | 0.187 mmol | 4-1-a1B01-0 | 321.8 mg | 0.197 mmol/g |
| C005-03R /C003-03R | 150 mg /150 mg | BB4-1-02 | 22 mg | 0.181 mmol | 4-1-a1B02-0 | 285.7 mg | 0.197 mmol/g |
| C005-03R /C003-03R | 165 mg /165 mg | BB4-1-03 | 23 mg | 0.182 mmol | 4-1-a1B03-0 | 351.0 mg | 0.197 mmol/g |
| C005-03R /C003-03R | 160 mg /160 mg | BB4-1-04 | 52 mg | 0.299 mmol | 4-1-a1B04-0 | 331.4 mg | 0.196 mmol/g |

Mixtures 4-1-a1B01-0 to 4-1-a1B04-0 can be treated by cleavage in the same manner as in Example 8-1-1 to obtain mixtures 4-1-a1B01-1 to 4-1-a1B04-0. The details of the mixtures are shown in Table 10-1-3 to Table 10-1-6.

A notation method in each table will be described. For example, in Table 10-1-23, each compound that may be contained in 4-1-a1B01-1 is indicated by ID, and core blocks and linkers in combination in the compound structure of mixture 4-1-a1B01-1 are indicated by symbols or chemical formulas. The correspondence between the symbols and specific structures are shown in Figure 8. In Table 10-1-2 to Table 10-1-6, "×" and "b" are represented by chemical formulas.

[Formula 695]

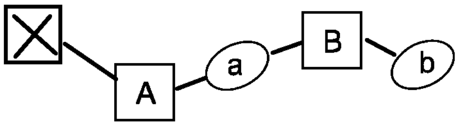


For example, when ID is 4-1-a1B01-1-0001, the compound is represented by the following structural formula.

[Formula 696]

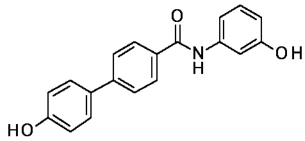

4-1-a1B01-1-0001

[Table 516]

| [Table 10-1-2] Structural notation in ID (4-1-a1B01-1-0001) | | | | | |
|---|---|---|---|---|---|
| ID | | A | a | B | b |
| 4-1-a1B01-1-0001 | OH | A01 | a1 | B01 | OH |

The mixture synthesized in step a1B01 using BB4-1-01 is shown in Table 10-1-3.

[Table 517]

| [Table 10-1-3] Compound contained in mixture (4-1-a1B01-0) synthesized in step a1B01 using BB4-1-01 | | | | | | |
|---|---|---|---|---|---|---|
| ID | Exact Mass | | A | a | B | b |
| 4-1-a1B01-1-0001 | 305.11 | OH | A01 | a1 | B01 | OH |
| 4-1-a1B01-1-0002 | 305.11 | OH | A02 | a1 | B01 | OH |

The mixture synthesized in step a1B02 using BB4-1-02 is shown in Table 10-1-4.

[Table 518]

| [Table 10-1-4] Compound contained in mixture (4-1-a1B02-0) synthesized in step a1B02 using BB4-1-02 | | | | | | |
|---|---|---|---|---|---|---|
| ID | Exact Mass | | A | a | B | b |
| 4-1-a1B02-1-0001 | 319.12 | OH | A01 | a1 | B02 | OH |
| 4-1-a1B02-1-0002 | 319.12 | OH | A02 | a1 | B02 | OH |

The mixture synthesized in step a1B03 using BB4-1-03 is shown in Table 10-1-5.

[Table 519]

| [Table 10-1-5] Compound contained in mixture (4-1-a1B03-0) synthesized in step a1B03 using BB4-1-03 | | | | | | |
|---|---|---|---|---|---|---|
| ID | Exact Mass | | A | a | B | b |
| 4-1-a1B03-1-0001 | 323.1 | OH | A01 | a1 | B03 | OH |
| 4-1-a1B03-1-0002 | 323.1 | OH | A02 | a1 | B03 | OH |

The mixture synthesized in step a1B04 using BB4-1-04 is shown in Table 10-1-6.
[Table 520]

| [Table 10-1-6] Compound contained in mixture (4-1-a1B04-0) synthesized in step a1B04 using BB4-1-04 | | | | | | |
|---|---|---|---|---|---|---|
| ID | Exact Mass | | A | a | B | b |
| 4-1-a1B04-1-0001 | 333.14 | OH | A01 | a1 | B04 | OH |
| 4-1-a1B04-1-0002 | 333.14 | OH | A02 | a1 | B04 | OH |

Example 10-2: Solid-phase mixing of compounds synthesized in step a1 in case 1

[Formula 697]

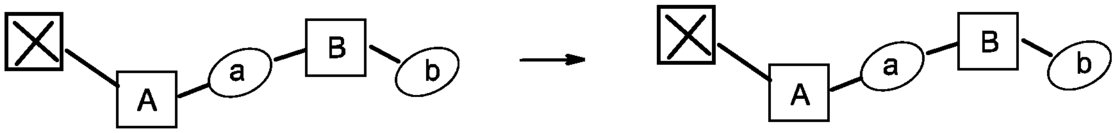


4-1-a1B01-0~4-1-a1B04-0

4-1-C00-0

4 types of mixtures described in Table 10-2-1 were added to a 10 mL column with a filter and washed twice with DMF (8 mL), three times with DCM (8 mL), and three times with heptane (8 mL), and the obtained solid phase was dried under reduced pressure to obtain mixture 4-1-C00-0 (838 mg, 0.197 mmol/g).
The mixtures synthesized in step a1 and the amounts of the mixtures used are shown in Table 10-2-1.
[Table 521]

| [Table 10-2-1] Synthesized mixture and amount used in step a1 | |
|---|---|
| Mixture | Amount used |
| 4-1-a1B01-0 | 210 mg |
| 4-1-a1B02-0 | 203 mg |
| 4-1-a1B03-0 | 210 mg |
| 4-1-a1B04-0 | 220 mg |

Mixture 4-1-C00-0 can be treated by cleavage in the same manner as in Example 10- 1 to obtain mixture 4-1-C00-1. The details of the mixture are shown in Table 10-2-3.
A notation method in each table will be described. For example, in Table 10-2-3, each compound that may be contained in 4-1-C00-1 is indicated by ID, and core blocks and linkers in combination in the structure of mixture 4-1-C00-1 are indicated by symbols or chemical formulas. The correspondence between the symbols and specific structures are shown in Figure 8.
In Table 10-2-2 and Table 10-2-3, "×" and "b" are represented by chemical formulas.

[Formula 698]

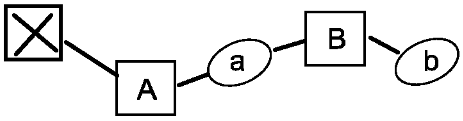

4-1-C00-0

For example, when ID is 4-1-C00-1-0001, the compound is represented by the following structural formula.

[Formula 699]

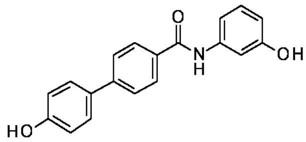

4-1-C00-1-0001

[Table 522]

| [Table 10-2-2] Structural notation in ID (4-1-C00-1-0001) | | | | | |
|---|---|---|---|---|---|
| ID | | A | a | B | b |
| 4-1-C00-1-0001 | OH | A01 | a1 | B01 | OH |

The mixture obtained by the solid-phase mixing of the mixtures synthesized in step a1 is shown in Table 10-2-3.

[Table 523]

| [Table 10-2-3] Compound contained in mixture 4-1-C00-0 | | | | | |
|---|---|---|---|---|---|
| ID | | A | a | B | b |
| 4-1-C00-1-0001 | OH | A01 | a1 | B01 | OH |
| 4-1-C00-1-0002 | OH | A02 | a1 | B01 | OH |
| 4-1-C00-1-0003 | OH | A01 | a1 | B02 | OH |
| 4-1-C00-1-0004 | OH | A02 | a1 | B02 | OH |
| 4-1-C00-1-0005 | OH | A01 | a1 | B03 | OH |
| 4-1-C00-1-0006 | OH | A02 | a1 | B03 | OH |
| 4-1-C00-1-0007 | OH | A01 | a2 | B04 | OH |
| 4-1-C00-1-0008 | OH | A02 | a2 | B04 | OH |

Example 10-3: Formation of linker b1 by step b1 in case 1

A reaction formula in step b1 performed using BB4-1-05 to -08 will be illustrated below.

[Formula 700]

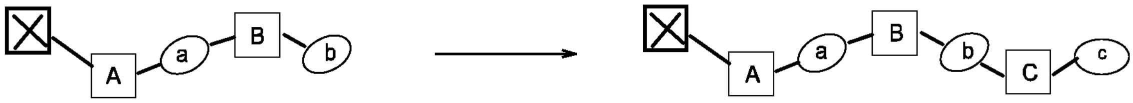

4-1-C00-0

4-1-b1C01-0 ~ 4-1-b1C04-0

Mixtures 4-1-b1C01-0 to 4-1-b1C04-0 were synthesized as follows. The following example shows step b1C01.

Under a nitrogen atmosphere, mixture 4-1-C00-0 (202 mg, 0.197 mmol/g) and THF (4.0 mL) were added to an 8 mL glass vial and shaken at room temperature for 1 hour. An alcohol reagent (BB4-1-05) (160 mg, 0.795 mmol) was added to the glass vial. A 0.5 M solution of CMMP (cyanomethylenetrimethylphosphorane) in THF (795 μL, 0.398 mmol) was added thereto, and the mixture was shaken at room temperature for 21 hours.

Solid-phase purification

The suspension of the reaction solution and the solid phase was transferred to a 6 mL column with a filter, filtered, and washed three times with NMP (4 mL), three times with NMP/water = 1/1 (4 mL), three times with NMP (4 mL), three times with MeOH (4 mL), three times with DCM (4 mL), and three times with heptane (4 mL), and the obtained solid phase was dried under reduced pressure to obtain mixture 4-1-b1C01-0.

Step b1 was performed in the same manner as in the synthesis of mixture 4-1- b1C01-0 using BB4-1-06 to -08 instead of BB4-1-05 to obtain mixture 4-1-b1C02-0 to mixture 4-1-b1 C04-0.

The alcohol reagents used in step b1 and the amounts of the reagents used are shown in Table 10-3-1. The details of the mixtures obtained in step b1 are shown in Table 10-3-3 to Table 10-3-6.

[Table 524]

[Table 10-3-1] Reagent used and obtained mixture in step b1

| Starting material used | Amount used | Alcohol reagent used | Amount used | Molar number | Obtained mixture | Yield | Amount supported |
|---|---|---|---|---|---|---|---|
| 4-1-C00-0 | 202 mg | BB4-1-05 | 160 mg | 0.795 mmol | 4-1-b1C01-0 | 227.5 mg | 0.190 mmol/g |
| 4-1-C00-0 | 200 mg | BB4-1-06 | 107 μL | 0.786 mmol | 4-1-b1C02-0 | 223.9 mg | 0.190 mmol/g |
| 4-1-C00-0 | 199 mg | BB4-1-07 | 161 mg | 0.784 mmol | 4-1-b1C03-0 | 221.5 mg | 0.190 mmol/g |
| 4-1-C00-0 | 195 mg | BB4-1-08 | 117 μL | 0.766 mmol | 4-1-b1C04-0 | 192.3 mg | 0.190 mmol/g |

Mixtures 4-1-b1C01-0 to 4-1-b1C04-0 can be treated by cleavage in the same manner as in Example 10-1 to obtain mixtures 4-1-b1C01-1 to 4-1-b1C04-1. The details of the mixtures are shown in Table 10-3-3 to Table 10-3-6.

A notation method in each table will be described. For example, in Table 10-3-3, each compound that may be contained in 4-1-b1C01-1 is indicated by ID, and core blocks and linkers in combination in the structure of mixture 4-1-b1C01-1 are indicated by symbols or chemical formulas. The correspondence between the symbols and specific structures are shown in Figure 8. In Table 10-3-2 to Table 10-3-6, "×" and "c" are represented by chemical formulas.

[Formula 701]

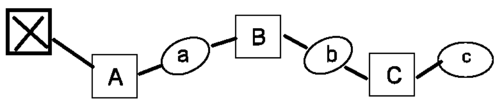

4-1-b1C01-0 ~ 4-1-b1C04-0

For example, when ID is 4-1-b1C01-1-0001, the compound is represented by the following structural formula.

[Formula 702]

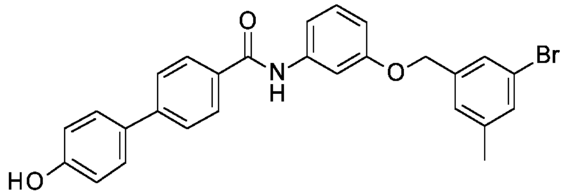

4-1-b1C01-1-0001

[Table 525]

| [Table 10-3-2] Structural notation in ID (4-1-b1C01-1-0001) | | | | | | | |
|---|---|---|---|---|---|---|---|
| ID | | A | a | B | b | C | c |
| 4-1-b1C01-1-0001 | OH | A01 | a1 | B01 | b1 | C01 | Br |

The mixture synthesized in step b1C01 using BB4-1-05 is shown in Table 10-3-3.

[Table 526]

| [Table 10-3-3] Compound contained in mixture (4-1-b1C01-1) synthesized in step b1C01 using BB4-1-05 | | | | | | | | |
|---|---|---|---|---|---|---|---|---|
| ID | Exact Mass | | A | a | B | b | C | c |
| 4-1-b1C01-1-0001 | 487.08 | OH | A01 | a1 | B01 | b1 | C01 | Br |
| 4-1-b1C01-1-0002 | 487.08 | OH | A02 | a1 | B01 | b1 | C01 | Br |
| 4-1-b1C01-1-0003 | 501.09 | OH | A01 | a1 | B02 | b1 | C01 | Br |
| 4-1-b1C01-1-0004 | 501.09 | OH | A02 | a1 | B02 | b1 | C01 | Br |
| 4-1-b1C01-1-0005 | 505.07 | OH | A01 | a1 | B03 | b1 | C01 | Br |
| 4-1-b1C01-1-0006 | 505.07 | OH | A02 | a1 | B03 | b1 | C01 | Br |
| 4-1-b1C01-1-0007 | 515.11 | OH | A01 | a1 | B04 | b1 | C01 | Br |
| 4-1-b1C01-1-0008 | 515.11 | OH | A02 | a1 | B04 | b1 | C01 | Br |

The mixture synthesized in step b1 using BB4-1-06 is shown in Table 10-3-4.

[Table 527]

| [Table 10-3-4] Compound contained in mixture (4-1-b1C02-1) synthesized in step b1C02 using BB4-1-06 | | | | | | | | |
|---|---|---|---|---|---|---|---|---|
| ID | Exact Mass | | A | a | B | b | C | c |
| 4-1-b1C02-1-0001 | 487.08 | OH | A01 | a1 | B01 | b1 | C02 | Br |
| 4-1-b1C02-1-0002 | 487.08 | OH | A02 | a1 | B01 | b1 | C02 | Br |
| 4-1-b1C02-1-0003 | 501.09 | OH | A01 | a1 | B02 | b1 | C02 | Br |
| 4-1-b1C02-1-0004 | 501.09 | OH | A02 | a1 | B02 | b1 | C02 | Br |
| 4-1-b1C02-1-0005 | 505.07 | OH | A01 | a1 | B03 | b1 | C02 | Br |
| 4-1-b1C02-1-0006 | 505.07 | OH | A02 | a1 | B03 | b1 | C02 | Br |
| 4-1-b1C02-1-0007 | 515.11 | OH | A01 | a1 | B04 | b1 | C02 | Br |

(continued)

| [Table 10-3-4] Compound contained in mixture (4-1-b1C02-1) synthesized in step b1C02 using BB4-1-06 | | | | | | | | |
|---|---|---|---|---|---|---|---|---|
| ID | Exact Mass | | A | a | B | b | C | c |
| 4-1-b1C02-1-0008 | 515.11 | OH | A02 | a1 | B04 | b1 | C02 | Br |

The mixture synthesized in step b1C03 using BB4-1-07 is shown in Table 10-3-5.

[Table 528]

| [Table 10-3-5] Compound contained in mixture (4-1-b1C03-1) synthesized in step b1C03 using BB4-1-07 | | | | | | | | |
|---|---|---|---|---|---|---|---|---|
| ID | Exact Mass | | A | a | B | b | C | c |
| 4-1-b1C03-1-0001 | 491.05 | OH | A01 | a1 | B01 | b1 | C03 | Br |
| 4-1-b1C03-1-0002 | 491.05 | OH | A02 | a1 | B01 | b1 | C03 | Br |
| 4-1-b1C03-1-0003 | 505.07 | OH | A01 | a1 | B02 | b1 | C03 | Br |
| 4-1-b1C03-1-0004 | 505.07 | OH | A02 | a1 | B02 | b1 | C03 | Br |
| 4-1-b1C03-1-0005 | 509.04 | OH | A01 | a1 | B03 | b1 | C03 | Br |
| 4-1-b1C03-1-0006 | 509.04 | OH | A02 | a1 | B03 | b1 | C03 | Br |
| 4-1-b1C03-1-0007 | 519.09 | OH | A01 | a1 | B04 | b1 | C03 | Br |
| 4-1-b1C03-1-0008 | 519.09 | OH | A02 | a1 | B04 | b1 | C03 | Br |

The mixture synthesized in step b1C04 using BB4-1-08 is shown in Table 10-3-6.

[Table 529]

| [Table 10-3-6] Compound contained in mixture (4-1-b1C04-1) synthesized in step b1C04 using BB4-1-08 | | | | | | | | |
|---|---|---|---|---|---|---|---|---|
| ID | Exact Mass | | A | a | B | b | C | c |
| 4-1-b1C04-1-0001 | 501.09 | OH | A01 | a1 | B01 | b1 | C04 | Br |
| 4-1-b1C04-1-0002 | 501.09 | OH | A02 | a1 | B01 | b1 | C04 | Br |
| 4-1-b1C04-1-0003 | 515.11 | OH | A01 | a1 | B02 | b1 | C04 | Br |
| 4-1-b1C04-1-0004 | 515.11 | OH | A02 | a1 | B02 | b1 | C04 | Br |
| 4-1-b1C04-1-0005 | 519.09 | OH | A01 | a1 | B03 | b1 | C04 | Br |
| 4-1-b1C04-1-0006 | 519.09 | OH | A02 | a1 | B03 | b1 | C04 | Br |
| 4-1-b1C04-1-0007 | 529.13 | OH | A01 | a1 | B04 | b1 | C04 | Br |
| 4-1-b1C04-1-0008 | 529.13 | OH | A02 | a1 | B04 | b1 | C04 | Br |

Example 10-4: Solid-phase mixing of mixtures synthesized in step b1 in case 1

[Formula 703]

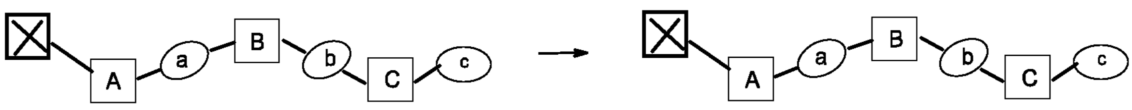


4-1-b1C01-0 ~ 4-1-b1C04-0

4-1-D00-0

4 types of mixtures described in Table 10-4-1 were added to a 6 mL column with a filter and washed twice with NMP (4 mL), three times with DCM (4 mL), and three times with heptane (4 mL), and the obtained solid phase was dried under reduced pressure to obtain mixture 4-1-D00-0 (498 mg, 0.190 mmol/g).

The mixtures synthesized in step b1 and the amounts of the mixtures used are shown in Table 10-4-1.

[Table 530]

| [Table 10-4-1] Synthesized mixture and amount used in step b1 | |
|---|---|
| Mixture | Amount used |
| 4-1-b1C01-0 | 118.0 mg |
| 4-1-b1C02-0 | 113.5 mg |
| 4-1-b1C03-0 | 119.0 mg |
| 4-1-b1C04-0 | 113.3 mg |

Mixture 4-1-D00-0 can be treated by cleavage in the same manner as in Example 10-1 to obtain mixture 4-1-D00-1. The details of the mixture are shown in Table 10-4-3.

A notation method in each table will be described. For example, in Table 10-4-3, each compound that may be contained in 4-1-D00-1 is indicated by ID, and core blocks and linkers in combination in the structure of mixture 4-1-D00-1 are indicated by symbols or chemical formulas. The correspondence between the symbols and specific structures are shown in Figure 8.

In Table 10-4-2 to Table 10-4-3, "×" and "c" are represented by chemical formulas.

[Formula 704]

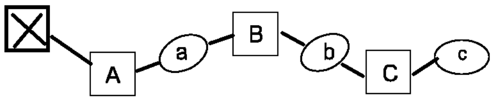


4-1-D00-0

For example, when ID is 4-1-D00-1-0001, the compound is represented by the following structural formula.

[Formula 705]

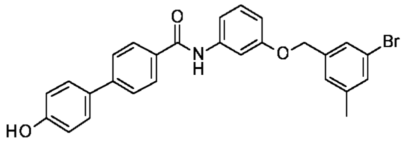

4-1-D00-1-0001

[Table 531]

| [Table 10-4-2] Structural notation in ID (4-1-D00-1-0001) | | | | | | | | |
|---|---|---|---|---|---|---|---|---|
| ID | | | A | a | B | b | C | c |
| 4-1-D00-1-0001 | | OH | A01 | a1 | B01 | b1 | C01 | Br |

The mixture obtained by the solid-phase mixing of the mixtures synthesized in step b1 is shown in Table 10-4-3.

[Table 532]

| [Table 10-4-3] Compound contained in mixture (4-1-D00-0) obtained by solid-phase mixing of mixture synthesized in step b1 | | | | | | | | |
|---|---|---|---|---|---|---|---|---|
| ID | Exact Mass | | A | a | B | b | C | c |
| 4-1-D00-1-0001 | 487.08 | OH | A01 | a1 | B01 | b1 | C01 | Br |
| 4-1-D00-1-0002 | 487.08 | OH | A02 | a1 | B01 | b1 | C01 | Br |
| 4-1-D00-1-0003 | 501.09 | OH | A01 | a1 | B02 | b1 | C01 | Br |
| 4-1-D00-1-0004 | 501.09 | OH | A02 | a1 | B02 | b1 | C01 | Br |
| 4-1-D00-1-0005 | 505.07 | OH | A01 | a1 | B03 | b1 | C01 | Br |
| 4-1-D00-1-0006 | 505.07 | OH | A02 | a1 | B03 | b1 | C01 | Br |
| 4-1-D00-1-0007 | 515.11 | OH | A01 | a1 | B04 | b1 | C01 | Br |
| 4-1-D00-1-0008 | 515.11 | OH | A02 | a1 | B04 | b1 | C01 | Br |
| 4-1-DOO-1-0009 | 487.08 | OH | A01 | a1 | B01 | b1 | C02 | Br |
| 4-1-D00-1-0010 | 487.08 | OH | A02 | a1 | B01 | b1 | C02 | Br |
| 4-1-D00-1-0011 | 501.09 | OH | A01 | a1 | B02 | b1 | C02 | Br |
| 4-1-D00-1-0012 | 501.09 | OH | A02 | a1 | B02 | b1 | C02 | Br |
| 4-1-D00-1-0013 | 505.07 | OH | A01 | a1 | B03 | b1 | C02 | Br |
| 4-1-D00-1-0014 | 505.07 | OH | A02 | a1 | B03 | b1 | C02 | Br |
| 4-1-D00-1-0015 | 515.11 | OH | A01 | a1 | B04 | b1 | C02 | Br |
| 4-1-D00-1-0016 | 515.11 | OH | A02 | a1 | B04 | b1 | C02 | Br |
| 4-1-D00-1-0017 | 491.05 | OH | A01 | a1 | B01 | b1 | C03 | Br |
| 4-1-D00-1-0018 | 491.05 | OH | A02 | a1 | B01 | b1 | C03 | Br |
| 4-1-DOO-1-0019 | 505.07 | OH | A01 | a1 | B02 | b1 | C03 | Br |
| 4-1-D00-1-0020 | 505.07 | OH | A02 | a1 | B02 | b1 | C03 | Br |
| 4-1-DOO-1-0021 | 509.04 | OH | A01 | a1 | B03 | b1 | C03 | Br |
| 4-1-D00-1-0022 | 509.04 | OH | A02 | a1 | B03 | b1 | C03 | Br |
| 4-1-D00-1-0023 | 519.09 | OH | A01 | a1 | B04 | b1 | C03 | Br |
| 4-1-D00-1-0024 | 519.09 | OH | A02 | a1 | B04 | b1 | C03 | Br |
| 4-1-D00-1-0025 | 501.09 | OH | A01 | a1 | B01 | b1 | C04 | Br |
| 4-1-D00-1-0026 | 501.09 | OH | A02 | a1 | B01 | b1 | C04 | Br |
| 4-1-D00-1-0027 | 515.11 | OH | A01 | a1 | B02 | b1 | C04 | Br |
| 4-1-D00-1-0028 | 515.11 | OH | A02 | a1 | B02 | b1 | C04 | Br |
| 4-1-D00-1-0029 | 519.09 | OH | A01 | a1 | B03 | b1 | C04 | Br |
| 4-1-D00-1-0030 | 519.09 | OH | A02 | a1 | B03 | b1 | C04 | Br |
| 4-1-D00-1-0031 | 529.13 | OH | A01 | a1 | B04 | b1 | C04 | Br |
| 4-1-D00-1-0032 | 529.13 | OH | A02 | a1 | B04 | b1 | C04 | Br |

Example 10-5: Formation of linker c1 by step c1 in case 1

A reaction formula in step c1 performed using BB4-1-09 to -12 will be illustrated below.

[Formula 706]

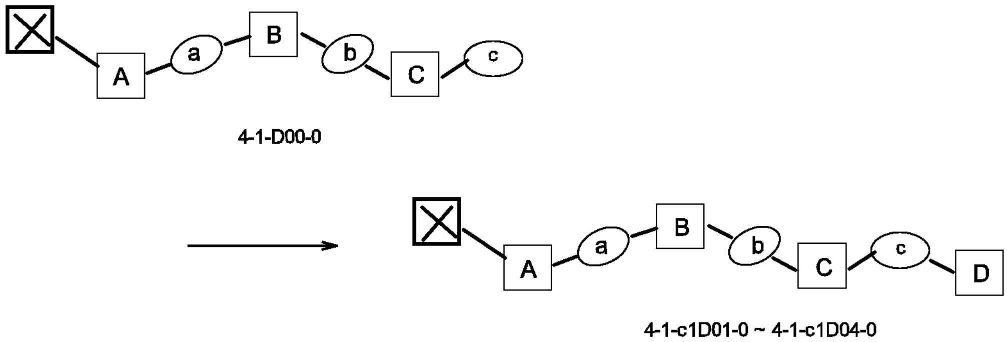


Mixture 4-1-c1D01-0 was synthesized as follows.

Under a nitrogen atmosphere, mixture 4-1-D00-0 (111 mg, 0.190 mmol/g) and THF (2.2 mL) were added to a 4 mL glass vial and shaken at room temperature for 1 hour. A sulfonamide reagent (BB4-1-09) (26.0 mg, 0.211 mmol) was added to the glass vial. tBuBrettphos Pd G4 (18.3 mg, 0.021 mmol) and P2Et (107 mg, 0.316 mmol) were added thereto, and the mixture was shaken at 60°C for 3 hours.

Solid-phase purification

The suspension of the reaction solution and the solid phase was transferred to a 3 mL column with a filter, filtered, and washed three times with NMP (2 mL), three times with NMP/water = 1/1 (2 mL), three times with 0.2 M NAC in NMP/water = 1/1 (2 mL), three times with 0.05 M $TBAHSO_4$ + DTBP in NMP (2 mL), three times with 0.05 M NMM in NMP (2 mL), three times with NMP/water = 1/1 (2 mL), three times with NMP (2 mL), three times with MeOH (2 mL), three times with DCM (2 mL), and three times with heptane (2 mL), and the obtained solid phase was dried under reduced pressure to obtain mixture 4-1- c1D01-0.

Step c1 was performed in the same manner as in the synthesis of mixture 4-1- c1D01-0 using BB4-1-10 to -12 instead of BB4-1-09 to obtain mixture 4-1-c 1 D02-0 to mixture 4-1-c 1 D04-0.

The sulfonamide reagents used in step c1 and the amounts of the reagents used are shown in Table 10-5-1. The mixtures obtained in step c1 are shown in Table 10-5-3 to Table 10-5-6.

[Table 533]

| [Table 10-5-1] Sulfonamide used and amount used in step c1 | | | | | | | |
|---|---|---|---|---|---|---|---|
| Starting material used | Amount used | Sulfonamide reagent used | Amount used | Molar number | Obtained mixture | Yield | Amount supported |
| 4-1-c1D00-0 | 111 mg | BB4-1-09 | 26.0 mg | 0.211 mmol | 4-1-c1D01-0 | 93.3 mg | 0.188 mmol/g |
| 4-1-c1D00-0 | 110 mg | BB4-1-10 | 46.3 mg | 0.209 mmol | 4-1-c1D02-0 | 102.7 mg | 0.185 mmol/g |
| 4-1-c1D00-0 | 110 mg | BB4-1-11 | 47.9 mg | 0.209 mmol | 4-1-c1D03-0 | 90.9 mg | 0.185 mmol/g |
| 4-1-c1D00-0 | 110 mg | BB4-1-12 | 51.3 mg | 0.209 mmol | 4-1-c1D04-0 | 99.8 mg | 0.184 mmol/g |

Mixture 4-1-c1D01-0 can be treated by cleavage in the same manner as in Example 10-1 to obtain mixture 4-1-c1D01-1. The details of the mixtures are shown in Table 10-5-3 to Table 10-5-6.

A notation method in each table will be described. For example, in Table 10-5-3, each compound that may be contained in 4-1-c1D01-1 is indicated by ID, and core blocks and linkers in combination in the structure of mixture 4-1-c1D01-1 are indicated by symbols or chemical formulas. The correspondence between the symbols and specific structures are shown in Figure 8. In Table 10-5-2 to Table 10-5-6, "×" is represented by a chemical formula.

[Formula 707]

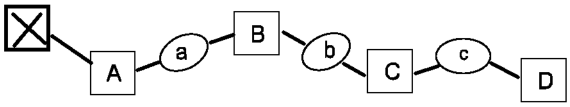

4-1-c1D01-0 ~ 4-1-c1D04-0

For example, when ID is 4-1-c1D01-1-0001, the compound is represented by the following structural formula.

[Formula 708]

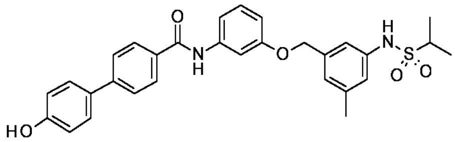

4-1-c1D01-1-0001

[Table 534]

| [Table 10-5-2] Structural notation in ID (4-1-c1D01-1-0001) | | | | | | | | |
|---|---|---|---|---|---|---|---|---|
| ID | | A | a | B | b | C | c | D |
| 4-1-c1D01-1-0001 | OH | A01 | a1 | B01 | b1 | C01 | c1 | D01 |

The mixture synthesized in step c1C01 using BB4-1-09 is shown in Table 10-5-3.

[Table 535]

| [Table 10-5-3] Compound contained in mixture (4-1-c1D01-1) synthesized in step c1C01 using BB4-1-09 | | | | | | | | | |
|---|---|---|---|---|---|---|---|---|---|
| ID | Exact Mass | | A | a | B | b | C | c | D |
| 4-1-c1D01-1-0001 | 530.19 | OH | A01 | a1 | B01 | b1 | C01 | c1 | D01 |
| 4-1-c1D01-1-0002 | 530.19 | OH | A02 | a1 | B01 | b1 | C01 | c1 | D01 |
| 4-1-c1D01-1-0003 | 544.20 | OH | A01 | a1 | B02 | b1 | C01 | c1 | D01 |
| 4-1-c1D01-1-0004 | 544.20 | OH | A02 | a1 | B02 | b1 | C01 | c1 | D01 |
| 4-1-c1D01-1-0005 | 548.18 | OH | A01 | a1 | B03 | b1 | C01 | c1 | D01 |
| 4-1-c1D01-1-0006 | 548.18 | OH | A02 | a1 | B03 | b1 | C01 | c1 | D01 |
| 4-1-c1D01-1-0007 | 558.22 | OH | A01 | a1 | B04 | b1 | C01 | c1 | D01 |
| 4-1-c1D01-1-0008 | 558.22 | OH | A02 | a1 | B04 | b1 | C01 | c1 | D01 |
| 4-1-c1D01-1-0009 | 530.19 | OH | A01 | a1 | B01 | b1 | C02 | c1 | D01 |
| 4-1-c1D01-1-0010 | 530.19 | OH | A02 | a1 | B01 | b1 | C02 | c1 | D01 |
| 4-1-c1D01-1-0011 | 544.20 | OH | A01 | a1 | B02 | b1 | C02 | c1 | D01 |
| 4-1-c1D01-1-0012 | 544.20 | OH | A02 | a1 | B02 | b1 | C02 | c1 | D01 |
| 4-1-c1D01-1-0013 | 548.18 | OH | A01 | a1 | B03 | b1 | C02 | c1 | D01 |
| 4-1-c1D01-1-0014 | 548.18 | OH | A02 | a1 | B03 | b1 | C02 | c1 | D01 |
| 4-1-c1D01-1-0015 | 558.22 | OH | A01 | a1 | B04 | b1 | C02 | c1 | D01 |
| 4-1-c1D01-1-0016 | 558.22 | OH | A02 | a1 | B04 | b1 | C02 | c1 | D01 |

(continued)

| [Table 10-5-3] Compound contained in mixture (4-1-c1D01-1) synthesized in step c1C01 using BB4-1-09 | | | | | | | | | |
|---|---|---|---|---|---|---|---|---|---|
| ID | Exact Mass | | A | a | B | b | C | c | D |
| 4-1-c1D01-1-0017 | 534.16 | OH | A01 | a1 | B01 | b1 | C03 | c1 | D01 |
| 4-1-c1D01-1-0018 | 534.16 | OH | A02 | a1 | B01 | b1 | C03 | c1 | D01 |
| 4-1-c1D01-1-0019 | 548.18 | OH | A01 | a1 | B02 | b1 | C03 | c1 | D01 |
| 4-1-c1D01-1-0020 | 548.18 | OH | A02 | a1 | B02 | b1 | C03 | c1 | D01 |
| 4-1-c1D01-1-0021 | 552.15 | OH | A01 | a1 | B03 | b1 | C03 | c1 | D01 |
| 4-1-c1D01-1-0022 | 552.15 | OH | A02 | a1 | B03 | b1 | C03 | c1 | D01 |
| 4-1-c1D01-1-0023 | 562.19 | OH | A01 | a1 | B04 | b1 | C03 | c1 | D01 |
| 4-1-c1D01-1-0024 | 562.19 | OH | A02 | a1 | B04 | b1 | C03 | c1 | D01 |
| 4-1-c1D01-1-0025 | 544.20 | OH | A01 | a1 | B01 | b1 | C04 | c1 | D01 |
| 4-1-c1D01-1-0026 | 544.20 | OH | A02 | a1 | B01 | b1 | C04 | c1 | D01 |
| 4-1-c1D01-1-0027 | 558.22 | OH | A01 | a1 | B02 | b1 | C04 | c1 | D01 |
| 4-1-c1D01-1-0028 | 558.22 | OH | A02 | a1 | B02 | b1 | C04 | c1 | D01 |
| 4-1-c1D01-1-0029 | 562.19 | OH | A01 | a1 | B03 | b1 | C04 | c1 | D01 |
| 4-1-c1D01-1-0030 | 562.19 | OH | A02 | a1 | B03 | b1 | C04 | c1 | D01 |
| 4-1-c1D01-1-0031 | 572.23 | OH | A01 | a1 | B04 | b1 | C04 | c1 | D01 |
| 4-1-c1D01-1-0032 | 572.23 | OH | A02 | a1 | B04 | b1 | C04 | c1 | D01 |

The mixture synthesized in step c1C02 using BB4-1-10 is shown in Table 10-5-4.

[Table 536]

| [Table 10-5-4] Compound contained in mixture (4-1-c1D02-1) synthesized in step c1D02 using BB4-1-10 | | | | | | | | | |
|---|---|---|---|---|---|---|---|---|---|
| ID | Exact Mass | | A | a | B | b | C | c | D |
| 4-1-c1D02-1-0001 | 628.13 | OH | A01 | a1 | B01 | b1 | C01 | c1 | D02 |
| 4-1-c1D02-1-0002 | 628.13 | OH | A02 | a1 | B01 | b1 | C01 | c1 | D02 |
| 4-1-c1D02-1-0003 | 642.15 | OH | A01 | a1 | B02 | b1 | C01 | c1 | D02 |
| 4-1-c1D02-1-0004 | 642.15 | OH | A02 | a1 | B02 | b1 | C01 | c1 | D02 |
| 4-1-c1D02-1-0005 | 646.12 | OH | A01 | a1 | B03 | b1 | C01 | c1 | D02 |
| 4-1-c1D02-1-0006 | 646.12 | OH | A02 | a1 | B03 | b1 | C01 | c1 | D02 |
| 4-1-c1D02-1-0007 | 656.17 | OH | A01 | a1 | B04 | b1 | C01 | c1 | D02 |
| 4-1-c1D02-1-0008 | 656.17 | OH | A02 | a1 | B04 | b1 | C01 | c1 | D02 |
| 4-1-c1D02-1-0009 | 628.13 | OH | A01 | a1 | B01 | b1 | C02 | c1 | D02 |
| 4-1-c1D02-1-0010 | 628.13 | OH | A02 | a1 | B01 | b1 | C02 | c1 | D02 |
| 4-1-c1D02-1-0011 | 642.15 | OH | A01 | a1 | B02 | b1 | C02 | c1 | D02 |
| 4-1-c1D02-1-0012 | 642.15 | OH | A02 | a1 | B02 | b1 | C02 | c1 | D02 |
| 4-1-c1D02-1-0013 | 646.12 | OH | A01 | a1 | B03 | b1 | C02 | c1 | D02 |
| 4-1-c1D02-1-0014 | 646.12 | OH | A02 | a1 | B03 | b1 | C02 | c1 | D02 |
| 4-1-c1D02-1-0015 | 656.17 | OH | A01 | a1 | B04 | b1 | C02 | c1 | D02 |

(continued)

| [Table 10-5-4] Compound contained in mixture (4-1-c1D02-1) synthesized in step c1D02 using BB4-1-10 | | | | | | | | | |
|---|---|---|---|---|---|---|---|---|---|
| ID | Exact Mass | | A | a | B | b | C | c | D |
| 4-1-c1D02-1-0016 | 656.17 | OH | A02 | a1 | B04 | b1 | C02 | c1 | D02 |
| 4-1-c1D02-1-0017 | 632.11 | OH | A01 | a1 | B01 | b1 | C03 | c1 | D02 |
| 4-1-c1D02-1-0018 | 632.11 | OH | A02 | a1 | B01 | b1 | C03 | c1 | D02 |
| 4-1-c1D02-1-0019 | 646.12 | OH | A01 | a1 | B02 | b1 | C03 | c1 | D02 |
| 4-1-c1D02-1-0020 | 646.12 | OH | A02 | a1 | B02 | b1 | C03 | c1 | D02 |
| 4-1-c1D02-1-0021 | 650.10 | OH | A01 | a1 | B03 | b1 | C03 | c1 | D02 |
| 4-1-c1D02-1-0022 | 650.10 | OH | A02 | a1 | B03 | b1 | C03 | c1 | D02 |
| 4-1-c1D02-1-0023 | 660.14 | OH | A01 | a1 | B04 | b1 | C03 | c1 | D02 |
| 4-1-c1D02-1-0024 | 660.14 | OH | A02 | a1 | B04 | b1 | C03 | c1 | D02 |
| 4-1-c1D02-1-0025 | 642.15 | OH | A01 | a1 | B01 | b1 | C04 | c1 | D02 |
| 4-1-c1D02-1-0026 | 642.15 | OH | A02 | a1 | B01 | b1 | C04 | c1 | D02 |
| 4-1-c1D02-1-0027 | 656.17 | OH | A01 | a1 | B02 | b1 | C04 | c1 | D02 |
| 4-1-c1D02-1-0028 | 656.17 | OH | A02 | a1 | B02 | b1 | C04 | c1 | D02 |
| 4-1-c1D02-1-0029 | 660.14 | OH | A01 | a1 | B03 | b1 | C04 | c1 | D02 |
| 4-1-c1D02-1-0030 | 660.14 | OH | A02 | a1 | B03 | b1 | C04 | c1 | D02 |
| 4-1-c1D02-1-0031 | 670.18 | OH | A01 | a1 | B04 | b1 | C04 | c1 | D02 |
| 4-1-c1D02-1-0032 | 670.18 | OH | A02 | a1 | B04 | b1 | C04 | c1 | D02 |

The mixture synthesized in step c1C03 using BB4-1-11 is shown in Table 10-5-5.

[Table 537]

| [Table 10-5-5] Compound contained in mixture (4-1-c1D03-1) synthesized in step c1D03 using BB4-1-11 | | | | | | | | | |
|---|---|---|---|---|---|---|---|---|---|
| ID | Exact Mass | | A | a | B | b | C | c | D |
| 4-1-c1D03-1-0001 | 636.19 | OH | A01 | a1 | B01 | b1 | C01 | c1 | D03 |
| 4-1-c1D03-1-0002 | 636.19 | OH | A02 | a1 | B01 | b1 | C01 | c1 | D03 |
| 4-1-c1D03-1-0003 | 650.21 | OH | A01 | a1 | B02 | b1 | C01 | c1 | D03 |
| 4-1-c1D03-1-0004 | 650.21 | OH | A02 | a1 | B02 | b1 | C01 | c1 | D03 |
| 4-1-c1D03-1-0005 | 654.18 | OH | A01 | a1 | B03 | b1 | C01 | c1 | D03 |
| 4-1-c1D03-1-0006 | 654.18 | OH | A02 | a1 | B03 | b1 | C01 | c1 | D03 |
| 4-1-c1D03-1-0007 | 664.22 | OH | A01 | a1 | B04 | b1 | C01 | c1 | D03 |
| 4-1-c1D03-1-0008 | 664.22 | OH | A02 | a1 | B04 | b1 | C01 | c1 | D03 |
| 4-1-c1D03-1-0009 | 636.19 | OH | A01 | a1 | B01 | b1 | C02 | c1 | D03 |
| 4-1-c1D03-1-0010 | 636.19 | OH | A02 | a1 | B01 | b1 | C02 | c1 | D03 |
| 4-1-c1D03-1-0011 | 650.21 | OH | A01 | a1 | B02 | b1 | C02 | c1 | D03 |
| 4-1-c1D03-1-0012 | 650.21 | OH | A02 | a1 | B02 | b1 | C02 | c1 | D03 |
| 4-1-c1D03-1-0013 | 654.18 | OH | A01 | a1 | B03 | b1 | C02 | c1 | D03 |
| 4-1-c1D03-1-0014 | 654.18 | OH | A02 | a1 | B03 | b1 | C02 | c1 | D03 |

(continued)

| [Table 10-5-5] Compound contained in mixture (4-1-c1D03-1) synthesized in step c1D03 using BB4-1-11 | | | | | | | | | |
|---|---|---|---|---|---|---|---|---|---|
| ID | Exact Mass | | A | a | B | b | C | c | D |
| 4-1-c1D03-1-0015 | 664.22 | OH | A01 | a1 | B04 | b1 | C02 | c1 | D03 |
| 4-1-c1D03-1-0016 | 664.22 | OH | A02 | a1 | B04 | b1 | C02 | c1 | D03 |
| 4-1-c1D03-1-0017 | 640.17 | OH | A01 | a1 | B01 | b1 | C03 | c1 | D03 |
| 4-1-c1D03-1-0018 | 640.17 | OH | A02 | a1 | B01 | b1 | C03 | c1 | D03 |
| 4-1-c1D03-1-0019 | 654.18 | OH | A01 | a1 | B02 | b1 | C03 | c1 | D03 |
| 4-1-c1D03-1-0020 | 654.18 | OH | A02 | a1 | B02 | b1 | C03 | c1 | D03 |
| 4-1-c1D03-1-0021 | 658.16 | OH | A01 | a1 | B03 | b1 | C03 | c1 | D03 |
| 4-1-c1D03-1-0022 | 658.16 | OH | A02 | a1 | B03 | b1 | C03 | c1 | D03 |
| 4-1-c1D03-1-0023 | 668.20 | OH | A01 | a1 | B04 | b1 | C03 | c1 | D03 |
| 4-1-c1D03-1-0024 | 668.20 | OH | A02 | a1 | B04 | b1 | C03 | c1 | D03 |
| 4-1-c1D03-1-0025 | 650.21 | OH | A01 | a1 | B01 | b1 | C04 | c1 | D03 |
| 4-1-c1D03-1-0026 | 650.21 | OH | A02 | a1 | B01 | b1 | C04 | c1 | D03 |
| 4-1-c1D03-1-0027 | 664.22 | OH | A01 | a1 | B02 | b1 | C04 | c1 | D03 |
| 4-1-c1D03-1-0028 | 664.22 | OH | A02 | a1 | B02 | b1 | C04 | c1 | D03 |
| 4-1-c1D03-1-0029 | 668.20 | OH | A01 | a1 | B03 | b1 | C04 | c1 | D03 |
| 4-1-c1D03-1-0030 | 668.20 | OH | A02 | a1 | B03 | b1 | C04 | c1 | D03 |
| 4-1-c1D03-1-0031 | 678.24 | OH | A01 | a1 | B04 | b1 | C04 | c1 | D03 |
| 4-1-c1D03-1-0032 | 678.24 | OH | A02 | a1 | B04 | b1 | C04 | c1 | D03 |

The mixture synthesized in step c1C04 using BB4-1-12 is shown in Table 10-5-6.

[Table 538]

| [Table 10-5-6] Compound contained in mixture (4-1-c1D04-1) synthesized in step c1D04 using BB4-1-12 | | | | | | | | | |
|---|---|---|---|---|---|---|---|---|---|
| ID | Exact Mass | | A | a | B | b | C | c | D |
| 4-1-c1D04-1-0001 | 652.19 | OH | A01 | a1 | B01 | b1 | C01 | c1 | D04 |
| 4-1-c1D04-1-0002 | 652.19 | OH | A02 | a1 | B01 | b1 | C01 | c1 | D04 |
| 4-1-c1D04-1-0003 | 666.20 | OH | A01 | a1 | B02 | b1 | C01 | c1 | D04 |
| 4-1-c1D04-1-0004 | 666.20 | OH | A02 | a1 | B02 | b1 | C01 | c1 | D04 |
| 4-1-c1D04-1-0005 | 670.18 | OH | A01 | a1 | B03 | b1 | C01 | c1 | D04 |
| 4-1-c1D04-1-0006 | 670.18 | OH | A02 | a1 | B03 | b1 | C01 | c1 | D04 |
| 4-1-c1D04-1-0007 | 680.22 | OH | A01 | a1 | B04 | b1 | C01 | c1 | D04 |
| 4-1-c1D04-1-0008 | 680.22 | OH | A02 | a1 | B04 | b1 | C01 | c1 | D04 |
| 4-1-c1D04-1-0009 | 652.19 | OH | A01 | a1 | B01 | b1 | C02 | c1 | D04 |
| 4-1-c1D04-1-0010 | 652.19 | OH | A02 | a1 | B01 | b1 | C02 | c1 | D04 |
| 4-1-c1D04-1-0011 | 666.20 | OH | A01 | a1 | B02 | b1 | C02 | c1 | D04 |
| 4-1-c1D04-1-0012 | 666.20 | OH | A02 | a1 | B02 | b1 | C02 | c1 | D04 |
| 4-1-c1D04-1-0013 | 670.18 | OH | A01 | a1 | B03 | b1 | C02 | c1 | D04 |
| 4-1-c1D04-1-0014 | 670.18 | OH | A02 | a1 | B03 | b1 | C02 | c1 | D04 |
| 4-1-c1D04-1-0015 | 680.22 | OH | A01 | a1 | B04 | b1 | C02 | c1 | D04 |

(continued)

| [Table 10-5-6] Compound contained in mixture (4-1-c1D04-1) synthesized in step c1D04 using BB4-1-12 | | | | | | | | | |
|---|---|---|---|---|---|---|---|---|---|
| ID | Exact Mass | | A | a | B | b | C | c | D |
| 4-1-c1D04-1-0016 | 680.22 | OH | A02 | a1 | B04 | b1 | C02 | c1 | D04 |
| 4-1-c1D04-1-0017 | 656.16 | OH | A01 | a1 | B01 | b1 | C03 | c1 | D04 |
| 4-1-c1D04-1-0018 | 656.16 | OH | A02 | a1 | B01 | b1 | C03 | c1 | D04 |
| 4-1-c1D04-1-0019 | 670.18 | OH | A01 | a1 | B02 | b1 | C03 | c1 | D04 |
| 4-1-c1D04-1-0020 | 670.18 | OH | A02 | a1 | B02 | b1 | C03 | c1 | D04 |
| 4-1-c1D04-1-0021 | 674.15 | OH | A01 | a1 | B03 | b1 | C03 | c1 | D04 |
| 4-1-c1D04-1-0022 | 674.15 | OH | A02 | a1 | B03 | b1 | C03 | c1 | D04 |
| 4-1-c1D04-1-0023 | 684.19 | OH | A01 | a1 | B04 | b1 | C03 | c1 | D04 |
| 4-1-c1D04-1-0024 | 684.19 | OH | A02 | a1 | B04 | b1 | C03 | c1 | D04 |
| 4-1-c1D04-1-0025 | 666.20 | OH | A01 | a1 | B01 | b1 | C04 | c1 | D04 |
| 4-1-c1D04-1-0026 | 666.20 | OH | A02 | a1 | B01 | b1 | C04 | c1 | D04 |
| 4-1-c1D04-1-0027 | 680.22 | OH | A01 | a1 | B02 | b1 | C04 | c1 | D04 |
| 4-1-c1D04-1-0028 | 680.22 | OH | A02 | a1 | B02 | b1 | C04 | c1 | D04 |
| 4-1-c1D04-1-0029 | 684.19 | OH | A01 | a1 | B03 | b1 | C04 | c1 | D04 |
| 4-1-c1D04-1-0030 | 684.19 | OH | A02 | a1 | B03 | b1 | C04 | c1 | D04 |
| 4-1-c1D04-1-0031 | 694.23 | OH | A01 | a1 | B04 | b1 | C04 | c1 | D04 |
| 4-1-c1D04-1-0032 | 694.23 | OH | A02 | a1 | B04 | b1 | C04 | c1 | D04 |

Example 10-6: Synthesis of mixture 4-1-c1D01-1 to mixture 4-1-c1D04-1 by cleavage steps D01-1 to D04-1 in case 1

A reaction formula in step D01-1 performed using mixture 4-1-c1D01-0 will be illustrated below.

[Formula 709]

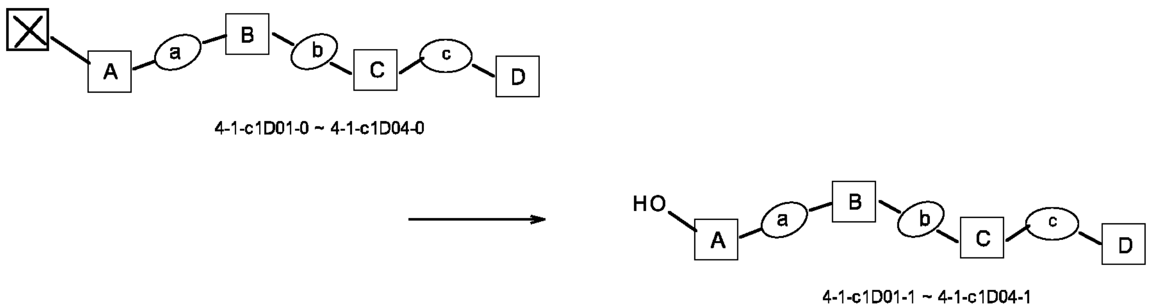


Mixture 4-1-c1D01-1 to mixture 4-1-c1D04-1 were synthesized as follows using mixture 4-1-c1D01-0 to mixture 4-1-c 1 D04-0.

Under a nitrogen atmosphere, separate mixtures (78 mg) described in Table 10-6-1 were added to four 4 mL glass vials, respectively. Pentamethylbenzene (17.1 mg, 0.115 mmol) and DCM (1.4 mL) were added thereto, and each mixture was shaken at room temperature for 1 hour. TFA (156 $\mu$L) was added thereto, and the mixture was shaken at room temperature for 1 hour. The suspension of the reaction solution and the solid phase was transferred to a 3 mL column with a filter using DMF (0.5 mL) twice and filtered. The resultant was washed three times with DMF (0.5 mL), and combined filtrates were collected into a 15 mm*75 mm test tube. The solvent in the solution was distilled off under reduced pressure (Genevac reduced-pressure concentrator was used). Solid-phase supported morpholine (Sigma-Aldrich Co., LLC, catalog No: 493813) (160 mg) and DMF (2.0 mL) were added thereto, and the mixture was shaken at room temperature for 20 minutes. The solution and the suspension of the solid phase were transferred to a 3 mL column with a filter using DMF (0.5 mL) twice and filtered. The resultant was washed three times with DMF (0.5 mL) and twice with DCM/DMSO (2/1, 0.5 mL), and combined filtrates were collected into a 15 mm*75 mm test tube. The solvent in the

solution was distilled off under reduced pressure (Genevac reduced-pressure concentrator was used) to obtain the mixtures (4-1- c1D01-1 to 4-1-c1D04-1) described in Table 10-6-1.

[Table 539]

| [Table 10-6-1] Starting material used and obtained mixture in steps D01-1 to D04-1 | | | | |
|---|---|---|---|---|
| Mixture used | Amount used | Amount of solid- phase supported morpholine used | Obtained mixture | Yield |
| 4-1-c1D01-0 | 78.0 mg | 160 mg | 4-1-c1D01-1 | 8.1 mg |
| 4-1-c1D02-0 | 82.4 mg | 160 mg | 4-1-c1D02-1 | 9.4 mg |
| 4-1-c1D03-0 | 77.2 mg | 160 mg | 4-1-c1D03-1 | 7.6 mg |
| 4-1-c1D04-0 | 81.5 mg | 160 mg | 4-1-c1D04-1 | 9.2 mg |

Example 10-7: Synthesis of mixture 4-1-h01D09-2 to mixture 4-1-h03D11-2 by hydrolysis steps h01D09 to h03D11 in case 1

A reaction formula in step h01D09 performed using mixture 4-1-c1D02-1 will be illustrated below.

[Formula 710]

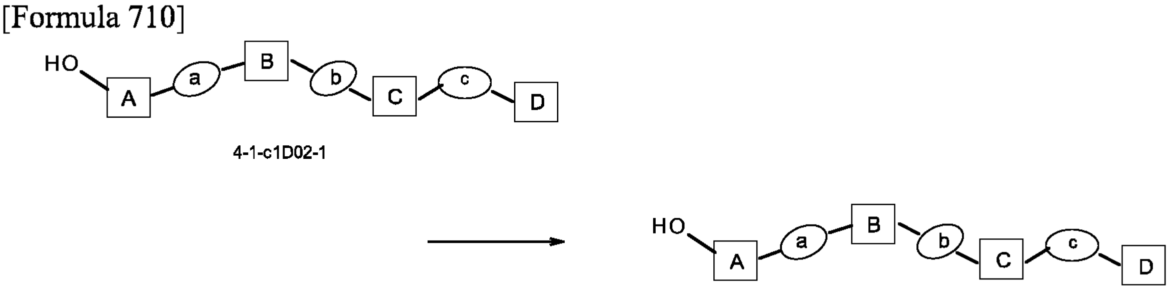

4-1-c1D02-1

4-1-h01D09-2

Mixture 4-1-h01D09-2 to mixture 4-1-h03D11-2 were synthesized as follows using mixture 4-1-c1D02-1 to mixture 4-1-c1D04-1. The following operation shows step h01D09.

Under a nitrogen atmosphere, DMF (658 μL) and tAmOH (188 μL) were added to mixture 4-1-c1D02-1 (9.4 mg) concentrated in a 1 5mm*75 mm test tube. A 2 M aqueous NaOH solution (29.0 μL) was added to the solution, and the mixture was stirred at room temperature for 3 hours. MP-TsOH (Biotage Japan Ltd., catalog No: 800462) (78 mg) was added thereto, and the mixture was shaken at room temperature for 30 minutes. The suspension of the reaction solution and the solid phase was transferred to a 3 mL column with a filter using DMF (0.5 mL) twice and filtered. The resultant was washed three times with DMF (0.5 mL) and twice with DCM/DMSO (2/1, 0.5 mL), and combined filtrates were collected into a 15 mm*75 mm test tube. The solvent in the solution was distilled off under reduced pressure (Genevac reduced-pressure concentrator was used) to obtain 8.8 mg of mixture 4-1-h01D09-2.

Steps h02D10 and h03D 1 1 were performed in the same manner as in the synthesis of mixture 4-1-h01D09-2 to obtain mixture 4-1-h02D10-2, and mixture 4-1-h03D11-2.

The starting materials used in steps h01 to h03, the amounts of the starting materials used, and the yields of the obtained mixtures are shown in Table 10-7-1. The mixtures obtained in steps h01 to h03 are shown in Table 10-7-3 to Table 10-7-6.

[Table 540]

| [Table 10-7-1] Starting material used and obtained mixture in hydrolysis steps h01D09-2 to h03D11-2 | | | | | | | |
|---|---|---|---|---|---|---|---|
| Mixture used | Amount used | DMF | tAmOH | Amount of 2 M NaOH aq. used | Amount of MP- TsOH used | Obtained mixture | Yield |
| 4-1-c1D02-1 | 9.4 mg | 658 μL | 188 μL | 29.0 μL (0.058 mmol) | 78 mg | 4-1-h01D09-2 | 8.8 mg |
| 4-1-c1D03-1 | 7.6 mg | 532 μL | 152 μL | 23.2 μL (0.046 mmol) | 62 mg | 4-1-h02D10-2 | 10.8 mg |

(continued)

| [Table 10-7-1] Starting material used and obtained mixture in hydrolysis steps h01D09-2 to h03D11-2 | | | | | | | |
|---|---|---|---|---|---|---|---|
| Mixture used | Amount used | DMF | tAmOH | Amount of 2 M NaOH aq. used | Amount of MP- TsOH used | Obtained mixture | Yield |
| 4-1-c1D04-1 | 9.2 mg | 644 μL | 184 μL | 27.4 μL (0.055 mmol) | 74 mg | 4-1-h03D11-2 | 15.2 mg |

The details of the mixtures are shown in Table 10-7-3 to Table 10-7-5.

A notation method in each table will be described. For example, in Table 10-7-3, each compound that may be contained in 4-1-h01D09-2 is indicated by ID, and core blocks and linkers in combination in the structure of mixture 4-1-h01D09-2 are indicated by symbols or chemical formulas. The correspondence between the symbols and specific structures are shown in Figure 8.

In Table 10-7-2 to Table 10-7-5, "×" is represented by a chemical formula.

[Formula 711]

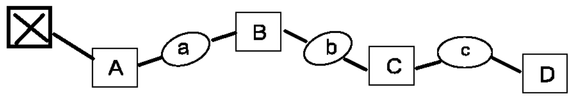


4-1-h01D09-2 ~ 4-1-h03D11-2

For example, when ID is 4-1-h01D09-2-0001, the compound is represented by the following structural formula.

[Formula 712]

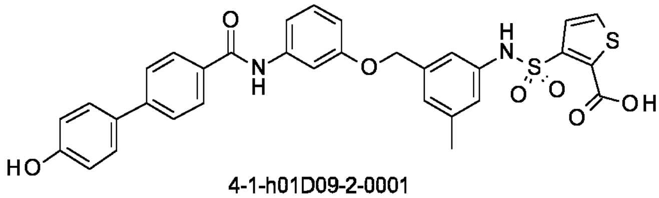

4-1-h01D09-2-0001

[Table 541]

| [Table 10-7-2] Structural notation in ID (4-1-h01D09-2-0001) | | | | | | | | |
|---|---|---|---|---|---|---|---|---|
| ID | | A | a | B | b | C | c | D |
| 4-1-h01D09-2-0001 | OH | A01 | a1 | B01 | b1 | C01 | c1 | D09 |

The mixture synthesized in step h01D09 from mixture 4-1-c1D02-1 is shown in Table 10-7-3.

[Table 542]

| [Table 10-7-3] Compound contained in mixture (4-1-h01D09-2) synthesized in step h01D09 from mixture 4-1-c 1 D02-1 | | | | | | | | | |
|---|---|---|---|---|---|---|---|---|---|
| ID | Exact Mass | | A | a | B | b | C | c | D |
| 4-1-h01D09-2-0001 | 614.12 | OH | A01 | a1 | B01 | b1 | C01 | c1 | D09 |
| 4-1-h01D09-2-0002 | 614.12 | OH | A02 | a1 | B01 | b1 | C01 | c1 | D09 |
| 4-1-h01D09-2-0003 | 628.13 | OH | A01 | a1 | B02 | b1 | C01 | c1 | D09 |
| 4-1-h01D09-2-0004 | 628.13 | OH | A02 | a1 | B02 | b1 | C01 | c1 | D09 |
| 4-1-h01D09-2-0005 | 632.11 | OH | A01 | a1 | B03 | b1 | C01 | c1 | D09 |
| 4-1-h01D09-2-0006 | 632.11 | OH | A02 | a1 | B03 | b1 | C01 | c1 | D09 |

(continued)

| [Table 10-7-3] Compound contained in mixture (4-1-h01D09-2) synthesized in step h01D09 from mixture 4-1-c 1 D02-1 | | | | | | | | | |
|---|---|---|---|---|---|---|---|---|---|
| ID | Exact Mass | | A | a | B | b | C | c | D |
| 4-1-h01D09-2-0007 | 642.15 | OH | A01 | a1 | B04 | b1 | C01 | c1 | D09 |
| 4-1-h01D09-2-0008 | 642.15 | OH | A02 | a1 | B04 | b1 | C01 | c1 | D09 |
| 4-1-h01D09-2-0009 | 614.12 | OH | A01 | a1 | B01 | b1 | C02 | c1 | D09 |
| 4-1-h01D09-2-0010 | 614.12 | OH | A02 | a1 | B01 | b1 | C02 | c1 | D09 |
| 4-1-h01D09-2-0011 | 628.13 | OH | A01 | a1 | B02 | b1 | C02 | c1 | D09 |
| 4-1-h01D09-2-0012 | 628.13 | OH | A02 | a1 | B02 | b1 | C02 | c1 | D09 |
| 4-1-h01D09-2-0013 | 632.11 | OH | A01 | a1 | B03 | b1 | C02 | c1 | D09 |
| 4-1-h01D09-2-0014 | 632.11 | OH | A02 | a1 | B03 | b1 | C02 | c1 | D09 |
| 4-1-h01D09-2-0015 | 642.15 | OH | A01 | a1 | B04 | b1 | C02 | c1 | D09 |
| 4-1-h01D09-2-0016 | 642.15 | OH | A02 | a1 | B04 | b1 | C02 | c1 | D09 |
| 4-1-h01D09-2-0017 | 618.09 | OH | A01 | a1 | B01 | b1 | C03 | c1 | D09 |
| 4-1-h01D09-2-0018 | 618.09 | OH | A02 | a1 | B01 | b1 | C03 | c1 | D09 |
| 4-1-h01D09-2-0019 | 632.11 | OH | A01 | a1 | B02 | b1 | C03 | c1 | D09 |
| 4-1-h01D09-2-0020 | 632.11 | OH | A02 | a1 | B02 | b1 | C03 | c1 | D09 |
| 4-1-h01D09-2-0021 | 636.08 | OH | A01 | a1 | B03 | b1 | C03 | c1 | D09 |
| 4-1-h01D09-2-0022 | 636.08 | OH | A02 | a1 | B03 | b1 | C03 | c1 | D09 |
| 4-1-h01D09-2-0023 | 646.12 | OH | A01 | a1 | B04 | b1 | C03 | c1 | D09 |
| 4-1-h01D09-2-0024 | 646.12 | OH | A02 | a1 | B04 | b1 | C03 | c1 | D09 |
| 4-1-h01D09-2-0025 | 628.13 | OH | A01 | a1 | B01 | b1 | C04 | c1 | D09 |
| 4-1-h01D09-2-0026 | 628.13 | OH | A02 | a1 | B01 | b1 | C04 | c1 | D09 |
| 4-1-h01D09-2-0027 | 642.15 | OH | A01 | a1 | B02 | b1 | C04 | c1 | D09 |
| 4-1-h01D09-2-0028 | 642.15 | OH | A02 | a1 | B02 | b1 | C04 | c1 | D09 |
| 4-1-h01D09-2-0029 | 646.12 | OH | A01 | a1 | B03 | b1 | C04 | c1 | D09 |
| 4-1-h01D09-2-0030 | 646.12 | OH | A02 | a1 | B03 | b1 | C04 | c1 | D09 |
| 4-1-h01D09-2-0031 | 656.17 | OH | A01 | a1 | B04 | b1 | C04 | c1 | D09 |
| 4-1-h01D09-2-0032 | 656.17 | OH | A02 | a1 | B04 | b1 | C04 | c1 | D09 |

The mixture synthesized in step h02D10 from mixture 4-1-c1D03-1 is shown in Table 10-7-4.

[Table 543]

| [Table 10-7-4] Compound contained in mixture (4-1-h02D10-2) synthesized in step h02D10 from mixture 4-1-c1D03-1 | | | | | | | | | |
|---|---|---|---|---|---|---|---|---|---|
| ID | Exact Mass | | A | a | B | b | C | c | D |
| 4-1-h02D10-2-0001 | 622.18 | OH | A01 | a1 | B01 | b1 | C01 | c1 | D10 |
| 4-1-h02D10-2-0002 | 622.18 | OH | A02 | a1 | B01 | b1 | C01 | c1 | D10 |
| 4-1-h02D10-2-0003 | 636.19 | OH | A01 | a1 | B02 | b1 | C01 | c1 | D10 |
| 4-1-h02D10-2-0004 | 636.19 | OH | A02 | a1 | B02 | b1 | C01 | c1 | D10 |
| 4-1-h02D10-2-0005 | 640.17 | OH | A01 | a1 | B03 | b1 | C01 | c1 | D10 |
| 4-1-h02D10-2-0006 | 640.17 | OH | A02 | a1 | B03 | b1 | C01 | c1 | D10 |

(continued)

| [Table 10-7-4] Compound contained in mixture (4-1-h02D10-2) synthesized in step h02D10 from mixture 4-1-c1D03-1 | | | | | | | | | |
|---|---|---|---|---|---|---|---|---|---|
| ID | Exact Mass | | A | a | B | b | C | c | D |
| 4-1-h02D10-2-0007 | 650.21 | OH | A01 | a1 | B04 | b1 | C01 | c1 | D10 |
| 4-1-h02D10-2-0008 | 650.21 | OH | A02 | a1 | B04 | b1 | C01 | c1 | D10 |
| 4-1-h02D10-2-0009 | 622.18 | OH | A01 | a1 | B01 | b1 | C02 | c1 | D10 |
| 4-1-h02D10-2-0010 | 622.18 | OH | A02 | a1 | B01 | b1 | C02 | c1 | D10 |
| 4-1-h02D10-2-0011 | 636.19 | OH | A01 | a1 | B02 | b1 | C02 | c1 | D10 |
| 4-1-h02D10-2-0012 | 636.19 | OH | A02 | a1 | B02 | b1 | C02 | c1 | D10 |
| 4-1-h02D10-2-0013 | 640.17 | OH | A01 | a1 | B03 | b1 | C02 | c1 | D10 |
| 4-1-h02D10-2-0014 | 640.17 | OH | A02 | a1 | B03 | b1 | C02 | c1 | D10 |
| 4-1-h02D10-2-0015 | 650.21 | OH | A01 | a1 | B04 | b1 | C02 | c1 | D10 |
| 4-1-h02D10-2-0016 | 650.21 | OH | A02 | a1 | B04 | b1 | C02 | c1 | D10 |
| 4-1-h02D10-2-0017 | 626.15 | OH | A01 | a1 | B01 | b1 | C03 | c1 | D10 |
| 4-1-h02D10-2-0018 | 626.15 | OH | A02 | a1 | B01 | b1 | C03 | c1 | D10 |
| 4-1-h02D10-2-0019 | 640.17 | OH | A01 | a1 | B02 | b1 | C03 | c1 | D10 |
| 4-1-h02D10-2-0020 | 640.17 | OH | A02 | a1 | B02 | b1 | C03 | c1 | D10 |
| 4-1-h02D10-2-0021 | 644.14 | OH | A01 | a1 | B03 | b1 | C03 | c1 | D10 |
| 4-1-h02D10-2-0022 | 644.14 | OH | A02 | a1 | B03 | b1 | C03 | c1 | D10 |
| 4-1-h02D10-2-0023 | 654.18 | OH | A01 | a1 | B04 | b1 | C03 | c1 | D10 |
| 4-1-h02D10-2-0024 | 654.18 | OH | A02 | a1 | B04 | b1 | C03 | c1 | D10 |
| 4-1-h02D10-2-0025 | 636.19 | OH | A01 | a1 | B01 | b1 | C04 | c1 | D10 |
| 4-1-h02D10-2-0026 | 636.19 | OH | A02 | a1 | B01 | b1 | C04 | c1 | D10 |
| 4-1-h02D10-2-0027 | 650.21 | OH | A01 | a1 | B02 | b1 | C04 | c1 | D10 |
| 4-1-h02D10-2-0028 | 650.21 | OH | A02 | a1 | B02 | b1 | C04 | c1 | D10 |
| 4-1-h02D10-2-0029 | 654.18 | OH | A01 | a1 | B03 | b1 | C04 | c1 | D10 |
| 4-1-h02D10-2-0030 | 654.18 | OH | A02 | a1 | B03 | b1 | C04 | c1 | D10 |
| 4-1-h02D10-2-0031 | 664.22 | OH | A01 | a1 | B04 | b1 | C04 | c1 | D10 |
| 4-1-h02D10-2-0032 | 664.22 | OH | A02 | a1 | B04 | b1 | C04 | c1 | D10 |

The mixture synthesized in step h03D11 from mixture 4-1-c1D04-1 is shown in Table 10-7-5.

[Table 544]

| [Table 10-7-5] Compound contained in mixture (4-1h03D1 1-2) synthesized in step h03D11 from mixture 4-1-c1D04-1 | | | | | | | | | |
|---|---|---|---|---|---|---|---|---|---|
| ID | Exact Mass | | A | a | B | b | C | c | D |
| 4-1-h03D11-2-0001 | 638.17 | OH | A01 | a1 | B01 | b1 | C01 | c1 | D11 |
| 4-1-h03D11-2-0002 | 638.17 | OH | A02 | a1 | B01 | b1 | C01 | c1 | D11 |
| 4-1-h03D11-2-0003 | 652.19 | OH | A01 | a1 | B02 | b1 | C01 | c1 | D11 |
| 4-1-h03D11-2-0004 | 652.19 | OH | A02 | a1 | B02 | b1 | C01 | c1 | D11 |
| 4-1-h03D11-2-0005 | 656.16 | OH | A01 | a1 | B03 | b1 | C01 | c1 | D11 |
| 4-1-h03D11-2-0006 | 656.16 | OH | A02 | a1 | B03 | b1 | C01 | c1 | D11 |
| 4-1-h03D11-2-0007 | 666.20 | OH | A01 | a1 | B04 | b1 | C01 | c1 | D11 |
| 4-1-h03D11-2-0008 | 666.20 | OH | A02 | a1 | B04 | b1 | C01 | c1 | D11 |
| 4-1-h03D11-2-0009 | 638.17 | OH | A01 | a1 | B01 | b1 | C02 | c1 | D11 |
| 4-1-h03D11-2-0010 | 638.17 | OH | A02 | a1 | B01 | b1 | C02 | c1 | D11 |
| 4-1-h03D11-2-0011 | 652.19 | OH | A01 | a1 | B02 | b1 | C02 | c1 | D11 |
| 4-1-h03D11-2-0012 | 652.19 | OH | A02 | a1 | B02 | b1 | C02 | c1 | D11 |
| 4-1-h03D11-2-0013 | 656.16 | OH | A01 | a1 | B03 | b1 | C02 | c1 | D11 |
| 4-1-h03D11-2-0014 | 656.16 | OH | A02 | a1 | B03 | b1 | C02 | c1 | D11 |
| 4-1-h03D11-2-0015 | 666.20 | OH | A01 | a1 | B04 | b1 | C02 | c1 | D11 |
| 4-1-h03D11-2-0016 | 666.20 | OH | A02 | a1 | B04 | b1 | C02 | c1 | D11 |
| 4-1-h03D11-2-0017 | 642.15 | OH | A01 | a1 | B01 | b1 | C03 | c1 | D11 |
| 4-1-h03D11-2-0018 | 642.15 | OH | A02 | a1 | B01 | b1 | C03 | c1 | D11 |
| 4-1-h03D11-2-0019 | 656.16 | OH | A01 | a1 | B02 | b1 | C03 | c1 | D11 |
| 4-1-h03D11-2-0020 | 656.16 | OH | A02 | a1 | B02 | b1 | C03 | c1 | D11 |
| 4-1-h03D11-2-0021 | 660.14 | OH | A01 | a1 | B03 | b1 | C03 | c1 | D11 |
| 4-1-h03D11-2-0022 | 660.14 | OH | A02 | a1 | B03 | b1 | C03 | c1 | D11 |
| 4-1-h03D11-2-0023 | 670.18 | OH | A01 | a1 | B04 | b1 | C03 | c1 | D11 |
| 4-1-h03D11-2-0024 | 670.18 | OH | A02 | a1 | B04 | b1 | C03 | c1 | D11 |
| 4-1-h03D11-2-0025 | 652.19 | OH | A01 | a1 | B01 | b1 | C04 | c1 | D11 |
| 4-1-h03D11-2-0026 | 652.19 | OH | A02 | a1 | B01 | b1 | C04 | c1 | D11 |
| 4-1-h03D11-2-0027 | 666.20 | OH | A01 | a1 | B02 | b1 | C04 | c1 | D11 |
| 4-1-h03D11-2-0028 | 666.20 | OH | A02 | a1 | B02 | b1 | C04 | c1 | D11 |
| 4-1-h03D11-2-0029 | 670.18 | OH | A01 | a1 | B03 | b1 | C04 | c1 | D11 |
| 4-1-h03D11-2-0030 | 670.18 | OH | A02 | a1 | B03 | b1 | C04 | c1 | D11 |
| 4-1-h03D11-2-0031 | 680.22 | OH | A01 | a1 | B04 | b1 | C04 | c1 | D11 |
| 4-1-h03D11-2-0032 | 680.22 | OH | A02 | a1 | B04 | b1 | C04 | c1 | D11 |

Example 10-8: Mixing of mixture 4-1-c1D01-1 and mixture 4-1-h01D09-2 to 4-1- h03D11-2 in case 1

[Formula 713]

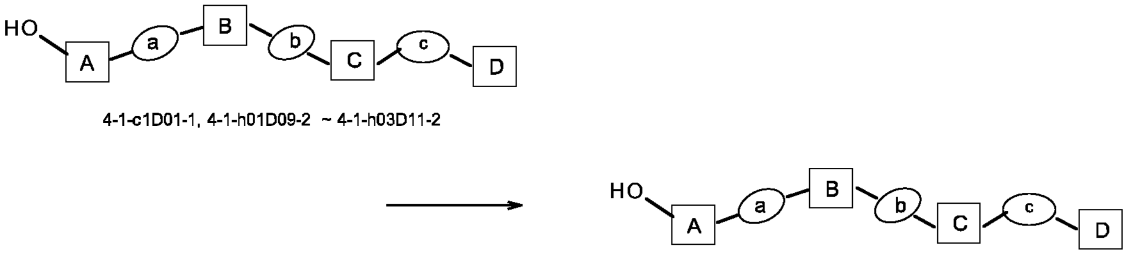

4-1-c1D01-1, 4-1-h01D09-2 ~ 4-1-h03D11-2

4-1-E00-1

4 types of mixtures described in Table 10-8-1 were mixed in a 15 mm*75 mm test tube using DMF (1.2 mL for each), and the solvent in the obtained solution was distilled off under reduced pressure (Genevac reduced-pressure concentrator was used) to obtain 24.6 mg of mixture 4-1-E00-1.

The mixtures used and the amounts of the mixtures used are shown in Table 10-8-1.

[Table 545]

| [Table 10-8-1] Mixture used and amount used in mixing | |
|---|---|
| Mixture | Amount used |
| 4-1-c1D01-1 | 8.1 mg |
| 4-1-h01 D09-2 | 8.8 mg |
| 4-1-h02D10-2 | 10.8 mg |
| 4-1-h03D11-2 | 15.2 mg |

The details of the mixtures are shown in Table 10-8-3.

A notation method in each table will be described. For example, in Table 10-8-3, each compound that may be contained in 4-1-E00-1 is indicated by ID, and core blocks and linkers in combination in the structure of mixture 4-1-E00-1 are indicated by symbols or chemical formulas. The correspondence between the symbols and specific structures are shown in Figure 8.

In Table 10-8-2 and Table 10-8-3, "×" is represented by a chemical formula.

[Formula 714]

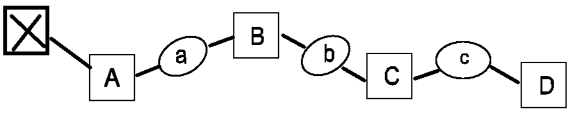

4-1-E00-1

For example, when ID is 4-1-E00-1-0001, the compound is represented by the following structural formula.

[Formula 715]

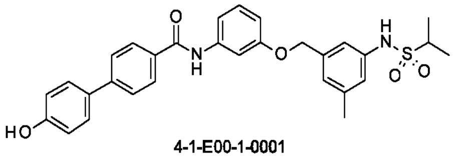

4-1-E00-1-0001

[Table 546]

| [Table 10-8-2] Structural notation in ID (4-1-E00-1-0001) | | | | | | | | |
|---|---|---|---|---|---|---|---|---|
| ID | | A | a | B | b | C | c | D |
| 4-1-E00-1-0001 | OH | A01 | a1 | B01 | b1 | C01 | c1 | D01 |

Compounds that may be contained in mixture 4-1-E00-1 are shown in the following Table 10-8-3.

[Table 547]

| [Table 10-8-3] Compound that may be contained in mixture 4-1-E00-1 | | | | | | | | | |
|---|---|---|---|---|---|---|---|---|---|
| ID | Exact Mass | | A | a | B | b | C | c | D |
| 4-1-E00-1-0001 | 530.19 | OH | A01 | a1 | B01 | b1 | C01 | c1 | D01 |
| 4-1-E00-1-0002 | 530.19 | OH | A02 | a1 | B01 | b1 | C01 | c1 | D01 |
| 4-1-E00-1-0003 | 544.20 | OH | A01 | a1 | B02 | b1 | C01 | c1 | D01 |
| 4-1-E00-1-0004 | 544.20 | OH | A02 | a1 | B02 | b1 | C01 | c1 | D01 |
| 4-1-E00-1-0005 | 548.18 | OH | A01 | a1 | B03 | b1 | C01 | c1 | D01 |
| 4-1-E00-1-0006 | 548.18 | OH | A02 | a1 | B03 | b1 | C01 | c1 | D01 |
| 4-1-E00-1-0007 | 558.22 | OH | A01 | a1 | B04 | b1 | C01 | c1 | D01 |
| 4-1-E00-1-0008 | 558.22 | OH | A02 | a1 | B04 | b1 | C01 | c1 | D01 |
| 4-1-E00-1-0009 | 530.19 | OH | A01 | a1 | B01 | b1 | C02 | c1 | D01 |
| 4-1-E00-1-0010 | 530.19 | OH | A02 | a1 | B01 | b1 | C02 | c1 | D01 |
| 4-1-E00-1-0011 | 544.20 | OH | A01 | a1 | B02 | b1 | C02 | c1 | D01 |
| 4-1-E00-1-0012 | 544.20 | OH | A02 | a1 | B02 | b1 | C02 | c1 | D01 |
| 4-1-E00-1-0013 | 548.18 | OH | A01 | a1 | B03 | b1 | C02 | c1 | D01 |
| 4-1-E00-1-0014 | 548.18 | OH | A02 | a1 | B03 | b1 | C02 | c1 | D01 |
| 4-1-E00-1-0015 | 558.22 | OH | A01 | a1 | B04 | b1 | C02 | c1 | D01 |
| 4-1-E00-1-0016 | 558.22 | OH | A02 | a1 | B04 | b1 | C02 | c1 | D01 |
| 4-1-E00-1-0017 | 534.16 | OH | A01 | a1 | B01 | b1 | C03 | c1 | D01 |
| 4-1-E00-1-0018 | 534.16 | OH | A02 | a1 | B01 | b1 | C03 | c1 | D01 |
| 4-1-E00-1-0019 | 548.18 | OH | A01 | a1 | B02 | b1 | C03 | c1 | D01 |
| 4-1-E00-1-0020 | 548.18 | OH | A02 | a1 | B02 | b1 | C03 | c1 | D01 |
| 4-1-E00-1-0021 | 552.15 | OH | A01 | a1 | B03 | b1 | C03 | c1 | D01 |
| 4-1-E00-1-0022 | 552.15 | OH | A02 | a1 | B03 | b1 | C03 | c1 | D01 |
| 4-1-E00-1-0023 | 562.19 | OH | A01 | a1 | B04 | b1 | C03 | c1 | D01 |
| 4-1-E00-1-0024 | 562.19 | OH | A02 | a1 | B04 | b1 | C03 | c1 | D01 |
| 4-1-E00-1-0025 | 544.20 | OH | A01 | a1 | B01 | b1 | C04 | c1 | D01 |
| 4-1-E00-1-0026 | 544.20 | OH | A02 | a1 | B01 | b1 | C04 | c1 | D01 |
| 4-1-E00-1-0027 | 558.22 | OH | A01 | a1 | B02 | b1 | C04 | c1 | D01 |
| 4-1-E00-1-0028 | 558.22 | OH | A02 | a1 | B02 | b1 | C04 | c1 | D01 |
| 4-1-E00-1-0029 | 562.19 | OH | A01 | a1 | B03 | b1 | C04 | c1 | D01 |
| 4-1-E00-1-0030 | 562.19 | OH | A02 | a1 | B03 | b1 | C04 | c1 | D01 |

(continued)

| [Table 10-8-3] Compound that may be contained in mixture 4-1-E00-1 | | | | | | | | | |
|---|---|---|---|---|---|---|---|---|---|
| ID | Exact Mass | | A | a | B | b | C | c | D |
| 4-1-E00-1-0031 | 572.23 | OH | A01 | a1 | B04 | b1 | C04 | c1 | D01 |
| 4-1-E00-1-0032 | 572.23 | OH | A02 | a1 | B04 | b1 | C04 | c1 | D01 |
| 4-1-E00-1-0033 | 614.12 | OH | A01 | a1 | B01 | b1 | C01 | c1 | D09 |
| 4-1-E00-1-0034 | 614.12 | OH | A02 | a1 | B01 | b1 | C01 | c1 | D09 |
| 4-1-E00-1-0035 | 628.13 | OH | A01 | a1 | B02 | b1 | C01 | c1 | D09 |
| 4-1-E00-1-0036 | 628.13 | OH | A02 | a1 | B02 | b1 | C01 | c1 | D09 |
| 4-1-E00-1-0037 | 632.11 | OH | A01 | a1 | B03 | b1 | C01 | c1 | D09 |
| 4-1-E00-1-0038 | 632.11 | OH | A02 | a1 | B03 | b1 | C01 | c1 | D09 |
| 4-1-E00-1-0039 | 642.15 | OH | A01 | a1 | B04 | b1 | C01 | c1 | D09 |
| 4-1-E00-1-0040 | 642.15 | OH | A02 | a1 | B04 | b1 | C01 | c1 | D09 |
| 4-1-E00-1-0041 | 614.12 | OH | A01 | a1 | B01 | b1 | C02 | c1 | D09 |
| 4-1-E00-1-0042 | 614.12 | OH | A02 | a1 | B01 | b1 | C02 | c1 | D09 |
| 4-1-E00-1-0043 | 628.13 | OH | A01 | a1 | B02 | b1 | C02 | c1 | D09 |
| 4-1-E00-1-0044 | 628.13 | OH | A02 | a1 | B02 | b1 | C02 | c1 | D09 |
| 4-1-E00-1-0045 | 632.11 | OH | A01 | a1 | B03 | b1 | C02 | c1 | D09 |
| 4-1-E00-1-0046 | 632.11 | OH | A02 | a1 | B03 | b1 | C02 | c1 | D09 |
| 4-1-E00-1-0047 | 642.15 | OH | A01 | a1 | B04 | b1 | C02 | c1 | D09 |
| 4-1-E00-1-0048 | 642.15 | OH | A02 | a1 | B04 | b1 | C02 | c1 | D09 |
| 4-1-E00-1-0049 | 618.09 | OH | A01 | a1 | B01 | b1 | C03 | c1 | D09 |
| 4-1-E00-1-0050 | 618.09 | OH | A02 | a1 | B01 | b1 | C03 | c1 | D09 |
| 4-1-E00-1-0051 | 632.11 | OH | A01 | a1 | B02 | b1 | C03 | c1 | D09 |
| 4-1-E00-1-0052 | 632.11 | OH | A02 | a1 | B02 | b1 | C03 | c1 | D09 |
| 4-1-E00-1-0053 | 636.08 | OH | A01 | a1 | B03 | b1 | C03 | c1 | D09 |
| 4-1-E00-1-0054 | 636.08 | OH | A02 | a1 | B03 | b1 | C03 | c1 | D09 |
| 4-1-E00-1-0055 | 646.12 | OH | A01 | a1 | B04 | b1 | C03 | c1 | D09 |
| 4-1-E00-1-0056 | 646.12 | OH | A02 | a1 | B04 | b1 | C03 | c1 | D09 |
| 4-1-E00-1-0057 | 628.13 | OH | A01 | a1 | B01 | b1 | C04 | c1 | D09 |
| 4-1-E00-1-0058 | 628.13 | OH | A02 | a1 | B01 | b1 | C04 | c1 | D09 |
| 4-1-E00-1-0059 | 642.15 | OH | A01 | a1 | B02 | b1 | C04 | c1 | D09 |
| 4-1-E00-1-0060 | 642.15 | OH | A02 | a1 | B02 | b1 | C04 | c1 | D09 |
| 4-1-E00-1-0061 | 646.12 | OH | A01 | a1 | B03 | b1 | C04 | c1 | D09 |
| 4-1-E00-1-0062 | 646.12 | OH | A02 | a1 | B03 | b1 | C04 | c1 | D09 |
| 4-1-E00-1-0063 | 656.17 | OH | A01 | a1 | B04 | b1 | C04 | c1 | D09 |
| 4-1-E00-1-0064 | 656.17 | OH | A02 | a1 | B04 | b1 | C04 | c1 | D09 |
| 4-1-E00-1-0065 | 622.18 | OH | A01 | a1 | B01 | b1 | C01 | c1 | D10 |
| 4-1-E00-1-0066 | 622.18 | OH | A02 | a1 | B01 | b1 | C01 | c1 | D10 |
| 4-1-E00-1-0067 | 636.19 | OH | A01 | a1 | B02 | b1 | C01 | c1 | D10 |
| 4-1-E00-1-0068 | 636.19 | OH | A02 | a1 | B02 | b1 | C01 | c1 | D10 |

(continued)

| [Table 10-8-3] Compound that may be contained in mixture 4-1-E00-1 | | | | | | | | | |
|---|---|---|---|---|---|---|---|---|---|
| ID | Exact Mass | | A | a | B | b | C | c | D |
| 4-1-E00-1-0069 | 640.17 | OH | A01 | a1 | B03 | b1 | C01 | c1 | D10 |
| 4-1-E00-1-0070 | 640.17 | OH | A02 | a1 | B03 | b1 | C01 | c1 | D10 |
| 4-1-E00-1-0071 | 650.21 | OH | A01 | a1 | B04 | b1 | C01 | c1 | D10 |
| 4-1-E00-1-0072 | 650.21 | OH | A02 | a1 | B04 | b1 | C01 | c1 | D10 |
| 4-1-E00-1-0073 | 622.18 | OH | A01 | a1 | B01 | b1 | C02 | c1 | D10 |
| 4-1-E00-1-0074 | 622.18 | OH | A02 | a1 | B01 | b1 | C02 | c1 | D10 |
| 4-1-E00-1-0075 | 636.19 | OH | A01 | a1 | B02 | b1 | C02 | c1 | D10 |
| 4-1-E00-1-0076 | 636.19 | OH | A02 | a1 | B02 | b1 | C02 | c1 | D10 |
| 4-1-E00-1-0077 | 640.17 | OH | A01 | a1 | B03 | b1 | C02 | c1 | D10 |
| 4-1-E00-1-0078 | 640.17 | OH | A02 | a1 | B03 | b1 | C02 | c1 | D10 |
| 4-1-E00-1-0079 | 650.21 | OH | A01 | a1 | B04 | b1 | C02 | c1 | D10 |
| 4-1-E00-1-0080 | 650.21 | OH | A02 | a1 | B04 | b1 | C02 | c1 | D10 |
| 4-1-E00-1-0081 | 626.15 | OH | A01 | a1 | B01 | b1 | C03 | c1 | D10 |
| 4-1-E00-1-0082 | 626.15 | OH | A02 | a1 | B01 | b1 | C03 | c1 | D10 |
| 4-1-E00-1-0083 | 640.17 | OH | A01 | a1 | B02 | b1 | C03 | c1 | D10 |
| 4-1-E00-1-0084 | 640.17 | OH | A02 | a1 | B02 | b1 | C03 | c1 | D10 |
| 4-1-E00-1-0085 | 644.14 | OH | A01 | a1 | B03 | b1 | C03 | c1 | D10 |
| 4-1-E00-1-0086 | 644.14 | OH | A02 | a1 | B03 | b1 | C03 | c1 | D10 |
| 4-1-E00-1-0087 | 654.18 | OH | A01 | a1 | B04 | b1 | C03 | c1 | D10 |
| 4-1-E00-1-0088 | 654.18 | OH | A02 | a1 | B04 | b1 | C03 | c1 | D10 |
| 4-1-E00-1-0089 | 636.19 | OH | A01 | a1 | B01 | b1 | C04 | c1 | D10 |
| 4-1-E00-1-0090 | 636.19 | OH | A02 | a1 | B01 | b1 | C04 | c1 | D10 |
| 4-1-E00-1-0091 | 650.21 | OH | A01 | a1 | B02 | b1 | C04 | c1 | D10 |
| 4-1-E00-1-0092 | 650.21 | OH | A02 | a1 | B02 | b1 | C04 | c1 | D10 |
| 4-1-E00-1-0093 | 654.18 | OH | A01 | a1 | B03 | b1 | C04 | c1 | D10 |
| 4-1-E00-1-0094 | 654.18 | OH | A02 | a1 | B03 | b1 | C04 | c1 | D10 |
| 4-1-E00-1-0095 | 664.22 | OH | A01 | a1 | B04 | b1 | C04 | c1 | D10 |
| 4-1-E00-1-0096 | 664.22 | OH | A02 | a1 | B04 | b1 | C04 | c1 | D10 |
| 4-1-E00-1-0097 | 638.17 | OH | A01 | a1 | B01 | b1 | C01 | c1 | D11 |
| 4-1-E00-1-0098 | 638.17 | OH | A02 | a1 | B01 | b1 | C01 | c1 | D11 |
| 4-1-E00-1-0099 | 652.19 | OH | A01 | a1 | B02 | b1 | C01 | c1 | D11 |
| 4-1-E00-1-0100 | 652.19 | OH | A02 | a1 | B02 | b1 | C01 | c1 | D11 |
| 4-1-E00-1-0101 | 656.16 | OH | A01 | a1 | B03 | b1 | C01 | c1 | D11 |
| 4-1-E00-1-0102 | 656.16 | OH | A02 | a1 | B03 | b1 | C01 | c1 | D11 |
| 4-1-E00-1-0103 | 666.20 | OH | A01 | a1 | B04 | b1 | C01 | c1 | D11 |
| 4-1-E00-1-0104 | 666.20 | OH | A02 | a1 | B04 | b1 | C01 | c1 | D11 |
| 4-1-E00-1-0105 | 638.17 | OH | A01 | a1 | B01 | b1 | C02 | c1 | D11 |

(continued)

| [Table 10-8-3] Compound that may be contained in mixture 4-1-E00-1 | | | | | | | | | |
|---|---|---|---|---|---|---|---|---|---|
| ID | Exact Mass | | A | a | B | b | C | c | D |
| 4-1-E00-1-0106 | 638.17 | OH | A02 | a1 | B01 | b1 | C02 | c1 | D11 |
| 4-1-E00-1-0107 | 652.19 | OH | A01 | a1 | B02 | b1 | C02 | c1 | D11 |
| 4-1-E00-1-0108 | 652.19 | OH | A02 | a1 | B02 | b1 | C02 | c1 | D11 |
| 4-1-E00-1-0109 | 656.16 | OH | A01 | a1 | B03 | b1 | C02 | c1 | D11 |
| 4-1-E00-1-0110 | 656.16 | OH | A02 | a1 | B03 | b1 | C02 | c1 | D11 |
| 4-1-E00-1-0111 | 666.20 | OH | A01 | a1 | B04 | b1 | C02 | c1 | D11 |
| 4-1-E00-1-0112 | 666.20 | OH | A02 | a1 | B04 | b1 | C02 | c1 | D11 |
| 4-1-E00-1-0113 | 642.15 | OH | A01 | a1 | B01 | b1 | C03 | c1 | D11 |
| 4-1-E00-1-0114 | 642.15 | OH | A02 | a1 | B01 | b1 | C03 | c1 | D11 |
| 4-1-E00-1-0115 | 656.16 | OH | A01 | a1 | B02 | b1 | C03 | c1 | D11 |
| 4-1-E00-1-0116 | 656.16 | OH | A02 | a1 | B02 | b1 | C03 | c1 | D11 |
| 4-1-E00-1-0117 | 660.14 | OH | A01 | a1 | B03 | b1 | C03 | c1 | D11 |
| 4-1-E00-1-0118 | 660.14 | OH | A02 | a1 | B03 | b1 | C03 | c1 | D11 |
| 4-1-E00-1-0119 | 670.18 | OH | A01 | a1 | B04 | b1 | C03 | c1 | D11 |
| 4-1-E00-1-0120 | 670.18 | OH | A02 | a1 | B04 | b1 | C03 | c1 | D11 |
| 4-1-E00-1-0121 | 652.19 | OH | A01 | a1 | B01 | b1 | C04 | c1 | D11 |
| 4-1-E00-1-0122 | 652.19 | OH | A02 | a1 | B01 | b1 | C04 | c1 | D11 |
| 4-1-E00-1-0123 | 666.20 | OH | A01 | a1 | B02 | b1 | C04 | c1 | D11 |
| 4-1-E00-1-0124 | 666.20 | OH | A02 | a1 | B02 | b1 | C04 | c1 | D11 |
| 4-1-E00-1-0125 | 670.18 | OH | A01 | a1 | B03 | b1 | C04 | c1 | D11 |
| 4-1-E00-1-0126 | 670.18 | OH | A02 | a1 | B03 | b1 | C04 | c1 | D11 |
| 4-1-E00-1-0127 | 680.22 | OH | A01 | a1 | B04 | b1 | C04 | c1 | D11 |
| 4-1-E00-1-0128 | 680.22 | OH | A02 | a1 | B04 | b1 | C04 | c1 | D11 |

Next, library case 2 shows a synthesis example of a library in which the first linking moiety (linker) (a1) is constituted by an amide bond, the second linker (b2) is constituted by a C-N bond through amide coupling, and the third linker (c2) is constituted by an ester bond.

Example 10-9: Formation of linker a1 by step a1 in case 2

A reaction formula in step a1B05 performed using BB4-2-01 will be illustrated below.

[Formula 716]

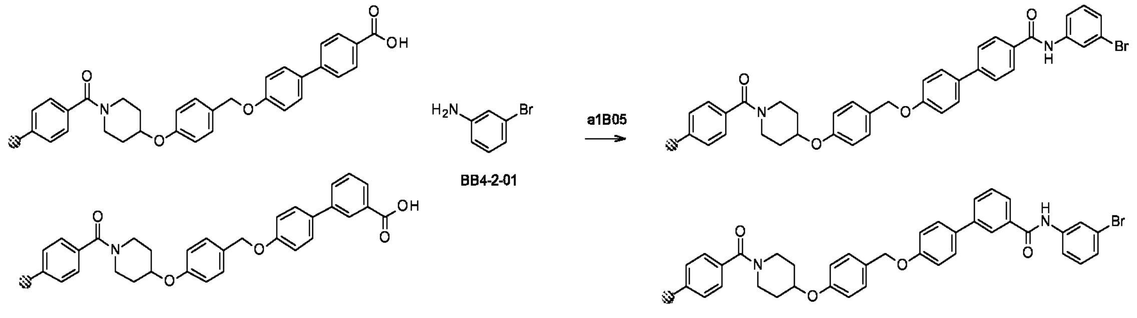


BB4-2-01

4-2-A00-0

4-2-a1B05-0

Mixture 4-2-a1B05-0 was synthesized as follows.

Under a nitrogen atmosphere, compound C005-03R (155 mg, 0.201 mmol/g), compound C003-03R (155 mg, 0.201 mmol/g), and DCM (4.5 mL) were added to an 8 mL glass vial, and the mixture was shaken at room temperature for 1 hour to prepare mixture 4-2- A00-0. A mixed solution of 3-bromoaniline (BB-4-2-01) (26.3 μL, 0.242 mmol), PipClU (chlorodipiperidinocarbenium hexafluorophosphate) (44 mg, 0.122 mmol) prepared at 1.0 M with acetonitrile (120 μL), and NMI (1-methylimidazole) (9.6 μL, 0.120 mmol) was added thereto, and the mixture was shaken at 25°C for 7 hours.

Solid-phase purification

The suspension of the reaction solution and the solid phase was wholly transferred onto the filter of a column and washed three times with NMP (6 mL), three times with NMP- water (1:1, 6 mL), three times with NMP (6 mL), three times with methanol (6 mL), three times with DCM (6 mL), and three times with heptane (6 mL), and the obtained solid phase was dried overnight under reduced pressure to obtain mixture 4-2-a1B05-0 (amount supported: 0.195 mmol/g, 0.320 g).

Steps a1B06 and a1B08 were performed in the same manner as in the synthesis of mixture 4-2-a1B05-0 using BB4-2-02 and -04 to obtain mixture 4-2-a1B06-0 (amount supported: 0.194 mmol/g, 0.352 g) and mixture 4-2-a1B08-0 (amount supported: 0.194 mmol/g, 0.370 g). These mixtures can also be treated by cleavage in the same manner as in Example 10-1 to obtain mixtures 4-2-a1B06-1 and 4-2-a1B08-1.

Next, a reaction formula in step a1B07 performed using BB4-2-03 will be illustrated below.

[Formula 717]

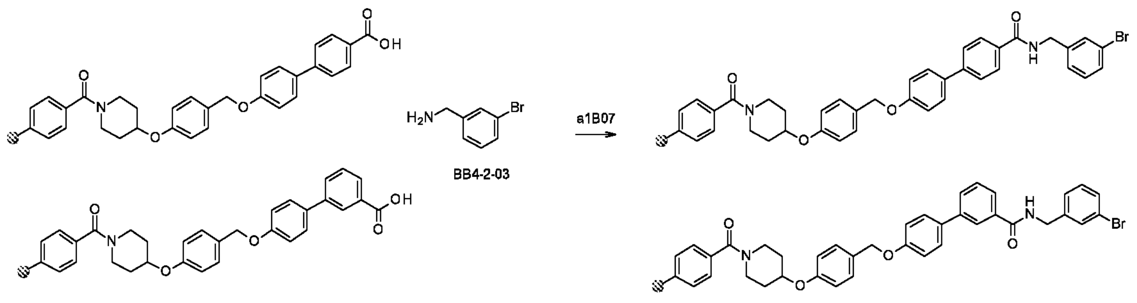


BB4-2-03

4-2-A00-0

4-2-a1B07-0

Mixture 4-2-a1B07-0 was synthesized as follows.

Under a nitrogen atmosphere, compound C005-03R (160 mg, 0.201 mmol/g), mixture C003-03R (160 mg, 0.201 mmol/g), and NMP (4.5 mL) were added to an 8 mL glass vial and shaken at room temperature for 1 hour. (3-Bromophenyl)methanamine (BB-4-2-03) (56 mg, 0.302 mmol), HOAt (41 mg, 0.302 mmol), and DIC (47 μL, 0.302 mmol) were added thereto, and the mixture was shaken at room temperature for 14 hours.

Solid-phase purification

The suspension of the reaction solution and the solid phase was wholly transferred onto the filter of a column and washed three times with NMP (6 mL), three times with NMP- water (1:1, 6 mL), three times with NMP (6 mL), three times with methanol (6 mL), three times with DCM (6 mL), and three times with heptane (6 mL), and the obtained solid

phase was dried overnight under reduced pressure to obtain mixture 4-2-a1B07-0 (amount supported: 0.194 mmol/g, 0.330 g).

The reagents used in step a1 and the obtained mixtures are shown in Table 10-9-1.

[Table 548]

| [Table 10-9-1] Reagent used and obtained mixture in step a1 | | | | | | | |
|---|---|---|---|---|---|---|---|
| Starting material used | Amount used | Amine reagent used | Amount used | Molar number | Obtained mixture | Yield | Amount supported |
| C005-03R /C003-03R | 155 mg /155 mg | BB4-2-01 | 26.3 μL | 0.242 mmol | 4-2-alB05-0 | 320.4 mg | 0.195 mmol/g |
| C005-03R /C003-03R | 170 mg /170 mg | BB4-2-02 | 45 mg | 0.244 mmol | 4-2-alB06-0 | 352.1 mg | 0.194 mmol/g |
| C005-03R /C003-03R | 160 mg /160 mg | BB4-2-03 | 56 mg | 0.302 mmol | 4-2-alB07-0 | 329.8 mg | 0.194 mmol/g |
| C005-03R /C003-03R | 175 mg /175 mg | BB4-2-04 | 49 mg | 0.245 mmol | 4-2-alB08-0 | 369.5 mg | 0.194 mmol/g |

The details of the mixtures are shown in Table 10-9-3 to Table 10-9-6.

Mixtures 4-2-a1B05-0 to 4-2-a1B08-0 can be treated by cleavage in the same manner as in Example 10-1 to obtain mixtures 4-2-a1B05-1 to 4-2-a1B08-1.

A notation method in each table will be described. For example, in Table 10-9-3, each compound that may be contained in 4-2-a1B05-1 is indicated by ID, and core blocks and linkers in combination in the structure of mixture 4-2-a1B05-1 are indicated by symbols or chemical formulas. The correspondence between the symbols and specific structures are shown in Figure 9. In Table 10-9-2 to Table 10-9-6, "×" and "b" are represented by chemical formulas.

[Formula 718]

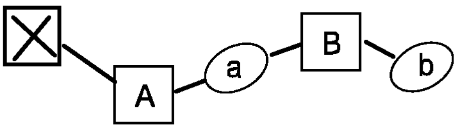

For example, when ID is 4-2-a1B05-1-0001, the compound is represented by the following structural formula.

[Formula 719]

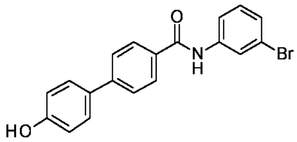

4-2-a1B05-1-0001

[Table 549]

| [Table 10-9-2] Structural notation in ID (4-2-a1B05-1-0001) | | | | | | |
|---|---|---|---|---|---|---|
| ID | | A | a | B | b | b |
| 4-2-a1B05-1-0001 | OH | A01 | a1 | B05 | Br | Br |

The mixture synthesized in step a1B05 using BB4-2-01 is shown in Table 10-9-3.

[Table 550]

| [Table 10-9-3] Compound contained in mixture (4-2-a1B05-0) synthesized in step a1B05 using BB4-2-01 | | | | | |
|---|---|---|---|---|---|
| ID | | A | a | B | b |
| 4-2-a1B05-1-0001 | OH | A01 | a1 | B05 | Br |
| 4-2-a1B05-1-0002 | OH | A02 | a1 | B05 | Br |

The mixture synthesized in step a1B06 using BB4-2-02 is shown in Table 10-9-4.

[Table 551]

| [Table 10-9-4] Compound contained in mixture (4-2-a1B06-0) synthesized in step a1B06 using BB4-2-02 | | | | | |
|---|---|---|---|---|---|
| ID | | A | a | B | b |
| 4-2-a1B06-1-0001 | OH | A01 | a1 | B06 | Br |
| 4-2-a1B06-1-0002 | OH | A02 | a1 | B06 | Br |

The mixture synthesized in step a1B07 using BB4-2-03 is shown in Table 10-9-5.

[Table 552]

| [Table 10-9-5] Compound contained in mixture (4-2-a1B07-0) synthesized in step a1B07 using BB4-2-03 | | | | | |
|---|---|---|---|---|---|
| ID | | A | a | B | b |
| 4-2-a1B07-1-0001 | OH | A01 | a1 | B07 | Br |
| 4-2-a1B07-1-0002 | OH | A02 | a1 | B07 | Br |

The mixture synthesized in step a1B08 using BB4-2-04 is shown in Table 10-9-6.

[Table 553]

| [Table 10-9-6] Compound contained in mixture (4-2-a1B08-0) synthesized in step a1B08 using BB4-2-04 | | | | | |
|---|---|---|---|---|---|
| ID | | A | a | B | b |
| 4-2-a1B08-1-0001 | OH | A01 | a1 | B08 | Br |
| 4-2-a1B08-1-0002 | OH | A02 | a1 | B08 | Br |

Example 10-10: Solid-phase mixing of mixtures obtained in step a1 in case 2

[Formula 720]

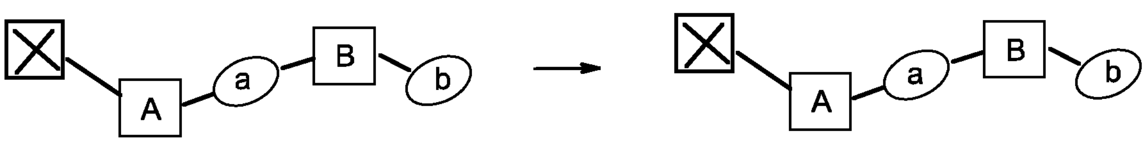

4-2-a1B05-0~4-2-a1B08-0

4-2-C00-0

4 types of mixtures described in Table 10-10-1 were added to a 10 mL column with a filter and washed twice

with DMF (8 mL), three times with DCM (8 mL), and three times with heptane (8 mL), and the obtained solid phase was dried under reduced pressure to obtain mixture 4-2-C00-0 (910 mg, 0.194 mmol/g).

[Table 554]

| [Table 10-10-1] Obtained mixture and amount used in step a1 | |
|---|---|
| Mixture | Amount used |
| 4-2-a1B05-0 | 221 mg |
| 4-2-a1B06-0 | 225 mg |
| 4-2-a1B07-0 | 235 mg |
| 4-2-a1B08-0 | 234 mg |

Mixture 4-2-C00-0 can be treated by cleavage in the same manner as in Example 10- 1 to obtain mixture 4-2-C00-1. The details of the mixture are shown in Table 10-10-3.

A notation method in each table will be described. For example, in Table 10-10-3, each compound that may be contained in 4-2-C00-1 is indicated by ID, and core blocks and linkers in combination in the structure of mixture 4-2-C00-1 are indicated by symbols or chemical formulas. The correspondence between the symbols and specific structures are shown in Figure 9.

In Table 10-10-2 and Table 10-10-3, "×" and "b" are represented by chemical formulas.

[Formula 721]

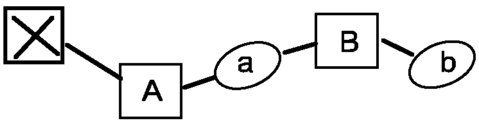


**4-2-C00-0**

For example, when ID is 4-2-C00-1-0001, the compound is represented by the following structural formula.

[Formula 722]

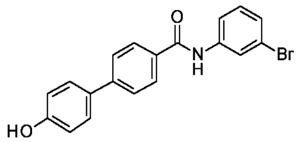

4-2-C00-1-0001

[Table 555]

| [Table 10-10-2] Structural notation in ID (4-2-C00-1-0001) | | | | | |
|---|---|---|---|---|---|
| ID | | A | a | B | b |
| 4-2-C00-1-0001 | OH | A01 | a1 | B05 | Br |

The mixture obtained by solid-phase mixing is shown in Table 10-10-3.

[Table 556]

| [Table 10-10-3] Compound contained in mixture (4-2-C00-0) obtained by solid-phase mixing | | | | | |
|---|---|---|---|---|---|
| ID | | A | a | B | b |
| 4-2-C00-1-0001 | OH | A01 | a1 | B05 | Br |
| 4-2-C00-1-0002 | OH | A02 | a1 | B05 | Br |
| 4-2-C00-1-0003 | OH | A01 | a1 | B06 | Br |
| 4-2-C00-1-0004 | OH | A02 | a1 | B06 | Br |
| 4-2-C00-1-0005 | OH | A01 | a1 | B07 | Br |
| 4-2-C00-1-0006 | OH | A02 | a1 | B07 | Br |
| 4-2-C00-1-0007 | OH | A01 | a1 | B08 | Br |
| 4-2-C00-1-0008 | OH | A02 | a1 | B08 | Br |

Example 10-11: Formation of linker b2 by step b2 in case 2

A reaction formula in step b2 performed using BB4-2-05 to -08 will be illustrated below.

[Formula 723]

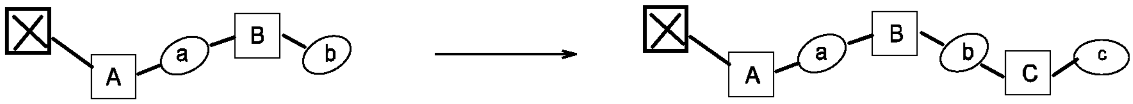


4-2-C00-0

4-2-b2C05-0 ~ 4-2-b2C08-0

Mixtures 4-2-b2C05-0 to 4-2-b2C08-0 were synthesized as follows. The following example shows step b2C05.

Under a nitrogen atmosphere, mixture 4-2-C00-0 (213 mg, 0.194 mmol/g) and THF (4.3 mL) were added to an 8 mL glass vial and shaken at room temperature for 1 hour. An amide reagent (BB4-2-05) (74.1 mg, 0.414 mmol) was added to the glass vial. tBuBrettphos Pd G4 (35.9 mg, 0.041 mmol) and MTBD (7-methyl-1,5,7-triazabicyclo[4.4.0]dec-5-ene) (89 μL, 0.621 mmol) were added thereto, and the mixture was shaken at 60°C for 3 hours.

Solid-phase purification

The suspension of the reaction solution and the solid phase was transferred to a 6 mL column with a filter, filtered, and washed three times with NMP (4 mL), three times with NMP/water = 1/1 (4 mL), three times with 0.2 M NAC in NMP/water = 1/1 (4 mL), three times with 0.05 M $TBAHSO_4$ + DTBP in NMP (4 mL), three times with 0.05 M NMM in NMP (4 mL), three times with NMP/water = 1/1 (4 mL), three times with NMP (4 mL), three times with MeOH (4 mL), three times with DCM (4 mL), and three times with heptane (4 mL), and the obtained solid phase was dried under reduced pressure to obtain mixture 4-2- b2C05-0.

Step b2 was performed in the same manner as in the synthesis of mixture 4-2- b2C05-0 using BB4-2-06 to -08 instead of BB4-2-05 to obtain mixture 4-2-b2C06-0 to mixture 4-2-b2C08-0.

The amide reagents used in step b2 and the amounts of the reagents used are shown in Table 10-11-1. The details of the mixtures obtained in step b2 are shown in Table 10-11-3 to Table 10-11-6.

The reagents used in step b2 and the obtained mixtures are shown in Table 10-11-1.

[Table 557]

| [Table 10-11-1] Reagent used and obtained mixture in step b2 | | | | | | | |
|---|---|---|---|---|---|---|---|
| Starting material used | Amount used | Amide reagent used | Amount used | Molar number | Obtained mixture | Yield | Amount supported |
| 4-2-C00-0 | 213 mg | BB4-2-05 | 74.1 mg | 0.414 mmol | 4-2-b2C05-0 | 225.3 mg | 0.191 mmol/g |
| 4-2-C00-0 | 213 mg | BB4-2-06 | 74.7 mg | 0.415 mmol | 4-2-b2C06-0 | 224.7 mg | 0.191 mmol/g |
| 4-2-C00-0 | 208 mg | BB4-2-07 | 78.0 mg | 0.403 mmol | 4-2-b2C07-0 | 214.1 mg | 0.190 mmol/g |
| 4-2-C00-0 | 213 mg | BB4-2-08 | 59.3mg | 0.414 mmol | 4-2-b2C08-0 | 216.4 mg | 0.192 mmol/g |

Mixture 4-2-b2C05-0 can be treated by cleavage in the same manner as in Example 10-1 to obtain mixture 4-2-b2C05-1. The details of the mixtures are shown in Table 10-11-3 to Table 10-11-6.

A notation method in each table will be described. For example, in Table 10-11-3, each compound that may be contained in 4-2-b2C05-1 is indicated by ID, and core blocks and linkers in combination in the structure of mixture 4-2-b2C05-1 are indicated by symbols or chemical formulas. The correspondence between the symbols and specific structures are shown in Figure 9.

In Table 10-11-2 to Table 10-11-6, "×" and "b" are represented by chemical formulas.

[Formula 724]

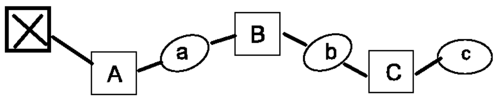


4-2-b2C05-0 ~ 4-2-b2C08-0

For example, when ID is 4-2-b2C05-1-0001, the compound is represented by the following structural formula.

[Formula 725]

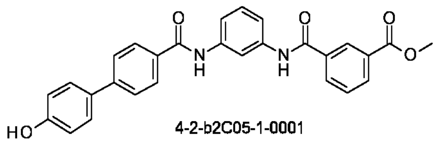

4-2-b2C05-1-0001

[Table 558]

| [Table 10-11-2] Structural notation in ID (4-2-b2C05-1-0001) | | | | | | | |
|---|---|---|---|---|---|---|---|
| ID | | A | a | B | b | C | c |
| 4-2-b2C05-1-0001 | OH | A01 | a1 | B05 | b2 | C05 | CO2Me |

The mixture synthesized in step b2C05 using BB4-2-05 is shown in Table 10-11-3.

[Table 559]

| [Table 10-11-3] Compound contained in mixture (4-2-b2C05-1) synthesized in step b2C05 using BB4-2-05 | | | | | | | | |
|---|---|---|---|---|---|---|---|---|
| ID | Exact Mass | | A | a | B | b | C | c |
| 4-2-b2C05-1-0001 | 466.15 | OH | A01 | a1 | B05 | b2 | C05 | CO2Me |

(continued)

| [Table 10-11-3] Compound contained in mixture (4-2-b2C05-1) synthesized in step b2C05 using BB4-2-05 | | | | | | | | |
|---|---|---|---|---|---|---|---|---|
| ID | Exact Mass | | A | a | B | b | C | c |
| 4-2-b2C05-1-0002 | 466.15 | OH | A02 | a1 | B05 | b2 | C05 | CO2Me |
| 4-2-b2C05-1-0003 | 480.17 | OH | A01 | a1 | B06 | b2 | C05 | CO2Me |
| 4-2-b2C05-1-0004 | 480.17 | OH | A02 | a1 | B06 | b2 | C05 | CO2Me |
| 4-2-b2C05-1-0005 | 480.17 | OH | A01 | a1 | B07 | b2 | C05 | CO2Me |
| 4-2-b2C05-1-0006 | 480.17 | OH | A02 | a1 | B07 | b2 | C05 | CO2Me |
| 4-2-b2C05-1-0007 | 496.16 | OH | A01 | a1 | B08 | b2 | C05 | CO2Me |
| 4-2-b2C05-1-0008 | 496.16 | OH | A02 | a1 | B08 | b2 | C05 | CO2Me |

The mixture synthesized in step b2C06 using BB4-2-06 is shown in Table 10-11-4.
[Table 560]

| [Table 10-11-4] Compound contained in mixture (4-2-b2C06-1) synthesized in step b2C06 using BB4-2-06 | | | | | | | | |
|---|---|---|---|---|---|---|---|---|
| ID | Exact Mass | | A | a | B | b | C | c |
| 4-2-b2C06-1-0001 | 467.15 | OH | A01 | a1 | B05 | b2 | C06 | CO2Me |
| 4-2-b2C06-1-0002 | 467.15 | OH | A02 | a1 | B05 | b2 | C06 | CO2Me |
| 4-2-b2C06-1-0003 | 481.16 | OH | A01 | a1 | B06 | b2 | C06 | CO2Me |
| 4-2-b2C06-1-0004 | 481.16 | OH | A02 | a1 | B06 | b2 | C06 | CO2Me |
| 4-2-b2C06-1-0005 | 481.16 | OH | A01 | a1 | B07 | b2 | C06 | CO2Me |
| 4-2-b2C06-1-0006 | 481.16 | OH | A02 | a1 | B07 | b2 | C06 | CO2Me |
| 4-2-b2C06-1-0007 | 497.16 | OH | A01 | a1 | B08 | b2 | C06 | CO2Me |
| 4-2-b2C06-1-0008 | 497.16 | OH | A02 | a1 | B08 | b2 | C06 | CO2Me |

The mixture synthesized in step b2C07 using BB4-2-07 is shown in Table 10-11-5.
[Table 561]

| [Table 10-11-5] Compound contained in mixture (4-2-b2C07-1) synthesized in step b2C07 using BB4-2-07 | | | | | | | | |
|---|---|---|---|---|---|---|---|---|
| ID | Exact Mass | | A | a | B | b | C | c |
| 4-2-b2C07-1-0001 | 480.17 | OH | A01 | a1 | B05 | b2 | C07 | CO2Me |
| 4-2-b2C07-1-0002 | 480.17 | OH | A02 | a1 | B05 | b2 | C07 | CO2Me |
| 4-2-b2C07-1-0003 | 494.18 | OH | A01 | a1 | B06 | b2 | C07 | CO2Me |
| 4-2-b2C07-1-0004 | 494.18 | OH | A02 | a1 | B06 | b2 | C07 | CO2Me |
| 4-2-b2C07-1-0005 | 494.18 | OH | A01 | a1 | B07 | b2 | C07 | CO2Me |
| 4-2-b2C07-1-0006 | 494.18 | OH | A02 | a1 | B07 | b2 | C07 | CO2Me |
| 4-2-b2C07-1-0007 | 510.18 | OH | A01 | a1 | B08 | b2 | C07 | CO2Me |
| 4-2-b2C07-1-0008 | 510.18 | OH | A02 | a1 | B08 | b2 | C07 | CO2Me |

The mixture synthesized in step b2C08 using BB4-2-08 is shown in Table 10-11-6.
[Table 562]

| [Table 10-11-6] Compound contained in mixture (4-2-b2C08-1) synthesized in step b2C08 using BB4-2-08 | | | | | | | | |
|---|---|---|---|---|---|---|---|---|
| ID | Exact Mass | | A | a | B | b | C | c |
| 4-2-b2C08-1-0001 | 430.15 | OH | A01 | a1 | B05 | b2 | C08 | CO2Me |
| 4-2-b2C08-1-0002 | 430.15 | OH | A02 | a1 | B05 | b2 | C08 | CO2Me |
| 4-2-b2C08-1-0003 | 444.17 | OH | A01 | a1 | B06 | b2 | C08 | CO2Me |
| 4-2-b2C08-1-0004 | 444.17 | OH | A02 | a1 | B06 | b2 | C08 | CO2Me |
| 4-2-b2C08-1-0005 | 444.17 | OH | A01 | a1 | B07 | b2 | C08 | CO2Me |
| 4-2-b2C08-1-0006 | 444.17 | OH | A02 | a1 | B07 | b2 | C08 | CO2Me |
| 4-2-b2C08-1-0007 | 460.16 | OH | A01 | a1 | B08 | b2 | C08 | CO2Me |
| 4-2-b2C08-1-0008 | 460.16 | OH | A02 | a1 | B08 | b2 | C08 | CO2Me |

Example 10-12: Synthesis of mixture 4-2-h04C05-0 to mixture 4-2-h07C08-0 by hydrolysis steps h04 to h07 in case 2

A reaction formula in steps h04 to h07 performed using mixtures 4-2-b2C05-0 to 4- 2-b2C08-0 will be illustrated below.

[Formula 726]

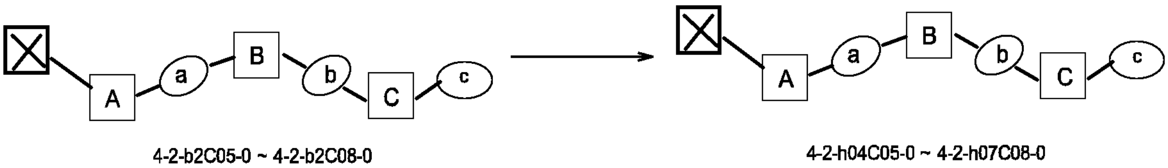


Mixtures 4-2-h04C05-0 to 4-2-h07C08-0 were synthesized as follows. The following example shows step h04C05.

Under a nitrogen atmosphere, mixture 4-2-b2C05-0 (144 mg, 0.191 mmol/g) was added to a 4 mL glass vial. NMP (2.0 mL) and tAmOH (575 μL) were added thereto, and the mixture was shaken at room temperature for 1 hour. A 1 M aqueous TBAOH (tetrabutylammonium hydroxide) solution (137 μL) was added thereto, and the mixture was shaken at room temperature for 6 hours.

Solid-phase purification

The suspension of the reaction solution and the solid phase was transferred to a 3 mL column with a filter, filtered, and washed three times with NMP (2 mL), three times with NMP/water = 1/1 (2 mL), three times with 0.05 M $TBAHSO_4$ + DTBP in NMP (2 mL), three times with NMP/water = 1/1 (2 mL), three times with NMP (2 mL), three times with MeOH (2 mL), three times with DCM (2 mL), and three times with heptane (2 mL), and the obtained solid phase was dried under reduced pressure to obtain mixture 4-2-h04C05-0.

Steps h05 to h07 were performed in the same manner as in the synthesis of mixture 4-2-h04C05-0 to obtain mixture 4-2-h05C06-0 to mixture 4-2-h07C08-0.

The mixtures used in steps h04 to h07 and the amounts of the solvents and the TBAOH solution used are shown in Table 10-12-1. The mixtures obtained in steps h04 to h07 are shown in Table 10-12-3 to Table 10-12-6.

The reagents used in steps h04 to h07 and the obtained mixtures are shown in Table 10-12-1.

[Table 563]

| [Table 10-12-1] Reagent used and obtained mixture in steps h04 to h07 | | | | | | | |
|---|---|---|---|---|---|---|---|
| Mixture used | Amount used | NMP | tAmOH | Amount of 1 M TBAOH aq. used | Obtained mixture | Yield | Amount supported |
| 4-2-b2C05-0 | 144.0 mg | 2013 μL | 575 μL | 137 μL (0.137 mmol) | 4-2-h04C05-0 | 143.3 mg | 0.191 mmol/g |
| 4-2-b2C06-0 | 150.0 mg | 2100 μL | 600 μL | 143 μL (0.143 mmol) | 4-2-h05C06-0 | 151.5mg | 0.191 mmol/g |
| 4-2-b2C07-0 | 143.0 mg | 2002 μL | 572 μL | 136 μL (0.136 mmol) | 4-2-h06C07-0 | 123.5 mg | 0.191 mmol/g |
| 4-2-b2C08-0 | 149.6 mg | 2094 μL | 598 μL | 144 μL (0.144 mmol) | 4-2-h07C08-0 | 145.3 mg | 0.193 mmol/g |

Mixture 4-2-h04C05-0 can be treated by cleavage in the same manner as in Example 10-1 to obtain mixture 4-2-h04C05-1. The details of the mixtures are shown in Table 10-12- 3 to Table 10-12-6.

A notation method in each table will be described. For example, in Table 10-12-3, each compound that may be contained in 4-2-h04C05-1 is indicated by ID, and core blocks and linkers in combination in the structure of mixture 4-2-h04C05-1 are indicated by symbols or chemical formulas. The correspondence between the symbols and specific structures are shown in Figure 9. In Table 10-12-2 to Table 10-12-6, "×" and "c" are represented by chemical formulas.

[Formula 727]

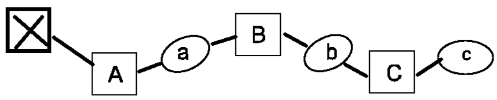


4-2-h04C05-0 ~ 4-2-h07C08-0

For example, when ID is 4-2-h04C05-1-0001, the compound is represented by the following structural formula.

[Formula 728]

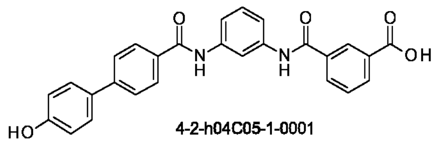

4-2-h04C05-1-0001

[Table 564]

| [Table 10-12-2] Structural notation in ID (4-2-h04C05-1-0001) | | | | | | | |
|---|---|---|---|---|---|---|---|
| ID | | A | a | B | b | C | c |
| 4-2-h04C05-1-0001 | OH | A01 | a1 | B05 | b2 | C05 | CO2H |

The mixture synthesized in step h04 from mixture 4-2-b2C05-0 is shown in Table 10-12-3.

[Table 565]

| [Table 10-12-3] Compound contained in mixture (4-2-h04C05-1) synthesized in step h04 from mixture 4-2-b2C05-0 | | | | | | | | |
|---|---|---|---|---|---|---|---|---|
| ID | Exact Mass | | A | a | B | b | C | c |
| 4-2-h04C05-1-0001 | 452.14 | OH | A01 | a1 | B05 | b2 | C05 | CO2H |
| 4-2-h04C05-1-0002 | 452.14 | OH | A02 | a1 | B05 | b2 | C05 | CO2H |
| 4-2-h04C05-1-0003 | 466.15 | OH | A01 | a1 | B06 | b2 | C05 | CO2H |
| 4-2-h04C05-1-0004 | 466.15 | OH | A02 | a1 | B06 | b2 | C05 | CO2H |
| 4-2-h04C05-1-0005 | 466.15 | OH | A01 | a1 | B07 | b2 | C05 | CO2H |
| 4-2-h04C05-1-0006 | 466.15 | OH | A02 | a1 | B07 | b2 | C05 | CO2H |
| 4-2-h04C05-1-0007 | 482.15 | OH | A01 | a1 | B08 | b2 | C05 | CO2H |
| 4-2-h04C05-1-0008 | 482.15 | OH | A02 | a1 | B08 | b2 | C05 | CO2H |

The mixture synthesized in step h05 from mixture 4-2-b2C06-0 is shown in Table 10-12-4.

[Table 566]

| [Table 10-12-4] Compound contained in mixture (4-2-h05C06-1) synthesized in step h05 from mixture 4-2-b2C06-0 | | | | | | | | |
|---|---|---|---|---|---|---|---|---|
| ID | Exact Mass | | A | a | B | b | C | c |
| 4-2-h05C06-1-0001 | 453.13 | OH | A01 | a1 | B05 | b2 | C06 | CO2H |
| 4-2-h05C06-1-0002 | 453.13 | OH | A02 | a1 | B05 | b2 | C06 | CO2H |
| 4-2-h05C06-1-0003 | 467.15 | OH | A01 | a1 | B06 | b2 | C06 | CO2H |
| 4-2-h05C06-1-0004 | 467.15 | OH | A02 | a1 | B06 | b2 | C06 | CO2H |
| 4-2-h05C06-1-0005 | 467.15 | OH | A01 | a1 | B07 | b2 | C06 | CO2H |
| 4-2-h05C06-1-0006 | 467.15 | OH | A02 | a1 | B07 | b2 | C06 | CO2H |
| 4-2-h05C06-1-0007 | 483.14 | OH | A01 | a1 | B08 | b2 | C06 | CO2H |
| 4-2-h05C06-1-0008 | 483.14 | OH | A02 | a1 | B08 | b2 | C06 | CO2H |

The mixture synthesized in step h06 from mixture 4-2-b2C07-0 is shown in Table 10-12-5.

[Table 567]

| [Table 10-12-5] Compound contained in mixture (4-2-h06C07-1) synthesized in step h06 from mixture 4-2-b2C07-0 | | | | | | | | |
|---|---|---|---|---|---|---|---|---|
| ID | Exact Mass | | A | a | B | b | C | c |
| 4-2-h06C07-1-0001 | 466.15 | OH | A01 | a1 | B05 | b2 | C07 | CO2H |
| 4-2-h06C07-1-0002 | 466.15 | OH | A02 | a1 | B05 | b2 | C07 | CO2H |
| 4-2-h06C07-1-0003 | 480.17 | OH | A01 | a1 | B06 | b2 | C07 | CO2H |
| 4-2-h06C07-1-0004 | 480.17 | OH | A02 | a1 | B06 | b2 | C07 | CO2H |
| 4-2-h06C07-1-0005 | 480.17 | OH | A01 | a1 | B07 | b2 | C07 | CO2H |
| 4-2-h06C07-1-0006 | 480.17 | OH | A02 | a1 | B07 | b2 | C07 | CO2H |
| 4-2-h06C07-1-0007 | 496.16 | OH | A01 | a1 | B08 | b2 | C07 | CO2H |
| 4-2-h06C07-1-0008 | 496.16 | OH | A02 | a1 | B08 | b2 | C07 | CO2H |

The mixture synthesized in step h07 from mixture 4-2-b2C08-0 is shown in Table 10-12-6.
[Table 568]

| [Table 10-12-6] Compound contained in mixture (4-2-h07C08-1) synthesized in step h07 from mixture 4-2-b2C08-0 | | | | | | | | |
|---|---|---|---|---|---|---|---|---|
| ID | Exact Mass | | A | a | B | b | C | c |
| 4-2-h07C08-1-0001 | 416.14 | OH | A01 | a1 | B05 | b2 | C08 | CO2H |
| 4-2-h07C08-1-0002 | 416.14 | OH | A02 | a1 | B05 | b2 | C08 | CO2H |
| 4-2-h07C08-1-0003 | 430.15 | OH | A01 | a1 | B06 | b2 | C08 | CO2H |
| 4-2-h07C08-1-0004 | 430.15 | OH | A02 | a1 | B06 | b2 | C08 | CO2H |
| 4-2-h07C08-1-0005 | 430.15 | OH | A01 | a1 | B07 | b2 | C08 | CO2H |
| 4-2-h07C08-1-0006 | 430.15 | OH | A02 | a1 | B07 | b2 | C08 | CO2H |
| 4-2-h07C08-1-0007 | 446.15 | OH | A01 | a1 | B08 | b2 | C08 | CO2H |
| 4-2-h07C08-1-0008 | 446.15 | OH | A02 | a1 | B08 | b2 | C08 | CO2H |

Example 10-13: Solid-phase mixing of mixtures obtained in steps h04 to O7 in case 2

[Formula 729]

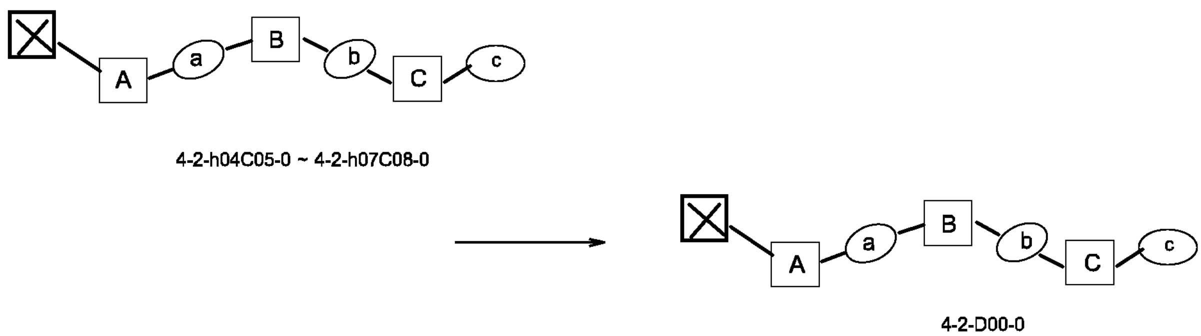


4 types of mixtures described in Table 10-13-1 were added to a 6 mL column with a filter and washed three times with DCM (5 mL) and three times with heptane (5 mL), and the obtained solid phase was dried under reduced pressure to obtain mixture 4-2-D00-0 (525 mg, 0.192 mmol/g).
[Table 569]

| [Table 10-13-1] Amount of mixture used | |
|---|---|
| Mixture | Amount used |
| 4-2-h04C05-0 | 134.5 mg |
| 4-2-h05C06-0 | 142.5 mg |
| 4-2-h06C07-0 | 110.1 mg |
| 4-2-h07C08-0 | 137.7 mg |

Mixture 4-2-D00-0 can be treated by cleavage in the same manner as in Example 10-1 to obtain mixture 4-2-D00-1. The details of the mixture are shown in Table 10-13-3.

A notation method in each table will be described. For example, in Table 10-13-3, each compound that may be contained in 4-2-D00-1 is indicated by ID, and core blocks and linkers in combination in the structure of mixture 4-2-D00-1 are indicated by symbols or chemical formulas. The correspondence between the symbols and specific structures are shown in Figure 9. In Table 10-13-2 and Table 10-13-3, "×" and "c" are represented by chemical formulas.

[Formula 730]

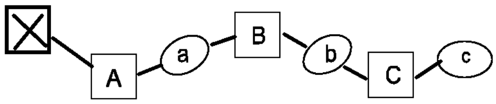

4-2-D00-0

For example, when ID is 4-2-D00-1-0001, the compound is represented by the following structural formula.

[Formula 731]

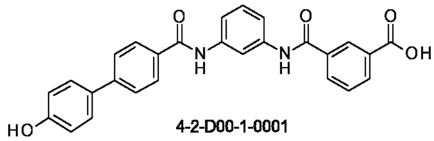

4-2-D00-1-0001

[Table 570]

| [Table 10-13-2] Structural notation in ID (4-2-D00-1-0001) | | | | | | | |
|---|---|---|---|---|---|---|---|
| ID | | A | a | B | b | C | c |
| 4-2-D00-1-0001 | OH | A01 | a1 | B05 | b2 | C05 | CO2H |

The mixture obtained by solid-phase mixing is shown in the following Table 10-13- 3.

[Table 571]

| [Table 10-13-3] Compound contained in mixture (4-2-D00-1) obtained by solid-phase mixing | | | | | | | | |
|---|---|---|---|---|---|---|---|---|
| ID | Exact Mass | | A | a | B | b | C | c |
| 4-2-D00-1-0001 | 452.14 | OH | A01 | a1 | B05 | b2 | C05 | CO2H |
| 4-2-D00-1-0002 | 452.14 | OH | A02 | a1 | B05 | b2 | C05 | CO2H |
| 4-2-D00-1-0003 | 466.15 | OH | A01 | a1 | B06 | b2 | C05 | CO2H |
| 4-2-D00-1-0004 | 466.15 | OH | A02 | a1 | B06 | b2 | C05 | CO2H |
| 4-2-D00-1-0005 | 466.15 | OH | A01 | a1 | B07 | b2 | C05 | CO2H |
| 4-2-D00-1-0006 | 466.15 | OH | A02 | a1 | B07 | b2 | C05 | CO2H |
| 4-2-D00-1-0007 | 482.15 | OH | A01 | a1 | B08 | b2 | C05 | CO2H |
| 4-2-D00-1-0008 | 482.15 | OH | A02 | a1 | B08 | b2 | C05 | CO2H |
| 4-2-D00-1-0009 | 453.13 | OH | A01 | a1 | B05 | b2 | C06 | CO2H |
| 4-2-D00-1-0010 | 453.13 | OH | A02 | a1 | B05 | b2 | C06 | CO2H |
| 4-2-D00-1-0011 | 467.15 | OH | A01 | a1 | B06 | b2 | C06 | CO2H |
| 4-2-D00-1-0012 | 467.15 | OH | A02 | a1 | B06 | b2 | C06 | CO2H |
| 4-2-D00-1-0013 | 467.15 | OH | A01 | a1 | B07 | b2 | C06 | CO2H |

(continued)

| [Table 10-13-3] Compound contained in mixture (4-2-D00-1) obtained by solid-phase mixing | | | | | | | | |
|---|---|---|---|---|---|---|---|---|
| ID | Exact Mass | | A | a | B | b | C | c |
| 4-2-D00-1-0014 | 467.15 | OH | A02 | a1 | B07 | b2 | C06 | CO2H |
| 4-2-D00-1-0015 | 483.14 | OH | A01 | a1 | B08 | b2 | C06 | CO2H |
| 4-2-D00-1-0016 | 483.14 | OH | A02 | a1 | B08 | b2 | C06 | CO2H |
| 4-2-D00-1-0017 | 466.15 | OH | A01 | a1 | B05 | b2 | C07 | CO2H |
| 4-2-D00-1-0018 | 466.15 | OH | A02 | a1 | B05 | b2 | C07 | CO2H |
| 4-2-D00-1-0019 | 480.17 | OH | A01 | a1 | B06 | b2 | C07 | CO2H |
| 4-2-D00-1-0020 | 480.17 | OH | A02 | a1 | B06 | b2 | C07 | CO2H |
| 4-2-D00-1-0021 | 480.17 | OH | A01 | a1 | B07 | b2 | C07 | CO2H |
| 4-2-D00-1-0022 | 480.17 | OH | A02 | a1 | B07 | b2 | C07 | CO2H |
| 4-2-D00-1-0023 | 496.16 | OH | A01 | a1 | B08 | b2 | C07 | CO2H |
| 4-2-D00-1-0024 | 496.16 | OH | A02 | a1 | B08 | b2 | C07 | CO2H |
| 4-2-D00-1-0025 | 416.14 | OH | A01 | a1 | B05 | b2 | C08 | CO2H |
| 4-2-D00-1-0026 | 416.14 | OH | A02 | a1 | B05 | b2 | C08 | CO2H |
| 4-2-D00-1-0027 | 430.15 | OH | A01 | a1 | B06 | b2 | C08 | CO2H |
| 4-2-D00-1-0028 | 430.15 | OH | A02 | a1 | B06 | b2 | C08 | CO2H |
| 4-2-D00-1-0029 | 430.15 | OH | A01 | a1 | B07 | b2 | C08 | CO2H |
| 4-2-D00-1-0030 | 430.15 | OH | A02 | a1 | B07 | b2 | C08 | CO2H |
| 4-2-D00-1-0031 | 446.15 | OH | A01 | a1 | B08 | b2 | C08 | CO2H |
| 4-2-D00-1-0032 | 446.15 | OH | A02 | a1 | B08 | b2 | C08 | CO2H |

Example 10-14: Formation of linker c2 by step c2 in case 2

A reaction formula in step c2 performed using BB4-2-09 and -10 will be illustrated below.

[Formula 732]

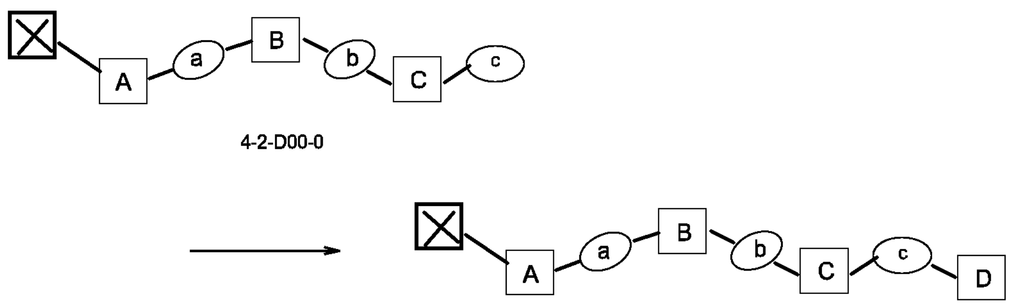

4-2-D00-0

4-2-c2D05-0 ~ 4-2-c2D08-0

Mixture 4-2-c2D05-0 and mixture 4-2-c2D06-0 were synthesized as follows. The following example shows step c2D05.

Under a nitrogen atmosphere, mixture 4-2-D00-0 (100 mg, 0.192 mmol/g) and DCM (2.0 mL) were added to a 4 mL glass vial and shaken at room temperature for 1 hour. An alcohol reagent (BB4-2-09) (35.0 μL, 0.383 mmol) was added to the glass vial. DMAP (4- dimethylaminopyridine) (23.4 mg, 0.192 mmol) and WSC-HCl (36.7 mg, 0.192 mmol) were added thereto, and the mixture was shaken at 25°C for 16.5 hours.

Solid-phase purification

The suspension of the reaction solution and the solid phase was transferred to a 3 mL column with a filter, filtered, and washed three times with NMP (2 mL), three times with NMP/water = 1/1 (2 mL), three times with NMP (2 mL), three times with MeOH (2 mL), three times with DCM (2 mL), and three times with heptane (2 mL), and the obtained solid phase was dried under reduced pressure to obtain mixture 4-2-c2D05-0.
Step c2D06 was performed in the same manner as in the synthesis of mixture 4-2- c2D05-0 to obtain mixture 4-2-c2D06-0.
The mixtures used in step c2D05 and step c2D06 and the amounts of the alcohol reagents used are shown in Table 10-14-1. The mixtures obtained in steps c2D05, and c2D06 are shown in Table 10-14-4 and Table 10-14-5.
[Table 572]

[Table 10-14-1] Reagent used and obtained mixture in steps c2D05 and c2D06

| Starting material used | Amount used | Alcohol reagent used | Amount used | Molar number | Obtained mixture | Yield | Amount supported |
|---|---|---|---|---|---|---|---|
| 4-2-D00-0 | 100 mg | BB4-2-09 | 35.0 μL | 0.383 mmol | 4-2-c2D05-0 | 94.8 mg | 0.190 mmol/g |
| 4-2-D00-0 | 145 mg | BB4-2-10 | 40.3 μL | 0.555 mmol | 4-2-c2D06-0 | 138.6 mg | 0.189 mmol/g |

Mixtures 4-2-c2D07-0, and 4-2-c2D08-0 were synthesized as follows. The following example shows step c2D07.
Under a nitrogen atmosphere, mixture 4-2-D00-0 (100 mg, 0.192 mmol/g) and DCM (2.0 mL) were added to a 4 mL glass vial and shaken at room temperature for 1 hour. A phenol reagent (BB4-2-11) (20.0 μL, 0.192 mmol) was added to the glass vial. PipClU (chlorodipiperidinocarbenium hexafluorophosphate) (69.1 mg, 0.192 mmol) and NMI (1-methylimidazole) (30.5 μL, 0.383 mmol) were added thereto, and the mixture was shaken at 25°C for 16.5 hours.

Solid-phase purification

The suspension of the reaction solution and the solid phase was transferred to a 3 mL column with a filter, filtered, and washed three times with NMP (2 mL), three times with NMP/water = 1/1 (2 mL), three times with NMP (2 mL), three times with MeOH (2 mL), three times with DCM (2 mL), and three times with heptane (2 mL), and the obtained solid phase was dried under reduced pressure to obtain mixture 4-2-c2D07-0.
Step c2D08 was performed in the same manner as in the synthesis of mixture 4-2- c2D07-0 to obtain mixture 4-2-c2D08-0.
The mixtures used in steps c2D07 and c2D08 and the amounts of the phenol reagents used are shown in Table 10-14-2. The mixtures obtained in steps c2D07 and c2D08 are shown in Table 10-14-6 to Table 10-14-7.
[Table 573]

[Table 10-14-2] Reagent used and obtained mixture in steps c2D07 and c2D08

| Starting material used | Amount used | Phenol reagent used | Amount used | Molar number | Obtained mixture | Yield | Amount supported |
|---|---|---|---|---|---|---|---|
| 4-2-D00-0 | 100 mg | BB4-2-11 | 20.0 μL | 0.192 mmol | 4-2-c2D07-0 | 92.8 mg | 0.188 mmol/g |
| 4-2-D00-0 | 100 mg | BB4-2-12 | 26.1 mg | 0.192 mmol | 4-2-c2D08-0 | 93.0 mg | 0.187 mmol/g |

Mixture 4-2-c2D05-0 can be treated by cleavage in the same manner as in Example 10-1 to obtain mixture 4-2-c2D05-1. The details of the mixtures are shown in Table 10-14-4 to Table 10-14-7.
A notation method in each table will be described. For example, in Table 10-14-4, each compound that may be contained in 4-2-c2D05-1 is indicated by ID, and core blocks and linkers in combination in the structure of mixture 4-2-c2D05-1 are indicated by symbols or chemical formulas. The correspondence between the symbols and specific

structures are shown in Figure 9. In Table 10-14-3 to Table 10-14-7, "×" is represented by a chemical formula.

[Formula 733]

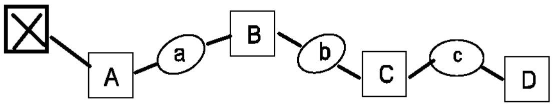

4-2-c2D05-0 ~ 4-2-c2D08-0

For example, when ID is 4-2-c2D05-1-0001, the compound is represented by the following structural formula.

[Formula 734]

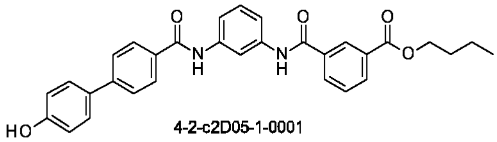

4-2-c2D05-1-0001

[Table 574]

| [Table 10-14-3] Structural notation in ID (4-2-c2D05-1-0001) | | | | | | | | |
|---|---|---|---|---|---|---|---|---|
| ID | | A | a | B | b | C | c | D |
| 4-2-c2D05-1-0001 | OH | A01 | a1 | B05 | b2 | C05 | c2 | D05 |

The mixture synthesized in step c2D05 using BB4-2-09 is shown in Table 10-14-4.

[Table 575]

| [Table 10-14-4] Compound contained in mixture (4-2-c2D05-1) synthesized in step c2D05 using BB4-2-09 | | | | | | | | | |
|---|---|---|---|---|---|---|---|---|---|
| ID | Exact Mass | | A | a | B | b | C | c | D |
| 4-2-c2D05-1-0001 | 508.20 | OH | A01 | a1 | B05 | b2 | C05 | c2 | D05 |
| 4-2-c2D05-1-0002 | 508.20 | OH | A02 | a1 | B05 | b2 | C05 | c2 | D05 |
| 4-2-c2D05-1-0003 | 522.22 | OH | A01 | a1 | B06 | b2 | C05 | c2 | D05 |
| 4-2-c2D05-1-0004 | 522.22 | OH | A02 | a1 | B06 | b2 | C05 | c2 | D05 |
| 4-2-c2D05-1-0005 | 522.22 | OH | A01 | a1 | B07 | b2 | C05 | c2 | D05 |
| 4-2-c2D05-1-0006 | 522.22 | OH | A02 | a1 | B07 | b2 | C05 | c2 | D05 |
| 4-2-c2D05-1-0007 | 538.21 | OH | A01 | a1 | B08 | b2 | C05 | c2 | D05 |
| 4-2-c2D05-1-0008 | 538.21 | OH | A02 | a1 | B08 | b2 | C05 | c2 | D05 |
| 4-2-c2D05-1-0009 | 509.20 | OH | A01 | a1 | B05 | b2 | C06 | c2 | D05 |
| 4-2-c2D05-1-0010 | 509.20 | OH | A02 | a1 | B05 | b2 | C06 | c2 | D05 |
| 4-2-c2D05-1-0011 | 523.21 | OH | A01 | a1 | B06 | b2 | C06 | c2 | D05 |
| 4-2-c2D05-1-0012 | 523.21 | OH | A02 | a1 | B06 | b2 | C06 | c2 | D05 |
| 4-2-c2D05-1-0013 | 523.21 | OH | A01 | a1 | B07 | b2 | C06 | c2 | D05 |
| 4-2-c2D05-1-0014 | 523.21 | OH | A02 | a1 | B07 | b2 | C06 | c2 | D05 |
| 4-2-c2D05-1-0013 | 539.21 | OH | A01 | a1 | B08 | b2 | C06 | c2 | D05 |

(continued)

| [Table 10-14-4] Compound contained in mixture (4-2-c2D05-1) synthesized in step c2D05 using BB4-2-09 | | | | | | | | | |
|---|---|---|---|---|---|---|---|---|---|
| ID | Exact Mass | | A | a | B | b | C | c | D |
| 4-2-c2D05-1-0016 | 539.21 | OH | A02 | a1 | B08 | b2 | C06 | c2 | D05 |
| 4-2-c2D05-1-0017 | 522.22 | OH | A01 | a1 | B05 | b2 | C07 | c2 | D05 |
| 4-2-c2D05-1-0018 | 522.22 | OH | A02 | a1 | B05 | b2 | C07 | c2 | D05 |
| 4-2-c2D05-1-0019 | 536.23 | OH | A01 | a1 | B06 | b2 | C07 | c2 | D05 |
| 4-2-c2D05-1-0020 | 536.23 | OH | A02 | a1 | B06 | b2 | C07 | c2 | D05 |
| 4-2-c2D05-1-0021 | 536.23 | OH | A01 | a1 | B07 | b2 | C07 | c2 | D05 |
| 4-2-c2D05-1-0022 | 536.23 | OH | A02 | a1 | B07 | b2 | C07 | c2 | D05 |
| 4-2-c2D05-1-0023 | 552.23 | OH | A01 | a1 | B08 | b2 | C07 | c2 | D05 |
| 4-2-c2D05-1-0024 | 552.23 | OH | A02 | a1 | B08 | b2 | C07 | c2 | D05 |
| 4-2-c2D05-1-0025 | 472.20 | OH | A01 | a1 | B05 | b2 | C08 | c2 | D05 |
| 4-2-c2D05-1-0026 | 472.20 | OH | A02 | a1 | B05 | b2 | C08 | c2 | D05 |
| 4-2-c2D05-1-0027 | 486.22 | OH | A01 | a1 | B06 | b2 | C08 | c2 | D05 |
| 4-2-c2D05-1-0028 | 486.22 | OH | A02 | a1 | B06 | b2 | C08 | c2 | D05 |
| 4-2-c2D05-1-0029 | 486.22 | OH | A01 | a1 | B07 | b2 | C08 | c2 | D05 |
| 4-2-c2D05-1-0030 | 486.22 | OH | A02 | a1 | B07 | b2 | C08 | c2 | D05 |
| 4-2-c2D05-1-0031 | 502.21 | OH | A01 | a1 | B08 | b2 | C08 | c2 | D05 |
| 4-2-c2D05-1-0032 | 502.21 | OH | A02 | a1 | B08 | b2 | C08 | c2 | D05 |

The mixture synthesized in step c2D06 using BB4-2-10 is shown in Table 10-14-5.

[Table 576]

| [Table 10-14-5] Compound contained in mixture (4-2-c2D06-1) synthesized in step c2D06 using BB4-2-10 | | | | | | | | | |
|---|---|---|---|---|---|---|---|---|---|
| ID | Exact Mass | | A | a | B | b | C | c | D |
| 4-2-c2D06-1-0001 | 534.14 | OH | A01 | a1 | B05 | b2 | C05 | c2 | D06 |
| 4-2-c2D06-1-0002 | 534.14 | OH | A02 | a1 | B05 | b2 | C05 | c2 | D06 |
| 4-2-c2D06-1-0003 | 548.16 | OH | A01 | a1 | B06 | b2 | C05 | c2 | D06 |
| 4-2-c2D06-1-0004 | 548.16 | OH | A02 | a1 | B06 | b2 | C05 | c2 | D06 |
| 4-2-c2D06-1-0005 | 548.16 | OH | A01 | a1 | B07 | b2 | C05 | c2 | D06 |
| 4-2-c2D06-1-0006 | 548.16 | OH | A02 | a1 | B07 | b2 | C05 | c2 | D06 |
| 4-2-c2D06-1-0007 | 564.15 | OH | A01 | a1 | B08 | b2 | C05 | c2 | D06 |
| 4-2-c2D06-1-0008 | 564.15 | OH | A02 | a1 | B08 | b2 | C05 | c2 | D06 |
| 4-2-c2D06-1-0009 | 535.14 | OH | A01 | a1 | B05 | b2 | C06 | c2 | D06 |
| 4-2-c2D06-1-0010 | 535.14 | OH | A02 | a1 | B05 | b2 | C06 | c2 | D06 |
| 4-2-c2D06-1-0011 | 549.15 | OH | A01 | a1 | B06 | b2 | C06 | c2 | D06 |
| 4-2-c2D06-1-0012 | 549.15 | OH | A02 | a1 | B06 | b2 | C06 | c2 | D06 |
| 4-2-c2D06-1-0013 | 549.15 | OH | A01 | a1 | B07 | b2 | C06 | c2 | D06 |
| 4-2-c2D06-1-0014 | 549.15 | OH | A02 | a1 | B07 | b2 | C06 | c2 | D06 |
| 4-2-c2D06-1-0015 | 565.15 | OH | A01 | a1 | B08 | b2 | C06 | c2 | D06 |
| 4-2-c2D06-1-0016 | 565.15 | OH | A02 | a1 | B08 | b2 | C06 | c2 | D06 |

(continued)

| [Table 10-14-5] Compound contained in mixture (4-2-c2D06-1) synthesized in step c2D06 using BB4-2-10 | | | | | | | | | |
|---|---|---|---|---|---|---|---|---|---|
| ID | Exact Mass | | A | a | B | b | C | c | D |
| 4-2-c2D06-1-0017 | 548.16 | OH | A01 | a1 | B05 | b2 | C07 | c2 | D06 |
| 4-2-c2D06-1-0018 | 548.16 | OH | A02 | a1 | B05 | b2 | C07 | c2 | D06 |
| 4-2-c2D06-1-0019 | 562.17 | OH | A01 | a1 | B06 | b2 | C07 | c2 | D06 |
| 4-2-c2D06-1-0020 | 562.17 | OH | A02 | a1 | B06 | b2 | C07 | c2 | D06 |
| 4-2-c2D06-1-0021 | 562.17 | OH | A01 | a1 | B07 | b2 | C07 | c2 | D06 |
| 4-2-c2D06-1-0022 | 562.17 | OH | A02 | a1 | B07 | b2 | C07 | c2 | D06 |
| 4-2-c2D06-1-0023 | 578.17 | OH | A01 | a1 | B08 | b2 | C07 | c2 | D06 |
| 4-2-c2D06-1-0024 | 578.17 | OH | A02 | a1 | B08 | b2 | C07 | c2 | D06 |
| 4-2-c2D06-1-0025 | 498.14 | OH | A01 | a1 | B05 | b2 | C08 | c2 | D06 |
| 4-2-c2D06-1-0026 | 498.14 | OH | A02 | a1 | B05 | b2 | C08 | c2 | D06 |
| 4-2-c2D06-1-0027 | 512.16 | OH | A01 | a1 | B06 | b2 | C08 | c2 | D06 |
| 4-2-c2D06-1-0028 | 512.16 | OH | A02 | a1 | B06 | b2 | C08 | c2 | D06 |
| 4-2-c2D06-1-0029 | 512.16 | OH | A01 | a1 | B07 | b2 | C08 | c2 | D06 |
| 4-2-c2D06-1-0030 | 512.16 | OH | A02 | a1 | B07 | b2 | C08 | c2 | D06 |
| 4-2-c2D06-1-0031 | 528.15 | OH | A01 | a1 | B08 | b2 | C08 | c2 | D06 |
| 4-2-c2D06-1-0032 | 528.15 | OH | A02 | a1 | B08 | b2 | C08 | c2 | D06 |

The mixture synthesized in step c2D07 using BB4-2-11 is shown in Table 10-14-6.

[Table 577]

| [Table 10-14-6] Compound contained in mixture (4-2-c2D07-1) synthesized in step c2D07 using BB4-2-11 | | | | | | | | | |
|---|---|---|---|---|---|---|---|---|---|
| ID | Exact Mass | | A | a | B | b | C | c | D |
| 4-2-c2D07-1-0001 | 542.18 | OH | A01 | a1 | B05 | b2 | C05 | c2 | D07 |
| 4-2-c2D07-1-0002 | 542.18 | OH | A02 | a1 | B05 | b2 | C05 | c2 | D07 |
| 4-2-c2D07-1-0003 | 556.20 | OH | A01 | a1 | B06 | b2 | C05 | c2 | D07 |
| 4-2-c2D07-1-0004 | 556.20 | OH | A02 | a1 | B06 | b2 | C05 | c2 | D07 |
| 4-2-c2D07-1-0005 | 556.20 | OH | A01 | a1 | B07 | b2 | C05 | c2 | D07 |
| 4-2-c2D07-1-0006 | 556.20 | OH | A02 | a1 | B07 | b2 | C05 | c2 | D07 |
| 4-2-c2D07-1-0007 | 572.19 | OH | A01 | a1 | B08 | b2 | C05 | c2 | D07 |
| 4-2-c2D07-1-0008 | 572.19 | OH | A02 | a1 | B08 | b2 | C05 | c2 | D07 |
| 4-2-c2D07-1-0009 | 543.18 | OH | A01 | a1 | B05 | b2 | C06 | c2 | D07 |
| 4-2-c2D07-1-0010 | 543.18 | OH | A02 | a1 | B05 | b2 | C06 | c2 | D07 |
| 4-2-c2D07-1-0011 | 557.20 | OH | A01 | a1 | B06 | b2 | C06 | c2 | D07 |
| 4-2-c2D07-1-0012 | 557.20 | OH | A02 | a1 | B06 | b2 | C06 | c2 | D07 |
| 4-2-c2D07-1-0013 | 557.20 | OH | A01 | a1 | B07 | b2 | C06 | c2 | D07 |
| 4-2-c2D07-1-0014 | 557.20 | OH | A02 | a1 | B07 | b2 | C06 | c2 | D07 |
| 4-2-c2D07-1-0015 | 573.19 | OH | A01 | a1 | B08 | b2 | C06 | c2 | D07 |
| 4-2c2D07-1-0016 | 573.19 | OH | A02 | a1 | B08 | b2 | C06 | c2 | D07 |
| 4-2-c2D07-1-0017 | 556.20 | OH | A01 | a1 | B05 | b2 | C07 | c2 | D07 |

(continued)

| [Table 10-14-6] Compound contained in mixture (4-2-c2D07-1) synthesized in step c2D07 using BB4-2-11 | | | | | | | | | |
|---|---|---|---|---|---|---|---|---|---|
| ID | Exact Mass | | A | a | B | b | C | c | D |
| 4-2-c2D07-1-0018 | 556.20 | OH | A02 | a1 | B05 | b2 | C07 | c2 | D07 |
| 4-2-c2D07-1-0019 | 570.22 | OH | A01 | a1 | B06 | b2 | C07 | c2 | D07 |
| 4-2-c2D07-1-0020 | 570.22 | OH | A02 | a1 | B06 | b2 | C07 | c2 | D07 |
| 4-2-c2D07-1-0021 | 570.22 | OH | A01 | a1 | B07 | b2 | C07 | c2 | D07 |
| 4-2-c2D07-1-0022 | 570.22 | OH | A02 | a1 | B07 | b2 | C07 | c2 | D07 |
| 4-2-c2D07-1-0023 | 586.21 | OH | A01 | a1 | B08 | b2 | C07 | c2 | D07 |
| 4-2-c2D07-1-0024 | 586.21 | OH | A02 | a1 | B08 | b2 | C07 | c2 | D07 |
| 4-2-c2D07-1-0025 | 506.18 | OH | A01 | a1 | B05 | b2 | C08 | c2 | D07 |
| 4-2-c2D07-1-0026 | 506.18 | OH | A02 | a1 | B05 | b2 | C08 | c2 | D07 |
| 4-2-c2D07-1-0027 | 520.20 | OH | A01 | a1 | B06 | b2 | C08 | c2 | D07 |
| 4-2-c2D07-1-0028 | 520.20 | OH | A02 | a1 | B06 | b2 | C08 | c2 | D07 |
| 4-2-c2D07-1-0029 | 520.20 | OH | A01 | a1 | B07 | b2 | C08 | c2 | D07 |
| 4-2-c2D07-1-0030 | 520.20 | OH | A02 | a1 | B07 | b2 | C08 | c2 | D07 |
| 4-2-c2D07-1-0031 | 536.19 | OH | A01 | a1 | B08 | b2 | C08 | c2 | D07 |
| 4-2-c2D07-1-0032 | 536.19 | OH | A02 | a1 | B08 | b2 | C08 | c2 | D07 |

The mixture synthesized in step c2D08 using BB4-2-12 is shown in Table 10-14-7.

[Table 578]

| [Table 10-14-7] Compound contained in mixture (4-2-c2D08-1) synthesized in step c2D08 using BB4-2-12 | | | | | | | | | |
|---|---|---|---|---|---|---|---|---|---|
| ID | Exact Mass | | A | a | B | b | C | c | D |
| 4-2-c2D08-1-0001 | 570.22 | OH | A01 | a1 | B05 | b2 | C05 | c2 | D08 |
| 4-2-c2D08-1-0002 | 570.22 | OH | A02 | a1 | B05 | b2 | C05 | c2 | D08 |
| 4-2-c2D08-1-0003 | 584.23 | OH | A01 | a1 | B06 | b2 | C05 | c2 | D08 |
| 4-2-c2D08-1-0004 | 584.23 | OH | A02 | a1 | B06 | b2 | C05 | c2 | D08 |
| 4-2-c2D08-1-0005 | 584.23 | OH | A01 | a1 | B07 | b2 | C05 | c2 | D08 |
| 4-2-c2D08-1-0006 | 584.23 | OH | A02 | a1 | B07 | b2 | C05 | c2 | D08 |
| 4-2-c2D08-1-0007 | 600.23 | OH | A01 | a1 | B08 | b2 | C05 | c2 | D08 |
| 4-2-c2D08-1-0008 | 600.23 | OH | A02 | a1 | B08 | b2 | C05 | c2 | D08 |
| 4-2-c2D08-1-0009 | 571.21 | OH | A01 | a1 | B05 | b2 | C06 | c2 | D08 |
| 4-2-c2D08-1-0010 | 571.21 | OH | A02 | a1 | B05 | b2 | C06 | c2 | D08 |
| 4-2-c2D08-1-0011 | 585.23 | OH | A01 | a1 | B06 | b2 | C06 | c2 | D08 |
| 4-2-c2D08-1-0012 | 585.23 | OH | A02 | a1 | B06 | b2 | C06 | c2 | D08 |
| 4-2-c2D08-1-0013 | 585.23 | OH | A01 | a1 | B07 | b2 | C06 | c2 | D08 |
| 4-2-c2D08-1-0014 | 585.23 | OH | A02 | a1 | B07 | b2 | C06 | c2 | D08 |
| 4-2-c2D08-1-0015 | 601.22 | OH | A01 | a1 | B08 | b2 | C06 | c2 | D08 |
| 4-2-c2D08-1-0016 | 601.22 | OH | A02 | a1 | B08 | b2 | C06 | c2 | D08 |
| 4-2-c2D08-1-0017 | 584.23 | OH | A01 | a1 | B05 | b2 | C07 | c2 | D08 |
| 4-2-c2D08-1-0018 | 584.23 | OH | A02 | a1 | B05 | b2 | C07 | c2 | D08 |

(continued)

| [Table 10-14-7] Compound contained in mixture (4-2-c2D08-1) synthesized in step c2D08 using BB4-2-12 | | | | | | | | | |
|---|---|---|---|---|---|---|---|---|---|
| ID | Exact Mass | | A | a | B | b | C | c | D |
| 4-2-c2D08-1-0019 | 598.25 | OH | A01 | a1 | B06 | b2 | C07 | c2 | D08 |
| 4-2-c2D08-1-0020 | 598.25 | OH | A02 | a1 | B06 | b2 | C07 | c2 | D08 |
| 4-2-c2D08-1-0021 | 598.25 | OH | A01 | a1 | B07 | b2 | C07 | c2 | D08 |
| 4-2-c2D08-1-0022 | 598.25 | OH | A02 | a1 | B07 | b2 | C07 | c2 | D08 |
| 4-2-c2D08-1-0023 | 614.24 | OH | A01 | a1 | B08 | b2 | C07 | c2 | D08 |
| 4-2-c2D08-1-0024 | 614.24 | OH | A02 | a1 | B08 | b2 | C07 | c2 | D08 |
| 4-2-c2D08-1-0025 | 534.22 | OH | A01 | a1 | B05 | b2 | C08 | c2 | D08 |
| 4-2-c2D08-1-0026 | 534.22 | OH | A02 | a1 | B05 | b2 | C08 | c2 | D08 |
| 4-2-c2D08-1-0027 | 548.23 | OH | A01 | a1 | B06 | b2 | C08 | c2 | D08 |
| 4-2-c2D08-1-0028 | 548.23 | OH | A02 | a1 | B06 | b2 | C08 | c2 | D08 |
| 4-2-c2D08-1-0029 | 548.23 | OH | A01 | a1 | B07 | b2 | C08 | c2 | D08 |
| 4-2-c2D08-1-0030 | 548.23 | OH | A02 | a1 | B07 | b2 | C08 | c2 | D08 |
| 4-2-c2D08-1-0031 | 564.23 | OH | A01 | a1 | B08 | b2 | C08 | c2 | D08 |
| 4-2-c2D08-1-0032 | 564.23 | OH | A02 | a1 | B08 | b2 | C08 | c2 | D08 |

Example 10-15: Synthesis of mixture 4-2-c2D05-1 to mixture 4-2-c2D08-1 by cleavage steps D05-1 to D08-1 in case 2

A reaction formula in step D05-1 performed using mixture 4-2-c2D05-0 will be illustrated below.

[Formula 735]

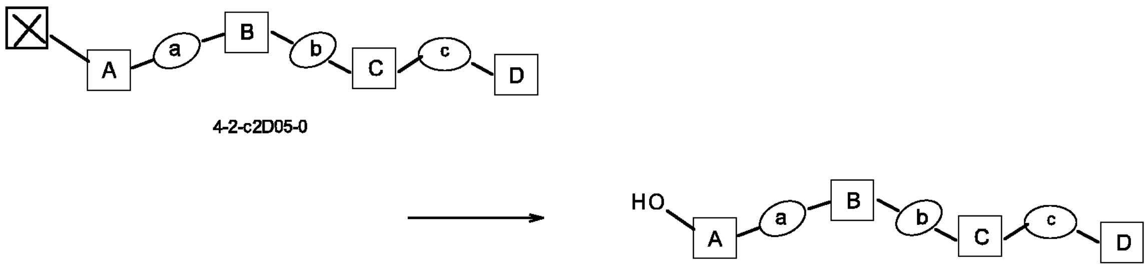

4-2-c2D05-0

4-2-c2D05-1

Mixture 4-2-c2D05-1 to mixture 4-2-c2D08-1 were synthesized as follows using mixture 4-2-c2D05-0 to mixture 4-2-c2D08-0. The following example shows step D05-1.

Under a nitrogen atmosphere, mixture 4-2-c2D05-0 (80 mg, 0.190 mmol/g) was added to a 4 mL glass vial. Pentamethylbenzene (17.1 mg, 0.115 mmol) and DCM (1.4 mL) were added thereto and shaken at room temperature for 1 hour. TFA (160 μL) was added thereto and shaken at room temperature for 1 hour. The suspension of the reaction solution and the solid phase was transferred to a 3 mL column with a filter using DMF (0.5 mL) twice and filtered. The resultant was washed three times with DMF (0.5 mL), and combined filtrates were collected into a 15 mm*75 mm test tube. The solvent in the solution was distilled off by Genevac. Solid-phase supported morpholine (Sigma-Aldrich Co., LLC, catalog No: 493813) (160 mg) and DMF (2.0 mL) were added thereto, and the mixture was shaken at room temperature for 20 minutes. The solution and the suspension of the solid phase were transferred to a 3 mL column with a filter using DMF (0.5 mL) twice and filtered. The resultant was washed three times with DMF (0.5 mL) and twice with DCM/DMSO (2/1, 0.5 mL), and combined filtrates were collected into a 15 mm*75 mm test tube. The solvent in the solution was distilled off under reduced pressure(Genevac reduced-pressure concentrator was used) to obtain mixture 4-2-c2D05-1.

Steps D06-1 to D08-1 were performed in the same manner as in the synthesis of mixture 4-2-c2D05-1 to obtain mixture 4-2-c2D06-1 to mixture 4-2-c2D08-1.

The reagents used in steps D05-1 to D08-1 and the obtained mixtures are shown in Table 10-15-1.

[Table 579]

| [Table 10-15-1] Starting material used and obtained mixture in steps D05-1 to D08-1 | | | | |
|---|---|---|---|---|
| Mixture used | Amount used | Amount of solid- phase supported morpholine used | Obtained mixture | Yield |
| 4-2-c2D05-0 | 80.0 mg | 160 mg | 4-2-c2D05-1 | 11.0 mg |
| 4-2-c2D06-0 | 84.0 mg | 160 mg | 4-2-c2D06-1 | 11.0 mg |
| 4-2-c2D07-0 | 80.9 mg | 160 mg | 4-2-c2D07-1 | 9.6 mg |
| 4-2-c2D08-0 | 80.0 mg | 160 mg | 4-2-c2D08-1 | 13.6 mg |

Example 10-16: Mixing of mixture 4-2-c2D05-1 to mixture 4-2-c2D08-1 in case 2

[Formula 736]

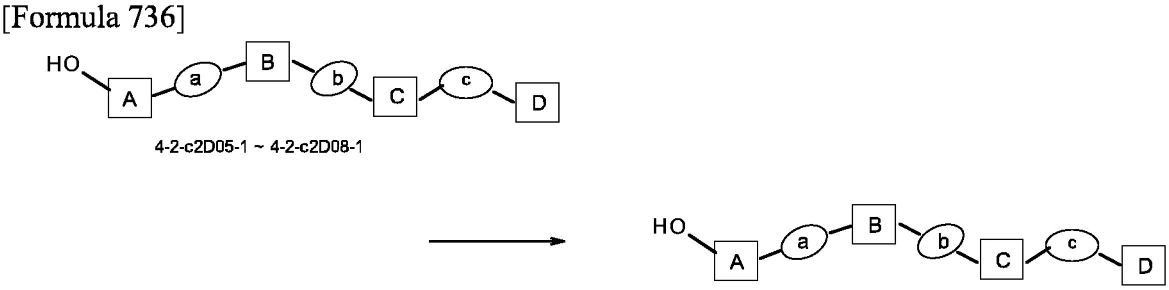

4-2-c2D05-1 ~ 4-2-c2D08-1

4-2-F00-1

4 types of mixtures described in Table 10-16-1 were mixed in a 15 mm*75 mm test tube using DMF (1.2 mL for each), and the solvent in the obtained solution was distilled off under reduced pressure (Genevac reduced-pressure concentrator was used) to obtain 25.8 mg of mixture 4-2-F00-1.
The mixtures used and the amounts of the mixtures used are shown in Table 10-16-1.
[Table 580]

| [Table 10-16-1] Mixture used and amount used in mixing | |
|---|---|
| Mixture | Amount used |
| 4-2-c2D05-1 | 11.0 mg |
| 4-2-c2D06-1 | 11.0 mg |
| 4-2-c2D07-1 | 9.6 mg |
| 4-2-c2D08-1 | 13.6 mg |

The details of the mixture obtained in the mixing step of mixture 4-2-c2D05-1 to mixture 4-2-c2D08-1 are shown in Table 10-16-3.
A notation method in each table will be described. In Table 10-16-3, each compound that may be contained in 4-2-F00-1 is indicated by ID, and core blocks and linkers in combination in the structure of mixture 4-2-F00-1 are indicated by symbols or chemical formulas. The correspondence between the symbols and specific structures are shown in Figure 9. In Table 10-16-2 to Table 10-16-3, "×" is represented by a chemical formula.

[Formula 737]

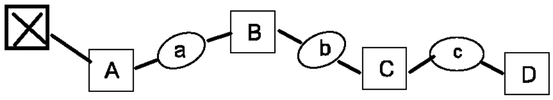

4-2-F00-1

For example, when ID is 4-2-F00-1-0001, the compound is represented by the following structural formula.

[Formula 738]

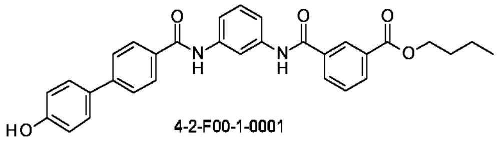

4-2-F00-1-0001

[Table 581]

| [Table 10-16-2] Structural notation in ID (4-2-F00-1-0001) | | | | | | | | |
|---|---|---|---|---|---|---|---|---|
| ID | | A | a | B | b | C | c | D |
| 4-2-F00-1-0001 | OH | A01 | a1 | B05 | b2 | C05 | c2 | D05 |

Compounds that may be contained in mixture 4-2-F00-1 are shown in the following Table 10-16-3.

[Table 582]

| [Table 10-16-3] Compound that may be used in mixture 4-2-F00-1 | | | | | | | | | |
|---|---|---|---|---|---|---|---|---|---|
| ID | Exact Mass | | A | a | B | b | C | c | D |
| 4-2-F00-1-0001 | 508.20 | OH | A01 | a1 | B05 | b2 | C05 | c2 | D05 |
| 4-2-F00-1-0002 | 508.20 | OH | A02 | a1 | B05 | b2 | C05 | c2 | D05 |
| 4-2-F00-1-0003 | 522.22 | OH | A01 | a1 | B06 | b2 | C05 | c2 | D05 |
| 4-2-F00-1-0004 | 522.22 | OH | A02 | a1 | B06 | b2 | C05 | c2 | D05 |
| 4-2-F00-1-0005 | 522.22 | OH | A01 | a1 | B07 | b2 | C05 | c2 | D05 |
| 4-2-F00-1-0006 | 522.22 | OH | A02 | a1 | B07 | b2 | C05 | c2 | D05 |
| 4-2-F00-1-0007 | 538.21 | OH | A01 | a1 | B08 | b2 | C05 | c2 | D05 |
| 4-2-F00-1-0008 | 538.21 | OH | A02 | a1 | B08 | b2 | C05 | c2 | D05 |
| 4-2-F00-1-0009 | 509.20 | OH | A01 | a1 | B05 | b2 | C06 | c2 | D05 |
| 4-2-F00-1-0010 | 509.20 | OH | A02 | a1 | B05 | b2 | C06 | c2 | D05 |
| 4-2-F00-1-0011 | 523.21 | OH | A01 | a1 | B06 | b2 | C06 | c2 | D05 |
| 4-2-F00-1-0012 | 523.21 | OH | A02 | a1 | B06 | b2 | C06 | c2 | D05 |
| 4-2-F00-1-0013 | 523.21 | OH | A01 | a1 | B07 | b2 | C06 | c2 | D05 |
| 4-2-F00-1-0014 | 523.21 | OH | A02 | a1 | B07 | b2 | C06 | c2 | D05 |
| 4-2-F00-1-0015 | 539.21 | OH | A01 | a1 | B08 | b2 | C06 | c2 | D05 |
| 4-2-F00-1-0016 | 539.21 | OH | A02 | a1 | B08 | b2 | C06 | c2 | D05 |
| 4-2-F00-1-0017 | 522.22 | OH | A01 | a1 | B05 | b2 | C07 | c2 | D05 |
| 4-2-F00-1-0018 | 522.22 | OH | A02 | a1 | B05 | b2 | C07 | c2 | D05 |
| 4-2-F00-1-0019 | 536.23 | OH | A01 | a1 | B06 | b2 | C07 | c2 | D05 |

(continued)

| [Table 10-16-3] Compound that may be used in mixture 4-2-F00-1 | | | | | | | | | |
|---|---|---|---|---|---|---|---|---|---|
| ID | Exact Mass | | A | a | B | b | C | c | D |
| 4-2-F00-1-0020 | 536.23 | OH | A02 | a1 | B06 | b2 | C07 | c2 | D05 |
| 4-2-F00-1-0021 | 536.23 | OH | A01 | a1 | B07 | b2 | C07 | c2 | D05 |
| 4-2-F00-1-0022 | 536.23 | OH | A02 | a1 | B07 | b2 | C07 | c2 | D05 |
| 4-2-F00-1-0023 | 552.23 | OH | A01 | a1 | B08 | b2 | C07 | c2 | D05 |
| 4-2-F00-1-0024 | 552.23 | OH | A02 | a1 | B08 | b2 | C07 | c2 | D05 |
| 4-2-F00-1-0025 | 472.20 | OH | A01 | a1 | B05 | b2 | C08 | c2 | D05 |
| 4-2-F00-1-0026 | 472.20 | OH | A02 | a1 | B05 | b2 | C08 | c2 | D05 |
| 4-2-F00-1-0027 | 486.22 | OH | A01 | a1 | B06 | b2 | C08 | c2 | D05 |
| 4-2-F00-1-0028 | 486.22 | OH | A02 | a1 | B06 | b2 | C08 | c2 | D05 |
| 4-2-F00-1-0029 | 486.22 | OH | A01 | a1 | B07 | b2 | C08 | c2 | D05 |
| 4-2-F00-1-0030 | 486.22 | OH | A02 | a1 | B07 | b2 | C08 | c2 | D05 |
| 4-2-F00-1-0031 | 502.21 | OH | A01 | a1 | B08 | b2 | C08 | c2 | D05 |
| 4-2-F00-1-0032 | 502.21 | OH | A02 | a1 | B08 | b2 | C08 | c2 | D05 |
| 4-2-F00-1-0033 | 534.14 | OH | A01 | a1 | B05 | b2 | C05 | c2 | D06 |
| 4-2-F00-1-0034 | 534.14 | OH | A02 | a1 | B05 | b2 | C05 | c2 | D06 |
| 4-2-F00-1-0035 | 548.16 | OH | A01 | a1 | B06 | b2 | C05 | c2 | D06 |
| 4-2-F00-1-0036 | 548.16 | OH | A02 | a1 | B06 | b2 | C05 | c2 | D06 |
| 4-2-F00-1-0037 | 548.16 | OH | A01 | a1 | B07 | b2 | C05 | c2 | D06 |
| 4-2-F00-1-0038 | 548.16 | OH | A02 | a1 | B07 | b2 | C05 | c2 | D06 |
| 4-2-F00-1-0039 | 564.15 | OH | A01 | a1 | B08 | b2 | C05 | c2 | D06 |
| 4-2-F00-1-0040 | 564.15 | OH | A02 | a1 | B08 | b2 | C05 | c2 | D06 |
| 4-2-F00-1-0041 | 535.14 | OH | A01 | a1 | B05 | b2 | C06 | c2 | D06 |
| 4-2-F00-1-0042 | 535.14 | OH | A02 | a1 | B05 | b2 | C06 | c2 | D06 |
| 4-2-F00-1-0043 | 549.15 | OH | A01 | a1 | B06 | b2 | C06 | c2 | D06 |
| 4-2-F00-1-0044 | 549.15 | OH | A02 | a1 | B06 | b2 | C06 | c2 | D06 |
| 4-2-F00-1-0045 | 549.15 | OH | A01 | a1 | B07 | b2 | C06 | c2 | D06 |
| 4-2-F00-1-0046 | 549.15 | OH | A02 | a1 | B07 | b2 | C06 | c2 | D06 |
| 4-2-F00-1-0047 | 565.15 | OH | A01 | a1 | B08 | b2 | C06 | c2 | D06 |
| 4-2-F00-1-0048 | 565.15 | OH | A02 | a1 | B08 | b2 | C06 | c2 | D06 |
| 4-2-F00-1-0049 | 548.16 | OH | A01 | a1 | B05 | b2 | C07 | c2 | D06 |
| 4-2-F00-1-0050 | 548.16 | OH | A02 | a1 | B05 | b2 | C07 | c2 | D06 |
| 4-2-F00-1-0051 | 562.17 | OH | A01 | a1 | B06 | b2 | C07 | c2 | D06 |
| 4-2-F00-1-0052 | 562.17 | OH | A02 | a1 | B06 | b2 | C07 | c2 | D06 |
| 4-2-F00-1-0053 | 562.17 | OH | A01 | a1 | B07 | b2 | C07 | c2 | D06 |
| 4-2-F00-1-0054 | 562.17 | OH | A02 | a1 | B07 | b2 | C07 | c2 | D06 |
| 4-2-F00-1-0055 | 578.17 | OH | A01 | a1 | B08 | b2 | C07 | c2 | D06 |
| 4-2-F00-1-0056 | 578.17 | OH | A02 | a1 | B08 | b2 | C07 | c2 | D06 |
| 4-2-F00-1-0057 | 498.14 | OH | A01 | a1 | B05 | b2 | C08 | c2 | D06 |

(continued)

| [Table 10-16-3] Compound that may be used in mixture 4-2-F00-1 | | | | | | | | | |
|---|---|---|---|---|---|---|---|---|---|
| ID | Exact Mass | | A | a | B | b | C | c | D |
| 4-2-F00-1-0058 | 498.14 | OH | A02 | a1 | B05 | b2 | C08 | c2 | D06 |
| 4-2-F00-1-0059 | 512.16 | OH | A01 | a1 | B06 | b2 | C08 | c2 | D06 |
| 4-2-F00-1-0060 | 512.16 | OH | A02 | a1 | B06 | b2 | C08 | c2 | D06 |
| 4-2-F00-1-0061 | 512.16 | OH | A01 | a1 | B07 | b2 | C08 | c2 | D06 |
| 4-2-F00-1-0062 | 512.16 | OH | A02 | a1 | B07 | b2 | C08 | c2 | D06 |
| 4-2-F00-1-0063 | 528.15 | OH | A01 | a1 | B08 | b2 | C08 | c2 | D06 |
| 4-2-F00-1-0064 | 528.15 | OH | A02 | a1 | B08 | b2 | C08 | c2 | D06 |
| 4-2-F00-1-0065 | 542.18 | OH | A01 | a1 | B05 | b2 | C05 | c2 | D07 |
| 4-2-F00-1-0066 | 542.18 | OH | A02 | a1 | B05 | b2 | C05 | c2 | D07 |
| 4-2-F00-1-0067 | 556.20 | OH | A01 | a1 | B06 | b2 | C05 | c2 | D07 |
| 4-2-F00-1-0068 | 556.20 | OH | A02 | a1 | B06 | b2 | C05 | c2 | D07 |
| 4-2-F00-1-0069 | 556.20 | OH | A01 | a1 | B07 | b2 | C05 | c2 | D07 |
| 4-2-F00-1-0070 | 556.20 | OH | A02 | a1 | B07 | b2 | C05 | c2 | D07 |
| 4-2-F00-1-0071 | 572.19 | OH | A01 | a1 | B08 | b2 | C05 | c2 | D07 |
| 4-2-F00-1-0072 | 572.19 | OH | A02 | a1 | B08 | b2 | C05 | c2 | D07 |
| 4-2-F00-1-0073 | 543.18 | OH | A01 | a1 | B05 | b2 | C06 | c2 | D07 |
| 4-2-F00-1-0074 | 543.18 | OH | A02 | a1 | B05 | b2 | C06 | c2 | D07 |
| 4-2-F00-1-0075 | 557.20 | OH | A01 | a1 | B06 | b2 | C06 | c2 | D07 |
| 4-2-F00-1-0076 | 557.20 | OH | A02 | a1 | B06 | b2 | C06 | c2 | D07 |
| 4-2-F00-1-0077 | 557.20 | OH | A01 | a1 | B07 | b2 | C06 | c2 | D07 |
| 4-2-F00-1-0078 | 557.20 | OH | A02 | a1 | B07 | b2 | C06 | c2 | D07 |
| 4-2-F00-1-0079 | 573.19 | OH | A01 | a1 | B08 | b2 | C06 | c2 | D07 |
| 4-2-F00-1-0080 | 573.19 | OH | A02 | a1 | B08 | b2 | C06 | c2 | D07 |
| 4-2-F00-1-0081 | 556.20 | OH | A01 | a1 | B05 | b2 | C07 | c2 | D07 |
| 4-2-F00-1-0082 | 556.20 | OH | A02 | a1 | B05 | b2 | C07 | c2 | D07 |
| 4-2-F00-1-0083 | 570.22 | OH | A01 | a1 | B06 | b2 | C07 | c2 | D07 |
| 4-2-F00-1-0084 | 570.22 | OH | A02 | a1 | B06 | b2 | C07 | c2 | D07 |
| 4-2-F00-1-0085 | 570.22 | OH | A01 | a1 | B07 | b2 | C07 | c2 | D07 |
| 4-2-F00-1-0086 | 570.22 | OH | A02 | a1 | B07 | b2 | C07 | c2 | D07 |
| 4-2-F00-1-0087 | 586.21 | OH | A01 | a1 | B08 | b2 | C07 | c2 | D07 |
| 4-2-F00-1-0088 | 586.21 | OH | A02 | a1 | B08 | b2 | C07 | c2 | D07 |
| 4-2-F00-1-0089 | 506.18 | OH | A01 | a1 | B05 | b2 | C08 | c2 | D07 |
| 4-2-F00-1-0090 | 506.18 | OH | A02 | a1 | B05 | b2 | C08 | c2 | D07 |
| 4-2-F00-1-0091 | 520.20 | OH | A01 | a1 | B06 | b2 | C08 | c2 | D07 |
| 4-2-F00-1-0092 | 520.20 | OH | A02 | a1 | B06 | b2 | C08 | c2 | D07 |
| 4-2-F00-1-0093 | 520.20 | OH | A01 | a1 | B07 | b2 | C08 | c2 | D07 |
| 4-2-F00-1-0094 | 520.20 | OH | A02 | a1 | B07 | b2 | C08 | c2 | D07 |

(continued)

| [Table 10-16-3] Compound that may be used in mixture 4-2-F00-1 | | | | | | | | | |
|---|---|---|---|---|---|---|---|---|---|
| ID | Exact Mass | | A | a | B | b | C | c | D |
| 4-2-F00-1-0095 | 536.19 | OH | A01 | a1 | B08 | b2 | C08 | c2 | D07 |
| 4-2-F00-1-0096 | 536.19 | OH | A02 | a1 | B08 | b2 | C08 | c2 | D07 |
| 4-2-F00-1-0097 | 570.22 | OH | A01 | a1 | B05 | b2 | C05 | c2 | D08 |
| 4-2-F00-1-0098 | 570.22 | OH | A02 | a1 | B05 | b2 | C05 | c2 | D08 |
| 4-2-F00-1-0099 | 584.23 | OH | A01 | a1 | B06 | b2 | C05 | c2 | D08 |
| 4-2-F00-1-0100 | 584.23 | OH | A02 | a1 | B06 | b2 | C05 | c2 | D08 |
| 4-2-F00-1-0101 | 584.23 | OH | A01 | a1 | B07 | b2 | C05 | c2 | D08 |
| 4-2-F00-1-0102 | 584.23 | OH | A02 | a1 | B07 | b2 | C05 | c2 | D08 |
| 4-2-F00-1-0103 | 600.23 | OH | A01 | a1 | B08 | b2 | C05 | c2 | D08 |
| 4-2-F00-1-0104 | 600.23 | OH | A02 | a1 | B08 | b2 | C05 | c2 | D08 |
| 4-2-F00-1-0105 | 571.21 | OH | A01 | a1 | B05 | b2 | C06 | c2 | D08 |
| 4-2-F00-1-0106 | 571.21 | OH | A02 | a1 | B05 | b2 | C06 | c2 | D08 |
| 4-2-F00-1-0107 | 585.23 | OH | A01 | a1 | B06 | b2 | C06 | c2 | D08 |
| 4-2-F00-1-0108 | 585.23 | OH | A02 | a1 | B06 | b2 | C06 | c2 | D08 |
| 4-2-F00-1-0109 | 585.23 | OH | A01 | a1 | B07 | b2 | C06 | c2 | D08 |
| 4-2-F00-1-0110 | 585.23 | OH | A02 | a1 | B07 | b2 | C06 | c2 | D08 |
| 4-2-F00-1-0111 | 601.22 | OH | A01 | a1 | B08 | b2 | C06 | c2 | D08 |
| 4-2-F00-1-0112 | 601.22 | OH | A02 | a1 | B08 | b2 | C06 | c2 | D08 |
| 4-2-F00-1-0113 | 584.23 | OH | A01 | a1 | B05 | b2 | C07 | c2 | D08 |
| 4-2-F00-1-0114 | 584.23 | OH | A02 | a1 | B05 | b2 | C07 | c2 | D08 |
| 4-2-F00-1-0115 | 598.25 | OH | A01 | a1 | B06 | b2 | C07 | c2 | D08 |
| 4-2-F00-1-0116 | 598.25 | OH | A02 | a1 | B06 | b2 | C07 | c2 | D08 |
| 4-2-F00-1-0117 | 598.25 | OH | A01 | a1 | B07 | b2 | C07 | c2 | D08 |
| 4-2-F00-1-0118 | 598.25 | OH | A02 | a1 | B07 | b2 | C07 | c2 | D08 |
| 4-2-F00-1-0119 | 614.24 | OH | A01 | a1 | B08 | b2 | C07 | c2 | D08 |
| 4-2-F00-1-0120 | 614.24 | OH | A02 | a1 | B08 | b2 | C07 | c2 | D08 |
| 4-2-F00-1-0121 | 534.22 | OH | A01 | a1 | B05 | b2 | C08 | c2 | D08 |
| 4-2-F00-1-0122 | 534.22 | OH | A02 | a1 | B05 | b2 | C08 | c2 | D08 |
| 4-2-F00-1-0123 | 548.23 | OH | A01 | a1 | B06 | b2 | C08 | c2 | D08 |
| 4-2-F00-1-0124 | 548.23 | OH | A02 | a1 | B06 | b2 | C08 | c2 | D08 |
| 4-2-F00-1-0125 | 548.23 | OH | A01 | a1 | B07 | b2 | C08 | c2 | D08 |
| 4-2-F00-1-0126 | 548.23 | OH | A02 | a1 | B07 | b2 | C08 | c2 | D08 |
| 4-2-F00-1-0127 | 564.23 | OH | A01 | a1 | B08 | b2 | C08 | c2 | D08 |
| 4-2-F00-1-0128 | 564.23 | OH | A02 | a1 | B08 | b2 | C08 | c2 | D08 |

Example 10-17: Mixing of mixtures 4-1-E00-1 and 4-2-F00-1 obtained in library case 1 and case 2

An actual example will be shown in which mixture 4-1-E00-1 and mixture 4-2-F00- 1 each independently synthesized were mixed to prepare one mixture library having bond diversity.

[Formula 739]

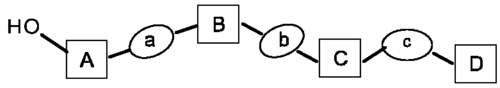

4-1-E00-1

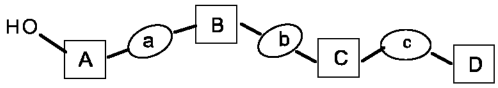

4-2-F00-1

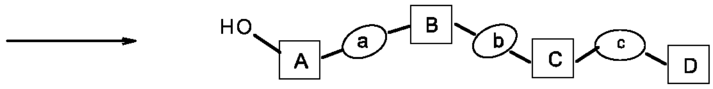

4-M00-1

2 types of mixtures described in Table 10-17-1 were mixed in a 15 mm*75 mm test tube using DMF (2.5 mL for each), and the solvent in the obtained solution was distilled off under reduced pressure (Genevac reduced-pressure concentrator was used) to obtain 49.6 mg of mixture 4-M00-1.

The mixtures used and the amounts of the mixtures used are shown in Table 10-17-1.

[Table 583]

| [Table 10-17-1] Mixture used and amount used in mixing | |
|---|---|
| Mixture | Amount used |
| 4-1-E00-1 | 24.6 mg |
| 4-2-F00-1 | 25.8 mg |

The details of the mixture are shown in Table 10-17-3.

A notation method in each table will be described. For example, in Table 10-17-3, each compound that may be contained in 4-M00-1 is indicated by ID, and core blocks and linkers in combination in the structure of mixture 4-M00-1 are indicated by symbols or chemical formulas. The correspondence between the symbols and specific structures are shown in Figure 9.

In Table 10-17-2 and Table 10-17-3, "×" is represented by a chemical formula.

[Formula 740]

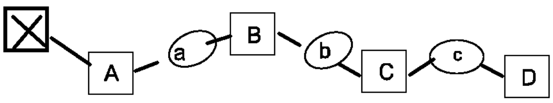

4-2-M00-1

For example, when ID is 4-M00-1-0001, the compound is represented by the following structural formula.

[Formula 741]

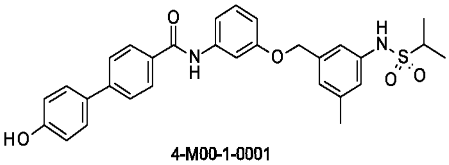

4-M00-1-0001

[Table 584]

| [Table 10-17-2] Structural notation in ID (4-M00-1-0001) | | | | | | | | |
|---|---|---|---|---|---|---|---|---|
| ID | | A | a | B | b | C | c | D |
| 4-M00-1-0001 | OH | A01 | a1 | B01 | b1 | C01 | c1 | D01 |

Compounds that may contained in mixture 4-M00-1 are shown in Table 10-17-3.

[Table 585]

| [Table 10-17-3] Compound (indicated by ID) that may be contained in mixture 4-M00-1 | | | | | | | | | |
|---|---|---|---|---|---|---|---|---|---|
| ID | Exact Mass | | A | a | B | b | C | c | D |
| 4-M00-1-0001 | 530.19 | OH | A01 | a1 | B01 | b1 | C01 | c1 | D01 |
| 4-M00-1-0002 | 530.19 | OH | A02 | a1 | B01 | b1 | C01 | c1 | D01 |
| 4-M00-1-0003 | 544.20 | OH | A01 | a1 | B02 | b1 | C01 | c1 | D01 |
| 4-M00-1-0004 | 544.20 | OH | A02 | a1 | B02 | b1 | C01 | c1 | D01 |
| 4-M00-1-0005 | 548.18 | OH | A01 | a1 | B03 | b1 | C01 | c1 | D01 |
| 4-M00-1-0006 | 548.18 | OH | A02 | a1 | B03 | b1 | C01 | c1 | D01 |
| 4-M00-1-0007 | 558.22 | OH | A01 | a1 | B04 | b1 | C01 | c1 | D01 |
| 4-M00-1-0008 | 558.22 | OH | A02 | a1 | B04 | b1 | C01 | c1 | D01 |
| 4-M00-1-0009 | 530.19 | OH | A01 | a1 | B01 | b1 | C02 | c1 | D01 |
| 4-M00-1-0010 | 530.19 | OH | A02 | a1 | B01 | b1 | C02 | c1 | D01 |
| 4-M00-1-0011 | 544.20 | OH | A01 | a1 | B02 | b1 | C02 | c1 | D01 |
| 4-M00-1-0012 | 544.20 | OH | A02 | a1 | B02 | b1 | C02 | c1 | D01 |
| 4-M00-1-0013 | 548.18 | OH | A01 | a1 | B03 | b1 | C02 | c1 | D01 |
| 4-M00-1-0014 | 548.18 | OH | A02 | a1 | B03 | b1 | C02 | c1 | D01 |
| 4-M00-1-0015 | 558.22 | OH | A01 | a1 | B04 | b1 | C02 | c1 | D01 |
| 4-M00-1-0016 | 558.22 | OH | A02 | a1 | B04 | b1 | C02 | c1 | D01 |
| 4-M00-1-0017 | 534.16 | OH | A01 | a1 | B01 | b1 | C03 | c1 | D01 |
| 4-M00-1-0018 | 534.16 | OH | A02 | a1 | B01 | b1 | C03 | c1 | D01 |
| 4-M00-1-0019 | 548.18 | OH | A01 | a1 | B02 | b1 | C03 | c1 | D01 |
| 4-M00-1-0020 | 548.18 | OH | A02 | a1 | B02 | b1 | C03 | c1 | D01 |
| 4-M00-1-0021 | 552.15 | OH | A01 | a1 | B03 | b1 | C03 | c1 | D01 |
| 4-M00-1-0022 | 552.15 | OH | A02 | a1 | B03 | b1 | C03 | c1 | D01 |
| 4-M00-1-0023 | 562.19 | OH | A01 | a1 | B04 | b1 | C03 | c1 | D01 |
| 4-M00-1-0024 | 562.19 | OH | A02 | a1 | B04 | b1 | C03 | c1 | D01 |
| 4-M00-1-0025 | 544.20 | OH | A01 | a1 | B01 | b1 | C04 | c1 | D01 |
| 4-M00-1-0026 | 544.20 | OH | A02 | a1 | B01 | b1 | C04 | c1 | D01 |
| 4-M00-1-0027 | 558.22 | OH | A01 | a1 | B02 | b1 | C04 | c1 | D01 |
| 4-M00-1-0028 | 558.22 | OH | A02 | a1 | B02 | b1 | C04 | c1 | D01 |
| 4-M00-1-0029 | 562.19 | OH | A01 | a1 | B03 | b1 | C04 | c1 | D01 |
| 4-M00-1-0030 | 562.19 | OH | A02 | a1 | B03 | b1 | C04 | c1 | D01 |

(continued)

| [Table 10-17-3] Compound (indicated by ID) that may be contained in mixture 4-M00-1 | | | | | | | | | |
|---|---|---|---|---|---|---|---|---|---|
| ID | Exact Mass | | A | a | B | b | C | c | D |
| 4-M00-1-0031 | 572.23 | OH | A01 | a1 | B04 | b1 | C04 | c1 | D01 |
| 4-M00-1-0032 | 572.23 | OH | A02 | a1 | B04 | b1 | C04 | c1 | D01 |
| 4-M00-1-0033 | 614.12 | OH | A01 | a1 | B01 | b1 | C01 | c1 | D09 |
| 4-M00-1-0034 | 614.12 | OH | A02 | a1 | B01 | b1 | C01 | c1 | D09 |
| 4-M00-1-0035 | 628.13 | OH | A01 | a1 | B02 | b1 | C01 | c1 | D09 |
| 4-M00-1-0036 | 628.13 | OH | A02 | a1 | B02 | b1 | C01 | c1 | D09 |
| 4-M00-1-0037 | 632.11 | OH | A01 | a1 | B03 | b1 | C01 | c1 | D09 |
| 4-M00-1-0038 | 632.11 | OH | A02 | a1 | B03 | b1 | C01 | c1 | D09 |
| 4-M00-1-0039 | 642.15 | OH | A01 | a1 | B04 | b1 | C01 | c1 | D09 |
| 4-M00-1-0040 | 642.15 | OH | A02 | a1 | B04 | b1 | C01 | c1 | D09 |
| 4-M00-1-0041 | 614.12 | OH | A01 | a1 | B01 | b1 | C02 | c1 | D09 |
| 4-M00-1-0042 | 614.12 | OH | A02 | a1 | B01 | b1 | C02 | c1 | D09 |
| 4-M00-1-0043 | 628.13 | OH | A01 | a1 | B02 | b1 | C02 | c1 | D09 |
| 4-M00-1-0044 | 628.13 | OH | A02 | a1 | B02 | b1 | C02 | c1 | D09 |
| 4-M00-1-0045 | 632.11 | OH | A01 | a1 | B03 | b1 | C02 | c1 | D09 |
| 4-M00-1-0046 | 632.11 | OH | A02 | a1 | B03 | b1 | C02 | c1 | D09 |
| 4-M00-1-0047 | 642.15 | OH | A01 | a1 | B04 | b1 | C02 | c1 | D09 |
| 4-M00-1-0048 | 642.15 | OH | A02 | a1 | B04 | b1 | C02 | c1 | D09 |
| 4-M00-1-0049 | 618.09 | OH | A01 | a1 | B01 | b1 | C03 | c1 | D09 |
| 4-M00-1-0050 | 618.09 | OH | A02 | a1 | B01 | b1 | C03 | c1 | D09 |
| 4-M00-1-0051 | 632.11 | OH | A01 | a1 | B02 | b1 | C03 | c1 | D09 |
| 4-M00-1-0052 | 632.11 | OH | A02 | a1 | B02 | b1 | C03 | c1 | D09 |
| 4-M00-1-0053 | 636.08 | OH | A01 | a1 | B03 | b1 | C03 | c1 | D09 |
| 4-M00-1-0054 | 636.08 | OH | A02 | a1 | B03 | b1 | C03 | c1 | D09 |
| 4-M00-1-0055 | 646.12 | OH | A01 | a1 | B04 | b1 | C03 | c1 | D09 |
| 4-M00-1-0056 | 646.12 | OH | A02 | a1 | B04 | b1 | C03 | c1 | D09 |
| 4-M00-1-0057 | 628.13 | OH | A01 | a1 | B01 | b1 | C04 | c1 | D09 |
| 4-M00-1-0058 | 628.13 | OH | A02 | a1 | B01 | b1 | C04 | c1 | D09 |
| 4-M00-1-0059 | 642.15 | OH | A01 | a1 | B02 | b1 | C04 | c1 | D09 |
| 4-M00-1-0060 | 642.15 | OH | A02 | a1 | B02 | b1 | C04 | c1 | D09 |
| 4-M00-1-0061 | 646.12 | OH | A01 | a1 | B03 | b1 | C04 | c1 | D09 |
| 4-M00-1-0062 | 646.12 | OH | A02 | a1 | B03 | b1 | C04 | c1 | D09 |
| 4-M00-1-0063 | 656.17 | OH | A01 | a1 | B04 | b1 | C04 | c1 | D09 |
| 4-M00-1-0064 | 656.17 | OH | A02 | a1 | B04 | b1 | C04 | c1 | D09 |
| 4-M00-1-0065 | 622.18 | OH | A01 | a1 | B01 | b1 | C01 | c1 | D10 |
| 4-M00-1-0066 | 622.18 | OH | A02 | a1 | B01 | b1 | C01 | c1 | D10 |
| 4-M00-1-0067 | 636.19 | OH | A01 | a1 | B02 | b1 | C01 | c1 | D10 |
| 4-M00-1-0068 | 636.19 | OH | A02 | a1 | B02 | b1 | C01 | c1 | D10 |

(continued)

| [Table 10-17-3] Compound (indicated by ID) that may be contained in mixture 4-M00-1 | | | | | | | | | |
|---|---|---|---|---|---|---|---|---|---|
| ID | Exact Mass | | A | a | B | b | C | c | D |
| 4-M00-1-0069 | 640.17 | OH | A01 | a1 | B03 | b1 | C01 | c1 | D10 |
| 4-M00-1-0070 | 640.17 | OH | A02 | a1 | B03 | b1 | C01 | c1 | D10 |
| 4-M00-1-0071 | 650.21 | OH | A01 | a1 | B04 | b1 | C01 | c1 | D10 |
| 4-M00-1-0072 | 650.21 | OH | A02 | a1 | B04 | b1 | C01 | c1 | D10 |
| 4-M00-1-0073 | 622.18 | OH | A01 | a1 | B01 | b1 | C02 | c1 | D10 |
| 4-M00-1-0074 | 622.18 | OH | A02 | a1 | B01 | b1 | C02 | c1 | D10 |
| 4-M00-1-0075 | 636.19 | OH | A01 | a1 | B02 | b1 | C02 | c1 | D10 |
| 4-M00-1-0076 | 636.19 | OH | A02 | a1 | B02 | b1 | C02 | c1 | D10 |
| 4-M00-1-0077 | 640.17 | OH | A01 | a1 | B03 | b1 | C02 | c1 | D10 |
| 4-M00-1-0078 | 640.17 | OH | A02 | a1 | B03 | b1 | C02 | c1 | D10 |
| 4-M00-1-0079 | 650.21 | OH | A01 | a1 | B04 | b1 | C02 | c1 | D10 |
| 4-M00-1-0080 | 650.21 | OH | A02 | a1 | B04 | b1 | C02 | c1 | D10 |
| 4-M00-1-0081 | 626.15 | OH | A01 | a1 | B01 | b1 | C03 | c1 | D10 |
| 4-M00-1-0082 | 626.15 | OH | A02 | a1 | B01 | b1 | C03 | c1 | D10 |
| 4-M00-1-0083 | 640.17 | OH | A01 | a1 | B02 | b1 | C03 | c1 | D10 |
| 4-M00-1-0084 | 640.17 | OH | A02 | a1 | B02 | b1 | C03 | c1 | D10 |
| 4-M00-1-0085 | 644.14 | OH | A01 | a1 | B03 | b1 | C03 | c1 | D10 |
| 4-M00-1-0086 | 644.14 | OH | A02 | a1 | B03 | b1 | C03 | c1 | D10 |
| 4-M00-1-0087 | 654.18 | OH | A01 | a1 | B04 | b1 | C03 | c1 | D10 |
| 4-M00-1-0088 | 654.18 | OH | A02 | a1 | B04 | b1 | C03 | c1 | D10 |
| 4-M00-1-0089 | 636.19 | OH | A01 | a1 | B01 | b1 | C04 | c1 | D10 |
| 4-M00-1-0090 | 636.19 | OH | A02 | a1 | B01 | b1 | C04 | c1 | D10 |
| 4-M00-1-0091 | 650.21 | OH | A01 | a1 | B02 | b1 | C04 | c1 | D10 |
| 4-M00-1-0092 | 650.21 | OH | A02 | a1 | B02 | b1 | C04 | c1 | D10 |
| 4-M00-1-0093 | 654.18 | OH | A01 | a1 | B03 | b1 | C04 | c1 | D10 |
| 4-M00-1-0094 | 654.18 | OH | A02 | a1 | B03 | b1 | C04 | c1 | D10 |
| 4-M00-1-0095 | 664.22 | OH | A01 | a1 | B04 | b1 | C04 | c1 | D10 |
| 4-M00-1-0096 | 664.22 | OH | A02 | a1 | B04 | b1 | C04 | c1 | D10 |
| 4-M00-1-0097 | 638.17 | OH | A01 | a1 | B01 | b1 | C01 | c1 | D11 |
| 4-M00-1-0098 | 638.17 | OH | A02 | a1 | B01 | b1 | C01 | c1 | D11 |
| 4-M00-1-0099 | 652.19 | OH | A01 | a1 | B02 | b1 | C01 | c1 | D11 |
| 4-M00-1-0100 | 652.19 | OH | A02 | a1 | B02 | b1 | C01 | c1 | D11 |
| 4-M00-1-0101 | 656.16 | OH | A01 | a1 | B03 | b1 | C01 | c1 | D11 |
| 4-M00-1-0102 | 656.16 | OH | A02 | a1 | B03 | b1 | C01 | c1 | D11 |
| 4-M00-1-0103 | 666.20 | OH | A01 | a1 | B04 | b1 | C01 | c1 | D11 |
| 4-M00-1-0104 | 666.20 | OH | A02 | a1 | B04 | b1 | C01 | c1 | D11 |
| 4-M00-1-0105 | 638.17 | OH | A01 | a1 | B01 | b1 | C02 | c1 | D11 |

(continued)

| [Table 10-17-3] Compound (indicated by ID) that may be contained in mixture 4-M00-1 | | | | | | | | | |
|---|---|---|---|---|---|---|---|---|---|
| ID | Exact Mass | | A | a | B | b | C | c | D |
| 4-M00-1-0106 | 638.17 | OH | A02 | a1 | B01 | b1 | C02 | c1 | D11 |
| 4-M00-1-0107 | 652.19 | OH | A01 | a1 | B02 | b1 | C02 | c1 | D11 |
| 4-M00-1-0108 | 652.19 | OH | A02 | a1 | B02 | b1 | C02 | c1 | D11 |
| 4-M00-1-0109 | 656.16 | OH | A01 | a1 | B03 | b1 | C02 | c1 | D11 |
| 4-M00-1-0110 | 656.16 | OH | A02 | a1 | B03 | b1 | C02 | c1 | D11 |
| 4-M00-1-0111 | 666.20 | OH | A01 | a1 | B04 | b1 | C02 | c1 | D11 |
| 4-M00-1-0112 | 666.20 | OH | A02 | a1 | B04 | b1 | C02 | c1 | D11 |
| 4-M00-1-0113 | 642.15 | OH | A01 | a1 | B01 | b1 | C03 | c1 | D11 |
| 4-M00-1-0114 | 642.15 | OH | A02 | a1 | B01 | b1 | C03 | c1 | D11 |
| 4-M00-1-0115 | 656.16 | OH | A01 | a1 | B02 | b1 | C03 | c1 | D11 |
| 4-M00-1-0116 | 656.16 | OH | A02 | a1 | B02 | b1 | C03 | c1 | D11 |
| 4-M00-1-0117 | 660.14 | OH | A01 | a1 | B03 | b1 | C03 | c1 | D11 |
| 4-M00-1-0118 | 660.14 | OH | A02 | a1 | B03 | b1 | C03 | c1 | D11 |
| 4-M00-1-0119 | 670.18 | OH | A01 | a1 | B04 | b1 | C03 | c1 | D11 |
| 4-M00-1-0120 | 670.18 | OH | A02 | a1 | B04 | b1 | C03 | c1 | D11 |
| 4-M00-1-0121 | 652.19 | OH | A01 | a1 | B01 | b1 | C04 | c1 | D11 |
| 4-M00-1-0122 | 652.19 | OH | A02 | a1 | B01 | b1 | C04 | c1 | D11 |
| 4-M00-1-0123 | 666.20 | OH | A01 | a1 | B02 | b1 | C04 | c1 | D11 |
| 4-M00-1-0124 | 666.20 | OH | A02 | a1 | B02 | b1 | C04 | c1 | D11 |
| 4-M00-1-0125 | 670.18 | OH | A01 | a1 | B03 | b1 | C04 | c1 | D11 |
| 4-M00-1-0126 | 670.18 | OH | A02 | a1 | B03 | b1 | C04 | c1 | D11 |
| 4-M00-1-0127 | 680.22 | OH | A01 | a1 | B04 | b1 | C04 | c1 | D11 |
| 4-M00-1-0128 | 680.22 | OH | A02 | a1 | B04 | b1 | C04 | c1 | D11 |
| 4-M00-1-0129 | 508.20 | OH | A01 | a1 | B05 | b2 | COS | c2 | D05 |
| 4-M00-1-0130 | 508.20 | OH | A02 | a1 | B05 | b2 | C05 | c2 | D05 |
| 4-M00-1-0131 | 522.22 | OH | A01 | a1 | B06 | b2 | C05 | c2 | DOS |
| 4-M00-1-0132 | 522.22 | OH | A02 | a1 | B06 | b2 | C05 | c2 | D05 |
| 4-M00-1-0133 | 522.22 | OH | A01 | a1 | B07 | b2 | C05 | c2 | DOS |
| 4-M00-1-0134 | 522.22 | OH | A02 | a1 | B07 | b2 | C05 | c2 | D05 |
| 4-M00-1-0135 | 538.21 | OH | A01 | a1 | B08 | b2 | C05 | c2 | D05 |
| 4-M00-1-0136 | 538.21 | OH | A02 | a1 | B08 | b2 | COS | c2 | DOS |
| 4-M00-1-0137 | 509.20 | OH | A01 | a1 | B05 | b2 | C06 | c2 | D05 |
| 4-M00-1-0138 | 509.20 | OH | A02 | a1 | B05 | b2 | C06 | c2 | D05 |
| 4-M00-1-0139 | 523.21 | OH | A01 | a1 | B06 | b2 | C06 | c2 | D05 |
| 4-M00-1-0140 | 523.21 | OH | A02 | a1 | B06 | b2 | C06 | c2 | DOS |
| 4-M00-1-0141 | 523.21 | OH | A01 | a1 | B07 | b2 | C06 | c2 | D05 |
| 4-M00-1-0142 | 523.21 | OH | A02 | a1 | B07 | b2 | C06 | c2 | D05 |

(continued)

| [Table 10-17-3] Compound (indicated by ID) that may be contained in mixture 4-M00-1 | | | | | | | | | |
|---|---|---|---|---|---|---|---|---|---|
| ID | Exact Mass | | A | a | B | b | C | c | D |
| 4-M00-1-0143 | 539.21 | OH | A01 | a1 | B08 | b2 | C06 | c2 | DOS |
| 4-M00-1-0144 | 539.21 | OH | A02 | a1 | B08 | b2 | C06 | c2 | DOS |
| 4-M00-1-0145 | 522.22 | OH | A01 | a1 | B05 | b2 | C07 | c2 | D05 |
| 4-M00-1-0146 | 522.22 | OH | A02 | a1 | B05 | b2 | C07 | c2 | DOS |
| 4-M00-1-0147 | 536.23 | OH | A01 | a1 | B06 | b2 | C07 | c2 | DOS |
| 4-M00-1-0148 | 536.23 | OH | A02 | a1 | B06 | b2 | C07 | c2 | DOS |
| 4-M00-1-0149 | 536.23 | OH | A01 | a1 | B07 | b2 | C07 | c2 | DOS |
| 4-M00-1-0150 | 536.23 | OH | A02 | a1 | B07 | b2 | C07 | c2 | DOS |
| 4-M00-1-0151 | 552.23 | OH | A01 | a1 | B08 | b2 | C07 | c2 | DOS |
| 4-M00-1-0152 | 552.23 | OH | A02 | a1 | B08 | b2 | C07 | c2 | DOS |
| 4-M00-1-0153 | 472.20 | OH | A01 | a1 | B05 | b2 | C08 | c2 | DOS |
| 4-M00-1-0154 | 472.20 | OH | A02 | a1 | B05 | b2 | C08 | c2 | DOS |
| 4-M00-1-0155 | 486.22 | OH | A01 | a1 | B06 | b2 | C08 | c2 | DOS |
| 4-M00-1-0156 | 486.22 | OH | A02 | a1 | B06 | b2 | C08 | c2 | DOS |
| 4-M00-1-0157 | 486.22 | OH | A01 | a1 | B07 | b2 | C08 | c2 | DOS |
| 4-M00-1-0158 | 486.22 | OH | A02 | a1 | B07 | b2 | C08 | c2 | DOS |
| 4-M00-1-0159 | 502.21 | OH | A01 | a1 | B08 | b2 | C08 | c2 | DOS |
| 4-M00-1-0160 | 502.21 | OH | A02 | a1 | B08 | b2 | C08 | c2 | DOS |
| 4-M00-1-0161 | 534.14 | OH | A01 | a1 | B05 | b2 | C05 | c2 | D06 |
| 4-M00-1-0162 | 534.14 | OH | A02 | a1 | B05 | b2 | C05 | c2 | D06 |
| 4-M00-1-0163 | 548.16 | OH | A01 | a1 | B06 | b2 | C05 | c2 | D06 |
| 4-M00-1-0164 | 548.16 | OH | A02 | a1 | B06 | b2 | C05 | c2 | D06 |
| 4-M00-1-0165 | 548.16 | OH | A01 | a1 | B07 | b2 | C05 | c2 | D06 |
| 4-M00-1-0166 | 548.16 | OH | A02 | a1 | B07 | b2 | C05 | c2 | D06 |
| 4-M00-1-0167 | 564.15 | OH | A01 | a1 | B08 | b2 | C05 | c2 | D06 |
| 4-M00-1-0168 | 564.15 | OH | A02 | a1 | B08 | b2 | C05 | c2 | D06 |
| 4-M00-1-0169 | 535.14 | OH | A01 | a1 | B05 | b2 | C06 | c2 | D06 |
| 4-M00-1-0170 | 535.14 | OH | A02 | a1 | B05 | b2 | C06 | c2 | D06 |
| 4-M00-1-0171 | 549.15 | OH | A01 | a1 | B06 | b2 | C06 | c2 | D06 |
| 4-M00-1-0172 | 549.15 | OH | A02 | a1 | B06 | b2 | C06 | c2 | D06 |
| 4-M00-1-0173 | 549.15 | OH | A01 | a1 | B07 | b2 | C06 | c2 | D06 |
| 4-M00-1-0174 | 549.15 | OH | A02 | a1 | B07 | b2 | C06 | c2 | D06 |
| 4-M00-1-0175 | 565.15 | OH | A01 | a1 | B08 | b2 | C06 | c2 | D06 |
| 4-M00-1-0176 | 565.15 | OH | A02 | a1 | B08 | b2 | C06 | c2 | D06 |
| 4-M00-1-0177 | 548.16 | OH | A01 | a1 | B05 | b2 | C07 | c2 | D06 |
| 4-M00-1-0178 | 548.16 | OH | A02 | a1 | B05 | b2 | C07 | c2 | D06 |
| 4-M00-1-0179 | 562.17 | OH | A01 | a1 | B06 | b2 | C07 | c2 | D06 |

(continued)

| [Table 10-17-3] Compound (indicated by ID) that may be contained in mixture 4-M00-1 | | | | | | | | | |
|---|---|---|---|---|---|---|---|---|---|
| ID | Exact Mass | | A | a | B | b | C | c | D |
| 4-M00-1-0180 | 562.17 | OH | A02 | a1 | B06 | b2 | C07 | c2 | D06 |
| 4-M00-1-0181 | 562.17 | OH | A01 | a1 | B07 | b2 | C07 | c2 | D06 |
| 4-M00-1-0182 | 562.17 | OH | A02 | a1 | B07 | b2 | C07 | c2 | D06 |
| 4-M00-1-0183 | 578.17 | OH | A01 | a1 | B08 | b2 | C07 | c2 | D06 |
| 4-M00-1-0184 | 578.17 | OH | A02 | a1 | B08 | b2 | C07 | c2 | D06 |
| 4-M00-1-0185 | 498.14 | OH | A01 | a1 | B05 | b2 | C08 | c2 | D06 |
| 4-M00-1-0186 | 498.14 | OH | A02 | a1 | B05 | b2 | C08 | c2 | D06 |
| 4-M00-1-0187 | 512.16 | OH | A01 | a1 | B06 | b2 | C08 | c2 | D06 |
| 4-M00-1-0188 | 512.16 | OH | A02 | a1 | B06 | b2 | C08 | c2 | D06 |
| 4-M00-1-0189 | 512.16 | OH | A01 | a1 | B07 | b2 | C08 | c2 | D06 |
| 4-M00-1-0190 | 512.16 | OH | A02 | a1 | B07 | b2 | C08 | c2 | D06 |
| 4-M00-1-0191 | 528.15 | OH | A01 | a1 | B08 | b2 | C08 | c2 | D06 |
| 4-M00-1-0192 | 528.15 | OH | A02 | a1 | B08 | b2 | C08 | c2 | D06 |
| 4-M00-1-0193 | 542.18 | OH | A01 | a1 | B05 | b2 | C05 | c2 | D07 |
| 4-M00-1-0194 | 542.18 | OH | A02 | a1 | B05 | b2 | COS | c2 | D07 |
| 4-M00-1-0195 | 556.20 | OH | A01 | a1 | B06 | b2 | COS | c2 | D07 |
| 4-M00-1-0196 | 556.20 | OH | A02 | a1 | B06 | b2 | C05 | c2 | D07 |
| 4-M00-1-0197 | 556.20 | OH | A01 | a1 | B07 | b2 | C05 | c2 | D07 |
| 4-M00-1-0198 | 556.20 | OH | A02 | a1 | B07 | b2 | C05 | c2 | D07 |
| 4-M00-1-0199 | 572.19 | OH | A01 | a1 | B08 | b2 | C05 | c2 | D07 |
| 4-M00-1-0200 | 572.19 | OH | A02 | a1 | B08 | b2 | C05 | c2 | D07 |
| 4-M00-1-0201 | 543.18 | OH | A01 | a1 | B05 | b2 | C06 | c2 | D07 |
| 4-M00-1-0202 | 543.18 | OH | A02 | a1 | B05 | b2 | C06 | c2 | D07 |
| 4-M00-1-0203 | 557.20 | OH | A01 | a1 | B06 | b2 | C06 | c2 | D07 |
| 4-M00-1-0204 | 557.20 | OH | A02 | a1 | B06 | b2 | C06 | c2 | D07 |
| 4-M00-1-0205 | 557.20 | OH | A01 | a1 | B07 | b2 | C06 | c2 | D07 |
| 4-M00-1-0206 | 557.20 | OH | A02 | a1 | B07 | b2 | C06 | c2 | D07 |
| 4-M00-1-0207 | 573.19 | OH | A01 | a1 | B08 | b2 | C06 | c2 | D07 |
| 4-M00-1-0208 | 573.19 | OH | A02 | a1 | B08 | b2 | C06 | c2 | D07 |
| 4-M00-1-0209 | 556.20 | OH | A01 | a1 | B05 | b2 | C07 | c2 | D07 |
| 4-M00-1-0210 | 556.20 | OH | A02 | a1 | B05 | b2 | C07 | c2 | D07 |
| 4-M00-1-0211 | 570.22 | OH | A01 | a1 | B06 | b2 | C07 | c2 | D07 |
| 4-M00-1-0212 | 570.22 | OH | A02 | a1 | B06 | b2 | C07 | c2 | D07 |
| 4-M00-1-0213 | 570.22 | OH | A01 | a1 | B07 | b2 | C07 | c2 | D07 |
| 4-M00-1-0214 | 570.22 | OH | A02 | a1 | B07 | b2 | C07 | c2 | D07 |
| 4-M00-1-0215 | 586.21 | OH | A01 | a1 | B08 | b2 | C07 | c2 | D07 |
| 4-M00-1-0216 | 586.21 | OH | A02 | a1 | B08 | b2 | C07 | c2 | D07 |

(continued)

| [Table 10-17-3] Compound (indicated by ID) that may be contained in mixture 4-M00-1 | | | | | | | | | |
|---|---|---|---|---|---|---|---|---|---|
| ID | Exact Mass | | A | a | B | b | C | c | D |
| 4-M00-1-0217 | 506.18 | OH | A01 | a1 | B05 | b2 | C08 | c2 | D07 |
| 4-M00-1-0218 | 506.18 | OH | A02 | a1 | B05 | b2 | C08 | c2 | D07 |
| 4-M00-1-0219 | 520.20 | OH | A01 | a1 | B06 | b2 | C08 | c2 | D07 |
| 4-M00-1-0220 | 520.20 | OH | A02 | a1 | B06 | b2 | C08 | c2 | D07 |
| 4-M00-1-0221 | 520.20 | OH | A01 | a1 | B07 | b2 | C08 | c2 | D07 |
| 4-M00-1-0222 | 520.20 | OH | A02 | a1 | B07 | b2 | C08 | c2 | D07 |
| 4-M00-1-0223 | 536.19 | OH | A01 | a1 | B08 | b2 | C08 | c2 | D07 |
| 4-M00-1-0224 | 536.19 | OH | A02 | a1 | B08 | b2 | C08 | c2 | D07 |
| 4-M00-1-0225 | 570.22 | OH | A01 | a1 | B05 | b2 | COS | c2 | D08 |
| 4-M00-1-0226 | 570.22 | OH | A02 | a1 | B05 | b2 | C05 | c2 | D08 |
| 4-M00-1-0227 | 584.23 | OH | A01 | a1 | B06 | b2 | C05 | c2 | D08 |
| 4-M00-1-0228 | 584.23 | OH | A02 | a1 | B06 | b2 | C05 | c2 | D08 |
| 4-M00-1-0229 | 584.23 | OH | A01 | a1 | B07 | b2 | C05 | c2 | D08 |
| 4-M00-1-0230 | 584.23 | OH | A02 | a1 | B07 | b2 | C05 | c2 | D08 |
| 4-M00-1-0231 | 600.23 | OH | A01 | a1 | B08 | b2 | C05 | c2 | D08 |
| 4-M00-1-0232 | 600.23 | OH | A02 | a1 | B08 | b2 | COS | c2 | D08 |
| 4-M00-1-0233 | 571.21 | OH | A01 | a1 | B05 | b2 | C06 | c2 | D08 |
| 4-M00-1-0234 | 571.21 | OH | A02 | a1 | B05 | b2 | C06 | c2 | D08 |
| 4-M00-1-0235 | 585.23 | OH | A01 | a1 | B06 | b2 | C06 | c2 | D08 |
| 4-M00-1-0236 | 585.23 | OH | A02 | a1 | B06 | b2 | C06 | c2 | D08 |
| 4-M00-1-0237 | 585.23 | OH | A01 | a1 | B07 | b2 | C06 | c2 | D08 |
| 4-M00-1-0238 | 585.23 | OH | A02 | a1 | B07 | b2 | C06 | c2 | D08 |
| 4-M00-1-0239 | 601.22 | OH | A01 | a1 | B08 | b2 | C06 | c2 | D08 |
| 4-M00-1-0240 | 601.22 | OH | A02 | a1 | B08 | b2 | C06 | c2 | D08 |
| 4-M00-1-0241 | 584.23 | OH | A01 | a1 | B05 | b2 | C07 | c2 | D08 |
| 4-M00-1-0242 | 584.23 | OH | A02 | a1 | B05 | b2 | C07 | c2 | D08 |
| 4-M00-1-0243 | 598.25 | OH | A01 | a1 | B06 | b2 | C07 | c2 | D08 |
| 4-M00-1-0244 | 598.25 | OH | A02 | a1 | B06 | b2 | C07 | c2 | D08 |
| 4-M00-1-0245 | 598.25 | OH | A01 | a1 | B07 | b2 | C07 | c2 | D08 |
| 4-M00-1-0246 | 598.25 | OH | A02 | a1 | B07 | b2 | C07 | c2 | D08 |
| 4-M00-1-0247 | 614.24 | OH | A01 | a1 | B08 | b2 | C07 | c2 | D08 |
| 4-M00-1-0248 | 614.24 | OH | A02 | a1 | B08 | b2 | C07 | c2 | D08 |
| 4-M00-1-0249 | 534.22 | OH | A01 | a1 | B05 | b2 | C08 | c2 | D08 |
| 4-M00-1-0250 | 534.22 | OH | A02 | a1 | B05 | b2 | C08 | c2 | D08 |
| 4-M00-1-0251 | 548.23 | OH | A01 | a1 | B06 | b2 | C08 | c2 | D08 |
| 4-M00-1-0252 | 548.23 | OH | A02 | a1 | B06 | b2 | C08 | c2 | D08 |
| 4-M00-1-0253 | 548.23 | OH | A01 | a1 | B07 | b2 | C08 | c2 | D08 |

(continued)

| [Table 10-17-3] Compound (indicated by ID) that may be contained in mixture 4-M00-1 | | | | | | | | | |
|---|---|---|---|---|---|---|---|---|---|
| ID | Exact Mass | | A | a | B | b | C | c | D |
| 4-M00-1-0254 | 548.23 | OH | A02 | a1 | B07 | b2 | C08 | c2 | D08 |
| 4-M00-1-0255 | 564.23 | OH | A01 | a1 | B08 | b2 | C08 | c2 | D08 |
| 4-M00-1-0256 | 564.23 | OH | A02 | a1 | B08 | b2 | C08 | c2 | D08 |

The compound names of the compounds that may be contained in mixture 4-M00-1 are shown in Table 10-17-4.

[Table 586]

[Table 10-17-4] Compound that may be contained in mixture 4-M00-1

| No. of compound that may be contained | Compound name |
|---|---|
| 4-M00-1-0001 | 4-(4-Hydroxyphenyl)-N-[3-[[3-methyl-5-(propan-2-ylsulfonylamino)phenyl]methoxy]phenyl]benzamide |
| 4-M00-1-0002 | 3-(4-Hydroxyphenyl)-N-[3-[[3-methyl-5-(propan-2-ylsulfonylamino)phenyl]methoxy]phenyl]benzamide |
| 4-M00-1-0003 | 4-(4-Hydroxyphenyl)-N-[3-methyl-4-[[3-methyl-5-(propan-2-ylsulfonylamino)phenyl]methoxy]phenyl]benzamide |
| 4-M00-1-0004 | 3-(4-Hydroxyphenyl)-N-[3-methyl-4-[[3-methyl-5-(propan-2-ylsulfonylamino)phenyl]methoxy]phenyl]benzamide |
| 4-M00-1-0005 | N-[3-Fluoro-4-[[3-methyl-5-(propan-2-ylsulfonylamino)phenyl]methoxy]phenyl]-4-(4-hydroxyphenyl)benzamide |
| 4-M00-1-0006 | N-[3-Fluoro-4-[[3-methyl-5-(propan-2-ylsulfonylamino)phenyl]methoxy]phenyl]-3-(4-hydroxyphenyl)benzamide |
| 4-M00-1-0007 | 4-(4-Hydroxyphenyl)-N-[[3-methyl-4-[[3-methyl-5-(propan-2-ylsulfonylamino)phenyl]methoxy]phenyl]methyl]benzamide |
| 4-M00-1-0008 | 3-(4-Hydroxyphenyl)-N-[[3-methyl-4-[[3-methyl-5-(propan-2-ylsulfonylamino)phenyl]methoxy]phenyl]methyl]benzamide |
| 4-M00-1-0009 | 4-(4-Hydroxyphenyl)-N-[3-[2-[3-(propan-2-ylsulfonylamino)phenyl]ethoxy]phenyl]benzamide |
| 4-M00-1-0010 | 3-(4-Hydroxyphenyl)-N-[3-[2-[3-(propan-2-ylsulfonylamino)phenyl]ethoxy]phenyl]benzamide |
| 4-M00-1-0011 | 4-(4-Hydroxyphenyl)-N-[3-methyl-4-[2-[3-(propan-2-ylsulfonylamino)phenyl]ethoxy]phenyl]benzamide |
| 4-M00-1-0012 | 3-(4-Hydroxyphenyl)-N-[3-methyl-4-[2-[3-(propan-2-ylsulfonylamino)phenyl]ethoxy]phenyl]benzamide |
| 4-M00-1-0013 | N-[3-Fluoro-4-[2-[3-(propan-2-ylsulfonylamino)phenyl]ethoxy]phenyl]-4-(4-hydroxyphenyl)benzamide |
| 4-M00-1-0014 | N-[3-Fluoro-4-[2-[3-(propan-2-ylsulfonylamino)phenyl]ethoxy]phenyl]-3-(4-hydroxyphenyl)benzamide |
| 4-M00-1-0015 | 4-(4-Hydroxyphenyl)-N-[[3-methyl-4-[2-[3-(propan-2-ylsulfonylamino)phenyl]ethoxy]phenyl]methyl]benzamide |
| 4-M00-1-0016 | 3-(4-Hydroxyphenyl)-N-[[3-methyl-4-[2-[3-(propan-2-ylsulfonylamino)phenyl]ethoxy]phenyl]methyl]benzamide |
| 4-M00-1-0017 | N-[3-[[2-Fluoro-3-(propan-2-ylsulfonylamino)phenyl]methoxy]phenyl]-4-(4-hydroxyphenyl)benzamide |
| 4-M00-1-0018 | N-[3-[[2-Fluoro-3-(propan-2-ylsulfonylamino)phenyl]methoxy]phenyl]-3-(4-hydroxyphenyl)benzamide |
| 4-M00-1-0019 | N-[4-[[2-Fluoro-3-(propan-2-ylsulfonylamino)phenyl]methoxy]-3-methylphenyl]-4-(4-hydroxyphenyl)benzamide |
| 4-M00-1-0020 | N-[4-[[2-Fluoro-3-(propan-2-ylsulfonylamino)phenyl]methoxy]-3-methylphenyl]-3-(4-hydroxyphenyl)benzamide |
| 4-M00-1-0021 | N-[3-Fluoro-4-[[2-fluoro-3-(propan-2-ylsulfonylamino)phenyl]methoxy]phenyl]-4-(4-hydroxyphenyl)benzamide |
| 4-M00-1-0022 | N-[3-Fluoro-4-[[2-fluoro-3-(propan-2-ylsulfonylamino)phenyl]methoxy]phenyl]-3-(4-hydroxyphenyl)benzamide |

| | |
|---|---|
| 4-M00-1-0023 | N-[[4-[[2-Fluoro-3-(propan-2-ylsulfonylamino)phenyl]methoxy]-3-methylphenyl]methyl]-4-(4-hydroxyphenyl)benzamide |
| 4-M00-1-0024 | N-[[4-[[2-Fluoro-3-(propan-2-ylsulfonylamino)phenyl]methoxy]-3-methylphenyl]methyl]-3-(4-hydroxyphenyl)benzamide |
| 4-M00-1-0025 | 4-(4-Hydroxyphenyl)-N-[3-[3-[3-(propan-2-ylsulfonylamino)phenyl]propoxy]phenyl]benzamide |
| 4-M00-1-0026 | 3-(4-Hydroxyphenyl)-N-[3-[3-[3-(propan-2-ylsulfonylamino)phenyl]propoxy]phenyl]benzamide |
| 4-M00-1-0027 | 4-(4-Hydroxyphenyl)-N-[3-methyl-4-[3-[3-(propan-2-ylsulfonylamino)phenyl]propoxy]phenyl]benzamide |
| 4-M00-1-0028 | 3-(4-hydroxyphenyl)-N-[3-methyl-4-[3-[3-(propan-2-ylsulfonylamino)phenyl]propoxy]phenyl]benzamide |
| 4-M00-1-0029 | N-[3-Fluoro-4-[3-[3-(propan-2-ylsulfonylamino)phenyl]propoxy]phenyl]-4-(4-hydroxyphenyl)benzamide |
| 4-M00-1-0030 | N-[3-Fluoro-4-[3-[3-(propan-2-ylsulfonylamino)phenyl]propoxy]phenyl]-3-(4-hydroxyphenyl)benzamide |
| 4-M00-1-0031 | 4-(4-Hydroxyphenyl)-N-[[3-methyl-4-[3-[3-(propan-2-ylsulfonylamino)phenyl]propoxy]phenyl]methyl]benzamide |
| 4-M00-1-0032 | 3-(4-Hydroxyphenyl)-N-[[3-methyl-4-[3-[3-(propan-2-ylsulfonylamino)phenyl]propoxy]phenyl]methyl]benzamide |
| 4-M00-1-0033 | 3-[[3-[[3-[[4-(4-Hydroxyphenyl)benzoyl]amino]phenoxy]methyl]-5-methylphenyl]sulfamoyl]thiophene-2-carboxylic acid |
| 4-M00-1-0034 | 3-[[3-[[3-[[3-(4-Hydroxyphenyl)benzoyl]amino]phenoxy]methyl]-5-methylphenyl]sulfamoyl]thiophene-2-carboxylic acid |
| 4-M00-1-0035 | 3-[[3-[[4-[[4-(4-Hydroxyphenyl)benzoyl]amino]-2-methylphenoxy]methyl]-5-methylphenyl]sulfamoyl]thiophene-2-carboxylic acid |
| 4-M00-1-0036 | 3-[[3-[[4-[[3-(4-Hydroxyphenyl)benzoyl]amino]-2-methylphenoxy]methyl]-5-methylphenyl]sulfamoyl]thiophene-2-carboxylic acid |
| 4-M00-1-0037 | 3-[[3-[[2-Fluoro-4-[[4-(4-hydroxyphenyl)benzoyl]amino]phenoxy]methyl]-5-methylphenyl]sulfamoyl]thiophene-2-carboxylic acid |
| 4-M00-1-0038 | 3-[[3-[[2-Fluoro-4-[[3-(4-hydroxyphenyl)benzoyl]amino]phenoxy]methyl]-5-methylphenyl]sulfamoyl]thiophene-2-carboxylic acid |
| 4-M00-1-0039 | 3-[[3-[[4-[[[4-(4-Hydroxyphenyl)benzoyl]amino]methyl]-2-methylphenoxy]methyl]-5-methylphenyl]sulfamoyl]thiophene-2-carboxylic acid |
| 4-M00-1-0040 | 3-[[3-[[4-[[[3-(4-Hydroxyphenyl)benzoyl]amino]methyl]-2-methylphenoxy]methyl]-5-methylphenyl]sulfamoyl]thiophene-2-carboxylic acid |
| 4-M00-1-0041 | 3-[[3-[2-[3-[[4-(4-Hydroxyphenyl)benzoyl]amino]phenoxy]ethyl]phenyl]sulfamoyl]thiophene-2-carboxylic acid |
| 4-M00-1-0042 | 3-[[3-[2-[3-[[3-(4-Hydroxyphenyl)benzoyl]amino]phenoxy]ethyl]phenyl]sulfamoyl]thiophene-2-carboxylic acid |
| 4-M00-1-0043 | 3-[[3-[2-[4-[[4-(4-Hydroxyphenyl)benzoyl]amino]-2-methylphenoxy]ethyl]phenyl]sulfamoyl]thiophene-2-carboxylic acid |
| 4-M00-1-0044 | 3-[[3-[2-[4-[[3-(4-Hydroxyphenyl)benzoyl]amino]-2-methylphenoxy]ethyl]phenyl]sulfamoyl]thiophene-2-carboxylic acid |
| 4-M00-1-0045 | 3-[[3-[2-[2-Fluoro-4-[[4-(4-hydroxyphenyl)benzoyl]amino]phenoxy]ethyl]phenyl]sulfamoyl]thiophene-2-carboxylic acid |
| 4-M00-1-0046 | 3-[[3-[2-[2-Fluoro-4-[[3-(4-hydroxyphenyl)benzoyl]amino]phenoxy]ethyl]phenyl]sulfamoyl]thiophene-2-carboxylic acid |

| | |
|---|---|
| 4-M00-1-0047 | 3-[[3-[2-[4-[[[4-(4-Hydroxyphenyl)benzoyl]amino]methyl]-2-methylphenoxy]ethyl]phenyl]sulfamoyl]thiophene-2-carboxylic acid |
| 4-M00-1-0048 | 3-[[3-[2-[4-[[[3-(4-Hydroxyphenyl)benzoyl]amino]methyl]-2-methylphenoxy]ethyl]phenyl]sulfamoyl]thiophene-2-carboxylic acid |
| 4-M00-1-0049 | 3-[[2-Fluoro-3-[[3-[[4-(4-hydroxyphenyl)benzoyl]amino]phenoxy]methyl]phenyl]sulfamoyl]thiophene-2-carboxylic acid |
| 4-M00-1-0050 | 3-[[2-Fluoro-3-[[3-[[3-(4-hydroxyphenyl)benzoyl]amino]phenoxy]methyl]phenyl]sulfamoyl]thiophene-2-carboxylic acid |
| 4-M00-1-0051 | 3-[[2-Fluoro-3-[[4-[[4-(4-hydroxyphenyl)benzoyl]amino]-2-methylphenoxy]methyl]phenyl]sulfamoyl]thiophene-2-carboxylic acid |
| 4-M00-1-0052 | 3-[[2-Fluoro-3-[[4-[[3-(4-hydroxyphenyl)benzoyl]amino]-2-methylphenoxy]methyl]phenyl]sulfamoyl]thiophene-2-carboxylic acid |
| 4-M00-1-0053 | 3-[[2-Fluoro-3-[[2-fluoro-4-[[4-(4-hydroxyphenyl)benzoyl]amino]phenoxy]methyl]phenyl]sulfamoyl]thiophene-2-carboxylic acid |
| 4-M00-1-0054 | 3-[[2-Fluoro-3-[[2-fluoro-4-[[3-(4-hydroxyphenyl)benzoyl]amino]phenoxy]methyl]phenyl]sulfamoyl]thiophene-2-carboxylic acid |
| 4-M00-1-0055 | 3-[[2-Fluoro-3-[[4-[[[4-(4-hydroxyphenyl)benzoyl]amino]methyl]-2-methylphenoxy]methyl]phenyl]sulfamoyl]thiophene-2-carboxylic acid |
| 4-M00-1-0056 | 3-[[2-Fluoro-3-[[4-[[[3-(4-hydroxyphenyl)benzoyl]amino]methyl]-2-methylphenoxy]methyl]phenyl]sulfamoyl]thiophene-2-carboxylic acid |
| 4-M00-1-0057 | 3-[[3-[3-[3-[[4-(4-Hydroxyphenyl)benzoyl]amino]phenoxy]propyl]phenyl]sulfamoyl]thiophene-2-carboxylic acid |
| 4-M00-1-0058 | 3-[[3-[3-[3-[[3-(4-Hydroxyphenyl)benzoyl]amino]phenoxy]propyl]phenyl]sulfamoyl]thiophene-2-carboxylic acid |
| 4-M00-1-0059 | 3-[[3-[3-[4-[[4-(4-Hydroxyphenyl)benzoyl]amino]-2-methylphenoxy]propyl]phenyl]sulfamoyl]thiophene-2-carboxylic acid |
| 4-M00-1-0060 | 3-[[3-[3-[4-[[3-(4-Hydroxyphenyl)benzoyl]amino]-2-methylphenoxy]propyl]phenyl]sulfamoyl]thiophene-2-carboxylic acid |
| 4-M00-1-0061 | 3-[[3-[3-[2-Fluoro-4-[[4-(4-hydroxyphenyl)benzoyl]amino]phenoxy]propyl]phenyl]sulfamoyl]thiophene-2-carboxylic acid |
| 4-M00-1-0062 | 3-[[3-[3-[2-Fluoro-4-[[3-(4-hydroxyphenyl)benzoyl]amino]phenoxy]propyl]phenyl]sulfamoyl]thiophene-2-carboxylic acid |
| 4-M00-1-0063 | 3-[[3-[3-[4-[[[4-(4-Hydroxyphenyl)benzoyl]amino]methyl]-2-methylphenoxy]propyl]phenyl]sulfamoyl]thiophene-2-carboxylic acid |
| 4-M00-1-0064 | 3-[[3-[3-[4-[[[3-(4-Hydroxyphenyl)benzoyl]amino]methyl]-2-methylphenoxy]propyl]phenyl]sulfamoyl]thiophene-2-carboxylic acid |
| 4-M00-1-0065 | 2-[[3-[[3-[[4-(4-Hydroxyphenyl)benzoyl]amino]phenoxy]methyl]-5-methylphenyl]sulfamoylmethyl]benzoic acid |
| 4-M00-1-0066 | 2-[[3-[[3-[[3-(4-Hydroxyphenyl)benzoyl]amino]phenoxy]methyl]-5-methylphenyl]sulfamoylmethyl]benzoic acid |
| 4-M00-1-0067 | 2-[[3-[[4-[[4-(4-Hydroxyphenyl)benzoyl]amino]-2-methylphenoxy]methyl]-5-methylphenyl]sulfamoylmethyl]benzoic acid |
| 4-M00-1-0068 | 2-[[3-[[4-[[3-(4-Hydroxyphenyl)benzoyl]amino]-2-methylphenoxy]methyl]-5-methylphenyl]sulfamoylmethyl]benzoic acid |

| | |
|---|---|
| 4-M00-1-0069 | 2-[[3-[[2-Fluoro-4-[[4-(4-hydroxyphenyl)benzoyl]amino]phenoxy]methyl]-5-methylphenyl]sulfamoylmethyl]benzoic acid |
| 4-M00-1-0070 | 2-[[3-[[2-Fluoro-4-[[3-(4-hydroxyphenyl)benzoyl]amino]phenoxy]methyl]-5-methylphenyl]sulfamoylmethyl]benzoic acid |
| 4-M00-1-0071 | 2-[[3-[[4-[[[4-(4-Hydroxyphenyl)benzoyl]amino]methyl]-2-methylphenoxy]methyl]-5-methylphenyl]sulfamoylmethyl]benzoic acid |
| 4-M00-1-0072 | 2-[[3-[[4-[[[3-(4-Hydroxyphenyl)benzoyl]amino]methyl]-2-methylphenoxy]methyl]-5-methylphenyl]sulfamoylmethyl]benzoic acid |
| 4-M00-1-0073 | 2-[[3-[2-[3-[[4-(4-Hydroxyphenyl)benzoyl]amino]phenoxy]ethyl]phenyl]sulfamoylmethyl]benzoic acid |
| 4-M00-1-0074 | 2-[[3-[2-[3-[[3-(4-Hydroxyphenyl)benzoyl]amino]phenoxy]ethyl]phenyl]sulfamoylmethyl]benzoic acid |
| 4-M00-1-0075 | 2-[[3-[2-[4-[[4-(4-Hydroxyphenyl)benzoyl]amino]-2-methylphenoxy]ethyl]phenyl]sulfamoylmethyl]benzoic acid |
| 4-M00-1-0076 | 2-[[3-[2-[4-[[3-(4-Hydroxyphenyl)benzoyl]amino]-2-methylphenoxy]ethyl]phenyl]sulfamoylmethyl]benzoic acid |
| 4-M00-1-0077 | 2-[[3-[2-[2-Fluoro-4-[[4-(4-hydroxyphenyl)benzoyl]amino]phenoxy]ethyl]phenyl]sulfamoylmethyl]benzoic acid |
| 4-M00-1-0078 | 2-[[3-[2-[2-Fluoro-4-[[3-(4-hydroxyphenyl)benzoyl]amino]phenoxy]ethyl]phenyl]sulfamoylmethyl]benzoic acid |
| 4-M00-1-0079 | 2-[[3-[2-[4-[[[4-(4-Hydroxyphenyl)benzoyl]amino]methyl]-2-methylphenoxy]ethyl]phenyl]sulfamoylmethyl]benzoic acid |
| 4-M00-1-0080 | 2-[[3-[2-[4-[[[3-(4-Hydroxyphenyl)benzoyl]amino]methyl]-2-methylphenoxy]ethyl]phenyl]sulfamoylmethyl]benzoic acid |
| 4-M00-1-0081 | 2-[[2-Fluoro-3-[[3-[[4-(4-hydroxyphenyl)benzoyl]amino]phenoxy]methyl]phenyl]sulfamoylmethyl]benzoic acid |
| 4-M00-1-0082 | 2-[[2-Fluoro-3-[[3-[[3-(4-hydroxyphenyl)benzoyl]amino]phenoxy]methyl]phenyl]sulfamoylmethyl]benzoic acid |
| 4-M00-1-0083 | 2-[[2-Fluoro-3-[[4-[[4-(4-hydroxyphenyl)benzoyl]amino]-2-methylphenoxy]methyl]phenyl]sulfamoylmethyl]benzoic acid |
| 4-M00-1-0084 | 2-[[2-Fluoro-3-[[4-[[3-(4-hydroxyphenyl)benzoyl]amino]-2-methylphenoxy]methyl]phenyl]sulfamoylmethyl]benzoic acid |
| 4-M00-1-0085 | 2-[[2-Fluoro-3-[[2-fluoro-4-[[4-(4-hydroxyphenyl)benzoyl]amino]phenoxy]methyl]phenyl]sulfamoylmethyl]benzoic acid |
| 4-M00-1-0086 | 2-[[2-Fluoro-3-[[2-fluoro-4-[[3-(4-hydroxyphenyl)benzoyl]amino]phenoxy]methyl]phenyl]sulfamoylmethyl]benzoic acid |
| 4-M00-1-0087 | 2-[[2-Fluoro-3-[[4-[[[4-(4-hydroxyphenyl)benzoyl]amino]methyl]-2-methylphenoxy]methyl]phenyl]sulfamoylmethyl]benzoic acid |
| 4-M00-1-0088 | 2-[[2-Fluoro-3-[[4-[[[3-(4-hydroxyphenyl)benzoyl]amino]methyl]-2-methylphenoxy]methyl]phenyl]sulfamoylmethyl]benzoic acid |
| 4-M00-1-0089 | 2-[[3-[3-[3-[[4-(4-Hydroxyphenyl)benzoyl]amino]phenoxy]propyl]phenyl]sulfamoylmethyl]benzoic acid |

| | |
|---|---|
| 4-M00-1-0090 | 2-[[3-[3-[3-[[3-(4-Hydroxyphenyl)benzoyl]amino]phenoxy]propyl]phenyl]sulfamoylmethyl]benzoic acid |
| 4-M00-1-0091 | 2-[[3-[3-[4-[[4-(4-Hydroxyphenyl)benzoyl]amino]-2-methylphenoxy]propyl]phenyl]sulfamoylmethyl]benzoic acid |
| 4-M00-1-0092 | 2-[[3-[3-[2-Fluoro-4-[[4-(4-hydroxyphenyl)benzoyl]amino]phenoxy]propyl]phenyl]sulfamoylmethyl]benzoic acid |
| 4-M00-1-0093 | 2-[[3-[3-[2-Fluoro-4-[[4-(4-hydroxyphenyl)benzoyl]amino]phenoxy]propyl]phenyl]sulfamoylmethyl]benzoic acid |
| 4-M00-1-0094 | 2-[[3-[3-[2-Fluoro-4-[[3-(4-hydroxyphenyl)benzoyl]amino]phenoxy]propyl]phenyl]sulfamoylmethyl]benzoic acid |
| 4-M00-1-0095 | 2-[[3-[3-[4-[[[4-(4-Hydroxyphenyl)benzoyl]amino]methyl]-2-methylphenoxy]propyl]phenyl]sulfamoylmethyl]benzoic acid |
| 4-M00-1-0096 | 2-[[3-[3-[4-[[[3-(4-Hydroxyphenyl)benzoyl]amino]methyl]-2-methylphenoxy]propyl]phenyl]sulfamoylmethyl]benzoic acid |
| 4-M00-1-0097 | 5-[[3-[[3-[[4-(4-Hydroxyphenyl)benzoyl]amino]phenoxy]methyl]-5-methylphenyl]sulfamoyl]-2-methoxybenzoic acid |
| 4-M00-1-0098 | 5-[[3-[[3-[[3-(4-Hydroxyphenyl)benzoyl]amino]phenoxy]methyl]-5-methylphenyl]sulfamoyl]-2-methoxybenzoic acid |
| 4-M00-1-0099 | 5-[[3-[[4-[[4-(4-Hydroxyphenyl)benzoyl]amino]-2-methylphenoxy]methyl]-5-methylphenyl]sulfamoyl]-2-methoxybenzoic acid |
| 4-M00-1-0100 | 5-[[3-[[4-[[3-(4-Hydroxyphenyl)benzoyl]amino]-2-methylphenoxy]methyl]-5-methylphenyl]sulfamoyl]-2-methoxybenzoic acid |
| 4-M00-1-0101 | 5-[[3-[[2-Fluoro-4-[[4-(4-hydroxyphenyl)benzoyl]amino]phenoxy]methyl]-5-methylphenyl]sulfamoyl]-2-methoxybenzoic acid |
| 4-M00-1-0102 | 5-[[3-[[2-Fluoro-4-[[3-(4-hydroxyphenyl)benzoyl]amino]phenoxy]methyl]-5-methylphenyl]sulfamoyl]-2-methoxybenzoic acid |
| 4-M00-1-0103 | 5-[[3-[[4-[[[4-(4-Hydroxyphenyl)benzoyl]amino]methyl]-2-methylphenoxy]methyl]-5-methylphenyl]sulfamoyl]-2-methoxybenzoic acid |
| 4-M00-1-0104 | 5-[[3-[[4-[[[3-(4-Hydroxyphenyl)benzoyl]amino]methyl]-2-methylphenoxy]methyl]-5-methylphenyl]sulfamoyl]-2-methoxybenzoic acid |
| 4-M00-1-0105 | 5-[[3-[2-[3-[[4-(4-Hydroxyphenyl)benzoyl]amino]phenoxy]ethyl]phenyl]sulfamoyl]-2-methoxybenzoic acid |
| 4-M00-1-0106 | 5-[[3-[2-[3-[[3-(4-Hydroxyphenyl)benzoyl]amino]phenoxy]ethyl]phenyl]sulfamoyl]-2-methoxybenzoic acid |
| 4-M00-1-0107 | 5-[[3-[2-[4-[[4-(4-Hydroxyphenyl)benzoyl]amino]-2-methylphenoxy]ethyl]phenyl]sulfamoyl]-2-methoxybenzoic acid |
| 4-M00-1-0108 | 5-[[3-[2-[4-[[3-(4-Hydroxyphenyl)benzoyl]amino]-2-methylphenoxy]ethyl]phenyl]sulfamoyl]-2-methoxybenzoic acid |
| 4-M00-1-0109 | 5-[[3-[2-[2-Fluoro-4-[[4-(4-hydroxyphenyl)benzoyl]amino]phenoxy]ethyl]phenyl]sulfamoyl]-2-methoxybenzoic acid |
| 4-M00-1-0110 | 5-[[3-[2-[2-Fluoro-4-[[3-(4-hydroxyphenyl)benzoyl]amino]phenoxy]ethyl]phenyl]sulfamoyl]-2-methoxybenzoic acid |
| 4-M00-1-0111 | 5-[[3-[2-[4-[[[4-(4-Hydroxyphenyl)benzoyl]amino]methyl]-2-methylphenoxy]ethyl]phenyl]sulfamoyl]-2-methoxybenzoic acid |

| | |
|---|---|
| 4-M00-1-0112 | 5-[[3-[2-[4-[[[3-(4-Hydroxyphenyl)benzoyl]amino]methyl]-2-methylphenoxy]ethyl]phenyl]sulfamoyl]-2-methoxybenzoic acid |
| 4-M00-1-0113 | 5-[[2-Fluoro-3-[[3-[[4-(4-hydroxyphenyl)benzoyl]amino]phenoxy]methyl]phenyl]sulfamoyl]-2-methoxybenzoic acid |
| 4-M00-1-0114 | 5-[[2-Fluoro-3-[[3-[[3-(4-hydroxyphenyl)benzoyl]amino]phenoxy]methyl]phenyl]sulfamoyl]-2-methoxybenzoic acid |
| 4-M00-1-0115 | 5-[[2-Fluoro-3-[[4-[[4-(4-hydroxyphenyl)benzoyl]amino]-2-methylphenoxy]methyl]phenyl]sulfamoyl]-2-methoxybenzoic acid |
| 4-M00-1-0116 | 5-[[2-Fluoro-3-[[4-[[3-(4-hydroxyphenyl)benzoyl]amino]-2-methylphenoxy]methyl]phenyl]sulfamoyl]-2-methoxybenzoic acid |
| 4-M00-1-0117 | 5-[[2-Fluoro-3-[[2-fluoro-4-[[4-(4-hydroxyphenyl)benzoyl]amino]phenoxy]methyl]phenyl]sulfamoyl]-2-methoxybenzoic acid |
| 4-M00-1-0118 | 5-[[2-Fluoro-3-[[2-fluoro-4-[[3-(4-hydroxyphenyl)benzoyl]amino]phenoxy]methyl]phenyl]sulfamoyl]-2-methoxybenzoic acid |
| 4-M00-1-0119 | 5-[[2-Fluoro-3-[[4-[[[4-(4-hydroxyphenyl)benzoyl]amino]methyl]-2-methylphenoxy]methyl]phenyl]sulfamoyl]-2-methoxybenzoic acid |
| 4-M00-1-0120 | 5-[[2-Fluoro-3-[[4-[[[3-(4-hydroxyphenyl)benzoyl]amino]methyl]-2-methylphenoxy]methyl]phenyl]sulfamoyl]-2-methoxybenzoic acid |
| 4-M00-1-0121 | 5-[[3-[3-[3-[[4-(4-Hydroxyphenyl)benzoyl]amino]phenoxy]propyl]phenyl]sulfamoyl]-2-methoxybenzoic acid |
| 4-M00-1-0122 | 5-[[3-[3-[3-[[3-(4-Hydroxyphenyl)benzoyl]amino]phenoxy]propyl]phenyl]sulfamoyl]-2-methoxybenzoic acid |
| 4-M00-1-0123 | 5-[[3-[3-[4-[[4-(4-Hydroxyphenyl)benzoyl]amino]-2-methylphenoxy]propyl]phenyl]sulfamoyl]-2-methoxybenzoic acid |
| 4-M00-1-0124 | 5-[[3-[3-[4-[[3-(4-Hydroxyphenyl)benzoyl]amino]-2-methylphenoxy]propyl]phenyl]sulfamoyl]-2-methoxybenzoic acid |
| 4-M00-1-0125 | 5-[[3-[3-[2-Fluoro-4-[[4-(4-hydroxyphenyl)benzoyl]amino]phenoxy]propyl]phenyl]sulfamoyl]-2-methoxybenzoic acid |
| 4-M00-1-0126 | 5-[[3-[3-[2-Fluoro-4-[[3-(4-hydroxyphenyl)benzoyl]amino]phenoxy]propyl]phenyl]sulfamoyl]-2-methoxybenzoic acid |
| 4-M00-1-0127 | 5-[[3-[3-[4-[[[4-(4-Hydroxyphenyl)benzoyl]amino]methyl]-2-methylphenoxy]propyl]phenyl]sulfamoyl]-2-methoxybenzoic acid |
| 4-M00-1-0128 | 5-[[3-[3-[4-[[[3-(4-Hydroxyphenyl)benzoyl]amino]methyl]-2-methylphenoxy]propyl]phenyl]sulfamoyl]-2-methoxybenzoic acid |
| 4-M00-1-0129 | Butyl 3-[[3-[[4-(4-hydroxyphenyl)benzoyl]amino]phenyl]carbamoyl]benzoate |
| 4-M00-1-0130 | Butyl 3-[[3-[[3-(4-hydroxyphenyl)benzoyl]amino]phenyl]carbamoyl]benzoate |
| 4-M00-1-0131 | Butyl 3-[[3-[[4-(4-hydroxyphenyl)benzoyl]amino]-4-methylphenyl]carbamoyl]benzoate |
| 4-M00-1-0132 | Butyl 3-[[3-[[3-(4-hydroxyphenyl)benzoyl]amino]-4-methylphenyl]carbamoyl]benzoate |
| 4-M00-1-0133 | Butyl 3-[[3-[[[4-(4-hydroxyphenyl)benzoyl]amino]methyl]phenyl]carbamoyl]benzoate |
| 4-M00-1-0134 | Butyl 3-[[3-[[[3-(4-hydroxyphenyl)benzoyl]amino]methyl]phenyl]carbamoyl]benzoate |

| | |
|---|---|
| 4-M00-1-0135 | Butyl 3-[[3-[[4-(4-hydroxyphenyl)benzoyl]amino]-4-methoxyphenyl]carbamoyl]benzoate |
| 4-M00-1-0136 | Butyl 3-[[3-[[3-(4-hydroxyphenyl)benzoyl]amino]-4-methoxyphenyl]carbamoyl]benzoate |
| 4-M00-1-0137 | Butyl 5-[[3-[[4-(4-hydroxyphenyl)benzoyl]amino]phenyl]carbamoyl]pyridine-3-carboxylate |
| 4-M00-1-0138 | Butyl 5-[[3-[[3-(4-hydroxyphenyl)benzoyl]amino]phenyl]carbamoyl]pyridine-3-carboxylate |
| 4-M00-1-0139 | Butyl 5-[[3-[[4-(4-hydroxyphenyl)benzoyl]amino]-4-methylphenyl]carbamoyl]pyridine-3-carboxylate |
| 4-M00-1-0140 | Butyl 5-[[3-[[3-(4-hydroxyphenyl)benzoyl]amino]-4-methylphenyl]carbamoyl]pyridine-3-carboxylate |
| 4-M00-1-0141 | Butyl 5-[[3-[[[4-(4-hydroxyphenyl)benzoyl]amino]methyl]phenyl]carbamoyl]pyridine-3-carboxylate |
| 4-M00-1-0142 | Butyl 5-[[3-[[[3-(4-hydroxyphenyl)benzoyl]amino]methyl]phenyl]carbamoyl]pyridine-3-carboxylate |
| 4-M00-1-0143 | Butyl 5-[[3-[[4-(4-hydroxyphenyl)benzoyl]amino]-4-methoxyphenyl]carbamoyl]pyridine-3-carboxylate |
| 4-M00-1-0144 | Butyl 5-[[3-[[3-(4-hydroxyphenyl)benzoyl]amino]-4-methoxyphenyl]carbamoyl]pyridine-3-carboxylate |
| 4-M00-1-0145 | Butyl 4-[[3-[[4-(4-hydroxyphenyl)benzoyl]amino]phenyl]carbamoyl]-2-methylbenzoate |
| 4-M00-1-0146 | Butyl 4-[[3-[[3-(4-hydroxyphenyl)benzoyl]amino]phenyl]carbamoyl]-2-methylbenzoate |
| 4-M00-1-0147 | Butyl 4-[[3-[[4-(4-hydroxyphenyl)benzoyl]amino]-4-methylphenyl]carbamoyl]-2-methylbenzoate |
| 4-M00-1-0148 | Butyl 4-[[3-[[3-(4-hydroxyphenyl)benzoyl]amino]-4-methylphenyl]carbamoyl]-2-methylbenzoate |
| 4-M00-1-0149 | Butyl 4-[[3-[[[4-(4-hydroxyphenyl)benzoyl]amino]methyl]phenyl]carbamoyl]-2-methylbenzoate |
| 4-M00-1-0150 | Butyl 4-[[3-[[[3-(4-hydroxyphenyl)benzoyl]amino]methyl]phenyl]carbamoyl]-2-methylbenzoate |
| 4-M00-1-0151 | Butyl 4-[[3-[[4-(4-hydroxyphenyl)benzoyl]amino]-4-methoxyphenyl]carbamoyl]-2-methylbenzoate |
| 4-M00-1-0152 | Butyl 4-[[3-[[3-(4-hydroxyphenyl)benzoyl]amino]-4-methoxyphenyl]carbamoyl]-2-methylbenzoate |
| 4-M00-1-0153 | Butyl 1-[3-[[4-(4-hydroxyphenyl)benzoyl]amino]phenyl]-5-oxopyrrolidine-3-carboxylate |
| 4-M00-1-0154 | Butyl 1-[3-[[3-(4-hydroxyphenyl)benzoyl]amino]phenyl]-5-oxopyrrolidine-3-carboxylate |
| 4-M00-1-0155 | Butyl 1-[3-[[4-(4-hydroxyphenyl)benzoyl]amino]-4-methylphenyl]-5-oxopyrrolidine-3-carboxylate |
| 4-M00-1-0156 | Butyl 1-[3-[[3-(4-hydroxyphenyl)benzoyl]amino]-4-methylphenyl]-5-oxopyrrolidine-3-carboxylate |
| 4-M00-1-0157 | Butyl 1-[3-[[[4-(4-hydroxyphenyl)benzoyl]amino]methyl]phenyl]-5-oxopyrrolidine-3-carboxylate |
| 4-M00-1-0158 | Butyl 1-[3-[[[3-(4-hydroxyphenyl)benzoyl]amino]methyl]phenyl]-5-oxopyrrolidine-3-carboxylate |
| 4-M00-1-0159 | Butyl 1-[3-[[4-(4-hydroxyphenyl)benzoyl]amino]-4-methoxyphenyl]-5-oxopyrrolidine-3-carboxylate |
| 4-M00-1-0160 | Butyl 1-[3-[[3-(4-hydroxyphenyl)benzoyl]amino]-4-methoxyphenyl]-5-oxopyrrolidine-3-carboxylate |

| | |
|---|---|
| 4-M00-1-0161 | 2,2,2-Trifluoroethyl 3-[[3-[[4-(4-hydroxyphenyl)benzoyl]amino]phenyl]carbamoyl]benzoate |
| 4-M00-1-0162 | 2,2,2-Trifluoroethyl 3-[[3-[[3-(4-hydroxyphenyl)benzoyl]amino]phenyl]carbamoyl]benzoate |
| 4-M00-1-0163 | 2,2,2-Trifluoroethyl 3-[[3-[[4-(4-hydroxyphenyl)benzoyl]amino]-4-methylphenyl]carbamoyl]benzoate |
| 4-M00-1-0164 | 2,2,2-Trifluoroethyl 3-[[3-[[3-(4-hydroxyphenyl)benzoyl]amino]-4-methylphenyl]carbamoyl]benzoate |
| 4-M00-1-0165 | 2,2,2-Trifluoroethyl 3-[[3-[[[4-(4-hydroxyphenyl)benzoyl]amino]methyl]phenyl]carbamoyl]benzoate |
| 4-M00-1-0166 | 2,2,2-Trifluoroethyl 3-[[3-[[[3-(4-hydroxyphenyl)benzoyl]amino]methyl]phenyl]carbamoyl]benzoate |
| 4-M00-1-0167 | 2,2,2-Trifluoroethyl 3-[[3-[[4-(4-hydroxyphenyl)benzoyl]amino]-4-methoxyphenyl]carbamoyl]benzoate |
| 4-M00-1-0168 | 2,2,2-Trifluoroethyl 3-[[3-[[3-(4-hydroxyphenyl)benzoyl]amino]-4-methoxyphenyl]carbamoyl]benzoate |
| 4-M00-1-0169 | 2,2,2-Trifluoroethyl 5-[[3-[[4-(4-hydroxyphenyl)benzoyl]amino]phenyl]carbamoyl]pyridine-3-carboxylate |
| 4-M00-1-0170 | 2,2,2-Trifluoroethyl 5-[[3-[[3-(4-hydroxyphenyl)benzoyl]amino]phenyl]carbamoyl]pyridine-3-carboxylate |
| 4-M00-1-0171 | 2,2,2-Trifluoroethyl 5-[[3-[[4-(4-hydroxyphenyl)benzoyl]amino]-4-methylphenyl]carbamoyl]pyridine-3-carboxylate |
| 4-M00-1-0172 | 2,2,2-Trifluoroethyl 5-[[3-[[3-(4-hydroxyphenyl)benzoyl]amino]-4-methylphenyl]carbamoyl]pyridine-3-carboxylate |
| 4-M00-1-0173 | 2,2,2-Trifluoroethyl 5-[[3-[[[4-(4-hydroxyphenyl)benzoyl]amino]methyl]phenyl]carbamoyl]pyridine-3-carboxylate |
| 4-M00-1-0174 | 2,2,2-Trifluoroethyl 5-[[3-[[[3-(4-hydroxyphenyl)benzoyl]amino]methyl]phenyl]carbamoyl]pyridine-3-carboxylate |
| 4-M00-1-0175 | 2,2,2-Trifluoroethyl 5-[[3-[[4-(4-hydroxyphenyl)benzoyl]amino]-4-methoxyphenyl]carbamoyl]pyridine-3-carboxylate |
| 4-M00-1-0176 | 2,2,2-Trifluoroethyl 5-[[3-[[3-(4-hydroxyphenyl)benzoyl]amino]-4-methoxyphenyl]carbamoyl]pyridine-3-carboxylate |
| 4-M00-1-0177 | 2,2,2-Trifluoroethyl 4-[[3-[[4-(4-hydroxyphenyl)benzoyl]amino]phenyl]carbamoyl]-2-methylbenzoate |
| 4-M00-1-0178 | 2,2,2-Trifluoroethyl 4-[[3-[[3-(4-hydroxyphenyl)benzoyl]amino]phenyl]carbamoyl]-2-methylbenzoate |
| 4-M00-1-0179 | 2,2,2-Trifluoroethyl 4-[[3-[[4-(4-hydroxyphenyl)benzoyl]amino]-4-methylphenyl]carbamoyl]-2-methylbenzoate |
| 4-M00-1-0180 | 2,2,2-Trifluoroethyl 4-[[3-[[3-(4-hydroxyphenyl)benzoyl]amino]-4-methylphenyl]carbamoyl]-2-methylbenzoate |
| 4-M00-1-0181 | 2,2,2-Trifluoroethyl 4-[[3-[[[4-(4-hydroxyphenyl)benzoyl]amino]methyl]phenyl]carbamoyl]-2-methylbenzoate |
| 4-M00-1-0182 | 2,2,2-Trifluoroethyl 4-[[3-[[[3-(4-hydroxyphenyl)benzoyl]amino]methyl]phenyl]carbamoyl]-2-methylbenzoate |
| 4-M00-1-0183 | 2,2,2-Trifluoroethyl 4-[[3-[[4-(4-hydroxyphenyl)benzoyl]amino]-4-methoxyphenyl]carbamoyl]-2-methylbenzoate |
| 4-M00-1-0184 | 2,2,2-Trifluoroethyl 4-[[3-[[3-(4-hydroxyphenyl)benzoyl]amino]-4-methoxyphenyl]carbamoyl]-2-methylbenzoate |
| 4-M00-1-0185 | 2,2,2-Trifluoroethyl 1-[3-[[4-(4-hydroxyphenyl)benzoyl]amino]phenyl]-5-oxopyrrolidine-3-carboxylate |
| 4-M00-1-0186 | 2,2,2-Trifluoroethyl 1-[3-[[3-(4-hydroxyphenyl)benzoyl]amino]phenyl]-5-oxopyrrolidine-3-carboxylate |

| | |
|---|---|
| 4-M00-1-0187 | 2,2,2-Trifluoroethyl 1-[3-[[4-(4-hydroxyphenyl)benzoyl]amino]-4-methylphenyl]-5-oxopyrrolidine-3-carboxylate |
| 4-M00-1-0188 | 2,2,2-Trifluoroethyl 1-[3-[[3-(4-hydroxyphenyl)benzoyl]amino]-4-methylphenyl]-5-oxopyrrolidine-3-carboxylate |
| 4-M00-1-0189 | 2,2,2-Trifluoroethyl 1-[3-[[[4-(4-hydroxyphenyl)benzoyl]amino]methyl]phenyl]-5-oxopyrrolidine-3-carboxylate |
| 4-M00-1-0190 | 2,2,2-Trifluoroethyl 1-[3-[[[3-(4-hydroxyphenyl)benzoyl]amino]methyl]phenyl]-5-oxopyrrolidine-3-carboxylate |
| 4-M00-1-0191 | 2,2,2-Trifluoroethyl 1-[3-[[4-(4-hydroxyphenyl)benzoyl]amino]-4-methoxyphenyl]-5-oxopyrrolidine-3-carboxylate |
| 4-M00-1-0192 | 2,2,2-Trifluoroethyl 1-[3-[[3-(4-hydroxyphenyl)benzoyl]amino]-4-methoxyphenyl]-5-oxopyrrolidine-3-carboxylate |
| 4-M00-1-0193 | (4-Methylphenyl) 3-[[3-[[4-(4-hydroxyphenyl)benzoyl]amino]phenyl]carbamoyl]benzoate |
| 4-M00-1-0194 | (4-Methylphenyl) 3-[[3-[[3-(4-hydroxyphenyl)benzoyl]amino]phenyl]carbamoyl]benzoate |
| 4-M00-1-0195 | (4-Methylphenyl) 3-[[3-[[4-(4-hydroxyphenyl)benzoyl]amino]-4-methylphenyl]carbamoyl]benzoate |
| 4-M00-1-0196 | (4-Methylphenyl) 3-[[3-[[3-(4-hydroxyphenyl)benzoyl]amino]-4-methylphenyl]carbamoyl]benzoate |
| 4-M00-1-0197 | (4-Methylphenyl) 3-[[3-[[[4-(4-hydroxyphenyl)benzoyl]amino]methyl]phenyl]carbamoyl]benzoate |
| 4-M00-1-0198 | (4-Methylphenyl) 3-[[3-[[[3-(4-hydroxyphenyl)benzoyl]amino]methyl]phenyl]carbamoyl]benzoate |
| 4-M00-1-0199 | (4-Methylphenyl) 3-[[3-[[4-(4-hydroxyphenyl)benzoyl]amino]-4-methoxyphenyl]carbamoyl]benzoate |
| 4-M00-1-0200 | (4-Methylphenyl) 3-[[3-[[3-(4-hydroxyphenyl)benzoyl]amino]-4-methoxyphenyl]carbamoyl]benzoate |
| 4-M00-1-0201 | (4-Methylphenyl) 5-[[3-[[4-(4-hydroxyphenyl)benzoyl]amino]phenyl]carbamoyl]pyridine-3-carboxylate |
| 4-M00-1-0202 | (4-Methylphenyl) 5-[[3-[[3-(4-hydroxyphenyl)benzoyl]amino]phenyl]carbamoyl]pyridine-3-carboxylate |
| 4-M00-1-0203 | (4-Methylphenyl) 5-[[3-[[4-(4-hydroxyphenyl)benzoyl]amino]-4-methylphenyl]carbamoyl]pyridine-3-carboxylate |
| 4-M00-1-0204 | (4-Methylphenyl) 5-[[3-[[3-(4-hydroxyphenyl)benzoyl]amino]-4-methylphenyl]carbamoyl]pyridine-3-carboxylate |
| 4-M00-1-0205 | (4-Methylphenyl) 5-[[3-[[[4-(4-hydroxyphenyl)benzoyl]amino]methyl]phenyl]carbamoyl]pyridine-3-carboxylate |
| 4-M00-1-0206 | (4-Methylphenyl) 5-[[3-[[[3-(4-hydroxyphenyl)benzoyl]amino]methyl]phenyl]carbamoyl]pyridine-3-carboxylate |
| 4-M00-1-0207 | (4-Methylphenyl) 5-[[3-[[4-(4-hydroxyphenyl)benzoyl]amino]-4-methoxyphenyl]carbamoyl]pyridine-3-carboxylate |
| 4-M00-1-0208 | (4-Methylphenyl) 5-[[3-[[3-(4-hydroxyphenyl)benzoyl]amino]-4-methoxyphenyl]carbamoyl]pyridine-3-carboxylate |
| 4-M00-1-0209 | (4-Methylphenyl) 4-[[3-[[4-(4-hydroxyphenyl)benzoyl]amino]phenyl]carbamoyl]-2-methylbenzoate |
| 4-M00-1-0210 | (4-Methylphenyl) 4-[[3-[[3-(4-hydroxyphenyl)benzoyl]amino]phenyl]carbamoyl]-2-methylbenzoate |
| 4-M00-1-0211 | (4-Methylphenyl) 4-[[3-[[4-(4-hydroxyphenyl)benzoyl]amino]-4-methylphenyl]carbamoyl]-2-methylbenzoate |
| 4-M00-1-0212 | (4-Methylphenyl) 4-[[3-[[3-(4-hydroxyphenyl)benzoyl]amino]-4-methylphenyl]carbamoyl]-2-methylbenzoate |

| | |
|---|---|
| 4-M00-1-0213 | (4-Methylphenyl) 4-[[3-[[[4-(4-hydroxyphenyl)benzoyl]amino]methyl]phenyl]carbamoyl]-2-methylbenzoate |
| 4-M00-1-0214 | (4-Methylphenyl) 4-[[3-[[[3-(4-hydroxyphenyl)benzoyl]amino]methyl]phenyl]carbamoyl]-2-methylbenzoate |
| 4-M00-1-0215 | (4-Methylphenyl) 4-[[3-[[4-(4-hydroxyphenyl)benzoyl]amino]-4-methoxyphenyl]carbamoyl]-2-methylbenzoate |
| 4-M00-1-0216 | (4-Methylphenyl) 4-[[3-[[3-(4-hydroxyphenyl)benzoyl]amino]-4-methoxyphenyl]carbamoyl]-2-methylbenzoate |
| 4-M00-1-0217 | (4-Methylphenyl) 1-[3-[[4-(4-hydroxyphenyl)benzoyl]amino]phenyl]-5-oxopyrrolidine-3-carboxylate |
| 4-M00-1-0218 | (4-Methylphenyl) 1-[3-[[3-(4-hydroxyphenyl)benzoyl]amino]phenyl]-5-oxopyrrolidine-3-carboxylate |
| 4-M00-1-0219 | (4-Methylphenyl) 1-[3-[[4-(4-hydroxyphenyl)benzoyl]amino]-4-methylphenyl]-5-oxopyrrolidine-3-carboxylate |
| 4-M00-1-0220 | (4-Methylphenyl) 1-[3-[[3-(4-hydroxyphenyl)benzoyl]amino]-4-methylphenyl]-5-oxopyrrolidine-3-carboxylate |
| 4-M00-1-0221 | (4-Methylphenyl) 1-[3-[[[4-(4-hydroxyphenyl)benzoyl]amino]methyl]phenyl]-5-oxopyrrolidine-3-carboxylate |
| 4-M00-1-0222 | (4-Methylphenyl) 1-[3-[[[3-(4-hydroxyphenyl)benzoyl]amino]methyl]phenyl]-5-oxopyrrolidine-3-carboxylate |
| 4-M00-1-0223 | (4-Methylphenyl) 1-[3-[[4-(4-hydroxyphenyl)benzoyl]amino]-4-methoxyphenyl]-5-oxopyrrolidine-3-carboxylate |
| 4-M00-1-0224 | (4-Methylphenyl) 1-[3-[[3-(4-hydroxyphenyl)benzoyl]amino]-4-methoxyphenyl]-5-oxopyrrolidine-3-carboxylate |
| 4-M00-1-0225 | (3-Propan-2-ylphenyl) 3-[[3-[[4-(4-hydroxyphenyl)benzoyl]amino]phenyl]carbamoyl]benzoate |
| 4-M00-1-0226 | (3-Propan-2-ylphenyl) 3-[[3-[[3-(4-hydroxyphenyl)benzoyl]amino]phenyl]carbamoyl]benzoate |
| 4-M00-1-0227 | (3-Propan-2-ylphenyl) 3-[[3-[[4-(4-hydroxyphenyl)benzoyl]amino]-4-methylphenyl]carbamoyl]benzoate |
| 4-M00-1-0228 | (3-Propan-2-ylphenyl) 3-[[3-[[3-(4-hydroxyphenyl)benzoyl]amino]-4-methylphenyl]carbamoyl]benzoate |
| 4-M00-1-0229 | (3-Propan-2-ylphenyl) 3-[[3-[[[4-(4-hydroxyphenyl)benzoyl]amino]methyl]phenyl]carbamoyl]benzoate |
| 4-M00-1-0230 | (3-Propan-2-ylphenyl) 3-[[3-[[[3-(4-hydroxyphenyl)benzoyl]amino]methyl]phenyl]carbamoyl]benzoate |
| 4-M00-1-0231 | (3-Propan-2-ylphenyl) 3-[[3-[[4-(4-hydroxyphenyl)benzoyl]amino]-4-methoxyphenyl]carbamoyl]benzoate |
| 4-M00-1-0232 | (3-Propan-2-ylphenyl) 3-[[3-[[3-(4-hydroxyphenyl)benzoyl]amino]-4-methoxyphenyl]carbamoyl]benzoate |
| 4-M00-1-0233 | (3-Propan-2-ylphenyl) 5-[[3-[[4-(4-hydroxyphenyl)benzoyl]amino]phenyl]carbamoyl]pyridine-3-carboxylate |
| 4-M00-1-0234 | (3-Propan-2-ylphenyl) 5-[[3-[[3-(4-hydroxyphenyl)benzoyl]amino]phenyl]carbamoyl]pyridine-3-carboxylate |
| 4-M00-1-0235 | (3-Propan-2-ylphenyl) 5-[[3-[[4-(4-hydroxyphenyl)benzoyl]amino]-4-methylphenyl]carbamoyl]pyridine-3-carboxylate |
| 4-M00-1-0236 | (3-Propan-2-ylphenyl) 5-[[3-[[3-(4-hydroxyphenyl)benzoyl]amino]-4-methylphenyl]carbamoyl]pyridine-3-carboxylate |
| 4-M00-1-0237 | (3-Propan-2-ylphenyl) 5-[[3-[[[4-(4-hydroxyphenyl)benzoyl]amino]methyl]phenyl]carbamoyl]pyridine-3-carboxylate |
| 4-M00-1-0238 | (3-Propan-2-ylphenyl) 5-[[3-[[[3-(4-hydroxyphenyl)benzoyl]amino]methyl]phenyl]carbamoyl]pyridine-3-carboxylate |

| | |
|---|---|
| 4-M00-1-0239 | (3-Propan-2-ylphenyl) 5-[[3-[[4-(4-hydroxyphenyl)benzoyl]amino]-4-methoxyphenyl]carbamoyl]pyridine-3-carboxylate |
| 4-M00-1-0240 | (3-Propan-2-ylphenyl) 5-[[3-[[3-(4-hydroxyphenyl)benzoyl]amino]-4-methoxyphenyl]carbamoyl]pyridine-3-carboxylate |
| 4-M00-1-0241 | (3-Propan-2-ylphenyl) 4-[[3-[[4-(4-hydroxyphenyl)benzoyl]amino]phenyl]carbamoyl]-2-methylbenzoate |
| 4-M00-1-0242 | (3-Propan-2-ylphenyl) 4-[[3-[[3-(4-hydroxyphenyl)benzoyl]amino]phenyl]carbamoyl]-2-methylbenzoate |
| 4-M00-1-0243 | (3-Propan-2-ylphenyl) 4-[[3-[[4-(4-hydroxyphenyl)benzoyl]amino]-4-methylphenyl]carbamoyl]-2-methylbenzoate |
| 4-M00-1-0244 | (3-Propan-2-ylphenyl) 4-[[3-[[3-(4-hydroxyphenyl)benzoyl]amino]-4-methylphenyl]carbamoyl]-2-methylbenzoate |
| 4-M00-1-0245 | (3-Propan-2-ylphenyl) 4-[[3-[[[4-(4-hydroxyphenyl)benzoyl]amino]methyl]phenyl]carbamoyl]-2-methylbenzoate |
| 4-M00-1-0246 | (3-Propan-2-ylphenyl) 4-[[3-[[[3-(4-hydroxyphenyl)benzoyl]amino]methyl]phenyl]carbamoyl]-2-methylbenzoate |
| 4-M00-1-0247 | (3-Propan-2-ylphenyl) 4-[[3-[[4-(4-hydroxyphenyl)benzoyl]amino]-4-methoxyphenyl]carbamoyl]-2-methylbenzoate |
| 4-M00-1-0248 | (3-Propan-2-ylphenyl) 4-[[3-[[3-(4-hydroxyphenyl)benzoyl]amino]-4-methoxyphenyl]carbamoyl]-2-methylbenzoate |
| 4-M00-1-0249 | (3-Propan-2-ylphenyl) 1-[3-[[4-(4-hydroxyphenyl)benzoyl]amino]phenyl]-5-oxopyrrolidine-3-carboxylate |
| 4-M00-1-0250 | (3-Propan-2-ylphenyl) 1-[3-[[3-(4-hydroxyphenyl)benzoyl]amino]phenyl]-5-oxopyrrolidine-3-carboxylate |
| 4-M00-1-0251 | (3-Propan-2-ylphenyl) 1-[3-[[4-(4-hydroxyphenyl)benzoyl]amino]-4-methylphenyl]-5-oxopyrrolidine-3-carboxylate |
| 4-M00-1-0252 | (3-Propan-2-ylphenyl) 1-[3-[[3-(4-hydroxyphenyl)benzoyl]amino]-4-methylphenyl]-5-oxopyrrolidine-3-carboxylate |
| 4-M00-1-0253 | (3-Propan-2-ylphenyl) 1-[3-[[[4-(4-hydroxyphenyl)benzoyl]amino]methyl]phenyl]-5-oxopyrrolidine-3-carboxylate |
| 4-M00-1-0254 | (3-Propan-2-ylphenyl) 1-[3-[[[3-(4-hydroxyphenyl)benzoyl]amino]methyl]phenyl]-5-oxopyrrolidine-3-carboxylate |
| 4-M00-1-0255 | (3-Propan-2-ylphenyl) 1-[3-[[4-(4-hydroxyphenyl)benzoyl]amino]-4-methoxyphenyl]-5-oxopyrrolidine-3-carboxylate |
| 4-M00-1-0256 | (3-Propan-2-ylphenyl) 1-[3-[[3-(4-hydroxyphenyl)benzoyl]amino]-4-methoxyphenyl]-5-oxopyrrolidine-3-carboxylate |

Example 10-18: Analysis of mixture 4-M00-1 by LCMS

100 μL of the solution of the mixture (4-M00-1) obtained in Example 10-17 in DMF (5 mL) was diluted with MeCN (1.0 mL) and analyzed by LCMS. The mixture was analyzed under analysis conditions SMD-FA05-long-25 min, and the UV area was confirmed at a UV wavelength of 299 nm. The analysis results are shown in Table 10-18-1.
[Table 587]

| [Table 10-18-1] LCMS analysis results | | | |
|---|---|---|---|
| Mixture No. | | | 4-M00-1 |
| m/z | Retention time of MS peak (min) | Retention time of UV peak (min) | No. of assigned compound described with 4-M00-1-omitted |
| 473.207 | 9.88 | 9.89 | 0153,0154 |
| 473.207 | 9.743 | 9.74 | 0153,0154 |

(continued)

| [Table 10-18-1] LCMS analysis results | | | |
|---|---|---|---|
| Mixture No. | | | 4-M00-1 |
| m/z | Retention time of MS peak (min) | Retention time of UV peak (min) | No. of assigned compound described with 4-M00-1-omitted |
| 487.223 | 9.187 | 9.18 | 0155, 0156, 0157, 0158 |
| 487.223 | 9.353 | 9.34 | 0155, 0156, 0157, 0158 |
| 487.223 | 9.607 | 9.59 | 0155, 0156, 0157, 0158 |
| 487.223 | 9.727 | 9.74 | 0155, 0156, 0157, 0158 |
| 499.148 | 9.11 | 9.1 | 0185, 0186 |
| 499.148 | 9.26 | 9.3 | 0185, 0186 |
| 503.218 | 9.987 | 9.98 | 0159, 0160 |
| 503.218 | 10.08 | 10.11 | 0159, 0160 |
| 507.191 | 10.167 | 10.11 | 0217, 0218 |
| 507.191 | 10.04 | 10.04 | 0217, 0218 |
| 509.207 | 11.043 | 11.02 | 0129, 0130 |
| 509.207 | 11.147 | 11.14 | 0129, 0130 |
| 510.202 | 10.23 | 10.28 | 0137, 0138 |
| 510.202 | 10.113 | 10.11 | 0137, 0138 |
| 513.163 | 8.787 | 8.78 | 0187, 0188, 0189, 0190 |
| 513.163 | 8.63 | 8.62 | 0187, 0188, 0189, 0190 |
| 513.163 | 9.043 | 9.02 | 0187, 0188, 0189, 0190 |
| 513.163 | 9.17 | 9.18 | 0187, 0188, 0189, 0190 |
| 521.207 | 9.503 | 9.49 | 0219, 0220, 0221, 0222 |
| 521.207 | 10.06 | 10.04 | 0219, 0220, 0221, 0222 |
| 521.207 | 9.663 | 9.69 | 0219, 0220, 0221, 0222 |
| 521.207 | 9.93 | 9.89 | 0219, 0220, 0221, 0222 |
| 523.223 | 11.487 | 11.48 | 0131, 0132, 0133, 0134, 0145, 0146 |
| 523.223 | 10.91 | 10.9 | 0131, 0132, 0133, 0134, 0145, 0146 |
| 523.223 | 10.513 | 10.5 | 0131, 0132, 0133, 0134, 0145, 0146 |
| 523.223 | 10.647 | 10.63 | 0131, 0132, 0133, 0134, 0145, 0146 |
| 523.223 | 10.997 | 11.02 | 0131, 0132, 0133, 0134, 0145, 0146 |
| 523.223 | 11.603 | 11.59 | 0131, 0132, 0133, 0134, 0145, 0146 |
| 524.218 | 9.61 | 9.59 | 0139, 0140, 0141, 0142 |
| 524.218 | 10.12 | 10.11 | 0139, 0140, 0141, 0142 |
| 524.218 | 9.75 | 9.74 | 0139, 0140, 0141, 0142 |
| 524.218 | 10.007 | 9.98 | 0139, 0140, 0141, 0142 |
| 529.158 | 9.317 | 9.3 | 0191, 0192 |
| 529.158 | 9.443 | 9.41 | 0191, 0192 |
| 531.195 | 10.317 | 10.37 | 0001, 0010, 0002, 0009 |
| 531.195 | 10.217 | 10.26 | 0001, 0010, 0002, 0009 |
| 531.195 | 10.397 | 10.42 | 0001, 0010, 0002, 0009 |

(continued)

| [Table 10-18-1] LCMS analysis results | | | |
|---|---|---|---|
| Mixture No. | | | 4-M00-1 |
| m/z | Retention time of MS peak (min) | Retention time of UV peak (min) | No. of assigned compound described with 4-M00-1-omitted |
| 531.195 | 11.043 | 11.02 | 0001, 0010, 0002, 0009 |
| 535.17 | 10.957 | 10.95 | 0161, 0162, 0017, 0018, 0249, 0250 |
| 535.17 | 8.797 | 8.78 | 0161, 0162, 0017, 0018, 0249, 0250 |
| 535.17 | 10.11 | 10.11 | 0161, 0162, 0017, 0018, 0249, 0250 |
| 535.17 | 10.44 | 10.42 | 0161, 0162, 0017, 0018, 0249, 0250 |
| 535.17 | 10.003 | 9.98 | 0161, 0162, 0017, 0018, 0249, 0250 |
| 535.17 | 11.08 | 11.09 | 0161, 0162, 0017, 0018, 0249, 0250 |
| 536.143 | 9.567 | 9.59 | 0169, 0170 |
| 536.143 | 9.417 | 9.41 | 0169, 0170 |
| 537.238 | 9.557 | 9.59 | 0147, 0148, 0149, 0150, 0223, 0224 |
| 537.238 | 10.357 | 10.37 | 0147, 0148, 0149, 0150, 0223, 0224 |
| 537.238 | 11.103 | 11.09 | 0147, 0148, 0149, 0150, 0223, 0224 |
| 537.238 | 11.437 | 11.43 | 0147, 0148, 0149, 0150, 0223, 0224 |
| 537.238 | 10.27 | 10.26 | 0147, 0148, 0149, 0150, 0223, 0224 |
| 537.238 | 11.35 | 11.33 | 0147, 0148, 0149, 0150, 0223, 0224 |
| 539.218 | 11.38 | 11.38 | 0135, 0136 |
| 539.218 | 11.443 | 11.43 | 0135, 0136 |
| 540.213 | 10.143 | 10.11 | 0143, 0144 |
| 540.213 | 10.473 | 10.46 | 0143, 0144 |
| 543.191 | 9.507 | 9.49 | 0193, 0194 |
| 543.191 | 9.927 | 9.89 | 0193, 0194 |
| 544.187 | 9.097 | 9.1 | 0201, 0202 |
| 544.187 | 10.51 | 10.5 | 0201, 0202 |
| 545.21 | 10.347 | 10.37 | 0011, 0012, 0025, 0026, 0003, 0004 |
| 545.21 | 10.553 | 10.58 | 0011, 0012, 0025, 0026, 0003, 0004 |
| 545.21 | 10.667 | 10.63 | 0011, 0012, 0025, 0026, 0003, 0004 |
| 545.21 | 10.76 | 10.79 | 0011, 0012, 0025, 0026, 0003, 0004 |
| 545.21 | 10.463 | 10.46 | 0011, 0012, 0025, 0026, 0003, 0004 |
| 545.21 | 10.73 | 10.72 | 0011, 0012, 0025, 0026, 0003, 0004 |
| 549.185 | 8.737 | 8.72 | 0013, 0014, 0163, 0164, 0165, 0166, 0177, 0178, 0019, 0020, 0251, 0252, 0253, 0254, 0005, 0006 |
| 549.185 | 9.82 | 9.83 | 0013, 0014, 0163, 0164, 0165, 0166, 0177, 0178, 0019, 0020, 0251, 0252, 0253, 0254, 0005, 0006 |
| 549.185 | 10.85 | 10.84 | 0013, 0014, 0163, 0164, 0165, 0166, 0177, 0178, 0019, 0020, 0251, 0252, 0253, 0254, 0005, 0006 |
| 550.158 | 9.35 | 9.34 | 0171, 0172, 0173, 0174 |
| 550.158 | 8.75 | 8.72 | 0171, 0172, 0173, 0174 |

(continued)

| [Table 10-18-1] LCMS analysis results | | | |
|---|---|---|---|
| Mixture No. | | | 4-M00-1 |
| m/z | Retention time of MS peak (min) | Retention time of UV peak (min) | No. of assigned compound described with 4-M00-1-omitted |
| 550.158 | 8.97 | 8.97 | 0171, 0172, 0173, 0174 |
| 550.158 | 9.13 | 9.1 | 0171, 0172, 0173, 0174 |
| 553.16 | 11.92 | 11.92 | 0151, 0152, 0021, 0022 |
| 553.16 | 9.963 | 9.98 | 0151, 0152, 0021, 0022 |
| 553.16 | 10.117 | 10.11 | 0151, 0152, 0021, 0022 |
| 553.16 | 11.863 | 11.86 | 0151, 0152, 0021, 0022 |
| 557.207 | 11.097 | 11.09 | 0195, 0196, 0197, 0198, 0209, 0210 |
| 557.207 | 10.72 | 10.72 | 0195, 0196, 0197, 0198, 0209, 0210 |
| 557.207 | 11.637 | 11.64 | 0195, 0196, 0197, 0198, 0209, 0210 |
| 557.207 | 11.673 | 11.64 | 0195, 0196, 0197, 0198, 0209, 0210 |
| 557.207 | 12.91 | 12.93 | 0195, 0196, 0197, 0198, 0209, 0210 |
| 557.207 | 12.97 | 12.93 | 0195, 0196, 0197, 0198, 0209, 0210 |
| 558.202 | 8.623 | 8.62 | 0203, 0204, 0205, 0206 |
| 558.202 | 10.283 | 10.26 | 0203, 0204, 0205, 0206 |
| 558.202 | 9.153 | 9.18 | 0203, 0204, 0205, 0206 |
| 558.202 | 10.053 | 10.04 | 0203, 0204, 0205, 0206 |
| 559.226 | 9.923 | 9.89 | 0015, 0016, 0027, 0028, 0007, 0008 |
| 559.226 | 10.113 | 10.11 | 0015, 0016, 0027, 0028, 0007, 0008 |
| 559.226 | 10.187 | 10.26 | 0015, 0016, 0027, 0028, 0007, 0008 |
| 559.226 | 10.387 | 10.37 | 0015, 0016, 0027, 0028, 0007, 0008 |
| 559.226 | 10.84 | 10.84 | 0015, 0016, 0027, 0028, 0007, 0008 |
| 559.226 | 10.94 | 10.95 | 0015, 0016, 0027, 0028, 0007, 0008 |
| 563.201 | 10.02 | 10.04 | 0179, 0180, 0181, 0182, 0023, 0024, 0029, 0030 |
| 563.201 | 10.26 | 10.26 | 0179, 0180, 0181, 0182, 0023, 0024, 0029, 0030 |
| 563.201 | 10.413 | 10.42 | 0179, 0180, 0181, 0182, 0023, 0024, 0029, 0030 |
| 563.201 | 7.79 | 7.81 | 0179, 0180, 0181, 0182, 0023, 0024, 0029, 0030 |
| 563.201 | 9.887 | 9.89 | 0179, 0180, 0181, 0182, 0023, 0024, 0029, 0030 |
| 563.201 | 11.507 | 11.51 | 0179, 0180, 0181, 0182, 0023, 0024, 0029, 0030 |
| 563.201 | 11.617 | 11.64 | 0179, 0180, 0181, 0182, 0023, 0024, 0029, 0030 |
| 563.201 | 13.067 | 13.08 | 0179, 0180, 0181, 0182, 0023, 0024, 0029, 0030 |
| 565.158 | 10.627 | 10.63 | 0167, 0168, 0255, 0256 |
| 565.158 | 10.723 | 10.72 | 0167, 0168, 0255, 0256 |
| 565.158 | 11.183 | 11.2 | 0167, 0168, 0255, 0256 |
| 565.158 | 11.287 | 11.29 | 0167, 0168, 0255, 0256 |
| 566.153 | 9.753 | 9.74 | 0175, 0176 |
| 566.153 | 11.28 | 11.29 | 0175, 0176 |

(continued)

| [Table 10-18-1] LCMS analysis results | | | |
|---|---|---|---|
| Mixture No. | | | 4-M00-1 |
| m/z | Retention time of MS peak (min) | Retention time of UV peak (min) | No. of assigned compound described with 4-M00-1-omitted |
| 571.223 | 12.123 | 12.09 | 0211, 0212, 0213, 0214, 0225, 0226 |
| 571.223 | 8.723 | 8.72 | 0211, 0212, 0213, 0214, 0225, 0226 |
| 571.223 | 8.757 | 8.78 | 0211, 0212, 0213, 0214, 0225, 0226 |
| 571.223 | 9.16 | 9.18 | 0211, 0212, 0213, 0214, 0225, 0226 |
| 572.218 | 11.313 | 11.33 | 0233, 0234 |
| 572.218 | 11.417 | 11.43 | 0233, 0234 |
| 573.242 | 10.69 | 10.72 | 0199, 0200, 0031, 0032 |
| 573.242 | 5.947 | 5.96 | 0199, 0200, 0031, 0032 |
| 573.242 | 8.967 | 8.97 | 0199, 0200, 0031, 0032 |
| 573.242 | 10.597 | 10.58 | 0199, 0200, 0031, 0032 |
| 574.197 | 10.72 | 10.72 | 0207, 0208 |
| 574.197 | 10.797 | 10.79 | 0207, 0208 |
| 579.174 | 11.103 | 11.09 | 0183, 0184 |
| 579.174 | 11.173 | 11.2 | 0183, 0184 |
| 585.238 | 11.59 | 11.59 | 0227, 0228, 0229, 0230, 0241, 0242 |
| 585.238 | 9.887 | 9.89 | 0227, 0228, 0229, 0230, 0241, 0242 |
| 585.238 | 9.907 | 9.89 | 0227, 0228, 0229, 0230, 0241, 0242 |
| 585.238 | 9.923 | 9.89 | 0227, 0228, 0229, 0230, 0241, 0242 |
| 585.238 | 11.767 | 11.75 | 0227, 0228, 0229, 0230, 0241, 0242 |
| 585.238 | 11.97 | 12.01 | 0227, 0228, 0229, 0230, 0241, 0242 |
| 586.234 | 10.803 | 10.79 | 0235, 0236, 0237, 0238 |
| 586.234 | 11.31 | 11.29 | 0235, 0236, 0237, 0238 |
| 586.234 | 10.967 | 10.95 | 0235, 0236, 0237, 0238 |
| 586.234 | 11.2 | 11.2 | 0235, 0236, 0237, 0238 |
| 587.218 | 12.093 | 12.09 | 0215, 0216 |
| 587.218 | 5.673 | 5.67 | 0215, 0216 |
| 599.254 | 12.17 | 12.16 | 0243, 0244, 0245, 0246 |
| 599.254 | 12.04 | 12.01 | 0243, 0244, 0245, 0246 |
| 599.254 | 12.367 | 12.37 | 0243, 0244, 0245, 0246 |
| 599.254 | 12.387 | 12.37 | 0243, 0244, 0245, 0246 |
| 601.233 | 12.447 | 12.45 | 0231, 0232 |
| 601.233 | 12.527 | 12.5 | 0231, 0232 |
| 602.229 | 11.733 | 11.75 | 0239, 0240 |
| 602.229 | 11.653 | 11.64 | 0239, 0240 |
| 615.125 | 6.673 | 6.64 | 0247, 0248, 0033, 0034, 0041, 0042 |
| 615.125 | 8.607 | 8.62 | 0247, 0248, 0033, 0034, 0041, 0042 |

(continued)

| [Table 10-18-1] LCMS analysis results | | | |
|---|---|---|---|
| Mixture No. | | | 4-M00-1 |
| m/z | Retention time of MS peak (min) | Retention time of UV peak (min) | No. of assigned compound described with 4-M00-1-omitted |
| 615.125 | 8.647 | 8.62 | 0247, 0248, 0033, 0034, 0041, 0042 |
| 615.125 | 10.277 | 10.26 | 0247, 0248, 0033, 0034, 0041, 0042 |
| 615.125 | 10.417 | 10.42 | 0247, 0248, 0033, 0034, 0041, 0042 |
| 615.125 | 12.88 | 12.86 | 0247, 0248, 0033, 0034, 0041, 0042 |
| 619.1 | 10.087 | 10.11 | 0049, 0050 |
| 619.1 | 10.23 | 10.26 | 0049, 0050 |
| 623.185 | 11.76 | 11.75 | 0065, 0066, 0073, 0074 |
| 623.185 | 12.46 | 12.45 | 0065, 0066, 0073, 0074 |
| 623.185 | 10.01 | 9.98 | 0065, 0066, 0073, 0074 |
| 623.185 | 10.037 | 10.04 | 0065, 0066, 0073, 0074 |
| 627.16 | 9.703 | 9.69 | 0081, 0082 |
| 627.16 | 11.71 | 11.75 | 0081, 0082 |
| 629.141 | 10.393 | 10.37 | 0035, 0036, 0043, 0044, 0057, 0058 |
| 629.141 | 9.697 | 9.69 | 0035, 0036, 0043, 0044, 0057, 0058 |
| 629.141 | 9.71 | 9.69 | 0035, 0036, 0043, 0044, 0057, 0058 |
| 629.141 | 10.54 | 10.5 | 0035, 0036, 0043, 0044, 0057, 0058 |
| 629.141 | 11.283 | 11.29 | 0035, 0036, 0043, 0044, 0057, 0058 |
| 629.141 | 12.22 | 12.21 | 0035, 0036, 0043, 0044, 0057, 0058 |
| 633.116 | 10.2 | 10.26 | 0037, 0038, 0045, 0046, 0051, 0052 |
| 633.116 | 10.15 | 10.11 | 0037, 0038, 0045, 0046, 0051, 0052 |
| 633.116 | 12.01 | 12.01 | 0037, 0038, 0045, 0046, 0051, 0052 |
| 633.116 | 12.067 | 12.09 | 0037, 0038, 0045, 0046, 0051, 0052 |
| 633.116 | 13.023 | 13.01 | 0037, 0038, 0045, 0046, 0051, 0052 |
| 637.091 | 9.58 | 9.59 | 0053, 0054, 0067, 0068, 0075, 0076, 0089, 0090 |
| 637.091 | 10.053 | 10.04 | 0053, 0054, 0067, 0068, 0075, 0076, 0089, 0090 |
| 637.091 | 10.827 | 10.84 | 0053, 0054, 0067, 0068, 0075, 0076, 0089, 0090 |
| 637.091 | 11.827 | 11.86 | 0053, 0054, 0067, 0068, 0075, 0076, 0089, 0090 |
| 637.091 | 12.023 | 12.01 | 0053, 0054, 0067, 0068, 0075, 0076, 0089, 0090 |
| 637.091 | 12.05 | 12.01 | 0053, 0054, 0067, 0068, 0075, 0076, 0089, 0090 |
| 637.091 | 12.88 | 12.86 | 0053, 0054, 0067, 0068, 0075, 0076, 0089, 0090 |
| 639.18 | 9.57 | 9.59 | 0105, 0106, 0097, 0098 |
| 639.18 | 9.667 | 9.69 | 0105, 0106, 0097, 0098 |
| 639.18 | 10.097 | 10.11 | 0105, 0106, 0097, 0098 |
| 639.18 | 12.45 | 12.45 | 0105, 0106, 0097, 0098 |
| 641.175 | 8.76 | 8.78 | 0069, 0070, 0077, 0078, 0083, 0084 |
| 641.175 | 8.737 | 8.72 | 0069, 0070, 0077, 0078, 0083, 0084 |

(continued)

| [Table 10-18-1] LCMS analysis results | | | |
|---|---|---|---|
| Mixture No. | | | 4-M00-1 |
| m/z | Retention time of MS peak (min) | Retention time of UV peak (min) | No. of assigned compound described with 4-M00-1-omitted |
| 641.175 | 9.577 | 9.59 | 0069, 0070, 0077, 0078, 0083, 0084 |
| 641.175 | 9.783 | 9.74 | 0069, 0070, 0077, 0078, 0083, 0084 |
| 641.175 | 10.48 | 10.46 | 0069, 0070, 0077, 0078, 0083, 0084 |
| 641.175 | 11.093 | 11.09 | 0069, 0070, 0077, 0078, 0083, 0084 |
| 643.157 | 10.31 | 10.26 | 0113, 0114, 0039, 0040, 0047, 0048, 0059, 0060 |
| 643.157 | 9.31 | 9.3 | 0113, 0114, 0039, 0040, 0047, 0048, 0059, 0060 |
| 643.157 | 9.39 | 9.41 | 0113, 0114, 0039, 0040, 0047, 0048, 0059, 0060 |
| 643.157 | 9.41 | 9.41 | 0113, 0114, 0039, 0040, 0047, 0048, 0059, 0060 |
| 643.157 | 10.137 | 10.11 | 0113, 0114, 0039, 0040, 0047, 0048, 0059, 0060 |
| 643.157 | 10.157 | 10.11 | 0113, 0114, 0039, 0040, 0047, 0048, 0059, 0060 |
| 643.157 | 10.853 | 10.84 | 0113, 0114, 0039, 0040, 0047, 0048, 0059, 0060 |
| 643.157 | 10.98 | 10.95 | 0113, 0114, 0039, 0040, 0047, 0048, 0059, 0060 |
| 645.15 | 9.687 | 9.69 | 0085, 0086 |
| 645.15 | 10.997 | 11.02 | 0085, 0086 |
| 647.132 | 10.603 | 10.58 | 0055, 0056, 0061, 0062 |
| 647.132 | 9.967 | 9.98 | 0055, 0056, 0061, 0062 |
| 647.132 | 10.117 | 10.11 | 0055, 0056, 0061, 0062 |
| 647.132 | 10.75 | 10.72 | 0055, 0056, 0061, 0062 |
| 651.216 | 9.943 | 9.98 | 0071, 0072, 0079, 0080, 0091 |
| 651.216 | 9.893 | 9.89 | 0071, 0072, 0079, 0080, 0091 |
| 651.216 | 10.013 | 10.04 | 0071, 0072, 0079, 0080, 0091 |
| 651.216 | 10.463 | 10.46 | 0071, 0072, 0079, 0080, 0091 |
| 651.216 | 10.56 | 10.58 | 0071, 0072, 0079, 0080, 0091 |
| 653.195 | 6.787 | 6.78 | 0100, 0107, 0108, 0121, 0122, 0099 |
| 653.195 | 6.807 | 6.78 | 0100, 0107, 0108, 0121, 0122, 0099 |
| 653.195 | 9.743 | 9.74 | 0100, 0107, 0108, 0121, 0122, 0099 |
| 653.195 | 9.793 | 9.83 | 0100, 0107, 0108, 0121, 0122, 0099 |
| 653.195 | 11.297 | 11.29 | 0100, 0107, 0108, 0121, 0122, 0099 |
| 653.195 | 12.897 | 12.93 | 0100, 0107, 0108, 0121, 0122, 0099 |
| 655.191 | 12.913 | 12.93 | 0087, 0088, 0092, 0093, 0094 |
| 657.172 | 9.417 | 9.42 | 0101, 0102, 0109, 0110, 0115, 0116, 0063, 0064 |
| 657.172 | 8.307 | 9.41 | 0101, 0102, 0109, 0110, 0115, 0116, 0063, 0064 |
| 657.172 | 10.61 | 10.63 | 0101, 0102, 0109, 0110, 0115, 0116, 0063, 0064 |
| 657.172 | 8.273 | 8.29 | 0101, 0102, 0109, 0110, 0115, 0116, 0063, 0064 |
| 657.172 | 8.287 | 8.29 | 0101, 0102, 0109, 0110, 0115, 0116, 0063, 0064 |
| 657.172 | 9.717 | 9.74 | 0101, 0102, 0109, 0110, 0115, 0116, 0063, 0064 |

(continued)

| [Table 10-18-1] LCMS analysis results | | | |
|---|---|---|---|
| Mixture No. | | | 4-M00-1 |
| m/z | Retention time of MS peak (min) | Retention time of UV peak (min) | No. of assigned compound described with 4-M00-1-omitted |
| 657.172 | 10.24 | 10.26 | 0101, 0102, 0109, 0110, 0115, 0116, 0063, 0064 |
| 657.172 | 10.76 | 10.79 | 0101, 0102, 0109, 0110, 0115, 0116, 0063, 0064 |
| 661.145 | 9.443 | 9.41 | 0117, 0118 |
| 661.145 | 9.3 | 9.3 | 0117, 0118 |
| 665.232 | 10.35 | 10.37 | 0095, 0096 |
| 665.232 | 11.113 | 11.09 | 0095, 0096 |
| 667.211 | 9.49 | 9.49 | 0103, 0104, 0111, 0112, 0123, 0124 |
| 667.211 | 10.163 | 10.11 | 0103, 0104, 0111, 0112, 0123, 0124 |
| 667.211 | 10.023 | 10.04 | 0103, 0104, 0111, 0112, 0123, 0124 |
| 667.211 | 9.803 | 9.83 | 0103, 0104, 0111, 0112, 0123, 0124 |
| 667.211 | 10.05 | 10.04 | 0103, 0104, 0111, 0112, 0123, 0124 |
| 667.211 | 10.073 | 10.04 | 0103, 0104, 0111, 0112, 0123, 0124 |
| 671.186 | 9.227 | 9.18 | 0119, 0120, 0125, 0126 |
| 671.186 | 9.837 | 9.83 | 0119, 0120, 0125, 0126 |
| 671.186 | 9.967 | 9.98 | 0119, 0120, 0125, 0126 |
| 671.186 | 9.33 | 9.34 | 0119, 0120, 0125, 0126 |
| 681.227 | 9.833 | 9.83 | 0127, 0128 |
| 681.227 | 9.947 | 9.98 | 0127, 0128 |

Table 10-18-1 shows the retention times of UV and MS peaks to which each compound was assigned, and the value of m/z $(M+H)^+$ detected in the time zone. As shown in Table 10-18-1, m/z $(M+H)^+$ peaks that corresponded to all of 256 compounds set as library compounds were able to be confirmed.

The results described above demonstrated that the step of identifying the scope of application of applicable building blocks using the highly robust reaction conditions and washing methods shown in Examples 2, 4, 8, and 9 is carried out so that a library having bond diversity can also be prepared with a high probability of success by a method of synthesizing a plurality of libraries still having the diversity of core blocks and also having linking moieties (linkers) that link building blocks, all of which are constituted by different types of bonds, followed by the mixing of the synthesized libraries.

The types of the bonds and the construction methods therefor in the preparation of a library having bond diversity by this approach are not limited to those used in Example 10.

Example 11; Evaluation of binding of synthesized mixture library compounds (8 types of mixtures libraries prepared in Example 8 and 8 types of mixtures libraries prepared in Example 9) to Bcl-2 using AS-MS

DMSO solutions (prepared into approximately 5 mM based on calculation from the average molecular weight of each mixture) of the mixtures of each Example shown in Table 11-1-1 were respectively dispensed to 8 wells of MICROPLATE, 96-WELL, PP, V- BOTTOM (Greiner Bio-One International GmbH, 651201). 400 nL each from the 8 wells of the mixtures obtained from Example 8 was dispensed into 3 designated wells while 200 nL each from the 8 wells of the mixtures obtained from Example 9 was dispensed into 3 designated wells, using LABCYTE ECHO(R) 555 acoustic dispensing system (Beckman Coulter, Inc.). 200 nL each of two types of 10 μM positive controls (B2Q045-01 and B2Q048-01) was dispensed to the mixtures obtained from Example 8. Likewise, 100 nL each of two types of 20 μM positive controls and 400 nL of a DMSO solution by backfill was dispensed to the mixtures obtained from Example 9. Further, Bcl-2 protein (Novus Biologicals, NBP2-34889, Recombinant Human Bcl-2 Protein, Lot E60145051) (19.2 μL)

prepared at 5 μM (final protein concentration) for the mixtures obtained from Example 8 and 10 μM (final protein concentration) for the mixtures obtained from Example 9, in advance using a Tris-HCl buffer (25 mM Tris-HCl, pH 7.4, 150 mM NaCl, 0.025% Tween-20, and 1 mM DTT) was added to the well containing the dispensed 1000 compounds or 500 compounds (final compound concentration: 100 nM), and the plate was incubated at 23°C for 1 hour in the dark. Subsequently, 200 μL of an assay buffer (25 mM Tris-HCl, pH 7.4, 150 mM NaCl, 0.025% Tween-20, and 1 mM DTT) was centrifuged three times at 2000 × G at 4°C for 2 minutes using 96-well MacroSpin column, P-2 Material (Harvard Bioscience, Inc., 74-5655) that had been allowed to swell to condition the 96-well MacroSpin column. Subsequently, 20 μL of the sample thus incubated was added to the 96-well MacroSpin column, P-2 Material after the completion of conditioning, and SEC treatment was performed by centrifugation at 2000 × G for 2 minutes. 32 μL of a 1:1 mixed solution of acetonitrile and methanol (LC/MS grade, FUJIFILM Wako Pure Chemical Corp.) was added to a flow- through liquid from SEC, and the mixture was centrifuged at 1800 × G at 4°C for 5 minutes. The supernatant was used as analysis sample ASMS (A). By a similar operation, a sample prepared at 500 compounds/well or 1000 compounds/well and supplemented with only a Tris-HCl buffer containing no Bcl-2 protein was incubated under the same conditions as above, and the same organic solvent as above was added thereto without 96-well MacroSpin column treatment. A supernatant obtained by a centrifugation operation was used as STD (B). Next, by a similar operation, a sample prepared at 1000 compounds/well or 500 compounds/well and supplemented with only a Tris-HCl buffer containing no Bcl-2 protein was incubated under the same conditions as above and treated with 96-well MacroSpin column in the same manner as above, and the same organic solvent as above was added thereto. A supernatant obtained by a centrifugation operation was used as BK (C). These samples were measured by reverse-phase LC/MS.

Then, the samples A, B and C were analyzed for the 1000 compounds or 500 compounds using TraceFinder 5.1 (Thermo Fisher Scientific Inc.) and CHANCE Software (in-house), and an ASMS ratio (%) was calculated according to the following expression.

$$\text{Expression: ASMS ratio (\%)} = (A - C) / B * 100$$

The mixtures obtained from Example 8 were tested at the number of data of 2 (N1 and N2) in order to ensure reproducibility. The mixtures obtained from Example 9 were tested at the total number of data 4 (N1, N2, N3, and N4) involving the number of data of 2/day (N1 and N2) and data obtained on additional days.

[Table 588]

| [Table 11-1-1] | | | |
|---|---|---|---|
| Table | No. of Example in which mixture was synthesized | Mixture name | The number of compounds mixed |
| Table 11-2-1 | 8 | 2-1-m1E01-1 | 1,000 |
| Table 11-2-2 | 8 | 2-1-m1E02-1 | 1,000 |
| Table 11-2-3 | 8 | 2-1-m1E03-1 | 1,000 |
| Table 11-2-4 | 8 | 2-1-m1E04-1 | 1,000 |
| Table 11-2-5 | 8 | 2-1-m1E01-2 | 1,000 |
| Table 11-2-6 | 8 | 2-1-m1E02-2 | 1,000 |
| Table 11-2-7 | 8 | 2-1-m1E03-2 | 1,000 |
| Table 11-2-8 | 8 | 2-1-m1E04-2 | 1,000 |
| Table 11-3-1 | 9 | 2-2-d1E01-1 | 500 |
| Table 11-3-2 | 9 | 2-2-d1E02-1 | 500 |
| Table 11-3-3 | 9 | 2-2-d1E03-1 | 500 |
| Table 11-3-4 | 9 | 2-2-d1E04-1 | 500 |
| Table 11-3-5 | 9 | 2-2-d1E01-2 | 500 |
| Table 11-3-6 | 9 | 2-2-d1E02-2 | 500 |
| Table 11-3-7 | 9 | 2-2-d1E03-2 | 500 |
| Table 11-3-8 | 9 | 2-2-d1E04-2 | 500 |

[Table 589]

| Reverse-phase LC/MS conditions | |
|---|---|
| Vanquish UHPLC (Thermo Fisher Scientific) | |
| Oven (°C) | 40 |
| Column | Column packed with chemically bonded porous spherical silica gel surface-modified with an ODS octadecylsilyl group as a stationary phase |
| Auto Sampler(°C) 4 | |
| Orbitrap Fusion Lumos Tribrid mass spectrometer (Thermo Fisher Scientific) | |
| Polarity | Positive mode |
| Data Type Centroid | |

The results are shown in Table 11-2-1 to Table 11-2-8 and Table 11-3-1 to Table 11- 3-8.

[Table 590]

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0001 | C36 H34 N4 O4 | 586.25801 | -12.0 | -0.2 |
| 2-1-m1E01-1-0002 | C35 H33 N5 O4 | 587.25325 | -9.4 | 4.1 |
| 2-1-m1E01-1-0003 | C35 H33 N5 O4 | 587.25325 | -10.5 | 2.2 |
| 2-1-m1E01-1-0004 | C34 H32 N6 O4 | 588.24850 | -5.3 | 0.4 |
| 2-1-m1E01-1-0005 | C34 H32 N6 O4 | 588.24850 | -7.1 | -1.4 |
| 2-1-m1E01-1-0006 | C33 H31 N7 O4 | 589.24375 | -17.2 | 4.7 |
| 2-1-m1E01-1-0007 | C33 H31 N5 O4 S | 593.20968 | -6.4 | 6.2 |
| 2-1-m1E01-1-0008 | C32 H30 N6 O4 S | 594.20492 | -17.5 | 1.8 |
| 2-1-m1E01-1-0009 | C37 H36 N4 O4 | 600.27366 | -29.6 | -2.4 |
| 2-1-m1E01-1-0010 | C36 H35 N5 O4 | 601.26890 | -0.1 | 1.2 |
| 2-1-m1E01-1-0011 | C35 H34 N6 O4 | 602.26415 | -19.6 | 15.1 |
| 2-1-m1E01-1-0012 | C35 H32 N4 O6 | 604.23218 | -1.9 | 3.7 |
| 2-1-m1E01-1-0013 | C36 H33 F N4 O4 | 604.24858 | -15.2 | 1.0 |
| 2-1-m1E01-1-0014 | C34 H31 N5 O6 | 605.22743 | -6.5 | 0.0 |
| 2-1-m1E01-1-0015 | C35 H32 F N5 O4 | 605.24383 | -12.1 | 4.2 |
| 2-1-m1E01-1-0016 | C35 H35 N5 O5 | 605.26382 | -9.6 | 2.6 |
| 2-1-m1E01-1-0017 | C34 H31 FN6 O4 | 606.23908 | -5.8 | 9.1 |
| 2-1-m1E01-1-0018 | C34 H34 N6 O5 | 606.25907 | -6.0 | 2.5 |
| 2-1-m1E01-1-0019 | C34 H34 N6 O5 | 606.25907 | -4.1 | 1.8 |
| 2-1-m1E01-1-0020 | C34 H33 N5 O4 S | 607.22533 | -28.4 | 0.7 |
| 2-1-m1E01-1-0021 | C33 H33 N7 O5 | 607.25432 | 0.0 | -1.3 |
| 2-1-m1E01-1-0022 | C33 H33 N7 O5 | 607.25432 | -11.9 | 8.6 |
| 2-1-m1E01-1-0023 | C32 H32 N8 O5 | 608.24957 | -7.1 | 1.2 |
| 2-1-m1E01-1-0024 | C33 H30 F N5 O4 S | 611.20025 | -7.0 | 4.5 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0025 | C32 H32 N6 O5 S | 612.21549 | -4.0 | 6.8 |
| 2-1-m1E01-1-0026 | C31 H31 N7 O5 S | 613.21074 | 0.0 | -0.6 |
| 2-1-m1E01-1-0027 | C37 H34 N4 O5 | 614.25292 | -6.7 | 1.2 |
| 2-1-m1E01-1-0028 | C38 H38 N4 O4 | 614.28931 | -8.9 | -5.8 |
| 2-1-m1E01-1-0029 | C36 H33 N5 O5 | 615.24817 | -6.0 | 0.3 |
| 2-1-m1E01-1-0030 | C36 H33 N5 O5 | 615.24817 | -4.1 | -4.3 |
| 2-1-m1E01-1-0031 | C37 H37 N5 O4 | 615.28455 | -22.2 | 8.2 |
| 2-1-m1E01-1-0032 | C35 H32 N6 O5 | 616.24342 | -3.2 | 1.4 |
| 2-1-m1E01-1-0033 | C37 H36 N4 O5 | 616.26857 | -4.3 | -1.9 |
| 2-1-m1E01-1-0034 | C36 H36 N6 O4 | 616.27980 | -9.6 | 3.6 |
| 2-1-m1E01-1-0035 | C36 H35 N5 O5 | 617.26382 | -11.1 | 3.6 |
| 2-1-m1E01-1-0036 | C36 H34 N4 O6 | 618.24783 | 0.0 | -8.2 |
| 2-1-m1E01-1-0037 | C35 H34 N6 O5 | 618.25907 | -3.9 | 5.9 |
| 2-1-m1E01-1-0038 | C36 H37 N5 O5 | 619.27947 | -9.2 | 7.4 |
| 2-1-m1E01-1-0039 | C35 H32 N4 O5 S | 620.20934 | -12.9 | -7.3 |
| 2-1-m1E01-1-0040 | C35 H36 N6 O5 | 620.27472 | -8.3 | 4.8 |
| 2-1-m1E01-1-0041 | C34 H31 N5 O5 S | 621.20459 | -5.3 | 1.0 |
| 2-1-m1E01-1-0042 | C35 H35 N5 O4 S | 621.24098 | 0.0 | -5.2 |
| 2-1-m1E01-1-0043 | C34 H35 N7 O5 | 621.26997 | -6.4 | 1.9 |
| 2-1-m1E01-1-0044 | C35 H31 F N4 O6 | 622.22276 | -10.7 | 4.1 |
| 2-1-m1E01-1-0045 | C36 H32 F2 N4 O4 | 622.23916 | -12.4 | 6.0 |
| 2-1-m1E01-1-0046 | C34 H33 N5 O5 S | 623.22024 | -20.1 | 3.8 |
| 2-1-m1E01-1-0047 | C35 H31 F2 N5 O4 | 623.23441 | -2.7 | 8.0 |
| 2-1-m1E01-1-0048 | C34 H33 N5 O7 | 623.23800 | 1.6 | 3.0 |
| 2-1-m1E01-1-0049 | C35 H34 F N5 O5 | 623.25440 | -7.2 | 4.7 |
| 2-1-m1E01-1-0050 | C34 H30 F2 N6 O4 | 624.22966 | -6.6 | -5.5 |
| 2-1-m1E01-1-0051 | C33 H32 N6 O7 | 624.23325 | -3.5 | 1.9 |
| 2-1-m1E01-1-0052 | C34 H33 F N6 O5 | 624.24965 | 0.3 | 5.3 |
| 2-1-m1E01-1-0053 | C33 H32 F N7 O5 | 625.24490 | -12.1 | 6.1 |
| 2-1-m1E01-1-0054 | C33 H34 N6 O5 S | 626.23114 | -21.0 | 1.6 |
| 2-1-m1E01-1-0055 | C38 H36 N4 O5 | 628.26857 | -9.3 | 7.1 |
| 2-1-m1E01-1-0056 | C33 H29 F2 N5 O4 S | 629.19083 | -13.7 | -1.2 |
| 2-1-m1E01-1-0057 | C37 H35 N5 O5 | 629.26382 | -7.2 | 2.5 |
| 2-1-m1E01-1-0058 | C37 H35 N5 O5 | 629.26382 | -3.8 | -1.2 |
| 2-1-m1E01-1-0059 | C32 H31 F N6 O5 S | 630.20607 | -6.2 | -17.2 |
| 2-1-m1E01-1-0060 | C36 H34 N6 O5 | 630.25907 | -3.7 | 0.3 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0061 | C37 H33 F N4 O5 | 632.24350 | -6.4 | 2.1 |
| 2-1-m1E01-1-0062 | C37 H33 F N4 O5 | 632.24350 | -11.2 | 4.5 |
| 2-1-m1E01-1-0063 | C37 H36 N4 O6 | 632.26348 | -4.1 | 3.7 |
| 2-1-m1E01-1-0064 | C36 H32 F N5 O5 | 633.23875 | -4.9 | 2.8 |
| 2-1-m1E01-1-0065 | C36 H32 F N5 O5 | 633.23875 | -7.3 | 5.0 |
| 2-1-m1E01-1-0066 | C36 H35 N5 O6 | 633.25873 | -4.8 | 1.3 |
| 2-1-m1E01-1-0067 | C37 H39 N5 O5 | 633.29512 | -8.9 | 1.8 |
| 2-1-m1E01-1-0068 | C36 H34 N4 O5 S | 634.22499 | -8.4 | -1.3 |
| 2-1-m1E01-1-0069 | C36 H34 N4 O7 | 634.24275 | -4.2 | 8.6 |
| 2-1-m1E01-1-0070 | C35 H34 N6 O6 | 634.25398 | -3.6 | -1.2 |
| 2-1-m1E01-1-0071 | C35 H34 N6 O6 | 634.25398 | -3.5 | 2.6 |
| 2-1-m1E01-1-0072 | C36 H38 N6 O5 | 634.29037 | -9.4 | 2.9 |
| 2-1-m1E01-1-0073 | C34 H33 N7 O6 | 635.24923 | -1.5 | 2.7 |
| 2-1-m1E01-1-0074 | C36 H37 N5 O6 | 635.27438 | -6.0 | 4.9 |
| 2-1-m1E01-1-0075 | C35 H37 N7 O5 | 635.28562 | -3.4 | 2.9 |
| 2-1-m1E01-1-0076 | C35 H36 N6 O6 | 636.26963 | -4.9 | 6.5 |
| 2-1-m1E01-1-0077 | C40 H36 N4 O4 | 636.27366 | 3.0 | -0.2 |
| 2-1-m1E01-1-0078 | C35 H35 N5 O7 | 637.25365 | -6.3 | 3.8 |
| 2-1-m1E01-1-0079 | C34 H35 N7 O6 | 637.26488 | 0.0 | 3.6 |
| 2-1-m1E01-1-0080 | C39 H35 N5 O4 | 637.26890 | -7.7 | -2.2 |
| 2-1-m1E01-1-0081 | C35 H31 F N4 O5 S | 638.19992 | -11.9 | 4.6 |
| 2-1-m1E01-1-0082 | C38 H34 N6 O4 | 638.26415 | -12.6 | 1.6 |
| 2-1-m1E01-1-0083 | C38 H34 N6 O4 | 638.26415 | -15.6 | 5.8 |
| 2-1-m1E01-1-0084 | C34 H33 N5 O6 S | 639.21515 | -1.1 | 2.1 |
| 2-1-m1E01-1-0085 | C37 H33 N7 O4 | 639.25940 | -6.9 | -3.6 |
| 2-1-m1E01-1-0086 | C34 H32 N4 O7 S | 640.19917 | -21.4 | -6.4 |
| 2-1-m1E01-1-0087 | C33 H32 N6 O6 S | 640.21040 | -4.4 | 0.2 |
| 2-1-m1E01-1-0088 | C35 H30 F2 N4 O6 | 640.21334 | -4.2 | 0.1 |
| 2-1-m1E01-1-0089 | C34 H36 N6 O5 S | 640.24679 | -4.2 | 0.6 |
| 2-1-m1E01-1-0090 | C33 H31 N5 O7 S | 641.19442 | 0.0 | 5.6 |
| 2-1-m1E01-1-0091 | C34 H32 F N5 O7 | 641.22858 | -4.8 | 1.2 |
| 2-1-m1E01-1-0092 | C35 H33 F2 N5 O5 | 641.24498 | -18.1 | -2.5 |
| 2-1-m1E01-1-0093 | C33 H34 N6 O6 S | 642.22605 | 0.0 | 0.0 |
| 2-1-m1E01-1-0094 | C38 H34 N4 O4 S | 642.23008 | -2.6 | 2.4 |
| 2-1-m1E01-1-0095 | C34 H32 F2 N6 O5 | 642.24022 | -15.7 | 3.3 |
| 2-1-m1E01-1-0096 | C35 H38 N4 O6 S | 642.25121 | -4.5 | -6.5 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0097 | C39 H38 N4 O5 | 642.28422 | -5.9 | 3.2 |
| 2-1-m1E01-1-0098 | C37 H33 N5 O4 S | 643.22533 | -6.1 | -3.8 |
| 2-1-m1E01-1-0099 | C37 H33 N5 O4 S | 643.22533 | 0.0 | 0.0 |
| 2-1-m1E01-1-0100 | C33 H31 F2 N7 O5 | 643.23547 | -6.4 | 6.7 |
| 2-1-m1E01-1-0101 | C34 H37 N5 O6 S | 643.24645 | -5.4 | 0.3 |
| 2-1-m1E01-1-0102 | C38 H37 N5 O5 | 643.27947 | -8.6 | 2.6 |
| 2-1-m1E01-1-0103 | C38 H37 N5 O5 | 643.27947 | -2.3 | 10.4 |
| 2-1-m1E01-1-0104 | C38 H36 N4 O6 | 644.26348 | -7.2 | 0.2 |
| 2-1-m1E01-1-0105 | C37 H35 N5 O6 | 645.25873 | -5.9 | 1.0 |
| 2-1-m1E01-1-0106 | C38 H35 F N4 O5 | 646.25915 | -7.3 | 1.3 |
| 2-1-m1E01-1-0107 | C39 H42 N4 O5 | 646.31552 | -12.0 | -7.2 |
| 2-1-m1E01-1-0108 | C37 H34 F N5 O5 | 647.25440 | -6.4 | 1.3 |
| 2-1-m1E01-1-0109 | C37 H37 N5 O6 | 647.27438 | -5.9 | 0.2 |
| 2-1-m1E01-1-0110 | C38 H41 N5 O5 | 647.31077 | -5.0 | 4.7 |
| 2-1-m1E01-1-0111 | C32 H30 F2 N6 O5 S | 648.19665 | -20.4 | 2.8 |
| 2-1-m1E01-1-0112 | C37 H36 N4 O5 S | 648.24064 | -9.5 | 3.6 |
| 2-1-m1E01-1-0113 | C36 H36 N6 O6 | 648.26963 | -3.1 | 0.2 |
| 2-1-m1E01-1-0114 | C36 H36 N6 O6 | 648.26963 | -8.9 | 0.9 |
| 2-1-m1E01-1-0115 | C35 H35 N7 O6 | 649.26488 | 0.4 | -1.1 |
| 2-1-m1E01-1-0116 | C36 H34 N4 O6 S | 650.21991 | -4.6 | 3.0 |
| 2-1-m1E01-1-0117 | C36 H34 N4 O6 S | 650.21991 | -7.0 | 3.0 |
| 2-1-m1E01-1-0118 | C36 H34 N4 O6 S | 650.21991 | 0.0 | 0.0 |
| 2-1-m1E01-1-0119 | C37 H32 F2 N4 O5 | 650.23408 | -10.0 | 2.8 |
| 2-1-m1E01-1-0120 | C37 H32 F2 N4 O5 | 650.23408 | -10.0 | 2.8 |
| 2-1-m1E01-1-0121 | C35 H33 N5 O6 S | 651.21515 | -6.0 | 0.5 |
| 2-1-m1E01-1-0122 | C35 H33 N5 O6 S | 651.21515 | -4.0 | 7.7 |
| 2-1-m1E01-1-0123 | C35 H33 N5 O6 S | 651.21515 | -5.8 | 1.1 |
| 2-1-m1E01-1-0124 | C36 H31 F2 N5 O5 | 651.22933 | 7.0 | 3.9 |
| 2-1-m1E01-1-0125 | C36 H34 F N5 O6 | 651.24931 | -8.3 | 2.7 |
| 2-1-m1E01-1-0126 | C36 H34 F N5 O6 | 651.24931 | 0.0 | -1.6 |
| 2-1-m1E01-1-0127 | C36 H37 N5 O7 | 651.26930 | -5.8 | 1.6 |
| 2-1-m1E01-1-0128 | C34 H32 N6 O6 S | 652.21040 | -2.8 | 0.7 |
| 2-1-m1E01-1-0129 | C34 H32 N6 O6 S | 652.21040 | -0.5 | 1.4 |
| 2-1-m1E01-1-0130 | C35 H33 F N6 O6 | 652.24456 | -3.4 | 0.0 |
| 2-1-m1E01-1-0131 | C35 H33 F N6 O6 | 652.24456 | -0.1 | 3.1 |
| 2-1-m1E01-1-0132 | C39 H36 N6 O4 | 652.27980 | -7.8 | 2.3 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0133 | C33 H31 N7 O6 S | 653.20565 | -2.2 | 1.2 |
| 2-1-m1E01-1-0134 | C35 H35 N5 O6 S | 653.23080 | -5.2 | 2.2 |
| 2-1-m1E01-1-0135 | C35 H35 N5 O8 | 653.24856 | -3.9 | 0.3 |
| 2-1-m1E01-1-0136 | C35 H34 N4 O7 S | 654.21482 | 0.0 | 0.0 |
| 2-1-m1E01-1-0137 | C37 H33 F3 N4 O4 | 654.24539 | -18.7 | -4.0 |
| 2-1-m1E01-1-0138 | C37 H33 F3 N4 O4 | 654.24539 | -3.9 | 12.5 |
| 2-1-m1E01-1-0139 | C39 H34 N4 O6 | 654.24783 | -9.3 | 3.4 |
| 2-1-m1E01-1-0140 | C36 H32 F3 N5 O4 | 655.24064 | -8.9 | -3.6 |
| 2-1-m1E01-1-0141 | C36 H32 F3 N5 O4 | 655.24064 | -2.4 | -2.9 |
| 2-1-m1E01-1-0142 | C34 H32 N4 O6 S2 | 656.17633 | -7.2 | 5.4 |
| 2-1-m1E01-1-0143 | C35 H30 F2 N4 O5 S | 656.19050 | -4.9 | 0.7 |
| 2-1-m1E01-1-0144 | C35 H31 F3 N6 O4 | 656.23589 | -10.0 | -5.6 |
| 2-1-m1E01-1-0145 | C39 H36 N4 O4 S | 656.24573 | -3.7 | 0.5 |
| 2-1-m1E01-1-0146 | C38 H33 F N6 O4 | 656.25473 | -20.1 | 8.4 |
| 2-1-m1E01-1-0147 | C36 H40 N4 O6 S | 656.26686 | 0.0 | 14.5 |
| 2-1-m1E01-1-0148 | C33 H31 N5 O6 S2 | 657.17158 | -8.6 | 3.8 |
| 2-1-m1E01-1-0149 | C33 H31 N5 O6 S2 | 657.17158 | -6.2 | 3.7 |
| 2-1-m1E01-1-0150 | C34 H32 F N5 O6 S | 657.20573 | -4.0 | 2.5 |
| 2-1-m1E01-1-0151 | C37 H35 N7 O5 | 657.26997 | -9.5 | 2.3 |
| 2-1-m1E01-1-0152 | C39 H39 N5 O5 | 657.29512 | -5.7 | 2.1 |
| 2-1-m1E01-1-0153 | C32 H30 N6 O6 S2 | 658.16682 | -6.9 | -0.1 |
| 2-1-m1E01-1-0154 | C34 H31 F N4 O7 S | 658.18975 | -20.9 | 3.1 |
| 2-1-m1E01-1-0155 | C36 H34 N8 O5 | 658.26522 | 0.0 | 0.0 |
| 2-1-m1E01-1-0156 | C33 H33 N5 O8 S | 659.20498 | -7.0 | 1.5 |
| 2-1-m1E01-1-0157 | C34 H31 F2 N5 O7 | 659.21915 | -1.2 | 3.3 |
| 2-1-m1E01-1-0158 | C38 H37 N5 O6 | 659.27438 | -5.1 | 1.5 |
| 2-1-m1E01-1-0159 | C32 H32 N6 O8 S | 660.20023 | -5.3 | 1.1 |
| 2-1-m1E01-1-0160 | C38 H33 F N4 O4 S | 660.22065 | -1.8 | -0.1 |
| 2-1-m1E01-1-0161 | C35 H37 F N4 O6 S | 660.24178 | -4.7 | 1.4 |
| 2-1-m1E01-1-0162 | C39 H37 F N4 O5 | 660.27480 | -9.6 | 4.3 |
| 2-1-m1E01-1-0163 | C40 H44 N4 O5 | 660.33117 | -3.8 | 7.1 |
| 2-1-m1E01-1-0164 | C34 H30 F3 N5 O4 S | 661.19706 | -7.6 | 1.7 |
| 2-1-m1E01-1-0165 | C37 H35 N5 O5 S | 661.23589 | -9.8 | 0.1 |
| 2-1-m1E01-1-0166 | C34 H39 N5 O7 S | 661.25702 | -8.8 | 2.6 |
| 2-1-m1E01-1-0167 | C38 H39 N5 O6 | 661.29003 | -6.3 | 0.7 |
| 2-1-m1E01-1-0168 | C36 H34 N6 O5 S | 662.23114 | -8.2 | -6.0 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0169 | C36 H34 N6 O5 S | 662.23114 | 0.0 | 0.0 |
| 2-1-m1E01-1-0170 | C33 H38 N6 O7 S | 662.25227 | -3.1 | 0.3 |
| 2-1-m1E01-1-0171 | C38 H35 F N4 O6 | 662.25406 | -3.1 | 0.3 |
| 2-1-m1E01-1-0172 | C38 H38 N4 O5 S | 662.25629 | -6.6 | 1.2 |
| 2-1-m1E01-1-0173 | C37 H38 N6 O6 | 662.28528 | -3.6 | -0.7 |
| 2-1-m1E01-1-0174 | C37 H38 N6 O6 | 662.28528 | -5.8 | 1.1 |
| 2-1-m1E01-1-0175 | C35 H33 N7 O5 S | 663.22639 | -8.3 | 1.7 |
| 2-1-m1E01-1-0176 | C37 H37 N5 O5 S | 663.25154 | -21.2 | 2.7 |
| 2-1-m1E01-1-0177 | C37 H37 N5 O7 | 663.26930 | -1.4 | 1.5 |
| 2-1-m1E01-1-0178 | C34 H32 N8 O5 S | 664.22164 | -5.0 | 4.1 |
| 2-1-m1E01-1-0179 | C37 H36 N4 O6 S | 664.23556 | -11.1 | 1.4 |
| 2-1-m1E01-1-0180 | C37 H36 N4 O6 S | 664.23556 | -7.1 | 3.7 |
| 2-1-m1E01-1-0181 | C37 H36 N4 O6 S | 664.23556 | -0.5 | 3.2 |
| 2-1-m1E01-1-0182 | C36 H36 N6 O7 | 664.26455 | 0.0 | 0.0 |
| 2-1-m1E01-1-0183 | C41 H36 N4 O5 | 664.26857 | -6.7 | 3.2 |
| 2-1-m1E01-1-0184 | C39 H41 F N4 O5 | 664.30610 | -5.3 | -1.7 |
| 2-1-m1E01-1-0185 | C36 H35 N5 O6 S | 665.23080 | -4.0 | 0.3 |
| 2-1-m1E01-1-0186 | C36 H35 N5 O6 S | 665.23080 | -2.9 | 2.2 |
| 2-1-m1E01-1-0187 | C36 H35 N5 O6 S | 665.23080 | -5.0 | 4.0 |
| 2-1-m1E01-1-0188 | C37 H33 F2 N5 O5 | 665.24498 | -11.7 | 2.1 |
| 2-1-m1E01-1-0189 | C40 H35 N5 O5 | 665.26382 | -7.6 | 2.4 |
| 2-1-m1E01-1-0190 | C37 H36 F N5 O6 | 665.26496 | -5.6 | 2.2 |
| 2-1-m1E01-1-0191 | C38 H43 N5 O6 | 665.32133 | 0.0 | 0.0 |
| 2-1-m1E01-1-0192 | C35 H34 N6 O6 S | 666.22605 | -6.5 | -2.7 |
| 2-1-m1E01-1-0193 | C35 H34 N6 O6 S | 666.22605 | -6.2 | 5.3 |
| 2-1-m1E01-1-0194 | C36 H35 F N6 O6 | 666.26021 | -1.2 | 0.1 |
| 2-1-m1E01-1-0195 | C40 H38 N6 04 | 666.29545 | -3.9 | 3.2 |
| 2-1-m1E01-1-0196 | C37 H42 N6 O6 | 666.31658 | -3.6 | 0.2 |
| 2-1-m1E01-1-0197 | C36 H37 N5 O6 S | 667.24645 | -7.8 | 0.8 |
| 2-1-m1E01-1-0198 | C35 H32 N4 O8 S | 668.19408 | -4.8 | 1.9 |
| 2-1-m1E01-1-0199 | C36 H33 F N4 O6 S | 668.21048 | -6.4 | 1.7 |
| 2-1-m1E01-1-0200 | C36 H33 F N4 O6 S | 668.21048 | -5.8 | 7.0 |
| 2-1-m1E01-1-0201 | C37 H31 F3 N4 O5 | 668.22465 | -7.6 | 6.3 |
| 2-1-m1E01-1-0202 | C36 H36 N4 O7 S | 668.23047 | 0.0 | 0.0 |
| 2-1-m1E01-1-0203 | C38 H35 F3 N4 04 | 668.26104 | -9.2 | 10.3 |
| 2-1-m1E01-1-0204 | C39 H36 N6 O5 | 668.27472 | -22.6 | -1.0 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0205 | C33 H31 N7 05 S2 | 669.18281 | -17.4 | -0.5 |
| 2-1-m1E01-1-0206 | C34 H31 N5 O8 S | 669.18933 | -1.5 | 0.0 |
| 2-1-m1E01-1-0207 | C35 H32 F N5 O6 S | 669.20573 | -5.8 | 1.0 |
| 2-1-m1E01-1-0208 | C35 H35 N5 O7 S | 669.22572 | -3.0 | 0.3 |
| 2-1-m1E01-1-0209 | C35 H35 N5 O7 S | 669.22572 | 0.0 | 1.7 |
| 2-1-m1E01-1-0210 | C35 H35 N5 O7 S | 669.22572 | -6.3 | 2.6 |
| 2-1-m1E01-1-0211 | C36 H33 F2 N5 O6 | 669.23989 | -5.4 | 0.7 |
| 2-1-m1E01-1-0212 | C36 H33 F2 N5 O6 | 669.23989 | 0.0 | 7.9 |
| 2-1-m1E01-1-0213 | C35 H34 N4 O6 S2 | 670.19198 | -2.0 | 0.6 |
| 2-1-m1E01-1-0214 | C34 H31 F N6 O6 S | 670.20098 | -9.7 | 7.3 |
| 2-1-m1E01-1-0215 | C35 H34 N4 O8 S | 670.20973 | -1.6 | -1.1 |
| 2-1-m1E01-1-0216 | C34 H34 N6 O7 S | 670.22097 | -5.0 | 1.3 |
| 2-1-m1E01-1-0217 | C34 H34 N6 O7 S | 670.22097 | -3.6 | 3.9 |
| 2-1-m1E01-1-0218 | C34 H34 N6 O7 S | 670.22097 | -3.3 | 0.4 |
| 2-1-m1E01-1-0219 | C39 H34 N4 O5 S | 670.22499 | -10.1 | 13.3 |
| 2-1-m1E01-1-0220 | C35 H32 F2 N6 O6 | 670.23514 | -8.0 | 3.6 |
| 2-1-m1E01-1-0221 | C37 H33 F3 N4 O5 | 670.24030 | 0.0 | -4.8 |
| 2-1-m1E01-1-0222 | C40 H38 N4 O4 S | 670.26138 | 0.0 | 5.5 |
| 2-1-m1E01-1-0223 | C37 H42 N4 O6 S | 670.28251 | -4.1 | 5.7 |
| 2-1-m1E01-1-0224 | C34 H33 N5 O6 S2 | 671.18723 | -32.6 | 11.3 |
| 2-1-m1E01-1-0225 | C33 H33 N7 O7 S | 671.21622 | 0.0 | 0.0 |
| 2-1-m1E01-1-0226 | C33 H33 N7 O7 S | 671.21622 | -0.4 | 0.1 |
| 2-1-m1E01-1-0227 | C36 H32 F3 N5 O5 | 671.23555 | -14.1 | 6.0 |
| 2-1-m1E01-1-0228 | C38 H37 N7 O5 | 671.28562 | -14.9 | 17.8 |
| 2-1-m1E01-1-0229 | C32 H32 N8 O7 S | 672.21147 | 0.0 | 0.0 |
| 2-1-mlE01-1-0230 | C36 H31 F3 N4 O6 | 672.21957 | 0.0 | 0.0 |
| 2-1-m1E01-1-0231 | C35 H31 F3 N6 O5 | 672.23080 | -9.7 | 3.7 |
| 2-1-m1E01-1-0232 | C37 H32 F4 N4 O4 | 672.23597 | -13.8 | 4.3 |
| 2-1-m1E01-1-0233 | C39 H36 N4 O5 S | 672.24064 | -4.2 | 0.0 |
| 2-1-m1E01-1-0234 | C36 H40 N4 O7 S | 672.26177 | -5.1 | -4.9 |
| 2-1-m1E01-1-0235 | C34 H35 N5 O8 S | 673.22063 | -7.5 | 2.4 |
| 2-1-m1E01-1-0236 | C36 H34 F3 N5 O5 | 673.25120 | 2.3 | 2.8 |
| 2-1-m1E01-1-0237 | C36 H34 F3 N5 O5 | 673.25120 | -7.3 | 6.3 |
| 2-1-m1E01-1-0238 | C34 H31 F N4 O6 S2 | 674.16690 | -3.8 | 5.4 |
| 2-1-m1E01-1-0239 | C38 H32 F2 N6 O4 | 674.24531 | -5.3 | 1.0 |
| 2-1-m1E01-1-0240 | C35 H33 F3 N6 O5 | 674.24645 | -7.3 | 5.1 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0241 | C35 H33 F3 N6 O5 | 674.24645 | -4.2 | 1.3 |
| 2-1-m1E01-1-0242 | C41 H46 N4 05 | 674.34682 | -1.7 | 3.8 |
| 2-1-m1E01-1-0243 | C33 H30 F N5 O6 S2 | 675.16215 | -2.9 | 7.9 |
| 2-1-m1E01-1-0244 | C33 H33 N5 O7 S2 | 675.18214 | -9.4 | 3.5 |
| 2-1-m1E01-1-0245 | C34 H31 F2 N5 O6 S | 675.19631 | -4.7 | 2.8 |
| 2-1-m1E01-1-0246 | C34 H32 F3 N7 O5 | 675.24170 | -5.6 | 1.6 |
| 2-1-m1E01-1-0247 | C38 H37 N5 05 S | 675.25154 | -9.7 | -0.4 |
| 2-1-m1E01-1-0248 | C37 H34 F N7 O5 | 675.26055 | -7.8 | 9.1 |
| 2-1-m1E01-1-0249 | C35 H41 N5 O7 S | 675.27267 | 0.0 | 4.4 |
| 2-1-m1E01-1-0250 | C32 H32 N6 O7 S2 | 676.17739 | -3.3 | 1.3 |
| 2-1-m1E01-1-0251 | C32 H32 N6 O7 S2 | 676.17739 | -7.0 | 0.0 |
| 2-1-m1E01-1-0252 | C34 H30 F2 N4 O7 S | 676.18033 | -3.0 | 2.2 |
| 2-1-m1E01-1-0253 | C39 H40 N4 O5 S | 676.27194 | 0.0 | 0.0 |
| 2-1-m1E01-1-0254 | C39 H40 N4 O5 S | 676.27194 | 0.0 | 0.0 |
| 2-1-m1E01-1-0255 | C38 H40 N6 O6 | 676.30093 | -4.0 | 0.7 |
| 2-1-m1E01-1-0256 | C40 H44 N4 O6 | 676.32609 | -39.7 | -0.6 |
| 2-1-m1E01-1-0257 | C31 H31 N7 O7 S2 | 677.17264 | 0.0 | 0.0 |
| 2-1-m1E01-1-0258 | C34 H30 F3 N5 O5 S | 677.19197 | -48.0 | 11.6 |
| 2-1-m1E01-1-0259 | C33 H32 F N5 O8 S | 677.19556 | -5.1 | 2.2 |
| 2-1-m1E01-1-0260 | C38 H39 N5 O5 S | 677.26719 | 0.0 | 0.0 |
| 2-1-m1E01-1-0261 | C38 H32 F2 N4 O4 S | 678.21123 | -2.5 | -6.7 |
| 2-1-m1E01-1-0262 | C37 H34 N4 O7 S | 678.21482 | -3.4 | 1.7 |
| 2-1-m1E01-1-0263 | C35 H36 F2 N4 O6 S | 678.23236 | -3.8 | 1.4 |
| 2-1-m1E01-1-0264 | C38 H38 N4 O6 S | 678.25121 | -7.3 | 15.3 |
| 2-1-m1E01-1-0265 | C38 H38 N4 O6 S | 678.25121 | 2.0 | 0.3 |
| 2-1-m1E01-1-0266 | C38 H38 N4 O6 S | 678.25121 | -7.7 | 6.4 |
| 2-1-m1E01-1-0267 | C38 H38 N4 O6 S | 678.25121 | 0.0 | 0.0 |
| 2-1-m1E01-1-0268 | C37 H38 N6 O7 | 678.28020 | 0.0 | -1.7 |
| 2-1-m1E01-1-0269 | C36 H33 N5 O7 S | 679.21007 | -1.5 | 0.6 |
| 2-1-m1E01-1-0270 | C36 H33 N5 O7 S | 679.21007 | -10.6 | -2.2 |
| 2-1-m1E01-1-0271 | C37 H34 F N5 O5 S | 679.22647 | 0.0 | 0.0 |
| 2-1-m1E01-1-0272 | C37 H37 N5 O6 S | 679.24645 | 3.4 | 1.5 |
| 2-1-m1E01-1-0273 | C37 H37 N5 O6 S | 679.24645 | 1.2 | 3.9 |
| 2-1-m1E01-1-0274 | C37 H37 N5 O6 S | 679.24645 | -8.7 | 0.1 |
| 2-1-m1E01-1-0275 | C34 H38 F N5 O7 S | 679.24760 | -1.1 | 1.0 |
| 2-1-m1E01-1-0276 | C41 H37 N5 O5 | 679.27947 | -6.9 | 3.3 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0277 | C38 H38 F N5 O6 | 679.28061 | -6.8 | 0.0 |
| 2-1-m1E01-1-0278 | C39 H45 N5 O6 | 679.33698 | -5.1 | 1.3 |
| 2-1-m1E01-1-0279 | C33 H31 F3 N6 O5 S | 680.20287 | -13.1 | 2.1 |
| 2-1-m1E01-1-0280 | C35 H32 N6 O7 S | 680.20532 | -5.1 | -2.5 |
| 2-1-m1E01-1-0281 | C35 H32 N6 O7 S | 680.20532 | -7.6 | 4.5 |
| 2-1-m1E01-1-0282 | C37 H36 N4 O7 S | 680.23047 | 0.0 | 0.0 |
| 2-1-m1E01-1-0283 | C37 H36 N4 O7 S | 680.23047 | -5.8 | 5.8 |
| 2-1-m1E01-1-0284 | C36 H36 N6 O6 S | 680.24170 | -5.0 | -0.1 |
| 2-1-m1E01-1-0285 | C38 H37 F N4 O5 S | 680.24687 | 5.7 | 0.0 |
| 2-1-m1E01-1-0286 | C36 H35 N5 O7 S | 681.22572 | -4.1 | 0.9 |
| 2-1-m1E01-1-0287 | C37 H36 F N5 O7 | 681.25988 | 0.0 | 0.0 |
| 2-1-m1E01-1-0288 | C37 H39 N5 O6 S | 681.26210 | -9.6 | -0.3 |
| 2-1-m1E01-1-0289 | C36 H34 N4 O8 S | 682.20973 | -2.9 | -0.7 |
| 2-1-m1E01-1-0290 | C35 H34 N6 O7 S | 682.22097 | -2.1 | -0.1 |
| 2-1-m1E01-1-0291 | C37 H35 F N4 O6 S | 682.22613 | -16.5 | 2.7 |
| 2-1-m1E01-1-0292 | C37 H35 F N4 O6 S | 682.22613 | 0.0 | -8.3 |
| 2-1-m1E01-1-0293 | C38 H33 F3 N4 O5 | 682.24030 | -5.7 | 5.6 |
| 2-1-m1E01-1-0294 | C36 H38 N6 O6 S | 682.25735 | -4.7 | 3.1 |
| 2-1-m1E01-1-0295 | C41 H35 F N4 O5 | 682.25915 | -1.6 | 4.9 |
| 2-1-m1E01-1-0296 | C39 H37 F3 N4 O4 | 682.27669 | -50.2 | -0.8 |
| 2-1-m1E01-1-0297 | C39 H40 F2 N4 O5 | 682.29668 | -11.7 | 2.8 |
| 2-1-m1E01-1-0298 | C36 H34 F N5 O6 S | 683.22138 | -5.9 | 0.0 |
| 2-1-m1E01-1-0299 | C36 H34 F N5 O6 S | 683.22138 | 0.0 | 0.0 |
| 2-1-m1E01-1-0300 | C37 H32 F3 N5 O5 | 683.23555 | -6.4 | 3.6 |
| 2-1-m1E01-1-0301 | C36 H37 N5 O7 S | 683.24137 | -6.2 | 0.5 |
| 2-1-m1E01-1-0302 | C36 H37 N5 O7 S | 683.24137 | -0.4 | 1.1 |
| 2-1-m1E01-1-0303 | C36 H37 N5 O7 S | 683.24137 | -11.3 | 10.7 |
| 2-1-m1E01-1-0304 | C38 H42 F N5 O6 | 683.31191 | -3.2 | 5.7 |
| 2-1-m1E01-1-0305 | C35 H32 N4 O7 S2 | 684.17124 | -3.5 | 1.9 |
| 2-1-m1E01-1-0306 | C36 H36 N4 O6 S2 | 684.20763 | -9.1 | -15.3 |
| 2-1-m1E01-1-0307 | C35 H36 N6 O7 S | 684.23662 | -3.7 | 2.3 |
| 2-1-m1E01-1-0308 | C35 H36 N6 O7 S | 684.23662 | -2.2 | 0.0 |
| 2-1-m1E01-1-0309 | C35 H36 N6 O7 S | 684.23662 | -0.6 | -4.1 |
| 2-1-m1E01-1-0310 | C36 H34 F2 N6 O6 | 684.25079 | -5.7 | 42.0 |
| 2-1-m1E01-1-0311 | C38 H35 F3 N4 O5 | 684.25595 | -1.4 | 2.6 |
| 2-1-m1E01-1-0312 | C34 H31 N5 O7 S2 | 685.16649 | -1.3 | 0.7 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0313 | C35 H35 N5 O6 S2 | 685.20288 | 0.0 | 1.7 |
| 2-1-m1E01-1-0314 | C34 H35 N7 O7 S | 685.23187 | 0.0 | 0.0 |
| 2-1-m1E01-1-0315 | C34 H35 N7 O7 S | 685.23187 | 0.0 | 0.0 |
| 2-1-m1E01-1-0316 | C39 H39 N7 O5 | 685.30127 | -10.4 | 2.7 |
| 2-1-m1E01-1-0317 | C35 H31 F N4 O8 S | 686.18466 | -1.8 | 0.7 |
| 2-1-m1E01-1-0318 | C35 H34 N4 O7 S2 | 686.18689 | -3.2 | 0.5 |
| 2-1-m1E01-1-0319 | C36 H32 F2 N4 O6 S | 686.20106 | -6.1 | 0.6 |
| 2-1-m1E01-1-0320 | C36 H32 F2 N4 O6 S | 686.20106 | -3.7 | 1.1 |
| 2-1-m1E01-1-0321 | C34 H33 N5 O7 S2 | 687.18214 | -1.9 | -3.0 |
| 2-1-m1E01-1-0322 | C35 H31 F2 N5 O6 S | 687.19631 | -3.6 | 1.8 |
| 2-1-m1E01-1-0323 | C34 H33 N5 O9 S | 687.19990 | -4.8 | 0.0 |
| 2-1-m1E01-1-0324 | C35 H34 F N5 O7 S | 687.21630 | -6.4 | 0.1 |
| 2-1-m1E01-1-0325 | C35 H34 F N5 O7 S | 687.21630 | -3.0 | 1.3 |
| 2-1-m1E01-1-0326 | C36 H32 F3 N5 O6 | 687.23047 | -3.9 | 4.0 |
| 2-1-m1E01-1-0327 | C35 H37 N5 O8 S | 687.23628 | -9.5 | 6.1 |
| 2-1-m1E01-1-0328 | C37 H36 F3 N5 O5 | 687.26685 | -5.2 | 1.6 |
| 2-1-m1E01-1-0329 | C38 H37 N7 O6 | 687.28053 | 0.0 | 0.1 |
| 2-1-m1E01-1-0330 | C34 H30 F2 N6 O6 S | 688.19156 | -10.0 | -4.4 |
| 2-1-m1E01-1-0331 | C33 H32 N6 O9 S | 688.19515 | 7.4 | 10.0 |
| 2-1-m1E01-1-0332 | C36 H31 F3 N4 O5 S | 688.19673 | 0.2 | 0.0 |
| 2-1-m1E01-1-0333 | C34 H33 F N6 O7 S | 688.21155 | -1.4 | 0.6 |
| 2-1-m1E01-1-0334 | C36 H31 F3 N4 O7 | 688.21448 | -2.9 | 2.4 |
| 2-1-m1E01-1-0335 | C42 H36 N6 04 | 688.27980 | -9.3 | 3.0 |
| 2-1-m1E01-1-0336 | C34 H35 N5 O7 S2 | 689.19779 | 0.0 | 0.0 |
| 2-1-m1E01-1-0337 | C33 H32 F N7 O7 S | 689.20680 | 0.0 | 0.0 |
| 2-1-m1E01-1-0338 | C34 H35 N5 O9 S | 689.21555 | -1.4 | 1.2 |
| 2-1-m1E01-1-0339 | C36 H34 F3 N5 O6 | 689.24612 | -8.9 | 2.1 |
| 2-1-m1E01-1-0340 | C39 H39 N5 O5 S | 689.26719 | -9.9 | 5.2 |
| 2-1-m1E01-1-0341 | C36 H43 N5 O7 S | 689.28832 | -6.2 | 0.0 |
| 2-1-m1E01-1-0342 | C33 H34 N6 O7 S2 | 690.19304 | -5.3 | 1.5 |
| 2-1-m1E01-1-0343 | C38 H34 N4 O7 S | 690.21482 | 0.0 | 0.0 |
| 2-1-m1E01-1-0344 | C37 H34 N6 O6 S | 690.22605 | -7.2 | -0.2 |
| 2-1-m1E01-1-0345 | C37 H31 F5 N4 O4 | 690.22655 | -31.9 | 3.2 |
| 2-1-m1E01-1-0346 | C35 H33 F3 N6 O6 | 690.24137 | -6.7 | 5.5 |
| 2-1-m1E01-1-0347 | C40 H42 N4 O5 S | 690.28759 | 0.0 | 0.0 |
| 2-1-m1E01-1-0348 | C40 H42 N4 O5 S | 690.28759 | 0.0 | 0.0 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0349 | C40 H42 N4 O5 S | 690.28759 | 0.0 | 12.3 |
| 2-1-m1E01-1-0350 | C36 H33 N7 O6 S | 691.22130 | -1.9 | -1.1 |
| 2-1-m1E01-1-0351 | C35 H32 F3 N5 O7 | 691.22538 | -4.8 | 3.2 |
| 2-1-m1E01-1-0352 | C34 H32 F3 N7 O6 | 691.23662 | -0.3 | 5.3 |
| 2-1-m1E01-1-0353 | C36 H33 F4 N5 O5 | 691.24178 | -5.1 | 2.4 |
| 2-1-m1E01-1-0354 | C38 H37 N5 O6 S | 691.24645 | -9.9 | 4.1 |
| 2-1-m1E01-1-0355 | C35 H41 N5 O8 S | 691.26758 | 0.0 | 0.0 |
| 2-1-m1E01-1-0356 | C39 H41 N5 O5 S | 691.28284 | -8.8 | -6.1 |
| 2-1-m1E01-1-0357 | C34 H30 F2 N4 O6 S2 | 692.15748 | -10.5 | 2.5 |
| 2-1-m1E01-1-0358 | C38 H36 N4 O7 S | 692.23047 | -7.7 | 2.1 |
| 2-1-m1E01-1-0359 | C42 H36 N4 O4 S | 692.24573 | -4.8 | -1.6 |
| 2-1-m1E01-1-0360 | C39 H40 N4 O6 S | 692.26686 | -16.4 | 13.2 |
| 2-1-m1E01-1-0361 | C39 H40 N4 O6 S | 692.26686 | -16.7 | 6.2 |
| 2-1-m1E01-1-0362 | C39 H40 N4 O6 S | 692.26686 | -23.8 | -2.5 |
| 2-1-m1E01-1-0363 | C39 H40 N4 O6 S | 692.26686 | 0.0 | 0.0 |
| 2-1-m1E01-1-0364 | C33 H29 F2 N5 O6 S2 | 693.15273 | -0.3 | 1.4 |
| 2-1-m1E01-1-0365 | C33 H32 F N5 O7 S2 | 693.17272 | -8.6 | 3.4 |
| 2-1-m1E01-1-0366 | C37 H35 N5 O7 S | 693.22572 | -2.8 | 1.4 |
| 2-1-m1E01-1-0367 | C37 H35 N5 O7 S | 693.22572 | 0.0 | 5.8 |
| 2-1-m1E01-1-0368 | C37 H33 F2 N7 O5 | 693.25112 | -7.6 | 2.3 |
| 2-1-m1E01-1-0369 | C38 H39 N5 O6 S | 693.26210 | -6.9 | 0.5 |
| 2-1-m1E01-1-0370 | C40 H47 N5 O6 | 693.35263 | -6.1 | 0.3 |
| 2-1-m1E01-1-0371 | C32 H31 F N6 O7 S2 | 694.16797 | -3.9 | 1.8 |
| 2-1-m1E01-1-0372 | C36 H34 N6 O7 S | 694.22097 | -1.3 | 1.3 |
| 2-1-m1E01-1-0373 | C38 H38 N4 O7 S | 694.24612 | -8.0 | 3.3 |
| 2-1-m1E01-1-0374 | C38 H38 N4 O7 S | 694.24612 | -9.1 | 1.9 |
| 2-1-m1E01-1-0375 | C39 H39 F N4 O5 S | 694.26252 | 0.9 | -6.3 |
| 2-1-m1E01-1-0376 | C33 H31 F2 N5 O8 S | 695.18614 | -5.7 | 9.6 |
| 2-1-m1E01-1-0377 | C37 H37 N5 O7 S | 695.24137 | 0.0 | -3.0 |
| 2-1-m1E01-1-0378 | C37 H37 N5 O7 S | 695.24137 | 0.0 | 0.0 |
| 2-1-m1E01-1-0379 | C38 H41 N5 O6 S | 695.27775 | -7.8 | 10.4 |
| 2-1-m1E01-1-0380 | C38 H41 N5 O6 S | 695.27775 | -5.8 | 2.3 |
| 2-1-m1E01-1-0381 | C39 H45 N5 O7 | 695.33190 | 0.0 | 0.0 |
| 2-1-m1E01-1-0382 | C35 H32 N6 O6 S2 | 696.18247 | -5.4 | 3.7 |
| 2-1-m1E01-1-0383 | C33 H31 F3 N6 O6 S | 696.19779 | -3.3 | 7.0 |
| 2-1-m1E01-1-0384 | C37 H33 F N4 O7 S | 696.20540 | -4.3 | 0.6 |

(continued)

[Table 11-2-1]

| | | | AS-MS ratio (%) | |
|---|---|---|---|---|
| ID | Molecular Formula | Exact Mass | N1 | N2 |
| 2-1-m1E01-1-0385 | C37 H33 F N4 O7 S | 696.20540 | 4.2 | 4.2 |
| 2-1-m1E01-1-0386 | C37 H36 N4 O8 S | 696.22538 | 0.0 | 0.0 |
| 2-1-m1E01-1-0387 | C38 H37 F N4 O6 S | 696.24178 | -8.0 | -1.6 |
| 2-1-m1E01-1-0388 | C38 H37 F N4 O6 S | 696.24178 | 0.0 | 0.0 |
| 2-1-m1E01-1-0389 | C37 H40 N6 O6 S | 696.27300 | -3.7 | 0.8 |
| 2-1-m1E01-1-0390 | C43 H44 N4 O5 | 696.33117 | -16.7 | 0.9 |
| 2-1-m1E01-1-0391 | C36 H32 F N5 O7 S | 697.20065 | -0.6 | 3.3 |
| 2-1-m1E01-1-0392 | C36 H32 F N5 O7 S | 697.20065 | -8.8 | -2.0 |
| 2-1-m1E01-1-0393 | C37 H33 F2 N5 O5 S | 697.21705 | 0.0 | 0.0 |
| 2-1-m1E01-1-0394 | C36 H35 N5 O8 S | 697.22063 | -0.6 | 2.0 |
| 2-1-m1E01-1-0395 | C34 H37 F2 N5 O7 S | 697.23818 | -5.3 | 0.9 |
| 2-1-m1E01-1-0396 | C38 H34 F3 N5 O5 | 697.25120 | -4.2 | 3.4 |
| 2-1-m1E01-1-0397 | C37 H39 N5 O7 S | 697.25702 | -6.4 | 2.4 |
| 2-1-m1E01-1-0398 | C37 H39 N5 O7 S | 697.25702 | -5.7 | 5.4 |
| 2-1-m1E01-1-0399 | C37 H39 N5 O7 S | 697.25702 | -14.4 | 2.8 |
| 2-1-m1E01-1-0400 | C37 H39 N5 O7 S | 697.25702 | -3.7 | -0.9 |
| 2-1-m1E01-1-0401 | C36 H34 N4 O7 S2 | 698.18689 | -0.7 | 7.2 |
| 2-1-m1E01-1-0402 | C36 H34 N4 O9 S | 698.20465 | 0.0 | 1.7 |
| 2-1-m1E01-1-0403 | C35 H34 N6 O8 S | 698.21588 | 0.0 | 0.4 |
| 2-1-m1E01-1-0404 | C35 H34 N6 O8 S | 698.21588 | -3.0 | 0.4 |
| 2-1-m1E01-1-0405 | C38 H33 F3 N4 O6 | 698.23522 | -5.7 | 2.4 |
| 2-1-m1E01-1-0406 | C38 H36 F2 N4 O5 S | 698.23745 | 0.0 | 0.0 |
| 2-1-m1E01-1-0407 | C36 H38 N6 O7 S | 698.25227 | -4.2 | 0.3 |
| 2-1-m1E01-1-0408 | C36 H38 N6 O7 S | 698.25227 | -4.0 | -1.7 |
| 2-1-m1E01-1-0409 | C36 H38 N6 O7 S | 698.25227 | -4.8 | 0.8 |
| 2-1-m1E01-1-0410 | C34 H33 N7 O8 S | 699.21113 | -0.1 | 0.0 |
| 2-1-m1E01-1-0411 | C37 H32 F3 N5 O6 | 699.23047 | -1.5 | 3.9 |
| 2-1-m1E01-1-0412 | C36 H37 N5 O8 S | 699.23628 | -2.7 | 0.5 |
| 2-1-m1E01-1-0413 | C36 H37 N5 O8 S | 699.23628 | -0.5 | -0.1 |
| 2-1-m1E01-1-0414 | C35 H37 N7 O7 S | 699.24752 | 0.0 | 0.0 |
| 2-1-m1E01-1-0415 | C37 H38 F N5 O6 S | 699.25268 | -4.9 | -0.9 |
| 2-1-m1E01-1-0416 | C37 H34 F2 N4 O6 S | 700.21671 | 0.0 | 0.0 |
| 2-1-m1E01-1-0417 | C37 H34 F2 N4 O6 S | 700.21671 | 0.0 | 0.0 |
| 2-1-m1E01-1-0418 | C38 H32 F4 N4 O5 | 700.23088 | -1.5 | 0.6 |
| 2-1-m1E01-1-0419 | C35 H36 N6 O8 S | 700.23153 | -1.9 | 0.6 |
| 2-1-m1E01-1-0420 | C40 H36 N4 O6 S | 700.23556 | -2.6 | 0.6 |

(continued)

[Table 11-2-1]

| | | | AS-MS ratio (%) | |
|---|---|---|---|---|
| ID | Molecular Formula | Exact Mass | N1 | N2 |
| 2-1-m1E01-1-0421 | C40 H36 N4 O6 S | 700.23556 | -1.7 | -39.2 |
| 2-1-m1E01-1-0422 | C36 H33 F2 N5 O6 S | 701.21196 | 1.3 | 0.0 |
| 2-1-m1E01-1-0423 | C35 H35 N5 O9 S | 701.21555 | 0.0 | 0.0 |
| 2-1-m1E01-1-0424 | C34 H35 N7 O8 S | 701.22678 | -5.1 | 0.1 |
| 2-1-m1E01-1-0425 | C39 H35 N5 O6 S | 701.23080 | -4.2 | -1.0 |
| 2-1-m1E01-1-0426 | C36 H36 F N5 O7 S | 701.23195 | -5.1 | 1.1 |
| 2-1-m1E01-1-0427 | C36 H36 F N5 O7 S | 701.23195 | -1.5 | 0.0 |
| 2-1-m1E01-1-0428 | C37 H34 F3 N5 O6 | 701.24612 | -4.4 | 2.0 |
| 2-1-m1E01-1-0429 | C38 H38 F3 N5 O5 | 701.28250 | -4.0 | 7.3 |
| 2-1-m1E01-1-0430 | C38 H41 F2 N5 O6 | 701.30249 | -7.4 | 1.6 |
| 2-1-m1E01-1-0431 | C35 H31 F N4 O7 S2 | 702.16182 | -3.9 | 1.2 |
| 2-1-m1E01-1-0432 | C38 H34 N6 O6 S | 702.22605 | 5.5 | -1.9 |
| 2-1-m1E01-1-0433 | C38 H34 N6 O6 S | 702.22605 | 5.9 | -1.3 |
| 2-1-m1E01-1-0434 | C35 H35 F N6 O7 S | 702.22720 | -7.5 | -12.7 |
| 2-1-m1E01-1-0435 | C35 H35 F N6 O7 S | 702.22720 | 2.4 | -19.8 |
| 2-1-m1E01-1-0436 | C36 H33 F3 N6 O6 | 702.24137 | -5.5 | 1.6 |
| 2-1-m1E01-1-0437 | C34 H33 N5 O8 S2 | 703.17705 | -0.5 | 0.9 |
| 2-1-m1E01-1-0438 | C35 H37 N5 O7 S2 | 703.21344 | -12.4 | 6.5 |
| 2-1-m1E01-1-0439 | C37 H33 N7 O6 S | 703.22130 | 0.0 | 0.0 |
| 2-1-m1E01-1-0440 | C37 H36 F3 N5 O6 | 703.26177 | 0.0 | 0.0 |
| 2-1-m1E01-1-0441 | C34 H32 N4 O9 S2 | 704.16107 | 0.0 | 0.0 |
| 2-1-m1E01-1-0442 | C33 H32 N6 O8 S2 | 704.17230 | -0.7 | 0.6 |
| 2-1-m1E01-1-0443 | C35 H30 F2 N4 O8 S | 704.17524 | -2.8 | 2.6 |
| 2-1-m1E01-1-0444 | C36 H31 F3 N4 O6 S | 704.19164 | -6.5 | -0.6 |
| 2-1-m1E01-1-0445 | C34 H36 N6 O7 S2 | 704.20869 | 0.0 | 0.9 |
| 2-1-m1E01-1-0446 | C39 H36 N4 O7 S | 704.23047 | 0.0 | 0.0 |
| 2-1-m1E01-1-0447 | C41 H35 F3 N4 04 | 704.26104 | -16.2 | 15.8 |
| 2-1-m1E01-1-0448 | C41 H44 N4 O5 S | 704.30324 | -16.0 | 38.7 |
| 2-1-m1E01-1-0449 | C41 H44 N4 O5 S | 704.30324 | -14.8 | 6.3 |
| 2-1-m1E01-1-0450 | C33 H31 N5 O9 S2 | 705.15632 | -0.4 | 2.2 |
| 2-1-m1E01-1-0451 | C34 H32 F N5 O9 S | 705.19048 | 0.0 | 0.9 |
| 2-1-m1E01-1-0452 | C34 H35 N5 O8 S2 | 705.19270 | 0.0 | 0.9 |
| 2-1-m1E01-1-0453 | C35 H33 F2 N5 O7 S | 705.20688 | -3.7 | 0.2 |
| 2-1-m1E01-1-0454 | C35 H33 F2 N5 O7 S | 705.20688 | -4.6 | 0.4 |
| 2-1-m1E01-1-0455 | C38 H35 N5 O7 S | 705.22572 | -14.9 | 6.6 |
| 2-1-m1E01-1-0456 | C37 H35 N7 O6 S | 705.23695 | -1.8 | 0.5 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0457 | C33 H34 N6 O8 S2 | 706.18795 | 0.0 | 0.2 |
| 2-1-m1E01-1-0458 | C38 H34 N4 O6 S2 | 706.19198 | 0.0 | 0.0 |
| 2-1-m1E01-1-0459 | C38 H34 N4 O6 S2 | 706.19198 | -9.4 | 7.0 |
| 2-1-m1E01-1-0460 | C38 H34 N4 O6 S2 | 706.19198 | -5.7 | -1.6 |
| 2-1-m1E01-1-0461 | C34 H32 F2 N6 O7 S | 706.20212 | -1.8 | 0.5 |
| 2-1-m1E01-1-0462 | C39 H38 N4 O7 S | 706.24612 | -4.9 | 2.8 |
| 2-1-m1E01-1-0463 | C39 H33 F3 N6 O4 | 706.25154 | -17.3 | 1.4 |
| 2-1-m1E01-1-0464 | C40 H42 N4 O6 S | 706.28251 | -24.0 | -0.7 |
| 2-1-m1E01-1-0465 | C40 H42 N4 O6 S | 706.28251 | 0.0 | 0.0 |
| 2-1-m1E01-1-0466 | C37 H33 N5 O6 S2 | 707.18723 | -17.9 | 1.1 |
| 2-1-m1E01-1-0467 | C37 H33 N5 O6 S2 | 707.18723 | -12.2 | -0.6 |
| 2-1-m1E01-1-0468 | C37 H33 N5 O6 S2 | 707.18723 | 0.0 | 0.0 |
| 2-1-m1E01-1-0469 | C33 H31 F2 N7 O7 S | 707.19737 | 0.0 | 0.0 |
| 2-1-m1E01-1-0470 | C35 H32 F3 N5 O6 S | 707.20254 | -6.6 | 2.2 |
| 2-1-m1E01-1-0471 | C35 H32 F3 N5 O8 | 707.22030 | -4.7 | 1.8 |
| 2-1-m1E01-1-0472 | C38 H37 N5 O7 S | 707.24137 | -3.3 | 0.2 |
| 2-1-m1E01-1-0473 | C38 H37 N5 O7 S | 707.24137 | 0.0 | 0.0 |
| 2-1-m1E01-1-0474 | C35 H31 F3 N4 O7 S | 708.18655 | -1.4 | -0.4 |
| 2-1-m1E01-1-0475 | C37 H33 F N6 O6 S | 708.21663 | -4.2 | 2.9 |
| 2-1-m1E01-1-0476 | C38 H36 N4 O8 S | 708.22538 | -5.2 | 1.1 |
| 2-1-m1E01-1-0477 | C39 H40 N4 O7 S | 708.26177 | -8.8 | 4.9 |
| 2-1-m1E01-1-0478 | C39 H40 N4 O7 S | 708.26177 | 0.0 | -1.6 |
| 2-1-m1E01-1-0479 | C40 H41 F N4 O5 S | 708.27817 | 0.0 | 0.0 |
| 2-1-m1E01-1-0480 | C37 H35 N5 O8 S | 709.22063 | -1.1 | 2.9 |
| 2-1-m1E01-1-0481 | C36 H32 F5 N5 O5 | 709.23236 | -6.2 | 5.7 |
| 2-1-m1E01-1-0482 | C39 H43 N5 O6 S | 709.29340 | -10.5 | 8.7 |
| 2-1-m1E01-1-0483 | C39 H43 N5 O6 S | 709.29340 | -10.9 | 11.4 |
| 2-1-m1E01-1-0484 | C39 H43 N5 O6 S | 709.29340 | -4.8 | 1.3 |
| 2-1-m1E01-1-0485 | C39 H33 F3 N4 O4 S | 710.21746 | 0.0 | 0.0 |
| 2-1-m1E01-1-0486 | C38 H35 F N4 O7 S | 710.22105 | -10.1 | 0.5 |
| 2-1-m1E01-1-0487 | C36 H37 F3 N4 O6 S | 710.23859 | -13.7 | 2.9 |
| 2-1-m1E01-1-0488 | C39 H39 F N4 O6 S | 710.25743 | 0.0 | 14.1 |
| 2-1-m1E01-1-0489 | C39 H42 N4 O7 S | 710.27742 | -2.7 | 0.7 |
| 2-1-m1E01-1-0490 | C38 H42 N6 O6 S | 710.28865 | 0.0 | 0.0 |
| 2-1-m1E01-1-0491 | C33 H31 F2 N5 O7 S2 | 711.16330 | -7.6 | 8.9 |
| 2-1-m1E01-1-0492 | C37 H34 F N5 O7 S | 711.21630 | -2.5 | 0.8 |

(continued)

[Table 11-2-1]

| | | | AS-MS ratio (%) | |
|---|---|---|---|---|
| ID | Molecular Formula | Exact Mass | N1 | N2 |
| 2-1-m1E01-1-0493 | C37 H37 N5 O8 S | 711.23628 | 0.0 | 0.0 |
| 2-1-m1E01-1-0494 | C38 H41 N5 O7 S | 711.27267 | -1.5 | 0.2 |
| 2-1-m1E01-1-0495 | C38 H41 N5 O7 S | 711.27267 | -3.8 | -0.8 |
| 2-1-m1E01-1-0496 | C38 H41 N5 O7 S | 711.27267 | 0.0 | 5.5 |
| 2-1-m1E01-1-0497 | C38 H41 N5 O7 S | 711.27267 | 0.0 | 0.0 |
| 2-1-m1E01-1-0498 | C32 H30 F2 N6 O7 S2 | 712.15855 | 3.5 | -2.2 |
| 2-1-m1E01-1-0499 | C37 H36 N4 O7 S2 | 712.20254 | -11.2 | 1.0 |
| 2-1-m1E01-1-0500 | C36 H36 N6 O8 S | 712.23153 | -0.7 | 1.1 |
| 2-1-m1E01-1-0501 | C36 H36 N6 O8 S | 712.23153 | 0.5 | 1.0 |
| 2-1-m1E01-1-0502 | C38 H37 F N4 O7 S | 712.23670 | -0.6 | 0.7 |
| 2-1-m1E01-1-0503 | C38 H37 F N4 O7 S | 712.23670 | -1.2 | 1.1 |
| 2-1-m1E01-1-0504 | C39 H38 F2 N4 O5 S | 712.25310 | 12.2 | 6.6 |
| 2-1-m1E01-1-0505 | C37 H40 N6 O7 S | 712.26792 | -2.4 | 0.9 |
| 2-1-m1E01-1-0506 | C42 H40 N4 O5 S | 712.27194 | 0.0 | 0.0 |
| 2-1-m1E01-1-0507 | C35 H35 N7 O8 S | 713.22678 | -0.7 | 1.6 |
| 2-1-m1E01-1-0508 | C38 H34 F3 N5 O6 | 713.24612 | -4.6 | 5.0 |
| 2-1-m1E01-1-0509 | C37 H39 N5 O8 S | 713.25193 | -4.1 | 0.4 |
| 2-1-m1E01-1-0510 | C37 H39 N5 O8 S | 713.25193 | 0.0 | 0.0 |
| 2-1-m1E01-1-0511 | C38 H40 F N5 O6 S | 713.26833 | 0.0 | 0.0 |
| 2-1-m1E01-1-0512 | C36 H34 N4 O8 S2 | 714.18181 | -5.5 | 2.9 |
| 2-1-m1E01-1-0513 | C36 H34 N4 O8 S2 | 714.18181 | 0.0 | 0.0 |
| 2-1-m1E01-1-0514 | C37 H32 F2 N4 O7 S | 714.19598 | -2.8 | 1.2 |
| 2-1-m1E01-1-0515 | C37 H32 F2 N4 O7 S | 714.19598 | -4.1 | -0.8 |
| 2-1-m1E01-1-0516 | C38 H36 F2 N4 O6 S | 714.23236 | 0.4 | 0.6 |
| 2-1-m1E01-1-0517 | C38 H34 N8 O5 S | 714.23729 | 1.0 | 0.0 |
| 2-1-m1E01-1-0518 | C36 H38 N6 O8 S | 714.24718 | 0.0 | 0.0 |
| 2-1-m1E01-1-0519 | C36 H38 N6 O8 S | 714.24718 | -5.4 | 2.5 |
| 2-1-m1E01-1-0520 | C41 H38 N4 O6 S | 714.25121 | -4.6 | 4.5 |
| 2-1-m1E01-1-0521 | C40 H41 F3 N4 O5 | 714.30291 | -5.7 | -0.6 |
| 2-1-m1E01-1-0522 | C35 H33 N5 O8 S2 | 715.17705 | -2.4 | 5.1 |
| 2-1-m1E01-1-0523 | C36 H31 F2 N5 O7 S | 715.19123 | -7.6 | -3.4 |
| 2-1-m1E01-1-0524 | C36 H34 F N5 O8 S | 715.21121 | -0.8 | 2.2 |
| 2-1-m1E01-1-0525 | C36 H34 F N5 O8 S | 715.21121 | 0.0 | 1.3 |
| 2-1-m1E01-1-0526 | C36 H37 N5 O9 S | 715.23120 | 3.4 | 0.0 |
| 2-1-m1E01-1-0527 | C40 H37 N5 O6 S | 715.24645 | -8.2 | 5.8 |
| 2-1-m1E01-1-0528 | C37 H38 F N5 O7 S | 715.24760 | -6.9 | -16.4 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0529 | C37 H38 F N5 O7 S | 715.24760 | -8.8 | 5.8 |
| 2-1-m1E01-1-0530 | C34 H32 N6 O8 S2 | 716.17230 | -6.9 | 3.0 |
| 2-1-m1E01-1-0531 | C35 H33 F N6 O8 S | 716.20646 | 0.1 | 1.4 |
| 2-1-m1E01-1-0532 | C35 H33 F N6 O8 S | 716.20646 | 0.0 | 0.0 |
| 2-1-m1E01-1-0533 | C38 H32 F4 N4 O6 | 716.22580 | -4.7 | 12.8 |
| 2-1-m1E01-1-0534 | C39 H36 N6 O6 S | 716.24170 | 0.0 | 0.0 |
| 2-1-m1E01-1-0535 | C37 H35 F3 N6 O6 | 716.25702 | -2.2 | 1.5 |
| 2-1-m1E01-1-0536 | C35 H35 N5 O8 S2 | 717.19270 | -0.5 | 2.0 |
| 2-1-m1E01-1-0537 | C35 H35 N5 O10 S | 717.21046 | 0.0 | 0.0 |
| 2-1-m1E01-1-0538 | C37 H34 F3 N5 O7 | 717.24103 | -5.7 | 1.9 |
| 2-1-m1E01-1-0539 | C37 H37 F2 N5 O6 S | 717.24326 | -5.6 | 1.8 |
| 2-1-m1E01-1-0540 | C35 H34 N4 O9 S2 | 718.17672 | -9.4 | 3.8 |
| 2-1-m1E01-1-0541 | C38 H34 N6 O5 S2 | 718.20321 | 0.0 | 0.0 |
| 2-1-m1E01-1-0542 | C37 H33 F3 N4 O6 S | 718.20729 | 0.0 | 0.0 |
| 2-1-m1E01-1-0543 | C37 H33 F3 N4 O6 S | 718.20729 | -26.1 | 9.0 |
| 2-1-m1E01-1-0544 | C37 H33 F3 N4 O6 S | 718.20729 | -2.4 | 7.5 |
| 2-1-m1E01-1-0545 | C37 H33 F3 N4 O6 S | 718.20729 | -2.8 | 5.5 |
| 2-1-m1E01-1-0546 | C39 H34 N4 O8 S | 718.20973 | 0.0 | 0.0 |
| 2-1-m1E01-1-0547 | C35 H38 N6 O7 S2 | 718.22434 | -6.5 | 2.3 |
| 2-1-m1E01-1-0548 | C36 H33 F3 N6 O7 | 718.23628 | -4.0 | 1.9 |
| 2-1-m1E01-1-0549 | C40 H38 N4 O7 S | 718.24612 | -5.1 | 1.9 |
| 2-1-m1E01-1-0550 | C41 H42 N4 O6 S | 718.28251 | 0.0 | 0.0 |
| 2-1-m1E01-1-0551 | C42 H46 N4 O5 S | 718.31889 | 0.0 | 0.0 |
| 2-1-m1E01-1-0552 | C36 H32 F3 N5 O6 S | 719.20254 | -2.1 | 1.1 |
| 2-1-m1E01-1-0553 | C36 H32 F3 N5 O6 S | 719.20254 | -4.7 | 1.0 |
| 2-1-m1E01-1-0554 | C36 H35 F2 N5 O7 S | 719.22253 | 0.0 | 2.7 |
| 2-1-m1E01-1-0555 | C36 H35 F2 N5 O7 S | 719.22253 | 0.0 | 0.0 |
| 2-1-m1E01-1-0556 | C37 H33 F4 N5 O6 | 719.23670 | 6.4 | 1.2 |
| 2-1-m1E01-1-0557 | C40 H41 N5 O6 S | 719.27775 | -2.6 | -3.3 |
| 2-1-m1E01-1-0558 | C34 H32 N4 O8 S3 | 720.13823 | 0.0 | 0.0 |
| 2-1-m1E01-1-0559 | C35 H30 F2 N4 O7 S2 | 720.15240 | -3.1 | 2.0 |
| 2-1-m1E01-1-0560 | C35 H31 F3 N6 O6 S | 720.19779 | -12.0 | -0.2 |
| 2-1-m1E01-1-0561 | C39 H36 N4 O6 S2 | 720.20763 | 0.5 | 0.9 |
| 2-1-m1E01-1-0562 | C39 H36 N4 O6 S2 | 720.20763 | -3.0 | 1.2 |
| 2-1-m1E01-1-0563 | C38 H33 F N6 O6 S | 720.21663 | -5.6 | 3.5 |
| 2-1-m1E01-1-0564 | C35 H34 F2 N6 O7 S | 720.21777 | 0.0 | 0.0 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0565 | C41 H44 N4 O6 S | 720.29816 | -32.1 | 28.0 |
| 2-1-m1E01-1-0566 | C33 H31 N5 O8 S3 | 721.13348 | -4.1 | 1.5 |
| 2-1-m1E01-1-0567 | C34 H32 F N5 O8 S2 | 721.16763 | -1.5 | 1.5 |
| 2-1-m1E01-1-0568 | C38 H35 N5 O6 S2 | 721.20288 | -5.6 | 2.4 |
| 2-1-m1E01-1-0569 | C37 H35 N7 O7 S | 721.23187 | -1.9 | 0.8 |
| 2-1-m1E01-1-0570 | C39 H39 N5 O7 S | 721.25702 | -2.9 | 4.8 |
| 2-1-m1E01-1-0571 | C34 H31 F N4 O9 S2 | 722.15165 | -6.5 | 1.0 |
| 2-1-m1E01-1-0572 | C37 H34 N6 O6 S2 | 722.19812 | -9.5 | 2.6 |
| 2-1-m1E01-1-0573 | C39 H35 F N4 O7 S | 722.22105 | -28.2 | 9.3 |
| 2-1-m1E01-1-0574 | C36 H34 N8 O7 S | 722.22712 | 4.4 | 0.0 |
| 2-1-m1E01-1-0575 | C38 H32 F6 N4 O4 | 722.23277 | 14.9 | 0.0 |
| 2-1-m1E01-1-0576 | C39 H33 F3 N6 O5 | 722.24645 | -4.3 | 3.7 |
| 2-1-m1E01-1-0577 | C40 H42 N4 O7 S | 722.27742 | 0.0 | 0.0 |
| 2-1-m1E01-1-0578 | C39 H42 N6 O6 S | 722.28865 | -7.1 | -0.4 |
| 2-1-m1E01-1-0579 | C33 H33 N5 O10 S2 | 723.16688 | 0.0 | 0.0 |
| 2-1-m1E01-1-0580 | C34 H31 F2 N5 O9 S | 723.18105 | -3.0 | 1.3 |
| 2-1-m1E01-1-0581 | C35 H32 F3 N5 O7 S | 723.19745 | -6.0 | 1.1 |
| 2-1-m1E01-1-0582 | C38 H37 N5 O8 S | 723.23628 | -2.5 | 2.2 |
| 2-1-m1E01-1-0583 | C38 H37 N5 O8 S | 723.23628 | -9.9 | 3.1 |
| 2-1-m1E01-1-0584 | C40 H45 N5 O6 S | 723.30905 | 10.6 | -7.5 |
| 2-1-m1E01-1-0585 | C40 H45 N5 O6 S | 723.30905 | -4.3 | -0.3 |
| 2-1-m1E01-1-0586 | C32 H32 N6 O10 S2 | 724.16213 | -0.8 | 1.4 |
| 2-1-m1E01-1-0587 | C35 H31 F3 N4 O6 S2 | 724.16371 | -1.0 | 0.8 |
| 2-1-m1E01-1-0588 | C35 H31 F3 N4 O8 S | 724.18147 | 0.0 | -2.0 |
| 2-1-m1E01-1-0589 | C38 H33 F N4 O6 S2 | 724.18255 | 0.0 | -2.0 |
| 2-1-m1E01-1-0590 | C38 H33 F N4 O6 S2 | 724.18255 | -9.2 | 0.4 |
| 2-1-m1E01-1-0591 | C37 H36 N6 O8 S | 724.23153 | -7.5 | 2.1 |
| 2-1-m1E01-1-0592 | C39 H37 F N4 O7 S | 724.23670 | -11.6 | -3.2 |
| 2-1-m1E01-1-0593 | C39 H40 N4 O8 S | 724.25668 | 1.6 | 0.0 |
| 2-1-m1E01-1-0594 | C40 H44 N4 O7 S | 724.29307 | -9.5 | 0.8 |
| 2-1-m1E01-1-0595 | C34 H30 F3 N5 O6 S2 | 725.15896 | -31.0 | 11.6 |
| 2-1-m1E01-1-0596 | C37 H35 N5 O7 S2 | 725.19779 | 5.7 | 6.4 |
| 2-1-m1E01-1-0597 | C37 H35 N5 O7 S2 | 725.19779 | -16.2 | -0.7 |
| 2-1-m1E01-1-0598 | C38 H39 N5 O8 S | 725.25193 | -0.3 | 0.6 |
| 2-1-m1E01-1-0599 | C38 H34 F3 N7 O5 | 725.25735 | 0.0 | 3.5 |
| 2-1-m1E01-1-0600 | C39 H43 N5 O7 S | 725.28832 | 0.0 | 0.0 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0601 | C39 H43 N5 O7 S | 725.28832 | -2.9 | 0.3 |
| 2-1-m1E01-1-0602 | C36 H34 N6 O7 S2 | 726.19304 | -3.0 | 4.8 |
| 2-1-m1E01-1-0603 | C36 H34 N6 O7 S2 | 726.19304 | -7.0 | 1.6 |
| 2-1-m1E01-1-0604 | C36 H34 N6 O7 S2 | 726.19304 | -9.1 | 0.8 |
| 2-1-m1E01-1-0605 | C36 H34 N6 O7 S2 | 726.19304 | -4.5 | 1.9 |
| 2-1-m1E01-1-0606 | C39 H33 F3 N4 O5 S | 726.21238 | 0.0 | 10.0 |
| 2-1-m1E01-1-0607 | C38 H35 F N4 O8 S | 726.21596 | -3.7 | 1.8 |
| 2-1-m1E01-1-0608 | C36 H37 F3 N4 O7 S | 726.23350 | -7.0 | 0.9 |
| 2-1-m1E01-1-0609 | C37 H38 N6 O8 S | 726.24718 | 0.1 | 2.3 |
| 2-1-m1E01-1-0610 | C37 H38 N6 O8 S | 726.24718 | -9.1 | -4.3 |
| 2-1-m1E01-1-0611 | C40 H40 F2 N4 O5 S | 726.26875 | -7.9 | 0.2 |
| 2-1-m1E01-1-0612 | C43 H42 N4 O5 S | 726.28759 | -60.1 | 29.6 |
| 2-1-m1E01-1-0613 | C35 H33 N7 O7 S2 | 727.18829 | -5.9 | 1.8 |
| 2-1-m1E01-1-0614 | C34 H32 F3 N5 O8 S | 727.19237 | -3.8 | 0.5 |
| 2-1-m1E01-1-0615 | C37 H37 N5 O9 S | 727.23120 | 0.0 | 0.0 |
| 2-1-m1E01-1-0616 | C38 H41 N5 O8 S | 727.26758 | 0.0 | -1.5 |
| 2-1-m1E01-1-0617 | C38 H41 N5 O8 S | 727.26758 | 0.0 | 0.0 |
| 2-1-m1E01-1-0618 | C39 H42 F N5 O6 S | 727.28398 | 0.0 | -7.8 |
| 2-1-m1E01-1-0619 | C34 H32 N8 O7 S2 | 728.18354 | 1.2 | 2.9 |
| 2-1-m1E01-1-0620 | C37 H36 N4 O8 S2 | 728.19746 | 0.0 | 1.9 |
| 2-1-m1E01-1-0621 | C36 H36 N6 O9 S | 728.22645 | 0.0 | -2.4 |
| 2-1-m1E01-1-0622 | C41 H36 N4 O7 S | 728.23047 | -7.4 | 12.1 |
| 2-1-m1E01-1-0623 | C42 H40 N4 O6 S | 728.26686 | -3.1 | 0.0 |
| 2-1-m1E01-1-0624 | C39 H41 F N4 O7 S | 728.26800 | 0.0 | 0.0 |
| 2-1-m1E01-1-0625 | C37 H33 F2 N5 O7 S | 729.20688 | -2.6 | 1.1 |
| 2-1-m1E01-1-0626 | C38 H34 F3 N5 O5 S | 729.22327 | 0.0 | 0.0 |
| 2-1-m1E01-1-0627 | C40 H35 N5 O7 S | 729.22572 | -6.2 | 1.1 |
| 2-1-m1E01-1-0628 | C37 H36 F N5 O8 S | 729.22686 | -0.7 | 0.6 |
| 2-1-m1E01-1-0629 | C35 H38 F3 N5 O7 S | 729.24440 | -8.4 | 6.2 |
| 2-1-m1E01-1-0630 | C38 H40 F N5 O7 S | 729.26325 | -4.0 | 3.2 |
| 2-1-m1E01-1-0631 | C38 H43 N5 O8 S | 729.28323 | 0.0 | 1.3 |
| 2-1-m1E01-1-0632 | C37 H33 F3 N6 O5 S | 730.21852 | -9.7 | 4.8 |
| 2-1-m1E01-1-0633 | C36 H35 F N6 O8 S | 730.22211 | 0.0 | 0.8 |
| 2-1-m1E01-1-0634 | C38 H36 F2 N4 O7 S | 730.22728 | -1.3 | 0.8 |
| 2-1-m1E01-1-0635 | C39 H37 F3 N4 O5 S | 730.24368 | -4.7 | 3.7 |
| 2-1-m1E01-1-0636 | C40 H38 N6 O6 S | 730.25735 | -10.8 | 0.8 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0637 | C37 H42 N6 O8 S | 730.27848 | -1.0 | 1.2 |
| 2-1-m1E01-1-0638 | C40 H41 F3 N4 O6 | 730.29782 | -5.6 | 2.4 |
| 2-1-m1E01-1-0639 | C36 H37 N5 O8 S2 | 731.20835 | 7.8 | 0.0 |
| 2-1-m1E01-1-0640 | C37 H38 F N5 O8 S | 731.24251 | -9.3 | 10.7 |
| 2-1-m1E01-1-0641 | C37 H38 F N5 O8 S | 731.24251 | 0.0 | 0.0 |
| 2-1-m1E01-1-0642 | C38 H39 F2 N5 O6 S | 731.25891 | -6.4 | 5.9 |
| 2-1-m1E01-1-0643 | C34 H32 N6 O7 S3 | 732.14946 | -9.2 | -0.5 |
| 2-1-m1E01-1-0644 | C36 H33 F N4 O8 S2 | 732.17238 | 0.0 | 10.3 |
| 2-1-m1E01-1-0645 | C37 H31 F3 N4 O7 S | 732.18655 | -4.1 | 2.8 |
| 2-1-m1E01-1-0646 | C36 H36 N4 O9 S2 | 732.19237 | -204.8 | 10.8 |
| 2-1-m1E01-1-0647 | C38 H35 F3 N4 O6 S | 732.22294 | -8.1 | 8.2 |
| 2-1-m1E01-1-0648 | C38 H35 F3 N4 O6 S | 732.22294 | 0.0 | 0.0 |
| 2-1-m1E01-1-0649 | C39 H36 N6 O7 S | 732.23662 | 0.0 | 0.0 |
| 2-1-m1E01-1-0650 | C41 H37 F N4 O6 S | 732.24178 | -3.7 | 1.3 |
| 2-1-m1E01-1-0651 | C37 H35 F3 N6 O7 | 732.25193 | -3.5 | 0.9 |
| 2-1-m1E01-1-0652 | C41 H40 N4 O7 S | 732.26177 | -9.2 | 4.7 |
| 2-1-m1E01-1-0653 | C42 H44 N4 O6 S | 732.29816 | -21.9 | -12.5 |
| 2-1-m1E01-1-0654 | C33 H31 N7 O7 S3 | 733.14471 | 0.0 | 0.0 |
| 2-1-m1E01-1-0655 | C35 H35 N5 O9 S2 | 733.18762 | 0.0 | -3.1 |
| 2-1-m1E01-1-0656 | C35 H35 N5 O9 S2 | 733.18762 | 0.0 | 0.0 |
| 2-1-m1E01-1-0657 | C36 H33 F2 N5 O8 S | 733.20179 | -1.2 | 2.9 |
| 2-1-m1E01-1-0658 | C36 H33 F2 N5 O8 S | 733.20179 | 0.0 | 0.0 |
| 2-1-m1E01-1-0659 | C37 H34 F3 N5 O6 S | 733.21819 | -3.9 | -2.9 |
| 2-1-m1E01-1-0660 | C37 H37 F2 N5 O7 S | 733.23818 | 0.0 | 0.0 |
| 2-1-m1E01-1-0661 | C39 H42 F3 N5 O6 | 733.30872 | -4.2 | 2.2 |
| 2-1-m1E01-1-0662 | C35 H34 N4 O8 S3 | 734.15388 | -20.9 | 10.9 |
| 2-1-m1E01-1-0663 | C35 H34 N4 O10 S2 | 734.17163 | 0.0 | 0.0 |
| 2-1-m1E01-1-0664 | C34 H34 N6 O9 S2 | 734.18287 | -2.6 | 0.7 |
| 2-1-m1E01-1-0665 | C39 H34 N4 O7 S2 | 734.18689 | -4.6 | 2.6 |
| 2-1-m1E01-1-0666 | C35 H32 F2 N6 O8 S | 734.19704 | 0.0 | 0.0 |
| 2-1-m1E01-1-0667 | C37 H33 F3 N4 O7 S | 734.20220 | -0.4 | 7.7 |
| 2-1-m1E01-1-0668 | C37 H33 F3 N4 O7 S | 734.20220 | 0.0 | 0.0 |
| 2-1-m1E01-1-0669 | C40 H38 N4 O6 S2 | 734.22328 | 0.0 | -9.1 |
| 2-1-m1E01-1-0670 | C40 H38 N4 O6 S2 | 734.22328 | 2.4 | 0.0 |
| 2-1-m1E01-1-0671 | C40 H38 N4 O8 S | 734.24103 | -6.6 | 30.2 |
| 2-1-m1E01-1-0672 | C36 H32 F3 N5 O7 S | 735.19745 | -4.4 | 2.8 |

(continued)

[Table 11-2-1]

| | | | AS-MS ratio (%) | |
|---|---|---|---|---|
| ID | Molecular Formula | Exact Mass | N1 | N2 |
| 2-1-m1E01-1-0673 | C39 H37 N5 O6 S2 | 735.21853 | 2.4 | 1.1 |
| 2-1-m1E01-1-0674 | C37 H33 F4 N5 O7 | 735.23161 | -5.1 | 1.9 |
| 2-1-m1E01-1-0675 | C38 H37 N7 O7 S | 735.24752 | -3.8 | 0.7 |
| 2-1-m1E01-1-0676 | C36 H31 F3 N4 O8 S | 736.18147 | -3.1 | -0.6 |
| 2-1-m1E01-1-0677 | C35 H31 F3 N6 O7 S | 736.19270 | 2.5 | -0.5 |
| 2-1-m1E01-1-0678 | C37 H32 F4 N4 O6 S | 736.19787 | -5.9 | 4.3 |
| 2-1-m1E01-1-0679 | C39 H36 N4 O7 S2 | 736.20254 | -5.4 | 2.3 |
| 2-1-m1E01-1-0680 | C39 H36 N4 O7 S2 | 736.20254 | -4.9 | 0.8 |
| 2-1-m1E01-1-0681 | C41 H41 F N4 O6 S | 736.27308 | -8.8 | -0.7 |
| 2-1-m1E01-1-0682 | C34 H35 N5 O10 S2 | 737.18253 | -2.7 | 1.1 |
| 2-1-m1E01-1-0683 | C36 H34 F3 N5 O7 S | 737.21310 | -15.0 | 9.0 |
| 2-1-m1E01-1-0684 | C36 H34 F3 N5 O7 S | 737.21310 | -7.2 | 1.7 |
| 2-1-m1E01-1-0685 | C36 H34 F3 N5 O7 S | 737.21310 | 0.0 | 0.0 |
| 2-1-m1E01-1-0686 | C36 H34 F3 N5 O7 S | 737.21310 | -3.3 | 0.0 |
| 2-1-m1E01-1-0687 | C39 H39 N5 O8 S | 737.25193 | -10.3 | 4.5 |
| 2-1-m1E01-1-0688 | C40 H43 N5 O7 S | 737.28832 | -10.6 | 4.8 |
| 2-1-m1E01-1-0689 | C41 H47 N5 O6 S | 737.32470 | 0.0 | 10.7 |
| 2-1-m1E01-1-0690 | C34 H31 F N4 O8 S3 | 738.12880 | 0.0 | 0.0 |
| 2-1-m1E01-1-0691 | C38 H32 F2 N6 O6 S | 738.20721 | 0.0 | 0.0 |
| 2-1-m1E01-1-0692 | C35 H33 F3 N6 O7 S | 738.20835 | -4.0 | 1.5 |
| 2-1-m1E01-1-0693 | C35 H33 F3 N6 O7 S | 738.20835 | 0.0 | 0.0 |
| 2-1-m1E01-1-0694 | C38 H32 F6 N4 O5 | 738.22769 | -13.9 | 1.9 |
| 2-1-m1E01-1-0695 | C38 H38 N6 O6 S2 | 738.22942 | -9.9 | -14.3 |
| 2-1-m1E01-1-0696 | C39 H42 N6 O7 S | 738.28357 | -9.9 | 0.0 |
| 2-1-m1E01-1-0697 | C41 H46 N4 O7 S | 738.30872 | -0.7 | -0.7 |
| 2-1-m1E01-1-0698 | C33 H33 N5 O9 S3 | 739.14404 | 0.0 | 0.0 |
| 2-1-m1E01-1-0699 | C34 H31 F2 N5 O8 S2 | 739.15821 | 0.0 | 0.0 |
| 2-1-m1E01-1-0700 | C38 H34 F N5 O6 S2 | 739.19345 | -8.2 | 8.9 |
| 2-1-m1E01-1-0701 | C34 H32 F3 N7 O7 S | 739.20360 | 0.0 | -2.3 |
| 2-1-m1E01-1-0702 | C38 H37 N5 O7 S2 | 739.21344 | -3.6 | 2.1 |
| 2-1-m1E01-1-0703 | C38 H37 N5 O7 S2 | 739.21344 | 0.0 | 0.0 |
| 2-1-m1E01-1-0704 | C37 H34 F N7 O7 S | 739.22245 | -2.4 | 1.5 |
| 2-1-m1E01-1-0705 | C40 H45 N5 O7 S | 739.30397 | 0.0 | 0.0 |
| 2-1-m1E01-1-0706 | C32 H32 N6 O9 S3 | 740.13929 | -0.1 | 0.9 |
| 2-1-m1E01-1-0707 | C34 H30 F2 N4 O9 S2 | 740.14223 | -0.1 | 1.3 |
| 2-1-m1E01-1-0708 | C35 H31 F3 N4 O7 S2 | 740.15863 | -3.9 | 1.6 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0709 | C37 H36 N6 O7 S2 | 740.20869 | -4.9 | 0.9 |
| 2-1-m1E01-1-0710 | C37 H36 N6 O7 S2 | 740.20869 | -5.1 | 1.7 |
| 2-1-m1E01-1-0711 | C39 H34 F2 N4 O7 S | 740.21163 | -11.9 | -0.3 |
| 2-1-m1E01-1-0712 | C38 H40 N6 O8 S | 740.26283 | 0.0 | 0.0 |
| 2-1-m1E01-1-0713 | C40 H44 N4 O8 S | 740.28799 | -4.4 | 2.4 |
| 2-1-m1E01-1-0714 | C44 H44 N4 O5 S | 740.30324 | 0.0 | 0.0 |
| 2-1-m1E01-1-0715 | C34 H30 F3 N5 O7 S2 | 741.15387 | -1.7 | 18.8 |
| 2-1-m1E01-1-0716 | C33 H32 F N5 O10 S2 | 741.15746 | 0.0 | 0.0 |
| 2-1-m1E01-1-0717 | C36 H35 N7 O7 S2 | 741.20394 | -4.5 | 0.8 |
| 2-1-m1E01-1-0718 | C36 H35 N7 O7 S2 | 741.20394 | 0.0 | 0.0 |
| 2-1-m1E01-1-0719 | C38 H36 F N5 O8 S | 741.22686 | -9.0 | 1.3 |
| 2-1-m1E01-1-0720 | C37 H33 F6 N5 O5 | 741.23859 | -2.0 | 9.0 |
| 2-1-m1E01-1-0721 | C38 H34 F3 N7 O6 | 741.25227 | -11.0 | 4.7 |
| 2-1-m1E01-1-0722 | C39 H43 N5 O8 S | 741.28323 | 0.0 | 0.0 |
| 2-1-m1E01-1-0723 | C38 H32 F2 N4 O6 S2 | 742.17313 | -16.0 | 3.9 |
| 2-1-m1E01-1-0724 | C38 H32 F2 N4 O6 S2 | 742.17313 | 0.0 | 1.4 |
| 2-1-m1E01-1-0725 | C38 H38 N4 O8 S2 | 742.21311 | -3.7 | 3.0 |
| 2-1-m1E01-1-0726 | C37 H38 N6 O9 S | 742.24210 | 0.0 | 0.0 |
| 2-1-m1E01-1-0727 | C34 H32 F3 N5 O7 S2 | 743.16952 | -3.5 | 3.1 |
| 2-1-m1E01-1-0728 | C34 H32 F3 N5 O9 S | 743.18728 | -23.8 | 5.2 |
| 2-1-m1E01-1-0729 | C37 H34 F N5 O7 S2 | 743.18837 | -17.2 | 5.1 |
| 2-1-m1E01-1-0730 | C37 H34 F N5 O7 S2 | 743.18837 | -13.6 | 5.5 |
| 2-1-m1E01-1-0731 | C41 H37 N5 O7 S | 743.24137 | -3.3 | 3.4 |
| 2-1-m1E01-1-0732 | C38 H38 F N5 O8 S | 743.24251 | 0.0 | 0.0 |
| 2-1-m1E01-1-0733 | C38 H41 N5 O9 S | 743.26250 | 0.0 | 5.8 |
| 2-1-m1E01-1-0734 | C39 H45 N5 O8 S | 743.29888 | 2.3 | 0.0 |
| 2-1-m1E01-1-0735 | C33 H31 F3 N6 O7 S2 | 744.16477 | -2.0 | 0.6 |
| 2-1-m1E01-1-0736 | C35 H32 N6 O9 S2 | 744.16722 | -2.0 | 0.1 |
| 2-1-m1E01-1-0737 | C37 H36 N4 O9 S2 | 744.19237 | -7.9 | 2.0 |
| 2-1-m1E01-1-0738 | C40 H39 F3 N4 O5 S | 744.25933 | -32.8 | 0.0 |
| 2-1-m1E01-1-0739 | C42 H40 N4 O7 S | 744.26177 | 0.0 | 0.0 |
| 2-1-m1E01-1-0740 | C38 H34 F3 N5 O6 S | 745.21819 | -5.7 | 6.2 |
| 2-1-m1E01-1-0741 | C37 H36 F N5 O9 S | 745.22178 | 0.0 | 0.0 |
| 2-1-m1E01-1-0742 | C35 H38 F3 N5 O8 S | 745.23932 | -5.6 | 2.1 |
| 2-1-m1E01-1-0743 | C39 H41 F2 N5 O6 S | 745.27456 | 0.0 | 0.0 |
| 2-1-m1E01-1-0744 | C38 H33 F3 N4 O7 S | 746.20220 | -3.3 | 1.0 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0745 | C41 H35 F N4 O7 S | 746.22105 | -8.5 | 4.6 |
| 2-1-m1E01-1-0746 | C39 H37 F3 N4 O6 S | 746.23859 | -28.2 | 13.7 |
| 2-1-m1E01-1-0747 | C39 H37 F3 N4 O6 S | 746.23859 | 0.0 | 0.0 |
| 2-1-m1E01-1-0748 | C39 H37 F3 N4 O6 S | 746.23859 | 0.0 | 0.0 |
| 2-1-m1E01-1-0749 | C39 H40 F2 N4 O7 S | 746.25858 | 3.6 | 0.0 |
| 2-1-m1E01-1-0750 | C43 H46 N4 O6 S | 746.31381 | -19.6 | 6.8 |
| 2-1-m1E01-1-0751 | C37 H32 F3 N5 O7 S | 747.19745 | -4.2 | -3.3 |
| 2-1-m1E01-1-0752 | C36 H37 N5 O9 S2 | 747.20327 | -0.2 | -0.9 |
| 2-1-m1E01-1-0753 | C38 H42 F N5 O8 S | 747.27381 | 0.1 | 0.0 |
| 2-1-m1E01-1-0754 | C36 H36 N4 O8 S3 | 748.16953 | -5.8 | 1.9 |
| 2-1-m1E01-1-0755 | C36 H34 F2 N6 O8 S | 748.21269 | 0.0 | 0.0 |
| 2-1-m1E01-1-0756 | C38 H35 F3 N4 O7 S | 748.21785 | -5.7 | 1.2 |
| 2-1-m1E01-1-0757 | C38 H35 F3 N4 O7 S | 748.21785 | 0.0 | 0.0 |
| 2-1-m1E01-1-0758 | C42 H44 N4 O7 S | 748.29307 | -14.5 | 1.9 |
| 2-1-m1E01-1-0759 | C37 H34 F3 N5 O7 S | 749.21310 | 0.0 | 0.0 |
| 2-1-m1E01-1-0760 | C37 H37 F2 N5 O8 S | 749.23309 | -17.7 | 6.8 |
| 2-1-m1E01-1-0761 | C40 H39 N5 O6 S2 | 749.23418 | -17.8 | 5.9 |
| 2-1-m1E01-1-0762 | C38 H38 F3 N5 O6 S | 749.24949 | 0.0 | 0.0 |
| 2-1-m1E01-1-0763 | C39 H39 N7 O7 S | 749.26317 | 0.0 | -3.1 |
| 2-1-m1E01-1-0764 | C39 H42 F3 N5 O7 | 749.30363 | -8.6 | 0.3 |
| 2-1-m1E01-1-0765 | C35 H34 N4 O9 S3 | 750.14879 | 0.0 | 0.0 |
| 2-1-m1E01-1-0766 | C36 H32 F2 N4 O8 S2 | 750.16296 | 0.0 | 0.0 |
| 2-1-m1E01-1-0767 | C38 H34 F4 N4 O6 S | 750.21352 | -41.6 | 21.2 |
| 2-1-m1E01-1-0768 | C35 H34 F N5 O9 S2 | 751.17820 | 0.0 | 2.0 |
| 2-1-m1E01-1-0769 | C36 H32 F3 N5 O8 S | 751.19237 | 0.0 | 0.0 |
| 2-1-m1E01-1-0770 | C35 H37 N5 O10 S2 | 751.19818 | -4.4 | -0.9 |
| 2-1-m1E01-1-0771 | C39 H37 N5 O7 S2 | 751.21344 | -2.5 | 2.4 |
| 2-1-m1E01-1-0772 | C37 H36 F3 N5 O7 S | 751.22875 | -2.0 | 3.1 |
| 2-1-m1E01-1-0773 | C37 H36 F3 N5 O7 S | 751.22875 | -1.3 | 2.0 |
| 2-1-m1E01-1-0774 | C38 H37 N7 O8 S | 751.24243 | 0.0 | 0.0 |
| 2-1-m1E01-1-0775 | C40 H41 N5 O8 S | 751.26758 | -0.1 | -2.4 |
| 2-1-m1E01-1-0776 | C41 H45 N5 O7 S | 751.30397 | -9.8 | -0.2 |
| 2-1-m1E01-1-0777 | C36 H31 F3 N4 O7 S2 | 752.15863 | -8.3 | -5.7 |
| 2-1-m1E01-1-0778 | C36 H31 F3 N4 O9 S | 752.17638 | 0.0 | 1.9 |
| 2-1-m1E01-1-0779 | C36 H35 F3 N6 O7 S | 752.22400 | 0.0 | 0.0 |
| 2-1-m1E01-1-0780 | C42 H36 N6 O6 S | 752.24170 | -4.8 | 8.7 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0781 | C39 H40 N6 O6 S2 | 752.24507 | -2.1 | 12.9 |
| 2-1-m1E01-1-0782 | C34 H35 N5 O9 S3 | 753.15969 | 0.0 | 0.5 |
| 2-1-m1E01-1-0783 | C34 H35 N5 O11 S2 | 753.17745 | 0.0 | 0.0 |
| 2-1-m1E01-1-0784 | C36 H34 F3 N5 O8 S | 753.20802 | -9.3 | 9.5 |
| 2-1-m1E01-1-0785 | C36 H34 F3 N5 O8 S | 753.20802 | -3.1 | 3.3 |
| 2-1-m1E01-1-0786 | C39 H39 N5 O7 S2 | 753.22909 | -8.1 | 5.4 |
| 2-1-m1E01-1-0787 | C39 H39 N5 O7 S2 | 753.22909 | 0.0 | 0.0 |
| 2-1-m1E01-1-0788 | C39 H39 N5 O9 S | 753.24685 | 0.0 | 0.0 |
| 2-1-m1E01-1-0789 | C38 H34 N4 O9 S2 | 754.17672 | 0.0 | 0.0 |
| 2-1-m1E01-1-0790 | C37 H34 N6 O8 S2 | 754.18795 | -1.4 | 3.2 |
| 2-1-m1E01-1-0791 | C37 H31 F5 N4 O6 S | 754.18845 | -1.4 | 3.2 |
| 2-1-m1E01-1-0792 | C35 H33 F3 N6 O8 S | 754.20327 | -2.6 | 0.4 |
| 2-1-m1E01-1-0793 | C38 H38 N6 O7 S2 | 754.22434 | -13.8 | 6.1 |
| 2-1-m1E01-1-0794 | C38 H38 N6 O7 S2 | 754.22434 | -17.4 | 13.2 |
| 2-1-m1E01-1-0795 | C43 H38 N4 O7 S | 754.24612 | -14.2 | 6.2 |
| 2-1-m1E01-1-0796 | C41 H40 F2 N4 O6 S | 754.26366 | -27.5 | -23.0 |
| 2-1-m1E01-1-0797 | C36 H33 N7 O8 S2 | 755.18320 | -4.1 | -6.5 |
| 2-1-m1E01-1-0798 | C35 H32 F3 N5 O9 S | 755.18728 | -2.5 | 0.9 |
| 2-1-m1E01-1-0799 | C34 H32 F3 N7 O8 S | 755.19852 | 0.0 | 0.0 |
| 2-1-m1E01-1-0800 | C36 H33 F4 N5 O7 S | 755.20368 | -3.7 | 5.6 |
| 2-1-m1E01-1-0801 | C38 H37 N5 O8 S2 | 755.20835 | -2.4 | 1.5 |
| 2-1-m1E01-1-0802 | C38 H37 N5 O8 S2 | 755.20835 | 0.0 | 0.0 |
| 2-1-m1E01-1-0803 | C40 H42 F N5 O7 S | 755.27890 | -1.3 | -2.2 |
| 2-1-m1E01-1-0804 | C34 H30 F2 N4 O8 S3 | 756.11938 | 0.0 | 0.0 |
| 2-1-m1E01-1-0805 | C42 H36 N4 O6 S2 | 756.20763 | -4.5 | 1.8 |
| 2-1-m1E01-1-0806 | C42 H36 N4 O6 S2 | 756.20763 | -2.4 | 1.3 |
| 2-1-m1E01-1-0807 | C33 H32 F N5 O9 S3 | 757.13462 | 0.0 | 0.0 |
| 2-1-m1E01-1-0808 | C38 H33 F2 N5 O6 S2 | 757.18403 | 13.9 | 0.0 |
| 2-1-m1E01-1-0809 | C37 H33 F2 N7 O7 S | 757.21302 | 0.0 | 0.0 |
| 2-1-m1E01-1-0810 | C37 H33 F6 N5 O6 | 757.23350 | 0.1 | 8.5 |
| 2-1-m1E01-1-0811 | C40 H47 N5 O8 S | 757.31453 | -1.3 | 1.8 |
| 2-1-m1E01-1-0812 | C37 H35 F N6 O7 S2 | 758.19927 | -12.3 | 0.7 |
| 2-1-m1E01-1-0813 | C37 H35 F N6 O7 S2 | 758.19927 | 0.0 | 6.1 |
| 2-1-m1E01-1-0814 | C41 H41 F3 N4 O5 S | 758.27498 | 0.0 | 0.0 |
| 2-1-m1E01-1-0815 | C33 H31 F2 N5 O10 S2 | 759.14804 | -2.5 | 3.7 |
| 2-1-m1E01-1-0816 | C34 H32 F3 N5 O8 S2 | 759.16444 | -7.0 | 8.5 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0817 | C38 H35 F2 N5 O8 S | 759.21744 | -5.1 | 4.5 |
| 2-1-m1E01-1-0818 | C39 H45 N5 O9 S | 759.29380 | 0.0 | 0.0 |
| 2-1-m1E01-1-0819 | C35 H32 N6 O8 S3 | 760.14437 | -0.8 | 1.7 |
| 2-1-m1E01-1-0820 | C33 H31 F3 N6 O8 S2 | 760.15969 | 0.0 | 0.0 |
| 2-1-m1E01-1-0821 | C40 H39 F3 N4 O6 S | 760.25424 | -33.8 | 16.8 |
| 2-1-m1E01-1-0822 | C43 H44 N4 O7 S | 760.29307 | -10.4 | -1.0 |
| 2-1-m1E01-1-0823 | C37 H33 F2 N5 O7 S2 | 761.17895 | -29.2 | 18.6 |
| 2-1-m1E01-1-0824 | C37 H33 F2 N5 O7 S2 | 761.17895 | 0.0 | 0.0 |
| 2-1-m1E01-1-0825 | C38 H34 F3 N5 O7 S | 761.21310 | -1.0 | 2.4 |
| 2-1-m1E01-1-0826 | C37 H39 N5 O9 S2 | 761.21892 | -1.7 | 0.1 |
| 2-1-m1E01-1-0827 | C38 H33 F3 N4 O8 S | 762.19712 | 2.8 | -5.0 |
| 2-1-m1E01-1-0828 | C39 H37 F3 N4 O7 S | 762.23350 | -11.0 | -10.7 |
| 2-1-m1E01-1-0829 | C39 H37 F3 N4 O7 S | 762.23350 | -7.4 | 6.1 |
| 2-1-m1E01-1-0830 | C37 H32 F3 N5 O8 S | 763.19237 | -5.1 | -0.9 |
| 2-1-m1E01-1-0831 | C36 H37 N5 O10 S2 | 763.19818 | -1.6 | 6.6 |
| 2-1-m1E01-1-0832 | C39 H40 F3 N5 O6 S | 763.26514 | -1.2 | 6.1 |
| 2-1-m1E01-1-0833 | C38 H32 F4 N4 O7 S | 764.19278 | -2.6 | 3.6 |
| 2-1-m1E01-1-0834 | C40 H36 N4 O8 S2 | 764.19746 | 4.8 | 4.7 |
| 2-1-m1E01-1-0835 | C37 H34 F3 N5 O8 S | 765.20802 | 2.4 | 4.4 |
| 2-1-m1E01-1-0836 | C38 H38 F3 N5 O7 S | 765.24440 | -0.2 | 3.1 |
| 2-1-m1E01-1-0837 | C38 H38 F3 N5 O7 S | 765.24440 | 0.0 | -3.6 |
| 2-1-m1E01-1-0838 | C38 H38 F3 N5 O7 S | 765.24440 | -6.9 | 0.0 |
| 2-1-m1E01-1-0839 | C38 H41 F2 N5 O8 S | 765.26439 | 0.0 | 0.0 |
| 2-1-m1E01-1-0840 | C42 H47 N5 O7 S | 765.31962 | -10.7 | 5.8 |
| 2-1-m1E01-1-0841 | C36 H33 F3 N6 O8 S | 766.20327 | -8.1 | -2.3 |
| 2-1-m1E01-1-0842 | C38 H34 F4 N4 O7 S | 766.20843 | 0.0 | 3.6 |
| 2-1-m1E01-1-0843 | C40 H42 N6 O6 S2 | 766.26072 | -5.1 | -0.3 |
| 2-1-m1E01-1-0844 | C35 H37 N5 O9 S3 | 767.17534 | -3.9 | 1.8 |
| 2-1-m1E01-1-0845 | C37 H36 F3 N5 O8 S | 767.22367 | -0.9 | 2.0 |
| 2-1-m1E01-1-0846 | C37 H36 F3 N5 O8 S | 767.22367 | 0.0 | 0.0 |
| 2-1-m1E01-1-0847 | C41 H45 N5 O8 S | 767.29888 | 0.0 | 8.3 |
| 2-1-m1E01-1-0848 | C36 H31 F3 N4 O8 S2 | 768.15354 | -8.7 | -1.2 |
| 2-1-m1E01-1-0849 | C39 H36 N4 O9 S2 | 768.19237 | -8.8 | 4.6 |
| 2-1-m1E01-1-0850 | C36 H35 F3 N6 O8 S | 768.21892 | -5.6 | 1.9 |
| 2-1-m1E01-1-0851 | C41 H35 F3 N4 O6 S | 768.22294 | -6.4 | 0.0 |
| 2-1-m1E01-1-0852 | C39 H40 N6 O7 S2 | 768.23999 | -8.1 | 3.5 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0853 | C45 H44 N4 O6 S | 768.29816 | -20.6 | 3.1 |
| 2-1-m1E01-1-0854 | C34 H35 N5 O10 S3 | 769.15460 | -3.2 | 8.3 |
| 2-1-m1E01-1-0855 | C35 H33 F2 N5 O9 S2 | 769.16878 | -4.8 | 0.7 |
| 2-1-m1E01-1-0856 | C38 H35 N5 O9 S2 | 769.18762 | -0.4 | 4.2 |
| 2-1-m1E01-1-0857 | C37 H35 N7 O8 S2 | 769.19885 | -0.9 | 1.4 |
| 2-1-m1E01-1-0858 | C37 H35 F4 N5 O7 S | 769.21933 | 6.5 | 0.0 |
| 2-1-m1E01-1-0859 | C38 H34 N4 O8 S3 | 770.15388 | -6.0 | 8.5 |
| 2-1-m1E01-1-0860 | C38 H34 N4 O8 S3 | 770.15388 | -2.3 | 18.1 |
| 2-1-m1E01-1-0861 | C39 H33 F3 N6 O6 S | 770.21344 | -16.6 | 9.4 |
| 2-1-m1E01-1-0862 | C38 H38 N6 O8 S2 | 770.21925 | 33.1 | 0.0 |
| 2-1-m1E01-1-0863 | C38 H38 N6 O8 S2 | 770.21925 | 3.2 | 0.0 |
| 2-1-m1E01-1-0864 | C37 H33 N5 O8 S3 | 771.14913 | -13.1 | 1.7 |
| 2-1-m1E01-1-0865 | C35 H32 F3 N5 O8 S2 | 771.16444 | -0.3 | 2.7 |
| 2-1-m1E01-1-0866 | C35 H32 F3 N5 O10 S | 771.18220 | -2.3 | 1.5 |
| 2-1-m1E01-1-0867 | C42 H37 N5 O6 S2 | 771.21853 | -30.3 | 13.3 |
| 2-1-m1E01-1-0868 | C35 H31 F3 N4 O9 S2 | 772.14845 | -13.3 | -1.7 |
| 2-1-m1E01-1-0869 | C37 H33 F N6 O8 S2 | 772.17853 | 0.0 | 1.4 |
| 2-1-m1E01-1-0870 | C40 H35 F3 N4 O7 S | 772.21785 | -5.4 | -2.5 |
| 2-1-m1E01-1-0871 | C36 H32 F5 N5 O7 S | 773.19426 | 0.0 | -6.0 |
| 2-1-m1E01-1-0872 | C40 H41 F2 N5 O7 S | 773.26948 | -20.2 | 15.4 |
| 2-1-m1E01-1-0873 | C39 H33 F3 N4 O6 S2 | 774.17936 | 0.0 | 0.0 |
| 2-1-m1E01-1-0874 | C39 H33 F3 N4 O6 S2 | 774.17936 | -23.2 | 18.4 |
| 2-1-m1E01-1-0875 | C41 H41 F3 N4 O6 S | 774.26989 | 0.0 | 0.0 |
| 2-1-m1E01-1-0876 | C33 H31 F2 N5 O9 S3 | 775.12520 | 0.0 | 0.0 |
| 2-1-m1E01-1-0877 | C37 H34 F2 N6 O7 S2 | 776.18985 | -8.2 | -2.3 |
| 2-1-m1E01-1-0878 | C38 H34 F3 N5 O8 S | 777.20802 | -8.5 | 3.9 |
| 2-1-m1E01-1-0879 | C40 H42 F3 N5 O6 S | 777.28079 | 0.0 | 0.0 |
| 2-1-m1E01-1-0880 | C38 H34 N8 O7 S2 | 778.19919 | 0.0 | 0.0 |
| 2-1-m1E01-1-0881 | C39 H37 F3 N4 O8 S | 778.22842 | 0.0 | 0.0 |
| 2-1-m1E01-1-0882 | C40 H41 F3 N4 O7 S | 778.26480 | -1.1 | 2.5 |
| 2-1-m1E01-1-0883 | C39 H40 F3 N5 O7 S | 779.26005 | -20.1 | 4.0 |
| 2-1-m1E01-1-0884 | C34 H32 N6 O10 S3 | 780.13420 | -2.1 | 1.7 |
| 2-1-m1E01-1-0885 | C38 H32 F4 N4 O8 S | 780.18770 | -0.9 | -0.5 |
| 2-1-m1E01-1-0886 | C39 H36 N6 O8 S2 | 780.20360 | -8.9 | 3.2 |
| 2-1-m1E01-1-0887 | C37 H35 F3 N6 O8 S | 780.21892 | 0.0 | 0.0 |
| 2-1-m1E01-1-0888 | C37 H34 F3 N5 O9 S | 781.20293 | -2.2 | 0.6 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0889 | C38 H38 F3 N5 O8 S | 781.23932 | 0.0 | 0.0 |
| 2-1-m1E01-1-0890 | C38 H38 F3 N5 O8 S | 781.23932 | 21.2 | 0.0 |
| 2-1-m1E01-1-0891 | C38 H34 N6 O7 S3 | 782.16511 | -13.9 | 1.3 |
| 2-1-m1E01-1-0892 | C38 H34 N6 O7 S3 | 782.16511 | 0.0 | 0.0 |
| 2-1-m1E01-1-0893 | C37 H33 F3 N4 O8 S2 | 782.16919 | 0.6 | 8.7 |
| 2-1-m1E01-1-0894 | C36 H33 F3 N6 O9 S | 782.19818 | -8.7 | -2.6 |
| 2-1-m1E01-1-0895 | C40 H38 N4 O9 S2 | 782.20802 | -1.6 | 0.3 |
| 2-1-m1E01-1-0896 | C37 H33 F4 N5 O8 S | 783.19860 | -0.7 | 1.5 |
| 2-1-m1E01-1-0897 | C39 H36 N4 O8 S3 | 784.16953 | -9.2 | 0.6 |
| 2-1-m1E01-1-0898 | C38 H35 N5 O8 S3 | 785.16478 | 0.0 | 11.4 |
| 2-1-m1E01-1-0899 | C37 H35 F4 N5 O8 S | 785.21425 | 0.0 | 0.0 |
| 2-1-m1E01-1-0900 | C37 H34 N6 O8 S3 | 786.16002 | -1.0 | 1.5 |
| 2-1-m1E01-1-0901 | C39 H35 F N4 O9 S2 | 786.18295 | 0.0 | 0.0 |
| 2-1-m1E01-1-0902 | C38 H32 F6 N4 O6 S | 786.19467 | 0.0 | 0.0 |
| 2-1-m1E01-1-0903 | C39 H33 F3 N6 O7 S | 786.20835 | -12.3 | -0.5 |
| 2-1-m1E01-1-0904 | C39 H42 N6 O8 S2 | 786.25055 | 0.0 | 2.5 |
| 2-1-m1E01-1-0905 | C42 H41 F3 N4 O6 S | 786.26989 | 3.0 | 9.1 |
| 2-1-m1E01-1-0906 | C35 H32 F3 N5 O9 S2 | 787.15935 | -1.0 | 2.4 |
| 2-1-m1E01-1-0907 | C38 H37 N5 O10 S2 | 787.19818 | -3.8 | 0.7 |
| 2-1-m1E01-1-0908 | C35 H31 F3 N4 O8 S3 | 788.12561 | -9.5 | 9.4 |
| 2-1-m1E01-1-0909 | C35 H31 F3 N4 O10 S2 | 788.14337 | -4.4 | -0.7 |
| 2-1-m1E01-1-0910 | C38 H33 F N4 O8 S3 | 788.14445 | 0.0 | 0.4 |
| 2-1-m1E01-1-0911 | C37 H36 N6 O10 S2 | 788.19343 | 0.0 | 1.7 |
| 2-1-m1E01-1-0912 | C40 H35 F3 N4 O8 S | 788.21277 | -8.0 | 5.3 |
| 2-1-m1E01-1-0913 | C37 H35 N5 O9 S3 | 789.15969 | 6.0 | 10.8 |
| 2-1-m1E01-1-0914 | C39 H34 F3 N5 O6 S2 | 789.19026 | -15.6 | -37.1 |
| 2-1-m1E01-1-0915 | C38 H34 F3 N7 O7 S | 789.21925 | 0.0 | 0.0 |
| 2-1-m1E01-1-0916 | C36 H34 N6 O9 S3 | 790.15494 | -4.3 | 2.1 |
| 2-1-m1E01-1-0917 | C36 H34 N6 O9 S3 | 790.15494 | -1.1 | 1.1 |
| 2-1-m1E01-1-0918 | C39 H33 F3 N4 O7 S2 | 790.17428 | -26.0 | 28.6 |
| 2-1-m1E01-1-0919 | C39 H33 F3 N4 O7 S2 | 790.17428 | 0.0 | 0.0 |
| 2-1-m1E01-1-0920 | C34 H32 F3 N5 O10 S2 | 791.15427 | 0.3 | 7.4 |
| 2-1-m1E01-1-0921 | C39 H36 F3 N5 O8 S | 791.22367 | -3.7 | 12.3 |
| 2-1-m1E01-1-0922 | C38 H34 F3 N5 O7 S2 | 793.18517 | -4.5 | 7.2 |
| 2-1-m1E01-1-0923 | C38 H34 F3 N5 O7 S2 | 793.18517 | 0.0 | 20.5 |
| 2-1-m1E01-1-0924 | C40 H42 F3 N5 O7 S | 793.27570 | 0.0 | 0.0 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0925 | C37 H33 F3 N6 O7 S2 | 794.18042 | -4.7 | 0.8 |
| 2-1-m1E01-1-0926 | C41 H42 N6 O7 S2 | 794.25564 | -14.7 | 1.9 |
| 2-1-m1E01-1-0927 | C40 H41 F3 N4 O8 S | 794.25972 | 0.0 | 0.4 |
| 2-1-m1E01-1-0928 | C34 H32 N6 O9 S4 | 796.11136 | -3.4 | 5.6 |
| 2-1-m1E01-1-0929 | C37 H35 F3 N6 O9 S | 796.21383 | 0.0 | 0.0 |
| 2-1-m1E01-1-0930 | C41 H40 N4 O9 S2 | 796.22367 | -4.4 | -3.5 |
| 2-1-m1E01-1-0931 | C38 H35 N7 O7 S3 | 797.17601 | -10.8 | 6.4 |
| 2-1-m1E01-1-0932 | C38 H38 F3 N5 O9 S | 797.23423 | 0.0 | 0.0 |
| 2-1-m1E01-1-0933 | C39 H42 F3 N5 O8 S | 797.27062 | -0.7 | 0.2 |
| 2-1-m1E01-1-0934 | C37 H33 F3 N4 O9 S2 | 798.16410 | 6.5 | 0.4 |
| 2-1-m1E01-1-0935 | C40 H38 N4 O8 S3 | 798.18518 | -108.6 | 0.0 |
| 2-1-m1E01-1-0936 | C40 H38 N4 O10 S2 | 798.20293 | -10.3 | -1.5 |
| 2-1-m1E01-1-0937 | C39 H37 N5 O8 S3 | 799.18043 | -49.3 | -2.3 |
| 2-1-m1E01-1-0938 | C37 H33 F4 N5 O9 S | 799.19351 | -2.4 | 0.7 |
| 2-1-m1E01-1-0939 | C39 H36 N4 O9 S3 | 800.16444 | -2.5 | 18.4 |
| 2-1-m1E01-1-0940 | C36 H34 F3 N5 O9 S2 | 801.17500 | -4.8 | 2.1 |
| 2-1-m1E01-1-0941 | C39 H39 N5 O10 S2 | 801.21383 | -2.8 | 4.1 |
| 2-1-m1E01-1-0942 | C38 H32 F6 N4 O7 S | 802.18959 | 1.9 | 9.1 |
| 2-1-m1E01-1-0943 | C42 H41 F3 N4 O7 S | 802.26480 | -15.0 | -4.3 |
| 2-1-m1E01-1-0944 | C38 H34 F N5 O8 S3 | 803.15535 | 10.8 | 1.6 |
| 2-1-m1E01-1-0945 | C38 H37 N5 O9 S3 | 803.17534 | -4.1 | 1.8 |
| 2-1-m1E01-1-0946 | C35 H31 F3 N4 O9 S3 | 804.12053 | 9.8 | 6.3 |
| 2-1-m1E01-1-0947 | C37 H36 N6 O9 S3 | 804.17059 | -2.4 | -0.1 |
| 2-1-m1E01-1-0948 | C39 H34 F2 N4 O9 S2 | 804.17353 | 3.8 | -2.5 |
| 2-1-m1E01-1-0949 | C36 H35 N7 O9 S3 | 805.16584 | -3.6 | 0.2 |
| 2-1-m1E01-1-0950 | C39 H34 F3 N5 O7 S2 | 805.18517 | -6.4 | 10.6 |
| 2-1-m1E01-1-0951 | C38 H36 F N5 O10 S2 | 805.18876 | -5.1 | 2.0 |
| 2-1-m1E01-1-0952 | C37 H33 F6 N5 O7 S | 805.20049 | 0.5 | 4.5 |
| 2-1-m1E01-1-0953 | C38 H34 F3 N7 O8 S | 805.21417 | 0.0 | 0.0 |
| 2-1-m1E01-1-0954 | C41 H42 F3 N5 O7 S | 805.27570 | -6.3 | 2.5 |
| 2-1-m1E01-1-0955 | C38 H32 F2 N4 O8 S3 | 806.13503 | -1.6 | 7.6 |
| 2-1-m1E01-1-0956 | C34 H32 F3 N5 O9 S3 | 807.13142 | -4.7 | 2.6 |
| 2-1-m1E01-1-0957 | C34 H32 F3 N5 Oil S2 | 807.14918 | 0.0 | 0.0 |
| 2-1-m1E01-1-0958 | C37 H34 F N5 O9 S3 | 807.15027 | -3.1 | 3.4 |
| 2-1-m1E01-1-0959 | C39 H36 F3 N5 O9 S | 807.21858 | -2.7 | 7.3 |
| 2-1-m1E01-1-0960 | C38 H35 F3 N6 O7 S2 | 808.19607 | -8.6 | 2.9 |

(continued)

[Table 11-2-1]

| | | | AS-MS ratio (%) | |
|---|---|---|---|---|
| ID | Molecular Formula | Exact Mass | N1 | N2 |
| 2-1-m1E01-1-0961 | C38 H34 F3 N5 O8 S2 | 809.18009 | -8.9 | 4.9 |
| 2-1-m1E01-1-0962 | C38 H34 F3 N5 O8 S2 | 809.18009 | 0.0 | 0.0 |
| 2-1-m1E01-1-0963 | C40 H39 N5 O8 S3 | 813.19608 | -5.8 | 3.7 |
| 2-1-m1E01-1-0964 | C39 H42 F3 N5 O9 S | 813.26553 | -2.3 | 0.1 |
| 2-1-m1E01-1-0965 | C39 H37 N5 O9 S3 | 815.17534 | 0.0 | 7.8 |
| 2-1-m1E01-1-0966 | C40 H41 N5 O10 S2 | 815.22948 | -7.0 | 6.5 |
| 2-1-m1E01-1-0967 | C36 H34 F3 N5 O10 S2 | 817.16992 | -3.2 | 1.4 |
| 2-1-m1E01-1-0968 | C39 H39 N5 O9 S3 | 817.19099 | -5.4 | 7.2 |
| 2-1-m1E01-1-0969 | C39 H39 N5 Oil S2 | 817.20875 | 10.1 | 2.4 |
| 2-1-m1E01-1-0970 | C38 H38 N6 O9 S3 | 818.18624 | -4.1 | 2.6 |
| 2-1-m1E01-1-0971 | C43 H38 N4 O9 S2 | 818.20802 | -1.9 | 5.8 |
| 2-1-m1E01-1-0972 | C38 H37 N5 O10 S3 | 819.17025 | 0.0 | 0.0 |
| 2-1-m1E01-1-0973 | C42 H36 N4 O8 S3 | 820.16953 | -3.8 | -17.3 |
| 2-1-m1E01-1-0974 | C38 H33 F2 N5 O8 S3 | 821.14593 | -1.9 | 5.0 |
| 2-1-m1E01-1-0975 | C37 H33 F6 N5 O8 S | 821.19540 | 0.4 | 5.6 |
| 2-1-m1E01-1-0976 | C41 H42 F3 N5 O8 S | 821.27062 | -3.3 | 4.9 |
| 2-1-m1E01-1-0977 | C37 H35 F N6 O9 S3 | 822.16117 | 0.0 | 0.0 |
| 2-1-m1E01-1-0978 | C34 H32 F3 N5 O10 S3 | 823.12634 | 0.9 | 0.4 |
| 2-1-m1E01-1-0979 | C38 H35 F2 N5 O10 S2 | 823.17934 | -2.5 | 6.5 |
| 2-1-m1E01-1-0980 | C38 H35 F3 N6 O8 S2 | 824.19099 | -3.6 | 7.3 |
| 2-1-m1E01-1-0981 | C37 H33 F2 N5 O9 S3 | 825.14085 | -3.0 | -4.0 |
| 2-1-m1E01-1-0982 | C39 H40 N6 O9 S3 | 832.20189 | -4.7 | 3.0 |
| 2-1-m1E01-1-0983 | C38 H38 N6 O10 S3 | 834.18115 | 0.0 | 3.7 |
| 2-1-m1E01-1-0984 | C42 H37 N5 O8 S3 | 835.18043 | 3.7 | 2.3 |
| 2-1-m1E01-1-0985 | C40 H35 F3 N4 O9 S2 | 836.17975 | 2.5 | 9.7 |
| 2-1-m1E01-1-0986 | C39 H33 F3 N4 O8 S3 | 838.14126 | -3.8 | 4.1 |
| 2-1-m1E01-1-0987 | C37 H34 F2 N6 O9 S3 | 840.15175 | 1.3 | -0.2 |
| 2-1-m1E01-1-0988 | C39 H36 N6 O10 S3 | 844.16550 | -3.9 | 3.7 |
| 2-1-m1E01-1-0989 | C38 H34 N6 O9 S4 | 846.12701 | -3.0 | 1.5 |
| 2-1-m1E01-1-0990 | C40 H35 F3 N4 O10 S2 | 852.17467 | 2.0 | -0.9 |
| 2-1-m1E01-1-0991 | C39 H34 F3 N5 O8 S3 | 853.15216 | -0.7 | 0.0 |
| 2-1-m1E01-1-0992 | C39 H33 F3 N4 O9 S3 | 854.13618 | -3.5 | 5.6 |
| 2-1-m1E01-1-0993 | C39 H36 F3 N5 O10 S2 | 855.18557 | -1.2 | -1.8 |
| 2-1-m1E01-1-0994 | C38 H34 F3 N5 O9 S3 | 857.14707 | -11.1 | 15.8 |
| 2-1-m1E01-1-0995 | C38 H35 N7 O9 S4 | 861.13791 | -3.9 | 2.8 |
| 2-1-m1E01-1-0996 | C39 H34 F3 N5 O9 S3 | 869.14707 | 1.1 | 4.4 |

(continued)

| [Table 11-2-1] | | | | |
|---|---|---|---|---|
| | | | AS-MS ratio (%) | |
| ID | Molecular Formula | Exact Mass | N1 | N2 |
| 2-1-m1E01-1-0997 | C39 H36 F3 N5 O11 S2 | 871.18048 | -3.8 | 2.8 |
| 2-1-m1E01-1-0998 | C38 H35 F3 N6 O9 S3 | 872.15797 | -4.4 | -0.1 |
| 2-1-m1E01-1-0999 | C38 H34 F3 N5 O10 S3 | 873.14199 | -10.6 | 20.8 |
| 2-1-m1E01-1-1000 | C38 H35 F3 N6 O10 S3 | 888.15289 | -4.4 | 5.2 |

[Table 591]

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0001 | C36 H34 N4 O5 | 602.25292 | 0.8 | 1.5 |
| 2-1-m1E02-1-0002 | C35 H33 N5 O5 | 603.24817 | -4.6 | -4.5 |
| 2-1-m1E02-1-0003 | C35 H33 N5 O5 | 603.24817 | -2.2 | -1.6 |
| 2-1-m1E02-1-0004 | C34 H32 N6 O5 | 604.24342 | -7.8 | -7.9 |
| 2-1-m1E02-1-0005 | C34 H32 N6 O5 | 604.24342 | -7.8 | -6.5 |
| 2-1-m1E02-1-0006 | C33 H31 N7 O5 | 605.23867 | -3.9 | -1.4 |
| 2-1-m1E02-1-0007 | C33 H31 N5 O5 S | 609.20459 | -7.2 | -7.3 |
| 2-1-m1E02-1-0008 | C32 H30 N6 O5 S | 610.19984 | -3.5 | 0.0 |
| 2-1-m1E02-1-0009 | C37 H36 N4 O5 | 616.26857 | -4.4 | -7.9 |
| 2-1-m1E02-1-0010 | C36 H35 N5 O5 | 617.26382 | -0.2 | -3.5 |
| 2-1-m1E02-1-0011 | C35 H34 N6 O5 | 618.25907 | 0.0 | -10.7 |
| 2-1-m1E02-1-0012 | C35 H32 N4 O7 | 620.22710 | -8.1 | -6.8 |
| 2-1-m1E02-1-0013 | C36 H33 F N4 O5 | 620.24350 | -7.6 | -11.6 |
| 2-1-m1E02-1-0014 | C34 H31 N5 O7 | 621.22235 | -6.1 | -4.2 |
| 2-1-m1E02-1-0015 | C35 H32 F N5 O5 | 621.23875 | -6.7 | -0.5 |
| 2-1-m1E02-1-0016 | C35 H35 N5 O6 | 621.25873 | -5.6 | -5.0 |
| 2-1-m1E02-1-0017 | C34 H31 F N6 O5 | 622.23400 | -7.5 | -7.0 |
| 2-1-m1E02-1-0018 | C34 H34 N6 O6 | 622.25398 | -5.5 | -5.4 |
| 2-1-m1E02-1-0019 | C34 H34 N6 O6 | 622.25398 | -3.7 | -2.4 |
| 2-1-m1E02-1-0020 | C34 H33 N5 O5 S | 623.22024 | -19.7 | 0.0 |
| 2-1-m1E02-1-0021 | C33 H33 N7 O6 | 623.24923 | -1.4 | -0.4 |
| 2-1-m1E02-1-0022 | C33 H33 N7 O6 | 623.24923 | -9.5 | -2.2 |
| 2-1-m1E02-1-0023 | C32 H32 N8 O6 | 624.24448 | 0.0 | 0.0 |
| 2-1-m1E02-1-0024 | C33 H30 F N5 O5 S | 627.19517 | -7.9 | -8.3 |
| 2-1-m1E02-1-0025 | C32 H32 N6 O6 S | 628.21040 | 0.0 | 0.0 |
| 2-1-m1E02-1-0026 | C31 H31 N7 O6 S | 629.20565 | 0.0 | -3.5 |
| 2-1-m1E02-1-0027 | C37 H34 N4 O6 | 630.24783 | -6.6 | -0.1 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0028 | C38 H38 N4 O5 | 630.28422 | -3.5 | -14.1 |
| 2-1-m1E02-1-0029 | C36 H33 N5 O6 | 631.24308 | -7.8 | -4.5 |
| 2-1-m1E02-1-0030 | C36 H33 N5 O6 | 631.24308 | -6.1 | -1.1 |
| 2-1-m1E02-1-0031 | C37 H37 N5 O5 | 631.27947 | -8.5 | -5.6 |
| 2-1-m1E02-1-0032 | C35 H32 N6 O6 | 632.23833 | -3.3 | -4.4 |
| 2-1-m1E02-1-0033 | C37 H36 N4 O6 | 632.26348 | 0.0 | -4.6 |
| 2-1-m1E02-1-0034 | C36 H36 N6 O5 | 632.27472 | -7.0 | -13.1 |
| 2-1-m1E02-1-0035 | C36 H35 N5 O6 | 633.25873 | -5.7 | -4.4 |
| 2-1-m1E02-1-0036 | C36 H34 N4 O7 | 634.24275 | -4.5 | -7.5 |
| 2-1-m1E02-1-0037 | C35 H34 N6 O6 | 634.25398 | -10.5 | -6.8 |
| 2-1-m1E02-1-0038 | C36 H37 N5 O6 | 635.27438 | -4.6 | -3.9 |
| 2-1-m1E02-1-0039 | C35 H32 N4 O6 S | 636.20426 | -7.7 | -6.6 |
| 2-1-m1E02-1-0040 | C35 H36 N6 O6 | 636.26963 | -6.8 | -5.5 |
| 2-1-m1E02-1-0041 | C34 H31 N5 O6 S | 637.19950 | -5.8 | -6.7 |
| 2-1-m1E02-1-0042 | C35 H35 N5 O5 S | 637.23589 | -8.5 | -6.9 |
| 2-1-m1E02-1-0043 | C34 H35 N7 O6 | 637.26488 | -6.2 | -9.1 |
| 2-1-m1E02-1-0044 | C35 H31 F N4 O7 | 638.21768 | -5.3 | -8.6 |
| 2-1-m1E02-1-0045 | C36 H32 F2 N4 O5 | 638.23408 | -6.1 | -6.9 |
| 2-1-m1E02-1-0046 | C34 H33 N5 O6 S | 639.21515 | -10.2 | -5.2 |
| 2-1-m1E02-1-0047 | C35 H31 F2 N5 O5 | 639.22933 | 0.1 | -3.1 |
| 2-1-m1E02-1-0048 | C34 H33 N5 O8 | 639.23291 | -0.2 | -1.8 |
| 2-1-m1E02-1-0049 | C35 H34 F N5 O6 | 639.24931 | -1.5 | 2.1 |
| 2-1-m1E02-1-0050 | C34 H30 F2 N6 O5 | 640.22457 | -5.1 | -11.6 |
| 2-1-m1E02-1-0051 | C33 H32 N6 O8 | 640.22816 | -1.4 | -1.8 |
| 2-1-m1E02-1-0052 | C34 H33 F N6 O6 | 640.24456 | -3.6 | -4.3 |
| 2-1-m1E02-1-0053 | C33 H32 F N7 O6 | 641.23981 | -5.6 | -4.6 |
| 2-1-m1E02-1-0054 | C33 H34 N6 O6 S | 642.22605 | -5.6 | -4.5 |
| 2-1-m1E02-1-0055 | C38 H36 N4 O6 | 644.26348 | -5.0 | -6.2 |
| 2-1-m1E02-1-0056 | C33 H29 F2 N5 O5 S | 645.18575 | -4.9 | -16.4 |
| 2-1-m1E02-1-0057 | C37 H35 N5 O6 | 645.25873 | -6.8 | -2.9 |
| 2-1-m1E02-1-0058 | C37 H35 N5 O6 | 645.25873 | -3.2 | -6.3 |
| 2-1-m1E02-1-0059 | C32 H31 F N6 O6 S | 646.20098 | -4.6 | 0.0 |
| 2-1-m1E02-1-0060 | C36 H34 N6 O6 | 646.25398 | -4.1 | -3.0 |
| 2-1-m1E02-1-0061 | C37 H33 F N4 O6 | 648.23841 | -6.0 | -6.7 |
| 2-1-m1E02-1-0062 | C37 H33 F N4 O6 | 648.23841 | -8.0 | -7.2 |
| 2-1-m1E02-1-0063 | C37 H36 N4 O7 | 648.25840 | -8.5 | -6.8 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0064 | C36 H32 F N5 O6 | 649.23366 | -3.8 | -2.4 |
| 2-1-m1E02-1-0065 | C36 H32 F N5 O6 | 649.23366 | -8.4 | -8.7 |
| 2-1-m1E02-1-0066 | C36 H35 N5 O7 | 649.25365 | 0.0 | -0.6 |
| 2-1-m1E02-1-0067 | C37 H39 N5 O6 | 649.29003 | -2.9 | -5.9 |
| 2-1-m1E02-1-0068 | C36 H34 N4 O6 S | 650.21991 | -8.7 | -3.8 |
| 2-1-m1E02-1-0069 | C36 H34 N4 O8 | 650.23766 | 0.0 | 0.0 |
| 2-1-m1E02-1-0070 | C35 H34 N6 O7 | 650.24890 | -0.9 | -1.7 |
| 2-1-m1E02-1-0071 | C35 H34 N6 O7 | 650.24890 | 0.0 | 0.0 |
| 2-1-m1E02-1-0072 | C36 H38 N6 O6 | 650.28528 | -6.0 | -6.4 |
| 2-1-m1E02-1-0073 | C34 H33 N7 O7 | 651.24415 | -1.3 | -1.7 |
| 2-1-m1E02-1-0074 | C36 H37 N5 O7 | 651.26930 | -4.3 | -7.8 |
| 2-1-m1E02-1-0075 | C35 H37 N7 O6 | 651.28053 | 0.0 | 0.0 |
| 2-1-m1E02-1-0076 | C35 H36 N6 O7 | 652.26455 | -2.9 | 0.0 |
| 2-1-m1E02-1-0077 | C40 H36 N4 O5 | 652.26857 | -4.4 | -21.0 |
| 2-1-m1E02-1-0078 | C35 H35 N5 O8 | 653.24856 | -4.9 | -3.7 |
| 2-1-m1E02-1-0079 | C34 H35 N7 O7 | 653.25980 | 0.0 | 0.0 |
| 2-1-m1E02-1-0080 | C39 H35 N5 O5 | 653.26382 | -7.3 | -1.0 |
| 2-1-m1E02-1-0081 | C35 H31 F N4 O6 S | 654.19483 | -5.9 | -6.3 |
| 2-1-m1E02-1-0082 | C38 H34 N6 O5 | 654.25907 | -5.3 | -6.9 |
| 2-1-m1E02-1-0083 | C38 H34 N6 O5 | 654.25907 | -2.9 | -7.9 |
| 2-1-m1E02-1-0084 | C34 H33 N5 O7 S | 655.21007 | 0.0 | -1.2 |
| 2-1-m1E02-1-0085 | C37 H33 N7 O5 | 655.25432 | -4.3 | -4.9 |
| 2-1-m1E02-1-0086 | C34 H32 N4 O8 S | 656.19408 | 0.0 | -4.0 |
| 2-1-m1E02-1-0087 | C33 H32 N6 O7 S | 656.20532 | -1.4 | -2.1 |
| 2-1-m1E02-1-0088 | C35 H30 F2 N4 O7 | 656.20826 | -3.8 | -6.5 |
| 2-1-m1E02-1-0089 | C34 H36 N6 O6 S | 656.24170 | -7.0 | -1.7 |
| 2-1-m1E02-1-0090 | C33 H31 N5 O8 S | 657.18933 | -10.7 | -9.0 |
| 2-1-m1E02-1-0091 | C34 H32 F N5 O8 | 657.22349 | -1.2 | -1.2 |
| 2-1-m1E02-1-0092 | C35 H33 F2 N5 O6 | 657.23989 | -4.8 | -6.9 |
| 2-1-m1E02-1-0093 | C33 H34 N6 O7 S | 658.22097 | 0.0 | 0.0 |
| 2-1-m1E02-1-0094 | C38 H34 N4 O5 S | 658.22499 | 0.0 | 0.0 |
| 2-1-m1E02-1-0095 | C34 H32 F2 N6 O6 | 658.23514 | -7.7 | -9.1 |
| 2-1-m1E02-1-0096 | C35 H38 N4 O7 S | 658.24612 | -0.4 | -2.7 |
| 2-1-m1E02-1-0097 | C39 H38 N4 O6 | 658.27913 | -6.3 | -5.3 |
| 2-1-m1E02-1-0098 | C37 H33 N5 O5 S | 659.22024 | -7.9 | -20.7 |
| 2-1-m1E02-1-0099 | C37 H33 N5 O5 S | 659.22024 | 0.0 | -3.3 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0100 | C33 H31 F2 N7 O6 | 659.23039 | -3.1 | -2.4 |
| 2-1-m1E02-1-0101 | C34 H37 N5 O7 S | 659.24137 | -4.7 | -2.7 |
| 2-1-m1E02-1-0102 | C38 H37 N5 O6 | 659.27438 | -7.9 | -6.0 |
| 2-1-m1E02-1-0103 | C38 H37 N5 O6 | 659.27438 | -7.0 | -9.0 |
| 2-1-m1E02-1-0104 | C38 H36 N4 O7 | 660.25840 | -7.5 | -3.3 |
| 2-1-m1E02-1-0105 | C37 H35 N5 O7 | 661.25365 | -3.4 | -4.8 |
| 2-1-m1E02-1-0106 | C38 H35 F N4 O6 | 662.25406 | -8.1 | -3.3 |
| 2-1-m1E02-1-0107 | C39 H42 N4 O6 | 662.31044 | -5.5 | -5.1 |
| 2-1-m1E02-1-0108 | C37 H34 F N5 O6 | 663.24931 | -8.8 | -5.0 |
| 2-1-m1E02-1-0109 | C37 H37 N5 O7 | 663.26930 | -4.4 | -3.7 |
| 2-1-m1E02-1-0110 | C38 H41 N5 O6 | 663.30568 | 0.0 | -1.9 |
| 2-1-m1E02-1-0111 | C32 H30 F2 N6 O6 S | 664.19156 | -1.4 | -3.9 |
| 2-1-m1E02-1-0112 | C37 H36 N4 O6 S | 664.23556 | -3.6 | -4.1 |
| 2-1-m1E02-1-0113 | C36 H36 N6 O7 | 664.26455 | -3.0 | -1.5 |
| 2-1-m1E02-1-0114 | C36 H36 N6 O7 | 664.26455 | -5.1 | -4.5 |
| 2-1-m1E02-1-0115 | C35 H35 N7 O7 | 665.25980 | -1.2 | -0.6 |
| 2-1-m1E02-1-0116 | C36 H34 N4 O7 S | 666.21482 | -9.0 | -6.8 |
| 2-1-m1E02-1-0117 | C36 H34 N4 O7 S | 666.21482 | -8.6 | -3.9 |
| 2-1-m1E02-1-0118 | C36 H34 N4 O7 S | 666.21482 | -6.7 | -5.7 |
| 2-1-m1E02-1-0119 | C37 H32 F2 N4 O6 | 666.22899 | -6.2 | -5.5 |
| 2-1-m1E02-1-0120 | C37 H32 F2 N4 O6 | 666.22899 | 0.0 | 0.0 |
| 2-1-m1E02-1-0121 | C35 H33 N5 O7 S | 667.21007 | -8.2 | -10.4 |
| 2-1-m1E02-1-0122 | C35 H33 N5 O7 S | 667.21007 | -3.0 | -2.7 |
| 2-1-m1E02-1-0123 | C35 H33 N5 O7 S | 667.21007 | 0.0 | 0.0 |
| 2-1-m1E02-1-0124 | C36 H31 F2 N5 O6 | 667.22424 | 0.0 | 0.0 |
| 2-1-m1E02-1-0125 | C36 H34 F N5 O7 | 667.24423 | -0.8 | -0.6 |
| 2-1-m1E02-1-0126 | C36 H34 F N5 O7 | 667.24423 | 0.0 | 0.0 |
| 2-1-m1E02-1-0127 | C36 H37 N5 O8 | 667.26421 | -5.2 | -5.4 |
| 2-1-m1E02-1-0128 | C34 H32 N6 O7 S | 668.20532 | -0.3 | -1.9 |
| 2-1-m1E02-1-0129 | C34 H32 N6 O7 S | 668.20532 | -5.2 | -5.4 |
| 2-1-m1E02-1-0130 | C35 H33 F N6 O7 | 668.23948 | -1.1 | -1.0 |
| 2-1-m1E02-1-0131 | C35 H33 F N6 O7 | 668.23948 | -1.2 | 0.0 |
| 2-1-m1E02-1-0132 | C39 H36 N6 O5 | 668.27472 | -4.3 | -2.6 |
| 2-1-m1E02-1-0133 | C33 H31 N7 O7 S | 669.20057 | -0.1 | 0.0 |
| 2-1-m1E02-1-0134 | C35 H35 N5 O7 S | 669.22572 | -6.3 | -5.4 |
| 2-1-m1E02-1-0135 | C35 H35 N5 O9 | 669.24348 | -0.9 | -1.4 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0136 | C35 H34 N4 O8 S | 670.20973 | 0.0 | 0.0 |
| 2-1-m1E02-1-0137 | C37 H33 F3 N4 O5 | 670.24030 | -0.2 | 1.0 |
| 2-1-m1E02-1-0138 | C37 H33 F3 N4 05 | 670.24030 | -6.7 | -7.7 |
| 2-1-m1E02-1-0139 | C39 H34 N4 O7 | 670.24275 | -2.8 | -5.5 |
| 2-1-m1E02-1-0140 | C36 H32 F3 N5 O5 | 671.23555 | -4.7 | -7.1 |
| 2-1-m1E02-1-0141 | C36 H32 F3 N5 O5 | 671.23555 | -9.1 | -5.3 |
| 2-1-mlE02-1-0142 | C34 H32 N4 O7 S2 | 672.17124 | 0.0 | 0.0 |
| 2-1-m1E02-1-0143 | C35 H30 F2 N4 O6 S | 672.18541 | -6.0 | -7.4 |
| 2-1-m1E02-1-0144 | C35 H31 F3 N6 O5 | 672.23080 | -7.9 | -2.2 |
| 2-1-m1E02-1-0145 | C39 H36 N4 O5 S | 672.24064 | -2.4 | -11.2 |
| 2-1-m1E02-1-0146 | C38 H33 F N6 O5 | 672.24965 | -4.9 | -6.6 |
| 2-1-m1E02-1-0147 | C36 H40 N4 O7 S | 672.26177 | -5.1 | -3.7 |
| 2-1-m1E02-1-0148 | C33 H31 N5 O7 S2 | 673.16649 | -9.3 | -11.7 |
| 2-1-m1E02-1-0149 | C33 H31 N5 O7 S2 | 673.16649 | -6.3 | 0.0 |
| 2-1-m1E02-1-0150 | C34 H32 F N5 O7 S | 673.20065 | 0.0 | -3.1 |
| 2-1-m1E02-1-0151 | C37 H35 N7 O6 | 673.26488 | 0.0 | 0.0 |
| 2-1-m1E02-1-0152 | C39 H39 N5 O6 | 673.29003 | -5.4 | -3.4 |
| 2-1-m1E02-1-0153 | C32 H30 N6 O7 S2 | 674.16174 | -0.5 | -0.4 |
| 2-1-m1E02-1-0154 | C34 H31 F N4 O8 S | 674.18466 | 0.0 | 0.0 |
| 2-1-m1E02-1-0155 | C36 H34 N8 O6 | 674.26013 | -2.6 | -2.1 |
| 2-1-m1E02-1-0156 | C33 H33 N5 O9 S | 675.19990 | -8.1 | -4.7 |
| 2-1-m1E02-1-0157 | C34 H31 F2 N5 O8 | 675.21407 | 0.0 | 0.0 |
| 2-1-m1E02-1-0158 | C38 H37 N5 O7 | 675.26930 | -4.0 | -1.8 |
| 2-1-m1E02-1-0159 | C32 H32 N6 O9 S | 676.19515 | -3.3 | -3.8 |
| 2-1-m1E02-1-0160 | C38 H33 F N4 O5 S | 676.21557 | 17.5 | -1.6 |
| 2-1-m1E02-1-0161 | C35 H37 F N4 O7 S | 676.23670 | -11.0 | -8.1 |
| 2-1-m1E02-1-0162 | C39 H37 F N4 O6 | 676.26971 | -4.5 | -5.7 |
| 2-1-m1E02-1-0163 | C40 H44 N4 O6 | 676.32609 | -6.5 | 1.4 |
| 2-1-m1E02-1-0164 | C34 H30 F3 N5 O5 S | 677.19197 | 1.5 | -9.3 |
| 2-1-m1E02-1-0165 | C37 H35 N5 O6 S | 677.23080 | -8.2 | -6.1 |
| 2-1-m1E02-1-0166 | C34 H39 N5 O8 S | 677.25193 | 0.0 | 0.0 |
| 2-1-m1E02-1-0167 | C38 H39 N5 O7 | 677.28495 | -4.0 | -4.4 |
| 2-1-m1E02-1-0168 | C36 H34 N6 O6 S | 678.22605 | -5.5 | -4.6 |
| 2-1-m1E02-1-0169 | C36 H34 N6 O6 S | 678.22605 | 4.7 | -6.3 |
| 2-1-m1E02-1-0170 | C33 H38 N6 O8 S | 678.24718 | -1.1 | -0.8 |
| 2-1-m1E02-1-0171 | C38 H35 F N4 O7 | 678.24898 | -1.1 | -1.5 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0172 | C38 H38 N4 O6 S | 678.25121 | -6.8 | -6.8 |
| 2-1-m1E02-1-0173 | C37 H38 N6 O7 | 678.28020 | -0.4 | -0.5 |
| 2-1-m1E02-1-0174 | C37 H38 N6 O7 | 678.28020 | -4.9 | -3.6 |
| 2-1-m1E02-1-0175 | C35 H33 N7 O6 S | 679.22130 | -8.9 | -7.1 |
| 2-1-m1E02-1-0176 | C37 H37 N5 O6 S | 679.24645 | 0.0 | 0.0 |
| 2-1-m1E02-1-0177 | C37 H37 N5 O8 | 679.26421 | 0.0 | 15.8 |
| 2-1-m1E02-1-0178 | C34 H32 N8 O6 S | 680.21655 | -4.6 | -3.9 |
| 2-1-m1E02-1-0179 | C37 H36 N4 O7 S | 680.23047 | 3.9 | -2.0 |
| 2-1-m1E02-1-0180 | C37 H36 N4 O7 S | 680.23047 | -7.7 | -6.3 |
| 2-1-m1E02-1-0181 | C37 H36 N4 O7 S | 680.23047 | -1.3 | 2.5 |
| 2-1-m1E02-1-0182 | C36 H36 N6 O8 | 680.25946 | 0.0 | 0.0 |
| 2-1-m1E02-1-0183 | C41 H36 N4 O6 | 680.26348 | -3.8 | -1.3 |
| 2-1-m1E02-1-0184 | C39 H41 F N4 O6 | 680.30101 | -5.0 | -4.7 |
| 2-1-m1E02-1-0185 | C36 H35 N5 O7 S | 681.22572 | -9.5 | -3.5 |
| 2-1-m1E02-1-0186 | C36 H35 N5 O7 S | 681.22572 | -2.1 | 0.0 |
| 2-1-m1E02-1-0187 | C36 H35 N5 O7 S | 681.22572 | -5.2 | -5.1 |
| 2-1-m1E02-1-0188 | C37 H33 F2 N5 O6 | 681.23989 | -6.8 | -6.2 |
| 2-1-m1E02-1-0189 | C40 H35 N5 O6 | 681.25873 | -3.5 | -3.7 |
| 2-1-m1E02-1-0190 | C37 H36 F N5 O7 | 681.25988 | -1.3 | -5.2 |
| 2-1-m1E02-1-0191 | C38 H43 N5 O7 | 681.31625 | -0.5 | -1.5 |
| 2-1-m1E02-1-0192 | C35 H34 N6 O7 S | 682.22097 | 0.0 | 0.0 |
| 2-1-m1E02-1-0193 | C35 H34 N6 O7 S | 682.22097 | -7.7 | 0.0 |
| 2-1-m1E02-1-0194 | C36 H35 F N6 O7 | 682.25513 | -3.0 | -2.9 |
| 2-1-m1E02-1-0195 | C40 H38 N6 O5 | 682.29037 | -19.6 | -0.1 |
| 2-1-m1E02-1-0196 | C37 H42 N6 O7 | 682.31150 | -0.6 | -0.6 |
| 2-1-m1E02-1-0197 | C36 H37 N5 O7 S | 683.24137 | -5.0 | -4.1 |
| 2-1-m1E02-1-0198 | C35 H32 N4 O9 S | 684.18900 | 0.0 | 0.0 |
| 2-1-m1E02-1-0199 | C36 H33 F N4 O7 S | 684.20540 | -6.7 | -4.8 |
| 2-1-m1E02-1-0200 | C36 H33 F N4 O7 S | 684.20540 | -7.9 | -23.2 |
| 2-1-m1E02-1-0201 | C37 H31 F3 N4 O6 | 684.21957 | -8.1 | -8.6 |
| 2-1-m1E02-1-0202 | C36 H36 N4 O8 S | 684.22538 | 5.8 | -10.4 |
| 2-1-m1E02-1-0203 | C38 H35 F3 N4 O5 | 684.25595 | -1.5 | -8.4 |
| 2-1-m1E02-1-0204 | C39 H36 N6 O6 | 684.26963 | -2.7 | -6.6 |
| 2-1-m1E02-1-0205 | C33 H31 N7 O6 S2 | 685.17772 | 0.0 | -4.6 |
| 2-1-m1E02-1-0206 | C34 H31 N5 O9 S | 685.18425 | 0.0 | 0.0 |
| 2-1-m1E02-1-0207 | C35 H32 F N5 O7 S | 685.20065 | -9.8 | -4.7 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0208 | C35 H35 N5 O8 S | 685.22063 | 0.0 | -0.7 |
| 2-1-m1E02-1-0209 | C35 H35 N5 O8 S | 685.22063 | -3.2 | -1.1 |
| 2-1-m1E02-1-0210 | C35 H35 N5 O8 S | 685.22063 | -8.1 | -8.0 |
| 2-1-m1E02-1-0211 | C36 H33 F2 N5 O7 | 685.23480 | 0.0 | 0.0 |
| 2-1-m1E02-1-0212 | C36 H33 F2 N5 O7 | 685.23480 | -2.9 | -1.2 |
| 2-1-m1E02-1-0213 | C35 H34 N4 O7 S2 | 686.18689 | 0.0 | 0.0 |
| 2-1-m1E02-1-0214 | C34 H31 F N6 O7 S | 686.19590 | -7.5 | -1.0 |
| 2-1-m1E02-1-0215 | C35 H34 N4 O9 S | 686.20465 | -1.1 | 0.0 |
| 2-1-m1E02-1-0216 | C34 H34 N6 O8 S | 686.21588 | -7.8 | -4.9 |
| 2-1-m1E02-1-0217 | C34 H34 N6 O8 S | 686.21588 | 0.0 | 0.0 |
| 2-1-m1E02-1-0218 | C34 H34 N6 O8 S | 686.21588 | -0.5 | -1.4 |
| 2-1-m1E02-1-0219 | C39 H34 N4 O6 S | 686.21991 | 0.0 | -0.3 |
| 2-1-m1E02-1-0220 | C35 H32 F2 N6 O7 | 686.23005 | 0.0 | 0.0 |
| 2-1-m1E02-1-0221 | C37 H33 F3 N4 O6 | 686.23522 | -19.0 | -1.0 |
| 2-1-m1E02-1-0222 | C40 H38 N4 O5 S | 686.25629 | 2.4 | -1.7 |
| 2-1-m1E02-1-0223 | C37 H42 N4 O7 S | 686.27742 | 0.0 | 0.0 |
| 2-1-m1E02-1-0224 | C34 H33 N5 O7 S2 | 687.18214 | -5.5 | -5.1 |
| 2-1-m1E02-1-0225 | C33 H33 N7 O8 S | 687.21113 | -0.3 | 0.0 |
| 2-1-m1E02-1-0226 | C33 H33 N7 O8 S | 687.21113 | 0.0 | 0.0 |
| 2-1-m1E02-1-0227 | C36 H32 F3 N5 O6 | 687.23047 | -9.1 | -3.5 |
| 2-1-m1E02-1-0228 | C38 H37 N7 O6 | 687.28053 | -10.0 | -8.7 |
| 2-1-m1E02-1-0229 | C32 H32 N8 O8 S | 688.20638 | 0.0 | 0.0 |
| 2-1-m1E02-1-0230 | C36 H31 F3 N4 O7 | 688.21448 | -3.3 | -1.0 |
| 2-1-m1E02-1-0231 | C35 H31 F3 N6 O6 | 688.22572 | -11.4 | -8.0 |
| 2-1-m1E02-1-0232 | C37 H32 F4 N4 O5 | 688.23088 | -1.9 | -16.5 |
| 2-1-m1E02-1-0233 | C39 H36 N4 O6 S | 688.23556 | -10.6 | -8.4 |
| 2-1-m1E02-1-0234 | C36 H40 N4 O8 S | 688.25668 | -0.5 | -4.8 |
| 2-1-m1E02-1-0235 | C34 H35 N5 O9 S | 689.21555 | 0.0 | -4.6 |
| 2-1-m1E02-1-0236 | C36 H34 F3 N5 O6 | 689.24612 | -5.9 | -6.0 |
| 2-1-m1E02-1-0237 | C36 H34 F3 N5 O6 | 689.24612 | -9.3 | -3.3 |
| 2-1-m1E02-1-0238 | C34 H31 F N4 O7 S2 | 690.16182 | 0.0 | 0.0 |
| 2-1-m1E02-1-0239 | C38 H32 F2 N6 O5 | 690.24022 | -5.2 | -7.5 |
| 2-1-m1E02-1-0240 | C35 H33 F3 N6 O6 | 690.24137 | -9.5 | -4.5 |
| 2-1-m1E02-1-0241 | C35 H33 F3 N6 O6 | 690.24137 | 0.5 | -6.6 |
| 2-1-m1E02-1-0242 | C41 H46 N4 O6 | 690.34174 | 0.0 | 0.0 |
| 2-1-m1E02-1-0243 | C33 H30 F N5 O7 S2 | 691.15707 | -5.1 | -2.3 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0244 | C33 H33 N5 O8 S2 | 691.17705 | -7.8 | -5.1 |
| 2-1-m1E02-1-0245 | C34 H31 F2 N5 O7 S | 691.19123 | -2.8 | -1.5 |
| 2-1-m1E02-1-0246 | C34 H32 F3 N7 O6 | 691.23662 | 0.0 | 0.0 |
| 2-1-m1E02-1-0247 | C38 H37 N5 O6 S | 691.24645 | 0.3 | -3.9 |
| 2-1-m1E02-1-0248 | C37 H34 F N7 O6 | 691.25546 | -0.2 | -3.6 |
| 2-1-m1E02-1-0249 | C35 H41 N5 O8 S | 691.26758 | -3.4 | -3.3 |
| 2-1-m1E02-1-0250 | C32 H32 N6 O8 S2 | 692.17230 | -5.3 | -2.0 |
| 2-1-m1E02-1-0251 | C32 H32 N6 O8 S2 | 692.17230 | 0.0 | 0.0 |
| 2-1-m1E02-1-0252 | C34 H30 F2 N4 O8 S | 692.17524 | -5.5 | -2.0 |
| 2-1-m1E02-1-0253 | C39 H40 N4 O6 S | 692.26686 | -4.2 | -3.7 |
| 2-1-m1E02-1-0254 | C39 H40 N4 O6 S | 692.26686 | 4.7 | 0.0 |
| 2-1-m1E02-1-0255 | C38 H40 N6 O7 | 692.29585 | -3.3 | -3.9 |
| 2-1-m1E02-1-0256 | C40 H44 N4 O7 | 692.32100 | -5.2 | -6.8 |
| 2-1-m1E02-1-0257 | C31 H31 N7 O8 S2 | 693.16755 | 0.0 | 0.0 |
| 2-1-m1E02-1-0258 | C34 H30 F3 N5 O6 S | 693.18689 | -3.1 | -7.0 |
| 2-1-m1E02-1-0259 | C33 H32 F N5 O9 S | 693.19048 | -5.9 | -5.6 |
| 2-1-m1E02-1-0260 | C38 H39 N5 O6 S | 693.26210 | 3.8 | 0.4 |
| 2-1-m1E02-1-0261 | C38 H32 F2 N4 O5 S | 694.20615 | 0.0 | -0.8 |
| 2-1-m1E02-1-0262 | C37 H34 N4 O8 S | 694.20973 | -2.5 | -3.1 |
| 2-1-m1E02-1-0263 | C35 H36 F2 N4 O7 S | 694.22728 | -7.1 | -4.2 |
| 2-1-m1E02-1-0264 | C38 H38 N4 O7 S | 694.24612 | -8.9 | -5.7 |
| 2-1-m1E02-1-0265 | C38 H38 N4 O7 S | 694.24612 | -5.2 | -12.2 |
| 2-1-m1E02-1-0266 | C38 H38 N4 O7 S | 694.24612 | -6.6 | -10.5 |
| 2-1-m1E02-1-0267 | C38 H38 N4 O7 S | 694.24612 | -10.7 | -6.8 |
| 2-1-m1E02-1-0268 | C37 H38 N6 O8 | 694.27511 | 0.0 | 0.0 |
| 2-1-m1E02-1-0269 | C36 H33 N5 O8 S | 695.20498 | -0.4 | -0.6 |
| 2-1-m1E02-1-0270 | C36 H33 N5 O8 S | 695.20498 | -2.2 | -1.8 |
| 2-1-m1E02-1-0271 | C37 H34 F N5 O6 S | 695.22138 | 7.6 | -2.9 |
| 2-1-m1E02-1-0272 | C37 H37 N5 O7 S | 695.24137 | -15.6 | 1.0 |
| 2-1-m1E02-1-0273 | C37 H37 N5 O7 S | 695.24137 | -4.1 | -5.1 |
| 2-1-m1E02-1-0274 | C37 H37 N5 O7 S | 695.24137 | 0.9 | 0.0 |
| 2-1-m1E02-1-0275 | C34 H38 F N5 O8 S | 695.24251 | -15.6 | 1.0 |
| 2-1-m1E02-1-0276 | C41 H37 N5 O6 | 695.27438 | -8.4 | -3.1 |
| 2-1-m1E02-1-0277 | C38 H38 F N5 O7 | 695.27553 | -4.0 | -3.6 |
| 2-1-m1E02-1-0278 | C39 H45 N5 O7 | 695.33190 | 0.0 | 0.0 |
| 2-1-m1E02-1-0279 | C33 H31 F3 N6 O6 S | 696.19779 | 0.0 | 0.0 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0280 | C35 H32 N6 O8 S | 696.20023 | -5.8 | -5.7 |
| 2-1-m1E02-1-0281 | C35 H32 N6 O8 S | 696.20023 | -2.5 | -1.0 |
| 2-1-m1E02-1-0282 | C37 H36 N4 O8 S | 696.22538 | 0.0 | 0.0 |
| 2-1-m1E02-1-0283 | C37 H36 N4 O8 S | 696.22538 | -4.7 | -1.0 |
| 2-1-m1E02-1-0284 | C36 H36 N6 O7 S | 696.23662 | 0.0 | 0.0 |
| 2-1-m1E02-1-0285 | C38 H37 F N4 O6 S | 696.24178 | 0.0 | 0.0 |
| 2-1-m1E02-1-0286 | C36 H35 N5 O8 S | 697.22063 | -7.4 | -6.4 |
| 2-1-m1E02-1-0287 | C37 H36 F N5 O8 | 697.25479 | 7.7 | 0.0 |
| 2-1-m1E02-1-0288 | C37 H39 N5 O7 S | 697.25702 | 0.0 | 0.0 |
| 2-1-m1E02-1-0289 | C36 H34 N4 O9 S | 698.20465 | 0.0 | 0.0 |
| 2-1-m1E02-1-0290 | C35 H34 N6 O8 S | 698.21588 | -3.6 | -4.8 |
| 2-1-m1E02-1-0291 | C37 H35 F N4 O7 S | 698.22105 | -7.2 | -8.2 |
| 2-1-m1E02-1-0292 | C37 H35 F N4 O7 S | 698.22105 | -7.1 | -9.7 |
| 2-1-m1E02-1-0293 | C38 H33 F3 N4 O6 | 698.23522 | -13.3 | -9.8 |
| 2-1-m1E02-1-0294 | C36 H38 N6 O7 S | 698.25227 | 0.0 | 0.0 |
| 2-1-m1E02-1-0295 | C41 H35 F N4 O6 | 698.25406 | -2.8 | -6.4 |
| 2-1-m1E02-1-0296 | C39 H37 F3 N4 O5 | 698.27160 | 2.1 | -3.2 |
| 2-1-m1E02-1-0297 | C39 H40 F2 N4 O6 | 698.29159 | -5.4 | -5.7 |
| 2-1-m1E02-1-0298 | C36 H34 F N5 O7 S | 699.21630 | 1.2 | -2.4 |
| 2-1-m1E02-1-0299 | C36 H34 F N5 O7 S | 699.21630 | 3.4 | 3.8 |
| 2-1-m1E02-1-0300 | C37 H32 F3 N5 O6 | 699.23047 | -4.0 | -6.8 |
| 2-1-m1E02-1-0301 | C36 H37 N5 O8 S | 699.23628 | 0.0 | -1.6 |
| 2-1-m1E02-1-0302 | C36 H37 N5 O8 S | 699.23628 | 0.8 | 0.0 |
| 2-1-m1E02-1-0303 | C36 H37 N5 O8 S | 699.23628 | -3.3 | -4.0 |
| 2-1-m1E02-1-0304 | C38 H42 F N5 O7 | 699.30683 | -5.3 | -4.1 |
| 2-1-m1E02-1-0305 | C35 H32 N4 O8 S2 | 700.16616 | -4.7 | -5.2 |
| 2-1-m1E02-1-0306 | C36 H36 N4 O7 S2 | 700.20254 | -0.6 | -8.2 |
| 2-1-m1E02-1-0307 | C35 H36 N6 O8 S | 700.23153 | 0.8 | -1.2 |
| 2-1-m1E02-1-0308 | C35 H36 N6 O8 S | 700.23153 | -2.6 | -1.8 |
| 2-1-m1E02-1-0309 | C35 H36 N6 O8 S | 700.23153 | -0.9 | -2.2 |
| 2-1-m1E02-1-0310 | C36 H34 F2 N6 O7 | 700.24570 | -1.2 | -2.5 |
| 2-1-m1E02-1-0311 | C38 H35 F3 N4 O6 | 700.25087 | -8.2 | -3.8 |
| 2-1-m1E02-1-0312 | C34 H31 N5 O8 S2 | 701.16140 | -2.4 | -1.5 |
| 2-1-m1E02-1-0313 | C35 H35 N5 O7 S2 | 701.19779 | 0.0 | 0.0 |
| 2-1-m1E02-1-0314 | C34 H35 N7 O8 S | 701.22678 | 2.7 | 0.0 |
| 2-1-m1E02-1-0315 | C34 H35 N7 O8 S | 701.22678 | 0.0 | 0.0 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0316 | C39 H39 N7 O6 | 701.29618 | -7.0 | -8.9 |
| 2-1-m1E02-1-0317 | C35 H31 F N4 O9 S | 702.17958 | -5.0 | 0.0 |
| 2-1-m1E02-1-0318 | C35 H34 N4 O8 S2 | 702.18181 | 0.0 | 0.0 |
| 2-1-m1E02-1-0319 | C36 H32 F2 N4 O7 S | 702.19598 | -0.8 | -6.7 |
| 2-1-m1E02-1-0320 | C36 H32 F2 N4 O7 S | 702.19598 | 7.0 | 3.1 |
| 2-1-m1E02-1-0321 | C34 H33 N5 O8 S2 | 703.17705 | 0.0 | 0.0 |
| 2-1-m1E02-1-0322 | C35 H31 F2 N5 O7 S | 703.19123 | -6.5 | -4.8 |
| 2-1-m1E02-1-0323 | C34 H33 N5 O10 S | 703.19481 | -3.0 | -4.0 |
| 2-1-m1E02-1-0324 | C35 H34 F N5 O8 S | 703.21121 | 0.0 | -0.9 |
| 2-1-m1E02-1-0325 | C35 H34 F N5 O8 S | 703.21121 | 0.0 | -0.5 |
| 2-1-m1E02-1-0326 | C36 H32 F3 N5 O7 | 703.22538 | 0.0 | 0.0 |
| 2-1-m1E02-1-0327 | C35 H37 N5 O9 S | 703.23120 | -5.5 | -6.9 |
| 2-1-m1E02-1-0328 | C37 H36 F3 N5 O6 | 703.26177 | -7.3 | -1.1 |
| 2-1-m1E02-1-0329 | C38 H37 N7 O7 | 703.27545 | 11.4 | 11.4 |
| 2-1-m1E02-1-0330 | C34 H30 F2 N6 O7 S | 704.18647 | -19.8 | -8.9 |
| 2-1-m1E02-1-0331 | C33 H32 N6 O10 S | 704.19006 | 0.3 | 0.1 |
| 2-1-m1E02-1-0332 | C36 H31 F3 N4 O6 S | 704.19164 | 0.3 | 0.1 |
| 2-1-m1E02-1-0333 | C34 H33 F N6 O8 S | 704.20646 | -1.8 | -1.6 |
| 2-1-m1E02-1-0334 | C36 H31 F3 N4 O8 | 704.20940 | 0.0 | 0.0 |
| 2-1-m1E02-1-0335 | C42 H36 N6 O5 | 704.27472 | -2.7 | -3.0 |
| 2-1-m1E02-1-0336 | C34 H35 N5 O8 S2 | 705.19270 | 0.4 | 0.3 |
| 2-1-m1E02-1-0337 | C33 H32 F N7 O8 S | 705.20171 | 0.0 | 0.0 |
| 2-1-m1E02-1-0338 | C34 H35 N5 O10 S | 705.21046 | 3.0 | 0.0 |
| 2-1-m1E02-1-0339 | C36 H34 F3 N5 O7 | 705.24103 | -3.7 | -4.7 |
| 2-1-m1E02-1-0340 | C39 H39 N5 O6 S | 705.26210 | -9.3 | -9.8 |
| 2-1-m1E02-1-0341 | C36 H43 N5 O8 S | 705.28323 | -4.7 | -4.0 |
| 2-1-m1E02-1-0342 | C33 H34 N6 O8 S2 | 706.18795 | -4.3 | -3.6 |
| 2-1-m1E02-1-0343 | C38 H34 N4 O8 S | 706.20973 | 0.0 | 0.0 |
| 2-1-m1E02-1-0344 | C37 H34 N6 O7 S | 706.22097 | -6.7 | -5.5 |
| 2-1-m1E02-1-0345 | C37 H31 F5 N4 O5 | 706.22146 | -5.7 | -7.3 |
| 2-1-m1E02-1-0346 | C35 H33 F3 N6 O7 | 706.23628 | -2.9 | -4.9 |
| 2-1-m1E02-1-0347 | C40 H42 N4 O6 S | 706.28251 | 6.5 | 0.0 |
| 2-1-m1E02-1-0348 | C40 H42 N4 O6 S | 706.28251 | 8.1 | -11.0 |
| 2-1-m1E02-1-0349 | C40 H42 N4 O6 S | 706.28251 | -0.8 | 8.3 |
| 2-1-m1E02-1-0350 | C36 H33 N7 O7 S | 707.21622 | -5.3 | -0.7 |
| 2-1-m1E02-1-0351 | C35 H32 F3 N5 O8 | 707.22030 | -4.5 | -4.7 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0352 | C34 H32 F3 N7 O7 | 707.23153 | 0.0 | 0.0 |
| 2-1-m1E02-1-0353 | C36 H33 F4 N5 O6 | 707.23670 | -5.0 | -5.5 |
| 2-1-m1E02-1-0354 | C38 H37 N5 O7 S | 707.24137 | 3.2 | -4.7 |
| 2-1-m1E02-1-0355 | C35 H41 N5 O9 S | 707.26250 | 0.0 | 0.0 |
| 2-1-m1E02-1-0356 | C39 H41 N5 O6 S | 707.27775 | 0.0 | 0.0 |
| 2-1-m1E02-1-0357 | C34 H30 F2 N4 O7 S2 | 708.15240 | 6.4 | 0.0 |
| 2-1-m1E02-1-0358 | C38 H36 N4 O8 S | 708.22538 | -4.9 | -5.4 |
| 2-1-m1E02-1-0359 | C42 H36 N4 O5 S | 708.24064 | -6.3 | -7.1 |
| 2-1-m1E02-1-0360 | C39 H40 N4 O7 S | 708.26177 | -0.7 | -0.8 |
| 2-1-m1E02-1-0361 | C39 H40 N4 O7 S | 708.26177 | -7.9 | -8.0 |
| 2-1-m1E02-1-0362 | C39 H40 N4 O7 S | 708.26177 | 0.0 | -3.1 |
| 2-1-m1E02-1-0363 | C39 H40 N4 O7 S | 708.26177 | 0.0 | 0.0 |
| 2-1-m1E02-1-0364 | C33 H29 F2 N5 O7 S2 | 709.14765 | -5.9 | -6.0 |
| 2-1-m1E02-1-0365 | C33 H32 F N5 O8 S2 | 709.16763 | -5.2 | -8.6 |
| 2-1-m1E02-1-0366 | C37 H35 N5 O8 S | 709.22063 | -1.9 | -0.2 |
| 2-1-m1E02-1-0367 | C37 H35 N5 O8 S | 709.22063 | -2.1 | -5.1 |
| 2-1-m1E02-1-0368 | C37 H33 F2 N7 O6 | 709.24604 | -6.5 | -9.1 |
| 2-1-m1E02-1-0369 | C38 H39 N5 O7 S | 709.25702 | 0.0 | 0.0 |
| 2-1-m1E02-1-0370 | C40 H47 N5 O7 | 709.34755 | 0.0 | 0.0 |
| 2-1-m1E02-1-0371 | C32 H31 F N6 O8 S2 | 710.16288 | 0.0 | 0.0 |
| 2-1-m1E02-1-0372 | C36 H34 N6 O8 S | 710.21588 | -2.3 | -0.6 |
| 2-1-m1E02-1-0373 | C38 H38 N4 O8 S | 710.24103 | -5.4 | -1.5 |
| 2-1-m1E02-1-0374 | C38 H38 N4 O8 S | 710.24103 | 0.0 | 0.0 |
| 2-1-m1E02-1-0375 | C39 H39 F N4 O6 S | 710.25743 | 0.5 | 0.0 |
| 2-1-m1E02-1-0376 | C33 H31 F2 N5 O9 S | 711.18105 | -2.1 | -2.1 |
| 2-1-m1E02-1-0377 | C37 H37 N5 O8 S | 711.23628 | -1.2 | -2.2 |
| 2-1-m1E02-1-0378 | C37 H37 N5 O8 S | 711.23628 | 0.0 | 0.0 |
| 2-1-m1E02-1-0379 | C38 H41 N5 O7 S | 711.27267 | -8.1 | -2.4 |
| 2-1-m1E02-1-0380 | C38 H41 N5 O7 S | 711.27267 | 0.0 | 0.0 |
| 2-1-m1E02-1-0381 | C39 H45 N5 O8 | 711.32681 | 0.0 | 0.0 |
| 2-1-m1E02-1-0382 | C35 H32 N6 O7 S2 | 712.17739 | -6.6 | -7.4 |
| 2-1-m1E02-1-0383 | C33 H31 F3 N6 O7 S | 712.19270 | 0.0 | 2.7 |
| 2-1-m1E02-1-0384 | C37 H33 F N4 O8 S | 712.20031 | -4.2 | -1.1 |
| 2-1-m1E02-1-0385 | C37 H33 F N4 O8 S | 712.20031 | -4.2 | -1.1 |
| 2-1-m1E02-1-0386 | C37 H36 N4 O9 S | 712.22030 | 0.0 | 0.0 |
| 2-1-m1E02-1-0387 | C38 H37 F N4 O7 S | 712.23670 | -3.5 | -3.7 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0388 | C38 H37 F N4 O7 S | 712.23670 | 3.9 | 0.0 |
| 2-1-m1E02-1-0389 | C37 H40 N6 O7 S | 712.26792 | -3.7 | 0.0 |
| 2-1-m1E02-1-0390 | C43 H44 N4 O6 | 712.32609 | 0.0 | 2.5 |
| 2-1-m1E02-1-0391 | C36 H32 F N5 O8 S | 713.19556 | 0.0 | 0.0 |
| 2-1-m1E02-1-0392 | C36 H32 F N5 O8 S | 713.19556 | 0.0 | 0.0 |
| 2-1-m1E02-1-0393 | C37 H33 F2 N5 O6 S | 713.21196 | 0.2 | 0.0 |
| 2-1-m1E02-1-0394 | C36 H35 N5 O9 S | 713.21555 | 0.0 | -0.8 |
| 2-1-m1E02-1-0395 | C34 H37 F2 N5 O8 S | 713.23309 | -4.5 | -4.8 |
| 2-1-m1E02-1-0396 | C38 H34 F3 N5 O6 | 713.24612 | -4.8 | -1.6 |
| 2-1-m1E02-1-0397 | C37 H39 N5 O8 S | 713.25193 | 1.3 | 0.0 |
| 2-1-m1E02-1-0398 | C37 H39 N5 O8 S | 713.25193 | -2.3 | -3.5 |
| 2-1-m1E02-1-0399 | C37 H39 N5 O8 S | 713.25193 | -3.3 | -3.9 |
| 2-1-m1E02-1-0400 | C37 H39 N5 O8 S | 713.25193 | -6.4 | -5.8 |
| 2-1-m1E02-1-0401 | C36 H34 N4 O8 S2 | 714.18181 | -2.4 | -7.2 |
| 2-1-m1E02-1-0402 | C36 H34 N4 O10 S | 714.19956 | 4.4 | 0.0 |
| 2-1-m1E02-1-0403 | C35 H34 N6 O9 S | 714.21080 | 0.5 | 0.0 |
| 2-1-m1E02-1-0404 | C35 H34 N6 O9 S | 714.21080 | 0.0 | 0.0 |
| 2-1-m1E02-1-0405 | C38 H33 F3 N4 O7 | 714.23013 | -3.2 | -3.7 |
| 2-1-m1E02-1-0406 | C38 H36 F2 N4 O6 S | 714.23236 | -3.9 | -8.2 |
| 2-1-m1E02-1-0407 | C36 H38 N6 O8 S | 714.24718 | -4.8 | -2.9 |
| 2-1-m1E02-1-0408 | C36 H38 N6 O8 S | 714.24718 | -1.7 | -1.2 |
| 2-1-m1E02-1-0409 | C36 H38 N6 O8 S | 714.24718 | -3.5 | -5.8 |
| 2-1-m1E02-1-0410 | C34 H33 N7 O9 S | 715.20605 | -0.1 | -1.2 |
| 2-1-m1E02-1-0411 | C37 H32 F3 N5 O7 | 715.22538 | -2.2 | -5.7 |
| 2-1-m1E02-1-0412 | C36 H37 N5 O9 S | 715.23120 | 0.6 | -0.6 |
| 2-1-m1E02-1-0413 | C36 H37 N5 O9 S | 715.23120 | -12.9 | -0.3 |
| 2-1-m1E02-1-0414 | C35 H37 N7 O8 S | 715.24243 | 0.0 | 0.0 |
| 2-1-m1E02-1-0415 | C37 H38 F N5 O7 S | 715.24760 | 2.5 | -3.9 |
| 2-1-m1E02-1-0416 | C37 H34 F2 N4 O7 S | 716.21163 | -8.4 | -5.9 |
| 2-1-m1E02-1-0417 | C37 H34 F2 N4 O7 S | 716.21163 | 0.7 | -0.2 |
| 2-1-m1E02-1-0418 | C38 H32 F4 N4 O6 | 716.22580 | -4.4 | -5.5 |
| 2-1-m1E02-1-0419 | C35 H36 N6 O9 S | 716.22645 | -0.2 | -0.8 |
| 2-1-m1E02-1-0420 | C40 H36 N4 O7 S | 716.23047 | -0.7 | -1.1 |
| 2-1-m1E02-1-0421 | C40 H36 N4 O7 S | 716.23047 | -5.5 | -3.3 |
| 2-1-m1E02-1-0422 | C36 H33 F2 N5 O7 S | 717.20688 | 0.0 | 0.0 |
| 2-1-m1E02-1-0423 | C35 H35 N5 O10 S | 717.21046 | -0.6 | -1.4 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0424 | C34 H35 N7 O9 S | 717.22170 | -8.0 | -7.4 |
| 2-1-m1E02-1-0425 | C39 H35 N5 O7 S | 717.22572 | -16.9 | -0.4 |
| 2-1-m1E02-1-0426 | C36 H36 F N5 O8 S | 717.22686 | -4.2 | -3.4 |
| 2-1-m1E02-1-0427 | C36 H36 F N5 O8 S | 717.22686 | 2.2 | 0.0 |
| 2-1-m1E02-1-0428 | C37 H34 F3 N5 O7 | 717.24103 | -5.9 | -3.4 |
| 2-1-m1E02-1-0429 | C38 H38 F3 N5 O6 | 717.27742 | -4.0 | -6.1 |
| 2-1-m1E02-1-0430 | C38 H41 F2 N5 O7 | 717.29741 | -3.7 | -4.4 |
| 2-1-m1E02-1-0431 | C35 H31 F N4 O8 S2 | 718.15673 | 0.0 | -2.9 |
| 2-1-m1E02-1-0432 | C38 H34 N6 O7 S | 718.22097 | 0.0 | 0.0 |
| 2-1-m1E02-1-0433 | C38 H34 N6 O7 S | 718.22097 | 0.0 | -7.2 |
| 2-1-m1E02-1-0434 | C35 H35 F N6 O8 S | 718.22211 | 0.0 | -7.2 |
| 2-1-m1E02-1-0435 | C35 H35 F N6 O8 S | 718.22211 | 0.0 | 0.0 |
| 2-1-m1E02-1-0436 | C36 H33 F3 N6 O7 | 718.23628 | -5.2 | -6.4 |
| 2-1-m1E02-1-0437 | C34 H33 N5 O9 S2 | 719.17197 | 0.3 | -0.2 |
| 2-1-m1E02-1-0438 | C35 H37 N5 O8 S2 | 719.20835 | -5.4 | -2.8 |
| 2-1-m1E02-1-0439 | C37 H33 N7 O7 S | 719.21622 | 0.0 | 0.0 |
| 2-1-m1E02-1-0440 | C37 H36 F3 N5 O7 | 719.25668 | -7.9 | -1.2 |
| 2-1-m1E02-1-0441 | C34 H32 N4 O10 S2 | 720.15598 | 9.5 | 0.0 |
| 2-1-m1E02-1-0442 | C33 H32 N6 O9 S2 | 720.16722 | 0.3 | 0.4 |
| 2-1-m1E02-1-0443 | C35 H30 F2 N4 O9 S | 720.17016 | -3.9 | -1.2 |
| 2-1-m1E02-1-0444 | C36 H31 F3 N4 O7 S | 720.18655 | 0.1 | -2.4 |
| 2-1-m1E02-1-0445 | C34 H36 N6 O8 S2 | 720.20360 | 0.0 | 4.0 |
| 2-1-m1E02-1-0446 | C39 H36 N4 O8 S | 720.22538 | 0.0 | 0.0 |
| 2-1-m1E02-1-0447 | C41 H35 F3 N4 O5 | 720.25595 | -4.5 | -11.7 |
| 2-1-m1E02-1-0448 | C41 H44 N4 O6 S | 720.29816 | -3.8 | -5.8 |
| 2-1-m1E02-1-0449 | C41 H44 N4 O6 S | 720.29816 | -3.9 | -5.9 |
| 2-1-m1E02-1-0450 | C33 H31 N5 O10 S2 | 721.15123 | -2.1 | 0.0 |
| 2-1-m1E02-1-0451 | C34 H32 F N5 O10 S | 721.18539 | 0.0 | 0.0 |
| 2-1-m1E02-1-0452 | C34 H35 N5 O9 S2 | 721.18762 | 0.0 | 0.0 |
| 2-1-m1E02-1-0453 | C35 H33 F2 N5 O8 S | 721.20179 | 0.0 | 0.0 |
| 2-1-m1E02-1-0454 | C35 H33 F2 N5 O8 S | 721.20179 | -10.8 | -6.6 |
| 2-1-m1E02-1-0455 | C38 H35 N5 O8 S | 721.22063 | -5.1 | -7.8 |
| 2-1-m1E02-1-0456 | C37 H35 N7 O7 S | 721.23187 | -0.7 | -1.5 |
| 2-1-m1E02-1-0457 | C33 H34 N6 O9 S2 | 722.18287 | 10.4 | 0.0 |
| 2-1-m1E02-1-0458 | C38 H34 N4 O7 S2 | 722.18689 | -2.8 | -8.6 |
| 2-1-m1E02-1-0459 | C38 H34 N4 O7 S2 | 722.18689 | 0.0 | 0.0 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0460 | C38 H34 N4 O7 S2 | 722.18689 | -4.8 | -6.5 |
| 2-1-m1E02-1-0461 | C34 H32 F2 N6 O8 S | 722.19704 | 0.0 | 0.0 |
| 2-1-m1E02-1-0462 | C39 H38 N4 O8 S | 722.24103 | -7.8 | -6.2 |
| 2-1-m1E02-1-0463 | C39 H33 F3 N6 O5 | 722.24645 | 0.0 | 0.0 |
| 2-1-m1E02-1-0464 | C40 H42 N4 O7 S | 722.27742 | 3.4 | -16.7 |
| 2-1-m1E02-1-0465 | C40 H42 N4 O7 S | 722.27742 | 0.0 | 0.0 |
| 2-1-m1E02-1-0466 | C37 H33 N5 O7 S2 | 723.18214 | -5.0 | -5.9 |
| 2-1-m1E02-1-0467 | C37 H33 N5 O7 S2 | 723.18214 | 0.0 | -2.7 |
| 2-1-m1E02-1-0468 | C37 H33 N5 O7 S2 | 723.18214 | 0.0 | 0.0 |
| 2-1-m1E02-1-0469 | C33 H31 F2 N7 O8 S | 723.19229 | 0.0 | -2.1 |
| 2-1-m1E02-1-0470 | C35 H32 F3 N5 O7 S | 723.19745 | -7.3 | -4.4 |
| 2-1-m1E02-1-0471 | C35 H32 F3 N5 O9 | 723.21521 | -3.8 | -4.4 |
| 2-1-m1E02-1-0472 | C38 H37 N5 O8 S | 723.23628 | -4.3 | -4.2 |
| 2-1-m1E02-1-0473 | C38 H37 N5 O8 S | 723.23628 | 18.3 | 0.0 |
| 2-1-m1E02-1-0474 | C35 H31 F3 N4 O8 S | 724.18147 | 2.6 | -4.8 |
| 2-1-m1E02-1-0475 | C37 H33 F N6 O7 S | 724.21155 | -4.9 | -3.8 |
| 2-1-m1E02-1-0476 | C38 H36 N4 O9 S | 724.22030 | 0.0 | 0.0 |
| 2-1-m1E02-1-0477 | C39 H40 N4 O8 S | 724.25668 | -4.4 | -5.5 |
| 2-1-m1E02-1-0478 | C39 H40 N4 O8 S | 724.25668 | 0.0 | 0.0 |
| 2-1-m1E02-1-0479 | C40 H41 F N4 O6 S | 724.27308 | 13.5 | -6.2 |
| 2-1-m1E02-1-0480 | C37 H35 N5 O9 S | 725.21555 | -3.9 | -3.0 |
| 2-1-m1E02-1-0481 | C36 H32 F5 N5 O6 | 725.22727 | -8.2 | -1.7 |
| 2-1-m1E02-1-0482 | C39 H43 N5 O7 S | 725.28832 | 0.0 | 0.0 |
| 2-1-m1E02-1-0483 | C39 H43 N5 O7 S | 725.28832 | 0.0 | -5.6 |
| 2-1-m1E02-1-0484 | C39 H43 N5 O7 S | 725.28832 | 0.0 | 0.0 |
| 2-1-m1E02-1-0485 | C39 H33 F3 N4 O5 S | 726.21238 | 0.0 | 0.0 |
| 2-1-m1E02-1-0486 | C38 H35 F N4 O8 S | 726.21596 | -6.0 | -5.1 |
| 2-1-m1E02-1-0487 | C36 H37 F3 N4 O7 S | 726.23350 | -7.4 | 0.0 |
| 2-1-m1E02-1-0488 | C39 H39 F N4 O7 S | 726.25235 | 0.0 | 0.0 |
| 2-1-m1E02-1-0489 | C39 H42 N4 O8 S | 726.27234 | 0.0 | 0.0 |
| 2-1-m1E02-1-0490 | C38 H42 N6 O7 S | 726.28357 | 0.0 | 0.0 |
| 2-1-m1E02-1-0491 | C33 H31 F2 N5 O8 S2 | 727.15821 | -7.5 | -5.5 |
| 2-1-m1E02-1-0492 | C37 H34 F N5 O8 S | 727.21121 | -4.4 | -4.2 |
| 2-1-m1E02-1-0493 | C37 H37 N5 O9 S | 727.23120 | -0.8 | 0.0 |
| 2-1-m1E02-1-0494 | C38 H41 N5 O8 S | 727.26758 | 0.0 | 0.0 |
| 2-1-m1E02-1-0495 | C38 H41 N5 O8 S | 727.26758 | -2.8 | -3.6 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0496 | C38 H41 N5 O8 S | 727.26758 | -8.1 | 0.7 |
| 2-1-m1E02-1-0497 | C38 H41 N5 O8 S | 727.26758 | -3.5 | -5.1 |
| 2-1-m1E02-1-0498 | C32 H30 F2 N6 O8 S2 | 728.15346 | 23.6 | -2.4 |
| 2-1-m1E02-1-0499 | C37 H36 N4 O8 S2 | 728.19746 | -5.6 | -5.1 |
| 2-1-m1E02-1-0500 | C36 H36 N6 O9 S | 728.22645 | -3.4 | -1.7 |
| 2-1-m1E02-1-0501 | C36 H36 N6 O9 S | 728.22645 | 0.7 | 0.0 |
| 2-1-m1E02-1-0502 | C38 H37 F N4 O8 S | 728.23161 | -3.8 | -1.2 |
| 2-1-m1E02-1-0503 | C38 H37 F N4 O8 S | 728.23161 | -0.5 | 0.0 |
| 2-1-m1E02-1-0504 | C39 H38 F2 N4 O6 S | 728.24801 | 7.4 | 0.0 |
| 2-1-m1E02-1-0505 | C37 H40 N6 O8 S | 728.26283 | -2.2 | -4.7 |
| 2-1-m1E02-1-0506 | C42 H40 N4 O6 S | 728.26686 | -23.8 | 8.6 |
| 2-1-m1E02-1-0507 | C35 H35 N7 O9 S | 729.22170 | -0.4 | 1.4 |
| 2-1-m1E02-1-0508 | C38 H34 F3 N5 O7 | 729.24103 | -8.2 | -3.1 |
| 2-1-m1E02-1-0509 | C37 H39 N5 O9 S | 729.24685 | 0.0 | 0.0 |
| 2-1-m1E02-1-0510 | C37 H39 N5 O9 S | 729.24685 | 0.0 | 0.0 |
| 2-1-m1E02-1-0511 | C38 H40 F N5 O7 S | 729.26325 | 0.3 | -4.3 |
| 2-1-m1E02-1-0512 | C36 H34 N4 O9 S2 | 730.17672 | -3.6 | -5.0 |
| 2-1-m1E02-1-0513 | C36 H34 N4 O9 S2 | 730.17672 | -5.5 | 0.0 |
| 2-1-m1E02-1-0514 | C37 H32 F2 N4 O8 S | 730.19089 | -8.5 | -3.0 |
| 2-1-m1E02-1-0515 | C37 H32 F2 N4 O8 S | 730.19089 | 2.6 | -1.9 |
| 2-1-m1E02-1-0516 | C38 H36 F2 N4 O7 S | 730.22728 | 1.8 | 3.1 |
| 2-1-m1E02-1-0517 | C38 H34 N8 O6 S | 730.23220 | 0.0 | 0.0 |
| 2-1-m1E02-1-0518 | C36 H38 N6 O9 S | 730.24210 | -3.8 | -52.7 |
| 2-1-m1E02-1-0519 | C36 H38 N6 O9 S | 730.24210 | 0.0 | 0.0 |
| 2-1-m1E02-1-0520 | C41 H38 N4 O7 S | 730.24612 | -6.7 | -4.6 |
| 2-1-m1E02-1-0521 | C40 H41 F3 N4 O6 | 730.29782 | 0.0 | -10.0 |
| 2-1-m1E02-1-0522 | C35 H33 N5 O9 S2 | 731.17197 | -5.1 | -3.4 |
| 2-1-m1E02-1-0523 | C36 H31 F2 N5 O8 S | 731.18614 | 0.0 | 0.0 |
| 2-1-m1E02-1-0524 | C36 H34 F N5 O9 S | 731.20613 | 0.0 | 0.0 |
| 2-1-m1E02-1-0525 | C36 H34 F N5 O9 S | 731.20613 | 1.5 | 1.4 |
| 2-1-m1E02-1-0526 | C36 H37 N5 O10 S | 731.22611 | 1.1 | -0.5 |
| 2-1-m1E02-1-0527 | C40 H37 N5 O7 S | 731.24137 | -3.2 | -4.1 |
| 2-1-m1E02-1-0528 | C37 H38 F N5 O8 S | 731.24251 | -3.1 | -3.7 |
| 2-1-m1E02-1-0529 | C37 H38 F N5 O8 S | 731.24251 | -8.1 | 0.0 |
| 2-1-m1E02-1-0530 | C34 H32 N6 O9 S2 | 732.16722 | -4.4 | -6.1 |
| 2-1-m1E02-1-0531 | C35 H33 F N6 O9 S | 732.20138 | 0.1 | 0.3 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0532 | C35 H33 F N6 O9 S | 732.20138 | 0.0 | 0.0 |
| 2-1-m1E02-1-0533 | C38 H32 F4 N4 O7 | 732.22071 | -8.4 | 3.5 |
| 2-1-m1E02-1-0534 | C39 H36 N6 O7 S | 732.23662 | -10.2 | -7.7 |
| 2-1-m1E02-1-0535 | C37 H35 F3 N6 O7 | 732.25193 | 0.0 | 0.0 |
| 2-1-m1E02-1-0536 | C35 H35 N5 O9 S2 | 733.18762 | 0.0 | 0.0 |
| 2-1-m1E02-1-0537 | C35 H35 N5 O11 S | 733.20538 | 0.1 | 0.9 |
| 2-1-m1E02-1-0538 | C37 H34 F3 N5 O8 | 733.23595 | -5.9 | -5.0 |
| 2-1-m1E02-1-0539 | C37 H37 F2 N5 O7 S | 733.23818 | -6.0 | -5.0 |
| 2-1-m1E02-1-0540 | C35 H34 N4 O10 S2 | 734.17163 | 0.0 | -6.7 |
| 2-1-m1E02-1-0541 | C38 H34 N6 O6 S2 | 734.19812 | 0.0 | 0.0 |
| 2-1-m1E02-1-0542 | C37 H33 F3 N4 O7 S | 734.20220 | -8.9 | -5.1 |
| 2-1-m1E02-1-0543 | C37 H33 F3 N4 O7 S | 734.20220 | -7.2 | -3.2 |
| 2-1-m1E02-1-0544 | C37 H33 F3 N4 O7 S | 734.20220 | 0.0 | 0.0 |
| 2-1-m1E02-1-0545 | C37 H33 F3 N4 O7 S | 734.20220 | 0.0 | 0.0 |
| 2-1-m1E02-1-0546 | C39 H34 N4 O9 S | 734.20465 | -9.1 | -5.1 |
| 2-1-m1E02-1-0547 | C35 H38 N6 O8 S2 | 734.21925 | -1.0 | -2.5 |
| 2-1-m1E02-1-0548 | C36 H33 F3 N6 O8 | 734.23120 | 0.7 | -5.1 |
| 2-1-m1E02-1-0549 | C40 H38 N4 O8 S | 734.24103 | -3.7 | -4.6 |
| 2-1-m1E02-1-0550 | C41 H42 N4 O7 S | 734.27742 | -1.0 | -6.0 |
| 2-1-m1E02-1-0551 | C42 H46 N4 O6 S | 734.31381 | 6.1 | -6.6 |
| 2-1-m1E02-1-0552 | C36 H32 F3 N5 O7 S | 735.19745 | -2.5 | -1.9 |
| 2-1-m1E02-1-0553 | C36 H32 F3 N5 O7 S | 735.19745 | -3.8 | -4.7 |
| 2-1-m1E02-1-0554 | C36 H35 F2 N5 O8 S | 735.21744 | 0.0 | 0.0 |
| 2-1-m1E02-1-0555 | C36 H35 F2 N5 O8 S | 735.21744 | 0.0 | 0.0 |
| 2-1-m1E02-1-0556 | C37 H33 F4 N5 O7 | 735.23161 | -5.8 | -4.9 |
| 2-1-m1E02-1-0557 | C40 H41 N5 O7 S | 735.27267 | -5.8 | -7.0 |
| 2-1-m1E02-1-0558 | C34 H32 N4 O9 S3 | 736.13314 | -9.4 | -1.6 |
| 2-1-m1E02-1-0559 | C35 H30 F2 N4 O8 S2 | 736.14731 | -4.1 | -5.7 |
| 2-1-m1E02-1-0560 | C35 H31 F3 N6 O7 S | 736.19270 | -5.8 | 0.0 |
| 2-1-m1E02-1-0561 | C39 H36 N4 O7 S2 | 736.20254 | 0.0 | 0.0 |
| 2-1-m1E02-1-0562 | C39 H36 N4 O7 S2 | 736.20254 | -5.1 | -7.2 |
| 2-1-m1E02-1-0563 | C38 H33 F N6 O7 S | 736.21155 | 0.0 | -0.4 |
| 2-1-m1E02-1-0564 | C35 H34 F2 N6 O8 S | 736.21269 | -11.8 | -0.2 |
| 2-1-m1E02-1-0565 | C41 H44 N4 O7 S | 736.29307 | 8.6 | 0.0 |
| 2-1-m1E02-1-0566 | C33 H31 N5 O9 S3 | 737.12839 | -6.2 | -5.5 |
| 2-1-m1E02-1-0567 | C34 H32 F N5 O9 S2 | 737.16255 | -0.4 | -0.5 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0568 | C38 H35 N5 O7 S2 | 737.19779 | 3.5 | -8.5 |
| 2-1-m1E02-1-0569 | C37 H35 N7 O8 S | 737.22678 | 63.9 | -0.4 |
| 2-1-m1E02-1-0570 | C39 H39 N5 O8 S | 737.25193 | -3.4 | -3.9 |
| 2-1-m1E02-1-0571 | C34 H31 F N4 O10 S2 | 738.14656 | 0.0 | 0.0 |
| 2-1-m1E02-1-0572 | C37 H34 N6 O7 S2 | 738.19304 | 1.2 | -5.8 |
| 2-1-m1E02-1-0573 | C39 H35 F N4 O8 S | 738.21596 | -6.7 | -6.6 |
| 2-1-m1E02-1-0574 | C36 H34 N8 O8 S | 738.22203 | 0.0 | 0.0 |
| 2-1-m1E02-1-0575 | C38 H32 F6 N4 O5 | 738.22769 | -5.9 | 4.7 |
| 2-1-m1E02-1-0576 | C39 H33 F3 N6 O6 | 738.24137 | -8.6 | -10.2 |
| 2-1-m1E02-1-0577 | C40 H42 N4 O8 S | 738.27234 | 0.0 | 0.0 |
| 2-1-m1E02-1-0578 | C39 H42 N6 O7 S | 738.28357 | -6.9 | -6.6 |
| 2-1-m1E02-1-0579 | C33 H33 N5 O11 S2 | 739.16180 | 0.0 | 0.0 |
| 2-1-m1E02-1-0580 | C34 H31 F2 N5 O10 S | 739.17597 | 1.2 | 0.0 |
| 2-1-m1E02-1-0581 | C35 H32 F3 N5 O8 S | 739.19237 | -2.2 | -10.9 |
| 2-1-m1E02-1-0582 | C38 H37 N5 O9 S | 739.23120 | -2.4 | -2.0 |
| 2-1-m1E02-1-0583 | C38 H37 N5 O9 S | 739.23120 | -7.3 | -5.7 |
| 2-1-m1E02-1-0584 | C40 H45 N5 O7 S | 739.30397 | 0.0 | 0.0 |
| 2-1-m1E02-1-0585 | C40 H45 N5 O7 S | 739.30397 | 0.0 | -19.5 |
| 2-1-m1E02-1-0586 | C32 H32 N6 O11 S2 | 740.15705 | 0.5 | -0.3 |
| 2-1-m1E02-1-0587 | C35 H31 F3 N4 O7 S2 | 740.15863 | -0.7 | -10.2 |
| 2-1-m1E02-1-0588 | C35 H31 F3 N4 O9 S | 740.17638 | -7.6 | -3.0 |
| 2-1-m1E02-1-0589 | C38 H33 F N4 O7 S2 | 740.17747 | 0.0 | 0.0 |
| 2-1-m1E02-1-0590 | C38 H33 F N4 O7 S2 | 740.17747 | -8.7 | -6.4 |
| 2-1-m1E02-1-0591 | C37 H36 N6 O9 S | 740.22645 | -5.5 | -8.4 |
| 2-1-m1E02-1-0592 | C39 H37 F N4 O8 S | 740.23161 | 0.0 | 0.0 |
| 2-1-m1E02-1-0593 | C39 H40 N4 O9 S | 740.25160 | -12.0 | -23.7 |
| 2-1-m1E02-1-0594 | C40 H44 N4 O8 S | 740.28799 | 0.0 | 0.0 |
| 2-1-m1E02-1-0595 | C34 H30 F3 N5 O7 S2 | 741.15387 | -2.9 | -2.3 |
| 2-1-m1E02-1-0596 | C37 H35 N5 O8 S2 | 741.19270 | 0.0 | 0.0 |
| 2-1-m1E02-1-0597 | C37 H35 N5 O8 S2 | 741.19270 | -8.0 | -7.9 |
| 2-1-m1E02-1-0598 | C38 H39 N5 O9 S | 741.24685 | -0.3 | -0.4 |
| 2-1-m1E02-1-0599 | C38 H34 F3 N7 O6 | 741.25227 | -3.0 | -2.9 |
| 2-1-m1E02-1-0600 | C39 H43 N5 O8 S | 741.28323 | 0.4 | 1.2 |
| 2-1-m1E02-1-0601 | C39 H43 N5 O8 S | 741.28323 | 0.0 | 0.0 |
| 2-1-m1E02-1-0602 | C36 H34 N6 O8 S2 | 742.18795 | -6.6 | -4.6 |
| 2-1-m1E02-1-0603 | C36 H34 N6 O8 S2 | 742.18795 | -13.9 | -8.1 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0604 | C36 H34 N6 O8 S2 | 742.18795 | -5.4 | -10.4 |
| 2-1-m1E02-1-0605 | C36 H34 N6 O8 S2 | 742.18795 | 0.0 | 0.0 |
| 2-1-m1E02-1-0606 | C39 H33 F3 N4 O6 S | 742.20729 | 2.7 | -13.2 |
| 2-1-m1E02-1-0607 | C38 H35 F N4 O9 S | 742.21088 | 0.0 | -3.5 |
| 2-1-m1E02-1-0608 | C36 H37 F3 N4 O8 S | 742.22842 | 0.0 | 0.0 |
| 2-1-m1E02-1-0609 | C37 H38 N6 O9 S | 742.24210 | 0.7 | 0.9 |
| 2-1-m1E02-1-0610 | C37 H38 N6 O9 S | 742.24210 | -1.4 | -0.6 |
| 2-1-m1E02-1-0611 | C40 H40 F2 N4 O6 S | 742.26366 | 0.0 | -10.8 |
| 2-1-m1E02-1-0612 | C43 H42 N4 O6 S | 742.28251 | -28.2 | -23.7 |
| 2-1-m1E02-1-0613 | C35 H33 N7 O8 S2 | 743.18320 | -2.0 | 0.0 |
| 2-1-m1E02-1-0614 | C34 H32 F3 N5 O9 S | 743.18728 | 0.0 | 0.5 |
| 2-1-m1E02-1-0615 | C37 H37 N5 O10 S | 743.22611 | 0.0 | 0.0 |
| 2-1-m1E02-1-0616 | C38 H41 N5 O9 S | 743.26250 | 0.0 | -1.5 |
| 2-1-m1E02-1-0617 | C38 H41 N5 O9 S | 743.26250 | 6.2 | -12.7 |
| 2-1-m1E02-1-0618 | C39 H42 F N5 O7 S | 743.27890 | 11.3 | 0.0 |
| 2-1-m1E02-1-0619 | C34 H32 N8 O8 S2 | 744.17845 | 0.0 | -1.4 |
| 2-1-m1E02-1-0620 | C37 H36 N4 O9 S2 | 744.19237 | -3.0 | -3.7 |
| 2-1-m1E02-1-0621 | C36 H36 N6 O10 S | 744.22136 | -3.0 | -5.1 |
| 2-1-m1E02-1-0622 | C41 H36 N4 O8 S | 744.22538 | -2.8 | -5.4 |
| 2-1-m1E02-1-0623 | C42 H40 N4 O7 S | 744.26177 | -5.6 | -5.5 |
| 2-1-m1E02-1-0624 | C39 H41 F N4 O8 S | 744.26291 | 0.0 | 0.0 |
| 2-1-m1E02-1-0625 | C37 H33 F2 N5 O8 S | 745.20179 | 0.0 | -1.3 |
| 2-1-m1E02-1-0626 | C38 H34 F3 N5 O6 S | 745.21819 | 3.7 | 0.0 |
| 2-1-m1E02-1-0627 | C40 H35 N5 O8 S | 745.22063 | -1.7 | -1.1 |
| 2-1-m1E02-1-0628 | C37 H36 F N5 O9 S | 745.22178 | -2.2 | -0.2 |
| 2-1-m1E02-1-0629 | C35 H38 F3 N5 O8 S | 745.23932 | -2.1 | 0.0 |
| 2-1-m1E02-1-0630 | C38 H40 F N5 O8 S | 745.25816 | 2.4 | 2.7 |
| 2-1-m1E02-1-0631 | C38 H43 N5 O9 S | 745.27815 | 2.2 | 0.0 |
| 2-1-m1E02-1-0632 | C37 H33 F3 N6 O6 S | 746.21344 | -1.9 | -5.2 |
| 2-1-m1E02-1-0633 | C36 H35 F N6 O9 S | 746.21703 | -0.1 | -1.8 |
| 2-1-m1E02-1-0634 | C38 H36 F2 N4 O8 S | 746.22219 | 0.0 | 0.0 |
| 2-1-m1E02-1-0635 | C39 H37 F3 N4 O6 S | 746.23859 | 0.0 | 0.0 |
| 2-1-m1E02-1-0636 | C40 H38 N6 O7 S | 746.25227 | -6.8 | -5.0 |
| 2-1-m1E02-1-0637 | C37 H42 N6 O9 S | 746.27340 | 0.8 | -0.6 |
| 2-1-m1E02-1-0638 | C40 H41 F3 N4 O7 | 746.29273 | -13.5 | -6.7 |
| 2-1-m1E02-1-0639 | C36 H37 N5 O9 S2 | 747.20327 | 1.8 | 0.0 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0640 | C37 H38 F N5 O9 S | 747.23743 | 0.0 | 0.0 |
| 2-1-m1E02-1-0641 | C37 H38 F N5 O9 S | 747.23743 | 0.0 | 0.0 |
| 2-1-m1E02-1-0642 | C38 H39 F2 N5 O7 S | 747.25383 | 0.0 | 0.0 |
| 2-1-m1E02-1-0643 | C34 H32 N6 O8 S3 | 748.14437 | -3.3 | -4.2 |
| 2-1-m1E02-1-0644 | C36 H33 F N4 O9 S2 | 748.16730 | -4.6 | -4.9 |
| 2-1-m1E02-1-0645 | C37 H31 F3 N4 O8 S | 748.18147 | -4.5 | -5.7 |
| 2-1-m1E02-1-0646 | C36 H36 N4 O10 S2 | 748.18728 | 0.0 | -7.5 |
| 2-1-m1E02-1-0647 | C38 H35 F3 N4 O7 S | 748.21785 | -2.4 | -7.3 |
| 2-1-m1E02-1-0648 | C38 H35 F3 N4 O7 S | 748.21785 | -11.7 | -6.2 |
| 2-1-m1E02-1-0649 | C39 H36 N6 O8 S | 748.23153 | -5.7 | -2.5 |
| 2-1-m1E02-1-0650 | C41 H37 F N4 O7 S | 748.23670 | -3.7 | 0.0 |
| 2-1-m1E02-1-0651 | C37 H35 F3 N6 O8 | 748.24685 | -3.7 | -3.6 |
| 2-1-m1E02-1-0652 | C41 H40 N4 O8 S | 748.25668 | 0.0 | -4.5 |
| 2-1-m1E02-1-0653 | C42 H44 N4 O7 S | 748.29307 | 25.7 | -9.2 |
| 2-1-m1E02-1-0654 | C33 H31 N7 O8 S3 | 749.13962 | -6.8 | -3.4 |
| 2-1-m1E02-1-0655 | C35 H35 N5 O10 S2 | 749.18253 | 3.4 | 0.0 |
| 2-1-m1E02-1-0656 | C35 H35 N5 O10 S2 | 749.18253 | 5.9 | 0.0 |
| 2-1-m1E02-1-0657 | C36 H33 F2 N5 O9 S | 749.19670 | 0.0 | 0.6 |
| 2-1-m1E02-1-0658 | C36 H33 F2 N5 O9 S | 749.19670 | -0.7 | -3.6 |
| 2-1-m1E02-1-0659 | C37 H34 F3 N5 O7 S | 749.21310 | -6.1 | -2.2 |
| 2-1-m1E02-1-0660 | C37 H37 F2 N5 O8 S | 749.23309 | 0.0 | 0.0 |
| 2-1-m1E02-1-0661 | C39 H42 F3 N5 O7 | 749.30363 | 0.0 | 0.0 |
| 2-1-m1E02-1-0662 | C35 H34 N4 O9 S3 | 750.14879 | -4.2 | -1.6 |
| 2-1-m1E02-1-0663 | C35 H34 N4 O11 S2 | 750.16655 | 0.0 | 0.0 |
| 2-1-m1E02-1-0664 | C34 H34 N6 O10 S2 | 750.17778 | 0.0 | 0.0 |
| 2-1-m1E02-1-0665 | C39 H34 N4 O8 S2 | 750.18181 | -0.5 | -1.1 |
| 2-1-m1E02-1-0666 | C35 H32 F2 N6 O9 S | 750.19195 | 0.0 | 0.0 |
| 2-1-m1E02-1-0667 | C37 H33 F3 N4 O8 S | 750.19712 | -6.9 | -5.7 |
| 2-1-m1E02-1-0668 | C37 H33 F3 N4 O8 S | 750.19712 | -9.2 | -10.1 |
| 2-1-m1E02-1-0669 | C40 H38 N4 O7 S2 | 750.21819 | -12.7 | -15.1 |
| 2-1-m1E02-1-0670 | C40 H38 N4 O7 S2 | 750.21819 | 0.0 | 0.0 |
| 2-1-m1E02-1-0671 | C40 H38 N4 O9 S | 750.23595 | -6.5 | -4.1 |
| 2-1-m1E02-1-0672 | C36 H32 F3 N5 O8 S | 751.19237 | -10.6 | -3.3 |
| 2-1-m1E02-1-0673 | C39 H37 N5 O7 S2 | 751.21344 | -7.6 | -7.5 |
| 2-1-m1E02-1-0674 | C37 H33 F4 N5 O8 | 751.22653 | -4.0 | -3.8 |
| 2-1-m1E02-1-0675 | C38 H37 N7 O8 S | 751.24243 | -2.8 | -4.1 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0676 | C36 H31 F3 N4 O9 S | 752.17638 | 0.0 | 0.0 |
| 2-1-m1E02-1-0677 | C35 H31 F3 N6 O8 S | 752.18762 | -15.5 | 0.0 |
| 2-1-m1E02-1-0678 | C37 H32 F4 N4 O7 S | 752.19278 | -6.7 | -0.2 |
| 2-1-m1E02-1-0679 | C39 H36 N4 O8 S2 | 752.19746 | -10.5 | 3.8 |
| 2-1-m1E02-1-0680 | C39 H36 N4 O8 S2 | 752.19746 | -2.5 | -4.2 |
| 2-1-m1E02-1-0681 | C41 H41 F N4 O7 S | 752.26800 | -3.1 | -5.2 |
| 2-1-m1E02-1-0682 | C34 H35 N5 O11 S2 | 753.17745 | 0.0 | 0.0 |
| 2-1-m1E02-1-0683 | C36 H34 F3 N5 O8 S | 753.20802 | -1.0 | 0.0 |
| 2-1-m1E02-1-0684 | C36 H34 F3 N5 O8 S | 753.20802 | -4.5 | -2.8 |
| 2-1-m1E02-1-0685 | C36 H34 F3 N5 O8 S | 753.20802 | -5.2 | -1.0 |
| 2-1-m1E02-1-0686 | C36 H34 F3 N5 O8 S | 753.20802 | -4.6 | -2.6 |
| 2-1-m1E02-1-0687 | C39 H39 N5 O9 S | 753.24685 | -4.2 | -4.0 |
| 2-1-m1E02-1-0688 | C40 H43 N5 O8 S | 753.28323 | -4.3 | -6.8 |
| 2-1-m1E02-1-0689 | C41 H47 N5 O7 S | 753.31962 | 0.0 | 0.0 |
| 2-1-m1E02-1-0690 | C34 H31 F N4 O9 S3 | 754.12372 | -8.8 | -7.8 |
| 2-1-m1E02-1-0691 | C38 H32 F2 N6 O7 S | 754.20212 | -8.8 | -2.0 |
| 2-1-m1E02-1-0692 | C35 H33 F3 N6 O8 S | 754.20327 | 0.0 | -1.2 |
| 2-1-m1E02-1-0693 | C35 H33 F3 N6 O8 S | 754.20327 | 9.0 | 0.0 |
| 2-1-m1E02-1-0694 | C38 H32 F6 N4 O6 | 754.22260 | 5.2 | -10.1 |
| 2-1-m1E02-1-0695 | C38 H38 N6 O7 S2 | 754.22434 | 5.2 | -12.0 |
| 2-1-m1E02-1-0696 | C39 H42 N6 O8 S | 754.27848 | -7.7 | -4.7 |
| 2-1-m1E02-1-0697 | C41 H46 N4 O8 S | 754.30364 | 0.0 | 0.0 |
| 2-1-m1E02-1-0698 | C33 H33 N5 O10 S3 | 755.13895 | -2.9 | -4.2 |
| 2-1-m1E02-1-0699 | C34 H31 F2 N5 O9 S2 | 755.15313 | -1.2 | -1.2 |
| 2-1-m1E02-1-0700 | C38 H34 F N5 O7 S2 | 755.18837 | -5.3 | -5.8 |
| 2-1-m1E02-1-0701 | C34 H32 F3 N7 O8 S | 755.19852 | 0.0 | 0.0 |
| 2-1-m1E02-1-0702 | C38 H37 N5 O8 S2 | 755.20835 | -2.4 | -1.8 |
| 2-1-m1E02-1-0703 | C38 H37 N5 O8 S2 | 755.20835 | 0.0 | 0.0 |
| 2-1-m1E02-1-0704 | C37 H34 F N7 O8 S | 755.21736 | -1.7 | -3.2 |
| 2-1-m1E02-1-0705 | C40 H45 N5 O8 S | 755.29888 | 0.0 | 0.0 |
| 2-1-m1E02-1-0706 | C32 H32 N6 O10 S3 | 756.13420 | -1.7 | -1.2 |
| 2-1-m1E02-1-0707 | C34 H30 F2 N4 O10 S2 | 756.13714 | -1.0 | -1.5 |
| 2-1-m1E02-1-0708 | C35 H31 F3 N4 O8 S2 | 756.15354 | -2.8 | -10.7 |
| 2-1-m1E02-1-0709 | C37 H36 N6 O8 S2 | 756.20360 | -7.3 | -4.5 |
| 2-1-m1E02-1-0710 | C37 H36 N6 O8 S2 | 756.20360 | -6.2 | -4.9 |
| 2-1-m1E02-1-0711 | C39 H34 F2 N4 O8 S | 756.20654 | -7.3 | -4.5 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0712 | C38 H40 N6 O9 S | 756.25775 | -2.8 | -1.9 |
| 2-1-m1E02-1-0713 | C40 H44 N4 O9 S | 756.28290 | -1.6 | 1.3 |
| 2-1-m1E02-1-0714 | C44 H44 N4 O6 S | 756.29816 | -3.1 | -2.4 |
| 2-1-m1E02-1-0715 | C34 H30 F3 N5 O8 S2 | 757.14879 | 0.0 | -3.7 |
| 2-1-m1E02-1-0716 | C33 H32 F N5 Oil S2 | 757.15238 | 0.0 | 0.0 |
| 2-1-m1E02-1-0717 | C36 H35 N7 O8 S2 | 757.19885 | 1.1 | -5.3 |
| 2-1-m1E02-1-0718 | C36 H35 N7 O8 S2 | 757.19885 | 0.0 | 0.0 |
| 2-1-m1E02-1-0719 | C38 H36 F N5 O9 S | 757.22178 | -7.5 | -6.5 |
| 2-1-m1E02-1-0720 | C37 H33 F6 N5 O6 | 757.23350 | -7.2 | -1.4 |
| 2-1-m1E02-1-0721 | C38 H34 F3 N7 O7 | 757.24718 | -2.7 | -4.3 |
| 2-1-m1E02-1-0722 | C39 H43 N5 O9 S | 757.27815 | 0.0 | 0.0 |
| 2-1-m1E02-1-0723 | C38 H32 F2 N4 O7 S2 | 758.16805 | -5.5 | -8.3 |
| 2-1-m1E02-1-0724 | C38 H32 F2 N4 O7 S2 | 758.16805 | 0.0 | 0.0 |
| 2-1-m1E02-1-0725 | C38 H38 N4 O9 S2 | 758.20802 | 0.0 | -2.1 |
| 2-1-m1E02-1-0726 | C37 H38 N6 O10 S | 758.23701 | 1.2 | -5.6 |
| 2-1-m1E02-1-0727 | C34 H32 F3 N5 O8 S2 | 759.16444 | -1.0 | 4.5 |
| 2-1-m1E02-1-0728 | C34 H32 F3 N5 O10 S | 759.18220 | -5.0 | -4.0 |
| 2-1-m1E02-1-0729 | C37 H34 F N5 O8 S2 | 759.18328 | -7.6 | -6.3 |
| 2-1-m1E02-1-0730 | C37 H34 F N5 O8 S2 | 759.18328 | 0.0 | 0.0 |
| 2-1-m1E02-1-0731 | C41 H37 N5 O8 S | 759.23628 | -0.8 | -1.7 |
| 2-1-m1E02-1-0732 | C38 H38 F N5 O9 S | 759.23743 | -6.5 | -5.9 |
| 2-1-m1E02-1-0733 | C38 H41 N5 O10 S | 759.25741 | 0.0 | 0.0 |
| 2-1-m1E02-1-0734 | C39 H45 N5 O9 S | 759.29380 | -0.1 | -1.4 |
| 2-1-m1E02-1-0735 | C33 H31 F3 N6 O8 S2 | 760.15969 | 0.0 | 0.0 |
| 2-1-m1E02-1-0736 | C35 H32 N6 O10 S2 | 760.16213 | -2.9 | -2.2 |
| 2-1-m1E02-1-0737 | C37 H36 N4 O10 S2 | 760.18728 | -8.0 | -8.4 |
| 2-1-m1E02-1-0738 | C40 H39 F3 N4 O6 S | 760.25424 | 0.0 | -7.6 |
| 2-1-m1E02-1-0739 | C42 H40 N4 O8 S | 760.25668 | 0.0 | -2.9 |
| 2-1-m1E02-1-0740 | C38 H34 F3 N5 O7 S | 761.21310 | -1.7 | 1.5 |
| 2-1-m1E02-1-0741 | C37 H36 F N5 O10 S | 761.21669 | 0.0 | 0.0 |
| 2-1-m1E02-1-0742 | C35 H38 F3 N5 O9 S | 761.23423 | 0.0 | -3.2 |
| 2-1-m1E02-1-0743 | C39 H41 F2 N5 O7 S | 761.26948 | 0.0 | 0.0 |
| 2-1-m1E02-1-0744 | C38 H33 F3 N4 O8 S | 762.19712 | 0.0 | 0.0 |
| 2-1-m1E02-1-0745 | C41 H35 F N4 O8 S | 762.21596 | -6.1 | -5.5 |
| 2-1-m1E02-1-0746 | C39 H37 F3 N4 O7 S | 762.23350 | -8.2 | -4.0 |
| 2-1-m1E02-1-0747 | C39 H37 F3 N4 O7 S | 762.23350 | 0.0 | 0.0 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0748 | C39 H37 F3 N4 O7 S | 762.23350 | 0.0 | 0.0 |
| 2-1-m1E02-1-0749 | C39 H40 F2 N4 O8 S | 762.25349 | -3.7 | -2.4 |
| 2-1-m1E02-1-0750 | C43 H46 N4 O7 S | 762.30872 | 3.5 | -16.9 |
| 2-1-m1E02-1-0751 | C37 H32 F3 N5 O8 S | 763.19237 | -3.3 | -4.7 |
| 2-1-m1E02-1-0752 | C36 H37 N5 O10 S2 | 763.19818 | -1.0 | -4.7 |
| 2-1-m1E02-1-0753 | C38 H42 F N5 O9 S | 763.26873 | 0.0 | 0.0 |
| 2-1-m1E02-1-0754 | C36 H36 N4 O9 S3 | 764.16444 | 0.0 | -6.2 |
| 2-1-m1E02-1-0755 | C36 H34 F2 N6 O9 S | 764.20760 | 0.0 | 0.0 |
| 2-1-m1E02-1-0756 | C38 H35 F3 N4 O8 S | 764.21277 | -5.2 | -5.3 |
| 2-1-m1E02-1-0757 | C38 H35 F3 N4 O8 S | 764.21277 | 0.0 | -3.3 |
| 2-1-m1E02-1-0758 | C42 H44 N4 O8 S | 764.28799 | -12.6 | -7.6 |
| 2-1-m1E02-1-0759 | C37 H34 F3 N5 O8 S | 765.20802 | 0.0 | 0.0 |
| 2-1-m1E02-1-0760 | C37 H37 F2 N5 O9 S | 765.22800 | 0.0 | 4.1 |
| 2-1-m1E02-1-0761 | C40 H39 N5 O7 S2 | 765.22909 | 0.0 | 3.7 |
| 2-1-m1E02-1-0762 | C38 H38 F3 N5 O7 S | 765.24440 | -1.4 | -12.9 |
| 2-1-m1E02-1-0763 | C39 H39 N7 O8 S | 765.25808 | -3.1 | -3.5 |
| 2-1-m1E02-1-0764 | C39 H42 F3 N5 O8 | 765.29855 | -2.2 | -5.1 |
| 2-1-m1E02-1-0765 | C35 H34 N4 O10 S3 | 766.14371 | -27.2 | -1.7 |
| 2-1-m1E02-1-0766 | C36 H32 F2 N4 O9 S2 | 766.15788 | 0.0 | 0.0 |
| 2-1-m1E02-1-0767 | C38 H34 F4 N4 O7 S | 766.20843 | -3.2 | -48.4 |
| 2-1-m1E02-1-0768 | C35 H34 F N5 O10 S2 | 767.17311 | 0.0 | -2.3 |
| 2-1-m1E02-1-0769 | C36 H32 F3 N5 O9 S | 767.18728 | 1.3 | -1.0 |
| 2-1-m1E02-1-0770 | C35 H37 N5 O11 S2 | 767.19310 | 0.0 | -1.0 |
| 2-1-m1E02-1-0771 | C39 H37 N5 O8 S2 | 767.20835 | 0.0 | 0.0 |
| 2-1-m1E02-1-0772 | C37 H36 F3 N5 O8 S | 767.22367 | -3.8 | -3.1 |
| 2-1-m1E02-1-0773 | C37 H36 F3 N5 O8 S | 767.22367 | -2.5 | -2.7 |
| 2-1-m1E02-1-0774 | C38 H37 N7 O9 S | 767.23735 | 0.0 | 0.0 |
| 2-1-m1E02-1-0775 | C40 H41 N5 O9 S | 767.26250 | 91.8 | 77.1 |
| 2-1-m1E02-1-0776 | C41 H45 N5 O8 S | 767.29888 | -4.2 | -6.1 |
| 2-1-m1E02-1-0777 | C36 H31 F3 N4 O8 S2 | 768.15354 | 0.0 | 0.0 |
| 2-1-m1E02-1-0778 | C36 H31 F3 N4 O10 S | 768.17130 | 0.0 | 0.0 |
| 2-1-m1E02-1-0779 | C36 H35 F3 N6 O8 S | 768.21892 | 77.4 | 0.0 |
| 2-1-m1E02-1-0780 | C42 H36 N6 O7 S | 768.23662 | -4.3 | -4.7 |
| 2-1-m1E02-1-0781 | C39 H40 N6 O7 S2 | 768.23999 | 0.3 | 0.0 |
| 2-1-m1E02-1-0782 | C34 H35 N5 O10 S3 | 769.15460 | -0.5 | -3.1 |
| 2-1-m1E02-1-0783 | C34 H35 N5 012 S2 | 769.17236 | 0.0 | -1.4 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0784 | C36 H34 F3 N5 O9 S | 769.20293 | -8.2 | -5.5 |
| 2-1-m1E02-1-0785 | C36 H34 F3 N5 O9 S | 769.20293 | -6.2 | -5.3 |
| 2-1-m1E02-1-0786 | C39 H39 N5 O8 S2 | 769.22400 | 4.4 | -8.6 |
| 2-1-m1E02-1-0787 | C39 H39 N5 O8 S2 | 769.22400 | -3.2 | -5.0 |
| 2-1-m1E02-1-0788 | C39 H39 N5 O10 S | 769.24176 | 0.0 | 9.3 |
| 2-1-m1E02-1-0789 | C38 H34 N4 O10 S2 | 770.17163 | 0.0 | 0.0 |
| 2-1-m1E02-1-0790 | C37 H34 N6 O9 S2 | 770.18287 | -1.4 | -2.2 |
| 2-1-m1E02-1-0791 | C37 H31 F5 N4 O7 S | 770.18336 | -4.0 | -2.4 |
| 2-1-m1E02-1-0792 | C35 H33 F3 N6 O9 S | 770.19818 | -3.0 | -5.2 |
| 2-1-m1E02-1-0793 | C38 H38 N6 O8 S2 | 770.21925 | -2.1 | -5.8 |
| 2-1-m1E02-1-0794 | C38 H38 N6 O8 S2 | 770.21925 | 0.0 | 0.0 |
| 2-1-m1E02-1-0795 | C43 H38 N4 O8 S | 770.24103 | 0.8 | 0.0 |
| 2-1-m1E02-1-0796 | C41 H40 F2 N4 O7 S | 770.25858 | 0.0 | 0.0 |
| 2-1-m1E02-1-0797 | C36 H33 N7 O9 S2 | 771.17812 | -3.4 | -2.7 |
| 2-1-m1E02-1-0798 | C35 H32 F3 N5 O10 S | 771.18220 | 0.0 | 0.0 |
| 2-1-m1E02-1-0799 | C34 H32 F3 N7 O9 S | 771.19343 | 0.0 | 0.0 |
| 2-1-m1E02-1-0800 | C36 H33 F4 N5 O8 S | 771.19860 | 0.0 | 0.0 |
| 2-1-m1E02-1-0801 | C38 H37 N5 O9 S2 | 771.20327 | -2.4 | 0.0 |
| 2-1-m1E02-1-0802 | C38 H37 N5 O9 S2 | 771.20327 | -1.1 | 0.0 |
| 2-1-m1E02-1-0803 | C40 H42 F N5 O8 S | 771.27381 | -7.0 | -7.8 |
| 2-1-m1E02-1-0804 | C34 H30 F2 N4 O9 S3 | 772.11430 | -8.4 | -7.3 |
| 2-1-m1E02-1-0805 | C42 H36 N4 O7 S2 | 772.20254 | 2.4 | -1.4 |
| 2-1-m1E02-1-0806 | C42 H36 N4 O7 S2 | 772.20254 | 8.0 | 0.0 |
| 2-1-m1E02-1-0807 | C33 H32 F N5 O10 S3 | 773.12953 | -6.4 | -4.5 |
| 2-1-m1E02-1-0808 | C38 H33 F2 N5 O7 S2 | 773.17895 | -7.3 | -5.6 |
| 2-1-m1E02-1-0809 | C37 H33 F2 N7 O8 S | 773.20794 | 0.0 | 0.0 |
| 2-1-m1E02-1-0810 | C37 H33 F6 N5 O7 | 773.22842 | 5.8 | -2.0 |
| 2-1-m1E02-1-0811 | C40 H47 N5 O9 S | 773.30945 | -0.8 | -0.3 |
| 2-1-m1E02-1-0812 | C37 H35 F N6 O8 S2 | 774.19418 | -6.1 | -3.9 |
| 2-1-m1E02-1-0813 | C37 H35 F N6 O8 S2 | 774.19418 | 0.0 | 0.0 |
| 2-1-m1E02-1-0814 | C41 H41 F3 N4 O6 S | 774.26989 | -4.0 | -7.2 |
| 2-1-m1E02-1-0815 | C33 H31 F2 N5 O11 S2 | 775.14295 | 0.0 | -4.2 |
| 2-1-m1E02-1-0816 | C34 H32 F3 N5 O9 S2 | 775.15935 | -4.7 | -3.1 |
| 2-1-m1E02-1-0817 | C38 H35 F2 N5 O9 S | 775.21235 | -6.0 | 0.0 |
| 2-1-m1E02-1-0818 | C39 H45 N5 O10 S | 775.28871 | 0.0 | 0.0 |
| 2-1-m1E02-1-0819 | C35 H32 N6 O9 S3 | 776.13929 | -4.1 | -3.2 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0820 | C33 H31 F3 N6 O9 S2 | 776.15460 | 0.0 | 0.0 |
| 2-1-m1E02-1-0821 | C40 H39 F3 N4 O7 S | 776.24915 | -1.5 | -8.8 |
| 2-1-m1E02-1-0822 | C43 H44 N4 O8 S | 776.28799 | -7.8 | 0.0 |
| 2-1-m1E02-1-0823 | C37 H33 F2 N5 O8 S2 | 777.17386 | 0.0 | 0.0 |
| 2-1-m1E02-1-0824 | C37 H33 F2 N5 O8 S2 | 777.17386 | 3.0 | -4.4 |
| 2-1-m1E02-1-0825 | C38 H34 F3 N5 O8 S | 777.20802 | -4.5 | -4.8 |
| 2-1-m1E02-1-0826 | C37 H39 N5 O10 S2 | 777.21383 | -5.8 | -9.1 |
| 2-1-m1E02-1-0827 | C38 H33 F3 N4 O9 S | 778.19203 | -2.8 | -5.9 |
| 2-1-m1E02-1-0828 | C39 H37 F3 N4 O8 S | 778.22842 | 0.0 | 0.0 |
| 2-1-m1E02-1-0829 | C39 H37 F3 N4 O8 S | 778.22842 | -2.7 | -4.9 |
| 2-1-m1E02-1-0830 | C37 H32 F3 N5 O9 S | 779.18728 | 0.0 | 0.0 |
| 2-1-m1E02-1-0831 | C36 H37 N5 Oil S2 | 779.19310 | -4.1 | -2.2 |
| 2-1-m1E02-1-0832 | C39 H40 F3 N5 O7 S | 779.26005 | -0.2 | -7.3 |
| 2-1-m1E02-1-0833 | C38 H32 F4 N4 O8 S | 780.18770 | -6.5 | -3.4 |
| 2-1-m1E02-1-0834 | C40 H36 N4 O9 S2 | 780.19237 | 0.0 | 0.0 |
| 2-1-m1E02-1-0835 | C37 H34 F3 N5 O9 S | 781.20293 | -0.7 | -1.9 |
| 2-1-m1E02-1-0836 | C38 H38 F3 N5 O8 S | 781.23932 | -4.5 | -1.4 |
| 2-1-m1E02-1-0837 | C38 H38 F3 N5 O8 S | 781.23932 | -6.8 | -6.1 |
| 2-1-m1E02-1-0838 | C38 H38 F3 N5 O8 S | 781.23932 | -3.7 | -1.4 |
| 2-1-m1E02-1-0839 | C38 H41 F2 N5 O9 S | 781.25931 | 3.1 | 0.0 |
| 2-1-m1E02-1-0840 | C42 H47 N5 O8 S | 781.31453 | 0.0 | 0.0 |
| 2-1-m1E02-1-0841 | C36 H33 F3 N6 O9 S | 782.19818 | 0.0 | 0.0 |
| 2-1-m1E02-1-0842 | C38 H34 F4 N4 O8 S | 782.20335 | -8.3 | -2.7 |
| 2-1-m1E02-1-0843 | C40 H42 N6 O7 S2 | 782.25564 | 0.0 | 0.0 |
| 2-1-m1E02-1-0844 | C35 H37 N5 O10 S3 | 783.17025 | 0.0 | 0.0 |
| 2-1-m1E02-1-0845 | C37 H36 F3 N5 O9 S | 783.21858 | 0.0 | 2.5 |
| 2-1-m1E02-1-0846 | C37 H36 F3 N5 O9 S | 783.21858 | -5.9 | -4.4 |
| 2-1-m1E02-1-0847 | C41 H45 N5 O9 S | 783.29380 | -8.7 | -7.6 |
| 2-1-m1E02-1-0848 | C36 H31 F3 N4 O9 S2 | 784.14845 | -6.6 | -2.4 |
| 2-1-m1E02-1-0849 | C39 H36 N4 O10 S2 | 784.18728 | 0.0 | 0.0 |
| 2-1-m1E02-1-0850 | C36 H35 F3 N6 O9 S | 784.21383 | 0.0 | 0.0 |
| 2-1-m1E02-1-0851 | C41 H35 F3 N4 O7 S | 784.21785 | -8.8 | -14.7 |
| 2-1-m1E02-1-0852 | C39 H40 N6 O8 S2 | 784.23490 | -5.1 | -2.1 |
| 2-1-m1E02-1-0853 | C45 H44 N4 O7 S | 784.29307 | -3.4 | -20.7 |
| 2-1-m1E02-1-0854 | C34 H35 N5 O11 S3 | 785.14952 | 0.0 | 0.0 |
| 2-1-m1E02-1-0855 | C35 H33 F2 N5 O10 S2 | 785.16369 | 0.0 | 0.0 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0856 | C38 H35 N5 O10 S2 | 785.18253 | -6.2 | -7.1 |
| 2-1-m1E02-1-0857 | C37 H35 N7 O9 S2 | 785.19377 | 0.0 | 0.6 |
| 2-1-m1E02-1-0858 | C37 H35 F4 N5 O8 S | 785.21425 | 0.0 | 0.0 |
| 2-1-m1E02-1-0859 | C38 H34 N4 O9 S3 | 786.14879 | -14.9 | -9.6 |
| 2-1-m1E02-1-0860 | C38 H34 N4 O9 S3 | 786.14879 | -4.9 | -6.7 |
| 2-1-m1E02-1-0861 | C39 H33 F3 N6 O7 S | 786.20835 | -11.2 | -4.7 |
| 2-1-m1E02-1-0862 | C38 H38 N6 O9 S2 | 786.21417 | 0.0 | 0.0 |
| 2-1-m1E02-1-0863 | C38 H38 N6 O9 S2 | 786.21417 | -5.5 | -3.2 |
| 2-1-m1E02-1-0864 | C37 H33 N5 O9 S3 | 787.14404 | -7.6 | -6.0 |
| 2-1-m1E02-1-0865 | C35 H32 F3 N5 O9 S2 | 787.15935 | -0.6 | -0.8 |
| 2-1-m1E02-1-0866 | C35 H32 F3 N5 O11 S | 787.17711 | 0.0 | 0.0 |
| 2-1-m1E02-1-0867 | C42 H37 N5 O7 S2 | 787.21344 | 3.0 | -7.8 |
| 2-1-m1E02-1-0868 | C35 H31 F3 N4 O10 S2 | 788.14337 | -4.2 | -5.9 |
| 2-1-m1E02-1-0869 | C37 H33 F N6 O9 S2 | 788.17345 | -1.1 | -1.6 |
| 2-1-m1E02-1-0870 | C40 H35 F3 N4 O8 S | 788.21277 | 12.5 | -25.9 |
| 2-1-m1E02-1-0871 | C36 H32 F5 N5 O8 S | 789.18917 | -0.5 | -3.4 |
| 2-1-m1E02-1-0872 | C40 H41 F2 N5 O8 S | 789.26439 | -6.7 | 0.0 |
| 2-1-m1E02-1-0873 | C39 H33 F3 N4 O7 S2 | 790.17428 | 0.0 | 0.0 |
| 2-1-m1E02-1-0874 | C39 H33 F3 N4 O7 S2 | 790.17428 | 1.1 | -7.5 |
| 2-1-m1E02-1-0875 | C41 H41 F3 N4 O7 S | 790.26480 | 5.1 | -1.4 |
| 2-1-m1E02-1-0876 | C33 H31 F2 N5 O10 S3 | 791.12011 | 0.0 | -2.0 |
| 2-1-m1E02-1-0877 | C37 H34 F2 N6 O8 S2 | 792.18476 | 1.6 | -9.9 |
| 2-1-m1E02-1-0878 | C38 H34 F3 N5 O9 S | 793.20293 | -4.7 | -2.9 |
| 2-1-m1E02-1-0879 | C40 H42 F3 N5 O7 S | 793.27570 | 0.0 | 0.0 |
| 2-1-m1E02-1-0880 | C38 H34 N8 O8 S2 | 794.19410 | 17.0 | 0.0 |
| 2-1-m1E02-1-0881 | C39 H37 F3 N4 O9 S | 794.22333 | 0.0 | 0.0 |
| 2-1-m1E02-1-0882 | C40 H41 F3 N4 O8 S | 794.25972 | 0.0 | 0.0 |
| 2-1-m1E02-1-0883 | C39 H40 F3 N5 O8 S | 795.25497 | 0.0 | 0.0 |
| 2-1-m1E02-1-0884 | C34 H32 N6 O11 S3 | 796.12912 | -5.3 | -5.4 |
| 2-1-m1E02-1-0885 | C38 H32 F4 N4 O9 S | 796.18261 | -4.7 | -4.0 |
| 2-1-m1E02-1-0886 | C39 H36 N6 O9 S2 | 796.19852 | 0.1 | -4.5 |
| 2-1-m1E02-1-0887 | C37 H35 F3 N6 O9 S | 796.21383 | 5.4 | 0.0 |
| 2-1-m1E02-1-0888 | C37 H34 F3 N5 O10 S | 797.19785 | 2.8 | -1.2 |
| 2-1-m1E02-1-0889 | C38 H38 F3 N5 O9 S | 797.23423 | -5.6 | -4.2 |
| 2-1-m1E02-1-0890 | C38 H38 F3 N5 O9 S | 797.23423 | 0.0 | 0.0 |
| 2-1-m1E02-1-0891 | C38 H34 N6 O8 S3 | 798.16002 | -18.1 | -3.4 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0892 | C38 H34 N6 O8 S3 | 798.16002 | -16.8 | 7.0 |
| 2-1-m1E02-1-0893 | C37 H33 F3 N4 O9 S2 | 798.16410 | -3.0 | -5.6 |
| 2-1-m1E02-1-0894 | C36 H33 F3 N6 O10 S | 798.19310 | 6.7 | 0.0 |
| 2-1-m1E02-1-0895 | C40 H38 N4 O10 S2 | 798.20293 | 0.0 | 0.0 |
| 2-1-m1E02-1-0896 | C37 H33 F4 N5 O9 S | 799.19351 | 0.0 | -1.3 |
| 2-1-m1E02-1-0897 | C39 H36 N4 O9 S3 | 800.16444 | -5.0 | 1.4 |
| 2-1-m1E02-1-0898 | C38 H35 N5 O9 S3 | 801.15969 | 2.0 | 0.0 |
| 2-1-m1E02-1-0899 | C37 H35 F4 N5 O9 S | 801.20916 | 0.0 | 0.0 |
| 2-1-m1E02-1-0900 | C37 H34 N6 O9 S3 | 802.15494 | -7.5 | -7.6 |
| 2-1-m1E02-1-0901 | C39 H35 F N4 O10 S2 | 802.17786 | 0.0 | 0.0 |
| 2-1-m1E02-1-0902 | C38 H32 F6 N4 O7 S | 802.18959 | -9.0 | -7.4 |
| 2-1-m1E02-1-0903 | C39 H33 F3 N6 O8 S | 802.20327 | -4.0 | -1.7 |
| 2-1-m1E02-1-0904 | C39 H42 N6 O9 S2 | 802.24547 | 0.0 | 0.0 |
| 2-1-m1E02-1-0905 | C42 H41 F3 N4 O7 S | 802.26480 | -3.8 | -13.1 |
| 2-1-m1E02-1-0906 | C35 H32 F3 N5 O10 S2 | 803.15427 | -4.4 | -6.4 |
| 2-1-m1E02-1-0907 | C38 H37 N5 O11 S2 | 803.19310 | -11.4 | -6.2 |
| 2-1-m1E02-1-0908 | C35 H31 F3 N4 O9 S3 | 804.12053 | -4.4 | -5.0 |
| 2-1-m1E02-1-0909 | C35 H31 F3 N4 O11 S2 | 804.13828 | -3.7 | -3.6 |
| 2-1-m1E02-1-0910 | C38 H33 F N4 O9 S3 | 804.13937 | -4.5 | -3.5 |
| 2-1-m1E02-1-0911 | C37 H36 N6 O11 S2 | 804.18835 | 11.9 | -3.0 |
| 2-1-m1E02-1-0912 | C40 H35 F3 N4 O9 S | 804.20768 | 6.9 | -0.4 |
| 2-1-m1E02-1-0913 | C37 H35 N5 O10 S3 | 805.15460 | -4.5 | -4.9 |
| 2-1-m1E02-1-0914 | C39 H34 F3 N5 O7 S2 | 805.18517 | -4.3 | -11.0 |
| 2-1-m1E02-1-0915 | C38 H34 F3 N7 O8 S | 805.21417 | -7.0 | -6.6 |
| 2-1-m1E02-1-0916 | C36 H34 N6 O10 S3 | 806.14985 | -10.7 | -6.0 |
| 2-1-m1E02-1-0917 | C36 H34 N6 O10 S3 | 806.14985 | -3.8 | -2.7 |
| 2-1-m1E02-1-0918 | C39 H33 F3 N4 O8 S2 | 806.16919 | -21.7 | 1.6 |
| 2-1-m1E02-1-0919 | C39 H33 F3 N4 O8 S2 | 806.16919 | 0.0 | 0.0 |
| 2-1-m1E02-1-0920 | C34 H32 F3 N5 O11 S2 | 807.14918 | 0.0 | 0.0 |
| 2-1-m1E02-1-0921 | C39 H36 F3 N5 O9 S | 807.21858 | 0.0 | -8.9 |
| 2-1-m1E02-1-0922 | C38 H34 F3 N5 O8 S2 | 809.18009 | 4.3 | 0.0 |
| 2-1-m1E02-1-0923 | C38 H34 F3 N5 O8 S2 | 809.18009 | 0.0 | 0.0 |
| 2-1-m1E02-1-0924 | C40 H42 F3 N5 O8 S | 809.27062 | 0.0 | 0.0 |
| 2-1-m1E02-1-0925 | C37 H33 F3 N6 O8 S2 | 810.17534 | 0.0 | 0.0 |
| 2-1-m1E02-1-0926 | C41 H42 N6 O8 S2 | 810.25055 | 5.6 | -1.6 |
| 2-1-m1E02-1-0927 | C40 H41 F3 N4 O9 S | 810.25463 | 0.0 | -14.4 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0928 | C34 H32 N6 O10 S4 | 812.10627 | -6.7 | -5.0 |
| 2-1-m1E02-1-0929 | C37 H35 F3 N6 O10 S | 812.20875 | -1.5 | -4.0 |
| 2-1-m1E02-1-0930 | C41 H40 N4 O10 S2 | 812.21858 | -18.2 | -9.2 |
| 2-1-m1E02-1-0931 | C38 H35 N7 O8 S3 | 813.17092 | -0.5 | -28.1 |
| 2-1-m1E02-1-0932 | C38 H38 F3 N5 O10 S | 813.22915 | 0.0 | 0.0 |
| 2-1-m1E02-1-0933 | C39 H42 F3 N5 O9 S | 813.26553 | 2.4 | 0.0 |
| 2-1-m1E02-1-0934 | C37 H33 F3 N4 O10 S2 | 814.15902 | 0.0 | 0.0 |
| 2-1-m1E02-1-0935 | C40 H38 N4 O9 S3 | 814.18009 | -19.0 | -11.1 |
| 2-1-m1E02-1-0936 | C40 H38 N4 O11 S2 | 814.19785 | 0.0 | -21.3 |
| 2-1-m1E02-1-0937 | C39 H37 N5 O9 S3 | 815.17534 | -3.3 | 0.6 |
| 2-1-m1E02-1-0938 | C37 H33 F4 N5 O10 S | 815.18843 | 0.0 | -1.0 |
| 2-1-m1E02-1-0939 | C39 H36 N4 O10 S3 | 816.15936 | 0.0 | 0.0 |
| 2-1-m1E02-1-0940 | C36 H34 F3 N5 O10 S2 | 817.16992 | -6.3 | -3.8 |
| 2-1-m1E02-1-0941 | C39 H39 N5 O11 S2 | 817.20875 | -7.5 | -5.3 |
| 2-1-m1E02-1-0942 | C38 H32 F6 N4 O8 S | 818.18450 | -10.4 | -14.0 |
| 2-1-m1E02-1-0943 | C42 H41 F3 N4 O8 S | 818.25972 | 2.4 | -6.2 |
| 2-1-m1E02-1-0944 | C38 H34 F N5 O9 S3 | 819.15027 | -10.0 | -5.3 |
| 2-1-m1E02-1-0945 | C38 H37 N5 O10 S3 | 819.17025 | -6.0 | -4.1 |
| 2-1-m1E02-1-0946 | C35 H31 F3 N4 O10 S3 | 820.11544 | -3.1 | -3.3 |
| 2-1-m1E02-1-0947 | C37 H36 N6 O10 S3 | 820.16550 | 0.0 | -0.7 |
| 2-1-m1E02-1-0948 | C39 H34 F2 N4 O10 S2 | 820.16844 | -1.5 | -6.1 |
| 2-1-m1E02-1-0949 | C36 H35 N7 O10 S3 | 821.16075 | -4.9 | -2.2 |
| 2-1-m1E02-1-0950 | C39 H34 F3 N5 O8 S2 | 821.18009 | -19.6 | 4.5 |
| 2-1-m1E02-1-0951 | C38 H36 F N5 O11 S2 | 821.18368 | 7.4 | 7.2 |
| 2-1-m1E02-1-0952 | C37 H33 F6 N5 O8 S | 821.19540 | -4.4 | 0.0 |
| 2-1-m1E02-1-0953 | C38 H34 F3 N7 O9 S | 821.20908 | 1.8 | 0.0 |
| 2-1-m1E02-1-0954 | C41 H42 F3 N5 O8 S | 821.27062 | 1.0 | -2.0 |
| 2-1-m1E02-1-0955 | C38 H32 F2 N4 O9 S3 | 822.12995 | 0.0 | -7.1 |
| 2-1-m1E02-1-0956 | C34 H32 F3 N5 O10 S3 | 823.12634 | 15.5 | 0.0 |
| 2-1-m1E02-1-0957 | C34 H32 F3 N5 012 S2 | 823.14410 | -4.1 | -1.2 |
| 2-1-m1E02-1-0958 | C37 H34 F N5 O10 S3 | 823.14518 | -7.7 | -2.1 |
| 2-1-m1E02-1-0959 | C39 H36 F3 N5 O10 S | 823.21350 | -7.4 | 0.4 |
| 2-1-m1E02-1-0960 | C38 H35 F3 N6 O8 S2 | 824.19099 | 0.0 | 5.7 |
| 2-1-m1E02-1-0961 | C38 H34 F3 N5 O9 S2 | 825.17500 | 9.5 | 3.4 |
| 2-1-m1E02-1-0962 | C38 H34 F3 N5 O9 S2 | 825.17500 | 0.0 | 0.0 |
| 2-1-m1E02-1-0963 | C40 H39 N5 O9 S3 | 829.19099 | 0.0 | -3.7 |

(continued)

| [Table 11-2-2] | | | | |
|---|---|---|---|---|
| | | | AS-MS ratio (%) | |
| ID | Molecular Formula | Exact Mass | N1 | N2 |
| 2-1-m1E02-1-0964 | C39 H42 F3 N5 O10 S | 829.26045 | 0.0 | 0.0 |
| 2-1-m1E02-1-0965 | C39 H37 N5 O10 S3 | 831.17025 | -57.3 | -18.2 |
| 2-1-m1E02-1-0966 | C40 H41 N5 O11 S2 | 831.22440 | -8.0 | -5.2 |
| 2-1-m1E02-1-0967 | C36 H34 F3 N5 O11 S2 | 833.16483 | -9.7 | -6.0 |
| 2-1-m1E02-1-0968 | C39 H39 N5 O10 S3 | 833.18590 | -6.0 | -4.3 |
| 2-1-m1E02-1-0969 | C39 H39 N5 O12 S2 | 833.20366 | -10.3 | -51.6 |
| 2-1-m1E02-1-0970 | C38 H38 N6 O10 S3 | 834.18115 | -7.4 | -6.0 |
| 2-1-m1E02-1-0971 | C43 H38 N4 O10 S2 | 834.20293 | -9.0 | -7.3 |
| 2-1-m1E02-1-0972 | C38 H37 N5 O11 S3 | 835.16517 | 5.2 | 2.2 |
| 2-1-m1E02-1-0973 | C42 H36 N4 O9 S3 | 836.16444 | -16.0 | -20.2 |
| 2-1-m1E02-1-0974 | C38 H33 F2 N5 O9 S3 | 837.14085 | -4.1 | -4.4 |
| 2-1-m1E02-1-0975 | C37 H33 F6 N5 O9 S | 837.19032 | -4.0 | -7.8 |
| 2-1-m1E02-1-0976 | C41 H42 F3 N5 O9 S | 837.26553 | 0.0 | 0.0 |
| 2-1-m1E02-1-0977 | C37 H35 F N6 O10 S3 | 838.15608 | -5.3 | -7.9 |
| 2-1-m1E02-1-0978 | C34 H32 F3 N5 O11 S3 | 839.12125 | -5.7 | -6.6 |
| 2-1-m1E02-1-0979 | C38 H35 F2 N5 O11 S2 | 839.17425 | 13.2 | -6.0 |
| 2-1-m1E02-1-0980 | C38 H35 F3 N6 O9 S2 | 840.18590 | 9.2 | -1.2 |
| 2-1-m1E02-1-0981 | C37 H33 F2 N5 O10 S3 | 841.13576 | -15.3 | -5.9 |
| 2-1-m1E02-1-0982 | C39 H40 N6 O10 S3 | 848.19680 | 0.0 | -1.8 |
| 2-1-m1E02-1-0983 | C38 H38 N6 O11 S3 | 850.17607 | 4.0 | -2.1 |
| 2-1-m1E02-1-0984 | C42 H37 N5 O9 S3 | 851.17534 | 0.0 | 4.7 |
| 2-1-m1E02-1-0985 | C40 H35 F3 N4 O10 S2 | 852.17467 | -9.2 | -5.4 |
| 2-1-m1E02-1-0986 | C39 H33 F3 N4 O9 S3 | 854.13618 | -12.8 | -4.0 |
| 2-1-m1E02-1-0987 | C37 H34 F2 N6 O10 S3 | 856.14666 | 7.9 | -4.4 |
| 2-1-m1E02-1-0988 | C39 H36 N6 O11 S3 | 860.16042 | -6.7 | -6.2 |
| 2-1-m1E02-1-0989 | C38 H34 N6 O10 S4 | 862.12192 | -7.1 | -8.7 |
| 2-1-m1E02-1-0990 | C40 H35 F3 N4 O11 S2 | 868.16958 | -10.2 | -15.6 |
| 2-1-m1E02-1-0991 | C39 H34 F3 N5 O9 S3 | 869.14707 | 5.7 | -5.6 |
| 2-1-m1E02-1-0992 | C39 H33 F3 N4 O10 S3 | 870.13109 | -13.1 | -10.2 |
| 2-1-m1E02-1-0993 | C39 H36 F3 N5 O11 S2 | 871.18048 | -9.2 | 0.0 |
| 2-1-m1E02-1-0994 | C38 H34 F3 N5 O10 S3 | 873.14199 | -13.1 | -4.9 |
| 2-1-m1E02-1-0995 | C38 H35 N7 O10 S4 | 877.13282 | -8.8 | -7.2 |
| 2-1-m1E02-1-0996 | C39 H34 F3 N5 O10 S3 | 885.14199 | -6.1 | -5.8 |
| 2-1-m1E02-1-0997 | C39 H36 F3 N5 012 S2 | 887.17540 | -6.2 | -2.9 |
| 2-1-m1E02-1-0998 | C38 H35 F3 N6 O10 S3 | 888.15289 | -4.8 | -10.8 |
| 2-1-m1E02-1-0999 | C38 H34 F3 N5 O11 S3 | 889.13690 | -6.8 | -3.4 |
| 2-1-m1E02-1-1000 | C38 H35 F3 N6 O11 S3 | 904.14780 | -5.5 | -4.6 |

[Table 592]

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0001 | C38 H32 N4 O4 S | 640.21443 | 5.4 | 6.0 |
| 2-1-m1E03-1-0002 | C37 H31 N5 O4 S | 641.20968 | 15.0 | -0.5 |
| 2-1-m1E03-1-0003 | C37 H31 N5 O4 S | 641.20968 | 0.0 | 7.5 |
| 2-1-m1E03-1-0004 | C36 H30 N6 O4 S | 642.20492 | -0.1 | -0.3 |
| 2-1-m1E03-1-0005 | C36 H30 N6 O4 S | 642.20492 | -9.9 | 1.1 |
| 2-1-m1E03-1-0006 | C35 H29 N7 O4 S | 643.20017 | 0.7 | -4.3 |
| 2-1-m1E03-1-0007 | C35 H29 N5 O4 S2 | 647.16610 | -6.2 | -3.3 |
| 2-1-m1E03-1-0008 | C34 H28 N6 O4 S2 | 648.16134 | -0.1 | -0.7 |
| 2-1-m1E03-1-0009 | C39 H34 N4 O4 S | 654.23008 | 2.1 | -1.9 |
| 2-1-m1E03-1-0010 | C38 H33 N5 O4 S | 655.22533 | 2.4 | -5.3 |
| 2-1-m1E03-1-0011 | C37 H32 N6 O4 S | 656.22057 | 5.8 | -5.0 |
| 2-1-m1E03-1-0012 | C37 H30 N4 O6 S | 658.18861 | -5.2 | -28.5 |
| 2-1-m1E03-1-0013 | C38 H31 F N4 O4 S | 658.20500 | -1.5 | 8.7 |
| 2-1-m1E03-1-0014 | C36 H29 N5 O6 S | 659.18385 | 0.1 | 0.8 |
| 2-1-m1E03-1-0015 | C37 H30 F N5 O4 S | 659.20025 | 2.5 | 4.2 |
| 2-1-m1E03-1-0016 | C37 H33 N5 O5 S | 659.22024 | 0.0 | 9.0 |
| 2-1-m1E03-1-0017 | C36 H29 F N6 O4 S | 660.19550 | -3.1 | 0.7 |
| 2-1-m1E03-1-0018 | C36 H32 N6 O5 S | 660.21549 | -1.1 | -0.9 |
| 2-1-m1E03-1-0019 | C36 H32 N6 O5 S | 660.21549 | -2.1 | 9.6 |
| 2-1-m1E03-1-0020 | C36 H31 N5 O4 S2 | 661.18175 | -16.0 | 0.9 |
| 2-1-m1E03-1-0021 | C35 H31 N7 O5 S | 661.21074 | -4.2 | -1.6 |
| 2-1-m1E03-1-0022 | C35 H31 N7 O5 S | 661.21074 | -2.7 | 0.7 |
| 2-1-m1E03-1-0023 | C34 H30 N8 O5 S | 662.20599 | 2.1 | 2.3 |
| 2-1-m1E03-1-0024 | C35 H28 F N5 O4 S2 | 665.15667 | -5.2 | -3.0 |
| 2-1-m1E03-1-0025 | C34 H30 N6 O5 S2 | 666.17191 | -49.5 | -6.4 |
| 2-1-m1E03-1-0026 | C33 H29 N7 O5 S2 | 667.16716 | -21.9 | -6.4 |
| 2-1-m1E03-1-0027 | C39 H32 N4 O5 S | 668.20934 | -1.0 | 5.9 |
| 2-1-m1E03-1-0028 | C40 H36 N4 O4 S | 668.24573 | -9.3 | 2.3 |
| 2-1-m1E03-1-0029 | C38 H31 N5 O5 S | 669.20459 | -2.2 | -3.3 |
| 2-1-m1E03-1-0030 | C38 H31 N5 O5 S | 669.20459 | -1.0 | -0.6 |
| 2-1-m1E03-1-0031 | C39 H35 N5 O4 S | 669.24098 | 2.5 | 4.3 |
| 2-1-m1E03-1-0032 | C37 H30 N6 O5 S | 670.19984 | -0.7 | -7.2 |
| 2-1-m1E03-1-0033 | C39 H34 N4 O5 S | 670.22499 | -13.0 | 39.2 |
| 2-1-m1E03-1-0034 | C38 H34 N6 O4 S | 670.23622 | 24.8 | -4.5 |
| 2-1-m1E03-1-0035 | C38 H33 N5 O5 S | 671.22024 | -6.5 | 1.5 |
| 2-1-m1E03-1-0036 | C38 H32 N4 O6 S | 672.20426 | 7.3 | 0.0 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0037 | C37 H32 N6 O5 S | 672.21549 | 3.0 | -1.7 |
| 2-1-m1E03-1-0038 | C38 H35 N5 O5 S | 673.23589 | 0.8 | -5.1 |
| 2-1-m1E03-1-0039 | C37 H30 N4 O5 S2 | 674.16576 | -3.7 | -5.2 |
| 2-1-m1E03-1-0040 | C37 H34 N6 O5 S | 674.23114 | -0.1 | -0.1 |
| 2-1-m1E03-1-0041 | C36 H29 N5 O5 S2 | 675.16101 | -0.8 | -3.4 |
| 2-1-m1E03-1-0042 | C37 H33 N5 O4 S2 | 675.19740 | -12.6 | 7.5 |
| 2-1-m1E03-1-0043 | C36 H33 N7 O5 S | 675.22639 | 0.0 | 0.0 |
| 2-1-m1E03-1-0044 | C37 H29 F N4 O6 S | 676.17918 | -3.3 | -7.5 |
| 2-1-m1E03-1-0045 | C38 H30 F2 N4 O4 S | 676.19558 | 5.6 | 1.8 |
| 2-1-m1E03-1-0046 | C36 H31 N5 O5 S2 | 677.17666 | 0.0 | 0.0 |
| 2-1-m1E03-1-0047 | C37 H29 F2 N5 O4 S | 677.19083 | 5.3 | -0.5 |
| 2-1-m1E03-1-0048 | C36 H31 N5 O7 S | 677.19442 | -0.1 | 0.4 |
| 2-1-m1E03-1-0049 | C37 H32 F N5 O5 S | 677.21082 | 12.1 | 20.5 |
| 2-1-m1E03-1-0050 | C36 H28 F2 N6 O4 S | 678.18608 | 7.9 | -5.3 |
| 2-1-m1E03-1-0051 | C35 H30 N6 O7 S | 678.18967 | -2.7 | -2.9 |
| 2-1-m1E03-1-0052 | C36 H31 F N6 O5 S | 678.20607 | 0.9 | 0.0 |
| 2-1-m1E03-1-0053 | C35 H30 F N7 O5 S | 679.20132 | -4.1 | 1.3 |
| 2-1-m1E03-1-0054 | C35 H32 N6 O5 S2 | 680.18756 | 4.3 | 2.8 |
| 2-1-m1E03-1-0055 | C40 H34 N4 O5 S | 682.22499 | 0.0 | 0.0 |
| 2-1-m1E03-1-0056 | C35 H27 F2 N5 O4 S2 | 683.14725 | 0.5 | -9.0 |
| 2-1-m1E03-1-0057 | C39 H33 N5 O5 S | 683.22024 | -4.2 | 13.9 |
| 2-1-m1E03-1-0058 | C39 H33 N5 O5 S | 683.22024 | -13.4 | -0.9 |
| 2-1-m1E03-1-0059 | C34 H29 F N6 O5 S2 | 684.16249 | 5.5 | -4.4 |
| 2-1-m1E03-1-0060 | C38 H32 N6 O5 S | 684.21549 | -2.4 | 0.3 |
| 2-1-m1E03-1-0061 | C39 H31 F N4 O5 S | 686.19992 | 0.0 | -20.8 |
| 2-1-m1E03-1-0062 | C39 H31 F N4 O5 S | 686.19992 | 0.0 | 0.9 |
| 2-1-m1E03-1-0063 | C39 H34 N4 O6 S | 686.21991 | 0.0 | 0.0 |
| 2-1-m1E03-1-0064 | C38 H30 F N5 O5 S | 687.19517 | -1.9 | -0.2 |
| 2-1-m1E03-1-0065 | C38 H30 F N5 O5 S | 687.19517 | -2.7 | -4.4 |
| 2-1-m1E03-1-0066 | C38 H33 N5 O6 S | 687.21515 | -2.8 | -0.5 |
| 2-1-m1E03-1-0067 | C39 H37 N5 O5 S | 687.25154 | -2.0 | -4.9 |
| 2-1-m1E03-1-0068 | C38 H32 N4 O5 S2 | 688.18141 | 0.0 | 0.0 |
| 2-1-m1E03-1-0069 | C38 H32 N4 O7 S | 688.19917 | 3.1 | 0.6 |
| 2-1-m1E03-1-0070 | C37 H32 N6 O6 S | 688.21040 | -5.0 | -1.2 |
| 2-1-m1E03-1-0071 | C37 H32 N6 O6 S | 688.21040 | -0.7 | -2.0 |
| 2-1-m1E03-1-0072 | C38 H36 N6 O5 S | 688.24679 | 4.7 | 14.2 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0073 | C36 H31 N7 O6 S | 689.20565 | -1.7 | 1.1 |
| 2-1-m1E03-1-0074 | C38 H35 N5 O6 S | 689.23080 | 9.6 | 0.8 |
| 2-1-m1E03-1-0075 | C37 H35 N7 O5 S | 689.24204 | 25.1 | -14.1 |
| 2-1-m1E03-1-0076 | C37 H34 N6 O6 S | 690.22605 | 0.0 | 0.0 |
| 2-1-m1E03-1-0077 | C42 H34 N4 O4 S | 690.23008 | -8.1 | -3.0 |
| 2-1-m1E03-1-0078 | C37 H33 N5 O7 S | 691.21007 | 1.5 | 10.2 |
| 2-1-m1E03-1-0079 | C36 H33 N7 O6 S | 691.22130 | 0.0 | 0.0 |
| 2-1-m1E03-1-0080 | C41 H33 N5 O4 S | 691.22533 | 5.9 | 4.0 |
| 2-1-m1E03-1-0081 | C37 H29 F N4 O5 S2 | 692.15634 | -2.8 | 2.6 |
| 2-1-m1E03-1-0082 | C40 H32 N6 O4 S | 692.22057 | 34.2 | -22.0 |
| 2-1-m1E03-1-0083 | C40 H32 N6 O4 S | 692.22057 | 2.7 | -4.3 |
| 2-1-m1E03-1-0084 | C36 H31 N5 O6 S2 | 693.17158 | -3.4 | -3.7 |
| 2-1-m1E03-1-0085 | C39 H31 N7 O4 S | 693.21582 | -0.3 | -1.4 |
| 2-1-m1E03-1-0086 | C36 H30 N4 O7 S2 | 694.15559 | 0.0 | 0.0 |
| 2-1-m1E03-1-0087 | C35 H30 N6 O6 S2 | 694.16682 | 3.6 | -1.7 |
| 2-1-m1E03-1-0088 | C37 H28 F2 N4 O6 S | 694.16976 | 0.4 | 0.4 |
| 2-1-m1E03-1-0089 | C36 H34 N6 O5 S2 | 694.20321 | -11.7 | -5.8 |
| 2-1-m1E03-1-0090 | C35 H29 N5 O7 S2 | 695.15084 | -0.9 | -2.4 |
| 2-1-m1E03-1-0091 | C36 H30 F N5 O7 S | 695.18500 | -2.5 | -0.5 |
| 2-1-m1E03-1-0092 | C37 H31 F2 N5 O5 S | 695.20140 | -3.5 | -1.5 |
| 2-1-m1E03-1-0093 | C35 H32 N6 O6 S2 | 696.18247 | 0.0 | 0.0 |
| 2-1-m1E03-1-0094 | C40 H32 N4 O4 S2 | 696.18650 | 1.0 | 0.2 |
| 2-1-m1E03-1-0095 | C36 H30 F2 N6 O5 S | 696.19665 | 2.3 | -2.4 |
| 2-1-m1E03-1-0096 | C37 H36 N4 O6 S2 | 696.20763 | 0.0 | 0.0 |
| 2-1-m1E03-1-0097 | C41 H36 N4 O5 S | 696.24064 | 0.0 | 6.5 |
| 2-1-m1E03-1-0098 | C39 H31 N5 O4 S2 | 697.18175 | -4.9 | 13.4 |
| 2-1-m1E03-1-0099 | C39 H31 N5 O4 S2 | 697.18175 | 16.1 | -3.3 |
| 2-1-m1E03-1-0100 | C35 H29 F2 N7 O5 S | 697.19189 | 0.0 | 24.8 |
| 2-1-m1E03-1-0101 | C36 H35 N5 O6 S2 | 697.20288 | -2.2 | -1.2 |
| 2-1-m1E03-1-0102 | C40 H35 N5 O5 S | 697.23589 | -2.8 | -4.4 |
| 2-1-m1E03-1-0103 | C40 H35 N5 O5 S | 697.23589 | 0.0 | 0.0 |
| 2-1-m1E03-1-0104 | C40 H34 N4 O6 S | 698.21991 | 0.0 | 0.0 |
| 2-1-m1E03-1-0105 | C39 H33 N5 O6 S | 699.21515 | 7.8 | 3.8 |
| 2-1-m1E03-1-0106 | C40 H33 F N4 O5 S | 700.21557 | 23.9 | 0.0 |
| 2-1-m1E03-1-0107 | C41 H40 N4 O5 S | 700.27194 | -5.7 | 0.0 |
| 2-1-m1E03-1-0108 | C39 H32 F N5 O5 S | 701.21082 | -3.1 | 1.4 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0109 | C39 H35 N5 O6 S | 701.23080 | 4.8 | 0.2 |
| 2-1-m1E03-1-0110 | C40 H39 N5 05 S | 701.26719 | -1.9 | -2.7 |
| 2-1-m1E03-1-0111 | C34 H28 F2 N6 O5 S2 | 702.15307 | 5.4 | 5.4 |
| 2-1-m1E03-1-0112 | C39 H34 N4 05 S2 | 702.19706 | 8.9 | 3.4 |
| 2-1-m1E03-1-0113 | C38 H34 N6 O6 S | 702.22605 | -3.8 | -1.3 |
| 2-1-m1E03-1-0114 | C38 H34 N6 O6 S | 702.22605 | 10.4 | 5.4 |
| 2-1-m1E03-1-0115 | C37 H33 N7 O6 S | 703.22130 | -10.4 | -0.2 |
| 2-1-m1E03-1-0116 | C38 H32 N4 O6 S2 | 704.17633 | -15.5 | -11.4 |
| 2-1-m1E03-1-0117 | C38 H32 N4 O6 S2 | 704.17633 | 16.1 | 0.0 |
| 2-1-m1E03-1-0118 | C38 H32 N4 O6 S2 | 704.17633 | -3.7 | 0.0 |
| 2-1-m1E03-1-0119 | C39 H30 F2 N4 05 S | 704.19050 | -4.7 | 2.9 |
| 2-1-m1E03-1-0120 | C39 H30 F2 N4 O5 S | 704.19050 | 0.0 | 0.0 |
| 2-1-m1E03-1-0121 | C37 H31 N5 O6 S2 | 705.17158 | -2.9 | -0.3 |
| 2-1-m1E03-1-0122 | C37 H31 N5 O6 S2 | 705.17158 | -0.6 | 11.0 |
| 2-1-m1E03-1-0123 | C37 H31 N5 O6 S2 | 705.17158 | -5.1 | -6.8 |
| 2-1-m1E03-1-0124 | C38 H29 F2 N5 O5 S | 705.18575 | -1.3 | 5.9 |
| 2-1-m1E03-1-0125 | C38 H32 F N5 O6 S | 705.20573 | -2.1 | -2.4 |
| 2-1-m1E03-1-0126 | C38 H32 F N5 O6 S | 705.20573 | 0.0 | -1.0 |
| 2-1-m1E03-1-0127 | C38 H35 N5 O7 S | 705.22572 | 4.5 | 16.5 |
| 2-1-m1E03-1-0128 | C36 H30 N6 O6 S2 | 706.16682 | -5.0 | -3.2 |
| 2-1-m1E03-1-0129 | C36 H30 N6 O6 S2 | 706.16682 | -1.8 | -2.1 |
| 2-1-m1E03-1-0130 | C37 H31 F N6 O6 S | 706.20098 | 1.2 | 2.0 |
| 2-1-m1E03-1-0131 | C37 H31 F N6 O6 S | 706.20098 | -1.4 | 0.4 |
| 2-1-m1E03-1-0132 | C41 H34 N6 O4 S | 706.23622 | -1.1 | 0.0 |
| 2-1-m1E03-1-0133 | C35 H29 N7 O6 S2 | 707.16207 | 0.0 | 0.0 |
| 2-1-m1E03-1-0134 | C37 H33 N5 O6 S2 | 707.18723 | -7.1 | -3.9 |
| 2-1-m1E03-1-0135 | C37 H33 N5 O8 S | 707.20498 | 5.1 | 5.5 |
| 2-1-m1E03-1-0136 | C37 H32 N4 O7 S2 | 708.17124 | 0.0 | 0.0 |
| 2-1-m1E03-1-0137 | C39 H31 F3 N4 O4 S | 708.20181 | 8.8 | 2.5 |
| 2-1-m1E03-1-0138 | C39 H31 F3 N4 O4 S | 708.20181 | 0.0 | 0.0 |
| 2-1-m1E03-1-0139 | C41 H32 N4 O6 S | 708.20426 | 3.5 | 14.9 |
| 2-1-m1E03-1-0140 | C38 H30 F3 N5 O4 S | 709.19706 | 14.1 | -5.0 |
| 2-1-m1E03-1-0141 | C38 H30 F3 N5 O4 S | 709.19706 | 2.0 | -1.4 |
| 2-1-m1E03-1-0142 | C36 H30 N4 O6 S3 | 710.13275 | 6.3 | -26.4 |
| 2-1-m1E03-1-0143 | C37 H28 F2 N4 05 S2 | 710.14692 | -3.8 | -0.8 |
| 2-1-m1E03-1-0144 | C37 H29 F3 N6 O4 S | 710.19231 | -17.7 | -4.5 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0145 | C41 H34 N4 04 S2 | 710.20215 | 2.6 | 3.1 |
| 2-1-m1E03-1-0146 | C40 H31 F N6 04 S | 710.21115 | -0.4 | 3.8 |
| 2-1-m1E03-1-0147 | C38 H38 N4 O6 S2 | 710.22328 | 13.1 | -2.0 |
| 2-1-m1E03-1-0148 | C35 H29 N5 O6 S3 | 711.12800 | -11.0 | 7.2 |
| 2-1-m1E03-1-0149 | C35 H29 N5 O6 S3 | 711.12800 | 12.1 | -10.8 |
| 2-1-m1E03-1-0150 | C36 H30 F N5 O6 S2 | 711.16215 | 2.5 | -0.4 |
| 2-1-m1E03-1-0151 | C39 H33 N7 O5 S | 711.22639 | -1.4 | -0.6 |
| 2-1-m1E03-1-0152 | C41 H37 N5 O5 S | 711.25154 | 0.2 | 3.7 |
| 2-1-m1E03-1-0153 | C34 H28 N6 O6 S3 | 712.12324 | 0.0 | 0.0 |
| 2-1-m1E03-1-0154 | C36 H29 F N4 O7 S2 | 712.14617 | -44.3 | 0.0 |
| 2-1-m1E03-1-0155 | C38 H32 N8 O5 S | 712.22164 | -4.8 | -2.9 |
| 2-1-m1E03-1-0156 | C35 H31 N5 O8 S2 | 713.16140 | 11.3 | 7.4 |
| 2-1-m1E03-1-0157 | C36 H29 F2 N5 O7 S | 713.17558 | 6.7 | 0.4 |
| 2-1-m1E03-1-0158 | C40 H35 N5 O6 S | 713.23080 | 0.0 | -2.4 |
| 2-1-m1E03-1-0159 | C34 H30 N6 O8 S2 | 714.15665 | -6.8 | -5.0 |
| 2-1-m1E03-1-0160 | C40 H31 F N4 O4 S2 | 714.17708 | 8.7 | 0.0 |
| 2-1-m1E03-1-0161 | C37 H35 F N4 O6 S2 | 714.19820 | 2.6 | -2.4 |
| 2-1-m1E03-1-0162 | C41 H35 F N4 O5 S | 714.23122 | 25.3 | -39.5 |
| 2-1-m1E03-1-0163 | C42 H42 N4 O5 S | 714.28759 | 0.0 | 0.0 |
| 2-1-m1E03-1-0164 | C36 H28 F3 N5 04 S2 | 715.15348 | -6.7 | -17.7 |
| 2-1-m1E03-1-0165 | C39 H33 N5 O5 S2 | 715.19231 | -9.7 | -1.9 |
| 2-1-m1E03-1-0166 | C36 H37 N5 O7 S2 | 715.21344 | -3.2 | 0.6 |
| 2-1-m1E03-1-0167 | C40 H37 N5 O6 S | 715.24645 | 6.2 | 5.4 |
| 2-1-m1E03-1-0168 | C38 H32 N6 O5 S2 | 716.18756 | -0.9 | 0.3 |
| 2-1-m1E03-1-0169 | C38 H32 N6 05 S2 | 716.18756 | 0.0 | 0.0 |
| 2-1-m1E03-1-0170 | C35 H36 N6 O7 S2 | 716.20869 | -4.5 | -1.8 |
| 2-1-m1E03-1-0171 | C40 H33 F N4 O6 S | 716.21048 | -2.7 | -1.2 |
| 2-1-m1E03-1-0172 | C40 H36 N4 O5 S2 | 716.21271 | -1.8 | 1.5 |
| 2-1-m1E03-1-0173 | C39 H36 N6 O6 S | 716.24170 | -1.9 | -0.3 |
| 2-1-m1E03-1-0174 | C39 H36 N6 O6 S | 716.24170 | 4.2 | 15.2 |
| 2-1-m1E03-1-0175 | C37 H31 N7 O5 S2 | 717.18281 | 12.1 | 15.9 |
| 2-1-m1E03-1-0176 | C39 H35 N5 O5 S2 | 717.20796 | 0.0 | 0.0 |
| 2-1-m1E03-1-0177 | C39 H35 N5 O7 S | 717.22572 | 0.0 | -8.3 |
| 2-1-m1E03-1-0178 | C36 H30 N8 O5 S2 | 718.17806 | 1.6 | 6.7 |
| 2-1-m1E03-1-0179 | C39 H34 N4 O6 S2 | 718.19198 | -8.6 | 5.6 |
| 2-1-m1E03-1-0180 | C39 H34 N4 O6 S2 | 718.19198 | 0.0 | -3.6 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0181 | C39 H34 N4 O6 S2 | 718.19198 | 8.0 | 0.0 |
| 2-1-m1E03-1-0182 | C38 H34 N6 O7 S | 718.22097 | 0.0 | 0.0 |
| 2-1-m1E03-1-0183 | C43 H34 N4 O5 S | 718.22499 | 49.2 | 0.0 |
| 2-1-m1E03-1-0184 | C41 H39 F N4 O5 S | 718.26252 | 0.0 | -23.5 |
| 2-1-m1E03-1-0185 | C38 H33 N5 O6 S2 | 719.18723 | 1.1 | 4.0 |
| 2-1-m1E03-1-0186 | C38 H33 N5 O6 S2 | 719.18723 | 6.2 | -2.1 |
| 2-1-m1E03-1-0187 | C38 H33 N5 O6 S2 | 719.18723 | 0.0 | 13.9 |
| 2-1-m1E03-1-0188 | C39 H31 F2 N5 O5 S | 719.20140 | -4.7 | -7.7 |
| 2-1-m1E03-1-0189 | C42 H33 N5 O5 S | 719.22024 | -1.6 | -3.0 |
| 2-1-m1E03-1-0190 | C39 H34 F N5 O6 S | 719.22138 | 0.0 | 0.0 |
| 2-1-m1E03-1-0191 | C40 H41 N5 O6 S | 719.27775 | 7.1 | 2.2 |
| 2-1-m1E03-1-0192 | C37 H32 N6 O6 S2 | 720.18247 | 4.1 | 0.0 |
| 2-1-m1E03-1-0193 | C37 H32 N6 O6 S2 | 720.18247 | -6.5 | 11.9 |
| 2-1-m1E03-1-0194 | C38 H33 F N6 O6 S | 720.21663 | -2.4 | 0.3 |
| 2-1-m1E03-1-0195 | C42 H36 N6 04 S | 720.25187 | -5.2 | 7.9 |
| 2-1-m1E03-1-0196 | C39 H40 N6 O6 S | 720.27300 | -4.3 | 4.8 |
| 2-1-m1E03-1-0197 | C38 H35 N5 O6 S2 | 721.20288 | 0.4 | -1.3 |
| 2-1-m1E03-1-0198 | C37 H30 N4 O8 S2 | 722.15051 | 6.9 | -14.2 |
| 2-1-m1E03-1-0199 | C38 H31 F N4 O6 S2 | 722.16690 | 7.1 | 0.0 |
| 2-1-m1E03-1-0200 | C38 H31 F N4 O6 S2 | 722.16690 | -0.7 | 0.4 |
| 2-1-m1E03-1-0201 | C39 H29 F3 N4 O5 S | 722.18108 | 1.7 | -0.1 |
| 2-1-m1E03-1-0202 | C38 H34 N4 O7 S2 | 722.18689 | -2.1 | 0.0 |
| 2-1-m1E03-1-0203 | C40 H33 F3 N4 04 S | 722.21746 | 1.5 | -11.0 |
| 2-1-m1E03-1-0204 | C41 H34 N6 05 S | 722.23114 | 4.9 | 1.9 |
| 2-1-m1E03-1-0205 | C35 H29 N7 O5 S3 | 723.13923 | 1.6 | 5.0 |
| 2-1-m1E03-1-0206 | C36 H29 N5 O8 S2 | 723.14575 | -3.8 | -2.3 |
| 2-1-m1E03-1-0207 | C37 H30 F N5 O6 S2 | 723.16215 | -2.1 | 0.7 |
| 2-1-m1E03-1-0208 | C37 H33 N5 O7 S2 | 723.18214 | 4.5 | 7.3 |
| 2-1-m1E03-1-0209 | C37 H33 N5 O7 S2 | 723.18214 | -3.1 | 6.2 |
| 2-1-m1E03-1-0210 | C37 H33 N5 O7 S2 | 723.18214 | -10.8 | -0.8 |
| 2-1-m1E03-1-0211 | C38 H31 F2 N5 O6 S | 723.19631 | 0.5 | -0.9 |
| 2-1-m1E03-1-0212 | C38 H31 F2 N5 O6 S | 723.19631 | -2.3 | 13.4 |
| 2-1-m1E03-1-0213 | C37 H32 N4 O6 S3 | 724.14840 | -2.8 | -2.0 |
| 2-1-m1E03-1-0214 | C36 H29 F N6 O6 S2 | 724.15740 | 3.3 | -6.5 |
| 2-1-m1E03-1-0215 | C37 H32 N4 O8 S2 | 724.16616 | -10.0 | 0.1 |
| 2-1-m1E03-1-0216 | C36 H32 N6 O7 S2 | 724.17739 | -14.0 | -2.4 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0217 | C36 H32 N6 O7 S2 | 724.17739 | 6.9 | 7.9 |
| 2-1-m1E03-1-0218 | C36 H32 N6 O7 S2 | 724.17739 | 10.4 | 1.3 |
| 2-1-m1E03-1-0219 | C41 H32 N4 O5 S2 | 724.18141 | -8.4 | -0.5 |
| 2-1-m1E03-1-0220 | C37 H30 F2 N6 O6 S | 724.19156 | 2.8 | 13.0 |
| 2-1-m1E03-1-0221 | C39 H31 F3 N4 O5 S | 724.19673 | 2.9 | -5.1 |
| 2-1-m1E03-1-0222 | C42 H36 N4 04 S2 | 724.21780 | 0.0 | 0.0 |
| 2-1-m1E03-1-0223 | C39 H40 N4 O6 S2 | 724.23893 | 6.2 | 7.7 |
| 2-1-m1E03-1-0224 | C36 H31 N5 O6 S3 | 725.14365 | -0.4 | 3.9 |
| 2-1-m1E03-1-0225 | C35 H31 N7 O7 S2 | 725.17264 | -7.7 | -0.6 |
| 2-1-m1E03-1-0226 | C35 H31 N7 O7 S2 | 725.17264 | 0.0 | 0.0 |
| 2-1-m1E03-1-0227 | C38 H30 F3 N5 O5 S | 725.19197 | 8.4 | 2.4 |
| 2-1-m1E03-1-0228 | C40 H35 N7 O5 S | 725.24204 | 3.9 | -0.5 |
| 2-1-m1E03-1-0229 | C34 H30 N8 O7 S2 | 726.16789 | 0.0 | 0.0 |
| 2-1-m1E03-1-0230 | C38 H29 F3 N4 O6 S | 726.17599 | 0.0 | -0.8 |
| 2-1-m1E03-1-0231 | C37 H29 F3 N6 O5 S | 726.18722 | -8.4 | 0.0 |
| 2-1-m1E03-1-0232 | C39 H30 F4 N4 O4 S | 726.19239 | 6.9 | -0.8 |
| 2-1-m1E03-1-0233 | C41 H34 N4 O5 S2 | 726.19706 | 8.4 | -1.6 |
| 2-1-m1E03-1-0234 | C38 H38 N4 O7 S2 | 726.21819 | 0.0 | 0.0 |
| 2-1-m1E03-1-0235 | C36 H33 N5 O8 S2 | 727.17705 | 6.9 | 11.4 |
| 2-1-m1E03-1-0236 | C38 H32 F3 N5 O5 S | 727.20762 | -3.0 | -4.0 |
| 2-1-m1E03-1-0237 | C38 H32 F3 N5 O5 S | 727.20762 | 0.0 | 6.9 |
| 2-1-m1E03-1-0238 | C36 H29 F N4 O6 S3 | 728.12332 | 0.0 | 0.0 |
| 2-1-m1E03-1-0239 | C40 H30 F2 N6 O4 S | 728.20173 | -0.4 | 3.0 |
| 2-1-m1E03-1-0240 | C37 H31 F3 N6 O5 S | 728.20287 | 5.2 | -1.3 |
| 2-1-m1E03-1-0241 | C37 H31 F3 N6 O5 S | 728.20287 | -7.6 | -9.9 |
| 2-1-m1E03-1-0242 | C43 H44 N4 O5 S | 728.30324 | 0.0 | 0.0 |
| 2-1-m1E03-1-0243 | C35 H28 F N5 O6 S3 | 729.11857 | 6.0 | 23.5 |
| 2-1-m1E03-1-0244 | C35 H31 N5 O7 S3 | 729.13856 | 8.6 | -3.4 |
| 2-1-m1E03-1-0245 | C36 H29 F2 N5 O6 S2 | 729.15273 | 8.8 | 3.9 |
| 2-1-m1E03-1-0246 | C36 H30 F3 N7 O5 S | 729.19812 | 9.9 | 2.6 |
| 2-1-m1E03-1-0247 | C40 H35 N5 O5 S2 | 729.20796 | -6.6 | -2.2 |
| 2-1-m1E03-1-0248 | C39 H32 F N7 O5 S | 729.21697 | 4.3 | 8.6 |
| 2-1-m1E03-1-0249 | C37 H39 N5 O7 S2 | 729.22909 | 0.1 | 0.8 |
| 2-1-m1E03-1-0250 | C34 H30 N6 O7 S3 | 730.13381 | -3.7 | 2.6 |
| 2-1-m1E03-1-0251 | C34 H30 N6 O7 S3 | 730.13381 | -3.9 | -3.1 |
| 2-1-m1E03-1-0252 | C36 H28 F2 N4 O7 S2 | 730.13675 | -3.7 | 2.7 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0253 | C41 H38 N4 O5 S2 | 730.22836 | 0.0 | 0.0 |
| 2-1-m1E03-1-0254 | C41 H38 N4 O5 S2 | 730.22836 | -11.5 | -6.6 |
| 2-1-m1E03-1-0255 | C40 H38 N6 O6 S | 730.25735 | 1.6 | 2.5 |
| 2-1-m1E03-1-0256 | C42 H42 N4 O6 S | 730.28251 | 0.0 | 0.0 |
| 2-1-m1E03-1-0257 | C33 H29 N7 O7 S3 | 731.12906 | 0.0 | 0.0 |
| 2-1-m1E03-1-0258 | C36 H28 F3 N5 05 S2 | 731.14840 | 3.3 | -20.8 |
| 2-1-m1E03-1-0259 | C35 H30 F N5 O8 S2 | 731.15198 | 4.6 | 0.9 |
| 2-1-m1E03-1-0260 | C40 H37 N5 05 S2 | 731.22361 | -6.8 | 5.2 |
| 2-1-m1E03-1-0261 | C40 H30 F2 N4 04 S2 | 732.16765 | -366.1 | 0.0 |
| 2-1-m1E03-1-0262 | C39 H32 N4 O7 S2 | 732.17124 | -2.8 | 6.2 |
| 2-1-m1E03-1-0263 | C37 H34 F2 N4 O6 S2 | 732.18878 | -0.1 | 4.2 |
| 2-1-m1E03-1-0264 | C40 H36 N4 O6 S2 | 732.20763 | 6.7 | 2.0 |
| 2-1-m1E03-1-0265 | C40 H36 N4 O6 S2 | 732.20763 | -1.8 | 0.0 |
| 2-1-m1E03-1-0266 | C40 H36 N4 O6 S2 | 732.20763 | 0.0 | 0.0 |
| 2-1-m1E03-1-0267 | C40 H36 N4 O6 S2 | 732.20763 | -21.3 | -16.5 |
| 2-1-m1E03-1-0268 | C39 H36 N6 O7 S | 732.23662 | -3.5 | 1.6 |
| 2-1-m1E03-1-0269 | C38 H31 N5 O7 S2 | 733.16649 | -1.9 | -0.1 |
| 2-1-m1E03-1-0270 | C38 H31 N5 O7 S2 | 733.16649 | 4.5 | 0.0 |
| 2-1-m1E03-1-0271 | C39 H32 F N5 O5 S2 | 733.18289 | 10.2 | 15.8 |
| 2-1-m1E03-1-0272 | C39 H35 N5 O6 S2 | 733.20288 | -1.5 | 3.0 |
| 2-1-m1E03-1-0273 | C39 H35 N5 O6 S2 | 733.20288 | -0.4 | -0.4 |
| 2-1-m1E03-1-0274 | C39 H35 N5 O6 S2 | 733.20288 | 4.1 | 2.7 |
| 2-1-m1E03-1-0275 | C36 H36 F N5 O7 S2 | 733.20402 | -1.5 | 3.0 |
| 2-1-m1E03-1-0276 | C43 H35 N5 O5 S | 733.23589 | 2.0 | 3.1 |
| 2-1-m1E03-1-0277 | C40 H36 F N5 O6 S | 733.23703 | 3.1 | 24.3 |
| 2-1-m1E03-1-0278 | C41 H43 N5 O6 S | 733.29340 | 7.5 | 2.1 |
| 2-1-m1E03-1-0279 | C35 H29 F3 N6 O5 S2 | 734.15929 | -10.7 | 3.6 |
| 2-1-m1E03-1-0280 | C37 H30 N6 O7 S2 | 734.16174 | -2.8 | 3.5 |
| 2-1-m1E03-1-0281 | C37 H30 N6 O7 S2 | 734.16174 | 1.8 | 1.0 |
| 2-1-m1E03-1-0282 | C39 H34 N4 O7 S2 | 734.18689 | 16.8 | -25.9 |
| 2-1-m1E03-1-0283 | C39 H34 N4 O7 S2 | 734.18689 | -3.4 | 0.0 |
| 2-1-m1E03-1-0284 | C38 H34 N6 O6 S2 | 734.19812 | 0.0 | 0.0 |
| 2-1-m1E03-1-0285 | C40 H35 F N4 O5 S2 | 734.20329 | 28.5 | -2.1 |
| 2-1-m1E03-1-0286 | C38 H33 N5 O7 S2 | 735.18214 | -6.9 | -0.1 |
| 2-1-m1E03-1-0287 | C39 H34 F N5 O7 S | 735.21630 | -4.3 | -2.8 |
| 2-1-m1E03-1-0288 | C39 H37 N5 O6 S2 | 735.21853 | -6.5 | 3.8 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0289 | C38 H32 N4 O8 S2 | 736.16616 | 0.0 | 0.0 |
| 2-1-m1E03-1-0290 | C37 H32 N6 O7 S2 | 736.17739 | 0.0 | 0.0 |
| 2-1-m1E03-1-0291 | C39 H33 F N4 O6 S2 | 736.18255 | -4.9 | -2.5 |
| 2-1-m1E03-1-0292 | C39 H33 F N4 O6 S2 | 736.18255 | 0.0 | 0.0 |
| 2-1-m1E03-1-0293 | C40 H31 F3 N4 O5 S | 736.19673 | -5.0 | -3.5 |
| 2-1-m1E03-1-0294 | C38 H36 N6 O6 S2 | 736.21377 | -2.5 | 3.6 |
| 2-1-m1E03-1-0295 | C43 H33 F N4 O5 S | 736.21557 | -4.1 | -21.9 |
| 2-1-m1E03-1-0296 | C41 H35 F3 N4 O4 S | 736.23311 | -11.0 | -2.1 |
| 2-1-m1E03-1-0297 | C41 H38 F2 N4 O5 S | 736.25310 | 28.2 | 8.8 |
| 2-1-m1E03-1-0298 | C38 H32 F N5 O6 S2 | 737.17780 | 4.4 | 6.6 |
| 2-1-m1E03-1-0299 | C38 H32 F N5 O6 S2 | 737.17780 | -3.5 | -15.6 |
| 2-1-m1E03-1-0300 | C39 H30 F3 N5 O5 S | 737.19197 | -9.0 | 20.9 |
| 2-1-m1E03-1-0301 | C38 H35 N5 O7 S2 | 737.19779 | 2.8 | -0.6 |
| 2-1-m1E03-1-0302 | C38 H35 N5 O7 S2 | 737.19779 | -0.3 | -0.5 |
| 2-1-m1E03-1-0303 | C38 H35 N5 O7 S2 | 737.19779 | 0.1 | 2.7 |
| 2-1-m1E03-1-0304 | C40 H40 F N5 O6 S | 737.26833 | 0.2 | -1.8 |
| 2-1-m1E03-1-0305 | C37 H30 N4 O7 S3 | 738.12766 | 0.0 | 1.4 |
| 2-1-m1E03-1-0306 | C38 H34 N4 O6 S3 | 738.16405 | 0.0 | 0.0 |
| 2-1-m1E03-1-0307 | C37 H34 N6 O7 S2 | 738.19304 | -10.5 | -2.8 |
| 2-1-m1E03-1-0308 | C37 H34 N6 O7 S2 | 738.19304 | -4.5 | -6.6 |
| 2-1-m1E03-1-0309 | C37 H34 N6 O7 S2 | 738.19304 | -0.3 | -1.8 |
| 2-1-m1E03-1-0310 | C38 H32 F2 N6 O6 S | 738.20721 | -6.5 | 2.0 |
| 2-1-m1E03-1-0311 | C40 H33 F3 N4 O5 S | 738.21238 | 8.4 | 6.8 |
| 2-1-m1E03-1-0312 | C36 H29 N5 O7 S3 | 739.12291 | 2.0 | -0.5 |
| 2-1-m1E03-1-0313 | C37 H33 N5 O6 S3 | 739.15930 | 0.0 | 0.0 |
| 2-1-m1E03-1-0314 | C36 H33 N7 O7 S2 | 739.18829 | 0.0 | 0.0 |
| 2-1-m1E03-1-0315 | C36 H33 N7 O7 S2 | 739.18829 | -7.2 | -2.2 |
| 2-1-m1E03-1-0316 | C41 H37 N7 O5 S | 739.25769 | 3.2 | 7.5 |
| 2-1-m1E03-1-0317 | C37 H29 F N4 O8 S2 | 740.14108 | -161.4 | 0.0 |
| 2-1-m1E03-1-0318 | C37 H32 N4 O7 S3 | 740.14331 | 0.0 | 0.0 |
| 2-1-m1E03-1-0319 | C38 H30 F2 N4 O6 S2 | 740.15748 | -5.4 | 0.0 |
| 2-1-m1E03-1-0320 | C38 H30 F2 N4 O6 S2 | 740.15748 | 13.0 | -10.2 |
| 2-1-m1E03-1-0321 | C36 H31 N5 O7 S3 | 741.13856 | 8.4 | 3.9 |
| 2-1-m1E03-1-0322 | C37 H29 F2 N5 O6 S2 | 741.15273 | -8.7 | -6.4 |
| 2-1-m1E03-1-0323 | C36 H31 N5 O9 S2 | 741.15632 | -2.3 | -0.7 |
| 2-1-m1E03-1-0324 | C37 H32 F N5 O7 S2 | 741.17272 | -2.9 | 2.2 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0325 | C37 H32 F N5 O7 S2 | 741.17272 | -1.9 | 0.9 |
| 2-1-m1E03-1-0326 | C38 H30 F3 N5 O6 S | 741.18689 | -0.2 | 5.0 |
| 2-1-m1E03-1-0327 | C37 H35 N5 O8 S2 | 741.19270 | -4.3 | 0.6 |
| 2-1-m1E03-1-0328 | C39 H34 F3 N5 O5 S | 741.22327 | -3.9 | -2.4 |
| 2-1-m1E03-1-0329 | C40 H35 N7 O6 S | 741.23695 | 6.2 | -13.3 |
| 2-1-m1E03-1-0330 | C36 H28 F2 N6 O6 S2 | 742.14798 | -0.1 | 6.5 |
| 2-1-m1E03-1-0331 | C35 H30 N6 O9 S2 | 742.15157 | -4.2 | -0.5 |
| 2-1-m1E03-1-0332 | C38 H29 F3 N4 O5 S2 | 742.15315 | -0.2 | 17.0 |
| 2-1-m1E03-1-0333 | C36 H31 F N6 O7 S2 | 742.16797 | -3.5 | -3.2 |
| 2-1-m1E03-1-0334 | C38 H29 F3 N4 O7 S | 742.17090 | -9.7 | -0.7 |
| 2-1-m1E03-1-0335 | C44 H34 N6 O4 S | 742.23622 | 0.0 | 3.4 |
| 2-1-m1E03-1-0336 | C36 H33 N5 O7 S3 | 743.15421 | -0.7 | -1.6 |
| 2-1-m1E03-1-0337 | C35 H30 F N7 O7 S2 | 743.16322 | 0.0 | 0.0 |
| 2-1-m1E03-1-0338 | C36 H33 N5 O9 S2 | 743.17197 | -4.1 | -4.6 |
| 2-1-m1E03-1-0339 | C38 H32 F3 N5 O6 S | 743.20254 | 5.6 | -4.5 |
| 2-1-m1E03-1-0340 | C41 H37 N5 O5 S2 | 743.22361 | -10.4 | 0.2 |
| 2-1-m1E03-1-0341 | C38 H41 N5 O7 S2 | 743.24474 | 15.2 | 7.4 |
| 2-1-m1E03-1-0342 | C35 H32 N6 O7 S3 | 744.14946 | 0.0 | -6.6 |
| 2-1-m1E03-1-0343 | C40 H32 N4 O7 S2 | 744.17124 | -2.6 | -4.1 |
| 2-1-m1E03-1-0344 | C39 H32 N6 O6 S2 | 744.18247 | 4.9 | 1.9 |
| 2-1-m1E03-1-0345 | C39 H29 F5 N4 O4 S | 744.18297 | -1.4 | -3.9 |
| 2-1-m1E03-1-0346 | C37 H31 F3 N6 O6 S | 744.19779 | 0.8 | 5.4 |
| 2-1-m1E03-1-0347 | C42 H40 N4 O5 S2 | 744.24401 | -40.3 | 15.1 |
| 2-1-m1E03-1-0348 | C42 H40 N4 O5 S2 | 744.24401 | 0.0 | 0.0 |
| 2-1-m1E03-1-0349 | C42 H40 N4 O5 S2 | 744.24401 | 0.0 | 0.0 |
| 2-1-m1E03-1-0350 | C38 H31 N7 O6 S2 | 745.17772 | -6.9 | -3.5 |
| 2-1-m1E03-1-0351 | C37 H30 F3 N5 O7 S | 745.18180 | 17.1 | 8.0 |
| 2-1-m1E03-1-0352 | C36 H30 F3 N7 O6 S | 745.19304 | -8.7 | -4.5 |
| 2-1-m1E03-1-0353 | C38 H31 F4 N5 O5 S | 745.19820 | -5.3 | 9.1 |
| 2-1-m1E03-1-0354 | C40 H35 N5 O6 S2 | 745.20288 | 6.6 | 7.9 |
| 2-1-m1E03-1-0355 | C37 H39 N5 O8 S2 | 745.22400 | 0.0 | 0.0 |
| 2-1-m1E03-1-0356 | C41 H39 N5 O5 S2 | 745.23926 | 0.0 | 7.2 |
| 2-1-m1E03-1-0357 | C36 H28 F2 N4 O6 S3 | 746.11390 | 4.6 | 0.0 |
| 2-1-m1E03-1-0358 | C40 H34 N4 O7 S2 | 746.18689 | 2.0 | 9.4 |
| 2-1-m1E03-1-0359 | C44 H34 N4 O4 S2 | 746.20215 | -1.9 | 0.3 |
| 2-1-m1E03-1-0360 | C41 H38 N4 O6 S2 | 746.22328 | 6.7 | 9.6 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0361 | C41 H38 N4 O6 S2 | 746.22328 | 0.0 | 0.0 |
| 2-1-m1E03-1-0362 | C41 H38 N4 O6 S2 | 746.22328 | 0.0 | 0.0 |
| 2-1-m1E03-1-0363 | C41 H38 N4 O6 S2 | 746.22328 | 7.0 | 8.2 |
| 2-1-m1E03-1-0364 | C35 H27 F2 N5 O6 S3 | 747.10915 | 4.3 | -3.4 |
| 2-1-m1E03-1-0365 | C35 H30 F N5 O7 S3 | 747.12914 | 8.7 | -1.8 |
| 2-1-m1E03-1-0366 | C39 H33 N5 O7 S2 | 747.18214 | -0.9 | 9.9 |
| 2-1-m1E03-1-0367 | C39 H33 N5 O7 S2 | 747.18214 | 0.0 | 0.0 |
| 2-1-m1E03-1-0368 | C39 H31 F2 N7 O5 S | 747.20754 | -2.7 | 2.7 |
| 2-1-m1E03-1-0369 | C40 H37 N5 O6 S2 | 747.21853 | 10.6 | 7.3 |
| 2-1-m1E03-1-0370 | C42 H45 N5 O6 S | 747.30905 | 4.1 | -1.7 |
| 2-1-m1E03-1-0371 | C34 H29 F N6 O7 S3 | 748.12439 | -1.4 | 0.0 |
| 2-1-m1E03-1-0372 | C38 H32 N6 O7 S2 | 748.17739 | -1.8 | 2.1 |
| 2-1-m1E03-1-0373 | C40 H36 N4 O7 S2 | 748.20254 | -8.7 | -14.4 |
| 2-1-m1E03-1-0374 | C40 H36 N4 O7 S2 | 748.20254 | 15.5 | 3.4 |
| 2-1-m1E03-1-0375 | C41 H37 F N4 O5 S2 | 748.21894 | 0.0 | 0.0 |
| 2-1-m1E03-1-0376 | C35 H29 F2 N5 O8 S2 | 749.14256 | 5.5 | 4.9 |
| 2-1-m1E03-1-0377 | C39 H35 N5 O7 S2 | 749.19779 | 0.0 | -4.1 |
| 2-1-m1E03-1-0378 | C39 H35 N5 O7 S2 | 749.19779 | 21.5 | 0.0 |
| 2-1-m1E03-1-0379 | C40 H39 N5 O6 S2 | 749.23418 | 10.6 | 0.0 |
| 2-1-m1E03-1-0380 | C40 H39 N5 O6 S2 | 749.23418 | -4.8 | -1.0 |
| 2-1-m1E03-1-0381 | C41 H43 N5 O7 S | 749.28832 | -3.0 | 3.8 |
| 2-1-m1E03-1-0382 | C37 H30 N6 O6 S3 | 750.13889 | 0.7 | 13.3 |
| 2-1-m1E03-1-0383 | C35 H29 F3 N6 O6 S2 | 750.15421 | 2.3 | -1.2 |
| 2-1-m1E03-1-0384 | C39 H31 F N4 O7 S2 | 750.16182 | 4.3 | 0.0 |
| 2-1-m1E03-1-0385 | C39 H31 F N4 O7 S2 | 750.16182 | 0.0 | 0.0 |
| 2-1-m1E03-1-0386 | C39 H34 N4 O8 S2 | 750.18181 | -4.9 | -2.5 |
| 2-1-m1E03-1-0387 | C40 H35 F N4 O6 S2 | 750.19820 | 14.1 | 0.0 |
| 2-1-m1E03-1-0388 | C40 H35 F N4 O6 S2 | 750.19820 | 0.0 | 0.0 |
| 2-1-m1E03-1-0389 | C39 H38 N6 O6 S2 | 750.22942 | 0.0 | 0.0 |
| 2-1-m1E03-1-0390 | C45 H42 N4 O5 S | 750.28759 | -37.8 | 10.3 |
| 2-1-m1E03-1-0391 | C38 H30 F N5 O7 S2 | 751.15707 | -2.9 | -2.3 |
| 2-1-m1E03-1-0392 | C38 H30 F N5 O7 S2 | 751.15707 | 0.0 | 0.0 |
| 2-1-m1E03-1-0393 | C39 H31 F2 N5 O5 S2 | 751.17347 | 0.0 | 0.0 |
| 2-1-m1E03-1-0394 | C38 H33 N5 O8 S2 | 751.17705 | -5.7 | -0.7 |
| 2-1-m1E03-1-0395 | C36 H35 F2 N5 O7 S2 | 751.19460 | -5.3 | -0.9 |
| 2-1-m1E03-1-0396 | C40 H32 F3 N5 O5 S | 751.20762 | 1.5 | -5.4 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0397 | C39 H37 N5 O7 S2 | 751.21344 | 5.8 | 3.0 |
| 2-1-m1E03-1-0398 | C39 H37 N5 O7 S2 | 751.21344 | -2.4 | -1.1 |
| 2-1-m1E03-1-0399 | C39 H37 N5 O7 S2 | 751.21344 | 2.8 | 2.1 |
| 2-1-m1E03-1-0400 | C39 H37 N5 O7 S2 | 751.21344 | -2.6 | 2.6 |
| 2-1-m1E03-1-0401 | C38 H32 N4 O7 S3 | 752.14331 | 23.9 | 8.5 |
| 2-1-m1E03-1-0402 | C38 H32 N4 O9 S2 | 752.16107 | -15.8 | -23.1 |
| 2-1-m1E03-1-0403 | C37 H32 N6 O8 S2 | 752.17230 | -4.5 | -1.5 |
| 2-1-m1E03-1-0404 | C37 H32 N6 O8 S2 | 752.17230 | -1.5 | -0.3 |
| 2-1-m1E03-1-0405 | C40 H31 F3 N4 O6 S | 752.19164 | 7.1 | -8.1 |
| 2-1-m1E03-1-0406 | C40 H34 F2 N4 O5 S2 | 752.19387 | -2.8 | -2.5 |
| 2-1-m1E03-1-0407 | C38 H36 N6 O7 S2 | 752.20869 | -0.9 | 1.9 |
| 2-1-m1E03-1-0408 | C38 H36 N6 O7 S2 | 752.20869 | 6.1 | -1.7 |
| 2-1-m1E03-1-0409 | C38 H36 N6 O7 S2 | 752.20869 | -8.1 | -0.6 |
| 2-1-m1E03-1-0410 | C36 H31 N7 O8 S2 | 753.16755 | -0.8 | 0.1 |
| 2-1-m1E03-1-0411 | C39 H30 F3 N5 O6 S | 753.18689 | -10.3 | -0.6 |
| 2-1-m1E03-1-0412 | C38 H35 N5 O8 S2 | 753.19270 | -0.7 | 4.9 |
| 2-1-m1E03-1-0413 | C38 H35 N5 O8 S2 | 753.19270 | -6.3 | 22.1 |
| 2-1-m1E03-1-0414 | C37 H35 N7 O7 S2 | 753.20394 | 0.0 | 0.0 |
| 2-1-m1E03-1-0415 | C39 H36 F N5 O6 S2 | 753.20910 | -5.3 | -1.7 |
| 2-1-m1E03-1-0416 | C39 H32 F2 N4 O6 S2 | 754.17313 | -0.6 | -0.9 |
| 2-1-m1E03-1-0417 | C39 H32 F2 N4 O6 S2 | 754.17313 | 0.0 | -1.4 |
| 2-1-m1E03-1-0418 | C40 H30 F4 N4 O5 S | 754.18730 | -0.8 | 9.8 |
| 2-1-m1E03-1-0419 | C37 H34 N6 O8 S2 | 754.18795 | -9.8 | -2.7 |
| 2-1-m1E03-1-0420 | C42 H34 N4 O6 S2 | 754.19198 | -11.8 | -3.5 |
| 2-1-m1E03-1-0421 | C42 H34 N4 O6 S2 | 754.19198 | -30.4 | 6.7 |
| 2-1-m1E03-1-0422 | C38 H31 F2 N5 O6 S2 | 755.16838 | 0.0 | 0.0 |
| 2-1-m1E03-1-0423 | C37 H33 N5 O9 S2 | 755.17197 | -1.3 | -3.6 |
| 2-1-m1E03-1-0424 | C36 H33 N7 O8 S2 | 755.18320 | 3.0 | 7.9 |
| 2-1-m1E03-1-0425 | C41 H33 N5 O6 S2 | 755.18723 | -3.3 | -3.1 |
| 2-1-m1E03-1-0426 | C38 H34 F N5 O7 S2 | 755.18837 | -2.3 | 2.6 |
| 2-1-m1E03-1-0427 | C38 H34 F N5 O7 S2 | 755.18837 | -7.4 | -12.1 |
| 2-1-m1E03-1-0428 | C39 H32 F3 N5 O6 S | 755.20254 | -11.3 | 15.6 |
| 2-1-m1E03-1-0429 | C40 H36 F3 N5 O5 S | 755.23892 | 11.3 | 11.4 |
| 2-1-m1E03-1-0430 | C40 H39 F2 N5 O6 S | 755.25891 | 0.0 | 0.0 |
| 2-1-m1E03-1-0431 | C37 H29 F N4 O7 S3 | 756.11824 | 0.0 | 0.0 |
| 2-1-m1E03-1-0432 | C40 H32 N6 O6 S2 | 756.18247 | -19.8 | 7.3 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0433 | C40 H32 N6 O6 S2 | 756.18247 | 0.0 | 0.0 |
| 2-1-m1E03-1-0434 | C37 H33 F N6 O7 S2 | 756.18362 | -18.5 | 5.6 |
| 2-1-m1E03-1-0435 | C37 H33 F N6 O7 S2 | 756.18362 | 0.0 | 0.0 |
| 2-1-m1E03-1-0436 | C38 H31 F3 N6 O6 S | 756.19779 | 11.0 | 4.6 |
| 2-1-m1E03-1-0437 | C36 H31 N5 O8 S3 | 757.13348 | -4.4 | 1.9 |
| 2-1-m1E03-1-0438 | C37 H35 N5 O7 S3 | 757.16986 | -6.2 | -5.3 |
| 2-1-m1E03-1-0439 | C39 H31 N7 O6 S2 | 757.17772 | -3.8 | -3.2 |
| 2-1-m1E03-1-0440 | C39 H34 F3 N5 O6 S | 757.21819 | 0.0 | 0.0 |
| 2-1-m1E03-1-0441 | C36 H30 N4 O9 S3 | 758.11749 | 1.4 | -1.5 |
| 2-1-m1E03-1-0442 | C35 H30 N6 O8 S3 | 758.12872 | -6.1 | -0.9 |
| 2-1-m1E03-1-0443 | C37 H28 F2 N4 O8 S2 | 758.13166 | -0.3 | -1.7 |
| 2-1-m1E03-1-0444 | C38 H29 F3 N4 O6 S2 | 758.14806 | 5.4 | -12.0 |
| 2-1-m1E03-1-0445 | C36 H34 N6 O7 S3 | 758.16511 | 0.0 | -1.2 |
| 2-1-m1E03-1-0446 | C41 H34 N4 O7 S2 | 758.18689 | 0.0 | 0.0 |
| 2-1-m1E03-1-0447 | C43 H33 F3 N4 O4 S | 758.21746 | -10.2 | -9.7 |
| 2-1-m1E03-1-0448 | C43 H42 N4 05 S2 | 758.25966 | 0.0 | 0.0 |
| 2-1-m1E03-1-0449 | C43 H42 N4 05 S2 | 758.25966 | 5.4 | 0.0 |
| 2-1-m1E03-1-0450 | C35 H29 N5 O9 S3 | 759.11274 | -5.4 | -1.7 |
| 2-1-m1E03-1-0451 | C36 H30 F N5 O9 S2 | 759.14690 | -1.8 | -1.9 |
| 2-1-m1E03-1-0452 | C36 H33 N5 O8 S3 | 759.14913 | 0.0 | 0.0 |
| 2-1-m1E03-1-0453 | C37 H31 F2 N5 O7 S2 | 759.16330 | -5.3 | 1.3 |
| 2-1-m1E03-1-0454 | C37 H31 F2 N5 O7 S2 | 759.16330 | 2.3 | 0.1 |
| 2-1-m1E03-1-0455 | C40 H33 N5 O7 S2 | 759.18214 | -2.8 | -8.0 |
| 2-1-m1E03-1-0456 | C39 H33 N7 O6 S2 | 759.19337 | -2.8 | -1.7 |
| 2-1-m1E03-1-0457 | C35 H32 N6 O8 S3 | 760.14437 | 8.3 | 0.0 |
| 2-1-m1E03-1-0458 | C40 H32 N4 O6 S3 | 760.14840 | 0.1 | 0.0 |
| 2-1-m1E03-1-0459 | C40 H32 N4 O6 S3 | 760.14840 | 15.0 | -76.5 |
| 2-1-m1E03-1-0460 | C40 H32 N4 O6 S3 | 760.14840 | 0.0 | 0.0 |
| 2-1-m1E03-1-0461 | C36 H30 F2 N6 O7 S2 | 760.15855 | -3.1 | 2.8 |
| 2-1-m1E03-1-0462 | C41 H36 N4 O7 S2 | 760.20254 | 0.0 | 0.0 |
| 2-1-m1E03-1-0463 | C41 H31 F3 N6 O4 S | 760.20796 | 3.1 | -4.1 |
| 2-1-m1E03-1-0464 | C42 H40 N4 O6 S2 | 760.23893 | 7.2 | -15.7 |
| 2-1-m1E03-1-0465 | C42 H40 N4 O6 S2 | 760.23893 | 0.0 | 0.0 |
| 2-1-m1E03-1-0466 | C39 H31 N5 O6 S3 | 761.14365 | 0.3 | 4.8 |
| 2-1-m1E03-1-0467 | C39 H31 N5 O6 S3 | 761.14365 | 0.6 | -18.2 |
| 2-1-m1E03-1-0468 | C39 H31 N5 O6 S3 | 761.14365 | 0.0 | 0.0 |

(continued)

[Table 11-2-3]

| | | | AS-MS ratio (%) | |
|---|---|---|---|---|
| ID | Molecular Formula | Exact Mass | N1 | N2 |
| 2-1-m1E03-1-0469 | C35 H29 F2 N7 O7 S2 | 761.15379 | 0.0 | 0.0 |
| 2-1-m1E03-1-0470 | C37 H30 F3 N5 O6 S2 | 761.15896 | 1.2 | 9.9 |
| 2-1-m1E03-1-0471 | C37 H30 F3 N5 O8 S | 761.17672 | 0.0 | 1.5 |
| 2-1-m1E03-1-0472 | C40 H35 N5 O7 S2 | 761.19779 | -6.2 | 0.0 |
| 2-1-m1E03-1-0473 | C40 H35 N5 O7 S2 | 761.19779 | 0.0 | 0.0 |
| 2-1-m1E03-1-0474 | C37 H29 F3 N4 O7 S2 | 762.14298 | 10.0 | 7.6 |
| 2-1-m1E03-1-0475 | C39 H31 F N6 O6 S2 | 762.17305 | 2.1 | 4.7 |
| 2-1-m1E03-1-0476 | C40 H34 N4 O8 S2 | 762.18181 | 22.4 | -6.5 |
| 2-1-m1E03-1-0477 | C41 H38 N4 O7 S2 | 762.21819 | 6.5 | -4.5 |
| 2-1-m1E03-1-0478 | C41 H38 N4 O7 S2 | 762.21819 | -17.4 | 0.0 |
| 2-1-m1E03-1-0479 | C42 H39 F N4 O5 S2 | 762.23459 | 0.0 | 0.0 |
| 2-1-m1E03-1-0480 | C39 H33 N5 O8 S2 | 763.17705 | 1.6 | 3.8 |
| 2-1-m1E03-1-0481 | C38 H30 F5 N5 O5 S | 763.18878 | -5.0 | 18.3 |
| 2-1-m1E03-1-0482 | C41 H41 N5 O6 S2 | 763.24983 | -8.2 | 0.0 |
| 2-1-m1E03-1-0483 | C41 H41 N5 O6 S2 | 763.24983 | 3.6 | 0.3 |
| 2-1-m1E03-1-0484 | C41 H41 N5 O6 S2 | 763.24983 | -7.6 | -1.0 |
| 2-1-m1E03-1-0485 | C41 H31 F3 N4 O4 S2 | 764.17388 | 11.4 | 0.8 |
| 2-1-m1E03-1-0486 | C40 H33 F N4 O7 S2 | 764.17747 | 0.8 | 1.7 |
| 2-1-m1E03-1-0487 | C38 H35 F3 N4 O6 S2 | 764.19501 | -2.1 | -0.2 |
| 2-1-m1E03-1-0488 | C41 H37 F N4 O6 S2 | 764.21385 | 11.3 | 2.0 |
| 2-1-m1E03-1-0489 | C41 H40 N4 O7 S2 | 764.23384 | 0.0 | 5.0 |
| 2-1-m1E03-1-0490 | C40 H40 N6 O6 S2 | 764.24507 | 0.0 | 0.0 |
| 2-1-m1E03-1-0491 | C35 H29 F2 N5 O7 S3 | 765.11972 | -3.7 | -2.4 |
| 2-1-m1E03-1-0492 | C39 H32 F N5 O7 S2 | 765.17272 | 0.0 | 0.0 |
| 2-1-m1E03-1-0493 | C39 H35 N5 O8 S2 | 765.19270 | -5.4 | -1.5 |
| 2-1-m1E03-1-0494 | C40 H39 N5 O7 S2 | 765.22909 | -3.0 | -0.6 |
| 2-1-m1E03-1-0495 | C40 H39 N5 O7 S2 | 765.22909 | 1.7 | 7.4 |
| 2-1-m1E03-1-0496 | C40 H39 N5 O7 S2 | 765.22909 | 0.0 | 0.0 |
| 2-1-m1E03-1-0497 | C40 H39 N5 O7 S2 | 765.22909 | -2.1 | 1.1 |
| 2-1-m1E03-1-0498 | C34 H28 F2 N6 O7 S3 | 766.11497 | 0.0 | 0.0 |
| 2-1-m1E03-1-0499 | C39 H34 N4 O7 S3 | 766.15896 | 0.0 | 0.0 |
| 2-1-m1E03-1-0500 | C38 H34 N6 O8 S2 | 766.18795 | -1.8 | 0.0 |
| 2-1-m1E03-1-0501 | C38 H34 N6 O8 S2 | 766.18795 | -2.6 | 0.4 |
| 2-1-m1E03-1-0502 | C40 H35 F N4 O7 S2 | 766.19312 | 30.1 | 1.3 |
| 2-1-m1E03-1-0503 | C40 H35 F N4 O7 S2 | 766.19312 | -0.7 | -0.7 |
| 2-1-m1E03-1-0504 | C41 H36 F2 N4 O5 S2 | 766.20952 | -5.9 | -16.2 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0505 | C39 H38 N6 O7 S2 | 766.22434 | 0.0 | 0.0 |
| 2-1-m1E03-1-0506 | C44 H38 N4 O5 S2 | 766.22836 | 0.0 | 0.0 |
| 2-1-m1E03-1-0507 | C37 H33 N7 O8 S2 | 767.18320 | -4.0 | 0.9 |
| 2-1-m1E03-1-0508 | C40 H32 F3 N5 O6 S | 767.20254 | 1.1 | 17.4 |
| 2-1-m1E03-1-0509 | C39 H37 N5 O8 S2 | 767.20835 | -3.3 | -0.6 |
| 2-1-m1E03-1-0510 | C39 H37 N5 O8 S2 | 767.20835 | 0.0 | 46.4 |
| 2-1-m1E03-1-0511 | C40 H38 F N5 O6 S2 | 767.22475 | 10.5 | 4.8 |
| 2-1-m1E03-1-0512 | C38 H32 N4 O8 S3 | 768.13823 | 0.0 | 0.0 |
| 2-1-m1E03-1-0513 | C38 H32 N4 O8 S3 | 768.13823 | 0.0 | 0.0 |
| 2-1-m1E03-1-0514 | C39 H30 F2 N4 O7 S2 | 768.15240 | -4.0 | 4.2 |
| 2-1-m1E03-1-0515 | C39 H30 F2 N4 O7 S2 | 768.15240 | 3.9 | 0.0 |
| 2-1-m1E03-1-0516 | C40 H34 F2 N4 O6 S2 | 768.18878 | -0.8 | 0.6 |
| 2-1-m1E03-1-0517 | C40 H32 N8 O5 S2 | 768.19371 | 12.9 | 0.0 |
| 2-1-m1E03-1-0518 | C38 H36 N6 O8 S2 | 768.20360 | 44.7 | -22.8 |
| 2-1-m1E03-1-0519 | C38 H36 N6 O8 S2 | 768.20360 | 4.8 | 0.0 |
| 2-1-m1E03-1-0520 | C43 H36 N4 O6 S2 | 768.20763 | 0.0 | 0.0 |
| 2-1-m1E03-1-0521 | C42 H39 F3 N4 O5 S | 768.25933 | 36.7 | 51.7 |
| 2-1-m1E03-1-0522 | C37 H31 N5 O8 S3 | 769.13348 | 0.0 | -2.1 |
| 2-1-m1E03-1-0523 | C38 H29 F2 N5 O7 S2 | 769.14765 | 1.1 | -4.1 |
| 2-1-m1E03-1-0524 | C38 H32 F N5 O8 S2 | 769.16763 | -0.5 | -0.8 |
| 2-1-m1E03-1-0525 | C38 H32 F N5 O8 S2 | 769.16763 | 0.0 | 0.0 |
| 2-1-m1E03-1-0526 | C38 H35 N5 O9 S2 | 769.18762 | -0.9 | 0.0 |
| 2-1-m1E03-1-0527 | C42 H35 N5 O6 S2 | 769.20288 | 0.9 | 2.4 |
| 2-1-m1E03-1-0528 | C39 H36 F N5 O7 S2 | 769.20402 | 0.0 | -1.1 |
| 2-1-m1E03-1-0529 | C39 H36 F N5 O7 S2 | 769.20402 | -0.9 | -0.8 |
| 2-1-m1E03-1-0530 | C36 H30 N6 O8 S3 | 770.12872 | 19.1 | 12.1 |
| 2-1-m1E03-1-0531 | C37 H31 F N6 O8 S2 | 770.16288 | -1.3 | 0.9 |
| 2-1-m1E03-1-0532 | C37 H31 F N6 O8 S2 | 770.16288 | 5.5 | 0.6 |
| 2-1-m1E03-1-0533 | C40 H30 F4 N4 O6 S | 770.18222 | 4.7 | -8.9 |
| 2-1-m1E03-1-0534 | C41 H34 N6 O6 S2 | 770.19812 | -9.1 | 1.7 |
| 2-1-m1E03-1-0535 | C39 H33 F3 N6 O6 S | 770.21344 | 7.5 | 3.0 |
| 2-1-m1E03-1-0536 | C37 H33 N5 O8 S3 | 771.14913 | 0.0 | 0.0 |
| 2-1-m1E03-1-0537 | C37 H33 N5 O10 S2 | 771.16688 | -1.7 | -1.7 |
| 2-1-m1E03-1-0538 | C39 H32 F3 N5 O7 S | 771.19745 | 6.0 | 15.6 |
| 2-1-m1E03-1-0539 | C39 H35 F2 N5 O6 S2 | 771.19968 | -6.8 | 0.0 |
| 2-1-m1E03-1-0540 | C37 H32 N4 O9 S3 | 772.13314 | 0.0 | -20.7 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0541 | C40 H32 N6 O5 S3 | 772.15963 | 0.0 | -30.5 |
| 2-1-m1E03-1-0542 | C39 H31 F3 N4 O6 S2 | 772.16371 | 0.0 | 3.5 |
| 2-1-m1E03-1-0543 | C39 H31 F3 N4 O6 S2 | 772.16371 | -5.3 | -0.9 |
| 2-1-m1E03-1-0544 | C39 H31 F3 N4 O6 S2 | 772.16371 | 3.8 | 6.9 |
| 2-1-m1E03-1-0545 | C39 H31 F3 N4 O6 S2 | 772.16371 | 45.2 | -6.8 |
| 2-1-m1E03-1-0546 | C41 H32 N4 O8 S2 | 772.16616 | 0.0 | 3.5 |
| 2-1-m1E03-1-0547 | C37 H36 N6 O7 S3 | 772.18076 | 1.8 | -1.8 |
| 2-1-m1E03-1-0548 | C38 H31 F3 N6 O7 S | 772.19270 | 2.7 | -2.8 |
| 2-1-m1E03-1-0549 | C42 H36 N4 O7 S2 | 772.20254 | 7.0 | 16.1 |
| 2-1-m1E03-1-0550 | C43 H40 N4 O6 S2 | 772.23893 | 5.9 | -0.9 |
| 2-1-m1E03-1-0551 | C44 H44 N4 O5 S2 | 772.27531 | 1023.2 | 289.9 |
| 2-1-m1E03-1-0552 | C38 H30 F3 N5 O6 S2 | 773.15896 | 7.6 | 0.0 |
| 2-1-m1E03-1-0553 | C38 H30 F3 N5 O6 S2 | 773.15896 | -6.1 | -11.9 |
| 2-1-m1E03-1-0554 | C38 H33 F2 N5 O7 S2 | 773.17895 | -15.4 | 14.8 |
| 2-1-m1E03-1-0555 | C38 H33 F2 N5 O7 S2 | 773.17895 | 0.0 | 0.0 |
| 2-1-m1E03-1-0556 | C39 H31 F4 N5 O6 S | 773.19312 | 2.3 | 24.1 |
| 2-1-m1E03-1-0557 | C42 H39 N5 O6 S2 | 773.23418 | -1.0 | -1.8 |
| 2-1-m1E03-1-0558 | C36 H30 N4 O8 S4 | 774.09465 | -2.6 | 7.5 |
| 2-1-m1E03-1-0559 | C37 H28 F2 N4 O7 S3 | 774.10882 | 1.4 | 17.9 |
| 2-1-m1E03-1-0560 | C37 H29 F3 N6 O6 S2 | 774.15421 | 5.4 | -9.9 |
| 2-1-m1E03-1-0561 | C41 H34 N4 O6 S3 | 774.16405 | 0.1 | 4.7 |
| 2-1-m1E03-1-0562 | C41 H34 N4 O6 S3 | 774.16405 | 0.0 | 0.0 |
| 2-1-m1E03-1-0563 | C40 H31 FN6 O6 S2 | 774.17305 | -2.0 | 17.1 |
| 2-1-m1E03-1-0564 | C37 H32 F2 N6 O7 S2 | 774.17420 | 1.2 | 12.6 |
| 2-1-m1E03-1-0565 | C43 H42 N4 O6 S2 | 774.25458 | 0.0 | 0.0 |
| 2-1-m1E03-1-0566 | C35 H29 N5 O8 S4 | 775.08990 | 0.7 | -0.1 |
| 2-1-m1E03-1-0567 | C36 H30 F N5 O8 S3 | 775.12405 | -6.4 | -0.8 |
| 2-1-m1E03-1-0568 | C40 H33 N5 O6 S3 | 775.15930 | 0.0 | 0.0 |
| 2-1-m1E03-1-0569 | C39 H33 N7 O7 S2 | 775.18829 | -5.6 | -1.4 |
| 2-1-m1E03-1-0570 | C41 H37 N5 O7 S2 | 775.21344 | -0.7 | -11.0 |
| 2-1-m1E03-1-0571 | C36 H29 F N4 O9 S3 | 776.10807 | -3.2 | 5.3 |
| 2-1-m1E03-1-0572 | C39 H32 N6 O6 S3 | 776.15454 | 0.0 | 0.0 |
| 2-1-m1E03-1-0573 | C41 H33 F N4 O7 S2 | 776.17747 | -9.3 | 0.0 |
| 2-1-m1E03-1-0574 | C38 H32 N8 O7 S2 | 776.18354 | 0.0 | 0.0 |
| 2-1-m1E03-1-0575 | C40 H30 F6 N4 O4 S | 776.18920 | 9.8 | -5.2 |
| 2-1-m1E03-1-0576 | C41 H31 F3 N6 O5 S | 776.20287 | 0.0 | 6.3 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0577 | C42 H40 N4 O7 S2 | 776.23384 | 0.0 | 0.0 |
| 2-1-m1E03-1-0578 | C41 H40 N6 O6 S2 | 776.24507 | 4.4 | 4.9 |
| 2-1-m1E03-1-0579 | C35 H31 N5 O10 S3 | 777.12330 | 0.0 | 0.0 |
| 2-1-m1E03-1-0580 | C36 H29 F2 N5 O9 S2 | 777.13748 | -11.1 | -7.6 |
| 2-1-m1E03-1-0581 | C37 H30 F3 N5 O7 S2 | 777.15387 | 6.5 | -2.1 |
| 2-1-m1E03-1-0582 | C40 H35 N5 O8 S2 | 777.19270 | 1.6 | -0.9 |
| 2-1-m1E03-1-0583 | C40 H35 N5 O8 S2 | 777.19270 | 0.0 | 0.0 |
| 2-1-m1E03-1-0584 | C42 H43 N5 O6 S2 | 777.26548 | -31.1 | 4.4 |
| 2-1-m1E03-1-0585 | C42 H43 N5 O6 S2 | 777.26548 | -14.1 | 21.1 |
| 2-1-m1E03-1-0586 | C34 H30 N6 O10 S3 | 778.11855 | -2.2 | 0.0 |
| 2-1-m1E03-1-0587 | C37 H29 F3 N4 O6 S3 | 778.12013 | -2.2 | 0.0 |
| 2-1-m1E03-1-0588 | C37 H29 F3 N4 O8 S2 | 778.13789 | 9.7 | 0.0 |
| 2-1-m1E03-1-0589 | C40 H31 F N4 O6 S3 | 778.13898 | -73.3 | 0.0 |
| 2-1-m1E03-1-0590 | C40 H31 F N4 O6 S3 | 778.13898 | -13.2 | 0.0 |
| 2-1-m1E03-1-0591 | C39 H34 N6 O8 S2 | 778.18795 | -1.7 | 3.3 |
| 2-1-m1E03-1-0592 | C41 H35 F N4 O7 S2 | 778.19312 | -14.1 | 2.4 |
| 2-1-m1E03-1-0593 | C41 H38 N4 O8 S2 | 778.21311 | 1.1 | 5.2 |
| 2-1-m1E03-1-0594 | C42 H42 N4 O7 S2 | 778.24949 | 0.0 | 0.0 |
| 2-1-m1E03-1-0595 | C36 H28 F3 N5 O6 S3 | 779.11538 | 9.5 | -4.3 |
| 2-1-m1E03-1-0596 | C39 H33 N5 O7 S3 | 779.15421 | -3.3 | -5.0 |
| 2-1-m1E03-1-0597 | C39 H33 N5 O7 S3 | 779.15421 | 0.0 | 7.3 |
| 2-1-m1E03-1-0598 | C40 H37 N5 O8 S2 | 779.20835 | -6.8 | -1.2 |
| 2-1-m1E03-1-0599 | C40 H32 F3 N7 O5 S | 779.21377 | 7.7 | -8.5 |
| 2-1-m1E03-1-0600 | C41 H41 N5 O7 S2 | 779.24474 | 1.5 | 4.4 |
| 2-1-m1E03-1-0601 | C41 H41 N5 O7 S2 | 779.24474 | 26.1 | 0.0 |
| 2-1-m1E03-1-0602 | C38 H32 N6 O7 S3 | 780.14946 | -0.9 | 0.6 |
| 2-1-m1E03-1-0603 | C38 H32 N6 O7 S3 | 780.14946 | -2.0 | -1.0 |
| 2-1-m1E03-1-0604 | C38 H32 N6 O7 S3 | 780.14946 | -1.6 | 0.8 |
| 2-1-m1E03-1-0605 | C38 H32 N6 O7 S3 | 780.14946 | 17.7 | 0.0 |
| 2-1-m1E03-1-0606 | C41 H31 F3 N4 O5 S2 | 780.16880 | 0.0 | 14.9 |
| 2-1-m1E03-1-0607 | C40 H33 F N4 O8 S2 | 780.17238 | -6.5 | 0.0 |
| 2-1-m1E03-1-0608 | C38 H35 F3 N4 O7 S2 | 780.18993 | 4.4 | 0.0 |
| 2-1-m1E03-1-0609 | C39 H36 N6 O8 S2 | 780.20360 | -4.4 | -1.8 |
| 2-1-m1E03-1-0610 | C39 H36 N6 O8 S2 | 780.20360 | 0.0 | 0.0 |
| 2-1-m1E03-1-0611 | C42 H38 F2 N4 O5 S2 | 780.22517 | 0.0 | 6.9 |
| 2-1-m1E03-1-0612 | C45 H40 N4 O5 S2 | 780.24401 | -5.3 | 0.0 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0613 | C37 H31 N7 O7 S3 | 781.14471 | 2.4 | 2.1 |
| 2-1-m1E03-1-0614 | C36 H30 F3 N5 O8 S2 | 781.14879 | 58.4 | -6.7 |
| 2-1-m1E03-1-0615 | C39 H35 N5 O9 S2 | 781.18762 | 0.0 | 3.9 |
| 2-1-m1E03-1-0616 | C40 H39 N5 O8 S2 | 781.22400 | -2.9 | 0.9 |
| 2-1-m1E03-1-0617 | C40 H39 N5 O8 S2 | 781.22400 | 12.2 | 0.0 |
| 2-1-m1E03-1-0618 | C41 H40 F N5 O6 S2 | 781.24040 | -11.2 | 3.3 |
| 2-1-m1E03-1-0619 | C36 H30 N8 O7 S3 | 782.13996 | -9.6 | -1.3 |
| 2-1-m1E03-1-0620 | C39 H34 N4 O8 S3 | 782.15388 | 0.0 | 4.9 |
| 2-1-m1E03-1-0621 | C38 H34 N6 O9 S2 | 782.18287 | -1.2 | 6.6 |
| 2-1-m1E03-1-0622 | C43 H34 N4 O7 S2 | 782.18689 | 0.0 | 0.0 |
| 2-1-m1E03-1-0623 | C44 H38 N4 O6 S2 | 782.22328 | -11.2 | 0.0 |
| 2-1-m1E03-1-0624 | C41 H39 F N4 O7 S2 | 782.22442 | 0.0 | 4.7 |
| 2-1-m1E03-1-0625 | C39 H31 F2 N5 O7 S2 | 783.16330 | 0.0 | 0.0 |
| 2-1-m1E03-1-0626 | C40 H32 F3 N5 O5 S2 | 783.17970 | 0.1 | 28.6 |
| 2-1-m1E03-1-0627 | C42 H33 N5 O7 S2 | 783.18214 | -2.8 | -1.5 |
| 2-1-m1E03-1-0628 | C39 H34 F N5 O8 S2 | 783.18328 | -2.8 | 0.8 |
| 2-1-m1E03-1-0629 | C37 H36 F3 N5 O7 S2 | 783.20082 | -1.1 | 5.3 |
| 2-1-m1E03-1-0630 | C40 H38 F N5 O7 S2 | 783.21967 | -15.5 | -9.7 |
| 2-1-m1E03-1-0631 | C40 H41 N5 O8 S2 | 783.23965 | -4.2 | -0.9 |
| 2-1-m1E03-1-0632 | C39 H31 F3 N6 O5 S2 | 784.17494 | 5.3 | -6.8 |
| 2-1-m1E03-1-0633 | C38 H33 F N6 O8 S2 | 784.17853 | -1.6 | 0.0 |
| 2-1-m1E03-1-0634 | C40 H34 F2 N4 O7 S2 | 784.18370 | -1.5 | 0.0 |
| 2-1-m1E03-1-0635 | C41 H35 F3 N4 O5 S2 | 784.20010 | 0.0 | 0.0 |
| 2-1-m1E03-1-0636 | C42 H36 N6 O6 S2 | 784.21377 | -4.3 | -11.6 |
| 2-1-m1E03-1-0637 | C39 H40 N6 O8 S2 | 784.23490 | -0.5 | -0.8 |
| 2-1-m1E03-1-0638 | C42 H39 F3 N4 O6 S | 784.25424 | -1.0 | 10.0 |
| 2-1-m1E03-1-0639 | C38 H35 N5 O8 S3 | 785.16478 | -6.2 | -5.7 |
| 2-1-m1E03-1-0640 | C39 H36 F N5 O8 S2 | 785.19893 | 27.9 | 0.0 |
| 2-1-m1E03-1-0641 | C39 H36 F N5 O8 S2 | 785.19893 | 0.0 | 0.0 |
| 2-1-m1E03-1-0642 | C40 H37 F2 N5 O6 S2 | 785.21533 | 0.0 | 0.0 |
| 2-1-m1E03-1-0643 | C36 H30 N6 O7 S4 | 786.10588 | -2.3 | -5.4 |
| 2-1-m1E03-1-0644 | C38 H31 F N4 O8 S3 | 786.12880 | -34.9 | -103.0 |
| 2-1-m1E03-1-0645 | C39 H29 F3 N4 O7 S2 | 786.14298 | -9.6 | 7.9 |
| 2-1-m1E03-1-0646 | C38 H34 N4 O9 S3 | 786.14879 | 0.0 | 0.0 |
| 2-1-m1E03-1-0647 | C40 H33 F3 N4 O6 S2 | 786.17936 | 0.2 | 2.6 |
| 2-1-m1E03-1-0648 | C40 H33 F3 N4 O6 S2 | 786.17936 | -2.1 | -7.1 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0649 | C41 H34 N6 O7 S2 | 786.19304 | -28.7 | 0.0 |
| 2-1-m1E03-1-0650 | C43 H35 F N4 O6 S2 | 786.19820 | 0.0 | 0.0 |
| 2-1-m1E03-1-0651 | C39 H33 F3 N6 O7 S | 786.20835 | 1.5 | -0.3 |
| 2-1-m1E03-1-0652 | C43 H38 N4 O7 S2 | 786.21819 | 0.0 | 0.0 |
| 2-1-m1E03-1-0653 | C44 H42 N4 O6 S2 | 786.25458 | 4.8 | -9.6 |
| 2-1-m1E03-1-0654 | C35 H29 N7 O7 S4 | 787.10113 | -1.3 | 6.8 |
| 2-1-m1E03-1-0655 | C37 H33 N5 O9 S3 | 787.14404 | -19.1 | -14.7 |
| 2-1-m1E03-1-0656 | C37 H33 N5 O9 S3 | 787.14404 | -13.8 | 5.9 |
| 2-1-m1E03-1-0657 | C38 H31 F2 N5 O8 S2 | 787.15821 | -4.7 | 3.4 |
| 2-1-m1E03-1-0658 | C38 H31 F2 N5 O8 S2 | 787.15821 | 0.0 | -1.7 |
| 2-1-m1E03-1-0659 | C39 H32 F3 N5 O6 S2 | 787.17461 | 9.5 | 17.4 |
| 2-1-m1E03-1-0660 | C39 H35 F2 N5 O7 S2 | 787.19460 | 0.0 | 0.0 |
| 2-1-m1E03-1-0661 | C41 H40 F3 N5 O6 S | 787.26514 | -1.4 | -1.2 |
| 2-1-m1E03-1-0662 | C37 H32 N4 O8 S4 | 788.11030 | 5.2 | 6.5 |
| 2-1-m1E03-1-0663 | C37 H32 N4 O10 S3 | 788.12806 | 0.0 | 0.0 |
| 2-1-m1E03-1-0664 | C36 H32 N6 O9 S3 | 788.13929 | 0.0 | -1.7 |
| 2-1-m1E03-1-0665 | C41 H32 N4 O7 S3 | 788.14331 | -0.4 | 4.2 |
| 2-1-m1E03-1-0666 | C37 H30 F2 N6 O8 S2 | 788.15346 | -5.7 | -0.8 |
| 2-1-m1E03-1-0667 | C39 H31 F3 N4 O7 S2 | 788.15863 | -3.0 | 0.9 |
| 2-1-m1E03-1-0668 | C39 H31 F3 N4 O7 S2 | 788.15863 | -0.7 | -1.7 |
| 2-1-m1E03-1-0669 | C42 H36 N4 O6 S3 | 788.17970 | 0.0 | 0.0 |
| 2-1-m1E03-1-0670 | C42 H36 N4 O6 S3 | 788.17970 | 2.5 | 4.3 |
| 2-1-m1E03-1-0671 | C42 H36 N4 O8 S2 | 788.19746 | 0.0 | 0.0 |
| 2-1-m1E03-1-0672 | C38 H30 F3 N5 O7 S2 | 789.15387 | -0.7 | -11.3 |
| 2-1-m1E03-1-0673 | C41 H35 N5 O6 S3 | 789.17495 | -6.0 | 0.3 |
| 2-1-m1E03-1-0674 | C39 H31 F4 N5 O7 S | 789.18803 | 4.9 | 20.4 |
| 2-1-m1E03-1-0675 | C40 H35 N7 O7 S2 | 789.20394 | 1.7 | -1.7 |
| 2-1-m1E03-1-0676 | C38 H29 F3 N4 O8 S2 | 790.13789 | 12.1 | 0.0 |
| 2-1-m1E03-1-0677 | C37 H29 F3 N6 O7 S2 | 790.14912 | -7.7 | 5.6 |
| 2-1-m1E03-1-0678 | C39 H30 F4 N4 O6 S2 | 790.15429 | 8.0 | 1.6 |
| 2-1-m1E03-1-0679 | C41 H34 N4 O7 S3 | 790.15896 | -1.1 | -6.4 |
| 2-1-m1E03-1-0680 | C41 H34 N4 O7 S3 | 790.15896 | 10.8 | -14.5 |
| 2-1-m1E03-1-0681 | C43 H39 F N4 O6 S2 | 790.22950 | 6.0 | 5.1 |
| 2-1-m1E03-1-0682 | C36 H33 N5 O10 S3 | 791.13895 | 0.0 | 0.0 |
| 2-1-m1E03-1-0683 | C38 H32 F3 N5 O7 S2 | 791.16952 | 19.2 | -0.8 |
| 2-1-m1E03-1-0684 | C38 H32 F3 N5 O7 S2 | 791.16952 | 1.2 | -0.2 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0685 | C38 H32 F3 N5 O7 S2 | 791.16952 | -4.8 | -7.4 |
| 2-1-m1E03-1-0686 | C38 H32 F3 N5 O7 S2 | 791.16952 | 0.0 | -0.5 |
| 2-1-m1E03-1-0687 | C41 H37 N5 O8 S2 | 791.20835 | 14.1 | -0.5 |
| 2-1-m1E03-1-0688 | C42 H41 N5 O7 S2 | 791.24474 | -0.4 | -5.5 |
| 2-1-m1E03-1-0689 | C43 H45 N5 O6 S2 | 791.28113 | 0.8 | 1.6 |
| 2-1-m1E03-1-0690 | C36 H29 F N4 O8 S4 | 792.08522 | -1.7 | 1.2 |
| 2-1-m1E03-1-0691 | C40 H30 F2 N6 O6 S2 | 792.16363 | -4.7 | 6.3 |
| 2-1-m1E03-1-0692 | C37 H31 F3 N6 O7 S2 | 792.16477 | -0.5 | -1.0 |
| 2-1-m1E03-1-0693 | C37 H31 F3 N6 O7 S2 | 792.16477 | 0.0 | 0.0 |
| 2-1-m1E03-1-0694 | C40 H30 F6 N4 O5 S | 792.18411 | -16.9 | -4.0 |
| 2-1-m1E03-1-0695 | C40 H36 N6 O6 S3 | 792.18585 | -18.5 | -4.5 |
| 2-1-m1E03-1-0696 | C41 H40 N6 O7 S2 | 792.23999 | 0.0 | 0.0 |
| 2-1-m1E03-1-0697 | C43 H44 N4 O7 S2 | 792.26514 | 0.0 | 0.0 |
| 2-1-m1E03-1-0698 | C35 H31 N5 O9 S4 | 793.10046 | 0.0 | -2.0 |
| 2-1-m1E03-1-0699 | C36 H29 F2 N5 O8 S3 | 793.11463 | -7.9 | -1.8 |
| 2-1-m1E03-1-0700 | C40 H32 F N5 O6 S3 | 793.14987 | 4.8 | 24.6 |
| 2-1-m1E03-1-0701 | C36 H30 F3 N7 O7 S2 | 793.16002 | -8.6 | 0.0 |
| 2-1-m1E03-1-0702 | C40 H35 N5 O7 S3 | 793.16986 | 0.0 | 0.0 |
| 2-1-m1E03-1-0703 | C40 H35 N5 O7 S3 | 793.16986 | 0.0 | 0.0 |
| 2-1-m1E03-1-0704 | C39 H32 F N7 O7 S2 | 793.17887 | 0.0 | 0.0 |
| 2-1-m1E03-1-0705 | C42 H43 N5 O7 S2 | 793.26039 | 62.8 | 41.9 |
| 2-1-m1E03-1-0706 | C34 H30 N6 O9 S4 | 794.09571 | -7.7 | -2.6 |
| 2-1-m1E03-1-0707 | C36 H28 F2 N4 O9 S3 | 794.09865 | -9.2 | -4.0 |
| 2-1-m1E03-1-0708 | C37 H29 F3 N4 O7 S3 | 794.11505 | 8.5 | 8.4 |
| 2-1-m1E03-1-0709 | C39 H34 N6 O7 S3 | 794.16511 | 0.6 | 0.2 |
| 2-1-m1E03-1-0710 | C39 H34 N6 O7 S3 | 794.16511 | -2.9 | -2.2 |
| 2-1-m1E03-1-0711 | C41 H32 F2 N4 O7 S2 | 794.16805 | 1.1 | 0.2 |
| 2-1-m1E03-1-0712 | C40 H38 N6 O8 S2 | 794.21925 | -2.0 | -1.5 |
| 2-1-m1E03-1-0713 | C42 H42 N4 O8 S2 | 794.24441 | 5.5 | 0.0 |
| 2-1-m1E03-1-0714 | C46 H42 N4 O5 S2 | 794.25966 | 0.0 | 19.1 |
| 2-1-m1E03-1-0715 | C36 H28 F3 N5 O7 S3 | 795.11029 | -11.9 | 2.5 |
| 2-1-m1E03-1-0716 | C35 H30 F N5 O10 S3 | 795.11388 | 0.0 | 0.0 |
| 2-1-m1E03-1-0717 | C38 H33 N7 O7 S3 | 795.16036 | -0.2 | 0.6 |
| 2-1-m1E03-1-0718 | C38 H33 N7 O7 S3 | 795.16036 | -5.4 | 0.9 |
| 2-1-m1E03-1-0719 | C40 H34 F N5 O8 S2 | 795.18328 | 0.2 | 8.4 |
| 2-1-m1E03-1-0720 | C39 H31 F6 N5 O5 S | 795.19501 | -0.6 | -15.9 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0721 | C40 H32 F3 N7 O6 S | 795.20869 | 0.3 | 8.9 |
| 2-1-m1E03-1-0722 | C41 H41 N5 O8 S2 | 795.23965 | -8.3 | -4.7 |
| 2-1-m1E03-1-0723 | C40 H30 F2 N4 O6 S3 | 796.12955 | 0.0 | 0.0 |
| 2-1-m1E03-1-0724 | C40 H30 F2 N4 O6 S3 | 796.12955 | 3.5 | 0.6 |
| 2-1-m1E03-1-0725 | C40 H36 N4 O8 S3 | 796.16953 | 0.0 | 0.0 |
| 2-1-m1E03-1-0726 | C39 H36 N6 O9 S2 | 796.19852 | 2.3 | 9.7 |
| 2-1-m1E03-1-0727 | C36 H30 F3 N5 O7 S3 | 797.12595 | -2.5 | -5.2 |
| 2-1-m1E03-1-0728 | C36 H30 F3 N5 O9 S2 | 797.14370 | -53.4 | 19.0 |
| 2-1-m1E03-1-0729 | C39 H32 F N5 O7 S3 | 797.14479 | -6.5 | 1.4 |
| 2-1-m1E03-1-0730 | C39 H32 F N5 O7 S3 | 797.14479 | -1.3 | 4.2 |
| 2-1-m1E03-1-0731 | C43 H35 N5 O7 S2 | 797.19779 | -6.5 | 4.9 |
| 2-1-m1E03-1-0732 | C40 H36 F N5 O8 S2 | 797.19893 | -4.3 | -1.2 |
| 2-1-m1E03-1-0733 | C40 H39 N5 O9 S2 | 797.21892 | 0.0 | 0.0 |
| 2-1-m1E03-1-0734 | C41 H43 N5 O8 S2 | 797.25530 | -0.5 | -1.2 |
| 2-1-m1E03-1-0735 | C35 H29 F3 N6 O7 S3 | 798.12119 | 12.3 | -5.1 |
| 2-1-m1E03-1-0736 | C37 H30 N6 O9 S3 | 798.12364 | -8.4 | -6.0 |
| 2-1-m1E03-1-0737 | C39 H34 N4 O9 S3 | 798.14879 | -4.4 | -0.5 |
| 2-1-m1E03-1-0738 | C42 H37 F3 N4 O5 S2 | 798.21575 | -11.6 | -1.3 |
| 2-1-m1E03-1-0739 | C44 H38 N4 O7 S2 | 798.21819 | -4.2 | 9.7 |
| 2-1-m1E03-1-0740 | C40 H32 F3 N5 O6 S2 | 799.17461 | 1.4 | 17.1 |
| 2-1-m1E03-1-0741 | C39 H34 F N5 O9 S2 | 799.17820 | 0.0 | 4.5 |
| 2-1-m1E03-1-0742 | C37 H36 F3 N5 O8 S2 | 799.19574 | 2.8 | 7.3 |
| 2-1-m1E03-1-0743 | C41 H39 F2 N5 O6 S2 | 799.23098 | 0.0 | 0.0 |
| 2-1-m1E03-1-0744 | C40 H31 F3 N4 O7 S2 | 800.15863 | 0.0 | 0.0 |
| 2-1-m1E03-1-0745 | C43 H33 F N4 O7 S2 | 800.17747 | 7.7 | 5.1 |
| 2-1-m1E03-1-0746 | C41 H35 F3 N4 O6 S2 | 800.19501 | -1.0 | -10.8 |
| 2-1-m1E03-1-0747 | C41 H35 F3 N4 O6 S2 | 800.19501 | 3.1 | 0.0 |
| 2-1-m1E03-1-0748 | C41 H35 F3 N4 O6 S2 | 800.19501 | 5.0 | 5.4 |
| 2-1-m1E03-1-0749 | C41 H38 F2 N4 O7 S2 | 800.21500 | -4.8 | -0.8 |
| 2-1-m1E03-1-0750 | C45 H44 N4 O6 S2 | 800.27023 | -95.8 | -9.1 |
| 2-1-m1E03-1-0751 | C39 H30 F3 N5 O7 S2 | 801.15387 | -0.6 | -0.4 |
| 2-1-m1E03-1-0752 | C38 H35 N5 O9 S3 | 801.15969 | 2.5 | -1.2 |
| 2-1-m1E03-1-0753 | C40 H40 F N5 O8 S2 | 801.23023 | -3.4 | -0.8 |
| 2-1-m1E03-1-0754 | C38 H34 N4 O8 S4 | 802.12595 | 24.6 | 9.9 |
| 2-1-m1E03-1-0755 | C38 H32 F2 N6 O8 S2 | 802.16911 | -5.0 | -1.0 |
| 2-1-m1E03-1-0756 | C40 H33 F3 N4 O7 S2 | 802.17428 | -1.6 | 0.2 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0757 | C40 H33 F3 N4 O7 S2 | 802.17428 | 0.4 | 0.4 |
| 2-1-m1E03-1-0758 | C44 H42 N4 O7 S2 | 802.24949 | 20.6 | 0.0 |
| 2-1-m1E03-1-0759 | C39 H32 F3 N5 O7 S2 | 803.16952 | -1.9 | 2.2 |
| 2-1-m1E03-1-0760 | C39 H35 F2 N5 O8 S2 | 803.18951 | 25.3 | 0.0 |
| 2-1-m1E03-1-0761 | C42 H37 N5 O6 S3 | 803.19060 | -4.2 | -14.5 |
| 2-1-m1E03-1-0762 | C40 H36 F3 N5 O6 S2 | 803.20591 | 0.0 | 0.0 |
| 2-1-m1E03-1-0763 | C41 H37 N7 O7 S2 | 803.21959 | -6.6 | -2.0 |
| 2-1-m1E03-1-0764 | C41 H40 F3 N5 O7 S | 803.26005 | 0.5 | 2.8 |
| 2-1-m1E03-1-0765 | C37 H32 N4 O9 S4 | 804.10521 | 0.0 | 0.0 |
| 2-1-m1E03-1-0766 | C38 H30 F2 N4 O8 S3 | 804.11938 | 20.4 | 3.2 |
| 2-1-m1E03-1-0767 | C40 H32 F4 N4 O6 S2 | 804.16994 | -1.8 | -2.2 |
| 2-1-m1E03-1-0768 | C37 H32 F N5 O9 S3 | 805.13462 | -2.4 | 9.5 |
| 2-1-m1E03-1-0769 | C38 H30 F3 N5 O8 S2 | 805.14879 | -2.3 | 0.6 |
| 2-1-m1E03-1-0770 | C37 H35 N5 O10 S3 | 805.15460 | 0.0 | 1.1 |
| 2-1-m1E03-1-0771 | C41 H35 N5 O7 S3 | 805.16986 | 0.0 | 0.0 |
| 2-1-m1E03-1-0772 | C39 H34 F3 N5 O7 S2 | 805.18517 | 11.1 | 0.7 |
| 2-1-m1E03-1-0773 | C39 H34 F3 N5 O7 S2 | 805.18517 | -2.9 | 2.4 |
| 2-1-m1E03-1-0774 | C40 H35 N7 O8 S2 | 805.19885 | 18.7 | 3.5 |
| 2-1-m1E03-1-0775 | C42 H39 N5 O8 S2 | 805.22400 | 16.2 | 5.1 |
| 2-1-m1E03-1-0776 | C43 H43 N5 O7 S2 | 805.26039 | 1.3 | 5.7 |
| 2-1-m1E03-1-0777 | C38 H29 F3 N4 O7 S3 | 806.11505 | -4.6 | 8.5 |
| 2-1-m1E03-1-0778 | C38 H29 F3 N4 O9 S2 | 806.13280 | 0.0 | -4.3 |
| 2-1-m1E03-1-0779 | C38 H33 F3 N6 O7 S2 | 806.18042 | 0.0 | 0.0 |
| 2-1-m1E03-1-0780 | C44 H34 N6 O6 S2 | 806.19812 | -11.0 | -2.1 |
| 2-1-m1E03-1-0781 | C41 H38 N6 O6 S3 | 806.20150 | -15.6 | -8.0 |
| 2-1-m1E03-1-0782 | C36 H33 N5 O9 S4 | 807.11611 | 0.0 | 0.0 |
| 2-1-m1E03-1-0783 | C36 H33 N5 011 S3 | 807.13387 | 0.0 | 0.0 |
| 2-1-m1E03-1-0784 | C38 H32 F3 N5 O8 S2 | 807.16444 | 10.8 | 10.2 |
| 2-1-m1E03-1-0785 | C38 H32 F3 N5 O8 S2 | 807.16444 | -20.4 | -16.0 |
| 2-1-m1E03-1-0786 | C41 H37 N5 O7 S3 | 807.18551 | -6.1 | 12.6 |
| 2-1-m1E03-1-0787 | C41 H37 N5 O7 S3 | 807.18551 | 0.0 | 0.0 |
| 2-1-m1E03-1-0788 | C41 H37 N5 O9 S2 | 807.20327 | 14.8 | 7.9 |
| 2-1-m1E03-1-0789 | C40 H32 N4 O9 S3 | 808.13314 | -37.8 | -14.2 |
| 2-1-m1E03-1-0790 | C39 H32 N6 O8 S3 | 808.14437 | -5.3 | -2.9 |
| 2-1-m1E03-1-0791 | C39 H29 F5 N4 O6 S2 | 808.14487 | -5.7 | -21.7 |
| 2-1-m1E03-1-0792 | C37 H31 F3 N6 O8 S2 | 808.15969 | -12.2 | 7.7 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0793 | C40 H36 N6 O7 S3 | 808.18076 | -1.4 | 2.2 |
| 2-1-m1E03-1-0794 | C40 H36 N6 O7 S3 | 808.18076 | 0.0 | 3.2 |
| 2-1-m1E03-1-0795 | C45 H36 N4 O7 S2 | 808.20254 | 0.0 | 0.0 |
| 2-1-m1E03-1-0796 | C43 H38 F2 N4 O6 S2 | 808.22008 | 8.1 | 5.5 |
| 2-1-m1E03-1-0797 | C38 H31 N7 O8 S3 | 809.13962 | -1.3 | 0.1 |
| 2-1-m1E03-1-0798 | C37 H30 F3 N5 O9 S2 | 809.14370 | -14.8 | 14.9 |
| 2-1-m1E03-1-0799 | C36 H30 F3 N7 O8 S2 | 809.15494 | 18.3 | 0.0 |
| 2-1-m1E03-1-0800 | C38 H31 F4 N5 O7 S2 | 809.16010 | 0.0 | 0.0 |
| 2-1-m1E03-1-0801 | C40 H35 N5 O8 S3 | 809.16478 | 0.0 | 0.0 |
| 2-1-m1E03-1-0802 | C40 H35 N5 O8 S3 | 809.16478 | 0.0 | 0.0 |
| 2-1-m1E03-1-0803 | C42 H40 F N5 O7 S2 | 809.23532 | -2.0 | -3.0 |
| 2-1-m1E03-1-0804 | C36 H28 F2 N4 O8 S4 | 810.07580 | -1.1 | 0.7 |
| 2-1-m1E03-1-0805 | C44 H34 N4 O6 S3 | 810.16405 | 0.0 | 0.0 |
| 2-1-m1E03-1-0806 | C44 H34 N4 O6 S3 | 810.16405 | 0.0 | 0.0 |
| 2-1-m1E03-1-0807 | C35 H30 F N5 O9 S4 | 811.09104 | 1.4 | -1.6 |
| 2-1-m1E03-1-0808 | C40 H31 F2 N5 O6 S3 | 811.14045 | 0.0 | 0.0 |
| 2-1-m1E03-1-0809 | C39 H31 F2 N7 O7 S2 | 811.16944 | 5.8 | 0.0 |
| 2-1-m1E03-1-0810 | C39 H31 F6 N5 O6 S | 811.18992 | -12.4 | 2.4 |
| 2-1-m1E03-1-0811 | C42 H45 N5 O8 S2 | 811.27095 | 0.0 | 0.0 |
| 2-1-m1E03-1-0812 | C39 H33 F N6 O7 S3 | 812.15569 | -3.4 | -8.0 |
| 2-1-m1E03-1-0813 | C39 H33 F N6 O7 S3 | 812.15569 | 2.2 | 1.2 |
| 2-1-m1E03-1-0814 | C43 H39 F3 N4 O5 S2 | 812.23140 | 0.0 | 0.0 |
| 2-1-m1E03-1-0815 | C35 H29 F2 N5 O10 S3 | 813.10446 | -2.6 | 0.9 |
| 2-1-m1E03-1-0816 | C36 H30 F3 N5 O8 S3 | 813.12086 | -3.0 | 0.0 |
| 2-1-m1E03-1-0817 | C40 H33 F2 N5 O8 S2 | 813.17386 | 1.5 | -3.5 |
| 2-1-m1E03-1-0818 | C41 H43 N5 O9 S2 | 813.25022 | 0.0 | 0.0 |
| 2-1-m1E03-1-0819 | C37 H30 N6 O8 S4 | 814.10079 | -5.1 | -4.4 |
| 2-1-m1E03-1-0820 | C35 H29 F3 N6 O8 S3 | 814.11611 | 0.0 | 0.0 |
| 2-1-m1E03-1-0821 | C42 H37 F3 N4 O6 S2 | 814.21066 | 0.0 | -32.9 |
| 2-1-m1E03-1-0822 | C45 H42 N4 O7 S2 | 814.24949 | 0.0 | 0.0 |
| 2-1-m1E03-1-0823 | C39 H31 F2 N5 O7 S3 | 815.13537 | 14.7 | 25.4 |
| 2-1-m1E03-1-0824 | C39 H31 F2 N5 O7 S3 | 815.13537 | 0.0 | 0.0 |
| 2-1-m1E03-1-0825 | C40 H32 F3 N5 O7 S2 | 815.16952 | -6.6 | -5.1 |
| 2-1-m1E03-1-0826 | C39 H37 N5 O9 S3 | 815.17534 | 3.1 | -0.1 |
| 2-1-m1E03-1-0827 | C40 H31 F3 N4 O8 S2 | 816.15354 | -4.0 | -4.5 |
| 2-1-m1E03-1-0828 | C41 H35 F3 N4 O7 S2 | 816.18993 | 7.0 | 0.0 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0829 | C41 H35 F3 N4 O7 S2 | 816.18993 | 0.0 | 0.0 |
| 2-1-m1E03-1-0830 | C39 H30 F3 N5 O8 S2 | 817.14879 | -1.6 | -0.2 |
| 2-1-m1E03-1-0831 | C38 H35 N5 O10 S3 | 817.15460 | 0.0 | 0.2 |
| 2-1-m1E03-1-0832 | C41 H38 F3 N5 O6 S2 | 817.22156 | -1.7 | -10.4 |
| 2-1-m1E03-1-0833 | C40 H30 F4 N4 O7 S2 | 818.14920 | -2.4 | -5.9 |
| 2-1-m1E03-1-0834 | C42 H34 N4 O8 S3 | 818.15388 | 0.0 | 0.0 |
| 2-1-m1E03-1-0835 | C39 H32 F3 N5 O8 S2 | 819.16444 | -4.9 | 1.6 |
| 2-1-m1E03-1-0836 | C40 H36 F3 N5 O7 S2 | 819.20082 | -3.5 | -3.9 |
| 2-1-m1E03-1-0837 | C40 H36 F3 N5 O7 S2 | 819.20082 | 1.2 | -6.2 |
| 2-1-m1E03-1-0838 | C40 H36 F3 N5 O7 S2 | 819.20082 | 0.0 | 1.5 |
| 2-1-m1E03-1-0839 | C40 H39 F2 N5 O8 S2 | 819.22081 | 0.0 | 0.0 |
| 2-1-m1E03-1-0840 | C44 H45 N5 O7 S2 | 819.27604 | 10.1 | 28.9 |
| 2-1-m1E03-1-0841 | C38 H31 F3 N6 O8 S2 | 820.15969 | 0.0 | 0.0 |
| 2-1-m1E03-1-0842 | C40 H32 F4 N4 O7 S2 | 820.16485 | 3.4 | -2.2 |
| 2-1-m1E03-1-0843 | C42 H40 N6 O6 S3 | 820.21715 | 0.0 | -9.7 |
| 2-1-m1E03-1-0844 | C37 H35 N5 O9 S4 | 821.13176 | 11.7 | -6.4 |
| 2-1-m1E03-1-0845 | C39 H34 F3 N5 O8 S2 | 821.18009 | 5.9 | 5.7 |
| 2-1-m1E03-1-0846 | C39 H34 F3 N5 O8 S2 | 821.18009 | 0.6 | 4.7 |
| 2-1-m1E03-1-0847 | C43 H43 N5 O8 S2 | 821.25530 | -9.8 | 7.4 |
| 2-1-m1E03-1-0848 | C38 H29 F3 N4 O8 S3 | 822.10996 | -2.8 | -5.4 |
| 2-1-m1E03-1-0849 | C41 H34 N4 O9 S3 | 822.14879 | 0.0 | 0.0 |
| 2-1-m1E03-1-0850 | C38 H33 F3 N6 O8 S2 | 822.17534 | -3.6 | 1.3 |
| 2-1-m1E03-1-0851 | C43 H33 F3 N4 O6 S2 | 822.17936 | -9.0 | -7.4 |
| 2-1-m1E03-1-0852 | C41 H38 N6 O7 S3 | 822.19641 | 8.1 | 35.1 |
| 2-1-m1E03-1-0853 | C47 H42 N4 O6 S2 | 822.25458 | 0.0 | 28.1 |
| 2-1-m1E03-1-0854 | C36 H33 N5 O10 S4 | 823.11103 | 4.0 | -12.1 |
| 2-1-m1E03-1-0855 | C37 H31 F2 N5 O9 S3 | 823.12520 | 0.0 | 3.8 |
| 2-1-m1E03-1-0856 | C40 H33 N5 O9 S3 | 823.14404 | 10.9 | 7.3 |
| 2-1-m1E03-1-0857 | C39 H33 N7 O8 S3 | 823.15527 | -2.6 | -2.4 |
| 2-1-m1E03-1-0858 | C39 H33 F4 N5 O7 S2 | 823.17575 | 22.1 | 11.3 |
| 2-1-m1E03-1-0859 | C40 H32 N4 O8 S4 | 824.11030 | -19.8 | 0.0 |
| 2-1-m1E03-1-0860 | C40 H32 N4 O8 S4 | 824.11030 | 0.0 | 0.0 |
| 2-1-m1E03-1-0861 | C41 H31 F3 N6 O6 S2 | 824.16986 | -1.7 | 4.2 |
| 2-1-m1E03-1-0862 | C40 H36 N6 O8 S3 | 824.17567 | -7.1 | -0.2 |
| 2-1-m1E03-1-0863 | C40 H36 N6 O8 S3 | 824.17567 | 0.0 | -0.4 |
| 2-1-m1E03-1-0864 | C39 H31 N5 O8 S4 | 825.10555 | 9.7 | -9.2 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0865 | C37 H30 F3 N5 O8 S3 | 825.12086 | 1.9 | -2.5 |
| 2-1-m1E03-1-0866 | C37 H30 F3 N5 O10 S2 | 825.13862 | 0.0 | 0.0 |
| 2-1-m1E03-1-0867 | C44 H35 N5 O6 S3 | 825.17495 | -17.4 | -31.1 |
| 2-1-m1E03-1-0868 | C37 H29 F3 N4 O9 S3 | 826.10488 | 20.4 | 0.0 |
| 2-1-m1E03-1-0869 | C39 H31 F N6 O8 S3 | 826.13495 | -2.9 | -2.5 |
| 2-1-m1E03-1-0870 | C42 H33 F3 N4 O7 S2 | 826.17428 | 23.8 | 12.0 |
| 2-1-m1E03-1-0871 | C38 H30 F5 N5 O7 S2 | 827.15068 | -9.5 | -4.1 |
| 2-1-m1E03-1-0872 | C42 H39 F2 N5 O7 S2 | 827.22590 | 4.7 | 12.9 |
| 2-1-m1E03-1-0873 | C41 H31 F3 N4 O6 S3 | 828.13578 | -5.6 | 1.1 |
| 2-1-m1E03-1-0874 | C41 H31 F3 N4 O6 S3 | 828.13578 | -7.5 | -3.8 |
| 2-1-m1E03-1-0875 | C43 H39 F3 N4 O6 S2 | 828.22631 | 0.0 | -1.6 |
| 2-1-m1E03-1-0876 | C35 H29 F2 N5 O9 S4 | 829.08162 | -3.9 | 2.8 |
| 2-1-m1E03-1-0877 | C39 H32 F2 N6 O7 S3 | 830.14627 | 4.2 | -6.3 |
| 2-1-m1E03-1-0878 | C40 H32 F3 N5 O8 S2 | 831.16444 | 0.7 | -7.7 |
| 2-1-m1E03-1-0879 | C42 H40 F3 N5 O6 S2 | 831.23721 | -1.1 | -9.8 |
| 2-1-m1E03-1-0880 | C40 H32 N8 O7 S3 | 832.15561 | 3.9 | 0.0 |
| 2-1-m1E03-1-0881 | C41 H35 F3 N4 O8 S2 | 832.18484 | 0.0 | 0.0 |
| 2-1-m1E03-1-0882 | C42 H39 F3 N4 O7 S2 | 832.22123 | -5.3 | 0.0 |
| 2-1-m1E03-1-0883 | C41 H38 F3 N5 O7 S2 | 833.21647 | 0.0 | -2.2 |
| 2-1-m1E03-1-0884 | C36 H30 N6 O10 S4 | 834.09062 | 0.0 | 0.0 |
| 2-1-m1E03-1-0885 | C40 H30 F4 N4 O8 S2 | 834.14412 | -7.9 | -3.2 |
| 2-1-m1E03-1-0886 | C41 H34 N6 O8 S3 | 834.16002 | 1.3 | -2.9 |
| 2-1-m1E03-1-0887 | C39 H33 F3 N6 O8 S2 | 834.17534 | -9.2 | 0.0 |
| 2-1-m1E03-1-0888 | C39 H32 F3 N5 O9 S2 | 835.15935 | -3.1 | 2.0 |
| 2-1-m1E03-1-0889 | C40 H36 F3 N5 O8 S2 | 835.19574 | -25.2 | -9.9 |
| 2-1-m1E03-1-0890 | C40 H36 F3 N5 O8 S2 | 835.19574 | 0.0 | 0.0 |
| 2-1-m1E03-1-0891 | C40 H32 N6 O7 S4 | 836.12153 | -5.4 | -6.4 |
| 2-1-m1E03-1-0892 | C40 H32 N6 O7 S4 | 836.12153 | 0.0 | 14.5 |
| 2-1-m1E03-1-0893 | C39 H31 F3 N4 O8 S3 | 836.12561 | 4.0 | -3.1 |
| 2-1-m1E03-1-0894 | C38 H31 F3 N6 O9 S2 | 836.15460 | 0.0 | 0.0 |
| 2-1-m1E03-1-0895 | C42 H36 N4 O9 S3 | 836.16444 | 0.0 | 0.0 |
| 2-1-m1E03-1-0896 | C39 H31 F4 N5 O8 S2 | 837.15502 | -4.0 | 0.0 |
| 2-1-m1E03-1-0897 | C41 H34 N4 O8 S4 | 838.12595 | 0.4 | 0.0 |
| 2-1-m1E03-1-0898 | C40 H33 N5 O8 S4 | 839.12120 | -10.6 | -7.2 |
| 2-1-m1E03-1-0899 | C39 H33 F4 N5 O8 S2 | 839.17067 | -20.6 | -19.3 |
| 2-1-m1E03-1-0900 | C39 H32 N6 O8 S4 | 840.11644 | -2.6 | -1.3 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0901 | C41 H33 F N4 O9 S3 | 840.13937 | 0.0 | 0.0 |
| 2-1-m1E03-1-0902 | C40 H30 F6 N4 O6 S2 | 840.15110 | -0.9 | 0.0 |
| 2-1-m1E03-1-0903 | C41 H31 F3 N6 O7 S2 | 840.16477 | 19.5 | 7.7 |
| 2-1-m1E03-1-0904 | C41 H40 N6 O8 S3 | 840.20697 | -8.6 | -0.9 |
| 2-1-m1E03-1-0905 | C44 H39 F3 N4 O6 S2 | 840.22631 | -21.8 | 1.4 |
| 2-1-m1E03-1-0906 | C37 H30 F3 N5 O9 S3 | 841.11577 | -3.9 | 3.2 |
| 2-1-m1E03-1-0907 | C40 H35 N5 O10 S3 | 841.15460 | 0.0 | 0.0 |
| 2-1-m1E03-1-0908 | C37 H29 F3 N4 O8 S4 | 842.08203 | 0.3 | 12.0 |
| 2-1-m1E03-1-0909 | C37 H29 F3 N4 O10 S3 | 842.09979 | 4.9 | -4.9 |
| 2-1-m1E03-1-0910 | C40 H31 F N4 O8 S4 | 842.10087 | 0.0 | 0.0 |
| 2-1-m1E03-1-0911 | C39 H34 N6 O10 S3 | 842.14985 | 0.8 | 2.8 |
| 2-1-m1E03-1-0912 | C42 H33 F3 N4 O8 S2 | 842.16919 | 13.4 | 0.0 |
| 2-1-m1E03-1-0913 | C39 H33 N5 O9 S4 | 843.11611 | -0.1 | -0.7 |
| 2-1-m1E03-1-0914 | C41 H32 F3 N5 O6 S3 | 843.14668 | 3.9 | -0.3 |
| 2-1-m1E03-1-0915 | C40 H32 F3 N7 O7 S2 | 843.17567 | 5.5 | -2.2 |
| 2-1-m1E03-1-0916 | C38 H32 N6 O9 S4 | 844.11136 | -14.8 | -2.5 |
| 2-1-m1E03-1-0917 | C38 H32 N6 O9 S4 | 844.11136 | 1.1 | -0.2 |
| 2-1-m1E03-1-0918 | C41 H31 F3 N4 O7 S3 | 844.13070 | 0.0 | 0.0 |
| 2-1-m1E03-1-0919 | C41 H31 F3 N4 O7 S3 | 844.13070 | -9.9 | -6.8 |
| 2-1-m1E03-1-0920 | C36 H30 F3 N5 O10 S3 | 845.11069 | -1.8 | -5.1 |
| 2-1-m1E03-1-0921 | C41 H34 F3 N5 O8 S2 | 845.18009 | 14.3 | -23.5 |
| 2-1-m1E03-1-0922 | C40 H32 F3 N5 O7 S3 | 847.14160 | -4.2 | 27.3 |
| 2-1-m1E03-1-0923 | C40 H32 F3 N5 O7 S3 | 847.14160 | -5.5 | 24.1 |
| 2-1-m1E03-1-0924 | C42 H40 F3 N5 O7 S2 | 847.23212 | 13.4 | -7.4 |
| 2-1-m1E03-1-0925 | C39 H31 F3 N6 O7 S3 | 848.13684 | -2.4 | -2.0 |
| 2-1-m1E03-1-0926 | C43 H40 N6 O7 S3 | 848.21206 | 4.0 | -10.4 |
| 2-1-m1E03-1-0927 | C42 H39 F3 N4 O8 S2 | 848.21614 | 0.0 | 0.0 |
| 2-1-m1E03-1-0928 | C36 H30 N6 O9 S5 | 850.06778 | -0.2 | -1.9 |
| 2-1-m1E03-1-0929 | C39 H33 F3 N6 O9 S2 | 850.17025 | -6.6 | 2.8 |
| 2-1-m1E03-1-0930 | C43 H38 N4 O9 S3 | 850.18009 | 0.0 | 0.0 |
| 2-1-m1E03-1-0931 | C40 H33 N7 O7 S4 | 851.13243 | -0.4 | 6.4 |
| 2-1-m1E03-1-0932 | C40 H36 F3 N5 O9 S2 | 851.19065 | 0.0 | 0.0 |
| 2-1-m1E03-1-0933 | C41 H40 F3 N5 O8 S2 | 851.22704 | 0.0 | 0.0 |
| 2-1-m1E03-1-0934 | C39 H31 F3 N4 O9 S3 | 852.12053 | 16.5 | 3.6 |
| 2-1-m1E03-1-0935 | C42 H36 N4 O8 S4 | 852.14160 | 0.0 | 0.0 |
| 2-1-m1E03-1-0936 | C42 H36 N4 O10 S3 | 852.15936 | 0.0 | 0.0 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0937 | C41 H35 N5 O8 S4 | 853.13685 | 5.2 | 15.4 |
| 2-1-m1E03-1-0938 | C39 H31 F4 N5 O9 S2 | 853.14993 | 0.0 | 2.2 |
| 2-1-m1E03-1-0939 | C41 H34 N4 O9 S4 | 854.12086 | 0.0 | 0.0 |
| 2-1-m1E03-1-0940 | C38 H32 F3 N5 O9 S3 | 855.13142 | -6.7 | -5.4 |
| 2-1-m1E03-1-0941 | C41 H37 N5 O10 S3 | 855.17025 | -1.8 | 17.5 |
| 2-1-m1E03-1-0942 | C40 H30 F6 N4 O7 S2 | 856.14601 | -5.0 | 0.8 |
| 2-1-m1E03-1-0943 | C44 H39 F3 N4 O7 S2 | 856.22123 | 0.0 | 0.0 |
| 2-1-m1E03-1-0944 | C40 H32 F N5 O8 S4 | 857.11177 | 24.6 | 0.0 |
| 2-1-m1E03-1-0945 | C40 H35 N5 O9 S4 | 857.13176 | -0.1 | 0.4 |
| 2-1-m1E03-1-0946 | C37 H29 F3 N4 O9 S4 | 858.07695 | 7.7 | -5.7 |
| 2-1-m1E03-1-0947 | C39 H34 N6 O9 S4 | 858.12701 | 0.0 | 18.6 |
| 2-1-m1E03-1-0948 | C41 H32 F2 N4 O9 S3 | 858.12995 | -13.8 | -0.5 |
| 2-1-m1E03-1-0949 | C38 H33 N7 O9 S4 | 859.12226 | -3.1 | -0.9 |
| 2-1-m1E03-1-0950 | C41 H32 F3 N5 O7 S3 | 859.14160 | -8.0 | -12.6 |
| 2-1-m1E03-1-0951 | C40 H34 F N5 O10 S3 | 859.14518 | 17.4 | 0.0 |
| 2-1-m1E03-1-0952 | C39 H31 F6 N5 O7 S2 | 859.15691 | -11.4 | 0.6 |
| 2-1-m1E03-1-0953 | C40 H32 F3 N7 O8 S2 | 859.17059 | -22.5 | 1.1 |
| 2-1-m1E03-1-0954 | C43 H40 F3 N5 O7 S2 | 859.23212 | 9.0 | 13.6 |
| 2-1-m1E03-1-0955 | C40 H30 F2 N4 O8 S4 | 860.09145 | -0.5 | 1.6 |
| 2-1-m1E03-1-0956 | C36 H30 F3 N5 O9 S4 | 861.08784 | 5.6 | 4.8 |
| 2-1-m1E03-1-0957 | C36 H30 F3 N5 O11 S3 | 861.10560 | -0.1 | -4.5 |
| 2-1-m1E03-1-0958 | C39 H32 F N5 O9 S4 | 861.10669 | 9.0 | 0.0 |
| 2-1-m1E03-1-0959 | C41 H34 F3 N5 O9 S2 | 861.17500 | 0.1 | 14.8 |
| 2-1-m1E03-1-0960 | C40 H33 F3 N6 O7 S3 | 862.15249 | 3.4 | 21.9 |
| 2-1-m1E03-1-0961 | C40 H32 F3 N5 O8 S3 | 863.13651 | -11.2 | 0.6 |
| 2-1-m1E03-1-0962 | C40 H32 F3 N5 O8 S3 | 863.13651 | -32.8 | 23.2 |
| 2-1-m1E03-1-0963 | C42 H37 N5 O8 S4 | 867.15250 | 5.9 | 14.8 |
| 2-1-m1E03-1-0964 | C41 H40 F3 N5 O9 S2 | 867.22195 | 0.0 | 0.0 |
| 2-1-m1E03-1-0965 | C41 H35 N5 O9 S4 | 869.13176 | -56.8 | -4.6 |
| 2-1-m1E03-1-0966 | C42 H39 N5 O10 S3 | 869.18590 | -11.0 | -0.7 |
| 2-1-m1E03-1-0967 | C38 H32 F3 N5 O10 S3 | 871.12634 | -5.6 | -4.3 |
| 2-1-m1E03-1-0968 | C41 H37 N5 O9 S4 | 871.14741 | -5.1 | 1.6 |
| 2-1-m1E03-1-0969 | C41 H37 N5 O11 S3 | 871.16517 | 0.0 | 0.0 |
| 2-1-m1E03-1-0970 | C40 H36 N6 O9 S4 | 872.14266 | -11.6 | 12.4 |
| 2-1-m1E03-1-0971 | C45 H36 N4 O9 S3 | 872.16444 | 0.0 | 20.3 |
| 2-1-m1E03-1-0972 | C40 H35 N5 O10 S4 | 873.12668 | -1.8 | -0.1 |

(continued)

| [Table 11-2-3] | | | | |
|---|---|---|---|---|
| | | | AS-MS ratio (%) | |
| ID | Molecular Formula | Exact Mass | N1 | N2 |
| 2-1-m1E03-1-0973 | C44 H34 N4 O8 S4 | 874.12595 | -1.1 | 2.8 |
| 2-1-m1E03-1-0974 | C40 H31 F2 N5 O8 S4 | 875.10235 | 0.0 | -14.5 |
| 2-1-m1E03-1-0975 | C39 H31 F6 N5 O8 S2 | 875.15182 | -10.3 | -1.9 |
| 2-1-m1E03-1-0976 | C43 H40 F3 N5 O8 S2 | 875.22704 | 5.3 | 8.2 |
| 2-1-m1E03-1-0977 | C39 H33 F N6 O9 S4 | 876.11759 | -2.0 | 2.0 |
| 2-1-m1E03-1-0978 | C36 H30 F3 N5 O10 S4 | 877.08276 | -3.1 | -0.6 |
| 2-1-m1E03-1-0979 | C40 H33 F2 N5 O10 S3 | 877.13576 | 7.0 | -8.4 |
| 2-1-m1E03-1-0980 | C40 H33 F3 N6 O8 S3 | 878.14741 | 0.0 | -7.7 |
| 2-1-m1E03-1-0981 | C39 H31 F2 N5 O9 S4 | 879.09727 | 10.9 | -5.5 |
| 2-1-m1E03-1-0982 | C41 H38 N6 O9 S4 | 886.15831 | -5.7 | 1.9 |
| 2-1-m1E03-1-0983 | C40 H36 N6 O10 S4 | 888.13757 | 22.8 | -3.6 |
| 2-1-m1E03-1-0984 | C44 H35 N5 O8 S4 | 889.13685 | -9.3 | 1.8 |
| 2-1-m1E03-1-0985 | C42 H33 F3 N4 O9 S3 | 890.13618 | 1.4 | 7.4 |
| 2-1-m1E03-1-0986 | C41 H31 F3 N4 O8 S4 | 892.09768 | 0.0 | 0.0 |
| 2-1-m1E03-1-0987 | C39 H32 F2 N6 O9 S4 | 894.10817 | 0.0 | 0.0 |
| 2-1-m1E03-1-0988 | C41 H34 N6 O10 S4 | 898.12192 | -3.0 | -8.4 |
| 2-1-m1E03-1-0989 | C40 H32 N6 O9 S5 | 900.08343 | 2.5 | 2.1 |
| 2-1-m1E03-1-0990 | C42 H33 F3 N4 O10 S3 | 906.13109 | 8.2 | -10.5 |
| 2-1-m1E03-1-0991 | C41 H32 F3 N5 O8 S4 | 907.10858 | 4.5 | -3.2 |
| 2-1-m1E03-1-0992 | C41 H31 F3 N4 O9 S4 | 908.09260 | 9.5 | 2.5 |
| 2-1-m1E03-1-0993 | C41 H34 F3 N5 O10 S3 | 909.14199 | -4.4 | -7.1 |
| 2-1-m1E03-1-0994 | C40 H32 F3 N5 O9 S4 | 911.10350 | 1.5 | 4.3 |
| 2-1-m1E03-1-0995 | C40 H33 N7 O9 S5 | 915.09433 | 0.0 | 0.0 |
| 2-1-m1E03-1-0996 | C41 H32 F3 N5 O9 S4 | 923.10350 | -29.3 | -29.0 |
| 2-1-m1E03-1-0997 | C41 H34 F3 N5 O11 S3 | 925.13690 | -25.2 | -24.6 |
| 2-1-m1E03-1-0998 | C40 H33 F3 N6 O9 S4 | 926.11439 | 0.2 | -32.4 |
| 2-1-m1E03-1-0999 | C40 H32 F3 N5 O10 S4 | 927.09841 | 20.8 | 2.8 |
| 2-1-m1E03-1-1000 | C40 H33 F3 N6 O10 S4 | 942.10931 | -6.8 | 1.9 |

[Table 593]

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0001 | C44 H43 N5 O5 S | 753.29849 | -1.2 | -17.1 |
| 2-1-m1E04-1-0002 | C43 H42 N6 O5 S | 754.29374 | -15.2 | -10.2 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0003 | C43 H42 N6 O5 S | 754.29374 | 0.0 | 0.0 |
| 2-1-m1E04-1-0004 | C42 H41 N7 O5 S | 755.28899 | 6.2 | -8.2 |
| 2-1-m1E04-1-0005 | C42 H41 N7 O5 S | 755.28899 | 5.9 | -4.3 |
| 2-1-m1E04-1-0006 | C41 H40 N8 O5 S | 756.28424 | 0.0 | 0.0 |
| 2-1-m1E04-1-0007 | C41 H40 N6 05 S2 | 760.25016 | -2.9 | -12.6 |
| 2-1-m1E04-1-0008 | C40 H39 N7 O5 S2 | 761.24541 | 0.0 | 0.0 |
| 2-1-m1E04-1-0009 | C45 H45 N5 O5 S | 767.31414 | 0.0 | 0.0 |
| 2-1-m1E04-1-0010 | C44 H44 N6 O5 S | 768.30939 | -3.6 | -4.5 |
| 2-1-m1E04-1-0011 | C43 H43 N7 O5 S | 769.30464 | -5.7 | -5.6 |
| 2-1-m1E04-1-0012 | C43 H41 N5 O7 S | 771.27267 | -2.0 | -7.0 |
| 2-1-m1E04-1-0013 | C44 H42 F N5 O5 S | 771.28907 | -8.7 | -11.6 |
| 2-1-m1E04-1-0014 | C42 H40 N6 O7 S | 772.26792 | -6.3 | -8.5 |
| 2-1-m1E04-1-0015 | C43 H41 F N6 O5 S | 772.28432 | -0.3 | -3.5 |
| 2-1-m1E04-1-0016 | C43 H44 N6 O6 S | 772.30430 | -11.7 | -1.5 |
| 2-1-m1E04-1-0017 | C42 H40 F N7 O5 S | 773.27957 | 0.2 | -4.6 |
| 2-1-m1E04-1-0018 | C42 H43 N7 O6 S | 773.29955 | 0.0 | 0.0 |
| 2-1-m1E04-1-0019 | C42 H43 N7 O6 S | 773.29955 | -10.6 | -12.2 |
| 2-1-m1E04-1-0020 | C42 H42 N6 O5 S2 | 774.26581 | -8.9 | -9.3 |
| 2-1-m1E04-1-0021 | C41 H42 N8 O6 S | 774.29480 | 0.0 | 0.0 |
| 2-1-m1E04-1-0022 | C41 H42 N8 O6 S | 774.29480 | 0.0 | 0.0 |
| 2-1-m1E04-1-0023 | C40 H41 N9 O6 S | 775.29005 | 0.0 | 0.0 |
| 2-1-m1E04-1-0024 | C41 H39 F N6 O5 S2 | 778.24074 | 8.8 | -9.6 |
| 2-1-m1E04-1-0025 | C40 H41 N7 O6 S2 | 779.25597 | 0.0 | 0.0 |
| 2-1-m1E04-1-0026 | C39 H40 N8 O6 S2 | 780.25122 | 0.0 | 0.0 |
| 2-1-m1E04-1-0027 | C45 H43 N5 O6 S | 781.29340 | -9.9 | -19.1 |
| 2-1-m1E04-1-0028 | C46 H47 N5 O5 S | 781.32979 | -6.6 | -13.0 |
| 2-1-m1E04-1-0029 | C44 H42 N6 O6 S | 782.28865 | 1.3 | -16.7 |
| 2-1-m1E04-1-0030 | C44 H42 N6 O6 S | 782.28865 | -7.2 | -5.3 |
| 2-1-m1E04-1-0031 | C45 H46 N6 O5 S | 782.32504 | 0.0 | 0.0 |
| 2-1-m1E04-1-0032 | C43 H41 N7 O6 S | 783.28390 | -8.7 | -9.4 |
| 2-1-m1E04-1-0033 | C45 H45 N5 O6 S | 783.30905 | 6.0 | 0.0 |
| 2-1-m1E04-1-0034 | C44 H45 N7 O5 S | 783.32029 | -8.3 | -13.3 |
| 2-1-m1E04-1-0035 | C44 H44 N6 O6 S | 784.30430 | 0.0 | 0.0 |
| 2-1-m1E04-1-0036 | C44 H43 N5 O7 S | 785.28832 | -7.2 | -11.3 |
| 2-1-m1E04-1-0037 | C43 H43 N7 O6 S | 785.29955 | 0.0 | 0.0 |
| 2-1-m1E04-1-0038 | C44 H46 N6 O6 S | 786.31995 | 0.0 | 10.2 |
| 2-1-m1E04-1-0039 | C43 H41 N5 O6 S2 | 787.24983 | -4.3 | -9.9 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0040 | C43 H45 N7 O6 S | 787.31520 | -6.0 | -5.4 |
| 2-1-m1E04-1-0041 | C42 H40 N6 O6 S2 | 788.24507 | 0.0 | 0.0 |
| 2-1-m1E04-1-0042 | C43 H44 N6 O5 S2 | 788.28146 | 4.1 | -5.5 |
| 2-1-m1E04-1-0043 | C42 H44 N8 O6 S | 788.31045 | -8.3 | -4.8 |
| 2-1-m1E04-1-0044 | C43 H40 F N5 O7 S | 789.26325 | -5.1 | -9.5 |
| 2-1-m1E04-1-0045 | C44 H41 F2 N5 O5 S | 789.27965 | -9.7 | 0.0 |
| 2-1-m1E04-1-0046 | C42 H42 N6 O6 S2 | 790.26072 | -5.6 | 0.0 |
| 2-1-m1E04-1-0047 | C43 H40 F2 N6 O5 S | 790.27490 | -12.1 | -16.3 |
| 2-1-m1E04-1-0048 | C42 H42 N6 O8 S | 790.27848 | -2.3 | -11.2 |
| 2-1-m1E04-1-0049 | C43 H43 F N6 O6 S | 790.29488 | 2.5 | -9.2 |
| 2-1-m1E04-1-0050 | C42 H39 F2 N7 O5 S | 791.27014 | -3.1 | -12.5 |
| 2-1-m1E04-1-0051 | C41 H41 N7 O8 S | 791.27373 | -1.9 | -7.2 |
| 2-1-m1E04-1-0052 | C42 H42 F N7 O6 S | 791.29013 | 2.8 | -14.1 |
| 2-1-m1E04-1-0053 | C41 H41 F N8 O6 S | 792.28538 | 0.0 | -2.9 |
| 2-1-m1E04-1-0054 | C41 H43 N7 O6 S2 | 793.27162 | 3.0 | -8.3 |
| 2-1-m1E04-1-0055 | C46 H45 N5 O6 S | 795.30905 | -2.9 | -6.3 |
| 2-1-m1E04-1-0056 | C41 H38 F2 N6 05 S2 | 796.23132 | -2.6 | -3.3 |
| 2-1-m1E04-1-0057 | C45 H44 N6 O6 S | 796.30430 | -3.8 | -7.1 |
| 2-1-m1E04-1-0058 | C45 H44 N6 O6 S | 796.30430 | -3.1 | -9.6 |
| 2-1-m1E04-1-0059 | C40 H40 F N7 O6 S2 | 797.24655 | 0.0 | 0.0 |
| 2-1-m1E04-1-0060 | C44 H43 N7 O6 S | 797.29955 | 0.0 | 13.5 |
| 2-1-m1E04-1-0061 | C45 H42 F N5 O6 S | 799.28398 | -12.6 | -12.7 |
| 2-1-m1E04-1-0062 | C45 H42 F N5 O6 S | 799.28398 | 0.0 | 0.0 |
| 2-1-m1E04-1-0063 | C45 H45 N5 O7 S | 799.30397 | -10.8 | -21.7 |
| 2-1-m1E04-1-0064 | C44 H41 F N6 O6 S | 800.27923 | -7.4 | -6.5 |
| 2-1-m1E04-1-0065 | C44 H41 F N6 O6 S | 800.27923 | 0.0 | 0.0 |
| 2-1-m1E04-1-0066 | C44 H44 N6 O7 S | 800.29922 | -1.9 | -9.9 |
| 2-1-m1E04-1-0067 | C45 H48 N6 O6 S | 800.33560 | -12.3 | -20.4 |
| 2-1-m1E04-1-0068 | C44 H43 N5 O6 S2 | 801.26548 | -7.2 | -9.1 |
| 2-1-m1E04-1-0069 | C44 H43 N5 O8 S | 801.28323 | -5.3 | -5.3 |
| 2-1-m1E04-1-0070 | C43 H43 N7 O7 S | 801.29447 | 0.0 | 0.0 |
| 2-1-m1E04-1-0071 | C43 H43 N7 O7 S | 801.29447 | -8.4 | -5.3 |
| 2-1-m1E04-1-0072 | C44 H47 N7 O6 S | 801.33085 | 0.2 | -3.5 |
| 2-1-m1E04-1-0073 | C42 H42 N8 O7 S | 802.28972 | 0.0 | 0.0 |
| 2-1-m1E04-1-0074 | C44 H46 N6 O7 S | 802.31487 | 0.0 | 0.0 |
| 2-1-m1E04-1-0075 | C43 H46 N8 O6 S | 802.32610 | 0.0 | 0.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0076 | C43 H45 N7 O7 S | 803.31012 | 12.7 | -8.0 |
| 2-1-m1E04-1-0077 | C48 H45 N5 O5 S | 803.31414 | -10.8 | -10.3 |
| 2-1-m1E04-1-0078 | C43 H44 N6 O8 S | 804.29413 | -8.6 | -1.5 |
| 2-1-m1E04-1-0079 | C42 H44 N8 O7 S | 804.30537 | 9.5 | 0.0 |
| 2-1-m1E04-1-0080 | C47 H44 N6 O5 S | 804.30939 | 0.0 | 0.0 |
| 2-1-m1E04-1-0081 | C43 H40 F N5 O6 S2 | 805.24040 | -7.2 | -6.0 |
| 2-1-m1E04-1-0082 | C46 H43 N7 O5 S | 805.30464 | -9.5 | -14.6 |
| 2-1-m1E04-1-0083 | C46 H43 N7 O5 S | 805.30464 | 6.4 | 0.0 |
| 2-1-m1E04-1-0084 | C42 H42 N6 O7 S2 | 806.25564 | -5.1 | -10.1 |
| 2-1-m1E04-1-0085 | C45 H42 N8 O5 S | 806.29989 | -9.6 | 12.7 |
| 2-1-m1E04-1-0086 | C42 H41 N5 O8 S2 | 807.23965 | -5.2 | -17.0 |
| 2-1-m1E04-1-0087 | C41 H41 N7 O7 S2 | 807.25089 | 0.0 | -2.4 |
| 2-1-m1E04-1-0088 | C43 H39 F2 N5 O7 S | 807.25383 | -5.2 | -8.0 |
| 2-1-m1E04-1-0089 | C42 H45 N7 O6 S2 | 807.28727 | 0.0 | 0.0 |
| 2-1-m1E04-1-0090 | C41 H40 N6 O8 S2 | 808.23490 | -5.7 | 2.0 |
| 2-1-m1E04-1-0091 | C42 H41 F N6 O8 S | 808.26906 | -8.3 | -15.7 |
| 2-1-m1E04-1-0092 | C43 H42 F2 N6 O6 S | 808.28546 | 0.0 | 0.0 |
| 2-1-m1E04-1-0093 | C41 H43 N7 O7 S2 | 809.26654 | 0.0 | 0.0 |
| 2-1-m1E04-1-0094 | C46 H43 N5 O5 S2 | 809.27056 | 0.0 | 0.0 |
| 2-1-m1E04-1-0095 | C42 H41 F2 N7 O6 S | 809.28071 | 6.7 | -1.7 |
| 2-1-m1E04-1-0096 | C43 H47 N5 O7 S2 | 809.29169 | 2.9 | -6.5 |
| 2-1-m1E04-1-0097 | C47 H47 N5 O6 S | 809.32470 | -20.1 | 8.9 |
| 2-1-m1E04-1-0098 | C45 H42 N6 05 S2 | 810.26581 | -7.3 | -12.9 |
| 2-1-m1E04-1-0099 | C45 H42 N6 O5 S2 | 810.26581 | 0.0 | 0.0 |
| 2-1-m1E04-1-0100 | C41 H40 F2 N8 O6 S | 810.27596 | -10.5 | -7.7 |
| 2-1-m1E04-1-0101 | C42 H46 N6 O7 S2 | 810.28694 | -6.6 | -9.6 |
| 2-1-m1E04-1-0102 | C46 H46 N6 O6 S | 810.31995 | -14.9 | -8.8 |
| 2-1-m1E04-1-0103 | C46 H46 N6 O6 S | 810.31995 | -7.5 | 0.0 |
| 2-1-m1E04-1-0104 | C46 H45 N5 O7 S | 811.30397 | 14.7 | 0.0 |
| 2-1-m1E04-1-0105 | C45 H44 N6 O7 S | 812.29922 | 0.0 | -16.8 |
| 2-1-m1E04-1-0106 | C46 H44 F N5 O6 S | 813.29963 | -6.1 | -15.4 |
| 2-1-m1E04-1-0107 | C47 H51 N5 O6 S | 813.35601 | -3.9 | 7.4 |
| 2-1-m1E04-1-0108 | C45 H43 F N6 O6 S | 814.29488 | 1.3 | -14.6 |
| 2-1-m1E04-1-0109 | C45 H46 N6 O7 S | 814.31487 | -5.8 | -12.1 |
| 2-1-m1E04-1-0110 | C46 H50 N6 O6 S | 814.35125 | -6.1 | 0.0 |
| 2-1-m1E04-1-0111 | C40 H39 F2 N7 O6 S2 | 815.23713 | 0.0 | 0.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0112 | C45 H45 N5 O6 S2 | 815.28113 | 0.0 | -11.3 |
| 2-1-m1E04-1-0113 | C44 H45 N7 O7 S | 815.31012 | 3.9 | -3.8 |
| 2-1-m1E04-1-0114 | C44 H45 N7 O7 S | 815.31012 | -3.1 | -5.3 |
| 2-1-m1E04-1-0115 | C43 H44 N8 O7 S | 816.30537 | -9.0 | 0.0 |
| 2-1-m1E04-1-0116 | C44 H43 N5 O7 S2 | 817.26039 | -2.1 | -11.2 |
| 2-1-m1E04-1-0117 | C44 H43 N5 O7 S2 | 817.26039 | 0.0 | 0.0 |
| 2-1-m1E04-1-0118 | C44 H43 N5 O7 S2 | 817.26039 | 5.6 | -2.7 |
| 2-1-m1E04-1-0119 | C45 H41 F2 N5 O6 S | 817.27456 | -0.3 | -11.3 |
| 2-1-m1E04-1-0120 | C45 H41 F2 N5 O6 S | 817.27456 | 1.8 | -5.7 |
| 2-1-m1E04-1-0121 | C43 H42 N6 O7 S2 | 818.25564 | -7.0 | -12.2 |
| 2-1-m1E04-1-0122 | C43 H42 N6 O7 S2 | 818.25564 | -1.0 | -3.9 |
| 2-1-m1E04-1-0123 | C43 H42 N6 O7 S2 | 818.25564 | 0.0 | 0.0 |
| 2-1-m1E04-1-0124 | C44 H40 F2 N6 O6 S | 818.26981 | -0.4 | -7.0 |
| 2-1-m1E04-1-0125 | C44 H43 F N6 O7 S | 818.28980 | -4.7 | -11.3 |
| 2-1-m1E04-1-0126 | C44 H43 F N6 O7 S | 818.28980 | -2.3 | -7.0 |
| 2-1-m1E04-1-0127 | C44 H46 N6 O8 S | 818.30978 | -5.4 | -9.6 |
| 2-1-m1E04-1-0128 | C42 H41 N7 O7 S2 | 819.25089 | 0.0 | 5.9 |
| 2-1-m1E04-1-0129 | C42 H41 N7 O7 S2 | 819.25089 | 5.2 | -1.5 |
| 2-1-m1E04-1-0130 | C43 H42 F N7 O7 S | 819.28505 | 0.0 | 0.0 |
| 2-1-m1E04-1-0131 | C43 H42 F N7 O7 S | 819.28505 | 0.0 | 0.0 |
| 2-1-m1E04-1-0132 | C47 H45 N7 O5 S | 819.32029 | 0.0 | 0.0 |
| 2-1-m1E04-1-0133 | C41 H40 N8 O7 S2 | 820.24614 | 0.0 | 0.0 |
| 2-1-m1E04-1-0134 | C43 H44 N6 O7 S2 | 820.27129 | -10.0 | 31.4 |
| 2-1-m1E04-1-0135 | C43 H44 N6 O9 S | 820.28905 | -2.9 | -16.4 |
| 2-1-m1E04-1-0136 | C43 H43 N5 O8 S2 | 821.25530 | 19.5 | 0.0 |
| 2-1-m1E04-1-0137 | C45 H42 F3 N5 O5 S | 821.28587 | 2.8 | -7.4 |
| 2-1-m1E04-1-0138 | C45 H42 F3 N5 O5 S | 821.28587 | -10.8 | -7.5 |
| 2-1-m1E04-1-0139 | C47 H43 N5 O7 S | 821.28832 | 2.8 | -7.4 |
| 2-1-m1E04-1-0140 | C44 H41 F3 N6 O5 S | 822.28112 | -10.5 | 9.3 |
| 2-1-m1E04-1-0141 | C44 H41 F3 N6 O5 S | 822.28112 | -0.6 | 0.0 |
| 2-1-m1E04-1-0142 | C42 H41 N5 O7 S3 | 823.21681 | 0.0 | 6.7 |
| 2-1-m1E04-1-0143 | C43 H39 F2 N5 O6 S2 | 823.23098 | -6.1 | -10.1 |
| 2-1-m1E04-1-0144 | C43 H40 F3 N7 O5 S | 823.27637 | -10.0 | -9.3 |
| 2-1-m1E04-1-0145 | C47 H45 N5 05 S2 | 823.28621 | 0.0 | 0.0 |
| 2-1-m1E04-1-0146 | C46 H42 F N7 O5 S | 823.29522 | -24.5 | -14.5 |
| 2-1-m1E04-1-0147 | C44 H49 N5 O7 S2 | 823.30734 | -66.8 | 0.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0148 | C41 H40 N6 O7 S3 | 824.21206 | -6.6 | -3.6 |
| 2-1-m1E04-1-0149 | C41 H40 N6 O7 S3 | 824.21206 | 1.3 | -12.2 |
| 2-1-m1E04-1-0150 | C42 H41 F N6 O7 S2 | 824.24622 | -5.9 | -11.7 |
| 2-1-m1E04-1-0151 | C45 H44 N8 O6 S | 824.31045 | -19.5 | 0.0 |
| 2-1-m1E04-1-0152 | C47 H48 N6 O6 S | 824.33560 | 0.0 | 0.6 |
| 2-1-m1E04-1-0153 | C40 H39 N7 O7 S3 | 825.20731 | 13.8 | 0.0 |
| 2-1-m1E04-1-0154 | C42 H40 F N5 O8 S2 | 825.23023 | 0.0 | 0.0 |
| 2-1-m1E04-1-0155 | C44 H43 N9 O6 S | 825.30570 | 0.0 | 0.0 |
| 2-1-m1E04-1-0156 | C41 H42 N6 O9 S2 | 826.24547 | -4.7 | -11.3 |
| 2-1-m1E04-1-0157 | C42 H40 F2 N6 O8 S | 826.25964 | -5.0 | -21.5 |
| 2-1-m1E04-1-0158 | C46 H46 N6 O7 S | 826.31487 | 9.0 | 0.0 |
| 2-1-m1E04-1-0159 | C40 H41 N7 O9 S2 | 827.24072 | -6.8 | 0.3 |
| 2-1-m1E04-1-0160 | C46 H42 F N5 O5 S2 | 827.26114 | -17.1 | -30.0 |
| 2-1-m1E04-1-0161 | C43 H46 F N5 O7 S2 | 827.28227 | 0.0 | 0.0 |
| 2-1-m1E04-1-0162 | C47 H46 F N5 O6 S | 827.31528 | 0.0 | 0.0 |
| 2-1-m1E04-1-0163 | C48 H53 N5 O6 S | 827.37166 | -42.2 | -24.2 |
| 2-1-m1E04-1-0164 | C42 H39 F3 N6 O5 S2 | 828.23754 | -10.0 | -9.5 |
| 2-1-m1E04-1-0165 | C45 H44 N6 O6 S2 | 828.27637 | 0.0 | 0.0 |
| 2-1-m1E04-1-0166 | C42 H48 N6 O8 S2 | 828.29750 | -5.1 | -9.9 |
| 2-1-m1E04-1-0167 | C46 H48 N6 O7 S | 828.33052 | -4.4 | -12.9 |
| 2-1-m1E04-1-0168 | C44 H43 N7 O6 S2 | 829.27162 | 0.0 | 0.0 |
| 2-1-m1E04-1-0169 | C44 H43 N7 O6 S2 | 829.27162 | 0.0 | 0.0 |
| 2-1-m 1 E04-1-0170 | C41 H47 N7 O8 S2 | 829.29275 | 0.0 | 0.0 |
| 2-1-m1E04-1-0171 | C46 H44 F N5 O7 S | 829.29455 | 0.0 | 0.0 |
| 2-1-m1E04-1-0172 | C46 H47 N5 O6 S2 | 829.29678 | -5.9 | -27.9 |
| 2-1-m1E04-1-0173 | C45 H47 N7 O7 S | 829.32577 | -16.9 | 7.8 |
| 2-1-m1E04-1-0174 | C45 H47 N7 O7 S | 829.32577 | 1.0 | 0.0 |
| 2-1-m1E04-1-0175 | C43 H42 N8 O6 S2 | 830.26687 | 5.1 | 14.8 |
| 2-1-m1E04-1-0176 | C45 H46 N6 O6 S2 | 830.29202 | -10.1 | -24.6 |
| 2-1-m1E04-1-0177 | C45 H46 N6 O8 S | 830.30978 | 0.0 | 0.0 |
| 2-1-m1E04-1-0178 | C42 H41 N9 O6 S2 | 831.26212 | -4.1 | 4.5 |
| 2-1-m1E04-1-0179 | C45 H45 N5 O7 S2 | 831.27604 | -1.5 | -13.6 |
| 2-1-m1E04-1-0180 | C45 H45 N5 O7 S2 | 831.27604 | 0.0 | 0.0 |
| 2-1-m1E04-1-0181 | C45 H45 N5 O7 S2 | 831.27604 | 0.0 | 0.0 |
| 2-1-m1E04-1-0182 | C44 H45 N7 O8 S | 831.30503 | 0.0 | 0.0 |
| 2-1-m1E04-1-0183 | C49 H45 N5 O6 S | 831.30905 | 1.3 | -16.8 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0184 | C47 H50 F N5 O6 S | 831.34658 | -1.7 | -11.2 |
| 2-1-m1E04-1-0185 | C44 H44 N6 O7 S2 | 832.27129 | 19.9 | 0.0 |
| 2-1-m1E04-1-0186 | C44 H44 N6 O7 S2 | 832.27129 | 4.5 | -12.4 |
| 2-1-m1E04-1-0187 | C44 H44 N6 O7 S2 | 832.27129 | -1.8 | -2.3 |
| 2-1-m1E04-1-0188 | C45 H42 F2 N6 O6 S | 832.28546 | 0.0 | 4.4 |
| 2-1-m1E04-1-0189 | C48 H44 N6 O6 S | 832.30430 | -4.8 | -10.7 |
| 2-1-m1E04-1-0190 | C45 H45 F N6 O7 S | 832.30545 | -79.9 | -20.3 |
| 2-1-m1E04-1-0191 | C46 H52 N6 O7 S | 832.36182 | 0.0 | 0.0 |
| 2-1-m1E04-1-0192 | C43 H43 N7 O7 S2 | 833.26654 | -6.0 | -0.1 |
| 2-1-m1E04-1-0193 | C43 H43 N7 O7 S2 | 833.26654 | 0.0 | 0.0 |
| 2-1-m1E04-1-0194 | C44 H44 F N7 O7 S | 833.30070 | -1.4 | -6.8 |
| 2-1-m1E04-1-0195 | C48 H47 N7 O5 S | 833.33594 | 5.5 | 0.0 |
| 2-1-m1E04-1-0196 | C45 H51 N7 O7 S | 833.35707 | 0.0 | 0.0 |
| 2-1-m1E04-1-0197 | C44 H46 N6 O7 S2 | 834.28694 | -5.3 | -6.7 |
| 2-1-m1E04-1-0198 | C43 H41 N5 O9 S2 | 835.23457 | -3.7 | -12.2 |
| 2-1-m1E04-1-0199 | C44 H42 F N5 O7 S2 | 835.25097 | -3.4 | -19.7 |
| 2-1-m1E04-1-0200 | C44 H42 F N5 O7 S2 | 835.25097 | -14.6 | -15.4 |
| 2-1-m1E04-1-0201 | C45 H40 F3 N5 O6 S | 835.26514 | -4.7 | -10.0 |
| 2-1-m1E04-1-0202 | C44 H45 N5 O8 S2 | 835.27095 | 6.0 | -5.6 |
| 2-1-m1E04-1-0203 | C46 H44 F3 N5 O5 S | 835.30152 | -15.9 | -9.0 |
| 2-1-m1E04-1-0204 | C47 H45 N7 O6 S | 835.31520 | 0.0 | 0.0 |
| 2-1-m1E04-1-0205 | C41 H40 N8 O6 S3 | 836.22329 | -101.9 | 0.0 |
| 2-1-m1E04-1-0206 | C42 H40 N6 O9 S2 | 836.22982 | -1.0 | -1.8 |
| 2-1-m1E04-1-0207 | C43 H41F N6 O7 S2 | 836.24622 | -3.1 | -20.6 |
| 2-1-m1E04-1-0208 | C43 H44 N6 O8 S2 | 836.26620 | -6.6 | -11.3 |
| 2-1-m1E04-1-0209 | C43 H44 N6 O8 S2 | 836.26620 | -5.6 | -5.4 |
| 2-1-m1E04-1-0210 | C43 H44 N6 O8 S2 | 836.26620 | -2.2 | -6.9 |
| 2-1-m1E04-1-0211 | C44 H42 F2 N6 O7 S | 836.28037 | -5.5 | -12.3 |
| 2-1-m1E04-1-0212 | C44 H42 F2 N6 O7 S | 836.28037 | -2.2 | -11.3 |
| 2-1-m1E04-1-0213 | C43 H43 N5 O7 S3 | 837.23246 | 0.0 | 0.0 |
| 2-1-m1E04-1-0214 | C42 H40 F N7 O7 S2 | 837.24147 | 0.0 | 0.0 |
| 2-1-m1E04-1-0215 | C43 H43 N5 O9 S2 | 837.25022 | 0.0 | -20.1 |
| 2-1-m1E04-1-0216 | C42 H43 N7 O8 S2 | 837.26145 | -9.9 | -8.6 |
| 2-1-m1E04-1-0217 | C42 H43 N7 O8 S2 | 837.26145 | -4.9 | 0.0 |
| 2-1-m1E04-1-0218 | C42 H43 N7 O8 S2 | 837.26145 | 0.0 | 0.0 |
| 2-1-m1E04-1-0219 | C47 H43 N5 O6 S2 | 837.26548 | -3.2 | -4.5 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0220 | C43 H41 F2 N7 O7 S | 837.27562 | -3.5 | -20.3 |
| 2-1-m1E04-1-0221 | C45 H42 F3 N5 O6 S | 837.28079 | -6.4 | -13.7 |
| 2-1-m1E04-1-0222 | C48 H47 N5 O5 S2 | 837.30186 | -5.8 | 6.6 |
| 2-1-m1E04-1-0223 | C45 H51 N5 O7 S2 | 837.32299 | 0.0 | 0.0 |
| 2-1-m1E04-1-0224 | C42 H42 N6 O7 S3 | 838.22771 | -5.3 | 6.0 |
| 2-1-m1E04-1-0225 | C41 H42 N8 O8 S2 | 838.25670 | 1.3 | -4.5 |
| 2-1-m1E04-1-0226 | C41 H42 N8 O8 S2 | 838.25670 | 0.0 | 0.0 |
| 2-1-m1E04-1-0227 | C44 H41 F3 N6 O6 S | 838.27604 | 13.0 | -5.0 |
| 2-1-m1E04-1-0228 | C46 H46 N8 O6 S | 838.32610 | -6.9 | -4.8 |
| 2-1-m1E04-1-0229 | C40 H41 N9 O8 S2 | 839.25195 | 0.0 | 0.0 |
| 2-1-m1E04-1-0230 | C44 H40 F3 N5 O7 S | 839.26005 | 0.0 | -2.4 |
| 2-1-m1E04-1-0231 | C43 H40 F3 N7 O6 S | 839.27129 | -6.8 | -14.9 |
| 2-1-m1E04-1-0232 | C45 H41 F4 N5 O5 S | 839.27645 | -3.4 | -26.8 |
| 2-1-m1E04-1-0233 | C47 H45 N5 O6 S2 | 839.28113 | -3.9 | -9.9 |
| 2-1-m1E04-1-0234 | C44 H49 N5 O8 S2 | 839.30225 | 0.0 | 0.0 |
| 2-1-m1E04-1-0235 | C42 H44 N6 O9 S2 | 840.26112 | -2.1 | -9.7 |
| 2-1-m1E04-1-0236 | C44 H43 F3 N6 O6 S | 840.29169 | 4.7 | 0.0 |
| 2-1-m1E04-1-0237 | C44 H43 F3 N6 O6 S | 840.29169 | 5.3 | -18.3 |
| 2-1-m1E04-1-0238 | C42 H40 F N5 O7 S3 | 841.20739 | -6.6 | -9.0 |
| 2-1-m1E04-1-0239 | C46 H41 F2 N7 O5 S | 841.28579 | 0.0 | 0.0 |
| 2-1-m1E04-1-0240 | C43 H42 F3 N7 O6 S | 841.28694 | 0.0 | 2.9 |
| 2-1-m1E04-1-0241 | C43 H42 F3 N7 O6 S | 841.28694 | 0.0 | 0.0 |
| 2-1-m1E04-1-0242 | C49 H55 N5 O6 S | 841.38731 | 0.0 | 0.0 |
| 2-1-m1E04-1-0243 | C41 H39 F N6 O7 S3 | 842.20264 | -1.5 | -12.0 |
| 2-1-m1E04-1-0244 | C41 H42 N6 O8 S3 | 842.22262 | -3.9 | -11.8 |
| 2-1-m1E04-1-0245 | C42 H40 F2 N6 O7 S2 | 842.23680 | -4.7 | -12.7 |
| 2-1-m1E04-1-0246 | C42 H41 F3 N8 O6 S | 842.28219 | 0.0 | 0.0 |
| 2-1-m1E04-1-0247 | C46 H46 N6 O6 S2 | 842.29202 | 2.0 | 0.0 |
| 2-1-m1E04-1-0248 | C45 H43 F N8 O6 S | 842.30103 | 0.0 | 0.0 |
| 2-1-m1E04-1-0249 | C43 H50 N6 O8 S2 | 842.31315 | -7.7 | -10.4 |
| 2-1-m1E04-1-0250 | C40 H41 N7 O8 S3 | 843.21787 | -12.0 | -11.6 |
| 2-1-m1E04-1-0251 | C40 H41 N7 O8 S3 | 843.21787 | -12.7 | -13.9 |
| 2-1-m1E04-1-0252 | C42 H39 F2 N5 O8 S2 | 843.22081 | -5.3 | -10.5 |
| 2-1-m1E04-1-0253 | C47 H49 N5 O6 S2 | 843.31243 | -66.1 | 0.0 |
| 2-1-m1E04-1-0254 | C47 H49 N5 O6 S2 | 843.31243 | 0.0 | 0.0 |
| 2-1-m1E04-1-0255 | C46 H49 N7 O7 S | 843.34142 | 0.0 | -10.4 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0256 | C48 H53 N5 O7 S | 843.36657 | 0.0 | 0.0 |
| 2-1-m1E04-1-0257 | C39 H40 N8 O8 S3 | 844.21312 | 0.0 | 0.0 |
| 2-1-m1E04-1-0258 | C42 H39 F3 N6 O6 S2 | 844.23246 | -4.7 | -20.9 |
| 2-1-m1E04-1-0259 | C41 H41 FN6 O9 S2 | 844.23605 | -6.4 | -10.5 |
| 2-1-m1E04-1-0260 | C46 H48 N6 O6 S2 | 844.30767 | -27.1 | -20.2 |
| 2-1-m1E04-1-0261 | C46 H41 F2 N5 05 S2 | 845.25172 | 1.6 | 0.0 |
| 2-1-m1E04-1-0262 | C45 H43 N5 O8 S2 | 845.25530 | 0.0 | 0.0 |
| 2-1-m1E04-1-0263 | C43 H45 F2 N5 O7 S2 | 845.27285 | -6.9 | -10.4 |
| 2-1-m1E04-1-0264 | C46 H47 N5 O7 S2 | 845.29169 | 0.0 | -5.0 |
| 2-1-m1E04-1-0265 | C46 H47 N5 O7 S2 | 845.29169 | -18.1 | -12.3 |
| 2-1-m1E04-1-0266 | C46 H47 N5 O7 S2 | 845.29169 | 0.0 | -8.9 |
| 2-1-m1E04-1-0267 | C46 H47 N5 O7 S2 | 845.29169 | 13.8 | -16.2 |
| 2-1-m1E04-1-0268 | C45 H47 N7 O8 S | 845.32068 | 0.0 | -11.7 |
| 2-1-m1E04-1-0269 | C44 H42 N6 O8 S2 | 846.25055 | 0.2 | -4.2 |
| 2-1-m1E04-1-0270 | C44 H42 N6 O8 S2 | 846.25055 | 1.6 | -5.3 |
| 2-1-m1E04-1-0271 | C45 H43 F N6 O6 S2 | 846.26695 | 0.0 | 0.0 |
| 2-1-m1E04-1-0272 | C45 H46 N6 O7 S2 | 846.28694 | -5.1 | -10.9 |
| 2-1-m1E04-1-0273 | C45 H46 N6 O7 S2 | 846.28694 | 0.0 | 0.0 |
| 2-1-m1E04-1-0274 | C45 H46 N6 O7 S2 | 846.28694 | 0.0 | 0.0 |
| 2-1-m1E04-1-0275 | C42 H47 F N6 O8 S2 | 846.28808 | -3.0 | -9.2 |
| 2-1-m1E04-1-0276 | C49 H46 N6 O6 S | 846.31995 | -6.7 | -5.6 |
| 2-1-m1E04-1-0277 | C46 H47 F N6 O7 S | 846.32110 | 0.0 | -6.8 |
| 2-1-m1E04-1-0278 | C47 H54 N6 O7 S | 846.37747 | -3.6 | -3.9 |
| 2-1-m1E04-1-0279 | C41 H40 F3 N7 O6 S2 | 847.24336 | -42.5 | 37.8 |
| 2-1-m1E04-1-0280 | C43 H41 N7 O8 S2 | 847.24580 | -7.3 | 10.4 |
| 2-1-m1E04-1-0281 | C43 H41 N7 O8 S2 | 847.24580 | -0.6 | -1.2 |
| 2-1-m1E04-1-0282 | C45 H45 N5 O8 S2 | 847.27095 | 1.1 | -9.3 |
| 2-1-m1E04-1-0283 | C45 H45 N5 O8 S2 | 847.27095 | 0.0 | -7.9 |
| 2-1-m1E04-1-0284 | C44 H45 N7 O7 S2 | 847.28219 | -7.8 | -21.9 |
| 2-1-m1E04-1-0285 | C46 H46 F N5 O6 S2 | 847.28735 | -30.5 | -28.2 |
| 2-1-m1E04-1-0286 | C44 H44 N6 O8 S2 | 848.26620 | -4.4 | -10.0 |
| 2-1-m1E04-1-0287 | C45 H45 F N6 O8 S | 848.30036 | 0.0 | 9.3 |
| 2-1-m1E04-1-0288 | C45 H48 N6 O7 S2 | 848.30259 | 9.2 | 7.7 |
| 2-1-m1E04-1-0289 | C44 H43 N5 O9 S2 | 849.25022 | -10.0 | -10.7 |
| 2-1-m1E04-1-0290 | C43 H43 N7 O8 S2 | 849.26145 | 0.0 | 0.0 |
| 2-1-m1E04-1-0291 | C45 H44 F N5 O7 S2 | 849.26662 | 0.0 | -6.5 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0292 | C45 H44 F N5 O7 S2 | 849.26662 | 0.0 | -5.3 |
| 2-1-m1E04-1-0293 | C46 H42 F3 N5 O6 S | 849.28079 | -10.0 | -14.5 |
| 2-1-m1E04-1-0294 | C44 H47 N7 O7 S2 | 849.29784 | 0.0 | 0.0 |
| 2-1-m1E04-1-0295 | C49 H44 F N5 O6 S | 849.29963 | 0.0 | 0.0 |
| 2-1-m1E04-1-0296 | C47 H46 F3 N5 O5 S | 849.31717 | -11.8 | -6.2 |
| 2-1-m1E04-1-0297 | C47 H49 F2 N5 O6 S | 849.33716 | -15.3 | -10.9 |
| 2-1-m1E04-1-0298 | C44 H43 F N6 O7 S2 | 850.26187 | 10.2 | 0.0 |
| 2-1-m1E04-1-0299 | C44 H43 F N6 O7 S2 | 850.26187 | 0.0 | -10.6 |
| 2-1-m1E04-1-0300 | C45 H41 F3 N6 O6 S | 850.27604 | -10.1 | -9.0 |
| 2-1-m1E04-1-0301 | C44 H46 N6 O8 S2 | 850.28185 | -4.8 | -15.6 |
| 2-1-m1E04-1-0302 | C44 H46 N6 O8 S2 | 850.28185 | -7.0 | -8.0 |
| 2-1-m1E04-1-0303 | C44 H46 N6 O8 S2 | 850.28185 | -9.2 | -15.9 |
| 2-1-m1E04-1-0304 | C46 H51 F N6 O7 S | 850.35240 | -11.4 | 4.9 |
| 2-1-m1E04-1-0305 | C43 H41 N5 O8 S3 | 851.21173 | -5.4 | -17.5 |
| 2-1-m1E04-1-0306 | C44 H45 N5 O7 S3 | 851.24811 | -0.8 | -4.0 |
| 2-1-m1E04-1-0307 | C43 H45 N7 O8 S2 | 851.27710 | -15.0 | -6.8 |
| 2-1-m1E04-1-0308 | C43 H45 N7 O8 S2 | 851.27710 | 0.0 | 0.0 |
| 2-1-m1E04-1-0309 | C43 H45 N7 O8 S2 | 851.27710 | 5.2 | -6.2 |
| 2-1-m1E04-1-0310 | C44 H43 F2 N7 O7 S | 851.29127 | -5.6 | -13.5 |
| 2-1-m1E04-1-0311 | C46 H44 F3 N5 O6 S | 851.29644 | 0.0 | 0.0 |
| 2-1-m1E04-1-0312 | C42 H40 N6 O8 S3 | 852.20697 | -1.5 | -4.2 |
| 2-1-m1E04-1-0313 | C43 H44 N6 O7 S3 | 852.24336 | -10.5 | -12.2 |
| 2-1-m1E04-1-0314 | C42 H44 N8 O8 S2 | 852.27235 | 0.0 | 2.7 |
| 2-1-m1E04-1-0315 | C42 H44 N8 O8 S2 | 852.27235 | 0.0 | -4.3 |
| 2-1-m1E04-1-0316 | C47 H48 N8 O6 S | 852.34175 | -8.5 | 0.0 |
| 2-1-m1E04-1-0317 | C43 H40 F N5 O9 S2 | 853.22515 | -7.4 | -13.0 |
| 2-1-m1E04-1-0318 | C43 H43 N5 O8 S3 | 853.22738 | -5.6 | -14.2 |
| 2-1-m1E04-1-0319 | C44 H41 F2 N5 O7 S2 | 853.24155 | -3.9 | -9.2 |
| 2-1-m1E04-1-0320 | C44 H41 F2 N5 O7 S2 | 853.24155 | -3.6 | -11.3 |
| 2-1-m1E04-1-0321 | C42 H42 N6 O8 S3 | 854.22262 | -11.5 | 0.0 |
| 2-1-m1E04-1-0322 | C43 H40 F2 N6 O7 S2 | 854.23680 | -3.2 | 6.0 |
| 2-1-m1E04-1-0323 | C42 H42 N6 O10 S2 | 854.24038 | -2.7 | -8.3 |
| 2-1-m1E04-1-0324 | C43 H43 F N6 O8 S2 | 854.25678 | 0.0 | 0.0 |
| 2-1-m1E04-1-0325 | C43 H43 F N6 O8 S2 | 854.25678 | -6.0 | -13.7 |
| 2-1-m1E04-1-0326 | C44 H41 F3 N6 O7 S | 854.27095 | -4.2 | -19.1 |
| 2-1-m1E04-1-0327 | C43 H46 N6 O9 S2 | 854.27677 | -6.0 | -7.4 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0328 | C45 H45 F3 N6 O6 S | 854.30734 | -14.7 | -8.7 |
| 2-1-m1E04-1-0329 | C46 H46 N8 O7 S | 854.32102 | 0.0 | 0.0 |
| 2-1-m1E04-1-0330 | C42 H39 F2 N7 O7 S2 | 855.23204 | 0.0 | 10.1 |
| 2-1-m1E04-1-0331 | C41 H41 N7 O10 S2 | 855.23563 | -17.2 | -4.4 |
| 2-1-m1E04-1-0332 | C44 H40 F3 N5 O6 S2 | 855.23721 | -14.9 | -7.5 |
| 2-1-m1E04-1-0333 | C42 H42 F N7 O8 S2 | 855.25203 | -39.2 | -12.2 |
| 2-1-m1E04-1-0334 | C44 H40 F3 N5 O8 S | 855.25497 | -11.9 | -14.5 |
| 2-1-m1E04-1-0335 | C50 H45 N7 O5 S | 855.32029 | -0.1 | -12.2 |
| 2-1-m1E04-1-0336 | C42 H44 N6 O8 S3 | 856.23827 | -0.9 | -4.9 |
| 2-1-m1E04-1-0337 | C41 H41 F N8 O8 S2 | 856.24728 | 0.0 | 0.0 |
| 2-1-m1E04-1-0338 | C42 H44 N6 O10 S2 | 856.25603 | -23.0 | -12.1 |
| 2-1-m1E04-1-0339 | C44 H43 F3 N6 O7 S | 856.28660 | -17.5 | 6.3 |
| 2-1-m1E04-1-0340 | C47 H48 N6 O6 S2 | 856.30767 | 0.0 | 1.9 |
| 2-1-m1E04-1-0341 | C44 H52 N6 O8 S2 | 856.32880 | -4.2 | -10.6 |
| 2-1-m1E04-1-0342 | C41 H43 N7 O8 S3 | 857.23352 | 0.0 | 0.0 |
| 2-1-m1E04-1-0343 | C46 H43 N5 O8 S2 | 857.25530 | 0.0 | 0.0 |
| 2-1-m1E04-1-0344 | C45 H43 N7 O7 S2 | 857.26654 | -8.3 | 28.9 |
| 2-1-m1E04-1-0345 | C45 H40 F5 N5 O5 S | 857.26703 | -6.2 | -11.6 |
| 2-1-m1E04-1-0346 | C43 H42 F3 N7 O7 S | 857.28185 | 0.0 | 0.0 |
| 2-1-m1E04-1-0347 | C48 H51 N5 O6 S2 | 857.32808 | 0.0 | -11.0 |
| 2-1-m1E04-1-0348 | C48 H51 N5 O6 S2 | 857.32808 | 0.0 | -26.8 |
| 2-1-m1E04-1-0349 | C48 H51 N5 O6 S2 | 857.32808 | 0.0 | -214.2 |
| 2-1-m1E04-1-0350 | C44 H42 N8 O7 S2 | 858.26179 | -9.3 | -281.4 |
| 2-1-m1E04-1-0351 | C43 H41 F3 N6 O8 S | 858.26587 | -4.8 | -19.8 |
| 2-1-m1E04-1-0352 | C42 H41 F3 N8 O7 S | 858.27710 | -6.8 | -18.9 |
| 2-1-m1E04-1-0353 | C44 H42 F4 N6 O6 S | 858.28227 | -14.9 | 0.0 |
| 2-1-m1E04-1-0354 | C46 H46 N6 O7 S2 | 858.28694 | 6.0 | -19.3 |
| 2-1-m1E04-1-0355 | C43 H50 N6 O9 S2 | 858.30807 | 0.0 | -1.2 |
| 2-1-m1E04-1-0356 | C47 H50 N6 O6 S2 | 858.32332 | -69.6 | 0.0 |
| 2-1-m1E04-1-0357 | C42 H39 F2 N5 O7 S3 | 859.19797 | -4.1 | -8.5 |
| 2-1-m1E04-1-0358 | C46 H45 N5 O8 S2 | 859.27095 | -6.3 | -14.5 |
| 2-1-m1E04-1-0359 | C50 H45 N5 O5 S2 | 859.28621 | 9.8 | 0.0 |
| 2-1-m1E04-1-0360 | C47 H49 N5 O7 S2 | 859.30734 | 0.0 | 5.9 |
| 2-1-m1E04-1-0361 | C47 H49 N5 O7 S2 | 859.30734 | 0.0 | 0.0 |
| 2-1-m1E04-1-0362 | C47 H49 N5 O7 S2 | 859.30734 | 0.0 | 0.0 |
| 2-1-m1E04-1-0363 | C47 H49 N5 O7 S2 | 859.30734 | 0.0 | 0.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0364 | C41 H38 F2 N6 O7 S3 | 860.19322 | -4.7 | 5.1 |
| 2-1-m1E04-1-0365 | C41 H41 F N6 O8 S3 | 860.21320 | -5.0 | -10.4 |
| 2-1-m1E04-1-0366 | C45 H44 N6 O8 S2 | 860.26620 | 1.7 | 1.6 |
| 2-1-m1E04-1-0367 | C45 H44 N6 O8 S2 | 860.26620 | 24.9 | -11.5 |
| 2-1-m1E04-1-0368 | C45 H42 F2 N8 O6 S | 860.29161 | 4.9 | 9.5 |
| 2-1-m1E04-1-0369 | C46 H48 N6 O7 S2 | 860.30259 | 4.0 | 0.0 |
| 2-1-m1E04-1-0370 | C48 H56 N6 O7 S | 860.39312 | -3.3 | -15.1 |
| 2-1-m1E04-1-0371 | C40 H40 F N7 O8 S3 | 861.20845 | 0.0 | 0.0 |
| 2-1-m1E04-1-0372 | C44 H43 N7 O8 S2 | 861.26145 | 0.0 | 0.0 |
| 2-1-m1E04-1-0373 | C46 H47 N5 O8 S2 | 861.28660 | 0.0 | 0.0 |
| 2-1-m1E04-1-0374 | C46 H47 N5 O8 S2 | 861.28660 | -7.7 | -6.8 |
| 2-1-m1E04-1-0375 | C47 H48 F N5 O6 S2 | 861.30300 | 0.0 | 8.6 |
| 2-1-m1E04-1-0376 | C41 H40 F2 N6 O9 S2 | 862.22662 | -1.5 | -7.7 |
| 2-1-m1E04-1-0377 | C45 H46 N6 O8 S2 | 862.28185 | -0.4 | -8.6 |
| 2-1-m1E04-1-0378 | C45 H46 N6 O8 S2 | 862.28185 | 0.0 | 3.5 |
| 2-1-m1E04-1-0379 | C46 H50 N6 O7 S2 | 862.31824 | 0.0 | -18.6 |
| 2-1-m1E04-1-0380 | C46 H50 N6 O7 S2 | 862.31824 | 0.0 | -1.5 |
| 2-1-m1E04-1-0381 | C47 H54 N6 O8 S | 862.37238 | 0.0 | 0.0 |
| 2-1-m1E04-1-0382 | C43 H41 N7 O7 S3 | 863.22296 | -3.9 | -10.4 |
| 2-1-m1E04-1-0383 | C41 H40 F3 N7 O7 S2 | 863.23827 | 2.9 | -25.8 |
| 2-1-m1E04-1-0384 | C45 H42 F N5 O8 S2 | 863.24588 | -3.9 | -10.7 |
| 2-1-m1E04-1-0385 | C45 H42 F N5 O8 S2 | 863.24588 | 0.0 | -4.0 |
| 2-1-m1E04-1-0386 | C45 H45 N5 O9 S2 | 863.26587 | 0.0 | -13.4 |
| 2-1-m1E04-1-0387 | C46 H46 F N5 O7 S2 | 863.28227 | -7.0 | 2.7 |
| 2-1-m1E04-1-0388 | C46 H46 F N5 O7 S2 | 863.28227 | -5.7 | -8.5 |
| 2-1-m1E04-1-0389 | C45 H49 N7 O7 S2 | 863.31349 | 0.0 | 0.0 |
| 2-1-m1E04-1-0390 | C51 H53 N5 O6 S | 863.37166 | 0.0 | 0.0 |
| 2-1-m1E04-1-0391 | C44 H41 F N6 O8 S2 | 864.24113 | 0.0 | 0.5 |
| 2-1-m1E04-1-0392 | C44 H41 F N6 O8 S2 | 864.24113 | -2.3 | -4.4 |
| 2-1-m1E04-1-0393 | C45 H42 F2 N6 O6 S2 | 864.25753 | 0.0 | 0.0 |
| 2-1-m1E04-1-0394 | C44 H44 N6 O9 S2 | 864.26112 | -1.8 | -6.7 |
| 2-1-m1E04-1-0395 | C42 H46 F2 N6 O8 S2 | 864.27866 | -9.6 | -10.5 |
| 2-1-m1E04-1-0396 | C46 H43 F3 N6 O6 S | 864.29169 | -7.5 | -8.1 |
| 2-1-m1E04-1-0397 | C45 H48 N6 O8 S2 | 864.29750 | -5.0 | -10.2 |
| 2-1-m1E04-1-0398 | C45 H48 N6 O8 S2 | 864.29750 | -4.1 | -8.5 |
| 2-1-m1E04-1-0399 | C45 H48 N6 O8 S2 | 864.29750 | -1.1 | -22.1 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0400 | C45 H48 N6 O8 S2 | 864.29750 | 0.8 | -11.9 |
| 2-1-m1E04-1-0401 | C44 H43 N5 O8 S3 | 865.22738 | -5.3 | -9.5 |
| 2-1-m1E04-1-0402 | C44 H43 N5 O10 S2 | 865.24513 | 0.0 | 0.0 |
| 2-1-m1E04-1-0403 | C43 H43 N7 O9 S2 | 865.25637 | -1.3 | -17.3 |
| 2-1-m1E04-1-0404 | C43 H43 N7 O9 S2 | 865.25637 | 0.0 | 0.0 |
| 2-1-m1E04-1-0405 | C46 H42 F3 N5 O7 S | 865.27570 | -10.9 | -15.7 |
| 2-1-m1E04-1-0406 | C46 H45 F2 N5 O6 S2 | 865.27793 | -9.3 | -14.4 |
| 2-1-m1E04-1-0407 | C44 H47 N7 O8 S2 | 865.29275 | -13.7 | -2.3 |
| 2-1-m1E04-1-0408 | C44 H47 N7 O8 S2 | 865.29275 | 0.9 | 14.2 |
| 2-1-m1E04-1-0409 | C44 H47 N7 O8 S2 | 865.29275 | -20.5 | -5.9 |
| 2-1-m1E04-1-0410 | C42 H42 N8 O9 S2 | 866.25162 | 0.0 | 0.0 |
| 2-1-m1E04-1-0411 | C45 H41 F3 N6 O7 S | 866.27095 | -15.1 | -15.4 |
| 2-1-m1E04-1-0412 | C44 H46 N6 O9 S2 | 866.27677 | -13.6 | -12.8 |
| 2-1-m1E04-1-0413 | C44 H46 N6 O9 S2 | 866.27677 | -1.5 | -7.0 |
| 2-1-m1E04-1-0414 | C43 H46 N8 O8 S2 | 866.28800 | 0.0 | 0.0 |
| 2-1-m1E04-1-0415 | C45 H47 F N6 O7 S2 | 866.29317 | 0.0 | 0.0 |
| 2-1-m1E04-1-0416 | C45 H43 F2 N5 O7 S2 | 867.25720 | -3.3 | -8.8 |
| 2-1-m1E04-1-0417 | C45 H43 F2 N5 O7 S2 | 867.25720 | 0.0 | 0.0 |
| 2-1-m1E04-1-0418 | C46 H41 F4 N5 O6 S | 867.27137 | -11.0 | -11.8 |
| 2-1-m1E04-1-0419 | C43 H45 N7 O9 S2 | 867.27202 | -13.6 | -11.6 |
| 2-1-m1E04-1-0420 | C48 H45 N5 O7 S2 | 867.27604 | -5.1 | -15.3 |
| 2-1-m1E04-1-0421 | C48 H45 N5 O7 S2 | 867.27604 | 1.9 | -4.6 |
| 2-1-m1E04-1-0422 | C44 H42 F2 N6 O7 S2 | 868.25245 | 0.0 | 0.0 |
| 2-1-m1E04-1-0423 | C43 H44 N6 O10 S2 | 868.25603 | 0.0 | 66.3 |
| 2-1-m1E04-1-0424 | C42 H44 N8 O9 S2 | 868.26727 | -3.1 | -14.1 |
| 2-1-m1E04-1-0425 | C47 H44 N6 O7 S2 | 868.27129 | -5.3 | -7.4 |
| 2-1-m1E04-1-0426 | C44 H45 F N6 O8 S2 | 868.27243 | -5.6 | -8.7 |
| 2-1-m1E04-1-0427 | C44 H45 F N6 O8 S2 | 868.27243 | -23.5 | -14.1 |
| 2-1-m1E04-1-0428 | C45 H43 F3 N6 O7 S | 868.28660 | -6.9 | -14.0 |
| 2-1-m1E04-1-0429 | C46 H47 F3 N6 O6 S | 868.32299 | -9.9 | -5.8 |
| 2-1-m1E04-1-0430 | C46 H50 F2 N6 O7 S | 868.34298 | -4.6 | -11.9 |
| 2-1-m1E04-1-0431 | C43 H40 F N5 O8 S3 | 869.20230 | -5.2 | -11.5 |
| 2-1-m1E04-1-0432 | C46 H43 N7 O7 S2 | 869.26654 | -6.3 | -9.5 |
| 2-1-m1E04-1-0433 | C46 H43 N7 O7 S2 | 869.26654 | -12.2 | -16.1 |
| 2-1-m1E04-1-0434 | C43 H44 F N7 O8 S2 | 869.26768 | -2.7 | -9.2 |
| 2-1-m1E04-1-0435 | C43 H44 F N7 O8 S2 | 869.26768 | -9.6 | -13.5 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0436 | C44 H42 F3 N7 O7 S | 869.28185 | -9.6 | -0.8 |
| 2-1-m1E04-1-0437 | C42 H42 N6 O9 S3 | 870.21754 | -0.1 | -3.8 |
| 2-1-m1E04-1-0438 | C43 H46 N6 O8 S3 | 870.25392 | -9.2 | -12.8 |
| 2-1-m1E04-1-0439 | C45 H42 N8 O7 S2 | 870.26179 | -0.3 | 0.0 |
| 2-1-m1E04-1-0440 | C45 H45 F3 N6 O7 S | 870.30225 | 0.0 | 0.0 |
| 2-1-m1E04-1-0441 | C42 H41 N5 O10 S3 | 871.20155 | 4.8 | -10.7 |
| 2-1-m1E04-1-0442 | C41 H41 N7 O9 S3 | 871.21279 | -4.0 | -7.7 |
| 2-1-m1E04-1-0443 | C43 H39 F2 N5 O9 S2 | 871.21573 | 2.8 | 0.0 |
| 2-1-m1E04-1-0444 | C44 H40 F3 N5 O7 S2 | 871.23212 | -10.6 | -10.8 |
| 2-1-m1E04-1-0445 | C42 H45 N7 O8 S3 | 871.24917 | -7.5 | 0.0 |
| 2-1-m1E04-1-0446 | C47 H45 N5 O8 S2 | 871.27095 | 0.0 | 0.0 |
| 2-1-m1E04-1-0447 | C49 H44 F3 N5 O5 S | 871.30152 | -5.4 | -10.5 |
| 2-1-m1E04-1-0448 | C49 H53 N5 O6 S2 | 871.34373 | 0.0 | 0.0 |
| 2-1-m1E04-1-0449 | C49 H53 N5 O6 S2 | 871.34373 | 0.0 | 0.0 |
| 2-1-m1E04-1-0450 | C41 H40 N6 O10 S3 | 872.19680 | -18.4 | -12.8 |
| 2-1-m1E04-1-0451 | C42 H41 F N6 O10 S2 | 872.23096 | 0.0 | -19.8 |
| 2-1-m1E04-1-0452 | C42 H44 N6 O9 S3 | 872.23319 | -2.5 | -7.4 |
| 2-1-m1E04-1-0453 | C43 H42 F2 N6 O8 S2 | 872.24736 | -3.5 | 8.1 |
| 2-1-m1E04-1-0454 | C43 H42 F2 N6 O8 S2 | 872.24736 | 8.4 | 3.2 |
| 2-1-m1E04-1-0455 | C46 H44 N6 O8 S2 | 872.26620 | 0.0 | 0.0 |
| 2-1-m1E04-1-0456 | C45 H44 N8 O7 S2 | 872.27744 | 0.0 | -4.1 |
| 2-1-m1E04-1-0457 | C41 H43 N7 O9 S3 | 873.22844 | 0.0 | 0.0 |
| 2-1-m1E04-1-0458 | C46 H43 N5 O7 S3 | 873.23246 | 2.0 | -5.9 |
| 2-1-m1E04-1-0459 | C46 H43 N5 O7 S3 | 873.23246 | 12.9 | 2.0 |
| 2-1-m1E04-1-0460 | C46 H43 N5 O7 S3 | 873.23246 | 0.0 | 0.0 |
| 2-1-m1E04-1-0461 | C42 H41 F2 N7 O8 S2 | 873.24261 | -0.7 | -5.7 |
| 2-1-m1E04-1-0462 | C47 H47 N5 O8 S2 | 873.28660 | -5.5 | -10.6 |
| 2-1-m1E04-1-0463 | C47 H42 F3 N7 O5 S | 873.29202 | 0.0 | 0.0 |
| 2-1-m1E04-1-0464 | C48 H51 N5 O7 S2 | 873.32299 | -8.2 | 0.0 |
| 2-1-m1E04-1-0465 | C48 H51 N5 O7 S2 | 873.32299 | 0.0 | 0.0 |
| 2-1-m1E04-1-0466 | C45 H42 N6 O7 S3 | 874.22771 | -16.8 | -14.1 |
| 2-1-m1E04-1-0467 | C45 H42 N6 O7 S3 | 874.22771 | 0.0 | 0.0 |
| 2-1-m1E04-1-0468 | C45 H42 N6 O7 S3 | 874.22771 | 0.0 | 0.0 |
| 2-1-m1E04-1-0469 | C41 H40 F2 N8 O8 S2 | 874.23786 | 0.0 | 0.0 |
| 2-1-m1E04-1-0470 | C43 H41 F3 N6 O7 S2 | 874.24302 | -6.0 | -11.5 |
| 2-1-m1E04-1-0471 | C43 H41 F3 N6 O9 S | 874.26078 | -4.0 | -10.5 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0472 | C46 H46 N6 O8 S2 | 874.28185 | 8.7 | 0.0 |
| 2-1-m1E04-1-0473 | C46 H46 N6 O8 S2 | 874.28185 | 3.3 | -17.6 |
| 2-1-m1E04-1-0474 | C43 H40 F3 N5 O8 S2 | 875.22704 | -8.4 | -9.0 |
| 2-1-m1E04-1-0475 | C45 H42 F N7 O7 S2 | 875.25712 | -3.7 | -18.4 |
| 2-1-m1E04-1-0476 | C46 H45 N5 O9 S2 | 875.26587 | -2.1 | -12.2 |
| 2-1-m1E04-1-0477 | C47 H49 N5 O8 S2 | 875.30225 | 4.6 | -2.7 |
| 2-1-m1E04-1-0478 | C47 H49 N5 O8 S2 | 875.30225 | -6.6 | 0.0 |
| 2-1-m1E04-1-0479 | C48 H50 F N5 O6 S2 | 875.31865 | 0.6 | 0.0 |
| 2-1-m1E04-1-0480 | C45 H44 N6 O9 S2 | 876.26112 | -11.9 | -13.8 |
| 2-1-m1E04-1-0481 | C44 H41 F5 N6 O6 S | 876.27284 | -12.4 | -8.5 |
| 2-1-m1E04-1-0482 | C47 H52 N6 O7 S2 | 876.33389 | 5.6 | -4.5 |
| 2-1-m1E04-1-0483 | C47 H52 N6 O7 S2 | 876.33389 | -9.9 | 0.0 |
| 2-1-m1E04-1-0484 | C47 H52 N6 O7 S2 | 876.33389 | 25.5 | 11.0 |
| 2-1-m1E04-1-0485 | C47 H42 F3 N5 O5 S2 | 877.25795 | 0.0 | 0.0 |
| 2-1-m1E04-1-0486 | C46 H44 F N5 O8 S2 | 877.26153 | -8.4 | -11.1 |
| 2-1-m1E04-1-0487 | C44 H46 F3 N5 O7 S2 | 877.27907 | -13.5 | -10.7 |
| 2-1-m1E04-1-0488 | C47 H48 F N5 O7 S2 | 877.29792 | 0.0 | 0.0 |
| 2-1-m1E04-1-0489 | C47 H51 N5 O8 S2 | 877.31791 | 0.0 | -13.2 |
| 2-1-m1E04-1-0490 | C46 H51 N7 O7 S2 | 877.32914 | 0.0 | 0.0 |
| 2-1-m1E04-1-0491 | C41 H40 F2 N6 O8 S3 | 878.20378 | -6.6 | -8.4 |
| 2-1-m1E04-1-0492 | C45 H43 F N6 O8 S2 | 878.25678 | -3.2 | -8.6 |
| 2-1-m1E04-1-0493 | C45 H46 N6 O9 S2 | 878.27677 | -10.2 | 61.1 |
| 2-1-m1E04-1-0494 | C46 H50 N6 O8 S2 | 878.31315 | -4.8 | -8.4 |
| 2-1-m1E04-1-0495 | C46 H50 N6 O8 S2 | 878.31315 | 0.0 | 0.0 |
| 2-1-m1E04-1-0496 | C46 H50 N6 O8 S2 | 878.31315 | -3.4 | 0.0 |
| 2-1-m1E04-1-0497 | C46 H50 N6 O8 S2 | 878.31315 | -39.0 | 0.0 |
| 2-1-m1E04-1-0498 | C40 H39 F2 N7 O8 S3 | 879.19903 | 6.5 | -3.0 |
| 2-1-m1E04-1-0499 | C45 H45 N5 O8 S3 | 879.24303 | 7.2 | -17.4 |
| 2-1-m1E04-1-0500 | C44 H45 N7 O9 S2 | 879.27202 | -5.1 | 2.8 |
| 2-1-m1E04-1-0501 | C44 H45 N7 O9 S2 | 879.27202 | 0.7 | -5.7 |
| 2-1-m1E04-1-0502 | C46 H46 F N5 O8 S2 | 879.27718 | -3.2 | 3.1 |
| 2-1-m1E04-1-0503 | C46 H46 F N5 O8 S2 | 879.27718 | 0.4 | -8.0 |
| 2-1-m1E04-1-0504 | C47 H47 F2 N5 O6 S2 | 879.29358 | 15.6 | -6.2 |
| 2-1-m1E04-1-0505 | C45 H49 N7 O8 S2 | 879.30840 | 0.0 | 0.0 |
| 2-1-m1E04-1-0506 | C50 H49 N5 O6 S2 | 879.31243 | 0.0 | 0.0 |
| 2-1-m1E04-1-0507 | C43 H44 N8 O9 S2 | 880.26727 | 0.0 | 0.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0508 | C46 H43 F3 N6 O7 S | 880.28660 | -20.6 | -17.2 |
| 2-1-m1E04-1-0509 | C45 H48 N6 O9 S2 | 880.29242 | -7.3 | 8.0 |
| 2-1-m1E04-1-0510 | C45 H48 N6 O9 S2 | 880.29242 | 0.1 | -5.3 |
| 2-1-m1E04-1-0511 | C46 H49 F N6 O7 S2 | 880.30882 | -1.5 | 0.0 |
| 2-1-m1E04-1-0512 | C44 H43 N5 O9 S3 | 881.22229 | -4.4 | -8.6 |
| 2-1-m1E04-1-0513 | C44 H43 N5 O9 S3 | 881.22229 | 0.0 | 0.0 |
| 2-1-m1E04-1-0514 | C45 H41 F2 N5 O8 S2 | 881.23646 | -5.3 | -6.6 |
| 2-1-m1E04-1-0515 | C45 H41 F2 N5 O8 S2 | 881.23646 | -2.3 | -9.0 |
| 2-1-m1E04-1-0516 | C46 H45 F2 N5 O7 S2 | 881.27285 | -0.8 | -18.2 |
| 2-1-m1E04-1-0517 | C46 H43 N9 O6 S2 | 881.27777 | 0.0 | -5.6 |
| 2-1-m1E04-1-0518 | C44 H47 N7 O9 S2 | 881.28767 | -6.7 | 1.2 |
| 2-1-m1E04-1-0519 | C44 H47 N7 O9 S2 | 881.28767 | 0.0 | -8.6 |
| 2-1-m1E04-1-0520 | C49 H47 N5 O7 S2 | 881.29169 | -0.3 | -11.8 |
| 2-1-m1E04-1-0521 | C48 H50 F3 N5 O6 8 | 881.34339 | -4.7 | -17.5 |
| 2-1-m1E04-1-0522 | C43 H42 N6 O9 S3 | 882.21754 | -10.7 | -14.3 |
| 2-1-m1E04-1-0523 | C44 H40 F2 N6 O8 S2 | 882.23171 | -2.7 | -2.8 |
| 2-1-m1E04-1-0524 | C44 H43 F N6 O9 S2 | 882.25170 | -1.3 | -6.3 |
| 2-1-m1E04-1-0525 | C44 H43 F N6 O9 S2 | 882.25170 | 0.0 | 0.0 |
| 2-1-m1E04-1-0526 | C44 H46 N6 010 S2 | 882.27168 | -3.7 | -10.7 |
| 2-1-m1E04-1-0527 | C48 H46 N6 O7 S2 | 882.28694 | -3.9 | -8.9 |
| 2-1-m1E04-1-0528 | C45 H47 F N6 O8 S2 | 882.28808 | -2.7 | -9.9 |
| 2-1-m1E04-1-0529 | C45 H47 F N6 O8 S2 | 882.28808 | -21.4 | -15.9 |
| 2-1-m1E04-1-0530 | C42 H41 N7 O9 S3 | 883.21279 | -0.1 | -2.5 |
| 2-1-m1E04-1-0531 | C43 H42 F N7 O9 S2 | 883.24695 | 2.1 | -10.2 |
| 2-1-m1E04-1-0532 | C43 H42 F N7 O9 S2 | 883.24695 | 0.0 | 0.0 |
| 2-1-m1E04-1-0533 | C46 H41 F4 N5 O7 8 | 883.26628 | -9.3 | -9.8 |
| 2-1-m1E04-1-0534 | C47 H45 N7 O7 S2 | 883.28219 | 7.1 | -40.0 |
| 2-1-m1E04-1-0535 | C45 H44 F3 N7 O7 8 | 883.29750 | -5.6 | 3.9 |
| 2-1-m1E04-1-0536 | C43 H44 N6 O9 S3 | 884.23319 | -0.6 | -7.2 |
| 2-1-m1E04-1-0537 | C43 H44 N6 O11 S2 | 884.25095 | 1.6 | -10.9 |
| 2-1-m1E04-1-0538 | C45 H43 F3 N6 O8 S | 884.28152 | -6.5 | -12.9 |
| 2-1-m1E04-1-0539 | C45 H46 F2 N6 O7 S2 | 884.28375 | -5.0 | -12.7 |
| 2-1-m1E04-1-0540 | C43 H43 N5 010 S3 | 885.21720 | 0.0 | 0.0 |
| 2-1-m1E04-1-0541 | C46 H43 N7 O6 S3 | 885.24369 | 0.0 | 0.0 |
| 2-1-m1E04-1-0542 | C45 H42 F3 N5 O7 S2 | 885.24777 | -12.5 | -12.3 |
| 2-1-m1E04-1-0543 | C45 H42 F3 N5 O7 S2 | 885.24777 | -1.7 | -17.9 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0544 | C45 H42 F3 N5 O7 S2 | 885.24777 | -6.2 | -14.1 |
| 2-1-m1E04-1-0545 | C45 H42 F3 N5 O7 S2 | 885.24777 | 0.0 | 0.0 |
| 2-1-m1E04-1-0546 | C47 H43 N5 O9 S2 | 885.25022 | 0.0 | 0.0 |
| 2-1-m1E04-1-0547 | C43 H47 N7 O8 S3 | 885.26482 | -11.1 | -15.7 |
| 2-1-m1E04-1-0548 | C44 H42 F3 N7 O8 8 | 885.27677 | -6.5 | -8.6 |
| 2-1-m1E04-1-0549 | C48 H47 N5 O8 S2 | 885.28660 | 0.0 | 0.0 |
| 2-1-m1E04-1-0550 | C49 H51 N5 O7 S2 | 885.32299 | 3.5 | 0.3 |
| 2-1-m1E04-1-0551 | C50 H55 N5 O6 S2 | 885.35938 | 0.2 | 6.3 |
| 2-1-m1E04-1-0552 | C44 H41 F3 N6 O7 S2 | 886.24302 | -4.7 | -11.7 |
| 2-1-m1E04-1-0553 | C44 H41 F3 N6 O7 S2 | 886.24302 | -12.8 | -9.4 |
| 2-1-m1E04-1-0554 | C44 H44 F2 N6 O8 S2 | 886.26301 | -5.1 | -7.7 |
| 2-1-m1E04-1-0555 | C44 H44 F2 N6 O8 S2 | 886.26301 | 0.0 | 0.0 |
| 2-1-m1E04-1-0556 | C45 H42 F4 N6 O7 8 | 886.27718 | -6.7 | -8.0 |
| 2-1-m1E04-1-0557 | C48 H50 N6 O7 S2 | 886.31824 | 0.0 | 0.0 |
| 2-1-m1E04-1-0558 | C42 H41 N5 O9 S4 | 887.17871 | 8.4 | 0.0 |
| 2-1-m1E04-1-0559 | C43 H39 F2 N5 O8 S3 | 887.19288 | -12.1 | -18.6 |
| 2-1-m1E04-1-0560 | C43 H40 F3 N7 O7 S2 | 887.23827 | -3.7 | -14.8 |
| 2-1-m1E04-1-0561 | C47 H45 N5 O7 S3 | 887.24811 | -8.3 | -11.7 |
| 2-1-m1E04-1-0562 | C47 H45 N5 O7 S3 | 887.24811 | -11.0 | 7.7 |
| 2-1-m1E04-1-0563 | C46 H42 F N7 O7 S2 | 887.25712 | -14.2 | -18.0 |
| 2-1-m1E04-1-0564 | C43 H43 F2 N7 O8 S2 | 887.25826 | 0.0 | 0.0 |
| 2-1-m1E04-1-0565 | C49 H53 N5 O7 S2 | 887.33864 | 0.0 | 0.0 |
| 2-1-m1E04-1-0566 | C41 H40 N6 O9 S4 | 888.17396 | 4.5 | -10.7 |
| 2-1-m1E04-1-0567 | C42 H41 FN6 O9 S3 | 888.20812 | 2.1 | -3.8 |
| 2-1-m1E04-1-0568 | C46 H44 N6 O7 S3 | 888.24336 | -3.8 | -11.6 |
| 2-1-m1E04-1-0569 | C45 H44 N8 O8 S2 | 888.27235 | 0.0 | 14.9 |
| 2-1-m1E04-1-0570 | C47 H48 N6 O8 S2 | 888.29750 | 0.0 | 0.0 |
| 2-1-m1E04-1-0571 | C42 H40 F N5 010S3 | 889.19213 | 0.0 | 4.6 |
| 2-1-m1E04-1-0572 | C45 H43 N7 O7 S3 | 889.23861 | -4.9 | -4.2 |
| 2-1-m1E04-1-0573 | C47 H44 F N5 O8 S2 | 889.26153 | 0.0 | 0.0 |
| 2-1-m1E04-1-0574 | C44 H43 N9 O8 S2 | 889.26760 | 0.0 | 0.0 |
| 2-1-m1E04-1-0575 | C46 H41 F6 N5 05 8 | 889.27326 | -9.4 | -23.2 |
| 2-1-m1E04-1-0576 | C47 H42 F3 N7 O6 8 | 889.28694 | 10.7 | 0.0 |
| 2-1-m1E04-1-0577 | C48 H51 N5 O8 S2 | 889.31791 | 3.3 | -8.4 |
| 2-1-m1E04-1-0578 | C47 H51 N7 O7 S2 | 889.32914 | 0.2 | -9.5 |
| 2-1-m1E04-1-0579 | C41 H42 N6 O11 S3 | 890.20737 | -3.0 | 8.3 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0580 | C42 H40 F2 N6 010 S2 | 890.22154 | 0.1 | -17.5 |
| 2-1-m1E04-1-0581 | C43 H41 F3 N6 O8 S2 | 890.23794 | -3.3 | -12.6 |
| 2-1-m1E04-1-0582 | C46 H46 N6 O9 S2 | 890.27677 | -3.0 | -7.0 |
| 2-1-m1E04-1-0583 | C46 H46 N6 O9 S2 | 890.27677 | -7.6 | -17.2 |
| 2-1-m1E04-1-0584 | C48 H54 N6 O7 S2 | 890.34954 | -5.2 | -14.0 |
| 2-1-m1E04-1-0585 | C48 H54 N6 O7 S2 | 890.34954 | 0.0 | 0.0 |
| 2-1-m1E04-1-0586 | C40 H41 N7 O11 S3 | 891.20262 | -8.8 | -21.7 |
| 2-1-m1E04-1-0587 | C43 H40 F3 N5 O7 S3 | 891.20420 | 0.0 | 0.0 |
| 2-1-m1E04-1-0588 | C43 H40 F3 N5 O9 S2 | 891.22195 | 4.7 | 7.6 |
| 2-1-m1E04-1-0589 | C46 H42 F N5 O7 S3 | 891.22304 | 2.3 | -7.0 |
| 2-1-m1E04-1-0590 | C46 H42 F N5 O7 S3 | 891.22304 | 0.0 | 0.0 |
| 2-1-m1E04-1-0591 | C45 H45 N7 O9 S2 | 891.27202 | -9.8 | -1.6 |
| 2-1-m1E04-1-0592 | C47 H46 F N5 O8 S2 | 891.27718 | -6.2 | -16.9 |
| 2-1-m1E04-1-0593 | C47 H49 N5 O9 S2 | 891.29717 | 0.0 | -18.1 |
| 2-1-m1E04-1-0594 | C48 H53 N5 O8 S2 | 891.33356 | -1.9 | -10.3 |
| 2-1-m1E04-1-0595 | C42 H39 F3 N6 O7 S3 | 892.19944 | -2.5 | -14.5 |
| 2-1-m1E04-1-0596 | C45 H44 N6 O8 S3 | 892.23827 | 1.7 | -16.1 |
| 2-1-m1E04-1-0597 | C45 H44 N6 O8 S3 | 892.23827 | -9.7 | -15.6 |
| 2-1-m1E04-1-0598 | C46 H48 N6 O9 S2 | 892.29242 | -2.0 | -7.3 |
| 2-1-m1E04-1-0599 | C46 H43 F3 N8 O6 8 | 892.29784 | 0.0 | 0.0 |
| 2-1-m1E04-1-0600 | C47 H52 N6 O8 S2 | 892.32880 | -3.7 | -12.4 |
| 2-1-m1E04-1-0601 | C47 H52 N6 O8 S2 | 892.32880 | -50.7 | 0.0 |
| 2-1-m1E04-1-0602 | C44 H43 N7 O8 S3 | 893.23352 | 1.4 | 0.0 |
| 2-1-m1E04-1-0603 | C44 H43 N7 O8 S3 | 893.23352 | -10.8 | -24.4 |
| 2-1-m1E04-1-0604 | C44 H43 N7 O8 S3 | 893.23352 | -7.6 | -6.2 |
| 2-1-m1E04-1-0605 | C44 H43 N7 O8 S3 | 893.23352 | 0.0 | 0.0 |
| 2-1-m1E04-1-0606 | C47 H42 F3 N5 O6 S2 | 893.25286 | 0.0 | 0.0 |
| 2-1-m1E04-1-0607 | C46 H44 F N5 O9 S2 | 893.25645 | 0.0 | -14.4 |
| 2-1-m1E04-1-0608 | C44 H46 F3 N5 O8 S2 | 893.27399 | -4.2 | -12.5 |
| 2-1-m1E04-1-0609 | C45 H47 N7 O9 S2 | 893.28767 | -3.3 | -10.7 |
| 2-1-m1E04-1-0610 | C45 H47 N7 O9 S2 | 893.28767 | 0.1 | -7.2 |
| 2-1-m1E04-1-0611 | C48 H49 F2 N5 O6 S2 | 893.30923 | 0.0 | 0.0 |
| 2-1-m1E04-1-0612 | C51 H51 N5 O6 S2 | 893.32808 | -11.2 | -10.9 |
| 2-1-m1E04-1-0613 | C43 H42 N8 O8 S3 | 894.22877 | 0.0 | 12.7 |
| 2-1-m1E04-1-0614 | C42 H41 F3 N6 O9 S2 | 894.23285 | -20.0 | -13.3 |
| 2-1-m1E04-1-0615 | C45 H46 N6 010 S2 | 894.27168 | 2.9 | -9.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0616 | C46 H50 N6 O9 S2 | 894.30807 | -3.4 | -9.8 |
| 2-1-m1E04-1-0617 | C46 H50 N6 O9 S2 | 894.30807 | 0.0 | 0.0 |
| 2-1-m1E04-1-0618 | C47 H51 FN6 O7 S2 | 894.32447 | -8.0 | -10.2 |
| 2-1-m1E04-1-0619 | C42 H41 N9 O8 S3 | 895.22402 | -3.1 | -2.7 |
| 2-1-m1E04-1-0620 | C45 H45 N5 O9 S3 | 895.23794 | -20.9 | -12.0 |
| 2-1-m1E04-1-0621 | C44 H45 N7 010 S2 | 895.26693 | -2.2 | -5.5 |
| 2-1-m1E04-1-0622 | C49 H45 N5 O8 S2 | 895.27095 | -3.7 | -15.1 |
| 2-1-m1E04-1-0623 | C50 H49 N5 O7 S2 | 895.30734 | 9.0 | 9.4 |
| 2-1-m1E04-1-0624 | C47 H50 F N5 O8 S2 | 895.30848 | 0.0 | 0.0 |
| 2-1-m1E04-1-0625 | C45 H42 F2 N6 O8 S2 | 896.24736 | -1.5 | -4.3 |
| 2-1-m1E04-1-0626 | C46 H43 F3 N6 O6 S2 | 896.26376 | 6.4 | -7.0 |
| 2-1-m1E04-1-0627 | C48 H44 N6 O8 S2 | 896.26620 | -14.4 | 0.0 |
| 2-1-m1E04-1-0628 | C45 H45 F N6 O9 S2 | 896.26735 | -20.4 | -13.6 |
| 2-1-m1E04-1-0629 | C43 H47 F3 N6 O8 S2 | 896.28489 | -5.3 | -12.5 |
| 2-1-m1E04-1-0630 | C46 H49 F N6 O8 S2 | 896.30373 | 0.0 | 0.0 |
| 2-1-m1E04-1-0631 | C46 H52 N6 O9 S2 | 896.32372 | 0.9 | -6.2 |
| 2-1-m1E04-1-0632 | C45 H42 F3 N7 O6 S2 | 897.25901 | 0.0 | 0.0 |
| 2-1-m1E04-1-0633 | C44 H44 F N7 O9 S2 | 897.26260 | -8.6 | -18.9 |
| 2-1-m1E04-1-0634 | C46 H45 F2 N5 O8 S2 | 897.26776 | -1.8 | -6.5 |
| 2-1-m1E04-1-0635 | C47 H46 F3 N5 O6 S2 | 897.28416 | -9.1 | -5.2 |
| 2-1-m1E04-1-0636 | C48 H47 N7 O7 S2 | 897.29784 | 0.0 | 0.0 |
| 2-1-m1E04-1-0637 | C45 H51 N7 O9 S2 | 897.31897 | 0.0 | 0.0 |
| 2-1-m1E04-1-0638 | C48 H50 F3 N5 O7 8 | 897.33830 | -11.3 | -20.4 |
| 2-1-m1E04-1-0639 | C44 H46 N6 O9 S3 | 898.24884 | -1.9 | -10.0 |
| 2-1-m1E04-1-0640 | C45 H47 F N6 O9 S2 | 898.28300 | 0.0 | 0.0 |
| 2-1-m1E04-1-0641 | C45 H47 F N6 O9 S2 | 898.28300 | -1.5 | -33.2 |
| 2-1-m1E04-1-0642 | C46 H48 F2 N6 O7 S2 | 898.29940 | 0.0 | 0.0 |
| 2-1-m1E04-1-0643 | C42 H41 N7 O8 S4 | 899.18994 | -6.0 | -3.6 |
| 2-1-m1E04-1-0644 | C44 H42 F N5 O9 S3 | 899.21287 | -24.7 | -17.2 |
| 2-1-m1E04-1-0645 | C45 H40 F3 N5 O8 S2 | 899.22704 | -5.7 | -10.6 |
| 2-1-m1E04-1-0646 | C44 H45 N5 010 S3 | 899.23285 | -12.4 | -30.2 |
| 2-1-m1E04-1-0647 | C46 H44 F3 N5 O7 S2 | 899.26342 | -14.6 | -12.1 |
| 2-1-m1E04-1-0648 | C46 H44 F3 N5 O7 S2 | 899.26342 | 5.0 | -14.0 |
| 2-1-m1E04-1-0649 | C47 H45 N7 O8 S2 | 899.27710 | 0.0 | 0.0 |
| 2-1-m1E04-1-0650 | C49 H46 F N5 O7 S2 | 899.28227 | -19.8 | 0.0 |
| 2-1-m1E04-1-0651 | C45 H44 F3 N7 O8 8 | 899.29242 | -7.3 | -12.1 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0652 | C49 H49 N5 O8 S2 | 899.30225 | 0.0 | 0.0 |
| 2-1-m1E04-1-0653 | C50 H53 N5 O7 S2 | 899.33864 | -8.0 | -14.3 |
| 2-1-m1E04-1-0654 | C41 H40 N8 O8 S4 | 900.18519 | 5.4 | 0.6 |
| 2-1-m1E04-1-0655 | C43 H44 N6 O10 S3 | 900.22810 | -2.6 | -12.2 |
| 2-1-m1E04-1-0656 | C43 H44 N6 O10 S3 | 900.22810 | 3.9 | -7.3 |
| 2-1-m1E04-1-0657 | C44 H42 F2 N6 O9 S2 | 900.24227 | -3.6 | -12.4 |
| 2-1-m1E04-1-0658 | C44 H42 F2 N6 O9 S2 | 900.24227 | -0.5 | -7.5 |
| 2-1-m1E04-1-0659 | C45 H43 F3 N6 O7 S2 | 900.25867 | -8.8 | 0.0 |
| 2-1-m1E04-1-0660 | C45 H46 F2 N6 O8 S2 | 900.27866 | -2.9 | 0.0 |
| 2-1-m1E04-1-0661 | C47 H51 F3 N6 O7 S | 900.34920 | 3.3 | -16.7 |
| 2-1-m1E04-1-0662 | C43 H43 N5 O9 S4 | 901.19436 | 0.0 | 0.0 |
| 2-1-m1E04-1-0663 | C43 H43 N5 Oil S3 | 901.21212 | 0.0 | 0.0 |
| 2-1-m1E04-1-0664 | C42 H43 N7 O10 S3 | 901.22335 | -3.3 | -2.4 |
| 2-1-m1E04-1-0665 | C47 H43 N5 O8 S3 | 901.22738 | -11.1 | -16.2 |
| 2-1-m1E04-1-0666 | C43 H41 F2 N7 O9 S2 | 901.23752 | -2.0 | -14.6 |
| 2-1-m1E04-1-0667 | C45 H42 F3 N5 O8 S2 | 901.24269 | 5.4 | -6.9 |
| 2-1-m1E04-1-0668 | C45 H42 F3 N5 O8 S2 | 901.24269 | -4.1 | -6.9 |
| 2-1-m1E04-1-0669 | C48 H47 N5 O7 S3 | 901.26376 | 0.0 | -11.7 |
| 2-1-m1E04-1-0670 | C48 H47 N5 O7 S3 | 901.26376 | 0.0 | 0.0 |
| 2-1-m1E04-1-0671 | C48 H47 N5 O9 S2 | 901.28152 | 0.0 | 0.0 |
| 2-1-m1E04-1-0672 | C44 H41 F3 N6 O8 S2 | 902.23794 | 0.2 | -7.1 |
| 2-1-m1E04-1-0673 | C47 H46 N6 O7 S3 | 902.25901 | -3.1 | -20.4 |
| 2-1-m1E04-1-0674 | C45 H42 F4 N6 O8 S | 902.27210 | -4.5 | -12.2 |
| 2-1-m1E04-1-0675 | C46 H46 N8 O8 S2 | 902.28800 | -7.4 | -9.0 |
| 2-1-m1E04-1-0676 | C44 H40 F3 N5 O9 S2 | 903.22195 | -6.6 | -13.5 |
| 2-1-m1E04-1-0677 | C43 H40 F3 N7 O8 S2 | 903.23319 | -9.1 | -19.4 |
| 2-1-m1E04-1-0678 | C45 H41 F4 N5 O7 S2 | 903.23835 | -7.3 | -15.3 |
| 2-1-m1E04-1-0679 | C47 H45 N5 O8 S3 | 903.24303 | 3.8 | -15.7 |
| 2-1-m1E04-1-0680 | C47 H45 N5 O8 S3 | 903.24303 | -4.0 | -14.0 |
| 2-1-m1E04-1-0681 | C49 H50 F N5 O7 S2 | 903.31357 | -0.9 | -10.7 |
| 2-1-m1E04-1-0682 | C42 H44 N6 O11 S3 | 904.22302 | -5.2 | -8.4 |
| 2-1-m1E04-1-0683 | C44 H43 F3 N6 O8 S2 | 904.25359 | -10.1 | -12.9 |
| 2-1-m1E04-1-0684 | C44 H43 F3 N6 O8 S2 | 904.25359 | -4.8 | -12.0 |
| 2-1-m1E04-1-0685 | C44 H43 F3 N6 O8 S2 | 904.25359 | -2.7 | -13.6 |
| 2-1-m1E04-1-0686 | C44 H43 F3 N6 O8 S2 | 904.25359 | 0.0 | 0.0 |
| 2-1-m1E04-1-0687 | C47 H48 N6 O9 S2 | 904.29242 | -11.2 | -11.6 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0688 | C48 H52 N6 O8 S2 | 904.32880 | -11.4 | 5.0 |
| 2-1-m1E04-1-0689 | C49 H56 N6 O7 S2 | 904.36519 | 0.0 | 0.0 |
| 2-1-m1E04-1-0690 | C42 H40 F N5 O9 S4 | 905.16929 | -5.0 | -14.6 |
| 2-1-m1E04-1-0691 | C46 H41 F2 N7 O7 S2 | 905.24769 | -27.7 | 5.9 |
| 2-1-m1E04-1-0692 | C43 H42 F3 N7 O8 S2 | 905.24884 | 0.0 | 0.0 |
| 2-1-m1E04-1-0693 | C43 H42 F3 N7 O8 S2 | 905.24884 | 0.0 | 0.0 |
| 2-1-m1E04-1-0694 | C46 H41 F6 N5 O6 S | 905.26817 | -9.4 | -10.7 |
| 2-1-m1E04-1-0695 | C46 H47 N7 O7 S3 | 905.26991 | -14.7 | 0.0 |
| 2-1-m1E04-1-0696 | C47 H51 N7 O8 S2 | 905.32405 | 0.0 | 0.0 |
| 2-1-m1E04-1-0697 | C49 H55 N5 O8 S2 | 905.34921 | 0.0 | 0.0 |
| 2-1-m1E04-1-0698 | C41 H42 N6 O10 S4 | 906.18452 | -5.0 | -9.0 |
| 2-1-m1E04-1-0699 | C42 H40 F2 N6 O9 S3 | 906.19870 | -8.1 | 13.7 |
| 2-1-m1E04-1-0700 | C46 H43 F N6 O7 S3 | 906.23394 | -6.6 | -5.1 |
| 2-1-m1E04-1-0701 | C42 H41 F3 N8 O8 S2 | 906.24409 | 0.0 | 0.0 |
| 2-1-m1E04-1-0702 | C46 H46 N6 O8 S3 | 906.25392 | -18.7 | -11.2 |
| 2-1-m1E04-1-0703 | C46 H46 N6 O8 S3 | 906.25392 | 2.4 | -4.0 |
| 2-1-m1E04-1-0704 | C45 H43 F N8 O8 S2 | 906.26293 | 0.0 | -3.6 |
| 2-1-m1E04-1-0705 | C48 H54 N6 O8 S2 | 906.34445 | 0.0 | 0.0 |
| 2-1-m1E04-1-0706 | C40 H41 N7 O10 S4 | 907.17977 | 0.0 | -7.9 |
| 2-1-m1E04-1-0707 | C42 H39 F2 N5 O10 S3 | 907.18271 | -15.8 | -12.3 |
| 2-1-m1E04-1-0708 | C43 H40 F3 N5 O8 S3 | 907.19911 | -5.8 | -10.1 |
| 2-1-m1E04-1-0709 | C45 H45 N7 O8 S3 | 907.24917 | -2.0 | -10.8 |
| 2-1-m1E04-1-0710 | C45 H45 N7 O8 S3 | 907.24917 | -1.0 | 13.9 |
| 2-1-m1E04-1-0711 | C47 H43 F2 N5 O8 S2 | 907.25211 | -8.4 | -10.5 |
| 2-1-m1E04-1-0712 | C46 H49 N7 O9 S2 | 907.30332 | -1.8 | 4.8 |
| 2-1-m1E04-1-0713 | C48 H53 N5 O9 S2 | 907.32847 | 0.0 | 0.0 |
| 2-1-m1E04-1-0714 | C52 H53 N5 O6 S2 | 907.34373 | -15.5 | -14.0 |
| 2-1-m1E04-1-0715 | C42 H39 F3 N6 O8 S3 | 908.19436 | -15.2 | -13.0 |
| 2-1-m1E04-1-0716 | C41 H41 F N6 O11 S3 | 908.19795 | -3.4 | -12.5 |
| 2-1-m1E04-1-0717 | C44 H44 N8 O8 S3 | 908.24442 | 0.0 | 13.5 |
| 2-1-m1E04-1-0718 | C44 H44 N8 O8 S3 | 908.24442 | -2.8 | -15.0 |
| 2-1-m1E04-1-0719 | C46 H45 F N6 O9 S2 | 908.26735 | -6.6 | -10.5 |
| 2-1-m1E04-1-0720 | C45 H42 F6 N6 O6 S | 908.27907 | -4.7 | -10.0 |
| 2-1-m1E04-1-0721 | C46 H43 F3 N8 O7 S | 908.29275 | -8.4 | -9.1 |
| 2-1-m1E04-1-0722 | C47 H52 N6 O9 S2 | 908.32372 | -10.5 | -17.8 |
| 2-1-m1E04-1-0723 | C46 H41 F2 N5 O7 S3 | 909.21362 | -4.5 | 0.5 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0724 | C46 H41 F2 N5 O7 S3 | 909.21362 | 0.0 | 0.0 |
| 2-1-m1E04-1-0725 | C46 H47 N5 O9 S3 | 909.25359 | 0.0 | 0.0 |
| 2-1-m1E04-1-0726 | C45 H47 N7 O10 S2 | 909.28258 | -7.4 | -13.3 |
| 2-1-m1E04-1-0727 | C42 H41 F3 N6 O8 S3 | 910.21001 | -14.4 | -14.2 |
| 2-1-m1E04-1-0728 | C42 H41 F3 N6 O10 S2 | 910.22777 | -2.6 | -9.9 |
| 2-1-m1E04-1-0729 | C45 H43 F N6 O8 S3 | 910.22885 | -7.9 | -11.1 |
| 2-1-m1E04-1-0730 | C45 H43 F N6 O8 S3 | 910.22885 | -2.6 | -9.9 |
| 2-1-m1E04-1-0731 | C49 H46 N6 O8 S2 | 910.28185 | -2.8 | -7.8 |
| 2-1-m1E04-1-0732 | C46 H47 F N6 O9 S2 | 910.28300 | -3.4 | -10.7 |
| 2-1-m1E04-1-0733 | C46 H50 N6 O10 S2 | 910.30298 | 5.6 | -3.3 |
| 2-1-m1E04-1-0734 | C47 H54 N6 O9 S2 | 910.33937 | -0.3 | -10.4 |
| 2-1-m1E04-1-0735 | C41 H40 F3 N7 O8 S3 | 911.20526 | -4.7 | 0.0 |
| 2-1-m1E04-1-0736 | C43 H41 N7 O10 S3 | 911.20770 | -4.7 | -11.1 |
| 2-1-m1E04-1-0737 | C45 H45 N5 O10 S3 | 911.23285 | -0.8 | -13.6 |
| 2-1-m1E04-1-0738 | C48 H48 F3 N5 O6 S2 | 911.29981 | -7.8 | -31.1 |
| 2-1-m1E04-1-0739 | C50 H49 N5 O8 S2 | 911.30225 | -26.9 | -9.6 |
| 2-1-m1E04-1-0740 | C46 H43 F3 N6 O7 S2 | 912.25867 | 0.0 | 0.0 |
| 2-1-m1E04-1-0741 | C45 H45 F N6 O10 S2 | 912.26226 | 3.4 | 0.0 |
| 2-1-m1E04-1-0742 | C43 H47 F3 N6 O9 S2 | 912.27980 | -3.4 | -13.2 |
| 2-1-m1E04-1-0743 | C47 H50 F2 N6 O7 S2 | 912.31505 | -23.7 | 0.0 |
| 2-1-m1E04-1-0744 | C46 H42 F3 N5 O8 S2 | 913.24269 | -7.8 | -10.8 |
| 2-1-m1E04-1-0745 | C49 H44 F N5 O8 S2 | 913.26153 | 0.1 | -21.9 |
| 2-1-m1E04-1-0746 | C47 H46 F3 N5 O7 S2 | 913.27907 | -10.4 | -10.7 |
| 2-1-m1E04-1-0747 | C47 H46 F3 N5 O7 S2 | 913.27907 | -83.9 | -3.5 |
| 2-1-m1E04-1-0748 | C47 H46 F3 N5 O7 S2 | 913.27907 | -1.2 | -2.2 |
| 2-1-m1E04-1-0749 | C47 H49 F2 N5 O8 S2 | 913.29906 | -5.3 | -10.5 |
| 2-1-m1E04-1-0750 | C51 H55 N5 O7 S2 | 913.35429 | -11.0 | -4.5 |
| 2-1-m1E04-1-0751 | C45 H41 F3 N6 O8 S2 | 914.23794 | -13.7 | -10.7 |
| 2-1-m1E04-1-0752 | C44 H46 N6 O10 S3 | 914.24375 | -4.6 | -9.8 |
| 2-1-m1E04-1-0753 | C46 H51 F N6 O9 S2 | 914.31430 | -23.7 | -77.8 |
| 2-1-m1E04-1-0754 | C44 H45 N5 O9 S4 | 915.21001 | 15.1 | 0.0 |
| 2-1-m1E04-1-0755 | C44 H43 F2 N7 O9 S2 | 915.25317 | -8.3 | -11.8 |
| 2-1-m1E04-1-0756 | C46 H44 F3 N5 O8 S2 | 915.25834 | -3.3 | -8.9 |
| 2-1-m1E04-1-0757 | C46 H44 F3 N5 O8 S2 | 915.25834 | -3.9 | -11.5 |
| 2-1-m1E04-1-0758 | C50 H53 N5 O8 S2 | 915.33356 | 0.0 | 0.0 |
| 2-1-m1E04-1-0759 | C45 H43 F3 N6 O8 S2 | 916.25359 | 0.0 | 0.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0760 | C45 H46 F2 N6 O9 S2 | 916.27357 | -14.4 | -8.1 |
| 2-1-m1E04-1-0761 | C48 H48 N6 O7 S3 | 916.27466 | -9.4 | -8.1 |
| 2-1-m1E04-1-0762 | C46 H47 F3 N6 O7 S2 | 916.28997 | -292.9 | -117.0 |
| 2-1-m1E04-1-0763 | C47 H48 N8 O8 S2 | 916.30365 | 0.0 | -8.2 |
| 2-1-m1E04-1-0764 | C47 H51 F3 N6 O8 S | 916.34412 | -4.2 | -11.2 |
| 2-1-m1E04-1-0765 | C43 H43 N5 O10 S4 | 917.18928 | 0.0 | 0.0 |
| 2-1-m1E04-1-0766 | C44 H41 F2 N5 O9 S3 | 917.20345 | -6.6 | -20.3 |
| 2-1-m1E04-1-0767 | C46 H43 F4 N5 O7 S2 | 917.25400 | -16.5 | -12.1 |
| 2-1-m1E04-1-0768 | C43 H43 F N6 O10 S3 | 918.21868 | -4.6 | -13.7 |
| 2-1-m1E04-1-0769 | C44 H41 F3 N6 O9 S2 | 918.23285 | -4.6 | 1.0 |
| 2-1-m1E04-1-0770 | C43 H46 N6 O11 S3 | 918.23867 | 0.0 | -4.4 |
| 2-1-m1E04-1-0771 | C47 H46 N6 O8 S3 | 918.25392 | -2.8 | -14.4 |
| 2-1-m1E04-1-0772 | C45 H45 F3 N6 O8 S2 | 918.26924 | -5.3 | -11.0 |
| 2-1-m1E04-1-0773 | C45 H45 F3 N6 O8 S2 | 918.26924 | -1.6 | -11.0 |
| 2-1-m1E04-1-0774 | C46 H46 N8 O9 S2 | 918.28292 | 0.0 | 0.0 |
| 2-1-m1E04-1-0775 | C48 H50 N6 O9 S2 | 918.30807 | -4.9 | -14.0 |
| 2-1-m1E04-1-0776 | C49 H54 N6 O8 S2 | 918.34445 | -5.8 | -10.7 |
| 2-1-m1E04-1-0777 | C44 H40 F3 N5 O8 S3 | 919.19911 | -8.4 | -12.5 |
| 2-1-m1E04-1-0778 | C44 H40 F3 N5 O10 S2 | 919.21687 | -9.1 | -12.2 |
| 2-1-m1E04-1-0779 | C44 H44 F3 N7 O8 S2 | 919.26449 | 16.9 | -25.6 |
| 2-1-m1E04-1-0780 | C50 H45 N7 O7 S2 | 919.28219 | -5.9 | -14.9 |
| 2-1-m1E04-1-0781 | C47 H49 N7 O7 S3 | 919.28556 | 14.3 | -42.6 |
| 2-1-m1E04-1-0782 | C42 H44 N6 O10 S4 | 920.20017 | -4.6 | -9.6 |
| 2-1-m1E04-1-0783 | C42 H44 N6 O12 S3 | 920.21793 | -6.2 | -0.4 |
| 2-1-m1E04-1-0784 | C44 H43 F3 N6 O9 S2 | 920.24850 | -7.3 | -12.9 |
| 2-1-m1E04-1-0785 | C44 H43 F3 N6 O9 S2 | 920.24850 | -4.3 | -14.4 |
| 2-1-m1E04-1-0786 | C47 H48 N6 O8 S3 | 920.26957 | -14.2 | -11.7 |
| 2-1-m1E04-1-0787 | C47 H48 N6 O8 S3 | 920.26957 | 0.0 | 0.0 |
| 2-1-m1E04-1-0788 | C47 H48 N6 O10 S2 | 920.28733 | -12.5 | -2.9 |
| 2-1-m1E04-1-0789 | C46 H43 N5 O10 S3 | 921.21720 | -1.9 | 0.0 |
| 2-1-m1E04-1-0790 | C45 H43 N7 O9 S3 | 921.22844 | -4.6 | -6.3 |
| 2-1-m1E04-1-0791 | C45 H40 F5 N5 O7 S2 | 921.22893 | 10.3 | -2.6 |
| 2-1-m1E04-1-0792 | C43 H42 F3 N7 O9 S2 | 921.24375 | -4.3 | 0.0 |
| 2-1-m1E04-1-0793 | C46 H47 N7 O8 S3 | 921.26482 | -6.3 | -10.6 |
| 2-1-m1E04-1-0794 | C46 H47 N7 O8 S3 | 921.26482 | 0.0 | -7.8 |
| 2-1-m1E04-1-0795 | C51 H47 N5 O8 S2 | 921.28660 | 0.0 | -14.7 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0796 | C49 H49 F2 N5 O7 S2 | 921.30415 | -17.9 | -10.2 |
| 2-1-m1E04-1-0797 | C44 H42 N8 O9 S3 | 922.22369 | -5.7 | 3.3 |
| 2-1-m1E04-1-0798 | C43 H41 F3 N6 O10 S2 | 922.22777 | -3.5 | -11.5 |
| 2-1-m1E04-1-0799 | C42 H41 F3 N8 O9 S2 | 922.23900 | 0.0 | 0.0 |
| 2-1-m1E04-1-0800 | C44 H42 F4 N6 O8 S2 | 922.24417 | -8.9 | -12.7 |
| 2-1-m1E04-1-0801 | C46 H46 N6 O9 S3 | 922.24884 | -2.6 | -16.3 |
| 2-1-m1E04-1-0802 | C46 H46 N6 O9 S3 | 922.24884 | -17.0 | 1.9 |
| 2-1-m1E04-1-0803 | C48 H51 F N6 O8 S2 | 922.31938 | -5.3 | -10.4 |
| 2-1-m1E04-1-0804 | C42 H39 F2 N5 O9 S4 | 923.15987 | -5.1 | -8.8 |
| 2-1-m1E04-1-0805 | C50 H45 N5 O7 S3 | 923.24811 | -8.7 | -15.2 |
| 2-1-m1E04-1-0806 | C50 H45 N5 O7 S3 | 923.24811 | -10.5 | -18.0 |
| 2-1-m1E04-1-0807 | C41 H41 F N6 O10 S4 | 924.17510 | -5.8 | -9.0 |
| 2-1-m1E04-1-0808 | C46 H42 F2 N6 O7 S3 | 924.22452 | -2.9 | -9.8 |
| 2-1-m1E04-1-0809 | C45 H42 F2 N8 O8 S2 | 924.25351 | 0.0 | 0.0 |
| 2-1-m1E04-1-0810 | C45 H42 F6 N6 O7 S | 924.27399 | -4.7 | -12.5 |
| 2-1-m1E04-1-0811 | C48 H56 N6 O9 S2 | 924.35502 | -3.0 | -9.0 |
| 2-1-m1E04-1-0812 | C45 H44 F N7 O8 S3 | 925.23975 | 0.7 | -5.3 |
| 2-1-m1E04-1-0813 | C45 H44 F N7 O8 S3 | 925.23975 | 0.0 | -7.3 |
| 2-1-m1E04-1-0814 | C49 H50 F3 N5 O6 S2 | 925.31546 | -11.3 | -10.8 |
| 2-1-m1E04-1-0815 | C41 H40 F2 N6 O11 S3 | 926.18852 | -7.0 | -7.1 |
| 2-1-m1E04-1-0816 | C42 H41 F3 N6 O9 S3 | 926.20492 | -7.7 | -14.6 |
| 2-1-m1E04-1-0817 | C46 H44 F2 N6 O9 S2 | 926.25792 | 3.8 | -4.6 |
| 2-1-m1E04-1-0818 | C47 H54 N6 O10 S2 | 926.33428 | 0.0 | 13.2 |
| 2-1-m1E04-1-0819 | C43 H41 N7 O9 S4 | 927.18486 | 29.1 | 29.4 |
| 2-1-m1E04-1-0820 | C41 H40 F3 N7 O9 S3 | 927.20017 | 6.0 | -12.1 |
| 2-1-m1E04-1-0821 | C48 H48 F3 N5 O7 S2 | 927.29472 | 0.0 | 0.0 |
| 2-1-m1E04-1-0822 | C51 H53 N5 O8 S2 | 927.33356 | 0.0 | 0.0 |
| 2-1-m1E04-1-0823 | C45 H42 F2 N6 O8 S3 | 928.21943 | -12.8 | -11.1 |
| 2-1-m1E04-1-0824 | C45 H42 F2 N6 O8 S3 | 928.21943 | 0.0 | -43.4 |
| 2-1-m1E04-1-0825 | C46 H43 F3 N6 O8 S2 | 928.25359 | -2.9 | -15.8 |
| 2-1-m1E04-1-0826 | C45 H48 N6 O10 S3 | 928.25940 | -5.2 | -9.0 |
| 2-1-m1E04-1-0827 | C46 H42 F3 N5 O9 S2 | 929.23760 | 1.8 | -18.4 |
| 2-1-m1E04-1-0828 | C47 H46 F3 N5 O8 S2 | 929.27399 | 0.0 | -10.1 |
| 2-1-m1E04-1-0829 | C47 H46 F3 N5 O8 S2 | 929.27399 | 0.0 | 0.0 |
| 2-1-m1E04-1-0830 | C45 H41 F3 N6 O9 S2 | 930.23285 | -2.8 | -9.4 |
| 2-1-m1E04-1-0831 | C44 H46 N6 O11 S3 | 930.23867 | -3.1 | -11.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0832 | C47 H49 F3 N6 O7 S2 | 930.30562 | 7.7 | -19.1 |
| 2-1-m1E04-1-0833 | C46 H41 F4 N5 O8 S2 | 931.23327 | -5.0 | -12.1 |
| 2-1-m1E04-1-0834 | C48 H45 N5 O9 S3 | 931.23794 | 4.6 | -13.6 |
| 2-1-m1E04-1-0835 | C45 H43 F3 N6 O9 S2 | 932.24850 | -4.2 | -10.8 |
| 2-1-m1E04-1-0836 | C46 H47 F3 N6 O8 S2 | 932.28489 | -13.9 | -16.0 |
| 2-1-m1E04-1-0837 | C46 H47 F3 N6 O8 S2 | 932.28489 | -1.7 | -0.5 |
| 2-1-m1E04-1-0838 | C46 H47 F3 N6 O8 S2 | 932.28489 | -12.9 | -10.9 |
| 2-1-m1E04-1-0839 | C46 H50 F2 N6 O9 S2 | 932.30488 | -5.0 | -4.9 |
| 2-1-m1E04-1-0840 | C50 H56 N6 O8 S2 | 932.36010 | -6.9 | -8.9 |
| 2-1-m1E04-1-0841 | C44 H42 F3 N7 O9 S2 | 933.24375 | -0.5 | -11.3 |
| 2-1-m1E04-1-0842 | C46 H43 F4 N5 O8 S2 | 933.24892 | -9.4 | -19.3 |
| 2-1-m1E04-1-0843 | C48 H51 N7 O7 S3 | 933.30121 | 5.3 | -11.7 |
| 2-1-m1E04-1-0844 | C43 H46 N6 O10 S4 | 934.21582 | -3.8 | -11.2 |
| 2-1-m1E04-1-0845 | C45 H45 F3 N6 O9 S2 | 934.26415 | -4.7 | -13.0 |
| 2-1-m1E04-1-0846 | C45 H45 F3 N6 O9 S2 | 934.26415 | -6.8 | -13.2 |
| 2-1-m1E04-1-0847 | C49 H54 N6 O9 S2 | 934.33937 | 0.0 | -4.6 |
| 2-1-m1E04-1-0848 | C44 H40 F3 N5 O9 S3 | 935.19402 | -6.0 | -13.0 |
| 2-1-m1E04-1-0849 | C47 H45 N5 O10 S3 | 935.23285 | 0.0 | -8.9 |
| 2-1-m1E04-1-0850 | C44 H44 F3 N7 O9 S2 | 935.25940 | 0.0 | -23.2 |
| 2-1-m1E04-1-0851 | C49 H44 F3 N5 O7 S2 | 935.26342 | -5.3 | -9.4 |
| 2-1-m1E04-1-0852 | C47 H49 N7 O8 S3 | 935.28047 | -4.4 | -3.3 |
| 2-1-m1E04-1-0853 | C53 H53 N5 O7 S2 | 935.33864 | -9.1 | -10.8 |
| 2-1-m1E04-1-0854 | C42 H44 N6 O11 S4 | 936.19509 | 7.7 | -16.4 |
| 2-1-m1E04-1-0855 | C43 H42 F2 N6 O10 S3 | 936.20926 | -2.5 | -16.1 |
| 2-1-m1E04-1-0856 | C46 H44 N6 O10 S3 | 936.22810 | 0.5 | 8.6 |
| 2-1-m1E04-1-0857 | C45 H44 N8 O9 S3 | 936.23934 | 0.0 | -2.6 |
| 2-1-m1E04-1-0858 | C45 H44 F4 N6 O8 S2 | 936.25982 | -6.3 | -18.5 |
| 2-1-m1E04-1-0859 | C46 H43 N5 O9 S4 | 937.19436 | -4.0 | 0.0 |
| 2-1-m1E04-1-0860 | C46 H43 N5 O9 S4 | 937.19436 | 3.7 | -17.4 |
| 2-1-m1E04-1-0861 | C47 H42 F3 N7 O7 S2 | 937.25392 | 17.1 | -12.7 |
| 2-1-m1E04-1-0862 | C46 H47 N7 O9 S3 | 937.25974 | 0.4 | -2.9 |
| 2-1-m1E04-1-0863 | C46 H47 N7 O9 S3 | 937.25974 | -4.4 | -4.3 |
| 2-1-m1E04-1-0864 | C45 H42 N6 O9 S4 | 938.18961 | -8.8 | -15.2 |
| 2-1-m1E04-1-0865 | C43 H41 F3 N6 O9 S3 | 938.20492 | -4.5 | -13.0 |
| 2-1-m1E04-1-0866 | C43 H41 F3 N6 O11 S2 | 938.22268 | -2.7 | -13.9 |
| 2-1-m1E04-1-0867 | C50 H46 N6 O7 S3 | 938.25901 | -14.1 | -12.7 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0868 | C43 H40 F3 N5 O10 S3 | 939.18894 | -2.9 | -11.8 |
| 2-1-m1E04-1-0869 | C45 H42 F N7 O9 S3 | 939.21902 | -0.6 | -11.5 |
| 2-1-m1E04-1-0870 | C48 H44 F3 N5 O8 S2 | 939.25834 | 6.5 | -16.6 |
| 2-1-m1E04-1-0871 | C44 H41 F5 N6 O8 S2 | 940.23474 | -3.5 | -16.3 |
| 2-1-m1E04-1-0872 | C48 H50 F2 N6 O8 S2 | 940.30996 | 0.0 | 0.0 |
| 2-1-m1E04-1-0873 | C47 H42 F3 N5 O7 S3 | 941.21985 | -17.8 | -11.1 |
| 2-1-m1E04-1-0874 | C47 H42 F3 N5 O7 S3 | 941.21985 | 2.2 | -18.6 |
| 2-1-m1E04-1-0875 | C49 H50 F3 N5 O7 S2 | 941.31037 | -5.3 | 0.0 |
| 2-1-m1E04-1-0876 | C41 H40 F2 N6 O10 S4 | 942.16568 | -0.7 | -9.6 |
| 2-1-m1E04-1-0877 | C45 H43 F2 N7 O8 S3 | 943.23033 | -8.2 | -9.9 |
| 2-1-m1E04-1-0878 | C46 H43 F3 N6 O9 S2 | 944.24850 | -12.8 | -13.9 |
| 2-1-m1E04-1-0879 | C48 H51 F3 N6 O7 S2 | 944.32127 | 0.0 | 0.0 |
| 2-1-m1E04-1-0880 | C46 H43 N9 O8 S3 | 945.23967 | 22.8 | -25.7 |
| 2-1-m1E04-1-0881 | C47 H46 F3 N5 O9 S2 | 945.26890 | 0.0 | 0.0 |
| 2-1-m1E04-1-0882 | C48 H50 F3 N5 O8 S2 | 945.30529 | -4.4 | -14.7 |
| 2-1-m1E04-1-0883 | C47 H49 F3 N6 O8 S2 | 946.30054 | 0.0 | 0.0 |
| 2-1-m1E04-1-0884 | C42 H41 N7 O11 S4 | 947.17469 | -5.4 | -12.4 |
| 2-1-m1E04-1-0885 | C46 H41 F4 N5 O9 S2 | 947.22818 | -6.6 | -14.9 |
| 2-1-m1E04-1-0886 | C47 H45 N7 O9 S3 | 947.24409 | -6.7 | -12.5 |
| 2-1-m1E04-1-0887 | C45 H44 F3 N7 O9 S2 | 947.25940 | -0.6 | -10.1 |
| 2-1-m1E04-1-0888 | C45 H43 F3 N6 O10 S2 | 948.24342 | -1.8 | -11.6 |
| 2-1-m1E04-1-0889 | C46 H47 F3 N6 O9 S2 | 948.27980 | 0.0 | 0.0 |
| 2-1-m1E04-1-0890 | C46 H47 F3 N6 O9 S2 | 948.27980 | -4.3 | -11.3 |
| 2-1-m1E04-1-0891 | C46 H43 N7 O8 S4 | 949.20559 | -15.8 | -13.8 |
| 2-1-m1E04-1-0892 | C46 H43 N7 O8 S4 | 949.20559 | 0.0 | 0.0 |
| 2-1-m1E04-1-0893 | C45 H42 F3 N5 O9 S3 | 949.20967 | -0.8 | -11.5 |
| 2-1-m1E04-1-0894 | C44 H42 F3 N7 O10 S2 | 949.23867 | -1.2 | -10.3 |
| 2-1-m1E04-1-0895 | C48 H47 N5 O10 S3 | 949.24850 | -3.9 | -12.1 |
| 2-1-m1E04-1-0896 | C45 H42 F4 N6 O9 S2 | 950.23908 | -2.7 | -8.5 |
| 2-1-m1E04-1-0897 | C47 H45 N5 O9 S4 | 951.21001 | -21.3 | -13.7 |
| 2-1-m1E04-1-0898 | C46 H44 N6 O9 S4 | 952.20526 | -2.4 | -18.1 |
| 2-1-m1E04-1-0899 | C45 H44 F4 N6 O9 S2 | 952.25473 | 0.0 | -17.2 |
| 2-1-m1E04-1-0900 | C45 H43 N7 O9 S4 | 953.20051 | -7.0 | 23.9 |
| 2-1-m1E04-1-0901 | C47 H44 F N5 O10 S3 | 953.22343 | -7.6 | -3.6 |
| 2-1-m1E04-1-0902 | C46 H41 F6 N5 O7 S2 | 953.23516 | -5.5 | -11.8 |
| 2-1-m1E04-1-0903 | C47 H42 F3 N7 O8 S2 | 953.24884 | 0.0 | 0.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0904 | C47 H51 N7 O9 S3 | 953.29104 | -5.0 | -6.5 |
| 2-1-m1E04-1-0905 | C50 H50 F3 N5 O7 S2 | 953.31037 | -9.2 | -8.2 |
| 2-1-m1E04-1-0906 | C43 H41 F3 N6 O10 S3 | 954.19984 | -5.4 | -13.1 |
| 2-1-m1E04-1-0907 | C46 H46 N6 O11 S3 | 954.23867 | 31.6 | 23.1 |
| 2-1-m1E04-1-0908 | C43 H40 F3 N5 O9 S4 | 955.16610 | -5.6 | -15.2 |
| 2-1-m1E04-1-0909 | C43 H40 F3 N5 O11 S3 | 955.18385 | -13.6 | -7.5 |
| 2-1-m1E04-1-0910 | C46 H42 F N5 O9 S4 | 955.18494 | 10.5 | -7.9 |
| 2-1-m1E04-1-0911 | C45 H45 N7 O11 S3 | 955.23392 | 28.5 | 9.7 |
| 2-1-m1E04-1-0912 | C48 H44 F3 N5 O9 S2 | 955.25325 | -8.8 | -11.7 |
| 2-1-m1E04-1-0913 | C45 H44 N6 O10 S4 | 956.20017 | -0.2 | -4.0 |
| 2-1-m1E04-1-0914 | C47 H43 F3 N6 O7 S3 | 956.23074 | -12.9 | -11.4 |
| 2-1-m1E04-1-0915 | C46 H43 F3 N8 O8 S2 | 956.25974 | 1.2 | -3.3 |
| 2-1-m1E04-1-0916 | C44 H43 N7 O10 S4 | 957.19542 | -4.9 | -18.9 |
| 2-1-m1E04-1-0917 | C44 H43 N7 O10 S4 | 957.19542 | 40.9 | 12.0 |
| 2-1-m1E04-1-0918 | C47 H42 F3 N5 O8 S3 | 957.21476 | -3.8 | -8.0 |
| 2-1-m1E04-1-0919 | C47 H42 F3 N5 O8 S3 | 957.21476 | 3.2 | -4.0 |
| 2-1-m1E04-1-0920 | C42 H41 F3 N6 O11 S3 | 958.19475 | -3.9 | -18.3 |
| 2-1-m1E04-1-0921 | C47 H45 F3 N6 O9 S2 | 958.26415 | -5.2 | -10.6 |
| 2-1-m1E04-1-0922 | C46 H43 F3 N6 O8 S3 | 960.22566 | -6.2 | -10.7 |
| 2-1-m1E04-1-0923 | C46 H43 F3 N6 O8 S3 | 960.22566 | 0.0 | 0.0 |
| 2-1-m1E04-1-0924 | C48 H51 F3 N6 O8 S2 | 960.31619 | -14.4 | 0.0 |
| 2-1-m1E04-1-0925 | C45 H42 F3 N7 O8 S3 | 961.22091 | 0.0 | 5.4 |
| 2-1-m1E04-1-0926 | C49 H51 N7 O8 S3 | 961.29612 | -1.5 | 0.0 |
| 2-1-m1E04-1-0927 | C48 H50 F3 N5 O9 S2 | 961.30020 | -3.7 | -12.8 |
| 2-1-m1E04-1-0928 | C42 H41 N7 O10 S5 | 963.15184 | -3.6 | 8.9 |
| 2-1-m1E04-1-0929 | C45 H44 F3 N7 O10 S2 | 963.25432 | 0.0 | -11.0 |
| 2-1-m1E04-1-0930 | C49 H49 N5 O10 S3 | 963.26415 | 0.0 | 0.0 |
| 2-1-m1E04-1-0931 | C46 H44 N8 O8 S4 | 964.21649 | -15.0 | -16.3 |
| 2-1-m1E04-1-0932 | C46 H47 F3 N6 O10 S2 | 964.27472 | 0.0 | -14.7 |
| 2-1-m1E04-1-0933 | C47 H51 F3 N6 O9 S2 | 964.31110 | -6.8 | -13.9 |
| 2-1-m1E04-1-0934 | C45 H42 F3 N5 O10 S3 | 965.20459 | 8.1 | -6.2 |
| 2-1-m1E04-1-0935 | C48 H47 N5 O9 S4 | 965.22566 | -32.4 | -40.9 |
| 2-1-m1E04-1-0936 | C48 H47 N5 Oil S3 | 965.24342 | 4.9 | -5.1 |
| 2-1-m1E04-1-0937 | C47 H46 N6 O9 S4 | 966.22091 | -9.2 | -16.8 |
| 2-1-m1E04-1-0938 | C45 H42 F4 N6 O10 S2 | 966.23400 | -2.9 | -7.2 |
| 2-1-m1E04-1-0939 | C47 H45 N5 O10 S4 | 967.20493 | 0.0 | 0.0 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0940 | C44 H43 F3 N6 O10 S3 | 968.21549 | 4.5 | -12.1 |
| 2-1-m1E04-1-0941 | C47 H48 N6 O11 S3 | 968.25432 | 7.5 | -2.0 |
| 2-1-m1E04-1-0942 | C46 H41 F6 N5 O8 S2 | 969.23007 | -12.2 | -10.4 |
| 2-1-m1E04-1-0943 | C50 H50 F3 N5 O8 S2 | 969.30529 | -11.2 | -6.9 |
| 2-1-m1E04-1-0944 | C46 H43 F N6 O9 S4 | 970.19584 | -18.0 | -14.1 |
| 2-1-m1E04-1-0945 | C46 H46 N6 O10 S4 | 970.21582 | -1.1 | -6.0 |
| 2-1-m1E04-1-0946 | C43 H40 F3 N5 O10 S4 | 971.16101 | 1.6 | -15.2 |
| 2-1-m1E04-1-0947 | C45 H45 N7 O10 S4 | 971.21107 | -0.8 | -11.9 |
| 2-1-m1E04-1-0948 | C47 H43 F2 N5 O10 S3 | 971.21401 | -34.7 | 3.4 |
| 2-1-m1E04-1-0949 | C44 H44 N8 O10 S4 | 972.20632 | 0.0 | -4.0 |
| 2-1-m1E04-1-0950 | C47 H43 F3 N6 O8 S3 | 972.22566 | -11.1 | -12.0 |
| 2-1-m1E04-1-0951 | C46 H45 F N6 O11 S3 | 972.22925 | 12.9 | -2.1 |
| 2-1-m1E04-1-0952 | C45 H42 F6 N6 O8 S2 | 972.24097 | -6.2 | -15.5 |
| 2-1-m1E04-1-0953 | C46 H43 F3 N8 O9 S2 | 972.25465 | 12.5 | -2.5 |
| 2-1-m1E04-1-0954 | C49 H51 F3 N6 O8 S2 | 972.31619 | -10.2 | -17.2 |
| 2-1-m1E04-1-0955 | C46 H41 F2 N5 O9 S4 | 973.17552 | -3.3 | -9.8 |
| 2-1-m1E04-1-0956 | C42 H41 F3 N6 O10 S4 | 974.17191 | -4.4 | -6.9 |
| 2-1-m1E04-1-0957 | C42 H41 F3 N6 012 S3 | 974.18967 | -5.2 | -11.6 |
| 2-1-m1E04-1-0958 | C45 H43 F N6 O10 S4 | 974.19075 | 0.5 | -7.7 |
| 2-1-m1E04-1-0959 | C47 H45 F3 N6 O10 S2 | 974.25907 | -4.7 | -13.0 |
| 2-1-m1E04-1-0960 | C46 H44 F3 N7 O8 S3 | 975.23656 | -15.6 | -14.9 |
| 2-1-m1E04-1-0961 | C46 H43 F3 N6 O9 S3 | 976.22057 | -6.5 | -10.8 |
| 2-1-m1E04-1-0962 | C46 H43 F3 N6 O9 S3 | 976.22057 | 0.0 | 0.0 |
| 2-1-m1E04-1-0963 | C48 H48 N6 O9 S4 | 980.23656 | -12.9 | -12.1 |
| 2-1-m1E04-1-0964 | C47 H51 F3 N6 O10 S2 | 980.30602 | -1.0 | -7.5 |
| 2-1-m1E04-1-0965 | C47 H46 N6 O10 S4 | 982.21582 | 0.0 | 0.0 |
| 2-1-m1E04-1-0966 | C48 H50 N6 O11 S3 | 982.26997 | -7.0 | -8.4 |
| 2-1-m1E04-1-0967 | C44 H43 F3 N6 O11 S3 | 984.21040 | -7.2 | -19.6 |
| 2-1-m1E04-1-0968 | C47 H48 N6 O10 S4 | 984.23147 | -0.8 | -12.2 |
| 2-1-m1E04-1-0969 | C47 H48 N6 012 S3 | 984.24923 | 0.0 | -13.0 |
| 2-1-m1E04-1-0970 | C46 H47 N7 O10 S4 | 985.22672 | -3.4 | -17.6 |
| 2-1-m1E04-1-0971 | C51 H47 N5 O10 S3 | 985.24850 | -13.9 | -19.5 |
| 2-1-m1E04-1-0972 | C46 H46 N6 O11 S4 | 986.21074 | 8.6 | 0.0 |
| 2-1-m1E04-1-0973 | C50 H45 N5 O9 S4 | 987.21001 | 0.0 | -16.5 |
| 2-1-m1E04-1-0974 | C46 H42 F2 N6 O9 S4 | 988.18642 | -4.5 | -10.4 |
| 2-1-m1E04-1-0975 | C45 H42 F6 N6 O9 S2 | 988.23589 | -9.6 | -2.1 |

(continued)

| [Table 11-2-4] | | | | |
|---|---|---|---|---|
| | | | AS-MS ratio (%) | |
| ID | Molecular Formula | Exact Mass | N1 | N2 |
| 2-1-m1E04-1-0976 | C49 H51 F3 N6 O9 S2 | 988.31110 | -3.6 | -10.6 |
| 2-1-m1E04-1-0977 | C45 H44 F N7 O10 S4 | 989.20165 | -5.7 | -11.2 |
| 2-1-m1E04-1-0978 | C42 H41 F3 N6 O11 S4 | 990.16682 | -5.8 | -10.6 |
| 2-1-m1E04-1-0979 | C46 H44 F2 N6 O11 S3 | 990.21982 | 17.5 | -8.2 |
| 2-1-m1E04-1-0980 | C46 H44 F3 N7 O9 S3 | 991.23147 | -5.4 | -11.0 |
| 2-1-m1E04-1-0981 | C45 H42 F2 N6 O10 S4 | 992.18133 | -5.7 | -16.6 |
| 2-1-m1E04-1-0982 | C47 H49 N7 O10 S4 | 999.24237 | -5.9 | -13.1 |
| 2-1-m1E04-1-0983 | C46 H47 N7 O11 S4 | 1001.22164 | 0.0 | 0.0 |
| 2-1-m1E04-1-0984 | C50 H46 N6 O9 S4 | 1002.22091 | -2.9 | -17.0 |
| 2-1-m1E04-1-0985 | C48 H44 F3 N5 O10 S3 | 1003.22024 | 4.7 | -5.4 |
| 2-1-m1E04-1-0986 | C47 H42 F3 N5 O9 S4 | 1005.18175 | -3.9 | -19.8 |
| 2-1-m1E04-1-0987 | C45 H43 F2 N7 O10 S4 | 1007.19223 | 7.1 | -12.9 |
| 2-1-m1E04-1-0988 | C47 H45 N7 O11 S4 | 1011.20599 | 15.9 | -2.9 |
| 2-1-m1E04-1-0989 | C46 H43 N7 O10 S5 | 1013.16749 | -14.4 | 0.2 |
| 2-1-m1E04-1-0990 | C48 H44 F3 N5 O11 S3 | 1019.21515 | -0.4 | -21.6 |
| 2-1-m1E04-1-0991 | C47 H43 F3 N6 O9 S4 | 1020.19264 | -5.7 | -12.1 |
| 2-1-m1E04-1-0992 | C47 H42 F3 N5 O10 S4 | 1021.17666 | -9.7 | -8.5 |
| 2-1-m1E04-1-0993 | C47 H45 F3 N6 O11 S3 | 1022.22605 | -6.1 | -11.0 |
| 2-1-m1E04-1-0994 | C46 H43 F3 N6 O10 S4 | 1024.18756 | -4.4 | -9.6 |
| 2-1-m1E04-1-0995 | C46 H44 N8 O10 S5 | 1028.17839 | -15.3 | -9.8 |
| 2-1-m1E04-1-0996 | C47 H43 F3 N6 O10 S4 | 1036.18756 | -2.8 | -9.8 |
| 2-1-m1E04-1-0997 | C47 H45 F3 N6 012 S3 | 1038.22097 | -12.7 | -19.1 |
| 2-1-m1E04-1-0998 | C46 H44 F3 N7 O10 S4 | 1039.19846 | -9.3 | -14.0 |
| 2-1-m1E04-1-0999 | C46 H43 F3 N6 O11 S4 | 1040.18247 | -11.9 | -12.1 |
| 2-1-m1E04-1-1000 | C46 H44 F3 N7 O11 S4 | 1055.19337 | -9.1 | -13.9 |

[Table 594]

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0001 | C38 H38 N4 O4 | 614.28931 | 0.0 | -9.0 |
| 2-1-m1E01-2-0002 | C37 H37 N5 O4 | 615.28455 | -52.9 | -21.3 |
| 2-1-m1E01-2-0003 | C37 H37 N5 O4 | 615.28455 | -6.5 | 18.9 |
| 2-1-m1E01-2-0004 | C36 H36 N6 O4 | 616.27980 | 0.0 | 0.0 |
| 2-1-m1E01-2-0005 | C36 H36 N6 O4 | 616.27980 | -33.1 | -0.7 |
| 2-1-m1E01-2-0006 | C35 H35 N7 O4 | 617.27505 | -53.1 | -19.7 |
| 2-1-m1E01-2-0007 | C35 H35 N5 O4 S | 621.24098 | 14.3 | -3.8 |
| 2-1-m1E01-2-0008 | C34 H34 N6 O4 S | 622.23622 | -19.2 | -24.2 |
| 2-1-m1E01-2-0009 | C39 H40 N4 O4 | 628.30496 | 0.0 | -9.0 |
| 2-1-m1E01-2-0010 | C38 H39 N5 O4 | 629.30020 | -140.5 | 0.0 |
| 2-1-m1E01-2-0011 | C37 H38 N6 O4 | 630.29545 | -25.3 | -20.9 |
| 2-1-m1E01-2-0012 | C37 H36 N4 O6 | 632.26348 | -29.4 | -27.1 |
| 2-1-m1E01-2-0013 | C38 H37 F N4 O4 | 632.27988 | -134.2 | 19.9 |
| 2-1-m1E01-2-0014 | C36 H35 N5 O6 | 633.25873 | -3.7 | 3.9 |
| 2-1-m1E01-2-0015 | C37 H36 F N5 O4 | 633.27513 | -2.5 | 0.0 |
| 2-1-m1E01-2-0016 | C37 H39 N5 O5 | 633.29512 | -14.4 | 3.9 |
| 2-1-m1E01-2-0017 | C36 H35 F N6 O4 | 634.27038 | -12.3 | 0.0 |
| 2-1-m1E01-2-0018 | C36 H38 N6 O5 | 634.29037 | -20.7 | 0.3 |
| 2-1-m1E01-2-0019 | C36 H38 N6 O5 | 634.29037 | 7.0 | 25.2 |
| 2-1-m1E01-2-0020 | C36 H37 N5 O4 S | 635.25663 | -7.7 | 12.9 |
| 2-1-m1E01-2-0021 | C35 H37 N7 O5 | 635.28562 | -11.5 | -1.5 |
| 2-1-m1E01-2-0022 | C35 H37 N7 O5 | 635.28562 | -11.5 | -1.5 |
| 2-1-m1E01-2-0023 | C34 H36 N8 O5 | 636.28087 | -23.6 | -12.6 |
| 2-1-m1E01-2-0024 | C35 H34 F N5 O4 S | 639.23155 | 3.3 | -44.1 |
| 2-1-m1E01-2-0025 | C34 H36 N6 O5 S | 640.24679 | 4.2 | 9.2 |
| 2-1-m1E01-2-0026 | C33 H35 N7 O5 S | 641.24204 | -22.2 | -18.5 |
| 2-1-m1E01-2-0027 | C39 H38 N4 O5 | 642.28422 | -61.7 | -8.0 |
| 2-1-m1E01-2-0028 | C40 H42 N4 O4 | 642.32061 | -87.4 | 13.3 |
| 2-1-m1E01-2-0029 | C38 H37 N5 O5 | 643.27947 | -12.5 | 1.4 |
| 2-1-m1E01-2-0030 | C38 H37 N5 O5 | 643.27947 | -14.2 | -7.6 |
| 2-1-m1E01-2-0031 | C39 H41 N5 O4 | 643.31585 | -26.1 | -19.3 |
| 2-1-m1E01-2-0032 | C37 H36 N6 O5 | 644.27472 | -19.3 | -6.9 |
| 2-1-m1E01-2-0033 | C39 H40 N4 O5 | 644.29987 | -9.9 | -8.4 |
| 2-1-m1E01-2-0034 | C38 H40 N6 O4 | 644.31110 | 0.0 | 0.0 |
| 2-1-m1E01-2-0035 | C38 H39 N5 O5 | 645.29512 | -20.5 | 2.3 |
| 2-1-m1E01-2-0036 | C38 H38 N4 O6 | 646.27913 | -20.2 | -17.6 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0037 | C37 H38 N6 O5 | 646.29037 | -49.1 | 0.0 |
| 2-1-m1E01-2-0038 | C38 H41 N5 O5 | 647.31077 | 5.4 | 7.1 |
| 2-1-m1E01-2-0039 | C37 H36 N4 O5 S | 648.24064 | 0.0 | 0.0 |
| 2-1-m1E01-2-0040 | C37 H40 N6 O5 | 648.30602 | 5.2 | -1.6 |
| 2-1-m1E01-2-0041 | C36 H35 N5 O5 S | 649.23589 | -17.5 | -7.1 |
| 2-1-m1E01-2-0042 | C37 H39 N5 O4 S | 649.27228 | -41.9 | -63.1 |
| 2-1-m1E01-2-0043 | C36 H39 N7 O5 | 649.30127 | -35.3 | -7.2 |
| 2-1-m1E01-2-0044 | C37 H35 F N4 O6 | 650.25406 | 0.0 | 0.0 |
| 2-1-m1E01-2-0045 | C38 H36 F2 N4 O4 | 650.27046 | 33.0 | 18.0 |
| 2-1-m1E01-2-0046 | C36 H37 N5 O5 S | 651.25154 | -10.8 | 5.6 |
| 2-1-m1E01-2-0047 | C37 H35 F2 N5 O4 | 651.26571 | -3.7 | 3.4 |
| 2-1-m1E01-2-0048 | C36 H37 N5 O7 | 651.26930 | -0.7 | -6.2 |
| 2-1-m1E01-2-0049 | C37 H38 F N5 O5 | 651.28570 | -20.2 | 3.8 |
| 2-1-m1E01-2-0050 | C36 H34 F2 N6 O4 | 652.26096 | -22.1 | 0.0 |
| 2-1-m1E01-2-0051 | C35 H36 N6 O7 | 652.26455 | -15.2 | -5.7 |
| 2-1-m1E01-2-0052 | C36 H37 F N6 O5 | 652.28095 | -14.0 | 8.2 |
| 2-1-m1E01-2-0053 | C35 H36 F N7 O5 | 653.27620 | -42.1 | -2.0 |
| 2-1-m1E01-2-0054 | C35 H38 N6 O5 S | 654.26244 | -26.0 | -50.3 |
| 2-1-m1E01-2-0055 | C40 H40 N4 O5 | 656.29987 | -51.4 | -23.8 |
| 2-1-m1E01-2-0056 | C35 H33 F2 N5 O4 S | 657.22213 | -7.8 | 103.0 |
| 2-1-m1E01-2-0057 | C39 H39 N5 O5 | 657.29512 | -25.7 | -7.1 |
| 2-1-m1E01-2-0058 | C39 H39 N5 O5 | 657.29512 | -20.5 | -21.6 |
| 2-1-m1E01-2-0059 | C34 H35 F N6 O5 S | 658.23737 | 10.1 | 16.7 |
| 2-1-m1E01-2-0060 | C38 H38 N6 O5 | 658.29037 | -10.8 | -0.7 |
| 2-1-m1E01-2-0061 | C39 H37 F N4 O5 | 660.27480 | -19.5 | 18.5 |
| 2-1-m1E01-2-0062 | C39 H37 F N4 O5 | 660.27480 | 0.0 | 0.0 |
| 2-1-m1E01-2-0063 | C39 H40 N4 O6 | 660.29479 | -11.3 | -4.5 |
| 2-1-m1E01-2-0064 | C38 H36 F N5 O5 | 661.27005 | -18.7 | -7.3 |
| 2-1-m1E01-2-0065 | C38 H36 F N5 O5 | 661.27005 | -18.5 | 1.2 |
| 2-1-m1E01-2-0066 | C38 H39 N5 O6 | 661.29003 | -9.4 | -9.8 |
| 2-1-m1E01-2-0067 | C39 H43 N5 O5 | 661.32642 | -17.6 | -19.2 |
| 2-1-m1E01-2-0068 | C38 H38 N4 O5 S | 662.25629 | -12.8 | 17.5 |
| 2-1-m1E01-2-0069 | C38 H38 N4 O7 | 662.27405 | -25.5 | 5.7 |
| 2-1-m1E01-2-0070 | C37 H38 N6 O6 | 662.28528 | -15.4 | -4.1 |
| 2-1-m1E01-2-0071 | C37 H38 N6 O6 | 662.28528 | 7.7 | 8.8 |
| 2-1-m1E01-2-0072 | C38 H42 N6 O5 | 662.32167 | -22.0 | 1.2 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0073 | C36 H37 N7 O6 | 663.28053 | -15.4 | -3.5 |
| 2-1-m1E01-2-0074 | C38 H41 N5 O6 | 663.30568 | -29.1 | -11.9 |
| 2-1-m1E01-2-0075 | C37 H41 N7 O5 | 663.31692 | -25.3 | 8.9 |
| 2-1-m1E01-2-0076 | C37 H40 N6 O6 | 664.30093 | -15.2 | 4.4 |
| 2-1-m1E01-2-0077 | C42 H40 N4 O4 | 664.30496 | -53.2 | -16.8 |
| 2-1-m1E01-2-0078 | C37 H39 N5 O7 | 665.28495 | -27.0 | 2.7 |
| 2-1-m1E01-2-0079 | C36 H39 N7 O6 | 665.29618 | 0.0 | 6.3 |
| 2-1-m1E01-2-0080 | C41 H39 N5 04 | 665.30020 | 85.0 | 7.6 |
| 2-1-m1E01-2-0081 | C37 H35 F N4 O5 S | 666.23122 | 0.0 | 0.0 |
| 2-1-m1E01-2-0082 | C40 H38 N6 04 | 666.29545 | -32.5 | 16.8 |
| 2-1-m1E01-2-0083 | C40 H38 N6 04 | 666.29545 | 0.0 | 0.0 |
| 2-1-m1E01-2-0084 | C36 H37 N5 O6 S | 667.24645 | -10.5 | -6.0 |
| 2-1-m1E01-2-0085 | C39 H37 N7 O4 | 667.29070 | -29.7 | 2.3 |
| 2-1-m1E01-2-0086 | C36 H36 N4 O7 S | 668.23047 | 0.0 | 0.0 |
| 2-1-mlE01-2-0087 | C35 H36 N6 O6 S | 668.24170 | -16.2 | -3.5 |
| 2-1-m1E01-2-0088 | C37 H34 F2 N4 O6 | 668.24464 | -16.2 | -4.9 |
| 2-1-m1E01-2-0089 | C36 H40 N6 O5 S | 668.27809 | 0.0 | 0.0 |
| 2-1-m1E01-2-0090 | C35 H35 N5 O7 S | 669.22572 | -49.2 | -51.5 |
| 2-1-m1E01-2-0091 | C36 H36 F N5 O7 | 669.25988 | -5.6 | -7.8 |
| 2-1-m1E01-2-0092 | C37 H37 F2 N5 O5 | 669.27628 | 0.0 | 0.0 |
| 2-1-m1E01-2-0093 | C35 H38 N6 O6 S | 670.25735 | 0.0 | 0.0 |
| 2-1-m1E01-2-0094 | C40 H38 N4 O4 S | 670.26138 | -11.7 | 1.2 |
| 2-1-m1E01-2-0095 | C36 H36 F2 N6 O5 | 670.27152 | -23.2 | -18.9 |
| 2-1-m1E01-2-0096 | C37 H42 N4 O6 S | 670.28251 | 0.0 | 0.0 |
| 2-1-m1E01-2-0097 | C41 H42 N4 O5 | 670.31552 | -155.6 | -156.2 |
| 2-1-m1E01-2-0098 | C39 H37 N5 04 S | 671.25663 | -13.1 | -11.3 |
| 2-1-m1E01-2-0099 | C39 H37 N5 04 S | 671.25663 | -47.6 | -72.8 |
| 2-1-m1E01-2-0100 | C35 H35 F2 N7 O5 | 671.26677 | 188.3 | 29.0 |
| 2-1-m1E01-2-0101 | C36 H41 N5 O6 S | 671.27775 | -21.6 | -1.9 |
| 2-1-mlE01-2-0102 | C40 H41 N5 O5 | 671.31077 | -18.0 | -0.2 |
| 2-1-m1E01-2-0103 | C40 H41 N5 O5 | 671.31077 | 0.0 | 0.0 |
| 2-1-m1E01-2-0104 | C40 H40 N4 O6 | 672.29479 | -19.2 | 9.7 |
| 2-1-m1E01-2-0105 | C39 H39 N5 O6 | 673.29003 | 9.9 | -6.6 |
| 2-1-m1E01-2-0106 | C40 H39 F N4 O5 | 674.29045 | -29.4 | -10.0 |
| 2-1-m1E01-2-0107 | C41 H46 N4 O5 | 674.34682 | -38.4 | -41.9 |
| 2-1-m1E01-2-0108 | C39 H38 F N5 O5 | 675.28570 | -17.8 | -3.6 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0109 | C39 H41 N5 O6 | 675.30568 | -8.5 | 12.6 |
| 2-1-m1E01-2-0110 | C40 H45 N5 O5 | 675.34207 | -20.2 | -3.0 |
| 2-1-m1E01-2-0111 | C34 H34 F2 N6 O5 S | 676.22795 | -52.0 | 12.6 |
| 2-1-m1E01-2-0112 | C39 H40 N4 O5 S | 676.27194 | 0.0 | 0.0 |
| 2-1-m1E01-2-0113 | C38 H40 N6 O6 | 676.30093 | -19.8 | -10.8 |
| 2-1-m1E01-2-0114 | C38 H40 N6 O6 | 676.30093 | -24.6 | -2.2 |
| 2-1-m1E01-2-0115 | C37 H39 N7 O6 | 677.29618 | -15.0 | -0.2 |
| 2-1-m1E01-2-0116 | C38 H38 N4 O6 S | 678.25121 | 1.7 | 2.4 |
| 2-1-m1E01-2-0117 | C38 H38 N4 O6 S | 678.25121 | 0.0 | 0.0 |
| 2-1-m1E01-2-0118 | C38 H38 N4 O6 S | 678.25121 | -4.1 | -2.5 |
| 2-1-m1E01-2-0119 | C39 H36 F2 N4 O5 | 678.26538 | 0.0 | 0.0 |
| 2-1-m1E01-2-0120 | C39 H36 F2 N4 O5 | 678.26538 | -14.6 | -37.0 |
| 2-1-m1E01-2-0121 | C37 H37 N5 O6 S | 679.24645 | -21.6 | 12.2 |
| 2-1-m1E01-2-0122 | C37 H37 N5 O6 S | 679.24645 | -8.6 | 8.3 |
| 2-1-m1E01-2-0123 | C37 H37 N5 O6 S | 679.24645 | -16.3 | -2.7 |
| 2-1-m1E01-2-0124 | C38 H35 F2 N5 O5 | 679.26063 | -27.1 | -12.8 |
| 2-1-m1E01-2-0125 | C38 H38 F N5 O6 | 679.28061 | -6.9 | -8.2 |
| 2-1-m1E01-2-0126 | C38 H38 F N5 O6 | 679.28061 | 0.0 | -28.2 |
| 2-1-m1E01-2-0127 | C38 H41 N5 O7 | 679.30060 | 0.0 | 0.0 |
| 2-1-m1E01-2-0128 | C36 H36 N6 O6 S | 680.24170 | -17.8 | -6.8 |
| 2-1-m1E01-2-0129 | C36 H36 N6 O6 S | 680.24170 | -19.5 | -5.9 |
| 2-1-m1E01-2-0130 | C37 H37 F N6 O6 | 680.27586 | 16.5 | 2.7 |
| 2-1-m1E01-2-0131 | C37 H37 F N6 O6 | 680.27586 | -125.8 | 0.0 |
| 2-1-m1E01-2-0132 | C41 H40 N6 O4 | 680.31110 | -15.7 | 4.0 |
| 2-1-m1E01-2-0133 | C35 H35 N7 O6 S | 681.23695 | -10.3 | -5.1 |
| 2-1-m1E01-2-0134 | C37 H39 N5 O6 S | 681.26210 | 0.1 | 8.2 |
| 2-1-m1E01-2-0135 | C37 H39 N5 O8 | 681.27986 | 11.5 | -21.8 |
| 2-1-m1E01-2-0136 | C37 H38 N4 O7 S | 682.24612 | 0.0 | 33.8 |
| 2-1-m1E01-2-0137 | C39 H37 F3 N4 04 | 682.27669 | 75.8 | -5.0 |
| 2-1-m1E01-2-0138 | C39 H37 F3 N4 O4 | 682.27669 | 8.5 | 0.0 |
| 2-1-m1E01-2-0139 | C41 H38 N4 O6 | 682.27913 | -128.3 | -37.4 |
| 2-1-m1E01-2-0140 | C38 H36 F3 N5 O4 | 683.27194 | 0.0 | -53.7 |
| 2-1-m1E01-2-0141 | C38 H36 F3 N5 O4 | 683.27194 | -14.7 | -2.4 |
| 2-1-m1E01-2-0142 | C36 H36 N4 O6 S2 | 684.20763 | 0.0 | 0.0 |
| 2-1-m1E01-2-0143 | C37 H34 F2 N4 O5 S | 684.22180 | 0.0 | 0.0 |
| 2-1-m1E01-2-0144 | C37 H35 F3 N6 O4 | 684.26719 | -21.9 | 7.5 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0145 | C41 H40 N4 O4 S | 684.27703 | -7.1 | -93.6 |
| 2-1-m1E01-2-0146 | C40 H37 F N6 04 | 684.28603 | 0.0 | 0.0 |
| 2-1-m1E01-2-0147 | C38 H44 N4 O6 S | 684.29816 | 0.0 | 0.0 |
| 2-1-m1E01-2-0148 | C35 H35 N5 O6 S2 | 685.20288 | -15.7 | 7.1 |
| 2-1-m1E01-2-0149 | C35 H35 N5 O6 S2 | 685.20288 | -24.7 | -17.6 |
| 2-1-m1E01-2-0150 | C36 H36 F N5 O6 S | 685.23703 | -14.5 | -2.3 |
| 2-1-m1E01-2-0151 | C39 H39 N7 O5 | 685.30127 | -17.6 | -3.9 |
| 2-1-m1E01-2-0152 | C41 H43 N5 O5 | 685.32642 | 5.1 | -5.3 |
| 2-1-m1E01-2-0153 | C34 H34 N6 O6 S2 | 686.19812 | -15.8 | 16.6 |
| 2-1-m1E01-2-0154 | C36 H35 F N4 O7 S | 686.22105 | 0.0 | 0.0 |
| 2-1-m1E01-2-0155 | C38 H38 N8 O5 | 686.29652 | -16.6 | 23.4 |
| 2-1-m1E01-2-0156 | C35 H37 N5 O8 S | 687.23628 | -14.8 | 13.3 |
| 2-1-m1E01-2-0157 | C36 H35 F2 N5 O7 | 687.25045 | -25.6 | 12.2 |
| 2-1-m1E01-2-0158 | C40 H41 N5 O6 | 687.30568 | -18.9 | -13.6 |
| 2-1-m1E01-2-0159 | C34 H36 N6 O8 S | 688.23153 | -6.4 | 0.8 |
| 2-1-m1E01-2-0160 | C40 H37 F N4 O4 S | 688.25195 | -37.0 | -6.0 |
| 2-1-m1E01-2-0161 | C37 H41 F N4 O6 S | 688.27308 | 0.0 | 0.0 |
| 2-1-m1E01-2-0162 | C41 H41 FN4 O5 | 688.30610 | 5.3 | -118.7 |
| 2-1-m1E01-2-0163 | C42 H48 N4 O5 | 688.36247 | -158.3 | -167.1 |
| 2-1-m1E01-2-0164 | C36 H34 F3 N5 04 S | 689.22836 | 5.6 | -17.2 |
| 2-1-m1E01-2-0165 | C39 H39 N5 O5 S | 689.26719 | 0.0 | 0.0 |
| 2-1-m1E01-2-0166 | C36 H43 N5 O7 S | 689.28832 | -17.3 | -0.9 |
| 2-1-m1E01-2-0167 | C40 H43 N5 O6 | 689.32133 | -21.2 | -13.6 |
| 2-1-m1E01-2-0168 | C38 H38 N6 O5 S | 690.26244 | -17.7 | -0.2 |
| 2-1-m1E01-2-0169 | C38 H38 N6 O5 S | 690.26244 | 0.0 | 0.0 |
| 2-1-m1E01-2-0170 | C35 H42 N6 O7 S | 690.28357 | -16.3 | -16.2 |
| 2-1-m1E01-2-0171 | C40 H39 F N4 O6 | 690.28536 | -341.4 | 0.0 |
| 2-1-m1E01-2-0172 | C40 H42 N4 O5 S | 690.28759 | -17.8 | -14.8 |
| 2-1-m1E01-2-0173 | C39 H42 N6 O6 | 690.31658 | -23.3 | -1.0 |
| 2-1-m1E01-2-0174 | C39 H42 N6 O6 | 690.31658 | -25.9 | -22.7 |
| 2-1-m1E01-2-0175 | C37 H37 N7 O5 S | 691.25769 | 6.0 | 0.0 |
| 2-1-m1E01-2-0176 | C39 H41 N5 O5 S | 691.28284 | 0.0 | 0.0 |
| 2-1-m1E01-2-0177 | C39 H41 N5 O7 | 691.30060 | -3.6 | -6.7 |
| 2-1-m1E01-2-0178 | C36 H36 N8 O5 S | 692.25294 | 2.4 | -9.2 |
| 2-1-m1E01-2-0179 | C39 H40 N4 O6 S | 692.26686 | 0.0 | 0.0 |
| 2-1-m1E01-2-0180 | C39 H40 N4 O6 S | 692.26686 | 0.0 | 0.0 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0181 | C39 H40 N4 O6 S | 692.26686 | 0.0 | 0.0 |
| 2-1-m1E01-2-0182 | C38 H40 N6 O7 | 692.29585 | 0.0 | -3.1 |
| 2-1-m1E01-2-0183 | C43 H40 N4 O5 | 692.29987 | 16.4 | 1.1 |
| 2-1-m1E01-2-0184 | C41 H45 F N4 O5 | 692.33740 | -13.3 | -6.6 |
| 2-1-m1E01-2-0185 | C38 H39 N5 O6 S | 693.26210 | 9.1 | 3.9 |
| 2-1-m1E01-2-0186 | C38 H39 N5 O6 S | 693.26210 | 30.2 | 43.4 |
| 2-1-m1E01-2-0187 | C38 H39 N5 O6 S | 693.26210 | -4.9 | -1.9 |
| 2-1-m1E01-2-0188 | C39 H37 F2 N5 O5 | 693.27628 | -21.0 | -5.4 |
| 2-1-m1E01-2-0189 | C42 H39 N5 O5 | 693.29512 | -11.2 | -2.9 |
| 2-1-m1E01-2-0190 | C39 H40 F N5 O6 | 693.29626 | 5.8 | 4.9 |
| 2-1-m1E01-2-0191 | C40 H47 N5 O6 | 693.35263 | -1.8 | 0.0 |
| 2-1-m1E01-2-0192 | C37 H38 N6 O6 S | 694.25735 | -27.4 | -19.8 |
| 2-1-m1E01-2-0193 | C37 H38 N6 O6 S | 694.25735 | 22.2 | -16.8 |
| 2-1-m1E01-2-0194 | C38 H39 F N6 O6 | 694.29151 | -21.3 | -6.0 |
| 2-1-m1E01-2-0195 | C42 H42 N6 O4 | 694.32675 | 0.0 | 0.0 |
| 2-1-m1E01-2-0196 | C39 H46 N6 O6 | 694.34788 | -16.9 | -3.7 |
| 2-1-m1E01-2-0197 | C38 H41 N5 O6 S | 695.27775 | -23.6 | -11.9 |
| 2-1-m1E01-2-0198 | C37 H36 N4 O8 S | 696.22538 | -18.9 | -2.2 |
| 2-1-m1E01-2-0199 | C38 H37 FN4 O6 S | 696.24178 | -4.1 | -5.7 |
| 2-1-m1E01-2-0200 | C38 H37 FN4 O6 S | 696.24178 | -31.6 | -2.0 |
| 2-1-m1E01-2-0201 | C39 H35 F3 N4 O5 | 696.25595 | 0.0 | -7.2 |
| 2-1-m1E01-2-0202 | C38 H40 N4 O7 S | 696.26177 | 0.0 | 0.0 |
| 2-1-m1E01-2-0203 | C40 H39 F3 N4 O4 | 696.29234 | -6.2 | -8.8 |
| 2-1-m1E01-2-0204 | C41 H40 N6 O5 | 696.30602 | 0.0 | 0.0 |
| 2-1-m1E01-2-0205 | C35 H35 N7 O5 S2 | 697.21411 | 0.0 | 20.8 |
| 2-1-m1E01-2-0206 | C36 H35 N5 O8 S | 697.22063 | -16.0 | -12.9 |
| 2-1-m1E01-2-0207 | C37 H36 F N5 O6 S | 697.23703 | -17.3 | -13.7 |
| 2-1-m1E01-2-0208 | C37 H39 N5 O7 S | 697.25702 | -27.8 | -0.7 |
| 2-1-m1E01-2-0209 | C37 H39 N5 O7 S | 697.25702 | -29.2 | 2.6 |
| 2-1-m1E01-2-0210 | C37 H39 N5 O7 S | 697.25702 | 1.0 | 0.0 |
| 2-1-m1E01-2-0211 | C38 H37 F2 N5 O6 | 697.27119 | 5.1 | 16.4 |
| 2-1-m1E01-2-0212 | C38 H37 F2 N5 O6 | 697.27119 | 0.0 | 0.0 |
| 2-1-m1E01-2-0213 | C37 H38 N4 O6 S2 | 698.22328 | -26.5 | -9.8 |
| 2-1-m1E01-2-0214 | C36 H35 F N6 O6 S | 698.23228 | -4.6 | 22.9 |
| 2-1-m1E01-2-0215 | C37 H38 N4 O8 S | 698.24103 | -23.2 | -0.6 |
| 2-1-m1E01-2-0216 | C36 H38 N6 O7 S | 698.25227 | -20.1 | -1.2 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0217 | C36 H38 N6 O7 S | 698.25227 | -10.2 | 3.7 |
| 2-1-m1E01-2-0218 | C36 H38 N6 O7 S | 698.25227 | -14.7 | -9.1 |
| 2-1-m1E01-2-0219 | C41 H38 N4 O5 S | 698.25629 | -15.8 | 6.9 |
| 2-1-m1E01-2-0220 | C37 H36 F2 N6 O6 | 698.26644 | -20.2 | -10.4 |
| 2-1-m1E01-2-0221 | C39 H37 F3 N4 O5 | 698.27160 | -21.7 | -7.4 |
| 2-1-m1E01-2-0222 | C42 H42 N4 04 S | 698.29268 | -4.0 | -18.1 |
| 2-1-m1E01-2-0223 | C39 H46 N4 O6 S | 698.31381 | 0.0 | 0.0 |
| 2-1-m1E01-2-0224 | C36 H37 N5 O6 S2 | 699.21853 | -22.4 | -9.2 |
| 2-1-m1E01-2-0225 | C35 H37 N7 O7 S | 699.24752 | -22.0 | -14.3 |
| 2-1-m1E01-2-0226 | C35 H37 N7 O7 S | 699.24752 | -5.6 | -0.9 |
| 2-1-m1E01-2-0227 | C38 H36 F3 N5 O5 | 699.26685 | -18.6 | -20.6 |
| 2-1-m1E01-2-0228 | C40 H41 N7 O5 | 699.31692 | -11.9 | 1.7 |
| 2-1-m1E01-2-0229 | C34 H36 N8 O7 S | 700.24277 | 0.0 | 0.0 |
| 2-1-m1E01-2-0230 | C38 H35 F3 N4 O6 | 700.25087 | 0.0 | -6.0 |
| 2-1-m1E01-2-0231 | C37 H35 F3 N6 O5 | 700.26210 | -98.7 | 0.0 |
| 2-1-m1E01-2-0232 | C39 H36 F4 N4 O4 | 700.26727 | 0.0 | 0.0 |
| 2-1-m1E01-2-0233 | C41 H40 N4 O5 S | 700.27194 | -56.4 | 64.2 |
| 2-1-m1E01-2-0234 | C38 H44 N4 O7 S | 700.29307 | 0.0 | 0.0 |
| 2-1-m1E01-2-0235 | C36 H39 N5 O8 S | 701.25193 | 0.0 | 0.0 |
| 2-1-m1E01-2-0236 | C38 H38 F3 N5 O5 | 701.28250 | 19.4 | -1.6 |
| 2-1-m1E01-2-0237 | C38 H38 F3 N5 O5 | 701.28250 | -16.7 | -42.3 |
| 2-1-m1E01-2-0238 | C36 H35 F N4 O6 S2 | 702.19820 | -19.2 | 13.0 |
| 2-1-m1E01-2-0239 | C40 H36 F2 N6 O4 | 702.27661 | -18.2 | 2.0 |
| 2-1-m1E01-2-0240 | C37 H37 F3 N6 O5 | 702.27775 | -30.3 | -3.2 |
| 2-1-m1E01-2-0241 | C37 H37 F3 N6 O5 | 702.27775 | -18.2 | 2.0 |
| 2-1-m1E01-2-0242 | C43 H50 N4 O5 | 702.37812 | 0.0 | 0.0 |
| 2-1-m1E01-2-0243 | C35 H34 F N5 O6 S2 | 703.19345 | -21.8 | -3.1 |
| 2-1-m1E01-2-0244 | C35 H37 N5 O7 S2 | 703.21344 | -24.1 | -6.8 |
| 2-1-m1E01-2-0245 | C36 H35 F2 N5 O6 S | 703.22761 | -14.1 | 6.7 |
| 2-1-m1E01-2-0246 | C36 H36 F3 N7 O5 | 703.27300 | -19.8 | 19.0 |
| 2-1-m1E01-2-0247 | C40 H41 N5 O5 S | 703.28284 | -18.7 | -4.6 |
| 2-1-m1E01-2-0248 | C39 H38 F N7 O5 | 703.29185 | -35.8 | -10.1 |
| 2-1-m1E01-2-0249 | C37 H45 N5 O7 S | 703.30397 | 17.9 | 3.9 |
| 2-1-m1E01-2-0250 | C34 H36 N6 O7 S2 | 704.20869 | -1.3 | 2.9 |
| 2-1-m1E01-2-0251 | C34 H36 N6 O7 S2 | 704.20869 | -13.8 | 3.4 |
| 2-1-m1E01-2-0252 | C36 H34 F2 N4 O7 S | 704.21163 | 0.7 | 2.4 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0253 | C41 H44 N4 O5 S | 704.30324 | -40.0 | -5.6 |
| 2-1-m1E01-2-0254 | C41 H44 N4 O5 S | 704.30324 | -24.2 | 3.0 |
| 2-1-m1E01-2-0255 | C40 H44 N6 O6 | 704.33223 | -8.6 | -3.1 |
| 2-1-m1E01-2-0256 | C42 H48 N4 O6 | 704.35739 | 14.4 | -7.1 |
| 2-1-m1E01-2-0257 | C33 H35 N7 O7 S2 | 705.20394 | 0.0 | 0.0 |
| 2-1-m1E01-2-0258 | C36 H34 F3 N5 O5 S | 705.22327 | -11.4 | -29.7 |
| 2-1-m1E01-2-0259 | C35 H36 F N5 O8 S | 705.22686 | -20.4 | -3.9 |
| 2-1-m1E01-2-0260 | C40 H43 N5 O5 S | 705.29849 | -32.3 | -0.8 |
| 2-1-m1E01-2-0261 | C40 H36 F2 N4 O4 S | 706.24253 | 0.0 | 0.0 |
| 2-1-m1E01-2-0262 | C39 H38 N4 O7 S | 706.24612 | -18.6 | -12.7 |
| 2-1-m1E01-2-0263 | C37 H40 F2 N4 O6 S | 706.26366 | 0.0 | 0.0 |
| 2-1-m1E01-2-0264 | C40 H42 N4 O6 S | 706.28251 | -128.5 | -0.2 |
| 2-1-m1E01-2-0265 | C40 H42 N4 O6 S | 706.28251 | 0.0 | 17.3 |
| 2-1-m1E01-2-0266 | C40 H42 N4 O6 S | 706.28251 | -40.0 | -19.2 |
| 2-1-m1E01-2-0267 | C40 H42 N4 O6 S | 706.28251 | 0.0 | 0.0 |
| 2-1-m1E01-2-0268 | C39 H42 N6 O7 | 706.31150 | -11.0 | -0.4 |
| 2-1-m1E01-2-0269 | C38 H37 N5 O7 S | 707.24137 | -19.9 | -5.0 |
| 2-1-m1E01-2-0270 | C38 H37 N5 O7 S | 707.24137 | 3.2 | -173.9 |
| 2-1-m1E01-2-0271 | C39 H38 F N5 O5 S | 707.25777 | 0.0 | 0.0 |
| 2-1-m1E01-2-0272 | C39 H41 N5 O6 S | 707.27775 | -28.6 | -10.6 |
| 2-1-m1E01-2-0273 | C39 H41 N5 O6 S | 707.27775 | 0.0 | -4.6 |
| 2-1-m1E01-2-0274 | C39 H41 N5 O6 S | 707.27775 | -26.9 | -23.8 |
| 2-1-m1E01-2-0275 | C36 H42 F N5 O7 S | 707.27890 | -28.6 | -10.6 |
| 2-1-m1E01-2-0276 | C43 H41 N5 O5 | 707.31077 | -1.5 | 2.3 |
| 2-1-m1E01-2-0277 | C40 H42 F N5 O6 | 707.31191 | -2.4 | 6.0 |
| 2-1-m1E01-2-0278 | C41 H49 N5 O6 | 707.36828 | -26.6 | -3.1 |
| 2-1-m1E01-2-0279 | C35 H35 F3 N6 O5 S | 708.23417 | 15.9 | -15.0 |
| 2-1-m1E01-2-0280 | C37 H36 N6 O7 S | 708.23662 | -16.7 | 2.8 |
| 2-1-m1E01-2-0281 | C37 H36 N6 O7 S | 708.23662 | 0.6 | 0.0 |
| 2-1-m1E01-2-0282 | C39 H40 N4 O7 S | 708.26177 | 0.1 | -1.7 |
| 2-1-m1E01-2-0283 | C39 H40 N4 O7 S | 708.26177 | -36.4 | 0.0 |
| 2-1-m1E01-2-0284 | C38 H40 N6 O6 S | 708.27300 | -7.8 | 0.0 |
| 2-1-m1E01-2-0285 | C40 H41 FN4 O5 S | 708.27817 | -1.6 | -106.7 |
| 2-1-m1E01-2-0286 | C38 H39 N5 O7 S | 709.25702 | -20.0 | -7.7 |
| 2-1-m1E01-2-0287 | C39 H40 F N5 O7 | 709.29118 | 6.7 | -42.7 |
| 2-1-m1E01-2-0288 | C39 H43 N5 O6 S | 709.29340 | -23.4 | 11.9 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0289 | C38 H38 N4 O8 S | 710.24103 | 0.0 | 0.0 |
| 2-1-m1E01-2-0290 | C37 H38 N6 O7 S | 710.25227 | -103.9 | 0.0 |
| 2-1-m1E01-2-0291 | C39 H39 FN4 O6 S | 710.25743 | -10.6 | 3.0 |
| 2-1-m1E01-2-0292 | C39 H39 FN4 O6 S | 710.25743 | 2.3 | 0.0 |
| 2-1-m1E01-2-0293 | C40 H37 F3 N4 O5 | 710.27160 | -24.6 | 7.3 |
| 2-1-m1E01-2-0294 | C38 H42 N6 O6 S | 710.28865 | 0.0 | -13.3 |
| 2-1-m1E01-2-0295 | C43 H39 F N4 O5 | 710.29045 | 0.0 | 0.0 |
| 2-1-m1E01-2-0296 | C41 H41 F3 N4 04 | 710.30799 | 19.0 | -41.4 |
| 2-1-m1E01-2-0297 | C41 H44 F2 N4 O5 | 710.32798 | -18.5 | 8.9 |
| 2-1-m1E01-2-0298 | C38 H38 FN5 O6 S | 711.25268 | 0.0 | 0.0 |
| 2-1-m1E01-2-0299 | C38 H38 F N5 O6 S | 711.25268 | 0.0 | 0.0 |
| 2-1-m1E01-2-0300 | C39 H36 F3 N5 O5 | 711.26685 | -39.9 | 14.4 |
| 2-1-m1E01-2-0301 | C38 H41 N5 O7 S | 711.27267 | -16.8 | -9.8 |
| 2-1-m1E01-2-0302 | C38 H41 N5 O7 S | 711.27267 | -21.3 | 0.2 |
| 2-1-m1E01-2-0303 | C38 H41 N5 O7 S | 711.27267 | -27.1 | -0.1 |
| 2-1-m1E01-2-0304 | C40 H46 F N5 O6 | 711.34321 | -5.6 | 3.7 |
| 2-1-m1E01-2-0305 | C37 H36 N4 O7 S2 | 712.20254 | -23.1 | 14.2 |
| 2-1-m1E01-2-0306 | C38 H40 N4 O6 S2 | 712.23893 | -20.5 | 1.4 |
| 2-1-m1E01-2-0307 | C37 H40 N6 O7 S | 712.26792 | -11.9 | 1.5 |
| 2-1-m1E01-2-0308 | C37 H40 N6 O7 S | 712.26792 | -24.6 | -5.2 |
| 2-1-m1E01-2-0309 | C37 H40 N6 O7 S | 712.26792 | -5.7 | -2.1 |
| 2-1-m1E01-2-0310 | C38 H38 F2 N6 O6 | 712.28209 | -2.4 | -3.5 |
| 2-1-m1E01-2-0311 | C40 H39 F3 N4 O5 | 712.28725 | -240.1 | 0.0 |
| 2-1-m1E01-2-0312 | C36 H35 N5 O7 S2 | 713.19779 | -2.1 | -2.8 |
| 2-1-m1E01-2-0313 | C37 H39 N5 O6 S2 | 713.23418 | 0.0 | 0.0 |
| 2-1-m1E01-2-0314 | C36 H39 N7 O7 S | 713.26317 | 0.0 | 0.0 |
| 2-1-m1E01-2-0315 | C36 H39 N7 O7 S | 713.26317 | -51.3 | -9.1 |
| 2-1-m1E01-2-0316 | C41 H43 N7 O5 | 713.33257 | -26.8 | -6.4 |
| 2-1-m1E01-2-0317 | C37 H35 F N4 O8 S | 714.21596 | -22.2 | -58.1 |
| 2-1-m1E01-2-0318 | C37 H38 N4 O7 S2 | 714.21819 | -2.3 | 31.9 |
| 2-1-m1E01-2-0319 | C38 H36 F2 N4 O6 S | 714.23236 | -7.4 | 0.0 |
| 2-1-m1E01-2-0320 | C38 H36 F2 N4 O6 S | 714.23236 | -37.7 | -9.2 |
| 2-1-m1E01-2-0321 | C36 H37 N5 O7 S2 | 715.21344 | -6.8 | -0.9 |
| 2-1-m1E01-2-0322 | C37 H35 F2 N5 O6 S | 715.22761 | -47.5 | -9.8 |
| 2-1-m1E01-2-0323 | C36 H37 N5 O9 S | 715.23120 | -15.2 | -0.2 |
| 2-1-m1E01-2-0324 | C37 H38 FN5 O7 S | 715.24760 | -10.2 | -7.4 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0325 | C37 H38 FN5 O7 S | 715.24760 | -26.8 | -6.0 |
| 2-1-m1E01-2-0326 | C38 H36 F3 N5 O6 | 715.26177 | -18.6 | -7.2 |
| 2-1-m1E01-2-0327 | C37 H41 N5 O8 S | 715.26758 | 0.0 | 0.0 |
| 2-1-m1E01-2-0328 | C39 H40 F3 N5 O5 | 715.29815 | -31.1 | -12.7 |
| 2-1-m1E01-2-0329 | C40 H41 N7 O6 | 715.31183 | -33.7 | 8.3 |
| 2-1-m1E01-2-0330 | C36 H34 F2 N6 O6 S | 716.22286 | -7.0 | -26.2 |
| 2-1-m1E01-2-0331 | C35 H36 N6 O9 S | 716.22645 | -9.2 | -3.6 |
| 2-1-m1E01-2-0332 | C38 H35 F3 N4 O5 S | 716.22803 | -9.2 | -3.6 |
| 2-1-m1E01-2-0333 | C36 H37 F N6 O7 S | 716.24285 | -17.9 | -2.3 |
| 2-1-m1E01-2-0334 | C38 H35 F3 N4 O7 | 716.24578 | -12.7 | -2.5 |
| 2-1-m1E01-2-0335 | C44 H40 N6 O4 | 716.31110 | 0.0 | 3.9 |
| 2-1-m1E01-2-0336 | C36 H39 N5 O7 S2 | 717.22909 | -8.6 | -5.9 |
| 2-1-m1E01-2-0337 | C35 H36 FN7 O7 S | 717.23810 | 0.0 | 0.0 |
| 2-1-m1E01-2-0338 | C36 H39 N5 O9 S | 717.24685 | -20.3 | -1.7 |
| 2-1-m1E01-2-0339 | C38 H38 F3 N5 O6 | 717.27742 | -12.7 | 23.1 |
| 2-1-m1E01-2-0340 | C41 H43 N5 O5 S | 717.29849 | 0.0 | 0.0 |
| 2-1-m1E01-2-0341 | C38 H47 N5 O7 S | 717.31962 | -25.2 | -1.6 |
| 2-1-m1E01-2-0342 | C35 H38 N6 O7 S2 | 718.22434 | 29.4 | 0.0 |
| 2-1-m1E01-2-0343 | C40 H38 N4 O7 S | 718.24612 | -23.9 | -5.2 |
| 2-1-m1E01-2-0344 | C39 H38 N6 O6 S | 718.25735 | -4.0 | 15.6 |
| 2-1-m1E01-2-0345 | C39 H35 F5 N4 O4 | 718.25785 | -40.2 | -11.3 |
| 2-1-m1E01-2-0346 | C37 H37 F3 N6 O6 | 718.27267 | -25.2 | 1.9 |
| 2-1-m1E01-2-0347 | C42 H46 N4 O5 S | 718.31889 | 1.8 | -342.1 |
| 2-1-m1E01-2-0348 | C42 H46 N4 O5 S | 718.31889 | 0.0 | 0.0 |
| 2-1-m1E01-2-0349 | C42 H46 N4 O5 S | 718.31889 | 0.0 | 0.0 |
| 2-1-m1E01-2-0350 | C38 H37 N7 O6 S | 719.25260 | -25.2 | -1.3 |
| 2-1-m1E01-2-0351 | C37 H36 F3 N5 O7 | 719.25668 | 5.2 | -8.8 |
| 2-1-m1E01-2-0352 | C36 H36 F3 N7 O6 | 719.26792 | -28.1 | 0.9 |
| 2-1-m1E01-2-0353 | C38 H37 F4 N5 O5 | 719.27308 | -2.3 | 7.2 |
| 2-1-m1E01-2-0354 | C40 H41 N5 O6 S | 719.27775 | 0.0 | -9.4 |
| 2-1-m1E01-2-0355 | C37 H45 N5 O8 S | 719.29888 | 10.6 | -35.0 |
| 2-1-m1E01-2-0356 | C41 H45 N5 O5 S | 719.31414 | 0.0 | 0.0 |
| 2-1-m1E01-2-0357 | C36 H34 F2 N4 O6 S2 | 720.18878 | 0.0 | 0.0 |
| 2-1-m1E01-2-0358 | C40 H40 N4 O7 S | 720.26177 | -149.2 | -98.0 |
| 2-1-m1E01-2-0359 | C44 H40 N4 O4 S | 720.27703 | -8.4 | 5.3 |
| 2-1-m1E01-2-0360 | C41 H44 N4 O6 S | 720.29816 | -17.2 | 0.4 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0361 | C41 H44 N4 O6 S | 720.29816 | -63.6 | 0.0 |
| 2-1-m1E01-2-0362 | C41 H44 N4 O6 S | 720.29816 | 0.0 | 0.0 |
| 2-1-m1E01-2-0363 | C41 H44 N4 O6 S | 720.29816 | -91.1 | -5.4 |
| 2-1-m1E01-2-0364 | C35 H33 F2 N5 O6 S2 | 721.18403 | -2.9 | -12.7 |
| 2-1-m1E01-2-0365 | C35 H36 F N5 O7 S2 | 721.20402 | -21.1 | -5.1 |
| 2-1-m1E01-2-0366 | C39 H39 N5 O7 S | 721.25702 | -11.3 | -13.1 |
| 2-1-m1E01-2-0367 | C39 H39 N5 O7 S | 721.25702 | -20.7 | 1.8 |
| 2-1-m1E01-2-0368 | C39 H37 F2 N7 O5 | 721.28242 | 0.0 | 0.0 |
| 2-1-m1E01-2-0369 | C40 H43 N5 O6 S | 721.29340 | -10.2 | -13.0 |
| 2-1-m1E01-2-0370 | C42 H51 N5 O6 | 721.38393 | -36.7 | 2.6 |
| 2-1-m1E01-2-0371 | C34 H35 F N6 O7 S2 | 722.19927 | -16.1 | -1.0 |
| 2-1-m1E01-2-0372 | C38 H38 N6 O7 S | 722.25227 | -9.7 | -8.3 |
| 2-1-m1E01-2-0373 | C40 H42 N4 O7 S | 722.27742 | -47.2 | 4.8 |
| 2-1-m1E01-2-0374 | C40 H42 N4 O7 S | 722.27742 | 0.0 | 0.0 |
| 2-1-m1E01-2-0375 | C41 H43 FN4 O5 S | 722.29382 | -42.7 | -49.8 |
| 2-1-m1E01-2-0376 | C35 H35 F2 N5 O8 S | 723.21744 | 0.0 | 0.0 |
| 2-1-m1E01-2-0377 | C39 H41 N5 O7 S | 723.27267 | 6.3 | 0.3 |
| 2-1-m1E01-2-0378 | C39 H41 N5 O7 S | 723.27267 | 10.4 | 0.0 |
| 2-1-m1E01-2-0379 | C40 H45 N5 O6 S | 723.30905 | -27.3 | -1.0 |
| 2-1-m1E01-2-0380 | C40 H45 N5 O6 S | 723.30905 | 0.0 | 0.0 |
| 2-1-m1E01-2-0381 | C41 H49 N5 O7 | 723.36320 | -13.2 | -36.5 |
| 2-1-m1E01-2-0382 | C37 H36 N6 O6 S2 | 724.21377 | -1.6 | -0.9 |
| 2-1-m1E01-2-0383 | C35 H35 F3 N6 O6 S | 724.22909 | -21.9 | -27.5 |
| 2-1-m1E01-2-0384 | C39 H37 F N4 O7 S | 724.23670 | -13.1 | -20.9 |
| 2-1-m1E01-2-0385 | C39 H37 F N4 O7 S | 724.23670 | 0.0 | 0.0 |
| 2-1-m1E01-2-0386 | C39 H40 N4 O8 S | 724.25668 | -4.4 | 10.2 |
| 2-1-m1E01-2-0387 | C40 H41 F N4 O6 S | 724.27308 | -15.1 | 1.4 |
| 2-1-m1E01-2-0388 | C40 H41 F N4 O6 S | 724.27308 | -60.3 | -11.1 |
| 2-1-m1E01-2-0389 | C39 H44 N6 O6 S | 724.30430 | -34.9 | 8.7 |
| 2-1-m1E01-2-0390 | C45 H48 N4 O5 | 724.36247 | -36.3 | -161.9 |
| 2-1-m1E01-2-0391 | C38 H36 FN5 O7 S | 725.23195 | -21.8 | -13.3 |
| 2-1-m 1 E01-2-0392 | C38 H36 FN5 O7 S | 725.23195 | -28.4 | 16.2 |
| 2-1-m1E01-2-0393 | C39 H37 F2 N5 O5 S | 725.24835 | 0.0 | 0.0 |
| 2-1-m1E01-2-0394 | C38 H39 N5 O8 S | 725.25193 | -14.8 | -7.1 |
| 2-1-m1E01-2-0395 | C36 H41 F2 N5 O7 S | 725.26948 | -22.1 | -7.0 |
| 2-1-m1E01-2-0396 | C40 H38 F3 N5 O5 | 725.28250 | -43.7 | -49.6 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0397 | C39 H43 N5 O7 S | 725.28832 | -21.3 | 8.1 |
| 2-1-m1E01-2-0398 | C39 H43 N5 O7 S | 725.28832 | -7.1 | 11.6 |
| 2-1-m1E01-2-0399 | C39 H43 N5 O7 S | 725.28832 | -10.6 | 17.3 |
| 2-1-m1E01-2-0400 | C39 H43 N5 O7 S | 725.28832 | -49.7 | -20.3 |
| 2-1-m1E01-2-0401 | C38 H38 N4 O7 S2 | 726.21819 | -36.4 | -29.3 |
| 2-1-m1E01-2-0402 | C38 H38 N4 O9 S | 726.23595 | -23.6 | -9.9 |
| 2-1-m1E01-2-0403 | C37 H38 N6 O8 S | 726.24718 | -11.4 | -1.4 |
| 2-1-m1E01-2-0404 | C37 H38 N6 O8 S | 726.24718 | -8.4 | -2.8 |
| 2-1-m1E01-2-0405 | C40 H37 F3 N4 O6 | 726.26652 | -10.6 | -12.2 |
| 2-1-m1E01-2-0406 | C40 H40 F2 N4 O5 S | 726.26875 | -5.8 | -11.7 |
| 2-1-m1E01-2-0407 | C38 H42 N6 O7 S | 726.28357 | -16.6 | -8.2 |
| 2-1-m1E01-2-0408 | C38 H42 N6 O7 S | 726.28357 | -6.5 | -0.2 |
| 2-1-m1E01-2-0409 | C38 H42 N6 O7 S | 726.28357 | -21.1 | -15.7 |
| 2-1-m1E01-2-0410 | C36 H37 N7 O8 S | 727.24243 | -9.3 | -0.6 |
| 2-1-m1E01-2-0411 | C39 H36 F3 N5 O6 | 727.26177 | -30.3 | 4.2 |
| 2-1-m1E01-2-0412 | C38 H41 N5 O8 S | 727.26758 | -28.3 | -2.6 |
| 2-1-m1E01-2-0413 | C38 H41 N5 O8 S | 727.26758 | 0.0 | 5.6 |
| 2-1-m1E01-2-0414 | C37 H41 N7 O7 S | 727.27882 | 0.0 | 0.0 |
| 2-1-m1E01-2-0415 | C39 H42 F N5 O6 S | 727.28398 | 1.1 | -0.1 |
| 2-1-m1E01-2-0416 | C39 H38 F2 N4 O6 S | 728.24801 | -7.2 | -4.5 |
| 2-1-m1E01-2-0417 | C39 H38 F2 N4 O6 S | 728.24801 | 0.0 | 0.0 |
| 2-1-m1E01-2-0418 | C40 H36 F4 N4 O5 | 728.26218 | -15.1 | 3.6 |
| 2-1-m1E01-2-0419 | C37 H40 N6 O8 S | 728.26283 | -13.1 | -8.0 |
| 2-1-m1E01-2-0420 | C42 H40 N4 O6 S | 728.26686 | -14.7 | -8.8 |
| 2-1-m1E01-2-0421 | C42 H40 N4 O6 S | 728.26686 | -3.1 | -1.9 |
| 2-1-m1E01-2-0422 | C38 H37 F2 N5 O6 S | 729.24326 | -15.2 | 9.5 |
| 2-1-m1E01-2-0423 | C37 H39 N5 O9 S | 729.24685 | -4.0 | 1.2 |
| 2-1-m1E01-2-0424 | C36 H39 N7 O8 S | 729.25808 | -30.1 | 0.0 |
| 2-1-m1E01-2-0425 | C41 H39 N5 O6 S | 729.26210 | -13.9 | 31.6 |
| 2-1-m1E01-2-0426 | C38 H40 F N5 O7 S | 729.26325 | -24.0 | -15.5 |
| 2-1-m1E01-2-0427 | C38 H40 F N5 O7 S | 729.26325 | -13.9 | 20.4 |
| 2-1-m1E01-2-0428 | C39 H38 F3 N5 O6 | 729.27742 | -18.5 | -15.2 |
| 2-1-m1E01-2-0429 | C40 H42 F3 N5 O5 | 729.31380 | -23.5 | -32.7 |
| 2-1-m1E01-2-0430 | C40 H45 F2 N5 O6 | 729.33379 | -24.8 | -4.8 |
| 2-1-m1E01-2-0431 | C37 H35 F N4 O7 S2 | 730.19312 | -28.5 | 13.2 |
| 2-1-m1E01-2-0432 | C40 H38 N6 O6 S | 730.25735 | -18.0 | 7.7 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0433 | C40 H38 N6 O6 S | 730.25735 | -49.7 | 0.0 |
| 2-1-m1E01-2-0434 | C37 H39 F N6 O7 S | 730.25850 | -13.0 | 6.4 |
| 2-1-m1E01-2-0435 | C37 H39 F N6 O7 S | 730.25850 | -24.1 | 0.0 |
| 2-1-m1E01-2-0436 | C38 H37 F3 N6 O6 | 730.27267 | -11.5 | -13.6 |
| 2-1-m1E01-2-0437 | C36 H37 N5 O8 S2 | 731.20835 | -21.7 | -5.8 |
| 2-1-m1E01-2-0438 | C37 H41 N5 O7 S2 | 731.24474 | 0.0 | 0.0 |
| 2-1-m1E01-2-0439 | C39 H37 N7 O6 S | 731.25260 | -3.7 | -14.4 |
| 2-1-m1E01-2-0440 | C39 H40 F3 N5 O6 | 731.29307 | 0.0 | 0.0 |
| 2-1-m1E01-2-0441 | C36 H36 N4 O9 S2 | 732.19237 | 0.0 | -15.6 |
| 2-1-m1E01-2-0442 | C35 H36 N6 O8 S2 | 732.20360 | -11.4 | -4.2 |
| 2-1-m1E01-2-0443 | C37 H34 F2 N4 O8 S | 732.20654 | -11.5 | -4.2 |
| 2-1-m1E01-2-0444 | C38 H35 F3 N4 O6 S | 732.22294 | -23.1 | 17.5 |
| 2-1-m1E01-2-0445 | C36 H40 N6 O7 S2 | 732.23999 | -13.6 | 5.8 |
| 2-1-m1E01-2-0446 | C41 H40 N4 O7 S | 732.26177 | 0.0 | 0.0 |
| 2-1-m1E01-2-0447 | C43 H39 F3 N4 04 | 732.29234 | 0.0 | -5.6 |
| 2-1-m1E01-2-0448 | C43 H48 N4 O5 S | 732.33454 | -22.8 | 62.9 |
| 2-1-m1E01-2-0449 | C43 H48 N4 O5 S | 732.33454 | 0.0 | 0.0 |
| 2-1-m1E01-2-0450 | C35 H35 N5 O9 S2 | 733.18762 | 1.6 | -5.0 |
| 2-1-m1E01-2-0451 | C36 H36 F N5 O9 S | 733.22178 | -14.8 | -14.2 |
| 2-1-m1E01-2-0452 | C36 H39 N5 O8 S2 | 733.22400 | -12.6 | -13.1 |
| 2-1-m1E01-2-0453 | C37 H37 F2 N5 O7 S | 733.23818 | -5.6 | 4.8 |
| 2-1-m1E01-2-0454 | C37 H37 F2 N5 O7 S | 733.23818 | -5.7 | 4.8 |
| 2-1-m1E01-2-0455 | C40 H39 N5 O7 S | 733.25702 | 0.0 | 0.0 |
| 2-1-m1E01-2-0456 | C39 H39 N7 O6 S | 733.26825 | -19.5 | 5.3 |
| 2-1-m1E01-2-0457 | C35 H38 N6 O8 S2 | 734.21925 | -81.4 | 4.3 |
| 2-1-m1E01-2-0458 | C40 H38 N4 O6 S2 | 734.22328 | -10.3 | -10.4 |
| 2-1-m1E01-2-0459 | C40 H38 N4 O6 S2 | 734.22328 | -26.8 | 0.0 |
| 2-1-m1E01-2-0460 | C40 H38 N4 O6 S2 | 734.22328 | -4.9 | -22.2 |
| 2-1-m1E01-2-0461 | C36 H36 F2 N6 O7 S | 734.23342 | -2.8 | -28.8 |
| 2-1-m1E01-2-0462 | C41 H42 N4 O7 S | 734.27742 | -42.3 | 14.9 |
| 2-1-m1E01-2-0463 | C41 H37 F3 N6 O4 | 734.28284 | -110.5 | -196.2 |
| 2-1-m1E01-2-0464 | C42 H46 N4 O6 S | 734.31381 | -27.8 | -88.6 |
| 2-1-m1E01-2-0465 | C42 H46 N4 O6 S | 734.31381 | 47.4 | -8.2 |
| 2-1-m1E01-2-0466 | C39 H37 N5 O6 S2 | 735.21853 | -33.9 | -14.2 |
| 2-1-m1E01-2-0467 | C39 H37 N5 O6 S2 | 735.21853 | -21.6 | -12.0 |
| 2-1-m1E01-2-0468 | C39 H37 N5 O6 S2 | 735.21853 | -12.5 | -47.5 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0469 | C35 H35 F2 N7 O7 S | 735.22867 | 0.0 | 0.0 |
| 2-1-m1E01-2-0470 | C37 H36 F3 N5 O6 S | 735.23384 | -22.4 | -5.5 |
| 2-1-m1E01-2-0471 | C37 H36 F3 N5 O8 | 735.25160 | -19.2 | -2.8 |
| 2-1-m1E01-2-0472 | C40 H41 N5 O7 S | 735.27267 | -5.2 | 19.0 |
| 2-1-m1E01-2-0473 | C40 H41 N5 O7 S | 735.27267 | -17.7 | -12.9 |
| 2-1-m1E01-2-0474 | C37 H35 F3 N4 O7 S | 736.21785 | 20.9 | 0.0 |
| 2-1-m1E01-2-0475 | C39 H37 F N6 O6 S | 736.24793 | 0.0 | 0.0 |
| 2-1-m1E01-2-0476 | C40 H40 N4 O8 S | 736.25668 | -20.1 | 4.9 |
| 2-1-m1E01-2-0477 | C41 H44 N4 O7 S | 736.29307 | -29.3 | -22.3 |
| 2-1-m1E01-2-0478 | C41 H44 N4 O7 S | 736.29307 | 5.4 | -34.1 |
| 2-1-m1E01-2-0479 | C42 H45 F N4 O5 S | 736.30947 | 0.0 | 0.0 |
| 2-1-m1E01-2-0480 | C39 H39 N5 O8 S | 737.25193 | -29.9 | 12.3 |
| 2-1-m1E01-2-0481 | C38 H36 F5 N5 O5 | 737.26366 | -11.4 | -28.2 |
| 2-1-m1E01-2-0482 | C41 H47 N5 O6 S | 737.32470 | 0.9 | 0.0 |
| 2-1-m1E01-2-0483 | C41 H47 N5 O6 S | 737.32470 | -48.4 | 0.0 |
| 2-1-m1E01-2-0484 | C41 H47 N5 O6 S | 737.32470 | -9.6 | 15.7 |
| 2-1-m1E01-2-0485 | C41 H37 F3 N4 O4 S | 738.24876 | 0.0 | -36.4 |
| 2-1-m1E01-2-0486 | C40 H39 F N4 O7 S | 738.25235 | 0.0 | 0.0 |
| 2-1-m1E01-2-0487 | C38 H41 F3 N4 O6 S | 738.26989 | 0.0 | 0.0 |
| 2-1-m1E01-2-0488 | C41 H43 F N4 O6 S | 738.28873 | -9.2 | -38.7 |
| 2-1-m1E01-2-0489 | C41 H46 N4 O7 S | 738.30872 | -13.8 | 1.0 |
| 2-1-m1E01-2-0490 | C40 H46 N6 O6 S | 738.31995 | 0.0 | 0.0 |
| 2-1-m1E01-2-0491 | C35 H35 F2 N5 O7 S2 | 739.19460 | -16.5 | 8.2 |
| 2-1-m1E01-2-0492 | C39 H38 F N5 O7 S | 739.24760 | -8.1 | -11.5 |
| 2-1-m1E01-2-0493 | C39 H41 N5 O8 S | 739.26758 | -25.2 | -2.5 |
| 2-1-m1E01-2-0494 | C40 H45 N5 O7 S | 739.30397 | -11.1 | -2.3 |
| 2-1-m1E01-2-0495 | C40 H45 N5 O7 S | 739.30397 | 0.0 | -4.6 |
| 2-1-m1E01-2-0496 | C40 H45 N5 O7 S | 739.30397 | -146.2 | 0.0 |
| 2-1-m1E01-2-0497 | C40 H45 N5 O7 S | 739.30397 | -4.2 | -13.5 |
| 2-1-m1E01-2-0498 | C34 H34 F2 N6 O7 S2 | 740.18985 | -3.8 | 13.7 |
| 2-1-m1E01-2-0499 | C39 H40 N4 O7 S2 | 740.23384 | -74.4 | -403.6 |
| 2-1-m1E01-2-0500 | C38 H40 N6 O8 S | 740.26283 | -12.9 | -4.3 |
| 2-1-m1E01-2-0501 | C38 H40 N6 O8 S | 740.26283 | -3.2 | -14.8 |
| 2-1-m1E01-2-0502 | C40 H41 F N4 O7 S | 740.26800 | -61.8 | 3.7 |
| 2-1-m1E01-2-0503 | C40 H41 F N4 O7 S | 740.26800 | -20.5 | -14.0 |
| 2-1-m1E01-2-0504 | C41 H42 F2 N4 05 S | 740.28440 | 23.7 | 24.0 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0505 | C39 H44 N6 O7 S | 740.29922 | 0.0 | 0.0 |
| 2-1-m1E01-2-0506 | C44 H44 N4 05 S | 740.30324 | 0.0 | 0.0 |
| 2-1-m1E01-2-0507 | C37 H39 N7 O8 S | 741.25808 | -9.2 | -0.5 |
| 2-1-m1E01-2-0508 | C40 H38 F3 N5 O6 | 741.27742 | -39.2 | -0.9 |
| 2-1-m1E01-2-0509 | C39 H43 N5 O8 S | 741.28323 | -30.8 | 1.1 |
| 2-1-m1E01-2-0510 | C39 H43 N5 O8 S | 741.28323 | 31.7 | 18.7 |
| 2-1-m1E01-2-0511 | C40 H44 F N5 O6 S | 741.29963 | -2.9 | 4.7 |
| 2-1-m1E01-2-0512 | C38 H38 N4 O8 S2 | 742.21311 | -26.8 | -19.1 |
| 2-1-m1E01-2-0513 | C38 H38 N4 O8 S2 | 742.21311 | 0.0 | 0.0 |
| 2-1-m1E01-2-0514 | C39 H36 F2 N4 O7 S | 742.22728 | -6.0 | -76.3 |
| 2-1-m1E01-2-0515 | C39 H36 F2 N4 O7 S | 742.22728 | -6.5 | 1.1 |
| 2-1-m1E01-2-0516 | C40 H40 F2 N4 O6 S | 742.26366 | -11.0 | 2.7 |
| 2-1-m1E01-2-0517 | C40 H38 N8 05 S | 742.26859 | -13.5 | -4.8 |
| 2-1-m1E01-2-0518 | C38 H42 N6 O8 S | 742.27848 | -225.1 | -32.6 |
| 2-1-m1E01-2-0519 | C38 H42 N6 O8 S | 742.27848 | -20.7 | -22.2 |
| 2-1-m1E01-2-0520 | C43 H42 N4 O6 S | 742.28251 | -14.4 | 2.2 |
| 2-1-m1E01-2-0521 | C42 H45 F3 N4 O5 | 742.33421 | -6.1 | 0.0 |
| 2-1-m1E01-2-0522 | C37 H37 N5 O8 S2 | 743.20835 | -26.0 | -1.1 |
| 2-1-m1E01-2-0523 | C38 H35 F2 N5 O7 S | 743.22253 | 7.3 | 7.0 |
| 2-1-m1E01-2-0524 | C38 H38 F N5 O8 S | 743.24251 | -18.8 | -8.0 |
| 2-1-m1E01-2-0525 | C38 H38 F N5 O8 S | 743.24251 | -18.3 | -7.8 |
| -1-m1E01-2-0526 | C38 H41 N5 O9 S | 743.26250 | -5.9 | 3.9 |
| 2-1-m1E01-2-0527 | C42 H41 N5 O6 S | 743.27775 | -32.5 | -2.0 |
| 2-1-m1E01-2-0528 | C39 H42 F N5 O7 S | 743.27890 | 0.0 | 0.0 |
| 2-1-m1E01-2-0529 | C39 H42 F N5 O7 S | 743.27890 | -32.7 | 1.9 |
| 2-1-m1E01-2-0530 | C36 H36 N6 O8 S2 | 744.20360 | -18.8 | -7.5 |
| 2-1-m1E01-2-0531 | C37 H37 F N6 O8 S | 744.23776 | -11.0 | 0.0 |
| 2-1-m1E01-2-0532 | C37 H37 F N6 O8 S | 744.23776 | -14.7 | -7.9 |
| 2-1-m1E01-2-0533 | C40 H36 F4 N4 O6 | 744.25710 | 0.0 | 0.0 |
| 2-1-m1E01-2-0534 | C41 H40 N6 O6 S | 744.27300 | 0.0 | 0.0 |
| 2-1-m1E01-2-0535 | C39 H39 F3 N6 O6 | 744.28832 | -7.5 | 2.4 |
| 2-1-m1E01-2-0536 | C37 H39 N5 O8 S2 | 745.22400 | -19.9 | -10.4 |
| 2-1-m1E01-2-0537 | C37 H39 N5 O10 S | 745.24176 | -5.4 | 3.8 |
| 2-1-m1E01-2-0538 | C39 H38 F3 N5 O7 | 745.27233 | -19.7 | -3.6 |
| 2-1-m1E01-2-0539 | C39 H41 F2 N5 O6 S | 745.27456 | -18.0 | -5.2 |
| 2-1-m1E01-2-0540 | C37 H38 N4 O9 S2 | 746.20802 | -36.9 | -20.6 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0541 | C40 H38 N6 05 S2 | 746.23451 | 0.0 | 25.3 |
| 2-1-m1E01-2-0542 | C39 H37 F3 N4 O6 S | 746.23859 | -15.9 | -32.7 |
| 2-1-m1E01-2-0543 | C39 H37 F3 N4 O6 S | 746.23859 | 0.0 | 0.0 |
| 2-1-m1E01-2-0544 | C39 H37 F3 N4 O6 S | 746.23859 | -150.5 | 45.9 |
| 2-1-m1E01-2-0545 | C39 H37 F3 N4 O6 S | 746.23859 | -16.7 | -45.3 |
| 2-1-m1E01-2-0546 | C41 H38 N4 O8 S | 746.24103 | 0.0 | 0.0 |
| 2-1-m1E01-2-0547 | C37 H42 N6 O7 S2 | 746.25564 | -8.4 | 5.3 |
| 2-1-m1E01-2-0548 | C38 H37 F3 N6 O7 | 746.26758 | 18.2 | 3.7 |
| 2-1-m1E01-2-0549 | C42 H42 N4 O7 S | 746.27742 | -17.1 | -4.5 |
| 2-1-m1E01-2-0550 | C43 H46 N4 O6 S | 746.31381 | 0.0 | 0.0 |
| 2-1-m1E01-2-0551 | C44 H50 N4 05 S | 746.35019 | 0.0 | 0.0 |
| 2-1-m1E01-2-0552 | C38 H36 F3 N5 O6 S | 747.23384 | -35.3 | 12.8 |
| 2-1-m1E01-2-0553 | C38 H36 F3 N5 O6 S | 747.23384 | -40.2 | -31.6 |
| 2-1-m1E01-2-0554 | C38 H39 F2 N5 O7 S | 747.25383 | 13.1 | 24.3 |
| 2-1-m1E01-2-0555 | C38 H39 F2 N5 O7 S | 747.25383 | 0.0 | 0.0 |
| 2-1-m1E01-2-0556 | C39 H37 F4 N5 O6 | 747.26800 | 88.0 | 45.3 |
| 2-1-m1E01-2-0557 | C42 H45 N5 O6 S | 747.30905 | 9.3 | 3.8 |
| 2-1-m1E01-2-0558 | C36 H36 N4 O8 S3 | 748.16953 | -3.4 | -3.5 |
| 2-1-m1E01-2-0559 | C37 H34 F2 N4 O7 S2 | 748.18370 | -23.0 | -35.5 |
| 2-1-m1E01-2-0560 | C37 H35 F3 N6 O6 S | 748.22909 | 12.9 | -14.3 |
| 2-1-m1E01-2-0561 | C41 H40 N4 O6 S2 | 748.23893 | -18.5 | 16.1 |
| 2-1-m1E01-2-0562 | C41 H40 N4 O6 S2 | 748.23893 | -27.0 | -9.2 |
| 2-1-m1E01-2-0563 | C40 H37 F N6 O6 S | 748.24793 | 0.0 | -61.6 |
| 2-1-m1E01-2-0564 | C37 H38 F2 N6 O7 S | 748.24907 | 0.0 | 0.0 |
| 2-1-m1E01-2-0565 | C43 H48 N4 O6 S | 748.32946 | 0.0 | 0.0 |
| 2-1-m1E01-2-0566 | C35 H35 N5 O8 S3 | 749.16478 | -22.3 | -14.6 |
| 2-1-m1E01-2-0567 | C36 H36 F N5 O8 S2 | 749.19893 | -19.6 | -5.9 |
| 2-1-m1E01-2-0568 | C40 H39 N5 O6 S2 | 749.23418 | -6.4 | 0.0 |
| 2-1-m1E01-2-0569 | C39 H39 N7 O7 S | 749.26317 | -11.3 | -1.9 |
| 2-1-m1E01-2-0570 | C41 H43 N5 O7 S | 749.28832 | -57.8 | -7.9 |
| 2-1-m1E01-2-0571 | C36 H35 F N4 O9 S2 | 750.18295 | -30.9 | 11.9 |
| 2-1-m1E01-2-0572 | C39 H38 N6 O6 S2 | 750.22942 | -4.5 | -15.6 |
| 2-1-m1E01-2-0573 | C41 H39 F N4 O7 S | 750.25235 | 0.0 | 0.0 |
| 2-1-m1E01-2-0574 | C38 H38 N8 O7 S | 750.25842 | -11.8 | -8.5 |
| 2-1-m1E01-2-0575 | C40 H36 F6 N4 04 | 750.26407 | 0.0 | 0.0 |
| 2-1-m1E01-2-0576 | C41 H37 F3 N6 O5 | 750.27775 | -13.6 | -12.0 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0577 | C42 H46 N4 O7 S | 750.30872 | -27.1 | 3.4 |
| 2-1-m1E01-2-0578 | C41 H46 N6 O6 S | 750.31995 | -31.4 | -2.8 |
| 2-1-m1E01-2-0579 | C35 H37 N5 O10 S2 | 751.19818 | -34.4 | 17.1 |
| 2-1-m1E01-2-0580 | C36 H35 F2 N5 O9 S | 751.21235 | -6.5 | 0.0 |
| 2-1-m1E01-2-0581 | C37 H36 F3 N5 O7 S | 751.22875 | -13.6 | -3.3 |
| 2-1-m1E01-2-0582 | C40 H41 N5 O8 S | 751.26758 | -44.6 | 3.7 |
| 2-1-m1E01-2-0583 | C40 H41 N5 O8 S | 751.26758 | -18.1 | -63.9 |
| 2-1-m1E01-2-0584 | C42 H49 N5 O6 S | 751.34036 | -8.8 | -1.7 |
| 2-1-m1E01-2-0585 | C42 H49 N5 O6 S | 751.34036 | 0.0 | 0.0 |
| 2-1-m1E01-2-0586 | C34 H36 N6 O10 S2 | 752.19343 | -14.3 | -6.2 |
| 2-1-m1E01-2-0587 | C37 H35 F3 N4 O6 S2 | 752.19501 | -14.3 | -6.2 |
| 2-1-m1E01-2-0588 | C37 H35 F3 N4 O8 S | 752.21277 | 92.2 | 253.5 |
| 2-1-m1E01-2-0589 | C40 H37 F N4 O6 S2 | 752.21385 | -34.0 | 0.0 |
| 2-1-m1E01-2-0590 | C40 H37 F N4 O6 S2 | 752.21385 | -8.9 | 0.0 |
| 2-1-m1E01-2-0591 | C39 H40 N6 O8 S | 752.26283 | -17.0 | -5.3 |
| 2-1-m1E01-2-0592 | C41 H41 F N4 O7 S | 752.26800 | -20.8 | -52.6 |
| 2-1-m1E01-2-0593 | C41 H44 N4 O8 S | 752.28799 | 0.0 | 0.0 |
| 2-1-m1E01-2-0594 | C42 H48 N4 O7 S | 752.32437 | -2.0 | -3.8 |
| 2-1-m1E01-2-0595 | C36 H34 F3 N5 O6 S2 | 753.19026 | -73.4 | 0.0 |
| 2-1-m1E01-2-0596 | C39 H39 N5 O7 S2 | 753.22909 | 12.8 | 2.6 |
| 2-1-m1E01-2-0597 | C39 H39 N5 O7 S2 | 753.22909 | 0.0 | -27.2 |
| 2-1-m1E01-2-0598 | C40 H43 N5 O8 S | 753.28323 | -15.8 | -14.9 |
| 2-1-m1E01-2-0599 | C40 H38 F3 N7 O5 | 753.28865 | -24.3 | -35.9 |
| 2-1-m1E01-2-0600 | C41 H47 N5 O7 S | 753.31962 | -40.2 | -18.5 |
| 2-1-m1E01-2-0601 | C41 H47 N5 O7 S | 753.31962 | -17.2 | 18.6 |
| 2-1-m1E01-2-0602 | C38 H38 N6 O7 S2 | 754.22434 | -20.4 | 0.0 |
| 2-1-m1E01-2-0603 | C38 H38 N6 O7 S2 | 754.22434 | -6.0 | -6.2 |
| 2-1-m1E01-2-0604 | C38 H38 N6 O7 S2 | 754.22434 | -21.4 | -1.0 |
| 2-1-m1E01-2-0605 | C38 H38 N6 O7 S2 | 754.22434 | -16.5 | -7.0 |
| 2-1-m1E01-2-0606 | C41 H37 F3 N4 05 S | 754.24368 | 0.0 | 0.0 |
| 2-1-m1E01-2-0607 | C40 H39 F N4 O8 S | 754.24726 | 0.0 | 0.0 |
| 2-1-mlE01-2-0608 | C38 H41 F3 N4 O7 S | 754.26480 | -26.3 | -5.4 |
| 2-1-mlE01-2-0609 | C39 H42 N6 O8 S | 754.27848 | -14.0 | -6.5 |
| 2-1-mlE01-2-0610 | C39 H42 N6 O8 S | 754.27848 | 0.0 | 0.0 |
| 2-1-mlE01-2-0611 | C42 H44 F2 N4 05 S | 754.30005 | 9.3 | -120.6 |
| 2-1-mlE01-2-0612 | C45 H46 N4 05 S | 754.31889 | -2.5 | 1.3 |

(continued)

| [Table 11-2-5] | | | | |
|---|---|---|---|---|
| | | | AS-MS ratio (%) | |
| ID | Molecular Formula | Exact Mass | N1 | N2 |
| 2-1-mlE01-2-0613 | C37 H37 N7 O7 S2 | 755.21959 | -21.4 | -9.3 |
| 2-1-m1E01-2-0614 | C36 H36 F3 N5 O8 S | 755.22367 | -24.7 | -10.5 |
| 2-1-mlE01-2-0615 | C39 H41 N5 O9 S | 755.26250 | -5.7 | -4.2 |
| 2-1-mlE01-2-0616 | C40 H45 N5 O8 S | 755.29888 | 15.2 | -2.0 |
| 2-1-mlE01-2-0617 | C40 H45 N5 O8 S | 755.29888 | 0.0 | 0.0 |
| 2-1-mlE01-2-0618 | C41 H46 F N5 O6 S | 755.31528 | 0.0 | 0.0 |
| 2-1-mlE01-2-0619 | C36 H36 N8 O7 S2 | 756.21484 | -28.7 | -6.5 |
| 2-1-mlE01-2-0620 | C39 H40 N4 O8 S2 | 756.22876 | -21.7 | 3.3 |
| 2-1-m1E01-2-0621 | C38 H40 N6 O9 S | 756.25775 | 0.0 | 0.0 |
| 2-1-mlE01-2-0622 | C43 H40 N4 O7 S | 756.26177 | -13.2 | -26.1 |
| 2-1-mlE01-2-0623 | C44 H44 N4 O6 S | 756.29816 | -17.9 | -1.9 |
| 2-1-mlE01-2-0624 | C41 H45 F N4 O7 S | 756.29930 | 0.0 | -104.0 |
| 2-1-mlE01-2-0625 | C39 H37 F2 N5 O7 S | 757.23818 | -31.1 | 17.7 |
| 2-1-m1E01-2-0626 | C40 H38 F3 N5 05 S | 757.25457 | 0.0 | 0.0 |
| 2-1-mlE01-2-0627 | C42 H39 N5 O7 S | 757.25702 | -24.1 | -4.6 |
| 2-1-mlE01-2-0628 | C39 H40 F N5 O8 S | 757.25816 | -4.1 | -0.7 |
| 2-1-mlE01-2-0629 | C37 H42 F3 N5 O7 S | 757.27570 | -13.5 | -1.2 |
| 2-1-mlE01-2-0630 | C40 H44 F N5 O7 S | 757.29455 | -18.9 | -28.7 |
| 2-1-m1E01-2-0631 | C40 H47 N5 O8 S | 757.31453 | -23.4 | -3.8 |
| 2-1-mlE01-2-0632 | C39 H37 F3 N6 05 S | 758.24982 | 0.0 | 0.0 |
| 2-1-mlE01-2-0633 | C38 H39 F N6 O8 S | 758.25341 | -13.1 | -3.8 |
| 2-1-mlE01-2-0634 | C40 H40 F2 N4 O7 S | 758.25858 | -18.0 | 16.6 |
| 2-1-m 1 E01-2-0635 | C41 H41 F3 N4 05 S | 758.27498 | 1.8 | 23.2 |
| 2-1-m1E01-2-0636 | C42 H42 N6 O6 S | 758.28865 | 0.0 | 0.0 |
| 2-1-m1E01-2-0637 | C39 H46 N6 O8 S | 758.30978 | -8.1 | -0.7 |
| 2-1-m1E01-2-0638 | C42 H45 F3 N4 O6 | 758.32912 | 11.1 | -21.2 |
| 2-1-m1E01-2-0639 | C38 H41 N5 O8 S2 | 759.23965 | 0.1 | -4.6 |
| 2-1-m1E01-2-0640 | C39 H42 F N5 O8 S | 759.27381 | -16.6 | 0.0 |
| 2-1-m1E01-2-0641 | C39 H42 F N5 O8 S | 759.27381 | 0.0 | 0.0 |
| 2-1-m1E01-2-0642 | C40 H43 F2 N5 O6 S | 759.29021 | -9.8 | -7.8 |
| 2-1-m1E01-2-0643 | C36 H36 N6 O7 S3 | 760.18076 | -11.4 | -9.0 |
| 2-1-m1E01-2-0644 | C38 H37 F N4 O8 S2 | 760.20368 | -19.2 | -103.2 |
| 2-1-m1E01-2-0645 | C39 H35 F3 N4 O7 S | 760.21785 | -23.3 | -98.8 |
| 2-1-m1E01-2-0646 | C38 H40 N4 O9 S2 | 760.22367 | -20.8 | 2.3 |
| 2-1-m1E01-2-0647 | C40 H39 F3 N4 O6 S | 760.25424 | -12.5 | -12.4 |
| 2-1-m1E01-2-0648 | C40 H39 F3 N4 O6 S | 760.25424 | -29.5 | -24.5 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0649 | C41 H40 N6 O7 S | 760.26792 | 0.0 | 0.0 |
| 2-1-m1E01-2-0650 | C43 H41 FN4 O6 S | 760.27308 | 5.8 | 17.7 |
| 2-1-mlE01-2-0651 | C39 H39 F3 N6 O7 | 760.28323 | -18.8 | -1.1 |
| 2-1-m1E01-2-0652 | C43 H44 N4 O7 S | 760.29307 | -6.5 | 20.3 |
| 2-1-m1E01-2-0653 | C44 H48 N4 O6 S | 760.32946 | -15.4 | -7.3 |
| 2-1-m1E01-2-0654 | C35 H35 N7 O7 S3 | 761.17601 | -9.7 | -11.0 |
| 2-1-m1E01-2-0655 | C37 H39 N5 O9 S2 | 761.21892 | -12.6 | -1.8 |
| 2-1-m1E01-2-0656 | C37 H39 N5 O9 S2 | 761.21892 | -28.0 | -69.7 |
| 2-1-m1E01-2-0657 | C38 H37 F2 N5 O8 S | 761.23309 | -25.6 | -5.6 |
| 2-1-m1E01-2-0658 | C38 H37 F2 N5 O8 S | 761.23309 | 2.7 | -1.0 |
| 2-1-m1E01-2-0659 | C39 H38 F3 N5 O6 S | 761.24949 | 0.0 | -50.0 |
| 2-1-m1E01-2-0660 | C39 H41 F2 N5 O7 S | 761.26948 | 0.0 | 0.0 |
| 2-1-m1E01-2-0661 | C41 H46 F3 N5 O6 | 761.34002 | -32.4 | 1.4 |
| 2-1-m1E01-2-0662 | C37 H38 N4 O8 S3 | 762.18518 | -27.4 | 31.3 |
| 2-1-m1E01-2-0663 | C37 H38 N4 O10 S2 | 762.20293 | -47.6 | 0.0 |
| 2-1-m1E01-2-0664 | C36 H38 N6 O9 S2 | 762.21417 | -16.6 | -7.3 |
| 2-1-m1E01-2-0665 | C41 H38 N4 O7 S2 | 762.21819 | -16.2 | -7.3 |
| 2-1-m1E01-2-0666 | C37 H36 F2 N6 O8 S | 762.22834 | 12.0 | -1.5 |
| 2-1-m1E01-2-0667 | C39 H37 F3 N4 O7 S | 762.23350 | -27.7 | 6.9 |
| 2-1-m1E01-2-0668 | C39 H37 F3 N4 O7 S | 762.23350 | -6.4 | -54.1 |
| 2-1-m1E01-2-0669 | C42 H42 N4 O6 S2 | 762.25458 | -19.4 | 12.7 |
| 2-1-m1E01-2-0670 | C42 H42 N4 O6 S2 | 762.25458 | -65.7 | -4.8 |
| 2-1-m1E01-2-0671 | C42 H42 N4 O8 S | 762.27234 | -11.5 | -23.1 |
| 2-1-m1E01-2-0672 | C38 H36 F3 N5 O7 S | 763.22875 | -35.3 | 4.0 |
| 2-1-mlE01-2-0673 | C41 H41 N5 O6 S2 | 763.24983 | -22.7 | 6.8 |
| 2-1-m1E01-2-0674 | C39 H37 F4 N5 O7 | 763.26291 | -16.4 | 25.7 |
| 2-1-m1E01-2-0675 | C40 H41 N7 O7 S | 763.27882 | -33.6 | -4.7 |
| 2-1-m1E01-2-0676 | C38 H35 F3 N4 O8 S | 764.21277 | -8.2 | -37.8 |
| 2-1-mlE01-2-0677 | C37 H35 F3 N6 O7 S | 764.22400 | -0.2 | -18.2 |
| 2-1-m1E01-2-0678 | C39 H36 F4 N4 O6 S | 764.22917 | -18.1 | -11.2 |
| 2-1-m1E01-2-0679 | C41 H40 N4 O7 S2 | 764.23384 | -67.3 | 7.9 |
| 2-1-mlE01-2-0680 | C41 H40 N4 O7 S2 | 764.23384 | 0.9 | -58.1 |
| 2-1-m1E01-2-0681 | C43 H45 F N4 O6 S | 764.30438 | 0.0 | 0.0 |
| 2-1-mlE01-2-0682 | C36 H39 N5 O10 S2 | 765.21383 | -12.6 | -17.1 |
| 2-1-m1E01-2-0683 | C38 H38 F3 N5 O7 S | 765.24440 | 12.4 | 3.5 |
| 2-1-m1E01-2-0684 | C38 H38 F3 N5 O7 S | 765.24440 | -20.7 | -13.3 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0685 | C38 H38 F3 N5 O7 S | 765.24440 | 9.4 | 11.9 |
| 2-1-m1E01-2-0686 | C38 H38 F3 N5 O7 S | 765.24440 | 0.0 | 0.0 |
| 2-1-m1E01-2-0687 | C41 H43 N5 O8 S | 765.28323 | -34.1 | -11.6 |
| 2-1-m1E01-2-0688 | C42 H47 N5 O7 S | 765.31962 | 8.1 | 14.7 |
| 2-1-m1E01-2-0689 | C43 H51 N5 O6 S | 765.35601 | -330.7 | 0.0 |
| 2-1-m1E01-2-0690 | C36 H35 F N4 O8 S3 | 766.16010 | -21.0 | 0.0 |
| 2-1-m1E01-2-0691 | C40 H36 F2 N6 O6 S | 766.23851 | -11.7 | 5.6 |
| 2-1-m1E01-2-0692 | C37 H37 F3 N6 O7 S | 766.23965 | -11.7 | 5.6 |
| 2-1-m1E01-2-0693 | C37 H37 F3 N6 O7 S | 766.23965 | -21.7 | -3.6 |
| 2-1-m1E01-2-0694 | C40 H36 F6 N4 O5 | 766.25899 | 52.2 | -29.3 |
| 2-1-m1E01-2-0695 | C40 H42 N6 O6 S2 | 766.26072 | 0.0 | 0.0 |
| 2-1-m1E01-2-0696 | C41 H46 N6 O7 S | 766.31487 | -20.1 | -10.3 |
| 2-1-m1E01-2-0697 | C43 H50 N4 O7 S | 766.34002 | -10.6 | -6.9 |
| 2-1-m1E01-2-0698 | C35 H37 N5 O9 S3 | 767.17534 | -23.0 | -0.8 |
| 2-1-m1E01-2-0699 | C36 H35 F2 N5 O8 S2 | 767.18951 | -18.8 | -10.8 |
| 2-1-m1E01-2-0700 | C40 H38 F N5 O6 S2 | 767.22475 | -3.8 | -12.8 |
| 2-1-m1E01-2-0701 | C36 H36 F3 N7 O7 S | 767.23490 | 0.0 | 0.0 |
| 2-1-m1E01-2-0702 | C40 H41 N5 O7 S2 | 767.24474 | -5.4 | -6.2 |
| 2-1-m1E01-2-0703 | C40 H41 N5 O7 S2 | 767.24474 | -82.8 | -3.1 |
| 2-1-m1E01-2-0704 | C39 H38 F N7 O7 S | 767.25375 | -21.6 | -7.1 |
| 2-1-m1E01-2-0705 | C42 H49 N5 O7 S | 767.33527 | -30.8 | 17.8 |
| 2-1-m1E01-2-0706 | C34 H36 N6 O9 S3 | 768.17059 | -15.0 | -4.9 |
| 2-1-m1E01-2-0707 | C36 H34 F2 N4 O9 S2 | 768.17353 | -14.4 | -7.2 |
| 2-1-m1E01-2-0708 | C37 H35 F3 N4 O7 S2 | 768.18993 | 31.3 | -259.8 |
| 2-1-m1E01-2-0709 | C39 H40 N6 O7 S2 | 768.23999 | -8.4 | -9.5 |
| 2-1-mlE01-2-0710 | C39 H40 N6 O7 S2 | 768.23999 | -26.3 | -2.3 |
| 2-1-m1E01-2-0711 | C41 H38 F2 N4 O7 S | 768.24293 | -11.2 | -7.0 |
| 2-1-m1E01-2-0712 | C40 H44 N6 O8 S | 768.29413 | -9.7 | -8.5 |
| 2-1-mlE01-2-0713 | C42 H48 N4 O8 S | 768.31929 | -97.3 | 3.9 |
| 2-1-m1E01-2-0714 | C46 H48 N4 O5 S | 768.33454 | -9.0 | 14.5 |
| 2-1-m1E01-2-0715 | C36 H34 F3 N5 O7 S2 | 769.18517 | -17.4 | 7.2 |
| 2-1-m1E01-2-0716 | C35 H36 F N5 O10 S2 | 769.18876 | -7.9 | -1.4 |
| 2-1-m1E01-2-0717 | C38 H39 N7 O7 S2 | 769.23524 | -31.9 | 8.2 |
| 2-1-m1E01-2-0718 | C38 H39 N7 O7 S2 | 769.23524 | 0.0 | 0.0 |
| 2-1-m1E01-2-0719 | C40 H40 FN5 O8 S | 769.25816 | 0.0 | 0.0 |
| 2-1-mlE01-2-0720 | C39 H37 F6 N5 O5 | 769.26989 | 0.0 | 0.0 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0721 | C40 H38 F3 N7 O6 | 769.28357 | -172.5 | 11.7 |
| 2-1-mlE01-2-0722 | C41 H47 N5 O8 S | 769.31453 | -21.9 | -16.1 |
| 2-1-m1E01-2-0723 | C40 H36 F2 N4 O6 S2 | 770.20443 | -45.1 | -5.3 |
| 2-1-mlE01-2-0724 | C40 H36 F2 N4 O6 S2 | 770.20443 | -9.6 | -5.0 |
| 2-1-mlE01-2-0725 | C40 H42 N4 O8 S2 | 770.24441 | 0.0 | 0.0 |
| 2-1-m1E01-2-0726 | C39 H42 N6 O9 S | 770.27340 | -14.7 | -9.8 |
| 2-1-mlE01-2-0727 | C36 H36 F3 N5 O7 S2 | 771.20082 | -53.2 | -27.3 |
| 2-1-m1E01-2-0728 | C36 H36 F3 N5 O9 S | 771.21858 | 22.4 | -12.4 |
| 2-1-mlE01-2-0729 | C39 H38 F N5 O7 S2 | 771.21967 | 11.2 | 3.6 |
| 2-1-m1E01-2-0730 | C39 H38 F N5 O7 S2 | 771.21967 | 0.0 | 0.0 |
| 2-1-m1E01-2-0731 | C43 H41 N5 O7 S | 771.27267 | -2.6 | 4.6 |
| 2-1-m1E01-2-0732 | C40 H42 F N5 O8 S | 771.27381 | -2.6 | 4.6 |
| 2-1-m1E01-2-0733 | C40 H45 N5 O9 S | 771.29380 | 9.5 | 6.4 |
| 2-1-m1E01-2-0734 | C41 H49 N5 O8 S | 771.33018 | -3.8 | -2.9 |
| 2-1-m 1 E01-2-0735 | C35 H35 F3 N6 O7 S2 | 772.19607 | 0.0 | 0.0 |
| 2-1-m1E01-2-0736 | C37 H36 N6 O9 S2 | 772.19852 | -10.1 | 9.5 |
| 2-1-m1E01-2-0737 | C39 H40 N4 O9 S2 | 772.22367 | -7.4 | -1.3 |
| 2-1-m1E01-2-0738 | C42 H43 F3 N4 O5 S | 772.29063 | -27.2 | 0.0 |
| 2-1-m1E01-2-0739 | C44 H44 N4 O7 S | 772.29307 | -48.8 | 0.0 |
| 2-1-m1E01-2-0740 | C40 H38 F3 N5 O6 S | 773.24949 | -15.7 | 0.0 |
| 2-1-m1E01-2-0741 | C39 H40 F N5 O9 S | 773.25308 | -18.7 | -13.9 |
| 2-1-m1E01-2-0742 | C37 H42 F3 N5 O8 S | 773.27062 | 0.0 | 0.0 |
| 2-1-m1E01-2-0743 | C41 H45 F2 N5 O6 S | 773.30586 | -5.4 | 2.5 |
| 2-1-m1E01-2-0744 | C40 H37 F3 N4 O7 S | 774.23350 | -6.6 | -18.9 |
| 2-1-m1E01-2-0745 | C43 H39 F N4 O7 S | 774.25235 | -4.2 | -11.0 |
| 2-1-m1E01-2-0746 | C41 H41 F3 N4 O6 S | 774.26989 | -154.4 | 4.7 |
| 2-1-m1E01-2-0747 | C41 H41 F3 N4 O6 S | 774.26989 | -326.6 | -181.6 |
| 2-1-m1E01-2-0748 | C41 H41 F3 N4 O6 S | 774.26989 | -8.2 | 0.0 |
| 2-1-m1E01-2-0749 | C41 H44 F2 N4 O7 S | 774.28988 | -5.4 | -2.7 |
| 2-1-m1E01-2-0750 | C45 H50 N4 O6 S | 774.34511 | 0.0 | 0.0 |
| 2-1-m1E01-2-0751 | C39 H36 F3 N5 O7 S | 775.22875 | -31.9 | 28.5 |
| 2-1-m1E01-2-0752 | C38 H41 N5 O9 S2 | 775.23457 | -11.6 | 2.2 |
| 2-1-m1E01-2-0753 | C40 H46 F N5 O8 S | 775.30511 | -15.3 | -3.6 |
| 2-1-m1E01-2-0754 | C38 H40 N4 O8 S3 | 776.20083 | 0.0 | -14.9 |
| 2-1-m1E01-2-0755 | C38 H38 F2 N6 O8 S | 776.24399 | -20.0 | -0.3 |
| 2-1-m1E01-2-0756 | C40 H39 F3 N4 O7 S | 776.24915 | -26.0 | -6.4 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0757 | C40 H39 F3 N4 O7 S | 776.24915 | -30.7 | -18.3 |
| 2-1-m1E01-2-0758 | C44 H48 N4 O7 S | 776.32437 | -118.1 | 66.6 |
| 2-1-m1E01-2-0759 | C39 H38 F3 N5 O7 S | 777.24440 | 0.0 | 0.0 |
| 2-1-m1E01-2-0760 | C39 H41 F2 N5 O8 S | 777.26439 | 119.2 | 39.9 |
| 2-1-m1E01-2-0761 | C42 H43 N5 O6 S2 | 777.26548 | 0.0 | 0.0 |
| 2-1-m1E01-2-0762 | C40 H42 F3 N5 O6 S | 777.28079 | 12.5 | -36.5 |
| 2-1-m1E01-2-0763 | C41 H43 N7 O7 S | 777.29447 | -20.1 | 10.8 |
| 2-1-m1E01-2-0764 | C41 H46 F3 N5 O7 | 777.33493 | -16.1 | -8.0 |
| 2-1-m1E01-2-0765 | C37 H38 N4 O9 S3 | 778.18009 | 1.7 | 2.2 |
| 2-1-m1E01-2-0766 | C38 H36 F2 N4 O8 S2 | 778.19426 | 7.4 | -55.9 |
| 2-1-m1E01-2-0767 | C40 H38 F4 N4 O6 S | 778.24482 | -37.6 | 1.8 |
| 2-1-m1E01-2-0768 | C37 H38 F N5 O9 S2 | 779.20950 | -14.3 | -11.1 |
| 2-1-m1E01-2-0769 | C38 H36 F3 N5 O8 S | 779.22367 | -12.8 | -7.2 |
| 2-1-m1E01-2-0770 | C37 H41 N5 O10 S2 | 779.22948 | -23.2 | 2.2 |
| 2-1-mlE01-2-0771 | C41 H41 N5 O7 S2 | 779.24474 | -16.7 | 0.0 |
| 2-1-mlE01-2-0772 | C39 H40 F3 N5 O7 S | 779.26005 | -22.0 | -4.3 |
| 2-1-m1E01-2-0773 | C39 H40 F3 N5 O7 S | 779.26005 | -12.3 | 7.9 |
| 2-1-mlE01-2-0774 | C40 H41 N7 O8 S | 779.27373 | -12.7 | 6.8 |
| 2-1-m1E01-2-0775 | C42 H45 N5 O8 S | 779.29888 | -3.3 | -3.5 |
| 2-1-mlE01-2-0776 | C43 H49 N5 O7 S | 779.33527 | -25.1 | 4.7 |
| 2-1-m1E01-2-0777 | C38 H35 F3 N4 O7 S2 | 780.18993 | -21.3 | 2.9 |
| 2-1-m1E01-2-0778 | C38 H35 F3 N4 O9 S | 780.20768 | -37.4 | -30.4 |
| 2-1-m1E01-2-0779 | C38 H39 F3 N6 O7 S | 780.25530 | -20.6 | -4.1 |
| 2-1-m1E01-2-0780 | C44 H40 N6 O6 S | 780.27300 | 0.0 | 0.0 |
| 2-1-m1E01-2-0781 | C41 H44 N6 O6 S2 | 780.27637 | 0.0 | 0.0 |
| 2-1-m1E01-2-0782 | C36 H39 N5 O9 S3 | 781.19099 | -28.8 | 0.8 |
| 2-1-m1E01-2-0783 | C36 H39 N5 O11 S2 | 781.20875 | -234.8 | -52.8 |
| 2-1-m1E01-2-0784 | C38 H38 F3 N5 O8 S | 781.23932 | -6.4 | -0.4 |
| 2-1-m1E01-2-0785 | C38 H38 F3 N5 O8 S | 781.23932 | -21.7 | 24.9 |
| 2-1-m1E01-2-0786 | C41 H43 N5 O7 S2 | 781.26039 | 0.0 | 0.0 |
| 2-1-m1E01-2-0787 | C41 H43 N5 O7 S2 | 781.26039 | -16.5 | -9.6 |
| 2-1-m1E01-2-0788 | C41 H43 N5 O9 S | 781.27815 | 0.0 | 0.0 |
| 2-1-m1E01-2-0789 | C40 H38 N4 O9 S2 | 782.20802 | 0.0 | 0.8 |
| 2-1-m1E01-2-0790 | C39 H38 N6 O8 S2 | 782.21925 | -14.3 | -14.2 |
| 2-1-m1E01-2-0791 | C39 H35 F5 N4 O6 S | 782.21975 | -21.2 | -131.2 |
| 2-1-m1E01-2-0792 | C37 H37 F3 N6 O8 S | 782.23457 | -14.6 | -2.6 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0793 | C40 H42 N6 O7 S2 | 782.25564 | -13.2 | -4.6 |
| 2-1-m1E01-2-0794 | C40 H42 N6 O7 S2 | 782.25564 | -19.3 | 4.2 |
| 2-1-m1E01-2-0795 | C45 H42 N4 O7 S | 782.27742 | 0.0 | 0.0 |
| 2-1-m1E01-2-0796 | C43 H44 F2 N4 O6 S | 782.29496 | 0.0 | 0.0 |
| 2-1-m1E01-2-0797 | C38 H37 N7 O8 S2 | 783.21450 | -2.5 | -8.8 |
| 2-1-m1E01-2-0798 | C37 H36 F3 N5 O9 S | 783.21858 | -21.8 | -9.6 |
| 2-1-m1E01-2-0799 | C36 H36 F3 N7 O8 S | 783.22982 | -76.8 | 60.7 |
| 2-1-m1E01-2-0800 | C38 H37 F4 N5 O7 S | 783.23498 | 5.6 | 21.5 |
| 2-1-m1E01-2-0801 | C40 H41 N5 O8 S2 | 783.23965 | 17.0 | 11.7 |
| 2-1-m1E01-2-0802 | C40 H41 N5 O8 S2 | 783.23965 | -6.9 | 11.8 |
| 2-1-m1E01-2-0803 | C42 H46 FN5 O7 S | 783.31020 | 1.1 | -39.9 |
| 2-1-m1E01-2-0804 | C36 H34 F2 N4 O8 S3 | 784.15068 | -42.4 | -3.1 |
| 2-1-m1E01-2-0805 | C44 H40 N4 O6 S2 | 784.23893 | -19.5 | -8.1 |
| 2-1-m1E01-2-0806 | C44 H40 N4 O6 S2 | 784.23893 | -16.2 | -7.1 |
| 2-1-m1E01-2-0807 | C35 H36 F N5 O9 S3 | 785.16592 | -7.9 | 8.5 |
| 2-1-m1E01-2-0808 | C40 H37 F2 N5 O6 S2 | 785.21533 | 0.0 | 0.0 |
| 2-1-m1E01-2-0809 | C39 H37 F2 N7 O7 S | 785.24432 | -53.7 | -23.9 |
| 2-1-m1E01-2-0810 | C39 H37 F6 N5 O6 | 785.26480 | -29.7 | 19.1 |
| 2-1-m1E01-2-0811 | C42 H51 N5 O8 S | 785.34583 | -23.6 | -8.0 |
| 2-1-m1E01-2-0812 | C39 H39 F N6 O7 S2 | 786.23057 | 0.0 | 0.0 |
| 2-1-m1E01-2-0813 | C39 H39 F N6 O7 S2 | 786.23057 | -27.4 | 2.9 |
| 2-1-m1E01-2-0814 | C43 H45 F3 N4 O5 S | 786.30628 | 0.0 | 0.0 |
| 2-1-m1E01-2-0815 | C35 H35 F2 N5 O10 S2 | 787.17934 | -19.7 | -15.6 |
| 2-1-m1E01-2-0816 | C36 H36 F3 N5 O8 S2 | 787.19574 | -18.2 | -12.1 |
| 2-1-m1E01-2-0817 | C40 H39 F2 N5 O8 S | 787.24874 | 0.0 | 0.0 |
| 2-1-m1E01-2-0818 | C41 H49 N5 O9 S | 787.32510 | 0.0 | 0.0 |
| 2-1-m1E01-2-0819 | C37 H36 N6 O8 S3 | 788.17567 | -17.4 | -0.4 |
| 2-1-m1E01-2-0820 | C35 H35 F3 N6 O8 S2 | 788.19099 | -31.0 | 31.6 |
| 2-1-m1E01-2-0821 | C42 H43 F3 N4 O6 S | 788.28554 | 4.1 | -20.0 |
| 2-1-m1E01-2-0822 | C45 H48 N4 O7 S | 788.32437 | -7.8 | -19.9 |
| 2-1-m1E01-2-0823 | C39 H37 F2 N5 O7 S2 | 789.21025 | -18.9 | -9.2 |
| 2-1-m1E01-2-0824 | C39 H37 F2 N5 O7 S2 | 789.21025 | -45.2 | 7.2 |
| 2-1-m1E01-2-0825 | C40 H38 F3 N5 O7 S | 789.24440 | 9.4 | -53.0 |
| 2-1-m1E01-2-0826 | C39 H43 N5 O9 S2 | 789.25022 | -34.5 | 3.0 |
| 2-1-m1E01-2-0827 | C40 H37 F3 N4 O8 S | 790.22842 | -12.3 | -38.0 |
| 2-1-m1E01-2-0828 | C41 H41 F3 N4 O7 S | 790.26480 | 0.0 | 0.0 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0829 | C41 H41 F3 N4 O7 S | 790.26480 | 0.0 | 0.0 |
| 2-1-m1E01-2-0830 | C39 H36 F3 N5 O8 S | 791.22367 | 0.0 | 0.0 |
| 2-1-m1E01-2-0831 | C38 H41 N5 O10 S2 | 791.22948 | -11.4 | -22.5 |
| 2-1-m1E01-2-0832 | C41 H44 F3 N5 O6 S | 791.29644 | 0.0 | 0.0 |
| 2-1-m1E01-2-0833 | C40 H36 F4 N4 O7 S | 792.22408 | -33.0 | -17.6 |
| 2-1-m1E01-2-0834 | C42 H40 N4 O8 S2 | 792.22876 | 0.0 | -30.0 |
| 2-1-m1E01-2-0835 | C39 H38 F3 N5 O8 S | 793.23932 | -1.3 | -3.7 |
| 2-1-m1E01-2-0836 | C40 H42 F3 N5 O7 S | 793.27570 | -25.4 | -14.1 |
| 2-1-m1E01-2-0837 | C40 H42 F3 N5 O7 S | 793.27570 | -18.7 | 0.7 |
| 2-1-m1E01-2-0838 | C40 H42 F3 N5 O7 S | 793.27570 | 0.0 | 0.0 |
| 2-1-m1E01-2-0839 | C40 H45 F2 N5 O8 S | 793.29569 | -6.0 | -0.2 |
| 2-1-m1E01-2-0840 | C44 H51 N5 O7 S | 793.35092 | -44.6 | 7.2 |
| 2-1-m1E01-2-0841 | C38 H37 F3 N6 O8 S | 794.23457 | -15.7 | -2.1 |
| 2-1-m1E01-2-0842 | C40 H38 F4 N4 O7 S | 794.23973 | -52.2 | -6.3 |
| 2-1-m1E01-2-0843 | C42 H46 N6 O6 S2 | 794.29202 | 0.0 | -10.1 |
| 2-1-m1E01-2-0844 | C37 H41 N5 O9 S3 | 795.20664 | 1.3 | 3.9 |
| 2-1-m1E01-2-0845 | C39 H40 F3 N5 O8 S | 795.25497 | -31.8 | 0.6 |
| 2-1-m1E01-2-0846 | C39 H40 F3 N5 O8 S | 795.25497 | 20.1 | -6.4 |
| 2-1-m1E01-2-0847 | C43 H49 N5 O8 S | 795.33018 | 0.0 | 0.0 |
| 2-1-m1E01-2-0848 | C38 H35 F3 N4 O8 S2 | 796.18484 | -7.3 | -16.9 |
| 2-1-m1E01-2-0849 | C41 H40 N4 O9 S2 | 796.22367 | -25.8 | -6.1 |
| 2-1-m1E01-2-0850 | C38 H39 F3 N6 O8 S | 796.25022 | -10.2 | -10.6 |
| 2-1-m1E01-2-0851 | C43 H39 F3 N4 O6 S | 796.25424 | -54.0 | 8.6 |
| 2-1-m1E01-2-0852 | C41 H44 N6 O7 S2 | 796.27129 | -8.0 | -9.3 |
| 2-1-m1E01-2-0853 | C47 H48 N4 O6 S | 796.32946 | 0.0 | 0.0 |
| 2-1-m1E01-2-0854 | C36 H39 N5 O10 S3 | 797.18590 | -106.3 | 7.9 |
| 2-1-m1E01-2-0855 | C37 H37 F2 N5 O9 S2 | 797.20008 | -29.2 | 5.2 |
| 2-1-m1E01-2-0856 | C40 H39 N5 O9 S2 | 797.21892 | -9.0 | -6.0 |
| 2-1-m1E01-2-0857 | C39 H39 N7 O8 S2 | 797.23015 | -17.1 | -5.0 |
| 2-1-m1E01-2-0858 | C39 H39 F4 N5 O7 S | 797.25063 | -3.6 | -28.3 |
| 2-1-m1E01-2-0859 | C40 H38 N4 O8 S3 | 798.18518 | -42.3 | -75.7 |
| 2-1-m1E01-2-0860 | C40 H38 N4 O8 S3 | 798.18518 | -30.9 | -61.1 |
| 2-1-m1E01-2-0861 | C41 H37 F3 N6 O6 S | 798.24474 | -10.2 | -6.0 |
| 2-1-m1E01-2-0862 | C40 H42 N6 O8 S2 | 798.25055 | -32.7 | -19.5 |
| 2-1-m1E01-2-0863 | C40 H42 N6 O8 S2 | 798.25055 | 0.0 | 0.0 |
| 2-1-m1E01-2-0864 | C39 H37 N5 O8 S3 | 799.18043 | -12.6 | -5.5 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0865 | C37 H36 F3 N5 O8 S2 | 799.19574 | -12.2 | -10.7 |
| 2-1-m1E01-2-0866 | C37 H36 F3 N5 O10 S | 799.21350 | -12.5 | -6.1 |
| 2-1-m1E01-2-0867 | C44 H41 N5 O6 S2 | 799.24983 | 0.0 | 0.0 |
| 2-1-m1E01-2-0868 | C37 H35 F3 N4 O9 S2 | 800.17975 | -117.1 | 8.0 |
| 2-1-m1E01-2-0869 | C39 H37 F N6 O8 S2 | 800.20983 | -13.3 | -2.6 |
| 2-1-m1E01-2-0870 | C42 H39 F3 N4 O7 S | 800.24915 | -24.1 | -23.9 |
| 2-1-m1E01-2-0871 | C38 H36 F5 N5 O7 S | 801.22556 | 5.3 | -11.7 |
| 2-1-m1E01-2-0872 | C42 H45 F2 N5 O7 S | 801.30078 | -60.7 | 1.5 |
| 2-1-m1E01-2-0873 | C41 H37 F3 N4 O6 S2 | 802.21066 | -19.1 | -1.1 |
| 2-1-m1E01-2-0874 | C41 H37 F3 N4 O6 S2 | 802.21066 | -64.0 | 76.4 |
| 2-1-m1E01-2-0875 | C43 H45 F3 N4 O6 S | 802.30119 | -34.3 | 0.0 |
| 2-1-m1E01-2-0876 | C35 H35 F2 N5 O9 S3 | 803.15650 | -14.7 | 1.1 |
| 2-1-m1E01-2-0877 | C39 H38 F2 N6 O7 S2 | 804.22115 | 0.0 | 0.0 |
| 2-1-m1E01-2-0878 | C40 H38 F3 N5 O8 S | 805.23932 | -6.1 | -27.3 |
| 2-1-m1E01-2-0879 | C42 H46 F3 N5 O6 S | 805.31209 | 0.0 | 0.0 |
| 2-1-m1E01-2-0880 | C40 H38 N8 O7 S2 | 806.23049 | -43.8 | -6.8 |
| 2-1-m1E01-2-0881 | C41 H41 F3 N4 O8 S | 806.25972 | 0.0 | 0.0 |
| 2-1-m1E01-2-0882 | C42 H45 F3 N4 O7 S | 806.29611 | 0.0 | -20.0 |
| 2-1-m1E01-2-0883 | C41 H44 F3 N5 O7 S | 807.29135 | -25.9 | -42.6 |
| 2-1-m1E01-2-0884 | C36 H36 N6 O10 S3 | 808.16550 | -7.7 | -9.5 |
| 2-1-m1E01-2-0885 | C40 H36 F4 N4 O8 S | 808.21900 | -29.1 | -35.8 |
| 2-1-m1E01-2-0886 | C41 H40 N6 O8 S2 | 808.23490 | 0.0 | 0.0 |
| 2-1-m1E01-2-0887 | C39 H39 F3 N6 O8 S | 808.25022 | -16.4 | -5.7 |
| 2-1-m1E01-2-0888 | C39 H38 F3 N5 O9 S | 809.23423 | -23.6 | 3.5 |
| 2-1-m1E01-2-0889 | C40 H42 F3 N5 O8 S | 809.27062 | 31.5 | 0.0 |
| 2-1-m1E01-2-0890 | C40 H42 F3 N5 O8 S | 809.27062 | 0.0 | 0.0 |
| 2-1-m1E01-2-0891 | C40 H38 N6 O7 S3 | 810.19641 | -55.9 | -50.9 |
| 2-1-m1E01-2-0892 | C40 H38 N6 O7 S3 | 810.19641 | -14.3 | -13.4 |
| 2-1-m1E01-2-0893 | C39 H37 F3 N4 O8 S2 | 810.20049 | -34.4 | 44.9 |
| 2-1-m1E01-2-0894 | C38 H37 F3 N6 O9 S | 810.22948 | -15.8 | -4.5 |
| 2-1-m1E01-2-0895 | C42 H42 N4 O9 S2 | 810.23932 | -27.6 | -1.8 |
| 2-1-m1E01-2-0896 | C39 H37 F4 N5 O8 S | 811.22990 | 1.3 | -10.8 |
| 2-1-m1E01-2-0897 | C41 H40 N4 O8 S3 | 812.20083 | 0.0 | 0.0 |
| 2-1-m1E01-2-0898 | C40 H39 N5 O8 S3 | 813.19608 | -0.6 | 0.0 |
| 2-1-m1E01-2-0899 | C39 H39 F4 N5 O8 S | 813.24555 | -138.8 | 0.1 |
| 2-1-m1E01-2-0900 | C39 H38 N6 O8 S3 | 814.19132 | -32.1 | -2.5 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0901 | C41 H39 F N4 O9 S2 | 814.21425 | 19.8 | -49.2 |
| 2-1-m1E01-2-0902 | C40 H36 F6 N4 O6 S | 814.22597 | -18.0 | -9.2 |
| 2-1-m1E01-2-0903 | C41 H37 F3 N6 O7 S | 814.23965 | 0.0 | 0.0 |
| 2-1-m1E01-2-0904 | C41 H46 N6 O8 S2 | 814.28185 | -6.1 | 4.9 |
| 2-1-m1E01-2-0905 | C44 H45 F3 N4 O6 S | 814.30119 | -12.3 | -7.8 |
| 2-1-m1E01-2-0906 | C37 H36 F3 N5 O9 S2 | 815.19065 | -15.5 | 6.6 |
| 2-1-m1E01-2-0907 | C40 H41 N5 O10 S2 | 815.22948 | -32.0 | -4.6 |
| 2-1-m1E01-2-0908 | C37 H35 F3 N4 O8 S3 | 816.15691 | 0.0 | -109.0 |
| 2-1-m1E01-2-0909 | C37 H35 F3 N4 O10 S2 | 816.17467 | -84.2 | -38.1 |
| 2-1-m1E01-2-0910 | C40 H37 F N4 O8 S3 | 816.17575 | -5.5 | 9.9 |
| 2-1-m1E01-2-0911 | C39 H40 N6 O10 S2 | 816.22473 | -12.8 | 7.0 |
| 2-1-m1E01-2-0912 | C42 H39 F3 N4 O8 S | 816.24407 | 0.0 | 0.0 |
| 2-1-m1E01-2-0913 | C39 H39 N5 O9 S3 | 817.19099 | -35.2 | 5.4 |
| 2-1-m1E01-2-0914 | C41 H38 F3 N5 O6 S2 | 817.22156 | 103.3 | -74.8 |
| 2-1-m1E01-2-0915 | C40 H38 F3 N7 O7 S | 817.25055 | -23.2 | -22.6 |
| 2-1-m1E01-2-0916 | C38 H38 N6 O9 S3 | 818.18624 | -11.0 | 1.2 |
| 2-1-m1E01-2-0917 | C38 H38 N6 O9 S3 | 818.18624 | -9.6 | 2.0 |
| 2-1-m1E01-2-0918 | C41 H37 F3 N4 O7 S2 | 818.20558 | -526.9 | -9.9 |
| 2-1-m1E01-2-0919 | C41 H37 F3 N4 O7 S2 | 818.20558 | 6.5 | -40.3 |
| 2-1-m1E01-2-0920 | C36 H36 F3 N5 O10 S2 | 819.18557 | -26.0 | -10.2 |
| 2-1-m1E01-2-0921 | C41 H40 F3 N5 O8 S | 819.25497 | 0.0 | 0.0 |
| 2-1-m1E01-2-0922 | C40 H38 F3 N5 O7 S2 | 821.21647 | 0.0 | 0.0 |
| 2-1-m1E01-2-0923 | C40 H38 F3 N5 O7 S2 | 821.21647 | 46.9 | 64.0 |
| 2-1-m1E01-2-0924 | C42 H46 F3 N5 O7 S | 821.30700 | 0.0 | 0.0 |
| 2-1-m1E01-2-0925 | C39 H37 F3 N6 O7 S2 | 822.21172 | -13.4 | -9.0 |
| 2-1-m1E01-2-0926 | C43 H46 N6 O7 S2 | 822.28694 | -66.2 | -5.6 |
| 2-1-m1E01-2-0927 | C42 H45 F3 N4 O8 S | 822.29102 | -11.6 | -1.9 |
| 2-1-m1E01-2-0928 | C36 H36 N6 O9 S4 | 824.14266 | -27.4 | -7.8 |
| 2-1-m1E01-2-0929 | C39 H39 F3 N6 O9 S | 824.24513 | -18.9 | 0.2 |
| 2-1-m1E01-2-0930 | C43 H44 N4 O9 S2 | 824.25497 | -16.7 | 0.0 |
| 2-1-m1E01-2-0931 | C40 H39 N7 O7 S3 | 825.20731 | -25.2 | 0.9 |
| 2-1-m1E01-2-0932 | C40 H42 F3 N5 O9 S | 825.26553 | -6.8 | -10.2 |
| 2-1-m1E01-2-0933 | C41 H46 F3 N5 O8 S | 825.30192 | -26.2 | 12.2 |
| 2-1-m1E01-2-0934 | C39 H37 F3 N4 O9 S2 | 826.19540 | -11.3 | -9.4 |
| 2-1-m1E01-2-0935 | C42 H42 N4 O8 S3 | 826.21648 | 0.0 | 0.0 |
| 2-1-m1E01-2-0936 | C42 H42 N4 O10 S2 | 826.23423 | 0.0 | 0.0 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0937 | C41 H41 N5 O8 S3 | 827.21173 | -39.4 | -13.3 |
| 2-1-mlE01-2-0938 | C39 H37 F4 N5 O9 S | 827.22481 | -15.2 | -12.8 |
| 2-1-m1E01-2-0939 | C41 H40 N4 O9 S3 | 828.19574 | -32.2 | 7.9 |
| 2-1-m1E01-2-0940 | C38 H38 F3 N5 O9 S2 | 829.20630 | -26.4 | -6.8 |
| 2-1-m1E01-2-0941 | C41 H43 N5 O10 S2 | 829.24513 | -8.8 | -2.6 |
| 2-1-m1E01-2-0942 | C40 H36 F6 N4 O7 S | 830.22089 | 41.5 | -8.6 |
| 2-1-m1E01-2-0943 | C44 H45 F3 N4 O7 S | 830.29611 | 0.0 | 0.0 |
| 2-1-m1E01-2-0944 | C40 H38 F N5 O8 S3 | 831.18665 | -11.3 | -4.0 |
| 2-1-m1E01-2-0945 | C40 H41 N5 O9 S3 | 831.20664 | -23.2 | -3.9 |
| 2-1-m1E01-2-0946 | C37 H35 F3 N4 O9 S3 | 832.15183 | -48.4 | -25.7 |
| 2-1-m1E01-2-0947 | C39 H40 N6 O9 S3 | 832.20189 | -3.6 | 3.4 |
| 2-1-mlE01-2-0948 | C41 H38 F2 N4 O9 S2 | 832.20483 | -2.6 | 10.3 |
| 2-1-m1E01-2-0949 | C38 H39 N7 O9 S3 | 833.19714 | -10.9 | -3.7 |
| 2-1-m1E01-2-0950 | C41 H38 F3 N5 O7 S2 | 833.21647 | 92.2 | 18.0 |
| 2-1-m1E01-2-0951 | C40 H40 F N5 O10 S2 | 833.22006 | -28.3 | 0.8 |
| 2-1-mlE01-2-0952 | C39 H37 F6 N5 O7 S | 833.23179 | -15.1 | 6.1 |
| 2-1-m1E01-2-0953 | C40 H38 F3 N7 O8 S | 833.24547 | -10.6 | -3.0 |
| 2-1-m1E01-2-0954 | C43 H46 F3 N5 O7 S | 833.30700 | 0.0 | 0.0 |
| 2-1-m1E01-2-0955 | C40 H36 F2 N4 O8 S3 | 834.16633 | 35.6 | -35.5 |
| 2-1-m1E01-2-0956 | C36 H36 F3 N5 O9 S3 | 835.16272 | -41.3 | 19.7 |
| 2-1-m1E01-2-0957 | C36 H36 F3 N5 O11 S2 | 835.18048 | -26.3 | -8.6 |
| 2-1-m1E01-2-0958 | C39 H38 F N5 O9 S3 | 835.18157 | -27.0 | 6.6 |
| 2-1-m1E01-2-0959 | C41 H40 F3 N5 O9 S | 835.24988 | -29.4 | 16.8 |
| 2-1-m1E01-2-0960 | C40 H39 F3 N6 O7 S2 | 836.22737 | -9.2 | -9.8 |
| 2-1-m1E01-2-0961 | C40 H38 F3 N5 O8 S2 | 837.21139 | -48.6 | 20.9 |
| 2-1-m1E01-2-0962 | C40 H38 F3 N5 O8 S2 | 837.21139 | -699.3 | 0.0 |
| 2-1-m1E01-2-0963 | C42 H43 N5 O8 S3 | 841.22738 | 0.0 | 0.0 |
| 2-1-m1E01-2-0964 | C41 H46 F3 N5 O9 S | 841.29683 | -23.0 | -12.8 |
| 2-1-m1E01-2-0965 | C41 H41 N5 O9 S3 | 843.20664 | -10.6 | -12.0 |
| 2-1-m1E01-2-0966 | C42 H45 N5 O10 S2 | 843.26078 | -17.3 | -7.0 |
| 2-1-m1E01-2-0967 | C38 H38 F3 N5 O10 S2 | 845.20122 | -22.9 | 2.4 |
| 2-1-m1E01-2-0968 | C41 H43 N5 O9 S3 | 845.22229 | -39.1 | 9.5 |
| 2-1-m1E01-2-0969 | C41 H43 N5 O11 S2 | 845.24005 | -49.4 | 0.0 |
| 2-1-m1E01-2-0970 | C40 H42 N6 O9 S3 | 846.21754 | -11.1 | -14.3 |
| 2-1-m1E01-2-0971 | C45 H42 N4 O9 S2 | 846.23932 | 2.3 | 14.5 |
| 2-1-m1E01-2-0972 | C40 H41 N5 O10 S3 | 847.20155 | 4.2 | 9.4 |

(continued)

| [Table 11-2-5] | | | | |
|---|---|---|---|---|
| | | | AS-MS ratio (%) | |
| ID | Molecular Formula | Exact Mass | N1 | N2 |
| 2-1-m1E01-2-0973 | C44 H40 N4 O8 S3 | 848.20083 | 10.8 | -4.9 |
| 2-1-m1E01-2-0974 | C40 H37 F2 N5 O8 S3 | 849.17723 | 0.0 | 0.0 |
| 2-1-m1E01-2-0975 | C39 H37 F6 N5 O8 S | 849.22670 | -17.2 | 29.8 |
| 2-1-m1E01-2-0976 | C43 H46 F3 N5 O8 S | 849.30192 | 0.0 | 0.0 |
| 2-1-m1E01-2-0977 | C39 H39 F N6 O9 S3 | 850.19247 | -11.3 | 3.4 |
| 2-1-m1E01-2-0978 | C36 H36 F3 N5 O10 S3 | 851.15764 | -40.7 | 11.3 |
| 2-1-m1E01-2-0979 | C40 H39 F2 N5 O10 S2 | 851.21064 | -26.4 | -2.1 |
| 2-1-m1E01-2-0980 | C40 H39 F3 N6 O8 S2 | 852.22229 | 0.0 | -17.9 |
| 2-1-mlE01-2-0981 | C39 H37 F2 N5 O9 S3 | 853.17215 | -4.8 | -7.2 |
| 2-1-m1E01-2-0982 | C41 H44 N6 O9 S3 | 860.23319 | -4.9 | -14.0 |
| 2-1-mlE01-2-0983 | C40 H42 N6 O10 S3 | 862.21245 | -20.4 | -16.8 |
| 2-1-m1E01-2-0984 | C44 H41 N5 O8 S3 | 863.21173 | 0.0 | 0.0 |
| 2-1-mlE01-2-0985 | C42 H39 F3 N4 O9 S2 | 864.21105 | -35.6 | 5.4 |
| 2-1-m1E01-2-0986 | C41 H37 F3 N4 O8 S3 | 866.17256 | -79.3 | -43.6 |
| 2-1-mlE01-2-0987 | C39 H38 F2 N6 O9 S3 | 868.18305 | 14.2 | -3.2 |
| 2-1-mlE01-2-0988 | C41 H40 N6 O10 S3 | 872.19680 | -33.2 | 9.3 |
| 2-1-m1E01-2-0989 | C40 H38 N6 O9 S4 | 874.15831 | -30.2 | -1.4 |
| 2-1-m1E01-2-0990 | C42 H39 F3 N4 O10 S2 | 880.20597 | -26.1 | -18.3 |
| 2-1-m1E01-2-0991 | C41 H38 F3 N5 O8 S3 | 881.18346 | 0.0 | 0.0 |
| 2-1-m1E01-2-0992 | C41 H37 F3 N4 O9 S3 | 882.16748 | -24.8 | -177.6 |
| 2-1-m1E01-2-0993 | C41 H40 F3 N5 O10 S2 | 883.21687 | -16.6 | 5.3 |
| 2-1-m1E01-2-0994 | C40 H38 F3 N5 O9 S3 | 885.17837 | -21.5 | 10.3 |
| 2-1-m1E01-2-0995 | C40 H39 N7 O9 S4 | 889.16921 | -23.7 | -6.1 |
| 2-1-m1E01-2-0996 | C41 H38 F3 N5 O9 S3 | 897.17837 | 63.1 | 83.5 |
| 2-1-m1E01-2-0997 | C41 H40 F3 N5 O11 S2 | 899.21178 | -7.4 | 38.5 |
| 2-1-m1E01-2-0998 | C40 H39 F3 N6 O9 S3 | 900.18927 | -5.7 | 16.0 |
| 2-1-m1E01-2-0999 | C40 H38 F3 N5 O10 S3 | 901.17329 | -9.5 | -12.6 |
| 2-1-m1E01-2-1000 | C40 H39 F3 N6 O10 S3 | 916.18419 | -38.4 | 2.5 |

[Table 595]

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0001 | C38 H38 N4 O5 | 630.28422 | -17.9 | 2.1 |
| 2-1-m1E02-2-0002 | C37 H37 N5 O5 | 631.27947 | -2.7 | -1.3 |

(continued)

| [Table 11-2-6] | | | | |
|---|---|---|---|---|
| | | | AS-MS ratio (%) | |
| ID | Molecular Formula | Exact Mass | N1 | N2 |
| 2-1-mlE02-2-0003 | C37 H37 N5 O5 | 631.27947 | 25.6 | -4.8 |
| 2-1-m1E02-2-0004 | C36 H36 N6 O5 | 632.27472 | 0.5 | -26.0 |
| 2-1-m1E02-2-0005 | C36 H36 N6 O5 | 632.27472 | -25.6 | -102.5 |
| 2-1-m1E02-2-0006 | C35 H35 N7 O5 | 633.26997 | -2.7 | -1.6 |
| 2-1-m1E02-2-0007 | C35 H35 N5 O5 S | 637.23589 | 0.0 | 0.0 |
| 2-1-m1E02-2-0008 | C34 H34 N6 O5 S | 638.23114 | -5.2 | 8.7 |
| 2-1-m1E02-2-0009 | C39 H40 N4 O5 | 644.29987 | -13.2 | -22.9 |
| 2-1-m1E02-2-0010 | C38 H39 N5 O5 | 645.29512 | 3.1 | -0.8 |
| 2-1-m1E02-2-0011 | C37 H38 N6 O5 | 646.29037 | -30.2 | 29.3 |
| 2-1-m1E02-2-0012 | C37 H36 N4 O7 | 648.25840 | -8.0 | -3.7 |
| 2-1-m1E02-2-0013 | C38 H37 F N4 O5 | 648.27480 | -19.0 | -5.0 |
| 2-1-m1E02-2-0014 | C36 H35 N5 O7 | 649.25365 | -4.7 | -2.3 |
| 2-1-m1E02-2-0015 | C37 H36 F N5 O5 | 649.27005 | -4.9 | 1.5 |
| 2-1-m1E02-2-0016 | C37 H39 N5 O6 | 649.29003 | 0.0 | -1.7 |
| 2-1-m1E02-2-0017 | C36 H35 F N6 O5 | 650.26530 | -19.5 | -1.1 |
| 2-1-m1E02-2-0018 | C36 H38 N6 O6 | 650.28528 | 0.0 | 19.4 |
| 2-1-m1E02-2-0019 | C36 H38 N6 O6 | 650.28528 | -0.3 | -12.2 |
| 2-1-m1E02-2-0020 | C36 H37 N5 O5 S | 651.25154 | -13.6 | 1.8 |
| 2-1-m1E02-2-0021 | C35 H37 N7 O6 | 651.28053 | 18.3 | -3.0 |
| 2-1-m1E02-2-0022 | C35 H37 N7 O6 | 651.28053 | -7.5 | -16.1 |
| 2-1-m1E02-2-0023 | C34 H36 N8 O6 | 652.27578 | -1.4 | 3.8 |
| 2-1-m1E02-2-0024 | C35 H34 F N5 O5 S | 655.22647 | 31.7 | 27.4 |
| 2-1-m1E02-2-0025 | C34 H36 N6 O6 S | 656.24170 | 11.3 | -1.4 |
| 2-1-m1E02-2-0026 | C33 H35 N7 O6 S | 657.23695 | -4.4 | -13.8 |
| 2-1-m1E02-2-0027 | C39 H38 N4 O6 | 658.27913 | 0.0 | 0.0 |
| 2-1-mlE02-2-0028 | C40 H42 N4 O5 | 658.31552 | -8.5 | 6.7 |
| 2-1-mlE02-2-0029 | C38 H37 N5 O6 | 659.27438 | -6.8 | -3.3 |
| 2-1-m1E02-2-0030 | C38 H37 N5 O6 | 659.27438 | 18.9 | 16.2 |
| 2-1-mlE02-2-0031 | C39 H41 N5 O5 | 659.31077 | 2.7 | -9.2 |
| 2-1-m1E02-2-0032 | C37 H36 N6 O6 | 660.26963 | -2.6 | -4.7 |
| 2-1-mlE02-2-0033 | C39 H40 N4 O6 | 660.29479 | -2.7 | -12.7 |
| 2-1-m1E02-2-0034 | C38 H40 N6 O5 | 660.30602 | -778.2 | 19.7 |
| 2-1-m1E02-2-0035 | C38 H39 N5 O6 | 661.29003 | 5.3 | -10.8 |
| 2-1-mlE02-2-0036 | C38 H38 N4 O7 | 662.27405 | 4.2 | -5.5 |
| 2-1-m1E02-2-0037 | C37 H38 N6 O6 | 662.28528 | -3.2 | -26.8 |
| 2-1-mlE02-2-0038 | C38 H41 N5 O6 | 663.30568 | -0.9 | -1.5 |
| 2-1-m1E02-2-0039 | C37 H36 N4 O6 S | 664.23556 | 2.8 | -13.7 |

(continued)

| [Table 11-2-6] | | | | |
|---|---|---|---|---|
| | | | AS-MS ratio (%) | |
| ID | Molecular Formula | Exact Mass | N1 | N2 |
| 2-1-mlE02-2-0040 | C37 H40 N6 O6 | 664.30093 | 10.4 | -3.4 |
| 2-1-m1E02-2-0041 | C36 H35 N5 O6 S | 665.23080 | -5.8 | -9.3 |
| 2-1-m1E02-2-0042 | C37 H39 N5 O5 S | 665.26719 | -16.2 | 42.5 |
| 2-1-mlE02-2-0043 | C36 H39 N7 O6 | 665.29618 | -0.8 | -3.3 |
| 2-1-m1E02-2-0044 | C37 H35 F N4 O7 | 666.24898 | -0.1 | 5.5 |
| 2-1-mlE02-2-0045 | C38 H36 F2 N4 O5 | 666.26538 | -6.5 | -30.4 |
| 2-1-m1E02-2-0046 | C36 H37 N5 O6 S | 667.24645 | 0.0 | -11.7 |
| 2-1-mlE02-2-0047 | C37 H35 F2 N5 O5 | 667.26063 | -17.0 | 19.4 |
| 2-1-mlE02-2-0048 | C36 H37 N5 O8 | 667.26421 | -5.2 | -9.9 |
| 2-1-m1E02-2-0049 | C37 H38 F N5 O6 | 667.28061 | -7.4 | -9.1 |
| 2-1-mlE02-2-0050 | C36 H34 F2 N6 O5 | 668.25587 | 0.0 | 0.0 |
| 2-1-m1E02-2-0051 | C35 H36 N6 O8 | 668.25946 | -3.7 | -9.5 |
| 2-1-mlE02-2-0052 | C36 H37 F N6 O6 | 668.27586 | -2.5 | -10.7 |
| 2-1-m1E02-2-0053 | C35 H36 F N7 O6 | 669.27111 | 4.2 | 23.4 |
| 2-1-m1E02-2-0054 | C35 H38 N6 O6 S | 670.25735 | 5.0 | -1.0 |
| 2-1-m1E02-2-0055 | C40 H40 N4 O6 | 672.29479 | -5.2 | -5.9 |
| 2-1-m1E02-2-0056 | C35 H33 F2 N5 O5 S | 673.21705 | -27.4 | -68.4 |
| 2-1-m1E02-2-0057 | C39 H39 N5 O6 | 673.29003 | 9.9 | 18.7 |
| 2-1-m1E02-2-0058 | C39 H39 N5 O6 | 673.29003 | 5.7 | -6.1 |
| 2-1-m1E02-2-0059 | C34 H35 F N6 O6 S | 674.23228 | 2.4 | -3.3 |
| 2-1-m1E02-2-0060 | C38 H38 N6 O6 | 674.28528 | -3.9 | -6.6 |
| 2-1-m1E02-2-0061 | C39 H37 F N4 O6 | 676.26971 | -11.1 | 2.8 |
| 2-1-m1E02-2-0062 | C39 H37 F N4 O6 | 676.26971 | -22.6 | 0.0 |
| 2-1-m1E02-2-0063 | C39 H40 N4 O7 | 676.28970 | 0.0 | 0.0 |
| 2-1-m1E02-2-0064 | C38 H36 F N5 O6 | 677.26496 | 0.0 | 0.0 |
| 2-1-m1E02-2-0065 | C38 H36 F N5 O6 | 677.26496 | 4.2 | -5.4 |
| 2-1-m1E02-2-0066 | C38 H39 N5 O7 | 677.28495 | -5.0 | -4.7 |
| 2-1-m1E02-2-0067 | C39 H43 N5 O6 | 677.32133 | -17.3 | -4.7 |
| 2-1-m1E02-2-0068 | C38 H38 N4 O6 S | 678.25121 | -5.3 | -2.2 |
| 2-1-m1E02-2-0069 | C38 H38 N4 O8 | 678.26896 | 8.8 | 12.2 |
| 2-1-m1E02-2-0070 | C37 H38 N6 O7 | 678.28020 | -5.3 | -7.5 |
| 2-1-m1E02-2-0071 | C37 H38 N6 O7 | 678.28020 | -4.9 | -8.1 |
| 2-1-m1E02-2-0072 | C38 H42 N6 O6 | 678.31658 | -13.8 | 2.8 |
| 2-1-m1E02-2-0073 | C36 H37 N7 O7 | 679.27545 | -5.3 | -5.1 |
| 2-1-m1E02-2-0074 | C38 H41 N5 O7 | 679.30060 | 0.0 | 0.0 |
| 2-1-mlE02-2-0075 | C37 H41 N7 O6 | 679.31183 | 0.9 | -0.1 |

(continued)

| [Table 11-2-6] | | | | |
|---|---|---|---|---|
| | | | AS-MS ratio (%) | |
| ID | Molecular Formula | Exact Mass | N1 | N2 |
| 2-1-mlE02-2-0076 | C37 H40 N6 O7 | 680.29585 | -4.7 | -8.0 |
| 2-1-m1E02-2-0077 | C42 H40 N4 O5 | 680.29987 | 11.4 | 2.8 |
| 2-1-mlE02-2-0078 | C37 H39 N5 O8 | 681.27986 | 0.8 | -1.5 |
| 2-1-m1E02-2-0079 | C36 H39 N7 O7 | 681.29110 | 9.5 | -22.5 |
| 2-1-mlE02-2-0080 | C41 H39 N5 O5 | 681.29512 | -15.5 | -30.8 |
| 2-1-m1E02-2-0081 | C37 H35 F N4 O6 S | 682.22613 | 0.2 | -0.2 |
| 2-1-m1E02-2-0082 | C40 H38 N6 O5 | 682.29037 | 1.0 | -1.6 |
| 2-1-mlE02-2-0083 | C40 H38 N6 O5 | 682.29037 | 34.6 | 413.8 |
| 2-1-m1E02-2-0084 | C36 H37 N5 O7 S | 683.24137 | -1.7 | -6.7 |
| 2-1-mlE02-2-0085 | C39 H37 N7 O5 | 683.28562 | 0.0 | 0.0 |
| 2-1-m1E02-2-0086 | C36 H36 N4 O8 S | 684.22538 | 0.0 | 0.0 |
| 2-1-mlE02-2-0087 | C35 H36 N6 O7 S | 684.23662 | -4.3 | -4.7 |
| 2-1-m1E02-2-0088 | C37 H34 F2 N4 O7 | 684.23956 | 2.4 | -0.3 |
| 2-1-m1E02-2-0089 | C36 H40 N6 O6 S | 684.27300 | -0.2 | -43.6 |
| 2-1-mlE02-2-0090 | C35 H35 N5 O8 S | 685.22063 | 0.0 | 0.0 |
| 2-1-m1E02-2-0091 | C36 H36 F N5 O8 | 685.25479 | -6.0 | -14.2 |
| 2-1-mlE02-2-0092 | C37 H37 F2 N5 O6 | 685.27119 | 1.2 | 3.0 |
| 2-1-m1E02-2-0093 | C35 H38 N6 O7 S | 686.25227 | 0.0 | 0.0 |
| 2-1-mlE02-2-0094 | C40 H38 N4 O5 S | 686.25629 | -9.0 | -13.3 |
| 2-1-mlE02-2-0095 | C36 H36 F2 N6 O6 | 686.26644 | -18.5 | 13.2 |
| 2-1-m1E02-2-0096 | C37 H42 N4 O7 S | 686.27742 | -21.0 | 0.0 |
| 2-1-mlE02-2-0097 | C41 H42 N4 O6 | 686.31044 | 0.0 | 0.0 |
| 2-1-m1E02-2-0098 | C39 H37 N5 O5 S | 687.25154 | -1.5 | -1.4 |
| 2-1-mlE02-2-0099 | C39 H37 N5 O5 S | 687.25154 | -575.6 | -31.3 |
| 2-1-m1E02-2-0100 | C35 H35 F2 N7 O6 | 687.26169 | -4.6 | 0.0 |
| 2-1-m1E02-2-0101 | C36 H41 N5 O7 S | 687.27267 | -2.7 | -6.3 |
| 2-1-m1E02-2-0102 | C40 H41 N5 O6 | 687.30568 | -6.3 | 2.6 |
| 2-1-m1E02-2-0103 | C40 H41 N5 O6 | 687.30568 | 0.0 | 0.0 |
| 2-1-m1E02-2-0104 | C40 H40 N4 O7 | 688.28970 | 0.0 | 0.0 |
| 2-1-m1E02-2-0105 | C39 H39 N5 O7 | 689.28495 | -4.9 | 23.2 |
| 2-1-m1E02-2-0106 | C40 H39 F N4 O6 | 690.28536 | -5.0 | -11.6 |
| 2-1-m1E02-2-0107 | C41 H46 N4 O6 | 690.34174 | 15.0 | -14.5 |
| 2-1-m1E02-2-0108 | C39 H38 F N5 O6 | 691.28061 | -9.7 | 6.4 |
| 2-1-m1E02-2-0109 | C39 H41 N5 O7 | 691.30060 | -3.9 | -5.4 |
| 2-1-m1E02-2-0110 | C40 H45 N5 O6 | 691.33698 | 0.0 | 0.0 |
| 2-1-m1E02-2-0111 | C34 H34 F2 N6 O6 S | 692.22286 | -4.5 | -5.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0112 | C39 H40 N4 O6 S | 692.26686 | -32.2 | -13.4 |
| 2-1-m1E02-2-0113 | C38 H40 N6 O7 | 692.29585 | -0.6 | -4.5 |
| 2-1-m1E02-2-0114 | C38 H40 N6 O7 | 692.29585 | -3.4 | -3.6 |
| 2-1-m1E02-2-0115 | C37 H39 N7 O7 | 693.29110 | -4.2 | -3.7 |
| 2-1-m1E02-2-0116 | C38 H38 N4 O7 S | 694.24612 | -3.5 | 9.6 |
| 2-1-m1E02-2-0117 | C38 H38 N4 O7 S | 694.24612 | 7.8 | -1.0 |
| 2-1-m1E02-2-0118 | C38 H38 N4 O7 S | 694.24612 | -0.6 | 0.0 |
| 2-1-m1E02-2-0119 | C39 H36 F2 N4 O6 | 694.26029 | 0.0 | 0.0 |
| 2-1-m1E02-2-0120 | C39 H36 F2 N4 O6 | 694.26029 | -5.6 | -4.9 |
| 2-1-m1E02-2-0121 | C37 H37 N5 O7 S | 695.24137 | -6.8 | -8.7 |
| 2-1-m1E02-2-0122 | C37 H37 N5 O7 S | 695.24137 | 3.3 | 11.9 |
| 2-1-m1E02-2-0123 | C37 H37 N5 O7 S | 695.24137 | -5.5 | -5.1 |
| 2-1-m1E02-2-0124 | C38 H35 F2 N5 O6 | 695.25554 | -6.6 | 10.3 |
| 2-1-m1E02-2-0125 | C38 H38 F N5 O7 | 695.27553 | -2.3 | -8.6 |
| 2-1-m1E02-2-0126 | C38 H38 F N5 O7 | 695.27553 | -6.8 | -6.9 |
| 2-1-m1E02-2-0127 | C38 H41 N5 O8 | 695.29551 | 12.0 | 2.5 |
| 2-1-m1E02-2-0128 | C36 H36 N6 O7 S | 696.23662 | -2.4 | -7.2 |
| 2-1-m1E02-2-0129 | C36 H36 N6 O7 S | 696.23662 | -3.6 | 0.2 |
| 2-1-m1E02-2-0130 | C37 H37 F N6 O7 | 696.27078 | -3.3 | -9.1 |
| 2-1-m1E02-2-0131 | C37 H37 F N6 O7 | 696.27078 | 0.0 | 0.0 |
| 2-1-m1E02-2-0132 | C41 H40 N6 O5 | 696.30602 | 0.0 | 0.0 |
| 2-1-m1E02-2-0133 | C35 H35 N7 O7 S | 697.23187 | 0.0 | 0.0 |
| 2-1-m1E02-2-0134 | C37 H39 N5 O7 S | 697.25702 | -2.4 | -2.0 |
| 2-1-m1E02-2-0135 | C37 H39 N5 O9 | 697.27478 | -0.5 | -4.3 |
| 2-1-m1E02-2-0136 | C37 H38 N4 O8 S | 698.24103 | 0.0 | 0.0 |
| 2-1-m1E02-2-0137 | C39 H37 F3 N4 O5 | 698.27160 | -37.4 | 4.8 |
| 2-1-m1E02-2-0138 | C39 H37 F3 N4 O5 | 698.27160 | -12.6 | -109.9 |
| 2-1-m1E02-2-0139 | C41 H38 N4 O7 | 698.27405 | -11.5 | -5.7 |
| 2-1-m1E02-2-0140 | C38 H36 F3 N5 O5 | 699.26685 | 16.8 | 30.0 |
| 2-1-mlE02-2-0141 | C38 H36 F3 N5 O5 | 699.26685 | -4.3 | 14.1 |
| 2-1-mlE02-2-0142 | C36 H36 N4 O7 S2 | 700.20254 | -9.9 | 8.3 |
| 2-1-m1E02-2-0143 | C37 H34 F2 N4 O6 S | 700.21671 | 4.5 | -9.2 |
| 2-1-mlE02-2-0144 | C37 H35 F3 N6 O5 | 700.26210 | -13.0 | -7.6 |
| 2-1-m1E02-2-0145 | C41 H40 N4 O5 S | 700.27194 | 14.5 | 25.4 |
| 2-1-mlE02-2-0146 | C40 H37 F N6 O5 | 700.28095 | 5.8 | -2.4 |
| 2-1-m1E02-2-0147 | C38 H44 N4 O7 S | 700.29307 | 0.0 | 0.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0148 | C35 H35 N5 O7 S2 | 701.19779 | -1.8 | 0.3 |
| 2-1-m1E02-2-0149 | C35 H35 N5 O7 S2 | 701.19779 | -32.6 | -19.8 |
| 2-1-m1E02-2-0150 | C36 H36 F N5 O7 S | 701.23195 | -1.7 | -6.9 |
| 2-1-m1E02-2-0151 | C39 H39 N7 O6 | 701.29618 | -1.8 | -3.1 |
| 2-1-m1E02-2-0152 | C41 H43 N5 O6 | 701.32133 | -6.4 | -9.9 |
| 2-1-m1E02-2-0153 | C34 H34 N6 O7 S2 | 702.19304 | 0.0 | -5.9 |
| 2-1-m1E02-2-0154 | C36 H35 F N4 O8 S | 702.21596 | -36.2 | -46.5 |
| 2-1-m1E02-2-0155 | C38 H38 N8 O6 | 702.29143 | 0.0 | 0.0 |
| 2-1-m1E02-2-0156 | C35 H37 N5 O9 S | 703.23120 | -7.0 | -7.8 |
| 2-1-m1E02-2-0157 | C36 H35 F2 N5 O8 | 703.24537 | 8.5 | -5.7 |
| 2-1-m1E02-2-0158 | C40 H41 N5 O7 | 703.30060 | -2.0 | -0.1 |
| 2-1-m1E02-2-0159 | C34 H36 N6 O9 S | 704.22645 | 4.2 | -6.9 |
| 2-1-m1E02-2-0160 | C40 H37 F N4 O5 S | 704.24687 | 0.0 | 0.0 |
| 2-1-m1E02-2-0161 | C37 H41 F N4 O7 S | 704.26800 | 0.0 | 0.0 |
| 2-1-m1E02-2-0162 | C41 H41 F N4 O6 | 704.30101 | -14.3 | 12.9 |
| 2-1-m1E02-2-0163 | C42 H48 N4 O6 | 704.35739 | -15.3 | 1.7 |
| 2-1-m1E02-2-0164 | C36 H34 F3 N5 O5 S | 705.22327 | -17.2 | -12.4 |
| 2-1-m1E02-2-0165 | C39 H39 N5 O6 S | 705.26210 | 0.0 | 0.0 |
| 2-1-m1E02-2-0166 | C36 H43 N5 O8 S | 705.28323 | -5.2 | -8.6 |
| 2-1-m1E02-2-0167 | C40 H43 N5 O7 | 705.31625 | -5.9 | 4.0 |
| 2-1-m1E02-2-0168 | C38 H38 N6 O6 S | 706.25735 | 1.3 | 3.6 |
| 2-1-m1E02-2-0169 | C38 H38 N6 O6 S | 706.25735 | 15.2 | 35.2 |
| 2-1-m1E02-2-0170 | C35 H42 N6 O8 S | 706.27848 | 0.0 | 0.0 |
| 2-1-m1E02-2-0171 | C40 H39 F N4 O7 | 706.28028 | -3.5 | -1.5 |
| 2-1-m1E02-2-0172 | C40 H42 N4 O6 S | 706.28251 | -3.5 | 0.0 |
| 2-1-m1E02-2-0173 | C39 H42 N6 O7 | 706.31150 | -5.6 | -3.1 |
| 2-1-mlE02-2-0174 | C39 H42 N6 O7 | 706.31150 | -7.2 | -15.6 |
| 2-1-m1E02-2-0175 | C37 H37 N7 O6 S | 707.25260 | 0.0 | 0.0 |
| 2-1-m1E02-2-0176 | C39 H41 N5 O6 S | 707.27775 | 0.0 | 0.0 |
| 2-1-m1E02-2-0177 | C39 H41 N5 O8 | 707.29551 | 0.0 | 0.0 |
| 2-1-m1E02-2-0178 | C36 H36 N8 O6 S | 708.24785 | 7.8 | 44.2 |
| 2-1-m1E02-2-0179 | C39 H40 N4 O7 S | 708.26177 | 0.0 | 24.1 |
| 2-1-m1E02-2-0180 | C39 H40 N4 O7 S | 708.26177 | 1.3 | -36.9 |
| 2-1-mlE02-2-0181 | C39 H40 N4 O7 S | 708.26177 | 94.2 | -113.0 |
| 2-1-m1E02-2-0182 | C38 H40 N6 O8 | 708.29076 | 18.9 | 1.6 |
| 2-1-m1E02-2-0183 | C43 H40 N4 O6 | 708.29479 | -2.9 | -1.8 |

(continued)

| [Table 11-2-6] | | | | |
|---|---|---|---|---|
| | | | AS-MS ratio (%) | |
| ID | Molecular Formula | Exact Mass | N1 | N2 |
| 2-1-mlE02-2-0184 | C41 H45 F N4 O6 | 708.33231 | 2.8 | -10.9 |
| 2-1-m1E02-2-0185 | C38 H39 N5 O7 S | 709.25702 | -3.9 | -3.8 |
| 2-1-mlE02-2-0186 | C38 H39 N5 O7 S | 709.25702 | 0.0 | -13.2 |
| 2-1-m1E02-2-0187 | C38 H39 N5 O7 S | 709.25702 | -11.8 | -3.6 |
| 2-1-m1E02-2-0188 | C39 H37 F2 N5 O6 | 709.27119 | -43.0 | -9.6 |
| 2-1-mlE02-2-0189 | C42 H39 N5 O6 | 709.29003 | -8.9 | 9.4 |
| 2-1-m1E02-2-0190 | C39 H40 F N5 O7 | 709.29118 | -8.8 | 3.7 |
| 2-1-mlE02-2-0191 | C40 H47 N5 O7 | 709.34755 | -4.7 | -3.7 |
| 2-1-m1E02-2-0192 | C37 H38 N6 O7 S | 710.25227 | 0.0 | -21.4 |
| 2-1-m1E02-2-0193 | C37 H38 N6 O7 S | 710.25227 | -2.6 | -48.7 |
| 2-1-m1E02-2-0194 | C38 H39 F N6 O7 | 710.28643 | -0.1 | -5.2 |
| 2-1-m1E02-2-0195 | C42 H42 N6 O5 | 710.32167 | 0.0 | 0.0 |
| 2-1-m1E02-2-0196 | C39 H46 N6 O7 | 710.34280 | -3.0 | -6.0 |
| 2-1-m1E02-2-0197 | C38 H41 N5 O7 S | 711.27267 | 13.3 | 11.2 |
| 2-1-m1E02-2-0198 | C37 H36 N4 O9 S | 712.22030 | 0.0 | 3.2 |
| 2-1-m1E02-2-0199 | C38 H37 F N4 O7 S | 712.23670 | -13.7 | 6.5 |
| 2-1-m1E02-2-0200 | C38 H37 F N4 O7 S | 712.23670 | -5.5 | -3.6 |
| 2-1-m1E02-2-0201 | C39 H35 F3 N4 O6 | 712.25087 | 0.0 | 0.0 |
| 2-1-m1E02-2-0202 | C38 H40 N4 O8 S | 712.25668 | 0.0 | 0.0 |
| 2-1-m1E02-2-0203 | C40 H39 F3 N4 O5 | 712.28725 | 5.9 | -124.0 |
| 2-1-m1E02-2-0204 | C41 H40 N6 O6 | 712.30093 | 5.1 | 4.0 |
| 2-1-m1E02-2-0205 | C35 H35 N7 O6 S2 | 713.20902 | 0.0 | -29.4 |
| 2-1-m1E02-2-0206 | C36 H35 N5 O9 S | 713.21555 | -3.1 | -4.0 |
| 2-1-m1E02-2-0207 | C37 H36 F N5 O7 S | 713.23195 | -3.7 | -5.7 |
| 2-1-mlE02-2-0208 | C37 H39 N5 O8 S | 713.25193 | 0.4 | -0.8 |
| 2-1-m1E02-2-0209 | C37 H39 N5 O8 S | 713.25193 | 3.2 | -6.0 |
| 2-1-m1E02-2-0210 | C37 H39 N5 O8 S | 713.25193 | 0.0 | 0.0 |
| 2-1-mlE02-2-0211 | C38 H37 F2 N5 O7 | 713.26610 | -2.1 | -10.8 |
| 2-1-m 1 E02-2-0212 | C38 H37 F2 N5 O7 | 713.26610 | -0.2 | -4.8 |
| 2-1-m1E02-2-0213 | C37 H38 N4 O7 S2 | 714.21819 | -3.5 | -5.0 |
| 2-1-m1E02-2-0214 | C36 H35 F N6 O7 S | 714.22720 | 0.0 | 5.5 |
| 2-1-m1E02-2-0215 | C37 H38 N4 O9 S | 714.23595 | -6.9 | -8.0 |
| 2-1-m1E02-2-0216 | C36 H38 N6 O8 S | 714.24718 | -2.5 | -3.6 |
| 2-1-m1E02-2-0217 | C36 H38 N6 O8 S | 714.24718 | -0.4 | 0.2 |
| 2-1-m1E02-2-0218 | C36 H38 N6 O8 S | 714.24718 | -8.0 | -7.1 |
| 2-1-m1E02-2-0219 | C41 H38 N4 O6 S | 714.25121 | 2.0 | -8.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0220 | C37 H36 F2 N6 O7 | 714.26135 | 0.0 | 0.0 |
| 2-1-m1E02-2-0221 | C39 H37 F3 N4 O6 | 714.26652 | -15.1 | -11.3 |
| 2-1-m1E02-2-0222 | C42 H42 N4 O5 S | 714.28759 | 0.0 | 0.0 |
| 2-1-m1E02-2-0223 | C39 H46 N4 O7 S | 714.30872 | -6.8 | -64.2 |
| 2-1-m1E02-2-0224 | C36 H37 N5 O7 S2 | 715.21344 | 2.5 | 5.4 |
| 2-1-m1E02-2-0225 | C35 H37 N7 O8 S | 715.24243 | -11.2 | 18.4 |
| 2-1-m1E02-2-0226 | C35 H37 N7 O8 S | 715.24243 | 0.0 | 0.0 |
| 2-1-m1E02-2-0227 | C38 H36 F3 N5 O6 | 715.26177 | -38.6 | -16.0 |
| 2-1-m1E02-2-0228 | C40 H41 N7 O6 | 715.31183 | -3.0 | -7.1 |
| 2-1-m1E02-2-0229 | C34 H36 N8 O8 S | 716.23768 | -5.4 | 0.0 |
| 2-1-m1E02-2-0230 | C38 H35 F3 N4 O7 | 716.24578 | -7.5 | -2.3 |
| 2-1-m1E02-2-0231 | C37 H35 F3 N6 O6 | 716.25702 | -11.8 | -4.4 |
| 2-1-m1E02-2-0232 | C39 H36 F4 N4 O5 | 716.26218 | -39.9 | -19.0 |
| 2-1-m1E02-2-0233 | C41 H40 N4 O6 S | 716.26686 | -20.8 | 4.9 |
| 2-1-m1E02-2-0234 | C38 H44 N4 O8 S | 716.28799 | 0.0 | 0.0 |
| 2-1-m1E02-2-0235 | C36 H39 N5 O9 S | 717.24685 | -3.8 | -4.7 |
| 2-1-m1E02-2-0236 | C38 H38 F3 N5 O6 | 717.27742 | -2.6 | -3.0 |
| 2-1-m1E02-2-0237 | C38 H38 F3 N5 O6 | 717.27742 | -14.8 | 0.0 |
| 2-1-m1E02-2-0238 | C36 H35 F N4 O7 S2 | 718.19312 | -5.4 | 7.1 |
| 2-1-m1E02-2-0239 | C40 H36 F2 N6 O5 | 718.27152 | 5.0 | 3.8 |
| 2-1-m1E02-2-0240 | C37 H37 F3 N6 O6 | 718.27267 | -5.3 | 1.9 |
| 2-1-m1E02-2-0241 | C37 H37 F3 N6 O6 | 718.27267 | 3.9 | -0.8 |
| 2-1-m1E02-2-0242 | C43 H50 N4 O6 | 718.37304 | 8.1 | 0.0 |
| 2-1-m1E02-2-0243 | C35 H34 F N5 O7 S2 | 719.18837 | -39.8 | 0.2 |
| 2-1-m1E02-2-0244 | C35 H37 N5 O8 S2 | 719.20835 | 8.8 | 28.3 |
| 2-1-m1E02-2-0245 | C36 H35 F2 N5 O7 S | 719.22253 | 8.0 | -8.3 |
| 2-1-m1E02-2-0246 | C36 H36 F3 N7 O6 | 719.26792 | 4.3 | 0.3 |
| 2-1-m1E02-2-0247 | C40 H41 N5 O6 S | 719.27775 | -4.6 | -12.2 |
| 2-1-m1E02-2-0248 | C39 H38 F N7 O6 | 719.28676 | -2.3 | -4.3 |
| 2-1-m1E02-2-0249 | C37 H45 N5 O8 S | 719.29888 | 3.3 | -6.9 |
| 2-1-m1E02-2-0250 | C34 H36 N6 O8 S2 | 720.20360 | -2.4 | -4.1 |
| 2-1-m1E02-2-0251 | C34 H36 N6 O8 S2 | 720.20360 | -4.5 | 0.0 |
| 2-1-m1E02-2-0252 | C36 H34 F2 N4 O8 S | 720.20654 | -2.4 | 0.3 |
| 2-1-m1E02-2-0253 | C41 H44 N4 O6 S | 720.29816 | 4.1 | -7.4 |
| 2-1-m1E02-2-0254 | C41 H44 N4 O6 S | 720.29816 | -3.6 | -2.0 |
| 2-1-m1E02-2-0255 | C40 H44 N6 O7 | 720.32715 | -5.2 | -2.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0256 | C42 H48 N4 O7 | 720.35230 | 10.1 | 9.4 |
| 2-1-m1E02-2-0257 | C33 H35 N7 O8 S2 | 721.19885 | 0.0 | 0.0 |
| 2-1-m1E02-2-0258 | C36 H34 F3 N5 O6 S | 721.21819 | -2.9 | -5.7 |
| 2-1-m1E02-2-0259 | C35 H36 F N5 O9 S | 721.22178 | -1.0 | 0.0 |
| 2-1-m1E02-2-0260 | C40 H43 N5 O6 S | 721.29340 | 0.0 | 0.0 |
| 2-1-m1E02-2-0261 | C40 H36 F2 N4 O5 S | 722.23745 | 0.0 | 0.0 |
| 2-1-m1E02-2-0262 | C39 H38 N4 O8 S | 722.24103 | -4.7 | -6.5 |
| 2-1-m1E02-2-0263 | C37 H40 F2 N4 O7 S | 722.25858 | 0.0 | 17.7 |
| 2-1-m1E02-2-0264 | C40 H42 N4 O7 S | 722.27742 | 0.2 | -9.0 |
| 2-1-m1E02-2-0265 | C40 H42 N4 O7 S | 722.27742 | -4.6 | -54.9 |
| 2-1-m1E02-2-0266 | C40 H42 N4 O7 S | 722.27742 | 0.0 | 0.0 |
| 2-1-m1E02-2-0267 | C40 H42 N4 O7 S | 722.27742 | -6.4 | -1.3 |
| 2-1-m1E02-2-0268 | C39 H42 N6 O8 | 722.30641 | -4.2 | 0.0 |
| 2-1-m1E02-2-0269 | C38 H37 N5 O8 S | 723.23628 | -3.2 | -7.0 |
| 2-1-m1E02-2-0270 | C38 H37 N5 O8 S | 723.23628 | -1.6 | 3.8 |
| 2-1-m1E02-2-0271 | C39 H38 F N5 O6 S | 723.25268 | 0.0 | 0.0 |
| 2-1-m1E02-2-0272 | C39 H41 N5 O7 S | 723.27267 | 0.0 | -4.1 |
| 2-1-m1E02-2-0273 | C39 H41 N5 O7 S | 723.27267 | -11.1 | -7.0 |
| 2-1-m1E02-2-0274 | C39 H41 N5 O7 S | 723.27267 | -1.4 | -6.8 |
| 2-1-m1E02-2-0275 | C36 H42 F N5 O8 S | 723.27381 | -14.0 | -9.8 |
| 2-1-m1E02-2-0276 | C43 H41 N5 O6 | 723.30568 | -3.9 | -5.9 |
| 2-1-m1E02-2-0277 | C40 H42 F N5 O7 | 723.30683 | -2.5 | -6.6 |
| 2-1-m1E02-2-0278 | C41 H49 N5 O7 | 723.36320 | 5.6 | 5.4 |
| 2-1-m1E02-2-0279 | C35 H35 F3 N6 O6 S | 724.22909 | 5.4 | -0.1 |
| 2-1-m1E02-2-0280 | C37 H36 N6 O8 S | 724.23153 | -1.8 | -2.7 |
| 2-1-m1E02-2-0281 | C37 H36 N6 O8 S | 724.23153 | -1.4 | -2.1 |
| 2-1-m1E02-2-0282 | C39 H40 N4 O8 S | 724.25668 | -0.5 | -5.2 |
| 2-1-m1E02-2-0283 | C39 H40 N4 O8 S | 724.25668 | 4.1 | -12.5 |
| 2-1-m1E02-2-0284 | C38 H40 N6 O7 S | 724.26792 | -55.7 | 0.0 |
| 2-1-m1E02-2-0285 | C40 H41 F N4 O6 S | 724.27308 | 0.0 | 0.0 |
| 2-1-m1E02-2-0286 | C38 H39 N5 O8 S | 725.25193 | 3.1 | -3.7 |
| 2-1-m1E02-2-0287 | C39 H40 F N5 O8 | 725.28609 | -9.7 | -21.8 |
| 2-1-m1E02-2-0288 | C39 H43 N5 O7 S | 725.28832 | -20.4 | 0.0 |
| 2-1-m1E02-2-0289 | C38 H38 N4 O9 S | 726.23595 | 0.0 | 0.0 |
| 2-1-m1E02-2-0290 | C37 H38 N6 O8 S | 726.24718 | -35.3 | -13.8 |
| 2-1-m1E02-2-0291 | C39 H39 F N4 O7 S | 726.25235 | 0.0 | 0.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0292 | C39 H39 F N4 O7 S | 726.25235 | 0.0 | 5.9 |
| 2-1-m1E02-2-0293 | C40 H37 F3 N4 O6 | 726.26652 | 4.3 | -13.6 |
| 2-1-m1E02-2-0294 | C38 H42 N6 O7 S | 726.28357 | 8.4 | -18.7 |
| 2-1-m1E02-2-0295 | C43 H39 F N4 O6 | 726.28536 | 0.0 | 0.0 |
| 2-1-m1E02-2-0296 | C41 H41 F3 N4 O5 | 726.30291 | -5.2 | -6.1 |
| 2-1-m1E02-2-0297 | C41 H44 F2 N4 O6 | 726.32289 | 12.7 | -5.2 |
| 2-1-m1E02-2-0298 | C38 H38 F N5 O7 S | 727.24760 | 0.0 | 0.0 |
| 2-1-m1E02-2-0299 | C38 H38 F N5 O7 S | 727.24760 | -32.2 | -30.1 |
| 2-1-m1E02-2-0300 | C39 H36 F3 N5 O6 | 727.26177 | -2.8 | -10.8 |
| 2-1-m1E02-2-0301 | C38 H41 N5 O8 S | 727.26758 | -3.2 | -7.1 |
| 2-1-m1E02-2-0302 | C38 H41 N5 O8 S | 727.26758 | -3.7 | -13.0 |
| 2-1-m1E02-2-0303 | C38 H41 N5 O8 S | 727.26758 | 2.2 | -7.0 |
| 2-1-m1E02-2-0304 | C40 H46 F N5 O7 | 727.33813 | -1.9 | -7.5 |
| 2-1-m1E02-2-0305 | C37 H36 N4 O8 S2 | 728.19746 | -12.1 | -27.4 |
| 2-1-m1E02-2-0306 | C38 H40 N4 O7 S2 | 728.23384 | 0.0 | 0.0 |
| 2-1-m1E02-2-0307 | C37 H40 N6 O8 S | 728.26283 | -2.7 | -5.4 |
| 2-1-m1E02-2-0308 | C37 H40 N6 O8 S | 728.26283 | -1.7 | -5.4 |
| 2-1-m1E02-2-0309 | C37 H40 N6 O8 S | 728.26283 | -4.1 | -6.1 |
| 2-1-m1E02-2-0310 | C38 H38 F2 N6 O7 | 728.27700 | -7.0 | -5.7 |
| 2-1-m1E02-2-0311 | C40 H39 F3 N4 O6 | 728.28217 | 0.8 | 20.8 |
| 2-1-m1E02-2-0312 | C36 H35 N5 O8 S2 | 729.19270 | -5.3 | -11.4 |
| 2-1-m1E02-2-0313 | C37 H39 N5 O7 S2 | 729.22909 | 0.0 | -16.0 |
| 2-1-m1E02-2-0314 | C36 H39 N7 O8 S | 729.25808 | 0.0 | -4.1 |
| 2-1-m1E02-2-0315 | C36 H39 N7 O8 S | 729.25808 | 0.0 | 0.0 |
| 2-1-m1E02-2-0316 | C41 H43 N7 O6 | 729.32748 | -10.8 | 0.0 |
| 2-1-m1E02-2-0317 | C37 H35 F N4 O9 S | 730.21088 | 0.0 | 0.0 |
| 2-1-m1E02-2-0318 | C37 H38 N4 O8 S2 | 730.21311 | 4.7 | -6.0 |
| 2-1-m1E02-2-0319 | C38 H36 F2 N4 O7 S | 730.22728 | -19.7 | -16.0 |
| 2-1-m1E02-2-0320 | C38 H36 F2 N4 O7 S | 730.22728 | 14.5 | 0.0 |
| 2-1-m1E02-2-0321 | C36 H37 N5 O8 S2 | 731.20835 | -7.7 | -67.4 |
| 2-1-m1E02-2-0322 | C37 H35 F2 N5 O7 S | 731.22253 | 2.0 | -13.3 |
| 2-1-m1E02-2-0323 | C36 H37 N5 O10 S | 731.22611 | 0.0 | -3.4 |
| 2-1-m1E02-2-0324 | C37 H38 F N5 O8 S | 731.24251 | -1.8 | -3.7 |
| 2-1-m1E02-2-0325 | C37 H38 F N5 O8 S | 731.24251 | -4.0 | -19.0 |
| 2-1-m1E02-2-0326 | C38 H36 F3 N5 O7 | 731.25668 | 0.0 | 0.0 |
| 2-1-m1E02-2-0327 | C37 H41 N5 O9 S | 731.26250 | 5.9 | -2.6 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0328 | C39 H40 F3 N5 O6 | 731.29307 | 4.7 | -4.2 |
| 2-1-m1E02-2-0329 | C40 H41 N7 O7 | 731.30675 | 9.3 | 3.8 |
| 2-1-m1E02-2-0330 | C36 H34 F2 N6 O7 S | 732.21777 | -21.5 | -37.7 |
| 2-1-m1E02-2-0331 | C35 H36 N6 O10 S | 732.22136 | -25.0 | -1.2 |
| 2-1-m1E02-2-0332 | C38 H35 F3 N4 O6 S | 732.22294 | -5.9 | -10.6 |
| 2-1-m1E02-2-0333 | C36 H37 F N6 O8 S | 732.23776 | -5.2 | -0.3 |
| 2-1-m1E02-2-0334 | C38 H35 F3 N4 O8 | 732.24070 | -3.8 | -6.6 |
| 2-1-m1E02-2-0335 | C44 H40 N6 O5 | 732.30602 | 22.0 | -5.4 |
| 2-1-m1E02-2-0336 | C36 H39 N5 O8 S2 | 733.22400 | 11.0 | 2.1 |
| 2-1-m1E02-2-0337 | C35 H36 F N7 O8 S | 733.23301 | 0.0 | 0.0 |
| 2-1-m1E02-2-0338 | C36 H39 N5 O10 S | 733.24176 | 0.0 | 0.0 |
| 2-1-m1E02-2-0339 | C38 H38 F3 N5 O7 | 733.27233 | -16.4 | 1.2 |
| 2-1-m1E02-2-0340 | C41 H43 N5 O6 S | 733.29340 | 0.4 | -12.2 |
| 2-1-m1E02-2-0341 | C38 H47 N5 O8 S | 733.31453 | -0.6 | -11.2 |
| 2-1-m1E02-2-0342 | C35 H38 N6 O8 S2 | 734.21925 | -2.7 | -9.3 |
| 2-1-m1E02-2-0343 | C40 H38 N4 O8 S | 734.24103 | 0.0 | -4.9 |
| 2-1-m1E02-2-0344 | C39 H38 N6 O7 S | 734.25227 | -4.0 | -4.2 |
| 2-1-m1E02-2-0345 | C39 H35 F5 N4 O5 | 734.25276 | -4.0 | -4.2 |
| 2-1-m1E02-2-0346 | C37 H37 F3 N6 O7 | 734.26758 | 3.3 | -1.7 |
| 2-1-m1E02-2-0347 | C42 H46 N4 O6 S | 734.31381 | 0.0 | 0.0 |
| 2-1-m1E02-2-0348 | C42 H46 N4 O6 S | 734.31381 | -20.6 | 0.0 |
| 2-1-m1E02-2-0349 | C42 H46 N4 O6 S | 734.31381 | 0.0 | 0.0 |
| 2-1-m1E02-2-0350 | C38 H37 N7 O7 S | 735.24752 | -2.7 | -6.4 |
| 2-1-m1E02-2-0351 | C37 H36 F3 N5 O8 | 735.25160 | 10.9 | 12.3 |
| 2-1-m1E02-2-0352 | C36 H36 F3 N7 O7 | 735.26283 | 9.5 | -1.7 |
| 2-1-mlE02-2-0353 | C38 H37 F4 N5 O6 | 735.26800 | 14.5 | -5.2 |
| 2-1-m1E02-2-0354 | C40 H41 N5 O7 S | 735.27267 | 4.5 | -3.2 |
| 2-1-m1E02-2-0355 | C37 H45 N5 O9 S | 735.29380 | -11.8 | 11.2 |
| 2-1-m1E02-2-0356 | C41 H45 N5 O6 S | 735.30905 | 0.0 | -15.5 |
| 2-1-mlE02-2-0357 | C36 H34 F2 N4 O7 S2 | 736.18370 | -40.7 | -7.9 |
| 2-1-mlE02-2-0358 | C40 H40 N4 O8 S | 736.25668 | -2.7 | -3.3 |
| 2-1-m1E02-2-0359 | C44 H40 N4 O5 S | 736.27194 | 14.6 | -4.0 |
| 2-1-mlE02-2-0360 | C41 H44 N4 O7 S | 736.29307 | -0.7 | 1.3 |
| 2-1-m1E02-2-0361 | C41 H44 N4 O7 S | 736.29307 | 3.2 | 0.0 |
| 2-1-m1E02-2-0362 | C41 H44 N4 O7 S | 736.29307 | 0.0 | 0.0 |
| 2-1-m1E02-2-0363 | C41 H44 N4 O7 S | 736.29307 | 0.0 | 0.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0364 | C35 H33 F2 N5 O7 S2 | 737.17895 | 18.0 | -5.3 |
| 2-1-m1E02-2-0365 | C35 H36 F N5 O8 S2 | 737.19893 | -5.4 | -6.5 |
| 2-1-m1E02-2-0366 | C39 H39 N5 O8 S | 737.25193 | -5.7 | -5.0 |
| 2-1-mlE02-2-0367 | C39 H39 N5 O8 S | 737.25193 | 0.0 | 0.0 |
| 2-1-m1E02-2-0368 | C39 H37 F2 N7 O6 | 737.27734 | 4.5 | -21.9 |
| 2-1-mlE02-2-0369 | C40 H43 N5 O7 S | 737.28832 | -3.5 | 8.2 |
| 2-1-m1E02-2-0370 | C42 H51 N5 O7 | 737.37885 | 6.7 | -6.8 |
| 2-1-m1E02-2-0371 | C34 H35 F N6 O8 S2 | 738.19418 | -6.8 | 2.1 |
| 2-1-mlE02-2-0372 | C38 H38 N6 O8 S | 738.24718 | -2.6 | -5.3 |
| 2-1-m1E02-2-0373 | C40 H42 N4 O8 S | 738.27234 | -2.2 | -18.7 |
| 2-1-mlE02-2-0374 | C40 H42 N4 O8 S | 738.27234 | -7.8 | -3.0 |
| 2-1-m1E02-2-0375 | C41 H43 F N4 O6 S | 738.28873 | -10.3 | 0.0 |
| 2-1-mlE02-2-0376 | C35 H35 F2 N5 O9 S | 739.21235 | 0.0 | 0.0 |
| 2-1-mlE02-2-0377 | C39 H41 N5 O8 S | 739.26758 | 0.0 | 0.5 |
| 2-1-m1E02-2-0378 | C39 H41 N5 O8 S | 739.26758 | 0.0 | 0.0 |
| 2-1-mlE02-2-0379 | C40 H45 N5 O7 S | 739.30397 | -7.4 | 0.3 |
| 2-1-m1E02-2-0380 | C40 H45 N5 O7 S | 739.30397 | -19.3 | -18.1 |
| 2-1-mlE02-2-0381 | C41 H49 N5 O8 | 739.35811 | -9.7 | -2.8 |
| 2-1-m1E02-2-0382 | C37 H36 N6 O7 S2 | 740.20869 | 4.0 | 9.6 |
| 2-1-m1E02-2-0383 | C35 H35 F3 N6 O7 S | 740.22400 | 0.0 | 0.0 |
| 2-1-m1E02-2-0384 | C39 H37 F N4 O8 S | 740.23161 | -4.3 | -9.8 |
| 2-1-m1E02-2-0385 | C39 H37 F N4 O8 S | 740.23161 | 0.0 | 0.0 |
| 2-1-m1E02-2-0386 | C39 H40 N4 O9 S | 740.25160 | 0.0 | 0.0 |
| 2-1-m1E02-2-0387 | C40 H41 F N4 O7 S | 740.26800 | 3.6 | -0.7 |
| 2-1-m1E02-2-0388 | C40 H41 F N4 O7 S | 740.26800 | 0.0 | 0.0 |
| 2-1-m1E02-2-0389 | C39 H44 N6 O7 S | 740.29922 | 0.0 | 0.0 |
| 2-1-m1E02-2-0390 | C45 H48 N4 O6 | 740.35739 | 0.0 | 0.0 |
| 2-1-m1E02-2-0391 | C38 H36 F N5 O8 S | 741.22686 | 0.0 | -7.9 |
| 2-1-m1E02-2-0392 | C38 H36 F N5 O8 S | 741.22686 | 6.9 | -3.4 |
| 2-1-m1E02-2-0393 | C39 H37 F2 N5 O6 S | 741.24326 | 0.0 | 0.0 |
| 2-1-mlE02-2-0394 | C38 H39 N5 O9 S | 741.24685 | 2.8 | -1.1 |
| 2-1-m1E02-2-0395 | C36 H41 F2 N5 O8 S | 741.26439 | 0.0 | 0.0 |
| 2-1-m1E02-2-0396 | C40 H38 F3 N5 O6 | 741.27742 | -0.7 | -5.8 |
| 2-1-m1E02-2-0397 | C39 H43 N5 O8 S | 741.28323 | 4.5 | -9.5 |
| 2-1-m1E02-2-0398 | C39 H43 N5 O8 S | 741.28323 | 2.8 | 1.3 |
| 2-1-m1E02-2-0399 | C39 H43 N5 O8 S | 741.28323 | -7.5 | -5.9 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0400 | C39 H43 N5 O8 S | 741.28323 | 11.3 | 24.3 |
| 2-1-m1E02-2-0401 | C38 H38 N4 O8 S2 | 742.21311 | 0.0 | 28.6 |
| 2-1-m1E02-2-0402 | C38 H38 N4 O10 S | 742.23086 | 0.0 | -4.0 |
| 2-1-m1E02-2-0403 | C37 H38 N6 O9 S | 742.24210 | -1.2 | -4.8 |
| 2-1-m1E02-2-0404 | C37 H38 N6 O9 S | 742.24210 | -1.3 | -1.5 |
| 2-1-m1E02-2-0405 | C40 H37 F3 N4 O7 | 742.26143 | 0.0 | 0.0 |
| 2-1-m1E02-2-0406 | C40 H40 F2 N4 O6 S | 742.26366 | -9.1 | 0.2 |
| 2-1-m1E02-2-0407 | C38 H42 N6 O8 S | 742.27848 | 0.0 | 0.0 |
| 2-1-m1E02-2-0408 | C38 H42 N6 O8 S | 742.27848 | -7.7 | -9.3 |
| 2-1-m1E02-2-0409 | C38 H42 N6 O8 S | 742.27848 | 0.0 | -9.3 |
| 2-1-m1E02-2-0410 | C36 H37 N7 O9 S | 743.23735 | -2.7 | -1.4 |
| 2-1-m1E02-2-0411 | C39 H36 F3 N5 O7 | 743.25668 | -10.8 | -2.8 |
| 2-1-m1E02-2-0412 | C38 H41 N5 O9 S | 743.26250 | -2.3 | -6.8 |
| 2-1-m1E02-2-0413 | C38 H41 N5 O9 S | 743.26250 | 21.0 | 6.9 |
| 2-1-m1E02-2-0414 | C37 H41 N7 O8 S | 743.27373 | 2.5 | 1.8 |
| 2-1-m1E02-2-0415 | C39 H42 F N5 O7 S | 743.27890 | -0.5 | 0.0 |
| 2-1-m1E02-2-0416 | C39 H38 F2 N4 O7 S | 744.24293 | 2.0 | -12.2 |
| 2-1-m1E02-2-0417 | C39 H38 F2 N4 O7 S | 744.24293 | -1.2 | 3.7 |
| 2-1-m1E02-2-0418 | C40 H36 F4 N4 O6 | 744.25710 | -2.7 | -3.2 |
| 2-1-m1E02-2-0419 | C37 H40 N6 O9 S | 744.25775 | -3.0 | -2.3 |
| 2-1-m1E02-2-0420 | C42 H40 N4 O7 S | 744.26177 | -0.3 | -17.4 |
| 2-1-m1E02-2-0421 | C42 H40 N4 O7 S | 744.26177 | 0.0 | 0.0 |
| 2-1-m1E02-2-0422 | C38 H37 F2 N5 O7 S | 745.23818 | 0.0 | 0.0 |
| 2-1-m1E02-2-0423 | C37 H39 N5 O10 S | 745.24176 | -0.7 | -3.4 |
| 2-1-m1E02-2-0424 | C36 H39 N7 O9 S | 745.25300 | 10.7 | -14.2 |
| 2-1-m1E02-2-0425 | C41 H39 N5 O7 S | 745.25702 | -4.4 | -5.0 |
| 2-1-m1E02-2-0426 | C38 H40 F N5 O8 S | 745.25816 | -2.7 | -5.1 |
| 2-1-m1E02-2-0427 | C38 H40 F N5 O8 S | 745.25816 | -0.1 | -19.4 |
| 2-1-m1E02-2-0428 | C39 H38 F3 N5 O7 | 745.27233 | 0.0 | 0.0 |
| 2-1-m1E02-2-0429 | C40 H42 F3 N5 O6 | 745.30872 | 3.4 | 1.0 |
| 2-1-m1E02-2-0430 | C40 H45 F2 N5 O7 | 745.32871 | 0.1 | -8.4 |
| 2-1-m1E02-2-0431 | C37 H35 F N4 O8 S2 | 746.18803 | -9.6 | -23.5 |
| 2-1-m1E02-2-0432 | C40 H38 N6 O7 S | 746.25227 | 2.1 | -3.4 |
| 2-1-m1E02-2-0433 | C40 H38 N6 O7 S | 746.25227 | 0.4 | -15.2 |
| 2-1-m1E02-2-0434 | C37 H39 F N6 O8 S | 746.25341 | 2.1 | -3.4 |
| 2-1-m1E02-2-0435 | C37 H39 F N6 O8 S | 746.25341 | 0.4 | -10.8 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0436 | C38 H37 F3 N6 O7 | 746.26758 | 4.5 | -1.6 |
| 2-1-m1E02-2-0437 | C36 H37 N5 O9 S2 | 747.20327 | 0.0 | 5.7 |
| 2-1-m1E02-2-0438 | C37 H41 N5 O8 S2 | 747.23965 | 0.9 | 4.6 |
| 2-1-m1E02-2-0439 | C39 H37 N7 O7 S | 747.24752 | -8.4 | 0.0 |
| 2-1-m1E02-2-0440 | C39 H40 F3 N5 O7 | 747.28798 | 11.8 | -15.0 |
| 2-1-m1E02-2-0441 | C36 H36 N4 O10 S2 | 748.18728 | -1.1 | 0.0 |
| 2-1-m1E02-2-0442 | C35 H36 N6 O9 S2 | 748.19852 | 2.7 | 0.6 |
| 2-1-m1E02-2-0443 | C37 H34 F2 N4 O9 S | 748.20146 | 5.5 | 8.4 |
| 2-1-m1E02-2-0444 | C38 H35 F3 N4 O7 S | 748.21785 | -22.3 | -6.4 |
| 2-1-m1E02-2-0445 | C36 H40 N6 O8 S2 | 748.23490 | 11.7 | -2.2 |
| 2-1-m1E02-2-0446 | C41 H40 N4 O8 S | 748.25668 | -12.1 | -13.3 |
| 2-1-mlE02-2-0447 | C43 H39 F3 N4 O5 | 748.28725 | -8.6 | -0.9 |
| 2-1-m1E02-2-0448 | C43 H48 N4 O6 S | 748.32946 | 0.0 | 0.0 |
| 2-1-m1E02-2-0449 | C43 H48 N4 O6 S | 748.32946 | 0.0 | 0.0 |
| 2-1-m1E02-2-0450 | C35 H35 N5 O10 S2 | 749.18253 | -6.4 | -0.1 |
| 2-1-m1E02-2-0451 | C36 H36 F N5 O10 S | 749.21669 | -1.0 | -2.3 |
| 2-1-mlE02-2-0452 | C36 H39 N5 O9 S2 | 749.21892 | -0.2 | -2.3 |
| 2-1-m1E02-2-0453 | C37 H37 F2 N5 O8 S | 749.23309 | -3.8 | -11.2 |
| 2-1-mlE02-2-0454 | C37 H37 F2 N5 O8 S | 749.23309 | 13.0 | 3.3 |
| 2-1-m1E02-2-0455 | C40 H39 N5 O8 S | 749.25193 | 8.1 | 20.2 |
| 2-1-mlE02-2-0456 | C39 H39 N7 O7 S | 749.26317 | -6.7 | -6.6 |
| 2-1-m1E02-2-0457 | C35 H38 N6 O9 S2 | 750.21417 | 0.0 | 0.0 |
| 2-1-m1E02-2-0458 | C40 H38 N4 O7 S2 | 750.21819 | 0.0 | 3.0 |
| 2-1-mlE02-2-0459 | C40 H38 N4 O7 S2 | 750.21819 | 0.0 | 0.0 |
| 2-1-m1E02-2-0460 | C40 H38 N4 O7 S2 | 750.21819 | 0.0 | 0.0 |
| 2-1-mlE02-2-0461 | C36 H36 F2 N6 O8 S | 750.22834 | 0.0 | -6.1 |
| 2-1-m1E02-2-0462 | C41 H42 N4 O8 S | 750.27234 | -7.5 | -6.5 |
| 2-1-mlE02-2-0463 | C41 H37 F3 N6 O5 | 750.27775 | 0.0 | 26.6 |
| 2-1-m1E02-2-0464 | C42 H46 N4 O7 S | 750.30872 | 0.0 | 0.0 |
| 2-1-m1E02-2-0465 | C42 H46 N4 O7 S | 750.30872 | 0.0 | 0.0 |
| 2-1-mlE02-2-0466 | C39 H37 N5 O7 S2 | 751.21344 | 23.5 | -9.3 |
| 2-1-m1E02-2-0467 | C39 H37 N5 O7 S2 | 751.21344 | -32.4 | -35.0 |
| 2-1-mlE02-2-0468 | C39 H37 N5 O7 S2 | 751.21344 | 12.9 | 0.0 |
| 2-1-m1E02-2-0469 | C35 H35 F2 N7 O8 S | 751.22359 | 0.0 | 0.0 |
| 2-1-mlE02-2-0470 | C37 H36 F3 N5 O7 S | 751.22875 | 8.6 | 0.0 |
| 2-1-mlE02-2-0471 | C37 H36 F3 N5 O9 | 751.24651 | 0.2 | 3.2 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0472 | C40 H41 N5 O8 S | 751.26758 | 0.0 | -13.6 |
| 2-1-mlE02-2-0473 | C40 H41 N5 O8 S | 751.26758 | -8.9 | -12.5 |
| 2-1-m1E02-2-0474 | C37 H35 F3 N4 O8 S | 752.21277 | 0.0 | 0.0 |
| 2-1-mlE02-2-0475 | C39 H37 F N6 O7 S | 752.24285 | 0.0 | 0.0 |
| 2-1-m1E02-2-0476 | C40 H40 N4 O9 S | 752.25160 | 84.5 | 84.6 |
| 2-1-m1E02-2-0477 | C41 H44 N4 O8 S | 752.28799 | 0.0 | 0.0 |
| 2-1-m1E02-2-0478 | C41 H44 N4 O8 S | 752.28799 | 14.0 | -37.2 |
| 2-1-m1E02-2-0479 | C42 H45 F N4 O6 S | 752.30438 | 0.0 | 0.0 |
| 2-1-m1E02-2-0480 | C39 H39 N5 O9 S | 753.24685 | 0.0 | -333.6 |
| 2-1-m1E02-2-0481 | C38 H36 F5 N5 O6 | 753.25857 | 0.0 | 0.0 |
| 2-1-m1E02-2-0482 | C41 H47 N5 O7 S | 753.31962 | 0.0 | 0.0 |
| 2-1-m1E02-2-0483 | C41 H47 N5 O7 S | 753.31962 | 0.0 | 0.0 |
| 2-1-m1E02-2-0484 | C41 H47 N5 O7 S | 753.31962 | 0.0 | -6.7 |
| 2-1-m1E02-2-0485 | C41 H37 F3 N4 O5 S | 754.24368 | 49.2 | -26.0 |
| 2-1-m1E02-2-0486 | C40 H39 F N4 O8 S | 754.24726 | 0.0 | 0.0 |
| 2-1-m1E02-2-0487 | C38 H41 F3 N4 O7 S | 754.26480 | -0.8 | -1.9 |
| 2-1-m1E02-2-0488 | C41 H43 F N4 O7 S | 754.28365 | 0.0 | 0.0 |
| 2-1-m1E02-2-0489 | C41 H46 N4 O8 S | 754.30364 | -5.6 | -7.7 |
| 2-1-m1E02-2-0490 | C40 H46 N6 O7 S | 754.31487 | 0.0 | 0.0 |
| 2-1-m1E02-2-0491 | C35 H35 F2 N5 O8 S2 | 755.18951 | 8.5 | -3.9 |
| 2-1-m1E02-2-0492 | C39 H38 F N5 O8 S | 755.24251 | -3.0 | -6.5 |
| 2-1-m1E02-2-0493 | C39 H41 N5 O9 S | 755.26250 | -3.8 | -6.7 |
| 2-1-m1E02-2-0494 | C40 H45 N5 O8 S | 755.29888 | -1.3 | -6.7 |
| 2-1-m1E02-2-0495 | C40 H45 N5 O8 S | 755.29888 | 4.7 | -2.1 |
| 2-1-m1E02-2-0496 | C40 H45 N5 O8 S | 755.29888 | -8.5 | 0.0 |
| 2-1-m1E02-2-0497 | C40 H45 N5 O8 S | 755.29888 | 0.0 | 0.0 |
| 2-1-m1E02-2-0498 | C34 H34 F2 N6 O8 S2 | 756.18476 | 0.0 | -6.0 |
| 2-1-m1E02-2-0499 | C39 H40 N4 O8 S2 | 756.22876 | -0.5 | -7.2 |
| 2-1-m1E02-2-0500 | C38 H40 N6 O9 S | 756.25775 | -0.8 | -2.8 |
| 2-1-m1E02-2-0501 | C38 H40 N6 O9 S | 756.25775 | 2.3 | -0.4 |
| 2-1-m1E02-2-0502 | C40 H41 F N4 O8 S | 756.26291 | -0.7 | 5.8 |
| 2-1-m1E02-2-0503 | C40 H41 F N4 O8 S | 756.26291 | 3.1 | -3.6 |
| 2-1-m1E02-2-0504 | C41 H42 F2 N4 O6 S | 756.27931 | 0.0 | 0.0 |
| 2-1-m1E02-2-0505 | C39 H44 N6 O8 S | 756.29413 | 0.9 | -1.5 |
| 2-1-m1E02-2-0506 | C44 H44 N4 O6 S | 756.29816 | -2.1 | -0.7 |
| 2-1-m1E02-2-0507 | C37 H39 N7 O9 S | 757.25300 | -3.0 | -199.8 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0508 | C40 H38 F3 N5 O7 | 757.27233 | 2.8 | -9.4 |
| 2-1-m1E02-2-0509 | C39 H43 N5 O9 S | 757.27815 | -7.7 | -14.6 |
| 2-1-m1E02-2-0510 | C39 H43 N5 O9 S | 757.27815 | 14.6 | 4.9 |
| 2-1-m1E02-2-0511 | C40 H44 F N5 O7 S | 757.29455 | 0.0 | -13.5 |
| 2-1-m1E02-2-0512 | C38 H38 N4 O9 S2 | 758.20802 | 0.0 | -16.9 |
| 2-1-m1E02-2-0513 | C38 H38 N4 O9 S2 | 758.20802 | 0.0 | 0.0 |
| 2-1-m1E02-2-0514 | C39 H36 F2 N4 O8 S | 758.22219 | 0.0 | 0.0 |
| 2-1-m1E02-2-0515 | C39 H36 F2 N4 O8 S | 758.22219 | -2.8 | -7.5 |
| 2-1-m1E02-2-0516 | C40 H40 F2 N4 O7 S | 758.25858 | -1.1 | -2.7 |
| 2-1-m1E02-2-0517 | C40 H38 N8 O6 S | 758.26350 | 0.0 | 0.0 |
| 2-1-m1E02-2-0518 | C38 H42 N6 O9 S | 758.27340 | -7.8 | 4.5 |
| 2-1-m1E02-2-0519 | C38 H42 N6 O9 S | 758.27340 | 0.0 | 0.0 |
| 2-1-m1E02-2-0520 | C43 H42 N4 O7 S | 758.27742 | 2.1 | -6.9 |
| 2-1-m1E02-2-0521 | C42 H45 F3 N4 O6 | 758.32912 | -6.0 | -27.1 |
| 2-1-m1E02-2-0522 | C37 H37 N5 O9 S2 | 759.20327 | -18.9 | -15.4 |
| 2-1-m1E02-2-0523 | C38 H35 F2 N5 O8 S | 759.21744 | 0.0 | -13.7 |
| 2-1-m1E02-2-0524 | C38 H38 F N5 O9 S | 759.23743 | -1.9 | -5.0 |
| 2-1-m1E02-2-0525 | C38 H38 F N5 O9 S | 759.23743 | 1.8 | -1.7 |
| 2-1-m1E02-2-0526 | C38 H41 N5 O10 S | 759.25741 | 0.0 | -3.7 |
| 2-1-m1E02-2-0527 | C42 H41 N5 O7 S | 759.27267 | 0.0 | 0.0 |
| 2-1-m1E02-2-0528 | C39 H42 F N5 O8 S | 759.27381 | 0.0 | 0.0 |
| 2-1-m1E02-2-0529 | C39 H42 F N5 O8 S | 759.27381 | 0.0 | 0.0 |
| 2-1-m1E02-2-0530 | C36 H36 N6 O9 S2 | 760.19852 | 2.7 | 0.0 |
| 2-1-m1E02-2-0531 | C37 H37 F N6 O9 S | 760.23268 | -2.7 | -2.0 |
| 2-1-m1E02-2-0532 | C37 H37 F N6 O9 S | 760.23268 | -3.2 | -1.3 |
| 2-1-m1E02-2-0533 | C40 H36 F4 N4 O7 | 760.25201 | 4.6 | 0.0 |
| 2-1-m1E02-2-0534 | C41 H40 N6 O7 S | 760.26792 | 0.0 | 0.0 |
| 2-1-m1E02-2-0535 | C39 H39 F3 N6 O7 | 760.28323 | -8.1 | -9.5 |
| 2-1-m1E02-2-0536 | C37 H39 N5 O9 S2 | 761.21892 | -4.0 | -4.6 |
| 2-1-m1E02-2-0537 | C37 H39 N5 O11 S | 761.23668 | -2.1 | -3.0 |
| 2-1-m1E02-2-0538 | C39 H38 F3 N5 O8 | 761.26725 | -6.8 | 2.3 |
| 2-1-m1E02-2-0539 | C39 H41 F2 N5 O7 S | 761.26948 | 0.0 | 0.0 |
| 2-1-m1E02-2-0540 | C37 H38 N4 O10 S2 | 762.20293 | 0.0 | 0.1 |
| 2-1-m1E02-2-0541 | C40 H38 N6 O6 S2 | 762.22942 | 29.0 | 0.8 |
| 2-1-m1E02-2-0542 | C39 H37 F3 N4 O7 S | 762.23350 | -1.0 | -25.8 |
| 2-1-m1E02-2-0543 | C39 H37 F3 N4 O7 S | 762.23350 | -24.8 | -13.2 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0544 | C39 H37 F3 N4 O7 S | 762.23350 | -29.0 | -37.3 |
| 2-1-m1E02-2-0545 | C39 H37 F3 N4 O7 S | 762.23350 | 0.0 | 0.0 |
| 2-1-m1E02-2-0546 | C41 H38 N4 O9 S | 762.23595 | 2.9 | 0.1 |
| 2-1-m1E02-2-0547 | C37 H42 N6 O8 S2 | 762.25055 | 0.6 | -5.8 |
| 2-1-m1E02-2-0548 | C38 H37 F3 N6 O8 | 762.26250 | -8.6 | -4.0 |
| 2-1-m1E02-2-0549 | C42 H42 N4 O8 S | 762.27234 | 0.0 | 0.0 |
| 2-1-m1E02-2-0550 | C43 H46 N4 O7 S | 762.30872 | 0.0 | 0.0 |
| 2-1-m1E02-2-0551 | C44 H50 N4 O6 S | 762.34511 | 0.0 | 0.0 |
| 2-1-m1E02-2-0552 | C38 H36 F3 N5 O7 S | 763.22875 | -2.9 | -4.8 |
| 2-1-m1E02-2-0553 | C38 H36 F3 N5 O7 S | 763.22875 | -17.1 | -1.3 |
| 2-1-m1E02-2-0554 | C38 H39 F2 N5 O8 S | 763.24874 | 0.0 | 0.0 |
| 2-1-m1E02-2-0555 | C38 H39 F2 N5 O8 S | 763.24874 | 0.0 | 0.0 |
| 2-1-m1E02-2-0556 | C39 H37 F4 N5 O7 | 763.26291 | 0.0 | 0.0 |
| 2-1-m1E02-2-0557 | C42 H45 N5 O7 S | 763.30397 | 2.3 | -3.5 |
| 2-1-m1E02-2-0558 | C36 H36 N4 O9 S3 | 764.16444 | -8.2 | -4.5 |
| 2-1-m1E02-2-0559 | C37 H34 F2 N4 O8 S2 | 764.17861 | -8.2 | 13.3 |
| 2-1-m1E02-2-0560 | C37 H35 F3 N6 O7 S | 764.22400 | -5.8 | 0.0 |
| 2-1-m1E02-2-0561 | C41 H40 N4 O7 S2 | 764.23384 | -4.5 | -14.2 |
| 2-1-m1E02-2-0562 | C41 H40 N4 O7 S2 | 764.23384 | 0.0 | 5.0 |
| 2-1-m1E02-2-0563 | C40 H37 F N6 O7 S | 764.24285 | 0.0 | 0.0 |
| 2-1-m1E02-2-0564 | C37 H38 F2 N6 O8 S | 764.24399 | 0.0 | 0.0 |
| 2-1-m1E02-2-0565 | C43 H48 N4 O7 S | 764.32437 | 0.0 | 0.0 |
| 2-1-m1E02-2-0566 | C35 H35 N5 O9 S3 | 765.15969 | -7.8 | -10.2 |
| 2-1-m1E02-2-0567 | C36 H36 F N5 O9 S2 | 765.19385 | 2.8 | -2.0 |
| 2-1-m1E02-2-0568 | C40 H39 N5 O7 S2 | 765.22909 | -7.6 | -3.4 |
| 2-1-m1E02-2-0569 | C39 H39 N7 O8 S | 765.25808 | -2.0 | -8.3 |
| 2-1-m1E02-2-0570 | C41 H43 N5 O8 S | 765.28323 | -0.4 | -4.6 |
| 2-1-m1E02-2-0571 | C36 H35 F N4 O10 S2 | 766.17786 | -5.5 | -3.8 |
| 2-1-m1E02-2-0572 | C39 H38 N6 O7 S2 | 766.22434 | 0.7 | 6.8 |
| 2-1-m1E02-2-0573 | C41 H39 F N4 O8 S | 766.24726 | -1.3 | -6.2 |
| 2-1-m1E02-2-0574 | C38 H38 N8 O8 S | 766.25333 | 0.0 | 0.0 |
| 2-1-m1E02-2-0575 | C40 H36 F6 N4 O5 | 766.25899 | -24.7 | 23.9 |
| 2-1-m1E02-2-0576 | C41 H37 F3 N6 O6 | 766.27267 | 0.0 | 0.0 |
| 2-1-m1E02-2-0577 | C42 H46 N4 O8 S | 766.30364 | 0.6 | -9.4 |
| 2-1-m1E02-2-0578 | C41 H46 N6 O7 S | 766.31487 | 2.6 | -4.7 |
| 2-1-m1E02-2-0579 | C35 H37 N5 Oil S2 | 767.19310 | -5.9 | -6.3 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0580 | C36 H35 F2 N5 O10 S | 767.20727 | 0.0 | 0.0 |
| 2-1-m1E02-2-0581 | C37 H36 F3 N5 O8 S | 767.22367 | -6.0 | 16.1 |
| 2-1-m1E02-2-0582 | C40 H41 N5 O9 S | 767.26250 | -5.1 | -7.1 |
| 2-1-m1E02-2-0583 | C40 H41 N5 O9 S | 767.26250 | 18.4 | -3.1 |
| 2-1-m1E02-2-0584 | C42 H49 N5 O7 S | 767.33527 | 0.0 | -0.4 |
| 2-1-m1E02-2-0585 | C42 H49 N5 O7 S | 767.33527 | 0.0 | 0.0 |
| 2-1-m1E02-2-0586 | C34 H36 N6 O11 S2 | 768.18835 | -1.8 | -4.6 |
| 2-1-m1E02-2-0587 | C37 H35 F3 N4 O7 S2 | 768.18993 | -2.2 | -3.1 |
| 2-1-m1E02-2-0588 | C37 H35 F3 N4 O9 S | 768.20768 | -3.2 | 0.0 |
| 2-1-m1E02-2-0589 | C40 H37 F N4 O7 S2 | 768.20877 | -2.5 | 0.0 |
| 2-1-m1E02-2-0590 | C40 H37 F N4 O7 S2 | 768.20877 | 11.0 | -35.5 |
| 2-1-m1E02-2-0591 | C39 H40 N6 O9 S | 768.25775 | -3.4 | -6.8 |
| 2-1-m1E02-2-0592 | C41 H41 F N4 O8 S | 768.26291 | 120.1 | 120.5 |
| 2-1-m1E02-2-0593 | C41 H44 N4 O9 S | 768.28290 | 0.0 | 0.0 |
| 2-1-m1E02-2-0594 | C42 H48 N4 O8 S | 768.31929 | -14.8 | -8.0 |
| 2-1-m1E02-2-0595 | C36 H34 F3 N5 O7 S2 | 769.18517 | 0.0 | 0.0 |
| 2-1-m1E02-2-0596 | C39 H39 N5 O8 S2 | 769.22400 | 4.8 | 5.0 |
| 2-1-m1E02-2-0597 | C39 H39 N5 O8 S2 | 769.22400 | -23.7 | 3.6 |
| 2-1-m1E02-2-0598 | C40 H43 N5 O9 S | 769.27815 | -2.7 | -2.0 |
| 2-1-m1E02-2-0599 | C40 H38 F3 N7 O6 | 769.28357 | -254.2 | 0.0 |
| 2-1-m1E02-2-0600 | C41 H47 N5 O8 S | 769.31453 | 0.0 | -8.9 |
| 2-1-m1E02-2-0601 | C41 H47 N5 O8 S | 769.31453 | -45.3 | -67.5 |
| 2-1-m1E02-2-0602 | C38 H38 N6 O8 S2 | 770.21925 | -3.3 | -5.3 |
| 2-1-m1E02-2-0603 | C38 H38 N6 O8 S2 | 770.21925 | -7.1 | -5.2 |
| 2-1-m1E02-2-0604 | C38 H38 N6 O8 S2 | 770.21925 | 13.9 | -4.1 |
| 2-1-m1E02-2-0605 | C38 H38 N6 O8 S2 | 770.21925 | 0.0 | -13.4 |
| 2-1-m1E02-2-0606 | C41 H37 F3 N4 O6 S | 770.23859 | 0.0 | 6.2 |
| 2-1-m1E02-2-0607 | C40 H39 F N4 O9 S | 770.24218 | -2.6 | 3.5 |
| 2-1-m1E02-2-0608 | C38 H41 F3 N4 O8 S | 770.25972 | -25.6 | 6.0 |
| 2-1-m1E02-2-0609 | C39 H42 N6 O9 S | 770.27340 | -3.4 | 1.4 |
| 2-1-m1E02-2-0610 | C39 H42 N6 O9 S | 770.27340 | -2.6 | 0.9 |
| 2-1-m1E02-2-0611 | C42 H44 F2 N4 O6 S | 770.29496 | -24.9 | -11.0 |
| 2-1-m1E02-2-0612 | C45 H46 N4 O6 S | 770.31381 | -3.8 | -4.7 |
| 2-1-m1E02-2-0613 | C37 H37 N7 O8 S2 | 771.21450 | -5.4 | -4.4 |
| 2-1-m1E02-2-0614 | C36 H36 F3 N5 O9 S | 771.21858 | -7.2 | -7.6 |
| 2-1-m1E02-2-0615 | C39 H41 N5 O10 S | 771.25741 | 0.0 | 0.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0616 | C40 H45 N5 O9 S | 771.29380 | 3.0 | -8.1 |
| 2-1-m1E02-2-0617 | C40 H45 N5 O9 S | 771.29380 | 0.0 | -18.8 |
| 2-1-m1E02-2-0618 | C41 H46 F N5 O7 S | 771.31020 | 0.0 | 0.0 |
| 2-1-m1E02-2-0619 | C36 H36 N8 O8 S2 | 772.20975 | -5.6 | -3.4 |
| 2-1-m1E02-2-0620 | C39 H40 N4 O9 S2 | 772.22367 | -0.2 | -12.4 |
| 2-1-m1E02-2-0621 | C38 H40 N6 O10 S | 772.25266 | -9.6 | -38.3 |
| 2-1-m1E02-2-0622 | C43 H40 N4 O8 S | 772.25668 | 2.4 | -15.9 |
| 2-1-m1E02-2-0623 | C44 H44 N4 O7 S | 772.29307 | 15.2 | 0.0 |
| 2-1-m1E02-2-0624 | C41 H45 FN4 O8 S | 772.29421 | 15.2 | 0.0 |
| 2-1-m1E02-2-0625 | C39 H37 F2 N5 O8 S | 773.23309 | -5.4 | 1.5 |
| 2-1-m1E02-2-0626 | C40 H38 F3 N5 O6 S | 773.24949 | -6.8 | -8.3 |
| 2-1-m1E02-2-0627 | C42 H39 N5 O8 S | 773.25193 | -3.2 | -5.3 |
| 2-1-m1E02-2-0628 | C39 H40 F N5 O9 S | 773.25308 | -2.9 | -6.2 |
| 2-1-m1E02-2-0629 | C37 H42 F3 N5 O8 S | 773.27062 | 0.0 | 12.3 |
| 2-1-m1E02-2-0630 | C40 H44 F N5 O8 S | 773.28946 | 8.5 | -10.6 |
| 2-1-m1E02-2-0631 | C40 H47 N5 O9 S | 773.30945 | 0.6 | -0.2 |
| 2-1-m1E02-2-0632 | C39 H37 F3 N6 O6 S | 774.24474 | -21.0 | 14.7 |
| 2-1-m1E02-2-0633 | C38 H39 F N6 O9 S | 774.24833 | -5.2 | -2.4 |
| 2-1-m1E02-2-0634 | C40 H40 F2 N4 O8 S | 774.25349 | 0.0 | 0.0 |
| 2-1-m1E02-2-0635 | C41 H41 F3 N4 O6 S | 774.26989 | -3.1 | -8.6 |
| 2-1-m1E02-2-0636 | C42 H42 N6 O7 S | 774.28357 | -19.3 | 4.0 |
| 2-1-m1E02-2-0637 | C39 H46 N6 O9 S | 774.30470 | 1.2 | 0.8 |
| 2-1-m1E02-2-0638 | C42 H45 F3 N4 O7 | 774.32403 | -9.6 | -32.2 |
| 2-1-m1E02-2-0639 | C38 H41 N5 O9 S2 | 775.23457 | -0.7 | -10.2 |
| 2-1-m1E02-2-0640 | C39 H42 F N5 O9 S | 775.26873 | -33.5 | 19.6 |
| 2-1-m1E02-2-0641 | C39 H42 F N5 O9 S | 775.26873 | 0.0 | 0.0 |
| 2-1-mlE02-2-0642 | C40 H43 F2 N5 O7 S | 775.28513 | 0.0 | 0.0 |
| 2-1-m1E02-2-0643 | C36 H36 N6 O8 S3 | 776.17567 | 10.4 | 4.9 |
| 2-1-mlE02-2-0644 | C38 H37 F N4 O9 S2 | 776.19860 | 0.0 | 0.0 |
| 2-1-m1E02-2-0645 | C39 H35 F3 N4 O8 S | 776.21277 | 0.0 | 0.0 |
| 2-1-m1E02-2-0646 | C38 H40 N4 O10 S2 | 776.21858 | 0.0 | 0.0 |
| 2-1-m1E02-2-0647 | C40 H39 F3 N4 O7 S | 776.24915 | 0.0 | 0.0 |
| 2-1-m1E02-2-0648 | C40 H39 F3 N4 O7 S | 776.24915 | -1.1 | -6.1 |
| 2-1-m1E02-2-0649 | C41 H40 N6 O8 S | 776.26283 | -19.7 | 0.0 |
| 2-1-m1E02-2-0650 | C43 H41 F N4 O7 S | 776.26800 | 0.0 | 0.0 |
| 2-1-m1E02-2-0651 | C39 H39 F3 N6 O8 | 776.27815 | 2.4 | -8.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0652 | C43 H44 N4 O8 S | 776.28799 | 0.0 | 0.0 |
| 2-1-m1E02-2-0653 | C44 H48 N4 O7 S | 776.32437 | 11.7 | -12.9 |
| 2-1-m1E02-2-0654 | C35 H35 N7 O8 S3 | 777.17092 | -1.7 | -0.4 |
| 2-1-m1E02-2-0655 | C37 H39 N5 O10 S2 | 777.21383 | -9.7 | -6.8 |
| 2-1-mlE02-2-0656 | C37 H39 N5 O10 S2 | 777.21383 | 0.0 | 9.5 |
| 2-1-m1E02-2-0657 | C38 H37 F2 N5 O9 S | 777.22800 | -5.1 | -5.4 |
| 2-1-mlE02-2-0658 | C38 H37 F2 N5 O9 S | 777.22800 | -2.8 | 1.4 |
| 2-1-mlE02-2-0659 | C39 H38 F3 N5 O7 S | 777.24440 | 0.0 | 0.0 |
| 2-1-m1E02-2-0660 | C39 H41 F2 N5 O8 S | 777.26439 | 0.0 | 0.0 |
| 2-1-mlE02-2-0661 | C41 H46 F3 N5 O7 | 777.33493 | 4.6 | -3.3 |
| 2-1-m1E02-2-0662 | C37 H38 N4 O9 S3 | 778.18009 | -6.5 | 5.9 |
| 2-1-m1E02-2-0663 | C37 H38 N4 O11 S2 | 778.19785 | -7.2 | -5.4 |
| 2-1-m1E02-2-0664 | C36 H38 N6 O10 S2 | 778.20908 | -3.2 | -5.6 |
| 2-1-m1E02-2-0665 | C41 H38 N4 O8 S2 | 778.21311 | -4.3 | -5.0 |
| 2-1-m1E02-2-0666 | C37 H36 F2 N6 O9 S | 778.22325 | 0.0 | 0.0 |
| 2-1-m1E02-2-0667 | C39 H37 F3 N4 O8 S | 778.22842 | 5.6 | -3.2 |
| 2-1-m1E02-2-0668 | C39 H37 F3 N4 O8 S | 778.22842 | 0.0 | 0.0 |
| 2-1-m1E02-2-0669 | C42 H42 N4 O7 S2 | 778.24949 | 0.0 | 0.0 |
| 2-1-m1E02-2-0670 | C42 H42 N4 O7 S2 | 778.24949 | 0.0 | 0.0 |
| 2-1-m1E02-2-0671 | C42 H42 N4 O9 S | 778.26725 | -11.6 | -9.2 |
| 2-1-m1E02-2-0672 | C38 H36 F3 N5 O8 S | 779.22367 | -1.0 | -11.6 |
| 2-1-m1E02-2-0673 | C41 H41 N5 O7 S2 | 779.24474 | 3.3 | -11.8 |
| 2-1-m1E02-2-0674 | C39 H37 F4 N5 O8 | 779.25783 | 0.0 | -9.8 |
| 2-1-m1E02-2-0675 | C40 H41 N7 O8 S | 779.27373 | 0.0 | -3.2 |
| 2-1-m1E02-2-0676 | C38 H35 F3 N4 O9 S | 780.20768 | -9.0 | -11.1 |
| 2-1-m1E02-2-0677 | C37 H35 F3 N6 O8 S | 780.21892 | -13.9 | -731.5 |
| 2-1-m1E02-2-0678 | C39 H36 F4 N4 O7 S | 780.22408 | 5.2 | -8.6 |
| 2-1-m1E02-2-0679 | C41 H40 N4 O8 S2 | 780.22876 | 9.2 | -1.5 |
| 2-1-m1E02-2-0680 | C41 H40 N4 O8 S2 | 780.22876 | -2.7 | 14.2 |
| 2-1-m1E02-2-0681 | C43 H45 F N4 O7 S | 780.29930 | 0.0 | 0.0 |
| 2-1-m1E02-2-0682 | C36 H39 N5 O11 S2 | 781.20875 | 0.0 | 0.0 |
| 2-1-m1E02-2-0683 | C38 H38 F3 N5 O8 S | 781.23932 | 2.5 | -1.6 |
| 2-1-m1E02-2-0684 | C38 H38 F3 N5 O8 S | 781.23932 | -19.7 | -31.0 |
| 2-1-m1E02-2-0685 | C38 H38 F3 N5 O8 S | 781.23932 | 0.0 | 0.0 |
| 2-1-mlE02-2-0686 | C38 H38 F3 N5 O8 S | 781.23932 | 0.0 | 0.0 |
| 2-1-mlE02-2-0687 | C41 H43 N5 O9 S | 781.27815 | 13.9 | -8.6 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0688 | C42 H47 N5 O8 S | 781.31453 | -5.4 | 6.5 |
| 2-1-mlE02-2-0689 | C43 H51 N5 O7 S | 781.35092 | 0.0 | 0.0 |
| 2-1-m1E02-2-0690 | C36 H35 F N4 O9 S3 | 782.15502 | -5.9 | 0.0 |
| 2-1-mlE02-2-0691 | C40 H36 F2 N6 O7 S | 782.23342 | -5.8 | -14.2 |
| 2-1-m1E02-2-0692 | C37 H37 F3 N6 O8 S | 782.23457 | 6.6 | 0.0 |
| 2-1-m1E02-2-0693 | C37 H37 F3 N6 O8 S | 782.23457 | -0.3 | -2.6 |
| 2-1-m1E02-2-0694 | C40 H36 F6 N4 O6 | 782.25390 | -44.3 | -4.8 |
| 2-1-m1E02-2-0695 | C40 H42 N6 O7 S2 | 782.25564 | -39.1 | -8.0 |
| 2-1-m1E02-2-0696 | C41 H46 N6 O8 S | 782.30978 | -11.8 | 3.0 |
| 2-1-m1E02-2-0697 | C43 H50 N4 O8 S | 782.33494 | -1.0 | -15.9 |
| 2-1-m1E02-2-0698 | C35 H37 N5 O10 S3 | 783.17025 | -5.7 | -8.7 |
| 2-1-m1E02-2-0699 | C36 H35 F2 N5 O9 S2 | 783.18443 | -2.4 | -2.7 |
| 2-1-m1E02-2-0700 | C40 H38 F N5 O7 S2 | 783.21967 | 4.1 | -0.2 |
| 2-1-m1E02-2-0701 | C36 H36 F3 N7 O8 S | 783.22982 | -7.3 | -11.7 |
| 2-1-m1E02-2-0702 | C40 H41 N5 O8 S2 | 783.23965 | 0.0 | 0.0 |
| 2-1-m1E02-2-0703 | C40 H41 N5 O8 S2 | 783.23965 | 0.0 | 0.0 |
| 2-1-m1E02-2-0704 | C39 H38 F N7 O8 S | 783.24866 | -2.4 | -6.7 |
| 2-1-m1E02-2-0705 | C42 H49 N5 O8 S | 783.33018 | 0.0 | 5.6 |
| 2-1-m1E02-2-0706 | C34 H36 N6 O10 S3 | 784.16550 | -4.3 | -10.0 |
| 2-1-m1E02-2-0707 | C36 H34 F2 N4 O10 S2 | 784.16844 | -4.6 | -8.0 |
| 2-1-mlE02-2-0708 | C37 H35 F3 N4 O8 S2 | 784.18484 | -28.4 | 8.2 |
| 2-1-m1E02-2-0709 | C39 H40 N6 O8 S2 | 784.23490 | -4.2 | 1.5 |
| 2-1-m1E02-2-0710 | C39 H40 N6 O8 S2 | 784.23490 | -0.7 | 10.1 |
| 2-1-m1E02-2-0711 | C41 H38 F2 N4 O8 S | 784.23784 | -8.7 | 1.6 |
| 2-1-m1E02-2-0712 | C40 H44 N6 O9 S | 784.28905 | -3.5 | -3.0 |
| 2-1-m1E02-2-0713 | C42 H48 N4 O9 S | 784.31420 | -6.8 | -13.5 |
| 2-1-m1E02-2-0714 | C46 H48 N4 O6 S | 784.32946 | 0.0 | 0.0 |
| 2-1-m1E02-2-0715 | C36 H34 F3 N5 O8 S2 | 785.18009 | -54.2 | 5.6 |
| 2-1-m1E02-2-0716 | C35 H36 F N5 O11 S2 | 785.18368 | 0.0 | -5.3 |
| 2-1-m1E02-2-0717 | C38 H39 N7 O8 S2 | 785.23015 | -4.0 | -5.4 |
| 2-1-m1E02-2-0718 | C38 H39 N7 O8 S2 | 785.23015 | 0.0 | 0.0 |
| 2-1-m1E02-2-0719 | C40 H40 F N5 O9 S | 785.25308 | 1.3 | 8.6 |
| 2-1-m1E02-2-0720 | C39 H37 F6 N5 O6 | 785.26480 | -53.2 | 66.4 |
| 2-1-m1E02-2-0721 | C40 H38 F3 N7 O7 | 785.27848 | 50.9 | 14.2 |
| 2-1-m1E02-2-0722 | C41 H47 N5 O9 S | 785.30945 | -0.3 | -3.2 |
| 2-1-m1E02-2-0723 | C40 H36 F2 N4 O7 S2 | 786.19935 | 0.0 | -23.7 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0724 | C40 H36 F2 N4 O7 S2 | 786.19935 | -20.8 | -4.8 |
| 2-1-m1E02-2-0725 | C40 H42 N4 O9 S2 | 786.23932 | -10.4 | 0.9 |
| 2-1-m1E02-2-0726 | C39 H42 N6 O10 S | 786.26831 | 0.0 | 0.0 |
| 2-1-m1E02-2-0727 | C36 H36 F3 N5 O8 S2 | 787.19574 | -1.8 | -7.1 |
| 2-1-m1E02-2-0728 | C36 H36 F3 N5 O10 S | 787.21350 | 0.3 | -0.7 |
| 2-1-m1E02-2-0729 | C39 H38 F N5 O8 S2 | 787.21458 | 0.3 | -0.7 |
| 2-1-m1E02-2-0730 | C39 H38 F N5 O8 S2 | 787.21458 | 7.4 | 54.4 |
| 2-1-m1E02-2-0731 | C43 H41 N5 O8 S | 787.26758 | -0.3 | -3.5 |
| 2-1-m1E02-2-0732 | C40 H42 F N5 O9 S | 787.26873 | 0.1 | -7.1 |
| 2-1-m1E02-2-0733 | C40 H45 N5 O10 S | 787.28871 | 0.0 | 0.0 |
| 2-1-m1E02-2-0734 | C41 H49 N5 O9 S | 787.32510 | -2.9 | 2.3 |
| 2-1-m1E02-2-0735 | C35 H35 F3 N6 O8 S2 | 788.19099 | -7.1 | -0.4 |
| 2-1-m1E02-2-0736 | C37 H36 N6 O10 S2 | 788.19343 | -5.3 | -6.4 |
| 2-1-m1E02-2-0737 | C39 H40 N4 O10 S2 | 788.21858 | -5.6 | 2.5 |
| 2-1-m1E02-2-0738 | C42 H43 F3 N4 O6 S | 788.28554 | 12.5 | 0.0 |
| 2-1-m1E02-2-0739 | C44 H44 N4 O8 S | 788.28799 | 12.5 | 0.0 |
| 2-1-m1E02-2-0740 | C40 H38 F3 N5 O7 S | 789.24440 | -10.5 | -9.9 |
| 2-1-m1E02-2-0741 | C39 H40 F N5 O10 S | 789.24799 | 0.0 | 0.0 |
| 2-1-m1E02-2-0742 | C37 H42 F3 N5 O9 S | 789.26553 | 0.0 | 0.0 |
| 2-1-m1E02-2-0743 | C41 H45 F2 N5 O7 S | 789.30078 | -1.0 | -4.9 |
| 2-1-m1E02-2-0744 | C40 H37 F3 N4 O8 S | 790.22842 | -3.5 | -5.7 |
| 2-1-m1E02-2-0745 | C43 H39 FN4 O8 S | 790.24726 | -1.1 | -8.8 |
| 2-1-m1E02-2-0746 | C41 H41 F3 N4 O7 S | 790.26480 | 28.7 | 89.1 |
| 2-1-m1E02-2-0747 | C41 H41 F3 N4 O7 S | 790.26480 | -11.5 | -73.7 |
| 2-1-m1E02-2-0748 | C41 H41 F3 N4 O7 S | 790.26480 | 0.0 | 35.4 |
| 2-1-m1E02-2-0749 | C41 H44 F2 N4 O8 S | 790.28479 | 0.0 | 0.0 |
| 2-1-m1E02-2-0750 | C45 H50 N4 O7 S | 790.34002 | 0.0 | 0.0 |
| 2-1-m1E02-2-0751 | C39 H36 F3 N5 O8 S | 791.22367 | 0.0 | -14.8 |
| 2-1-m1E02-2-0752 | C38 H41 N5 O10 S2 | 791.22948 | 5.4 | -0.9 |
| 2-1-m1E02-2-0753 | C40 H46 F N5 O9 S | 791.30003 | -0.3 | -2.6 |
| 2-1-m1E02-2-0754 | C38 H40 N4 O9 S3 | 792.19574 | -14.1 | -9.8 |
| 2-1-m1E02-2-0755 | C38 H38 F2 N6 O9 S | 792.23890 | -3.9 | -4.9 |
| 2-1-m1E02-2-0756 | C40 H39 F3 N4 O8 S | 792.24407 | 0.0 | 0.0 |
| 2-1-m1E02-2-0757 | C40 H39 F3 N4 O8 S | 792.24407 | 2.7 | -70.2 |
| 2-1-m1E02-2-0758 | C44 H48 N4 O8 S | 792.31929 | 0.0 | 0.0 |
| 2-1-m1E02-2-0759 | C39 H38 F3 N5 O8 S | 793.23932 | 0.0 | -9.6 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0760 | C39 H41 F2 N5 O9 S | 793.25931 | 53.9 | 79.8 |
| 2-1-m1E02-2-0761 | C42 H43 N5 O7 S2 | 793.26039 | 0.0 | -2.8 |
| 2-1-m1E02-2-0762 | C40 H42 F3 N5 O7 S | 793.27570 | 0.0 | -19.1 |
| 2-1-m1E02-2-0763 | C41 H43 N7 O8 S | 793.28938 | -3.8 | -10.3 |
| 2-1-m1E02-2-0764 | C41 H46 F3 N5 O8 | 793.32985 | 0.2 | 0.5 |
| 2-1-m1E02-2-0765 | C37 H38 N4 O10 S3 | 794.17501 | -10.3 | -5.0 |
| 2-1-m1E02-2-0766 | C38 H36 F2 N4 O9 S2 | 794.18918 | 15.0 | -19.8 |
| 2-1-m1E02-2-0767 | C40 H38 F4 N4 O7 S | 794.23973 | 0.0 | 0.0 |
| 2-1-m1E02-2-0768 | C37 H38 F N5 O10 S2 | 795.20441 | -3.1 | -3.0 |
| 2-1-m1E02-2-0769 | C38 H36 F3 N5 O9 S | 795.21858 | -1.0 | -3.6 |
| 2-1-m1E02-2-0770 | C37 H41 N5 O11 S2 | 795.22440 | 0.0 | -11.6 |
| 2-1-m1E02-2-0771 | C41 H41 N5 O8 S2 | 795.23965 | -13.5 | 0.2 |
| 2-1-m1E02-2-0772 | C39 H40 F3 N5 O8 S | 795.25497 | 0.0 | -6.9 |
| 2-1-m1E02-2-0773 | C39 H40 F3 N5 O8 S | 795.25497 | -5.3 | -5.1 |
| 2-1-m1E02-2-0774 | C40 H41 N7 O9 S | 795.26865 | 0.0 | 0.0 |
| 2-1-m1E02-2-0775 | C42 H45 N5 O9 S | 795.29380 | 0.1 | 3.2 |
| 2-1-mlE02-2-0776 | C43 H49 N5 O8 S | 795.33018 | 0.0 | 0.0 |
| 2-1-m1E02-2-0777 | C38 H35 F3 N4 O8 S2 | 796.18484 | 3.3 | -31.2 |
| 2-1-m1E02-2-0778 | C38 H35 F3 N4 O10 S | 796.20260 | 12.0 | -3.4 |
| 2-1-m1E02-2-0779 | C38 H39 F3 N6 O8 S | 796.25022 | 0.0 | -10.8 |
| 2-1-m1E02-2-0780 | C44 H40 N6 O7 S | 796.26792 | 0.0 | 0.0 |
| 2-1-m1E02-2-0781 | C41 H44 N6 O7 S2 | 796.27129 | 0.0 | 0.0 |
| 2-1-m1E02-2-0782 | C36 H39 N5 O10 S3 | 797.18590 | -6.4 | -16.1 |
| 2-1-m1E02-2-0783 | C36 H39 N5 O12 S2 | 797.20366 | -9.5 | -12.6 |
| 2-1-m1E02-2-0784 | C38 H38 F3 N5 O9 S | 797.23423 | 0.0 | 0.0 |
| 2-1-m1E02-2-0785 | C38 H38 F3 N5 O9 S | 797.23423 | 4.5 | -0.5 |
| 2-1-m1E02-2-0786 | C41 H43 N5 O8 S2 | 797.25530 | 2.9 | 12.1 |
| 2-1-m1E02-2-0787 | C41 H43 N5 O8 S2 | 797.25530 | 0.0 | 0.0 |
| 2-1-m1E02-2-0788 | C41 H43 N5 O10 S | 797.27306 | 1.4 | -12.4 |
| 2-1-m1E02-2-0789 | C40 H38 N4 O10 S2 | 798.20293 | 0.0 | 0.0 |
| 2-1-m1E02-2-0790 | C39 H38 N6 O9 S2 | 798.21417 | -6.6 | -8.7 |
| 2-1-m1E02-2-0791 | C39 H35 F5 N4 O7 S | 798.21466 | -6.1 | -8.1 |
| 2-1-m1E02-2-0792 | C37 H37 F3 N6 O9 S | 798.22948 | -7.5 | -13.7 |
| 2-1-m1E02-2-0793 | C40 H42 N6 O8 S2 | 798.25055 | -9.5 | -5.8 |
| 2-1-m1E02-2-0794 | C40 H42 N6 O8 S2 | 798.25055 | 5.8 | 20.2 |
| 2-1-m1E02-2-0795 | C45 H42 N4 O8 S | 798.27234 | 0.0 | 0.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0796 | C43 H44 F2 N4 O7 S | 798.28988 | 0.0 | 0.0 |
| 2-1-mlE02-2-0797 | C38 H37 N7 O9 S2 | 799.20942 | -1.4 | -1.8 |
| 2-1-m1E02-2-0798 | C37 H36 F3 N5 O10 S | 799.21350 | -2.1 | 1.6 |
| 2-1-mlE02-2-0799 | C36 H36 F3 N7 O9 S | 799.22473 | -7.1 | -7.7 |
| 2-1-mlE02-2-0800 | C38 H37 F4 N5 O8 S | 799.22990 | 0.0 | -10.5 |
| 2-1-m1E02-2-0801 | C40 H41 N5 O9 S2 | 799.23457 | -6.1 | -6.1 |
| 2-1-mlE02-2-0802 | C40 H41 N5 O9 S2 | 799.23457 | 5.5 | 0.0 |
| 2-1-m1E02-2-0803 | C42 H46 F N5 O8 S | 799.30511 | 0.0 | 0.0 |
| 2-1-m1E02-2-0804 | C36 H34 F2 N4 O9 S3 | 800.14560 | 0.0 | 0.0 |
| 2-1-m1E02-2-0805 | C44 H40 N4 O7 S2 | 800.23384 | -9.3 | 3.4 |
| 2-1-m1E02-2-0806 | C44 H40 N4 O7 S2 | 800.23384 | 0.0 | -17.2 |
| 2-1-m1E02-2-0807 | C35 H36 F N5 O10 S3 | 801.16083 | 0.0 | 0.0 |
| 2-1-m1E02-2-0808 | C40 H37 F2 N5 O7 S2 | 801.21025 | 0.0 | 0.0 |
| 2-1-m1E02-2-0809 | C39 H37 F2 N7 O8 S | 801.23924 | -10.2 | -4.5 |
| 2-1-m1E02-2-0810 | C39 H37 F6 N5 O7 | 801.25972 | -17.7 | 34.9 |
| 2-1-m1E02-2-0811 | C42 H51 N5 O9 S | 801.34075 | 0.0 | 0.0 |
| 2-1-m1E02-2-0812 | C39 H39 F N6 O8 S2 | 802.22548 | 3.2 | -0.7 |
| 2-1-m1E02-2-0813 | C39 H39 F N6 O8 S2 | 802.22548 | 0.0 | 0.0 |
| 2-1-m1E02-2-0814 | C43 H45 F3 N4 O6 S | 802.30119 | 0.0 | 0.0 |
| 2-1-m1E02-2-0815 | C35 H35 F2 N5 O11 S2 | 803.17425 | -7.5 | -11.1 |
| 2-1-m1E02-2-0816 | C36 H36 F3 N5 O9 S2 | 803.19065 | 11.2 | -4.3 |
| 2-1-m1E02-2-0817 | C40 H39 F2 N5 O9 S | 803.24365 | 0.0 | 0.0 |
| 2-1-m1E02-2-0818 | C41 H49 N5 O10 S | 803.32001 | -0.1 | -5.5 |
| 2-1-m1E02-2-0819 | C37 H36 N6 O9 S3 | 804.17059 | -4.8 | -8.8 |
| 2-1-m1E02-2-0820 | C35 H35 F3 N6 O9 S2 | 804.18590 | 0.0 | -7.0 |
| 2-1-m1E02-2-0821 | C42 H43 F3 N4 O7 S | 804.28046 | -5.3 | 0.0 |
| 2-1-m1E02-2-0822 | C45 H48 N4 O8 S | 804.31929 | -6.5 | -10.0 |
| 2-1-m1E02-2-0823 | C39 H37 F2 N5 O8 S2 | 805.20516 | -6.3 | 3.8 |
| 2-1-m1E02-2-0824 | C39 H37 F2 N5 O8 S2 | 805.20516 | 0.0 | 0.0 |
| 2-1-m1E02-2-0825 | C40 H38 F3 N5 O8 S | 805.23932 | -3.0 | -20.2 |
| 2-1-m1E02-2-0826 | C39 H43 N5 O10 S2 | 805.24513 | -0.9 | -5.2 |
| 2-1-m1E02-2-0827 | C40 H37 F3 N4 O9 S | 806.22333 | -3.5 | -3.6 |
| 2-1-m1E02-2-0828 | C41 H41 F3 N4 O8 S | 806.25972 | 0.0 | 0.0 |
| 2-1-m1E02-2-0829 | C41 H41 F3 N4 O8 S | 806.25972 | 0.0 | 0.0 |
| 2-1-m1E02-2-0830 | C39 H36 F3 N5 O9 S | 807.21858 | -7.4 | -10.7 |
| 2-1-m1E02-2-0831 | C38 H41 N5 O11 S2 | 807.22440 | 0.0 | -28.6 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0832 | C41 H44 F3 N5 O7 S | 807.29135 | 0.0 | -11.3 |
| 2-1-m1E02-2-0833 | C40 H36 F4 N4 O8 S | 808.21900 | -7.0 | -15.9 |
| 2-1-m1E02-2-0834 | C42 H40 N4 O9 S2 | 808.22367 | -16.7 | -13.9 |
| 2-1-m1E02-2-0835 | C39 H38 F3 N5 O9 S | 809.23423 | 0.0 | -1.1 |
| 2-1-m1E02-2-0836 | C40 H42 F3 N5 O8 S | 809.27062 | -9.4 | -6.9 |
| 2-1-m1E02-2-0837 | C40 H42 F3 N5 O8 S | 809.27062 | -11.3 | -2.6 |
| 2-1-m1E02-2-0838 | C40 H42 F3 N5 O8 S | 809.27062 | 0.0 | 0.0 |
| 2-1-m1E02-2-0839 | C40 H45 F2 N5 O9 S | 809.29061 | 0.0 | 0.0 |
| 2-1-m1E02-2-0840 | C44 H51 N5 O8 S | 809.34583 | 0.0 | 0.0 |
| 2-1-m1E02-2-0841 | C38 H37 F3 N6 O9 S | 810.22948 | 0.0 | 0.0 |
| 2-1-m1E02-2-0842 | C40 H38 F4 N4 O8 S | 810.23465 | 0.0 | 0.0 |
| 2-1-m1E02-2-0843 | C42 H46 N6 O7 S2 | 810.28694 | 0.0 | 0.0 |
| 2-1-m1E02-2-0844 | C37 H41 N5 O10 S3 | 811.20155 | 0.0 | -6.3 |
| 2-1-m1E02-2-0845 | C39 H40 F3 N5 O9 S | 811.24988 | 0.3 | -6.1 |
| 2-1-mlE02-2-0846 | C39 H40 F3 N5 O9 S | 811.24988 | 0.0 | 0.0 |
| 2-1-mlE02-2-0847 | C43 H49 N5 O9 S | 811.32510 | 0.0 | 0.0 |
| 2-1-m1E02-2-0848 | C38 H35 F3 N4 O9 S2 | 812.17975 | -3.7 | -10.2 |
| 2-1-mlE02-2-0849 | C41 H40 N4 O10 S2 | 812.21858 | -4.0 | 0.0 |
| 2-1-m1E02-2-0850 | C38 H39 F3 N6 O9 S | 812.24513 | 15.6 | -22.0 |
| 2-1-m1E02-2-0851 | C43 H39 F3 N4 O7 S | 812.24915 | -14.4 | -5.7 |
| 2-1-m1E02-2-0852 | C41 H44 N6 O8 S2 | 812.26620 | 0.1 | -3.4 |
| 2-1-m1E02-2-0853 | C47 H48 N4 O7 S | 812.32437 | -9.8 | 0.0 |
| 2-1-m1E02-2-0854 | C36 H39 N5 O11 S3 | 813.18082 | -7.9 | -6.9 |
| 2-1-m1E02-2-0855 | C37 H37 F2 N5 O10 S2 | 813.19499 | 0.0 | -12.1 |
| 2-1-m1E02-2-0856 | C40 H39 N5 O10 S2 | 813.21383 | -5.2 | -2.5 |
| 2-1-m1E02-2-0857 | C39 H39 N7 O9 S2 | 813.22507 | -2.0 | -4.0 |
| 2-1-m1E02-2-0858 | C39 H39 F4 N5 O8 S | 813.24555 | 0.0 | 0.0 |
| 2-1-m1E02-2-0859 | C40 H38 N4 O9 S3 | 814.18009 | -18.0 | 5.6 |
| 2-1-m1E02-2-0860 | C40 H38 N4 O9 S3 | 814.18009 | -6.2 | 0.0 |
| 2-1-m1E02-2-0861 | C41 H37 F3 N6 O7 S | 814.23965 | -8.8 | -9.5 |
| 2-1-m1E02-2-0862 | C40 H42 N6 O9 S2 | 814.24547 | 0.0 | 0.0 |
| 2-1-m1E02-2-0863 | C40 H42 N6 O9 S2 | 814.24547 | 0.0 | 9.7 |
| 2-1-m1E02-2-0864 | C39 H37 N5 O9 S3 | 815.17534 | 1.4 | 2.0 |
| 2-1-m1E02-2-0865 | C37 H36 F3 N5 O9 S2 | 815.19065 | 0.0 | -10.1 |
| 2-1-m1E02-2-0866 | C37 H36 F3 N5 O11 S | 815.20841 | 0.0 | 0.0 |
| 2-1-m1E02-2-0867 | C44 H41 N5 O7 S2 | 815.24474 | 5.5 | 12.7 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0868 | C37 H35 F3 N4 O10 S2 | 816.17467 | -44.7 | -12.0 |
| 2-1-m1E02-2-0869 | C39 H37 F N6 O9 S2 | 816.20475 | -4.1 | -4.9 |
| 2-1-m1E02-2-0870 | C42 H39 F3 N4 O8 S | 816.24407 | -31.7 | 16.7 |
| 2-1-m1E02-2-0871 | C38 H36 F5 N5 O8 S | 817.22047 | 0.4 | -5.2 |
| 2-1-m1E02-2-0872 | C42 H45 F2 N5 O8 S | 817.29569 | 0.0 | 0.0 |
| 2-1-m1E02-2-0873 | C41 H37 F3 N4 O7 S2 | 818.20558 | 0.0 | 0.0 |
| 2-1-m1E02-2-0874 | C41 H37 F3 N4 O7 S2 | 818.20558 | -5.9 | -38.5 |
| 2-1-m1E02-2-0875 | C43 H45 F3 N4 O7 S | 818.29611 | 0.0 | 0.0 |
| 2-1-m1E02-2-0876 | C35 H35 F2 N5 O10 S3 | 819.15141 | -7.1 | -11.3 |
| 2-1-m1E02-2-0877 | C39 H38 F2 N6 O8 S2 | 820.21606 | 3.4 | 2.5 |
| 2-1-m1E02-2-0878 | C40 H38 F3 N5 O9 S | 821.23423 | -6.7 | -12.3 |
| 2-1-m1E02-2-0879 | C42 H46 F3 N5 O7 S | 821.30700 | 0.0 | 0.0 |
| 2-1-m1E02-2-0880 | C40 H38 N8 O8 S2 | 822.22540 | 0.0 | 0.0 |
| 2-1-m1E02-2-0881 | C41 H41 F3 N4 O9 S | 822.25463 | 0.0 | 0.0 |
| 2-1-m1E02-2-0882 | C42 H45 F3 N4 O8 S | 822.29102 | 0.0 | 0.0 |
| 2-1-m1E02-2-0883 | C41 H44 F3 N5 O8 S | 823.28627 | 2.0 | -0.7 |
| 2-1-m1E02-2-0884 | C36 H36 N6 O11 S3 | 824.16042 | 0.0 | 0.0 |
| 2-1-m1E02-2-0885 | C40 H36 F4 N4 O9 S | 824.21391 | -1.0 | -8.7 |
| 2-1-m1E02-2-0886 | C41 H40 N6 O9 S2 | 824.22982 | -1.3 | -12.6 |
| 2-1-m1E02-2-0887 | C39 H39 F3 N6 O9 S | 824.24513 | -3.9 | -4.0 |
| 2-1-m1E02-2-0888 | C39 H38 F3 N5 O10 S | 825.22915 | 0.0 | -6.1 |
| 2-1-m1E02-2-0889 | C40 H42 F3 N5 O9 S | 825.26553 | -2.2 | -3.1 |
| 2-1-m1E02-2-0890 | C40 H42 F3 N5 O9 S | 825.26553 | 0.0 | -6.6 |
| 2-1-m1E02-2-0891 | C40 H38 N6 O8 S3 | 826.19132 | 3.6 | 2.2 |
| 2-1-m1E02-2-0892 | C40 H38 N6 O8 S3 | 826.19132 | 0.0 | 40.4 |
| 2-1-mlE02-2-0893 | C39 H37 F3 N4 O9 S2 | 826.19540 | 3.0 | -3.8 |
| 2-1-mlE02-2-0894 | C38 H37 F3 N6 O10 S | 826.22440 | 0.0 | 0.0 |
| 2-1-m1E02-2-0895 | C42 H42 N4 O10 S2 | 826.23423 | 0.0 | 0.0 |
| 2-1-mlE02-2-0896 | C39 H37 F4 N5 O9 S | 827.22481 | 0.0 | -5.4 |
| 2-1-m1E02-2-0897 | C41 H40 N4 O9 S3 | 828.19574 | -1.9 | -7.9 |
| 2-1-m1E02-2-0898 | C40 H39 N5 O9 S3 | 829.19099 | -2.3 | -11.8 |
| 2-1-m1E02-2-0899 | C39 H39 F4 N5 O9 S | 829.24046 | 0.0 | 0.0 |
| 2-1-m1E02-2-0900 | C39 H38 N6 O9 S3 | 830.18624 | -0.9 | -6.3 |
| 2-1-m1E02-2-0901 | C41 H39 F N4 O10 S2 | 830.20916 | 12.4 | 0.5 |
| 2-1-m1E02-2-0902 | C40 H36 F6 N4 O7 S | 830.22089 | 0.0 | -8.0 |
| 2-1-m1E02-2-0903 | C41 H37 F3 N6 O8 S | 830.23457 | 12.6 | -75.7 |

(continued)

[Table 11-2-6]

| | | | AS-MS ratio (%) | |
|---|---|---|---|---|
| ID | Molecular Formula | Exact Mass | N1 | N2 |
| 2-1-m1E02-2-0904 | C41 H46 N6 O9 S2 | 830.27677 | 0.0 | 0.0 |
| 2-1-m1E02-2-0905 | C44 H45 F3 N4 O7 S | 830.29611 | 0.0 | 0.0 |
| 2-1-m1E02-2-0906 | C37 H36 F3 N5 O10 S2 | 831.18557 | -5.0 | -7.4 |
| 2-1-m1E02-2-0907 | C40 H41 N5 O11 S2 | 831.22440 | 0.0 | 0.0 |
| 2-1-m1E02-2-0908 | C37 H35 F3 N4 O9 S3 | 832.15183 | 0.0 | 0.0 |
| 2-1-m1E02-2-0909 | C37 H35 F3 N4 O11 S2 | 832.16958 | -7.1 | 6.0 |
| 2-1-m1E02-2-0910 | C40 H37 F N4 O9 S3 | 832.17067 | -6.8 | -13.0 |
| 2-1-m1E02-2-0911 | C39 H40 N6 O11 S2 | 832.21965 | 0.2 | -7.8 |
| 2-1-m1E02-2-0912 | C42 H39 F3 N4 O9 S | 832.23898 | 5.5 | 9.4 |
| 2-1-m1E02-2-0913 | C39 H39 N5 O10 S3 | 833.18590 | -8.9 | -10.3 |
| 2-1-m1E02-2-0914 | C41 H38 F3 N5 O7 S2 | 833.21647 | 5.3 | -25.6 |
| 2-1-m1E02-2-0915 | C40 H38 F3 N7 O8 S | 833.24547 | 1.1 | -8.8 |
| 2-1-m1E02-2-0916 | C38 H38 N6 O10 S3 | 834.18115 | 3.8 | -8.5 |
| 2-1-m1E02-2-0917 | C38 H38 N6 O10 S3 | 834.18115 | 9.1 | 0.0 |
| 2-1-m1E02-2-0918 | C41 H37 F3 N4 O8 S2 | 834.20049 | -31.4 | -42.5 |
| 2-1-m1E02-2-0919 | C41 H37 F3 N4 O8 S2 | 834.20049 | 4.1 | 11.7 |
| 2-1-m1E02-2-0920 | C36 H36 F3 N5 O11 S2 | 835.18048 | -12.5 | -8.2 |
| 2-1-m1E02-2-0921 | C41 H40 F3 N5 O9 S | 835.24988 | 31.2 | 29.4 |
| 2-1-m1E02-2-0922 | C40 H38 F3 N5 O8 S2 | 837.21139 | -13.3 | 29.9 |
| 2-1-m1E02-2-0923 | C40 H38 F3 N5 O8 S2 | 837.21139 | -32.7 | -27.5 |
| 2-1-m1E02-2-0924 | C42 H46 F3 N5 O8 S | 837.30192 | 0.0 | 0.0 |
| 2-1-m1E02-2-0925 | C39 H37 F3 N6 O8 S2 | 838.20664 | 4.0 | -8.1 |
| 2-1-m1E02-2-0926 | C43 H46 N6 O8 S2 | 838.28185 | -7.2 | -17.4 |
| 2-1-m1E02-2-0927 | C42 H45 F3 N4 O9 S | 838.28593 | -2.2 | -7.9 |
| 2-1-m1E02-2-0928 | C36 H36 N6 O10 S4 | 840.13757 | -1.0 | -12.3 |
| 2-1-m1E02-2-0929 | C39 H39 F3 N6 O10 S | 840.24005 | -5.2 | -7.0 |
| 2-1-m1E02-2-0930 | C43 H44 N4 O10 S2 | 840.24989 | 0.0 | 0.0 |
| 2-1-m1E02-2-0931 | C40 H39 N7 O8 S3 | 841.20222 | 8.6 | 2.3 |
| 2-1-m1E02-2-0932 | C40 H42 F3 N5 O10 S | 841.26045 | 2.5 | -3.5 |
| 2-1-m1E02-2-0933 | C41 H46 F3 N5 O9 S | 841.29683 | 0.0 | -7.2 |
| 2-1-m1E02-2-0934 | C39 H37 F3 N4 O10 S2 | 842.19032 | -9.6 | -17.3 |
| 2-1-m1E02-2-0935 | C42 H42 N4 O9 S3 | 842.21139 | 24.3 | 91.2 |
| 2-1-m1E02-2-0936 | C42 H42 N4 O11 S2 | 842.22915 | 10.2 | 0.4 |
| 2-1-m1E02-2-0937 | C41 H41 N5 O9 S3 | 843.20664 | 0.0 | 0.0 |
| 2-1-m1E02-2-0938 | C39 H37 F4 N5 O10 S | 843.21973 | 0.0 | 0.0 |
| 2-1-m1E02-2-0939 | C41 H40 N4 O10 S3 | 844.19066 | 18.3 | -12.5 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0940 | C38 H38 F3 N5 O10 S2 | 845.20122 | 8.8 | 1.0 |
| 2-1-m1E02-2-0941 | C41 H43 N5 O11 S2 | 845.24005 | -4.4 | -4.3 |
| 2-1-m1E02-2-0942 | C40 H36 F6 N4 O8 S | 846.21580 | -5.8 | -24.5 |
| 2-1-m1E02-2-0943 | C44 H45 F3 N4 O8 S | 846.29102 | -24.9 | 23.9 |
| 2-1-m1E02-2-0944 | C40 H38 F N5 O9 S3 | 847.18157 | -8.1 | -3.4 |
| 2-1-m1E02-2-0945 | C40 H41 N5 O10 S3 | 847.20155 | 0.3 | -25.4 |
| 2-1-m1E02-2-0946 | C37 H35 F3 N4 O10 S3 | 848.14674 | -1.7 | -12.8 |
| 2-1-m1E02-2-0947 | C39 H40 N6 O10 S3 | 848.19680 | -6.5 | -9.4 |
| 2-1-m1E02-2-0948 | C41 H38 F2 N4 O10 S2 | 848.19974 | -5.2 | -9.4 |
| 2-1-m1E02-2-0949 | C38 H39 N7 O10 S3 | 849.19205 | -4.4 | -17.1 |
| 2-1-m1E02-2-0950 | C41 H38 F3 N5 O8 S2 | 849.21139 | -29.7 | -42.1 |
| 2-1-m1E02-2-0951 | C40 H40 F N5 O11 S2 | 849.21498 | 0.0 | 0.0 |
| 2-1-m1E02-2-0952 | C39 H37 F6 N5 O8 S | 849.22670 | 0.0 | 0.0 |
| 2-1-m1E02-2-0953 | C40 H38 F3 N7 O9 S | 849.24038 | 0.0 | 4.8 |
| 2-1-m1E02-2-0954 | C43 H46 F3 N5 O8 S | 849.30192 | -8.3 | -4.1 |
| 2-1-m1E02-2-0955 | C40 H36 F2 N4 O9 S3 | 850.16125 | -5.1 | -24.2 |
| 2-1-m1E02-2-0956 | C36 H36 F3 N5 O10 S3 | 851.15764 | 0.0 | -7.2 |
| 2-1-m1E02-2-0957 | C36 H36 F3 N5 012 S2 | 851.17540 | 0.6 | 0.0 |
| 2-1-m1E02-2-0958 | C39 H38 F N5 O10 S3 | 851.17648 | 0.0 | -29.7 |
| 2-1-m1E02-2-0959 | C41 H40 F3 N5 O10 S | 851.24480 | -13.2 | -13.5 |
| 2-1-m1E02-2-0960 | C40 H39 F3 N6 O8 S2 | 852.22229 | -1.9 | -10.0 |
| 2-1-m1E02-2-0961 | C40 H38 F3 N5 O9 S2 | 853.20630 | -20.0 | 18.2 |
| 2-1-m1E02-2-0962 | C40 H38 F3 N5 O9 S2 | 853.20630 | 0.0 | 0.0 |
| 2-1-m1E02-2-0963 | C42 H43 N5 O9 S3 | 857.22229 | -11.3 | 8.9 |
| 2-1-m1E02-2-0964 | C41 H46 F3 N5 O10 S | 857.29175 | 0.0 | 0.0 |
| 2-1-m1E02-2-0965 | C41 H41 N5 O10 S3 | 859.20155 | 6.8 | 3.0 |
| 2-1-m1E02-2-0966 | C42 H45 N5 O11 S2 | 859.25570 | -3.6 | 0.7 |
| 2-1-m1E02-2-0967 | C38 H38 F3 N5 O11 S2 | 861.19613 | -7.3 | -4.7 |
| 2-1-m1E02-2-0968 | C41 H43 N5 O10 S3 | 861.21720 | -1.6 | -1.9 |
| 2-1-m1E02-2-0969 | C41 H43 N5 012 S2 | 861.23496 | -21.9 | 5.8 |
| 2-1-m1E02-2-0970 | C40 H42 N6 O10 S3 | 862.21245 | 0.0 | 0.0 |
| 2-1-m1E02-2-0971 | C45 H42 N4 O10 S2 | 862.23423 | -450.8 | 34.1 |
| 2-1-m1E02-2-0972 | C40 H41 N5 O11 S3 | 863.19647 | -24.6 | -5.0 |
| 2-1-m1E02-2-0973 | C44 H40 N4 O9 S3 | 864.19574 | 0.0 | 0.0 |
| 2-1-m1E02-2-0974 | C40 H37 F2 N5 O9 S3 | 865.17215 | -16.7 | -3.1 |
| 2-1-m1E02-2-0975 | C39 H37 F6 N5 O9 S | 865.22162 | 1.3 | 0.0 |

(continued)

| [Table 11-2-6] | | | | |
|---|---|---|---|---|
| | | | AS-MS ratio (%) | |
| ID | Molecular Formula | Exact Mass | N1 | N2 |
| 2-1-m1E02-2-0976 | C43 H46 F3 N5 O9 S | 865.29683 | 0.0 | 0.0 |
| 2-1-m1E02-2-0977 | C39 H39 F N6 O10 S3 | 866.18738 | -4.1 | -10.0 |
| 2-1-m1E02-2-0978 | C36 H36 F3 N5 O11 S3 | 867.15255 | 0.0 | -5.1 |
| 2-1-m1E02-2-0979 | C40 H39 F2 N5 O11 S2 | 867.20555 | 1.3 | -11.4 |
| 2-1-m1E02-2-0980 | C40 H39 F3 N6 O9 S2 | 868.21720 | 0.4 | 17.0 |
| 2-1-m1E02-2-0981 | C39 H37 F2 N5 O10 S3 | 869.16706 | 9.4 | 5.2 |
| 2-1-m1E02-2-0982 | C41 H44 N6 O10 S3 | 876.22810 | -7.5 | -8.6 |
| 2-1-m1E02-2-0983 | C40 H42 N6 O11 S3 | 878.20737 | 0.0 | 0.0 |
| 2-1-m1E02-2-0984 | C44 H41 N5 O9 S3 | 879.20664 | -14.1 | 2.5 |
| 2-1-m1E02-2-0985 | C42 H39 F3 N4 O10 S2 | 880.20597 | -14.5 | -36.2 |
| 2-1-m1E02-2-0986 | C41 H37 F3 N4 O9 S3 | 882.16748 | 14.2 | -9.7 |
| 2-1-m1E02-2-0987 | C39 H38 F2 N6 O10 S3 | 884.17796 | -9.6 | -12.1 |
| 2-1-m1E02-2-0988 | C41 H40 N6 O11 S3 | 888.19172 | 3.1 | -5.3 |
| 2-1-m1E02-2-0989 | C40 H38 N6 O10 S4 | 890.15322 | 8.4 | -4.6 |
| 2-1-m1E02-2-0990 | C42 H39 F3 N4 O11 S2 | 896.20088 | -24.4 | -178.7 |
| 2-1-m1E02-2-0991 | C41 H38 F3 N5 O9 S3 | 897.17837 | 5.0 | -20.7 |
| 2-1-m1E02-2-0992 | C41 H37 F3 N4 O10 S3 | 898.16239 | 18.6 | -5.2 |
| 2-1-m1E02-2-0993 | C41 H40 F3 N5 O11 S2 | 899.21178 | -4.6 | 0.2 |
| 2-1-m1E02-2-0994 | C40 H38 F3 N5 O10 S3 | 901.17329 | -6.7 | -10.0 |
| 2-1-m1E02-2-0995 | C40 H39 N7 O10 S4 | 905.16412 | 8.6 | 6.5 |
| 2-1-m1E02-2-0996 | C41 H38 F3 N5 O10 S3 | 913.17329 | 47.6 | -21.7 |
| 2-1-m1E02-2-0997 | C41 H40 F3 N5 012 S2 | 915.20670 | 3.1 | -10.1 |
| 2-1-m1E02-2-0998 | C40 H39 F3 N6 O10 S3 | 916.18419 | -3.2 | 2.0 |
| 2-1-m1E02-2-0999 | C40 H38 F3 N5 O11 S3 | 917.16820 | -5.9 | -10.2 |
| 2-1-m1E02-2-1000 | C40 H39 F3 N6 O11 S3 | 932.17910 | -6.6 | -19.0 |

[Table 596]

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0001 | C40 H36 N4 O4 S | 668.24573 | -35.2 | 0.0 |
| 2-1-m1E03-2-0002 | C39 H35 N5 O4 S | 669.24098 | -15.5 | 4.9 |
| 2-1-m1E03-2-0003 | C39 H35 N5 O4 S | 669.24098 | 0.0 | 0.0 |
| 2-1-m1E03-2-0004 | C38 H34 N6 O4 S | 670.23622 | -4.8 | 12.0 |
| 2-1-m1E03-2-0005 | C38 H34 N6 O4 S | 670.23622 | -33.3 | 10.9 |
| 2-1-m1E03-2-0006 | C37 H33 N7 O4 S | 671.23147 | -22.4 | -21.2 |
| 2-1-m1E03-2-0007 | C37 H33 N5 O4 S2 | 675.19740 | 0.0 | -4.0 |
| 2-1-m1E03-2-0008 | C36 H32 N6 O4 S2 | 676.19264 | -0.5 | 10.0 |
| 2-1-m1E03-2-0009 | C41 H38 N4 O4 S | 682.26138 | 24.5 | 14.6 |
| 2-1-m1E03-2-0010 | C40 H37 N5 O4 S | 683.25663 | 0.0 | 0.0 |
| 2-1-m1E03-2-0011 | C39 H36 N6 O4 S | 684.25187 | -18.9 | -19.3 |
| 2-1-m1E03-2-0012 | C39 H34 N4 O6 S | 686.21991 | 3.7 | -1.5 |
| 2-1-m1E03-2-0013 | C40 H35 FN4 O4 S | 686.23630 | 16.3 | -49.3 |
| 2-1-m1E03-2-0014 | C38 H33 N5 O6 S | 687.21515 | 10.3 | 20.9 |
| 2-1-m1E03-2-0015 | C39 H34 FN5 O4 S | 687.23155 | -56.4 | 9.9 |
| 2-1-m1E03-2-0016 | C39 H37 N5 O5 S | 687.25154 | 0.6 | 5.5 |
| 2-1-m1E03-2-0017 | C38 H33 F N6 O4 S | 688.22680 | 0.0 | 0.0 |
| 2-1-m1E03-2-0018 | C38 H36 N6 O5 S | 688.24679 | -9.7 | -1.5 |
| 2-1-m1E03-2-0019 | C38 H36 N6 O5 S | 688.24679 | 3.5 | -8.0 |
| 2-1-m1E03-2-0020 | C38 H35 N5 O4 S2 | 689.21305 | 0.0 | 0.0 |
| 2-1-m1E03-2-0021 | C37 H35 N7 O5 S | 689.24204 | -2.1 | 0.3 |
| 2-1-m1E03-2-0022 | C37 H35 N7 O5 S | 689.24204 | 0.0 | 0.0 |
| 2-1-m1E03-2-0023 | C36 H34 N8 O5 S | 690.23729 | -9.4 | -26.1 |
| 2-1-m1E03-2-0024 | C37 H32 F N5 O4 S2 | 693.18797 | 0.0 | -84.6 |
| 2-1-m1E03-2-0025 | C36 H34 N6 O5 S2 | 694.20321 | 8.0 | -2.4 |
| 2-1-m1E03-2-0026 | C35 H33 N7 O5 S2 | 695.19846 | 0.0 | -1.9 |
| 2-1-m1E03-2-0027 | C41 H36 N4 O5 S | 696.24064 | 0.0 | 0.0 |
| 2-1-m1E03-2-0028 | C42 H40 N4 O4 S | 696.27703 | 0.0 | 0.0 |
| 2-1-m1E03-2-0029 | C40 H35 N5 O5 S | 697.23589 | 8.5 | 7.7 |
| 2-1-m1E03-2-0030 | C40 H35 N5 O5 S | 697.23589 | 0.0 | 0.0 |
| 2-1-m1E03-2-0031 | C41 H39 N5 O4 S | 697.27228 | -12.6 | 41.4 |
| 2-1-m1E03-2-0032 | C39 H34 N6 O5 S | 698.23114 | 12.9 | 24.1 |
| 2-1-m1E03-2-0033 | C41 H38 N4 O5 S | 698.25629 | 0.0 | 0.0 |
| 2-1-m1E03-2-0034 | C40 H38 N6 04 S | 698.26752 | 16.4 | -5.7 |
| 2-1-m1E03-2-0035 | C40 H37 N5 O5 S | 699.25154 | -2.4 | 4.6 |
| 2-1-m1E03-2-0036 | C40 H36 N4 O6 S | 700.23556 | 0.0 | 0.0 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0037 | C39 H36 N6 O5 S | 700.24679 | 0.0 | 0.0 |
| 2-1-m1E03-2-0038 | C40 H39 N5 O5 S | 701.26719 | -11.4 | 0.8 |
| 2-1-m1E03-2-0039 | C39 H34 N4 O5 S2 | 702.19706 | 0.0 | 0.0 |
| 2-1-m1E03-2-0040 | C39 H38 N6 O5 S | 702.26244 | 10.9 | 4.1 |
| 2-1-m1E03-2-0041 | C38 H33 N5 O5 S2 | 703.19231 | -12.8 | 18.5 |
| 2-1-m1E03-2-0042 | C39 H37 N5 04 S2 | 703.22870 | 17.7 | -89.5 |
| 2-1-m1E03-2-0043 | C38 H37 N7 O5 S | 703.25769 | 0.0 | 15.0 |
| 2-1-m1E03-2-0044 | C39 H33 F N4 O6 S | 704.21048 | 0.0 | 0.0 |
| 2-1-m1E03-2-0045 | C40 H34 F2 N4 04 S | 704.22688 | 3.7 | 12.6 |
| 2-1-m1E03-2-0046 | C38 H35 N5 O5 S2 | 705.20796 | 0.0 | 0.0 |
| 2-1-m1E03-2-0047 | C39 H33 F2 N5 O4 S | 705.22213 | 22.1 | 81.1 |
| 2-1-m1E03-2-0048 | C38 H35 N5 O7 S | 705.22572 | -3.9 | -2.8 |
| 2-1-m1E03-2-0049 | C39 H36 F N5 O5 S | 705.24212 | 1.1 | 12.9 |
| 2-1-m1E03-2-0050 | C38 H32 F2 N6 04 S | 706.21738 | 0.0 | 0.0 |
| 2-1-m1E03-2-0051 | C37 H34 N6 O7 S | 706.22097 | -17.1 | -11.3 |
| 2-1-m1E03-2-0052 | C38 H35 F N6 O5 S | 706.23737 | -3.1 | 1.4 |
| 2-1-m1E03-2-0053 | C37 H34 F N7 O5 S | 707.23262 | -6.5 | 4.1 |
| 2-1-m1E03-2-0054 | C37 H36 N6 O5 S2 | 708.21886 | -10.5 | 1.9 |
| 2-1-m1E03-2-0055 | C42 H38 N4 O5 S | 710.25629 | -27.3 | -17.3 |
| 2-1-m1E03-2-0056 | C37 H31 F2 N5 04 S2 | 711.17855 | 0.0 | 0.0 |
| 2-1-m1E03-2-0057 | C41 H37 N5 O5 S | 711.25154 | -48.6 | 19.5 |
| 2-1-m1E03-2-0058 | C41 H37 N5 O5 S | 711.25154 | 0.0 | 0.0 |
| 2-1-m1E03-2-0059 | C36 H33 F N6 O5 S2 | 712.19379 | -4.1 | 4.5 |
| 2-1-m1E03-2-0060 | C40 H36 N6 O5 S | 712.24679 | 0.2 | 21.3 |
| 2-1-m1E03-2-0061 | C41 H35 F N4 O5 S | 714.23122 | 0.0 | 0.0 |
| 2-1-m1E03-2-0062 | C41 H35 F N4 O5 S | 714.23122 | -6.4 | -24.1 |
| 2-1-m1E03-2-0063 | C41 H38 N4 O6 S | 714.25121 | 0.0 | 0.0 |
| 2-1-m1E03-2-0064 | C40 H34 F N5 O5 S | 715.22647 | 0.0 | 0.0 |
| 2-1-mlE03-2-0065 | C40 H34 F N5 O5 S | 715.22647 | 1.1 | -8.1 |
| 2-1-mlE03-2-0066 | C40 H37 N5 O6 S | 715.24645 | 0.2 | -7.5 |
| 2-1-m1E03-2-0067 | C41 H41 N5 O5 S | 715.28284 | -11.6 | 1.9 |
| 2-1-m1E03-2-0068 | C40 H36 N4 O5 S2 | 716.21271 | 33.6 | -10.2 |
| 2-1-mlE03-2-0069 | C40 H36 N4 O7 S | 716.23047 | 0.0 | 0.0 |
| 2-1-m1E03-2-0070 | C39 H36 N6 O6 S | 716.24170 | 0.3 | -9.4 |
| 2-1-m1E03-2-0071 | C39 H36 N6 O6 S | 716.24170 | -1.9 | -4.7 |
| 2-1-m1E03-2-0072 | C40 H40 N6 O5 S | 716.27809 | 6.2 | 14.6 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0073 | C38 H35 N7 O6 S | 717.23695 | 3.1 | 0.1 |
| 2-1-m1E03-2-0074 | C40 H39 N5 O6 S | 717.26210 | 35.6 | 20.9 |
| 2-1-m1E03-2-0075 | C39 H39 N7 O5 S | 717.27334 | 33.9 | -11.0 |
| 2-1-m1E03-2-0076 | C39 H38 N6 O6 S | 718.25735 | -12.8 | 9.8 |
| 2-1-m1E03-2-0077 | C44 H38 N4 O4 S | 718.26138 | -5.6 | 15.9 |
| 2-1-m1E03-2-0078 | C39 H37 N5 O7 S | 719.24137 | -3.6 | -10.0 |
| 2-1-m1E03-2-0079 | C38 H37 N7 O6 S | 719.25260 | 0.0 | 0.0 |
| 2-1-m1E03-2-0080 | C43 H37 N5 O4 S | 719.25663 | 0.0 | -90.5 |
| 2-1-m1E03-2-0081 | C39 H33 F N4 O5 S2 | 720.18764 | 0.0 | 0.0 |
| 2-1-m1E03-2-0082 | C42 H36 N6 O4 S | 720.25187 | 0.0 | 0.0 |
| 2-1-m1E03-2-0083 | C42 H36 N6 04 S | 720.25187 | 0.0 | 0.0 |
| 2-1-m1E03-2-0084 | C38 H35 N5 O6 S2 | 721.20288 | -3.3 | 0.3 |
| 2-1-m1E03-2-0085 | C41 H35 N7 O4 S | 721.24712 | -91.2 | 0.0 |
| 2-1-m1E03-2-0086 | C38 H34 N4 O7 S2 | 722.18689 | -19.8 | 2.1 |
| 2-1-m1E03-2-0087 | C37 H34 N6 O6 S2 | 722.19812 | -5.9 | -0.5 |
| 2-1-m1E03-2-0088 | C39 H32 F2 N4 O6 S | 722.20106 | -1.1 | -1.6 |
| 2-1-m1E03-2-0089 | C38 H38 N6 O5 S2 | 722.23451 | -3.5 | 5.7 |
| 2-1-m1E03-2-0090 | C37 H33 N5 O7 S2 | 723.18214 | -15.4 | 23.8 |
| 2-1-m1E03-2-0091 | C38 H34 F N5 O7 S | 723.21630 | -2.1 | -2.0 |
| 2-1-m1E03-2-0092 | C39 H35 F2 N5 O5 S | 723.23270 | -45.6 | 9.1 |
| 2-1-m1E03-2-0093 | C37 H36 N6 O6 S2 | 724.21377 | 0.0 | 0.0 |
| 2-1-m1E03-2-0094 | C42 H36 N4 04 S2 | 724.21780 | 0.0 | 0.0 |
| 2-1-m1E03-2-0095 | C38 H34 F2 N6 O5 S | 724.22795 | -1.9 | 12.2 |
| 2-1-m1E03-2-0096 | C39 H40 N4 O6 S2 | 724.23893 | 0.0 | 0.0 |
| 2-1-m1E03-2-0097 | C43 H40 N4 O5 S | 724.27194 | -11.1 | 3.5 |
| 2-1-m1E03-2-0098 | C41 H35 N5 04 S2 | 725.21305 | 0.0 | 0.0 |
| 2-1-mlE03-2-0099 | C41 H35 N5 04 S2 | 725.21305 | 0.0 | 0.0 |
| 2-1-m1E03-2-0100 | C37 H33 F2 N7 O5 S | 725.22319 | -15.8 | 31.4 |
| 2-1-m1E03-2-0101 | C38 H39 N5 O6 S2 | 725.23418 | 31.8 | 21.0 |
| 2-1-m1E03-2-0102 | C42 H39 N5 O5 S | 725.26719 | 0.0 | 0.0 |
| 2-1-m1E03-2-0103 | C42 H39 N5 O5 S | 725.26719 | 0.0 | 45.2 |
| 2-1-m1E03-2-0104 | C42 H38 N4 O6 S | 726.25121 | 0.0 | 0.0 |
| 2-1-m1E03-2-0105 | C41 H37 N5 O6 S | 727.24645 | 0.0 | 0.0 |
| 2-1-m1E03-2-0106 | C42 H37 F N4 O5 S | 728.24687 | 0.0 | 0.0 |
| 2-1-m1E03-2-0107 | C43 H44 N4 O5 S | 728.30324 | 0.0 | 0.0 |
| 2-1-m1E03-2-0108 | C41 H36 F N5 O5 S | 729.24212 | -30.2 | -4.8 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0109 | C41 H39 N5 O6 S | 729.26210 | -7.4 | 4.8 |
| 2-1-m1E03-2-0110 | C42 H43 N5 O5 S | 729.29849 | -9.7 | 13.6 |
| 2-1-m1E03-2-0111 | C36 H32 F2 N6 O5 S2 | 730.18437 | -20.1 | 9.0 |
| 2-1-m1E03-2-0112 | C41 H38 N4 O5 S2 | 730.22836 | -5.0 | -0.6 |
| 2-1-m1E03-2-0113 | C40 H38 N6 O6 S | 730.25735 | 7.1 | 1.7 |
| 2-1-m1E03-2-0114 | C40 H38 N6 O6 S | 730.25735 | 2.5 | 1.8 |
| 2-1-m1E03-2-0115 | C39 H37 N7 O6 S | 731.25260 | 1.5 | -7.0 |
| 2-1-m1E03-2-0116 | C40 H36 N4 O6 S2 | 732.20763 | -6.8 | 0.0 |
| 2-1-mlE03-2-0117 | C40 H36 N4 O6 S2 | 732.20763 | 4.0 | 7.5 |
| 2-1-m1E03-2-0118 | C40 H36 N4 O6 S2 | 732.20763 | 0.0 | 0.0 |
| 2-1-m1E03-2-0119 | C41 H34 F2 N4 O5 S | 732.22180 | 9.7 | 0.4 |
| 2-1-m1E03-2-0120 | C41 H34 F2 N4 O5 S | 732.22180 | -1.7 | -10.1 |
| 2-1-m1E03-2-0121 | C39 H35 N5 O6 S2 | 733.20288 | -9.2 | -1.3 |
| 2-1-m1E03-2-0122 | C39 H35 N5 O6 S2 | 733.20288 | -10.2 | -12.8 |
| 2-1-m1E03-2-0123 | C39 H35 N5 O6 S2 | 733.20288 | -17.2 | -6.8 |
| 2-1-m1E03-2-0124 | C40 H33 F2 N5 O5 S | 733.21705 | -63.2 | 25.8 |
| 2-1-m1E03-2-0125 | C40 H36 F N5 O6 S | 733.23703 | -3.0 | -2.2 |
| 2-1-m1E03-2-0126 | C40 H36 F N5 O6 S | 733.23703 | -11.0 | 122.9 |
| 2-1-m1E03-2-0127 | C40 H39 N5 O7 S | 733.25702 | -8.5 | 14.2 |
| 2-1-m1E03-2-0128 | C38 H34 N6 O6 S2 | 734.19812 | -15.0 | -3.6 |
| 2-1-m1E03-2-0129 | C38 H34 N6 O6 S2 | 734.19812 | -7.1 | 5.9 |
| 2-1-m1E03-2-0130 | C39 H35 F N6 O6 S | 734.23228 | -1.9 | -3.3 |
| 2-1-m1E03-2-0131 | C39 H35 F N6 O6 S | 734.23228 | 0.0 | 0.0 |
| 2-1-m 1 E03-2-0132 | C43 H38 N6 04 S | 734.26752 | 0.0 | 11.3 |
| 2-1-mlE03-2-0133 | C37 H33 N7 O6 S2 | 735.19337 | -24.7 | -8.1 |
| 2-1-m 1 E03-2-0134 | C39 H37 N5 O6 S2 | 735.21853 | -6.0 | 10.1 |
| 2-1-mlE03-2-0135 | C39 H37 N5 O8 S | 735.23628 | -5.8 | 1.9 |
| 2-1-m1E03-2-0136 | C39 H36 N4 O7 S2 | 736.20254 | -9.6 | -19.3 |
| 2-1-mlE03-2-0137 | C41 H35 F3 N4 04 S | 736.23311 | 26.3 | 22.5 |
| 2-1-m1E03-2-0138 | C41 H35 F3 N4 O4 S | 736.23311 | -44.1 | 30.2 |
| 2-1-m1E03-2-0139 | C43 H36 N4 O6 S | 736.23556 | -3.9 | 7.9 |
| 2-1-m1E03-2-0140 | C40 H34 F3 N5 O4 S | 737.22836 | -13.9 | -7.4 |
| 2-1-m1E03-2-0141 | C40 H34 F3 N5 O4 S | 737.22836 | -0.5 | 5.0 |
| 2-1-m1E03-2-0142 | C38 H34 N4 O6 S3 | 738.16405 | 0.0 | 0.0 |
| 2-1-m1E03-2-0143 | C39 H32 F2 N4 O5 S2 | 738.17822 | 83.8 | -22.6 |
| 2-1-m1E03-2-0144 | C39 H33 F3 N6 O4 S | 738.22361 | 68.9 | -9.4 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0145 | C43 H38 N4 04 S2 | 738.23345 | -4.1 | -25.7 |
| 2-1-m1E03-2-0146 | C42 H35 F N6 04 S | 738.24245 | -29.6 | 61.1 |
| 2-1-m1E03-2-0147 | C40 H42 N4 O6 S2 | 738.25458 | 0.0 | 0.0 |
| 2-1-m1E03-2-0148 | C37 H33 N5 O6 S3 | 739.15930 | -11.5 | -23.7 |
| 2-1-m1E03-2-0149 | C37 H33 N5 O6 S3 | 739.15930 | 0.0 | 0.0 |
| 2-1-m1E03-2-0150 | C38 H34 F N5 O6 S2 | 739.19345 | -7.6 | 2.9 |
| 2-1-m1E03-2-0151 | C41 H37 N7 O5 S | 739.25769 | -8.0 | -2.3 |
| 2-1-m1E03-2-0152 | C43 H41 N5 O5 S | 739.28284 | 0.0 | 0.0 |
| 2-1-m1E03-2-0153 | C36 H32 N6 O6 S3 | 740.15454 | -9.5 | -3.2 |
| 2-1-m1E03-2-0154 | C38 H33 F N4 O7 S2 | 740.17747 | 0.0 | 0.0 |
| 2-1-m 1 E03-2-0155 | C40 H36 N8 O5 S | 740.25294 | 10.2 | 30.3 |
| 2-1-m1E03-2-0156 | C37 H35 N5 O8 S2 | 741.19270 | -19.8 | 22.2 |
| 2-1-m1E03-2-0157 | C38 H33 F2 N5 O7 S | 741.20688 | 0.0 | 0.0 |
| 2-1-m1E03-2-0158 | C42 H39 N5 O6 S | 741.26210 | -0.5 | -3.4 |
| 2-1-m1E03-2-0159 | C36 H34 N6 O8 S2 | 742.18795 | -1.9 | 7.4 |
| 2-1-m1E03-2-0160 | C42 H35 F N4 04 S2 | 742.20838 | -7.2 | 10.1 |
| 2-1-m1E03-2-0161 | C39 H39 F N4 O6 S2 | 742.22950 | 0.0 | 0.0 |
| 2-1-m1E03-2-0162 | C43 H39 F N4 O5 S | 742.26252 | 0.0 | 0.0 |
| 2-1-m1E03-2-0163 | C44 H46 N4 O5 S | 742.31889 | 0.0 | 0.0 |
| 2-1-m1E03-2-0164 | C38 H32 F3 N5 04 S2 | 743.18478 | 0.0 | 0.0 |
| 2-1-m1E03-2-0165 | C41 H37 N5 O5 S2 | 743.22361 | 0.0 | 0.0 |
| 2-1-m1E03-2-0166 | C38 H41 N5 O7 S2 | 743.24474 | -5.5 | -3.5 |
| 2-1-m1E03-2-0167 | C42 H41 N5 O6 S | 743.27775 | 5.1 | 17.5 |
| 2-1-m1E03-2-0168 | C40 H36 N6 O5 S2 | 744.21886 | 0.0 | 0.0 |
| 2-1-m1E03-2-0169 | C40 H36 N6 O5 S2 | 744.21886 | -10.2 | -1.0 |
| 2-1-m1E03-2-0170 | C37 H40 N6 O7 S2 | 744.23999 | -8.7 | 2.0 |
| 2-1-m1E03-2-0171 | C42 H37 F N4 O6 S | 744.24178 | -6.5 | 2.0 |
| 2-1-m1E03-2-0172 | C42 H40 N4 O5 S2 | 744.24401 | -5.3 | -11.1 |
| 2-1-mlE03-2-0173 | C41 H40 N6 O6 S | 744.27300 | -2.5 | -5.3 |
| 2-1-m1E03-2-0174 | C41 H40 N6 O6 S | 744.27300 | -5.2 | 1.1 |
| 2-1-m1E03-2-0175 | C39 H35 N7 O5 S2 | 745.21411 | -2.0 | -4.3 |
| 2-1-m1E03-2-0176 | C41 H39 N5 O5 S2 | 745.23926 | -26.7 | -9.3 |
| 2-1-m1E03-2-0177 | C41 H39 N5 O7 S | 745.25702 | 19.2 | 0.0 |
| 2-1-m1E03-2-0178 | C38 H34 N8 O5 S2 | 746.20936 | 20.0 | -13.8 |
| 2-1-m1E03-2-0179 | C41 H38 N4 O6 S2 | 746.22328 | 11.0 | 14.2 |
| 2-1-m1E03-2-0180 | C41 H38 N4 O6 S2 | 746.22328 | 0.0 | 0.0 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0181 | C41 H38 N4 O6 S2 | 746.22328 | -16.0 | 17.1 |
| 2-1-m1E03-2-0182 | C40 H38 N6 O7 S | 746.25227 | -9.1 | 14.7 |
| 2-1-m1E03-2-0183 | C45 H38 N4 O5 S | 746.25629 | 0.0 | 0.0 |
| 2-1-m1E03-2-0184 | C43 H43 F N4 O5 S | 746.29382 | 0.0 | 0.0 |
| 2-1-m1E03-2-0185 | C40 H37 N5 O6 S2 | 747.21853 | -4.6 | 9.0 |
| 2-1-m1E03-2-0186 | C40 H37 N5 O6 S2 | 747.21853 | 0.0 | 0.0 |
| 2-1-m1E03-2-0187 | C40 H37 N5 O6 S2 | 747.21853 | -39.3 | 4.9 |
| 2-1-m1E03-2-0188 | C41 H35 F2 N5 O5 S | 747.23270 | 27.0 | -43.4 |
| 2-1-m1E03-2-0189 | C44 H37 N5 O5 S | 747.25154 | -7.7 | 12.3 |
| 2-1-m1E03-2-0190 | C41 H38 F N5 O6 S | 747.25268 | -6.6 | 4.9 |
| 2-1-m1E03-2-0191 | C42 H45 N5 O6 S | 747.30905 | -0.8 | -8.0 |
| 2-1-m1E03-2-0192 | C39 H36 N6 O6 S2 | 748.21377 | -58.3 | 0.0 |
| 2-1-m1E03-2-0193 | C39 H36 N6 O6 S2 | 748.21377 | 0.0 | 0.0 |
| 2-1-m1E03-2-0194 | C40 H37 F N6 O6 S | 748.24793 | 4.8 | 0.7 |
| 2-1-m1E03-2-0195 | C44 H40 N6 O4 S | 748.28317 | -5.7 | -8.9 |
| 2-1-m1E03-2-0196 | C41 H44 N6 O6 S | 748.30430 | 9.0 | 11.7 |
| 2-1-m1E03-2-0197 | C40 H39 N5 O6 S2 | 749.23418 | -10.1 | -3.2 |
| 2-1-m1E03-2-0198 | C39 H34 N4 O8 S2 | 750.18181 | 2.7 | 16.3 |
| 2-1-m1E03-2-0199 | C40 H35 F N4 O6 S2 | 750.19820 | 0.0 | 0.0 |
| 2-1-m1E03-2-0200 | C40 H35 F N4 O6 S2 | 750.19820 | 16.7 | 10.1 |
| 2-1-m1E03-2-0201 | C41 H33 F3 N4 O5 S | 750.21238 | 0.0 | -7.5 |
| 2-1-m1E03-2-0202 | C40 H38 N4 O7 S2 | 750.21819 | 0.0 | 0.0 |
| 2-1-m1E03-2-0203 | C42 H37 F3 N4 O4 S | 750.24876 | 0.0 | 0.0 |
| 2-1-m1E03-2-0204 | C43 H38 N6 O5 S | 750.26244 | 0.0 | 0.0 |
| 2-1-m1E03-2-0205 | C37 H33 N7 O5 S3 | 751.17053 | 0.0 | 0.0 |
| 2-1-m1E03-2-0206 | C38 H33 N5 O8 S2 | 751.17705 | -11.7 | -9.9 |
| 2-1-m1E03-2-0207 | C39 H34 F N5 O6 S2 | 751.19345 | -7.2 | -6.6 |
| 2-1-m1E03-2-0208 | C39 H37 N5 O7 S2 | 751.21344 | -1.9 | 25.2 |
| 2-1-m1E03-2-0209 | C39 H37 N5 O7 S2 | 751.21344 | 7.1 | 13.2 |
| 2-1-m1E03-2-0210 | C39 H37 N5 O7 S2 | 751.21344 | 58.2 | 16.7 |
| 2-1-m1E03-2-0211 | C40 H35 F2 N5 O6 S | 751.22761 | -45.9 | -33.7 |
| 2-1-m1E03-2-0212 | C40 H35 F2 N5 O6 S | 751.22761 | 0.0 | 0.0 |
| 2-1-m1E03-2-0213 | C39 H36 N4 O6 S3 | 752.17970 | -12.7 | -5.9 |
| 2-1-m1E03-2-0214 | C38 H33 F N6 O6 S2 | 752.18870 | -65.5 | 0.0 |
| 2-1-m1E03-2-0215 | C39 H36 N4 O8 S2 | 752.19746 | -10.1 | -10.5 |
| 2-1-m1E03-2-0216 | C38 H36 N6 O7 S2 | 752.20869 | -2.4 | 4.6 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0217 | C38 H36 N6 O7 S2 | 752.20869 | 1.2 | 6.3 |
| 2-1-m1E03-2-0218 | C38 H36 N6 O7 S2 | 752.20869 | -16.2 | -6.9 |
| 2-1-m1E03-2-0219 | C43 H36 N4 O5 S2 | 752.21271 | -14.4 | 25.4 |
| 2-1-m1E03-2-0220 | C39 H34 F2 N6 O6 S | 752.22286 | 0.0 | 0.0 |
| 2-1-m1E03-2-0221 | C41 H35 F3 N4 O5 S | 752.22803 | 51.4 | -4.5 |
| 2-1-m1E03-2-0222 | C44 H40 N4 04 S2 | 752.24910 | 0.0 | 9.5 |
| 2-1-m1E03-2-0223 | C41 H44 N4 O6 S2 | 752.27023 | 0.0 | 0.0 |
| 2-1-m1E03-2-0224 | C38 H35 N5 O6 S3 | 753.17495 | -34.2 | 24.4 |
| 2-1-m1E03-2-0225 | C37 H35 N7 O7 S2 | 753.20394 | -6.6 | -0.5 |
| 2-1-m1E03-2-0226 | C37 H35 N7 O7 S2 | 753.20394 | -9.5 | -4.4 |
| 2-1-m1E03-2-0227 | C40 H34 F3 N5 O5 S | 753.22327 | 0.0 | 0.0 |
| 2-1-m1E03-2-0228 | C42 H39 N7 O5 S | 753.27334 | 1.7 | 2.0 |
| 2-1-m1E03-2-0229 | C36 H34 N8 O7 S2 | 754.19919 | -17.3 | 0.0 |
| 2-1-m1E03-2-0230 | C40 H33 F3 N4 O6 S | 754.20729 | 1.3 | 0.0 |
| 2-1-m1E03-2-0231 | C39 H33 F3 N6 O5 S | 754.21852 | 0.0 | 0.0 |
| 2-1-m1E03-2-0232 | C41 H34 F4 N4 04 S | 754.22369 | -2.3 | -10.8 |
| 2-1-m1E03-2-0233 | C43 H38 N4 O5 S2 | 754.22836 | 0.0 | 0.0 |
| 2-1-m1E03-2-0234 | C40 H42 N4 O7 S2 | 754.24949 | 42.5 | 75.0 |
| 2-1-m1E03-2-0235 | C38 H37 N5 O8 S2 | 755.20835 | -6.6 | 0.0 |
| 2-1-m1E03-2-0236 | C40 H36 F3 N5 O5 S | 755.23892 | -9.9 | -0.1 |
| 2-1-m1E03-2-0237 | C40 H36 F3 N5 O5 S | 755.23892 | 6.6 | -2.4 |
| 2-1-m1E03-2-0238 | C38 H33 F N4 O6 S3 | 756.15463 | 0.0 | 0.0 |
| 2-1-m1E03-2-0239 | C42 H34 F2 N6 04 S | 756.23303 | -6.5 | 12.6 |
| 2-1-m1E03-2-0240 | C39 H35 F3 N6 O5 S | 756.23417 | -6.5 | 12.6 |
| 2-1-m1E03-2-0241 | C39 H35 F3 N6 O5 S | 756.23417 | 9.8 | 6.8 |
| 2-1-m1E03-2-0242 | C45 H48 N4 O5 S | 756.33454 | 0.0 | 0.0 |
| 2-1-m1E03-2-0243 | C37 H32 F N5 O6 S3 | 757.14987 | -17.3 | 5.6 |
| 2-1-m1E03-2-0244 | C37 H35 N5 O7 S3 | 757.16986 | -6.0 | 14.8 |
| 2-1-m1E03-2-0245 | C38 H33 F2 N5 O6 S2 | 757.18403 | 34.2 | 30.9 |
| 2-1-m1E03-2-0246 | C38 H34 F3 N7 O5 S | 757.22942 | -20.0 | 65.0 |
| 2-1-m1E03-2-0247 | C42 H39 N5 O5 S2 | 757.23926 | 32.4 | 44.4 |
| 2-1-m1E03-2-0248 | C41 H36 F N7 O5 S | 757.24827 | -3.8 | -8.5 |
| 2-1-m1E03-2-0249 | C39 H43 N5 O7 S2 | 757.26039 | -13.8 | 22.0 |
| 2-1-m1E03-2-0250 | C36 H34 N6 O7 S3 | 758.16511 | 0.4 | 7.9 |
| 2-1-m1E03-2-0251 | C36 H34 N6 O7 S3 | 758.16511 | 57.6 | -20.8 |
| 2-1-m1E03-2-0252 | C38 H32 F2 N4 O7 S2 | 758.16805 | 0.4 | 7.9 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0253 | C43 H42 N4 O5 S2 | 758.25966 | 0.0 | -22.0 |
| 2-1-m1E03-2-0254 | C43 H42 N4 O5 S2 | 758.25966 | 0.0 | 0.0 |
| 2-1-m1E03-2-0255 | C42 H42 N6 O6 S | 758.28865 | 0.7 | -4.4 |
| 2-1-m1E03-2-0256 | C44 H46 N4 O6 S | 758.31381 | 0.0 | 0.0 |
| 2-1-m1E03-2-0257 | C35 H33 N7 O7 S3 | 759.16036 | 0.0 | 7.5 |
| 2-1-m1E03-2-0258 | C38 H32 F3 N5 O5 S2 | 759.17970 | 0.0 | 0.0 |
| 2-1-m1E03-2-0259 | C37 H34 F N5 O8 S2 | 759.18328 | -23.5 | 6.9 |
| 2-1-m1E03-2-0260 | C42 H41 N5 O5 S2 | 759.25491 | -12.3 | 0.0 |
| 2-1-m1E03-2-0261 | C42 H34 F2 N4 O4 S2 | 760.19895 | 0.0 | 11.0 |
| 2-1-m1E03-2-0262 | C41 H36 N4 O7 S2 | 760.20254 | -7.0 | -4.9 |
| 2-1-m1E03-2-0263 | C39 H38 F2 N4 O6 S2 | 760.22008 | -169.5 | 0.0 |
| 2-1-m1E03-2-0264 | C42 H40 N4 O6 S2 | 760.23893 | 0.0 | 0.0 |
| 2-1-m1E03-2-0265 | C42 H40 N4 O6 S2 | 760.23893 | 52.0 | -41.8 |
| 2-1-m1E03-2-0266 | C42 H40 N4 O6 S2 | 760.23893 | 5.0 | 8.6 |
| 2-1-m1E03-2-0267 | C42 H40 N4 O6 S2 | 760.23893 | 0.0 | 0.0 |
| 2-1-m1E03-2-0268 | C41 H40 N6 O7 S | 760.26792 | -10.3 | 2.6 |
| 2-1-m1E03-2-0269 | C40 H35 N5 O7 S2 | 761.19779 | -11.8 | -12.2 |
| 2-1-m1E03-2-0270 | C40 H35 N5 O7 S2 | 761.19779 | -72.4 | 25.6 |
| 2-1-m1E03-2-0271 | C41 H36 F N5 O5 S2 | 761.21419 | -0.8 | 0.0 |
| 2-1-m1E03-2-0272 | C41 H39 N5 O6 S2 | 761.23418 | 7.5 | -12.1 |
| 2-1-m1E03-2-0273 | C41 H39 N5 O6 S2 | 761.23418 | 0.0 | 0.0 |
| 2-1-m1E03-2-0274 | C41 H39 N5 O6 S2 | 761.23418 | 0.0 | 0.0 |
| 2-1-m1E03-2-0275 | C38 H40 F N5 O7 S2 | 761.23532 | 7.0 | -5.6 |
| 2-1-m1E03-2-0276 | C45 H39 N5 O5 S | 761.26719 | 0.0 | 0.0 |
| 2-1-m1E03-2-0277 | C42 H40 F N5 O6 S | 761.26833 | -12.3 | -7.9 |
| 2-1-m1E03-2-0278 | C43 H47 N5 O6 S | 761.32470 | 11.4 | -6.5 |
| 2-1-m1E03-2-0279 | C37 H33 F3 N6 O5 S2 | 762.19059 | -35.5 | 6.1 |
| 2-1-m1E03-2-0280 | C39 H34 N6 O7 S2 | 762.19304 | -16.6 | -4.4 |
| 2-1-m1E03-2-0281 | C39 H34 N6 O7 S2 | 762.19304 | -0.6 | 9.0 |
| 2-1-m1E03-2-0282 | C41 H38 N4 O7 S2 | 762.21819 | -32.7 | 12.5 |
| 2-1-m1E03-2-0283 | C41 H38 N4 O7 S2 | 762.21819 | -32.9 | 41.6 |
| 2-1-m1E03-2-0284 | C40 H38 N6 O6 S2 | 762.22942 | -76.2 | -69.3 |
| 2-1-m1E03-2-0285 | C42 H39 F N4 O5 S2 | 762.23459 | -21.6 | -27.6 |
| 2-1-m1E03-2-0286 | C40 H37 N5 O7 S2 | 763.21344 | 3.6 | -2.6 |
| 2-1-m1E03-2-0287 | C41 H38 F N5 O7 S | 763.24760 | 0.0 | 0.0 |
| 2-1-m1E03-2-0288 | C41 H41 N5 O6 S2 | 763.24983 | 9.0 | -3.2 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0289 | C40 H36 N4 O8 S2 | 764.19746 | 1.2 | -12.4 |
| 2-1-m1E03-2-0290 | C39 H36 N6 O7 S2 | 764.20869 | 0.0 | -29.1 |
| 2-1-m1E03-2-0291 | C41 H37 F N4 O6 S2 | 764.21385 | -3.5 | 0.9 |
| 2-1-m1E03-2-0292 | C41 H37 F N4 O6 S2 | 764.21385 | 0.0 | 0.0 |
| 2-1-m1E03-2-0293 | C42 H35 F3 N4 O5 S | 764.22803 | 0.0 | -22.2 |
| 2-1-m1E03-2-0294 | C40 H40 N6 O6 S2 | 764.24507 | 0.0 | 0.0 |
| 2-1-mE03-2-0295 | C45 H37 FN4 O5 S | 764.24687 | 5.9 | -2.8 |
| 2-1-m1E03-2-0296 | C43 H39 F3 N4 O4 S | 764.26441 | -35.7 | -3.6 |
| 2-1-m1E03-2-0297 | C43 H42 F2 N4 O5 S | 764.28440 | 6.7 | -10.6 |
| 2-1-m1E03-2-0298 | C40 H36 F N5 O6 S2 | 765.20910 | 0.0 | 0.0 |
| 2-1-m1E03-2-0299 | C40 H36 F N5 O6 S2 | 765.20910 | 0.0 | 0.0 |
| 2-1-m1E03-2-0300 | C41 H34 F3 N5 O5 S | 765.22327 | -15.6 | 0.0 |
| 2-1-m1E03-2-0301 | C40 H39 N5 O7 S2 | 765.22909 | -14.7 | -3.8 |
| 2-1-m1E03-2-0302 | C40 H39 N5 O7 S2 | 765.22909 | -0.6 | -9.6 |
| 2-1-m1E03-2-0303 | C40 H39 N5 O7 S2 | 765.22909 | 1.2 | 3.8 |
| 2-1-m1E03-2-0304 | C42 H44 F N5 O6 S | 765.29963 | -2.9 | 3.8 |
| 2-1-m1E03-2-0305 | C39 H34 N4 O7 S3 | 766.15896 | -19.8 | -2.1 |
| 2-1-m1E03-2-0306 | C40 H38 N4 O6 S3 | 766.19535 | -14.1 | 0.0 |
| 2-1-m1E03-2-0307 | C39 H38 N6 O7 S2 | 766.22434 | -5.1 | -0.6 |
| 2-1-m1E03-2-0308 | C39 H38 N6 O7 S2 | 766.22434 | 5.7 | -2.3 |
| 2-1-m1E03-2-0309 | C39 H38 N6 O7 S2 | 766.22434 | 4.2 | -2.4 |
| 2-1-m1E03-2-0310 | C40 H36 F2 N6 O6 S | 766.23851 | 0.0 | 0.0 |
| 2-1-m1E03-2-0311 | C42 H37 F3 N4 O5 S | 766.24368 | 0.0 | 0.0 |
| 2-1-m1E03-2-0312 | C38 H33 N5 O7 S3 | 767.15421 | -15.3 | -8.1 |
| 2-1-m1E03-2-0313 | C39 H37 N5 O6 S3 | 767.19060 | -0.5 | -8.1 |
| 2-1-m 1 E03-2-0314 | C38 H37 N7 O7 S2 | 767.21959 | 11.9 | 14.8 |
| 2-1-m1E03-2-0315 | C38 H37 N7 O7 S2 | 767.21959 | -4.3 | 5.0 |
| 2-1-m1E03-2-0316 | C43 H41 N7 O5 S | 767.28899 | 23.3 | 53.3 |
| 2-1-m1E03-2-0317 | C39 H33 F N4 O8 S2 | 768.17238 | -42.9 | 12.2 |
| 2-1-m1E03-2-0318 | C39 H36 N4 O7 S3 | 768.17461 | 0.0 | 0.0 |
| 2-1-m1E03-2-0319 | C40 H34 F2 N4 O6 S2 | 768.18878 | -7.0 | 1.2 |
| 2-1-m1E03-2-0320 | C40 H34 F2 N4 O6 S2 | 768.18878 | 0.0 | 0.0 |
| 2-1-m1E03-2-0321 | C38 H35 N5 O7 S3 | 769.16986 | -6.6 | 59.2 |
| 2-1-m1E03-2-0322 | C39 H33 F2 N5 O6 S2 | 769.18403 | -9.5 | 0.0 |
| 2-1-m1E03-2-0323 | C38 H35 N5 O9 S2 | 769.18762 | -10.8 | -10.5 |
| 2-1-m1E03-2-0324 | C39 H36 F N5 O7 S2 | 769.20402 | -4.3 | -3.9 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0325 | C39 H36 F N5 O7 S2 | 769.20402 | 2.1 | 16.3 |
| 2-1-m1E03-2-0326 | C40 H34 F3 N5 O6 S | 769.21819 | 12.6 | 35.9 |
| 2-1-m1E03-2-0327 | C39 H39 N5 O8 S2 | 769.22400 | 6.1 | 0.0 |
| 2-1-m1E03-2-0328 | C41 H38 F3 N5 O5 S | 769.25457 | 14.0 | 12.7 |
| 2-1-m1E03-2-0329 | C42 H39 N7 O6 S | 769.26825 | 0.0 | 17.9 |
| 2-1-m1E03-2-0330 | C38 H32 F2 N6 O6 S2 | 770.17928 | 2.6 | -9.5 |
| 2-1-m1E03-2-0331 | C37 H34 N6 O9 S2 | 770.18287 | -5.9 | -13.2 |
| 2-1-m1E03-2-0332 | C40 H33 F3 N4 O5 S2 | 770.18445 | -7.1 | -13.2 |
| 2-1-m1E03-2-0333 | C38 H35 F N6 O7 S2 | 770.19927 | -11.7 | 2.6 |
| 2-1-m1E03-2-0334 | C40 H33 F3 N4 O7 S | 770.20220 | -11.7 | 2.6 |
| 2-1-m1E03-2-0335 | C46 H38 N6 O4 S | 770.26752 | 116.2 | 145.5 |
| 2-1-m1E03-2-0336 | C38 H37 N5 O7 S3 | 771.18551 | -16.2 | -9.2 |
| 2-1-m1E03-2-0337 | C37 H34 F N7 O7 S2 | 771.19452 | 0.0 | 0.0 |
| 2-1-m1E03-2-0338 | C38 H37 N5 O9 S2 | 771.20327 | -3.0 | -2.9 |
| 2-1-m1E03-2-0339 | C40 H36 F3 N5 O6 S | 771.23384 | -73.9 | 0.0 |
| 2-1-m1E03-2-0340 | C43 H41 N5 O5 S2 | 771.25491 | 0.0 | 0.0 |
| 2-1-m1E03-2-0341 | C40 H45 N5 O7 S2 | 771.27604 | 43.7 | 25.1 |
| 2-1-m1E03-2-0342 | C37 H36 N6 O7 S3 | 772.18076 | 33.3 | 8.8 |
| 2-1-m1E03-2-0343 | C42 H36 N4 O7 S2 | 772.20254 | -1.4 | 12.4 |
| 2-1-m1E03-2-0344 | C41 H36 N6 O6 S2 | 772.21377 | -4.1 | 3.7 |
| 2-1-m1E03-2-0345 | C41 H33 F5 N4 O4 S | 772.21427 | -4.1 | 2.5 |
| 2-1-m1E03-2-0346 | C39 H35 F3 N6 O6 S | 772.22909 | 9.2 | 2.6 |
| 2-1-m1E03-2-0347 | C44 H44 N4 O5 S2 | 772.27531 | 27.4 | 0.0 |
| 2-1-m1E03-2-0348 | C44 H44 N4 O5 S2 | 772.27531 | 0.0 | 0.0 |
| 2-1-m1E03-2-0349 | C44 H44 N4 O5 S2 | 772.27531 | -5.3 | 9.7 |
| 2-1-m1E03-2-0350 | C40 H35 N7 O6 S2 | 773.20902 | 7.3 | 1.4 |
| 2-1-m1E03-2-0351 | C39 H34 F3 N5 O7 S | 773.21310 | -0.8 | -6.3 |
| 2-1-m1E03-2-0352 | C38 H34 F3 N7 O6 S | 773.22434 | 0.0 | 0.0 |
| 2-1-m1E03-2-0353 | C40 H35 F4 N5 O5 S | 773.22950 | -46.5 | -31.8 |
| 2-1-m1E03-2-0354 | C42 H39 N5 O6 S2 | 773.23418 | -0.1 | 1.5 |
| 2-1-m1E03-2-0355 | C39 H43 N5 O8 S2 | 773.25530 | 0.0 | -42.9 |
| 2-1-m1E03-2-0356 | C43 H43 N5 O5 S2 | 773.27056 | 0.0 | 0.0 |
| 2-1-m1E03-2-0357 | C38 H32 F2 N4 O6 S3 | 774.14520 | -13.1 | -16.0 |
| 2-1-m1E03-2-0358 | C42 H38 N4 O7 S2 | 774.21819 | 30.3 | 12.7 |
| 2-1-m1E03-2-0359 | C46 H38 N4 O4 S2 | 774.23345 | -4.4 | 5.5 |
| 2-1-m1E03-2-0360 | C43 H42 N4 O6 S2 | 774.25458 | 0.0 | 0.0 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0361 | C43 H42 N4 O6 S2 | 774.25458 | 0.0 | 0.0 |
| 2-1-m1E03-2-0362 | C43 H42 N4 O6 S2 | 774.25458 | 0.0 | 0.0 |
| 2-1-m1E03-2-0363 | C43 H42 N4 O6 S2 | 774.25458 | -30.8 | 0.0 |
| 2-1-m1E03-2-0364 | C37 H31 F2 N5 O6 S3 | 775.14045 | 0.0 | 0.0 |
| 2-1-m1E03-2-0365 | C37 H34 F N5 O7 S3 | 775.16044 | 19.0 | 11.9 |
| 2-1-m1E03-2-0366 | C41 H37 N5 O7 S2 | 775.21344 | -3.6 | -5.9 |
| 2-1-m1E03-2-0367 | C41 H37 N5 O7 S2 | 775.21344 | 0.0 | -12.6 |
| 2-1-m1E03-2-0368 | C41 H35 F2 N7 O5 S | 775.23884 | 0.0 | 0.0 |
| 2-1-m1E03-2-0369 | C42 H41 N5 O6 S2 | 775.24983 | 5.7 | -3.3 |
| 2-1-m1E03-2-0370 | C44 H49 N5 O6 S | 775.34036 | 8.8 | -15.2 |
| 2-1-m1E03-2-0371 | C36 H33 F N6 O7 S3 | 776.15569 | 0.0 | 0.0 |
| 2-1-m1E03-2-0372 | C40 H36 N6 O7 S2 | 776.20869 | -18.9 | -16.7 |
| 2-1-m1E03-2-0373 | C42 H40 N4 O7 S2 | 776.23384 | -28.2 | -0.1 |
| 2-1-m1E03-2-0374 | C42 H40 N4 O7 S2 | 776.23384 | -3.2 | 4.4 |
| 2-1-m1E03-2-0375 | C43 H41 F N4 O5 S2 | 776.25024 | 0.0 | 0.0 |
| 2-1-m1E03-2-0376 | C37 H33 F2 N5 O8 S2 | 777.17386 | 0.0 | 0.0 |
| 2-1-m1E03-2-0377 | C41 H39 N5 O7 S2 | 777.22909 | -1.0 | -3.8 |
| 2-1-m1E03-2-0378 | C41 H39 N5 O7 S2 | 777.22909 | 0.0 | 0.0 |
| 2-1-m1E03-2-0379 | C42 H43 N5 O6 S2 | 777.26548 | -3.6 | 8.3 |
| 2-1-m1E03-2-0380 | C42 H43 N5 O6 S2 | 777.26548 | 22.0 | -9.8 |
| 2-1-m1E03-2-0381 | C43 H47 N5 O7 S | 777.31962 | 7.4 | 9.6 |
| 2-1-m1E03-2-0382 | C39 H34 N6 O6 S3 | 778.17019 | 7.2 | 14.4 |
| 2-1-m1E03-2-0383 | C37 H33 F3 N6 O6 S2 | 778.18551 | 0.0 | 0.0 |
| 2-1-m1E03-2-0384 | C41 H35 F N4 O7 S2 | 778.19312 | 5.4 | 7.8 |
| 2-1-m1E03-2-0385 | C41 H35 FN4 O7 S2 | 778.19312 | 0.0 | 0.0 |
| 2-1-m1E03-2-0386 | C41 H38 N4 O8 S2 | 778.21311 | 0.0 | 0.0 |
| 2-1-m1E03-2-0387 | C42 H39 F N4 O6 S2 | 778.22950 | 1.4 | 21.6 |
| 2-1-m1E03-2-0388 | C42 H39 F N4 O6 S2 | 778.22950 | 0.0 | 0.0 |
| 2-1-m1E03-2-0389 | C41 H42 N6 O6 S2 | 778.26072 | -21.9 | 0.0 |
| 2-1-m1E03-2-0390 | C47 H46 N4 O5 S | 778.31889 | 0.0 | 0.0 |
| 2-1-m1E03-2-0391 | C40 H34 F N5 O7 S2 | 779.18837 | -24.9 | -12.3 |
| 2-1-m1E03-2-0392 | C40 H34 F N5 O7 S2 | 779.18837 | 0.0 | 0.0 |
| 2-1-m1E03-2-0393 | C41 H35 F2 N5 O5 S2 | 779.20477 | -9.9 | -3.2 |
| 2-1-m1E03-2-0394 | C40 H37 N5 O8 S2 | 779.20835 | -9.8 | -3.4 |
| 2-1-m1E03-2-0395 | C38 H39 F2 N5 O7 S2 | 779.22590 | 2.6 | 12.4 |
| 2-1-m1E03-2-0396 | C42 H36 F3 N5 O5 S | 779.23892 | 20.4 | -62.6 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0397 | C41 H41 N5 O7 S2 | 779.24474 | -3.8 | 0.8 |
| 2-1-m1E03-2-0398 | C41 H41 N5 O7 S2 | 779.24474 | 9.1 | -4.2 |
| 2-1-m1E03-2-0399 | C41 H41 N5 O7 S2 | 779.24474 | 0.0 | 18.5 |
| 2-1-m1E03-2-0400 | C41 H41 N5 O7 S2 | 779.24474 | 0.0 | 0.0 |
| 2-1-m1E03-2-0401 | C40 H36 N4 O7 S3 | 780.17461 | -19.3 | -1.7 |
| 2-1-m1E03-2-0402 | C40 H36 N4 O9 S2 | 780.19237 | -57.0 | -7.6 |
| 2-1-m1E03-2-0403 | C39 H36 N6 O8 S2 | 780.20360 | -4.5 | -20.9 |
| 2-1-m1E03-2-0404 | C39 H36 N6 O8 S2 | 780.20360 | -8.0 | -8.0 |
| 2-1-m1E03-2-0405 | C42 H35 F3 N4 O6 S | 780.22294 | -19.5 | -36.8 |
| 2-1-m1E03-2-0406 | C42 H38 F2 N4 O5 S2 | 780.22517 | -43.8 | -27.9 |
| 2-1-m1E03-2-0407 | C40 H40 N6 O7 S2 | 780.23999 | 5.8 | -1.5 |
| 2-1-m1E03-2-0408 | C40 H40 N6 O7 S2 | 780.23999 | -2.9 | 1.2 |
| 2-1-m1E03-2-0409 | C40 H40 N6 O7 S2 | 780.23999 | -15.9 | 5.8 |
| 2-1-m1E03-2-0410 | C38 H35 N7 O8 S2 | 781.19885 | -4.6 | 0.8 |
| 2-1-m1E03-2-0411 | C41 H34 F3 N5 O6 S | 781.21819 | -6.0 | 0.0 |
| 2-1-m1E03-2-0412 | C40 H39 N5 O8 S2 | 781.22400 | 24.0 | -5.2 |
| 2-1-m1E03-2-0413 | C40 H39 N5 O8 S2 | 781.22400 | -5.7 | 8.4 |
| 2-1-m1E03-2-0414 | C39 H39 N7 O7 S2 | 781.23524 | 0.0 | 0.0 |
| 2-1-m1E03-2-0415 | C41 H40 F N5 O6 S2 | 781.24040 | 0.0 | 0.0 |
| 2-1-m1E03-2-0416 | C41 H36 F2 N4 O6 S2 | 782.20443 | 0.2 | -1.2 |
| 2-1-m1E03-2-0417 | C41 H36 F2 N4 O6 S2 | 782.20443 | -5.4 | -9.3 |
| 2-1-m1E03-2-0418 | C42 H34 F4 N4 O5 S | 782.21860 | 14.0 | 15.3 |
| 2-1-m1E03-2-0419 | C39 H38 N6 O8 S2 | 782.21925 | -28.2 | -71.7 |
| 2-1-m1E03-2-0420 | C44 H38 N4 O6 S2 | 782.22328 | 16.3 | 12.2 |
| 2-1-m1E03-2-0421 | C44 H38 N4 O6 S2 | 782.22328 | -17.6 | 3.8 |
| 2-1-m1E03-2-0422 | C40 H35 F2 N5 O6 S2 | 783.19968 | -4.0 | 0.0 |
| 2-1-m1E03-2-0423 | C39 H37 N5 O9 S2 | 783.20327 | -12.1 | -5.1 |
| 2-1-m1E03-2-0424 | C38 H37 N7 O8 S2 | 783.21450 | -32.4 | 0.0 |
| 2-1-m1E03-2-0425 | C43 H37 N5 O6 S2 | 783.21853 | -8.5 | 3.0 |
| 2-1-m1E03-2-0426 | C40 H38 F N5 O7 S2 | 783.21967 | -8.9 | -2.0 |
| 2-1-m1E03-2-0427 | C40 H38 F N5 O7 S2 | 783.21967 | 2.3 | -1.0 |
| 2-1-m1E03-2-0428 | C41 H36 F3 N5 O6 S | 783.23384 | -14.3 | -3.3 |
| 2-1-m1E03-2-0429 | C42 H40 F3 N5 O5 S | 783.27022 | -45.9 | -12.9 |
| 2-1-m1E03-2-0430 | C42 H43 F2 N5 O6 S | 783.29021 | -37.7 | 166.3 |
| 2-1-m1E03-2-0431 | C39 H33 F N4 O7 S3 | 784.14954 | 0.0 | 0.0 |
| 2-1-m1E03-2-0432 | C42 H36 N6 O6 S2 | 784.21377 | 0.0 | 0.0 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0433 | C42 H36 N6 O6 S2 | 784.21377 | 0.0 | 0.0 |
| 2-1-m1E03-2-0434 | C39 H37 F N6 O7 S2 | 784.21492 | 0.0 | 0.0 |
| 2-1-m1E03-2-0435 | C39 H37 F N6 O7 S2 | 784.21492 | 0.0 | 0.0 |
| 2-1-m1E03-2-0436 | C40 H35 F3 N6 O6 S | 784.22909 | 25.0 | -0.7 |
| 2-1-m1E03-2-0437 | C38 H35 N5 O8 S3 | 785.16478 | -4.2 | 9.7 |
| 2-1-m1E03-2-0438 | C39 H39 N5 O7 S3 | 785.20116 | -5.7 | 8.3 |
| 2-1-m1E03-2-0439 | C41 H35 N7 O6 S2 | 785.20902 | 10.9 | -14.5 |
| 2-1-m1E03-2-0440 | C41 H38 F3 N5 O6 S | 785.24949 | -28.6 | 10.1 |
| 2-1-m1E03-2-0441 | C38 H34 N4 O9 S3 | 786.14879 | -38.3 | 6.4 |
| 2-1-m1E03-2-0442 | C37 H34 N6 O8 S3 | 786.16002 | -7.5 | -5.0 |
| 2-1-m1E03-2-0443 | C39 H32 F2 N4 O8 S2 | 786.16296 | -6.3 | -4.7 |
| 2-1-m1E03-2-0444 | C40 H33 F3 N4 O6 S2 | 786.17936 | 20.2 | 12.8 |
| 2-1-m1E03-2-0445 | C38 H38 N6 O7 S3 | 786.19641 | 0.0 | 0.0 |
| 2-1-m1E03-2-0446 | C43 H38 N4 O7 S2 | 786.21819 | -4.8 | 14.5 |
| 2-1-m1E03-2-0447 | C45 H37 F3 N4 O4 S | 786.24876 | 29.2 | 29.2 |
| 2-1-m1E03-2-0448 | C45 H46 N4 O5 S2 | 786.29096 | 0.0 | 0.0 |
| 2-1-m1E03-2-0449 | C45 H46 N4 O5 S2 | 786.29096 | 0.0 | 0.0 |
| 2-1-m1E03-2-0450 | C37 H33 N5 O9 S3 | 787.14404 | -12.0 | -14.0 |
| 2-1-m1E03-2-0451 | C38 H34 F N5 O9 S2 | 787.17820 | -18.7 | 0.3 |
| 2-1-m1E03-2-0452 | C38 H37 N5 O8 S3 | 787.18043 | -18.7 | 1.1 |
| 2-1-m1E03-2-0453 | C39 H35 F2 N5 O7 S2 | 787.19460 | -3.8 | -7.0 |
| 2-1-m1E03-2-0454 | C39 H35 F2 N5 O7 S2 | 787.19460 | 0.0 | -4.3 |
| 2-1-m1E03-2-0455 | C42 H37 N5 O7 S2 | 787.21344 | -6.8 | -0.7 |
| 2-1-m1E03-2-0456 | C41 H37 N7 O6 S2 | 787.22467 | -8.7 | -1.3 |
| 2-1-m1E03-2-0457 | C37 H36 N6 O8 S3 | 788.17567 | 0.0 | 0.0 |
| 2-1-m1E03-2-0458 | C42 H36 N4 O6 S3 | 788.17970 | -36.0 | 15.1 |
| 2-1-m1E03-2-0459 | C42 H36 N4 O6 S3 | 788.17970 | 0.0 | 0.0 |
| 2-1-m1E03-2-0460 | C42 H36 N4 O6 S3 | 788.17970 | 0.0 | 0.0 |
| 2-1-m1E03-2-0461 | C38 H34 F2 N6 O7 S2 | 788.18985 | -97.6 | -11.9 |
| 2-1-m1E03-2-0462 | C43 H40 N4 O7 S2 | 788.23384 | 24.4 | -23.8 |
| 2-1-m1E03-2-0463 | C43 H35 F3 N6 O4 S | 788.23926 | 0.0 | 0.0 |
| 2-1-m1E03-2-0464 | C44 H44 N4 O6 S2 | 788.27023 | 0.0 | 0.0 |
| 2-1-m1E03-2-0465 | C44 H44 N4 O6 S2 | 788.27023 | 0.0 | 0.0 |
| 2-1-m1E03-2-0466 | C41 H35 N5 O6 S3 | 789.17495 | -6.6 | 4.2 |
| 2-1-m1E03-2-0467 | C41 H35 N5 O6 S3 | 789.17495 | 6.5 | 38.6 |
| 2-1-m1E03-2-0468 | C41 H35 N5 O6 S3 | 789.17495 | 10.2 | 3.7 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0469 | C37 H33 F2 N7 O7 S2 | 789.18509 | -3.8 | 10.6 |
| 2-1-m1E03-2-0470 | C39 H34 F3 N5 O6 S2 | 789.19026 | -18.1 | 8.6 |
| 2-1-m1E03-2-0471 | C39 H34 F3 N5 O8 S | 789.20802 | 16.9 | -14.2 |
| 2-1-m1E03-2-0472 | C42 H39 N5 O7 S2 | 789.22909 | 0.0 | -38.2 |
| 2-1-m1E03-2-0473 | C42 H39 N5 O7 S2 | 789.22909 | -15.5 | 19.9 |
| 2-1-m1E03-2-0474 | C39 H33 F3 N4 O7 S2 | 790.17428 | 0.0 | 0.0 |
| 2-1-m1E03-2-0475 | C41 H35 F N6 O6 S2 | 790.20435 | 0.0 | 0.0 |
| 2-1-m1E03-2-0476 | C42 H38 N4 O8 S2 | 790.21311 | 19.9 | 12.9 |
| 2-1-m1E03-2-0477 | C43 H42 N4 O7 S2 | 790.24949 | 0.0 | 0.0 |
| 2-1-m1E03-2-0478 | C43 H42 N4 O7 S2 | 790.24949 | -45.4 | -29.2 |
| 2-1-m1E03-2-0479 | C44 H43 F N4 O5 S2 | 790.26589 | -2.5 | -15.2 |
| 2-1-m1E03-2-0480 | C41 H37 N5 O8 S2 | 791.20835 | -169.7 | 0.0 |
| 2-1-m1E03-2-0481 | C40 H34 F5 N5 O5 S | 791.22008 | 1.7 | -8.6 |
| 2-1-m1E03-2-0482 | C43 H45 N5 O6 S2 | 791.28113 | 0.0 | 0.0 |
| 2-1-m1E03-2-0483 | C43 H45 N5 O6 S2 | 791.28113 | 0.0 | 0.0 |
| 2-1-m1E03-2-0484 | C43 H45 N5 O6 S2 | 791.28113 | -20.7 | -13.4 |
| 2-1-m1E03-2-0485 | C43 H35 F3 N4 O4 S2 | 792.20518 | 0.0 | 0.0 |
| 2-1-m1E03-2-0486 | C42 H37 F N4 O7 S2 | 792.20877 | 0.0 | 0.0 |
| 2-1-m1E03-2-0487 | C40 H39 F3 N4 O6 S2 | 792.22631 | 0.0 | 0.0 |
| 2-1-m1E03-2-0488 | C43 H41 F N4 O6 S2 | 792.24515 | 0.0 | 0.0 |
| 2-1-m1E03-2-0489 | C43 H44 N4 O7 S2 | 792.26514 | 0.0 | 0.0 |
| 2-1-m1E03-2-0490 | C42 H44 N6 O6 S2 | 792.27637 | 0.0 | 0.0 |
| 2-1-m1E03-2-0491 | C37 H33 F2 N5 O7 S3 | 793.15102 | -5.5 | 2.6 |
| 2-1-m1E03-2-0492 | C41 H36 F N5 O7 S2 | 793.20402 | -3.5 | -9.5 |
| 2-1-m1E03-2-0493 | C41 H39 N5 O8 S2 | 793.22400 | 0.1 | 13.2 |
| 2-1-m1E03-2-0494 | C42 H43 N5 O7 S2 | 793.26039 | -16.4 | -4.8 |
| 2-1-m1E03-2-0495 | C42 H43 N5 O7 S2 | 793.26039 | -34.3 | 18.6 |
| 2-1-m1E03-2-0496 | C42 H43 N5 O7 S2 | 793.26039 | 12.1 | 9.4 |
| 2-1-m1E03-2-0497 | C42 H43 N5 O7 S2 | 793.26039 | 36.9 | 27.4 |
| 2-1-m1E03-2-0498 | C36 H32 F2 N6 O7 S3 | 794.14627 | -11.1 | -2.9 |
| 2-1-m1E03-2-0499 | C41 H38 N4 O7 S3 | 794.19026 | 0.0 | 0.0 |
| 2-1-m1E03-2-0500 | C40 H38 N6 O8 S2 | 794.21925 | -11.6 | 0.0 |
| 2-1-m1E03-2-0501 | C40 H38 N6 O8 S2 | 794.21925 | -14.0 | -6.5 |
| 2-1-m1E03-2-0502 | C42 H39 F N4 O7 S2 | 794.22442 | 0.0 | 0.0 |
| 2-1-m1E03-2-0503 | C42 H39 F N4 O7 S2 | 794.22442 | 20.3 | 8.9 |
| 2-1-m1E03-2-0504 | C43 H40 F2 N4 O5 S2 | 794.24082 | 18.5 | 8.9 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0505 | C41 H42 N6 O7 S2 | 794.25564 | -4.7 | 12.1 |
| 2-1-m1E03-2-0506 | C46 H42 N4 O5 S2 | 794.25966 | -5.0 | 14.8 |
| 2-1-m1E03-2-0507 | C39 H37 N7 O8 S2 | 795.21450 | -1.4 | 6.9 |
| 2-1-m1E03-2-0508 | C42 H36 F3 N5 O6 S | 795.23384 | -10.6 | -31.5 |
| 2-1-m1E03-2-0509 | C41 H41 N5 O8 S2 | 795.23965 | -8.2 | -4.0 |
| 2-1-m1E03-2-0510 | C41 H41 N5 O8 S2 | 795.23965 | -0.1 | 6.4 |
| 2-1-m1E03-2-0511 | C42 H42 F N5 O6 S2 | 795.25605 | 21.6 | 39.0 |
| 2-1-m1E03-2-0512 | C40 H36 N4 O8 S3 | 796.16953 | 8.3 | -4.9 |
| 2-1-m1E03-2-0513 | C40 H36 N4 O8 S3 | 796.16953 | 0.0 | 0.0 |
| 2-1-m1E03-2-0514 | C41 H34 F2 N4 O7 S2 | 796.18370 | 2.0 | 12.5 |
| 2-1-m1E03-2-0515 | C41 H34 F2 N4 O7 S2 | 796.18370 | -11.6 | 7.4 |
| 2-1-m1E03-2-0516 | C42 H38 F2 N4 O6 S2 | 796.22008 | 2.9 | -3.9 |
| 2-1-m1E03-2-0517 | C42 H36 N8 O5 S2 | 796.22501 | 10.7 | -38.1 |
| 2-1-m1E03-2-0518 | C40 H40 N6 O8 S2 | 796.23490 | 0.0 | 0.0 |
| 2-1-m1E03-2-0519 | C40 H40 N6 O8 S2 | 796.23490 | 0.0 | 0.0 |
| 2-1-m1E03-2-0520 | C45 H40 N4 O6 S2 | 796.23893 | -4.1 | 16.3 |
| 2-1-m1E03-2-0521 | C44 H43 F3 N4 OS S | 796.29063 | 0.0 | 0.0 |
| 2-1-m1E03-2-0522 | C39 H35 N5 O8 S3 | 797.16478 | -23.3 | 16.8 |
| 2-1-m1E03-2-0523 | C40 H33 F2 N5 O7 S2 | 797.17895 | -16.7 | 12.3 |
| 2-1-m1E03-2-0524 | C40 H36 F N5 O8 S2 | 797.19893 | -3.3 | -8.6 |
| 2-1-m1E03-2-0525 | C40 H36 F N5 O8 S2 | 797.19893 | 8.6 | 1.8 |
| 2-1-m1E03-2-0526 | C40 H39 N5 O9 S2 | 797.21892 | -32.6 | -39.7 |
| 2-1-m1E03-2-0527 | C44 H39 N5 O6 S2 | 797.23418 | 20.3 | 19.9 |
| 2-1-m1E03-2-0528 | C41 H40 F N5 O7 S2 | 797.23532 | 24.0 | 11.1 |
| 2-1-m1E03-2-0529 | C41 H40 F N5 O7 S2 | 797.23532 | 0.0 | 0.0 |
| 2-1-m1E03-2-0530 | C38 H34 N6 O8 S3 | 798.16002 | -1.6 | -34.4 |
| 2-1-m1E03-2-0531 | C39 H35 F N6 O8 S2 | 798.19418 | -7.8 | -4.0 |
| 2-1-m1E03-2-0532 | C39 H35 F N6 O8 S2 | 798.19418 | -6.6 | -10.0 |
| 2-1-m1E03-2-0533 | C42 H34 F4 N4 O6 S | 798.21352 | -6.9 | -2.9 |
| 2-1-m1E03-2-0534 | C43 H38 N6 O6 S2 | 798.22942 | 0.0 | 37.6 |
| 2-1-m1E03-2-0535 | C41 H37 F3 N6 O6 S | 798.24474 | 10.6 | -11.1 |
| 2-1-m1E03-2-0536 | C39 H37 N5 O8 S3 | 799.18043 | -8.4 | -12.2 |
| 2-1-m1E03-2-0537 | C39 H37 N5 O10 S2 | 799.19818 | -12.3 | -3.0 |
| 2-1-m1E03-2-0538 | C41 H36 F3 N5 O7 S | 799.22875 | 11.1 | 11.7 |
| 2-1-m1E03-2-0539 | C41 H39 F2 N5 O6 S2 | 799.23098 | 11.1 | 14.9 |
| 2-1-m1E03-2-0540 | C39 H36 N4 O9 S3 | 800.16444 | -13.2 | 16.4 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0541 | C42 H36 N6 O5 S3 | 800.19093 | 16.1 | 30.1 |
| 2-1-m1E03-2-0542 | C41 H35 F3 N4 O6 S2 | 800.19501 | -2.8 | 0.0 |
| 2-1-m1E03-2-0543 | C41 H35 F3 N4 O6 S2 | 800.19501 | 22.6 | -1.0 |
| 2-1-m1E03-2-0544 | C41 H35 F3 N4 O6 S2 | 800.19501 | 0.0 | 0.0 |
| 2-1-m1E03-2-0545 | C41 H35 F3 N4 O6 S2 | 800.19501 | 0.0 | 0.0 |
| 2-1-m1E03-2-0546 | C43 H36 N4 O8 S2 | 800.19746 | -2.5 | -4.1 |
| 2-1-m1E03-2-0547 | C39 H40 N6 O7 S3 | 800.21206 | 1.6 | 17.8 |
| 2-1-m1E03-2-0548 | C40 H35 F3 N6 O7 S | 800.22400 | 1.8 | 4.7 |
| 2-1-m1E03-2-0549 | C44 H40 N4 O7 S2 | 800.23384 | -0.4 | 19.5 |
| 2-1-m1E03-2-0550 | C45 H44 N4 O6 S2 | 800.27023 | 0.0 | 0.0 |
| 2-1-m1E03-2-0551 | C46 H48 N4 O5 S2 | 800.30661 | 0.0 | 0.0 |
| 2-1-m1E03-2-0552 | C40 H34 F3 N5 O6 S2 | 801.19026 | 1.5 | -0.3 |
| 2-1-m1E03-2-0553 | C40 H34 F3 N5 O6 S2 | 801.19026 | -4.4 | 4.1 |
| 2-1-m1E03-2-0554 | C40 H37 F2 N5 O7 S2 | 801.21025 | -20.8 | -4.0 |
| 2-1-m1E03-2-0555 | C40 H37 F2 N5 O7 S2 | 801.21025 | 9.2 | 31.3 |
| 2-1-m1E03-2-0556 | C41 H35 F4 N5 O6 S | 801.22442 | -16.8 | -7.3 |
| 2-1-m1E03-2-0557 | C44 H43 N5 O6 S2 | 801.26548 | -15.1 | -14.0 |
| 2-1-m1E03-2-0558 | C38 H34 N4 O8 S4 | 802.12595 | -1.3 | 9.9 |
| 2-1-m1E03-2-0559 | C39 H32 F2 N4 O7 S3 | 802.14012 | -26.9 | -19.1 |
| 2-1-m1E03-2-0560 | C39 H33 F3 N6 O6 S2 | 802.18551 | 0.0 | -10.7 |
| 2-1-m1E03-2-0561 | C43 H38 N4 O6 S3 | 802.19535 | -5.5 | 2.1 |
| 2-1-m1E03-2-0562 | C43 H38 N4 O6 S3 | 802.19535 | 0.0 | 0.0 |
| 2-1-m1E03-2-0563 | C42 H35 F N6 O6 S2 | 802.20435 | 0.0 | 0.0 |
| 2-1-m1E03-2-0564 | C39 H36 F2 N6 O7 S2 | 802.20550 | 0.0 | 9.1 |
| 2-1-m1E03-2-0565 | C45 H46 N4 O6 S2 | 802.28588 | 0.0 | 0.0 |
| 2-1-m1E03-2-0566 | C37 H33 N5 O8 S4 | 803.12120 | 16.6 | -18.1 |
| 2-1-m1E03-2-0567 | C38 H34 F N5 O8 S3 | 803.15535 | -3.4 | -1.0 |
| 2-1-m1E03-2-0568 | C42 H37 N5 O6 S3 | 803.19060 | -11.5 | -2.5 |
| 2-1-m1E03-2-0569 | C41 H37 N7 O7 S2 | 803.21959 | -10.8 | 3.9 |
| 2-1-m1E03-2-0570 | C43 H41 N5 O7 S2 | 803.24474 | 4.5 | 27.7 |
| 2-1-m1E03-2-0571 | C38 H33 F N4 O9 S3 | 804.13937 | 0.0 | 0.0 |
| 2-1-m1E03-2-0572 | C41 H36 N6 O6 S3 | 804.18585 | 0.0 | 0.0 |
| 2-1-m1E03-2-0573 | C43 H37 F N4 O7 S2 | 804.20877 | 41.0 | -10.0 |
| 2-1-m1E03-2-0574 | C40 H36 N8 O7 S2 | 804.21484 | -0.3 | 0.0 |
| 2-1-m1E03-2-0575 | C42 H34 F6 N4 O4 S | 804.22050 | 61.4 | 8.4 |
| 2-1-m1E03-2-0576 | C43 H35 F3 N6 O5 S | 804.23417 | -43.9 | -32.6 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0577 | C44 H44 N4 O7 S2 | 804.26514 | -4.1 | 18.2 |
| 2-1-m1E03-2-0578 | C43 H44 N6 O6 S2 | 804.27637 | -6.3 | -4.1 |
| 2-1-m1E03-2-0579 | C37 H35 N5 O10 S3 | 805.15460 | -24.8 | -19.6 |
| 2-1-m1E03-2-0580 | C38 H33 F2 N5 O9 S2 | 805.16878 | -36.4 | -12.6 |
| 2-1-m1E03-2-0581 | C39 H34 F3 N5 O7 S2 | 805.18517 | -4.1 | 8.5 |
| 2-1-m1E03-2-0582 | C42 H39 N5 O8 S2 | 805.22400 | 0.0 | 0.0 |
| 2-1-m1E03-2-0583 | C42 H39 N5 O8 S2 | 805.22400 | -3.7 | -21.9 |
| 2-1-m1E03-2-0584 | C44 H47 N5 O6 S2 | 805.29678 | 47.5 | 0.7 |
| 2-1-m1E03-2-0585 | C44 H47 N5 O6 S2 | 805.29678 | 0.0 | 0.0 |
| 2-1-m1E03-2-0586 | C36 H34 N6 O10 S3 | 806.14985 | -11.0 | -4.0 |
| 2-1-m1E03-2-0587 | C39 H33 F3 N4 O6 S3 | 806.15143 | -11.0 | -4.0 |
| 2-1-m1E03-2-0588 | C39 H33 F3 N4 O8 S2 | 806.16919 | -38.3 | -6.2 |
| 2-1-m1E03-2-0589 | C42 H35 F N4 O6 S3 | 806.17028 | 30.2 | 6.8 |
| 2-1-m1E03-2-0590 | C42 H35 F N4 O6 S3 | 806.17028 | 0.0 | 0.0 |
| 2-1-m1E03-2-0591 | C41 H38 N6 O8 S2 | 806.21925 | -8.3 | 22.1 |
| 2-1-m1E03-2-0592 | C43 H39 F N4 O7 S2 | 806.22442 | -1.8 | 104.0 |
| 2-1-m1E03-2-0593 | C43 H42 N4 O8 S2 | 806.24441 | 0.0 | 0.0 |
| 2-1-m1E03-2-0594 | C44 H46 N4 O7 S2 | 806.28079 | 15.3 | -18.6 |
| 2-1-m1E03-2-0595 | C38 H32 F3 N5 O6 S3 | 807.14668 | -32.0 | 76.6 |
| 2-1-m1E03-2-0596 | C41 H37 N5 O7 S3 | 807.18551 | -0.5 | -5.1 |
| 2-1-m1E03-2-0597 | C41 H37 N5 O7 S3 | 807.18551 | -17.0 | -5.6 |
| 2-1-m1E03-2-0598 | C42 H41 N5 O8 S2 | 807.23965 | -6.7 | -2.7 |
| 2-1-m1E03-2-0599 | C42 H36 F3 N7 O5 S | 807.24507 | -14.0 | -47.2 |
| 2-1-m1E03-2-0600 | C43 H45 N5 O7 S2 | 807.27604 | 0.0 | 9.9 |
| 2-1-m1E03-2-0601 | C43 H45 N5 O7 S2 | 807.27604 | 0.0 | 0.0 |
| 2-1-m1E03-2-0602 | C40 H36 N6 O7 S3 | 808.18076 | -6.0 | -4.3 |
| 2-1-m1E03-2-0603 | C40 H36 N6 O7 S3 | 808.18076 | 4.2 | 5.1 |
| 2-1-m1E03-2-0604 | C40 H36 N6 O7 S3 | 808.18076 | -4.8 | 5.7 |
| 2-1-m1E03-2-0605 | C40 H36 N6 O7 S3 | 808.18076 | 0.0 | 7.3 |
| 2-1-m1E03-2-0606 | C43 H35 F3 N4 O5 S2 | 808.20010 | 0.0 | 0.0 |
| 2-1-m1E03-2-0607 | C42 H37 F N4 O8 S2 | 808.20368 | -5.4 | 3.0 |
| 2-1-m1E03-2-0608 | C40 H39 F3 N4 O7 S2 | 808.22123 | -61.6 | 7.1 |
| 2-1-m1E03-2-0609 | C41 H40 N6 O8 S2 | 808.23490 | -16.9 | -3.8 |
| 2-1-m1E03-2-0610 | C41 H40 N6 O8 S2 | 808.23490 | 5.0 | -4.9 |
| 2-1-m1E03-2-0611 | C44 H42 F2 N4 O5 S2 | 808.25647 | 16.3 | -41.7 |
| 2-1-m1E03-2-0612 | C47 H44 N4 O5 S2 | 808.27531 | 0.0 | 0.0 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0613 | C39 H35 N7 O7 S3 | 809.17601 | -18.6 | 1.2 |
| 2-1-m1E03-2-0614 | C38 H34 F3 N5 O8 S2 | 809.18009 | -23.5 | 3.8 |
| 2-1-m1E03-2-0615 | C41 H39 N5 O9 S2 | 809.21892 | 0.0 | 0.0 |
| 2-1-m1E03-2-0616 | C42 H43 N5 O8 S2 | 809.25530 | -1.8 | 0.0 |
| 2-1-m1E03-2-0617 | C42 H43 N5 O8 S2 | 809.25530 | 9.5 | 4.5 |
| 2-1-m1E03-2-0618 | C43 H44 F N5 O6 S2 | 809.27170 | 0.0 | 0.0 |
| 2-1-m1E03-2-0619 | C38 H34 N8 O7 S3 | 810.17126 | -28.8 | -4.2 |
| 2-1-m1E03-2-0620 | C41 H38 N4 O8 S3 | 810.18518 | 4.4 | 22.0 |
| 2-1-m1E03-2-0621 | C40 H38 N6 O9 S2 | 810.21417 | 0.0 | 0.0 |
| 2-1-m1E03-2-0622 | C45 H38 N4 O7 S2 | 810.21819 | 0.0 | 0.0 |
| 2-1-m1E03-2-0623 | C46 H42 N4 O6 S2 | 810.25458 | -12.9 | 15.6 |
| 2-1-m1E03-2-0624 | C43 H43 F N4 O7 S2 | 810.25572 | 17.1 | 8.2 |
| 2-1-m1E03-2-0625 | C41 H35 F2 N5 O7 S2 | 811.19460 | -22.6 | -6.0 |
| 2-1-m1E03-2-0626 | C42 H36 F3 N5 O5 S2 | 811.21100 | 0.0 | 0.0 |
| 2-1-m1E03-2-0627 | C44 H37 N5 O7 S2 | 811.21344 | -8.4 | -15.7 |
| 2-1-m1E03-2-0628 | C41 H38 F N5 O8 S2 | 811.21458 | -7.4 | -9.6 |
| 2-1-m1E03-2-0629 | C39 H40 F3 N5 O7 S2 | 811.23212 | -10.9 | 3.7 |
| 2-1-m1E03-2-0630 | C42 H42 F N5 O7 S2 | 811.25097 | 19.2 | 0.0 |
| 2-1-m1E03-2-0631 | C42 H45 N5 O8 S2 | 811.27095 | -8.0 | -7.0 |
| 2-1-m1E03-2-0632 | C41 H35 F3 N6 O5 S2 | 812.20624 | -14.9 | 7.5 |
| 2-1-m1E03-2-0633 | C40 H37 F N6 O8 S2 | 812.20983 | 0.0 | -9.0 |
| 2-1-m1E03-2-0634 | C42 H38 F2 N4 O7 S2 | 812.21500 | 0.0 | 0.0 |
| 2-1-m1E03-2-0635 | C43 H39 F3 N4 O5 S2 | 812.23140 | -12.1 | 1.7 |
| 2-1-m1E03-2-0636 | C44 H40 N6 O6 S2 | 812.24507 | 0.0 | -1.0 |
| 2-1-m1E03-2-0637 | C41 H44 N6 O8 S2 | 812.26620 | -4.4 | -2.9 |
| 2-1-m1E03-2-0638 | C44 H43 F3 N4 O6 S | 812.28554 | 0.0 | 0.0 |
| 2-1-m1E03-2-0639 | C40 H39 N5 O8 S3 | 813.19608 | 3.3 | 3.4 |
| 2-1-m1E03-2-0640 | C41 H40 F N5 O8 S2 | 813.23023 | 0.0 | 0.0 |
| 2-1-m1E03-2-0641 | C41 H40 F N5 O8 S2 | 813.23023 | 0.0 | 0.0 |
| 2-1-m1E03-2-0642 | C42 H41 F2 N5 O6 S2 | 813.24663 | 6.7 | -49.1 |
| 2-1-m1E03-2-0643 | C38 H34 N6 O7 S4 | 814.13718 | 8.6 | -3.2 |
| 2-1-m1E03-2-0644 | C40 H35 F N4 O8 S3 | 814.16010 | 0.0 | 0.0 |
| 2-1-m1E03-2-0645 | C41 H33 F3 N4 O7 S2 | 814.17428 | -22.6 | -6.1 |
| 2-1-m1E03-2-0646 | C40 H38 N4 O9 S3 | 814.18009 | 0.0 | 0.0 |
| 2-1-m1E03-2-0647 | C42 H37 F3 N4 O6 S2 | 814.21066 | -15.3 | -1.9 |
| 2-1-m1E03-2-0648 | C42 H37 F3 N4 O6 S2 | 814.21066 | 55.7 | 47.3 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0649 | C43 H38 N6 O7 S2 | 814.22434 | 0.0 | 0.0 |
| 2-1-m1E03-2-0650 | C45 H39 F N4 O6 S2 | 814.22950 | 0.0 | 0.0 |
| 2-1-m1E03-2-0651 | C41 H37 F3 N6 O7 S | 814.23965 | -2.9 | 10.9 |
| 2-1-m1E03-2-0652 | C45 H42 N4 O7 S2 | 814.24949 | 0.0 | 0.0 |
| 2-1-m1E03-2-0653 | C46 H46 N4 O6 S2 | 814.28588 | 0.0 | 0.0 |
| 2-1-mlE03-2-0654 | C37 H33 N7 O7 S4 | 815.13243 | -9.0 | -9.8 |
| 2-1-m1E03-2-0655 | C39 H37 N5 O9 S3 | 815.17534 | -9.8 | -11.2 |
| 2-1-m1E03-2-0656 | C39 H37 N5 O9 S3 | 815.17534 | -5.6 | 0.0 |
| 2-1-m1E03-2-0657 | C40 H35 F2 N5 O8 S2 | 815.18951 | -19.0 | -10.4 |
| 2-1-m1E03-2-0658 | C40 H35 F2 N5 O8 S2 | 815.18951 | -9.2 | -10.7 |
| 2-1-m1E03-2-0659 | C41 H36 F3 N5 O6 S2 | 815.20591 | 0.0 | 0.0 |
| 2-1-m1E03-2-0660 | C41 H39 F2 N5 O7 S2 | 815.22590 | -1.7 | -9.5 |
| 2-1-m1E03-2-0661 | C43 H44 F3 N5 O6 S | 815.29644 | 5.6 | 12.8 |
| 2-1-m1E03-2-0662 | C39 H36 N4 O8 S4 | 816.14160 | -74.9 | 0.0 |
| 2-1-m1E03-2-0663 | C39 H36 N4 O10 S3 | 816.15936 | 0.0 | 0.0 |
| 2-1-m1E03-2-0664 | C38 H36 N6 O9 S3 | 816.17059 | -0.7 | -7.7 |
| 2-1-m1E03-2-0665 | C43 H36 N4 O7 S3 | 816.17461 | 1.3 | -12.5 |
| 2-1-m1E03-2-0666 | C39 H34 F2 N6 O8 S2 | 816.18476 | 4.7 | 20.2 |
| 2-1-m1E03-2-0667 | C41 H35 F3 N4 O7 S2 | 816.18993 | 15.4 | 17.4 |
| 2-1-m1E03-2-0668 | C41 H35 F3 N4 O7 S2 | 816.18993 | -22.4 | 13.3 |
| 2-1-m1E03-2-0669 | C44 H40 N4 O6 S3 | 816.21100 | -23.9 | 0.0 |
| 2-1-m1E03-2-0670 | C44 H40 N4 O6 S3 | 816.21100 | 0.0 | 0.0 |
| 2-1-m1E03-2-0671 | C44 H40 N4 O8 S2 | 816.22876 | 32.6 | 0.0 |
| 2-1-m1E03-2-0672 | C40 H34 F3 N5 O7 S2 | 817.18517 | -16.5 | -0.9 |
| 2-1-m1E03-2-0673 | C43 H39 N5 O6 S3 | 817.20625 | -5.0 | -6.3 |
| 2-1-m1E03-2-0674 | C41 H35 F4 N5 O7 S | 817.21933 | -6.1 | 3.2 |
| 2-1-m1E03-2-0675 | C42 H39 N7 O7 S2 | 817.23524 | 9.3 | 24.4 |
| 2-1-m1E03-2-0676 | C40 H33 F3 N4 O8 S2 | 818.16919 | 7.9 | 13.6 |
| 2-1-m1E03-2-0677 | C39 H33 F3 N6 O7 S2 | 818.18042 | -31.1 | 26.6 |
| 2-1-m1E03-2-0678 | C41 H34 F4 N4 O6 S2 | 818.18559 | -85.3 | -7.0 |
| 2-1-m1E03-2-0679 | C43 H38 N4 O7 S3 | 818.19026 | 0.0 | 0.0 |
| 2-1-m1E03-2-0680 | C43 H38 N4 O7 S3 | 818.19026 | 22.8 | 16.4 |
| 2-1-m1E03-2-0681 | C45 H43 F N4 O6 S2 | 818.26080 | 0.0 | 0.0 |
| 2-1-m1E03-2-0682 | C38 H37 N5 O10 S3 | 819.17025 | -13.7 | 6.6 |
| 2-1-m1E03-2-0683 | C40 H36 F3 N5 O7 S2 | 819.20082 | -13.5 | 4.5 |
| 2-1-m1E03-2-0684 | C40 H36 F3 N5 O7 S2 | 819.20082 | -23.5 | 28.4 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0685 | C40 H36 F3 N5 O7 S2 | 819.20082 | -7.9 | -26.2 |
| 2-1-m1E03-2-0686 | C40 H36 F3 N5 O7 S2 | 819.20082 | 6.2 | 4.1 |
| 2-1-m1E03-2-0687 | C43 H41 N5 O8 S2 | 819.23965 | 14.2 | -4.7 |
| 2-1-m1E03-2-0688 | C44 H45 N5 O7 S2 | 819.27604 | 0.0 | 0.0 |
| 2-1-m1E03-2-0689 | C45 H49 N5 O6 S2 | 819.31243 | 0.0 | 0.0 |
| 2-1-m1E03-2-0690 | C38 H33 F N4 O8 S4 | 820.11653 | -27.9 | -0.5 |
| 2-1-m1E03-2-0691 | C42 H34 F2 N6 O6 S2 | 820.19493 | -5.5 | -8.0 |
| 2-1-m1E03-2-0692 | C39 H35 F3 N6 O7 S2 | 820.19607 | -7.5 | -5.6 |
| 2-1-m1E03-2-0693 | C39 H35 F3 N6 O7 S2 | 820.19607 | -11.8 | 2.9 |
| 2-1-m1E03-2-0694 | C42 H34 F6 N4 O5 S | 820.21541 | -28.9 | 13.3 |
| 2-1-m1E03-2-0695 | C42 H40 N6 O6 S3 | 820.21715 | -5.4 | 22.0 |
| 2-1-m1E03-2-0696 | C43 H44 N6 O7 S2 | 820.27129 | -2.3 | 8.1 |
| 2-1-m1E03-2-0697 | C45 H48 N4 O7 S2 | 820.29644 | 0.0 | 0.0 |
| 2-1-m1E03-2-0698 | C37 H35 N5 O9 S4 | 821.13176 | -7.5 | -5.4 |
| 2-1-m1E03-2-0699 | C38 H33 F2 N5 O8 S3 | 821.14593 | -9.0 | -0.5 |
| 2-1-m1E03-2-0700 | C42 H36 F N5 O6 S3 | 821.18117 | -31.5 | 8.1 |
| 2-1-m1E03-2-0701 | C38 H34 F3 N7 O7 S2 | 821.19132 | -27.6 | 5.5 |
| 2-1-m1E03-2-0702 | C42 H39 N5 O7 S3 | 821.20116 | 0.0 | -7.9 |
| 2-1-m1E03-2-0703 | C42 H39 N5 O7 S3 | 821.20116 | -5.9 | 19.7 |
| 2-1-m1E03-2-0704 | C41 H36 F N7 O7 S2 | 821.21017 | 1.0 | 1.5 |
| 2-1-m1E03-2-0705 | C44 H47 N5 O7 S2 | 821.29169 | 0.0 | 0.0 |
| 2-1-m1E03-2-0706 | C36 H34 N6 O9 S4 | 822.12701 | -8.5 | -5.8 |
| 2-1-m1E03-2-0707 | C38 H32 F2 N4 O9 S3 | 822.12995 | -9.0 | -5.8 |
| 2-1-m1E03-2-0708 | C39 H33 F3 N4 O7 S3 | 822.14635 | 0.0 | 0.0 |
| 2-1-m1E03-2-0709 | C41 H38 N6 O7 S3 | 822.19641 | -7.0 | -5.7 |
| 2-1-m1E03-2-0710 | C41 H38 N6 O7 S3 | 822.19641 | -27.1 | 21.7 |
| 2-1-m1E03-2-0711 | C43 H36 F2 N4 O7 S2 | 822.19935 | -5.8 | 0.6 |
| 2-1-m1E03-2-0712 | C42 H42 N6 O8 S2 | 822.25055 | -13.4 | -8.2 |
| 2-1-m1E03-2-0713 | C44 H46 N4 O8 S2 | 822.27571 | 18.7 | 0.0 |
| 2-1-m1E03-2-0714 | C48 H46 N4 O5 S2 | 822.29096 | 0.0 | 0.0 |
| 2-1-m1E03-2-0715 | C38 H32 F3 N5 O7 S3 | 823.14160 | -7.6 | 9.4 |
| 2-1-m1E03-2-0716 | C37 H34 F N5 O10 S3 | 823.14518 | -12.2 | -3.2 |
| 2-1-m1E03-2-0717 | C40 H37 N7 O7 S3 | 823.19166 | 6.2 | 8.5 |
| 2-1-m1E03-2-0718 | C40 H37 N7 O7 S3 | 823.19166 | 0.0 | -0.9 |
| 2-1-m1E03-2-0719 | C42 H38 F N5 O8 S2 | 823.21458 | -19.9 | -12.4 |
| 2-1-m1E03-2-0720 | C41 H35 F6 N5 O5 S | 823.22631 | -16.9 | 3.7 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0721 | C42 H36 F3 N7 O6 S | 823.23999 | -7.2 | -0.4 |
| 2-1-m1E03-2-0722 | C43 H45 N5 O8 S2 | 823.27095 | -10.7 | 5.8 |
| 2-1-m1E03-2-0723 | C42 H34 F2 N4 O6 S3 | 824.16085 | 9.1 | -9.0 |
| 2-1-m1E03-2-0724 | C42 H34 F2 N4 O6 S3 | 824.16085 | 0.0 | 0.0 |
| 2-1-m1E03-2-0725 | C42 H40 N4 O8 S3 | 824.20083 | 0.0 | 0.0 |
| 2-1-m1E03-2-0726 | C41 H40 N6 O9 S2 | 824.22982 | 0.0 | -14.1 |
| 2-1-m1E03-2-0727 | C38 H34 F3 N5 O7 S3 | 825.15725 | 18.3 | 20.9 |
| 2-1-m1E03-2-0728 | C38 H34 F3 N5 O9 S2 | 825.17500 | -28.6 | -10.4 |
| 2-1-m1E03-2-0729 | C41 H36 F N5 O7 S3 | 825.17609 | -3.3 | -4.7 |
| 2-1-m1E03-2-0730 | C41 H36 F N5 O7 S3 | 825.17609 | -6.7 | -9.8 |
| 2-1-m1E03-2-0731 | C45 H39 N5 O7 S2 | 825.22909 | 0.0 | 0.0 |
| 2-1-m1E03-2-0732 | C42 H40 F N5 O8 S2 | 825.23023 | -14.1 | 2.8 |
| 2-1-mlE03-2-0733 | C42 H43 N5 O9 S2 | 825.25022 | 0.0 | 0.0 |
| 2-1-mlE03-2-0734 | C43 H47 N5 O8 S2 | 825.28660 | -8.4 | -7.6 |
| 2-1-m1E03-2-0735 | C37 H33 F3 N6 O7 S3 | 826.15249 | -22.1 | 12.1 |
| 2-1-mlE03-2-0736 | C39 H34 N6 O9 S3 | 826.15494 | -11.0 | -4.5 |
| 2-1-m1E03-2-0737 | C41 H38 N4 O9 S3 | 826.18009 | 0.0 | 0.0 |
| 2-1-m1E03-2-0738 | C44 H41 F3 N4 O5 S2 | 826.24705 | 0.0 | 0.0 |
| 2-1-m1E03-2-0739 | C46 H42 N4 O7 S2 | 826.24949 | 0.0 | 0.0 |
| 2-1-m1E03-2-0740 | C42 H36 F3 N5 O6 S2 | 827.20591 | 0.0 | 0.0 |
| 2-1-m1E03-2-0741 | C41 H38 F N5 O9 S2 | 827.20950 | 0.0 | 0.0 |
| 2-1-m1E03-2-0742 | C39 H40 F3 N5 O8 S2 | 827.22704 | 0.0 | 0.0 |
| 2-1-m1E03-2-0743 | C43 H43 F2 N5 O6 S2 | 827.26228 | 13.4 | -2.1 |
| 2-1-m1E03-2-0744 | C42 H35 F3 N4 O7 S2 | 828.18993 | 7.6 | 14.4 |
| 2-1-m1E03-2-0745 | C45 H37 F N4 O7 S2 | 828.20877 | 0.0 | 0.0 |
| 2-1-m1E03-2-0746 | C43 H39 F3 N4 O6 S2 | 828.22631 | 0.0 | 0.0 |
| 2-1-m1E03-2-0747 | C43 H39 F3 N4 O6 S2 | 828.22631 | 0.0 | 0.0 |
| 2-1-m1E03-2-0748 | C43 H39 F3 N4 O6 S2 | 828.22631 | -59.1 | -20.3 |
| 2-1-m1E03-2-0749 | C43 H42 F2 N4 O7 S2 | 828.24630 | -14.6 | 0.0 |
| 2-1-m1E03-2-0750 | C47 H48 N4 O6 S2 | 828.30153 | 0.0 | 0.0 |
| 2-1-m1E03-2-0751 | C41 H34 F3 N5 O7 S2 | 829.18517 | -16.8 | -46.2 |
| 2-1-m1E03-2-0752 | C40 H39 N5 O9 S3 | 829.19099 | 0.0 | 5.1 |
| 2-1-m1E03-2-0753 | C42 H44 F N5 O8 S2 | 829.26153 | -14.4 | -7.1 |
| 2-1-m1E03-2-0754 | C40 H38 N4 O8 S4 | 830.15725 | -25.6 | 0.0 |
| 2-1-m1E03-2-0755 | C40 H36 F2 N6 O8 S2 | 830.20041 | -12.0 | -10.7 |
| 2-1-m1E03-2-0756 | C42 H37 F3 N4 O7 S2 | 830.20558 | -41.4 | -0.1 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0757 | C42 H37 F3 N4 O7 S2 | 830.20558 | 10.1 | 16.7 |
| 2-1-m1E03-2-0758 | C46 H46 N4 O7 S2 | 830.28079 | 0.0 | 0.0 |
| 2-1-m1E03-2-0759 | C41 H36 F3 N5 O7 S2 | 831.20082 | -0.5 | 11.7 |
| 2-1-m1E03-2-0760 | C41 H39 F2 N5 O8 S2 | 831.22081 | 0.8 | -6.8 |
| 2-1-m1E03-2-0761 | C44 H41 N5 O6 S3 | 831.22190 | 0.8 | -6.8 |
| 2-1-m1E03-2-0762 | C42 H40 F3 N5 O6 S2 | 831.23721 | -4.7 | -2.5 |
| 2-1-m1E03-2-0763 | C43 H41 N7 O7 S2 | 831.25089 | -15.4 | -8.6 |
| 2-1-m1E03-2-0764 | C43 H44 F3 N5 O7 S | 831.29135 | 9.9 | -0.1 |
| 2-1-m1E03-2-0765 | C39 H36 N4 O9 S4 | 832.13651 | 0.0 | -104.4 |
| 2-1-m1E03-2-0766 | C40 H34 F2 N4 O8 S3 | 832.15068 | 0.0 | 19.9 |
| 2-1-m1E03-2-0767 | C42 H36 F4 N4 O6 S2 | 832.20124 | 0.0 | 8.7 |
| 2-1-m1E03-2-0768 | C39 H36 F N5 O9 S3 | 833.16592 | -6.7 | -11.5 |
| 2-1-m1E03-2-0769 | C40 H34 F3 N5 O8 S2 | 833.18009 | -13.8 | -5.9 |
| 2-1-m1E03-2-0770 | C39 H39 N5 O10 S3 | 833.18590 | -4.9 | 20.9 |
| 2-1-m1E03-2-0771 | C43 H39 N5 O7 S3 | 833.20116 | 0.0 | 0.0 |
| 2-1-m1E03-2-0772 | C41 H38 F3 N5 O7 S2 | 833.21647 | -123.3 | -29.2 |
| 2-1-m1E03-2-0773 | C41 H38 F3 N5 O7 S2 | 833.21647 | -6.5 | 4.7 |
| 2-1-m1E03-2-0774 | C42 H39 N7 O8 S2 | 833.23015 | 0.0 | 0.0 |
| 2-1-m1E03-2-0775 | C44 H43 N5 O8 S2 | 833.25530 | -17.7 | 5.4 |
| 2-1-m1E03-2-0776 | C45 H47 N5 O7 S2 | 833.29169 | -3.4 | 0.0 |
| 2-1-m1E03-2-0777 | C40 H33 F3 N4 O7 S3 | 834.14635 | 123.2 | 7.8 |
| 2-1-m1E03-2-0778 | C40 H33 F3 N4 O9 S2 | 834.16410 | -30.1 | -49.4 |
| 2-1-m1E03-2-0779 | C40 H37 F3 N6 O7 S2 | 834.21172 | 0.0 | 0.0 |
| 2-1-m1E03-2-0780 | C46 H38 N6 O6 S2 | 834.22942 | 0.0 | 0.0 |
| 2-1-m1E03-2-0781 | C43 H42 N6 O6 S3 | 834.23280 | -5.5 | 0.0 |
| 2-1-m1E03-2-0782 | C38 H37 N5 O9 S4 | 835.14741 | -47.2 | 0.0 |
| 2-1-m1E03-2-0783 | C38 H37 N5 O11 S3 | 835.16517 | -27.1 | 8.2 |
| 2-1-m1E03-2-0784 | C40 H36 F3 N5 O8 S2 | 835.19574 | -2.7 | 1.5 |
| 2-1-m1E03-2-0785 | C40 H36 F3 N5 O8 S2 | 835.19574 | -14.6 | 10.1 |
| 2-1-m1E03-2-0786 | C43 H41 N5 O7 S3 | 835.21681 | -8.2 | 0.8 |
| 2-1-m1E03-2-0787 | C43 H41 N5 O7 S3 | 835.21681 | 0.0 | 0.0 |
| 2-1-m1E03-2-0788 | C43 H41 N5 O9 S2 | 835.23457 | 0.0 | 0.0 |
| 2-1-m1E03-2-0789 | C42 H36 N4 O9 S3 | 836.16444 | 0.0 | 0.0 |
| 2-1-m1E03-2-0790 | C41 H36 N6 O8 S3 | 836.17567 | -7.7 | -18.2 |
| 2-1-m1E03-2-0791 | C41 H33 F5 N4 O6 S2 | 836.17617 | -10.4 | -12.2 |
| 2-1-m1E03-2-0792 | C39 H35 F3 N6 O8 S2 | 836.19099 | -17.7 | -1.0 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0793 | C42 H40 N6 O7 S3 | 836.21206 | -5.4 | -18.5 |
| 2-1-m1E03-2-0794 | C42 H40 N6 O7 S3 | 836.21206 | 18.7 | 32.4 |
| 2-1-m1E03-2-0795 | C47 H40 N4 O7 S2 | 836.23384 | 0.0 | 0.0 |
| 2-1-m1E03-2-0796 | C45 H42 F2 N4 O6 S2 | 836.25138 | 0.0 | 0.0 |
| 2-1-m1E03-2-0797 | C40 H35 N7 O8 S3 | 837.17092 | 9.9 | -3.2 |
| 2-1-m1E03-2-0798 | C39 H34 F3 N5 O9 S2 | 837.17500 | 0.0 | 1.2 |
| 2-1-m1E03-2-0799 | C38 H34 F3 N7 O8 S2 | 837.18624 | 0.0 | 0.0 |
| 2-1-m1E03-2-0800 | C40 H35 F4 N5 O7 S2 | 837.19140 | -0.7 | 3.1 |
| 2-1-m1E03-2-0801 | C42 H39 N5 O8 S3 | 837.19608 | 6.3 | 0.1 |
| 2-1-m1E03-2-0802 | C42 H39 N5 O8 S3 | 837.19608 | -59.8 | 9.0 |
| 2-1-m1E03-2-0803 | C44 H44 F N5 O7 S2 | 837.26662 | 0.0 | 0.0 |
| 2-1-m1E03-2-0804 | C38 H32 F2 N4 O8 S4 | 838.10710 | -16.7 | -18.4 |
| 2-1-m1E03-2-0805 | C46 H38 N4 O6 S3 | 838.19535 | -3.1 | 35.7 |
| 2-1-m1E03-2-0806 | C46 H38 N4 O6 S3 | 838.19535 | 0.1 | 4.8 |
| 2-1-m1E03-2-0807 | C37 H34 F N5 O9 S4 | 839.12234 | -3.8 | -3.8 |
| 2-1-m1E03-2-0808 | C42 H35 F2 N5 O6 S3 | 839.17175 | -24.0 | -14.1 |
| 2-1-m1E03-2-0809 | C41 H35 F2 N7 O7 S2 | 839.20074 | -7.2 | -15.6 |
| 2-1-m1E03-2-0810 | C41 H35 F6 N5 O6 S | 839.22122 | -1.2 | 6.1 |
| 2-1-m1E03-2-0811 | C44 H49 N5 O8 S2 | 839.30225 | 0.0 | 0.0 |
| 2-1-m1E03-2-0812 | C41 H37 F N6 O7 S3 | 840.18699 | -3.3 | -4.1 |
| 2-1-m1E03-2-0813 | C41 H37 F N6 O7 S3 | 840.18699 | 0.0 | 0.0 |
| 2-1-m1E03-2-0814 | C45 H43 F3 N4 O5 S2 | 840.26270 | 0.0 | 0.0 |
| 2-1-m1E03-2-0815 | C37 H33 F2 N5 O10 S3 | 841.13576 | -13.6 | -2.1 |
| 2-1-m1E03-2-0816 | C38 H34 F3 N5 O8 S3 | 841.15216 | 6.5 | 19.8 |
| 2-1-m1E03-2-0817 | C42 H37 F2 N5 O8 S2 | 841.20516 | -7.1 | 19.1 |
| 2-1-m1E03-2-0818 | C43 H47 N5 O9 S2 | 841.28152 | 0.0 | 0.0 |
| 2-1-m1E03-2-0819 | C39 H34 N6 O8 S4 | 842.13209 | -17.3 | -8.0 |
| 2-1-m1E03-2-0820 | C37 H33 F3 N6 O8 S3 | 842.14741 | 16.5 | 9.4 |
| 2-1-m1E03-2-0821 | C44 H41 F3 N4 O6 S2 | 842.24196 | 10.1 | 14.1 |
| 2-1-m1E03-2-0822 | C47 H46 N4 O7 S2 | 842.28079 | 0.0 | 0.0 |
| 2-1-m1E03-2-0823 | C41 H35 F2 N5 O7 S3 | 843.16667 | 0.0 | 0.0 |
| 2-1-m1E03-2-0824 | C41 H35 F2 N5 O7 S3 | 843.16667 | -84.3 | 0.0 |
| 2-1-m1E03-2-0825 | C42 H36 F3 N5 O7 S2 | 843.20082 | 14.0 | 4.5 |
| 2-1-m1E03-2-0826 | C41 H41 N5 O9 S3 | 843.20664 | -6.5 | 18.0 |
| 2-1-m1E03-2-0827 | C42 H35 F3 N4 O8 S2 | 844.18484 | -12.6 | -22.8 |
| 2-1-m1E03-2-0828 | C43 H39 F3 N4 O7 S2 | 844.22123 | 0.0 | 0.0 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0829 | C43 H39 F3 N4 O7 S2 | 844.22123 | 0.0 | 0.0 |
| 2-1-m1E03-2-0830 | C41 H34 F3 N5 O8 S2 | 845.18009 | -29.2 | 14.7 |
| 2-1-m1E03-2-0831 | C40 H39 N5 O10 S3 | 845.18590 | -5.6 | 0.0 |
| 2-1-m1E03-2-0832 | C43 H42 F3 N5 O6 S2 | 845.25286 | 0.0 | -0.8 |
| 2-1-m1E03-2-0833 | C42 H34 F4 N4 O7 S2 | 846.18050 | 27.6 | 13.5 |
| 2-1-m1E03-2-0834 | C44 H38 N4 O8 S3 | 846.18518 | -6.0 | -24.5 |
| 2-1-m1E03-2-0835 | C41 H36 F3 N5 O8 S2 | 847.19574 | -3.1 | 4.2 |
| 2-1-m1E03-2-0836 | C42 H40 F3 N5 O7 S2 | 847.23212 | -11.2 | -0.3 |
| 2-1-m1E03-2-0837 | C42 H40 F3 N5 O7 S2 | 847.23212 | 0.0 | 0.0 |
| 2-1-m1E03-2-0838 | C42 H40 F3 N5 O7 S2 | 847.23212 | -23.4 | 31.9 |
| 2-1-m1E03-2-0839 | C42 H43 F2 N5 O8 S2 | 847.25211 | 0.0 | 0.0 |
| 2-1-m1E03-2-0840 | C46 H49 N5 O7 S2 | 847.30734 | 0.0 | 0.0 |
| 2-1-m1E03-2-0841 | C40 H35 F3 N6 O8 S2 | 848.19099 | 19.7 | 0.0 |
| 2-1-m1E03-2-0842 | C42 H36 F4 N4 O7 S2 | 848.19615 | -209.2 | -108.7 |
| 2-1-m1E03-2-0843 | C44 H44 N6 O6 S3 | 848.24845 | -36.3 | -0.2 |
| 2-1-m1E03-2-0844 | C39 H39 N5 O9 S4 | 849.16306 | 0.0 | 0.0 |
| 2-1-m1E03-2-0845 | C41 H38 F3 N5 O8 S2 | 849.21139 | 46.1 | -16.9 |
| 2-1-m1E03-2-0846 | C41 H38 F3 N5 O8 S2 | 849.21139 | -13.8 | 24.1 |
| 2-1-m1E03-2-0847 | C45 H47 N5 O8 S2 | 849.28660 | 0.0 | 0.0 |
| 2-1-m1E03-2-0848 | C40 H33 F3 N4 O8 S3 | 850.14126 | -16.9 | -27.5 |
| 2-1-m1E03-2-0849 | C43 H38 N4 O9 S3 | 850.18009 | -87.4 | 0.0 |
| 2-1-m1E03-2-0850 | C40 H37 F3 N6 O8 S2 | 850.20664 | -36.9 | -13.7 |
| 2-1-m1E03-2-0851 | C45 H37 F3 N4 O6 S2 | 850.21066 | 1.1 | 0.0 |
| 2-1-m1E03-2-0852 | C43 H42 N6 O7 S3 | 850.22771 | 3.0 | 22.6 |
| 2-1-m1E03-2-0853 | C49 H46 N4 O6 S2 | 850.28588 | 0.0 | 0.0 |
| 2-1-m1E03-2-0854 | C38 H37 N5 O10 S4 | 851.14233 | 19.5 | -24.1 |
| 2-1-m1E03-2-0855 | C39 H35 F2 N5 O9 S3 | 851.15650 | -0.6 | -9.2 |
| 2-1-m1E03-2-0856 | C42 H37 N5 O9 S3 | 851.17534 | -8.5 | -16.7 |
| 2-1-m1E03-2-0857 | C41 H37 N7 O8 S3 | 851.18657 | -9.3 | 14.0 |
| 2-1-m1E03-2-0858 | C41 H37 F4 N5 O7 S2 | 851.20705 | -14.1 | -0.1 |
| 2-1-m1E03-2-0859 | C42 H36 N4 O8 S4 | 852.14160 | 0.0 | 0.0 |
| 2-1-m1E03-2-0860 | C42 H36 N4 O8 S4 | 852.14160 | 20.0 | 0.0 |
| 2-1-m1E03-2-0861 | C43 H35 F3 N6 O6 S2 | 852.20116 | -6.9 | 0.0 |
| 2-1-m1E03-2-0862 | C42 H40 N6 O8 S3 | 852.20697 | 0.0 | -87.9 |
| 2-1-m1E03-2-0863 | C42 H40 N6 O8 S3 | 852.20697 | 0.0 | 0.0 |
| 2-1-m1E03-2-0864 | C41 H35 N5 O8 S4 | 853.13685 | -12.8 | 2.4 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0865 | C39 H34 F3 N5 O8 S3 | 853.15216 | 4.3 | -18.2 |
| 2-1-m1E03-2-0866 | C39 H34 F3 N5 O10 S2 | 853.16992 | 3.8 | -24.5 |
| 2-1-m1E03-2-0867 | C46 H39 N5 O6 S3 | 853.20625 | 0.0 | 0.0 |
| 2-1-m1E03-2-0868 | C39 H33 F3 N4 O9 S3 | 854.13618 | -5.2 | -4.1 |
| 2-1-m1E03-2-0869 | C41 H35 FN6 O8 S3 | 854.16625 | -15.4 | -0.4 |
| 2-1-m1E03-2-0870 | C44 H37 F3 N4 O7 S2 | 854.20558 | -34.8 | -13.0 |
| 2-1-m1E03-2-0871 | C40 H34 F5 N5 O7 S2 | 855.18198 | -9.4 | 9.7 |
| 2-1-m1E03-2-0872 | C44 H43 F2 N5 O7 S2 | 855.25720 | -4.2 | -9.8 |
| 2-1-m1E03-2-0873 | C43 H35 F3 N4 O6 S3 | 856.16708 | -20.1 | -13.7 |
| 2-1-m1E03-2-0874 | C43 H35 F3 N4 O6 S3 | 856.16708 | 0.0 | 0.0 |
| 2-1-m1E03-2-0875 | C45 H43 F3 N4 O6 S2 | 856.25761 | 0.0 | 0.0 |
| 2-1-m1E03-2-0876 | C37 H33 F2 N5 O9 S4 | 857.11292 | -10.4 | -0.8 |
| 2-1-m1E03-2-0877 | C41 H36 F2 N6 O7 S3 | 858.17757 | -3.4 | 15.4 |
| 2-1-m1E03-2-0878 | C42 H36 F3 N5 O8 S2 | 859.19574 | -20.5 | 47.3 |
| 2-1-m1E03-2-0879 | C44 H44 F3 N5 O6 S2 | 859.26851 | 0.0 | 0.0 |
| 2-1-m1E03-2-0880 | C42 H36 N8 O7 S3 | 860.18691 | -12.2 | -2.6 |
| 2-1-m1E03-2-0881 | C43 H39 F3 N4 O8 S2 | 860.21614 | 0.0 | 0.0 |
| 2-1-m1E03-2-0882 | C44 H43 F3 N4 O7 S2 | 860.25253 | 11.8 | -3.2 |
| 2-1-m1E03-2-0883 | C43 H42 F3 N5 O7 S2 | 861.24777 | -1.8 | 5.0 |
| 2-1-m1E03-2-0884 | C38 H34 N6 O10 S4 | 862.12192 | -14.8 | -15.0 |
| 2-1-m1E03-2-0885 | C42 H34 F4 N4 O8 S2 | 862.17542 | -14.2 | -20.3 |
| 2-1-m1E03-2-0886 | C43 H38 N6 O8 S3 | 862.19132 | -8.1 | 1.7 |
| 2-1-m1E03-2-0887 | C41 H37 F3 N6 O8 S2 | 862.20664 | -14.2 | -4.9 |
| 2-1-m1E03-2-0888 | C41 H36 F3 N5 O9 S2 | 863.19065 | -8.7 | 26.7 |
| 2-1-m1E03-2-0889 | C42 H40 F3 N5 O8 S2 | 863.22704 | 0.0 | 0.0 |
| 2-1-m1E03-2-0890 | C42 H40 F3 N5 O8 S2 | 863.22704 | 0.0 | 0.0 |
| 2-1-m1E03-2-0891 | C42 H36 N6 O7 S4 | 864.15283 | 1.1 | -26.8 |
| 2-1-m1E03-2-0892 | C42 H36 N6 O7 S4 | 864.15283 | 0.6 | 6.1 |
| 2-1-m1E03-2-0893 | C41 H35 F3 N4 O8 S3 | 864.15691 | 0.0 | 0.0 |
| 2-1-m1E03-2-0894 | C40 H35 F3 N6 O9 S2 | 864.18590 | -0.9 | 4.8 |
| 2-1-m1E03-2-0895 | C44 H40 N4 O9 S3 | 864.19574 | 0.0 | 0.0 |
| 2-1-m1E03-2-0896 | C41 H35 F4 N5 O8 S2 | 865.18632 | -17.9 | 3.4 |
| 2-1-m1E03-2-0897 | C43 H38 N4 O8 S4 | 866.15725 | 0.0 | 0.0 |
| 2-1-m1E03-2-0898 | C42 H37 N5 O8 S4 | 867.15250 | -8.8 | 2.0 |
| 2-1-m1E03-2-0899 | C41 H37 F4 N5 O8 S2 | 867.20197 | 4.1 | 30.1 |
| 2-1-m1E03-2-0900 | C41 H36 N6 O8 S4 | 868.14774 | -0.5 | 8.8 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0901 | C43 H37 F N4 O9 S3 | 868.17067 | -16.7 | 1.0 |
| 2-1-m1E03-2-0902 | C42 H34 F6 N4 O6 S2 | 868.18240 | 0.0 | 0.0 |
| 2-1-m1E03-2-0903 | C43 H35 F3 N6 O7 S2 | 868.19607 | 13.6 | -34.5 |
| 2-1-m1E03-2-0904 | C43 H44 N6 O8 S3 | 868.23827 | -11.9 | 2.1 |
| 2-1-m1E03-2-0905 | C46 H43 F3 N4 O6 S2 | 868.25761 | -14.3 | 0.0 |
| 2-1-m1E03-2-0906 | C39 H34 F3 N5 O9 S3 | 869.14707 | -12.9 | 30.3 |
| 2-1-m1E03-2-0907 | C42 H39 N5 O10 S3 | 869.18590 | 81.0 | 80.0 |
| 2-1-m1E03-2-0908 | C39 H33 F3 N4 O8 S4 | 870.11333 | -3.0 | 8.6 |
| 2-1-m1E03-2-0909 | C39 H33 F3 N4 O10 S3 | 870.13109 | -10.6 | 12.3 |
| 2-1-m1E03-2-0910 | C42 H35 F N4 O8 S4 | 870.13218 | -16.1 | 39.9 |
| 2-1-m1E03-2-0911 | C41 H38 N6 O10 S3 | 870.18115 | -11.8 | -6.6 |
| 2-1-m1E03-2-0912 | C44 H37 F3 N4 O8 S2 | 870.20049 | -23.4 | -29.3 |
| 2-1-m1E03-2-0913 | C41 H37 N5 O9 S4 | 871.14741 | 1.0 | 32.4 |
| 2-1-m1E03-2-0914 | C43 H36 F3 N5 O6 S3 | 871.17798 | -108.8 | 0.0 |
| 2-1-m1E03-2-0915 | C42 H36 F3 N7 O7 S2 | 871.20697 | -8.8 | -11.2 |
| 2-1-m1E03-2-0916 | C40 H36 N6 O9 S4 | 872.14266 | -9.6 | -0.3 |
| 2-1-m1E03-2-0917 | C40 H36 N6 O9 S4 | 872.14266 | -26.3 | -3.4 |
| 2-1-m1E03-2-0918 | C43 H35 F3 N4 O7 S3 | 872.16200 | 0.0 | 0.0 |
| 2-1-m1E03-2-0919 | C43 H35 F3 N4 O7 S3 | 872.16200 | 4.8 | 10.4 |
| 2-1-m1E03-2-0920 | C38 H34 F3 N5 O10 S3 | 873.14199 | -11.8 | 0.0 |
| 2-1-m1E03-2-0921 | C43 H38 F3 N5 O8 S2 | 873.21139 | 9.5 | 6.8 |
| 2-1-m1E03-2-0922 | C42 H36 F3 N5 O7 S3 | 875.17290 | -4.4 | 4.6 |
| 2-1-m1E03-2-0923 | C42 H36 F3 N5 O7 S3 | 875.17290 | -15.8 | 16.5 |
| 2-1-m1E03-2-0924 | C44 H44 F3 N5 O7 S2 | 875.26342 | 0.0 | 0.0 |
| 2-1-m1E03-2-0925 | C41 H35 F3 N6 O7 S3 | 876.16814 | -6.0 | -11.8 |
| 2-1-m1E03-2-0926 | C45 H44 N6 O7 S3 | 876.24336 | -14.3 | -8.7 |
| 2-1-m1E03-2-0927 | C44 H43 F3 N4 O8 S2 | 876.24744 | -22.2 | -16.1 |
| 2-1-m1E03-2-0928 | C38 H34 N6 O9 S5 | 878.09908 | -7.3 | -34.5 |
| 2-1-m1E03-2-0929 | C41 H37 F3 N6 O9 S2 | 878.20155 | -14.7 | -5.2 |
| 2-1-m1E03-2-0930 | C45 H42 N4 O9 S3 | 878.21139 | 0.0 | 0.0 |
| 2-1-m1E03-2-0931 | C42 H37 N7 O7 S4 | 879.16373 | -8.7 | -23.6 |
| 2-1-m1E03-2-0932 | C42 H40 F3 N5 O9 S2 | 879.22195 | -2.5 | -10.5 |
| 2-1-m1E03-2-0933 | C43 H44 F3 N5 O8 S2 | 879.25834 | 0.0 | 32.3 |
| 2-1-m1E03-2-0934 | C41 H35 F3 N4 O9 S3 | 880.15183 | -16.5 | 0.0 |
| 2-1-m1E03-2-0935 | C44 H40 N4 O8 S4 | 880.17290 | 3.1 | 8.5 |
| 2-1-m1E03-2-0936 | C44 H40 N4 O10 S3 | 880.19066 | 0.0 | -4.9 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0937 | C43 H39 N5 O8 S4 | 881.16815 | -0.4 | -40.3 |
| 2-1-m1E03-2-0938 | C41 H35 F4 N5 O9 S2 | 881.18123 | -55.3 | 6.0 |
| 2-1-m1E03-2-0939 | C43 H38 N4 O9 S4 | 882.15216 | 9.3 | -4.5 |
| 2-1-m1E03-2-0940 | C40 H36 F3 N5 O9 S3 | 883.16272 | 0.5 | 20.8 |
| 2-1-m1E03-2-0941 | C43 H41 N5 O10 S3 | 883.20155 | -20.0 | -19.8 |
| 2-1-m1E03-2-0942 | C42 H34 F6 N4 O7 S2 | 884.17731 | -34.3 | 15.4 |
| 2-1-m1E03-2-0943 | C46 H43 F3 N4 O7 S2 | 884.25253 | 0.0 | 0.0 |
| 2-1-m1E03-2-0944 | C42 H36 F N5 O8 S4 | 885.14307 | 0.0 | 0.0 |
| 2-1-m1E03-2-0945 | C42 H39 N5 O9 S4 | 885.16306 | 5.6 | 19.4 |
| 2-1-m1E03-2-0946 | C39 H33 F3 N4 O9 S4 | 886.10825 | -21.6 | -7.5 |
| 2-1-m1E03-2-0947 | C41 H38 N6 O9 S4 | 886.15831 | -3.4 | 8.4 |
| 2-1-m1E03-2-0948 | C43 H36 F2 N4 O9 S3 | 886.16125 | -3.4 | 11.4 |
| 2-1-m1E03-2-0949 | C40 H37 N7 O9 S4 | 887.15356 | -5.9 | -2.5 |
| 2-1-m1E03-2-0950 | C43 H36 F3 N5 O7 S3 | 887.17290 | -25.7 | 3.7 |
| 2-1-m1E03-2-0951 | C42 H38 F N5 O10 S3 | 887.17648 | 27.0 | -9.6 |
| 2-1-m1E03-2-0952 | C41 H35 F6 N5 O7 S2 | 887.18821 | 0.4 | -0.9 |
| 2-1-m1E03-2-0953 | C42 H36 F3 N7 O8 S2 | 887.20189 | -3.2 | 9.1 |
| 2-1-m1E03-2-0954 | C45 H44 F3 N5 O7 S2 | 887.26342 | 0.0 | 0.0 |
| 2-1-m1E03-2-0955 | C42 H34 F2 N4 O8 S4 | 888.12275 | -12.2 | 0.0 |
| 2-1-m1E03-2-0956 | C38 H34 F3 N5 O9 S4 | 889.11915 | 3.3 | -7.8 |
| 2-1-m1E03-2-0957 | C38 H34 F3 N5 O11 S3 | 889.13690 | 16.7 | -19.0 |
| 2-1-m1E03-2-0958 | C41 H36 F N5 O9 S4 | 889.13799 | 16.7 | -19.0 |
| 2-1-m1E03-2-0959 | C43 H38 F3 N5 O9 S2 | 889.20630 | 37.1 | -24.7 |
| 2-1-m1E03-2-0960 | C42 H37 F3 N6 O7 S3 | 890.18379 | 5.6 | 1.1 |
| 2-1-m1E03-2-0961 | C42 H36 F3 N5 O8 S3 | 891.16781 | -36.5 | 23.2 |
| 2-1-m1E03-2-0962 | C42 H36 F3 N5 O8 S3 | 891.16781 | -8.0 | -4.3 |
| 2-1-m1E03-2-0963 | C44 H41 N5 O8 S4 | 895.18380 | 0.3 | 0.0 |
| 2-1-m1E03-2-0964 | C43 H44 F3 N5 O9 S2 | 895.25325 | 20.1 | 18.8 |
| 2-1-m1E03-2-0965 | C43 H39 N5 O9 S4 | 897.16306 | 0.7 | 0.0 |
| 2-1-m1E03-2-0966 | C44 H43 N5 O10 S3 | 897.21720 | -13.0 | -3.9 |
| 2-1-m1E03-2-0967 | C40 H36 F3 N5 O10 S3 | 899.15764 | -18.5 | -0.1 |
| 2-1-m1E03-2-0968 | C43 H41 N5 O9 S4 | 899.17871 | 80.2 | 76.5 |
| 2-1-m1E03-2-0969 | C43 H41 N5 O11 S3 | 899.19647 | 0.4 | 4.2 |
| 2-1-m1E03-2-0970 | C42 H40 N6 O9 S4 | 900.17396 | 15.6 | 11.3 |
| 2-1-m1E03-2-0971 | C47 H40 N4 O9 S3 | 900.19574 | 0.0 | 0.0 |
| 2-1-m1E03-2-0972 | C42 H39 N5 O10 S4 | 901.15798 | -24.3 | -0.8 |

(continued)

| [Table 11-2-7] | | | | |
|---|---|---|---|---|
| | | | AS-MS ratio (%) | |
| ID | Molecular Formula | Exact Mass | N1 | N2 |
| 2-1-m1E03-2-0973 | C46 H38 N4 O8 S4 | 902.15725 | -11.3 | 8.0 |
| 2-1-m1E03-2-0974 | C42 H35 F2 N5 O8 S4 | 903.13365 | 48.8 | 11.3 |
| 2-1-m1E03-2-0975 | C41 H35 F6 N5 O8 S2 | 903.18312 | 7.9 | -8.3 |
| 2-1-m1E03-2-0976 | C45 H44 F3 N5 O8 S2 | 903.25834 | 0.0 | 0.0 |
| 2-1-m1E03-2-0977 | C41 H37 F N6 O9 S4 | 904.14889 | 6.1 | -21.7 |
| 2-1-m1E03-2-0978 | C38 H34 F3 N5 O10 S4 | 905.11406 | 20.9 | 26.1 |
| 2-1-m1E03-2-0979 | C42 H37 F2 N5 O10 S3 | 905.16706 | -17.9 | -14.4 |
| 2-1-m1E03-2-0980 | C42 H37 F3 N6 O8 S3 | 906.17871 | -14.9 | 10.0 |
| 2-1-m1E03-2-0981 | C41 H35 F2 N5 O9 S4 | 907.12857 | -8.9 | 17.1 |
| 2-1-m1E03-2-0982 | C43 H42 N6 O9 S4 | 914.18961 | 73.1 | 68.2 |
| 2-1-m1E03-2-0983 | C42 H40 N6 O10 S4 | 916.16887 | 0.0 | 0.0 |
| 2-1-m1E03-2-0984 | C46 H39 N5 O8 S4 | 917.16815 | 0.0 | 0.0 |
| 2-1-m1E03-2-0985 | C44 H37 F3 N4 O9 S3 | 918.16748 | 0.0 | 0.0 |
| 2-1-m1E03-2-0986 | C43 H35 F3 N4 O8 S4 | 920.12898 | -25.6 | 9.9 |
| 2-1-m1E03-2-0987 | C41 H36 F2 N6 O9 S4 | 922.13947 | 45.5 | 52.0 |
| 2-1-m1E03-2-0988 | C43 H38 N6 O10 S4 | 926.15322 | -26.0 | 41.1 |
| 2-1-m1E03-2-0989 | C42 H36 N6 O9 S5 | 928.11473 | 17.4 | -15.5 |
| 2-1-m1E03-2-0990 | C44 H37 F3 N4 O10 S3 | 934.16239 | 0.0 | -4.5 |
| 2-1-m1E03-2-0991 | C43 H36 F3 N5 O8 S4 | 935.13988 | 15.2 | -45.9 |
| 2-1-m1E03-2-0992 | C43 H35 F3 N4 O9 S4 | 936.12390 | -3.0 | 23.2 |
| 2-1-m1E03-2-0993 | C43 H38 F3 N5 O10 S3 | 937.17329 | 20.1 | -27.9 |
| 2-1-m1E03-2-0994 | C42 H36 F3 N5 O9 S4 | 939.13480 | 1.5 | 3.8 |
| 2-1-m1E03-2-0995 | C42 H37 N7 O9 S5 | 943.12563 | -12.2 | 10.9 |
| 2-1-m1E03-2-0996 | C43 H36 F3 N5 O9 S4 | 951.13480 | -3026.6 | 0.0 |
| 2-1-m1E03-2-0997 | C43 H38 F3 N5 O11 S3 | 953.16820 | -19.9 | -28.0 |
| 2-1-m1E03-2-0998 | C42 H37 F3 N6 O9 S4 | 954.14569 | -11.5 | -5.8 |
| 2-1-m1E03-2-0999 | C42 H36 F3 N5 O10 S4 | 955.12971 | -26.0 | -16.3 |
| 2-1-m1E03-2-1000 | C42 H37 F3 N6 O10 S4 | 970.14061 | 3.9 | 6.7 |

[Table 597]

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0001 | C46 H47 N5 O5 S | 781.32979 | -1.8 | -5.8 |
| 2-1-m1E04-2-0002 | C45 H46 N6 O5 S | 782.32504 | 32.2 | 1.2 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0003 | C45 H46 N6 O5 S | 782.32504 | 0.0 | 0.0 |
| 2-1-m1E04-2-0004 | C44 H45 N7 O5 S | 783.32029 | -2.3 | 0.6 |
| 2-1-m1E04-2-0005 | C44 H45 N7 O5 S | 783.32029 | 0.0 | -14.6 |
| 2-1-m1E04-2-0006 | C43 H44 N8 O5 S | 784.31554 | 18.9 | 19.3 |
| 2-1-m1E04-2-0007 | C43 H44 N6 O5 S2 | 788.28146 | -6.2 | 3.8 |
| 2-1-m1E04-2-0008 | C42 H43 N7 O5 S2 | 789.27671 | 6.8 | 20.7 |
| 2-1-m1E04-2-0009 | C47 H49 N5 O5 S | 795.34544 | 5.2 | 3.6 |
| 2-1-m1E04-2-0010 | C46 H48 N6 O5 S | 796.34069 | -9.3 | 14.3 |
| 2-1-m1E04-2-0011 | C45 H47 N7 O5 S | 797.33594 | -6.7 | 11.9 |
| 2-1-m1E04-2-0012 | C45 H45 N5 O7 S | 799.30397 | 2.7 | 1.9 |
| 2-1-m1E04-2-0013 | C46 H46 F N5 O5 S | 799.32037 | -0.6 | -1.1 |
| 2-1-m1E04-2-0014 | C44 H44 N6 O7 S | 800.29922 | -7.2 | 10.0 |
| 2-1-m1E04-2-0015 | C45 H45 F N6 O5 S | 800.31562 | 11.1 | 4.5 |
| 2-1-m1E04-2-0016 | C45 H48 N6 O6 S | 800.33560 | 25.5 | 15.1 |
| 2-1-m1E04-2-0017 | C44 H44 F N7 O5 S | 801.31087 | 13.5 | 5.4 |
| 2-1-m1E04-2-0018 | C44 H47 N7 O6 S | 801.33085 | -10.4 | 5.8 |
| 2-1-m1E04-2-0019 | C44 H47 N7 O6 S | 801.33085 | 0.0 | 25.9 |
| 2-1-m1E04-2-0020 | C44 H46 N6 O5 S2 | 802.29711 | 5.5 | -8.5 |
| 2-1-m1E04-2-0021 | C43 H46 N8 O6 S | 802.32610 | 0.0 | 0.0 |
| 2-1-m1E04-2-0022 | C43 H46 N8 O6 S | 802.32610 | 0.9 | -7.0 |
| 2-1-m1E04-2-0023 | C42 H45 N9 O6 S | 803.32135 | -0.1 | 4.4 |
| 2-1-m1E04-2-0024 | C43 H43 F N6 O5 S2 | 806.27204 | 3.9 | 8.1 |
| 2-1-m1E04-2-0025 | C42 H45 N7 O6 S2 | 807.28727 | -19.6 | 0.2 |
| 2-1-m1E04-2-0026 | C41 H44 N8 O6 S2 | 808.28252 | 0.0 | 77.4 |
| 2-1-m1E04-2-0027 | C47 H47 N5 O6 S | 809.32470 | 2.4 | 4.4 |
| 2-1-m1E04-2-0028 | C48 H51 N5 O5 S | 809.36109 | 0.0 | 0.0 |
| 2-1-m1E04-2-0029 | C46 H46 N6 O6 S | 810.31995 | -4.8 | -3.5 |
| 2-1-m1E04-2-0030 | C46 H46 N6 O6 S | 810.31995 | 1.2 | 1.9 |
| 2-1-m1E04-2-0031 | C47 H50 N6 O5 S | 810.35634 | 12.6 | 6.8 |
| 2-1-m1E04-2-0032 | C45 H45 N7 O6 S | 811.31520 | 7.0 | 0.3 |
| 2-1-m1E04-2-0033 | C47 H49 N5 O6 S | 811.34036 | 0.0 | 0.0 |
| 2-1-m1E04-2-0034 | C46 H49 N7 O5 S | 811.35159 | 0.0 | -125.4 |
| 2-1-m1E04-2-0035 | C46 H48 N6 O6 S | 812.33560 | 0.0 | 0.0 |
| 2-1-m1E04-2-0036 | C46 H47 N5 O7 S | 813.31962 | 12.9 | -17.6 |
| 2-1-m1E04-2-0037 | C45 H47 N7 O6 S | 813.33085 | 22.5 | 17.3 |
| 2-1-m1E04-2-0038 | C46 H50 N6 O6 S | 814.35125 | 0.0 | 0.0 |
| 2-1-m1E04-2-0039 | C45 H45 N5 O6 S2 | 815.28113 | 5.3 | 1.9 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0040 | C45 H49 N7 O6 S | 815.34650 | -63.0 | -67.2 |
| 2-1-m1E04-2-0041 | C44 H44 N6 O6 S2 | 816.27637 | 2.9 | 1.5 |
| 2-1-m1E04-2-0042 | C45 H48 N6 O5 S2 | 816.31276 | -35.3 | 24.8 |
| 2-1-m1E04-2-0043 | C44 H48 N8 O6 S | 816.34175 | 0.0 | 0.0 |
| 2-1-m1E04-2-0044 | C45 H44 F N5 O7 S | 817.29455 | 7.1 | 6.2 |
| 2-1-m1E04-2-0045 | C46 H45 F2 N5 O5 S | 817.31095 | -0.1 | -4.6 |
| 2-1-m1E04-2-0046 | C44 H46 N6 O6 S2 | 818.29202 | 16.7 | 1.0 |
| 2-1-m1E04-2-0047 | C45 H44 F2 N6 O5 S | 818.30620 | -30.1 | 28.2 |
| 2-1-m1E04-2-0048 | C44 H46 N6 O8 S | 818.30978 | 5.4 | 4.1 |
| 2-1-m1E04-2-0049 | C45 H47 F N6 O6 S | 818.32618 | -4.9 | -17.2 |
| 2-1-m1E04-2-0050 | C44 H43 F2 N7 O5 S | 819.30144 | 2.1 | 5.3 |
| 2-1-m1E04-2-0051 | C43 H45 N7 O8 S | 819.30503 | 3.8 | 3.3 |
| 2-1-m1E04-2-0052 | C44 H46 F N7 O6 S | 819.32143 | -9.2 | 11.2 |
| 2-1-m1E04-2-0053 | C43 H45 FN8 O6 S | 820.31668 | -16.5 | 35.8 |
| 2-1-m1E04-2-0054 | C43 H47 N7 O6 S2 | 821.30292 | 0.0 | 0.6 |
| 2-1-m1E04-2-0055 | C48 H49 N5 O6 S | 823.34036 | -26.2 | 11.4 |
| 2-1-m1E04-2-0056 | C43 H42 F2 N6 O5 S2 | 824.26262 | -2.8 | -2.7 |
| 2-1-m1E04-2-0057 | C47 H48 N6 O6 S | 824.33560 | 2.8 | 0.9 |
| 2-1-m1E04-2-0058 | C47 H48 N6 O6 S | 824.33560 | 13.2 | 5.9 |
| 2-1-m1E04-2-0059 | C42 H44 F N7 O6 S2 | 825.27785 | -18.6 | 0.0 |
| 2-1-m1E04-2-0060 | C46 H47 N7 O6 S | 825.33085 | 0.0 | -8.6 |
| 2-1-m1E04-2-0061 | C47 H46 F N5 O6 S | 827.31528 | 0.6 | 1.2 |
| 2-1-m1E04-2-0062 | C47 H46 F N5 O6 S | 827.31528 | 0.0 | 0.0 |
| 2-1-m1E04-2-0063 | C47 H49 N5 O7 S | 827.33527 | 6.2 | -18.8 |
| 2-1-m1E04-2-0064 | C46 H45 F N6 O6 S | 828.31053 | -2.6 | -0.1 |
| 2-1-m1E04-2-0065 | C46 H45 F N6 O6 S | 828.31053 | -106.5 | 0.0 |
| 2-1-m1E04-2-0066 | C46 H48 N6 O7 S | 828.33052 | 6.4 | 8.2 |
| 2-1-m1E04-2-0067 | C47 H52 N6 O6 S | 828.36690 | 6.2 | -0.5 |
| 2-1-m1E04-2-0068 | C46 H47 N5 O6 S2 | 829.29678 | 10.7 | -8.3 |
| 2-1-m1E04-2-0069 | C46 H47 N5 O8 S | 829.31453 | -4.5 | 1.2 |
| 2-1-m1E04-2-0070 | C45 H47 N7 O7 S | 829.32577 | 7.9 | 7.2 |
| 2-1-m1E04-2-0071 | C45 H47 N7 O7 S | 829.32577 | 4.8 | 4.9 |
| 2-1-m1E04-2-0072 | C46 H51 N7 O6 S | 829.36215 | 3.1 | 3.5 |
| 2-1-m1E04-2-0073 | C44 H46 N8 O7 S | 830.32102 | 4.8 | -6.4 |
| 2-1-m1E04-2-0074 | C46 H50 N6 O7 S | 830.34617 | 0.0 | -124.4 |
| 2-1-m1E04-2-0075 | C45 H50 N8 O6 S | 830.35740 | 20.8 | 15.9 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0076 | C45 H49 N7 O7 S | 831.34142 | 0.0 | 0.0 |
| 2-1-m1E04-2-0077 | C50 H49 N5 O5 S | 831.34544 | 0.0 | 0.0 |
| 2-1-m1E04-2-0078 | C45 H48 N6 O8 S | 832.32543 | -5.7 | -2.4 |
| 2-1-m1E04-2-0079 | C44 H48 N8 O7 S | 832.33667 | 16.7 | 0.0 |
| 2-1-m1E04-2-0080 | C49 H48 N6 O5 S | 832.34069 | 11.2 | -0.2 |
| 2-1-m1E04-2-0081 | C45 H44 F N5 O6 S2 | 833.27170 | -4.9 | 9.0 |
| 2-1-m1E04-2-0082 | C48 H47 N7 O5 S | 833.33594 | 9.8 | 32.3 |
| 2-1-m1E04-2-0083 | C48 H47 N7 O5 S | 833.33594 | 18.7 | -55.0 |
| 2-1-m1E04-2-0084 | C44 H46 N6 O7 S2 | 834.28694 | 5.5 | -8.6 |
| 2-1-m1E04-2-0085 | C47 H46 N8 O5 S | 834.33119 | -12.4 | 0.9 |
| 2-1-m1E04-2-0086 | C44 H45 N5 O8 S2 | 835.27095 | -4.6 | -2.6 |
| 2-1-m1E04-2-0087 | C43 H45 N7 O7 S2 | 835.28219 | 0.0 | 0.0 |
| 2-1-m1E04-2-0088 | C45 H43 F2 N5 O7 S | 835.28513 | 3.8 | -5.7 |
| 2-1-m1E04-2-0089 | C44 H49 N7 O6 S2 | 835.31857 | 7.9 | -16.7 |
| 2-1-m1E04-2-0090 | C43 H44 N6 O8 S2 | 836.26620 | 0.0 | -32.3 |
| 2-1-m1E04-2-0091 | C44 H45 F N6 O8 S | 836.30036 | 0.3 | -0.5 |
| 2-1-m1E04-2-0092 | C45 H46 F2 N6 O6 S | 836.31676 | -3.2 | -0.6 |
| 2-1-m1E04-2-0093 | C43 H47 N7 O7 S2 | 837.29784 | 26.3 | -129.8 |
| 2-1-m1E04-2-0094 | C48 H47 N5 O5 S2 | 837.30186 | 1.1 | -5.3 |
| 2-1-m1E04-2-0095 | C44 H45 F2 N7 O6 S | 837.31201 | 4.1 | 1.3 |
| 2-1-m1E04-2-0096 | C45 H51 N5 O7 S2 | 837.32299 | -0.6 | 15.8 |
| 2-1-m1E04-2-0097 | C49 H51 N5 O6 S | 837.35601 | -4.7 | 23.4 |
| 2-1-m1E04-2-0098 | C47 H46 N6 O5 S2 | 838.29711 | -16.4 | 17.0 |
| 2-1-m1E04-2-0099 | C47 H46 N6 O5 S2 | 838.29711 | 0.0 | 0.0 |
| 2-1-m1E04-2-0100 | C43 H44 F2 N8 O6 S | 838.30726 | 16.3 | 4.6 |
| 2-1-m1E04-2-0101 | C44 H50 N6 O7 S2 | 838.31824 | -12.0 | -3.4 |
| 2-1-m1E04-2-0102 | C48 H50 N6 O6 S | 838.35125 | 5.4 | 1.3 |
| 2-1-m1E04-2-0103 | C48 H50 N6 O6 S | 838.35125 | 0.0 | 0.0 |
| 2-1-m1E04-2-0104 | C48 H49 N5 O7 S | 839.33527 | -6.3 | -1.0 |
| 2-1-m1E04-2-0105 | C47 H48 N6 O7 S | 840.33052 | -3.0 | -3.5 |
| 2-1-m1E04-2-0106 | C48 H48 F N5 O6 S | 841.33093 | -22.7 | 15.3 |
| 2-1-m1E04-2-0107 | C49 H55 N5 O6 S | 841.38731 | 0.0 | 59.2 |
| 2-1-m1E04-2-0108 | C47 H47 F N6 O6 S | 842.32618 | 3.0 | 3.3 |
| 2-1-m1E04-2-0109 | C47 H50 N6 O7 S | 842.34617 | -11.1 | -4.1 |
| 2-1-m1E04-2-0110 | C48 H54 N6 O6 S | 842.38255 | 0.0 | 0.0 |
| 2-1-m1E04-2-0111 | C42 H43 F2 N7 O6 S2 | 843.26843 | 23.1 | 1.9 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0112 | C47 H49 N5 O6 S2 | 843.31243 | 30.5 | 32.6 |
| 2-1-m1E04-2-0113 | C46 H49 N7 O7 S | 843.34142 | 8.4 | -10.8 |
| 2-1-m1E04-2-0114 | C46 H49 N7 O7 S | 843.34142 | -75.1 | 13.8 |
| 2-1-m1E04-2-0115 | C45 H48 N8 O7 S | 844.33667 | -15.2 | -14.5 |
| 2-1-m1E04-2-0116 | C46 H47 N5 O7 S2 | 845.29169 | -2.1 | 0.0 |
| 2-1-m1E04-2-0117 | C46 H47 N5 O7 S2 | 845.29169 | -3.6 | -2.0 |
| 2-1-m1E04-2-0118 | C46 H47 N5 O7 S2 | 845.29169 | 32.9 | 0.0 |
| 2-1-m1E04-2-0119 | C47 H45 F2 N5 O6 S | 845.30586 | -1.7 | 4.4 |
| 2-1-m1E04-2-0120 | C47 H45 F2 N5 O6 S | 845.30586 | 0.0 | 0.0 |
| 2-1-m1E04-2-0121 | C45 H46 N6 O7 S2 | 846.28694 | 19.5 | 0.1 |
| 2-1-m1E04-2-0122 | C45 H46 N6 O7 S2 | 846.28694 | 0.0 | 0.0 |
| 2-1-m1E04-2-0123 | C45 H46 N6 O7 S2 | 846.28694 | 3.4 | 6.5 |
| 2-1-m1E04-2-0124 | C46 H44 F2 N6 O6 S | 846.30111 | 0.6 | 3.3 |
| 2-1-m1E04-2-0125 | C46 H47 F N6 O7 S | 846.32110 | 10.9 | -3.3 |
| 2-1-m1E04-2-0126 | C46 H47 F N6 O7 S | 846.32110 | 0.0 | 0.0 |
| 2-1-m1E04-2-0127 | C46 H50 N6 O8 S | 846.34108 | 4.2 | -1.6 |
| 2-1-m1E04-2-0128 | C44 H45 N7 O7 S2 | 847.28219 | 0.0 | 0.0 |
| 2-1-m1E04-2-0129 | C44 H45 N7 O7 S2 | 847.28219 | 2.2 | -6.7 |
| 2-1-m1E04-2-0130 | C45 H46 F N7 O7 S | 847.31635 | 8.3 | 3.9 |
| 2-1-m1E04-2-0131 | C45 H46 F N7 O7 S | 847.31635 | 0.0 | 0.0 |
| 2-1-m1E04-2-0132 | C49 H49 N7 O5 S | 847.35159 | 0.0 | -19.8 |
| 2-1-m1E04-2-0133 | C43 H44 N8 O7 S2 | 848.27744 | 0.0 | -22.3 |
| 2-1-m1E04-2-0134 | C45 H48 N6 O7 S2 | 848.30259 | -6.4 | -3.7 |
| 2-1-m1E04-2-0135 | C45 H48 N6 O9 S | 848.32035 | 4.1 | 7.3 |
| 2-1-m1E04-2-0136 | C45 H47 N5 O8 S2 | 849.28660 | 0.0 | -8.6 |
| 2-1-m1E04-2-0137 | C47 H46 F3 N5 O5 S | 849.31717 | 10.8 | 40.6 |
| 2-1-m1E04-2-0138 | C47 H46 F3 N5 O5 S | 849.31717 | -5.7 | -3.6 |
| 2-1-m1E04-2-0139 | C49 H47 N5 O7 S | 849.31962 | 1.0 | 40.6 |
| 2-1-m1E04-2-0140 | C46 H45 F3 N6 O5 S | 850.31242 | -0.1 | 39.3 |
| 2-1-m1E04-2-0141 | C46 H45 F3 N6 O5 S | 850.31242 | 4.1 | 11.8 |
| 2-1-m1E04-2-0142 | C44 H45 N5 O7 S3 | 851.24811 | 0.0 | -15.8 |
| 2-1-m1E04-2-0143 | C45 H43 F2 N5 O6 S2 | 851.26228 | 10.7 | 5.8 |
| 2-1-m1E04-2-0144 | C45 H44 F3 N7 O5 S | 851.30767 | -1.7 | -4.9 |
| 2-1-m1E04-2-0145 | C49 H49 N5 O5 S2 | 851.31751 | -8.3 | -23.4 |
| 2-1-m1E04-2-0146 | C48 H46 F N7 O5 S | 851.32652 | 0.0 | 0.0 |
| 2-1-m1E04-2-0147 | C46 H53 N5 O7 S2 | 851.33864 | 0.0 | 0.0 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0148 | C43 H44 N6 O7 S3 | 852.24336 | 7.1 | -29.3 |
| 2-1-m1E04-2-0149 | C43 H44 N6 O7 S3 | 852.24336 | -7.2 | 4.2 |
| 2-1-m1E04-2-0150 | C44 H45 F N6 O7 S2 | 852.27752 | 1.8 | -0.6 |
| 2-1-m1E04-2-0151 | C47 H48 N8 O6 S | 852.34175 | 0.0 | -5.5 |
| 2-1-m1E04-2-0152 | C49 H52 N6 O6 S | 852.36690 | 2.3 | -31.0 |
| 2-1-m1E04-2-0153 | C42 H43 N7 O7 S3 | 853.23861 | 1.7 | 0.0 |
| 2-1-m1E04-2-0154 | C44 H44 F N5 O8 S2 | 853.26153 | -23.2 | -36.3 |
| 2-1-m1E04-2-0155 | C46 H47 N9 O6 S | 853.33700 | 0.0 | 0.0 |
| 2-1-m1E04-2-0156 | C43 H46 N6 O9 S2 | 854.27677 | 8.0 | -9.9 |
| 2-1-m1E04-2-0157 | C44 H44 F2 N6 O8 S | 854.29094 | -13.5 | 3.4 |
| 2-1-m1E04-2-0158 | C48 H50 N6 O7 S | 854.34617 | 5.0 | -7.0 |
| 2-1-m1E04-2-0159 | C42 H45 N7 O9 S2 | 855.27202 | -1.4 | -17.4 |
| 2-1-m1E04-2-0160 | C48 H46 F N5 O5 S2 | 855.29244 | -1.4 | 29.3 |
| 2-1-m1E04-2-0161 | C45 H50 F N5 O7 S2 | 855.31357 | 3.7 | 0.2 |
| 2-1-m1E04-2-0162 | C49 H50 F N5 O6 S | 855.34658 | 0.0 | 0.0 |
| 2-1-m1E04-2-0163 | C50 H57 N5 O6 S | 855.40296 | 0.0 | 0.0 |
| 2-1-m1E04-2-0164 | C44 H43 F3 N6 O5 S2 | 856.26884 | 18.9 | -1.8 |
| 2-1-m1E04-2-0165 | C47 H48 N6 O6 S2 | 856.30767 | 0.9 | 35.6 |
| 2-1-m1E04-2-0166 | C44 H52 N6 O8 S2 | 856.32880 | 5.8 | -3.3 |
| 2-1-m1E04-2-0167 | C48 H52 N6 O7 S | 856.36182 | -2.2 | 4.0 |
| 2-1-m1E04-2-0168 | C46 H47 N7 O6 S2 | 857.30292 | 0.0 | 28.6 |
| 2-1-m1E04-2-0169 | C46 H47 N7 O6 S2 | 857.30292 | 0.0 | 0.0 |
| 2-1-m1E04-2-0170 | C43 H51 N7 O8 S2 | 857.32405 | 0.0 | 0.0 |
| 2-1-m1E04-2-0171 | C48 H48 F N5 O7 S | 857.32585 | 0.0 | 0.0 |
| 2-1-m1E04-2-0172 | C48 H51 N5 O6 S2 | 857.32808 | 0.0 | 0.0 |
| 2-1-m1E04-2-0173 | C47 H51 N7 O7 S | 857.35707 | -3.1 | -4.7 |
| 2-1-m1E04-2-0174 | C47 H51 N7 O7 S | 857.35707 | 22.0 | 22.5 |
| 2-1-m1E04-2-0175 | C45 H46 N8 O6 S2 | 858.29817 | -3.4 | -3.9 |
| 2-1-m1E04-2-0176 | C47 H50 N6 O6 S2 | 858.32332 | -31.3 | 0.0 |
| 2-1-m1E04-2-0177 | C47 H50 N6 O8 S | 858.34108 | -10.1 | 0.5 |
| 2-1-m1E04-2-0178 | C44 H45 N9 O6 S2 | 859.29342 | -8.3 | -3.0 |
| 2-1-m1E04-2-0179 | C47 H49 N5 O7 S2 | 859.30734 | 0.8 | -1.9 |
| 2-1-m1E04-2-0180 | C47 H49 N5 O7 S2 | 859.30734 | 0.8 | -0.3 |
| 2-1-m1E04-2-0181 | C47 H49 N5 O7 S2 | 859.30734 | 3.7 | -10.4 |
| 2-1-m1E04-2-0182 | C46 H49 N7 O8 S | 859.33633 | -1.8 | 5.2 |
| 2-1-mlE04-2-0183 | C51 H49 N5 O6 S | 859.34036 | 18.7 | 26.6 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0184 | C49 H54 F N5 O6 S | 859.37788 | 0.0 | -10.5 |
| 2-1-m1E04-2-0185 | C46 H48 N6 O7 S2 | 860.30259 | 6.3 | 0.0 |
| 2-1-m1E04-2-0186 | C46 H48 N6 O7 S2 | 860.30259 | -5.7 | -14.9 |
| 2-1-m1E04-2-0187 | C46 H48 N6 O7 S2 | 860.30259 | 2.0 | -35.5 |
| 2-1-m1E04-2-0188 | C47 H46 F2 N6 O6 S | 860.31676 | 18.8 | 14.6 |
| 2-1-m1E04-2-0189 | C50 H48 N6 O6 S | 860.33560 | 2.4 | -2.2 |
| 2-1-m1E04-2-0190 | C47 H49 F N6 O7 S | 860.33675 | -1.9 | -11.8 |
| 2-1-m1E04-2-0191 | C48 H56 N6 O7 S | 860.39312 | 9.7 | 1.3 |
| 2-1-m1E04-2-0192 | C45 H47 N7 O7 S2 | 861.29784 | 0.0 | 0.0 |
| 2-1-m1E04-2-0193 | C45 H47 N7 O7 S2 | 861.29784 | -3.8 | 1.5 |
| 2-1-m1E04-2-0194 | C46 H48 F N7 O7 S | 861.33200 | 13.1 | -9.5 |
| 2-1-m1E04-2-0195 | C50 H51 N7 O5 S | 861.36724 | 0.0 | 0.0 |
| 2-1-m1E04-2-0196 | C47 H55 N7 O7 S | 861.38837 | 1.2 | -15.6 |
| 2-1-m1E04-2-0197 | C46 H50 N6 O7 S2 | 862.31824 | 0.8 | 0.6 |
| 2-1-m1E04-2-0198 | C45 H45 N5 O9 S2 | 863.26587 | -6.7 | -5.3 |
| 2-1-m1E04-2-0199 | C46 H46 F N5 O7 S2 | 863.28227 | -56.1 | 0.0 |
| 2-1-m1E04-2-0200 | C46 H46 F N5 O7 S2 | 863.28227 | 1.4 | -0.3 |
| 2-1-m1E04-2-0201 | C47 H44 F3 N5 O6 S | 863.29644 | 0.0 | 0.0 |
| 2-1-m1E04-2-0202 | C46 H49 N5 O8 S2 | 863.30225 | 24.7 | 0.0 |
| 2-1-m1E04-2-0203 | C48 H48 F3 N5 O5 S | 863.33282 | 6.2 | 1.8 |
| 2-1-m1E04-2-0204 | C49 H49 N7 O6 S | 863.34650 | 3.1 | 35.5 |
| 2-1-m1E04-2-0205 | C43 H44 N8 O6 S3 | 864.25459 | 0.0 | 0.0 |
| 2-1-m1E04-2-0206 | C44 H44 N6 O9 S2 | 864.26112 | 0.0 | 0.0 |
| 2-1-m1E04-2-0207 | C45 H45 F N6 O7 S2 | 864.27752 | 14.2 | 8.6 |
| 2-1-m1E04-2-0208 | C45 H48 N6 O8 S2 | 864.29750 | 1.7 | 2.4 |
| 2-1-m1E04-2-0209 | C45 H48 N6 O8 S2 | 864.29750 | 8.0 | 1.2 |
| 2-1-m1E04-2-0210 | C45 H48 N6 O8 S2 | 864.29750 | 2.4 | -2.6 |
| 2-1-m1E04-2-0211 | C46 H46 F2 N6 O7 S | 864.31167 | -8.4 | -1.3 |
| 2-1-m1E04-2-0212 | C46 H46 F2 N6 O7 S | 864.31167 | 0.0 | 0.0 |
| 2-1-m1E04-2-0213 | C45 H47 N5 O7 S3 | 865.26376 | 0.0 | 0.0 |
| 2-1-m1E04-2-0214 | C44 H44 F N7 O7 S2 | 865.27277 | -1.8 | -8.8 |
| 2-1-m1E04-2-0215 | C45 H47 N5 O9 S2 | 865.28152 | 19.7 | 36.1 |
| 2-1-m1E04-2-0216 | C44 H47 N7 O8 S2 | 865.29275 | -5.2 | -5.1 |
| 2-1-m1E04-2-0217 | C44 H47 N7 O8 S2 | 865.29275 | 0.0 | 0.0 |
| 2-1-m1E04-2-0218 | C44 H47 N7 O8 S2 | 865.29275 | 0.0 | 0.0 |
| 2-1-m1E04-2-0219 | C49 H47 N5 O6 S2 | 865.29678 | 0.0 | 0.0 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0220 | C45 H45 F2 N7 O7 S | 865.30692 | -1.8 | -2.3 |
| 2-1-m1E04-2-0221 | C47 H46 F3 N5 O6 S | 865.31209 | 1.4 | -5.1 |
| 2-1-m1E04-2-0222 | C50 H51 N5 O5 S2 | 865.33316 | 0.0 | 28.2 |
| 2-1-m1E04-2-0223 | C47 H55 N5 O7 S2 | 865.35429 | 0.0 | 0.0 |
| 2-1-m1E04-2-0224 | C44 H46 N6 O7 S3 | 866.25901 | -2.6 | 10.0 |
| 2-1-m1E04-2-0225 | C43 H46 N8 O8 S2 | 866.28800 | 0.0 | 0.0 |
| 2-1-m1E04-2-0226 | C43 H46 N8 O8 S2 | 866.28800 | 50.9 | 34.1 |
| 2-1-m1E04-2-0227 | C46 H45 F3 N6 O6 S | 866.30734 | -2.2 | 18.9 |
| 2-1-m1E04-2-0228 | C48 H50 N8 O6 S | 866.35740 | 0.0 | 0.0 |
| 2-1-m1E04-2-0229 | C42 H45 N9 O8 S2 | 867.28325 | 12.4 | 17.6 |
| 2-1-mlE04-2-0230 | C46 H44 F3 N5 O7 S | 867.29135 | 7.0 | 8.8 |
| 2-1-m1E04-2-0231 | C45 H44 F3 N7 O6 S | 867.30259 | -3.1 | 28.4 |
| 2-1-m1E04-2-0232 | C47 H45 F4 N5 O5 S | 867.30775 | 0.3 | -4.9 |
| 2-1-m1E04-2-0233 | C49 H49 N5 O6 S2 | 867.31243 | 1.7 | 4.0 |
| 2-1-m1E04-2-0234 | C46 H53 N5 O8 S2 | 867.33356 | -94.1 | 0.0 |
| 2-1-m1E04-2-0235 | C44 H48 N6 O9 S2 | 868.29242 | 15.8 | 27.4 |
| 2-1-m1E04-2-0236 | C46 H47 F3 N6 O6 S | 868.32299 | 15.4 | 8.9 |
| 2-1-m1E04-2-0237 | C46 H47 F3 N6 O6 S | 868.32299 | -13.0 | -14.0 |
| 2-1-m1E04-2-0238 | C44 H44 F N5 O7 S3 | 869.23869 | 0.0 | 0.0 |
| 2-1-m1E04-2-0239 | C48 H45 F2 N7 O5 S | 869.31709 | -17.8 | -36.9 |
| 2-1-m1E04-2-0240 | C45 H46 F3 N7 O6 S | 869.31824 | -26.9 | 0.0 |
| 2-1-m1E04-2-0241 | C45 H46 F3 N7 O6 S | 869.31824 | 6.7 | 0.0 |
| 2-1-m1E04-2-0242 | C51 H59 N5 O6 S | 869.41861 | 0.0 | 0.0 |
| 2-1-m1E04-2-0243 | C43 H43 F N6 O7 S3 | 870.23394 | -3.1 | -2.8 |
| 2-1-m1E04-2-0244 | C43 H46 N6 O8 S3 | 870.25392 | 3.9 | -0.6 |
| 2-1-m1E04-2-0245 | C44 H44 F2 N6 O7 S2 | 870.26810 | 6.5 | -5.5 |
| 2-1-m1E04-2-0246 | C44 H45 F3 N8 O6 S | 870.31349 | 12.6 | 1.8 |
| 2-1-m1E04-2-0247 | C48 H50 N6 O6 S2 | 870.32332 | 0.0 | 1.5 |
| 2-1-m1E04-2-0248 | C47 H47 F N8 O6 S | 870.33233 | 0.0 | 0.0 |
| 2-1-m1E04-2-0249 | C45 H54 N6 O8 S2 | 870.34445 | -15.4 | 0.1 |
| 2-1-m1E04-2-0250 | C42 H45 N7 O8 S3 | 871.24917 | -51.4 | -35.5 |
| 2-1-m1E04-2-0251 | C42 H45 N7 O8 S3 | 871.24917 | 0.0 | 20.8 |
| 2-1-m1E04-2-0252 | C44 H43 F2 N5 O8 S2 | 871.25211 | -10.5 | -3.3 |
| 2-1-m1E04-2-0253 | C49 H53 N5 O6 S2 | 871.34373 | 4.9 | 12.0 |
| 2-1-m1E04-2-0254 | C49 H53 N5 O6 S2 | 871.34373 | 5.6 | 9.5 |
| 2-1-m1E04-2-0255 | C48 H53 N7 O7 S | 871.37272 | -0.5 | 0.0 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0256 | C50 H57 N5 O7 S | 871.39787 | 0.0 | 0.0 |
| 2-1-m1E04-2-0257 | C41 H44 N8 O8 S3 | 872.24442 | 0.0 | 0.0 |
| 2-1-m1E04-2-0258 | C44 H43 F3 N6 O6 S2 | 872.26376 | -4.0 | 2.1 |
| 2-1-m1E04-2-0259 | C43 H45 F N6 O9 S2 | 872.26735 | 1.5 | -0.5 |
| 2-1-m1E04-2-0260 | C48 H52 N6 O6 S2 | 872.33897 | 0.0 | 0.0 |
| 2-1-m1E04-2-0261 | C48 H45 F2 N5 O5 S2 | 873.28302 | 13.5 | 0.0 |
| 2-1-m1E04-2-0262 | C47 H47 N5 O8 S2 | 873.28660 | -5.6 | -5.1 |
| 2-1-m1E04-2-0263 | C45 H49 F2 N5 O7 S2 | 873.30415 | -1.1 | 7.4 |
| 2-1-m1E04-2-0264 | C48 H51 N5 O7 S2 | 873.32299 | 0.8 | 12.7 |
| 2-1-m1E04-2-0265 | C48 H51 N5 O7 S2 | 873.32299 | 0.0 | -6.8 |
| 2-1-m1E04-2-0266 | C48 H51 N5 O7 S2 | 873.32299 | 0.0 | -2.3 |
| 2-1-m1E04-2-0267 | C48 H51 N5 O7 S2 | 873.32299 | 0.0 | 0.0 |
| 2-1-m1E04-2-0268 | C47 H51 N7 O8 S | 873.35198 | 0.0 | 5.5 |
| 2-1-m1E04-2-0269 | C46 H46 N6 O8 S2 | 874.28185 | 7.4 | 0.0 |
| 2-1-m1E04-2-0270 | C46 H46 N6 O8 S2 | 874.28185 | -14.3 | -18.8 |
| 2-1-m1E04-2-0271 | C47 H47 F N6 O6 S2 | 874.29825 | 30.5 | 24.4 |
| 2-1-m1E04-2-0272 | C47 H50 N6 O7 S2 | 874.31824 | -0.2 | -17.3 |
| 2-1-m1E04-2-0273 | C47 H50 N6 O7 S2 | 874.31824 | -2.9 | -12.1 |
| 2-1-m1E04-2-0274 | C47 H50 N6 O7 S2 | 874.31824 | 2.4 | -22.5 |
| 2-1-m1E04-2-0275 | C44 H51 F N6 O8 S2 | 874.31938 | -6.0 | -6.6 |
| 2-1-m1E04-2-0276 | C51 H50 N6 O6 S | 874.35125 | 26.0 | -4.8 |
| 2-1-m1E04-2-0277 | C48 H51 F N6 O7 S | 874.35240 | -41.6 | 27.4 |
| 2-1-m1E04-2-0278 | C49 H58 N6 O7 S | 874.40877 | -10.5 | -39.8 |
| 2-1-m1E04-2-0279 | C43 H44 F3 N7 O6 S2 | 875.27466 | -20.6 | -7.5 |
| 2-1-m1E04-2-0280 | C45 H45 N7 O8 S2 | 875.27710 | -0.4 | -0.1 |
| 2-1-m1E04-2-0281 | C45 H45 N7 O8 S2 | 875.27710 | 18.0 | 15.5 |
| 2-1-m1E04-2-0282 | C47 H49 N5 O8 S2 | 875.30225 | 21.8 | 6.8 |
| 2-1-m1E04-2-0283 | C47 H49 N5 O8 S2 | 875.30225 | 5.9 | 0.0 |
| 2-1-m1E04-2-0284 | C46 H49 N7 O7 S2 | 875.31349 | -9.9 | -5.9 |
| 2-1-m1E04-2-0285 | C48 H50 F N5 O6 S2 | 875.31865 | -6.6 | -19.4 |
| 2-1-m1E04-2-0286 | C46 H48 N6 O8 S2 | 876.29750 | 0.0 | 0.0 |
| 2-1-m1E04-2-0287 | C47 H49 F N6 O8 S | 876.33166 | 4.3 | -17.8 |
| 2-1-m1E04-2-0288 | C47 H52 N6 O7 S2 | 876.33389 | 8.7 | -11.9 |
| 2-1-m1E04-2-0289 | C46 H47 N5 O9 S2 | 877.28152 | 1.7 | -4.5 |
| 2-1-m1E04-2-0290 | C45 H47 N7 O8 S2 | 877.29275 | -9.5 | -29.0 |
| 2-1-m1E04-2-0291 | C47 H48 F N5 O7 S2 | 877.29792 | 13.8 | 9.8 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0292 | C47 H48 F N5 O7 S2 | 877.29792 | 0.0 | -67.4 |
| 2-1-m1E04-2-0293 | C48 H46 F3 N5 O6 S | 877.31209 | -3.1 | 2.3 |
| 2-1-m1E04-2-0294 | C46 H51 N7 O7 S2 | 877.32914 | 0.0 | 0.0 |
| 2-1-m1E04-2-0295 | C51 H48 F N5 O6 S | 877.33093 | 0.0 | 0.0 |
| 2-1-m1E04-2-0296 | C49 H50 F3 N5 O5 S | 877.34848 | -11.1 | -20.7 |
| 2-1-m1E04-2-0297 | C49 H53 F2 N5 O6 S | 877.36846 | 0.0 | 24.5 |
| 2-1-m1E04-2-0298 | C46 H47 F N6 O7 S2 | 878.29317 | 0.0 | 0.0 |
| 2-1-m1E04-2-0299 | C46 H47 F N6 O7 S2 | 878.29317 | 0.0 | 0.0 |
| 2-1-m1E04-2-0300 | C47 H45 F3 N6 O6 S | 878.30734 | 12.2 | 4.3 |
| 2-1-m1E04-2-0301 | C46 H50 N6 O8 S2 | 878.31315 | 1.9 | -2.1 |
| 2-1-m1E04-2-0302 | C46 H50 N6 O8 S2 | 878.31315 | 0.6 | -2.0 |
| 2-1-m1E04-2-0303 | C46 H50 N6 O8 S2 | 878.31315 | -0.1 | -1.4 |
| 2-1-m1E04-2-0304 | C48 H55 F N6 O7 S | 878.38370 | -12.7 | -1.2 |
| 2-1-m1E04-2-0305 | C45 H45 N5 O8 S3 | 879.24303 | -6.9 | -10.2 |
| 2-1-m1E04-2-0306 | C46 H49 N5 O7 S3 | 879.27941 | -1.5 | -13.9 |
| 2-1-m1E04-2-0307 | C45 H49 N7 O8 S2 | 879.30840 | -4.2 | -2.4 |
| 2-1-m1E04-2-0308 | C45 H49 N7 O8 S2 | 879.30840 | -6.7 | 0.9 |
| 2-1-m1E04-2-0309 | C45 H49 N7 O8 S2 | 879.30840 | 14.1 | 4.9 |
| 2-1-m1E04-2-0310 | C46 H47 F2 N7 O7 S | 879.32257 | 21.4 | -45.1 |
| 2-1-m1E04-2-0311 | C48 H48 F3 N5 O6 S | 879.32774 | 4.6 | 22.4 |
| 2-1-m1E04-2-0312 | C44 H44 N6 O8 S3 | 880.23827 | 0.0 | 0.0 |
| 2-1-m1E04-2-0313 | C45 H48 N6 O7 S3 | 880.27466 | 10.4 | -4.5 |
| 2-1-m1E04-2-0314 | C44 H48 N8 O8 S2 | 880.30365 | 0.0 | 2.6 |
| 2-1-m1E04-2-0315 | C44 H48 N8 O8 S2 | 880.30365 | -26.4 | 17.0 |
| 2-1-m1E04-2-0316 | C49 H52 N8 O6 S | 880.37305 | 12.5 | 0.0 |
| 2-1-m1E04-2-0317 | C45 H44 F N5 O9 S2 | 881.25645 | -6.0 | -5.3 |
| 2-1-m1E04-2-0318 | C45 H47 N5 O8 S3 | 881.25868 | -5.0 | -6.3 |
| 2-1-m1E04-2-0319 | C46 H45 F2 N5 O7 S2 | 881.27285 | 15.8 | -21.5 |
| 2-1-mlE04-2-0320 | C46 H45 F2 N5 O7 S2 | 881.27285 | 8.8 | 3.7 |
| 2-1-m1E04-2-0321 | C44 H46 N6 O8 S3 | 882.25392 | 0.0 | -0.8 |
| 2-1-mlE04-2-0322 | C45 H44 F2 N6 O7 S2 | 882.26810 | 2.8 | -4.3 |
| 2-1-m1E04-2-0323 | C44 H46 N6 O10 S2 | 882.27168 | 2.5 | -7.4 |
| 2-1-mlE04-2-0324 | C45 H47 F N6 O8 S2 | 882.28808 | 0.2 | -0.2 |
| 2-1-m1E04-2-0325 | C45 H47 F N6 O8 S2 | 882.28808 | 3.4 | 0.3 |
| 2-1-m1E04-2-0326 | C46 H45 F3 N6 O7 S | 882.30225 | 0.0 | 0.0 |
| 2-1-mlE04-2-0327 | C45 H50 N6 O9 S2 | 882.30807 | 9.3 | 14.5 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0328 | C47 H49 F3 N6 O6 S | 882.33864 | 3.4 | -0.6 |
| 2-1-m1E04-2-0329 | C48 H50 N8 O7 S | 882.35232 | 0.0 | 0.0 |
| 2-1-m1E04-2-0330 | C44 H43 F2 N7 O7 S2 | 883.26334 | 5.4 | 3.2 |
| 2-1-m1E04-2-0331 | C43 H45 N7 O10 S2 | 883.26693 | 2.8 | -1.6 |
| 2-1-m1E04-2-0332 | C46 H44 F3 N5 O6 S2 | 883.26851 | 0.0 | 0.0 |
| 2-1-m1E04-2-0333 | C44 H46 F N7 O8 S2 | 883.28333 | 30.2 | -25.9 |
| 2-1-m1E04-2-0334 | C46 H44 F3 N5 O8 S | 883.28627 | 3.6 | -16.3 |
| 2-1-m1E04-2-0335 | C52 H49 N7 O5 S | 883.35159 | 0.0 | 15.4 |
| 2-1-m1E04-2-0336 | C44 H48 N6 O8 S3 | 884.26957 | -19.4 | -5.1 |
| 2-1-m1E04-2-0337 | C43 H45 F N8 O8 S2 | 884.27858 | 10.2 | 5.6 |
| 2-1-mlE04-2-0338 | C44 H48 N6 O10 S2 | 884.28733 | 5.9 | -0.5 |
| 2-1-m1E04-2-0339 | C46 H47 F3 N6 O7 S | 884.31790 | 15.3 | 0.5 |
| 2-1-m1E04-2-0340 | C49 H52 N6 O6 S2 | 884.33897 | -4.8 | 16.5 |
| 2-1-m1E04-2-0341 | C46 H56 N6 O8 S2 | 884.36010 | 4.3 | 1.2 |
| 2-1-m1E04-2-0342 | C43 H47 N7 O8 S3 | 885.26482 | 0.0 | 0.0 |
| 2-1-mlE04-2-0343 | C48 H47 N5 O8 S2 | 885.28660 | -4.2 | 0.0 |
| 2-1-m1E04-2-0344 | C47 H47 N7 O7 S2 | 885.29784 | -0.1 | -6.6 |
| 2-1-m1E04-2-0345 | C47 H44 F5 N5 O5 S | 885.29833 | -4.7 | 12.4 |
| 2-1-m1E04-2-0346 | C45 H46 F3 N7 O7 S | 885.31315 | 8.1 | 13.9 |
| 2-1-m1E04-2-0347 | C50 H55 N5 O6 S2 | 885.35938 | 0.0 | 0.0 |
| 2-1-m1E04-2-0348 | C50 H55 N5 O6 S2 | 885.35938 | 0.0 | -49.4 |
| 2-1-m1E04-2-0349 | C50 H55 N5 O6 S2 | 885.35938 | -1.4 | 8.7 |
| 2-1-m1E04-2-0350 | C46 H46 N8 O7 S2 | 886.29309 | -0.5 | 2.8 |
| 2-1-m1E04-2-0351 | C45 H45 F3 N6 O8 S | 886.29717 | 2.8 | -1.8 |
| 2-1-m1E04-2-0352 | C44 H45 F3 N8 O7 S | 886.30840 | 23.2 | -3.1 |
| 2-1-m1E04-2-0353 | C46 H46 F4 N6 O6 S | 886.31357 | 14.0 | -2.9 |
| 2-1-m1E04-2-0354 | C48 H50 N6 O7 S2 | 886.31824 | 13.4 | -2.9 |
| 2-1-m1E04-2-0355 | C45 H54 N6 O9 S2 | 886.33937 | 0.0 | 0.0 |
| 2-1-m1E04-2-0356 | C49 H54 N6 O6 S2 | 886.35462 | 0.0 | 0.0 |
| 2-1-m1E04-2-0357 | C44 H43 F2 N5 O7 S3 | 887.22927 | 15.8 | 25.6 |
| 2-1-m1E04-2-0358 | C48 H49 N5 O8 S2 | 887.30225 | -0.3 | 0.2 |
| 2-1-m1E04-2-0359 | C52 H49 N5 O5 S2 | 887.31751 | 25.1 | 30.7 |
| 2-1-m1E04-2-0360 | C49 H53 N5 O7 S2 | 887.33864 | 0.0 | 0.4 |
| 2-1-m1E04-2-0361 | C49 H53 N5 O7 S2 | 887.33864 | 0.0 | 0.0 |
| 2-1-m1E04-2-0362 | C49 H53 N5 O7 S2 | 887.33864 | 0.0 | -31.6 |
| 2-1-m1E04-2-0363 | C49 H53 N5 O7 S2 | 887.33864 | 0.0 | 0.0 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0364 | C43 H42 F2 N6 O7 S3 | 888.22452 | -2.3 | -5.2 |
| 2-1-m1E04-2-0365 | C43 H45 F N6 O8 S3 | 888.24450 | 0.5 | -0.9 |
| 2-1-m1E04-2-0366 | C47 H48 N6 O8 S2 | 888.29750 | -7.7 | -0.8 |
| 2-1-m1E04-2-0367 | C47 H48 N6 O8 S2 | 888.29750 | -2.5 | -5.8 |
| 2-1-m1E04-2-0368 | C47 H46 F2 N8 O6 S | 888.32291 | -11.8 | 14.4 |
| 2-1-m1E04-2-0369 | C48 H52 N6 O7 S2 | 888.33389 | 0.0 | 0.0 |
| 2-1-m1E04-2-0370 | C50 H60 N6 O7 S | 888.42442 | -0.3 | -6.2 |
| 2-1-m1E04-2-0371 | C42 H44 F N7 O8 S3 | 889.23975 | -25.9 | 7.9 |
| 2-1-m1E04-2-0372 | C46 H47 N7 O8 S2 | 889.29275 | -3.3 | -7.6 |
| 2-1-m1E04-2-0373 | C48 H51 N5 O8 S2 | 889.31791 | 10.4 | 4.4 |
| 2-1-m1E04-2-0374 | C48 H51 N5 O8 S2 | 889.31791 | 0.0 | -109.4 |
| 2-1-m1E04-2-0375 | C49 H52 F N5 O6 S2 | 889.33430 | 11.9 | 8.7 |
| 2-1-mlE04-2-0376 | C43 H44 F2 N6 O9 S2 | 890.25792 | -10.3 | -13.8 |
| 2-1-m1E04-2-0377 | C47 H50 N6 O8 S2 | 890.31315 | -29.9 | 0.5 |
| 2-1-m1E04-2-0378 | C47 H50 N6 O8 S2 | 890.31315 | -22.5 | -0.3 |
| 2-1-m1E04-2-0379 | C48 H54 N6 O7 S2 | 890.34954 | -5.0 | -0.9 |
| 2-1-m1E04-2-0380 | C48 H54 N6 O7 S2 | 890.34954 | 20.8 | 10.1 |
| 2-1-mlE04-2-0381 | C49 H58 N6 O8 S | 890.40368 | 0.0 | 0.0 |
| 2-1-m1E04-2-0382 | C45 H45 N7 O7 S3 | 891.25426 | -3.0 | 22.1 |
| 2-1-mlE04-2-0383 | C43 H44 F3 N7 O7 S2 | 891.26957 | 16.9 | 31.9 |
| 2-1-m1E04-2-0384 | C47 H46 F N5 O8 S2 | 891.27718 | -6.7 | -8.9 |
| 2-1-mlE04-2-0385 | C47 H46 F N5 O8 S2 | 891.27718 | 0.0 | 0.0 |
| 2-1-mlE04-2-0386 | C47 H49 N5 O9 S2 | 891.29717 | 0.0 | 14.8 |
| 2-1-m1E04-2-0387 | C48 H50 F N5 O7 S2 | 891.31357 | 40.3 | 17.9 |
| 2-1-mlE04-2-0388 | C48 H50 F N5 O7 S2 | 891.31357 | 0.0 | 0.0 |
| 2-1-m1E04-2-0389 | C47 H53 N7 O7 S2 | 891.34479 | 0.0 | 0.0 |
| 2-1-mlE04-2-0390 | C53 H57 N5 O6 S | 891.40296 | 0.0 | 0.0 |
| 2-1-m1E04-2-0391 | C46 H45 F N6 O8 S2 | 892.27243 | 0.0 | -9.5 |
| 2-1-m1E04-2-0392 | C46 H45 F N6 O8 S2 | 892.27243 | -6.9 | -5.8 |
| 2-1-m1E04-2-0393 | C47 H46 F2 N6 O6 S2 | 892.28883 | 0.4 | -7.2 |
| 2-1-m1E04-2-0394 | C46 H48 N6 O9 S2 | 892.29242 | -3.3 | -4.9 |
| 2-1-m1E04-2-0395 | C44 H50 F2 N6 O8 S2 | 892.30996 | 4.1 | -4.6 |
| 2-1-m1E04-2-0396 | C48 H47 F3 N6 O6 S | 892.32299 | 18.8 | -2.6 |
| 2-1-m1E04-2-0397 | C47 H52 N6 O8 S2 | 892.32880 | -1.5 | -5.7 |
| 2-1-m1E04-2-0398 | C47 H52 N6 O8 S2 | 892.32880 | -1.1 | 0.5 |
| 2-1-m1E04-2-0399 | C47 H52 N6 O8 S2 | 892.32880 | 8.3 | 3.1 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0400 | C47 H52 N6 O8 S2 | 892.32880 | 0.0 | -28.2 |
| 2-1-m1E04-2-0401 | C46 H47 N5 O8 S3 | 893.25868 | 17.8 | -7.0 |
| 2-1-m1E04-2-0402 | C46 H47 N5 O10 S2 | 893.27643 | 1.2 | -3.5 |
| 2-1-m1E04-2-0403 | C45 H47 N7 O9 S2 | 893.28767 | 0.3 | -5.7 |
| 2-1-m1E04-2-0404 | C45 H47 N7 O9 S2 | 893.28767 | 0.0 | 0.0 |
| 2-1-m1E04-2-0405 | C48 H46 F3 N5 O7 S | 893.30700 | 5.6 | 8.0 |
| 2-1-m1E04-2-0406 | C48 H49 F2 N5 O6 S2 | 893.30923 | 5.9 | 9.7 |
| 2-1-m1E04-2-0407 | C46 H51 N7 O8 S2 | 893.32405 | -8.1 | -9.4 |
| 2-1-m1E04-2-0408 | C46 H51 N7 O8 S2 | 893.32405 | -1.7 | 1.2 |
| 2-1-m1E04-2-0409 | C46 H51 N7 O8 S2 | 893.32405 | 0.0 | 0.0 |
| 2-1-m1E04-2-0410 | C44 H46 N8 O9 S2 | 894.28292 | -9.4 | -5.8 |
| 2-1-mlE04-2-0411 | C47 H45 F3 N6 O7 S | 894.30225 | -2.3 | -1.4 |
| 2-1-m1E04-2-0412 | C46 H50 N6 O9 S2 | 894.30807 | -7.6 | 5.5 |
| 2-1-mlE04-2-0413 | C46 H50 N6 O9 S2 | 894.30807 | -20.0 | -4.2 |
| 2-1-m1E04-2-0414 | C45 H50 N8 O8 S2 | 894.31930 | 8.3 | 7.1 |
| 2-1-m1E04-2-0415 | C47 H51 F N6 O7 S2 | 894.32447 | -2.9 | 0.6 |
| 2-1-mlE04-2-0416 | C47 H47 F2 N5 O7 S2 | 895.28850 | 13.0 | -2.8 |
| 2-1-m1E04-2-0417 | C47 H47 F2 N5 O7 S2 | 895.28850 | 0.0 | 0.0 |
| 2-1-mlE04-2-0418 | C48 H45 F4 N5 O6 S | 895.30267 | 0.0 | 0.0 |
| 2-1-m1E04-2-0419 | C45 H49 N7 O9 S2 | 895.30332 | -3.9 | -15.3 |
| 2-1-m1E04-2-0420 | C50 H49 N5 O7 S2 | 895.30734 | 13.7 | 2.0 |
| 2-1-mlE04-2-0421 | C50 H49 N5 O7 S2 | 895.30734 | -5.5 | -5.9 |
| 2-1-m1E04-2-0422 | C46 H46 F2 N6 O7 S2 | 896.28375 | 0.0 | 0.0 |
| 2-1-mlE04-2-0423 | C45 H48 N6 O10 S2 | 896.28733 | -5.6 | -9.0 |
| 2-1-m1E04-2-0424 | C44 H48 N8 O9 S2 | 896.29857 | 10.3 | 6.4 |
| 2-1-mlE04-2-0425 | C49 H48 N6 O7 S2 | 896.30259 | -6.4 | -4.7 |
| 2-1-m1E04-2-0426 | C46 H49 F N6 O8 S2 | 896.30373 | -5.8 | -3.0 |
| 2-1-m1E04-2-0427 | C46 H49 F N6 O8 S2 | 896.30373 | 4.3 | -2.2 |
| 2-1-mlE04-2-0428 | C47 H47 F3 N6 O7 S | 896.31790 | 3.6 | -2.8 |
| 2-1-m1E04-2-0429 | C48 H51 F3 N6 O6 S | 896.35429 | 5.2 | 1.8 |
| 2-1-m1E04-2-0430 | C48 H54 F2 N6 O7 S | 896.37428 | -2.3 | 15.3 |
| 2-1-m1E04-2-0431 | C45 H44 F N5 O8 S3 | 897.23360 | -5.0 | -6.3 |
| 2-1-mlE04-2-0432 | C48 H47 N7 O7 S2 | 897.29784 | 3.6 | -7.3 |
| 2-1-m1E04-2-0433 | C48 H47 N7 O7 S2 | 897.29784 | 0.0 | 0.0 |
| 2-1-m1E04-2-0434 | C45 H48 F N7 O8 S2 | 897.29898 | -29.9 | 31.0 |
| 2-1-m1E04-2-0435 | C45 H48 F N7 O8 S2 | 897.29898 | -4.1 | 0.0 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0436 | C46 H46 F3 N7 O7 S | 897.31315 | 0.9 | 5.0 |
| 2-1-mlE04-2-0437 | C44 H46 N6 O9 S3 | 898.24884 | 1.1 | -3.9 |
| 2-1-m1E04-2-0438 | C45 H50 N6 O8 S3 | 898.28522 | 3.1 | 0.1 |
| 2-1-m1E04-2-0439 | C47 H46 N8 O7 S2 | 898.29309 | -1.1 | -9.9 |
| 2-1-m1E04-2-0440 | C47 H49 F3 N6 O7 S | 898.33355 | 0.0 | -60.4 |
| 2-1-m1E04-2-0441 | C44 H45 N5 O10 S3 | 899.23285 | 11.4 | -0.1 |
| 2-1-m1E04-2-0442 | C43 H45 N7 O9 S3 | 899.24409 | 0.0 | 0.0 |
| 2-1-m1E04-2-0443 | C45 H43 F2 N5 O9 S2 | 899.24703 | 6.1 | 1.6 |
| 2-1-m1E04-2-0444 | C46 H44 F3 N5 O7 S2 | 899.26342 | 21.6 | 6.3 |
| 2-1-m1E04-2-0445 | C44 H49 N7 O8 S3 | 899.28047 | -3.0 | -4.9 |
| 2-1-m1E04-2-0446 | C49 H49 N5 O8 S2 | 899.30225 | 0.0 | 0.0 |
| 2-1-m1E04-2-0447 | C51 H48 F3 N5 O5 S | 899.33282 | 37.8 | -12.2 |
| 2-1-m1E04-2-0448 | C51 H57 N5 O6 S2 | 899.37503 | 0.0 | 0.0 |
| 2-1-m1E04-2-0449 | C51 H57 N5 O6 S2 | 899.37503 | 0.0 | 0.0 |
| 2-1-m1E04-2-0450 | C43 H44 N6 O10 S3 | 900.22810 | 5.2 | -12.7 |
| 2-1-m1E04-2-0451 | C44 H45 F N6 O10 S2 | 900.26226 | 4.2 | -1.0 |
| 2-1-m1E04-2-0452 | C44 H48 N6 O9 S3 | 900.26449 | 14.4 | 23.4 |
| 2-1-m1E04-2-0453 | C45 H46 F2 N6 O8 S2 | 900.27866 | 3.6 | -2.2 |
| 2-1-mlE04-2-0454 | C45 H46 F2 N6 O8 S2 | 900.27866 | -1.2 | -2.4 |
| 2-1-m1E04-2-0455 | C48 H48 N6 O8 S2 | 900.29750 | -4.4 | 6.4 |
| 2-1-mlE04-2-0456 | C47 H48 N8 O7 S2 | 900.30874 | 0.0 | 0.0 |
| 2-1-m1E04-2-0457 | C43 H47 N7 O9 S3 | 901.25974 | 36.0 | 67.3 |
| 2-1-mlE04-2-0458 | C48 H47 N5 O7 S3 | 901.26376 | -1.3 | -3.8 |
| 2-1-m1E04-2-0459 | C48 H47 N5 O7 S3 | 901.26376 | 22.1 | 21.1 |
| 2-1-mlE04-2-0460 | C48 H47 N5 O7 S3 | 901.26376 | 0.0 | 0.0 |
| 2-1-mlE04-2-0461 | C44 H45 F2 N7 O8 S2 | 901.27391 | -9.7 | 2.2 |
| 2-1-m1E04-2-0462 | C49 H51 N5 O8 S2 | 901.31791 | -2.8 | 47.7 |
| 2-1-mlE04-2-0463 | C49 H46 F3 N7 O5 S | 901.32332 | 2.4 | -9.4 |
| 2-1-m1E04-2-0464 | C50 H55 N5 O7 S2 | 901.35429 | 0.0 | 0.0 |
| 2-1-mlE04-2-0465 | C50 H55 N5 O7 S2 | 901.35429 | 0.0 | 0.0 |
| 2-1-m1E04-2-0466 | C47 H46 N6 O7 S3 | 902.25901 | -8.8 | 8.0 |
| 2-1-m1E04-2-0467 | C47 H46 N6 O7 S3 | 902.25901 | -5.4 | 4.9 |
| 2-1-mlE04-2-0468 | C47 H46 N6 O7 S3 | 902.25901 | 0.0 | 0.0 |
| 2-1-m1E04-2-0469 | C43 H44 F2 N8 O8 S2 | 902.26916 | 11.1 | 4.7 |
| 2-1-mlE04-2-0470 | C45 H45 F3 N6 O7 S2 | 902.27432 | 6.8 | -2.4 |
| 2-1-m1E04-2-0471 | C45 H45 F3 N6 O9 S | 902.29208 | 5.0 | -1.5 |

(continued)

| [Table 11-2-8] | | | | |
|---|---|---|---|---|
| | | | AS-MS rati o (%) | |
| ID | Molecular Formula | Exact Mass | N1 | N2 |
| 2-1-mlE04-2-0472 | C48 H50 N6 O8 S2 | 902.31315 | 0.0 | -7.2 |
| 2-1-m1E04-2-0473 | C48 H50 N6 O8 S2 | 902.31315 | 0.0 | 25.0 |
| 2-1-m1E04-2-0474 | C45 H44 F3 N5 O8 S2 | 903.25834 | 47.4 | 6.3 |
| 2-1-m1E04-2-0475 | C47 H46 F N7 O7 S2 | 903.28842 | -5.8 | -5.5 |
| 2-1-m1E04-2-0476 | C48 H49 N5 O9 S2 | 903.29717 | 10.8 | 12.8 |
| 2-1-m1E04-2-0477 | C49 H53 N5 O8 S2 | 903.33356 | -2.8 | -1.4 |
| 2-1-m1E04-2-0478 | C49 H53 N5 O8 S2 | 903.33356 | 0.0 | 0.0 |
| 2-1-mlE04-2-0479 | C50 H54 F N5 O6 S2 | 903.34995 | -27.7 | -79.9 |
| 2-1-mlE04-2-0480 | C47 H48 N6 O9 S2 | 904.29242 | 3.6 | -29.0 |
| 2-1-m1E04-2-0481 | C46 H45 F5 N6 O6 S | 904.30414 | 8.4 | -3.7 |
| 2-1-mlE04-2-0482 | C49 H56 N6 O7 S2 | 904.36519 | 32.7 | 6.1 |
| 2-1-m1E04-2-0483 | C49 H56 N6 O7 S2 | 904.36519 | 18.5 | -2.3 |
| 2-1-mlE04-2-0484 | C49 H56 N6 O7 S2 | 904.36519 | -10.6 | -4.1 |
| 2-1-m1E04-2-0485 | C49 H46 F3 N5 O5 S2 | 905.28925 | 39.2 | 6.0 |
| 2-1-m1E04-2-0486 | C48 H48 F N5 O8 S2 | 905.29283 | -2.0 | -3.6 |
| 2-1-m1E04-2-0487 | C46 H50 F3 N5 O7 S2 | 905.31037 | 28.9 | 13.4 |
| 2-1-m1E04-2-0488 | C49 H52 F N5 O7 S2 | 905.32922 | 20.4 | 6.9 |
| 2-1-m1E04-2-0489 | C49 H55 N5 O8 S2 | 905.34921 | 5.3 | -1.7 |
| 2-1-m1E04-2-0490 | C48 H55 N7 O7 S2 | 905.36044 | 0.0 | 0.0 |
| 2-1-m1E04-2-0491 | C43 H44 F2 N6 O8 S3 | 906.23508 | -4.0 | 7.5 |
| 2-1-m1E04-2-0492 | C47 H47 F N6 O8 S2 | 906.28808 | -6.2 | -6.7 |
| 2-1-m1E04-2-0493 | C47 H50 N6 O9 S2 | 906.30807 | -2.7 | -9.0 |
| 2-1-m1E04-2-0494 | C48 H54 N6 O8 S2 | 906.34445 | 0.1 | -1.0 |
| 2-1-m1E04-2-0495 | C48 H54 N6 O8 S2 | 906.34445 | -2.9 | -0.9 |
| 2-1-m1E04-2-0496 | C48 H54 N6 O8 S2 | 906.34445 | 0.0 | 0.0 |
| 2-1-m1E04-2-0497 | C48 H54 N6 O8 S2 | 906.34445 | 7.2 | 15.7 |
| 2-1-m1E04-2-0498 | C42 H43 F2 N7 O8 S3 | 907.23033 | 9.4 | 1.2 |
| 2-1-m1E04-2-0499 | C47 H49 N5 O8 S3 | 907.27433 | -4.0 | -7.8 |
| 2-1-m1E04-2-0500 | C46 H49 N7 O9 S2 | 907.30332 | -4.2 | -11.4 |
| 2-1-m1E04-2-0501 | C46 H49 N7 O9 S2 | 907.30332 | -4.9 | -5.1 |
| 2-1-m1E04-2-0502 | C48 H50 F N5 O8 S2 | 907.30848 | -2.4 | -8.5 |
| 2-1-mlE04-2-0503 | C48 H50 F N5 O8 S2 | 907.30848 | -4.3 | -6.7 |
| 2-1-m1E04-2-0504 | C49 H51 F2 N5 O6 S2 | 907.32488 | -43.2 | 2.1 |
| 2-1-mlE04-2-0505 | C47 H53 N7 O8 S2 | 907.33970 | 0.0 | 6.6 |
| 2-1-m1E04-2-0506 | C52 H53 N5 O6 S2 | 907.34373 | -25.4 | 18.4 |
| 2-1-mlE04-2-0507 | C45 H48 N8 O9 S2 | 908.29857 | 0.0 | 0.0 |

(continued)

| [Table 11-2-8] | | | | |
|---|---|---|---|---|
| | | | AS-MS rati o (%) | |
| ID | Molecular Formula | Exact Mass | N1 | N2 |
| 2-1-mlE04-2-0508 | C48 H47 F3 N6 O7 S | 908.31790 | -7.6 | -10.6 |
| 2-1-m1E04-2-0509 | C47 H52 N6 O9 S2 | 908.32372 | -3.8 | -1.4 |
| 2-1-mlE04-2-0510 | C47 H52 N6 O9 S2 | 908.32372 | 0.0 | 0.0 |
| 2-1-m1E04-2-0511 | C48 H53 F N6 O7 S2 | 908.34012 | 0.9 | -1.4 |
| 2-1-m1E04-2-0512 | C46 H47 N5 O9 S3 | 909.25359 | 0.8 | 13.0 |
| 2-1-m1E04-2-0513 | C46 H47 N5 O9 S3 | 909.25359 | 0.0 | 0.0 |
| 2-1-m1E04-2-0514 | C47 H45 F2 N5 O8 S2 | 909.26776 | -2.2 | -2.6 |
| 2-1-m1E04-2-0515 | C47 H45 F2 N5 O8 S2 | 909.26776 | -9.8 | -8.1 |
| 2-1-m1E04-2-0516 | C48 H49 F2 N5 O7 S2 | 909.30415 | 18.7 | -1.7 |
| 2-1-m1E04-2-0517 | C48 H47 N9 O6 S2 | 909.30907 | 5.6 | 12.2 |
| 2-1-m1E04-2-0518 | C46 H51 N7 O9 S2 | 909.31897 | -6.3 | 0.0 |
| 2-1-mlE04-2-0519 | C46 H51 N7 O9 S2 | 909.31897 | 8.7 | 8.0 |
| 2-1-m1E04-2-0520 | C51 H51 N5 O7 S2 | 909.32299 | -1.3 | -14.7 |
| 2-1-m1E04-2-0521 | C50 H54 F3 N5 O6 S | 909.37469 | 60.5 | 0.0 |
| 2-1-mlE04-2-0522 | C45 H46 N6 O9 S3 | 910.24884 | 2.2 | -3.5 |
| 2-1-m1E04-2-0523 | C46 H44 F2 N6 O8 S2 | 910.26301 | -7.8 | -10.6 |
| 2-1-mlE04-2-0524 | C46 H47 F N6 O9 S2 | 910.28300 | -3.3 | -6.9 |
| 2-1-m1E04-2-0525 | C46 H47 F N6 O9 S2 | 910.28300 | -3.0 | -8.3 |
| 2-1-mlE04-2-0526 | C46 H50 N6 O10 S2 | 910.30298 | -1.9 | -9.7 |
| 2-1-mlE04-2-0527 | C50 H50 N6 O7 S2 | 910.31824 | 4.4 | -0.1 |
| 2-1-m1E04-2-0528 | C47 H51 F N6 O8 S2 | 910.31938 | 4.0 | -0.1 |
| 2-1-mlE04-2-0529 | C47 H51 F N6 O8 S2 | 910.31938 | 0.0 | 3.1 |
| 2-1-m1E04-2-0530 | C44 H45 N7 O9 S3 | 911.24409 | 11.4 | 23.5 |
| 2-1-mlE04-2-0531 | C45 H46 F N7 O9 S2 | 911.27825 | -1.7 | -7.8 |
| 2-1-m1E04-2-0532 | C45 H46 F N7 O9 S2 | 911.27825 | 1.1 | 13.5 |
| 2-1-m1E04-2-0533 | C48 H45 F4 N5 O7 S | 911.29758 | 0.0 | 0.0 |
| 2-1-m1E04-2-0534 | C49 H49 N7 O7 S2 | 911.31349 | -21.3 | -20.9 |
| 2-1-m1E04-2-0535 | C47 H48 F3 N7 O7 S | 911.32880 | 8.5 | 0.0 |
| 2-1-m1E04-2-0536 | C45 H48 N6 O9 S3 | 912.26449 | 2.5 | -6.5 |
| 2-1-m1E04-2-0537 | C45 H48 N6 O11 S2 | 912.28225 | 3.6 | 6.8 |
| 2-1-m1E04-2-0538 | C47 H47 F3 N6 O8 S | 912.31282 | -10.3 | 2.2 |
| 2-1-m1E04-2-0539 | C47 H50 F2 N6 O7 S2 | 912.31505 | -0.5 | 3.5 |
| 2-1-m1E04-2-0540 | C45 H47 N5 O10 S3 | 913.24850 | 0.0 | 0.0 |
| 2-1-m1E04-2-0541 | C48 H47 N7 O6 S3 | 913.27499 | 14.2 | -14.8 |
| 2-1-m1E04-2-0542 | C47 H46 F3 N5 O7 S2 | 913.27907 | 5.7 | -4.5 |
| 2-1-m1E04-2-0543 | C47 H46 F3 N5 O7 S2 | 913.27907 | 0.0 | -8.1 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0544 | C47 H46 F3 N5 O7 S2 | 913.27907 | 9.6 | -3.2 |
| 2-1-mlE04-2-0545 | C47 H46 F3 N5 O7 S2 | 913.27907 | -9.3 | -7.3 |
| 2-1-m1E04-2-0546 | C49 H47 N5 O9 S2 | 913.28152 | 6.8 | -4.9 |
| 2-1-m1E04-2-0547 | C45 H51 N7 O8 S3 | 913.29612 | -34.0 | 4.5 |
| 2-1-mlE04-2-0548 | C46 H46 F3 N7 O8 S | 913.30807 | -10.6 | -3.5 |
| 2-1-m1E04-2-0549 | C50 H51 N5 O8 S2 | 913.31791 | 7.2 | 0.0 |
| 2-1-mlE04-2-0550 | C51 H55 N5 O7 S2 | 913.35429 | 5.1 | -3.0 |
| 2-1-m1E04-2-0551 | C52 H59 N5 O6 S2 | 913.39068 | 0.0 | 0.0 |
| 2-1-mlE04-2-0552 | C46 H45 F3 N6 O7 S2 | 914.27432 | 26.0 | 39.7 |
| 2-1-m1E04-2-0553 | C46 H45 F3 N6 O7 S2 | 914.27432 | 19.7 | -4.4 |
| 2-1-mlE04-2-0554 | C46 H48 F2 N6 O8 S2 | 914.29431 | -31.3 | -14.5 |
| 2-1-mlE04-2-0555 | C46 H48 F2 N6 O8 S2 | 914.29431 | 7.3 | 0.0 |
| 2-1-m1E04-2-0556 | C47 H46 F4 N6 O7 S | 914.30848 | 0.0 | 0.0 |
| 2-1-mlE04-2-0557 | C50 H54 N6 O7 S2 | 914.34954 | 26.9 | -0.2 |
| 2-1-m1E04-2-0558 | C44 H45 N5 O9 S4 | 915.21001 | 22.2 | -10.3 |
| 2-1-m1E04-2-0559 | C45 H43 F2 N5 O8 S3 | 915.22418 | -0.3 | -12.1 |
| 2-1-m1E04-2-0560 | C45 H44 F3 N7 O7 S2 | 915.26957 | 12.8 | -13.8 |
| 2-1-m1E04-2-0561 | C49 H49 N5 O7 S3 | 915.27941 | 0.0 | 0.0 |
| 2-1-mlE04-2-0562 | C49 H49 N5 O7 S3 | 915.27941 | 0.0 | 0.0 |
| 2-1-m1E04-2-0563 | C48 H46 F N7 O7 S2 | 915.28842 | 23.2 | 1.0 |
| 2-1-mlE04-2-0564 | C45 H47 F2 N7 O8 S2 | 915.28956 | 2.0 | -19.8 |
| 2-1-m1E04-2-0565 | C51 H57 N5 O7 S2 | 915.36994 | 0.0 | 0.0 |
| 2-1-mlE04-2-0566 | C43 H44 N6 O9 S4 | 916.20526 | 1.9 | -12.3 |
| 2-1-m1E04-2-0567 | C44 H45 F N6 O9 S3 | 916.23942 | 0.1 | -4.3 |
| 2-1-m1E04-2-0568 | C48 H48 N6 O7 S3 | 916.27466 | -1.2 | 24.6 |
| 2-1-mlE04-2-0569 | C47 H48 N8 O8 S2 | 916.30365 | 0.0 | 0.0 |
| 2-1-m1E04-2-0570 | C49 H52 N6 O8 S2 | 916.32880 | 11.6 | -18.3 |
| 2-1-mlE04-2-0571 | C44 H44 F N5 O10 S3 | 917.22343 | 14.9 | -7.9 |
| 2-1-m1E04-2-0572 | C47 H47 N7 O7 S3 | 917.26991 | 0.0 | 0.0 |
| 2-1-mlE04-2-0573 | C49 H48 F N5 O8 S2 | 917.29283 | 18.5 | 33.8 |
| 2-1-mlE04-2-0574 | C46 H47 N9 O8 S2 | 917.29890 | 30.0 | 36.7 |
| 2-1-m1E04-2-0575 | C48 H45 F6 N5 O5 S | 917.30456 | 13.8 | -18.7 |
| 2-1-mlE04-2-0576 | C49 H46 F3 N7 O6 S | 917.31824 | 4.8 | -6.0 |
| 2-1-m1E04-2-0577 | C50 H55 N5 O8 S2 | 917.34921 | 0.0 | 0.0 |
| 2-1-mlE04-2-0578 | C49 H55 N7 O7 S2 | 917.36044 | 10.5 | 18.5 |
| 2-1-m1E04-2-0579 | C43 H46 N6 O11 S3 | 918.23867 | -7.1 | -6.0 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0580 | C44 H44 F2 N6 O10 S2 | 918.25284 | -10.0 | -5.5 |
| 2-1-m1E04-2-0581 | C45 H45 F3 N6 O8 S2 | 918.26924 | 3.0 | 0.9 |
| 2-1-m1E04-2-0582 | C48 H50 N6 O9 S2 | 918.30807 | 7.7 | -11.1 |
| 2-1-m1E04-2-0583 | C48 H50 N6 O9 S2 | 918.30807 | 0.0 | 0.0 |
| 2-1-m1E04-2-0584 | C50 H58 N6 O7 S2 | 918.38084 | 21.9 | 15.6 |
| 2-1-m1E04-2-0585 | C50 H58 N6 O7 S2 | 918.38084 | 19.6 | 40.0 |
| 2-1-m1E04-2-0586 | C42 H45 N7 O11 S3 | 919.23392 | 18.4 | 2.6 |
| 2-1-m1E04-2-0587 | C45 H44 F3 N5 O7 S3 | 919.23550 | 0.0 | -12.4 |
| 2-1-m1E04-2-0588 | C45 H44 F3 N5 O9 S2 | 919.25325 | 29.8 | -0.7 |
| 2-1-m1E04-2-0589 | C48 H46 F N5 O7 S3 | 919.25434 | 5.6 | -7.8 |
| 2-1-m1E04-2-0590 | C48 H46 F N5 O7 S3 | 919.25434 | 27.1 | -9.1 |
| 2-1-m1E04-2-0591 | C47 H49 N7 O9 S2 | 919.30332 | 6.2 | -7.3 |
| 2-1-m1E04-2-0592 | C49 H50 F N5 O8 S2 | 919.30848 | 12.3 | 1.4 |
| 2-1-m1E04-2-0593 | C49 H53 N5 O9 S2 | 919.32847 | 0.0 | 0.0 |
| 2-1-m1E04-2-0594 | C50 H57 N5 O8 S2 | 919.36486 | 1.3 | 0.3 |
| 2-1-m1E04-2-0595 | C44 H43 F3 N6 O7 S3 | 920.23074 | -3.7 | -2.7 |
| 2-1-m1E04-2-0596 | C47 H48 N6 O8 S3 | 920.26957 | 3.8 | -1.5 |
| 2-1-mlE04-2-0597 | C47 H48 N6 O8 S3 | 920.26957 | 3.8 | -1.5 |
| 2-1-m1E04-2-0598 | C48 H52 N6 O9 S2 | 920.32372 | -7.1 | -4.5 |
| 2-1-mlE04-2-0599 | C48 H47 F3 N8 O6 S | 920.32914 | -2.6 | 11.9 |
| 2-1-m1E04-2-0600 | C49 H56 N6 O8 S2 | 920.36010 | 0.0 | 0.0 |
| 2-1-mlE04-2-0601 | C49 H56 N6 O8 S2 | 920.36010 | 0.0 | 34.8 |
| 2-1-m1E04-2-0602 | C46 H47 N7 O8 S3 | 921.26482 | 6.3 | 13.8 |
| 2-1-m1E04-2-0603 | C46 H47 N7 O8 S3 | 921.26482 | -8.4 | -2.8 |
| 2-1-m1E04-2-0604 | C46 H47 N7 O8 S3 | 921.26482 | 2.3 | -7.7 |
| 2-1-m1E04-2-0605 | C46 H47 N7 O8 S3 | 921.26482 | 0.0 | 0.0 |
| 2-1-m1E04-2-0606 | C49 H46 F3 N5 O6 S2 | 921.28416 | 0.0 | -60.6 |
| 2-1-m1E04-2-0607 | C48 H48 F N5 O9 S2 | 921.28775 | -2.1 | -5.3 |
| 2-1-m1E04-2-0608 | C46 H50 F3 N5 O8 S2 | 921.30529 | 57.1 | -11.0 |
| 2-1-m1E04-2-0609 | C47 H51 N7 O9 S2 | 921.31897 | -1.1 | -5.6 |
| 2-1-m1E04-2-0610 | C47 H51 N7 O9 S2 | 921.31897 | -1.3 | -3.5 |
| 2-1-m1E04-2-0611 | C50 H53 F2 N5 O6 S2 | 921.34053 | 36.3 | -1.5 |
| 2-1-m1E04-2-0612 | C53 H55 N5 O6 S2 | 921.35938 | 0.0 | 0.0 |
| 2-1-mlE04-2-0613 | C45 H46 N8 O8 S3 | 922.26007 | 0.0 | -27.0 |
| 2-1-m1E04-2-0614 | C44 H45 F3 N6 O9 S2 | 922.26415 | -3.2 | 0.0 |
| 2-1-m1E04-2-0615 | C47 H50 N6 O10 S2 | 922.30298 | 0.4 | -4.7 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0616 | C48 H54 N6 O9 S2 | 922.33937 | -6.6 | 5.0 |
| 2-1-m1E04-2-0617 | C48 H54 N6 O9 S2 | 922.33937 | 42.5 | 25.8 |
| 2-1-mlE04-2-0618 | C49 H55 F N6 O7 S2 | 922.35577 | 26.4 | -11.4 |
| 2-1-m1E04-2-0619 | C44 H45 N9 O8 S3 | 923.25532 | -28.6 | 0.0 |
| 2-1-mlE04-2-0620 | C47 H49 N5 O9 S3 | 923.26924 | 0.0 | -13.0 |
| 2-1-mlE04-2-0621 | C46 H49 N7 O10 S2 | 923.29823 | 0.9 | 2.2 |
| 2-1-m1E04-2-0622 | C51 H49 N5 O8 S2 | 923.30225 | -5.8 | -4.9 |
| 2-1-m1E04-2-0623 | C52 H53 N5 O7 S2 | 923.33864 | 1.4 | 13.2 |
| 2-1-m1E04-2-0624 | C49 H54 F N5 O8 S2 | 923.33978 | -11.2 | 7.0 |
| 2-1-mlE04-2-0625 | C47 H46 F2 N6 O8 S2 | 924.27866 | -11.9 | -5.8 |
| 2-1-m1E04-2-0626 | C48 H47 F3 N6 O6 S2 | 924.29506 | -329.8 | -14.1 |
| 2-1-m1E04-2-0627 | C50 H48 N6 O8 S2 | 924.29750 | -7.1 | 11.0 |
| 2-1-m1E04-2-0628 | C47 H49 F N6 O9 S2 | 924.29865 | -1.9 | -7.6 |
| 2-1-m1E04-2-0629 | C45 H51 F3 N6 O8 S2 | 924.31619 | 4.0 | -5.5 |
| 2-1-m1E04-2-0630 | C48 H53 F N6 O8 S2 | 924.33503 | 10.0 | 0.0 |
| 2-1-m1E04-2-0631 | C48 H56 N6 O9 S2 | 924.35502 | -0.1 | -2.4 |
| 2-1-m1E04-2-0632 | C47 H46 F3 N7 O6 S2 | 925.29031 | -0.1 | -4.6 |
| 2-1-m1E04-2-0633 | C46 H48 F N7 O9 S2 | 925.29390 | -9.2 | -9.8 |
| 2-1-m1E04-2-0634 | C48 H49 F2 N5 O8 S2 | 925.29906 | -4.0 | -8.7 |
| 2-1-m1E04-2-0635 | C49 H50 F3 N5 O6 S2 | 925.31546 | 23.7 | -4.4 |
| 2-1-m1E04-2-0636 | C50 H51 N7 O7 S2 | 925.32914 | 22.8 | -22.6 |
| 2-1-m1E04-2-0637 | C47 H55 N7 O9 S2 | 925.35027 | 18.1 | -9.4 |
| 2-1-m1E04-2-0638 | C50 H54 F3 N5 O7 S | 925.36960 | 38.7 | 23.5 |
| 2-1-m1E04-2-0639 | C46 H50 N6 O9 S3 | 926.28014 | -4.0 | 0.9 |
| 2-1-m1E04-2-0640 | C47 H51 F N6 O9 S2 | 926.31430 | 11.1 | -8.9 |
| 2-1-m1E04-2-0641 | C47 H51 F N6 O9 S2 | 926.31430 | -16.6 | 1.8 |
| 2-1-m1E04-2-0642 | C48 H52 F2 N6 O7 S2 | 926.33070 | 0.0 | 0.0 |
| 2-1-m1E04-2-0643 | C44 H45 N7 O8 S4 | 927.22124 | -6.6 | 6.0 |
| 2-1-m1E04-2-0644 | C46 H46 F N5 O9 S3 | 927.24417 | 20.0 | -2.0 |
| 2-1-m1E04-2-0645 | C47 H44 F3 N5 O8 S2 | 927.25834 | -7.8 | -4.7 |
| 2-1-m1E04-2-0646 | C46 H49 N5 O10 S3 | 927.26415 | 7.9 | -7.7 |
| 2-1-m1E04-2-0647 | C48 H48 F3 N5 O7 S2 | 927.29472 | 1.8 | -0.5 |
| 2-1-m1E04-2-0648 | C48 H48 F3 N5 O7 S2 | 927.29472 | 5.6 | -27.7 |
| 2-1-m1E04-2-0649 | C49 H49 N7 O8 S2 | 927.30840 | 15.7 | 2.4 |
| 2-1-m1E04-2-0650 | C51 H50 F N5 O7 S2 | 927.31357 | 5.8 | -16.7 |
| 2-1-m1E04-2-0651 | C47 H48 F3 N7 O8 S | 927.32372 | 0.0 | 0.9 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0652 | C51 H53 N5 O8 S2 | 927.33356 | 0.0 | 0.0 |
| 2-1-mlE04-2-0653 | C52 H57 N5 O7 S2 | 927.36994 | 12.0 | 14.9 |
| 2-1-m1E04-2-0654 | C43 H44 N8 O8 S4 | 928.21649 | 0.0 | -13.7 |
| 2-1-m1E04-2-0655 | C45 H48 N6 O10 S3 | 928.25940 | -17.6 | -4.4 |
| 2-1-m1E04-2-0656 | C45 H48 N6 O10 S3 | 928.25940 | -12.1 | -10.4 |
| 2-1-m1E04-2-0657 | C46 H46 F2 N6 O9 S2 | 928.27357 | -7.0 | -12.6 |
| 2-1-mlE04-2-0658 | C46 H46 F2 N6 O9 S2 | 928.27357 | 3.5 | -3.6 |
| 2-1-m1E04-2-0659 | C47 H47 F3 N6 O7 S2 | 928.28997 | 2.1 | 0.0 |
| 2-1-mlE04-2-0660 | C47 H50 F2 N6 O8 S2 | 928.30996 | 8.7 | -4.7 |
| 2-1-m1E04-2-0661 | C49 H55 F3 N6 O7 S | 928.38050 | 16.8 | -19.1 |
| 2-1-m1E04-2-0662 | C45 H47 N5 O9 S4 | 929.22566 | 0.0 | 0.0 |
| 2-1-mlE04-2-0663 | C45 H47 N5 O11 S3 | 929.24342 | 7.3 | -2.8 |
| 2-1-m1E04-2-0664 | C44 H47 N7 O10 S3 | 929.25465 | 0.0 | 0.0 |
| 2-1-mlE04-2-0665 | C49 H47 N5 O8 S3 | 929.25868 | 0.0 | 0.0 |
| 2-1-m1E04-2-0666 | C45 H45 F2 N7 O9 S2 | 929.26882 | -3.6 | -1.6 |
| 2-1-mlE04-2-0667 | C47 H46 F3 N5 O8 S2 | 929.27399 | 0.0 | 1.7 |
| 2-1-mlE04-2-0668 | C47 H46 F3 N5 O8 S2 | 929.27399 | 31.8 | 13.3 |
| 2-1-m1E04-2-0669 | C50 H51 N5 O7 S3 | 929.29506 | 14.8 | 13.5 |
| 2-1-mlE04-2-0670 | C50 H51 N5 O7 S3 | 929.29506 | 0.0 | 0.0 |
| 2-1-m1E04-2-0671 | C50 H51 N5 O9 S2 | 929.31282 | 0.9 | -11.0 |
| 2-1-mlE04-2-0672 | C46 H45 F3 N6 O8 S2 | 930.26924 | -10.2 | -1.6 |
| 2-1-m1E04-2-0673 | C49 H50 N6 O7 S3 | 930.29031 | -10.2 | -19.4 |
| 2-1-m1E04-2-0674 | C47 H46 F4 N6 O8 S | 930.30340 | 9.4 | -7.0 |
| 2-1-m1E04-2-0675 | C48 H50 N8 O8 S2 | 930.31930 | -6.4 | -1.6 |
| 2-1-m1E04-2-0676 | C46 H44 F3 N5 O9 S2 | 931.25325 | -0.7 | -5.1 |
| 2-1-m1E04-2-0677 | C45 H44 F3 N7 O8 S2 | 931.26449 | -22.0 | -20.2 |
| 2-1-m1E04-2-0678 | C47 H45 F4 N5 O7 S2 | 931.26965 | 22.1 | 16.9 |
| 2-1-m1E04-2-0679 | C49 H49 N5 O8 S3 | 931.27433 | -4.4 | -18.0 |
| 2-1-m1E04-2-0680 | C49 H49 N5 O8 S3 | 931.27433 | 30.1 | 45.4 |
| 2-1-m1E04-2-0681 | C51 H54 F N5 O7 S2 | 931.34487 | -6.6 | 6.1 |
| 2-1-m1E04-2-0682 | C44 H48 N6 O11 S3 | 932.25432 | -3.3 | -8.4 |
| 2-1-m1E04-2-0683 | C46 H47 F3 N6 O8 S2 | 932.28489 | 0.5 | -3.8 |
| 2-1-m1E04-2-0684 | C46 H47 F3 N6 O8 S2 | 932.28489 | 1.4 | 2.4 |
| 2-1-m1E04-2-0685 | C46 H47 F3 N6 O8 S2 | 932.28489 | 2.4 | 2.6 |
| 2-1-m1E04-2-0686 | C46 H47 F3 N6 O8 S2 | 932.28489 | 13.6 | 0.0 |
| 2-1-m1E04-2-0687 | C49 H52 N6 O9 S2 | 932.32372 | 4.3 | -1.0 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0688 | C50 H56 N6 O8 S2 | 932.36010 | 10.8 | -5.5 |
| 2-1-m1E04-2-0689 | C51 H60 N6 O7 S2 | 932.39649 | 20.1 | 6.2 |
| 2-1-m1E04-2-0690 | C44 H44 F N5 O9 S4 | 933.20059 | 4.8 | 20.6 |
| 2-1-m1E04-2-0691 | C48 H45 F2 N7 O7 S2 | 933.27899 | 0.0 | 0.0 |
| 2-1-m1E04-2-0692 | C45 H46 F3 N7 O8 S2 | 933.28014 | -4.2 | -10.1 |
| 2-1-m1E04-2-0693 | C45 H46 F3 N7 O8 S2 | 933.28014 | 0.0 | 0.0 |
| 2-1-m1E04-2-0694 | C48 H45 F6 N5 O6 S | 933.29947 | -8.6 | 41.3 |
| 2-1-mlE04-2-0695 | C48 H51 N7 O7 S3 | 933.30121 | -23.9 | 33.8 |
| 2-1-mlE04-2-0696 | C49 H55 N7 O8 S2 | 933.35535 | 0.0 | -12.9 |
| 2-1-m1E04-2-0697 | C51 H59 N5 O8 S2 | 933.38051 | -19.4 | -3.4 |
| 2-1-m1E04-2-0698 | C43 H46 N6 O10 S4 | 934.21582 | -5.9 | -6.8 |
| 2-1-m1E04-2-0699 | C44 H44 F2 N6 O9 S3 | 934.23000 | -9.9 | -2.7 |
| 2-1-m1E04-2-0700 | C48 H47 F N6 O7 S3 | 934.26524 | 9.0 | 33.8 |
| 2-1-m1E04-2-0701 | C44 H45 F3 N8 O8 S2 | 934.27539 | 1.3 | 12.0 |
| 2-1-m1E04-2-0702 | C48 H50 N6 O8 S3 | 934.28522 | 3.7 | 0.7 |
| 2-1-m1E04-2-0703 | C48 H50 N6 O8 S3 | 934.28522 | 16.7 | -2.0 |
| 2-1-m1E04-2-0704 | C47 H47 F N8 O8 S2 | 934.29423 | -20.5 | -14.9 |
| 2-1-m1E04-2-0705 | C50 H58 N6 O8 S2 | 934.37575 | 0.0 | 0.0 |
| 2-1-m1E04-2-0706 | C42 H45 N7 O10 S4 | 935.21107 | 16.3 | -12.8 |
| 2-1-mlE04-2-0707 | C44 H43 F2 N5 O10 S3 | 935.21401 | -3.0 | -9.6 |
| 2-1-m1E04-2-0708 | C45 H44 F3 N5 O8 S3 | 935.23041 | -12.0 | -0.2 |
| 2-1-m1E04-2-0709 | C47 H49 N7 O8 S3 | 935.28047 | 5.4 | 0.7 |
| 2-1-mlE04-2-0710 | C47 H49 N7 O8 S3 | 935.28047 | -7.6 | -5.7 |
| 2-1-m1E04-2-0711 | C49 H47 F2 N5 O8 S2 | 935.28341 | -2.1 | 10.2 |
| 2-1-m1E04-2-0712 | C48 H53 N7 O9 S2 | 935.33462 | -8.1 | -1.0 |
| 2-1-m1E04-2-0713 | C50 H57 N5 O9 S2 | 935.35977 | 0.0 | 0.0 |
| 2-1-m1E04-2-0714 | C54 H57 N5 O6 S2 | 935.37503 | 0.0 | 0.0 |
| 2-1-m1E04-2-0715 | C44 H43 F3 N6 O8 S3 | 936.22566 | -28.8 | -9.5 |
| 2-1-m1E04-2-0716 | C43 H45 FN6 O11 S3 | 936.22925 | -2.8 | -3.2 |
| 2-1-m1E04-2-0717 | C46 H48 N8 O8 S3 | 936.27572 | -14.4 | -9.9 |
| 2-1-m1E04-2-0718 | C46 H48 N8 O8 S3 | 936.27572 | 0.0 | 0.0 |
| 2-1-mlE04-2-0719 | C48 H49 F N6 O9 S2 | 936.29865 | 2.8 | -0.4 |
| 2-1-m1E04-2-0720 | C47 H46 F6 N6 O6 S | 936.31037 | 3.8 | 3.7 |
| 2-1-m1E04-2-0721 | C48 H47 F3 N8 O7 S | 936.32405 | 8.6 | 2.4 |
| 2-1-m1E04-2-0722 | C49 H56 N6 O9 S2 | 936.35502 | -1.6 | -4.6 |
| 2-1-m1E04-2-0723 | C48 H45 F2 N5 O7 S3 | 937.24492 | -1.9 | 3.4 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0724 | C48 H45 F2 N5 O7 S3 | 937.24492 | 0.0 | 0.0 |
| 2-1-m1E04-2-0725 | C48 H51 N5 O9 S3 | 937.28489 | 7.1 | 3.9 |
| 2-1-m1E04-2-0726 | C47 H51 N7 O10 S2 | 937.31388 | 8.6 | 3.7 |
| 2-1-m1E04-2-0727 | C44 H45 F3 N6 O8 S3 | 938.24131 | -2.7 | 0.2 |
| 2-1-m1E04-2-0728 | C44 H45 F3 N6 O10 S2 | 938.25907 | 4.7 | 2.2 |
| 2-1-m1E04-2-0729 | C47 H47 F N6 O8 S3 | 938.26015 | 5.2 | 2.2 |
| 2-1-m1E04-2-0730 | C47 H47 F N6 O8 S3 | 938.26015 | 17.2 | 2.0 |
| 2-1-m1E04-2-0731 | C51 H50 N6 O8 S2 | 938.31315 | -10.5 | -4.0 |
| 2-1-m1E04-2-0732 | C48 H51 F N6 O9 S2 | 938.31430 | 24.0 | -4.4 |
| 2-1-m1E04-2-0733 | C48 H54 N6 O10 S2 | 938.33428 | 8.6 | -7.6 |
| 2-1-m1E04-2-0734 | C49 H58 N6 O9 S2 | 938.37067 | -6.5 | -7.0 |
| 2-1-m1E04-2-0735 | C43 H44 F3 N7 O8 S3 | 939.23656 | -41.4 | -20.4 |
| 2-1-m1E04-2-0736 | C45 H45 N7 O10 S3 | 939.23900 | -19.8 | -5.0 |
| 2-1-m1E04-2-0737 | C47 H49 N5 O10 S3 | 939.26415 | 3.6 | 4.3 |
| 2-1-m1E04-2-0738 | C50 H52 F3 N5 O6 S2 | 939.33111 | 4.3 | -18.1 |
| 2-1-m1E04-2-0739 | C52 H53 N5 O8 S2 | 939.33356 | 0.0 | 1.6 |
| 2-1-m1E04-2-0740 | C48 H47 F3 N6 O7 S2 | 940.28997 | 8.3 | 11.6 |
| 2-1-m 1 E04-2-0741 | C47 H49 F N6 O10 S2 | 940.29356 | 3.8 | -1.6 |
| 2-1-m 1 E04-2-0742 | C45 H51 F3 N6 O9 S2 | 940.31110 | 0.6 | -0.2 |
| 2-1-m1E04-2-0743 | C49 H54 F2 N6 O7 S2 | 940.34635 | 7.0 | -1.8 |
| 2-1-m 1 E04-2-0744 | C48 H46 F3 N5 O8 S2 | 941.27399 | 4.0 | 1.1 |
| 2-1-m1E04-2-0745 | C51 H48 F N5 O8 S2 | 941.29283 | 9.7 | -14.6 |
| 2-1-m1E04-2-0746 | C49 H50 F3 N5 O7 S2 | 941.31037 | -6.2 | -5.1 |
| 2-1-m1E04-2-0747 | C49 H50 F3 N5 O7 S2 | 941.31037 | 0.0 | 0.0 |
| 2-1-m1E04-2-0748 | C49 H50 F3 N5 O7 S2 | 941.31037 | -50.8 | 2.8 |
| 2-1-m1E04-2-0749 | C49 H53 F2 N5 O8 S2 | 941.33036 | 1.5 | -6.1 |
| 2-1-m1E04-2-0750 | C53 H59 N5 O7 S2 | 941.38559 | 0.0 | 0.0 |
| 2-1-m1E04-2-0751 | C47 H45 F3 N6 O8 S2 | 942.26924 | -0.2 | 2.7 |
| 2-1-m1E04-2-0752 | C46 H50 N6 O10 S3 | 942.27505 | -8.6 | -0.7 |
| 2-1-m1E04-2-0753 | C48 H55 F N6 O9 S2 | 942.34560 | 1.5 | -6.2 |
| 2-1-m1E04-2-0754 | C46 H49 N5 O9 S4 | 943.24131 | -3.0 | 9.5 |
| 2-1-m1E04-2-0755 | C46 H47 F2 N7 O9 S2 | 943.28447 | -6.2 | -13.3 |
| 2-1-m1E04-2-0756 | C48 H48 F3 N5 O8 S2 | 943.28964 | 37.1 | 39.8 |
| 2-1-m 1 E04-2-0757 | C48 H48 F3 N5 O8 S2 | 943.28964 | 11.4 | -2.2 |
| 2-1-m1E04-2-0758 | C52 H57 N5 O8 S2 | 943.36486 | -3.5 | 3.4 |
| 2-1-m 1 E04-2-0759 | C47 H47 F3 N6 O8 S2 | 944.28489 | 0.0 | 0.0 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0760 | C47 H50 F2 N6 O9 S2 | 944.30488 | -0.1 | 10.5 |
| 2-1-m1E04-2-0761 | C50 H52 N6 O7 S3 | 944.30596 | -2.7 | 10.5 |
| 2-1-m1E04-2-0762 | C48 H51 F3 N6 O7 S2 | 944.32127 | -3.7 | 12.8 |
| 2-1-m1E04-2-0763 | C49 H52 N8 O8 S2 | 944.33495 | -6.3 | -1.8 |
| 2-1-m1E04-2-0764 | C49 H55 F3 N6 O8 S | 944.37542 | -2.6 | -4.8 |
| 2-1-m1E04-2-0765 | C45 H47 N5 O10 S4 | 945.22058 | -26.2 | -27.7 |
| 2-1-m1E04-2-0766 | C46 H45 F2 N5 O9 S3 | 945.23475 | 12.0 | -23.6 |
| 2-1-m1E04-2-0767 | C48 H47 F4 N5 O7 S2 | 945.28530 | 0.0 | 0.0 |
| 2-1-m1E04-2-0768 | C45 H47 F N6 O10 S3 | 946.24998 | -10.9 | -1.3 |
| 2-1-m1E04-2-0769 | C46 H45 F3 N6 O9 S2 | 946.26415 | -10.3 | -10.3 |
| 2-1-m1E04-2-0770 | C45 H50 N6 O11 S3 | 946.26997 | -0.9 | -6.6 |
| 2-1-m1E04-2-0771 | C49 H50 N6 O8 S3 | 946.28522 | 0.0 | 15.1 |
| 2-1-m1E04-2-0772 | C47 H49 F3 N6 O8 S2 | 946.30054 | -2.6 | -6.2 |
| 2-1-m1E04-2-0773 | C47 H49 F3 N6 O8 S2 | 946.30054 | 2.3 | -2.5 |
| 2-1-m1E04-2-0774 | C48 H50 N8 O9 S2 | 946.31422 | -6.9 | 25.2 |
| 2-1-m1E04-2-0775 | C50 H54 N6 O9 S2 | 946.33937 | -2.2 | 2.0 |
| 2-1-m1E04-2-0776 | C51 H58 N6 O8 S2 | 946.37575 | 2.7 | 6.6 |
| 2-1-m1E04-2-0777 | C46 H44 F3 N5 O8 S3 | 947.23041 | -3.4 | 4.1 |
| 2-1-m1E04-2-0778 | C46 H44 F3 N5 O10 S2 | 947.24817 | 13.5 | 3.9 |
| 2-1-m1E04-2-0779 | C46 H48 F3 N7 O8 S2 | 947.29579 | 15.9 | -28.5 |
| 2-1-m1E04-2-0780 | C52 H49 N7 O7 S2 | 947.31349 | 0.0 | 0.0 |
| 2-1-m1E04-2-0781 | C49 H53 N7 O7 S3 | 947.31686 | 16.7 | 0.0 |
| 2-1-m1E04-2-0782 | C44 H48 N6 O10 S4 | 948.23147 | -3.1 | -4.3 |
| 2-1-m1E04-2-0783 | C44 H48 N6 O12 S3 | 948.24923 | -19.9 | 4.7 |
| 2-1-m1E04-2-0784 | C46 H47 F3 N6 O9 S2 | 948.27980 | -1.1 | -2.5 |
| 2-1-m1E04-2-0785 | C46 H47 F3 N6 O9 S2 | 948.27980 | 0.1 | -1.4 |
| 2-1-m1E04-2-0786 | C49 H52 N6 O8 S3 | 948.30087 | 5.8 | 5.3 |
| 2-1-m1E04-2-0787 | C49 H52 N6 O8 S3 | 948.30087 | -11.4 | 1.8 |
| 2-1-m1E04-2-0788 | C49 H52 N6 O10 S2 | 948.31863 | 1.7 | 27.9 |
| 2-1-m1E04-2-0789 | C48 H47 N5 O10 S3 | 949.24850 | 31.4 | 50.4 |
| 2-1-m1E04-2-0790 | C47 H47 N7 O9 S3 | 949.25974 | -11.9 | -6.5 |
| 2-1-m1E04-2-0791 | C47 H44 F5 N5 O7 S2 | 949.26023 | 14.9 | -4.3 |
| 2-1-m1E04-2-0792 | C45 H46 F3 N7 O9 S2 | 949.27505 | 0.1 | 2.5 |
| 2-1-m1E04-2-0793 | C48 H51 N7 O8 S3 | 949.29612 | -9.2 | 3.5 |
| 2-1-m1E04-2-0794 | C48 H51 N7 O8 S3 | 949.29612 | 9.5 | 14.7 |
| 2-1-m1E04-2-0795 | C53 H51 N5 O8 S2 | 949.31791 | 21.3 | -3.7 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0796 | C51 H53 F2 N5 O7 S2 | 949.33545 | 14.0 | -12.4 |
| 2-1-m1E04-2-0797 | C46 H46 N8 O9 S3 | 950.25499 | -13.7 | -14.6 |
| 2-1-m1E04-2-0798 | C45 H45 F3 N6 O10 S2 | 950.25907 | -1.2 | -4.7 |
| 2-1-m1E04-2-0799 | C44 H45 F3 N8 O9 S2 | 950.27030 | 26.0 | -6.6 |
| 2-1-m1E04-2-0800 | C46 H46 F4 N6 O8 S2 | 950.27547 | 0.1 | -4.0 |
| 2-1-m1E04-2-0801 | C48 H50 N6 O9 S3 | 950.28014 | 3.2 | 2.6 |
| 2-1-m1E04-2-0802 | C48 H50 N6 O9 S3 | 950.28014 | -5.4 | -9.1 |
| 2-1-m1E04-2-0803 | C50 H55 F N6 O8 S2 | 950.35068 | 13.2 | 8.5 |
| 2-1-m1E04-2-0804 | C44 H43 F2 N5 O9 S4 | 951.19117 | 0.7 | 15.6 |
| 2-1-m 1 E04-2-0805 | C52 H49 N5 O7 S3 | 951.27941 | 6.6 | 5.6 |
| 2-1-m 1 E04-2-0806 | C52 H49 N5 O7 S3 | 951.27941 | -4.4 | -2.7 |
| 2-1-m 1 E04-2-0807 | C43 H45 FN6 O10 S4 | 952.20640 | -9.0 | -11.0 |
| 2-1-m1E04-2-0808 | C48 H46 F2 N6 O7 S3 | 952.25582 | 8.5 | 1.5 |
| 2-1-m 1 E04-2-0809 | C47 H46 F2 N8 O8 S2 | 952.28481 | -6.0 | -20.4 |
| 2-1-m 1 E04-2-0810 | C47 H46 F6 N6 O7 S | 952.30529 | 11.1 | -17.2 |
| 2-1-m1E04-2-0811 | C50 H60 N6 O9 S2 | 952.38632 | -3.4 | -7.8 |
| 2-1-m1E04-2-0812 | C47 H48 F N7 O8 S3 | 953.27105 | 5.4 | -1.0 |
| 2-1-m 1 E04-2-0813 | C47 H48 F N7 O8 S3 | 953.27105 | 0.0 | 0.0 |
| 2-1-m1E04-2-0814 | C51 H54 F3 N5 O6 S2 | 953.34676 | 0.0 | 0.0 |
| 2-1-m1E04-2-0815 | C43 H44 F2 N6 O11 S3 | 954.21982 | -1.6 | -9.4 |
| 2-1-m1E04-2-0816 | C44 H45 F3 N6 O9 S3 | 954.23622 | 1.2 | 2.2 |
| 2-1-m1E04-2-0817 | C48 H48 F2 N6 O9 S2 | 954.28922 | 5.6 | 0.4 |
| 2-1-m1E04-2-0818 | C49 H58 N6 O10 S2 | 954.36558 | 0.0 | 0.0 |
| 2-1-m1E04-2-0819 | C45 H45 N7 O9 S4 | 955.21616 | -15.9 | -2.2 |
| 2-1-m1E04-2-0820 | C43 H44 F3 N7 O9 S3 | 955.23147 | 10.0 | -4.0 |
| 2-1-m1E04-2-0821 | C50 H52 F3 N5 O7 S2 | 955.32603 | -16.5 | -49.7 |
| 2-1-m1E04-2-0822 | C53 H57 N5 O8 S2 | 955.36486 | -29.7 | 0.0 |
| 2-1-m1E04-2-0823 | C47 H46 F2 N6 O8 S3 | 956.25073 | 3.6 | -0.8 |
| 2-1-m1E04-2-0824 | C47 H46 F2 N6 O8 S3 | 956.25073 | 0.0 | 0.0 |
| 2-1-m 1 E04-2-0825 | C48 H47 F3 N6 O8 S2 | 956.28489 | 10.5 | -19.2 |
| 2-1-m 1 E04-2-0826 | C47 H52 N6 O10 S3 | 956.29070 | -5.6 | -7.2 |
| 2-1-m 1 E04-2-0827 | C48 H46 F3 N5 O9 S2 | 957.26890 | -3.1 | -25.3 |
| 2-1-m 1 E04-2-0828 | C49 H50 F3 N5 O8 S2 | 957.30529 | 0.0 | -3.4 |
| 2-1-m 1 E04-2-0829 | C49 H50 F3 N5 O8 S2 | 957.30529 | 1.9 | -2.6 |
| 2-1-m1E04-2-0830 | C47 H45 F3 N6 O9 S2 | 958.26415 | 4.2 | -11.6 |
| 2-1-m1E04-2-0831 | C46 H50 N6 O11 S3 | 958.26997 | -6.5 | -8.6 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0832 | C49 H53 F3 N6 O7 S2 | 958.33692 | 0.0 | 10.5 |
| 2-1-m1E04-2-0833 | C48 H45 F4 N5 O8 S2 | 959.26457 | 2.4 | 15.4 |
| 2-1-m1E04-2-0834 | C50 H49 N5 O9 S3 | 959.26924 | 6.9 | -4.4 |
| 2-1-m1E04-2-0835 | C47 H47 F3 N6 O9 S2 | 960.27980 | -3.9 | -7.5 |
| 2-1-m1E04-2-0836 | C48 H51 F3 N6 O8 S2 | 960.31619 | -4.3 | -0.8 |
| 2-1-m1E04-2-0837 | C48 H51 F3 N6 O8 S2 | 960.31619 | 3.3 | 11.3 |
| 2-1-m1E04-2-0838 | C48 H51 F3 N6 O8 S2 | 960.31619 | -4.3 | -0.8 |
| 2-1-m1E04-2-0839 | C48 H54 F2 N6 O9 S2 | 960.33618 | -7.3 | 4.0 |
| 2-1-m 1 E04-2-0840 | C52 H60 N6 O8 S2 | 960.39140 | 8.6 | 4.7 |
| 2-1-m 1 E04-2-0841 | C46 H46 F3 N7 O9 S2 | 961.27505 | -2.9 | -6.9 |
| 2-1-m1E04-2-0842 | C48 H47 F4 N5 O8 S2 | 961.28022 | -8.5 | -20.3 |
| 2-1-m1E04-2-0843 | C50 H55 N7 O7 S3 | 961.33251 | 0.0 | 0.0 |
| 2-1-m1E04-2-0844 | C45 H50 N6 O10 S4 | 962.24712 | -1.2 | -1.8 |
| 2-1-m1E04-2-0845 | C47 H49 F3 N6 O9 S2 | 962.29545 | 7.7 | -2.2 |
| 2-1-m1E04-2-0846 | C47 H49 F3 N6 O9 S2 | 962.29545 | 1.5 | -5.1 |
| 2-1-m1E04-2-0847 | C51 H58 N6 O9 S2 | 962.37067 | 0.0 | 0.0 |
| 2-1-m1E04-2-0848 | C46 H44 F3 N5 O9 S3 | 963.22532 | 9.5 | 2.5 |
| 2-1-m1E04-2-0849 | C49 H49 N5 O10 S3 | 963.26415 | 0.0 | 0.0 |
| 2-1-m1E04-2-0850 | C46 H48 F3 N7 O9 S2 | 963.29070 | 5.9 | 0.0 |
| 2-1-m1E04-2-0851 | C51 H48 F3 N5 O7 S2 | 963.29472 | -3.1 | -4.6 |
| 2-1-m1E04-2-0852 | C49 H53 N7 O8 S3 | 963.31177 | 0.9 | 2.4 |
| 2-1-m1E04-2-0853 | C55 H57 N5 O7 S2 | 963.36994 | 1.8 | -6.3 |
| 2-1-m1E04-2-0854 | C44 H48 N6 O11 S4 | 964.22639 | 3.4 | 6.5 |
| 2-1-m1E04-2-0855 | C45 H46 F2 N6 O10 S3 | 964.24056 | -2.0 | -10.7 |
| 2-1-m1E04-2-0856 | C48 H48 N6 O10 S3 | 964.25940 | 8.8 | -15.4 |
| 2-1-m1E04-2-0857 | C47 H48 N8 O9 S3 | 964.27064 | 0.0 | -14.2 |
| 2-1-m1E04-2-0858 | C47 H48 F4 N6 O8 S2 | 964.29112 | -30.3 | -3.0 |
| 2-1-m1E04-2-0859 | C48 H47 N5 O9 S4 | 965.22566 | 0.0 | -10.5 |
| 2-1-m 1 E04-2-0860 | C48 H47 N5 O9 S4 | 965.22566 | 0.8 | 24.8 |
| 2-1-m1E04-2-0861 | C49 H46 F3 N7 O7 S2 | 965.28522 | -8.4 | 4.7 |
| 2-1-m1E04-2-0862 | C48 H51 N7 O9 S3 | 965.29104 | 9.8 | 11.7 |
| 2-1-m1E04-2-0863 | C48 H51 N7 O9 S3 | 965.29104 | -20.7 | -17.6 |
| 2-1-m1E04-2-0864 | C47 H46 N6 O9 S4 | 966.22091 | 20.0 | -2.2 |
| 2-1-m1E04-2-0865 | C45 H45 F3 N6 O9 S3 | 966.23622 | 2.1 | -5.1 |
| 2-1-m1E04-2-0866 | C45 H45 F3 N6 O11 S2 | 966.25398 | -2.5 | -3.8 |
| 2-1-m1E04-2-0867 | C52 H50 N6 O7 S3 | 966.29031 | -1.7 | 14.4 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0868 | C45 H44 F3 N5 O10 S3 | 967.22024 | 15.1 | 0.0 |
| 2-1-m1E04-2-0869 | C47 H46 F N7 O9 S3 | 967.25032 | -17.1 | 5.7 |
| 2-1-m1E04-2-0870 | C50 H48 F3 N5 O8 S2 | 967.28964 | -0.6 | -4.5 |
| 2-1-m1E04-2-0871 | C46 H45 F5 N6 O8 S2 | 968.26604 | -19.3 | -0.2 |
| 2-1-m1E04-2-0872 | C50 H54 F2 N6 O8 S2 | 968.34126 | -11.4 | 0.3 |
| 2-1-m1E04-2-0873 | C49 H46 F3 N5 O7 S3 | 969.25115 | -6.1 | 15.4 |
| 2-1-m1E04-2-0874 | C49 H46 F3 N5 O7 S3 | 969.25115 | 28.5 | -7.1 |
| 2-1-m1E04-2-0875 | C51 H54 F3 N5 O7 S2 | 969.34168 | -8.6 | 12.5 |
| 2-1-m1E04-2-0876 | C43 H44 F2 N6 O10 S4 | 970.19698 | 1.6 | -8.9 |
| 2-1-m1E04-2-0877 | C47 H47 F2 N7 O8 S3 | 971.26163 | 3.9 | 2.4 |
| 2-1-m1E04-2-0878 | C48 H47 F3 N6 O9 S2 | 972.27980 | 2.3 | -9.8 |
| 2-1-mlE04-2-0879 | C50 H55 F3 N6 O7 S2 | 972.35257 | 0.0 | 0.0 |
| 2-1-m1E04-2-0880 | C48 H47 N9 O8 S3 | 973.27097 | 41.3 | -2.0 |
| 2-1-m1E04-2-0881 | C49 H50 F3 N5 O9 S2 | 973.30020 | 0.0 | 0.0 |
| 2-1-m1E04-2-0882 | C50 H54 F3 N5 O8 S2 | 973.33659 | 19.8 | -16.9 |
| 2-1-mlE04-2-0883 | C49 H53 F3 N6 O8 S2 | 974.33184 | 2.5 | -7.8 |
| 2-1-m1E04-2-0884 | C44 H45 N7 O11 S4 | 975.20599 | -31.0 | 5.4 |
| 2-1-m1E04-2-0885 | C48 H45 F4 N5 O9 S2 | 975.25948 | -7.4 | -12.2 |
| 2-1-mlE04-2-0886 | C49 H49 N7 O9 S3 | 975.27539 | 5.8 | 0.0 |
| 2-1-m1E04-2-0887 | C47 H48 F3 N7 O9 S2 | 975.29070 | 0.0 | 0.0 |
| 2-1-mlE04-2-0888 | C47 H47 F3 N6 O10 S2 | 976.27472 | -1.9 | -6.7 |
| 2-1-m1E04-2-0889 | C48 H51 F3 N6 O9 S2 | 976.31110 | 6.3 | 1.1 |
| 2-1-m1E04-2-0890 | C48 H51 F3 N6 O9 S2 | 976.31110 | 6.7 | 19.1 |
| 2-1-m1E04-2-0891 | C48 H47 N7 O8 S4 | 977.23689 | 5.1 | 1.0 |
| 2-1-m1E04-2-0892 | C48 H47 N7 O8 S4 | 977.23689 | 15.4 | 17.8 |
| 2-1-mlE04-2-0893 | C47 H46 F3 N5 O9 S3 | 977.24097 | 10.4 | -3.7 |
| 2-1-m1E04-2-0894 | C46 H46 F3 N7 O10 S2 | 977.26997 | -1.8 | -6.7 |
| 2-1-mlE04-2-0895 | C50 H51 N5 O10 S3 | 977.27980 | -1.0 | -7.1 |
| 2-1-m1E04-2-0896 | C47 H46 F4 N6 O9 S2 | 978.27038 | -7.2 | -6.6 |
| 2-1-m1E04-2-0897 | C49 H49 N5 O9 S4 | 979.24131 | 0.2 | -0.6 |
| 2-1-m1E04-2-0898 | C48 H48 N6 O9 S4 | 980.23656 | 3.3 | -1.0 |
| 2-1-m1E04-2-0899 | C47 H48 F4 N6 O9 S2 | 980.28603 | 0.0 | 0.0 |
| 2-1-mlE04-2-0900 | C47 H47 N7 O9 S4 | 981.23181 | -7.7 | -3.4 |
| 2-1-m1E04-2-0901 | C49 H48 F N5 O10 S3 | 981.25473 | 0.0 | 0.0 |
| 2-1-m1E04-2-0902 | C48 H45 F6 N5 O7 S2 | 981.26646 | -2.6 | 6.4 |
| 2-1-m1E04-2-0903 | C49 H46 F3 N7 O8 S2 | 981.28014 | 12.3 | 7.6 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0904 | C49 H55 N7 O9 S3 | 981.32234 | -4.3 | -18.6 |
| 2-1-mlE04-2-0905 | C52 H54 F3 N5 O7 S2 | 981.34168 | 0.0 | -47.9 |
| 2-1-m1E04-2-0906 | C45 H45 F3 N6 O10 S3 | 982.23114 | -1.1 | -5.9 |
| 2-1-m1E04-2-0907 | C48 H50 N6 O11 S3 | 982.26997 | -0.8 | -6.1 |
| 2-1-m1E04-2-0908 | C45 H44 F3 N5 O9 S4 | 983.19740 | 6.8 | -7.0 |
| 2-1-m1E04-2-0909 | C45 H44 F3 N5 O11 S3 | 983.21515 | 0.0 | 0.0 |
| 2-1-m1E04-2-0910 | C48 H46 F N5 O9 S4 | 983.21624 | 0.0 | 3.6 |
| 2-1-m1E04-2-0911 | C47 H49 N7 O11 S3 | 983.26522 | 0.0 | 19.0 |
| 2-1-m1E04-2-0912 | C50 H48 F3 N5 O9 S2 | 983.28455 | 1.7 | 1.7 |
| 2-1-m1E04-2-0913 | C47 H48 N6 O10 S4 | 984.23147 | -1.9 | -2.5 |
| 2-1-m1E04-2-0914 | C49 H47 F3 N6 O7 S3 | 984.26204 | 1.9 | 6.5 |
| 2-1-m1E04-2-0915 | C48 H47 F3 N8 O8 S2 | 984.29104 | 0.0 | -7.3 |
| 2-1-m1E04-2-0916 | C46 H47 N7 O10 S4 | 985.22672 | 1.4 | -43.3 |
| 2-1-m1E04-2-0917 | C46 H47 N7 O10 S4 | 985.22672 | 0.0 | 0.0 |
| 2-1-m1E04-2-0918 | C49 H46 F3 N5 O8 S3 | 985.24606 | 6.5 | 4.3 |
| 2-1-m1E04-2-0919 | C49 H46 F3 N5 O8 S3 | 985.24606 | 9.4 | 21.4 |
| 2-1-m1E04-2-0920 | C44 H45 F3 N6 O11 S3 | 986.22605 | 7.6 | -2.2 |
| 2-1-m1E04-2-0921 | C49 H49 F3 N6 O9 S2 | 986.29545 | 19.2 | 1.4 |
| 2-1-m1E04-2-0922 | C48 H47 F3 N6 O8 S3 | 988.25696 | 5.3 | 2.6 |
| 2-1-m1E04-2-0923 | C48 H47 F3 N6 O8 S3 | 988.25696 | 0.0 | -33.8 |
| 2-1-m1E04-2-0924 | C50 H55 F3 N6 O8 S2 | 988.34749 | 26.2 | -18.2 |
| 2-1-m1E04-2-0925 | C47 H46 F3 N7 O8 S3 | 989.25221 | 5.9 | 0.0 |
| 2-1-m1E04-2-0926 | C51 H55 N7 O8 S3 | 989.32742 | 9.5 | 14.0 |
| 2-1-m1E04-2-0927 | C50 H54 F3 N5 O9 S2 | 989.33150 | 0.0 | 0.0 |
| 2-1-m1E04-2-0928 | C44 H45 N7 O10 S5 | 991.18314 | 0.6 | -8.3 |
| 2-1-m1E04-2-0929 | C47 H48 F3 N7 O10 S2 | 991.28562 | -10.2 | -6.2 |
| 2-1-mlE04-2-0930 | C51 H53 N5 O10 S3 | 991.29546 | 0.0 | 0.0 |
| 2-1-m1E04-2-0931 | C48 H48 N8 O8 S4 | 992.24779 | -1.3 | 24.9 |
| 2-1-m1E04-2-0932 | C48 H51 F3 N6 O10 S2 | 992.30602 | 0.0 | 0.0 |
| 2-1-m1E04-2-0933 | C49 H55 F3 N6 O9 S2 | 992.34240 | -4.7 | -9.2 |
| 2-1-m1E04-2-0934 | C47 H46 F3 N5 O10 S3 | 993.23589 | 27.8 | -7.7 |
| 2-1-mlE04-2-0935 | C50 H51 N5 O9 S4 | 993.25696 | 0.0 | 23.8 |
| 2-1-m1E04-2-0936 | C50 H51 N5 Oil S3 | 993.27472 | 0.0 | 0.0 |
| 2-1-m1E04-2-0937 | C49 H50 N6 O9 S4 | 994.25221 | 0.0 | 0.0 |
| 2-1-m1E04-2-0938 | C47 H46 F4 N6 O10 S2 | 994.26530 | -1.3 | -7.1 |
| 2-1-m1E04-2-0939 | C49 H49 N5 O10 S4 | 995.23623 | 23.2 | 24.6 |

(continued)

| [Table 11-2-8] | | | | |
|---|---|---|---|---|
| | | | AS-MS rati o (%) | |
| ID | Molecular Formula | Exact Mass | N1 | N2 |
| 2-1-mlE04-2-0940 | C46 H47 F3 N6 O10 S3 | 996.24679 | 0.1 | -3.5 |
| 2-1-m1E04-2-0941 | C49 H52 N6 Oil S3 | 996.28562 | 2.3 | 2.0 |
| 2-1-mlE04-2-0942 | C48 H45 F6 N5 O8 S2 | 997.26137 | -13.3 | -5.6 |
| 2-1-m1E04-2-0943 | C52 H54 F3 N5 O8 S2 | 997.33659 | -17.5 | 29.6 |
| 2-1-m1E04-2-0944 | C48 H47 F N6 O9 S4 | 998.22714 | 14.4 | 2.6 |
| 2-1-mlE04-2-0945 | C48 H50 N6 O10 S4 | 998.24712 | 3.0 | -2.4 |
| 2-1-m1E04-2-0946 | C45 H44 F3 N5 O10 S4 | 999.19231 | -1.6 | -9.3 |
| 2-1-mlE04-2-0947 | C47 H49 N7 O10 S4 | 999.24237 | -7.8 | -7.3 |
| 2-1-m1E04-2-0948 | C49 H47 F2 N5 O10 S3 | 999.24531 | 18.4 | 15.5 |
| 2-1-mlE04-2-0949 | C46 H48 N8 O10 S4 | 1000.23762 | 23.2 | 0.0 |
| 2-1-mlE04-2-0950 | C49 H47 F3 N6 O8 S3 | 1000.25696 | -2.6 | -1.3 |
| 2-1-m1E04-2-0951 | C48 H49 F N6 O11 S3 | 1000.26055 | 0.7 | -4.1 |
| 2-1-mlE04-2-0952 | C47 H46 F6 N6 O8 S2 | 1000.27227 | 3.1 | -0.4 |
| 2-1-m1E04-2-0953 | C48 H47 F3 N8 O9 S2 | 1000.28595 | -2.6 | -1.7 |
| 2-1-mlE04-2-0954 | C51 H55 F3 N6 O8 S2 | 1000.34749 | 13.7 | -8.9 |
| 2-1-m1E04-2-0955 | C48 H45 F2 N5 O9 S4 | 1001.20682 | 11.7 | -15.8 |
| 2-1-m1E04-2-0956 | C44 H45 F3 N6 O10 S4 | 1002.20321 | 10.4 | -3.0 |
| 2-1-m1E04-2-0957 | C44 H45 F3 N6 O12 S3 | 1002.22097 | 2.4 | -3.1 |
| 2-1-m1E04-2-0958 | C47 H47 F N6 O10 S4 | 1002.22205 | -12.7 | -9.8 |
| 2-1-m1E04-2-0959 | C49 H49 F3 N6 O10 S2 | 1002.29037 | 5.4 | -1.6 |
| 2-1-m1E04-2-0960 | C48 H48 F3 N7 O8 S3 | 1003.26786 | 5.5 | 4.9 |
| 2-1-m1E04-2-0961 | C48 H47 F3 N6 O9 S3 | 1004.25187 | 17.5 | 8.0 |
| 2-1-m1E04-2-0962 | C48 H47 F3 N6 O9 S3 | 1004.25187 | 0.0 | 0.0 |
| 2-1-m1E04-2-0963 | C50 H52 N6 O9 S4 | 1008.26786 | -1.6 | 1.1 |
| 2-1-m1E04-2-0964 | C49 H55 F3 N6 O10 S2 | 1008.33732 | -4.0 | -6.9 |
| 2-1-m1E04-2-0965 | C49 H50 N6 O10 S4 | 1010.24712 | 0.0 | 19.4 |
| 2-1-m1E04-2-0966 | C50 H54 N6 Oil S3 | 1010.30127 | 3.1 | 1.5 |
| 2-1-m1E04-2-0967 | C46 H47 F3 N6 Oil S3 | 1012.24170 | 3.8 | -1.2 |
| 2-1-m1E04-2-0968 | C49 H52 N6 O10 S4 | 1012.26277 | 1.1 | 0.3 |
| 2-1-m1E04-2-0969 | C49 H52 N6 O12 S3 | 1012.28053 | -8.9 | -8.5 |
| 2-1-m1E04-2-0970 | C48 H51 N7 O10 S4 | 1013.25802 | 0.0 | -3.0 |
| 2-1-m1E04-2-0971 | C53 H51 N5 O10 S3 | 1013.27980 | 0.0 | 12.6 |
| 2-1-m1E04-2-0972 | C48 H50 N6 Oil S4 | 1014.24204 | 4.3 | 1.8 |
| 2-1-mlE04-2-0973 | C52 H49 N5 O9 S4 | 1015.24131 | 16.0 | 0.0 |
| 2-1-m1E04-2-0974 | C48 H46 F2 N6 O9 S4 | 1016.21772 | 7.9 | -1.3 |
| 2-1-mlE04-2-0975 | C47 H46 F6 N6 O9 S2 | 1016.26719 | -5.9 | -3.1 |

(continued)

| [Table 11-2-8] | | | | |
|---|---|---|---|---|
| | | | AS-MS rati o (%) | |
| ID | Molecular Formula | Exact Mass | N1 | N2 |
| 2-1-m1E04-2-0976 | C51 H55 F3 N6 O9 S2 | 1016.34240 | 35.3 | 18.1 |
| 2-1-mlE04-2-0977 | C47 H48 F N7 O10 S4 | 1017.23295 | -3.3 | -2.7 |
| 2-1-mlE04-2-0978 | C44 H45 F3 N6 O11 S4 | 1018.19812 | 0.4 | -3.9 |
| 2-1-m1E04-2-0979 | C48 H48 F2 N6 O11 S3 | 1018.25112 | 17.7 | -1.7 |
| 2-1-mlE04-2-0980 | C48 H48 F3 N7 O9 S3 | 1019.26277 | 3.3 | 5.7 |
| 2-1-m1E04-2-0981 | C47 H46 F2 N6 O10 S4 | 1020.21263 | -6.9 | -4.6 |
| 2-1-mlE04-2-0982 | C49 H53 N7 O10 S4 | 1027.27367 | 4.2 | -0.2 |
| 2-1-m1E04-2-0983 | C48 H51 N7 O11 S4 | 1029.25294 | 9.3 | -7.5 |
| 2-1-m1E04-2-0984 | C52 H50 N6 O9 S4 | 1030.25221 | 0.0 | 0.0 |
| 2-1-m1E04-2-0985 | C50 H48 F3 N5 O10 S3 | 1031.25154 | 0.3 | -1.2 |
| 2-1-m1E04-2-0986 | C49 H46 F3 N5 O9 S4 | 1033.21305 | -10.4 | -8.9 |
| 2-1-mlE04-2-0987 | C47 H47 F2 N7 O10 S4 | 1035.22353 | -4.4 | -3.4 |
| 2-1-m1E04-2-0988 | C49 H49 N7 O11 S4 | 1039.23729 | 5.0 | -0.5 |
| 2-1-mlE04-2-0989 | C48 H47 N7 O10 S5 | 1041.19879 | 2.9 | -7.6 |
| 2-1-m1E04-2-0990 | C50 H48 F3 N5 O11 S3 | 1047.24645 | 16.3 | -5.5 |
| 2-1-m1E04-2-0991 | C49 H47 F3 N6 O9 S4 | 1048.22394 | -4.8 | -2.4 |
| 2-1-mlE04-2-0992 | C49 H46 F3 N5 O10 S4 | 1049.20796 | -5.3 | 8.3 |
| 2-1-m1E04-2-0993 | C49 H49 F3 N6 O11 S3 | 1050.25735 | -0.6 | -6.0 |
| 2-1-mlE04-2-0994 | C48 H47 F3 N6 O10 S4 | 1052.21886 | -6.0 | -3.0 |
| 2-1-m1E04-2-0995 | C48 H48 N8 O10 S5 | 1056.20969 | -0.7 | -8.1 |
| 2-1-mlE04-2-0996 | C49 H47 F3 N6 O10 S4 | 1064.21886 | -6.4 | 13.6 |
| 2-1-mlE04-2-0997 | C49 H49 F3 N6 O12 S3 | 1066.25227 | -5.9 | -3.5 |
| 2-1-m1E04-2-0998 | C48 H48 F3 N7 O10 S4 | 1067.22976 | -0.3 | -1.7 |
| 2-1-mlE04-2-0999 | C48 H47 F3 N6 O11 S4 | 1068.21377 | 1.7 | -0.6 |
| 2-1-m1E04-2-1000 | C48 H48 F3 N7 O11 S4 | 1083.22467 | -2.9 | -0.3 |

[Table 598]

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0001 | C32 H32 N4 O6 | 568.23218 | 5.3 | 28.1 | -8.0 | 6.0 |
| 2-2-d1E01-1-0002 | C33 H40 N4 O5 | 572.29987 | 3.2 | 11.8 | -10.6 | 3.9 |
| 2-2-d1E01-1-0003 | C32 H38 N4 O6 | 574.27913 | 2.8 | 9.8 | -8.0 | 3.4 |
| 2-2-d1E01-1-0004 | C34 H34 N4 O5 | 578.25292 | 12.3 | 33.0 | -4.6 | 4.7 |
| 2-2-d1E01-1-0005 | C34 H34 N4 O5 | 578.25292 | 12.3 | 35.0 | -6.6 | 5.2 |
| 2-2-d1E01-1-0006 | C33 H33 N5 O5 | 579.24817 | 4.3 | 21.0 | -8.4 | 3.9 |
| 2-2-d1E01-1-0007 | C33 H34 N4 O6 | 582.24783 | 5.9 | 31.6 | -2.1 | 0.8 |
| 2-2-d1E01-1-0008 | C32 H32 N4 O5 S | 584.20934 | 13.3 | 37.7 | -3.4 | 8.5 |
| 2-2-d1E01-1-0009 | C31 H31 N5 O7 | 585.22235 | 5.4 | 20.2 | -9.6 | 3.9 |
| 2-2-d1E01-1-0010 | C32 H31 F N4 O6 | 586.22276 | 7.6 | 30.0 | -6.5 | 7.1 |
| 2-2-d1E01-1-0011 | C34 H42 N4 O5 | 586.31552 | 0.8 | 9.2 | -9.3 | -0.4 |
| 2-2-d1E01-1-0012 | C33 H40 N4 O6 | 588.29479 | 0.8 | 9.3 | -10.5 | 3.5 |
| 2-2-d1E01-1-0013 | C33 H39 F N4 O5 | 590.29045 | 1.7 | 13.2 | -10.0 | 3.8 |
| 2-2-d1E01-1-0014 | C35 H36 N4 O5 | 592.26857 | 14.4 | 38.2 | -5.0 | 1.5 |
| 2-2-d1E01-1-0015 | C35 H36 N4 O5 | 592.26857 | 16.6 | 49.9 | -9.1 | 12.1 |
| 2-2-d1E01-1-0016 | C32 H37 F N4 O6 | 592.26971 | 2.3 | 11.5 | -11.1 | 3.3 |
| 2-2-d1E01-1-0017 | C34 H35 N5 O5 | 593.26382 | 6.5 | 27.3 | -4.6 | 5.0 |
| 2-2-d1E01-1-0018 | C34 H34 N4 O6 | 594.24783 | 5.1 | 35.4 | -4.9 | 7.4 |
| 2-2-d1E01-1-0019 | C32 H32 N6 O6 | 596.23833 | 5.5 | 13.2 | -8.6 | 4.9 |
| 2-2-d1E01-1-0020 | C34 H33 F N4 O5 | 596.24350 | 6.4 | 38.6 | -5.3 | 2.5 |
| 2-2-d1E01-1-0021 | C34 H33 F N4 O5 | 596.24350 | 6.4 | 34.2 | -2.4 | 7.3 |
| 2-2-d1E01-1-0022 | C33 H32 F N5 O5 | 597.23875 | 5.8 | 21.9 | -5.2 | 0.6 |
| 2-2-d1E01-1-0023 | C33 H34 N4 O5 S | 598.22499 | 14.6 | 38.8 | 4.9 | 4.9 |
| 2-2-d1E01-1-0024 | C32 H34 N6 O6 | 598.25398 | 6.4 | 17.5 | -9.6 | 4.2 |
| 2-2-d1E01-1-0025 | C35 H42 N4 O5 | 598.31552 | 7.9 | 27.1 | -14.7 | 3.2 |
| 2-2-d1E01-1-0026 | C32 H33 N5 O7 | 599.23800 | 3.8 | 21.8 | -8.8 | 4.0 |
| 2-2-d1E01-1-0027 | C34 H40 N4 O6 | 600.29479 | 1.0 | 9.9 | -10.6 | 3.1 |
| 2-2-d1E01-1-0028 | C31 H31 N5 O6 S | 601.19950 | 7.2 | 24.3 | -7.1 | 4.4 |
| 2-2-d1E01-1-0029 | C32 H31 F N4 O5 S | 602.19992 | 9.3 | 42.1 | -3.1 | 4.0 |
| 2-2-d1E01-1-0030 | C36 H34 N4 O5 | 602.25292 | 10.7 | 48.3 | 6.0 | 4.6 |
| 2-2-d1E01-1-0031 | C36 H34 N4 O5 | 602.25292 | 7.0 | 26.3 | -7.9 | 7.9 |
| 2-2-d1E01-1-0032 | C31 H30 F N5 O7 | 603.21293 | 5.9 | 19.0 | -9.2 | 0.0 |
| 2-2-d1E01-1-0033 | C35 H33 N5 O5 | 603.24817 | 7.2 | 34.6 | -6.2 | 7.6 |
| 2-2-d1E01-1-0034 | C35 H33 N5 O5 | 603.24817 | 11.6 | 42.5 | -3.4 | 11.0 |
| 2-2-d1E01-1-0035 | C35 H33 N5 O5 | 603.24817 | 11.4 | 34.1 | -0.1 | 6.0 |
| 2-2-d1E01-1-0036 | C36 H36 N4 O5 | 604.26857 | 15.6 | 39.5 | -23.7 | 25.0 |
| 2-2-d1E01-1-0037 | C36 H36 N4 O5 | 604.26857 | 12.5 | 54.2 | -16.0 | -21.2 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0038 | C35 H35 N5 O5 | 605.26382 | 10.6 | 25.1 | -6.7 | 4.0 |
| 2-2-d1E01-1-0039 | C35 H37 N5 O5 | 607.27947 | 2.2 | 34.4 | -8.0 | 9.3 |
| 2-2-d1E01-1-0040 | C35 H36 N4 O6 | 608.26348 | 4.5 | 39.7 | 1.0 | 6.5 |
| 2-2-d1E01-1-0041 | C34 H36 N6 O5 | 608.27472 | 10.5 | 41.6 | 0.2 | 8.8 |
| 2-2-d1E01-1-0042 | C33 H35 N7 O5 | 609.26997 | 3.3 | 17.2 | -8.6 | 3.8 |
| 2-2-d1E01-1-0043 | C34 H34 N4 O5 S | 610.22499 | 0.0 | 17.6 | -19.2 | 16.9 |
| 2-2-d1E01-1-0044 | C33 H34 N6 O6 | 610.25398 | 5.8 | 16.4 | -9.1 | 1.3 |
| 2-2-d1E01-1-0045 | C35 H38 N4 O6 | 610.27913 | 9.1 | 35.4 | -8.9 | 6.3 |
| 2-2-d1E01-1-0046 | C33 H33 N5 O7 | 611.23800 | 4.0 | 22.8 | -7.3 | 4.9 |
| 2-2-d1E01-1-0047 | C34 H33 F N4 O6 | 612.23841 | 8.1 | 38.8 | -7.7 | 7.5 |
| 2-2-d1E01-1-0048 | C33 H36 N6 O6 | 612.26963 | 7.2 | 22.0 | -8.4 | 2.4 |
| 2-2-d1E01-1-0049 | C36 H44 N4 O5 | 612.33117 | 9.1 | 32.8 | -4.0 | -33.4 |
| 2-2-d1E01-1-0050 | C32 H31 F N6 O6 | 614.22891 | 6.0 | 23.4 | -5.5 | -4.6 |
| 2-2-d1E01-1-0051 | C32 H34 N6 O5 S | 614.23114 | 2.1 | 20.0 | -10.8 | 4.6 |
| 2-2-d1E01-1-0052 | C35 H42 N4 O6 | 614.31044 | 4.2 | 19.1 | -13.8 | 4.4 |
| 2-2-d1E01-1-0053 | C32 H33 N5 O6 S | 615.21515 | 9.1 | 24.1 | 1.3 | 1.0 |
| 2-2-d1E01-1-0054 | C32 H33 F N6 O6 | 616.24456 | 5.1 | 18.7 | -9.7 | 5.3 |
| 2-2-d1E01-1-0055 | C37 H36 N4 O5 | 616.26857 | 9.7 | 48.2 | 2.6 | 7.7 |
| 2-2-d1E01-1-0056 | C37 H36 N4 O5 | 616.26857 | 14.5 | 33.8 | -3.4 | 10.6 |
| 2-2-d1E01-1-0057 | C35 H41 F N4 O5 | 616.30610 | 8.8 | 26.4 | -16.4 | 1.4 |
| 2-2-d1E01-1-0058 | C36 H35 N5 O5 | 617.26382 | 10.4 | 34.0 | -1.2 | 7.5 |
| 2-2-d1E01-1-0059 | C36 H35 N5 O5 | 617.26382 | 7.4 | 37.5 | -3.1 | 0.2 |
| 2-2-d1E01-1-0060 | C36 H35 N5 O5 | 617.26382 | 9.3 | 38.7 | 2.1 | 20.0 |
| 2-2-d1E01-1-0061 | C37 H38 N4 O5 | 618.28422 | 3.4 | 22.4 | 34.2 | 20.8 |
| 2-2-d1E01-1-0062 | C37 H38 N4 O5 | 618.28422 | 3.4 | 22.4 | 2.1 | -2.6 |
| 2-2-d1E01-1-0063 | C34 H39 F N4 O6 | 618.28536 | 1.6 | 12.7 | -10.1 | 3.2 |
| 2-2-d1E01-1-0064 | C31 H30 F N5 O6 S | 619.19008 | 7.9 | 30.1 | -11.2 | 2.3 |
| 2-2-d1E01-1-0065 | C36 H37 N5 O5 | 619.27947 | 7.4 | 32.6 | -7.9 | 9.5 |
| 2-2-d1E01-1-0066 | C36 H33 F N4 O5 | 620.24350 | 14.5 | 50.2 | 9.4 | 10.6 |
| 2-2-d1E01-1-0067 | C36 H33 F N4 O5 | 620.24350 | 0.0 | 0.0 | 0.0 | 0.0 |
| 2-2-d1E01-1-0068 | C37 H40 N4 O5 | 620.29987 | 15.6 | 33.1 | -1.5 | 6.9 |
| 2-2-d1E01-1-0069 | C35 H32 F N5 O5 | 621.23875 | 7.1 | 35.0 | -4.9 | 9.7 |
| 2-2-d1E01-1-0070 | C35 H32 F N5 O5 | 621.23875 | 10.1 | 37.8 | -1.2 | 5.0 |
| 2-2-d1E01-1-0071 | C35 H32 F N5 O5 | 621.23875 | 14.6 | 36.2 | -8.4 | 10.1 |
| 2-2-d1E01-1-0072 | C36 H39 N5 O5 | 621.29512 | 14.4 | 59.5 | -3.4 | -7.1 |
| 2-2-d1E01-1-0073 | C34 H34 N6 O6 | 622.25398 | -1.4 | 8.4 | -59.1 | 3.2 |

(continued)

[Table 11-3-1]

| | | | AS-MS(%) | | | |
|---|---|---|---|---|---|---|
| ID | Molecular Formula | Exact Mass | N1 | N2 | N3 | N4 |
| 2-2-d1E01-1-0074 | C36 H35 F N4 O5 | 622.25915 | 21.1 | 53.3 | 2.2 | 20.5 |
| 2-2-d1E01-1-0075 | C36 H35 F N4 O5 | 622.25915 | 5.0 | 49.3 | -9.1 | -0.5 |
| 2-2-d1E01-1-0076 | C35 H38 N6 O5 | 622.29037 | 9.5 | 30.0 | -2.8 | 0.9 |
| 2-2-d1E01-1-0077 | C35 H34 F N5 O5 | 623.25440 | 7.3 | 31.6 | -3.5 | 8.4 |
| 2-2-d1E01-1-0078 | C34 H37 N7 O5 | 623.28562 | 5.5 | 23.5 | -8.4 | 2.1 |
| 2-2-d1E01-1-0079 | C35 H36 N4 O5 S | 624.24064 | 9.5 | 33.8 | 0.0 | 9.9 |
| 2-2-d1E01-1-0080 | C34 H36 N6 O6 | 624.26963 | 4.8 | 22.1 | -8.0 | 3.6 |
| 2-2-d1E01-1-0081 | C36 H40 N4 O6 | 624.29479 | 5.2 | 37.9 | 2.3 | 6.8 |
| 2-2-d1E01-1-0082 | C33 H31 N5 O6 S | 625.19950 | 7.1 | 32.5 | -5.2 | 2.9 |
| 2-2-d1E01-1-0083 | C34 H35 N5 O7 | 625.25365 | 4.0 | 34.6 | -0.3 | 0.9 |
| 2-2-d1E01-1-0084 | C35 H36 F N5 O5 | 625.27005 | 8.4 | 26.0 | -13.7 | 4.3 |
| 2-2-d1E01-1-0085 | C35 H38 N4 O5 S | 626.25629 | 10.6 | 40.6 | 11.7 | 1.8 |
| 2-2-d1E01-1-0086 | C34 H35 F N6 O5 | 626.26530 | 5.4 | 30.5 | 1.2 | 5.0 |
| 2-2-d1E01-1-0087 | C33 H33 N5 O6 S | 627.21515 | 0.0 | 0.0 | -9.6 | 6.5 |
| 2-2-d1E01-1-0088 | C33 H34 F N7 O5 | 627.26055 | 4.6 | 22.8 | -9.5 | 4.4 |
| 2-2-d1E01-1-0089 | C34 H33 F N4 O5 S | 628.21557 | 20.1 | 43.0 | -14.3 | 11.0 |
| 2-2-d1E01-1-0090 | C33 H36 N6 O5 S | 628.24679 | 10.3 | 30.7 | 3.1 | 2.2 |
| 2-2-d1E01-1-0091 | C38 H36 N4 O5 | 628.26857 | 22.5 | 64.8 | -2.4 | 17.0 |
| 2-2-d1E01-1-0092 | C38 H36 N4 O5 | 628.26857 | 15.0 | 55.6 | 1.1 | 13.1 |
| 2-2-d1E01-1-0093 | C35 H37 F N4 O6 | 628.26971 | 11.4 | 46.6 | 0.2 | 5.9 |
| 2-2-d1E01-1-0094 | C33 H32 F N5 O7 | 629.22858 | 3.7 | 24.3 | -6.8 | 4.1 |
| 2-2-d1E01-1-0095 | C37 H35 N5 O5 | 629.26382 | 6.7 | 18.2 | 14.7 | -17.7 |
| 2-2-d1E01-1-0096 | C37 H35 N5 O5 | 629.26382 | 5.6 | 50.8 | -0.7 | 6.8 |
| 2-2-d1E01-1-0097 | C37 H35 N5 O5 | 629.26382 | 10.2 | 37.9 | -13.3 | 8.7 |
| 2-2-d1E01-1-0098 | C34 H39 N5 O5 S | 629.26719 | 4.3 | 18.8 | -9.0 | 1.4 |
| 2-2-d1E01-1-0099 | C33 H37 N5 O6 S | 631.24645 | 2.9 | 13.8 | -10.3 | 1.7 |
| 2-2-d1E01-1-0100 | C32 H32 N4 O8 S | 632.19408 | 1.7 | 8.6 | -5.9 | 8.6 |
| 2-2-d1E01-1-0101 | C32 H33 F N6 O5 S | 632.22172 | 9.8 | 30.5 | -9.9 | 8.0 |
| 2-2-d1E01-1-0102 | C37 H39 N5 O5 | 633.29512 | 8.0 | 18.7 | 3.3 | 6.4 |
| 2-2-d1E01-1-0103 | C36 H34 N4 O7 | 634.24275 | 14.5 | 37.8 | -1.1 | 9.7 |
| 2-2-d1E01-1-0104 | C36 H38 N6 O5 | 634.29037 | 25.2 | 0.0 | 18.6 | 11.9 |
| 2-2-d1E01-1-0105 | C38 H42 N4 O5 | 634.31552 | 3.1 | 37.2 | 1.6 | 5.0 |
| 2-2-d1E01-1-0106 | C35 H33 N5 O5 S | 635.22024 | 9.3 | 47.9 | -2.9 | 9.0 |
| 2-2-d1E01-1-0107 | C35 H33 N5 O5 S | 635.22024 | 19.2 | 52.1 | -0.3 | 8.0 |
| 2-2-d1E01-1-0108 | C35 H37 N7 O5 | 635.28562 | 5.8 | 25.2 | -7.3 | 4.1 |
| 2-2-d1E01-1-0109 | C34 H32 N6 O5 S | 636.21549 | 6.0 | 31.3 | -5.0 | 6.1 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0110 | C33 H31 F3 N4 O6 | 636.21957 | 14.9 | 36.7 | 1.6 | 12.8 |
| 2-2-d1E01-1-0111 | C33 H40 N4 O7 S | 636.26177 | 0.0 | 7.0 | -8.6 | 4.9 |
| 2-2-d1E01-1-0112 | C35 H36 N6 O6 | 636.26963 | 8.4 | 31.0 | -13.1 | 11.9 |
| 2-2-d1E01-1-0113 | C37 H40 N4 O6 | 636.29479 | 6.5 | 41.7 | -2.6 | -1.5 |
| 2-2-d1E01-1-0114 | C32 H38 N4 O8 S | 638.24103 | 1.6 | 6.2 | -8.3 | 4.4 |
| 2-2-d1E01-1-0115 | C35 H38 N6 O6 | 638.28528 | 6.2 | 29.4 | -2.8 | 2.9 |
| 2-2-d1E01-1-0116 | C37 H39 F N4 O5 | 638.29045 | 12.5 | 45.7 | -3.1 | 10.6 |
| 2-2-d1E01-1-0117 | C34 H33 F N6 O6 | 640.24456 | 0.0 | 0.0 | 0.0 | 0.0 |
| 2-2-d1E01-1-0118 | C34 H36 N6 O5 S | 640.24679 | 10.0 | 31.3 | -8.1 | -0.4 |
| 2-2-d1E01-1-0119 | C36 H40 N4 O5 S | 640.27194 | 11.1 | 43.8 | 2.7 | 3.2 |
| 2-2-d1E01-1-0120 | C34 H39 F3 N4 O5 | 640.28725 | 9.6 | 27.1 | -3.9 | 6.3 |
| 2-2-d1E01-1-0121 | C33 H31 N5 O5 S2 | 641.17666 | 13.7 | 38.7 | 0.0 | 5.9 |
| 2-2-d1E01-1-0122 | C34 H35 N5 O6 S | 641.23080 | 22.9 | 56.5 | 8.6 | 15.0 |
| 2-2-d1E01-1-0123 | C32 H30 N6 O7 S | 642.18967 | 5.0 | 21.0 | -8.7 | 2.3 |
| 2-2-d1E01-1-0124 | C34 H34 N4 O7 S | 642.21482 | 4.3 | 4.5 | -8.9 | 6.2 |
| 2-2-d1E01-1-0125 | C34 H34 N4 O7 S | 642.21482 | 4.2 | 5.3 | -5.3 | 7.5 |
| 2-2-d1E01-1-0126 | C34 H35 F N6 O6 | 642.26021 | 4.8 | 21.3 | -5.1 | 2.3 |
| 2-2-d1E01-1-0127 | C33 H37 F3 N4 O6 | 642.26652 | 5.0 | 18.0 | -8.0 | 1.9 |
| 2-2-d1E01-1-0128 | C39 H38 N4 O5 | 642.28422 | 9.1 | 41.3 | 2.1 | 8.7 |
| 2-2-d1E01-1-0129 | C39 H38 N4 O5 | 642.28422 | 10.0 | 46.7 | 0.2 | 1.2 |
| 2-2-d1E01-1-0130 | C33 H33 N5 O7 S | 643.21007 | 2.3 | 3.1 | -7.1 | 5.7 |
| 2-2-d1E01-1-0131 | C38 H37 N5 O5 | 643.27947 | 12.7 | 40.6 | 49.2 | 9.7 |
| 2-2-d1E01-1-0132 | C38 H37 N5 O5 | 643.27947 | 11.8 | 40.1 | -17.2 | 18.2 |
| 2-2-d1E01-1-0133 | C38 H37 N5 O5 | 643.27947 | 9.9 | 35.0 | -5.8 | 13.5 |
| 2-2-d1E01-1-0134 | C35 H37 F N4 O5 S | 644.24687 | 10.1 | 44.2 | 8.7 | 13.3 |
| 2-2-d1E01-1-0135 | C33 H32 F N5 O6 S | 645.20573 | 14.1 | 45.8 | -5.2 | 9.7 |
| 2-2-d1E01-1-0136 | C33 H34 N4 O8 S | 646.20973 | 2.2 | 4.6 | -9.2 | 6.8 |
| 2-2-d1E01-1-0137 | C35 H33 F3 N4 O5 | 646.24030 | 15.0 | 43.2 | 2.4 | 2.3 |
| 2-2-d1E01-1-0138 | C35 H33 F3 N4 O5 | 646.24030 | 16.3 | 50.9 | 3.3 | 10.8 |
| 2-2-d1E01-1-0139 | C38 H35 F N4 O5 | 646.25915 | 18.3 | 54.1 | 7.8 | 7.0 |
| 2-2-d1E01-1-0140 | C38 H35 F N4 O5 | 646.25915 | 11.6 | 36.3 | -8.7 | 19.6 |
| 2-2-d1E01-1-0141 | C39 H42 N4 O5 | 646.31552 | 7.7 | 54.7 | 4.5 | 11.0 |
| 2-2-d1E01-1-0142 | C34 H32 F3 N5 O5 | 647.23555 | 7.9 | 39.6 | -8.2 | 3.5 |
| 2-2-d1E01-1-0143 | C37 H34 F N5 O5 | 647.25440 | 12.3 | 41.9 | -0.5 | 10.1 |
| 2-2-d1E01-1-0144 | C37 H34 F N5 O5 | 647.25440 | 8.8 | 35.1 | -9.7 | 7.0 |
| 2-2-d1E01-1-0145 | C37 H34 F N5 O5 | 647.25440 | 37.1 | 65.5 | -5.5 | -18.4 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0146 | C38 H41 N5 O5 | 647.31077 | 1.0 | 9.0 | -9.2 | 18.2 |
| 2-2-d1E01-1-0147 | C32 H32 N4 O7 S2 | 648.17124 | 3.9 | 8.4 | -3.0 | 4.2 |
| 2-2-d1E01-1-0148 | C37 H36 N4 O7 | 648.25840 | 14.4 | 47.2 | -2.1 | 8.6 |
| 2-2-d1E01-1-0149 | C37 H40 N6 O5 | 648.30602 | 30.8 | 25.1 | -13.4 | 0.0 |
| 2-2-d1E01-1-0150 | C31 H31 N5 O9 S | 649.18425 | 2.7 | 5.0 | -9.7 | 0.0 |
| 2-2-d1E01-1-0151 | C36 H39 N7 O5 | 649.30127 | 4.5 | 25.1 | -3.1 | 3.2 |
| 2-2-d1E01-1-0152 | C32 H31 F N4 O8 S | 650.18466 | 1.8 | 9.1 | -5.5 | 7.0 |
| 2-2-d1E01-1-0153 | C34 H42 N4 O7 S | 650.27742 | 1.2 | 3.0 | -10.1 | 5.5 |
| 2-2-d1E01-1-0154 | C38 H42 N4 O6 | 650.31044 | 2.9 | 41.1 | -1.6 | -1.5 |
| 2-2-d1E01-1-0155 | C35 H33 N5 O6 S | 651.21515 | 12.7 | 41.1 | -0.8 | 8.9 |
| 2-2-d1E01-1-0156 | C37 H38 F N5 O5 | 651.28570 | 11.7 | 0.0 | -39.4 | 8.0 |
| 2-2-d1E01-1-0157 | C33 H31 F3 N4 O5 S | 652.19673 | 10.3 | 49.1 | 5.5 | 7.9 |
| 2-2-d1E01-1-0158 | C36 H33 F N4 O7 | 652.23333 | 0.3 | 48.6 | 5.8 | 6.6 |
| 2-2-d1E01-1-0159 | C33 H40 N4 O8 S | 652.25668 | 1.0 | 4.7 | -8.5 | 6.2 |
| 2-2-d1E01-1-0160 | C37 H40 N4 O5 S | 652.27194 | 10.9 | 50.1 | 2.5 | 10.0 |
| 2-2-d1E01-1-0161 | C36 H37 F N6 O5 | 652.28095 | 6.1 | 36.6 | 3.1 | 3.5 |
| 2-2-d1E01-1-0162 | C33 H31 N7 O6 S | 653.20565 | 2.9 | 16.4 | -6.8 | 3.5 |
| 2-2-d1E01-1-0163 | C32 H30 F3 N5 O7 | 653.20973 | 7.7 | 33.2 | 3.8 | 9.3 |
| 2-2-d1E01-1-0164 | C35 H36 F N7 O5 | 653.27620 | 4.6 | 25.6 | -6.4 | 4.8 |
| 2-2-d1E01-1-0165 | C33 H39 F N4 O7 S | 654.25235 | 1.1 | 3.4 | -6.8 | 3.0 |
| 2-2-d1E01-1-0166 | C35 H38 N6 O5 S | 654.26244 | 0.0 | 2.7 | -10.5 | 3.1 |
| 2-2-d1E01-1-0167 | C37 H39 F N4 O6 | 654.28536 | 3.8 | 38.0 | 4.3 | 5.3 |
| 2-2-d1E01-1-0168 | C33 H33 N7 O6 S | 655.22130 | 4.3 | 24.4 | -9.1 | 2.7 |
| 2-2-d1E01-1-0169 | C36 H41 N5 O5 S | 655.28284 | 7.2 | 26.2 | 3.3 | 0.0 |
| 2-2-d1E01-1-0170 | C35 H36 N4 O7 S | 656.23047 | 2.6 | 3.1 | -4.7 | 3.2 |
| 2-2-d1E01-1-0171 | C35 H36 N4 O7 S | 656.23047 | 2.9 | 1.6 | -8.4 | 6.4 |
| 2-2-d1E01-1-0172 | C32 H37 F N4 O8 S | 656.23161 | 1.2 | 1.9 | -5.6 | 5.9 |
| 2-2-d1E01-1-0173 | C34 H35 N5 O7 S | 657.22572 | 1.9 | 4.0 | -6.8 | 4.2 |
| 2-2-d1E01-1-0174 | C35 H39 N5 O6 S | 657.26210 | 6.7 | 22.6 | -11.6 | 5.5 |
| 2-2-d1E01-1-0175 | C32 H30 N6 O6 S2 | 658.16682 | 9.0 | 27.8 | -1.6 | 13.6 |
| 2-2-d1E01-1-0176 | C34 H34 N4 O8 S | 658.20973 | 1.7 | 5.9 | -8.3 | 5.4 |
| 2-2-d1E01-1-0177 | C34 H35 F N6 O5 S | 658.23737 | 4.9 | 25.0 | -10.7 | 0.8 |
| 2-2-d1E01-1-0178 | C37 H33 N5 O5 S | 659.22024 | 9.7 | 33.7 | 0.5 | 2.0 |
| 2-2-d1E01-1-0179 | C37 H33 N5 O5 S | 659.22024 | 9.7 | 41.2 | -7.7 | 3.4 |
| 2-2-d1E01-1-0180 | C32 H32 N6 O8 S | 660.20023 | 0.0 | 0.0 | 0.0 | 0.0 |
| 2-2-d1E01-1-0181 | C34 H33 F N4 O7 S | 660.20540 | 5.0 | 4.9 | -8.6 | 7.0 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0182 | C34 H33 F N4 O7 S | 660.20540 | 3.3 | 4.3 | -8.6 | 8.0 |
| 2-2-d1E01-1-0183 | C36 H32 N6 O5 S | 660.21549 | 13.4 | 31.5 | -8.2 | 9.7 |
| 2-2-d1E01-1-0184 | C36 H32 N6 O5 S | 660.21549 | 8.4 | 35.9 | -6.0 | 2.7 |
| 2-2-d1E01-1-0185 | C36 H32 N6 O5 S | 660.21549 | 16.0 | 28.6 | -6.4 | 9.5 |
| 2-2-d1E01-1-0186 | C38 H36 N4 O7 | 660.25840 | 6.4 | 55.3 | 15.2 | 16.7 |
| 2-2-d1E01-1-0187 | C40 H44 N4 O5 | 660.33117 | 16.5 | 45.0 | 0.1 | 3.8 |
| 2-2-d1E01-1-0188 | C33 H32 F N5 O7 S | 661.20065 | 2.3 | 4.0 | -5.5 | 5.0 |
| 2-2-d1E01-1-0189 | C37 H35 N5 O5 S | 661.23589 | 21.6 | 48.4 | -3.5 | 9.8 |
| 2-2-d1E01-1-0190 | C37 H35 N5 O5 S | 661.23589 | 2.5 | 5.2 | -9.8 | 8.8 |
| 2-2-d1E01-1-0191 | C33 H34 N4 O7 S2 | 662.18689 | 2.1 | 5.6 | -7.9 | 5.7 |
| 2-2-d1E01-1-0192 | C32 H34 N6 O8 S | 662.21588 | 2.8 | 6.4 | -6.1 | 3.1 |
| 2-2-d1E01-1-0193 | C36 H34 N6 O5 S | 662.23114 | 7.5 | 37.0 | -5.1 | 11.9 |
| 2-2-d1E01-1-0194 | C35 H33 F3 N4 O6 | 662.23522 | 6.6 | 50.7 | -5.4 | 5.8 |
| 2-2-d1E01-1-0195 | C35 H42 N4 O7 S | 662.27742 | 2.7 | 3.3 | -10.2 | 6.7 |
| 2-2-d1E01-1-0196 | C38 H42 N6 O5 | 662.32167 | 6.4 | 36.1 | -3.1 | 10.4 |
| 2-2-d1E01-1-0197 | C32 H33 N5 O9 S | 663.19990 | 1.3 | 13.6 | -7.8 | 7.3 |
| 2-2-d1E01-1-0198 | C33 H31 F3 N6 O6 | 664.22572 | 15.1 | 36.9 | -1.8 | -1.0 |
| 2-2-d1E01-1-0199 | C36 H36 N6 O5 S | 664.24679 | 17.9 | 35.9 | 1.4 | 9.9 |
| 2-2-d1E01-1-0200 | C34 H40 N4 O8 S | 664.25668 | 1.7 | 6.5 | -7.2 | 4.7 |
| 2-2-d1E01-1-0201 | C39 H41 F N4 O5 | 664.30610 | 18.4 | 40.7 | 1.8 | 8.4 |
| 2-2-d1E01-1-0202 | C31 H31 N5 O8 S2 | 665.16140 | -0.1 | 6.4 | -7.2 | 3.7 |
| 2-2-d1E01-1-0203 | C35 H35 N7 O5 S | 665.24204 | 13.4 | 27.1 | -7.3 | -2.2 |
| 2-2-d1E01-1-0204 | C32 H31 F N4 O7 S2 | 666.16182 | 4.8 | 7.2 | -9.9 | 7.1 |
| 2-2-d1E01-1-0205 | C36 H34 N4 O7 S | 666.21482 | 5.8 | 13.0 | -5.9 | 4.8 |
| 2-2-d1E01-1-0206 | C36 H34 N4 O7 S | 666.21482 | 0.6 | 5.5 | -3.2 | 4.4 |
| 2-2-d1E01-1-0207 | C34 H34 N8 O5 S | 666.23729 | 14.3 | 35.1 | -0.5 | 6.8 |
| 2-2-d1E01-1-0208 | C33 H33 F3 N6 O6 | 666.24137 | 11.6 | 36.5 | 4.7 | 8.0 |
| 2-2-d1E01-1-0209 | C38 H42 N4 O5 S | 666.28759 | 0.0 | 0.0 | 0.0 | 0.0 |
| 2-2-d1E01-1-0210 | C36 H41 F3 N4 O5 | 666.30291 | 8.0 | 28.1 | -3.9 | 1.5 |
| 2-2-d1E01-1-0211 | C31 H30 F N5 O9 S | 667.17483 | 0.0 | 0.0 | -3.8 | 6.6 |
| 2-2-d1E01-1-0212 | C35 H33 N5 O5 S2 | 667.19231 | 9.8 | 48.7 | 11.9 | 9.5 |
| 2-2-d1E01-1-0213 | C35 H33 N5 O7 S | 667.21007 | 5.1 | 10.9 | -1.7 | 5.1 |
| 2-2-d1E01-1-0214 | C35 H33 N5 O7 S | 667.21007 | 21.9 | 44.9 | 2.1 | 7.1 |
| 2-2-d1E01-1-0215 | C35 H33 N5 O7 S | 667.21007 | 0.0 | 0.0 | -12.3 | 5.7 |
| 2-2-d1E01-1-0216 | C36 H37 N5 O6 S | 667.24645 | 12.0 | 34.9 | 2.4 | 9.9 |
| 2-2-d1E01-1-0217 | C34 H32 N6 O7 S | 668.20532 | 18.2 | 38.2 | -0.2 | -0.8 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0218 | C36 H36 N4 O7 S | 668.23047 | -0.2 | 3.9 | -7.4 | 5.0 |
| 2-2-d1E01-1-0219 | C36 H36 N4 O7 S | 668.23047 | 6.8 | 4.4 | -2.7 | 10.4 |
| 2-2-d1E01-1-0220 | C35 H39 F3 N4 O6 | 668.28217 | 7.1 | 31.5 | -7.2 | 2.7 |
| 2-2-d1E01-1-0221 | C32 H30 F3 N5 O6 S | 669.18689 | 19.4 | 42.5 | -1.8 | 1.9 |
| 2-2-d1E01-1-0222 | C35 H35 N5 O7 S | 669.22572 | 2.2 | 3.4 | -7.3 | 5.0 |
| 2-2-d1E01-1-0223 | C37 H33 F3 N4 O5 | 670.24030 | 4.7 | 42.2 | -5.0 | 4.6 |
| 2-2-d1E01-1-0224 | C37 H33 F3 N4 O5 | 670.24030 | 13.1 | 47.3 | 14.8 | 3.4 |
| 2-2-d1E01-1-0225 | C37 H39 F N4 O5 S | 670.26252 | 11.0 | 54.4 | 1.1 | 4.6 |
| 2-2-d1E01-1-0226 | C33 H33 N7 O5 S2 | 671.19846 | 2.6 | 33.7 | -6.8 | 12.0 |
| 2-2-d1E01-1-0227 | C36 H32 F3 N5 O5 | 671.23555 | 13.7 | 40.3 | -0.8 | 8.0 |
| 2-2-d1E01-1-0228 | C36 H32 F3 N5 O5 | 671.23555 | 6.8 | 29.3 | -6.0 | 13.5 |
| 2-2-d1E01-1-0229 | C36 H32 F3 N5 O5 | 671.23555 | 24.0 | 32.2 | 3.3 | 5.3 |
| 2-2-d1E01-1-0230 | C35 H37 N5 O7 S | 671.24137 | 0.5 | 6.7 | -7.3 | 6.2 |
| 2-2-dlE01-1-0231 | C35 H36 N4 O8 S | 672.22538 | 2.4 | 5.9 | -9.3 | 6.5 |
| 2-2-d1E01-1-0232 | C34 H36 N6 O7 S | 672.23662 | 10.1 | 14.4 | -6.3 | 5.2 |
| 2-2-d1E01-1-0233 | C37 H35 F3 N4 O5 | 672.25595 | 10.8 | 39.6 | -1.0 | 3.1 |
| 2-2-d1E01-1-0234 | C37 H35 F3 N4 O5 | 672.25595 | 7.0 | 43.6 | 1.4 | 0.7 |
| 2-2-d1E01-1-0235 | C33 H35 N7 O7 S | 673.23187 | 9.7 | 24.3 | 7.1 | 2.9 |
| 2-2-d1E01-1-0236 | C36 H34 F3 N5 O5 | 673.25120 | 7.9 | 38.1 | -3.1 | 4.2 |
| 2-2-d1E01-1-0237 | C34 H34 N4 O7 S2 | 674.18689 | 0.0 | 0.0 | 0.0 | 0.0 |
| 2-2-d1E01-1-0238 | C33 H34 N6 O8 S | 674.21588 | 1.8 | 5.1 | -8.9 | 3.2 |
| 2-2-d1E01-1-0239 | C35 H38 N4 O8 S | 674.24103 | 4.7 | 5.8 | -6.6 | 9.1 |
| 2-2-d1E01-1-0240 | C39 H38 N4 O7 | 674.27405 | 8.0 | 52.5 | -3.8 | 13.0 |
| 2-2-d1E01-1-0241 | C33 H33 N5 O9 S | 675.19990 | 4.8 | 6.1 | -4.4 | 6.6 |
| 2-2-d1E01-1-0242 | C36 H36 F3 N5 O5 | 675.26685 | 5.9 | 47.3 | -8.5 | 3.3 |
| 2-2-d1E01-1-0243 | C34 H33 F N4 O8 S | 676.20031 | 3.5 | 8.1 | -8.8 | 4.7 |
| 2-2-d1E01-1-0244 | C33 H36 N6 O8 S | 676.23153 | 2.4 | 4.8 | -7.9 | 4.6 |
| 2-2-d1E01-1-0245 | C35 H35 F3 N6 O5 | 676.26210 | 2.3 | 33.1 | -6.8 | 8.0 |
| 2-2-d1E01-1-0246 | C36 H44 N4 O7 S | 676.29307 | 0.0 | 0.0 | -10.3 | 3.2 |
| 2-2-d1E01-1-0247 | C39 H44 N6 O5 | 676.33732 | 7.6 | 43.1 | -1.2 | 3.5 |
| 2-2-d1E01-1-0248 | C34 H34 F3 N7 O5 | 677.25735 | 9.1 | 36.5 | -6.1 | 8.8 |
| 2-2-d1E01-1-0249 | C38 H39 N5 O5 S | 677.26719 | 8.3 | 33.4 | 1.0 | 8.0 |
| 2-2-d1E01-1-0250 | C32 H31 F N6 O8 S | 678.19081 | 3.4 | 5.5 | -7.3 | 6.0 |
| 2-2-d1E01-1-0251 | C32 H34 N6 O7 S2 | 678.19304 | 0.0 | 0.0 | -6.3 | 0.0 |
| 2-2-d1E01-1-0252 | C35 H33 F3 N4 O5 S | 678.21238 | 0.0 | 0.0 | -2.5 | 0.0 |
| 2-2-d1E01-1-0253 | C38 H35 F N4 O7 | 678.24898 | 10.7 | 59.9 | 19.9 | 15.3 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0254 | C36 H37 F3 N4 O6 | 678.26652 | 9.1 | 35.0 | 1.5 | 9.7 |
| 2-2-d1E01-1-0255 | C35 H42 N4 O8 S | 678.27234 | 2.5 | 4.8 | -7.6 | 7.1 |
| 2-2-d1E01-1-0256 | C32 H33 N5 O8 S2 | 679.17705 | 4.5 | 8.7 | -7.1 | 7.3 |
| 2-2-d1E01-1-0257 | C35 H33 N7 O6 S | 679.22130 | 12.6 | 26.6 | -6.8 | 8.4 |
| 2-2-d1E01-1-0258 | C34 H32 F3 N5 O7 | 679.22538 | 4.2 | 38.3 | -1.8 | 5.8 |
| 2-2-d1E01-1-0259 | C32 H33 F N6 O8 S | 680.20646 | 1.3 | 8.5 | -4.7 | 6.2 |
| 2-2-d1E01-1-0260 | C37 H36 N4 O7 S | 680.23047 | 19.4 | 33.4 | 5.4 | 3.4 |
| 2-2-d1E01-1-0261 | C37 H36 N4 O7 S | 680.23047 | -3.9 | 10.2 | -5.6 | 6.7 |
| 2-2-d1E01-1-0262 | C35 H41 F N4 O7 S | 680.26800 | -0.6 | 1.1 | -10.7 | 3.8 |
| 2-2-d1E01-1-0263 | C38 H41 F N6 O5 | 680.31225 | 9.1 | 38.3 | -3.1 | 6.9 |
| 2-2-d1E01-1-0264 | C36 H35 N5 O7 S | 681.22572 | 5.5 | 13.2 | -3.2 | 8.2 |
| 2-2-d1E01-1-0265 | C36 H35 N5 O7 S | 681.22572 | 17.4 | 51.6 | 8.0 | 6.8 |
| 2-2-d1E01-1-0266 | C36 H35 N5 O7 S | 681.22572 | 0.0 | 0.0 | 0.0 | 0.0 |
| 2-2-d1E01-1-0267 | C35 H35 N7 O6 S | 681.23695 | 13.8 | 30.5 | -4.3 | 9.9 |
| 2-2-d1E01-1-0268 | C33 H33 F3 N6 O5 S | 682.21852 | 8.4 | 30.7 | 0.2 | 7.6 |
| 2-2-d1E01-1-0269 | C37 H38 N4 O7 S | 682.24612 | 26.3 | 3.1 | -17.1 | -0.1 |
| 2-2-d1E01-1-0270 | C37 H38 N4 O7 S | 682.24612 | 2.1 | 6.1 | -7.7 | 5.3 |
| 2-2-d1E01-1-0271 | C34 H39 FN4 O8 S | 682.24726 | 2.1 | 6.1 | -7.7 | 5.3 |
| 2-2-d1E01-1-0272 | C31 H30 F N5 O8 S2 | 683.15198 | 3.1 | 6.5 | -8.7 | 7.9 |
| 2-2-d1E01-1-0273 | C36 H37 N5 O5 S2 | 683.22361 | -14.6 | 35.5 | 0.4 | -23.7 |
| 2-2-d1E01-1-0274 | C36 H37 N5 O7 S | 683.24137 | 12.9 | 6.7 | -9.0 | 1.0 |
| 2-2-d1E01-1-0275 | C34 H32 N6 O6 S2 | 684.18247 | 10.9 | 38.8 | 1.9 | 12.0 |
| 2-2-d1E01-1-0276 | C36 H33 F N4 O7 S | 684.20540 | 7.3 | 17.8 | -8.6 | 4.0 |
| 2-2-d1E01-1-0277 | C36 H33 F N4 O7 S | 684.20540 | 1.2 | 8.4 | -11.1 | -1.1 |
| 2-2-d1E01-1-0278 | C37 H40 N4 O7 S | 684.26177 | 7.4 | 10.5 | -10.6 | 9.3 |
| 2-2-d1E01-1-0279 | C35 H32 F N5 O7 S | 685.20065 | 7.6 | 11.2 | 0.3 | 5.0 |
| 2-2-d1E01-1-0280 | C35 H32 F N5 O7 S | 685.20065 | 10.7 | 34.9 | 10.0 | 23.4 |
| 2-2-d1E01-1-0281 | C35 H32 F N5 O7 S | 685.20065 | 7.6 | 11.2 | 0.3 | 5.0 |
| 2-2-d1E01-1-0282 | C39 H35 N5 O5 S | 685.23589 | 7.3 | 39.2 | 2.4 | 8.8 |
| 2-2-d1E01-1-0283 | C39 H35 N5 O5 S | 685.23589 | 24.9 | 52.8 | 8.7 | 12.9 |
| 2-2-d1E01-1-0284 | C36 H39 N5 O7 S | 685.25702 | 3.6 | 3.4 | -8.3 | 6.2 |
| 2-2-d1E01-1-0285 | C34 H34 N6 O8 S | 686.21588 | 7.2 | 6.5 | -8.4 | 7.7 |
| 2-2-d1E01-1-0286 | C36 H35 F N4 O7 S | 686.22105 | -0.4 | -1.1 | 1.2 | 5.4 |
| 2-2-d1E01-1-0287 | C36 H35 F N4 O7 S | 686.22105 | 3.1 | 4.5 | -8.9 | 5.3 |
| 2-2-d1E01-1-0288 | C38 H34 N6 O5 S | 686.23114 | 14.9 | 37.6 | 0.0 | 9.0 |
| 2-2-d1E01-1-0289 | C38 H34 N6 O5 S | 686.23114 | 16.5 | 31.8 | -11.3 | 1.9 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0290 | C38 H34 N6 O5 S | 686.23114 | 5.2 | 43.6 | -2.7 | 6.4 |
| 2-2-d1E01-1-0291 | C35 H38 N6 O7 S | 686.25227 | 4.1 | 17.1 | -12.5 | 1.4 |
| 2-2-d1E01-1-0292 | C35 H34 F N5 O7 S | 687.21630 | 2.9 | 5.4 | -8.0 | 5.0 |
| 2-2-d1E01-1-0293 | C34 H37 N7 O7 S | 687.24752 | 10.9 | 34.2 | 4.9 | 13.9 |
| 2-2-d1E01-1-0294 | C35 H36 N4 O7 S2 | 688.20254 | 0.0 | 0.0 | 9.0 | 0.0 |
| 2-2-d1E01-1-0295 | C34 H36 N6 O8 S | 688.23153 | 7.2 | 10.4 | -7.4 | 2.3 |
| 2-2-d1E01-1-0296 | C36 H40 N4 O8 S | 688.25668 | 2.0 | 6.4 | -7.8 | 3.3 |
| 2-2-d1E01-1-0297 | C38 H39 F3 N4 O5 | 688.28725 | 12.3 | 40.2 | -6.6 | 17.9 |
| 2-2-d1E01-1-0298 | C40 H44 N6 O5 | 688.33732 | 9.2 | 42.7 | -5.0 | 10.7 |
| 2-2-d1E01-1-0299 | C33 H31 N5 O8 S2 | 689.16140 | 5.0 | 6.9 | -7.6 | 6.8 |
| 2-2-d1E01-1-0300 | C34 H35 N5 O9 S | 689.21555 | -3.4 | 3.3 | -5.0 | 1.1 |
| 2-2-d1E01-1-0301 | C35 H36 F N5 O7 S | 689.23195 | 2.1 | 6.5 | -7.7 | 4.0 |
| 2-2-d1E01-1-0302 | C35 H38 N4 O7 S2 | 690.21819 | 4.1 | 6.9 | -9.0 | 6.0 |
| 2-2-d1E01-1-0303 | C34 H35 F N6 O7 S | 690.22720 | 5.9 | 15.1 | 2.2 | 1.7 |
| 2-2-d1E01-1-0304 | C35 H33 F3 N6 O6 | 690.24137 | 7.0 | 36.6 | 4.0 | 5.9 |
| 2-2-d1E01-1-0305 | C38 H38 N6 O5 S | 690.26244 | 8.0 | 52.0 | -1.9 | 8.2 |
| 2-2-d1E01-1-0306 | C33 H33 N5 O8 S2 | 691.17705 | 6.4 | 5.9 | -9.0 | 6.2 |
| 2-2-d1E01-1-0307 | C37 H33 N5 O7 S | 691.21007 | 5.3 | 47.3 | -2.9 | 6.9 |
| 2-2-d1E01-1-0308 | C33 H34 F N7 O7 S | 691.22245 | 3.3 | 10.7 | -4.5 | 5.8 |
| 2-2-d1E01-1-0309 | C37 H37 N7 O5 S | 691.25769 | 11.9 | 33.1 | -10.7 | 2.4 |
| 2-2-d1E01-1-0310 | C34 H33 F N4 O7 S2 | 692.17747 | 7.8 | 13.9 | -13.8 | 15.9 |
| 2-2-d1E01-1-0311 | C33 H36 N6 O7 S2 | 692.20869 | 2.5 | 8.5 | -8.5 | 2.3 |
| 2-2-d1E01-1-0312 | C38 H36 N4 O7 S | 692.23047 | 6.7 | 43.9 | -6.3 | -0.2 |
| 2-2-d1E01-1-0313 | C38 H36 N4 O7 S | 692.23047 | 0.0 | 0.0 | 0.0 | 0.0 |
| 2-2-d1E01-1-0314 | C35 H37 F N4 O8 S | 692.23161 | 2.1 | 4.6 | -6.5 | 8.5 |
| 2-2-d1E01-1-0315 | C36 H36 N8 O5 S | 692.25294 | 15.4 | 29.3 | -1.7 | 8.6 |
| 2-2-d1E01-1-0316 | C35 H35 F3 N6 O6 | 692.25702 | 1.9 | 30.0 | -5.7 | 1.6 |
| 2-2-d1E01-1-0317 | C33 H32 FN5 O9 S | 693.19048 | 4.1 | 3.0 | -5.1 | 5.5 |
| 2-2-d1E01-1-0318 | C37 H35 N5 O7 S | 693.22572 | 6.0 | 11.2 | -5.8 | 8.8 |
| 2-2-d1E01-1-0319 | C37 H35 N5 O7 S | 693.22572 | 9.8 | 22.4 | 26.5 | 2.2 |
| 2-2-d1E01-1-0320 | C37 H35 N5 O7 S | 693.22572 | -1.9 | 0.7 | -0.1 | 10.8 |
| 2-2-d1E01-1-0321 | C34 H39 N5 O7 S2 | 693.22909 | 0.7 | 2.6 | -6.6 | 4.4 |
| 2-2-d1E01-1-0322 | C38 H39 N5 O6 S | 693.26210 | 9.1 | 40.5 | -1.9 | 6.6 |
| 2-2-d1E01-1-0323 | C36 H37 F3 N4 O5 S | 694.24368 | 15.5 | 44.2 | 5.7 | 5.7 |
| 2-2-d1E01-1-0324 | C34 H32 F3 N5 O6 S | 695.20254 | 11.3 | 33.3 | -2.5 | 1.1 |
| 2-2-d1E01-1-0325 | C33 H37 N5 O8 S2 | 695.20835 | 0.6 | 3.4 | -6.4 | 5.0 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0326 | C32 H33 F N6 O7 S2 | 696.18362 | 3.8 | 7.2 | -7.2 | 5.2 |
| 2-2-d1E01-1-0327 | C39 H35 F3 N4 O5 | 696.25595 | 0.0 | 0.0 | 0.0 | 0.0 |
| 2-2-d1E01-1-0328 | C39 H35 F3 N4 O5 | 696.25595 | 6.8 | 37.4 | -4.5 | 8.0 |
| 2-2-d1E01-1-0329 | C35 H35 N7 O5 S2 | 697.21411 | 14.4 | 32.6 | -0.6 | 3.1 |
| 2-2-d1E01-1-0330 | C38 H34 F3 N5 O5 | 697.25120 | 1.4 | 34.9 | -8.8 | 16.3 |
| 2-2-d1E01-1-0331 | C38 H34 F3 N5 O5 | 697.25120 | 10.3 | 49.5 | 0.6 | 12.4 |
| 2-2-d1E01-1-0332 | C38 H34 F3 N5 O5 | 697.25120 | 4.5 | 36.2 | -21.8 | -4.2 |
| 2-2-d1E01-1-0333 | C37 H39 N5 O7 S | 697.25702 | 37.8 | 44.6 | 4.9 | 16.0 |
| 2-2-d1E01-1-0334 | C36 H34 N4 O9 S | 698.20465 | 6.0 | 8.8 | -6.4 | 7.0 |
| 2-2-d1E01-1-0335 | C36 H38 N6 O7 S | 698.25227 | 2.8 | 0.0 | -13.4 | 13.4 |
| 2-2-d1E01-1-0336 | C38 H42 N4 O7 S | 698.27742 | 3.2 | 9.8 | -0.4 | 7.6 |
| 2-2-d1E01-1-0337 | C35 H33 N5 O7 S2 | 699.18214 | 6.9 | 12.2 | -9.0 | 1.6 |
| 2-2-d1E01-1-0338 | C35 H33 N5 O7 S2 | 699.18214 | 5.1 | 9.6 | -7.2 | 6.1 |
| 2-2-d1E01-1-0339 | C35 H37 N7 O7 S | 699.24752 | 2.5 | 11.8 | -6.2 | 7.1 |
| 2-2-d1E01-1-0340 | C34 H32 N6 O7 S2 | 700.17739 | 5.6 | 5.4 | -8.3 | 3.4 |
| 2-2-d1E01-1-0341 | C33 H31 F3 N4 O8 S | 700.18147 | 10.0 | 30.6 | 0.1 | 0.5 |
| 2-2-d1E01-1-0342 | C35 H36 N6 O8 S | 700.23153 | 0.0 | 0.0 | -4.4 | 4.6 |
| 2-2-d1E01-1-0343 | C37 H40 N4 O8 S | 700.25668 | 0.4 | 12.8 | -2.9 | 5.3 |
| 2-2-d1E01-1-0344 | C38 H38 F3 N5 O5 | 701.28250 | 12.0 | 38.1 | 6.9 | 1.4 |
| 2-2-d1E01-1-0345 | C37 H33 F3 N4 O7 | 702.23013 | 0.3 | 18.0 | -2.0 | 5.0 |
| 2-2-d1E01-1-0346 | C35 H38 N6 O8 S | 702.24718 | 7.9 | 13.2 | -5.8 | 3.9 |
| 2-2-d1E01-1-0347 | C37 H39 F N4 O7 S | 702.25235 | 8.1 | 20.7 | -7.8 | 12.1 |
| 2-2-d1E01-1-0348 | C37 H37 F3 N6 O5 | 702.27775 | 1.7 | 44.9 | -13.7 | 9.1 |
| 2-2-d1E01-1-0349 | C41 H46 N6 O5 | 702.35297 | 24.6 | 59.0 | 5.5 | 16.8 |
| 2-2-d1E01-1-0350 | C36 H36 F3 N7 O5 | 703.27300 | 11.2 | 41.4 | 3.3 | 5.7 |
| 2-2-d1E01-1-0351 | C40 H41 N5 O5 S | 703.28284 | 10.7 | 38.3 | -0.3 | 6.3 |
| 2-2-d1E01-1-0352 | C34 H33 F N6 O8 S | 704.20646 | 0.0 | 0.0 | 0.0 | 0.0 |
| 2-2-d1E01-1-0353 | C34 H36 N6 O7 S2 | 704.20869 | 0.0 | 13.7 | 10.9 | 0.0 |
| 2-2-d1E01-1-0354 | C36 H40 N4 O7 S2 | 704.23384 | 5.5 | 16.3 | -12.9 | 10.6 |
| 2-2-d1E01-1-0355 | C34 H39 F3 N4 O7 S | 704.24915 | 6.1 | 9.5 | -2.1 | 6.6 |
| 2-2-d1E01-1-0356 | C38 H39 F3 N4 O6 | 704.28217 | 12.3 | 36.8 | -16.4 | 7.6 |
| 2-2-d1E01-1-0357 | C33 H31 N5 O7 S3 | 705.13856 | 5.2 | 11.0 | -7.1 | 6.7 |
| 2-2-d1E01-1-0358 | C34 H35 N5 O8 S2 | 705.19270 | 4.4 | 35.0 | 3.7 | 16.0 |
| 2-2-d1E01-1-0359 | C32 H30 N6 O9 S2 | 706.15157 | 6.3 | 4.6 | -2.8 | 4.7 |
| 2-2-d1E01-1-0360 | C34 H35 F N6 O8 S | 706.22211 | 6.4 | 13.3 | -9.2 | 3.3 |
| 2-2-d1E01-1-0361 | C33 H37 F3 N4 O8 S | 706.22842 | 0.5 | 4.3 | -10.8 | 3.6 |

(continued)

[Table 11-3-1]

| | | | AS-MS(%) | | | |
|---|---|---|---|---|---|---|
| ID | Molecular Formula | Exact Mass | N1 | N2 | N3 | N4 |
| 2-2-d1E01-1-0362 | C39 H38 N4 O7 S | 706.24612 | 9.6 | 57.9 | -5.7 | 12.5 |
| 2-2-d1E01-1-0363 | C39 H38 N4 O7 S | 706.24612 | -2.8 | 36.9 | 2.3 | 12.5 |
| 2-2-d1E01-1-0364 | C40 H43 F N6 O5 | 706.32790 | 9.3 | 40.0 | -1.1 | 8.7 |
| 2-2-d1E01-1-0365 | C38 H37 N5 O7 S | 707.24137 | 5.6 | 9.6 | -9.5 | 6.4 |
| 2-2-d1E01-1-0366 | C38 H37 N5 O7 S | 707.24137 | 7.7 | 48.3 | -4.6 | -1.2 |
| 2-2-d1E01-1-0367 | C38 H37 N5 O7 S | 707.24137 | 0.0 | 0.0 | 0.0 | 0.0 |
| 2-2-d1E01-1-0368 | C35 H37 F N4 O7 S2 | 708.20877 | 0.9 | 6.9 | -6.4 | 5.8 |
| 2-2-d1E01-1-0369 | C35 H35 F3 N6 O5 S | 708.23417 | 9.0 | 31.1 | -9.6 | 4.1 |
| 2-2-d1E01-1-0370 | C33 H32 F N5 O8 S2 | 709.16763 | 2.8 | 10.6 | -8.6 | 4.8 |
| 2-2-d1E01-1-0371 | C38 H39 N5 O5 S2 | 709.23926 | 11.7 | 36.8 | -5.7 | 6.8 |
| 2-2-d1E01-1-0372 | C35 H33 F3 N4 O7 S | 710.20220 | 5.6 | 28.3 | -7.5 | 5.9 |
| 2-2-d1E01-1-0373 | C35 H33 F3 N4 O7 S | 710.20220 | 6.9 | 16.4 | -6.8 | 4.3 |
| 2-2-d1E01-1-0374 | C38 H35 F N4 O7 S | 710.22105 | 12.4 | 29.3 | -8.2 | 1.1 |
| 2-2-d1E01-1-0375 | C38 H35 F N4 O7 S | 710.22105 | 0.0 | 9.6 | 0.0 | 0.0 |
| 2-2-d1E01-1-0376 | C39 H42 N4 O7 S | 710.27742 | 4.1 | 4.6 | 9.3 | 6.8 |
| 2-2-d1E01-1-0377 | C34 H32 F3 N5 O7 S | 711.19745 | 3.8 | 11.8 | -4.5 | 6.4 |
| 2-2-d1E01-1-0378 | C37 H34 F N5 O7 S | 711.21630 | 16.0 | 44.4 | 14.0 | 13.0 |
| 2-2-d1E01-1-0379 | C37 H34 F N5 O7 S | 711.21630 | 0.0 | 9.5 | -16.6 | 8.2 |
| 2-2-d1E01-1-0380 | C37 H34 F N5 O7 S | 711.21630 | -2.3 | 18.8 | 0.0 | 0.0 |
| 2-2-d1E01-1-0381 | C38 H41 N5 O7 S | 711.27267 | 0.0 | 0.0 | 0.0 | 0.0 |
| 2-2-d1E01-1-0382 | C37 H36 N4 O9 S | 712.22030 | 3.5 | 13.2 | -6.2 | 4.7 |
| 2-2-d1E01-1-0383 | C37 H40 N6 O7 S | 712.26792 | 2.3 | 2.7 | 0.0 | 0.0 |
| 2-2-d1E01-1-0384 | C36 H39 N7 O7 S | 713.26317 | 7.0 | 35.6 | 5.4 | 13.8 |
| 2-2-d1E01-1-0385 | C38 H42 N4 O8 S | 714.27234 | -3.9 | 8.0 | -3.5 | 7.3 |
| 2-2-d1E01-1-0386 | C40 H41 F3 N4 O5 | 714.30291 | -0.8 | 48.4 | -11.5 | 11.1 |
| 2-2-d1E01-1-0387 | C35 H33 N5 O8 S2 | 715.17705 | 3.5 | 11.1 | -6.5 | 5.0 |
| 2-2-d1E01-1-0388 | C37 H38 F N5 O7 S | 715.24760 | 0.0 | 7.7 | -16.9 | 2.7 |
| 2-2-d1E01-1-0389 | C33 H31 F3 N4 O7 S2 | 716.15863 | 8.4 | 25.0 | -5.3 | -0.1 |
| 2-2-d1E01-1-0390 | C36 H33 F N4 O9 S | 716.19523 | 5.0 | 9.6 | -6.9 | 6.6 |
| 2-2-d1E01-1-0391 | C37 H40 N4 O7 S2 | 716.23384 | 4.7 | 23.8 | -10.2 | 8.5 |
| 2-2-d1E01-1-0392 | C36 H37 F N6 O7 S | 716.24285 | 10.0 | 0.0 | -13.5 | 0.0 |
| 2-2-d1E01-1-0393 | C33 H31 N7 O8 S2 | 717.16755 | 3.8 | 5.7 | -6.2 | 6.2 |
| 2-2-d1E01-1-0394 | C32 H30 F3 N5 O9 S | 717.17163 | 9.0 | 9.4 | -27.5 | -7.7 |
| 2-2-d1E01-1-0395 | C39 H35 N5 O7 S | 717.22572 | 22.5 | 44.6 | 3.3 | 2.4 |
| 2-2-d1E01-1-0396 | C35 H36 F N7 O7 S | 717.23810 | 9.5 | 40.2 | 9.4 | 14.2 |
| 2-2-d1E01-1-0397 | C35 H38 N6 O7 S2 | 718.22434 | 0.0 | 0.0 | 0.0 | 0.0 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0398 | C37 H39 F N4 O8 S | 718.24726 | 3.4 | 6.8 | -12.8 | 7.1 |
| 2-2-d1E01-1-0399 | C33 H33 N7 O8 S2 | 719.18320 | 3.7 | 6.5 | -6.4 | 6.5 |
| 2-2-d1E01-1-0400 | C36 H41 N5 O7 S2 | 719.24474 | 3.5 | 11.3 | -11.7 | 2.6 |
| 2-2-d1E01-1-0401 | C39 H41 N7 O5 S | 719.28899 | 25.3 | 44.9 | 7.3 | 10.3 |
| 2-2-d1E01-1-0402 | C38 H39 F3 N4 O5 S | 720.25933 | 9.0 | 28.5 | -5.9 | 16.5 |
| 2-2-d1E01-1-0403 | C35 H39 N5 O8 S2 | 721.22400 | 1.6 | 5.1 | -5.7 | 5.0 |
| 2-2-d1E01-1-0404 | C32 H30 N6 O8 S3 | 722.12872 | 6.4 | 9.4 | -5.8 | 3.6 |
| 2-2-d1E01-1-0405 | C34 H35 F N6 O7 S2 | 722.19927 | 10.7 | 34.7 | -4.5 | -0.3 |
| 2-2-d1E01-1-0406 | C37 H33 N5 O7 S2 | 723.18214 | 6.9 | 21.7 | 0.0 | 8.3 |
| 2-2-d1E01-1-0407 | C37 H33 N5 O7 S2 | 723.18214 | 19.2 | 30.0 | -7.7 | -1.2 |
| 2-2-d1E01-1-0408 | C36 H32 N6 O7 S2 | 724.17739 | 5.9 | 16.5 | -5.9 | 5.6 |
| 2-2-d1E01-1-0409 | C36 H32 N6 O7 S2 | 724.17739 | 26.0 | 46.0 | -8.2 | 5.9 |
| 2-2-d1E01-1-0410 | C36 H32 N6 O7 S2 | 724.17739 | 0.0 | 0.0 | 0.0 | 0.0 |
| 2-2-d1E01-1-0411 | C38 H36 N4 O9 S | 724.22030 | 4.4 | 9.2 | -4.7 | 7.2 |
| 2-2-d1E01-1-0412 | C40 H44 N4 O7 S | 724.29307 | 10.3 | 18.3 | -11.6 | 2.7 |
| 2-2-d1E01-1-0413 | C37 H35 N5 O7 S2 | 725.19779 | 7.2 | 19.4 | -6.5 | 1.4 |
| 2-2-d1E01-1-0414 | C37 H35 N5 O7 S2 | 725.19779 | 24.0 | 17.5 | -1.1 | -0.3 |
| 2-2-d1E01-1-0415 | C36 H34 N6 O7 S2 | 726.19304 | 2.8 | 9.9 | -5.8 | 5.0 |
| 2-2-d1E01-1-0416 | C35 H33 F3 N4 O8 S | 726.19712 | 5.0 | 21.8 | -6.9 | 7.7 |
| 2-2-d1E01-1-0417 | C38 H42 N6 O7 S | 726.28357 | 3.4 | 6.4 | -1.2 | 8.1 |
| 2-2-d1E01-1-0418 | C33 H31 F3 N6 O8 S | 728.18762 | 0.0 | 0.0 | 0.0 | 0.0 |
| 2-2-d1E01-1-0419 | C36 H36 N6 O7 S2 | 728.20869 | 3.9 | 7.2 | -6.2 | 4.6 |
| 2-2-d1E01-1-0420 | C39 H35 F3 N4 O7 | 728.24578 | 10.7 | 44.0 | -9.6 | 12.3 |
| 2-2-d1E01-1-0421 | C39 H41 F N4 O7 S | 728.26800 | -0.5 | 4.3 | -4.1 | 2.8 |
| 2-2-d1E01-1-0422 | C35 H35 N7 O7 S2 | 729.20394 | 0.0 | 25.7 | 0.2 | 1.7 |
| 2-2-d1E01-1-0423 | C34 H34 N8 O7 S2 | 730.19919 | 8.0 | 33.5 | 3.1 | 9.2 |
| 2-2-d1E01-1-0424 | C33 H33 F3 N6 O8 S | 730.20327 | 4.0 | 17.1 | -10.1 | 6.7 |
| 2-2-d1E01-1-0425 | C38 H42 N4 O7 S2 | 730.24949 | 0.0 | 0.0 | -3.5 | -2.0 |
| 2-2-d1E01-1-0426 | C36 H41 F3 N4 O7 S | 730.26480 | -1.9 | 7.2 | -4.2 | -2.0 |
| 2-2-d1E01-1-0427 | C39 H41 F3 N6 O5 | 730.30905 | 11.7 | 46.7 | -5.1 | 8.5 |
| 2-2-d1E01-1-0428 | C35 H33 N5 O7 S3 | 731.15421 | 9.2 | 20.0 | -8.2 | 2.7 |
| 2-2-d1E01-1-0429 | C36 H37 N5 O8 S2 | 731.20835 | 1.1 | 8.2 | -5.3 | 2.7 |
| 2-2-d1E01-1-0430 | C34 H32 N6 O9 S2 | 732.16722 | 1.8 | 6.9 | -6.8 | 4.4 |
| 2-2-d1E01-1-0431 | C35 H39 F3 N4 O8 S | 732.24407 | 6.1 | 2.8 | 4.7 | 8.8 |
| 2-2-d1E01-1-0432 | C32 H30 F3 N5 O8 S2 | 733.14879 | 21.6 | 16.8 | -24.7 | 9.6 |
| 2-2-d1E01-1-0433 | C37 H33 F3 N4 O7 S | 734.20220 | 2.5 | 30.0 | -13.9 | 5.1 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0434 | C37 H33 F3 N4 O7 S | 734.20220 | 24.4 | 40.0 | -13.9 | 15.8 |
| 2-2-d1E01-1-0435 | C37 H39 F N4 O7 S2 | 734.22442 | -7.4 | 33.1 | -17.4 | 9.7 |
| 2-2-d1E01-1-0436 | C33 H33 N7 O7 S3 | 735.16036 | 2.2 | 14.4 | -6.6 | 3.5 |
| 2-2-d1E01-1-0437 | C36 H32 F3 N5 O7 S | 735.19745 | 5.5 | 21.9 | -11.2 | -0.2 |
| 2-2-d1E01-1-0438 | C36 H32 F3 N5 O7 S | 735.19745 | 12.0 | 36.3 | -3.7 | 7.5 |
| 2-2-d1E01-1-0439 | C36 H32 F3 N5 O7 S | 735.19745 | 0.0 | 0.0 | -21.8 | 19.6 |
| 2-2-d1E01-1-0440 | C37 H35 F3 N4 O7 S | 736.21785 | 9.1 | 25.7 | -8.4 | 2.4 |
| 2-2-d1E01-1-0441 | C37 H35 F3 N4 O7 S | 736.21785 | 9.1 | 39.9 | -2.2 | 1.5 |
| 2-2-d1E01-1-0442 | C36 H34 F3 N5 O7 S | 737.21310 | 4.3 | 11.8 | -8.9 | 4.4 |
| 2-2-d1E01-1-0443 | C39 H38 N4 O9 S | 738.23595 | 20.2 | 22.1 | -11.5 | 6.7 |
| 2-2-d1E01-1-0444 | C36 H36 F3 N5 O7 S | 739.22875 | 19.1 | 21.8 | 6.0 | -1.9 |
| 2-2-d1E01-1-0445 | C35 H35 F3 N6 O7 S | 740.22400 | 4.0 | 12.7 | -8.2 | 4.0 |
| 2-2-d1E01-1-0446 | C39 H44 N6 O7 S | 740.29922 | 5.3 | 7.0 | -6.9 | 9.6 |
| 2-2-d1E01-1-0447 | C34 H34 F3 N7 O7 S | 741.21925 | 11.1 | 47.2 | -0.6 | 8.3 |
| 2-2-d1E01-1-0448 | C38 H39 N5 O7 S2 | 741.22909 | 5.9 | 20.4 | -8.4 | 10.4 |
| 2-2-d1E01-1-0449 | C35 H33 F3 N4 O7 S2 | 742.17428 | 8.5 | 45.5 | -12.1 | 9.1 |
| 2-2-d1E01-1-0450 | C38 H35 F N4 O9 S | 742.21088 | 6.0 | 9.8 | -9.1 | 4.8 |
| 2-2-d1E01-1-0451 | C36 H37 F3 N4 O8 S | 742.22842 | 8.8 | 17.9 | -8.8 | 5.4 |
| 2-2-d1E01-1-0452 | C35 H33 N7 O8 S2 | 743.18320 | 2.4 | 8.6 | -7.3 | 5.3 |
| 2-2-d1E01-1-0453 | C34 H32 F3 N5 O9 S | 743.18728 | -10.7 | 77.6 | -18.6 | -42.7 |
| 2-2-d1E01-1-0454 | C38 H41 F N6 O7 S | 744.27415 | 4.8 | 9.3 | -5.4 | 8.9 |
| 2-2-d1E01-1-0455 | C35 H35 N7 O8 S2 | 745.19885 | 5.0 | 16.1 | -5.0 | 5.9 |
| 2-2-d1E01-1-0456 | C41 H43 N7 O5 S | 745.30464 | 1.9 | 40.9 | -0.3 | 15.7 |
| 2-2-d1E01-1-0457 | C33 H33 F3 N6 O7 S2 | 746.18042 | 11.1 | 12.8 | -8.3 | 2.6 |
| 2-2-d1E01-1-0458 | C36 H37 N5 O7 S3 | 747.18551 | 14.3 | 19.5 | -0.8 | 2.8 |
| 2-2-d1E01-1-0459 | C34 H32 N6 O8 S3 | 748.14437 | 8.7 | 33.2 | -3.8 | 9.0 |
| 2-2-d1E01-1-0460 | C39 H35 N5 O7 S2 | 749.19779 | 2.7 | 39.4 | -1.3 | 20.3 |
| 2-2-d1E01-1-0461 | C39 H35 N5 O7 S2 | 749.19779 | 1.9 | 37.5 | -5.9 | 6.0 |
| 2-2-d1E01-1-0462 | C38 H34 N6 O7 S2 | 750.19304 | 7.0 | 16.4 | -8.8 | 6.0 |
| 2-2-d1E01-1-0463 | C38 H34 N6 O7 S2 | 750.19304 | 17.0 | 41.7 | 6.2 | -3.1 |
| 2-2-d1E01-1-0464 | C38 H34 N6 O7 S2 | 750.19304 | 0.0 | 0.0 | 0.0 | 0.0 |
| 2-2-d1E01-1-0465 | C38 H39 F3 N4 O7 S | 752.24915 | 82.4 | 91.8 | 111.8 | 101.3 |
| 2-2-d1E01-1-0466 | C40 H44 N6 O7 S | 752.29922 | 6.4 | 5.3 | -3.2 | 4.8 |
| 2-2-d1E01-1-0467 | C35 H33 F3 N6 O8 S | 754.20327 | 4.0 | 26.2 | -3.4 | 10.2 |
| 2-2-d1E01-1-0468 | C38 H38 N6 O7 S2 | 754.22434 | 7.3 | 22.8 | -8.0 | 9.9 |
| 2-2-d1E01-1-0469 | C37 H33 N5 O9 S2 | 755.17197 | 0.2 | 289.5 | -6.2 | 1.9 |

(continued)

| [Table 11-3-1] | | | | | | |
|---|---|---|---|---|---|---|
| | | | AS-MS(%) | | | |
| ID | Molecular Formula | Exact Mass | N1 | N2 | N3 | N4 |
| 2-2-d1E01-1-0470 | C37 H37 N7 O7 S2 | 755.21959 | 12.0 | 22.0 | 0.3 | -2.1 |
| 2-2-d1E01-1-0471 | C36 H36 N8 O7 S2 | 756.21484 | 10.5 | 39.1 | -3.1 | 11.1 |
| 2-2-d1E01-1-0472 | C35 H35 F3 N6 O8 S | 756.21892 | 4.6 | 29.2 | -1.7 | 4.5 |
| 2-2-d1E01-1-0473 | C41 H43 F3 N6 O5 | 756.32470 | 7.5 | 42.6 | 4.8 | 7.6 |
| 2-2-d1E01-1-0474 | C38 H39 N5 O8 S2 | 757.22400 | -0.1 | 12.0 | 0.6 | 9.3 |
| 2-2-d1E01-1-0475 | C36 H37 F3 N4 O7 S2 | 758.20558 | 8.7 | 27.8 | -10.2 | 2.9 |
| 2-2-d1E01-1-0476 | C34 H32 F3 N5 O8 S2 | 759.16444 | 5.9 | 0.0 | 23.3 | 2.1 |
| 2-2-d1E01-1-0477 | C39 H35 F3 N4 O7 S | 760.21785 | 10.6 | 26.2 | 0.2 | 7.4 |
| 2-2-d1E01-1-0478 | C39 H35 F3 N4 O7 S | 760.21785 | 0.0 | 0.0 | 0.0 | 0.0 |
| 2-2-d1E01-1-0479 | C35 H35 N7 O7 S3 | 761.17601 | 18.1 | 26.6 | -10.9 | 3.3 |
| 2-2-d1E01-1-0480 | C38 H34 F3 N5 O7 S | 761.21310 | 2.9 | 27.4 | -6.0 | 6.9 |
| 2-2-d1E01-1-0481 | C38 H34 F3 N5 O7 S | 761.21310 | 19.8 | 48.1 | -2.1 | 5.3 |
| 2-2-d1E01-1-0482 | C38 H34 F3 N5 O7 S | 761.21310 | 4.1 | 5.7 | -3.6 | 4.2 |
| 2-2-d1E01-1-0483 | C38 H38 F3 N5 O7 S | 765.24440 | 0.0 | 25.1 | 0.0 | 0.0 |
| 2-2-d1E01-1-0484 | C37 H33 F3 N4 O9 S | 766.19203 | -11.3 | 36.6 | 10.4 | 10.2 |
| 2-2-d1E01-1-0485 | C37 H37 F3 N6 O7 S | 766.23965 | 8.3 | 23.5 | -7.5 | 3.6 |
| 2-2-d1E01-1-0486 | C41 H46 N6 O7 S | 766.31487 | 4.3 | 9.2 | -6.4 | 6.5 |
| 2-2-d1E01-1-0487 | C36 H36 F3 N7 O7 S | 767.23490 | 12.7 | 34.2 | 15.9 | 21.4 |
| 2-2-d1E01-1-0488 | C40 H41 N5 O7 S2 | 767.24474 | 3.3 | 19.8 | -8.6 | 5.6 |
| 2-2-d1E01-1-0489 | C38 H39 F3 N4 O8 S | 768.24407 | 9.8 | 11.9 | 17.8 | -2.4 |
| 2-2-d1E01-1-0490 | C40 H43 F N6 O7 S | 770.28980 | 2.0 | 7.6 | -3.7 | 3.0 |
| 2-2-d1E01-1-0491 | C35 H35 F3 N6 O7 S2 | 772.19607 | 10.0 | 40.8 | -1.5 | 3.9 |
| 2-2-d1E01-1-0492 | C38 H39 N5 O7 S3 | 773.20116 | 9.4 | 30.4 | -4.2 | 6.1 |
| 2-2-d1E01-1-0493 | C40 H41 F3 N4 O7 S | 778.26480 | 8.2 | 20.1 | -3.0 | 3.0 |
| 2-2-d1E01-1-0494 | C39 H35 N5 O9 S2 | 781.18762 | 8.0 | 14.6 | 9.2 | 7.0 |
| 2-2-d1E01-1-0495 | C39 H41 N7 O7 S2 | 783.25089 | 3.7 | 9.7 | -4.2 | 7.8 |
| 2-2-d1E01-1-0496 | C38 H39 F3 N4 O7 S2 | 784.22123 | 6.6 | 22.4 | -6.0 | 4.3 |
| 2-2-d1E01-1-0497 | C39 H35 F3 N4 O9 S | 792.20768 | 9.7 | 36.1 | 8.7 | -5.2 |
| 2-2-d1E01-1-0498 | C39 H41 F3 N6 O7 S | 794.27095 | 14.9 | 30.5 | 8.0 | 14.0 |
| 2-2-d1E01-1-0499 | C41 H43 N7 O7 S2 | 809.26654 | 3.9 | 15.2 | -5.9 | 6.1 |
| 2-2-d1E01-1-0500 | C41 H43 F3 N6 O7 S | 820.28660 | 2.5 | 20.7 | 1.8 | 14.1 |

[Table 599]

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0001 | C32 H32 N4 O7 | 584.22710 | 2.6 | 8.5 | 2.8 | -1.5 |
| 2-2-d1E02-1-0002 | C33 H40 N4 O6 | 588.29479 | 2.9 | 4.8 | 0.0 | -2.6 |
| 2-2-d1E02-1-0003 | C32 H38 N4 O7 | 590.27405 | 2.2 | 4.1 | -0.6 | -1.9 |
| 2-2-d1E02-1-0004 | C34 H34 N4 O6 | 594.24783 | 4.8 | 9.8 | 3.5 | 0.4 |
| 2-2-d1E02-1-0005 | C34 H34 N4 O6 | 594.24783 | 5.8 | 10.8 | 5.5 | 0.3 |
| 2-2-d1E02-1-0006 | C33 H33 N5 O6 | 595.24308 | 4.0 | 5.3 | 1.9 | 0.3 |
| 2-2-d1E02-1-0007 | C33 H34 N4 O7 | 598.24275 | 6.5 | 9.8 | 2.8 | 2.0 |
| 2-2-d1E02-1-0008 | C32 H32 N4 O6 S | 600.20426 | 5.5 | 4.6 | 3.7 | 0.6 |
| 2-2-d1E02-1-0009 | C31 H31 N5 O8 | 601.21726 | 1.9 | 5.3 | 0.6 | -3.0 |
| 2-2-d1E02-1-0010 | C32 H31 F N4 O7 | 602.21768 | 5.7 | 8.4 | 1.4 | -1.3 |
| 2-2-d1E02-1-0011 | C34 H42 N4 O6 | 602.31044 | 3.6 | 5.2 | 0.0 | -2.0 |
| 2-2-d1E02-1-0012 | C33 H40 N4 O7 | 604.28970 | 2.1 | 4.2 | 1.1 | -2.2 |
| 2-2-d1E02-1-0013 | C33 H39 F N4 O6 | 606.28536 | 3.0 | 4.0 | 2.2 | -1.3 |
| 2-2-d1E02-1-0014 | C35 H36 N4 O6 | 608.26348 | 4.2 | 12.1 | 5.5 | 1.6 |
| 2-2-d1E02-1-0015 | C35 H36 N4 O6 | 608.26348 | 9.2 | 13.6 | 3.1 | 1.4 |
| 2-2-d1E02-1-0016 | C32 H37 F N4 O7 | 608.26463 | 1.5 | 3.9 | 1.8 | -1.4 |
| 2-2-d1E02-1-0017 | C34 H35 N5 O6 | 609.25873 | 4.0 | 7.0 | 2.2 | 0.9 |
| 2-2-d1E02-1-0018 | C34 H34 N4 O7 | 610.24275 | 6.3 | 7.8 | 7.6 | 3.3 |
| 2-2-d1E02-1-0019 | C32 H32 N6 O7 | 612.23325 | 4.3 | 5.7 | 0.6 | 1.4 |
| 2-2-d1E02-1-0020 | C34 H33 F N4 O6 | 612.23841 | 9.1 | 9.7 | 3.2 | 2.6 |
| 2-2-d1E02-1-0021 | C34 H33 F N4 O6 | 612.23841 | 4.8 | 8.0 | 2.8 | 0.1 |
| 2-2-d1E02-1-0022 | C33 H32 F N5 O6 | 613.23366 | 2.4 | 5.2 | 0.1 | -1.4 |
| 2-2-d1E02-1-0023 | C33 H34 N4 O6 S | 614.21991 | 5.5 | 11.7 | 3.9 | 2.4 |
| 2-2-d1E02-1-0024 | C32 H34 N6 O7 | 614.24890 | 2.0 | 5.8 | 1.9 | -0.7 |
| 2-2-d1E02-1-0025 | C35 H42 N4 O6 | 614.31044 | 9.2 | 4.4 | 5.4 | -6.0 |
| 2-2-d1E02-1-0026 | C32 H33 N5 O8 | 615.23291 | 3.7 | 5.2 | 1.5 | -0.8 |
| 2-2-d1E02-1-0027 | C34 H40 N4 O7 | 616.28970 | 2.6 | 5.8 | 1.2 | -2.9 |
| 2-2-d1E02-1-0028 | C31 H31 N5 O7 S | 617.19442 | 2.9 | 5.4 | 1.6 | 2.5 |
| 2-2-d1E02-1-0029 | C32 H31 F N4 O6 S | 618.19483 | 7.3 | 9.7 | 3.7 | 2.2 |
| 2-2-d1E02-1-0030 | C36 H34 N4 O6 | 618.24783 | 9.7 | 13.2 | 6.0 | 3.6 |
| 2-2-d1E02-1-0031 | C36 H34 N4 O6 | 618.24783 | 9.5 | 13.8 | 7.6 | 0.6 |
| 2-2-d1E02-1-0032 | C31 H30 F N5 O8 | 619.20784 | 2.4 | 5.4 | 0.9 | -2.7 |
| 2-2-d1E02-1-0033 | C35 H33 N5 O6 | 619.24308 | 7.8 | 12.3 | 5.8 | 3.2 |
| 2-2-d1E02-1-0034 | C35 H33 N5 O6 | 619.24308 | 6.8 | 10.8 | 3.4 | -2.8 |
| 2-2-d1E02-1-0035 | C35 H33 N5 O6 | 619.24308 | 9.4 | 13.1 | 9.0 | 1.9 |
| 2-2-d1E02-1-0036 | C36 H36 N4 O6 | 620.26348 | 7.3 | 12.9 | 3.5 | -1.6 |
| 2-2-d1E02-1-0037 | C36 H36 N4 O6 | 620.26348 | 10.5 | 0.0 | 5.8 | 10.8 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0038 | C35 H35 N5 O6 | 621.25873 | 2.9 | 7.5 | 1.5 | -2.4 |
| 2-2-d1E02-1-0039 | C35 H37 N5 O6 | 623.27438 | 11.7 | 10.0 | 11.4 | 5.2 |
| 2-2-d1E02-1-0040 | C35 H36 N4 O7 | 624.25840 | 5.0 | 13.1 | 3.2 | -0.5 |
| 2-2-d1E02-1-0041 | C34 H36 N6 O6 | 624.26963 | 4.7 | 8.5 | 3.5 | -0.2 |
| 2-2-d1E02-1-0042 | C33 H35 N7 O6 | 625.26488 | 1.9 | 6.0 | 2.3 | -1.5 |
| 2-2-d1E02-1-0043 | C34 H34 N4 O6 S | 626.21991 | 0.0 | 0.0 | 0.0 | 0.0 |
| 2-2-d1E02-1-0044 | C33 H34 N6 O7 | 626.24890 | 1.9 | 5.5 | 1.6 | -3.4 |
| 2-2-d1E02-1-0045 | C35 H38 N4 O7 | 626.27405 | 5.6 | 10.6 | 3.5 | 0.5 |
| 2-2-d1E02-1-0046 | C33 H33 N5 O8 | 627.23291 | 14.7 | 25.4 | 7.9 | 9.1 |
| 2-2-d1E02-1-0047 | C34 H33 F N4 O7 | 628.23333 | 4.1 | 12.5 | 0.6 | 2.4 |
| 2-2-d1E02-1-0048 | C33 H36 N6 O7 | 628.26455 | 1.3 | 3.8 | 2.6 | 0.4 |
| 2-2-d1E02-1-0049 | C36 H44 N4 O6 | 628.32609 | 5.0 | 6.3 | 1.4 | 0.3 |
| 2-2-d1E02-1-0050 | C32 H31 F N6 O7 | 630.22383 | 79.9 | 82.8 | 86.2 | 81.6 |
| 2-2-d1E02-1-0051 | C32 H34 N6 O6 S | 630.22605 | 3.2 | 6.3 | 3.5 | 0.1 |
| 2-2-d1E02-1-0052 | C35 H42 N4 O7 | 630.30535 | 3.7 | 6.2 | 0.9 | -1.4 |
| 2-2-d1E02-1-0053 | C32 H33 N5 O7 S | 631.21007 | 1.9 | 6.9 | 0.7 | -0.4 |
| 2-2-d1E02-1-0054 | C32 H33 F N6 O7 | 632.23948 | 2.7 | 5.4 | 1.6 | -2.0 |
| 2-2-d1E02-1-0055 | C37 H36 N4 O6 | 632.26348 | 18.1 | 12.8 | 8.6 | 0.9 |
| 2-2-d1E02-1-0056 | C37 H36 N4 O6 | 632.26348 | 15.0 | 18.0 | 4.9 | -1.5 |
| 2-2-d1E02-1-0057 | C35 H41 F N4 O6 | 632.30101 | 1.9 | 4.0 | -0.3 | -1.5 |
| 2-2-d1E02-1-0058 | C36 H35 N5 O6 | 633.25873 | 14.8 | 15.1 | 10.8 | 0.1 |
| 2-2-d1E02-1-0059 | C36 H35 N5 O6 | 633.25873 | 11.4 | 14.2 | 9.0 | -1.7 |
| 2-2-d1E02-1-0060 | C36 H35 N5 O6 | 633.25873 | 7.2 | 11.0 | 7.3 | 6.1 |
| 2-2-d1E02-1-0061 | C37 H38 N4 O6 | 634.27913 | 5.0 | 0.0 | -0.5 | -1.2 |
| 2-2-d1E02-1-0062 | C37 H38 N4 O6 | 634.27913 | 2.1 | 5.6 | 1.7 | -2.0 |
| 2-2-d1E02-1-0063 | C34 H39 F N4 O7 | 634.28028 | 8.7 | 15.8 | 2.2 | -1.0 |
| 2-2-d1E02-1-0064 | C31 H30 F N5 O7 S | 635.18500 | 5.5 | 4.9 | 0.4 | 0.2 |
| 2-2-d1E02-1-0065 | C36 H37 N5 O6 | 635.27438 | 3.4 | 10.1 | 3.2 | -1.2 |
| 2-2-d1E02-1-0066 | C36 H33 F N4 O6 | 636.23841 | 11.1 | 12.8 | 6.5 | 3.5 |
| 2-2-d1E02-1-0067 | C36 H33 F N4 O6 | 636.23841 | 9.0 | 11.9 | 9.2 | 2.2 |
| 2-2-d1E02-1-0068 | C37 H40 N4 O6 | 636.29479 | 7.4 | 10.0 | 6.7 | 2.1 |
| 2-2-d1E02-1-0069 | C35 H32 F N5 O6 | 637.23366 | 10.1 | 10.2 | 7.4 | 4.1 |
| 2-2-d1E02-1-0070 | C35 H32 F N5 O6 | 637.23366 | 8.6 | 10.8 | 7.4 | 1.1 |
| 2-2-d1E02-1-0071 | C35 H32 F N5 O6 | 637.23366 | 13.2 | 10.2 | 9.5 | 4.4 |
| 2-2-d1E02-1-0072 | C36 H39 N5 O6 | 637.29003 | 0.9 | 12.2 | 5.6 | 3.6 |
| 2-2-d1E02-1-0073 | C34 H34 N6 O7 | 638.24890 | 0.0 | 0.0 | 0.0 | 0.0 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0074 | C36 H35 F N4 O6 | 638.25406 | 5.5 | 12.4 | 4.8 | -1.1 |
| 2-2-d1E02-1-0075 | C36 H35 F N4 O6 | 638.25406 | 6.1 | 4.4 | 0.0 | 5.0 |
| 2-2-d1E02-1-0076 | C35 H38 N6 O6 | 638.28528 | 6.8 | 10.2 | 3.2 | 2.4 |
| 2-2-d1E02-1-0077 | C35 H34 F N5 O6 | 639.24931 | 5.4 | 8.7 | 4.3 | -0.8 |
| 2-2-d1E02-1-0078 | C34 H37 N7 O6 | 639.28053 | 3.0 | 7.5 | 2.7 | -1.4 |
| 2-2-d1E02-1-0079 | C35 H36 N4 O6 S | 640.23556 | 5.2 | 0.0 | -17.8 | -14.7 |
| 2-2-d1E02-1-0080 | C34 H36 N6 O7 | 640.26455 | 2.8 | 7.1 | 2.3 | 2.8 |
| 2-2-d1E02-1-0081 | C36 H40 N4 O7 | 640.28970 | 4.0 | 9.7 | 5.4 | 0.6 |
| 2-2-d1E02-1-0082 | C33 H31 N5 O7 S | 641.19442 | 8.4 | 10.1 | 5.1 | 3.2 |
| 2-2-d1E02-1-0083 | C34 H35 N5 O8 | 641.24856 | 1.2 | 16.4 | 2.8 | 1.5 |
| 2-2-d1E02-1-0084 | C35 H36 F N5 O6 | 641.26496 | 3.0 | 4.2 | 2.9 | 0.2 |
| 2-2-d1E02-1-0085 | C35 H38 N4 O6 S | 642.25121 | 14.2 | 38.5 | 0.8 | -17.2 |
| 2-2-d1E02-1-0086 | C34 H35 F N6 O6 | 642.26021 | 3.3 | -1.1 | -13.6 | -16.8 |
| 2-2-d1E02-1-0087 | C33 H33 N5 O7 S | 643.21007 | 1.7 | 4.2 | -14.3 | -18.2 |
| 2-2-d1E02-1-0088 | C33 H34 F N7 O6 | 643.25546 | 2.4 | 6.7 | 1.8 | -2.3 |
| 2-2-d1E02-1-0089 | C34 H33 F N4 O6 S | 644.21048 | 4.4 | 12.9 | 1.8 | 1.3 |
| 2-2-d1E02-1-0090 | C33 H36 N6 O6 S | 644.24170 | 3.8 | 8.9 | 4.1 | 0.9 |
| 2-2-d1E02-1-0091 | C38 H36 N4 O6 | 644.26348 | 9.7 | 17.1 | 1.9 | 1.7 |
| 2-2-d1E02-1-0092 | C38 H36 N4 O6 | 644.26348 | 17.3 | 14.1 | 11.4 | 6.6 |
| 2-2-d1E02-1-0093 | C35 H37 F N4 O7 | 644.26463 | 4.0 | 7.2 | 1.9 | 1.7 |
| 2-2-d1E02-1-0094 | C33 H32 F N5 O8 | 645.22349 | -0.5 | 9.2 | 4.3 | 0.4 |
| 2-2-d1E02-1-0095 | C37 H35 N5 O6 | 645.25873 | 1.1 | -0.1 | 4.9 | -9.3 |
| 2-2-d1E02-1-0096 | C37 H35 N5 O6 | 645.25873 | 15.6 | 20.8 | 12.0 | 6.3 |
| 2-2-d1E02-1-0097 | C37 H35 N5 O6 | 645.25873 | 10.1 | 22.0 | 12.1 | 2.9 |
| 2-2-d1E02-1-0098 | C34 H39 N5 O6 S | 645.26210 | 3.6 | 5.0 | 1.7 | -0.5 |
| 2-2-d1E02-1-0099 | C33 H37 N5 O7 S | 647.24137 | 1.3 | 4.3 | 2.5 | -2.7 |
| 2-2-d1E02-1-0100 | C32 H32 N4 O9 S | 648.18900 | 2.2 | 2.3 | 0.6 | -0.7 |
| 2-2-d1E02-1-0101 | C32 H33 F N6 O6 S | 648.21663 | 4.8 | 7.7 | 2.8 | 2.5 |
| 2-2-d1E02-1-0102 | C37 H39 N5 O6 | 649.29003 | 0.0 | 0.0 | -1.2 | 0.1 |
| 2-2-d1E02-1-0103 | C36 H34 N4 O8 | 650.23766 | 6.9 | 12.8 | 5.3 | 2.9 |
| 2-2-d1E02-1-0104 | C36 H38 N6 O6 | 650.28528 | 0.0 | 22.8 | 4.1 | -6.1 |
| 2-2-d1E02-1-0105 | C38 H42 N4 O6 | 650.31044 | 10.3 | 11.2 | 4.2 | 0.0 |
| 2-2-d1E02-1-0106 | C35 H33 N5 O6 S | 651.21515 | 8.0 | 11.9 | 6.1 | 0.2 |
| 2-2-d1E02-1-0107 | C35 H33 N5 O6 S | 651.21515 | 5.9 | 9.5 | 4.4 | 1.7 |
| 2-2-d1E02-1-0108 | C35 H37 N7 O6 | 651.28053 | 5.0 | 8.5 | 1.0 | -0.3 |
| 2-2-d1E02-1-0109 | C34 H32 N6 O6 S | 652.21040 | 5.8 | 8.6 | 2.8 | 3.4 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0110 | C33 H31 F3 N4 O7 | 652.21448 | 8.8 | 9.7 | 6.3 | 0.2 |
| 2-2-d1E02-1-0111 | C33 H40 N4 O8 S | 652.25668 | 2.3 | 2.4 | -0.4 | -1.8 |
| 2-2-d1E02-1-0112 | C35 H36 N6 O7 | 652.26455 | 2.8 | 9.3 | 3.0 | -2.4 |
| 2-2-d1E02-1-0113 | C37 H40 N4 O7 | 652.28970 | 5.3 | 12.1 | 4.5 | 1.3 |
| 2-2-d1E02-1-0114 | C32 H38 N4 O9 S | 654.23595 | 3.1 | 2.0 | -0.7 | -0.6 |
| 2-2-d1E02-1-0115 | C35 H38 N6 O7 | 654.28020 | 3.8 | 18.5 | 2.4 | 0.3 |
| 2-2-d1E02-1-0116 | C37 H39 F N4 O6 | 654.28536 | 11.0 | 12.2 | 2.3 | 2.6 |
| 2-2-d1E02-1-0117 | C34 H33 F N6 O7 | 656.23948 | 1.8 | 3.1 | 0.9 | 0.4 |
| 2-2-d1E02-1-0118 | C34 H36 N6 O6 S | 656.24170 | 1.9 | 0.0 | 2.8 | 2.3 |
| 2-2-d1E02-1-0119 | C36 H40 N4 O6 S | 656.26686 | 4.6 | 5.3 | 3.7 | 1.0 |
| 2-2-d1E02-1-0120 | C34 H39 F3 N4 O6 | 656.28217 | 2.3 | 0.3 | -2.6 | -3.7 |
| 2-2-d1E02-1-0121 | C33 H31 N5 O6 S2 | 657.17158 | 11.0 | 15.2 | 6.9 | 2.6 |
| 2-2-d1E02-1-0122 | C34 H35 N5 O7 S | 657.22572 | 7.3 | 12.7 | 2.7 | 0.0 |
| 2-2-d1E02-1-0123 | C32 H30 N6 O8 S | 658.18458 | 4.6 | 6.8 | 2.2 | 0.4 |
| 2-2-d1E02-1-0124 | C34 H34 N4 O8 S | 658.20973 | 2.9 | 2.7 | 1.2 | -0.8 |
| 2-2-d1E02-1-0125 | C34 H34 N4 O8 S | 658.20973 | 2.5 | 3.0 | 0.0 | -1.7 |
| 2-2-d1E02-1-0126 | C34 H35 F N6 O7 | 658.25513 | 3.4 | 7.4 | 2.8 | -0.9 |
| 2-2-d1E02-1-0127 | C33 H37 F3 N4 O7 | 658.26143 | 2.6 | 4.6 | 1.4 | -1.6 |
| 2-2-d1E02-1-0128 | C39 H38 N4 O6 | 658.27913 | 18.4 | 11.9 | 15.5 | 5.5 |
| 2-2-d1E02-1-0129 | C39 H38 N4 O6 | 658.27913 | 20.2 | 15.7 | 8.6 | 0.0 |
| 2-2-d1E02-1-0130 | C33 H33 N5 O8 S | 659.20498 | 1.9 | 1.6 | -1.0 | -1.8 |
| 2-2-d1E02-1-0131 | C38 H37 N5 O6 | 659.27438 | 11.7 | 23.4 | 8.8 | 10.4 |
| 2-2-d1E02-1-0132 | C38 H37 N5 O6 | 659.27438 | 9.0 | 15.2 | 7.3 | 3.8 |
| 2-2-d1E02-1-0133 | C38 H37 N5 O6 | 659.27438 | 16.3 | 19.9 | 13.9 | 2.5 |
| 2-2-d1E02-1-0134 | C35 H37 F N4 O6 S | 660.24178 | 8.3 | 12.1 | 5.3 | 2.8 |
| 2-2-d1E02-1-0135 | C33 H32 F N5 O7 S | 661.20065 | -0.3 | 13.2 | -0.5 | -0.1 |
| 2-2-d1E02-1-0136 | C33 H34 N4 O9 S | 662.20465 | 3.4 | 2.9 | 0.4 | -0.1 |
| 2-2-d1E02-1-0137 | C35 H33 F3 N4 O6 | 662.23522 | 11.9 | 11.7 | 8.0 | -0.1 |
| 2-2-d1E02-1-0138 | C35 H33 F3 N4 O6 | 662.23522 | 6.4 | 8.4 | 3.6 | 0.6 |
| 2-2-d1E02-1-0139 | C38 H35 F N4 O6 | 662.25406 | 11.4 | 15.8 | 10.3 | 1.3 |
| 2-2-d1E02-1-0140 | C38 H35 F N4 O6 | 662.25406 | 17.4 | 28.3 | 4.6 | 1.1 |
| 2-2-d1E02-1-0141 | C39 H42 N4 O6 | 662.31044 | 9.3 | 13.7 | 9.7 | 6.1 |
| 2-2-d1E02-1-0142 | C34 H32 F3 N5 O6 | 663.23047 | 6.2 | 6.1 | 3.4 | 1.5 |
| 2-2-d1E02-1-0143 | C37 H34 F N5 O6 | 663.24931 | 13.2 | 13.6 | 8.2 | 2.9 |
| 2-2-d1E02-1-0144 | C37 H34 F N5 O6 | 663.24931 | 15.4 | 14.4 | 2.7 | 2.9 |
| 2-2-d1E02-1-0145 | C37 H34 F N5 O6 | 663.24931 | 11.3 | 13.7 | 10.7 | 4.8 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0146 | C38 H41 N5 O6 | 663.30568 | 3.4 | 5.2 | 3.4 | 0.2 |
| 2-2-d1E02-1-0147 | C32 H32 N4 O8 S2 | 664.16616 | 1.8 | 3.6 | 1.2 | 0.1 |
| 2-2-d1E02-1-0148 | C37 H36 N4 O8 | 664.25331 | 9.9 | 14.5 | 8.3 | 3.8 |
| 2-2-d1E02-1-0149 | C37 H40 N6 O6 | 664.30093 | -9.9 | -1.1 | -3.0 | 10.3 |
| 2-2-d1E02-1-0150 | C31 H31 N5 O10 S | 665.17916 | 0.0 | 0.0 | 0.0 | 0.0 |
| 2-2-d1E02-1-0151 | C36 H39 N7 O6 | 665.29618 | 4.6 | 9.4 | 4.1 | -0.4 |
| 2-2-d1E02-1-0152 | C32 H31 F N4 O9 S | 666.17958 | 2.0 | 2.8 | 0.3 | -2.0 |
| 2-2-d1E02-1-0153 | C34 H42 N4 O8 S | 666.27234 | 1.0 | 0.4 | -1.5 | -0.5 |
| 2-2-d1E02-1-0154 | C38 H42 N4 O7 | 666.30535 | 4.2 | 15.4 | 6.8 | -3.5 |
| 2-2-d1E02-1-0155 | C35 H33 N5 O7 S | 667.21007 | 7.7 | 14.7 | 6.8 | 3.1 |
| 2-2-d1E02-1-0156 | C37 H38 F N5 O6 | 667.28061 | 3.8 | 9.4 | 16.9 | 4.2 |
| 2-2-d1E02-1-0157 | C33 H31 F3 N4 O6 S | 668.19164 | 16.9 | 11.6 | 10.4 | 3.0 |
| 2-2-d1E02-1-0158 | C36 H33 F N4 O8 | 668.22824 | 9.9 | 12.7 | 6.1 | 0.7 |
| 2-2-d1E02-1-0159 | C33 H40 N4 O9 S | 668.25160 | 2.3 | 2.2 | -0.7 | -1.8 |
| 2-2-d1E02-1-0160 | C37 H40 N4 O6 S | 668.26686 | 11.9 | 0.0 | -5.0 | 6.1 |
| 2-2-d1E02-1-0161 | C36 H37 F N6 O6 | 668.27586 | 9.8 | 11.0 | 3.4 | 14.9 |
| 2-2-d1E02-1-0162 | C33 H31 N7 O7 S | 669.20057 | 2.2 | 4.8 | 1.0 | -1.6 |
| 2-2-d1E02-1-0163 | C32 H30 F3 N5 O8 | 669.20465 | 3.6 | 6.1 | 2.9 | -0.7 |
| 2-2-d1E02-1-0164 | C35 H36 F N7 O6 | 669.27111 | 2.4 | 8.5 | 4.2 | -1.2 |
| 2-2-d1E02-1-0165 | C33 H39 F N4 O8 S | 670.24726 | 1.2 | 1.6 | -1.5 | -1.9 |
| 2-2-d1E02-1-0166 | C35 H38 N6 O6 S | 670.25735 | 0.0 | 0.0 | 0.0 | 0.0 |
| 2-2-d1E02-1-0167 | C37 H39 F N4 O7 | 670.28028 | 5.2 | 10.8 | 5.9 | 2.9 |
| 2-2-d1E02-1-0168 | C33 H33 N7 O7 S | 671.21622 | 5.5 | 7.7 | 2.7 | 1.8 |
| 2-2-d1E02-1-0169 | C36 H41 N5 O6 S | 671.27775 | 4.1 | 6.8 | 4.3 | -0.6 |
| 2-2-d1E02-1-0170 | C35 H36 N4 O8 S | 672.22538 | 3.3 | 3.0 | 2.3 | 0.5 |
| 2-2-d1E02-1-0171 | C35 H36 N4 O8 S | 672.22538 | 3.1 | 3.6 | 1.8 | 0.4 |
| 2-2-d1E02-1-0172 | C32 H37 F N4 O9 S | 672.22653 | 2.1 | 1.9 | -1.8 | -0.9 |
| 2-2-d1E02-1-0173 | C34 H35 N5 O8 S | 673.22063 | 1.7 | 2.0 | 0.2 | 0.8 |
| 2-2-d1E02-1-0174 | C35 H39 N5 O7 S | 673.25702 | 2.7 | 6.3 | 2.0 | -2.2 |
| 2-2-d1E02-1-0175 | C32 H30 N6 O7 S2 | 674.16174 | 4.0 | 6.4 | 2.1 | 0.3 |
| 2-2-d1E02-1-0176 | C34 H34 N4 O9 S | 674.20465 | 2.6 | 1.9 | -0.5 | -1.2 |
| 2-2-d1E02-1-0177 | C34 H35 F N6 O6 S | 674.23228 | 5.1 | 8.3 | 0.7 | 0.6 |
| 2-2-d1E02-1-0178 | C37 H33 N5 O6 S | 675.21515 | 23.2 | 16.4 | 12.8 | 1.3 |
| 2-2-d1E02-1-0179 | C37 H33 N5 O6 S | 675.21515 | 20.9 | 17.0 | 9.5 | 3.3 |
| 2-2-d1E02-1-0180 | C32 H32 N6 O9 S | 676.19515 | -0.9 | 0.4 | 1.0 | -0.9 |
| 2-2-d1E02-1-0181 | C34 H33 F N4 O8 S | 676.20031 | 3.5 | 2.8 | 1.5 | -0.4 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0182 | C34 H33 F N4 O8 S | 676.20031 | 2.9 | 3.4 | 1.7 | -2.1 |
| 2-2-d1E02-1-0183 | C36 H32 N6 O6 S | 676.21040 | 17.8 | 10.3 | 5.4 | 2.7 |
| 2-2-d1E02-1-0184 | C36 H32 N6 O6 S | 676.21040 | 12.1 | 14.5 | 9.8 | 5.9 |
| 2-2-d1E02-1-0185 | C36 H32 N6 O6 S | 676.21040 | 20.3 | 13.3 | 8.0 | 5.1 |
| 2-2-d1E02-1-0186 | C38 H36 N4 O8 | 676.25331 | 11.2 | 16.8 | 8.9 | 3.2 |
| 2-2-d1E02-1-0187 | C40 H44 N4 O6 | 676.32609 | 9.0 | 16.4 | 2.9 | 13.3 |
| 2-2-d1E02-1-0188 | C33 H32 F N5 O8 S | 677.19556 | 2.1 | 1.4 | -0.6 | -2.1 |
| 2-2-d1E02-1-0189 | C37 H35 N5 O6 S | 677.23080 | 9.9 | 14.4 | 7.5 | 2.4 |
| 2-2-d1E02-1-0190 | C37 H35 N5 O6 S | 677.23080 | 11.6 | 15.6 | 10.8 | 4.3 |
| 2-2-d1E02-1-0191 | C33 H34 N4 O8 S2 | 678.18181 | 1.3 | 6.7 | 2.0 | -0.5 |
| 2-2-d1E02-1-0192 | C32 H34 N6 O9 S | 678.21080 | 1.0 | 0.0 | 0.0 | 0.2 |
| 2-2-d1E02-1-0193 | C36 H34 N6 O6 S | 678.22605 | 7.0 | 12.5 | 5.1 | 0.7 |
| 2-2-d1E02-1-0194 | C35 H33 F3 N4 O7 | 678.23013 | 14.2 | 11.5 | 9.3 | 1.9 |
| 2-2-d1E02-1-0195 | C35 H42 N4 O8 S | 678.27234 | 2.5 | 0.0 | -1.9 | 0.0 |
| 2-2-d1E02-1-0196 | C38 H42 N6 O6 | 678.31658 | 5.7 | 11.3 | 6.4 | 0.8 |
| 2-2-d1E02-1-0197 | C32 H33 N5 O10 S | 679.19481 | 0.0 | 1.7 | 0.0 | -5.5 |
| 2-2-d1E02-1-0198 | C33 H31 F3 N6 O7 | 680.22063 | 5.0 | 4.2 | 3.2 | -1.1 |
| 2-2-d1E02-1-0199 | C36 H36 N6 O6 S | 680.24170 | 6.3 | 12.4 | 6.2 | 3.2 |
| 2-2-d1E02-1-0200 | C34 H40 N4 O9 S | 680.25160 | 4.8 | 3.9 | 0.2 | -1.1 |
| 2-2-d1E02-1-0201 | C39 H41 F N4 O6 | 680.30101 | 8.3 | 11.4 | 10.2 | 2.7 |
| 2-2-d1E02-1-0202 | C31 H31 N5 O9 S2 | 681.15632 | 3.1 | 3.6 | 1.8 | -0.7 |
| 2-2-d1E02-1-0203 | C35 H35 N7 O6 S | 681.23695 | 8.1 | 8.6 | 3.9 | 0.9 |
| 2-2-d1E02-1-0204 | C32 H31 F N4 O8 S2 | 682.15673 | 3.0 | 3.3 | 2.6 | -0.8 |
| 2-2-d1E02-1-0205 | C36 H34 N4 O8 S | 682.20973 | 13.4 | 12.1 | 6.2 | 6.7 |
| 2-2-d1E02-1-0206 | C36 H34 N4 O8 S | 682.20973 | 3.0 | 2.5 | -0.5 | -1.1 |
| 2-2-d1E02-1-0207 | C34 H34 N8 O6 S | 682.23220 | 2.7 | 10.4 | 4.4 | 2.4 |
| 2-2-d1E02-1-0208 | C33 H33 F3 N6 O7 | 682.23628 | 4.6 | 6.9 | 4.6 | 1.3 |
| 2-2-d1E02-1-0209 | C38 H42 N4 O6 S | 682.28251 | 21.2 | 18.6 | 14.1 | 7.9 |
| 2-2-d1E02-1-0210 | C36 H41 F3 N4 O6 | 682.29782 | 5.9 | 5.8 | 0.9 | 0.1 |
| 2-2-d1E02-1-0211 | C31 H30 F N5 O10 S | 683.16974 | 0.0 | 0.0 | 0.0 | 0.0 |
| 2-2-d1E02-1-0212 | C35 H33 N5 O6 S2 | 683.18723 | 13.6 | 15.3 | 9.9 | 5.6 |
| 2-2-d1E02-1-0213 | C35 H33 N5 O8 S | 683.20498 | 29.6 | 27.6 | 11.8 | 9.7 |
| 2-2-d1E02-1-0214 | C35 H33 N5 O8 S | 683.20498 | 9.9 | 16.5 | 5.5 | 0.3 |
| 2-2-d1E02-1-0215 | C35 H33 N5 O8 S | 683.20498 | 0.0 | 0.0 | 0.0 | 0.0 |
| 2-2-d1E02-1-0216 | C36 H37 N5 O7 S | 683.24137 | 6.5 | 9.3 | 4.7 | 2.3 |
| 2-2-d1E02-1-0217 | C34 H32 N6 O8 S | 684.20023 | 6.7 | 11.9 | 2.9 | 0.1 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0218 | C36 H36 N4 O8 S | 684.22538 | 0.0 | 0.0 | 0.0 | -1.7 |
| 2-2-d1E02-1-0219 | C36 H36 N4 O8 S | 684.22538 | 0.0 | 0.0 | 0.0 | 0.0 |
| 2-2-d1E02-1-0220 | C35 H39 F3 N4 O7 | 684.27708 | 4.9 | 5.4 | -0.5 | 0.0 |
| 2-2-d1E02-1-0221 | C32 H30 F3 N5 O7 S | 685.18180 | 7.5 | 6.8 | 4.6 | 1.8 |
| 2-2-d1E02-1-0222 | C35 H35 N5 O8 S | 685.22063 | 1.5 | 2.0 | -0.2 | -1.0 |
| 2-2-d1E02-1-0223 | C37 H33 F3 N4 O6 | 686.23522 | 25.4 | 11.7 | 10.4 | 4.4 |
| 2-2-d1E02-1-0224 | C37 H33 F3 N4 O6 | 686.23522 | 24.8 | 23.6 | 8.7 | 8.0 |
| 2-2-d1E02-1-0225 | C37 H39 F N4 O6 S | 686.25743 | 10.0 | 14.4 | 6.8 | 3.7 |
| 2-2-d1E02-1-0226 | C33 H33 N7 O6 S2 | 687.19337 | 4.8 | 9.4 | 3.6 | 0.5 |
| 2-2-d1E02-1-0227 | C36 H32 F3 N5 O6 | 687.23047 | 15.8 | 13.9 | 6.8 | 3.8 |
| 2-2-d1E02-1-0228 | C36 H32 F3 N5 O6 | 687.23047 | 13.9 | 12.0 | 8.7 | 5.6 |
| 2-2-d1E02-1-0229 | C36 H32 F3 N5 O6 | 687.23047 | 18.0 | 13.6 | 7.3 | 2.7 |
| 2-2-d1E02-1-0230 | C35 H37 N5 O8 S | 687.23628 | 5.4 | 7.2 | 2.3 | 2.7 |
| 2-2-d1E02-1-0231 | C35 H36 N4 O9 S | 688.22030 | 5.2 | 1.2 | 1.2 | -2.2 |
| 2-2-d1E02-1-0232 | C34 H36 N6 O8 S | 688.23153 | 13.7 | 17.4 | 5.9 | 6.8 |
| 2-2-d1E02-1-0233 | C37 H35 F3 N4 O6 | 688.25087 | 15.0 | 17.0 | 9.2 | 2.3 |
| 2-2-d1E02-1-0234 | C37 H35 F3 N4 O6 | 688.25087 | 4.3 | 11.7 | 8.9 | -2.0 |
| 2-2-d1E02-1-0235 | C33 H35 N7 O8 S | 689.22678 | 9.5 | 15.0 | 5.9 | 2.9 |
| 2-2-d1E02-1-0236 | C36 H34 F3 N5 O6 | 689.24612 | 6.1 | 9.8 | 3.9 | 0.2 |
| 2-2-d1E02-1-0237 | C34 H34 N4 O8 S2 | 690.18181 | 0.0 | 0.0 | 0.0 | 0.0 |
| 2-2-d1E02-1-0238 | C33 H34 N6 O9 S | 690.21080 | 4.1 | 4.2 | 0.1 | 0.5 |
| 2-2-d1E02-1-0239 | C35 H38 N4 O9 S | 690.23595 | 10.8 | 11.2 | 11.1 | 9.1 |
| 2-2-d1E02-1-0240 | C39 H38 N4 O8 | 690.26896 | 11.8 | 16.3 | 4.1 | 0.7 |
| 2-2-d1E02-1-0241 | C33 H33 N5 O10 S | 691.19481 | -10.8 | 6.9 | 0.0 | -13.7 |
| 2-2-d1E02-1-0242 | C36 H36 F3 N5 O6 | 691.26177 | 12.5 | 8.8 | 14.8 | 0.8 |
| 2-2-d1E02-1-0243 | C34 H33 F N4 O9 S | 692.19523 | 2.0 | 3.1 | 2.0 | -0.1 |
| 2-2-d1E02-1-0244 | C33 H36 N6 O9 S | 692.22645 | 2.9 | 2.0 | -0.9 | 0.0 |
| 2-2-d1E02-1-0245 | C35 H35 F3 N6 O6 | 692.25702 | 1.8 | 9.6 | 5.0 | 2.3 |
| 2-2-d1E02-1-0246 | C36 H44 N4 O8 S | 692.28799 | 2.9 | 5.4 | 8.8 | -0.8 |
| 2-2-d1E02-1-0247 | C39 H44 N6 O6 | 692.33223 | 9.1 | 11.1 | 9.9 | 3.6 |
| 2-2-d1E02-1-0248 | C34 H34 F3 N7 O6 | 693.25227 | 5.7 | 8.3 | 3.5 | 0.8 |
| 2-2-d1E02-1-0249 | C38 H39 N5 O6 S | 693.26210 | 12.2 | 8.5 | 5.5 | 4.1 |
| 2-2-d1E02-1-0250 | C32 H31 F N6 O9 S | 694.18573 | 0.0 | 0.0 | 0.0 | 4.8 |
| 2-2-d1E02-1-0251 | C32 H34 N6 O8 S2 | 694.18795 | 3.5 | 3.1 | 0.1 | -0.8 |
| 2-2-d1E02-1-0252 | C35 H33 F3 N4 O6 S | 694.20729 | 10.7 | 20.8 | 6.9 | -0.3 |
| 2-2-d1E02-1-0253 | C38 H35 F N4 O8 | 694.24389 | 8.2 | 13.7 | 11.6 | 4.6 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0254 | C36 H37 F3 N4 O7 | 694.26143 | 10.7 | 8.6 | 2.8 | 1.3 |
| 2-2-d1E02-1-0255 | C35 H42 N4 O9 S | 694.26725 | 6.2 | 4.0 | -1.5 | 2.5 |
| 2-2-d1E02-1-0256 | C32 H33 N5 O9 S2 | 695.17197 | 3.3 | 1.5 | 3.5 | 3.5 |
| 2-2-d1E02-1-0257 | C35 H33 N7 O7 S | 695.21622 | 5.0 | 9.9 | 2.2 | 2.7 |
| 2-2-d1E02-1-0258 | C34 H32 F3 N5 O8 | 695.22030 | 9.9 | 10.0 | 0.7 | 3.6 |
| 2-2-d1E02-1-0259 | C32 H33 F N6 O9 S | 696.20138 | 2.7 | 1.7 | 0.7 | -0.4 |
| 2-2-d1E02-1-0260 | C37 H36 N4 O8 S | 696.22538 | 4.7 | 7.9 | 4.9 | -0.2 |
| 2-2-d1E02-1-0261 | C37 H36 N4 O8 S | 696.22538 | 1.0 | 0.0 | 3.2 | 3.8 |
| 2-2-d1E02-1-0262 | C35 H41 F N4 O8 S | 696.26291 | 1.7 | 1.8 | 3.0 | 0.6 |
| 2-2-d1E02-1-0263 | C38 H41 F N6 O6 | 696.30716 | 4.7 | 10.6 | 7.9 | 2.0 |
| 2-2-d1E02-1-0264 | C36 H35 N5 O8 S | 697.22063 | 12.8 | 14.2 | 6.4 | 1.7 |
| 2-2-d1E02-1-0265 | C36 H35 N5 O8 S | 697.22063 | 10.3 | 12.1 | 8.4 | -1.5 |
| 2-2-d1E02-1-0266 | C36 H35 N5 O8 S | 697.22063 | 12.1 | 0.0 | 0.3 | 6.4 |
| 2-2-d1E02-1-0267 | C35 H35 N7 O7 S | 697.23187 | 4.2 | 10.1 | 3.4 | 1.7 |
| 2-2-d1E02-1-0268 | C33 H33 F3 N6 O6 S | 698.21344 | 6.8 | 9.3 | 6.6 | -0.3 |
| 2-2-d1E02-1-0269 | C37 H38 N4 O8 S | 698.24103 | 7.3 | -0.6 | -3.7 | -5.3 |
| 2-2-d1E02-1-0270 | C37 H38 N4 O8 S | 698.24103 | 3.6 | -0.6 | 3.4 | -6.5 |
| 2-2-d1E02-1-0271 | C34 H39 F N4 O9 S | 698.24218 | 3.8 | 3.6 | -0.9 | -1.1 |
| 2-2-d1E02-1-0272 | C31 H30 F N5 O9 S2 | 699.14690 | 2.2 | 5.6 | 2.1 | 1.1 |
| 2-2-d1E02-1-0273 | C36 H37 N5 O6 S2 | 699.21853 | 8.1 | 10.8 | 8.0 | 1.7 |
| 2-2-d1E02-1-0274 | C36 H37 N5 O8 S | 699.23628 | 2.2 | 2.1 | -0.7 | -0.2 |
| 2-2-d1E02-1-0275 | C34 H32 N6 O7 S2 | 700.17739 | 5.6 | 11.0 | 7.1 | 0.3 |
| 2-2-d1E02-1-0276 | C36 H33 F N4 O8 S | 700.20031 | 5.0 | 6.7 | 3.4 | -0.2 |
| 2-2-d1E02-1-0277 | C36 H33 F N4 O8 S | 700.20031 | 2.6 | 0.6 | -1.1 | -1.9 |
| 2-2-d1E02-1-0278 | C37 H40 N4 O8 S | 700.25668 | 3.8 | 5.9 | 3.3 | 2.3 |
| 2-2-d1E02-1-0279 | C35 H32 F N5 O8 S | 701.19556 | 12.3 | 13.0 | 5.5 | 2.6 |
| 2-2-d1E02-1-0280 | C35 H32 F N5 O8 S | 701.19556 | 10.3 | 15.4 | 5.2 | -1.2 |
| 2-2-d1E02-1-0281 | C35 H32 F N5 O8 S | 701.19556 | 0.0 | 0.0 | 0.0 | 0.0 |
| 2-2-d1E02-1-0282 | C39 H35 N5 O6 S | 701.23080 | 24.7 | 13.8 | 11.4 | 8.0 |
| 2-2-d1E02-1-0283 | C39 H35 N5 O6 S | 701.23080 | 22.5 | 22.9 | 12.1 | 3.4 |
| 2-2-d1E02-1-0284 | C36 H39 N5 O8 S | 701.25193 | 7.9 | 6.6 | 4.6 | 2.2 |
| 2-2-d1E02-1-0285 | C34 H34 N6 O9 S | 702.21080 | 2.6 | 0.0 | 0.0 | 1.7 |
| 2-2-d1E02-1-0286 | C36 H35 F N4 O8 S | 702.21596 | 7.9 | 3.7 | 2.4 | -1.7 |
| 2-2-d1E02-1-0287 | C36 H35 F N4 O8 S | 702.21596 | 1.2 | 1.0 | -3.9 | -3.8 |
| 2-2-d1E02-1-0288 | C38 H34 N6 O6 S | 702.22605 | 17.0 | 18.6 | 9.4 | 7.4 |
| 2-2-d1E02-1-0289 | C38 H34 N6 O6 S | 702.22605 | 11.4 | 15.7 | 10.4 | 6.2 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0290 | C38 H34 N6 O6 S | 702.22605 | 22.7 | 21.8 | 9.3 | 7.7 |
| 2-2-d1E02-1-0291 | C35 H38 N6 O8 S | 702.24718 | 3.0 | 3.6 | 0.6 | -1.5 |
| 2-2-d1E02-1-0292 | C35 H34 F N5 O8 S | 703.21121 | 2.6 | 3.8 | 0.0 | -0.2 |
| 2-2-d1E02-1-0293 | C34 H37 N7 O8 S | 703.24243 | 7.4 | 15.3 | 9.0 | 1.2 |
| 2-2-d1E02-1-0294 | C35 H36 N4 O8 S2 | 704.19746 | 4.8 | 0.0 | 0.0 | 0.0 |
| 2-2-d1E02-1-0295 | C34 H36 N6 O9 S | 704.22645 | 3.3 | 3.5 | -1.0 | -1.4 |
| 2-2-d1E02-1-0296 | C36 H40 N4 O9 S | 704.25160 | 7.8 | 8.2 | 5.0 | 3.9 |
| 2-2-d1E02-1-0297 | C38 H39 F3 N4 O6 | 704.28217 | 10.4 | 15.2 | 7.2 | 2.8 |
| 2-2-d1E02-1-0298 | C40 H44 N6 O6 | 704.33223 | 9.3 | 14.3 | 9.5 | 3.3 |
| 2-2-d1E02-1-0299 | C33 H31 N5 O9 S2 | 705.15632 | 1.6 | 4.5 | 5.1 | -0.3 |
| 2-2-d1E02-1-0300 | C34 H35 N5 O10 S | 705.21046 | 0.0 | 0.0 | 0.0 | 0.0 |
| 2-2-d1E02-1-0301 | C35 H36 F N5 O8 S | 705.22686 | 5.1 | 3.7 | 3.2 | 3.4 |
| 2-2-d1E02-1-0302 | C35 H38 N4 O8 S2 | 706.21311 | 10.1 | 9.4 | 5.8 | 6.2 |
| 2-2-d1E02-1-0303 | C34 H35 F N6 O8 S | 706.22211 | 2.3 | 2.5 | -0.8 | -0.1 |
| 2-2-d1E02-1-0304 | C35 H33 F3 N6 O7 | 706.23628 | 6.2 | 8.1 | 2.0 | 1.4 |
| 2-2-d1E02-1-0305 | C38 H38 N6 O6 S | 706.25735 | 8.8 | 12.4 | 8.1 | 3.3 |
| 2-2-d1E02-1-0306 | C33 H33 N5 O9 S2 | 707.17197 | 0.0 | 0.0 | 0.0 | 0.0 |
| 2-2-d1E02-1-0307 | C37 H33 N5 O8 S | 707.20498 | 19.5 | 17.0 | 9.6 | 6.5 |
| 2-2-d1E02-1-0308 | C33 H34 F N7 O8 S | 707.21736 | 8.0 | 15.5 | 9.3 | 2.8 |
| 2-2-d1E02-1-0309 | C37 H37 N7 O6 S | 707.25260 | 9.9 | 8.6 | 15.3 | -2.1 |
| 2-2-d1E02-1-0310 | C34 H33 F N4 O8 S2 | 708.17238 | 5.2 | 0.0 | 1.5 | 3.7 |
| 2-2-d1E02-1-0311 | C33 H36 N6 O8 S2 | 708.20360 | 4.2 | 4.1 | -0.2 | -0.9 |
| 2-2-d1E02-1-0312 | C38 H36 N4 O8 S | 708.22538 | 5.8 | 7.7 | 4.9 | 0.6 |
| 2-2-d1E02-1-0313 | C38 H36 N4 O8 S | 708.22538 | 11.9 | 10.7 | 12.1 | 7.7 |
| 2-2-d1E02-1-0314 | C35 H37 F N4 O9 S | 708.22653 | 0.0 | 0.0 | 0.0 | 0.0 |
| 2-2-d1E02-1-0315 | C36 H36 N8 O6 S | 708.24785 | 4.5 | 9.5 | 5.0 | 2.1 |
| 2-2-d1E02-1-0316 | C35 H35 F3 N6 O7 | 708.25193 | 3.6 | 8.1 | 4.7 | -0.3 |
| 2-2-d1E02-1-0317 | C33 H32 F N5 O10 S | 709.18539 | 0.0 | 0.0 | 0.0 | 0.0 |
| 2-2-d1E02-1-0318 | C37 H35 N5 O8 S | 709.22063 | 9.3 | 13.1 | 5.6 | 3.7 |
| 2-2-d1E02-1-0319 | C37 H35 N5 O8 S | 709.22063 | 0.9 | 2.7 | 26.0 | -59.3 |
| 2-2-d1E02-1-0320 | C37 H35 N5 O8 S | 709.22063 | 10.4 | 10.6 | 3.2 | -2.8 |
| 2-2-d1E02-1-0321 | C34 H39 N5 O8 S2 | 709.22400 | 3.2 | 1.7 | 0.7 | 0.6 |
| 2-2-d1E02-1-0322 | C38 H39 N5 O7 S | 709.25702 | 9.4 | 14.1 | 8.7 | 3.8 |
| 2-2-d1E02-1-0323 | C36 H37 F3 N4 O6 S | 710.23859 | 11.9 | 15.0 | 9.4 | 6.6 |
| 2-2-d1E02-1-0324 | C34 H32 F3 N5 O7 S | 711.19745 | 12.9 | 4.0 | 1.8 | 5.5 |
| 2-2-d1E02-1-0325 | C33 H37 N5 O9 S2 | 711.20327 | 2.8 | 2.0 | 0.3 | 0.3 |

(continued)

[Table 11-3-2]

| | | | AS-MS(%) | | | |
|---|---|---|---|---|---|---|
| ID | Molecular Formula | Exact Mass | N1 | N2 | N3 | N4 |
| 2-2-d1E02-1-0326 | C32 H33 F N6 O8 S2 | 712.17853 | 3.5 | 3.4 | -0.3 | -1.0 |
| 2-2-d1E02-1-0327 | C39 H35 F3 N4 O6 | 712.25087 | 21.3 | 14.6 | 7.1 | 2.7 |
| 2-2-d1E02-1-0328 | C39 H35 F3 N4 O6 | 712.25087 | 0.0 | 9.7 | 0.0 | -0.3 |
| 2-2-d1E02-1-0329 | C35 H35 N7 O6 S2 | 713.20902 | 6.6 | 11.2 | 5.9 | 3.8 |
| 2-2-d1E02-1-0330 | C38 H34 F3 N5 O6 | 713.24612 | 19.1 | 14.8 | 9.9 | 6.0 |
| 2-2-d1E02-1-0331 | C38 H34 F3 N5 O6 | 713.24612 | 21.4 | 15.8 | 12.9 | 0.1 |
| 2-2-d1E02-1-0332 | C38 H34 F3 N5 O6 | 713.24612 | 18.1 | 13.0 | 8.7 | 5.8 |
| 2-2-d1E02-1-0333 | C37 H39 N5 O8 S | 713.25193 | 24.9 | 16.0 | 6.9 | 2.8 |
| 2-2-d1E02-1-0334 | C36 H34 N4 O10 S | 714.19956 | 10.2 | 9.3 | 5.1 | -0.5 |
| 2-2-d1E02-1-0335 | C36 H38 N6 O8 S | 714.24718 | 0.0 | -16.2 | 14.7 | 10.7 |
| 2-2-d1E02-1-0336 | C38 H42 N4 O8 S | 714.27234 | 4.8 | 4.4 | 2.5 | 0.4 |
| 2-2-d1E02-1-0337 | C35 H33 N5 O8 S2 | 715.17705 | 4.2 | 5.0 | 2.5 | 2.3 |
| 2-2-d1E02-1-0338 | C35 H33 N5 O8 S2 | 715.17705 | 3.1 | 5.1 | 1.9 | 1.1 |
| 2-2-d1E02-1-0339 | C35 H37 N7 O8 S | 715.24243 | 16.2 | 19.9 | 12.1 | 5.2 |
| 2-2-d1E02-1-0340 | C34 H32 N6 O8 S2 | 716.17230 | 4.5 | 2.4 | -0.3 | 0.2 |
| 2-2-d1E02-1-0341 | C33 H31 F3 N4 O9 S | 716.17638 | 4.7 | 4.3 | 3.2 | -1.5 |
| 2-2-d1E02-1-0342 | C35 H36 N6 O9 S | 716.22645 | 0.0 | 0.0 | 0.0 | 0.0 |
| 2-2-d1E02-1-0343 | C37 H40 N4 O9 S | 716.25160 | 14.6 | 11.4 | 3.8 | 4.8 |
| 2-2-d1E02-1-0344 | C38 H38 F3 N5 O6 | 717.27742 | 0.0 | -13.9 | 0.0 | 0.0 |
| 2-2-d1E02-1-0345 | C37 H33 F3 N4 O8 | 718.22505 | 2.8 | 2.9 | 3.0 | -0.8 |
| 2-2-d1E02-1-0346 | C35 H38 N6 O9 S | 718.24210 | 3.4 | 2.2 | 0.3 | 1.0 |
| 2-2-d1E02-1-0347 | C37 H39 F N4 O8 S | 718.24726 | 4.2 | 5.8 | 4.5 | 1.2 |
| 2-2-d1E02-1-0348 | C37 H37 F3 N6 O6 | 718.27267 | 8.6 | 10.5 | 5.1 | -0.5 |
| 2-2-d1E02-1-0349 | C41 H46 N6 O6 | 718.34788 | 7.8 | 16.3 | 7.6 | 3.2 |
| 2-2-d1E02-1-0350 | C36 H36 F3 N7 O6 | 719.26792 | 7.6 | 9.1 | 6.2 | 3.7 |
| 2-2-d1E02-1-0351 | C40 H41 N5 O6 S | 719.27775 | 10.8 | 14.3 | 5.2 | 5.7 |
| 2-2-d1E02-1-0352 | C34 H33 F N6 O9 S | 720.20138 | 0.0 | 0.0 | 0.0 | 1.3 |
| 2-2-d1E02-1-0353 | C34 H36 N6 O8 S2 | 720.20360 | 0.0 | 0.0 | 0.0 | 0.0 |
| 2-2-d1E02-1-0354 | C36 H40 N4 O8 S2 | 720.22876 | 7.5 | 8.2 | 3.6 | 2.8 |
| 2-2-d1E02-1-0355 | C34 H39 F3 N4 O8 S | 720.24407 | 2.5 | 1.3 | -0.6 | -1.9 |
| 2-2-d1E02-1-0356 | C38 H39 F3 N4 O7 | 720.27708 | 8.5 | 19.1 | 1.5 | 25.0 |
| 2-2-d1E02-1-0357 | C33 H31 N5 O8 S3 | 721.13348 | 4.2 | 3.4 | 1.7 | 0.9 |
| 2-2-d1E02-1-0358 | C34 H35 N5 O9 S2 | 721.18762 | 6.2 | 2.3 | -1.6 | 2.2 |
| 2-2-d1E02-1-0359 | C32 H30 N6 O10 S2 | 722.14648 | 2.0 | 5.4 | 10.2 | -4.5 |
| 2-2-d1E02-1-0360 | C34 H35 F N6 O9 S | 722.21703 | 3.2 | 4.4 | 0.0 | -1.1 |
| 2-2-d1E02-1-0361 | C33 H37 F3 N4 O9 S | 722.22333 | 2.4 | 2.5 | 0.3 | -2.4 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0362 | C39 H38 N4 O8 S | 722.24103 | 3.2 | 5.1 | 6.6 | -0.2 |
| 2-2-d1E02-1-0363 | C39 H38 N4 O8 S | 722.24103 | 9.1 | 10.6 | -1.5 | 2.2 |
| 2-2-d1E02-1-0364 | C40 H43 F N6 O6 | 722.32281 | 7.8 | 10.7 | 9.1 | 3.8 |
| 2-2-d1E02-1-0365 | C38 H37 N5 O8 S | 723.23628 | 8.5 | 4.7 | 0.1 | 2.1 |
| 2-2-d1E02-1-0366 | C38 H37 N5 O8 S | 723.23628 | 15.6 | 17.5 | 5.6 | 0.7 |
| 2-2-d1E02-1-0367 | C38 H37 N5 O8 S | 723.23628 | 0.0 | 0.0 | 0.0 | 5.9 |
| 2-2-d1E02-1-0368 | C35 H37 F N4 O8 S2 | 724.20368 | 10.4 | 7.5 | 12.6 | 3.1 |
| 2-2-d1E02-1-0369 | C35 H35 F3 N6 O6 S | 724.22909 | 9.0 | 12.0 | 8.8 | -2.2 |
| 2-2-d1E02-1-0370 | C33 H32 F N5 O9 S2 | 725.16255 | 0.0 | 0.0 | 0.0 | 0.0 |
| 2-2-d1E02-1-0371 | C38 H39 N5 O6 S2 | 725.23418 | 14.1 | 12.9 | 8.5 | 6.2 |
| 2-2-d1E02-1-0372 | C35 H33 F3 N4 O8 S | 726.19712 | 4.0 | 4.1 | 3.1 | 2.9 |
| 2-2-d1E02-1-0373 | C35 H33 F3 N4 O8 S | 726.19712 | 2.5 | 3.7 | 2.8 | 1.2 |
| 2-2-d1E02-1-0374 | C38 H35 F N4 O8 S | 726.21596 | 3.6 | 7.8 | 4.3 | 0.7 |
| 2-2-d1E02-1-0375 | C38 H35 F N4 O8 S | 726.21596 | 16.7 | 23.9 | 11.4 | 10.6 |
| 2-2-d1E02-1-0376 | C39 H42 N4 O8 S | 726.27234 | 6.6 | 3.4 | 4.8 | -0.3 |
| 2-2-d1E02-1-0377 | C34 H32 F3 N5 O8 S | 727.19237 | 2.2 | 3.0 | 2.0 | -2.0 |
| 2-2-d1E02-1-0378 | C37 H34 F N5 O8 S | 727.21121 | 7.1 | 11.1 | 2.0 | 0.5 |
| 2-2-d1E02-1-0379 | C37 H34 F N5 O8 S | 727.21121 | 15.5 | 15.1 | 9.4 | -0.1 |
| 2-2-d1E02-1-0380 | C37 H34 F N5 O8 S | 727.21121 | 0.0 | 0.0 | 0.0 | 0.0 |
| 2-2-d1E02-1-0381 | C38 H41 N5 O8 S | 727.26758 | 0.0 | 0.0 | 0.0 | 0.0 |
| 2-2-d1E02-1-0382 | C37 H36 N4 O10 S | 728.21521 | 5.4 | 8.3 | 2.4 | 2.3 |
| 2-2-d1E02-1-0383 | C37 H40 N6 O8 S | 728.26283 | 0.0 | 0.0 | 0.0 | 6.5 |
| 2-2-d1E02-1-0384 | C36 H39 N7 O8 S | 729.25808 | 12.1 | 15.7 | 2.1 | 6.1 |
| 2-2-d1E02-1-0385 | C38 H42 N4 O9 S | 730.26725 | 10.0 | 11.2 | 7.1 | 4.8 |
| 2-2-d1E02-1-0386 | C40 H41 F3 N4 O6 | 730.29782 | 14.0 | 13.3 | 6.1 | 4.5 |
| 2-2-d1E02-1-0387 | C35 H33 N5 O9 S2 | 731.17197 | 3.9 | 7.2 | 0.5 | 0.9 |
| 2-2-d1E02-1-0388 | C37 H38 F N5 O8 S | 731.24251 | 0.0 | 0.0 | 0.0 | 0.0 |
| 2-2-d1E02-1-0389 | C33 H31 F3 N4 O8 S2 | 732.15354 | 3.0 | 4.8 | 3.3 | 1.1 |
| 2-2-d1E02-1-0390 | C36 H33 F N4 O10 S | 732.19014 | 6.5 | 0.0 | 2.9 | 6.1 |
| 2-2-d1E02-1-0391 | C37 H40 N4 O8 S2 | 732.22876 | 13.3 | 13.1 | 4.7 | 2.6 |
| 2-2-d1E02-1-0392 | C36 H37 F N6 O8 S | 732.23776 | 3.7 | 8.7 | 0.4 | -1.8 |
| 2-2-d1E02-1-0393 | C33 H31 N7 O9 S2 | 733.16247 | 3.8 | 2.4 | 1.6 | 0.1 |
| 2-2-d1E02-1-0394 | C32 H30 F3 N5 O10 S | 733.16655 | 0.0 | 2.4 | 5.8 | 7.9 |
| 2-2-d1E02-1-0395 | C39 H35 N5 O8 S | 733.22063 | 15.7 | 14.2 | 13.8 | 9.8 |
| 2-2-d1E02-1-0396 | C35 H36 F N7 O8 S | 733.23301 | 9.0 | 16.7 | 10.1 | 2.3 |
| 2-2-d1E02-1-0397 | C35 H38 N6 O8 S2 | 734.21925 | 3.6 | 0.0 | 0.0 | 0.0 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0398 | C37 H39 F N4 O9 S | 734.24218 | 8.4 | 6.3 | 2.8 | 2.1 |
| 2-2-d1E02-1-0399 | C33 H33 N7 O9 S2 | 735.17812 | 5.8 | 4.7 | 2.1 | 2.4 |
| 2-2-d1E02-1-0400 | C36 H41 N5 O8 S2 | 735.23965 | 2.7 | 2.0 | 1.1 | -0.3 |
| 2-2-d1E02-1-0401 | C39 H41 N7 O6 S | 735.28390 | 7.8 | 13.7 | 6.6 | 5.3 |
| 2-2-d1E02-1-0402 | C38 H39 F3 N4 O6 S | 736.25424 | 12.8 | 12.1 | 9.6 | 1.7 |
| 2-2-d1E02-1-0403 | C35 H39 N5 O9 S2 | 737.21892 | 5.3 | 4.8 | 0.7 | 1.6 |
| 2-2-d1E02-1-0404 | C32 H30 N6 O9 S3 | 738.12364 | 8.1 | 5.1 | 3.1 | 2.0 |
| 2-2-d1E02-1-0405 | C34 H35 F N6 O8 S2 | 738.19418 | 4.0 | 0.0 | -0.9 | -8.5 |
| 2-2-d1E02-1-0406 | C37 H33 N5 O8 S2 | 739.17705 | 5.2 | 8.2 | 6.1 | 2.2 |
| 2-2-d1E02-1-0407 | C37 H33 N5 O8 S2 | 739.17705 | 6.5 | 7.1 | 5.7 | 2.7 |
| 2-2-d1E02-1-0408 | C36 H32 N6 O8 S2 | 740.17230 | 7.0 | 10.1 | 4.5 | 0.6 |
| 2-2-d1E02-1-0409 | C36 H32 N6 O8 S2 | 740.17230 | 10.9 | 11.6 | 7.2 | 3.2 |
| 2-2-d1E02-1-0410 | C36 H32 N6 O8 S2 | 740.17230 | 7.0 | 10.1 | 4.5 | 0.6 |
| 2-2-d1E02-1-0411 | C38 H36 N4 O10 S | 740.21521 | 16.7 | 19.7 | 5.1 | 13.1 |
| 2-2-d1E02-1-0412 | C40 H44 N4 O8 S | 740.28799 | 8.1 | 5.2 | 11.8 | -0.8 |
| 2-2-d1E02-1-0413 | C37 H35 N5 O8 S2 | 741.19270 | 4.4 | 3.3 | 1.9 | 3.2 |
| 2-2-d1E02-1-0414 | C37 H35 N5 O8 S2 | 741.19270 | 2.5 | 5.1 | 1.1 | 1.1 |
| 2-2-d1E02-1-0415 | C36 H34 N6 O8 S2 | 742.18795 | 4.4 | 4.1 | 0.9 | -0.6 |
| 2-2-d1E02-1-0416 | C35 H33 F3 N4 O9 S | 742.19203 | 6.3 | 5.3 | 0.1 | 0.8 |
| 2-2-d1E02-1-0417 | C38 H42 N6 O8 S | 742.27848 | 15.6 | 15.6 | 16.8 | 14.0 |
| 2-2-d1E02-1-0418 | C33 H31 F3 N6 O9 S | 744.18253 | 0.6 | 4.2 | 2.9 | -0.1 |
| 2-2-d1E02-1-0419 | C36 H36 N6 O8 S2 | 744.20360 | 5.1 | 7.4 | 3.4 | -0.7 |
| 2-2-d1E02-1-0420 | C39 H35 F3 N4 O8 | 744.24070 | 12.9 | 15.4 | 4.7 | 12.1 |
| 2-2-d1E02-1-0421 | C39 H41 F N4 O8 S | 744.26291 | 7.9 | 7.3 | 4.0 | 0.1 |
| 2-2-d1E02-1-0422 | C35 H35 N7 O8 S2 | 745.19885 | 7.1 | 7.9 | 3.2 | 0.9 |
| 2-2-d1E02-1-0423 | C34 H34 N8 O8 S2 | 746.19410 | 8.5 | 15.7 | 8.1 | 2.7 |
| 2-2-d1E02-1-0424 | C33 H33 F3 N6 O9 S | 746.19818 | 4.0 | 3.7 | 2.0 | -1.0 |
| 2-2-d1E02-1-0425 | C38 H42 N4 O8 S2 | 746.24441 | 13.9 | 12.5 | 1.8 | 0.7 |
| 2-2-d1E02-1-0426 | C36 H41 F3 N4 O8 S | 746.25972 | 0.0 | 0.0 | 0.5 | 0.6 |
| 2-2-d1E02-1-0427 | C39 H41 F3 N6 O6 | 746.30397 | 13.7 | 15.1 | 4.7 | 0.3 |
| 2-2-d1E02-1-0428 | C35 H33 N5 O8 S3 | 747.14913 | 0.5 | 4.9 | 1.8 | 0.8 |
| 2-2-d1E02-1-0429 | C36 H37 N5 O9 S2 | 747.20327 | 5.4 | 5.7 | 3.6 | 2.2 |
| 2-2-d1E02-1-0430 | C34 H32 N6 O10 S2 | 748.16213 | 0.0 | 0.0 | 3.8 | 0.0 |
| 2-2-d1E02-1-0431 | C35 H39 F3 N4 O9 S | 748.23898 | 3.3 | 4.7 | 1.4 | -1.2 |
| 2-2-d1E02-1-0432 | C32 H30 F3 N5 O9 S2 | 749.14370 | 11.9 | 9.4 | 2.0 | -3.2 |
| 2-2-d1E02-1-0433 | C37 H33 F3 N4 O8 S | 750.19712 | 6.4 | 4.9 | 3.1 | 4.2 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0434 | C37 H33 F3 N4 O8 S | 750.19712 | 12.4 | 4.7 | 10.0 | -1.4 |
| 2-2-d1E02-1-0435 | C37 H39 F N4 O8 S2 | 750.21933 | 9.6 | 10.5 | 3.2 | 0.5 |
| 2-2-d1E02-1-0436 | C33 H33 N7 O8 S3 | 751.15527 | 1.5 | 2.7 | 1.6 | 0.9 |
| 2-2-d1E02-1-0437 | C36 H32 F3 N5 O8 S | 751.19237 | 9.8 | 8.8 | 2.1 | 1.3 |
| 2-2-d1E02-1-0438 | C36 H32 F3 N5 O8 S | 751.19237 | 1.9 | 0.3 | 2.3 | 1.3 |
| 2-2-d1E02-1-0439 | C36 H32 F3 N5 O8 S | 751.19237 | 0.0 | 0.0 | 0.0 | 0.0 |
| 2-2-d1E02-1-0440 | C37 H35 F3 N4 O8 S | 752.21277 | 1.5 | 5.9 | 1.0 | -7.5 |
| 2-2-d1E02-1-0441 | C37 H35 F3 N4 O8 S | 752.21277 | 10.5 | 4.4 | 5.8 | 0.5 |
| 2-2-d1E02-1-0442 | C36 H34 F3 N5 O8 S | 753.20802 | 4.6 | 2.0 | -0.3 | -1.0 |
| 2-2-d1E02-1-0443 | C39 H38 N4 O10 S | 754.23086 | 0.0 | -1.6 | 7.1 | 2.4 |
| 2-2-d1E02-1-0444 | C36 H36 F3 N5 O8 S | 755.22367 | 5.1 | 5.9 | 3.7 | 0.6 |
| 2-2-d1E02-1-0445 | C35 H35 F3 N6 O8 S | 756.21892 | 9.5 | 4.5 | 0.5 | 0.6 |
| 2-2-d1E02-1-0446 | C39 H44 N6 O8 S | 756.29413 | 11.7 | 11.4 | 11.4 | 5.4 |
| 2-2-d1E02-1-0447 | C34 H34 F3 N7 O8 S | 757.21417 | 10.0 | 16.7 | 9.1 | 7.1 |
| 2-2-d1E02-1-0448 | C38 H39 N5 O8 S2 | 757.22400 | 6.0 | 6.8 | 1.8 | 2.0 |
| 2-2-d1E02-1-0449 | C35 H33 F3 N4 O8 S2 | 758.16919 | 3.1 | 8.1 | 4.2 | 5.4 |
| 2-2-d1E02-1-0450 | C38 H35 F N4 O10 S | 758.20579 | 15.2 | 14.6 | 5.0 | 4.2 |
| 2-2-d1E02-1-0451 | C36 H37 F3 N4 O9 S | 758.22333 | 3.6 | 5.5 | 0.2 | -8.8 |
| 2-2-d1E02-1-0452 | C35 H33 N7 O9 S2 | 759.17812 | 0.0 | 0.0 | 4.8 | 0.0 |
| 2-2-d1E02-1-0453 | C34 H32 F3 N5 O10 S | 759.18220 | 0.0 | 0.0 | 0.0 | 0.0 |
| 2-2-d1E02-1-0454 | C38 H41 F N6 O8 S | 760.26906 | 15.8 | 18.0 | 14.2 | 11.9 |
| 2-2-d1E02-1-0455 | C35 H35 N7 O9 S2 | 761.19377 | 3.8 | 6.5 | -0.7 | 1.0 |
| 2-2-d1E02-1-0456 | C41 H43 N7 O6 S | 761.29955 | 9.6 | 15.3 | 9.7 | 6.2 |
| 2-2-d1E02-1-0457 | C33 H33 F3 N6 O8 S2 | 762.17534 | 4.0 | 4.5 | 0.7 | -0.7 |
| 2-2-d1E02-1-0458 | C36 H37 N5 O8 S3 | 763.18043 | 6.3 | 6.6 | 5.2 | 2.7 |
| 2-2-d1E02-1-0459 | C34 H32 N6 O9 S3 | 764.13929 | 4.1 | 5.9 | 5.4 | 2.5 |
| 2-2-d1E02-1-0460 | C39 H35 N5 O8 S2 | 765.19270 | 6.1 | 9.5 | 4.8 | 0.6 |
| 2-2-d1E02-1-0461 | C39 H35 N5 O8 S2 | 765.19270 | 0.0 | 0.0 | 0.0 | 0.0 |
| 2-2-d1E02-1-0462 | C38 H34 N6 O8 S2 | 766.18795 | 17.9 | 11.2 | 8.6 | 2.8 |
| 2-2-d1E02-1-0463 | C38 H34 N6 O8 S2 | 766.18795 | 9.1 | 7.0 | 4.7 | 1.5 |
| 2-2-d1E02-1-0464 | C38 H34 N6 O8 S2 | 766.18795 | 0.0 | 0.0 | 6.6 | 10.4 |
| 2-2-d1E02-1-0465 | C38 H39 F3 N4 O8 S | 768.24407 | 8.8 | 7.3 | 8.6 | 5.9 |
| 2-2-d1E02-1-0466 | C40 H44 N6 O8 S | 768.29413 | 3.0 | 3.8 | 1.6 | 0.4 |
| 2-2-d1E02-1-0467 | C35 H33 F3 N6 O9 S | 770.19818 | 0.0 | 3.3 | 9.4 | 4.6 |
| 2-2-d1E02-1-0468 | C38 H38 N6 O8 S2 | 770.21925 | 8.9 | 10.5 | 4.2 | 3.2 |
| 2-2-d1E02-1-0469 | C37 H33 N5 O10 S2 | 771.16688 | 0.0 | 3.7 | 3.3 | -0.5 |

(continued)

| [Table 11-3-2] | | | | | | |
|---|---|---|---|---|---|---|
| | | | AS-MS(%) | | | |
| ID | Molecular Formula | Exact Mass | N1 | N2 | N3 | N4 |
| 2-2-d1E02-1-0470 | C37 H37 N7 O8 S2 | 771.21450 | 8.1 | 2.0 | 6.4 | -0.9 |
| 2-2-d1E02-1-0471 | C36 H36 N8 O8 S2 | 772.20975 | 12.4 | 15.2 | 8.7 | 4.0 |
| 2-2-d1E02-1-0472 | C35 H35 F3 N6 O9 S | 772.21383 | 6.7 | 4.6 | 0.9 | 0.7 |
| 2-2-d1E02-1-0473 | C41 H43 F3 N6 O6 | 772.31962 | 15.3 | 13.0 | 13.5 | 6.9 |
| 2-2-d1E02-1-0474 | C38 H39 N5 O9 S2 | 773.21892 | 6.1 | 7.8 | 6.5 | 2.1 |
| 2-2-d1E02-1-0475 | C36 H37 F3 N4 O8 S2 | 774.20049 | 6.9 | 6.4 | 8.4 | -2.1 |
| 2-2-d1E02-1-0476 | C34 H32 F3 N5 O9 S2 | 775.15935 | 0.0 | 0.0 | 0.0 | 0.0 |
| 2-2-d1E02-1-0477 | C39 H35 F3 N4 O8 S | 776.21277 | 19.5 | 12.6 | -0.3 | 2.5 |
| 2-2-d1E02-1-0478 | C39 H35 F3 N4 O8 S | 776.21277 | 0.0 | 0.0 | 0.0 | 16.3 |
| 2-2-d1E02-1-0479 | C35 H35 N7 O8 S3 | 777.17092 | 7.9 | 8.4 | 2.7 | -0.5 |
| 2-2-d1E02-1-0480 | C38 H34 F3 N5 O8 S | 777.20802 | 11.0 | 11.4 | 8.9 | 4.3 |
| 2-2-d1E02-1-0481 | C38 H34 F3 N5 O8 S | 777.20802 | 5.4 | 7.8 | 4.2 | 2.2 |
| 2-2-d1E02-1-0482 | C38 H34 F3 N5 O8 S | 777.20802 | 0.0 | 0.0 | 0.0 | 0.0 |
| 2-2-d1E02-1-0483 | C38 H38 F3 N5 O8 S | 781.23932 | 3.9 | 0.0 | 5.6 | -0.4 |
| 2-2-d1E02-1-0484 | C37 H33 F3 N4 O10 S | 782.18695 | 9.1 | 8.4 | 1.3 | -3.2 |
| 2-2-d1E02-1-0485 | C37 H37 F3 N6 O8 S | 782.23457 | 5.9 | 2.3 | -0.2 | 0.5 |
| 2-2-d1E02-1-0486 | C41 H46 N6 O8 S | 782.30978 | 8.0 | 5.3 | 5.7 | 0.9 |
| 2-2-d1E02-1-0487 | C36 H36 F3 N7 O8 S | 783.22982 | 10.7 | 16.2 | 10.9 | 5.4 |
| 2-2-d1E02-1-0488 | C40 H41 N5 O8 S2 | 783.23965 | 6.5 | 5.7 | 6.1 | -0.1 |
| 2-2-d1E02-1-0489 | C38 H39 F3 N4 O9 S | 784.23898 | 7.8 | 11.7 | 5.6 | -1.6 |
| 2-2-d1E02-1-0490 | C40 H43 F N6 O8 S | 786.28471 | 3.7 | 4.0 | 3.0 | 1.6 |
| 2-2-d1E02-1-0491 | C35 H35 F3 N6 O8 S2 | 788.19099 | 9.7 | 6.0 | -0.2 | 0.0 |
| 2-2-d1E02-1-0492 | C38 H39 N5 O8 S3 | 789.19608 | 8.6 | 8.6 | 3.3 | 2.6 |
| 2-2-d1E02-1-0493 | C40 H41 F3 N4 O8 S | 794.25972 | 8.3 | 5.0 | 1.4 | 2.4 |
| 2-2-d1E02-1-0494 | C39 H35 N5 O10 S2 | 797.18253 | 10.0 | 11.2 | 6.2 | -0.4 |
| 2-2-d1E02-1-0495 | C39 H41 N7 O8 S2 | 799.24580 | 16.3 | 17.3 | 15.0 | 12.7 |
| 2-2-d1E02-1-0496 | C38 H39 F3 N4 O8 S2 | 800.21614 | 6.3 | 0.0 | 2.5 | -1.3 |
| 2-2-d1E02-1-0497 | C39 H35 F3 N4 O10 S | 808.20260 | 4.2 | 11.0 | 5.5 | -4.7 |
| 2-2-d1E02-1-0498 | C39 H41 F3 N6 O8 S | 810.26587 | 6.2 | 7.2 | 6.5 | 5.0 |
| 2-2-d1E02-1-0499 | C41 H43 N7 O8 S2 | 825.26145 | 2.0 | 4.9 | 3.0 | 2.4 |
| 2-2-d1E02-1-0500 | C41 H43 F3 N6 O8 S | 836.28152 | 6.7 | 3.7 | 3.1 | 4.8 |

[Table 600]

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0001 | C34 H30 N4 O6 S | 622.18861 | 6.6 | 5.1 | 11.6 | 16.3 |
| 2-2-d1E03-1-0002 | C35 H38 N4 O5 S | 626.25629 | 1.7 | 2.5 | 8.7 | 7.7 |
| 2-2-d1E03-1-0003 | C34 H36 N4 O6 S | 628.23556 | -1.3 | 3.4 | 7.2 | 6.1 |
| 2-2-d1E03-1-0004 | C36 H32 N4 O5 S | 632.20934 | 7.1 | 13.0 | 15.2 | 10.4 |
| 2-2-d1E03-1-0005 | C36 H32 N4 O5 S | 632.20934 | 8.5 | 11.2 | 24.9 | 1.5 |
| 2-2-d1E03-1-0006 | C35 H31 N5 O5 S | 633.20459 | 2.2 | 7.0 | 7.4 | 4.2 |
| 2-2-d1E03-1-0007 | C35 H32 N4 O6 S | 636.20426 | 3.9 | 10.4 | 16.8 | 6.5 |
| 2-2-d1E03-1-0008 | C34 H30 N4 O5 S2 | 638.16576 | 13.6 | 11.8 | 11.5 | 7.6 |
| 2-2-d1E03-1-0009 | C33 H29 N5 O7 S | 639.17877 | -0.3 | 4.4 | 5.9 | 10.5 |
| 2-2-d1E03-1-0010 | C34 H29 F N4 O6 S | 640.17918 | 4.8 | 5.7 | 13.3 | 9.0 |
| 2-2-d1E03-1-0011 | C36 H40 N4 O5 S | 640.27194 | 3.3 | 5.2 | 5.7 | 3.8 |
| 2-2-d1E03-1-0012 | C35 H38 N4 O6 S | 642.25121 | 1.7 | 5.5 | 6.3 | 6.5 |
| 2-2-d1E03-1-0013 | C35 H37 F N4 O5 S | 644.24687 | 1.5 | 2.8 | 9.8 | 6.6 |
| 2-2-d1E03-1-0014 | C37 H34 N4 O5 S | 646.22499 | 8.9 | 13.5 | 13.9 | 5.1 |
| 2-2-d1E03-1-0015 | C37 H34 N4 O5 S | 646.22499 | 12.4 | 7.5 | 10.5 | 4.5 |
| 2-2-d1E03-1-0016 | C34 H35 F N4 O6 S | 646.22613 | -0.7 | 5.2 | 7.4 | 7.6 |
| 2-2-d1E03-1-0017 | C36 H33 N5 O5 S | 647.22024 | 0.5 | 6.8 | 9.9 | 8.3 |
| 2-2-d1E03-1-0018 | C36 H32 N4 O6 S | 648.20426 | 8.7 | 8.7 | 14.8 | 6.9 |
| 2-2-d1E03-1-0019 | C34 H30 N6 O6 S | 650.19475 | 3.6 | 3.8 | 8.7 | 12.8 |
| 2-2-d1E03-1-0020 | C36 H31 F N4 O5 S | 650.19992 | 5.8 | 15.2 | 16.6 | 11.6 |
| 2-2-d1E03-1-0021 | C36 H31 F N4 O5 S | 650.19992 | 8.4 | 7.6 | 11.4 | 7.4 |
| 2-2-d1E03-1-0022 | C35 H30 F N5 O5 S | 651.19517 | 4.0 | 9.4 | 9.3 | 5.2 |
| 2-2-d1E03-1-0023 | C35 H32 N4 O5 S2 | 652.18141 | 4.9 | 11.1 | 20.1 | 5.9 |
| 2-2-d1E03-1-0024 | C34 H32 N6 O6 S | 652.21040 | 8.2 | 8.9 | 12.7 | 5.2 |
| 2-2-d1E03-1-0025 | C37 H40 N4 O5 S | 652.27194 | 3.7 | 5.2 | 8.4 | 5.6 |
| 2-2-d1E03-1-0026 | C34 H31 N5 O7 S | 653.19442 | 4.9 | 4.1 | 7.6 | 5.4 |
| 2-2-d1E03-1-0027 | C36 H38 N4 O6 S | 654.25121 | 4.0 | 9.4 | 16.5 | 11.6 |
| 2-2-d1E03-1-0028 | C33 H29 N5 O6 S2 | 655.15593 | -6.5 | 4.5 | 19.6 | 7.6 |
| 2-2-d1E03-1-0029 | C34 H29 F N4 O5 S2 | 656.15634 | 2.5 | 18.4 | 13.5 | 8.0 |
| 2-2-d1E03-1-0030 | C38 H32 N4 O5 S | 656.20934 | 6.9 | 6.1 | 15.4 | 7.8 |
| 2-2-d1E03-1-0031 | C38 H32 N4 O5 S | 656.20934 | 16.4 | 22.1 | 14.0 | 0.0 |
| 2-2-d1E03-1-0032 | C33 H28 F N5 O7 S | 657.16935 | 0.3 | 4.0 | 10.7 | 12.2 |
| 2-2-d1E03-1-0033 | C37 H31 N5 O5 S | 657.20459 | 3.8 | 8.3 | 17.2 | 17.0 |
| 2-2-d1E03-1-0034 | C37 H31 N5 O5 S | 657.20459 | 8.7 | 16.4 | 19.7 | 3.7 |
| 2-2-d1E03-1-0035 | C37 H31 N5 O5 S | 657.20459 | 1.1 | 13.0 | 14.1 | 3.1 |
| 2-2-d1E03-1-0036 | C38 H34 N4 O5 S | 658.22499 | 8.2 | 6.1 | 14.7 | 5.9 |
| 2-2-d1E03-1-0037 | C38 H34 N4 O5 S | 658.22499 | 5.2 | -24.3 | 20.1 | 19.9 |

(continued)

[Table 11-3-3]

| | | | AS-MS(%) | | | |
|---|---|---|---|---|---|---|
| ID | Molecular Formula | Exact Mass | N1 | N2 | N3 | N4 |
| 2-2-d1E03-1-0038 | C37 H33 N5 O5 S | 659.22024 | 4.5 | 4.2 | 12.0 | 6.4 |
| 2-2-d1E03-1-0039 | C37 H35 N5 O5 S | 661.23589 | -6.4 | 37.9 | 13.2 | 14.9 |
| 2-2-d1E03-1-0040 | C37 H34 N4 O6 S | 662.21991 | 7.2 | 13.5 | 17.9 | 0.5 |
| 2-2-d1E03-1-0041 | C36 H34 N6 O5 S | 662.23114 | 4.6 | 6.9 | 10.5 | 4.3 |
| 2-2-d1E03-1-0042 | C35 H33 N7 O5 S | 663.22639 | 5.1 | 2.7 | 15.7 | 10.2 |
| 2-2-d1E03-1-0043 | C36 H32 N4 O5 S2 | 664.18141 | 0.0 | 0.0 | 0.0 | 0.0 |
| 2-2-d1E03-1-0044 | C35 H32 N6 O6 S | 664.21040 | 4.9 | 6.5 | 10.7 | 13.7 |
| 2-2-d1E03-1-0045 | C37 H36 N4 O6 S | 664.23556 | 5.8 | 5.8 | 6.6 | 5.8 |
| 2-2-d1E03-1-0046 | C35 H31 N5 O7 S | 665.19442 | 25.1 | 2.2 | 14.0 | 10.3 |
| 2-2-d1E03-1-0047 | C36 H31 F N4 O6 S | 666.19483 | 9.7 | 16.7 | 14.2 | 13.9 |
| 2-2-d1E03-1-0048 | C35 H34 N6 O6 S | 666.22605 | 5.6 | 3.9 | 9.2 | 5.6 |
| 2-2-d1E03-1-0049 | C38 H42 N4 O5 S | 666.28759 | -2.3 | 10.9 | 12.4 | -1.6 |
| 2-2-d1E03-1-0050 | C34 H29 FN6 O6 S | 668.18533 | 6.8 | 10.9 | 23.7 | 6.2 |
| 2-2-d1E03-1-0051 | C34 H32 N6 O5 S2 | 668.18756 | 1.5 | 13.6 | 16.6 | 8.8 |
| 2-2-d1E03-1-0052 | C37 H40 N4 O6 S | 668.26686 | 2.0 | 4.8 | 6.5 | 2.5 |
| 2-2-d1E03-1-0053 | C34 H31 N5 O6 S2 | 669.17158 | 5.2 | 5.5 | 13.0 | 2.6 |
| 2-2-d1E03-1-0054 | C34 H31 FN6 O6 S | 670.20098 | 3.9 | 6.1 | 9.6 | 6.2 |
| 2-2-d1E03-1-0055 | C39 H34 N4 O5 S | 670.22499 | 10.6 | 14.8 | 17.9 | 9.5 |
| 2-2-d1E03-1-0056 | C39 H34 N4 O5 S | 670.22499 | 5.3 | 7.9 | 9.6 | 5.8 |
| 2-2-d1E03-1-0057 | C37 H39 FN4 O5 S | 670.26252 | 3.2 | 3.9 | 5.2 | 2.8 |
| 2-2-d1E03-1-0058 | C38 H33 N5 O5 S | 671.22024 | 4.7 | 15.8 | 21.1 | 8.3 |
| 2-2-d1E03-1-0059 | C38 H33 N5 O5 S | 671.22024 | 11.8 | 8.4 | 26.1 | 10.9 |
| 2-2-d1E03-1-0060 | C38 H33 N5 O5 S | 671.22024 | 7.6 | 14.2 | 25.4 | 10.9 |
| 2-2-d1E03-1-0061 | C39 H36 N4 O5 S | 672.24064 | 10.3 | 14.1 | 16.5 | -4.4 |
| 2-2-d1E03-1-0062 | C39 H36 N4 O5 S | 672.24064 | -2.1 | 4.1 | -24.6 | -28.0 |
| 2-2-d1E03-1-0063 | C36 H37 F N4 O6 S | 672.24178 | -0.7 | 2.5 | 11.0 | 4.4 |
| 2-2-d1E03-1-0064 | C33 H28 F N5 O6 S2 | 673.14650 | 10.4 | 7.0 | 12.4 | 6.2 |
| 2-2-d1E03-1-0065 | C38 H35 N5 O5 S | 673.23589 | 3.1 | 12.1 | 18.8 | 17.3 |
| 2-2-d1E03-1-0066 | C38 H31 FN4 O5 S | 674.19992 | 10.0 | 10.3 | 11.5 | 3.5 |
| 2-2-d1E03-1-0067 | C38 H31 FN4 O5 S | 674.19992 | -4.1 | 0.0 | 0.0 | -5.1 |
| 2-2-d1E03-1-0068 | C39 H38 N4 O5 S | 674.25629 | -1.1 | 13.5 | 13.7 | 1.3 |
| 2-2-d1E03-1-0069 | C37 H30 F N5 O5 S | 675.19517 | 9.3 | 8.5 | 14.2 | 16.4 |
| 2-2-d1E03-1-0070 | C37 H30 F N5 O5 S | 675.19517 | 9.3 | 12.2 | 10.4 | 5.0 |
| 2-2-d1E03-1-0071 | C37 H30 F N5 O5 S | 675.19517 | 5.4 | 10.3 | 21.3 | 6.9 |
| 2-2-d1E03-1-0072 | C38 H37 N5 O5 S | 675.25154 | 4.2 | 5.9 | 14.4 | 10.0 |
| 2-2-d1E03-1-0073 | C36 H32 N6 O6 S | 676.21040 | 21.4 | 17.6 | 11.2 | -5.4 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0074 | C38 H33 FN4 O5 S | 676.21557 | -0.1 | -0.9 | 25.9 | 12.5 |
| 2-2-d1E03-1-0075 | C38 H33 FN4 O5 S | 676.21557 | -1.6 | 6.8 | 14.9 | 6.5 |
| 2-2-d1E03-1-0076 | C37 H36 N6 O5 S | 676.24679 | 9.5 | 10.3 | 5.0 | 9.8 |
| 2-2-d1E03-1-0077 | C37 H32 F N5 O5 S | 677.21082 | 4.7 | 3.1 | 9.1 | 6.1 |
| 2-2-d1E03-1-0078 | C36 H35 N7 O5 S | 677.24204 | 2.8 | 13.3 | 13.9 | 9.3 |
| 2-2-d1E03-1-0079 | C37 H34 N4 O5 S2 | 678.19706 | -13.2 | 0.0 | 29.6 | -5.5 |
| 2-2-d1E03-1-0080 | C36 H34 N6 O6 S | 678.22605 | 1.5 | 12.8 | 15.6 | 24.4 |
| 2-2-d1E03-1-0081 | C38 H38 N4 O6 S | 678.25121 | 4.8 | 4.7 | 14.2 | 5.0 |
| 2-2-d1E03-1-0082 | C35 H29 N5 O6 S2 | 679.15593 | 6.8 | 10.6 | 15.8 | 8.6 |
| 2-2-d1E03-1-0083 | C36 H33 N5 O7 S | 679.21007 | 2.3 | 5.2 | 11.6 | 3.8 |
| 2-2-d1E03-1-0084 | C37 H34 F N5 O5 S | 679.22647 | 9.3 | 18.5 | 8.7 | 21.8 |
| 2-2-d1E03-1-0085 | C37 H36 N4 O5 S2 | 680.21271 | 4.6 | 9.4 | 15.5 | 4.8 |
| 2-2-d1E03-1-0086 | C36 H33 F N6 O5 S | 680.22172 | 3.1 | 3.0 | 13.2 | 6.2 |
| 2-2-d1E03-1-0087 | C35 H31 N5 O6 S2 | 681.17158 | 0.0 | 2.2 | 0.0 | -4.7 |
| 2-2-d1E03-1-0088 | C35 H32 F N7 O5 S | 681.21697 | 2.6 | 2.4 | 11.7 | 8.2 |
| 2-2-d1E03-1-0089 | C36 H31 FN4 O5 S2 | 682.17199 | 12.4 | 11.4 | 19.2 | -1.4 |
| 2-2-d1E03-1-0090 | C35 H34 N6 O5 S2 | 682.20321 | 5.4 | 7.8 | 14.5 | 3.1 |
| 2-2-d1E03-1-0091 | C40 H34 N4 O5 S | 682.22499 | 13.7 | 13.1 | 12.7 | 7.4 |
| 2-2-d1E03-1-0092 | C40 H34 N4 O5 S | 682.22499 | 0.0 | -85.5 | 20.8 | 0.0 |
| 2-2-d1E03-1-0093 | C37 H35 FN4 O6 S | 682.22613 | 2.5 | 7.2 | 11.3 | 4.4 |
| 2-2-d1E03-1-0094 | C35 H30 F N5 O7 S | 683.18500 | 6.1 | 8.0 | 5.8 | 0.9 |
| 2-2-d1E03-1-0095 | C39 H33 N5 O5 S | 683.22024 | 18.5 | 19.1 | 16.4 | 10.2 |
| 2-2-d1E03-1-0096 | C39 H33 N5 O5 S | 683.22024 | 7.9 | 1.3 | 10.5 | 10.0 |
| 2-2-d1E03-1-0097 | C39 H33 N5 O5 S | 683.22024 | 14.8 | 11.3 | 14.2 | -27.0 |
| 2-2-d1E03-1-0098 | C36 H37 N5 O5 S2 | 683.22361 | 4.8 | 5.5 | 4.2 | 5.4 |
| 2-2-d1E03-1-0099 | C35 H35 N5 O6 S2 | 685.20288 | 4.6 | 8.1 | 4.5 | 5.7 |
| 2-2-d1E03-1-0100 | C34 H30 N4 O8 S2 | 686.15051 | 1.0 | 3.5 | 8.0 | 3.5 |
| 2-2-d1E03-1-0101 | C34 H31 FN6 O5 S2 | 686.17814 | 4.2 | 9.1 | 12.1 | 5.9 |
| 2-2-d1E03-1-0102 | C39 H37 N5 O5 S | 687.25154 | 0.0 | 0.0 | 7.2 | 0.0 |
| 2-2-d1E03-1-0103 | C38 H32 N4 O7 S | 688.19917 | 4.6 | 13.7 | 17.0 | 3.9 |
| 2-2-d1E03-1-0104 | C38 H36 N6 O5 S | 688.24679 | 0.0 | 0.0 | 0.0 | 0.0 |
| 2-2-d1E03-1-0105 | C40 H40 N4 O5 S | 688.27194 | 7.7 | 9.3 | 18.9 | 11.7 |
| 2-2-d1E03-1-0106 | C37 H31 N5 O5 S2 | 689.17666 | 8.7 | 9.4 | 14.8 | 4.1 |
| 2-2-d1E03-1-0107 | C37 H31 N5 O5 S2 | 689.17666 | 6.0 | 5.9 | 14.7 | 8.9 |
| 2-2-d1E03-1-0108 | C37 H35 N7 O5 S | 689.24204 | 12.8 | 13.9 | 14.7 | 13.5 |
| 2-2-d1E03-1-0109 | C36 H30 N6 O5 S2 | 690.17191 | 5.1 | 9.3 | 11.9 | 6.3 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0110 | C35 H29 F3 N4 O6 S | 690.17599 | 1.0 | 10.8 | 13.3 | 0.8 |
| 2-2-d1E03-1-0111 | C35 H38 N4 O7 S2 | 690.21819 | 3.7 | -1.0 | 5.5 | 5.8 |
| 2-2-d1E03-1-0112 | C37 H34 N6 O6 S | 690.22605 | 3.0 | 11.1 | 11.7 | -2.7 |
| 2-2-d1E03-1-0113 | C39 H38 N4 O6 S | 690.25121 | 3.1 | 6.0 | 10.8 | 3.4 |
| 2-2-d1E03-1-0114 | C34 H36 N4 O8 S2 | 692.19746 | 2.1 | 3.2 | 5.7 | 5.2 |
| 2-2-d1E03-1-0115 | C37 H36 N6 O6 S | 692.24170 | 2.5 | 5.8 | 11.8 | 4.5 |
| 2-2-d1E03-1-0116 | C39 H37 F N4 O5 S | 692.24687 | 6.2 | 14.1 | 11.9 | 6.4 |
| 2-2-d1E03-1-0117 | C36 H31 F N6 O6 S | 694.20098 | 0.0 | 4.8 | 0.0 | 3.0 |
| 2-2-d1E03-1-0118 | C36 H34 N6 O5 S2 | 694.20321 | -12.7 | -0.4 | 12.6 | 5.0 |
| 2-2-d1E03-1-0119 | C38 H38 N4 O5 S2 | 694.22836 | 13.0 | 9.7 | 7.3 | 4.2 |
| 2-2-d1E03-1-0120 | C36 H37 F3 N4 O5 S | 694.24368 | 3.7 | 7.3 | 6.5 | 4.7 |
| 2-2-d1E03-1-0121 | C35 H29 N5 O5 S3 | 695.13308 | 7.0 | 11.7 | 16.8 | 4.5 |
| 2-2-d1E03-1-0122 | C36 H33 N5 O6 S2 | 695.18723 | 2.0 | -1.0 | 16.1 | 8.1 |
| 2-2-d1E03-1-0123 | C34 H28 N6 O7 S2 | 696.14609 | 3.7 | 7.6 | 10.9 | 9.2 |
| 2-2-d1E03-1-0124 | C36 H32 N4 O7 S2 | 696.17124 | 1.5 | 3.2 | 8.6 | 7.4 |
| 2-2-d1E03-1-0125 | C36 H32 N4 O7 S2 | 696.17124 | 2.0 | 2.6 | 2.9 | 8.6 |
| 2-2-d1E03-1-0126 | C36 H33 F N6 O6 S | 696.21663 | 7.9 | 6.0 | 18.8 | 3.5 |
| 2-2-d1E03-1-0127 | C35 H35 F3 N4 O6 S | 696.22294 | 1.6 | 6.2 | 6.7 | 2.6 |
| 2-2-d1E03-1-0128 | C41 H36 N4 O5 S | 696.24064 | 31.8 | 13.0 | 18.0 | 21.5 |
| 2-2-d1E03-1-0129 | C41 H36 N4 O5 S | 696.24064 | 2.4 | 21.0 | 6.8 | 42.8 |
| 2-2-d1E03-1-0130 | C35 H31 N5 O7 S2 | 697.16649 | 1.5 | 2.5 | 6.7 | 3.5 |
| 2-2-d1E03-1-0131 | C40 H35 N5 O5 S | 697.23589 | 8.2 | 4.8 | 22.4 | 10.1 |
| 2-2-d1E03-1-0132 | C40 H35 N5 O5 S | 697.23589 | 10.8 | 15.9 | 18.9 | 1.2 |
| 2-2-d1E03-1-0133 | C40 H35 N5 O5 S | 697.23589 | 9.0 | 11.5 | 15.7 | 1.3 |
| 2-2-d1E03-1-0134 | C37 H35 F N4 O5 S2 | 698.20329 | 6.2 | 5.9 | 15.6 | 5.5 |
| 2-2-d1E03-1-0135 | C35 H30 F N5 O6 S2 | 699.16215 | 9.0 | 6.0 | 6.7 | 6.1 |
| 2-2-d1E03-1-0136 | C35 H32 N4 O8 S2 | 700.16616 | 3.2 | 2.6 | 9.7 | 8.7 |
| 2-2-d1E03-1-0137 | C37 H31 F3 N4 O5 S | 700.19673 | 6.5 | 10.8 | 9.6 | 3.8 |
| 2-2-d1E03-1-0138 | C37 H31 F3 N4 O5 S | 700.19673 | 0.0 | 0.0 | 0.0 | 0.0 |
| 2-2-d1E03-1-0139 | C40 H33 F N4 O5 S | 700.21557 | 19.5 | 12.8 | 13.0 | 4.1 |
| 2-2-d1E03-1-0140 | C40 H33 F N4 O5 S | 700.21557 | 7.3 | 13.6 | -15.4 | 1.4 |
| 2-2-d1E03-1-0141 | C41 H40 N4 O5 S | 700.27194 | 21.9 | 21.8 | 35.6 | 22.8 |
| 2-2-d1E03-1-0142 | C36 H30 F3 N5 O5 S | 701.19197 | 5.0 | 14.9 | 15.1 | 9.8 |
| 2-2-d1E03-1-0143 | C39 H32 F N5 O5 S | 701.21082 | 21.6 | 10.5 | 12.8 | 6.6 |
| 2-2-d1E03-1-0144 | C39 H32 F N5 O5 S | 701.21082 | 10.0 | 15.1 | 12.1 | 13.7 |
| 2-2-d1E03-1-0145 | C39 H32 F N5 O5 S | 701.21082 | 0.4 | 3.6 | 6.0 | 6.6 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0146 | C40 H39 N5 O5 S | 701.26719 | 10.7 | 33.4 | -7.2 | 0.0 |
| 2-2-d1E03-1-0147 | C34 H30 N4 O7 S3 | 702.12766 | 2.2 | 3.6 | 6.6 | 5.4 |
| 2-2-d1E03-1-0148 | C39 H34 N4 O7 S | 702.21482 | 0.0 | 14.3 | 18.1 | 7.1 |
| 2-2-d1E03-1-0149 | C39 H38 N6 O5 S | 702.26244 | 0.0 | 0.0 | 0.0 | 0.0 |
| 2-2-d1E03-1-0150 | C33 H29 N5 O9 S2 | 703.14067 | 0.0 | 0.0 | 14.4 | 0.0 |
| 2-2-d1E03-1-0151 | C38 H37 N7 O5 S | 703.25769 | 1.3 | 7.5 | 17.2 | 7.7 |
| 2-2-d1E03-1-0152 | C34 H29 F N4 O8 S2 | 704.14108 | 1.2 | 0.4 | 5.6 | 7.7 |
| 2-2-d1E03-1-0153 | C36 H40 N4 O7 S2 | 704.23384 | 0.0 | 0.0 | 5.0 | 2.7 |
| 2-2-d1E03-1-0154 | C40 H40 N4 O6 S | 704.26686 | 16.1 | 24.2 | 20.9 | 22.0 |
| 2-2-d1E03-1-0155 | C37 H31 N5 O6 S2 | 705.17158 | 6.8 | 14.8 | 18.0 | 5.5 |
| 2-2-d1E03-1-0156 | C39 H36 F N5 O5 S | 705.24212 | 0.7 | 25.9 | 0.0 | 0.0 |
| 2-2-d1E03-1-0157 | C35 H29 F3 N4 O5 S2 | 706.15315 | 10.6 | 6.5 | 17.2 | 6.3 |
| 2-2-d1E03-1-0158 | C38 H31 FN4 O7 S | 706.18975 | 10.0 | 11.2 | 13.3 | 1.7 |
| 2-2-d1E03-1-0159 | C35 H38 N4 O8 S2 | 706.21311 | 0.3 | 1.7 | 6.4 | 5.4 |
| 2-2-d1E03-1-0160 | C39 H38 N4 O5 S2 | 706.22836 | 3.3 | 25.8 | 30.3 | 26.3 |
| 2-2-d1E03-1-0161 | C38 H35 F N6 O5 S | 706.23737 | 10.8 | 5.6 | 49.1 | 33.7 |
| 2-2-d1E03-1-0162 | C35 H29 N7 O6 S2 | 707.16207 | 2.7 | 1.0 | 12.0 | 9.4 |
| 2-2-d1E03-1-0163 | C34 H28 F3 N5 O7 S | 707.16615 | 4.7 | 1.3 | 10.2 | 3.9 |
| 2-2-d1E03-1-0164 | C37 H34 F N7 O5 S | 707.23262 | 5.6 | 11.9 | 17.6 | 7.3 |
| 2-2-d1E03-1-0165 | C35 H37 F N4 O7 S2 | 708.20877 | 3.3 | 0.6 | 8.0 | 4.6 |
| 2-2-d1E03-1-0166 | C37 H36 N6 O5 S2 | 708.21886 | 0.0 | 0.0 | 0.0 | 0.0 |
| 2-2-d1E03-1-0167 | C39 H37 F N4 O6 S | 708.24178 | 6.0 | 6.4 | 13.9 | 2.5 |
| 2-2-d1E03-1-0168 | C35 H31 N7 O6 S2 | 709.17772 | 1.0 | 8.9 | 11.3 | 10.3 |
| 2-2-d1E03-1-0169 | C38 H39 N5 O5 S2 | 709.23926 | 0.0 | 11.0 | 9.6 | 1.4 |
| 2-2-d1E03-1-0170 | C37 H34 N4 O7 S2 | 710.18689 | -1.8 | 3.6 | 9.0 | -1.9 |
| 2-2-d1E03-1-0171 | C37 H34 N4 O7 S2 | 710.18689 | -1.2 | 2.3 | 8.7 | 7.9 |
| 2-2-d1E03-1-0172 | C34 H35 F N4 O8 S2 | 710.18803 | 1.9 | 1.6 | 4.9 | 3.9 |
| 2-2-d1E03-1-0173 | C36 H33 N5 O7 S2 | 711.18214 | 1.1 | 3.9 | 5.4 | 2.1 |
| 2-2-d1E03-1-0174 | C37 H37 N5 O6 S2 | 711.21853 | 2.3 | 5.7 | 8.0 | 3.7 |
| 2-2-d1E03-1-0175 | C34 H28 N6 O6 S3 | 712.12324 | 2.1 | 6.8 | 11.2 | 5.4 |
| 2-2-d1E03-1-0176 | C36 H32 N4 O8 S2 | 712.16616 | 2.8 | 1.6 | 4.4 | 7.4 |
| 2-2-d1E03-1-0177 | C36 H33 F N6 O5 S2 | 712.19379 | 0.0 | 11.2 | 14.1 | 7.4 |
| 2-2-d1E03-1-0178 | C39 H31 N5 O5 S2 | 713.17666 | 11.6 | 20.5 | 17.9 | 7.0 |
| 2-2-d1E03-1-0179 | C39 H31 N5 O5 S2 | 713.17666 | 14.8 | 6.1 | 2.4 | 7.0 |
| 2-2-d1E03-1-0180 | C34 H30 N6 O8 S2 | 714.15665 | 0.6 | 3.9 | 6.9 | 3.4 |
| 2-2-d1E03-1-0181 | C36 H31 F N4 O7 S2 | 714.16182 | -2.5 | 4.6 | 6.0 | 5.2 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0182 | C36 H31 F N4 O7 S2 | 714.16182 | 5.5 | 4.6 | 7.0 | 7.2 |
| 2-2-d1E03-1-0183 | C38 H30 N6 O5 S2 | 714.17191 | 12.9 | 0.0 | 19.5 | 12.6 |
| 2-2-d1E03-1-0184 | C38 H30 N6 O5 S2 | 714.17191 | 7.6 | 12.8 | 16.9 | 12.8 |
| 2-2-d1E03-1-0185 | C38 H30 N6 O5 S2 | 714.17191 | 9.7 | 12.0 | 11.2 | 7.9 |
| 2-2-d1E03-1-0186 | C40 H34 N4 O7 S | 714.21482 | 0.0 | 0.0 | 0.0 | 0.0 |
| 2-2-d1E03-1-0187 | C42 H42 N4 O5 S | 714.28759 | 5.6 | 12.6 | 18.0 | 11.6 |
| 2-2-d1E03-1-0188 | C35 H30 F N5 O7 S2 | 715.15707 | 1.0 | 4.1 | 4.8 | 3.4 |
| 2-2-d1E03-1-0189 | C39 H33 N5 O5 S2 | 715.19231 | 7.2 | 11.9 | 11.8 | 4.3 |
| 2-2-d1E03-1-0190 | C39 H33 N5 O5 S2 | 715.19231 | 7.9 | 11.5 | 14.0 | 8.1 |
| 2-2-d1E03-1-0191 | C35 H32 N4 O7 S3 | 716.14331 | 5.5 | 6.3 | 5.8 | 15.5 |
| 2-2-d1E03-1-0192 | C34 H32 N6 O8 S2 | 716.17230 | 1.4 | 4.7 | 5.1 | 5.8 |
| 2-2-d1E03-1-0193 | C38 H32 N6 O5 S2 | 716.18756 | 9.8 | 16.1 | 8.0 | 17.5 |
| 2-2-d1E03-1-0194 | C37 H31 F3 N4 O6 S | 716.19164 | 11.1 | 8.5 | 22.6 | 10.2 |
| 2-2-d1E03-1-0195 | C37 H40 N4 O7 S2 | 716.23384 | 0.0 | 0.0 | 0.0 | 0.0 |
| 2-2-d1E03-1-0196 | C40 H40 N6 O5 S | 716.27809 | 7.4 | 6.2 | 18.8 | 13.3 |
| 2-2-d1E03-1-0197 | C34 H31 N5 O9 S2 | 717.15632 | -11.4 | 4.4 | 6.8 | 1.3 |
| 2-2-d1E03-1-0198 | C35 H29 F3 N6 O6 S | 718.18214 | 1.6 | 1.5 | 7.8 | 5.7 |
| 2-2-d1E03-1-0199 | C38 H34 N6 O5 S2 | 718.20321 | 8.7 | 11.3 | 15.7 | 5.8 |
| 2-2-d1E03-1-0200 | C36 H38 N4 O8 S2 | 718.21311 | 3.6 | 0.2 | 11.7 | 7.7 |
| 2-2-d1E03-1-0201 | C41 H39 FN4 O5 S | 718.26252 | 9.7 | 27.3 | 15.4 | 13.8 |
| 2-2-d1E03-1-0202 | C33 H29 N5 O8 S3 | 719.11783 | 7.5 | 7.2 | 19.9 | 12.1 |
| 2-2-d1E03-1-0203 | C37 H33 N7 O5 S2 | 719.19846 | 25.4 | -2.6 | 13.0 | 22.0 |
| 2-2-d1E03-1-0204 | C34 H29 F N4 O7 S3 | 720.11824 | 0.0 | 0.2 | 4.8 | 9.4 |
| 2-2-d1E03-1-0205 | C38 H32 N4 O7 S2 | 720.17124 | 8.3 | 4.4 | 6.9 | 7.2 |
| 2-2-d1E03-1-0206 | C38 H32 N4 O7 S2 | 720.17124 | -3.2 | -0.2 | 13.4 | 3.1 |
| 2-2-d1E03-1-0207 | C36 H32 N8 O5 S2 | 720.19371 | 5.3 | 7.0 | 9.0 | 7.5 |
| 2-2-d1E03-1-0208 | C35 H31 F3 N6 O6 S | 720.19779 | 2.9 | 4.4 | 13.7 | 5.1 |
| 2-2-d1E03-1-0209 | C40 H40 N4 O5 S2 | 720.24401 | 11.0 | 30.3 | 0.0 | 0.0 |
| 2-2-d1E03-1-0210 | C38 H39 F3 N4 O5 S | 720.25933 | 1.6 | 9.4 | 19.6 | 9.8 |
| 2-2-d1E03-1-0211 | C33 H28 F N5 O9 S2 | 721.13125 | 0.0 | -7.9 | 9.6 | 10.9 |
| 2-2-d1E03-1-0212 | C37 H31 N5 O5 S3 | 721.14873 | 10.0 | 8.1 | 14.4 | 9.5 |
| 2-2-d1E03-1-0213 | C37 H31 N5 O7 S2 | 721.16649 | 2.2 | 5.8 | 6.0 | 4.6 |
| 2-2-d1E03-1-0214 | C37 H31 N5 O7 S2 | 721.16649 | 20.3 | 22.3 | 14.8 | 11.6 |
| 2-2-d1E03-1-0215 | C37 H31 N5 O7 S2 | 721.16649 | 0.0 | 0.0 | 19.4 | 15.4 |
| 2-2-d1E03-1-0216 | C38 H35 N5 O6 S2 | 721.20288 | 8.4 | 6.8 | 10.8 | 3.9 |
| 2-2-d1E03-1-0217 | C36 H30 N6 O7 S2 | 722.16174 | 2.8 | 7.8 | 12.6 | 6.7 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0218 | C38 H34 N4 O7 S2 | 722.18689 | 0.0 | 0.0 | 0.0 | 0.0 |
| 2-2-d1E03-1-0219 | C38 H34 N4 O7 S2 | 722.18689 | 0.0 | 0.0 | 0.0 | 0.0 |
| 2-2-d1E03-1-0220 | C37 H37 F3 N4 O6 S | 722.23859 | 6.9 | -0.6 | -2.4 | 0.2 |
| 2-2-d1E03-1-0221 | C34 H28 F3 N5 O6 S2 | 723.14331 | 9.0 | 21.9 | 23.7 | 8.0 |
| 2-2-d1E03-1-0222 | C37 H33 N5 O7 S2 | 723.18214 | 0.8 | 2.8 | 5.6 | 3.5 |
| 2-2-d1E03-1-0223 | C39 H31 F3 N4 O5 S | 724.19673 | 4.1 | 1.2 | 13.3 | 12.8 |
| 2-2-d1E03-1-0224 | C39 H31 F3 N4 O5 S | 724.19673 | 0.0 | 0.0 | 0.0 | 0.0 |
| 2-2-d1E03-1-0225 | C39 H37 F N4 O5 S2 | 724.21894 | 19.5 | 11.1 | 8.0 | 4.9 |
| 2-2-d1E03-1-0226 | C35 H31 N7 O5 S3 | 725.15488 | 3.6 | 7.8 | 10.7 | 7.3 |
| 2-2-d1E03-1-0227 | C38 H30 F3 N5 O5 S | 725.19197 | 12.0 | 12.1 | 21.3 | 14.8 |
| 2-2-d1E03-1-0228 | C38 H30 F3 N5 O5 S | 725.19197 | -2.0 | 11.8 | 13.9 | 0.6 |
| 2-2-d1E03-1-0229 | C38 H30 F3 N5 O5 S | 725.19197 | 5.3 | 10.1 | 28.3 | 4.8 |
| 2-2-d1E03-1-0230 | C37 H35 N5 O7 S2 | 725.19779 | 6.8 | 3.3 | 7.3 | 12.4 |
| 2-2-d1E03-1-0231 | C37 H34 N4 O8 S2 | 726.18181 | 8.7 | 5.3 | 8.5 | 5.0 |
| 2-2-d1E03-1-0232 | C36 H34 N6 O7 S2 | 726.19304 | 0.1 | 7.8 | 12.1 | 3.6 |
| 2-2-d1E03-1-0233 | C39 H33 F3 N4 O5 S | 726.21238 | -0.2 | 25.3 | 18.5 | 12.5 |
| 2-2-d1E03-1-0234 | C39 H33 F3 N4 O5 S | 726.21238 | 6.1 | 24.9 | 4.1 | -8.3 |
| 2-2-d1E03-1-0235 | C35 H33 N7 O7 S2 | 727.18829 | 7.7 | 11.2 | 14.3 | 7.3 |
| 2-2-d1E03-1-0236 | C38 H32 F3 N5 O5 S | 727.20762 | 7.3 | 11.2 | 16.7 | 4.7 |
| 2-2-d1E03-1-0237 | C36 H32 N4 O7 S3 | 728.14331 | 0.0 | 0.0 | 0.0 | 0.0 |
| 2-2-d1E03-1-0238 | C35 H32 N6 O8 S2 | 728.17230 | 0.0 | 0.0 | 0.0 | 0.0 |
| 2-2-d1E03-1-0239 | C37 H36 N4 O8 S2 | 728.19746 | 3.2 | 4.8 | 9.0 | 7.1 |
| 2-2-d1E03-1-0240 | C41 H36 N4 O7 S | 728.23047 | 6.2 | 5.1 | 5.5 | -7.5 |
| 2-2-d1E03-1-0241 | C35 H31 N5 O9 S2 | 729.15632 | 14.4 | 0.8 | 15.7 | -2.6 |
| 2-2-d1E03-1-0242 | C38 H34 F3 N5 O5 S | 729.22327 | 17.1 | 23.9 | 28.9 | -8.0 |
| 2-2-d1E03-1-0243 | C36 H31 F N4 O8 S2 | 730.15673 | 5.3 | 3.1 | 9.7 | 7.5 |
| 2-2-d1E03-1-0244 | C35 H34 N6 O8 S2 | 730.18795 | 1.4 | 6.0 | 9.4 | 5.9 |
| 2-2-d1E03-1-0245 | C37 H33 F3 N6 O5 S | 730.21852 | 6.1 | 1.5 | 13.4 | 9.3 |
| 2-2-d1E03-1-0246 | C38 H42 N4 O7 S2 | 730.24949 | 0.9 | -0.9 | 3.2 | 1.4 |
| 2-2-d1E03-1-0247 | C41 H42 N6 O5 S | 730.29374 | 9.7 | 10.3 | 18.3 | 12.0 |
| 2-2-d1E03-1-0248 | C36 H32 F3 N7 O5 S | 731.21377 | -0.6 | 12.9 | 9.4 | 3.3 |
| 2-2-d1E03-1-0249 | C40 H37 N5 O5 S2 | 731.22361 | 11.7 | 7.9 | 12.6 | 10.9 |
| 2-2-d1E03-1-0250 | C34 H29 F N6 O8 S2 | 732.14723 | 2.0 | 5.3 | 9.0 | 2.4 |
| 2-2-d1E03-1-0251 | C34 H32 N6 O7 S3 | 732.14946 | 7.7 | 7.2 | 13.2 | 10.3 |
| 2-2-d1E03-1-0252 | C37 H31 F3 N4 O5 S2 | 732.16880 | -3.8 | 3.1 | 6.8 | 2.3 |
| 2-2-d1E03-1-0253 | C40 H33 F N4 O7 S | 732.20540 | 7.2 | 9.9 | 15.7 | 3.7 |

(continued)

[Table 11-3-3]

| | | | AS-MS(%) | | | |
|---|---|---|---|---|---|---|
| ID | Molecular Formula | Exact Mass | N1 | N2 | N3 | N4 |
| 2-2-d1E03-1-0254 | C38 H35 F3 N4 O6 S | 732.22294 | 31.7 | 20.7 | 31.0 | 38.1 |
| 2-2-d1E03-1-0255 | C37 H40 N4 O8 S2 | 732.22876 | 5.8 | 0.6 | 10.0 | 7.1 |
| 2-2-d1E03-1-0256 | C34 H31 N5 O8 S3 | 733.13348 | -1.0 | 4.4 | 18.7 | 7.6 |
| 2-2-d1E03-1-0257 | C37 H31 N7 O6 S2 | 733.17772 | 1.8 | 6.0 | 12.4 | 5.9 |
| 2-2-d1E03-1-0258 | C36 H30 F3 N5 O7 S | 733.18180 | 9.4 | 3.3 | -1.4 | -2.5 |
| 2-2-d1E03-1-0259 | C34 H31 FN6 O8 S2 | 734.16288 | 3.4 | 4.6 | 6.4 | 5.6 |
| 2-2-d1E03-1-0260 | C39 H34 N4 O7 S2 | 734.18689 | 6.0 | 2.4 | 0.0 | 17.9 |
| 2-2-d1E03-1-0261 | C39 H34 N4 O7 S2 | 734.18689 | 5.9 | 1.2 | 6.8 | 17.5 |
| 2-2-d1E03-1-0262 | C37 H39 F N4 O7 S2 | 734.22442 | 0.0 | -0.1 | -2.7 | 4.9 |
| 2-2-d1E03-1-0263 | C40 H39 F N6 O5 S | 734.26867 | 14.4 | 15.1 | 13.1 | 13.9 |
| 2-2-d1E03-1-0264 | C38 H33 N5 O7 S2 | 735.18214 | 1.4 | 2.9 | 6.4 | 6.2 |
| 2-2-d1E03-1-0265 | C38 H33 N5 O7 S2 | 735.18214 | 14.1 | 15.6 | 17.1 | 7.5 |
| 2-2-d1E03-1-0266 | C38 H33 N5 O7 S2 | 735.18214 | 15.2 | 2.8 | 12.5 | 9.5 |
| 2-2-d1E03-1-0267 | C37 H33 N7 O6 S2 | 735.19337 | 1.9 | 7.4 | 6.7 | 4.9 |
| 2-2-d1E03-1-0268 | C35 H31 F3 N6 O5 S2 | 736.17494 | 4.5 | 5.9 | 19.3 | 10.2 |
| 2-2-d1E03-1-0269 | C39 H36 N4 O7 S2 | 736.20254 | 0.3 | 0.0 | 2.4 | 16.3 |
| 2-2-d1E03-1-0270 | C39 H36 N4 O7 S2 | 736.20254 | 0.0 | 0.0 | 0.0 | 0.0 |
| 2-2-d1E03-1-0271 | C36 H37 F N4 O8 S2 | 736.20368 | 0.0 | 0.0 | 0.0 | 0.0 |
| 2-2-d1E03-1-0272 | C33 H28 F N5 O8 S3 | 737.10840 | 0.0 | 0.0 | 0.0 | 0.0 |
| 2-2-d1E03-1-0273 | C38 H35 N5 O5 S3 | 737.18003 | 7.3 | 10.2 | 20.1 | 9.4 |
| 2-2-d1E03-1-0274 | C38 H35 N5 O7 S2 | 737.19779 | 0.4 | 3.2 | 5.2 | 4.7 |
| 2-2-d1E03-1-0275 | C36 H30 N6 O6 S3 | 738.13889 | 3.8 | 6.6 | 11.2 | 3.9 |
| 2-2-d1E03-1-0276 | C38 H31 FN4 O7 S2 | 738.16182 | -0.6 | 10.9 | 5.4 | 9.7 |
| 2-2-d1E03-1-0277 | C38 H31 FN4 O7 S2 | 738.16182 | -7.0 | 4.9 | 10.1 | 7.2 |
| 2-2-d1E03-1-0278 | C39 H38 N4 O7 S2 | 738.21819 | 1.7 | 6.5 | 6.8 | 7.0 |
| 2-2-d1E03-1-0279 | C37 H30 F N5 O7 S2 | 739.15707 | 0.9 | 6.9 | 5.8 | 4.7 |
| 2-2-d1E03-1-0280 | C37 H30 F N5 O7 S2 | 739.15707 | 11.4 | 17.5 | 18.1 | 6.9 |
| 2-2-d1E03-1-0281 | C37 H30 F N5 O7 S2 | 739.15707 | 0.0 | 0.0 | 0.0 | 0.0 |
| 2-2-d1E03-1-0282 | C41 H33 N5 O5 S2 | 739.19231 | 16.5 | 14.1 | 20.7 | 15.0 |
| 2-2-d1E03-1-0283 | C41 H33 N5 O5 S2 | 739.19231 | 4.2 | 23.5 | 14.4 | 13.5 |
| 2-2-d1E03-1-0284 | C38 H37 N5 O7 S2 | 739.21344 | 8.8 | 8.9 | 15.7 | 10.2 |
| 2-2-d1E03-1-0285 | C36 H32 N6 O8 S2 | 740.17230 | 0.0 | 0.0 | 0.0 | 0.0 |
| 2-2-d1E03-1-0286 | C38 H33 F N4 O7 S2 | 740.17747 | 0.2 | 6.0 | 6.4 | 14.8 |
| 2-2-d1E03-1-0287 | C38 H33 F N4 O7 S2 | 740.17747 | -2.8 | 6.0 | 7.2 | -3.0 |
| 2-2-d1E03-1-0288 | C40 H32 N6 O5 S2 | 740.18756 | 1.5 | 16.0 | 15.8 | 9.9 |
| 2-2-d1E03-1-0289 | C40 H32 N6 O5 S2 | 740.18756 | 7.7 | 11.5 | 17.5 | 8.2 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0290 | C40 H32 N6 O5 S2 | 740.18756 | 9.8 | 7.5 | 15.3 | 7.6 |
| 2-2-d1E03-1-0291 | C37 H36 N6 O7 S2 | 740.20869 | 2.5 | 6.7 | 7.6 | 5.0 |
| 2-2-d1E03-1-0292 | C37 H32 F N5 O7 S2 | 741.17272 | 1.4 | 4.4 | 5.7 | 2.9 |
| 2-2-d1E03-1-0293 | C36 H35 N7 O7 S2 | 741.20394 | 8.2 | 12.4 | 15.9 | 5.4 |
| 2-2-d1E03-1-0294 | C37 H34 N4 O7 S3 | 742.15896 | 3.8 | 2.9 | 6.7 | 8.9 |
| 2-2-d1E03-1-0295 | C36 H34 N6 O8 S2 | 742.18795 | 4.9 | 24.3 | 16.2 | 4.8 |
| 2-2-d1E03-1-0296 | C38 H38 N4 O8 S2 | 742.21311 | 2.9 | 4.9 | 12.7 | 5.1 |
| 2-2-d1E03-1-0297 | C40 H37 F3 N4 O5 S | 742.24368 | 2.4 | 15.1 | 21.6 | 8.4 |
| 2-2-d1E03-1-0298 | C42 H42 N6 O5 S | 742.29374 | 16.6 | 14.7 | 16.7 | 11.0 |
| 2-2-d1E03-1-0299 | C35 H29 N5 O8 S3 | 743.11783 | 3.1 | 3.3 | 8.2 | 8.3 |
| 2-2-d1E03-1-0300 | C36 H33 N5 O9 S2 | 743.17197 | -3.0 | 0.0 | 3.2 | 9.7 |
| 2-2-d1E03-1-0301 | C37 H34 F N5 O7 S2 | 743.18837 | 12.0 | 11.3 | 10.2 | 9.1 |
| 2-2-d1E03-1-0302 | C37 H36 N4 O7 S3 | 744.17461 | 4.5 | 5.8 | 7.6 | 4.5 |
| 2-2-d1E03-1-0303 | C36 H33 F N6 O7 S2 | 744.18362 | 2.5 | 5.7 | 7.3 | 5.5 |
| 2-2-d1E03-1-0304 | C37 H31 F3 N6 O6 S | 744.19779 | 36.4 | 12.0 | -9.8 | 57.8 |
| 2-2-d1E03-1-0305 | C40 H36 N6 O5 S2 | 744.21886 | 11.3 | 10.7 | 15.6 | 5.8 |
| 2-2-d1E03-1-0306 | C35 H31 N5 O8 S3 | 745.13348 | 0.0 | 0.0 | 11.6 | 8.4 |
| 2-2-d1E03-1-0307 | C39 H31 N5 O7 S2 | 745.16649 | 13.9 | 4.4 | 11.9 | 16.9 |
| 2-2-d1E03-1-0308 | C35 H32 F N7 O7 S2 | 745.17887 | 9.1 | 15.5 | 14.3 | 8.1 |
| 2-2-d1E03-1-0309 | C39 H35 N7 O5 S2 | 745.21411 | 5.6 | 5.5 | -1.9 | 14.4 |
| 2-2-d1E03-1-0310 | C36 H31 F N4 O7 S3 | 746.13389 | 0.0 | 0.0 | 0.0 | 0.0 |
| 2-2-d1E03-1-0311 | C35 H34 N6 O7 S3 | 746.16511 | 8.0 | 9.8 | 9.7 | 8.1 |
| 2-2-d1E03-1-0312 | C40 H34 N4 O7 S2 | 746.18689 | 5.1 | 55.5 | 11.9 | -56.5 |
| 2-2-d1E03-1-0313 | C40 H34 N4 O7 S2 | 746.18689 | 0.7 | 4.4 | 2.5 | 6.6 |
| 2-2-d1E03-1-0314 | C37 H35 F N4 O8 S2 | 746.18803 | 2.6 | 6.3 | 10.8 | 9.4 |
| 2-2-d1E03-1-0315 | C38 H34 N8 O5 S2 | 746.20936 | 5.9 | 10.2 | 15.8 | 7.6 |
| 2-2-d1E03-1-0316 | C37 H33 F3 N6 O6 S | 746.21344 | 13.6 | 1.0 | -2.8 | -2.9 |
| 2-2-d1E03-1-0317 | C35 H30 F N5 O9 S2 | 747.14690 | -2.2 | 5.0 | 12.2 | 8.9 |
| 2-2-d1E03-1-0318 | C39 H33 N5 O7 S2 | 747.18214 | 3.6 | 2.8 | 5.2 | 8.3 |
| 2-2-d1E03-1-0319 | C39 H33 N5 O7 S2 | 747.18214 | -3.6 | 0.3 | -12.6 | -10.7 |
| 2-2-d1E03-1-0320 | C39 H33 N5 O7 S2 | 747.18214 | 16.5 | 26.5 | 23.4 | -8.6 |
| 2-2-d1E03-1-0321 | C36 H37 N5 O7 S3 | 747.18551 | 1.8 | 1.4 | 6.6 | 4.6 |
| 2-2-d1E03-1-0322 | C40 H37 N5 O6 S2 | 747.21853 | 11.7 | 7.5 | 19.4 | 6.9 |
| 2-2-d1E03-1-0323 | C38 H35 F3 N4 O5 S2 | 748.20010 | 10.0 | 8.9 | 19.7 | 12.3 |
| 2-2-d1E03-1-0324 | C36 H30 F3 N5 O6 S2 | 749.15896 | 7.0 | 7.1 | 38.8 | 24.2 |
| 2-2-d1E03-1-0325 | C35 H35 N5 O8 S3 | 749.16478 | 4.1 | 3.3 | 7.9 | 5.4 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0326 | C34 H31 F N6 O7 S3 | 750.14004 | 5.9 | 6.3 | 10.5 | 12.5 |
| 2-2-d1E03-1-0327 | C41 H33 F3 N4 O5 S | 750.21238 | 4.5 | 15.5 | 16.0 | 11.3 |
| 2-2-d1E03-1-0328 | C41 H33 F3 N4 O5 S | 750.21238 | 11.0 | 6.3 | 30.3 | 9.1 |
| 2-2-d1E03-1-0329 | C37 H33 N7 O5 S3 | 751.17053 | 9.1 | 9.8 | 13.7 | 5.6 |
| 2-2-d1E03-1-0330 | C40 H32 F3 N5 O5 S | 751.20762 | 5.3 | -7.2 | 10.4 | 19.5 |
| 2-2-d1E03-1-0331 | C40 H32 F3 N5 O5 S | 751.20762 | 5.2 | 11.0 | 29.8 | 5.3 |
| 2-2-d1E03-1-0332 | C40 H32 F3 N5 O5 S | 751.20762 | -9.9 | 12.9 | 9.4 | 19.5 |
| 2-2-d1E03-1-0333 | C39 H37 N5 O7 S2 | 751.21344 | -0.8 | 18.4 | 16.5 | 20.8 |
| 2-2-d1E03-1-0334 | C38 H32 N4 O9 S2 | 752.16107 | 10.3 | 3.9 | 6.2 | 12.8 |
| 2-2-d1E03-1-0335 | C38 H36 N6 O7 S2 | 752.20869 | 34.0 | 2.4 | 19.1 | 26.0 |
| 2-2-d1E03-1-0336 | C40 H40 N4 O7 S2 | 752.23384 | -0.2 | 2.0 | 4.3 | 2.3 |
| 2-2-d1E03-1-0337 | C37 H31 N5 O7 S3 | 753.13856 | 0.2 | 3.3 | 6.6 | 13.5 |
| 2-2-d1E03-1-0338 | C37 H31 N5 O7 S3 | 753.13856 | 0.4 | 5.0 | 7.8 | 6.4 |
| 2-2-d1E03-1-0339 | C37 H35 N7 O7 S2 | 753.20394 | 10.8 | 5.4 | 12.4 | 15.5 |
| 2-2-d1E03-1-0340 | C36 H30 N6 O7 S3 | 754.13381 | 5.0 | 1.7 | -8.4 | -12.4 |
| 2-2-d1E03-1-0341 | C35 H29 F3 N4 O8 S2 | 754.13789 | 0.4 | 2.3 | 2.7 | 3.0 |
| 2-2-d1E03-1-0342 | C37 H34 N6 O8 S2 | 754.18795 | 0.0 | -42.0 | 27.9 | 42.9 |
| 2-2-d1E03-1-0343 | C39 H38 N4 O8 S2 | 754.21311 | 7.4 | 4.6 | -2.0 | 14.3 |
| 2-2-d1E03-1-0344 | C40 H36 F3 N5 O5 S | 755.23892 | 17.6 | -4.5 | 3.2 | 2.1 |
| 2-2-d1E03-1-0345 | C39 H31 F3 N4 O7 S | 756.18655 | 0.7 | 2.5 | 4.2 | 2.5 |
| 2-2-d1E03-1-0346 | C37 H36 N6 O8 S2 | 756.20360 | 14.7 | 13.8 | 8.1 | 4.4 |
| 2-2-d1E03-1-0347 | C39 H37 F N4 O7 S2 | 756.20877 | 2.0 | 4.6 | 3.7 | 2.0 |
| 2-2-d1E03-1-0348 | C39 H35 F3 N6 O5 S | 756.23417 | 5.9 | 10.5 | 12.0 | 17.4 |
| 2-2-d1E03-1-0349 | C43 H44 N6 O5 S | 756.30939 | 7.1 | 12.5 | 22.5 | 11.5 |
| 2-2-d1E03-1-0350 | C38 H34 F3 N7 O5 S | 757.22942 | 3.4 | 6.5 | 25.1 | 10.6 |
| 2-2-d1E03-1-0351 | C42 H39 N5 O5 S2 | 757.23926 | 6.7 | 6.6 | 17.9 | 11.7 |
| 2-2-d1E03-1-0352 | C36 H31 F N6 O8 S2 | 758.16288 | 52.6 | 9.8 | 0.0 | 1.8 |
| 2-2-d1E03-1-0353 | C36 H34 N6 O7 S3 | 758.16511 | 0.0 | 0.0 | 0.0 | 6.1 |
| 2-2-d1E03-1-0354 | C38 H38 N4 O7 S3 | 758.19026 | 5.2 | 6.3 | 8.0 | 7.2 |
| 2-2-d1E03-1-0355 | C36 H37 F3 N4 O7 S2 | 758.20558 | 0.7 | 4.0 | 4.7 | 3.1 |
| 2-2-d1E03-1-0356 | C40 H37 F3 N4 O6 S | 758.23859 | 9.0 | 8.4 | 39.0 | 10.3 |
| 2-2-d1E03-1-0357 | C35 H29 N5 O7 S4 | 759.09498 | 4.3 | 2.6 | 10.7 | 11.8 |
| 2-2-d1E03-1-0358 | C36 H33 N5 O8 S3 | 759.14913 | 8.4 | 13.0 | 6.9 | 1.9 |
| 2-2-d1E03-1-0359 | C34 H28 N6 O9 S3 | 760.10799 | 0.0 | 0.0 | 0.0 | 0.0 |
| 2-2-d1E03-1-0360 | C36 H33 F N6 O8 S2 | 760.17853 | 10.3 | 18.8 | 8.6 | 10.0 |
| 2-2-d1E03-1-0361 | C35 H35 F3 N4 O8 S2 | 760.18484 | 5.2 | 3.9 | 4.9 | 1.6 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0362 | C41 H36 N4 O7 S2 | 760.20254 | 3.9 | 2.2 | 8.1 | -0.8 |
| 2-2-d1E03-1-0363 | C41 H36 N4 O7 S2 | 760.20254 | 0.4 | 5.6 | 5.3 | 5.7 |
| 2-2-d1E03-1-0364 | C42 H41 F N6 O5 S | 760.28432 | 12.7 | 11.6 | 28.1 | 12.2 |
| 2-2-d1E03-1-0365 | C40 H35 N5 O7 S2 | 761.19779 | -1.5 | 4.5 | 7.7 | 11.8 |
| 2-2-d1E03-1-0366 | C40 H35 N5 O7 S2 | 761.19779 | 23.5 | 17.6 | 18.3 | 18.5 |
| 2-2-d1E03-1-0367 | C40 H35 N5 O7 S2 | 761.19779 | 15.5 | 4.7 | 0.0 | 0.0 |
| 2-2-d1E03-1-0368 | C37 H35 F N4 O7 S3 | 762.16519 | 2.1 | 9.5 | 4.9 | 4.0 |
| 2-2-d1E03-1-0369 | C37 H33 F3 N6 O5 S2 | 762.19059 | 10.5 | 21.5 | 14.1 | 14.8 |
| 2-2-d1E03-1-0370 | C35 H30 F N5 O8 S3 | 763.12405 | 0.0 | 10.2 | 0.0 | 0.0 |
| 2-2-d1E03-1-0371 | C40 H37 N5 O5 S3 | 763.19568 | 8.3 | 18.6 | 14.3 | 17.9 |
| 2-2-d1E03-1-0372 | C37 H31 F3 N4 O7 S2 | 764.15863 | 0.2 | 2.3 | 5.1 | 3.0 |
| 2-2-d1E03-1-0373 | C37 H31 F3 N4 O7 S2 | 764.15863 | 0.9 | 4.8 | 5.9 | 4.9 |
| 2-2-d1E03-1-0374 | C40 H33 F N4 O7 S2 | 764.17747 | 1.8 | 5.5 | 5.8 | 5.2 |
| 2-2-d1E03-1-0375 | C40 H33 F N4 O7 S2 | 764.17747 | 1.6 | -0.8 | 2.4 | 5.6 |
| 2-2-d1E03-1-0376 | C41 H40 N4 O7 S2 | 764.23384 | 4.9 | 2.1 | 2.8 | 1.4 |
| 2-2-d1E03-1-0377 | C36 H30 F3 N5 O7 S2 | 765.15387 | -1.3 | 3.7 | 6.9 | 5.0 |
| 2-2-d1E03-1-0378 | C39 H32 F N5 O7 S2 | 765.17272 | 17.5 | 14.6 | 18.3 | 8.6 |
| 2-2-d1E03-1-0379 | C39 H32 F N5 O7 S2 | 765.17272 | 0.0 | 0.0 | 52.1 | -34.1 |
| 2-2-d1E03-1-0380 | C39 H32 F N5 O7 S2 | 765.17272 | 0.0 | 0.0 | 0.0 | 0.0 |
| 2-2-d1E03-1-0381 | C40 H39 N5 O7 S2 | 765.22909 | 5.7 | 4.8 | 21.5 | 6.3 |
| 2-2-d1E03-1-0382 | C39 H34 N4 O9 S2 | 766.17672 | 4.9 | 6.5 | 13.7 | 7.8 |
| 2-2-d1E03-1-0383 | C39 H38 N6 O7 S2 | 766.22434 | 0.0 | -5.2 | 0.0 | -4.5 |
| 2-2-d1E03-1-0384 | C38 H37 N7 O7 S2 | 767.21959 | 39.9 | 19.0 | 23.2 | 36.4 |
| 2-2-d1E03-1-0385 | C40 H40 N4 O8 S2 | 768.22876 | 5.2 | 5.9 | 9.7 | 1.6 |
| 2-2-d1E03-1-0386 | C42 H39 F3 N4 O5 S | 768.25933 | 7.3 | 8.6 | 27.2 | 4.0 |
| 2-2-d1E03-1-0387 | C37 H31 N5 O8 S3 | 769.13348 | 4.3 | 3.4 | 9.6 | 3.1 |
| 2-2-d1E03-1-0388 | C39 H36 F N5 O7 S2 | 769.20402 | 0.0 | 0.0 | 0.0 | 0.0 |
| 2-2-d1E03-1-0389 | C35 H29 F3 N4 O7 S3 | 770.11505 | -1.5 | 3.0 | 5.3 | 1.7 |
| 2-2-d1E03-1-0390 | C38 H31 FN4 O9 S2 | 770.15165 | -0.4 | -1.5 | 21.8 | 19.1 |
| 2-2-d1E03-1-0391 | C39 H38 N4 O7 S3 | 770.19026 | 0.0 | 0.0 | 5.2 | 29.3 |
| 2-2-d1E03-1-0392 | C38 H35 F N6 O7 S2 | 770.19927 | 12.9 | 14.5 | 10.9 | 12.4 |
| 2-2-d1E03-1-0393 | C35 H29 N7 O8 S3 | 771.12397 | 1.1 | -10.4 | -4.6 | 8.2 |
| 2-2-d1E03-1-0394 | C34 H28 F3 N5 O9 S2 | 771.12805 | 6.0 | -0.6 | 16.7 | 23.2 |
| 2-2-d1E03-1-0395 | C41 H33 N5 O7 S2 | 771.18214 | 0.0 | 0.0 | 0.0 | 0.0 |
| 2-2-d1E03-1-0396 | C37 H34 F N7 O7 S2 | 771.19452 | 5.9 | 8.8 | 19.7 | 11.8 |
| 2-2-d1E03-1-0397 | C37 H36 N6 O7 S3 | 772.18076 | 0.0 | 0.0 | 0.0 | 0.0 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0398 | C39 H37 F N4 O8 S2 | 772.20368 | 3.1 | 6.5 | 8.7 | 6.0 |
| 2-2-d1E03-1-0399 | C35 H31 N7 O8 S3 | 773.13962 | 4.4 | 6.8 | 7.5 | 8.5 |
| 2-2-d1E03-1-0400 | C38 H39 N5 O7 S3 | 773.20116 | 1.3 | 2.8 | 5.3 | 3.6 |
| 2-2-d1E03-1-0401 | C41 H39 N7 O5 S2 | 773.24541 | 5.0 | 12.4 | 18.7 | 9.9 |
| 2-2-d1E03-1-0402 | C40 H37 F3 N4 O5 S2 | 774.21575 | -0.4 | 11.5 | 11.0 | 21.4 |
| 2-2-d1E03-1-0403 | C37 H37 N5 O8 S3 | 775.18043 | 3.0 | 7.5 | 6.9 | 0.0 |
| 2-2-d1E03-1-0404 | C34 H28 N6 O8 S4 | 776.08514 | 8.0 | 5.9 | 16.3 | 10.7 |
| 2-2-d1E03-1-0405 | C36 H33 F N6 O7 S3 | 776.15569 | 31.8 | 23.0 | 0.0 | 28.6 |
| 2-2-d1E03-1-0406 | C39 H31 N5 O7 S3 | 777.13856 | 2.7 | 7.5 | 8.3 | 3.7 |
| 2-2-d1E03-1-0407 | C39 H31 N5 O7 S3 | 777.13856 | 0.0 | 0.0 | 0.0 | 0.0 |
| 2-2-d1E03-1-0408 | C38 H30 N6 O7 S3 | 778.13381 | 2.3 | 2.7 | 4.3 | 7.2 |
| 2-2-d1E03-1-0409 | C38 H30 N6 O7 S3 | 778.13381 | 8.3 | 11.0 | 22.2 | 12.1 |
| 2-2-d1E03-1-0410 | C38 H30 N6 O7 S3 | 778.13381 | 2.3 | 2.7 | 4.3 | 7.2 |
| 2-2-d1E03-1-0411 | C40 H34 N4 O9 S2 | 778.17672 | 14.8 | 9.4 | 10.9 | 11.7 |
| 2-2-d1E03-1-0412 | C42 H42 N4 O7 S2 | 778.24949 | 18.7 | 8.4 | 0.0 | 0.0 |
| 2-2-d1E03-1-0413 | C39 H33 N5 O7 S3 | 779.15421 | 36.8 | 11.9 | 6.6 | 3.6 |
| 2-2-d1E03-1-0414 | C39 H33 N5 O7 S3 | 779.15421 | -0.7 | 3.3 | 6.6 | 3.6 |
| 2-2-d1E03-1-0415 | C38 H32 N6 O7 S3 | 780.14946 | 0.3 | 4.6 | 6.4 | 3.1 |
| 2-2-d1E03-1-0416 | C37 H31 F3 N4 O8 S2 | 780.15354 | 7.8 | 9.0 | 6.6 | 8.9 |
| 2-2-d1E03-1-0417 | C40 H40 N6 O7 S2 | 780.23999 | 34.2 | 33.3 | 44.4 | 38.0 |
| 2-2-d1E03-1-0418 | C35 H29 F3 N6 O8 S2 | 782.14404 | 16.0 | 29.9 | 5.5 | 1.7 |
| 2-2-d1E03-1-0419 | C38 H34 N6 O7 S3 | 782.16511 | -1.2 | 7.9 | 15.8 | 12.0 |
| 2-2-d1E03-1-0420 | C41 H33 F3 N4 O7 S | 782.20220 | 10.0 | -7.9 | 22.3 | 10.1 |
| 2-2-d1E03-1-0421 | C41 H39 F N4 O7 S2 | 782.22442 | 0.5 | 6.0 | 4.7 | 1.9 |
| 2-2-d1E03-1-0422 | C37 H33 N7 O7 S3 | 783.16036 | 2.1 | 4.2 | 7.2 | 0.9 |
| 2-2-d1E03-1-0423 | C36 H32 N8 O7 S3 | 784.15561 | 7.7 | 9.5 | 10.5 | 11.6 |
| 2-2-d1E03-1-0424 | C35 H31 F3 N6 O8 S2 | 784.15969 | 2.8 | 6.4 | 5.3 | 5.6 |
| 2-2-d1E03-1-0425 | C40 H40 N4 O7 S3 | 784.20591 | 2.5 | 1.7 | 6.3 | 4.5 |
| 2-2-d1E03-1-0426 | C38 H39 F3 N4 O7 S2 | 784.22123 | 0.0 | 0.0 | 1.5 | 12.0 |
| 2-2-d1E03-1-0427 | C41 H39 F3 N6 O5 S | 784.26547 | 6.2 | 12.3 | 15.6 | 9.6 |
| 2-2-d1E03-1-0428 | C37 H31 N5 O7 S4 | 785.11063 | 2.8 | 7.5 | 7.7 | 7.8 |
| 2-2-d1E03-1-0429 | C38 H35 N5 O8 S3 | 785.16478 | -0.4 | 6.1 | 5.4 | 6.2 |
| 2-2-d1E03-1-0430 | C36 H30 N6 O9 S3 | 786.12364 | 24.9 | -3.3 | 10.3 | -13.1 |
| 2-2-d1E03-1-0431 | C37 H37 F3 N4 O8 S2 | 786.20049 | 1.6 | -0.5 | 1.5 | 0.9 |
| 2-2-d1E03-1-0432 | C34 H28 F3 N5 O8 S3 | 787.10521 | -1.2 | 2.1 | 7.6 | 8.4 |
| 2-2-d1E03-1-0433 | C39 H31 F3 N4 O7 S2 | 788.15863 | -0.4 | 10.9 | 2.5 | 8.3 |

(continued)

| [Table 11-3-3] | | | | | | |
|---|---|---|---|---|---|---|
| | | | AS-MS(%) | | | |
| ID | Molecular Formula | Exact Mass | N1 | N2 | N3 | N4 |
| 2-2-d1E03-1-0434 | C39 H31 F3 N4 O7 S2 | 788.15863 | -3.4 | 3.9 | -9.9 | 8.1 |
| 2-2-d1E03-1-0435 | C39 H37 F N4 O7 S3 | 788.18084 | 5.9 | 9.2 | 3.3 | 4.5 |
| 2-2-d1E03-1-0436 | C35 H31 N7 O7 S4 | 789.11678 | 2.6 | 7.9 | 8.5 | 6.1 |
| 2-2-d1E03-1-0437 | C38 H30 F3 N5 O7 S2 | 789.15387 | 13.4 | 13.5 | 21.0 | 10.1 |
| 2-2-d1E03-1-0438 | C38 H30 F3 N5 O7 S2 | 789.15387 | 1.2 | 13.2 | -0.7 | 9.2 |
| 2-2-d1E03-1-0439 | C38 H30 F3 N5 O7 S2 | 789.15387 | -5.8 | -1.0 | 0.6 | 0.0 |
| 2-2-d1E03-1-0440 | C39 H33 F3 N4 O7 S2 | 790.17428 | 14.5 | 8.5 | -4.9 | 6.0 |
| 2-2-d1E03-1-0441 | C39 H33 F3 N4 O7 S2 | 790.17428 | 2.0 | 3.1 | 3.4 | -8.2 |
| 2-2-d1E03-1-0442 | C38 H32 F3 N5 O7 S2 | 791.16952 | -2.9 | 2.6 | 7.5 | 11.4 |
| 2-2-d1E03-1-0443 | C41 H36 N4 O9 S2 | 792.19237 | 4.4 | 9.3 | 8.4 | 1.3 |
| 2-2-d1E03-1-0444 | C38 H34 F3 N5 O7 S2 | 793.18517 | 2.8 | 2.5 | 7.1 | 0.7 |
| 2-2-d1E03-1-0445 | C37 H33 F3 N6 O7 S2 | 794.18042 | -0.4 | 2.5 | 8.5 | 4.3 |
| 2-2-d1E03-1-0446 | C41 H42 N6 O7 S2 | 794.25564 | 37.6 | 15.8 | 38.9 | 49.5 |
| 2-2-d1E03-1-0447 | C36 H32 F3 N7 O7 S2 | 795.17567 | 8.2 | 18.8 | 23.2 | 14.3 |
| 2-2-d1E03-1-0448 | C40 H37 N5 O7 S3 | 795.18551 | 0.1 | 1.7 | 5.8 | 3.8 |
| 2-2-d1E03-1-0449 | C37 H31 F3 N4 O7 S3 | 796.13070 | 12.3 | 6.3 | 2.3 | 3.5 |
| 2-2-d1E03-1-0450 | C40 H33 F N4 O9 S2 | 796.16730 | -4.5 | 2.2 | 13.6 | 7.7 |
| 2-2-d1E03-1-0451 | C38 H35 F3 N4 O8 S2 | 796.18484 | 0.7 | 0.7 | 4.2 | 5.5 |
| 2-2-d1E03-1-0452 | C37 H31 N7 O8 S3 | 797.13962 | 5.5 | 33.1 | 2.3 | 0.3 |
| 2-2-d1E03-1-0453 | C36 H30 F3 N5 O9 S2 | 797.14370 | 2.0 | -5.3 | 9.8 | -5.9 |
| 2-2-d1E03-1-0454 | C40 H39 F N6 O7 S2 | 798.23057 | 34.7 | 33.4 | 39.2 | 38.2 |
| 2-2-d1E03-1-0455 | C37 H33 N7 O8 S3 | 799.15527 | 8.3 | 11.7 | 12.8 | 29.2 |
| 2-2-d1E03-1-0456 | C43 H41 N7 O5 S2 | 799.26106 | 11.3 | 12.0 | 20.0 | 8.2 |
| 2-2-d1E03-1-0457 | C35 H31 F3 N6 O7 S3 | 800.13684 | 5.1 | 5.5 | 5.9 | 2.6 |
| 2-2-d1E03-1-0458 | C38 H35 N5 O7 S4 | 801.14193 | 2.2 | 5.4 | 4.9 | 4.6 |
| 2-2-d1E03-1-0459 | C36 H30 N6 O8 S4 | 802.10079 | 4.0 | 8.7 | 5.8 | 5.5 |
| 2-2-d1E03-1-0460 | C41 H33 N5 O7 S3 | 803.15421 | 2.3 | 4.6 | 7.8 | 5.3 |
| 2-2-d1E03-1-0461 | C41 H33 N5 O7 S3 | 803.15421 | 0.0 | 0.0 | 0.0 | 0.0 |
| 2-2-d1E03-1-0462 | C40 H32 N6 O7 S3 | 804.14946 | 4.7 | 9.7 | 8.7 | 7.6 |
| 2-2-d1E03-1-0463 | C40 H32 N6 O7 S3 | 804.14946 | 21.6 | 21.0 | 13.6 | 7.4 |
| 2-2-d1E03-1-0464 | C40 H32 N6 O7 S3 | 804.14946 | 4.7 | 9.7 | 8.7 | 7.6 |
| 2-2-d1E03-1-0465 | C40 H37 F3 N4 O7 S2 | 806.20558 | -2.2 | 2.2 | 7.0 | 5.3 |
| 2-2-d1E03-1-0466 | C42 H42 N6 O7 S2 | 806.25564 | 6.7 | 11.6 | 9.0 | 11.0 |
| 2-2-d1E03-1-0467 | C37 H31 F3 N6 O8 S2 | 808.15969 | -15.7 | -2.1 | 15.8 | -18.0 |
| 2-2-d1E03-1-0468 | C40 H36 N6 O7 S3 | 808.18076 | 4.0 | 6.5 | 4.4 | 3.7 |
| 2-2-d1E03-1-0469 | C39 H31 N5 O9 S3 | 809.12839 | 13.0 | 33.4 | 15.0 | 2.9 |

(continued)

[Table 11-3-3]

| | | | AS-MS(%) | | | |
|---|---|---|---|---|---|---|
| ID | Molecular Formula | Exact Mass | N1 | N2 | N3 | N4 |
| 2-2-d1E03-1-0470 | C39 H35 N7 O7 S3 | 809.17601 | 7.1 | 15.5 | 15.4 | 2.5 |
| 2-2-d1E03-1-0471 | C38 H34 N8 O7 S3 | 810.17126 | 23.5 | 14.6 | 23.6 | 17.4 |
| 2-2-d1E03-1-0472 | C37 H33 F3 N6 O8 S2 | 810.17534 | 6.5 | 7.5 | 12.2 | 3.8 |
| 2-2-d1E03-1-0473 | C43 H41 F3 N6 O5 S | 810.28112 | 13.9 | 10.1 | 8.6 | 12.2 |
| 2-2-d1E03-1-0474 | C40 H37 N5 O8 S3 | 811.18043 | 2.3 | 5.1 | 6.3 | 2.6 |
| 2-2-d1E03-1-0475 | C38 H35 F3 N4 O7 S3 | 812.16200 | -5.0 | 9.0 | 4.3 | 4.2 |
| 2-2-d1E03-1-0476 | C36 H30 F3 N5 O8 S3 | 813.12086 | 28.1 | 18.0 | -1.8 | -3.7 |
| 2-2-d1E03-1-0477 | C41 H33 F3 N4 O7 S2 | 814.17428 | 13.4 | 7.7 | 25.1 | 18.2 |
| 2-2-d1E03-1-0478 | C41 H33 F3 N4 O7 S2 | 814.17428 | 0.0 | 0.0 | 0.0 | 0.0 |
| 2-2-d1E03-1-0479 | C37 H33 N7 O7 S4 | 815.13243 | 9.4 | 13.4 | 11.9 | 6.5 |
| 2-2-d1E03-1-0480 | C40 H32 F3 N5 O7 S2 | 815.16952 | 4.7 | 9.6 | 9.0 | 5.7 |
| 2-2-d1E03-1-0481 | C40 H32 F3 N5 O7 S2 | 815.16952 | 17.1 | 17.0 | 12.4 | 14.2 |
| 2-2-d1E03-1-0482 | C40 H32 F3 N5 O7 S2 | 815.16952 | 0.0 | 0.0 | 0.0 | 0.0 |
| 2-2-d1E03-1-0483 | C40 H36 F3 N5 O7 S2 | 819.20082 | -2.7 | 13.2 | 8.2 | 4.7 |
| 2-2-d1E03-1-0484 | C39 H31 F3 N4 O9 S2 | 820.14845 | 1.4 | 2.6 | 13.0 | 3.2 |
| 2-2-d1E03-1-0485 | C39 H35 F3 N6 O7 S2 | 820.19607 | -16.4 | 13.0 | -1.3 | 11.2 |
| 2-2-d1E03-1-0486 | C43 H44 N6 O7 S2 | 820.27129 | 6.1 | 14.2 | 18.6 | 20.6 |
| 2-2-d1E03-1-0487 | C38 H34 F3 N7 O7 S2 | 821.19132 | 16.4 | 12.8 | 19.6 | 8.5 |
| 2-2-d1E03-1-0488 | C42 H39 N5 O7 S3 | 821.20116 | 0.5 | 8.3 | 3.7 | 4.9 |
| 2-2-d1E03-1-0489 | C40 H37 F3 N4 O8 S2 | 822.20049 | 23.4 | 10.2 | -7.7 | 9.0 |
| 2-2-d1E03-1-0490 | C42 H41 F N6 O7 S2 | 824.24622 | 3.8 | 4.5 | 12.7 | 11.6 |
| 2-2-d1E03-1-0491 | C37 H33 F3 N6 O7 S3 | 826.15249 | 12.2 | 19.1 | 23.2 | 3.7 |
| 2-2-d1E03-1-0492 | C40 H37 N5 O7 S4 | 827.15758 | 0.5 | 1.5 | 5.1 | 2.1 |
| 2-2-d1E03-1-0493 | C42 H39 F3 N4 O7 S2 | 832.22123 | 7.5 | -4.7 | 8.2 | -1.3 |
| 2-2-d1E03-1-0494 | C41 H33 N5 O9 S3 | 835.14404 | 1.3 | 6.6 | 6.6 | 8.5 |
| 2-2-d1E03-1-0495 | C41 H39 N7 O7 S3 | 837.20731 | 28.9 | 38.1 | 35.6 | 39.3 |
| 2-2-d1E03-1-0496 | C40 H37 F3 N4 O7 S3 | 838.17765 | 32.2 | -3.8 | -0.2 | 3.7 |
| 2-2-d1E03-1-0497 | C41 H33 F3 N4 O9 S2 | 846.16410 | 1.4 | 6.1 | -1.9 | 8.3 |
| 2-2-d1E03-1-0498 | C41 H39 F3 N6 O7 S2 | 848.22737 | 10.0 | 18.2 | 19.8 | 18.3 |
| 2-2-d1E03-1-0499 | C43 H41 N7 O7 S3 | 863.22296 | 10.7 | 7.9 | 4.4 | 17.0 |
| 2-2-d1E03-1-0500 | C43 H41 F3 N6 O7 S2 | 874.24302 | 1.5 | 3.6 | 9.6 | 5.8 |

[Table 601]

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0001 | C40 H41 N5 O7 S | 735.27267 | -5.4 | -0.1 | -1.0 | -7.8 |
| 2-2-d1E04-1-0002 | C41 H49 N5 O6 S | 739.34036 | -7.9 | -4.3 | -2.7 | -10.5 |
| 2-2-d1E04-1-0003 | C40 H47 N5 O7 S | 741.31962 | -7.9 | -3.8 | -4.2 | -7.7 |
| 2-2-d1E04-1-0004 | C42 H43 N5 O6 S | 745.29340 | -1.4 | -2.1 | 2.9 | -5.6 |
| 2-2-d1E04-1-0005 | C42 H43 N5 O6 S | 745.29340 | -3.7 | -0.9 | 0.9 | -6.7 |
| 2-2-d1E04-1-0006 | C41 H42 N6 O6 S | 746.28865 | -26.5 | -7.8 | -4.2 | -8.0 |
| 2-2-d1E04-1-0007 | C41 H43 N5 O7 S | 749.28832 | -6.4 | -1.8 | 3.6 | -4.0 |
| 2-2-d1E04-1-0008 | C40 H41 N5 O6 S2 | 751.24983 | -7.2 | 0.7 | 2.1 | -7.3 |
| 2-2-d1E04-1-0009 | C39 H40 N6 O8 S | 752.26283 | -6.9 | -2.5 | -2.3 | -8.1 |
| 2-2-d1E04-1-0010 | C40 H40 F N5 O7 S | 753.26325 | -7.2 | -2.2 | -0.9 | -8.2 |
| 2-2-d1E04-1-0011 | C42 H51 N5 O6 S | 753.35601 | -7.0 | -3.2 | -2.0 | -7.3 |
| 2-2-d1E04-1-0012 | C41 H49 N5 O7 S | 755.33527 | -7.6 | -6.0 | -1.9 | -8.2 |
| 2-2-d1E04-1-0013 | C41 H48 F N5 O6 S | 757.33093 | -7.0 | 0.6 | -1.9 | -9.7 |
| 2-2-d1E04-1-0014 | C43 H45 N5 O6 S | 759.30905 | -3.3 | 1.8 | 3.7 | -2.9 |
| 2-2-d1E04-1-0015 | C43 H45 N5 O6 S | 759.30905 | -8.1 | -1.9 | -3.5 | -7.3 |
| 2-2-d1E04-1-0016 | C40 H46 F N5 O7 S | 759.31020 | -3.1 | 0.9 | 0.1 | -6.3 |
| 2-2-d1E04-1-0017 | C42 H44 N6 O6 S | 760.30430 | -6.1 | -2.4 | -0.1 | -7.0 |
| 2-2-d1E04-1-0018 | C42 H43 N5 O7 S | 761.28832 | -4.0 | -3.1 | 5.9 | -7.2 |
| 2-2-d1E04-1-0019 | C40 H41 N7 O7 S | 763.27882 | -7.6 | -3.8 | -2.6 | -5.9 |
| 2-2-d1E04-1-0020 | C42 H42 F N5 O6 S | 763.28398 | -6.6 | 2.3 | 6.6 | -8.0 |
| 2-2-d1E04-1-0021 | C42 H42 F N5 O6 S | 763.28398 | -2.4 | 3.7 | 2.8 | -2.6 |
| 2-2-d1E04-1-0022 | C41 H41 F N6 O6 S | 764.27923 | -8.9 | -10.0 | -6.3 | -5.3 |
| 2-2-d1E04-1-0023 | C41 H43 N5 O6 S2 | 765.26548 | -4.5 | 1.1 | 2.8 | -2.4 |
| 2-2-d1E04-1-0024 | C40 H43 N7 O7 S | 765.29447 | -6.4 | 0.4 | 0.3 | -4.7 |
| 2-2-d1E04-1-0025 | C43 H51 N5 O6 S | 765.35601 | -8.4 | -6.1 | -0.3 | -7.6 |
| 2-2-d1E04-1-0026 | C40 H42 N6 O8 S | 766.27848 | -6.9 | -3.2 | 0.3 | -8.1 |
| 2-2-d1E04-1-0027 | C42 H49 N5 O7 S | 767.33527 | -8.1 | -4.8 | -2.4 | -9.9 |
| 2-2-d1E04-1-0028 | C39 H40 N6 O7 S2 | 768.23999 | -6.1 | -2.1 | 2.9 | -7.9 |
| 2-2-d1E04-1-0029 | C40 H40 F N5 O6 S2 | 769.24040 | -4.3 | 0.7 | -0.3 | -7.4 |
| 2-2-d1E04-1-0030 | C44 H43 N5 O6 S | 769.29340 | -3.5 | 1.9 | 4.0 | -4.1 |
| 2-2-d1E04-1-0031 | C44 H43 N5 O6 S | 769.29340 | -2.9 | 3.0 | 2.9 | -4.4 |
| 2-2-d1E04-1-0032 | C39 H39 F N6 O8 S | 770.25341 | -8.9 | -2.5 | -1.6 | -9.8 |
| 2-2-d1E04-1-0033 | C43 H42 N6 O6 S | 770.28865 | -3.8 | 2.8 | 5.1 | 9.7 |
| 2-2-d1E04-1-0034 | C43 H42 N6 O6 S | 770.28865 | -5.8 | 1.0 | 0.4 | -7.8 |
| 2-2-d1E04-1-0035 | C43 H42 N6 O6 S | 770.28865 | 0.0 | 0.0 | 0.0 | 0.0 |
| 2-2-d1E04-1-0036 | C44 H45 N5 O6 S | 771.30905 | -4.8 | 0.5 | 1.4 | 1.8 |
| 2-2-d1E04-1-0037 | C44 H45 N5 O6 S | 771.30905 | -4.8 | 0.5 | 4.5 | 7.9 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0038 | C43 H44 N6 O6 S | 772.30430 | -7.6 | 0.1 | -5.5 | -30.5 |
| 2-2-d1E04-1-0039 | C43 H46 N6 O6 S | 774.31995 | -6.7 | -1.8 | 14.6 | -3.9 |
| 2-2-d1E04-1-0040 | C43 H45 N5 O7 S | 775.30397 | 1.6 | 0.7 | -1.8 | -13.6 |
| 2-2-d1E04-1-0041 | C42 H45 N7 O6 S | 775.31520 | -2.9 | 1.6 | 17.4 | 3.9 |
| 2-2-d1E04-1-0042 | C41 H44 N8 O6 S | 776.31045 | -7.0 | -0.6 | 1.9 | -3.5 |
| 2-2-d1E04-1-0043 | C42 H43 N5 O6 S2 | 777.26548 | 0.0 | 0.0 | 0.0 | 0.0 |
| 2-2-d1E04-1-0044 | C41 H43 N7 O7 S | 777.29447 | -6.7 | -4.5 | -2.5 | -8.8 |
| 2-2-d1E04-1-0045 | C43 H47 N5 O7 S | 777.31962 | -4.3 | 0.0 | 1.5 | -5.6 |
| 2-2-d1E04-1-0046 | C41 H42 N6 O8 S | 778.27848 | -7.3 | 0.9 | -0.5 | -5.5 |
| 2-2-d1E04-1-0047 | C42 H42 F N5 O7 S | 779.27890 | -5.2 | 0.8 | 4.1 | -4.7 |
| 2-2-d1E04-1-0048 | C41 H45 N7 O7 S | 779.31012 | -7.4 | -2.0 | 0.5 | -9.6 |
| 2-2-d1E04-1-0049 | C44 H53 N5 O6 S | 779.37166 | -3.6 | -3.6 | 1.3 | -11.4 |
| 2-2-d1E04-1-0050 | C40 H40 F N7 O7 S | 781.26940 | -8.0 | -1.7 | -2.8 | -7.9 |
| 2-2-d1E04-1-0051 | C40 H43 N7 O6 S2 | 781.27162 | -7.6 | 0.8 | -1.2 | -11.8 |
| 2-2-d1E04-1-0052 | C43 H51 N5 O7 S | 781.35092 | -7.6 | -2.3 | -2.9 | -8.2 |
| 2-2-d1E04-1-0053 | C40 H42 N6 O7 S2 | 782.25564 | -5.1 | -0.4 | 2.0 | -4.4 |
| 2-2-d1E04-1-0054 | C40 H42 F N7 O7 S | 783.28505 | -5.1 | -0.7 | 0.2 | -6.2 |
| 2-2-d1E04-1-0055 | C45 H45 N5 O6 S | 783.30905 | -2.2 | 0.8 | 4.2 | -3.3 |
| 2-2-d1E04-1-0056 | C45 H45 N5 O6 S | 783.30905 | -2.7 | 3.5 | 1.6 | -2.9 |
| 2-2-d1E04-1-0057 | C43 H50 F N5 O6 S | 783.34658 | -7.7 | -2.6 | 0.5 | -8.2 |
| 2-2-d1E04-1-0058 | C44 H44 N6 O6 S | 784.30430 | -2.4 | 3.6 | 3.0 | -3.3 |
| 2-2-d1E04-1-0059 | C44 H44 N6 O6 S | 784.30430 | -4.1 | 2.1 | 5.0 | -6.4 |
| 2-2-d1E04-1-0060 | C44 H44 N6 O6 S | 784.30430 | -2.0 | 2.7 | -5.7 | -4.0 |
| 2-2-d1E04-1-0061 | C45 H47 N5 O6 S | 785.32470 | -4.0 | 3.8 | 4.8 | -4.4 |
| 2-2-d1E04-1-0062 | C45 H47 N5 O6 S | 785.32470 | 0.0 | 0.0 | 0.0 | 0.0 |
| 2-2-d1E04-1-0063 | C42 H48 F N5 O7 S | 785.32585 | -7.4 | -3.5 | -0.5 | -8.4 |
| 2-2-d1E04-1-0064 | C39 H39 F N6 O7 S2 | 786.23057 | -11.4 | -0.9 | -1.0 | -3.0 |
| 2-2-d1E04-1-0065 | C44 H46 N6 O6 S | 786.31995 | -6.5 | -1.6 | 1.9 | -10.1 |
| 2-2-d1E04-1-0066 | C44 H42 F N5 O6 S | 787.28398 | -3.9 | 3.4 | 3.5 | -4.3 |
| 2-2-d1E04-1-0067 | C44 H42 F N5 O6 S | 787.28398 | -3.6 | 0.7 | 0.9 | -3.4 |
| 2-2-d1E04-1-0068 | C45 H49 N5 O6 S | 787.34036 | -6.7 | 2.0 | 0.9 | -6.9 |
| 2-2-d1E04-1-0069 | C43 H41 F N6 O6 S | 788.27923 | -3.5 | 3.9 | 3.1 | -4.5 |
| 2-2-d1E04-1-0070 | C43 H41 F N6 O6 S | 788.27923 | -2.7 | 1.9 | -0.6 | -7.0 |
| 2-2-d1E04-1-0071 | C43 H41 F N6 O6 S | 788.27923 | -7.7 | 0.4 | 0.0 | 5.9 |
| 2-2-d1E04-1-0072 | C44 H48 N6 O6 S | 788.33560 | -4.4 | 3.1 | 2.7 | -5.8 |
| 2-2-d1E04-1-0073 | C42 H43 N7 O7 S | 789.29447 | -2.2 | 0.0 | -2.0 | -5.4 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0074 | C44 H44 F N5 O6 S | 789.29963 | -3.9 | 0.9 | 2.7 | -5.2 |
| 2-2-d1E04-1-0075 | C44 H44 F N5 O6 S | 789.29963 | -8.4 | -0.7 | 2.7 | -9.3 |
| 2-2-d1E04-1-0076 | C43 H47 N7 O6 S | 789.33085 | -5.2 | 6.1 | 9.0 | -2.7 |
| 2-2-d1E04-1-0077 | C43 H43 F N6 O6 S | 790.29488 | -6.9 | 0.4 | -3.4 | -5.1 |
| 2-2-d1E04-1-0078 | C42 H46 N8 O6 S | 790.32610 | -7.7 | -1.5 | 1.9 | -7.7 |
| 2-2-d1E04-1-0079 | C43 H45 N5 O6 S2 | 791.28113 | -16.2 | -11.1 | -3.7 | 8.3 |
| 2-2-d1E04-1-0080 | C42 H45 N7 O7 S | 791.31012 | 9.4 | 2.7 | 11.4 | 16.6 |
| 2-2-d1E04-1-0081 | C44 H49 N5 O7 S | 791.33527 | -4.1 | 1.4 | 2.0 | -7.1 |
| 2-2-d1E04-1-0082 | C41 H40 N6 O7 S2 | 792.23999 | -0.9 | 0.6 | 4.3 | -3.2 |
| 2-2-d1E04-1-0083 | C42 H44 N6 O8 S | 792.29413 | -8.5 | -4.3 | 4.1 | -10.8 |
| 2-2-d1E04-1-0084 | C43 H45 F N6 O6 S | 792.31053 | -9.7 | -6.8 | -1.3 | 2.8 |
| 2-2-d1E04-1-0085 | C43 H47 N5 O6 S2 | 793.29678 | -4.6 | 1.6 | 2.1 | -6.1 |
| 2-2-d1E04-1-0086 | C42 H44 F N7 O6 S | 793.30578 | -6.6 | -0.3 | 0.6 | -8.1 |
| 2-2-d1E04-1-0087 | C41 H42 N6 O7 S2 | 794.25564 | 1.7 | 4.7 | 6.8 | -19.8 |
| 2-2-d1E04-1-0088 | C41 H43 F N8 O6 S | 794.30103 | -4.5 | 1.0 | 2.6 | -6.3 |
| 2-2-d1E04-1-0089 | C42 H42 F N5 O6 S2 | 795.25605 | -3.4 | 4.3 | 1.9 | -7.7 |
| 2-2-d1E04-1-0090 | C41 H45 N7 O6 S2 | 795.28727 | -9.5 | 3.9 | 9.1 | 4.4 |
| 2-2-d1E04-1-0091 | C46 H45 N5 O6 S | 795.30905 | -0.1 | 38.0 | 22.4 | 8.7 |
| 2-2-d1E04-1-0092 | C46 H45 N5 O6 S | 795.30905 | -2.6 | 3.4 | 1.8 | -2.6 |
| 2-2-d1E04-1-0093 | C43 H46 F N5 O7 S | 795.31020 | -4.2 | -1.6 | 0.3 | -4.1 |
| 2-2-d1E04-1-0094 | C41 H41 F N6 O8 S | 796.26906 | -6.6 | -3.9 | 0.2 | -15.5 |
| 2-2-d1E04-1-0095 | C45 H44 N6 O6 S | 796.30430 | -1.2 | 10.9 | -1.9 | -0.8 |
| 2-2-d1E04-1-0096 | C45 H44 N6 O6 S | 796.30430 | -3.8 | -0.6 | 5.7 | -0.5 |
| 2-2-d1E04-1-0097 | C45 H44 N6 O6 S | 796.30430 | 0.0 | -3.2 | 5.7 | -0.5 |
| 2-2-d1E04-1-0098 | C42 H48 N6 O6 S2 | 796.30767 | -6.3 | 2.0 | -7.0 | -9.7 |
| 2-2-d1E04-1-0099 | C41 H46 N6 O7 S2 | 798.28694 | -10.0 | -4.0 | -1.6 | -9.9 |
| 2-2-d1E04-1-0100 | C40 H41 N5 O9 S2 | 799.23457 | -4.9 | -1.5 | -10.6 | -17.3 |
| 2-2-d1E04-1-0101 | C40 H42 F N7 O6 S2 | 799.26220 | -8.4 | -1.1 | 9.7 | 0.1 |
| 2-2-d1E04-1-0102 | C45 H48 N6 O6 S | 800.33560 | 0.0 | 0.0 | 0.0 | 0.0 |
| 2-2-d1E04-1-0103 | C44 H43 N5 O8 S | 801.28323 | -1.9 | 2.4 | 2.2 | -6.0 |
| 2-2-d1E04-1-0104 | C44 H47 N7 O6 S | 801.33085 | 19.3 | 4.5 | 0.0 | 0.0 |
| 2-2-d1E04-1-0105 | C46 H51 N5 O6 S | 801.35601 | -2.8 | 1.4 | 3.1 | -4.4 |
| 2-2-d1E04-1-0106 | C43 H42 N6 O6 S2 | 802.26072 | -2.7 | 2.2 | 3.0 | -1.1 |
| 2-2-d1E04-1-0107 | C43 H42 N6 O6 S2 | 802.26072 | -3.8 | 1.2 | -0.9 | -6.2 |
| 2-2-d1E04-1-0108 | C43 H46 N8 O6 S | 802.32610 | -15.5 | 1.1 | -5.2 | -4.1 |
| 2-2-d1E04-1-0109 | C42 H41 N7 O6 S2 | 803.25597 | -3.0 | 3.8 | 3.7 | -26.0 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0110 | C41 H40 F3 N5 O7 S | 803.26005 | -5.9 | 1.0 | 1.8 | -6.1 |
| 2-2-d1E04-1-0111 | C41 H49 N5 O8 S2 | 803.30225 | -3.9 | -0.4 | -1.4 | -5.8 |
| 2-2-d1E04-1-0112 | C43 H45 N7 O7 S | 803.31012 | -16.3 | -21.8 | 13.2 | -3.8 |
| 2-2-d1E04-1-0113 | C45 H49 N5 O7 S | 803.33527 | -4.3 | 1.7 | 2.2 | -11.0 |
| 2-2-d1E04-1-0114 | C40 H47 N5 O9 S2 | 805.28152 | -3.3 | 0.5 | -2.6 | -5.1 |
| 2-2-d1E04-1-0115 | C43 H47 N7 O7 S | 805.32577 | -8.6 | 4.2 | 4.2 | 17.3 |
| 2-2-d1E04-1-0116 | C45 H48 F N5 O6 S | 805.33093 | -3.9 | 2.0 | 3.4 | -3.1 |
| 2-2-d1E04-1-0117 | C42 H42 F N7 O7 S | 807.28505 | -3.1 | 1.4 | 5.7 | -2.1 |
| 2-2-d1E04-1-0118 | C42 H45 N7 O6 S2 | 807.28727 | -9.0 | 0.1 | 2.1 | -9.0 |
| 2-2-d1E04-1-0119 | C44 H49 N5 O6 S2 | 807.31243 | -11.5 | -4.4 | -2.8 | -4.5 |
| 2-2-d1E04-1-0120 | C42 H48 F3 N5 O6 S | 807.32774 | -6.7 | -7.5 | -1.5 | -3.6 |
| 2-2-d1E04-1-0121 | C41 H40 N6 O6 S3 | 808.21715 | -2.1 | 3.2 | 1.3 | -3.8 |
| 2-2-d1E04-1-0122 | C42 H44 N6 O7 S2 | 808.27129 | -1.5 | 0.2 | -5.2 | 0.1 |
| 2-2-d1E04-1-0123 | C40 H39 N7 O8 S2 | 809.23015 | -8.9 | -2.2 | 0.5 | -1.4 |
| 2-2-d1E04-1-0124 | C42 H43 N5 O8 S2 | 809.25530 | -3.5 | 3.7 | 1.7 | -4.2 |
| 2-2-d1E04-1-0125 | C42 H43 N5 O8 S2 | 809.25530 | -5.6 | -1.9 | -1.5 | -6.2 |
| 2-2-d1E04-1-0126 | C42 H44 F N7 O7 S | 809.30070 | -5.6 | -4.7 | 1.8 | -16.5 |
| 2-2-d1E04-1-0127 | C41 H46 F3 N5 O7 S | 809.30700 | -6.6 | -1.2 | 0.1 | -6.7 |
| 2-2-d1E04-1-0128 | C47 H47 N5 O6 S | 809.32470 | -1.9 | 4.4 | 3.3 | -2.8 |
| 2-2-d1E04-1-0129 | C47 H47 N5 O6 S | 809.32470 | -13.3 | -9.0 | -6.0 | 19.2 |
| 2-2-d1E04-1-0130 | C41 H42 N6 O8 S2 | 810.25055 | -3.2 | -0.5 | -0.3 | -5.5 |
| 2-2-d1E04-1-0131 | C46 H46 N6 O6 S | 810.31995 | -5.4 | 3.6 | 1.0 | -6.7 |
| 2-2-d1E04-1-0132 | C46 H46 N6 O6 S | 810.31995 | -15.2 | 8.4 | 3.8 | 6.1 |
| 2-2-d1E04-1-0133 | C46 H46 N6 O6 S | 810.31995 | -3.2 | 6.2 | 7.7 | -5.8 |
| 2-2-d1E04-1-0134 | C43 H46 F N5 O6 S2 | 811.28735 | -2.8 | 4.6 | 4.4 | -4.5 |
| 2-2-d1E04-1-0135 | C41 H41 FN6 O7 S2 | 812.24622 | -6.9 | 1.9 | 0.8 | -6.1 |
| 2-2-d1E04-1-0136 | C41 H43 N5 O9 S2 | 813.25022 | -6.0 | -2.7 | -1.7 | -7.8 |
| 2-2-d1E04-1-0137 | C43 H42 F3 N5 O6 S | 813.28079 | -2.0 | 3.5 | 4.3 | 3.7 |
| 2-2-d1E04-1-0138 | C43 H42 F3 N5 O6 S | 813.28079 | -1.3 | 1.9 | 9.2 | -3.1 |
| 2-2-d1E04-1-0139 | C46 H44 F N5 O6 S | 813.29963 | -1.9 | 4.6 | 4.9 | -2.2 |
| 2-2-d1E04-1-0140 | C46 H44 F N5 O6 S | 813.29963 | 6.9 | 16.5 | -2.4 | 10.5 |
| 2-2-d1E04-1-0141 | C47 H51 N5 O6 S | 813.35601 | -9.1 | 5.1 | 14.9 | -8.1 |
| 2-2-d1E04-1-0142 | C42 H41 F3 N6 O6 S | 814.27604 | 39.1 | -0.6 | -5.4 | -30.0 |
| 2-2-d1E04-1-0143 | C45 H43 F N6 O6 S | 814.29488 | -1.9 | -16.0 | -14.5 | -7.3 |
| 2-2-d1E04-1-0144 | C45 H43 F N6 O6 S | 814.29488 | 4.9 | 2.0 | 3.8 | -2.4 |
| 2-2-d1E04-1-0145 | C45 H43 F N6 O6 S | 814.29488 | 8.5 | 6.0 | -4.5 | -3.1 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0146 | C46 H50 N6 O6 S | 814.35125 | -54.3 | -9.0 | 0.0 | 0.0 |
| 2-2-d1E04-1-0147 | C40 H41 N5 O8 S3 | 815.21173 | -4.7 | -1.4 | -0.3 | -7.9 |
| 2-2-d1E04-1-0148 | C45 H45 N5 O8 S | 815.29888 | -2.8 | 4.6 | 1.8 | -3.0 |
| 2-2-d1E04-1-0149 | C45 H49 N7 O6 S | 815.34650 | 0.0 | 0.0 | 23.3 | -3.4 |
| 2-2-d1E04-1-0150 | C39 H40 N6 O10 S2 | 816.22473 | -6.3 | -6.6 | -3.3 | -3.9 |
| 2-2-d1E04-1-0151 | C44 H48 N8 O6 S | 816.34175 | -9.5 | 0.4 | 10.3 | -13.5 |
| 2-2-d1E04-1-0152 | C40 H40 F N5 O9 S2 | 817.22515 | -5.9 | -1.3 | -2.6 | -5.9 |
| 2-2-d1E04-1-0153 | C42 H51 N5 O8 S2 | 817.31791 | -5.0 | -1.1 | -1.1 | -5.4 |
| 2-2-d1E04-1-0154 | C46 H51 N5 O7 S | 817.35092 | -1.8 | 3.4 | 0.0 | 8.3 |
| 2-2-d1E04-1-0155 | C43 H42 N6 O7 S2 | 818.25564 | -3.9 | 1.2 | 2.0 | -3.1 |
| 2-2-d1E04-1-0156 | C45 H47 F N6 O6 S | 818.32618 | -0.7 | -17.6 | 11.4 | -7.7 |
| 2-2-d1E04-1-0157 | C41 H40 F3 N5 O6 S2 | 819.23721 | -2.5 | 4.5 | 2.6 | -3.5 |
| 2-2-d1E04-1-0158 | C44 H42 F N5 O8 S | 819.27381 | -5.2 | 1.9 | 4.6 | -7.3 |
| 2-2-d1E04-1-0159 | C41 H49 N5 O9 S2 | 819.29717 | -3.9 | 10.9 | -2.1 | -5.3 |
| 2-2-d1E04-1-0160 | C45 H49 N5 O6 S2 | 819.31243 | -0.5 | 6.1 | 4.9 | -4.4 |
| 2-2-d1E04-1-0161 | C44 H46 F N7 O6 S | 819.32143 | 0.0 | -3.6 | -5.2 | -2.8 |
| 2-2-d1E04-1-0162 | C41 H40 N8 O7 S2 | 820.24614 | -5.9 | 3.3 | -2.2 | -4.9 |
| 2-2-d1E04-1-0163 | C40 H39 F3 N6 O8 S | 820.25022 | -5.0 | -2.6 | 1.4 | -5.5 |
| 2-2-d1E04-1-0164 | C43 H45 F N8 O6 S | 820.31668 | -3.5 | 6.5 | 3.3 | -4.0 |
| 2-2-d1E04-1-0165 | C41 H48 F N5 O8 S2 | 821.29283 | -4.7 | -10.8 | -2.8 | -8.9 |
| 2-2-d1E04-1-0166 | C43 H47 N7 O6 S2 | 821.30292 | -6.0 | -5.5 | 0.0 | 14.8 |
| 2-2-d1E04-1-0167 | C45 H48 F N5 O7 S | 821.32585 | -4.0 | 2.7 | 2.7 | -6.3 |
| 2-2-d1E04-1-0168 | C41 H42 N8 O7 S2 | 822.26179 | -3.7 | 2.6 | 9.8 | -15.9 |
| 2-2-d1E04-1-0169 | C44 H50 N6 O6 S2 | 822.32332 | -5.2 | 31.4 | 6.5 | -4.9 |
| 2-2-d1E04-1-0170 | C43 H45 N5 O8 S2 | 823.27095 | -3.2 | 0.1 | 2.5 | -6.0 |
| 2-2-d1E04-1-0171 | C43 H45 N5 O8 S2 | 823.27095 | -2.9 | 0.3 | -1.6 | -3.3 |
| 2-2-d1E04-1-0172 | C40 H46 F N5 O9 S2 | 823.27210 | -6.1 | -1.2 | 0.7 | -4.2 |
| 2-2-d1E04-1-0173 | C42 H44 N6 O8 S2 | 824.26620 | -3.9 | -0.2 | 1.0 | -4.9 |
| 2-2-d1E04-1-0174 | C43 H48 N6 O7 S2 | 824.30259 | -8.2 | -1.6 | -1.4 | 7.9 |
| 2-2-d1E04-1-0175 | C40 H39 N7 O7 S3 | 825.20731 | -3.6 | 0.9 | -2.3 | 5.7 |
| 2-2-d1E04-1-0176 | C42 H43 N5 O9 S2 | 825.25022 | -6.5 | -2.0 | -3.7 | -7.5 |
| 2-2-d1E04-1-0177 | C42 H44 F N7 O6 S2 | 825.27785 | -1.7 | -0.2 | -1.8 | 2.4 |
| 2-2-d1E04-1-0178 | C45 H42 N6 O6 S2 | 826.26072 | -0.1 | 3.9 | 4.0 | -0.1 |
| 2-2-d1E04-1-0179 | C45 H42 N6 O6 S2 | 826.26072 | -0.1 | 4.0 | -1.3 | -0.1 |
| 2-2-d1E04-1-0180 | C40 H41 N7 O9 S2 | 827.24072 | -5.4 | -1.4 | 6.8 | 38.5 |
| 2-2-d1E04-1-0181 | C42 H42 F N5 O8 S2 | 827.24588 | -1.1 | 2.1 | 0.2 | -9.0 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0182 | C42 H42 F N5 O8 S2 | 827.24588 | -6.0 | -2.1 | 3.3 | -3.5 |
| 2-2-d1E04-1-0183 | C44 H41 N7 O6 S2 | 827.25597 | -9.5 | 38.6 | -8.8 | -11.8 |
| 2-2-d1E04-1-0184 | C44 H41 N7 O6 S2 | 827.25597 | 2.9 | 3.2 | 6.5 | -1.2 |
| 2-2-d1E04-1-0185 | C44 H41 N7 O6 S2 | 827.25597 | -6.1 | -2.9 | 8.4 | 5.0 |
| 2-2-d1E04-1-0186 | C46 H45 N5 O8 S | 827.29888 | -2.7 | 2.4 | 0.1 | -6.8 |
| 2-2-d1E04-1-0187 | C48 H53 N5 O6 S | 827.37166 | -3.2 | 5.2 | 7.6 | -10.8 |
| 2-2-d1E04-1-0188 | C41 H41 F N6 O8 S2 | 828.24113 | -3.3 | -0.2 | -0.3 | -5.3 |
| 2-2-d1E04-1-0189 | C45 H44 N6 O6 S2 | 828.27637 | 1.1 | -5.0 | 5.3 | -1.9 |
| 2-2-d1E04-1-0190 | C45 H44 N6 O6 S2 | 828.27637 | 0.0 | 0.0 | 0.0 | 0.0 |
| 2-2-d1E04-1-0191 | C41 H43 N5 O8 S3 | 829.22738 | -6.4 | -1.7 | -0.5 | -6.3 |
| 2-2-d1E04-1-0192 | C40 H43 N7 O9 S2 | 829.25637 | -5.0 | 1.0 | -4.5 | 0.2 |
| 2-2-d1E04-1-0193 | C44 H43 N7 O6 S2 | 829.27162 | -17.5 | 13.5 | 2.2 | 7.5 |
| 2-2-d1E04-1-0194 | C43 H42 F3 N5 O7 S | 829.27570 | -4.3 | 4.6 | 3.9 | 3.2 |
| 2-2-d1E04-1-0195 | C43 H51 N5 O8 S2 | 829.31791 | -3.9 | -5.3 | -3.7 | -8.9 |
| 2-2-d1E04-1-0196 | C46 H51 N7 O6 S | 829.36215 | -4.2 | 0.4 | 3.5 | -2.8 |
| 2-2-d1E04-1-0197 | C40 H42 N6 O10 S2 | 830.24038 | -6.9 | -9.5 | -6.9 | -15.4 |
| 2-2-d1E04-1-0198 | C41 H40 F3 N7 O7 S | 831.26620 | -6.4 | -0.7 | -0.8 | -6.7 |
| 2-2-d1E04-1-0199 | C44 H45 N7 O6 S2 | 831.28727 | -4.1 | 5.4 | -13.9 | 40.8 |
| 2-2-d1E04-1-0200 | C42 H49 N5 O9 S2 | 831.29717 | -4.0 | 13.1 | -1.7 | -4.3 |
| 2-2-d1E04-1-0201 | C47 H50 F N5 O6 S | 831.34658 | -2.7 | 0.8 | 4.0 | 0.0 |
| 2-2-d1E04-1-0202 | C39 H40 N6 O9 S3 | 832.20189 | -7.2 | -3.0 | -3.7 | -8.0 |
| 2-2-d1E04-1-0203 | C43 H44 N8 O6 S2 | 832.28252 | 13.6 | -6.4 | 0.8 | 2.2 |
| 2-2-d1E04-1-0204 | C40 H40 F N5 O8 S3 | 833.20230 | -7.8 | -0.8 | -2.1 | -6.3 |
| 2-2-d1E04-1-0205 | C44 H43 N5 O8 S2 | 833.25530 | -10.1 | -0.3 | -2.8 | -0.8 |
| 2-2-d1E04-1-0206 | C44 H43 N5 O8 S2 | 833.25530 | 2.7 | 10.5 | 10.5 | 7.4 |
| 2-2-d1E04-1-0207 | C42 H43 N9 O6 S2 | 833.27777 | -73.1 | 1.3 | 1.4 | -13.4 |
| 2-2-d1E04-1-0208 | C41 H42 F3 N7 O7 S | 833.28185 | -3.4 | 1.7 | 3.3 | -5.4 |
| 2-2-d1E04-1-0209 | C46 H51 N5 O6 S2 | 833.32808 | 0.9 | 6.3 | 0.4 | -3.1 |
| 2-2-d1E04-1-0210 | C44 H50 F3 N5 O6 S | 833.34339 | -27.8 | -3.2 | 1.7 | -4.8 |
| 2-2-d1E04-1-0211 | C39 H39 F N6 O10 S2 | 834.21531 | -11.1 | -3.7 | -5.7 | -7.8 |
| 2-2-d1E04-1-0212 | C43 H42 N6 O6 S3 | 834.23280 | -3.6 | 6.3 | 5.3 | -2.7 |
| 2-2-d1E04-1-0213 | C43 H42 N6 O8 S2 | 834.25055 | -8.9 | -20.0 | -7.8 | -16.4 |
| 2-2-d1E04-1-0214 | C43 H42 N6 O8 S2 | 834.25055 | -4.4 | 0.2 | 0.3 | -8.3 |
| 2-2-d1E04-1-0215 | C43 H42 N6 O8 S2 | 834.25055 | 0.0 | 0.0 | 0.0 | 0.0 |
| 2-2-d1E04-1-0216 | C44 H46 N6 O7 S2 | 834.28694 | -5.8 | 3.4 | 1.6 | -4.0 |
| 2-2-d1E04-1-0217 | C42 H41 N7 O8 S2 | 835.24580 | -1.0 | -1.2 | 6.0 | -7.0 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0218 | C44 H45 N5 O8 S2 | 835.27095 | -4.5 | -5.4 | -2.5 | -16.0 |
| 2-2-d1E04-1-0219 | C44 H45 N5 O8 S2 | 835.27095 | 17.4 | 29.1 | 0.0 | 0.0 |
| 2-2-d1E04-1-0220 | C43 H48 F3 N5 O7 S | 835.32265 | -7.1 | -2.8 | -2.7 | -6.5 |
| 2-2-d1E04-1-0221 | C40 H39 F3 N6 O7 S2 | 836.22737 | -6.4 | 0.9 | -1.1 | -4.9 |
| 2-2-d1E04-1-0222 | C43 H44 N6 O8 S2 | 836.26620 | -5.5 | -2.1 | -0.1 | -6.8 |
| 2-2-d1E04-1-0223 | C45 H42 F3 N5 O6 S | 837.28079 | 3.9 | 6.1 | 7.3 | -4.7 |
| 2-2-d1E04-1-0224 | C45 H42 F3 N5 O6 S | 837.28079 | -3.6 | 4.8 | 8.6 | -0.3 |
| 2-2-d1E04-1-0225 | C45 H48 F N5 O6 S2 | 837.30300 | -2.9 | 3.0 | 3.5 | -1.3 |
| 2-2-d1E04-1-0226 | C41 H42 N8 O6 S3 | 838.23894 | -2.4 | -0.6 | 0.1 | -3.5 |
| 2-2-d1E04-1-0227 | C44 H41 F3 N6 O6 S | 838.27604 | -1.4 | 10.4 | 1.5 | 2.3 |
| 2-2-d1E04-1-0228 | C44 H41 F3 N6 O6 S | 838.27604 | -2.5 | 3.9 | 3.4 | -7.3 |
| 2-2-d1E04-1-0229 | C44 H41 F3 N6 O6 S | 838.27604 | -0.3 | 6.2 | 13.9 | 4.5 |
| 2-2-d1E04-1-0230 | C43 H46 N6 O8 S2 | 838.28185 | 2.0 | 9.6 | 4.9 | -2.6 |
| 2-2-d1E04-1-0231 | C43 H45 N5 O9 S2 | 839.26587 | -7.2 | -3.0 | -1.4 | -6.2 |
| 2-2-d1E04-1-0232 | C42 H45 N7 O8 S2 | 839.27710 | -5.7 | 3.1 | 7.4 | 4.7 |
| 2-2-d1E04-1-0233 | C45 H44 F3 N5 O6 S | 839.29644 | -7.5 | 1.9 | 4.3 | -2.4 |
| 2-2-d1E04-1-0234 | C45 H44 F3 N5 O6 S | 839.29644 | 0.3 | -0.7 | -2.9 | -6.3 |
| 2-2-d1E04-1-0235 | C41 H44 N8 O8 S2 | 840.27235 | -0.1 | -1.4 | -4.9 | -3.1 |
| 2-2-d1E04-1-0236 | C44 H43 F3 N6 O6 S | 840.29169 | -2.2 | 3.4 | 1.8 | -6.2 |
| 2-2-d1E04-1-0237 | C42 H43 N5 O8 S3 | 841.22738 | 0.2 | 2.9 | 9.2 | -2.1 |
| 2-2-d1E04-1-0238 | C41 H43 N7 O9 S2 | 841.25637 | -8.7 | -1.6 | -5.5 | -12.4 |
| 2-2-d1E04-1-0239 | C43 H47 N5 O9 S2 | 841.28152 | -3.5 | 0.3 | 1.3 | -4.2 |
| 2-2-d1E04-1-0240 | C47 H47 N5 O8 S | 841.31453 | -2.3 | 2.4 | 4.5 | -3.5 |
| 2-2-d1E04-1-0241 | C41 H42 N6 O10 S2 | 842.24038 | -10.3 | -5.9 | -1.0 | -8.3 |
| 2-2-d1E04-1-0242 | C44 H45 F3 N6 O6 S | 842.30734 | -0.6 | 0.0 | 3.3 | -0.2 |
| 2-2-d1E04-1-0243 | C42 H42 F N5 O9 S2 | 843.24080 | -5.7 | -3.1 | -1.0 | -7.7 |
| 2-2-d1E04-1-0244 | C41 H45 N7 O9 S2 | 843.27202 | -5.9 | -0.4 | -1.6 | -6.2 |
| 2-2-d1E04-1-0245 | C43 H44 F3 N7 O6 S | 843.30259 | -5.7 | 2.1 | 4.0 | -2.0 |
| 2-2-d1E04-1-0246 | C44 H53 N5 O8 S2 | 843.33356 | -4.3 | -2.6 | -2.4 | -6.2 |
| 2-2-d1E04-1-0247 | C47 H53 N7 O6 S | 843.37780 | -4.5 | 1.0 | 2.9 | -0.2 |
| 2-2-d1E04-1-0248 | C42 H43 F3 N8 O6 S | 844.29784 | -4.3 | 1.0 | 1.8 | -3.3 |
| 2-2-d1E04-1-0249 | C46 H48 N6 O6 S2 | 844.30767 | -7.0 | 27.5 | 6.2 | -1.2 |
| 2-2-d1E04-1-0250 | C40 H40 F N7 O9 S2 | 845.23130 | -2.8 | -4.1 | -11.0 | -1.1 |
| 2-2-d1E04-1-0251 | C40 H43 N7 O8 S3 | 845.23352 | -1.7 | 0.0 | 1.8 | -2.5 |
| 2-2-d1E04-1-0252 | C43 H42 F3 N5 O6 S2 | 845.25286 | -3.9 | 3.3 | 6.0 | 1.2 |
| 2-2-d1E04-1-0253 | C46 H44 F N5 O8 S | 845.28946 | -1.7 | 1.3 | 4.2 | -6.2 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0254 | C44 H46 F3 N5 O7 S | 845.30700 | -2.1 | -0.3 | 4.0 | -2.7 |
| 2-2-d1E04-1-0255 | C43 H51 N5 O9 S2 | 845.31282 | -1.6 | -0.1 | -1.1 | -3.4 |
| 2-2-d1E04-1-0256 | C40 H42 N6 O9 53 | 846.21754 | -7.0 | -4.7 | -1.8 | -8.4 |
| 2-2-d1E04-1-0257 | C43 H42 N8 O7 S2 | 846.26179 | -13.1 | 6.0 | 2.0 | -2.9 |
| 2-2-d1E04-1-0258 | C42 H41 F3 N6 O8 S | 846.26587 | -3.7 | -1.6 | 1.4 | -2.0 |
| 2-2-d1E04-1-0259 | C40 H42 F N7 O9 S2 | 847.24695 | -3.1 | 1.5 | -0.6 | -5.4 |
| 2-2-d1E04-1-0260 | C45 H45 N5 O8 S2 | 847.27095 | -4.7 | 1.3 | 1.7 | -6.2 |
| 2-2-d1E04-1-0261 | C45 H45 N5 O8 S2 | 847.27095 | -4.7 | 1.5 | -2.7 | -4.8 |
| 2-2-d1E04-1-0262 | C43 H50 F N5 O8 S2 | 847.30848 | -6.2 | -2.5 | -6.2 | -9.1 |
| 2-2-d1E04-1-0263 | C46 H50 F N7 O6 S | 847.35273 | -8.0 | -1.9 | 1.9 | -8.9 |
| 2-2-d1E04-1-0264 | C44 H44 N6 O8 S2 | 848.26620 | -6.3 | 1.5 | 0.0 | -6.6 |
| 2-2-d1E04-1-0265 | C44 H44 N6 O8 S2 | 848.26620 | -5.7 | 0.9 | -1.8 | -7.7 |
| 2-2-d1E04-1-0266 | C44 H44 N6 O8 S2 | 848.26620 | 0.0 | -15.3 | -8.6 | -10.0 |
| 2-2-d1E04-1-0267 | C43 H44 N8 O7 S2 | 848.27744 | -5.9 | -3.9 | 1.2 | 0.4 |
| 2-2-d1E04-1-0268 | C41 H42 F3 N7 O6 S2 | 849.25901 | -2.4 | 4.5 | -0.3 | -3.7 |
| 2-2-d1E04-1-0269 | C45 H47 N5 O8 S2 | 849.28660 | -6.9 | -4.5 | -1.3 | -12.4 |
| 2-2-d1E04-1-0270 | C45 H47 N5 O8 S2 | 849.28660 | -3.5 | -0.4 | -1.4 | -6.6 |
| 2-2-d1E04-1-0271 | C42 H48 F N5 O9 S2 | 849.28775 | -3.5 | -0.7 | -1.0 | -6.2 |
| 2-2-d1E04-1-0272 | C39 H39 F N6 O9 S3 | 850.19247 | -6.9 | -4.1 | -2.6 | -9.7 |
| 2-2-d1E04-1-0273 | C44 H46 N6 O6 S3 | 850.26410 | -3.6 | 1.8 | 4.1 | -1.6 |
| 2-2-d1E04-1-0274 | C44 H46 N6 O8 S2 | 850.28185 | -4.9 | -34.9 | -14.6 | 0.1 |
| 2-2-d1E04-1-0275 | C42 H41 N7 O7 S3 | 851.22296 | -2.0 | 1.0 | 1.3 | -8.9 |
| 2-2-d1E04-1-0276 | C44 H42 F N5 O8 S2 | 851.24588 | -1.8 | 4.7 | 4.1 | 4.4 |
| 2-2-d1E04-1-0277 | C44 H42 F N5 O8 S2 | 851.24588 | 0.0 | 0.0 | 0.0 | 20.4 |
| 2-2-d1E04-1-0278 | C45 H49 N5 O8 S2 | 851.30225 | -4.4 | -1.4 | -1.6 | -6.9 |
| 2-2-d1E04-1-0279 | C43 H41 F N6 O8 S2 | 852.24113 | -4.8 | -0.5 | -2.1 | -6.2 |
| 2-2-d1E04-1-0280 | C43 H41 F N6 O8 S2 | 852.24113 | -5.3 | -1.3 | 0.3 | -8.3 |
| 2-2-d1E04-1-0281 | C43 H41 F N6 O8 S2 | 852.24113 | 0.0 | 0.0 | 0.0 | 0.0 |
| 2-2-d1E04-1-0282 | C47 H44 N6 O6 S2 | 852.27637 | -1.5 | 2.9 | 4.7 | -3.0 |
| 2-2-d1E04-1-0283 | C47 H44 N6 O6 S2 | 852.27637 | 4.7 | 23.0 | -5.8 | 10.8 |
| 2-2-d1E04-1-0284 | C44 H48 N6 O8 S2 | 852.29750 | -1.6 | 3.5 | 7.6 | -0.4 |
| 2-2-d1E04-1-0285 | C42 H43 N7 O9 S2 | 853.25637 | -17.3 | -300.5 | 0.0 | -282.8 |
| 2-2-d1E04-1-0286 | C44 H44 F N5 O8 S2 | 853.26153 | -7.8 | -3.9 | -8.8 | -4.6 |
| 2-2-d1E04-1-0287 | C44 H44 F N5 O8 S2 | 853.26153 | -4.0 | -1.8 | -0.7 | -3.5 |
| 2-2-d1E04-1-0288 | C46 H43 N7 O6 S2 | 853.27162 | -13.3 | 2.9 | -2.7 | 0.1 |
| 2-2-d1E04-1-0289 | C46 H43 N7 O6 S2 | 853.27162 | 6.3 | 4.7 | 0.4 | -6.5 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0290 | C46 H43 N7 O6 S2 | 853.27162 | -9.6 | 1.9 | 4.2 | 2.5 |
| 2-2-d1E04-1-0291 | C43 H47 N7 O8 S2 | 853.29275 | -7.0 | -2.9 | -1.8 | -7.4 |
| 2-2-d1E04-1-0292 | C43 H43 F N6 O8 S2 | 854.25678 | -4.7 | -1.7 | 1.1 | -4.7 |
| 2-2-d1E04-1-0293 | C42 H46 N8 O8 S2 | 854.28800 | -2.6 | 3.7 | 2.0 | -6.8 |
| 2-2-d1E04-1-0294 | C43 H45 N5 O8 S3 | 855.24303 | -6.4 | -0.5 | -23.1 | -24.8 |
| 2-2-d1E04-1-0295 | C42 H45 N7 O9 S2 | 855.27202 | -9.9 | 5.3 | 0.2 | 14.9 |
| 2-2-d1E04-1-0296 | C44 H49 N5 O9 S2 | 855.29717 | -5.8 | -2.3 | 3.0 | -5.3 |
| 2-2-d1E04-1-0297 | C46 H48 F3 N5 O6 S | 855.32774 | -3.7 | 2.3 | 9.1 | -0.8 |
| 2-2-d1E04-1-0298 | C48 H53 N7 O6 S | 855.37780 | 4.0 | 2.9 | -2.9 | -11.6 |
| 2-2-d1E04-1-0299 | C41 H40 N6 O9 S3 | 856.20189 | -5.9 | -3.1 | 2.0 | 1.9 |
| 2-2-d1E04-1-0300 | C42 H44 N6 O10 S2 | 856.25603 | 13.3 | 0.0 | -3.7 | -14.5 |
| 2-2-d1E04-1-0301 | C43 H45 F N6 O8 S2 | 856.27243 | 1.6 | 8.6 | 8.9 | 0.8 |
| 2-2-d1E04-1-0302 | C43 H47 N5 O8 S3 | 857.25868 | 0.5 | 4.4 | 5.5 | -1.7 |
| 2-2-d1E04-1-0303 | C42 H44 F N7 O8 S2 | 857.26768 | -1.9 | -0.1 | 0.6 | -3.8 |
| 2-2-d1E04-1-0304 | C43 H42 F3 N7 O7 S | 857.28185 | -5.8 | 3.5 | 1.0 | 8.2 |
| 2-2-d1E04-1-0305 | C46 H47 N7 O6 S2 | 857.30292 | -7.5 | -1.6 | -5.0 | 5.7 |
| 2-2-d1E04-1-0306 | C41 H42 N6 O9 S3 | 858.21754 | -9.7 | -4.6 | -16.5 | -31.3 |
| 2-2-d1E04-1-0307 | C45 H42 N6 O8 S2 | 858.25055 | 3.2 | -15.1 | 4.1 | -0.2 |
| 2-2-d1E04-1-0308 | C41 H43 F N8 O8 S2 | 858.26293 | -1.9 | 0.0 | 0.4 | -10.2 |
| 2-2-d1E04-1-0309 | C45 H46 N8 O6 S2 | 858.29817 | -10.5 | 4.9 | 8.5 | 5.0 |
| 2-2-d1E04-1-0310 | C42 H42 F N5 O8 S3 | 859.21795 | -1.4 | -10.1 | -6.2 | -19.7 |
| 2-2-d1E04-1-0311 | C41 H45 N7 O8 S3 | 859.24917 | -5.3 | -1.2 | -0.6 | -6.7 |
| 2-2-d1E04-1-0312 | C46 H45 N5 O8 S2 | 859.27095 | -6.1 | -0.8 | -0.6 | -6.8 |
| 2-2-d1E04-1-0313 | C46 H45 N5 O8 S2 | 859.27095 | 7.5 | 13.1 | -4.5 | -29.1 |
| 2-2-d1E04-1-0314 | C43 H46 F N5 O9 S2 | 859.27210 | -5.1 | 21.2 | 3.0 | -8.7 |
| 2-2-d1E04-1-0315 | C44 H45 N9 O6 S2 | 859.29342 | 0.0 | 12.6 | 0.1 | -13.3 |
| 2-2-d1E04-1-0316 | C43 H44 F3 N7 O7 S | 859.29750 | -9.2 | 23.0 | -11.8 | -13.0 |
| 2-2-d1E04-1-0317 | C41 H41 F N6 O10 S2 | 860.23096 | -6.7 | -6.0 | -3.8 | -10.2 |
| 2-2-d1E04-1-0318 | C45 H44 N6 O8 S2 | 860.26620 | -6.8 | -0.3 | -2.8 | -7.1 |
| 2-2-d1E04-1-0319 | C45 H44 N6 O8 S2 | 860.26620 | -3.0 | 4.1 | 1.6 | -7.8 |
| 2-2-d1E04-1-0320 | C45 H44 N6 O8 S2 | 860.26620 | 1.6 | -4.2 | -1.3 | 12.0 |
| 2-2-d1E04-1-0321 | C42 H48 N6 O8 S3 | 860.26957 | -1.7 | -0.5 | 0.8 | 10.0 |
| 2-2-d1E04-1-0322 | C46 H48 N6 O7 S2 | 860.30259 | -2.0 | 7.2 | -2.0 | -3.7 |
| 2-2-d1E04-1-0323 | C44 H46 F3 N5 O6 S2 | 861.28416 | -2.3 | 4.1 | 5.4 | -0.4 |
| 2-2-d1E04-1-0324 | C42 H41 F3 N6 O7 S2 | 862.24302 | -5.0 | 1.4 | 1.6 | -6.5 |
| 2-2-d1E04-1-0325 | C41 H46 N6 O9 S3 | 862.24884 | -9.4 | -4.9 | -4.7 | -11.8 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0326 | C40 H42 F N7 O8 S3 | 863.22410 | -2.6 | 2.5 | -0.2 | -7.2 |
| 2-2-d1E04-1-0327 | C47 H44 F3 N5 O6 S | 863.29644 | -2.4 | 3.5 | 5.5 | 0.1 |
| 2-2-d1E04-1-0328 | C47 H44 F3 N5 O6 S | 863.29644 | -10.1 | 2.9 | -19.2 | 0.0 |
| 2-2-d1E04-1-0329 | C43 H44 N8 O6 S3 | 864.25459 | 1.0 | 6.2 | 8.2 | 1.8 |
| 2-2-d1E04-1-0330 | C46 H43 F3 N6 O6 S | 864.29169 | -3.8 | 3.8 | 5.4 | -4.0 |
| 2-2-d1E04-1-0331 | C46 H43 F3 N6 O6 S | 864.29169 | -6.9 | 1.1 | 9.1 | -5.1 |
| 2-2-d1E04-1-0332 | C46 H43 F3 N6 O6 S | 864.29169 | -1.4 | 6.6 | -0.5 | -6.7 |
| 2-2-d1E04-1-0333 | C45 H48 N6 O8 S2 | 864.29750 | -3.0 | -1.5 | 5.3 | 0.0 |
| 2-2-d1E04-1-0334 | C44 H43 N5 O10 S2 | 865.24513 | 1.7 | 7.8 | 7.7 | -0.6 |
| 2-2-d1E04-1-0335 | C44 H47 N7 O8 S2 | 865.29275 | 8.3 | 1.7 | -1.0 | 10.3 |
| 2-2-d1E04-1-0336 | C46 H51 N5 O8 S2 | 865.31791 | -7.1 | -0.9 | -2.0 | -6.4 |
| 2-2-d1E04-1-0337 | C43 H42 N6 O8 S3 | 866.22262 | -2.7 | 1.4 | -0.3 | -4.6 |
| 2-2-d1E04-1-0338 | C43 H42 N6 O8 S3 | 866.22262 | -5.5 | 3.2 | 10.3 | 3.0 |
| 2-2-d1E04-1-0339 | C43 H46 N8 O8 S2 | 866.28800 | -8.8 | -0.2 | 1.0 | -10.2 |
| 2-2-d1E04-1-0340 | C42 H41 N7 O8 S3 | 867.21787 | -101.9 | 0.0 | 0.0 | 0.0 |
| 2-2-d1E04-1-0341 | C41 H40 F3 N5 O9 S2 | 867.22195 | -4.3 | 0.1 | 0.0 | -17.0 |
| 2-2-d1E04-1-0342 | C43 H45 N7 O9 S2 | 867.27202 | -3.2 | 2.7 | 0.0 | 0.0 |
| 2-2-d1E04-1-0343 | C45 H49 N5 O9 S2 | 867.29717 | -11.3 | -3.9 | 0.8 | -8.1 |
| 2-2-d1E04-1-0344 | C46 H47 F3 N6 O6 S | 868.32299 | -16.6 | 0.0 | -2.7 | -16.5 |
| 2-2-d1E04-1-0345 | C45 H42 F3 N5 O8 S | 869.27062 | -3.8 | 3.8 | 7.7 | -1.1 |
| 2-2-d1E04-1-0346 | C43 H47 N7 O9 S2 | 869.28767 | -3.9 | 1.4 | 3.7 | -3.8 |
| 2-2-d1E04-1-0347 | C45 H48 F N5 O8 S2 | 869.29283 | -5.7 | -1.4 | -0.4 | -7.7 |
| 2-2-d1E04-1-0348 | C45 H46 F3 N7 O6 S | 869.31824 | -6.8 | 2.2 | 15.3 | -1.6 |
| 2-2-d1E04-1-0349 | C49 H55 N7 O6 S | 869.39345 | -2.2 | 0.3 | 3.4 | -0.8 |
| 2-2-d1E04-1-0350 | C44 H45 F3 N8 O6 S | 870.31349 | -4.4 | 2.0 | 1.7 | -5.4 |
| 2-2-d1E04-1-0351 | C48 H50 N6 O6 S2 | 870.32332 | -2.7 | 1.2 | 5.0 | 16.7 |
| 2-2-d1E04-1-0352 | C42 H42 F N7 O9 S2 | 871.24695 | -0.3 | -3.9 | -3.0 | 7.5 |
| 2-2-d1E04-1-0353 | C42 H45 N7 O8 S3 | 871.24917 | -4.8 | 2.4 | -4.2 | -4.6 |
| 2-2-d1E04-1-0354 | C44 H49 N5 O8 S3 | 871.27433 | -1.4 | 2.1 | 1.3 | -4.3 |
| 2-2-d1E04-1-0355 | C42 H48 F3 N5 O8 S2 | 871.28964 | -4.8 | -1.5 | -1.6 | -5.7 |
| 2-2-d1E04-1-0356 | C46 H48 F3 N5 O7 S | 871.32265 | -2.0 | 2.2 | 5.4 | 1.9 |
| 2-2-d1E04-1-0357 | C41 H40 N6 O8 S4 | 872.17905 | -2.4 | 2.2 | 2.3 | -5.3 |
| 2-2-d1E04-1-0358 | C42 H44 N6 O9 S3 | 872.23319 | -11.4 | -10.1 | -17.4 | -10.7 |
| 2-2-d1E04-1-0359 | C40 H39 N7 O10 S3 | 873.19205 | -2.5 | -6.2 | -4.2 | -21.2 |
| 2-2-d1E04-1-0360 | C42 H44 F N7 O9 S2 | 873.26260 | -5.2 | -5.2 | -0.8 | -8.1 |
| 2-2-d1E04-1-0361 | C41 H46 F3 N5 O9 S2 | 873.26890 | -3.5 | 0.7 | 0.2 | -5.9 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0362 | C47 H47 N5 O8 S2 | 873.28660 | -5.5 | -0.8 | -1.7 | -7.0 |
| 2-2-d1E04-1-0363 | C47 H47 N5 O8 S2 | 873.28660 | 0.0 | 0.0 | 7.8 | 17.5 |
| 2-2-d1E04-1-0364 | C48 H52 F N7 O6 S | 873.36838 | 0.5 | 0.1 | 6.9 | -3.6 |
| 2-2-d1E04-1-0365 | C46 H46 N6 O8 S2 | 874.28185 | -7.1 | -3.8 | -0.2 | -13.8 |
| 2-2-d1E04-1-0366 | C46 H46 N6 O8 S2 | 874.28185 | -2.4 | 2.0 | 6.6 | -6.7 |
| 2-2-d1E04-1-0367 | C46 H46 N6 O8 S2 | 874.28185 | -7.1 | -3.8 | -0.2 | -13.8 |
| 2-2-d1E04-1-0368 | C43 H46 F N5 O8 S3 | 875.24925 | 1.3 | 5.5 | 3.6 | -0.7 |
| 2-2-d1E04-1-0369 | C43 H44 F3 N7 O6 S2 | 875.27466 | -9.4 | 2.6 | 7.7 | -1.7 |
| 2-2-d1E04-1-0370 | C41 H41 F N6 O9 S3 | 876.20812 | -6.0 | -4.8 | -4.6 | -13.4 |
| 2-2-d1E04-1-0371 | C46 H48 N6 O6 S3 | 876.27975 | -5.6 | -1.1 | 8.3 | -1.7 |
| 2-2-d1E04-1-0372 | C43 H42 F3 N5 O8 S2 | 877.24269 | -7.1 | 1.7 | -0.2 | -8.2 |
| 2-2-d1E04-1-0373 | C43 H42 F3 N5 O8 S2 | 877.24269 | -3.3 | 2.4 | 3.1 | -6.2 |
| 2-2-d1E04-1-0374 | C46 H44 F N5 O8 S2 | 877.26153 | -5.1 | -1.2 | -0.3 | -8.7 |
| 2-2-d1E04-1-0375 | C46 H44 F N5 O8 S2 | 877.26153 | -37.6 | 0.0 | 0.7 | -9.8 |
| 2-2-d1E04-1-0376 | C47 H51 N5 O8 S2 | 877.31791 | -4.0 | -0.1 | -2.5 | -8.6 |
| 2-2-d1E04-1-0377 | C42 H41 F3 N6 O8 S2 | 878.23794 | -6.3 | 0.4 | -0.3 | -6.1 |
| 2-2-d1E04-1-0378 | C45 H43 F N6 O8 S2 | 878.25678 | -6.1 | 0.4 | -2.0 | -8.8 |
| 2-2-d1E04-1-0379 | C45 H43 F N6 O8 S2 | 878.25678 | 5.0 | 11.0 | 11.2 | 13.9 |
| 2-2-d1E04-1-0380 | C45 H43 F N6 O8 S2 | 878.25678 | 1.2 | 16.0 | -8.2 | -4.8 |
| 2-2-d1E04-1-0381 | C46 H50 N6 O8 S2 | 878.31315 | 0.0 | 0.0 | 0.0 | 0.0 |
| 2-2-d1E04-1-0382 | C45 H45 N5 O10 S2 | 879.26078 | -3.6 | 5.1 | 7.4 | -1.4 |
| 2-2-d1E04-1-0383 | C45 H49 N7 O8 S2 | 879.30840 | -9.0 | -23.0 | 24.7 | -11.6 |
| 2-2-d1E04-1-0384 | C44 H48 N8 O8 S2 | 880.30365 | -3.6 | 6.5 | 10.1 | 8.3 |
| 2-2-d1E04-1-0385 | C46 H51 N5 O9 S2 | 881.31282 | -5.6 | -1.7 | -1.9 | -8.3 |
| 2-2-d1E04-1-0386 | C48 H50 F3 N5 O6 S | 881.34339 | -2.2 | 3.8 | 6.0 | -1.3 |
| 2-2-d1E04-1-0387 | C43 H42 N6 O9 S3 | 882.21754 | -5.1 | 0.6 | -3.5 | -8.7 |
| 2-2-d1E04-1-0388 | C45 H47 F N6 O8 S2 | 882.28808 | -3.5 | -29.3 | 5.8 | -3.0 |
| 2-2-d1E04-1-0389 | C41 H40 F3 N5 O8 S3 | 883.19911 | -5.1 | 0.9 | 1.0 | -7.4 |
| 2-2-d1E04-1-0390 | C44 H42 F N5 O10 S2 | 883.23571 | 1.9 | 5.5 | 7.2 | -2.9 |
| 2-2-d1E04-1-0391 | C45 H49 N5 O8 S3 | 883.27433 | -5.7 | 0.3 | 0.0 | -10.5 |
| 2-2-d1E04-1-0392 | C44 H46 F N7 O8 S2 | 883.28333 | -5.6 | -6.4 | -8.1 | 19.4 |
| 2-2-d1E04-1-0393 | C41 H40 N8 O9 S3 | 884.20804 | -6.1 | -0.9 | -0.3 | -5.8 |
| 2-2-d1E04-1-0394 | C40 H39 F3 N6 O10 S2 | 884.21212 | -5.1 | -3.5 | -6.6 | -1.0 |
| 2-2-d1E04-1-0395 | C47 H44 N6 O8 S2 | 884.26620 | -5.9 | 2.5 | 4.4 | 18.0 |
| 2-2-d1E04-1-0396 | C43 H45 F N8 O8 S2 | 884.27858 | -0.4 | -3.6 | -1.7 | 4.3 |
| 2-2-d1E04-1-0397 | C43 H47 N7 O8 S3 | 885.26482 | 0.0 | 0.0 | 0.0 | 0.0 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0398 | C45 H48 F N5 O9 S2 | 885.28775 | -6.2 | -1.8 | 0.4 | -7.6 |
| 2-2-d1E04-1-0399 | C41 H42 N8 O9 S3 | 886.22369 | -9.7 | -7.6 | -9.5 | -15.0 |
| 2-2-d1E04-1-0400 | C44 H50 N6 O8 S3 | 886.28522 | -2.9 | -6.0 | -3.8 | -10.7 |
| 2-2-d1E04-1-0401 | C47 H50 N8 O6 S2 | 886.32947 | -2.7 | 0.4 | 1.2 | -8.8 |
| 2-2-d1E04-1-0402 | C46 H48 F3 N5 O6 S2 | 887.29981 | -3.1 | 3.0 | 6.5 | -2.8 |
| 2-2-d1E04-1-0403 | C43 H48 N6 O9 S3 | 888.26449 | -8.8 | -8.8 | 0.1 | 2.2 |
| 2-2-d1E04-1-0404 | C40 H39 N7 O9 S4 | 889.16921 | -4.3 | -4.7 | 5.5 | -1.9 |
| 2-2-d1E04-1-0405 | C42 H44 F N7 O8 S3 | 889.23975 | -3.6 | 0.0 | 23.7 | 30.0 |
| 2-2-d1E04-1-0406 | C45 H42 N6 O8 S3 | 890.22262 | 4.6 | 10.0 | 0.1 | -3.9 |
| 2-2-d1E04-1-0407 | C45 H42 N6 O8 S3 | 890.22262 | -7.7 | 2.9 | 8.2 | -2.4 |
| 2-2-d1E04-1-0408 | C44 H41 N7 O8 S3 | 891.21787 | -6.7 | 3.7 | 5.2 | -7.4 |
| 2-2-d1E04-1-0409 | C44 H41 N7 O8 S3 | 891.21787 | -2.9 | 0.1 | 0.3 | 0.2 |
| 2-2-d1E04-1-0410 | C44 H41 N7 O8 S3 | 891.21787 | 0.0 | 0.0 | 0.0 | -214.5 |
| 2-2-d1E04-1-0411 | C46 H45 N5 O10 S2 | 891.26078 | 7.3 | 13.5 | 11.8 | -11.6 |
| 2-2-d1E04-1-0412 | C48 H53 N5 O8 S2 | 891.33356 | -6.3 | 0.2 | 8.2 | 8.2 |
| 2-2-d1E04-1-0413 | C45 H44 N6 O8 S3 | 892.23827 | -5.3 | 2.2 | -0.5 | -7.4 |
| 2-2-d1E04-1-0414 | C45 H44 N6 O8 S3 | 892.23827 | 0.0 | 0.0 | 0.0 | 0.0 |
| 2-2-d1E04-1-0415 | C44 H43 N7 O8 S3 | 893.23352 | -6.7 | -0.4 | -7.2 | -7.9 |
| 2-2-d1E04-1-0416 | C43 H42 F3 N5 O9 S2 | 893.23760 | -5.8 | 0.3 | 1.1 | -4.2 |
| 2-2-d1E04-1-0417 | C46 H51 N7 O8 S2 | 893.32405 | 70.3 | 77.8 | 72.8 | 71.8 |
| 2-2-d1E04-1-0418 | C41 H40 F3 N7 O9 S2 | 895.22810 | -4.0 | -2.6 | -5.8 | -10.2 |
| 2-2-d1E04-1-0419 | C44 H45 N7 O8 S3 | 895.24917 | -2.4 | 4.9 | 2.7 | 11.6 |
| 2-2-d1E04-1-0420 | C47 H44 F3 N5 O8 S | 895.28627 | 0.7 | 2.0 | 5.4 | -4.9 |
| 2-2-d1E04-1-0421 | C47 H50 F N5 O8 S2 | 895.30848 | -6.9 | -1.4 | -2.0 | -7.5 |
| 2-2-d1E04-1-0422 | C43 H44 N8 O8 S3 | 896.24442 | -7.5 | 2.5 | 5.1 | 7.0 |
| 2-2-d1E04-1-0423 | C42 H43 N9 O8 S3 | 897.23967 | 2.8 | 2.9 | 0.9 | -32.7 |
| 2-2-d1E04-1-0424 | C41 H42 F3 N7 O9 S2 | 897.24375 | -4.6 | 3.2 | 0.6 | -27.3 |
| 2-2-d1E04-1-0425 | C46 H51 N5 O8 S3 | 897.28998 | -0.4 | -1.4 | -4.4 | -11.6 |
| 2-2-d1E04-1-0426 | C44 H50 F3 N5 O8 S2 | 897.30529 | -5.7 | -1.1 | -0.6 | -7.4 |
| 2-2-d1E04-1-0427 | C47 H50 F3 N7 O6 S | 897.34954 | -3.1 | 3.3 | 4.4 | -0.7 |
| 2-2-d1E04-1-0428 | C43 H42 N6 O8 S4 | 898.19470 | -7.9 | 0.0 | -0.1 | -5.3 |
| 2-2-d1E04-1-0429 | C44 H46 N6 O9 S3 | 898.24884 | 0.2 | 1.7 | 3.1 | 0.1 |
| 2-2-d1E04-1-0430 | C42 H41 N7 O10 S3 | 899.20770 | -10.6 | -14.0 | -19.9 | -0.9 |
| 2-2-d1E04-1-0431 | C43 H48 F3 N5 O9 S2 | 899.28455 | -4.4 | -0.3 | -1.4 | -1.5 |
| 2-2-d1E04-1-0432 | C40 H39 F3 N6 O9 S3 | 900.18927 | -9.9 | 1.1 | -2.7 | -10.9 |
| 2-2-d1E04-1-0433 | C45 H42 F3 N5 O8 S2 | 901.24269 | -2.2 | 1.6 | 3.0 | -5.2 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0434 | C45 H42 F3 N5 O8 S2 | 901.24269 | 0.0 | 0.8 | 0.0 | 14.5 |
| 2-2-d1E04-1-0435 | C45 H48 F N5 O8 S3 | 901.26490 | -3.6 | -1.2 | -0.7 | -5.0 |
| 2-2-d1E04-1-0436 | C41 H42 N8 O8 S4 | 902.20084 | -4.9 | 4.7 | 3.9 | -12.4 |
| 2-2-d1E04-1-0437 | C44 H41 F3 N6 O8 S2 | 902.23794 | -2.7 | 1.5 | -0.7 | -5.4 |
| 2-2-d1E04-1-0438 | C44 H41 F3 N6 O8 S2 | 902.23794 | -8.5 | 4.7 | 1.6 | -6.2 |
| 2-2-d1E04-1-0439 | C44 H41 F3 N6 O8 S2 | 902.23794 | 11.5 | -7.9 | 19.1 | -18.5 |
| 2-2-d1E04-1-0440 | C45 H44 F3 N5 O8 S2 | 903.25834 | -4.7 | 0.7 | -0.1 | -4.7 |
| 2-2-d1E04-1-0441 | C45 H44 F3 N5 O8 S2 | 903.25834 | 0.0 | 0.0 | 0.0 | 6.8 |
| 2-2-d1E04-1-0442 | C44 H43 F3 N6 O8 S2 | 904.25359 | -3.9 | 4.2 | -2.9 | -10.6 |
| 2-2-d1E04-1-0443 | C47 H47 N5 O10 S2 | 905.27643 | -4.0 | -1.0 | 4.5 | -12.7 |
| 2-2-d1E04-1-0444 | C44 H45 F3 N6 O8 S2 | 906.26924 | -6.9 | 3.7 | 0.8 | -5.0 |
| 2-2-d1E04-1-0445 | C43 H44 F3 N7 O8 S2 | 907.26449 | -4.4 | 1.6 | 0.6 | -6.1 |
| 2-2-d1E04-1-0446 | C47 H53 N7 O8 S2 | 907.33970 | 57.8 | 68.3 | 60.9 | 58.1 |
| 2-2-d1E04-1-0447 | C42 H43 F3 N8 O8 S2 | 908.25974 | -1.1 | 3.1 | 3.4 | -6.3 |
| 2-2-d1E04-1-0448 | C46 H48 N6 O8 S3 | 908.26957 | -2.9 | 1.7 | -0.7 | -6.2 |
| 2-2-d1E04-1-0449 | C43 H42 F3 N5 O8 S3 | 909.21476 | -6.1 | 0.9 | 0.0 | -4.3 |
| 2-2-d1E04-1-0450 | C46 H44 F N5 O10 S2 | 909.25136 | -8.0 | 3.0 | 5.8 | 4.5 |
| 2-2-d1E04-1-0451 | C44 H46 F3 N5 O9 S2 | 909.26890 | -4.0 | 0.4 | -2.2 | -2.0 |
| 2-2-d1E04-1-0452 | C43 H42 N8 O9 S3 | 910.22369 | -3.8 | 5.9 | -0.7 | -3.5 |
| 2-2-d1E04-1-0453 | C42 H41 F3 N6 O10 S2 | 910.22777 | 18.9 | -19.9 | 4.9 | 9.0 |
| 2-2-d1E04-1-0454 | C46 H50 F N7 O8 S2 | 911.31463 | 72.6 | 87.8 | 78.5 | 74.6 |
| 2-2-d1E04-1-0455 | C43 H44 N8 O9 S3 | 912.23934 | -13.4 | -5.6 | 0.0 | 0.0 |
| 2-2-d1E04-1-0456 | C49 H52 N8 O6 S2 | 912.34512 | -9.5 | 22.0 | 11.3 | 5.6 |
| 2-2-d1E04-1-0457 | C41 H42 F3 N7 O8 S3 | 913.22091 | -4.0 | 1.9 | -2.1 | -7.9 |
| 2-2-d1E04-1-0458 | C44 H46 N6 O8 S4 | 914.22600 | -3.5 | -1.3 | -2.2 | -4.7 |
| 2-2-d1E04-1-0459 | C42 H41 N7 O9 S4 | 915.18486 | -3.1 | 0.9 | 3.6 | -6.3 |
| 2-2-d1E04-1-0460 | C47 H44 N6 O8 S3 | 916.23827 | -3.6 | 0.9 | 2.5 | -3.3 |
| 2-2-d1E04-1-0461 | C47 H44 N6 O8 S3 | 916.23827 | 0.0 | 0.0 | 0.0 | -42.4 |
| 2-2-d1E04-1-0462 | C46 H43 N7 O8 S3 | 917.23352 | -5.6 | 0.2 | 1.9 | -2.1 |
| 2-2-d1E04-1-0463 | C46 H43 N7 O8 S3 | 917.23352 | -3.3 | 2.8 | -2.6 | -1.9 |
| 2-2-d1E04-1-0464 | C46 H43 N7 O8 S3 | 917.23352 | -20.2 | -0.7 | -14.2 | 0.0 |
| 2-2-d1E04-1-0465 | C46 H48 F3 N5 O8 S2 | 919.28964 | -4.8 | 2.2 | 1.6 | -4.3 |
| 2-2-d1E04-1-0466 | C48 H53 N7 O8 S2 | 919.33970 | 23.3 | 28.9 | 19.8 | 13.2 |
| 2-2-d1E04-1-0467 | C43 H42 F3 N7 O9 S2 | 921.24375 | 0.0 | 0.0 | -1.5 | -14.1 |
| 2-2-d1E04-1-0468 | C46 H47 N7 O8 S3 | 921.26482 | -3.6 | 0.9 | 1.4 | -1.0 |
| 2-2-d1E04-1-0469 | C45 H42 N6 O10 S3 | 922.21245 | 2.5 | 12.9 | 17.6 | 21.0 |

(continued)

| [Table 11-3-4] | | | | | | |
|---|---|---|---|---|---|---|
| | | | AS-MS(%) (%) | | | |
| ID | Molecular Formula | Exact Mass | N1 | N2 | N3 | N4 |
| 2-2-d1E04-1-0470 | C45 H46 N8 O8 S3 | 922.26007 | 0.0 | 0.0 | -7.4 | -15.5 |
| 2-2-d1E04-1-0471 | C44 H45 N9 O8 S3 | 923.25532 | -1.6 | -16.1 | 3.5 | 0.0 |
| 2-2-d1 E04-1-04 72 | C43 H44 F3 N7 O9 S2 | 923.25940 | 1.8 | -17.0 | 12.2 | 17.9 |
| 2-2-d1 E04-1-04 73 | C49 H52 F3 N7 O6 S | 923.36519 | -0.4 | 3.7 | 4.8 | -4.5 |
| 2-2-d1E04-1-0474 | C46 H48 N6 O9 S3 | 924.26449 | -0.7 | -0.7 | 2.5 | -8.0 |
| 2-2-d1E04-1-0475 | C44 H46 F3 N5 O8 S3 | 925.24606 | -2.8 | 2.8 | 1.7 | -1.1 |
| 2-2-d1E04-1-0476 | C42 H41 F3 N6 O9 S3 | 926.20492 | -5.6 | -2.5 | -2.2 | -10.9 |
| 2-2-d1E04-1-0477 | C47 H44 F3 N5 O8 S2 | 927.25834 | -0.4 | 3.7 | 4.6 | -6.6 |
| 2-2-d1E04-1-0478 | C47 H44 F3 N5 O8 S2 | 927.25834 | 10.2 | 13.5 | 20.4 | 0.0 |
| 2-2-d1E04-1-0479 | C43 H44 N8 O8 S4 | 928.21649 | -2.9 | 1.3 | 0.1 | -8.8 |
| 2-2-d1E04-1-0480 | C46 H43 F3 N6 O8 S2 | 928.25359 | -0.5 | 4.1 | 3.5 | -5.2 |
| 2-2-d1E04-1-0481 | C46 H43 F3 N6 O8 S2 | 928.25359 | -2.4 | 3.7 | 0.3 | -3.1 |
| 2-2-d1E04-1-0482 | C46 H43 F3 N6 O8 S2 | 928.25359 | 0.0 | -3.0 | 2.4 | 0.0 |
| 2-2-d1E04-1-0483 | C46 H47 F3 N6 O8 S2 | 932.28489 | -7.4 | 0.9 | -2.7 | -5.3 |
| 2-2-d1E04-1-0484 | C45 H42 F3 N5 O10 S2 | 933.23252 | -4.0 | 2.5 | 3.0 | -2.7 |
| 2-2-d1E04-1-0485 | C45 H46 F3 N7 O8 S2 | 933.28014 | -2.5 | 0.8 | -1.2 | -6.8 |
| 2-2-d1E04-1-0486 | C49 H55 N7 O8 S2 | 933.35535 | 0.3 | 10.6 | 9.1 | 5.6 |
| 2-2-d1E04-1-0487 | C44 H45 F3 N8 O8 S2 | 934.27539 | -0.3 | 13.5 | 1.7 | 0.8 |
| 2-2-d1E04-1-0488 | C48 H50 N6 O8 S3 | 934.28522 | -5.5 | 0.2 | -1.4 | -5.1 |
| 2-2-d1E04-1-0489 | C46 H48 F3 N5 O9 S2 | 935.28455 | -4.3 | -2.2 | 1.2 | -6.7 |
| 2-2-d1E04-1-0490 | C48 H52 F N7 O8 S2 | 937.33028 | 7.6 | 14.5 | 10.7 | 5.5 |
| 2-2-d1E04-1-0491 | C43 H44 F3 N7 O8 S3 | 939.23656 | -5.0 | 7.5 | -1.4 | -6.0 |
| 2-2-d1E04-1-0492 | C46 H48 N6 O8 S4 | 940.24165 | -1.7 | 1.2 | 0.0 | -1.2 |
| 2-2-d1E04-1-0493 | C48 H50 F3 N5 O8 S2 | 945.30529 | -7.1 | 0.7 | 0.4 | -5.2 |
| 2-2-d1E04-1-0494 | C47 H44 N6 O10 S3 | 948.22810 | -1.4 | 5.6 | 5.9 | 12.7 |
| 2-2-d1E04-1-0495 | C47 H50 N8 O8 S3 | 950.29137 | 59.4 | 93.2 | 85.8 | 69.0 |
| 2-2-d1E04-1-0496 | C46 H48 F3 N5 O8 S3 | 951.26171 | -10.0 | 1.2 | 2.3 | -5.1 |
| 2-2-d1E04-1-0497 | C47 H44 F3 N5 O10 S2 | 959.24817 | -5.4 | 0.3 | 0.3 | -6.3 |
| 2-2-d1E04-1-0498 | C47 H50 F3 N7 O8 S2 | 961.31144 | 45.5 | 61.4 | 52.7 | 51.0 |
| 2-2-d1E04-1-0499 | C49 H52 N8 O8 S3 | 976.30702 | 0.5 | 1.5 | 8.3 | -3.7 |
| 2-2-d1E04-1-0500 | C49 H52 F3 N7 O8 S2 | 987.32709 | -3.3 | 0.0 | 3.9 | -3.8 |

[Table 602]

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0001 | C34 H36 N4 O6 | 596.26348 | -0.3 | -3.8 | 5.4 | 14.5 |
| 2-2-d1E01-2-0002 | C35 H44 N4 O5 | 600.33117 | -0.3 | 8.5 | 6.0 | 16.9 |
| 2-2-d1E01-2-0003 | C34 H42 N4 O6 | 602.31044 | -3.3 | 10.7 | 0.7 | 5.6 |
| 2-2-d1E01-2-0004 | C36 H38 N4 O5 | 606.28422 | -9.1 | -8.2 | 12.2 | 19.2 |
| 2-2-d1E01-2-0005 | C36 H38 N4 O5 | 606.28422 | 12.2 | 22.7 | 8.1 | 12.1 |
| 2-2-d1E01-2-0006 | C35 H37 N5 O5 | 607.27947 | 1.6 | -10.4 | 10.4 | 14.3 |
| 2-2-d1E01-2-0007 | C35 H38 N4 O6 | 610.27913 | 16.8 | 41.5 | -5.5 | 21.9 |
| 2-2-d1E01-2-0008 | C34 H36 N4 O5 S | 612.24064 | 0.9 | -7.7 | 5.5 | 21.6 |
| 2-2-d1E01-2-0009 | C33 H35 N5 O7 | 613.25365 | 0.0 | 0.0 | 0.0 | 0.0 |
| 2-2-d1E01-2-0010 | C34 H35 F N4 O6 | 614.25406 | 6.3 | 2.9 | -2.5 | 13.2 |
| 2-2-d1E01-2-0011 | C36 H46 N4 O5 | 614.34682 | -7.5 | 14.7 | 0.0 | 15.6 |
| 2-2-d1E01-2-0012 | C35 H44 N4 O6 | 616.32609 | -0.8 | 7.4 | 2.1 | 8.2 |
| 2-2-d1E01-2-0013 | C35 H43 F N4 O5 | 618.32175 | 5.2 | -8.8 | 2.3 | 16.2 |
| 2-2-d1E01-2-0014 | C37 H40 N4 O5 | 620.29987 | 2.5 | -1.1 | -9.4 | 9.4 |
| 2-2-d1E01-2-0015 | C37 H40 N4 O5 | 620.29987 | 15.4 | 19.0 | -8.2 | 14.9 |
| 2-2-d1E01-2-0016 | C34 H41 F N4 O6 | 620.30101 | -15.0 | -9.2 | 3.2 | 4.7 |
| 2-2-d1E01-2-0017 | C36 H39 N5 O5 | 621.29512 | 7.2 | -12.7 | 10.7 | 10.2 |
| 2-2-d1E01-2-0018 | C36 H38 N4 O6 | 622.27913 | -16.4 | -31.6 | 19.3 | 15.8 |
| 2-2-d1E01-2-0019 | C34 H36 N6 O6 | 624.26963 | 8.9 | -34.2 | 12.5 | 27.1 |
| 2-2-d1E01-2-0020 | C36 H37 F N4 O5 | 624.27480 | 3.8 | -25.2 | 4.3 | 23.4 |
| 2-2-d1E01-2-0021 | C36 H37 F N4 O5 | 624.27480 | 22.9 | -10.0 | 5.9 | 16.5 |
| 2-2-d1E01-2-0022 | C35 H36 F N5 O5 | 625.27005 | 2.8 | -4.1 | 8.0 | 12.8 |
| 2-2-d1E01-2-0023 | C35 H38 N4 O5 S | 626.25629 | -6.5 | 24.6 | 10.7 | 20.8 |
| 2-2-d1E01-2-0024 | C34 H38 N6 O6 | 626.28528 | -0.2 | 12.5 | 14.3 | 12.0 |
| 2-2-d1E01-2-0025 | C37 H46 N4 O5 | 626.34682 | 14.3 | 10.7 | 9.3 | 24.1 |
| 2-2-d1E01-2-0026 | C34 H37 N5 O7 | 627.26930 | -2.0 | -10.1 | -12.2 | 3.1 |
| 2-2-d1E01-2-0027 | C36 H44 N4 O6 | 628.32609 | 8.5 | 5.3 | -3.8 | 3.3 |
| 2-2-d1E01-2-0028 | C33 H35 N5 O6 S | 629.23080 | 4.2 | 19.0 | 12.9 | 22.5 |
| 2-2-d1E01-2-0029 | C34 H35 F N4 05 S | 630.23122 | -8.3 | 44.1 | 1.7 | 17.1 |
| 2-2-d1E01-2-0030 | C38 H38 N4 O5 | 630.28422 | 3.1 | 29.6 | -7.5 | 11.8 |
| 2-2-d1E01-2-0031 | C38 H38 N4 O5 | 630.28422 | 9.9 | 30.2 | -7.5 | 11.8 |
| 2-2-d1E01-2-0032 | C33 H34 F N5 O7 | 631.24423 | -2.9 | -2.2 | 5.2 | 6.6 |
| 2-2-d1E01-2-0033 | C37 H37 N5 O5 | 631.27947 | 6.9 | 1.1 | 1.6 | 15.2 |
| 2-2-d1E01-2-0034 | C37 H37 N5 O5 | 631.27947 | 0.0 | 14.5 | 15.7 | 6.9 |
| 2-2-d1E01-2-0035 | C37 H37 N5 O5 | 631.27947 | 105.1 | -31.4 | 8.8 | 35.0 |
| 2-2-d1E01-2-0036 | C38 H40 N4 O5 | 632.29987 | 0.0 | 0.0 | 3.5 | 0.0 |
| 2-2-d1E01-2-0037 | C38 H40 N4 O5 | 632.29987 | 10.4 | -111.0 | -11.2 | 52.4 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0038 | C37 H39 N5 O5 | 633.29512 | -2.6 | -9.7 | 5.9 | 10.0 |
| 2-2-d1E01-2-0039 | C37 H41 N5 O5 | 635.31077 | 14.7 | 23.7 | 8.1 | 21.6 |
| 2-2-d1E01-2-0040 | C37 H40 N4 O6 | 636.29479 | 2.9 | 10.4 | 12.0 | 10.4 |
| 2-2-d1E01-2-0041 | C36 H40 N6 O5 | 636.30602 | -32.2 | -81.1 | 12.8 | -11.0 |
| 2-2-d1E01-2-0042 | C35 H39 N7 O5 | 637.30127 | 6.9 | -10.9 | 4.7 | 11.9 |
| 2-2-d1E01-2-0043 | C36 H38 N4 O5 S | 638.25629 | 0.0 | 0.0 | 0.0 | 0.0 |
| 2-2-d1E01-2-0044 | C35 H38 N6 O6 | 638.28528 | 7.9 | -25.1 | 4.4 | 26.8 |
| 2-2-d1E01-2-0045 | C37 H42 N4 O6 | 638.31044 | 8.7 | 11.2 | -8.2 | 2.6 |
| 2-2-d1E01-2-0046 | C35 H37 N5 O7 | 639.26930 | 0.0 | 0.0 | 0.0 | 0.0 |
| 2-2-d1E01-2-0047 | C36 H37 F N4 O6 | 640.26971 | -9.8 | -43.5 | 25.6 | 11.4 |
| 2-2-d1E01-2-0048 | C35 H40 N6 O6 | 640.30093 | -3.9 | -14.0 | 3.9 | 16.9 |
| 2-2-d1E01-2-0049 | C38 H48 N4 O5 | 640.36247 | -0.7 | 31.9 | 7.0 | 16.7 |
| 2-2-d1E01-2-0050 | C34 H35 F N6 O6 | 642.26021 | -10.7 | -67.0 | 47.6 | 0.0 |
| 2-2-d1E01-2-0051 | C34 H38 N6 O5 S | 642.26244 | 1.3 | 4.2 | -3.8 | 9.0 |
| 2-2-d1E01-2-0052 | C37 H46 N4 O6 | 642.34174 | -32.1 | 10.4 | -6.0 | 36.0 |
| 2-2-d1E01-2-0053 | C34 H37 N5 O6 S | 643.24645 | 7.9 | 0.4 | 31.4 | 0.6 |
| 2-2-d1E01-2-0054 | C34 H37 F N6 O6 | 644.27586 | 3.1 | 13.7 | 7.3 | 13.5 |
| 2-2-d1E01-2-0055 | C39 H40 N4 O5 | 644.29987 | 0.0 | 0.0 | 0.0 | 9.1 |
| 2-2-d1E01-2-0056 | C39 H40 N4 O5 | 644.29987 | 0.0 | 0.0 | 0.0 | 9.1 |
| 2-2-d1E01-2-0057 | C37 H45 F N4 O5 | 644.33740 | -4.7 | -6.9 | 7.6 | 5.7 |
| 2-2-d1E01-2-0058 | C38 H39 N5 O5 | 645.29512 | 97.8 | -47.2 | 16.1 | 28.5 |
| 2-2-d1E01-2-0059 | C38 H39 N5 O5 | 645.29512 | 18.8 | 40.0 | -0.4 | 22.4 |
| 2-2-d1E01-2-0060 | C38 H39 N5 O5 | 645.29512 | 2.8 | -37.8 | 17.1 | -12.0 |
| 2-2-d1E01-2-0061 | C39 H42 N4 O5 | 646.31552 | -5.5 | -72.3 | 7.2 | 4.5 |
| 2-2-d1E01-2-0062 | C39 H42 N4 O5 | 646.31552 | 0.0 | 0.0 | 0.0 | 0.0 |
| 2-2-d1E01-2-0063 | C36 H43 F N4 O6 | 646.31666 | 17.5 | 3.6 | -1.4 | 0.4 |
| 2-2-d1E01-2-0064 | C33 H34 F N5 O6 S | 647.22138 | 8.5 | 35.1 | 4.5 | 22.4 |
| 2-2-d1E01-2-0065 | C38 H41 N5 O5 | 647.31077 | 15.6 | 52.6 | 14.2 | 10.4 |
| 2-2-d1E01-2-0066 | C38 H37 F N4 O5 | 648.27480 | -7.9 | 0.0 | 0.0 | 0.0 |
| 2-2-d1E01-2-0067 | C38 H37 F N4 O5 | 648.27480 | 8.3 | 16.4 | 19.0 | 13.4 |
| 2-2-d1E01-2-0068 | C39 H44 N4 O5 | 648.33117 | 31.6 | -63.1 | -24.3 | 37.5 |
| 2-2-d1E01-2-0069 | C37 H36 F N5 O5 | 649.27005 | 10.8 | 10.7 | -3.8 | 10.8 |
| 2-2-d1E01-2-0070 | C37 H36 F N5 O5 | 649.27005 | 23.4 | -12.5 | 13.4 | 11.7 |
| 2-2-d1E01-2-0071 | C37 H36 F N5 O5 | 649.27005 | 111.4 | -23.3 | 10.6 | 28.8 |
| 2-2-d1E01-2-0072 | C38 H43 N5 O5 | 649.32642 | -2.7 | -15.9 | 5.0 | 6.9 |
| 2-2-d1E01-2-0073 | C36 H38 N6 O6 | 650.28528 | 0.0 | 0.0 | 0.0 | 0.0 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0074 | C38 H39 F N4 O5 | 650.29045 | -20.8 | 0.0 | -30.0 | 11.7 |
| 2-2-d1E01-2-0075 | C38 H39 F N4 O5 | 650.29045 | 20.6 | -13.3 | 17.0 | 15.2 |
| 2-2-d1E01-2-0076 | C37 H42 N6 O5 | 650.32167 | 11.7 | 18.8 | 10.7 | -7.4 |
| 2-2-d1E01-2-0077 | C37 H38 F N5 O5 | 651.28570 | 3.0 | -23.3 | 7.5 | 9.8 |
| 2-2-d1E01-2-0078 | C36 H41 N7 O5 | 651.31692 | 11.9 | 26.3 | -1.3 | 18.7 |
| 2-2-d1E01-2-0079 | C37 H40 N4 O5 S | 652.27194 | 1.5 | 0.2 | 2.2 | -1.5 |
| 2-2-d1E01-2-0080 | C36 H40 N6 O6 | 652.30093 | -7.6 | 24.8 | 3.4 | 13.1 |
| 2-2-d1E01-2-0081 | C38 H44 N4 O6 | 652.32609 | -4.1 | -28.4 | -1.9 | 23.7 |
| 2-2-d1E01-2-0082 | C35 H35 N5 O6 S | 653.23080 | 7.7 | -5.1 | 6.2 | 13.0 |
| 2-2-d1E01-2-0083 | C36 H39 N5 O7 | 653.28495 | 3.3 | 7.0 | 7.2 | 12.4 |
| 2-2-d1E01-2-0084 | C37 H40 F N5 O5 | 653.30135 | -6.0 | 2.2 | 6.6 | 20.7 |
| 2-2-d1E01-2-0085 | C37 H42 N4 O5 S | 654.28759 | 3.1 | -7.5 | -2.3 | 0.0 |
| 2-2-d1E01-2-0086 | C36 H39 F N6 O5 | 654.29660 | 3.1 | -0.1 | 7.1 | 6.3 |
| 2-2-d1E01-2-0087 | C35 H37 N5 O6 S | 655.24645 | 6.8 | 0.0 | -1.1 | 2.9 |
| 2-2-d1E01-2-0088 | C35 H38 F N7 O5 | 655.29185 | 4.1 | 1.5 | 0.0 | 10.7 |
| 2-2-d1E01-2-0089 | C36 H37 F N4 O5 S | 656.24687 | 36.3 | 6.3 | 32.1 | 9.7 |
| 2-2-d1E01-2-0090 | C35 H40 N6 O5 S | 656.27809 | -28.0 | 28.5 | -0.5 | 6.5 |
| 2-2-d1E01-2-0091 | C40 H40 N4 O5 | 656.29987 | 37.3 | 28.7 | 26.9 | 37.2 |
| 2-2-d1E01-2-0092 | C40 H40 N4 O5 | 656.29987 | 0.0 | 0.0 | 0.0 | 0.0 |
| 2-2-d1E01-2-0093 | C37 H41 F N4 O6 | 656.30101 | 2.7 | 11.3 | -3.4 | 2.8 |
| 2-2-d1E01-2-0094 | C35 H36 F N5 O7 | 657.25988 | 14.9 | -37.2 | 33.2 | -3.0 |
| 2-2-d1E01-2-0095 | C39 H39 N5 O5 | 657.29512 | 0.0 | 0.0 | 32.3 | 54.2 |
| 2-2-d1E01-2-0096 | C39 H39 N5 O5 | 657.29512 | -16.1 | -39.9 | 7.3 | 18.5 |
| 2-2-d1E01-2-0097 | C39 H39 N5 O5 | 657.29512 | -14.3 | -24.5 | 29.2 | 51.1 |
| 2-2-d1E01-2-0098 | C36 H43 N5 O5 S | 657.29849 | -3.3 | -20.8 | 1.3 | 8.9 |
| 2-2-d1E01-2-0099 | C35 H41 N5 O6 S | 659.27775 | -7.9 | 3.0 | 5.4 | 6.2 |
| 2-2-d1E01-2-0100 | C34 H36 N4 O8 S | 660.22538 | -2.2 | 0.7 | 1.0 | 6.9 |
| 2-2-d1E01-2-0101 | C34 H37 F N6 O5 S | 660.25302 | 3.6 | 8.5 | 0.5 | 2.4 |
| 2-2-d1E01-2-0102 | C39 H43 N5 O5 | 661.32642 | -3.2 | -3.2 | 9.1 | 19.2 |
| 2-2-d1E01-2-0103 | C38 H38 N4 O7 | 662.27405 | -6.6 | -30.4 | 21.4 | 16.0 |
| 2-2-d1E01-2-0104 | C38 H42 N6 O5 | 662.32167 | -1.3 | -1.5 | 11.8 | -0.2 |
| 2-2-d1E01-2-0105 | C40 H46 N4 O5 | 662.34682 | -0.8 | 64.6 | 7.4 | 0.6 |
| 2-2-d1E01-2-0106 | C37 H37 N5 O5 S | 663.25154 | 15.3 | 11.3 | 25.2 | 20.9 |
| 2-2-d1E01-2-0107 | C37 H37 N5 O5 S | 663.25154 | -3.2 | -19.7 | 16.2 | 13.7 |
| 2-2-d1E01-2-0108 | C37 H41 N7 O5 | 663.31692 | -7.2 | -2.0 | 13.0 | 0.0 |
| 2-2-d1E01-2-0109 | C36 H36 N6 O5 S | 664.24679 | -3.4 | -13.8 | 5.1 | 7.3 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0110 | C35 H35 F3 N4 O6 | 664.25087 | 1.7 | -17.1 | -9.7 | 25.7 |
| 2-2-d1E01-2-0111 | C35 H44 N4 O7 S | 664.29307 | -7.7 | -15.6 | -3.7 | -1.9 |
| 2-2-d1E01-2-0112 | C37 H40 N6 O6 | 664.30093 | 35.5 | -167.7 | 0.0 | 0.0 |
| 2-2-d1E01-2-0113 | C39 H44 N4 O6 | 664.32609 | 0.0 | 0.0 | -12.9 | 56.8 |
| 2-2-dlE01-2-0114 | C34 H42 N4 O8 S | 666.27234 | -1.7 | -8.9 | 1.1 | 1.2 |
| 2-2-d1E01-2-0115 | C37 H42 N6 O6 | 666.31658 | 0.0 | 0.0 | 0.0 | 0.0 |
| 2-2-d1E01-2-0116 | C39 H43 F N4 O5 | 666.32175 | 19.6 | -14.1 | -7.0 | 38.1 |
| 2-2-d1E01-2-0117 | C36 H37 F N6 O6 | 668.27586 | 0.0 | 0.3 | 0.0 | 0.0 |
| 2-2-d1E01-2-0118 | C36 H40 N6 O5 S | 668.27809 | 0.0 | 0.0 | 0.0 | 0.0 |
| 2-2-d1E01-2-0119 | C38 H44 N4 O5 S | 668.30324 | -0.5 | -28.4 | 49.4 | 66.6 |
| 2-2-dlE01-2-0120 | C36 H43 F3 N4 O5 | 668.31856 | -16.6 | 55.1 | -4.1 | 28.9 |
| 2-2-d1E01-2-0121 | C35 H35 N5 O5 S2 | 669.20796 | 18.1 | -2.6 | 25.6 | 6.2 |
| 2-2-d1E01-2-0122 | C36 H39 N5 O6 S | 669.26210 | 0.0 | 0.0 | 0.0 | 0.0 |
| 2-2-dlE01-2-0123 | C34 H34 N6 O7 S | 670.22097 | 27.6 | 0.5 | -0.6 | 3.2 |
| 2-2-d1E01-2-0124 | C36 H38 N4 O7 S | 670.24612 | -1.1 | -19.0 | 2.6 | 2.6 |
| 2-2-dlE01-2-0125 | C36 H38 N4 O7 S | 670.24612 | 2.2 | -7.0 | 3.3 | 10.7 |
| 2-2-d1E01-2-0126 | C36 H39 F N6 O6 | 670.29151 | 9.0 | 8.9 | -4.6 | 6.5 |
| 2-2-d1E01-2-0127 | C35 H41 F3 N4 O6 | 670.29782 | -7.4 | -63.0 | 16.0 | 13.4 |
| 2-2-dlE01-2-0128 | C41 H42 N4 O5 | 670.31552 | 37.5 | -19.1 | 64.1 | 31.4 |
| 2-2-d1E01-2-0129 | C41 H42 N4 O5 | 670.31552 | 37.0 | 54.5 | 64.8 | -9.1 |
| 2-2-d1E01-2-0130 | C35 H37 N5 O7 S | 671.24137 | 0.7 | -2.0 | 2.3 | 6.6 |
| 2-2-d1E01-2-0131 | C40 H41 N5 O5 | 671.31077 | 15.1 | -71.4 | -22.9 | 30.4 |
| 2-2-d1E01-2-0132 | C40 H41 N5 O5 | 671.31077 | 40.4 | -116.9 | 21.7 | 55.9 |
| 2-2-d1E01-2-0133 | C40 H41 N5 O5 | 671.31077 | -44.3 | -48.1 | -6.5 | 19.4 |
| 2-2-d1E01-2-0134 | C37 H41 F N4 O5 S | 672.27817 | 7.6 | -26.2 | 0.4 | 32.8 |
| 2-2-d1E01-2-0135 | C35 H36 F N5 O6 S | 673.23703 | -5.3 | 3.1 | -10.6 | 42.3 |
| 2-2-d1E01-2-0136 | C35 H38 N4 O8 S | 674.24103 | -6.3 | 0.8 | 5.6 | 4.8 |
| 2-2-d1E01-2-0137 | C37 H37 F3 N4 O5 | 674.27160 | 10.7 | -102.1 | -47.3 | 23.7 |
| 2-2-d1E01-2-0138 | C37 H37 F3 N4 O5 | 674.27160 | 5.4 | -20.8 | 11.5 | -1.0 |
| 2-2-d1E01-2-0139 | C40 H39 F N4 O5 | 674.29045 | 0.0 | 0.0 | -15.8 | 3.4 |
| 2-2-d1E01-2-0140 | C40 H39 F N4 O5 | 674.29045 | 3.5 | 13.8 | 22.1 | -1.1 |
| 2-2-dlE01-2-0141 | C41 H46 N4 O5 | 674.34682 | 78.9 | -6.5 | -4.4 | 23.0 |
| 2-2-dlE01-2-0142 | C36 H36 F3 N5 O5 | 675.26685 | 3.1 | 25.3 | 10.1 | 9.3 |
| 2-2-d1E01-2-0143 | C39 H38 F N5 O5 | 675.28570 | -20.3 | -8.6 | 50.6 | 11.6 |
| 2-2-d1E01-2-0144 | C39 H38 F N5 O5 | 675.28570 | -5.3 | -54.6 | 23.2 | 6.1 |
| 2-2-d1E01-2-0145 | C39 H38 F N5 O5 | 675.28570 | 1.3 | -31.2 | 20.6 | 16.0 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0146 | C40 H45 N5 O5 | 675.34207 | 0.2 | -14.3 | 9.7 | 10.0 |
| 2-2-d1E01-2-0147 | C34 H36 N4 O7 S2 | 676.20254 | 2.5 | -11.5 | 10.3 | 6.3 |
| 2-2-d1E01-2-0148 | C39 H40 N4 O7 | 676.28970 | 15.4 | -36.9 | 23.7 | 25.9 |
| 2-2-d1E01-2-0149 | C39 H44 N6 O5 | 676.33732 | 0.0 | 64.0 | 0.0 | 0.0 |
| 2-2-d1E01-2-0150 | C33 H35 N5 O9 S | 677.21555 | -4.8 | 5.6 | 2.5 | 3.8 |
| 2-2-d1E01-2-0151 | C38 H43 N7 O5 | 677.33257 | -13.7 | 26.9 | 7.1 | 28.9 |
| 2-2-d1E01-2-0152 | C34 H35 F N4 O8 S | 678.21596 | -5.7 | 7.8 | 2.9 | 8.7 |
| 2-2-d1E01-2-0153 | C36 H46 N4 O7 S | 678.30872 | -6.4 | -5.4 | 0.6 | 1.0 |
| 2-2-d1E01-2-0154 | C40 H46 N4 O6 | 678.34174 | 16.4 | 49.3 | 50.6 | -10.6 |
| 2-2-d1E01-2-0155 | C37 H37 N5 O6 S | 679.24645 | -4.6 | -30.1 | 63.1 | 59.4 |
| 2-2-d1E01-2-0156 | C39 H42 F N5 O5 | 679.31700 | 9.9 | 2.4 | -14.3 | 17.9 |
| 2-2-d1E01-2-0157 | C35 H35 F3 N4 O5 S | 680.22803 | -16.8 | 18.1 | 39.3 | -32.3 |
| 2-2-d1E01-2-0158 | C38 H37 F N4 O7 | 680.26463 | 13.4 | -8.8 | 23.2 | 69.0 |
| 2-2-d1E01-2-0159 | C35 H44 N4 O8 S | 680.28799 | 1.3 | 0.2 | 0.0 | 3.4 |
| 2-2-d1E01-2-0160 | C39 H44 N4 O5 S | 680.30324 | 0.0 | 0.0 | 0.0 | 0.0 |
| 2-2-d1E01-2-0161 | C38 H41 F N6 O5 | 680.31225 | -9.7 | 11.5 | -2.9 | 1.5 |
| 2-2-d1E01-2-0162 | C35 H35 N7 O6 S | 681.23695 | -8.7 | 41.3 | 1.7 | 14.8 |
| 2-2-d1E01-2-0163 | C34 H34 F3 N5 O7 | 681.24103 | 0.0 | 0.0 | 0.0 | 0.0 |
| 2-2-d1E01-2-0164 | C37 H40 F N7 O5 | 681.30750 | 7.5 | 17.8 | 0.2 | 7.7 |
| 2-2-dlE01-2-0165 | C35 H43 F N4 O7 S | 682.28365 | -5.2 | 14.8 | -2.3 | 3.0 |
| 2-2-d1E01-2-0166 | C37 H42 N6 O5 S | 682.29374 | 0.0 | 0.0 | 0.0 | 0.0 |
| 2-2-d1E01-2-0167 | C39 H43 F N4 O6 | 682.31666 | 6.6 | -75.5 | 14.9 | 13.3 |
| 2-2-d1E01-2-0168 | C35 H37 N7 O6 S | 683.25260 | 5.6 | 44.1 | 3.5 | 17.9 |
| 2-2-d1E01-2-0169 | C38 H45 N5 O5 S | 683.31414 | 5.7 | 44.6 | 9.0 | 24.2 |
| 2-2-d1E01-2-0170 | C37 H40 N4 O7 S | 684.26177 | 0.1 | 5.8 | 9.5 | 6.2 |
| 2-2-d1E01-2-0171 | C37 H40 N4 O7 S | 684.26177 | 8.1 | 27.3 | 4.3 | 12.3 |
| 2-2-dlE01-2-0172 | C34 H41 F N4 O8 S | 684.26291 | 1.5 | -4.6 | 0.1 | 4.3 |
| 2-2-d1E01-2-0173 | C36 H39 N5 O7 S | 685.25702 | -0.8 | 4.9 | -0.4 | 9.3 |
| 2-2-d1E01-2-0174 | C37 H43 N5 O6 S | 685.29340 | 21.7 | -48.9 | -12.7 | 25.4 |
| 2-2-d1E01-2-0175 | C34 H34 N6 O6 S2 | 686.19812 | 7.7 | -15.0 | 7.8 | 16.7 |
| 2-2-d1E01-2-0176 | C36 H38 N4 O8 S | 686.24103 | 9.9 | -26.9 | 0.6 | 13.6 |
| 2-2-d1E01-2-0177 | C36 H39 F N6 O5 S | 686.26867 | -2.4 | 4.8 | 0.0 | 0.0 |
| 2-2-d1E01-2-0178 | C39 H37 N5 O5 S | 687.25154 | 8.9 | 50.1 | 24.4 | -3.1 |
| 2-2-d1E01-2-0179 | C39 H37 N5 O5 S | 687.25154 | 0.0 | 0.0 | 0.0 | 0.0 |
| 2-2-d1E01-2-0180 | C34 H36 N6 O8 S | 688.23153 | 0.0 | -3.5 | 6.8 | 15.5 |
| 2-2-d1E01-2-0181 | C36 H37 F N4 O7 S | 688.23670 | -3.6 | -9.4 | 4.5 | 4.2 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0182 | C36 H37 F N4 O7 S | 688.23670 | 2.1 | -16.6 | 7.7 | 6.2 |
| 2-2-d1E01-2-0183 | C38 H36 N6 O5 S | 688.24679 | -15.5 | -19.6 | 31.3 | 22.5 |
| 2-2-d1E01-2-0184 | C38 H36 N6 O5 S | 688.24679 | 3.4 | -3.3 | 5.5 | 14.2 |
| 2-2-d1E01-2-0185 | C38 H36 N6 O5 S | 688.24679 | 0.0 | 0.0 | 0.0 | 0.0 |
| 2-2-d1E01-2-0186 | C40 H40 N4 O7 | 688.28970 | 9.9 | -8.2 | -5.2 | 3.9 |
| 2-2-d1E01-2-0187 | C42 H48 N4 O5 | 688.36247 | -30.8 | -537.4 | 30.3 | 4.0 |
| 2-2-d1E01-2-0188 | C35 H36 FN5 O7 S | 689.23195 | 0.2 | 6.1 | 3.4 | 4.9 |
| 2-2-d1E01-2-0189 | C39 H39 N5 O5 S | 689.26719 | -1.3 | 3.3 | 25.1 | 10.9 |
| 2-2-d1E01-2-0190 | C39 H39 N5 O5 S | 689.26719 | 12.0 | -22.7 | 59.0 | 65.4 |
| 2-2-d1E01-2-0191 | C35 H38 N4 O7 S2 | 690.21819 | 11.5 | -10.7 | 1.5 | 14.9 |
| 2-2-dlE01-2-0192 | C34 H38 N6 O8 S | 690.24718 | 3.1 | -19.2 | 4.8 | 3.9 |
| 2-2-d1E01-2-0193 | C38 H38 N6 O5 S | 690.26244 | 0.0 | -15.8 | 3.4 | 20.7 |
| 2-2-dlE01-2-0194 | C37 H37 F3 N4 O6 | 690.26652 | 34.6 | 68.4 | 13.8 | 16.5 |
| 2-2-d1E01-2-0195 | C37 H46 N4 O7 S | 690.30872 | 2.4 | 0.0 | 27.7 | -6.9 |
| 2-2-d1E01-2-0196 | C40 H46 N6 O5 | 690.35297 | 14.0 | -0.3 | 6.4 | 22.9 |
| 2-2-d1E01-2-0197 | C34 H37 N5 O9 S | 691.23120 | -0.7 | -19.5 | 6.9 | 2.0 |
| 2-2-d1E01-2-0198 | C35 H35 F3 N6 O6 | 692.25702 | 0.0 | 0.0 | 0.0 | 0.0 |
| 2-2-d1E01-2-0199 | C38 H40 N6 O5 S | 692.27809 | -4.6 | 1.8 | 20.8 | 16.3 |
| 2-2-d1E01-2-0200 | C36 H44 N4 O8 S | 692.28799 | 4.3 | 0.0 | -3.2 | 9.5 |
| 2-2-d1E01-2-0201 | C41 H45 F N4 O5 | 692.33740 | 24.5 | 14.2 | 27.8 | -1661.0 |
| 2-2-d1E01-2-0202 | C33 H35 N5 O8 S2 | 693.19270 | 6.7 | -5.7 | -1.0 | 3.8 |
| 2-2-d1E01-2-0203 | C37 H39 N7 O5 S | 693.27334 | -5.1 | 1.7 | 0.0 | 0.0 |
| 2-2-d1E01-2-0204 | C34 H35 F N4 O7 S2 | 694.19312 | 3.7 | 3.5 | 2.2 | 5.7 |
| 2-2-d1E01-2-0205 | C38 H38 N4 O7 S | 694.24612 | -1.1 | 35.8 | 6.3 | 10.8 |
| 2-2-d1E01-2-0206 | C38 H38 N4 O7 S | 694.24612 | -5.6 | 16.2 | 2.9 | 3.2 |
| 2-2-d1E01-2-0207 | C36 H38 N8 O5 S | 694.26859 | -6.3 | -13.8 | 17.3 | 16.7 |
| 2-2-d1E01-2-0208 | C35 H37 F3 N6 O6 | 694.27267 | 10.0 | -19.6 | -0.3 | 15.7 |
| 2-2-d1E01-2-0209 | C40 H46 N4 O5 S | 694.31889 | 23.9 | 20.3 | 4.1 | 20.7 |
| 2-2-d1E01-2-0210 | C38 H45 F3 N4 O5 | 694.33421 | -2.0 | -34.0 | 45.6 | -4.4 |
| 2-2-d1E01-2-0211 | C33 H34 F N5 O9 S | 695.20613 | -5.5 | -11.5 | 3.7 | 4.9 |
| 2-2-d1E01-2-0212 | C37 H37 N5 O5 S2 | 695.22361 | 5.1 | 34.4 | 3.3 | 15.7 |
| 2-2-d1E01-2-0213 | C37 H37 N5 O7 S | 695.24137 | -3.2 | 10.6 | 5.3 | 9.0 |
| 2-2-d1E01-2-0214 | C37 H37 N5 O7 S | 695.24137 | 27.4 | 6.2 | 4.4 | 18.3 |
| 2-2-d1E01-2-0215 | C37 H37 N5 O7 S | 695.24137 | -0.3 | 0.0 | 1.9 | 4.7 |
| 2-2-dlE01-2-0216 | C38 H41 N5 O6 S | 695.27775 | 10.8 | -31.3 | -5.9 | 25.9 |
| 2-2-d1E01-2-0217 | C36 H36 N6 O7 S | 696.23662 | 0.0 | 0.0 | -29.0 | -5.9 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-dlE01-2-0218 | C38 H40 N4 O7 S | 696.26177 | -1.8 | 0.0 | 0.0 | 0.0 |
| 2-2-dlE01-2-0219 | C38 H40 N4 O7 S | 696.26177 | 0.0 | 0.0 | 169.3 | 0.0 |
| 2-2-dlE01-2-0220 | C37 H43 F3 N4 O6 | 696.31347 | -11.6 | -8.0 | 22.2 | 2.3 |
| 2-2-d1E01-2-0221 | C34 H34 F3 N5 O6 S | 697.21819 | -27.4 | 0.0 | 39.2 | 45.3 |
| 2-2-d1E01-2-0222 | C37 H39 N5 O7 S | 697.25702 | -4.3 | 3.1 | -0.4 | 7.1 |
| 2-2-d1E01-2-0223 | C39 H37 F3 N4 O5 | 698.27160 | -17.6 | -52.5 | 15.7 | 13.6 |
| 2-2-d1E01-2-0224 | C39 H37 F3 N4 O5 | 698.27160 | -29.8 | 0.0 | 0.0 | -7.3 |
| 2-2-dlE01-2-0225 | C39 H43 F N4 O5 S | 698.29382 | 76.1 | -19.4 | 15.1 | 8.3 |
| 2-2-d1E01-2-0226 | C35 H37 N7 O5 S2 | 699.22976 | 23.4 | -48.1 | -11.5 | 25.5 |
| 2-2-d1E01-2-0227 | C38 H36 F3 N5 O5 | 699.26685 | -110.3 | -71.6 | -1.5 | 17.2 |
| 2-2-d1E01-2-0228 | C38 H36 F3 N5 O5 | 699.26685 | 17.4 | -83.5 | -25.9 | 31.6 |
| 2-2-d1E01-2-0229 | C38 H36 F3 N5 O5 | 699.26685 | 12.5 | -24.0 | 0.4 | 46.3 |
| 2-2-d1E01-2-0230 | C37 H41 N5 O7 S | 699.27267 | 7.1 | -11.4 | 7.3 | 8.2 |
| 2-2-d1E01-2-0231 | C37 H40 N4 O8 S | 700.25668 | 1.6 | -15.0 | 6.9 | 0.0 |
| 2-2-d1E01-2-0232 | C36 H40 N6 O7 S | 700.26792 | -4.1 | 0.2 | 2.3 | 1.9 |
| 2-2-d1E01-2-0233 | C39 H39 F3 N4 O5 | 700.28725 | 5.1 | 1.8 | 21.9 | 31.8 |
| 2-2-d1E01-2-0234 | C39 H39 F3 N4 O5 | 700.28725 | 0.0 | 0.0 | 0.0 | 0.0 |
| 2-2-d1E01-2-0235 | C35 H39 N7 O7 S | 701.26317 | 6.8 | 6.3 | 17.5 | 26.8 |
| 2-2-d1E01-2-0236 | C38 H38 F3 N5 O5 | 701.28250 | -1.1 | -12.5 | 1.0 | 30.5 |
| 2-2-d1E01-2-0237 | C36 H38 N4 O7 S2 | 702.21819 | 0.0 | 0.0 | 0.0 | 0.0 |
| 2-2-d1E01-2-0238 | C35 H38 N6 O8 S | 702.24718 | 7.3 | -1.1 | 3.9 | 7.7 |
| 2-2-d1E01-2-0239 | C37 H42 N4 O8 S | 702.27234 | -3.0 | -15.7 | 3.5 | 2.8 |
| 2-2-d1E01-2-0240 | C41 H42 N4 O7 | 702.30535 | 2.8 | 28.8 | 23.1 | 62.3 |
| 2-2-d1E01-2-0241 | C35 H37 N5 O9 S | 703.23120 | 4.8 | -11.9 | -3.4 | -12.4 |
| 2-2-d1E01-2-0242 | C38 H40 F3 N5 O5 | 703.29815 | 21.6 | 42.6 | -7.1 | 14.8 |
| 2-2-d1E01-2-0243 | C36 H37 F N4 O8 S | 704.23161 | 6.9 | -14.6 | -0.6 | 13.6 |
| 2-2-d1E01-2-0244 | C35 H40 N6 O8 S | 704.26283 | 3.1 | -3.1 | 7.7 | 6.3 |
| 2-2-d1E01-2-0245 | C37 H39 F3 N6 O5 | 704.29340 | 1.6 | -48.7 | 19.6 | -6.4 |
| 2-2-d1E01-2-0246 | C38 H48 N4 O7 S | 704.32437 | -19.3 | 33.2 | -0.3 | -0.5 |
| 2-2-d1E01-2-0247 | C41 H48 N6 O5 | 704.36862 | -8.6 | -14.3 | 5.1 | 21.6 |
| 2-2-d1E01-2-0248 | C36 H38 F3 N7 O5 | 705.28865 | 23.7 | -53.2 | -16.7 | 31.9 |
| 2-2-d1E01-2-0249 | C40 H43 N5 O5 S | 705.29849 | -20.4 | -0.8 | 18.9 | -8.6 |
| 2-2-d1E01-2-0250 | C34 H35 F N6 O8 S | 706.22211 | -5.4 | 0.0 | 20.0 | 13.4 |
| 2-2-d1E01-2-0251 | C34 H38 N6 O7 S2 | 706.22434 | -3.6 | -4.3 | 0.0 | 8.3 |
| 2-2-d1E01-2-0252 | C37 H37 F3 N4 O5 S | 706.24368 | 0.0 | 0.0 | 0.0 | 0.0 |
| 2-2-d1E01-2-0253 | C40 H39 F N4 O7 | 706.28028 | -51.5 | -28.1 | -8.5 | -31.1 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0254 | C38 H41 F3 N4 O6 | 706.29782 | 11.8 | 3.2 | 20.4 | 9.0 |
| 2-2-d1E01-2-0255 | C37 H46 N4 O8 S | 706.30364 | 0.0 | 6.2 | 1.4 | 4.7 |
| 2-2-d1E01-2-0256 | C34 H37 N5 O8 S2 | 707.20835 | 1.5 | 12.8 | 3.3 | 8.4 |
| 2-2-d1E01-2-0257 | C37 H37 N7 O6 S | 707.25260 | -13.3 | 13.6 | 18.1 | 5.4 |
| 2-2-d1E01-2-0258 | C36 H36 F3 N5 O7 | 707.25668 | 0.0 | 0.0 | 0.0 | 0.0 |
| 2-2-d1E01-2-0259 | C34 H37 F N6 O8 S | 708.23776 | 1.3 | 1.3 | 0.7 | 9.0 |
| 2-2-d1E01-2-0260 | C39 H40 N4 O7 S | 708.26177 | -5.7 | -15.2 | 3.2 | 4.3 |
| 2-2-d1E01-2-0261 | C39 H40 N4 O7 S | 708.26177 | -1.6 | -16.2 | -20.6 | 3.6 |
| 2-2-d1E01-2-0262 | C37 H45 F N4 O7 S | 708.29930 | -16.7 | 1.2 | -5.0 | 5.1 |
| 2-2-d1E01-2-0263 | C40 H45 F N6 O5 | 708.34355 | 26.3 | -3.9 | 5.6 | 23.1 |
| 2-2-d1E01-2-0264 | C38 H39 N5 O7 S | 709.25702 | -4.8 | -82.4 | 2.9 | 5.0 |
| 2-2-d1E01-2-0265 | C38 H39 N5 O7 S | 709.25702 | 27.1 | 0.0 | 0.0 | 0.0 |
| 2-2-d1E01-2-0266 | C38 H39 N5 O7 S | 709.25702 | 8.2 | -0.6 | 24.5 | 24.6 |
| 2-2-d1E01-2-0267 | C37 H39 N7 O6 S | 709.26825 | 26.2 | -49.7 | 3.2 | 40.3 |
| 2-2-d1E01-2-0268 | C35 H37 F3 N6 O5 S | 710.24982 | 10.2 | 58.2 | 6.4 | -4.1 |
| 2-2-d1E01-2-0269 | C39 H42 N4 O7 S | 710.27742 | -14.4 | 0.0 | 10.1 | -5.9 |
| 2-2-d1E01-2-0270 | C39 H42 N4 O7 S | 710.27742 | 0.0 | 0.0 | 0.0 | 0.0 |
| 2-2-d1E01-2-0271 | C36 H43 F N4 O8 S | 710.27856 | 6.1 | -4.8 | -2.8 | 5.9 |
| 2-2-d1E01-2-0272 | C33 H34 F N5 O8 S2 | 711.18328 | -8.6 | -6.9 | 2.5 | 5.0 |
| 2-2-d1E01-2-0273 | C38 H41 N5 O5 S2 | 711.25491 | 4.8 | 28.2 | 25.7 | 0.0 |
| 2-2-d1E01-2-0274 | C38 H41 N5 O7 S | 711.27267 | 0.5 | 12.7 | 3.6 | 10.1 |
| 2-2-d1E01-2-0275 | C36 H36 N6 O6 S2 | 712.21377 | 8.0 | 8.0 | 21.1 | 26.8 |
| 2-2-d1E01-2-0276 | C38 H37 F N4 O7 S | 712.23670 | 1.8 | 13.1 | 9.3 | 10.9 |
| 2-2-d1E01-2-0277 | C38 H37 F N4 O7 S | 712.23670 | -5.6 | 5.4 | 5.7 | -2.2 |
| 2-2-d1E01-2-0278 | C39 H44 N4 O7 S | 712.29307 | 0.3 | 38.3 | 2.7 | 16.9 |
| 2-2-d1E01-2-0279 | C37 H36 F N5 O7 S | 713.23195 | -3.4 | 27.9 | 1.8 | 10.2 |
| 2-2-d1E01-2-0280 | C37 H36 F N5 O7 S | 713.23195 | 14.4 | 41.7 | 9.0 | 12.7 |
| 2-2-d1E01-2-0281 | C37 H36 F N5 O7 S | 713.23195 | 0.0 | 0.0 | 0.0 | 0.0 |
| 2-2-d1E01-2-0282 | C41 H39 N5 O5 S | 713.26719 | 10.8 | -61.4 | 38.7 | 101.8 |
| 2-2-d1E01-2-0283 | C41 H39 N5 O5 S | 713.26719 | 0.0 | 0.0 | 0.0 | 0.0 |
| 2-2-d1E01-2-0284 | C38 H43 N5 O7 S | 713.28832 | 5.7 | -3.6 | -0.3 | 9.6 |
| 2-2-d1E01-2-0285 | C36 H38 N6 O8 S | 714.24718 | 0.0 | 0.0 | 0.0 | 0.0 |
| 2-2-d1E01-2-0286 | C38 H39 F N4 O7 S | 714.25235 | 0.0 | 0.0 | 5.1 | 7.5 |
| 2-2-d1E01-2-0287 | C38 H39 F N4 O7 S | 714.25235 | -33.6 | 53.2 | -19.9 | 0.0 |
| 2-2-d1E01-2-0288 | C40 H38 N6 O5 S | 714.26244 | 26.1 | 14.5 | 6.4 | 14.9 |
| 2-2-d1E01-2-0289 | C40 H38 N6 O5 S | 714.26244 | 1.2 | -599.4 | 22.7 | 52.1 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0290 | C40 H38 N6 O5 S | 714.26244 | -16.9 | -34.4 | 39.2 | 29.4 |
| 2-2-d1E01-2-0291 | C37 H42 N6 O7 S | 714.28357 | 0.0 | 0.0 | 0.0 | 0.0 |
| 2-2-d1E01-2-0292 | C37 H38 F N5 O7 S | 715.24760 | -6.3 | -3.3 | 6.0 | 6.4 |
| 2-2-d1E01-2-0293 | C36 H41 N7 O7 S | 715.27882 | 9.2 | 10.9 | 16.0 | 12.5 |
| 2-2-d1E01-2-0294 | C37 H40 N4 O7 S2 | 716.23384 | -7.0 | -9.1 | 0.0 | 0.0 |
| 2-2-d1E01-2-0295 | C36 H40 N6 O8 S | 716.26283 | -1.8 | -3.0 | 9.0 | 10.8 |
| 2-2-d1E01-2-0296 | C38 H44 N4 O8 S | 716.28799 | 3.3 | 28.8 | 6.5 | 6.9 |
| 2-2-d1E01-2-0297 | C40 H43 F3 N4 O5 | 716.31856 | 21.4 | 8.5 | -6.8 | 37.9 |
| 2-2-d1E01-2-0298 | C42 H48 N6 O5 | 716.36862 | 7.7 | -11.8 | 6.8 | 22.3 |
| 2-2-d1E01-2-0299 | C35 H35 N5 O8 S2 | 717.19270 | -0.6 | 15.5 | 10.4 | 8.8 |
| 2-2-d1E01-2-0300 | C36 H39 N5 O9 S | 717.24685 | -9.7 | 0.0 | -9.4 | 17.9 |
| 2-2-d1E01-2-0301 | C37 H40 F N5 O7 S | 717.26325 | 1.2 | -14.7 | -1.4 | 2.2 |
| 2-2-d1E01-2-0302 | C37 H42 N4 O7 S2 | 718.24949 | -3.0 | -0.7 | -0.7 | 8.3 |
| 2-2-d1E01-2-0303 | C36 H39 F N6 O7 S | 718.25850 | -6.2 | 0.4 | -4.2 | 8.0 |
| 2-2-d1E01-2-0304 | C37 H37 F3 N6 O6 | 718.27267 | 3.2 | -0.5 | 0.0 | 0.0 |
| 2-2-d1E01-2-0305 | C40 H42 N6 O5 S | 718.29374 | 0.2 | 6.0 | 4.8 | 5.6 |
| 2-2-d1E01-2-0306 | C35 H37 N5 O8 S2 | 719.20835 | -13.8 | -29.5 | 20.9 | 6.2 |
| 2-2-d1E01-2-0307 | C39 H37 N5 O7 S | 719.24137 | -12.9 | 0.0 | 0.4 | 0.0 |
| 2-2-d1E01-2-0308 | C35 H38 F N7 O7 S | 719.25375 | 3.3 | 8.3 | 10.3 | 19.2 |
| 2-2-d1E01-2-0309 | C39 H41 N7 O5 S | 719.28899 | -14.2 | -43.2 | 33.4 | 35.6 |
| 2-2-d1E01-2-0310 | C36 H37 F N4 O7 S2 | 720.20877 | 0.0 | 0.0 | -1.0 | 8.9 |
| 2-2-d1E01-2-0311 | C35 H40 N6 O7 S2 | 720.23999 | -2.8 | 2.0 | 9.9 | 10.8 |
| 2-2-d1E01-2-0312 | C40 H40 N4 O7 S | 720.26177 | 0.0 | 0.0 | 25.4 | 0.4 |
| 2-2-d1E01-2-0313 | C40 H40 N4 O7 S | 720.26177 | -1.9 | 11.0 | 7.5 | 5.4 |
| 2-2-d1E01-2-0314 | C37 H41 F N4 O8 S | 720.26291 | -4.9 | -9.6 | 3.8 | 4.7 |
| 2-2-d1E01-2-0315 | C38 H40 N8 O5 S | 720.28424 | -5.4 | -25.0 | 46.0 | 26.9 |
| 2-2-d1E01-2-0316 | C37 H39 F3 N6 O6 | 720.28832 | -1.4 | 67.2 | 32.6 | 10.4 |
| 2-2-d1E01-2-0317 | C35 H36 F N5 O9 S | 721.22178 | 8.1 | 0.0 | 1.8 | 9.1 |
| 2-2-d1E01-2-0318 | C39 H39 N5 O7 S | 721.25702 | 25.4 | -9.5 | 8.2 | 0.0 |
| 2-2-d1E01-2-0319 | C39 H39 N5 O7 S | 721.25702 | 9.6 | 44.2 | -10.8 | 21.2 |
| 2-2-d1E01-2-0320 | C39 H39 N5 O7 S | 721.25702 | 0.0 | 0.0 | 0.0 | 0.0 |
| 2-2-d1E01-2-0321 | C36 H43 N5 O7 S2 | 721.26039 | 3.3 | 1.8 | 4.9 | 5.9 |
| 2-2-d1E01-2-0322 | C40 H43 N5 O6 S | 721.29340 | 14.9 | -14.6 | -6.9 | 9.3 |
| 2-2-d1E01-2-0323 | C38 H41 F3 N4 O5 S | 722.27498 | 34.6 | 55.8 | 18.1 | -22.9 |
| 2-2-d1E01-2-0324 | C36 H36 F3 N5 O6 S | 723.23384 | -7.4 | 10.2 | 15.0 | 37.3 |
| 2-2-d1E01-2-0325 | C35 H41 N5 O8 S2 | 723.23965 | -5.5 | -5.9 | -1.1 | 1.1 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0326 | C34 H37 F N6 O7 S2 | 724.21492 | 1.2 | 8.5 | 11.1 | 10.4 |
| 2-2-d1E01-2-0327 | C41 H39 F3 N4 O5 | 724.28725 | -12.4 | -46.9 | 47.6 | 32.0 |
| 2-2-d1E01-2-0328 | C41 H39 F3 N4 O5 | 724.28725 | -107.4 | -70.9 | -4.4 | 21.8 |
| 2-2-d1E01-2-0329 | C37 H39 N7 O5 S2 | 725.24541 | 20.6 | 8.5 | -1.4 | -3.9 |
| 2-2-d1E01-2-0330 | C40 H38 F3 N5 O5 | 725.28250 | 1.7 | 36.4 | 25.0 | 39.2 |
| 2-2-d1E01-2-0331 | C40 H38 F3 N5 O5 | 725.28250 | 1.3 | -127.8 | -2.3 | 16.3 |
| 2-2-d1E01-2-0332 | C40 H38 F3 N5 O5 | 725.28250 | -73.0 | -443.2 | 0.0 | -1184.1 |
| 2-2-d1E01-2-0333 | C39 H43 N5 O7 S | 725.28832 | 1.2 | 0.0 | 0.0 | 0.0 |
| 2-2-d1E01-2-0334 | C38 H38 N4 O9 S | 726.23595 | -1.6 | -17.1 | -1.3 | 1.3 |
| 2-2-d1E01-2-0335 | C38 H42 N6 O7 S | 726.28357 | 30.1 | -6.5 | -18.4 | 31.6 |
| 2-2-d1E01-2-0336 | C40 H46 N4 O7 S | 726.30872 | -7.0 | -8.9 | 11.5 | 8.9 |
| 2-2-d1E01-2-0337 | C37 H37 N5 O7 S2 | 727.21344 | -7.4 | -3.0 | 8.2 | 8.5 |
| 2-2-d1E01-2-0338 | C37 H37 N5 O7 S2 | 727.21344 | 1.9 | 8.0 | -0.8 | 8.1 |
| 2-2-d1E01-2-0339 | C37 H41 N7 O7 S | 727.27882 | 0.0 | 0.0 | 0.0 | 0.0 |
| 2-2-d1E01-2-0340 | C36 H36 N6 O7 S2 | 728.20869 | -1.2 | -1.3 | 4.4 | 8.0 |
| 2-2-d1E01-2-0341 | C35 H35 F3 N4 O8 S | 728.21277 | -19.3 | 48.6 | 1.5 | 13.9 |
| 2-2-d1E01-2-0342 | C37 H40 N6 O8 S | 728.26283 | 0.0 | -14.8 | 0.0 | 0.0 |
| 2-2-d1E01-2-0343 | C39 H44 N4 O8 S | 728.28799 | -35.1 | 11.3 | 45.0 | 35.8 |
| 2-2-d1E01-2-0344 | C40 H42 F3 N5 O5 | 729.31380 | -5.1 | 6.3 | 0.0 | 10.7 |
| 2-2-d1E01-2-0345 | C39 H37 F3 N4 O7 | 730.26143 | -56.8 | -4.3 | 5.7 | -3.6 |
| 2-2-d1E01-2-0346 | C37 H42 N6 O8 S | 730.27848 | 1.0 | -0.4 | -5.8 | 7.6 |
| 2-2-d1E01-2-0347 | C39 H43 F N4 O7 S | 730.28365 | 9.9 | 4.1 | -8.9 | 13.3 |
| 2-2-d1E01-2-0348 | C39 H41 F3 N6 O5 | 730.30905 | 5.0 | -278.3 | 4.8 | 12.3 |
| 2-2-d1E01-2-0349 | C43 H50 N6 O5 | 730.38427 | -5.6 | -6.8 | 15.0 | 14.5 |
| 2-2-d1E01-2-0350 | C38 H40 F3 N7 O5 | 731.30430 | 26.8 | 68.9 | 15.5 | 21.1 |
| 2-2-d1E01-2-0351 | C42 H45 N5 O5 S | 731.31414 | -15.6 | -67.1 | -8.1 | 17.4 |
| 2-2-d1E01-2-0352 | C36 H37 F N6 O8 S | 732.23776 | -7.3 | -3.9 | 0.3 | 4.1 |
| 2-2-d1E01-2-0353 | C36 H40 N6 O7 S2 | 732.23999 | 0.0 | 0.0 | 0.0 | 0.0 |
| 2-2-d1E01-2-0354 | C38 H44 N4 O7 S2 | 732.26514 | 1.2 | 11.5 | 17.0 | -22.5 |
| 2-2-d1E01-2-0355 | C36 H43 F3 N4 O7 S | 732.28046 | -0.7 | -13.9 | -16.2 | 0.9 |
| 2-2-d1E01-2-0356 | C40 H43 F3 N4 O6 | 732.31347 | -0.6 | -3.8 | -26.4 | 5.3 |
| 2-2-d1E01-2-0357 | C35 H35 N5 O7 S3 | 733.16986 | 9.2 | 19.3 | -3.5 | 6.9 |
| 2-2-d1E01-2-0358 | C36 H39 N5 O8 S2 | 733.22400 | 24.5 | -4.2 | 2.5 | 6.3 |
| 2-2-d1E01-2-0359 | C34 H34 N6 O9 S2 | 734.18287 | -2.3 | -3.7 | 2.0 | 2.7 |
| 2-2-d1E01-2-0360 | C36 H39 F N6 O8 S | 734.25341 | -0.7 | -3.5 | 11.7 | 15.4 |
| 2-2-d1E01-2-0361 | C35 H41 F3 N4 O8 S | 734.25972 | 5.0 | 8.0 | 1.0 | 4.3 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0362 | C41 H42 N4 O7 S | 734.27742 | 0.0 | 0.0 | 0.0 | 0.0 |
| 2-2-d1E01-2-0363 | C41 H42 N4 O7 S | 734.27742 | 7.7 | -23.0 | 0.0 | 14.7 |
| 2-2-d1E01-2-0364 | C42 H47 F N6 O5 | 734.35920 | 22.0 | 55.5 | -5.2 | 27.1 |
| 2-2-d1E01-2-0365 | C40 H41 N5 O7 S | 735.27267 | -6.7 | 47.4 | 7.9 | 8.5 |
| 2-2-d1E01-2-0366 | C40 H41 N5 O7 S | 735.27267 | 1.5 | -14.7 | 31.9 | 31.6 |
| 2-2-d1E01-2-0367 | C40 H41 N5 O7 S | 735.27267 | 0.0 | 0.0 | 0.0 | 0.0 |
| 2-2-d1E01-2-0368 | C37 H41 F N4 O7 S2 | 736.24007 | 1.2 | 17.4 | -3.9 | 6.3 |
| 2-2-d1E01-2-0369 | C37 H39 F3 N6 O5 S | 736.26547 | 24.5 | 11.7 | -20.2 | 40.5 |
| 2-2-d1E01-2-0370 | C35 H36 F N5 O8 S2 | 737.19893 | -5.1 | 16.0 | 4.4 | -4.2 |
| 2-2-d1E01-2-0371 | C40 H43 N5 O5 S2 | 737.27056 | 18.2 | -16.9 | -21.2 | 55.9 |
| 2-2-d1E01-2-0372 | C37 H37 F3 N4 O7 S | 738.23350 | -15.8 | 33.0 | 2.5 | 2.2 |
| 2-2-d1E01-2-0373 | C37 H37 F3 N4 O7 S | 738.23350 | 18.6 | 34.0 | -1.5 | 6.7 |
| 2-2-d1E01-2-0374 | C40 H39 F N4 O7 S | 738.25235 | 0.0 | 0.0 | 8.0 | 0.0 |
| 2-2-d1E01-2-0375 | C40 H39 F N4 O7 S | 738.25235 | -11.4 | -0.5 | 0.0 | 17.5 |
| 2-2-d1E01-2-0376 | C41 H46 N4 O7 S | 738.30872 | 7.7 | -31.6 | 5.3 | 2.9 |
| 2-2-d1E01-2-0377 | C36 H36 F3 N5 O7 S | 739.22875 | 5.0 | 3.4 | 1.4 | 12.0 |
| 2-2-d1E01-2-0378 | C39 H38 F N5 O7 S | 739.24760 | 12.8 | -11.0 | 10.4 | 7.7 |
| 2-2-d1E01-2-0379 | C39 H38 F N5 O7 S | 739.24760 | 0.0 | 0.0 | 0.0 | 0.0 |
| 2-2-d1E01-2-0380 | C39 H38 F N5 O7 S | 739.24760 | 7.3 | 18.9 | 9.8 | 21.8 |
| 2-2-d1E01-2-0381 | C40 H45 N5 O7 S | 739.30397 | 0.0 | 0.0 | 0.0 | 0.0 |
| 2-2-d1E01-2-0382 | C39 H40 N4 O9 S | 740.25160 | 11.5 | 10.4 | 3.4 | 7.4 |
| 2-2-d1E01-2-0383 | C39 H44 N6 O7 S | 740.29922 | 0.0 | 0.0 | 0.0 | 0.0 |
| 2-2-d1E01-2-0384 | C38 H43 N7 O7 S | 741.29447 | 11.9 | 23.3 | 15.3 | 16.0 |
| 2-2-d1E01-2-0385 | C40 H46 N4 O8 S | 742.30364 | 0.0 | 0.0 | 0.0 | 0.0 |
| 2-2-d1E01-2-0386 | C42 H45 F3 N4 O5 | 742.33421 | -30.2 | -1428.2 | 18.6 | 74.9 |
| 2-2-d1E01-2-0387 | C37 H37 N5 O8 S2 | 743.20835 | 1.9 | 10.6 | -3.6 | 7.8 |
| 2-2-d1E01-2-0388 | C39 H42 F N5 O7 S | 743.27890 | 32.2 | -5.3 | 7.8 | 10.7 |
| 2-2-d1E01-2-0389 | C35 H35 F3 N4 O7 S2 | 744.18993 | -12.2 | 0.0 | -17.4 | 15.6 |
| 2-2-d1E01-2-0390 | C38 H37 F N4 O9 S | 744.22653 | -6.0 | -7.4 | 2.5 | 4.7 |
| 2-2-d1E01-2-0391 | C39 H44 N4 O7 S2 | 744.26514 | 2.6 | -6.8 | 4.6 | 0.0 |
| 2-2-d1E01-2-0392 | C38 H41 F N6 O7 S | 744.27415 | 0.0 | 0.0 | 0.0 | 0.0 |
| 2-2-d1E01-2-0393 | C35 H35 N7 O8 S2 | 745.19885 | 3.3 | -9.7 | 6.3 | 2.8 |
| 2-2-d1E01-2-0394 | C34 H34 F3 N5 O9 S | 745.20293 | 9.8 | 14.7 | 21.1 | 0.0 |
| 2-2-d1E01-2-0395 | C41 H39 N5 O7 S | 745.25702 | -1.2 | -159.3 | -8.5 | 48.6 |
| 2-2-d1E01-2-0396 | C37 H40 F N7 O7 S | 745.26940 | 5.5 | -12.9 | 11.2 | 12.1 |
| 2-2-d1E01-2-0397 | C37 H42 N6 O7 S2 | 746.25564 | 0.0 | 0.0 | 0.0 | 0.0 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0398 | C39 H43 F N4 O8 S | 746.27856 | 0.5 | -2.6 | -14.1 | -6.9 |
| 2-2-d1E01-2-0399 | C35 H37 N7 O8 S2 | 747.21450 | -5.0 | -8.0 | 2.3 | 6.6 |
| 2-2-d1E01-2-0400 | C38 H45 N5 O7 S2 | 747.27604 | -8.6 | -14.8 | 0.1 | 5.3 |
| 2-2-d1E01-2-0401 | C41 H45 N7 O5 S | 747.32029 | -34.6 | 82.2 | -16.0 | 15.7 |
| 2-2-d1E01-2-0402 | C40 H43 F3 N4 O5 S | 748.29063 | -32.9 | -46.6 | -9.8 | 21.5 |
| 2-2-d1E01-2-0403 | C37 H43 N5 O8 S2 | 749.25530 | 7.6 | -18.9 | -2.0 | 1.6 |
| 2-2-d1E01-2-0404 | C34 H34 N6 O8 S3 | 750.16002 | 6.3 | 15.9 | 3.7 | 6.8 |
| 2-2-d1E01-2-0405 | C36 H39 F N6 O7 S2 | 750.23057 | 15.7 | 16.4 | 44.6 | 4.5 |
| 2-2-d1E01-2-0406 | C39 H37 N5 O7 S2 | 751.21344 | -0.8 | -10.5 | 0.0 | 16.6 |
| 2-2-d1E01-2-0407 | C39 H37 N5 O7 S2 | 751.21344 | 5.9 | -5.8 | 8.8 | 13.4 |
| 2-2-d1E01-2-0408 | C38 H36 N6 O7 S2 | 752.20869 | 1.3 | 3.2 | 0.0 | 0.0 |
| 2-2-d1E01-2-0409 | C38 H36 N6 O7 S2 | 752.20869 | -54.9 | 66.2 | 7.9 | 43.9 |
| 2-2-d1E01-2-0410 | C38 H36 N6 O7 S2 | 752.20869 | 0.0 | 0.0 | 0.6 | 3.8 |
| 2-2-d1E01-2-0411 | C40 H40 N4 O9 S | 752.25160 | 79.1 | 78.4 | 72.9 | 84.8 |
| 2-2-d1E01-2-0412 | C42 H48 N4 O7 S | 752.32437 | -39.4 | 42.7 | 42.3 | 59.9 |
| 2-2-d1E01-2-0413 | C39 H39 N5 O7 S2 | 753.22909 | 0.6 | -13.0 | 30.5 | 19.7 |
| 2-2-d1E01-2-0414 | C39 H39 N5 O7 S2 | 753.22909 | 5.9 | -17.7 | -2.9 | 13.0 |
| 2-2-d1E01-2-0415 | C38 H38 N6 O7 S2 | 754.22434 | 1.1 | 0.9 | 6.2 | 7.5 |
| 2-2-d1E01-2-0416 | C37 H37 F3 N4 O8 S | 754.22842 | 0.0 | -9.6 | 0.0 | 12.8 |
| 2-2-d1E01-2-0417 | C40 H46 N6 O7 S | 754.31487 | 4.4 | 9.8 | 2.6 | 11.7 |
| 2-2-d1E01-2-0418 | C35 H35 F3 N6 O8 S | 756.21892 | 2.3 | -2.7 | 6.6 | 9.7 |
| 2-2-d1E01-2-0419 | C38 H40 N6 O7 S2 | 756.23999 | 9.1 | 7.4 | 2.8 | 8.0 |
| 2-2-d1E01-2-0420 | C41 H39 F3 N4 O7 | 756.27708 | 45.4 | 20.2 | -31.7 | 21.5 |
| 2-2-d1E01-2-0421 | C41 H45 FN4 O7 S | 756.29930 | 2.8 | 18.0 | 3.9 | 6.5 |
| 2-2-d1E01-2-0422 | C37 H39 N7 O7 S2 | 757.23524 | -8.1 | 9.4 | -3.0 | 5.2 |
| 2-2-d1E01-2-0423 | C36 H38 N8 O7 S2 | 758.23049 | 0.0 | -6.7 | 0.0 | 0.0 |
| 2-2-d1E01-2-0424 | C35 H37 F3 N6 O8 S | 758.23457 | 6.2 | 100.6 | 5.9 | 8.4 |
| 2-2-d1E01-2-0425 | C40 H46 N4 O7 S2 | 758.28079 | 0.0 | 0.0 | 0.0 | 0.0 |
| 2-2-d1E01-2-0426 | C38 H45 F3 N4 O7 S | 758.29611 | 46.2 | -5.5 | 84.9 | -5.7 |
| 2-2-d1E01-2-0427 | C41 H45 F3 N6 O5 | 758.34035 | -11.1 | -33.9 | -3.0 | 22.0 |
| 2-2-d1E01-2-0428 | C37 H37 N5 O7 S3 | 759.18551 | -2.2 | -10.3 | -1.4 | 10.7 |
| 2-2-d1E01-2-0429 | C38 H41 N5 O8 S2 | 759.23965 | -2.5 | 0.6 | 2.4 | 8.7 |
| 2-2-dlE01-2-0430 | C36 H36 N6 O9 S2 | 760.19852 | -3.6 | 3.6 | 5.4 | 2.7 |
| 2-2-dlE01-2-0431 | C37 H43 F3 N4 O8 S | 760.27537 | 9.2 | -16.6 | -0.1 | 13.2 |
| 2-2-dlE01-2-0432 | C34 H34 F3 N5 O8 S2 | 761.18009 | 69.1 | -16.8 | -63.2 | 21.4 |
| 2-2-d1E01-2-0433 | C39 H37 F3 N4 O7 S | 762.23350 | -30.1 | -28.4 | 13.6 | 27.8 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0434 | C39 H37 F3 N4 O7 S | 762.23350 | 0.0 | 0.0 | -13.9 | 2.4 |
| 2-2-d1E01-2-0435 | C39 H43 F N4 O7 S2 | 762.25572 | 16.1 | -5.2 | -2.1 | -10.2 |
| 2-2-d1E01-2-0436 | C35 H37 N7 O7 S3 | 763.19166 | 0.4 | 16.3 | 4.1 | 12.2 |
| 2-2-d1E01-2-0437 | C38 H36 F3 N5 O7 S | 763.22875 | -8.2 | 74.2 | 8.2 | 4.0 |
| 2-2-d1E01-2-0438 | C38 H36 F3 N5 O7 S | 763.22875 | -2.4 | 44.9 | 15.5 | 30.4 |
| 2-2-d1E01-2-0439 | C38 H36 F3 N5 O7 S | 763.22875 | 0.0 | 0.0 | 0.0 | 0.0 |
| 2-2-d1E01-2-0440 | C39 H39 F3 N4 O7 S | 764.24915 | -2.7 | -51.4 | -11.9 | 11.6 |
| 2-2-d1E01-2-0441 | C39 H39 F3 N4 O7 S | 764.24915 | -13.0 | 0.0 | -1.5 | -8.1 |
| 2-2-d1E01-2-0442 | C38 H38 F3 N5 O7 S | 765.24440 | 6.8 | 27.5 | -4.7 | 6.2 |
| 2-2-d1E01-2-0443 | C41 H42 N4 O9 S | 766.26725 | 0.0 | 0.0 | 0.0 | 0.0 |
| 2-2-d1E01-2-0444 | C38 H40 F3 N5 O7 S | 767.26005 | 0.1 | 12.8 | -18.5 | 9.1 |
| 2-2-d1E01-2-0445 | C37 H39 F3 N6 O7 S | 768.25530 | 4.8 | 17.5 | 19.2 | 8.5 |
| 2-2-d1E01-2-0446 | C41 H48 N6 O7 S | 768.33052 | 0.2 | -20.9 | 3.1 | 4.2 |
| 2-2-d1E01-2-0447 | C36 H38 F3 N7 O7 S | 769.25055 | 22.0 | -20.9 | 0.0 | 22.0 |
| 2-2-d1E01-2-0448 | C40 H43 N5 O7 S2 | 769.26039 | 64.3 | 80.6 | 74.8 | 109.4 |
| 2-2-d1E01-2-0449 | C37 H37 F3 N4 O7 S2 | 770.20558 | 12.8 | -102.1 | 4.5 | -3.0 |
| 2-2-d1E01-2-0450 | C40 H39 F N4 O9 S | 770.24218 | -6.0 | -25.4 | 4.0 | 7.5 |
| 2-2-d1E01-2-0451 | C38 H41 F3 N4 O8 S | 770.25972 | -14.1 | -14.4 | 5.3 | 4.4 |
| 2-2-d1E01-2-0452 | C37 H37 N7 O8 S2 | 771.21450 | -1.9 | -20.3 | 12.8 | 5.4 |
| 2-2-d1E01-2-0453 | C36 H36 F3 N5 O9 S | 771.21858 | 0.0 | 0.0 | 0.0 | 0.0 |
| 2-2-d1E01-2-0454 | C40 H45 F N6 O7 S | 772.30545 | -4.1 | 2.7 | 1.1 | 8.6 |
| 2-2-d1E01-2-0455 | C37 H39 N7 O8 S2 | 773.23015 | -2.6 | 7.0 | 3.4 | 13.5 |
| 2-2-d1E01-2-0456 | C43 H47 N7 O5 S | 773.33594 | 0.0 | 0.0 | 0.0 | 0.0 |
| 2-2-d1E01-2-0457 | C35 H37 F3 N6 O7 S2 | 774.21172 | -0.2 | -5.5 | 9.1 | 16.0 |
| 2-2-d1E01-2-0458 | C38 H41 N5 O7 S3 | 775.21681 | -5.6 | -18.2 | 3.9 | 1.7 |
| 2-2-d1E01-2-0459 | C36 H36 N6 O8 S3 | 776.17567 | 9.2 | -25.0 | 17.2 | 6.5 |
| 2-2-d1E01-2-0460 | C41 H39 N5 O7 S2 | 777.22909 | -2.1 | 27.9 | 16.5 | 4.9 |
| 2-2-d1E01-2-0461 | C41 H39 N5 O7 S2 | 777.22909 | -32.3 | -16.3 | 27.7 | 57.1 |
| 2-2-d1E01-2-0462 | C40 H38 N6 O7 S2 | 778.22434 | 2.9 | 14.4 | 2.9 | 19.4 |
| 2-2-d1E01-2-0463 | C40 H38 N6 O7 S2 | 778.22434 | 9.1 | 23.5 | 56.4 | 42.1 |
| 2-2-d1E01-2-0464 | C40 H38 N6 O7 S2 | 778.22434 | -1044.2 | 0.0 | 0.0 | 0.0 |
| 2-2-d1E01-2-0465 | C40 H43 F3 N4 O7 S | 780.28046 | -11.2 | 6.1 | 14.8 | -17.3 |
| 2-2-d1E01-2-0466 | C42 H48 N6 O7 S | 780.33052 | 24.6 | -6.6 | -1.4 | 12.2 |
| 2-2-d1E01-2-0467 | C37 H37 F3 N6 O8 S | 782.23457 | 18.0 | 11.9 | 0.0 | 0.0 |
| 2-2-d1E01-2-0468 | C40 H42 N6 O7 S2 | 782.25564 | -1.9 | -10.7 | 0.7 | 25.8 |
| 2-2-d1E01-2-0469 | C39 H37 N5 O9 S2 | 783.20327 | 6.0 | 0.4 | 0.0 | 0.0 |

(continued)

| [Table 11-3-5] | | | | | | |
|---|---|---|---|---|---|---|
| | | | AS-MS ratio(%) | | | |
| ID | Molecular Formula | Exact Mass | N1 | N2 | N3 | N4 |
| 2-2-d1E01-2-0470 | C39 H41 N7 O7 S2 | 783.25089 | -2.9 | 0.0 | 2.8 | 8.0 |
| 2-2-d1E01-2-0471 | C38 H40 N8 O7 S2 | 784.24614 | -15.0 | -2.2 | 6.6 | 17.7 |
| 2-2-d1E01-2-0472 | C37 H39 F3 N6 O8 S | 784.25022 | -1.1 | -1.6 | -5.4 | 6.0 |
| 2-2-d1E01-2-0473 | C43 H47 F3 N6 O5 | 784.35600 | 0.0 | -2.9 | 0.0 | 0.0 |
| 2-2-d1E01-2-0474 | C40 H43 N5 O8 S2 | 785.25530 | 45.5 | -5.3 | -7.1 | 11.7 |
| 2-2-d1E01-2-0475 | C38 H41 F3 N4 O7 S2 | 786.23688 | 1.1 | 21.5 | 13.3 | 6.2 |
| 2-2-d1E01-2-0476 | C36 H36 F3 N5 O8 S2 | 787.19574 | 6.1 | 1.1 | 10.0 | 6.9 |
| 2-2-d1E01-2-0477 | C41 H39 F3 N4 O7 S | 788.24915 | 0.0 | 0.0 | 0.0 | 0.0 |
| 2-2-d1E01-2-0478 | C41 H39 F3 N4 O7 S | 788.24915 | 20.7 | 21.1 | -0.1 | 9.2 |
| 2-2-d1E01-2-0479 | C37 H39 N7 O7 S3 | 789.20731 | -6.7 | 35.3 | 10.5 | 16.7 |
| 2-2-d1E01-2-0480 | C40 H38 F3 N5 O7 S | 789.24440 | -6.3 | -0.4 | 0.0 | 0.0 |
| 2-2-d1E01-2-0481 | C40 H38 F3 N5 O7 S | 789.24440 | 8.0 | 17.9 | 15.6 | 6.7 |
| 2-2-d1E01-2-0482 | C40 H38 F3 N5 O7 S | 789.24440 | -26.5 | -2.8 | -12.4 | 3.4 |
| 2-2-d1E01-2-0483 | C40 H42 F3 N5 O7 S | 793.27570 | 0.0 | 5.5 | -7.1 | 2.4 |
| 2-2-d1E01-2-0484 | C39 H37 F3 N4 O9 S | 794.22333 | -24.7 | 39.5 | 4.3 | 4.7 |
| 2-2-d1E01-2-0485 | C39 H41 F3 N6 O7 S | 794.27095 | 13.9 | 22.3 | 5.7 | 9.1 |
| 2-2-d1E01-2-0486 | C43 H50 N6 O7 S | 794.34617 | -11.3 | 31.7 | 2.7 | 13.6 |
| 2-2-d1E01-2-0487 | C38 H40 F3 N7 O7 S | 795.26620 | 8.1 | -17.5 | 16.1 | 19.2 |
| 2-2-d1E01-2-0488 | C42 H45 N5 O7 S2 | 795.27604 | -11.9 | 0.5 | 7.0 | 23.3 |
| 2-2-d1E01-2-0489 | C40 H43 F3 N4 O8 S | 796.27537 | 8.6 | 9.5 | 8.5 | 35.2 |
| 2-2-d1E01-2-0490 | C42 H47 F N6 O7 S | 798.32110 | 18.7 | -15.2 | -4.2 | 9.4 |
| 2-2-d1E01-2-0491 | C37 H39 F3 N6 O7 S2 | 800.22737 | 28.1 | 7.3 | 22.0 | 13.6 |
| 2-2-d1E01-2-0492 | C40 H43 N5 O7 S3 | 801.23246 | 18.6 | 11.2 | 11.7 | 17.7 |
| 2-2-d1E01-2-0493 | C42 H45 F3 N4 O7 S | 806.29611 | 46.9 | -8.0 | -4.9 | 51.3 |
| 2-2-d1E01-2-0494 | C41 H39 N5 O9 S2 | 809.21892 | 0.0 | 0.0 | 0.0 | 0.0 |
| 2-2-d1E01-2-0495 | C41 H45 N7 O7 S2 | 811.28219 | 17.4 | 1.9 | 2.5 | 12.1 |
| 2-2-d1E01-2-0496 | C40 H43 F3 N4 O7 S2 | 812.25253 | -1.9 | 11.1 | -0.1 | 16.0 |
| 2-2-d1E01-2-0497 | C41 H39 F3 N4 O9 S | 820.23898 | 65.9 | -18.2 | 4.7 | -0.2 |
| 2-2-d1E01-2-0498 | C41 H45 F3 N6 O7 S | 822.30225 | -3.1 | -9.0 | 0.9 | 8.2 |
| 2-2-d1E01-2-0499 | C43 H47 N7 O7 S2 | 837.29784 | -24.9 | 0.1 | -10.0 | 15.9 |
| 2-2-d1E01-2-0500 | C43 H47 F3 N6 O7 S | 848.31790 | 3.3 | 34.0 | 7.0 | 0.6 |

[Table 603]

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0001 | C34 H36 N4 O7 | 612.25840 | 1.4 | 0.7 | 5.4 | 5.5 |
| 2-2-d1E02-2-0002 | C35 H44 N4 O6 | 616.32609 | -4.1 | 0.6 | 3.2 | 1.9 |
| 2-2-d1E02-2-0003 | C34 H42 N4 O7 | 618.30535 | -4.7 | -2.0 | 2.7 | 3.4 |
| 2-2-d1E02-2-0004 | C36 H38 N4 O6 | 622.27913 | 0.2 | -7.3 | 8.1 | 10.8 |
| 2-2-d1E02-2-0005 | C36 H38 N4 O6 | 622.27913 | -4.7 | 1.3 | 0.3 | 6.1 |
| 2-2-d1E02-2-0006 | C35 H37 N5 O6 | 623.27438 | -5.1 | 2.6 | 2.6 | 4.1 |
| 2-2-d1E02-2-0007 | C35 H38 N4 O7 | 626.27405 | -7.4 | -22.9 | 3.7 | 8.1 |
| 2-2-d1E02-2-0008 | C34 H36 N4 O6 S | 628.23556 | -8.5 | -9.6 | 5.8 | 9.3 |
| 2-2-d1E02-2-0009 | C33 H35 N5 O8 | 629.24856 | -4.7 | 0.0 | 0.0 | 0.0 |
| 2-2-d1E02-2-0010 | C34 H35 F N4 O7 | 630.24898 | -1.9 | 5.1 | 5.3 | 5.3 |
| 2-2-d1E02-2-0011 | C36 H46 N4 O6 | 630.34174 | -6.1 | -15.6 | 2.5 | 4.7 |
| 2-2-dlE02-2-0012 | C35 H44 N4 O7 | 632.32100 | -1.3 | -7.3 | 1.3 | 3.3 |
| 2-2-d1E02-2-0013 | C35 H43 F N4 O6 | 634.31666 | -6.3 | -8.3 | 5.1 | 4.6 |
| 2-2-dlE02-2-0014 | C37 H40 N4 O6 | 636.29479 | -23.3 | 2.0 | 0.3 | 1.8 |
| 2-2-d1E02-2-0015 | C37 H40 N4 O6 | 636.29479 | -25.6 | -7.1 | 9.7 | 8.4 |
| 2-2-d1E02-2-0016 | C34 H41 F N4 O7 | 636.29593 | -9.4 | -4.5 | 1.4 | 3.8 |
| 2-2-d1E02-2-0017 | C36 H39 N5 O6 | 637.29003 | -6.6 | -13.3 | 4.4 | 6.6 |
| 2-2-dlE02-2-0018 | C36 H38 N4 O7 | 638.27405 | -3.7 | 2.5 | 6.4 | 11.1 |
| 2-2-dlE02-2-0019 | C34 H36 N6 O7 | 640.26455 | -4.1 | 0.0 | 2.2 | 0.0 |
| 2-2-d1E02-2-0020 | C36 H37 F N4 O6 | 640.26971 | -3.8 | -14.3 | 3.7 | 7.5 |
| 2-2-dlE02-2-0021 | C36 H37 F N4 O6 | 640.26971 | -22.2 | -8.1 | 1.1 | 4.1 |
| 2-2-d1E02-2-0022 | C35 H36 F N5 O6 | 641.26496 | -1.1 | -5.9 | 3.0 | 5.7 |
| 2-2-d1E02-2-0023 | C35 H38 N4 O6 S | 642.25121 | -16.6 | -2.5 | 8.5 | 10.4 |
| 2-2-d1E02-2-0024 | C34 H38 N6 O7 | 642.28020 | -5.9 | 0.4 | 4.7 | 5.5 |
| 2-2-d1E02-2-0025 | C37 H46 N4 O6 | 642.34174 | -1.9 | -2.5 | -2.1 | -0.7 |
| 2-2-d1E02-2-0026 | C34 H37 N5 O8 | 643.26421 | -10.4 | -26.8 | -8.5 | -11.9 |
| 2-2-d1E02-2-0027 | C36 H44 N4 O7 | 644.32100 | -10.9 | -17.0 | 0.5 | 3.5 |
| 2-2-d1E02-2-0028 | C33 H35 N5 O7 S | 645.22572 | -12.3 | -1.9 | 4.3 | 6.6 |
| 2-2-d1E02-2-0029 | C34 H35 F N4 O6 S | 646.22613 | -9.6 | -15.6 | 5.8 | 6.9 |
| 2-2-d1E02-2-0030 | C38 H38 N4 O6 | 646.27913 | -2.9 | 41.3 | 8.5 | 10.0 |
| 2-2-d1E02-2-0031 | C38 H38 N4 O6 | 646.27913 | -13.8 | 4.4 | 5.5 | 13.5 |
| 2-2-d1E02-2-0032 | C33 H34 F N5 O8 | 647.23914 | -7.1 | 0.0 | 3.3 | 2.8 |
| 2-2-d1E02-2-0033 | C37 H37 N5 O6 | 647.27438 | 9.1 | -5.1 | 5.6 | 8.3 |
| 2-2-d1E02-2-0034 | C37 H37 N5 O6 | 647.27438 | -0.1 | 4.4 | 3.2 | 6.7 |
| 2-2-d1E02-2-0035 | C37 H37 N5 O6 | 647.27438 | -13.2 | 15.3 | 5.9 | 8.3 |
| 2-2-d1E02-2-0036 | C38 H40 N4 O6 | 648.29479 | 0.0 | 3.6 | 4.1 | 2.0 |
| 2-2-d1E02-2-0037 | C38 H40 N4 O6 | 648.29479 | 0.0 | 0.0 | 0.0 | 0.0 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0038 | C37 H39 N5 O6 | 649.29003 | -17.3 | -4.2 | 5.2 | 8.2 |
| 2-2-d1E02-2-0039 | C37 H41 N5 O6 | 651.30568 | 8.5 | 13.9 | 0.1 | 0.0 |
| 2-2-d1E02-2-0040 | C37 H40 N4 O7 | 652.28970 | -2.2 | 3.0 | 0.0 | 0.0 |
| 2-2-d1E02-2-0041 | C36 H40 N6 O6 | 652.30093 | -9.4 | -4.1 | 4.3 | 12.0 |
| 2-2-d1E02-2-0042 | C35 H39 N7 O6 | 653.29618 | -22.1 | -13.6 | 4.2 | 5.4 |
| 2-2-d1E02-2-0043 | C36 H38 N4 O6 S | 654.25121 | 0.0 | 6.5 | 0.0 | 0.0 |
| 2-2-d1E02-2-0044 | C35 H38 N6 O7 | 654.28020 | 0.0 | 0.0 | 13.3 | 0.0 |
| 2-2-d1E02-2-0045 | C37 H42 N4 O7 | 654.30535 | -8.5 | 0.0 | 6.0 | 7.2 |
| 2-2-d1E02-2-0046 | C35 H37 N5 O8 | 655.26421 | 0.0 | 0.0 | 0.0 | 0.0 |
| 2-2-d1E02-2-0047 | C36 H37 F N4 O7 | 656.26463 | -21.9 | 0.6 | 6.9 | 10.7 |
| 2-2-dlE02-2-0048 | C35 H40 N6 O7 | 656.29585 | -5.4 | -0.7 | 7.9 | 6.1 |
| 2-2-d1E02-2-0049 | C38 H48 N4 O6 | 656.35739 | 2.0 | -3.5 | 1.9 | 0.0 |
| 2-2-d1E02-2-0050 | C34 H35 F N6 O7 | 658.25513 | 7.1 | -2.1 | 4.5 | 5.2 |
| 2-2-d1E02-2-0051 | C34 H38 N6 O6 S | 658.25735 | -6.0 | -14.9 | 0.0 | 0.0 |
| 2-2-d1E02-2-0052 | C37 H46 N4 O7 | 658.33665 | 4.4 | 15.8 | 3.6 | 7.8 |
| 2-2-d1E02-2-0053 | C34 H37 N5 O7 S | 659.24137 | -15.9 | -8.6 | 5.7 | 9.2 |
| 2-2-d1E02-2-0054 | C34 H37 F N6 O7 | 660.27078 | -8.6 | -14.6 | 6.5 | 4.4 |
| 2-2-d1E02-2-0055 | C39 H40 N4 O6 | 660.29479 | 9.1 | 0.8 | 7.2 | 10.9 |
| 2-2-d1E02-2-0056 | C39 H40 N4 O6 | 660.29479 | -4.4 | 1.4 | 7.3 | 9.0 |
| 2-2-d1E02-2-0057 | C37 H45 F N4 O6 | 660.33231 | 21.1 | -22.9 | 4.7 | 4.8 |
| 2-2-d1E02-2-0058 | C38 H39 N5 O6 | 661.29003 | -9.0 | 7.4 | 4.6 | 6.5 |
| 2-2-d1E02-2-0059 | C38 H39 N5 O6 | 661.29003 | -9.5 | 12.1 | 9.7 | 10.3 |
| 2-2-d1E02-2-0060 | C38 H39 N5 O6 | 661.29003 | -25.0 | -20.1 | 1.9 | 6.9 |
| 2-2-dlE02-2-0061 | C39 H42 N4 O6 | 662.31044 | 10.4 | 7.0 | 3.7 | 8.1 |
| 2-2-d1E02-2-0062 | C39 H42 N4 O6 | 662.31044 | 0.5 | -19.9 | -16.7 | 35.4 |
| 2-2-d1E02-2-0063 | C36 H43 F N4 O7 | 662.31158 | -6.2 | -6.2 | 0.0 | 2.3 |
| 2-2-d1E02-2-0064 | C33 H34 F N5 O7 S | 663.21630 | -6.9 | -18.7 | 1.8 | 6.5 |
| 2-2-d1E02-2-0065 | C38 H41 N5 O6 | 663.30568 | -8.6 | -3.4 | 4.2 | 3.9 |
| 2-2-d1E02-2-0066 | C38 H37 F N4 O6 | 664.26971 | -4.9 | -6.1 | 2.1 | 10.4 |
| 2-2-d1E02-2-0067 | C38 H37 F N4 O6 | 664.26971 | -20.9 | 3.9 | -0.4 | 0.0 |
| 2-2-d1E02-2-0068 | C39 H44 N4 O6 | 664.32609 | 0.3 | 6.8 | 5.3 | 5.4 |
| 2-2-d1E02-2-0069 | C37 H36 F N5 O6 | 665.26496 | -17.0 | -13.5 | 4.0 | 4.6 |
| 2-2-d1E02-2-0070 | C37 H36 F N5 O6 | 665.26496 | 15.1 | -4.9 | 13.3 | 0.8 |
| 2-2-d1E02-2-0071 | C37 H36 F N5 O6 | 665.26496 | -16.1 | 7.8 | 3.4 | 2.5 |
| 2-2-d1E02-2-0072 | C38 H43 N5 O6 | 665.32133 | -1.2 | 2.1 | 1.2 | 2.1 |
| 2-2-d1E02-2-0073 | C36 H38 N6 O7 | 666.28020 | 10.1 | 4.2 | 4.7 | 17.8 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0074 | C38 H39 F N4 O6 | 666.28536 | -7.3 | -3.9 | 3.1 | 8.2 |
| 2-2-d1E02-2-0075 | C38 H39 F N4 O6 | 666.28536 | 6.7 | -0.3 | 7.3 | 15.9 |
| 2-2-d1E02-2-0076 | C37 H42 N6 O6 | 666.31658 | 1.9 | -0.6 | 8.1 | 9.3 |
| 2-2-d1E02-2-0077 | C37 H38 F N5 O6 | 667.28061 | -9.5 | -5.4 | 5.1 | 7.5 |
| 2-2-d1E02-2-0078 | C36 H41 N7 O6 | 667.31183 | -10.8 | -0.8 | 4.2 | 6.9 |
| 2-2-d1E02-2-0079 | C37 H40 N4 O6 S | 668.26686 | 0.0 | 0.0 | 0.0 | 0.0 |
| 2-2-d1E02-2-0080 | C36 H40 N6 O7 | 668.29585 | -4.1 | -9.9 | 2.9 | 5.8 |
| 2-2-d1E02-2-0081 | C38 H44 N4 O7 | 668.32100 | -6.2 | 5.9 | 3.9 | 9.3 |
| 2-2-d1E02-2-0082 | C35 H35 N5 O7 S | 669.22572 | -6.8 | 3.5 | 5.1 | 7.4 |
| 2-2-d1E02-2-0083 | C36 H39 N5 O8 | 669.27986 | -1.9 | -61.7 | 6.3 | 1.7 |
| 2-2-d1E02-2-0084 | C37 H40 F N5 O6 | 669.29626 | 6.7 | 7.9 | 11.8 | 6.4 |
| 2-2-d1E02-2-0085 | C37 H42 N4 O6 S | 670.28251 | -8.1 | 9.5 | 4.4 | 14.9 |
| 2-2-d1E02-2-0086 | C36 H39 F N6 O6 | 670.29151 | -11.0 | 5.8 | 7.2 | 17.1 |
| 2-2-d1E02-2-0087 | C35 H37 N5 O7 S | 671.24137 | 0.0 | 0.0 | 0.0 | 0.0 |
| 2-2-d1E02-2-0088 | C35 H38 F N7 O6 | 671.28676 | 2.1 | -2.4 | 6.1 | 4.5 |
| 2-2-d1E02-2-0089 | C36 H37 F N4 O6 S | 672.24178 | -11.7 | 2.5 | 15.4 | 19.7 |
| 2-2-d1E02-2-0090 | C35 H40 N6 O6 S | 672.27300 | 0.4 | 11.2 | 4.2 | 10.1 |
| 2-2-d1E02-2-0091 | C40 H40 N4 O6 | 672.29479 | -7.4 | 1.3 | 3.3 | 7.9 |
| 2-2-d1E02-2-0092 | C40 H40 N4 O6 | 672.29479 | -8.2 | -3.0 | 0.0 | 0.0 |
| 2-2-d1E02-2-0093 | C37 H41 F N4 O7 | 672.29593 | -14.1 | 0.5 | 0.0 | 9.5 |
| 2-2-d1E02-2-0094 | C35 H36 F N5 O8 | 673.25479 | -4.5 | -6.6 | -64.3 | 3.7 |
| 2-2-d1E02-2-0095 | C39 H39 N5 O6 | 673.29003 | 3.1 | 4.5 | 14.6 | 7.0 |
| 2-2-d1E02-2-0096 | C39 H39 N5 O6 | 673.29003 | -11.2 | -4.6 | -6.4 | 5.4 |
| 2-2-dlE02-2-0097 | C39 H39 N5 O6 | 673.29003 | 10.7 | 9.5 | 15.3 | 12.1 |
| 2-2-d1E02-2-0098 | C36 H43 N5 O6 S | 673.29340 | -10.5 | -0.8 | 0.4 | 4.7 |
| 2-2-d1E02-2-0099 | C35 H41 N5 O7 S | 675.27267 | -20.1 | -8.6 | 3.5 | 3.8 |
| 2-2-d1E02-2-0100 | C34 H36 N4 O9 S | 676.22030 | -7.6 | -7.5 | 2.0 | 2.6 |
| 2-2-d1E02-2-0101 | C34 H37 F N6 O6 S | 676.24793 | -3.5 | -2.8 | 5.4 | 4.8 |
| 2-2-d1E02-2-0102 | C39 H43 N5 O6 | 677.32133 | 0.0 | 0.0 | 0.0 | 0.0 |
| 2-2-d1E02-2-0103 | C38 H38 N4 O8 | 678.26896 | -28.7 | -1.8 | 8.8 | 9.3 |
| 2-2-d1E02-2-0104 | C38 H42 N6 O6 | 678.31658 | 0.3 | -2.7 | 4.4 | 2.5 |
| 2-2-d1E02-2-0105 | C40 H46 N4 O6 | 678.34174 | -8.6 | 9.4 | 11.7 | 6.1 |
| 2-2-d1E02-2-0106 | C37 H37 N5 O6 S | 679.24645 | -5.8 | 5.2 | 5.7 | 10.6 |
| 2-2-dlE02-2-0107 | C37 H37 N5 O6 S | 679.24645 | 4.5 | -1.8 | 4.4 | 11.9 |
| 2-2-d1E02-2-0108 | C37 H41 N7 O6 | 679.31183 | 0.2 | -0.3 | 3.6 | 2.9 |
| 2-2-dlE02-2-0109 | C36 H36 N6 O6 S | 680.24170 | -6.9 | 8.8 | 6.0 | 4.1 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0110 | C35 H35 F3 N4 O7 | 680.24578 | -5.9 | -6.0 | 3.3 | 18.3 |
| 2-2-d1E02-2-0111 | C35 H44 N4 O8 S | 680.28799 | -9.8 | -1.2 | 0.6 | 0.7 |
| 2-2-d1E02-2-0112 | C37 H40 N6 O7 | 680.29585 | 0.0 | 0.0 | 0.0 | 0.0 |
| 2-2-d1E02-2-0113 | C39 H44 N4 O7 | 680.32100 | 10.9 | 11.9 | 27.2 | -2.1 |
| 2-2-d1E02-2-0114 | C34 H42 N4 O9 S | 682.26725 | -3.4 | -0.6 | 1.3 | 1.4 |
| 2-2-d1E02-2-0115 | C37 H42 N6 O7 | 682.31150 | 10.5 | 5.7 | 3.4 | 7.2 |
| 2-2-d1E02-2-0116 | C39 H43 F N4 O6 | 682.31666 | 7.7 | -0.6 | 8.0 | 9.1 |
| 2-2-dlE02-2-0117 | C36 H37 F N6 O7 | 684.27078 | 0.0 | 3.2 | 0.0 | 0.0 |
| 2-2-d1E02-2-0118 | C36 H40 N6 O6 S | 684.27300 | 0.0 | 0.0 | 1.9 | 0.0 |
| 2-2-d1E02-2-0119 | C38 H44 N4 O6 S | 684.29816 | 13.6 | 20.0 | 3.5 | -1.5 |
| 2-2-d1E02-2-0120 | C36 H43 F3 N4 O6 | 684.31347 | 5.9 | 6.8 | -1.8 | 10.0 |
| 2-2-d1E02-2-0121 | C35 H35 N5 O6 S2 | 685.20288 | -1.9 | 5.5 | 6.4 | 7.9 |
| 2-2-dlE02-2-0122 | C36 H39 N5 O7 S | 685.25702 | 0.0 | -73.9 | -10.4 | 19.6 |
| 2-2-dlE02-2-0123 | C34 H34 N6 O8 S | 686.21588 | 0.0 | 0.0 | 0.0 | 0.0 |
| 2-2-dlE02-2-0124 | C36 H38 N4 O8 S | 686.24103 | -10.0 | -5.8 | 2.6 | 3.3 |
| 2-2-dlE02-2-0125 | C36 H38 N4 O8 S | 686.24103 | -7.7 | 0.4 | 1.1 | 1.1 |
| 2-2-dlE02-2-0126 | C36 H39 F N6 O7 | 686.28643 | -1.9 | -4.8 | 4.0 | 4.5 |
| 2-2-d1E02-2-0127 | C35 H41 F3 N4 O7 | 686.29273 | -8.0 | -4.1 | 0.9 | 7.7 |
| 2-2-d1E02-2-0128 | C41 H42 N4 O6 | 686.31044 | -9.4 | 44.5 | 5.3 | 10.6 |
| 2-2-d1E02-2-0129 | C41 H42 N4 O6 | 686.31044 | -10.2 | 4.3 | 4.7 | -2.3 |
| 2-2-d1E02-2-0130 | C35 H37 N5 O8 S | 687.23628 | 0.4 | -3.1 | 2.0 | 2.5 |
| 2-2-d1E02-2-0131 | C40 H41 N5 O6 | 687.30568 | -8.2 | -22.2 | 13.6 | 20.5 |
| 2-2-dlE02-2-0132 | C40 H41 N5 O6 | 687.30568 | -22.1 | -2.4 | 4.5 | 7.0 |
| 2-2-d1E02-2-0133 | C40 H41 N5 O6 | 687.30568 | 2.0 | -2.4 | 5.3 | 12.2 |
| 2-2-d1E02-2-0134 | C37 H41 F N4 O6 S | 688.27308 | -3.6 | 1.9 | 5.6 | 3.4 |
| 2-2-d1E02-2-0135 | C35 H36 F N5 O7 S | 689.23195 | 23.2 | 13.8 | -19.6 | 10.8 |
| 2-2-d1E02-2-0136 | C35 H38 N4 O9 S | 690.23595 | -5.9 | -3.5 | 2.8 | 3.4 |
| 2-2-d1E02-2-0137 | C37 H37 F3 N4 O6 | 690.26652 | -0.9 | 4.9 | 12.1 | 7.2 |
| 2-2-d1E02-2-0138 | C37 H37 F3 N4 O6 | 690.26652 | -2.9 | -5.7 | -0.6 | 3.3 |
| 2-2-d1E02-2-0 139 | C40 H39 F N4 O6 | 690.28536 | -18.6 | 34.8 | 8.2 | 10.0 |
| 2-2-dlE02-2-0140 | C40 H39 F N4 O6 | 690.28536 | 1.8 | 0.0 | 0.0 | 11.3 |
| 2-2-d1E02-2-0141 | C41 H46 N4 O6 | 690.34174 | -17.3 | 39.7 | -0.4 | 25.2 |
| 2-2-d1E02-2-0142 | C36 H36 F3 N5 O6 | 691.26177 | -2.2 | -5.9 | 5.5 | 7.0 |
| 2-2-d1E02-2-0 143 | C39 H38 F N5 O6 | 691.28061 | -21.3 | -5.3 | 8.4 | 8.8 |
| 2-2-d1E02-2-0144 | C39 H38 F N5 O6 | 691.28061 | -3.0 | 2.3 | 11.2 | 13.3 |
| 2-2-d1E02-2-0145 | C39 H38 F N5 O6 | 691.28061 | -19.8 | 1.9 | 6.1 | 8.0 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-dlE02-2-0146 | C40 H45 N5 O6 | 691.33698 | 0.0 | 0.0 | 0.0 | 0.0 |
| 2-2-dlE02-2-0147 | C34 H36 N4 O8 S2 | 692.19746 | -6.9 | 2.9 | 4.5 | 3.4 |
| 2-2-d1E02-2-0148 | C39 H40 N4 O8 | 692.28461 | -18.1 | -6.5 | 1.2 | 13.1 |
| 2-2-d1E02-2-0149 | C39 H44 N6 O6 | 692.33223 | 4.6 | 0.0 | 0.0 | 0.0 |
| 2-2-d1E02-2-0150 | C33 H35 N5 O10 S | 693.21046 | 0.0 | 0.0 | 0.0 | 0.0 |
| 2-2-dlE02-2-0151 | C38 H43 N7 O6 | 693.32748 | 4.1 | 7.6 | 1.2 | 10.9 |
| 2-2-dlE02-2-0152 | C34 H35 F N4 O9 S | 694.21088 | -8.2 | -2.8 | 1.9 | 1.6 |
| 2-2-dlE02-2-0153 | C36 H46 N4 O8 S | 694.30364 | -4.4 | -3.0 | -0.9 | 0.0 |
| 2-2-d1E02-2-0154 | C40 H46 N4 O7 | 694.33665 | -8.3 | -5.1 | 0.0 | 3.5 |
| 2-2-dlE02-2-0155 | C37 H37 N5 O7 S | 695.24137 | 13.1 | 14.5 | 5.3 | 8.7 |
| 2-2-dlE02-2-0156 | C39 H42 F N5 O6 | 695.31191 | -63.6 | 0.0 | 4.1 | 11.6 |
| 2-2-dlE02-2-0157 | C35 H35 F3 N4 O6 S | 696.22294 | -8.8 | 14.0 | 5.9 | 24.6 |
| 2-2-dlE02-2-0158 | C38 H37 F N4 O8 | 696.25954 | -3.1 | 2.6 | 8.5 | 16.8 |
| 2-2-d1E02-2-0159 | C35 H44 N4 O9 S | 696.28290 | -8.8 | -1.5 | 0.8 | 3.5 |
| 2-2-d1E02-2-0160 | C39 H44 N4 O6 S | 696.29816 | 21.3 | -5.7 | 16.9 | 4.5 |
| 2-2-dlE02-2-0161 | C38 H41 F N6 O6 | 696.30716 | -9.4 | -3.3 | -1.9 | -0.8 |
| 2-2-d1E02-2-0162 | C35 H35 N7 O7 S | 697.23187 | 0.0 | 0.0 | 0.0 | 0.0 |
| 2-2-d1E02-2-0163 | C34 H34 F3 N5 O8 | 697.23595 | -13.3 | 4.8 | 0.0 | 0.0 |
| 2-2-dlE02-2-0164 | C37 H40 F N7 O6 | 697.30241 | -4.7 | -0.6 | 4.5 | 7.1 |
| 2-2-d1E02-2-0165 | C35 H43 F N4 O8 S | 698.27856 | -13.4 | -3.9 | 2.2 | 1.7 |
| 2-2-d1E02-2-0166 | C37 H42 N6 O6 S | 698.28865 | 0.0 | 0.0 | 0.0 | 0.0 |
| 2-2-d1E02-2-0167 | C39 H43 F N4 O7 | 698.31158 | -15.5 | 12.5 | 21.4 | 15.7 |
| 2-2-d1E02-2-0168 | C35 H37 N7 O7 S | 699.24752 | -8.2 | -5.7 | 1.7 | 6.3 |
| 2-2-d1E02-2-0169 | C38 H45 N5 O6 S | 699.30905 | -8.3 | 12.4 | 5.5 | 16.1 |
| 2-2-dlE02-2-0170 | C37 H40 N4 O8 S | 700.25668 | -8.6 | -0.4 | 2.3 | 3.9 |
| 2-2-d1E02-2-0171 | C37 H40 N4 O8 S | 700.25668 | 1.5 | 4.9 | 1.0 | 8.0 |
| 2-2-d1E02-2-0172 | C34 H41 F N4 O9 S | 700.25783 | -1.1 | -0.9 | 1.2 | 1.4 |
| 2-2-d1E02-2-0173 | C36 H39 N5 O8 S | 701.25193 | -12.4 | 1.5 | 1.0 | 1.6 |
| 2-2-d1E02-2-0174 | C37 H43 N5 O7 S | 701.28832 | 2.3 | -9.4 | 0.0 | 10.5 |
| 2-2-d1E02-2-0175 | C34 H34 N6 O7 S2 | 702.19304 | 10.5 | 13.3 | 5.8 | 8.6 |
| 2-2-d1E02-2-0176 | C36 H38 N4 O9 S | 702.23595 | -9.3 | -4.3 | -0.1 | -1.9 |
| 2-2-d1E02-2-0177 | C36 H39 F N6 O6 S | 702.26358 | -3.5 | 0.0 | 0.0 | 3.1 |
| 2-2-d1E02-2-0178 | C39 H37 N5 O6 S | 703.24645 | 18.0 | -1.4 | 8.6 | 7.8 |
| 2-2-d1E02-2-0179 | C39 H37 N5 O6 S | 703.24645 | -9.3 | 11.1 | 5.6 | 4.2 |
| 2-2-d1E02-2-0180 | C34 H36 N6 O9 S | 704.22645 | -4.2 | 1.0 | 0.0 | 3.9 |
| 2-2-d1E02-2-0181 | C36 H37 F N4 O8 S | 704.23161 | -10.7 | 4.8 | 5.0 | 1.5 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0182 | C36 H37 F N4 O8 S | 704.23161 | -5.4 | 0.1 | 2.7 | 0.4 |
| 2-2-d1E02-2-0183 | C38 H36 N6 O6 S | 704.24170 | -23.3 | 8.2 | 2.9 | 7.1 |
| 2-2-dlE02-2-0184 | C38 H36 N6 O6 S | 704.24170 | 14.2 | 14.3 | 8.1 | 8.4 |
| 2-2-d1E02-2-0185 | C38 H36 N6 O6 S | 704.24170 | -7.8 | 1.1 | 5.8 | 9.9 |
| 2-2-d1E02-2-0186 | C40 H40 N4 O8 | 704.28461 | 66.6 | 44.6 | 90.5 | 30.2 |
| 2-2-d1E02-2-0187 | C42 H48 N4 O6 | 704.35739 | -18.1 | -4.5 | -3.2 | 5.0 |
| 2-2-d1E02-2-0188 | C35 H36 F N5 O8 S | 705.22686 | -3.6 | -2.4 | 1.1 | 1.7 |
| 2-2-d1E02-2-0189 | C39 H39 N5 O6 S | 705.26210 | -1.4 | 7.5 | 0.0 | 15.3 |
| 2-2-d1E02-2-0190 | C39 H39 N5 O6 S | 705.26210 | 0.0 | 0.0 | -79.9 | 0.0 |
| 2-2-dlE02-2-0191 | C35 H38 N4 O8 S2 | 706.21311 | -6.2 | -4.9 | 0.8 | 2.4 |
| 2-2-dlE02-2-0192 | C34 H38 N6 O9 S | 706.24210 | -2.8 | -1.6 | 2.0 | 1.9 |
| 2-2-dlE02-2-0193 | C38 H38 N6 O6 S | 706.25735 | -6.0 | 3.3 | 3.3 | 7.1 |
| 2-2-dlE02-2-0194 | C37 H37 F3 N4 O7 | 706.26143 | -0.5 | 8.8 | 3.2 | 15.6 |
| 2-2-dlE02-2-0195 | C37 H46 N4 O8 S | 706.30364 | -3.4 | -2.5 | 2.0 | -1.0 |
| 2-2-dlE02-2-0196 | C40 H46 N6 O6 | 706.34788 | 1.6 | 2.5 | 8.5 | 8.2 |
| 2-2-dlE02-2-0197 | C34 H37 N5 O10 S | 707.22611 | 0.0 | 0.0 | 0.0 | 0.0 |
| 2-2-dlE02-2-0198 | C35 H35 F3 N6 O7 | 708.25193 | -7.0 | -1.2 | 0.0 | -12.2 |
| 2-2-dlE02-2-0199 | C38 H40 N6 O6 S | 708.27300 | 13.4 | 23.2 | 1.8 | 11.1 |
| 2-2-d1E02-2-0200 | C36 H44 N4 O9 S | 708.28290 | -7.0 | -3.7 | 0.0 | 0.0 |
| 2-2-dlE02-2-0201 | C41 H45 F N4 O6 | 708.33231 | -5.6 | 2.7 | -1.4 | 4.9 |
| 2-2-d1E02-2-0202 | C33 H35 N5 O9 S2 | 709.18762 | 0.0 | 0.0 | 5.6 | 0.0 |
| 2-2-d1E02-2-0203 | C37 H39 N7 O6 S | 709.26825 | 10.2 | -6.8 | 17.7 | 14.4 |
| 2-2-d1E02-2-0204 | C34 H35 F N4 O8 S2 | 710.18803 | -9.6 | -5.7 | 2.3 | 1.2 |
| 2-2-d1E02-2-0205 | C38 H38 N4 O8 S | 710.24103 | -11.4 | -8.1 | 3.2 | 3.0 |
| 2-2-d1E02-2-0206 | C38 H38 N4 O8 S | 710.24103 | 0.0 | 2.3 | 0.8 | 0.0 |
| 2-2-d1E02-2-0207 | C36 H38 N8 O6 S | 710.26350 | -17.8 | -9.6 | 5.8 | 5.8 |
| 2-2-dlE02-2-0208 | C35 H37 F3 N6 O7 | 710.26758 | -7.4 | 5.3 | 3.4 | 3.4 |
| 2-2-d1E02-2-0209 | C40 H46 N4 O6 S | 710.31381 | -23.7 | -23.2 | 9.2 | 14.2 |
| 2-2-d1E02-2-0210 | C38 H45 F3 N4 O6 | 710.32912 | -8.0 | 3.4 | -1.3 | 12.4 |
| 2-2-d1E02-2-0211 | C33 H34 F N5 O10 S | 711.20104 | 0.0 | 0.0 | 0.0 | 3.4 |
| 2-2-d1E02-2-0212 | C37 H37 N5 O6 S2 | 711.21853 | 4.7 | -5.5 | 13.1 | 9.7 |
| 2-2-d1E02-2-0213 | C37 H37 N5 O8 S | 711.23628 | -3.2 | 2.2 | 0.2 | 2.5 |
| 2-2-d1E02-2-0214 | C37 H37 N5 O8 S | 711.23628 | -6.0 | 1.2 | 7.6 | 10.7 |
| 2-2-d1E02-2-0215 | C37 H37 N5 O8 S | 711.23628 | 0.0 | 0.0 | 0.0 | 0.0 |
| 2-2-d1E02-2-0216 | C38 H41 N5 O7 S | 711.27267 | -3.9 | 33.7 | 4.7 | 9.4 |
| 2-2-d1E02-2-0217 | C36 H36 N6 O8 S | 712.23153 | -7.5 | 9.9 | 0.0 | -17.2 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0218 | C38 H40 N4 O8 S | 712.25668 | 0.2 | -3.6 | 0.0 | 0.0 |
| 2-2-d1E02-2-0219 | C38 H40 N4 O8 S | 712.25668 | 0.0 | 0.0 | 0.0 | 0.0 |
| 2-2-d1E02-2-0220 | C37 H43 F3 N4 O7 | 712.30838 | -3.3 | 15.5 | 4.2 | 18.5 |
| 2-2-d1E02-2-0221 | C34 H34 F3 N5 O7 S | 713.21310 | 29.1 | -33.3 | 11.9 | 6.0 |
| 2-2-d1E02-2-0222 | C37 H39 N5 O8 S | 713.25193 | 0.6 | -0.4 | 1.5 | 1.6 |
| 2-2-d1E02-2-0223 | C39 H37 F3 N4 O6 | 714.26652 | -17.3 | 6.7 | 5.6 | 10.2 |
| 2-2-d1E02-2-0224 | C39 H37 F3 N4 O6 | 714.26652 | 0.0 | 0.0 | 0.0 | 0.0 |
| 2-2-d1E02-2-0225 | C39 H43 F N4 O6 S | 714.28873 | -22.2 | 2.4 | 3.2 | 16.3 |
| 2-2-d1E02-2-0226 | C35 H37 N7 O6 S2 | 715.22467 | -2.0 | -1.3 | 4.5 | 6.9 |
| 2-2-d1E02-2-0227 | C38 H36 F3 N5 O6 | 715.26177 | -15.0 | 4.7 | -1.0 | 4.3 |
| 2-2-d1E02-2-0228 | C38 H36 F3 N5 O6 | 715.26177 | -20.8 | 8.6 | -0.2 | 6.8 |
| 2-2-d1E02-2-0229 | C38 H36 F3 N5 O6 | 715.26177 | 0.0 | 0.0 | 4.0 | 8.0 |
| 2-2-d1E02-2-0230 | C37 H41 N5 O8 S | 715.26758 | -8.4 | -3.1 | 0.0 | 10.3 |
| 2-2-d1E02-2-0231 | C37 H40 N4 O9 S | 716.25160 | 13.6 | -9.6 | 1.8 | 4.8 |
| 2-2-d1E02-2-0232 | C36 H40 N6 O8 S | 716.26283 | -9.0 | -1.7 | 2.8 | 1.6 |
| 2-2-d1E02-2-0233 | C39 H39 F3 N4 O6 | 716.28217 | -5.9 | 7.2 | 4.7 | 7.9 |
| 2-2-d1E02-2-0234 | C39 H39 F3 N4 O6 | 716.28217 | -6.9 | 7.8 | 1.7 | 7.1 |
| 2-2-d1E02-2-0235 | C35 H39 N7 O8 S | 717.25808 | -0.6 | -13.7 | 7.2 | 6.5 |
| 2-2-d1E02-2-0236 | C38 H38 F3 N5 O6 | 717.27742 | 2.6 | 7.3 | 0.8 | 9.9 |
| 2-2-d1E02-2-0237 | C36 H38 N4 O8 S2 | 718.21311 | 0.0 | 0.0 | 0.0 | 0.0 |
| 2-2-dlE02-2-0238 | C35 H38 N6 O9 S | 718.24210 | -4.5 | 0.0 | 0.0 | 0.0 |
| 2-2-dlE02-2-0239 | C37 H42 N4 O9 S | 718.26725 | -5.3 | -0.9 | 0.0 | 0.0 |
| 2-2-d1E02-2-0240 | C41 H42 N4 O8 | 718.30026 | -7.5 | 0.0 | 4.5 | 0.0 |
| 2-2-dlE02-2-0241 | C35 H37 N5 O10 S | 719.22611 | 0.0 | 0.0 | 0.0 | 0.0 |
| 2-2-dlE02-2-0242 | C38 H40 F3 N5 O6 | 719.29307 | -12.9 | 1.7 | 1.0 | 3.5 |
| 2-2-dlE02-2-0243 | C36 H37 F N4 O9 S | 720.22653 | -11.0 | -1.5 | 1.1 | 1.3 |
| 2-2-d1E02-2-0244 | C35 H40 N6 O9 S | 720.25775 | -7.9 | -0.3 | 1.7 | 5.4 |
| 2-2-d1E02-2-0245 | C37 H39 F3 N6 O6 | 720.28832 | -21.9 | 12.0 | 4.1 | 4.1 |
| 2-2-d1E02-2-0246 | C38 H48 N4 O8 S | 720.31929 | -8.4 | -6.2 | 3.7 | 8.5 |
| 2-2-dlE02-2-0247 | C41 H48 N6 O6 | 720.36353 | 2.0 | -1.7 | 7.4 | 9.2 |
| 2-2-d1E02-2-0248 | C36 H38 F3 N7 O6 | 721.28357 | -10.3 | -14.2 | 5.9 | 8.3 |
| 2-2-d1E02-2-0249 | C40 H43 N5 O6 S | 721.29340 | 44.1 | 1.4 | 6.9 | 10.5 |
| 2-2-d1E02-2-0250 | C34 H35 F N6 O9 S | 722.21703 | -2.5 | 0.0 | 3.4 | 3.5 |
| 2-2-d1E02-2-0251 | C34 H38 N6 O8 S2 | 722.21925 | -4.6 | 2.6 | 0.0 | 0.0 |
| 2-2-dlE02-2-0252 | C37 H37 F3 N4 O6 S | 722.23859 | -13.5 | -12.0 | 4.8 | 14.4 |
| 2-2-dlE02-2-0253 | C40 H39 F N4 O8 | 722.27519 | 9.9 | -16.5 | 7.4 | 16.3 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0254 | C38 H41 F3 N4 O7 | 722.29273 | -3.9 | 1.6 | 7.3 | 4.3 |
| 2-2-d1E02-2-0255 | C37 H46 N4 O9 S | 722.29855 | -9.0 | 1.9 | 3.2 | 4.9 |
| 2-2-d1E02-2-0256 | C34 H37 N5 O9 S2 | 723.20327 | -7.6 | 0.2 | 9.1 | -1.6 |
| 2-2-dlE02-2-0257 | C37 H37 N7 O7 S | 723.24752 | -14.9 | -7.4 | 2.5 | 7.0 |
| 2-2-d1E02-2-0258 | C36 H36 F3 N5 O8 | 723.25160 | -2.7 | 2.0 | 1.5 | 21.7 |
| 2-2-dlE02-2-0259 | C34 H37 F N6 O9 S | 724.23268 | -4.7 | -1.6 | 1.8 | 2.0 |
| 2-2-d1E02-2-0260 | C39 H40 N4 O8 S | 724.25668 | -9.8 | 2.6 | 3.9 | 4.2 |
| 2-2-d1E02-2-0261 | C39 H40 N4 O8 S | 724.25668 | -3.7 | 0.0 | 0.9 | 0.6 |
| 2-2-d1E02-2-0262 | C37 H45 F N4 O8 S | 724.29421 | -9.1 | -2.2 | -1.8 | 0.2 |
| 2-2-d1E02-2-0263 | C40 H45 F N6 O6 | 724.33846 | -3.8 | 3.6 | 5.9 | 9.0 |
| 2-2-d1E02-2-0264 | C38 H39 N5 O8 S | 725.25193 | -9.6 | -3.5 | 2.1 | 3.7 |
| 2-2-d1E02-2-0265 | C38 H39 N5 O8 S | 725.25193 | -0.5 | 1.7 | 2.1 | 3.7 |
| 2-2-dlE02-2-0266 | C38 H39 N5 O8 S | 725.25193 | -0.5 | 1.7 | -5.5 | 6.8 |
| 2-2-d1E02-2-0267 | C37 H39 N7 O7 S | 725.26317 | 0.0 | -30.2 | -3.7 | 0.0 |
| 2-2-dlE02-2-0268 | C35 H37 F3 N6 O6 S | 726.24474 | -3.0 | 5.7 | 4.6 | 15.6 |
| 2-2-d1E02-2-0269 | C39 H42 N4 O8 S | 726.27234 | -6.0 | 2.6 | 0.0 | 0.0 |
| 2-2-d1E02-2-0270 | C39 H42 N4 O8 S | 726.27234 | 11.4 | 0.0 | -6.2 | -6.4 |
| 2-2-d1E02-2-0271 | C36 H43 F N4 O9 S | 726.27348 | -8.0 | -1.8 | 0.0 | 2.6 |
| 2-2-d1E02-2-0272 | C33 H34 F N5 O9 S2 | 727.17820 | 0.0 | 7.8 | 0.0 | 0.0 |
| 2-2-d1E02-2-0273 | C38 H41 N5 O6 S2 | 727.24983 | 0.7 | 1.5 | 4.2 | 16.1 |
| 2-2-d1E02-2-0274 | C38 H41 N5 O8 S | 727.26758 | -8.8 | 1.6 | 2.4 | 3.6 |
| 2-2-d1E02-2-0275 | C36 H36 N6 O7 S2 | 728.20869 | 5.9 | 3.9 | 6.7 | 12.7 |
| 2-2-d1E02-2-0276 | C38 H37 F N4 O8 S | 728.23161 | -9.5 | -6.4 | 2.7 | 3.6 |
| 2-2-d1E02-2-0277 | C38 H37 F N4 O8 S | 728.23161 | -6.5 | 0.0 | 0.0 | 1.3 |
| 2-2-dlE02-2-0278 | C39 H44 N4 O8 S | 728.28799 | 0.3 | -2.9 | 1.7 | 3.5 |
| 2-2-d1E02-2-0279 | C37 H36 F N5 O8 S | 729.22686 | -5.5 | -2.8 | 1.0 | 1.9 |
| 2-2-d1E02-2-0280 | C37 H36 F N5 O8 S | 729.22686 | -12.4 | 7.1 | 6.4 | 10.7 |
| 2-2-dlE02-2-0281 | C37 H36 F N5 O8 S | 729.22686 | -5.2 | -2.6 | 0.0 | 0.0 |
| 2-2-dlE02-2-0282 | C41 H39 N5 O6 S | 729.26210 | -14.0 | -3.8 | 8.3 | 8.6 |
| 2-2-d1E02-2-0283 | C41 H39 N5 O6 S | 729.26210 | -14.6 | -7.0 | 10.3 | 9.3 |
| 2-2-d1E02-2-0284 | C38 H43 N5 O8 S | 729.28323 | -2.4 | -2.7 | 4.9 | 0.7 |
| 2-2-d1E02-2-0285 | C36 H38 N6 O9 S | 730.24210 | 3.4 | -8.4 | -1.6 | -0.1 |
| 2-2-dlE02-2-0286 | C38 H39 F N4 O8 S | 730.24726 | -6.6 | -0.9 | -12.2 | 0.0 |
| 2-2-d1E02-2-0287 | C38 H39 F N4 O8 S | 730.24726 | -10.2 | -1.6 | 2.2 | 2.2 |
| 2-2-d1E02-2-0288 | C40 H38 N6 O6 S | 730.25735 | 14.8 | -3.4 | -0.7 | 16.1 |
| 2-2-d1E02-2-0289 | C40 H38 N6 O6 S | 730.25735 | -8.5 | -14.5 | 0.2 | 3.5 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0290 | C40 H38 N6 O6 S | 730.25735 | -2.9 | -1.1 | 7.3 | 6.6 |
| 2-2-dlE02-2-0291 | C37 H42 N6 O8 S | 730.27848 | -6.3 | -7.6 | 0.5 | 0.8 |
| 2-2-dlE02-2-0292 | C37 H38 F N5 O8 S | 731.24251 | -7.8 | -1.1 | 1.1 | 1.2 |
| 2-2-dlE02-2-0293 | C36 H41 N7 O8 S | 731.27373 | -6.8 | 13.8 | 3.3 | 10.6 |
| 2-2-d1E02-2-0294 | C37 H40 N4 O8 S2 | 732.22876 | 0.0 | 0.0 | 0.0 | 0.0 |
| 2-2-dlE02-2-0295 | C36 H40 N6 O9 S | 732.25775 | -5.9 | 0.0 | 0.0 | 0.0 |
| 2-2-d1E02-2-0296 | C38 H44 N4 O9 S | 732.28290 | -11.1 | -10.8 | 2.1 | 3.5 |
| 2-2-d1E02-2-0297 | C40 H43 F3 N4 O6 | 732.31347 | -20.0 | 0.2 | 8.1 | 4.7 |
| 2-2-dlE02-2-0298 | C42 H48 N6 O6 | 732.36353 | 0.2 | 1.5 | 3.5 | 12.2 |
| 2-2-d1E02-2-0299 | C35 H35 N5 O9 S2 | 733.18762 | -9.2 | -5.6 | 0.8 | 0.8 |
| 2-2-d1E02-2-0300 | C36 H39 N5 O10 S | 733.24176 | 12.3 | 2.1 | 0.0 | 3.3 |
| 2-2-d1E02-2-0301 | C37 H40 F N5 O8 S | 733.25816 | -12.4 | 1.0 | 4.1 | 4.8 |
| 2-2-d1E02-2-0302 | C37 H42 N4 O8 S2 | 734.24441 | -8.2 | -1.2 | 3.1 | 5.6 |
| 2-2-d1E02-2-0303 | C36 H39 F N6 O8 S | 734.25341 | -3.8 | -5.6 | 0.6 | 1.9 |
| 2-2-d1E02-2-0304 | C37 H37 F3 N6 O7 | 734.26758 | 0.0 | 0.0 | 0.0 | 0.0 |
| 2-2-d1E02-2-0305 | C40 H42 N6 O6 S | 734.28865 | 6.5 | 7.9 | 12.3 | 6.8 |
| 2-2-d1E02-2-0306 | C35 H37 N5 O9 S2 | 735.20327 | -2.3 | -0.3 | 0.0 | 2.1 |
| 2-2-d1E02-2-0307 | C39 H37 N5 O8 S | 735.23628 | 33.6 | 0.0 | 0.0 | 10.9 |
| 2-2-d1E02-2-0308 | C35 H38 F N7 O8 S | 735.24866 | 0.8 | 4.8 | 6.1 | 7.9 |
| 2-2-d1E02-2-0309 | C39 H41 N7 O6 S | 735.28390 | 1.1 | -15.9 | -1.5 | 4.2 |
| 2-2-d1E02-2-0310 | C36 H37 F N4 O8 S2 | 736.20368 | -7.2 | -5.6 | -3.7 | 3.2 |
| 2-2-d1E02-2-0311 | C35 H40 N6 O8 S2 | 736.23490 | -6.8 | -7.3 | 2.8 | 2.5 |
| 2-2-d1E02-2-0312 | C40 H40 N4 O8 S | 736.25668 | 0.4 | -12.8 | 9.2 | 5.7 |
| 2-2-d1E02-2-0313 | C40 H40 N4 O8 S | 736.25668 | 7.3 | 3.1 | -0.4 | 3.8 |
| 2-2-d1E02-2-0314 | C37 H41 F N4 O9 S | 736.25783 | 4.3 | -0.1 | 4.3 | 7.0 |
| 2-2-d1E02-2-0315 | C38 H40 N8 O6 S | 736.27915 | 8.7 | 8.1 | -0.3 | 10.1 |
| 2-2-d1E02-2-0316 | C37 H39 F3 N6 O7 | 736.28323 | -17.7 | 24.6 | 2.9 | 12.6 |
| 2-2-d1E02-2-0317 | C35 H36 F N5 O10 S | 737.21669 | -147.8 | 0.0 | 0.0 | 1.6 |
| 2-2-d1E02-2-0318 | C39 H39 N5 O8 S | 737.25193 | 27.7 | 5.7 | 0.0 | 0.0 |
| 2-2-d1E02-2-0319 | C39 H39 N5 O8 S | 737.25193 | 14.3 | 5.7 | 14.2 | 8.6 |
| 2-2-dlE02-2-0320 | C39 H39 N5 O8 S | 737.25193 | -1.2 | 4.7 | -2.9 | 0.1 |
| 2-2-dlE02-2-0321 | C36 H43 N5 O8 S2 | 737.25530 | -6.0 | 0.0 | -1.7 | -2.2 |
| 2-2-d1E02-2-0322 | C40 H43 N5 O7 S | 737.28832 | -11.7 | 5.4 | 2.9 | 8.2 |
| 2-2-d1E02-2-0323 | C38 H41 F3 N4 O6 S | 738.26989 | -6.6 | 2.7 | -1.6 | 6.1 |
| 2-2-d1E02-2-0324 | C36 H36 F3 N5 O7 S | 739.22875 | -12.5 | 20.5 | 0.9 | 26.0 |
| 2-2-d1E02-2-0325 | C35 H41 N5 O9 S2 | 739.23457 | 0.0 | 0.0 | 0.0 | 0.0 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-dlE02-2-0326 | C34 H37 F N6 O8 S2 | 740.20983 | -0.9 | -2.9 | 1.4 | 2.5 |
| 2-2-d1E02-2-0327 | C41 H39 F3 N4 O6 | 740.28217 | 0.0 | -39.1 | 0.0 | 0.0 |
| 2-2-dlE02-2-0328 | C41 H39 F3 N4 O6 | 740.28217 | -18.5 | -28.7 | 4.9 | 9.3 |
| 2-2-d1E02-2-0329 | C37 H39 N7 O6 S2 | 741.24032 | -0.7 | -1.1 | 12.2 | 5.9 |
| 2-2-d1E02-2-0330 | C40 H38 F3 N5 O6 | 741.27742 | 25.5 | -2.3 | 11.0 | 14.9 |
| 2-2-d1E02-2-0331 | C40 H38 F3 N5 O6 | 741.27742 | -23.3 | 42.3 | 3.6 | 3.5 |
| 2-2-dlE02-2-0332 | C40 H38 F3 N5 O6 | 741.27742 | 0.0 | 0.0 | 8.2 | 15.0 |
| 2-2-dlE02-2-0333 | C39 H43 N5 O8 S | 741.28323 | 0.7 | 5.5 | 9.7 | 2.1 |
| 2-2-dlE02-2-0334 | C38 H38 N4 O10 S | 742.23086 | -8.5 | -2.5 | 7.2 | 13.2 |
| 2-2-d1E02-2-0335 | C38 H42 N6 O8 S | 742.27848 | 15.8 | 0.0 | 28.1 | 13.3 |
| 2-2-d1E02-2-0336 | C40 H46 N4 O8 S | 742.30364 | -8.8 | -11.9 | 3.5 | 4.6 |
| 2-2-dlE02-2-0337 | C37 H37 N5 O8 S2 | 743.20835 | -9.7 | 1.5 | 4.8 | 5.1 |
| 2-2-d1E02-2-0338 | C37 H37 N5 O8 S2 | 743.20835 | -12.5 | -9.6 | 1.9 | 2.2 |
| 2-2-dlE02-2-0339 | C37 H41 N7 O8 S | 743.27373 | 0.0 | 0.0 | 0.0 | 0.0 |
| 2-2-dlE02-2-0340 | C36 H36 N6 O8 S2 | 744.20360 | -3.6 | -0.4 | 2.6 | 2.5 |
| 2-2-dlE02-2-0341 | C35 H35 F3 N4 O9 S | 744.20768 | -1.4 | -3.4 | 2.8 | 3.4 |
| 2-2-dlE02-2-0342 | C37 H40 N6 O9 S | 744.25775 | 0.0 | -29.3 | -1.3 | 8.6 |
| 2-2-d1E02-2-0343 | C39 H44 N4 O9 S | 744.28290 | -11.4 | -3.9 | 3.5 | -2.3 |
| 2-2-dlE02-2-0344 | C40 H42 F3 N5 O6 | 745.30872 | 0.0 | 15.9 | 0.0 | 0.0 |
| 2-2-d1E02-2-0345 | C39 H37 F3 N4 O8 | 746.25635 | -10.2 | 15.4 | 12.7 | 15.1 |
| 2-2-dlE02-2-0346 | C37 H42 N6 O9 S | 746.27340 | -3.7 | -5.4 | 3.9 | 3.8 |
| 2-2-dlE02-2-0347 | C39 H43 F N4 O8 S | 746.27856 | -13.7 | -4.4 | 3.0 | 2.1 |
| 2-2-dlE02-2-0348 | C39 H41 F3 N6 O6 | 746.30397 | 3.2 | 41.3 | -0.7 | 0.2 |
| 2-2-dlE02-2-0349 | C43 H50 N6 O6 | 746.37918 | 3.9 | 9.5 | 40.5 | 14.2 |
| 2-2-d1E02-2-0350 | C38 H40 F3 N7 O6 | 747.29922 | -15.1 | -5.3 | 3.3 | 5.7 |
| 2-2-dlE02-2-0351 | C42 H45 N5 O6 S | 747.30905 | -26.2 | -16.1 | 3.4 | 3.7 |
| 2-2-dlE02-2-0352 | C36 H37 F N6 O9 S | 748.23268 | 0.0 | 0.0 | 0.0 | 0.0 |
| 2-2-dlE02-2-0353 | C36 H40 N6 O8 S2 | 748.23490 | 0.0 | 0.0 | 0.0 | 0.0 |
| 2-2-d1E02-2-0354 | C38 H44 N4 O8 S2 | 748.26006 | -1.9 | -4.5 | 3.5 | 3.4 |
| 2-2-d1E02-2-0355 | C36 H43 F3 N4 O8 S | 748.27537 | -1.5 | -8.2 | -0.3 | -0.6 |
| 2-2-d1E02-2-0356 | C40 H43 F3 N4 O7 | 748.30838 | -33.9 | 4.0 | 9.4 | 9.2 |
| 2-2-d1E02-2-0357 | C35 H35 N5 O8 S3 | 749.16478 | -3.0 | -1.3 | 3.9 | 4.0 |
| 2-2-dlE02-2-0358 | C36 H39 N5 O9 S2 | 749.21892 | -0.9 | -8.4 | 0.8 | 0.0 |
| 2-2-d1E02-2-0359 | C34 H34 N6 O10 S2 | 750.17778 | 0.0 | 0.0 | 0.0 | 0.0 |
| 2-2-d1E02-2-0360 | C36 H39 F N6 O9 S | 750.24833 | -2.3 | -1.1 | 0.0 | 6.6 |
| 2-2-d1E02-2-0361 | C35 H41 F3 N4 O9 S | 750.25463 | -7.5 | 0.4 | 0.5 | 0.1 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0362 | C41 H42 N4 O8 S | 750.27234 | -0.2 | 1.9 | 4.3 | 11.7 |
| 2-2-d1E02-2-0363 | C41 H42 N4 O8 S | 750.27234 | 13.7 | 0.0 | 0.0 | 0.0 |
| 2-2-d1E02-2-0364 | C42 H47 F N6 O6 | 750.35411 | -5.6 | 12.4 | 3.6 | 10.2 |
| 2-2-d1E02-2-0365 | C40 H41 N5 O8 S | 751.26758 | -7.7 | -4.1 | -0.5 | 2.2 |
| 2-2-d1E02-2-0366 | C40 H41 N5 O8 S | 751.26758 | 3.7 | 2.2 | 18.8 | 12.0 |
| 2-2-d1E02-2-0367 | C40 H41 N5 O8 S | 751.26758 | 4.6 | -4.4 | 8.2 | 0.0 |
| 2-2-d1E02-2-0368 | C37 H41 F N4 O8 S2 | 752.23498 | -15.6 | -4.4 | 4.2 | 5.0 |
| 2-2-d1E02-2-0369 | C37 H39 F3 N6 O6 S | 752.26039 | -26.5 | 1.0 | 6.8 | 7.0 |
| 2-2-d1E02-2-0370 | C35 H36 F N5 O9 S2 | 753.19385 | -4.1 | 1.3 | 4.1 | -0.7 |
| 2-2-d1E02-2-0371 | C40 H43 N5 O6 S2 | 753.26548 | -41.0 | -2.4 | -4.4 | 1.4 |
| 2-2-dlE02-2-0372 | C37 H37 F3 N4 O8 S | 754.22842 | -0.9 | -1.2 | 2.6 | 2.9 |
| 2-2-d1E02-2-0373 | C37 H37 F3 N4 O8 S | 754.22842 | -1.8 | -11.0 | 2.6 | 2.9 |
| 2-2-d1E02-2-0374 | C40 H39 F N4 O8 S | 754.24726 | -14.6 | -4.0 | 11.3 | 0.0 |
| 2-2-d1E02-2-0375 | C40 H39 F N4 O8 S | 754.24726 | -6.3 | -11.5 | 7.6 | 25.4 |
| 2-2-d1E02-2-0376 | C41 H46 N4 O8 S | 754.30364 | -25.3 | 0.0 | -22.1 | 0.0 |
| 2-2-d1E02-2-0377 | C36 H36 F3 N5 O8 S | 755.22367 | -4.8 | -0.8 | 1.0 | 2.2 |
| 2-2-d1E02-2-0378 | C39 H38 F N5 O8 S | 755.24251 | -8.9 | -2.3 | 5.3 | 5.0 |
| 2-2-d1E02-2-0379 | C39 H38 F N5 O8 S | 755.24251 | -25.5 | 22.0 | 5.4 | 21.3 |
| 2-2-dlE02-2-0380 | C39 H38 F N5 O8 S | 755.24251 | 0.0 | 0.0 | 0.0 | 0.0 |
| 2-2-dlE02-2-0381 | C40 H45 N5 O8 S | 755.29888 | 0.0 | 0.0 | 0.0 | 0.0 |
| 2-2-dlE02-2-0382 | C39 H40 N4 O10 S | 756.24651 | -5.8 | -1.7 | 2.0 | 4.7 |
| 2-2-dlE02-2-0383 | C39 H44 N6 O8 S | 756.29413 | 0.0 | 0.0 | 0.0 | 4.3 |
| 2-2-dlE02-2-0384 | C38 H43 N7 O8 S | 757.28938 | -6.5 | -2.2 | 7.0 | 13.0 |
| 2-2-dlE02-2-0385 | C40 H46 N4 O9 S | 758.29855 | 0.0 | -11.6 | 0.0 | 12.5 |
| 2-2-d1E02-2-0386 | C42 H45 F3 N4 O6 | 758.32912 | -5.6 | 17.3 | 0.0 | 18.5 |
| 2-2-dlE02-2-0387 | C37 H37 N5 O9 S2 | 759.20327 | -19.7 | -6.2 | 3.9 | 2.7 |
| 2-2-d1E02-2-0388 | C39 H42 F N5 O8 S | 759.27381 | -44.4 | -33.7 | 9.3 | 6.7 |
| 2-2-d1E02-2-0389 | C35 H35 F3 N4 O8 S2 | 760.18484 | 8.4 | 7.3 | 2.9 | 7.9 |
| 2-2-d1E02-2-0390 | C38 H37 F N4 O10 S | 760.22144 | 4.1 | 0.5 | 11.7 | 5.7 |
| 2-2-d1E02-2-0391 | C39 H44 N4 O8 S2 | 760.26006 | 0.0 | 0.0 | 0.0 | 0.0 |
| 2-2-d1E02-2-0392 | C38 H41 F N6 O8 S | 760.26906 | 9.1 | -0.2 | 0.0 | 0.0 |
| 2-2-d1E02-2-0393 | C35 H35 N7 O9 S2 | 761.19377 | 0.0 | 0.0 | 0.0 | 0.0 |
| 2-2-d1E02-2-0394 | C34 H34 F3 N5 O10 S | 761.19785 | -3.1 | 2.7 | 0.0 | 0.0 |
| 2-2-d1E02-2-0395 | C41 H39 N5 O8 S | 761.25193 | 3.8 | 32.3 | 5.9 | 17.4 |
| 2-2-dlE02-2-0396 | C37 H40 F N7 O8 S | 761.26431 | -3.7 | 7.5 | 11.4 | 1.6 |
| 2-2-d1E02-2-0397 | C37 H42 N6 O8 S2 | 762.25055 | 0.0 | 0.0 | 0.0 | 0.0 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0398 | C39 H43 F N4 O9 S | 762.27348 | -8.2 | -4.3 | 4.1 | 9.0 |
| 2-2-dlE02-2-0399 | C35 H37 N7 O9 S2 | 763.20942 | -6.0 | -7.7 | 0.0 | 0.0 |
| 2-2-d1E02-2-0400 | C38 H45 N5 O8 S2 | 763.27095 | -6.6 | -4.9 | 0.0 | 1.2 |
| 2-2-d1E02-2-0401 | C41 H45 N7 O6 S | 763.31520 | -8.3 | -3.3 | 3.8 | 8.5 |
| 2-2-d1E02-2-0402 | C40 H43 F3 N4 O6 S | 764.28554 | -38.7 | -22.0 | 11.6 | 8.2 |
| 2-2-d1E02-2-0403 | C37 H43 N5 O9 S2 | 765.25022 | -3.2 | -2.4 | 2.6 | 3.5 |
| 2-2-d1E02-2-0404 | C34 H34 N6 O9 S3 | 766.15494 | -8.7 | -0.4 | 2.2 | 7.8 |
| 2-2-d1E02-2-0405 | C36 H39 F N6 O8 S2 | 766.22548 | -10.3 | 7.5 | 5.7 | 11.7 |
| 2-2-d1E02-2-0406 | C39 H37 N5 O8 S2 | 767.20835 | 1.2 | 1.9 | 3.4 | 4.8 |
| 2-2-d1E02-2-0407 | C39 H37 N5 O8 S2 | 767.20835 | 2.1 | 1.0 | 3.5 | 4.9 |
| 2-2-dlE02-2-0408 | C38 H36 N6 O8 S2 | 768.20360 | -8.1 | -0.9 | 7.9 | 3.9 |
| 2-2-d1E02-2-0409 | C38 H36 N6 O8 S2 | 768.20360 | -6.9 | 5.3 | 4.4 | 19.6 |
| 2-2-d1E02-2-0410 | C38 H36 N6 O8 S2 | 768.20360 | 0.0 | -27.1 | 0.0 | 0.0 |
| 2-2-d1E02-2-0411 | C40 H40 N4 O10 S | 768.24651 | -13.2 | -7.9 | 0.7 | 5.1 |
| 2-2-d1E02-2-0412 | C42 H48 N4 O8 S | 768.31929 | 6.7 | 11.6 | 0.0 | 10.7 |
| 2-2-d1E02-2-0413 | C39 H39 N5 O8 S2 | 769.22400 | -3.5 | -3.3 | 1.6 | 4.8 |
| 2-2-d1E02-2-0414 | C39 H39 N5 O8 S2 | 769.22400 | 10.2 | 7.6 | 0.0 | -3.6 |
| 2-2-d1E02-2-0415 | C38 H38 N6 O8 S2 | 770.21925 | -6.8 | -1.4 | -6.4 | 3.0 |
| 2-2-d1E02-2-0416 | C37 H37 F3 N4 O9 S | 770.22333 | -9.2 | 0.0 | 2.4 | 1.5 |
| 2-2-d1E02-2-0417 | C40 H46 N6 O8 S | 770.30978 | -3.5 | -0.7 | 4.3 | 8.9 |
| 2-2-d1E02-2-0418 | C35 H35 F3 N6 O9 S | 772.21383 | -9.8 | -1.1 | 0.0 | 0.0 |
| 2-2-d1E02-2-0419 | C38 H40 N6 O8 S2 | 772.23490 | -9.8 | -3.7 | 2.0 | 3.6 |
| 2-2-d1E02-2-0420 | C41 H39 F3 N4 O8 | 772.27200 | -13.1 | 0.8 | 4.1 | 6.6 |
| 2-2-d1E02-2-0421 | C41 H45 F N4 O8 S | 772.29421 | 5.8 | -10.9 | -6.0 | 0.5 |
| 2-2-d1E02-2-0422 | C37 H39 N7 O8 S2 | 773.23015 | 0.0 | 0.0 | 33.8 | 0.0 |
| 2-2-d1E02-2-0423 | C36 H38 N8 O8 S2 | 774.22540 | -3.8 | -3.9 | 13.0 | 9.8 |
| 2-2-d1E02-2-0424 | C35 H37 F3 N6 O9 S | 774.22948 | -2.8 | -1.6 | 1.9 | 2.3 |
| 2-2-d1E02-2-0425 | C40 H46 N4 O8 S2 | 774.27571 | -9.9 | -1.4 | 5.8 | 0.0 |
| 2-2-d1E02-2-0426 | C38 H45 F3 N4 O8 S | 774.29102 | -7.5 | 0.0 | -4.8 | 0.0 |
| 2-2-d1E02-2-0427 | C41 H45 F3 N6 O6 | 774.33527 | 4.4 | -3.9 | 2.0 | 7.0 |
| 2-2-d1E02-2-0428 | C37 H37 N5 O8 S3 | 775.18043 | -1.4 | -6.3 | 1.3 | -0.1 |
| 2-2-d1E02-2-0429 | C38 H41 N5 O9 S2 | 775.23457 | -8.3 | 1.3 | 1.0 | 2.8 |
| 2-2-dlE02-2-0430 | C36 H36 N6 O10 S2 | 776.19343 | -9.1 | -5.4 | 3.9 | 5.6 |
| 2-2-dlE02-2-0431 | C37 H43 F3 N4 O9 S | 776.27028 | -15.7 | -15.1 | 0.0 | 0.0 |
| 2-2-dlE02-2-0432 | C34 H34 F3 N5 O9 S2 | 777.17500 | -7.0 | -2.3 | -4.3 | 7.5 |
| 2-2-dlE02-2-0433 | C39 H37 F3 N4 O8 S | 778.22842 | -7.5 | -2.1 | 5.6 | 17.5 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-dlE02-2-0434 | C39 H37 F3 N4 O8 S | 778.22842 | -10.5 | -2.3 | 0.8 | 3.4 |
| 2-2-dlE02-2-0435 | C39 H43 F N4 O8 S2 | 778.25063 | -7.7 | 0.0 | 0.0 | 0.0 |
| 2-2-dlE02-2-0436 | C35 H37 N7 O8 S3 | 779.18657 | -6.8 | -0.4 | 1.9 | 2.8 |
| 2-2-d1E02-2-0437 | C38 H36 F3 N5 O8 S | 779.22367 | -13.6 | 0.4 | 2.3 | 2.8 |
| 2-2-d1E02-2-0438 | C38 H36 F3 N5 O8 S | 779.22367 | -24.5 | 11.6 | 9.2 | 11.5 |
| 2-2-d1E02-2-0439 | C38 H36 F3 N5 O8 S | 779.22367 | 0.0 | 0.0 | 0.0 | 0.0 |
| 2-2-dlE02-2-0440 | C39 H39 F3 N4 O8 S | 780.24407 | 2.3 | -8.8 | -5.3 | 4.7 |
| 2-2-d1E02-2-0441 | C39 H39 F3 N4 O8 S | 780.24407 | 0.0 | 0.0 | 0.0 | 0.0 |
| 2-2-d1E02-2-0442 | C38 H38 F3 N5 O8 S | 781.23932 | -4.7 | -3.5 | 1.1 | 2.1 |
| 2-2-d1E02-2-0443 | C41 H42 N4 O10 S | 782.26216 | -7.8 | -3.7 | 6.6 | 9.7 |
| 2-2-d1E02-2-0444 | C38 H40 F3 N5 O8 S | 783.25497 | -8.1 | 9.2 | -0.1 | 1.4 |
| 2-2-d1E02-2-0445 | C37 H39 F3 N6 O8 S | 784.25022 | -23.0 | -3.3 | -0.5 | 1.3 |
| 2-2-dlE02-2-0446 | C41 H48 N6 O8 S | 784.32543 | -7.1 | -5.3 | -3.1 | 0.0 |
| 2-2-d1E02-2-0447 | C36 H38 F3 N7 O8 S | 785.24547 | -6.7 | -9.0 | 5.5 | 6.7 |
| 2-2-dlE02-2-0448 | C40 H43 N5 O8 S2 | 785.25530 | 5.4 | 0.5 | 8.7 | 1.1 |
| 2-2-d1E02-2-0449 | C37 H37 F3 N4 O8 S2 | 786.20049 | 6.7 | 7.7 | 9.1 | 5.8 |
| 2-2-dlE02-2-0450 | C40 H39 F N4 O10 S | 786.23709 | -8.5 | -1.0 | -1.8 | 2.2 |
| 2-2-d1E02-2-0451 | C38 H41 F3 N4 O9 S | 786.25463 | -18.8 | -2.5 | 5.0 | 0.1 |
| 2-2-d1E02-2-0452 | C37 H37 N7 O9 S2 | 787.20942 | -2.3 | -4.6 | 4.7 | 4.4 |
| 2-2-d1E02-2-0453 | C36 H36 F3 N5 O10 S | 787.21350 | -1.8 | -1.5 | 2.2 | 10.4 |
| 2-2-d1E02-2-0454 | C40 H45 F N6 O8 S | 788.30036 | -7.4 | -1.5 | 6.1 | 6.8 |
| 2-2-d1E02-2-0455 | C37 H39 N7 O9 S2 | 789.22507 | -4.9 | -8.3 | 3.9 | 6.7 |
| 2-2-dlE02-2-0456 | C43 H47 N7 O6 S | 789.33085 | -0.5 | -8.1 | 4.0 | 23.9 |
| 2-2-dlE02-2-0457 | C35 H37 F3 N6 O8 S2 | 790.20664 | -12.0 | -11.2 | 0.7 | 5.2 |
| 2-2-d1E02-2-0458 | C38 H41 N5 O8 S3 | 791.21173 | -19.2 | -19.7 | 5.8 | 5.6 |
| 2-2-dlE02-2-0459 | C36 H36 N6 O9 S3 | 792.17059 | 2.4 | 0.1 | 5.9 | 9.8 |
| 2-2-d1E02-2-0460 | C41 H39 N5 O8 S2 | 793.22400 | 6.2 | 6.8 | 7.5 | 17.0 |
| 2-2-d1E02-2-0461 | C41 H39 N5 O8 S2 | 793.22400 | 0.0 | 0.0 | 0.0 | 0.0 |
| 2-2-d1E02-2-0462 | C40 H38 N6 O8 S2 | 794.21925 | 2.4 | -1.9 | 3.1 | 4.2 |
| 2-2-d1E02-2-0463 | C40 H38 N6 O8 S2 | 794.21925 | 29.3 | -4.5 | 7.1 | 14.2 |
| 2-2-d1E02-2-0464 | C40 H38 N6 O8 S2 | 794.21925 | 0.0 | 0.0 | 0.0 | 0.0 |
| 2-2-d1E02-2-0465 | C40 H43 F3 N4 O8 S | 796.27537 | -23.1 | -0.7 | 7.0 | 6.7 |
| 2-2-d1E02-2-0466 | C42 H48 N6 O8 S | 796.32543 | -2.6 | -4.2 | 1.2 | 1.1 |
| 2-2-d1E02-2-0467 | C37 H37 F3 N6 O9 S | 798.22948 | 13.5 | 3.2 | 0.0 | 0.0 |
| 2-2-d1E02-2-0468 | C40 H42 N6 O8 S2 | 798.25055 | 3.9 | -14.7 | -4.0 | 5.6 |
| 2-2-d1E02-2-0469 | C39 H37 N5 O10 S2 | 799.19818 | -7.6 | -12.6 | -1.1 | 4.1 |

(continued)

| [Table 11-3-6] | | | | | | |
|---|---|---|---|---|---|---|
| | | | AS-MS(%) | | | |
| ID | Molecular Formula | Exact Mass | N1 | N2 | N3 | N4 |
| 2-2-d1E02-2-0470 | C39 H41 N7 O8 S2 | 799.24580 | 0.0 | 0.0 | 0.0 | 0.0 |
| 2-2-d1E02-2-0471 | C38 H40 N8 O8 S2 | 800.24105 | -0.9 | 5.9 | 6.7 | 9.2 |
| 2-2-d1E02-2-0472 | C37 H39 F3 N6 O9 S | 800.24513 | -16.3 | -0.1 | -5.4 | -0.9 |
| 2-2-dIE02-2-0473 | C43 H47 F3 N6 O6 | 800.35092 | -12.2 | 16.1 | 9.2 | 15.1 |
| 2-2-d1 E02-2-04 7 4 | C40 H43 N5 O9 S2 | 801.25022 | 3.8 | -0.2 | 7.4 | 3.9 |
| 2-2-d1E02-2-0475 | C38 H41 F3 N4 O8 S2 | 802.23179 | 6.8 | -3.3 | 11.3 | 9.5 |
| 2-2-d1E02-2-0476 | C36 H36 F3 N5 O9 S2 | 803.19065 | -5.8 | 4.4 | 3.6 | 0.0 |
| 2-2-dIE02-2-0477 | C41 H39 F3 N4 O8 S | 804.24407 | -11.3 | 1.2 | 4.0 | 2.7 |
| 2-2-dIE02-2-0478 | C41 H39 F3 N4 O8 S | 804.24407 | 0.0 | 0.0 | 0.0 | 0.0 |
| 2-2-dIE02-2-0479 | C37 H39 N7 O8 S3 | 805.20222 | 2.1 | -0.4 | 5.7 | 9.8 |
| 2-2-d1E02-2-0480 | C40 H38 F3 N5 O8 S | 805.23932 | 37.3 | 42.2 | 7.7 | -5.6 |
| 2-2-dIE02-2-0481 | C40 H38 F3 N5 O8 S | 805.23932 | -30.6 | -5.6 | 7.0 | 10.0 |
| 2-2-dIE02-2-0482 | C40 H38 F3 N5 O8 S | 805.23932 | 0.6 | -0.4 | 10.4 | 0.0 |
| 2-2-d1E02-2-0483 | C40 H42 F3 N5 O8 S | 809.27062 | 0.0 | 0.0 | 0.0 | 0.0 |
| 2-2-d1E02-2-0484 | C39 H37 F3 N4 O10 S | 810.21825 | -10.6 | 10.4 | 7.4 | -0.3 |
| 2-2-d1E02-2-0485 | C39 H41 F3 N6 O8 S | 810.26587 | -32.2 | -3.3 | 5.9 | 0.3 |
| 2-2-d1E02-2-0486 | C43 H50 N6 O8 S | 810.34108 | -5.5 | -0.6 | -0.6 | 0.7 |
| 2-2-d1E02-2-0487 | C38 H40 F3 N7 O8 S | 811.26112 | -4.8 | 7.4 | 0.0 | 0.0 |
| 2-2-d1E02-2-0488 | C42 H45 N5 O8 S2 | 811.27095 | -11.0 | 15.1 | 0.5 | 4.7 |
| 2-2-d1E02-2-0489 | C40 H43 F3 N4 O9 S | 812.27028 | 34.5 | 46.3 | 10.8 | 33.5 |
| 2-2-d1E02-2-0490 | C42 H47 F N6 O8 S | 814.31601 | -9.5 | 1.3 | -2.6 | 1.4 |
| 2-2-d1E02-2-0491 | C37 H39 F3 N6 O8 S2 | 816.22229 | -3.2 | 9.3 | 8.7 | 19.0 |
| 2-2-dIE02-2-0492 | C40 H43 N5 O8 S3 | 817.22738 | 0.4 | 1.4 | 12.8 | 7.0 |
| 2-2-dIE02-2-0493 | C42 H45 F3 N4 O8 S | 822.29102 | -12.9 | 2.7 | 3.2 | 1.4 |
| 2-2-d1E02-2-0494 | C41 H39 N5 O10 S2 | 825.21383 | 4.9 | 0.0 | -6.6 | 19.5 |
| 2-2-d1E02-2-0495 | C41 H45 N7 O8 S2 | 827.27710 | 4.2 | 7.6 | 11.2 | 11.1 |
| 2-2-d1E02-2-0496 | C40 H43 F3 N4 O8 S2 | 828.24744 | -33.0 | 2.4 | 0.6 | 6.3 |
| 2-2-d1E02-2-0497 | C41 H39 F3 N4 O10 S | 836.23390 | 6.4 | 0.0 | 0.0 | 0.0 |
| 2-2-dIE02-2-0498 | C41 H45 F3 N6 O8 S | 838.29717 | -13.1 | -8.5 | 2.6 | 7.3 |
| 2-2-d1E02-2-0499 | C43 H47 N7 O8 S2 | 853.29275 | 10.1 | 4.2 | 1.6 | 4.5 |
| 2-2-dIE02-2-0500 | C43 H47 F3 N6 O8 S | 864.31282 | -20.0 | 0.0 | -4.7 | 5.2 |

[Table 604]

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0001 | C36 H34 N4 O6 S | 650.21991 | 19.0 | 6.3 | 9.3 | 8.0 |
| 2-2-d1E03-2-0002 | C37 H42 N4 O5 S | 654.28759 | -13.1 | -9.1 | 10.8 | 1.2 |
| 2-2-d1E03-2-0003 | C36 H40 N4 O6 S | 656.26686 | -31.6 | -2.0 | 15.2 | 13.3 |
| 2-2-d1E03-2-0004 | C38 H36 N4 O5 S | 660.24064 | -4.9 | -4.8 | 6.6 | 13.3 |
| 2-2-d1E03-2-0005 | C38 H36 N4 O5 S | 660.24064 | -14.8 | 18.7 | 11.3 | 10.8 |
| 2-2-d1E03-2-0006 | C37 H35 N5 O5 S | 661.23589 | -16.7 | 14.6 | 11.1 | 3.3 |
| 2-2-d1E03-2-0007 | C37 H36 N4 O6 S | 664.23556 | -5.9 | 33.3 | 4.9 | 6.5 |
| 2-2-d1E03-2-0008 | C36 H34 N4 O5 S2 | 666.19706 | -53.3 | -7.9 | 6.8 | -0.3 |
| 2-2-d1E03-2-0009 | C35 H33 N5 O7 S | 667.21007 | -1.3 | -5.9 | 8.0 | 6.7 |
| 2-2-d1E03-2-0010 | C36 H33 FN4 O6 S | 668.21048 | 13.3 | 34.3 | 10.8 | 14.0 |
| 2-2-d1E03-2-0011 | C38 H44 N4 O5 S | 668.30324 | -7.3 | 4.9 | 7.2 | 1.2 |
| 2-2-dlE03-2-0012 | C37 H42 N4 O6 S | 670.28251 | 8.6 | 65.3 | 10.2 | 10.5 |
| 2-2-d1E03-2-0013 | C37 H41 FN4 O5 S | 672.27817 | 9.5 | -4.0 | 5.3 | 7.3 |
| 2-2-dlE03-2-0014 | C39 H38 N4 O5 S | 674.25629 | 0.7 | -3.9 | 17.6 | 2.4 |
| 2-2-d1E03-2-0015 | C39 H38 N4 O5 S | 674.25629 | 0.0 | 14.0 | 12.3 | 0.7 |
| 2-2-dlE03-2-0016 | C36 H39 F N4 O6 S | 674.25743 | -12.6 | -8.4 | 12.9 | 15.4 |
| 2-2-d1E03-2-0017 | C38 H37 N5 O5 S | 675.25154 | 2.5 | 15.7 | 17.5 | 13.6 |
| 2-2-dlE03-2-0018 | C38 H36 N4 O6 S | 676.23556 | -1.0 | -2.1 | 17.8 | -0.8 |
| 2-2-dlE03-2-0019 | C36 H34 N6 O6 S | 678.22605 | 8.4 | 4.3 | 16.8 | 10.7 |
| 2-2-dlE03-2-0020 | C38 H35 F N4 O5 S | 678.23122 | -1.0 | -8.4 | 11.9 | 2.6 |
| 2-2-d1E03-2-0021 | C38 H35 F N4 O5 S | 678.23122 | 0.0 | 0.0 | 0.0 | -14.0 |
| 2-2-d1E03-2-0022 | C37 H34 F N5 O5 S | 679.22647 | -11.9 | 1.8 | 11.3 | 10.3 |
| 2-2-d1E03-2-0023 | C37 H36 N4 O5 S2 | 680.21271 | -43.2 | 1.7 | 17.5 | 11.8 |
| 2-2-dlE03-2-0024 | C36 H36 N6 O6 S | 680.24170 | -3.6 | -7.0 | 11.3 | 5.0 |
| 2-2-d1E03-2-0025 | C39 H44 N4 O5 S | 680.30324 | -15.2 | 46.7 | 1.4 | 20.3 |
| 2-2-dlE03-2-0026 | C36 H35 N5 O7 S | 681.22572 | 0.0 | 0.0 | 0.0 | 0.0 |
| 2-2-d1E03-2-0027 | C38 H42 N4 O6 S | 682.28251 | -22.7 | 17.2 | 11.7 | 10.2 |
| 2-2-d1E03-2-0028 | C35 H33 N5 O6 S2 | 683.18723 | -3.4 | 11.2 | 31.4 | 11.9 |
| 2-2-d1E03-2-0029 | C36 H33 F N4 O5 S2 | 684.18764 | -45.1 | 18.3 | 11.8 | -2.3 |
| 2-2-d1E03-2-0030 | C40 H36 N4 O5 S | 684.24064 | 2.7 | 36.5 | 6.3 | 19.7 |
| 2-2-d1E03-2-0031 | C40 H36 N4 O5 S | 684.24064 | 18.0 | 24.5 | 27.6 | 2.8 |
| 2-2-d1E03-2-0032 | C35 H32 F N5 O7 S | 685.20065 | -0.1 | -4.6 | 10.2 | 3.9 |
| 2-2-dlE03-2-0033 | C39 H35 N5 O5 S | 685.23589 | 5.1 | -7.9 | 6.4 | 0.9 |
| 2-2-dlE03-2-0034 | C39 H35 N5 O5 S | 685.23589 | -25.0 | 13.4 | 11.0 | 19.2 |
| 2-2-dlE03-2-0035 | C39 H35 N5 O5 S | 685.23589 | 14.5 | -92.3 | 7.4 | 34.5 |
| 2-2-d1E03-2-0036 | C40 H38 N4 O5 S | 686.25629 | 0.0 | 0.0 | 0.0 | 0.0 |
| 2-2-d1E03-2-0037 | C40 H38 N4 O5 S | 686.25629 | 0.0 | 0.0 | 0.0 | 0.0 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0038 | C39 H37 N5 O5 S | 687.25154 | 9.8 | 8.8 | 9.9 | 17.7 |
| 2-2-d1E03-2-0039 | C39 H39 N5 O5 S | 689.26719 | -15.4 | 9.8 | 8.4 | 5.0 |
| 2-2-dlE03-2-0040 | C39 H38 N4 O6 S | 690.25121 | 0.0 | 0.0 | 5.0 | 4.4 |
| 2-2-d1E03-2-0041 | C38 H38 N6 O5 S | 690.26244 | -8.8 | -12.7 | 8.0 | 6.7 |
| 2-2-dlE03-2-0042 | C37 H37 N7 O5 S | 691.25769 | -10.6 | -4.0 | 14.8 | -5.0 |
| 2-2-dlE03-2-0043 | C38 H36 N4 O5 S2 | 692.21271 | -17.2 | -2.2 | 9.0 | 3.5 |
| 2-2-d1E03-2-0044 | C37 H36 N6 O6 S | 692.24170 | 2.3 | -0.7 | 14.2 | 5.6 |
| 2-2-dlE03-2-0045 | C39 H40 N4 O6 S | 692.26686 | -17.2 | 4.7 | 13.4 | 14.5 |
| 2-2-d1E03-2-0046 | C37 H35 N5 O7 S | 693.22572 | 0.8 | 7.8 | 5.8 | -8.0 |
| 2-2-dlE03-2-0047 | C38 H35 FN4 O6 S | 694.22613 | -3.5 | -5.9 | 20.0 | 5.1 |
| 2-2-dlE03-2-0048 | C37 H38 N6 O6 S | 694.25735 | 3.7 | 16.8 | 12.4 | 11.9 |
| 2-2-dlE03-2-0049 | C40 H46 N4 O5 S | 694.31889 | -68.4 | 9.7 | 5.0 | -5.9 |
| 2-2-dlE03-2-0050 | C36 H33 F N6 O6 S | 696.21663 | 0.0 | 0.0 | 0.0 | 0.0 |
| 2-2-dlE03-2-0051 | C36 H36 N6 O5 S2 | 696.21886 | -19.7 | 19.0 | 10.8 | 9.7 |
| 2-2-d1E03-2-0052 | C39 H44 N4 O6 S | 696.29816 | 7.1 | 23.2 | 0.7 | -1.0 |
| 2-2-dlE03-2-0053 | C36 H35 N5 O6 S2 | 697.20288 | -11.9 | 9.9 | 19.0 | 16.1 |
| 2-2-d1E03-2-0054 | C36 H35 F N6 O6 S | 698.23228 | 0.6 | 6.3 | 13.0 | 3.9 |
| 2-2-dlE03-2-0055 | C41 H38 N4 O5 S | 698.25629 | -24.5 | 0.6 | 11.8 | 8.1 |
| 2-2-dlE03-2-0056 | C41 H38 N4 O5 S | 698.25629 | 0.0 | 0.0 | 0.0 | 0.0 |
| 2-2-dlE03-2-0057 | C39 H43 FN4 O5 S | 698.29382 | 54.6 | 91.6 | 9.6 | 25.1 |
| 2-2-dlE03-2-0058 | C40 H37 N5 O5 S | 699.25154 | -12.1 | 29.9 | 7.0 | 11.4 |
| 2-2-d1E03-2-0059 | C40 H37 N5 O5 S | 699.25154 | 25.8 | -32.6 | 23.2 | 40.8 |
| 2-2-dlE03-2-0060 | C40 H37 N5 O5 S | 699.25154 | 0.0 | 0.0 | 0.0 | 0.0 |
| 2-2-d1E03-2-0061 | C41 H40 N4 O5 S | 700.27194 | 0.0 | -8.9 | 0.0 | 0.0 |
| 2-2-d1E03-2-0062 | C41 H40 N4 O5 S | 700.27194 | 0.0 | 29.9 | 0.0 | -15.5 |
| 2-2-d1E03-2-0063 | C38 H41 F N4 O6 S | 700.27308 | -12.7 | 10.8 | 14.1 | 7.8 |
| 2-2-d1E03-2-0064 | C35 H32 F N5 O6 S2 | 701.17780 | 2.6 | 20.9 | 2.0 | 0.2 |
| 2-2-d1E03-2-0065 | C40 H39 N5 O5 S | 701.26719 | 15.4 | 8.4 | 20.5 | 5.4 |
| 2-2-d1E03-2-0066 | C40 H35 F N4 O5 S | 702.23122 | -12.2 | 38.4 | 16.4 | 14.8 |
| 2-2-d1E03-2-0067 | C40 H35 F N4 O5 S | 702.23122 | -86.7 | 0.0 | 0.0 | 10.6 |
| 2-2-d1E03-2-0068 | C41 H42 N4 O5 S | 702.28759 | 3.7 | -19.9 | -7.9 | 9.1 |
| 2-2-d1E03-2-0069 | C39 H34 F N5 O5 S | 703.22647 | 15.1 | 38.3 | 9.3 | 9.7 |
| 2-2-d1E03-2-0070 | C39 H34 F N5 O5 S | 703.22647 | -7.9 | 12.1 | 9.3 | 8.4 |
| 2-2-d1E03-2-0071 | C39 H34 F N5 O5 S | 703.22647 | 0.0 | 0.0 | 0.0 | 0.0 |
| 2-2-d1E03-2-0072 | C40 H41 N5 O5 S | 703.28284 | -1.5 | 2.3 | 37.4 | -21.1 |
| 2-2-d1E03-2-0073 | C38 H36 N6 O6 S | 704.24170 | -46.0 | -8.7 | 13.4 | 10.7 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0074 | C40 H37 F N4 O5 S | 704.24687 | 0.0 | 0.0 | 9.9 | -0.7 |
| 2-2-d1E03-2-0075 | C40 H37 F N4 O5 S | 704.24687 | 0.0 | 0.0 | 0.0 | 0.0 |
| 2-2-d1E03-2-0076 | C39 H40 N6 O5 S | 704.27809 | -12.6 | -59.1 | -6.8 | 2.8 |
| 2-2-d1E03-2-0077 | C39 H36 F N5 O5 S | 705.24212 | -14.2 | 2.1 | 7.4 | 6.9 |
| 2-2-d1E03-2-0078 | C38 H39 N7 O5 S | 705.27334 | 0.3 | 11.3 | 12.7 | 4.0 |
| 2-2-d1E03-2-0079 | C39 H38 N4 O5 S2 | 706.22836 | 0.0 | 0.0 | 0.0 | 0.0 |
| 2-2-d1E03-2-0080 | C38 H38 N6 O6 S | 706.25735 | -26.7 | 18.6 | 6.7 | 15.9 |
| 2-2-d1E03-2-0081 | C40 H42 N4 O6 S | 706.28251 | 9.9 | 6.8 | 7.6 | 4.3 |
| 2-2-d1E03-2-0082 | C37 H33 N5 O6 S2 | 707.18723 | -35.6 | 25.4 | 16.5 | 8.8 |
| 2-2-d1E03-2-0083 | C38 H37 N5 O7 S | 707.24137 | -0.8 | -2.4 | 13.3 | 6.8 |
| 2-2-d1E03-2-0084 | C39 H38 F N5 O5 S | 707.25777 | -2.5 | 5.8 | 8.6 | 17.2 |
| 2-2-d1E03-2-0085 | C39 H40 N4 O5 S2 | 708.24401 | 7.7 | 20.5 | 16.1 | 27.9 |
| 2-2-d1E03-2-0086 | C38 H37 F N6 O5 S | 708.25302 | 13.8 | -54.8 | -0.5 | 7.8 |
| 2-2-d1E03-2-0087 | C37 H35 N5 O6 S2 | 709.20288 | -2.6 | 0.0 | 0.0 | -3.6 |
| 2-2-d1E03-2-0088 | C37 H36 F N7 O5 S | 709.24827 | -1.0 | -2.8 | 7.5 | 2.0 |
| 2-2-d1E03-2-0089 | C38 H35 F N4 O5 S2 | 710.20329 | 0.0 | 0.0 | 0.0 | 0.0 |
| 2-2-d1E03-2-0090 | C37 H38 N6 O5 S2 | 710.23451 | 39.9 | 30.4 | 6.1 | 18.7 |
| 2-2-d1E03-2-0091 | C42 H38 N4 O5 S | 710.25629 | 0.0 | 0.0 | 0.0 | 0.0 |
| 2-2-d1E03-2-0092 | C42 H38 N4 O5 S | 710.25629 | 11.8 | 9.1 | 15.7 | 4.0 |
| 2-2-d1E03-2-0093 | C39 H39 F N4 O6 S | 710.25743 | -51.0 | 1.8 | 18.8 | 9.3 |
| 2-2-d1E03-2-0094 | C37 H34 F N5 O7 S | 711.21630 | -7.5 | -0.8 | 9.3 | 16.5 |
| 2-2-d1E03-2-0095 | C41 H37 N5 O5 S | 711.25154 | 7.9 | -8.1 | 8.0 | -4.1 |
| 2-2-d1E03-2-0096 | C41 H37 N5 O5 S | 711.25154 | -107.7 | 4.9 | -12.2 | 22.3 |
| 2-2-d1E03-2-0097 | C41 H37 N5 O5 S | 711.25154 | 0.0 | 0.0 | 0.0 | 0.0 |
| 2-2-d1E03-2-0098 | C38 H41 N5 O5 S2 | 711.25491 | -11.3 | 5.8 | 8.0 | 16.2 |
| 2-2-d1E03-2-0099 | C37 H39 N5 O6 S2 | 713.23418 | 1.4 | 14.0 | 12.1 | 6.1 |
| 2-2-d1E03-2-0100 | C36 H34 N4 O8 S2 | 714.18181 | -7.9 | 0.1 | 2.5 | 9.4 |
| 2-2-d1E03-2-0101 | C36 H35 F N6 O5 S2 | 714.20944 | -6.2 | 7.4 | 12.5 | 14.2 |
| 2-2-d1E03-2-0102 | C41 H41 N5 O5 S | 715.28284 | -39.8 | -23.7 | 0.0 | 0.0 |
| 2-2-d1E03-2-0103 | C40 H36 N4 O7 S | 716.23047 | -1.0 | -2.1 | 20.2 | 7.0 |
| 2-2-d1E03-2-0104 | C40 H40 N6 O5 S | 716.27809 | 4.7 | -0.7 | -20.0 | 3.3 |
| 2-2-d1E03-2-0105 | C42 H44 N4 O5 S | 716.30324 | -8.0 | 1.2 | 3.1 | 6.4 |
| 2-2-d1E03-2-0106 | C39 H35 N5 O5 S2 | 717.20796 | 28.3 | 35.8 | 8.8 | 56.8 |
| 2-2-d1E03-2-0107 | C39 H35 N5 O5 S2 | 717.20796 | -0.3 | -7.2 | 16.6 | -48.8 |
| 2-2-d1E03-2-0108 | C39 H39 N7 O5 S | 717.27334 | 2.1 | -0.2 | 12.7 | 22.9 |
| 2-2-d1E03-2-0109 | C38 H34 N6 O5 S2 | 718.20321 | -75.6 | 19.2 | 13.6 | 14.3 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0110 | C37 H33 F3 N4 O6 S | 718.20729 | -23.8 | 35.0 | 16.6 | 13.7 |
| 2-2-d1E03-2-0111 | C37 H42 N4 O7 S2 | 718.24949 | -5.4 | -8.7 | 0.4 | 2.4 |
| 2-2-d1E03-2-0112 | C39 H38 N6 O6 S | 718.25735 | -11.2 | -0.8 | 0.0 | 0.0 |
| 2-2-d1E03-2-0113 | C41 H42 N4 O6 S | 718.28251 | -3.4 | 1.1 | 0.0 | 0.0 |
| 2-2-d1E03-2-0114 | C36 H40 N4 O8 S2 | 720.22876 | -6.5 | -6.7 | 5.3 | -0.8 |
| 2-2-d1E03-2-0115 | C39 H40 N6 O6 S | 720.27300 | 14.5 | 2.3 | 10.1 | 5.2 |
| 2-2-d1E03-2-0116 | C41 H41 F N4 O5 S | 720.27817 | 2.0 | -158.2 | -1.1 | 7.3 |
| 2-2-d1E03-2-0117 | C38 H35 F N6 O6 S | 722.23228 | -1.2 | -10.6 | 1.3 | 7.9 |
| 2-2-d1E03-2-0118 | C38 H38 N6 O5 S2 | 722.23451 | 2.0 | -19.3 | 30.6 | 0.0 |
| 2-2-d1E03-2-0119 | C40 H42 N4 O5 S2 | 722.25966 | 30.9 | -38.3 | 5.9 | 13.3 |
| 2-2-d1E03-2-0120 | C38 H41 F3 N4 O5 S | 722.27498 | -26.0 | 34.2 | 16.9 | 12.6 |
| 2-2-d1E03-2-0121 | C37 H33 N5 O5 S3 | 723.16438 | 12.3 | 26.1 | 2.5 | 16.8 |
| 2-2-d1E03-2-0122 | C38 H37 N5 O6 S2 | 723.21853 | 19.8 | 7.9 | 29.1 | 12.5 |
| 2-2-d1E03-2-0123 | C36 H32 N6 O7 S2 | 724.17739 | 0.0 | 0.0 | 0.0 | 0.0 |
| 2-2-d1E03-2-0124 | C38 H36 N4 O7 S2 | 724.20254 | -40.6 | 10.3 | 17.3 | 8.6 |
| 2-2-d1E03-2-0125 | C38 H36 N4 O7 S2 | 724.20254 | -1.2 | -16.1 | 15.4 | 10.1 |
| 2-2-d1E03-2-0126 | C38 H37 F N6 O6 S | 724.24793 | -10.7 | 15.3 | 8.2 | 10.9 |
| 2-2-d1E03-2-0127 | C37 H39 F3 N4 O6 S | 724.25424 | -34.6 | -13.0 | 14.1 | 4.8 |
| 2-2-d1E03-2-0128 | C43 H40 N4 O5 S | 724.27194 | 0.0 | -99.1 | 0.0 | 0.0 |
| 2-2-d1E03-2-0129 | C43 H40 N4 O5 S | 724.27194 | 80.8 | 16.8 | 32.6 | 9.8 |
| 2-2-d1E03-2-0130 | C37 H35 N5 O7 S2 | 725.19779 | -5.4 | -2.7 | 7.5 | 2.2 |
| 2-2-d1E03-2-0131 | C42 H39 N5 O5 S | 725.26719 | -16.8 | 9.3 | 27.5 | 15.2 |
| 2-2-d1E03-2-0132 | C42 H39 N5 O5 S | 725.26719 | -19.7 | -23.9 | 0.3 | -11.2 |
| 2-2-d1E03-2-0133 | C42 H39 N5 O5 S | 725.26719 | 12.1 | -18.6 | -2.4 | 6.6 |
| 2-2-d1E03-2-0134 | C39 H39 F N4 O5 S2 | 726.23459 | 22.5 | 40.9 | 6.4 | 28.3 |
| 2-2-d1E03-2-0135 | C37 H34 F N5 O6 S2 | 727.19345 | -16.1 | 43.8 | -7.8 | 14.5 |
| 2-2-d1E03-2-0136 | C37 H36 N4 O8 S2 | 728.19746 | -3.0 | 0.3 | 8.6 | 11.2 |
| 2-2-d1E03-2-0137 | C39 H35 F3 N4 O5 S | 728.22803 | 19.7 | -37.5 | -6.6 | -14.0 |
| 2-2-d1E03-2-0138 | C39 H35 F3 N4 O5 S | 728.22803 | 0.0 | 0.0 | 0.0 | 0.0 |
| 2-2-d1E03-2-0139 | C42 H37 F N4 O5 S | 728.24687 | 17.2 | 7.7 | 0.0 | 0.0 |
| 2-2-d1E03-2-0140 | C42 H37 F N4 O5 S | 728.24687 | -29.2 | -17.0 | -17.6 | 10.0 |
| 2-2-d1E03-2-0141 | C43 H44 N4 O5 S | 728.30324 | 0.0 | 0.0 | 0.0 | -19.7 |
| 2-2-d1E03-2-0142 | C38 H34 F3 N5 O5 S | 729.22327 | 15.7 | -1.5 | 22.9 | 7.5 |
| 2-2-d1E03-2-0143 | C41 H36 F N5 O5 S | 729.24212 | -5.4 | 6.7 | 16.0 | 11.7 |
| 2-2-d1E03-2-0144 | C41 H36 F N5 O5 S | 729.24212 | -45.9 | -17.6 | -6.6 | 37.6 |
| 2-2-d1E03-2-0145 | C41 H36 F N5 O5 S | 729.24212 | 27.2 | -41.3 | 17.8 | 29.4 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0146 | C42 H43 N5 O5 S | 729.29849 | -30.8 | 24.6 | 22.2 | 2.7 |
| 2-2-d1E03-2-0147 | C36 H34 N4 O7 S3 | 730.15896 | -18.8 | 7.4 | 10.7 | 6.7 |
| 2-2-d1E03-2-0148 | C41 H38 N4 O7 S | 730.24612 | -21.8 | 26.8 | 22.0 | 19.7 |
| 2-2-d1E03-2-0149 | C41 H42 N6 O5 S | 730.29374 | -0.7 | 7.4 | 26.1 | 0.0 |
| 2-2-d1E03-2-0150 | C35 H33 N5 O9 S2 | 731.17197 | -2.4 | -6.2 | 6.2 | 3.6 |
| 2-2-d1E03-2-0151 | C40 H41 N7 O5 S | 731.28899 | -1.2 | 6.5 | 8.7 | 6.1 |
| 2-2-d1E03-2-0152 | C36 H33 F N4 O8 S2 | 732.17238 | -5.5 | 3.7 | 4.8 | 4.0 |
| 2-2-d1E03-2-0153 | C38 H44 N4 O7 S2 | 732.26514 | -5.5 | -1.7 | 10.9 | -1.3 |
| 2-2-d1E03-2-0154 | C42 H44 N4 O6 S | 732.29816 | -23.4 | 28.2 | 7.6 | 1.7 |
| 2-2-d1E03-2-0155 | C39 H35 N5 O6 S2 | 733.20288 | -1.0 | -1.5 | 11.9 | 15.1 |
| 2-2-d1E03-2-0156 | C41 H40 F N5 O5 S | 733.27342 | 0.0 | 0.0 | 20.4 | -30.6 |
| 2-2-d1E03-2-0157 | C37 H33 F3 N4 O5 S2 | 734.18445 | -49.4 | 11.8 | 10.4 | -11.3 |
| 2-2-d1E03-2-0158 | C40 H35 F N4 O7 S | 734.22105 | -22.9 | -27.9 | 16.3 | -1.6 |
| 2-2-d1E03-2-0159 | C37 H42 N4 O8 S2 | 734.24441 | -8.5 | -11.0 | 7.0 | 5.9 |
| 2-2-d1E03-2-0160 | C41 H42 N4 O5 S2 | 734.25966 | 34.7 | 13.1 | -1.6 | 25.0 |
| 2-2-d1E03-2-0161 | C40 H39 F N6 O5 S | 734.26867 | -14.6 | 0.0 | 0.0 | -7.9 |
| 2-2-d1E03-2-0162 | C37 H33 N7 O6 S2 | 735.19337 | 15.9 | -4.6 | -2.7 | 23.8 |
| 2-2-d1E03-2-0163 | C36 H32 F3 N5 O7 S | 735.19745 | 3.9 | -5.3 | 38.6 | 7.4 |
| 2-2-d1E03-2-0164 | C39 H38 F N7 O5 S | 735.26392 | -16.3 | 11.9 | 14.4 | 13.4 |
| 2-2-d1E03-2-0165 | C37 H41 F N4 O7 S2 | 736.24007 | -118.0 | -4.0 | 10.2 | -1.8 |
| 2-2-d1E03-2-0166 | C39 H40 N6 O5 S2 | 736.25016 | -20.3 | 15.2 | -3.0 | 0.0 |
| 2-2-d1E03-2-0167 | C41 H41 F N4 O6 S | 736.27308 | -43.5 | 32.7 | 14.4 | 14.5 |
| 2-2-d1E03-2-0168 | C37 H35 N7 O6 S2 | 737.20902 | -6.7 | 15.1 | 8.6 | 1.9 |
| 2-2-d1E03-2-0169 | C40 H43 N5 O5 S2 | 737.27056 | 13.6 | 4.2 | 12.3 | 2.5 |
| 2-2-d1E03-2-0170 | C39 H38 N4 O7 S2 | 738.21819 | -4.9 | 4.1 | 16.5 | 3.2 |
| 2-2-d1E03-2-0171 | C39 H38 N4 O7 S2 | 738.21819 | -0.5 | 2.7 | 3.5 | 6.6 |
| 2-2-d1E03-2-0172 | C36 H39 F N4 O8 S2 | 738.21933 | -1.3 | -9.8 | 5.1 | 0.8 |
| 2-2-d1E03-2-0173 | C38 H37 N5 O7 S2 | 739.21344 | -5.5 | 4.4 | 7.0 | 4.6 |
| 2-2-d1E03-2-0174 | C39 H41 N5 O6 S2 | 739.24983 | -1.2 | 27.0 | 19.1 | 9.2 |
| 2-2-d1E03-2-0175 | C36 H32 N6 O6 S3 | 740.15454 | 38.0 | 37.2 | 5.3 | 21.1 |
| 2-2-d1E03-2-0176 | C38 H36 N4 O8 S2 | 740.19746 | 3.8 | -20.1 | 1.7 | 0.2 |
| 2-2-d1E03-2-0177 | C38 H37 F N6 O5 S2 | 740.22509 | 1.3 | -2.1 | 7.2 | 1.2 |
| 2-2-d1E03-2-0178 | C41 H35 N5 O5 S2 | 741.20796 | -31.3 | -1.7 | 6.9 | -2.0 |
| 2-2-d1E03-2-0179 | C41 H35 N5 O5 S2 | 741.20796 | 0.0 | 0.0 | 0.0 | 0.0 |
| 2-2-d1E03-2-0180 | C36 H34 N6 O8 S2 | 742.18795 | 1.0 | 7.6 | 8.0 | 1.5 |
| 2-2-d1E03-2-0181 | C38 H35 F N4 O7 S2 | 742.19312 | -0.5 | -0.2 | 13.5 | 10.7 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0182 | C38 H35 F N4 O7 S2 | 742.19312 | -8.8 | 4.0 | 13.5 | 9.8 |
| 2-2-d1E03-2-0183 | C40 H34 N6 O5 S2 | 742.20321 | -48.0 | 21.9 | 14.6 | 10.4 |
| 2-2-d1E03-2-0184 | C40 H34 N6 O5 S2 | 742.20321 | -80.8 | 6.6 | 13.0 | 11.0 |
| 2-2-d1E03-2-0185 | C40 H34 N6 O5 S2 | 742.20321 | 23.5 | -16.9 | 16.8 | 24.5 |
| 2-2-d1E03-2-0186 | C42 H38 N4 O7 S | 742.24612 | 1.6 | -11.5 | -1.0 | 25.2 |
| 2-2-d1E03-2-0187 | C44 H46 N4 O5 S | 742.31889 | 0.0 | 0.0 | 5.2 | 11.6 |
| 2-2-d1E03-2-0188 | C37 H34 F N5 O7 S2 | 743.18837 | -3.5 | -5.1 | 8.0 | 4.6 |
| 2-2-d1E03-2-0189 | C41 H37 N5 O5 S2 | 743.22361 | -4.9 | 19.8 | 22.4 | -14.3 |
| 2-2-d1E03-2-0190 | C41 H37 N5 O5 S2 | 743.22361 | 0.0 | 0.0 | 0.0 | 0.0 |
| 2-2-d1E03-2-0191 | C37 H36 N4 O7 S3 | 744.17461 | -3.7 | 8.7 | 18.6 | 3.4 |
| 2-2-d1E03-2-0192 | C36 H36 N6 O8 S2 | 744.20360 | -5.1 | 0.9 | 8.4 | 5.3 |
| 2-2-d1E03-2-0193 | C40 H36 N6 O5 S2 | 744.21886 | -16.3 | 9.7 | 5.5 | 5.7 |
| 2-2-d1E03-2-0194 | C39 H35 F3 N4 O6 S | 744.22294 | 5.2 | -173.2 | 27.4 | 21.3 |
| 2-2-d1E03-2-0195 | C39 H44 N4 O7 S2 | 744.26514 | 27.9 | -9.7 | 5.9 | 5.0 |
| 2-2-d1E03-2-0196 | C42 H44 N6 O5 S | 744.30939 | 11.7 | -7.8 | 9.2 | 10.7 |
| 2-2-d1E03-2-0197 | C36 H35 N5 O9 S2 | 745.18762 | 0.0 | -97.6 | 12.7 | 3.0 |
| 2-2-d1E03-2-0198 | C37 H33 F3 N6 O6 S | 746.21344 | -22.3 | -0.9 | 4.6 | 11.8 |
| 2-2-d1E03-2-0199 | C40 H38 N6 O5 S2 | 746.23451 | -17.3 | 41.3 | 19.1 | 16.1 |
| 2-2-d1E03-2-0200 | C38 H42 N4 O8 S2 | 746.24441 | -0.2 | -19.7 | 7.1 | 1.5 |
| 2-2-d1E03-2-0201 | C43 H43 F N4 O5 S | 746.29382 | -65.5 | -129.0 | 7.0 | 34.5 |
| 2-2-d1E03-2-0202 | C35 H33 N5 O8 S3 | 747.14913 | -8.7 | -7.4 | 6.1 | 35.1 |
| 2-2-d1E03-2-0203 | C39 H37 N7 O5 S2 | 747.22976 | 3.0 | -60.8 | 5.2 | 20.4 |
| 2-2-d1E03-2-0204 | C36 H33 F N4 O7 S3 | 748.14954 | -4.8 | 24.3 | 12.5 | 11.4 |
| 2-2-d1E03-2-0205 | C40 H36 N4 O7 S2 | 748.20254 | 3.2 | -5.4 | 15.3 | 7.8 |
| 2-2-d1E03-2-0206 | C40 H36 N4 O7 S2 | 748.20254 | 22.9 | -24.9 | 15.1 | 7.8 |
| 2-2-d1E03-2-0207 | C38 H36 N8 O5 S2 | 748.22501 | -15.8 | 18.5 | 14.3 | 8.5 |
| 2-2-d1E03-2-0208 | C37 H35 F3 N6 O6 S | 748.22909 | 18.2 | 29.8 | 5.3 | 18.9 |
| 2-2-d1E03-2-0209 | C42 H44 N4 O5 S2 | 748.27531 | 0.0 | 0.0 | 0.0 | 0.0 |
| 2-2-d1E03-2-0210 | C40 H43 F3 N4 O5 S | 748.29063 | 15.6 | -28.5 | -18.2 | 34.6 |
| 2-2-d1E03-2-0211 | C35 H32 F N5 O9 S2 | 749.16255 | -8.4 | 32.9 | 4.4 | -3.4 |
| 2-2-d1E03-2-0212 | C39 H35 N5 O5 S3 | 749.18003 | -46.4 | 6.0 | 16.4 | 11.9 |
| 2-2-d1E03-2-0213 | C39 H35 N5 O7 S2 | 749.19779 | -2.5 | 7.5 | 10.0 | 6.7 |
| 2-2-d1E03-2-0214 | C39 H35 N5 O7 S2 | 749.19779 | -3.2 | 23.4 | 14.0 | 12.1 |
| 2-2-d1E03-2-0215 | C39 H35 N5 O7 S2 | 749.19779 | 0.0 | 0.0 | 0.0 | 0.0 |
| 2-2-d1E03-2-0216 | C40 H39 N5 O6 S2 | 749.23418 | -52.4 | 0.0 | 11.5 | 12.3 |
| 2-2-d1E03-2-0217 | C38 H34 N6 O7 S2 | 750.19304 | 0.0 | -20.2 | 0.0 | 0.0 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0218 | C40 H38 N4 O7 S2 | 750.21819 | 0.0 | 0.0 | 0.0 | 0.0 |
| 2-2-d1E03-2-0219 | C40 H38 N4 O7 S2 | 750.21819 | -0.3 | -2.7 | -7.9 | -8.6 |
| 2-2-d1E03-2-0220 | C39 H41 F3 N4 O6 S | 750.26989 | -37.5 | 15.9 | 26.2 | 14.2 |
| 2-2-d1E03-2-0221 | C36 H32 F3 N5 O6 S2 | 751.17461 | -60.9 | 17.3 | -11.7 | -29.5 |
| 2-2-d1E03-2-0222 | C39 H37 N5 O7 S2 | 751.21344 | -13.2 | -4.1 | 5.6 | 3.6 |
| 2-2-d1E03-2-0223 | C41 H35 F3 N4 O5 S | 752.22803 | 0.0 | 1.4 | 1.8 | -28.9 |
| 2-2-d1E03-2-0224 | C41 H35 F3 N4 O5 S | 752.22803 | 0.0 | 0.0 | 0.0 | 0.0 |
| 2-2-d1E03-2-0225 | C41 H41 F N4 O5 S2 | 752.25024 | -4.5 | 10.0 | 75.2 | 80.0 |
| 2-2-d1E03-2-0226 | C37 H35 N7 O5 S3 | 753.18618 | 7.6 | 3.3 | 10.0 | 8.5 |
| 2-2-d1E03-2-0227 | C40 H34 F3 N5 O5 S | 753.22327 | 2.0 | 21.8 | 17.4 | 12.7 |
| 2-2-d1E03-2-0228 | C40 H34 F3 N5 O5 S | 753.22327 | -9.7 | -18.7 | 13.0 | 30.5 |
| 2-2-d1E03-2-0229 | C40 H34 F3 N5 O5 S | 753.22327 | 0.0 | 0.0 | 0.0 | 0.0 |
| 2-2-d1E03-2-0230 | C39 H39 N5 O7 S2 | 753.22909 | -25.1 | 3.5 | 11.1 | -3.1 |
| 2-2-d1E03-2-0231 | C39 H38 N4 O8 S2 | 754.21311 | 0.0 | 18.1 | 0.0 | 7.7 |
| 2-2-d1E03-2-0232 | C38 H38 N6 O7 S2 | 754.22434 | -9.3 | -6.9 | 11.0 | 4.1 |
| 2-2-d1E03-2-0233 | C41 H37 F3 N4 O5 S | 754.24368 | -33.5 | -19.2 | 25.6 | 3.6 |
| 2-2-d1E03-2-0234 | C41 H37 F3 N4 O5 S | 754.24368 | 0.0 | 0.0 | 0.0 | 0.0 |
| 2-2-d1E03-2-0235 | C37 H37 N7 O7 S2 | 755.21959 | -25.7 | 20.8 | 11.2 | 5.4 |
| 2-2-d1E03-2-0236 | C40 H36 F3 N5 O5 S | 755.23892 | 11.6 | 29.8 | 9.6 | 31.7 |
| 2-2-d1E03-2-0237 | C38 H36 N4 O7 S3 | 756.17461 | 0.0 | 0.0 | 0.0 | 0.0 |
| 2-2-d1E03-2-0238 | C37 H36 N6 O8 S2 | 756.20360 | 52.3 | -17.7 | -2.2 | 5.0 |
| 2-2-d1E03-2-0239 | C39 H40 N4 O8 S2 | 756.22876 | -10.6 | -14.3 | 5.4 | 7.2 |
| 2-2-d1E03-2-0240 | C43 H40 N4 O7 S | 756.26177 | 0.0 | 0.0 | 0.0 | 0.0 |
| 2-2-d1E03-2-0241 | C37 H35 N5 O9 S2 | 757.18762 | 0.0 | 0.0 | 0.0 | 0.0 |
| 2-2-d1E03-2-0242 | C40 H38 F3 N5 O5 S | 757.25457 | -0.3 | 10.6 | 12.1 | 22.2 |
| 2-2-d1E03-2-0243 | C38 H35 F N4 O8 S2 | 758.18803 | 5.2 | 5.9 | 1.9 | 7.1 |
| 2-2-d1E03-2-0244 | C37 H38 N6 O8 S2 | 758.21925 | -14.1 | 0.7 | 12.0 | 2.3 |
| 2-2-d1E03-2-0245 | C39 H37 F3 N6 O5 S | 758.24982 | -3.7 | -4.7 | 10.4 | 5.9 |
| 2-2-d1E03-2-0246 | C40 H46 N4 O7 S2 | 758.28079 | -13.5 | -11.0 | 1.4 | -0.4 |
| 2-2-d1E03-2-0247 | C43 H46 N6 O5 S | 758.32504 | 6.2 | 25.8 | 3.6 | 2.4 |
| 2-2-d1E03-2-0248 | C38 H36 F3 N7 O5 S | 759.24507 | -19.5 | 19.7 | 24.3 | 14.2 |
| 2-2-d1E03-2-0249 | C42 H41 N5 O5 S2 | 759.25491 | -3.2 | -31.8 | 27.3 | 26.4 |
| 2-2-d1E03-2-0250 | C36 H33 F N6 O8 S2 | 760.17853 | 1.6 | 0.3 | 4.8 | 7.6 |
| 2-2-d1E03-2-0251 | C36 H36 N6 O7 S3 | 760.18076 | -6.6 | -3.6 | 9.3 | 7.2 |
| 2-2-d1E03-2-0252 | C39 H35 F3 N4 O5 S2 | 760.20010 | -4.9 | -1.6 | 6.4 | 0.0 |
| 2-2-d1E03-2-0253 | C42 H37 F N4 O7 S | 760.23670 | 0.0 | 0.0 | 0.0 | 0.0 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0254 | C40 H39 F3 N4 O6 S | 760.25424 | -79.8 | 61.2 | 33.7 | 19.0 |
| 2-2-d1E03-2-0255 | C39 H44 N4 O8 S2 | 760.26006 | -1.3 | 17.1 | 4.3 | 1.8 |
| 2-2-d1E03-2-0256 | C36 H35 N5 O8 S3 | 761.16478 | 3.1 | 23.8 | 17.6 | -9.0 |
| 2-2-d1E03-2-0257 | C39 H35 N7 O6 S2 | 761.20902 | 0.5 | 29.2 | 24.1 | 14.4 |
| 2-2-d1E03-2-0258 | C38 H34 F3 N5 O7 S | 761.21310 | 0.0 | 0.0 | 0.0 | 0.0 |
| 2-2-d1E03-2-0259 | C36 H35 F N6 O8 S2 | 762.19418 | -9.1 | -4.8 | 10.8 | 4.3 |
| 2-2-d1E03-2-0260 | C41 H38 N4 O7 S2 | 762.21819 | -29.5 | 0.9 | 14.3 | 10.8 |
| 2-2-d1E03-2-0261 | C41 H38 N4 O7 S2 | 762.21819 | 0.0 | -89.3 | 26.7 | -29.1 |
| 2-2-d1E03-2-0262 | C39 H43 F N4 O7 S2 | 762.25572 | -59.7 | -43.8 | 0.1 | -2.1 |
| 2-2-d1E03-2-0263 | C42 H43 F N6 O5 S | 762.29997 | 11.3 | 33.9 | 8.7 | 20.0 |
| 2-2-d1E03-2-0264 | C40 H37 N5 O7 S2 | 763.21344 | -21.1 | 9.6 | 17.8 | 11.1 |
| 2-2-d1E03-2-0265 | C40 H37 N5 O7 S2 | 763.21344 | 0.1 | 18.2 | 25.4 | 31.1 |
| 2-2-d1E03-2-0266 | C40 H37 N5 O7 S2 | 763.21344 | -21.1 | 9.6 | 17.8 | 11.1 |
| 2-2-d1E03-2-0267 | C39 H37 N7 O6 S2 | 763.22467 | 12.4 | 6.0 | 12.7 | 8.1 |
| 2-2-d1E03-2-0268 | C37 H35 F3 N6 O5 S2 | 764.20624 | -11.1 | 26.4 | 13.1 | 17.1 |
| 2-2-d1E03-2-0269 | C41 H40 N4 O7 S2 | 764.23384 | 0.0 | 0.0 | 0.0 | 0.0 |
| 2-2-d1E03-2-0270 | C41 H40 N4 O7 S2 | 764.23384 | 15.2 | 8.6 | 0.0 | 0.0 |
| 2-2-d1E03-2-0271 | C38 H41 F N4 O8 S2 | 764.23498 | -1.8 | 3.6 | 5.7 | 4.4 |
| 2-2-d1E03-2-0272 | C35 H32 F N5 O8 S3 | 765.13970 | 11.2 | -3.0 | 0.6 | 15.8 |
| 2-2-d1E03-2-0273 | C40 H39 N5 O5 S3 | 765.21133 | 11.2 | 11.9 | 9.2 | 15.1 |
| 2-2-d1E03-2-0274 | C40 H39 N5 O7 S2 | 765.22909 | -3.7 | -16.1 | 12.9 | 5.8 |
| 2-2-d1E03-2-0275 | C38 H34 N6 O6 S3 | 766.17019 | -1.8 | 19.9 | 26.1 | 1.8 |
| 2-2-d1E03-2-0276 | C40 H35 F N4 O7 S2 | 766.19312 | -3.8 | 7.7 | 12.2 | 7.6 |
| 2-2-d1E03-2-0277 | C40 H35 F N4 O7 S2 | 766.19312 | -20.7 | 3.9 | 22.8 | -30.6 |
| 2-2-d1E03-2-0278 | C41 H42 N4 O7 S2 | 766.24949 | -7.4 | 17.8 | 4.7 | -2.9 |
| 2-2-d1E03-2-0279 | C39 H34 F N5 O7 S2 | 767.18837 | 5.8 | 2.1 | 10.0 | 4.5 |
| 2-2-d1E03-2-0280 | C39 H34 F N5 O7 S2 | 767.18837 | 1.1 | 25.0 | 13.5 | 18.6 |
| 2-2-d1E03-2-0281 | C39 H34 F N5 O7 S2 | 767.18837 | 0.0 | 0.0 | 0.0 | 0.0 |
| 2-2-d1E03-2-0282 | C43 H37 N5 O5 S2 | 767.22361 | -301.2 | -36.9 | 11.8 | 18.1 |
| 2-2-d1E03-2-0283 | C43 H37 N5 O5 S2 | 767.22361 | 0.0 | 0.0 | 0.0 | 0.0 |
| 2-2-d1E03-2-0284 | C40 H41 N5 O7 S2 | 767.24474 | -28.4 | 18.6 | 18.2 | -8.5 |
| 2-2-d1E03-2-0285 | C38 H36 N6 O8 S2 | 768.20360 | -1.9 | 4.2 | 23.7 | 24.2 |
| 2-2-d1E03-2-0286 | C40 H37 F N4 O7 S2 | 768.20877 | 0.0 | 0.0 | 6.7 | -5.3 |
| 2-2-d1E03-2-0287 | C40 H37 F N4 O7 S2 | 768.20877 | -9.3 | -5.6 | 12.5 | 3.6 |
| 2-2-d1E03-2-0288 | C42 H36 N6 O5 S2 | 768.21886 | -11.7 | 5.3 | 5.2 | 19.4 |
| 2-2-d1E03-2-0289 | C42 H36 N6 O5 S2 | 768.21886 | 11.5 | -15.0 | 8.6 | 9.2 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0290 | C42 H36 N6 O5 S2 | 768.21886 | -42.7 | 1.2 | -6.6 | -7.7 |
| 2-2-d1E03-2-0291 | C39 H40 N6 O7 S2 | 768.23999 | -1.0 | 7.1 | 11.1 | 18.6 |
| 2-2-d1E03-2-0292 | C39 H36 F N5 O7 S2 | 769.20402 | -23.1 | 8.4 | 5.6 | 1.9 |
| 2-2-d1E03-2-0293 | C38 H39 N7 O7 S2 | 769.23524 | 11.3 | 25.3 | 18.2 | 14.9 |
| 2-2-d1E03-2-0294 | C39 H38 N4 O7 S3 | 770.19026 | 2.0 | 1.2 | 16.0 | 11.7 |
| 2-2-d1E03-2-0295 | C38 H38 N6 O8 S2 | 770.21925 | 4.0 | -10.8 | 8.6 | 21.9 |
| 2-2-d1E03-2-0296 | C40 H42 N4 O8 S2 | 770.24441 | -14.4 | -7.5 | 14.1 | 1.7 |
| 2-2-d1E03-2-0297 | C42 H41 F3 N4 O5 S | 770.27498 | 12.0 | -5.9 | 26.1 | -6.5 |
| 2-2-d1E03-2-0298 | C44 H46 N6 O5 S | 770.32504 | -30.9 | 14.3 | 21.3 | 10.4 |
| 2-2-d1E03-2-0299 | C37 H33 N5 O8 S3 | 771.14913 | -15.8 | -0.4 | 4.8 | 4.4 |
| 2-2-d1E03-2-0300 | C38 H37 N5 O9 S2 | 771.20327 | -5.8 | -4.0 | -0.4 | -3.3 |
| 2-2-d1E03-2-0301 | C39 H38 F N5 O7 S2 | 771.21967 | -4.3 | -11.0 | 20.1 | 19.8 |
| 2-2-d1E03-2-0302 | C39 H40 N4 O7 S3 | 772.20591 | -0.2 | -19.6 | 6.0 | 2.1 |
| 2-2-d1E03-2-0303 | C38 H37 F N6 O7 S2 | 772.21492 | -17.0 | 3.9 | 4.7 | 6.7 |
| 2-2-d1E03-2-0304 | C39 H35 F3 N6 O6 S | 772.22909 | 9.8 | 12.6 | 0.0 | 0.0 |
| 2-2-d1E03-2-0305 | C42 H40 N6 O5 S2 | 772.25016 | 3.6 | -20.1 | 25.6 | -114.8 |
| 2-2-d1E03-2-0306 | C37 H35 N5 O8 S3 | 773.16478 | -51.8 | -9.2 | 26.3 | -3.6 |
| 2-2-d1E03-2-0307 | C41 H35 N5 O7 S2 | 773.19779 | 0.0 | 0.0 | -10.7 | -84.6 |
| 2-2-d1E03-2-0308 | C37 H36 F N7 O7 S2 | 773.21017 | -5.1 | 11.2 | 14.4 | 9.9 |
| 2-2-d1E03-2-0309 | C41 H39 N7 O5 S2 | 773.24541 | 5.0 | 8.9 | 9.6 | 12.8 |
| 2-2-d1E03-2-0310 | C38 H35 F N4 O7 S3 | 774.16519 | 7.7 | 26.1 | 0.0 | 0.0 |
| 2-2-d1E03-2-0311 | C37 H38 N6 O7 S3 | 774.19641 | 6.9 | 3.0 | 18.1 | 7.8 |
| 2-2-d1E03-2-0312 | C42 H38 N4 O7 S2 | 774.21819 | 5.5 | 3.9 | -90.6 | 8.9 |
| 2-2-d1E03-2-0313 | C42 H38 N4 O7 S2 | 774.21819 | 0.0 | 0.0 | 0.0 | 0.0 |
| 2-2-d1E03-2-0314 | C39 H39 F N4 O8 S2 | 774.21933 | -11.7 | -7.3 | 4.7 | -1.6 |
| 2-2-d1E03-2-0315 | C40 H38 N8 O5 S2 | 774.24066 | -6.9 | -46.3 | 4.8 | 8.4 |
| 2-2-d1E03-2-0316 | C39 H37 F3 N6 O6 S | 774.24474 | -44.1 | -24.0 | 3.6 | -5.2 |
| 2-2-d1E03-2-0317 | C37 H34 F N5 O9 S2 | 775.17820 | -9.5 | -5.7 | 14.2 | 8.8 |
| 2-2-d1E03-2-0318 | C41 H37 N5 O7 S2 | 775.21344 | 6.6 | 4.5 | 10.4 | 8.5 |
| 2-2-d1E03-2-0319 | C41 H37 N5 O7 S2 | 775.21344 | -15.8 | 1.2 | 12.0 | -4.7 |
| 2-2-d1E03-2-0320 | C41 H37 N5 O7 S2 | 775.21344 | -39.4 | 39.8 | 32.7 | 26.1 |
| 2-2-d1E03-2-0321 | C38 H41 N5 O7 S3 | 775.21681 | -2.2 | 5.4 | 2.4 | 3.2 |
| 2-2-d1E03-2-0322 | C42 H41 N5 O6 S2 | 775.24983 | -68.0 | 59.3 | 9.8 | 13.3 |
| 2-2-d1E03-2-0323 | C40 H39 F3 N4 O5 S2 | 776.23140 | -159.5 | 21.0 | 31.1 | 10.0 |
| 2-2-d1E03-2-0324 | C38 H34 F3 N5 O6 S2 | 777.19026 | 4.8 | 8.7 | 15.0 | 12.9 |
| 2-2-d1E03-2-0325 | C37 H39 N5 O8 S3 | 777.19608 | 0.0 | 0.0 | 10.2 | 2.2 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0326 | C36 H35 F N6 O7 S3 | 778.17134 | -9.1 | -5.2 | 14.6 | 7.4 |
| 2-2-d1E03-2-0327 | C43 H37 F3 N4 O5 S | 778.24368 | -86.0 | 0.0 | 7.1 | 40.4 |
| 2-2-d1E03-2-0328 | C43 H37 F3 N4 O5 S | 778.24368 | 1.5 | 21.7 | 3.6 | 20.4 |
| 2-2-d1E03-2-0329 | C39 H37 N7 O5 S3 | 779.20183 | 17.0 | -35.3 | 7.9 | 14.8 |
| 2-2-d1E03-2-0330 | C42 H36 F3 N5 O5 S | 779.23892 | -70.5 | -3.9 | -6.2 | 1.4 |
| 2-2-d1E03-2-0331 | C42 H36 F3 N5 O5 S | 779.23892 | 77.0 | -218.1 | 12.8 | 41.9 |
| 2-2-d1E03-2-0332 | C42 H36 F3 N5 O5 S | 779.23892 | 0.0 | 0.0 | 0.0 | 0.0 |
| 2-2-d1E03-2-0333 | C41 H41 N5 O7 S2 | 779.24474 | 0.0 | 0.0 | 0.0 | 0.0 |
| 2-2-d1E03-2-0334 | C40 H36 N4 O9 S2 | 780.19237 | -3.1 | -10.4 | 15.8 | 7.8 |
| 2-2-d1E03-2-0335 | C40 H40 N6 O7 S2 | 780.23999 | 0.0 | 0.0 | 0.0 | 0.0 |
| 2-2-d1E03-2-0336 | C42 H44 N4 O7 S2 | 780.26514 | 5.5 | 8.4 | 11.7 | 19.4 |
| 2-2-d1E03-2-0337 | C39 H35 N5 O7 S3 | 781.16986 | -1.2 | 26.9 | 8.0 | 4.1 |
| 2-2-d1E03-2-0338 | C39 H35 N5 O7 S3 | 781.16986 | 7.3 | 10.1 | 0.0 | 2.8 |
| 2-2-d1E03-2-0339 | C39 H39 N7 O7 S2 | 781.23524 | 15.2 | -0.3 | 5.1 | 9.1 |
| 2-2-d1E03-2-0340 | C38 H34 N6 O7 S3 | 782.16511 | -12.5 | -7.9 | 7.7 | 7.7 |
| 2-2-d1E03-2-0341 | C37 H33 F3 N4 O8 S2 | 782.16919 | -5.3 | -12.3 | 6.6 | 6.8 |
| 2-2-d1E03-2-0342 | C39 H38 N6 O8 S2 | 782.21925 | 1.3 | 39.3 | 0.0 | 13.9 |
| 2-2-d1E03-2-0343 | C41 H42 N4 O8 S2 | 782.24441 | 1.2 | 0.0 | 10.7 | 3.1 |
| 2-2-d1E03-2-0344 | C42 H40 F3 N5 O5 S | 783.27022 | -5.9 | 5.7 | -0.7 | -0.6 |
| 2-2-d1E03-2-0345 | C41 H35 F3 N4 O7 S | 784.21785 | 70.6 | 2.7 | 29.2 | -5.7 |
| 2-2-d1E03-2-0346 | C39 H40 N6 O8 S2 | 784.23490 | 0.0 | 0.0 | 0.0 | 0.0 |
| 2-2-d1E03-2-0347 | C41 H41 F N4 O7 S2 | 784.24007 | -10.9 | 14.5 | 7.2 | 7.8 |
| 2-2-d1E03-2-0348 | C41 H39 F3 N6 O5 S | 784.26547 | -403.9 | -7.3 | -19.8 | 3.1 |
| 2-2-d1E03-2-0349 | C45 H48 N6 O5 S | 784.34069 | 16.2 | 37.6 | 11.6 | 13.6 |
| 2-2-d1E03-2-0350 | C40 H38 F3 N7 O5 S | 785.26072 | 7.1 | 31.4 | 18.9 | -15.3 |
| 2-2-d1E03-2-0351 | C44 H43 N5 O5 S2 | 785.27056 | -55.0 | 5.5 | 12.5 | 21.8 |
| 2-2-d1E03-2-0352 | C38 H35 F N6 O8 S2 | 786.19418 | -93.3 | -48.3 | -61.4 | 0.0 |
| 2-2-d1E03-2-0353 | C38 H38 N6 O7 S3 | 786.19641 | 6.2 | -6.3 | 0.0 | 0.0 |
| 2-2-d1E03-2-0354 | C40 H42 N4 O7 S3 | 786.22156 | 0.5 | 3.2 | 10.8 | -4.3 |
| 2-2-d1E03-2-0355 | C38 H41 F3 N4 O7 S2 | 786.23688 | -27.2 | 5.8 | 6.4 | -1.1 |
| 2-2-d1E03-2-0356 | C42 H41 F3 N4 O6 S | 786.26989 | -5.8 | 4.0 | -10.3 | -6.0 |
| 2-2-d1E03-2-0357 | C37 H33 N5 O7 S4 | 787.12628 | 5.9 | 33.7 | 5.1 | 8.8 |
| 2-2-d1E03-2-0358 | C38 H37 N5 O8 S3 | 787.18043 | -9.1 | 5.8 | 5.6 | 0.5 |
| 2-2-d1E03-2-0359 | C36 H32 N6 O9 S3 | 788.13929 | 9.7 | 0.0 | 0.0 | -9.8 |
| 2-2-d1E03-2-0360 | C38 H37 F N6 O8 S2 | 788.20983 | -4.8 | 11.2 | 17.9 | 8.8 |
| 2-2-d1E03-2-0361 | C37 H39 F3 N4 O8 S2 | 788.21614 | 8.0 | -12.7 | 11.4 | 5.2 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0362 | C43 H40 N4 O7 S2 | 788.23384 | 4.7 | 20.7 | 8.0 | 20.4 |
| 2-2-d1E03-2-0363 | C43 H40 N4 O7 S2 | 788.23384 | 10.3 | 7.0 | 30.8 | 15.3 |
| 2-2-d1E03-2-0364 | C44 H45 F N6 O5 S | 788.31562 | -29.2 | 26.7 | 19.7 | 16.1 |
| 2-2-d1E03-2-0365 | C42 H39 N5 O7 S2 | 789.22909 | 4.0 | 6.9 | 9.1 | 11.3 |
| 2-2-d1E03-2-0366 | C42 H39 N5 O7 S2 | 789.22909 | 1.9 | 1.1 | -2.1 | -17.4 |
| 2-2-d1E03-2-0367 | C42 H39 N5 O7 S2 | 789.22909 | 0.0 | 0.0 | 0.0 | 0.0 |
| 2-2-d1E03-2-0368 | C39 H39 F N4 O7 S3 | 790.19649 | -11.9 | 21.2 | 6.6 | 6.6 |
| 2-2-d1E03-2-0369 | C39 H37 F3 N6 O5 S2 | 790.22189 | 0.0 | 0.0 | 3.1 | 5.0 |
| 2-2-d1E03-2-0370 | C37 H34 F N5 O8 S3 | 791.15535 | -16.9 | 4.0 | 18.1 | 5.3 |
| 2-2-d1E03-2-0371 | C42 H41 N5 O5 S3 | 791.22698 | 0.0 | 0.0 | 18.6 | -16.5 |
| 2-2-d1E03-2-0372 | C39 H35 F3 N4 O7 S2 | 792.18993 | -33.7 | 1.6 | 14.7 | -2.2 |
| 2-2-d1E03-2-0373 | C39 H35 F3 N4 O7 S2 | 792.18993 | 4.5 | 3.5 | 0.0 | 0.0 |
| 2-2-d1E03-2-0374 | C42 H37 F N4 O7 S2 | 792.20877 | 0.0 | 0.0 | 2.0 | -0.7 |
| 2-2-d1E03-2-0375 | C42 H37 F N4 O7 S2 | 792.20877 | 10.4 | 26.6 | 10.3 | 25.0 |
| 2-2-d1E03-2-0376 | C43 H44 N4 O7 S2 | 792.26514 | 10.0 | 20.9 | 0.0 | 0.0 |
| 2-2-d1E03-2-0377 | C38 H34 F3 N5 O7 S2 | 793.18517 | -5.0 | 26.3 | 13.3 | 8.2 |
| 2-2-d1E03-2-0378 | C41 H36 FN5 O7 S2 | 793.20402 | -4.1 | -12.8 | 15.5 | -2.2 |
| 2-2-d1E03-2-0379 | C41 H36 FN5 O7 S2 | 793.20402 | -16.0 | 48.3 | 29.3 | 32.3 |
| 2-2-d1E03-2-0380 | C41 H36 F N5 O7 S2 | 793.20402 | 0.0 | 0.0 | 0.0 | 0.0 |
| 2-2-d1E03-2-0381 | C42 H43 N5 O7 S2 | 793.26039 | 21.5 | 63.7 | 38.8 | 23.9 |
| 2-2-d1E03-2-0382 | C41 H38 N4 O9 S2 | 794.20802 | -51.7 | -0.7 | 3.6 | 5.1 |
| 2-2-d1E03-2-0383 | C41 H42 N6 O7 S2 | 794.25564 | -6.4 | 6.5 | 0.0 | 0.0 |
| 2-2-d1E03-2-0384 | C40 H41 N7 O7 S2 | 795.25089 | -15.9 | -3.1 | 15.5 | -15.3 |
| 2-2-d1E03-2-0385 | C42 H44 N4 O8 S2 | 796.26006 | 3.2 | -11.5 | 0.0 | 8.2 |
| 2-2-d1E03-2-0386 | C44 H43 F3 N4 O5 S | 796.29063 | -175.7 | 9.1 | -13.4 | -10.7 |
| 2-2-d1E03-2-0387 | C39 H35 N5 O8 S3 | 797.16478 | -43.5 | 11.5 | 9.4 | 7.1 |
| 2-2-d1E03-2-0388 | C41 H40 F N5 O7 S2 | 797.23532 | 6.2 | 1.1 | -5.4 | 40.4 |
| 2-2-d1E03-2-0389 | C37 H33 F3 N4 O7 S3 | 798.14635 | -25.7 | 26.0 | 7.5 | 8.1 |
| 2-2-d1E03-2-0390 | C40 H35 F N4 O9 S2 | 798.18295 | -13.0 | -45.1 | 9.0 | 0.5 |
| 2-2-d1E03-2-0391 | C41 H42 N4 O7 S3 | 798.22156 | 0.0 | 0.0 | 0.0 | 0.0 |
| 2-2-d1E03-2-0392 | C40 H39 F N6 O7 S2 | 798.23057 | 13.6 | 14.0 | 9.9 | 12.7 |
| 2-2-d1E03-2-0393 | C37 H33 N7 O8 S3 | 799.15527 | 2.2 | -7.5 | 5.1 | 38.5 |
| 2-2-d1E03-2-0394 | C36 H32 F3 N5 O9 S2 | 799.15935 | -10.2 | 0.1 | 4.6 | 5.1 |
| 2-2-d1E03-2-0395 | C43 H37 N5 O7 S2 | 799.21344 | -2.9 | -11.4 | 0.0 | 0.0 |
| 2-2-d1E03-2-0396 | C39 H38 F N7 O7 S2 | 799.22582 | 12.7 | 9.5 | 17.5 | 19.2 |
| 2-2-d1E03-2-0397 | C39 H40 N6 O7 S3 | 800.21206 | 0.0 | 0.0 | 0.0 | 0.0 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0398 | C41 H41 FN4 O8 S2 | 800.23498 | -24.5 | -11.2 | 7.2 | 4.6 |
| 2-2-d1E03-2-0399 | C37 H35 N7 O8 S3 | 801.17092 | -2.4 | -2.4 | 13.0 | 2.7 |
| 2-2-d1E03-2-0400 | C40 H43 N5 O7 S3 | 801.23246 | -20.2 | 5.9 | 7.8 | 2.2 |
| 2-2-d1E03-2-0401 | C43 H43 N7 O5 S2 | 801.27671 | -2.0 | 3.3 | 13.8 | 19.6 |
| 2-2-d1E03-2-0402 | C42 H41 F3 N4 O5 S2 | 802.24705 | -97.6 | 0.0 | 70.3 | 74.5 |
| 2-2-d1E03-2-0403 | C39 H41 N5 O8 S3 | 803.21173 | -11.2 | 5.2 | 6.1 | 4.3 |
| 2-2-d1E03-2-0404 | C36 H32 N6 O8 S4 | 804.11644 | -16.7 | -0.4 | 16.6 | 1.6 |
| 2-2-d1E03-2-0405 | C38 H37 F N6 O7 S3 | 804.18699 | -19.2 | 17.0 | 13.8 | 17.5 |
| 2-2-d1E03-2-0406 | C41 H35 N5 O7 S3 | 805.16986 | -17.0 | 4.2 | 16.3 | 5.2 |
| 2-2-d1E03-2-0407 | C41 H35 N5 O7 S3 | 805.16986 | 0.0 | 0.0 | 0.0 | 0.0 |
| 2-2-d1E03-2-0408 | C40 H34 N6 O7 S3 | 806.16511 | 1.0 | 0.9 | 13.7 | 13.0 |
| 2-2-d1E03-2-0409 | C40 H34 N6 O7 S3 | 806.16511 | 27.0 | 7.0 | 5.9 | 8.7 |
| 2-2-d1E03-2-0410 | C40 H34 N6 O7 S3 | 806.16511 | 0.0 | -167.2 | -9.6 | 0.0 |
| 2-2-d1E03-2-0411 | C42 H38 N4 O9 S2 | 806.20802 | 11.9 | -40.2 | -3.6 | 7.8 |
| 2-2-d1E03-2-0412 | C44 H46 N4 O7 S2 | 806.28079 | -90.2 | 0.0 | -25.7 | 9.3 |
| 2-2-d1E03-2-0413 | C41 H37 N5 O7 S3 | 807.18551 | -2.0 | -1.0 | 0.5 | -0.2 |
| 2-2-d1E03-2-0414 | C41 H37 N5 O7 S3 | 807.18551 | -10.1 | -8.0 | 11.0 | -30.5 |
| 2-2-d1E03-2-0415 | C40 H36 N6 O7 S3 | 808.18076 | -7.2 | 64.5 | 13.0 | 8.8 |
| 2-2-d1E03-2-0416 | C39 H35 F3 N4 O8 S2 | 808.18484 | -5.8 | -6.0 | 7.2 | 1.5 |
| 2-2-d1E03-2-0417 | C42 H44 N6 O7 S2 | 808.27129 | 25.8 | 20.1 | 25.4 | 17.4 |
| 2-2-d1E03-2-0418 | C37 H33 F3 N6 O8 S2 | 810.17534 | -3.7 | -7.6 | 8.0 | 9.1 |
| 2-2-d1E03-2-0419 | C40 H38 N6 O7 S3 | 810.19641 | 2.4 | 9.5 | 12.4 | 5.7 |
| 2-2-d1E03-2-0420 | C43 H37 F3 N4 O7 S | 810.23350 | 0.0 | 0.0 | 4.2 | 0.0 |
| 2-2-d1E03-2-0421 | C43 H43 F N4 O7 S2 | 810.25572 | 38.0 | -127.7 | -5.6 | 7.6 |
| 2-2-d1E03-2-0422 | C39 H37 N7 O7 S3 | 811.19166 | -24.7 | 1.3 | -8.2 | 4.4 |
| 2-2-d1E03-2-0423 | C38 H36 N8 O7 S3 | 812.18691 | -74.3 | -4.2 | 24.6 | 24.1 |
| 2-2-d1E03-2-0424 | C37 H35 F3 N6 O8 S2 | 812.19099 | 5.4 | -1.6 | 12.8 | 5.6 |
| 2-2-d1E03-2-0425 | C42 H44 N4 O7 S3 | 812.23721 | -16.3 | 0.0 | 0.1 | 14.9 |
| 2-2-d1E03-2-0426 | C40 H43 F3 N4 O7 S2 | 812.25253 | -42.7 | 2.4 | -13.6 | -10.8 |
| 2-2-d1E03-2-0427 | C43 H43 F3 N6 O5 S | 812.29677 | -28.1 | 36.2 | 23.0 | 18.5 |
| 2-2-d1E03-2-0428 | C39 H35 N5 O7 S4 | 813.14193 | -24.8 | 8.3 | 17.1 | 7.5 |
| 2-2-d1E03-2-0429 | C40 H39 N5 O8 S3 | 813.19608 | -31.6 | 0.0 | 8.8 | 0.7 |
| 2-2-d1E03-2-0430 | C38 H34 N6 O9 S3 | 814.15494 | -18.6 | 2.4 | -15.9 | -1.8 |
| 2-2-d1E03-2-0431 | C39 H41 F3 N4 O8 S2 | 814.23179 | -21.4 | 13.2 | 14.9 | 5.5 |
| 2-2-d1E03-2-0432 | C36 H32 F3 N5 O8 S3 | 815.13651 | -0.6 | -4.5 | 21.5 | 19.4 |
| 2-2-d1E03-2-0433 | C41 H35 F3 N4 O7 S2 | 816.18993 | 8.8 | -3.8 | 3.0 | 10.9 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0434 | C41 H35 F3 N4 O7 S2 | 816.18993 | 1.2 | -42.1 | 13.0 | 20.1 |
| 2-2-d1E03-2-0435 | C41 H41 F N4 O7 S3 | 816.21214 | -9.6 | 5.4 | 6.3 | -11.8 |
| 2-2-d1E03-2-0436 | C37 H35 N7 O7 S4 | 817.14808 | -5.8 | -9.6 | 7.9 | 7.8 |
| 2-2-d1E03-2-0437 | C40 H34 F3 N5 O7 S2 | 817.18517 | -76.1 | 12.8 | 18.3 | 7.2 |
| 2-2-d1E03-2-0438 | C40 H34 F3 N5 O7 S2 | 817.18517 | -16.1 | 0.6 | 7.0 | 0.1 |
| 2-2-d1E03-2-0439 | C40 H34 F3 N5 O7 S2 | 817.18517 | 0.0 | 0.0 | -20.3 | 8.4 |
| 2-2-d1E03-2-0440 | C41 H37 F3 N4 O7 S2 | 818.20558 | -9.1 | -8.3 | 0.0 | 0.0 |
| 2-2-d1E03-2-0441 | C41 H37 F3 N4 O7 S2 | 818.20558 | 0.3 | 1.9 | -8.7 | -0.4 |
| 2-2-d1E03-2-0442 | C40 H36 F3 N5 O7 S2 | 819.20082 | -2.1 | -3.6 | 10.4 | 7.1 |
| 2-2-d1E03-2-0443 | C43 H40 N4 O9 S2 | 820.22367 | -45.8 | -5.4 | 23.2 | -5.4 |
| 2-2-d1E03-2-0444 | C40 H38 F3 N5 O7 S2 | 821.21647 | -32.0 | 0.6 | 7.4 | 6.3 |
| 2-2-d1E03-2-0445 | C39 H37 F3 N6 O7 S2 | 822.21172 | 7.6 | -8.1 | -1.4 | -5.3 |
| 2-2-d1E03-2-0446 | C43 H46 N6 O7 S2 | 822.28694 | 17.6 | 3.2 | 11.5 | 15.7 |
| 2-2-d1E03-2-0447 | C38 H36 F3 N7 O7 S2 | 823.20697 | -0.7 | 10.7 | 19.6 | 8.1 |
| 2-2-d1E03-2-0448 | C42 H41 N5 O7 S3 | 823.21681 | -6.5 | 40.1 | 4.4 | 3.2 |
| 2-2-d1E03-2-0449 | C39 H35 F3 N4 O7 S3 | 824.16200 | -42.4 | 57.2 | 7.7 | 2.5 |
| 2-2-d1E03-2-0450 | C42 H37 F N4 O9 S2 | 824.19860 | -12.9 | 4.2 | -3.0 | 13.7 |
| 2-2-d1E03-2-0451 | C40 H39 F3 N4 O8 S2 | 824.21614 | -46.4 | 2.7 | 19.6 | 0.5 |
| 2-2-d1E03-2-0452 | C39 H35 N7 O8 S3 | 825.17092 | 26.8 | -24.4 | 0.0 | 0.0 |
| 2-2-d1E03-2-0453 | C38 H34 F3 N5 O9 S2 | 825.17500 | 11.6 | -10.6 | 2.2 | 16.7 |
| 2-2-d1E03-2-0454 | C42 H43 F N6 O7 S2 | 826.26187 | 18.8 | 14.3 | 26.3 | 24.5 |
| 2-2-d1E03-2-0455 | C39 H37 N7 O8 S3 | 827.18657 | -13.2 | 20.0 | 11.1 | 8.2 |
| 2-2-d1E03-2-0456 | C45 H45 N7 O5 S2 | 827.29236 | 2.5 | 4.7 | 9.3 | 17.6 |
| 2-2-d1E03-2-0457 | C37 H35 F3 N6 O7 S3 | 828.16814 | 23.1 | 15.9 | 13.7 | 21.3 |
| 2-2-d1E03-2-0458 | C40 H39 N5 O7 S4 | 829.17323 | -36.6 | -28.7 | -11.9 | 6.8 |
| 2-2-d1E03-2-0459 | C38 H34 N6 O8 S4 | 830.13209 | -27.5 | -12.7 | 18.7 | -11.5 |
| 2-2-d1E03-2-0460 | C43 H37 N5 O7 S3 | 831.18551 | -22.8 | 2.4 | 14.9 | 2.9 |
| 2-2-d1E03-2-0461 | C43 H37 N5 O7 S3 | 831.18551 | 0.0 | 0.0 | -36.0 | -34.5 |
| 2-2-d1E03-2-0462 | C42 H36 N6 O7 S3 | 832.18076 | -26.9 | 22.4 | 7.0 | 9.9 |
| 2-2-d1E03-2-0463 | C42 H36 N6 O7 S3 | 832.18076 | 14.5 | -40.3 | 1.0 | 10.8 |
| 2-2-d1E03-2-0464 | C42 H36 N6 O7 S3 | 832.18076 | 0.0 | 0.0 | 0.0 | 0.0 |
| 2-2-d1E03-2-0465 | C42 H41 F3 N4 O7 S2 | 834.23688 | -15.9 | -1.3 | 8.3 | -22.2 |
| 2-2-d1E03-2-0466 | C44 H46 N6 O7 S2 | 834.28694 | -23.0 | 28.8 | -1.1 | 34.4 |
| 2-2-d1E03-2-0467 | C39 H35 F3 N6 O8 S2 | 836.19099 | 91.4 | 92.4 | 27.1 | 103.9 |
| 2-2-d1E03-2-0468 | C42 H40 N6 O7 S3 | 836.21206 | -27.4 | 4.8 | 12.0 | 11.4 |
| 2-2-d1E03-2-0469 | C41 H35 N5 O9 S3 | 837.15969 | -34.8 | -31.9 | -2.3 | 27.1 |

(continued)

| [Table 11-3-7] | | | | | | |
|---|---|---|---|---|---|---|
| | | | AS-MS(%) | | | |
| ID | Molecular Formula | Exact Mass | N1 | N2 | N3 | N4 |
| 2-2-d1E03-2-0470 | C41 H39 N7 O7 S3 | 837.20731 | 0.0 | 0.0 | 0.0 | 0.0 |
| 2-2-d1E03-2-0471 | C40 H38 N8 O7 S3 | 838.20256 | -0.8 | -0.9 | 17.2 | 3.0 |
| 2-2-d1E03-2-0472 | C39 H37 F3 N6 O8 S2 | 838.20664 | 8.0 | 40.5 | 15.8 | 44.9 |
| 2-2-d1E03-2-0473 | C45 H45 F3 N6 O5 S | 838.31242 | 0.3 | -4.8 | 32.3 | 21.6 |
| 2-2-d1E03-2-0474 | C42 H41 N5 O8 S3 | 839.21173 | -2.7 | -28.2 | -2.0 | 5.8 |
| 2-2-d1E03-2-0475 | C40 H39 F3 N4 O7 S3 | 840.19330 | -11.9 | 4.1 | 4.1 | 19.5 |
| 2-2-d1E03-2-0476 | C38 H34 F3 N5 O8 S3 | 841.15216 | 11.5 | -142.9 | 1.6 | 13.1 |
| 2-2-d1E03-2-0477 | C43 H37 F3 N4 O7 S2 | 842.20558 | 13.2 | 56.2 | -8.7 | 9.7 |
| 2-2-d1E03-2-0478 | C43 H37 F3 N4 O7 S2 | 842.20558 | 0.0 | 0.0 | 0.0 | 0.0 |
| 2-2-d1E03-2-0479 | C39 H37 N7 O7 S4 | 843.16373 | -8.0 | 23.6 | 12.6 | 17.6 |
| 2-2-d1E03-2-0480 | C42 H36 F3 N5 O7 S2 | 843.20082 | 0.0 | 0.0 | 0.0 | 0.0 |
| 2-2-d1E03-2-0481 | C42 H36 F3 N5 O7 S2 | 843.20082 | -0.1 | 2.7 | 10.2 | -1.6 |
| 2-2-d1E03-2-0482 | C42 H36 F3 N5 O7 S2 | 843.20082 | 9.3 | -19.2 | -26.4 | 14.9 |
| 2-2-d1E03-2-0483 | C42 H40 F3 N5 O7 S2 | 847.23212 | 2.6 | -9.8 | 24.2 | 3.4 |
| 2-2-d1E03-2-0484 | C41 H35 F3 N4 O9 S2 | 848.17975 | -53.8 | 16.2 | 17.9 | -14.1 |
| 2-2-d1E03-2-0485 | C41 H39 F3 N6 O7 S2 | 848.22737 | 4.2 | 49.4 | 13.7 | 2.8 |
| 2-2-d1E03-2-0486 | C45 H48 N6 O7 S2 | 848.30259 | 2.8 | 26.0 | 2.3 | 0.8 |
| 2-2-d1E03-2-0487 | C40 H38 F3 N7 O7 S2 | 849.22262 | -52.0 | -1.9 | 25.2 | 11.3 |
| 2-2-d1E03-2-0488 | C44 H43 N5 O7 S3 | 849.23246 | -10.4 | 22.2 | 28.6 | 4.2 |
| 2-2-d1E03-2-0489 | C42 H41 F3 N4 O8 S2 | 850.23179 | -222.4 | -30.7 | 3.0 | 6.1 |
| 2-2-d1E03-2-0490 | C44 H45 F N6 O7 S2 | 852.27752 | -1.8 | 14.2 | 26.6 | 7.4 |
| 2-2-d1E03-2-0491 | C39 H37 F3 N6 O7 S3 | 854.18379 | -57.5 | 4.6 | -8.5 | -2.1 |
| 2-2-d1E03-2-0492 | C42 H41 N5 O7 S4 | 855.18888 | 9.3 | -2.9 | -0.2 | -2.8 |
| 2-2-d1E03-2-0493 | C44 H43 F3 N4 O7 S2 | 860.25253 | -128.8 | 2.3 | 14.9 | -67.7 |
| 2-2-d1E03-2-0494 | C43 H37 N5 O9 S3 | 863.17534 | -48.8 | 18.8 | 12.5 | -6.1 |
| 2-2-d1E03-2-0495 | C43 H43 N7 O7 S3 | 865.23861 | 7.1 | 1.3 | 30.5 | 10.4 |
| 2-2-d1E03-2-0496 | C42 H41 F3 N4 O7 S3 | 866.20895 | 5.3 | -1.1 | 9.3 | 7.0 |
| 2-2-d1E03-2-0497 | C43 H37 F3 N4 O9 S2 | 874.19540 | 3.5 | -1.7 | 6.8 | 9.7 |
| 2-2-d1E03-2-0498 | C43 H43 F3 N6 O7 S2 | 876.25867 | -18.4 | 29.1 | 8.0 | 2.3 |
| 2-2-d1E03-2-0499 | C45 H45 N7 O7 S3 | 891.25426 | -3.1 | -13.0 | 11.8 | -3.2 |
| 2-2-d1E03-2-0500 | C45 H45 F3 N6 O7 S2 | 902.27432 | -32.1 | 20.1 | 14.4 | 15.0 |

[Table 605]

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0001 | C42 H45 N5 O7 S | 763.30397 | -1.9 | -1.4 | 13.2 | 9.9 |
| 2-2-d1E04-2-0002 | C43 H53 N5 O6 S | 767.37166 | -1.9 | -10.7 | 10.8 | 7.9 |
| 2-2-d1E04-2-0003 | C42 H51 N5 O7 S | 769.35092 | -18.1 | -6.0 | 8.2 | 9.8 |
| 2-2-d1E04-2-0004 | C44 H47 N5 O6 S | 773.32470 | 14.8 | -94.8 | 13.4 | 12.3 |
| 2-2-d1E04-2-0005 | C44 H47 N5 O6 S | 773.32470 | -3.4 | 11.3 | 9.3 | 11.4 |
| 2-2-d1E04-2-0006 | C43 H46 N6 O6 S | 774.31995 | -30.0 | -17.1 | 13.5 | 11.1 |
| 2-2-d1E04-2-0007 | C43 H47 N5 O7 S | 777.31962 | 3.9 | 0.7 | 12.1 | 10.6 |
| 2-2-d1E04-2-0008 | C42 H45 N5 O6 S2 | 779.28113 | -9.0 | -1.1 | 11.4 | 13.0 |
| 2-2-d1E04-2-0009 | C41 H44 N6 O8 S | 780.29413 | -51.2 | 0.0 | 0.0 | 0.0 |
| 2-2-d1E04-2-0010 | C42 H44 F N5 O7 S | 781.29455 | 2.6 | -8.9 | 12.0 | 9.8 |
| 2-2-d1E04-2-0011 | C44 H55 N5 O6 S | 781.38731 | -1.3 | -1.3 | 10.0 | 9.7 |
| 2-2-d1E04-2-0012 | C43 H53 N5 O7 S | 783.36657 | 0.8 | -4.7 | 8.9 | 7.2 |
| 2-2-d1E04-2-0013 | C43 H52 F N5 O6 S | 785.36223 | -4.0 | -8.9 | 11.8 | 9.1 |
| 2-2-d1E04-2-0014 | C45 H49 N5 O6 S | 787.34036 | 23.4 | 4.4 | 11.6 | 12.3 |
| 2-2-d1E04-2-0015 | C45 H49 N5 O6 S | 787.34036 | 23.4 | 4.4 | 11.4 | 10.9 |
| 2-2-d1E04-2-0016 | C42 H50 F N5 O7 S | 787.34150 | -24.7 | 3.7 | 7.0 | 9.0 |
| 2-2-d1E04-2-0017 | C44 H48 N6 O6 S | 788.33560 | 0.5 | 2.2 | 9.9 | 10.7 |
| 2-2-d1E04-2-0018 | C44 H47 N5 O7 S | 789.31962 | 21.2 | -18.8 | 8.9 | 10.9 |
| 2-2-d1E04-2-0019 | C42 H45 N7 O7 S | 791.31012 | 0.0 | 0.0 | 0.0 | 0.0 |
| 2-2-d1E04-2-0020 | C44 H46 F N5 O6 S | 791.31528 | 5.8 | 0.1 | 10.5 | 13.2 |
| 2-2-d1E04-2-0021 | C44 H46 F N5 O6 S | 791.31528 | 0.3 | -0.3 | 13.3 | 10.1 |
| 2-2-d1E04-2-0022 | C43 H45 F N6 O6 S | 792.31053 | 26.4 | -28.1 | 9.7 | 9.5 |
| 2-2-d1E04-2-0023 | C43 H47 N5 O6 S2 | 793.29678 | 28.1 | 1.1 | 13.5 | 12.2 |
| 2-2-d1E04-2-0024 | C42 H47 N7 O7 S | 793.32577 | -3.0 | 3.4 | 12.5 | 11.5 |
| 2-2-d1E04-2-0025 | C45 H55 N5 O6 S | 793.38731 | 13.7 | 0.2 | 13.3 | 14.0 |
| 2-2-d1E04-2-0026 | C42 H46 N6 O8 S | 794.30978 | 0.0 | -4.6 | 0.0 | 0.0 |
| 2-2-d1E04-2-0027 | C44 H53 N5 O7 S | 795.36657 | 0.6 | 4.8 | 11.3 | 10.7 |
| 2-2-d1E04-2-0028 | C41 H44 N6 O7 S2 | 796.27129 | 14.1 | -3.1 | 8.3 | 8.5 |
| 2-2-d1E04-2-0029 | C42 H44 F N5 O6 S2 | 797.27170 | -2.8 | 0.3 | 11.6 | 12.0 |
| 2-2-d1E04-2-0030 | C46 H47 N5 O6 S | 797.32470 | 12.9 | 16.9 | 8.9 | 13.7 |
| 2-2-d1E04-2-0031 | C46 H47 N5 O6 S | 797.32470 | -19.8 | -3.3 | 12.8 | 10.1 |
| 2-2-d1E04-2-0032 | C41 H43 F N6 O8 S | 798.28471 | 29.6 | -1.9 | 0.0 | 0.0 |
| 2-2-d1E04-2-0033 | C45 H46 N6 O6 S | 798.31995 | -0.4 | 1.4 | 11.4 | 10.6 |
| 2-2-d1E04-2-0034 | C45 H46 N6 O6 S | 798.31995 | 16.9 | -1.5 | 15.7 | 9.7 |
| 2-2-d1E04-2-0035 | C45 H46 N6 O6 S | 798.31995 | 22.6 | 6.0 | 15.7 | 9.7 |
| 2-2-d1E04-2-0036 | C46 H49 N5 O6 S | 799.34036 | 0.0 | 0.0 | 14.3 | 14.5 |
| 2-2-d1E04-2-0037 | C46 H49 N5 O6 S | 799.34036 | 0.0 | 0.0 | 0.0 | 0.0 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0038 | C45 H48 N6 O6 S | 800.33560 | 2.1 | -2.2 | 8.2 | 15.7 |
| 2-2-d1E04-2-0039 | C45 H50 N6 O6 S | 802.35125 | 5.7 | 2.3 | 30.7 | 13.2 |
| 2-2-d1E04-2-0040 | C45 H49 N5 O7 S | 803.33527 | 3.6 | 3.1 | 15.4 | 13.2 |
| 2-2-d1E04-2-0041 | C44 H49 N7 O6 S | 803.34650 | 19.1 | 3.9 | 11.9 | 11.4 |
| 2-2-d1E04-2-0042 | C43 H48 N8 O6 S | 804.34175 | 3.7 | 14.4 | 8.3 | 14.6 |
| 2-2-d1E04-2-0043 | C44 H47 N5 O6 S2 | 805.29678 | 7.9 | -50.1 | 0.0 | 0.0 |
| 2-2-d1E04-2-0044 | C43 H47 N7 O7 S | 805.32577 | -7.5 | -5.4 | 4.4 | 7.5 |
| 2-2-d1E04-2-0045 | C45 H51 N5 O7 S | 805.35092 | -2.6 | -0.6 | 7.0 | 6.6 |
| 2-2-d1E04-2-0046 | C43 H46 N6 O8 S | 806.30978 | 0.0 | 0.0 | 0.0 | 0.0 |
| 2-2-d1E04-2-0047 | C44 H46 F N5 O7 S | 807.31020 | 24.6 | -0.8 | 12.2 | 10.8 |
| 2-2-d1E04-2-0048 | C43 H49 N7 O7 S | 807.34142 | 20.7 | -48.8 | 14.8 | 13.2 |
| 2-2-d1E04-2-0049 | C46 H57 N5 O6 S | 807.40296 | 1.2 | -14.5 | 8.6 | 8.7 |
| 2-2-d1E04-2-0050 | C42 H44 F N7 O7 S | 809.30070 | 80.4 | 112.0 | 13.4 | 11.1 |
| 2-2-d1E04-2-0051 | C42 H47 N7 O6 S2 | 809.30292 | -2.6 | -1.8 | 10.3 | 11.1 |
| 2-2-d1E04-2-0052 | C45 H55 N5 O7 S | 809.38222 | 24.9 | 2.1 | 6.1 | 10.1 |
| 2-2-d1E04-2-0053 | C42 H46 N6 O7 S2 | 810.28694 | 21.3 | -9.8 | 11.7 | 11.4 |
| 2-2-d1E04-2-0054 | C42 H46 F N7 O7 S | 811.31635 | 43.5 | 5.0 | 10.6 | 10.4 |
| 2-2-d1E04-2-0055 | C47 H49 N5 O6 S | 811.34036 | 4.1 | -1.3 | 11.9 | 13.1 |
| 2-2-d1E04-2-0056 | C47 H49 N5 O6 S | 811.34036 | 0.0 | 0.0 | 0.0 | 0.0 |
| 2-2-d1E04-2-0057 | C45 H54 F N5 O6 S | 811.37788 | -54.0 | -54.7 | 15.9 | 8.8 |
| 2-2-d1E04-2-0058 | C46 H48 N6 O6 S | 812.33560 | 28.2 | 6.3 | 12.8 | 11.0 |
| 2-2-d1E04-2-0059 | C46 H48 N6 O6 S | 812.33560 | -46.2 | -37.0 | 11.8 | 10.9 |
| 2-2-d1E04-2-0060 | C46 H48 N6 O6 S | 812.33560 | 0.0 | 0.0 | 0.0 | 0.0 |
| 2-2-d1E04-2-0061 | C47 H51 N5 O6 S | 813.35601 | 5.8 | 7.0 | -21.6 | -4.9 |
| 2-2-d1E04-2-0062 | C47 H51 N5 O6 S | 813.35601 | 0.0 | 0.0 | 0.0 | 0.0 |
| 2-2-d1E04-2-0063 | C44 H52 F N5 O7 S | 813.35715 | 5.8 | 7.0 | 0.0 | 0.0 |
| 2-2-d1E04-2-0064 | C41 H43 F N6 O7 S2 | 814.26187 | -30.0 | -31.9 | 9.1 | 8.2 |
| 2-2-d1E04-2-0065 | C46 H50 N6 O6 S | 814.35125 | 25.8 | -15.5 | 11.5 | 10.4 |
| 2-2-d1E04-2-0066 | C46 H46 F N5 O6 S | 815.31528 | -20.7 | -10.2 | 12.6 | 7.6 |
| 2-2-d1E04-2-0067 | C46 H46 F N5 O6 S | 815.31528 | 17.4 | -47.3 | 9.7 | 15.0 |
| 2-2-d1E04-2-0068 | C47 H53 N5 O6 S | 815.37166 | 20.6 | -7.1 | 13.9 | 9.9 |
| 2-2-d1E04-2-0069 | C45 H45 F N6 O6 S | 816.31053 | 2.9 | -46.1 | 14.7 | 13.1 |
| 2-2-d1E04-2-0070 | C45 H45 F N6 O6 S | 816.31053 | 13.4 | -11.4 | 14.7 | 13.9 |
| 2-2-d1E04-2-0071 | C45 H45 F N6 O6 S | 816.31053 | 0.0 | -9.2 | 4.2 | 2.1 |
| 2-2-d1E04-2-0072 | C46 H52 N6 O6 S | 816.36690 | 25.9 | 5.4 | 12.3 | 11.3 |
| 2-2-d1E04-2-0073 | C44 H47 N7 O7 S | 817.32577 | 0.0 | 0.0 | 10.1 | 9.9 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0074 | C46 H48 F N5 O6 S | 817.33093 | 4.3 | 0.6 | 10.6 | 12.6 |
| 2-2-d1E04-2-0075 | C46 H48 F N5 O6 S | 817.33093 | 1.2 | -24.5 | 17.3 | 17.8 |
| 2-2-d1E04-2-0076 | C45 H51 N7 O6 S | 817.36215 | 9.4 | -164.0 | 17.3 | 19.2 |
| 2-2-d1E04-2-0077 | C45 H47 F N6 O6 S | 818.32618 | 7.4 | 10.7 | 20.3 | 10.0 |
| 2-2-d1E04-2-0078 | C44 H50 N8 O6 S | 818.35740 | -2.4 | -2.9 | 10.1 | 11.1 |
| 2-2-d1E04-2-0079 | C45 H49 N5 O6 S2 | 819.31243 | -9.0 | 30.6 | 0.0 | 0.0 |
| 2-2-d1E04-2-0080 | C44 H49 N7 O7 S | 819.34142 | -4.7 | 2.2 | 0.0 | 11.5 |
| 2-2-d1E04-2-0081 | C46 H53 N5 O7 S | 819.36657 | 21.9 | -9.8 | 10.1 | 11.8 |
| 2-2-d1E04-2-0082 | C43 H44 N6 O7 S2 | 820.27129 | -9.5 | 3.5 | 12.5 | 10.8 |
| 2-2-d1E04-2-0083 | C44 H48 N6 O8 S | 820.32543 | -1.4 | -4.0 | 11.2 | 10.6 |
| 2-2-d1E04-2-0084 | C45 H49 F N6 O6 S | 820.34183 | -36.1 | -16.1 | 3.1 | 9.7 |
| 2-2-d1E04-2-0085 | C45 H51 N5 O6 S2 | 821.32808 | 18.6 | -6.1 | 10.3 | 12.5 |
| 2-2-d1E04-2-0086 | C44 H48 F N7 O6 S | 821.33708 | -29.6 | 9.8 | 15.4 | 8.6 |
| 2-2-d1E04-2-0087 | C43 H46 N6 O7 S2 | 822.28694 | -16.5 | 14.9 | 14.8 | 14.3 |
| 2-2-d1E04-2-0088 | C43 H47 F N8 O6 S | 822.33233 | -1.9 | 1.4 | 10.5 | 13.2 |
| 2-2-d1E04-2-0089 | C44 H46 F N5 O6 S2 | 823.28735 | 10.7 | -12.8 | 18.5 | 17.9 |
| 2-2-d1E04-2-0090 | C43 H49 N7 O6 S2 | 823.31857 | 35.4 | -4.8 | 11.6 | 10.6 |
| 2-2-d1E04-2-0091 | C48 H49 N5 O6 S | 823.34036 | -4.8 | -2.8 | 8.2 | -41.0 |
| 2-2-d1E04-2-0092 | C48 H49 N5 O6 S | 823.34036 | 11.1 | 5.5 | 19.6 | 13.2 |
| 2-2-d1E04-2-0093 | C45 H50 F N5 O7 S | 823.34150 | 10.7 | 0.8 | 12.9 | 11.7 |
| 2-2-d1E04-2-0094 | C43 H45 F N6 O8 S | 824.30036 | -14.7 | 0.0 | 0.0 | 0.0 |
| 2-2-d1E04-2-0095 | C47 H48 N6 O6 S | 824.33560 | 1.5 | 5.3 | 10.4 | 18.3 |
| 2-2-d1E04-2-0096 | C47 H48 N6 O6 S | 824.33560 | 1.8 | 9.3 | 10.5 | 3.7 |
| 2-2-d1E04-2-0097 | C47 H48 N6 O6 S | 824.33560 | -1.5 | -69.6 | 15.0 | 23.7 |
| 2-2-d1E04-2-0098 | C44 H52 N6 O6 S2 | 824.33897 | 3.9 | -2.2 | 17.1 | 15.2 |
| 2-2-d1E04-2-0099 | C43 H50 N6 O7 S2 | 826.31824 | -17.9 | -24.9 | 12.1 | 12.3 |
| 2-2-d1E04-2-0100 | C42 H45 N5 O9 S2 | 827.26587 | -2.2 | -0.9 | 5.1 | 5.1 |
| 2-2-d1E04-2-0101 | C42 H46 F N7 O6 S2 | 827.29350 | -0.8 | -3.4 | 10.9 | 10.8 |
| 2-2-d1E04-2-0102 | C47 H52 N6 O6 S | 828.36690 | -0.7 | -1.9 | 0.0 | 0.0 |
| 2-2-d1E04-2-0103 | C46 H47 N5 O8 S | 829.31453 | 29.3 | 4.8 | 12.1 | 12.4 |
| 2-2-d1E04-2-0104 | C46 H51 N7 O6 S | 829.36215 | -3.4 | -14.7 | 16.6 | 13.8 |
| 2-2-d1E04-2-0105 | C48 H55 N5 O6 S | 829.38731 | 9.4 | 17.1 | 17.6 | 13.8 |
| 2-2-d1E04-2-0106 | C45 H46 N6 O6 S2 | 830.29202 | 0.7 | -9.0 | 14.8 | 12.9 |
| 2-2-d1E04-2-0107 | C45 H46 N6 O6 S2 | 830.29202 | 0.0 | 0.0 | 14.4 | 12.8 |
| 2-2-d1E04-2-0108 | C45 H50 N8 O6 S | 830.35740 | 4.2 | 0.7 | 12.6 | 11.0 |
| 2-2-d1E04-2-0109 | C44 H45 N7 O6 S2 | 831.28727 | -1.2 | 4.8 | 31.3 | 1.3 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0110 | C43 H44 F3 N5 O7 S | 831.29135 | -4.7 | -17.1 | 12.2 | 11.3 |
| 2-2-d1E04-2-0111 | C43 H53 N5 O8 S2 | 831.33356 | -12.4 | -6.9 | 2.4 | 2.4 |
| 2-2-d1E04-2-0112 | C45 H49 N7 O7 S | 831.34142 | -5.2 | -14.4 | 0.0 | 0.0 |
| 2-2-d1E04-2-0113 | C47 H53 N5 O7 S | 831.36657 | 2.0 | 2.8 | 10.9 | 7.4 |
| 2-2-d1E04-2-0114 | C42 H51 N5 O9 S2 | 833.31282 | -7.6 | -5.0 | 2.3 | 1.8 |
| 2-2-d1E04-2-0115 | C45 H51 N7 O7 S | 833.35707 | -1.5 | 8.4 | 15.7 | 16.6 |
| 2-2-d1E04-2-0116 | C47 H52 F N5 O6 S | 833.36223 | 23.6 | 3.7 | 13.7 | 12.9 |
| 2-2-d1E04-2-0117 | C44 H46 F N7 O7 S | 835.31635 | -1.3 | -6.3 | 2.1 | 2.1 |
| 2-2-d1E04-2-0118 | C44 H49 N7 O6 S2 | 835.31857 | -2.0 | -7.1 | 1.2 | -1.4 |
| 2-2-d1E04-2-0119 | C46 H53 N5 O6 S2 | 835.34373 | 10.2 | -20.9 | 10.4 | 10.4 |
| 2-2-d1E04-2-0120 | C44 H52 F3 N5 O6 S | 835.35904 | 5.5 | -10.8 | 7.7 | 12.0 |
| 2-2-d1E04-2-0121 | C43 H44 N6 O6 S3 | 836.24845 | 8.2 | 0.0 | 12.6 | 12.1 |
| 2-2-d1E04-2-0122 | C44 H48 N6 O7 S2 | 836.30259 | 41.0 | -21.1 | 7.2 | 5.8 |
| 2-2-d1E04-2-0123 | C42 H43 N7 O8 S2 | 837.26145 | -7.0 | -8.8 | 7.0 | 5.2 |
| 2-2-d1E04-2-0124 | C44 H47 N5 O8 S2 | 837.28660 | -13.3 | -3.9 | 6.4 | 7.2 |
| 2-2-d1E04-2-0125 | C44 H47 N5 O8 S2 | 837.28660 | -13.3 | -3.9 | 6.4 | 7.2 |
| 2-2-d1E04-2-0126 | C44 H48 F N7 O7 S | 837.33200 | 2.4 | 23.7 | 12.2 | 11.1 |
| 2-2-d1E04-2-0127 | C43 H50 F3 N5 O7 S | 837.33830 | 35.8 | -8.8 | 10.9 | 9.4 |
| 2-2-d1E04-2-0128 | C49 H51 N5 O6 S | 837.35601 | -6.4 | 11.5 | 15.3 | 19.4 |
| 2-2-d1E04-2-0129 | C49 H51 N5 O6 S | 837.35601 | -6.4 | 11.5 | 15.3 | 19.4 |
| 2-2-d1E04-2-0130 | C43 H46 N6 O8 S2 | 838.28185 | 1.4 | -3.3 | 3.9 | 3.1 |
| 2-2-d1E04-2-0131 | C48 H50 N6 O6 S | 838.35125 | 5.4 | -40.0 | 8.4 | 12.6 |
| 2-2-d1E04-2-0132 | C48 H50 N6 O6 S | 838.35125 | 0.0 | 6.5 | 10.2 | 13.2 |
| 2-2-d1E04-2-0133 | C48 H50 N6 O6 S | 838.35125 | -4.3 | -40.0 | 8.4 | 12.6 |
| 2-2-d1E04-2-0134 | C45 H50 F N5 O6 S2 | 839.31865 | -3.3 | -10.5 | 13.1 | 11.4 |
| 2-2-d1E04-2-0135 | C43 H45 F N6 O7 S2 | 840.27752 | -3.6 | -1.3 | 9.6 | 10.5 |
| 2-2-d1E04-2-0136 | C43 H47 N5 O9 S2 | 841.28152 | -1.4 | -33.0 | 5.5 | 5.2 |
| 2-2-d1E04-2-0137 | C45 H46 F3 N5 O6 S | 841.31209 | 2.2 | 1.2 | 19.2 | 12.4 |
| 2-2-d1E04-2-0138 | C45 H46 F3 N5 O6 S | 841.31209 | 2.2 | 1.2 | 17.9 | 11.9 |
| 2-2-d1E04-2-0139 | C48 H48 F N5 O6 S | 841.33093 | 7.5 | 7.2 | 17.7 | 15.0 |
| 2-2-d1E04-2-0140 | C48 H48 F N5 O6 S | 841.33093 | 5.2 | 1.3 | 13.5 | 3.5 |
| 2-2-d1E04-2-0141 | C49 H55 N5 O6 S | 841.38731 | 0.6 | -1.4 | 41.3 | 0.0 |
| 2-2-d1E04-2-0142 | C44 H45 F3 N6 O6 S | 842.30734 | 0.2 | -2.4 | 12.8 | 10.3 |
| 2-2-d1E04-2-0143 | C47 H47 F N6 O6 S | 842.32618 | 32.6 | -1.7 | 13.5 | 12.8 |
| 2-2-d1E04-2-0144 | C47 H47 F N6 O6 S | 842.32618 | -7.9 | -7.6 | 18.9 | 9.6 |
| 2-2-d1E04-2-0145 | C47 H47 F N6 O6 S | 842.32618 | 1.2 | 2.6 | 17.2 | 5.1 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0146 | C48 H54 N6 O6 S | 842.38255 | 15.6 | -31.8 | 0.0 | 0.0 |
| 2-2-d1E04-2-0147 | C42 H45 N5 O8 S3 | 843.24303 | -6.9 | -5.0 | 5.3 | 6.9 |
| 2-2-d1E04-2-0148 | C47 H49 N5 O8 S | 843.33018 | 8.7 | -8.3 | 10.6 | 11.0 |
| 2-2-d1E04-2-0149 | C47 H53 N7 O6 S | 843.37780 | 0.0 | 10.2 | 0.0 | 0.0 |
| 2-2-d1E04-2-0150 | C41 H44 N6 O10 S2 | 844.25603 | -14.3 | -6.4 | 4.2 | 4.3 |
| 2-2-d1E04-2-0151 | C46 H52 N8 O6 S | 844.37305 | 42.4 | 33.9 | 11.6 | 20.0 |
| 2-2-d1E04-2-0152 | C42 H44 F N5 O9 S2 | 845.25645 | -4.6 | -6.6 | 5.0 | 9.9 |
| 2-2-d1E04-2-0153 | C44 H55 N5 O8 S2 | 845.34921 | -5.1 | -8.1 | 2.9 | 2.6 |
| 2-2-d1E04-2-0154 | C48 H55 N5 O7 S | 845.38222 | 0.0 | 0.0 | 13.3 | 13.9 |
| 2-2-d1E04-2-0155 | C45 H46 N6 O7 S2 | 846.28694 | 18.0 | -1.9 | 17.3 | 11.1 |
| 2-2-d1E04-2-0156 | C47 H51 F N6 O6 S | 846.35748 | -7.1 | -6.1 | 10.6 | -3.9 |
| 2-2-d1E04-2-0157 | C43 H44 F3 N5 O6 S2 | 847.26851 | 3.8 | -0.2 | 13.1 | 12.6 |
| 2-2-d1E04-2-0158 | C46 H46 F N5 O8 S | 847.30511 | 2.4 | 2.4 | 15.1 | 13.4 |
| 2-2-d1E04-2-0159 | C43 H53 N5 O9 S2 | 847.32847 | -5.8 | -0.4 | 2.7 | 1.3 |
| 2-2-d1E04-2-0160 | C47 H53 N5 O6 S2 | 847.34373 | 4.5 | -7.0 | 40.6 | 0.0 |
| 2-2-d1E04-2-0161 | C46 H50 F N7 O6 S | 847.35273 | -2.2 | -4.6 | 0.2 | 1.3 |
| 2-2-d1E04-2-0162 | C43 H44 N8 O7 S2 | 848.27744 | 1.4 | -0.4 | 0.0 | 0.0 |
| 2-2-d1E04-2-0163 | C42 H43 F3 N6 O8 S | 848.28152 | -1.1 | 9.0 | 0.0 | 0.0 |
| 2-2-d1E04-2-0164 | C45 H49 F N8 O6 S | 848.34798 | 12.9 | 5.8 | 12.2 | 11.1 |
| 2-2-d1E04-2-0165 | C43 H52 F N5 O8 S2 | 849.32413 | -143.2 | 0.0 | 1.6 | 2.2 |
| 2-2-d1E04-2-0166 | C45 H51 N7 O6 S2 | 849.33422 | -3.9 | -6.1 | 0.0 | 0.0 |
| 2-2-d1E04-2-0167 | C47 H52 F N5 O7 S | 849.35715 | 11.2 | -2.0 | 26.9 | 16.4 |
| 2-2-d1E04-2-0168 | C43 H46 N8 O7 S2 | 850.29309 | 0.0 | -14.7 | 11.2 | 6.7 |
| 2-2-d1E04-2-0169 | C46 H54 N6 O6 S2 | 850.35462 | 1.8 | -7.3 | 7.0 | 11.1 |
| 2-2-d1E04-2-0170 | C45 H49 N5 O8 S2 | 851.30225 | -3.6 | -3.3 | 9.0 | 9.1 |
| 2-2-d1E04-2-0171 | C45 H49 N5 O8 S2 | 851.30225 | -6.7 | -17.8 | 9.0 | 11.2 |
| 2-2-d1E04-2-0172 | C42 H50 F N5 O9 S2 | 851.30340 | 0.2 | -4.5 | 2.4 | 1.8 |
| 2-2-d1E04-2-0173 | C44 H48 N6 O8 S2 | 852.29750 | -3.5 | -2.1 | 4.0 | 4.1 |
| 2-2-d1E04-2-0174 | C45 H52 N6 O7 S2 | 852.33389 | 20.0 | 2.5 | 5.0 | 7.5 |
| 2-2-d1E04-2-0175 | C42 H43 N7 O7 S3 | 853.23861 | 7.1 | -3.4 | 11.0 | 8.3 |
| 2-2-d1E04-2-0176 | C44 H47 N5 O9 S2 | 853.28152 | 16.3 | -247.1 | 7.0 | 6.3 |
| 2-2-d1E04-2-0177 | C44 H48 F N7 O6 S2 | 853.30915 | -5.1 | -3.8 | 2.6 | 0.9 |
| 2-2-d1E04-2-0178 | C47 H46 N6 O6 S2 | 854.29202 | -11.2 | -2.3 | 5.3 | 8.2 |
| 2-2-d1E04-2-0179 | C47 H46 N6 O6 S2 | 854.29202 | 5.2 | 4.1 | 15.1 | 1.8 |
| 2-2-d1E04-2-0180 | C42 H45 N7 O9 S2 | 855.27202 | 0.0 | 1.2 | 6.0 | 1.7 |
| 2-2-d1E04-2-0181 | C44 H46 F N5 O8 S2 | 855.27718 | -2.8 | -3.8 | 6.7 | 6.6 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0182 | C44 H46 F N5 O8 S2 | 855.27718 | -2.8 | -3.8 | 6.7 | 6.6 |
| 2-2-d1E04-2-0183 | C46 H45 N7 O6 S2 | 855.28727 | 0.4 | -2.1 | 7.6 | 15.2 |
| 2-2-d1E04-2-0184 | C46 H45 N7 O6 S2 | 855.28727 | -12.2 | -1.1 | 9.0 | 1.9 |
| 2-2-d1E04-2-0185 | C46 H45 N7 O6 S2 | 855.28727 | 0.0 | 0.0 | 14.3 | 8.9 |
| 2-2-d1E04-2-0186 | C48 H49 N5 O8 S | 855.33018 | 33.1 | -14.2 | 29.3 | 19.8 |
| 2-2-d1E04-2-0187 | C50 H57 N5 O6 S | 855.40296 | -0.2 | -4.1 | 15.7 | 12.9 |
| 2-2-d1E04-2-0188 | C43 H45 F N6 O8 S2 | 856.27243 | -4.9 | -7.1 | 3.9 | 3.4 |
| 2-2-d1E04-2-0189 | C47 H48 N6 O6 S2 | 856.30767 | -7.3 | 26.2 | 1.7 | 0.0 |
| 2-2-d1E04-2-0190 | C47 H48 N6 O6 S2 | 856.30767 | 0.0 | 0.0 | -71.4 | 0.0 |
| 2-2-d1E04-2-0191 | C43 H47 N5 O8 S3 | 857.25868 | -4.6 | -8.6 | 6.1 | 5.9 |
| 2-2-d1E04-2-0192 | C42 H47 N7 O9 S2 | 857.28767 | -5.6 | -4.6 | 5.0 | 4.3 |
| 2-2-d1E04-2-0193 | C46 H47 N7 O6 S2 | 857.30292 | 19.5 | -43.1 | 17.6 | 11.2 |
| 2-2-d1E04-2-0194 | C45 H46 F3 N5 O7 S | 857.30700 | 3.5 | 11.7 | 24.9 | 17.9 |
| 2-2-d1E04-2-0195 | C45 H55 N5 O8 S2 | 857.34921 | -13.5 | -30.1 | 5.6 | 1.6 |
| 2-2-d1E04-2-0196 | C48 H55 N7 O6 S | 857.39345 | 10.7 | 2.5 | 15.8 | 13.1 |
| 2-2-d1E04-2-0197 | C42 H46 N6 O10 S2 | 858.27168 | 0.0 | -228.7 | 3.8 | 5.4 |
| 2-2-d1E04-2-0198 | C43 H44 F3 N7 O7 S | 859.29750 | -63.8 | -67.4 | -35.7 | 0.0 |
| 2-2-d1E04-2-0199 | C46 H49 N7 O6 S2 | 859.31857 | 16.4 | 18.6 | 0.0 | 0.0 |
| 2-2-d1E04-2-0200 | C44 H53 N5 O9 S2 | 859.32847 | -14.6 | -2.1 | 4.5 | 3.4 |
| 2-2-d1E04-2-0201 | C49 H54 F N5 O6 S | 859.37788 | 1.9 | -4.2 | 10.1 | 6.5 |
| 2-2-d1E04-2-0202 | C41 H44 N6 O9 S3 | 860.23319 | -2.1 | -2.4 | 4.4 | 6.0 |
| 2-2-d1E04-2-0203 | C45 H48 N8 O6 S2 | 860.31382 | -15.1 | -7.5 | 0.0 | 12.5 |
| 2-2-d1E04-2-0204 | C42 H44 F N5 O8 S3 | 861.23360 | -24.3 | -0.3 | 4.9 | 7.4 |
| 2-2-d1E04-2-0205 | C46 H47 N5 O8 S2 | 861.28660 | 5.3 | 0.4 | 9.8 | 9.9 |
| 2-2-d1E04-2-0206 | C46 H47 N5 O8 S2 | 861.28660 | 0.0 | 0.0 | 10.0 | 9.6 |
| 2-2-d1E04-2-0207 | C44 H47 N9 O6 S2 | 861.30907 | 0.0 | 0.0 | 12.2 | 8.7 |
| 2-2-d1E04-2-0208 | C43 H46 F3 N7 O7 S | 861.31315 | 22.7 | -6.0 | 9.9 | 10.7 |
| 2-2-d1E04-2-0209 | C48 H55 N5 O6 S2 | 861.35938 | 0.0 | 53.0 | 20.3 | 22.7 |
| 2-2-d1E04-2-0210 | C46 H54 F3 N5 O6 S | 861.37469 | 12.6 | 12.8 | 13.6 | 15.1 |
| 2-2-d1E04-2-0211 | C41 H43 F N6 O10 S2 | 862.24661 | -5.0 | -0.2 | 4.0 | 4.1 |
| 2-2-d1E04-2-0212 | C45 H46 N6 O6 S3 | 862.26410 | 9.0 | -14.7 | 0.0 | 0.0 |
| 2-2-d1E04-2-0213 | C45 H46 N6 O8 S2 | 862.28185 | -4.8 | -2.2 | 6.7 | 6.0 |
| 2-2-d1E04-2-0214 | C45 H46 N6 O8 S2 | 862.28185 | 11.9 | -1.1 | 11.5 | 12.2 |
| 2-2-d1E04-2-0215 | C45 H46 N6 O8 S2 | 862.28185 | -4.0 | -19.1 | 6.7 | 6.0 |
| 2-2-d1E04-2-0216 | C46 H50 N6 O7 S2 | 862.31824 | -4.4 | -1.2 | 13.9 | 12.1 |
| 2-2-d1E04-2-0217 | C44 H45 N7 O8 S2 | 863.27710 | -16.8 | 0.0 | 0.0 | 0.0 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0218 | C46 H49 N5 O8 S2 | 863.30225 | 0.0 | 0.0 | 2.9 | 3.5 |
| 2-2-d1E04-2-0219 | C46 H49 N5 O8 S2 | 863.30225 | 27.3 | -3.8 | 0.0 | 0.0 |
| 2-2-d1E04-2-0220 | C45 H52 F3 N5 O7 S | 863.35395 | 2.9 | 3.7 | -5.6 | 17.7 |
| 2-2-d1E04-2-0221 | C42 H43 F3 N6 O7 S2 | 864.25867 | 13.1 | -7.3 | 12.0 | 10.4 |
| 2-2-d1E04-2-0222 | C45 H48 N6 O8 S2 | 864.29750 | -2.4 | -11.5 | 5.8 | 2.5 |
| 2-2-d1E04-2-0223 | C47 H46 F3 N5 O6 S | 865.31209 | 7.0 | 8.6 | 16.5 | 16.5 |
| 2-2-d1E04-2-0224 | C47 H46 F3 N5 O6 S | 865.31209 | 0.0 | 0.0 | 0.0 | 0.0 |
| 2-2-d1E04-2-0225 | C47 H52 F N5 O6 S2 | 865.33430 | -0.6 | 13.6 | 27.6 | 23.7 |
| 2-2-d1E04-2-0226 | C43 H46 N8 O6 S3 | 866.27024 | 10.5 | 7.5 | 14.5 | 11.4 |
| 2-2-d1E04-2-0227 | C46 H45 F3 N6 O6 S | 866.30734 | 16.0 | -3.4 | 13.7 | 12.7 |
| 2-2-d1E04-2-0228 | C46 H45 F3 N6 O6 S | 866.30734 | 4.0 | -4.3 | 14.7 | 15.7 |
| 2-2-d1E04-2-0229 | C46 H45 F3 N6 O6 S | 866.30734 | -6.5 | -9.5 | 0.0 | 0.0 |
| 2-2-d1E04-2-0230 | C45 H50 N6 O8 S2 | 866.31315 | 3.0 | 4.7 | 15.2 | 23.8 |
| 2-2-d1E04-2-0231 | C45 H49 N5 O9 S2 | 867.29717 | 7.0 | -0.8 | 6.9 | 6.7 |
| 2-2-d1E04-2-0232 | C44 H49 N7 O8 S2 | 867.30840 | -14.2 | -1.4 | 8.0 | 7.3 |
| 2-2-d1E04-2-0233 | C47 H48 F3 N5 O6 S | 867.32774 | 13.6 | 11.7 | 18.9 | 22.9 |
| 2-2-d1E04-2-0234 | C47 H48 F3 N5 O6 S | 867.32774 | 0.0 | 0.0 | 0.0 | 0.0 |
| 2-2-d1E04-2-0235 | C43 H48 N8 O8 S2 | 868.30365 | -5.3 | -3.5 | 7.0 | 5.8 |
| 2-2-d1E04-2-0236 | C46 H47 F3 N6 O6 S | 868.32299 | 3.9 | 6.0 | 14.8 | 13.2 |
| 2-2-d1E04-2-0237 | C44 H47 N5 O8 S3 | 869.25868 | 0.0 | 17.2 | 0.0 | 0.0 |
| 2-2-d1E04-2-0238 | C43 H47 N7 O9 S2 | 869.28767 | 14.1 | -0.8 | 6.0 | 5.2 |
| 2-2-d1E04-2-0239 | C45 H51 N5 O9 S2 | 869.31282 | -5.3 | -12.3 | 6.5 | 4.6 |
| 2-2-d1E04-2-0240 | C49 H51 N5 O8 S | 869.34583 | -9.9 | -0.8 | 13.9 | 6.8 |
| 2-2-d1E04-2-0241 | C43 H46 N6 O10 S2 | 870.27168 | -8.0 | -5.5 | 5.6 | 16.7 |
| 2-2-d1E04-2-0242 | C46 H49 F3 N6 O6 S | 870.33864 | 5.5 | -2.1 | 0.0 | 18.0 |
| 2-2-d1E04-2-0243 | C44 H46 F N5 O9 S2 | 871.27210 | -7.7 | -4.9 | 5.9 | 5.2 |
| 2-2-d1E04-2-0244 | C43 H49 N7 O9 S2 | 871.30332 | -2.3 | -5.3 | 5.0 | 5.9 |
| 2-2-d1E04-2-0245 | C45 H48 F3 N7 O6 S | 871.33389 | 5.3 | 4.6 | 13.7 | 11.1 |
| 2-2-d1E04-2-0246 | C46 H57 N5 O8 S2 | 871.36486 | 3.1 | -45.5 | 3.9 | 4.7 |
| 2-2-d1E04-2-0247 | C49 H57 N7 O6 S | 871.40910 | 12.9 | 14.3 | 15.2 | 13.2 |
| 2-2-d1E04-2-0248 | C44 H47 F3 N8 O6 S | 872.32914 | 2.5 | -17.2 | 10.5 | 10.3 |
| 2-2-d1E04-2-0249 | C48 H52 N6 O6 S2 | 872.33897 | -2.2 | 0.7 | 11.6 | 18.5 |
| 2-2-d1E04-2-0250 | C42 H44 F N7 O9 S2 | 873.26260 | 14.3 | -1.7 | 5.7 | 3.5 |
| 2-2-d1E04-2-0251 | C42 H47 N7 O8 S3 | 873.26482 | -8.2 | -6.3 | 9.9 | 9.8 |
| 2-2-d1E04-2-0252 | C45 H46 F3 N5 O6 S2 | 873.28416 | 5.6 | 3.9 | 13.3 | 12.9 |
| 2-2-d1E04-2-0253 | C48 H48 F N5 O8 S | 873.32076 | 17.9 | -8.2 | 17.3 | 22.8 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0254 | C46 H50 F3 N5 O7 S | 873.33830 | -2.9 | 5.5 | 11.8 | 13.0 |
| 2-2-d1E04-2-0255 | C45 H55 N5 O9 S2 | 873.34412 | -11.3 | 2.4 | 3.9 | 2.3 |
| 2-2-d1E04-2-0256 | C42 H46 N6 O9 S3 | 874.24884 | 7.5 | -0.9 | 8.3 | 9.4 |
| 2-2-d1E04-2-0257 | C45 H46 N8 O7 S2 | 874.29309 | 28.0 | 0.9 | 0.0 | 0.0 |
| 2-2-d1E04-2-0258 | C44 H45 F3 N6 O8 S | 874.29717 | -7.1 | 4.1 | -1.2 | 5.6 |
| 2-2-d1E04-2-0259 | C42 H46 F N7 O9 S2 | 875.27825 | -4.5 | -4.3 | 3.7 | 3.3 |
| 2-2-d1E04-2-0260 | C47 H49 N5 O8 S2 | 875.30225 | -8.6 | -5.9 | 11.2 | 10.8 |
| 2-2-d1E04-2-0261 | C47 H49 N5 O8 S2 | 875.30225 | 0.0 | 0.0 | 0.0 | 0.0 |
| 2-2-d1E04-2-0262 | C45 H54 F N5 O8 S2 | 875.33978 | 16.1 | 1.3 | 9.1 | 7.2 |
| 2-2-d1E04-2-0263 | C48 H54 F N7 O6 S | 875.38403 | 7.2 | 28.0 | 12.2 | 11.6 |
| 2-2-d1E04-2-0264 | C46 H48 N6 O8 S2 | 876.29750 | 1.2 | -4.3 | 5.7 | 6.9 |
| 2-2-d1E04-2-0265 | C46 H48 N6 O8 S2 | 876.29750 | -12.8 | -5.0 | 13.7 | 10.5 |
| 2-2-d1E04-2-0266 | C46 H48 N6 O8 S2 | 876.29750 | -70.1 | -32.7 | 13.9 | 10.7 |
| 2-2-d1E04-2-0267 | C45 H48 N8 O7 S2 | 876.30874 | -2.4 | -1.4 | 14.7 | 11.6 |
| 2-2-d1E04-2-0268 | C43 H46 F3 N7 O6 S2 | 877.29031 | -0.1 | 4.5 | 16.5 | 11.1 |
| 2-2-d1E04-2-0269 | C47 H51 N5 O8 S2 | 877.31791 | 11.5 | -61.0 | 3.0 | 3.2 |
| 2-2-d1E04-2-0270 | C47 H51 N5 O8 S2 | 877.31791 | 0.0 | 0.0 | 0.0 | 0.0 |
| 2-2-d1E04-2-0271 | C44 H52 F N5 O9 S2 | 877.31905 | 8.3 | -3.3 | 2.8 | 3.2 |
| 2-2-d1E04-2-0272 | C41 H43 F N6 O9 S3 | 878.22377 | -2.3 | -6.4 | 10.2 | 7.1 |
| 2-2-d1E04-2-0273 | C46 H50 N6 O6 S3 | 878.29540 | -4.7 | 0.6 | 11.7 | 15.5 |
| 2-2-d1E04-2-0274 | C46 H50 N6 O8 S2 | 878.31315 | -3.3 | -10.2 | 4.8 | 5.3 |
| 2-2-d1E04-2-0275 | C44 H45 N7 O7 S3 | 879.25426 | 0.9 | 11.4 | 6.3 | 14.6 |
| 2-2-d1E04-2-0276 | C46 H46 F N5 O8 S2 | 879.27718 | -1.4 | 2.0 | 11.8 | 11.1 |
| 2-2-d1E04-2-0277 | C46 H46 F N5 O8 S2 | 879.27718 | -1.4 | 2.0 | 11.8 | 11.1 |
| 2-2-d1E04-2-0278 | C47 H53 N5 O8 S2 | 879.33356 | 11.5 | -5.8 | 6.0 | 5.9 |
| 2-2-d1E04-2-0279 | C45 H45 F N6 O8 S2 | 880.27243 | 1.7 | 0.0 | 6.0 | 6.4 |
| 2-2-d1E04-2-0280 | C45 H45 F N6 O8 S2 | 880.27243 | 37.3 | 2.3 | 11.9 | 11.5 |
| 2-2-d1E04-2-0281 | C45 H45 F N6 O8 S2 | 880.27243 | 1.7 | 0.0 | 6.0 | 6.4 |
| 2-2-d1E04-2-0282 | C49 H48 N6 O6 S2 | 880.30767 | -6.5 | -6.4 | -18.2 | -14.2 |
| 2-2-d1E04-2-0283 | C49 H48 N6 O6 S2 | 880.30767 | -5.5 | -9.4 | 12.0 | 10.4 |
| 2-2-d1E04-2-0284 | C46 H52 N6 O8 S2 | 880.32880 | -9.4 | -4.2 | 7.1 | 7.2 |
| 2-2-d1E04-2-0285 | C44 H47 N7 O9 S2 | 881.28767 | -0.8 | 6.4 | 11.0 | 7.3 |
| 2-2-d1E04-2-0286 | C46 H48 F N5 O8 S2 | 881.29283 | -5.6 | -12.5 | 0.0 | 0.0 |
| 2-2-d1E04-2-0287 | C46 H48 F N5 O8 S2 | 881.29283 | -10.4 | 1.7 | 6.2 | 6.1 |
| 2-2-d1E04-2-0288 | C48 H47 N7 O6 S2 | 881.30292 | -6.7 | -4.1 | 3.7 | -2.1 |
| 2-2-d1E04-2-0289 | C48 H47 N7 O6 S2 | 881.30292 | 4.8 | 2.5 | 14.3 | 10.1 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0290 | C48 H47 N7 O6 S2 | 881.30292 | -2.8 | -16.1 | -2.3 | 0.0 |
| 2-2-d1E04-2-0291 | C45 H51 N7 O8 S2 | 881.32405 | -12.6 | -23.8 | 8.4 | 8.7 |
| 2-2-d1E04-2-0292 | C45 H47 F N6 O8 S2 | 882.28808 | -3.7 | -2.0 | 4.4 | 6.2 |
| 2-2-d1E04-2-0293 | C44 H50 N8 O8 S2 | 882.31930 | 6.7 | 3.4 | 12.4 | 8.6 |
| 2-2-d1E04-2-0294 | C45 H49 N5 O8 S3 | 883.27433 | 6.2 | 17.6 | 8.9 | 16.8 |
| 2-2-d1E04-2-0295 | C44 H49 N7 O9 S2 | 883.30332 | -19.3 | 1.9 | 2.6 | 6.9 |
| 2-2-d1E04-2-0296 | C46 H53 N5 O9 S2 | 883.32847 | 0.0 | -33.9 | 5.8 | 6.3 |
| 2-2-d1E04-2-0297 | C48 H52 F3 N5 O6 S | 883.35904 | 0.2 | 7.4 | 13.6 | 12.3 |
| 2-2-d1E04-2-0298 | C50 H57 N7 O6 S | 883.40910 | 15.8 | 9.8 | 20.1 | 16.1 |
| 2-2-d1E04-2-0299 | C43 H44 N6 O9 S3 | 884.23319 | 4.0 | -10.4 | 3.3 | 2.9 |
| 2-2-d1E04-2-0300 | C44 H48 N6 O10 S2 | 884.28733 | -5.3 | 8.2 | 9.7 | 8.4 |
| 2-2-d1E04-2-0301 | C45 H49 F N6 O8 S2 | 884.30373 | -0.9 | -6.8 | 9.1 | 8.3 |
| 2-2-d1E04-2-0302 | C45 H51 N5 O8 S3 | 885.28998 | -0.9 | -3.1 | 8.2 | 7.3 |
| 2-2-d1E04-2-0303 | C44 H48 F N7 O8 S2 | 885.29898 | -2.0 | -5.1 | 8.4 | 6.6 |
| 2-2-d1E04-2-0304 | C45 H46 F3 N7 O7 S | 885.31315 | -1.2 | 0.5 | 17.3 | -194.5 |
| 2-2-d1E04-2-0305 | C48 H51 N7 O6 S2 | 885.33422 | -4.1 | -4.6 | 6.3 | 4.8 |
| 2-2-d1E04-2-0306 | C43 H46 N6 O9 S3 | 886.24884 | -14.8 | -2.5 | 4.3 | 6.6 |
| 2-2-d1E04-2-0307 | C47 H46 N6 O8 S2 | 886.28185 | 0.0 | 31.2 | 0.0 | 0.0 |
| 2-2-d1E04-2-0308 | C43 H47 F N8 O8 S2 | 886.29423 | -2.9 | 0.2 | 9.8 | 7.4 |
| 2-2-d1E04-2-0309 | C47 H50 N8 O6 S2 | 886.32947 | 0.0 | 0.0 | 9.1 | 6.5 |
| 2-2-d1E04-2-0310 | C44 H46 F N5 O8 S3 | 887.24925 | -2.5 | -11.6 | 7.2 | 3.4 |
| 2-2-d1E04-2-0311 | C43 H49 N7 O8 S3 | 887.28047 | -4.0 | 0.4 | 8.9 | 8.5 |
| 2-2-d1E04-2-0312 | C48 H49 N5 O8 S2 | 887.30225 | 2.8 | -23.8 | 9.9 | 5.9 |
| 2-2-d1E04-2-0313 | C48 H49 N5 O8 S2 | 887.30225 | 4.6 | 6.5 | 5.6 | 6.6 |
| 2-2-d1E04-2-0314 | C45 H50 F N5 O9 S2 | 887.30340 | -3.8 | -16.6 | 5.7 | 6.9 |
| 2-2-d1E04-2-0315 | C46 H49 N9 O6 S2 | 887.32472 | 0.0 | 0.0 | 0.0 | 0.0 |
| 2-2-d1E04-2-0316 | C45 H48 F3 N7 O7 S | 887.32880 | 29.7 | 7.4 | 4.6 | 4.7 |
| 2-2-d1E04-2-0317 | C43 H45 F N6 O10 S2 | 888.26226 | -14.6 | -2.6 | 9.6 | 9.8 |
| 2-2-d1E04-2-0318 | C47 H48 N6 O8 S2 | 888.29750 | 0.0 | 0.0 | 5.8 | 8.7 |
| 2-2-d1E04-2-0319 | C47 H48 N6 O8 S2 | 888.29750 | 0.0 | 0.0 | 7.3 | 4.1 |
| 2-2-d1E04-2-0320 | C47 H48 N6 O8 S2 | 888.29750 | 23.5 | 13.0 | 13.5 | 9.1 |
| 2-2-d1E04-2-0321 | C44 H52 N6 O8 S3 | 888.30087 | -43.3 | 0.0 | 5.3 | 6.6 |
| 2-2-d1E04-2-0322 | C48 H52 N6 O7 S2 | 888.33389 | 20.2 | -20.7 | 0.0 | 0.0 |
| 2-2-d1E04-2-0323 | C46 H50 F3 N5 O6 S2 | 889.31546 | 0.8 | 11.5 | 16.2 | 19.8 |
| 2-2-d1E04-2-0324 | C44 H45 F3 N6 O7 S2 | 890.27432 | -0.6 | 14.5 | 0.0 | 0.0 |
| 2-2-d1E04-2-0325 | C43 H50 N6 O9 S3 | 890.28014 | -3.6 | -9.3 | 4.8 | 8.1 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0326 | C42 H46 F N7 O8 S3 | 891.25540 | -5.6 | -8.1 | 10.2 | 8.7 |
| 2-2-d1E04-2-0327 | C49 H48 F3 N5 O6 S | 891.32774 | -5.8 | -1.8 | 16.5 | 11.3 |
| 2-2-d1E04-2-0328 | C49 H48 F3 N5 O6 S | 891.32774 | 0.0 | 0.0 | 0.0 | 0.0 |
| 2-2-d1E04-2-0329 | C45 H48 N8 O6 S3 | 892.28589 | -8.2 | -30.7 | 8.1 | 12.0 |
| 2-2-d1E04-2-0330 | C48 H47 F3 N6 O6 S | 892.32299 | -2.5 | 3.2 | 10.8 | 6.1 |
| 2-2-d1E04-2-0331 | C48 H47 F3 N6 O6 S | 892.32299 | 0.0 | 0.0 | 9.6 | 9.2 |
| 2-2-d1E04-2-0332 | C48 H47 F3 N6 O6 S | 892.32299 | -3.1 | 3.8 | 10.8 | 6.1 |
| 2-2-d1E04-2-0333 | C47 H52 N6 O8 S2 | 892.32880 | 0.5 | 5.3 | 25.1 | 15.8 |
| 2-2-d1E04-2-0334 | C46 H47 N5 O10 S2 | 893.27643 | -4.1 | 0.3 | 11.0 | 11.2 |
| 2-2-d1E04-2-0335 | C46 H51 N7 O8 S2 | 893.32405 | 43.2 | 26.2 | 0.0 | 0.0 |
| 2-2-d1E04-2-0336 | C48 H55 N5 O8 S2 | 893.34921 | -8.0 | -19.5 | 6.9 | 6.1 |
| 2-2-d1E04-2-0337 | C45 H46 N6 O8 S3 | 894.25392 | -11.1 | -4.8 | 8.1 | 7.2 |
| 2-2-d1E04-2-0338 | C45 H46 N6 O8 S3 | 894.25392 | 0.0 | 0.0 | 5.8 | 0.0 |
| 2-2-d1E04-2-0339 | C45 H50 N8 O8 S2 | 894.31930 | -9.6 | -6.9 | 3.9 | 2.6 |
| 2-2-d1E04-2-0340 | C44 H45 N7 O8 S3 | 895.24917 | -14.2 | 0.6 | 5.0 | 5.1 |
| 2-2-d1E04-2-0341 | C43 H44 F3 N5 O9 S2 | 895.25325 | 11.2 | -3.8 | 6.5 | 5.7 |
| 2-2-d1E04-2-0342 | C45 H49 N7 O9 S2 | 895.30332 | 0.0 | 0.3 | 0.0 | 0.0 |
| 2-2-d1E04-2-0343 | C47 H53 N5 O9 S2 | 895.32847 | 1.1 | -18.8 | 0.0 | 0.0 |
| 2-2-d1E04-2-0344 | C48 H51 F3 N6 O6 S | 896.35429 | -12.5 | -3.2 | 7.8 | 5.1 |
| 2-2-d1E04-2-0345 | C47 H46 F3 N5 O8 S | 897.30192 | -2.9 | 2.0 | 23.0 | 9.0 |
| 2-2-d1E04-2-0346 | C45 H51 N7 O9 S2 | 897.31897 | 0.0 | 0.0 | 11.4 | 4.4 |
| 2-2-d1E04-2-0347 | C47 H52 F N5 O8 S2 | 897.32413 | 12.8 | 3.1 | 6.0 | 5.5 |
| 2-2-d1E04-2-0348 | C47 H50 F3 N7 O6 S | 897.34954 | -15.6 | -5.1 | 0.0 | 0.0 |
| 2-2-d1E04-2-0349 | C51 H59 N7 O6 S | 897.42475 | 0.9 | 0.6 | 11.9 | -6.4 |
| 2-2-d1E04-2-0350 | C46 H49 F3 N8 O6 S | 898.34479 | 20.6 | 10.0 | 0.0 | 0.0 |
| 2-2-d1E04-2-0351 | C50 H54 N6 O6 S2 | 898.35462 | -6.8 | -0.7 | 5.7 | -90.0 |
| 2-2-d1E04-2-0352 | C44 H46 F N7 O9 S2 | 899.27825 | -19.1 | 1.1 | 0.0 | 0.0 |
| 2-2-d1E04-2-0353 | C44 H49 N7 O8 S3 | 899.28047 | 0.0 | 0.0 | 0.0 | 0.0 |
| 2-2-d1E04-2-0354 | C46 H53 N5 O8 S3 | 899.30563 | 9.0 | -1.0 | 9.1 | 8.5 |
| 2-2-d1E04-2-0355 | C44 H52 F3 N5 O8 S2 | 899.32094 | -6.5 | -11.3 | 3.4 | 4.5 |
| 2-2-d1E04-2-0356 | C48 H52 F3 N5 O7 S | 899.35395 | 12.3 | -4.8 | 14.3 | 11.6 |
| 2-2-d1E04-2-0357 | C43 H44 N6 O8 S4 | 900.21035 | -1.1 | -6.1 | 3.6 | 6.1 |
| 2-2-d1E04-2-0358 | C44 H48 N6 O9 S3 | 900.26449 | 0.0 | -13.3 | 10.0 | 17.5 |
| 2-2-d1E04-2-0359 | C42 H43 N7 O10 S3 | 901.22335 | -7.3 | -7.8 | 2.9 | 1.6 |
| 2-2-d1E04-2-0360 | C44 H48 F N7 O9 S2 | 901.29390 | -2.8 | 11.6 | 10.3 | 8.7 |
| 2-2-d1E04-2-0361 | C43 H50 F3 N5 O9 S2 | 901.30020 | -6.0 | -15.0 | 4.0 | 2.6 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0362 | C49 H51 N5 O8 S2 | 901.31791 | 5.5 | -1.4 | 9.2 | 12.3 |
| 2-2-d1E04-2-0363 | C49 H51 N5 O8 S2 | 901.31791 | 0.0 | 0.0 | 0.0 | 4.5 |
| 2-2-d1E04-2-0364 | C50 H56 F N7 O6 S | 901.39968 | -6.7 | -1.8 | 11.4 | 19.4 |
| 2-2-d1E04-2-0365 | C48 H50 N6 O8 S2 | 902.31315 | -2.4 | -2.3 | 7.3 | 8.1 |
| 2-2-d1E04-2-0366 | C48 H50 N6 O8 S2 | 902.31315 | 21.5 | 5.0 | 0.0 | 14.7 |
| 2-2-d1E04-2-0367 | C48 H50 N6 O8 S2 | 902.31315 | -2.4 | -2.3 | 7.3 | 8.1 |
| 2-2-d1E04-2-0368 | C45 H50 F N5 O8 S3 | 903.28055 | -2.1 | -8.0 | 8.9 | 8.1 |
| 2-2-d1E04-2-0369 | C45 H48 F3 N7 O6 S2 | 903.30596 | -5.6 | -21.0 | 4.2 | 2.2 |
| 2-2-d1E04-2-0370 | C43 H45 F N6 O9 S3 | 904.23942 | 5.9 | -7.4 | 8.9 | 8.1 |
| 2-2-d1E04-2-0371 | C48 H52 N6 O6 S3 | 904.31105 | -4.1 | -22.5 | 0.0 | 0.0 |
| 2-2-d1E04-2-0372 | C45 H46 F3 N5 O8 S2 | 905.27399 | 3.9 | -7.5 | 6.2 | 7.6 |
| 2-2-d1E04-2-0373 | C45 H46 F3 N5 O8 S2 | 905.27399 | 16.7 | -47.4 | 0.0 | 0.0 |
| 2-2-d1E04-2-0374 | C48 H48 F N5 O8 S2 | 905.29283 | 13.9 | -7.7 | 9.2 | 8.7 |
| 2-2-d1E04-2-0375 | C48 H48 F N5 O8 S2 | 905.29283 | -0.9 | 15.7 | -26.7 | 0.0 |
| 2-2-d1E04-2-0376 | C49 H55 N5 O8 S2 | 905.34921 | 12.3 | -12.4 | 13.7 | 6.7 |
| 2-2-d1E04-2-0377 | C44 H45 F3 N6 O8 S2 | 906.26924 | -17.5 | -5.5 | 6.3 | 5.5 |
| 2-2-d1E04-2-0378 | C47 H47 F N6 O8 S2 | 906.28808 | 8.7 | -2.7 | 11.0 | 5.6 |
| 2-2-d1E04-2-0379 | C47 H47 F N6 O8 S2 | 906.28808 | 19.6 | 2.5 | 17.0 | 27.6 |
| 2-2-d1E04-2-0380 | C47 H47 F N6 O8 S2 | 906.28808 | 8.7 | -2.7 | 11.0 | 5.6 |
| 2-2-d1E04-2-0381 | C48 H54 N6 O8 S2 | 906.34445 | 0.0 | 0.0 | 0.0 | 0.0 |
| 2-2-d1E04-2-0382 | C47 H49 N5 O10 S2 | 907.29208 | 2.5 | -1.9 | 11.6 | 9.6 |
| 2-2-d1E04-2-0383 | C47 H53 N7 O8 S2 | 907.33970 | -12.2 | 6.2 | 9.0 | 6.9 |
| 2-2-d1E04-2-0384 | C46 H52 N8 O8 S2 | 908.33495 | 0.0 | 0.0 | 14.0 | 8.0 |
| 2-2-d1E04-2-0385 | C48 H55 N5 O9 S2 | 909.34412 | -3.3 | -11.8 | 4.0 | 15.6 |
| 2-2-d1E04-2-0386 | C50 H54 F3 N5 O6 S | 909.37469 | -7.1 | 10.1 | 15.3 | 14.0 |
| 2-2-d1E04-2-0387 | C45 H46 N6 O9 S3 | 910.24884 | 11.8 | -3.3 | 8.8 | 5.2 |
| 2-2-d1E04-2-0388 | C47 H51 F N6 O8 S2 | 910.31938 | 4.3 | 1.8 | -17.0 | 0.0 |
| 2-2-d1E04-2-0389 | C43 H44 F3 N5 O8 S3 | 911.23041 | -7.8 | -4.0 | 8.9 | 7.7 |
| 2-2-d1E04-2-0390 | C46 H46 F N5 O10 S2 | 911.26701 | -3.8 | -23.1 | 11.0 | 9.2 |
| 2-2-d1E04-2-0391 | C47 H53 N5 O8 S3 | 911.30563 | 5.6 | -8.1 | 10.5 | 6.0 |
| 2-2-d1E04-2-0392 | C46 H50 F N7 O8 S2 | 911.31463 | -11.5 | 0.1 | 0.0 | 0.0 |
| 2-2-d1E04-2-0393 | C43 H44 N8 O9 S3 | 912.23934 | -2.9 | -2.0 | 22.7 | 3.6 |
| 2-2-d1E04-2-0394 | C42 H43 F3 N6 O10 S2 | 912.24342 | 5.6 | -4.7 | 3.9 | 4.2 |
| 2-2-d1E04-2-0395 | C49 H48 N6 O8 S2 | 912.29750 | -0.6 | -0.9 | 0.0 | 0.0 |
| 2-2-d1E04-2-0396 | C45 H49 F N8 O8 S2 | 912.30988 | 5.2 | -7.9 | 11.6 | 8.5 |
| 2-2-d1E04-2-0397 | C45 H51 N7 O8 S3 | 913.29612 | 2.0 | 5.4 | 36.0 | 0.0 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0398 | C47 H52 F N5 O9 S2 | 913.31905 | 5.3 | -4.5 | 7.3 | 7.1 |
| 2-2-d1E04-2-0399 | C43 H46 N8 O9 S3 | 914.25499 | -39.9 | 0.9 | 0.0 | 0.0 |
| 2-2-d1E04-2-0400 | C46 H54 N6 O8 S3 | 914.31652 | -13.7 | -2.7 | 0.0 | 0.0 |
| 2-2-d1 E04-2-040 1 | C49 H54 N8 O6 S2 | 914.36077 | -16.3 | -20.0 | 11.7 | 16.1 |
| 2-2-d1E04-2-0402 | C48 H52 F3 N5 O6 S2 | 915.33111 | -4.9 | 2.7 | 16.8 | 13.1 |
| 2-2-d1E04-2-0403 | C45 H52 N6 O9 S3 | 916.29579 | 8.2 | -6.9 | 8.4 | 0.0 |
| 2-2-d1E04-2-0404 | C42 H43 N7 O9 S4 | 917.20051 | 24.6 | 5.9 | 7.2 | 6.4 |
| 2-2-d1E04-2-0405 | C44 H48 F N7 O8 S3 | 917.27105 | 8.3 | -30.8 | 6.6 | 15.7 |
| 2-2-d1E04-2-0406 | C47 H46 N6 O8 S3 | 918.25392 | -1.4 | 3.4 | 9.3 | 11.8 |
| 2-2-d1E04-2-0407 | C47 H46 N6 O8 S3 | 918.25392 | 0.0 | 0.0 | 9.3 | 11.8 |
| 2-2-d1E04-2-0408 | C46 H45 N7 O8 S3 | 919.24917 | -6.6 | -7.1 | -1.4 | 7.8 |
| 2-2-d1E04-2-0409 | C46 H45 N7 O8 S3 | 919.24917 | 3.1 | -13.8 | 10.4 | 10.7 |
| 2-2-d1E04-2-0410 | C46 H45 N7 O8 S3 | 919.24917 | 0.0 | 0.0 | 0.0 | 0.0 |
| 2-2-d1E04-2-0411 | C48 H49 N5 O10 S2 | 919.29208 | -4.8 | -1.2 | -23.2 | -3.3 |
| 2-2-d1E04-2-0412 | C50 H57 N5 O8 S2 | 919.36486 | -3.1 | -1.3 | 0.0 | 17.7 |
| 2-2-d1E04-2-0413 | C47 H48 N6 O8 S3 | 920.26957 | 1.4 | 1.5 | 12.8 | 3.9 |
| 2-2-d1E04-2-0414 | C47 H48 N6 O8 S3 | 920.26957 | 1.4 | 1.5 | 12.8 | 3.9 |
| 2-2-d1E04-2-0415 | C46 H47 N7 O8 S3 | 921.26482 | -28.5 | -1.3 | 1.3 | 10.5 |
| 2-2-d1E04-2-0416 | C45 H46 F3 N5 O9 S2 | 921.26890 | 6.4 | -8.9 | 5.8 | 9.1 |
| 2-2-d1E04-2-0417 | C48 H55 N7 O8 S2 | 921.35535 | 43.8 | 48.8 | 53.8 | 65.5 |
| 2-2-d1E04-2-0418 | C43 H44 F3 N7 O9 S2 | 923.25940 | -23.7 | 44.7 | 2.9 | 3.8 |
| 2-2-d1E04-2-0419 | C46 H49 N7 O8 S3 | 923.28047 | -3.9 | 21.2 | 15.2 | 7.5 |
| 2-2-d1E04-2-0420 | C49 H48 F3 N5 O8 S | 923.31757 | 16.3 | 2.6 | 14.5 | 13.4 |
| 2-2-d1E04-2-0421 | C49 H54 F N5 O8 S2 | 923.33978 | -7.5 | -4.9 | 9.8 | 7.7 |
| 2-2-d1E04-2-0422 | C45 H48 N8 O8 S3 | 924.27572 | 1.7 | 3.5 | 0.0 | 0.0 |
| 2-2-d1E04-2-0423 | C44 H47 N9 O8 S3 | 925.27097 | -0.3 | -0.6 | 0.0 | 0.0 |
| 2-2-d1E04-2-0424 | C43 H46 F3 N7 O9 S2 | 925.27505 | 1.5 | -6.2 | 6.1 | 5.3 |
| 2-2-d1E04-2-0425 | C48 H55 N5 O8 S3 | 925.32128 | 0.0 | 0.0 | 0.0 | 0.0 |
| 2-2-d1E04-2-0426 | C46 H54 F3 N5 O8 S2 | 925.33659 | 4.9 | -0.7 | 8.5 | 1.9 |
| 2-2-d1E04-2-0427 | C49 H54 F3 N7 O6 S | 925.38084 | 23.2 | -7.1 | 9.1 | 18.2 |
| 2-2-d1E04-2-0428 | C45 H46 N6 O8 S4 | 926.22600 | -4.3 | -10.3 | 4.1 | 9.3 |
| 2-2-d1E04-2-0429 | C46 H50 N6 O9 S3 | 926.28014 | -3.1 | -2.3 | 4.8 | 6.2 |
| 2-2-d1E04-2-0430 | C44 H45 N7 O10 S3 | 927.23900 | 2.6 | -10.1 | 12.0 | 3.5 |
| 2-2-d1E04-2-0431 | C45 H52 F3 N5 O9 S2 | 927.31585 | 15.0 | -13.2 | 11.5 | 7.0 |
| 2-2-d1E04-2-0432 | C42 H43 F3 N6 O9 S3 | 928.22057 | -7.5 | -4.1 | 7.0 | 7.6 |
| 2-2-d1E04-2-0433 | C47 H46 F3 N5 O8 S2 | 929.27399 | 1.9 | 2.3 | 13.3 | 10.5 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0434 | C47 H46 F3 N5 O8 S2 | 929.27399 | 0.0 | 0.0 | 0.0 | 0.0 |
| 2-2-d1E04-2-0435 | C47 H52 F N5 O8 S3 | 929.29620 | -16.1 | -7.4 | 8.5 | 3.6 |
| 2-2-d1E04-2-0436 | C43 H46 N8 O8 S4 | 930.23214 | -0.6 | -11.6 | 13.1 | 0.0 |
| 2-2-d1E04-2-0437 | C46 H45 F3 N6 O8 S2 | 930.26924 | 22.6 | 1.9 | 9.0 | 7.0 |
| 2-2-d1E04-2-0438 | C46 H45 F3 N6 O8 S2 | 930.26924 | 2.8 | -3.0 | 13.3 | 13.4 |
| 2-2-d1E04-2-0439 | C46 H45 F3 N6 O8 S2 | 930.26924 | 0.0 | 0.0 | 11.5 | 5.6 |
| 2-2-d1E04-2-0440 | C47 H48 F3 N5 O8 S2 | 931.28964 | -1.2 | -0.4 | 5.1 | -0.1 |
| 2-2-d1E04-2-0441 | C47 H48 F3 N5 O8 S2 | 931.28964 | 0.0 | 0.0 | 0.0 | 0.0 |
| 2-2-d1E04-2-0442 | C46 H47 F3 N6 O8 S2 | 932.28489 | -2.2 | -9.9 | 6.9 | 6.5 |
| 2-2-d1E04-2-0443 | C49 H51 N5 O10 S2 | 933.30773 | -0.7 | -6.5 | 11.4 | 10.1 |
| 2-2-d1E04-2-0444 | C46 H49 F3 N6 O8 S2 | 934.30054 | 19.6 | -24.2 | 5.7 | 7.0 |
| 2-2-d1E04-2-0445 | C45 H48 F3 N7 O8 S2 | 935.29579 | -2.4 | -2.5 | 8.1 | 4.4 |
| 2-2-d1E04-2-0446 | C49 H57 N7 O8 S2 | 935.37100 | 62.1 | 45.0 | 44.0 | 46.2 |
| 2-2-dlE04-2-0447 | C44 H47 F3 N8 O8 S2 | 936.29104 | -3.4 | 0.9 | 13.9 | 12.2 |
| 2-2-d1E04-2-0448 | C48 H52 N6 O8 S3 | 936.30087 | -5.3 | -0.7 | 10.6 | 9.8 |
| 2-2-d1E04-2-0449 | C45 H46 F3 N5 O8 S3 | 937.24606 | -0.3 | -6.8 | 15.1 | 15.6 |
| 2-2-d1E04-2-0450 | C48 H48 F N5 O10 S2 | 937.28266 | 7.3 | -1.8 | 9.6 | 8.0 |
| 2-2-d1E04-2-0451 | C46 H50 F3 N5 O9 S2 | 937.30020 | 2.2 | -40.7 | 6.5 | 6.5 |
| 2-2-d1E04-2-0452 | C45 H46 N8 O9 S3 | 938.25499 | -2.1 | 8.6 | 11.5 | 19.9 |
| 2-2-d1E04-2-0453 | C44 H45 F3 N6 O10 S2 | 938.25907 | 12.5 | 4.0 | 11.2 | 19.9 |
| 2-2-d1E04-2-0454 | C48 H54 F N7 O8 S2 | 939.34593 | 36.4 | 42.2 | 58.2 | 62.7 |
| 2-2-d1E04-2-0455 | C45 H48 N8 O9 S3 | 940.27064 | -13.5 | -11.3 | 3.7 | 13.8 |
| 2-2-d1E04-2-0456 | C51 H56 N8 O6 S2 | 940.37642 | -3.9 | -2.7 | 23.8 | 28.1 |
| 2-2-d1E04-2-0457 | C43 H46 F3 N7 O8 S3 | 941.25221 | -1.7 | -8.2 | 5.0 | 6.3 |
| 2-2-d1E04-2-0458 | C46 H50 N6 O8 S4 | 942.25730 | -0.9 | -3.8 | 8.1 | 8.7 |
| 2-2-d1E04-2-0459 | C44 H45 N7 O9 S4 | 943.21616 | -9.8 | -4.2 | 14.9 | 8.0 |
| 2-2-d1E04-2-0460 | C49 H48 N6 O8 S3 | 944.26957 | 6.1 | 7.3 | 16.3 | 16.2 |
| 2-2-d1E04-2-0461 | C49 H48 N6 O8 S3 | 944.26957 | 0.0 | 0.0 | 0.0 | 0.0 |
| 2-2-d1E04-2-0462 | C48 H47 N7 O8 S3 | 945.26482 | 7.4 | 0.1 | 9.4 | 8.0 |
| 2-2-d1E04-2-0463 | C48 H47 N7 O8 S3 | 945.26482 | 11.8 | 0.1 | 9.9 | 8.7 |
| 2-2-d1E04-2-0464 | C48 H47 N7 O8 S3 | 945.26482 | 7.4 | 0.1 | 9.4 | 8.0 |
| 2-2-d1E04-2-0465 | C48 H52 F3 N5 O8 S2 | 947.32094 | 7.4 | -0.6 | 8.8 | 10.4 |
| 2-2-d1E04-2-0466 | C50 H57 N7 O8 S2 | 947.37100 | 53.4 | 45.9 | 33.3 | 36.8 |
| 2-2-d1E04-2-0467 | C45 H46 F3 N7 O9 S2 | 949.27505 | -7.0 | -7.3 | 2.5 | 2.1 |
| 2-2-d1E04-2-0468 | C48 H51 N7 O8 S3 | 949.29612 | -18.4 | -16.6 | 8.0 | 13.4 |
| 2-2-d1E04-2-0469 | C47 H46 N6 O10 S3 | 950.24375 | 38.9 | 36.0 | 27.7 | 17.9 |

(continued)

| [Table 11-3-8] | | | | | | |
|---|---|---|---|---|---|---|
| | | | AS-MS (%) | | | |
| ID | Molecular Formula | Exact Mass | N1 | N2 | N3 | N4 |
| 2-2-d1E04-2-0470 | C47 H50 N8 O8 S3 | 950.29137 | -9.5 | 7.1 | 6.7 | 0.0 |
| 2-2-d1E04-2-0471 | C46 H49 N9 O8 S3 | 951.28662 | 0.0 | 47.6 | 12.9 | 9.6 |
| 2-2-d1 E04-2-04 72 | C45 H48 F3 N7 O9 S2 | 951.29070 | 4.7 | 0.1 | 9.5 | 1.8 |
| 2-2-d1E04-2-0473 | C51 H56 F3 N7 O6 S | 951.39649 | 3.9 | 11.5 | 0.0 | 0.0 |
| 2-2-d1E04-2-0474 | C48 H52 N6 O9 S3 | 952.29579 | 13.8 | -6.6 | 16.3 | 7.3 |
| 2-2-d1E04-2-0475 | C46 H50 F3 N5 O8 S3 | 953.27736 | -0.2 | -3.9 | 8.3 | 11.2 |
| 2-2-d1E04-2-0476 | C44 H45 F3 N6 O9 S3 | 954.23622 | -6.2 | 7.9 | -0.2 | 7.1 |
| 2-2-d1E04-2-0477 | C49 H48 F3 N5 O8 S2 | 955.28964 | -5.8 | 9.2 | 10.4 | 13.0 |
| 2-2-d1E04-2-0478 | C49 H48 F3 N5 O8 S2 | 955.28964 | 0.0 | 0.0 | 0.0 | 0.0 |
| 2-2-d1E04-2-0479 | C45 H48 N8 O8 S4 | 956.24779 | 8.1 | -6.9 | 0.0 | 0.0 |
| 2-2-d1E04-2-0480 | C48 H47 F3 N6 O8 S2 | 956.28489 | -2.2 | -3.1 | 18.4 | 8.2 |
| 2-2-dlE04-2-0481 | C48 H47 F3 N6 O8 S2 | 956.28489 | -7.7 | -8.4 | 2.1 | 1.6 |
| 2-2-dlE04-2-0482 | C48 H47 F3 N6 O8 S2 | 956.28489 | -12.1 | -30.6 | 12.4 | 11.4 |
| 2-2-dlE04-2-0483 | C48 H51 F3 N6 O8 S2 | 960.31619 | -23.8 | 0.0 | 0.0 | 0.0 |
| 2-2-dlE04-2-0484 | C47 H46 F3 N5 O10 S2 | 961.26382 | 7.2 | 1.2 | 7.0 | 10.2 |
| 2-2-d1E04-2-0485 | C47 H50 F3 N7 O8 S2 | 961.31144 | 0.0 | 0.0 | 13.5 | 15.0 |
| 2-2-d1E04-2-0486 | C51 H59 N7 O8 S2 | 961.38665 | 17.2 | -0.9 | 18.6 | 24.0 |
| 2-2-d1E04-2-0487 | C46 H49 F3 N8 O8 S2 | 962.30669 | 0.0 | 0.0 | 0.0 | -23.8 |
| 2-2-d1E04-2-0488 | C50 H54 N6 O8 S3 | 962.31652 | 22.1 | -1.1 | 14.2 | 4.2 |
| 2-2-d1E04-2-0489 | C48 H52 F3 N5 O9 S2 | 963.31585 | -0.1 | -0.3 | 17.3 | 15.8 |
| 2-2-d1E04-2-0490 | C50 H56 F N7 O8 S2 | 965.36158 | 12.5 | 31.5 | 24.4 | 23.2 |
| 2-2-d1E04-2-0491 | C45 H48 F3 N7 O8 S3 | 967.26786 | -10.5 | 11.5 | 0.0 | 0.0 |
| 2-2-d1E04-2-0492 | C48 H52 N6 O8 S4 | 968.27295 | -6.4 | -28.6 | 0.0 | 0.0 |
| 2-2-d1E04-2-0493 | C50 H54 F3 N5 O8 S2 | 973.33659 | 3.1 | -3.1 | 11.1 | 10.1 |
| 2-2-dlE04-2-0494 | C49 H48 N6 O10 S3 | 976.25940 | -27.0 | -11.9 | 0.0 | 0.0 |
| 2-2-d1E04-2-0495 | C49 H54 N8 O8 S3 | 978.32267 | 52.7 | 137.5 | 56.0 | 50.3 |
| 2-2-d1E04-2-0496 | C48 H52 F3 N5 O8 S3 | 979.29301 | -3.9 | 8.6 | 6.1 | 20.2 |
| 2-2-d1E04-2-0497 | C49 H48 F3 N5 O10 S2 | 987.27947 | 5.7 | 1.4 | 4.8 | 5.4 |
| 2-2-d1E04-2-0498 | C49 H54 F3 N7 O8 S2 | 989.34274 | 10.2 | 54.2 | 32.0 | 32.5 |
| 2-2-d1E04-2-0499 | C51 H56 N8 O8 S3 | 1004.33832 | 11.6 | -17.3 | 16.4 | 17.1 |
| 2-2-d1E04-2-0500 | C51 H56 F3 N7 O8 S2 | 1015.35839 | 5.2 | 14.4 | 18.7 | 15.0 |

From the results of evaluating the mixtures obtained from Examples 8 and 9, binding to Bcl-2 was detected as to two compounds, B2Q045-01 and B2Q046-01, which were involved as positive controls, as well as a plurality of compounds. Of these compounds, 13 compounds shown in Table 11-4 were synthesized separately in Example 12 and a binding confirmation assay was carried out by SPR in Example 13.

[Table 606]

| [Table 11-4] | | | | | | |
|---|---|---|---|---|---|---|
| | | | AS-MS(%) | | | |
| ID | Compound | No. Exact Mass | N1 | N2 | N3 | N4 |
| 2-2-d1E03-1-0417 | D152 | 780.23999 | 34.2 | 33.3 | 44.4 | 38.0 |
| 2-2-d1E03-1-0454 | D155 | 798.23057 | 34.7 | 33.4 | 39.2 | 38.2 |
| 2-2-d1E03-1-0495 | D158 | 837.20731 | 28.9 | 38.1 | 35.6 | 39.3 |
| 2-2-d1E04-1-0417 | D153 | 893.32405 | 70.3 | 77.8 | 72.8 | 71.8 |
| 2-2-d1E04-1-0446 | D154 | 907.33970 | 57.8 | 68.3 | 60.9 | 58.1 |
| 2-2-d1E04-1-0454 | D156 | 911.31463 | 72.6 | 87.8 | 78.5 | 74.6 |
| 2-2-d1E04-1-0495 | D159 | 950.29137 | 59.4 | 93.2 | 85.8 | 69.0 |
| 2-2-d1E04-1-0498 | D157 | 961.31144 | 45.5 | 61.4 | 52.7 | 51.0 |
| 2-2-d1E04-2-0417 | D163 | 921.35535 | 43.8 | 48.8 | 53.8 | 65.5 |
| 2-2-d1E04-2-0446 | D164 | 935.37100 | 62.1 | 45.0 | 44.0 | 46.2 |
| 2-2-d1E04-2-0454 | D165 | 939.34593 | 36.4 | 42.2 | 58.2 | 62.7 |
| 2-2-d1E04-2-0466 | D161 | 947.37100 | 53.4 | 45.9 | 33.3 | 36.8 |
| 2-2-d1E04-2-0495 | D162 | 978.32267 | 52.7 | 137.5 | 56.0 | 50.3 |

Example 12; Individual synthesis of Bcl-2 binding compounds identified by AS-MS experiment of Example 11

Example 12-1-1: Synthesis of D129-03R

[Formula 742]

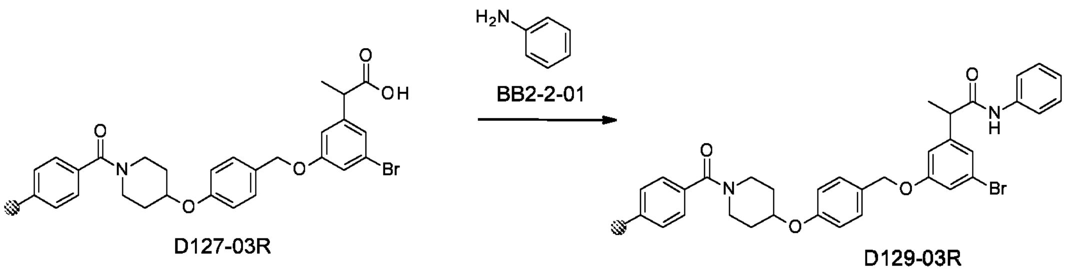


D127-03R

D129-03R

Solid-phase supported carboxylic acid (compound D127-03R) (1.8 g, 0.201 mmol/g) and DCM (21.6 mL) were added to a 50 mL column and shaken at room temperature for 1 hour. Aniline (BB2-2-01, 0.231 mL, 2.53 mmol) and 1 M PipClU (0.392 g, 0.109 mmol) + NMI (0.089 mL, 1.085 mmol) in MeCN (1.085 mL) (1 M each of PipClU and NMI) were added thereto, and the mixture was shaken at room temperature for 4.5 hours.

Solid-phase purification

The suspension of the reaction solution and the solid phase was filtered through the filter of the column and washed three times with NMP/water = 1/1 (36 mL), three times with NMP (36 mL), three times with MeOH (36 mL), three times with DCM (36 mL), and three times with heptane (36 mL), and the obtained solid phase was dried under reduced pressure to obtain the title compound D129-03R (2.07 g, 0.198 mmol/g).

Reaction monitoring

A small amount of the solid phase thus washed was transferred onto a filter, washed three times with DMA (0.1 mL), three times with methanol (0.1 mL), and three times with DCM (0.1 mL), and then dipped in a 10% solution of TFA in DCM containing 0.05 M pentamethylbenzene (0.05 mL) for 5 minutes. After filtration, the resin on the filter was washed with DMA (0.05 mL). Acetonitrile (0.10 mL) was added to the obtained filtrate to prepare an LC sample. The degree of progression of the reaction was measured by LCMS measurement. As a result, compound D129 of interest was obtained at a purity of 98.5%.

[Formula 743]

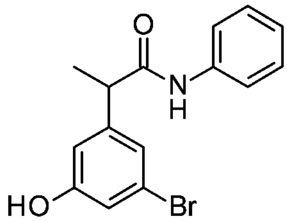

D129

Compound D129

LCMS: m/z 320, 322$[M+H]^+$

Retention time: 1.027 min (analysis conditions SMD-FA05-1, 280 nm).

Example 12-1-2: Synthesis of D130-03R

[Formula 744]

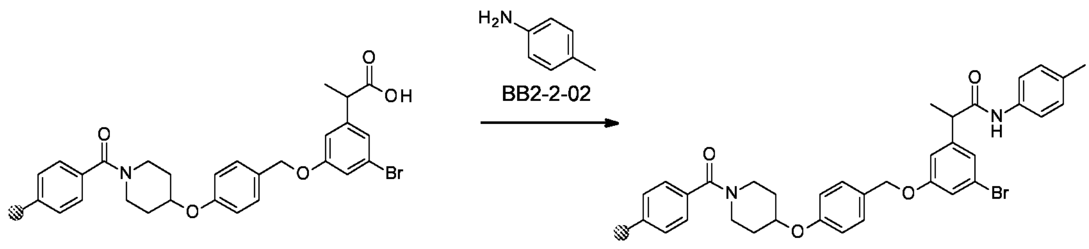


D127-03R D130-03R

The title compound D130-03R (1.50 g, 0.197 mmol/g) was obtained by synthesis by the same approach as in Example 12-1-1 using solid-phase supported carboxylic acid (compound D127-03R) (1.2 g, 0.201 mmol/g) and 4-methylaniline (BB2-2-02, 0.181 g, 1.69 mmol). The same cleavage operation as in Example 12-1-1 was performed, so that D130 was obtained at a purity of 97.8%.

[Formula 745]

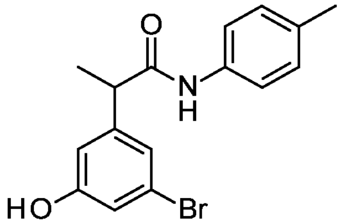

D130

Compound D130

LCMS: m/z 334, 336[M+H]$^+$

Retention time: 1.085 min (analysis conditions SMD-FA05-1, 280 nm)

Example 12-1-3: Synthesis of D167-03R

[Formula 746]

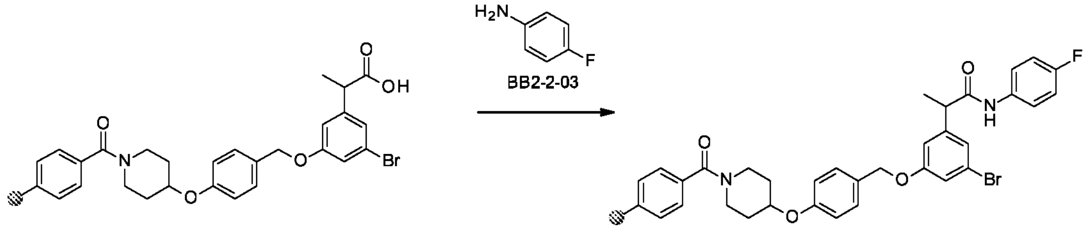


D127-03R

D167-03R

The title compound D167-03R (3.17 g, 0.197 mmol/g) was obtained by synthesis by the same approach as in Example 12-1-1 using solid-phase supported carboxylic acid (compound D127-03R) (3.00 g, 0.201 mmol/g) and 4-fluoroaniline (BB2-2-03, 0.400 mL, 4.22 mmol). The same cleavage operation as in Example 12-1-1 was performed, so that D167 was obtained at a purity of 98.9%.

[Formula 747]

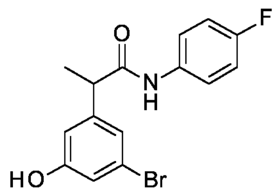

D167

Compound D167

LCMS: m/z 338, 340[M+H]$^+$

Retention time: 1.048 min (analysis conditions SMD-FA05-1, 280 nm)

Example 12-1-4: Synthesis of D131-03R

[Formula 748]

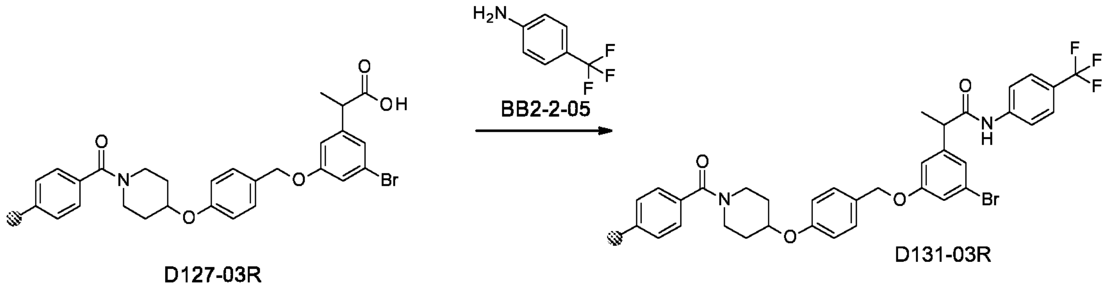


D127-03R

D131-03R

The title compound D131-03R (1.58 g, 0.195 mmol/g) was obtained by synthesis by the same approach as in Example 12-1-1 using solid-phase supported carboxylic acid (compound D127-03R) (1.2 g, 0.201 mmol/g) and 4-(trifluoromethyl)aniline (BB2-2-05, 0.209 mL, 1.69 mmol). The same cleavage operation as in Example 12-1-1 was performed, so that D131 was obtained at a purity of 97.2%.

[Formula 749]

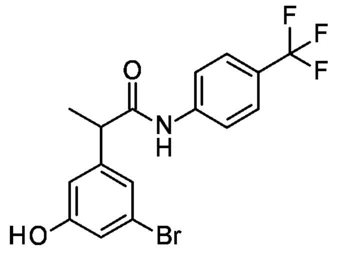

D131

Compound D131

LCMS: m/z 388, 390$[M+H]^+$

Retention time: 1.187 min (analysis conditions SMD-FA05-1, 280 nm)

Example 12-1-5: Synthesis of D132-03R

[Formula 750]

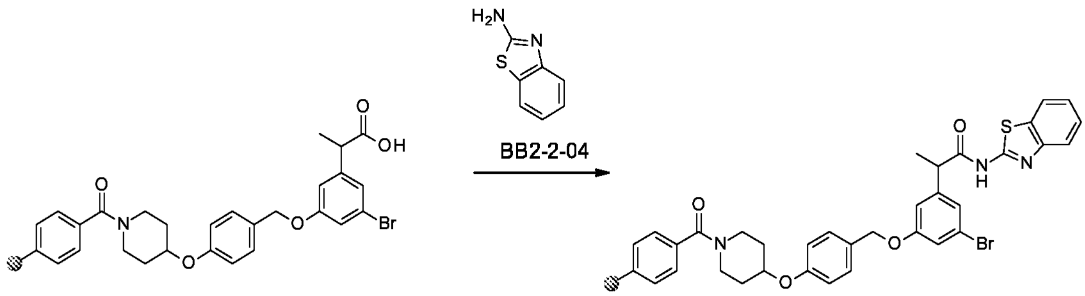


D127-03R D132-03R

The title compound D132-03R (4.87 g, 0.196 mmol/g) was obtained by synthesis by the same approach as in Example 12-1-1 using solid-phase supported carboxylic acid (compound D127-03R) (4.5 g, 0.201 mmol/g) and 1,3-benzothiazol-2-amine (BB2-2-04, 0.951 g, 6.33 mmol). The same cleavage operation as in Example 12-1-1 was performed, so that D132 was obtained at a purity of 99.9%.

[Formula 751]

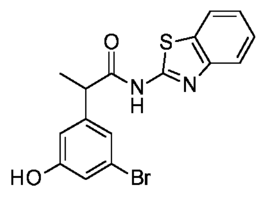

D132

Compound D132

LCMS: m/z 377, 379$[M+H]^+$

Retention time: 1.00 min (analysis conditions SMD-FA05-1, 280 nm)

Example 12-1-6: Synthesis ofD133-03R

[Formula 752]

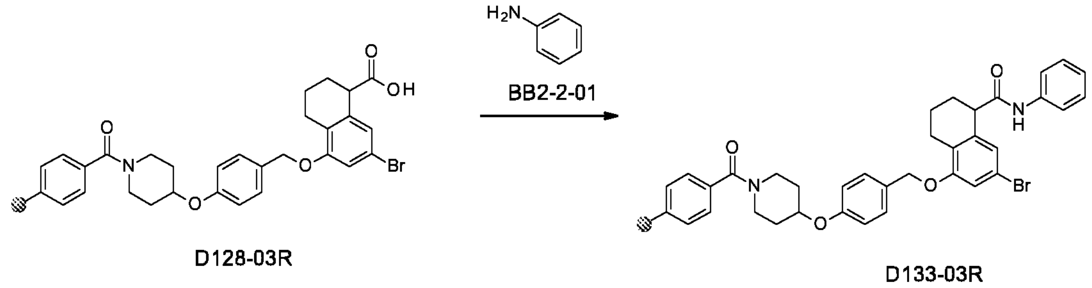


The title compound D133-03R (4.06 g, 0.197 mmol/g) was obtained by synthesis by the same approach as in Example 12-1-1 using solid-phase supported carboxylic acid (compound D128-03R) (4.0 g, 0.201 mmol/g) and aniline (BB2-2-01, 0.514 mL, 5.63 mmol). The same cleavage operation as in Example 12-1-1 was performed, so that D133 was obtained at a purity of 95.1%.

[Formula 753]

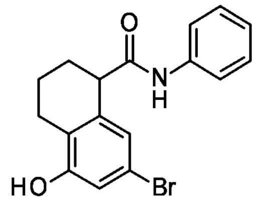

D133

Compound D133
LCMS: m/z 346, 348[M+H]+
Retention time: 1.052 min (analysis conditions SMD-FA05-1, 290 nm)

Example 12-2-1: Synthesis of D134-03R

[Formula 754]

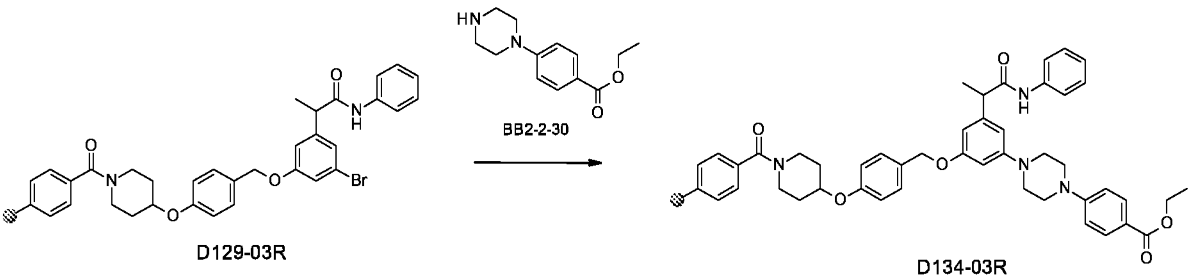


Under a nitrogen atmosphere, D129-03R (250 mg, 0.198 mmol/g, 0.049 mmol), DMPr (5.0 mL), and ethyl 4-piperazin-1-ylbenzoate (BB2-2-30, 116 mg, 0.495 mmol) were added and shaken at room temperature for 1 hour. (tBu)PhCPhos Pd G4 (39.0 mg, 0.050 mmol) and 2 M $P_2$tBu in THF (0.371 mL, 0.743 mmol) were added thereto, and the mixture was shaken at 80°C for 2.5 hours.
Solid-phase purification
The suspension of the reaction solution and the solid phase was transferred to a 6 mL column with a filter, filtered, and washed three times with NMP/water = 1/1 (5 mL), three times with NMP (5 mL), three times with 0.2 M NAC in NMP/water = 5/1 (5 mL), three times with 0.05 M $TBAHSO_4$ + DTBP in NMP (5 mL), three times with 0.05 M BTMG in NMP (5 mL), three times with NMP/water = 1/1 (5 mL), three times with NMP (5 mL), three times with MeOH (5 mL), three times with DCM (5 mL), and three times with heptane (5 mL), and the obtained solid phase was dried under reduced

pressure to obtain the title compound D134-03R (268.3 mg, 0.1922 mmol/g). The same cleavage operation as in Example 12-1-1 was performed, so that D134 was obtained at a purity of 88.5%.

[Formula 755]

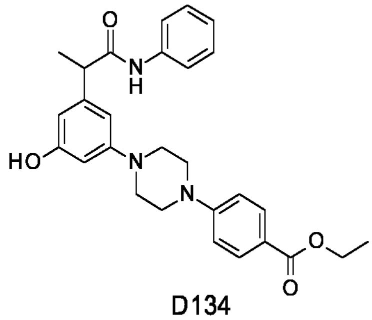

D134

Compound D134

LCMS: m/z 474[M+H]+

Retention time: 1.191 min (analysis conditions SMD-FA05-1, 280 nm)

Example 12-2-2: Synthesis of D135-03R

[Formula 756]

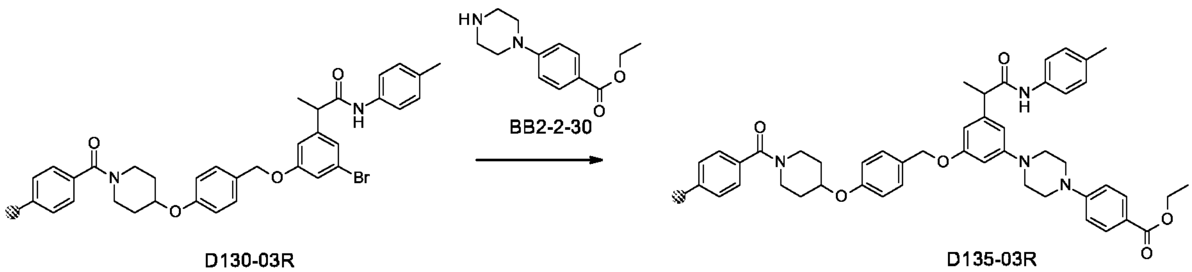


The title compound D135-03R (257.3 mg, 0.1912 mmol/g) was obtained by synthesis by the same approach as in Example 12-2-1 using D135-03R (250 mg, 0.197 mmol/g, 0.049 mmol) and ethyl 4-piperazin-1-ylbenzoate (BB2-2-30, 115 mg, 0.493 mmol). The same cleavage operation as in Example 12-1-1 was performed, so that D135 was obtained at a purity of 96.9%.

[Formula 757]

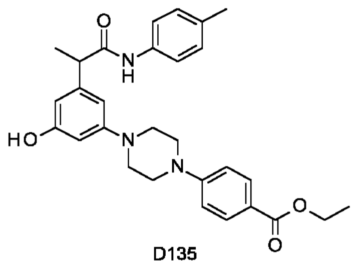

D135

Compound D135

LCMS: m/z 488[M+H]+

Retention time: 1.211 min (analysis conditions SMD-FA05-1, 280 nm)

Example 12-2-3: Synthesis of D136-03R

[Formula 758]

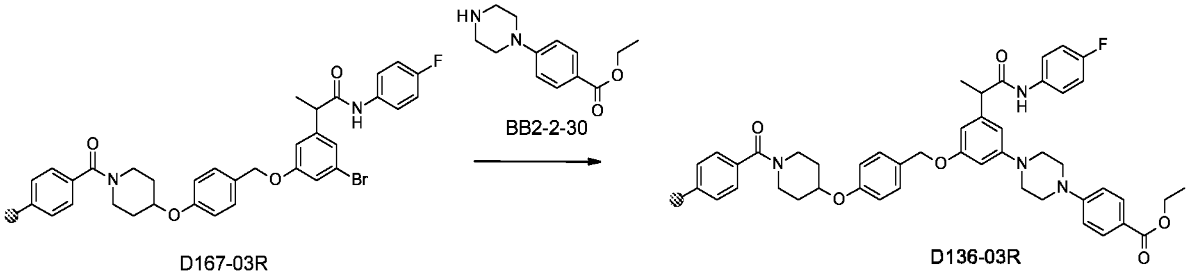


D167-03R

D136-03R

The title compound D136-03R (0.189 mmol/g) was obtained by synthesis by the same approach as in Example 12-2-1 using D167-03R (250 mg, 0.197 mmol/g, 0.049 mmol) and ethyl 4-piperazin-1-ylbenzoate (BB2-2-30, 115 mg, 0.493 mmol). The same cleavage operation as in Example 12-1-1 was performed, so that D136 was obtained at a purity of 96.0%.

[Formula 759]

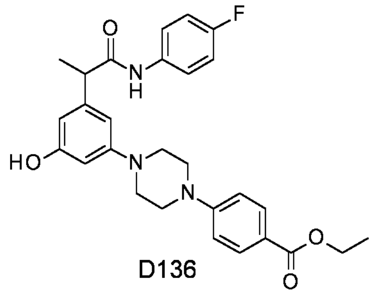

D136

Compound D136
LCMS: m/z 492[M+H]$^+$
Retention time: 1.177 min (analysis conditions SMD-FA05-1, 280 nm)

Example 12-2-4: Synthesis of D137-03R

[Formula 760]

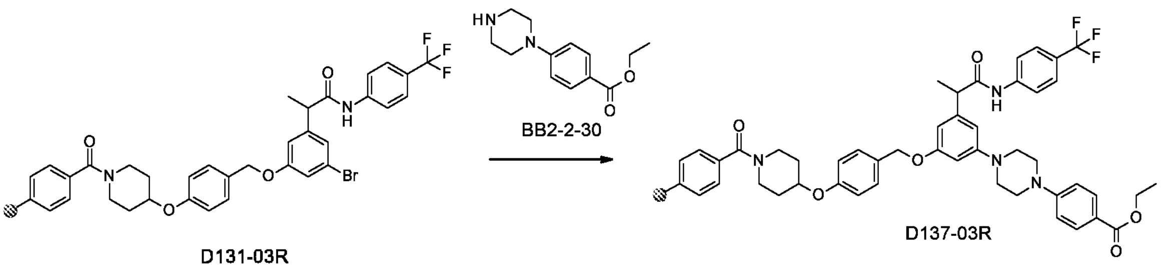


D131-03R

D137-03R

The title compound D137-03R (263.5 mg, 0.189 mmol/g) was obtained by synthesis by the same approach as in Example 12-2-1 using D131-03R (250 mg, 0.195 mmol/g, 0.049 mmol) and ethyl 4-piperazin-1-ylbenzoate (BB2-2-30, 114 mg, 0.488 mmol). The same cleavage operation as in Example 12-1-1 was performed, so that D137 was obtained at a purity of 91.0%.

[Formula 761]

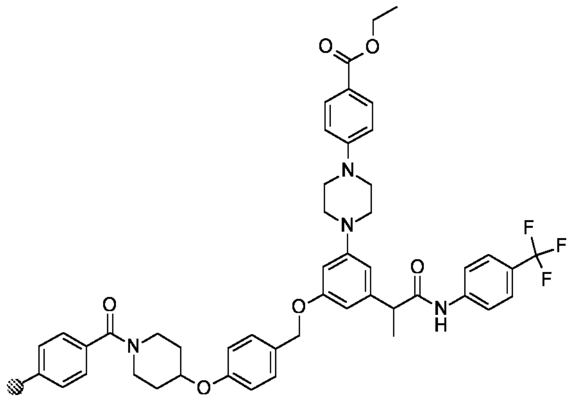

D137

Compound D137

LCMS: m/z 542[M+H]+

Retention time: 1.295 min (analysis conditions SMD-FA05-1, 280 nm)

Example 12-2-5: Synthesis of D138-03R

[Formula 762]

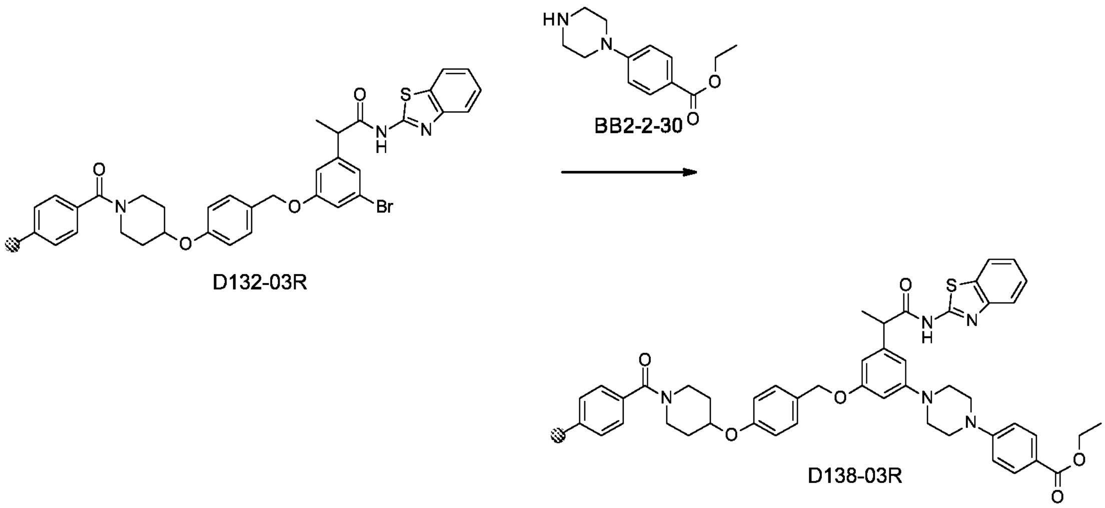

BB2-2-30

D132-03R

D138-03R

The title compound D138-03R (259.8 mg, 0.193 mmol/g) was obtained by synthesis by the same approach as in Example 12-2-1 using D132-03R (250 mg, 0.196 mmol/g) and ethyl 4-piperazin-1-ylbenzoate (BB2-2-30, 115 mg, 0.490 mmol). The same cleavage operation as in Example 12-1-1 was performed, so that D138 was obtained at a purity of 93.3%.

[Formula 763]

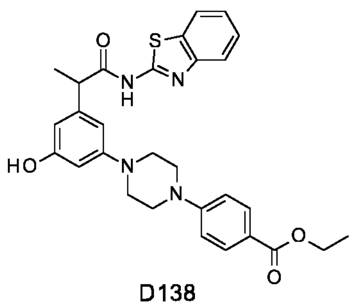

D138

Compound D138

LCMS: m/z 531[M+H]+

Retention time: 1.232 min (analysis conditions SMD-FA05-1, 290 nm)

Example 12-2-6: Synthesis of D139-03R

[Formula 764]

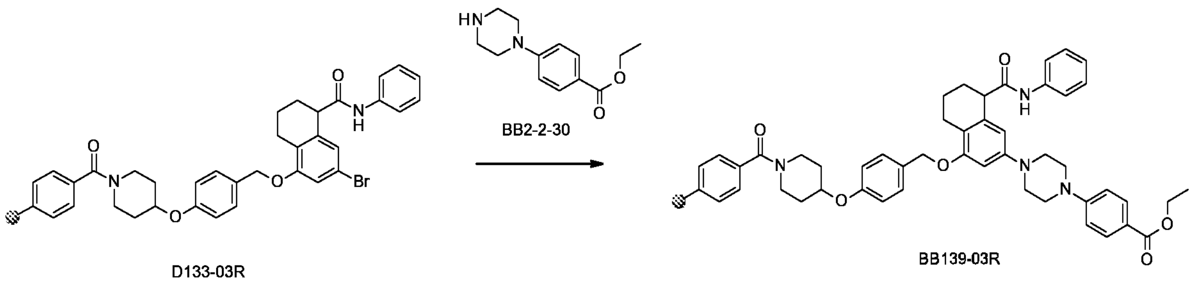


The title compound D139-03R (252.0 mg, 0.192 mmol/g) was obtained by synthesis by the same approach as in Example 12-2-1 using D133-03R (250 mg, 0.198 mmol/g, 0.0495 mmol) and ethyl 4-piperazin-1-ylbenzoate (BB2-2-30, 116 mg, 0.495 mmol). The same cleavage operation as in Example 12-1-1 was performed, so that D139 was obtained at a purity of 79.4%.

[Formula 765]

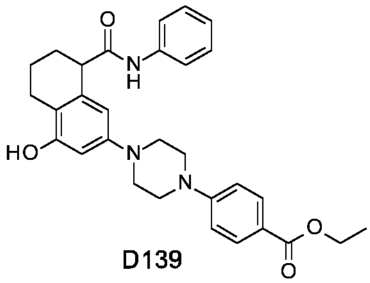

D139

Compound D139

LCMS: m/z 500[M+H]+

Retention time: 1.163 min (analysis conditions SMD-FA05-1, 280 nm)

Example 12-3-1: Synthesis of D140-03R

[Formula 766]

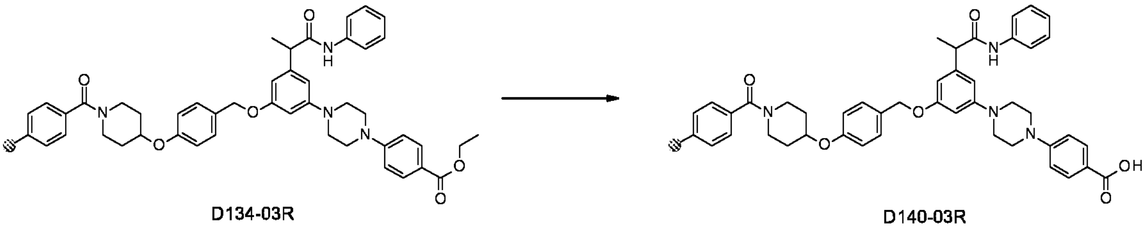

D134-03R D140-03R

Under a nitrogen atmosphere, NMP (3.5 mL) and tAmylOH (1.0 mL) were added to a 6 mL column with a filter containing D134-03R (250 mg, 0.192 mmol/g) and shaken at room temperature for 1 hour. A 1 M aqueous TBAOH solution (0.240 mL, 0.240 mmol) was added thereto, and the mixture was shaken at 60°C for 14 hours.

Solid-phase purification

The suspension of the reaction solution and the solid phase was filtered through the filter of the column and washed three times with NMP/water = 1/1 (5 mL), three times with 0.05 M $TBAHSO_4$ + DTBP in NMP (5 mL), three times with NMP/water = 1/1 (5 mL), three times with NMP (5 mL), three times with MeOH (5 mL), three times with DCM (5 mL), and three times with heptane (5 mL), and the obtained solid phase was dried under reduced pressure to obtain the title compound D140-03R (246.8 mg, 0.1932 mmol/g). The same cleavage operation as in Example 12-1-1 was performed, so that D140 was obtained at a purity of 87.4%.

[Formula 767]

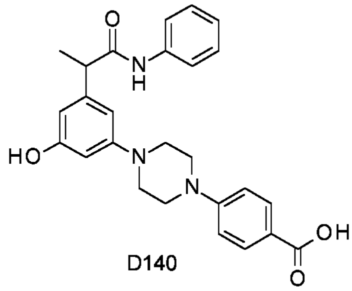

D140

Compound D140

LCMS: m/z 446$[M+H]^+$

Retention time: 0.909 min (analysis conditions SMD-FA05-1, 280 nm)

Example 12-3-2: Synthesis of D141-03R

[Formula 768]

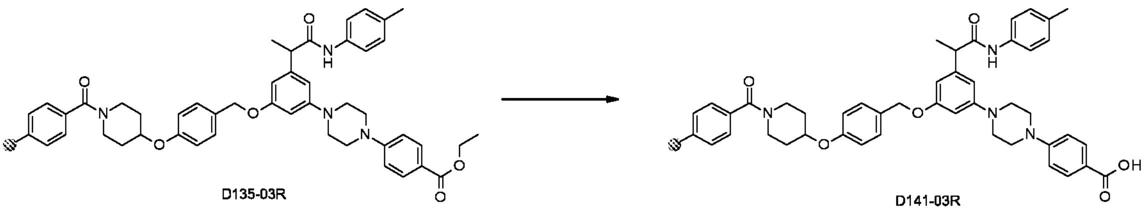

D135-03R D141-03R

The title compound D141-03R (239.6 mg, 0.1922 mmol/g) was obtained by synthesis by the same approach as in Example 12-3-1 using D135-03R (250 mg, 0.1912 mmol/g). The same cleavage operation as in Example 12-1-1 was performed, so that D141 was obtained at a purity of 88.8%.

[Formula 769]

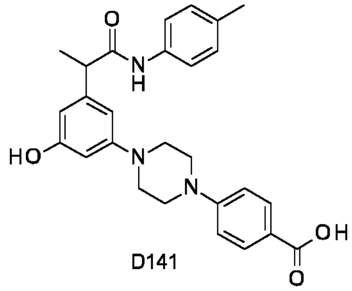

D141

Compound D141
LCMS: m/z 460[M+H]+
Retention time: 0.964 min (analysis conditions SMD-FA05-1, 280 nm)

Example 12-3-3: Synthesis of D142-03R

[Formula 770]

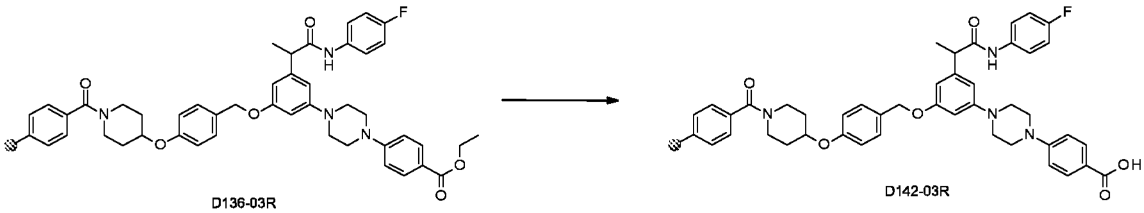

D136-03R

D142-03R

The title compound D142-03R (247.0 mg, 0.1932 mmol/g) was obtained by synthesis by the same approach as in Example 12-3-1 using D136-03R (250 mg, 0.1922 mmol/g). The same cleavage operation as in Example 12-1-1 was performed, so that D142 was obtained at a purity of 87.4%.

[Formula 771]

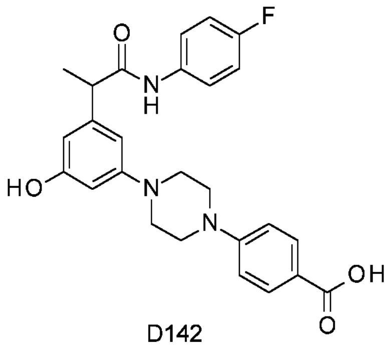

D142

Compound D142
LCMS: m/z 464[M+H]+
Retention time: 0.933 min (analysis conditions SMD-FA05-1, 280 nm)

Example 12-3-4: Synthesis of D143-03R

[Formula 772]

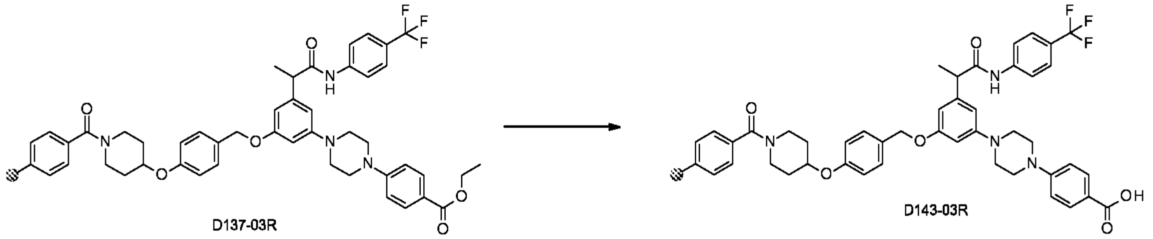

The title compound D143-03R (253.7 mg, 0.1900 mmol/g) was obtained by synthesis by the same approach as in Example 12-3-1 using D137-03R (250 mg, 0.189 mmol/g). The same cleavage operation as in Example 12-1-1 was performed, so that D143 was obtained at a purity of 79.5%.

[Formula 773]

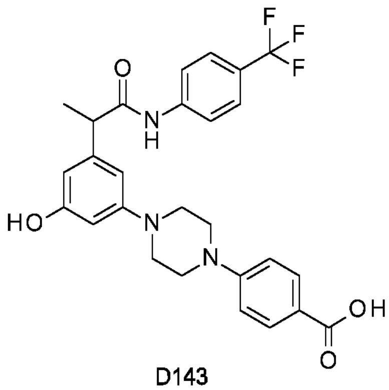

D143

Compound D143
LCMS: m/z 514[M+H]+
Retention time: 1.059 min (analysis conditions SMD-FA05-1, 280 nm)

Example 12-3-5: Synthesis of D144-03R

[Formula 774]

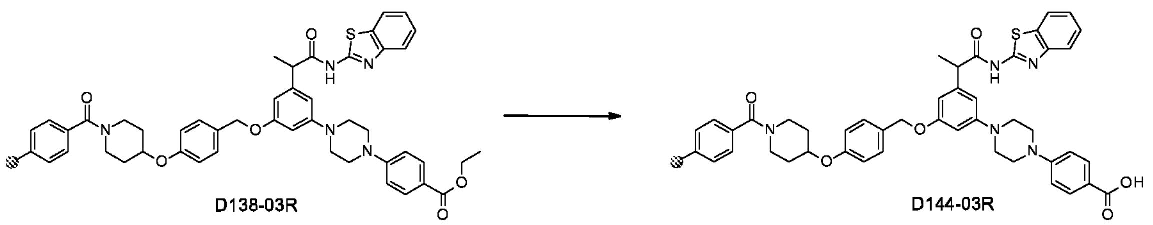

The title compound D144-03R (248.7 mg, 0.191 mmol/g) was obtained by synthesis by the same approach as in Example 12-3-1 using D138-03R (250 mg, 0.1903 mmol/g). The same cleavage operation as in Example 12-1-1 was performed, so that D144 was obtained at a purity of 89.9%.

[Formula 775]

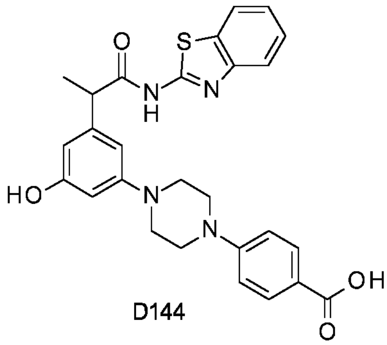

D144

Compound D144

LCMS: m/z 503$[M+H]^+$

Retention time: 0.991 min (analysis conditions SMD-FA05-1, 280 nm)

Example 12-3-6: Synthesis of D145-03R

[Formula 776]

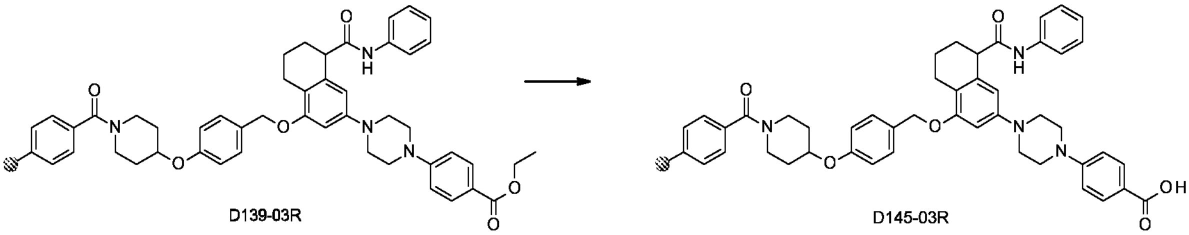

D139-03R D145-03R

The title compound D145-03R (251.8 mg, 0.193 mmol/g) was obtained by synthesis by the same approach as in Example 12-3-1 using D139-03R (250 mg, 0.192 mmol/g). The same cleavage operation as in Example 12-1-1 was performed, so that D145 was obtained at a purity of 84.4%.

[Formula 777]

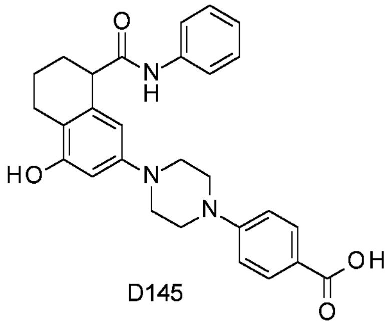

D145

Compound D145

LCMS: m/z 472$[M+H]^+$

Retention time: 0.920 min (analysis conditions SMD-FA05-1, 280 nm)

Example 12-4-1: Synthesis of D146-03R

[Formula 778]

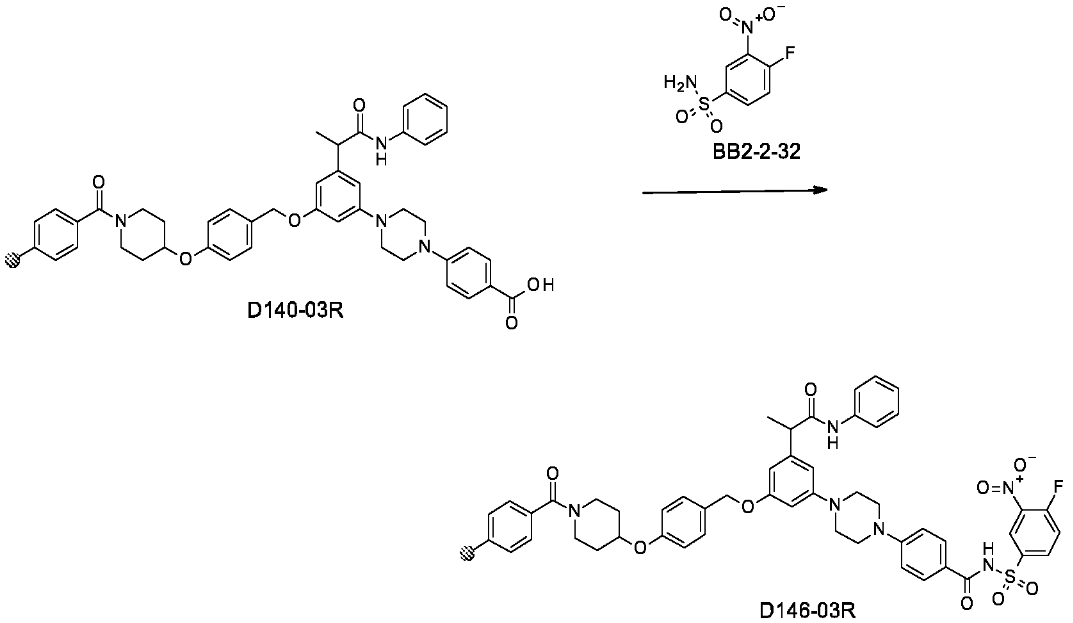

D140-03R

BB2-2-32

D146-03R

Under a nitrogen atmosphere, NMP (3.75 mL) was added to a 6 mL column with a filter containing D140-03R (250 mg, 0.1932 mmol/g) and shaken at room temperature for 1 hour. 2,6-Lutidine (33.8 μL, 0.290 mmol) and HATU (0.110 g, 0.290 mmol) were added thereto, and the mixture was shaken at 40°C for 6 hours. 4-Fluoro-3- nitrobenzenesulfonamide (BB2-2-32, 74.4 mg, 0.338 mmol) and P1tBu (0.184 mL, 0.725 mmol) were added thereto, and the mixture was shaken at room temperature for 15 hours.

Solid-phase purification

The suspension of the reaction solution and the solid phase was filtered through the filter of the column and washed three times with NMP/water = 1/1 (5 mL), three times with NMP (5 mL), three times with 0.05 M $TBAHSO_4$ + DTBP in NMP (5 mL), three times with NMP/water = 1/1 (5 mL), three times with NMP (5 mL), three times with MeOH (5 mL), three times with DCM (14 mL), and three times with heptane (5 mL). The obtained solid phase was dried under reduced pressure to obtain the title compound D146-03R (272.3 mg, 0.1859 mmol/g). The same cleavage operation as in Example 12-1-1 was performed, so that D146 was obtained at a purity of 69.4%.

[Formula 779]

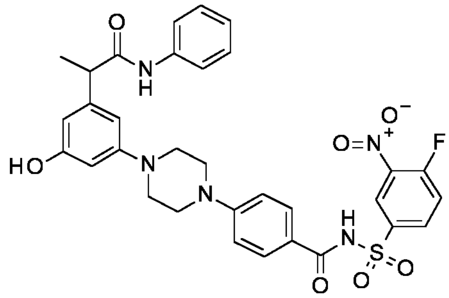

D146

Compound D146

LCMS: m/z 648$[M+H]^+$

Retention time: 1.123 min (analysis conditions SMD-FA05-1, 280 nm)

Example 12-4-2: Synthesis of D147-03R

[Formula 780]

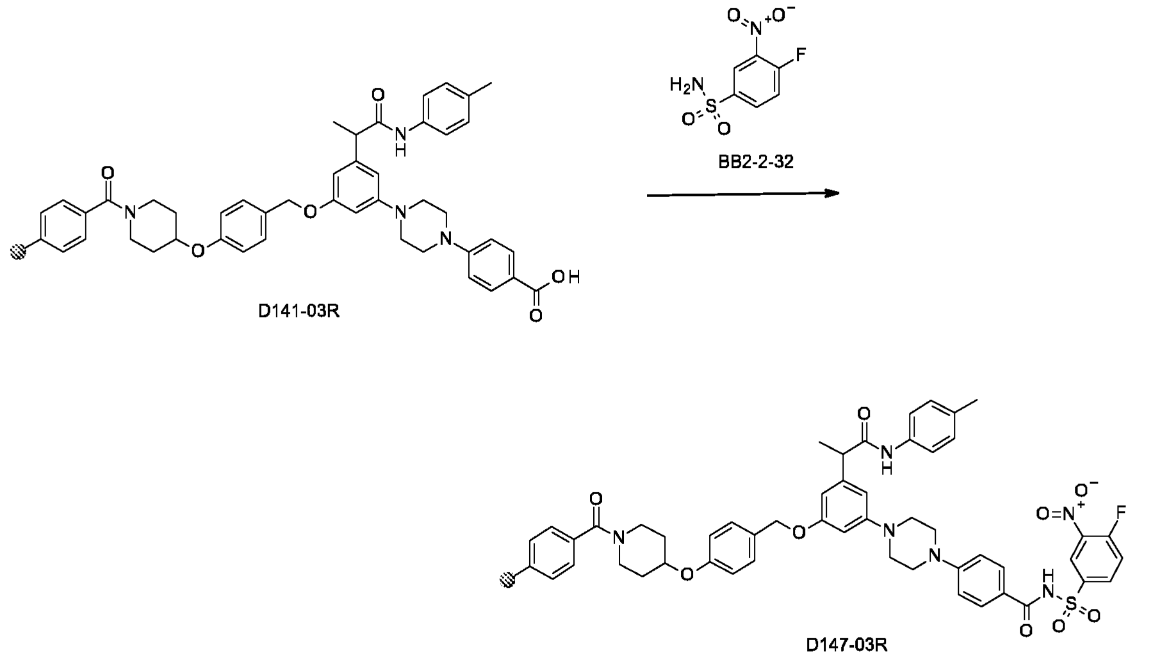

BB2-2-32

D141-03R

D147-03R

The title compound D147-03R (261.2 mg, 0.1850 mmol/g) was obtained by synthesis by the same approach as in Example 12-4-1 using D141-03R (250 mg, 0.1932 mmol/g) and 4-fluoro-3-nitrobenzenesulfonamide (BB2-2-32, 74.4 mg, 0.338 mmol). The same cleavage operation as in Example 12-1-1 was performed, so that D147 was obtained at a purity of 82.9%.

[Formula 781]

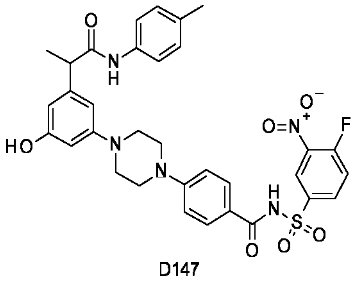

D147

Compound D147

LCMS: m/z 662[M+H]+

Retention time: 1.163 min (analysis conditions SMD-FA05-1, 280 nm)

Example 12-4-3: Synthesis of D148-03R

[Formula 782]

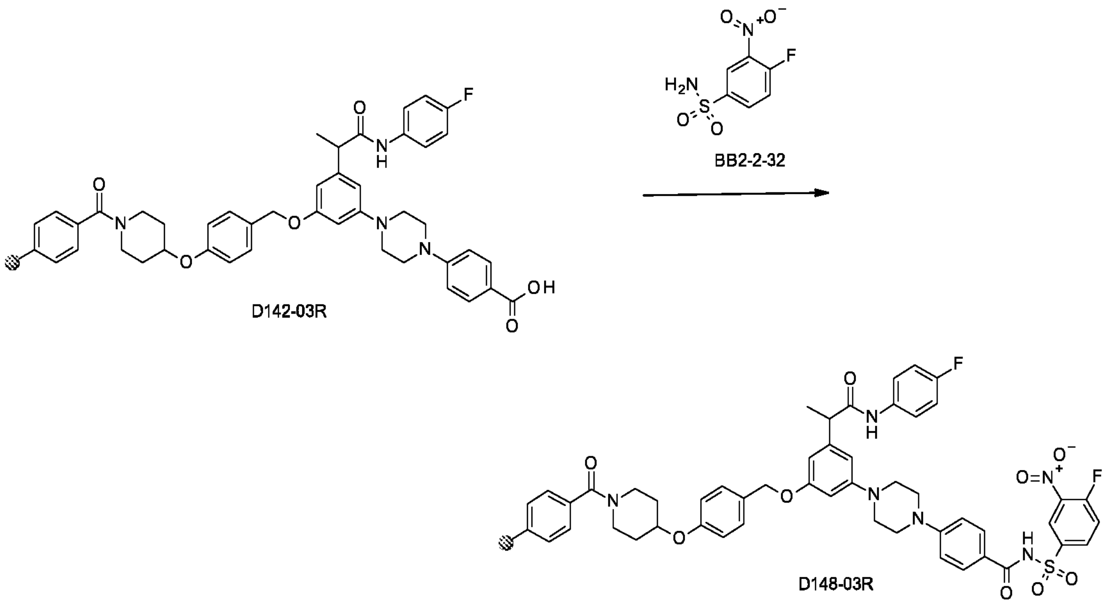


D142-03R

D148-03R

The title compound D148-03R (269.1 mg, 0.1850 mmol/g) was obtained by synthesis by the same approach as in Example 12-4-1 using D142-03R (250 mg, 0.1932 mmol/g) and 4-fluoro-3-nitrobenzenesulfonamide (BB2-2-32, 74.4 mg, 0.338 mmol). The same cleavage operation as in Example 12-1-1 was performed, so that D148 was obtained at a purity of 65.2%.

[Formula 783]

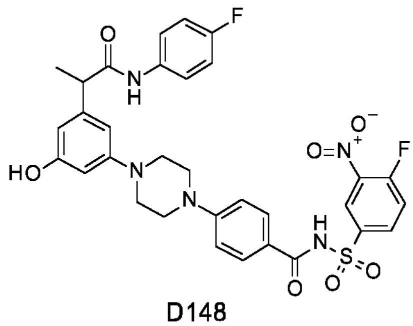

D148

Compound D148

LCMS: m/z 666[M+H]$^+$

Retention time: 1.136 min (analysis conditions SMD-FA05-1, 280 nm)

Example 12-4-4: Synthesis of D149-03R

[Formula 784]

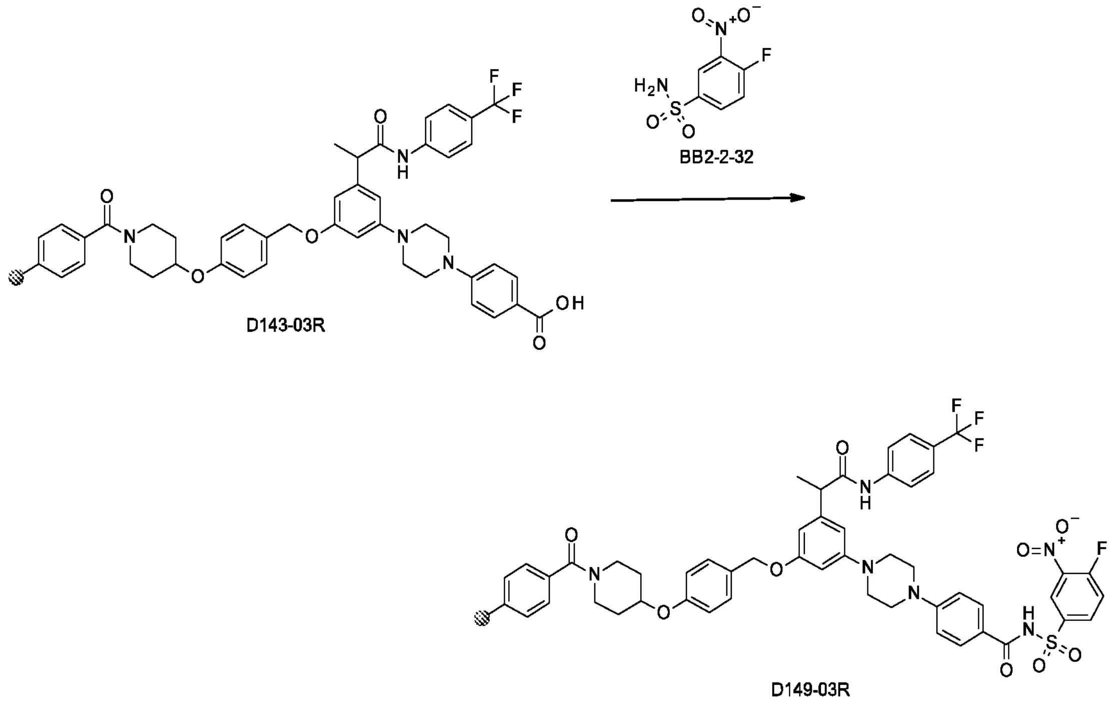

BB2-2-32

D143-03R

D149-03R

The title compound D149-03R (280.4 mg, 0.183 mmol/g) was obtained by synthesis by the same approach as in Example 12-4-1 using D143-03R (250 mg, 0.190 mmol/g) and 4- fluoro-3-nitrobenzenesulfonamide (BB2-2-32, 74.4 mg, 0.338 mmol). The same cleavage operation as in Example 12-1-1 was performed, so that D149 was obtained at a purity of 65.6%.

[Formula 785]

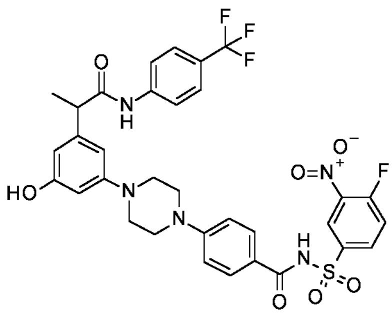

D149

Compound D149

LCMS: m/z 716[M+H]$^+$

Retention time: 1.235 min (analysis conditions SMD-FA05-1, 280 nm)

Example 12-4-5: Synthesis of D150-03R

[Formula 786]

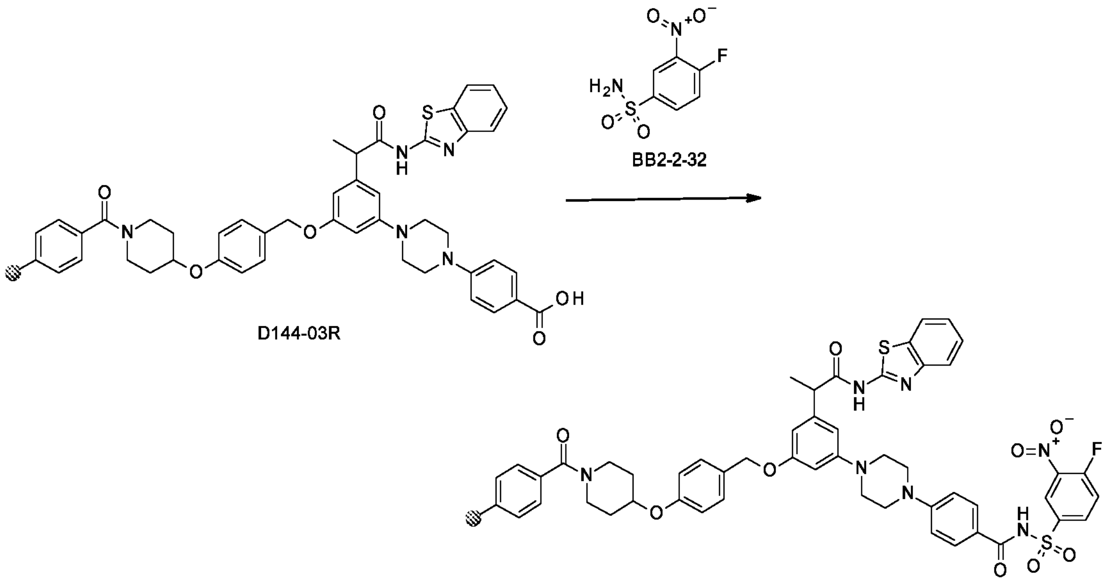

BB2-2-32

D144-03R

D150-03R

The title compound D150-03R (259.8 mg, 0.1839 mmol/g) was obtained by synthesis by the same approach as in Example 12-4-1 using D144-03R (250 mg, 0.191 mmol/g) and 4-fluoro-3-nitrobenzenesulfonamide (BB2-2-32, 74.4 mg, 0.338 mmol). The same cleavage operation as in Example 12-1-1 was performed, so that D150 was obtained at a purity of 72.8%.

[Formula 787]

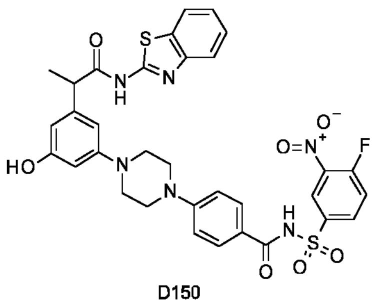

D150

Compound D150

LCMS: m/z 705[M+H]$^+$

Retention time: 1.179 min (analysis conditions SMD-FA05-1, 290 nm)

Example 12-4-6: Synthesis of D151-03R

[Formula 788]

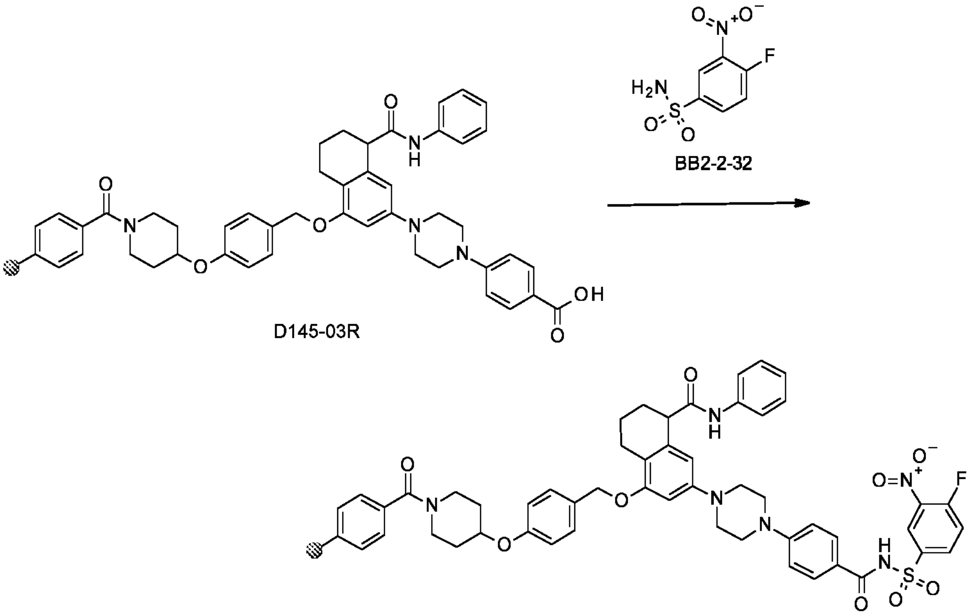

BB2-2-32

D145-03R

D151-03R

The title compound D151-03R (285.5 mg, 0.186 mmol/g) was obtained by synthesis by the same approach as in Example 12-4-1 using D145-03R (250 mg, 0.193 mmol/g) and 4- fluoro-3-nitrobenzenesulfonamide (BB2-2-32, 74.4 mg, 0.338 mmol). The same cleavage operation as in Example 12-1-1 was performed, so that D151 was obtained at a purity of 83.8%.

[Formula 789]

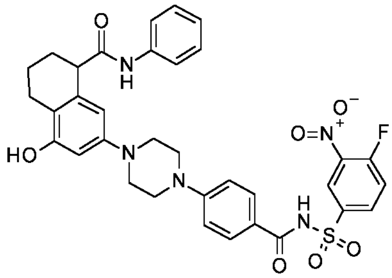

D151

Compound D151

LCMS: m/z 674[M+H]$^+$

Retention time: 1.132 min (analysis conditions SMD-FA05-1, 280 nm)

Example 12-5-1: Synthesis of D152-03R

[Formula 790]

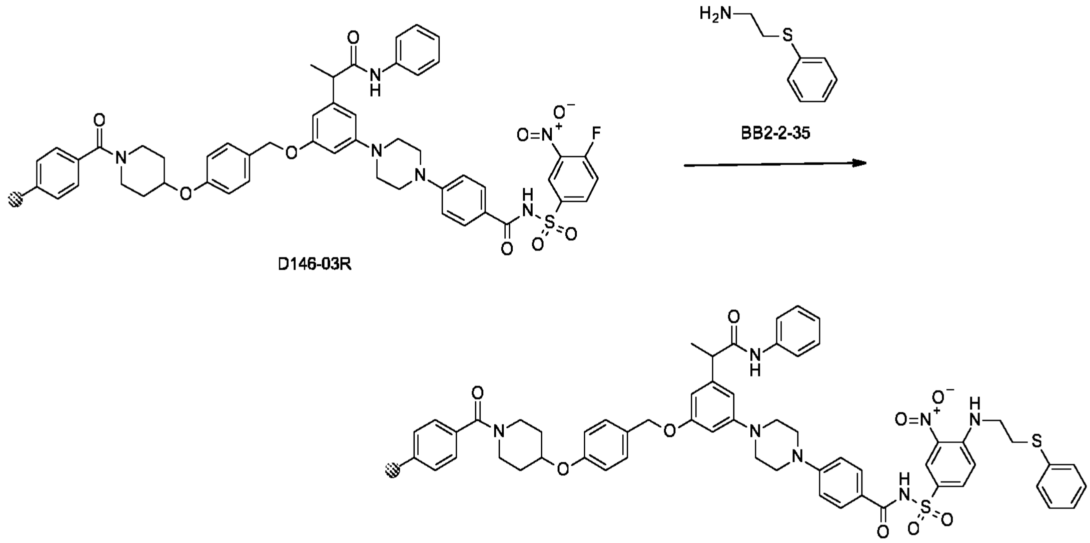


D146-03R

D152-03R

Under a nitrogen atmosphere, NMP (2.88 mL) was added to a 6 mL column with a filter containing D146-03R (240 mg, 0.1859 mmol/g) and shaken at room temperature for 1 hour. 2-Phenylsulfanylethanamine (BB2-2-35, 73.6 mg, 0.480 mmol) and 1,8- bis(dimethylamino)naphthalene (154 mg, 0.720 mmol) were added thereto, and the mixture was shaken at 60°C for 15 hours.

Solid-phase purification

The suspension of the reaction solution and the solid phase was transferred to a column with a filter, filtered, and washed three times with NMP/water = 1/1 (5 mL), three times with NMP (5 mL), three times with 0.05 M $TBAHSO_4$ + DTBP in NMP (5 mL), three times with NMP/water = 1/1 (5 mL), three times with NMP (5 mL), three times with MeOH (5 mL), three times with DCM (5 mL), and three times with heptane (5 mL). The obtained solid phase was dried under reduced pressure to obtain the title compound D152-03R (251.3 mg, 0.181 mmol/g). The same cleavage operation as in Example 12-1-1 was performed, so that D 152 was obtained at a purity of 81.1%.

[Formula 791]

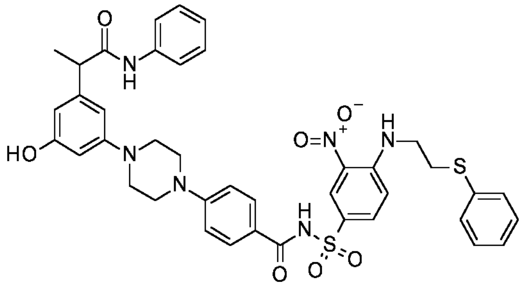

D152

Compound D152
LCMS: m/z 781$[M+H]^+$
Retention time: 1.265 min (analysis conditions SMD-FA05-1, 280 nm)

Example 12-5-2: Synthesis of D153-03R

[Formula 792]

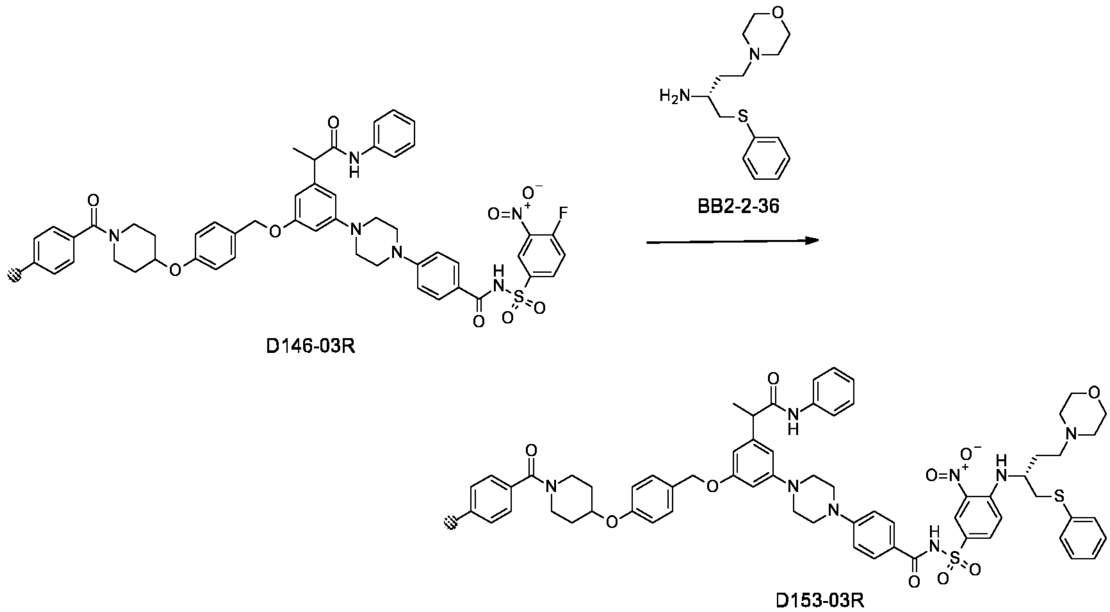

D146-03R

BB2-2-36

D153-03R

The title compound D153-03R (258.5 mg, 0.178 mmol/g) was obtained by synthesis by the same approach as in Example 12-5-1 using D146-03R (250 mg, 0.1859 mmol/g) and (2R)-4-morpholin-4-yl-1-phenylsulfanylbutan-2-amine (BB2-2-36, 128 mg, 0.480 mmol). The same cleavage operation as in Example 12-1-1 was performed, so that D153 was obtained at a purity of 80.9%.

[Formula 793]

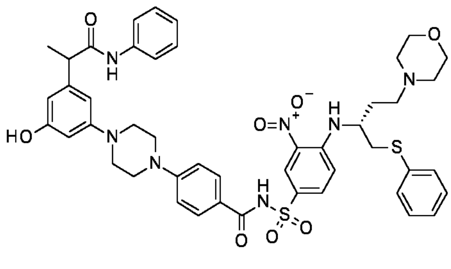

D153

Compound D153

LCMS: m/z 894$[M+H]^+$

Retention time: 0.921 min (analysis conditions SMD-FA05-1, 280 nm)

Example 12-5-3: Synthesis of D154-03R

[Formula 794]

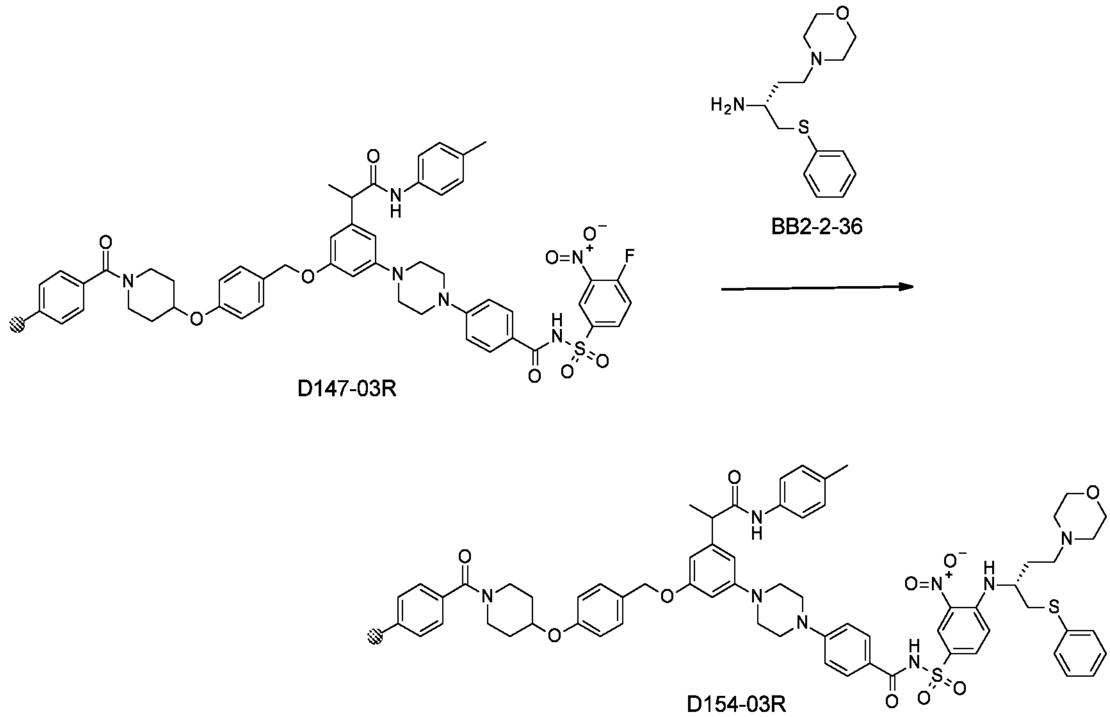


The title compound D154-03R (248.2 mg, 0.177 mmol/g) was obtained by synthesis by the same approach as in Example 12-5-1 using D147-03R (250 mg, 0.1850 mmol/g) and (2R)-4-morpholin-4-yl-1-phenylsulfanylbutan-2-amine (BB2-2-36, 128 mg, 0.480 mmol). The same cleavage operation as in Example 12-1-1 was performed, so that D154 was obtained at a purity of 77.1%.

[Formula 795]

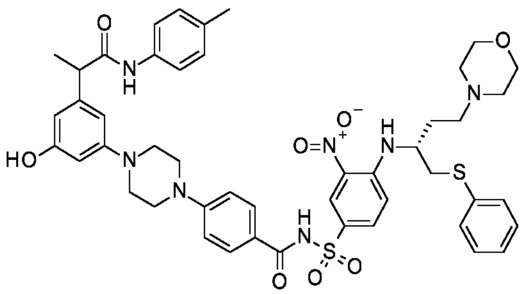

D154

Compound D154
LCMS: m/z 908[M+H]+
Retention time: 0.939 min (analysis conditions SMD-FA05-1, 290 nm)

Example 12-5-4: Synthesis of D155-03R

[Formula 796]

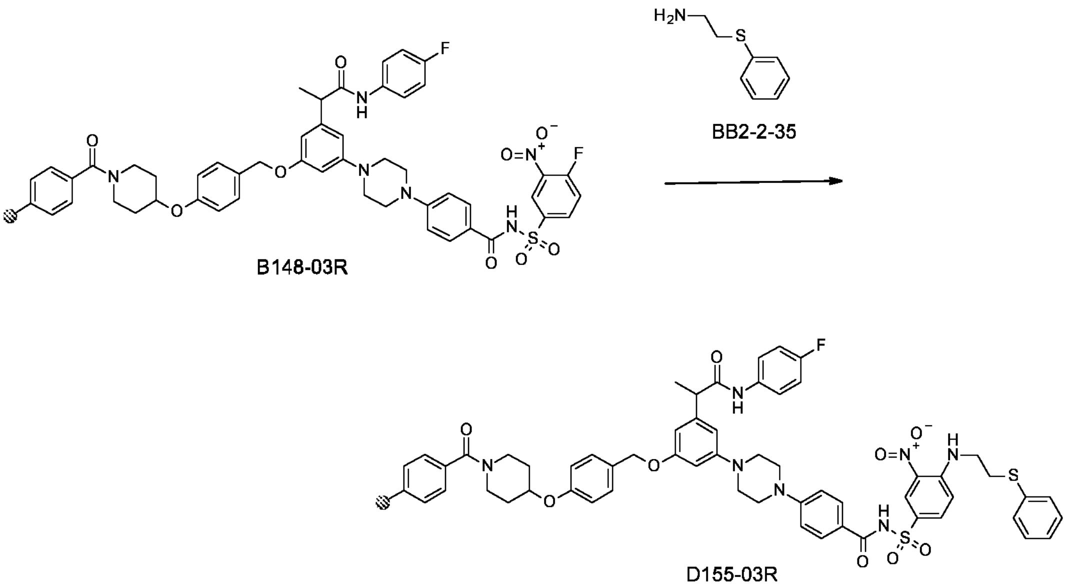

BB2-2-35

B148-03R

D155-03R

The title compound D155-03R (255.4 mg, 0.181 mmol/g) was obtained by synthesis by the same approach as in Example 12-5-1 using D148-03R (250 mg, 0.1850 mmol/g) and 2-phenylsulfanylethanamine (BB2-2-35, 73.6 mg, 0.480 mmol). The same cleavage operation as in Example 12-1-1 was performed, so that D155 was obtained at a purity of 75.5%.

[Formula 797]

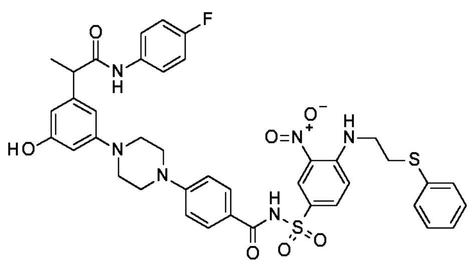

D155

Compound D155

LCMS: m/z 799[M+H]$^+$

Retention time: 1.279 min (analysis conditions SMD-FA05-1, 280 nm)

Example 12-5-5: Synthesis of D156-03R

[Formula 798]

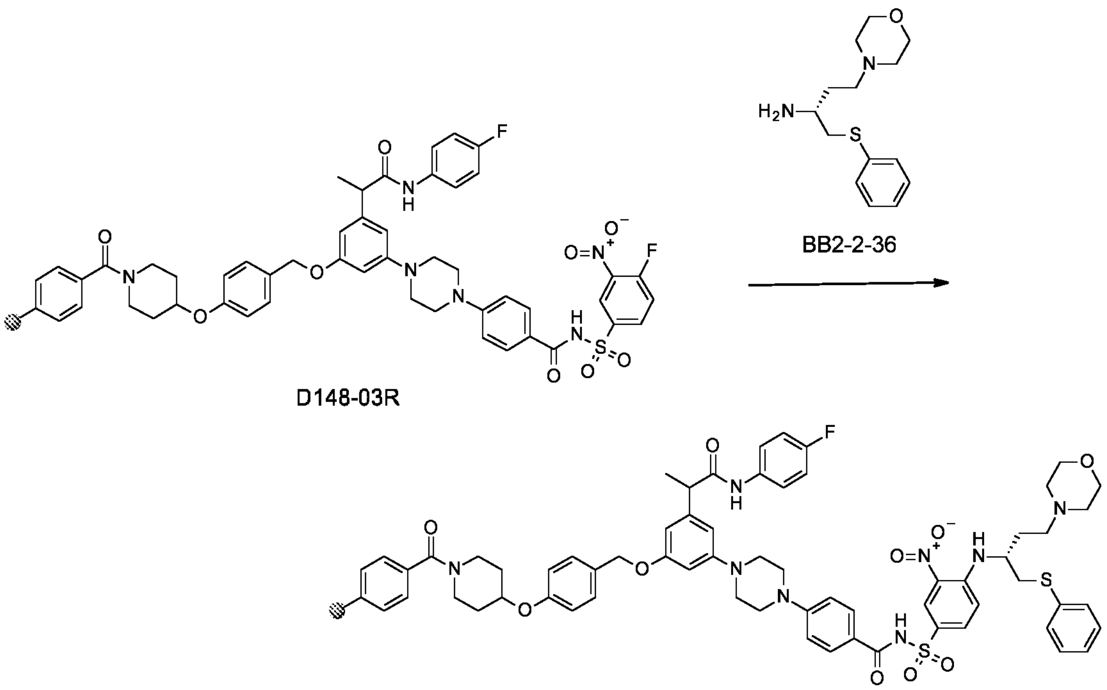

BB2-2-36

D148-03R

D156-03R

The title compound D156-03R (261.7 mg, 0.177 mmol/g) was obtained by synthesis by the same approach as in Example 12-5-1 using D148-03R (240 mg, 0.177 mmol/g) and (2R)-4-morpholin-4-yl-1-phenylsulfanylbutan-2-amine (BB2-2-36, 128 mg, 0.480 mmol). The same cleavage operation as in Example 12-1-1 was performed, so that D156 was obtained at a purity of 73.1%.

[Formula 799]

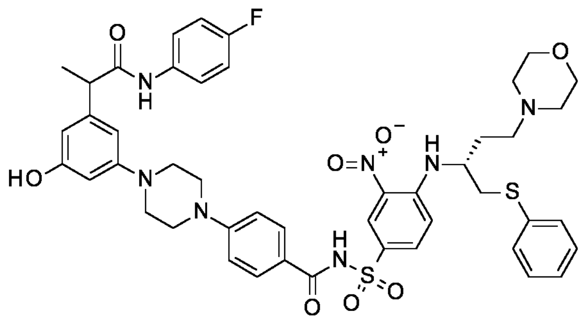

D156

Compound D156

LCMS: m/z 912$[M+H]^+$

Retention time: 0.927 min (analysis conditions SMD-FA05-1, 280 nm)

Example 12-5-6: Synthesis of D157-03R

[Formula 800]

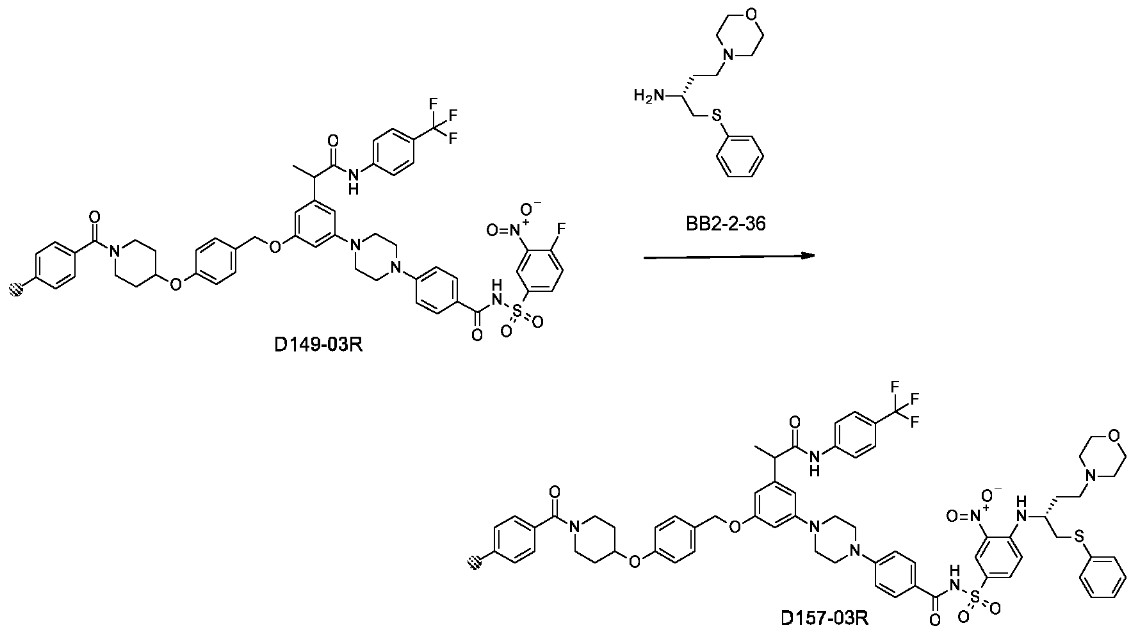


D149-03R

D157-03R

The title compound D157-03R (259.5 mg, 0.175 mmol/g) was obtained by synthesis by the same approach as in Example 12-5-1 using D149-03R (240 mg, 0.183 mmol/g) and (2R)-4-morpholin-4-yl-1-phenylsulfanylbutan-2-amine (BB2-2-36, 128 mg, 0.480 mmol). The same cleavage operation as in Example 12-1-1 was performed, so that D157 was obtained at a purity of 61.9%.

[Formula 801]

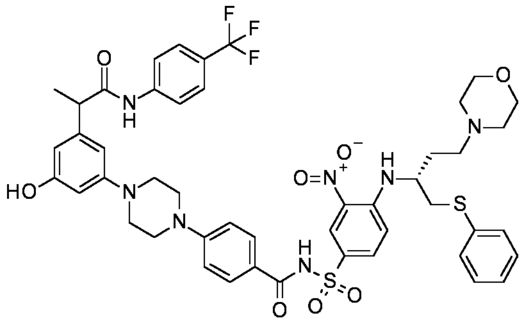

D157

Compound D157

LCMS: m/z 962[M+H]+

Retention time: 0.999 min (analysis conditions SMD-FA05-1, 280 nm)

Example 12-5-7: Synthesis of D158-03R

[Formula 802]

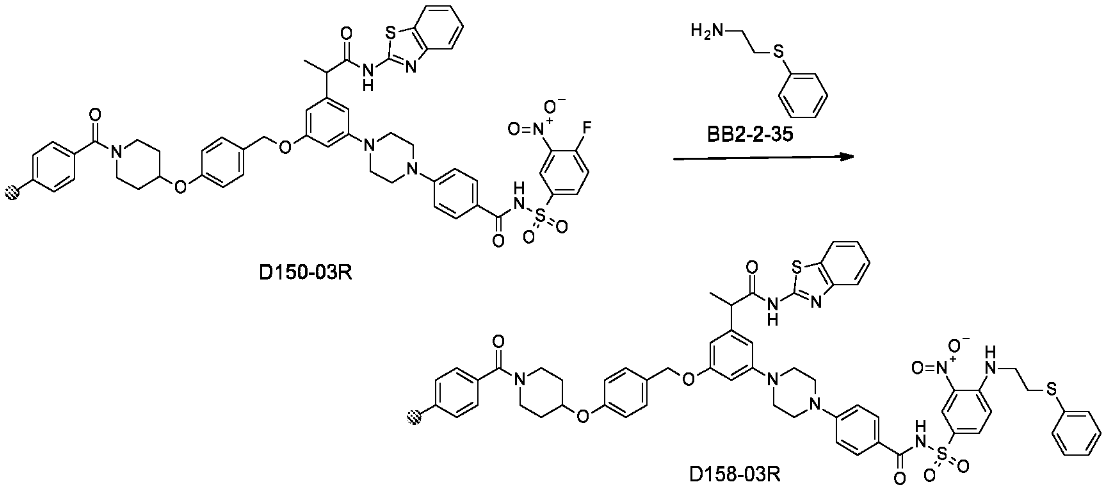


D150-03R

D158-03R

The title compound D158-03R (240.3 mg, 0.176 mmol/g) was obtained by synthesis by the same approach as in Example 12-5-1 using D150-03R (240 mg, 0.191 mmol/g) and 2- phenylsulfanylethanamine (BB2-2-35, 73.6 mg, 0.480 mmol). The same cleavage operation as in Example 12-1-1 was performed, so that D158 was obtained at a purity of 74.3%.

[Formula 803]

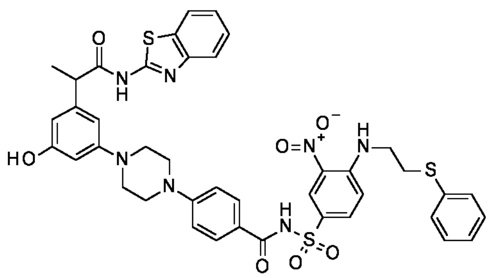

D158

Compound D158

LCMS: m/z 838[M+H]+

Retention time: 1.315 min (analysis conditions SMD-FA05-1, 280 nm)

Example 12-5-8: Synthesis of D159-03R

[Formula 804]

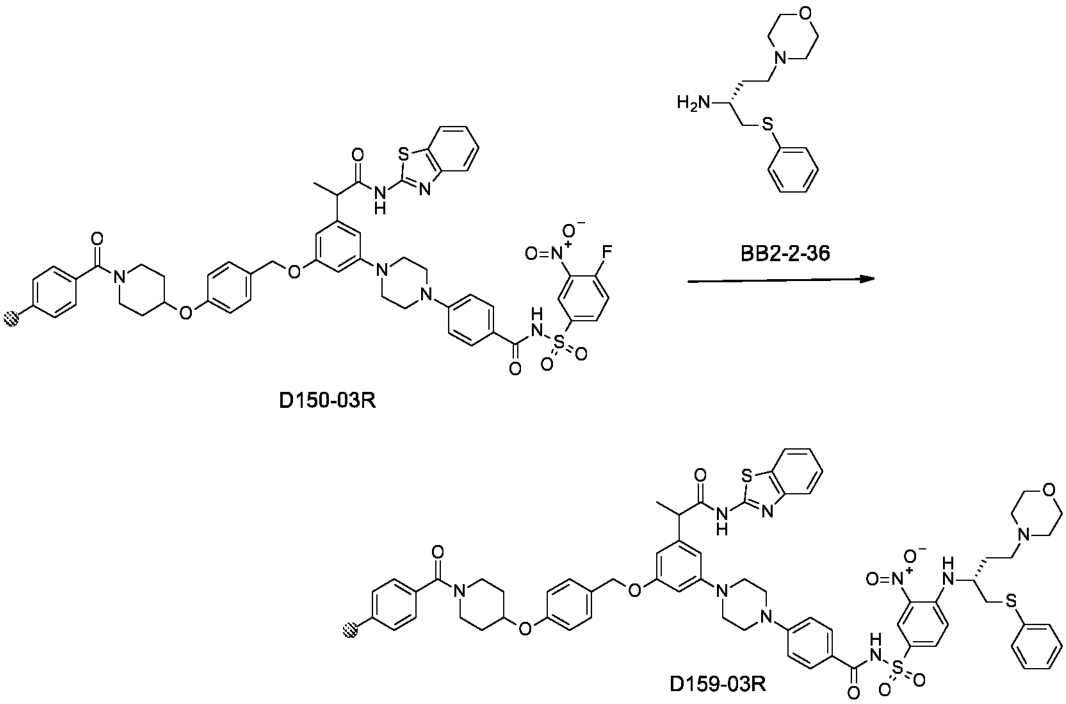


D150-03R

D159-03R

The title compound D159-03R (259.5 mg, 0.175 mmol/g) was obtained by synthesis by the same approach as in Example 12-5-1 using D150-03R (240 mg, 0.191 mmol/g) and (2R)-4-morpholin-4-yl-1-phenylsulfanylbutan-2-amin (BB2-2-36, 128 mg, 0.480 mmol). The same cleavage operation as in Example 12-1-1 was performed, so that D159 was obtained at a purity of 74.5%.

[Formula 805]

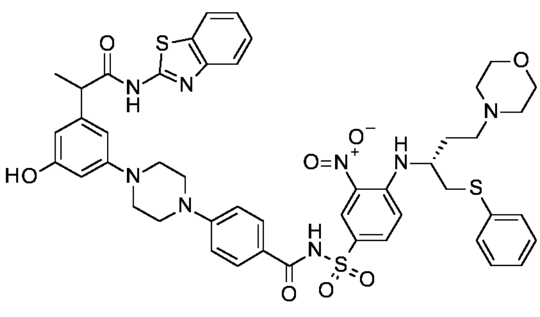

D159

Compound D159

LCMS: m/z 951[M+H]+

Retention time: 0.973 min (analysis conditions SMD-FA05-1, 280 nm)

Example 12-5-9: Synthesis of D160-03R

[Formula 806]

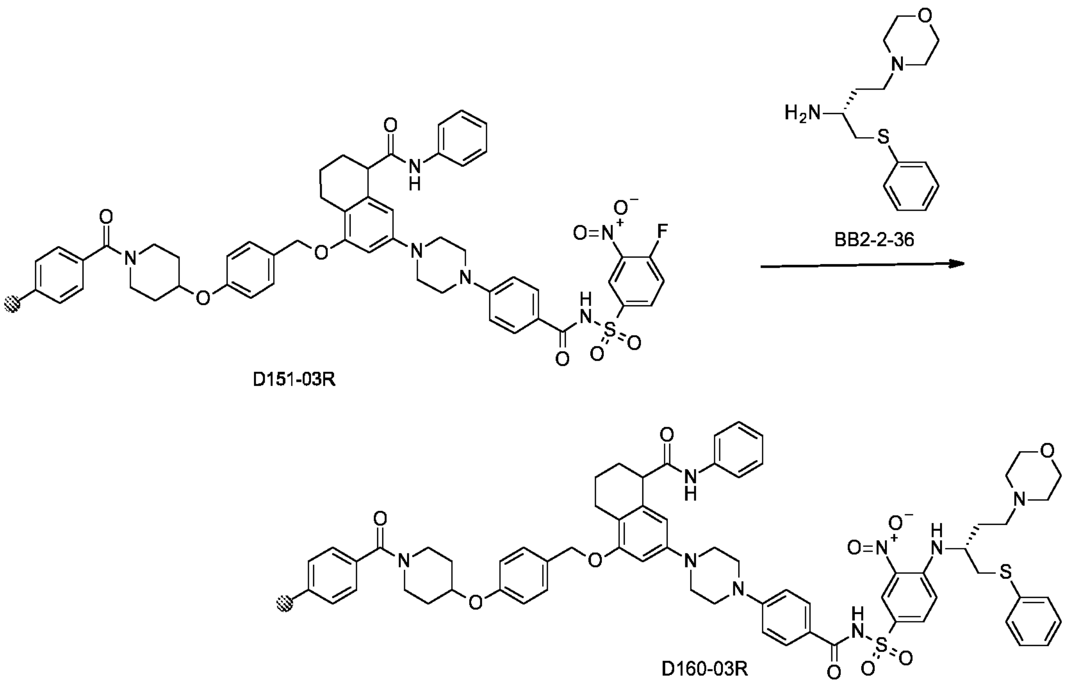


D151-03R

D160-03R

The title compound D160-03R (246.0 mg, 0.178 mmol/g) was obtained by synthesis by the same approach as in Example 12-5-1 using D151-03R (243.4 mg, 0.186 mmol/g) and (2R)-4-morpholin-4-yl-1-phenylsulfanylbutan-2-amine (BB2-2-36, 121 mg, 0.453 mmol). The same cleavage operation as in Example 12-1-1 was performed, so that D160 was obtained at a purity of 83.3%.

[Formula 807]

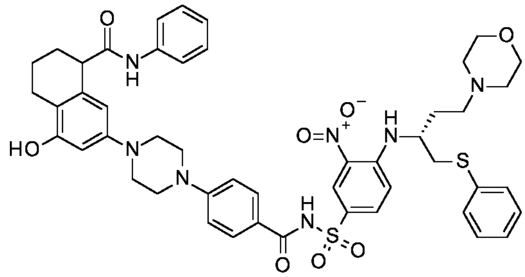

D160

Compound D160

LCMS: m/z 920[M+H]+

Retention time: 0.937 min (analysis conditions SMD-FA05-1, 280 nm)

Example 12-6-1: Synthesis of D152

[Formula 808]

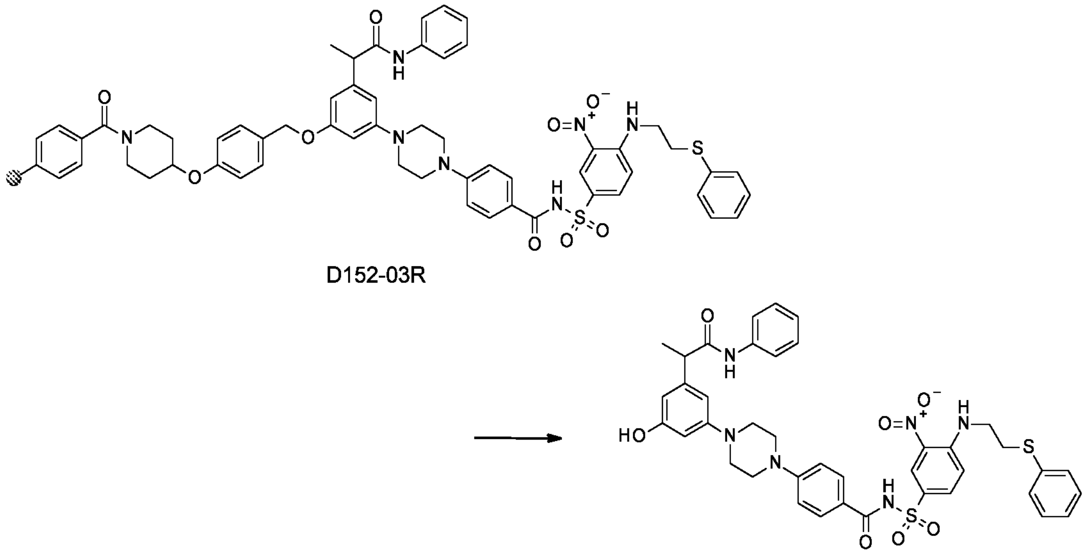

D152-03R

D152

Under a nitrogen atmosphere, D152-03R (247.8 mg, 0.181 mmol/g) and 0.08 M pentamethylbenzene in DCM (4.96 mL) were added to an 8 mL glass vial and shaken at room temperature for 1 hour. TFA (0.496 mL, 6.43 mmol) was added thereto, and the mixture was shaken at room temperature for 1.5 hours. The suspension of the reaction solution and the solid phase was transferred to a 6 mL column with a filter using DMF (1 mL) twice and filtered. The resultant was washed three times with DMF (1.8 mL), and the solvent in combined filtrates was distilled off by Genevac. The residue after concentration was dissolved in DMSO (1.9 mL), and water (95 μL) and formic acid (34.4 μL) were then added to the solution. The mixture was purified by preparative HPLC (C18, 0.1% solution of FA in acetonitrile/0.1% aqueous FA solution, 20-100%) and then freeze-dried to obtain the title compound D152 (4.4 mg, 5.6 μmol, 12.6%) as a yellow powder.

[Formula 809]

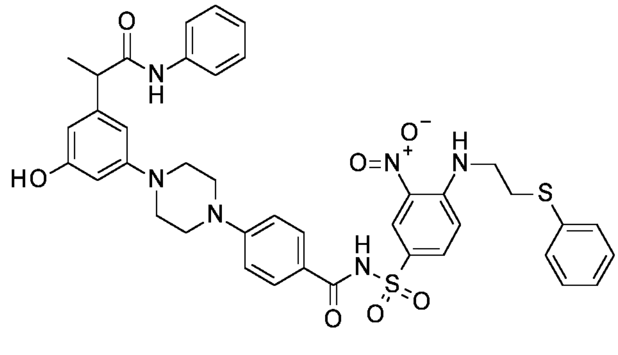

D152

Compound D152

$^1$H NMR (400 MHz, DMSO-d6) δ: 9.92 (1H, s), 9.16 (1H, s), 8.73 (1H, t, J = 10.0 Hz), 8.59 (1H, d, J = 2.1 Hz), 7.91-7.90 (1H, m), 7.77 (2H, d, J = 8.8 Hz), 7.58 (2H, d, J = 8.0 Hz), 7.37 (2H, d, J = 7.6 Hz), 7.27 (4H, t, J = 7.8 Hz), 7.18-7.16 (2H, m), 7.02-6.98 (3H, m), 6.50 (1H, d, J = 13.2 Hz), 6.30-6.22 (2H, m), 3.66-3.65 (3H, m), 3.45-3.43 (4H, br m), 3.44-3.26 (0H, m), 3.44 (4H, s), 3.29-3.27 (2H, m), 3.20-3.19 (4H, m), 1.35 (3H, d, J = 6.8 Hz).

LCMS: m/z 781 $[M+H]^+$.

Retention time: 1.267 min (analysis conditions SMD-FA05-1, 299 nm).

Example 12-6-2: Synthesis of D153

[Formula 810]

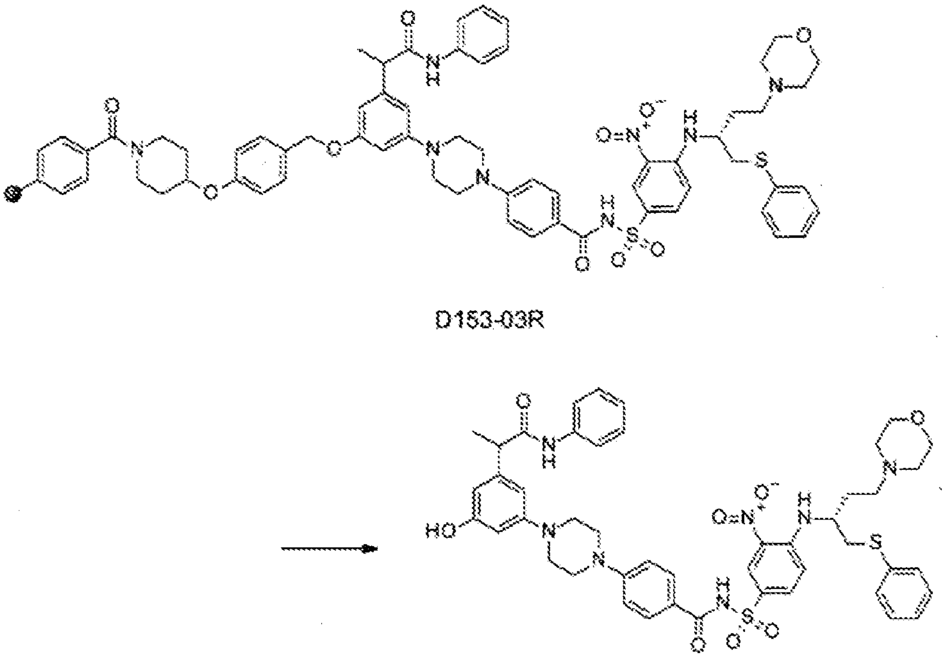

D153-03R

D153

The title compound D153 (11.0 mg, 0.012 mmol, 27%) was obtained by synthesis by the same approach as in Example 12-6-1 using D153-03R (253.7 mg, 0.178 mmol/g).

[Formula 811]

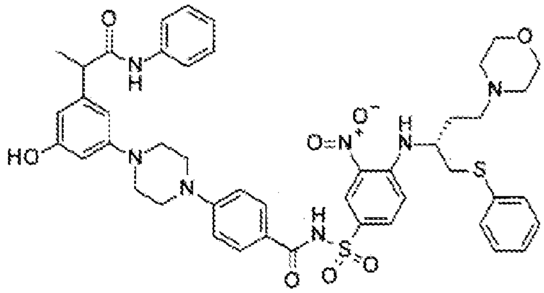

D153

Compound D153

$^1$H NMR (400 MHz., DMSO-d6) δ: 9.91 (1H, s), 9.15 (1H, s), 8.52 (1H, d, J = 2.0 Hz), 8.37 (1H, s), 7.82 (1H, d, J = 9.1 Hz), 7.77 (2H, d, J = 8.8 Hz), 7.58 (2H, d, J = 7.9 Hz), 7.29-6.95 (10H, m), 6.51-6.48 (2H, m), 6.30-6.22 (2H, m), 4.15 (1H, s), 3.65 (1H, q, J = 6.8 Hz), 3.55-2.50 (17H, m), 2.18 (2H, t, J = 8.1 Hz), 2.03-2.00 (1H, br m), 1.91 (2H, dt, J = 22.5, 7.5 Hz), 1.35 (3H, d, J = 7.0 Hz).

LCMS: m/z 894 $[M+H]^+$.

Retention time: 0.923 min (analysis conditions SMD-FA05-1, 280 nm).

Example 12-6-3: Synthesis of D154

[Formula 812]

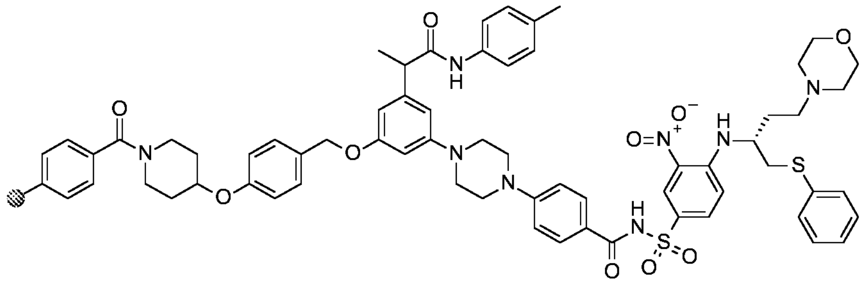

D154-03R

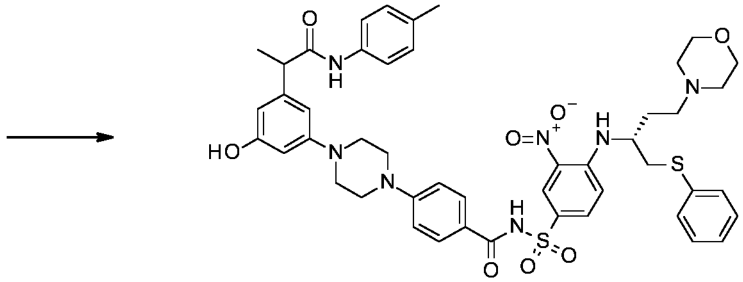

D154

The title compound D154 (7.3 mg, 8.0 μmol, 19%) was obtained by synthesis by the same approach as in Example 12-6-1 using D154-03R (244.6 mg, 0.177 mmol/g).

[Formula 813]

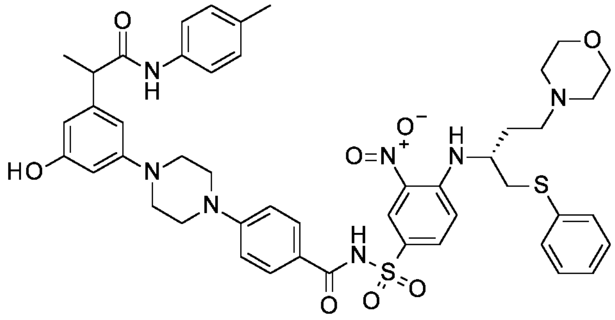

D154

Compound D154

$^1H$ NMR (400 MHz, DMSO-d6) δ: 9.82 (1H, s), 9.14 (1H, s), 8.49 (1H, s), 8.34 (1H, s), 8.35-8.32 (1H, m), 7.80-7.77 (3H, m), 7.46 (2H, d, J = 8.4 Hz), 7.27-7.17 (5H, m), 7.08- 6.92 (5H, m), 6.49-6.46 (2H, m), 6.29-6.21 (2H, m), 4.12 (1H, s), 3.62 (1H, q, J = 7.0 Hz), 3.54-3.51 (5H, br m), 3.33-1.23 (22H, m).

LCMS: m/z 908 $[M+H]^+$.

Retention time: 0.928 min (analysis conditions SMD-FA05-1, 299 nm).

Example 12-6-4: Synthesis of D155

[Formula 814]

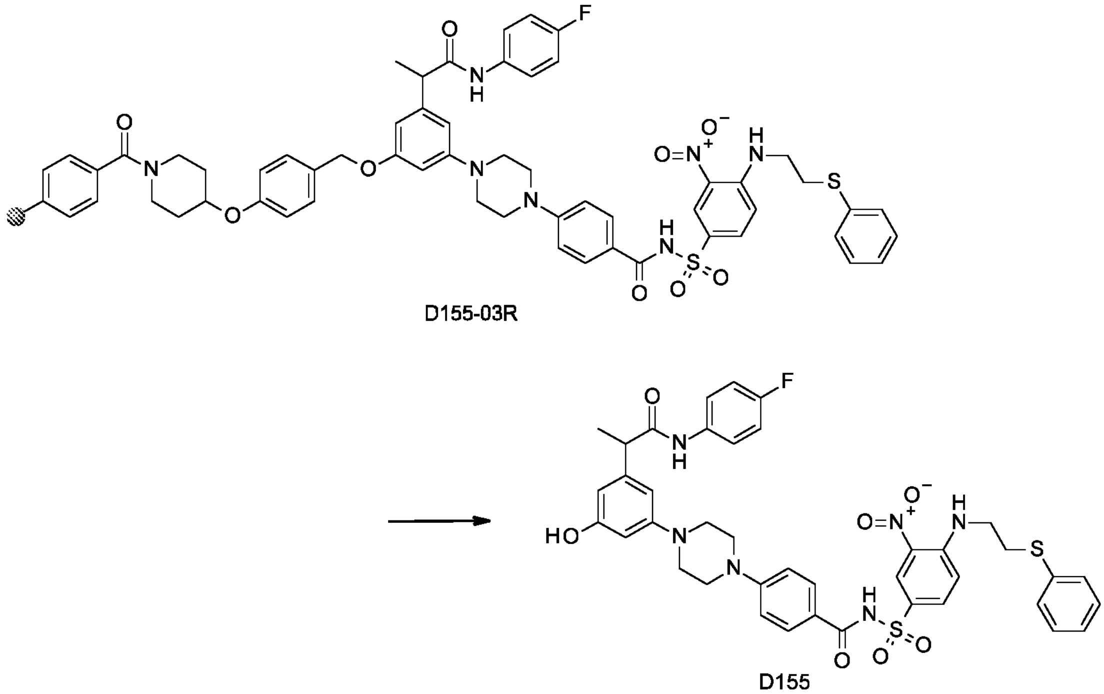

D155-03R

D155

The title compound D155 (2.1 mg, 2.6 μmol, 5.7%) was obtained by synthesis by the same approach as in Example 12-6-1 using D155-03R (255.4 mg, 0.181 mmol/g).

[Formula 815]

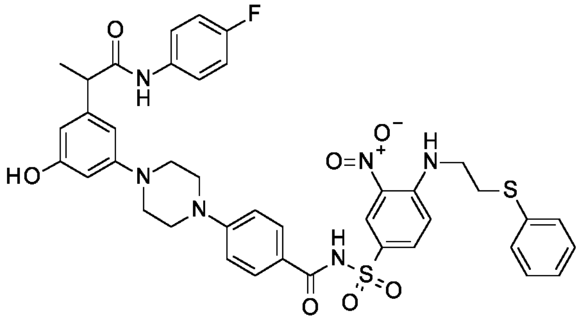

D155

Compound D155

$^1$H NMR (400 MHz, DMSO-d6) δ: 9.98 (1H, s), 9.16 (1H, s), 8.64 (1H, s), 8.55 (1H, d, J = 2.0 Hz), 7.89 (1H, dd, J = 9.1, 2.0 Hz), 7.77 (2H, d, J = 8.9 Hz), 7.60 (2H, dd, J = 9.1, 5.1 Hz), 7.38 (2H, d, J = 7.4 Hz), 7.28 (3H, t, J = 7.6 Hz), 7.18 (1H, t, J = 7.3 Hz), 7.13-7.09 (3H, m), 6.94 (2H, d, J = 8.8 Hz), 6.93 (2H, s), 6.51-6.47 (2H, m), 6.28-6.22 (1H, m), 3.64- 3.62 (3H, m), 3.27 (4H, t, J = 6.9 Hz), 3.20 (4H, s), 1.34 (3H, d, J = 7.0 Hz).

LCMS: m/z 799 [M+H]$^+$

Retention time: 1.277 min (analysis conditions SMD-FA05-1, 299 nm)

Example 12-6-5: Synthesis of D156

[Formula 816]

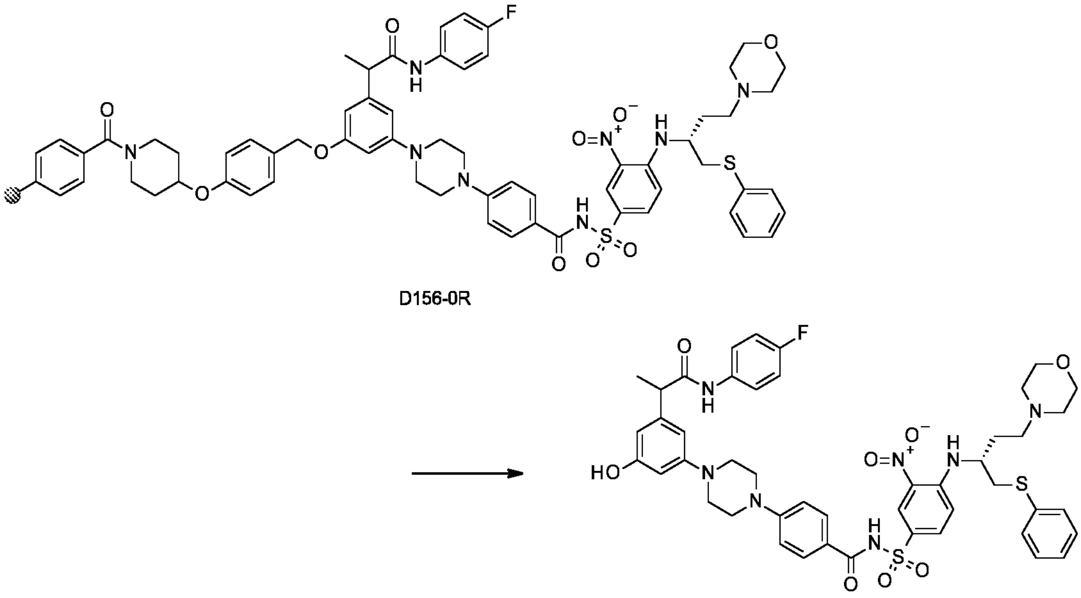

D156-0R

D156

The title compound D156 (8.6 mg, 9.4 μmol, 22%) was obtained by synthesis by the same approach as in Example 12-6-1 using D156-03R (239.5 mg, 0.177 mmol/g).

[Formula 817]

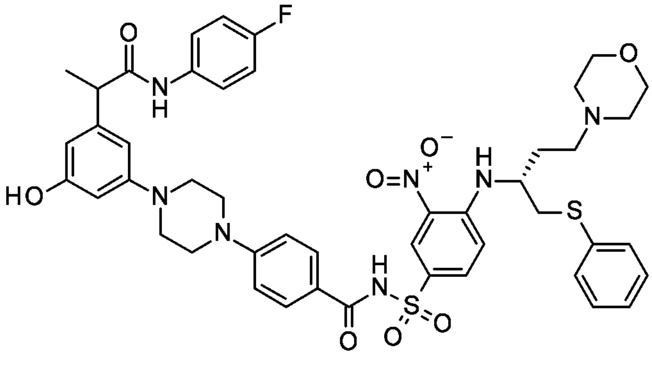

D156

Compound D156

$^1$H NMR (400 MHz, DMSO-d6) δ: 9.99 (1H, s), 9.17 (1H, s), 8.46 (1H, d, J = 2.1 Hz), 8.30 (1H, d, J = 8.9 Hz), 7.77 (3H, d, J = 8.9 Hz), 7.60 (2H, dd, J = 9.2, 5.0 Hz), 7.33-7.09 (7H, m), 6.92-6.88 (3H, m), 6.50 (2H, d, J = 15.8 Hz), 6.26 (2H, d, J = 23.2 Hz), 4.10-4.07 (1H, m), 3.63 (1H, q, J = 6.8 Hz), 3.47-3.25 (14H, m), 2.34-2.25 (6H, m), 2.00- 1.98 (1H, m), 1.82-1.79 (1H, m), 1.35 (3H, d, J = 6.8 Hz).

LCMS: m/z 912 $[M+H]^+$

Retention time: 0.933 min (analysis conditions SMD-FA05-1, 299 nm)

Example 12-6-6: Synthesis of D157

[Formula 818]

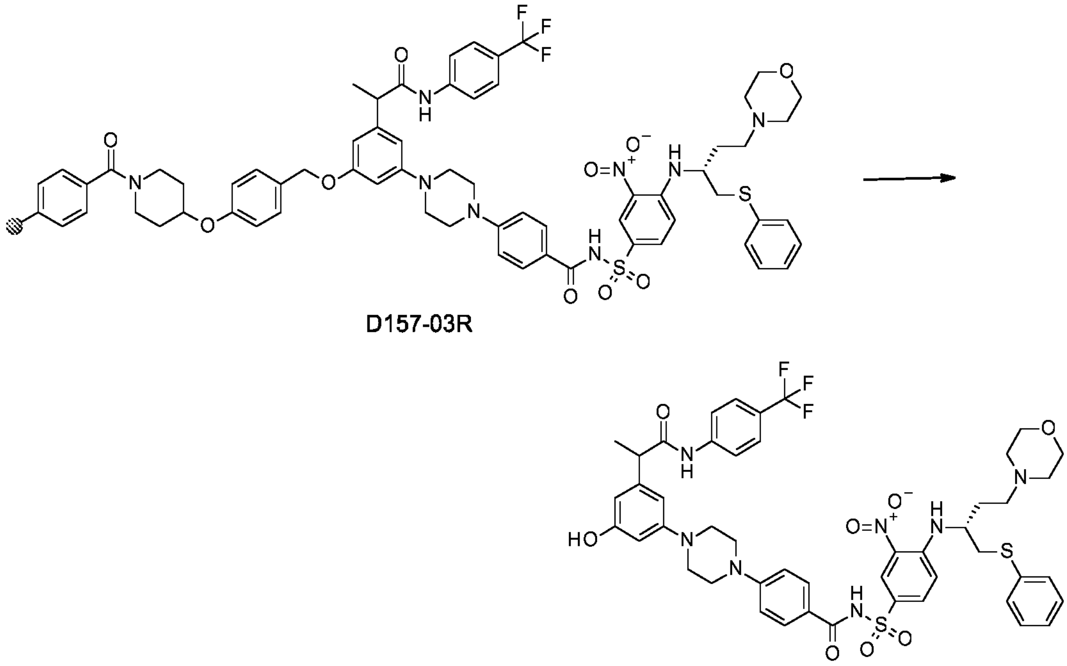

D157-03R

D157

The title compound D157 (4.9 mg, 5.1 μmol, 11%) was obtained by synthesis by the same approach as in Example 12-6-1 using D157-03R (254.8 mg, 0.175 mmol/g).

[Formula 819]

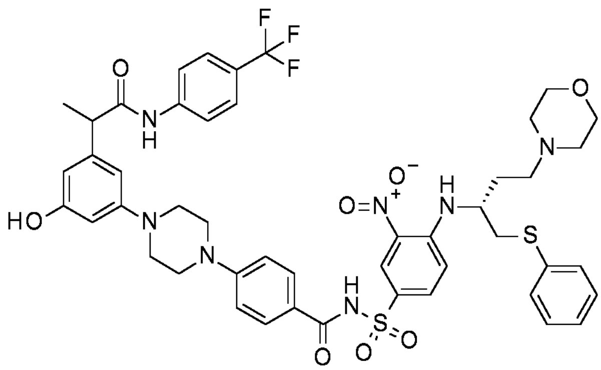

D157

Compound D157

$^1$H NMR (400 MHz, DMSO-d6) δ: 10.29 (1H, s), 9.17 (1H, s), 8.49 (1H, s), 8.35 (1H, s), 7.80-7.77 (5H, m), 7.64 (2H, d, J = 8.7 Hz), 7.31-7.14 (5H, m), 7.00-6.91 (3H, m), 6.49 (2H, d, J = 12.4 Hz), 6.25 (2H, d, J = 20.9 Hz), 4.13 (1H, s), 3.68 (1H, q, J = 7.1 Hz), 3.53-3.50 (13H, m), 3.21-3.19 (7H, m), 2.01-1.98 (1H, m), 1.85-1.83 (1H, m), 1.36 (3H, d, J = 7.0 Hz).

LCMS: m/z 962 [M+H]$^+$

Retention time: 1.000 min (analysis conditions SMD-FA05-1, 299 nm)

Example 12-6-7: Synthesis of D158

[Formula 820]

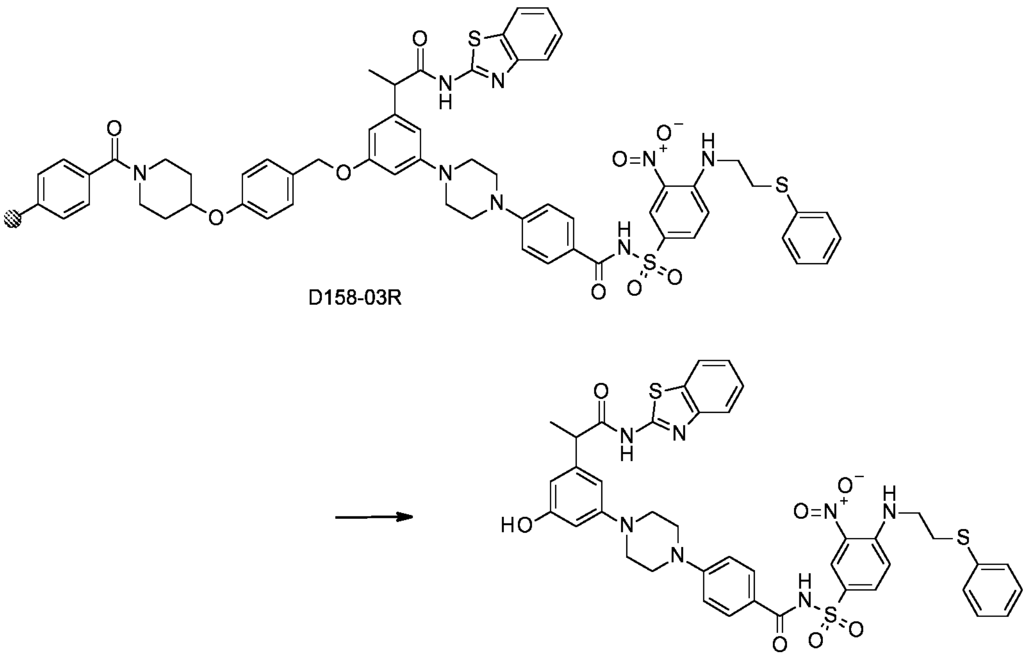

D158-03R

D158

The title compound D158 (7.0 mg, 8.4 μmol, 18%) was obtained by synthesis by the same approach as in Example 12-6-1 using D158-03R (255.4 mg, 0.180 mmol/g).

[Formula 821]

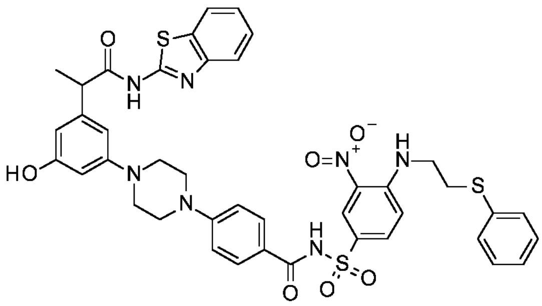

**D158**

Compound D158

$^1$H NMR (400 MHz, DMSO-d6) δ: 12.42 (1H, s), 12.04 (1H, s), 9.22 (1H, s), 8.71 (1H, s), 8.57 (1H, s), 7.97-7.89 (2H, m), 7.78-7.71 (3H, m), 7.44-7.13 (9H, m), 6.97 (2H, d, J = 8.9 Hz), 6.26 (2H, d, J = 18.4 Hz), 3.86 (1H, q, J = 6.7 Hz), 3.65 (2H, q, J = 6.5 Hz), 3.45- 3.42 (5H, m), 3.21-3.05 (5H, m), 1.41 (3H, d, J = 6.8 Hz).

LCMS: m/z 838 $[M+H]^+$

Retention time: 1.316 min (analysis conditions SMD-FA05-1, 299 nm)

Example 12-6-8: Synthesis of D159

[Formula 822]

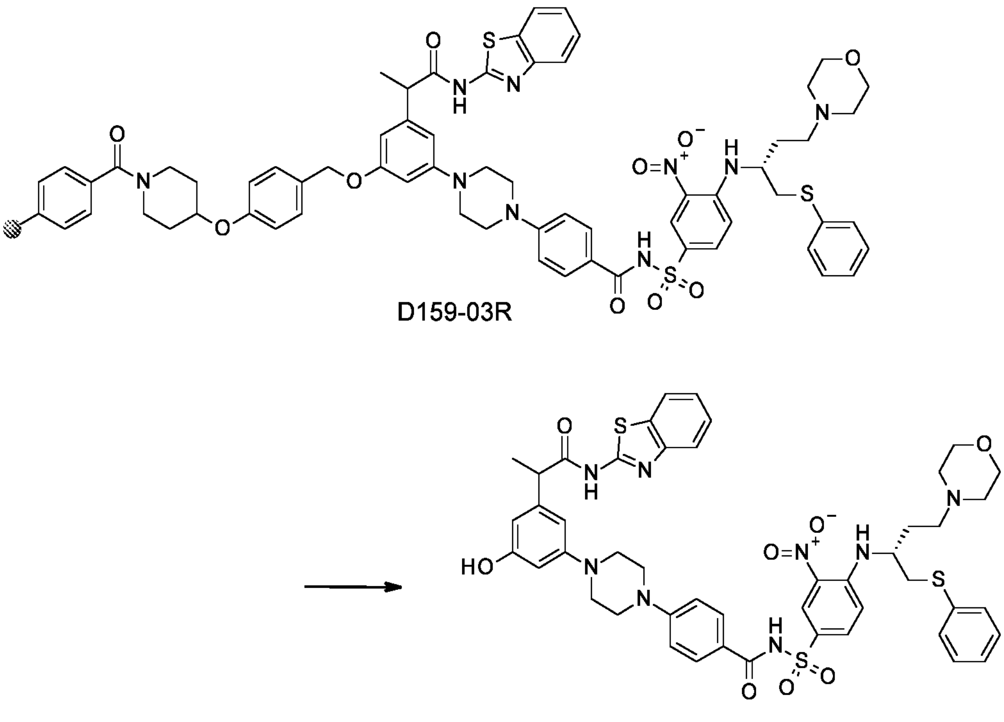

D159-03R

D159

The title compound D159 (5.6 mg, 5.9 μmol, 14%) was obtained by synthesis by the same approach as in Example 12-6-1 using D159-03R (233.8 mg, 0.176 mmol/g).

[Formula 823]

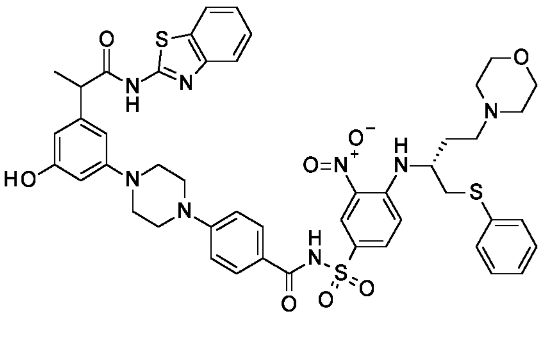

D159

Compound D159

$^{1}$H NMR (400 MHz, DMSO-d6) δ: 12.43 (1H, s), 9.21 (1H, s), 8.47 (1H, s), 8.31 (1H, s), 7.96 (1H, d, J = 8.4 Hz), 7.76-7.71 (4H, m), 7.42 (1H, t, J = 7.6 Hz), 7.27-7.21 (6H, m), 6.91-6.87 (3H, m), 6.52-6.51 (2H, m), 6.26 (2H, d, J = 14.2 Hz), 4.11-4.09 (1H, m), 3.86 (1H, q, J = 7.0 Hz), 3.51-3.35 (9H, m), 3.30-3.21 (11H, m), 1.97-1.94 (1H, m), 1.82-1.80 (1H, m), 1.41 (3H, d, J = 6.8 Hz).

LCMS: m/z 951 $[M+H]^+$.

Retention time: 0.960 min (analysis conditions SMD-FA05-1, 299 nm).

Example 12-7-1: Synthesis of D161

[Formula 824]

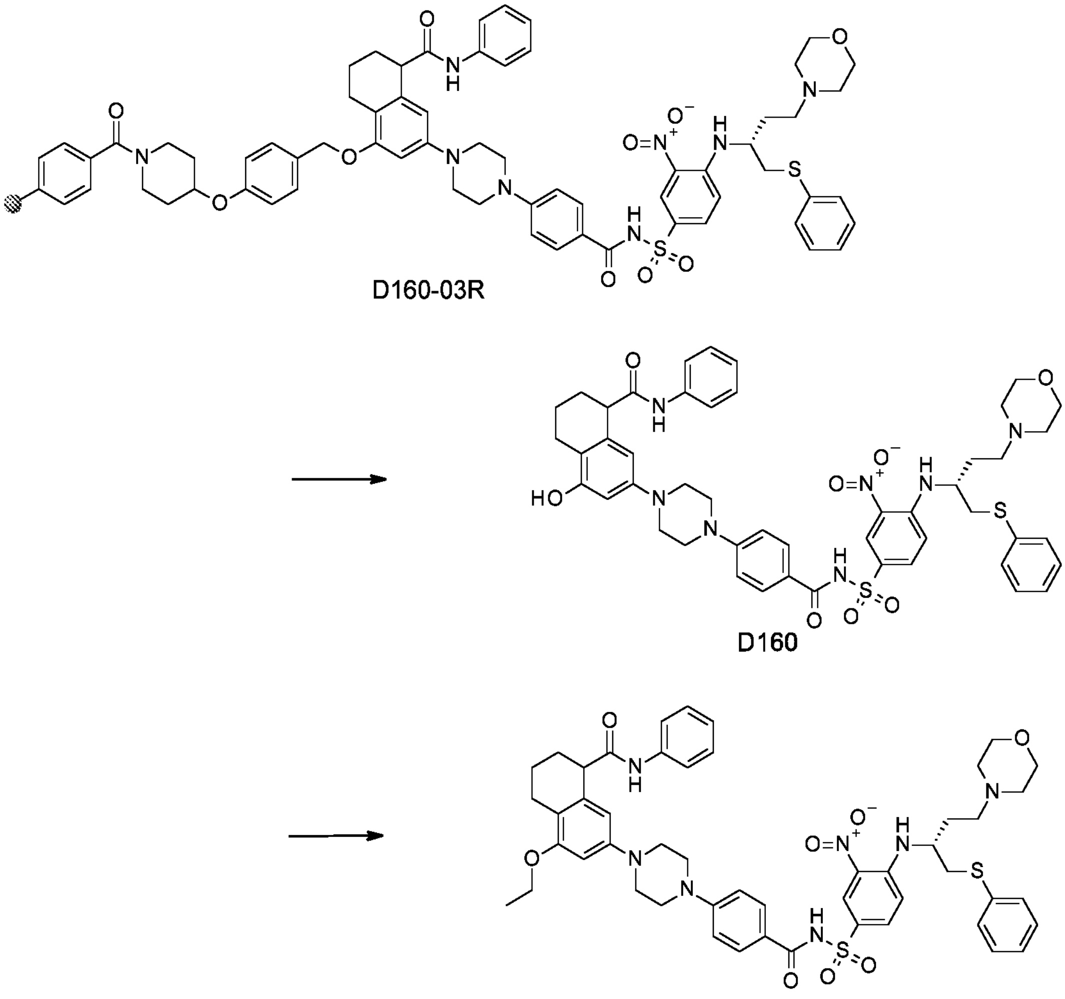

D160-03R

D160

D161

Under a nitrogen atmosphere, D160-03R (241.6 mg, 0.178 mmol/g) and 0.08 M pentamethylbenzene in DCM (4.8 mL) were added to an 8 mL glass vial and shaken at room temperature for 1 hour. TFA (0.483 mL, 6.27 mmol) was added thereto, and the mixture was shaken at room temperature for 1 hour. The suspension of the reaction solution and the solid phase was transferred to a 6 mL column with a filter using DMF (1 mL) twice and filtered. The resultant was washed three times with DMF (1.8 mL), and the solvent in combined filtrates was distilled off by Genevac. Solid-phase supported morpholine (Sigma-Aldrich Co., LLC, catalog No: 493813) (358 mg) and DMF (1 mL) were added to the obtained residue, and the mixture was shaken at room temperature for 15 minutes. The solution and the suspension of the solid phase were transferred to a 6 mL column with a filter using DMF (0.4 mL) twice and filtered. The resultant was washed three times with DMF (0.4 mL), and the solvent in combined filtrates was distilled off under reduced pressure to obtain a crude product of D160. The crude product of D160 was dissolved in DMF (491 μL) and EtOH (123 μL). Then, BTPP (131 μL, 0.430 mmol) and $Et_3PO_4$ (110 μL, 0.645 mmol) were added to the solution, and the mixture was stirred at 80°C for 16 hours. The reaction solution was transferred to a 3 mL column with a filter packed with ISOLUTE(R) CBA (670 mg, 0.7 mmol/g) using MeCN (0.1 mL) three times. 10 minutes later, the resultant was washed three times with MeCN (0.5 mL), and the solvent in combined filtrates was concentrated under reduced pressure. The obtained residue was dissolved in DMSO (800 μL), and water (40 μL) and formic acid (35 μL) were then added to the solution. The mixture was purified by preparative HPLC (C18, 0.1% solution of FA in acetonitrile/0.1% aqueous FA solution, 20-100%) and then freeze-dried to obtain the title compound D161 (4.6 mg, 4.9 μmol, 11%) as a yellow powder.

[Formula 825]

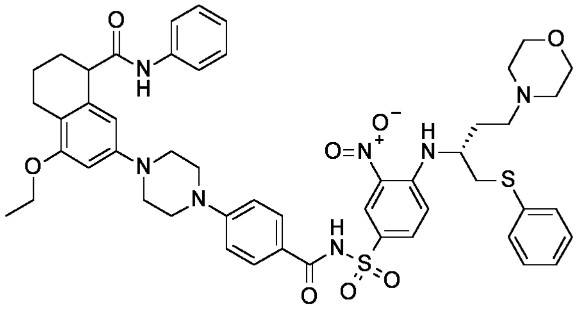

D161

Compound D161

$^1$H NMR (400 MHz, DMSO-d6) δ: 10.08 (1H, s), 8.50 (1H, s), 8.35 (1H, s), 7.80- 7.74 (3H, m), 7.61 (2H, d, J = 7.6 Hz), 7.32-7.13 (8H, m), 7.06-7.02 (2H, m), 6.91 (2H, d, J = 8.8 Hz), 6.51-6.46 (1H, m), 6.30-6.28 (1H, m), 4.14-4.12 (1H, br m), 4.02 (2H, q, J = 7.0 Hz), 3.77 (1H, t, J = 6.2 Hz), 3.48-3.40 (13H, m), 3.26-3.15 (6H, m), 2.68-2.66 (1H, m), 2.34-2.32 (1H, m), 1.99-1.88 (6H, m), 1.60-1.58 (1H, m), 1.33 (3H, t, J = 7.0 Hz).

LCMS: m/z 948 $[M+H]^+$

Retention time: 1.064 min (analysis conditions SMD-FA05-1, 299 nm)

Example 12-7-2: Synthesis of D162

[Formula 826]

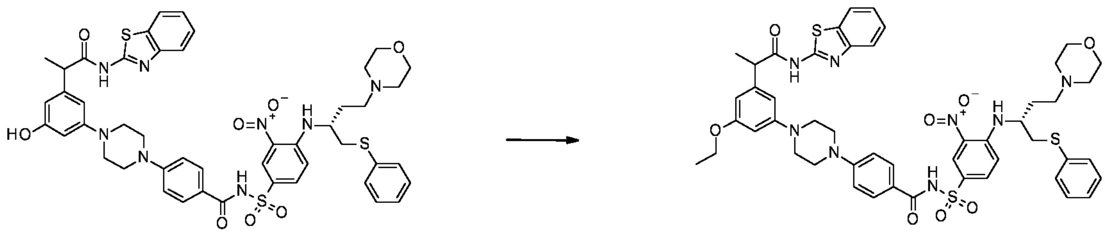

D159

D162

BTPP (18.0 μL, 0.059 mmol) and $Et_3PO_4$ (15.0 μL, 0.088 mmol) were added into a solution of D159 (5.6 mg, 5.89 μmol) in DMF (67.3 μL) and EtOH (16.8 μL), and the mixture was shaken at 70°C for 16 hours. The reaction solution was transferred to a 3 mL column with a filter packed with ISOLUTE(R) CBA (82 mg, 0.7 mmol/g) using MeCN (0.1 mL) three times. 10 minutes later, the resultant was washed three times with MeCN (0.5 mL), and the solvent in combined filtrates was concentrated under reduced pressure. The obtained residue was dissolved in DMSO (800 μL), and water (40 μL) and formic acid (35 μL) were then added to the solution. The mixture was purified by preparative HPLC (C18, 0.1% solution of FA in acetonitrile/0.1% aqueous FA solution, 20-100%) and then freeze-dried to obtain the title compound D162 (0.4 mg, 0.41 μmol, 7%) as a yellow powder.

[Formula 827]

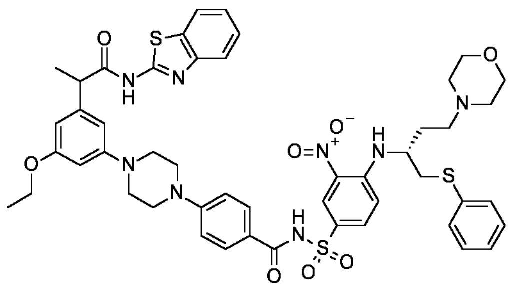

D162

Compound D162

$^1$H NMR (400 MHz, DMSO-d6) δ: 12.43 (1H, s), 8.47 (1H, s), 8.31 (1H, br s), 8.13 (1H, s), 7.96 (1H, d, J = 8.0 Hz), 7.77-7.70 (4H, m), 7.42 (1H, t, J = 7.4 Hz), 7.32-7.17 (6H, m), 6.93-6.90 (3H, br m), 6.63 (1H, s), 6.51 (1H, s), 6.40 (2H, s), 4.10 (1H, s), 3.99 (2H, q, J = 7.0 Hz), 3.91 (1H, q, J = 6.8 Hz), 3.71-2.12 (19H, m), 2.00-1.98 (1H, m), 1.82-1.79 (1H, m), 1.44 (3H, d, J = 6.8 Hz), 1.29 (3H, t, J = 6.9 Hz).

LCMS: m/z 979 $[M+H]^+$

Retention time: 1.093 min (analysis conditions SMD-FA05-1, 299 nm)

Example 12-7-3: Synthesis of D163

[Formula 828]

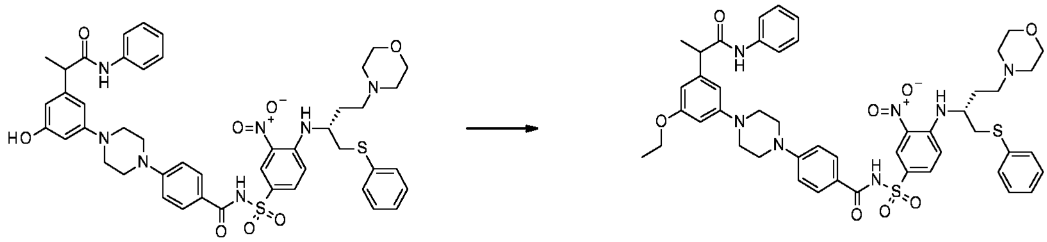

D153 D163

The title compound D153 (4.0 mg, 4.34 μmol, 32%) was obtained as a yellow powder by synthesis by the same approach as in Example 12-7-2 using D153 (12.3 mg, 0.014 mmol/g).

[Formula 829]

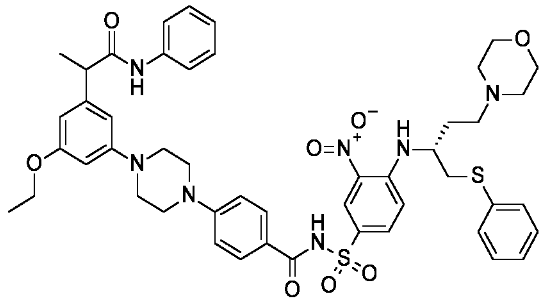

D163

Compound D163

$^1$H NMR (400 MHz, DMSO-d6) δ: 9.93 (1H, s), 8.51 (1H, s), 8.37 (1H, d, J = 8.3 Hz), 7.82-7.76 (3H, m), 7.57 (2H, d, J = 7.9 Hz), 7.29-6.94 (12H, m), 6.59 (1H, s), 6.39 (2H, d, J = 17.9 Hz), 4.15-4.13 (1H, br m), 3.98 (2H, q, J =

6.9 Hz), 3.70 (1H, q, J = 6.7 Hz), 3.66-2.24 (19H, m), 2.07-2.01 (1H, m), 1.90-1.87 (1H, m), 1.37 (3H, d, J = 7.0 Hz), 1.29 (3H, t, J = 6.9 Hz).

LCMS: m/z 922 $[M+H]^+$.

Retention time: 1.039 min (analysis conditions SMD-FA05-1, 299 nm).

Example 12-7-4: Synthesis of D164

[Formula 830]

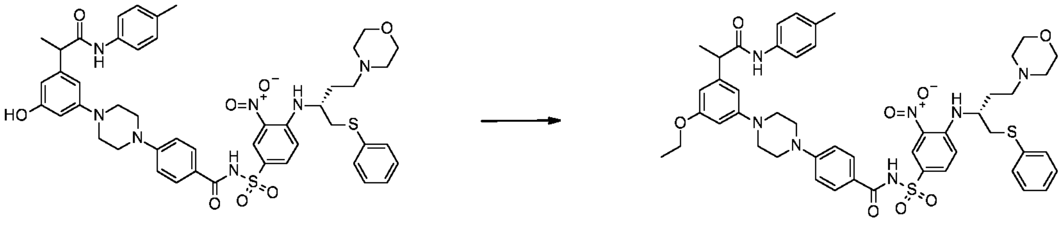

D154 D164

The title compound D164 (1.5 mg, 1.6 μmol, 29%) was obtained as a yellow powder by synthesis by the same approach as in Example 12-7-2 using D154 (5.1 mg, 5.62 μmol).

[Formula 831]

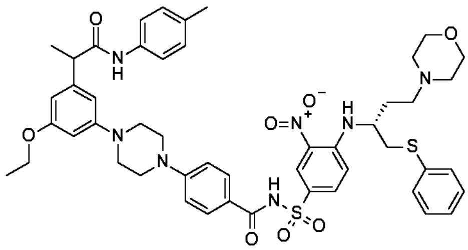

D164

Compound D164

$^1$H NMR (400 MHz, DMSO-d6) δ: 9.83 (1H, s), 8.49 (1H, s), 8.44-8.25 (1H, m), 7.78-7.76 (3H, m), 7.45 (2H, d, J = 8.4 Hz), 7.31-7.16 (6H, m), 7.07 (2H, d, J = 8.2 Hz), 6.95-6.92 (3H, m), 6.59 (1H, s), 6.38 (2H, d, J = 14.9 Hz), 4.19-4.06 (1H, m), 3.98 (2H, q, J = 6.9 Hz), 3.67 (1H, q, J = 7.4 Hz), 3.61-3.11 (19H, m), 2.68-2.66 (1H, m), 2.22 (3H, s), 2.07- 1.94 (1H, m), 1.92-1.77 (1H, m), 1.36 (3H, d, J = 6.8 Hz), 1.29 (3H, t, J = 7.0 Hz).

LCMS: m/z 936 $[M+H]^+$

Retention time: 1.076 min (analysis conditions SMD-FA05-1, 299 nm)

Example 12-7-5: Synthesis of D165

[Formula 832]

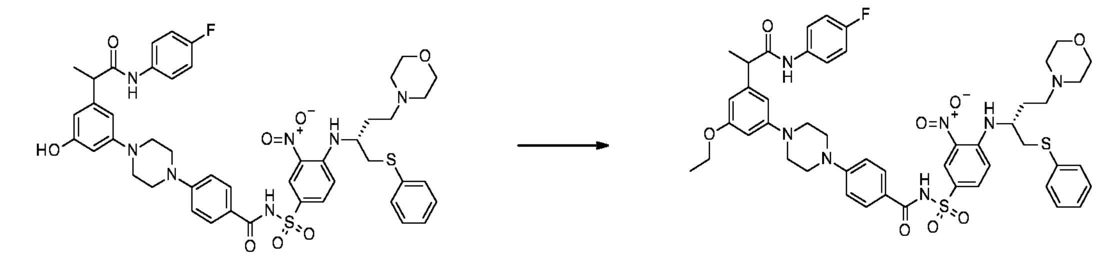

D156 D165

The title compound D165 (4.8 mg, 5.1 μmol, 56%) was obtained as a yellow powder by synthesis by the same approach as in Example 12-7-2 using D156 (8.3 mg, 9.1 μmol/g).

[Formula 833]

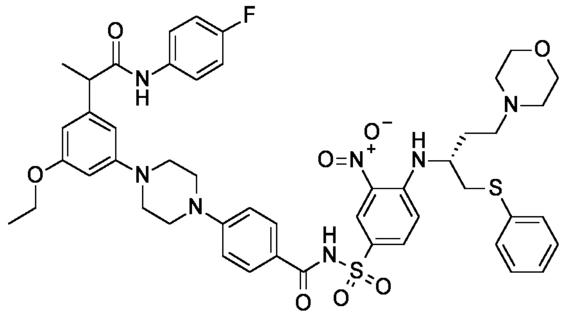

D165

Compound D165

$^1$H-NMR (400 MHz, DMSO-d6) δ: 9.99 (1H, s), 8.50 (1H, s), 8.35 (1H, s), 7.82- 7.76 (3H, m), 7.59 (2H, dd, J = 8.9, 5.1 Hz), 7.30-7.01 (9H, m), 6.94 (2H, d, J = 8.6 Hz), 6.58 (1H, s), 6.38 (2H, d, J = 12.1 Hz), 4.15-4.12 (1H, m), 3.98 (2H, q, J = 6.9 Hz), 3.68 (1H, q, J = 13.6 Hz), 3.64-2.22 (20H, m), 2.02-1.99 (1H, m), 1.88-1.85 (1H, m), 1.37 (3H, d, J = 6.8 Hz), 1.29 (3H, t, J = 7.0 Hz).

LCMS: m/z 940 $[M+H]^+$.

Retention time: 1.061 min (analysis conditions SMD-FA05-1, 299 nm).

Example 13: Evaluation of binding of compound to Bcl-2 by surface plasmon resonance (SPR) - 2

A surface plasmon resonance method was used in order to analyze the binding affinity of the compounds synthesized in Example 12 for Bcl-2, which are shown in Table 13-1. The apparatus used was Biacore T200 (GE Healthcare Japan Corp.), and the running buffer used was HBS (10 mM HEPES-NaOH and 150 mM NaCl, pH 7.4) supplemented with 1 mM DTT, 3 mM EDTA, 0.01% Tween 20 and 4% DMSO. Human Bcl-2 protein (Novus Biologicals) was biotinylated using EZ-Link NHS-PEG4-Biotin (Thermo Fisher scientific Inc.) and immobilized on the surface of Sensor Chip SA (Cytiva). Then, compound solutions having a plurality of concentrations and the running buffer (blank) were added to the Bcl-2- immobilized surface to obtain the binding response of the compounds. Measurement was performed at 15°C.

The obtained sensorgrams were treated with Biacore T200 Evaluation Software, and the equilibrium dissociation constant $K_D$ of each compound for Bcl-2 was determined as shown in the following Table 13-2.

[Table 607]

| [Table 13-1]: Structural formula of compound used in present Example | | | |
|---|---|---|---|
| ID | Compound No. | Structure | Exact Mass |
| 2-2-d1E03-1- 0417 | D152 | 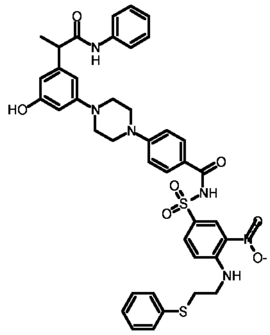 | 780.24 |
| 2-2-d1E03-1- 0454 | D155 | 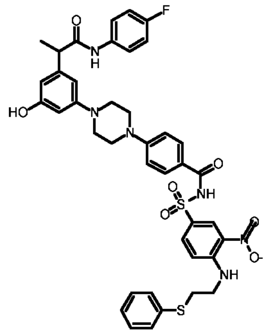 | 798.231 |
| 2-2-d1E03-1- 0495 | D158 | 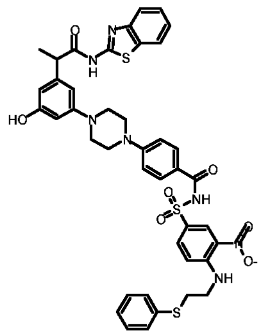 | 837.207 |
| 2-2-d1E04-1- 0417 | D153 | 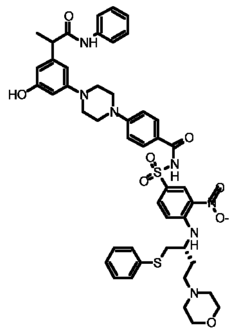 | 893.324 |

(continued)

| [Table 13-1]: Structural formula of compound used in present Example | | | |
|---|---|---|---|
| ID | Compound No. | Structure | Exact Mass |
| 2-2-d1E04-1- 0446 | D154 | 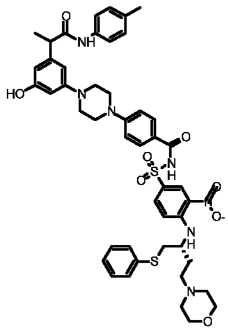 | 907.34 |
| 2-2-d1E04-1- 0454 | D156 | 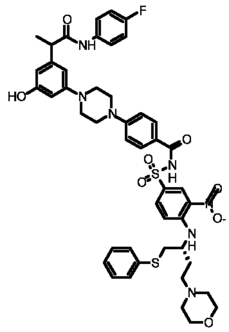 | 911.315 |
| 2-2-d1E04-1- 0495 | D159 | 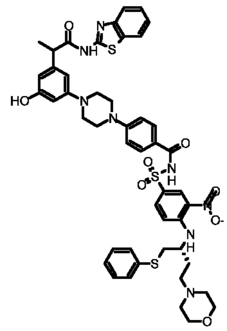 | 950.291 |
| 2-2-d1E04-1- 0498 | D157 | 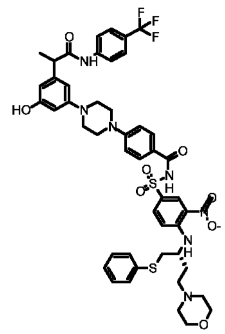 | 961.311 |

(continued)

| [Table 13-1]: Structural formula of compound used in present Example | | | |
|---|---|---|---|
| ID | Compound No. | Structure | Exact Mass |
| 2-2-d1E04-2- 0417 | D163 | 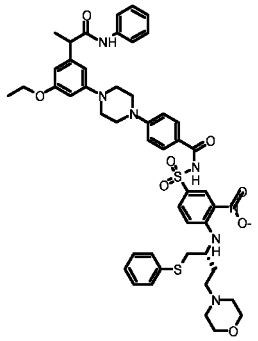 | 921.355 |
| 2-2-d1E04-2- 0446 | D164 | 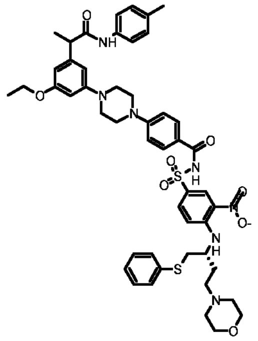 | 935.371 |
| 2-2-d1E04-2- 0454 | D165 | 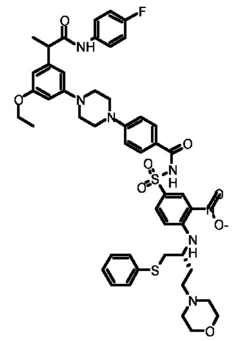 | 939.346 |
| 2-2-d1E04-2- 0466 | D161 | 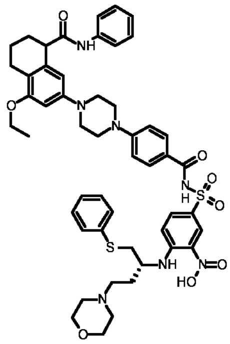 | 947.371 |

(continued)

| [Table 13-1]: Structural formula of compound used in present Example | | | |
|---|---|---|---|
| ID | Compound No. | Structure | Exact Mass |
| 2-2-d1E04-2- 0495 | D162 | 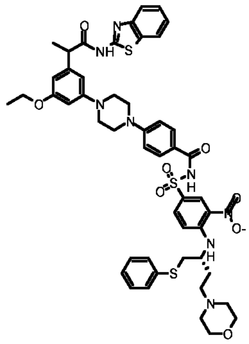 | 978.323 |

[Table 608]

| [Table 13-2]: Equilibrium dissociation constant $K_D$ of compound found by AS-MS evaluation of synthesized library | | | |
|---|---|---|---|
| ID | Compound No. | Compositional formula | $K_D$ [μmol/L] |
| 2-2-d1E03-1-0417 | D152 | C40 H40 N6 O7 S2 | 7.91 |
| 2-2-d1E03-1-0454 | D155 | C40 H39 F N6 O7 S2 | 15.2 |
| 2-2-d1E03-1-0495 | D158 | C41 H39 N7 O7 S3 | 5.34 |
| 2-2-d1E04-1-0417 | D153 | C46 H51 N7 O8 S2 | 0.582 |
| 2-2-d1E04-1-0446 | D154 | C47 H53 N7 O8 S2 | 0.775 |
| 2-2-d1E04-1-0454 | D156 | C46 H50 F N7 O8 S2 | 0.788 |
| 2-2-d1E04-1-0495 | D159 | C47 H50 N8 O8 S3 | 0.162 |
| 2-2-d1E04-1-0498 | D157 | C47 H50 F3 N7 O8 S2 | 2.51 |
| 2-2-d1E04-2-0417 | D163 | C45 H46 F3 N5 O9 S2 | 0.846 |
| 2-2-d1E04-2-0446 | D164 | C45 H48 F3 N7 O8 S2 | 1.33 |
| 2-2-d1E04-2-0454 | D165 | C44 H45 F3 N6 O10 S2 | 1.06 |
| 2-2-d1E04-2-0466 | D161 | C48 H52 F3 N5 O8 S2 | 2.45 |
| 2-2-d1E04-2-0495 | D162 | C49 H48 N6 O10 S3 | 0.706 |

As shown in Table 13-2, compounds (2-2-d1E04-1-0495, 2-2-d1E04-1-0417, 2-2- d1E04-2-0495, 2-2-d1E04-1-0446, 2-2-d1E04-1-0454, 2-2-d1E04-2-0417, and 2-2-d1E04-2- 0454) were found to have a smaller equilibrium dissociation constant $K_D$ and more highly bind to Bcl-2 than compound B2Q045-01 ($K_D$ = 1.20 μM) used as a positive control or compound B2Q048-01 ($K_D$ = 0.480 μM) evaluated for its ability to bind in Example 7.

A library that was searched in a wide range at once was newly synthesized by multiplying the "diversity of core blocks" and the "diversity of linkers", increasing the scale (a total of 12,000 compounds) over the mixtures (a total of 1,200 compounds) synthesized in Example 4 (Examples 8 and 9), and compounds that bound to Bcl-2 were identified by AS- MS (Example 11). Furthermore, the identified compounds were individually synthesized and found by SPR to specifically bind to Bcl-2.

[Formula 834]

[Formula 13-01]

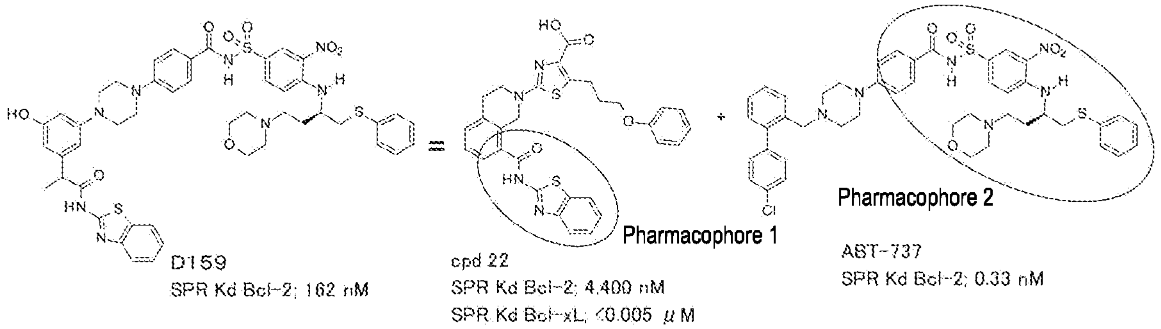


D159
SPR Kd Bcl-2; 162 nM

cpd 22
SPR Kd Bcl-2; 4,400 nM
SPR Kd Bcl-xL; <0.005 μM

ABT-737
SPR Kd Bcl-2; 0.33 nM

13 compounds confirmed to bind by SPR are, as shown in formula 13-01, hybrid compounds of two compound types reportedly having the ability to bind to Bcl family protein. Specifically, compound D159 is a compound having a structure where a portion of pharmacophore 1 of cpd22 (ACS Med. Chem. Lett. 2014, 5, 6, 662-667) and a portion of pharmacophore 2 of ABT-737 are combined and linked via a suitable linker. Scaffold hopping by hybridizing pharmacophores of respectively optimized compounds is usually difficult. Particularly, a suitable linker is very difficult to set. The results about the libraries implemented in Examples 8 and 9 indicate that according to the present invention, a new hit compound can be obtained by linking mutually combined structures of two pharmacophores via a suitable linker. An important feature of the present invention is to provide mixtures that enable many potential substructures and a plurality of pharmacophores to be evaluated at once. Hybridization that involves mutually fusing the structures of a plurality compound types, and acquiring a new hit compound can be regarded as an approach of scaffold hopping.

In search for new compounds aimed at developing medicaments, a compound is generally derivatized while the scaffold structure of the compounds is fixed on a given structure. In other words, the scaffold is fixed and the other moieties are generally optimized. The search approaches used in many reported library techniques also utilize such manner. Scaffold is same among such library. Hence, although not only combinations of core blocks but combinations of types of linkers that link the core blocks and substitution patterns on the core blocks cover a wide scope in a matrical manner, derivatization is carried out by a method that has the difficulty in searching all of them. As a result, such an approach is very likely to miss a better compound (in this context, the term "better" specifically refers to, for example, the ability to bind, selectivity for a target protein, or physical properties such as lipophilicity or hydrophilicity) that may fall outside the range where limited combinations are searched. The approach of the present invention was found capable of searching diverse compounds at once by multiplying the diversity of core blocks and the diversity of linkers and further combining different substitution patterns, and capable of testing effects of compounds that may fall outside a range searched by conventional approaches. This approach is one aspect to solve challenges required for the drug discovery of hit generation, scaffold hopping and FBDD (fragment-based drug discovery), and the value of the present invention was demonstrated.

## Claims

**1.** A library comprising $1 \times 10^2$ to $1 \times 10^8$ compounds, wherein
the compounds have the following structure where a first core block (first CB), a first linker (first L), and a second core block (second CB) are covalently linked:
(first CB)-(first L)-(second CB), wherein

the library comprises two or more types of first CB, two or more types of first L, and two or more types of second CB, and
the library is constituted by one or two or more mixtures comprising $1 \times 10^2$ to $1 \times 10^5$ of the compounds.

**2.** The library according to claim 1, wherein
the compounds have the following structure where a second linker (second L) and a third core block (third CB) are further covalently linked:
(first CB)-(first L)-(second CB)-(second L)-(third CB), and
the library comprises two or more types of second L and two or more types of third CB.

**3.** The library according to claim 1 or 2, wherein the compounds comprise no oligonucleotide tag.

**4.** The library according to any one of claims 1 to 3, wherein the first L, the second L, and the third L have a structure independently selected from the group consisting of amide, sulfonamide, acylsulfonamide, amino, ether, thioether, ester, ketone, sulfone, a single bond, ethyne-1,2-diyl (-C=C-), and nitrogen-containing 5-membered heteroarylene.

**5.** A method for screening for a compound that binds to a target molecule, comprising:

(1) providing a library according to any one of claims 1 to 4;
(2) bringing each of the one or two or more mixtures constituting the library into contact with the target molecule; and
(3) identifying a compound that binds to the target molecule.

**6.** A method for producing a library, comprising:

(a) preparing supporting carriers to which a first building block (first BB) is covalently linked (first BB-supporting carriers), wherein the first BB-supporting carriers are solid-phase carriers comprising na types of first building blocks (first BBi to first $BB_{na}$) (first $BB_{1-na}$-supporting carriers), the first $BB_{1-na}$ moieties of the first BBi-na-supporting carriers each have a reactive functional group for forming a bond with a second building block (second BB), and the reactive functional groups contained in the first $BB_{1-na}$-supporting carriers are of one type or two or more types;
(b) preparing mixtures of supporting carriers having the reactive functional group common among the first $BB_{1-na}$-supporting carriers, and reacting each mixture with one type of second building block introduction reagent (second BB introduction reagent) or a mixture of two or more types of second BB introduction reagents to prepare supporting carriers in which the second BB is covalently linked to the first BB (second BB-first BB-supporting carriers), wherein the resulting supporting carriers are supporting carriers comprising nb types of second building blocks (second BBi to second $BB_{nb}$) (second $BB_{1-nb}$-first $BB_{1-na}$- supporting carriers), and any of the second BBi introduction reagent to the second $BB_{nb}$ introduction reagent reacts with the respective reactive functional groups of the first $BB_{1-na}$- supporting carriers to form first linkers (first L); and
(e) cleaving compounds from the solid-phase carriers, wherein
na is an integer of 2 or larger, nb is an integer of 2 or larger, and the resulting compound library comprises 100 or more types of compounds as a mixture.

**7.** The method according to claim 6, wherein (b) comprises preparing mixtures of supporting carriers having the reactive functional group common among the first $BB_{1-na}$- supporting carriers, and reacting each mixture with one of nb types of second BB introduction reagents.

**8.** The method according to claim 6 or 7, wherein

the second $BB_{1-nb}$-first $BB_{1-na}$-supporting carriers each have a reactive functional group capable of forming a linker with a third building block (third BB), and the reactive functional group that is singly contained in each of the first $BB_{1-na}$ moieties or the second $BB_{1-nb}$ moieties is of one type or two or more types,
the method further comprising
(c) preparing mixtures of supporting carriers having the reactive functional group common among the second $BB_{1-nb}$-first BBi-na-supporting carriers, and reacting each mixture with one type of third building block introduction reagent (third BB introduction reagent) or a mixture of two or more types of third BB introduction reagents to prepare supporting carriers in which the third BB is covalently linked to the first BB or the second BB (third BB-second BB-first BB-supporting carriers or second BB-(third BB-)first BB-supporting carriers), wherein the resulting supporting carriers are supporting carriers comprising nc types of third building blocks (third $BB_1$ to third $BB_{nc}$) (third $BB_{1-nc}$-second $BB_{1-nb}$-first $BB_{1-na}$-supporting carriers or second $BB_{1-nb}$-(third BBi-nc-)first $BB_{1-na}$-supporting carriers), and any of the third $BB_1$ introduction reagent to the third $BB_{nc}$ introduction reagent reacts with the respective reactive functional groups of the second $BB_{1-nb}$-first $BB_{1-na}$-supporting carriers to form second linkers (second L), wherein
nc is an integer of 2 or larger, and the first linkers or the second linkers of the compounds contained in the library are of two or more types.

**9.** The method according to any one of claims 6 to 8, wherein

na is an integer of 2 to 1000, nb is an integer of 2 to 1000, and any of na and nb is an integer of 3 or larger; or na is an integer of 2 to 1000, nb is an integer of 2 to 1000, nc is an integer of 2 to 1000, and any of na, nb and nc is an integer of 3 or larger.

**10.** The method according to any one of claims 6 to 9, further comprising a derivatization step for the reactive functional group.

**11.** The method according to any one of claims 6 to 10, further comprising modifying the functional groups contained in the compounds cleaved from the solid-phase carriers in (e).

**12.** The method according to any one of claims 6 to 11, wherein one or more compounds contained in the library comprise a UV tag.

**13.** The method according to any one of claims 6 to 12, wherein the compounds contained in the library comprise no oligonucleotide tag.

**14.** The method according to any one of claims 6 to 13, wherein the first linkers, the second linkers, and the third linkers are formed through reaction independently selected from the group consisting of amidation, sulfonamidation, acylsulfonamidation, amination, etherification, thioetherification, esterification, carbon-nitrogen bond formation reaction, carbon-carbon bond formation reaction, and nitrogen-containing 5-membered heteroarylene formation reaction.

**15.** The method according to any one of claims 6 to 14, wherein the resulting library comprises 100 or more compounds.

# Figure 1

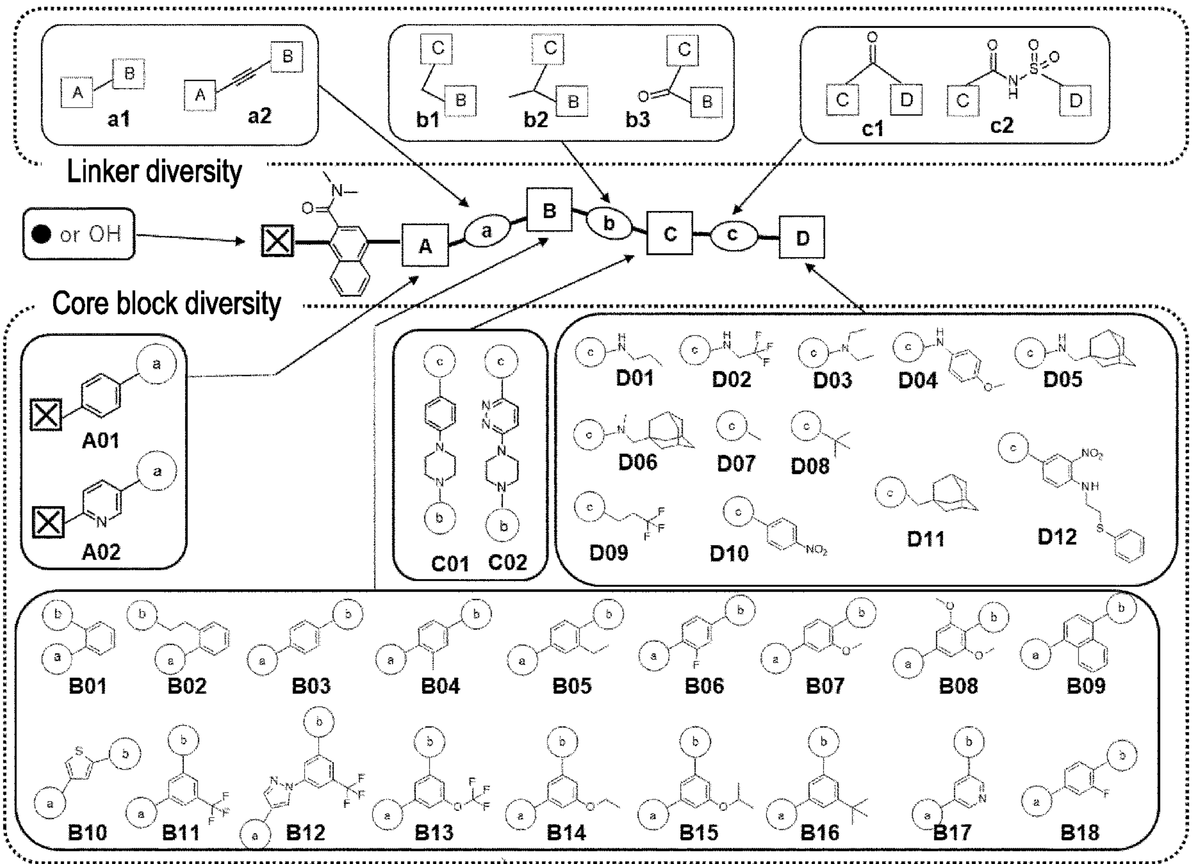


In Figure 1, ● represents 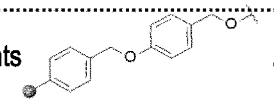 .

# Figure 2

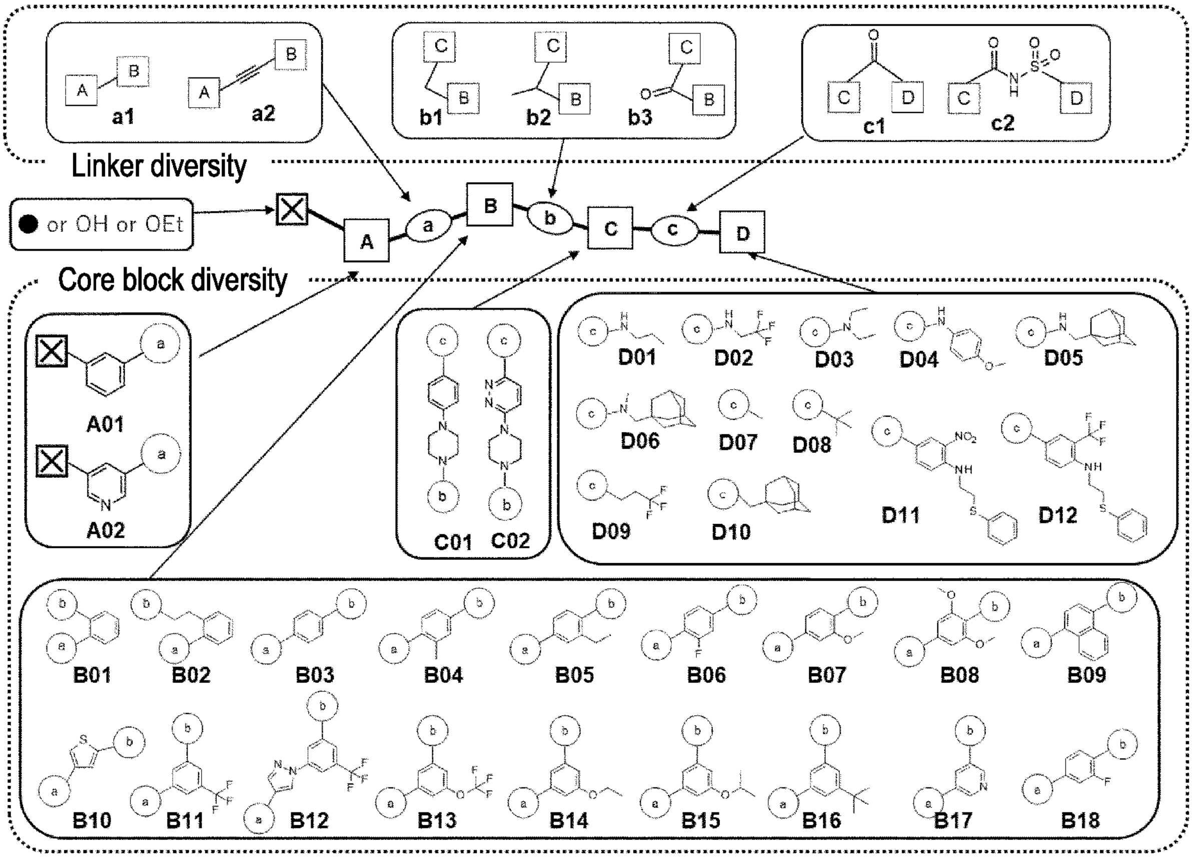


In Figure 2, ● represent 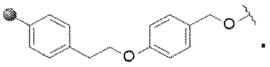.

# Figure 3

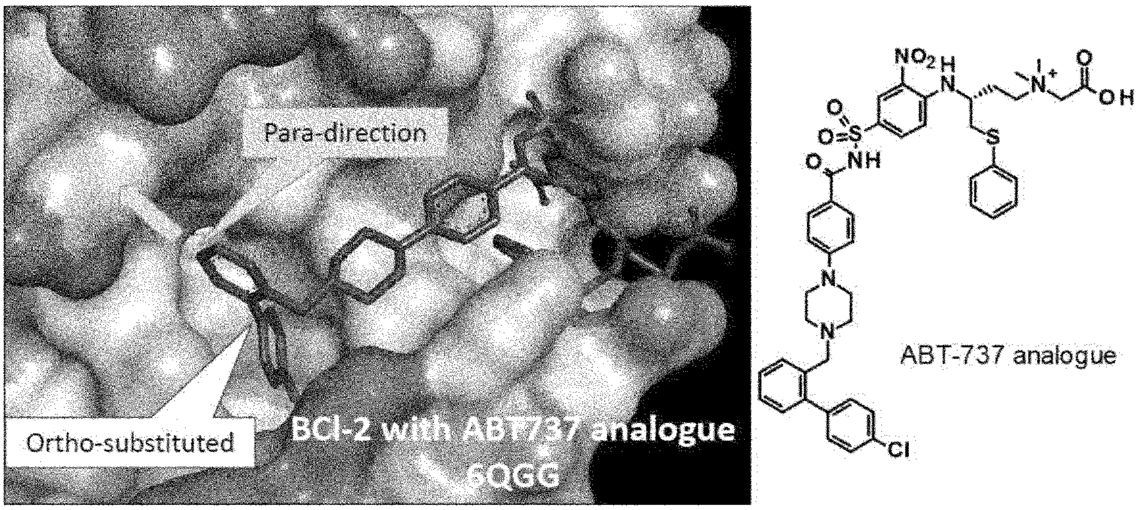

# Figure 4

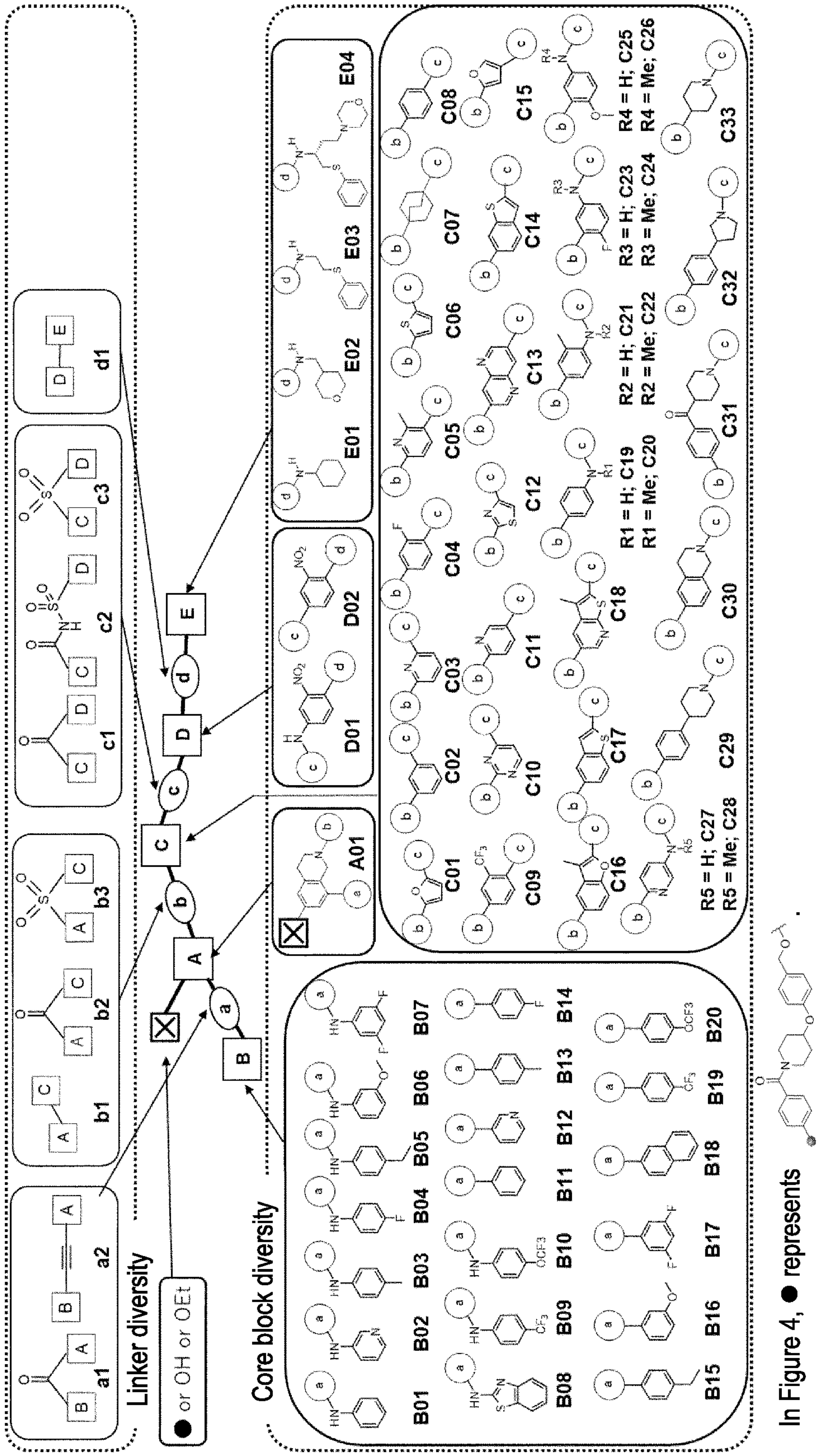
Linker diversity
a1
a2
b1
b2
b3
c1
c2
c3
d1
● or OH or OEt
Core block diversity
A01
B01
B02
B03
B04
B05
B06
B07
B08
B09
B10
B11
B12
B13
B14
B15
B16
B17
B18
B19
B20
C01
C02
C03
C04
C05
C06
C07
C08
C09
C10
C11
C12
C13
C14
C15
C16
C17
C18
R1 = H; C19
R1 = Me; C20
R2 = H; C21
R2 = Me; C22
R3 = H; C23
R3 = Me; C24
R4 = H; C25
R4 = Me; C26
R5 = H; C27
R5 = Me; C28
C29
C30
C31
C32
C33
D01
D02
E01
E02
E03
E04
In Figure 4, ● represents

# Figure 5A

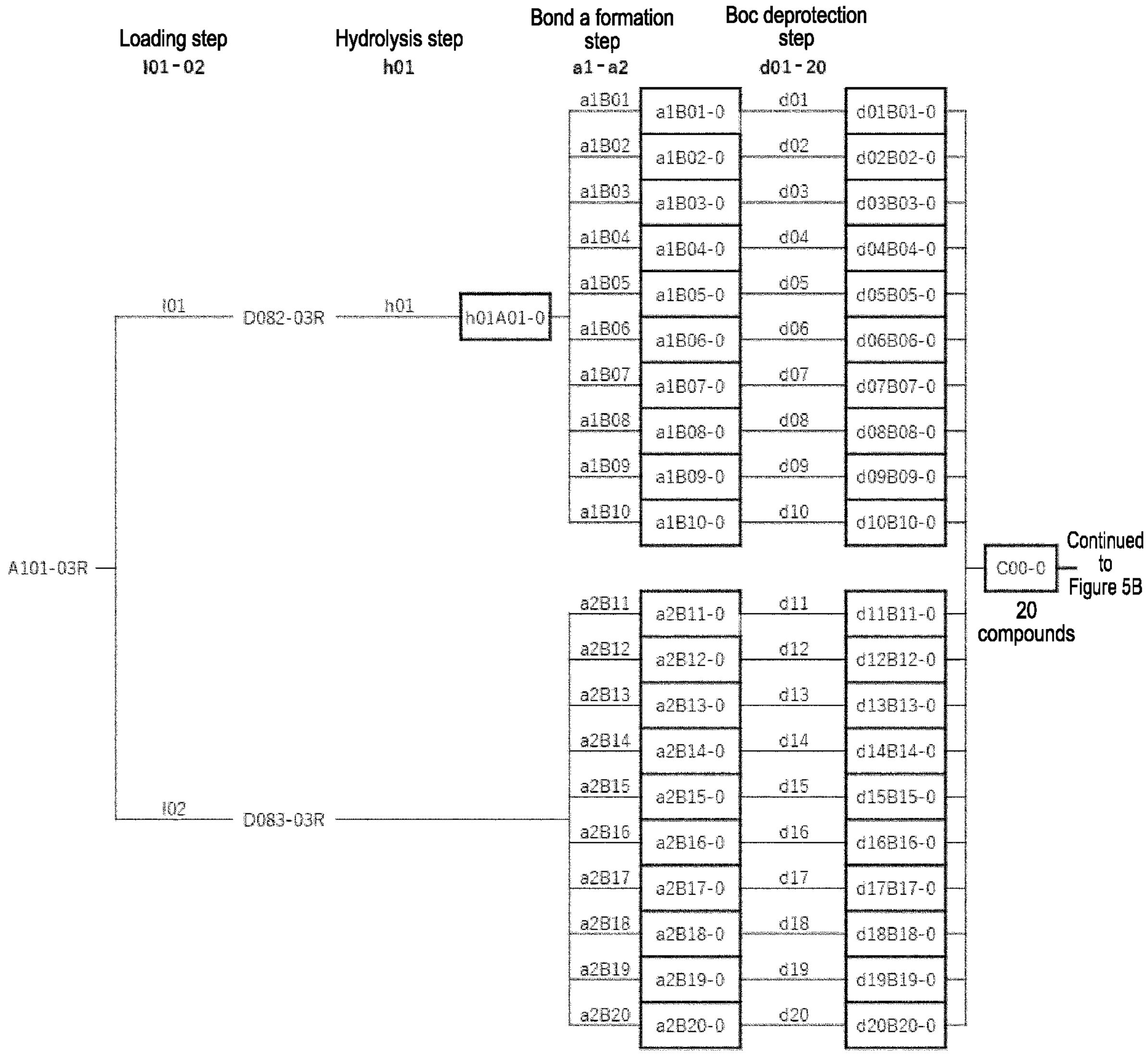
Loading step
l01 - 02
Hydrolysis step
h01
Bond a formation step
a1 - a2
Boc deprotection step
d01 - 20
A101-03R
l01
D082-03R
h01
h01A01-0
l02
D083-03R
a1B01 a1B01-0 d01 d01B01-0
a1B02 a1B02-0 d02 d02B02-0
a1B03 a1B03-0 d03 d03B03-0
a1B04 a1B04-0 d04 d04B04-0
a1B05 a1B05-0 d05 d05B05-0
a1B06 a1B06-0 d06 d06B06-0
a1B07 a1B07-0 d07 d07B07-0
a1B08 a1B08-0 d08 d08B08-0
a1B09 a1B09-0 d09 d09B09-0
a1B10 a1B10-0 d10 d10B10-0
a2B11 a2B11-0 d11 d11B11-0
a2B12 a2B12-0 d12 d12B12-0
a2B13 a2B13-0 d13 d13B13-0
a2B14 a2B14-0 d14 d14B14-0
a2B15 a2B15-0 d15 d15B15-0
a2B16 a2B16-0 d16 d16B16-0
a2B17 a2B17-0 d17 d17B17-0
a2B18 a2B18-0 d18 d18B18-0
a2B19 a2B19-0 d19 d19B19-0
a2B20 a2B20-0 d20 d20B20-0
C00-0
20 compounds
Continued to Figure 5B

# Figure 5B

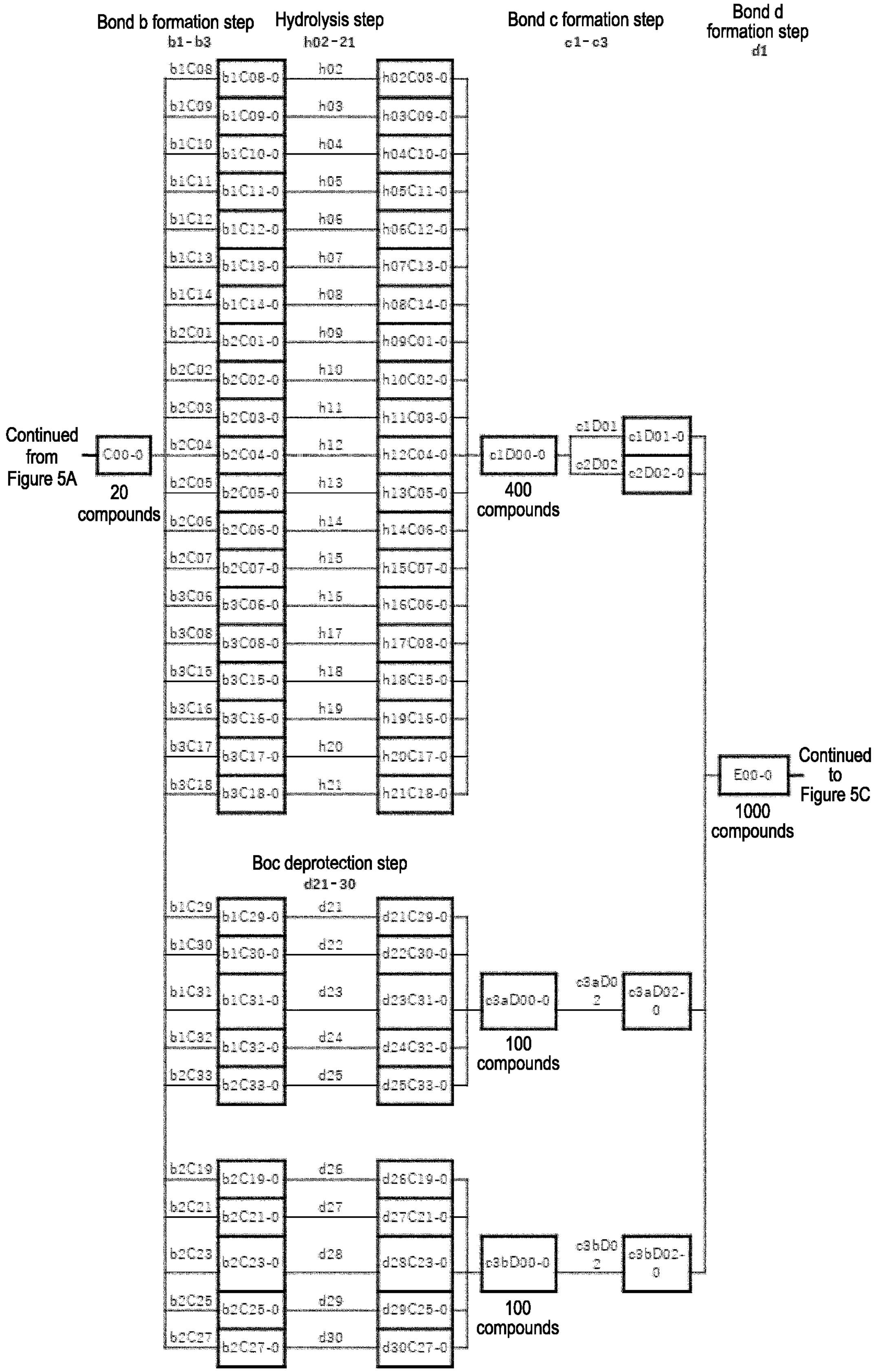

Bond b formation step
b1-b3
Hydrolysis step
h02-21
Bond c formation step
c1-c3
Bond d formation step
d1
b1C08
b1C08-0
h02
h02C08-0
b1C09
b1C09-0
h03
h03C09-0
b1C10
b1C10-0
h04
h04C10-0
b1C11
b1C11-0
h05
h05C11-0
b1C12
b1C12-0
h06
h06C12-0
b1C13
b1C13-0
h07
h07C13-0
b1C14
b1C14-0
h08
h08C14-0
b2C01
b2C01-0
h09
h09C01-0
b2C02
b2C02-0
h10
h10C02-0
b2C03
b2C03-0
h11
h11C03-0
b2C04
b2C04-0
h12
h12C04-0
b2C05
b2C05-0
h13
h13C05-0
b2C06
b2C06-0
h14
h14C06-0
b2C07
b2C07-0
h15
h15C07-0
b3C06
b3C06-0
h16
h16C06-0
b3C08
b3C08-0
h17
h17C08-0
b3C15
b3C15-0
h18
h18C15-0
b3C16
b3C16-0
h19
h19C16-0
b3C17
b3C17-0
h20
h20C17-0
b3C18
b3C18-0
h21
h21C18-0
Continued from Figure 5A
C00-0
20 compounds
c1D00-0
400 compounds
c1D01
c1D01-0
c2D02
c2D02-0
E00-0
1000 compounds
Continued to Figure 5C
Boc deprotection step
d21-30
b1C29
b1C29-0
d21
d21C29-0
b1C30
b1C30-0
d22
d22C30-0
b1C31
b1C31-0
d23
d23C31-0
b1C32
b1C32-0
d24
d24C32-0
b2C33
b2C33-0
d25
d25C33-0
c3aD00-0
100 compounds
c3aD02
c3aD02-0
b2C19
b2C19-0
d26
d26C19-0
b2C21
b2C21-0
d27
d27C21-0
b2C23
b2C23-0
d28
d28C23-0
b2C25
b2C25-0
d29
d29C25-0
b2C27
b2C27-0
d30
d30C27-0
c3bD00-0
100 compounds
c3bD02
c3bD02-0

# Figure 5C

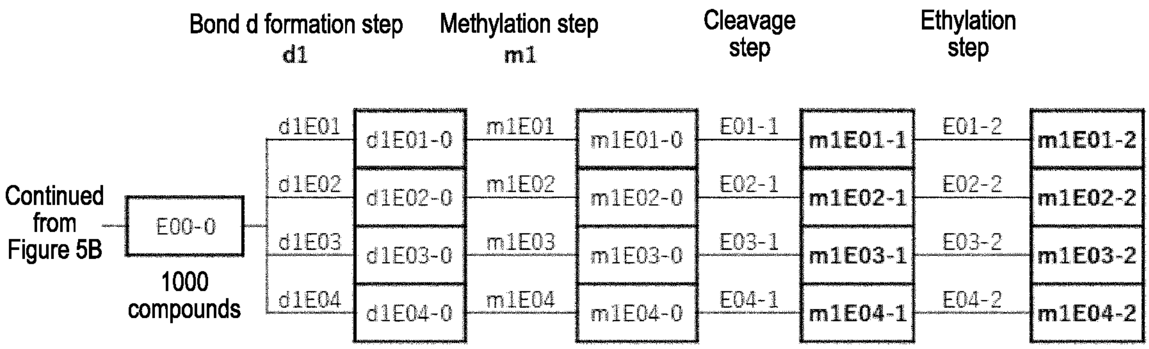
Bond d formation step
d1
Methylation step
m1
Cleavage step
Ethylation step
Continued from Figure 5B
E00-0
1000 compounds
d1E01
d1E02
d1E03
d1E04
d1E01-0
d1E02-0
d1E03-0
d1E04-0
m1E01
m1E02
m1E03
m1E04
m1E01-0
m1E02-0
m1E03-0
m1E04-0
E01-1
E02-1
E03-1
E04-1
m1E01-1
m1E02-1
m1E03-1
m1E04-1
E01-2
E02-2
E03-2
E04-2
m1E01-2
m1E02-2
m1E03-2
m1E04-2

Figure 6

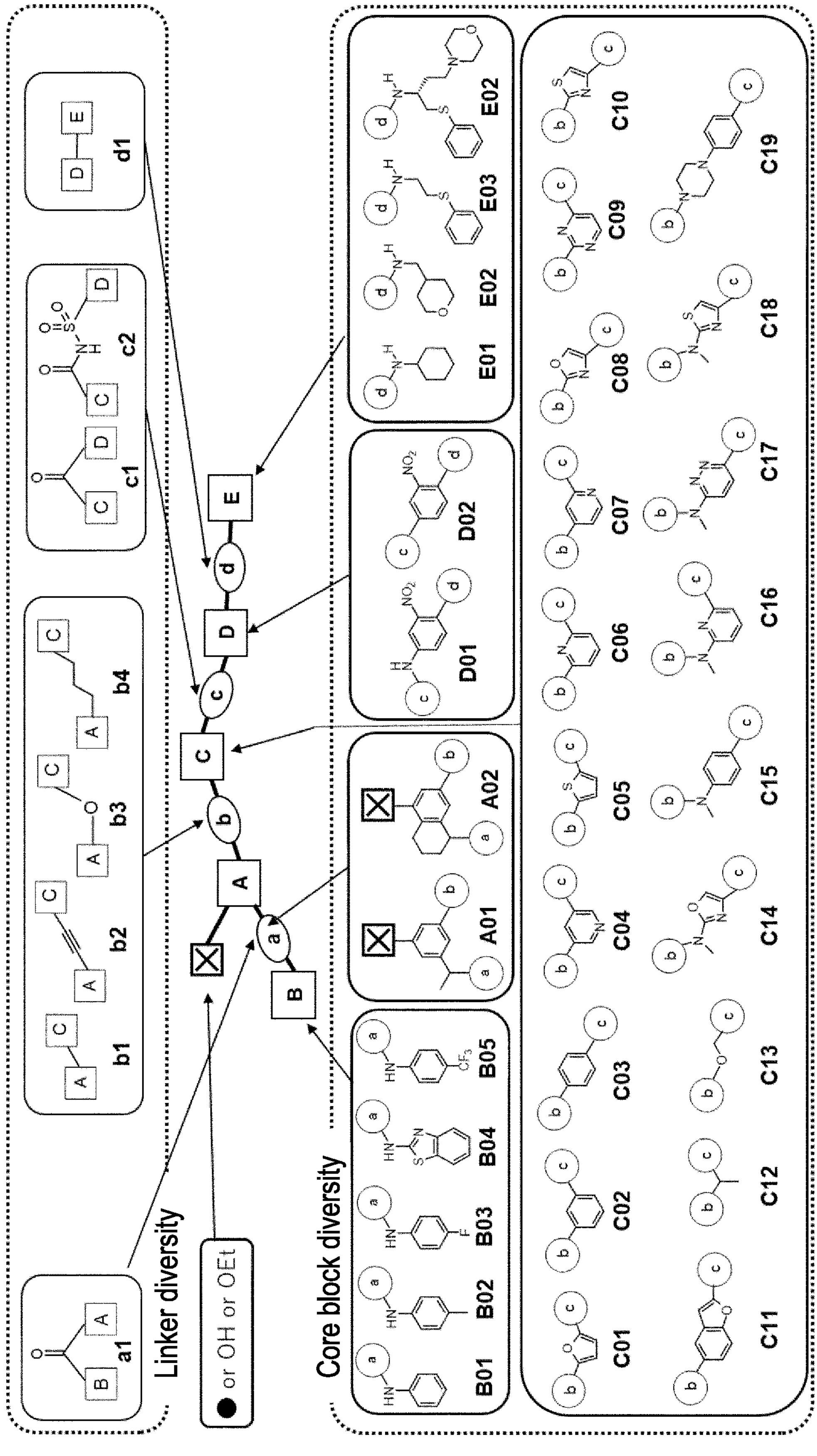


In Figure 6, ● represents 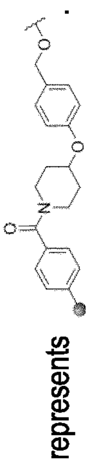 .

Figure 7A

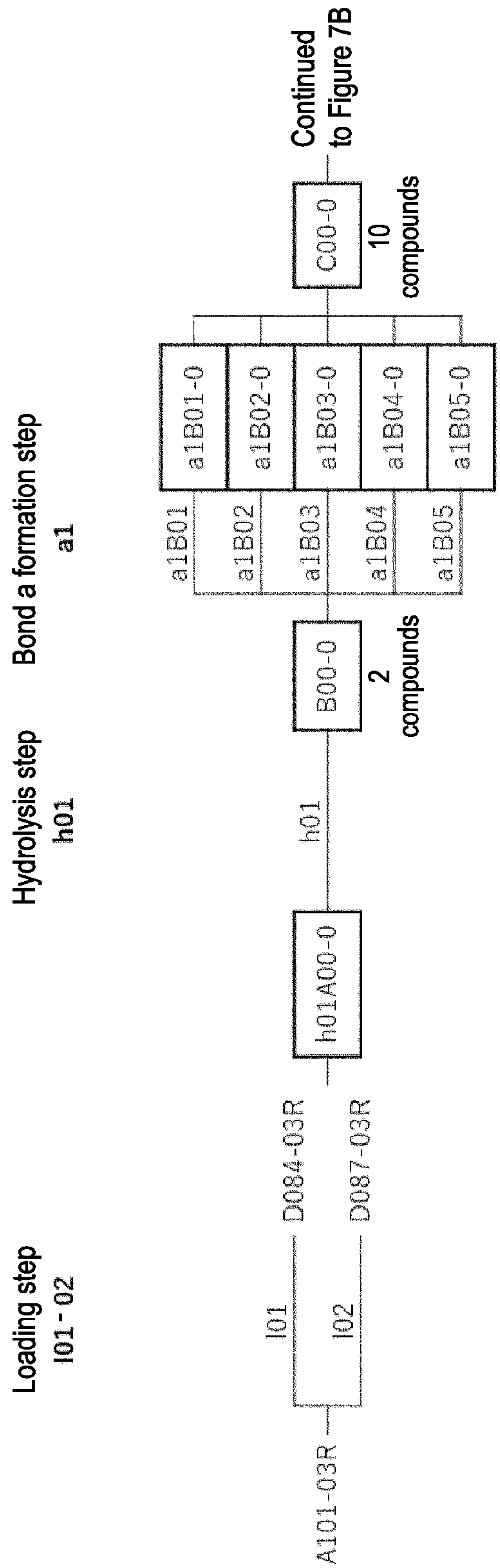
Loading step
l01 - 02
Hydrolysis step
h01
Bond a formation step
a1
A101-03R
l01
l02
D084-03R
D087-03R
h01A00-0
h01
B00-0
2
compounds
a1B01
a1B02
a1B03
a1B04
a1B05
a1B01-0
a1B02-0
a1B03-0
a1B04-0
a1B05-0
C00-0
10
compounds
Continued
to Figure 7B

# Figure 7B

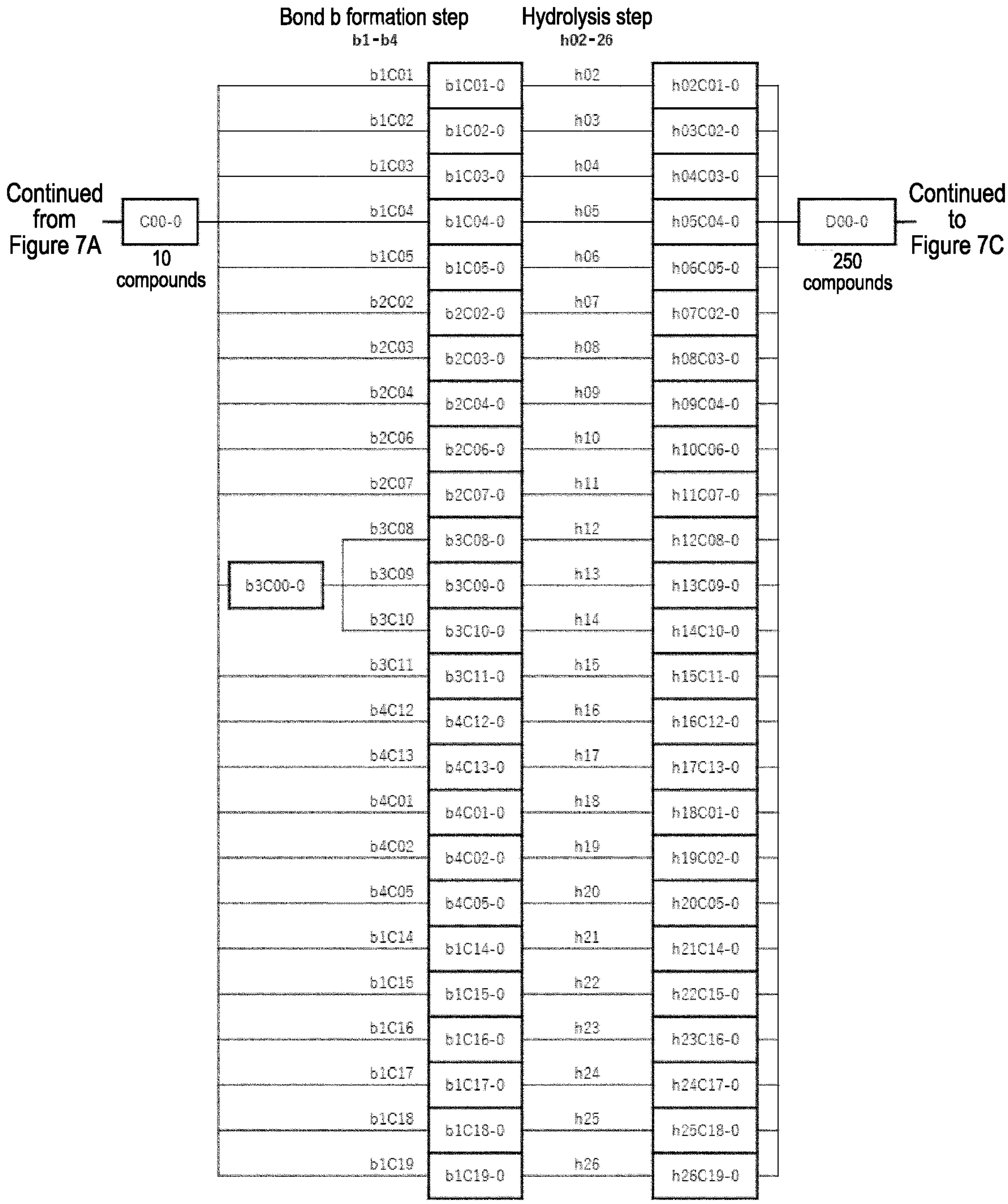
Bond b formation step
b1-b4
Hydrolysis step
h02-26
Continued from Figure 7A
C00-0
10 compounds
b1C01
b1C01-0
h02
h02C01-0
b1C02
b1C02-0
h03
h03C02-0
b1C03
b1C03-0
h04
h04C03-0
b1C04
b1C04-0
h05
h05C04-0
b1C05
b1C05-0
h06
h06C05-0
b2C02
b2C02-0
h07
h07C02-0
b2C03
b2C03-0
h08
h08C03-0
b2C04
b2C04-0
h09
h09C04-0
b2C06
b2C06-0
h10
h10C06-0
b2C07
b2C07-0
h11
h11C07-0
b3C00-0
b3C08
b3C08-0
h12
h12C08-0
b3C09
b3C09-0
h13
h13C09-0
b3C10
b3C10-0
h14
h14C10-0
b3C11
b3C11-0
h15
h15C11-0
b4C12
b4C12-0
h16
h16C12-0
b4C13
b4C13-0
h17
h17C13-0
b4C01
b4C01-0
h18
h18C01-0
b4C02
b4C02-0
h19
h19C02-0
b4C05
b4C05-0
h20
h20C05-0
b1C14
b1C14-0
h21
h21C14-0
b1C15
b1C15-0
h22
h22C15-0
b1C16
b1C16-0
h23
h23C16-0
b1C17
b1C17-0
h24
h24C17-0
b1C18
b1C18-0
h25
h25C18-0
b1C19
b1C19-0
h26
h26C19-0
D00-0
250 compounds
Continued to Figure 7C

Figure 7C

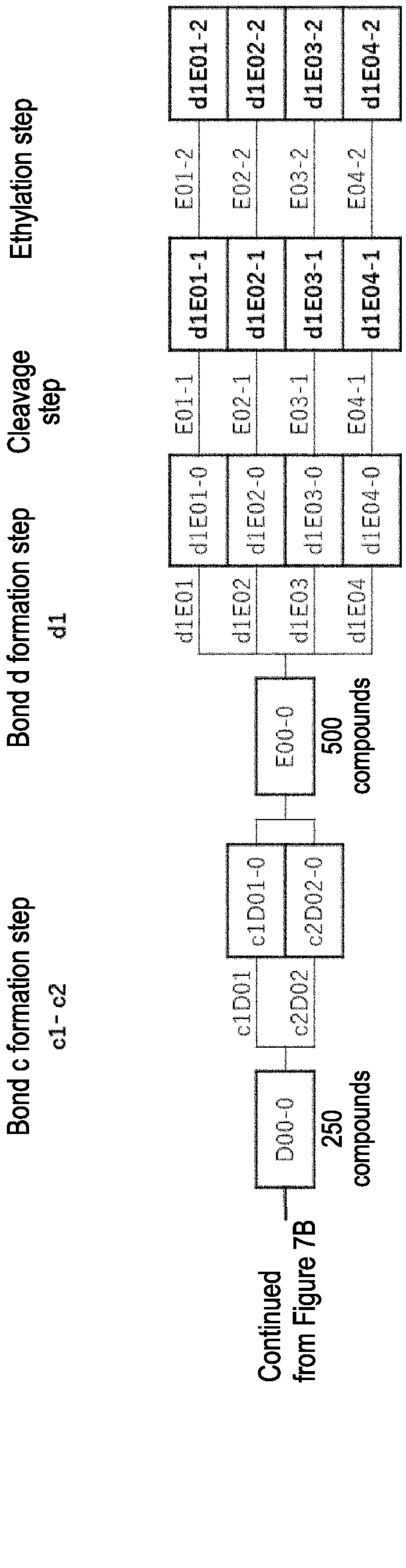
Continued from Figure 7B
D00-0
250 compounds
Bond c formation step
c1 - c2
c1D01
c2D02
c1D01-0
c2D02-0
E00-0
500 compounds
Bond d formation step
d1
d1E01
d1E02
d1E03
d1E04
d1E01-0
d1E02-0
d1E03-0
d1E04-0
Cleavage step
E01-1
E02-1
E03-1
E04-1
d1E01-1
d1E02-1
d1E03-1
d1E04-1
Ethylation step
E01-2
E02-2
E03-2
E04-2
d1E01-2
d1E02-2
d1E03-2
d1E04-2

# Figure 8

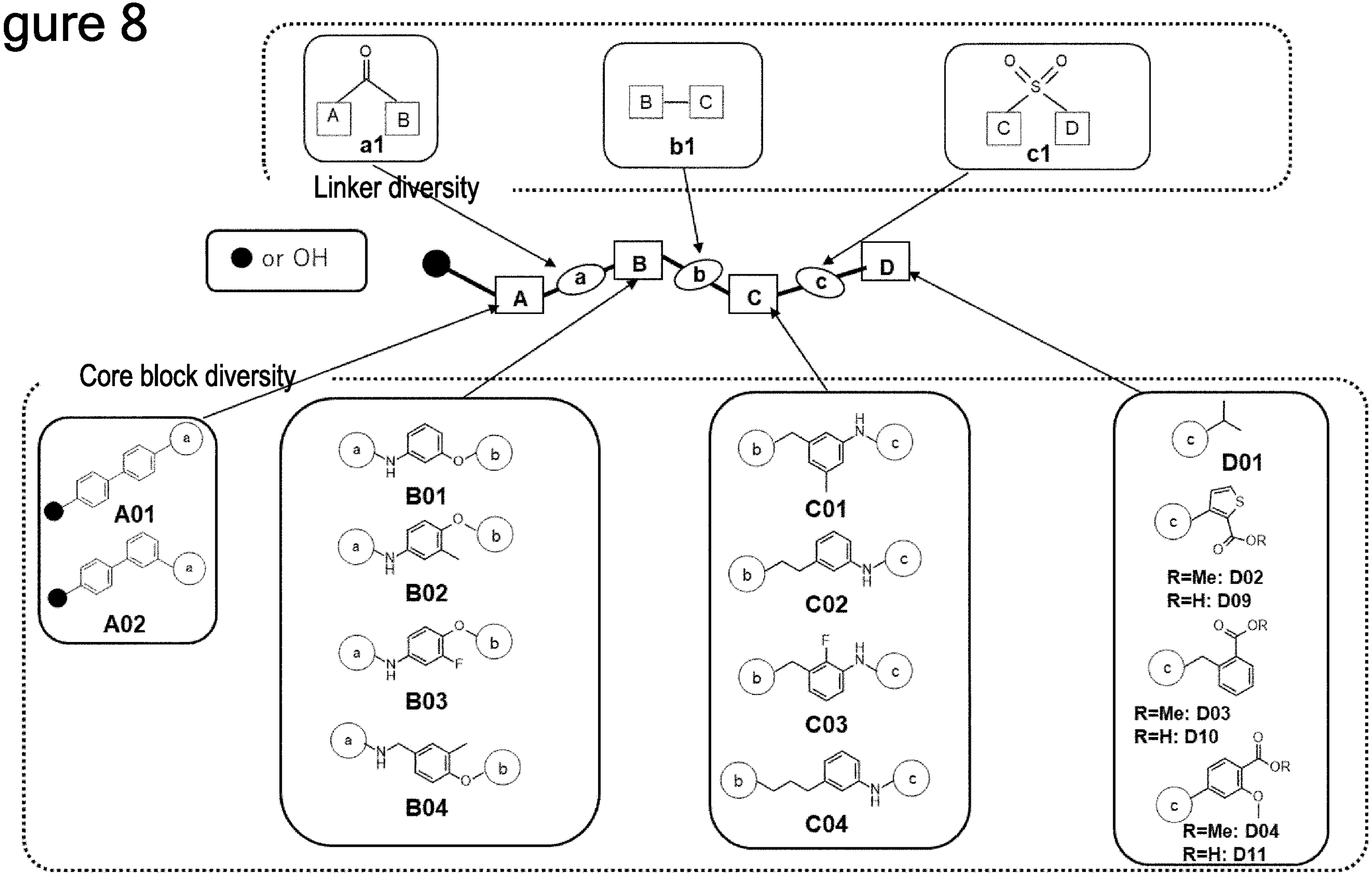


In Figure 8, ● represents 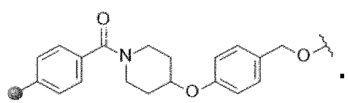 .

Figure 9

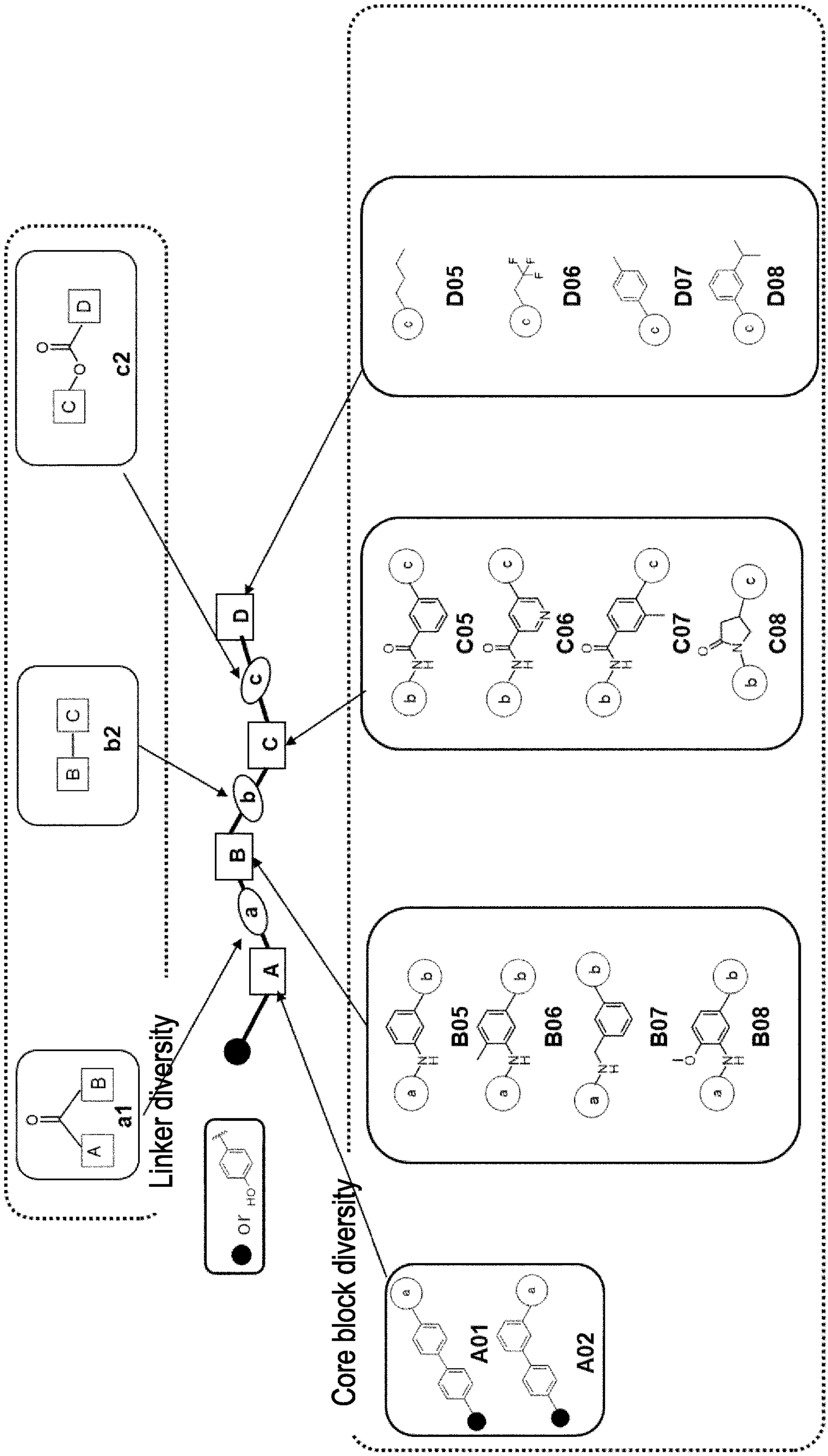


In Figure 9, ● represents 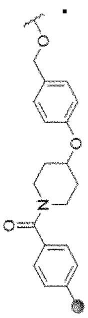 .

Figure 10

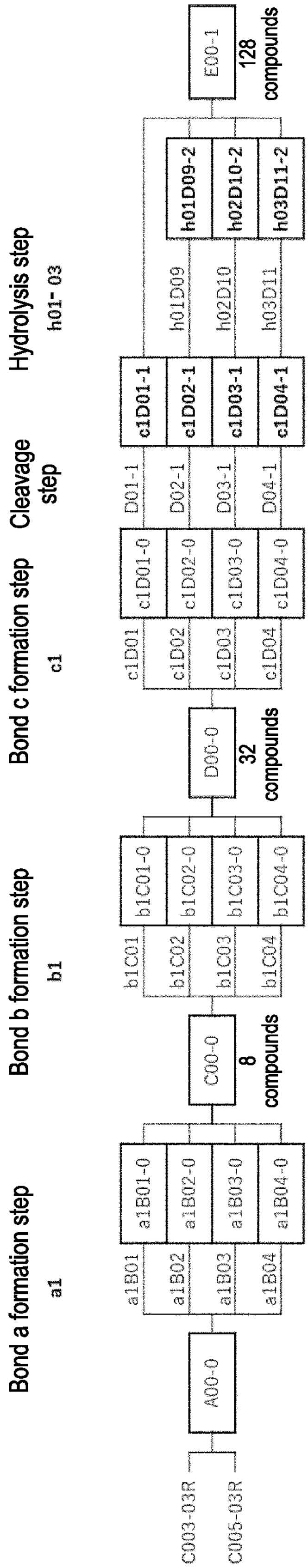
C003-03R
C005-03R
A00-0
Bond a formation step
a1
a1B01
a1B02
a1B03
a1B04
a1B01-0
a1B02-0
a1B03-0
a1B04-0
C00-0
8 compounds
Bond b formation step
b1
b1C01
b1C02
b1C03
b1C04
b1C01-0
b1C02-0
b1C03-0
b1C04-0
D00-0
32 compounds
Bond c formation step
c1
c1D01
c1D02
c1D03
c1D04
c1D01-0
c1D02-0
c1D03-0
c1D04-0
Cleavage step
D01-1
D02-1
D03-1
D04-1
c1D01-1
c1D02-1
c1D03-1
c1D04-1
Hydrolysis step
h01- 03
h01D09
h02D10
h03D11
h01D09-2
h02D10-2
h03D11-2
E00-1
128 compounds

Figure 11

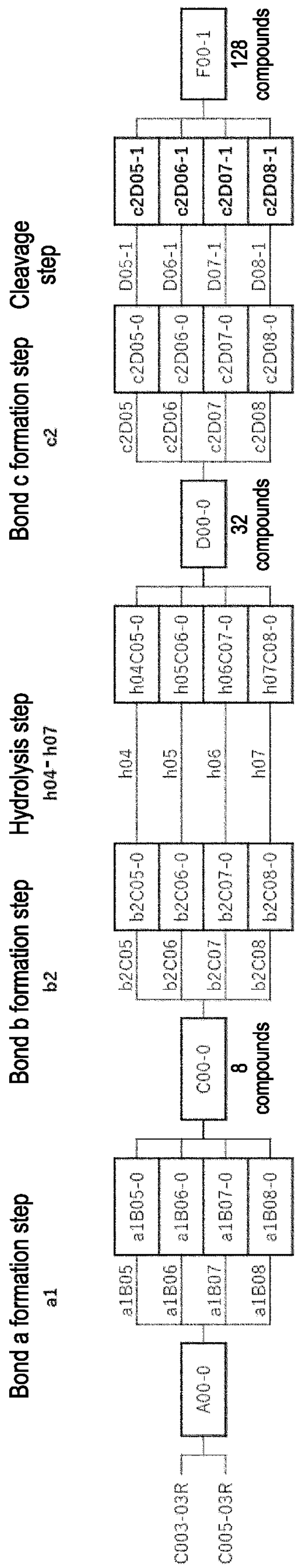
Bond a formation step
a1
Bond b formation step
b2
Hydrolysis step
h04-h07
Bond c formation step
c2
Cleavage step
C003-03R
C005-03R
A00-0
a1B05
a1B06
a1B07
a1B08
a1B05-0
a1B06-0
a1B07-0
a1B08-0
C00-0
8 compounds
b2C05
b2C06
b2C07
b2C08
b2C05-0
b2C06-0
b2C07-0
b2C08-0
h04
h05
h06
h07
h04C05-0
h05C06-0
h06C07-0
h07C08-0
D00-0
32 compounds
c2D05
c2D06
c2D07
c2D08
c2D05-0
c2D06-0
c2D07-0
c2D08-0
D05-1
D06-1
D07-1
D08-1
c2D05-1
c2D06-1
c2D07-1
c2D08-1
F00-1
128 compounds

## INTERNATIONAL SEARCH REPORT

International application No.
**PCT/JP2022/001007**

**A. CLASSIFICATION OF SUBJECT MATTER**

***C07C 311/21***(2006.01)i; ***C07D 237/24***(2006.01)i; ***C07D 333/20***(2006.01)i; ***C07D 333/24***(2006.01)i; ***C07D 333/34***(2006.01)i; ***C07D 333/38***(2006.01)i; ***C07D 333/40***(2006.01)i; ***C07D 401/04***(2006.01)i; ***C07D 401/12***(2006.01)i; ***C07D 401/14***(2006.01)i; ***C07D 403/12***(2006.01)i; ***C07D 409/04***(2006.01)i; ***C07D 409/12***(2006.01)i; ***C07D 409/14***(2006.01)i; ***C40B 40/04***(2006.01)i; ***C07D 213/56***(2006.01)i; ***C07D 213/65***(2006.01)i; ***C07D 295/155***(2006.01)i; ***C07D 295/185***(2006.01)i; ***C07D 295/192***(2006.01)i; ***C07D 295/26***(2006.01)i; ***C07D 231/12***(2006.01)i; ***C40B 30/04***(2006.01)i; ***C40B 50/14***(2006.01)i; ***G01N 33/15***(2006.01)i; ***G01N 33/50***(2006.01)i

FI: C07C311/21; C07D213/56; C07D213/65; C07D231/12 C; C07D237/24; C07D295/155; C07D295/185; C07D295/192 CSP; C07D295/26; C07D333/20; C07D333/24; C07D333/34; C07D333/38; C07D333/40; C07D401/04; C07D401/12; C07D401/14; C07D403/12; C07D409/04; C07D409/12; C07D409/14; C40B30/04; C40B40/04; C40B50/14; G01N33/15 Z; G01N33/50 Z

According to International Patent Classification (IPC) or to both national classification and IPC

**B. FIELDS SEARCHED**

Minimum documentation searched (classification system followed by classification symbols)

C07C311/21; C07D237/24; C07D333/20; C07D333/24; C07D333/34; C07D333/38; C07D333/40; C07D401/04; C07D401/12; C07D401/14; C07D403/12; C07D409/04; C07D409/12; C07D409/14; C40B40/04; C07D213/56; C07D213/65; C07D295/155; C07D295/185; C07D295/192; C07D295/26; C07D231/12; C40B30/04; C40B50/14; G01N33/15; G01N33/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus (JDreamIII); JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | Annu. Rev. Pharmacol. Toxicol. 2000, 40, 273-282<br>pp. 275-279 | 1-15 |
| A | Journal of Medicinal Chemistry. 1999, 42(19), 3743-3778<br>pp. 3757-3764, fig. 3, 4, table 13 | 1-15 |
| A | Bioconjugate Chemistry. 2020, 31, 770-780<br>abstract, tables 4, 5, fig. 2 | 1-15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 March 2022** | **22 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019527672 A
- JP 2020524701 A
- WO 2020178282 A


### Non-patent literature cited in the description


- **RUIWU LIU et al.** Combinatorial chemistry in drug discovery. *Current Opinion in Chemical Biology,* 2017, vol. 38, 117-126
- **JOERG HOLENZ ; PATRICK STOY.** Advances in Lead Generation. *Bioorganic & Medicinal Chemistry Letters,* 2019, vol. 29, 517-524
- **R. MACARRON et al.** Impact of high-throughput screening in biomedical research. *Nature Reviews Drug Discovery,* 2011, vol. 10, 188-195
- **M. J. WIGGLESWORTH et al.** Increasing the delivery of next generation therapeutics from high throughput screening libraries. *Current Opinion in Chemical Biology,* 2015, vol. 26, 104-110
- **P. J. HAJDUK et al.** A decade of fragment-based drug design: strategic advances and lessons learned. *Nature Reviews Drug Discovery,* 2007, vol. 6, 211-219
- **G. CHESSARI et al.** From fragment to clinical candidate- a historical perspective. *Drug Discovery Today,* July 2009, vol. 14 (13-14), 668-675
- **A. BANCET. et al.** Fragment Linking Strategies for Structure-Based Drug Design. *J. Med. Chem.,* 2020, vol. 63 (20), 11420-11435
- **F. IMRIE et al.** Deep Generative Models for 3D Linker Design. *J. Chem. Inf. Model.,* 2020, vol. 60 (4), 1983-1995
- **R. LIU et al.** Combinatorial peptide library methods for immunobiology research. *Experimental Hematology,* 2003, vol. 31, 11-30
- The "One-Bead-One-Compound" Combinatorial Library. *Method. Chem. Rev.,* 1997, vol. 97 (2), 411-448
- Structurally homogeneous and heterogeneous synthetic combinatorial libraries. *Molecular Diversity,* 1996, vol. 1, 149-164
- **R. SEVERINSEN et al.** Library of Biphenyl Privileged Substructures using a Safety-Catch Linker Approach. *J. Chem. Inf. Model.,* 2020, vol. 60 (4), 1983-1995
- Souyaku Kagaku (Drug Discovery Chemistry in English). Tokyo Kagaku Dojin, 186
- *The Practice of Medicinal Chemistry,* 483
- **RICHARD A. HOUGHTEN et al.** Mixture-Based Synthetic Combinatorial Libraries. *J. Med. Chem.,* 1999, vol. 42 (19), 3743-3778
- **ROBERT A. GOODNOW et al.** DNA-encoded chemistry: enabling the deeper sampling of chemical space. *Nature Reviews Drug Discovery,* 2017, vol. 16, 131-147
- DNA-Compatible Chemistry. **KIN-CHUN LUK ; ALEXANDER LEE SATZ.** A Handbook for DNA-Encoded Chemistry. 67-98
- **MARIE L. MALONE ; BRIAN M. PAEGEL.** What is a "DNA- Compatible" Reaction?. *ACS Combinatorial Science,* 2016, vol. 18 (4), 182-187
- **A. SATZ et al.** Analysis of Current DNA Encoded Library Screening Data Indicates Higher False Negative Rates for Numerically Larger Libraries. *ACS Comb. Sci.,* 2017, vol. 19 (4), 234-238
- **C. S. KOLLMANN et al.** Application of encoded library technology (ELT) to a protein-protein interaction target: Discovery of a potent class of integrin lymphocyte function-associated antigen 1 (LFA-1) antagonists. *Bioorg. Med. Chem.,* 2014, vol. 22, 235
- **Y. C. CHEN et al.** C-N Coupling of DNA-Conjugated (Hetero)aryl Bromides and Chlorides for DNA-Encoded Chemical Library Synthesis. *Bioconjugate Chem.,* 2020, vol. 31 (3), 770-780
- **D. A. ANNIS et al.** Affinity selection-mass spectrometry screening techniques for small molecule drug discovery. *Current Opinion in Chemical Biology,* 2007, vol. 11, 518-526
- **J. QIAN et al.** Discovery of novel inhibitors of Bcl-xL using multiplehigh-throughput screening platforms. *Analytical Biochemistry,* 2004, vol. 328, 131-138
- **K. M. COMESS et al.** Kinase Drug Discovery by Affinity Selection/Mass Spectrometry (ASMS): Application to DNA Damage Checkpoint Kinase Chk1. *Journal of Biomolecular Screening,* 2006, vol. 11 (7), 755-764

- **D. A. ANNIS et al.** An affinity selection-mass spectrometry method for the identification of small molecule ligands from self-encoded combinatorial libraries: Discovery of a novel antagonist of E. coli dihydrofolate reductase. *International Journal of Mass Spectrometry,* 2004, vol. 238, 77-83
- **B. R. LAHUE et al.** Substituted benzimidazoles: A novel chemotype for small molecule hKSP inhibitors. *Bioorganic & Medicinal Chemistry Letters,* 2009, vol. 19, 3405
- **B. C. DOAK ; J. KIHLBERG.** Drug discovery beyond the rule of 5 - Opportunities and challenges. *Expert Opinion on Drug Discovery,* 2017, vol. 12 (2), 115-119
- **H. LU et al.** Recent advances in the development of protein-protein interactions modulators: mechanisms and clinical trials. *Sig Transduct Target Ther,* 2020, vol. 5, 213, https://doi.org/10.1038/s41392-020-00315-3
- **F. LOVERING et al.** Escape from Flatland: Increasing Saturation as an Approach to Improving Clinical Success. *J. Med. Chem.,* 2009, vol. 52 (21), 6752-6756
- **G. SCHNEIDER et al.** Scaffold-Hopping" by Topological Pharmacophore Search: A Contribution to Virtual Screening. *Angew. Chem. Int. Ed.,* 1999, vol. 38, 2894-2896
- **H. SUN et al.** Classification of scaffold-hopping approaches. *Drug Discovery Today,* April 2012, vol. 17 (7-8), 310-32
- *Current Opinion in Chemical Biology,* 2007, vol. 11, 518-526
- *CHEMICAL ABSTRACTS,* 2097600-15-0
- *Science,* 19 August 1983, vol. 221 (4612), 709-13
- *J. Org. Chem.,* 2011, vol. 76 (2), 435-440
- *CHEMICAL ABSTRACTS,* 5096-73-1
- *CHEMICAL ABSTRACTS,* 57260-71-6
- *CHEMICAL ABSTRACTS,* 1340506-55-9
- *CHEMICAL ABSTRACTS,* 110-85-0
- *CHEMICAL ABSTRACTS,* 321946-09-2
- *CHEMICAL ABSTRACTS,* 1310-65-2
- *CHEMICAL ABSTRACTS,* 42058-59-3
- *CHEMICAL ABSTRACTS,* 459- 57-4
- *CHEMICAL ABSTRACTS,* 187265-40-3
- *CHEMICAL ABSTRACTS,* 893566-75-1
- *CHEMICAL ABSTRACTS,* 1251336-69-2
- *CHEMICAL ABSTRACTS,* 1579518-76-5
- *CHEMICAL ABSTRACTS,* 321337-38-6
- *ACS Catal.,* 2018, vol. 8 (4), 2989-2994
- *J. Org. Chem.,* 2013, vol. 78, 568-581
- *J. Orgnomet. Chem.,* 1975, vol. 93, 253
- *J. Organomet. Chem.,* 1975, vol. 93, 259
- *J. Med. Chem.,* 2016, vol. 59, 9837-9854
- *J. Med. Chem.,* 2007, vol. 50, 3528
- *J. Med. Chem.,* 2006, vol. 49, 1165-1181
- *CHEMICAL ABSTRACTS,* 86620-62-4
- *CHEMICAL ABSTRACTS,* 71799-35-4
- *CHEMICAL ABSTRACTS,* 406233- 13-4
- *CHEMICAL ABSTRACTS,* 140-75-0
- *Org. Lett.,* 2015, vol. 17, 3370-3383
- *Chem. Commun.,* 2011, vol. 47, 5181-5183
- *J. Am. Chem. Soc.,* 2001, vol. 123, 7727
- *Eur. J. Org. Chem.,* 2012, 764-771
- *Eur. J. Med. Chem.,* 2018, vol. 143, 48-65
- *Chem. Pharm. Bull.,* 1999, vol. 47, 809-812
- *Tetrahedron Lett.,* 2001, vol. 42, 979-982
- *Angew. Chem. Int. Ed.,* 2014, vol. 53, 10204-10208
- *Nature,* 2018, vol. 557, 228-232
- *RSC Advances,* 2018, vol. 8, 25168
- *Tetrahedron Lett.,* 1995, vol. 36, 2529
- *J. Org. Chem.,* 2011, vol. 76, 6832
- *Org. Chem.,* 2017, vol. 19, 2518
- *Org. Biomol. Chem.,* 2019, vol. 17, 88
- *Tetrahedron,* 2013, vol. 69, 3511
- *Expert Opinion on Drug Discovery,* 2008, vol. 3 (9), 1123-1143
- *J. Med. Chem.,* 2008, vol. 51 (21), 6902-6915
- *CHEMICAL ABSTRACTS,* 406233-75-8
- *CHEMICAL ABSTRACTS,* 1506205-91-9
- *CHEMICAL ABSTRACTS,* 1545014-81-0
- *CHEMICAL ABSTRACTS,* 1690948-00-5
- *CHEMICAL ABSTRACTS,* 1261964-16-2
- *CHEMICAL ABSTRACTS,* 1261907- 60-1
- *CHEMICAL ABSTRACTS,* 406233-13-4
- *ACS Omega,* 2019, vol. 4 (5), 8892-8906
- *ACS Med. Chem. Lett.,* 2014, vol. 5 (6), 662-667
- *J. Med. Chem.,* 2007, vol. 50 (4), 641-662
- *ACS Med. Chem. Lett.,* 2013, vol. 4 (2), 186-190